(19) Europäisches Patentamt / European Patent Office / Office européen des brevets

(11) **EP 2 254 592 B1**

(12) # EUROPEAN PATENT SPECIFICATION

(45) Date of publication and mention of the grant of the patent:
**05.06.2019 Bulletin 2019/23**

(51) Int Cl.:
**A61K 39/02** [(2006.01)]  **C07K 14/705** [(2006.01)]
**G01N 33/569** [(2006.01)]  **C07K 14/74** [(2006.01)]
**A61P 31/04** [(2006.01)]

(21) Application number: **08872852.2**

(22) Date of filing: **30.12.2008**

(86) International application number:
**PCT/DK2008/000451**

(87) International publication number:
**WO 2009/106073 (03.09.2009 Gazette 2009/36)**

(54) **MHC MULTIMERS IN BORRELIA DIAGNOSTICS AND DISEASE**

MHC-MULTIMERE IN BORRELIA-DIAGNOSTIKA UND ERKRANKUNGEN

MULTIMÈRES DE MHC DANS LE DIAGNOSTIC ET LE TRAITEMENT DE LA BORRÉLIOSE

(84) Designated Contracting States:
**AT BE BG CH CY CZ DE DK EE ES FI FR GB GR HR HU IE IS IT LI LT LU LV MC MT NL NO PL PT RO SE SI SK TR**

(30) Priority: **28.02.2008 DK 200800295**
**28.02.2008 US 67831 P**
**17.07.2008 DK 200801011**
**24.07.2008 US 83481 P**
**01.10.2008 DK 200801380**
**01.10.2008 US 101931 P**

(43) Date of publication of application:
**01.12.2010 Bulletin 2010/48**

(73) Proprietor: **Dako Denmark A/S**
**2600 Glostrup (DK)**

(72) Inventors:
• **BRIX, Liselotte**
**DK-2880 Bagsvaerd (DK)**
• **PEDERSEN, Henrik**
**DK-3540 Lynge (DK)**
• **SCHOLLER, Jorgen**
**DK-2800 Lyngby (DK)**

(74) Representative: **Høiberg P/S**
**Adelgade 12**
**1304 Copenhagen K (DK)**

(56) References cited:
**WO-A1-00/21989**   **WO-A1-2009/003492**
**WO-A2-02/072631**   **WO-A2-2005/049073**
**WO-A2-2006/081826**   **WO-A2-2008/116468**

WO-A2-2009/039854   US-A- 5 635 363
US-A1- 2004 209 295

• **KNABEL MICHAEL ET AL: "Reversible MHC multimer staining for functional isolation of T-cell populations and effective adoptive transfer" NATURE MEDICINE, NATURE PUBLISHING GROUP, NEW YORK, NY, US, vol. 8, no. 6, 1 June 2002 (2002-06-01), pages 631-637, XP002460640 ISSN: 1078-8956**
• **BATARD P ET AL: "Dextramers: New generation of fluorescent MHC class I/peptide multimers for visualization of antigen-specific CD8<+> T cells" JOURNAL OF IMMUNOLOGICAL METHODS, ELSEVIER SCIENCE PUBLISHERS B.V.,AMSTERDAM, NL, vol. 310, no. 1-2, 20 March 2006 (2006-03-20), pages 136-148, XP025158203 ISSN: 0022-1759 [retrieved on 2006-03-20]**
• **MEYER ABBIE L ET AL: "Direct enumeration of Borrelia-reactive CD4 T cells ex vivo by using MHC class II tetramers" PROCEEDINGS OF THE NATIONAL ACADEMY OF SCIENCES OF USA. (PNAS), NATIONAL ACADEMY OF SCIENCE, WASHINGTON, DC, US, vol. 97, no. 21, 10 October 2000 (2000-10-10), pages 11433-11438, XP002508233 ISSN: 0027-8424**
• **AUSUBEL L J ET AL: "Characterization of in vivo expanded OspA-specific human T-cell clones" CLINICAL IMMUNOLOGY, ACADEMIC PRESS, US, vol. 115, no. 3, 1 June 2005 (2005-06-01), pages 313-322, XP004887961 ISSN: 1521-6616**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

(Cont. next page)

• DROUIN ET AL: "Searching for borrelial T cell epitopes associated with antibiotic-refractory Lyme arthritis" MOLECULAR IMMUNOLOGY, PERGAMON, GB, vol. 45, no. 8, 11 January 2008 (2008-01-11), pages 2323-2332, XP022513113 ISSN: 0161-5890

Remarks:
•The complete document including Reference Tables and the Sequence Listing can be downloaded from the EPO website
•The file contains technical information submitted after the application was filed and not included in this specification

## Description

## TECHNICAL FIELD

The present disclosure relates to MHC-peptide complexes and uses thereof in the diagnosis, treatment and monitoring of treatment of a disease in an individual.

## BACKGROUND

Biochemical interactions between peptide epitope specific membrane molecules encoded by the Major Histocompatibility Complex (MHC, in humans HLA) and T-cell receptors (TCR) are required to elicit specific immune responses. This requires activation of T-cells by presentation to the T-cells of peptides against which a T-cell response should be raised. The peptides are presented to the T-cells by the MHC complexes.

## The immune response

The immune response is divided into two parts termed the innate immune response and the adaptive immune response. Both responses work together to eliminate pathogens (antigens). Innate immunity is present at all times and is the first line of defense against invading pathogens. The immediate response by means of pre-existing elements, i.e. various proteins and phagocytic cells that recognize conserved features on the pathogens, is important in clearing and control of spreading of pathogens. If a pathogen is persistent in the body and thus only partially cleared by the actions of the innate immune system, the adaptive immune system initiate a response against the pathogen. The adaptive immune system is capable of eliciting a response against virtually any type of pathogen and is unlike the innate immune system capable of establishing immunological memory.

The adaptive response is highly specific to the particular pathogen that activated it but it is not so quickly launched as the innate when first encountering a pathogen. However, due to the generation of memory cells, a fast and more efficient response is generated upon repeated exposure to the same pathogen. The adaptive response is carried out by two distinct sets of lymphocytes, the B cells producing antibodies leading to the humoral or antibody mediated immune response, and the T cells leading to the cell mediated immune response.

T cells express a clonotypic T cell receptor (TCR) on the surface. This receptor enable the T cell to recognize peptide antigens bound to major histocompatibility complex (MHC) molecules, called human leukocyte antigens (HLA) in man. Depending on the type of pathogen, being intracellular or extracellular, the antigenic peptides are bound to MHC class I or MHC class II, respectively. The two classes of MHC complexes are recognized by different subsets of T cells; Cytotoxic CD8+ T cells recognizing MHC class I and CD4+ helper cells recognizing MHC class II. In general, TCR recognition of MHC-peptide complexes result in T cell activation, clonal expansion and differentiation of the T cells into effector, memory and regulatory T cells.

B cells express a membrane bound form of immunoglobulin (Ig) called the B cell receptor (BCR). The BCR recognizes an epitope that is part of an intact three dimensional antigenic molecule. Upon BCR recognition of an antigen the BCR:antigen complex is internalized and fragments from the internalized antigen is presented in the context of MHC class II on the surface of the B cell to CD4+ helper T-cells (Th). The specific Th cell will then activate the B cell leading to differentiation into an antibody producing plasma cell.

A very important feature of the adaptive immune system is its ability to distinguish between self and non-self antigens, and preferably respond against non-self. If the immune system fails to discriminate between the two, specific immune responses against self-antigens are generated. These autoimmune reactions can lead to damage of self-tissue.

The adaptive immune response is initiated when antigens are taken up by professional antigen presenting cells such as dendritic cells, Macrophages, Langerhans cells and B-cells. These cells present peptide fragments, resulting from the degradation of proteins, in the context of MHC class II proteins (Major Histocompatibility Complex) to helper T cells. The T helper cells then mediate help to B-cells and antigen-specific cytotoxic T cells, both of which have received primary activation signals via their BCR respective TCR. The help from the Th-cell is mediated by means of soluble mediators e.g. cytokines.

In general the interactions between the various cells of the cellular immune response is governed by receptor-ligand interactions directly between the cells and by production of various soluble reporter substances e.g. cytokines by activated cells.

## MHC-peptide complexes

MHC complexes function as antigenic peptide receptors, collecting peptides inside the cell and transporting them to the cell surface, where the MHC-peptide complex can be recognized by T-lymphocytes. Two classes of classical MHC complexes exist, MHC class I and II. The most important difference between these two molecules lies in the protein source from which they obtain their associated peptides. MHC class I molecules present peptides derived from endogenous antigens degraded in the cytosol and are thus able to display fragments of viral proteins and unique proteins derived from cancerous cells. Almost all nucleated cells express MHC class I on their surface even though the expression level varies among different cell types. MHC class II molecules bind peptides derived from exogenous antigens. Exogenous proteins enter the cells by endocytosis or phagocytosis, and these proteins are degraded by proteases in acidified intracellular vesicles before presentation by MHC class II molecules. MHC class II molecules are only expressed on professional antigen presenting cells like B cells and macrophages.

The three-dimensional structure of MHC class I and II molecules are very similar but important differences exist. MHC class I molecules consist of two polypeptide chains, a heavy chain, $\alpha$, spanning the membrane and a light chain, $\beta$2-microglobulin ($\beta$2m). The heavy chain is encoded in the gene complex termed the major histocompatibility complex (MHC), and its extracellular portion comprises three domains, $\alpha$1, $\alpha$2 and $\alpha$3. The $\beta$2m chain is not encoded in the MHC gene and consists of a single domain, which together with the $\alpha$3 domain of the heavy chain make up a folded structure that closely resembles that of the immunoglobulin. The $\alpha$1 and $\alpha$2 domains pair to form the peptide binding cleft, consisting of two segmented $\alpha$ helices lying on a sheet of eight $\beta$-strands. In humans as well as in mice three different types of MHC class I molecule exist. HLA-A, B, C are found in humans while MHC class I molecules in mice are designated H-2K, H-2D and H-2L.

The MHC class II molecule is composed of two membrane spanning polypeptide chains, $\alpha$ and $\beta$, of similar size (about 30000 Da). Genes located in the major histocompatibility complex encode both chains. Each chain consists of two domains, where $\alpha$1 and $\beta$1 forms a 9-pocket peptide-binding cleft, where pocket 1, 4, 6 and 9 are considered as major peptide binding pockets. The $\alpha$2 and $\beta$2, like the $\alpha$2 and $\beta$2m in the MHC class I molecules, have amino acid sequence and structural similarities to immunoglobulin constant domains. In contrast to MHC class I complexes, where the ends of the antigenic peptide is buried, peptide-ends in MHC class II complexes are not. HLA-DR, DQ and DP are the human class II molecules, H-2A, M and E are those of the mice.

A remarkable feature of MHC genes is their polymorphism accomplished by multiple alleles at each gene. The polygenic and polymorphic nature of MHC genes is reflected in the peptide-binding cleft so that different MHC complexes bind different sets of peptides. The variable amino acids in the peptide binding cleft form pockets where the amino acid side chains of the bound peptide can be buried. This permits a specific variant of MHC to bind some peptides better than others.

MHC multimers

Due to the short half-life of the peptide-MHC-T cell receptor ternary complex (typically between 10 and 25 seconds) it is difficult to label specific T cells with labelled MHC-peptide complexes, and like-wise, it is difficult to employ such monomers of MHC-peptide for therapeutic and vaccine purposes because of their weak binding. In order to circumvent this problem, MHC multimers have been developed. These are complexes that include multiple copies of MHC-peptide complexes, providing these complexes with an increased affinity and half-life of interaction, compared to that of the monomer MHC-peptide complex. The multiple copies of MHC-peptide complexes are attached, covalently or non-covalently, to a multimerization domain. Known examples of such MHC multimers include the following:

- MHC-dimers: Each MHC dimer contains two copies of MHC-peptide. IgG is used as multimerization domain, and one of the domains of the MHC protein is covalently linked to IgG.
- MHC-tetramers: Each MHC-tetramer contains four copies of MHC-peptide, each of which is biotinylated. The MHC complexes are held together in a complex by the streptavidin tetramer protein, providing a non-covalent linkage between a streptavidin monomer and the MHC protein. Tetramers are described in US patent 5,635,363.
- MHC pentamers: Five copies of MHC-peptide complexes are multimerised by a self-assembling coiled-coil domain, to form a MHC pentamer. MHC pentamers are described in the US patent 2004209295
- MHC dextramers: A large number of MHC-peptide complexes, typically more than ten, are attached to a dextran polymer. MHC-dextramers are described in the patent application WO 02/072631 A2.
- MHC streptamers: 8-12 MHC-peptide complexes attached to Streptactin. MHC streptamers are described in Knabel M et al. Reversibel MHC multimer staining for functional isolation of T-cell populations and effective adoptive transfer. Nature medicine 6. 631-637 (2002).

**Use of MHC multimers in flow cytometry and related techniques**

The concentration of antigen-specific T-cells in samples from e.g. peripheral blood can be very low. Flow cytometry and related methods offer the ability to analyze a large number of cells and simultaneously identify the few of interest. MHC multimers have turned out to be very valuable reagents for detection and characterization of antigen-specific T-cells in flow cytometer experiments. The relative amount of antigen-specific T cells in a sample can be determined and also the affinity of the binding of MHC multimer to the T-cell receptor can be determined.

The basic function of a flow cytometer is its ability to analyse and identify fluorochrome labelled entities in a liquid sample, by means of its excitation, using a light source such as a laser beam and the light emission from the bound fluorochrome.

MHC multimers is used as detections molecule for identification of antigen-specific T-cells in flow cytometry, by labelling the MHC multimer with a specific fluorochrome, which is detectable, by the flow cytometer used.

In order to facilitate the identification of a small amount of cells, the cells can be sub-categorized using antibodies or other fluorochrome labelled detections molecules directed against surface markers other than the TCR on the specific T-cells population. Antibodies or other fluorochrome labelled detections molecules can also be used to identify cells known not to be antigen-specific T-cells. Both kinds of detections molecules are in the following referred to as gating reagents. Gating reagents, helps identify the "true" antigen-specific T cells bound by MHC multimers by identifying specific subpopulations in a sample, e.g. T cells and by excluding cells that for some reason bind MHC mulimers without being antigen-specific T-cells.

Other cytometry methods, e.g. fluorescence microscopy and IHC can like flow cytometry be employed in identification of antigen-specific T cells in a cell sample using MHC multimers.

**Application of MHC multimers in immune monitoring, diagnostics, prognostics, therapy and vaccines**

T cells are pivotal for mounting an adaptive immune response. It is therefore of importance to be able to measure the number of specific T cells when performing a monitoring of a given immune response, for example in connection with vaccine development, autologous cancer therapy, transplantation, infectious diseases, toxicity studies etc.

Accordingly, the present disclosure further provides powerful tools in the fields of vaccines, therapy and diagnosis. One objective of the present disclosure is to provide methods for anti-bacterial and anti-virus immunotherapy by generating antigen-specific T-cells capable of inactivating or eliminating undesirable target cells. Another objective is to isolate antigen-specific T-cells and culture these in the presence of co-stimulatory molecules. Ex vivo priming and expansion of T-cell populations allows the T-cells to be used in immunotherapy of various types of infectious diseases. A third objective of the present disclosure is to identify and label specific subsets of cells with relevance for the development or treatment of diseases.

Meyer Abbie et al. PNAS Vol. 97, no. 21, 2000, discloses an MHC tetramer loaded with an antigenic peptide from the borrelia burgdorferi antigen OspA and its use in the identification of specific T cells.

WO 00/21989 dicloses the borrelia antigen DBP-A (decorin binding protein A) and vaccine compositions comprising the same.

## SUMMARY

Measurement of antigen-specific T cells during an immune response are important parameters in vaccine development, therapy and infectious diseases, inflammation, autoimmunity, toxicity studies etc. MHC multimers are crucial reagents in monitoring of antigen-specific T cells. The present disclosure describes novel methods to generate MHC multimers and methods to improve existing and new MHC multimers. The disclosure also describes improved methods for the use of MHC multimers in analysis of T cells in samples including diagnostic and prognostic methods. Furthermore the use of MHC multimers in therapy are described, e.g. anti-bacteria therapy.. The present disclosure also relates to MHC multimers comprising one or more Borrelia peptides. In one preferred embodiment the present disclosure relates to a Borrelia vaccine. In a Borrelia vaccine the peptides bound in the peptide binding cleft of MHC are derived from antigenic borrelia proteins. In another preferred embodiment the present disclosure relates to diagnosis and monitoring of Borrelia infection using MHC multimers with Borrelia derived peptides bound in the peptide binding cleft of the MHC molecules.

## DEFINITIONS

As used everywhere herein, the term"a", "an" or "the" is meant to be one or more, i. e. at least one.

"8 mers" are peptides consisting of 8 amino acids.
"9 mers" are peptides consisting of 9 amino acids.
"10 mers" are peptides consisting of 10 amino acids.
"11 mers" are peptides consisting of 11 amino acids.
"13 mers" are peptides consisting of 13 amino acids.
"14 mers" are peptides consisting of 14 amino acids.

"15 mers" are peptides consisting of 15 amino acids.

"16 mers" are peptides consisting of 16 amino acids.

As used everywhere herein, the term "Borrelia garini" also relates to "Borrelia garinii".

An "amino acid residue" can be a natural or non-natural amino acid residue linked peptide bonds or bonds different from peptide bonds. The amino acid residues can be in D-configuration or L-configuration. An amino acid residue comprises an amino terminal part ($NH_2$) and a carboxy terminal part (COOH) separated by a central part comprising a carbon atom, or a chain of carbon atoms, at least one of which comprises at least one side chain or functional group. $NH_2$ refers to the amino group present at the amino terminal end of an amino acid or peptide, and COOH refers to the carboxy group present at the carboxy terminal end of an amino acid or peptide. The generic term amino acid comprises both natural and non-natural amino acids. Natural amino acids of standard nomenclature as listed in J. Biol. Chem., 243:3552-59 (1969) and adopted in 37 C.F.R., section 1.822(b)(2) belong to the group of amino acids listed in the table herein below. Non-natural amino acids are those not listed in the Table below. Examples of non-natural amino acids are those listed e.g. in 37 C.F.R. section 1.822(b)(4). Also, non-natural amino acid residues include, but are not limited to, modified amino acid residues, L-amino acid residues, and stereoisomers of D-amino acid residues.

| Symbols | | Amino acid |
| 1-Letter | 3-Letter | |
| --- | --- | --- |
| Y | Tyr | tyrosine |
| G | Gly | glycine |
| F | Phe | phenylalanine |
| M | Met | methionine |
| A | Ala | alanine |
| S | Ser | serine |
| I | Ile | isoleucine |
| L | Leu | leucine |
| T | Thr | threonine |
| V | Val | valine |
| P | Pro | proline |
| K | Lys | lysine |
| H | His | histidine |
| Q | Gln | glutamine |
| E | Glu | glutamic acid |
| W | Trp | tryptophan |
| R | Arg | arginine |
| D | Asp | aspartic acid |
| N | Asn | asparagine |
| C | Cys | cysteine |

Natural amino acids and their respective codes.

The terms "amino-terminal" and "carboxyl-terminal" are used herein to denote positions within polypeptides. Where the context allows, these terms are used with reference to a particular sequence or portion of a polypeptide to denote proximity or relative position. For example, a certain sequence positioned carboxyl-terminal to a reference sequence within a polypeptide is located proximal to the carboxyl terminus of the reference sequence, but is not necessarily at the carboxyl terminus of the complete polypeptide.

Adjuvant: adjuvants are drugs that have few or no pharmacological effects by themselves, but can increase the efficacy or potency of other drugs when given at the same time. In another embodiment of the present disclosure, an adjuvant is an agent which, while not having any specific antigenic effect in itself, can stimulate the immune system, increasing the response to a vaccine.

Agonist: agonist as used herein is a substance that binds to a specific receptor and triggers a response in the cell. It mimics the action of an endogenous ligand that binds to the same receptor.

Anchor amino acid: Anchor amino acid is used interchangeably herein with anchor residue and is an amino acid of antigenic peptide having amino acid sidechains that bind into pockets lining the peptide-binding groove of MHC molecules thereby anchoring the peptide to the MHC molecule. Anchor residues being responsible for the main anchoring of peptide to MHC molecule are aclled primary anchor amino acids. Amino acids contributing to the binding of antigenic peptide to MHC molecule but in a lesser extend than primary anchor amino acids are called secondary anchor amino acids.

Anchor motif: The pattern of anchor residues in an antigenic peptide binding a certain MHC molecule. Peptides binding different MHC molecules have different anchor motifs defined by the patterns of anchor residues in the peptide sequence.

Anchor residue: Anchor residue is used interchangeably herein with anchor amino acid

Anchor position: The position of an anchor amino acid in antigenic peptide sequence. For MHC II the anchor positions is defined in the 9-mer core motif.

Antagonist: antagonist as used herein is a substance that binds to a specific receptor and blocks the response in the cell. It blocks the action of an endogenous ligand that binds to the same receptor.

Antibodies: As used herein, the term "antibody" means an isolated or recombinant binding agent that comprises the necessary variable region sequences to specifically bind an antigenic epitope. Therefore, an antibody is any form of antibody or fragment thereof that exhibits the desired biological activity, e.g., binding the specific target antigen. Antibodies can derive from multiple species. For example, antibodies include rodent (such as mouse and rat), rabbit, sheep, camel, and human antibodies. Antibodies can also include chimeric antibodies, which join variable regions from one species to constant regions from another species. Likewise, antibodies can be humanized, that is constructed by recombinant DNA technology to produce immunoglobulins which have human framework regions from one species combined with complementarity determining regions (CDR's) from a another species' immunoglobulin. The antibody can be monoclonal or polyclonal. Antibodies can be divided into isotypes (IgA, IgG, IgM, IgD, IgE, IgG1, IgG2, IgG3, IgG4, IgA1, IgA2, IgM1, IgM2)

Antibodies: In another embodiment of the present disclosure the term "antibody" refers to an intact antibody, or a fragment of an antibody that competes with the intact antibody for antigen binding. In certain embodiments of the present disclosure, antibody fragments are produced by recombinant DNA techniques. In certain embodiments of the present disclosure, antibody fragments are produced by enzymatic or chemical cleavage of intact antibodies. Exemplary antibody fragments include, but are not limited to, Fab, Fab', F(ab')2, Fv, and scFv. Exemplary antibody fragments also include, but are not limited to, domain antibodies, nanobodies, minibodies ((scFv-C.sub.H3).sub.2), maxibodies ((scFv-C.sub.H2-C.sub.H3).sub.2), diabodies (noncovalent dimer of scFv).

Antigen presenting cell: An antigen-presenting cell (APC) as used herein is a cell that displays foreign antigen complexed with MHC on its surface.

Antigenic peptide: Used interchangeably with binding peptide. Any peptide molecule that is bound or able to bind into the binding groove of either MHC class 1 or MHC class 2 molecules.

Antigenic polypeptide: Polypeptide that contains one or more antigenic peptide sequences.

APC: Antigen presenting cell

Aptamer: the term aptamer as used herein is defined as oligonucleic acid or peptide molecules that bind a specific target molecule. Aptamers are usually created by selecting them from a large random sequence pool, but natural aptamers also exist. Aptamers can be divided into DNA amtamers, RNA aptamers and peptide aptamers.

Avidin: Avidin as used herein is a glycoprotein found in the egg white and tissues of birds, reptiles and amphibians. It contains four identical subunits having a combined mass of 67,000-68,000 daltons. Each subunit consists of 128 amino acids and binds one molecule of biotin.

Biologically active molecule: A biologically active molecule is a molecule having itself a biological activity/effect or is able to induce a biological activity/effect when administered to a biological system. Biologically active molecules include adjuvants, immune targets (e.g. antigens), enzymes, regulators of receptor activity, receptor ligands, immune potentiators, drugs, toxins, cytotoxic molecules, co-receptors, proteins and peptides in general, sugar moieties, lipid groups, nucleic acids including siRNA, nanoparticles, small molecules.

Bioluminescent: Bioluminescence, as used herein, is the production and emission of light by a living organism as the result of a chemical reaction during which chemical energy is converted to light energy.

Biotin: Biotin, as used herein, is also known as vitamin H or $B_7$. Niotin has the chemical formula $C_{10}H_{16}N_2O_3S$.

Bispecific antibodies: The term bispecific antibodies as used herein is defined as antibodies that have binding specificities for at least two different antigens. The antibody can also be trispecific or multispecific.

Bispecific capture molecule: Molecule that have binding specificities for at least two different antigens. The molecule can also be trispecific or multispecific.

Carrier: A carrier as used herin can be any type of molecule that is directly or indirectly associated with the MHC peptide complex. In this disclosure, a carrier will typically refer to a functionalized polymer (e.g. dextran) that is capable of reacting with MHC-peptide complexes, thus covalently attaching the MHC-peptide complex to the carrier, or that is capable of reacting with scaffold molecules (e.g. streptavidin), thus covalently attaching streptavidin to the carrier; the streptavidin then may bind MHC-peptide complexes. Carrier and scaffold are used interchangeably herein where scaffold typically refers to smaller molecules of a multimerization domain and carrier typically refers to larger molecule and/or cell like structures.

Chelating chemical compound: Chelating chemical compound, as used herein, is the process of reversible bindingof a ligand to a metal ion, forming a metal complex.

Chemiluminescent: Chemiluminescence, as used herein, is the emission of light (luminescence) without emission of heat as the result of a chemical reaction.

Chromophore: A chromophore, as used herein, is the part of a visibly coloured molecule responsible for light absorption over a range of wavelengths thus giving rise to the colour. By extension the term can be applied to uv or ir absorbing parts of molecules.

Coiled-coil polypeptide: Used interchangeably with coiled-coil peptide and coiled-coil structure. The term coiled-coil polypeptide as used herein is a structural motif in proteins, in which 2-7 alpha-helices are coiled together like the strands of a rope

Complement protein: Protein of the complement system.

Counting beads: Beads countable in a flow cytometry experiment.

Covalent binding: The term covalent binding is used herein to describe a form of chemical bonding that is characterized by the sharing of pairs of electrons between atoms. Attraction-to-repulsion stability that forms between atoms when they share electrons is known as covalent bonding.

Crosslinking is the process of chemically joining two or more molecules by a covalent bond. Crosslinking reagents contain reactive ends to specific functional groups (primary amines, sulfhydryls, etc.) on proteins or other molecules.

CSF: Cerebrospinal fluid.

Diagnosis: The act or process of identifying or determining the nature and cause of a disease or injury through evaluation

Diabodies: The term "diabodies" refers to small antibody fragments with two antigen-binding sites, which fragments comprise a heavy-chain variable domain (VH) connected to a light-chain variable domain (VL) in the same polypeptide chain (VH-VL). By using a linker that is too short to allow pairing between the two domains on the same chain, the domains are forced to pair with the complementary domains of another chain and create two antigen-binding sites.

Dendritic cell: The term dendritic cell as used herein is a type of immune cells. Their main function is to process antigen material and present it on the surface to other cells of the immune system, thus functioning as antigen-presenting cells.

Detection: In this disclosure detection means any method capable of measuringen one molecule bound to anoher molecule. The molecules are typically proteins but can be any type of molecule

Dextran: the term dextran as used herein is is a complex, branched polysaccharide made of many glucose molecules joined into chains of varying lengths. The straight chain consists of $\alpha$1->6 glycosidic linkages between glucose molecules, while branches begin from $\alpha$1->3 linkages (and in some cases, $\alpha$1->2 and $\alpha$1->4 linkages as well).

Direct detection of T cells: Direct detection of T cells is used herein interchangeably with direct detection of TCR and direct detection of T cell receptor. As used herein direct detection of T cells is detection directly of the binding interaction between a specific T cell receptor and a MHC multimer.

DNA: The term DNA (Deoxyribonucleic acid) duplex as used herein is a polymer of simple units called nucleotides, with a backbone made of sugars and phosphate atoms joined by ester bonds. Attached to each sugar is one of four types of molecules called bases.

DNA duplex: In living organisms, DNA does not usually exist as a single molecule, but instead as a tightly-associated pair of molecules. These two long strands entwine like vines, in the shape of a double helix.

Electrophilic: electrophile, as used herein, is a reagent attracted to electrons that participates in a chemical reaction by accepting an electron pair in order to bond to a nucleophile.

Enzyme label: enzyme labelling, as used herein, involves a detection method comprising a reaction catalysed by an enzyme.

Epitope-focused antibody: Antibodies also include epitope-focused antibodies, which have at least one minimal essential binding specificity determinant from a heavy chain or light chain CDR3 from a reference antibody, methods for making such epitope-focused antibodies are described in U.S. patent application US 2005-0255552 A1.

Flow cytomerty: The analysis of single cells using a flow cytometer.

Flow cytomter: Instrument that measures cell size, granularity and flourescence due to bound fluorescent marker molecules as single cells pass in a stream past photodectors. A flow cytomter carry out the measurements and/or sorting of individual cells.

Fluorescent: the term fluorescent as used herein is to have the ability to emit light of a certain wavelength when activated by light of another wavelength.

Fluorochromes: fluorochrome, as used herein, is any fluorescent compound used as a dye to mark e.g. protein with a fluorescent label.

Fluorophore: A fluorophore, as used herein, is a component of a molecule which causes a molecule to be fluorescent.

Folding: In this disclosure folding means in vitro or in vivo folding of proteins in a tertiery structure.

Fusion antibody: As used herein, the term "fusion antibody" refers to a molecule in which an antibody is fused to a non-antibody polypeptide at the N- or C-terminus of the antibody polypeptide.

Glycosylated: Glycosylation, as used herein, is the process or result of addition of saccharides to proteins and lipids.

Hapten: A residue on a molecule for which there is a specific molecule that can bind, e.g. an antibody.

Heteroconjugate antibodies are composed of two covalently joined antibodies. Such antibodies have, for example, been proposed to target immune system cells to unwanted cells.

IgG: IgG as used herein is a monomeric immunoglobulin, built of two heavy chains and two light chains. Each molecule has two antigen binding sites.

Isolated antibody: The term "isolated" antibody as used herein is an antibody which has been identified and separated and/or recovered from a component of its natural environment.

Immunoconjugates: The disclosure also pertains to immunoconjugates comprising an antibody conjugated to a cytotoxic agent such as a chemotherapeutic agent, toxin (e.g., an enzymatically active toxin of bacterial, fungal, plant, or animal origin, or fragments thereof), or a radioactive isotope (i.e., a radioconjugate). Enzymatically active toxins and fragments thereof that can be used include diphtheria A chain, nonbinding active fragments of diphtheria toxin, exotoxin A chain (from Pseudomonas aeruginosa), ricin A chain, abrin A chain, modeccin A chain, alpha-sarcin, Aleurites fordii proteins, dianthin proteins, Phytolaca americana proteins (PAPI, PAPII, and PAP-S), momordica charantia inhibitor, curcin, crotin, sapaonaria officinalis inhibitor, gelonin, mitogellin, restrictocin, phenomycin, enomycin, and the tricothecenes. A variety of radionuclides are available for the production of radioconjugated antibodies. Conjugates of the antibody and cytotoxic agent are made using a variety of bifunctional protein-coupling agents such as N-succinimidyl-3-(2-pyridyldithiol) propionate (SPDP), iminothiolane (IT), bifunctional derivatives of imidoesters (such as dimethyl adipimidate HCL), active esters (such as disuccinimidyl suberate), aldehydes (such as glutaraldehyde), bis-azido compounds (such as bis(p-azidobenzoyl)hexanediamine), bis-diazonium derivatives (such as bis-(p-diazoniumbenzoyl)-ethylenediamine), diisocyanates (such as tolyene 2,6-diisocyanate), and bis-active fluorine compounds (such as 1,5-difluoro-2,4-dinitrobenzene).

Immune monitoring: Immune monitoring of the present disclosure refers to testing of immune status in the diagnosis and therapy of diseases like but not limited to cancer, immunoproliferative and immunodeficiency disorders, autoimmune abnormalities, and infectious disease. It also refers to testing of immune status before, during and after vaccination and transplantation procedures.

Immune monitoring process: a serie of one or more immune monitoring analysis

Indirect detection of T cells: Indirect detection of T cells is used interchangeably herein with Indirect detection of TCR and indirect detection of T cell receptor. As used herein indirect detection of T cells is detection of the binding interaction between a specific T cell receptor and a MHC multimer by measurement of the effect of the binding interaction. Ionophore: ionophore, as used herein, is a lipid-soluble molecule usually synthesized by microorganisms capable of transporting ions.

Label: Label herein is used interchangeable with labeling molecule. Label as described herein is an identifiable substance that is detectable in an assay and that can be attached to a molecule creating a labeled molecule. The behavior of the labeled molecule can then be studied.

Labelling: Labelling herein means attachment of a label to a molecule.

Lanthanide: lanthanide, as used herein, series comprises the 15 elements with atomic numbers 57 through 71, from lanthanum to lutetium.

Linker molecule: Linker molecule and linker is used interchangeable herein. A linker molecule is a molecule that covalently or non-covalently connects two or more molecules, thereby creating a larger complex consisting of all molecules including the linker molecule.

LDA: limiting dilution assay

Liposomes: The term liposomes as used herein is defined as a spherical vesicle with a membrane composed of a phospholipid and cholesterol bilayer. Liposomes, usually but not by definition, contain a core of aqueous solution; lipid spheres that contain no aqueous material are called micelles.

Immunoliposomes: The antibodies disclosed herein can also be formulated as immunoliposomes. Liposomes comprising the antibody are prepared by methods known in the art, such as described in Epstein et al., Proc. Natl. Acad. Sci. USA 82: 3688 (1985); Hwang et al., Proc. Natl. Acad. Sci. USA 77: 4030 (1980); and U.S. Pat. Nos. 4,485,045 and 4,544,545. Particularly useful liposomes can be generated by the reverse-phase evaporation method with a lipid composition comprising phosphatidylcholine, cholesterol, and PEG-derivatized phosphatidylethanolamine (PEG-PE).

Immuno profiling: Immuno profiling as used herein defines the profiling of an individual's antigen-specific T-cell repertoire

Marker: Marker is used interchangeably with marker molecule herein. A marker is molecule that specifically associates covalently or non-covalently with a molecule belonging to or associated with an entity.

MHC: Denotes the major histocompatibility complex.

MHC I is used interchangeably herein with MHC class I and denotes the major histocompatibility complex class I.

MHC II is used interchangeably herein with MHC class II and denotes the major histocompatibility complex class I.

MHC molecule: a MHC molecule as used everywhere herein is defined as any MHC class I molecule or MHC class II molecule as defined herein.

A "MHC Class I molecule" as used everywhere herein is used interchangeably with MHC I molecule and is defined as a molecule which comprises 1-3 subunits, including a MHC I heavy chain, a MHC I heavy chain combined with a MHC I beta2microglobulin chain, a MHC I heavy chain combined with MHC I beta2microglobulin chain through a flexible linker, a MHC I heavy chain combined with an antigenic peptide, a MHC I heavy chain combined with an antigenic peptide through a linker, a MHC I heavy chain/ MHC I beta2microglobulin dimer combined with an antigenic peptide, and a MHC I heavy chain/ MHC I beta2microglobulin dimer combined with an antigenic peptide through a flexible linker to the heavy chain or beta2microglobulin. The MHC I molecule chains can be changed by substitution of single or by cohorts of native amino acids, or by inserts, or deletions to enhance or impair the functions attributed to said molecule. MHC complex: MHC complex is herein used interchangeably with MHC-peptide complex, and defines any MHC I and/or MHC II molecule combined with antigenic peptide unless it is specified that the MHC complex is empty, i.e. is not complexed with antigenic peptide

MHC Class I like molecules (including non-classical MHC Class I molecules) include CD1d, HLA E, HLA G, HLA F, HLA H, MICA, MIC B, ULBP-1, ULBP-2, and ULBP-3.

A "MHC Class II molecule" as used everywhere herein is used interchangeably with MHC II molecule and is defined as a molecule which comprises 2-3 subunits including a MHC II alpha-chain and a MHC II beta-chain (i.e. a MHC II alpha/beta-dimer), an MHC II alpha/beta dimer with an antigenic peptide, and an MHC II alpha/beta dimer combined with an antigenic peptide through a flexible linker to the MHC II alpha or MHC II beta chain, a MHC II alpha/beta dimer combined through an interaction by affinity tags e.g. jun-fos, a MHC II alpha/beta dimer combined through an interaction by affinity tags e.g. jun-fos and further combined with an antigenic peptide through a flexible linker to the MHC II alpha or MHC II beta chain. The MHC II molecule chains can be changed by substitution of single or by cohorts of native amino acids, or by inserts, or deletions to enhance or impair the functions attributed to said molecule. Under circumstances where the MHC II alpha-chain and MHC II beta-chain have been fused, to form one subunit, the "MHC Class II molecule" can comprise only 1 subunit or 2 subunits if antigenic peptide is also included.

MHC Class II like molecules (including non-classical MHC Class II molecules) include HLA DM, HLA DO, I-A beta2, and I-E beta2.

A "peptide free MHC Class I molecule" is used interchangeably herein with "peptide free MHC I molecule" and as used everywhere herein is meant to be a MHC Class I molecule as defined above with no peptide.

A "peptide free MHC Class II molecule" is used interchangeably herein with "peptide free MHC II molecule" and as used everywhere herein is meant to be a MHC Class II molecule as defined above with no peptide.

Such peptide free MHC Class I and II molecules are also called "empty" MHC Class I and II molecules.

The MHC molecule may suitably be a vertebrate MHC molecule such as a human, a mouse, a rat, a porcine, a bovine or an avian MHC molecule. Such MHC complexes from different species have different names. E.g. in humans, MHC complexes are denoted HLA. The person skilled in the art will readily know the name of the MHC complexes from various species.

In general, the term "MHC molecule" is intended to include all alleles. By way of example, in humans e.g. HLA A, HLA B, HLA C, HLA D, HLA E, HLA F, HLA G, HLA H, HLA DR, HLA DQ and HLA DP alleles are of interestshall be included, and in the mouse system, H-2 alleles are of interestshall be included. Likewise, in the rat system RT1-alleles, in the porcine system SLA-alleles, in the bovine system BoLA, in the avian system e.g. chicken-B alleles, are of interestshall be included.

"MHC complexes" and "MHC constructs" are used interchangeably herein.

By the terms "MHC complexes" and "MHC multimers" as used herein are meant such complexes and multimers thereof, which are capable of performing at least one of the functions attributed to said complex or multimer. The terms include both classical and non-classical MHC complexes. The meaning of "classical" and "non-classical" in connection with MHC complexes is well known to the person skilled in the art. Non-classical MHC complexes are subgroups of MHC-like complexes. The term "MHC complex" includes MHC Class I molecules, MHC Class II molecules, as well as MHC-like molecules (both Class I and Class II), including the subgroup non-classical MHC Class I and Class II molecules.

MHC multimer: The terms MHC multimer, MHC-multimer, MHCmer and MHC'mer herein are used interchangeably, to denote a complex comprising more than one MHC-peptide complexes, held together by covalent or non-covalent bonds.

Monoclonal antibodies: Monoclonal antibodies, as used herein, are antibodies that are identical because they were produced by one type of immune cell and are all clones of a single parent cell.

## EP 2 254 592 B1

Monovalent antibodies: The antibodies in the present disclosure can be monovalent antibodies. Methods for preparing monovalent antibodies are well known in the art. For example, one method involves recombinant expression of immunoglobulin light chain and modified heavy chain. The heavy chain is truncated generally at any point in the Fc region so as to prevent heavy chain crosslinking. Alternatively, the relevant cysteine residues are substituted with another amino acid residue or are deleted so as to prevent crosslinking. In vitro methods are also suitable for preparing monovalent antibodies. Digestion of antibodies to produce fragments thereof, particularly, Fab fragments, can be accomplished using routine techniques known in the art.

Multimerization domain: A multimerization domain is a molecule, a complex of molecules, or a solid support, to which one or more MHC or MHC-peptide complexes can be attached. A multimerization domain consist of one or more carriers and/or one or more scaffolds and may also contain one or more linkers connecting carrier to scaffold, carrier to carrier, scaffold to scaffold. The multimerization domain may also contain one or more linkers that can be used for attachment of MHC complexes and/or other molecules to the multimerization domain. Multimerization domains thus include IgG, streptavidin, streptactin, micelles, cells, polymers, beads and other types of solid support, and small organic molecules carrying reactive groups or carrying chemical motifs that can bind MHC complexes and other molecules.

Nanobodies: Nanobodies as used herein is a type of antibodies derived from camels, and are much smaller than traditional antibodies.

Neutralizing antibodies: neutralizing antibodies as used herein is an antibody which, on mixture with the homologous infectious agent, reduces the infectious titer.

NMR: NMR (Nuclear magnetic resonance), as used herein, is a physical phenomenon based upon the quantum mechanical magnetic properties of an atom's nucleus. NMR refers to a family of scientific methods that exploit nuclear magnetic resonance to study molecules.

Non-covalent: The term noncovalent bond as used herein is a type of chemical bond that does not involve the sharing of pairs of electrons, but rather involves more dispersed variations of electromagnetic interactions.

Nucleic acid duplex: A nucleic acid is a complex, high-molecular-weight biochemical macromolecule composed of nucleotide chains that convey genetic information. The most common nucleic acids are deoxyribonucleic acid (DNA) and ribonucleic acid (RNA).

Nucleophilic: a nucleophile, as used herein, is a reagent that forms a chemical bond to its reaction partner (the electrophile) by donating both bonding electrons.

"One or more" as used everywhere herein is intended to include one and a plurality.

A "peptide free MHC Class I molecule" as used everywhere herein is meant to be a MHC Class I molecule as defined above with no peptide.

A "peptide free MHC Class II molecule" as used everywhere herein is meant to be a MHC Class II molecule as defined above with no peptide.

Such peptide free MHC Class I and II molecules are also called "empty" MHC Class I and II molecules.

Pegylated: pegylated, as used herein, is conjugation of Polyethylene glycol (PEG) to proteins.

Peptide or protein: Any molecule composed of at least two amino acids. Peptide normally refers to smaller molecules of up to around 30 amino acids and protein to larger molecules containing more amino acids.

Phosphorylated; phosphorylated, as used herein, is is the addition of a phosphate ($PO_4$) group to a protein molecule or a small molecule.

PNA: PNA (Peptide nucleic acid) as used herein is a chemical similar to DNA or RNA. PNA is not known to occur naturally in existing life on Earth but is artificially synthesized and used in some biological research and medical treatments. DNA and RNA have a deoxyribose and ribose sugar backbone, respectively, whereas PNA's backbone is composed of repeating N-(2-aminoethyl)-glycine units linked by peptide bonds. The various purine and pyrimidine bases are linked to the backbone by methylene carbonyl bonds. PNAs are depicted like peptides, with the N-terminus at the first (left) position and the C-terminus at the right.

"A plurality" as used everywhere herein should be interpreted as two or more.

This applies i.a. to the MHC peptide complex and the binding entity. When a plurality of MHC peptide complexes is attached to the multimerization domain, such as a scaffold or a carrier molecule, the number of MHC peptide complexes need only be limited by the capacity of the multimerization domain.

Polyclonal antibodies: a polyclonal antibody as used herein is an antibody that is derived from different B-cell lines. They are a mixture of immunoglobulin molecules secreted against a specific antigen, each recognising a different epitope.

Polymer: the tern polymer as used herein is defined as a compound composed of repeating structural units, or monomers, connected by covalent chemical bonds.

Polypeptide: Peptides are the family of short molecules formed from the linking, in a defined order, of various α-amino acids. The link between one amino acid residue and the next is an amide bond and is sometimes referred to as a peptide bond. Longer peptides are reffered to as proteins or polypeptide.

11

Polysaccharide: The term polysaccharide as used herein is defined as polymers made up of many monosaccharides joined together by glycosidic linkages.

Radicals: radicals, as used herein, are atomic or molecular species with unpaired electrons on an otherwise open shell configuration. These unpaired electrons are usually highly reactive, so radicals are likely to take part in chemical reactions.

Radioactivity: Radioactive decay is the process in which an unstable atomic nucleus loses energy by emitting radiation in the form of particles or electromagnetic waves. RNA: RNA (Ribonucleic acid) as used herein is a nucleic acid polymer consisting of nucleotide monomers that plays several important roles in the processes that translate genetic information from deoxyribonucleic acid (DNA) into protein products.

RNA: RNA (Ribonucleic acid) as used herein is a nucleic acid polymer consisting of nucleotide monomers that plays several important roles in the processes that translate genetic information from deoxyribonucleic acid (DNA) into protein products

Scaffold: A scaffold is typically an organic molecule carrying reactive groups, capable of reacting with reactive groups on a MHC-peptide complex. Particularly small organic molecules of cyclic structure (e.g. functionalized cycloalkanes or functionalized aromatic ring structures) are termed scaffolds. Scaffold and carrier are used interchangeably herein where scaffold typically refers to smaller molecules of a multimerization domain and carrier typically refers to larger molecule and/or cell like structures.

Staining: In this disclosure staining means specific or unspecific labelling of cells by binding labeled molecules to defined proteins or other structures on the surface of cells or inside cells. The cells are either in suspension or part of a tissue. The labeled molecules can be MHC multimers, antibodies or similar molecules capable of binding specific structures on the surface of cells.

Streptavidin: Streptavidin as used herein is a tetrameric protein purified from the bacterium Streptomyces avidinii. Streptavidin is widely use in molecular biology through its extraordinarily strong affinity for biotin.

Sugar: Sugars as used herein include monosaccharides, disaccharides, trisaccharides and the oligosaccharides - comprising 1, 2, 3, and 4 or more monosaccharide units respectively.

Therapy: Treatment of illness or disability

Vaccine: A vaccine is an antigenic preparation used to establish immunity to a disease or illness and thereby protect or cure the body from a specific disease or illness. Vaccines are either prophylactic and prevent disease or therapeutic and treat disease. Vaccines may contain more than one type of antigen and is then called a combined vaccine.

Vaccination: The introduction of vaccine into the body of human or animals for the purpose of inducing immunity.

'B.L.' is an abbreviation for Bind level.

'Aff.' is an abbreviation for affinity.

## DETAILED DESCRIPTION

The invention is defined by the appended claims. Any embodiment not falling under the scope of the appended claims does not form part of the invention.

The present disclosure in one aspect refers to a MHC monomer comprising a-b-P, or a MHC multimer comprising (a-b-P)$_n$, wherein n > 1,

wherein a and b together form a functional MHC protein capable of binding the antigenic peptide P,
wherein (a-b-P) is the MHC-peptide complex formed when the antigenic peptide P binds to the functional MHC protein, and wherein each MHC peptide complex of a MHC multimer is associated with one or more multimerization domains.

Another aspect of the present disclosure refers to an antigenic peptide P or an antigenic polypeptide featuring one or more anitgenic peptides P.
The antigenic peptide P is in one embodiment of the present disclosure a Borrelia peptide such as e.g. a Borrelia burgdorferi B31 peptide, a Borreila afzelii PKo peptide or a Borrelia garinii PBi peptide.

MHC monomers and MHC multimers comprising one or more MHC peptide complexes of class 1 or class 2 MHC are covered by the present disclosure. Accordingly, the antigenic peptide P can have a length of e.g. 8, 9, 10, 11, 12, 13, 14, 15, 16, 16-20, or 20-30 amino acid residues.

Examples of the antigenic peptide P is provided herein below. In one embodiment of the present disclosure, the antigenic peptide P can be selected from the group consisting of sequences disclosed in the "Sequence Listing" and annotated consecutively (using integers) starting with SEQ ID NO:1 and ending with SEQ ID NO:217791 or any fragment thereof.

In another aspect the present disclosure is directed to a composition comprising a plurality of MHC monomers and/or MHC multimers according to the present disclosure, wherein the MHC multimers are identical or different, and a carrier.

The present disclosure further relates to a method for detection of antigen-specific T cells, said method comprising the steps of 1) providing the MHC multimer described above, 2) providing a population of antigen-specific T cells, and 3) detecting antigen-specific T cells specific for the peptide P of the MHC multimer.

The present disclosure also relates to a method for detection of antigen-specific T cells, said method comprising the steps of 1) providing the antigenic peptid or antigenic polypeptide described above, 2) providing a population of antigen-specific T cells, and 3) detecting antigen-specific T cells specific for the antigenic peptide P in complex with MHC molecules.

In a further embodiment the present disclosure relates to a method for counting of antigen-specific T cells, said method comprising the steps of 1) providing the MHC multimer described above, 2) providing a population of antigen-specific T cells, and 3) counting antigen-specific T cells specific for the peptide P of the MHC multimer.

The present disclosure also relates to a method for sorting of antigen-specific T cells, said method comprising the steps of 1) providing the MHC multimer described above, 2) providing a population of antigen-specific T cells, and 3) sorting antigen-specific T cells specific for the peptide P of the MHC multimer.

In yet another embodiment the present disclosure relates to a method for isolation of antigen-specific T cells, said method comprising the steps of 1) providing the MHC multimer described above, 2) providing a population of antigen-specific T cells, and 3) isolating antigen-specific T cells specific for the peptide P of the MHC multimer.

In yet another aspect of the present disclosure there is provided a kit comprising an antigenic peptide, an antigenic polypeptide, a MHC monomer or a MHC multimer according to the present disclosure, or a composition according to the present disclosure, and at least one additional component, such as a positive control and/or instructions for use.

In a still further aspect of the present disclosure there is provided a method for immune monitoring one or more diseases comprising monitoring of antigen-specific T cells, said method comprising the steps of

i) providing the MHC monomer or MHC multimer or individual components thereof according to the present disclosure,
ii) providing a population of antigen-specific T cells or individual antigen-specific T cells, and
iii) measuring the number, activity or state and/or presence of antigen-specific of T cells specific for the peptide P of the said MHC monomer or MHC multimer, thereby immune monitoring said one or more diseases.

Or i) providing the antigenic peptide or antigenic polypeptide according to the present disclosure,

ii) providing a population of antigen-specific T cells or individual antigen-specific T cells, and providing antigen presenting cells expressing MHC molecules on their surface

iii) measuring the number, activity or state and/or presence of antigen-specific of T cells specific for the antigenic peptide P in complex with one or more MHC molecules displayed on the surface of the antigen presenting cell, thereby immune monitoring said one or more diseases.

In yet another aspect of the present disclosure there is provided a method for diagnosing one or more diseases comprising immune monitoring of antigen-specific T cells, said method comprising the following steps: of

i) providing the MHC monomer or MHC multimer or individual components thereof according to the present disclosure, or individual components thereof,
ii) providing a population of antigen-specific T cells or individual antigen-specific T cells, and
iii) measuring the number, activity or state and/or presence of T cells specific for said MHC monomer or the peptide P of the MHC multimer, thereby diagnosing said one or more diseases.

Or

i) providing the antigenic peptide or antigenic polypeptide according to the present disclosure,
ii) providing a population of antigen-specific T cells or individual antigen-specific T cells, and providing antigen presenting cells expressing MHC molecules on their surface
iii) measuring the number, activity or state and/or presence of T cells specific for said antigenic peptide P in complex with one or more MHC molecules displayed on the surface of the antigen presenting cell, , thereby diagnosing said one or more diseases.

There is also provided a method for isolation of one or more antigen-specific T cells, said method comprising the steps of

i) providing the MHC monomer or MHC multimer or individual components thereof according to the present disclosure, or individual components thereof, and

ii) providing a population of antigen-specific T cells or individual antigen-specific T cells, and

iii) thereby isolating said T cells specific for the peptide P of the said MHC monomer or MHC multimer.

The present disclosure makes it possible to pursue different immune monitoring methods using the MHC monomers, MHC multimers, antigenic peptides or antigenic polypeptides according to the present disclosure. The immune monitoring methods include e.g. flow cytometry, ELISPOT, LDA, Quantiferon and Quantiferon-like methods. Using the above-cited methods, the MHC monomers and/or the MHC multimers can be provided as a MHC peptide complex, or the antigenic peptide and the MHC monomer and/or multimer can be provided separately.

Accordingly, recognition of TCR's can be achieved by direct or indirect detection, e.g. by using one or more of the following methods:

ELISPOT technique using indirect detection, e.g. by adding the antigenic peptide optionally associated with a MHC monomer or MHC multimer, followed by measurement of INF-gamma secretion from a population of cells or from individual cells.

Another technique involves a Quantiferon-like detection assay, e.g. by using indirect detection, e.g. by adding the antigenic peptide optionally associated with a MHC monomer or MHC multimer, followed by measurement of INF-gamma secretion from a population of cells or from individual cells.

Flow cytometry offers another alternative for performing detection assays, e.g. by using direct detection (e.g. of MHC tetramers), e.g. by adding the antigenic peptide optionally associated with a MHC monomer or MHC multimer, followed by detection of a fluorescein label, thereby measuring the number of TCRs on specific T-cells.

Flow cytometry can also be used for indirect detection, e.g. by adding the antigenic peptide optionally associated with a MHC monomer or MHC multimer, followed by addition of a "cell-permeabilizing factor", and subsequent measurement of an intracellular component (e.g. INF-gamma mRNA), from individual cells or populations of cells.

By using the above-mentioned and other techniques, one can diagnose and/or monitor e.g. infectious diseases caused e.g. by mycobacetrium, Gram positive bacteria, Gram negative bacteria, Spirochetes, intracellular bacterium, extracelular bacterium, Borrelia, TB, CMV, HPV, Hepatitis, BK, fungal organisms and microorganisms. The diagnosis and/or monitoring of a particular disease can greatly aid in directing an optimal treatment of said disease in an individual.

In still further aspects of the present disclosure there is provided a method for performing a vaccination of an individual in need thereof, said method comprising the steps of

providing an antigenic peptide, an antigenic polypeptide, a MHC monomer or a MHC multimer, according to the present disclosure, or the individual components thereof, and

administering said antigenic peptide, antigenic polypeptide, MHC monomer or MHC multimer to said individual and obtaining a protective immune response, thereby performing a vaccination of the said individual.

In yet another embodiment of the present disclosure there is provided a method for performing therapeutic treatment of an individual comprising the steps of

Providing the MHC multimer according to the present disclosure, or individual components thereof, and

Isolating or obtaining T-cells from a source, such as an individual or an ex-vivo library or cell bank, wherein said isolated or obtained T-cells are specific for said provided MHC multimer,

Optionally manipulating said T-cells, and

Introducing said isolated or obtianed T-cells into an individual to be subjected to a therapeutic treatment, wherein the individual can be the same individual or a different individual from the source individual.

There is also provided a method comprising one or more steps for minimizing undesired binding of the MHC multimer according to the present disclosure. This method is disclosed herein below in more detail.

In further aspects the present disclosure provides:

A method for performing a control experiment comprising the step of counting of particles comprising the MHC multimer according to the present disclosure.

A method for performing a control experiment comprising the step of sorting of particles comprising the MHC multimer according to the present disclosure.

A method for performing a control experiment comprising the step of performing flow cytometry analysis of particles comprising the MHC multimer according to the present disclosure.

A method for performing a control experiment comprising the step of performing a immunohistochemistry analysis comprising the MHC multimer according to the present disclosure.

A method for performing a control experiment comprising the step of performing a immunocytochemistry analysis comprising the MHC multimer according to the present disclosure.

A method for performing a control experiment comprising the step of performing an ELISA analysis comprising the MHC multimer according to the present disclosure.

In a still further aspect of the present disclosure there is provided a method for generating MHC multimers according to the present disclosure, said method comprising the steps of

i) providing one or more antigenic peptides P; and/or

ii) providing one or more functional MHC proteins,

iii) optionally providing one or more multimerization domains, and

iv) contacting the one or more peptides P and the one or more functional MHC proteins and the one or more multimerization domains simultaneously or sequentialy in any order, thereby obtaining MHC multimers according to the present disclosure.

The method can also be performed by initially providing one or more antigenic peptide(s) P and optionally one or more functional MHC proteins to generate a MHC-peptide complex (a-b-P); subsequently or simultaneusly providing one or more multimerisation domain(s); and reacting the one or more MHC-peptide complexes and the one or more multimerization domain(s) to generate a MHC multimer according to the present disclosure.

In one aspect, the present disclosure is directed to novel MHC complexes optionally comprising a multimerization domain preferably comprising a carrier molecule and/or a scaffold.

There is also provided a MHC multimer comprising 2 or more MHC-peptide complexes and a multimerization domain to which the 2 or more MHC-peptide complexes are associated. The MHC multimer can generally be formed by association of the 2 or more MHC-peptide complexes with the multimerization domain to which the 2 or more MHC-peptide complexes are capable of associating.

The multimerization domain can be a scaffold associated with one or more MHC-peptide complexes, or a carrier associated with one or more, preferably more than one, MHC-peptide complex(es), or a carrier associated with a plurality of scaffolds each associated with one or more MHC-peptide complexes, such as 2 MHC-peptide complexes, 3 MHC-peptide complexes, 4 MHC-peptide complexes, 5 MHC-peptide complexes or more than 5 MHC-peptide complexes. Accordingly, multimerization domain collectively refers to each and every of the above. It will be clear from the detailed description of the disclosure provided herein below when the multimerization domain refers to a scaffold or a carrier or a carrier comprising one or more scaffolds.

Generally, when a multimerization domain comprising a carrier and/or a scaffold is present, the MHC complexes can be associated with this domain either directly or via one or more binding entities. The association can be covalent or non-covalent.

Accordingly, there is provided in one embodiment of the present disclosure a MHC complex comprising one or more entities $(a-b-P)_n$, wherein a and b together form a functional MHC protein capable of binding a peptide P, and wherein (a-b-P) is the MHC-peptide complex formed when the peptide P binds to the functional MHC protein, said MHC complex optionally further comprising a multimerization domain comprising a carrier molecule and/or a scaffold. "MHC complex" refers to any MHC complex, including MHC monomers in the form of a single MHC-peptide complex and MHC multimers comprising a multimerization domain to which more than one MHC peptide complex is associated.

When the disclosure is directed to complexes comprising a MHC multimer, i.e. a plurality of MHC peptide complexes of the general composition $(a-b-P)_n$ associated with a multimerization domain, n is by definition more than 1, i.e. at least 2 or more. Accordingly, the term "MHC multimer" is used herein specifically to indicate that more than one MHC-peptide complex is associated with a multimerization domain, such as a scaffold or carrier or carrier comprising one or more scaffolds. Accordingly, a single MHC-peptide complex can be associated with a scaffold or a carrier or a carrier comprising a scaffold and a MHC-multimer comprising 2 or more MHC-peptide complexes can be formed by association of the individual MHC-peptide complexes with a scaffold or a carrier or a carrier comprising one or more scaffolds each associated with one or more MHC-peptide complexes.

When the MHC complex comprises a multimerization domain to which the n MHC-peptide complexes are associated, the association can be a covalent linkage so that each or at least some of the n MHC-peptide complexes is covalently linked to the multimerization domain, or the association can be a non-covalent association so that each or at least some of the n MHC-peptide complexes are non-covalently associated with the multimerization domain.

The MHC complexes of the disclosure may be provided in non-soluble or soluble form, depending on the intended application.

Effective methods to produce a variety of MHC complexes comprising highly polymorphic human HLA encoded proteins makes it possible to perform advanced analyses of complex immune responses, which may comprise a variety of peptide epitope specific T-cell clones.

One of the benefits of the MHC complexes of the present disclosure is that the MHC complexes overcome low intrinsic affinities of monomer ligands and counter receptors. The MHC complexes have a large variety of applications that include targeting of high affinity receptors (e.g. hormone peptide receptors for insulin) on target cells. Taken together poly-ligand binding to target cells has numerous practical, clinical and scientifically uses.

Thus, the present disclosure provides MHC complexes which present mono-valent or multi-valent binding sites for MHC-peptide recognising cells, such as MHC complexes optionally comprising a multimerization domain, such as a scaffold or a carrier molecule, which multimerization domain have attached thereto, directly or indirectly via one or more linkers, covalently or non-covalently, one or more MHC peptide complexes. "One or more" as used herein is intended to include one as well as a plurality, such as at least 2. This applies i.a. to the MHC peptide complexes and to the binding entities of the multimerization domain. The scaffold or carrier molecule may thus have attached thereto a MHC peptide complex or a plurality of such MHC peptide complexes, and/or a linker or a plurality of linkers.

## PRODUCT

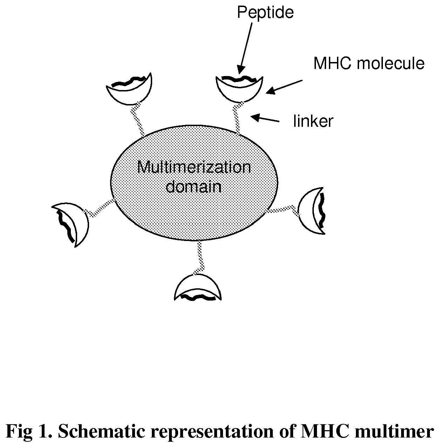

In one embodiment of the present disclosure the product is a MHC monomer or a MHC multimer as described above. As used in the description of this disclosure, the term "MHC multimers" will be used interchangeably with the terms MHC'mers and MHCmers, and will include any number, (larger than one) of MHC-peptide complexes, held together in a large complex by covalent or non-covalent interactions between a multimerization domain and one or more MHC-peptide complexes, and will also include the monomeric form of the MHC-peptide complex, i.e. a MHC-peptide complex that is not attached to a multimerization domain. The multimerization domain consists of one or more carriers and/or one or more scaffolds while the MHC-peptide complex consists of MHC molecule and antigenic peptide. MHC-peptide complexes may be attached to the multimerization domain through one or more linkers. A schematic representation of a MHC multimer is presented in figure 1.

In another embodiment of the present disclosure the product is antigenic peptide or antigenic polypeptide containing one or more antigenic peptide sequences. As used in the description of this disclosure the term antigenic peptide will be used interchangeably with the term binding peptide and refers to any peptide molecule that is bound or able to bind into the binding groove of either MHC class 1 or MHC class 2.

In the following the design and generation of antigenic peptides, antigenic polypeptides and the different components of MHC monomers and/or MHC multimers are described.

### Design and generation of antigenic peptides

Antigenic peptides of the present disclosure may be used in processes of the present disclosure either as part of MHC monomers, MHC multimers or antigenic polypeptides or used themselves as a product. Antigenic polypeptide and antigenic peptide products will later in the process they are used for, bind MHC molecules and thereby generate MHC monomers and/or MHC multimers, e.g. when used as a vaccine the antigenic peptides may bind MHC molecules on cells inside the body or when used for an immune monitoring process antigenic peptides binds MHC molcules present in the sample they are applied to.

Therefore the features of and principles for design and generation of antigenic peptides used themselves as a product or used in MHC monormers, MHC multimers or in antigenic polypeptides are identical and will be described in more detail in the following.

MHC class 1 protein typically binds octa-, nona-, deca- or ondecamer (8-, 9-, 10,- 11-mer) peptides in their peptide binding groove. The individual MHC class 1 alleles have individual preferences for the peptide length within the given range. MHC class 2 proteins typically bind peptides with a total length of 13-18 amino acids, comprising a 9'-mer core motif containing the important amino acid anchor residues. However the total length is not strictly defined, as opposed to most MHC class 1 molecules.

For some of the MHC alleles the optimal peptide length and the preferences for specific amino acid residues in the so called anchor positions are known.

To identify high-afinity binding peptides derived from a specific protein for a given MHC allele it is necessary to systematically work through the amino acid sequence of the protein to identify the putative high-affinity binding peptides. Although a given peptide is a binder it is not necessarily a functional T-cell epitope. Functionality needs to be confirmed by a functional analysis e.g. ELISPOT, CTL killing assay or flow cytometry assay as described elsewhere herein.

The binding affinity of the peptide for the MHC molecules can for some MHC molecules be predicted in databases such as www.syfpeithi.de; http://www-bimas.cit.nih.gov/molbio/hla_bind/; www.cbs.dtu.dk/services/NetMHC/; www.cbs.dtu.dk/services/NetMHCII/

### Design of binding peptides

The first step in the design of binding peptides is obtaining the protein's amino acid sequence. In many cases the amino acid sequence of the protein from which antigenic peptides have to be identified from are known. However, when only the genomic DNA sequences are known, i.e. the reading frame and direction of transcription of the genes is unknown, the DNA sequence needs to be translated in all three reading frames in both directions leading to a total of six amino acid sequences for a given genome. From these amino acid sequences binding peptides can then be identified as described below. In organisms having intron/exon gene structure the present approach must be modified accordingly, to identify peptide sequence motifs that are derived by combination of amino acid sequences derived partly from two separate introns. cDNA sequences can be translated into the actual amino acid sequences to allow peptide identification.

In cases where the protein sequence is known, these can directly be used to predict peptide epitopes.

Binding peptide sequences can be predicted from any protein sequence by either a total approach, generating binding peptide sequences for potentially any MHC allele, or by a directed approach, identifying a subset of binding peptides with certain preferred characteristics such as affinity for MHC protein, specificity for MHC protein, likelihood of being formed by proteolysis in the cell, and other important characteristics.

Design of MHC class 1 binding peptide sequence

Many parameters influence the design of the individual binding peptide, as well as the choice of the set of binding peptides to be used in a particular application. Important characteristics of the MHC-peptide complex are physical and chemical (e.g. proteolytic) stability. The relevance of these parameters must be considered for the production of the antigenic peptides, the antigenic polypeptides, the MHC-peptide complexes and the MHC multimers, as well as for their use in a given application. As an example, the stability of the MHC-peptide complex in assay buffer (e.g. PBS), in blood, or in the body can be very important for a particular application.

In the interaction of the MHC-peptide complex with the TCR, a number of additional characteristics must be considered, including binding affinity and specificity for the TCR, degree of cross-talk, undesired binding or interaction with other TCRs. Finally, a number of parameters must be considered for the interaction of MHC-peptide complexes, MHC multimers, antigenic peptides or antigenic polypeptides with the sample or individual it is being applied to. These include immunogenicity, allergenicity, as well as side effects resulting from un-desired interaction with "wrong" T cells, including cross-talk with e.g. autoimmune diseases and un-desired interaction with other cells than antigen-specific T cells.

For some applications, e.g. immuno profiling of an individual's immune response focused on one antigen, it is preferred that all possible binding peptides of that antigen are included in the application (i.e. the "total approach" for the design of binding peptides described below). For other applications, e.g vaccines it may be adequate to include a few or just one binding peptide for each of the HLA-alleles included in the application (i.e. the "directed approach" whereby only the most potent binding peptides can be included). Personalized diagnostics, therapeutics and vaccines will often fall in-between these two extremes, as it will only be necessary to include a few or just one binding peptide in e.g. a vaccine targeting a given individual, but the specific binding peptide may have to be picked from binding peptides designed by the total approach, and identified through the use of immuno profiling studies involving all possible binding peptides. The principles of immuno profiling is described elsewhere herein.

**a) Total approach**

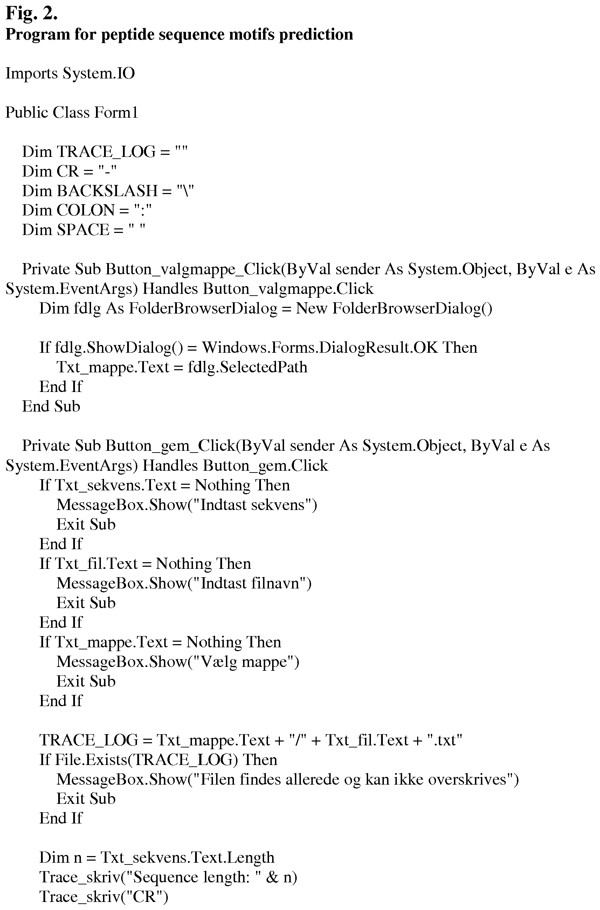

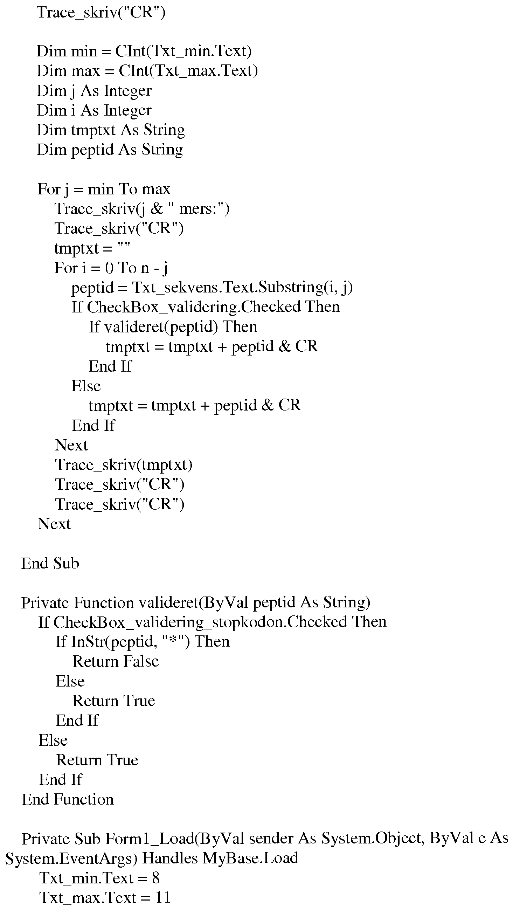

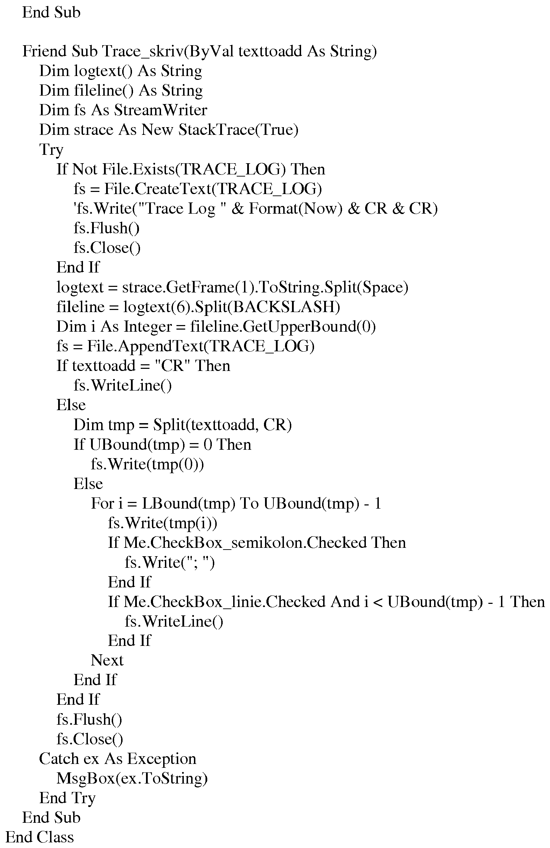

The MHC class 1 binding peptide prediction is done as follows using the total approach. The actual protein sequence is split up into 8-, 9-, 10-, and 11-mer peptide sequences. This is performed by starting at amino acid position 1 identifying the first 8-mer; then move the start position by one amino acid identifying the second 8-mer; then move the start position by one amino acid, identifying the third 8-mer. This procedure continues by moving start position by one amino acid for each round of peptide identification. Generated peptides will be amino acid position 1-8, 2-9, 3-10 etc. This procedure can be carried out manually or by means of a software program (Figure 2). This procedure is then repeated in an identical fashion for 9-, 10 and 11-mers, respectively.

**b) Directed approach**

The directed approach identifies a preferred subset of binding peptides from the binding peptides generated in the total approach. This preferred subset is of particularly value in a given context.

One way to select subsets of antigenic peptides is to use consensus sequences to choose a set of relevant binding peptides able to bind the individual MHC allele and that will suit the "average" individual. Such consensus sequences often solely consider the affinity of the binding peptide for the MHC protein; in other words, a subset of binding peptides is identified where the designed binding peptides have a high probability of forming stable MHC-peptide complexes, but where it is uncertain whether this MHC-peptide complex is of high relevance in a population, and more uncertain whether this MHC-peptide complex is of high relevance in a given individual.

For class I MHC-alleles, the consensus sequence for a binding peptide is generally given by the formula

$$X1\text{-}X2\text{-}X3\text{-}X4\text{-}....\text{-}Xn,$$

where n equals 8, 9, 10, or 11, and where X represents one of the twenty naturally occurring amino acids, optionally modified as described elsewhere in this application. X1-Xn can be further defined. Thus certain positions in the consensus sequence are more likely to contribute to binding to a given MHC molecule than others.

Antigenic peptide-binding by MHC I is accomplished by interaction of specific amino acid side chains of the antigenic peptide with discrete pockets within the peptide-binding groove of the MHC molecule. The peptide-binding groove is formed by the $\alpha1$ and $\alpha2$ domains of the MHC I heavy chain and contains six pockets denoted A, B, C, D, E, F. For human HLA molecules the main binding energy associating antigenic peptide to MHC I is provided by interaction of amino acids in position 2 and at the c-terminus of the antigenic peptide with the B and F binding pockets of the MHC I molecule. The amino acids of the antigenic peptide being responsible for the main anchoring of the peptide to the MHC molecule are in the following called primary anchor amino acids and the motif they form for primary anchor motif. Other amino acid side chains of an antigenic peptide may also contribute to the anchoring of the antigenic peptide to the MHC molecule but to a lesser extent. Such amino acids are often referred to as secondary anchor amino acids and form a secondary anchor motif.

Different HLA alleles have different amino acids lining the various pockets of the peptide-binding groove enabling the various alleles to bind unique repertoires of antigenic peptides with specific anchor amino acid motifs. Thus for a selected consensus sequence certain positions are the socalled anchor positions and the selection of useful amino acids for these positions is limited to those able to fit into the corresponding binding pockets in the HLA molecule. For example for peptides binding HLA-A*02, X2 and X9 are primary anchor positions docking into the B and F pocket of the HLA molecule respectively, and useful amino acids at these two positions in the binding peptide are preferable limited to leucine or methionine for X2 and to valine or leucine at postion X9. In contrast the primary anchor positions of peptides binding HLA-B*08 are X3, X5 and X9 and the corresponding preferred amino acids at these positions are lysine at position X3, lysine or arginine at position X5 and leucine at position X9.

However, the different HLA alleles can be grouped into clusters or supertypes where the alleles of the supertype share peptide-binding pocket similarities in that they are able to recognize the same type of antigenic peptide primary anchor motif. Therefore antigenic peptides can be selcted on their ability to bind a given HLA molecule or a given HLA supertype on the basis of their amino acid sequence, e.g. the identity of the primary anchor motif.

Antigenic peptide primary anchor motifs of special interest of the present disclosure are listed in table 6.

**Table 6. HLA I supertype familie's and their antigenic peptide anchor motifs. Examples of usefull amino acids binding in pocket B and pocket F are shown as one letter code.**

| Supertype | Anchor motif | | | | Example of HLA allele's |
|---|---|---|---|---|---|
| | B pocket specificity | Example aa B pocket | F pocket specificity | Example aa F pocket | |
| A01 | Small and aliphatic | A, T, S, V, L, I, M, Q | Aromatic and large hydrophobic | F, W, Y, L, I, M | A*0101, A*2601, A*2602, A*2603, A*3002, A*3003, A*3004, A*3201 |
| A01/A03 | Small and aliphatic | A, T, S, V, L, I, M, Q | Aromatic and basic | Y, R, K | A*3001, A*3201, A*7401 |
| A01/A24 | Small, aliphatic and aromatic | A, S, T, V, L, I, M, Q, F, W, Y | Aromatic and large hydrophobic | F, W, Y, L, I, M | A*2902 |
| A02 | Small and aliphatic | A, T, S, V, L, I, M, Q | Aliphatic and small hydrophobic | L, I, V, M, Q, A | A*0201, A*0202, A*0203, A*0204, A*0205, A*0206, A*0207, A*0214, A*0217, A*6802, A*6901 |
| A03 | Small and aliphatic | A, T, S, V, L, I, M, Q | Basic | R, H, K | A*0301, A*1101, A*3101, A*3301, A*3303, A*6601, A*6801, A*7401 |
| A24 | Aromatic and aliphatic | F, W, Y, L, I, V, M, Q | Aromatic, aliphatic and hydrophbic | F, W, Y, L, I, V, M, Q, A | A*2301, A*2402 |
| B07 | Proline | P | Aromatic, aliphatic and hydrophbic | F, W, Y, L, I, V, M, Q, A | B*0702, B*0703, B*0705, B*1508, B*3501, B*3503, B*4201, B*5101, B*5102, B*5103, B*5301, B*5401, B*5501, B*5502, B*5601, B*6701, B*7801 |

(continued)

| Supertype | Anchor motif | | | | Example of HLA allele's |
|---|---|---|---|---|---|
| | *B pocket specificity* | *Example aa B pocket* | *F pocket specificity* | *Example aa F pocket* | |
| B08 | Undefined | | Aromatic, aliphatic and hydrophbic | F, W, Y, L, I, V, M, Q, A | B*0801, B*0802 |
| B27 | Basic | R, H, K | Aromatic, aliphatic, basic and hydrophbic | F, W, Y, L, I, V, M, Q, A, R, H, K | B*1402, B*1503, B*1509, B*1510, B*1518, B*2702, B*2703, B*2704, B*2705, B*2706, B*2707, B*2709, B*3801, B*3901, B*3902, B*3909, B*4801, B*7301 |
| B44 | Acidic | D, E | Aromatic, aliphatic and hydrophbic | F, W, Y, L, I, V, M, Q, A | B*1801, B*3701, B*4001, B*4002, B*4006, B*4402, B*4403, B*4501 |
| B58 | Small | A, S, T | Aromatic, aliphatic and hydrophbic | F, W, Y, L, I, V, M, Q, A | B*1516, B*1517, B*5701, B*5702, B*5801, B*5802 |
| B62 | Aliphatic | L, I, V, M, Q | Aromatic, aliphatic and hydrophbic | F, W, Y, L, I, V, M, Q, A | B*1501, B*1502, B*1512, B*1513, B*4501, B*4601, B*5201 |

Antigenic peptides able to bind a given MHC molecule do not necessarily have primary anchor amino acid residues compatible with both main anchoring pockets of the MHC molecule but may have one or no primary anchor amino acids suitable for binding the MHC molecule in question. However, having the preferred primary anchor motif for a given MHC allele increases the affinity of the antigenic peptide for that given allele and thereby the likelihood of making a stable and usefull MHC-peptide molecule.

Therefore in one embodiment of the present disclosure antigenic peptides can be identified and selected on their ability to bind a given HLA or other MHC molecule based on what amino acids they have at primary anchor positions and/or secondary anchor positions.

Software programs are available that use neural networks or established binding preferences to predict the interaction of specific binding peptides with specific MHC class I alleles. Examples of such programs are www.syfpeithi.de; www.imtech.res.in/raghava/propred1/index.html;www.cbs.dtu.dk/services/NetMHC/.

Another usefull parameter for prediction and selection of usefull antigenic peptides are the probability of the binding peptide in question to be generated *in vivo* by the proteolytic machinery inside cells. For example for a given antigen the combined action of endosolic, cytosolic and membrane bound protease activities as well as the TAP1 and TAP2 transporter specificities can be taken into consideration. However, the proteolytic activitiy varies a lot among individuals, and for personalized diagnostics, treatment or vaccination it may be desirable to disregard these general proteolytic data. An example of a program predicting the ability of antigenic peptides to be processed is www.cbs.dtu.dk/services/N etCTL/.

Using the above described principles individual peptides or a subset of peptides able to bind one or more types of MHC molecules and make stable MHC-peptide complexes can be identified. The identified peptides can then be tested for biological relevance in functional assays such as interferon gamma release assays (e.g. ELISPOT), cytotoxicity assays (e.g. CTL killing assays) or using other methods as described in the section "Detection" elsewhere herein. Alternatively or complementary hereto the abillity of the identified antigenic peptides to bind selected MHC molecules may be determined in binding assays like Biacore measurement, compettion assays or other assays usefull for measurement of binding of peptide to MHC molecules, known by persons skilled in the art.

Design of MHC class 2 binding peptide sequence.

**a) Total approach and b) directed approach**

The approach to predict antigenic peptide binders for MHC class 2 can be done in a similar way as described for MHC class 1 binding peptide prediction above. The change is the different size of the antigenic peptides binding MHC II compared to MHC I. MHC II molecules bind antigenic peptides with a size of 12-24 amino acids or even longer peptides. From a given antigenic protein, MHC II molecules typically can bind sets of overlapping peptides that shares a common core sequence but differs in the overall peptide size and in positioning of the core sequence in the peptide. The core peptide sequence is typically 9 amino acids long but may also be shorter or longer. Usefull antigenic peptide sequences binding MHC II of the present disclosure are described by the central part of the peptide mainly the 9-mer core peptide. The core peptide sequence may be flanked with a few or several important amino acids, generating antigenic peptides with a length of 13-16 amino acids. In some cases the peptide may contain even more flanking residues resulting in binding peptides longer than 13-16 amino acids. Thus, antigenic peptides of special interest of the present disclosure are peptides consisting of or containing 9-mer core peptide sequences The antigenic peptide sequences may be selected using the total approach as described for MHC I antigenic peptides elsewhere herein, e.g. using the software program shown in figure 2.

Alternatively a directed approach identifying a preferred subset of binding peptides from the binding peptides generated in the total approach can be used. As for MHC I one way to select subsets of antigenic peptides is to use consensus sequences to choose a set of relevant binding peptides able to bind the individual MHC allele and that will suit the "average" individual. Such consensus sequences often solely consider the affinity of the binding peptide for the MHC protein; in other words, a subset of binding peptides is identified where the designed binding peptides have a high probability of forming stable MHC-peptide complexes, but where it is uncertain whether this MHC-peptide complex is of high relevance in a population, and more uncertain whether this MHC-peptide complex is of high relevance in a given individual.

For class II MHC-alleles, the consensus sequence for the interacting **core** of a binding peptide is generally given by the formula

$$X1-X2-X3-X4-....-Xn,$$

where n equals 9, and where X represents one of the twenty naturally occurring amino acids, optionally modified as described elsewhere in this application.

X1-Xn can be further defined. Thus, certain positions in the consensus sequence are the socalled anchor positions and the selection of useful amino acids for these positions is limited to those able to fit into the corresponding binding pockets in the HLA molecule. For example HLA-DRB1*1501 have X1, X4 and X7 as primary anchor positions where preferred amino acids at the three positions are as follows, X1: leucine, valine and isoleucine, X4: phenylalanine, tyrosine or isoleucine, X7: isoleucine, leucine, valine, methionine or phenylalanine.

Therefore in one embodiment of the present disclosure antigenic peptides can be identified and selected on their ability to bind a given HLA or other MHC molecule based on what amino acids they have at various anchor positions.

In general, MHC II binding peptides have much more varied anchor positions than MHC I binding peptides and the number of usefull amino acids at each anchor position is much higher. For some MHC II alleles no really consensus sequence has been identified. In general position 1, 4, 6 and 9 of the 9-mer core motif of MHC II antigenic peptides are important for anchoring of the antigenic peptide to the MHC II molecule.

Table 7 shows examples of primary anchor positions and corresponding usefull amino acids for antigenic peptides binding various MHC II molecules.

**Table 7. Examples of primary anchor positions and corresponding usefull amino acids shown as one letter code.**

| MHC II | 1 | 2 | 3 | 4 | 5 | 6 | 7 | 8 | 9 |
|---|---|---|---|---|---|---|---|---|---|
| DQ2 | F, W, Y, I, L, V | | | D, E, L, V, I, H | | P, D, E, H, P, A | D, E | | F, W, Y, I, L, V, M |
| DQA1*0101/DQB1*0501 | L | | | | Y, F, W | | | | |
| DQA1*0102/DQB1*0602 | | | | | | L, I, V | | | A, G, S, T |

(continued)

| MHC II | 1 | 2 | 3 | 4 | 5 | 6 | 7 | 8 | 9 |
|---|---|---|---|---|---|---|---|---|---|
| DR17 (DRB1*0301) | L, I, F, M, V | | | D | | K, R, E, Q, N | | | Y, L, F |
| DR4 (DRB1*0401) | F, Y, W, I, L, V, M | | | P, W, I, L, V, A, D, E | | N, S, T, Q, H, R | D, E, H, K, N, Q, R, S, T, Y, A, C, I, L, M, V | | E, H, K, N, Q, R, S, T, Y, A, C, I, L, M, V |
| DRB1*1101 | W, Y, F | | | L, V, M, A, F, Y | | R, K, H | | | A, G, S, P |
| DRB1*1301 | I, V, F, L | | | Y, W, L, V, A, M | | R, K | | | Y, F, A, S, T |
| DRB1*1302 | Y, F, V, A, I | | | Y, W, L, V, A, M | | R, K | | | Y, F, A, S, T |

Another usefull parameter for prediction and selection of usefull antigenic peptides are the probability of the binding peptide in question to be generated *in vivo* or processed by the proteolytic machinery inside cells. However, like for MHC I, the proteolytic activitiy varies a lot among individuals, and for personalized diagnostics, treatment or vaccination it may be desirable to disregard these general proteolytic data.

Using the above described principles individual peptides or one or more subsets of peptides able to bind one or more types of MHC molecules and make stable MHC-peptide complexes can be identified. The identified peptides can then be tested for biological relevance in functional assays such as interferon gamma release assays, ELISPOT, CTL killing assays or using other methods as described in the section "Detection" elsewhere herein. Alternatively or complementary hereto the abillity of the identified antigenic peptides to bind selected MHC molecules may be determined in binding assays like Biacore measurement, compettion assays or other assays usefull for measurement of binding of peptide to MHC molecules, known by persons skilled in the art.

Peptide modifications

In addition to the binding peptides designed by the total approach and/or directed approach, homologous peptides and peptides that have been modified in the amino acid side chains or in the backbone can be used as binding peptides.

Homologous peptides

Homologues MHC peptide sequences may arise from the existence of multiple strongly homologous alleles, from small insertions, deletions, inversions or substitutions. If they are sufficiently homologous to peptides derived by the total approach, i.e. have an amino acid sequence identity greater than e.g. more than 90%, more than 80%, or more than 70%, or more than 60%, to one or two binding peptides derived by the total approach, they may be good candidates. Identity is often most important for the anchor residues.

A MHC binding peptide may be of split- or combinatorial epitope origin i.e. formed by linkage of peptide fragments derived from two different peptide fragments and/or proteins. Such peptides can be the result of either genetic recombination on the DNA level or due to peptide fragment association during the complex break down of proteins during protein turnover. Possibly it could also be the result of faulty reactions during protein synthesis i.e. caused by some kind of mixed RNA handling. A kind of combinatorial peptide epitope can also be seen if a portion of a longer peptide make a loop out leaving only the terminal parts of the peptide bound in the groove.

Uncommon, artificial and chemically modified amino acids.

Peptides having un-common amino acids, such as selenocysteine and pyrrolysine, may be bound in the MHC groove as well. Artificial amino acids e.g. having the isomeric D-form may also make up isomeric D-peptides that can bind in the binding groove of the MHC molecules. Bound peptides may also contain amino acids that are chemically modified or being linked to reactive groups that can be activated to induce changes in or disrupt the peptide. Example post-translational modifications are shown below. However, chemical modifications of amino acid side chains or the peptide backbone can also be performed.

Any of the modifications can be found individually or in combination at any position of the peptide, e.g. position 1, 2, 3, 4, 5, 6, etc. up to n.

Table 1 Post translational modification of peptides

| Protein primary structure and posttranslational modifications | |
|---|---|
| | |
| **N-terminus** | Acetylation, Formylation, Pyroglutamate, Methylation, Glycation, Myristoylation (Gly), carbamylation |
| **C-terminus** | Amidation, Glycosyl phosphatidylinositol (GPI), O-methylation, Glypiation, Ubiquitination, Sumoylation |
| **Lysine** | Methylation, Acetylation, Acylation, Hydroxylation, Ubiquitination, SUMOylation, Desmosine formation, ADP-ribosylation, Deamination and Oxidation to aldehyde |
| **Cysteine** | Disulfide bond, Prenylation, Palmitoylation |
| **Serine/Threonine** | Phosphorylation, Glycosylation |
| **Tyrosine** | Phosphorylation, Sulfation, Porphyrin ring linkage, Flavin linkage GFP prosthetic group (Thr-Tyr-Gly sequence) formation, Lysine tyrosine quinone (LTQ) formation, Topaquinone (TPQ) formation |
| **Asparagine** | Deamidation, Glycosylation |
| **Aspartate** | Succinimide formation |
| **Glutamine** | Transglutamination |
| **Glutamate** | Carboxylation, Methylation, Polyglutamylation, Polyglycylation |
| **Arginine** | Citrullination, Methylation |
| **Proline** | Hydroxylation |

Post translationally modified peptides

The amino acids of the antigenic peptides can also be modified in various ways dependent on the amino acid in question, or the modification can affect the amino- or carboxy-terminal end of the peptide. See table 1. Such peptide modifications are occuring naturally as the result of post tranlational processing of the parental protein. A non-exhaustive description of the major post translational modifications is given below, divided into three main types.

**a) Modification that adds a chemical moiety to the binding peptide.**
acetylation, the addition of an acetyl group, usually at the N-terminus of the protein
alkylation, the addition of an alkyl group (e.g. methyl, ethyl). Methylation, the addition of a methyl group, usually at lysine or arginine residues is a type of alkylation. Demethylation involves the removal of a methyl-group.
amidation at C-terminus
biotinylation, acylation of conserved lysine residues with a biotin appendage
formylation
gamma-carboxylation dependent on Vitamin K
glutamylation, covalent linkage of glutamic acid residues to tubulin and some other proteins by means of tubulin polyglutamylase
glycosylation, the addition of a glycosyl group to either asparagine, hydroxylysine, serine, or threonine, resulting in a glycoprotein. Distinct from glycation, which is regarded as a nonenzymatic attachment of sugars.
glycylation, covalent linkage of one to more than 40 glycine residues to the tubulin C-terminal tail
heme moiety may be covalently attached
hydroxylation, is any chemical process that introduces one or more hydroxyl groups (-OH) into a compound (or radical) thereby oxidizing it. The principal residue to be hydroxylated is Proline. The hydroxilation occurs at the $C^\gamma$ atom, forming hydroxyproline (Hyp). In some cases, proline may be hydroxylated instead on its $C^\beta$ atom. Lysine may also be hydroxylated on its $C^\delta$ atom, forming hydroxylysine (Hyl).
iodination

isoprenylation, the addition of an isoprenoid group (e.g. farnesol and geranylgeraniol)

lipoylation, attachment of a lipoate functionality, as in prenylation, GPI anchor formation, myristoylation, farnesylation, geranylation

nucleotides or derivatives thereof may be covalently attached, as in ADP-ribosylation and flavin attachment

oxidation, lysine can be oxidized to aldehyde

pegylation, addition of poly-ethylen-glycol groups to a protein. Typical reactive amino acids include lysine, cysteine, histidine, arginine, aspartic acid, glutamic acid, serine, threonine, tyrosine. The N-terminal amino group and the C-terminal carboxylic acid can also be used

phosphatidylinositol may be covalently attached

phosphopantetheinylation, the addition of a 4'-phosphopantetheinyl moiety from coenzyme A, as in fatty acid, polyketide, non-ribosomal peptide and leucine biosynthesis

phosphorylation, the addition of a phosphate group, usually to serine, tyrosine, threonine or histidine

pyroglutamate formation as a result of N-terminal glutamine self-attack, resulting in formation of a cyclic pyroglutamate group.

racemization of proline by prolyl isomerase

tRNA-mediated addition of amino acids such as arginylation

sulfation, the addition of a sulfate group to a tyrosine.

Selenoylation (co-translational incorporation of selenium in selenoproteins)

**b) Modification that adds protein or peptide.**

ISGylation, the covalent linkage to the ISG15 protein (Interferon-Stimulated Gene 15)

SUMOylation, the covalent linkage to the SUMO protein (Small Ubiquitin-related MOdifier)

ubiquitination, the covalent linkage to the protein ubiquitin.

**c) Modification that converts one or more amino acids to different amino acids.**

citrullination, or deimination the conversion of arginine to citrulline

deamidation, the conversion of glutamine to glutamic acid or asparagine to aspartic acid

The peptide modifications can occur as modification of a single amino acid or more than one i.e. in combinations. Modifications can be present on any position within the peptide i.e. on position 1, 2, 3, 4, 5,etc. for the entire length of the peptide.

**Sources of binding peptides**

**a) From natural sources**

The binding peptides can be obtained from natural sources by enzymatic digestion or proteolysis of natural proteins or proteins derived by *in vitro* translation of mRNA. Binding peptides may also be eluted from the MHC binding groove.

**b) From recombinant sources**

**1) as monomeric or multimeric peptide**

Alternatively peptides can be produced recombinantly by transfected cells either as monomeric antigenic peptides or as multimeric (concatemeric) antigenic peptides. Optionally, the Multimeric antigenic peptides are cleaved to form monomeric antigenic peptides before binding to MHC protein.

**2) as part of a bigger recombinant protein**

Binding peptides may also constitute a part of a bigger recombinant protein e.g.consisting of,

**2a) For MHC class 1 binding peptides,**

Peptide-linker-β2m, β2m being full length or truncated;

Peptide-linker-MHC class 1 heavy chain, the heavy chain being full length or truncated. Most importantly the truncated class I heavy chain will consist of the extracellular part i.e the α1,□ α2, and α domains. The heavy chain fragment may also only contain the α1 and α2 domains, or α1 domain alone, or any fragment or full length β2m or heavy chain attached to a designer domain(s) or protein fragment(s).

**2b) For MHC class 2 binding peptides** the recombinant construction can consist of,

Peptide-linker-MHC class 2 α-chain, full length or truncated;

Peptide-linker-MHC class 2 β-chain, full length or truncated;

Peptide-linker-MHC class 2 α-chain-linker-MHC class 2 β-chain, both chains can be full length or truncated, truncation may involve, omission of α- and/or β-chain intermembrane domain, or omission of α- and/or β-chain intermembrane plus cytoplasmic domains. MHC class 2 part of the construction may consist of fused domains from NH2-terminal, MHC class 2 β1domain-MHC class 2 α1 domain-constant α3 of MHC class 1, or alternatively of fused domains from NH2-terminal, MHC class 2 α1domain-MHC class 2 β1 domain-constant α3 of MHC class 1. In both cases β2m will be associated non-covalently in the folded MHC complex. β2m can also be covalently associated in the folded MHC class 2 complex if the following constructs are used from NH2 terminal, MHC class 2 β1domain-MHC class 2 α1domain-constant α3 of MHC class 1-linker-β2m, or alternatively of fused domains from NH2-terminal, MHC class 2 α1domain-MHC class 2 β1 domain-constant α3 of MHC class 1-linker-β2m; the construct may also consist of any of the above MHC class 2 constructs with added designer domain(s) or sequence(s).

## c) From chemical synthesis

MHC binding peptide may also be chemically synthesized by solid phase or fluid phase synthesis, according to standard protocols.

Comprehensive collections of antigenic peptides, derived from one antigen, may be prepared by a modification of the solid phase synthesis protocol, as described in the following and exemplified in Example 24.

The protocol for the synthesis of the full-length antigen on solid support is modified by adding a partial cleavage step after each coupling of an amino acid. Thus, the starting point for the synthesis is a solid support to which has been attached a cleavable linker. Then the first amino acid X1 (corresponding to the C-terminal end of the antigen) is added and a coupling reaction performed. The solid support now carries the molecule "linker-X1". After washing, a fraction (e.g. 10%) of the cleavable linkers are now cleaved, to release into solution X1. The supernatant is transferred to a collection container. Additional solid support carrying a cleavable linker is added, e.g. corresponding to 10% of the initial amount of solid support.

Then the second amino acid X2 is added and coupled to X1 or the cleavable linker, to form on solid support the molecules "linker-X2" and "linker-X1-X2". After washing, a fraction (e.g. 10%) of the cleavable linker is cleaved, to release into solution X2 and X1-X2. The supernatant is collected into the collection container, which therefore now contains X1, X2, and X1-X2. Additional solid support carrying a cleavable linker is added, e.g. corresponding to 10% of the initial amount of solid support.

Then the third amino acid X3 is added and coupled to X2 or the cleavable linker, to form on solid support the molecules "linker-X3", "linker-X2-X3" and "linker-X1-X2-X3". After washing, a fraction (e.g. 10%) of the cleavable linker is cleaved, to release into solution X3, X2-X3 and X1-X2-X3. The supernatant is collected into the collection container, which therefore now contains X1, X2, X3, X1-X2, X2-X3 and X1-X2-X3. Additional solid support carrying a cleavable linker is added, e.g. corresponding to 10% of the initial amount of solid support.

This step-wise coupling and partial cleavage of the linker is continued until the N-terminal end of the antigen is reached. The collection container will now contain a large number of peptides of different length and sequence. In the present example where a 10% partial cleavage was employed, a large fraction of the peptides will be 8'-mers, 9'-mers, 10'-mers and 11'-mers, corresponding to class I antigenic peptides. As an example, for a 100 amino acid antigen the 8'-mers will consist of the sequences X1-X2-X3-X4-X5-X6-X7-X8, X2-X3-X4-X5-X6-X7-X8-X9, ....., X93-X94-X95-X96-X97-X98-X99-X100.

Optionally, after a number of coupling and cleavage steps or after each coupling and cleavage step, the used (inactivated) linkers on solid support can be regenerated, in order to maintain a high fraction of linkers available for synthesis. The collection of antigenic peptides can be used as a pool for e.g. the display by APCs to stimulate CTLs in ELISPOT assays, or the antigenic peptides may be mixed with one or more MHC alleles, to form a large number of different MHC-peptide complexes which can e.g. be used to form a large number of different MHC multimers which can e.g. be used in flow cytometry experiments.

## Sequences for use in MHC monomers, MHC multimers, antigenic polypepitdes and antigenic peptides

MHC Class I and MHC Class II molecules have different structures, as described above, and therefore have different restrictions on the size of the peptide which may be accommodated. In general, MHC Class I molecules will accommodate peptides of from about 8 amino acids in length to about 11 amino acids. MHC Class II molecules will in general accommodate peptides of from about 13 amino acids in length to about 16 amino acids.

Of special interest of the present disclosure is antigenic peptide sequences derived from proteins of Borrelia bacteria. In the following Borrelia bacteria and their genes and proteins are described in more detail.

Borrelia bacteria

The various species of Borrelia are known to humans in the form of Lyme disease and recurring fever, transmitted through tick or flea bite. The cycle of Borrelia through animals is related to the tick's life cycle. The tick has four stages in its two-year life cycle, egg, larva, nymph and adult. Between each stage the tick needs a blood meal in order to mature. The tick usually acquires the spirochaete during its larval stage, when it feeds on small animals such as rodents or birds. Usually the tick picks up Borrelia from the white-footed mouse, which is commonly infected. The tick then becomes the host for the spirochaete. The bacteria resides in the digestive tract of the host for its next nymph and adult stages during which it is passed on to other animals, and sometimes humans.

*Borrelia species*

Borrelia is a genus of bacteria of the spirochete class. It is a zoonotic, vector-borne disease transmitted primarily by ticks and some by lice, depending on the species. There are at least 37 known species of Borrelia. Different species of Borrelia results in different clinical symptoms. Of the 37 known species of Borrelia, 12 of these species are known to cause Lyme disease or borreliosis and are transmitted by ticks. The major Borrelia species causing Lyme disease are Borrelia burgdorferi, Borrelia afzelii, Borrelia garinii and Borrelia valaisiana.

Other Borrelia species cause relapsing fever such as Borrelia recurrentis, caused by the human body louse. No animal reservoir of B. recurrentis exists. Lice that feed on infected humans acquire the Borrelia organisms that then multiply in the gut of the louse. When an infected louse feeds on an uninfected human, the organism gains access when the victim crushes the louse or scratches the area where the louse is feeding. B. recurrentis infects the person via mucous membranes and then invades the bloodstream.

Other tick-borne relapsing infections are acquired from other species, such as Borrelia hermsii or Borrelia Parkeri, which can be spread from rodents, and serve as a reservoir for the infection, via a tick vector. Borrelia hermsii and Borrelia recurrentis cause very similar diseases although the disease associated with Borrelia hermsii has more relapses and is responsible for more fatalities, while the disease caused by B. recurrentis has longer febrile and afebrile intervals and a longer incubation period. The present disclosure relates in one embodiment to one or more antigenic peptides or one or more antigenic polypeptides or to MHC-peptide complexes or MHC multimers comprising one or more antigenic peptides comprising one or more sequences from one or more Borrelia species including the ones mentioned above.

*Borrelia burgdorferi*

In one preferered embodiment the disclosure relates to one or more antigenic peptides or antigenic polypeptides comprising one or more sequences from Borrelia burgdorferi such as Borrelia burgdorferi B31orto one or more MHC-peptide complexes or MHC multiemrs comprising one or more such antigenic peptides.
Borrelia burgdorferi is species of bacteria of the spirochete class of the genus Borrelia. B. burgdorferi is predominant in North America, but also exists in Europe, and is the agent of Lyme disease.

It is a zoonotic, vector-borne disease transmitted by ticks and is named after the researcher Willy Burgdorfer who first isolated the bacterium in 1982. B. burgdorferi is one of the few pathogenic bacteria that can survive without iron, having replaced all of its iron-sulphur cluster enzymes with enzymes that use manganese, thus avoiding the problem many pathogenic bacteria face in acquiring iron. B. burgdorferi infections have been linked to non-Hodgkin lymphomas. The B. burgdorferi genome (B31 strain) contains 910,725 base pairs and 853 genes.

*Borrelia afzelii*

In one preferered embodiment the disclosure relates to one or more antigenic peptides or one or more antigenic polypeptides comprising one or more sequences from Borrelia afzelii such as borrelia afzelii Pko or one or more MHC-peptide complexes or one or more MHC multimers comprising one or more such antigenic peptides .

*Borrelia afzelii* is considered a new species of the Genus *Borrelia* and considered homologous to *Borrelia burgdorferi* with regard to phenotypic, genetic, and immunological characteristics. Diseases linked to this species of *Borrelia* are Lyme disease and Acrodermatitis chronica atrophicans (ACA). Better understanding of the structure and function of this pathogen will create better methods of treatment to people with the diseases it causes.

*Borrelia garini*

In one preferered embodiment the disclosure relates to one or more antigenic peptides or one or more antigenic polypeptides comprising one or more sequences from Borrelia garinii such as garinii PBi or to one or more MHC-peptide complexes or MHC multimers comprising one or more such antigenic peptides.

*Borrelia garinii* is one of two major strains found in Europe. It usually causes Lyme Disease symptoms of the neurological kind - such as extreme back- and leg-pains, meningitis and partial facial paralysis, Lyme arthritis due to *B garinii* may be associated in susceptible hosts with amoxicillin resistance or treatment resistance.

*Other Borrelia species*

In one preferred embodiment the present disclosure relates to or to one or more antigenic peptides or antigenic polypeptides, wherein the sequence of one or more antigenic peptides is from Borrelia anserine, or to one or more MHC-peptide complexes or MHC multimers, compricing such antigenic peptides.

In one preferred embodiment the present disclosure relates to one or more antigenic peptides or antigenic polypeptides, wherein the sequence of one or more antigenic peptides or antigenic polypeptides is from Borrelia barbouri, or to one or more MHC-peptide complexes or MHC multimers, compricing one or more such antigenic peptides.

In one preferred embodiment the present disclosure relates to one or more antigenic peptides or antigenic polypeptides, wherein the sequence of one or more antigenic peptides or antigenic polypeptides is from the Borrelia burgdorferi group such as burgdorferi B31, or to one or more MHC-peptide complexes or MHC multimers, compricing one or more such antigenic peptides.

In one preferred embodiment the present disclosure relates to one or more antigenic peptides or antigenic polypeptides, wherein the sequence of one or more antigenic peptides or antigenic polypeptides is from Borrelia afzelii such as Borrelia afzelii ACA-1, Borrelia afzelii K78 and/or Borrelia afzelii PKo, or to one or more MHC-peptide complexes or MHC multimers, compricing one or more such antigenic peptides.

In one preferred embodiment the present disclosure relates to one or more antigenic peptides or antigenic polypeptides, wherein the sequence of one or more antigenic peptides or antigenic polypeptides is from afzelii PKo, or to one or more MHC-peptide complexes or MHC multimers, compricing one or more such antigenic peptides.

In one preferred embodiment the present disclosure relates to one or more antigenic peptides or antigenic polypeptides, wherein the sequence of one or more antigenic peptides or antigenic polypeptides is from Borrelia andersonii,or to one or more MHC-peptide complexes or MHC multimers, compricing one or more such antigenic peptides.

In one preferred embodiment the present disclosure relates to one or more antigenic peptides or antigenic polypeptides, wherein the sequence of one or more antigenic peptides or antigenic polypeptides is from Borrelia bissettii,or to one or more MHC-peptide complexes or MHC multimers, compricing one or more such antigenic peptides.

In one preferred embodiment the present disclosure relates to one or more Antigenic peptides or antigenic polypeptides, wherein the sequence of one or more antigenic peptides or antigenic polypeptides is from Borrelia burgdorferi (Lyme disease spirochete),or to one or more MHC-peptide complexes or MHC multimers, compricing one or more such antigenic peptides.

In one preferred embodiment the present disclosure relates to one or more Antigenic peptides or antigenic polypeptides, wherein the sequence of one or more antigenic peptides or antigenic polypeptides is from Borrelia burgdorferi 118a,or to one or more MHC-peptide complexes or MHC multimers, compricing one or more such antigenic peptides.

In one preferred embodiment the present disclosure relates to one or more antigenic peptides or antigenic polypeptides, wherein the sequence of one or more antigenic peptides or antigenic polypeptides is from Borrelia burgdorferi 156a,or to one or more MHC-peptide complexes or MHC multimers, compricing one or more such antigenic peptides.

In one preferred embodiment the present disclosure relates to one or more Antigenic peptides or antigenic polypeptides, wherein the sequence of one or more antigenic peptides or antigenic polypeptides is from Borrelia burgdorferi 29805,or to one or more MHC-peptide complexes or MHC multimers, compricing one or more such antigenic peptides.

In one preferred embodiment the present disclosure relates to one or more Antigenic peptides or antigenic polypeptides, wherein the sequence of one or more antigenic peptides or antigenic polypeptides is from Borrelia burgdorferi 64b,or to one or more MHC-peptide complexes or MHC multimers, compricing one or more such antigenic peptides.

In one preferred embodiment the present disclosure relates to one or more Antigenic peptides or antigenic polypeptides, wherein the sequence of one or more antigenic peptides or antigenic polypeptides is from Borrelia burgdorferi 72a,or to one or more MHC-peptide complexes or MHC multimers, compricing one or more such antigenic peptides.

In one preferred embodiment the present disclosure relates to one or more Antigenic peptides or antigenic polypeptides, wherein the sequence of one or more antigenic peptides or antigenic polypeptides is from Borrelia burgdorferi 80a,or to one or more MHC-peptide complexes or MHC multimers, compricing one or more such antigenic peptides.

In one preferred embodiment the present disclosure relates to one or more Antigenic peptides or antigenic polypeptides, wherein the sequence of one or more antigenic peptides or antigenic polypeptides is from Borrelia burgdorferi 94a,or to one or more MHC-peptide complexes or MHC multimers, compricing one or more such antigenic peptides.

In one preferred embodiment the present disclosure relates to one or more antigenic peptides or antigenic polypeptides, wherein the sequence of one or more antigenic peptides or antigenic polypeptides is from Borrelia burgdorferi B31,or to one or more MHC-peptide complexes or MHC multimers, compricing one or more such antigenic peptides.

In one preferred embodiment the present disclosure relates to one or more antigenic peptides or antigenic polypeptides, wherein the sequence of one or more antigenic peptides or antigenic polypeptides is from Borrelia burgdorferi Bol26,or to one or more MHC-peptide complexes or MHC multimers, compricing one or more such antigenic peptides.

In one preferred embodiment the present disclosure relates to one or more antigenic peptides or antigenic polypeptides, wherein the sequence of one or more antigenic peptides or antigenic polypeptides is from Borrelia burgdorferi CA-11.2a, or to one or more MHC-peptide complexes or MHC multimers, compricing one or more such antigenic peptides.

In one preferred embodiment the present disclosure relates to one or more antigenic peptides or antigenic polypeptides, wherein the sequence of one or more antigenic peptides or antigenic polypeptides is from Borrelia burgdorferi WI91-23, or to one or more MHC-peptide complexes or MHC multimers, compricing one or more such antigenic peptides.

In one preferred embodiment the present disclosure relates to one or more antigenic peptides or antigenic polypeptides, wherein the sequence of one or more antigenic peptides or antigenic polypeptides is from Borrelia burgdorferi ZS7, or to one or more MHC-peptide complexes or MHC multimers, compricing one or more such antigenic peptides.

In one preferred embodiment the present disclosure relates to one or more antigenic peptides or antigenic polypeptides, wherein the sequence of one or more antigenic peptides or antigenic polypeptides is from Borrelia californiensis, or to one or more MHC-peptide complexes or MHC multimers, compricing one or more such antigenic peptides.

In one preferred embodiment the present disclosure relates to one or more antigenic peptides or antigenic polypeptides, wherein the sequence of one or more antigenic peptides or antigenic polypeptides is from Borrelia garinii such as garinii PBi, or to one or more MHC-peptide complexes or MHC multimers, compricing one or more such antigenic peptides.

In one preferred embodiment the present disclosure relates to one or more antigenic peptides or antigenic polypeptides, wherein the sequence of one or more antigenic peptides or antigenic polypeptides is from Borrelia garinii PBr, or to one or more MHC-peptide complexes or MHC multimers, compricing one or more such antigenic peptides.

In one preferred embodiment the present disclosure relates to one or more antigenic peptides or antigenic polypeptides, wherein the sequence of one or more antigenic peptides or antigenic polypeptides is from Borrelia genomosp. 1, or to one or more MHC-peptide complexes or MHC multimers, compricing one or more such antigenic peptides.

In one preferred embodiment the present disclosure relates to one or more antigenic peptides or antigenic polypeptides, wherein the sequence of one or more antigenic peptides or antigenic polypeptides is from Borrelia genomosp. 2,or to one or more MHC-peptide complexes or MHC multimers, compricing one or more such antigenic peptides.

In one preferred embodiment the present disclosure relates to one or more antigenic peptides or antigenic polypeptides, wherein the sequence of one or more antigenic peptides or antigenic polypeptides is from Borrelia japonica,or to one or more MHC-peptide complexes or MHC multimers, compricing one or more such antigenic peptides.

In one preferred embodiment the present disclosure relates to one or more antigenic peptides or antigenic polypeptides, wherein the sequence of one or more antigenic peptides or antigenic polypeptides is from Borrelia lusitaniae,or to one or more MHC-peptide complexes or MHC multimers, compricing one or more such antigenic peptides.

In one preferred embodiment the present disclosure relates to one or more antigenic peptides or antigenic polypeptides, wherein the sequence of one or more antigenic peptides or antigenic polypeptides is from Borrelia spielmanii,or to one or more MHC-peptide complexes or MHC multimers, compricing one or more such antigenic peptides.

In one preferred embodiment the present disclosure relates to one or more antigenic peptides or antigenic polypeptides, wherein the sequence of one or more antigenic peptides or antigenic polypeptides is from Borrelia spielmanii A14S,or to one or more MHC-peptide complexes or MHC multimers, compricing one or more such antigenic peptides.

In one preferred embodiment the present disclosure relates to one or more antigenic peptides or antigenic polypeptides, wherein the sequence of one or more antigenic peptides or antigenic polypeptides is from Borrelia tanukii,or to one or more MHC-peptide complexes or MHC multimers, compricing one or more such antigenic peptides.

In one preferred embodiment the present disclosure relates to one or more antigenic peptides or antigenic polypeptides, wherein the sequence of one or more antigenic peptides or antigenic polypeptides is from Borrelia turdi,or to one or more MHC-peptide complexes or MHC multimers, compricing one or more such antigenic peptides.

In one preferred embodiment the present disclosure relates to one or more antigenic peptides or antigenic polypeptides, wherein the sequence of one or more antigenic peptides or antigenic polypeptides is from Borrelia valaisiana,or to one or more MHC-peptide complexes or MHC multimers, compricing one or more such antigenic peptides.

In one preferred embodiment the present disclosure relates to one or more antigenic peptides or antigenic polypeptides, wherein the sequence of one or more antigenic peptides or antigenic polypeptides is from Borrelia valaisiana VS116,or to one or more MHC-peptide complexes or MHC multimers, compricing one or more such antigenic peptides.

In one preferred embodiment the present disclosure relates to one or more antigenic peptides or antigenic polypeptides, wherein the sequence of one or more antigenic peptides or antigenic polypeptides is from Candidatus Borrelia texasensis,or to one or more MHC-peptide complexes or MHC multimers, compricing one or more such antigenic peptides.

In one preferred embodiment the present disclosure relates to one or more antigenic peptides or antigenic polypeptides, wherein the sequence of one or more antigenic peptides or antigenic polypeptides is from Borrelia sp. AA4Pool,or to one or more MHC-peptide complexes or MHC multimers, compricing one or more such antigenic peptides.

In one preferred embodiment the present disclosure relates to one or more antigenic peptides or antigenic polypeptides, wherein the sequence of one or more antigenic peptides or antigenic polypeptides is from Borrelia sp. AI-1,or to one or more MHC-peptide complexes or MHC multimers, compricing one or more such antigenic peptides.

In one preferred embodiment the present disclosure relates to one or more antigenic peptides or antigenic polypeptides, wherein the sequence of one or more antigenic peptides or antigenic polypeptides is from Borrelia sp. B31,or to one or more MHC-peptide complexes or MHC multimers, compricing one or more such antigenic peptides.

In one preferred embodiment the present disclosure relates to one or more antigenic peptides or antigenic polypeptides, wherein the sequence of one or more antigenic peptides or antigenic polypeptides is from Borrelia sp. BC-1,or to one or more MHC-peptide complexes or MHC multimers, compricing one or more such antigenic peptides.

In one preferred embodiment the present disclosure relates to one or more antigenic peptides or antigenic polypeptides, wherein the sequence of one or more antigenic peptides or antigenic polypeptides is from Borrelia sp. CA1133,or to one or more MHC-peptide complexes or MHC multimers, compricing one or more such antigenic peptides.

In one preferred embodiment the present disclosure relates to one or more antigenic peptides or antigenic polypeptides, wherein the sequence of one or more antigenic peptides or antigenic polypeptides is from Borrelia sp. CA1176,or to one or more MHC-peptide complexes or MHC multimers, compricing one or more such antigenic peptides.

In one preferred embodiment the present disclosure relates to one or more antigenic peptides or antigenic polypeptides, wherein the sequence of one or more antigenic peptides or antigenic polypeptides is from Borrelia sp. CA128,or to one or more MHC-peptide complexes or MHC multimers, compricing one or more such antigenic peptides.

In one preferred embodiment the present disclosure relates to one or more antigenic peptides or antigenic polypeptides, wherein the sequence of one or more antigenic peptides or antigenic polypeptides is from Borrelia sp. CA13,or to one or more MHC-peptide complexes or MHC multimers, compricing one or more such antigenic peptides.

In one preferred embodiment the present disclosure relates to one or more antigenic peptides or antigenic polypeptides, wherein the sequence of one or more antigenic peptides or antigenic polypeptides is from Borrelia sp. CA134,or to one or more MHC-peptide complexes or MHC multimers, compricing one or more such antigenic peptides.

In one preferred embodiment the present disclosure relates to one or more antigenic peptides or antigenic polypeptides, wherein the sequence of one or more antigenic peptides or antigenic polypeptides is from Borrelia sp. CA142,or to one or more MHC-peptide complexes or MHC multimers, compricing one or more such antigenic peptides.

In one preferred embodiment the present disclosure relates to one or more antigenic peptides or antigenic polypeptides, wherein the sequence of one or more antigenic peptides or antigenic polypeptides is from Borrelia sp. CA20,or to one or more MHC-peptide complexes or MHC multimers, compricing one or more such antigenic peptides.

In one preferred embodiment the present disclosure relates to one or more antigenic peptides or antigenic polypeptides, wherein the sequence of one or more antigenic peptides or antigenic polypeptides is from Borrelia sp. CA22,or to one or more MHC-peptide complexes or MHC multimers, compricing one or more such antigenic peptides.

In one preferred embodiment the present disclosure relates to one or more antigenic peptides or antigenic polypeptides, wherein the sequence of one or more antigenic peptides or antigenic polypeptides is from Borrelia sp. CA27,or to one or more MHC-peptide complexes or MHC multimers, compricing one or more such antigenic peptides.

In one preferred embodiment the present disclosure relates to one or more antigenic peptides or antigenic polypeptides, wherein the sequence of one or more antigenic peptides or antigenic polypeptides is from Borrelia sp. CA28,or to one or more MHC-peptide complexes or MHC multimers, compricing one or more such antigenic peptides.

In one preferred embodiment the present disclosure relates to one or more antigenic peptides or antigenic polypeptides, wherein the sequence of one or more antigenic peptides or antigenic polypeptides is from Borrelia sp. CA29,or to one or more MHC-peptide complexes or MHC multimers, compricing one or more such antigenic peptides.

In one preferred embodiment the present disclosure relates to one or more antigenic peptides or antigenic polypeptides, wherein the sequence of one or more antigenic peptides or antigenic polypeptides is from Borrelia sp. CA31, or to one or more MHC-peptide complexes or MHC multimers, compricing one or more such antigenic peptides.

In one preferred embodiment the present disclosure relates to one or more antigenic peptides or antigenic polypeptides, wherein the sequence of one or more antigenic peptides or antigenic polypeptides is from Borrelia sp. CA33,or to one or more MHC-peptide complexes or MHC multimers, compricing one or more such antigenic peptides.

In one preferred embodiment the present disclosure relates to one or more antigenic peptides or antigenic polypeptides, wherein the sequence of one or more antigenic peptides or antigenic polypeptides is from Borrelia sp. CA370,or to one or more MHC-peptide complexes or MHC multimers, compricing one or more such antigenic peptides.

In one preferred embodiment the present disclosure relates to one or more antigenic peptides or antigenic polypeptides, wherein the sequence of one or more antigenic peptides or antigenic polypeptides is from Borrelia sp. CA372,or to one or more MHC-peptide complexes or MHC multimers, compricing one or more such antigenic peptides.

In one preferred embodiment the present disclosure relates to one or more antigenic peptides or antigenic polypeptides, wherein the sequence of one or more antigenic peptides or antigenic polypeptides is from Borrelia sp. CA378,or to one or more MHC-peptide complexes or MHC multimers, compricing one or more such antigenic peptides.

In one preferred embodiment the present disclosure relates to one or more antigenic peptides or antigenic polypeptides, wherein the sequence of one or more antigenic peptides or antigenic polypeptides is from Borrelia sp. CA388,or to one or more MHC-peptide complexes or MHC multimers, compricing one or more such antigenic peptides.

In one preferred embodiment the present disclosure relates to one or more antigenic peptides or antigenic polypeptides, wherein the sequence of one or more antigenic peptides or antigenic polypeptides is from Borrelia sp. CA393,or to one or more MHC-peptide complexes or MHC multimers, compricing one or more such antigenic peptides.

In one preferred embodiment the present disclosure relates to one or more antigenic peptides or antigenic polypeptides, wherein the sequence of one or more antigenic peptides or antigenic polypeptides is from Borrelia sp. CA394,or to one or more MHC-peptide complexes or MHC multimers, compricing one or more such antigenic peptides.

In one preferred embodiment the present disclosure relates to one or more antigenic peptides or antigenic polypeptides, wherein the sequence of one or more antigenic peptides or antigenic polypeptides is from Borrelia sp. CA395,or to one or more MHC-peptide complexes or MHC multimers, compricing one or more such antigenic peptides.

In one preferred embodiment the present disclosure relates to one or more antigenic peptides or antigenic polypeptides, wherein the sequence of one or more antigenic peptides or antigenic polypeptides is from Borrelia sp. CA399,or to one or more MHC-peptide complexes or MHC multimers, compricing one or more such antigenic peptides.

In one preferred embodiment the present disclosure relates to one or more antigenic peptides or antigenic polypeptides, wherein the sequence of one or more antigenic peptides or antigenic polypeptides is from Borrelia sp. CA400,or to one or more MHC-peptide complexes or MHC multimers, compricing one or more such antigenic peptides.

In one preferred embodiment the present disclosure relates to one or more antigenic peptides or antigenic polypeptides, wherein the sequence of one or more antigenic peptides or antigenic polypeptides is from Borrelia sp. CA401, or to one or more MHC-peptide complexes or MHC multimers, compricing one or more such antigenic peptides.

In one preferred embodiment the present disclosure relates to one or more antigenic peptides or antigenic polypeptides, wherein the sequence of one or more antigenic peptides or antigenic polypeptides is from Borrelia sp. CA402, or to one or more MHC-peptide complexes or MHC multimers, compricing one or more such antigenic peptides.

In one preferred embodiment the present disclosure relates to one or more antigenic peptides or antigenic polypeptides, wherein the sequence of one or more antigenic peptides or antigenic polypeptides is from Borrelia sp. CA404,or to one or more MHC-peptide complexes or MHC multimers, compricing one or more such antigenic peptides.

In one preferred embodiment the present disclosure relates to one or more antigenic peptides or antigenic polypeptides, wherein the sequence of one or more antigenic peptides or antigenic polypeptides is from Borrelia sp. CA411, or to one or more MHC-peptide complexes or MHC multimers, compricing one or more such antigenic peptides.

In one preferred embodiment the present disclosure relates to one or more antigenic peptides or antigenic polypeptides, wherein the sequence of one or more antigenic peptides or antigenic polypeptides is from Borrelia sp. CA426, or to one or more MHC-peptide complexes or MHC multimers, compricing one or more such antigenic peptides.

In one preferred embodiment the present disclosure relates to one or more antigenic peptides or antigenic polypeptides, wherein the sequence of one or more antigenic peptides or antigenic polypeptides is from Borrelia sp. CA443,or to one or more MHC-peptide complexes or MHC multimers, compricing one or more such antigenic peptides.

In one preferred embodiment the present disclosure relates to one or more antigenic peptides or antigenic polypeptides, wherein the sequence of one or more antigenic peptides or antigenic polypeptides is from Borrelia sp. CA446,or to one or more MHC-peptide complexes or MHC multimers, compricing one or more such antigenic peptides.

In one preferred embodiment the present disclosure relates to one or more antigenic peptides or antigenic polypeptides, wherein the sequence of one or more antigenic peptides or antigenic polypeptides is from Borrelia sp. CA448,or to one or more MHC-peptide complexes or MHC multimers, compricing one or more such antigenic peptides.

In one preferred embodiment the present disclosure relates to one or more antigenic peptides or antigenic polypeptides, wherein the sequence of one or more antigenic peptides or antigenic polypeptides is from Borrelia sp. CA462,or to one or more MHC-peptide complexes or MHC multimers, compricing one or more such antigenic peptides.

In one preferred embodiment the present disclosure relates to one or more antigenic peptides or antigenic polypeptides, wherein the sequence of one or more antigenic peptides or antigenic polypeptides is from Borrelia sp. CA468,or to one or more MHC-peptide complexes or MHC multimers, compricing one or more such antigenic peptides.

In one preferred embodiment the present disclosure relates to one or more antigenic peptides or antigenic polypeptides, wherein the sequence of one or more antigenic peptides or antigenic polypeptides is from Borrelia sp.

CA502,or to one or more MHC-peptide complexes or MHC multimers, compricing one or more such antigenic peptides.

In one preferred embodiment the present disclosure relates to one or more antigenic peptides or antigenic polypeptides, wherein the sequence of one or more antigenic peptides or antigenic polypeptides is from Borrelia sp. CA504,or to one or more MHC-peptide complexes or MHC multimers, compricing one or more such antigenic peptides.

In one preferred embodiment the present disclosure relates to one or more antigenic peptides or antigenic polypeptides, wherein the sequence of one or more antigenic peptides or antigenic polypeptides is from Borrelia sp. CA507,or to one or more MHC-peptide complexes or MHC multimers, compricing one or more such antigenic peptides.

In one preferred embodiment the present disclosure relates to one or more antigenic peptides or antigenic polypeptides, wherein the sequence of one or more antigenic peptides or antigenic polypeptides is from Borrelia sp. CA547,or to one or more MHC-peptide complexes or MHC multimers, compricing one or more such antigenic peptides.

In one preferred embodiment the present disclosure relates to one or more antigenic peptides or antigenic polypeptides, wherein the sequence of one or more antigenic peptides or antigenic polypeptides is from Borrelia sp. CA552,or to one or more MHC-peptide complexes or MHC multimers, compricing one or more such antigenic peptides.

In one preferred embodiment the present disclosure relates to one or more antigenic peptides or antigenic polypeptides, wherein the sequence of one or more antigenic peptides or antigenic polypeptides is from Borrelia sp. CA8,or to one or more MHC-peptide complexes or MHC multimers, compricing one or more such antigenic peptides.

In one preferred embodiment the present disclosure relates to one or more antigenic peptides or antigenic polypeptides, wherein the sequence of one or more antigenic peptides or antigenic polypeptides is from Borrelia sp. D22,or to one or more MHC-peptide complexes or MHC multimers, compricing one or more such antigenic peptides.

In one preferred embodiment the present disclosure relates to one or more antigenic peptides or antigenic polypeptides, wherein the sequence of one or more antigenic peptides or antigenic polypeptides is from Borrelia sp. D35,or to one or more MHC-peptide complexes or MHC multimers, compricing one or more such antigenic peptides.

In one preferred embodiment the present disclosure relates to one or more antigenic peptides or antigenic polypeptides, wherein the sequence of one or more antigenic peptides or antigenic polypeptides is from Borrelia sp. FD-1,or to one or more MHC-peptide complexes or MHC multimers, compricing one or more such antigenic peptides.

In one preferred embodiment the present disclosure relates to one or more antigenic peptides or antigenic polypeptides, wherein the sequence of one or more antigenic peptides or antigenic polypeptides is from Borrelia sp. FL18,or to one or more MHC-peptide complexes or MHC multimers, compricing one or more such antigenic peptides.

In one preferred embodiment the present disclosure relates to one or more antigenic peptides or antigenic polypeptides, wherein the sequence of one or more antigenic peptides or antigenic polypeptides is from Borrelia sp. FL27,or to one or more MHC-peptide complexes or MHC multimers, compricing one or more such antigenic peptides.

In one preferred embodiment the present disclosure relates to one or more antigenic peptides or antigenic polypeptides, wherein the sequence of one or more antigenic peptides or antigenic polypeptides is from Borrelia sp. FL35,or to one or more MHC-peptide complexes or MHC multimers, compricing one or more such antigenic peptides.

In one preferred embodiment the present disclosure relates to one or more antigenic peptides or antigenic polypeptides, wherein the sequence of one or more antigenic peptides or antigenic polypeptides is from Borrelia sp. FL42,or to one or more MHC-peptide complexes or MHC multimers, compricing one or more such antigenic peptides.

In one preferred embodiment the present disclosure relates to one or more antigenic peptides or antigenic polypeptides, wherein the sequence of one or more antigenic peptides or antigenic polypeptides is from Borrelia sp. HN6,or to one or more MHC-peptide complexes or MHC multimers, compricing one or more such antigenic peptides.

In one preferred embodiment the present disclosure relates to one or more antigenic peptides or antigenic polypeptides, wherein the sequence of one or more antigenic peptides or antigenic polypeptides is from Borrelia sp. HN7,or to one or more MHC-peptide complexes or MHC multimers, compricing one or more such antigenic peptides.

In one preferred embodiment the present disclosure relates to one or more antigenic peptides or antigenic polypeptides, wherein the sequence of one or more antigenic peptides or antigenic polypeptides is from Borrelia sp. HN8,or to one or more MHC-peptide complexes or MHC multimers, compricing one or more such antigenic peptides.

In one preferred embodiment the present disclosure relates to one or more antigenic peptides or antigenic polypeptides, wherein the sequence of one or more antigenic peptides or antigenic polypeptides is from Borrelia sp. HNM13,or to one or more MHC-peptide complexes or MHC multimers, compricing one or more such antigenic peptides.

In one preferred embodiment the present disclosure relates to one or more antigenic peptides or antigenic polypeptides, wherein the sequence of one or more antigenic peptides or antigenic polypeptides is from Borrelia sp. HNM14,or to one or more MHC-peptide complexes or MHC multimers, compricing one or more such antigenic peptides.

In one preferred embodiment the present disclosure relates to one or more antigenic peptides or antigenic polypeptides, wherein the sequence of one or more antigenic peptides or antigenic polypeptides is from Borrelia sp. HNM19,or to one or more MHC-peptide complexes or MHC multimers, compricing one or more such antigenic peptides.

In one preferred embodiment the present disclosure relates to one or more antigenic peptides or antigenic polypeptides, wherein the sequence of one or more antigenic peptides or antigenic polypeptides is from Borrelia sp. IA1,or to one or more MHC-peptide complexes or MHC multimers, compricing one or more such antigenic peptides.

In one preferred embodiment the present disclosure relates to one or more antigenic peptides or antigenic polypeptides, wherein the sequence of one or more antigenic peptides or antigenic polypeptides is from Borrelia sp. Ir-3519,or to one or more MHC-peptide complexes or MHC multimers, compricing one or more such antigenic peptides.

In one preferred embodiment the present disclosure relates to one or more antigenic peptides or antigenic polypeptides, wherein the sequence of one or more antigenic peptides or antigenic polypeptides is from Borrelia sp. Ir-4721, or to one or more MHC-peptide complexes or MHC multimers, compricing one or more such antigenic peptides.

In one preferred embodiment the present disclosure relates to one or more antigenic peptides or antigenic polypeptides, wherein the sequence of one or more antigenic peptides or antigenic polypeptides is from Borrelia sp. Ir-4812,or to one or more MHC-peptide complexes or MHC multimers, compricing one or more such antigenic peptides.

In one preferred embodiment the present disclosure relates to one or more antigenic peptides or antigenic polypeptides, wherein the sequence of one or more antigenic peptides or antigenic polypeptides is from Borrelia sp. Ir-5215,or to one or more MHC-peptide complexes or MHC multimers, compricing one or more such antigenic peptides.

In one preferred embodiment the present disclosure relates to one or more antigenic peptides or antigenic polypeptides, wherein the sequence of one or more antigenic peptides or antigenic polypeptides is from Borrelia sp. LV5,or to one or more MHC-peptide complexes or MHC multimers, compricing one or more such antigenic peptides.

In one preferred embodiment the present disclosure relates to one or more antigenic peptides or antigenic polypeptides, wherein the sequence of one or more antigenic peptides or antigenic polypeptides is from Borrelia sp. MI-2,or to one or more MHC-peptide complexes or MHC multimers, compricing one or more such antigenic peptides.

In one preferred embodiment the present disclosure relates to one or more antigenic peptides or antigenic polypeptides, wherein the sequence of one or more antigenic peptides or antigenic polypeptides is from Borrelia sp. MI-5,or to one or more MHC-peptide complexes or MHC multimers, compricing one or more such antigenic peptides.

In one preferred embodiment the present disclosure relates to one or more antigenic peptides or antigenic polypeptides, wherein the sequence of one or more antigenic peptides or antigenic polypeptides is from Borrelia sp. MI-6,or to one or more MHC-peptide complexes or MHC multimers, compricing one or more such antigenic peptides.

In one preferred embodiment the present disclosure relates to one or more antigenic peptides or antigenic polypeptides, wherein the sequence of one or more antigenic peptides or antigenic polypeptides is from Borrelia sp. MI-8,or to one or more MHC-peptide complexes or MHC multimers, compricing one or more such antigenic peptides.

In one preferred embodiment the present disclosure relates to one or more antigenic peptides or antigenic polypeptides, wherein the sequence of one or more antigenic peptides or antigenic polypeptides is from Borrelia sp. MI-9,or to one or more MHC-peptide complexes or MHC multimers, compricing one or more such antigenic peptides.

In one preferred embodiment the present disclosure relates to one or more antigenic peptides or antigenic polypeptides, wherein the sequence of one or more antigenic peptides or antigenic polypeptides is from Borrelia sp. MOD-1, or to one or more MHC-peptide complexes or MHC multimers, compricing one or more such antigenic peptides.

In one preferred embodiment the present disclosure relates to one or more antigenic peptides or antigenic polypeptides, wherein the sequence of one or more antigenic peptides or antigenic polypeptides is from Borrelia sp. MOD-5, or to one or more MHC-peptide complexes or MHC multimers, compricing one or more such antigenic peptides.

In one preferred embodiment the present disclosure relates to one or more antigenic peptides or antigenic polypeptides, wherein the sequence of one or more antigenic peptides or antigenic polypeptides is from Borrelia sp. MOK-3a,or to one or more MHC-peptide complexes or MHC multimers, compricing one or more such antigenic peptides.

In one preferred embodiment the present disclosure relates to one or more antigenic peptides or antigenic polypeptides, wherein the sequence of one or more antigenic peptides or antigenic polypeptides is from Borrelia sp. MOS-1b, or to one or more MHC-peptide complexes or MHC multimers, compricing one or more such antigenic peptides.

In one preferred embodiment the present disclosure relates to one or more antigenic peptides or antigenic polypeptides, wherein the sequence of one or more antigenic peptides or antigenic polypeptides is from Borrelia sp. NE49, or to one or more MHC-peptide complexes or MHC multimers, compricing one or more such antigenic peptides.

In one preferred embodiment the present disclosure relates to one or more antigenic peptides or antigenic polypeptides, wherein the sequence of one or more antigenic peptides or antigenic polypeptides is from Borrelia sp. NE581, or to one or more MHC-peptide complexes or MHC multimers, compricing one or more such antigenic peptides.

In one preferred embodiment the present disclosure relates to one or more antigenic peptides or antigenic polypeptides, wherein the sequence of one or more antigenic peptides or antigenic polypeptides is from Borrelia sp. PHaP,or to one or more MHC-peptide complexes or MHC multimers, compricing one or more such antigenic peptides.

In one preferred embodiment the present disclosure relates to one or more antigenic peptides or antigenic polypeptides, wherein the sequence of one or more antigenic peptides or antigenic polypeptides is from Borrelia sp. PSigII,or to one or more MHC-peptide complexes or MHC multimers, compricing one or more such antigenic peptides.

In one preferred embodiment the present disclosure relates to one or more antigenic peptides or antigenic polypeptides, wherein the sequence of one or more antigenic peptides or antigenic polypeptides is from Borrelia sp. SCGT-10,or to one or more MHC-peptide complexes or MHC multimers, compricing one or more such antigenic peptides.

In one preferred embodiment the present disclosure relates to one or more antigenic peptides or antigenic polypeptides, wherein the sequence of one or more antigenic peptides or antigenic polypeptides is from Borrelia sp. SCGT-8a,or to one or more MHC-peptide complexes or MHC multimers, compricing one or more such antigenic peptides.

In one preferred embodiment the present disclosure relates to one or more antigenic peptides or antigenic polypeptides, wherein the sequence of one or more antigenic peptides or antigenic polypeptides is from Borrelia sp. SCI-2,or to one or more MHC-peptide complexes or MHC multimers, compricing one or more such antigenic peptides.

In one preferred embodiment the present disclosure relates to one or more antigenic peptides or antigenic polypeptides, wherein the sequence of one or more antigenic peptides or antigenic polypeptides is from Borrelia sp. SCW-30h,or to one or more MHC-peptide complexes or MHC multimers, compricing one or more such antigenic peptides.

In one preferred embodiment the present disclosure relates to one or more antigenic peptides or antigenic polypeptides, wherein the sequence of one or more antigenic peptides or antigenic polypeptides is from Borrelia sp. SI-1,or to one or more MHC-peptide complexes or MHC multimers, compricing one or more such antigenic peptides.

In one preferred embodiment the present disclosure relates to one or more antigenic peptides or antigenic polypeptides, wherein the sequence of one or more antigenic peptides or antigenic polypeptides is from Borrelia sp. SI-10,or to one or more MHC-peptide complexes or MHC multimers, compricing one or more such antigenic peptides.

In one preferred embodiment the present disclosure relates to one or more antigenic peptides or antigenic polypeptides, wherein the sequence of one or more antigenic peptides or antigenic polypeptides is from Borrelia sp. SM-1,or to one or more MHC-peptide complexes or MHC multimers, compricing one or more such antigenic peptides.

In one preferred embodiment the present disclosure relates to one or more antigenic peptides or antigenic polypeptides, wherein the sequence of one or more antigenic peptides or antigenic polypeptides is from Borrelia sp. SV1,or to one or more MHC-peptide complexes or MHC multimers, compricing one or more such antigenic peptides.

In one preferred embodiment the present disclosure relates to one or more antigenic peptides or antigenic polypeptides, wherein the sequence of one or more antigenic peptides or antigenic polypeptides is from Borrelia sp. W97F51,or to one or more MHC-peptide complexes or MHC multimers, compricing one or more such antigenic peptides.

In one preferred embodiment the present disclosure relates to one or more antigenic peptides or antigenic polypeptides, wherein the sequence of one or more antigenic peptides or antigenic polypeptides is from Borrelia sp. Z41293,or to one or more MHC-peptide complexes or MHC multimers, compricing one or more such antigenic peptides.

In one preferred embodiment the present disclosure relates to one or more antigenic peptides or antigenic polypeptides, wherein the sequence of one or more antigenic peptides or antigenic polypeptides is from Borrelia sp. Z41493,or to one or more MHC-peptide complexes or MHC multimers, compricing one or more such antigenic peptides.

In one preferred embodiment the present disclosure relates to one or more antigenic peptides or antigenic polypeptides, wherein the sequence of one or more antigenic peptides or antigenic polypeptides is from Borrelia coriaceae,or to one or more MHC-peptide complexes or MHC multimers, compricing one or more such antigenic peptides.

In one preferred embodiment the present disclosure relates to one or more antigenic peptides or antigenic polypeptides, wherein the sequence of one or more antigenic peptides or antigenic polypeptides is from Borrelia crocidurae,or to one or more MHC-peptide complexes or MHC multimers, compricing one or more such antigenic peptides.

In one preferred embodiment the present disclosure relates to one or more antigenic peptides or antigenic polypeptides, wherein the sequence of one or more antigenic peptides or antigenic polypeptides is from Borrelia duttonii,or to one or more MHC-peptide complexes or MHC multimers, compricing one or more such antigenic peptides.

In one preferred embodiment the present disclosure relates to one or more antigenic peptides or antigenic polypeptides, wherein the sequence of one or more antigenic peptides or antigenic polypeptides is from Borrelia duttonii Ly,or to one or more MHC-peptide complexes or MHC multimers, compricing one or more such antigenic peptides.

In one preferred embodiment the present disclosure relates to one or more antigenic peptides or antigenic polypeptides, wherein the sequence of one or more antigenic peptides or antigenic polypeptides is from Borrelia hermsii,or to one or more MHC-peptide complexes or MHC multimers, compricing one or more such antigenic peptides.

In one preferred embodiment the present disclosure relates to one or more antigenic peptides or antigenic polypeptides, wherein the sequence of one or more antigenic peptides or antigenic polypeptides is from Borrelia hermsii DAH,or to one or more MHC-peptide complexes or MHC multimers, compricing one or more such antigenic peptides.

In one preferred embodiment the present disclosure relates to one or more antigenic peptides or antigenic polypeptides, wherein the sequence of one or more antigenic peptides or antigenic polypeptides is from Borrelia hispanica,or to one or more MHC-peptide complexes or MHC multimers, compricing one or more such antigenic peptides.

In one preferred embodiment the present disclosure relates to one or more antigenic peptides or antigenic polypeptides, wherein the sequence of one or more antigenic peptides or antigenic polypeptides is from Borrelia lonestari,or to one or more MHC-peptide complexes or MHC multimers, compricing one or more such antigenic peptides.

In one preferred embodiment the present disclosure relates to one or more antigenic peptides or antigenic polypeptides, wherein the sequence of one or more antigenic peptides or antigenic polypeptides is from Borrelia miyamotoi,or to one or more MHC-peptide complexes or MHC multimers, compricing one or more such antigenic peptides.

In one preferred embodiment the present disclosure relates to one or more antigenic peptides or antigenic polypeptides, wherein the sequence of one or more antigenic peptides or antigenic polypeptides is from Borrelia parkeri,or to one or more MHC-peptide complexes or MHC multimers, compricing one or more such antigenic peptides.

In one preferred embodiment the present disclosure relates to one or more antigenic peptides or antigenic polypeptides, wherein the sequence of one or more antigenic peptides or antigenic polypeptides is from Borrelia persica,or to one or more MHC-peptide complexes or MHC multimers, compricing one or more such antigenic peptides.

In one preferred embodiment the present disclosure relates to one or more antigenic peptides or antigenic polypeptides, wherein the sequence of one or more antigenic peptides or antigenic polypeptides is from Borrelia recurrentis,or to one or more MHC-peptide complexes or MHC multimers, compricing one or more such antigenic peptides.

In one preferred embodiment the present disclosure relates to one or more antigenic peptides or antigenic polypeptides, wherein the sequence of one or more antigenic peptides or antigenic polypeptides is from Borrelia recurrentis A1,or to one or more MHC-peptide complexes or MHC multimers, compricing one or more such antigenic peptides.

In one preferred embodiment the present disclosure relates to one or more antigenic peptides or antigenic polypeptides, wherein the sequence of one or more antigenic peptides or antigenic polypeptides is from Borrelia sinica,or to one or more MHC-peptide complexes or MHC multimers, compricing one or more such antigenic peptides.

In one preferred embodiment the present disclosure relates to one or more antigenic peptides or antigenic polypeptides, wherein the sequence of one or more antigenic peptides or antigenic polypeptides is from Borrelia theileri,or to one or more MHC-peptide complexes or MHC multimers, compricing one or more such antigenic peptides.

In one preferred embodiment the present disclosure relates to one or more antigenic peptides or antigenic polypeptides, wherein the sequence of one or more antigenic peptides or antigenic polypeptides is from Borrelia turcica,or to one or more MHC-peptide complexes or MHC multimers, compricing one or more such antigenic peptides.

In one preferred embodiment the present disclosure relates to one or more antigenic peptides or antigenic polypeptides, wherein the sequence of one or more antigenic peptides or antigenic polypeptides is from Borrelia turicatae,or to one or more MHC-peptide complexes or MHC multimers, compricing one or more such antigenic peptides.

In one preferred embodiment the present disclosure relates to one or more antigenic peptides or antigenic polypeptides, wherein the sequence of one or more antigenic peptides or antigenic polypeptides is from Borrelia turicatae 91E135,or to one or more MHC-peptide complexes or MHC multimers, compricing one or more such antigenic peptides.

In one preferred embodiment the present disclosure relates to one or more antigenic peptides or antigenic polypeptides, wherein the sequence of one or more antigenic peptides or antigenic polypeptides is from Borrelia sp. 'Lake Gaillard',or to one or more MHC-peptide complexes or MHC multimers, compricing one or more such antigenic peptides.

In one preferred embodiment the present disclosure relates to one or more antigenic peptides or antigenic polypeptides, wherein the sequence of one or more antigenic peptides or antigenic polypeptides is from Borrelia sp. 000133,or to one or more MHC-peptide complexes or MHC multimers, compricing one or more such antigenic peptides.

In one preferred embodiment the present disclosure relates to one or more antigenic peptides or antigenic polypeptides, wherein the sequence of one or more antigenic peptides or antigenic polypeptides is from Borrelia sp. 010298,or to one or more MHC-peptide complexes or MHC multimers, compricing one or more such antigenic peptides.

In one preferred embodiment the present disclosure relates to one or more antigenic peptides or antigenic polypeptides, wherein the sequence of one or more antigenic peptides or antigenic polypeptides is from Borrelia sp. 10MT,or to one or more MHC-peptide complexes or MHC multimers, compricing one or more such antigenic peptides.

In one preferred embodiment the present disclosure relates to one or more antigenic peptides or antigenic polypeptides, wherein the sequence of one or more antigenic peptides or antigenic polypeptides is from Borrelia sp. 5145,or to one or more MHC-peptide complexes or MHC multimers, compricing one or more such antigenic peptides.

In one preferred embodiment the present disclosure relates to one or more antigenic peptides or antigenic polypeptides, wherein the sequence of one or more antigenic peptides or antigenic polypeptides is from Borrelia sp. 57Nsk,or to one or more MHC-peptide complexes or MHC multimers, compricing one or more such antigenic peptides.

In one preferred embodiment the present disclosure relates to one or more antigenic peptides or antigenic polypeptides, wherein the sequence of one or more antigenic peptides or antigenic polypeptides is from Borrelia sp. 5MT,or to one or more MHC-peptide complexes or MHC multimers, compricing one or more such antigenic peptides.

In one preferred embodiment the present disclosure relates to one or more antigenic peptides or antigenic polypeptides, wherein the sequence of one or more antigenic peptides or antigenic polypeptides is from Borrelia sp. 6T04-2,or to one or more MHC-peptide complexes or MHC multimers, compricing one or more such antigenic peptides.

In one preferred embodiment the present disclosure relates to one or more antigenic peptides or antigenic polypeptides, wherein the sequence of one or more antigenic peptides or antigenic polypeptides is from Borrelia sp. BR,or to one or more MHC-peptide complexes or MHC multimers, compricing one or more such antigenic peptides.

In one preferred embodiment the present disclosure relates to one or more antigenic peptides or antigenic polypeptides, wherein the sequence of one or more antigenic peptides or antigenic polypeptides is from Borrelia sp. BR 2007,or to one or more MHC-peptide complexes or MHC multimers, compricing one or more such antigenic peptides.

In one preferred embodiment the present disclosure relates to one or more antigenic peptides or antigenic polypeptides, wherein the sequence of one or more antigenic peptides or antigenic polypeptides is from Borrelia sp. C5-N52,or to one or more MHC-peptide complexes or MHC multimers, compricing one or more such antigenic peptides.

In one preferred embodiment the present disclosure relates to one or more antigenic peptides or antigenic polypeptides, wherein the sequence of one or more antigenic peptides or antigenic polypeptides is from Borrelia sp. CB-A1,or to one or more MHC-peptide complexes or MHC multimers, compricing one or more such antigenic peptides.

In one preferred embodiment the present disclosure relates to one or more antigenic peptides or antigenic polypeptides, wherein the sequence of one or more antigenic peptides or antigenic polypeptides is from Borrelia sp. CB-A11,or to one or more MHC-peptide complexes or MHC multimers, compricing one or more such antigenic peptides.

In one preferred embodiment the present disclosure relates to one or more antigenic peptides or antigenic polypeptides, wherein the sequence of one or more antigenic peptides or antigenic polypeptides is from Borrelia sp. CB-A3,or to one or more MHC-peptide complexes or MHC multimers, compricing one or more such antigenic peptides.

In one preferred embodiment the present disclosure relates to one or more antigenic peptides or antigenic polypeptides, wherein the sequence of one or more antigenic peptides or antigenic polypeptides is from Borrelia sp. EFL-S0100110,or to one or more MHC-peptide complexes or MHC multimers, compricing one or more such antigenic peptides.

In one preferred embodiment the present disclosure relates to one or more antigenic peptides or antigenic polypeptides, wherein the sequence of one or more antigenic peptides or antigenic polypeptides is from Borrelia sp. IK/23,or to one or more MHC-peptide complexes or MHC multimers, compricing one or more such antigenic peptides.

In one preferred embodiment the present disclosure relates to one or more antigenic peptides or antigenic polypeptides, wherein the sequence of one or more antigenic peptides or antigenic polypeptides is from Borrelia sp. IM/16,or to one or more MHC-peptide complexes or MHC multimers, compricing one or more such antigenic peptides.

In one preferred embodiment the present disclosure relates to one or more antigenic peptides or antigenic polypeptides, wherein the sequence of one or more antigenic peptides or antigenic polypeptides is from Borrelia sp. IM/19,or to one or more MHC-peptide complexes or MHC multimers, compricing one or more such antigenic peptides.

In one preferred embodiment the present disclosure relates to one or more antigenic peptides or antigenic polypeptides, wherein the sequence of one or more antigenic peptides or antigenic polypeptides is from Borrelia sp. KR1,or to one or more MHC-peptide complexes or MHC multimers, compricing one or more such antigenic peptides.

In one preferred embodiment the present disclosure relates to one or more antigenic peptides or antigenic polypeptides, wherein the sequence of one or more antigenic peptides or antigenic polypeptides is from Borrelia sp. KR3,or to one or more MHC-peptide complexes or MHC multimers, compricing one or more such antigenic peptides.

In one preferred embodiment the present disclosure relates to one or more antigenic peptides or antigenic polypeptides, wherein the sequence of one or more antigenic peptides or antigenic polypeptides is from Borrelia sp. LB-2001,or to one or more MHC-peptide complexes or MHC multimers, compricing one or more such antigenic peptides.

In one preferred embodiment the present disclosure relates to one or more antigenic peptides or antigenic polypeptides, wherein the sequence of one or more antigenic peptides or antigenic polypeptides is from Borrelia sp. LB-M56,or to one or more MHC-peptide complexes or MHC multimers, compricing one or more such antigenic peptides.

In one preferred embodiment the present disclosure relates to one or more antigenic peptides or antigenic polypeptides, wherein the sequence of one or more antigenic peptides or antigenic polypeptides is from Borrelia sp. LB-W100,or to one or more MHC-peptide complexes or MHC multimers, compricing one or more such antigenic peptides.

In one preferred embodiment the present disclosure relates to one or more antigenic peptides or antigenic polypeptides, wherein the sequence of one or more antigenic peptides or antigenic polypeptides is from Borrelia sp. MK-N61,or to one or more MHC-peptide complexes or MHC multimers, compricing one or more such antigenic peptides.

In one preferred embodiment the present disclosure relates to one or more antigenic peptides or antigenic polypeptides, wherein the sequence of one or more antigenic peptides or antigenic polypeptides is from Borrelia sp. NR-N8,or to one or more MHC-peptide complexes or MHC multimers, compricing one or more such antigenic peptides.

In one preferred embodiment the present disclosure relates to one or more antigenic peptides or antigenic polypeptides, wherein the sequence of one or more antigenic peptides or antigenic polypeptides is from Borrelia sp. OkME1,or to one or more MHC-peptide complexes or MHC multimers, compricing one or more such antigenic peptides.

In one preferred embodiment the present disclosure relates to one or more antigenic peptides or antigenic polypeptides, wherein the sequence of one or more antigenic peptides or antigenic polypeptides is from Borrelia sp. PAnz,or to one or more MHC-peptide complexes or MHC multimers, compricing one or more such antigenic peptides.

In one preferred embodiment the present disclosure relates to one or more antigenic peptides or antigenic polypeptides, wherein the sequence of one or more antigenic peptides or antigenic polypeptides is from Borrelia sp. PJes,or to one or more MHC-peptide complexes or MHC multimers, compricing one or more such antigenic peptides.

In one preferred embodiment the present disclosure relates to one or more antigenic peptides or antigenic polypeptides, wherein the sequence of one or more antigenic peptides or antigenic polypeptides is from Borrelia sp. PMai,or to one or more MHC-peptide complexes or MHC multimers, compricing one or more such antigenic peptides.

In one preferred embodiment the present disclosure relates to one or more antigenic peptides or antigenic polypeptides, wherein the sequence of one or more antigenic peptides or antigenic polypeptides is from Borrelia sp.

PMew,or to one or more MHC-peptide complexes or MHC multimers, compricing one or more such antigenic peptides.

In one preferred embodiment the present disclosure relates to one or more antigenic peptides or antigenic polypeptides, wherein the sequence of one or more antigenic peptides or antigenic polypeptides is from Borrelia sp. R57,or to one or more MHC-peptide complexes or MHC multimers, compricing one or more such antigenic peptides.

In one preferred embodiment the present disclosure relates to one or more antigenic peptides or antigenic polypeptides, wherein the sequence of one or more antigenic peptides or antigenic polypeptides is from Borrelia sp. strain Spain,or to one or more MHC-peptide complexes or MHC multimers, compricing one or more such antigenic peptides.

In one preferred embodiment the present disclosure relates to one or more antigenic peptides or antigenic polypeptides, wherein the sequence of one or more antigenic peptides or antigenic polypeptides is from Borrelia sp. TA1,or to one or more MHC-peptide complexes or MHC multimers, compricing one or more such antigenic peptides.

In one preferred embodiment the present disclosure relates to one or more antigenic peptides or antigenic polypeptides, wherein the sequence of one or more antigenic peptides or antigenic polypeptides is from Borrelia sp. TM,or to one or more MHC-peptide complexes or MHC multimers, compricing one or more such antigenic peptides.

In one preferred embodiment the present disclosure relates to one or more antigenic peptides or antigenic polypeptides, wherein the sequence of one or more antigenic peptides or antigenic polypeptides is from Borrelia sp. TM1,or to one or more MHC-peptide complexes or MHC multimers, compricing one or more such antigenic peptides.

In one preferred embodiment the present disclosure relates to one or more antigenic peptides or antigenic polypeptides, wherein the sequence of one or more antigenic peptides or antigenic polypeptides is from Borrelia sp. TM2,or to one or more MHC-peptide complexes or MHC multimers, compricing one or more such antigenic peptides.

Borrelia genome

All species of Borrelia have linear chromosomes ranging in size from 900,000 to 920,000 base pairs, with an accompaniment of circular and linear plasmids (some species contain up to 20 different plasmids). Between the linear chromosome and array of plasmids there is a high degree of redundancy in the genetic sequence. Although it has not been determined yet, it seems likely that the origin of replication of the main chromosome in species of Borrelia is almost exactly centered on the linear chromosome.

Some of the protein sequences from Borrelia burgdorferi, Borrelia garinii and Borrelia afzelii are homolog protein sequences. This means that the present disclosure in one embodiment relates to one or more antigenic peptides or antigenic polypeptides, wherein the one or more antigenic peptides or antigenic polypeptides are encoded by one or more of the Borrelia species Borrelia burgdorferi, Borrelia garinii and Borrelia afzelii, or to one or more MHC-peptide complexes or MHC multimers, compricing one or more such antigenic peptides.

In another embodiment the disclosure relates to one or more antigenic peptides or antigenic polypeptides, wherein the one or more antigenic peptides or antigenic polypeptides are encoded by Borrelia burgdorferi and not by Borrelia garinii or Borrelia afzelii, or to one or more MHC-peptide complexes or MHC multimers, compricing one or more such antigenic peptides.

In another embodiment the disclosure relates to one or more antigenic peptides or antigenic polypeptides, wherein the one or more antigenic peptides or antigenic polypeptides are encoded by Borrelia garinii and not by Borrelia burgdorferi or Borrelia afzelii, or to one or more MHC-peptide complexes or MHC multimers, compricing one or more such antigenic peptides.

In another embodiment the disclosure relates to one or more antigenic peptides or antigenic polypeptides, wherein the one or more antigenic peptides or antigenic polypeptides are encoded by Borrelia afzelii and not by Borrelia garinii or Borrelia burgdorferi, or to one or more MHC-peptide complexes or MHC multimers, compricing one or more such antigenic peptides.

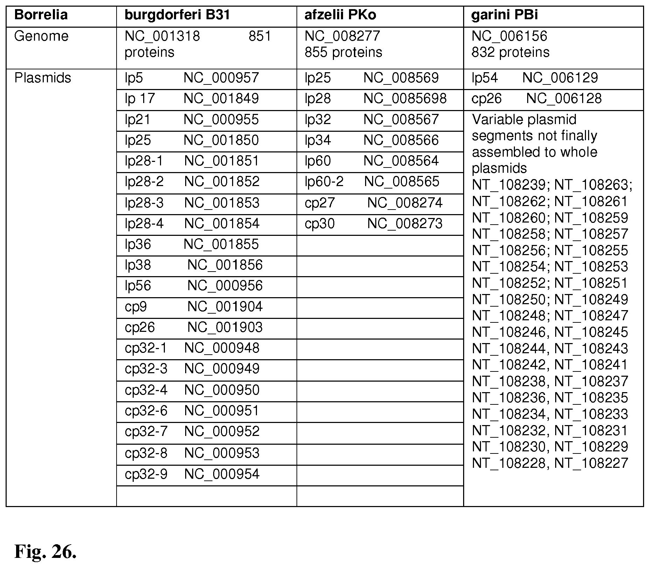

The present disclosure relates in one embodiment to one or more antigenic peptides or antigenic polypeptides comprising a sequence encoded by a Borrelia genome and/or plasmid, or to one or more MHC-peptide complexes or MHC multimers, compricing one or more such antigenic peptides. The genomes and plasmids of Borrelia burgdoferi B31, Borrelia afzelii Pko and Borrelia garinii PBi are listed in Figure 26-29. The peptides can e.g. be 8 mers, 9 mers, 10 mers, 11 mers, 12 mers, 13 mers, 14 mers, 15 mers or 16 mers.

In one preferred embodiment the present disclosure relates to one or more antigenic peptides or antigenic polypeptides comprising a sequence encoded by the genome (NC_001318) of Borrelia burgdorferi B31, or to one or more MHC-peptide complexes or MHC multimers, compricing one or more such antigenic peptides.

In one preferred embodiment the present disclosure relates to one or more antigenic peptides or antigenic polypeptides comprising a sequence encoded by the plasmid lp5 (NC_000957) of Borrelia burgdorferi B31, or to one or more MHC-peptide complexes or MHC multimers, compricing one or more such antigenic peptides.

In one preferred embodiment the present disclosure relates to one or more antigenic peptides or antigenic polypeptides comprising a sequence encoded by the plasmid lp 17 (NC_001849) of Borrelia burgdorferi B31, or to one or more MHC-peptide complexes or MHC multimers, compricing one or more such antigenic peptides.

In one preferred embodiment the present disclosure relates to one or more antigenic peptides or antigenic polypeptides comprising a sequence encoded by the plasmid lp21 (NC_000955) of Borrelia burgdorferi B31, or to one or more MHC-peptide complexes or MHC multimers, compricing one or more such antigenic peptides.

In one preferred embodiment the present disclosure relates to one or more antigenic peptides or antigenic polypeptides comprising a sequence encoded by the plasmid lp25 (NC_001850) of Borrelia burgdorferi B31, or to one or more MHC-peptide complexes or MHC multimers, compricing one or more such antigenic peptides.

In one preferred embodiment the present disclosure relates to one or more antigenic peptides or antigenic polypeptides comprising a sequence encoded by the plasmid lp28-1 (NC_001851) of Borrelia burgdorferi B31, or to one or more MHC-peptide complexes or MHC multimers, compricing one or more such antigenic peptides.

In one preferred embodiment the present disclosure relates to one or more antigenic peptides or antigenic polypeptides comprising a sequence encoded by the plasmid lp28-2 (NC_001852) of Borrelia burgdorferi B31, or to one or more MHC-peptide complexes or MHC multimers, compricing one or more such antigenic peptides.

In one preferred embodiment the present disclosure relates to one or more antigenic peptides or antigenic polypeptides comprising a sequence encoded by the plasmid lp28-3 (NC_001853) of Borrelia burgdorferi B31, or to one or more MHC-peptide complexes or MHC multimers, compricing one or more such antigenic peptides.

In one preferred embodiment the present disclosure relates to one or more antigenic peptides or antigenic polypeptides comprising a sequence encoded by the plasmid lp28-4 (NC_001854) of Borrelia burgdorferi B31, or to one or more MHC-peptide complexes or MHC multimers, compricing one or more such antigenic peptides.

In one preferred embodiment the present disclosure relates to one or more antigenic peptides or antigenic polypeptides comprising a sequence encoded by the plasmid lp36 (NC_001855) of Borrelia burgdorferi B31, or to one or more MHC-peptide complexes or MHC multimers, compricing one or more such antigenic peptides.

In one preferred embodiment the present disclosure relates to one or more antigenic peptides or antigenic polypeptides comprising a sequence encoded by the plasmid lp38 (NC_001856) of Borrelia burgdorferi B31, or to one or more MHC-peptide complexes or MHC multimers, compricing one or more such antigenic peptides.

In one preferred embodiment the present disclosure relates to one or more antigenic peptides or antigenic polypeptides comprising a sequence encoded by the plasmid lp56 (NC_000956) of Borrelia burgdorferi B31, or to one or more MHC-peptide complexes or MHC multimers, compricing one or more such antigenic peptides.

In one preferred embodiment the present disclosure relates to one or more antigenic peptides or antigenic polypeptides comprising a sequence encoded by the plasmid cp9 (NC_001904) of Borrelia burgdorferi B31, or to one or more MHC-peptide complexes or MHC multimers, compricing one or more such antigenic peptides.

In one preferred embodiment the present disclosure relates to one or more antigenic peptides or antigenic polypeptides comprising a sequence encoded by the plasmid lp28-4 (NC_001854) of Borrelia burgdorferi B31, or to one or more MHC-peptide complexes or MHC multimers, compricing one or more such antigenic peptides.

In one preferred embodiment the present disclosure relates to one or more antigenic peptides or antigenic polypeptides comprising a sequence encoded by the plasmid cp26 (NC_001903) of Borrelia burgdorferi B31, or to one or more MHC-peptide complexes or MHC multimers, compricing one or more such antigenic peptides.

In one preferred embodiment the present disclosure relates to one or more antigenic peptides or antigenic polypeptides comprising a sequence encoded by the plasmid cp32-1 (NC_000948) of Borrelia burgdorferi B31, or to one or more MHC-peptide complexes or MHC multimers, compricing one or more such antigenic peptides.

In one preferred embodiment the present disclosure relates to one or more antigenic peptides or antigenic polypeptides comprising a sequence encoded by the plasmid cp32-3 (NC_000949) of Borrelia burgdorferi B31, or to one or more MHC-peptide complexes or MHC multimers, compricing one or more such antigenic peptides.

In one preferred embodiment the present disclosure relates to one or more antigenic peptides or antigenic polypeptides comprising a sequence encoded by the plasmid cp32-4 (NC_000950) of Borrelia burgdorferi B31, or to one or more MHC-peptide complexes or MHC multimers, compricing one or more such antigenic peptides.

In one preferred embodiment the present disclosure relates to one or more antigenic peptides or antigenic polypeptides comprising a sequence encoded by the plasmid cp32-6 (NC_000951) of Borrelia burgdorferi B31, or to one or more MHC-peptide complexes or MHC multimers, compricing one or more such antigenic peptides.

In one preferred embodiment the present disclosure relates to one or more antigenic peptides or antigenic polypeptides comprising a sequence encoded by the plasmid cp32-7 (NC_000952) of Borrelia burgdorferi B31, or to one or more MHC-peptide complexes or MHC multimers, compricing one or more such antigenic peptides.

In one preferred embodiment the present disclosure relates to one or more antigenic peptides or antigenic polypeptides comprising a sequence encoded by the plasmid cp32-8 (NC_000953) of Borrelia burgdorferi B31, or to one or more MHC-peptide complexes or MHC multimers, compricing one or more such antigenic peptides.

In one preferred embodiment the present disclosure relates to one or more antigenic peptides or antigenic polypeptides comprising a sequence encoded by the plasmid cp32-9 (NC_000954) of Borrelia burgdorferi B31, or to one or more MHC-peptide complexes or MHC multimers, compricing one or more such antigenic peptides.

In one preferred embodiment the present disclosure relates to one or more antigenic peptides or antigenic polypeptides comprising a sequence encoded by the genome (NC_008277) of Borrelia afzelii PKo, or to one or more MHC-peptide complexes or MHC multimers, compricing one or more such antigenic peptides.

In one preferred embodiment the present disclosure relates to one or more antigenic peptides or antigenic polypeptides comprising a sequence encoded by the plasmid lp25 (NC_008569) of Borrelia afzelii PKo, or to one or more MHC-peptide complexes or MHC multimers, compricing one or more such antigenic peptides.

In one preferred embodiment the present disclosure relates to one or more antigenic peptides or antigenic polypeptides comprising a sequence encoded by the plasmid lp28 (NC_0085698) of Borrelia afzelii PKo, or to one or more MHC-peptide complexes or MHC multimers, compricing one or more such antigenic peptides.

In one preferred embodiment the present disclosure relates to one or more antigenic peptides or antigenic polypeptides comprising a sequence encoded by the plasmid lp32 (NC_008567) of Borrelia afzelii PKo, or to one or more MHC-peptide complexes or MHC multimers, compricing one or more such antigenic peptides.

In one preferred embodiment the present disclosure relates to one or more antigenic peptides or antigenic polypeptides comprising a sequence encoded by the plasmid lp34 (NC_008566) of Borrelia afzelii PKo, or to one or more MHC-peptide complexes or MHC multimers, compricing one or more such antigenic peptides.

In one preferred embodiment the present disclosure relates to one or more antigenic peptides or antigenic polypeptides comprising a sequence encoded by the plasmid lp60 (NC_008564) of Borrelia afzelii PKo, or to one or more MHC-peptide complexes or MHC multimers, compricing one or more such antigenic peptides.

In one preferred embodiment the present disclosure relates to one or more antigenic peptides or antigenic polypeptides comprising a sequence encoded by the plasmid lp60-2 (NC_008565) of Borrelia afzelii PKo, or to one or more MHC-peptide complexes or MHC multimers, compricing one or more such antigenic peptides.

In one preferred embodiment the present disclosure relates to one or more antigenic peptides or antigenic polypeptides comprising a sequence encoded by the plasmid cp27 (NC_008274) of Borrelia afzelii PKo, or to one or more MHC-peptide complexes or MHC multimers, compricing one or more such antigenic peptides.

In one preferred embodiment the present disclosure relates to one or more antigenic peptides or antigenic polypeptides comprising a sequence encoded by the plasmid cp30 (NC_008273) of Borrelia afzelii PKo, or to one or more MHC-peptide complexes or MHC multimers, compricing one or more such antigenic peptides.

In one preferred embodiment the present disclosure relates to one or more antigenic peptides or antigenic polypeptides comprising a sequence encoded by the genome (NC_006156) of Borrelia garinii PBi, or to one or more MHC-peptide complexes or MHC multimers, compricing one or more such antigenic peptides.

In one preferred embodiment the present disclosure relates to one or more antigenic peptides or antigenic polypeptides comprising a sequence encoded by the plasmid Ip54 (NC_006129) of Borrelia garinii PBi, or to one or more MHC-peptide complexes or MHC multimers, compricing one or more such antigenic peptides.

In one preferred embodiment the present disclosure relates to one or more antigenic peptides or antigenic polypeptides comprising a sequence encoded by the plasmid cp26 (NC_006128) of Borrelia garinii PBi, or to one or more MHC-peptide complexes or MHC multimers, compricing one or more such antigenic peptides.

In another preferred embodiment the present disclosure relates to one or more antigenic peptides or antigenic polypeptides comprising a sequence encoded by variable plasmid segments not finally assembled to whole plasmids of Borrelia garinii PBi, or to one or more MHC-peptide complexes or MHC multimers, compricing one or more such antigenic peptides. These variable plasmid segments not finally assembled to whole plasmids can in one preferred embodiment of the present disclosure be selected from the group consisting of NT_108239, NT_108263, NT_108262, NT_108261 , NT_108260, NT_108259 , NT_108258, NT_108257 , NT_108256, NT_108255 , NT_108254, NT_108253 NT_108252, NT_108251, NT_108250, NT_108249, NT_108248, NT_108247, NT_108246, NT_108245 , NT_108244, NT_108243, NT_108242, NT_108241, NT_108240, NT_108238, NT_108237, NT_108236, NT_108235, NT_108234, NT_108233, NT_108232, NT_108231, NT_108230, NT_108229, NT_108228 and NT_108227.

In yet another embodiment the present disclosure relates to one or more antigenic peptides or antigenic polypeptides, optionally compriced in one or more MHC-peptide complexes or MHC multimers, where the antigenic peptides or antigenic polypeptides comprise a sequence encoded by a Borrelia genome and/or plasmid from one or more of the Borrelia species or subspecies: Borrelia anserina, Borrelia barbouri, Borrelia afzelii, Borrelia afzelii ACA-1, Borrelia afzelii K78, Borrelia afzelii PKo, Borrelia andersonii, Borrelia bissettii, Borrelia burgdorferi, Borrelia burgdorferi 118a, Borrelia burgdorferi 156a, Borrelia burgdorferi 29805, Borrelia burgdorferi 64b, Borrelia burgdorferi 72a, Borrelia burgdorferi 80a, Borrelia burgdorferi 94a, Borrelia burgdorferi B31, Borrelia burgdorferi Bol26, Borrelia burgdorferi CA-11.2a, Borrelia burgdorferi Borrelia burgdorferi ZS7, Borrelia californiensis, Borrelia garinii, Borrelia garinii PBi, Borrelia garinii PBr, Borrelia genomosp. 1, Borrelia genomosp. 2, Borrelia japonica, Borrelia lusitaniae, Borrelia spielmanii, Borrelia spielmanii A14S, Borrelia tanukii, Borrelia turdi, Borrelia valaisiana, Borrelia valaisiana VS116, Candidatus Borrelia texasensis, Borrelia sp. AA4Pool, Borrelia sp. AI-1, Borrelia sp. B31, Borrelia sp. BC-1, Borrelia sp. CA1133, Borrelia sp. CA1176, Borrelia sp. CA128, Borrelia sp. CA13, Borrelia sp. CA134, Borrelia sp. CA142, Borrelia sp. CA20, Borrelia sp. CA22, Borrelia sp. CA27, Borrelia sp. CA28, Borrelia sp. CA29, Borrelia sp. CA31, Borrelia sp. CA33, Borrelia sp. CA370, Borrelia sp. CA372, Borrelia sp. CA378, Borrelia sp. CA388, Borrelia sp. CA393, Borrelia sp. CA394, Borrelia sp. CA395, Borrelia sp. CA399, Borrelia sp. CA400, Borrelia sp. CA401, Borrelia sp. CA402, Borrelia sp. CA404, Borrelia sp. CA411, Borrelia sp. CA426, Borrelia sp. CA443, Borrelia sp. CA446, Borrelia sp. CA448, Borrelia sp. CA462, Borrelia sp. CA468, Borrelia sp. CA502, Borrelia sp. CA504, Borrelia sp. CA507, Borrelia sp. CA547, Borrelia sp. CA552, Borrelia sp. CA8, Borrelia sp. D22, Borrelia sp. D35, Borrelia sp. FD-1, Borrelia sp. FL18, Borrelia sp. FL27, Borrelia sp. FL35, Borrelia sp. FL42, Borrelia sp. HN6, Borrelia sp. HN7, Borrelia sp. HN8, Borrelia sp. HNM13, Borrelia sp. HNM14, Borrelia sp. HNM19, Borrelia sp. IA1, Borrelia sp. Ir-3519, Borrelia sp. Ir-4721, Borrelia sp. Ir-4812, Borrelia sp. Ir-5215, Borrelia sp. LV5, Borrelia sp. MI-2, Borrelia sp. MI-5, Borrelia sp. MI-6, Borrelia sp. MI-8, Borrelia sp. MI-9, Borrelia sp. MOD-1, Borrelia sp. MOD-5, Borrelia sp. MOK-3a, Borrelia sp. MOS-1b, Borrelia sp. NE49, Borrelia sp. NE581, Borrelia sp. PHaP, Borrelia sp. PSigll, Borrelia sp. SCGT-10, Borrelia sp. SCGT-8a, Borrelia sp. SCI-2, Borrelia sp. SCW-30h, Borrelia sp. SI-1, Borrelia sp. SI-10, Borrelia sp. SM-1, Borrelia sp. SV1, Borrelia sp. W97F51, Borrelia sp. Z41293, Borrelia sp. Z41493, Borrelia coriaceae, Borrelia crocidurae, Borrelia duttonii, Borrelia duttonii Ly, Borrelia hermsii, Borrelia hermsii DAH, Borrelia hispanica, Borrelia lonestari, Borrelia miyamotoi, Borrelia parkeri, Borrelia persica, Borrelia recurrentis, Borrelia recurrentis A1, Borrelia sinica, Borrelia theileri, Borrelia turcica, Borrelia turicatae, Borrelia turicatae 91E135, Borrelia sp., Borrelia sp. 'Lake Gaillard', Borrelia sp. 000133, Borrelia sp. 010298, Borrelia sp. 10MT, Borrelia sp. 5145, Borrelia sp. 57Nsk, Borrelia sp. 5MT, Borrelia sp. 6T04-2, Borrelia sp. BR, Borrelia sp. BR 2007, Borrelia sp. C5-N52, Borrelia sp. CB-A1, Borrelia sp. CB-A11, Borrelia sp. CB-A3, Borrelia sp. EFL-S0100110, Borrelia sp. IK/23, Borrelia sp. IM/16, Borrelia sp. IM/19, Borrelia sp. KR1, Borrelia sp. KR3, Borrelia sp. LB-2001, Borrelia sp. LB-M56, Borrelia sp. LB-W100, Borrelia sp. MK-N61, Borrelia sp. NR-N8, Borrelia sp. OkME1, Borrelia sp. PAnz, Borrelia sp. PJes, Borrelia sp. PMai, Borrelia sp. PMew, Borrelia sp. R57, Borrelia sp. strain Spain, Borrelia sp. TA1, Borrelia sp. TM, Borrelia sp. TM1 and Borrelia sp. TM2. These peptides can e.g. be 8 mers, 9 mers, 10 mers, 11 mers, 12 mers, 13 mers, 14 mers, 15 mers or 16 mers.

Borrelia proteome

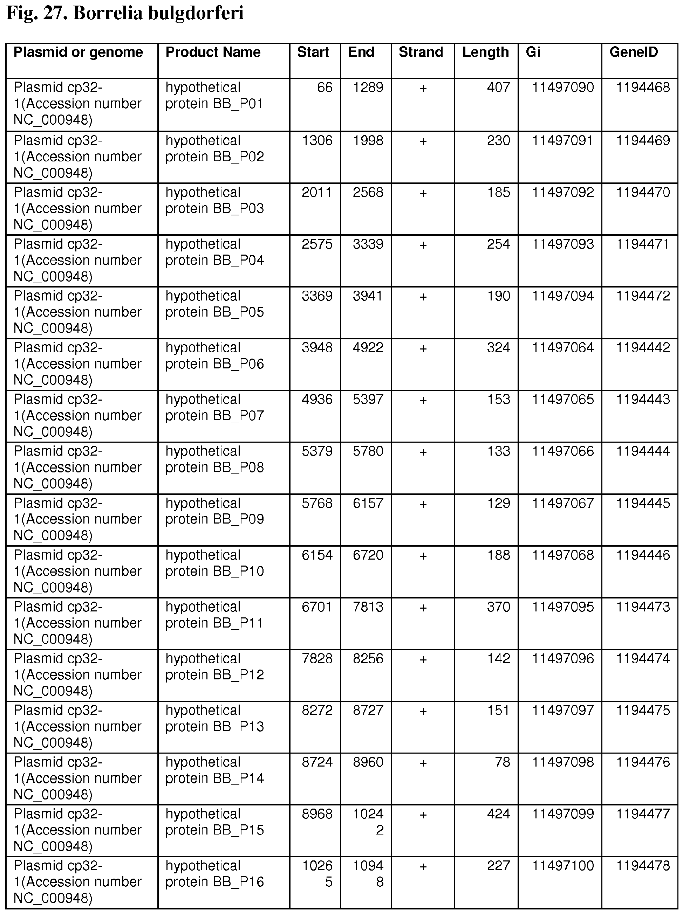

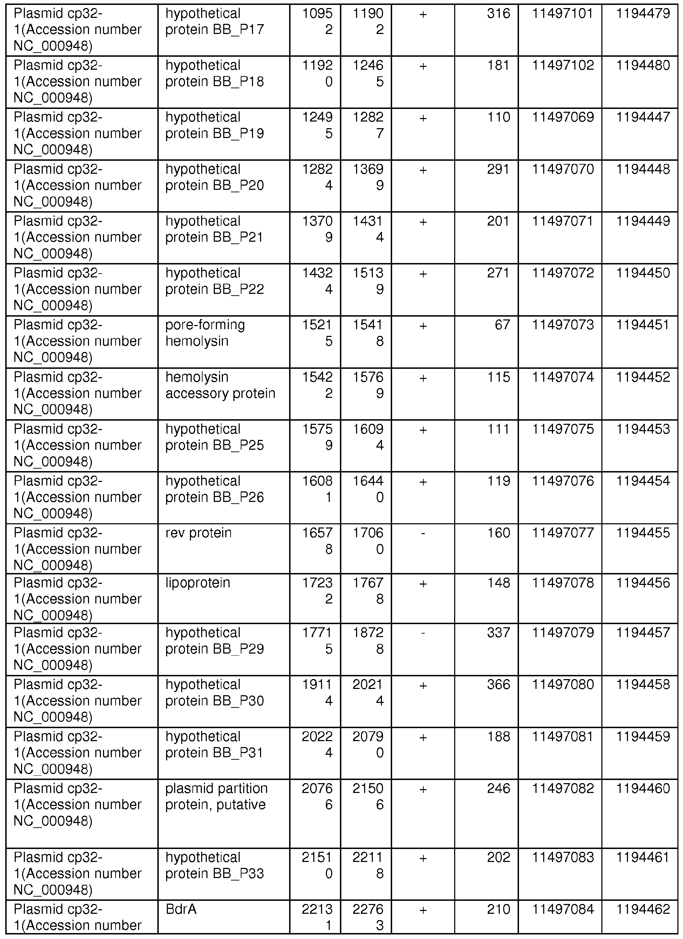

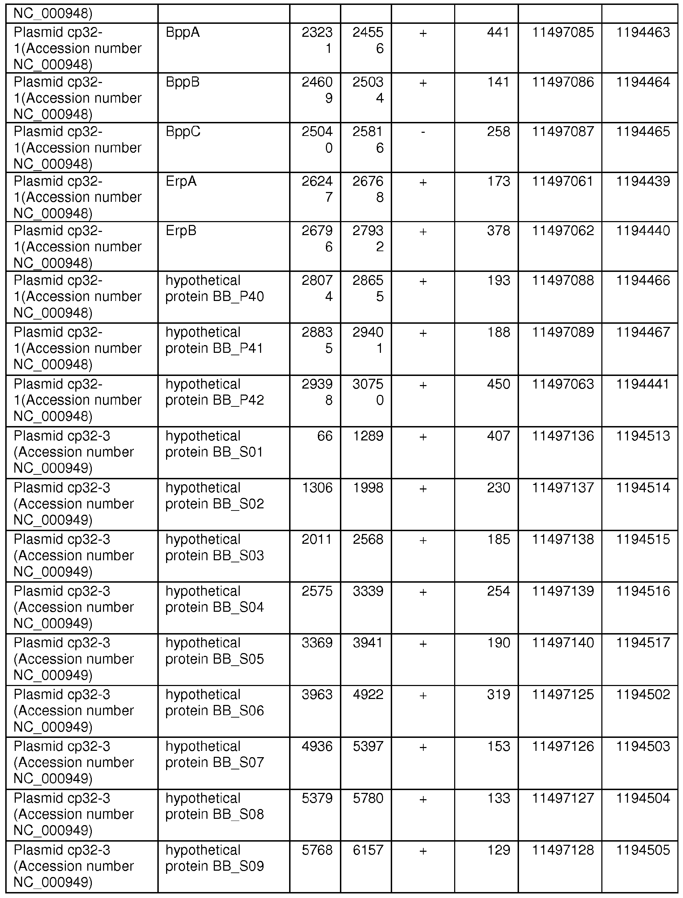

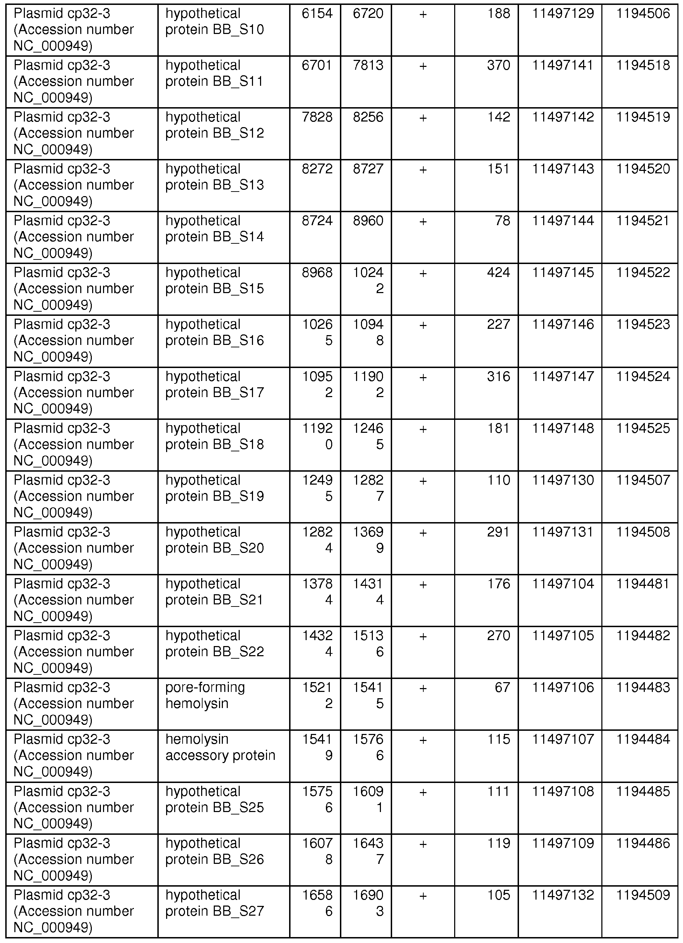

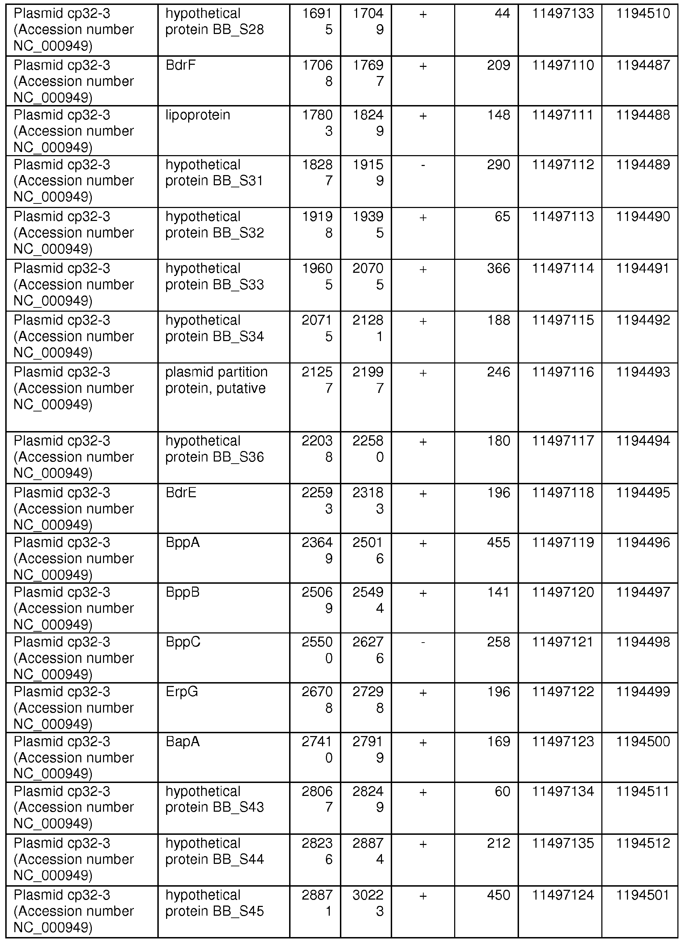

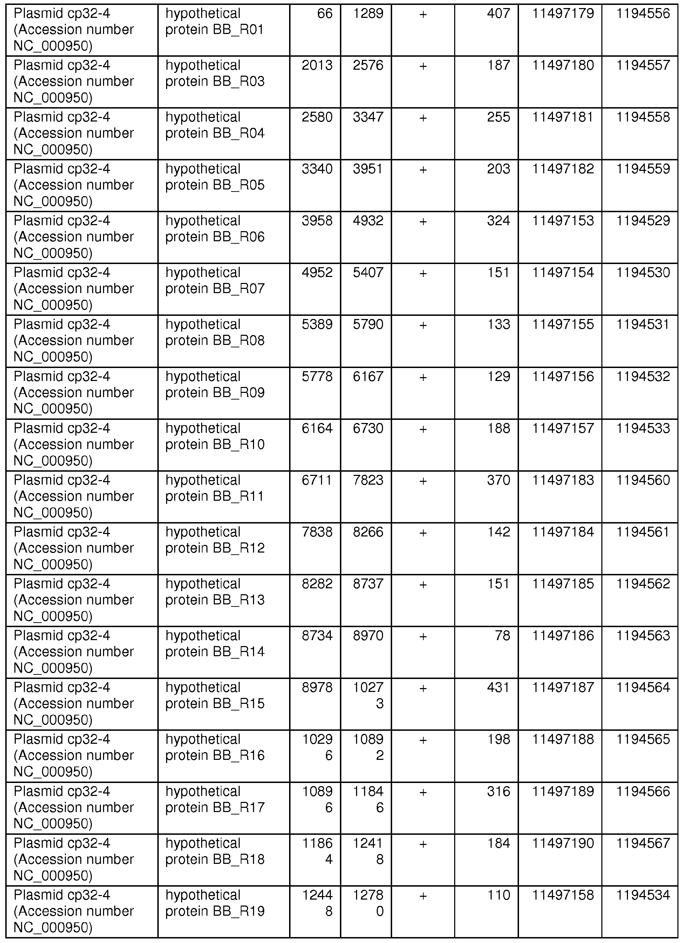

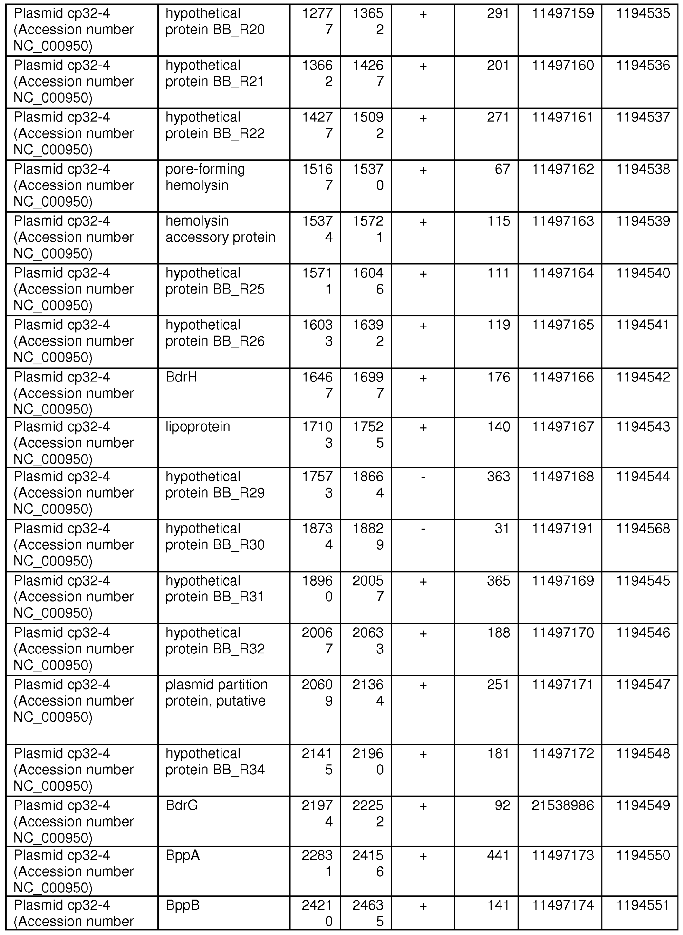

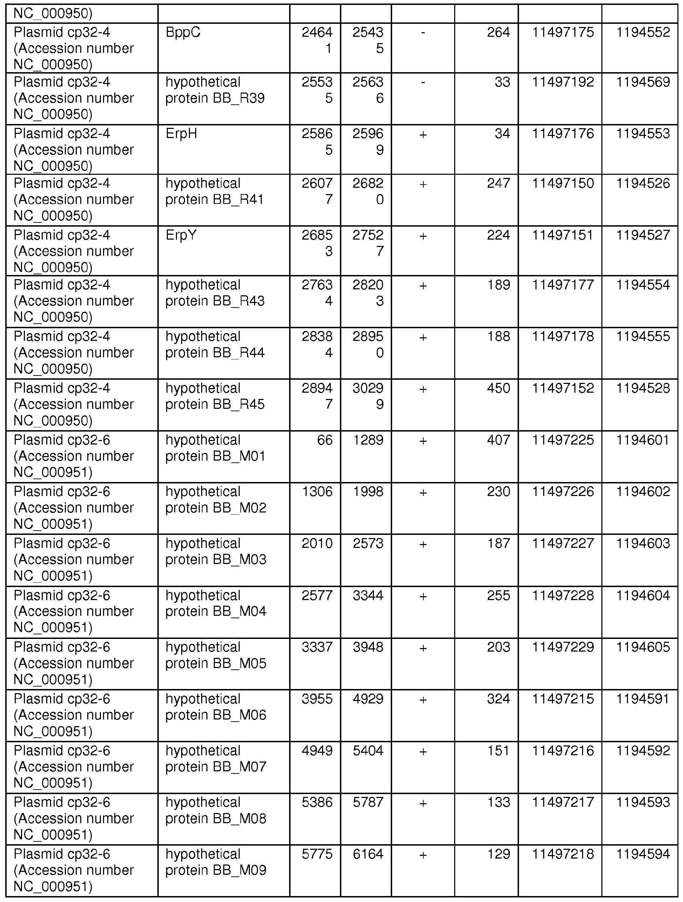

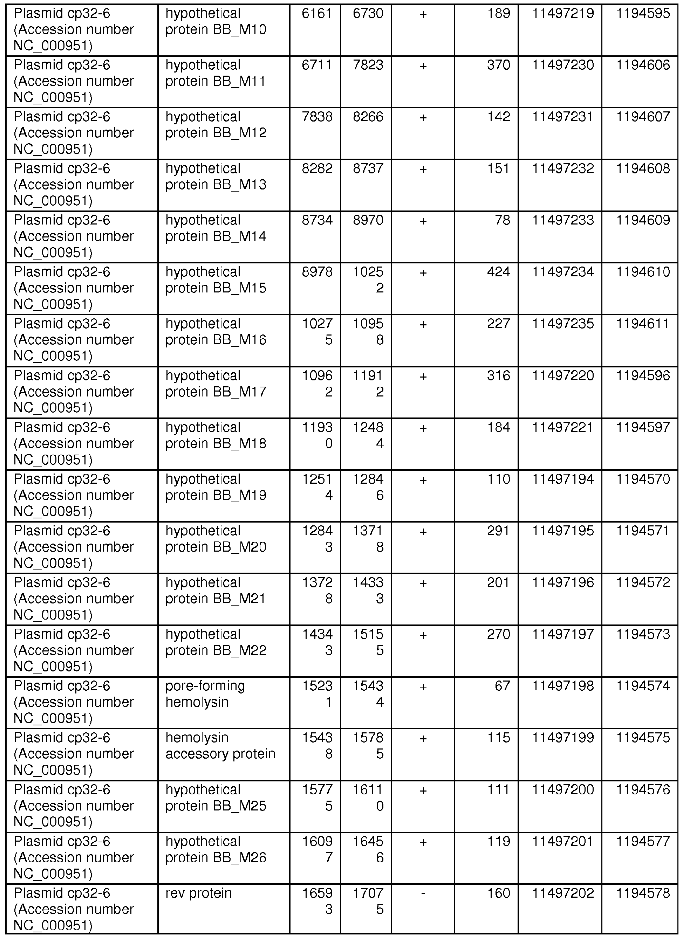

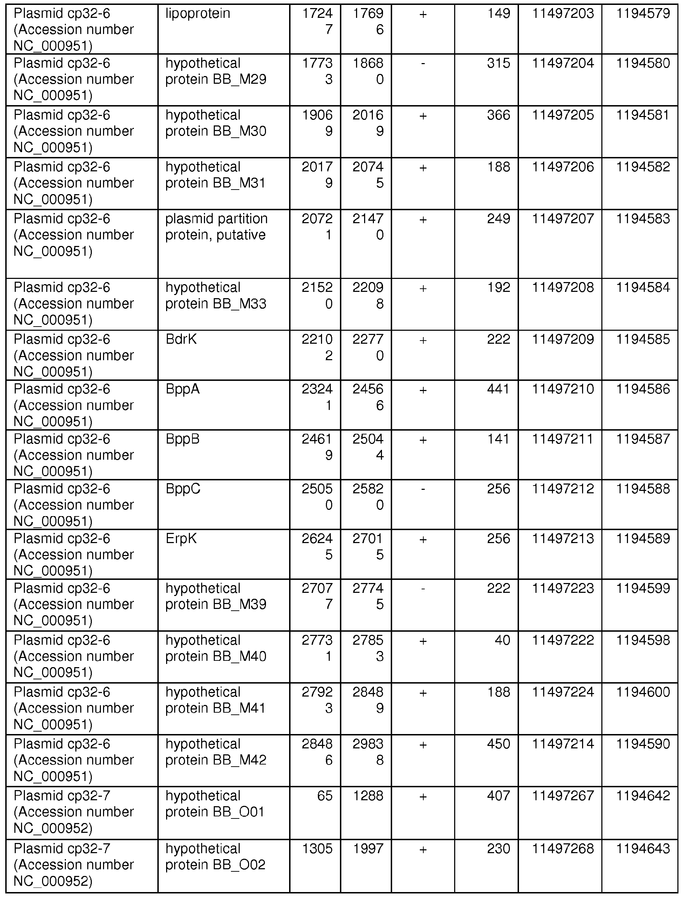

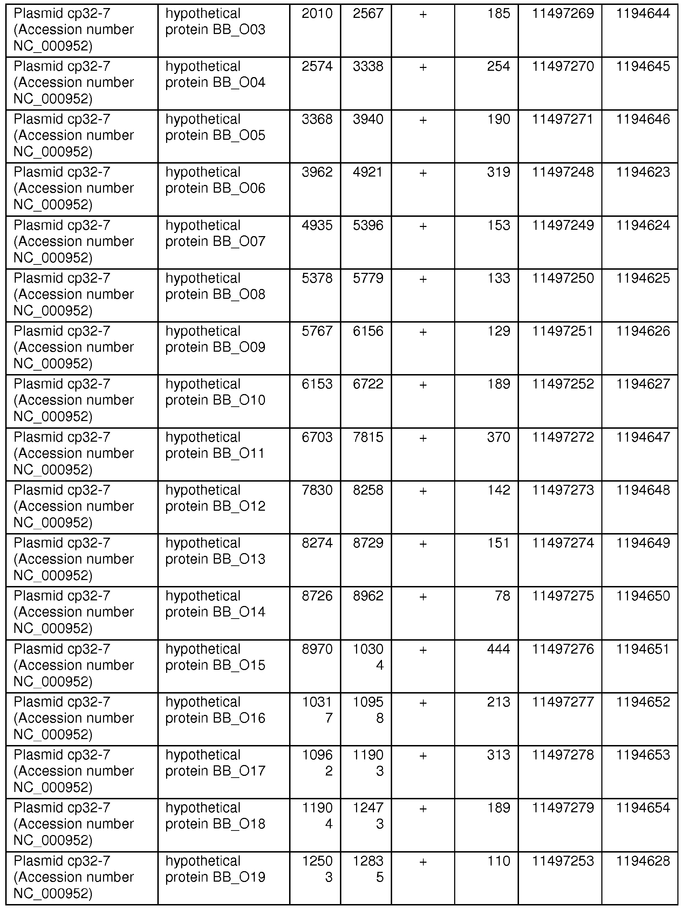

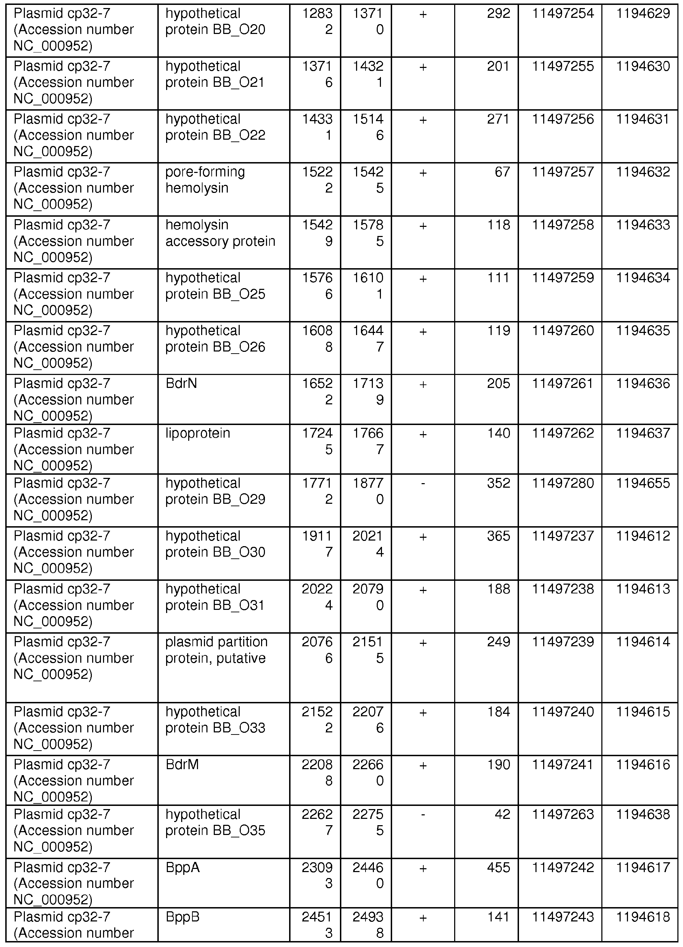

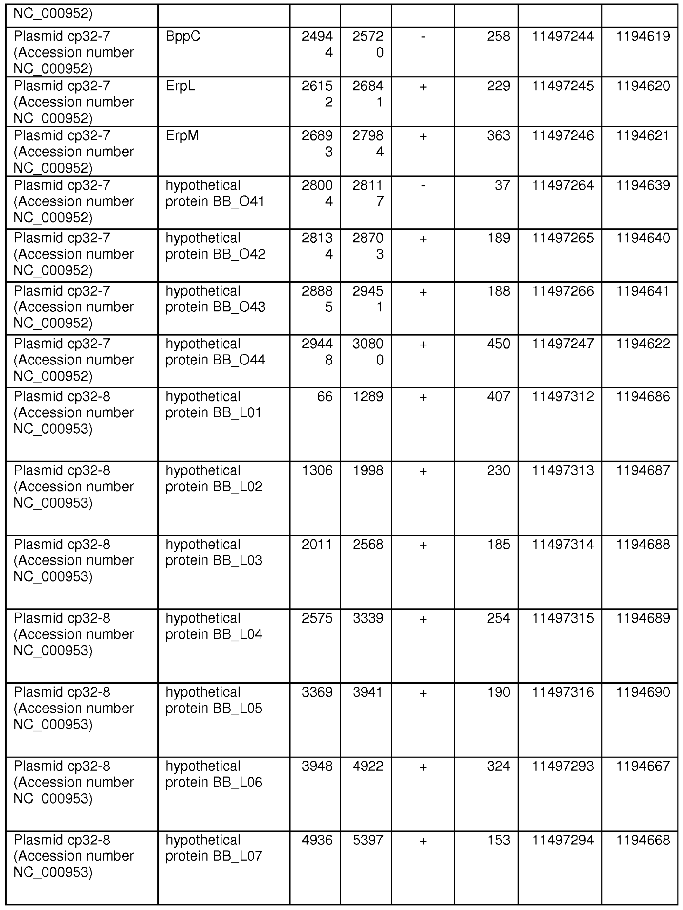

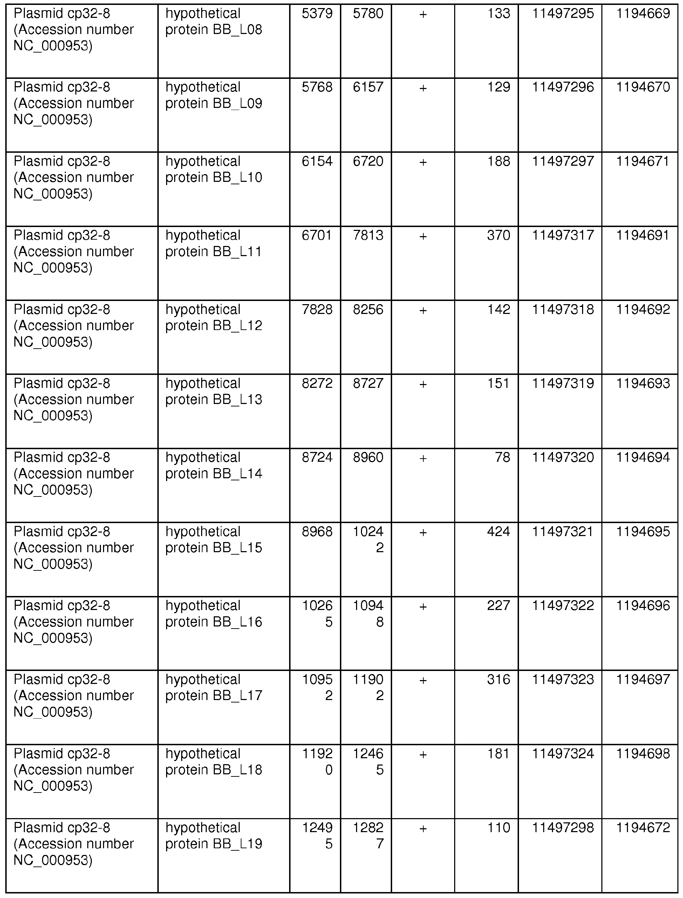

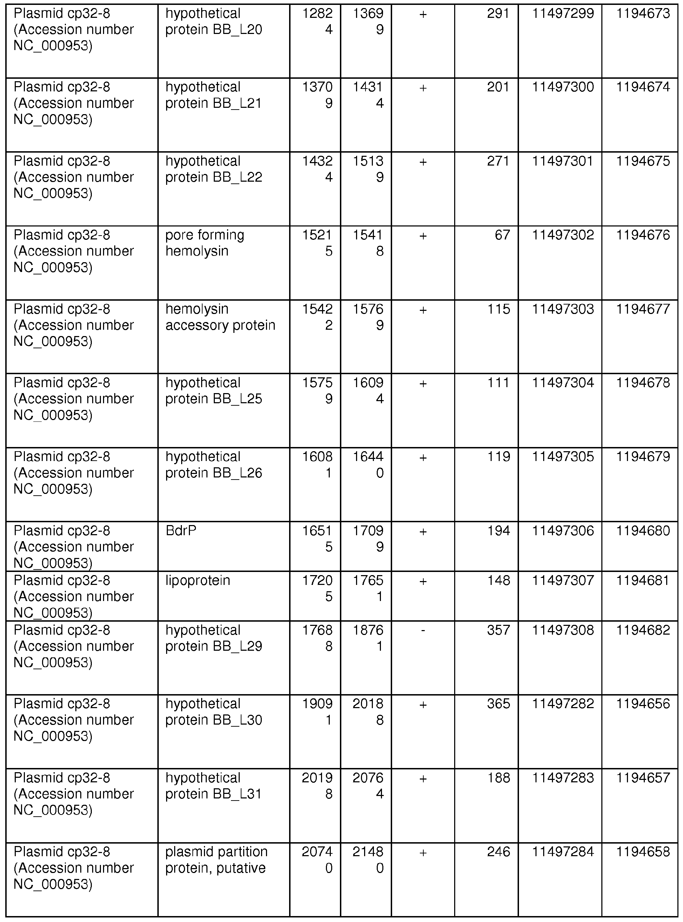

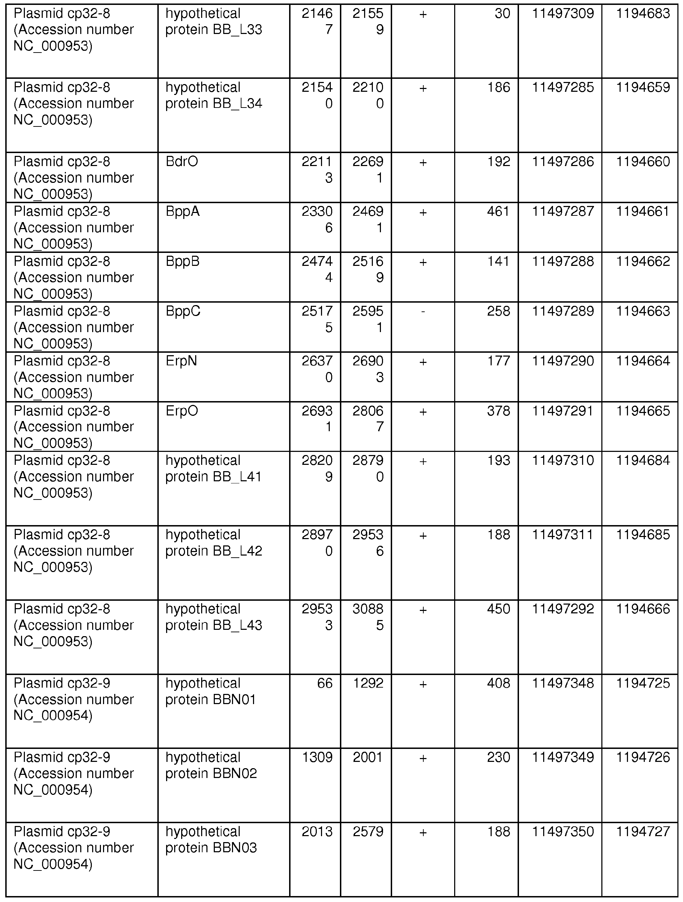

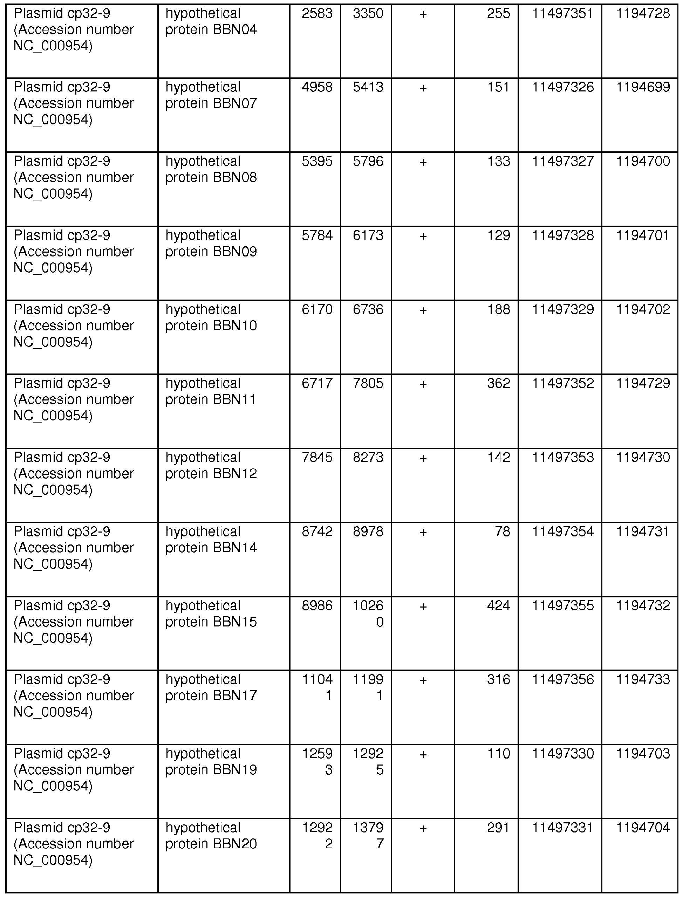

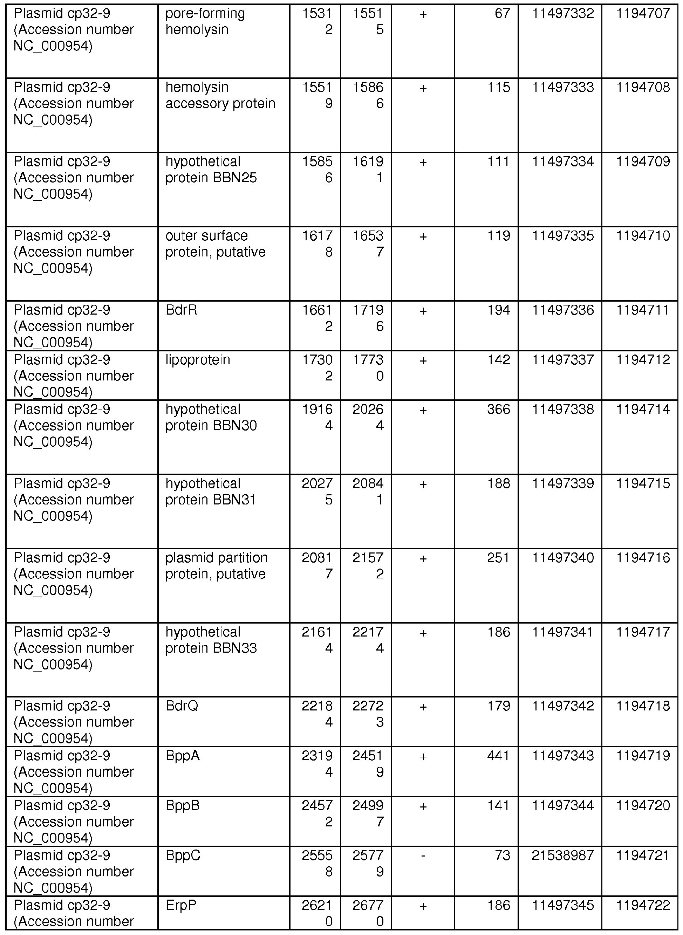

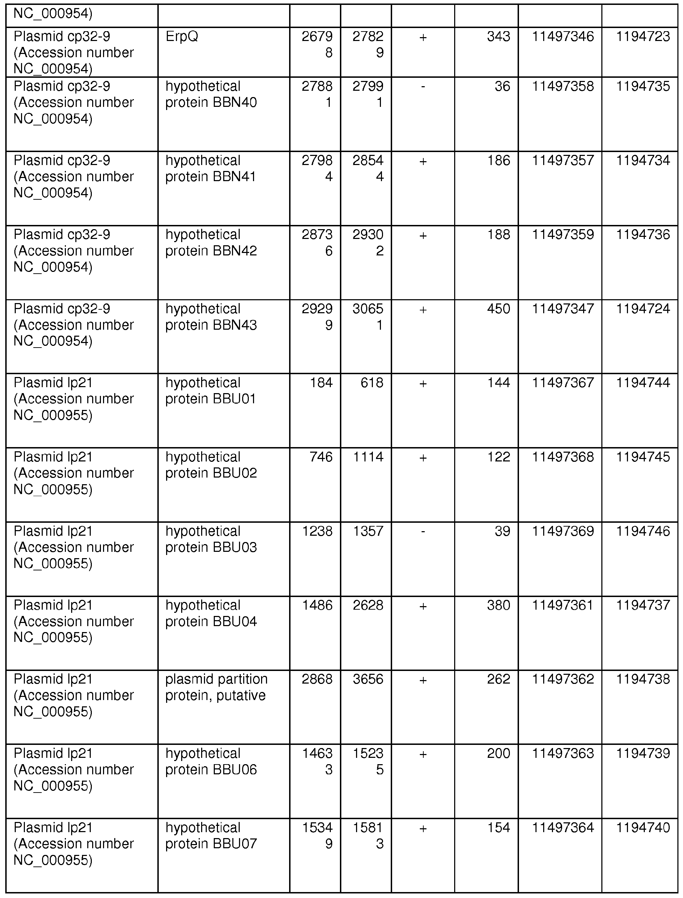

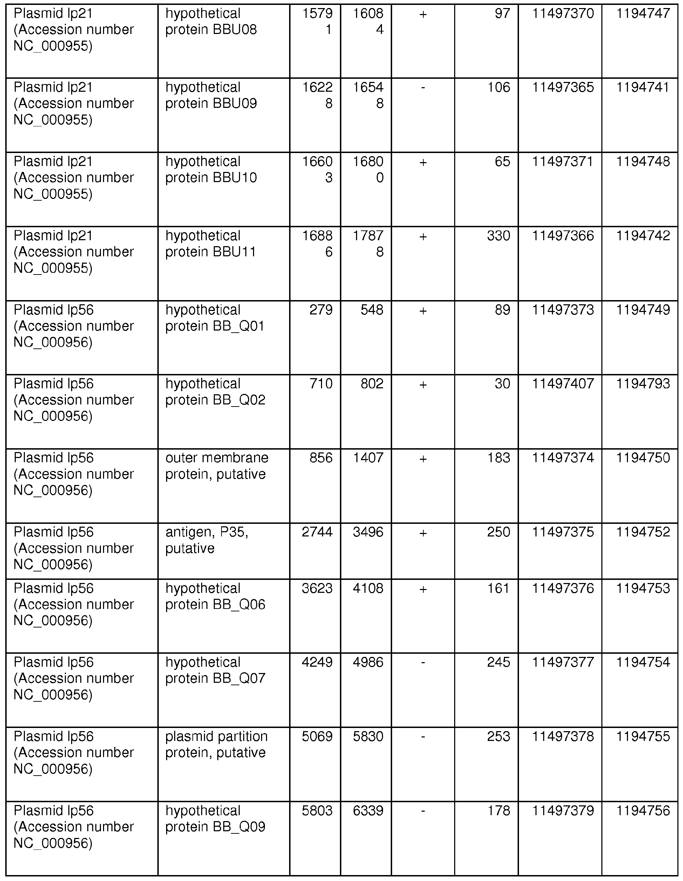

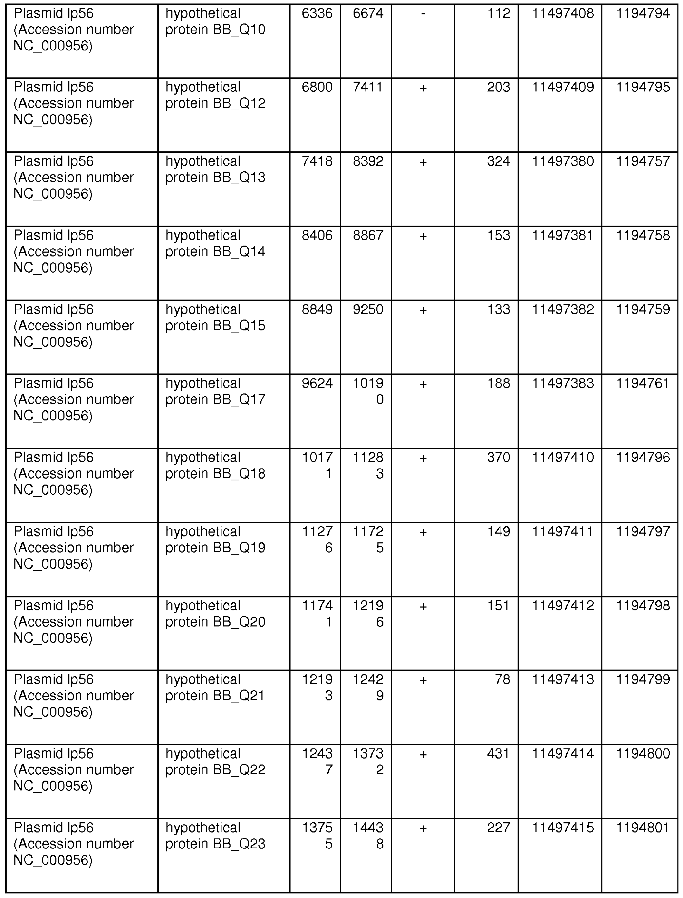

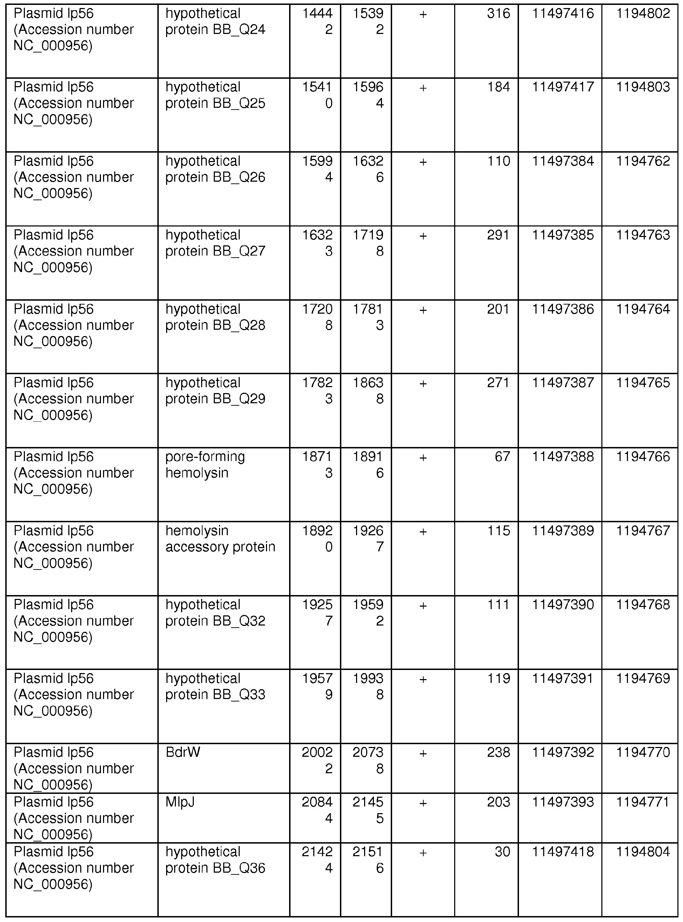

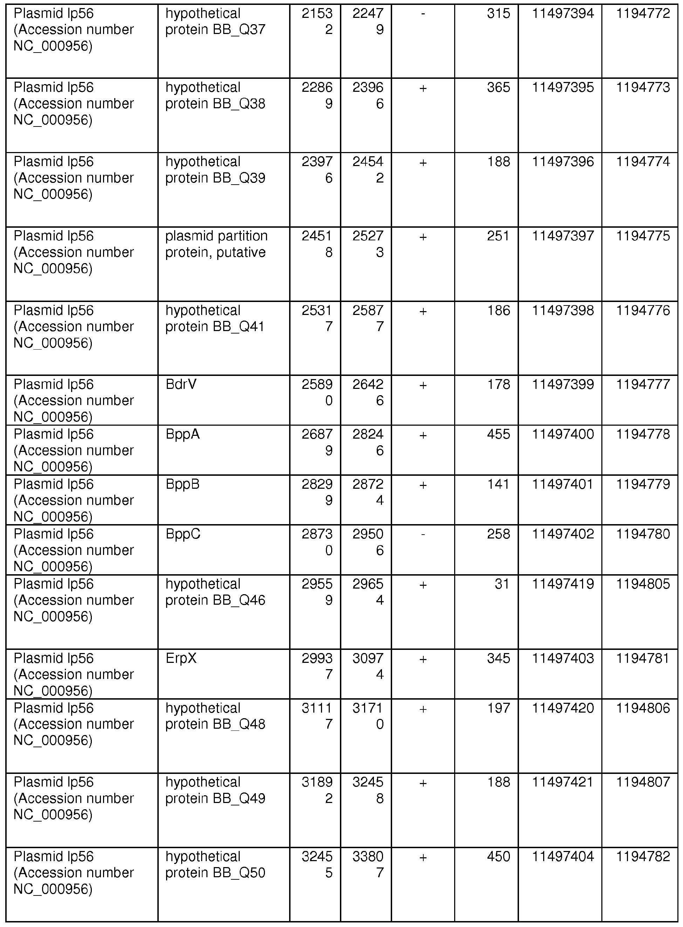

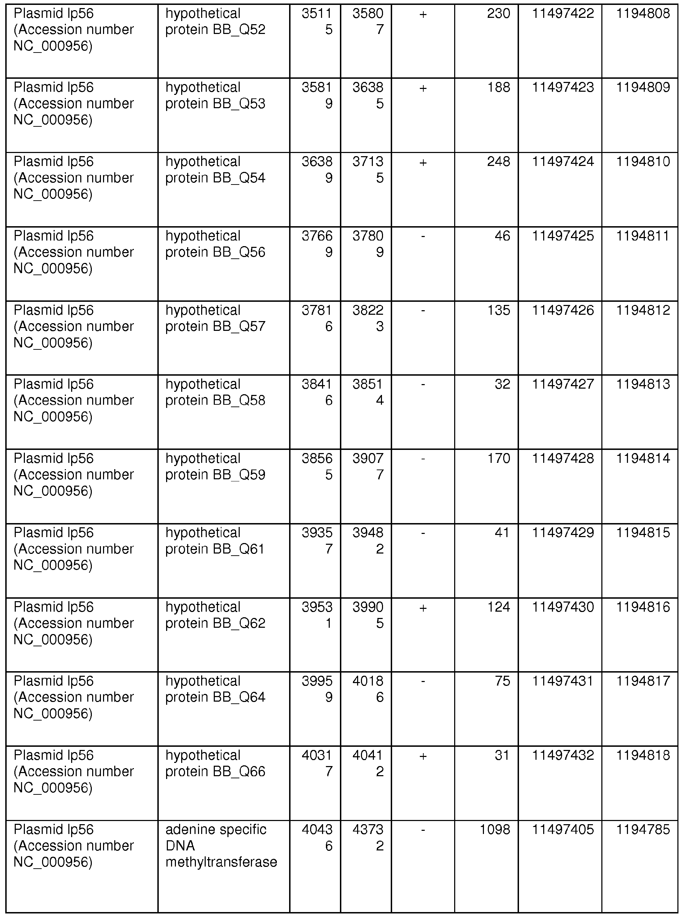

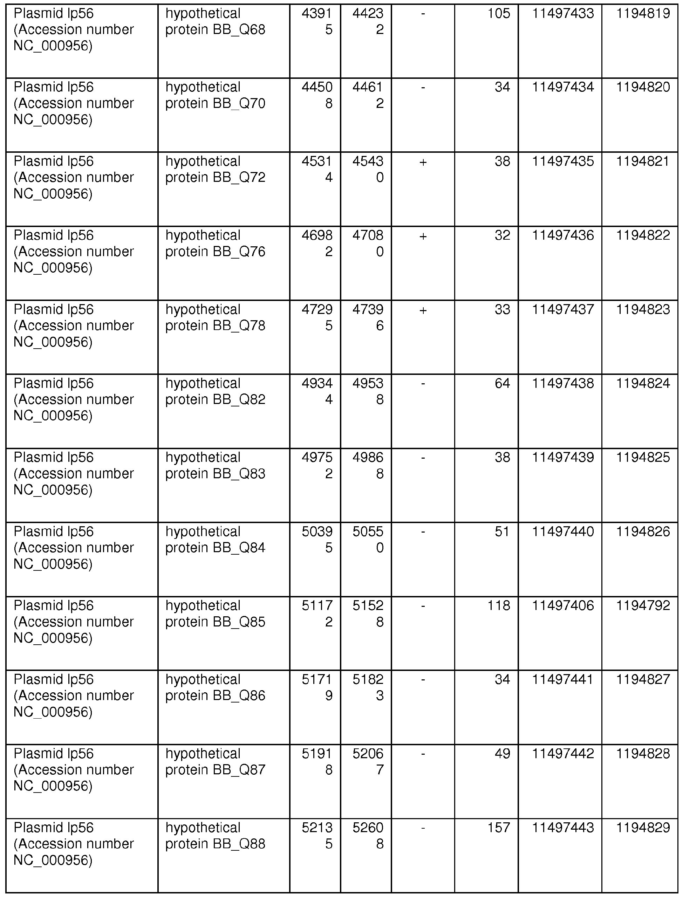

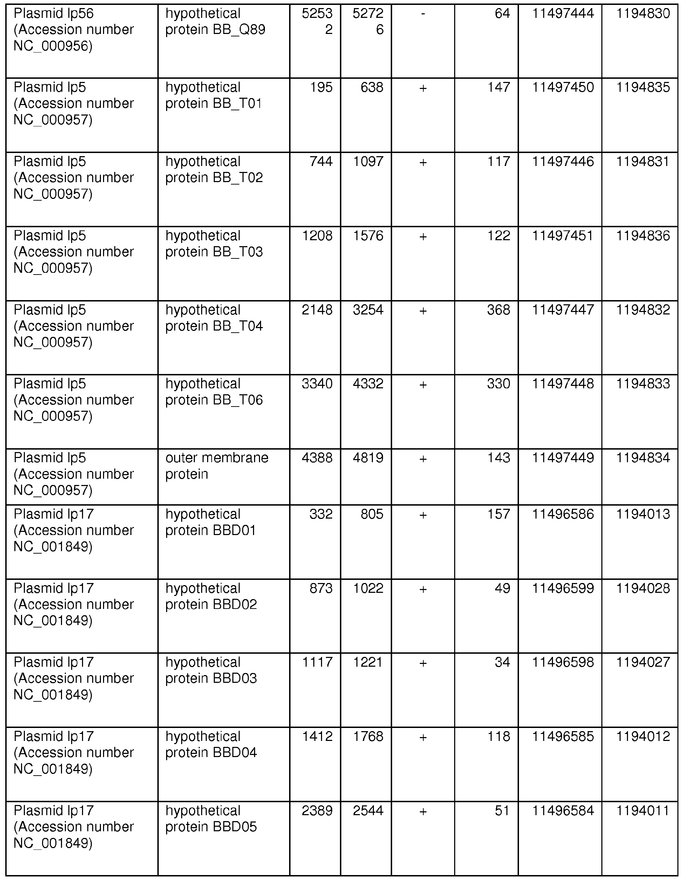

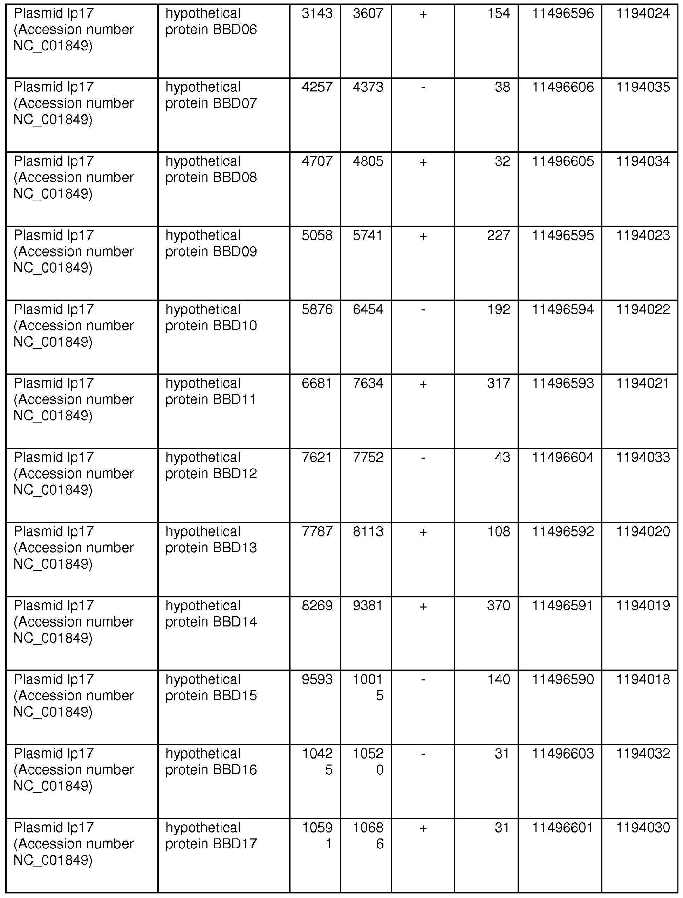

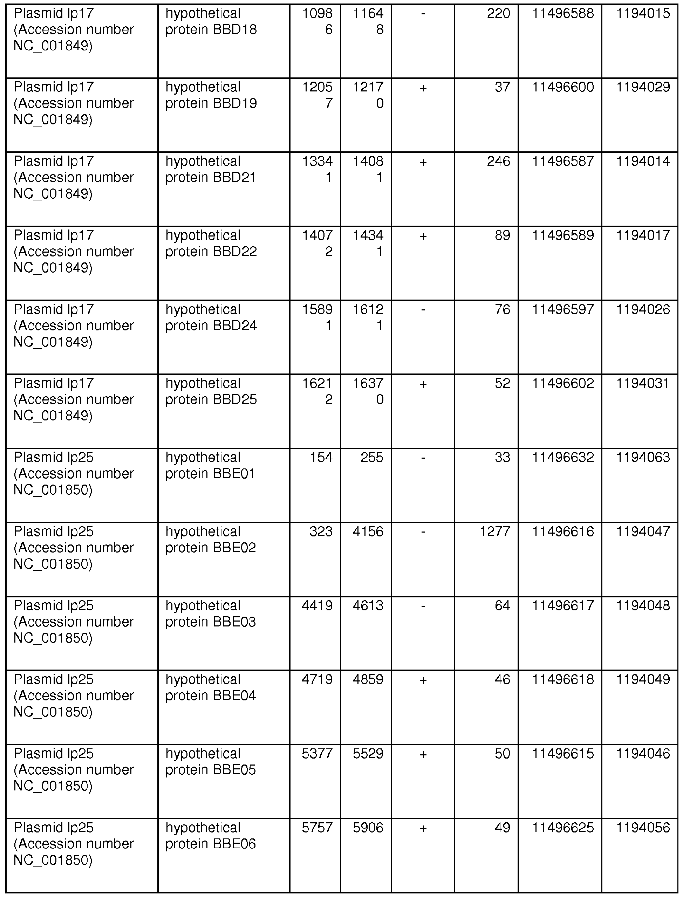

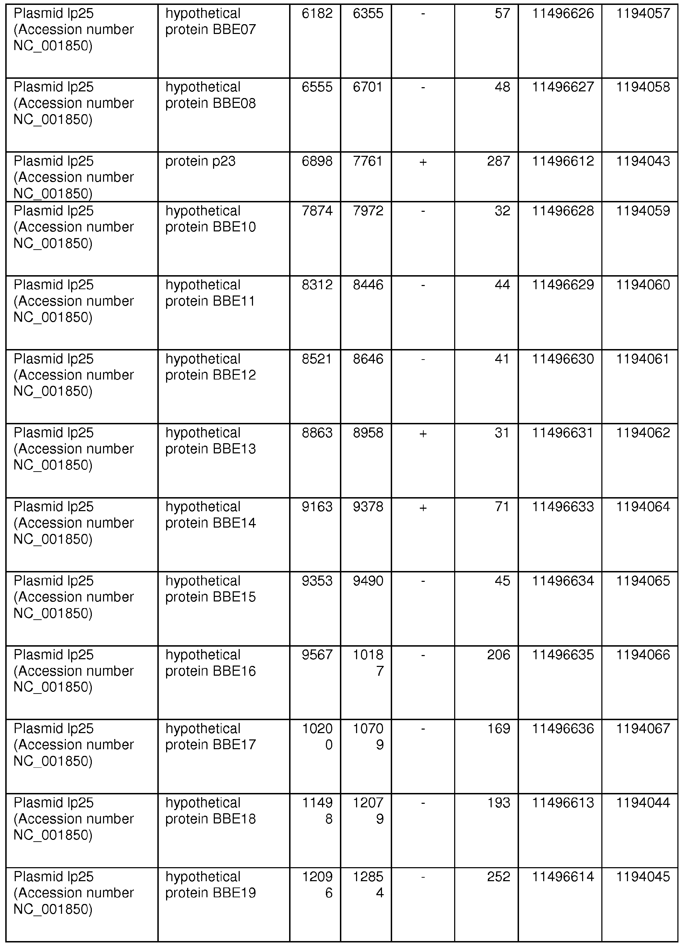

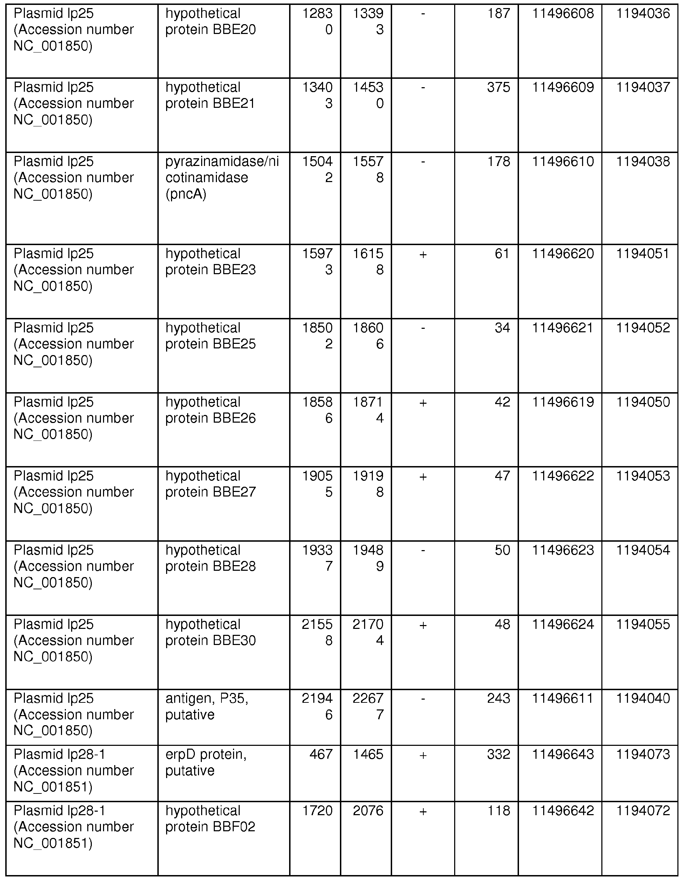

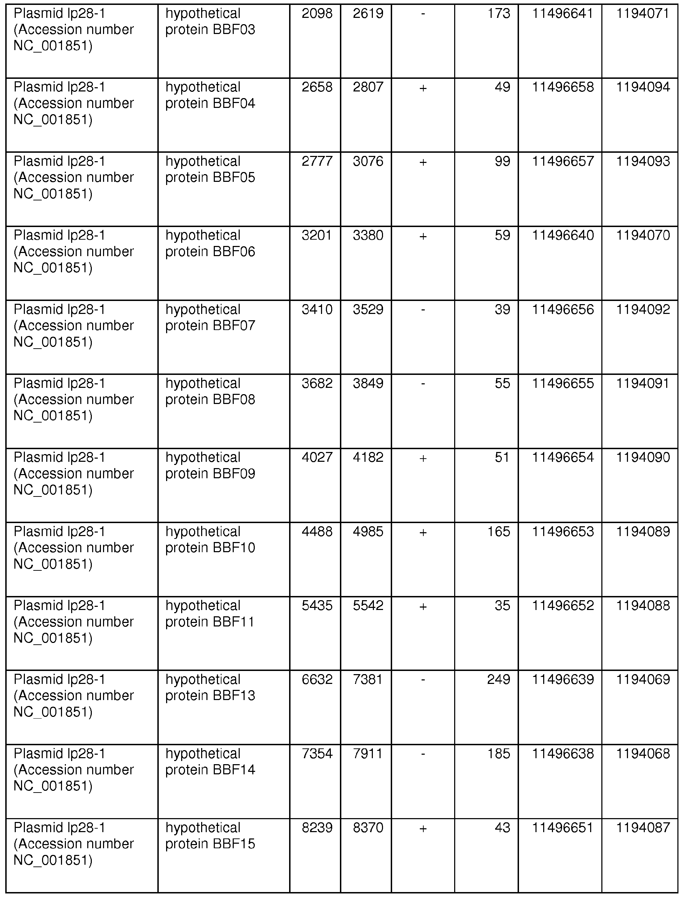

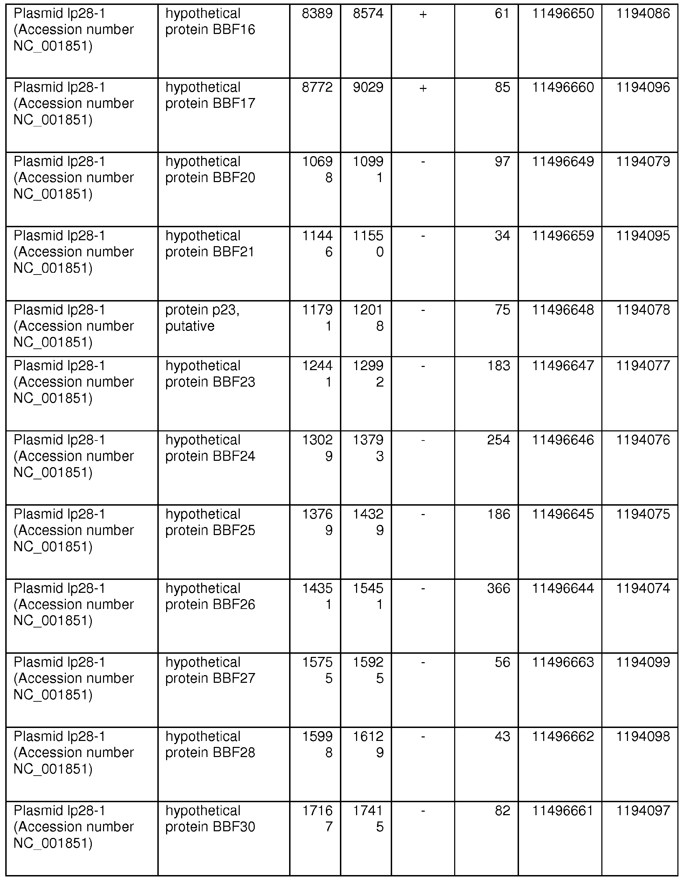

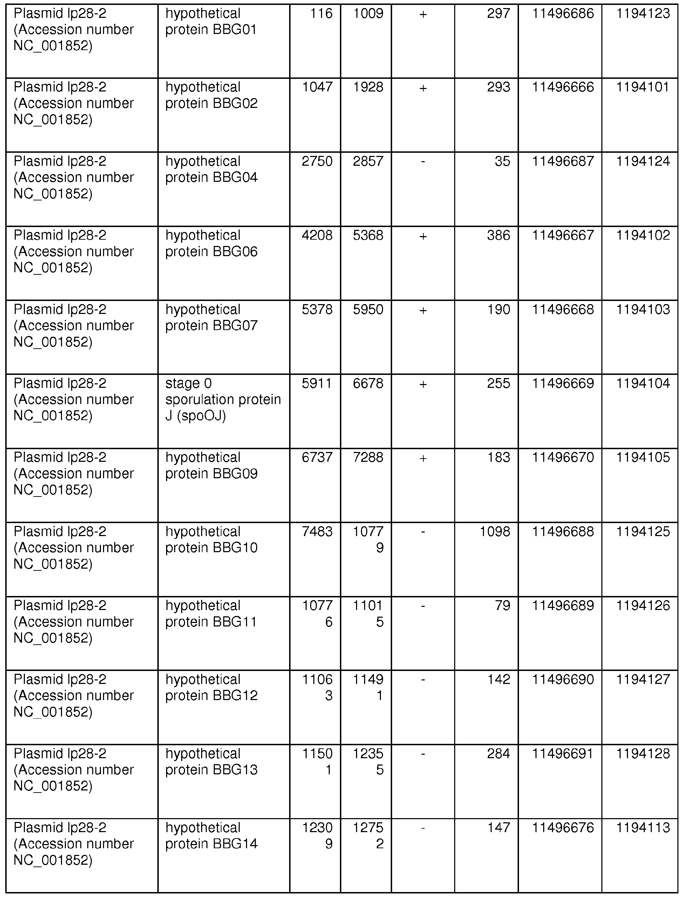

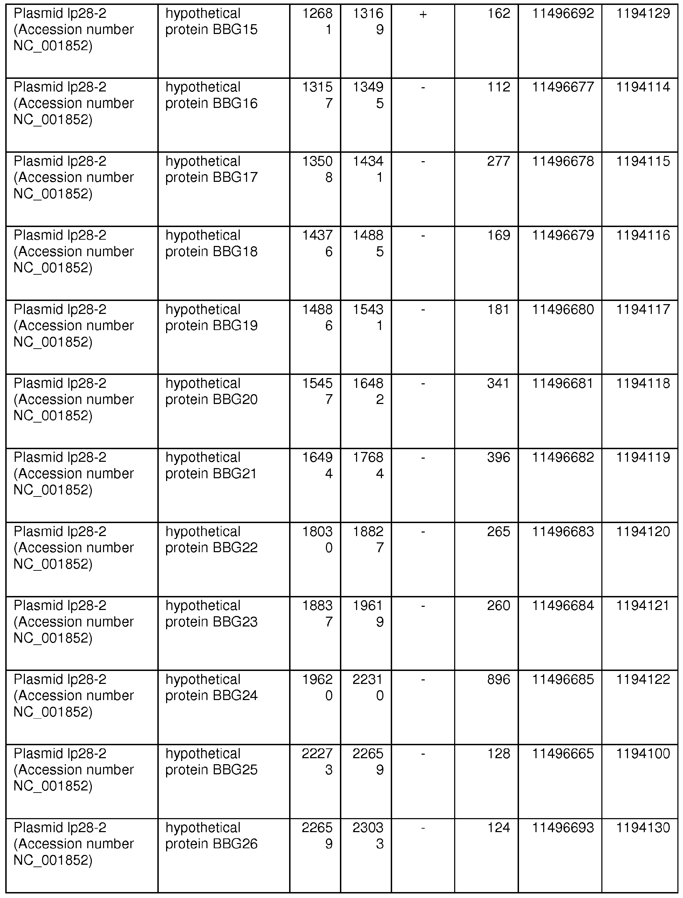

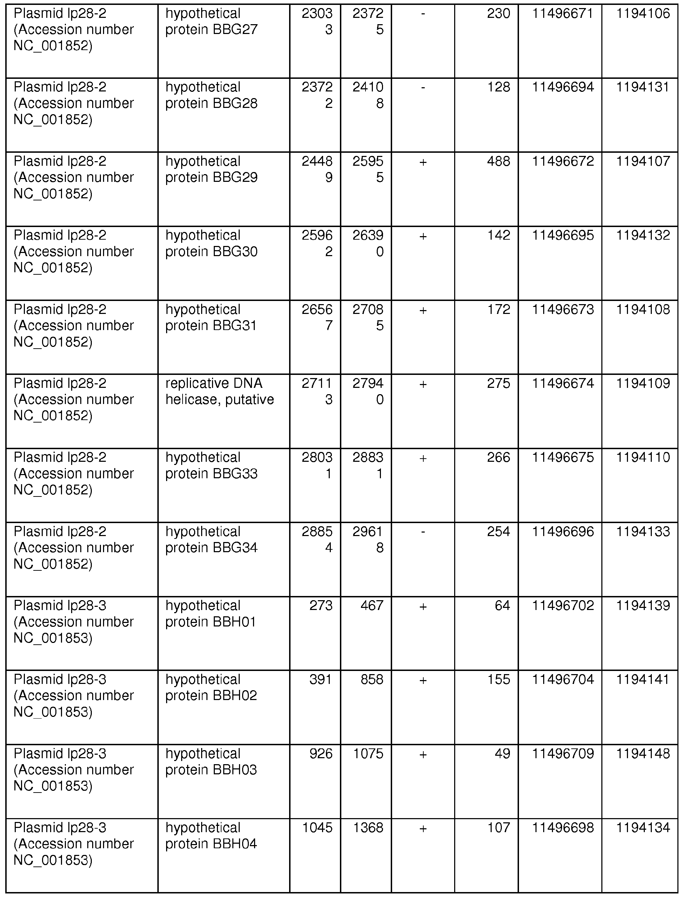

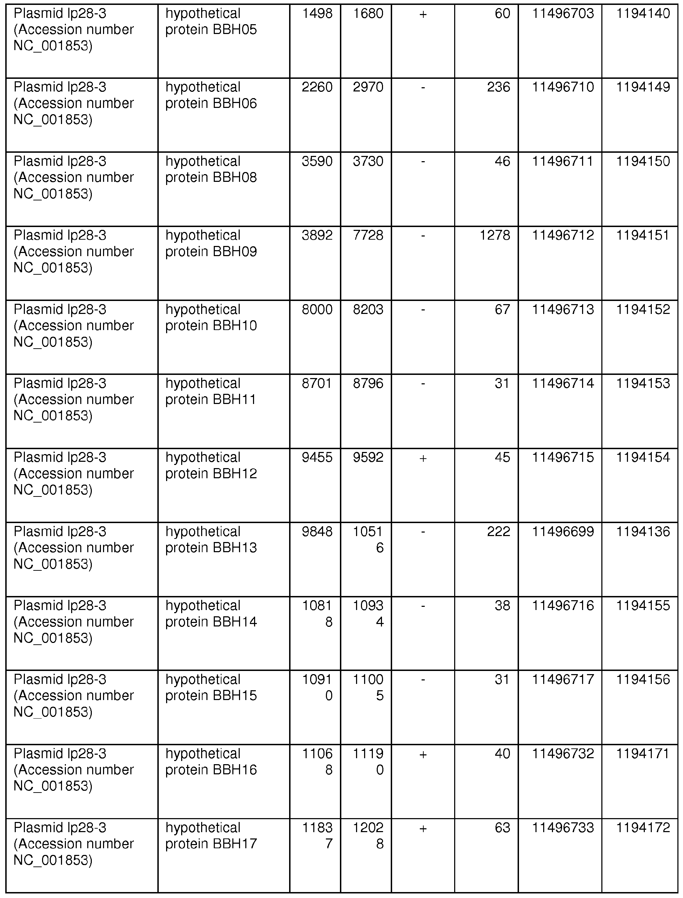

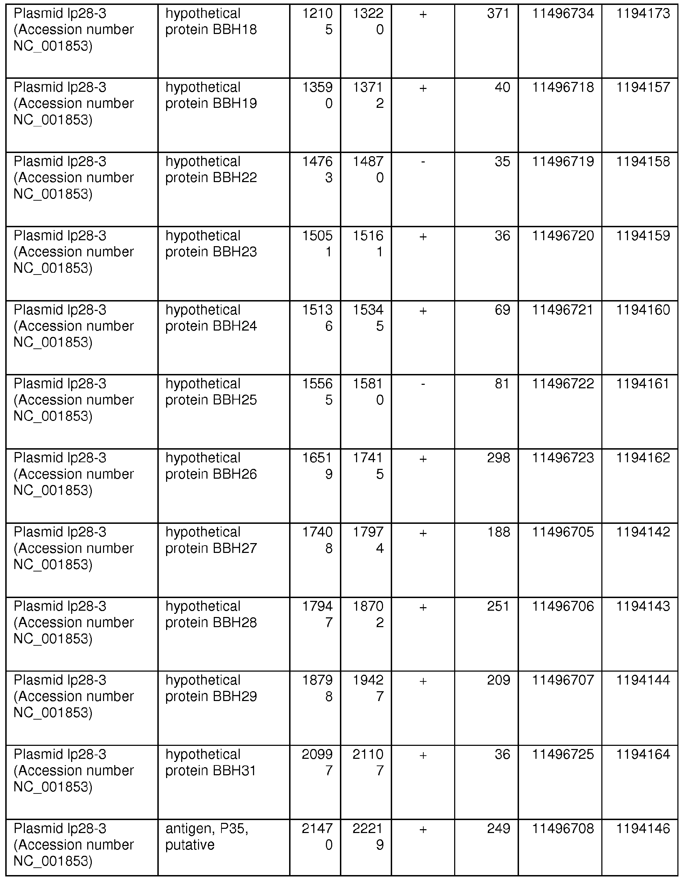

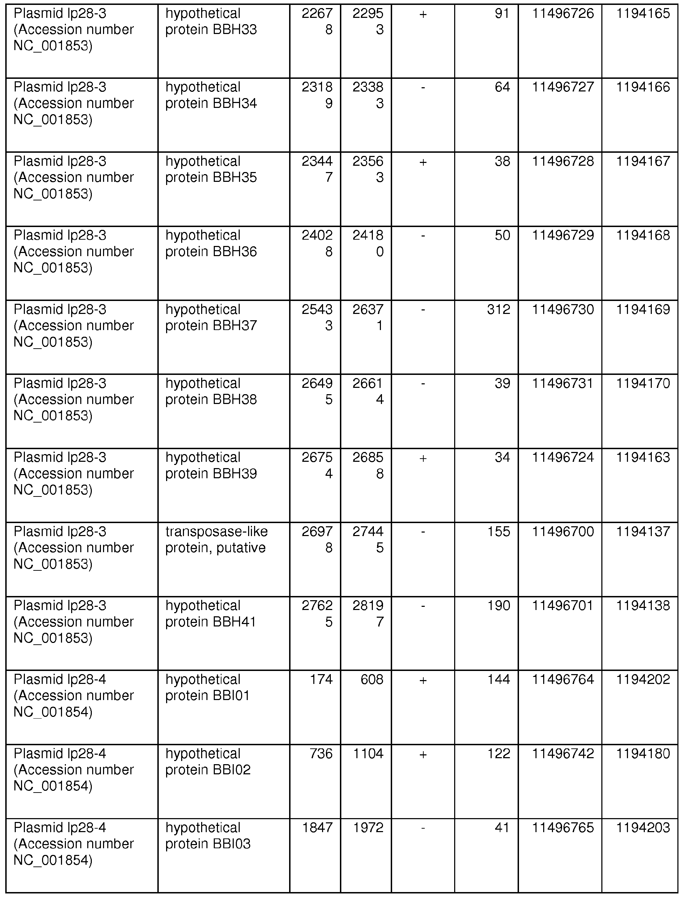

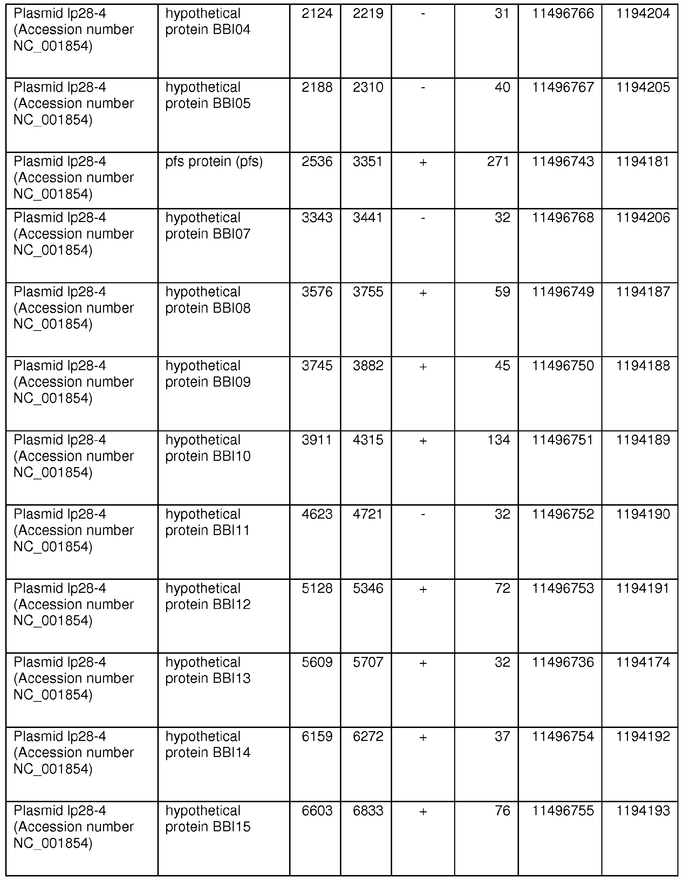

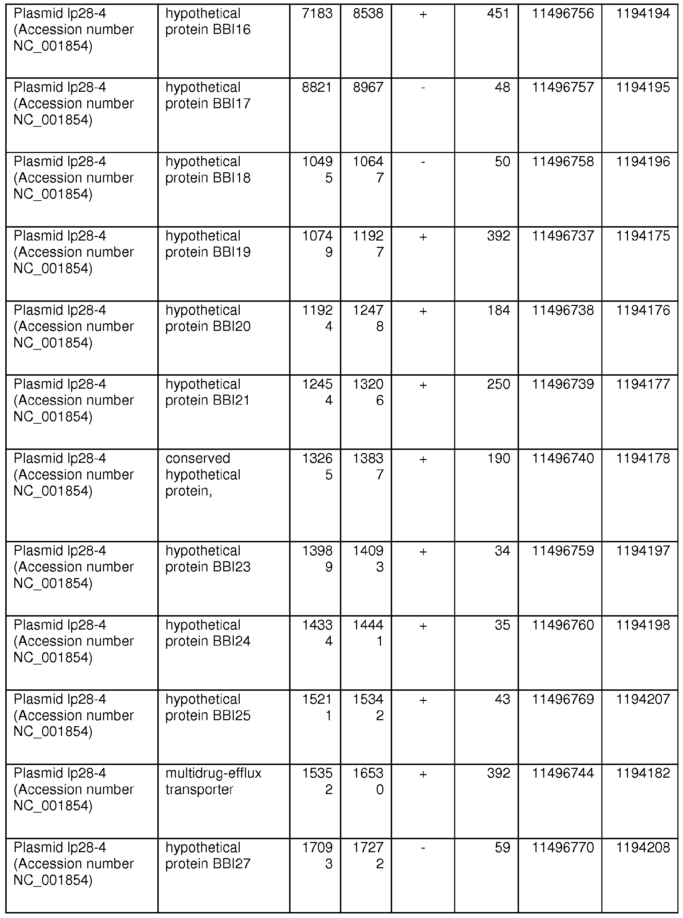

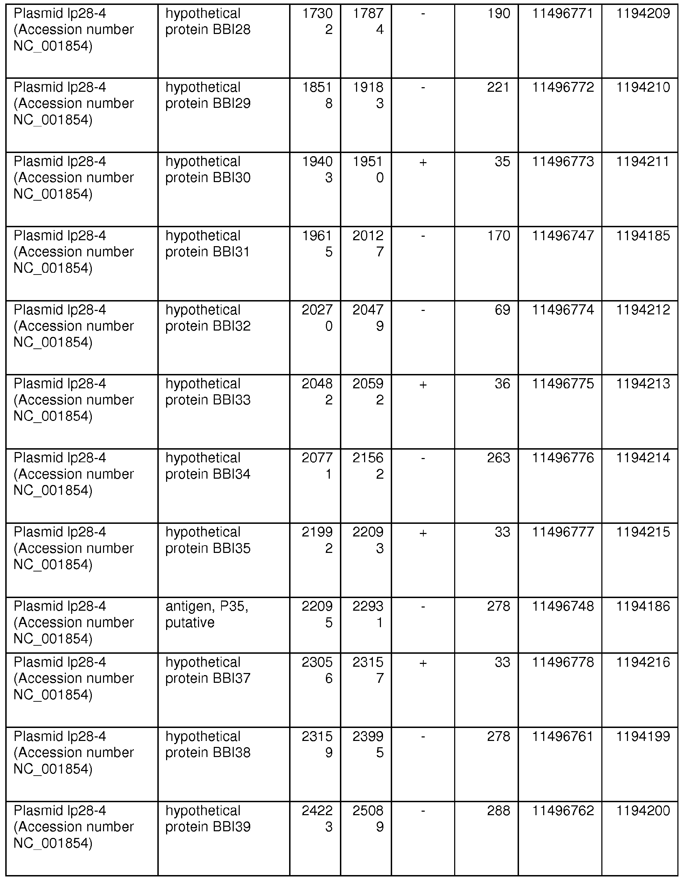

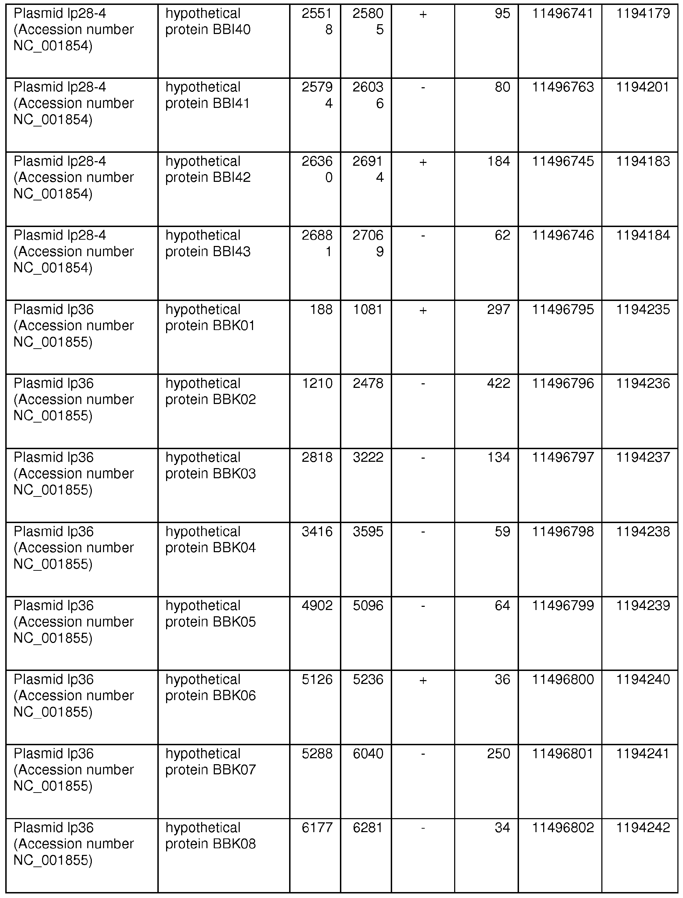

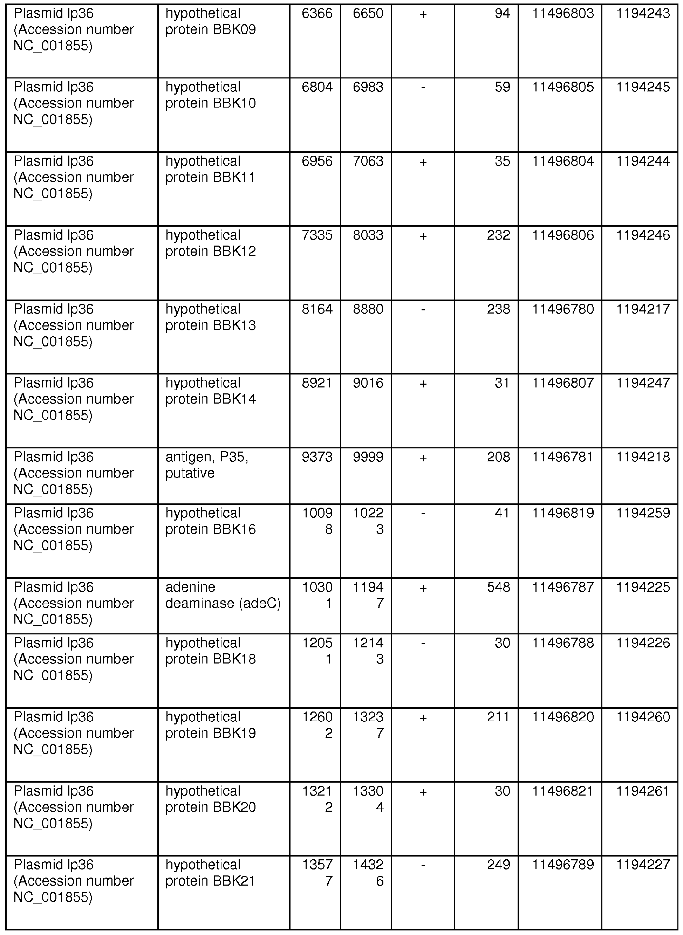

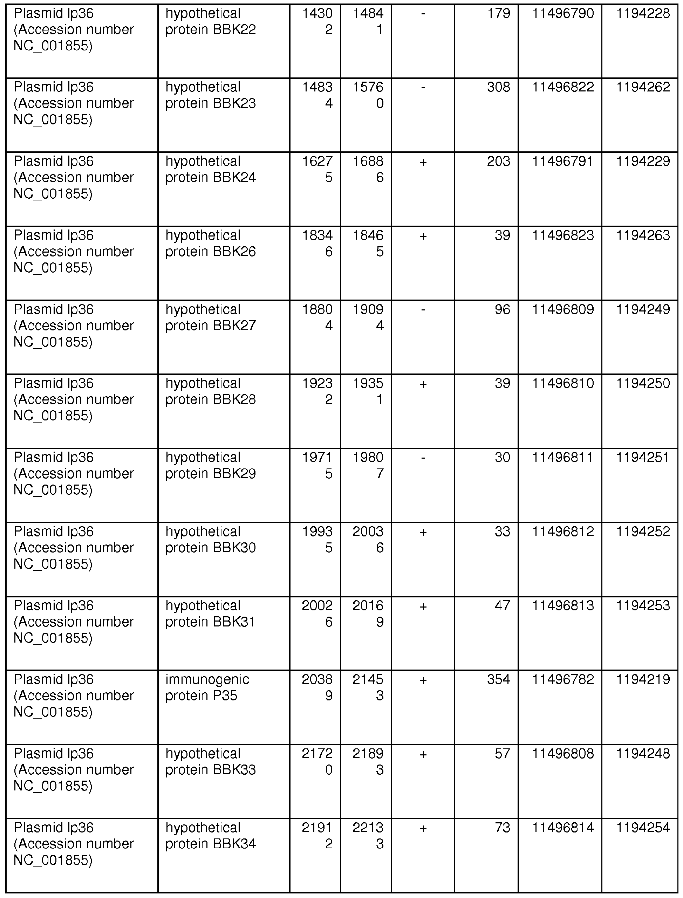

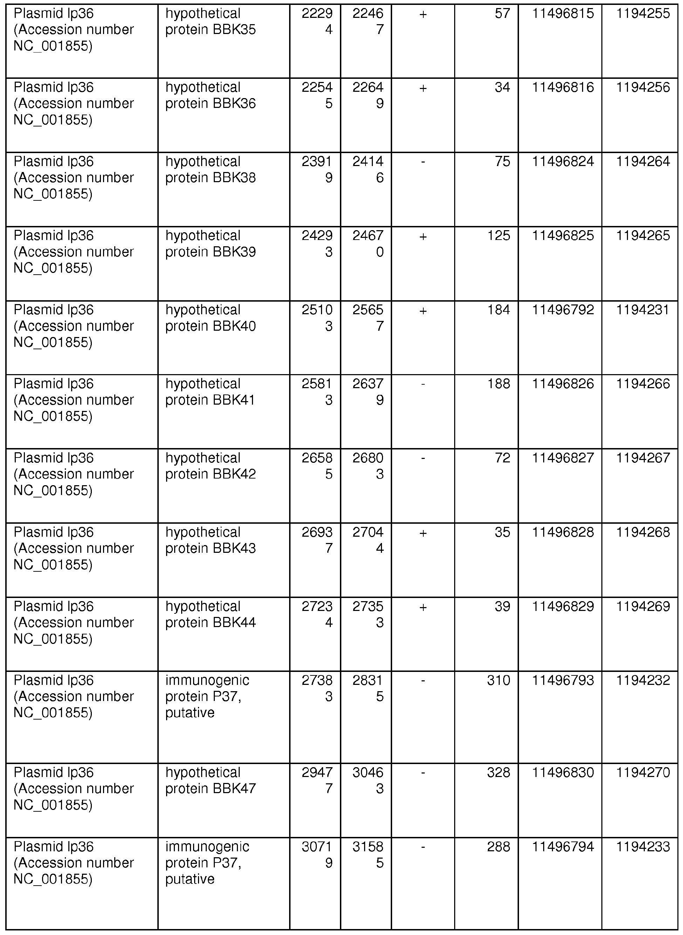

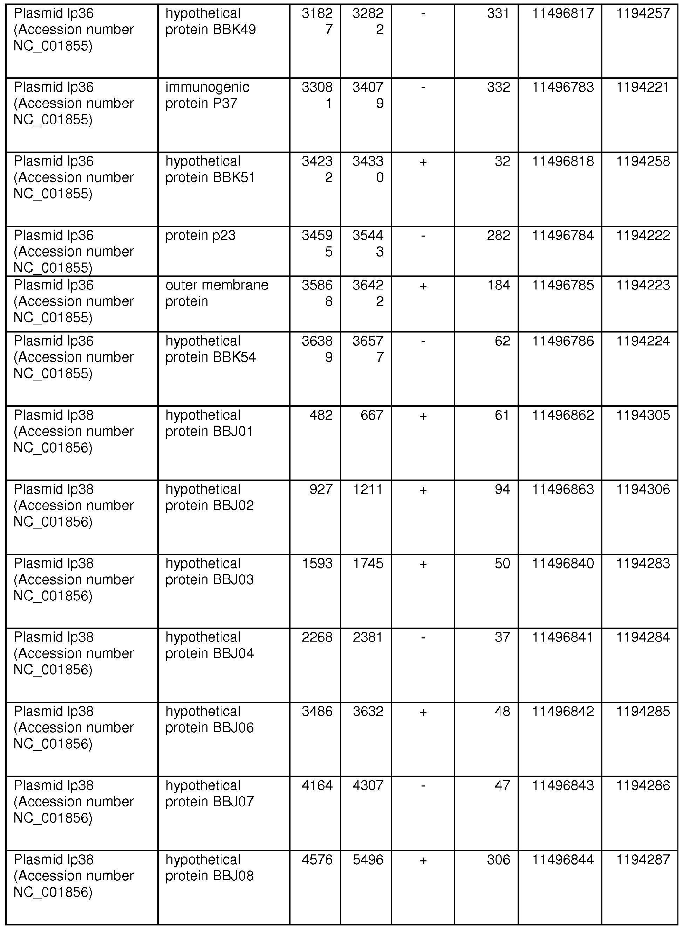

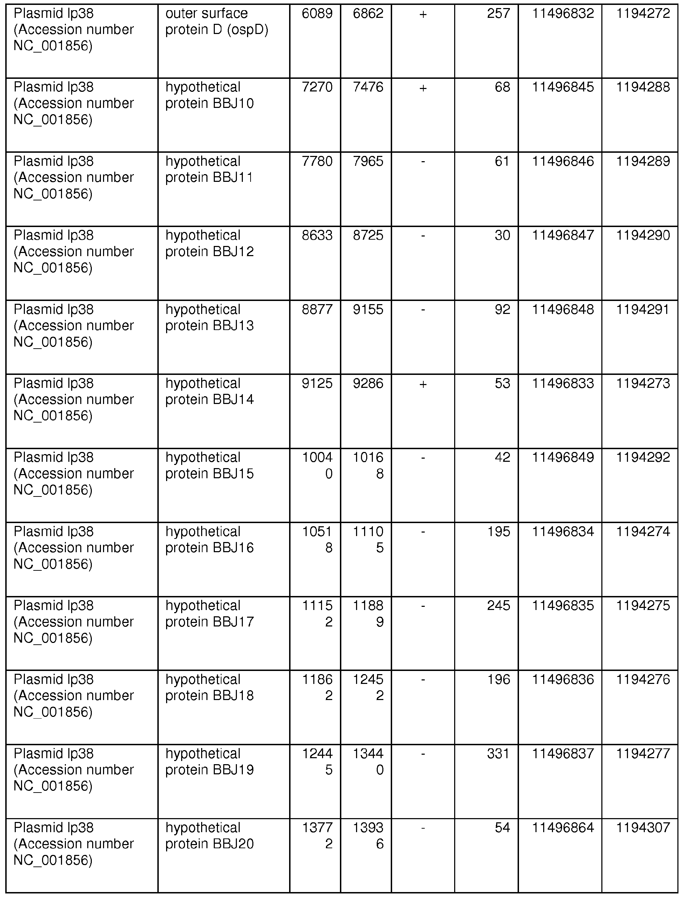

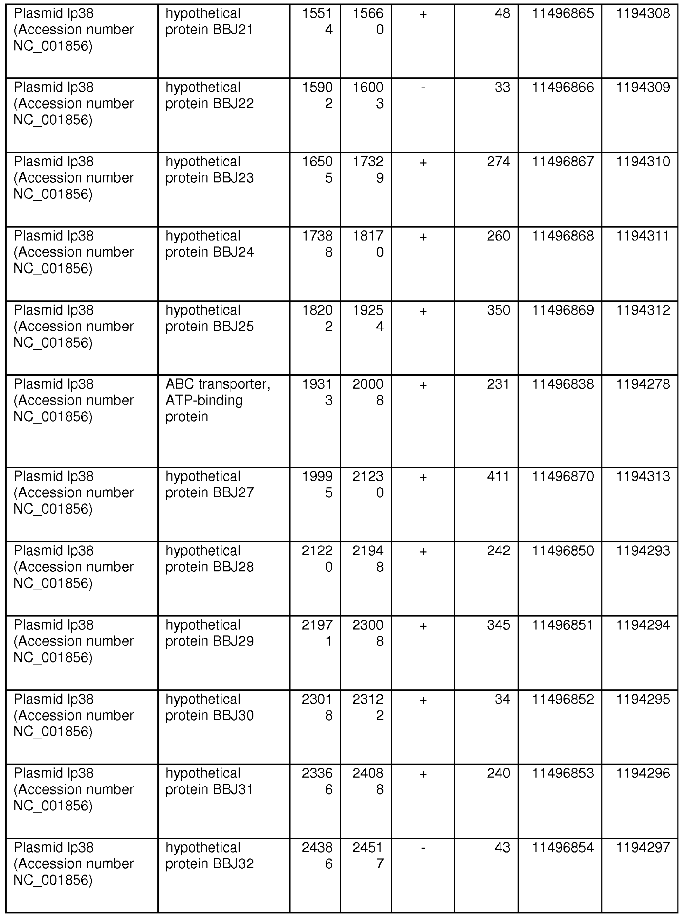

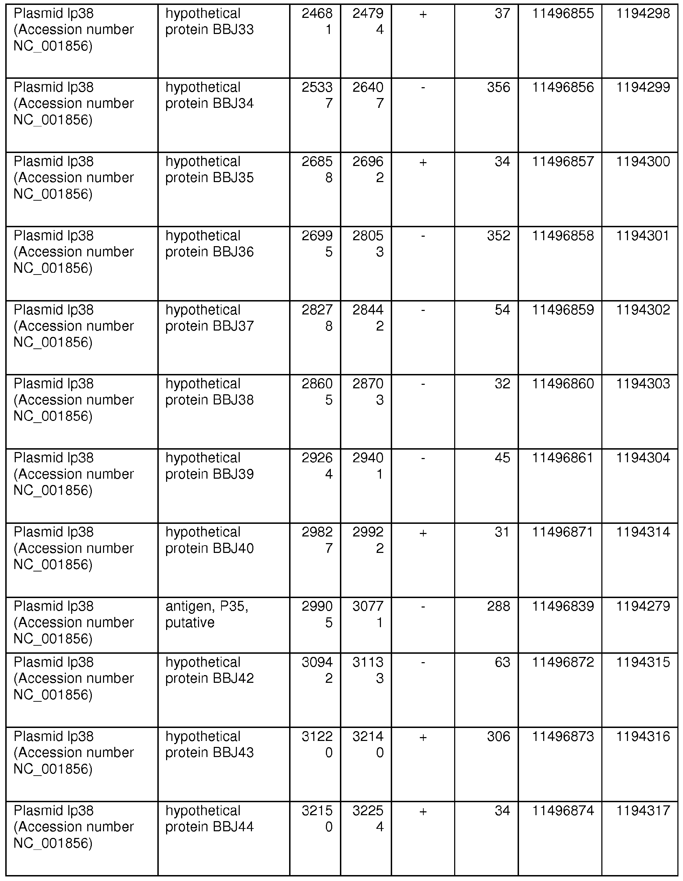

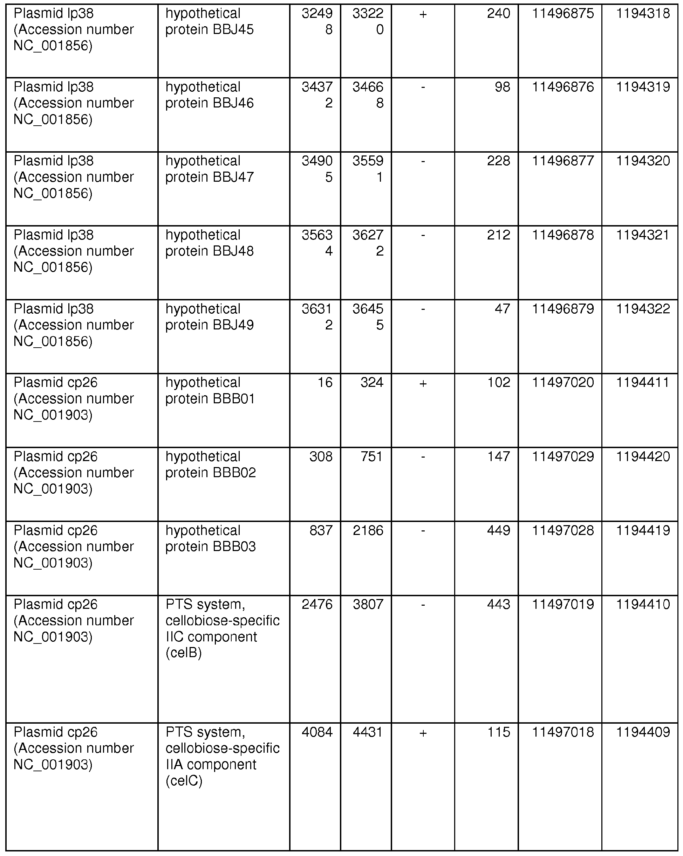

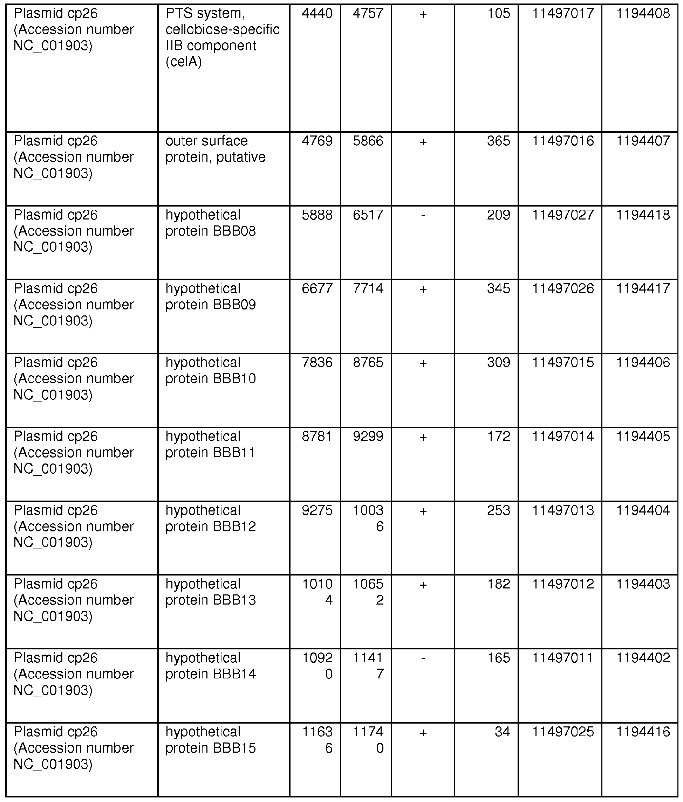

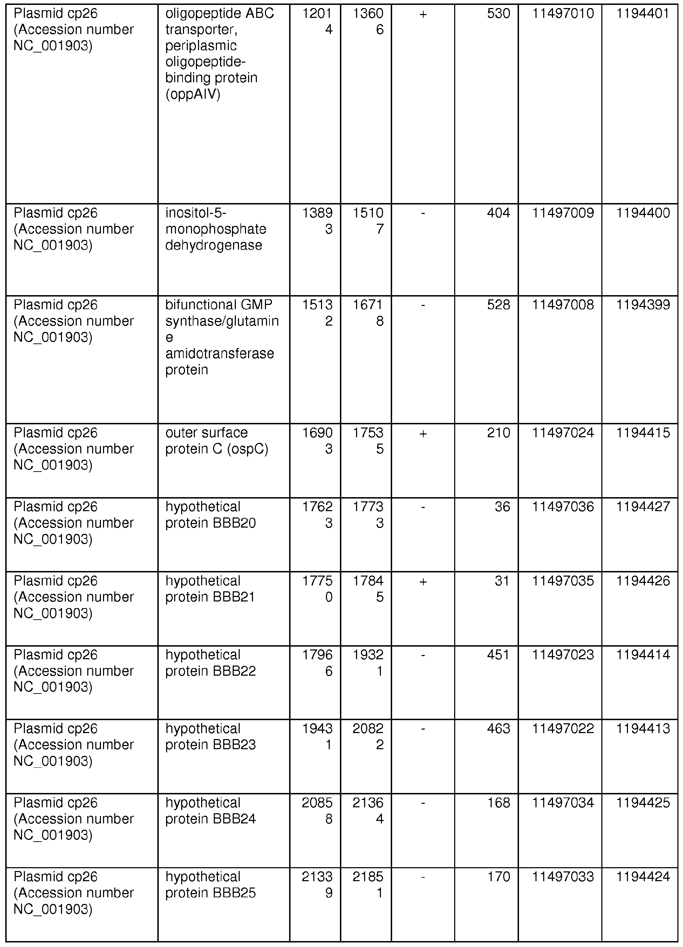

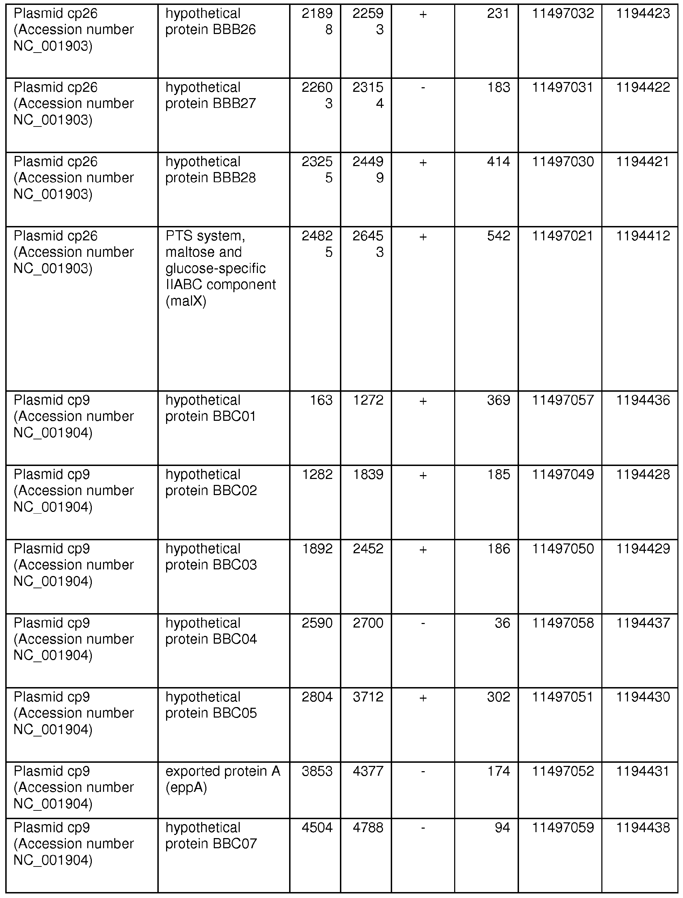

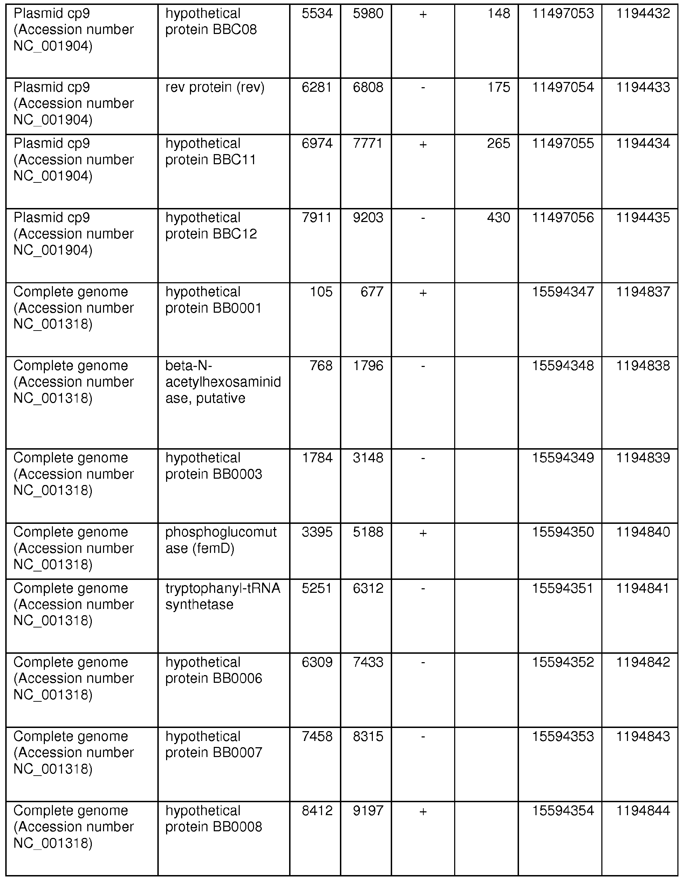

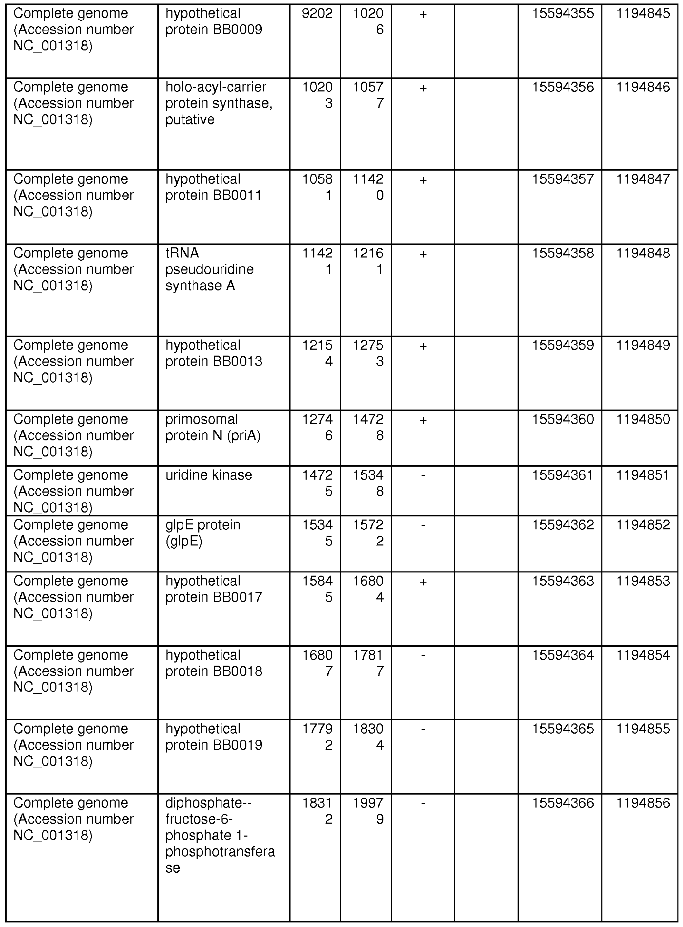

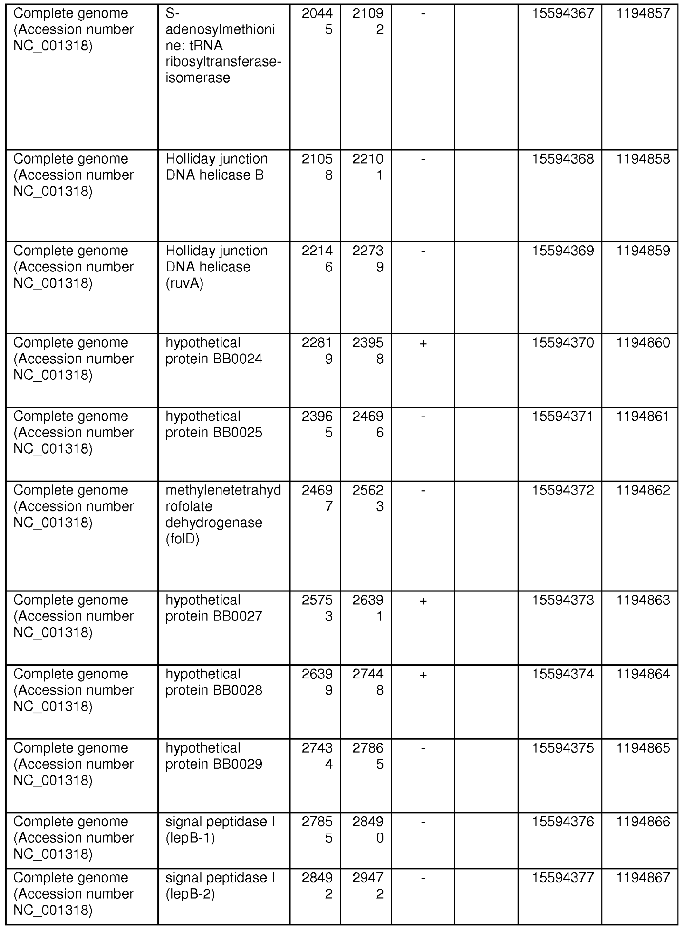

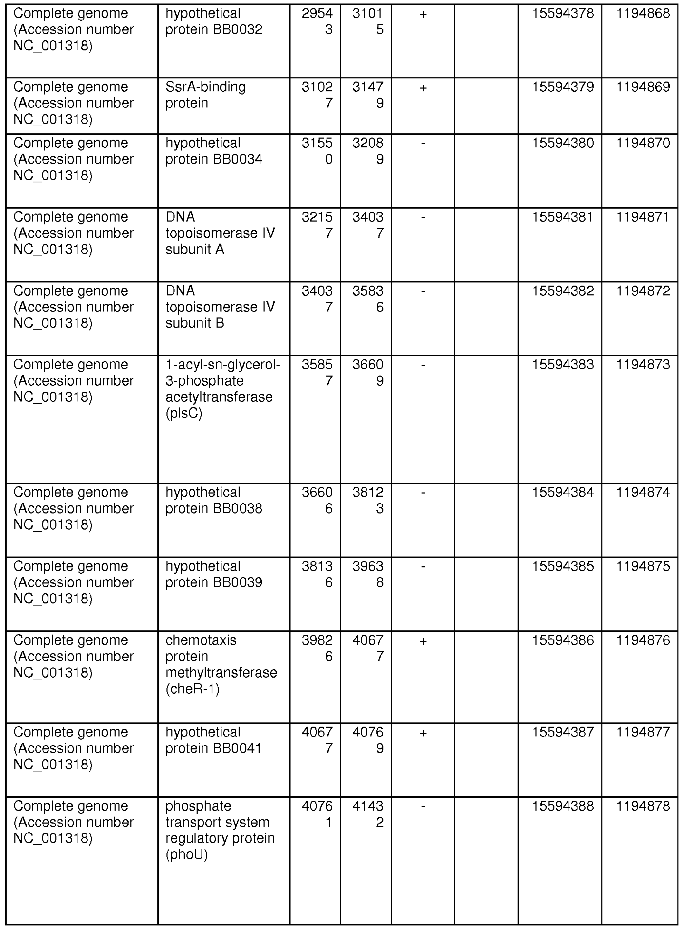

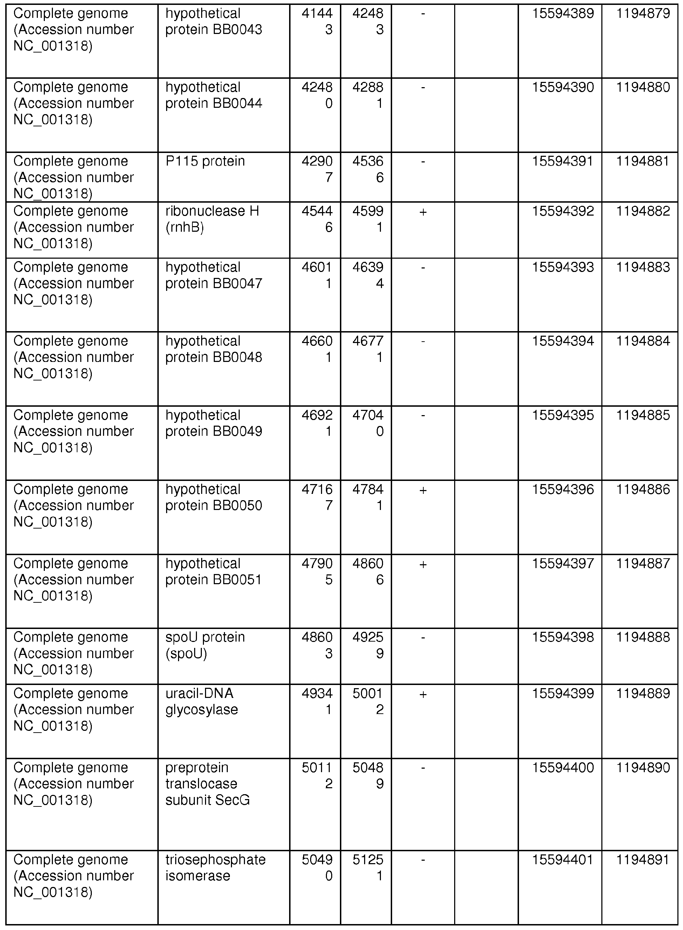

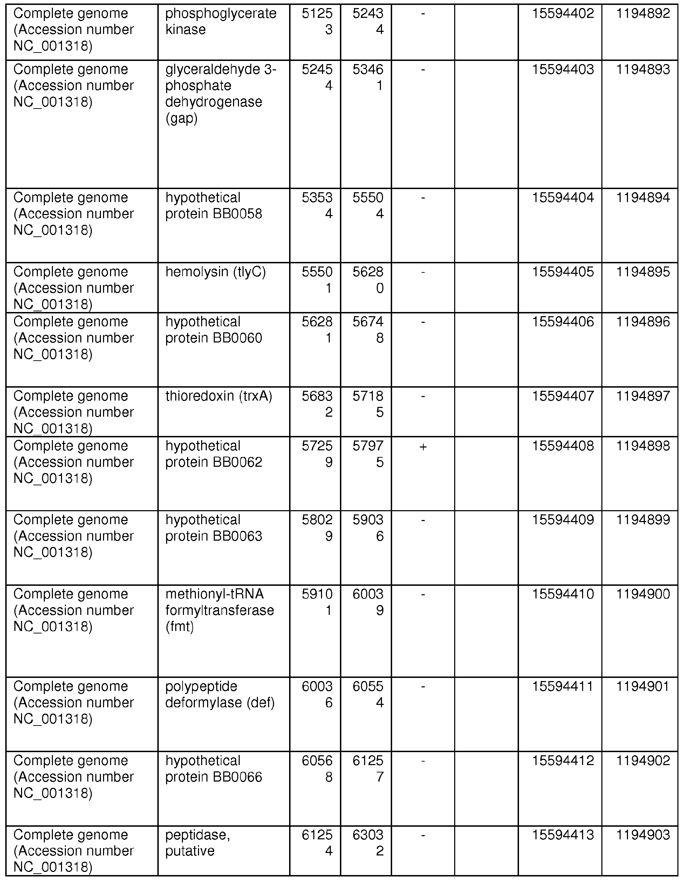

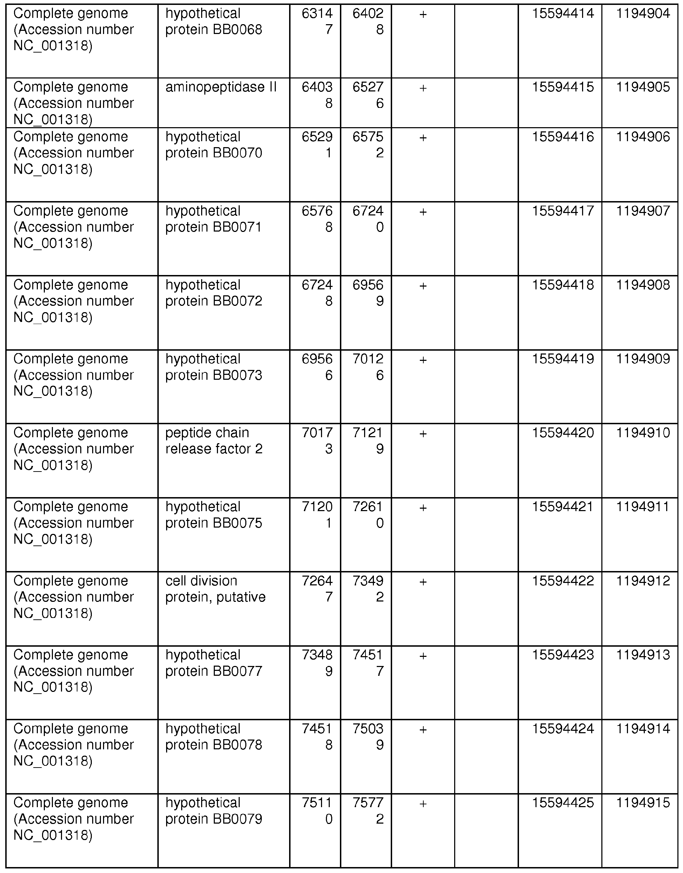

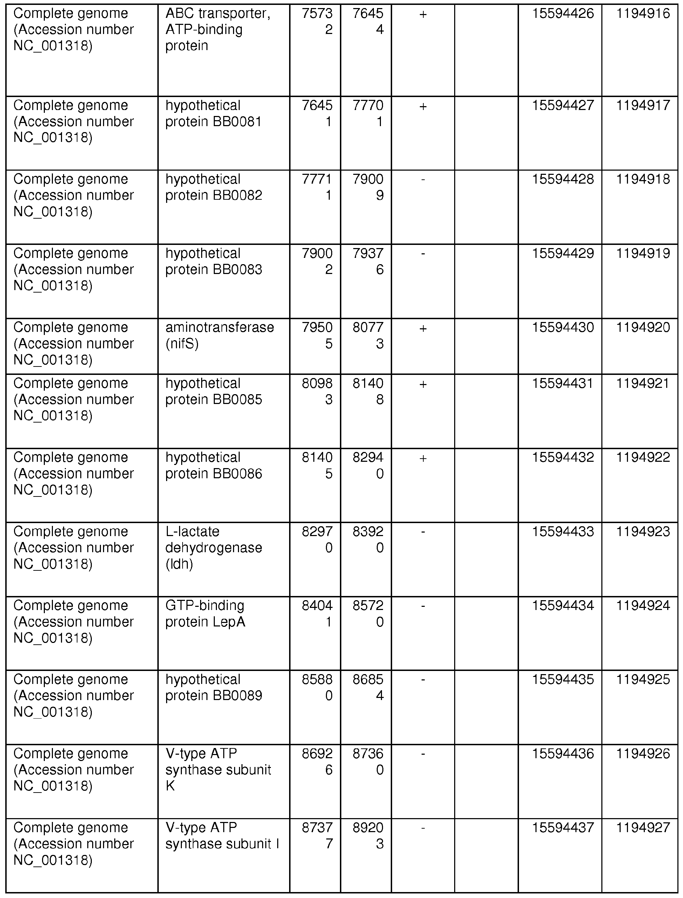

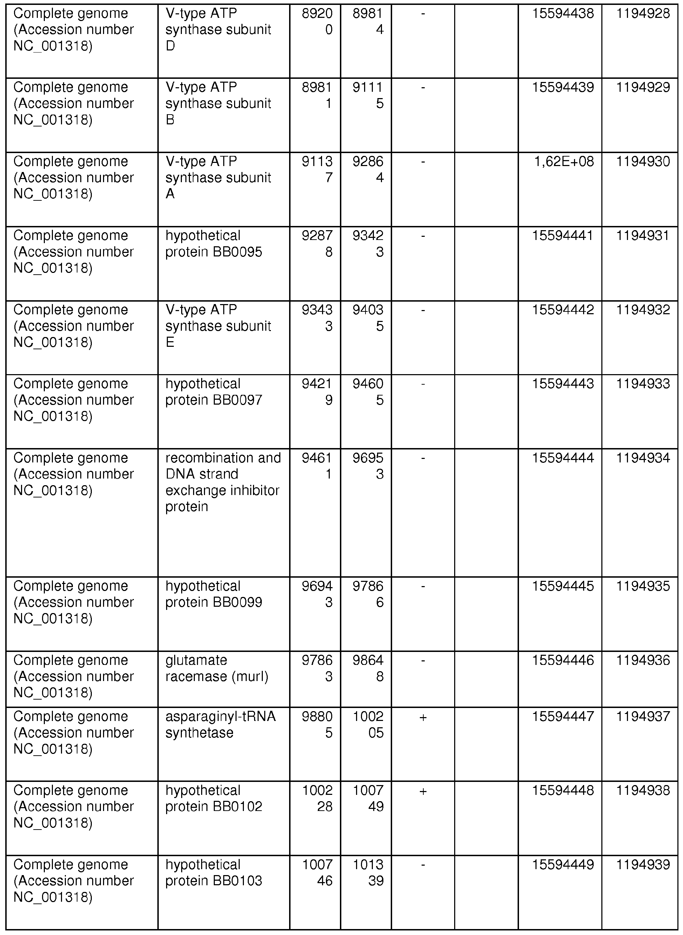

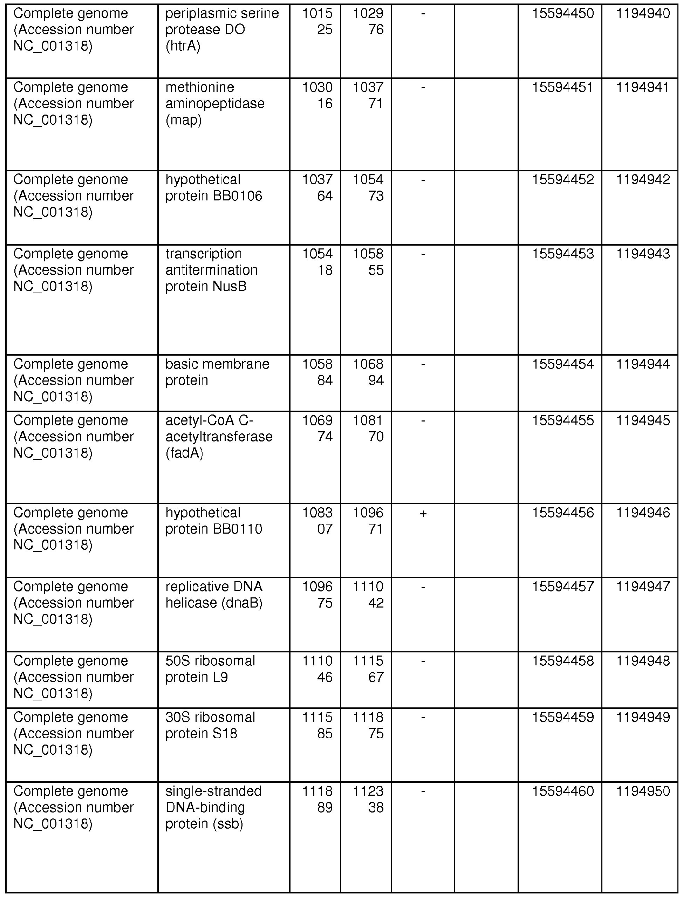

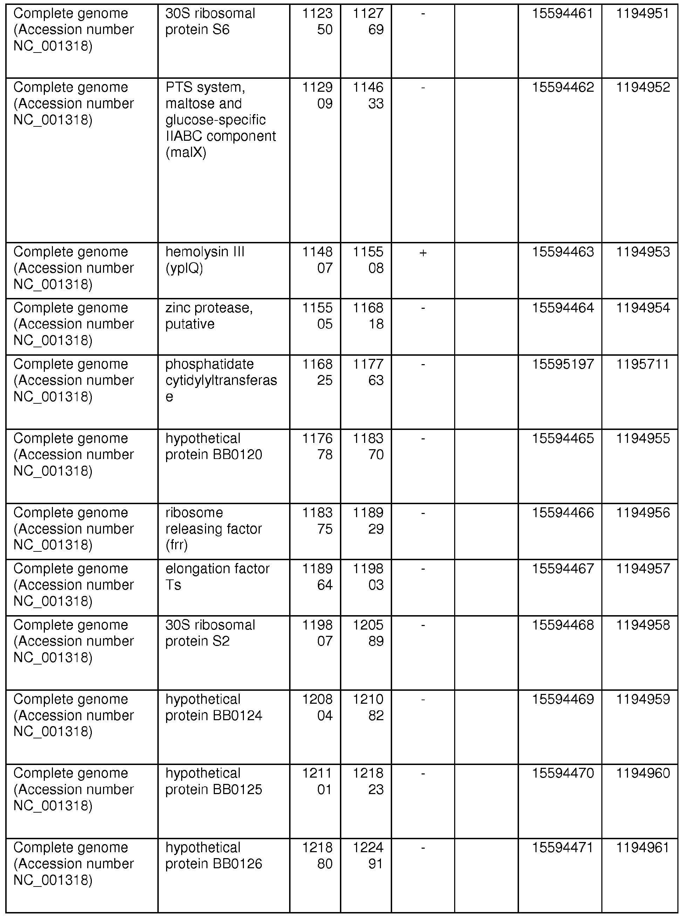

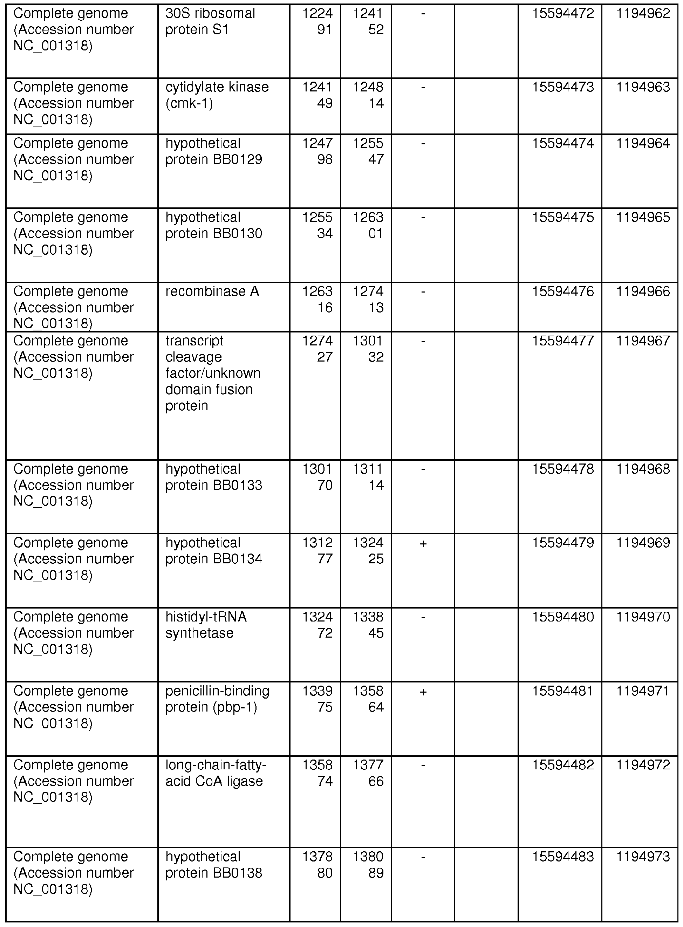

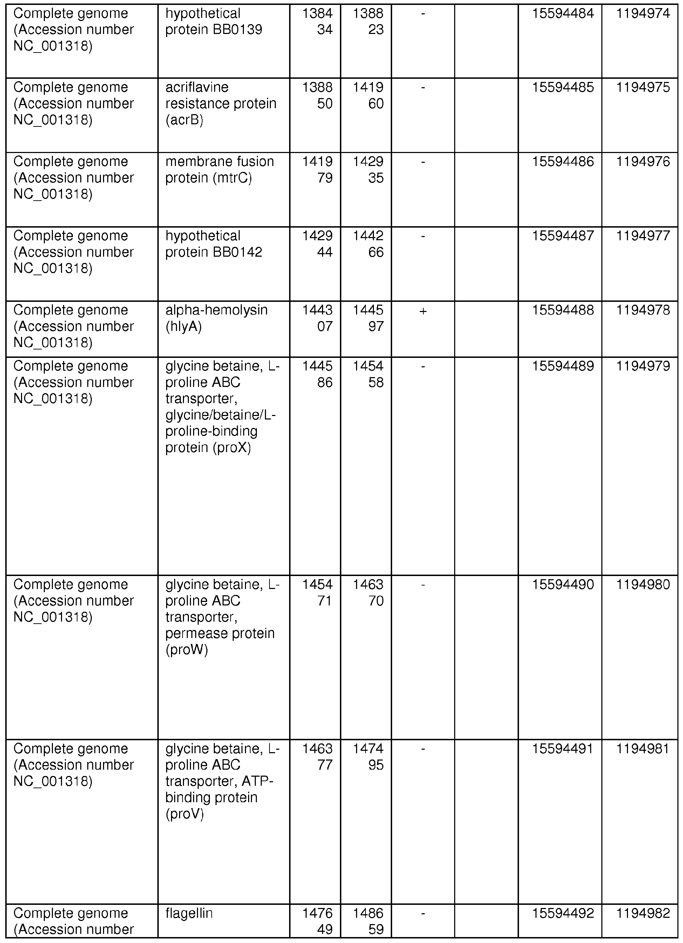

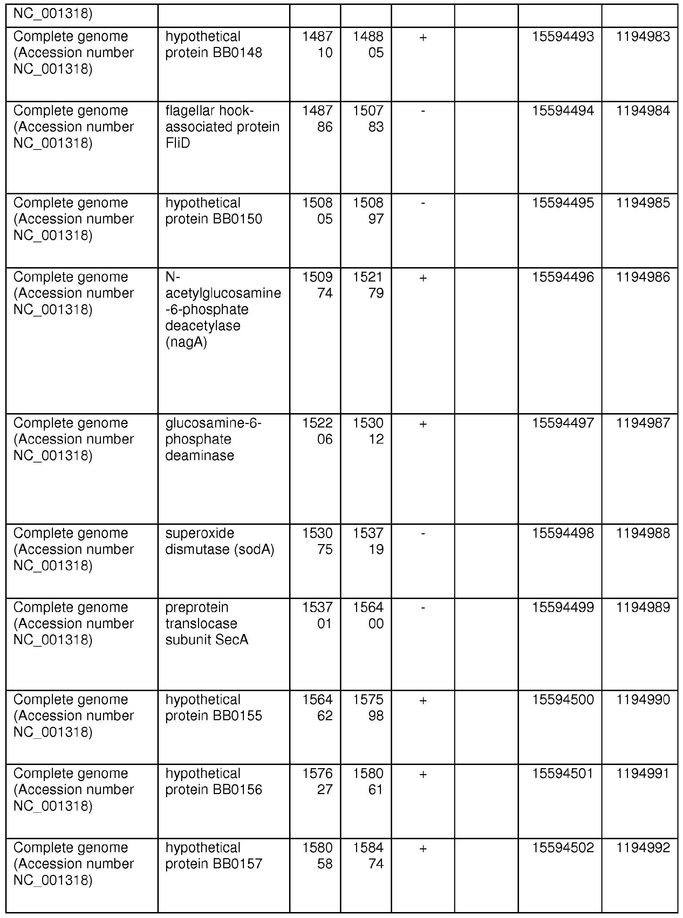

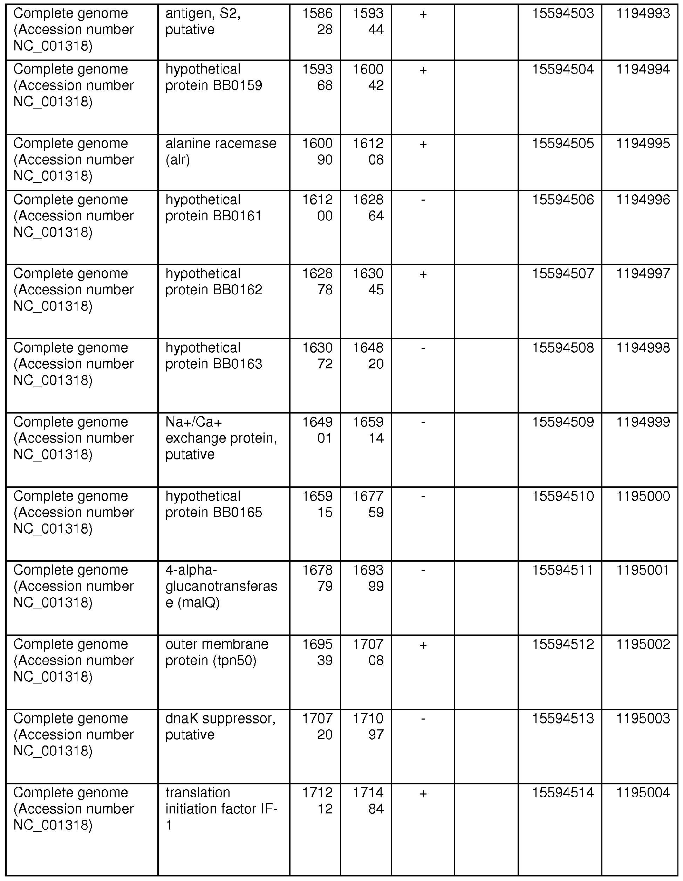

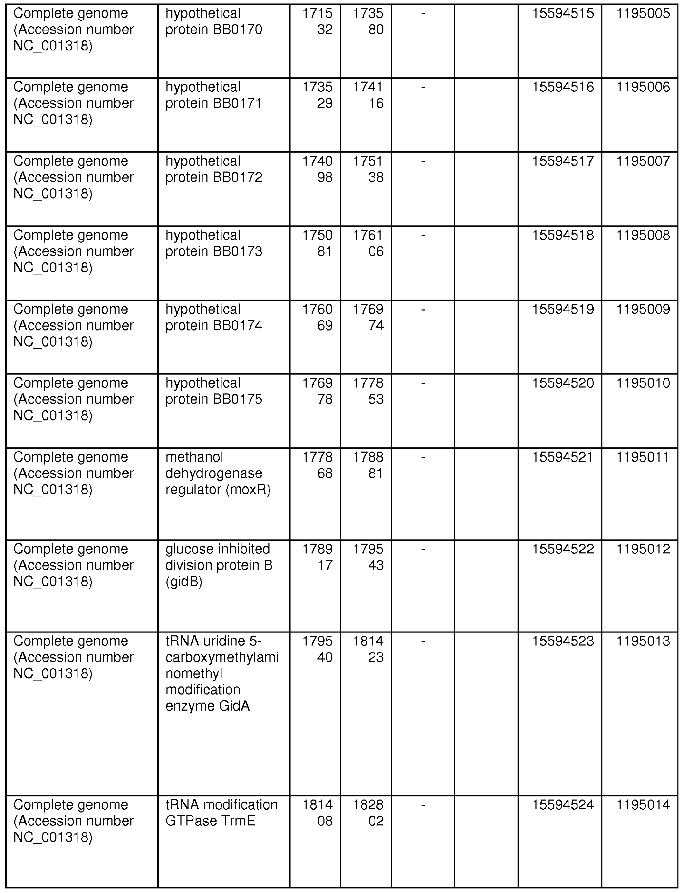

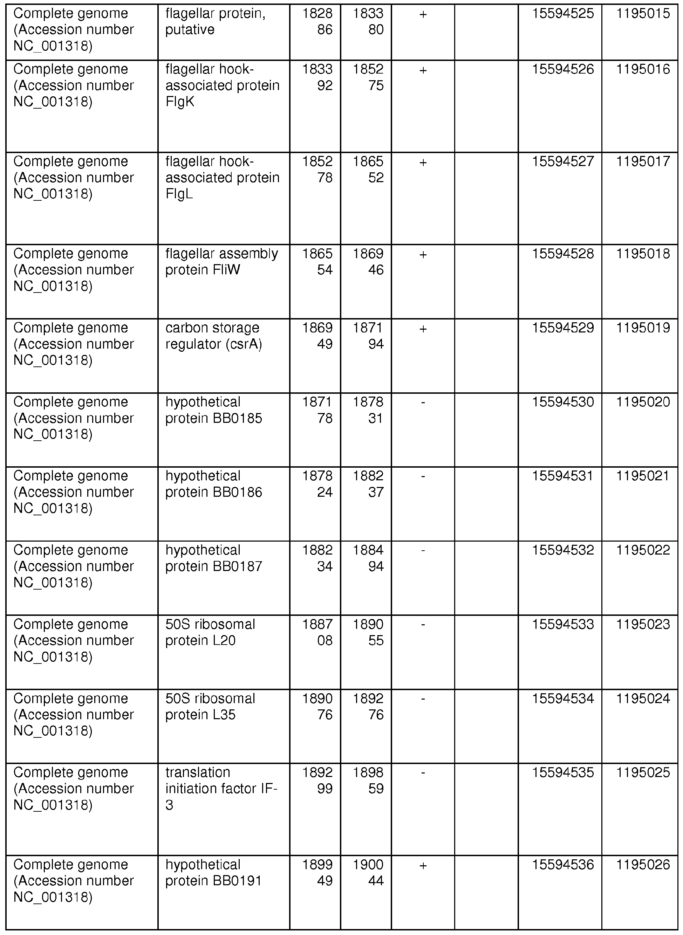

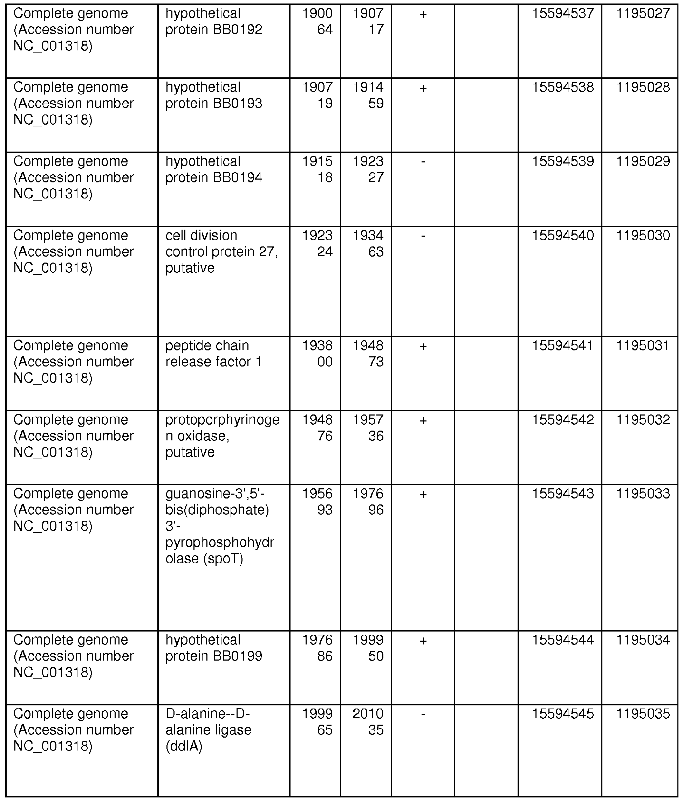

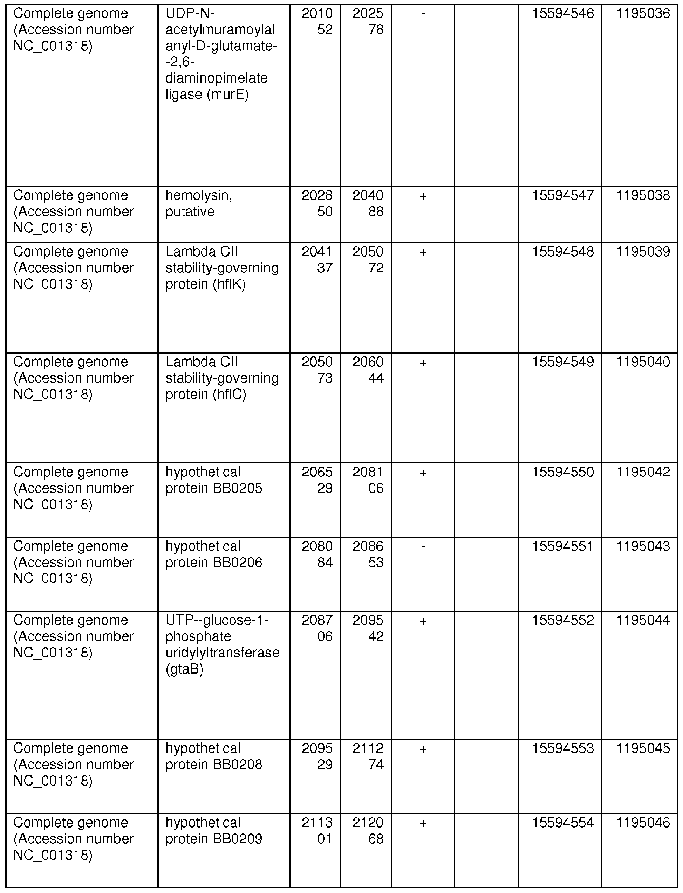

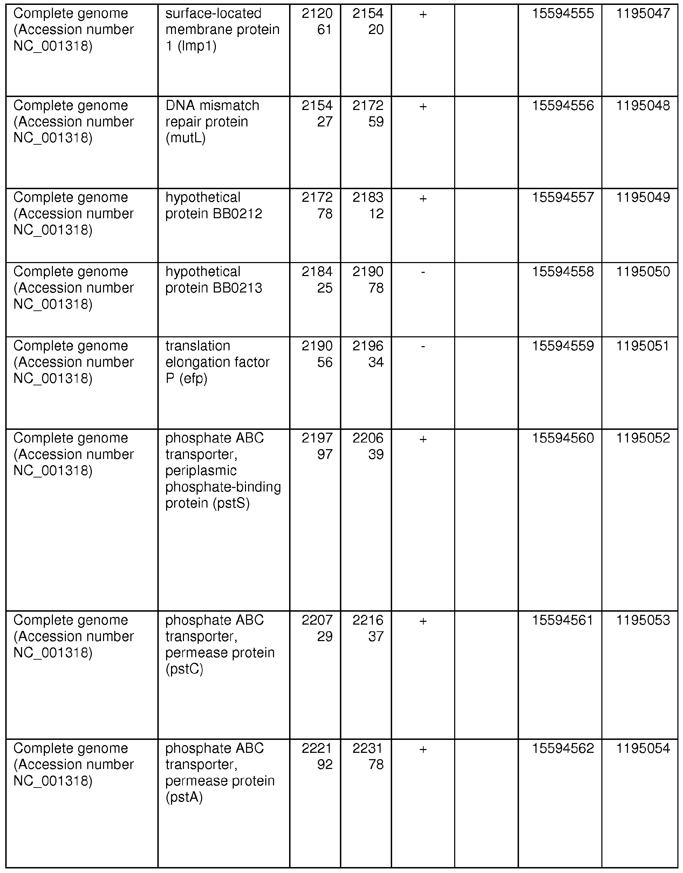

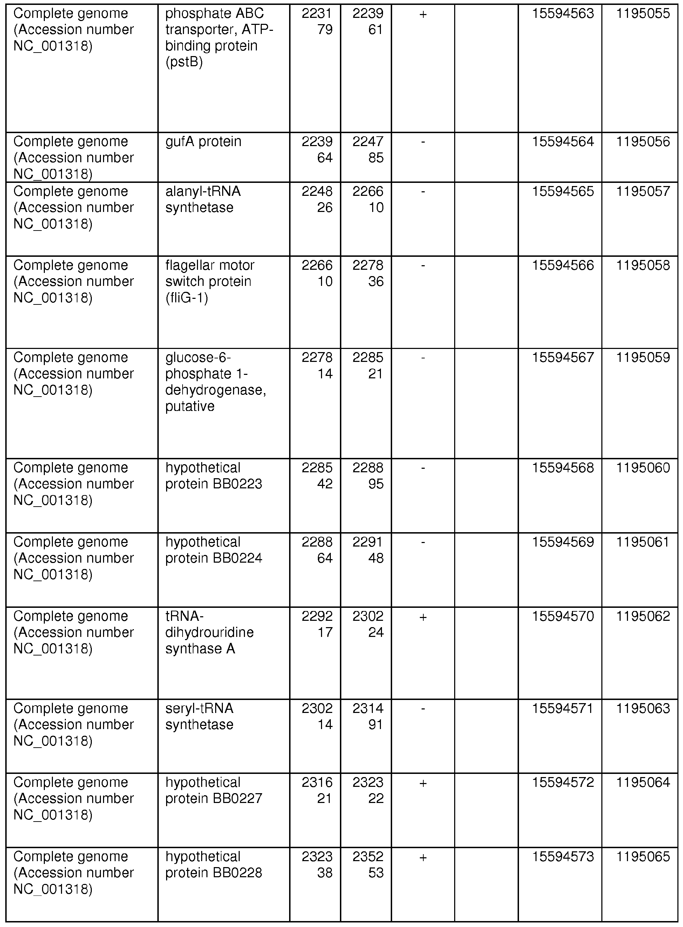

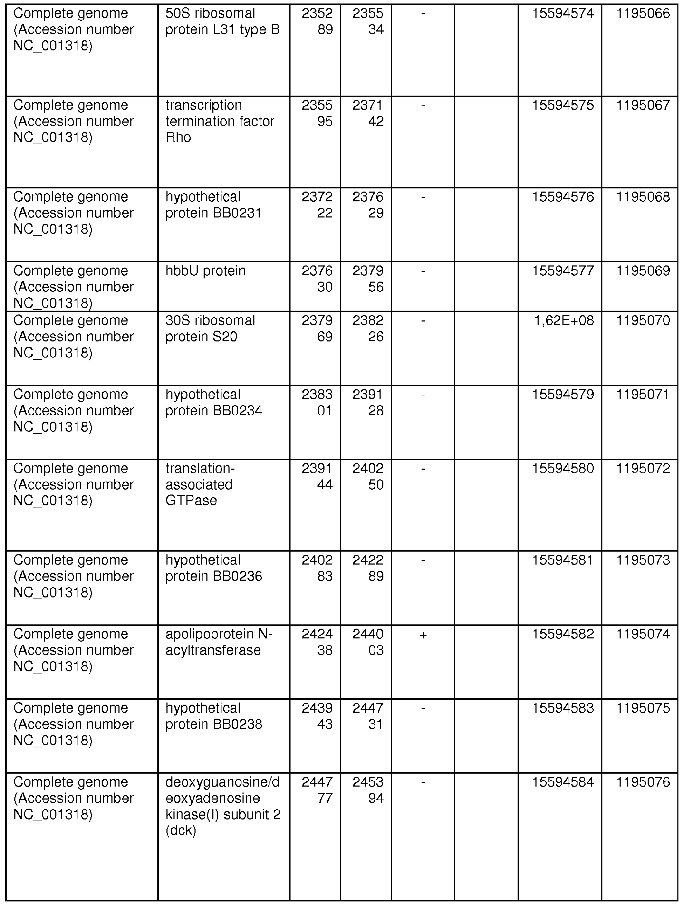

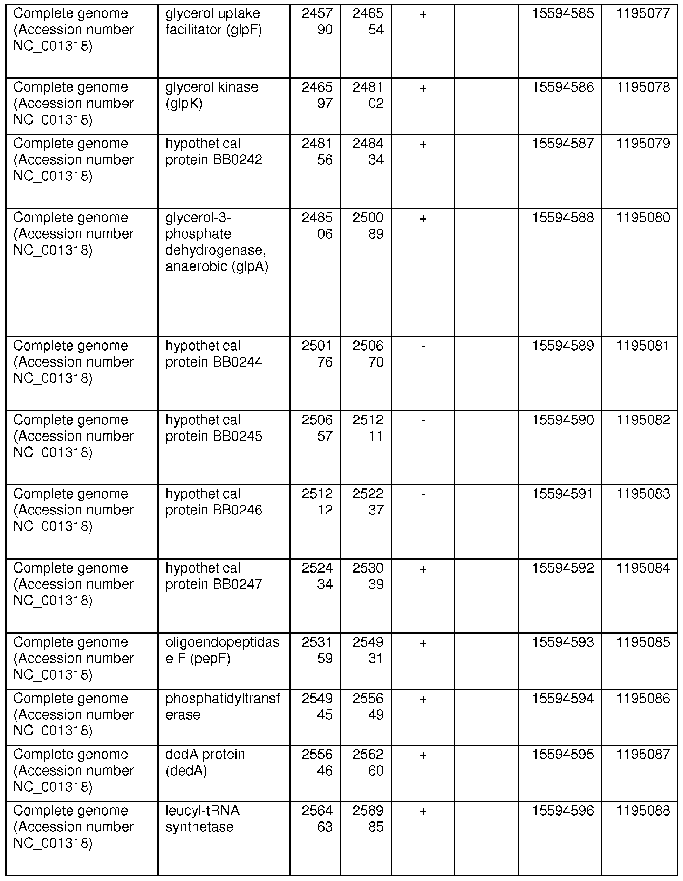

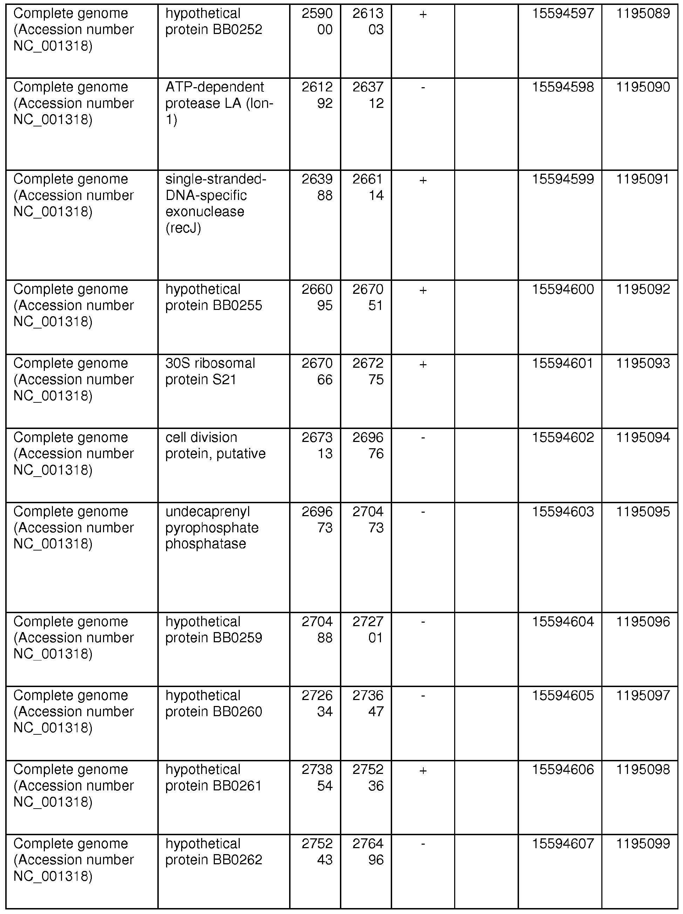

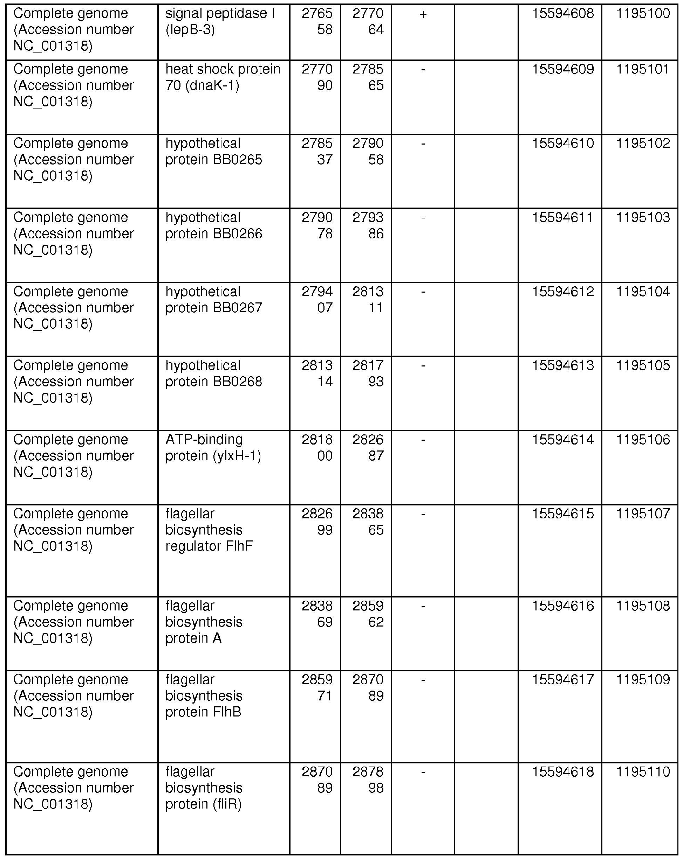

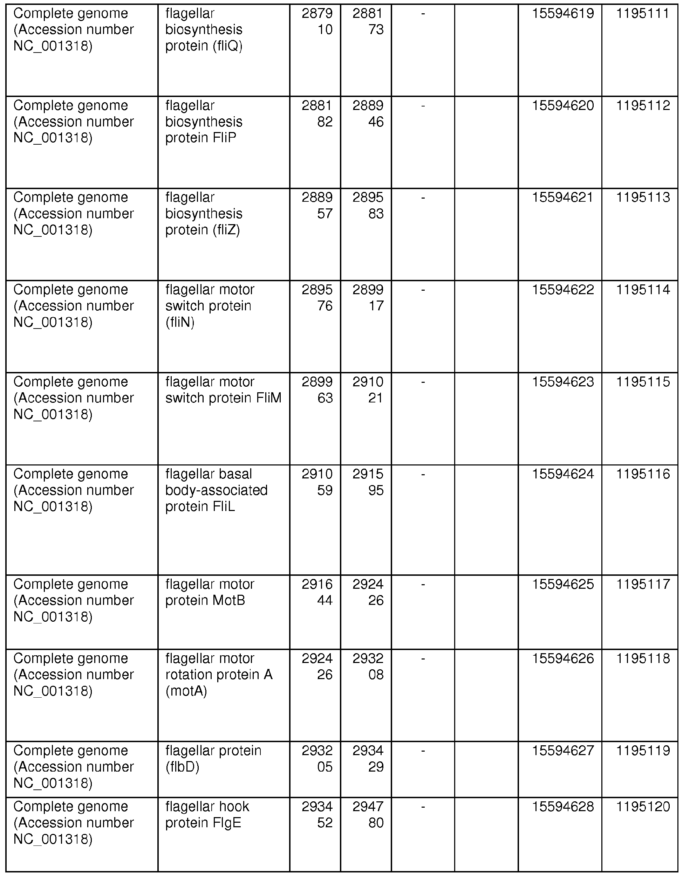

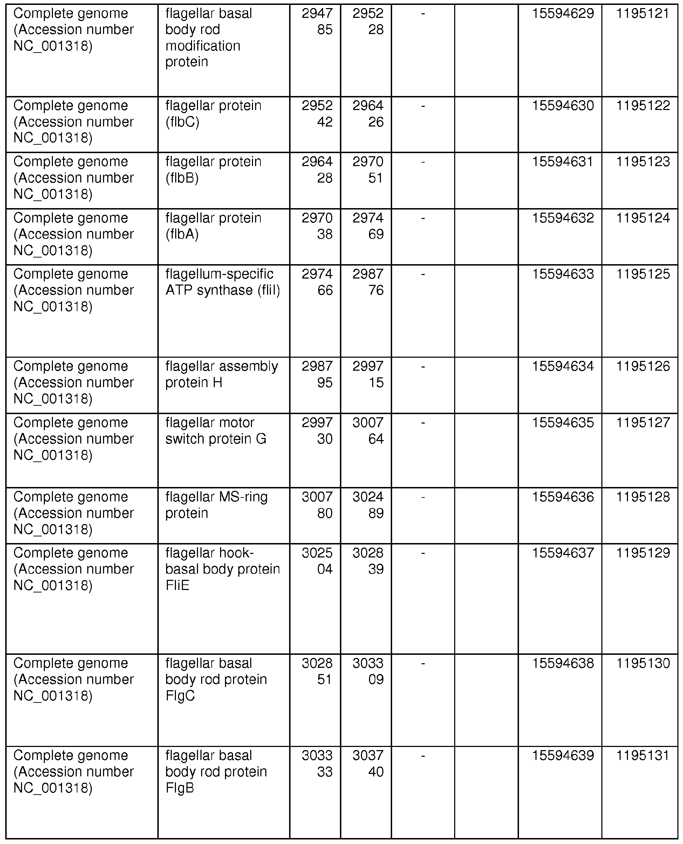

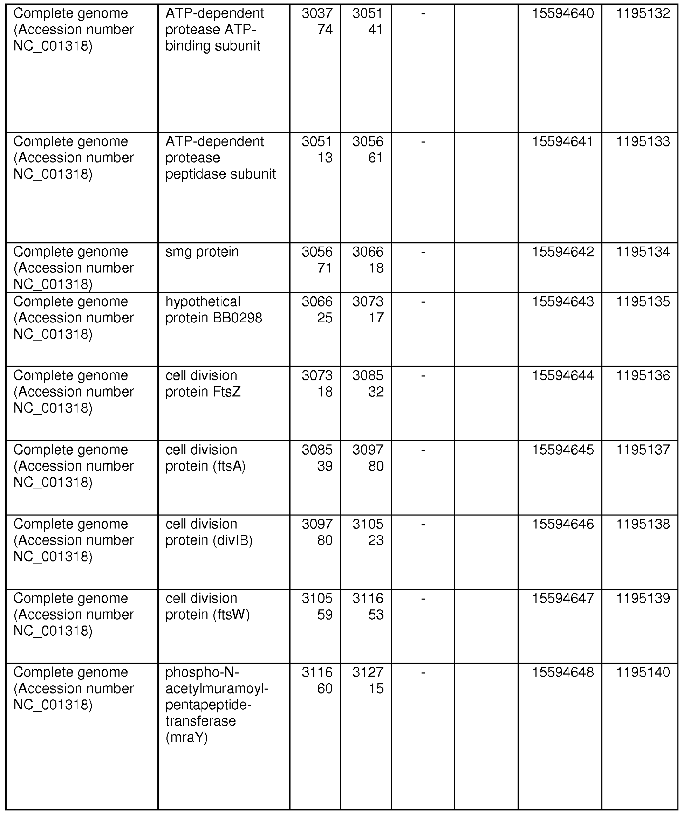

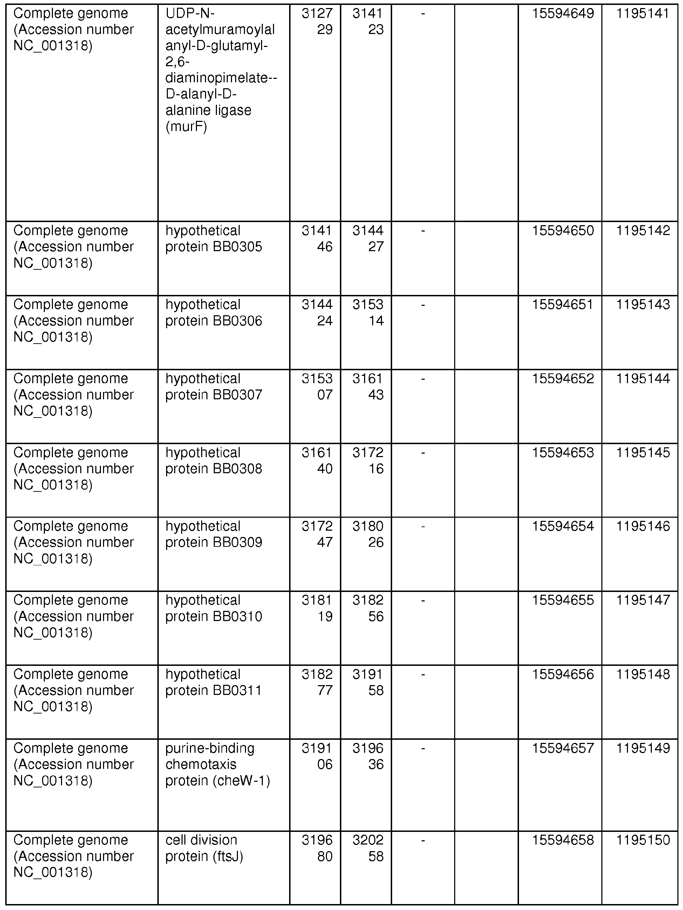

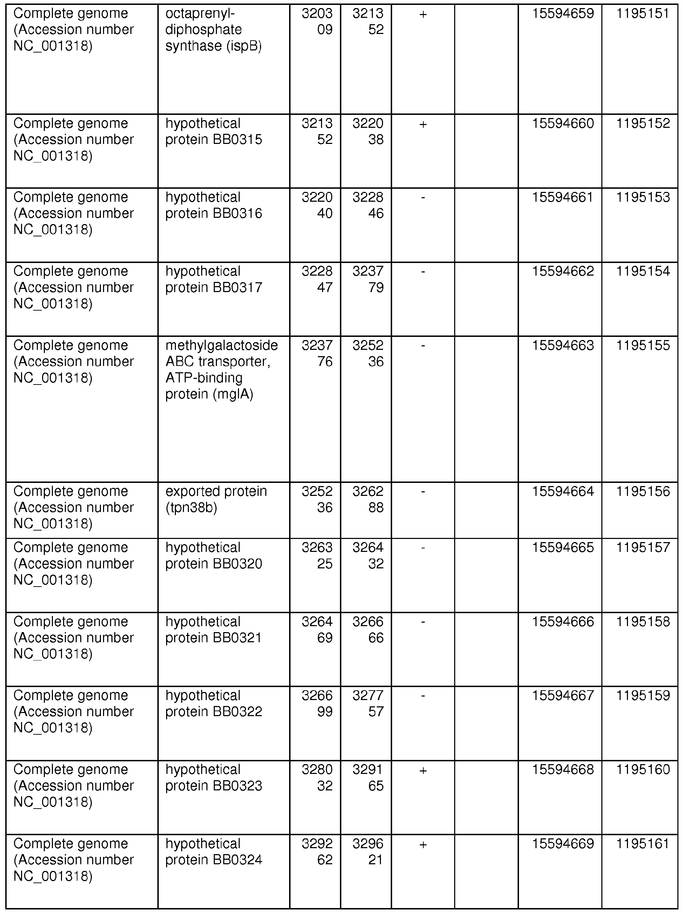

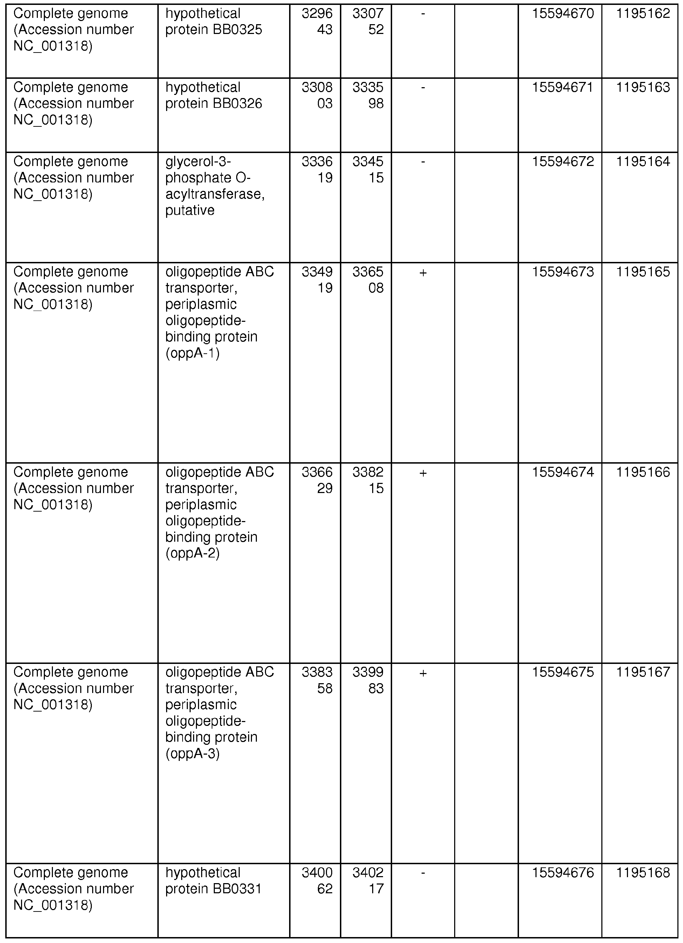

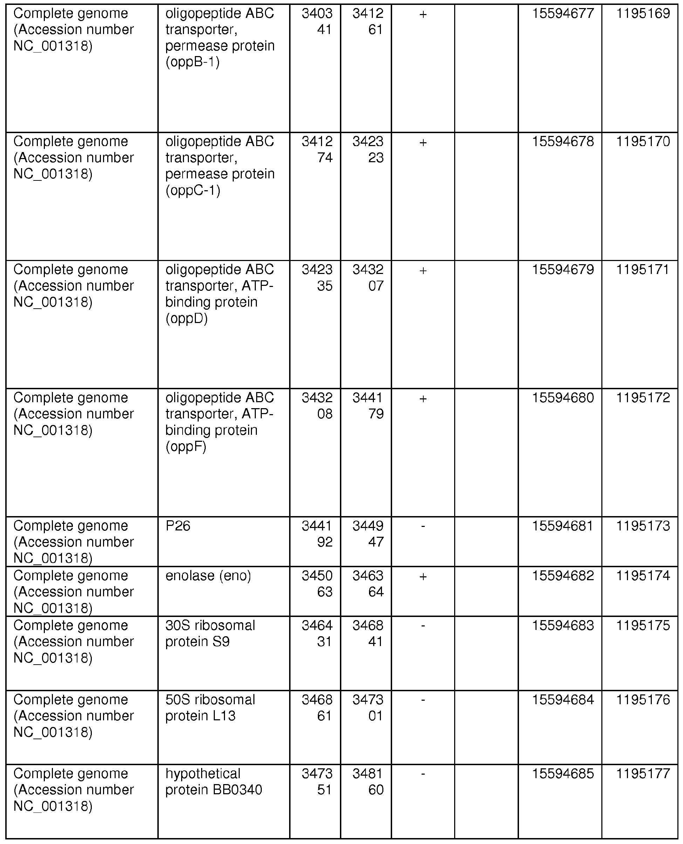

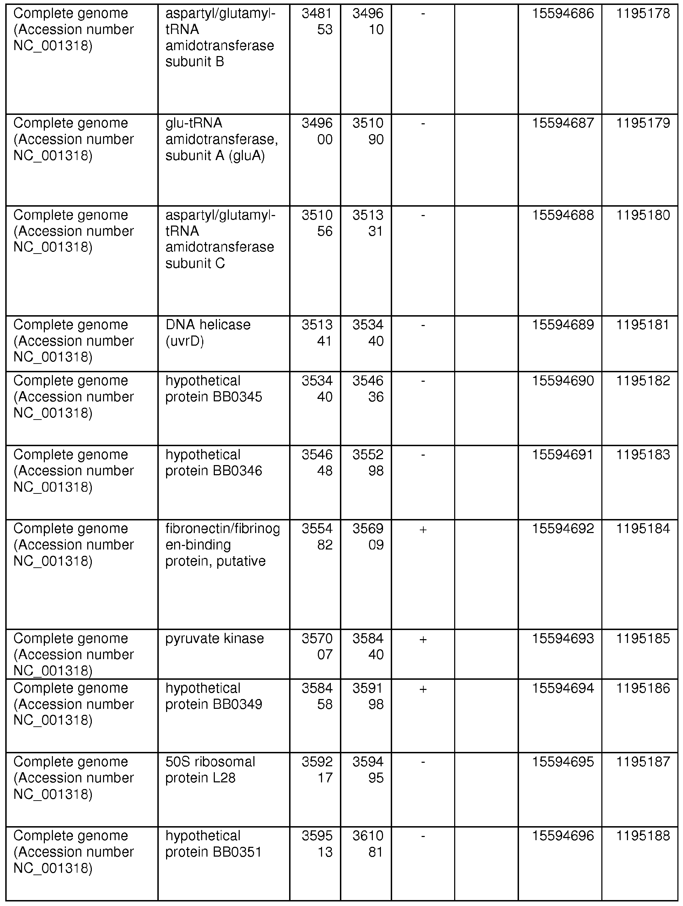

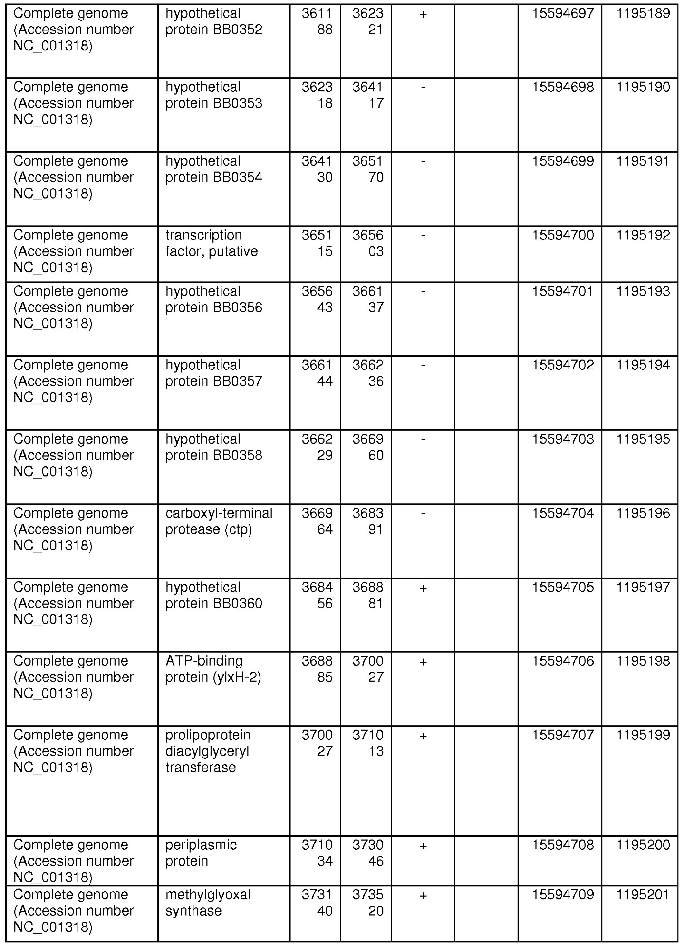

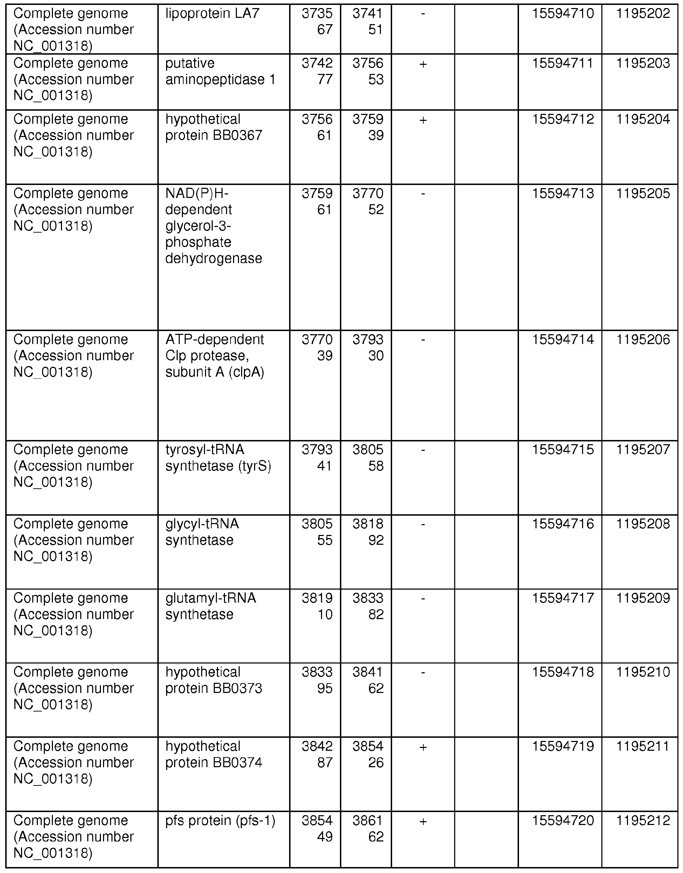

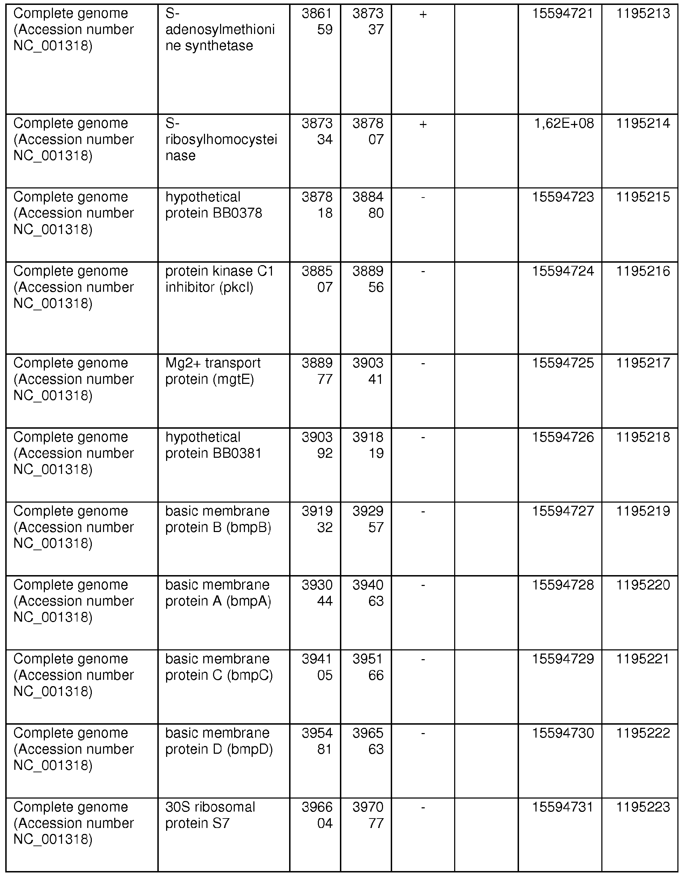

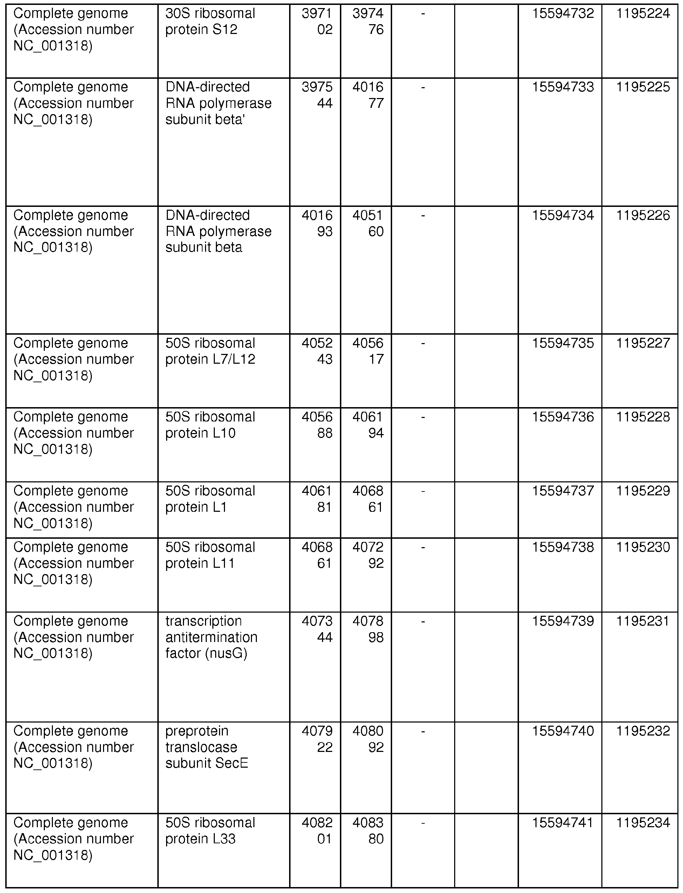

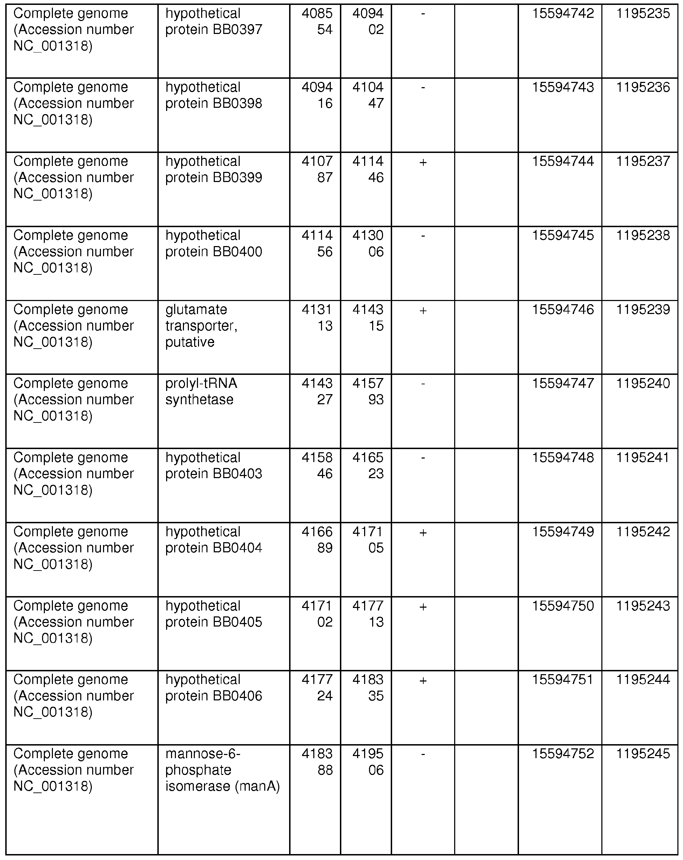

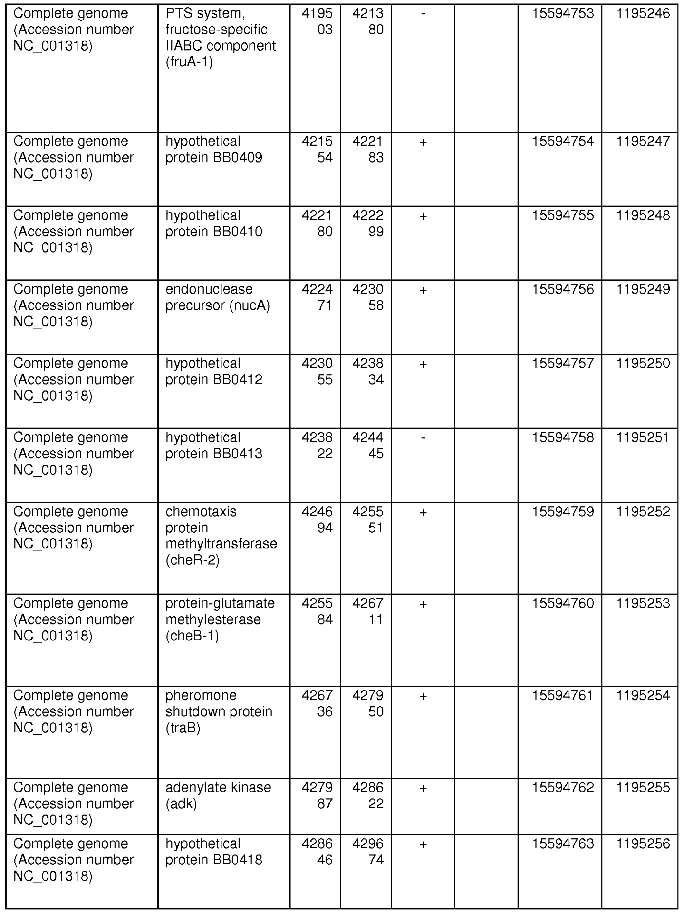

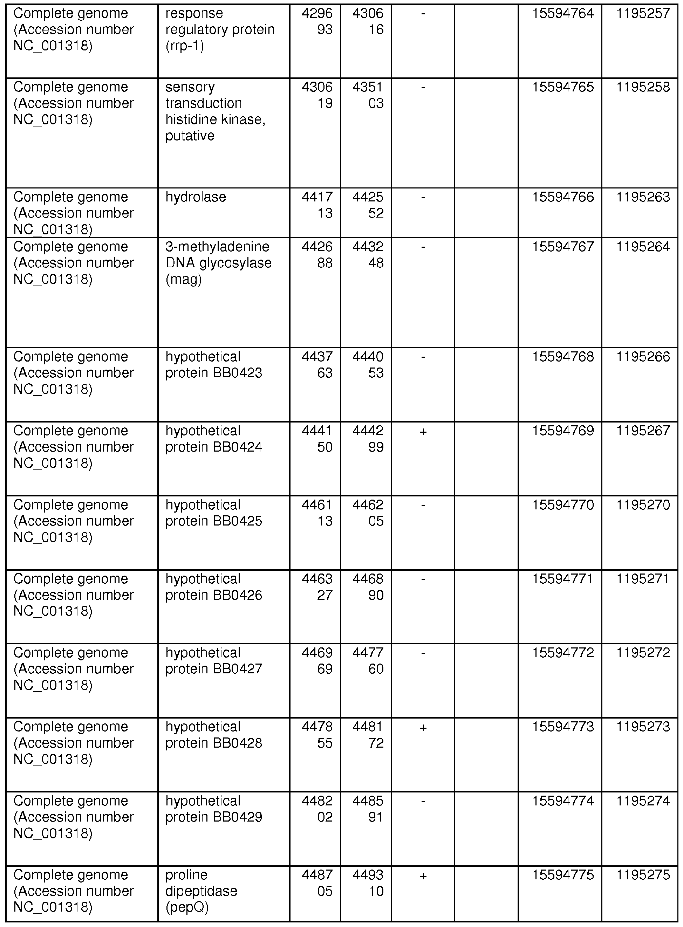

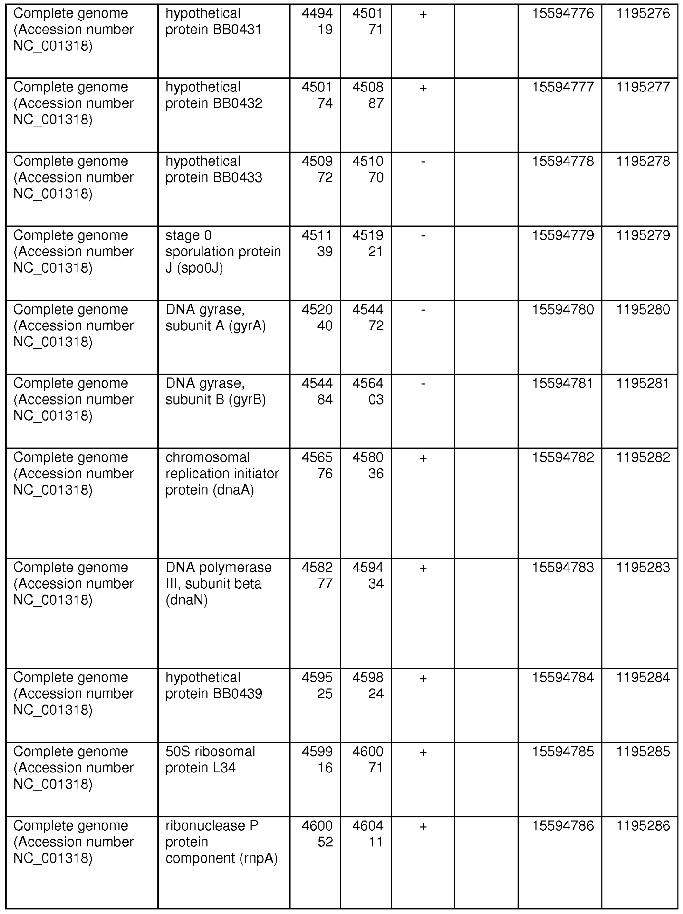

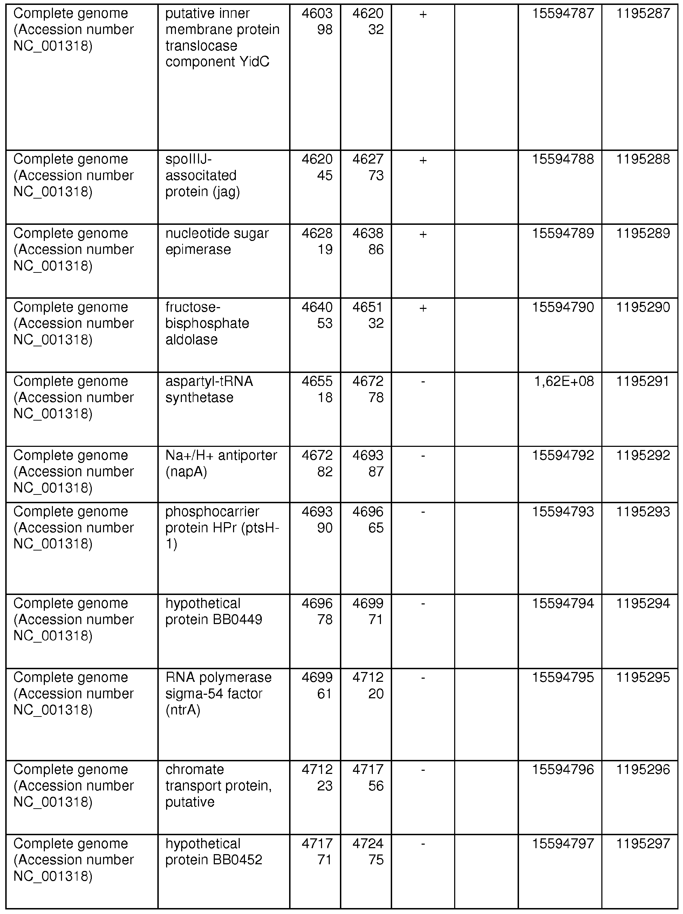

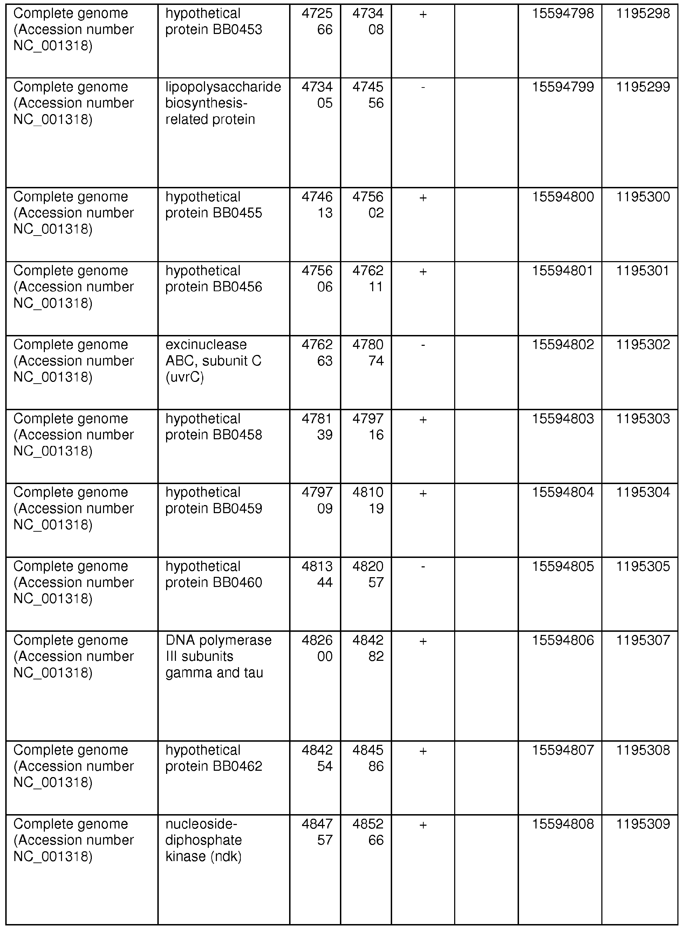

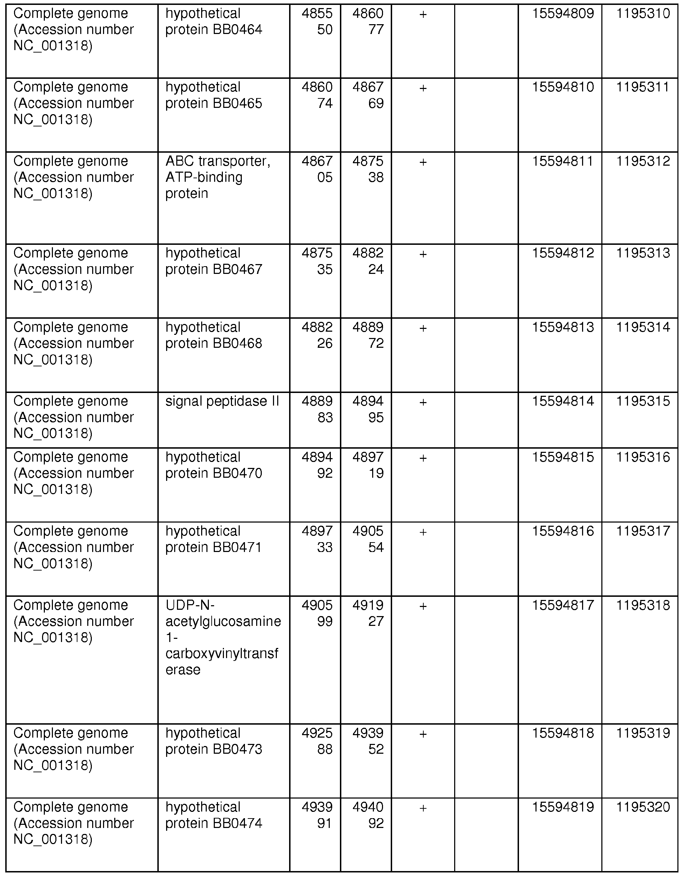

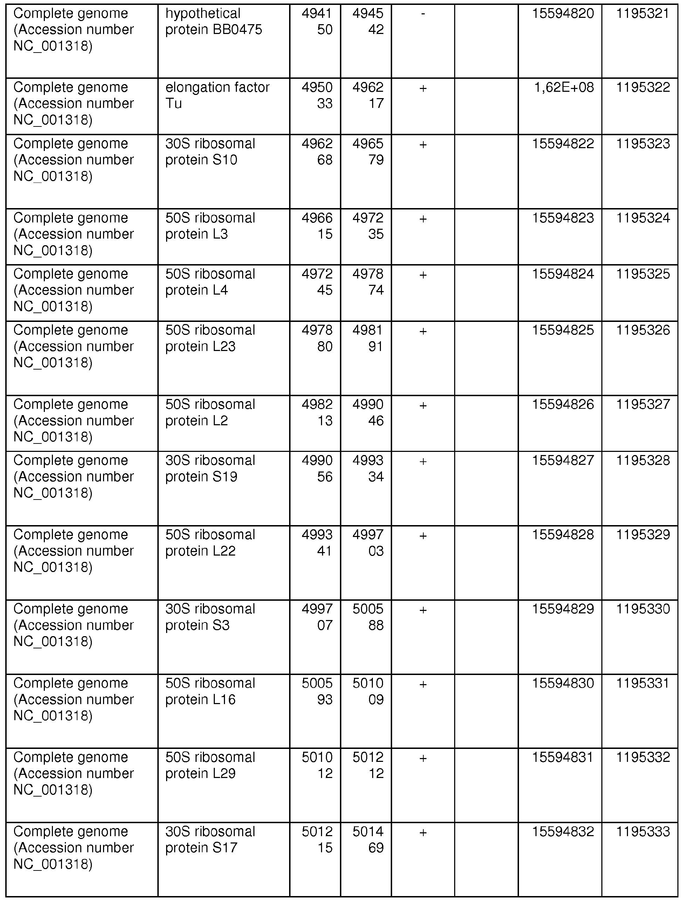

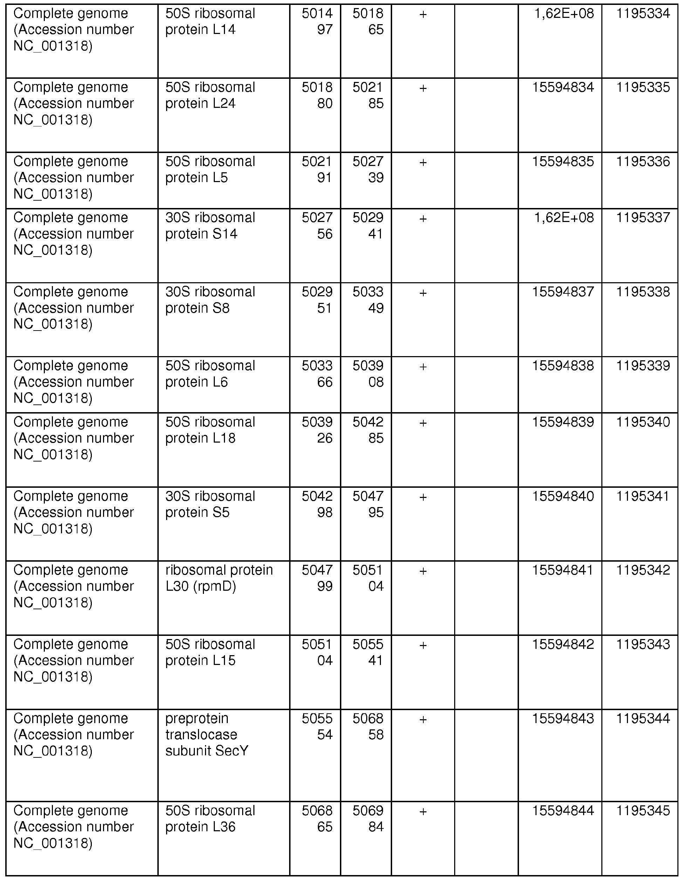

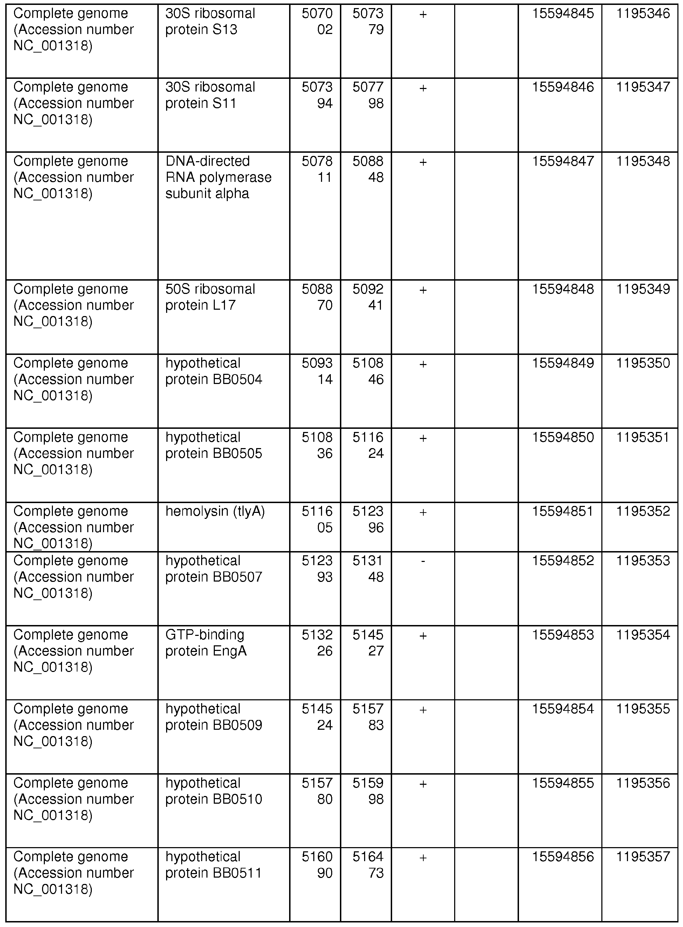

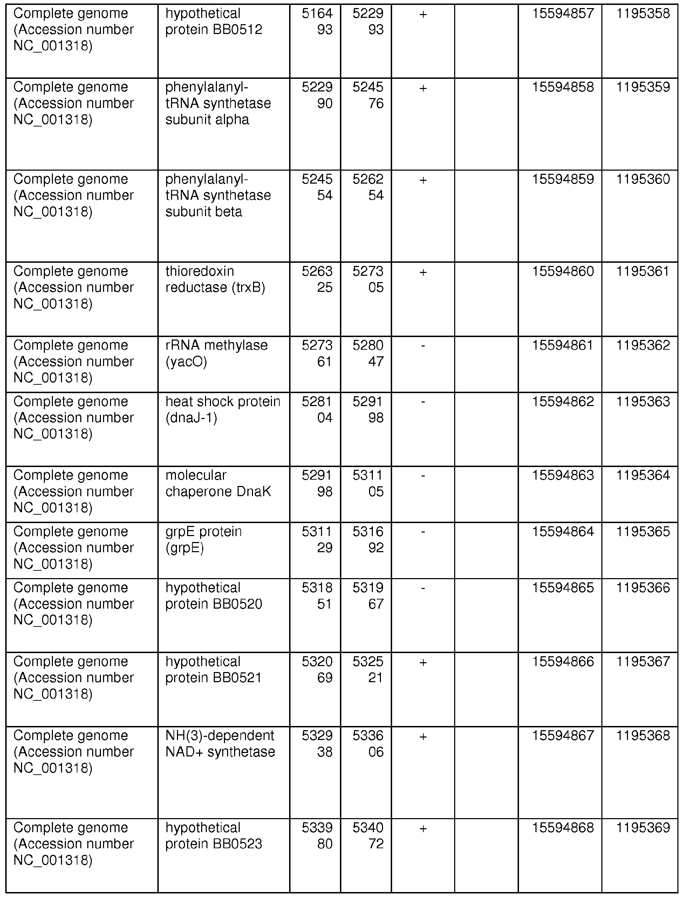

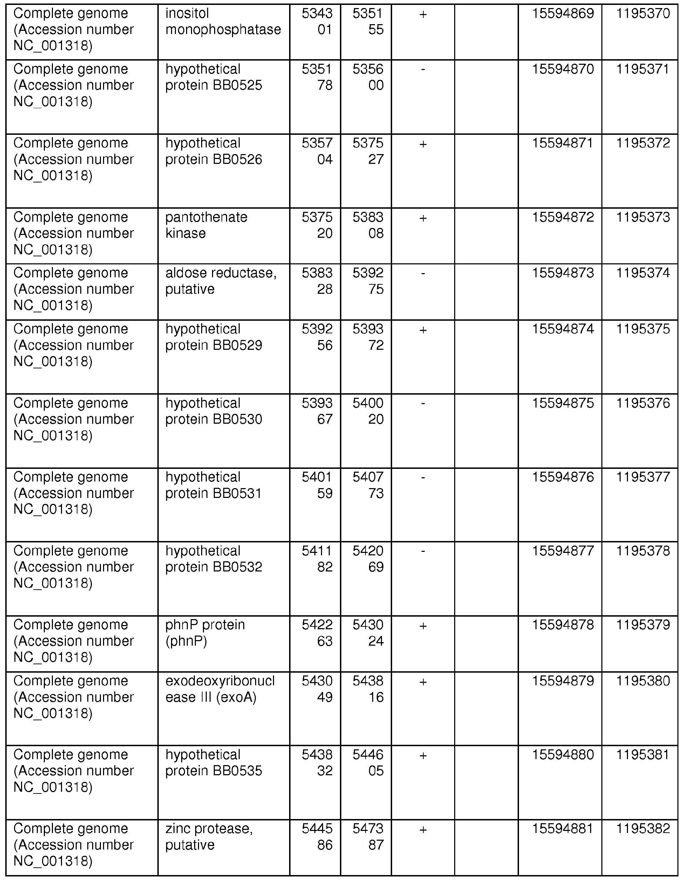

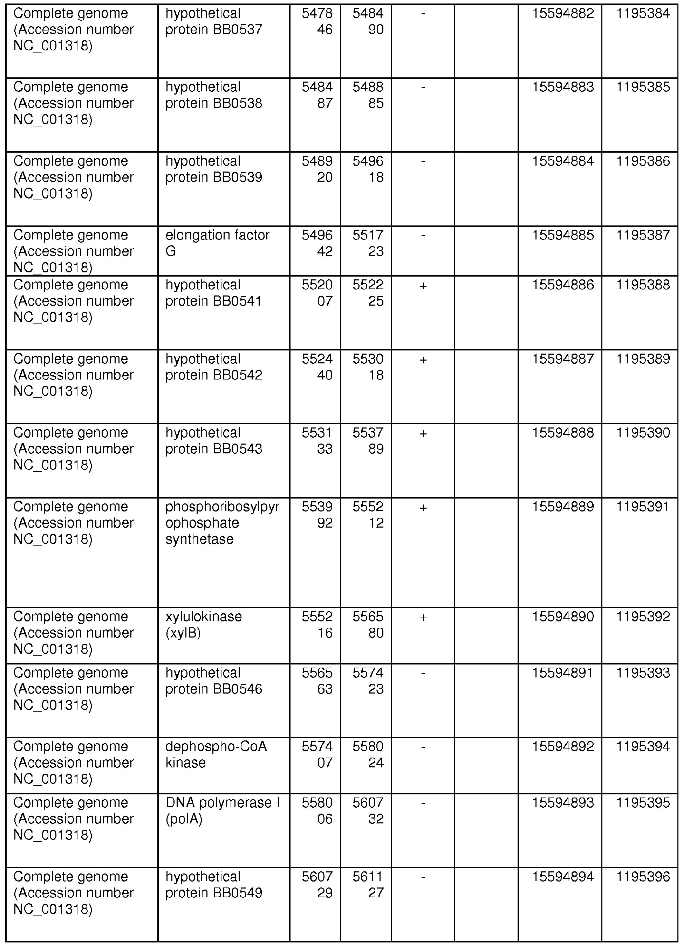

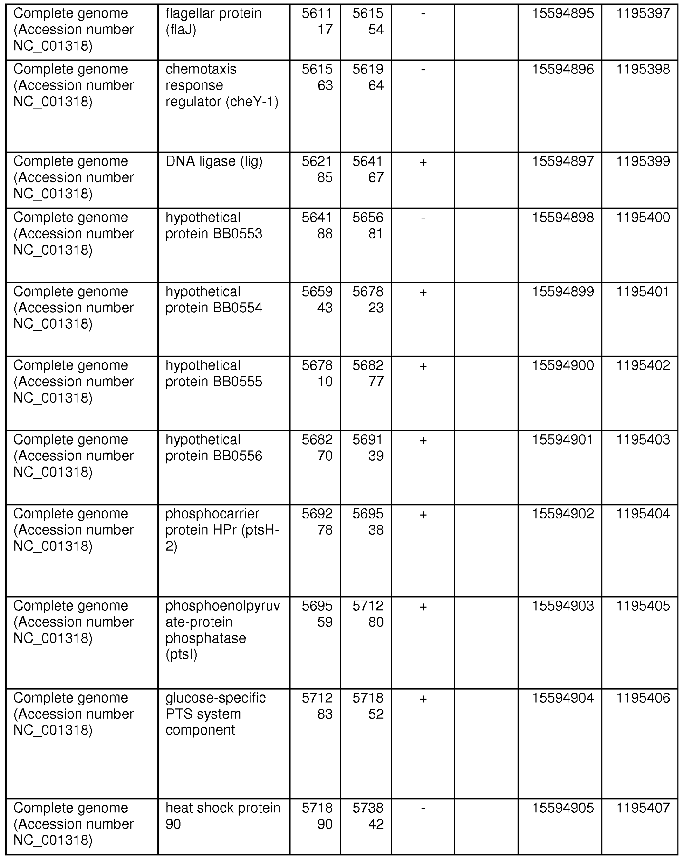

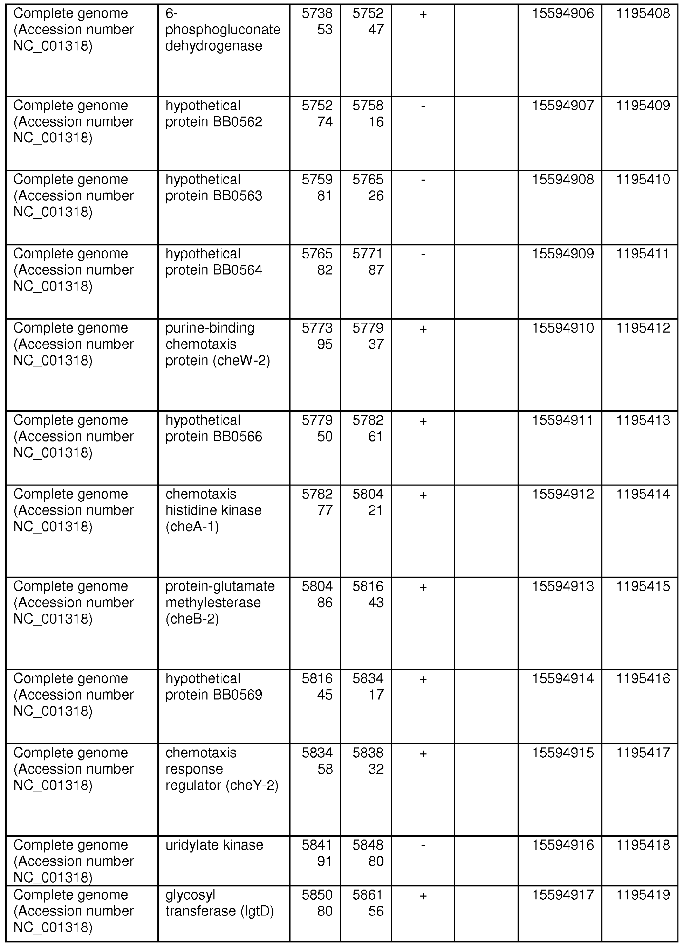

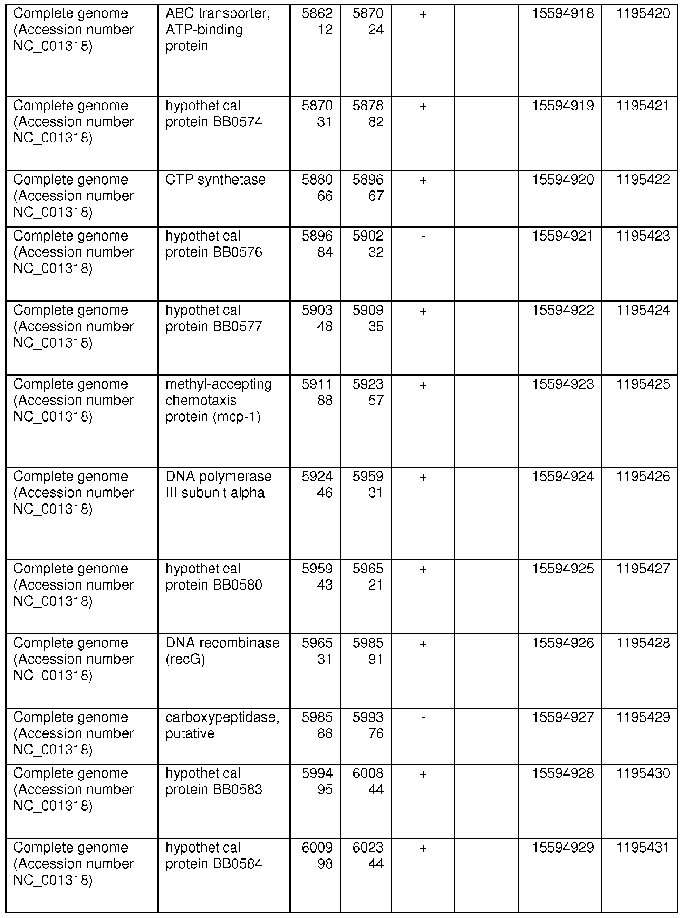

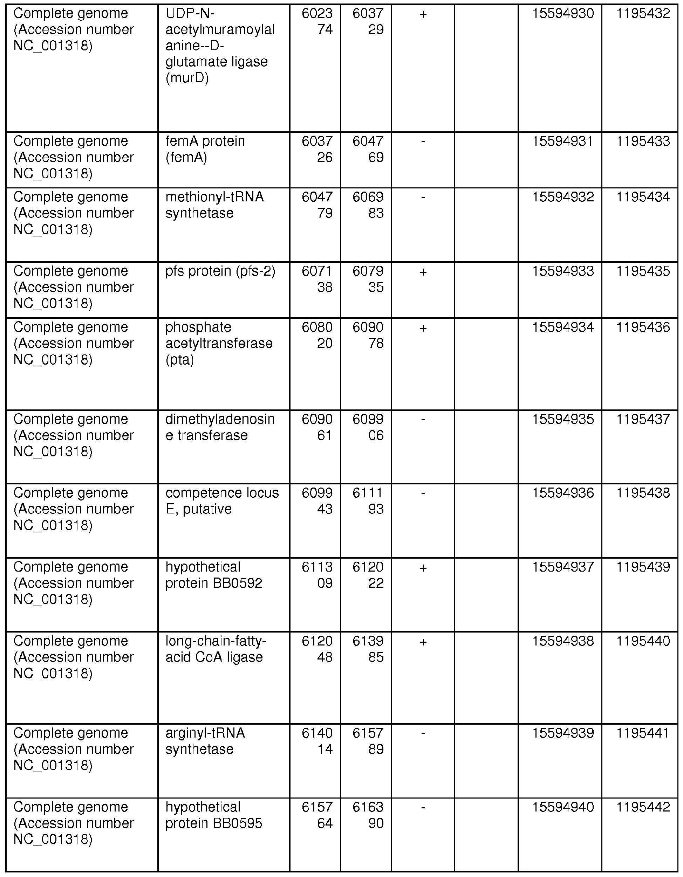

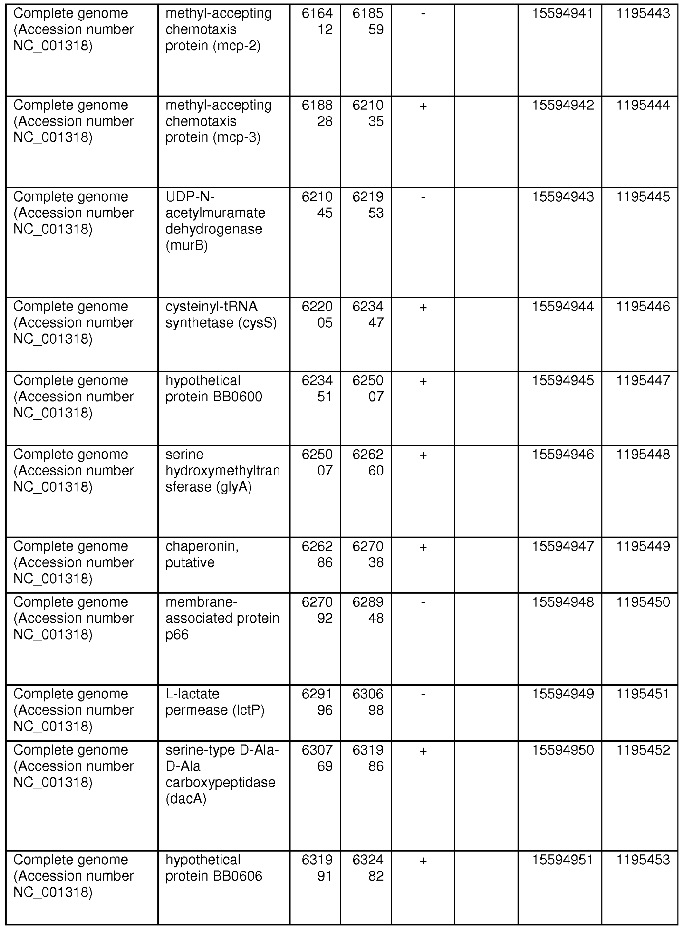

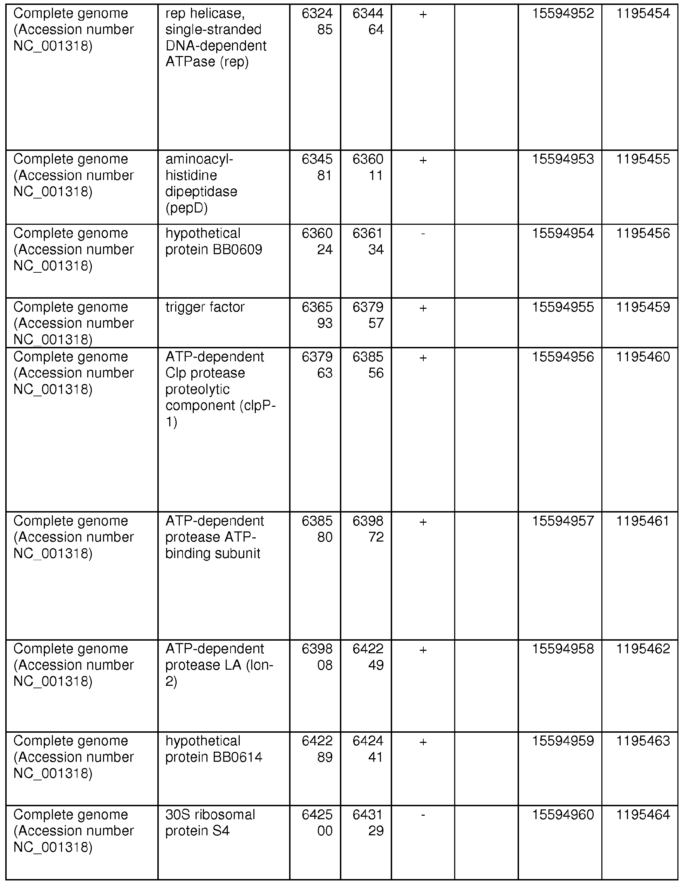

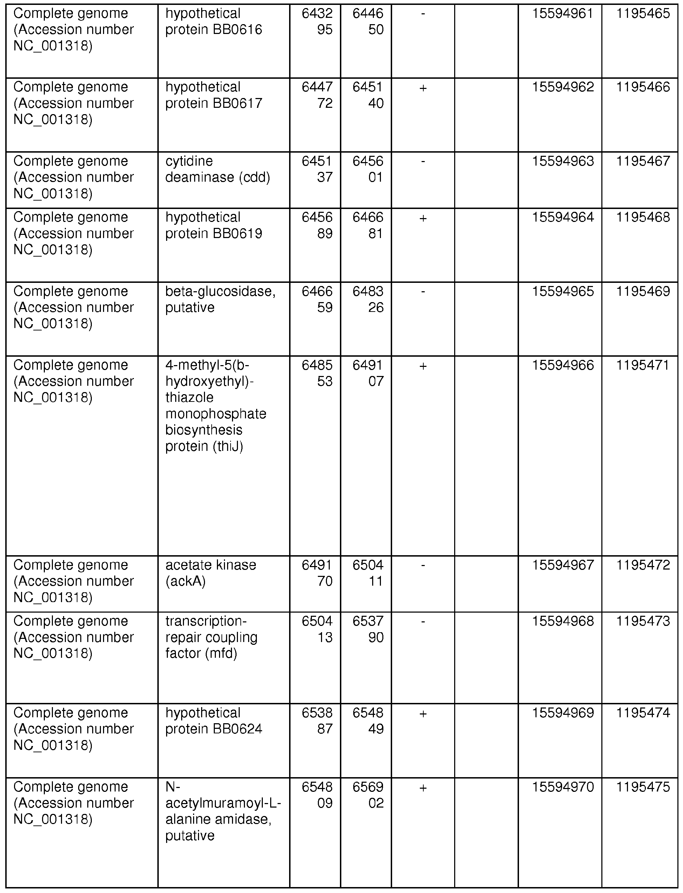

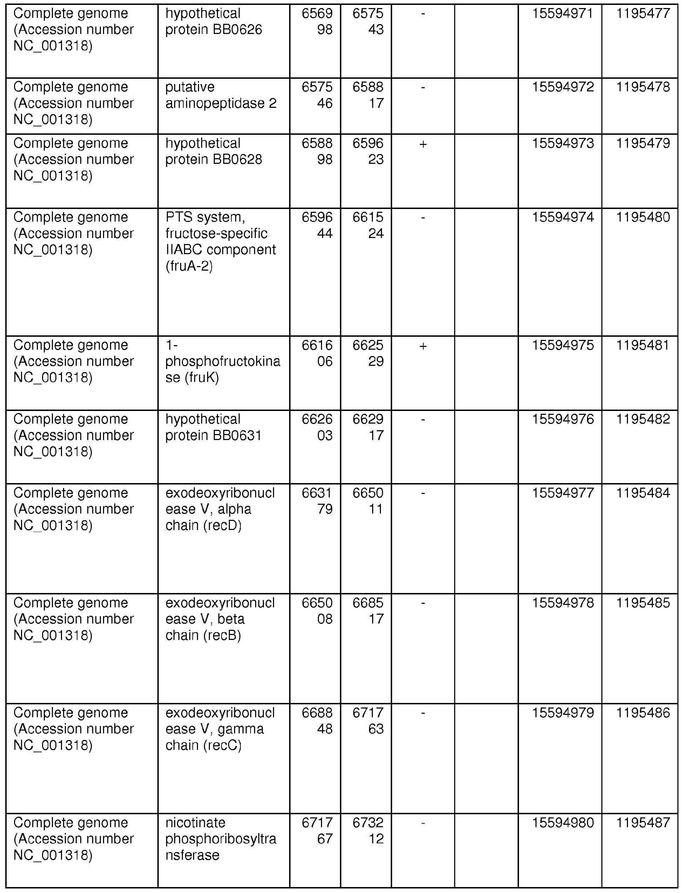

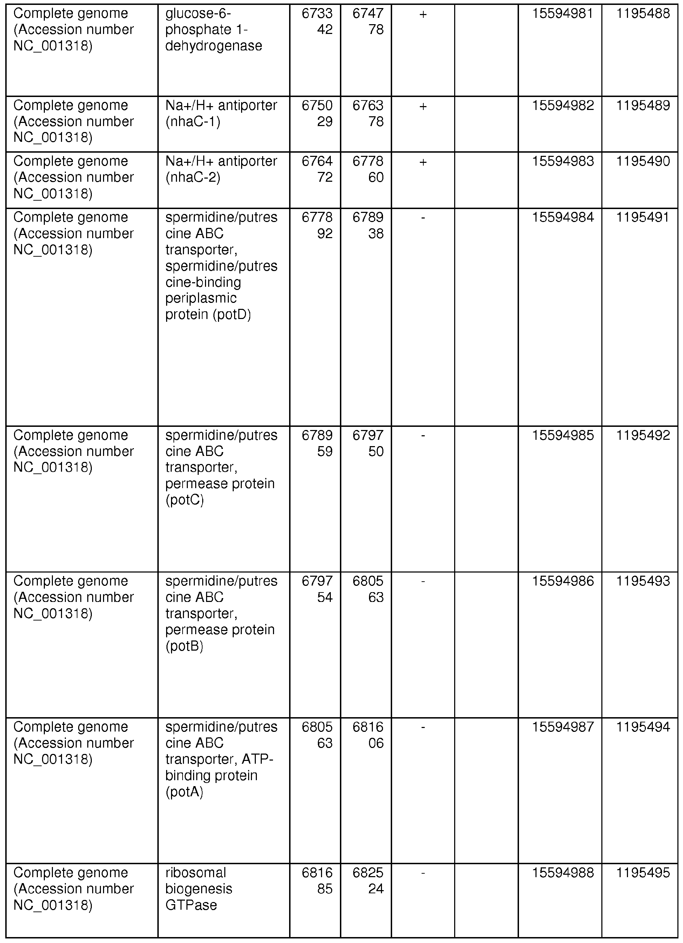

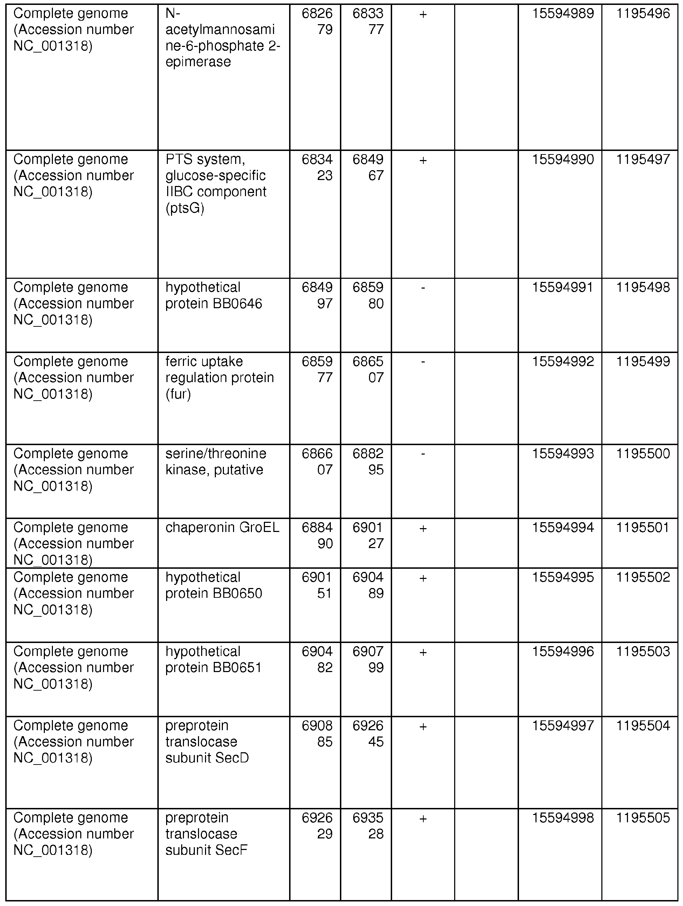

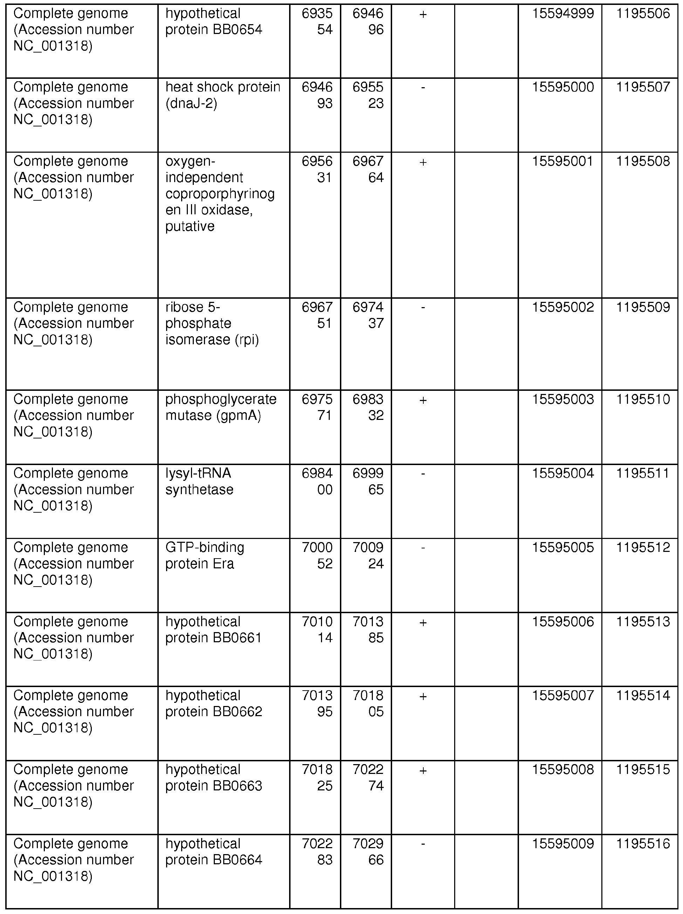

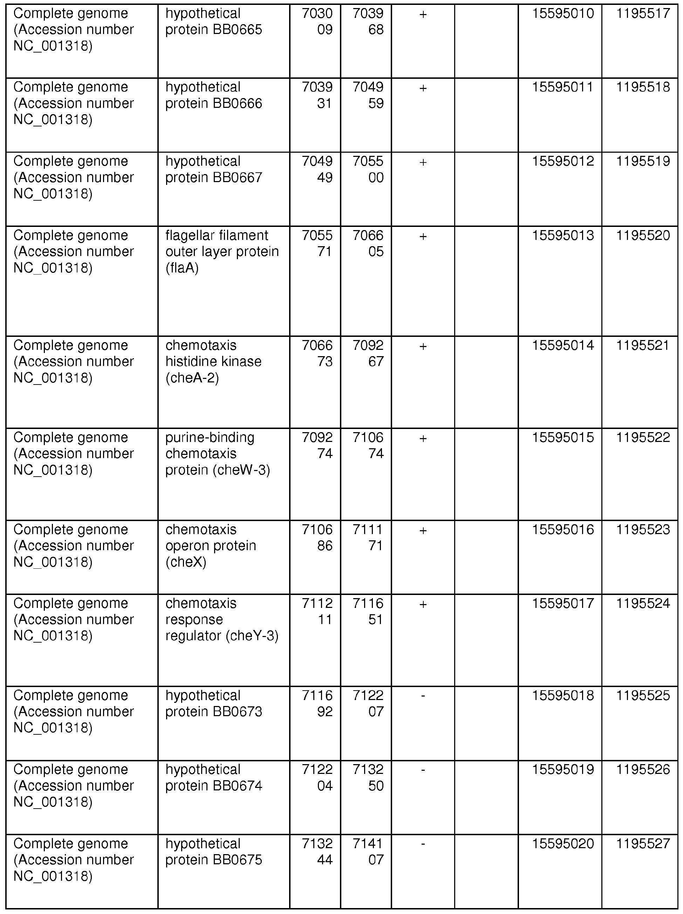

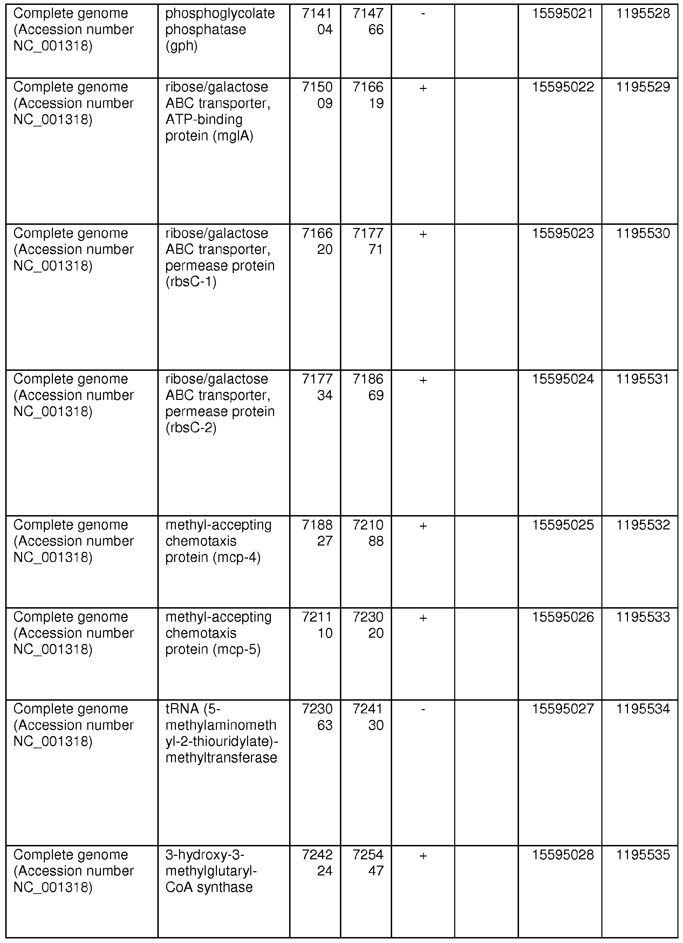

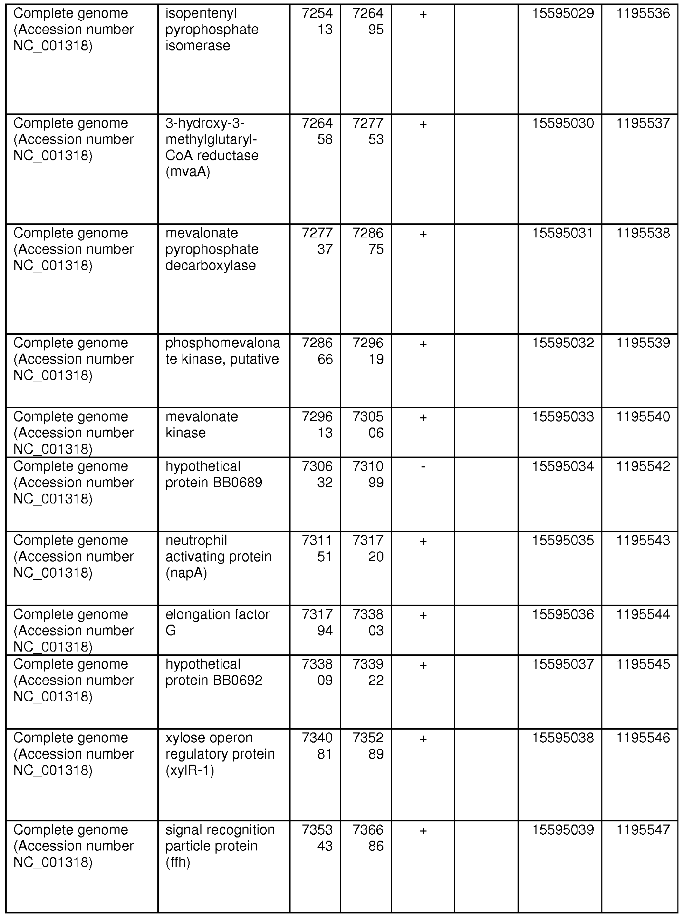

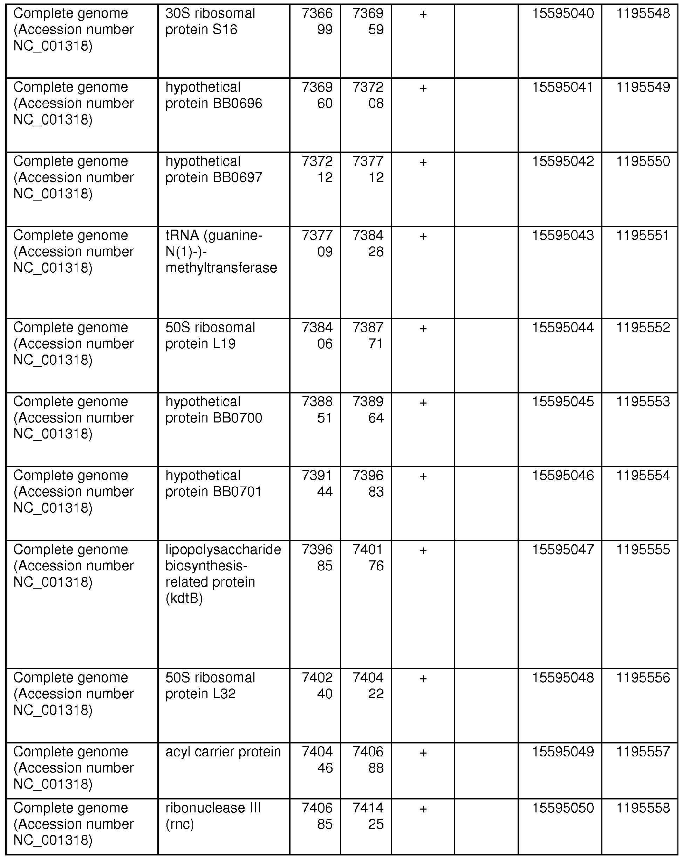

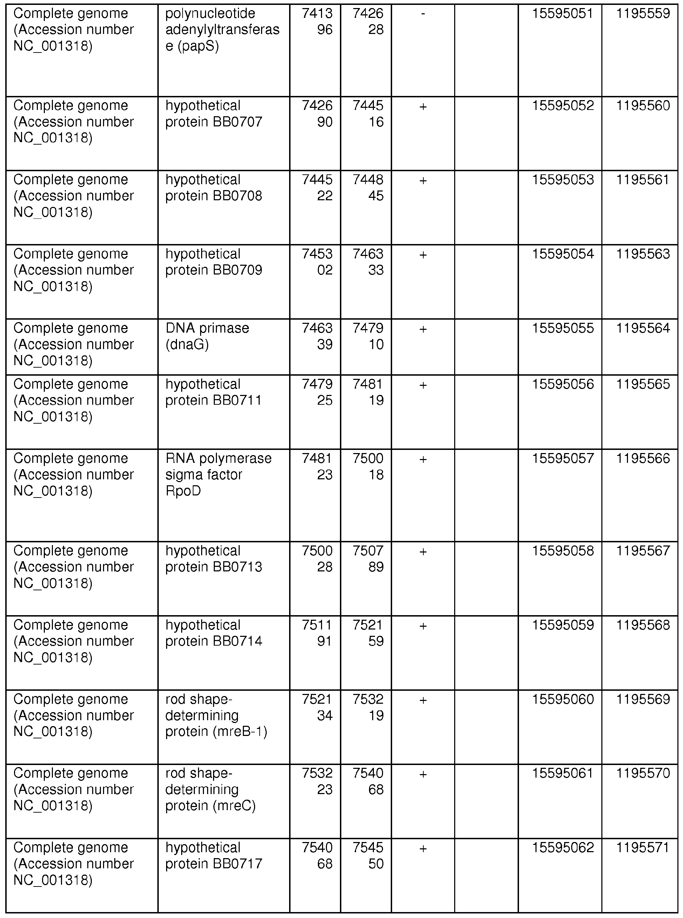

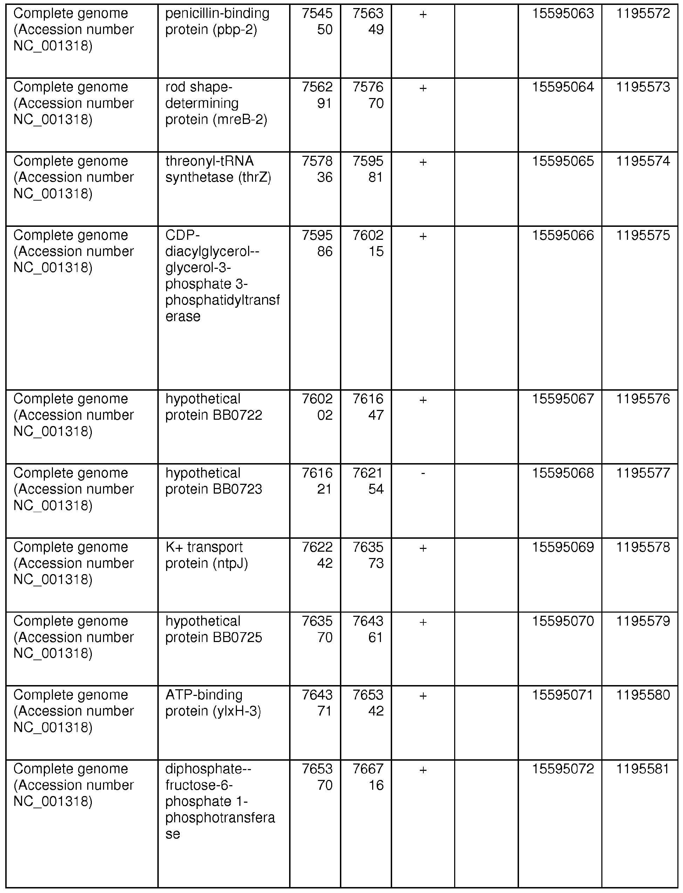

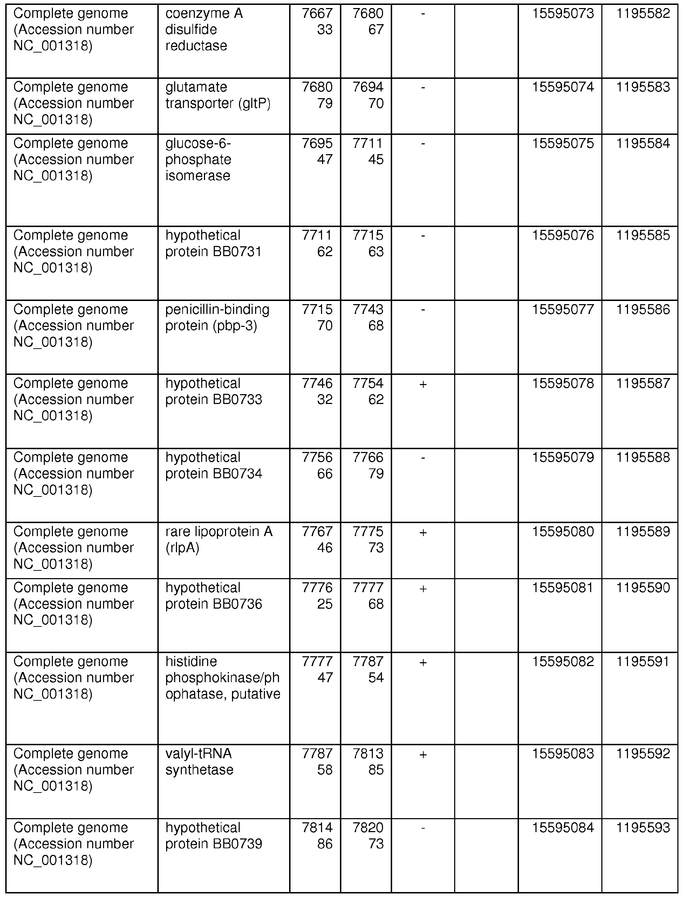

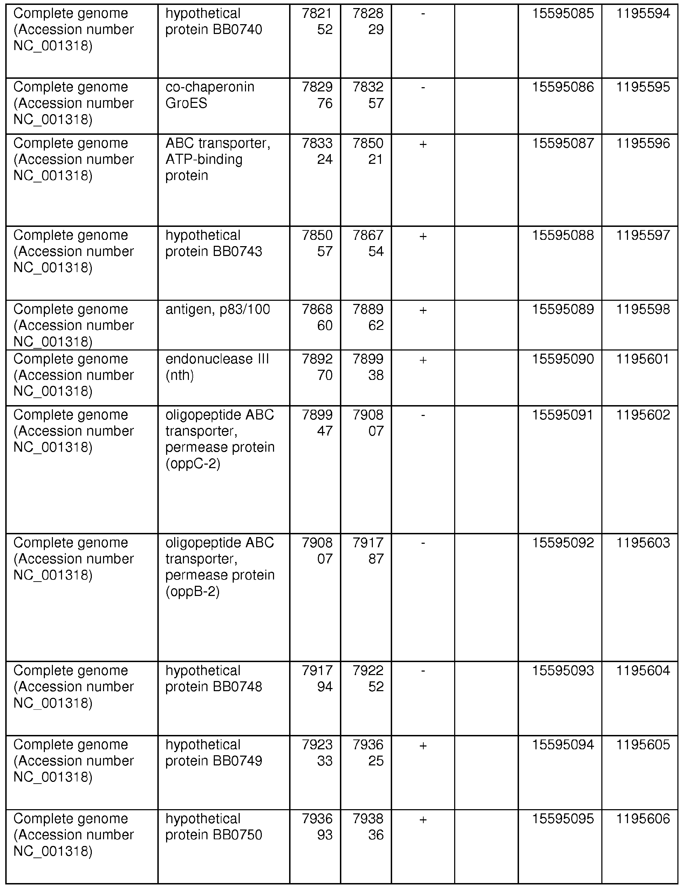

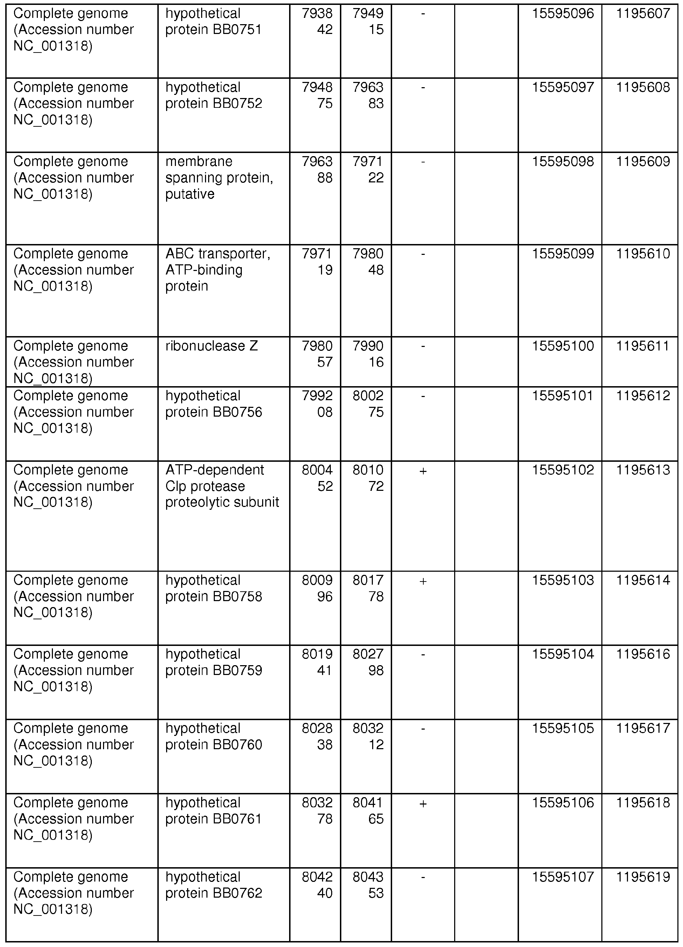

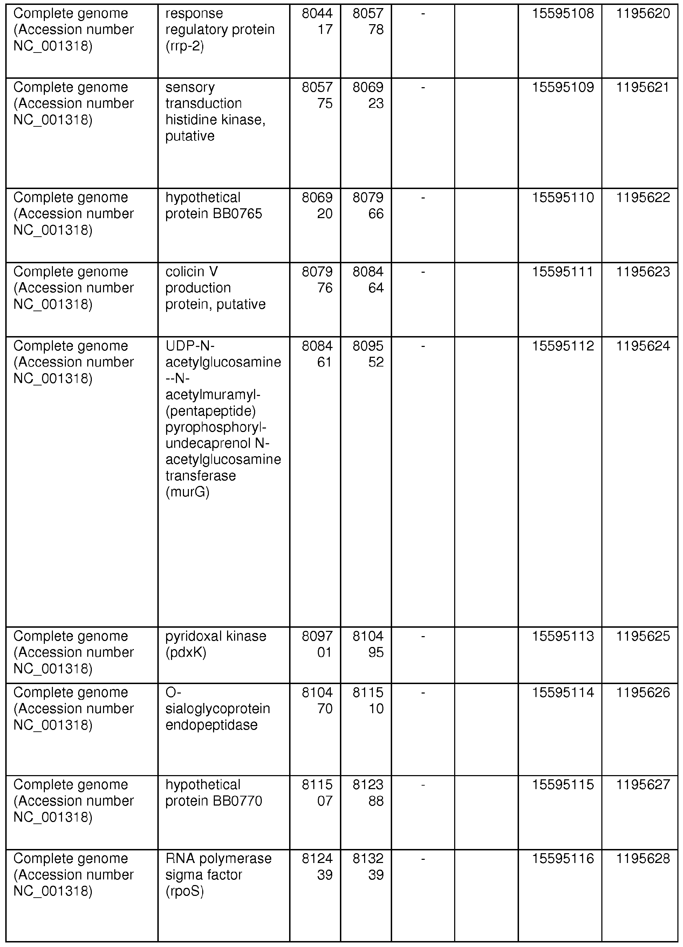

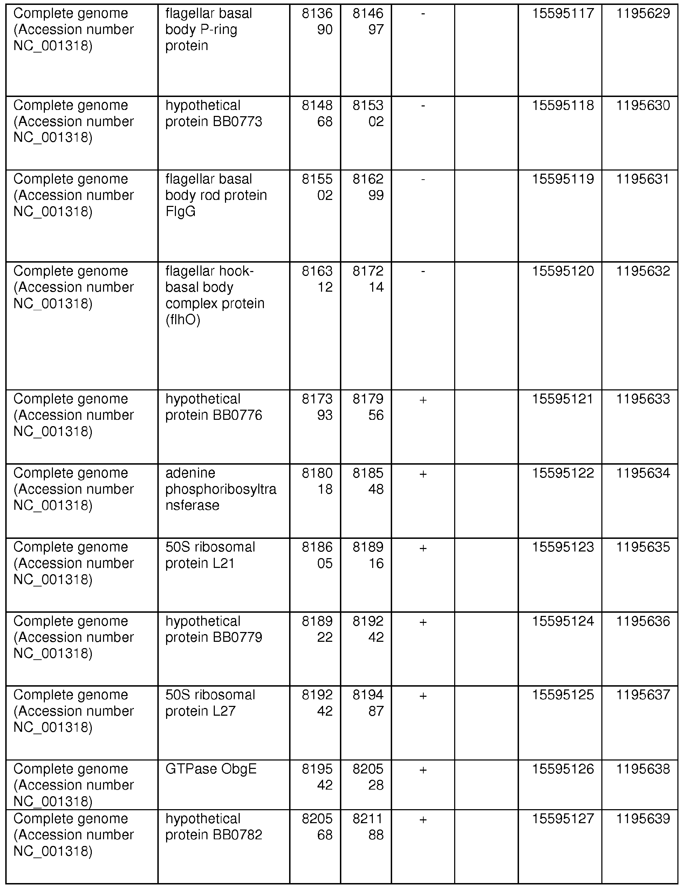

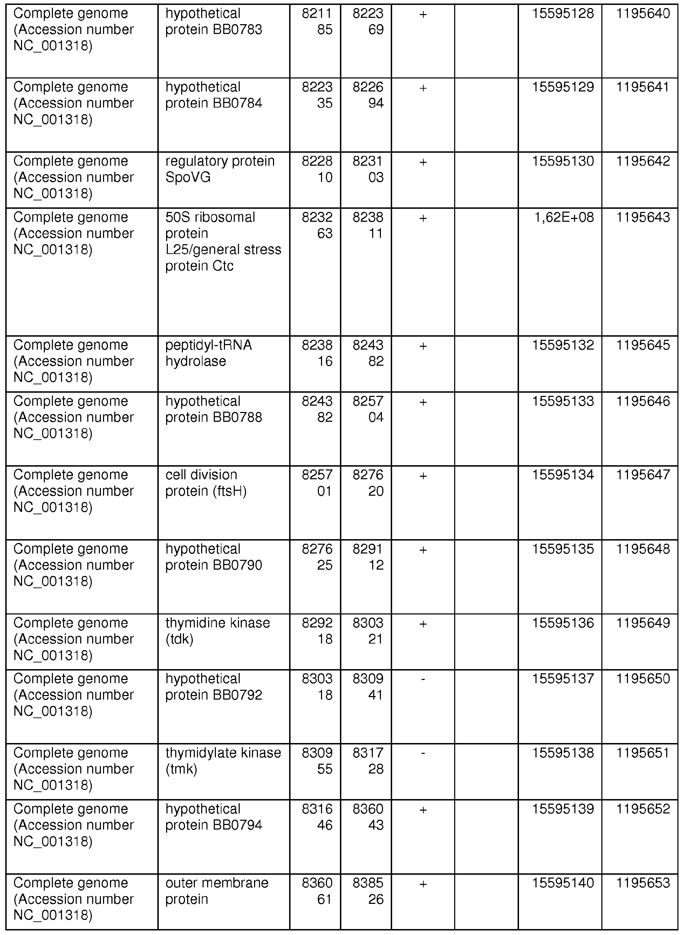

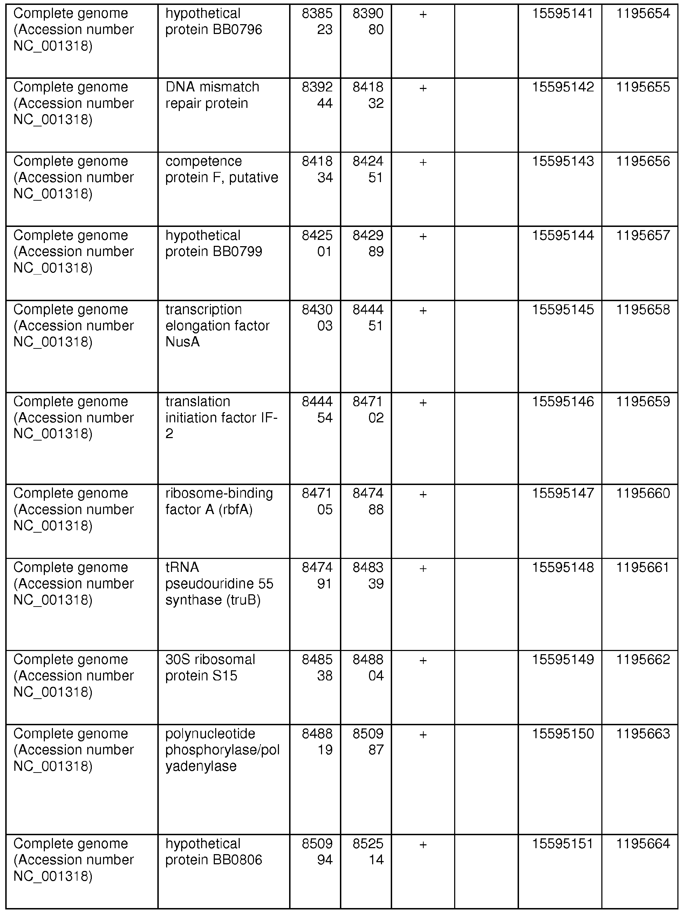

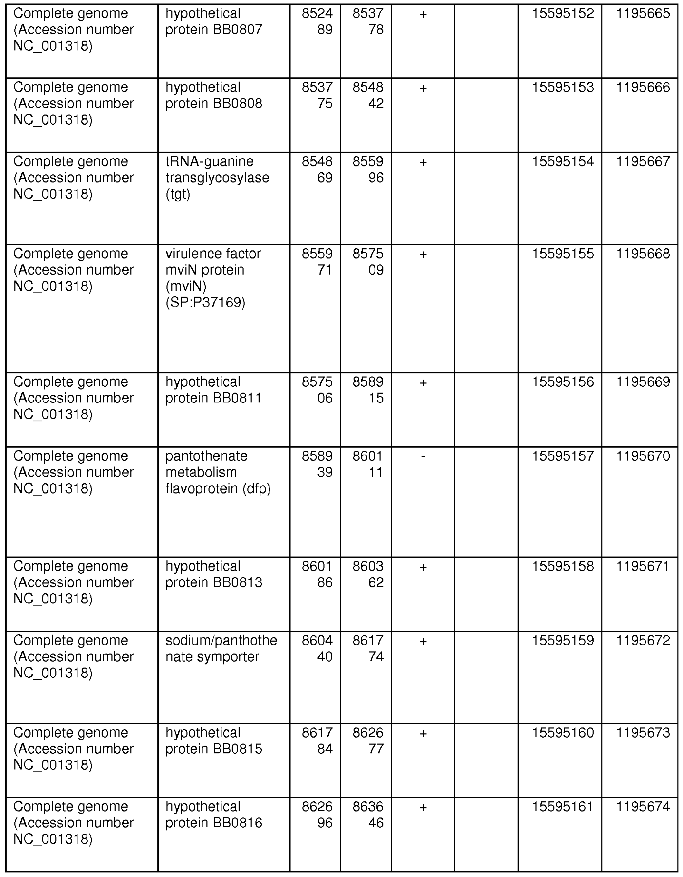

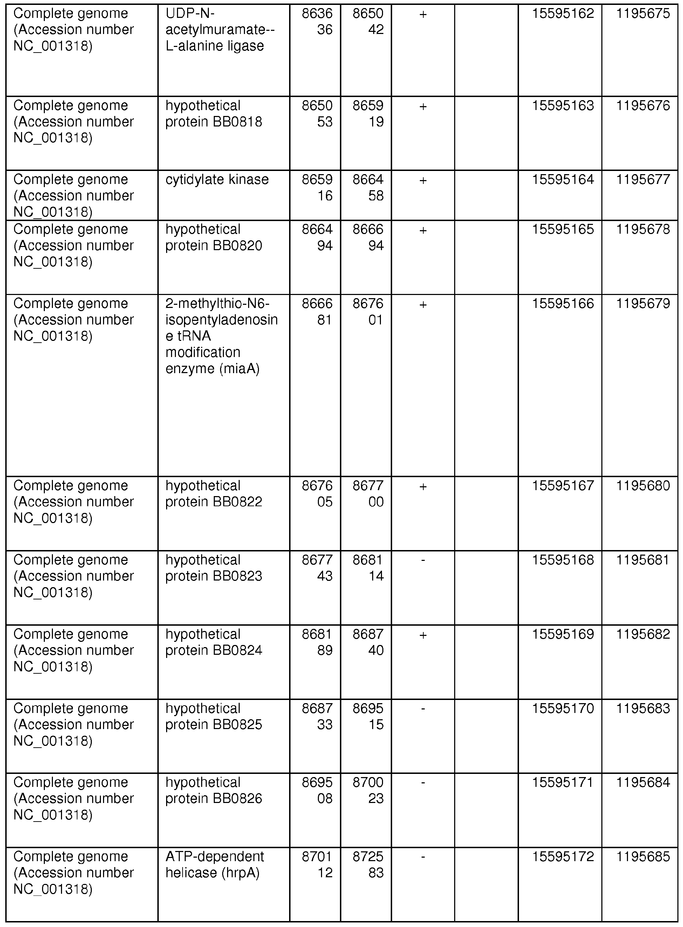

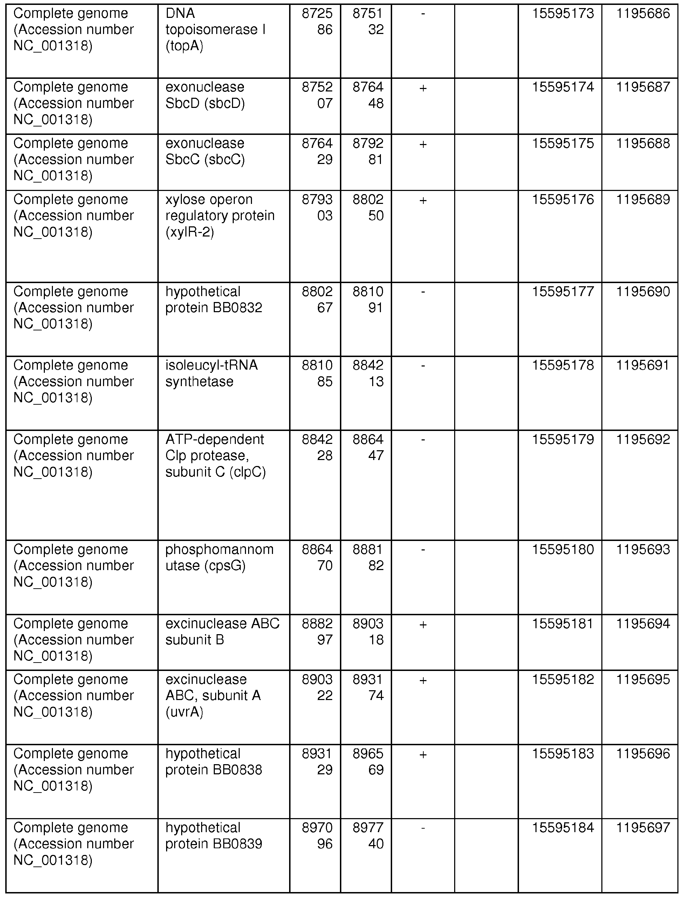

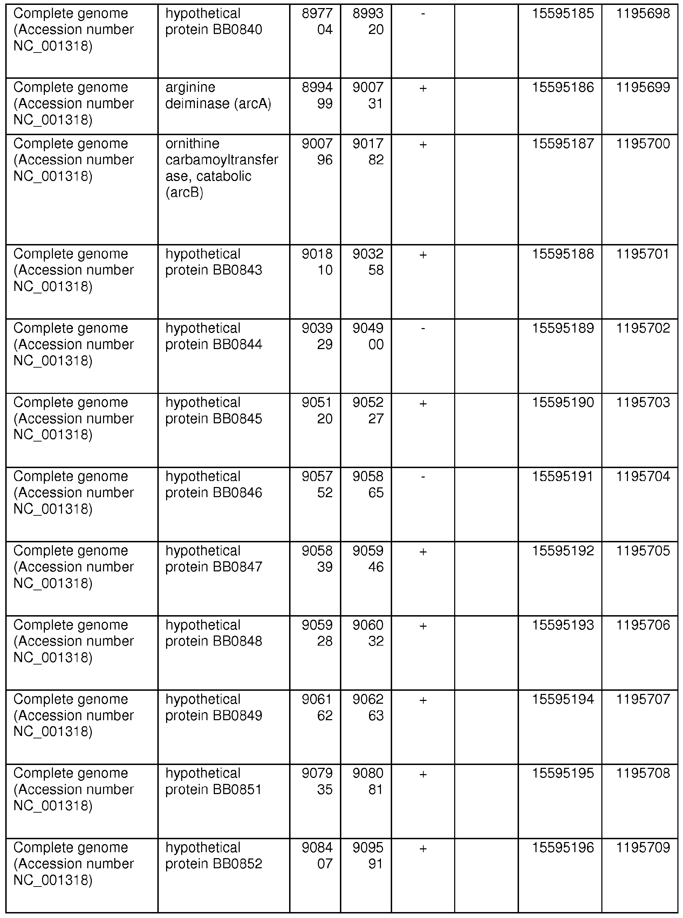

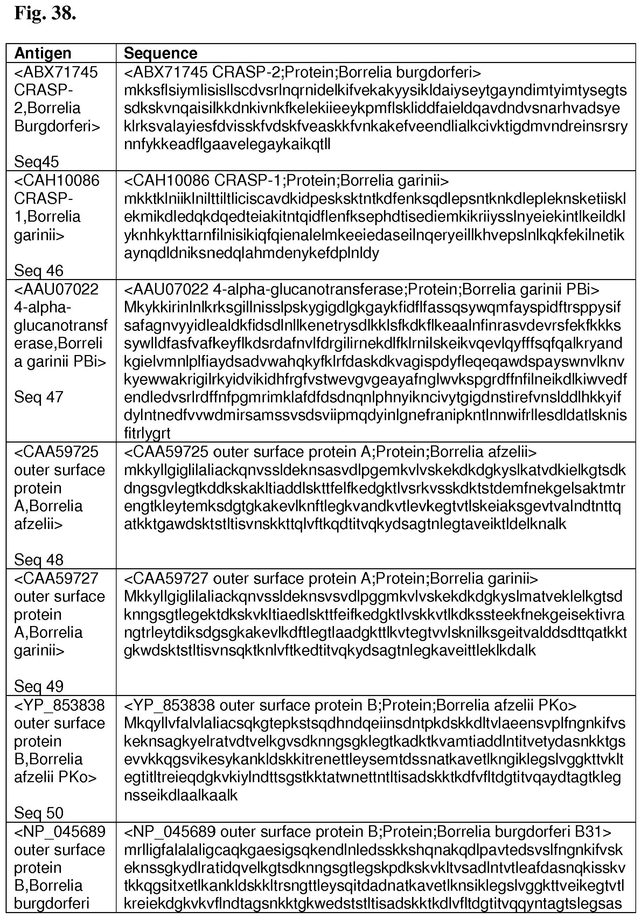

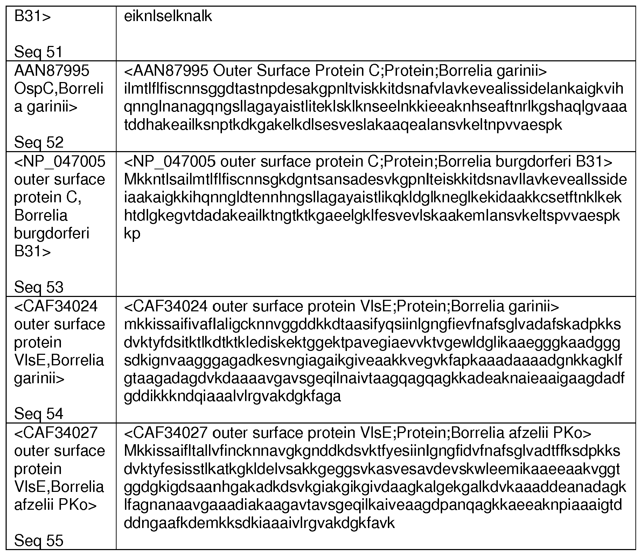

In another preferred embodiment the present disclosure relates to one or more antigenic peptides or antigenic polypeptides comprising a fragment of the sequence from one or more proteins from Borrelia bulgdorferi B31, or to one or more MHC-peptide complexes or MHC multimers, compricing one or more such antigenic peptides. The one or more proteins can be selected from the proteins listed in Figure 27 and Figure 38. These fagments can e.g. be 8 mers, 9 mers, 10 mers, 11 mers, 12 mers, 13 mers, 14 mers, 15 mers or 16 mers.

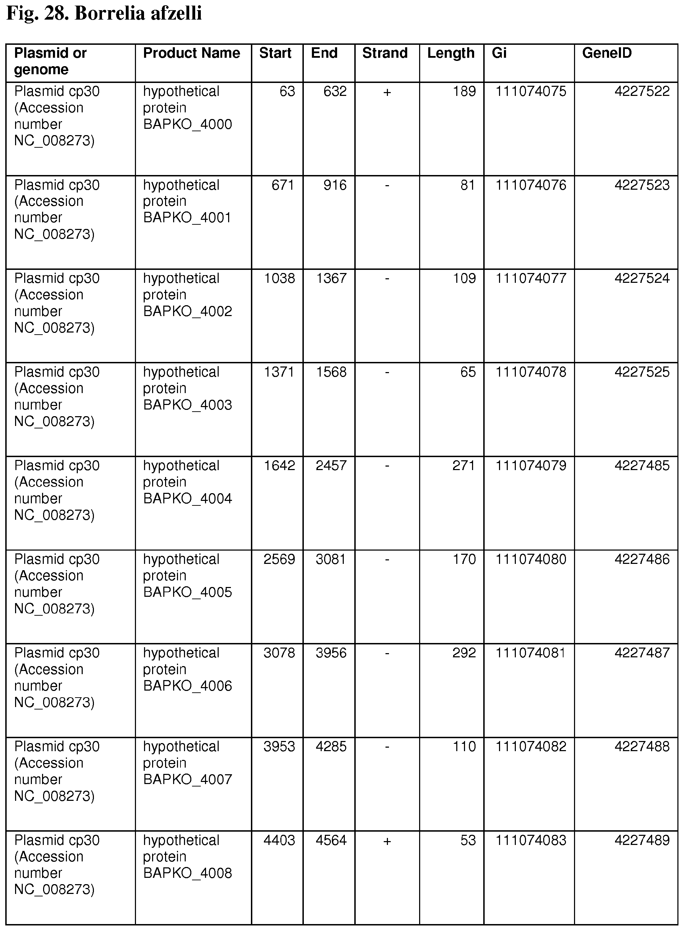

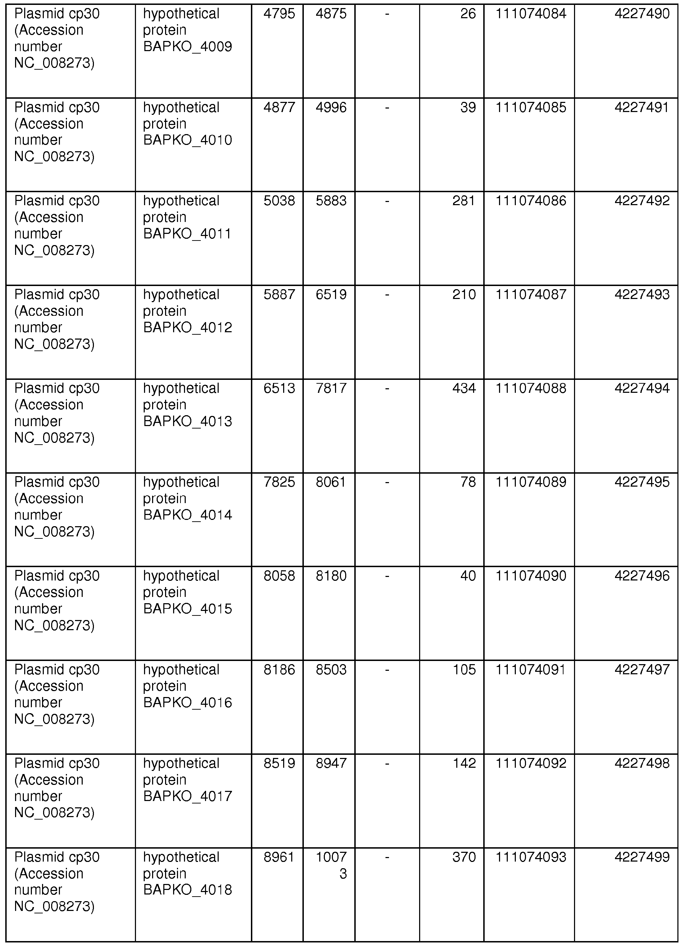

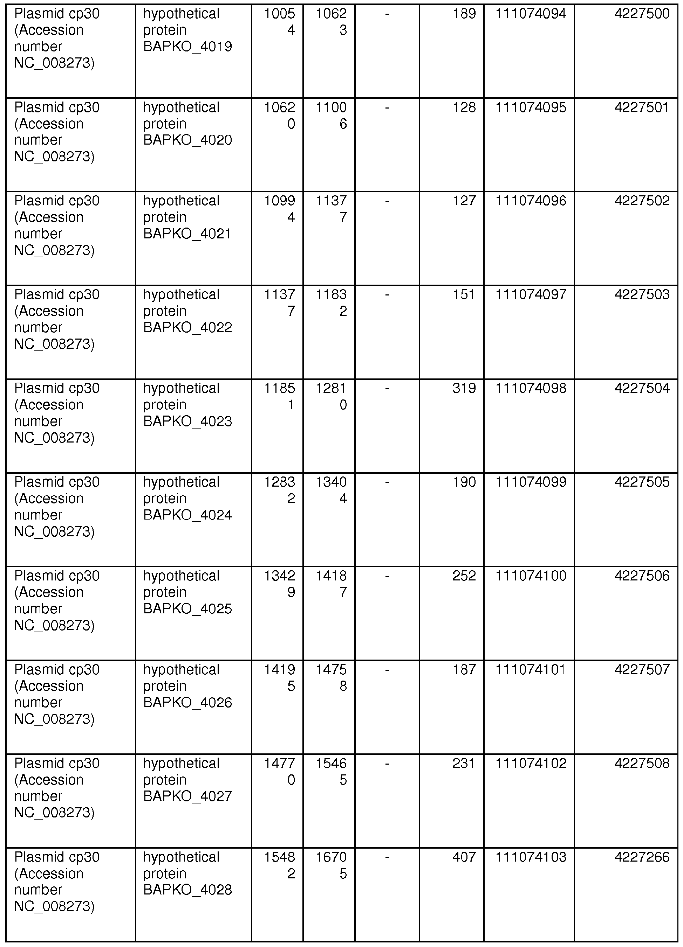

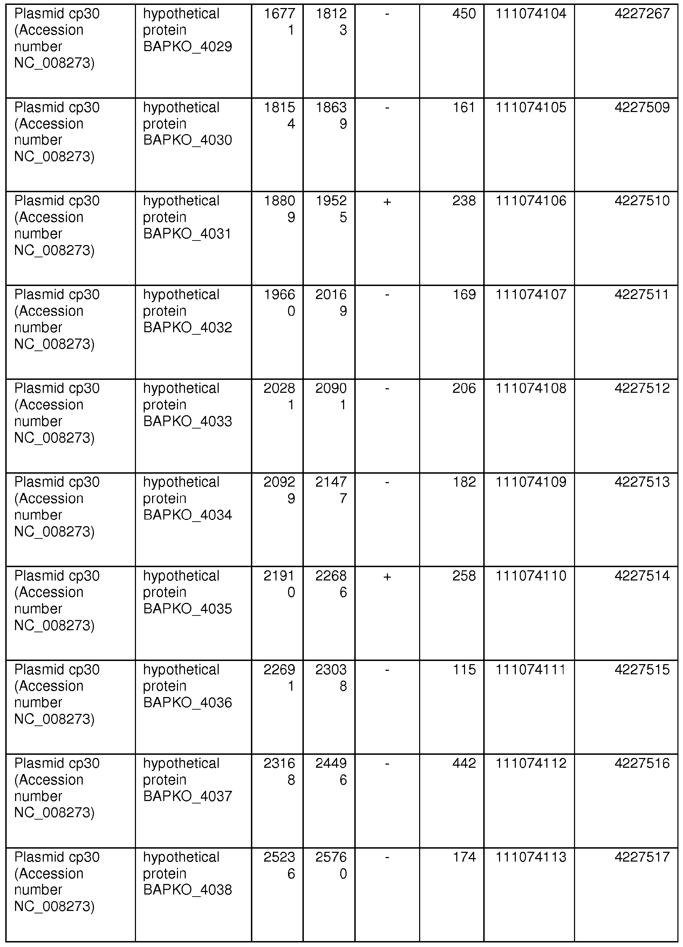

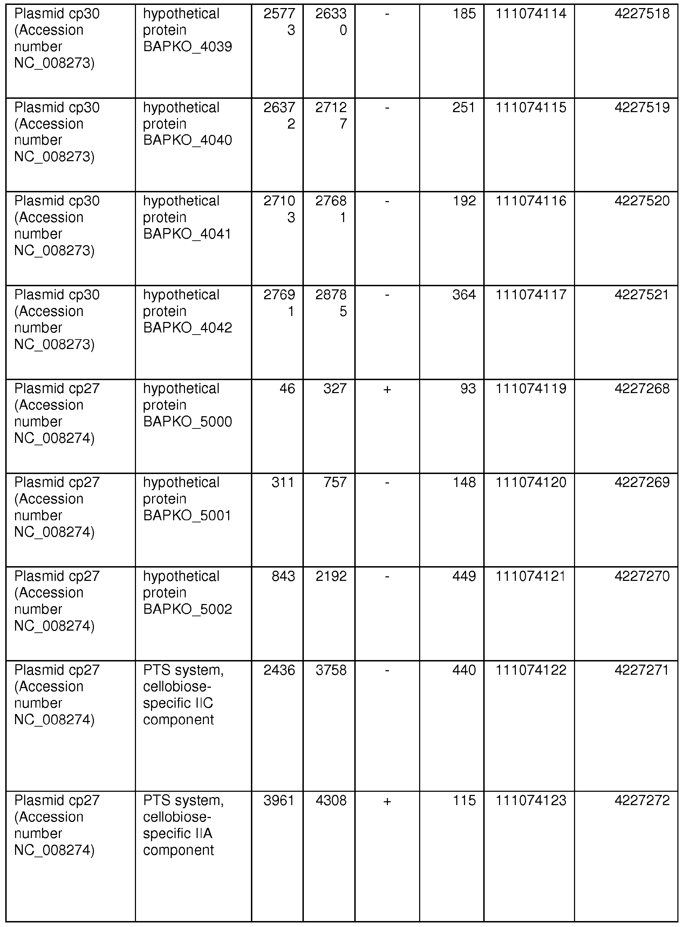

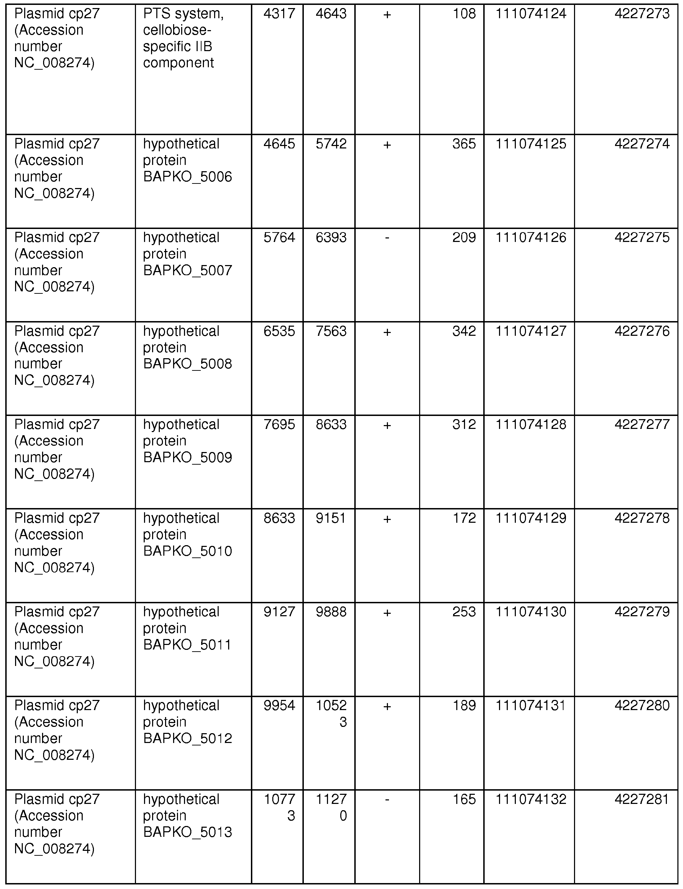

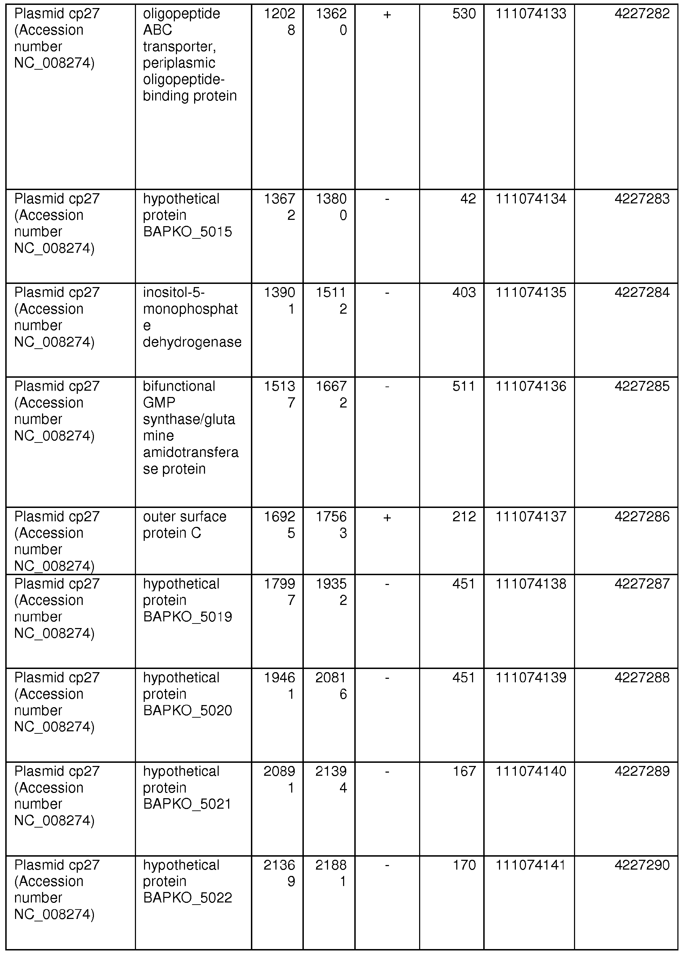

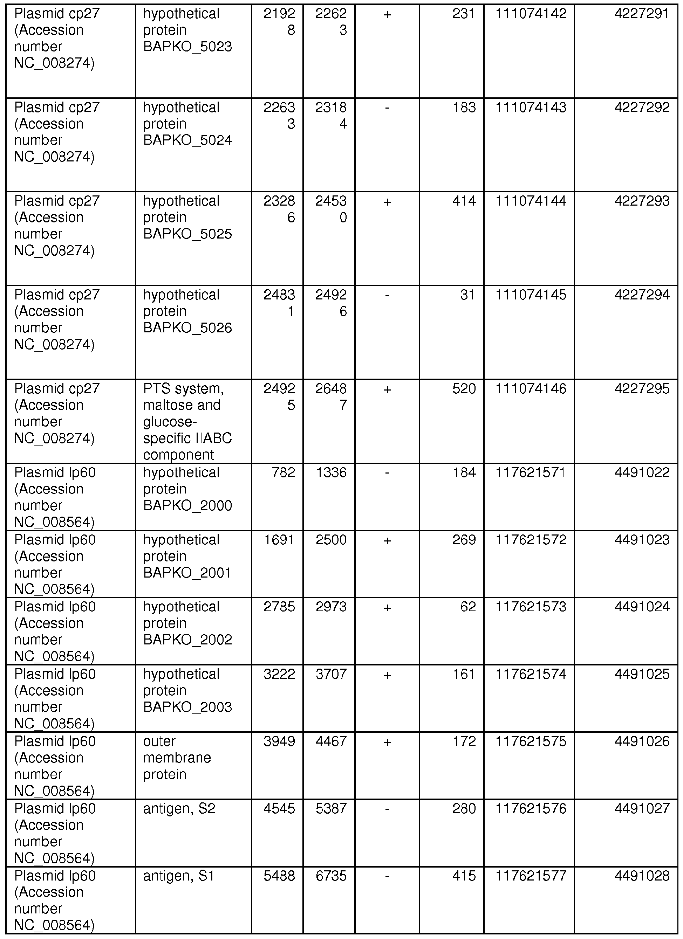

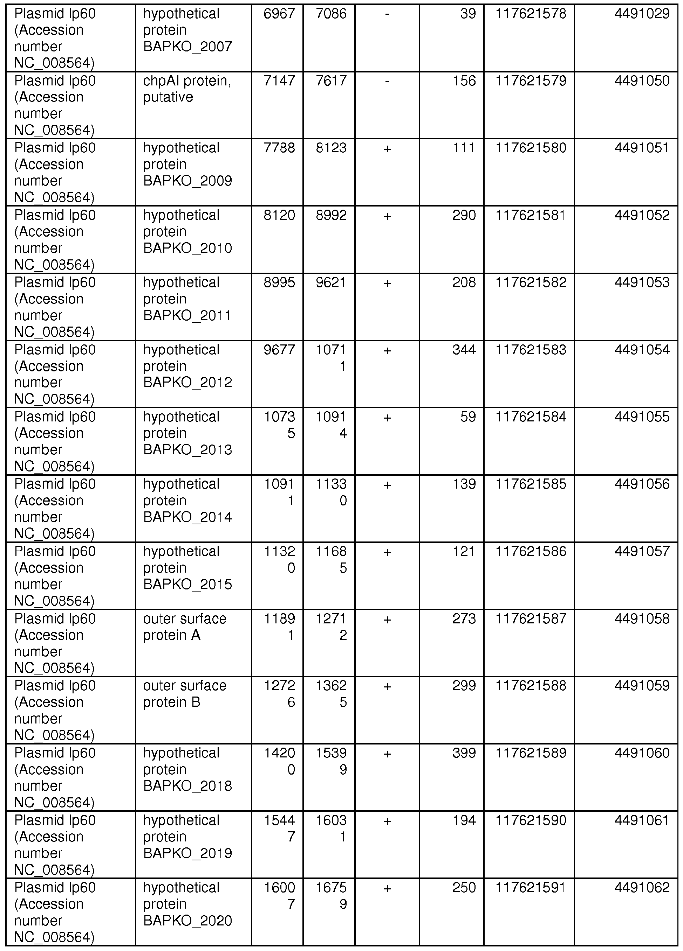

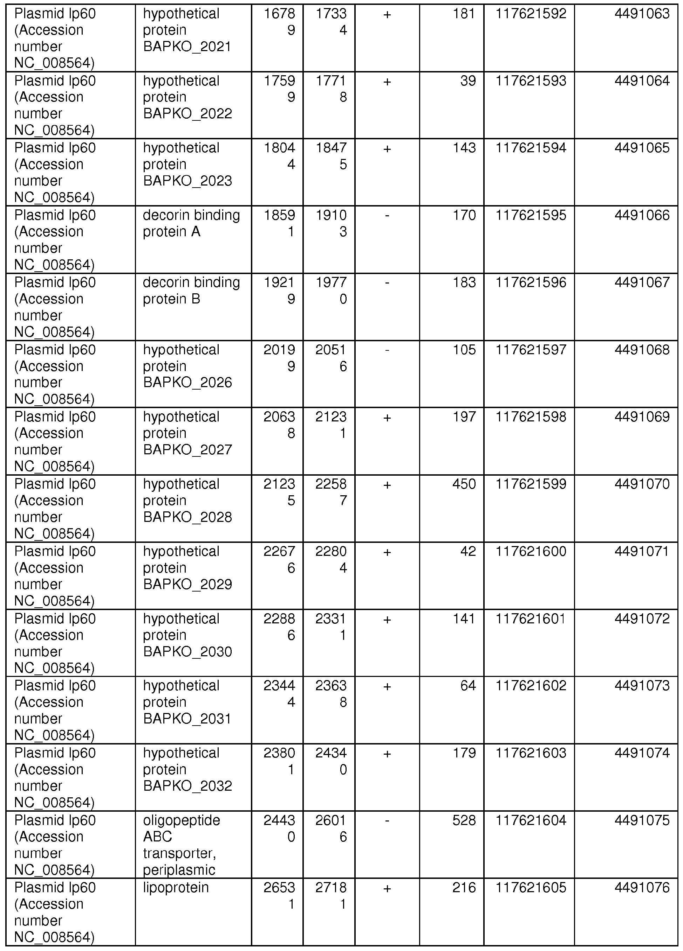

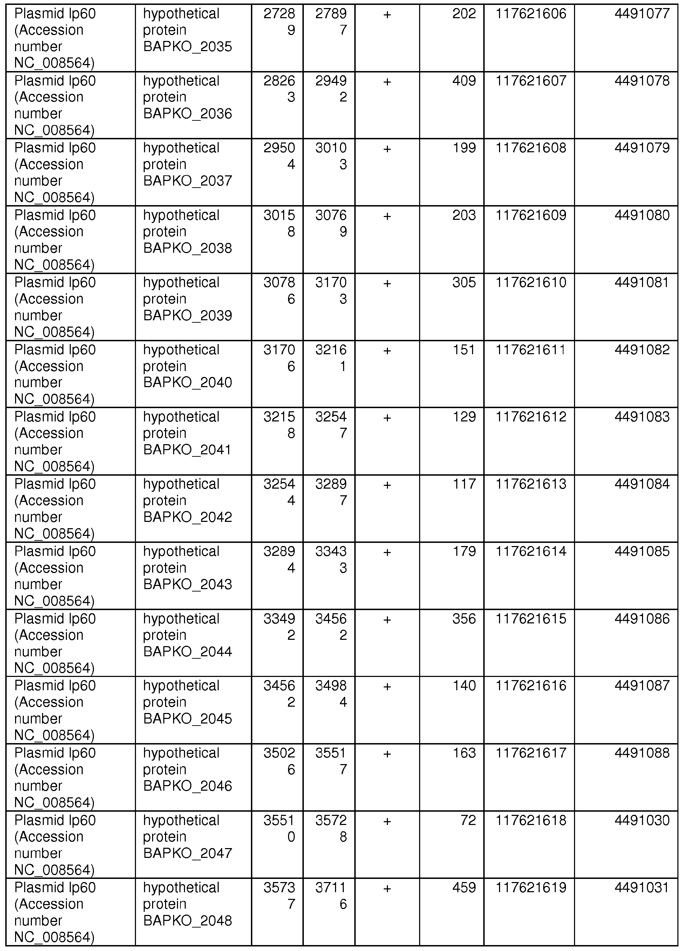

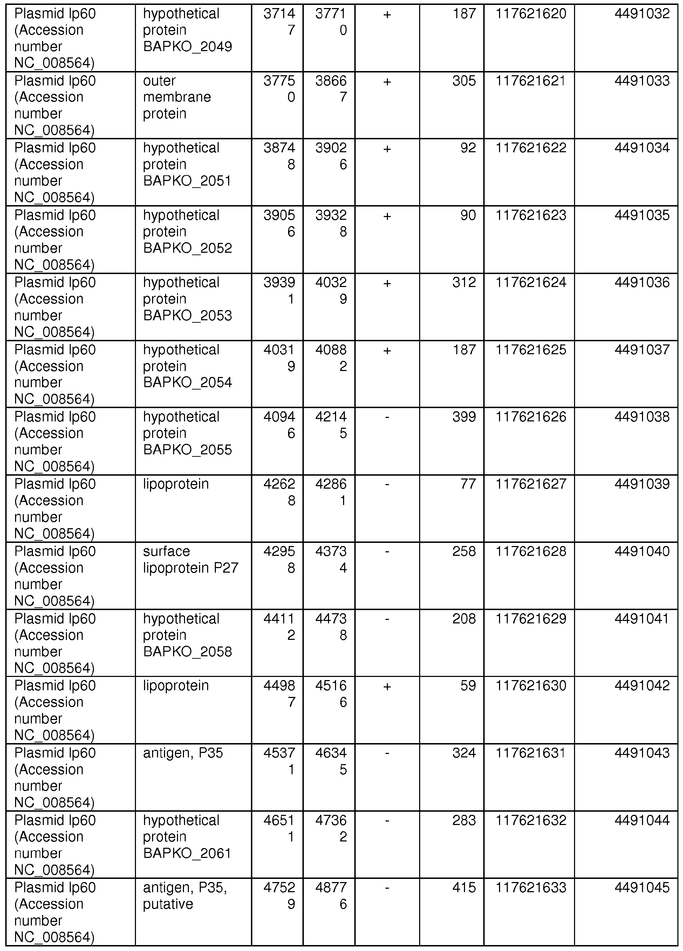

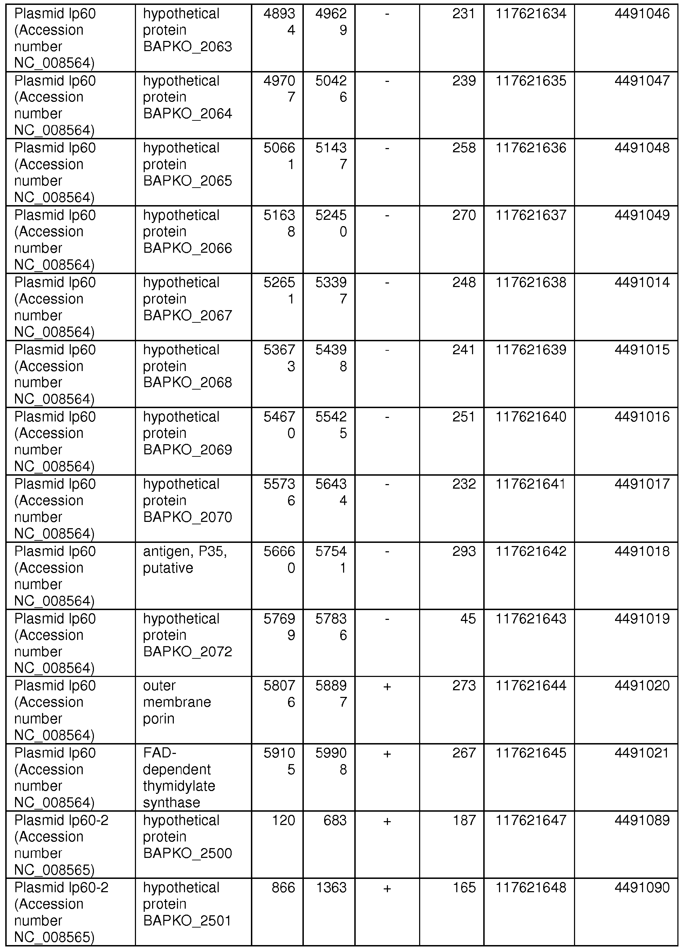

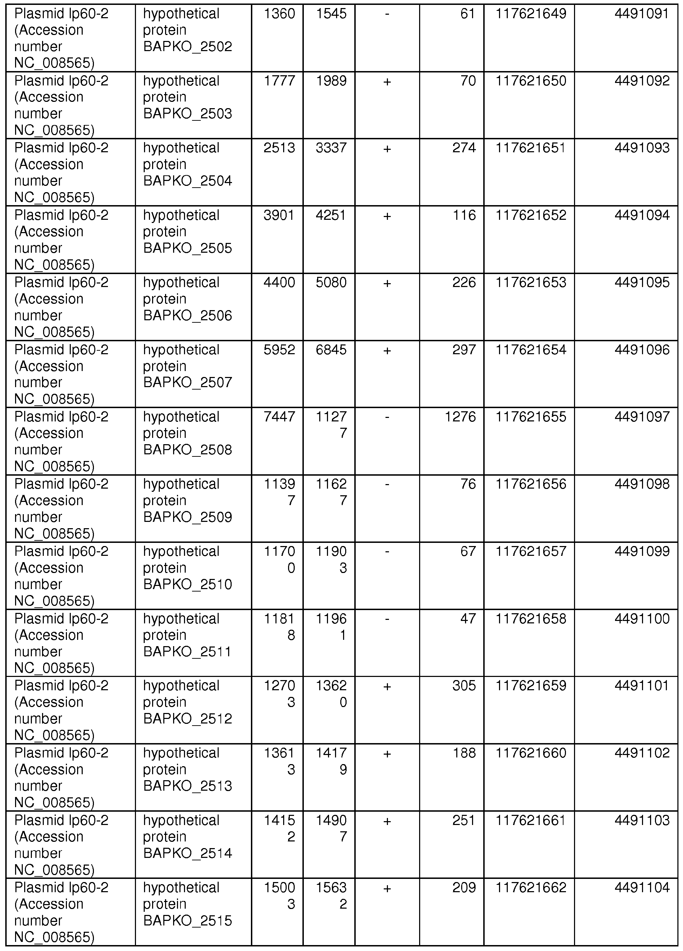

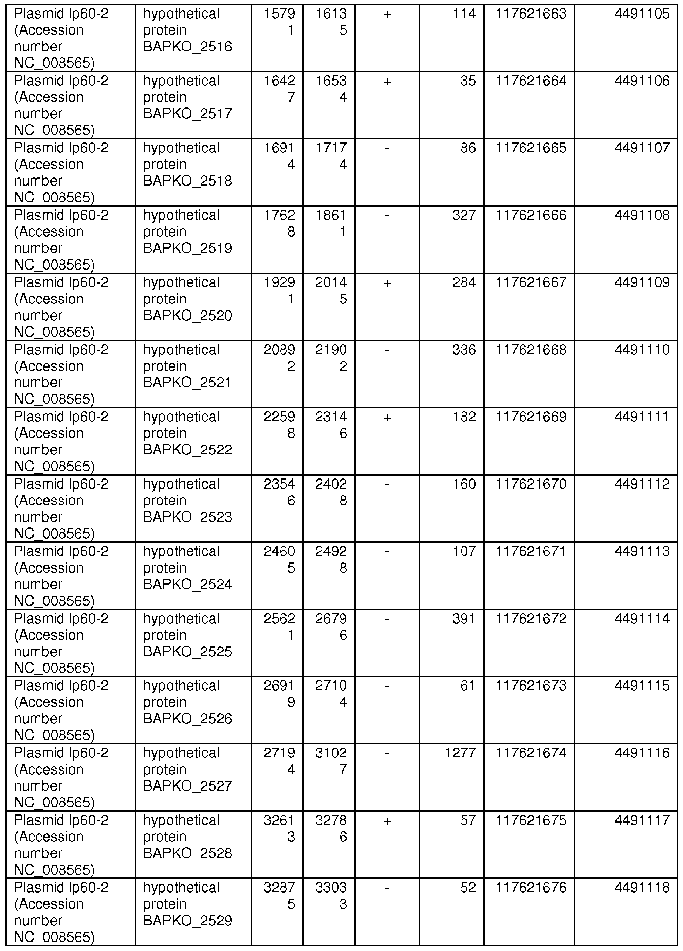

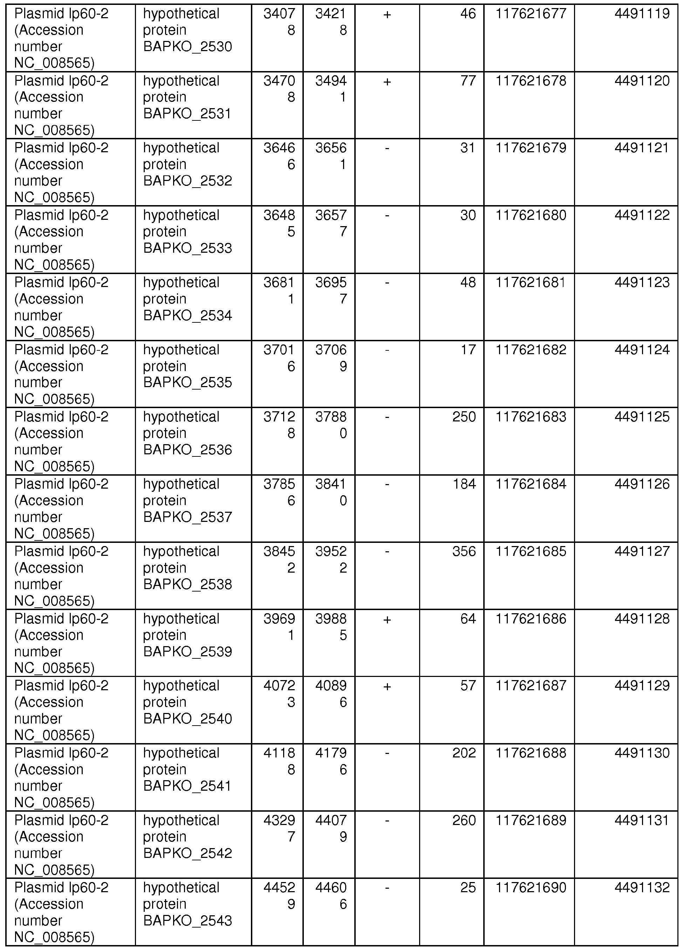

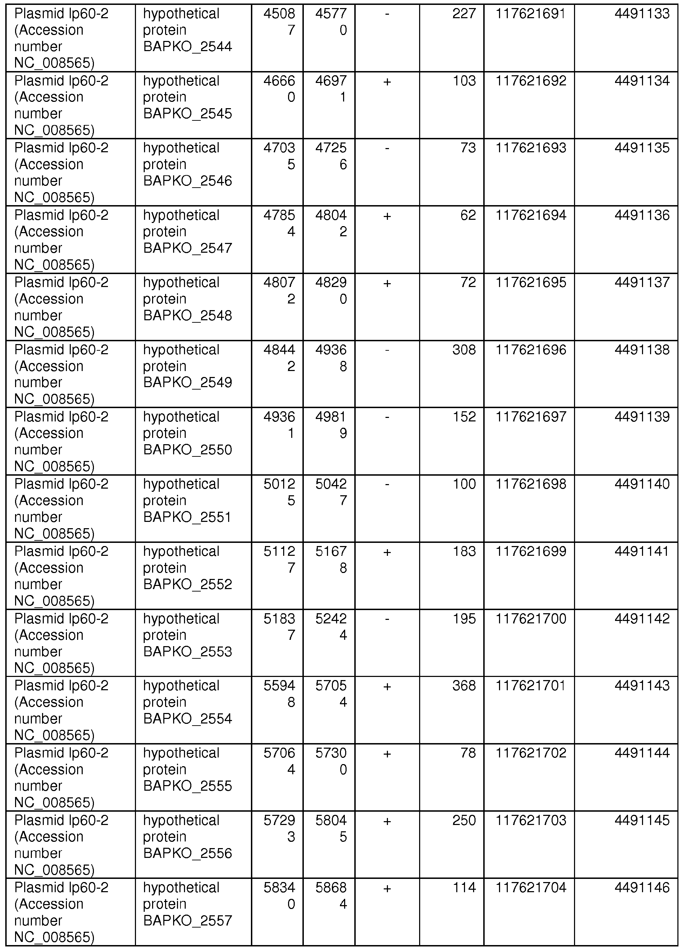

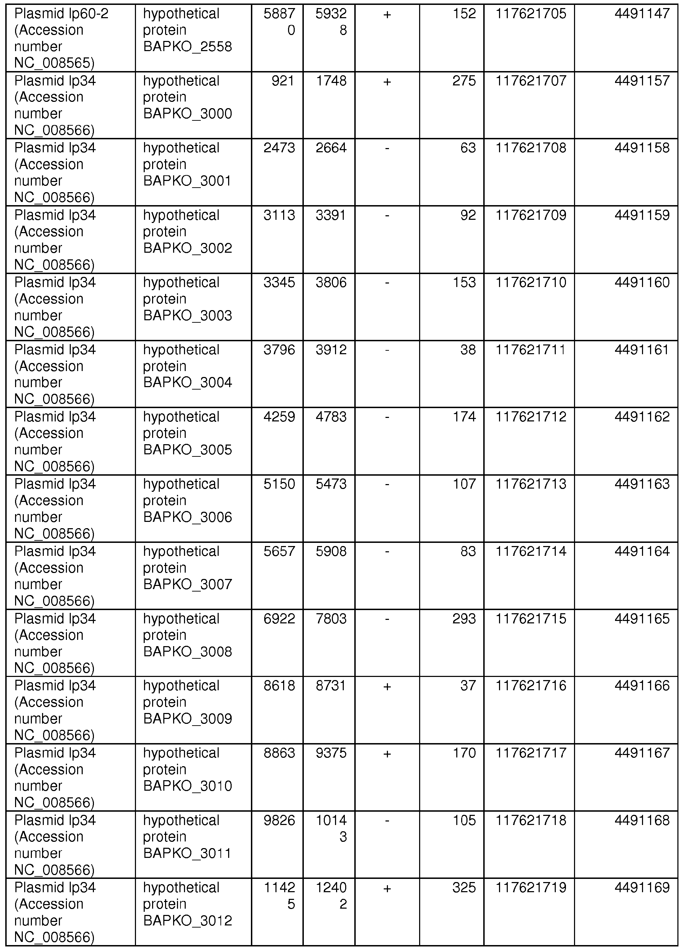

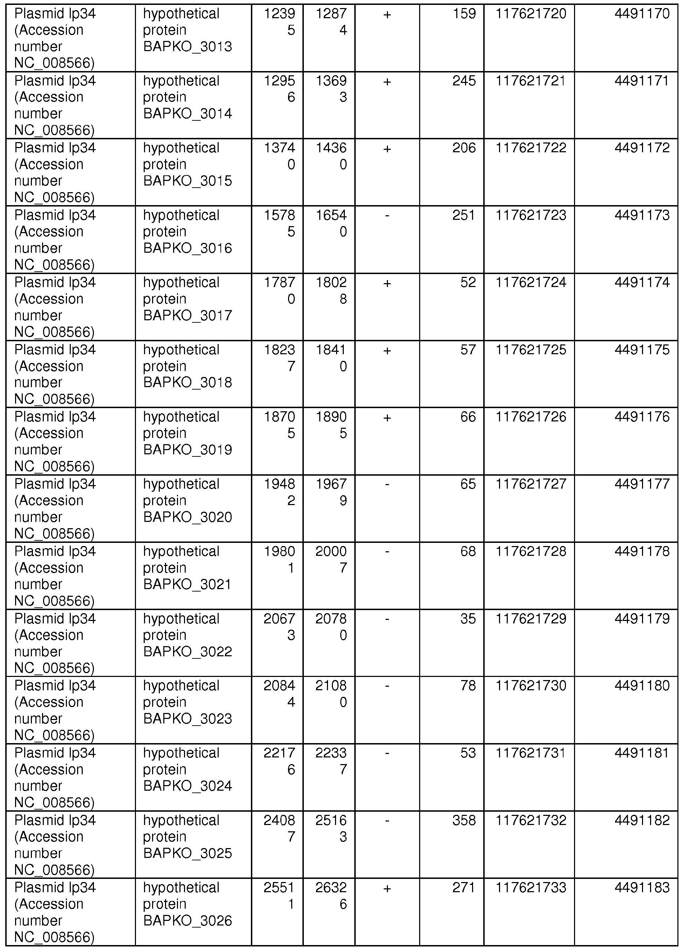

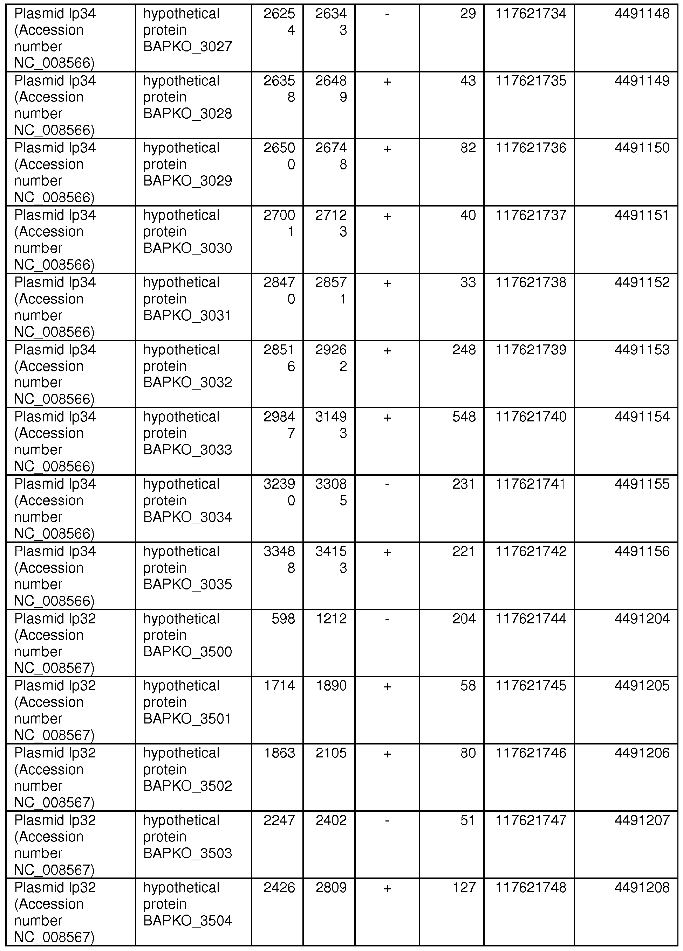

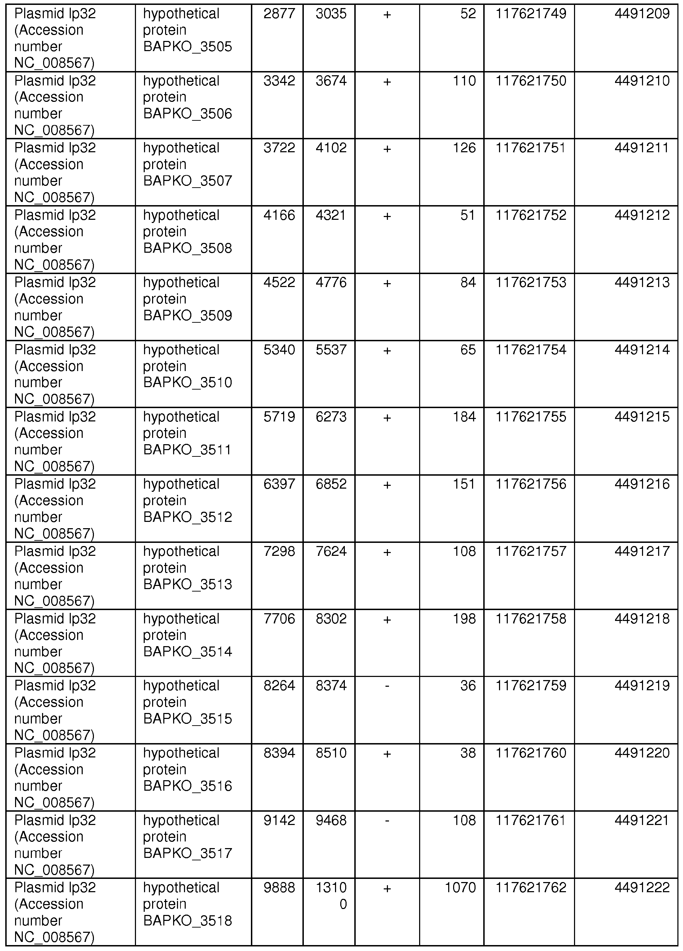

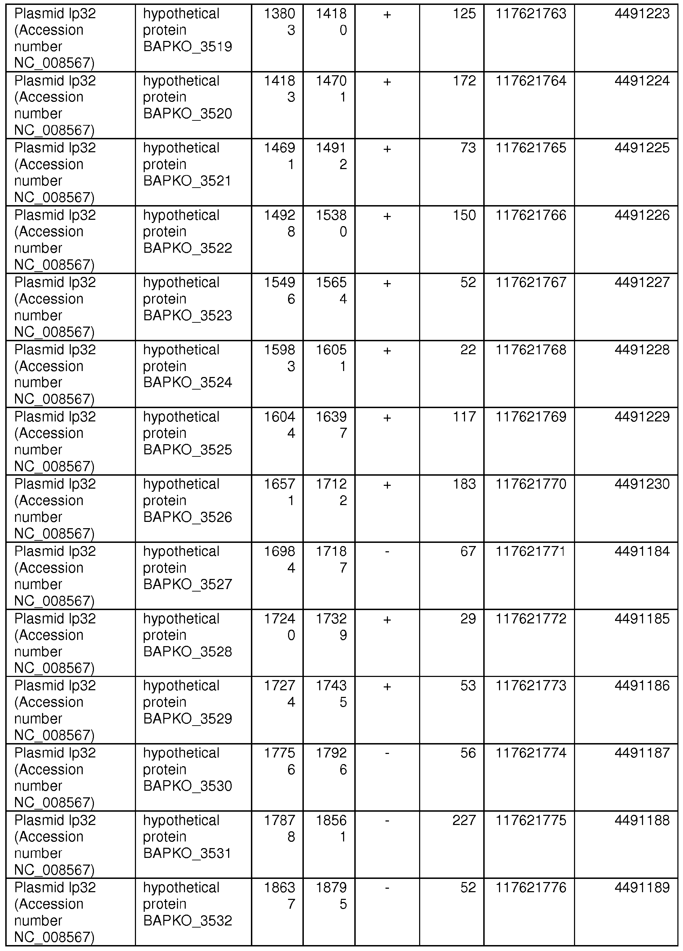

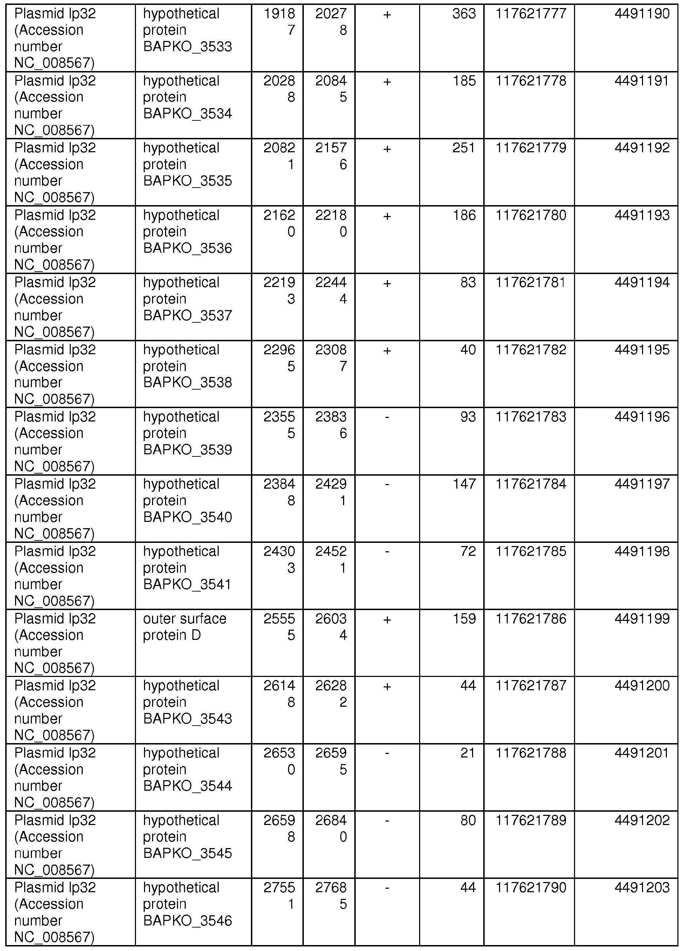

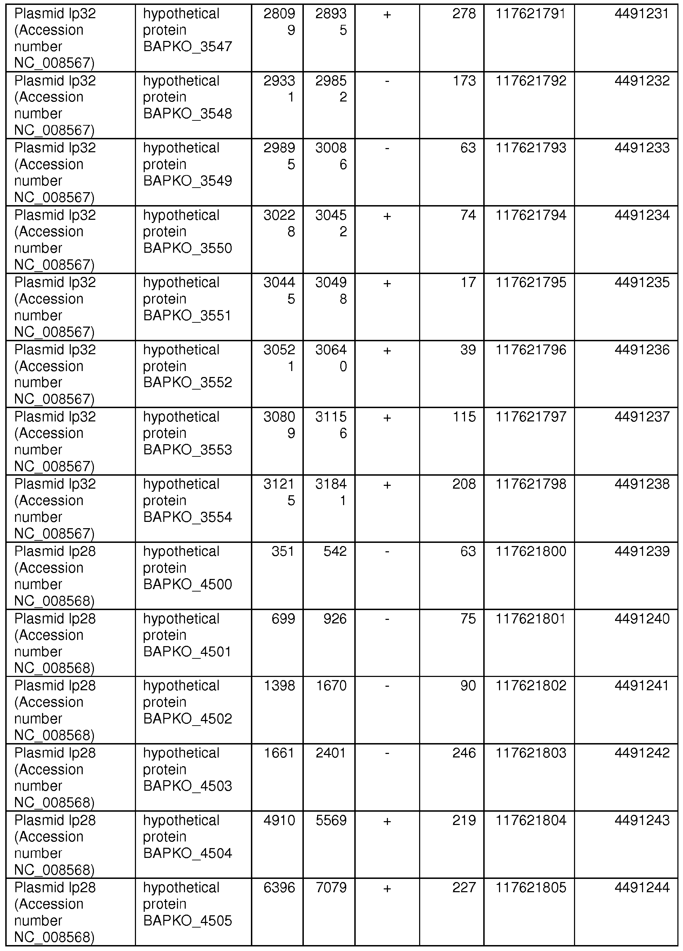

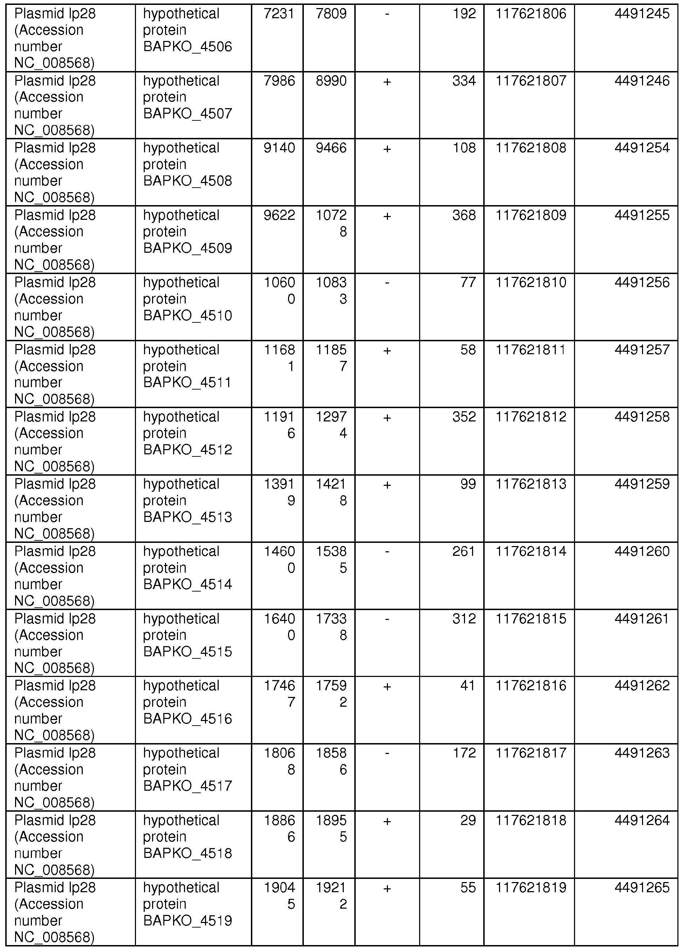

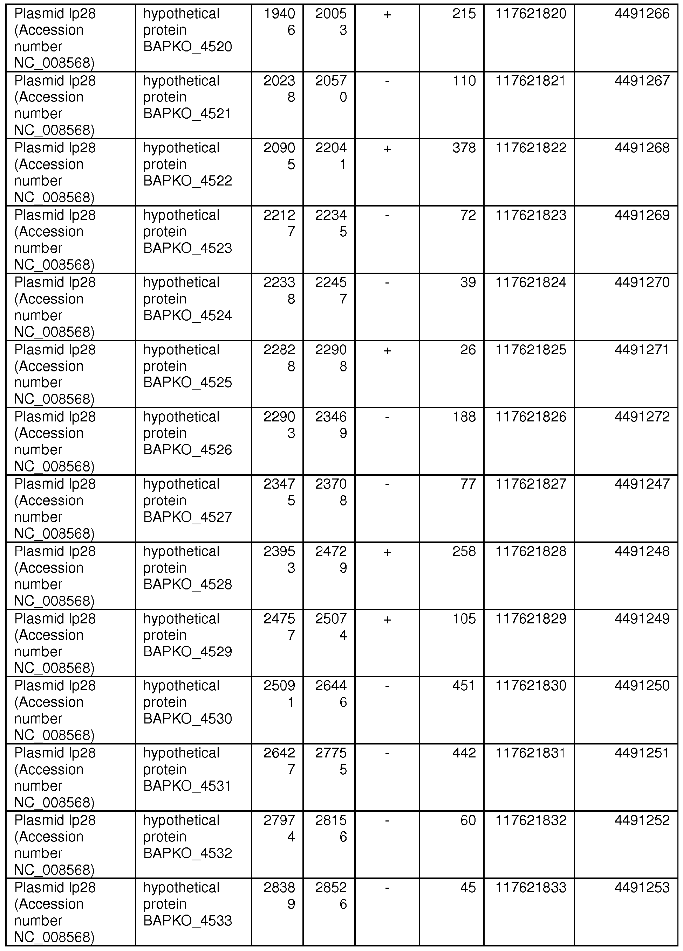

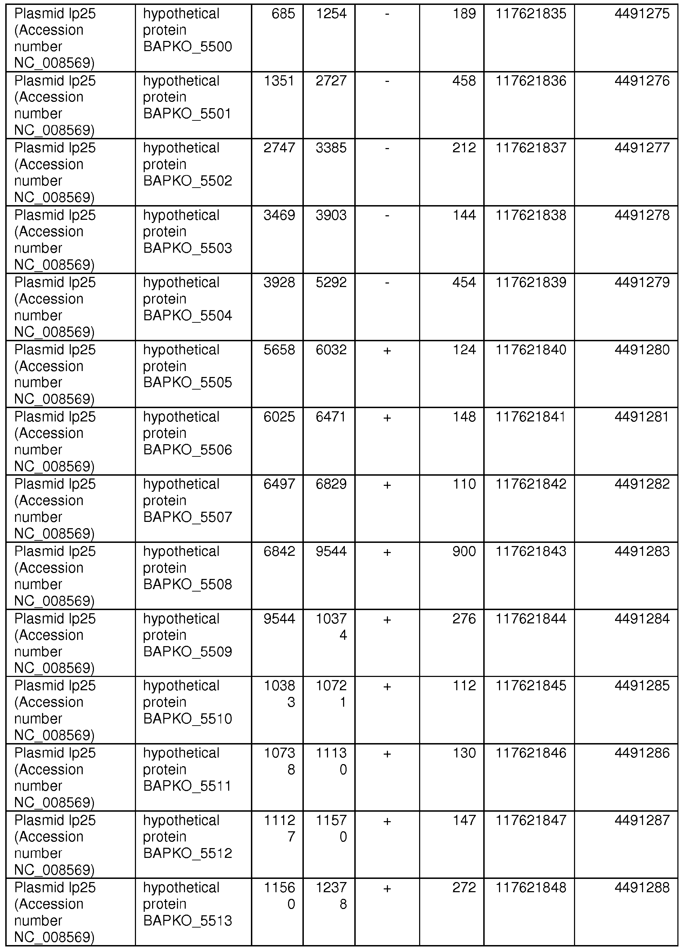

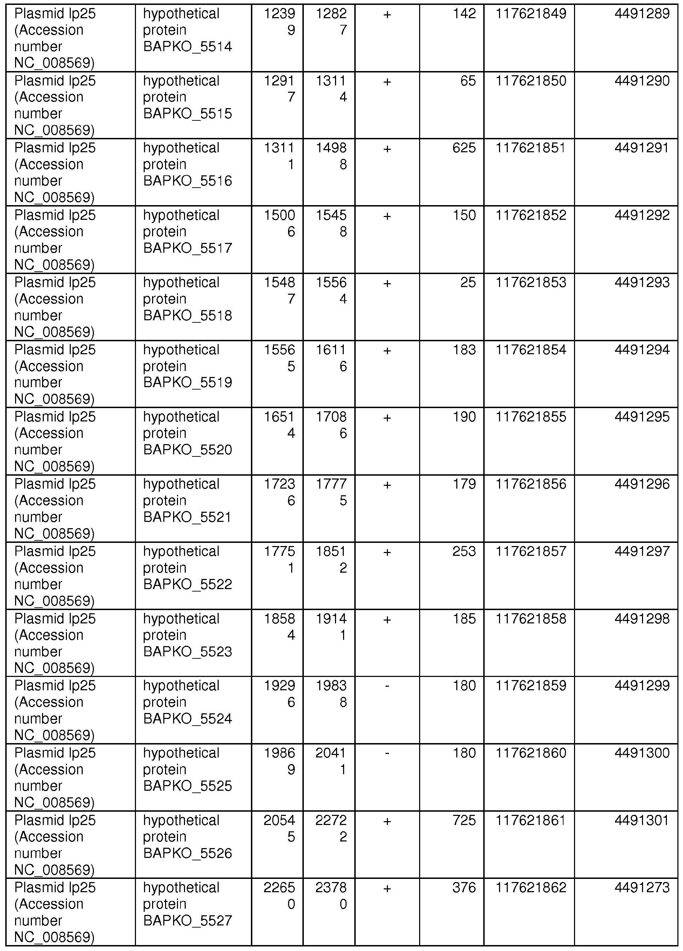

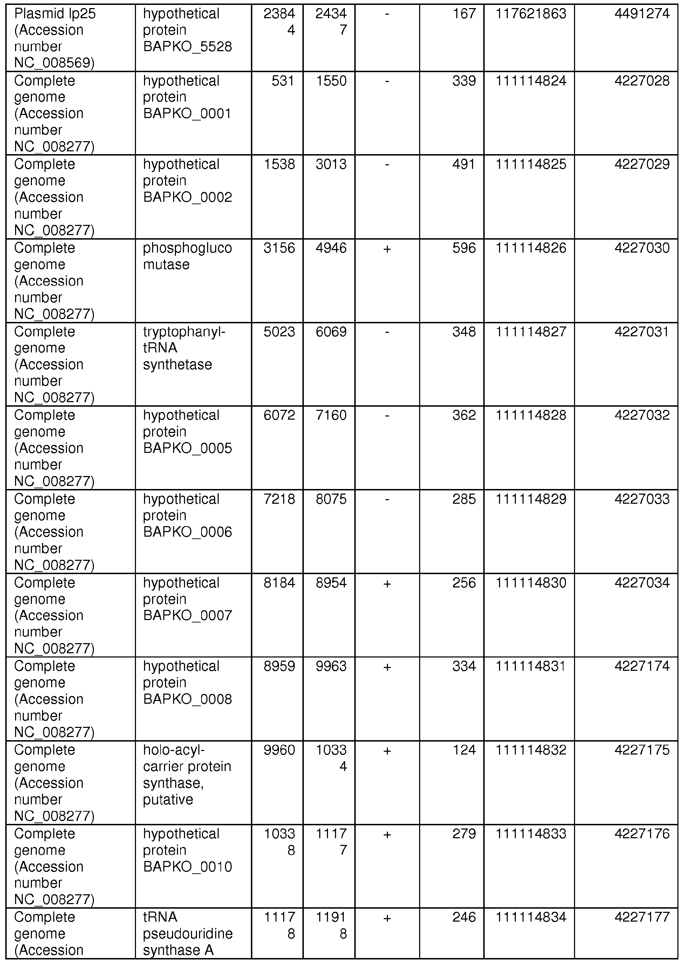

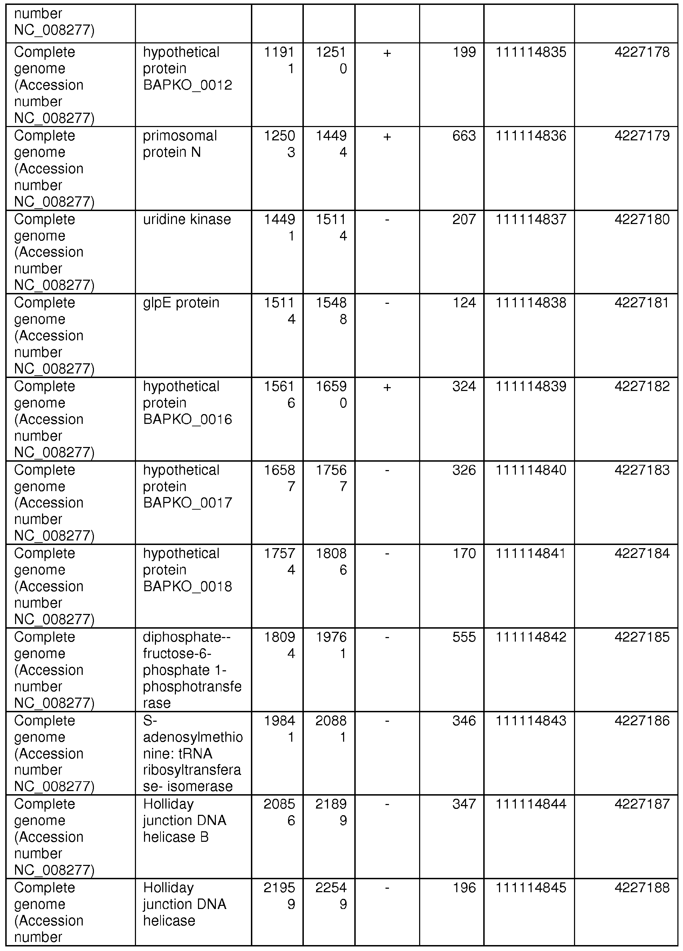

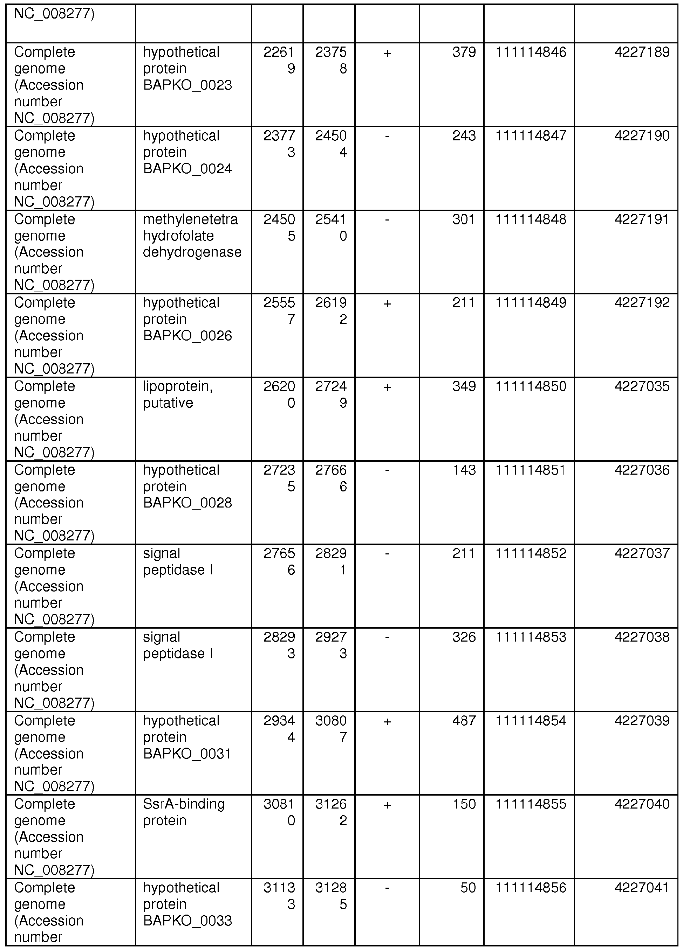

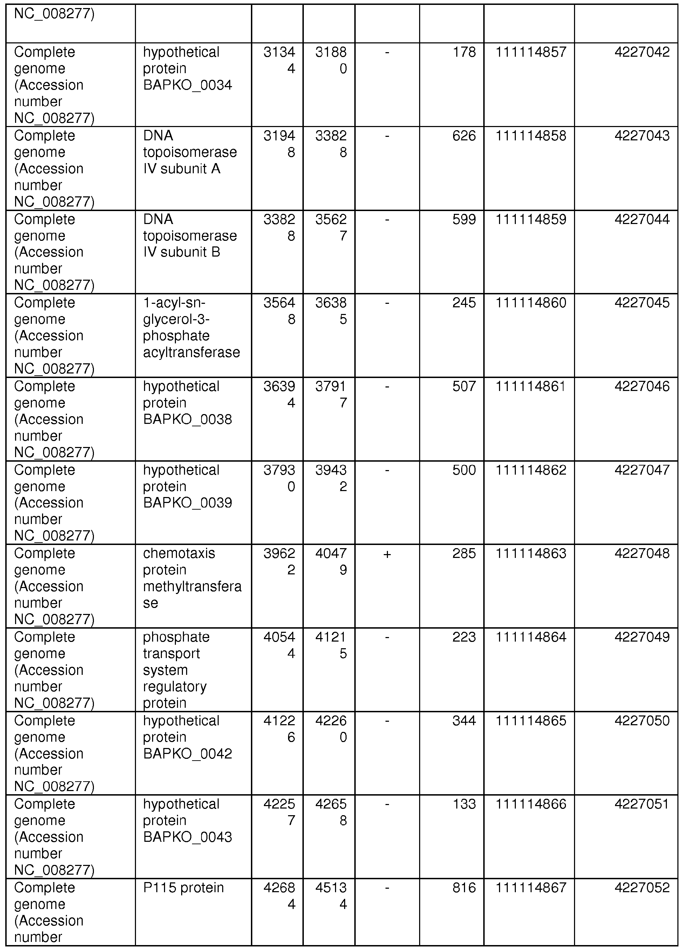

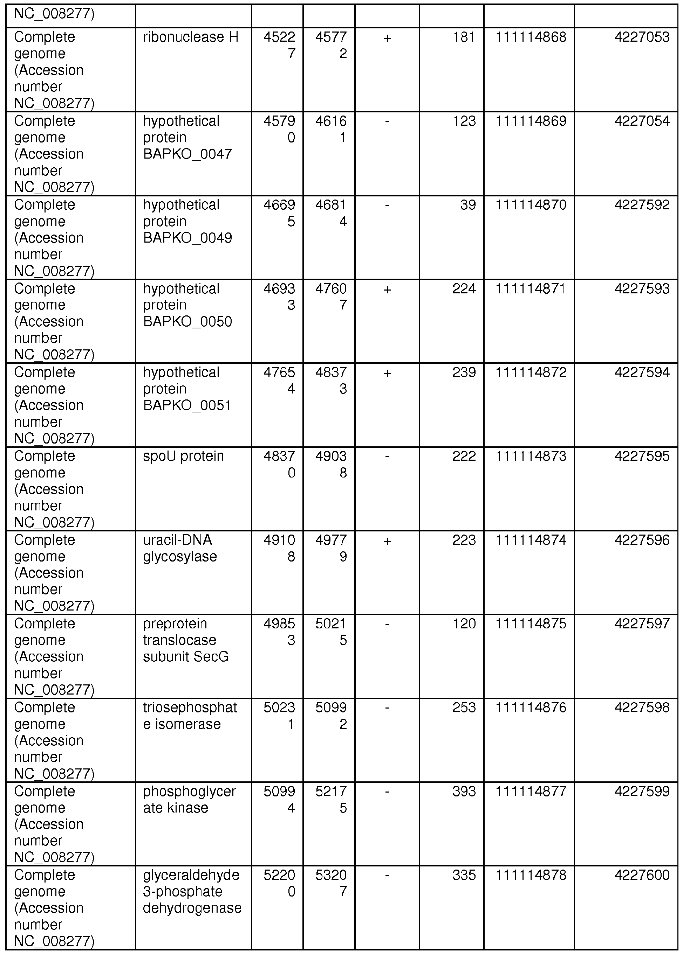

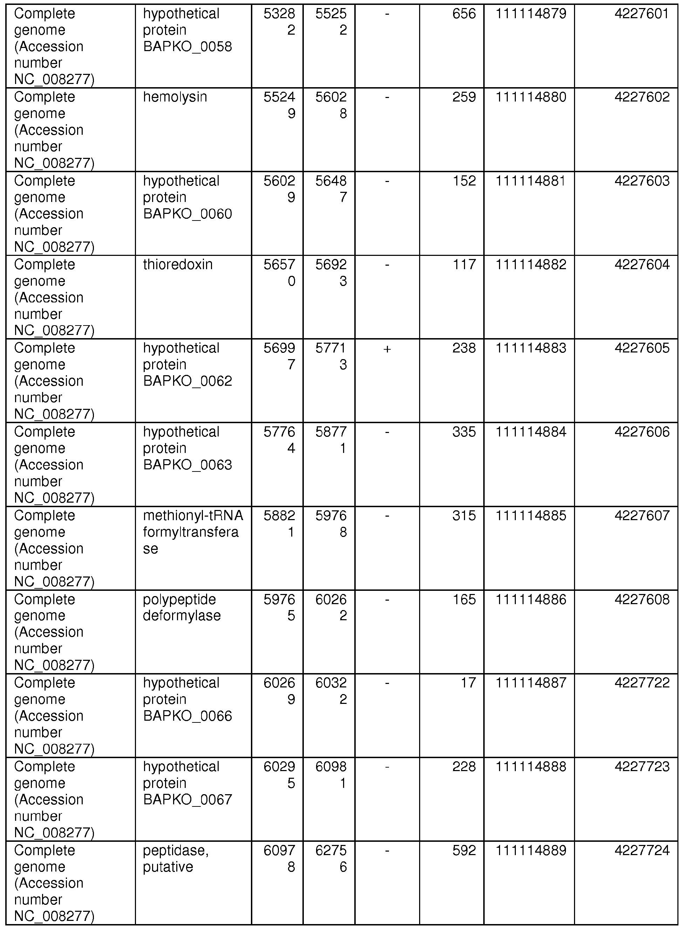

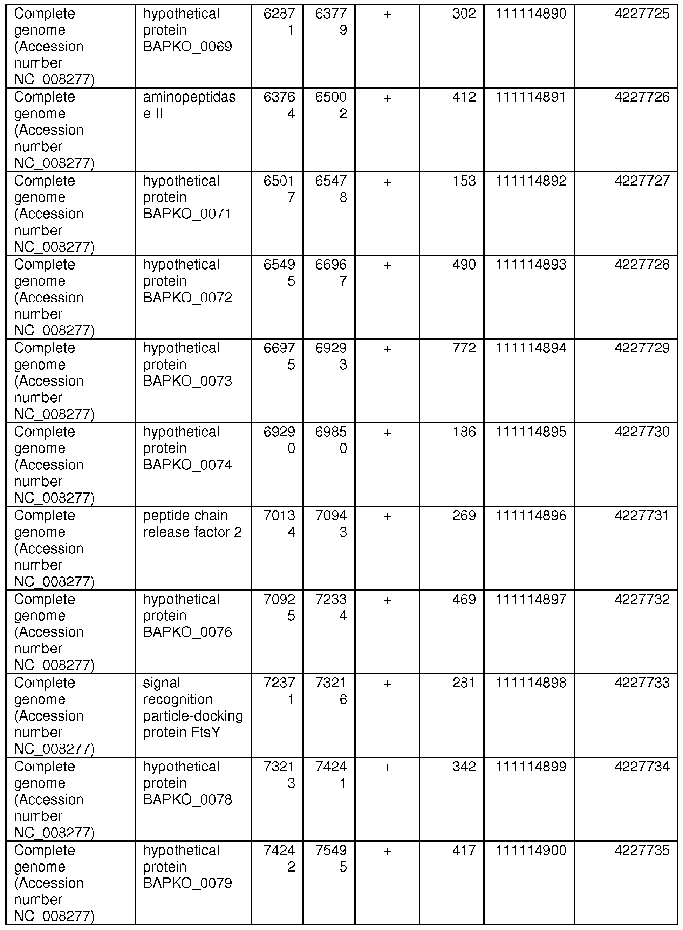

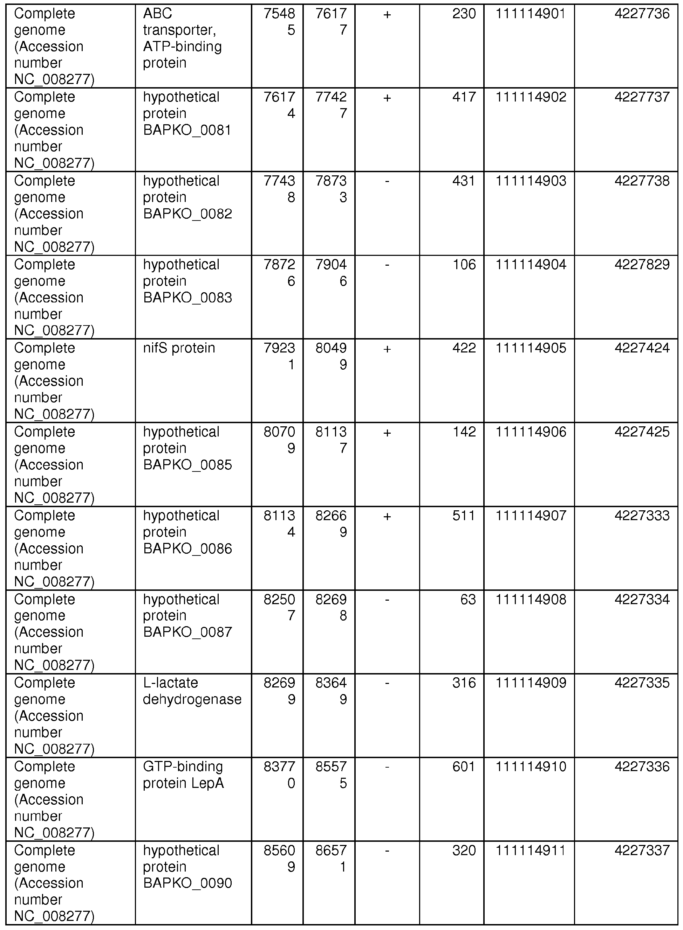

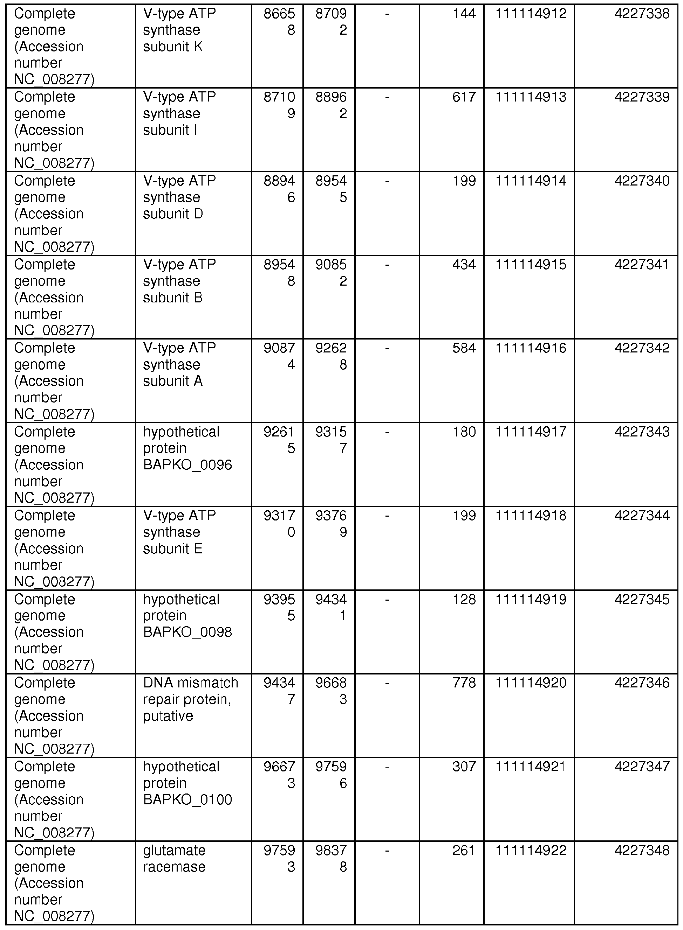

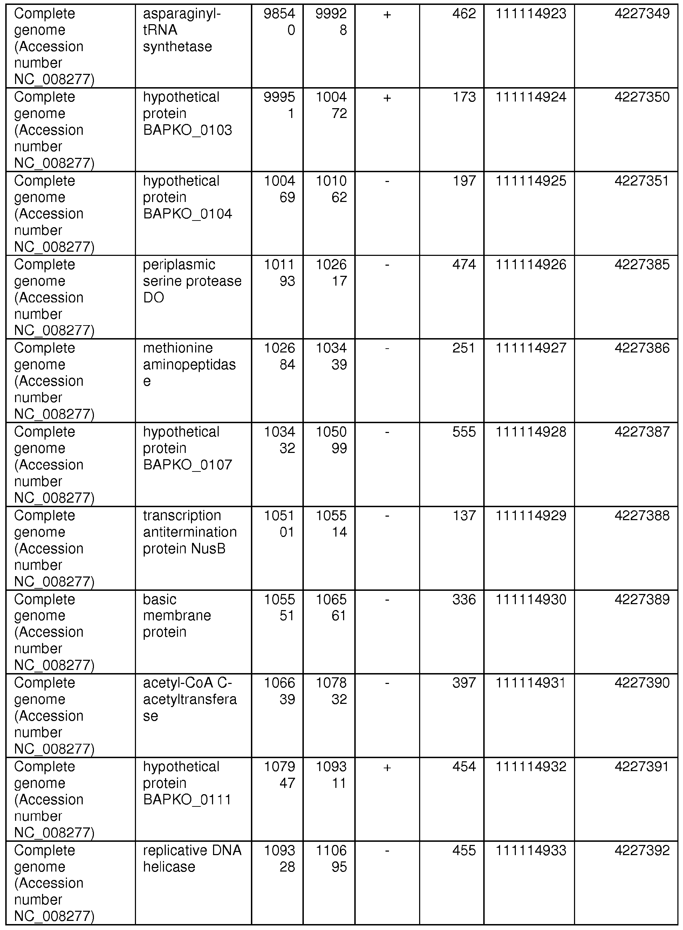

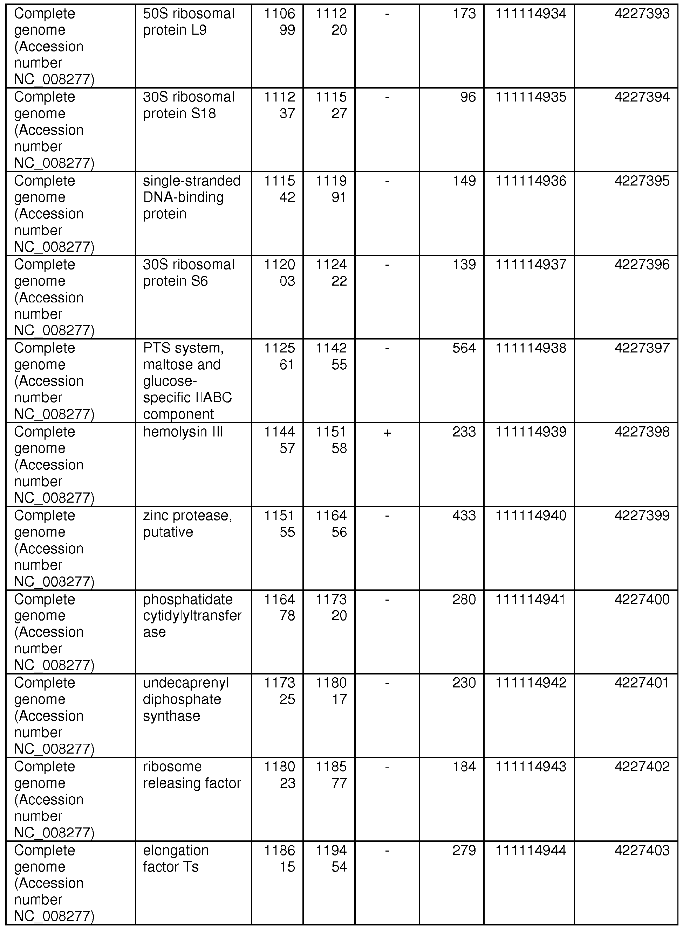

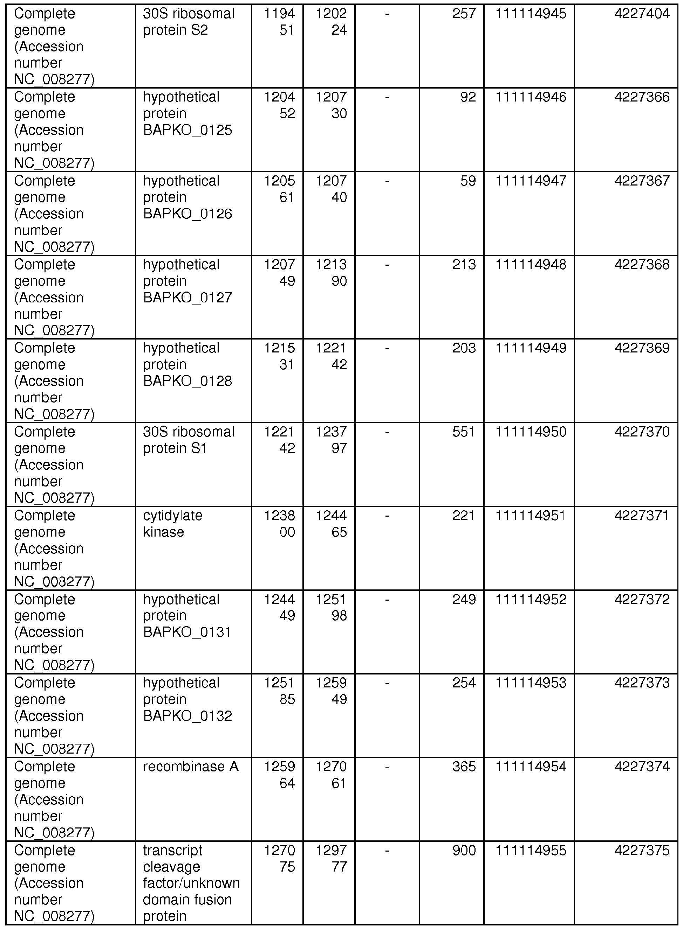

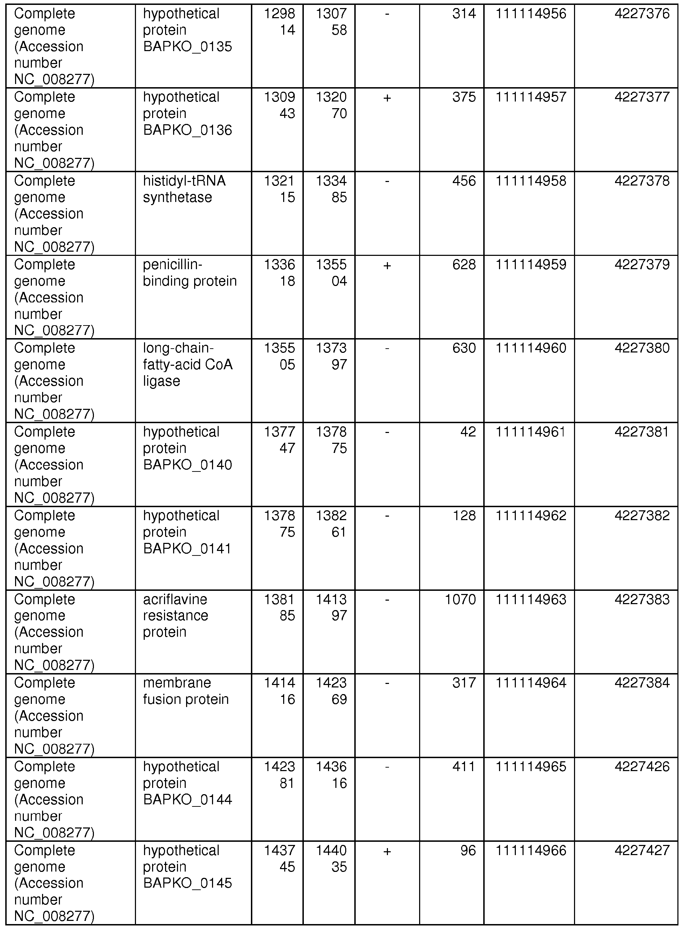

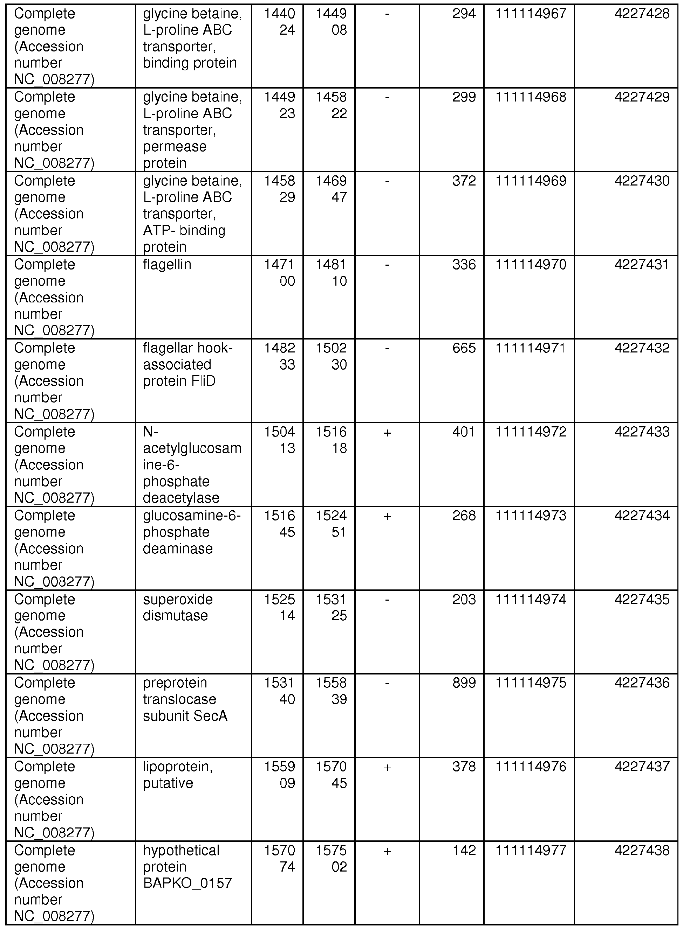

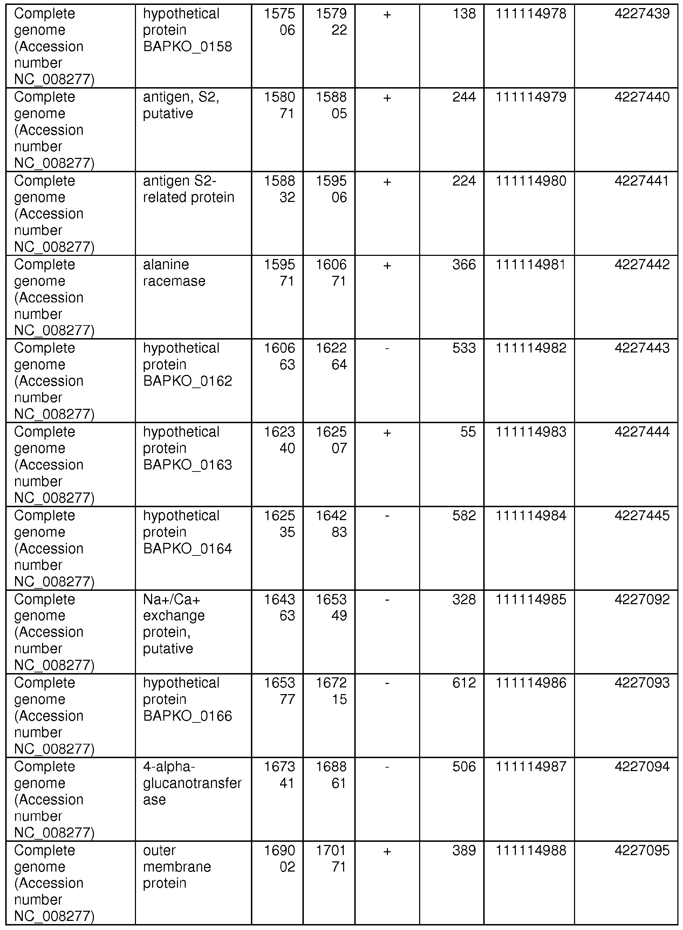

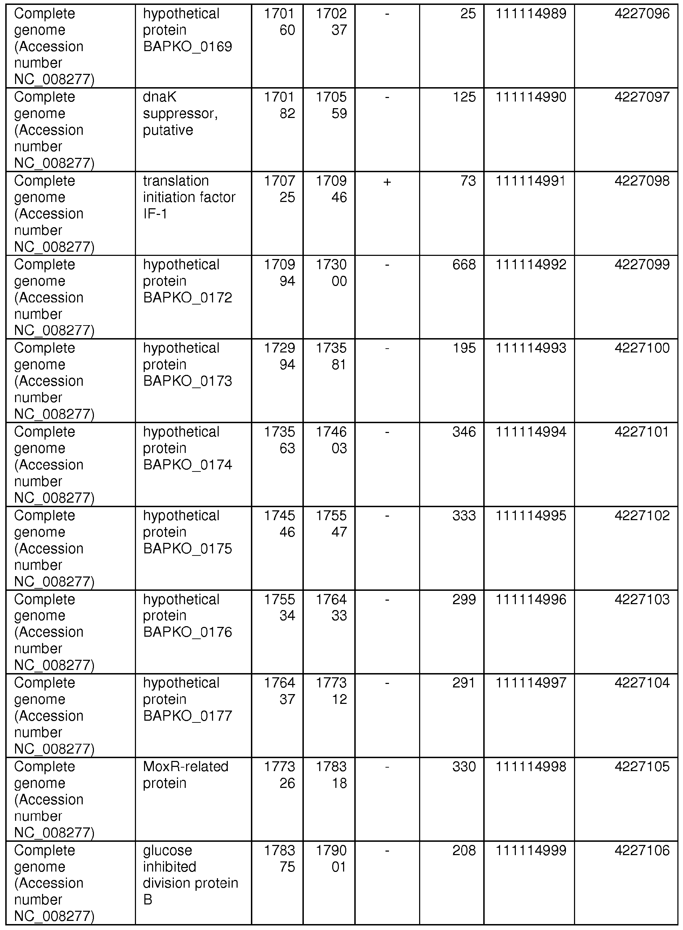

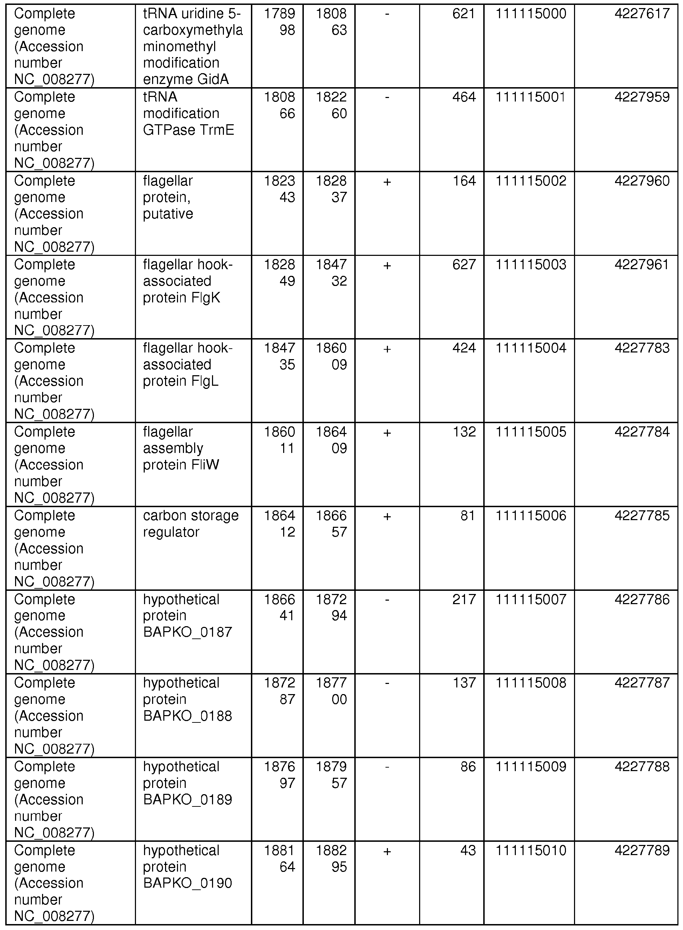

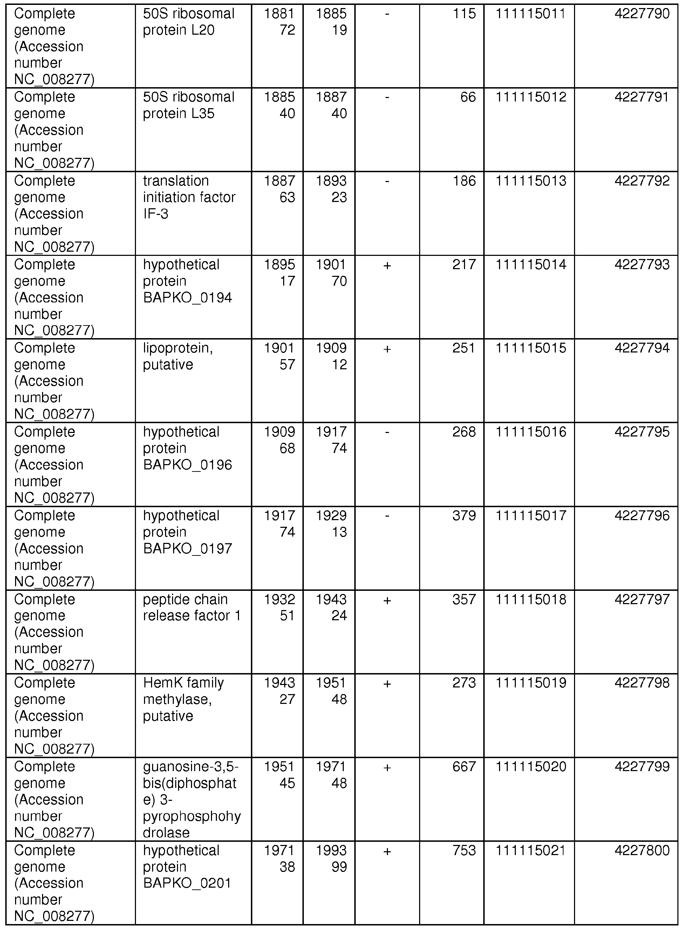

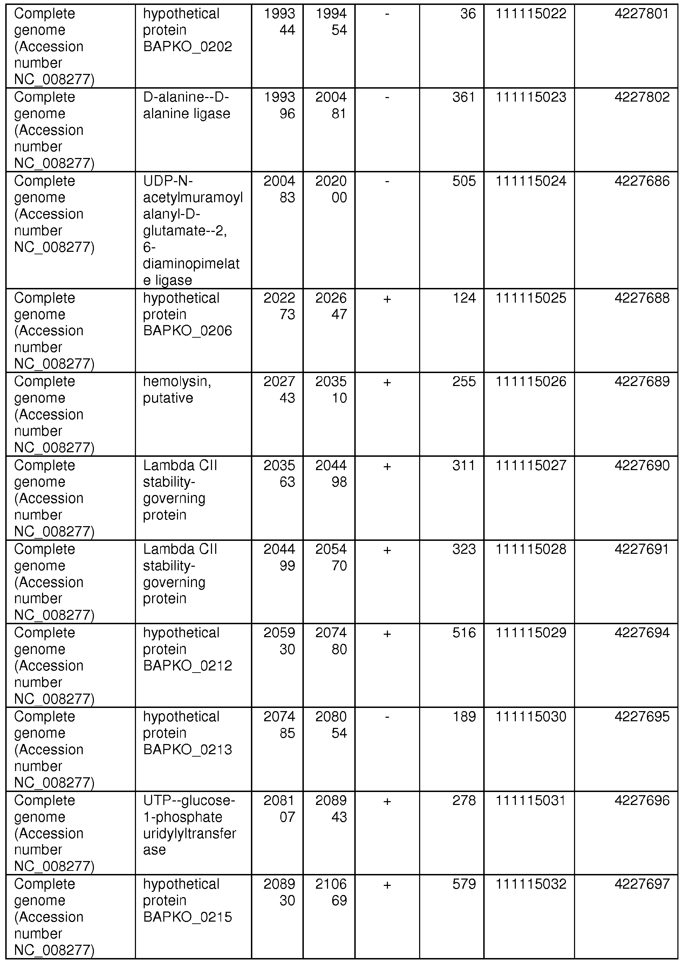

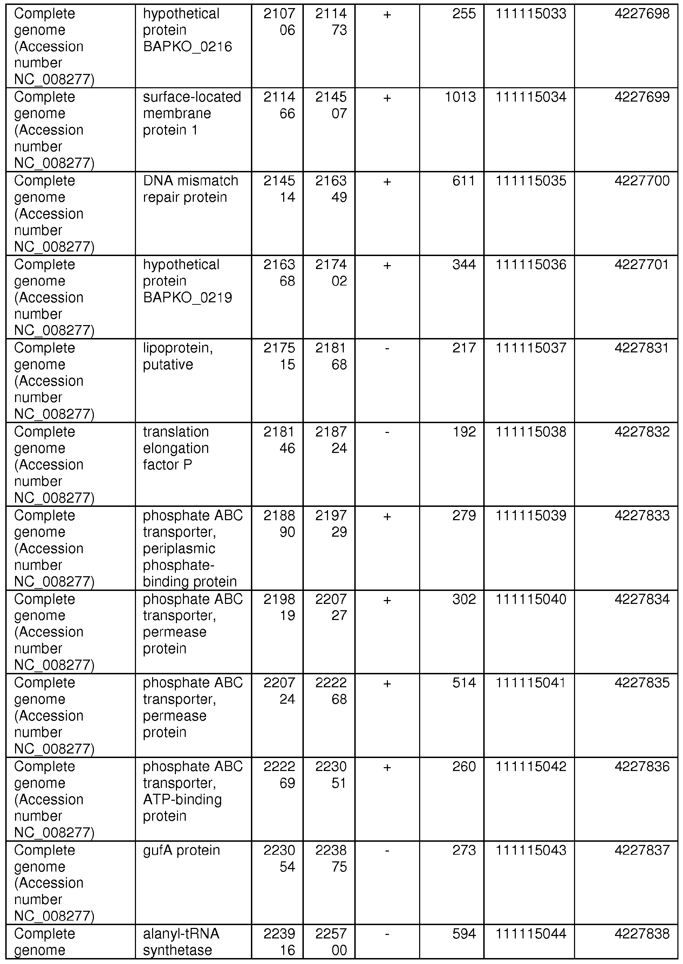

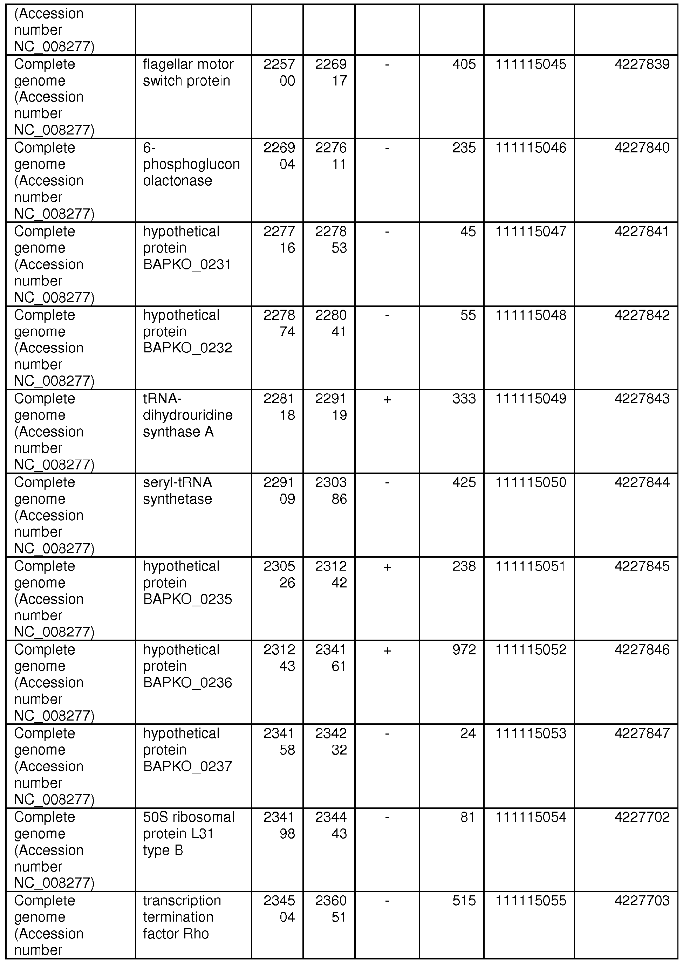

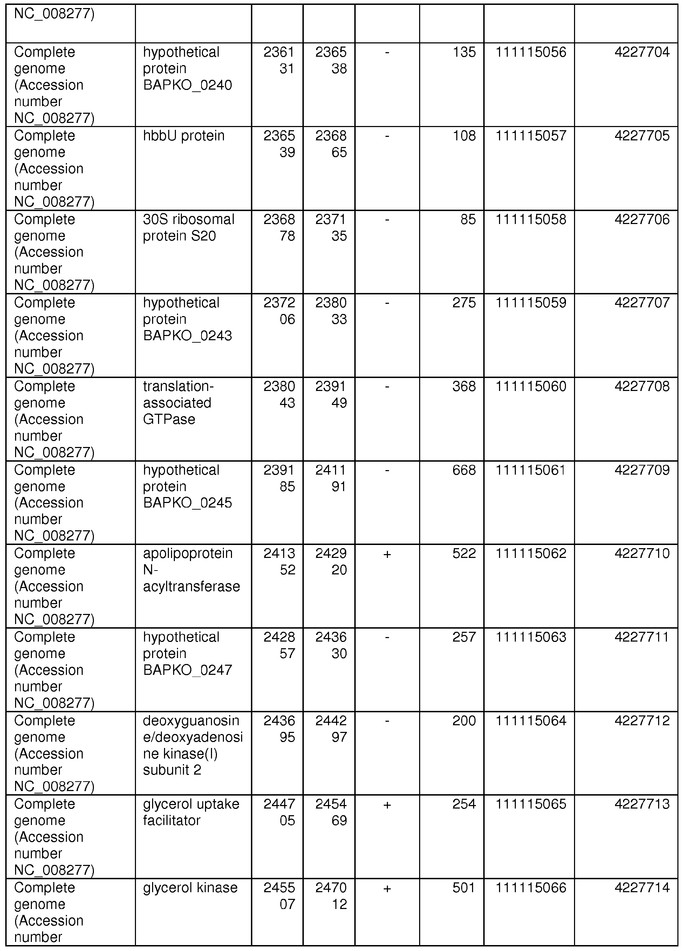

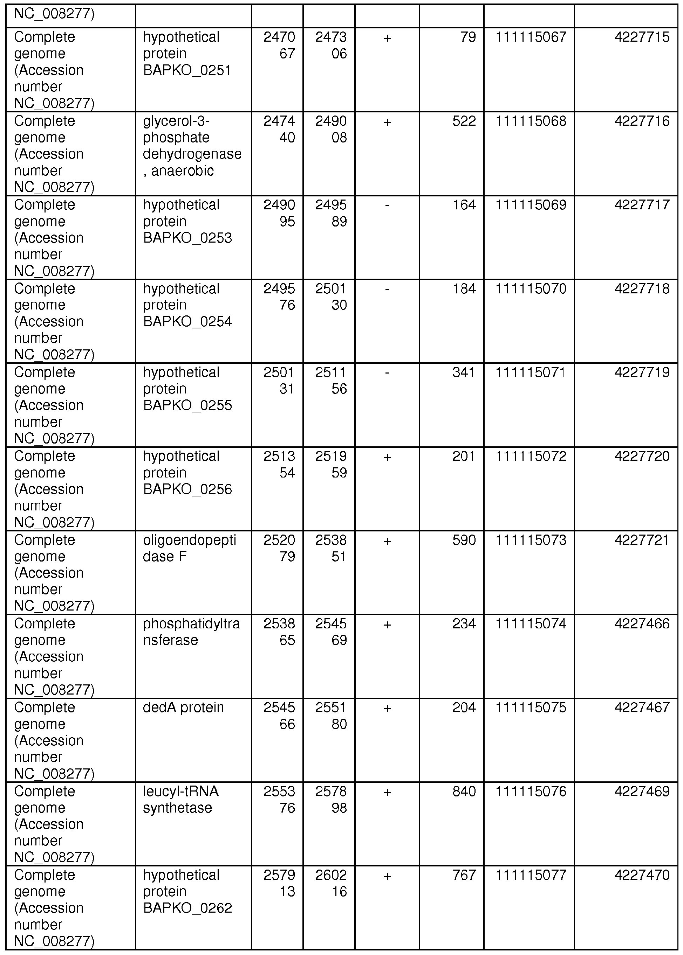

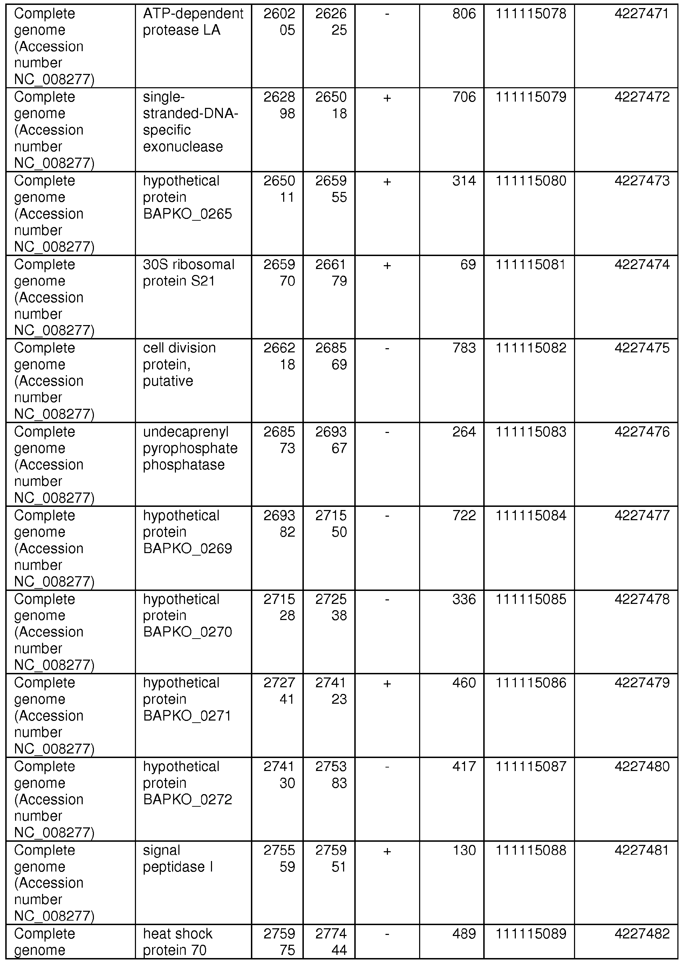

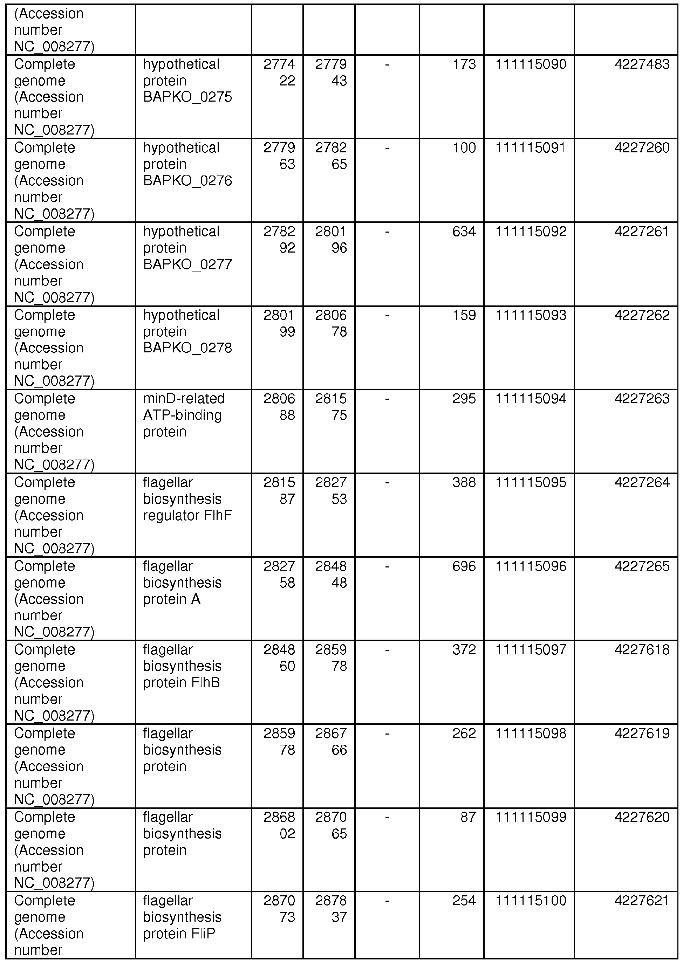

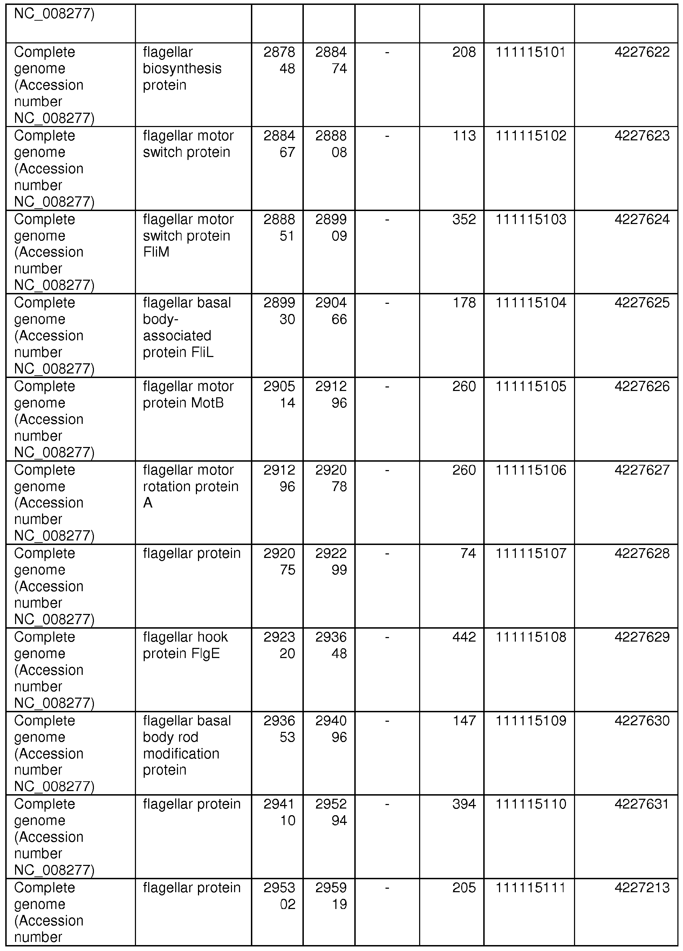

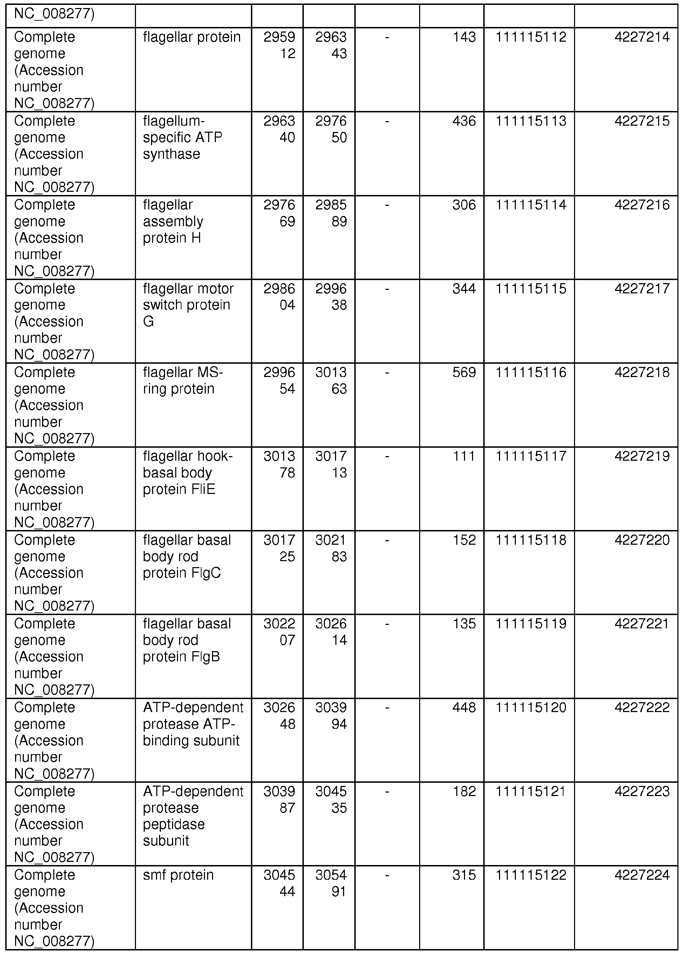

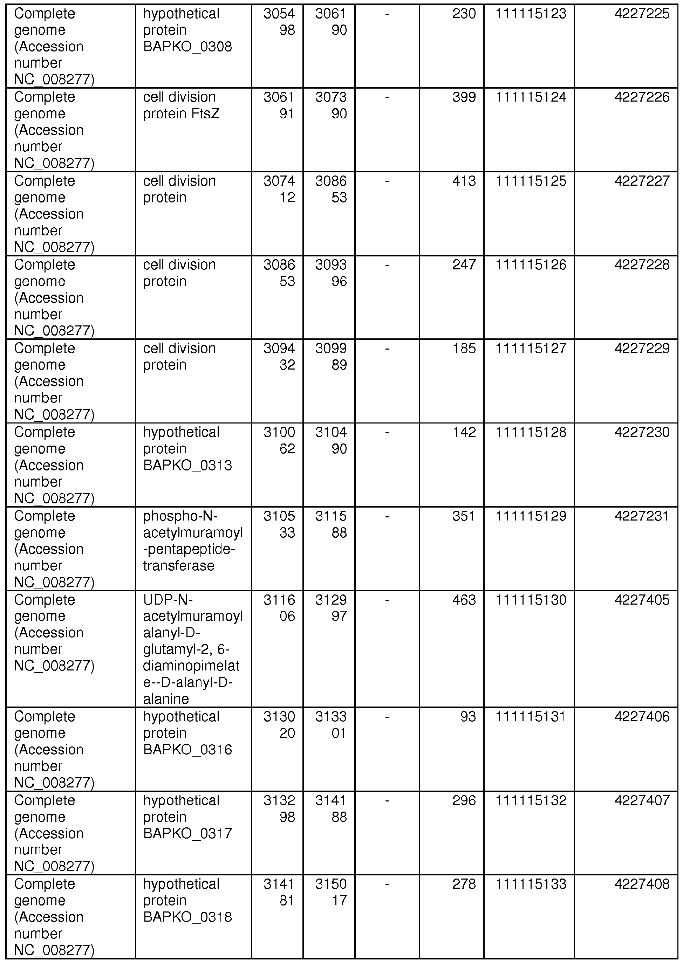

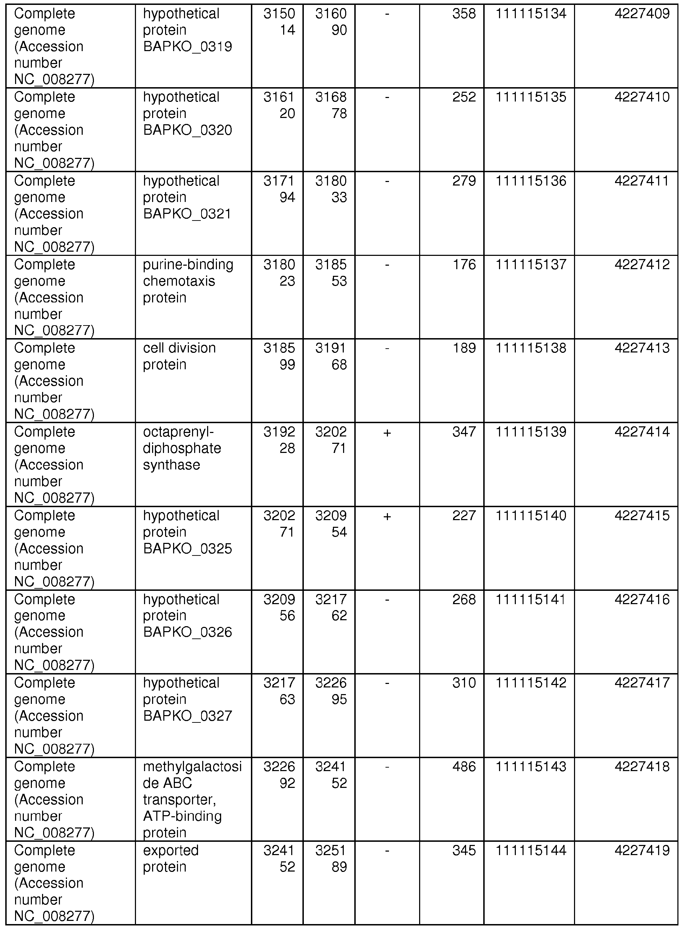

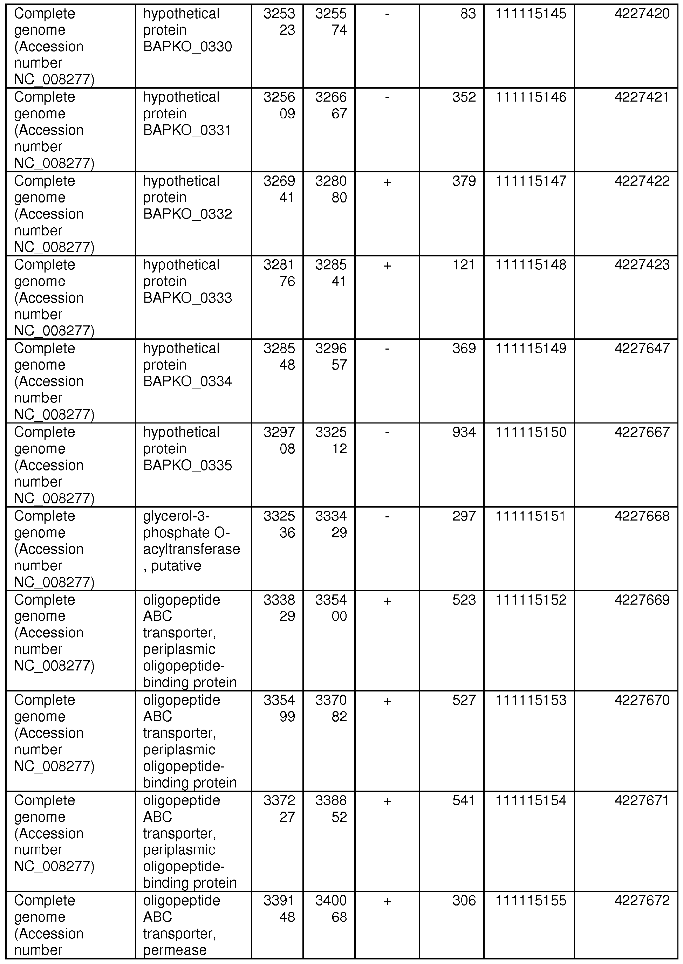

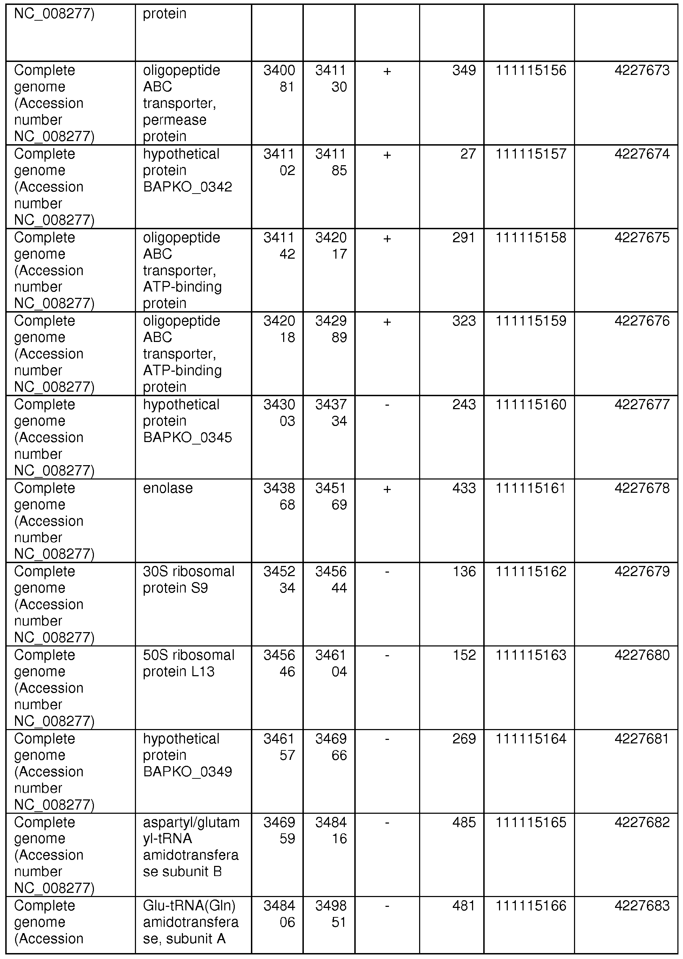

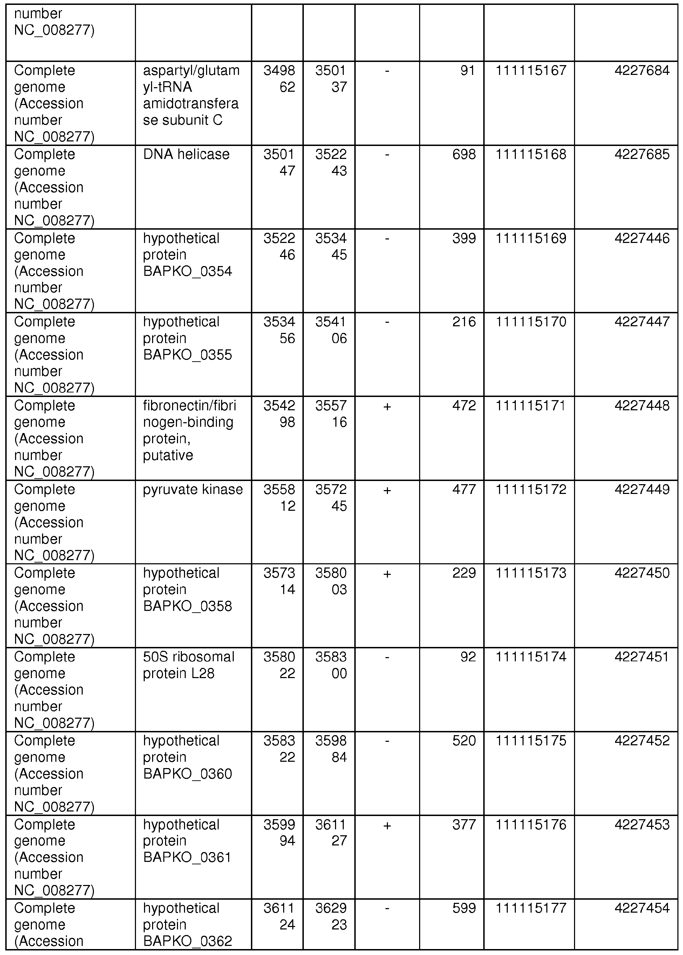

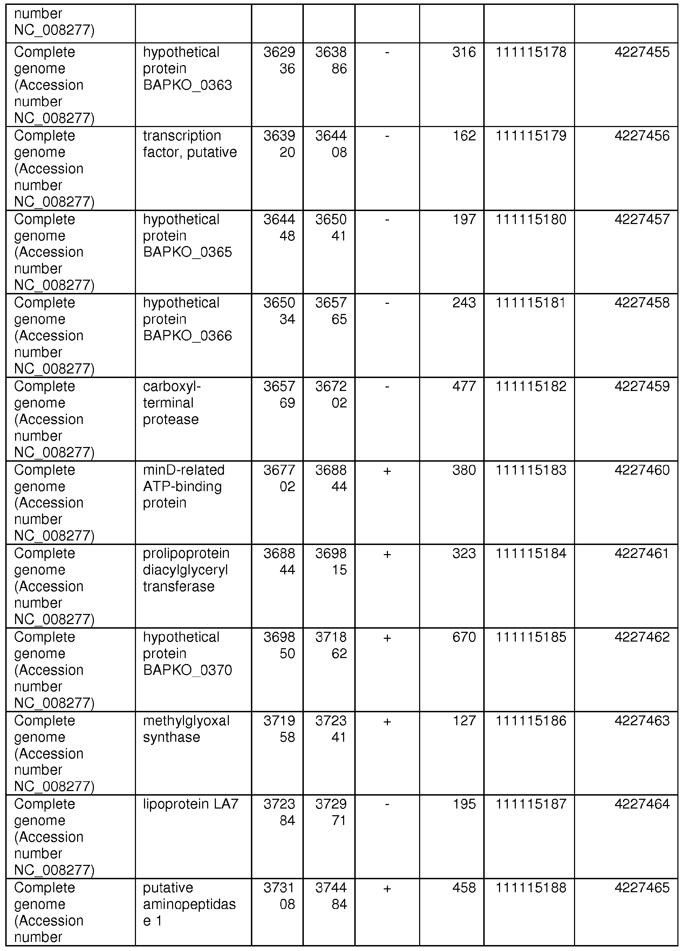

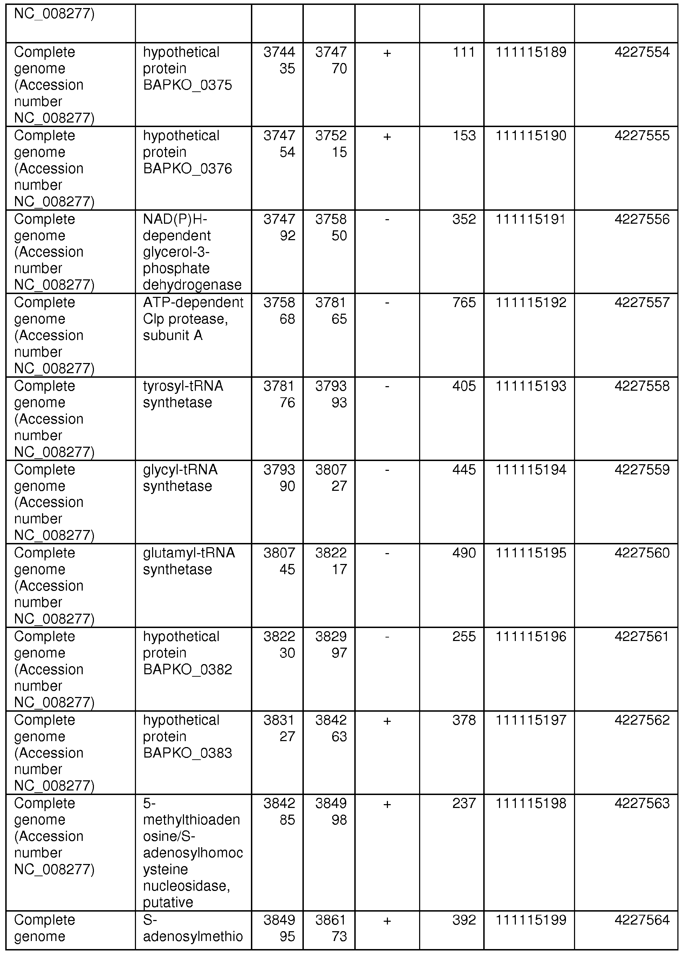

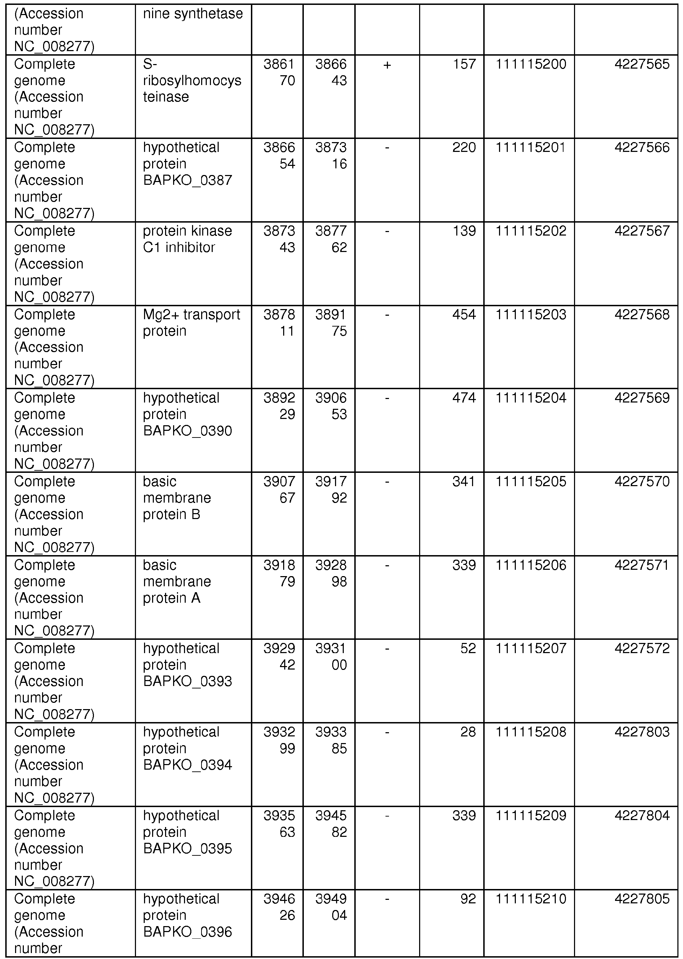

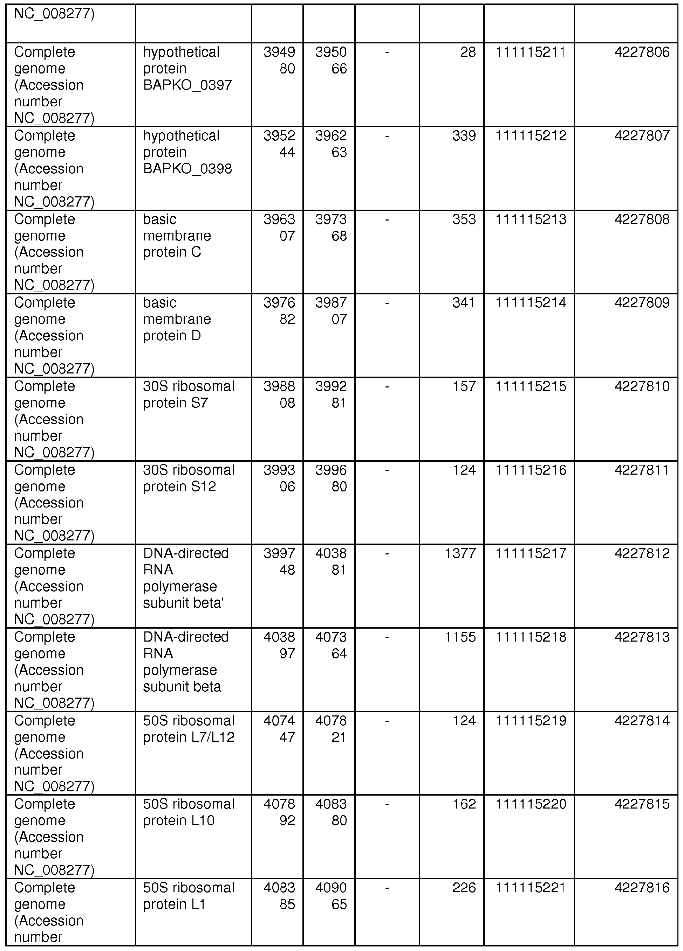

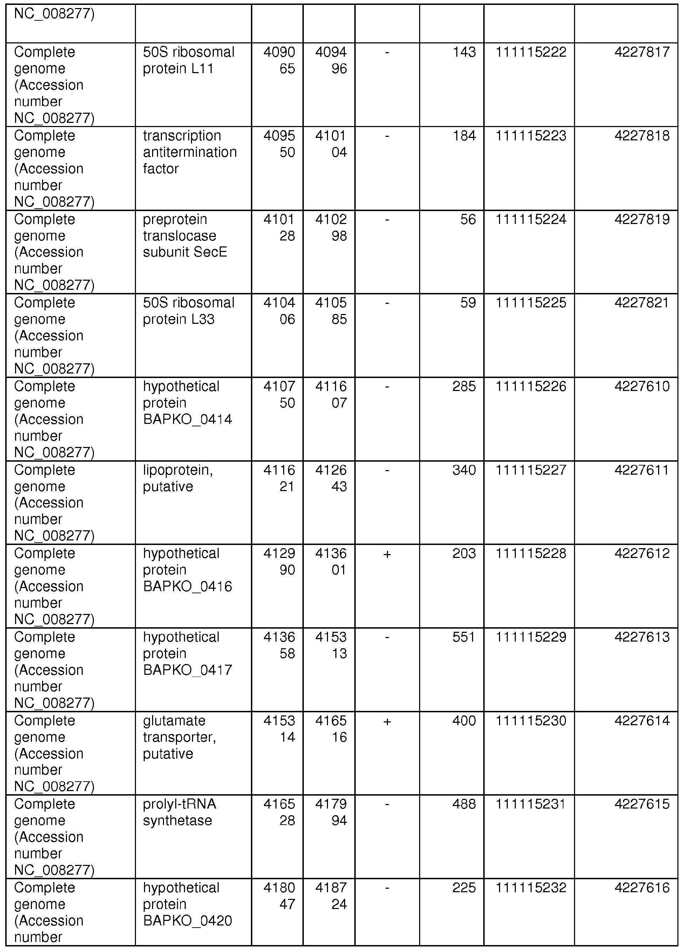

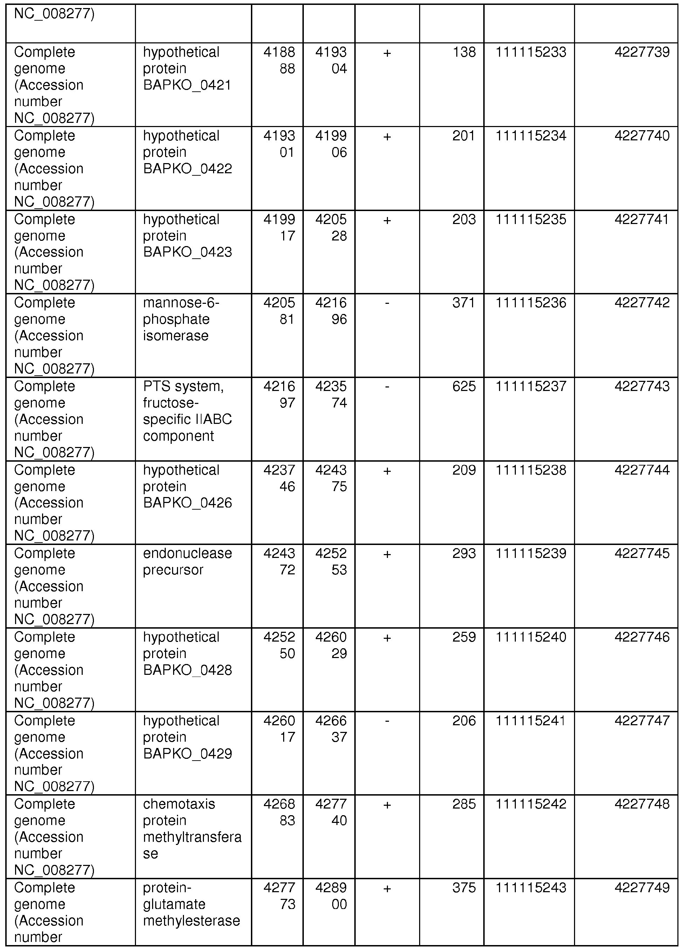

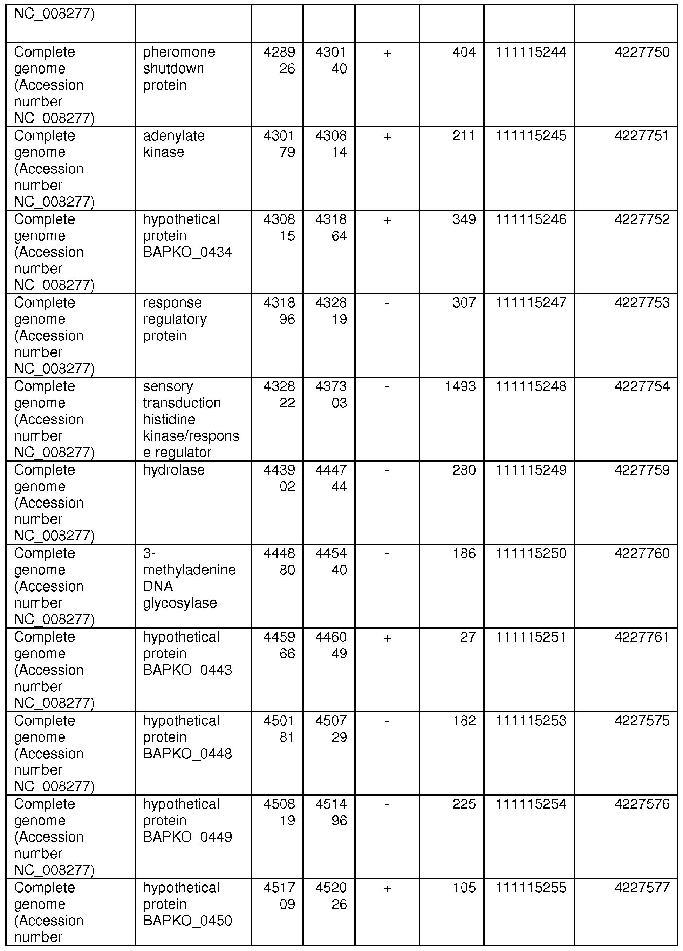

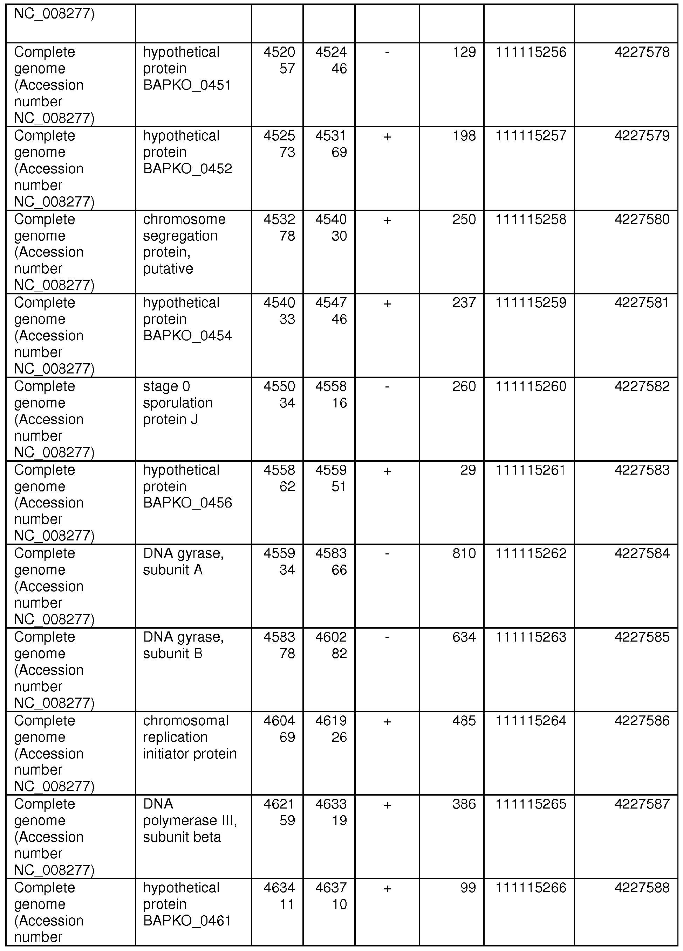

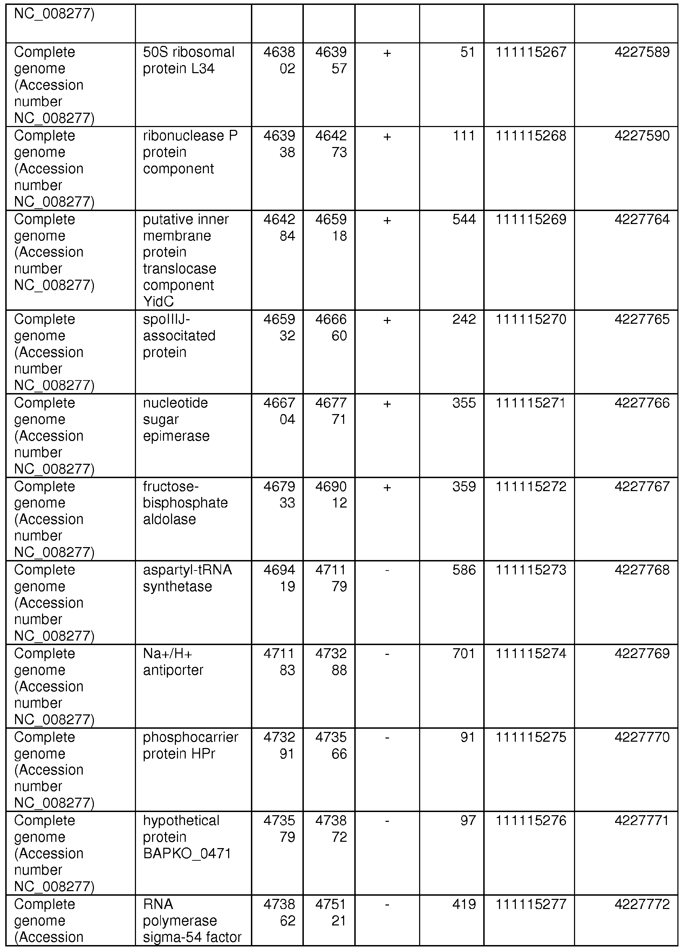

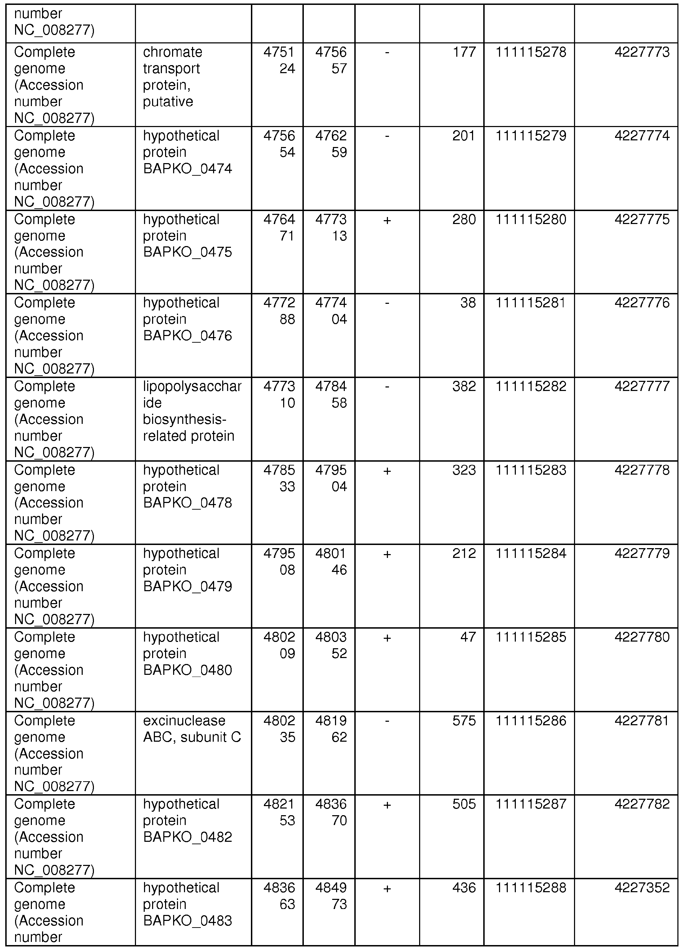

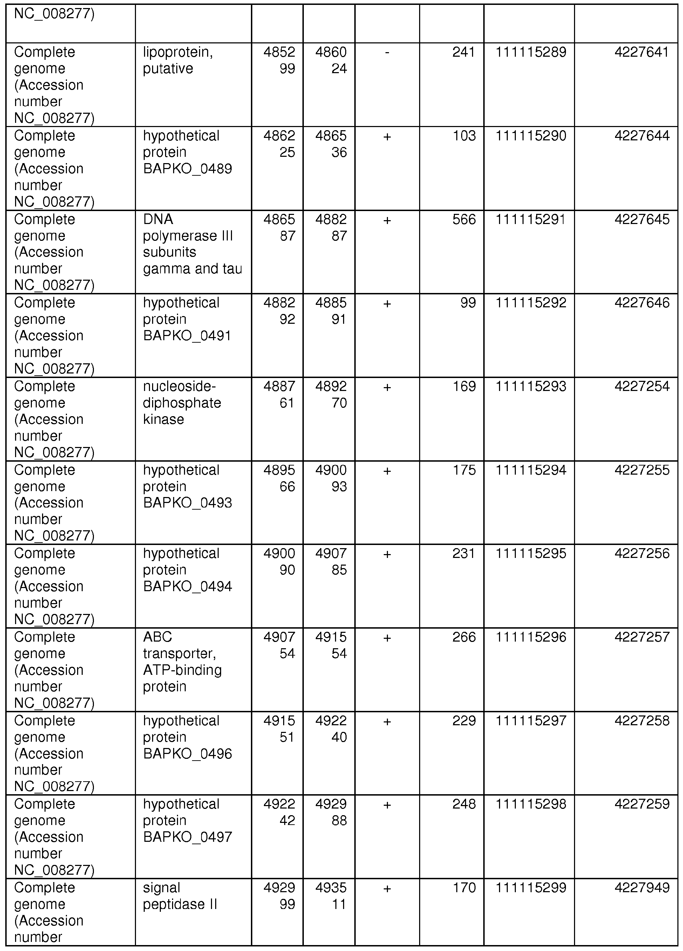

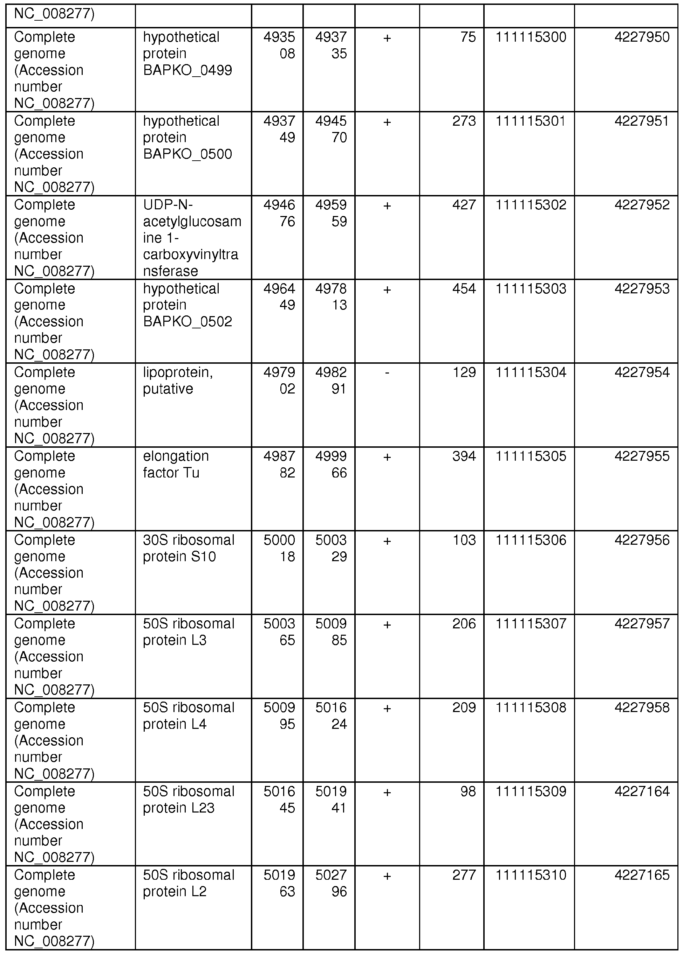

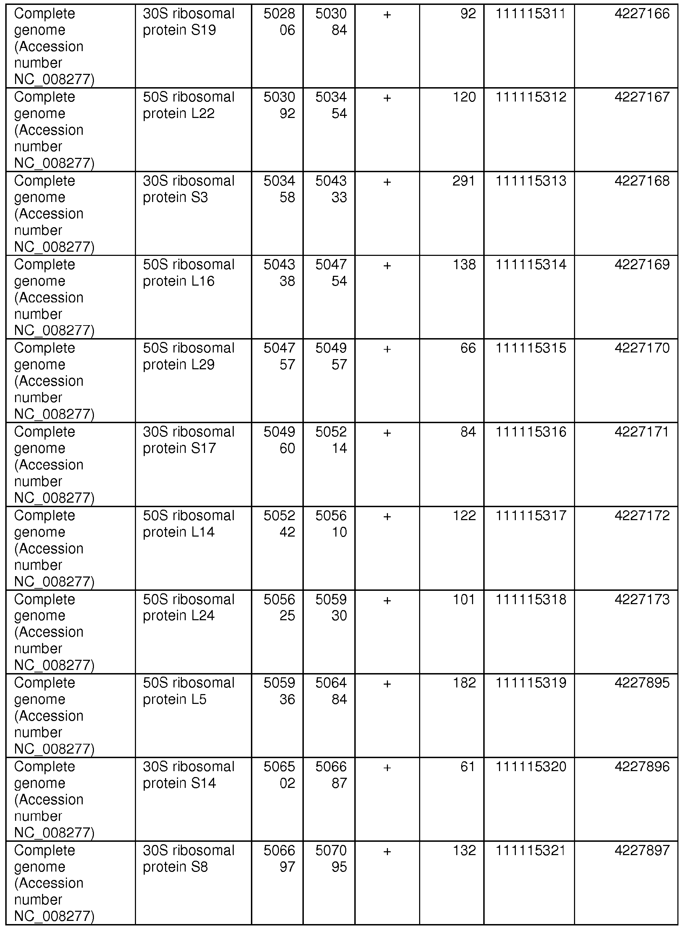

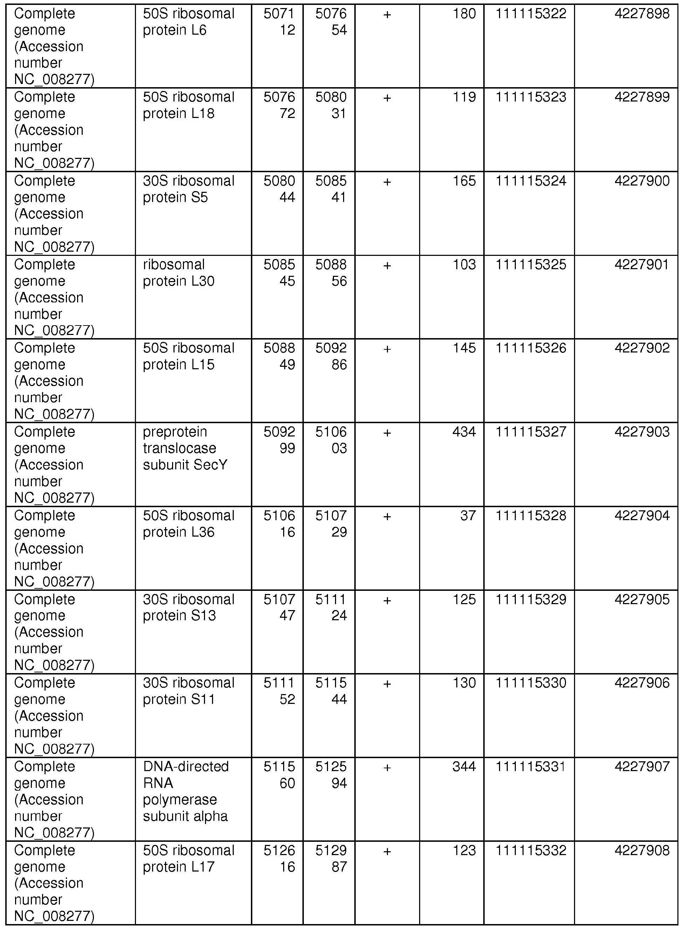

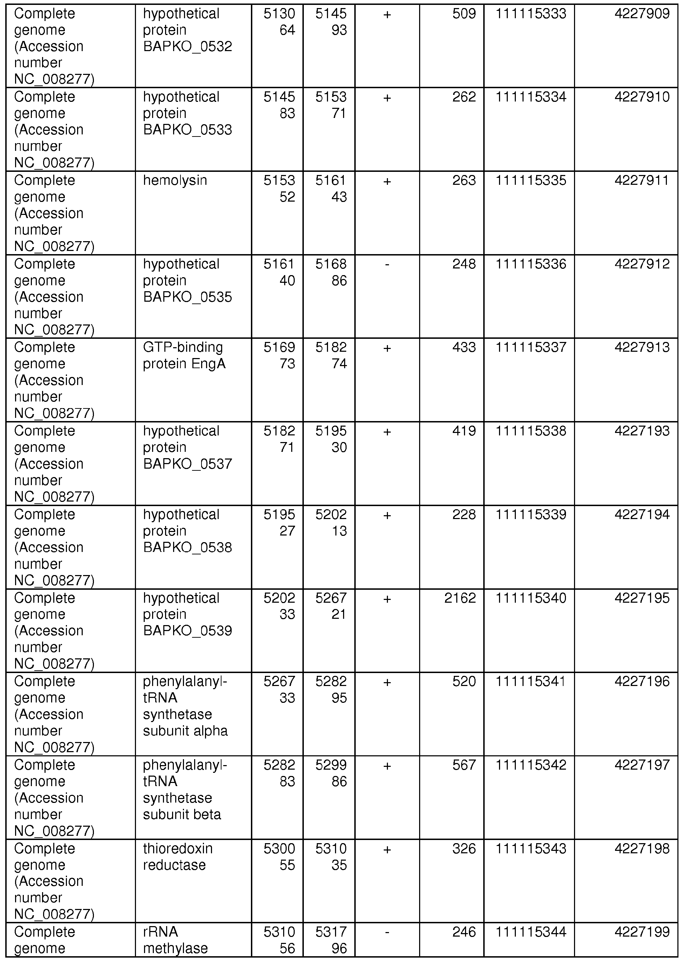

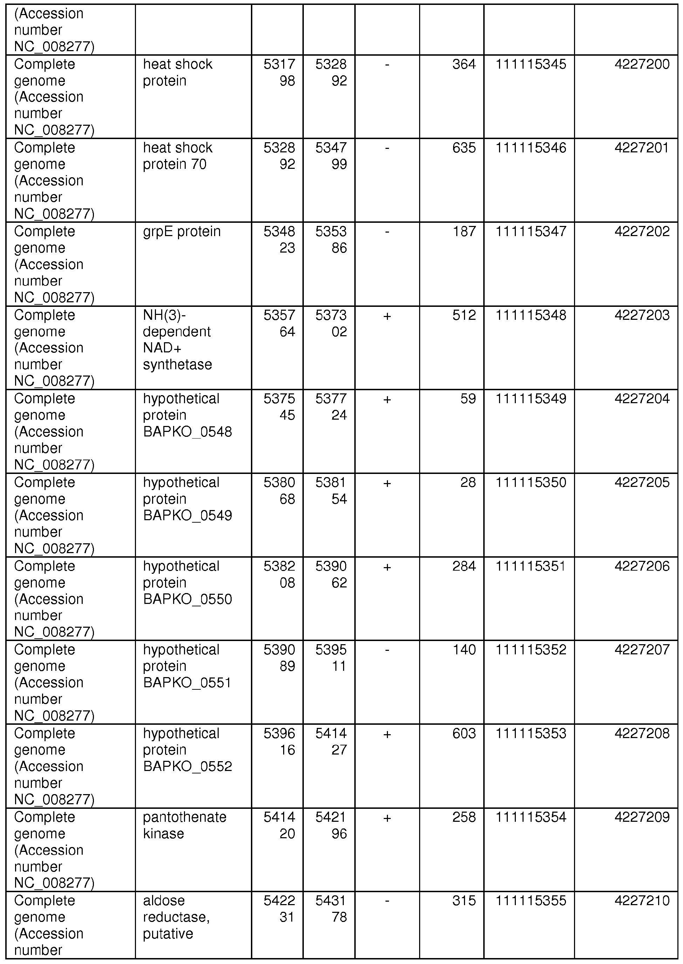

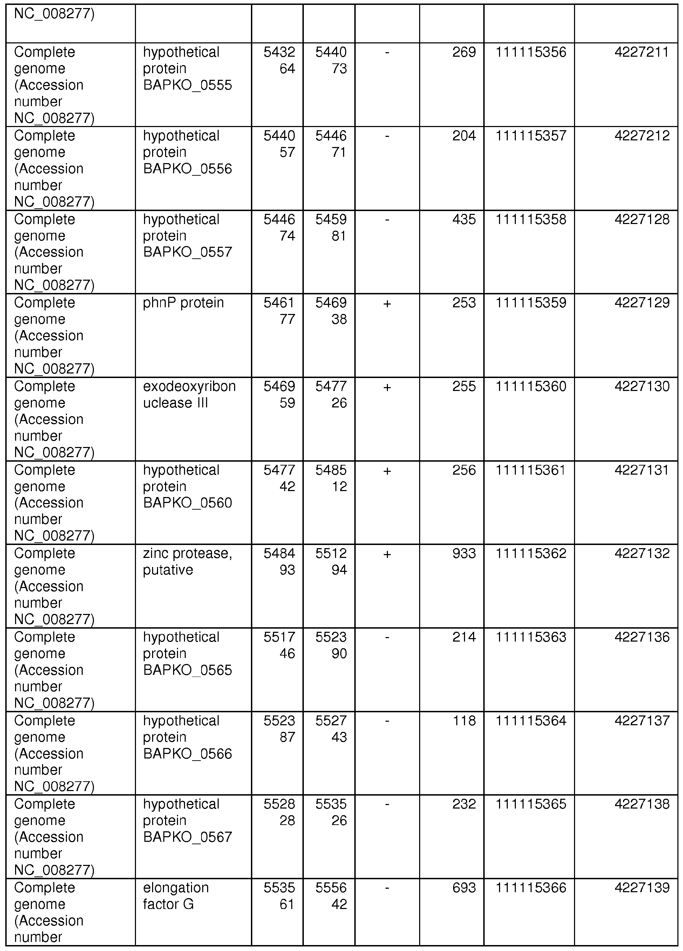

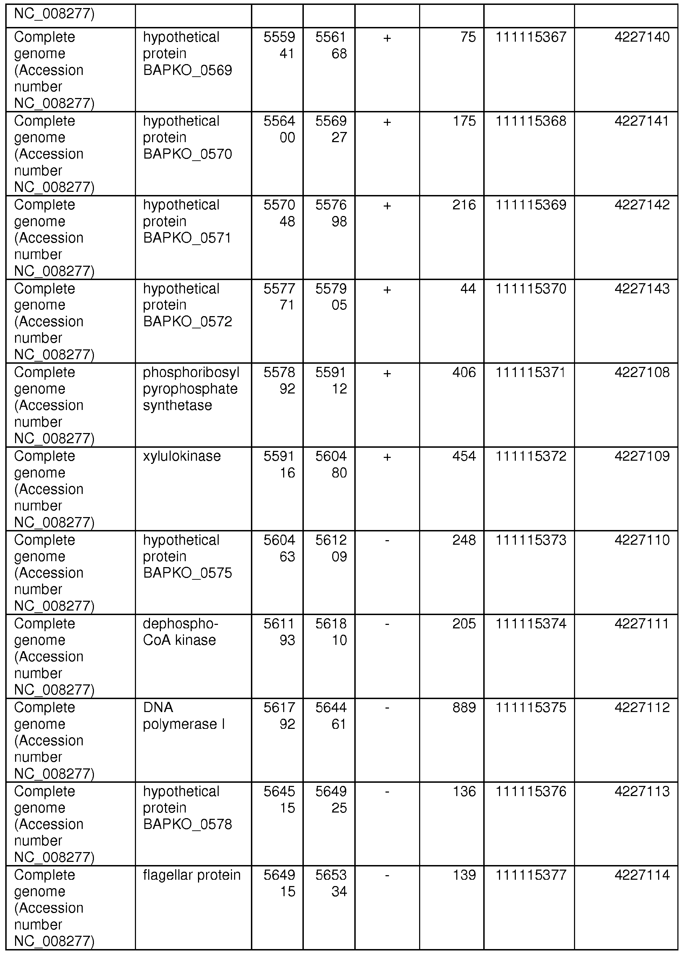

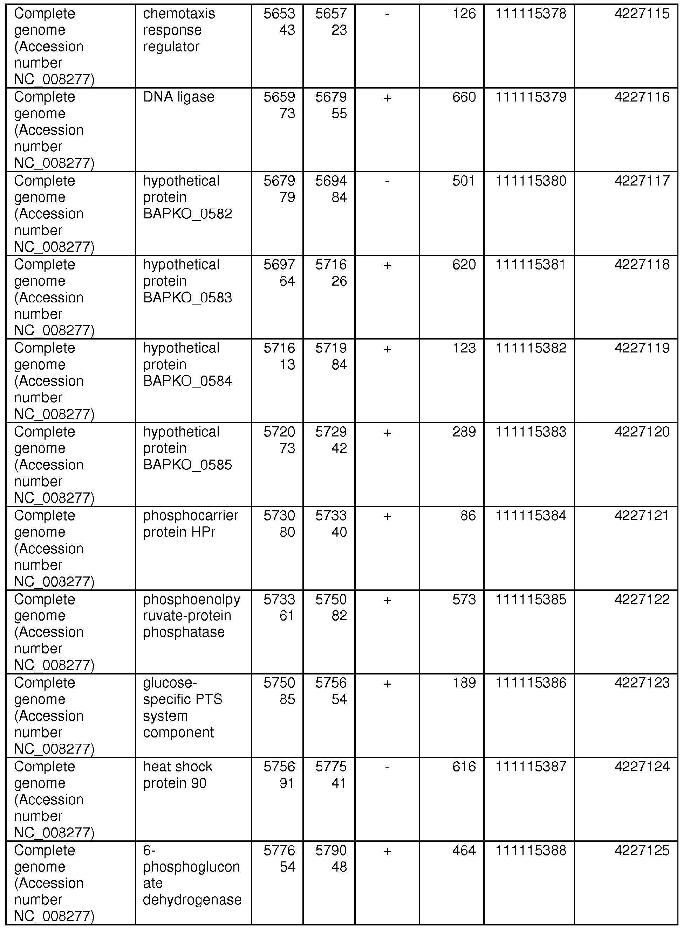

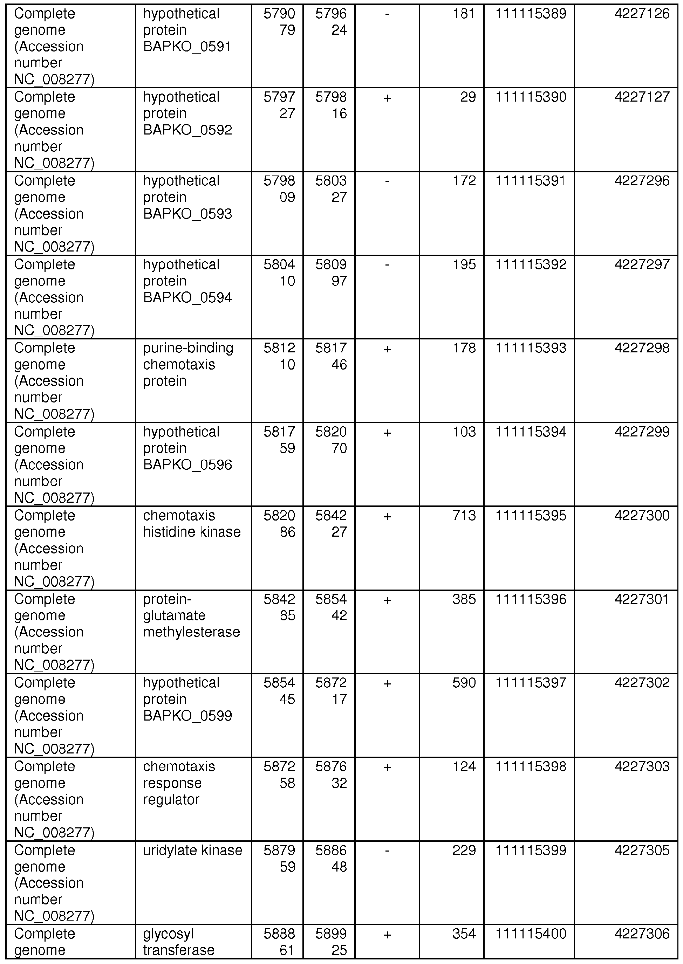

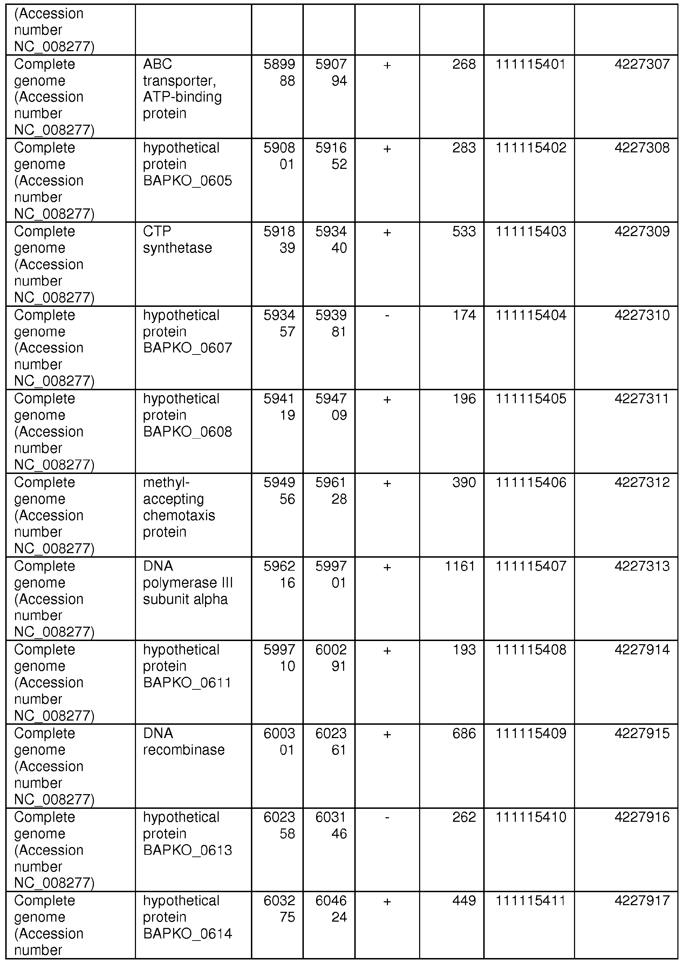

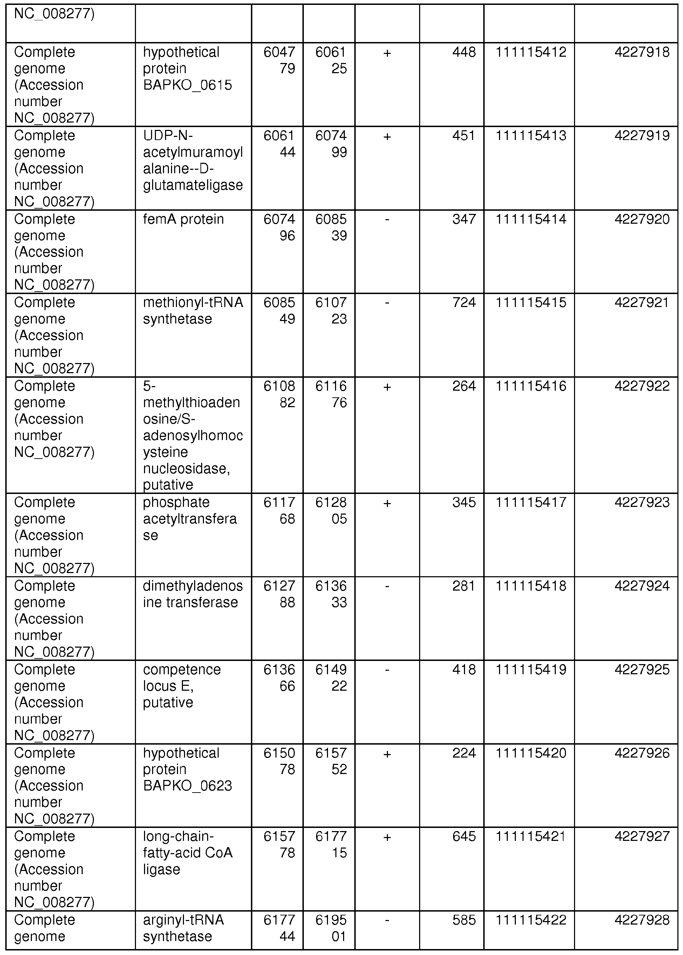

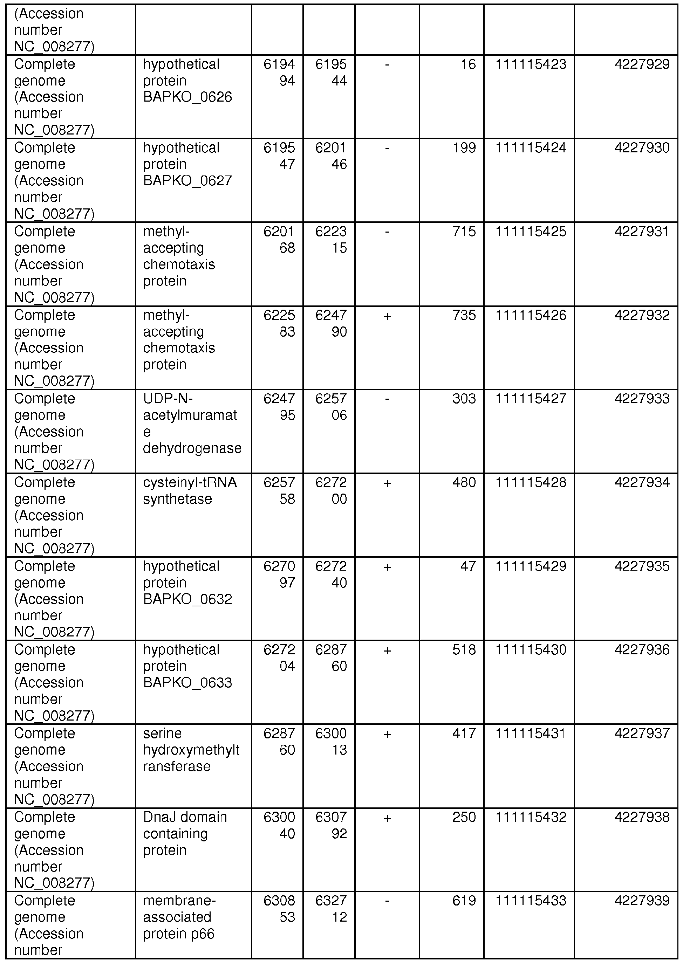

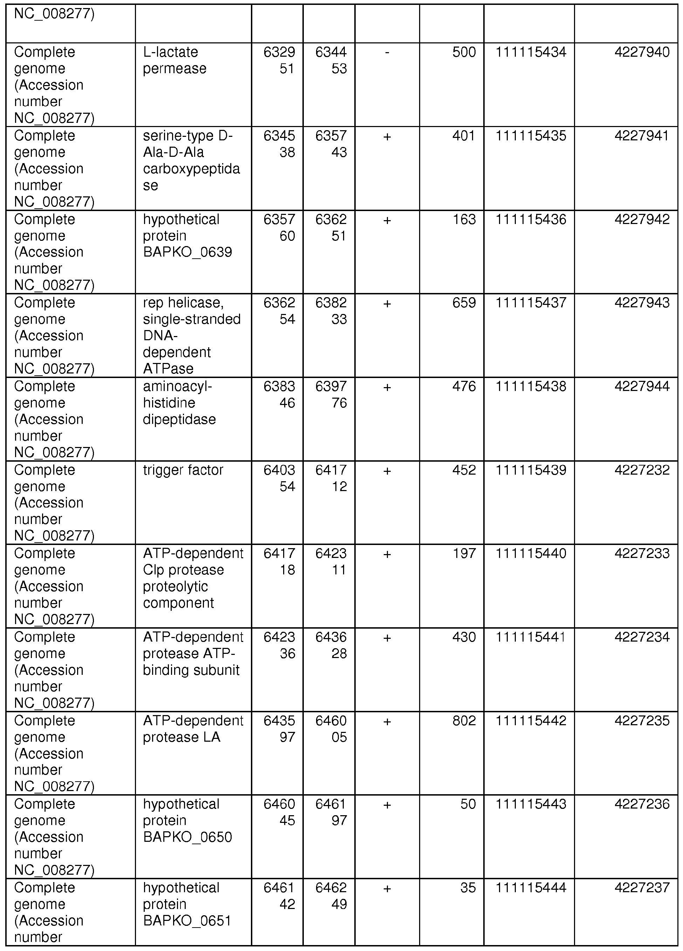

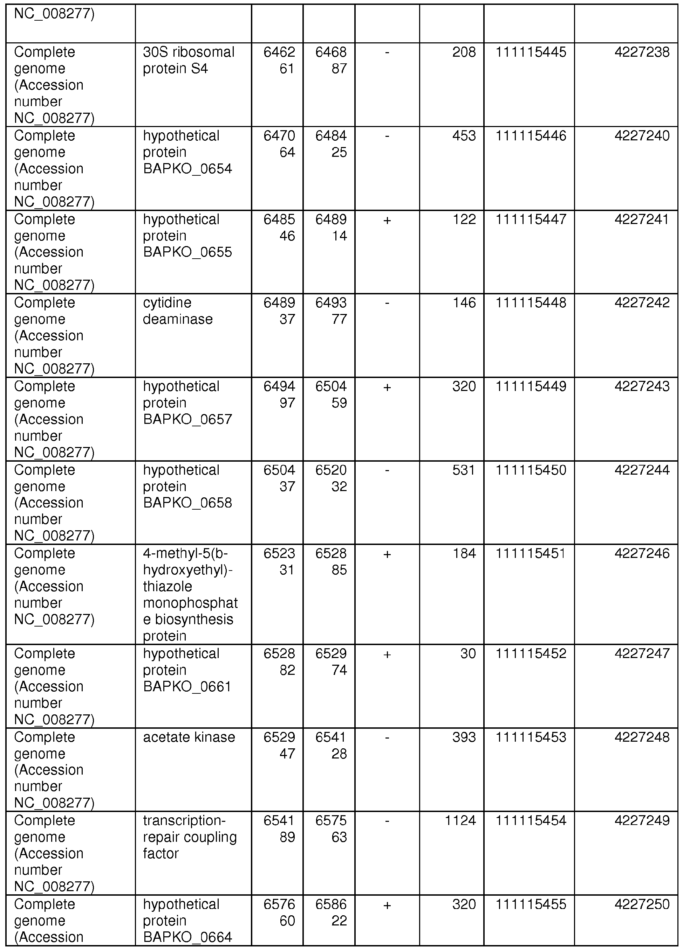

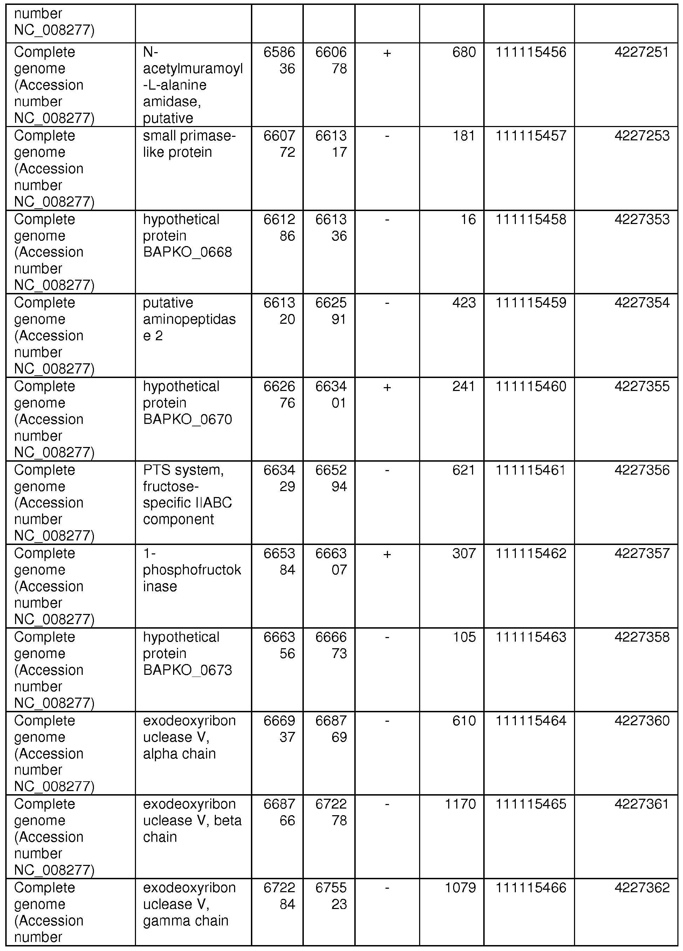

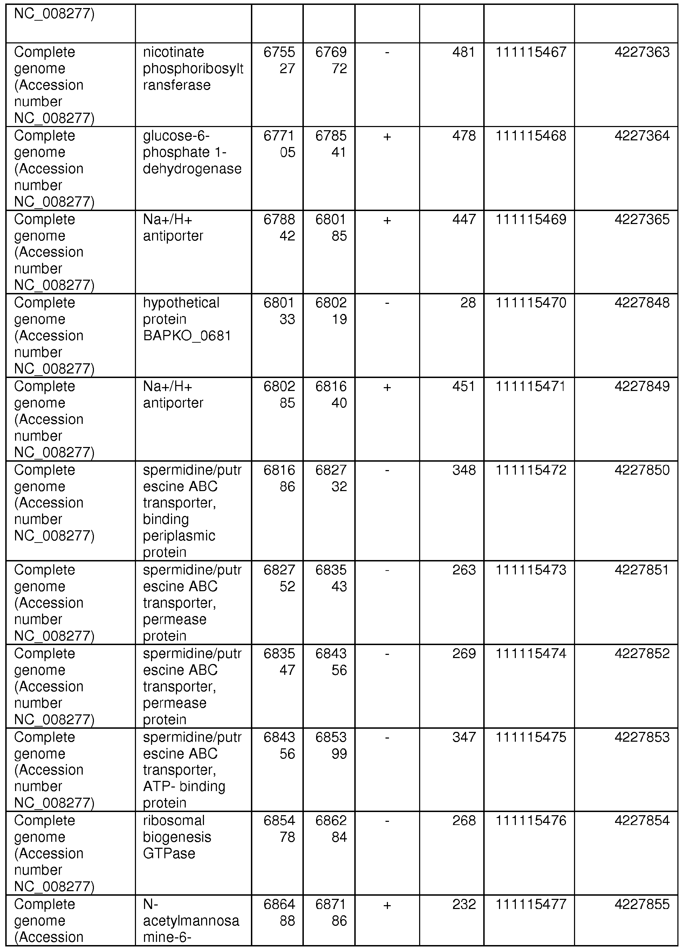

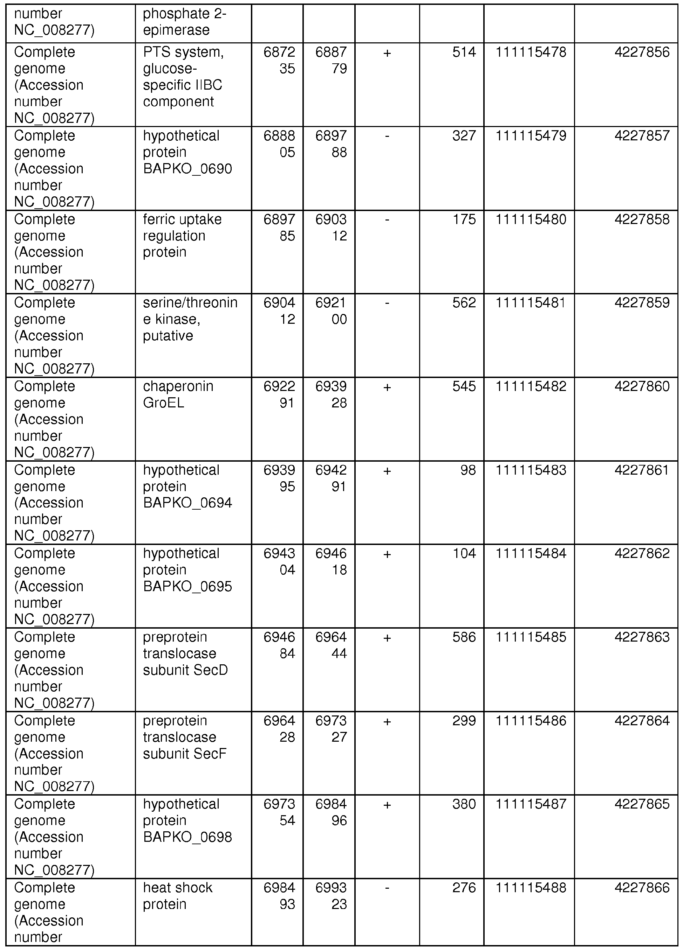

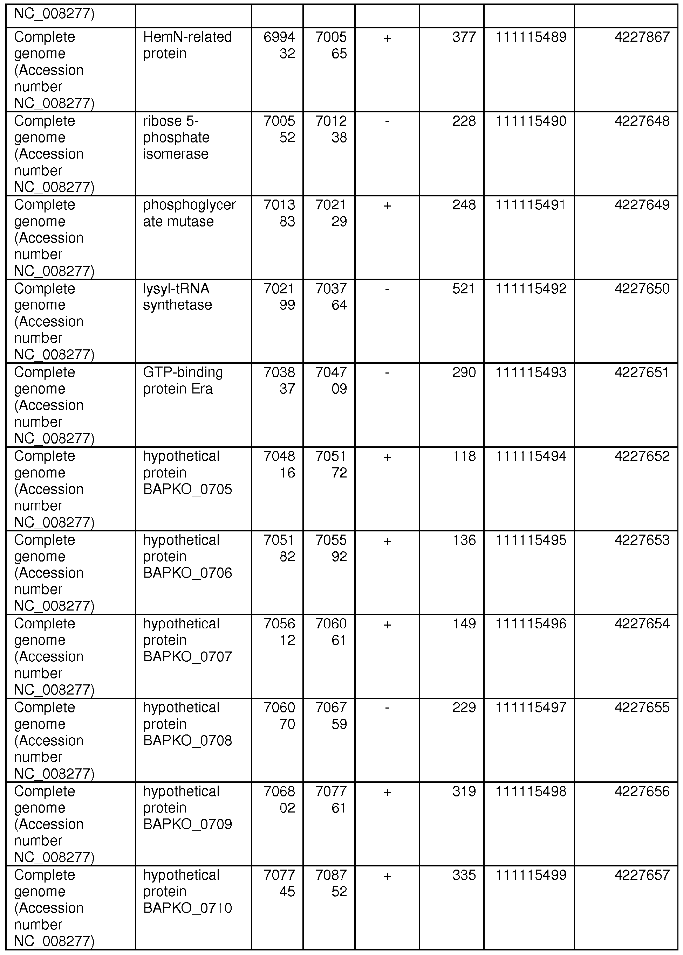

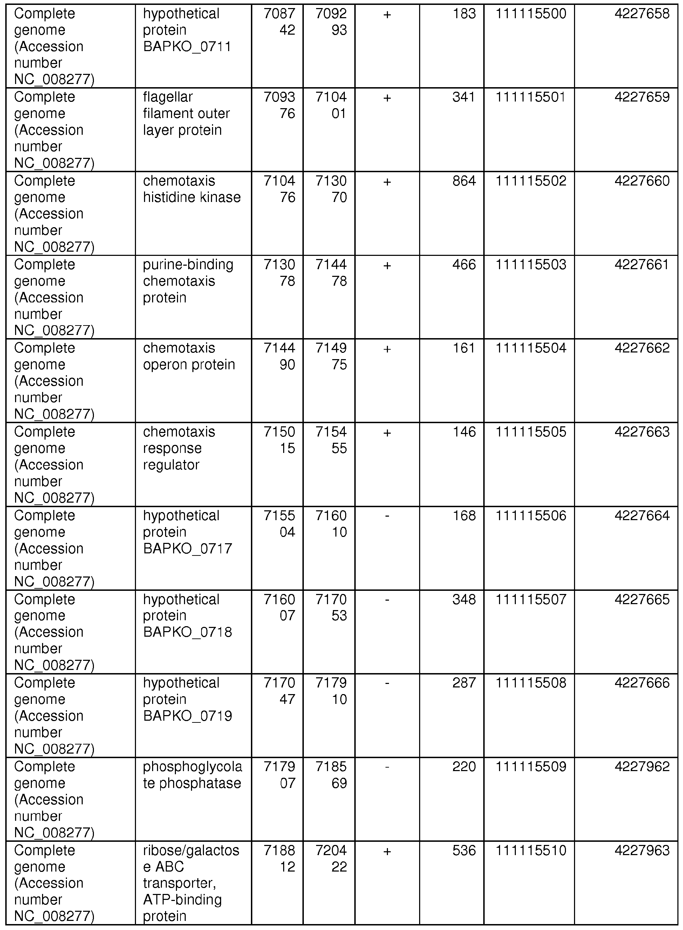

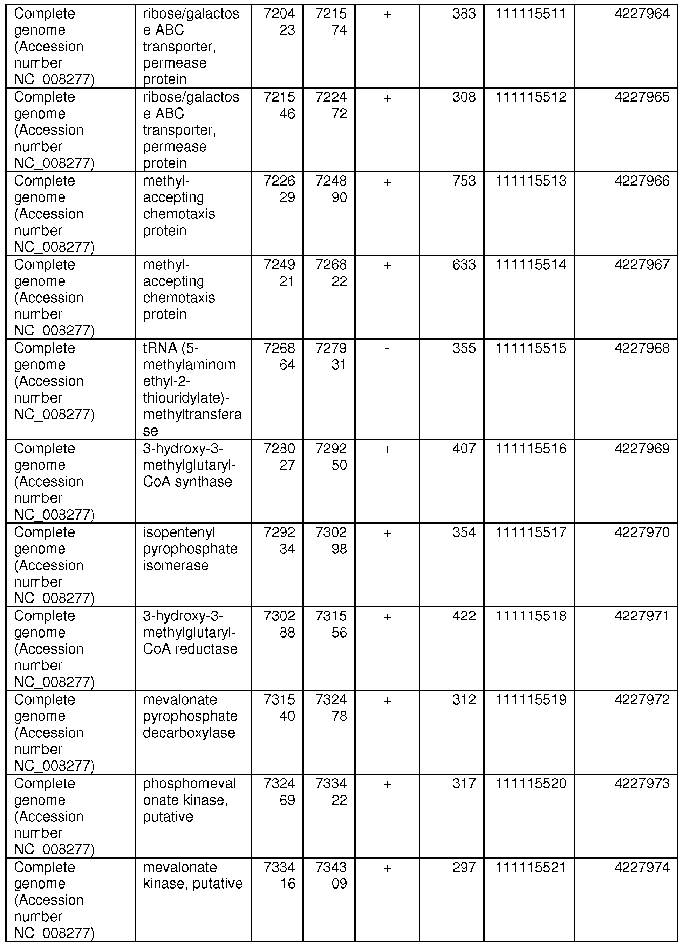

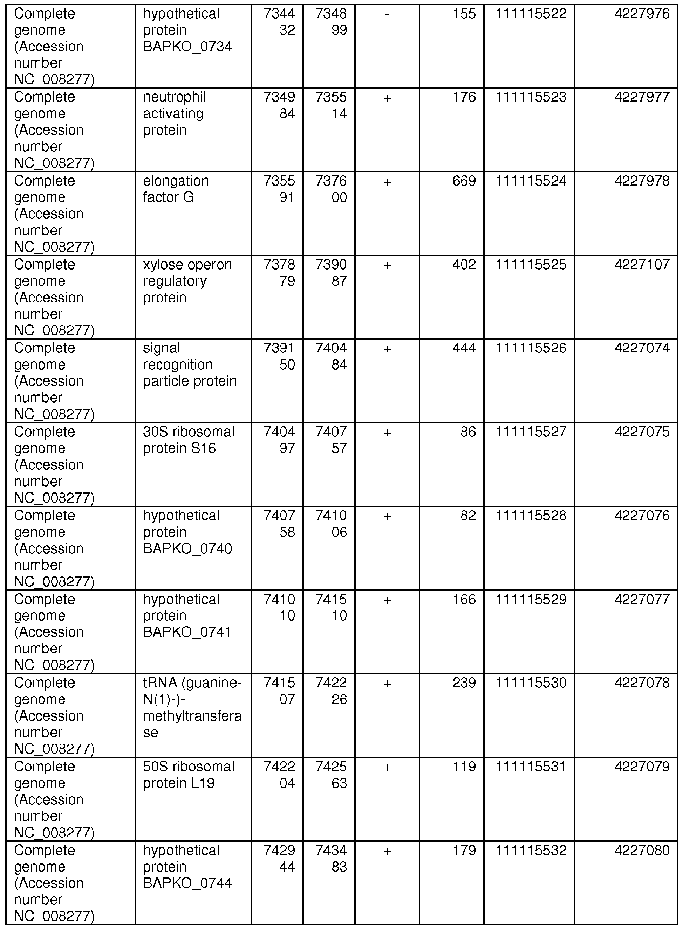

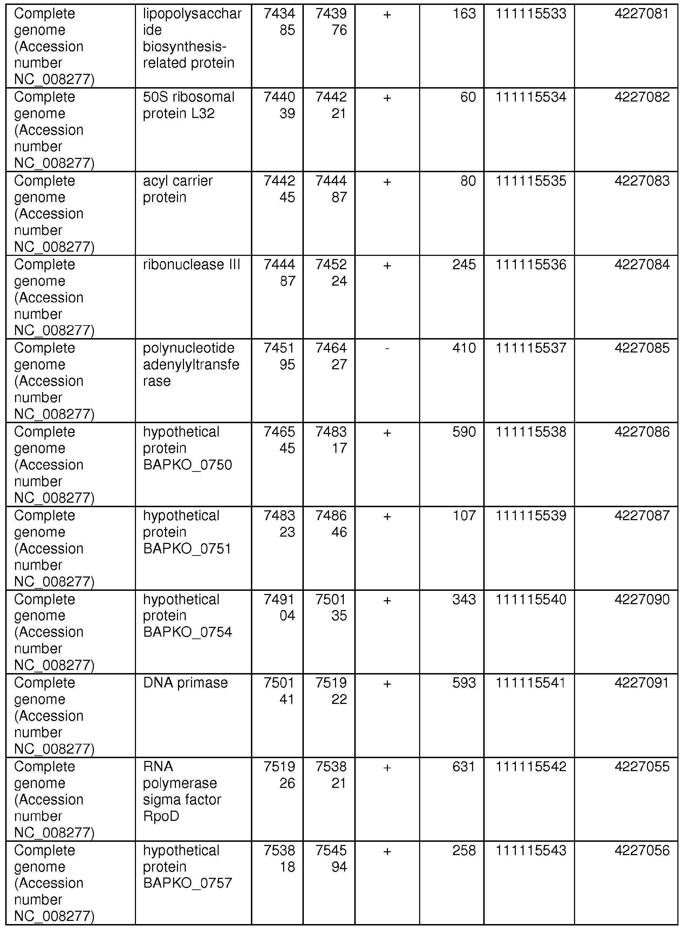

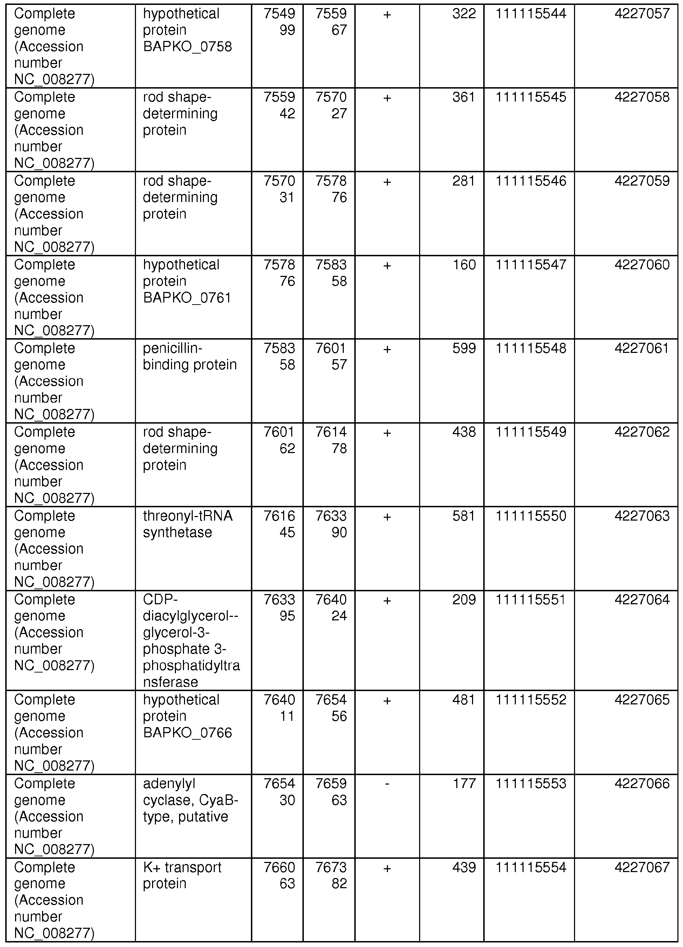

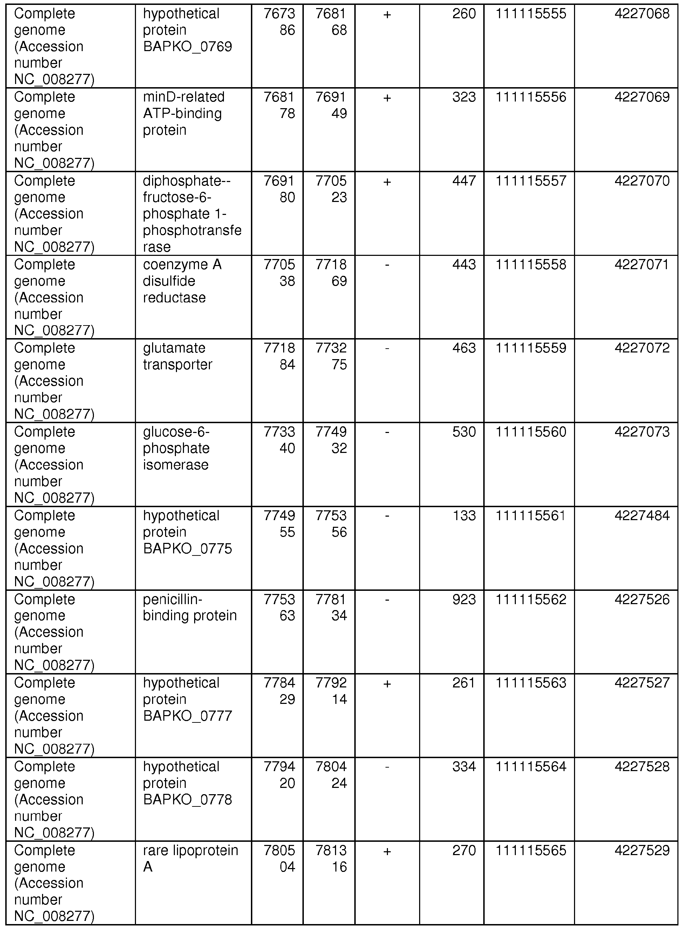

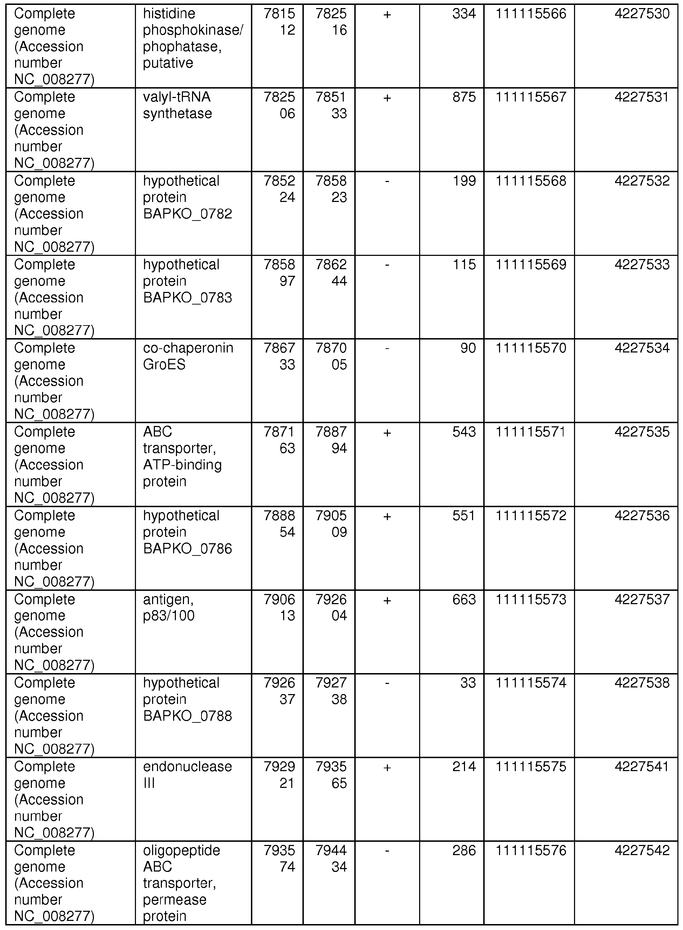

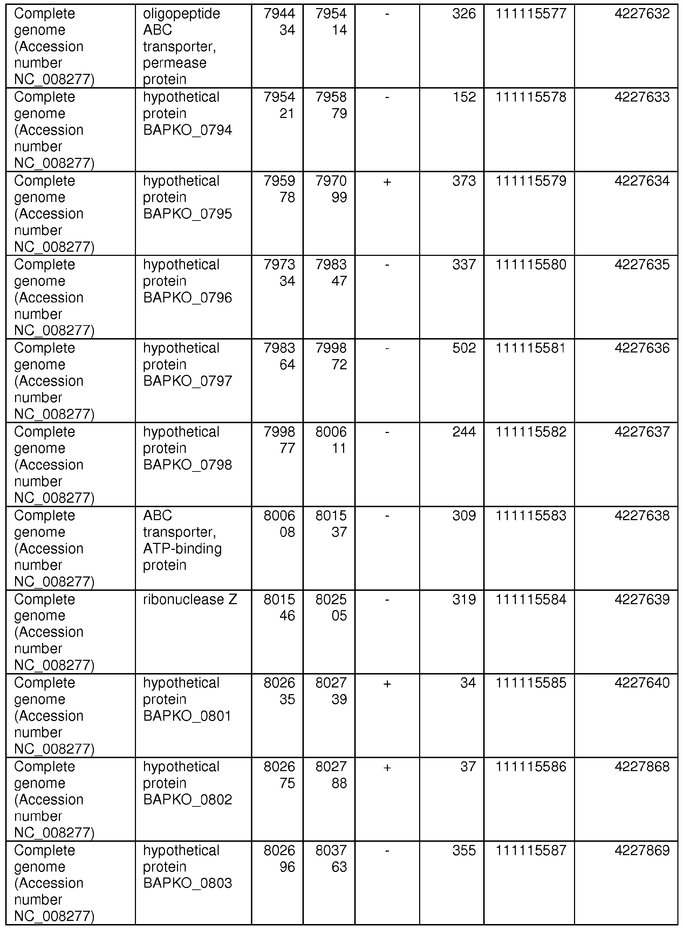

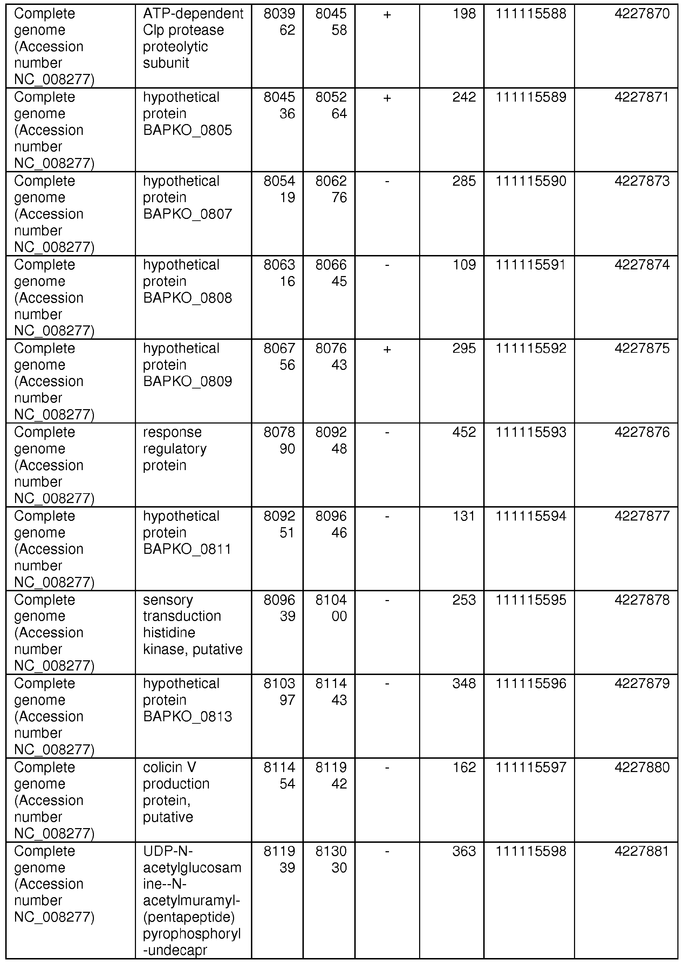

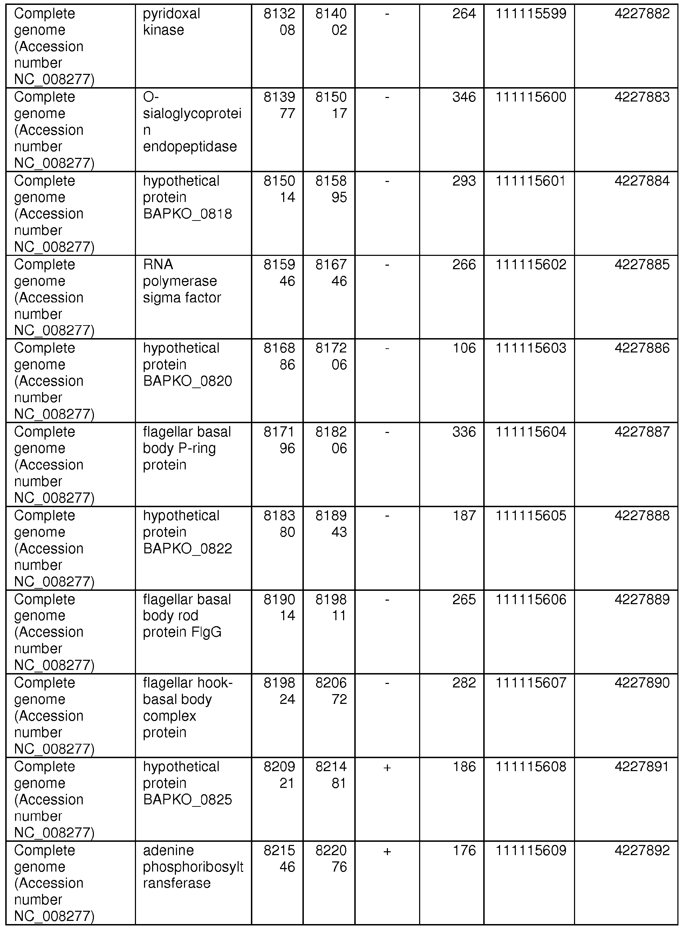

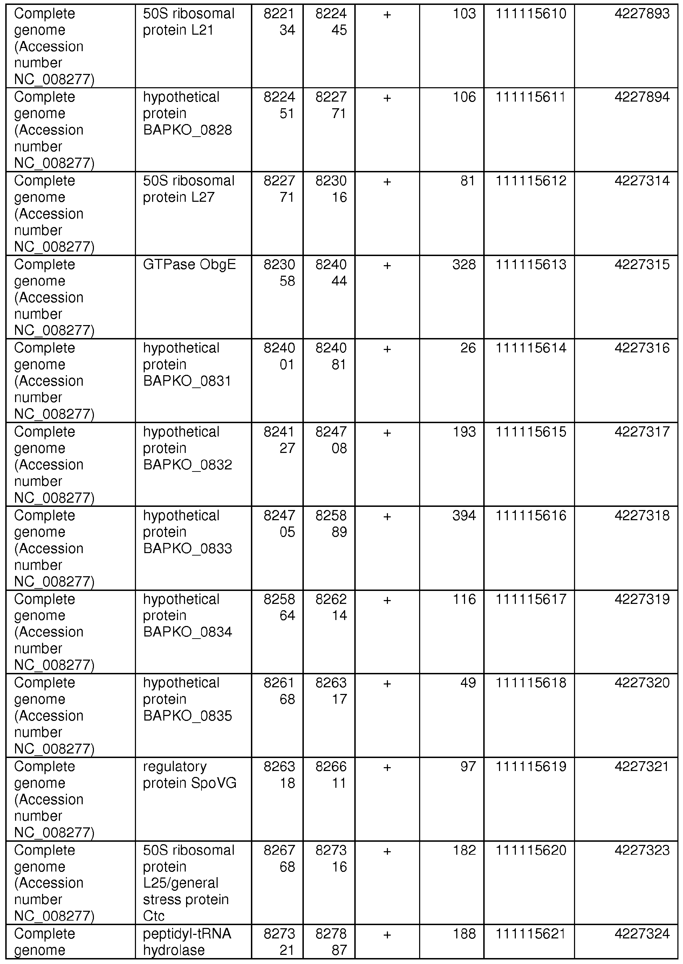

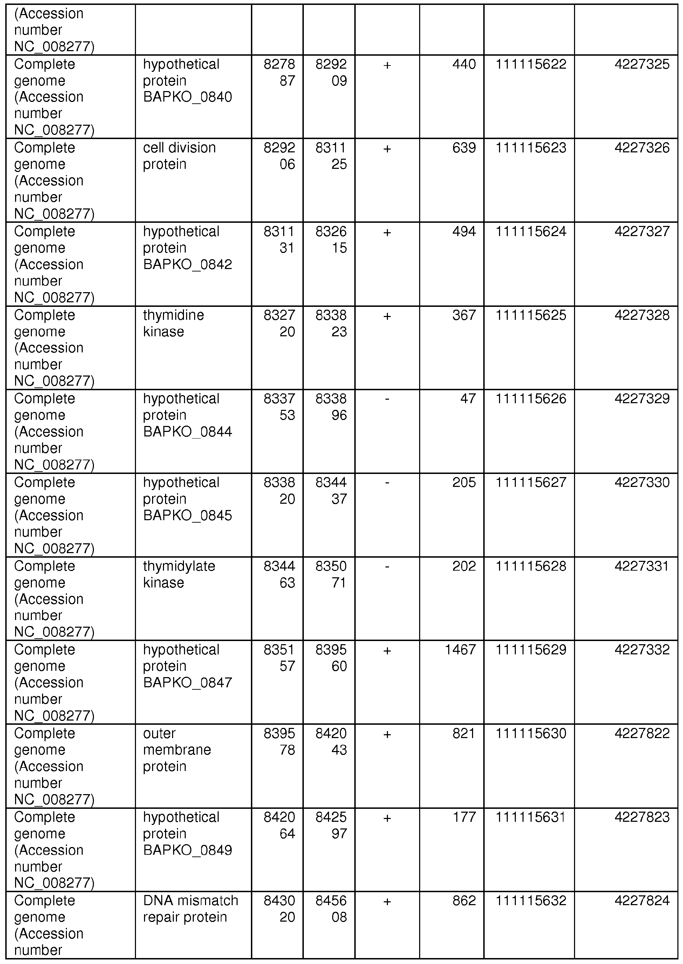

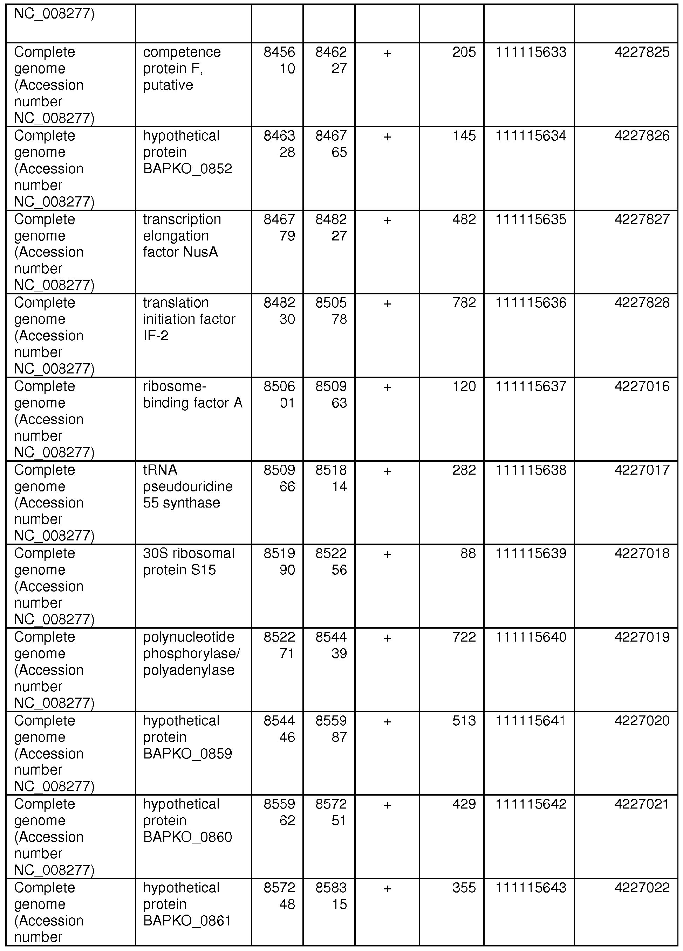

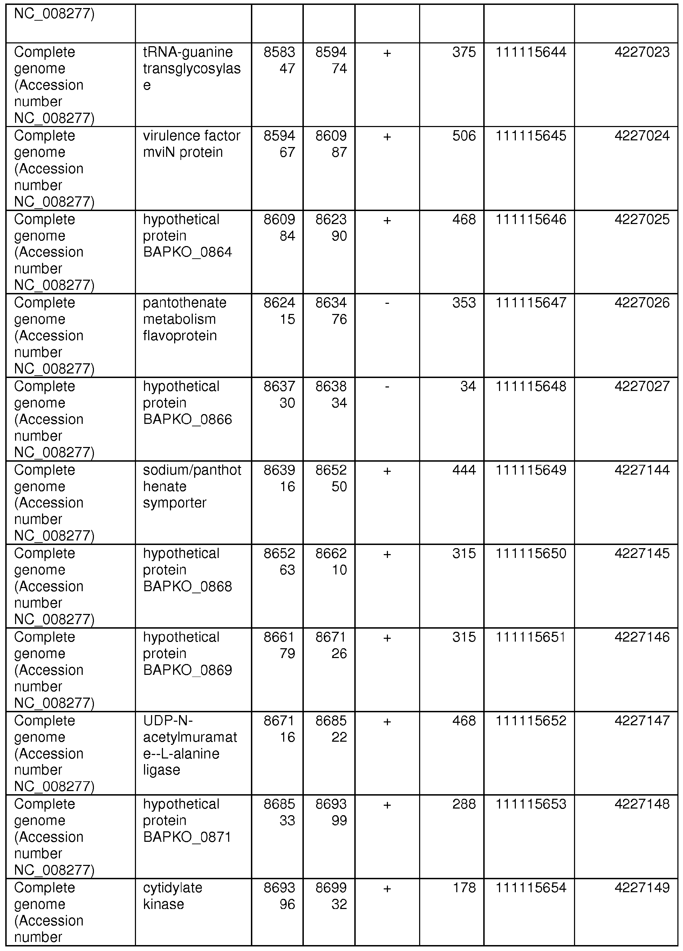

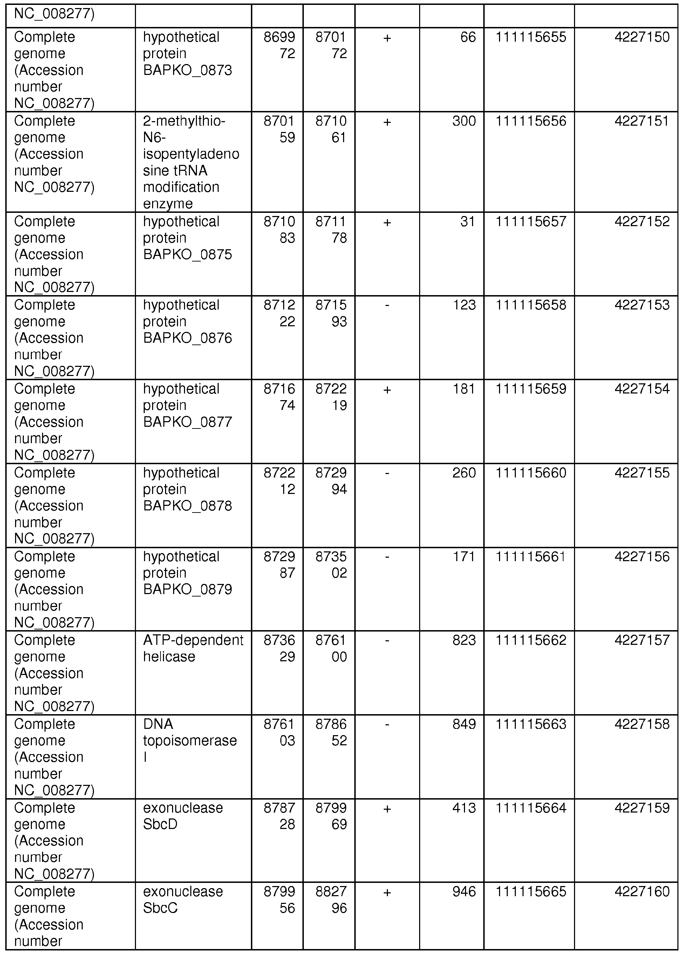

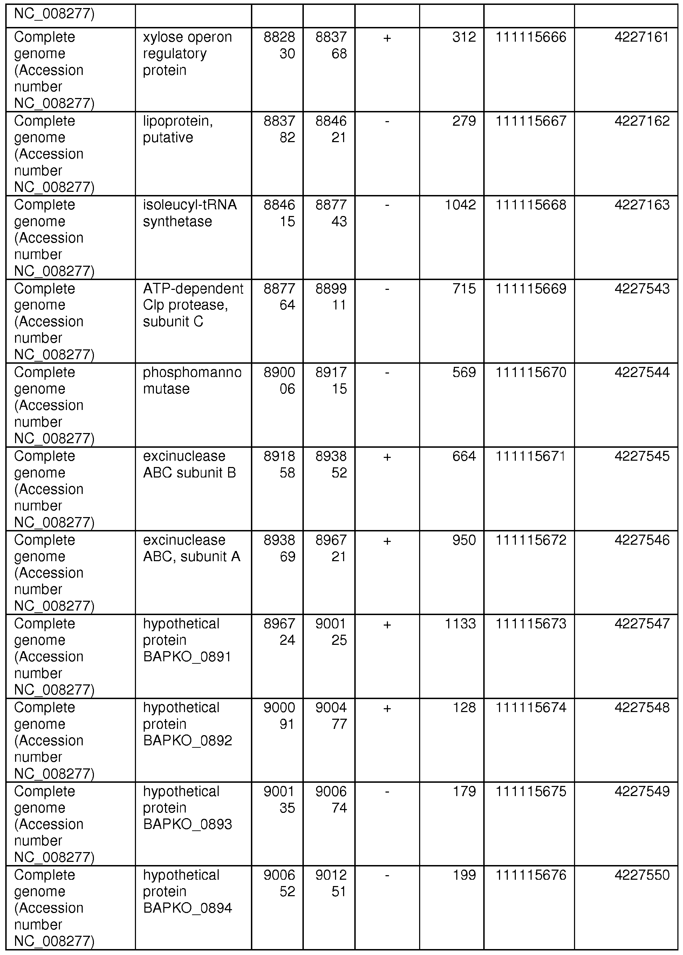

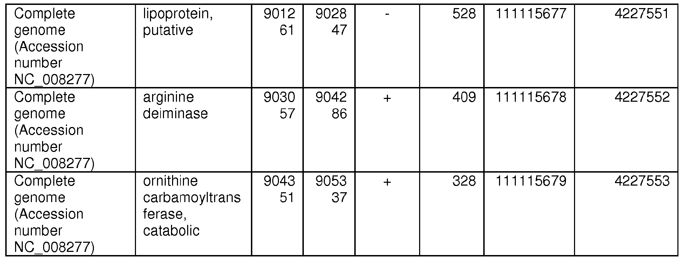

In another preferred embodiment the present disclosure relates to one or more antigenic peptides or antigenic polypeptides comprising a fragment of the sequence from one or more proteins from Borrelia afzelii Pko, or to one or more MHC-peptide complexes or MHC multimers, compricing one or more such antigenic peptides. The one or more proteins can be selected from the proteins listed in Figure 28 and Figure 38. These fagments can e.g. be 8 mers, 9 mers, 10 mers, 11 mers, 12 mers, 13 mers, 14 mers, 15 mers or 16 mers.

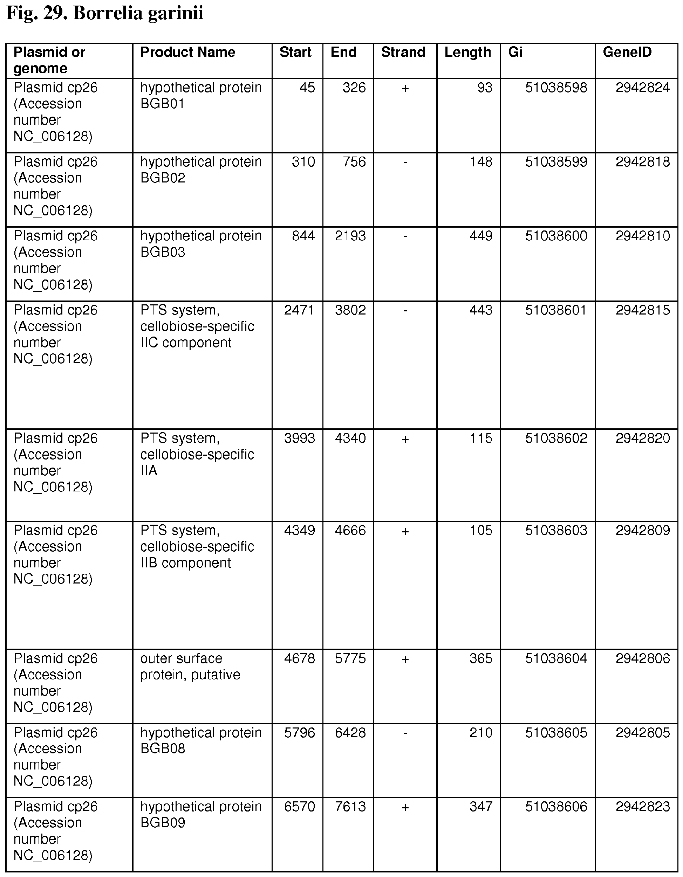

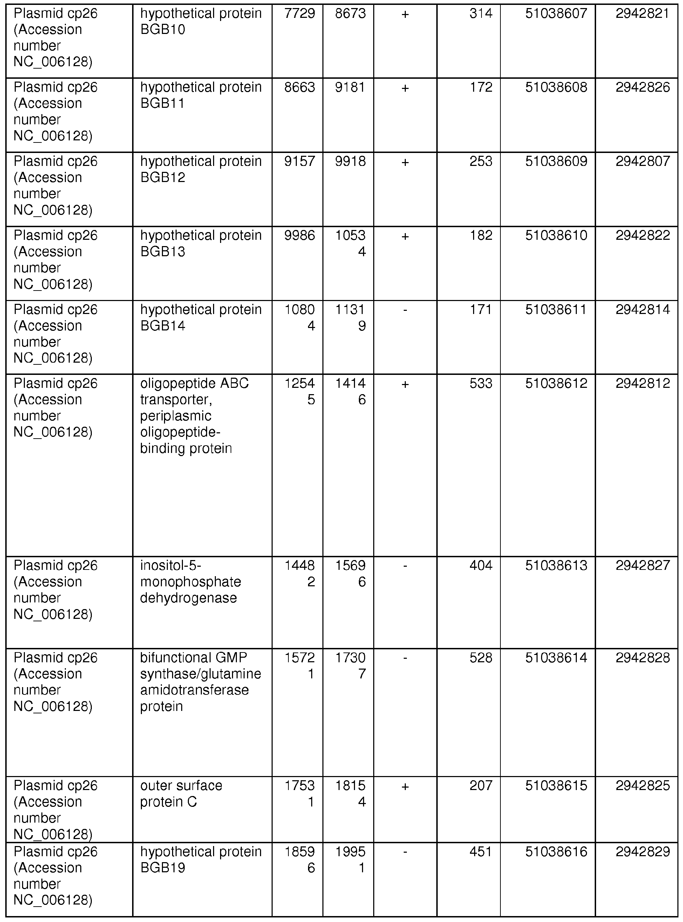

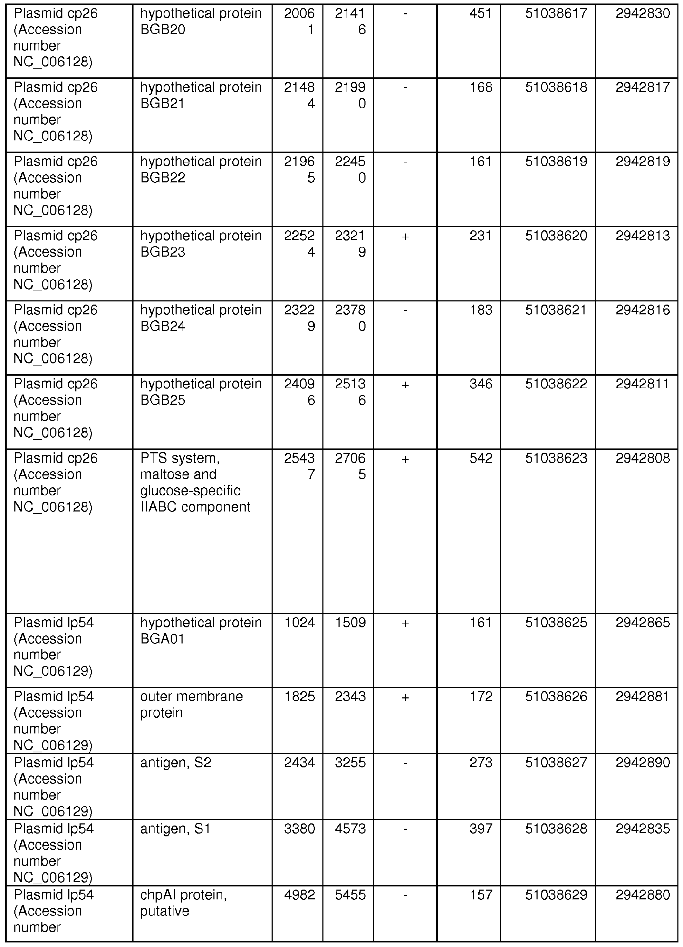

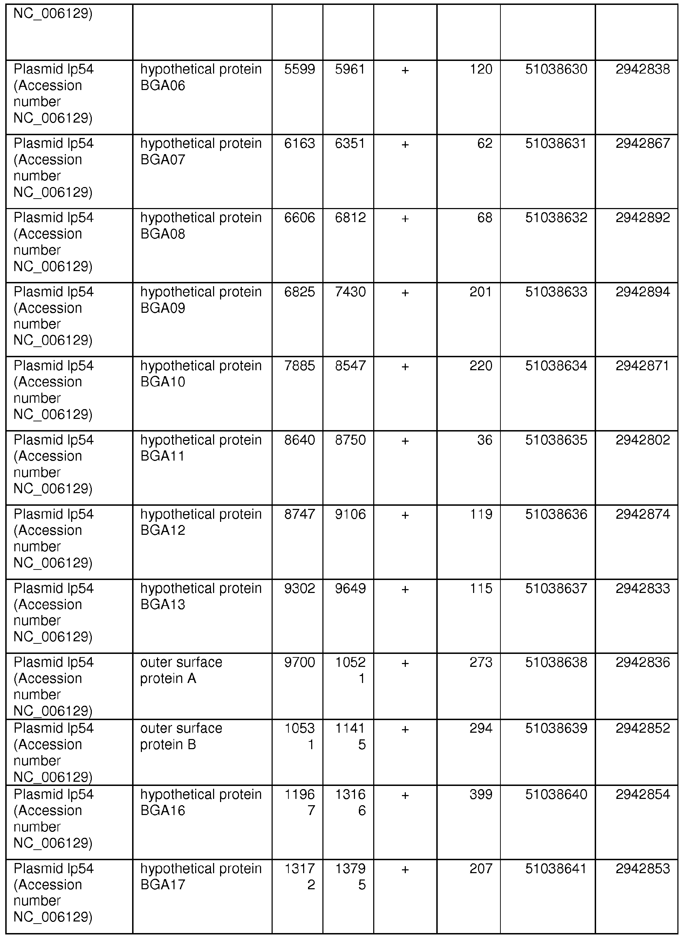

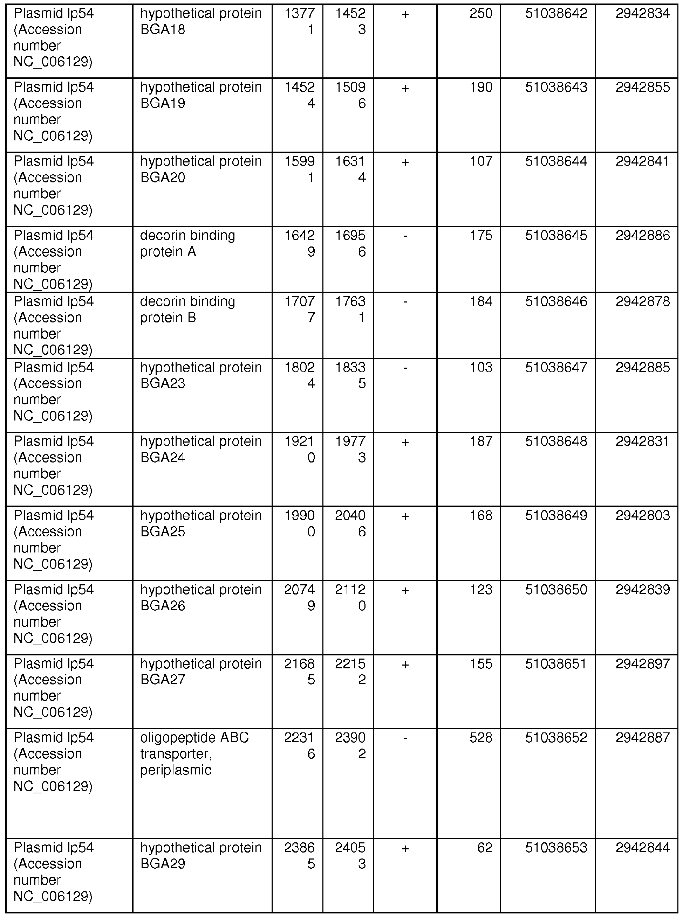

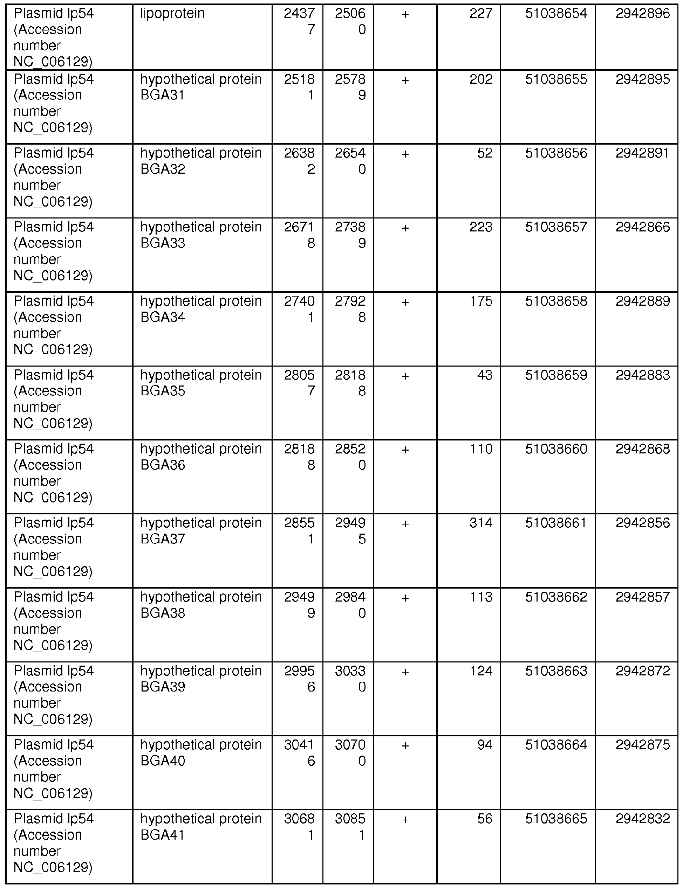

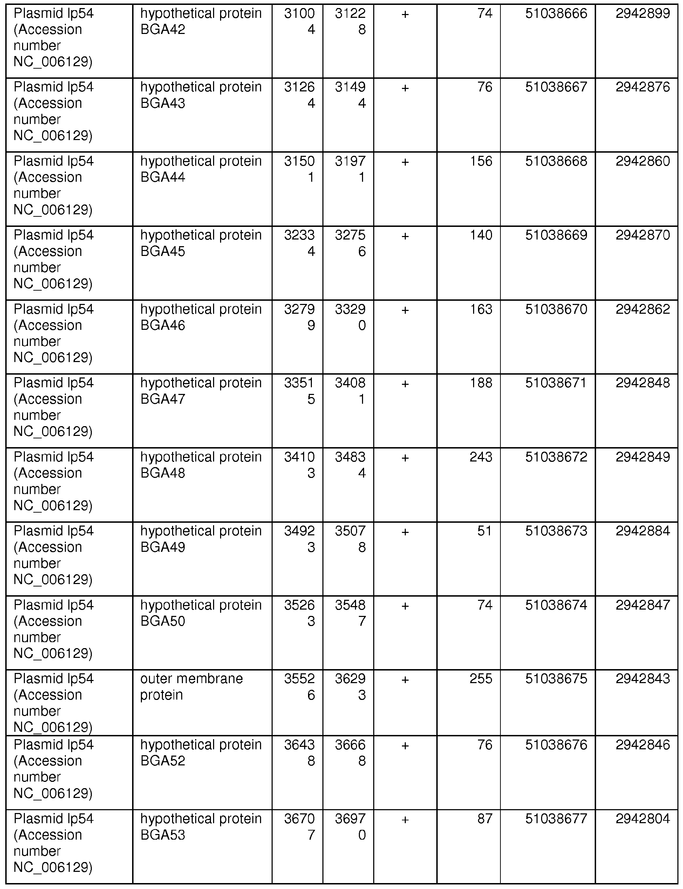

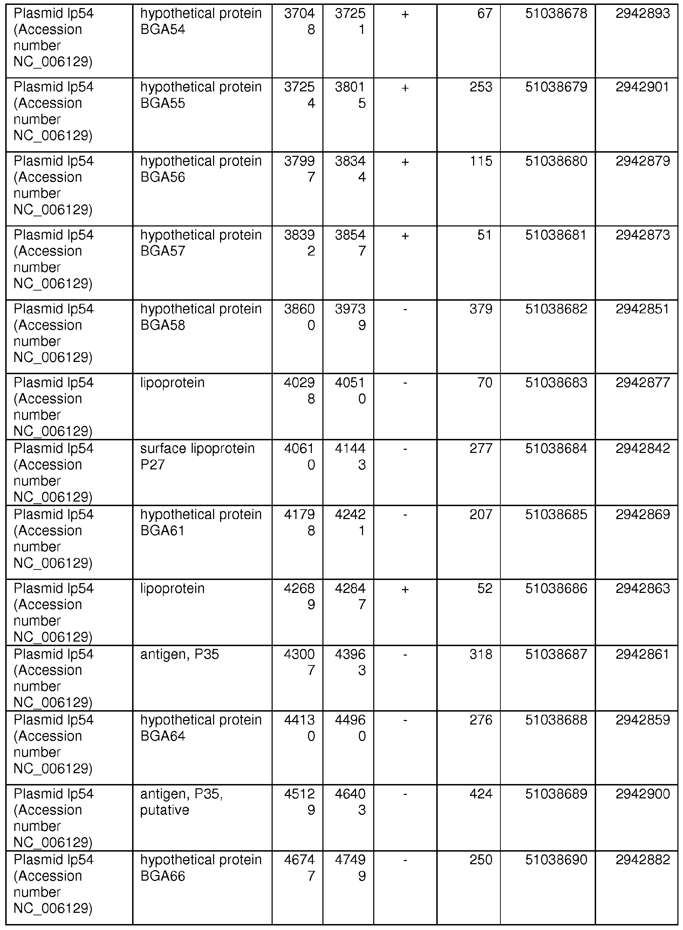

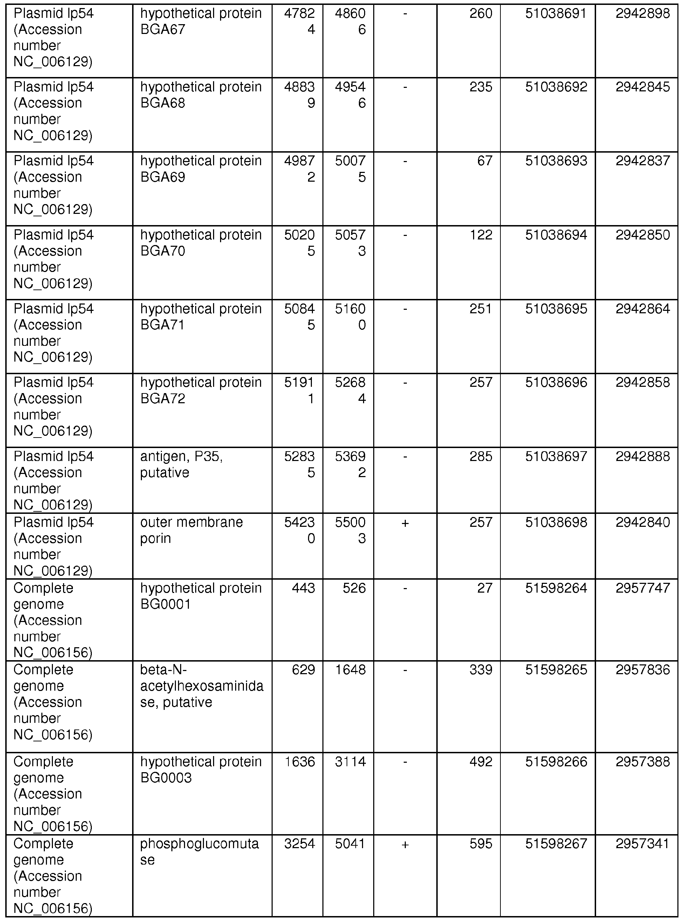

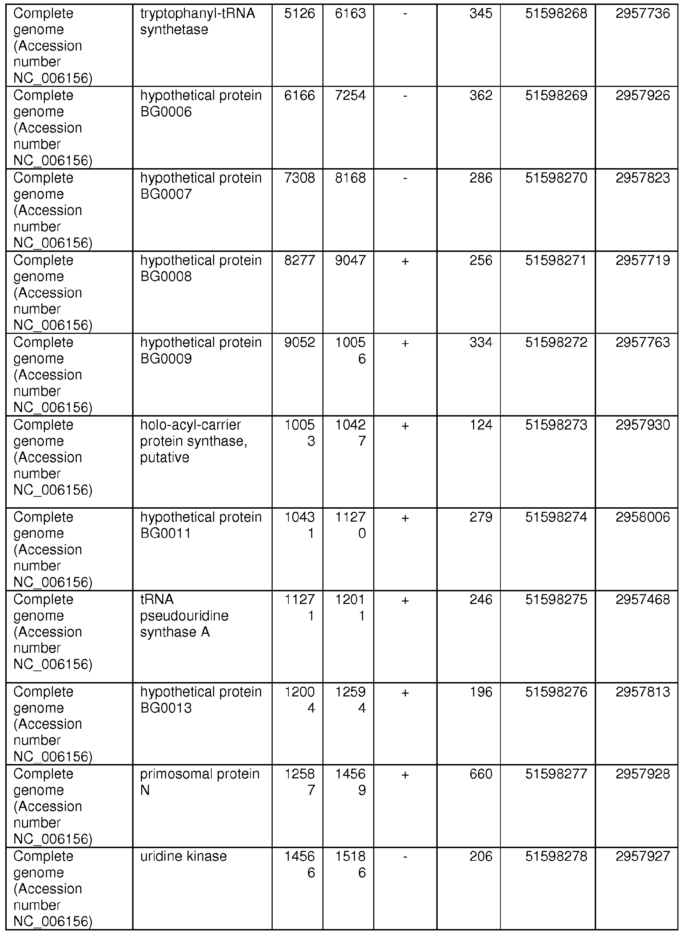

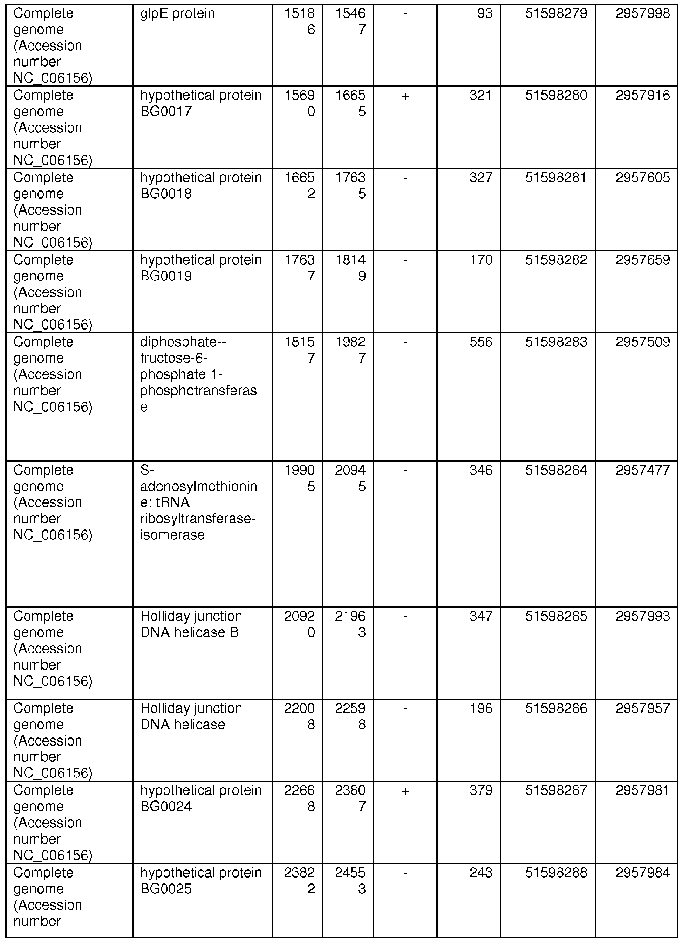

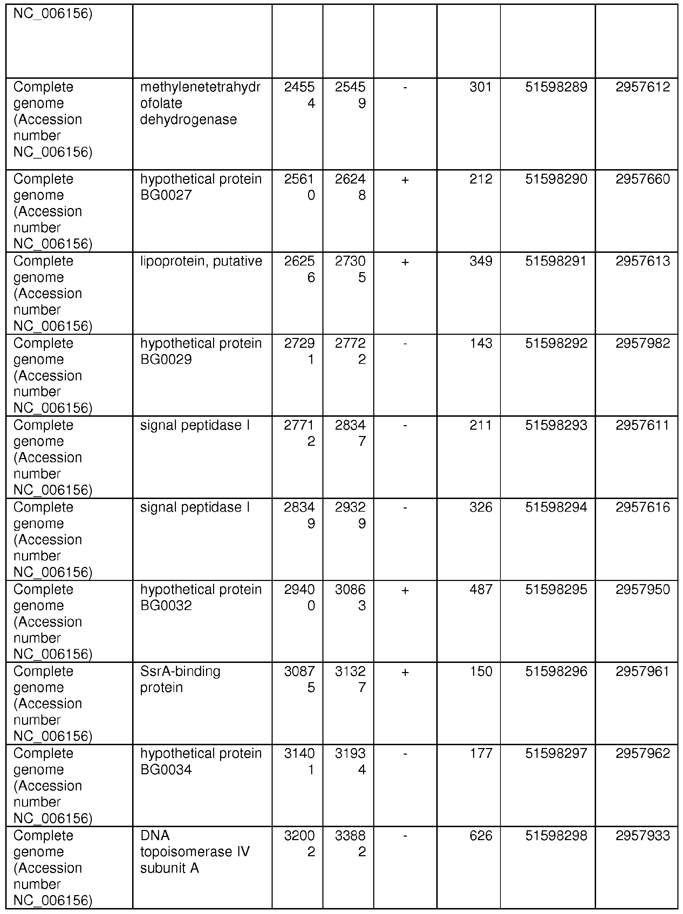

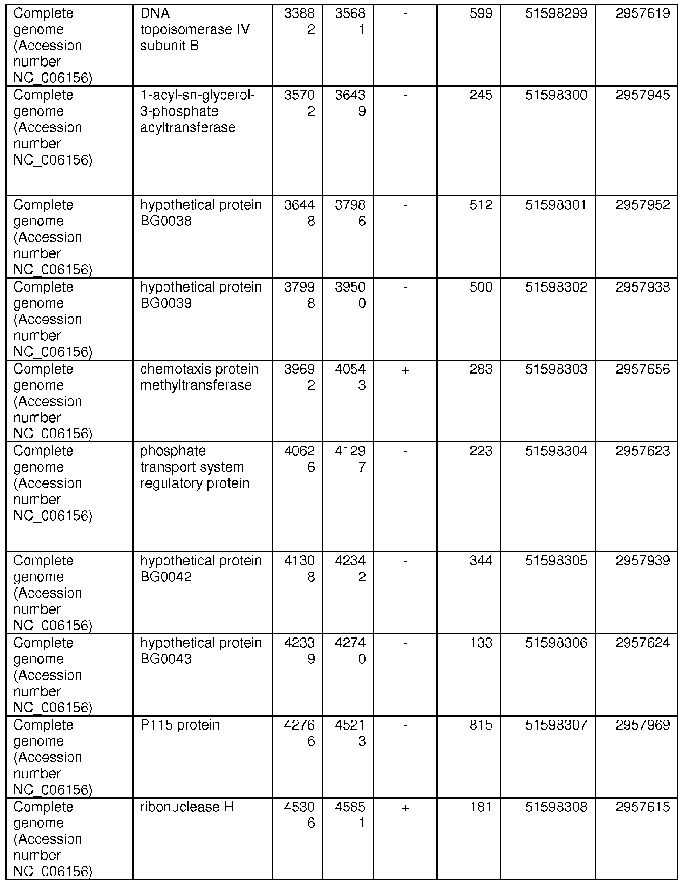

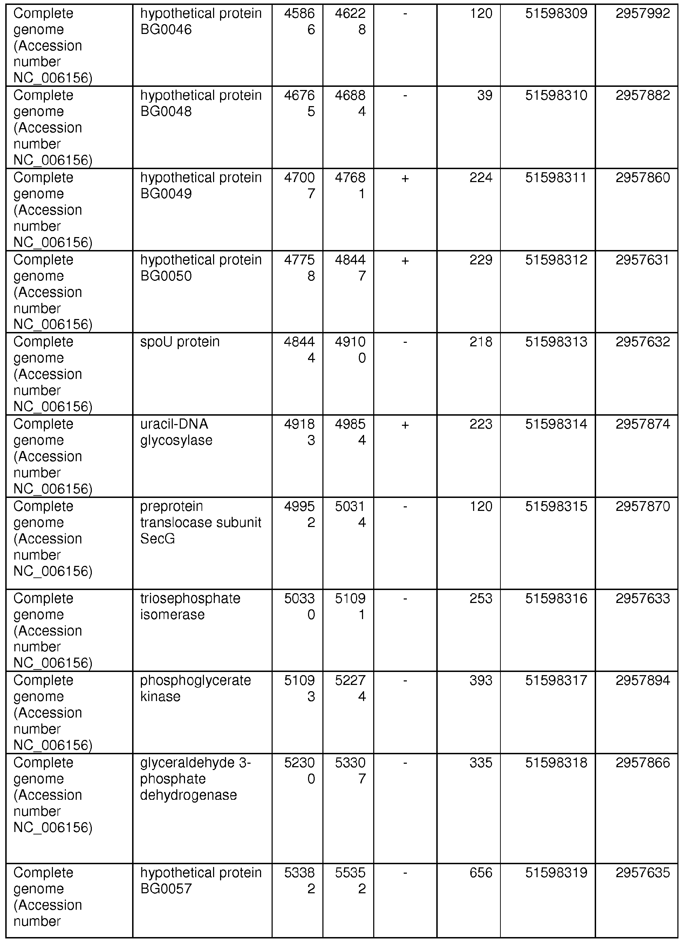

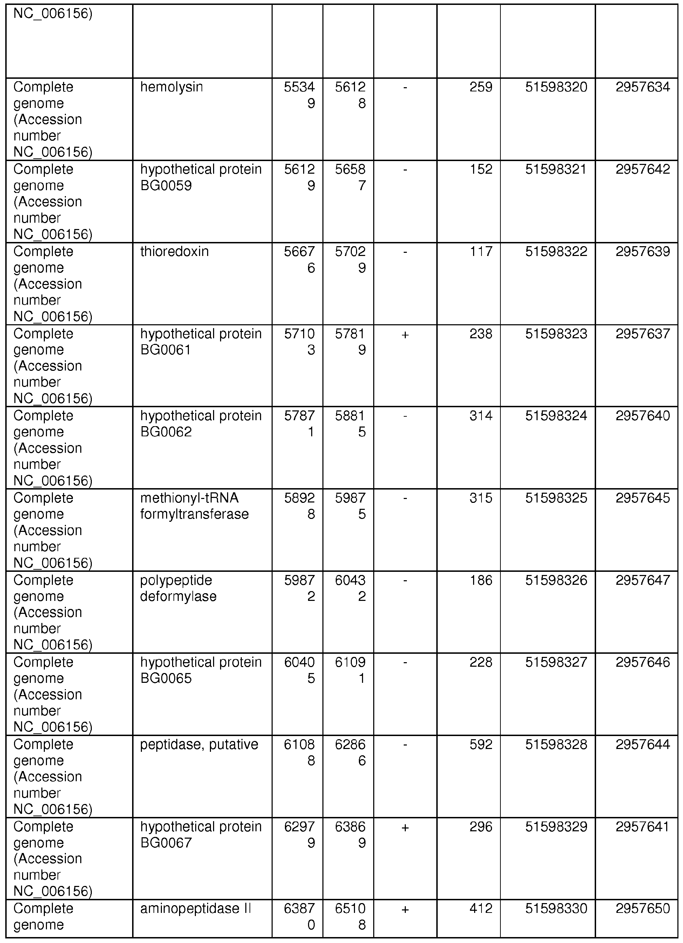

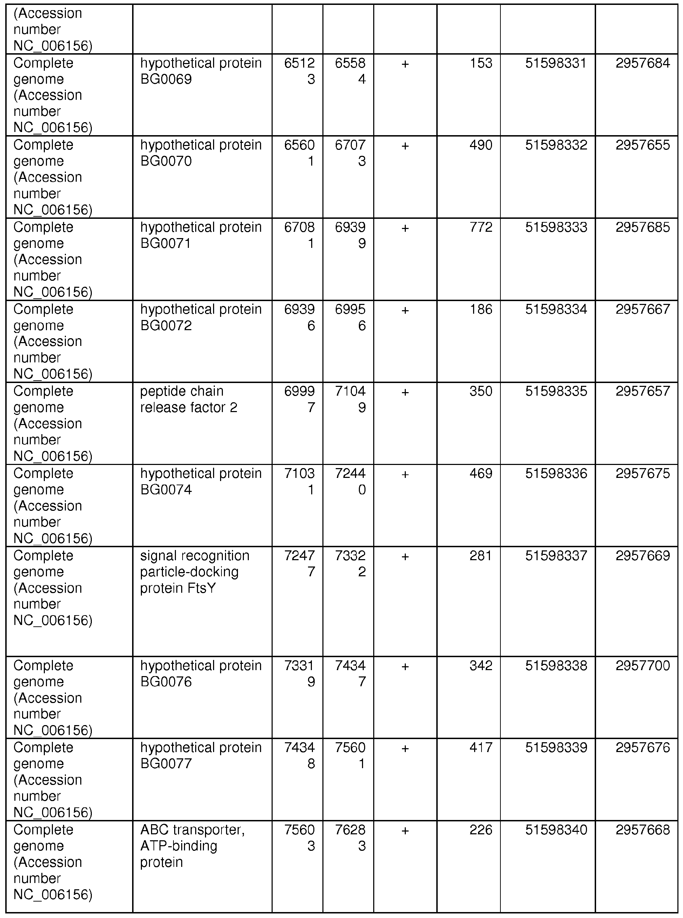

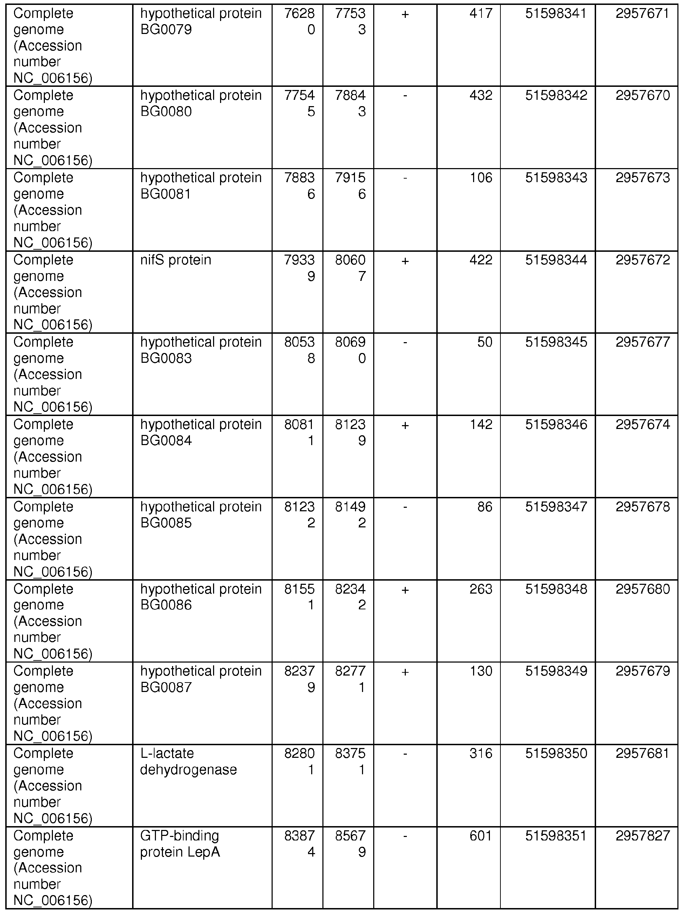

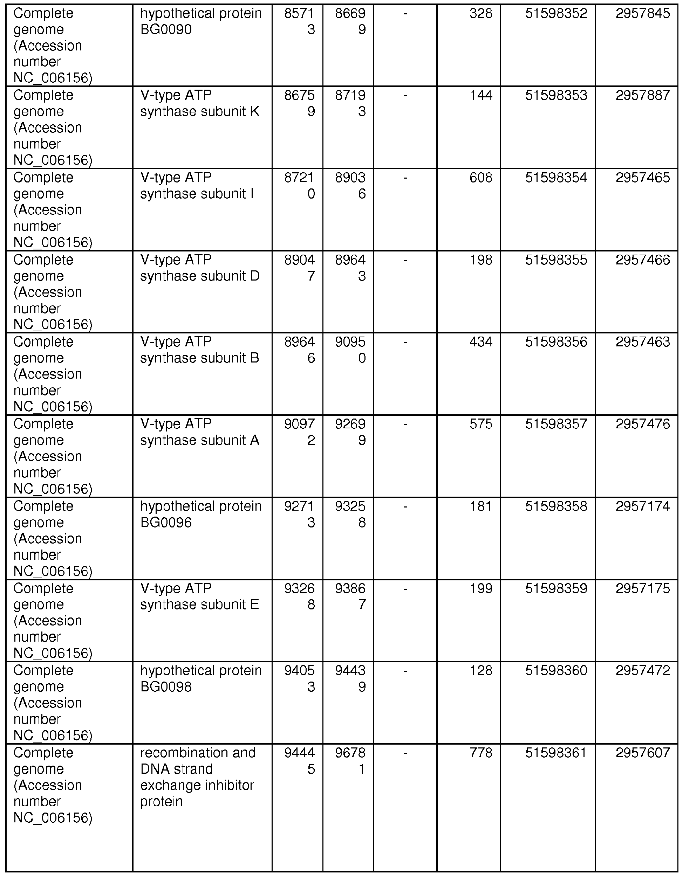

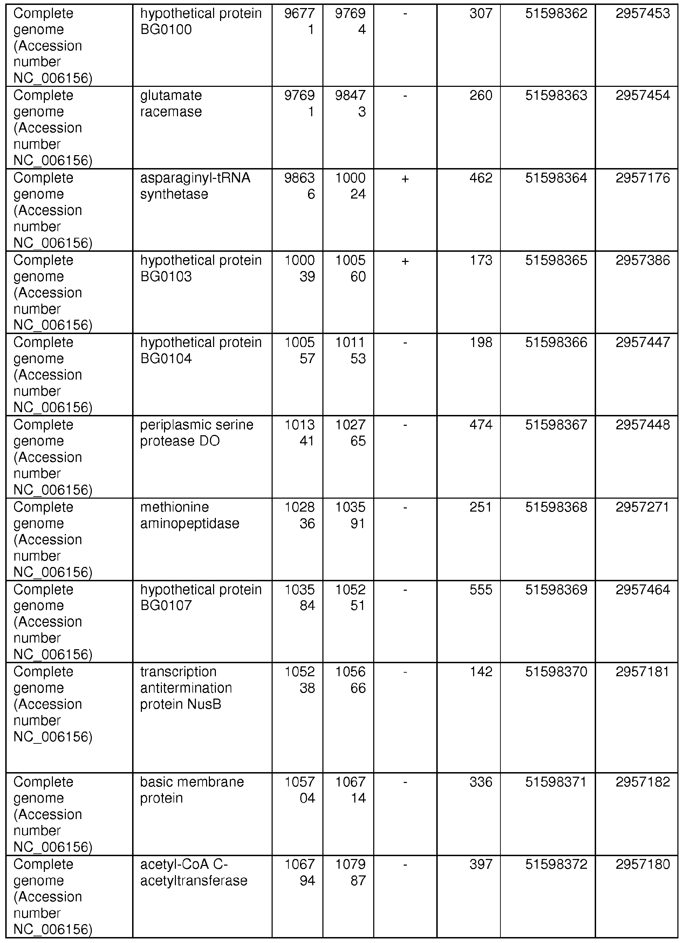

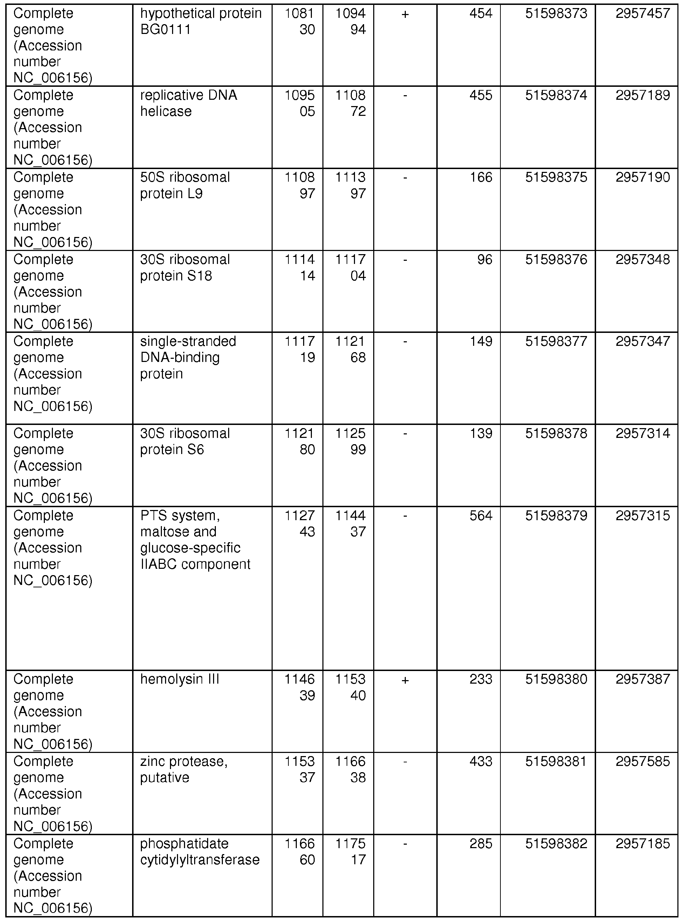

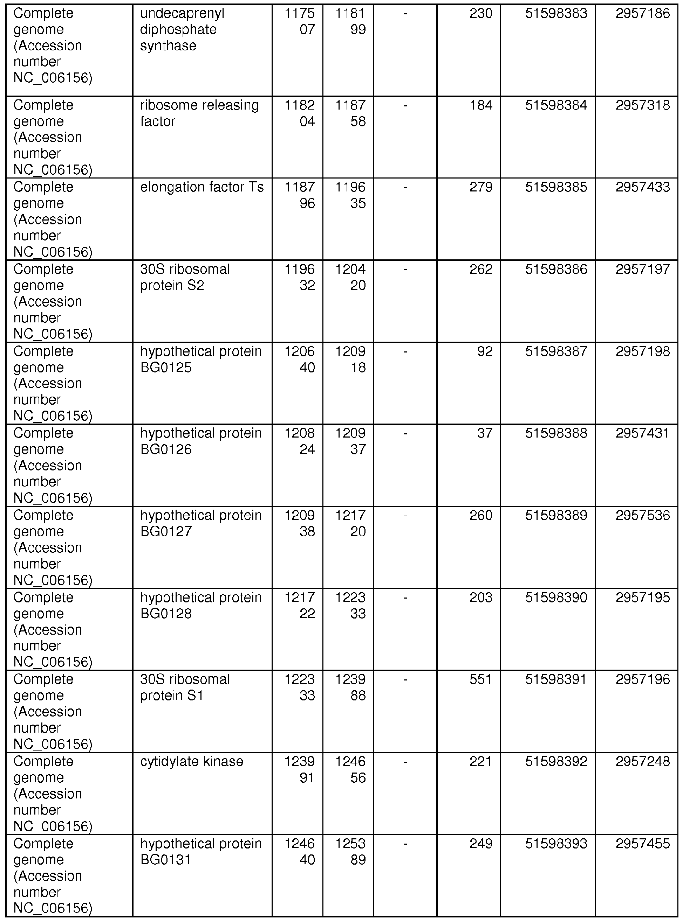

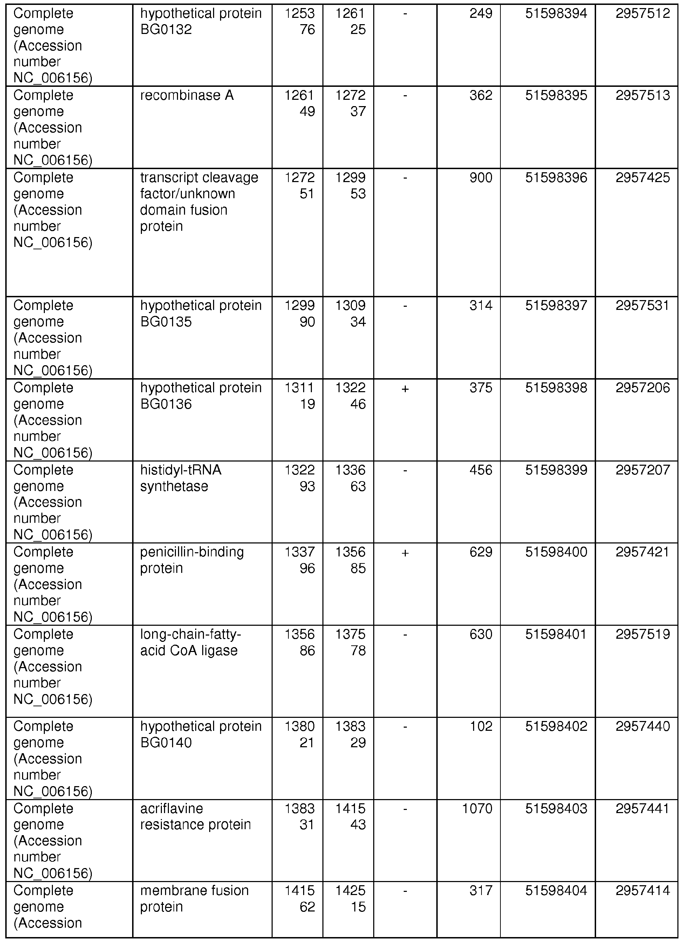

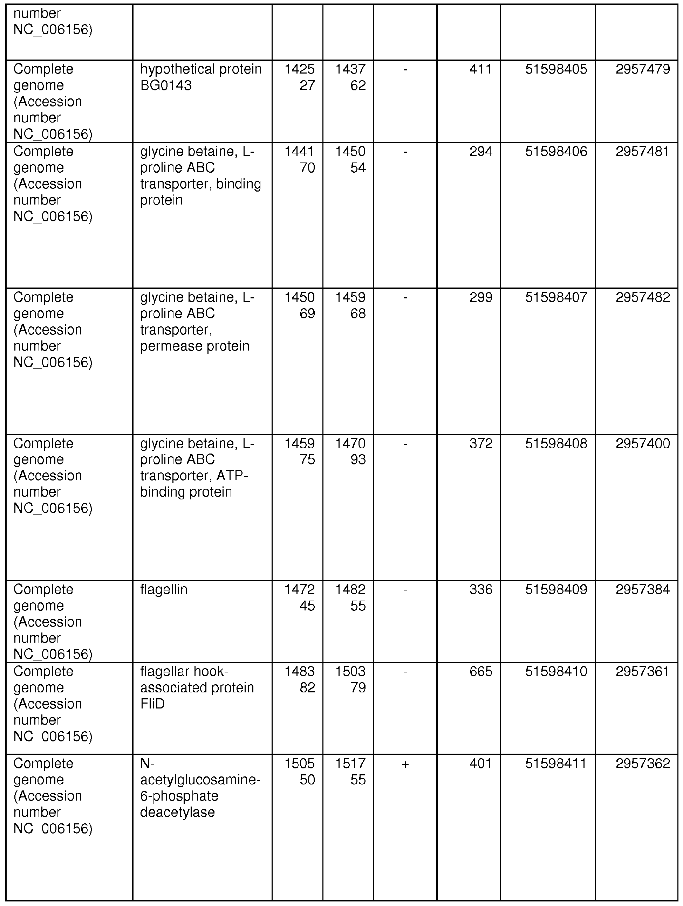

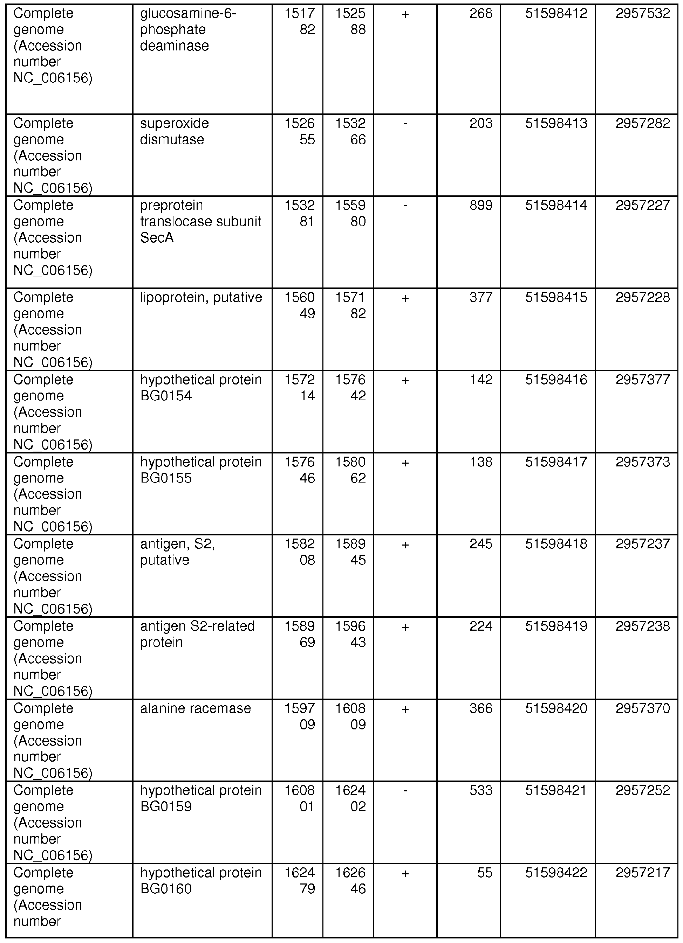

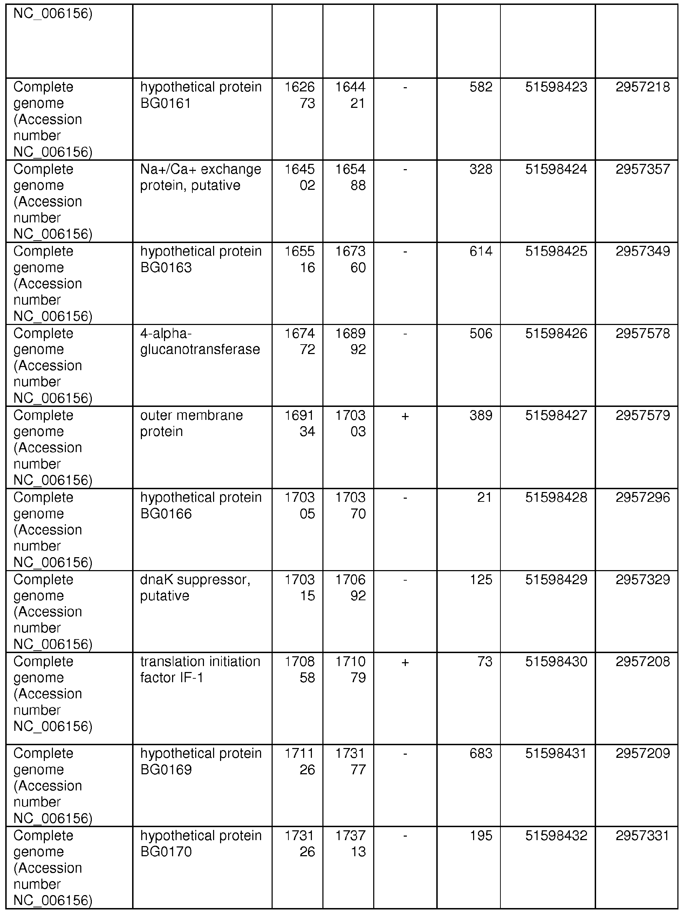

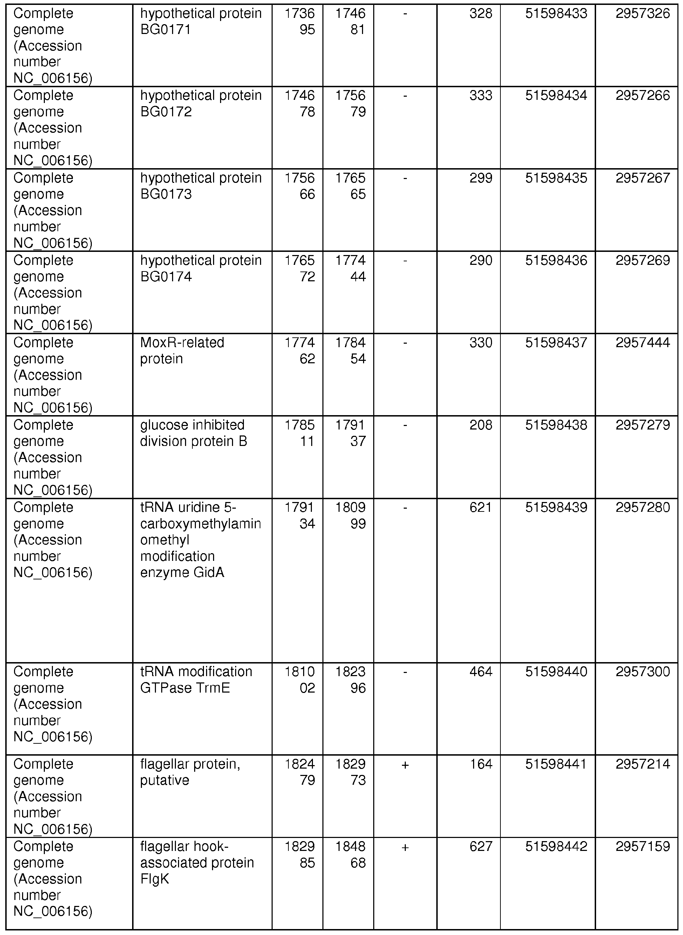

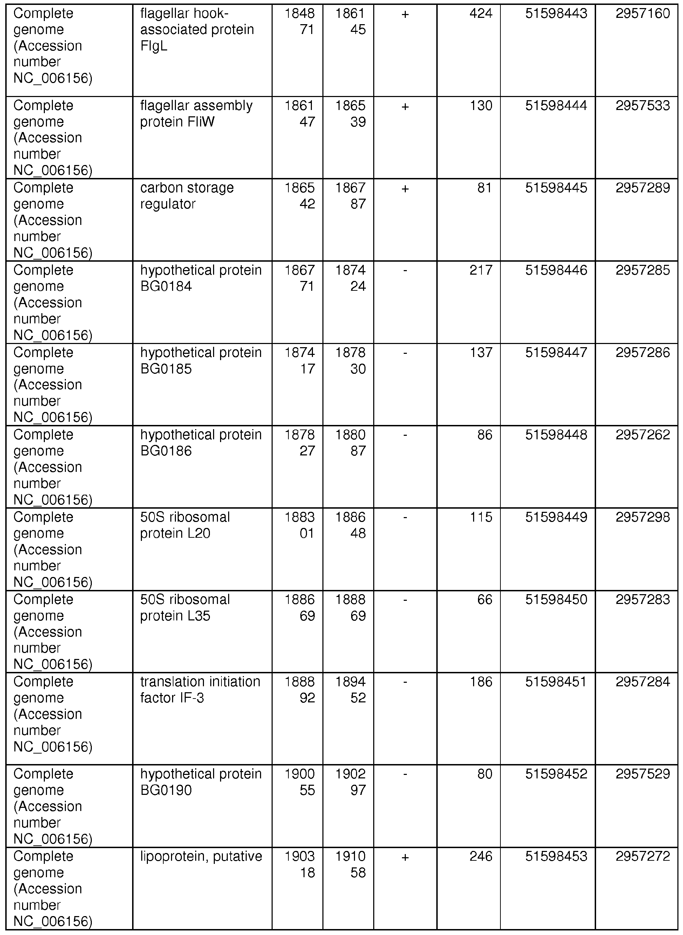

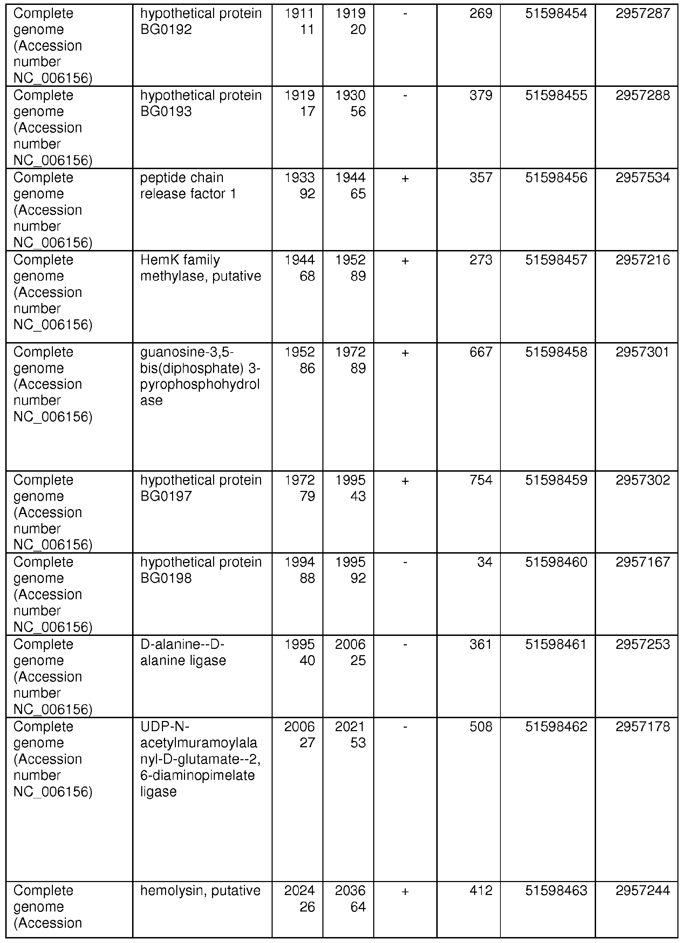

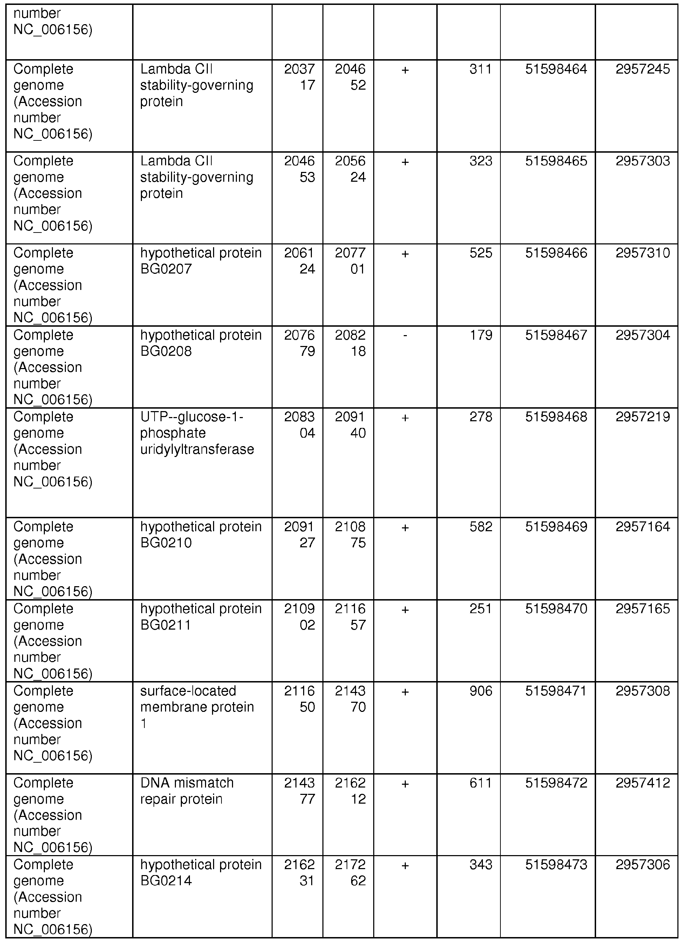

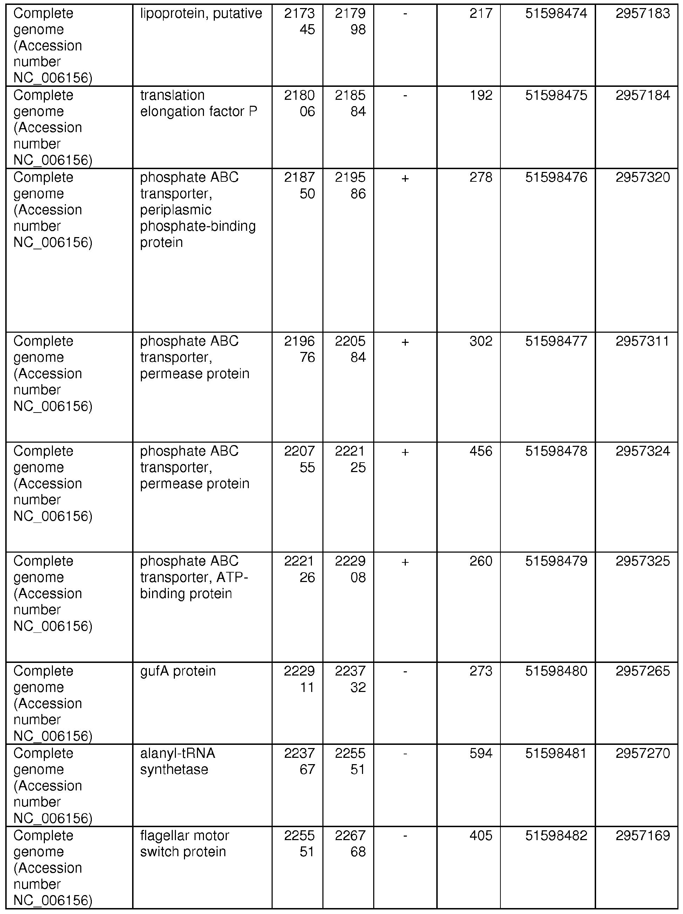

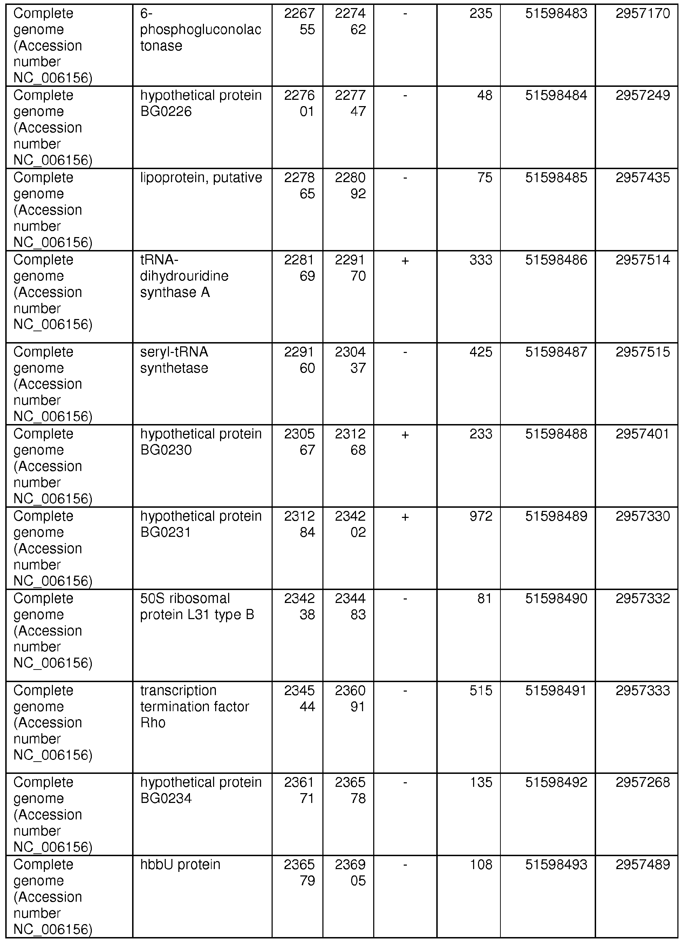

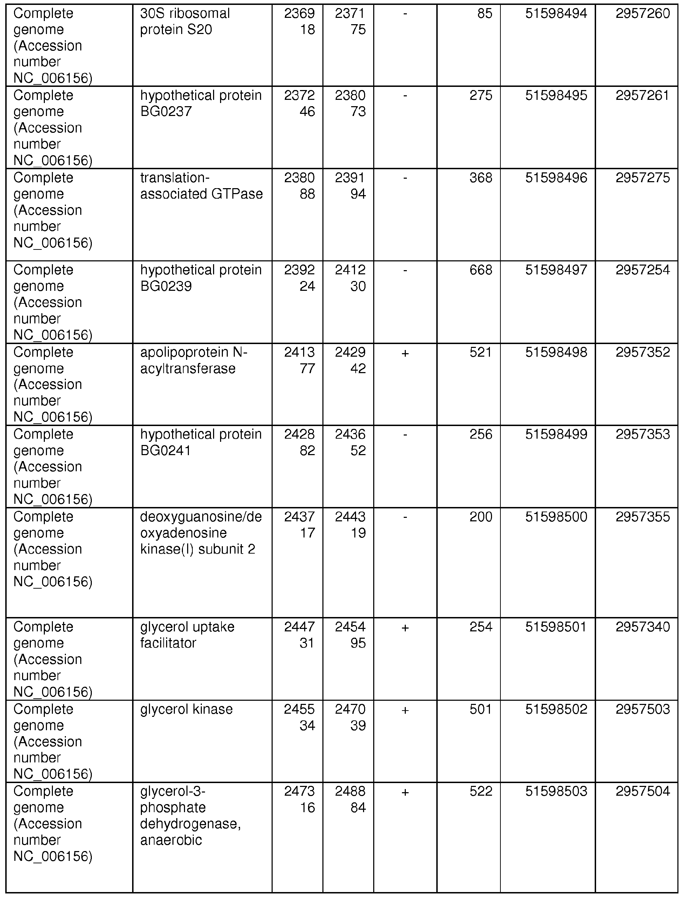

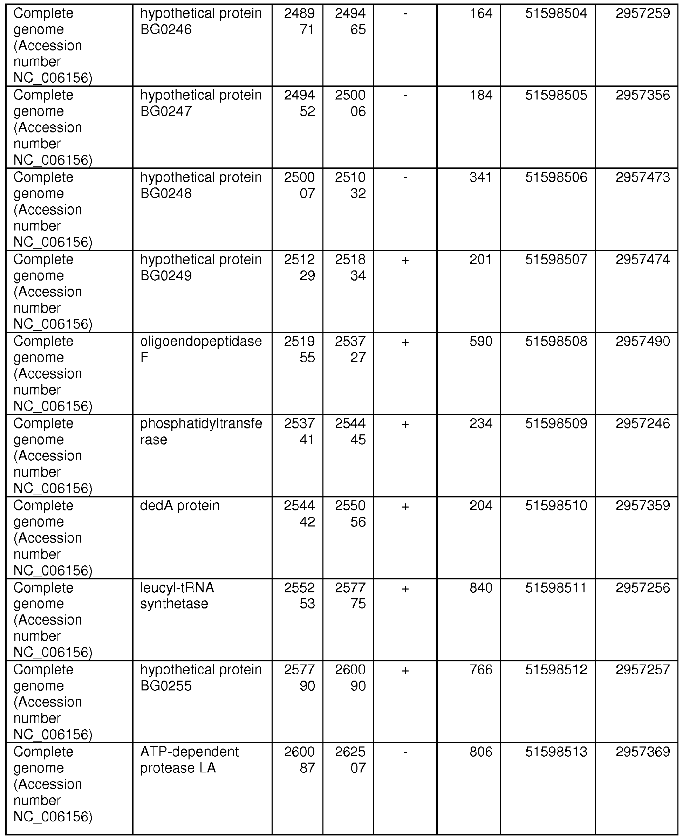

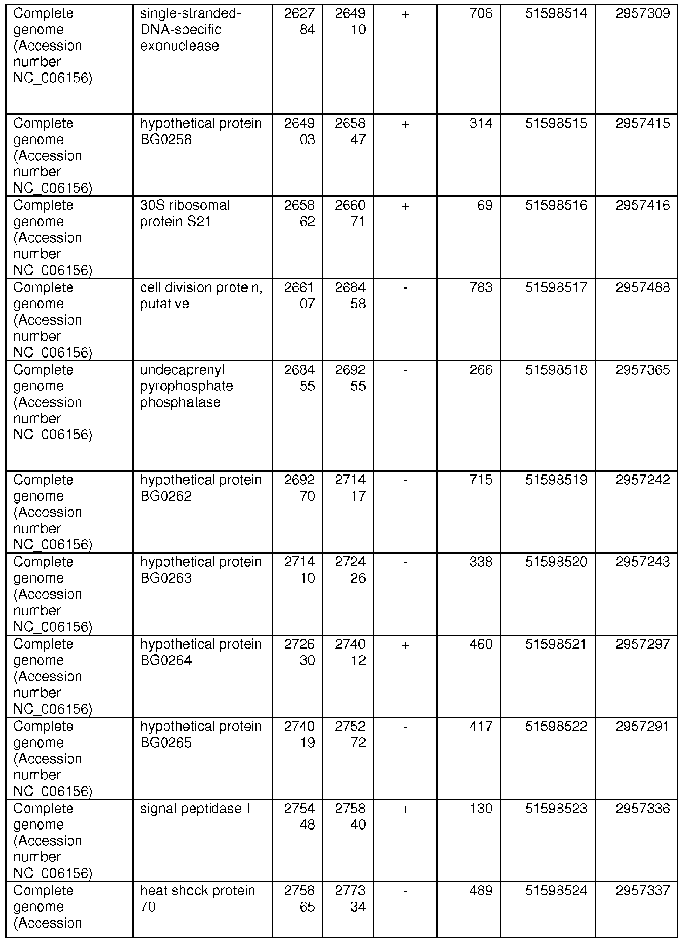

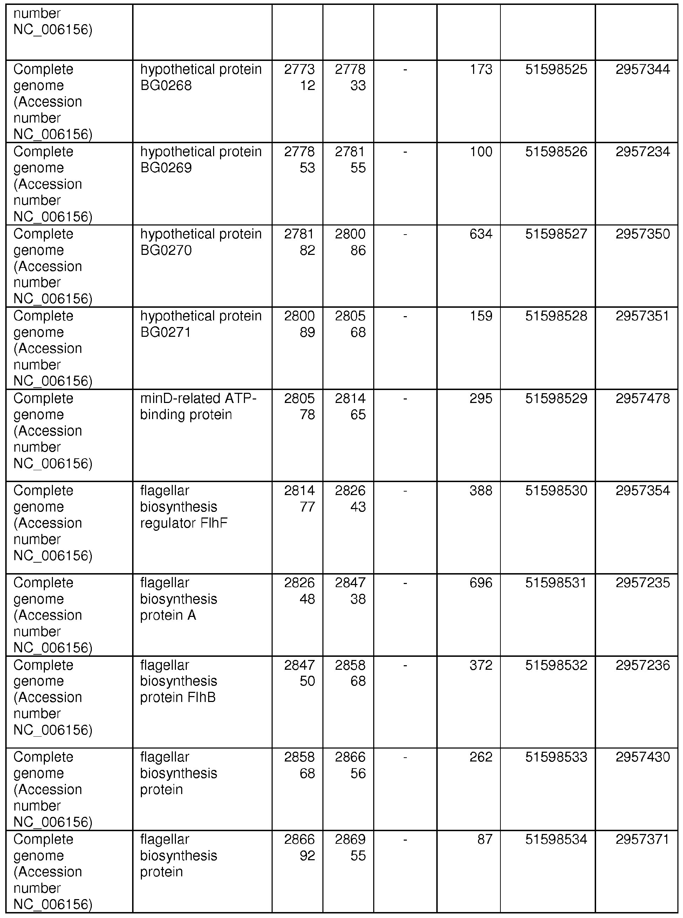

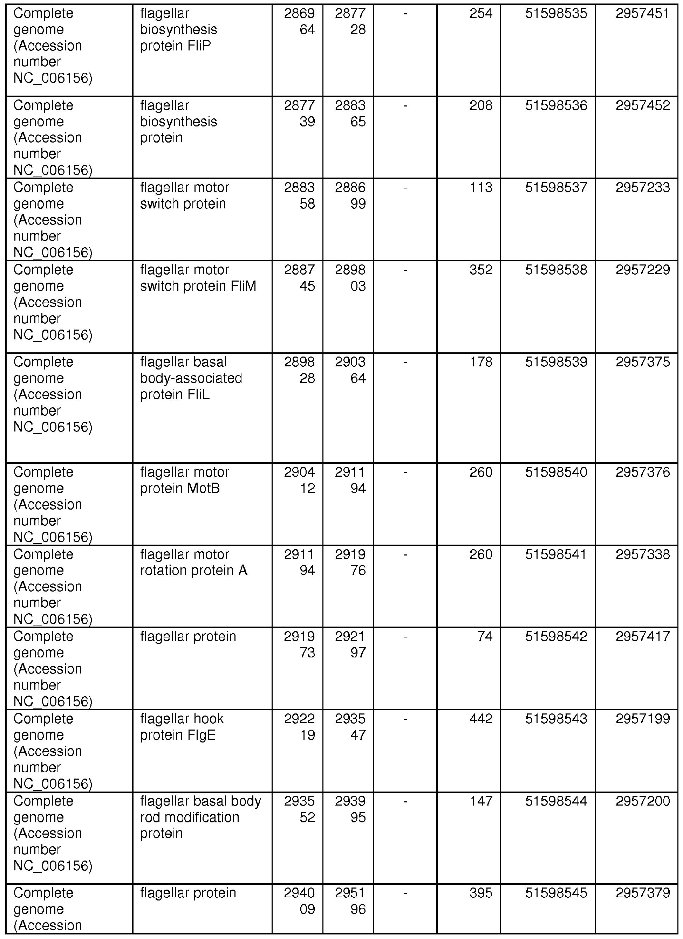

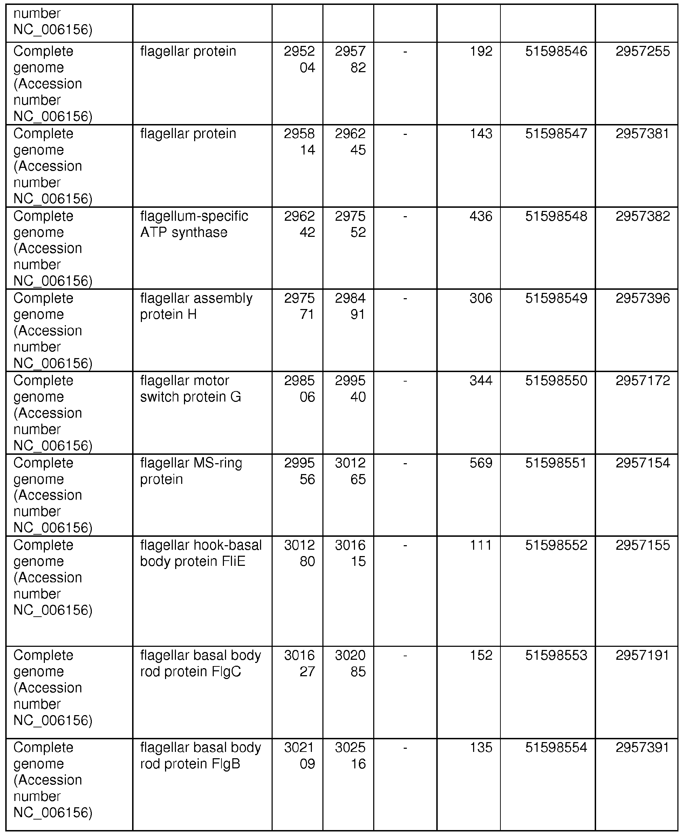

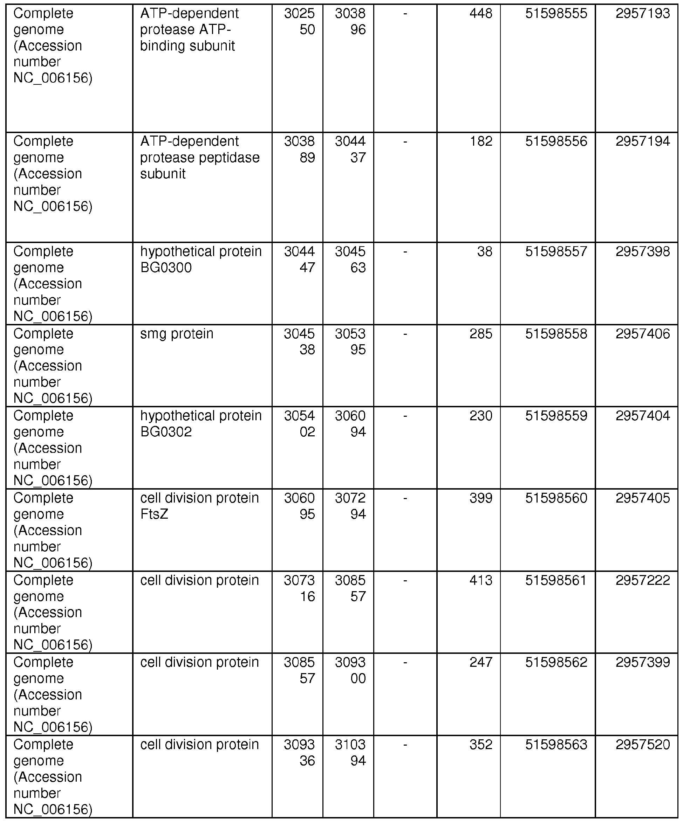

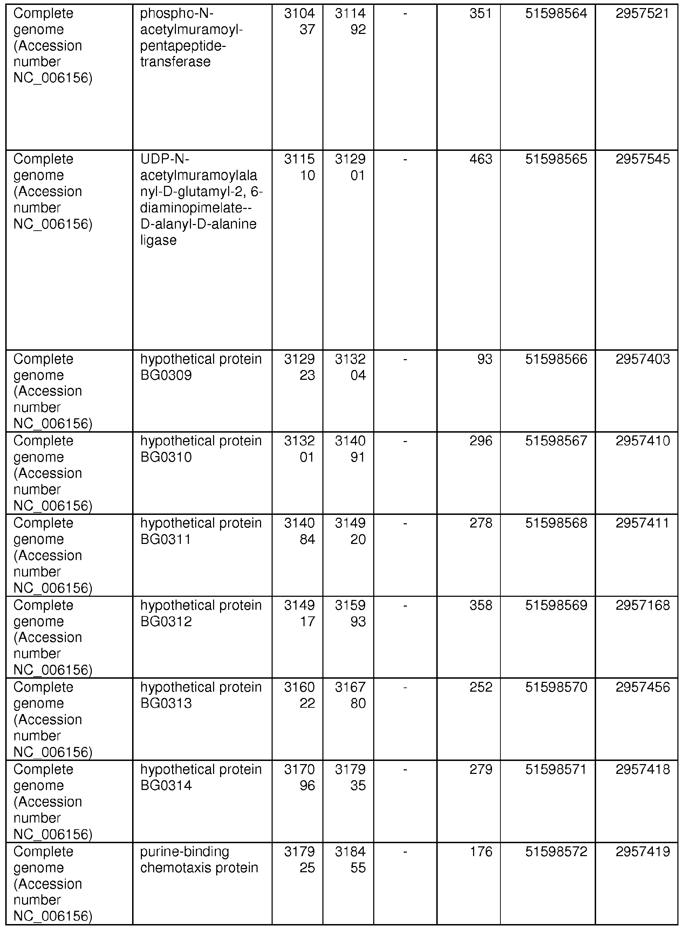

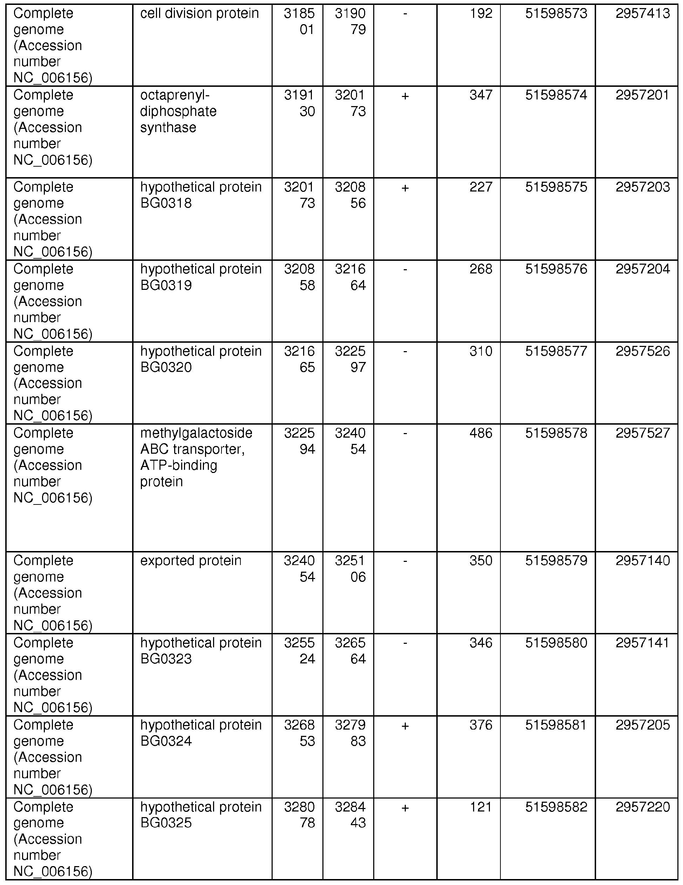

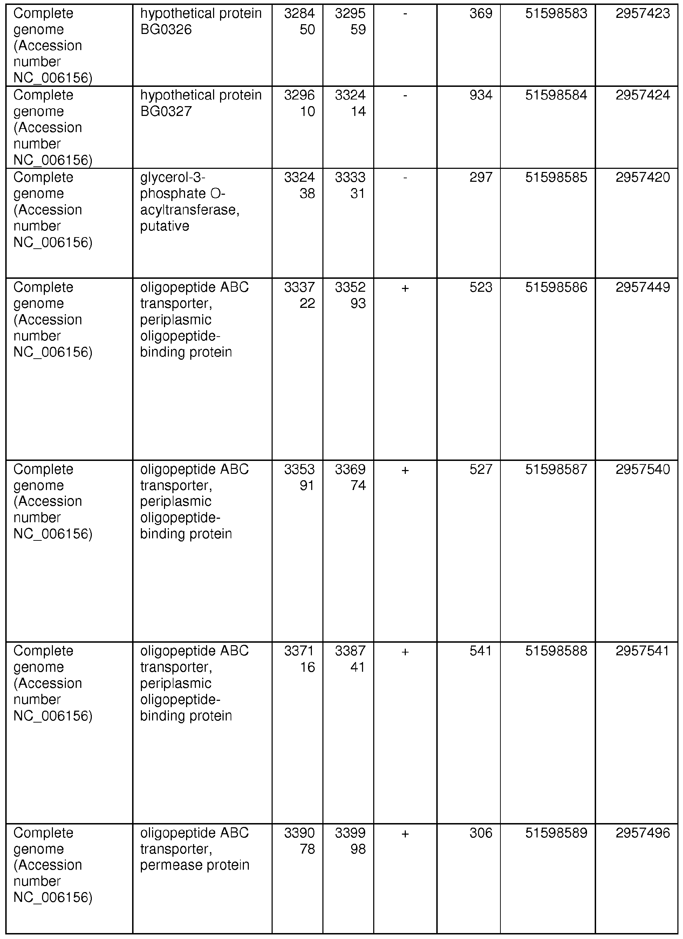

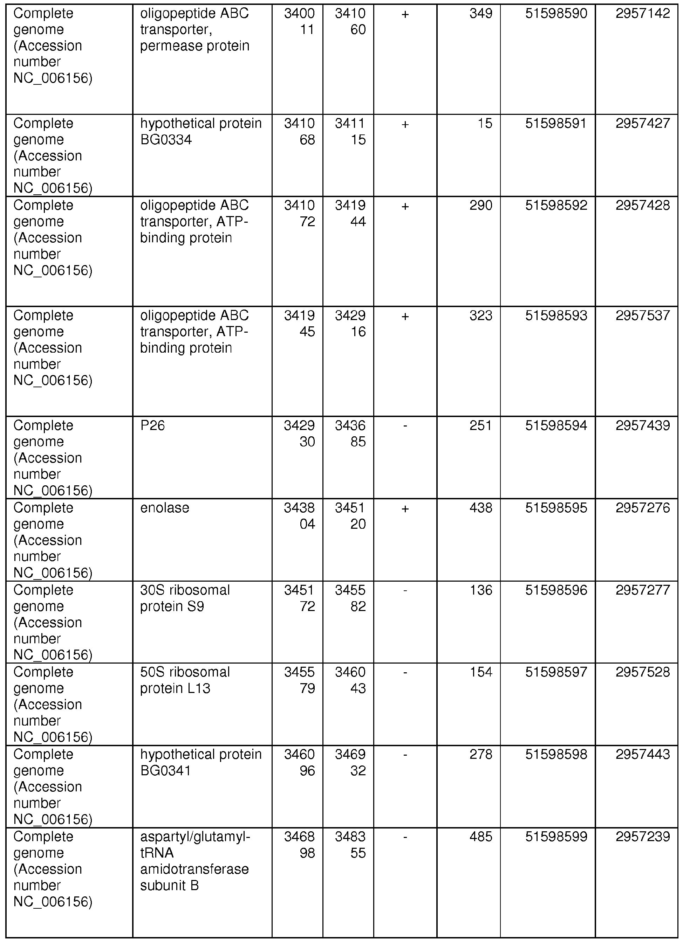

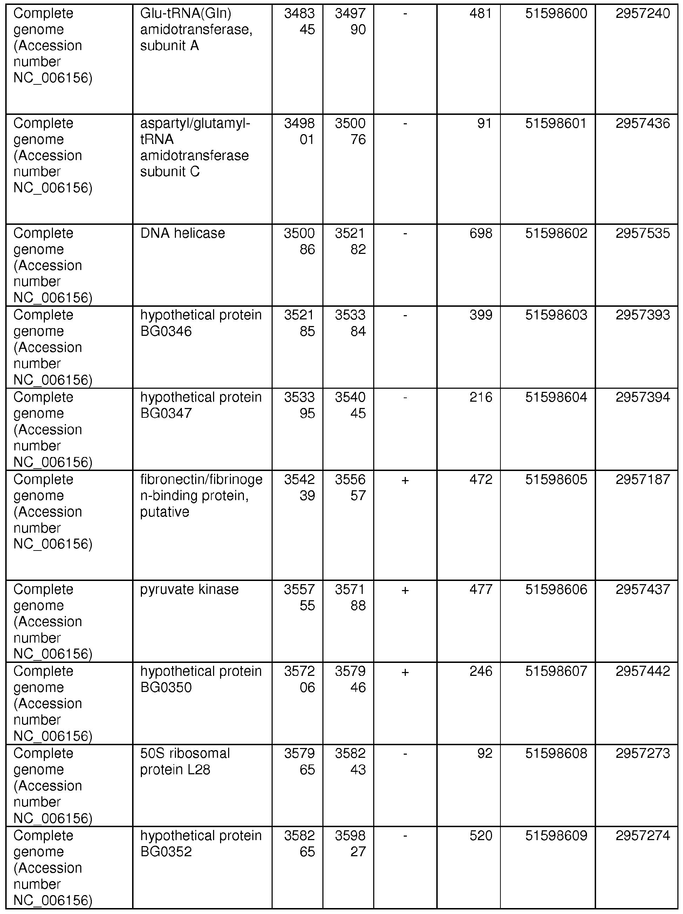

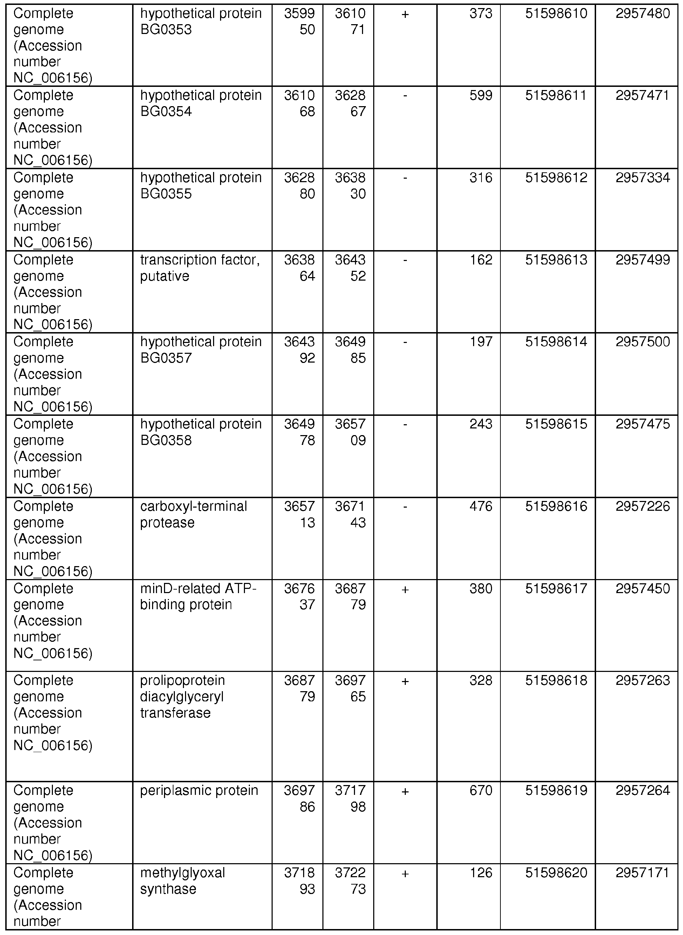

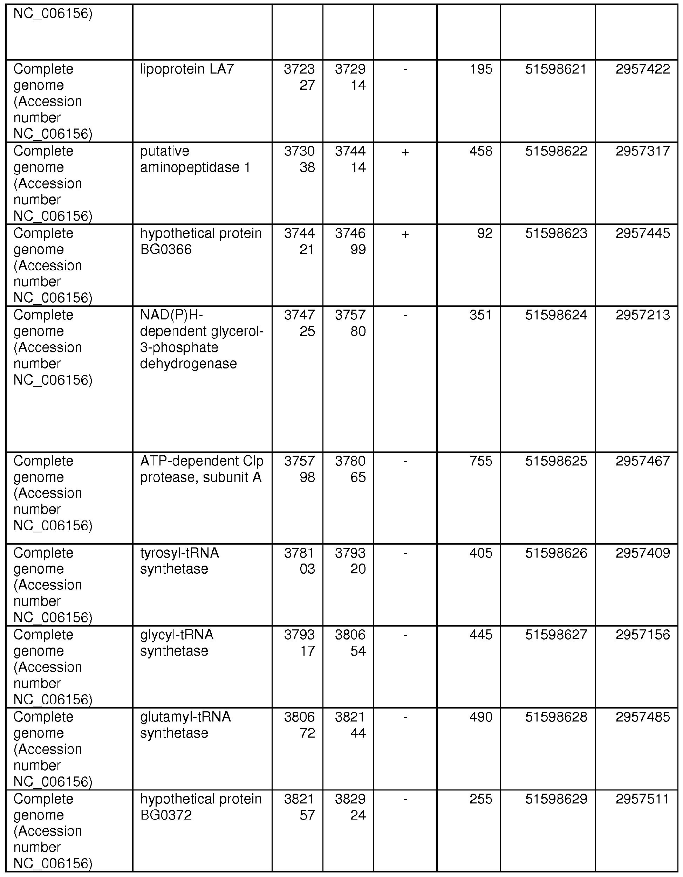

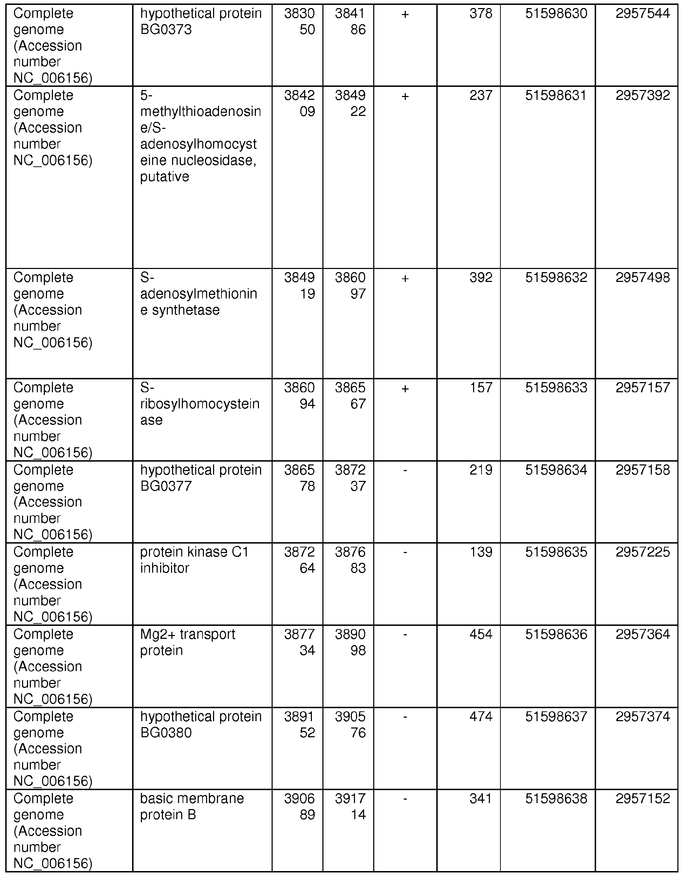

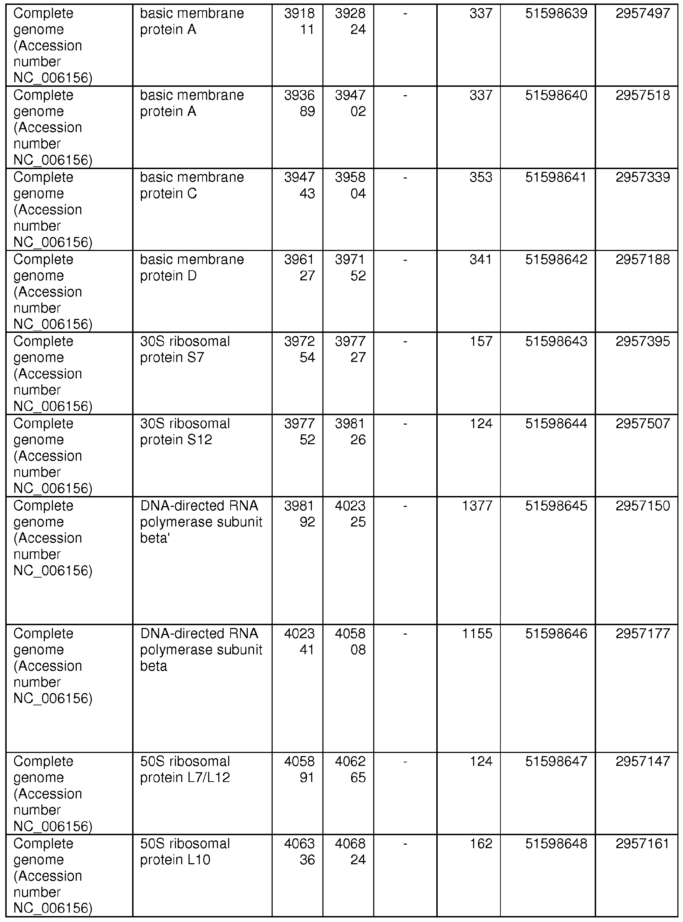

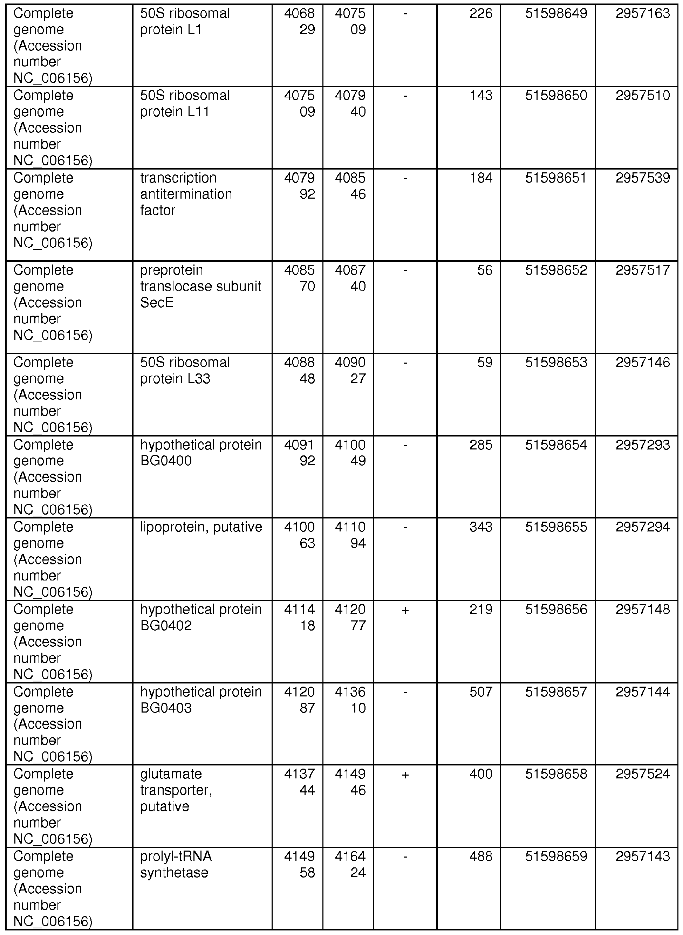

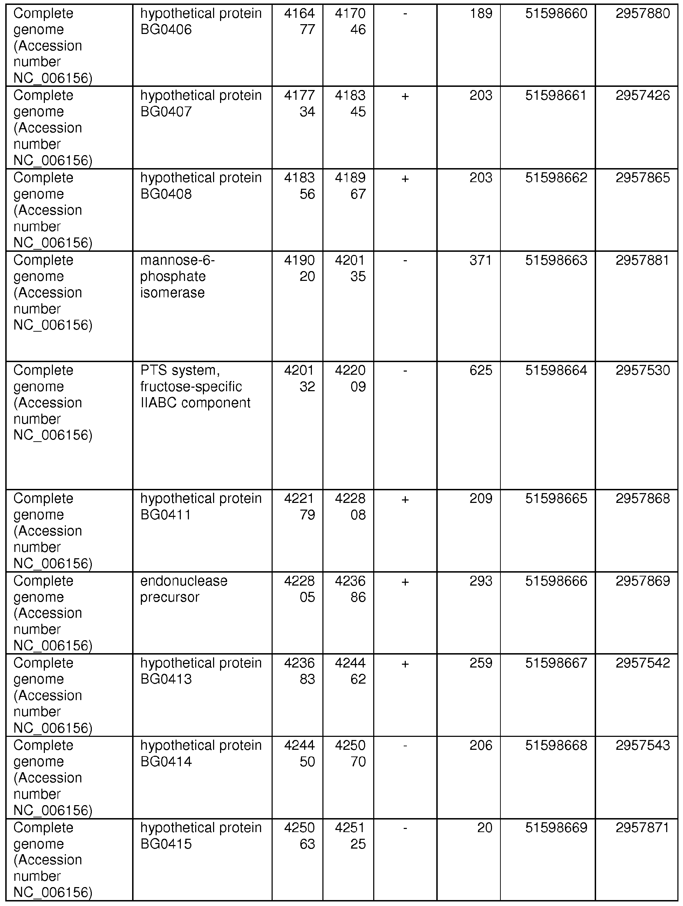

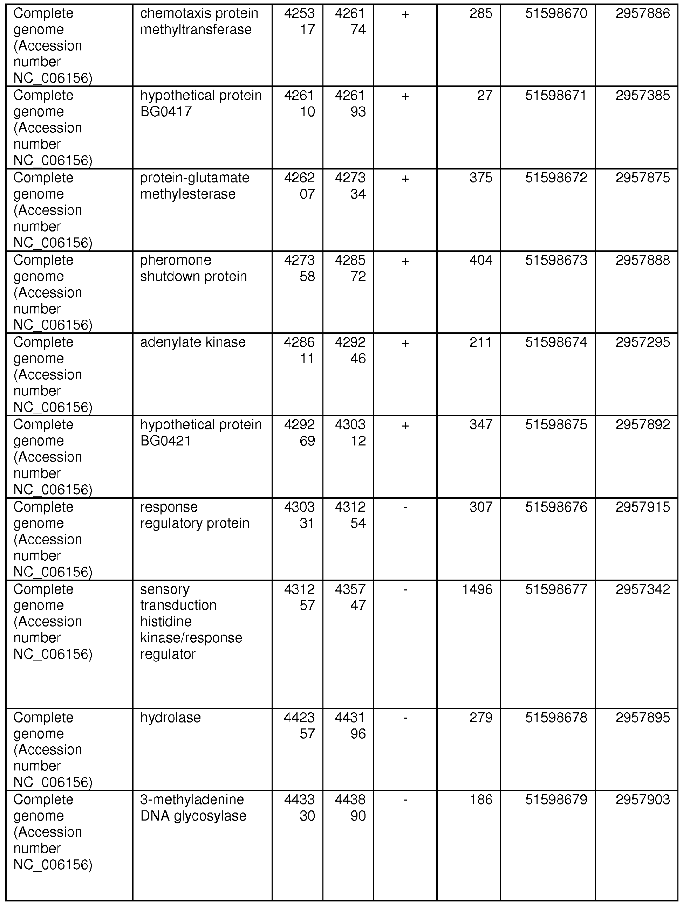

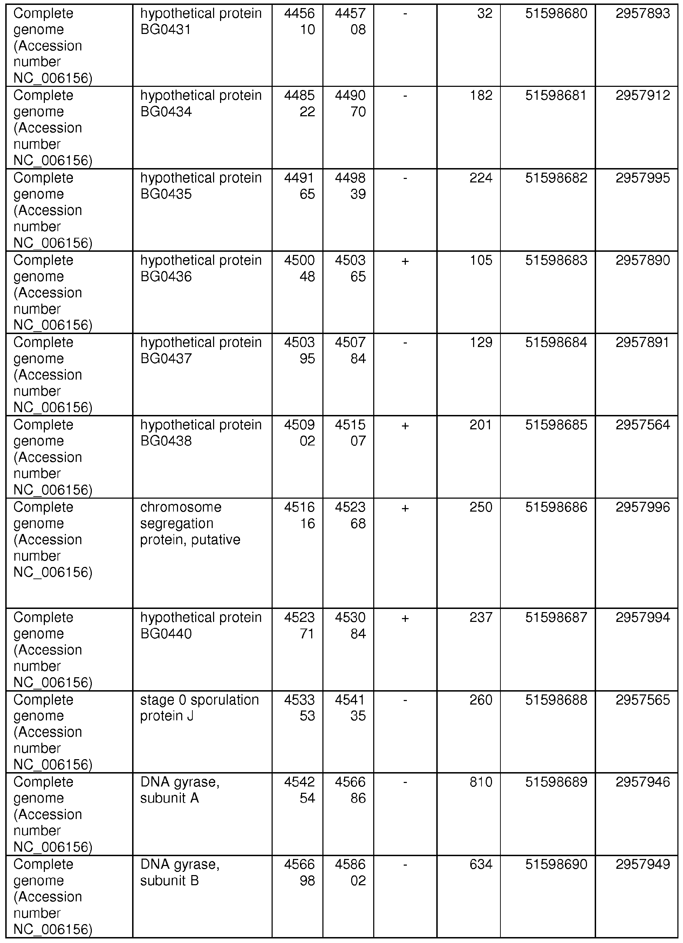

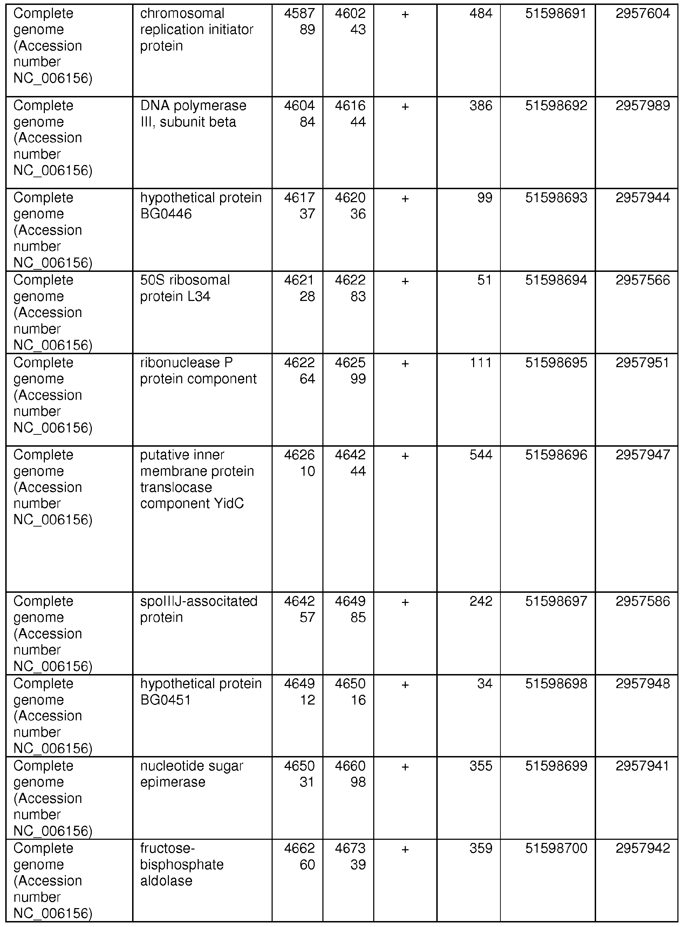

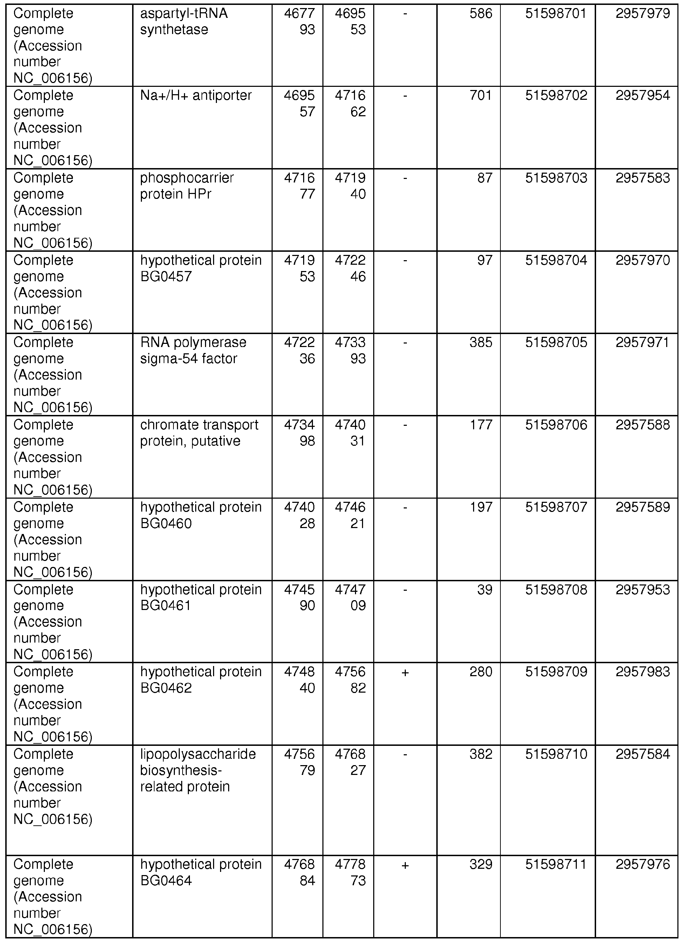

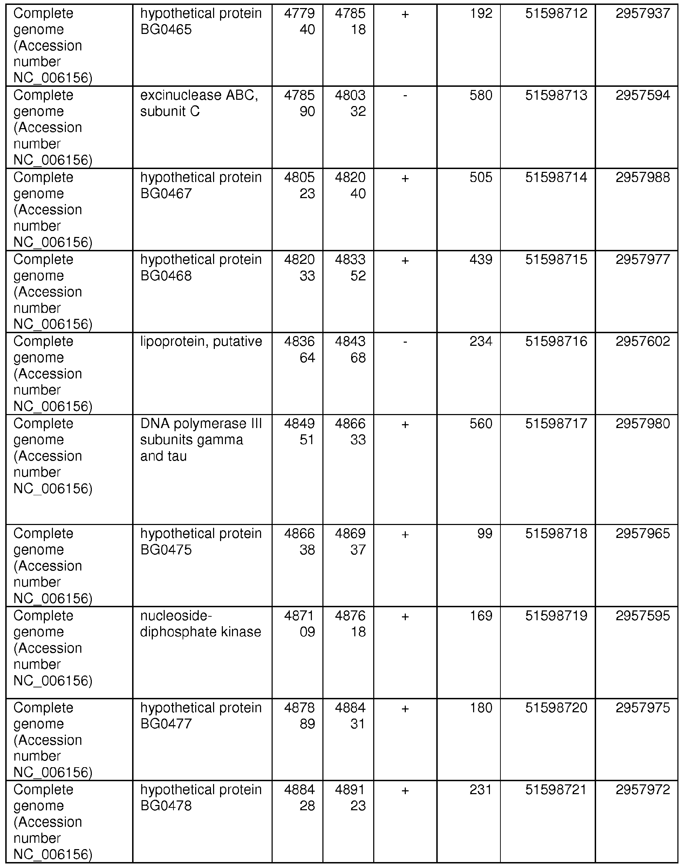

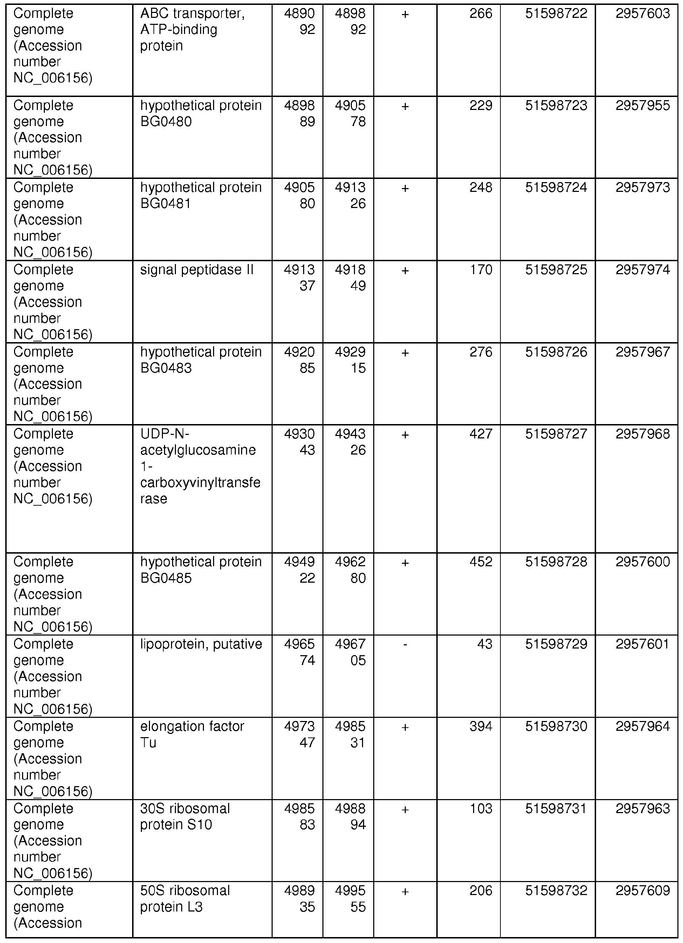

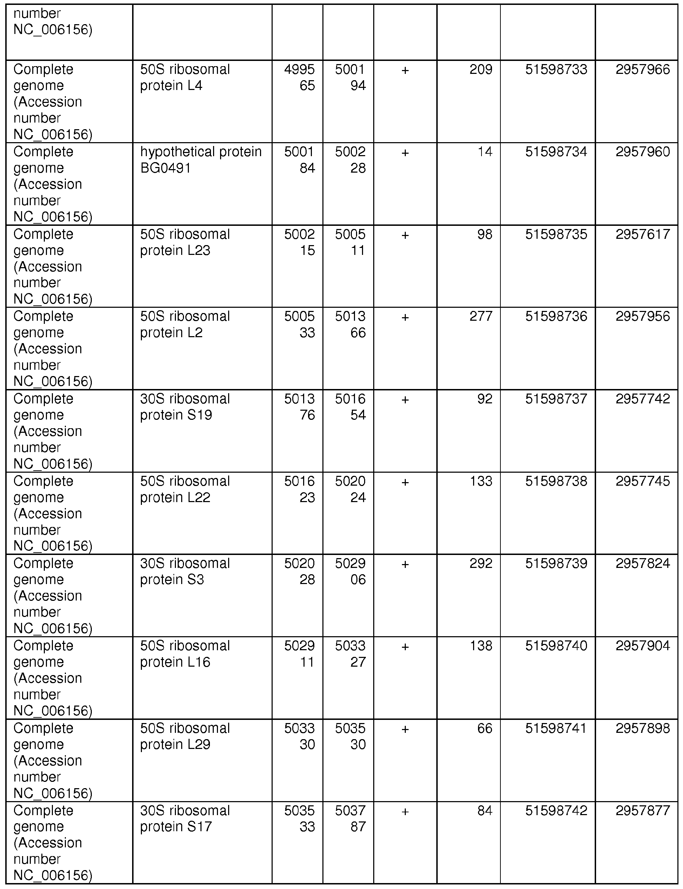

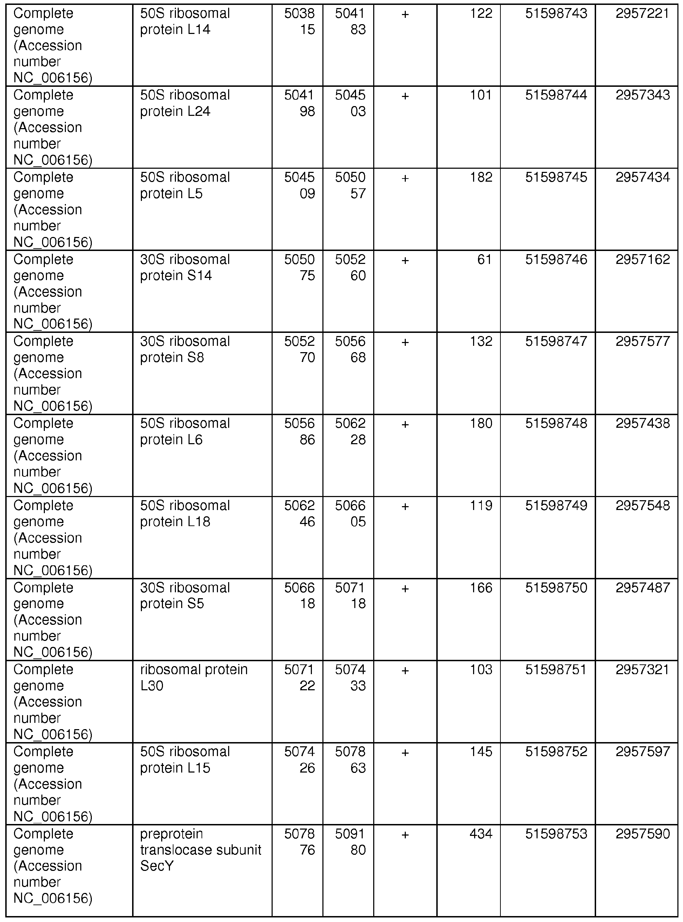

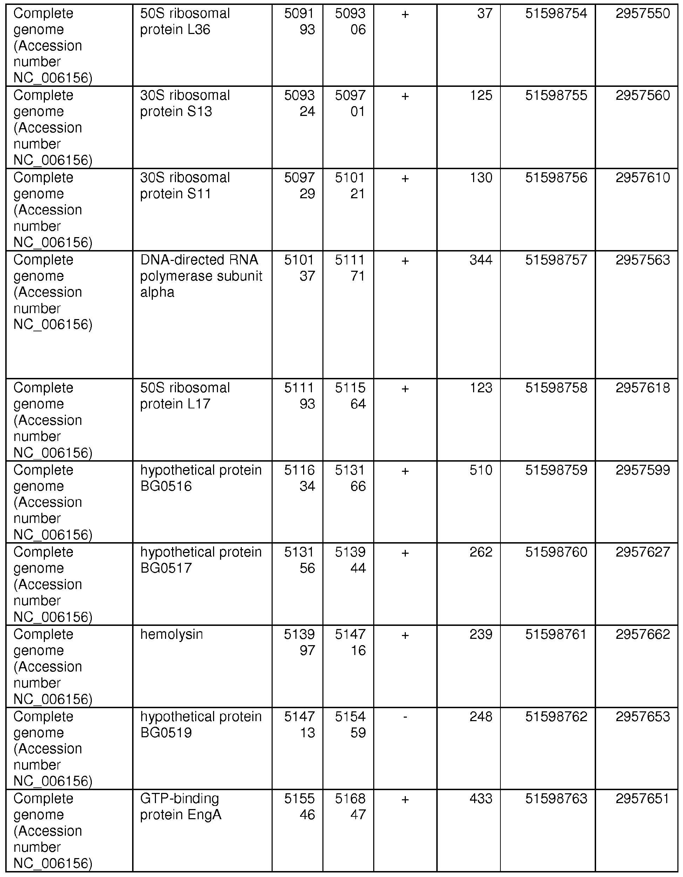

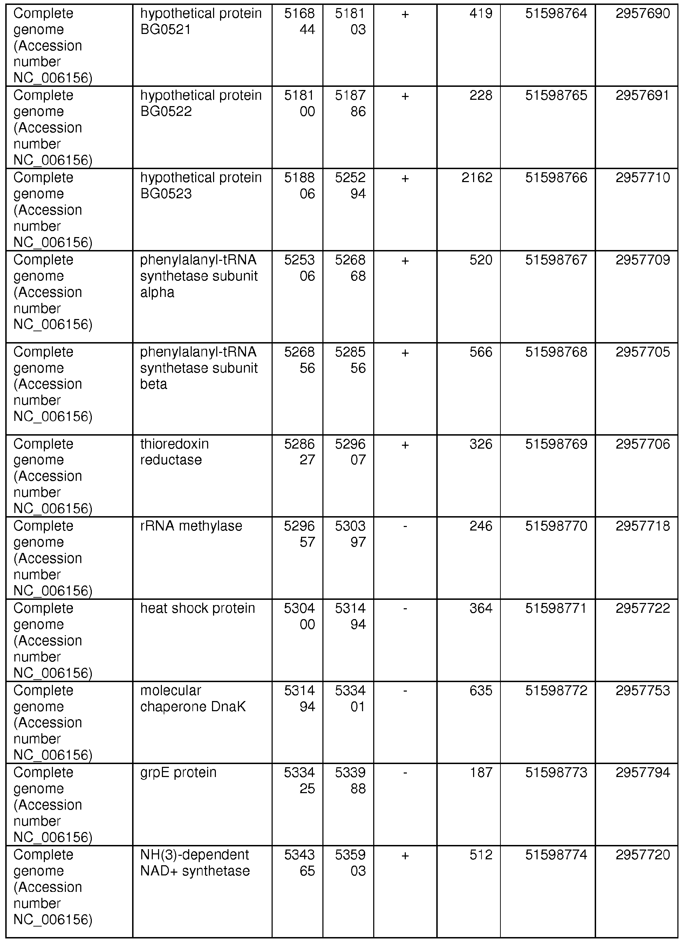

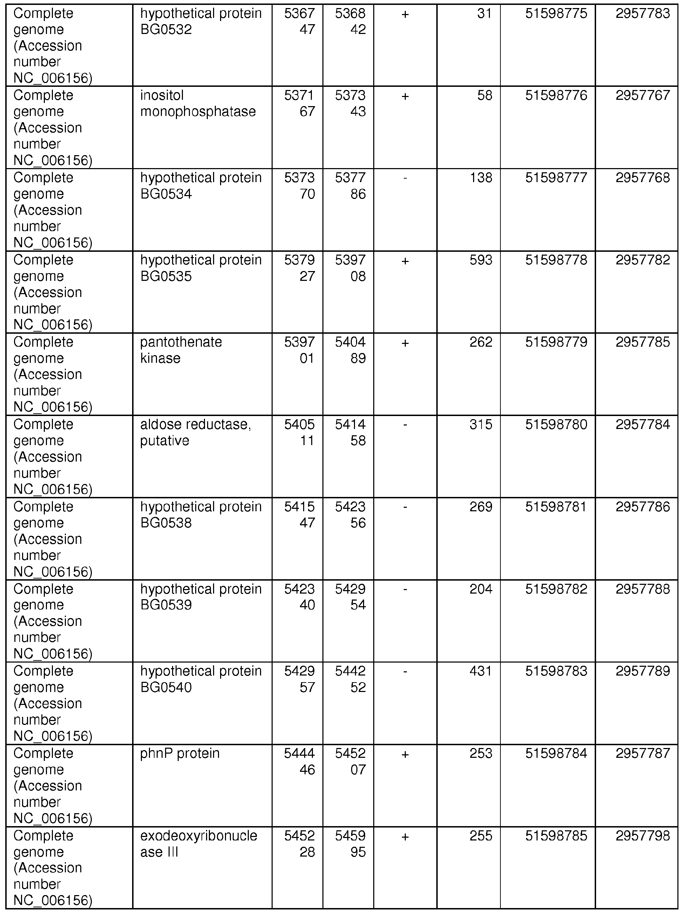

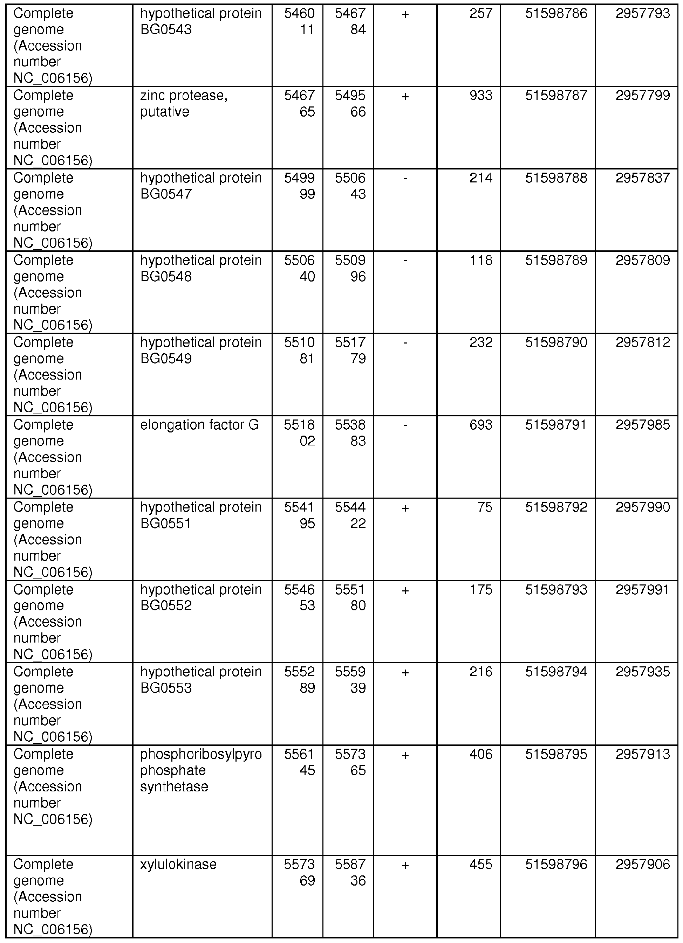

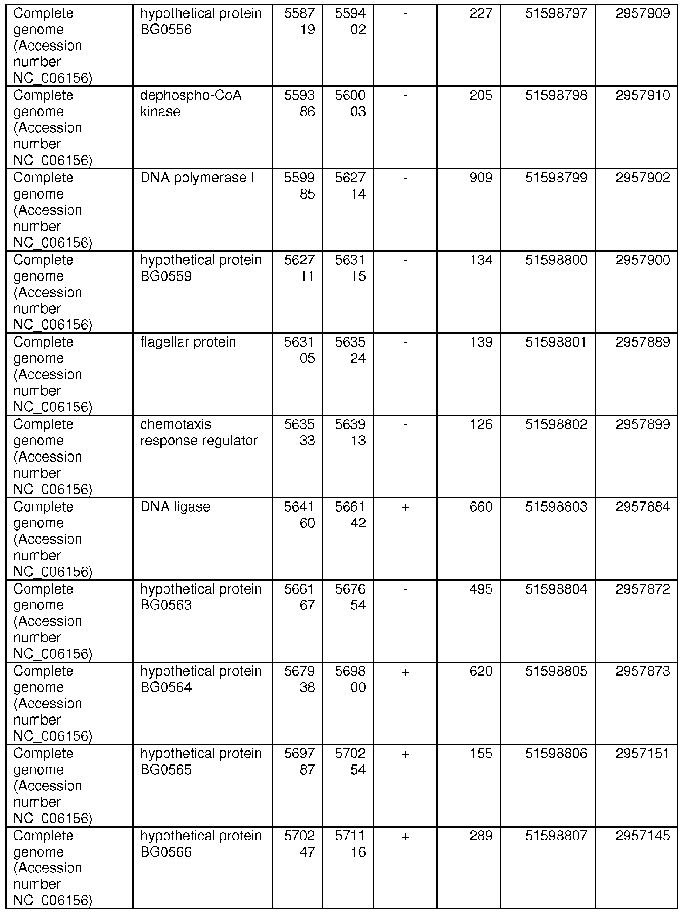

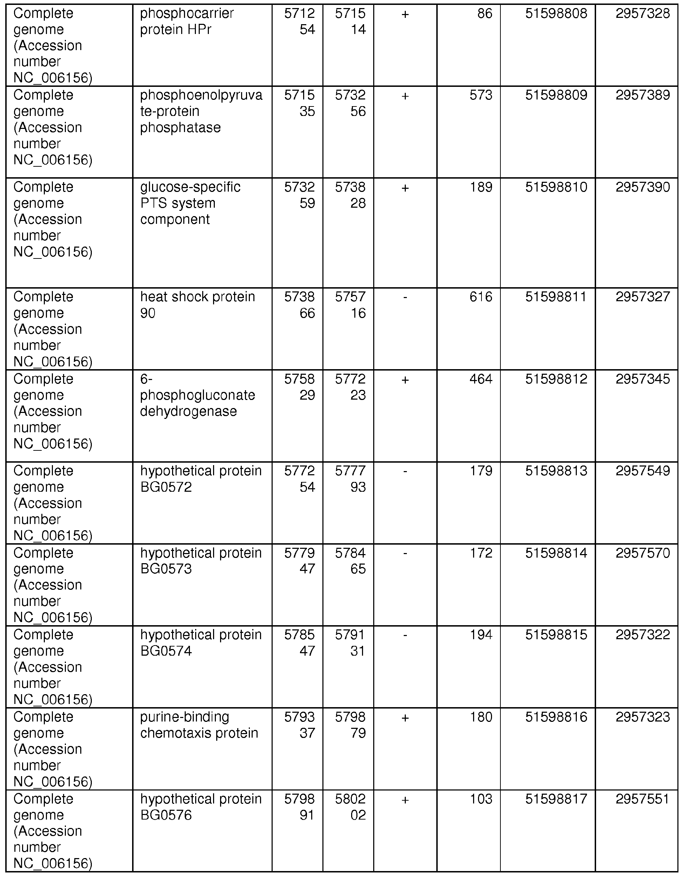

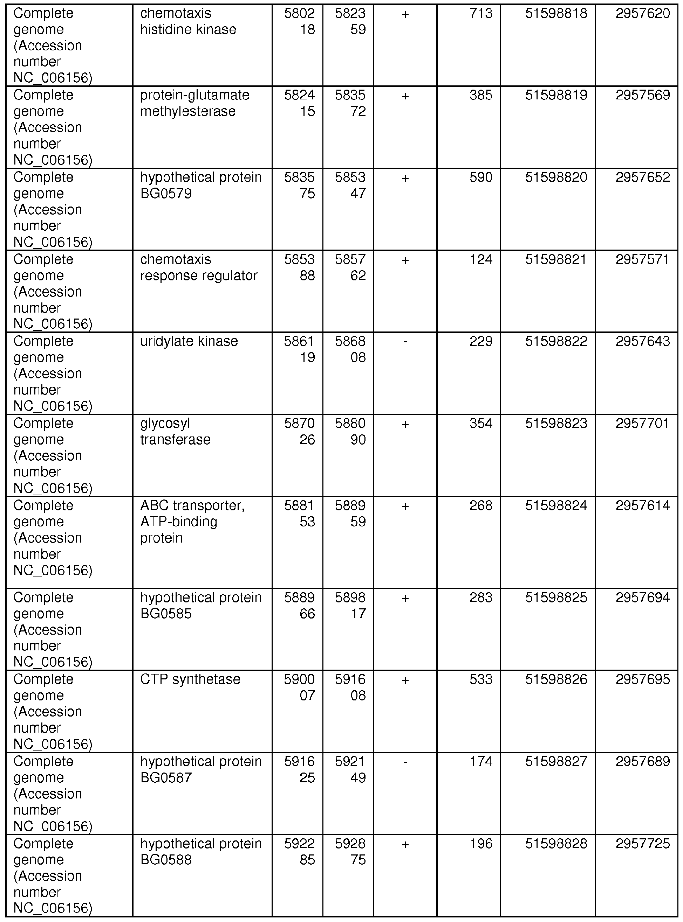

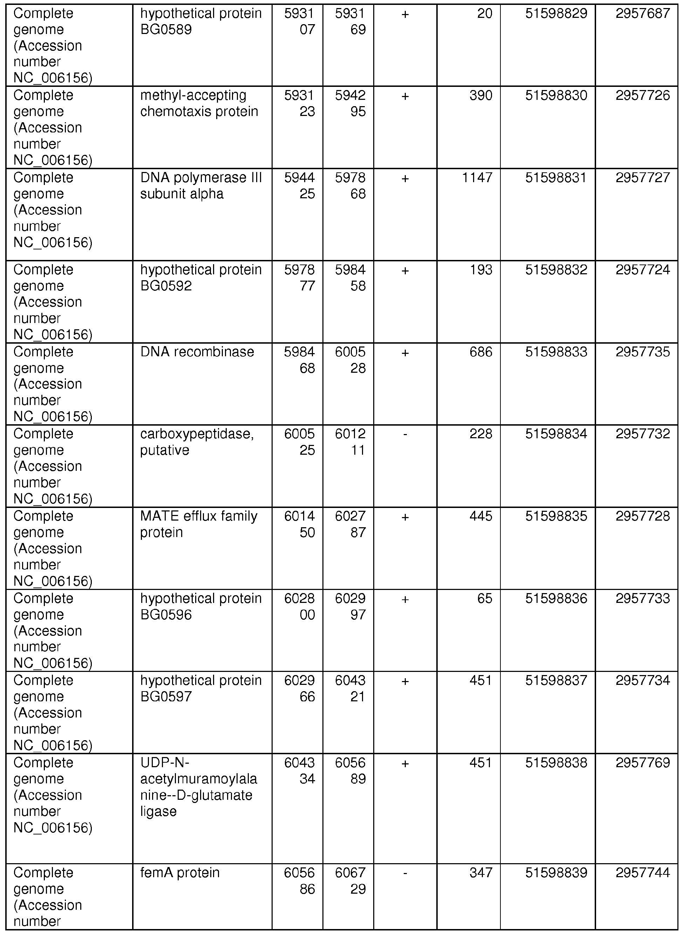

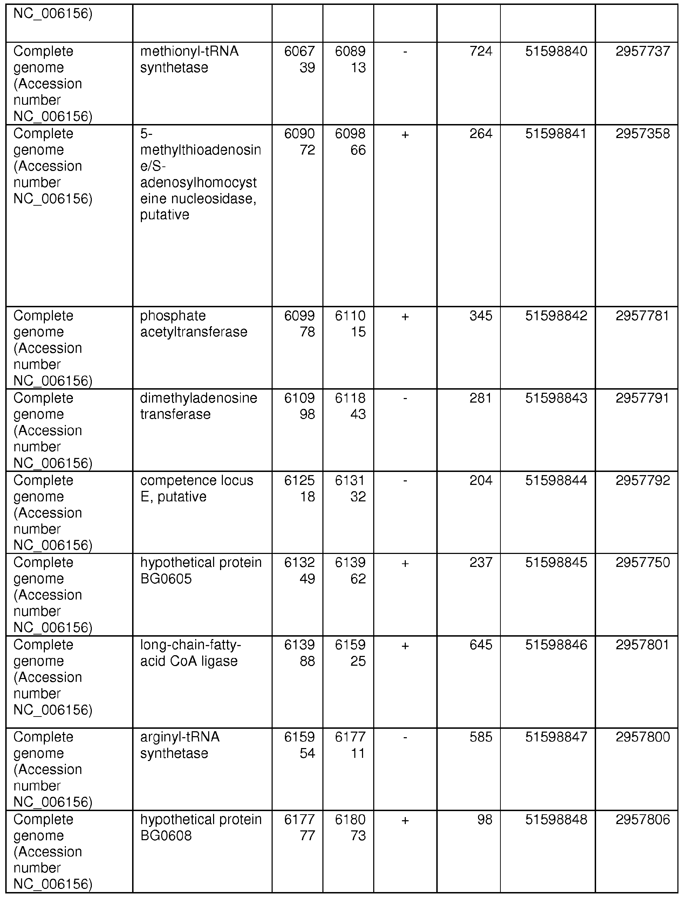

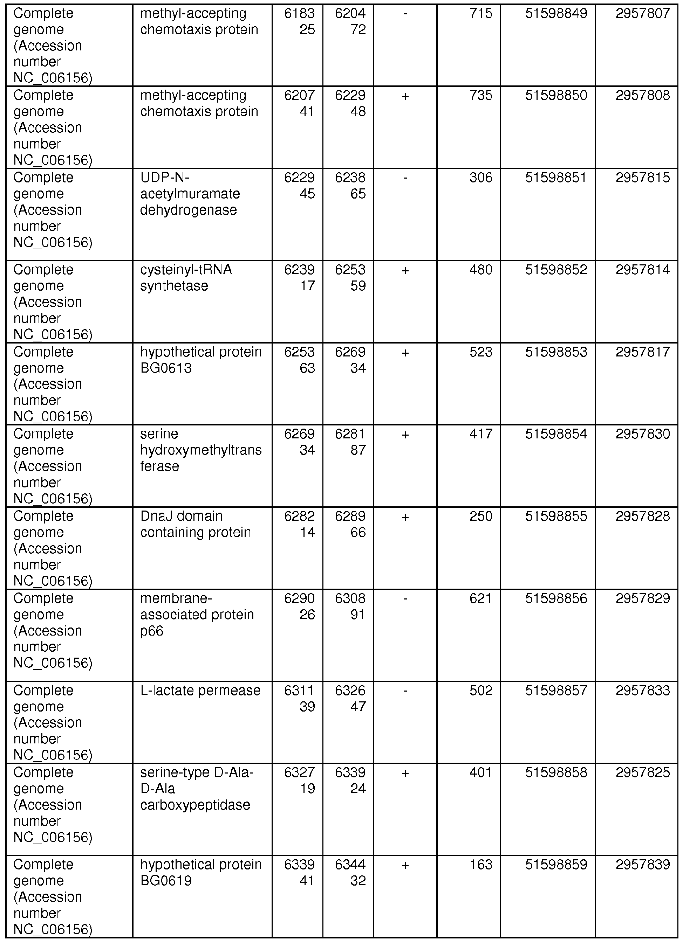

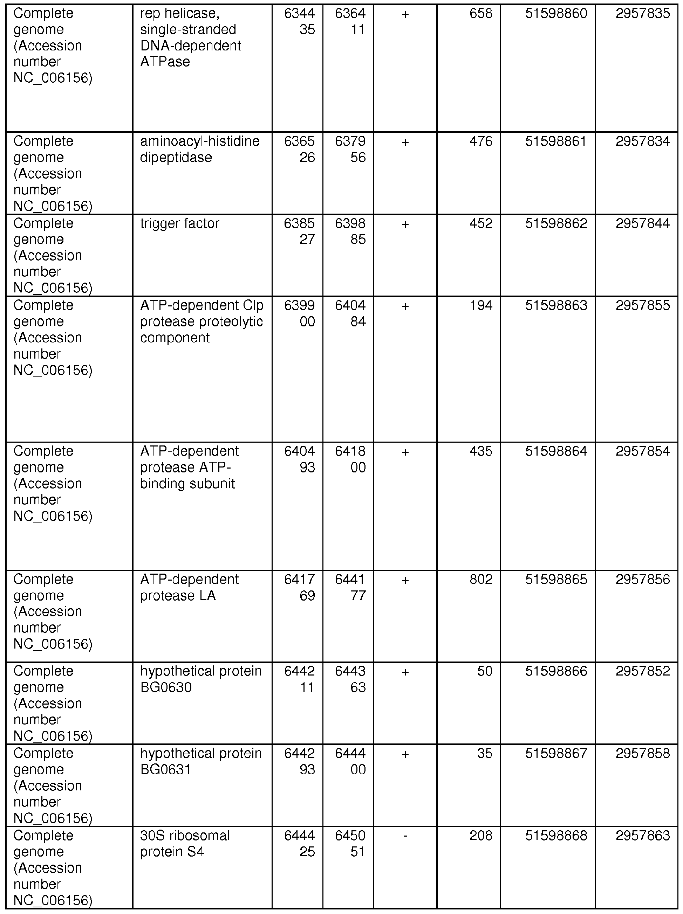

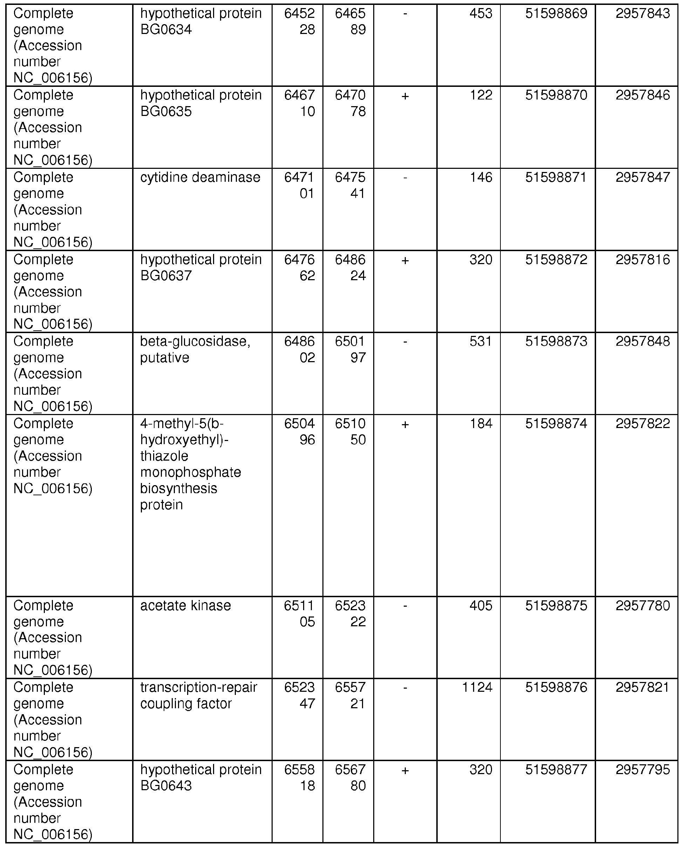

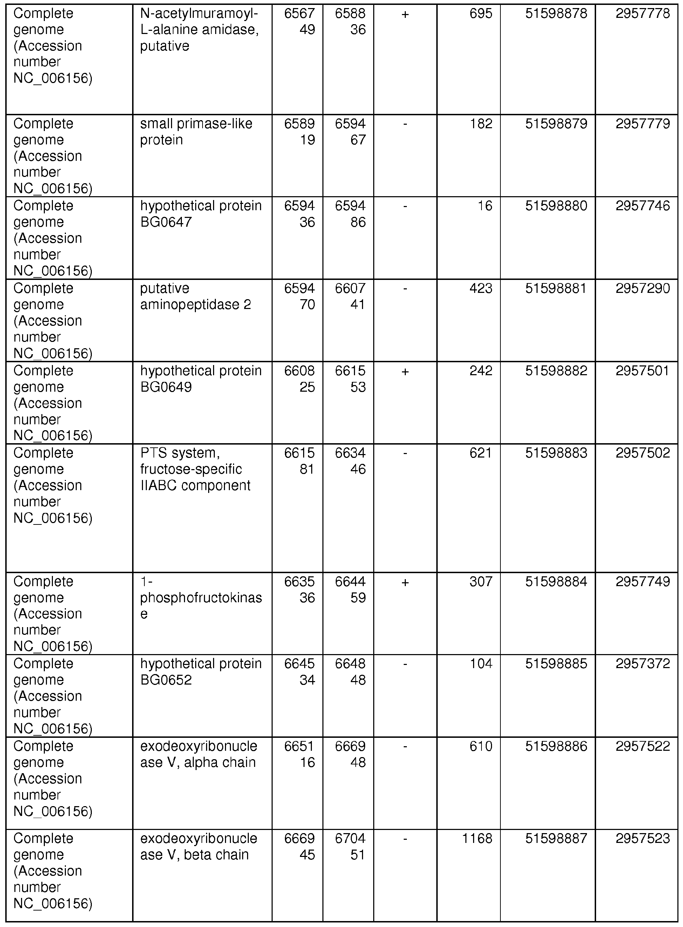

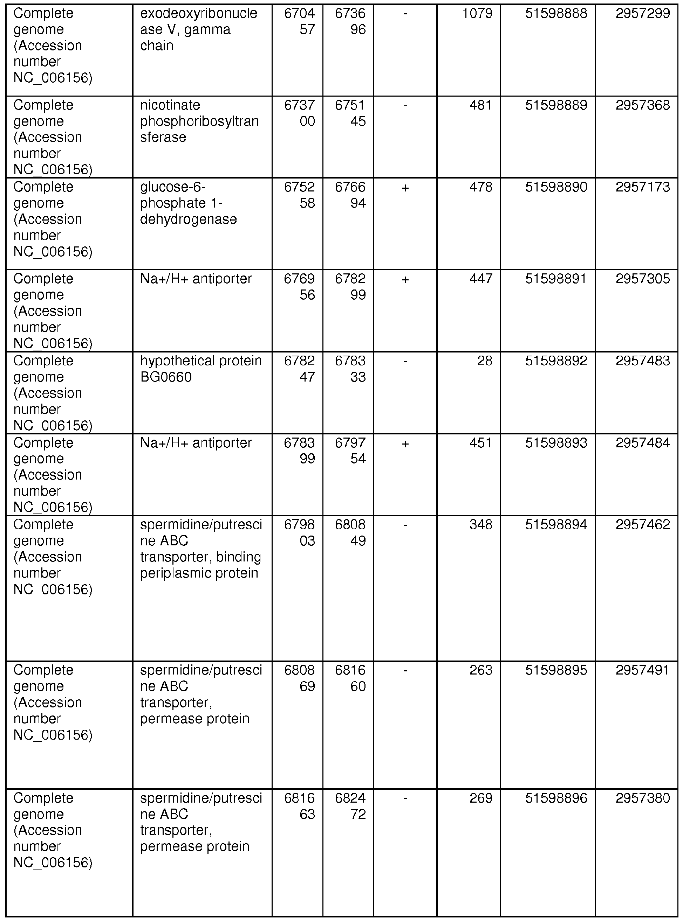

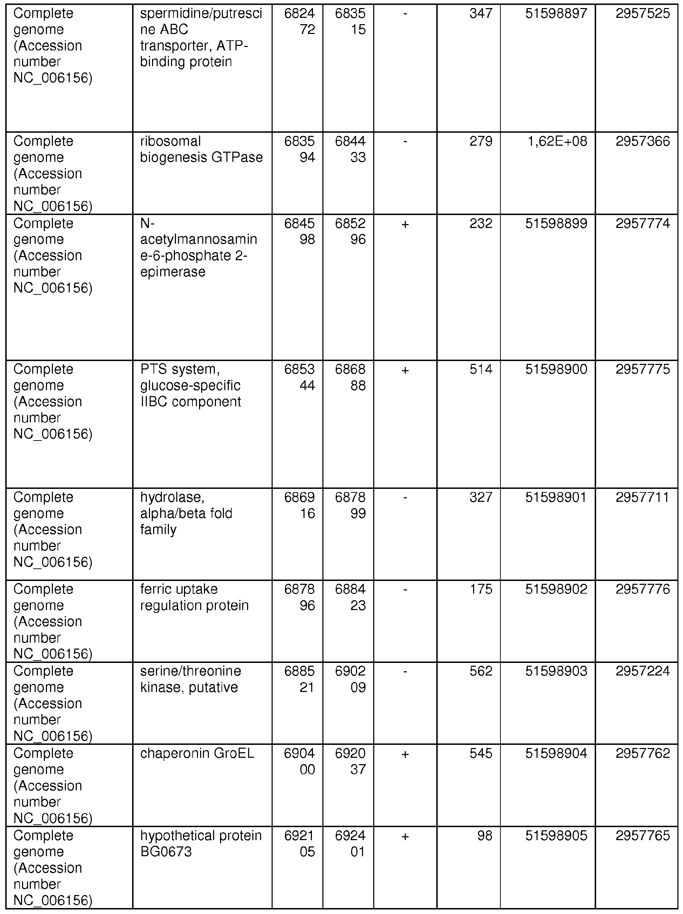

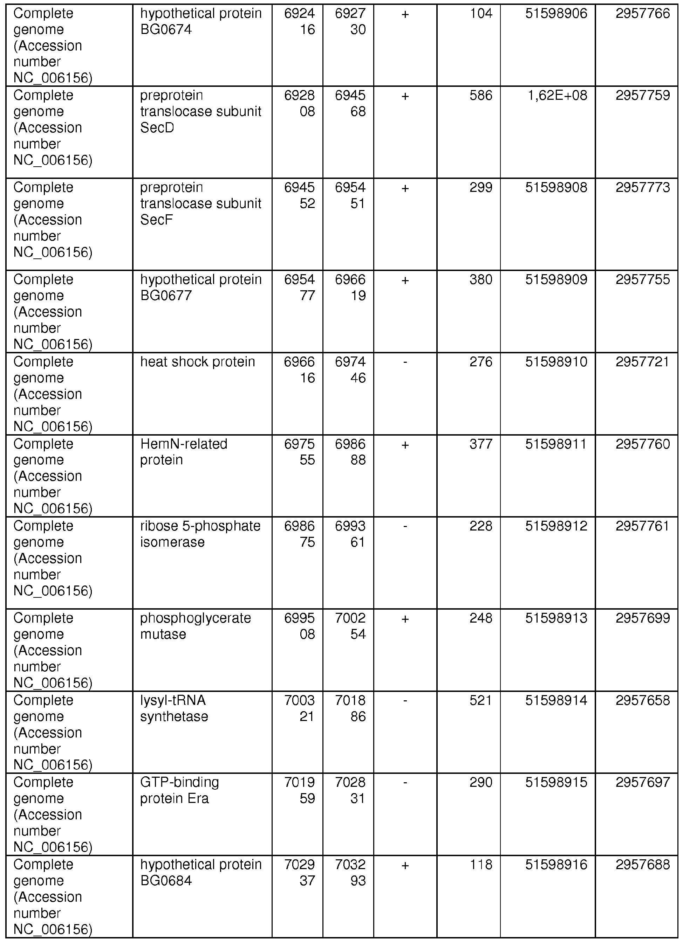

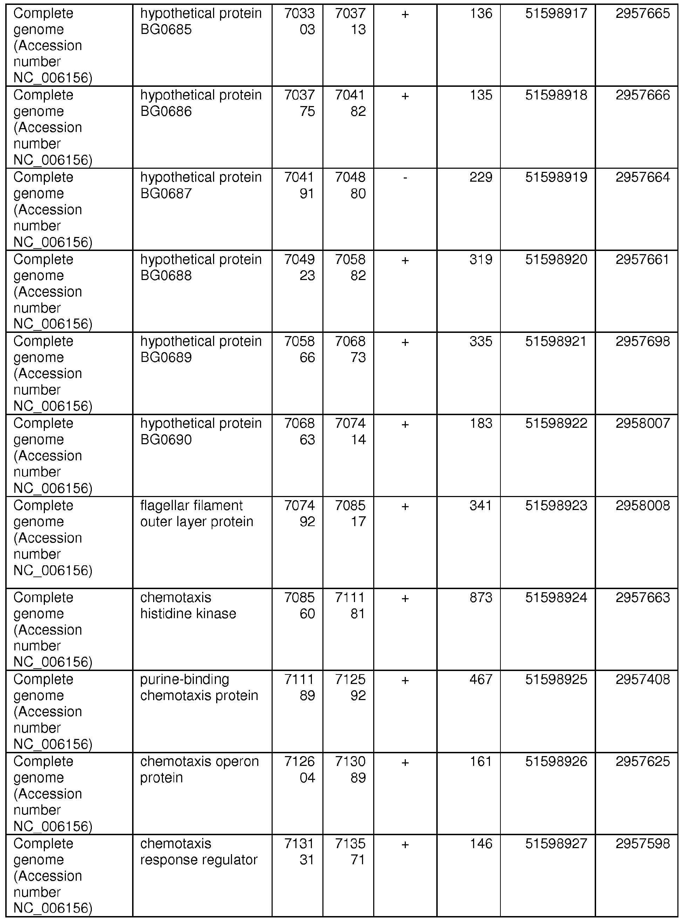

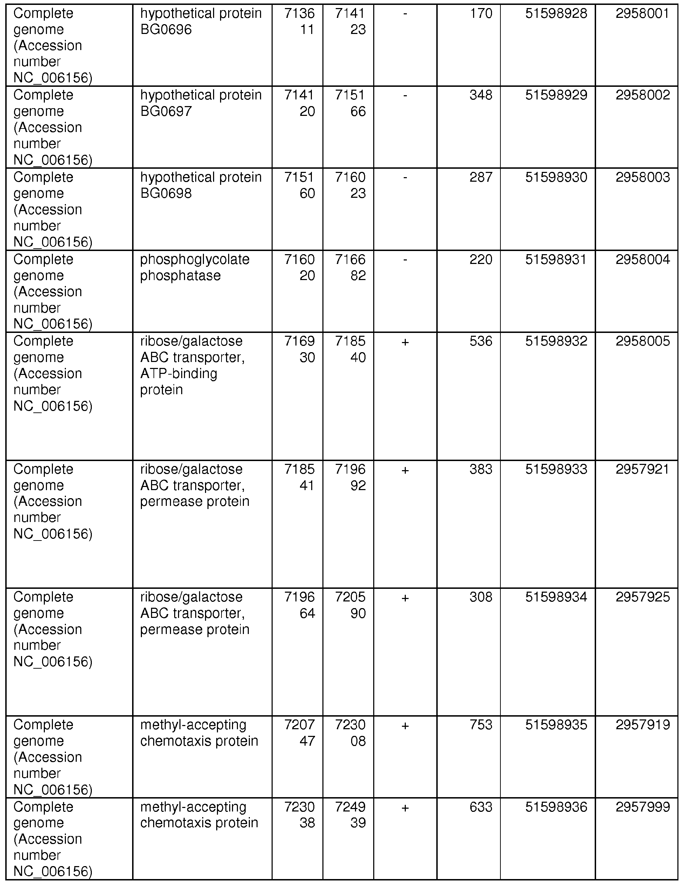

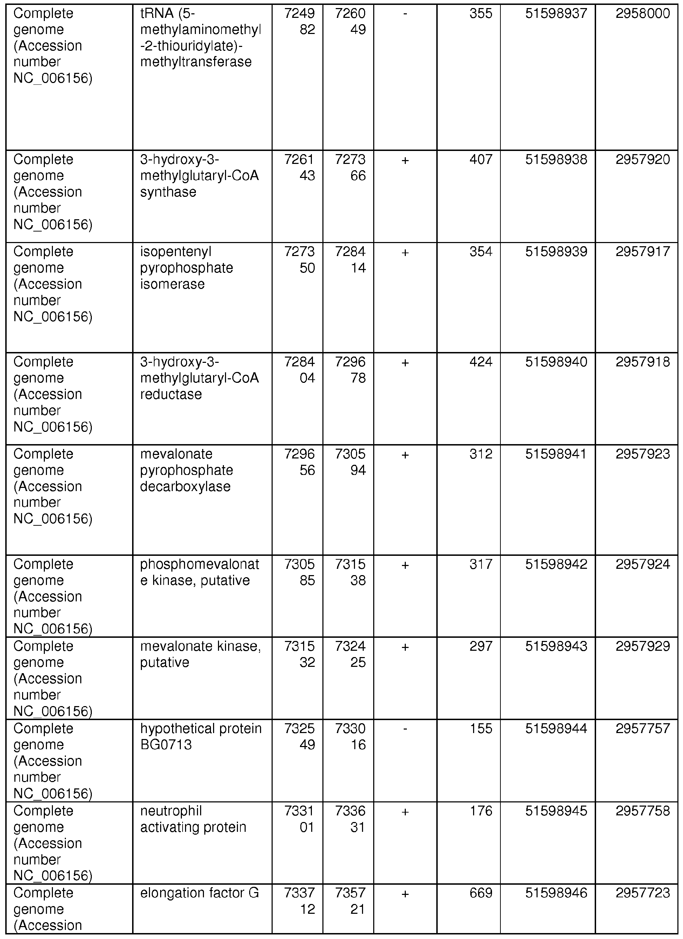

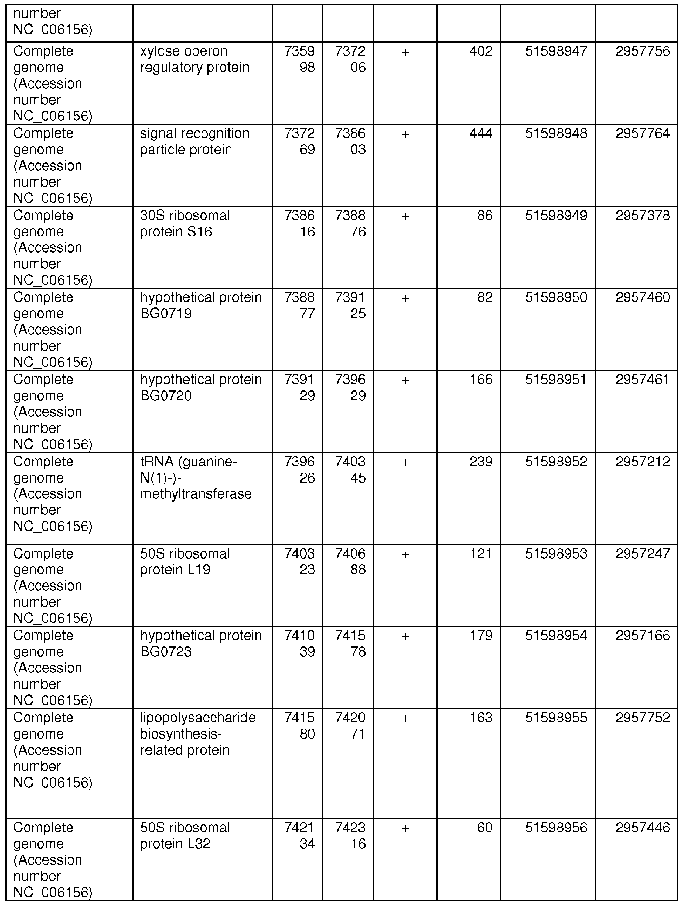

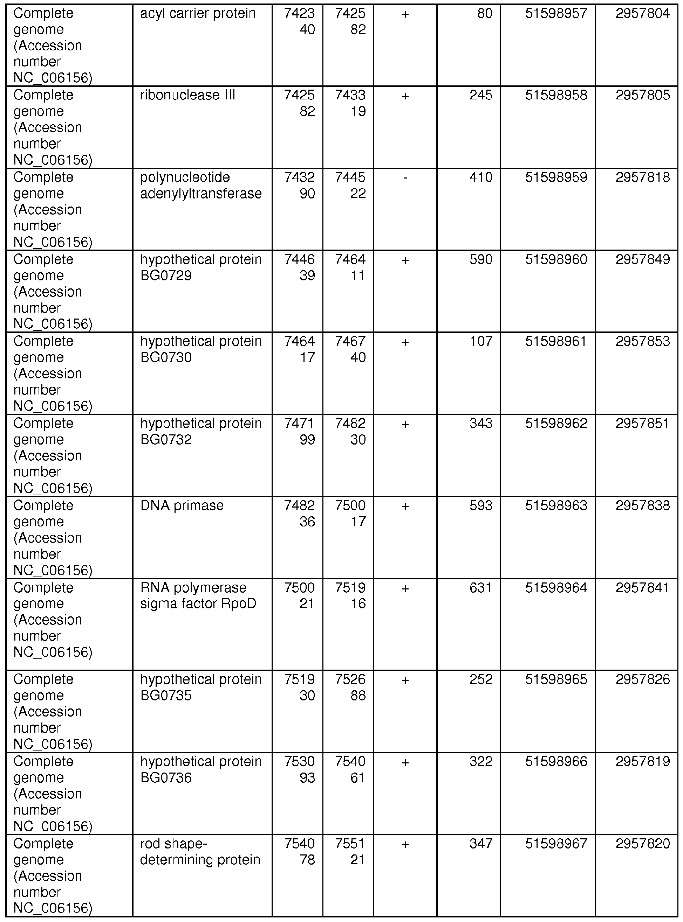

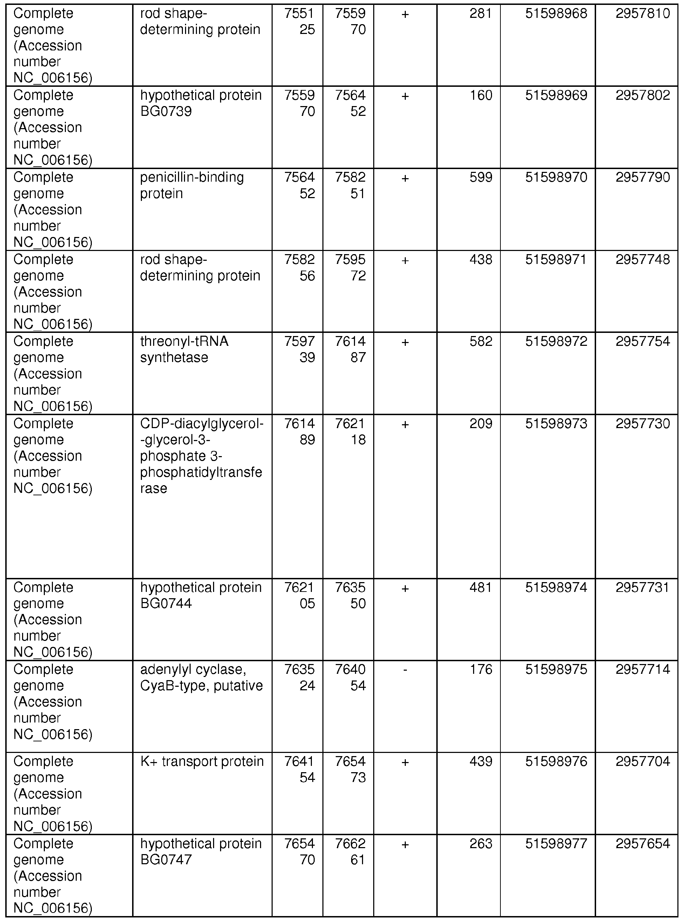

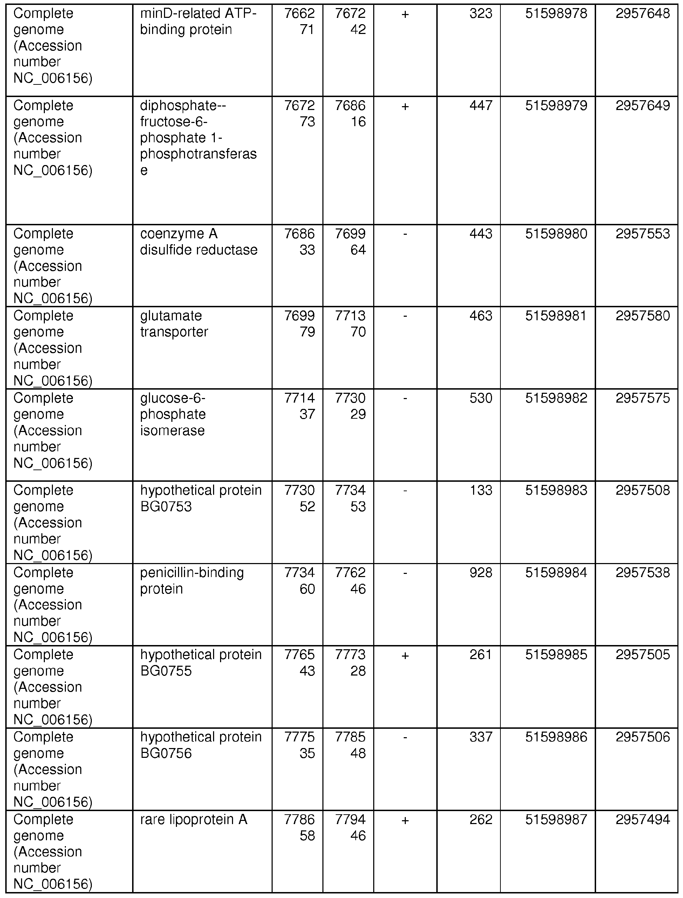

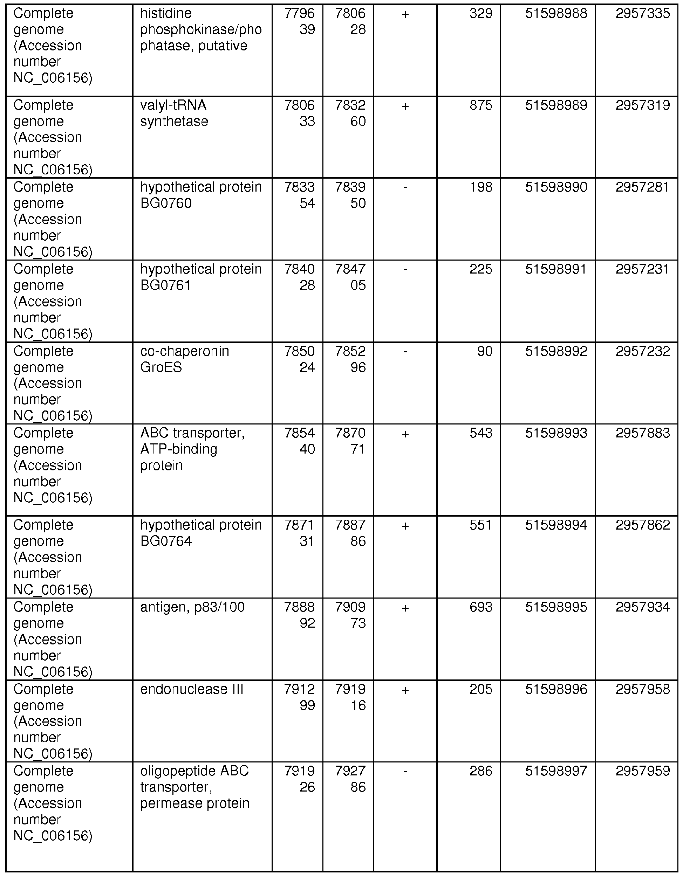

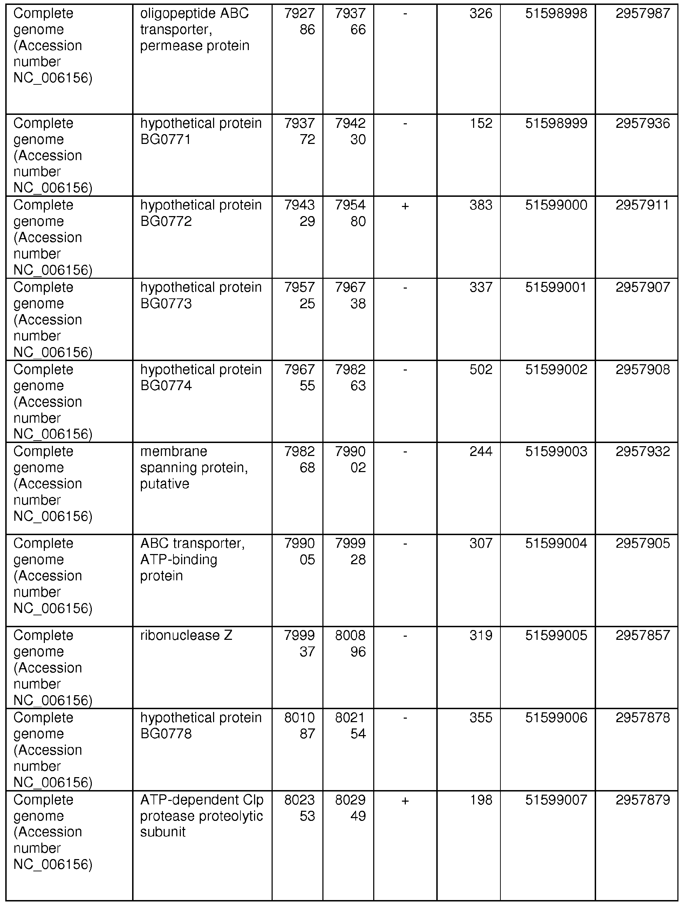

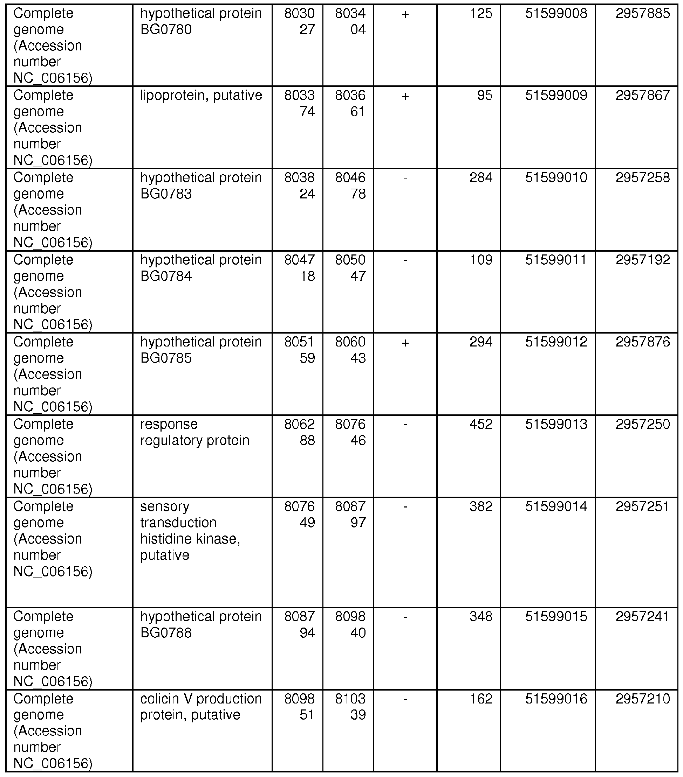

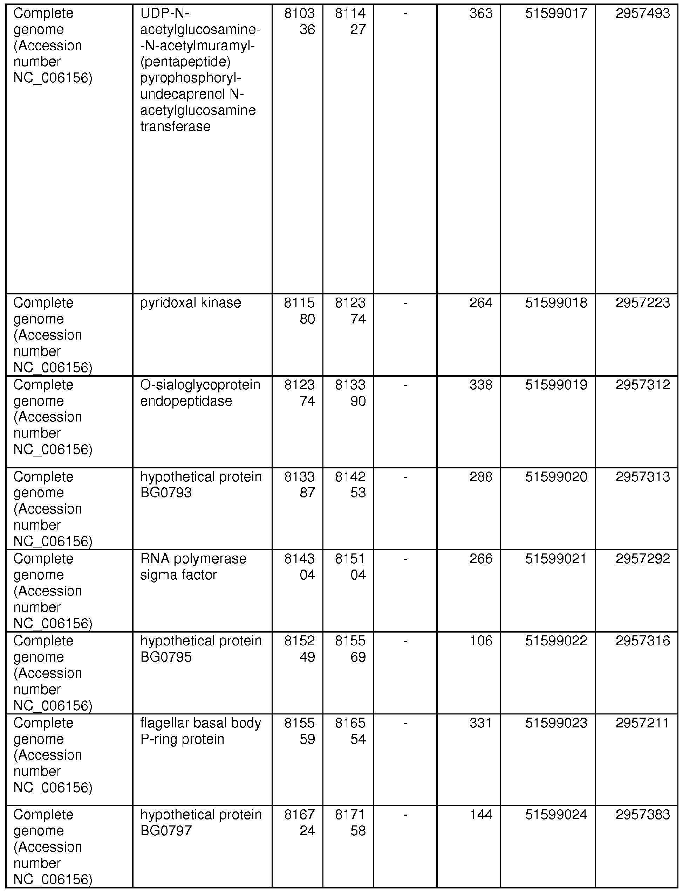

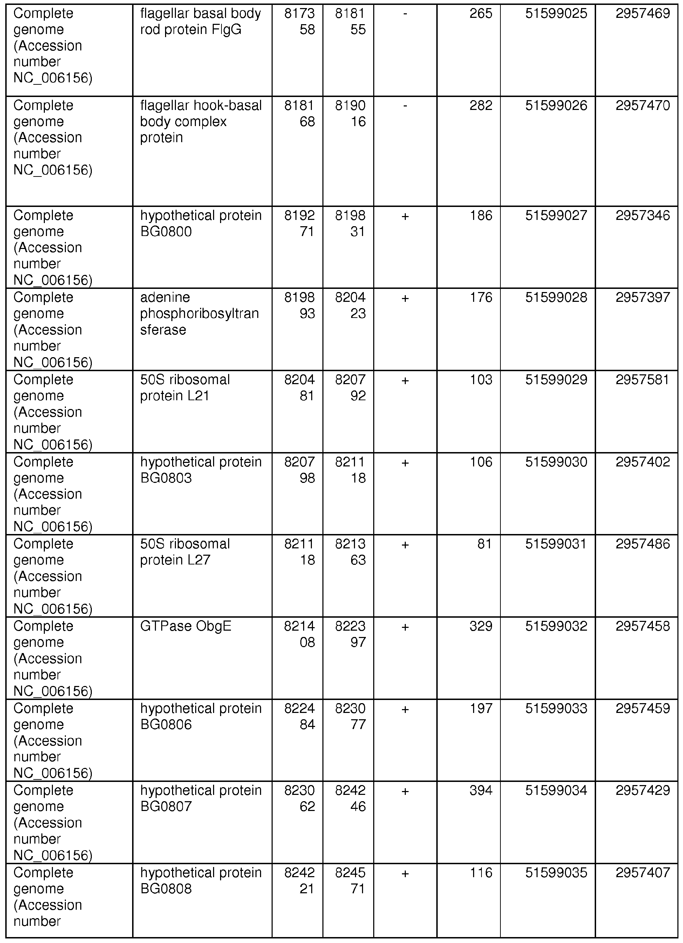

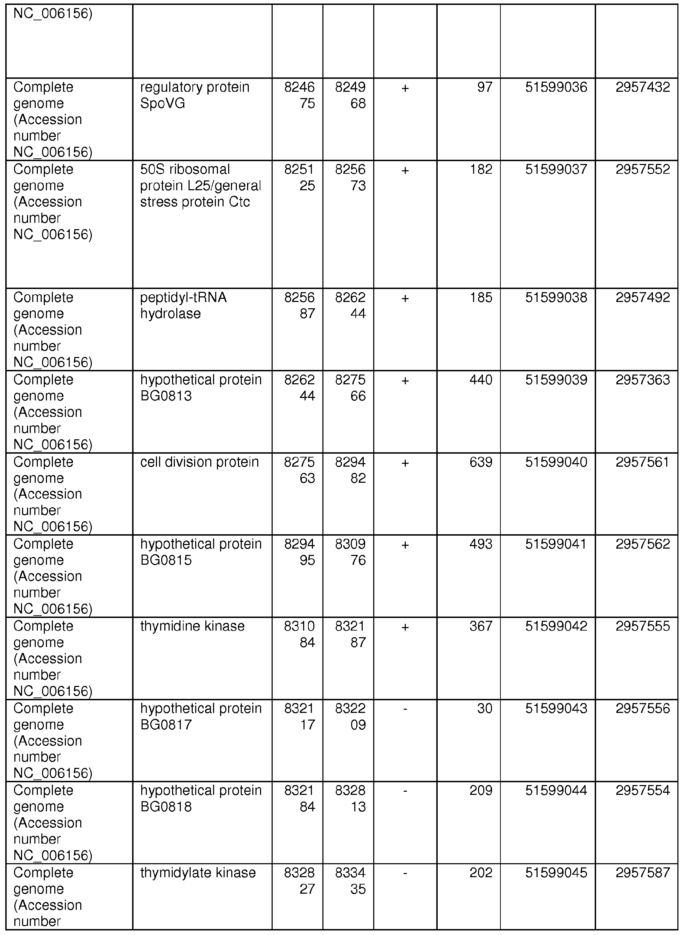

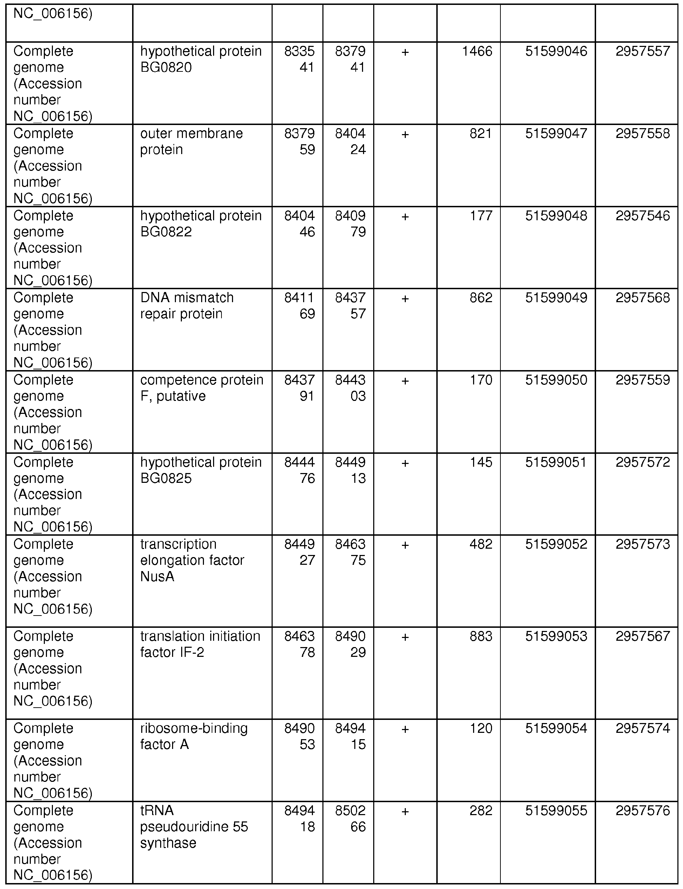

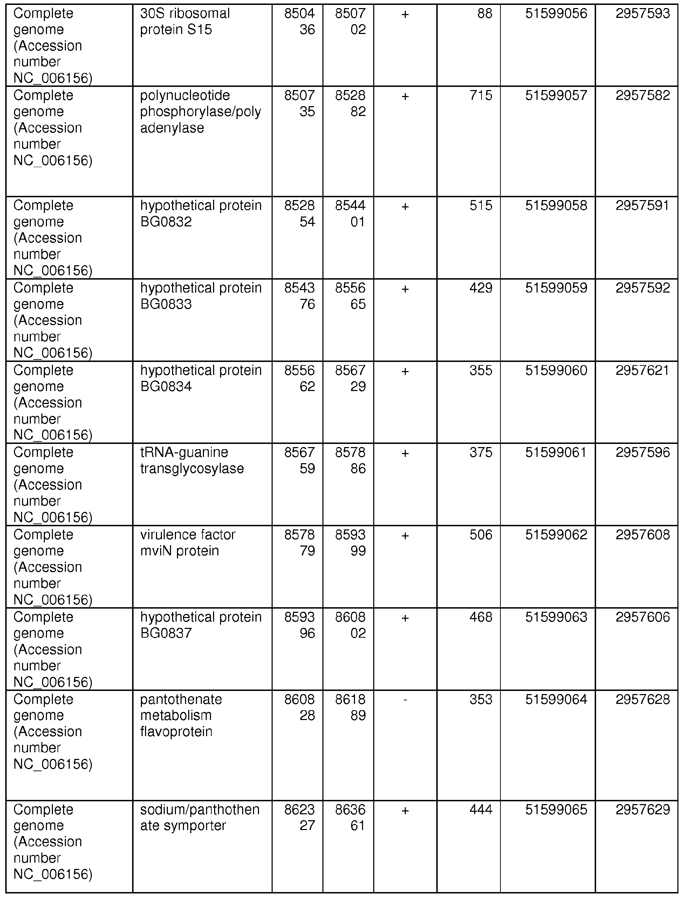

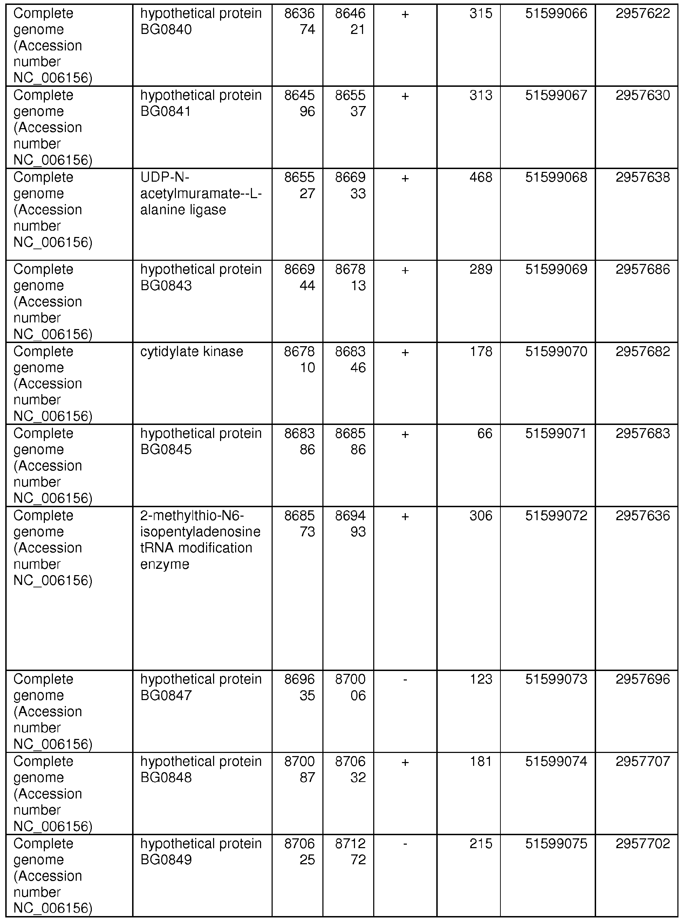

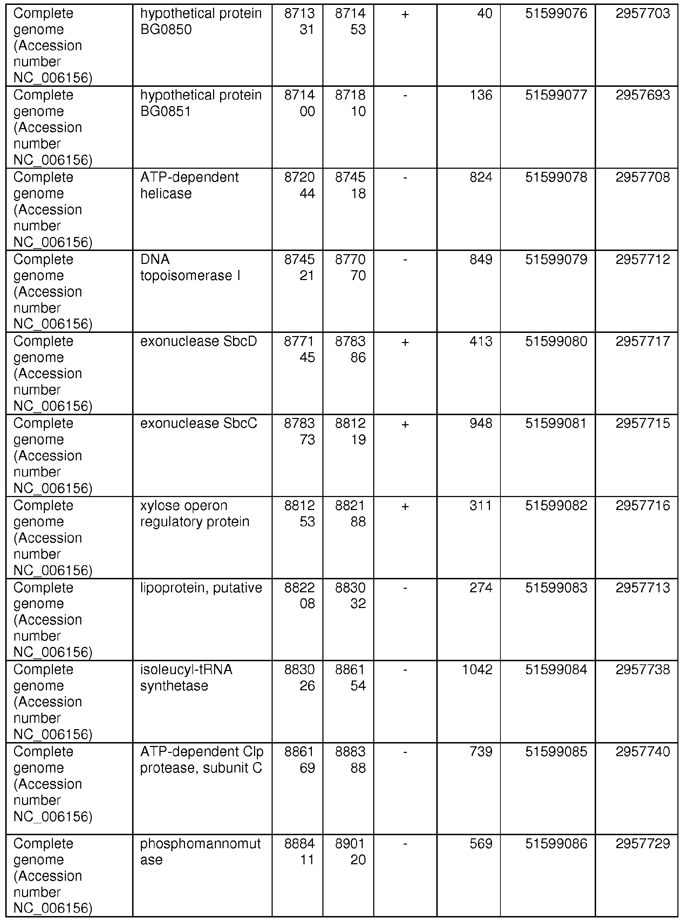

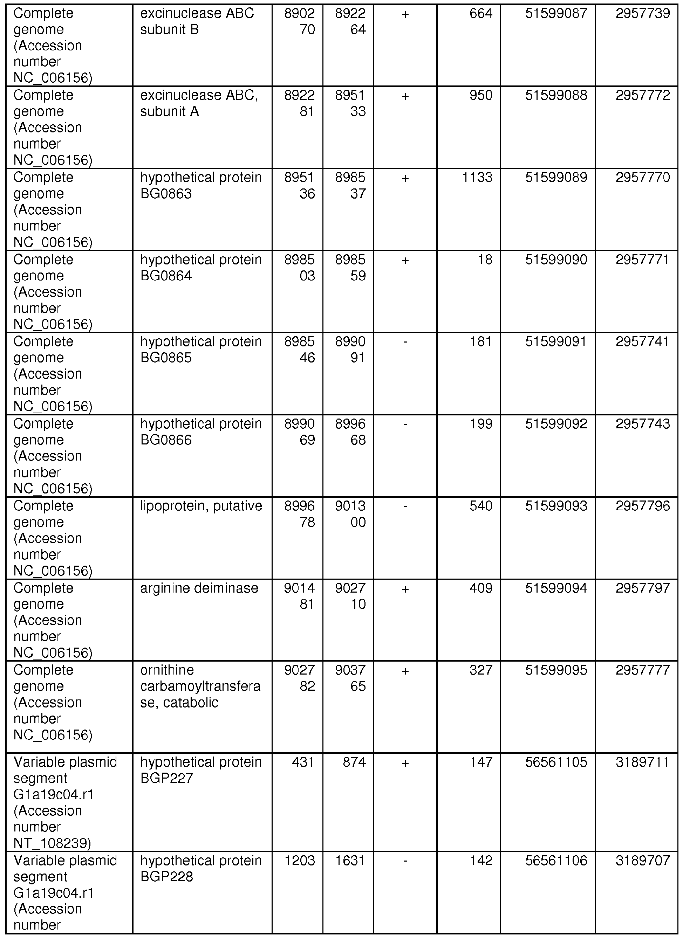

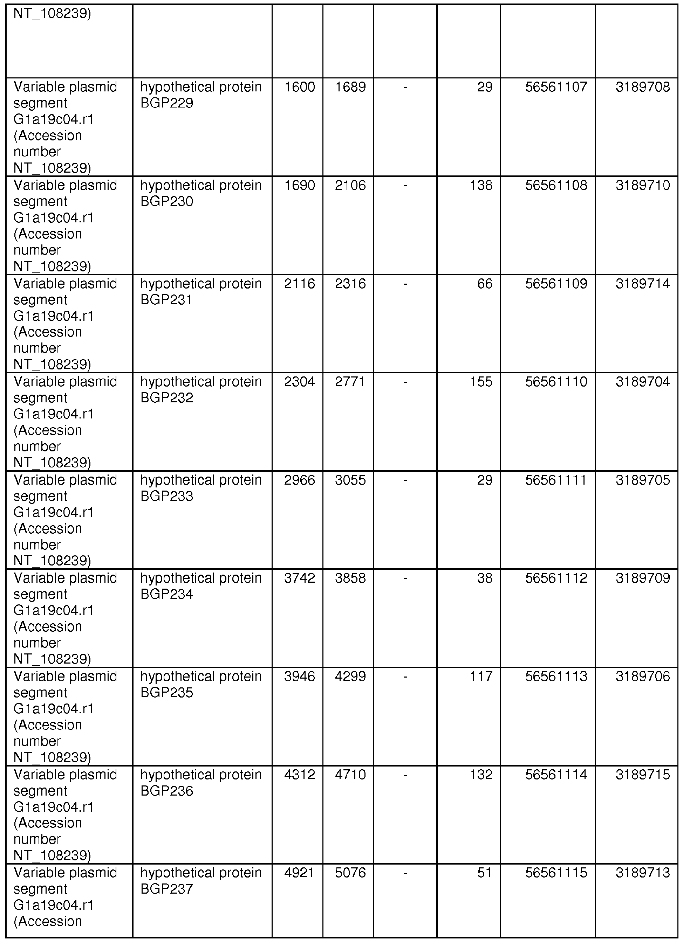

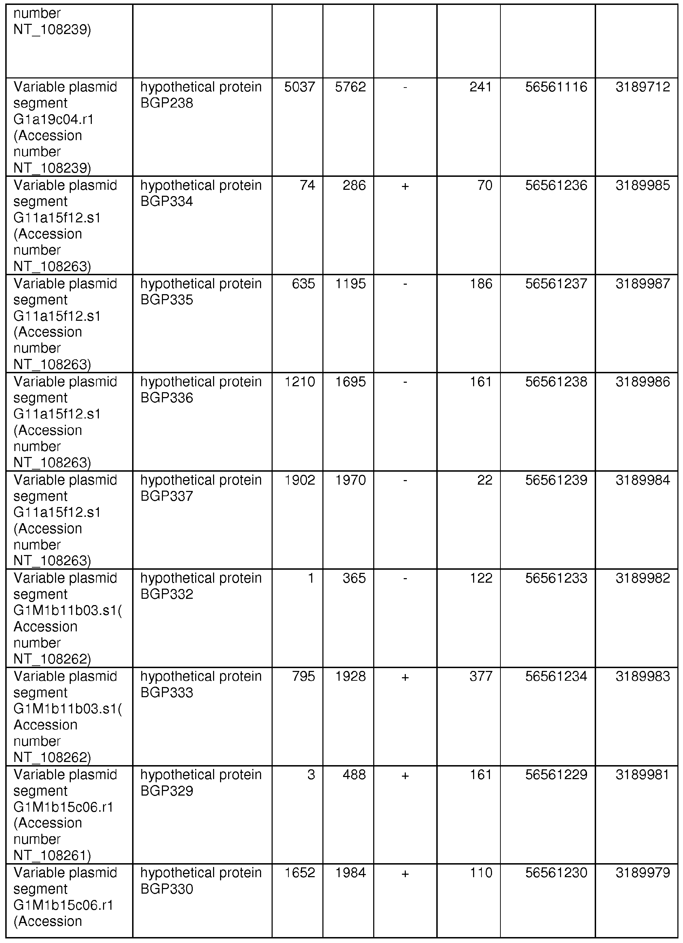

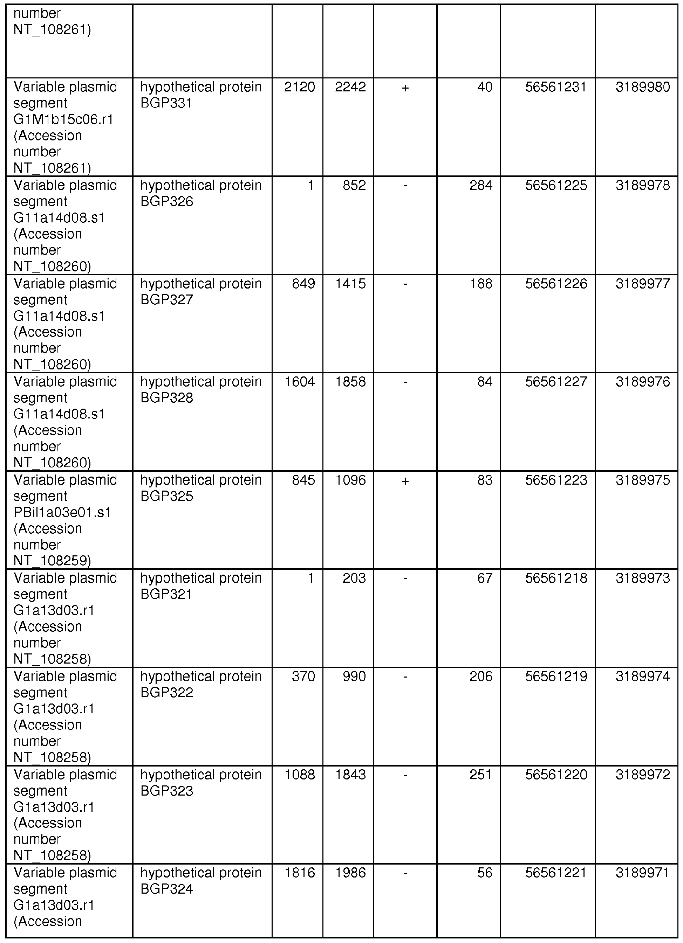

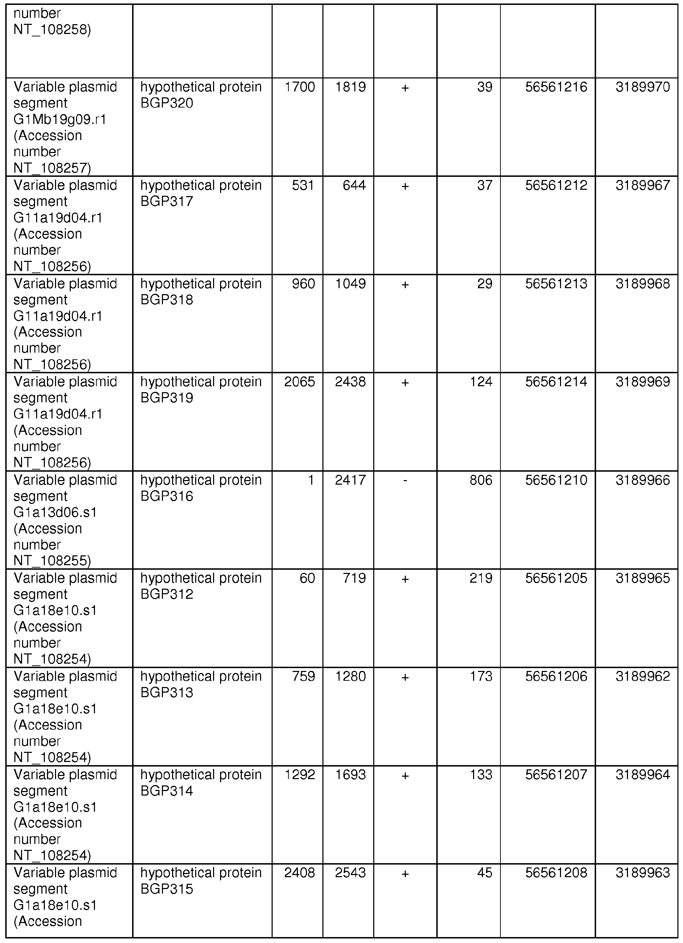

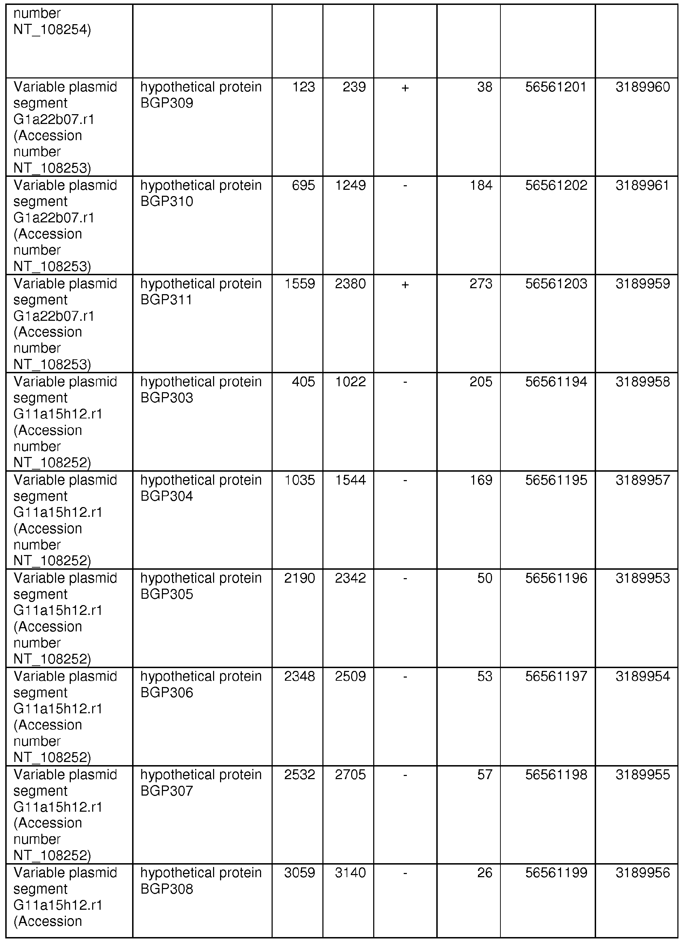

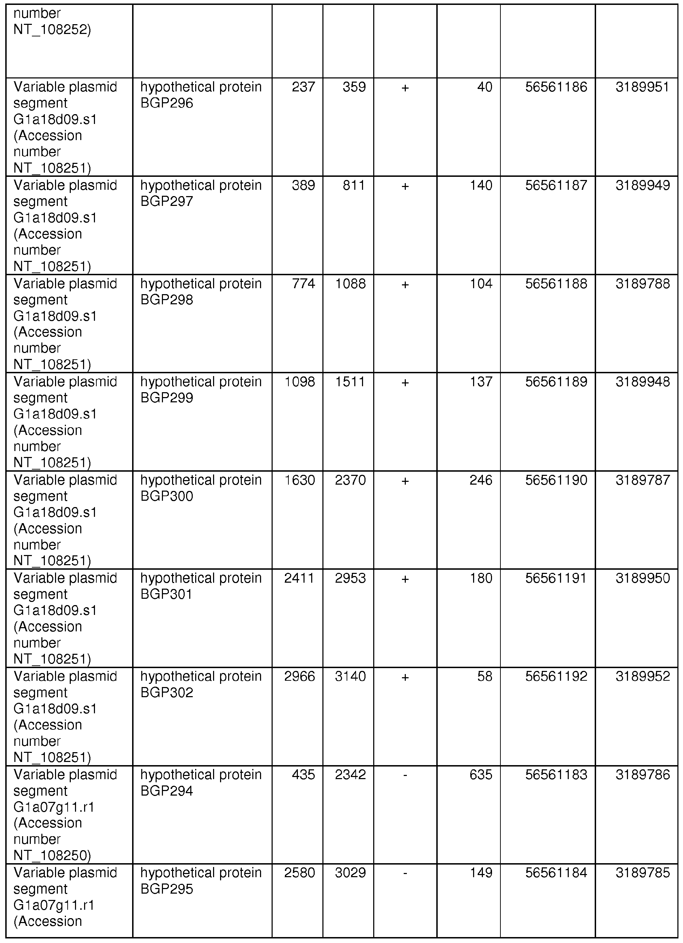

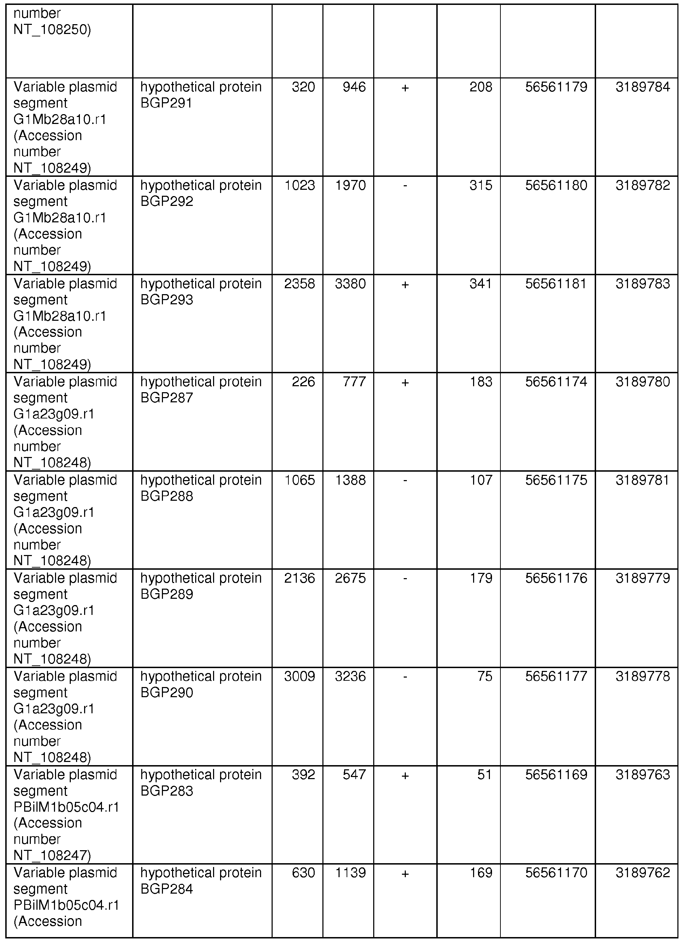

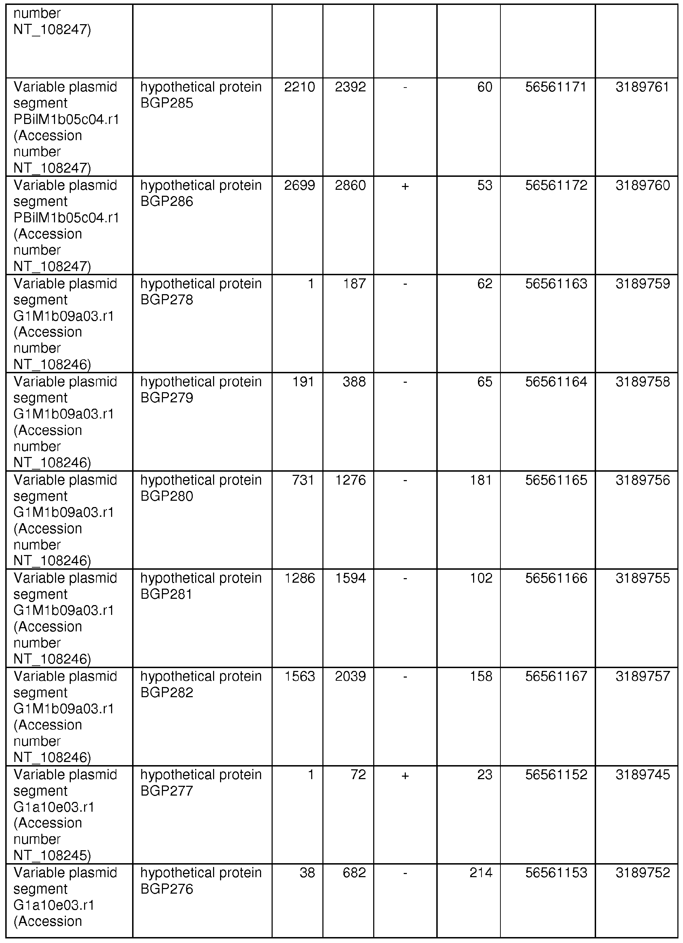

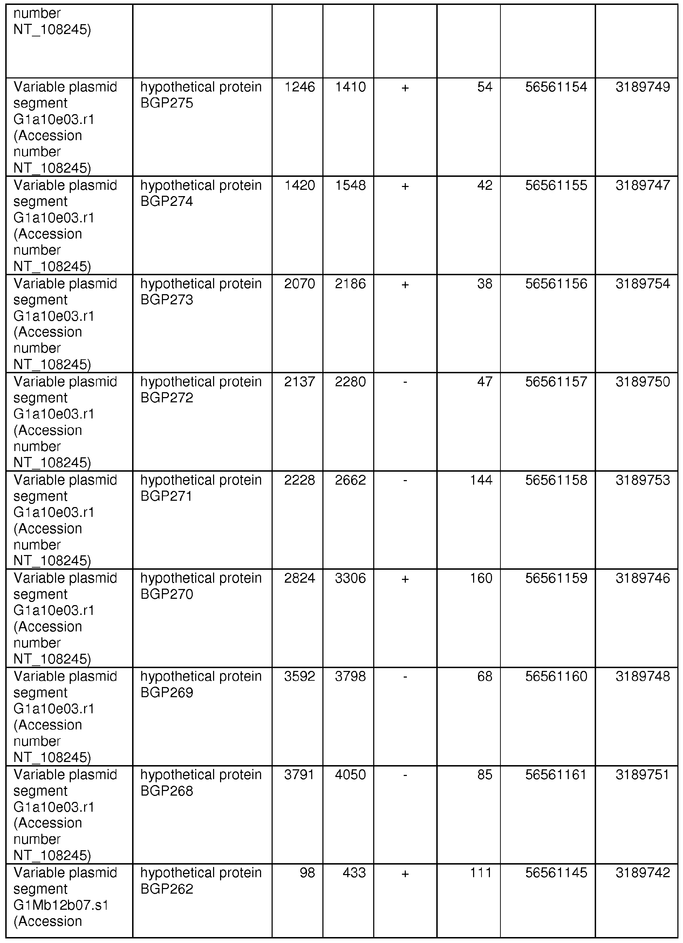

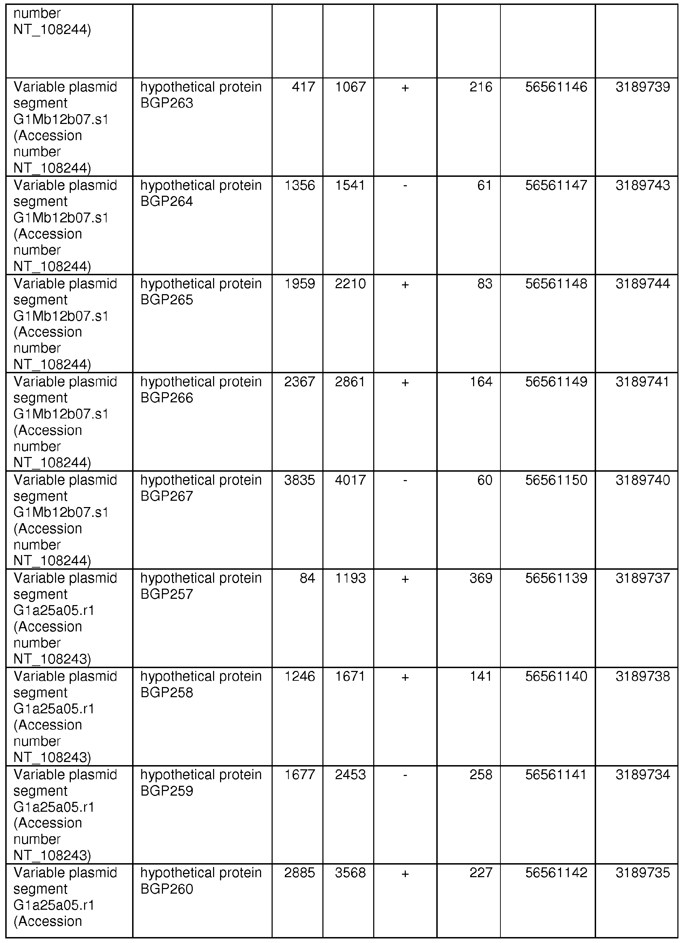

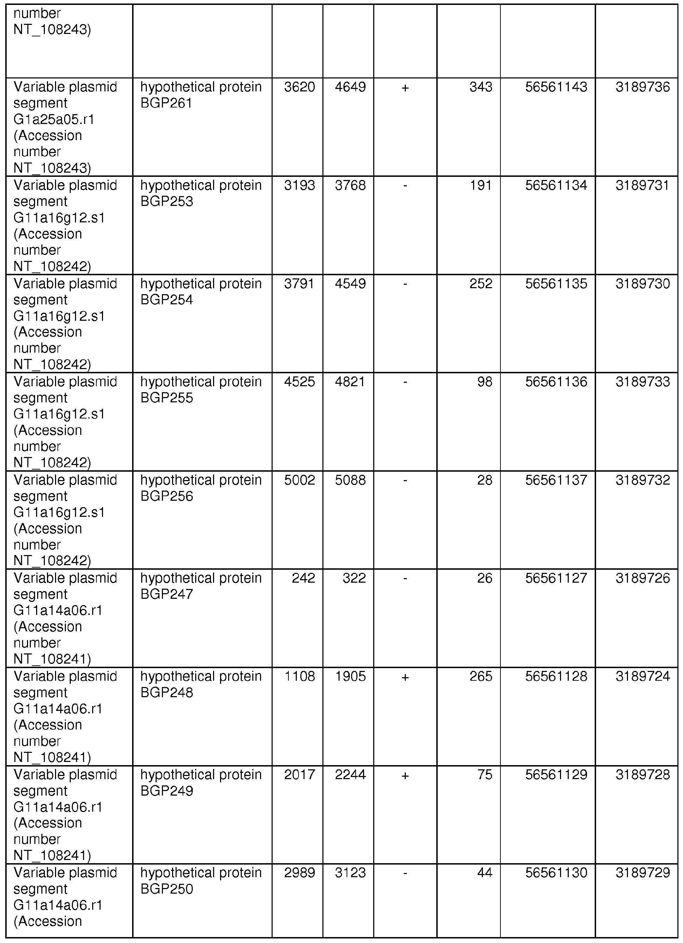

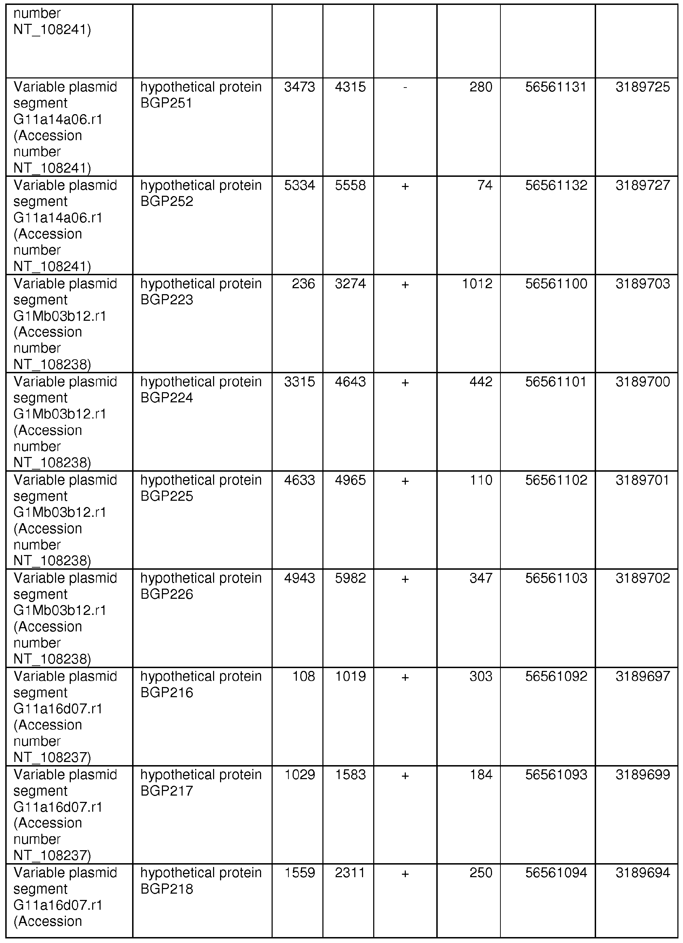

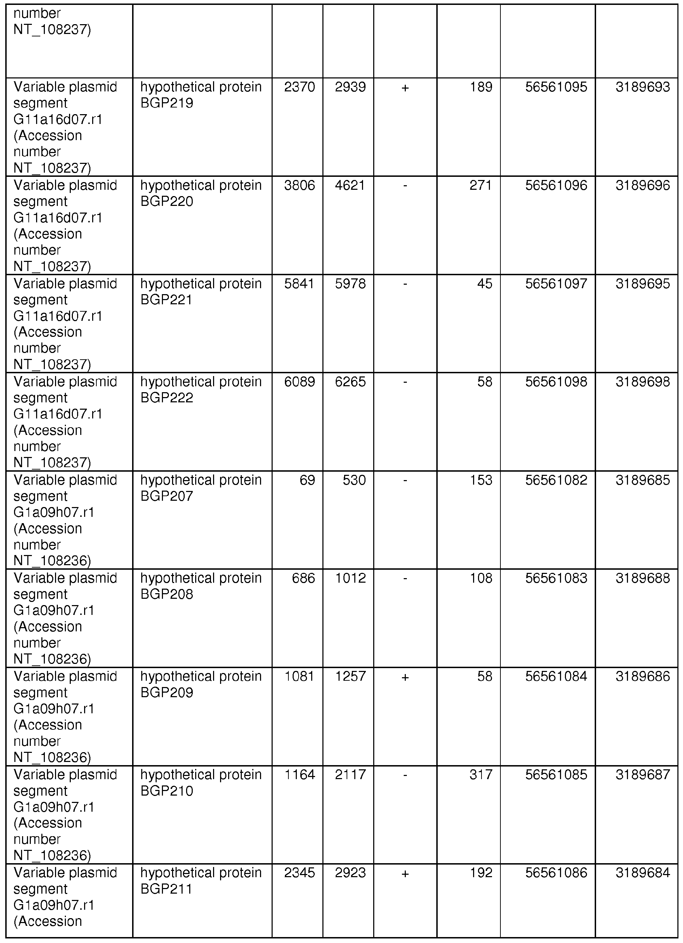

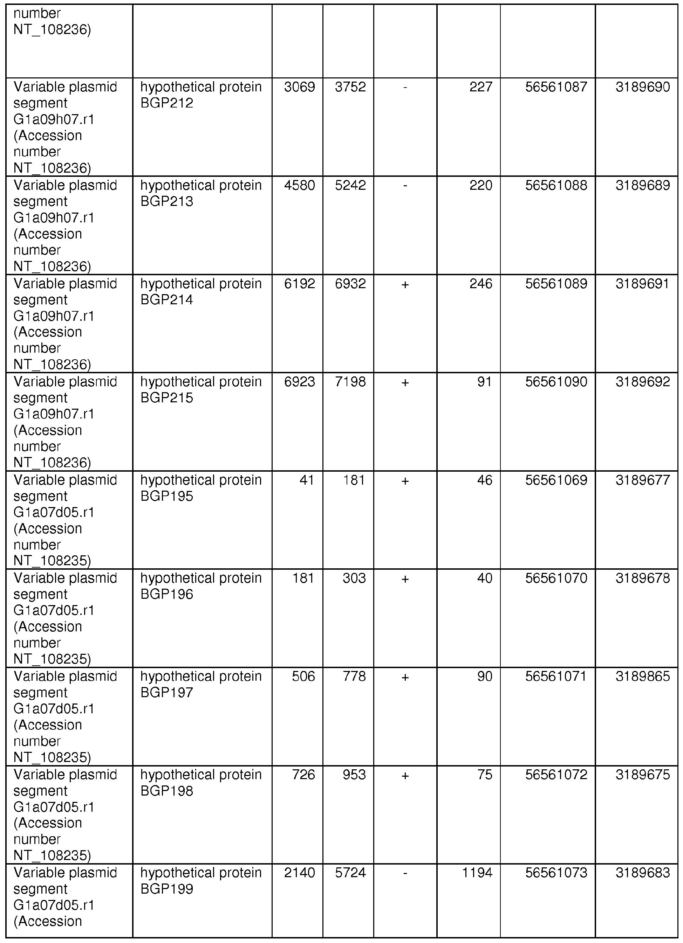

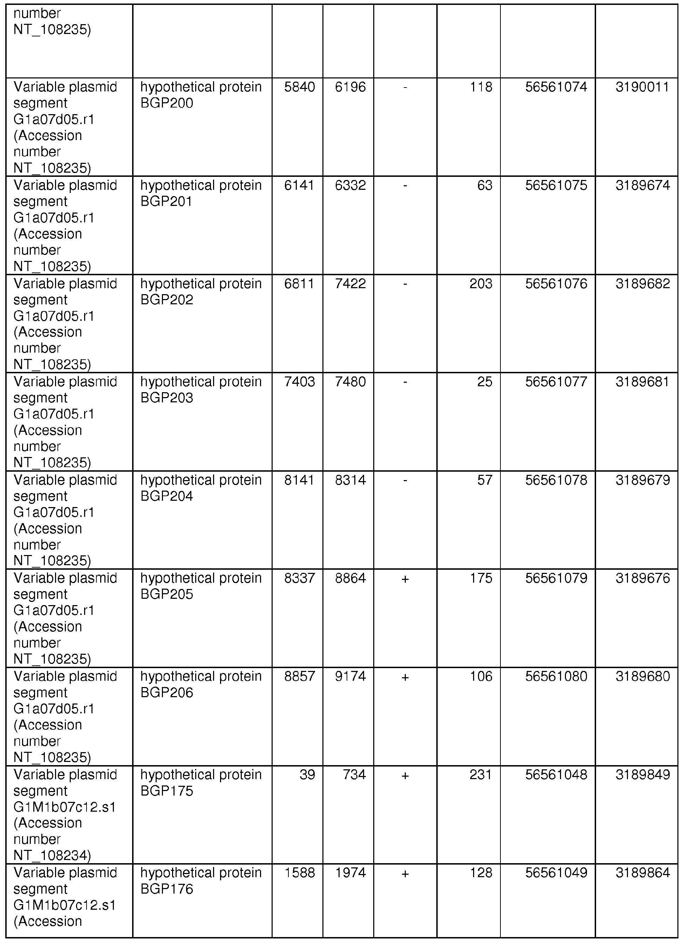

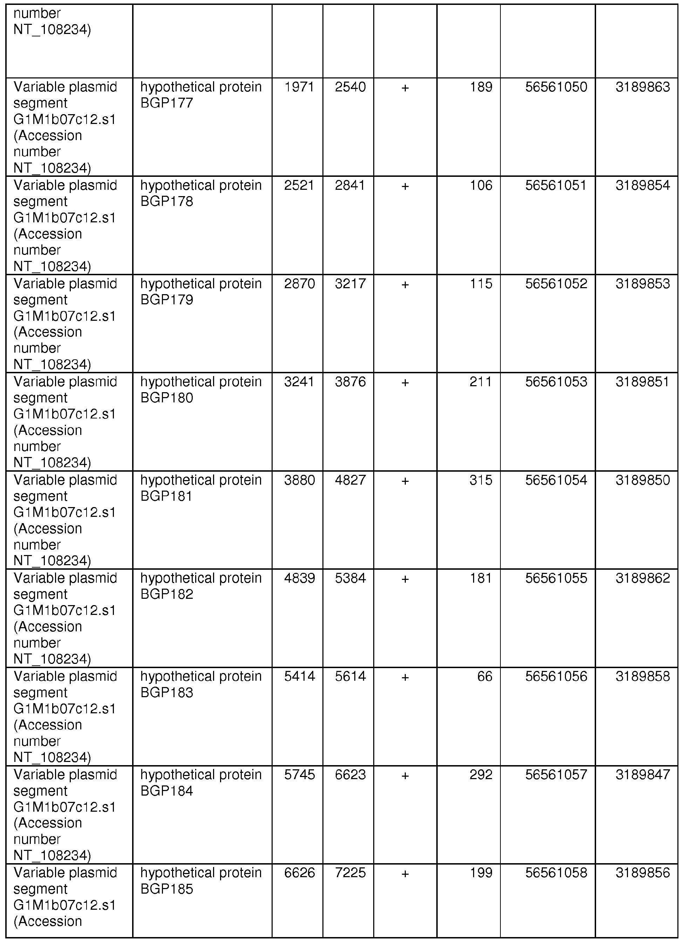

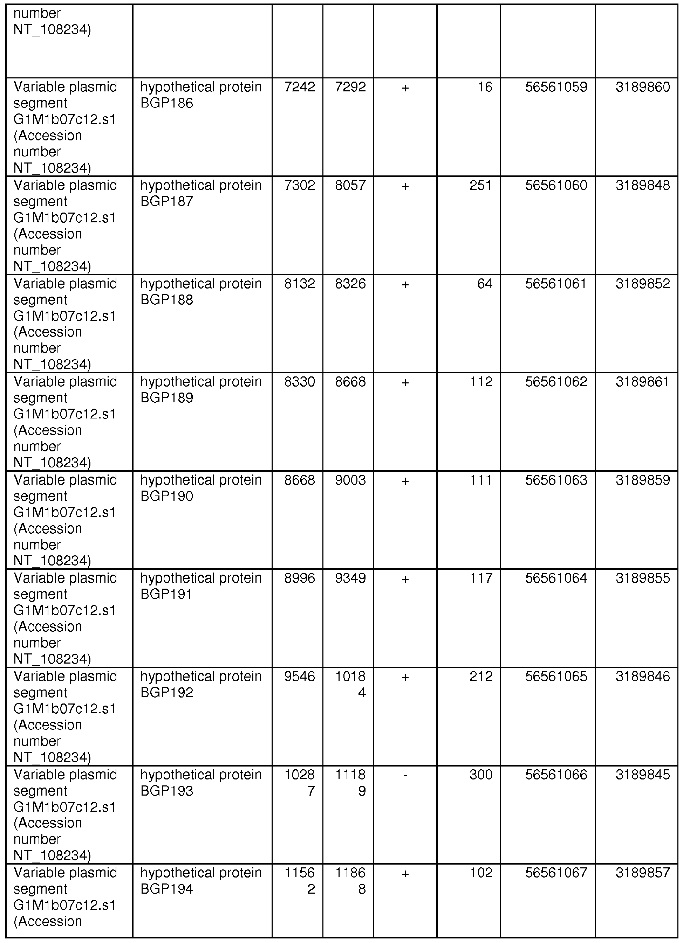

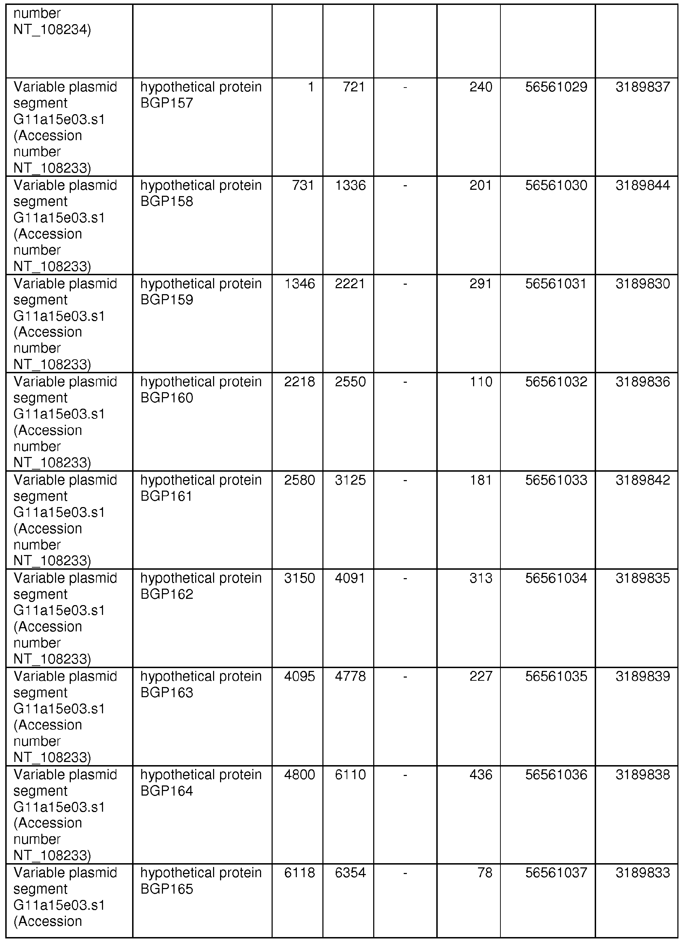

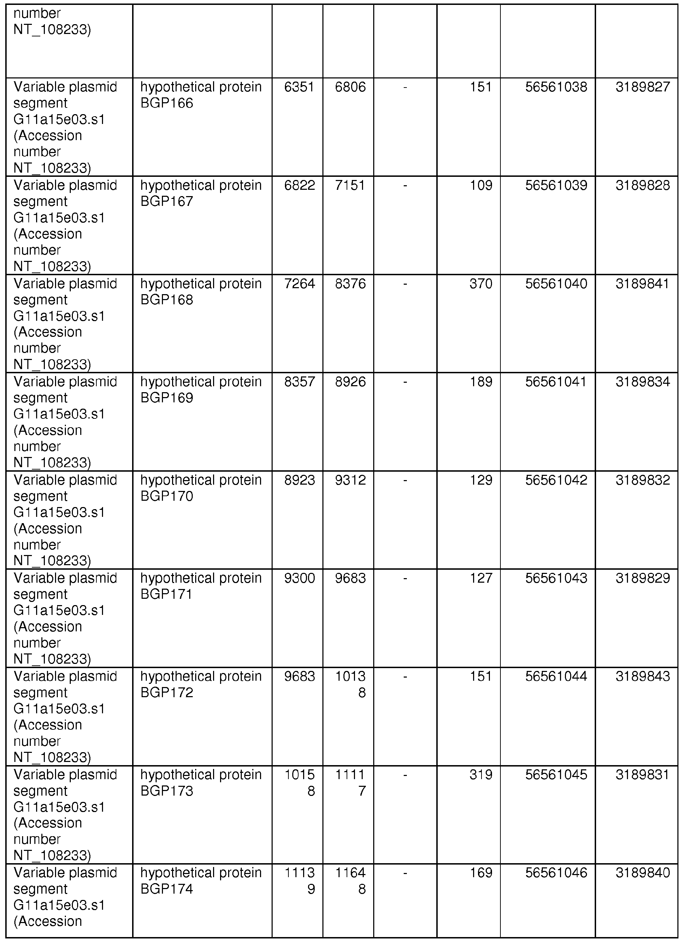

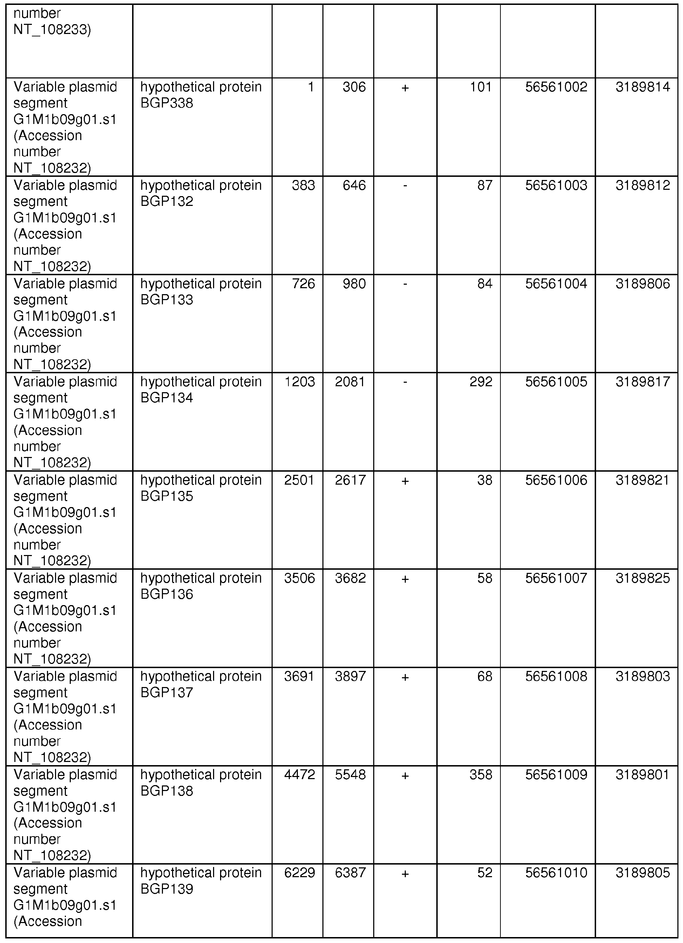

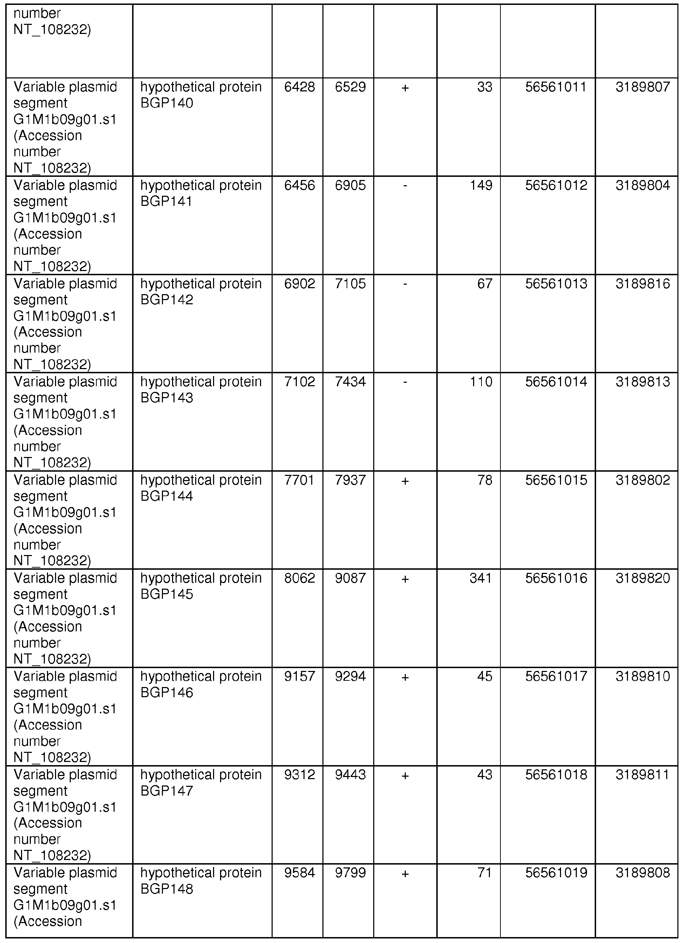

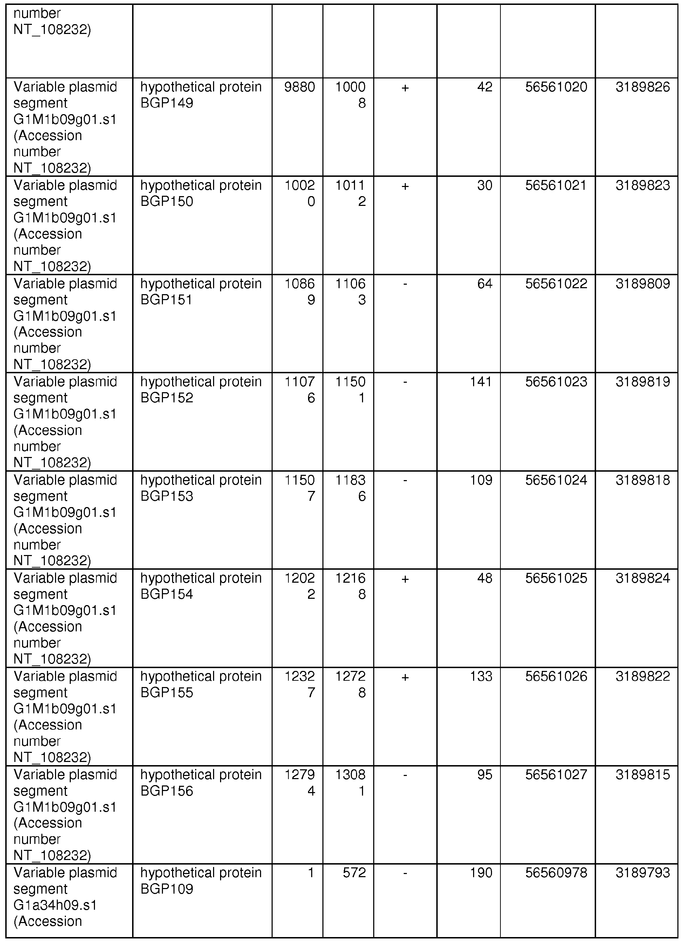

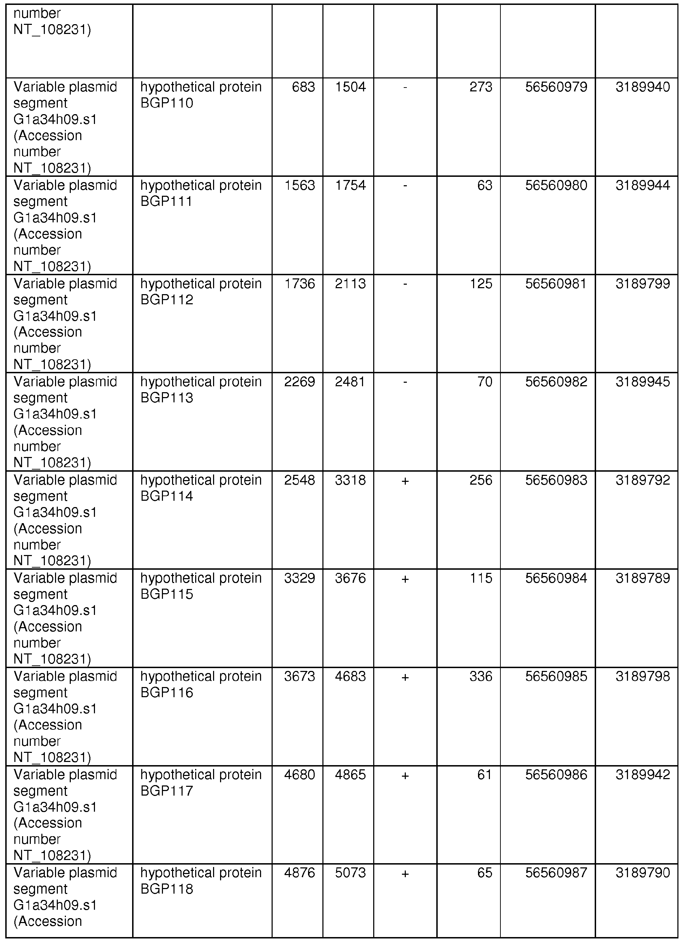

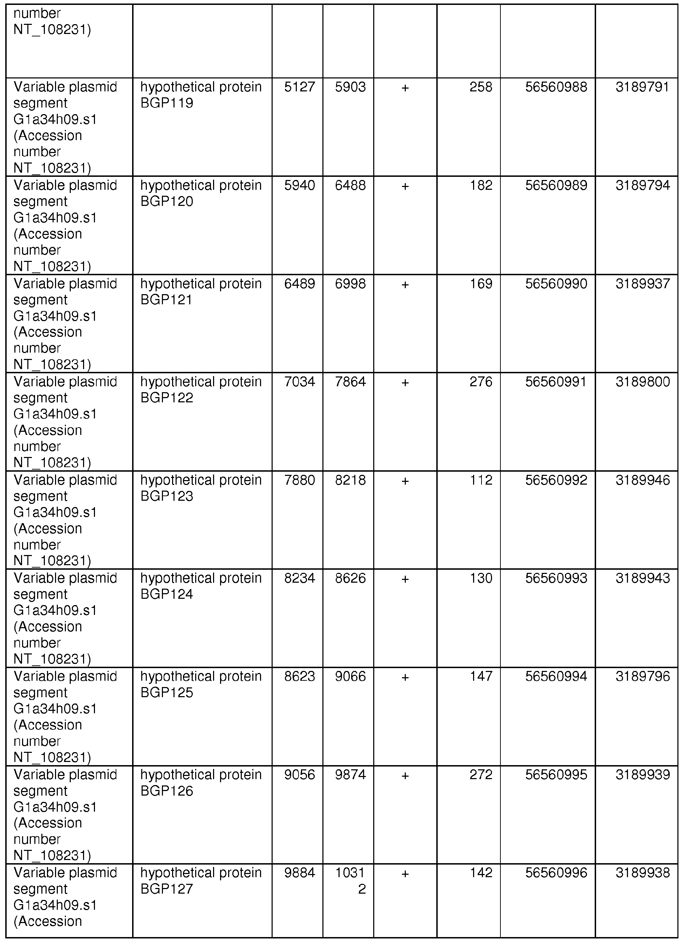

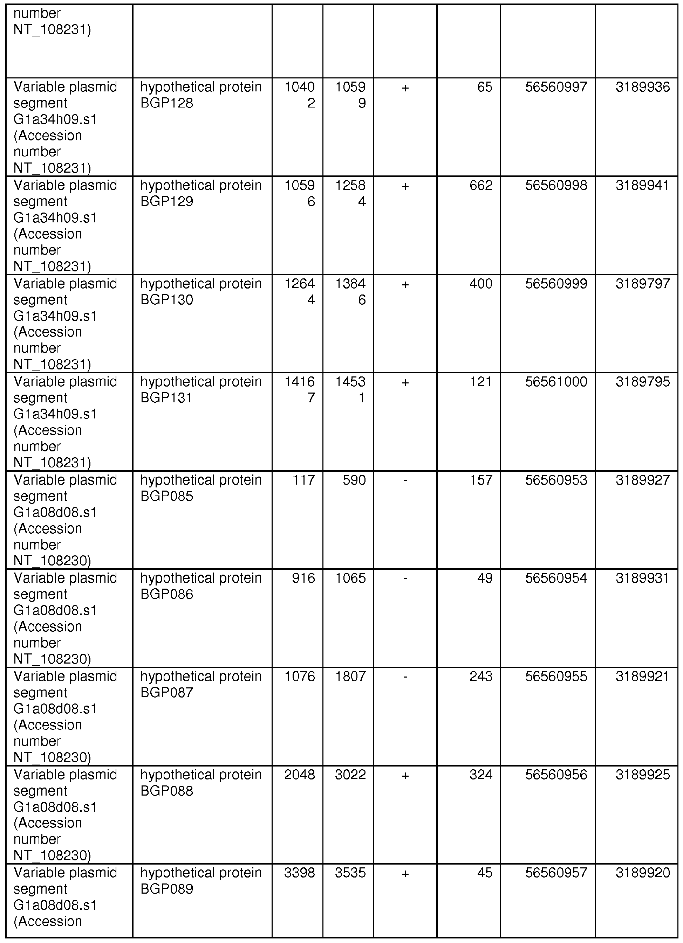

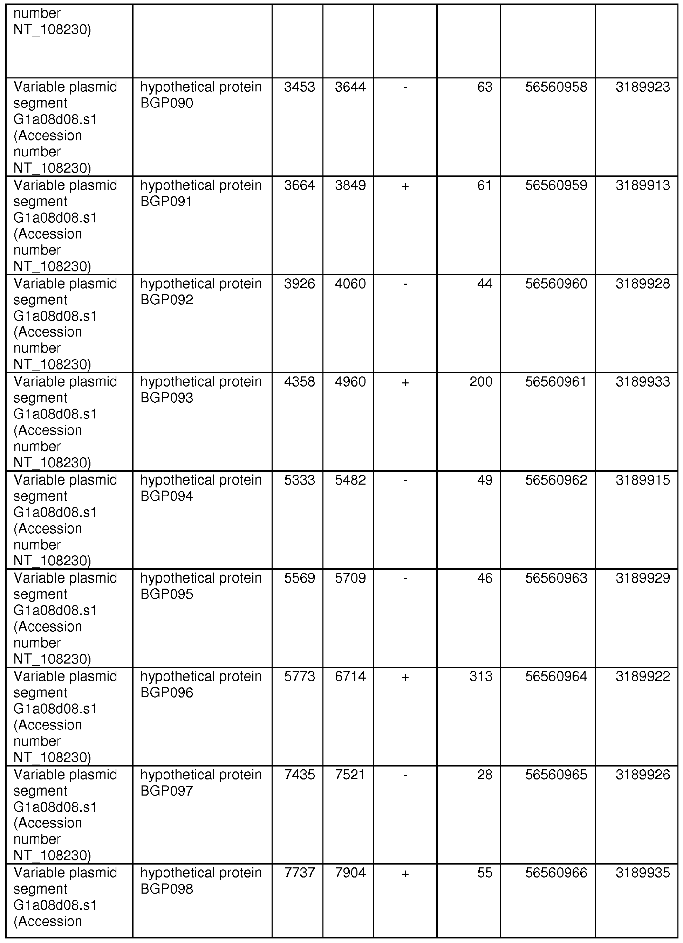

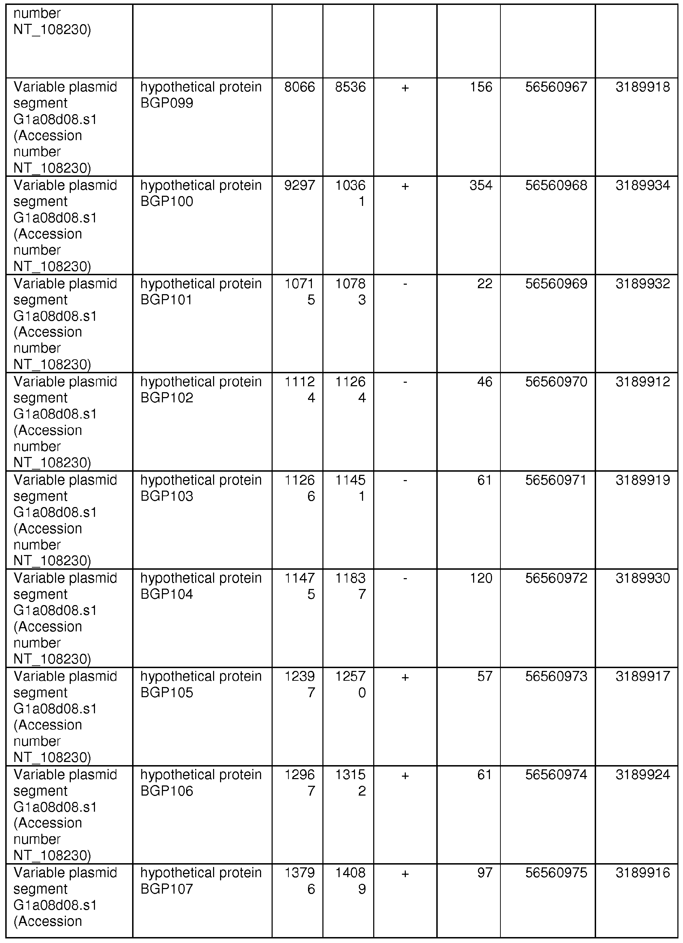

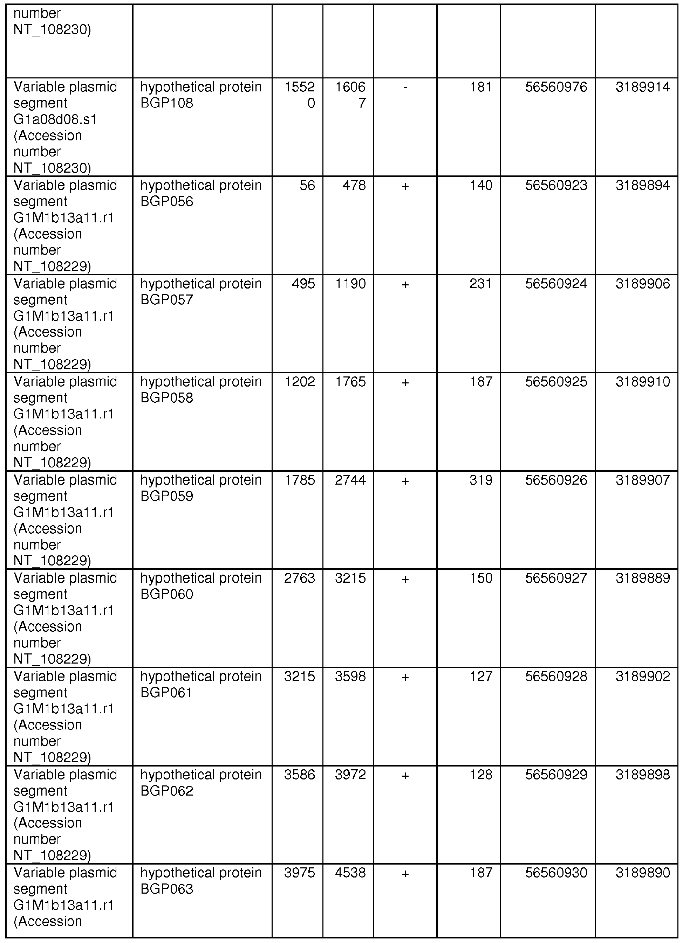

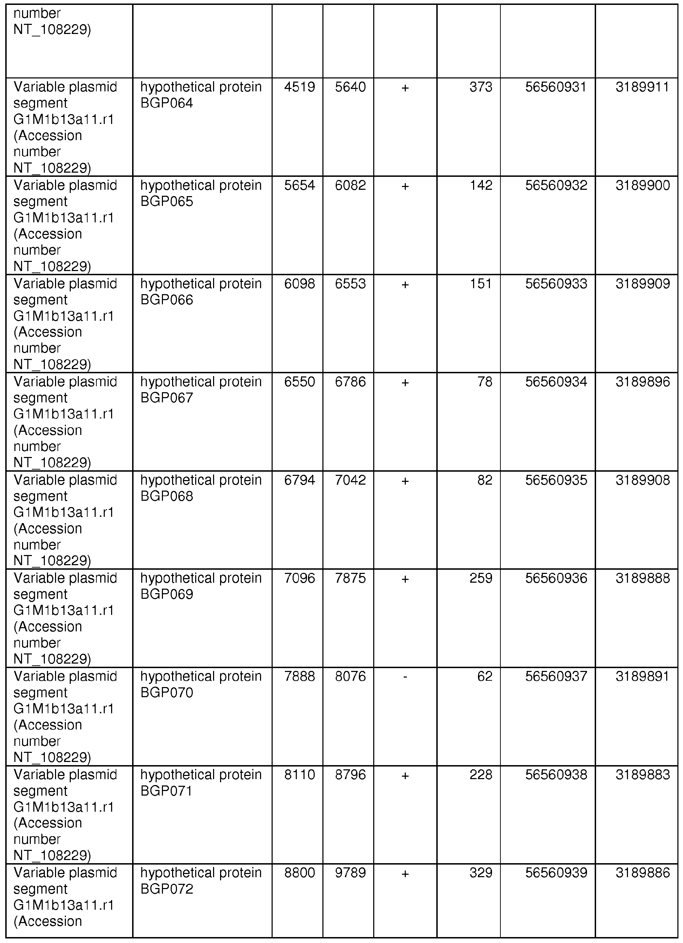

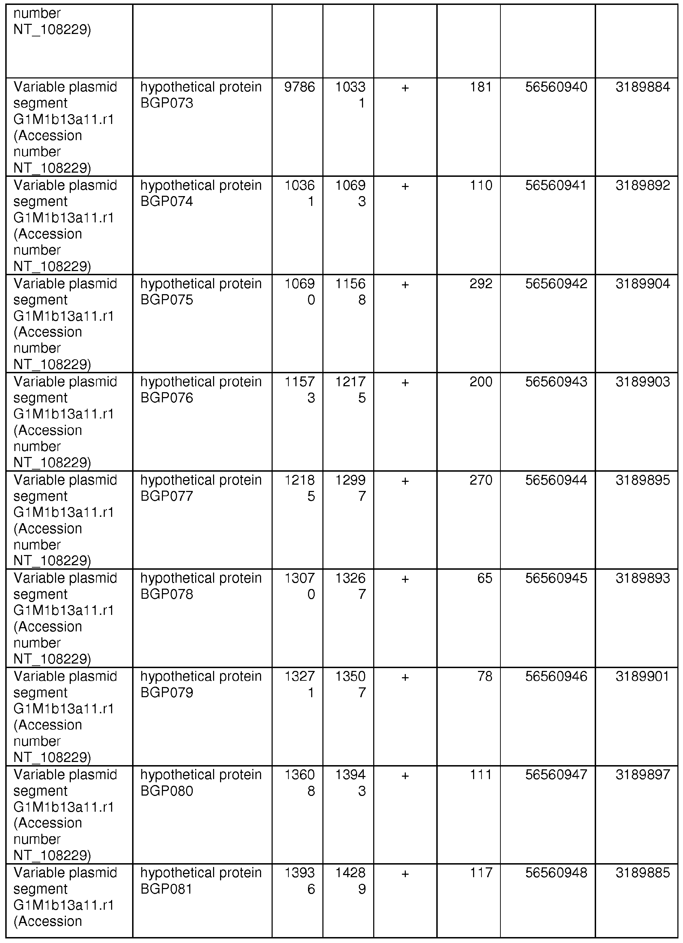

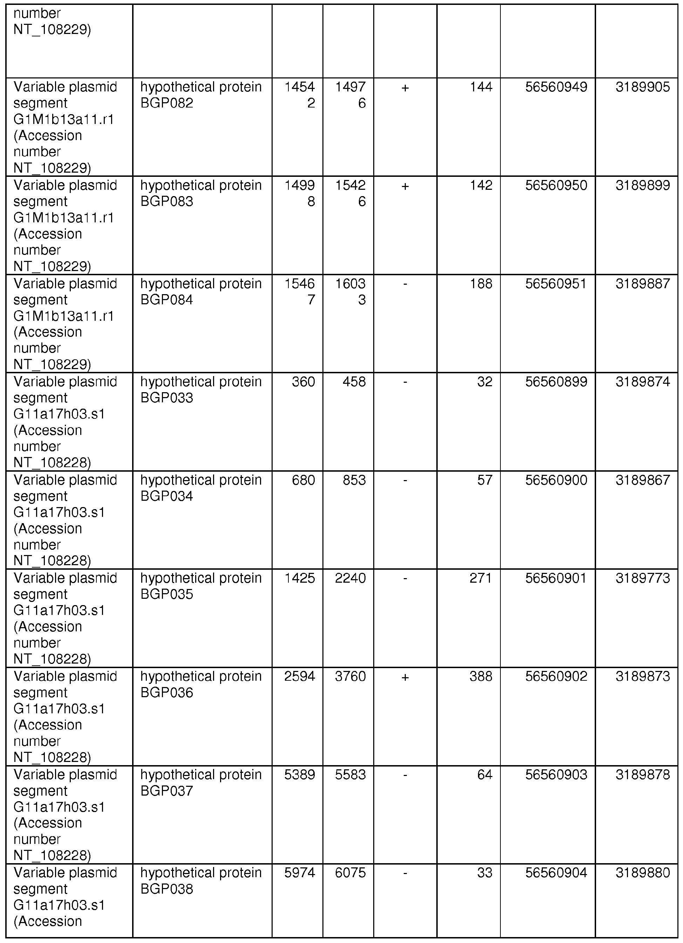

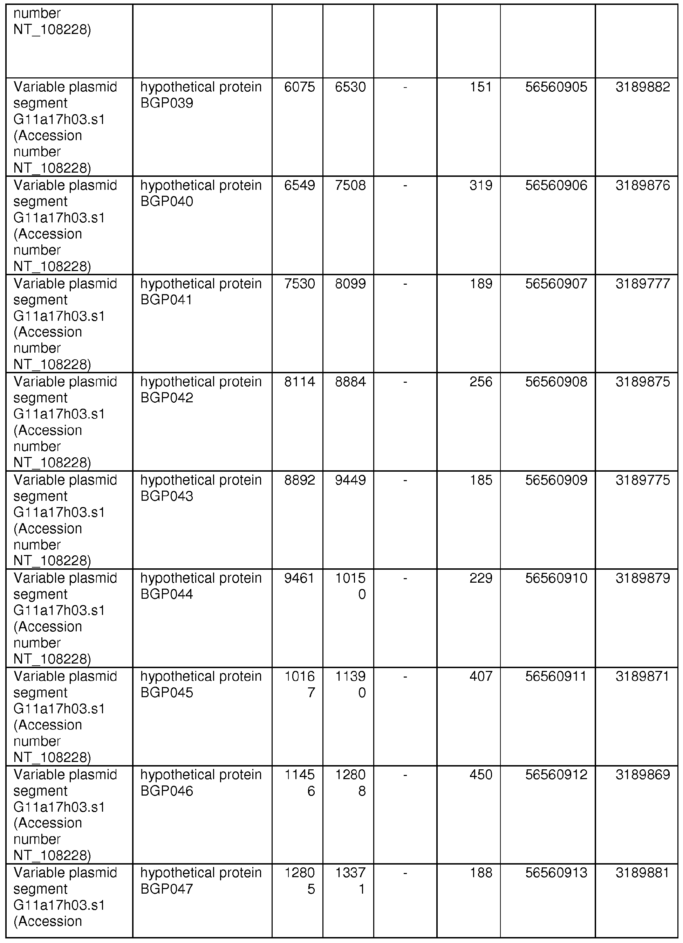

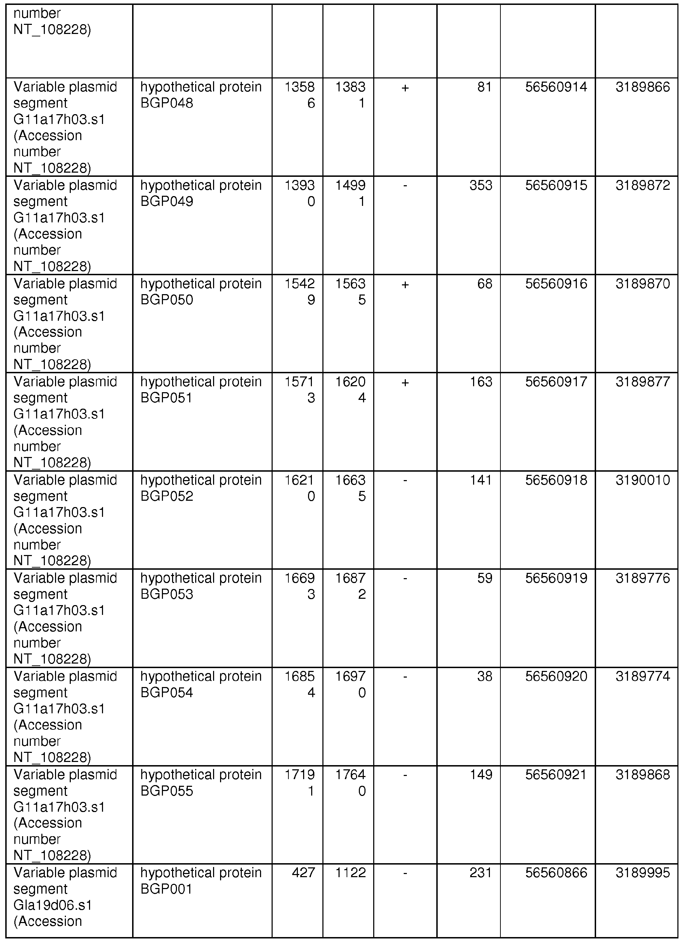

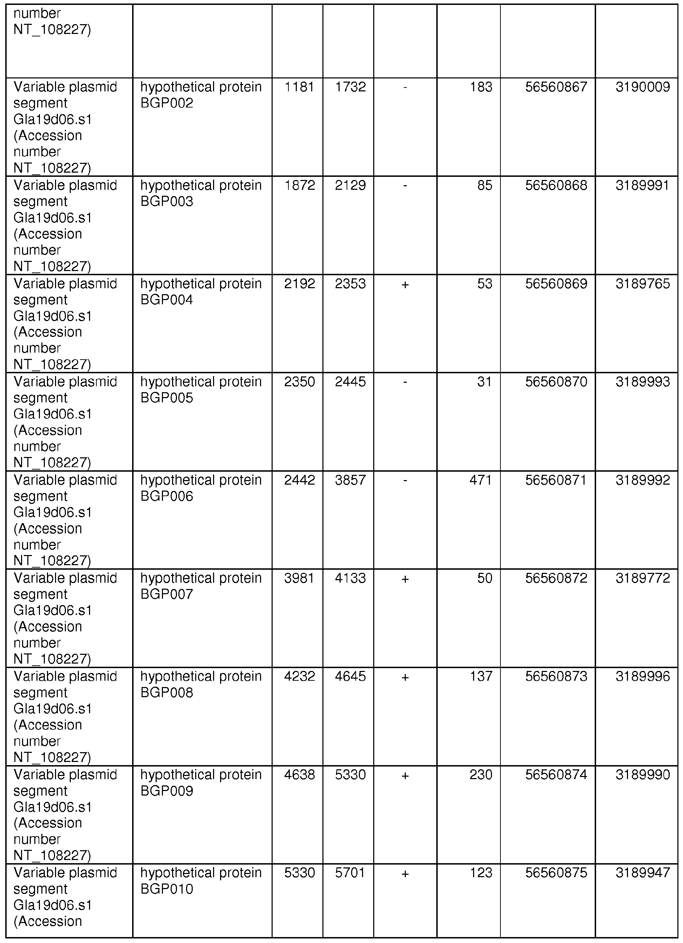

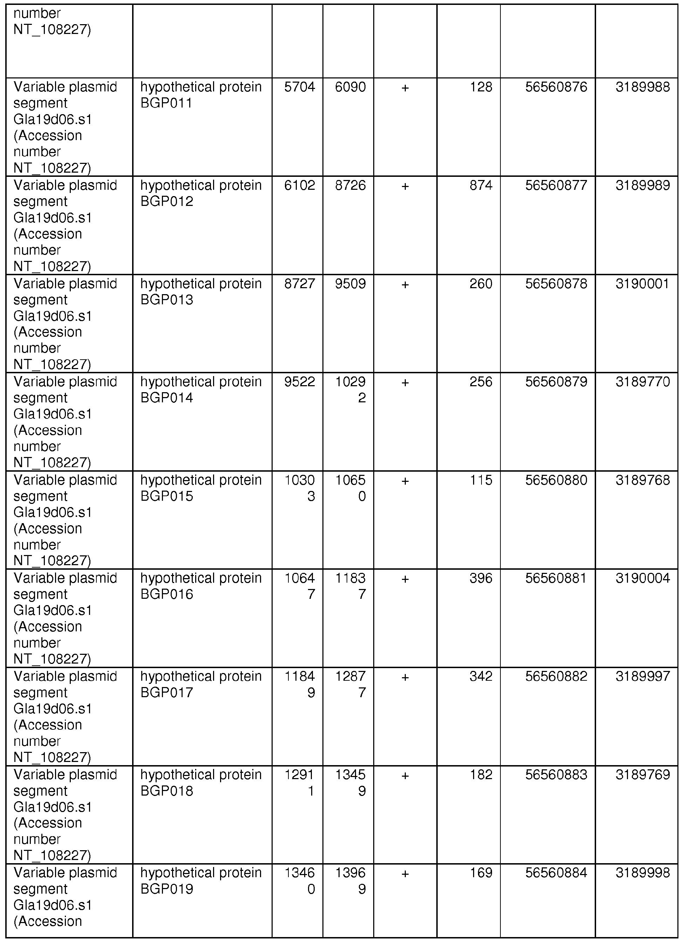

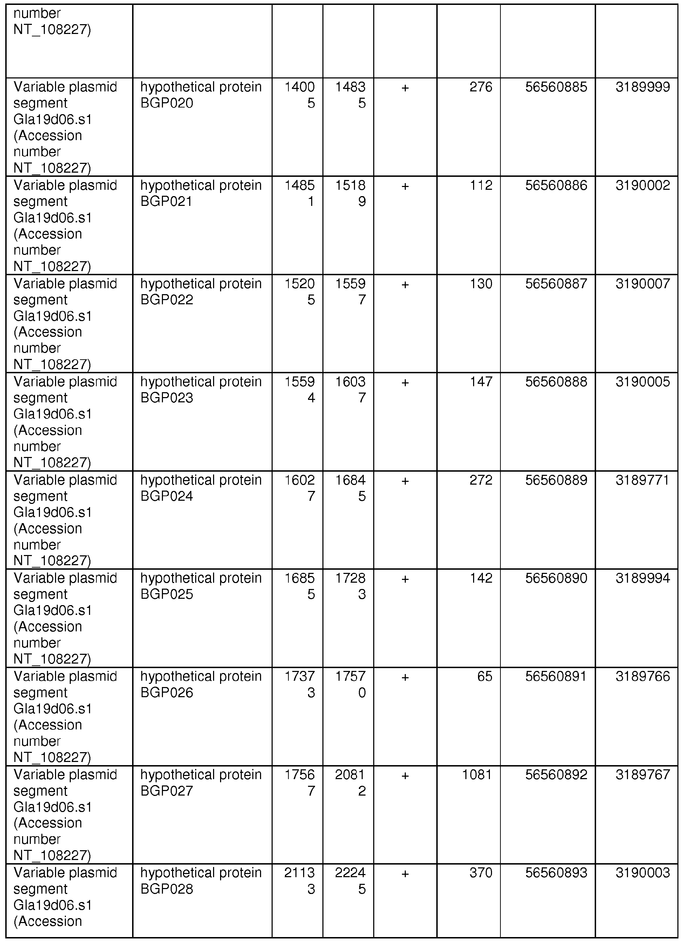

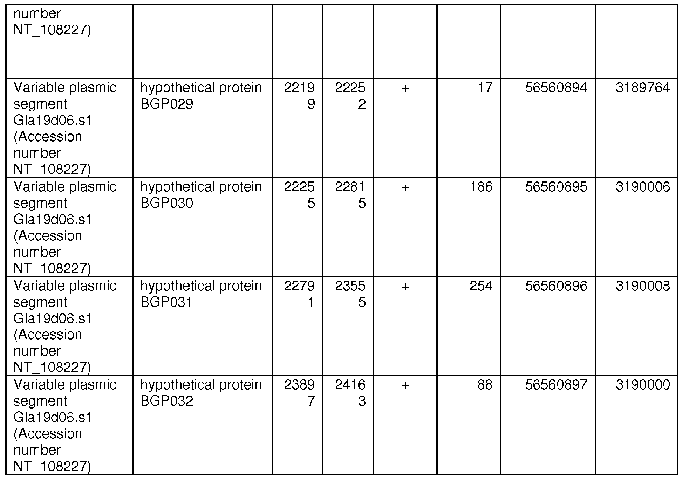

In another preferred embodiment the present disclosure relates to one or more antigenic peptides or antigenic polypeptides comprising a fragment of the sequence from one or more proteins from Borrelia garinii PBi, or to one or more MHC-peptide complexes or MHC multimers, compricing one or more such antigenic peptides. The one or more proteins can be selected from the proteins listed in Figure 29 and Figure 38. These fagments can e.g. be 8 mers, 9 mers, 10 mers, 11 mers, 12 mers, 13 mers, 14 mers, 15 mers or 16 mers.

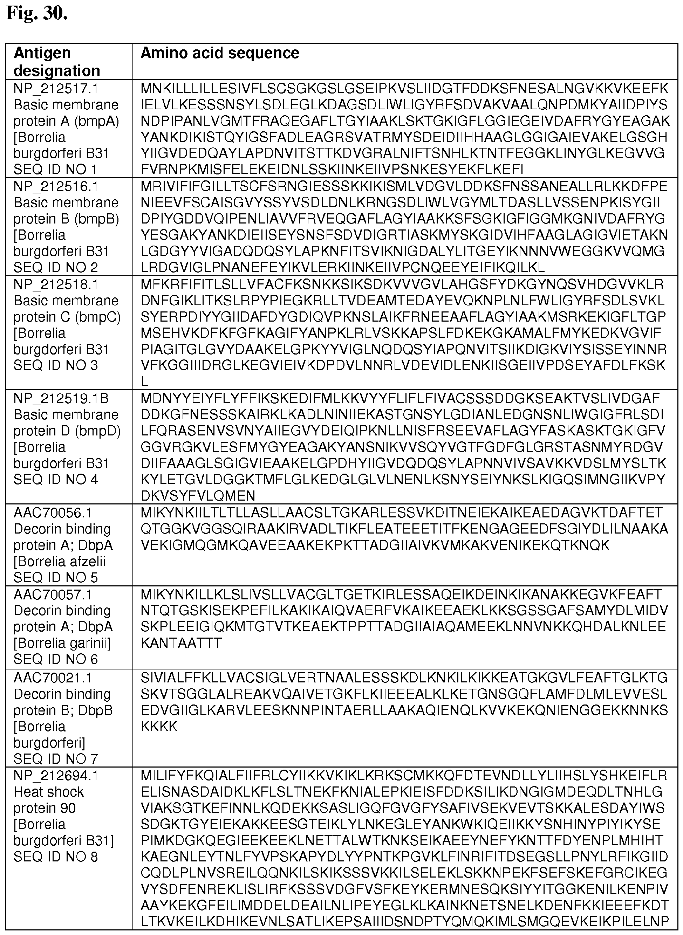

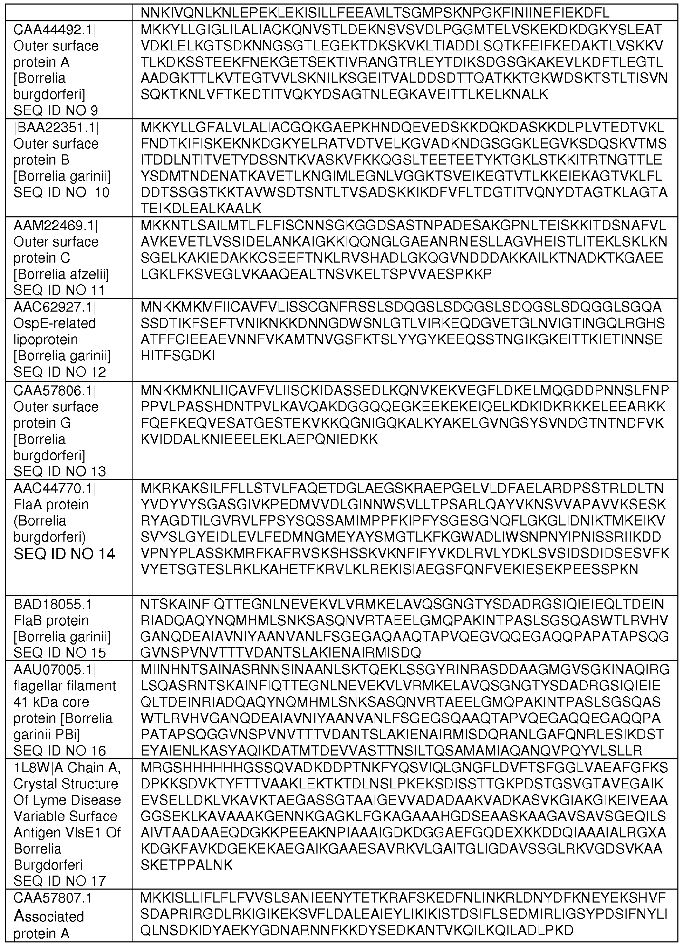

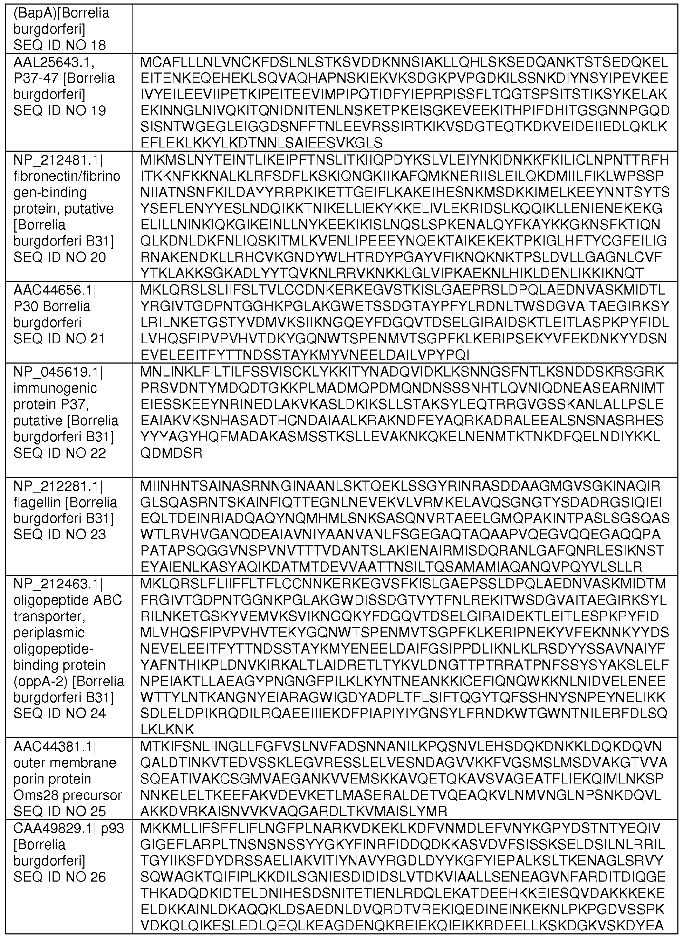

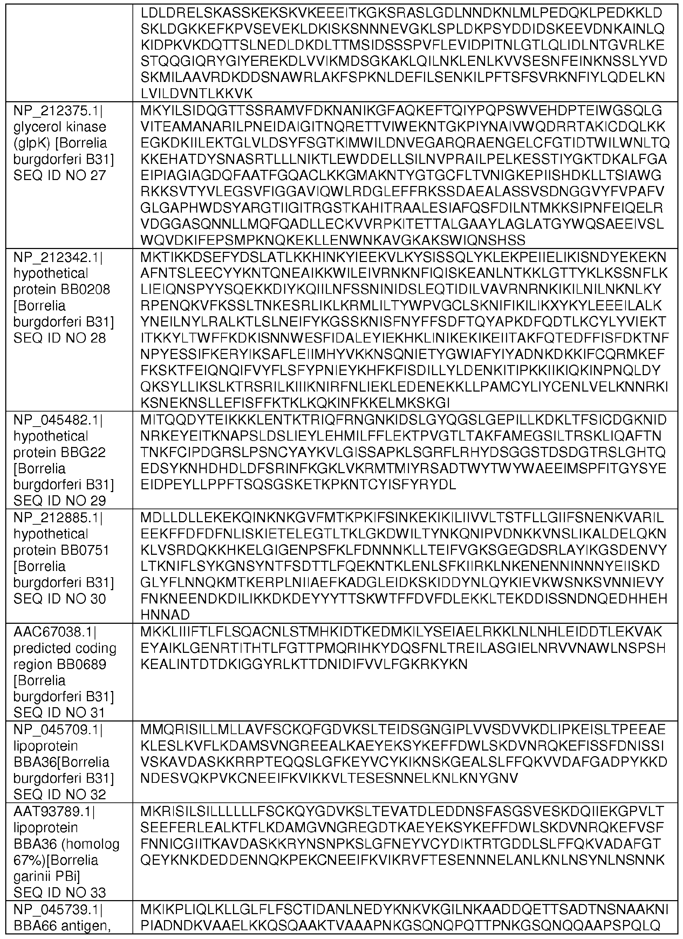

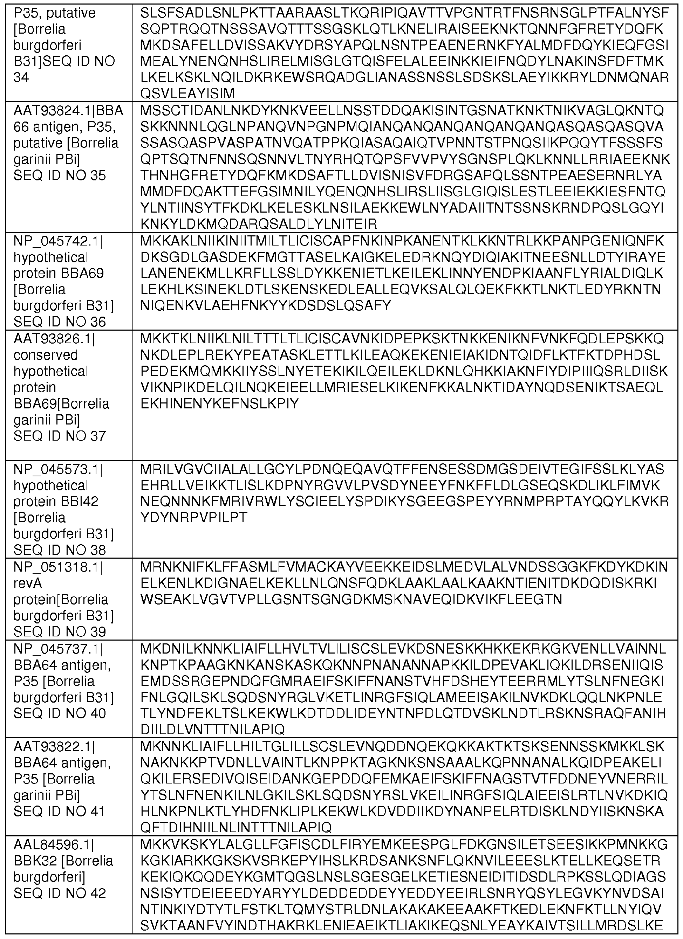

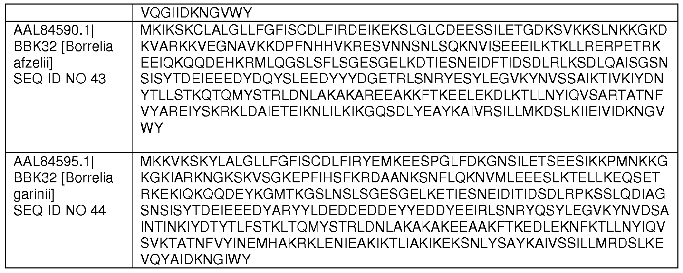

In another preferred embodiment the present disclosure relates to one or more antigenic peptides or antigenic polypeptides comprising a fragment of the sequence from one or more proteins listed in figure 30 and Figure 38, or to one or more MHC-peptide complexes or MHC multimers, compricing one or more such antigenic peptides. These fagments can e.g. be 8 mers, 9 mers, 10 mers, 11 mers, 12 mers, 13 mers, 14 mers, 15 mers or 16 mers.

In yet another preferred embodiment the present disclosure relates to one or more antigenic peptides or antigenic polypeptides or MHC-peptide complexes containing such antigenic peptides, where the antigenic peptides or antigenic polypeptides comprise a fragment of the sequence from one or more protein(s) from one or more of the Borrelia species or subspecies: Borrelia anserina, Borrelia barbouri, Borrelia afzelii, Borrelia afzelii ACA-1, Borrelia afzelii K78, Borrelia afzelii PKo, Borrelia andersonii, Borrelia bissettii, Borrelia burgdorferi, Borrelia burgdorferi 118a, Borrelia burgdorferi 156a, Borrelia burgdorferi 29805, Borrelia burgdorferi 64b, Borrelia burgdorferi 72a, Borrelia burgdorferi 80a, Borrelia burgdorferi 94a, Borrelia burgdorferi B31, Borrelia burgdorferi Bol26, Borrelia burgdorferi CA-11.2a, Borrelia burgdorferi Borrelia burgdorferi ZS7, Borrelia californiensis, Borrelia garinii, Borrelia garinii PBi, Borrelia garinii PBr, Borrelia genomosp. 1, Borrelia genomosp. 2, Borrelia japonica, Borrelia lusitaniae, Borrelia spielmanii, Borrelia spielmanii A14S, Borrelia tanukii, Borrelia turdi, Borrelia valaisiana, Borrelia valaisiana VS116, Candidatus Borrelia texasensis, Borrelia sp. AA4Pool, Borrelia sp. AI-1, Borrelia sp. B31, Borrelia sp. BC-1, Borrelia sp. CA1133, Borrelia sp. CA1176, Borrelia sp. CA128, Borrelia sp. CA13, Borrelia sp. CA134, Borrelia sp. CA142, Borrelia sp. CA20, Borrelia sp. CA22, Borrelia sp. CA27, Borrelia sp. CA28, Borrelia sp. CA29, Borrelia sp. CA31, Borrelia sp. CA33, Borrelia sp. CA370, Borrelia sp. CA372, Borrelia sp. CA378, Borrelia sp. CA388, Borrelia sp. CA393, Borrelia sp. CA394, Borrelia sp. CA395, Borrelia sp. CA399, Borrelia sp. CA400, Borrelia sp. CA401, Borrelia sp. CA402, Borrelia sp. CA404, Borrelia sp. CA411, Borrelia sp. CA426, Borrelia sp. CA443, Borrelia sp. CA446, Borrelia sp. CA448, Borrelia sp. CA462, Borrelia sp. CA468, Borrelia sp. CA502, Borrelia sp. CA504, Borrelia sp. CA507, Borrelia sp. CA547, Borrelia sp. CA552, Borrelia sp. CA8, Borrelia sp. D22, Borrelia sp. D35, Borrelia sp. FD-1, Borrelia sp. FL18, Borrelia sp. FL27, Borrelia sp. FL35, Borrelia sp. FL42, Borrelia sp. HN6, Borrelia sp. HN7, Borrelia sp. HN8, Borrelia sp. HNM13, Borrelia sp. HNM14, Borrelia sp. HNM19, Borrelia sp. IA1, Borrelia sp. Ir-3519, Borrelia sp. Ir-4721, Borrelia sp. Ir-4812, Borrelia sp. Ir-5215, Borrelia sp. LV5, Borrelia sp. MI-2, Borrelia sp. MI-5, Borrelia sp. MI-6, Borrelia sp. MI-8, Borrelia sp. MI-9, Borrelia sp. MOD-1, Borrelia sp. MOD-5, Borrelia sp. MOK-3a, Borrelia sp. MOS-1b, Borrelia sp. NE49, Borrelia sp. NE581, Borrelia sp. PHaP, Borrelia sp. PSigII, Borrelia sp. SCGT-10, Borrelia sp. SCGT-8a, Borrelia sp. SCI-2, Borrelia sp. SCW-30h, Borrelia sp. SI-1, Borrelia sp. SI-10, Borrelia sp. SM-1, Borrelia sp. SV1, Borrelia sp. W97F51, Borrelia sp. Z41293, Borrelia sp. Z41493, Borrelia coriaceae, Borrelia crocidurae, Borrelia duttonii, Borrelia duttonii Ly, Borrelia hermsii, Borrelia hermsii DAH, Borrelia hispanica, Borrelia lonestari, Borrelia miyamotoi, Borrelia parkeri, Borrelia persica, Borrelia recurrentis, Borrelia recurrentis A1, Borrelia sinica, Borrelia theileri, Borrelia turcica, Borrelia turicatae, Borrelia turicatae 91E135, Borrelia sp., Borrelia sp. 'Lake Gaillard', Borrelia sp. 000133, Borrelia sp. 010298, Borrelia sp. 10MT, Borrelia sp. 5145, Borrelia sp. 57Nsk, Borrelia sp. 5MT, Borrelia sp. 6T04-2, Borrelia sp. BR, Borrelia sp. BR 2007, Borrelia sp. C5-N52, Borrelia sp. CB-A1, Borrelia sp. CB-A11, Borrelia sp. CB-A3, Borrelia sp. EFL-S0100110, Borrelia sp. IK/23, Borrelia sp. IM/16, Borrelia sp. IM/19, Borrelia sp. KR1, Borrelia sp. KR3, Borrelia sp. LB-2001, Borrelia sp. LB-M56, Borrelia sp. LB-W100, Borrelia sp. MK-N61, Borrelia sp. NR-N8, Borrelia sp. OkME1, Borrelia sp. PAnz, Borrelia sp. PJes, Borrelia sp. PMai, Borrelia sp. PMew, Borrelia sp. R57, Borrelia sp. strain Spain, Borrelia sp. TA1, Borrelia sp. TM, Borrelia sp. TM1 and Borrelia sp. TM2. These peptides can e.g. be 8 mers, 9 mers, 10 mers, 11 mers, 12 mers, 13 mers, 14 mers, 15 mers or 16 mers.

Borrelia class I antigen peptide fragments

In one preferred embodiment the present disclosure relates to one or more MHC-peptide complexes or MHC multimers comprising one or more borrelia class I antigen peptide fragments or to the borrelia class I antigen peptide fragments themselves.

In one preferred embodiment the present disclosure relates to one or more MHC-peptide complexes or MHC multimers comprising one or more borrelia class I antigen peptide fragments, wherein said peptide fragments are 8 mers, 9 mers, 10 mers and/or 11 mers.

In one preferred embodiment the present disclosure relates to one or more antigenic peptides or antigenic polypeptides being borrelia class I antigen peptide fragments, wherein said peptide fragments are 8 mers, 9 mers, 10 mers and/or 11 mers.

In one preferred embodiment the present disclosure relates to one or more MHC-peptide complexes or MHC multimers comprising one or more borrelia class I antigen peptide fragments or to the borrelia class I antigenic peptide fragments themselves, wherein said peptide fragments are 8 mers, 9 mers, 10 mers and/or 11 mers of a protein from or encoded by Borrelia burgdorferi such as Borrelia burgdorferi B31.

In one preferred embodiment the present disclosure relates to one or more MHC-peptide complexes or MHC multimers comprising one or more borrelia class I antigen peptide fragments or to the borrelia class I antigenic peptide fragments themselves, wherein said peptide fragments are 8 mers, 9 mers, 10 mers and/or 11 mers of a protein from or encoded by Borrelia afzelii such as Borrelia afzelii PKo.

In one preferred embodiment the present disclosure relates to one or more MHC-peptide complexes or MHC multimers comprising one or more borrelia class I antigen peptide fragments or to the borrelia class I antigenic peptide fragments themselves, wherein said peptide fragments are 8 mers, 9 mers, 10 mers and/or 11 mers of a protein from or encoded by Borrelia garinii such as Borrelia garinii PBi.

In one preferred embodiment the present disclosure relates to one or more MHC-peptide complexes or MHC multimers comprising one or more borrelia class I antigen peptide fragments or to the borrelia class I antigenic peptide fragments themselves, wherein said peptide fragments are 8 mers, 9 mers, 10 mers and/or 11 mers of a protein from or encoded by Borrelia anserina, Borrelia barbouri, Borrelia afzelii, Borrelia afzelii ACA-1, Borrelia afzelii K78, Borrelia afzelii PKo, Borrelia andersonii, Borrelia bissettii, Borrelia burgdorferi, Borrelia burgdorferi 118a, Borrelia burgdorferi 156a, Borrelia burgdorferi 29805, Borrelia burgdorferi 64b, Borrelia burgdorferi 72a, Borrelia burgdorferi 80a, Borrelia burgdorferi 94a, Borrelia burgdorferi B31, Borrelia burgdorferi Bol26, Borrelia burgdorferi CA-11.2a, Borrelia burgdorferi Borrelia burgdorferi ZS7, Borrelia californiensis, Borrelia garinii, Borrelia garinii PBi, Borrelia garinii PBr, Borrelia genomosp. 1, Borrelia genomosp. 2, Borrelia japonica, Borrelia lusitaniae, Borrelia spielmanii, Borrelia spielmanii A14S, Borrelia tanukii, Borrelia turdi, Borrelia valaisiana, Borrelia valaisiana VS116, Candidatus Borrelia texasensis, Borrelia sp. AA4Pool, Borrelia sp. AI-1, Borrelia sp. B31, Borrelia sp. BC-1, Borrelia sp. CA1133, Borrelia sp. CA1176, Borrelia sp. CA128, Borrelia sp. CA13, Borrelia sp. CA134, Borrelia sp. CA142, Borrelia sp. CA20, Borrelia sp. CA22, Borrelia sp. CA27, Borrelia sp. CA28, Borrelia sp. CA29, Borrelia sp. CA31, Borrelia sp. CA33, Borrelia sp. CA370, Borrelia sp.

CA372, Borrelia sp. CA378, Borrelia sp. CA388, Borrelia sp. CA393, Borrelia sp. CA394, Borrelia sp. CA395, Borrelia sp. CA399, Borrelia sp. CA400, Borrelia sp. CA401, Borrelia sp. CA402, Borrelia sp. CA404, Borrelia sp. CA411, Borrelia sp. CA426, Borrelia sp. CA443, Borrelia sp. CA446, Borrelia sp. CA448, Borrelia sp. CA462, Borrelia sp. CA468, Borrelia sp. CA502, Borrelia sp. CA504, Borrelia sp. CA507, Borrelia sp. CA547, Borrelia sp. CA552, Borrelia sp. CA8, Borrelia sp. D22, Borrelia sp. D35, Borrelia sp. FD-1, Borrelia sp. FL18, Borrelia sp. FL27, Borrelia sp. FL35, Borrelia sp. FL42, Borrelia sp. HN6, Borrelia sp. HN7, Borrelia sp. HN8, Borrelia sp. HNM13, Borrelia sp. HNM14, Borrelia sp. HNM19, Borrelia sp. IA1, Borrelia sp. Ir-3519, Borrelia sp. Ir-4721, Borrelia sp. Ir-4812, Borrelia sp. Ir-5215, Borrelia sp. LV5, Borrelia sp. MI-2, Borrelia sp. MI-5, Borrelia sp. MI-6, Borrelia sp. MI-8, Borrelia sp. MI-9, Borrelia sp. MOD-1, Borrelia sp. MOD-5, Borrelia sp. MOK-3a, Borrelia sp. MOS-1b, Borrelia sp. NE49, Borrelia sp. NE581, Borrelia sp. PHaP, Borrelia sp. PSigII, Borrelia sp. SCGT-10, Borrelia sp. SCGT-8a, Borrelia sp. SCI-2, Borrelia sp. SCW-30h, Borrelia sp. SI-1, Borrelia sp. SI-10, Borrelia sp. SM-1, Borrelia sp. SV1, Borrelia sp. W97F51, Borrelia sp. Z41293, Borrelia sp. Z41493, Borrelia coriaceae, Borrelia crocidurae, Borrelia duttonii, Borrelia duttonii Ly, Borrelia hermsii, Borrelia hermsii DAH, Borrelia hispanica, Borrelia lonestari, Borrelia miyamotoi, Borrelia parkeri, Borrelia persica, Borrelia recurrentis, Borrelia recurrentis A1, Borrelia sinica, Borrelia theileri, Borrelia turcica, Borrelia turicatae, Borrelia turicatae 91E135, Borrelia sp., Borrelia sp. 'Lake Gaillard', Borrelia sp. 000133, Borrelia sp. 010298, Borrelia sp. 10MT, Borrelia sp. 5145, Borrelia sp. 57Nsk, Borrelia sp. 5MT, Borrelia sp. 6T04-2, Borrelia sp. BR, Borrelia sp. BR 2007, Borrelia sp. C5-N52, Borrelia sp. CB-A1, Borrelia sp. CB-A11, Borrelia sp. CB-A3, Borrelia sp. EFL-S0100110, Borrelia sp. IK/23, Borrelia sp. IM/16, Borrelia sp. IM/19, Borrelia sp. KR1, Borrelia sp. KR3, Borrelia sp. LB-2001, Borrelia sp. LB-M56, Borrelia sp. LB-W100, Borrelia sp. MK-N61, Borrelia sp. NR-N8, Borrelia sp. OkME1, Borrelia sp. PAnz, Borrelia sp. PJes, Borrelia sp. PMai, Borrelia sp. PMew, Borrelia sp. R57, Borrelia sp. strain Spain, Borrelia sp. TA1, Borrelia sp. TM, Borrelia sp. TM1 and/or Borrelia sp. TM2.

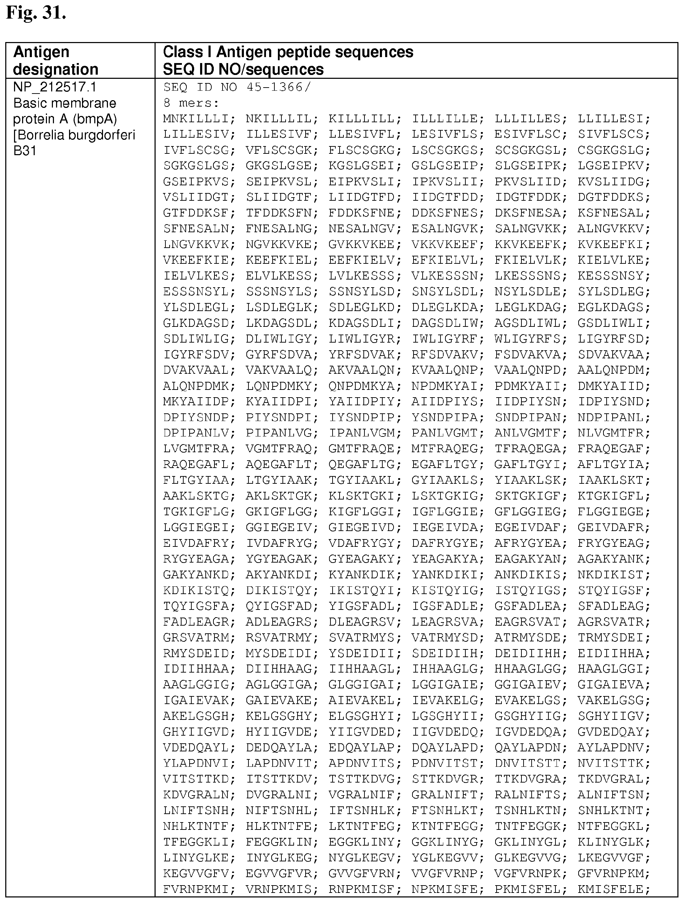

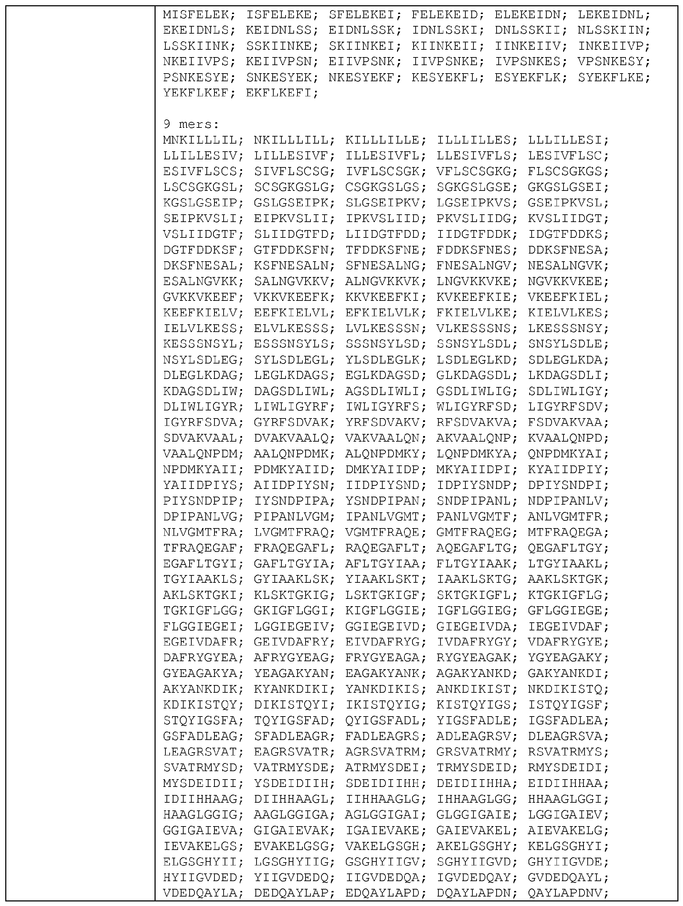

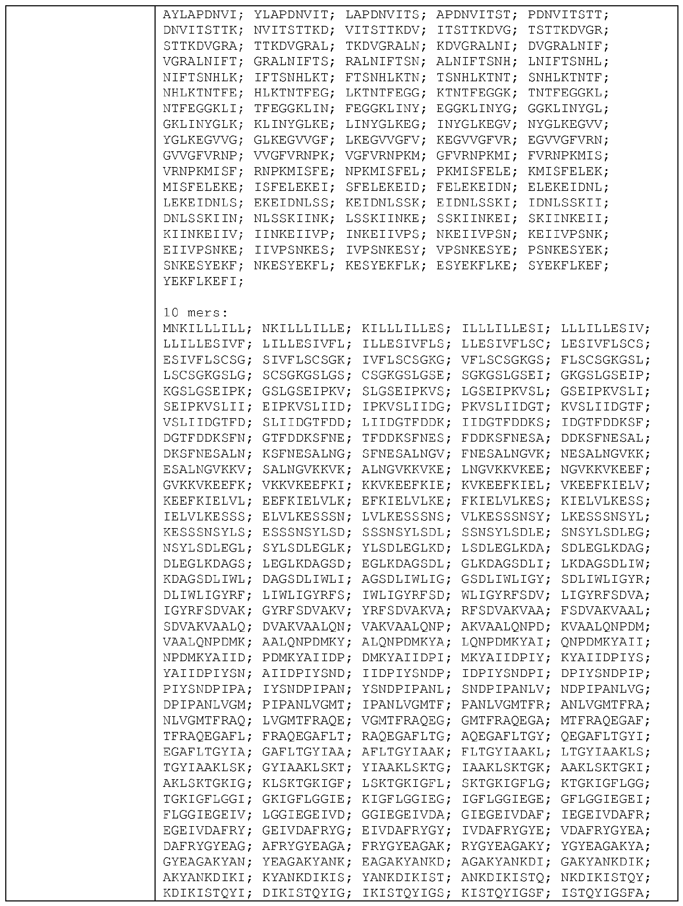

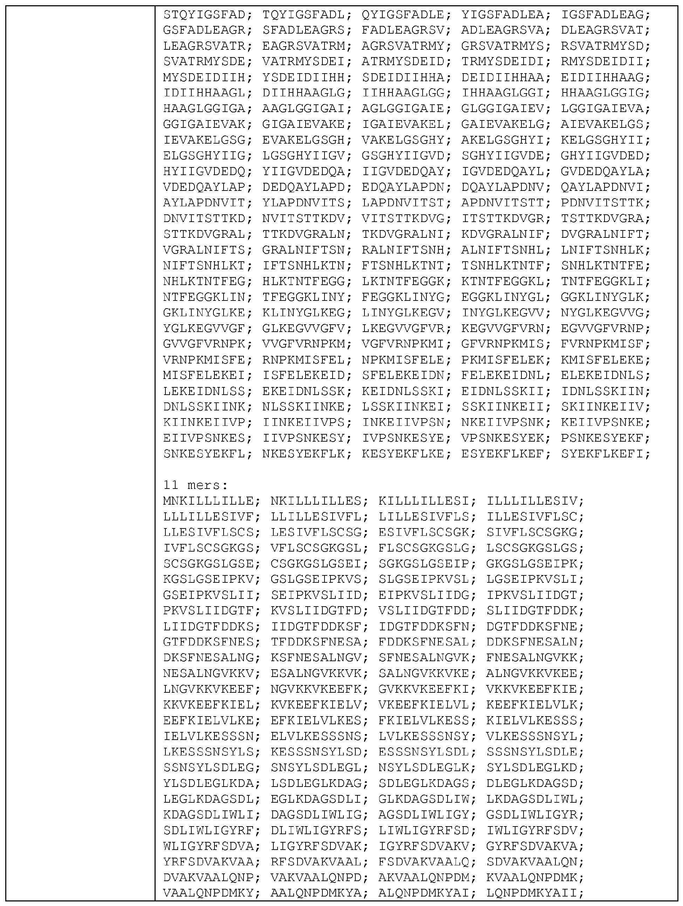

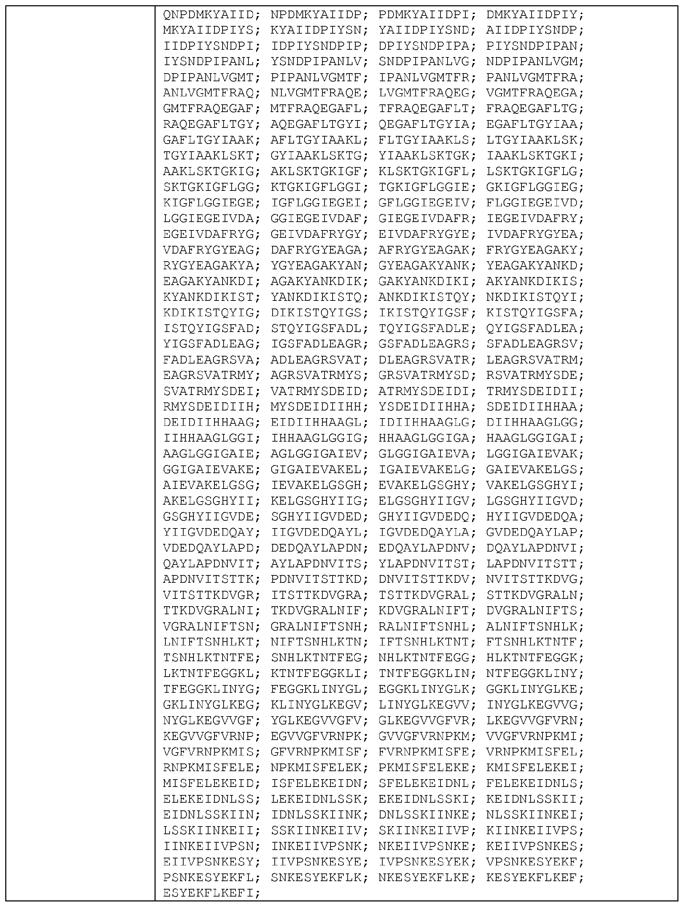

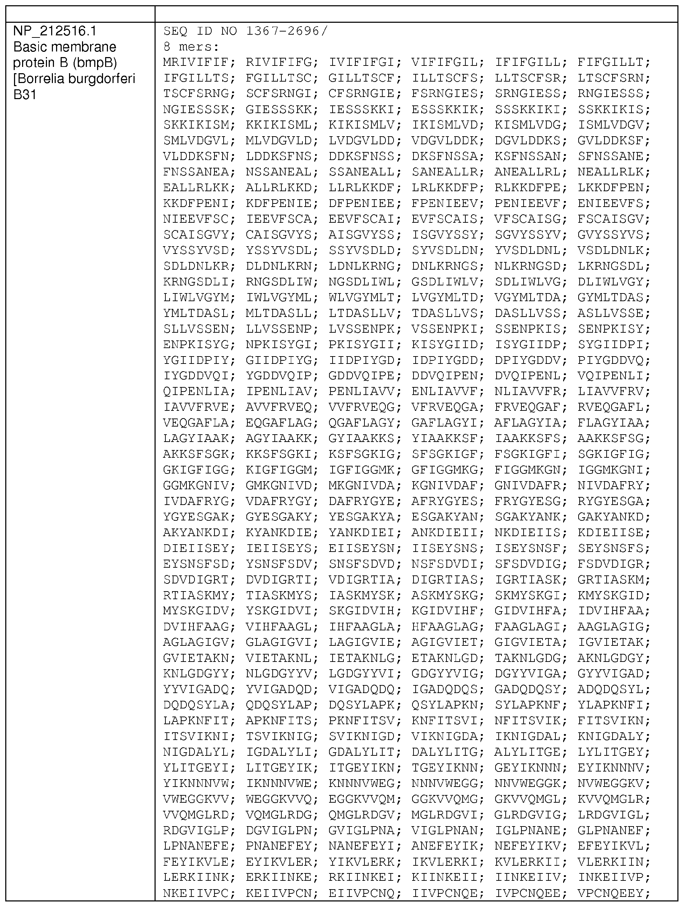

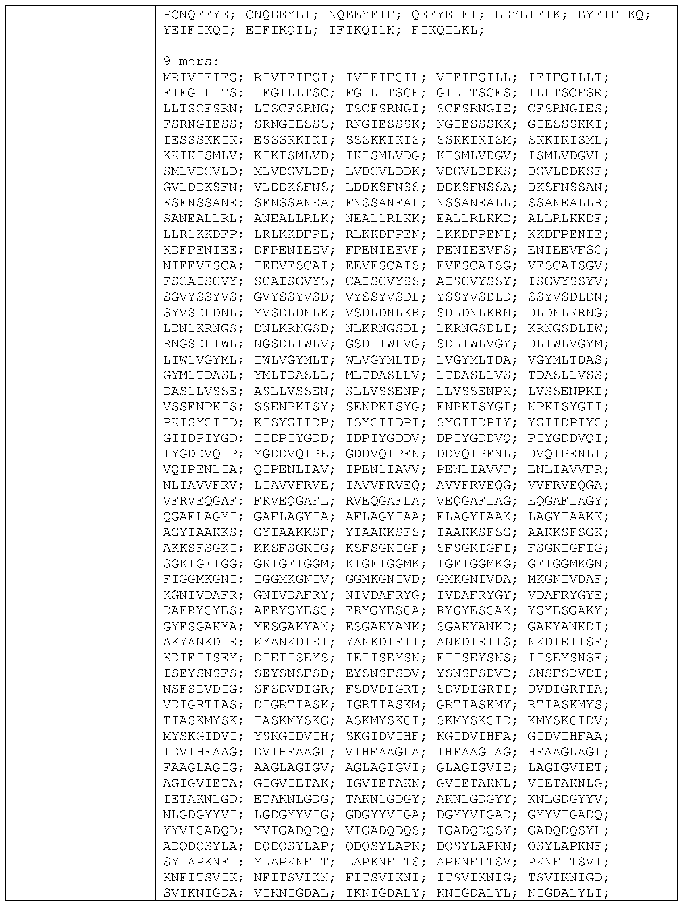

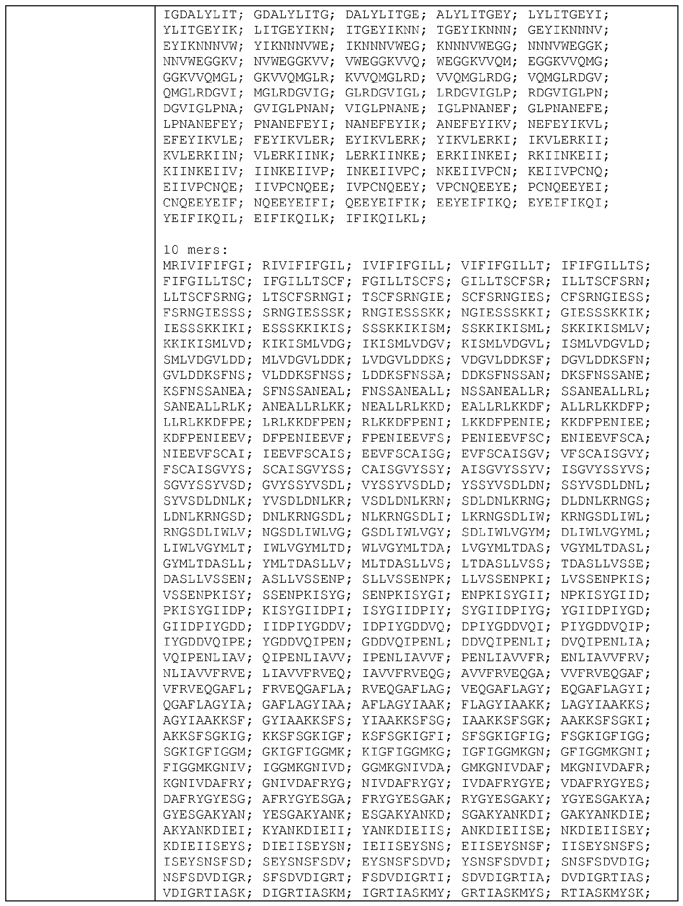

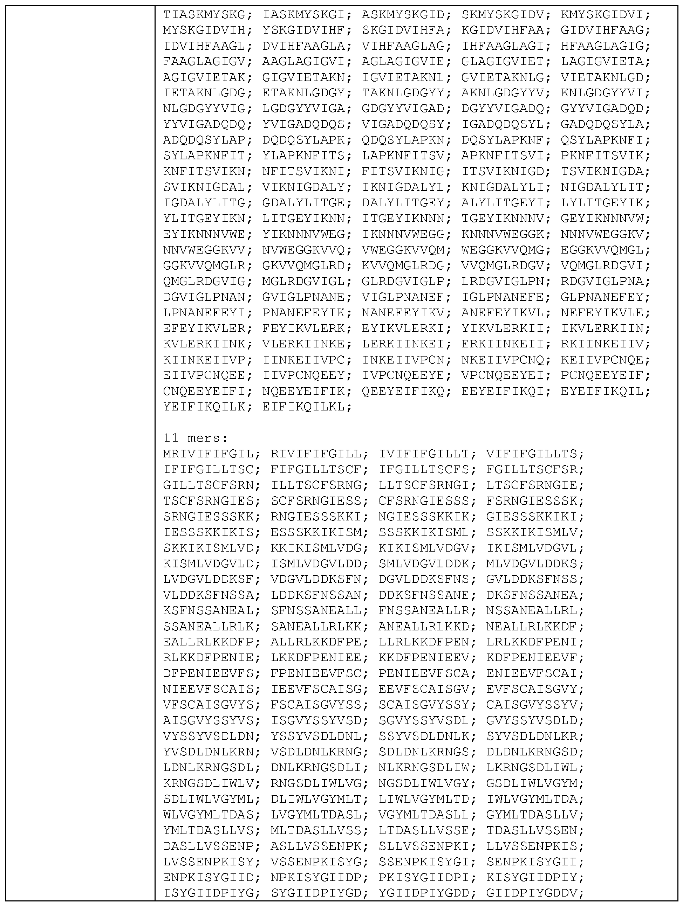

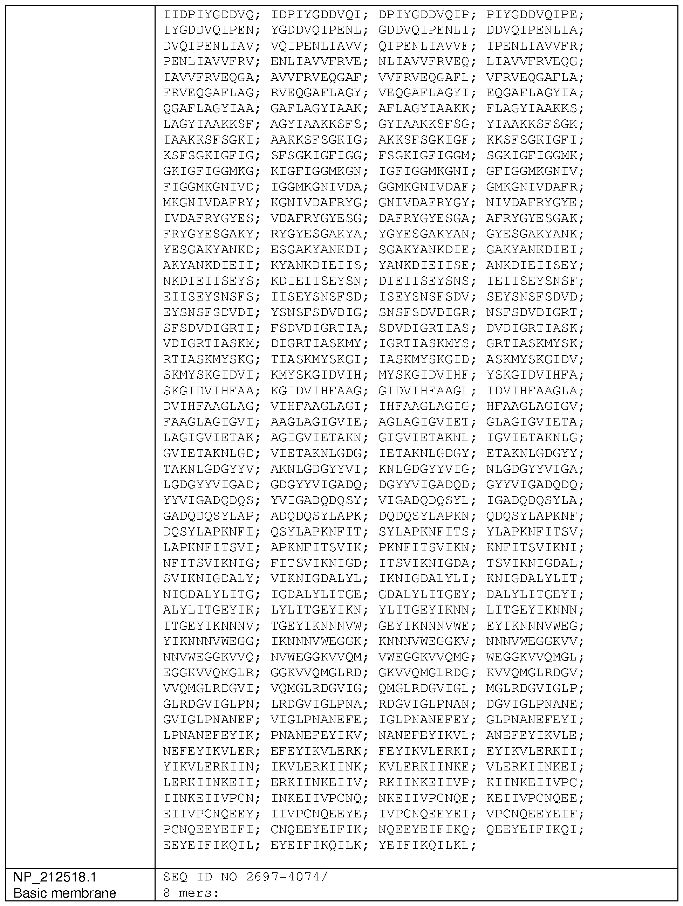

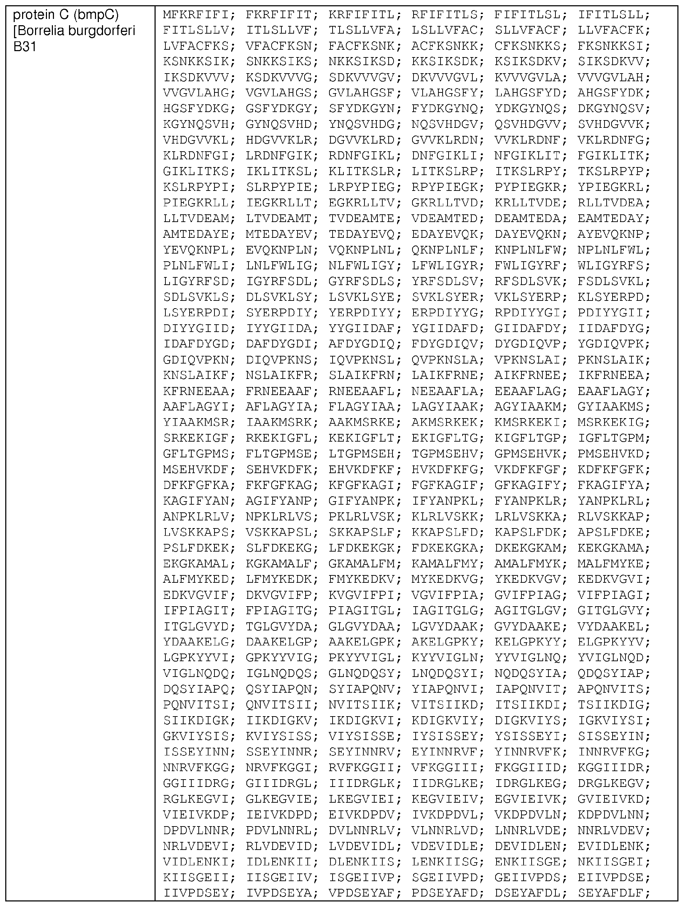

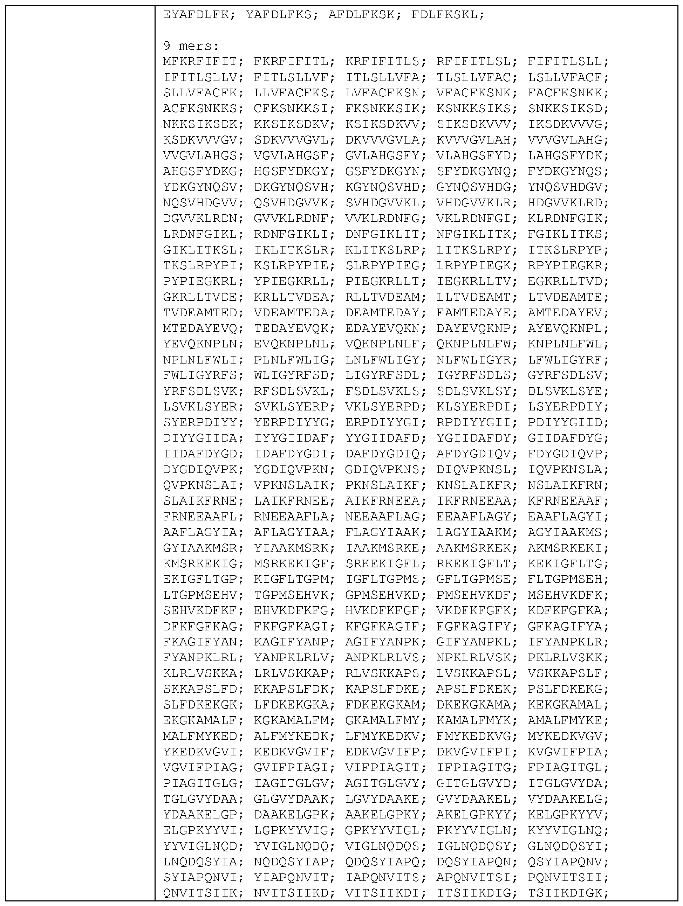

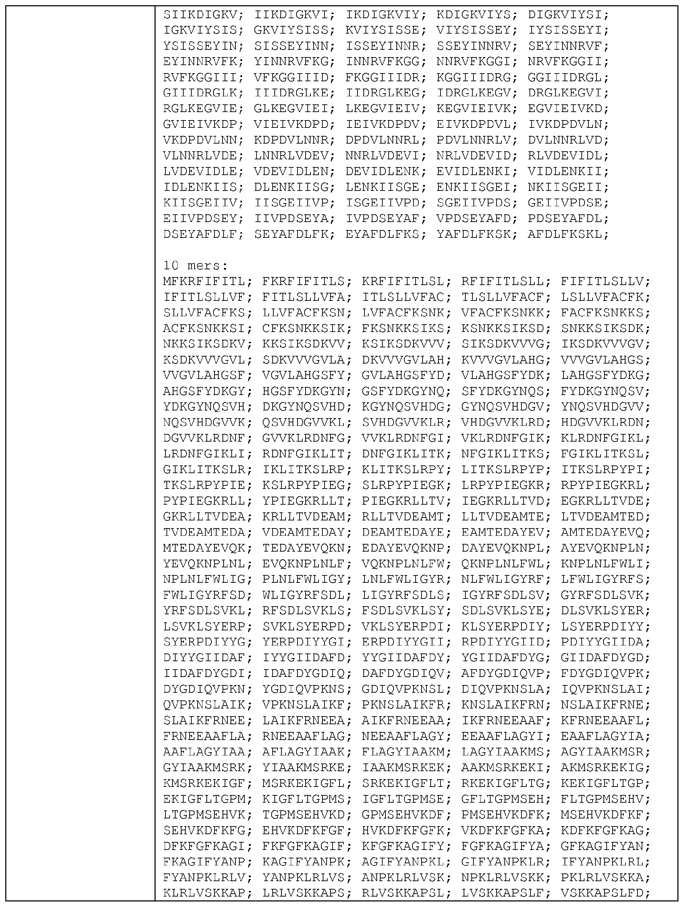

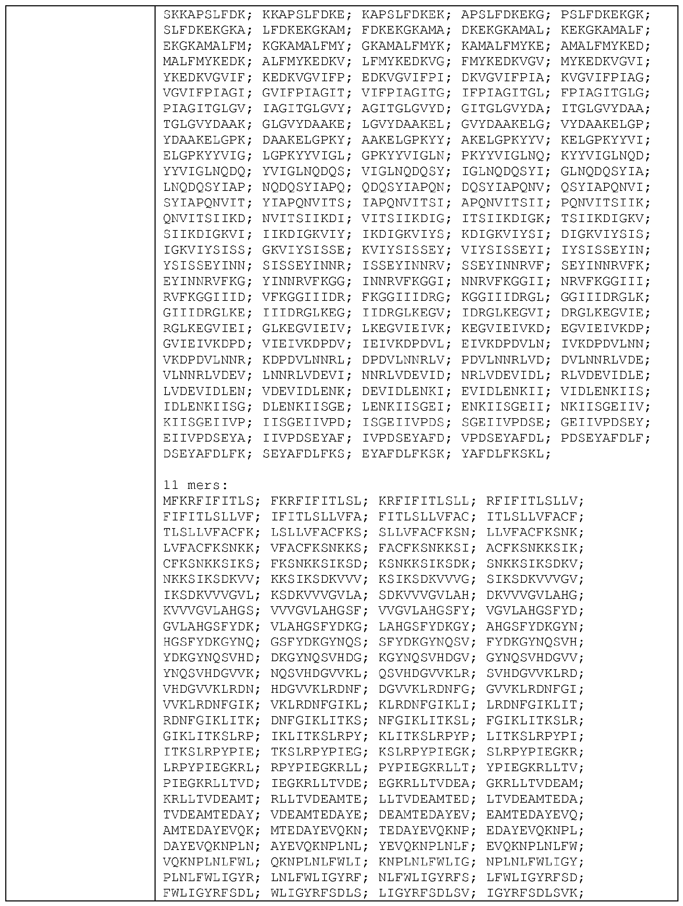

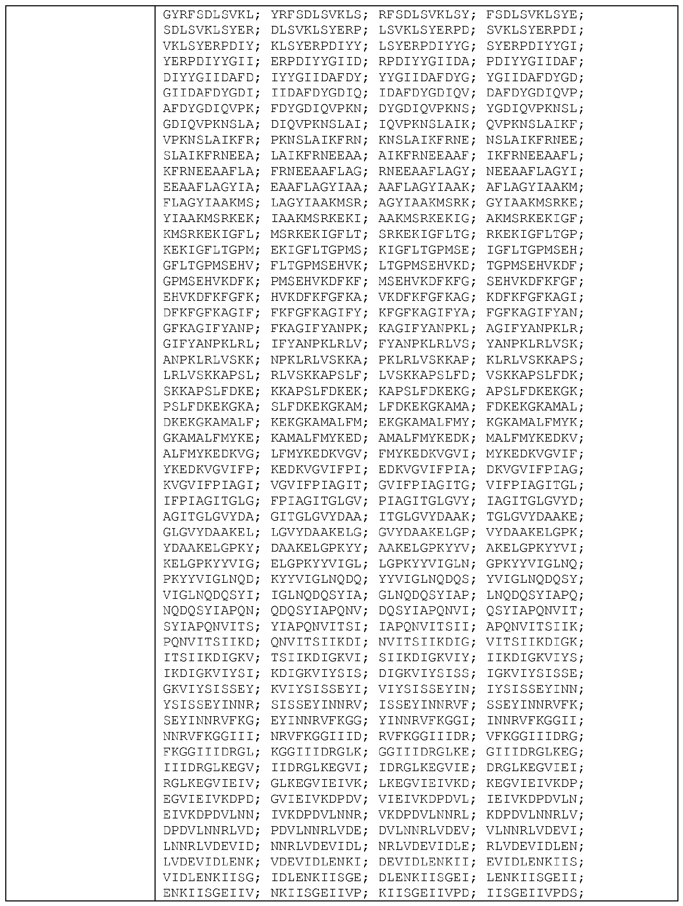

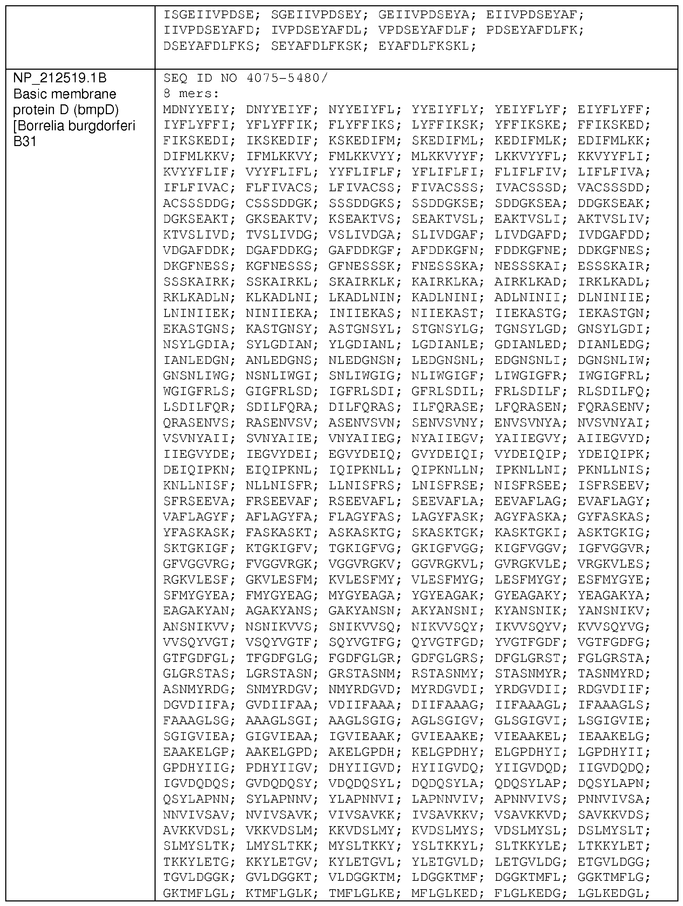

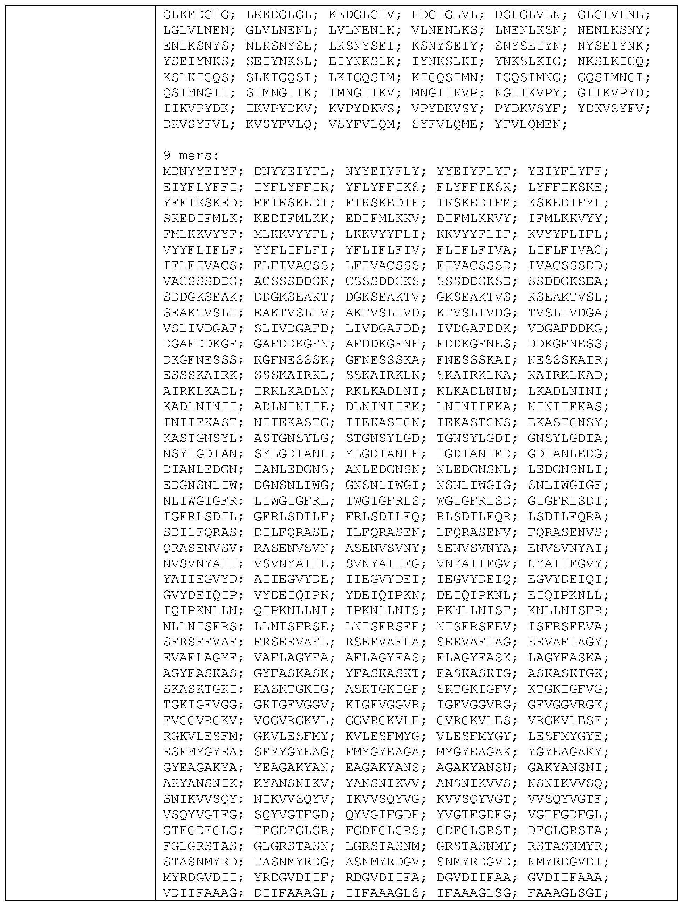

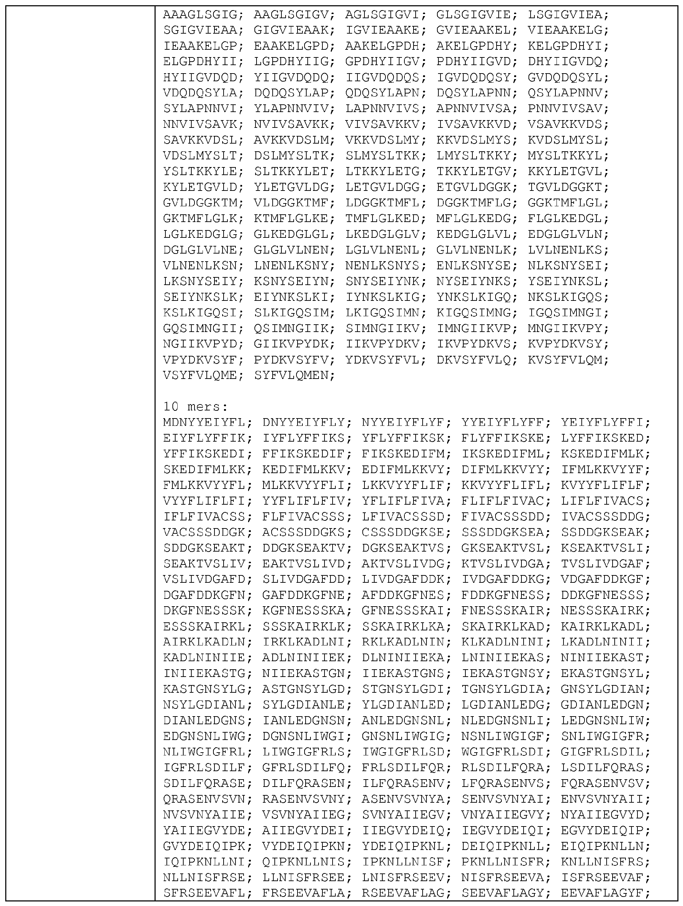

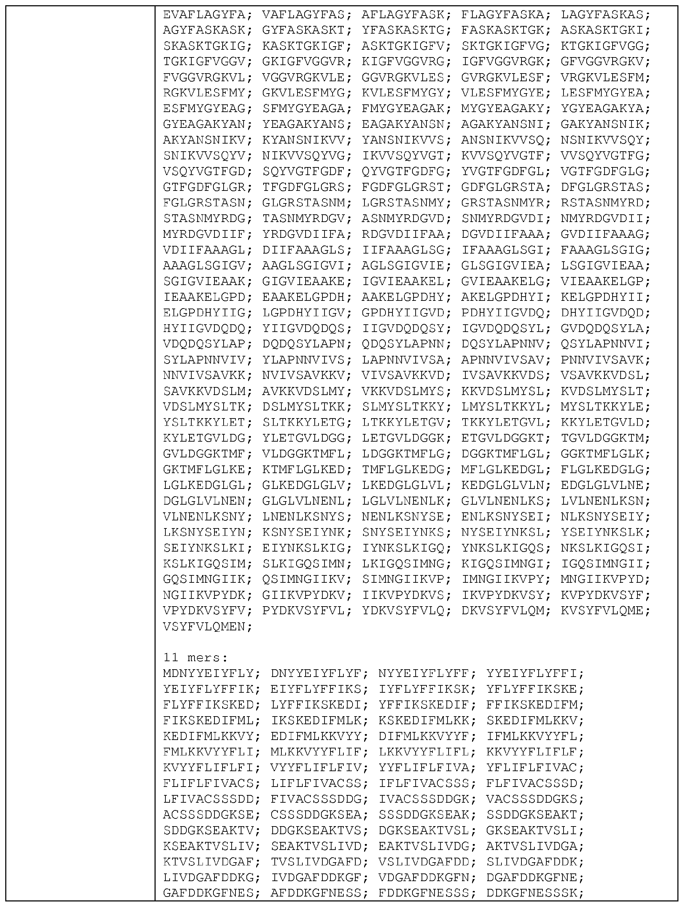

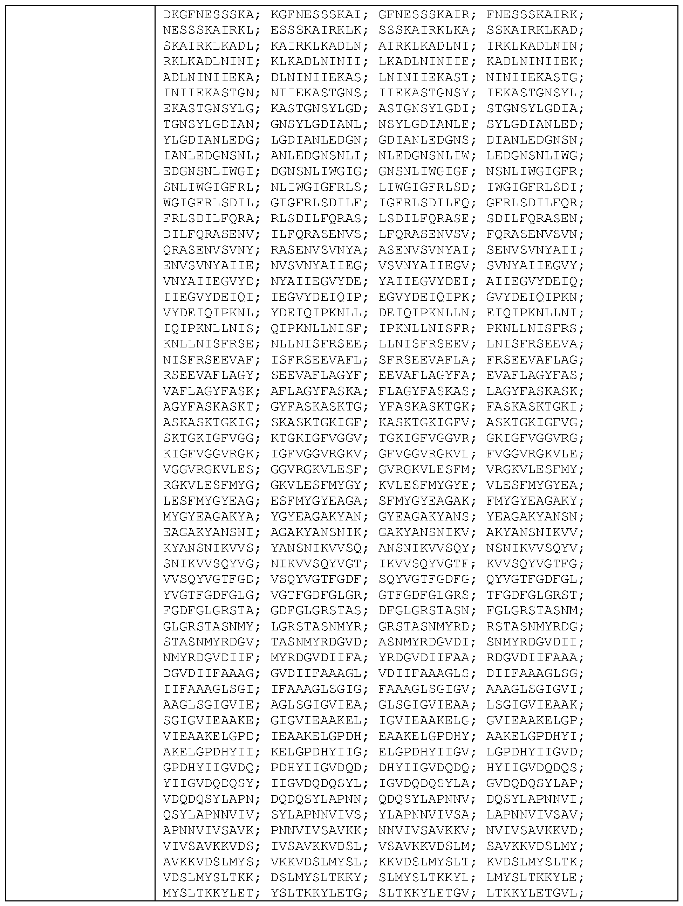

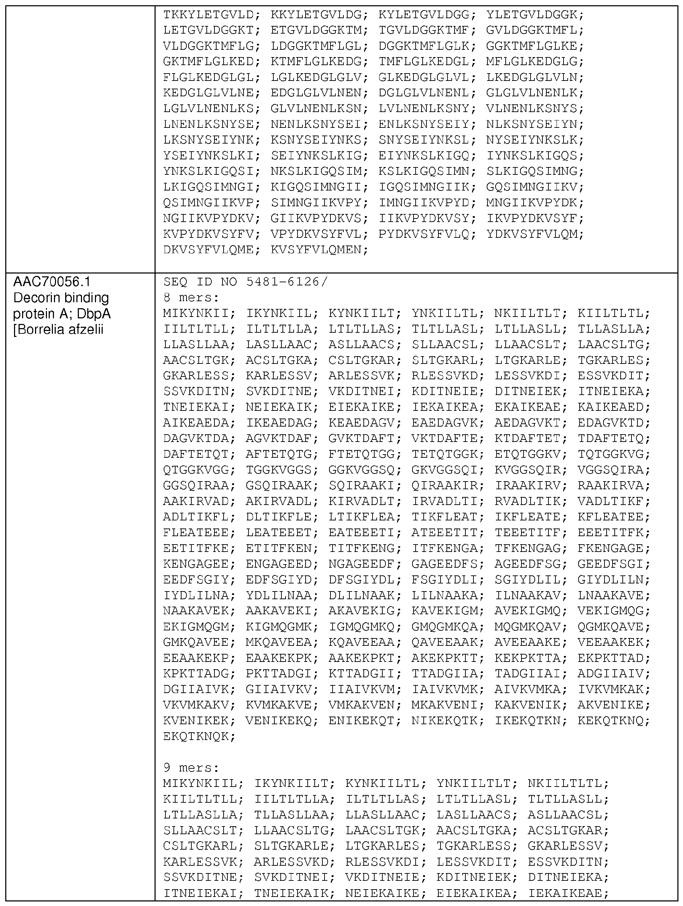

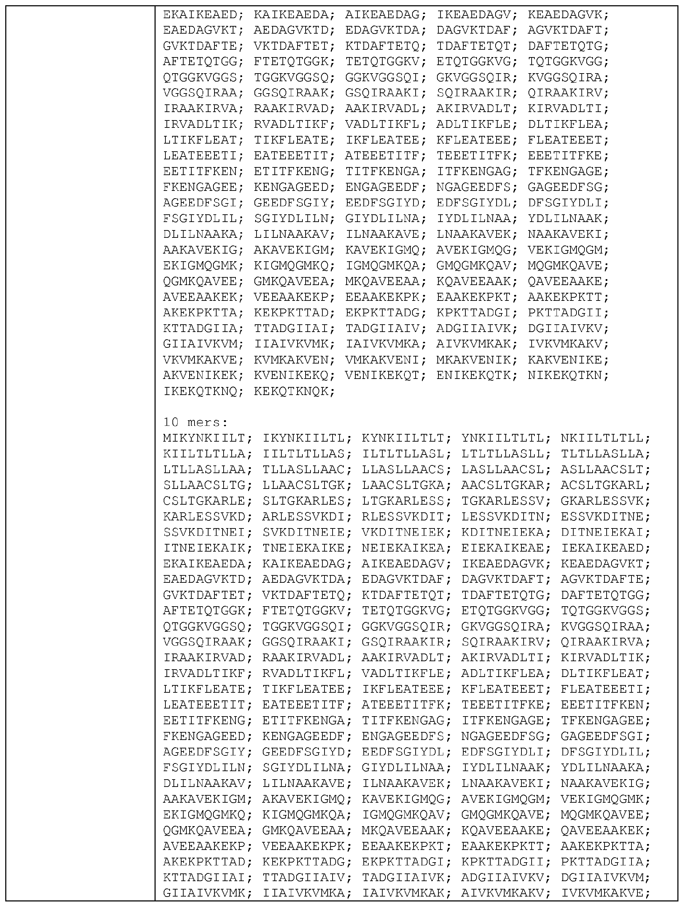

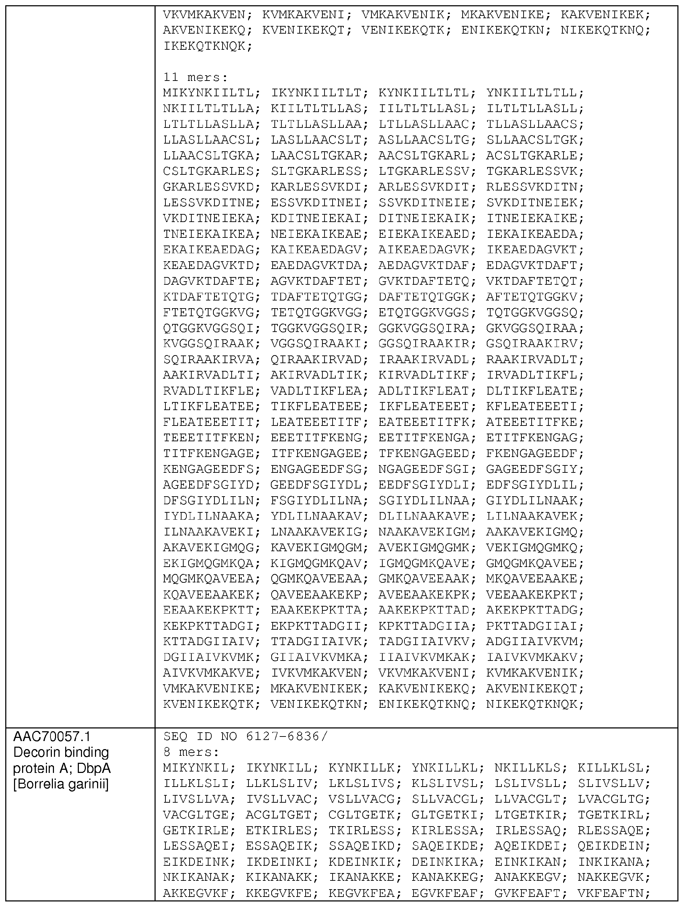

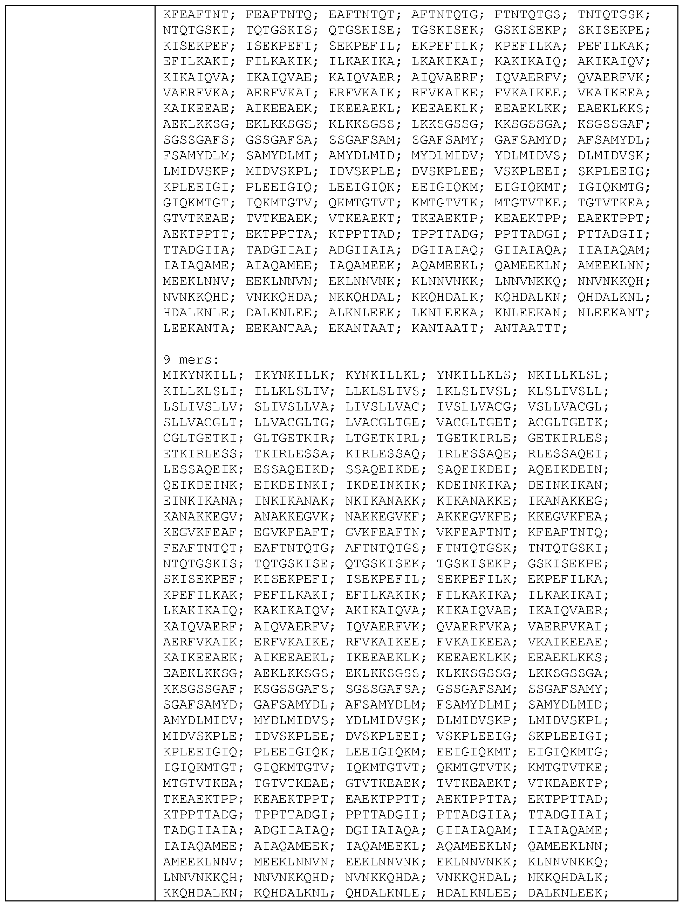

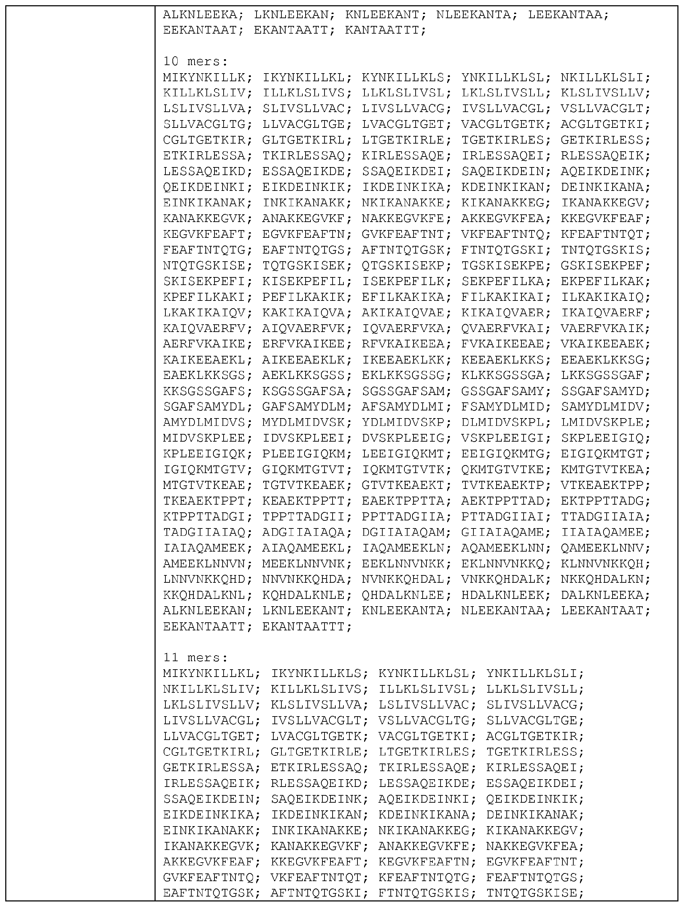

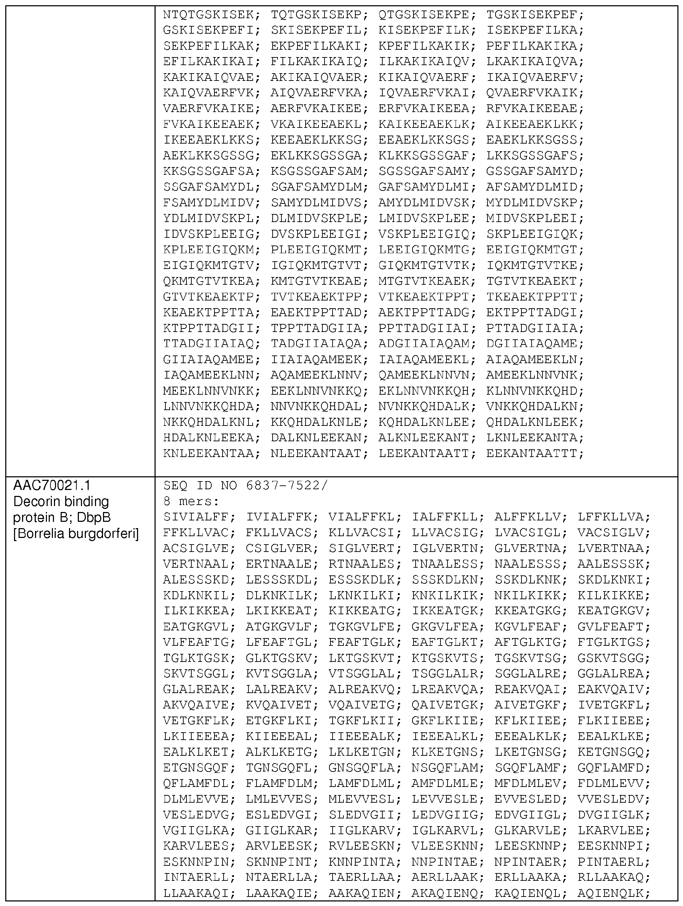

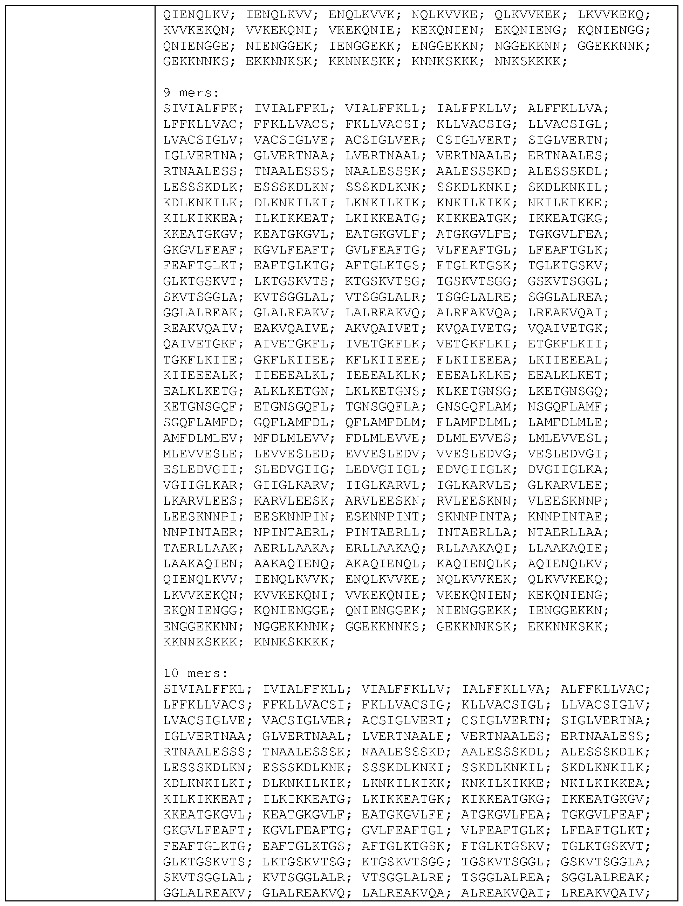

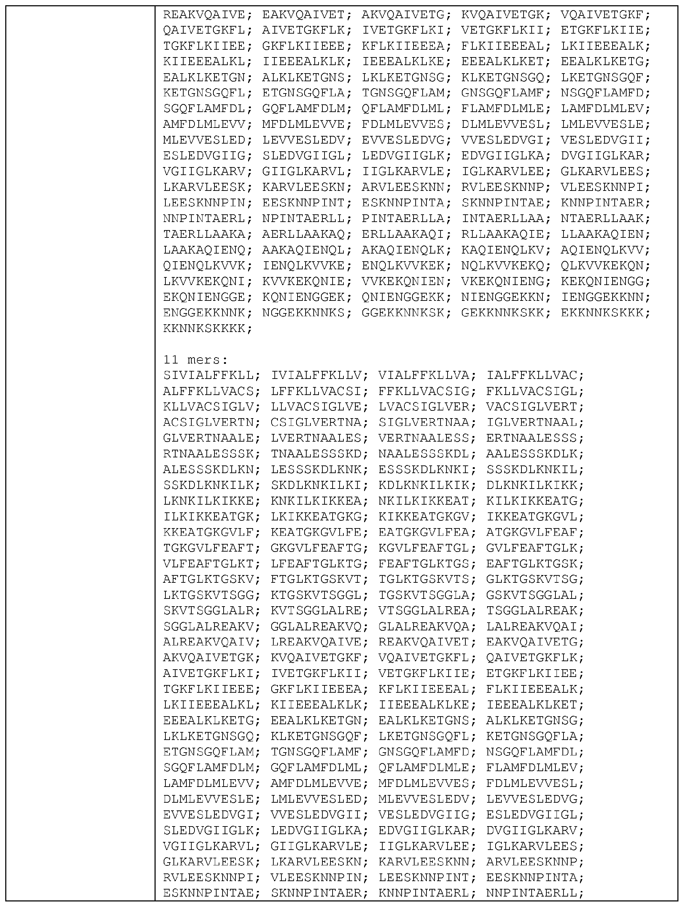

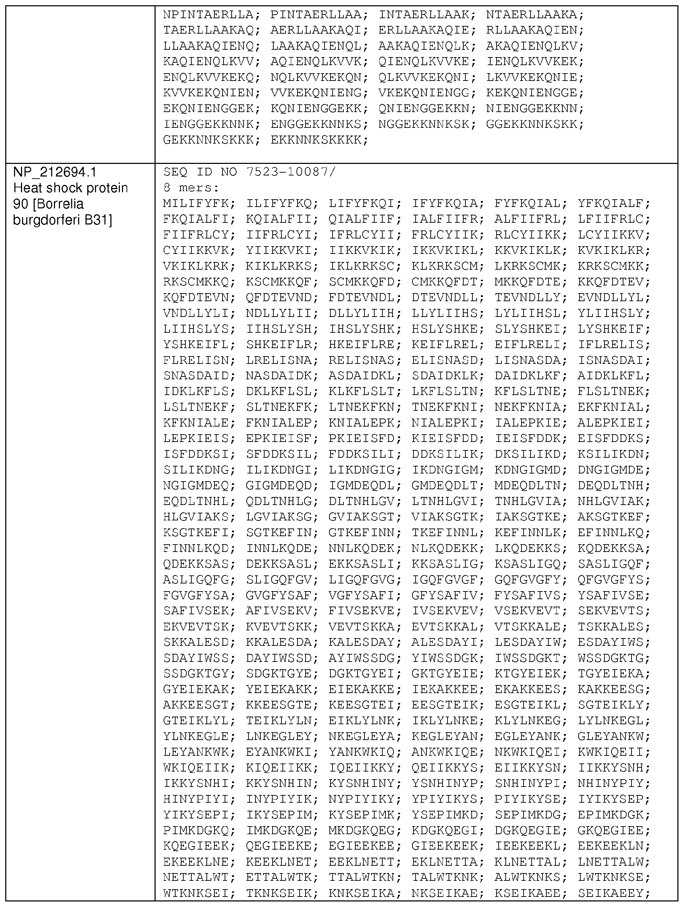

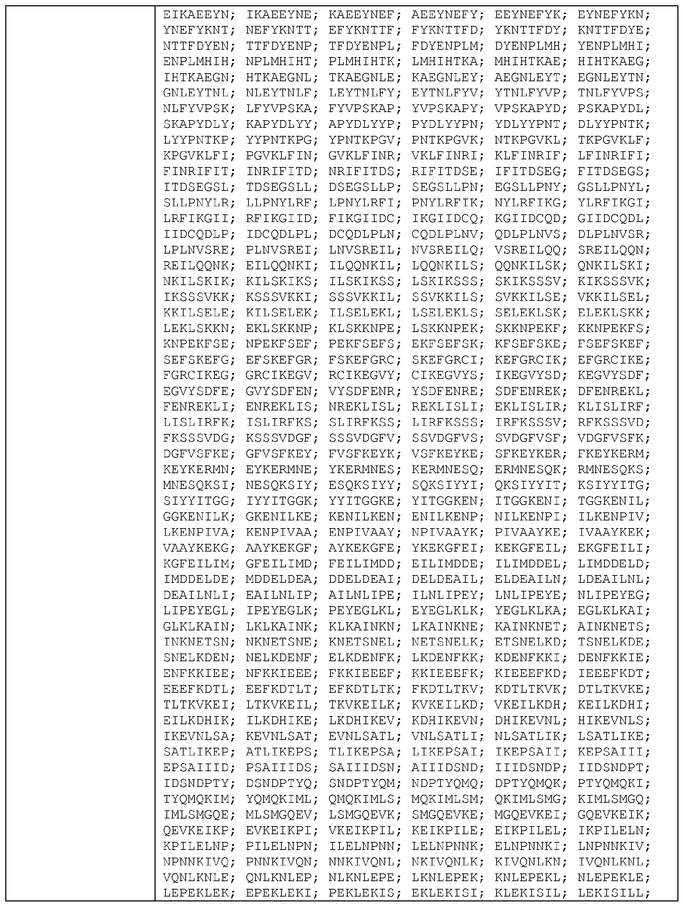

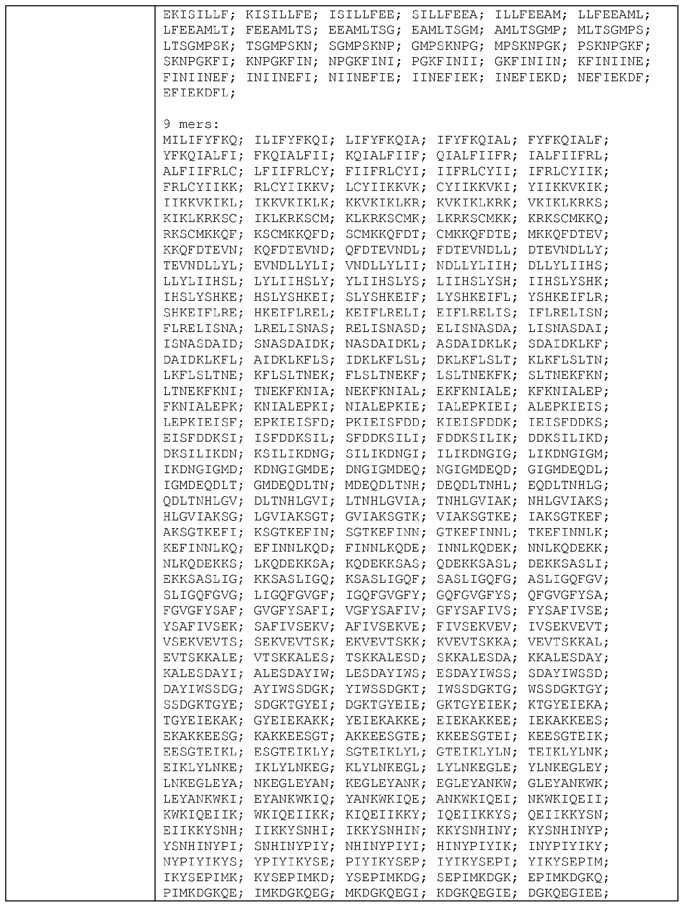

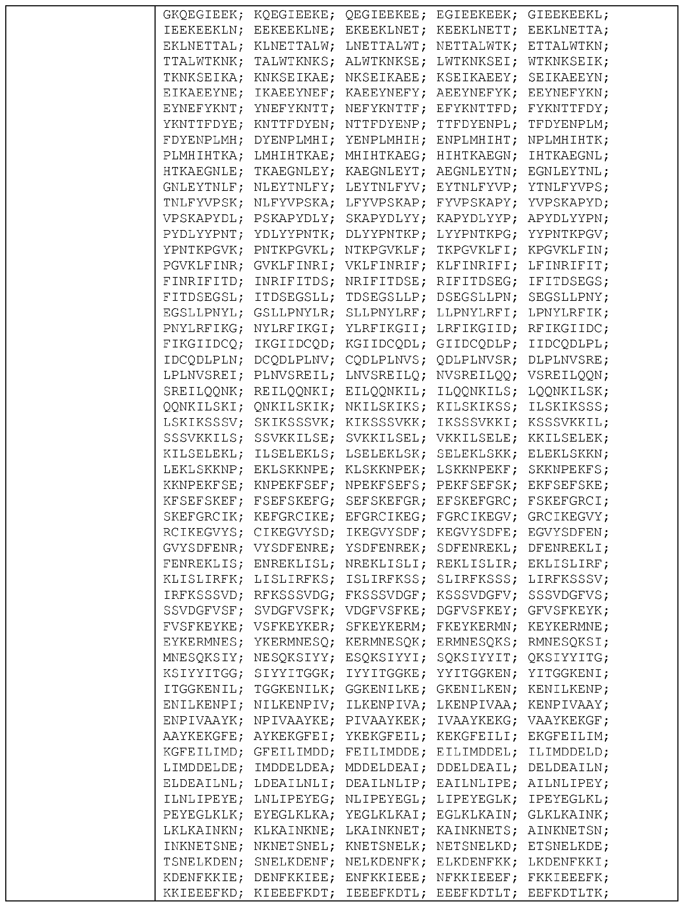

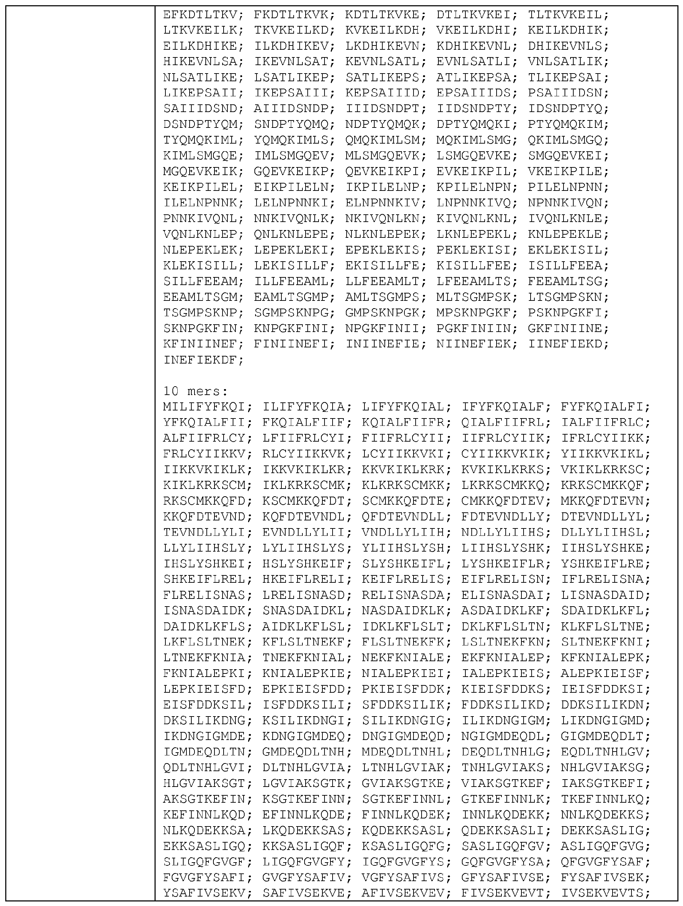

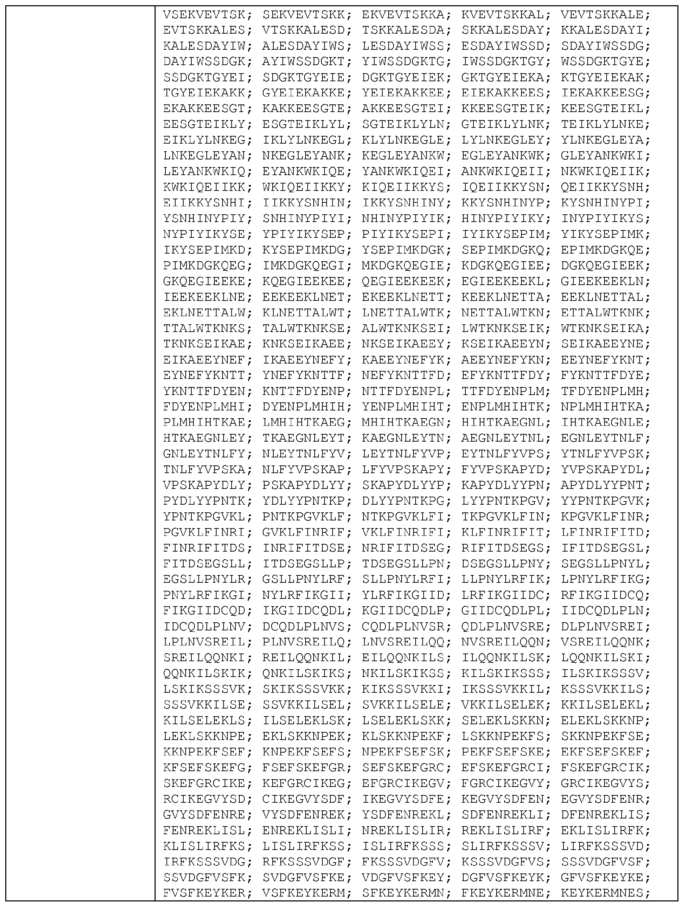

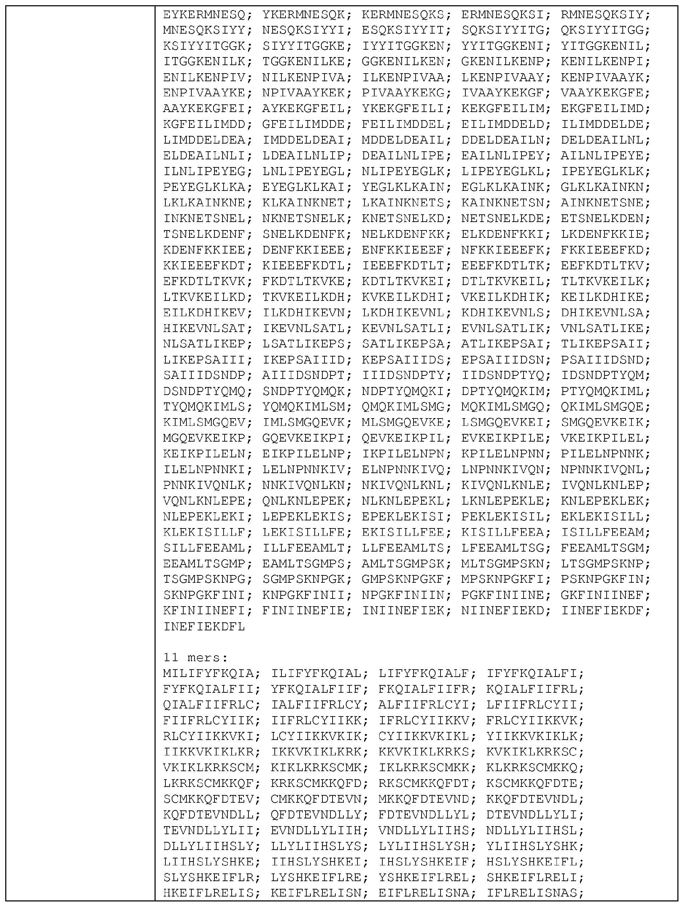

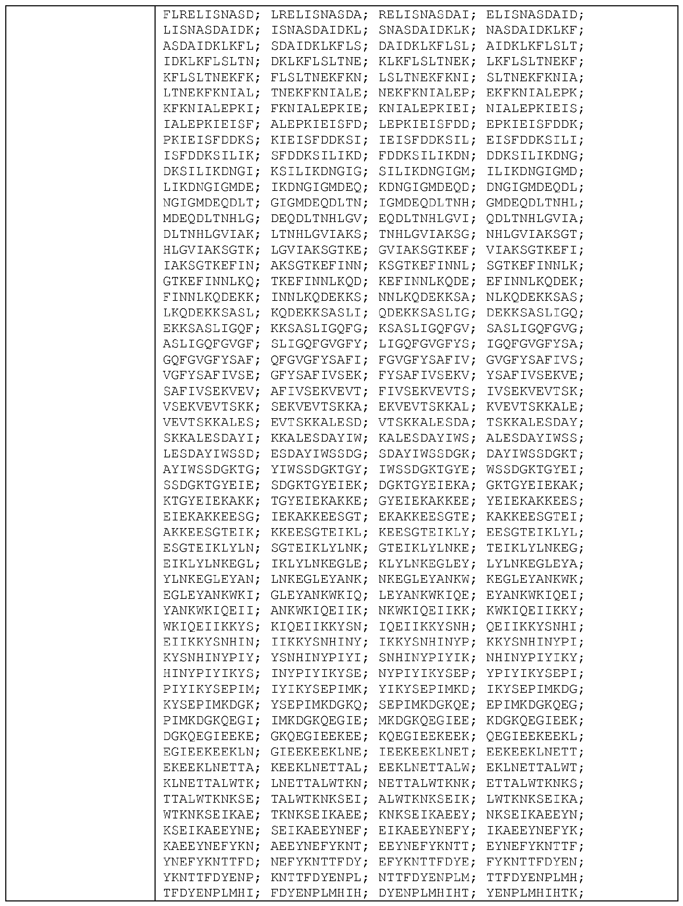

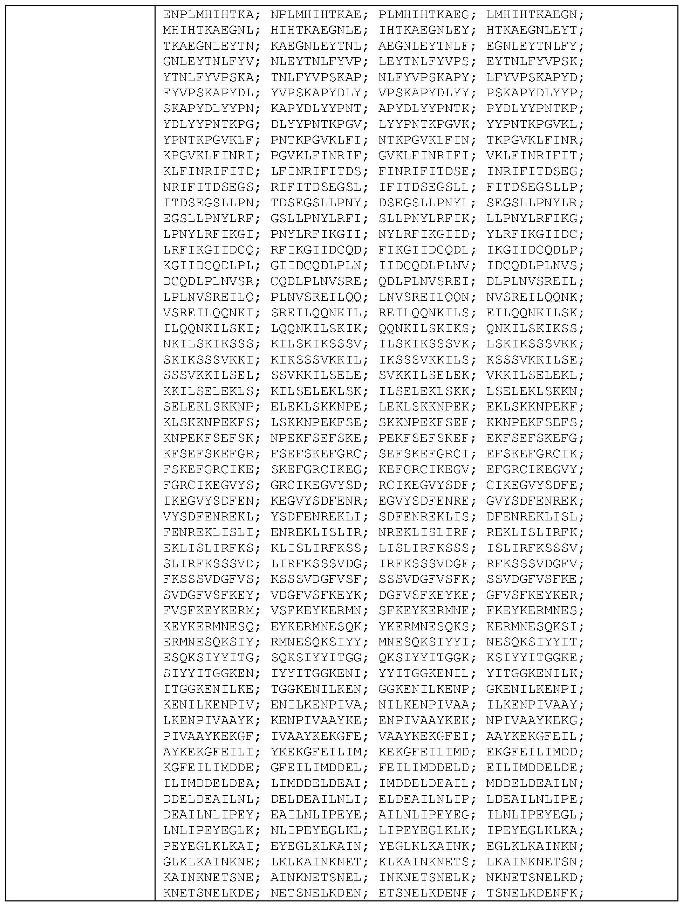

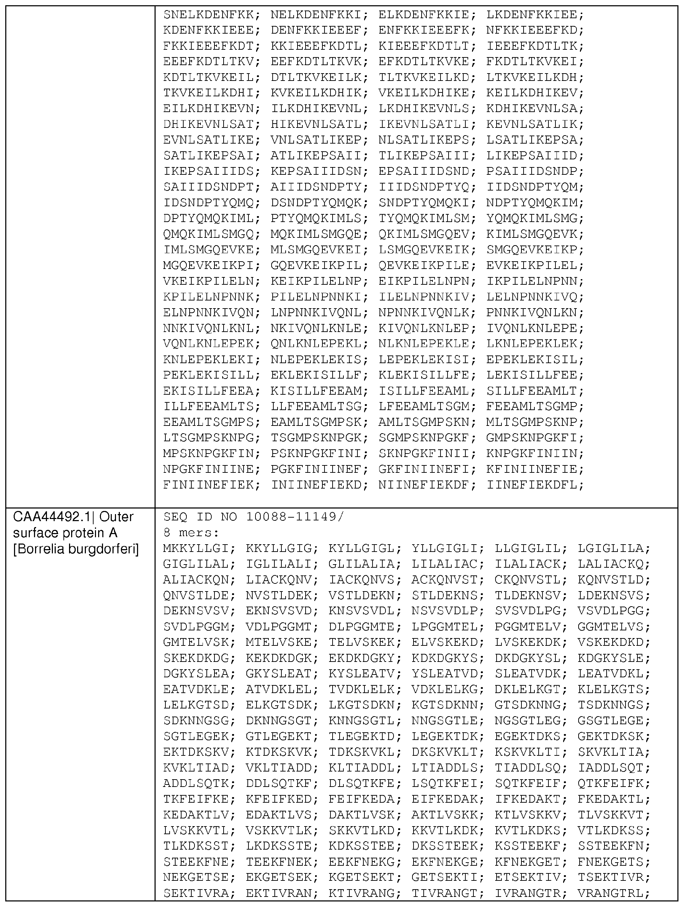

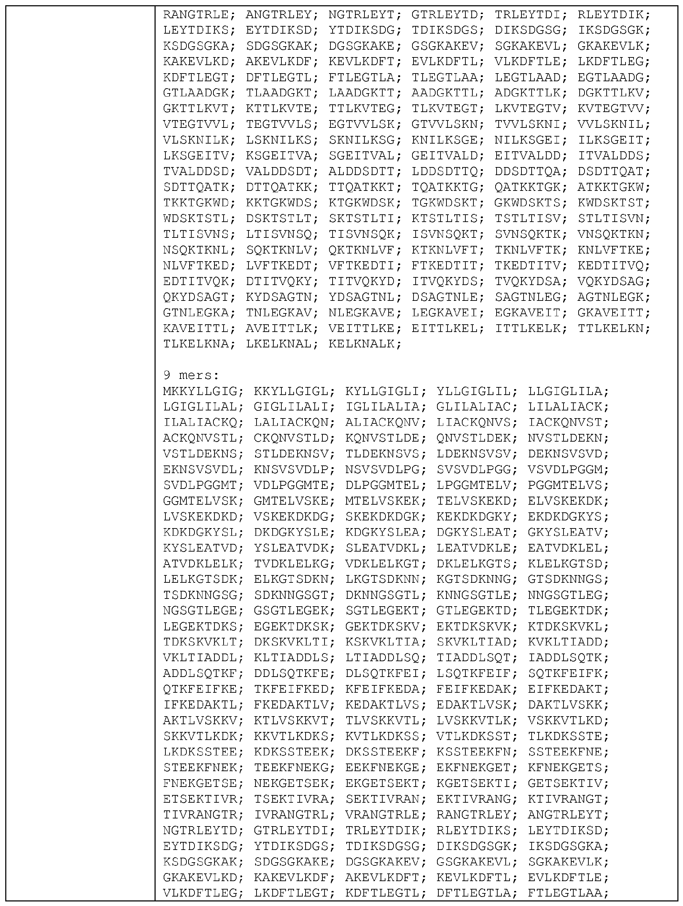

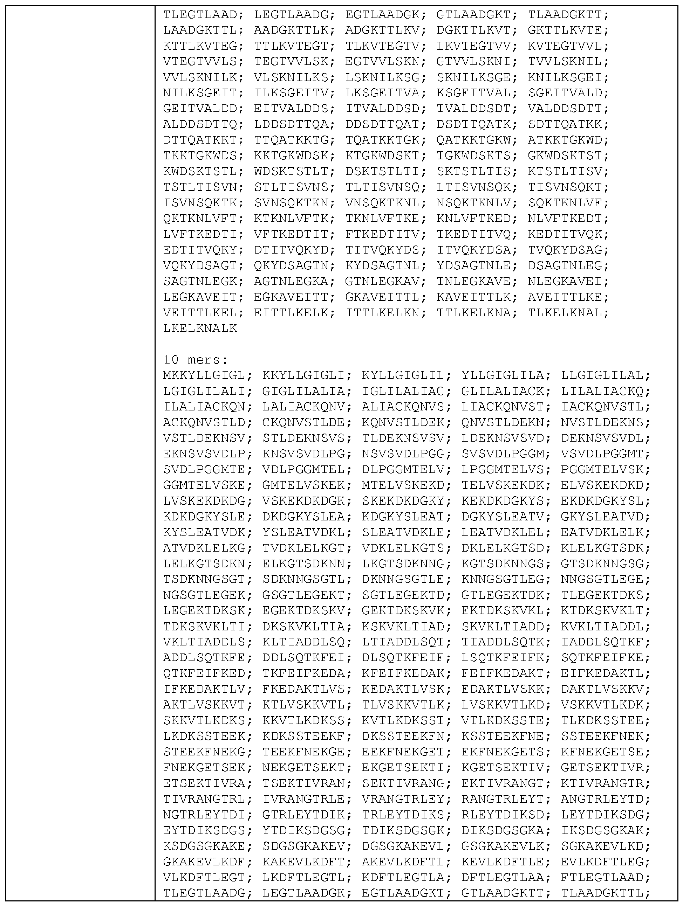

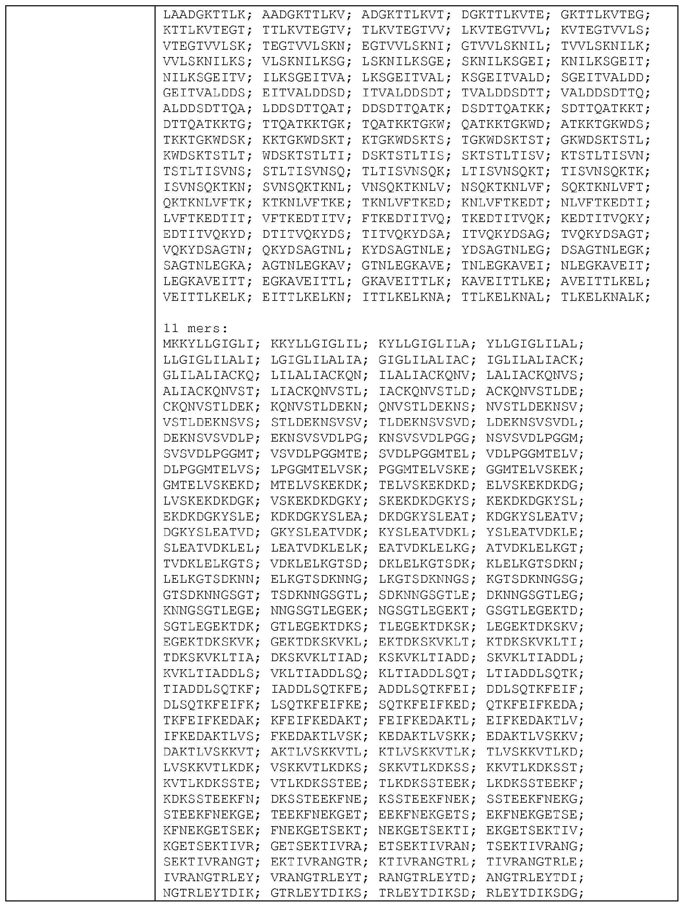

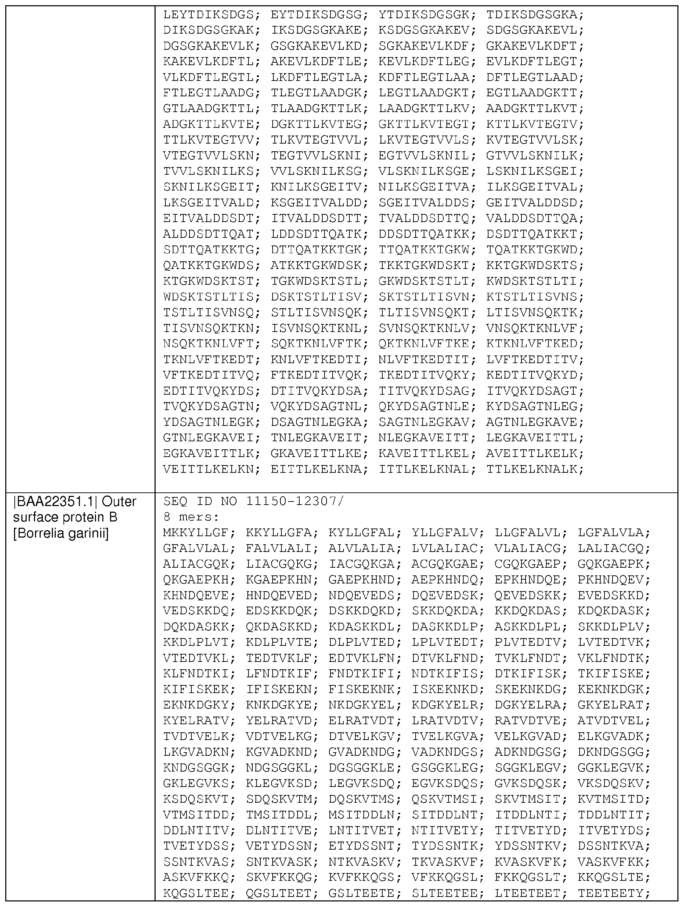

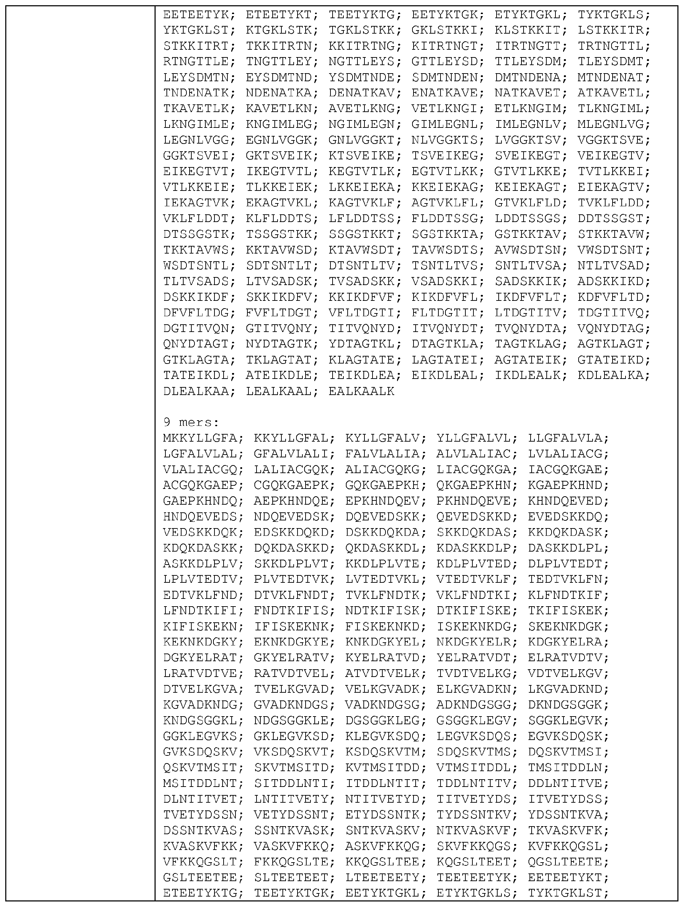

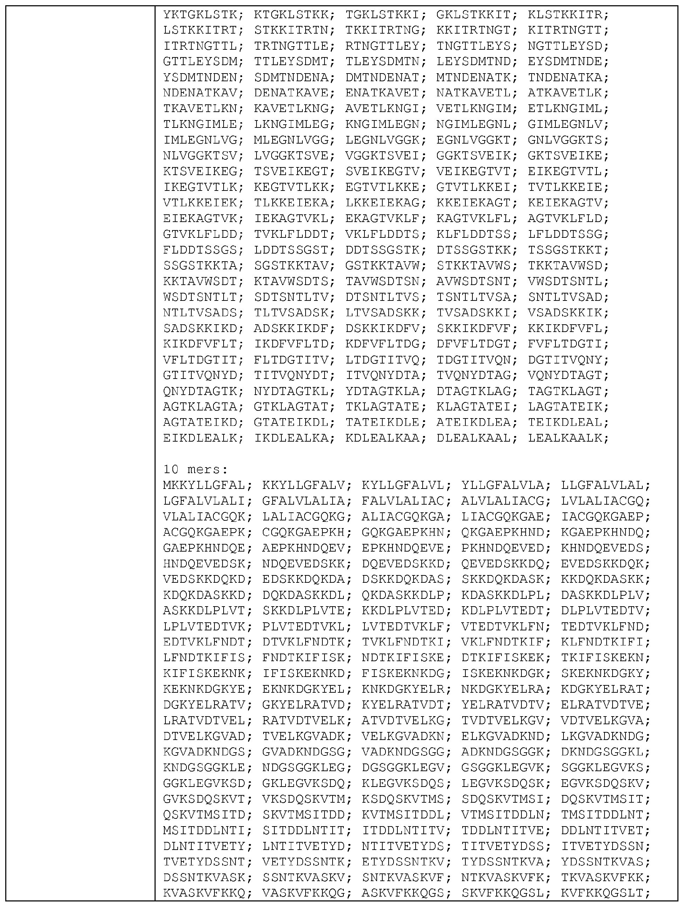

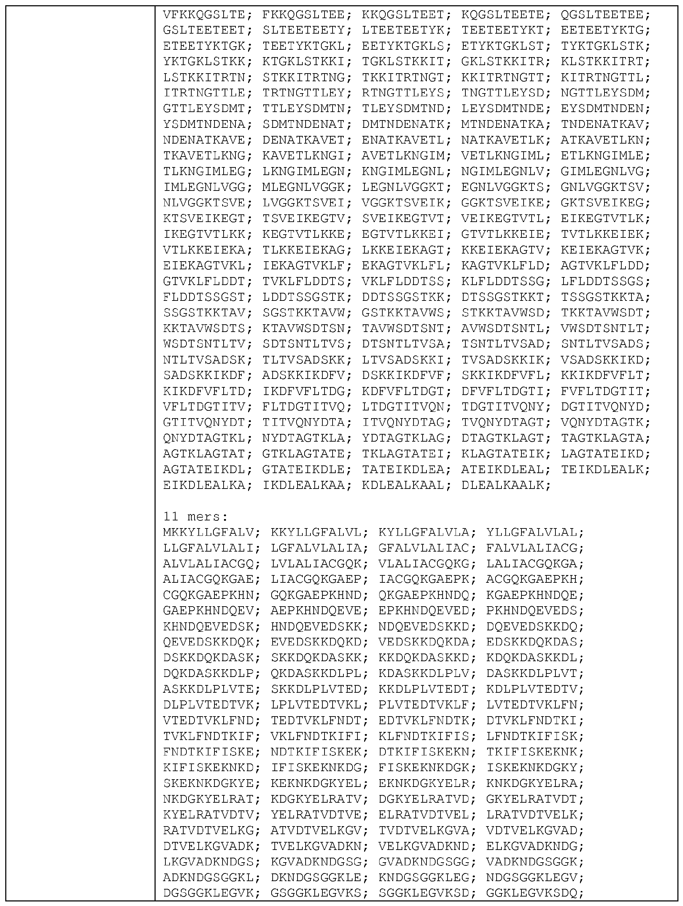

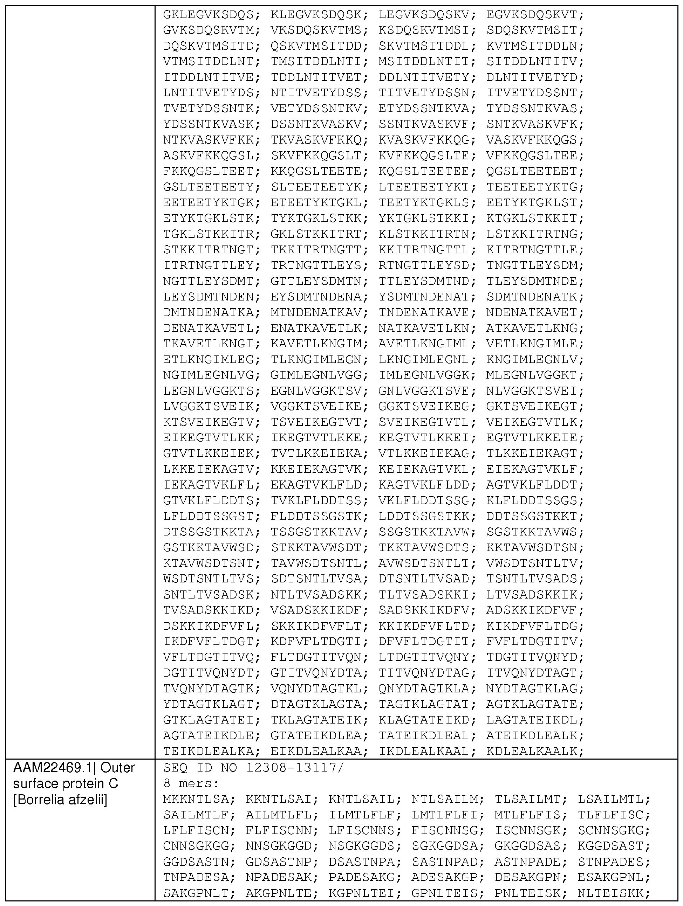

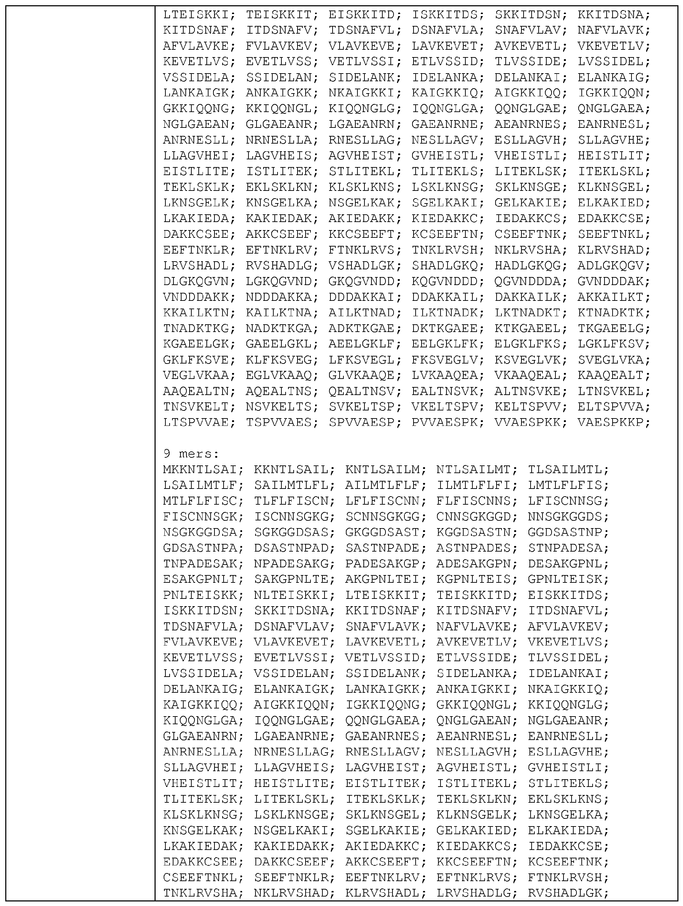

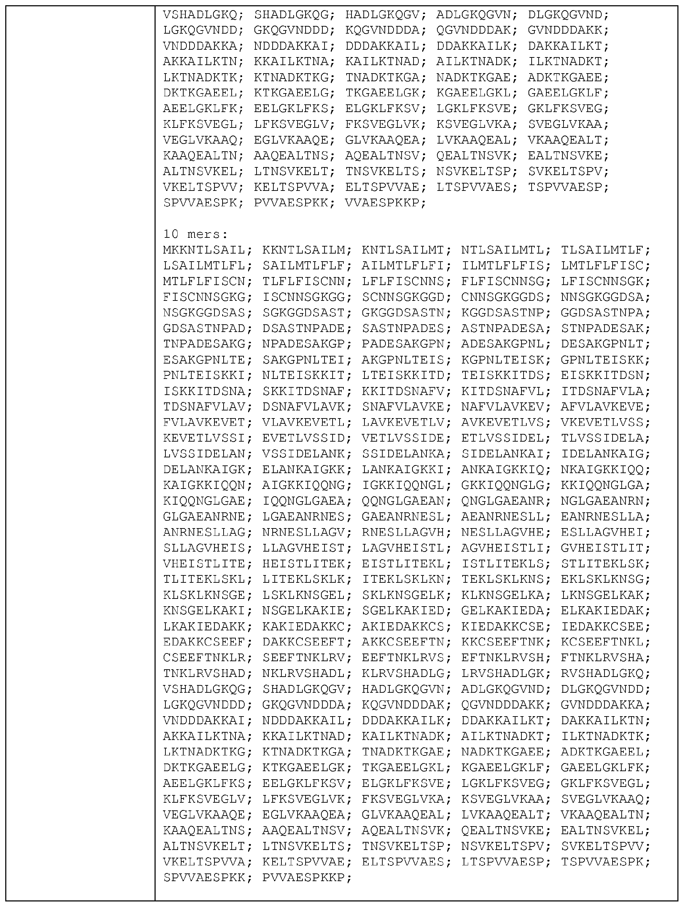

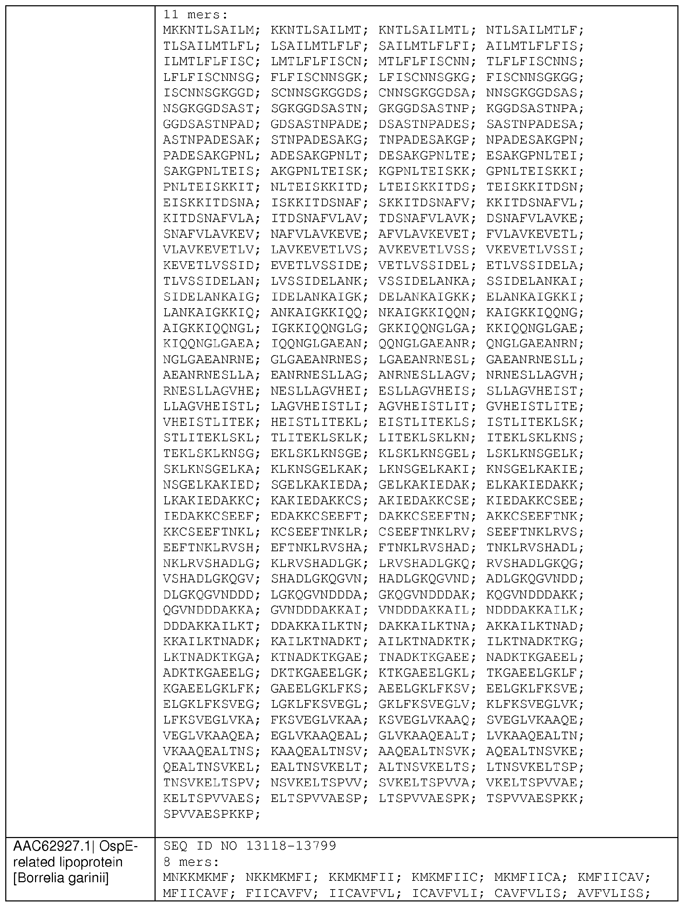

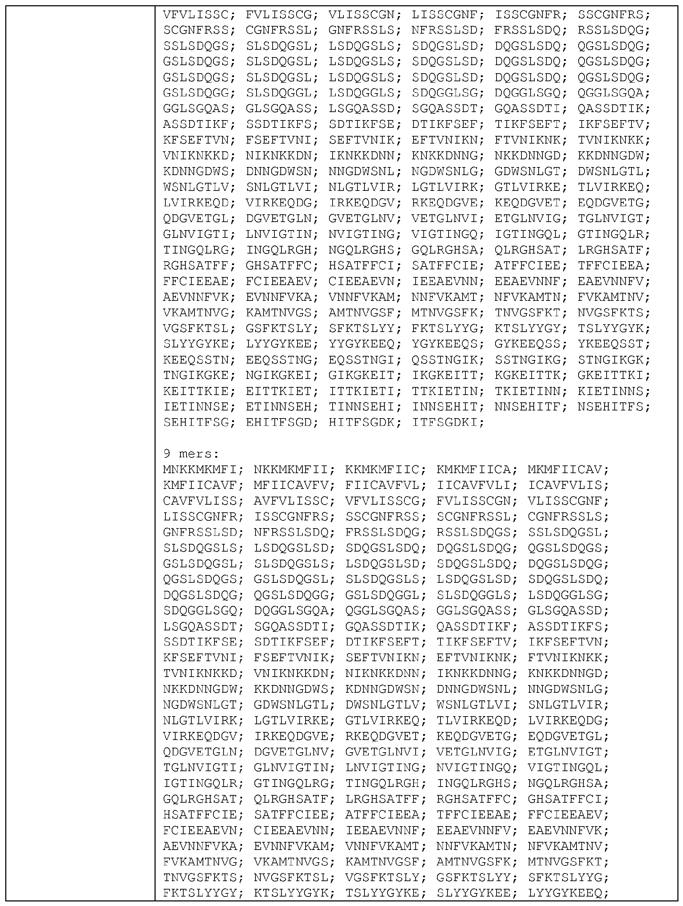

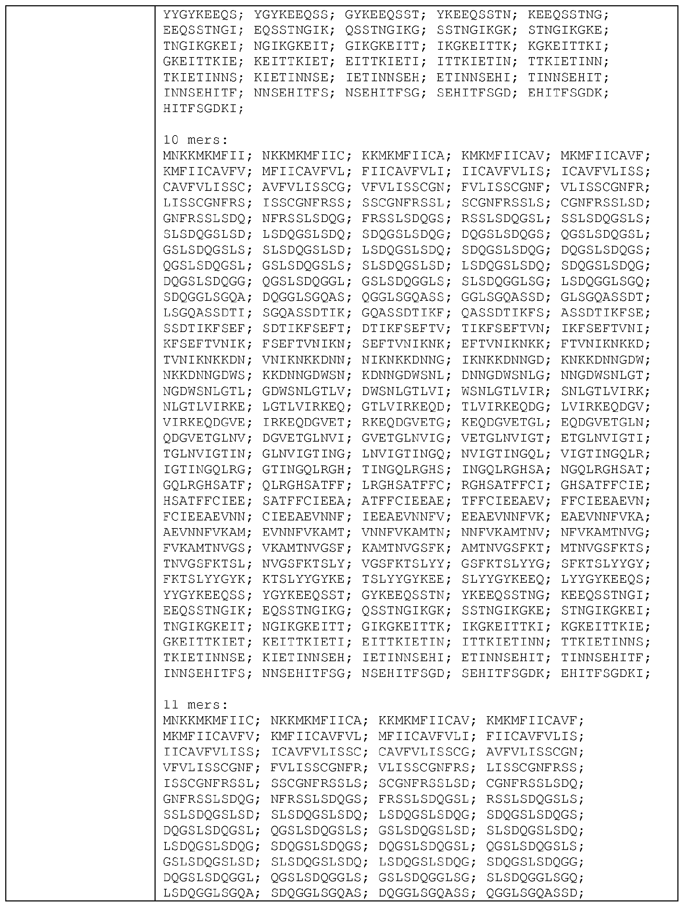

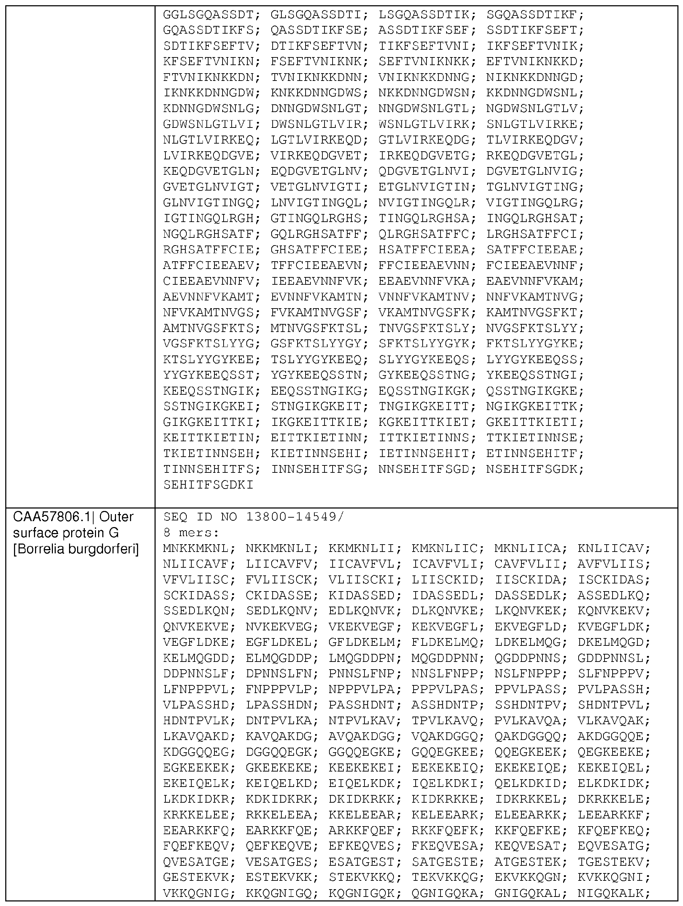

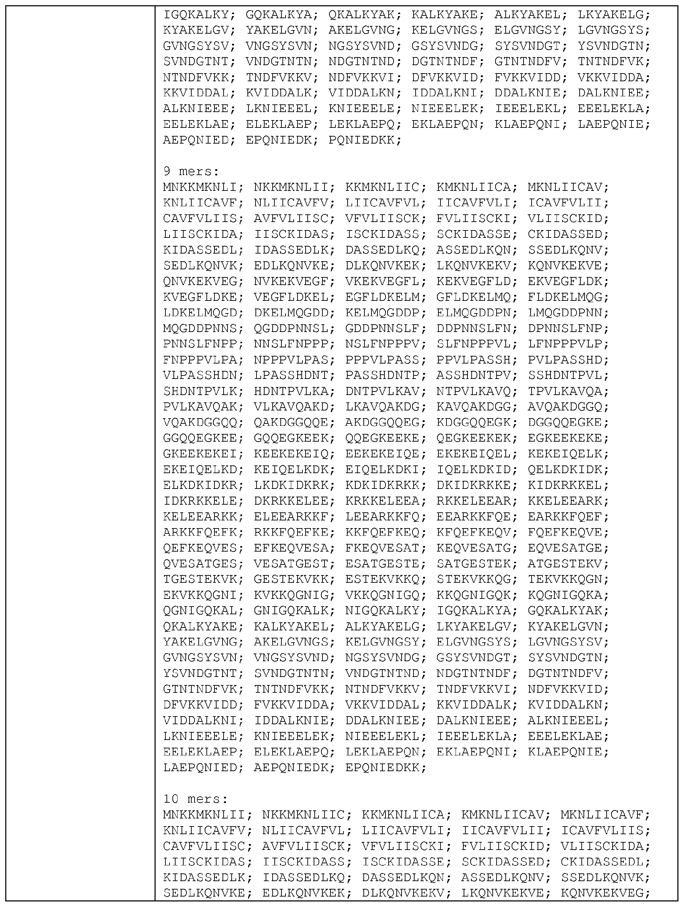

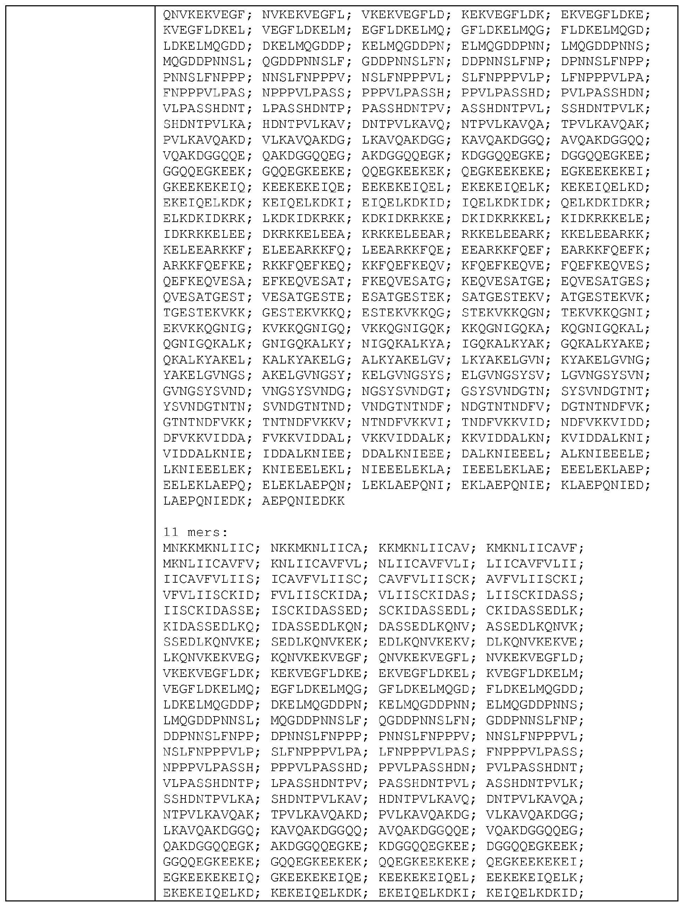

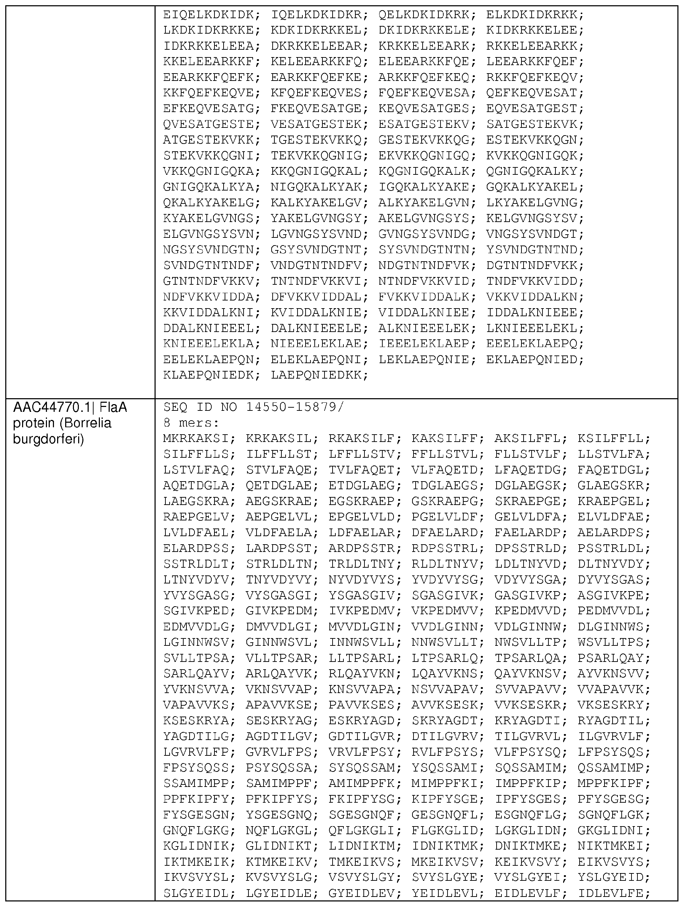

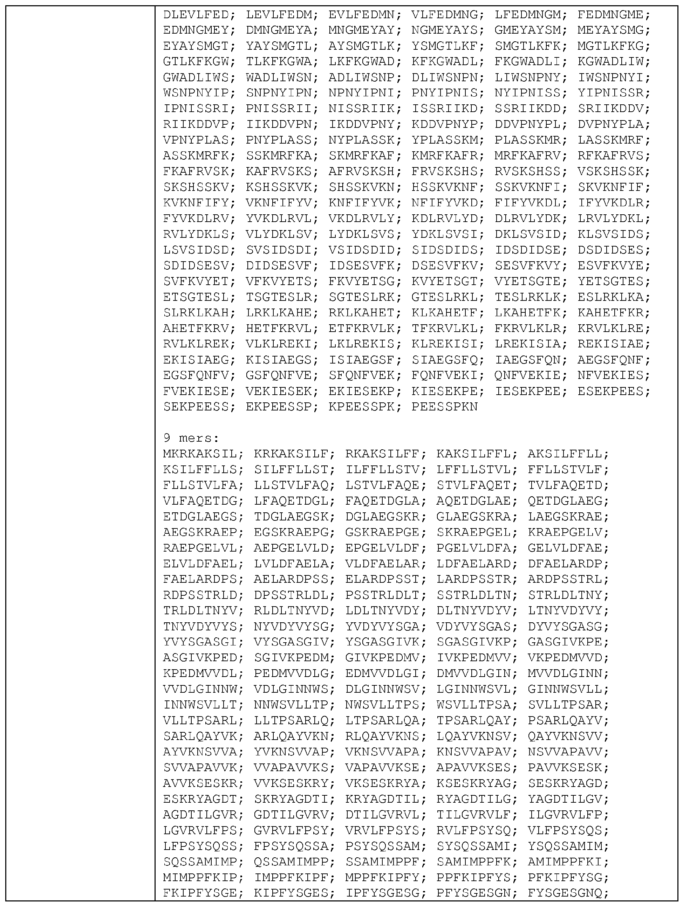

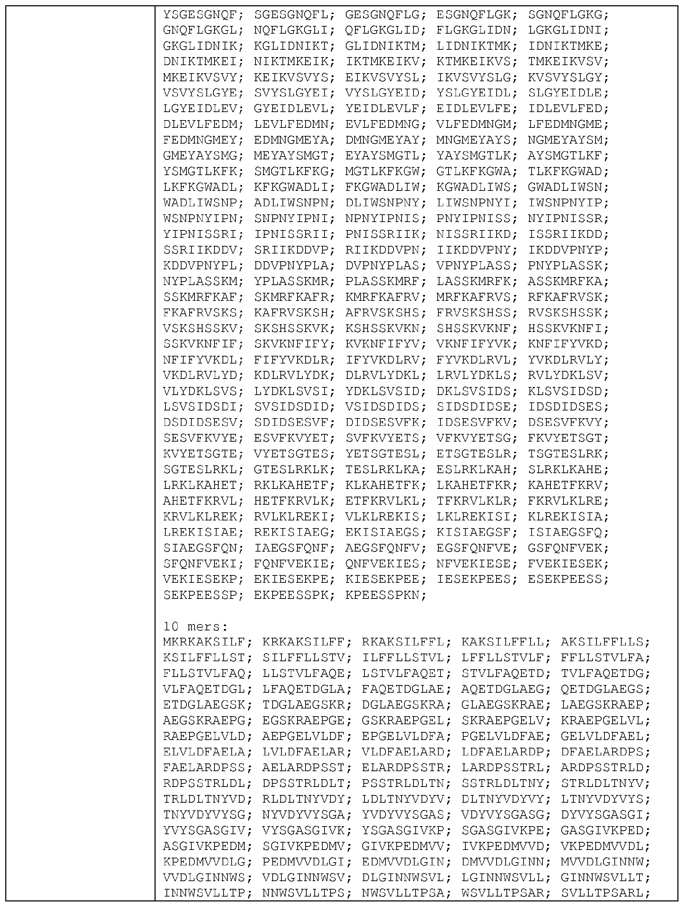

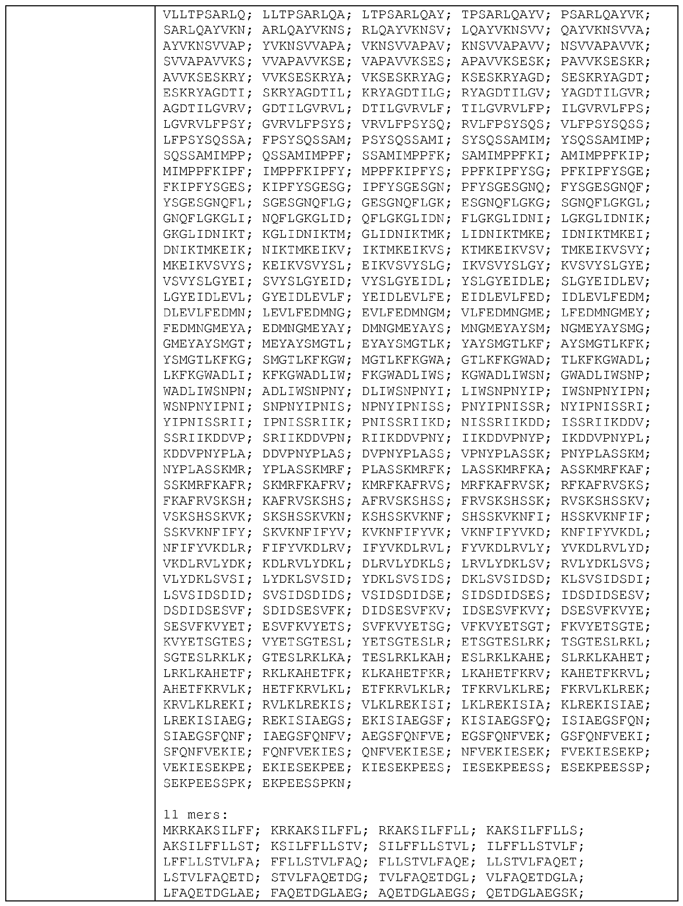

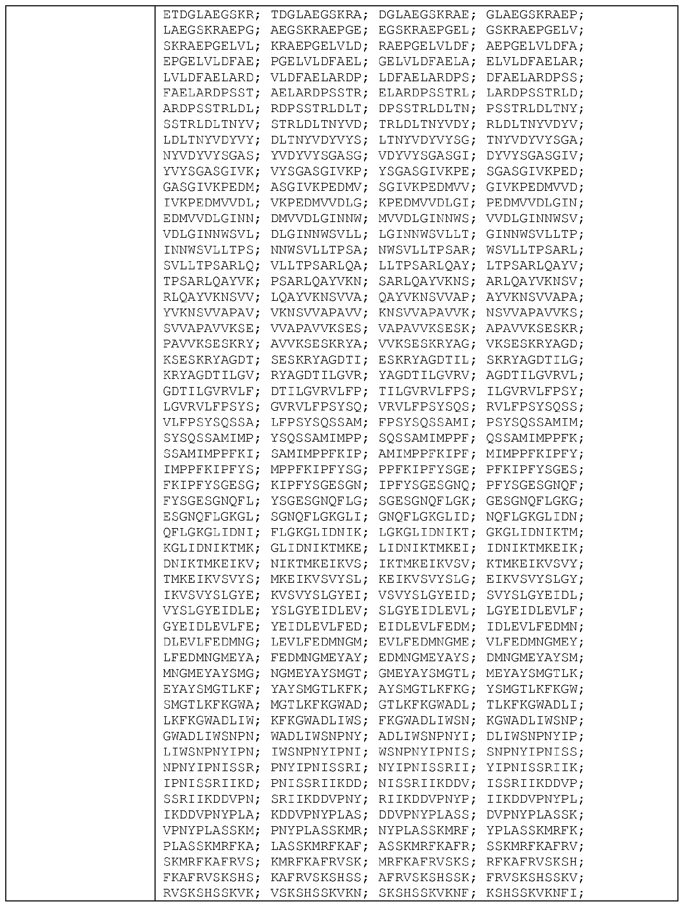

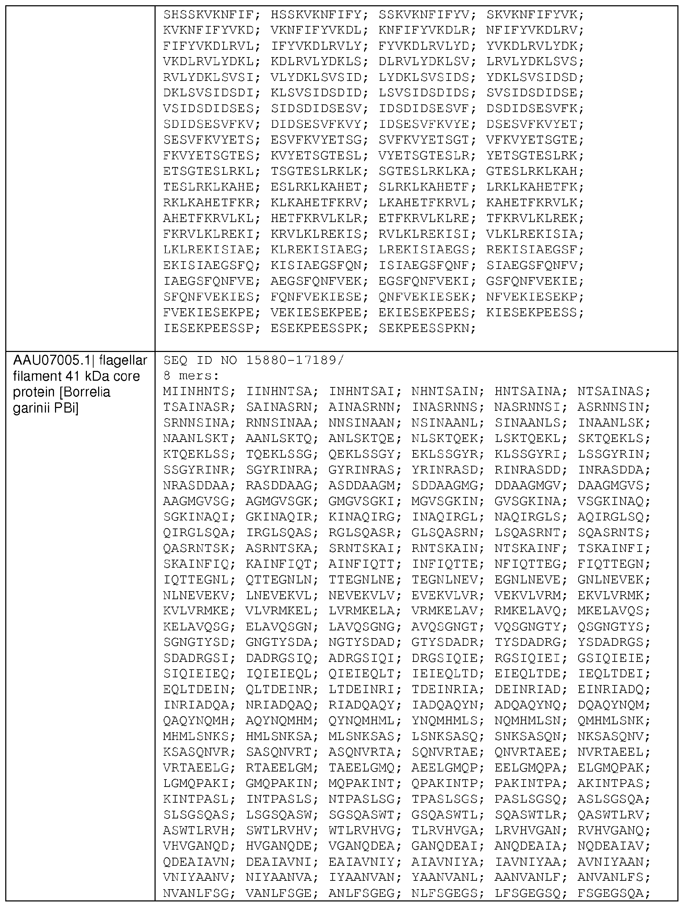

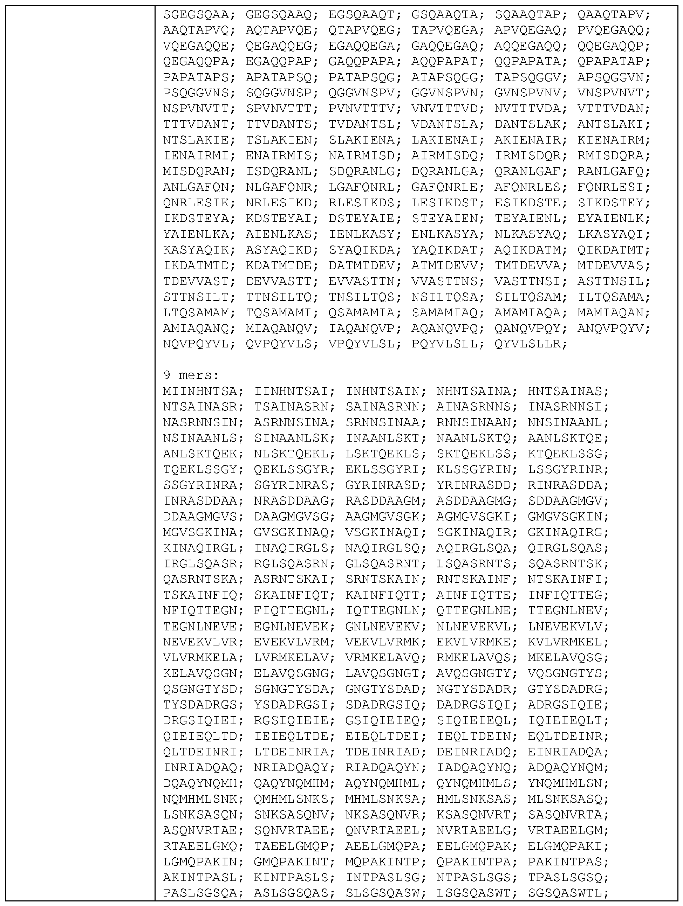

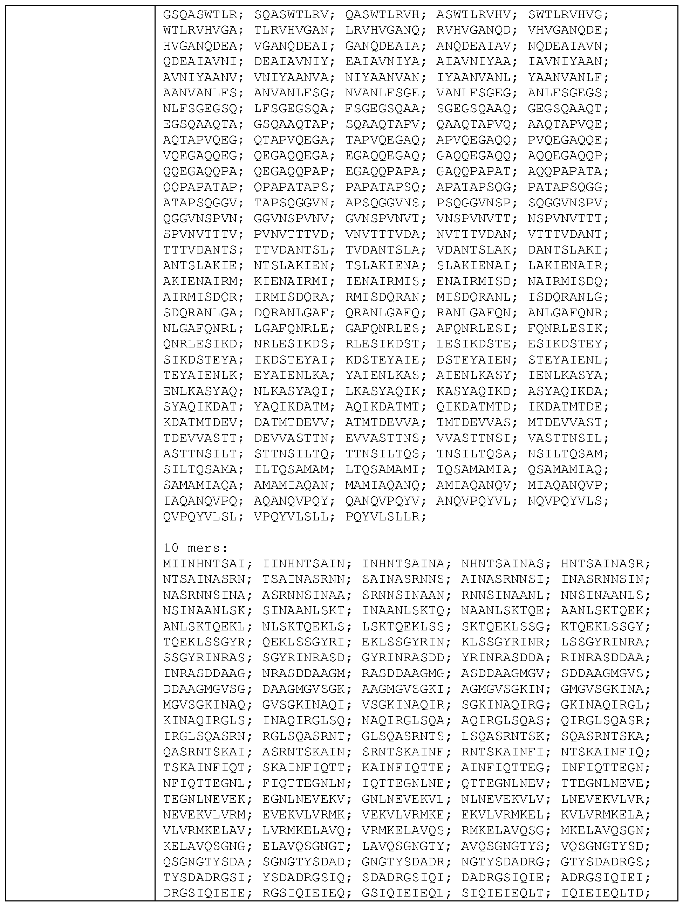

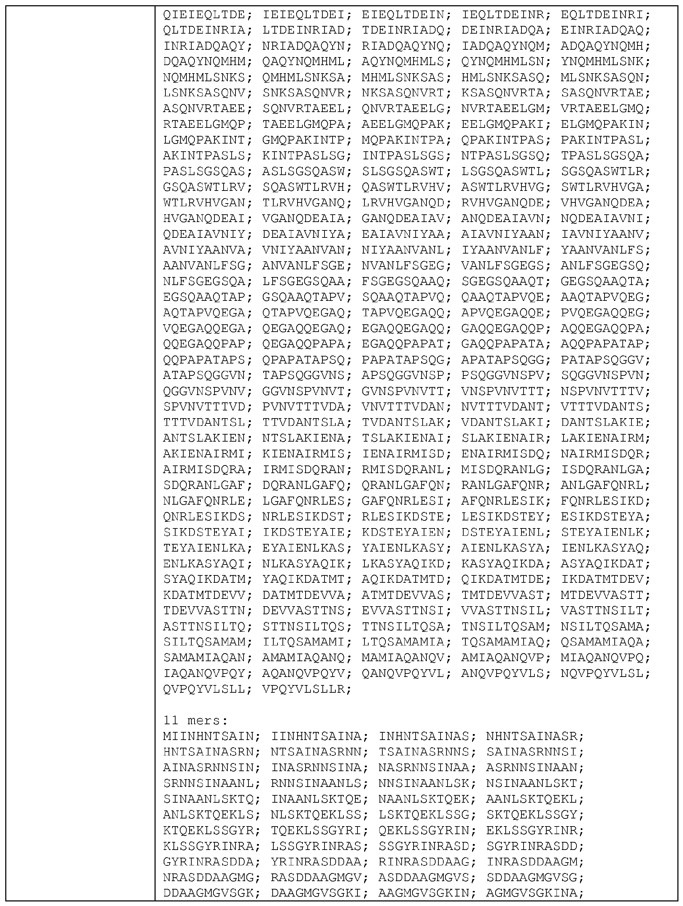

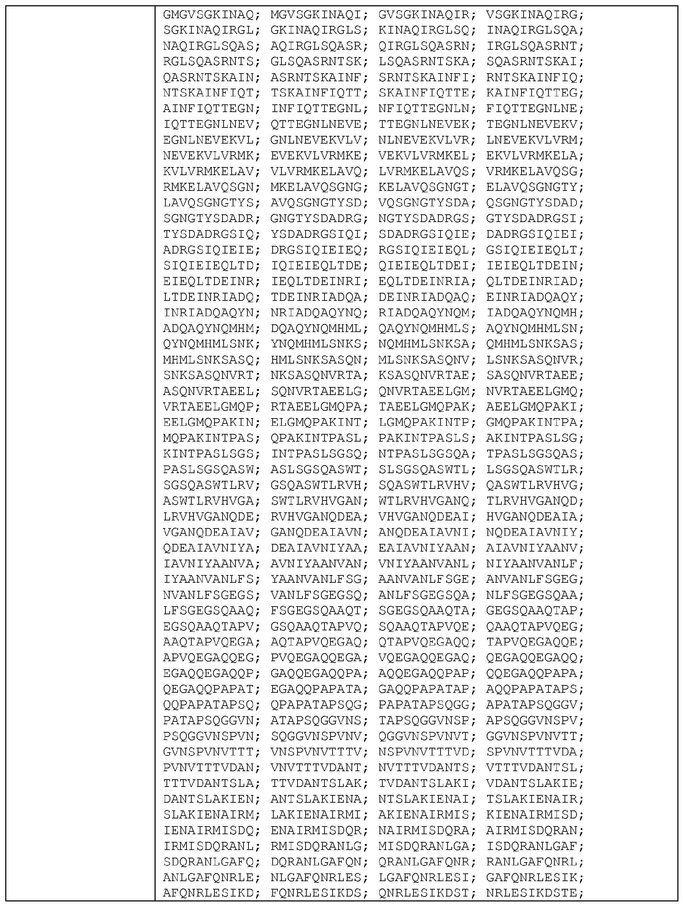

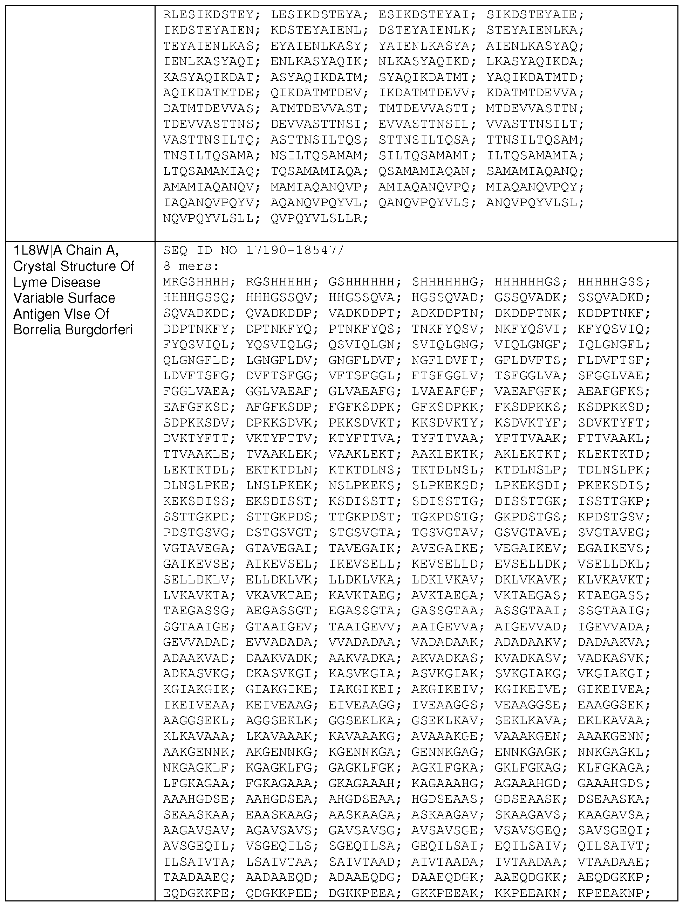

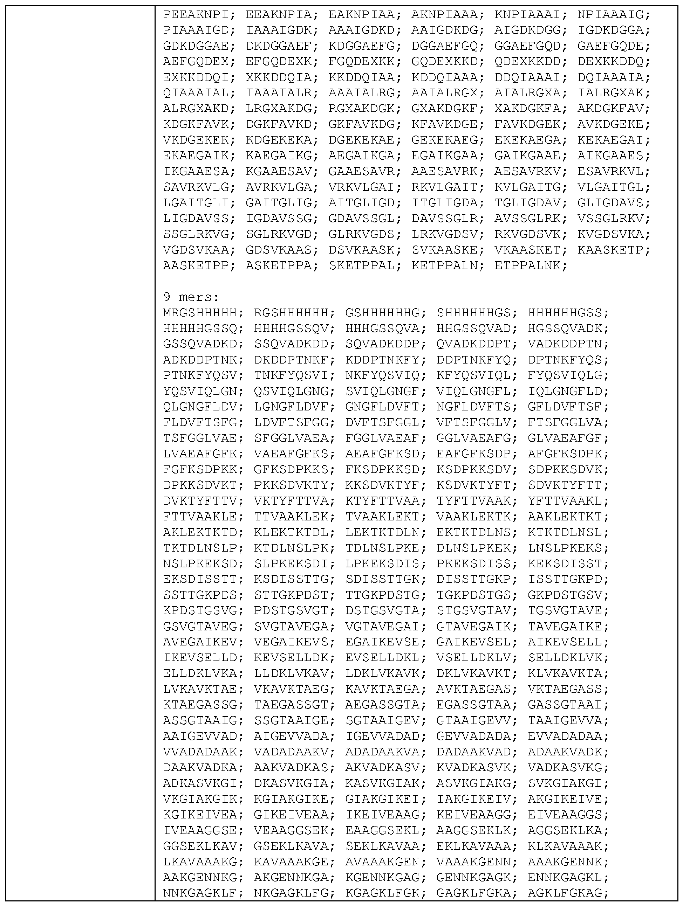

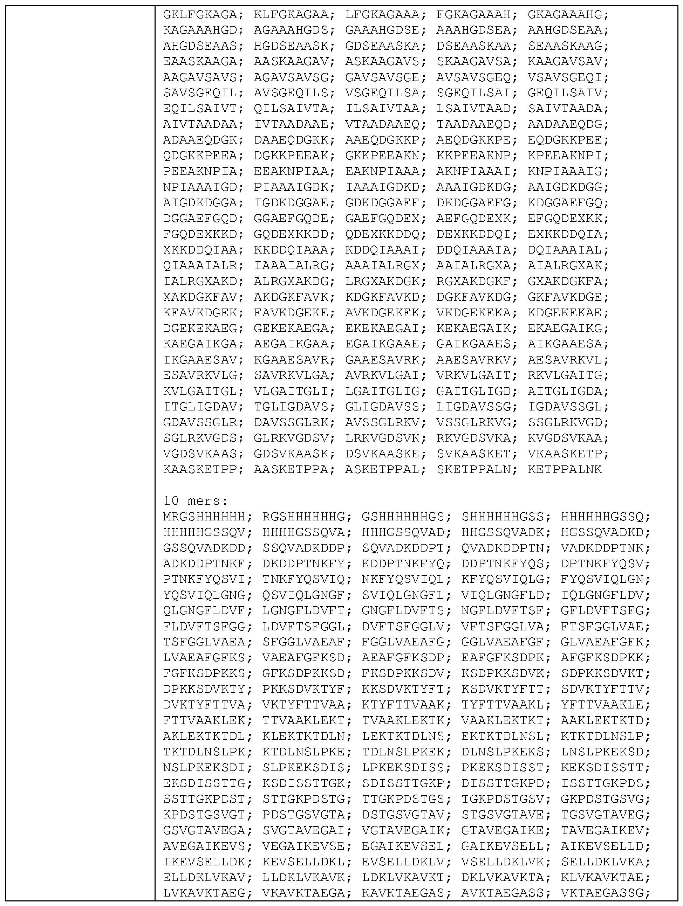

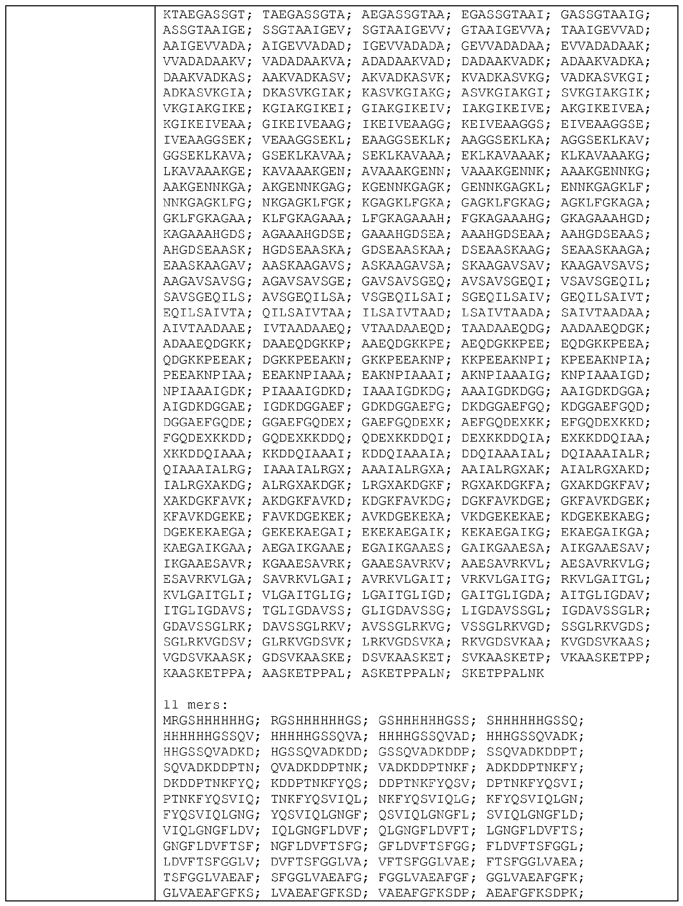

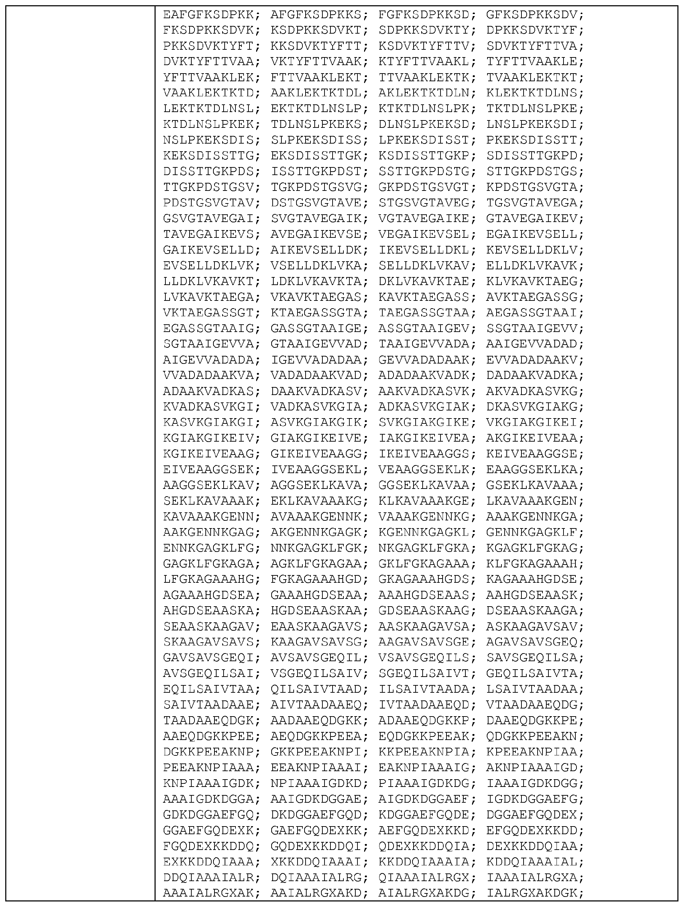

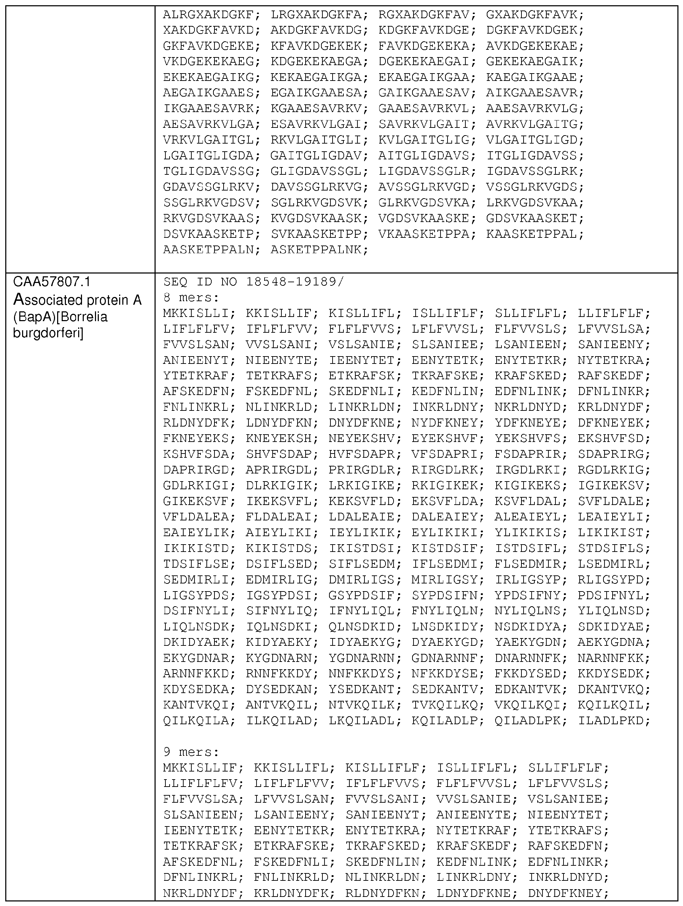

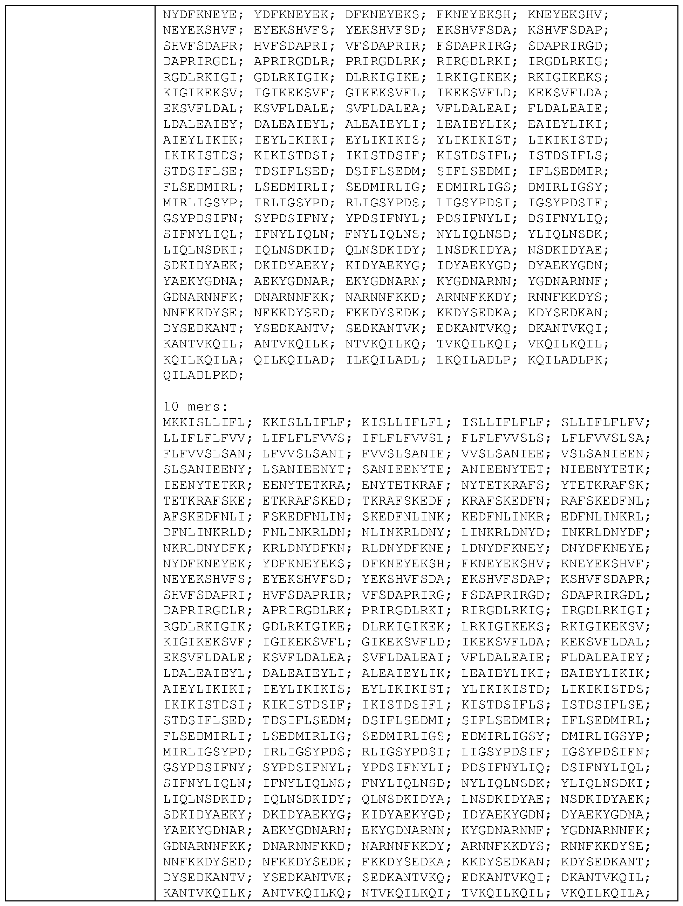

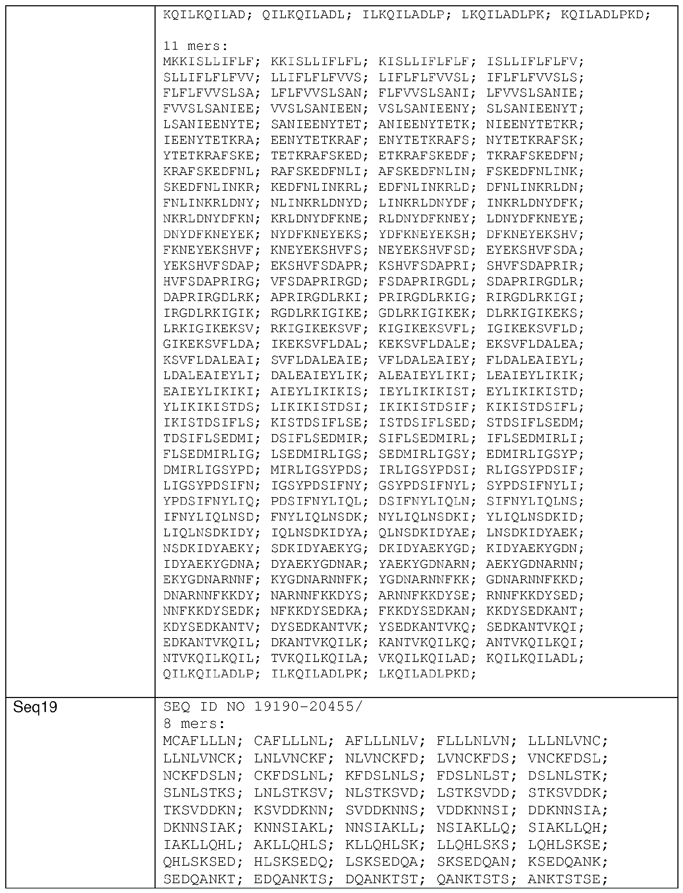

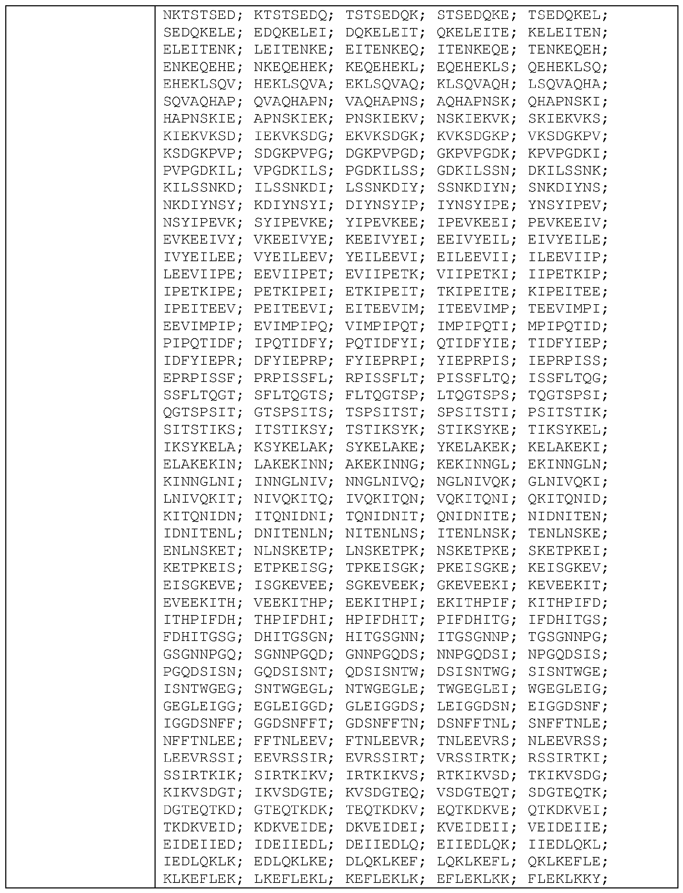

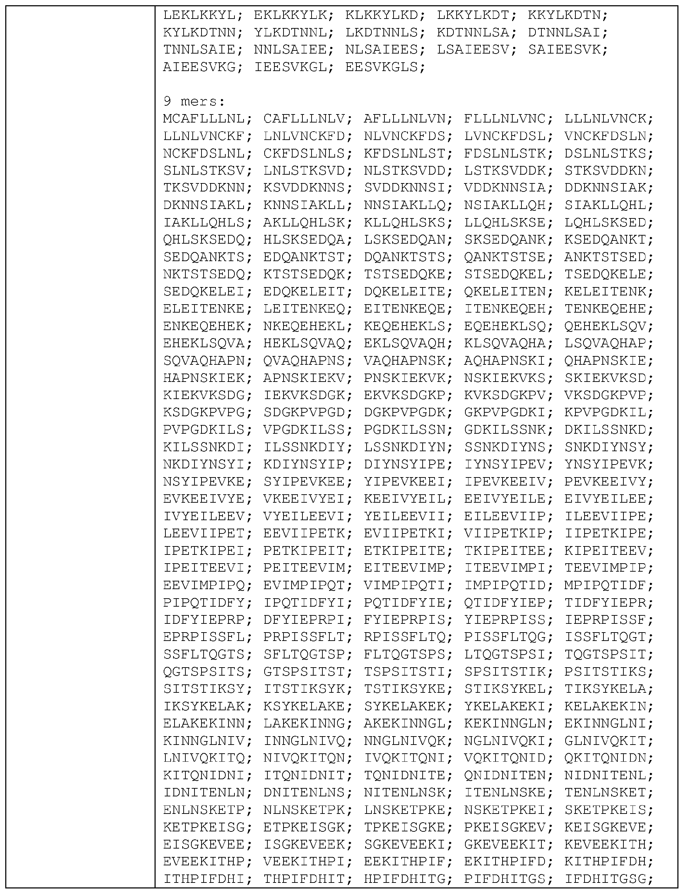

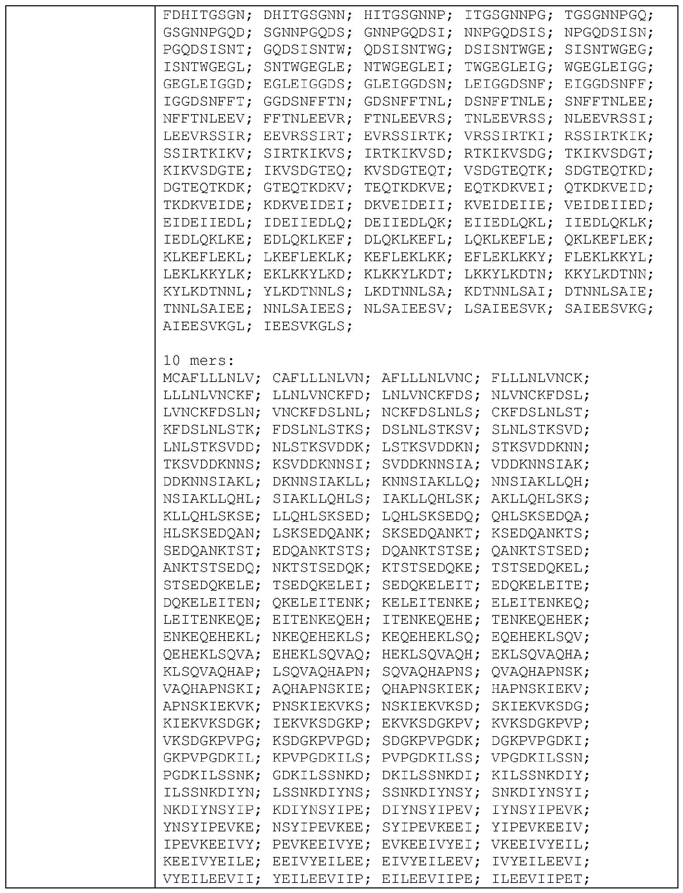

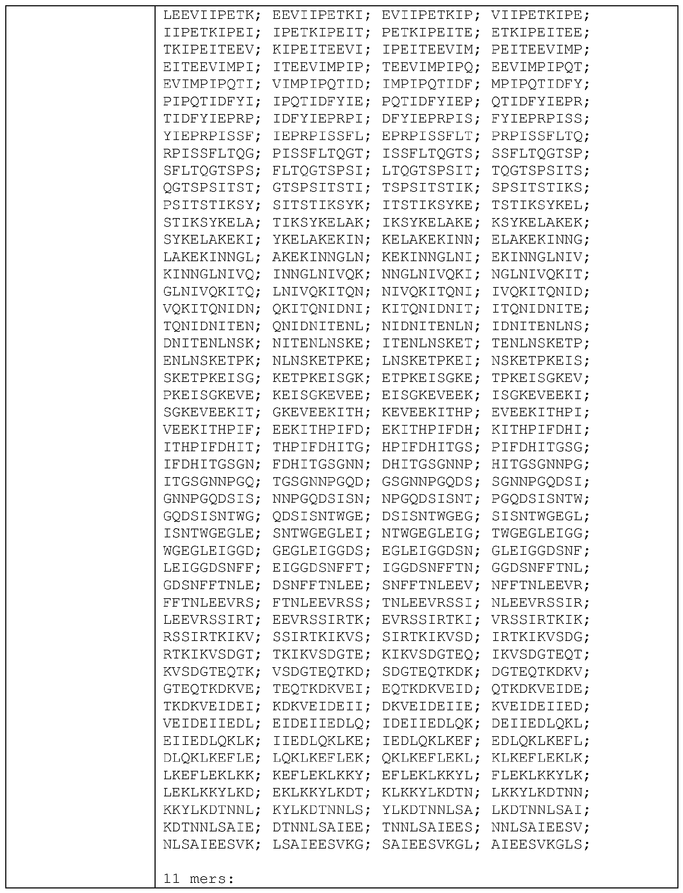

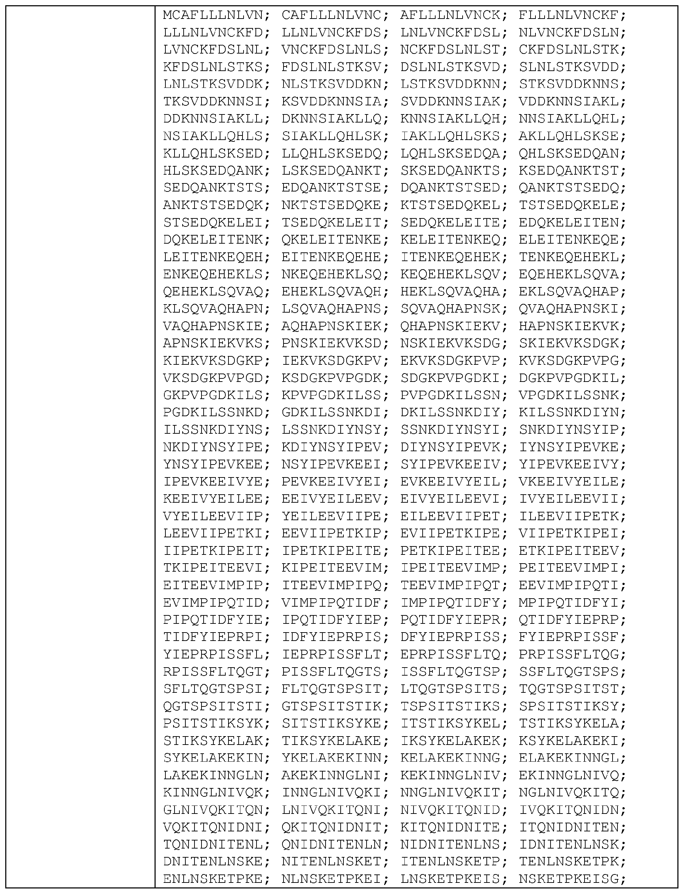

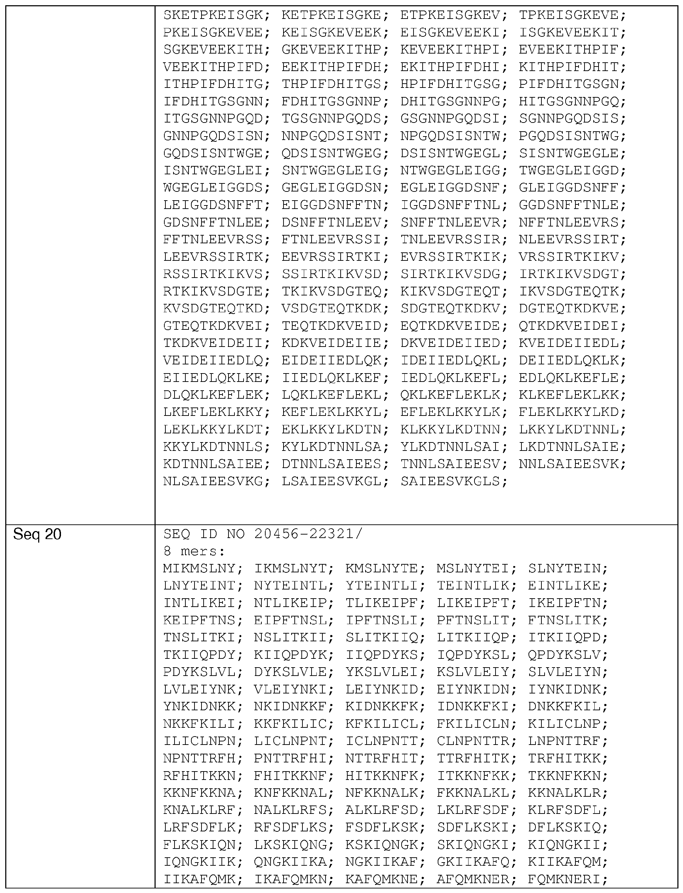

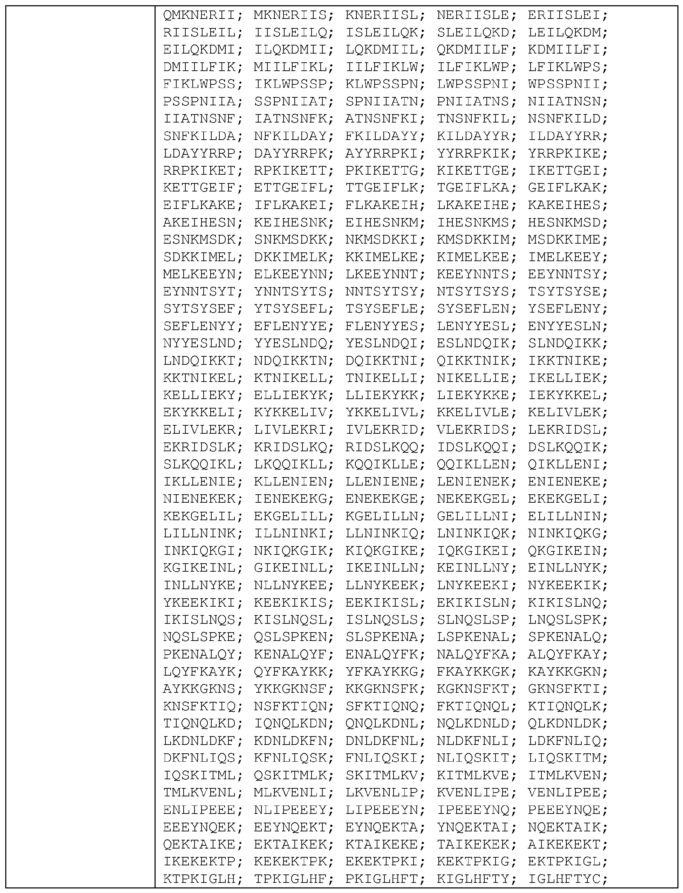

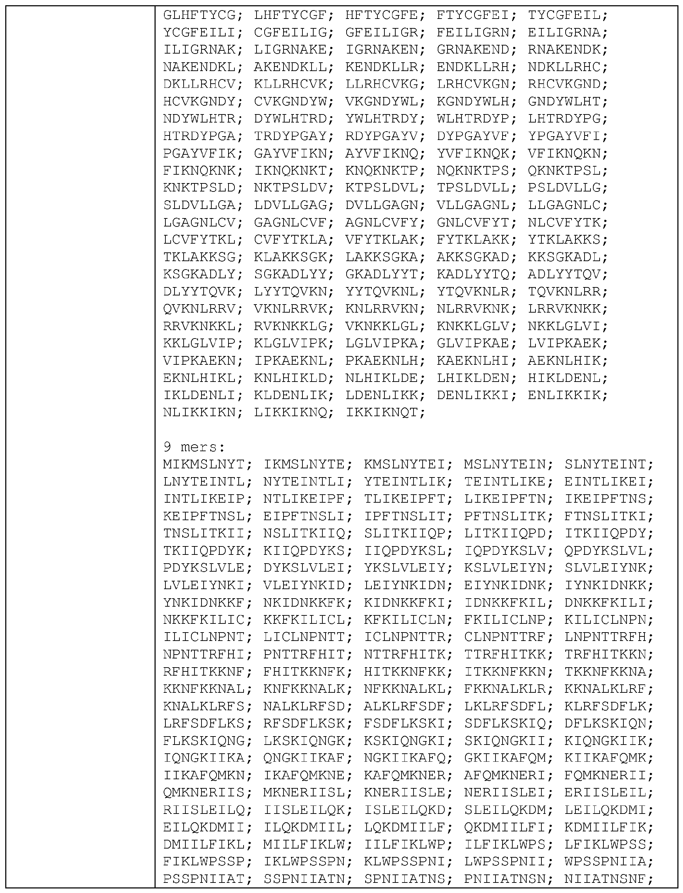

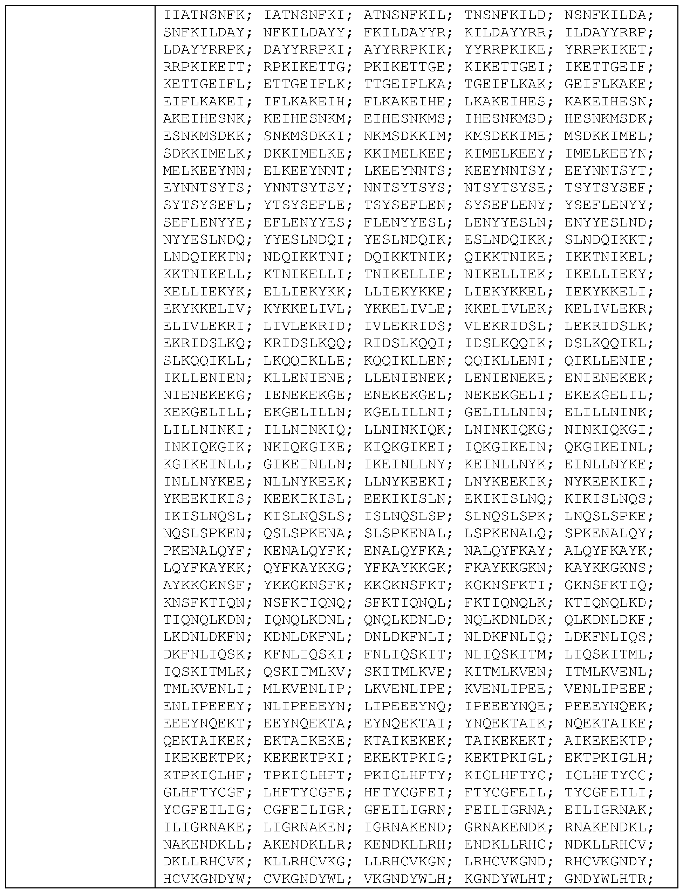

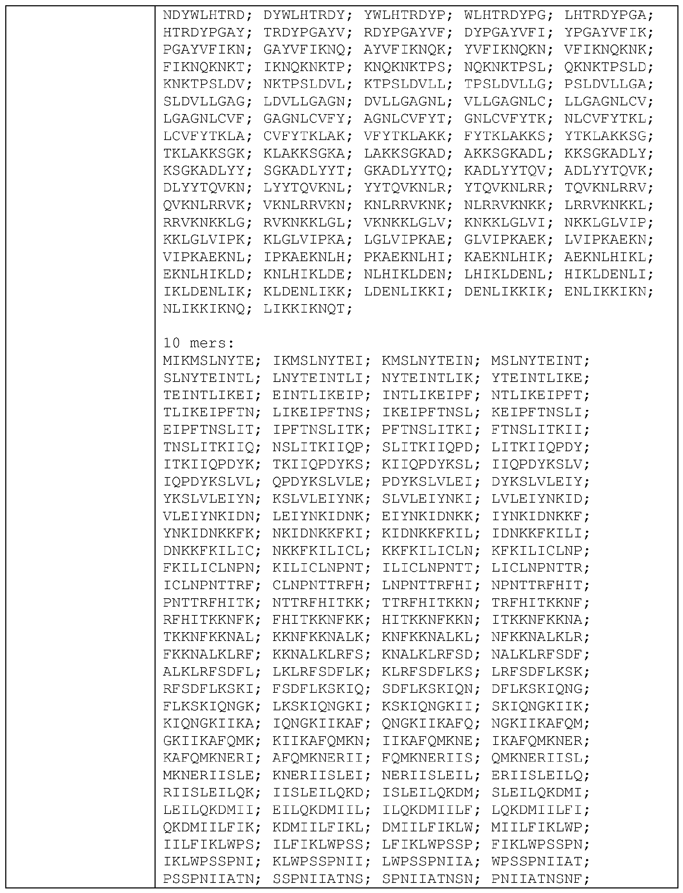

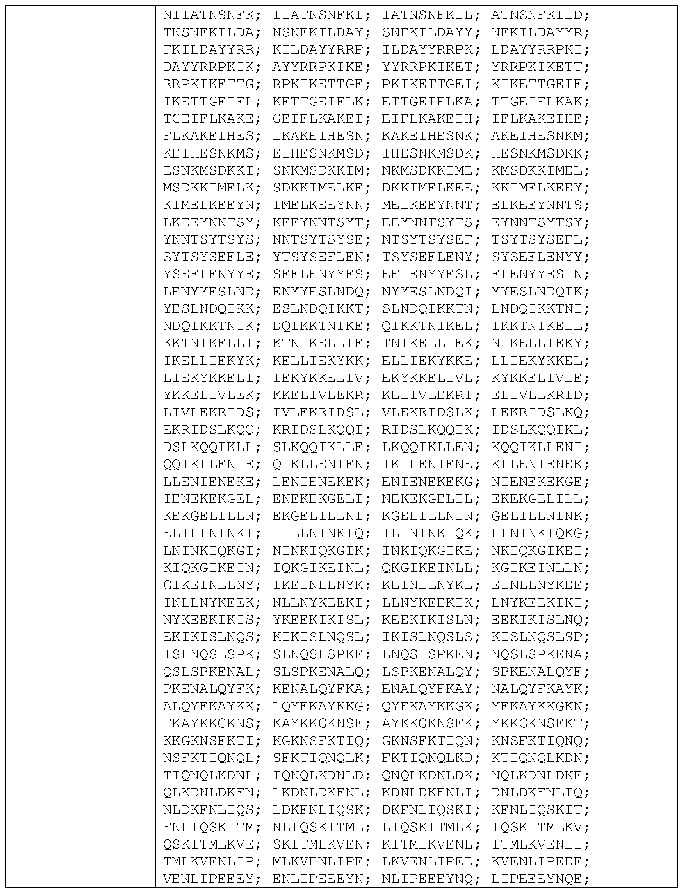

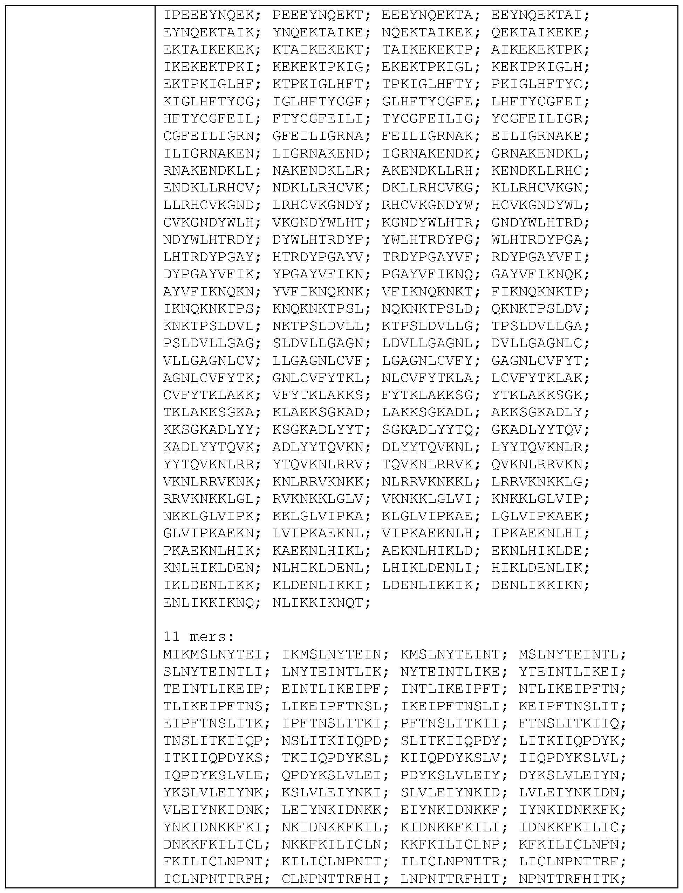

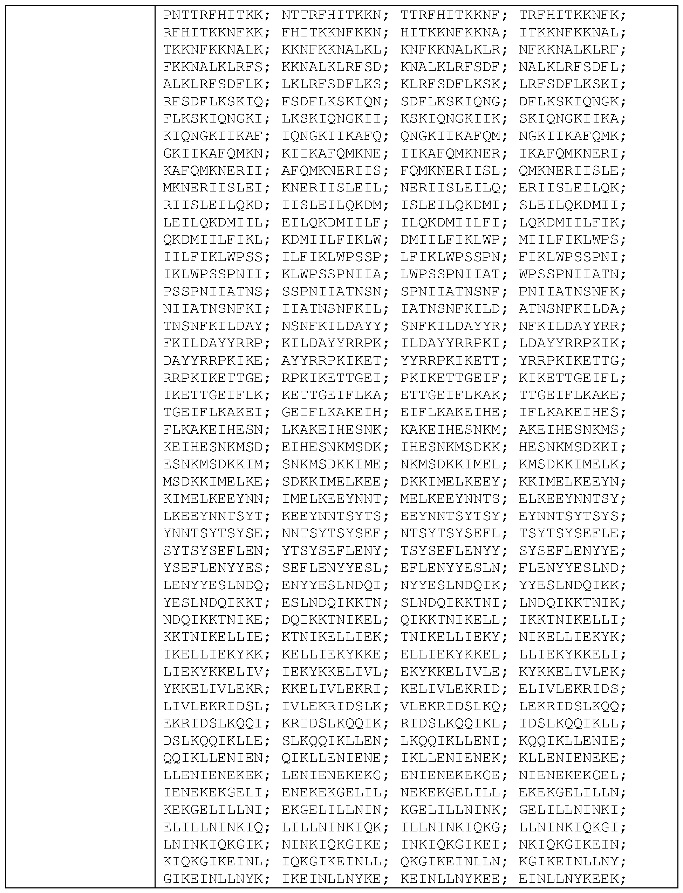

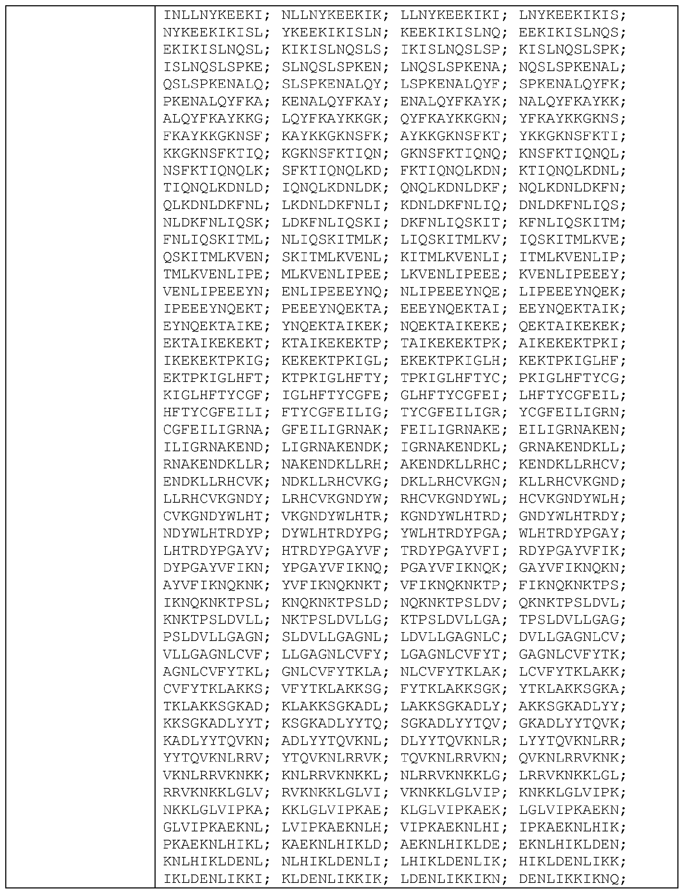

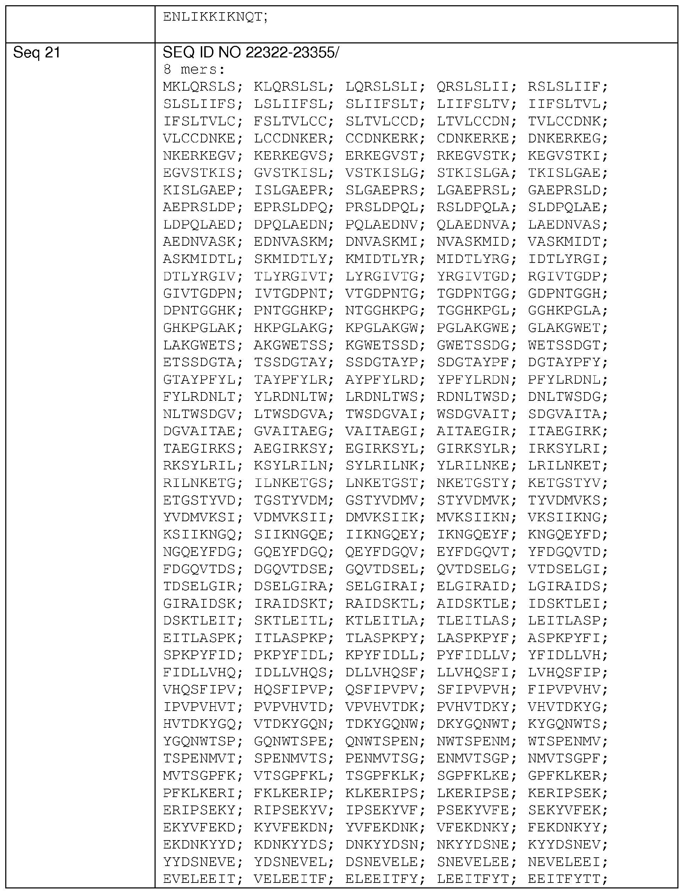

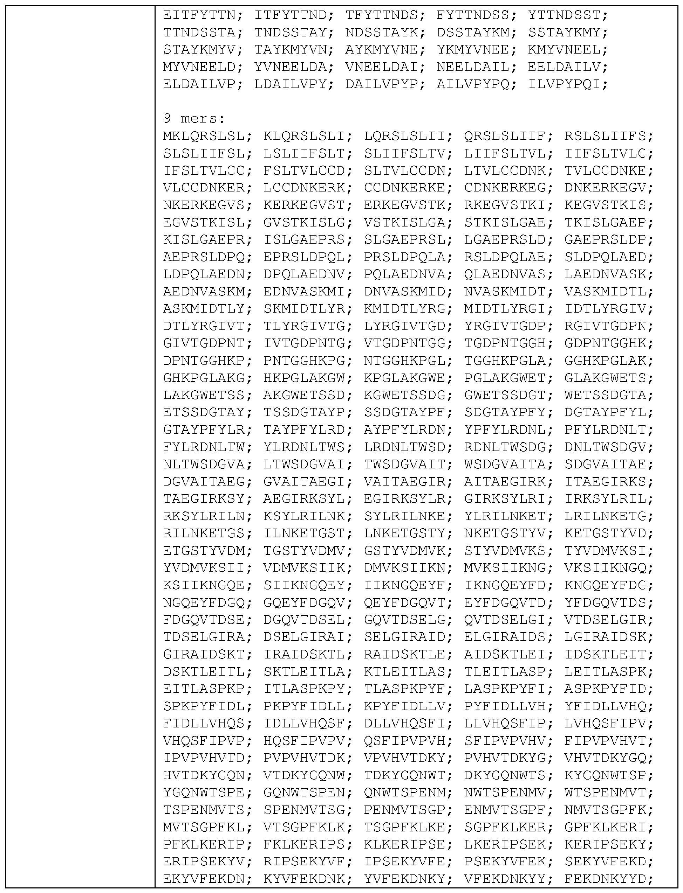

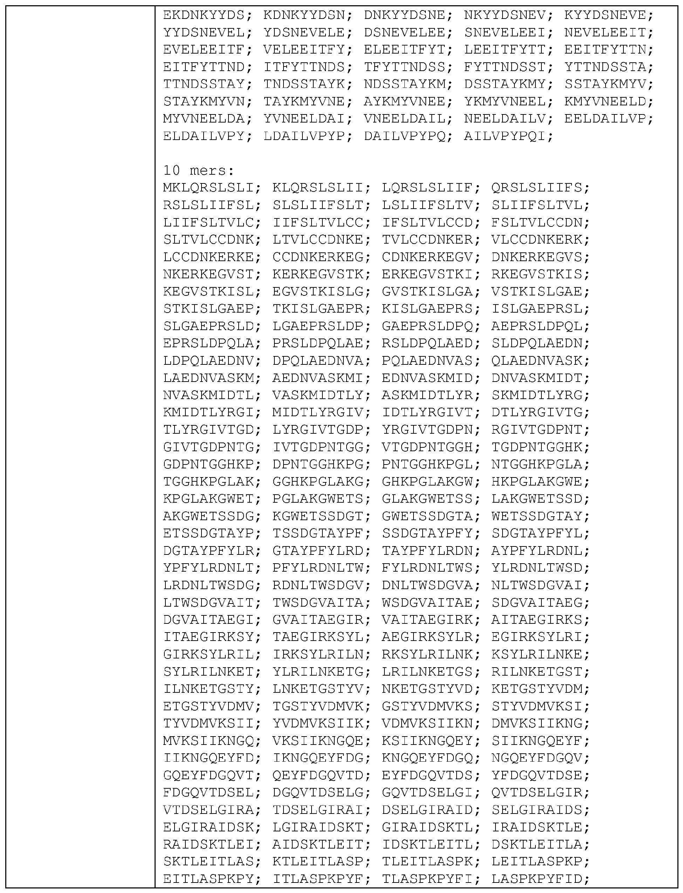

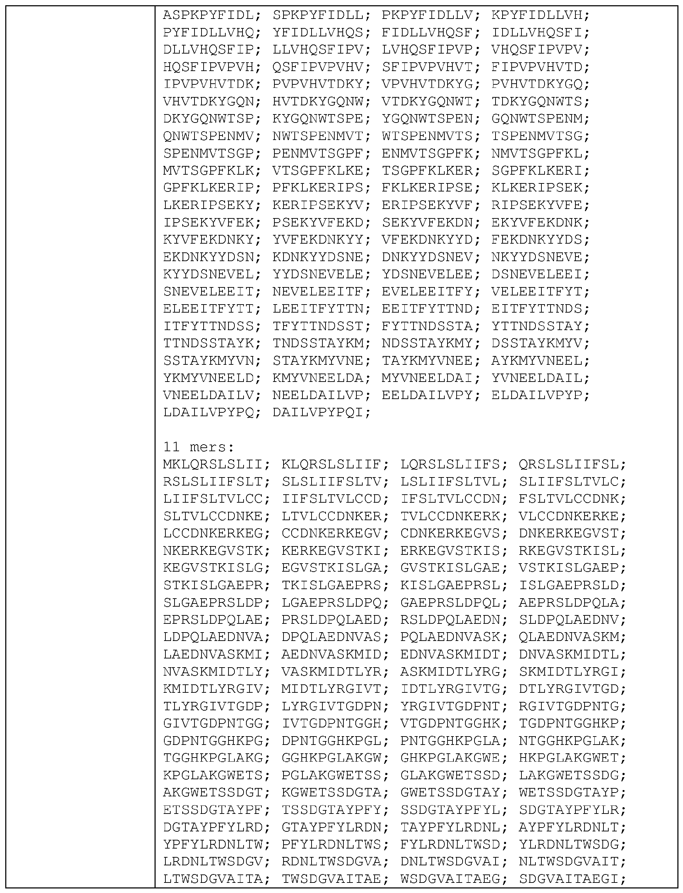

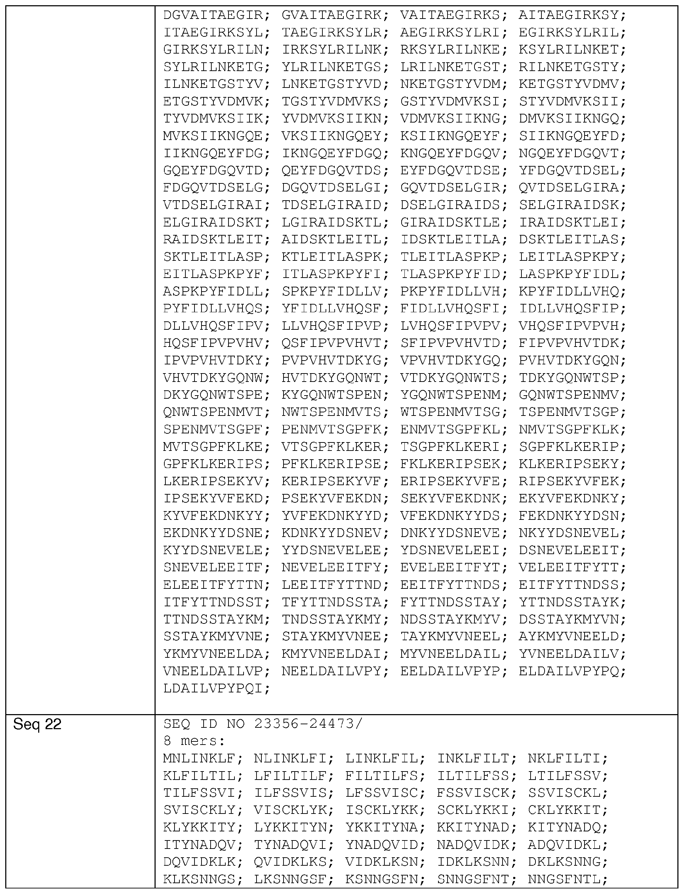

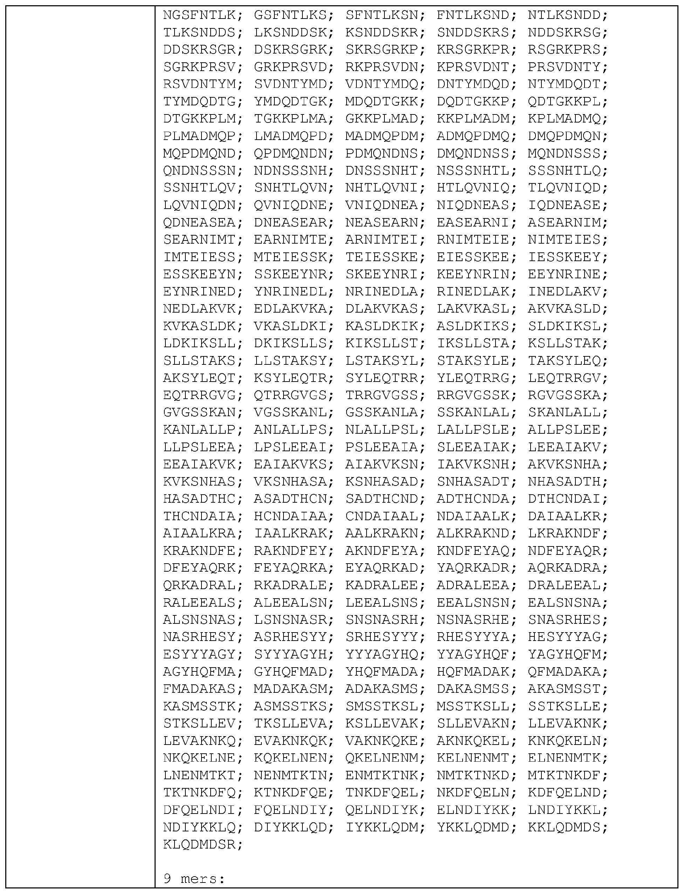

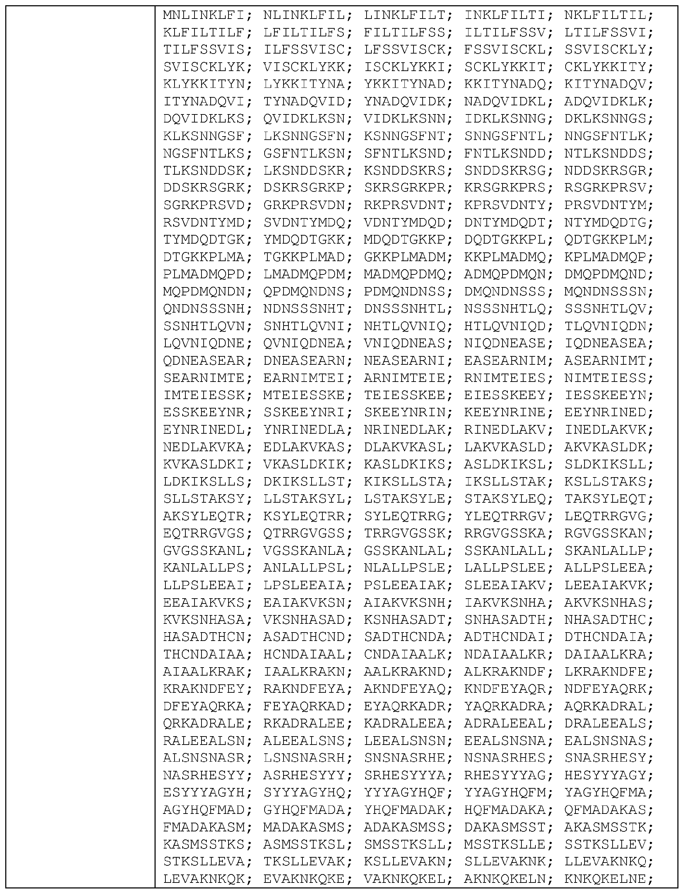

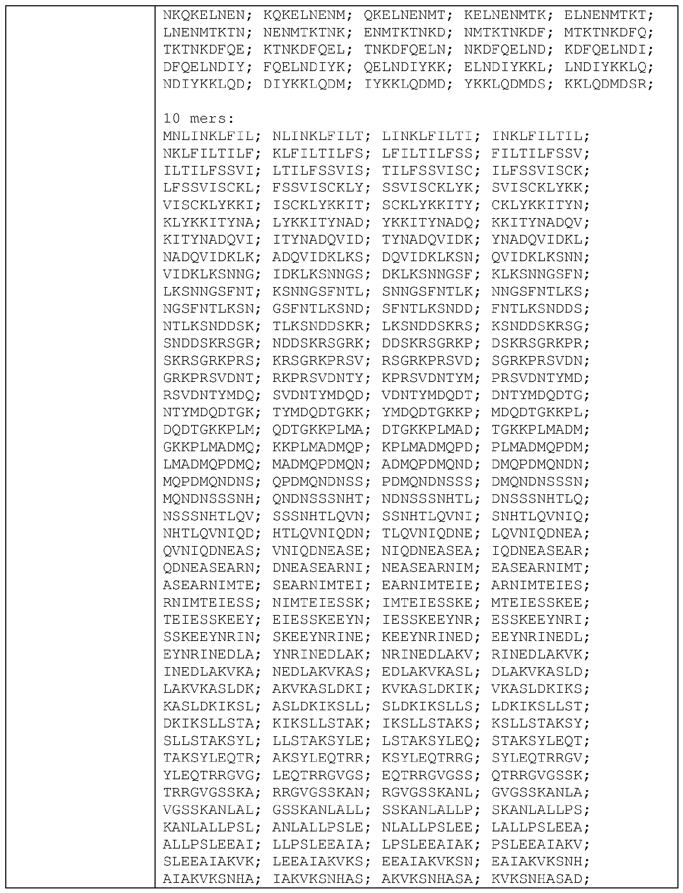

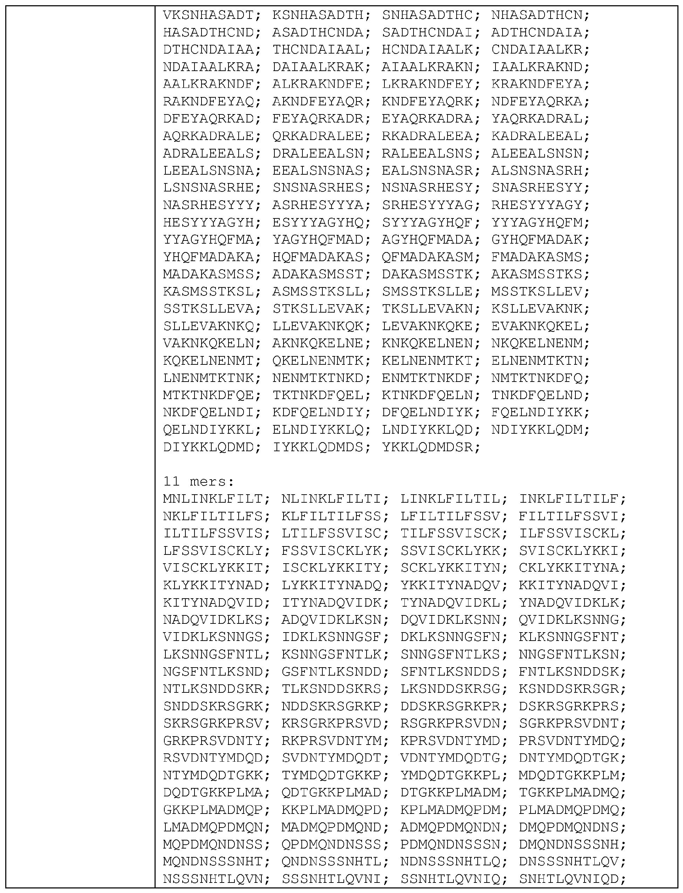

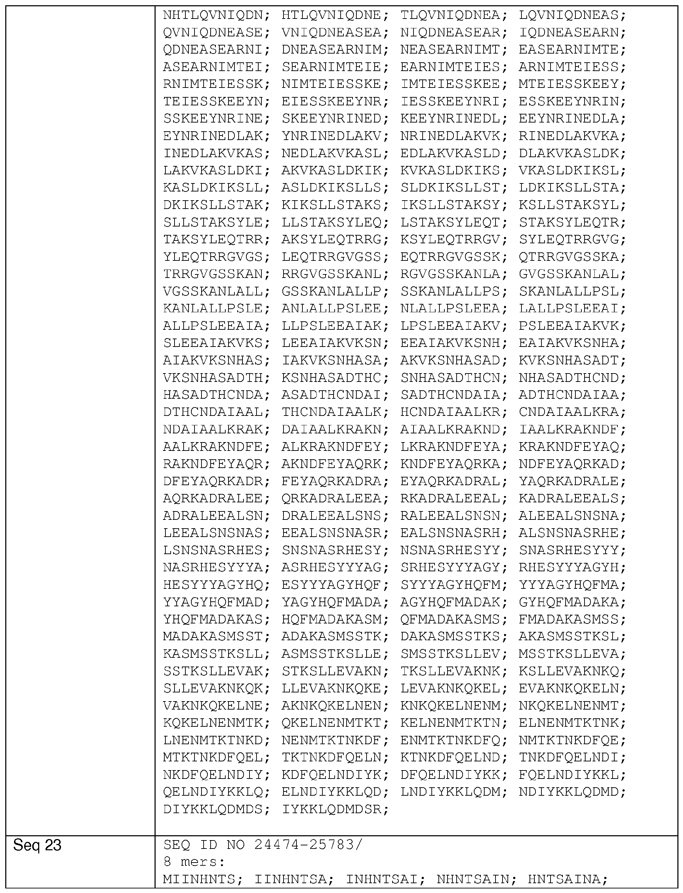

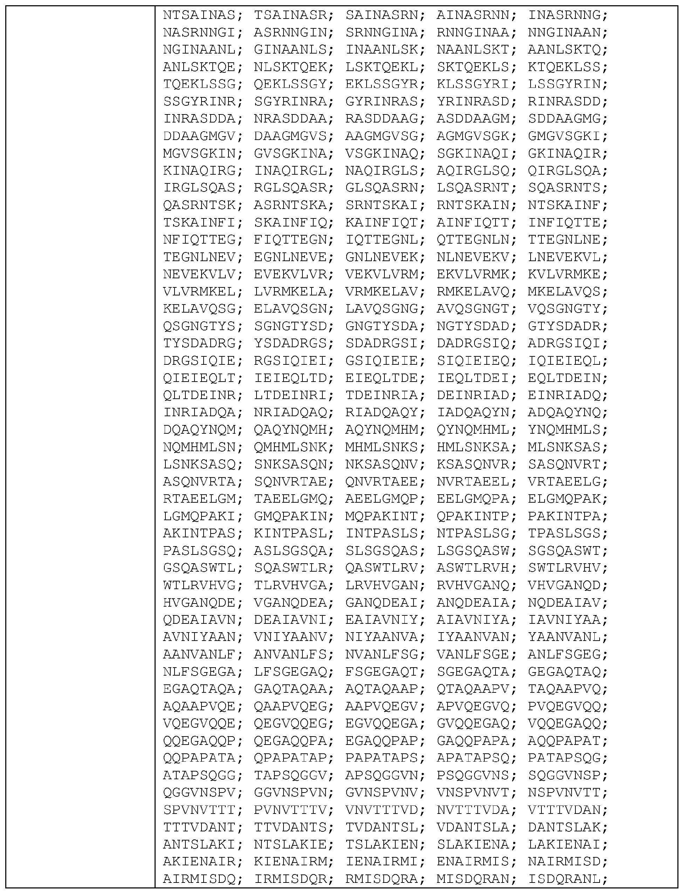

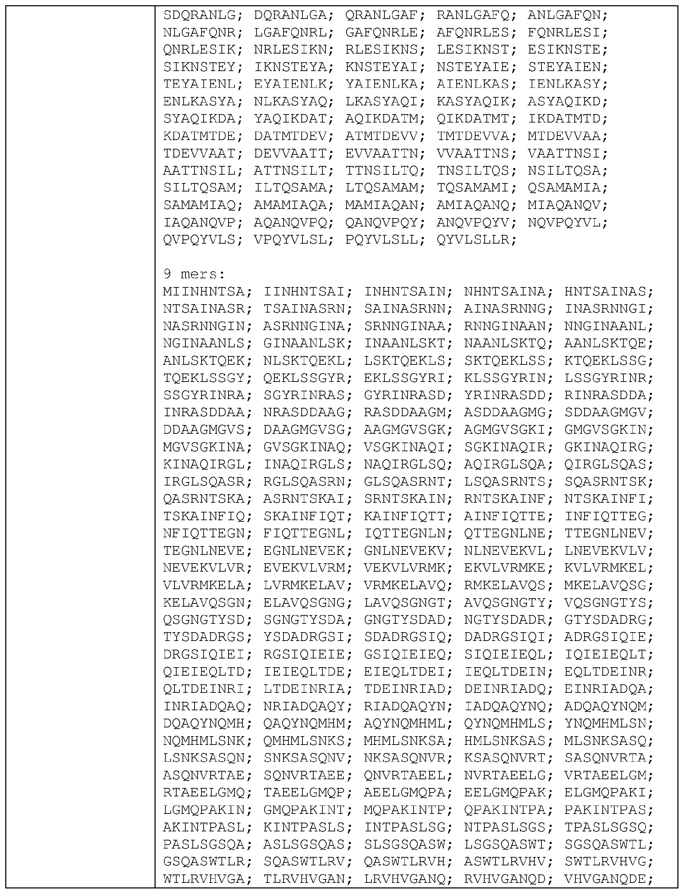

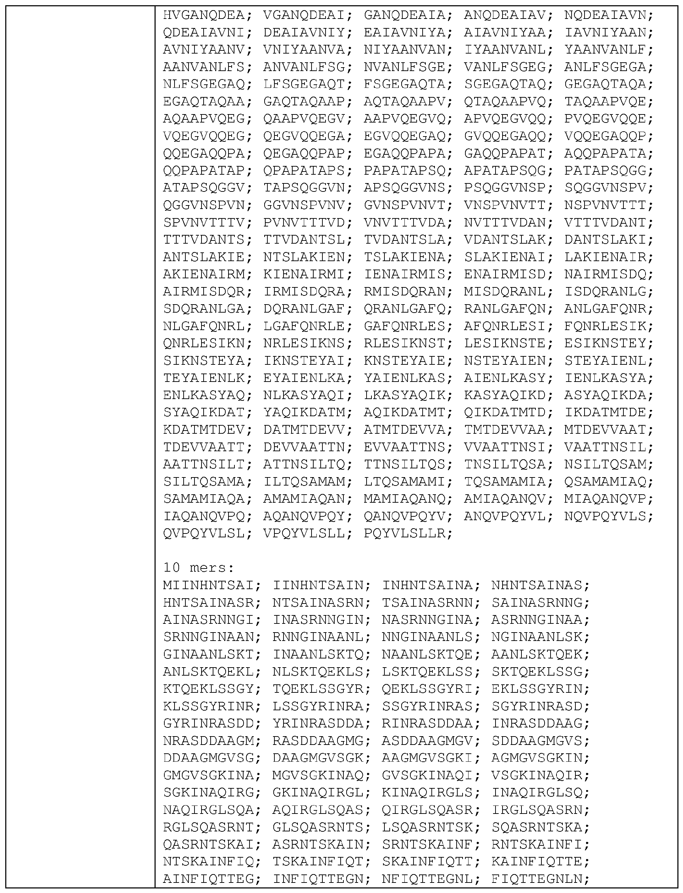

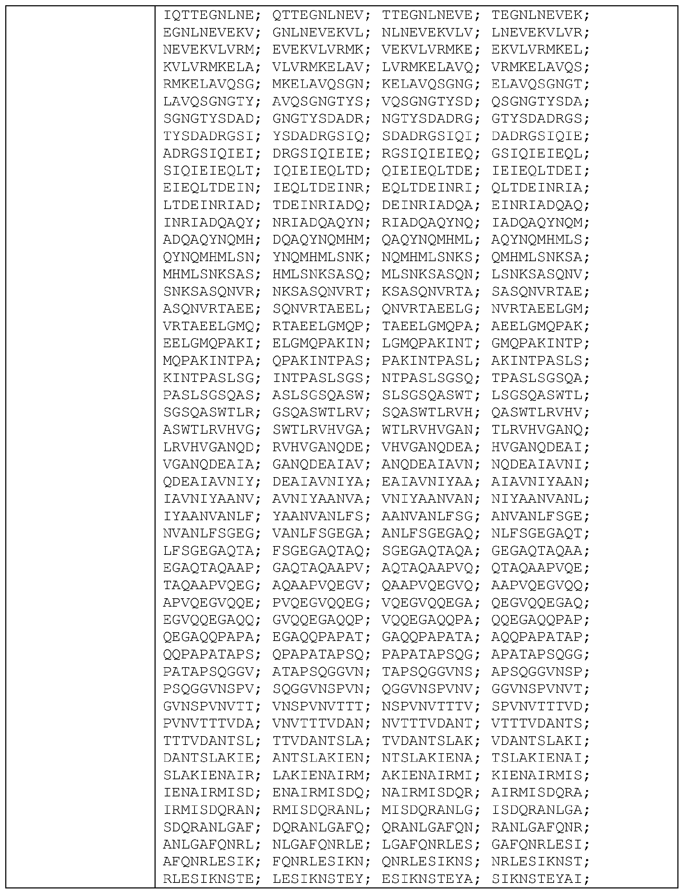

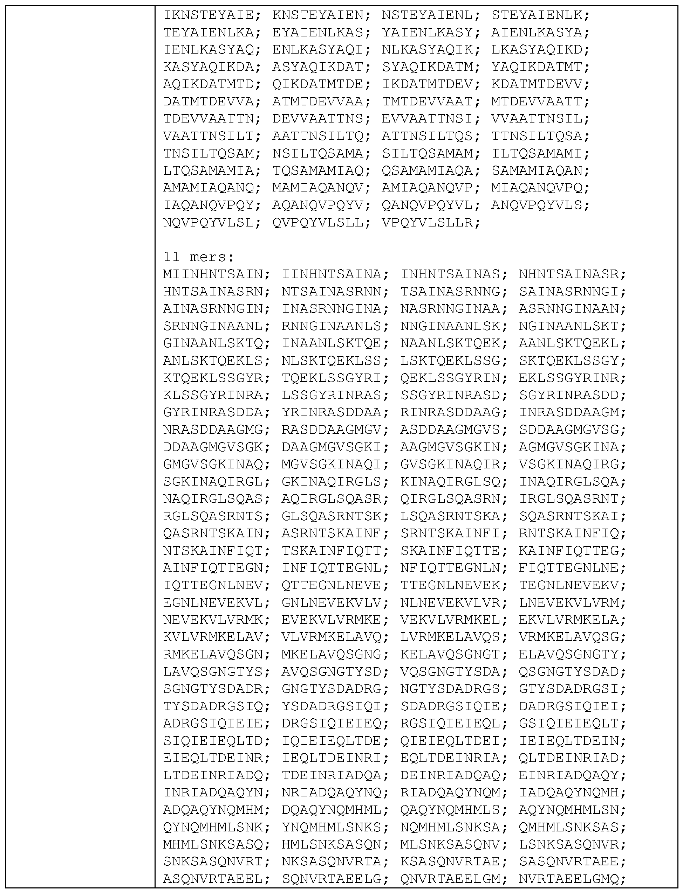

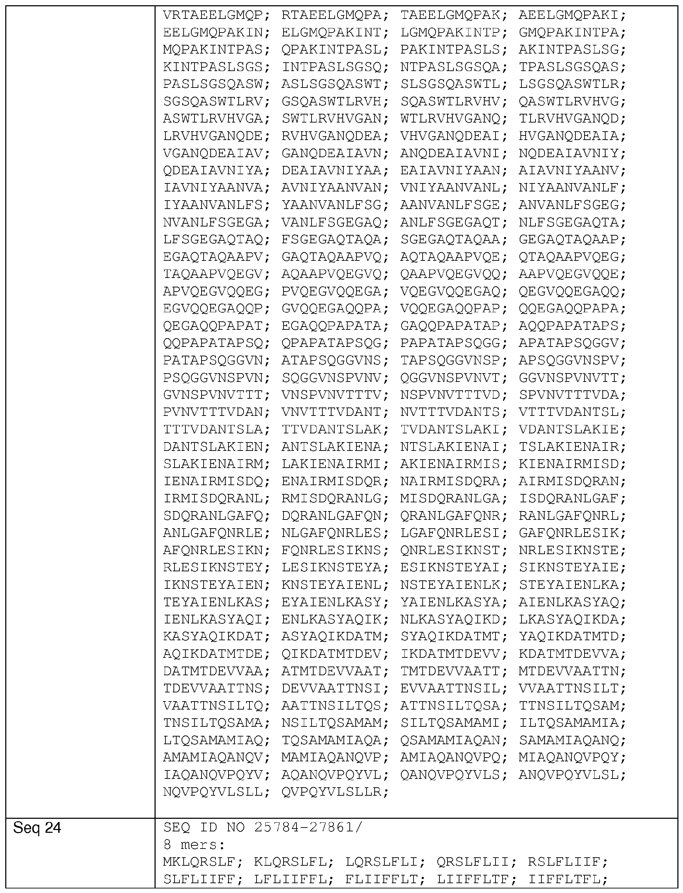

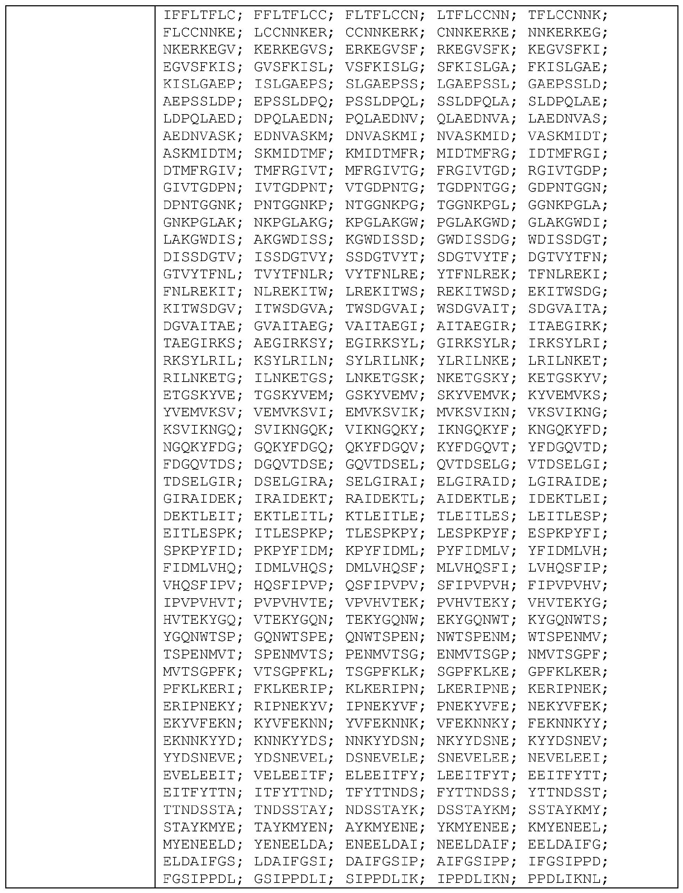

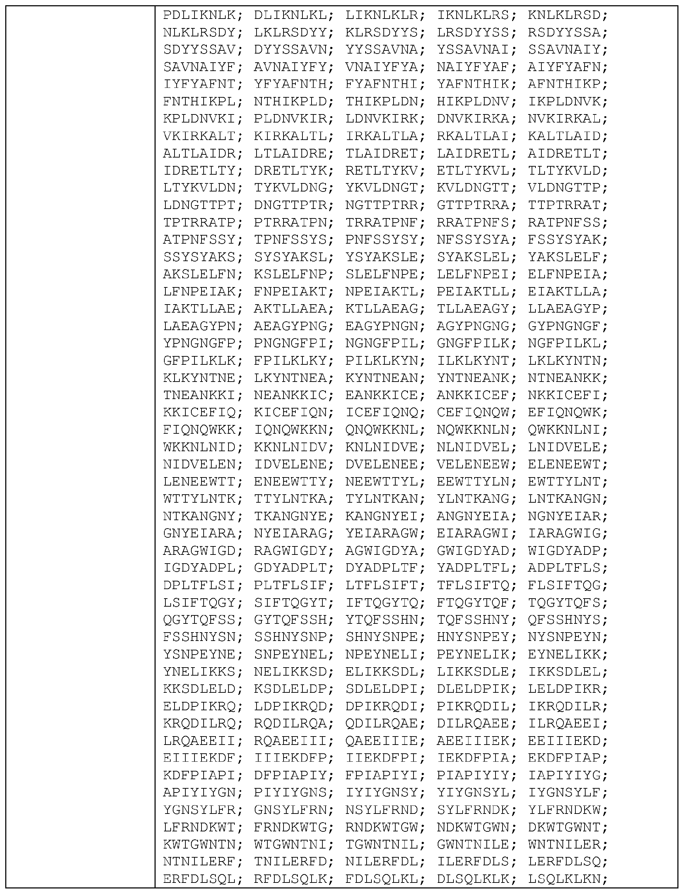

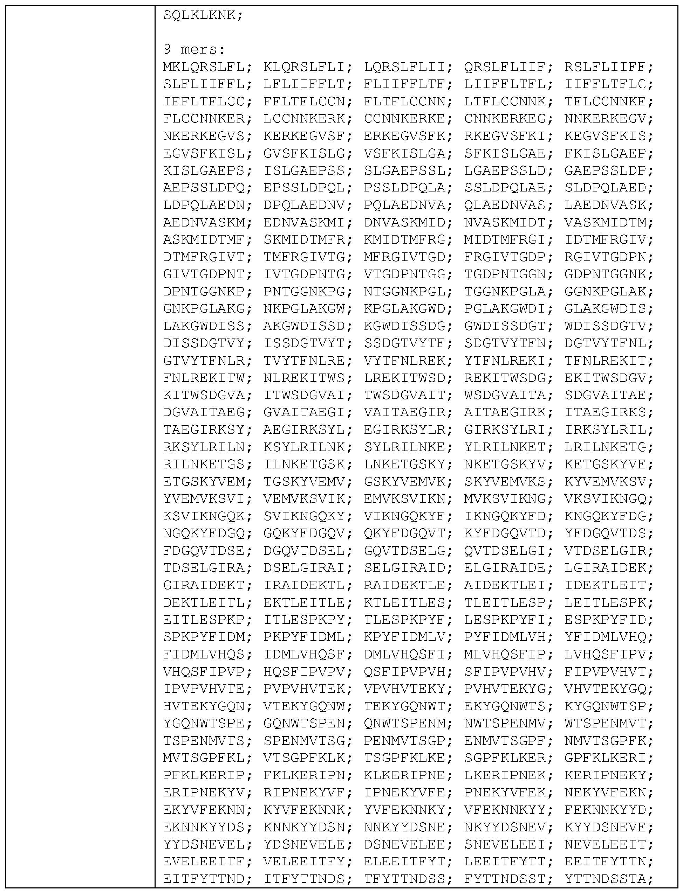

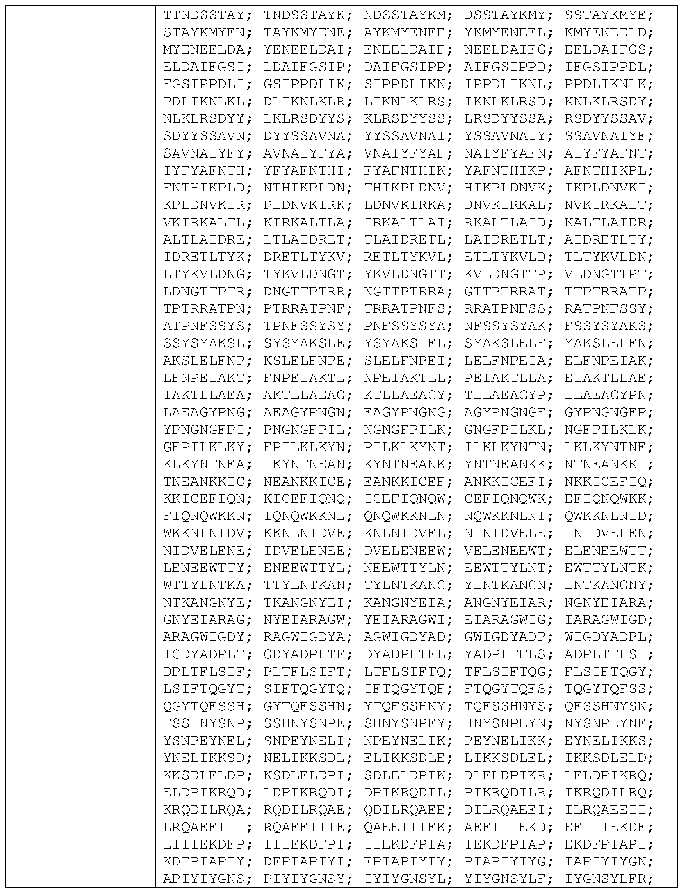

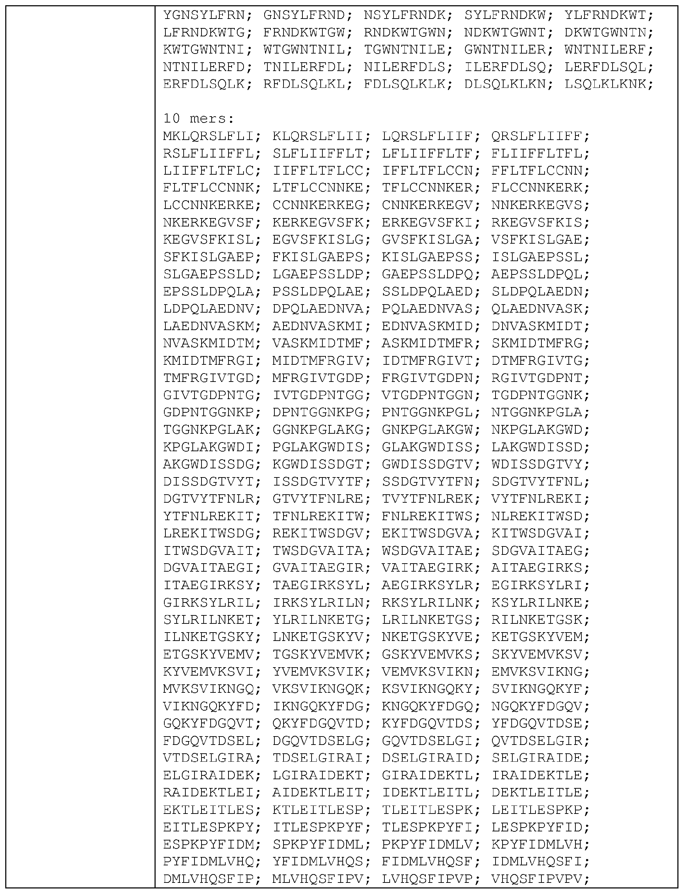

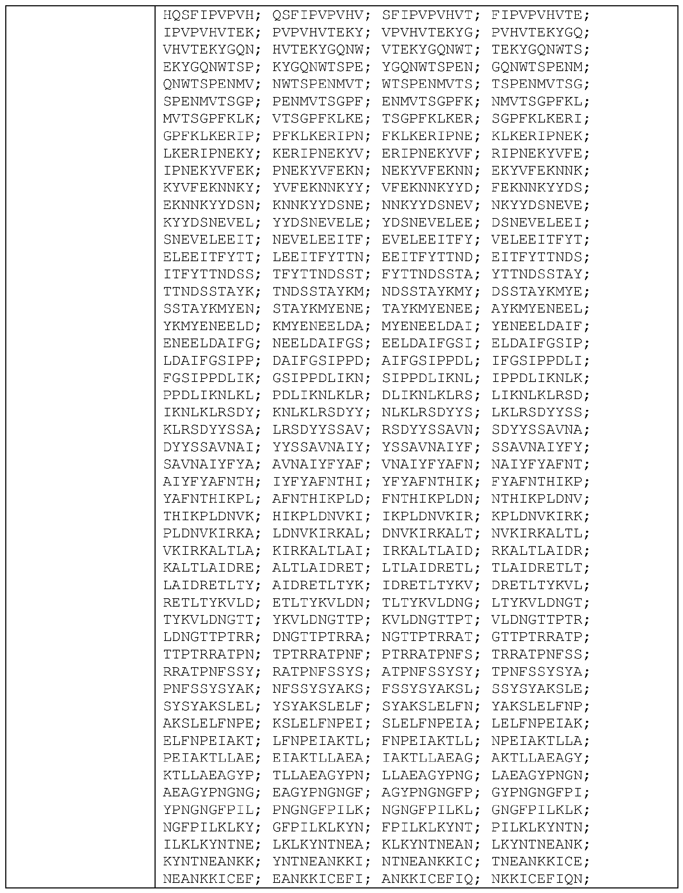

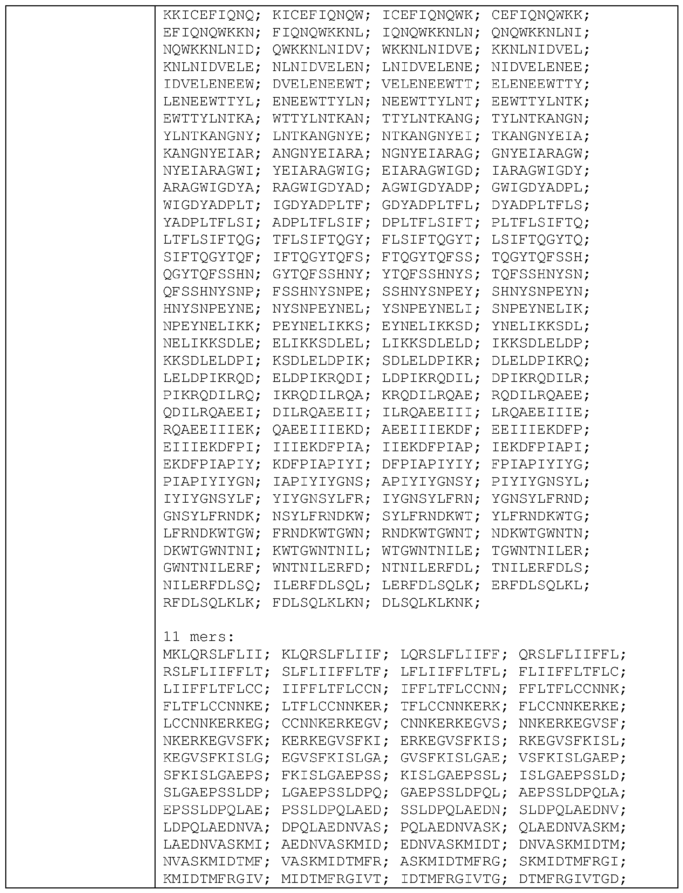

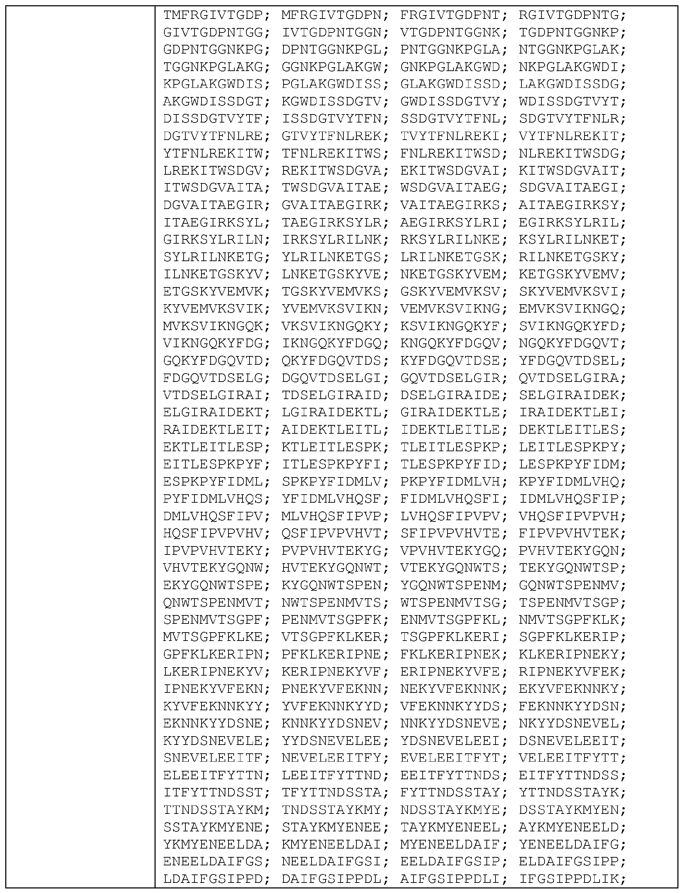

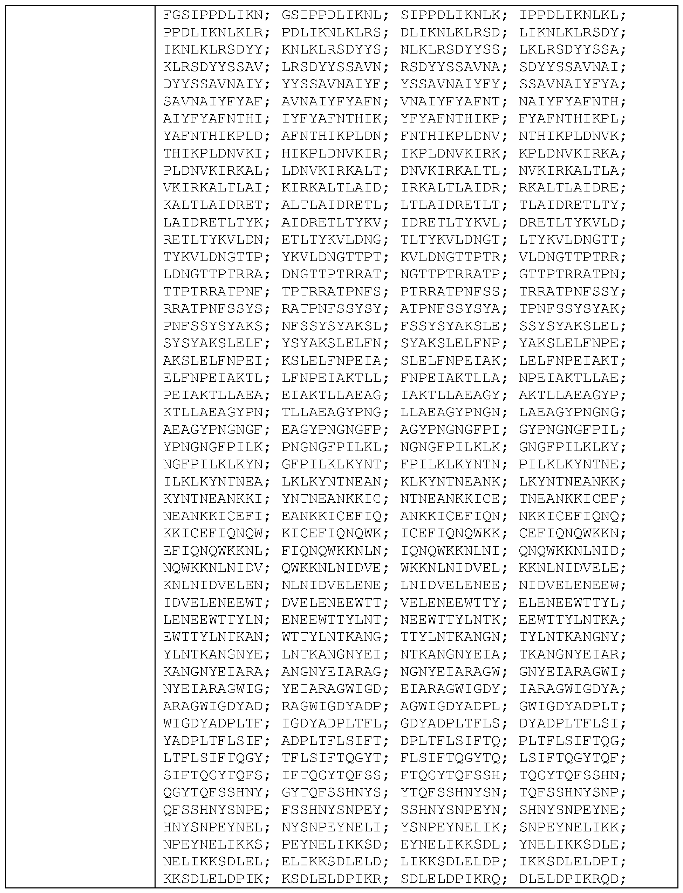

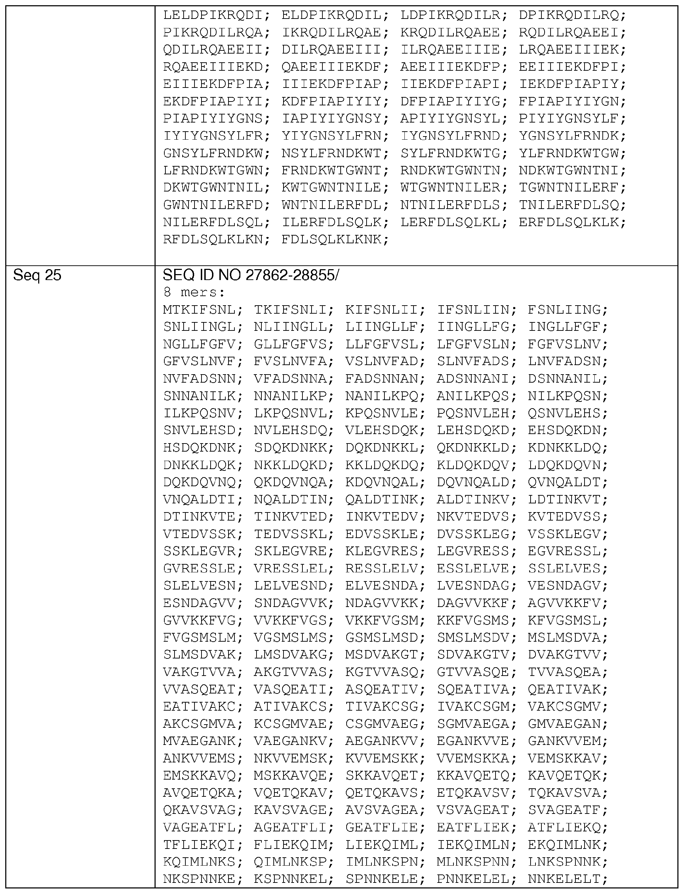

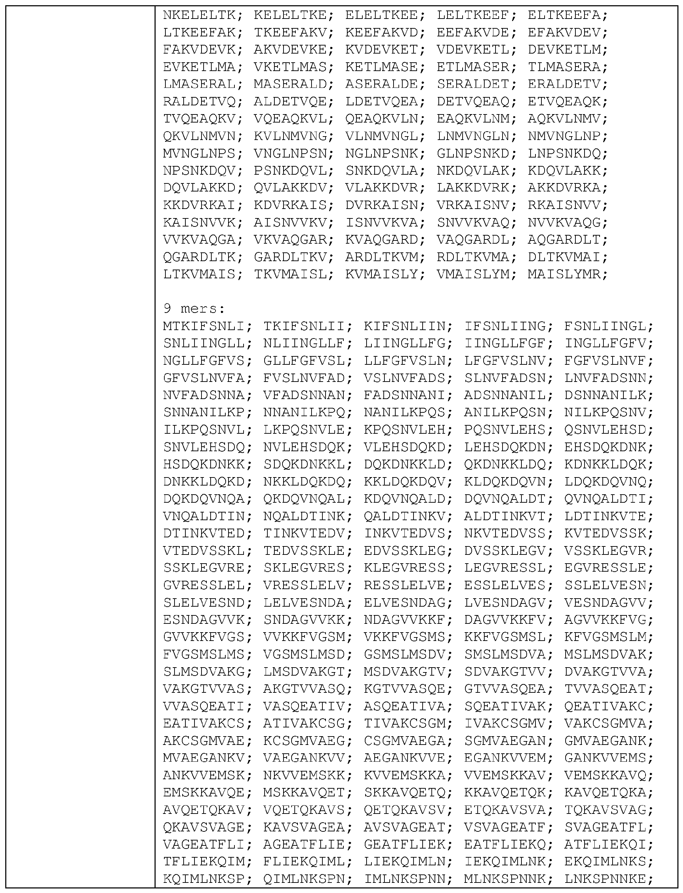

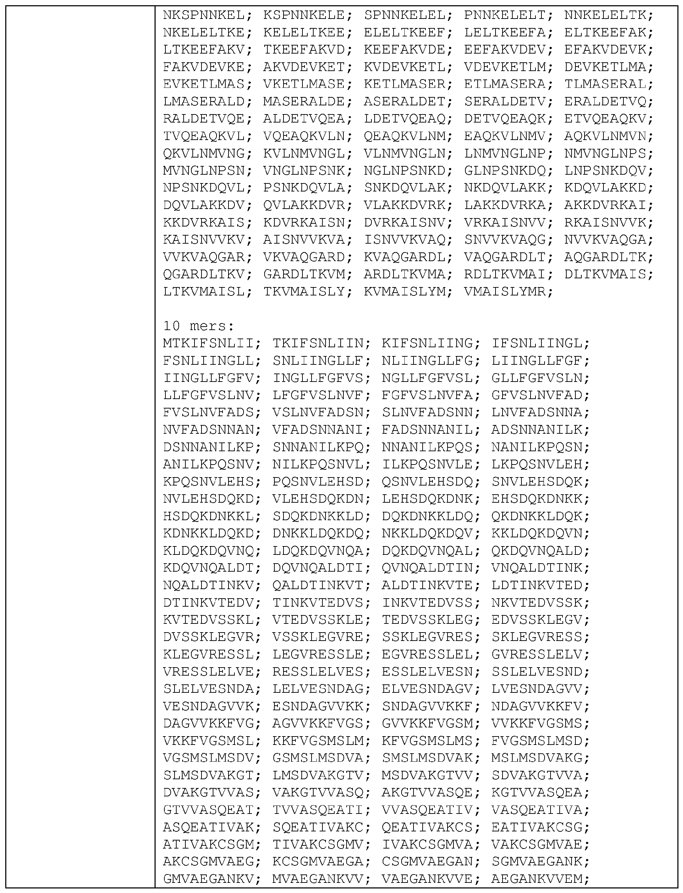

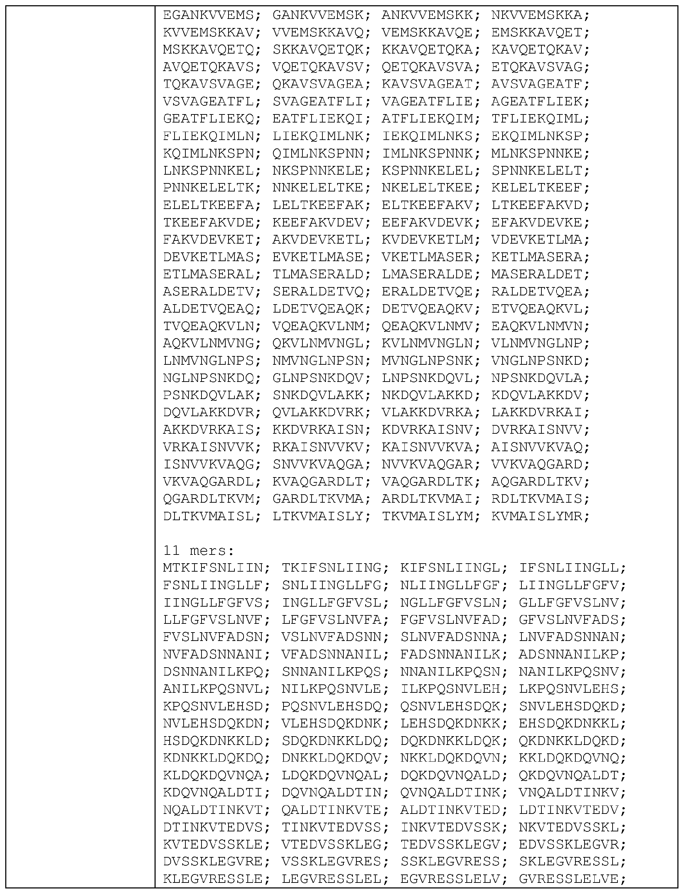

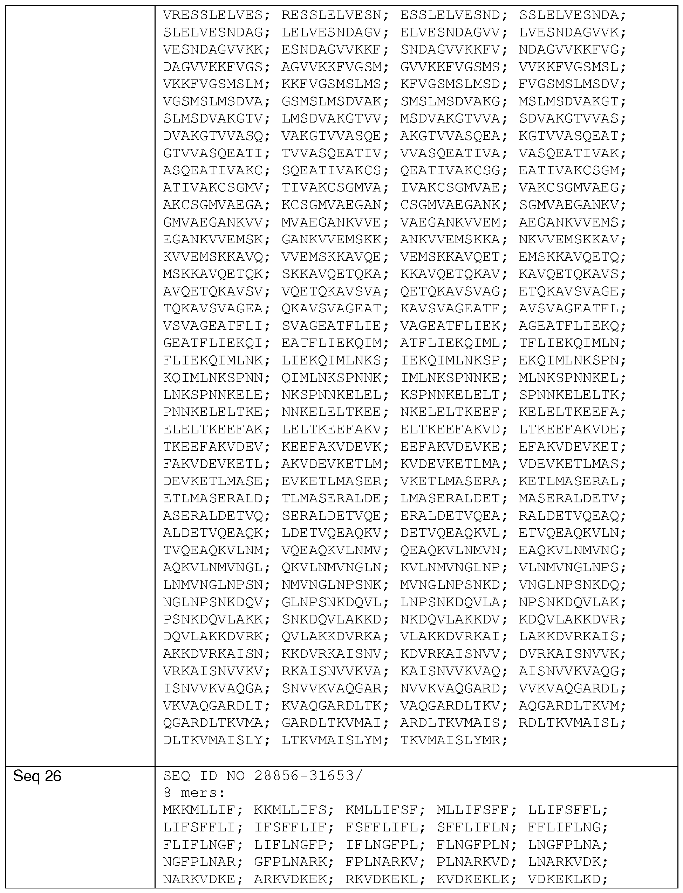

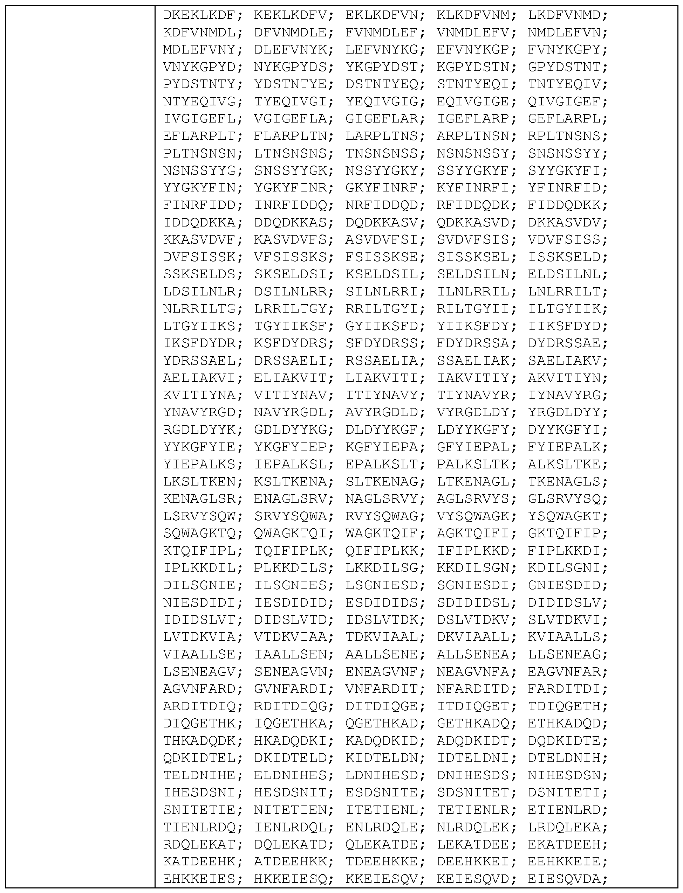

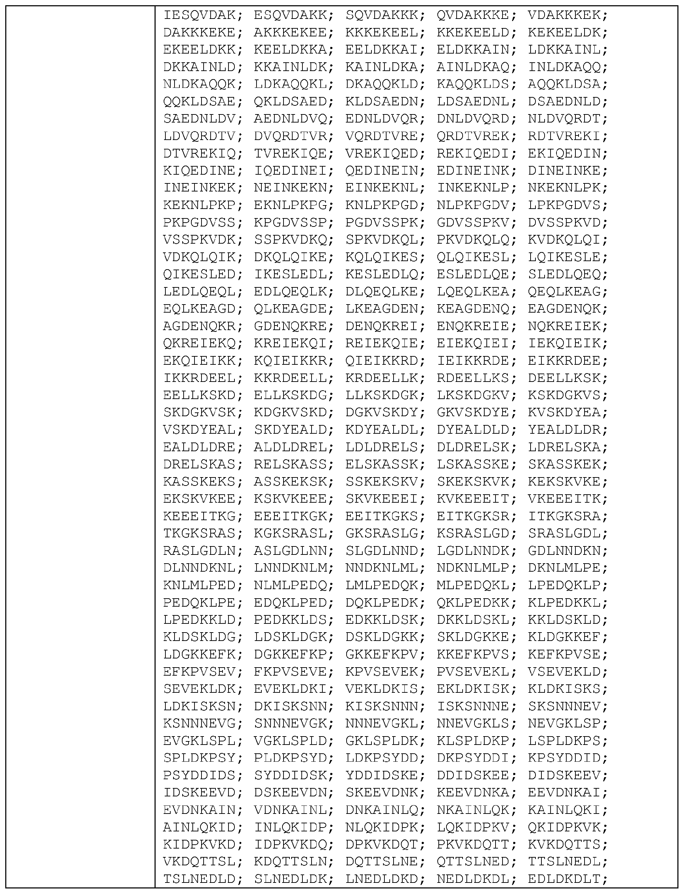

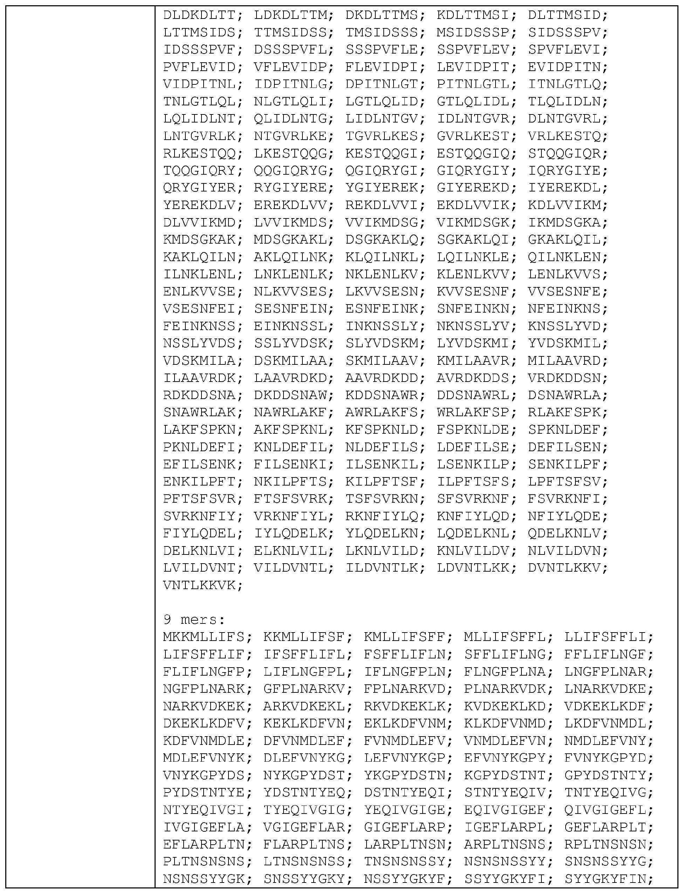

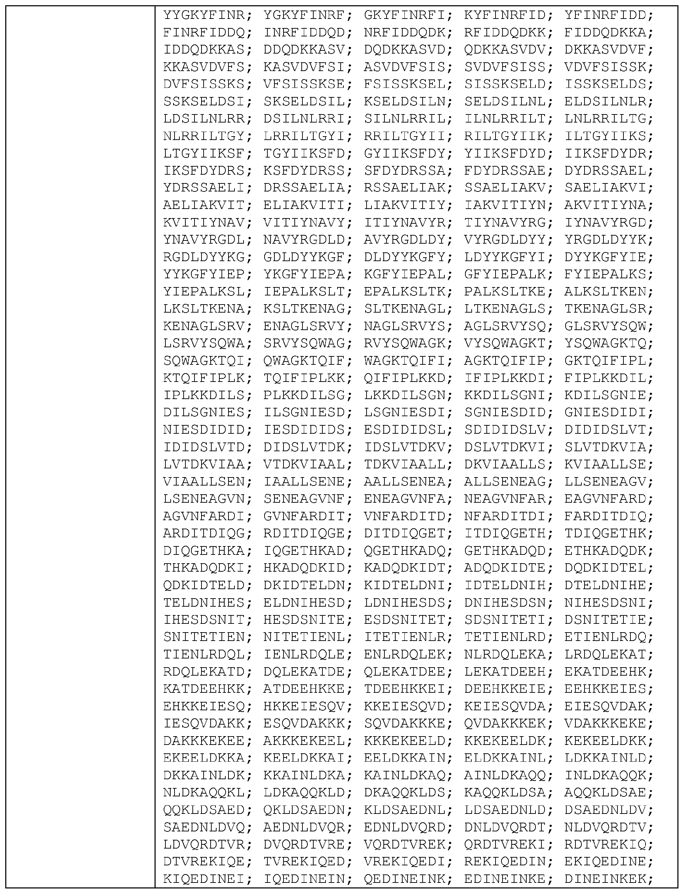

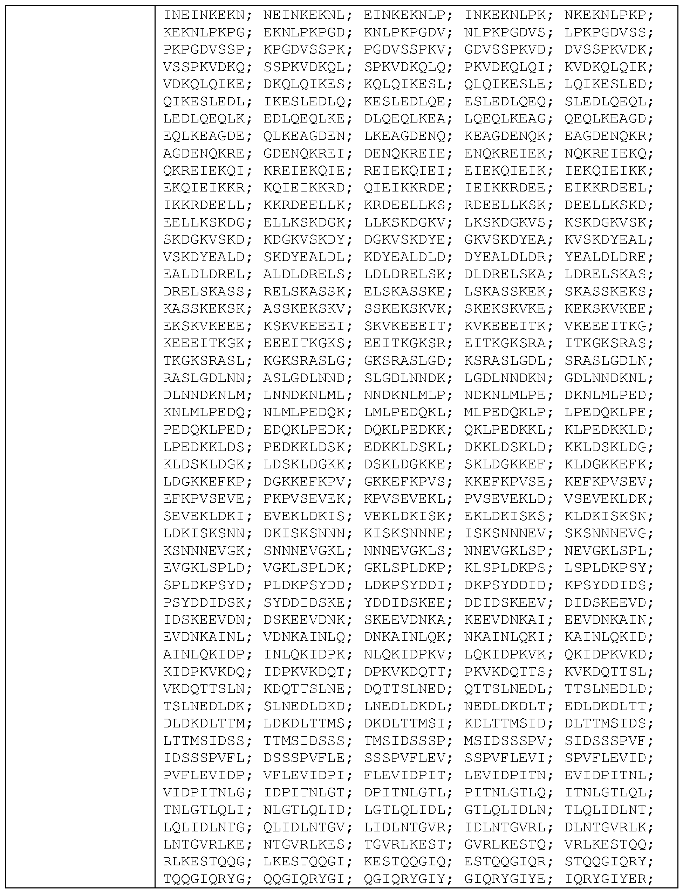

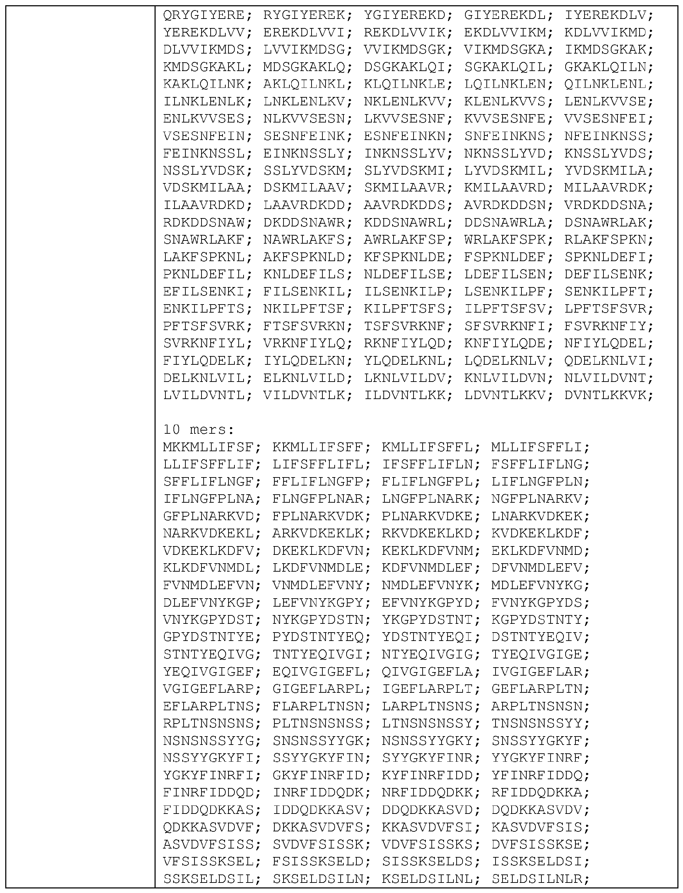

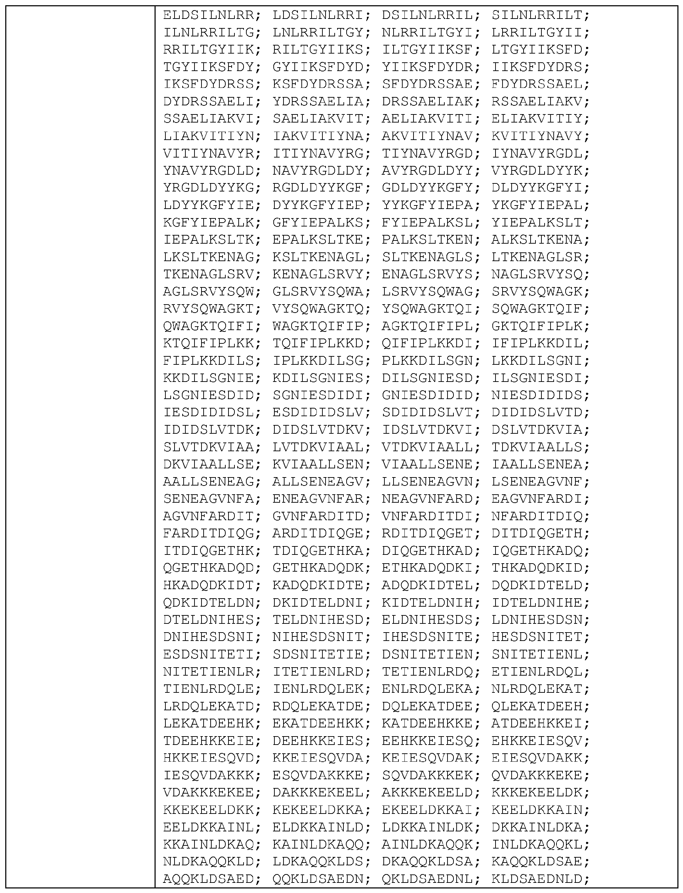

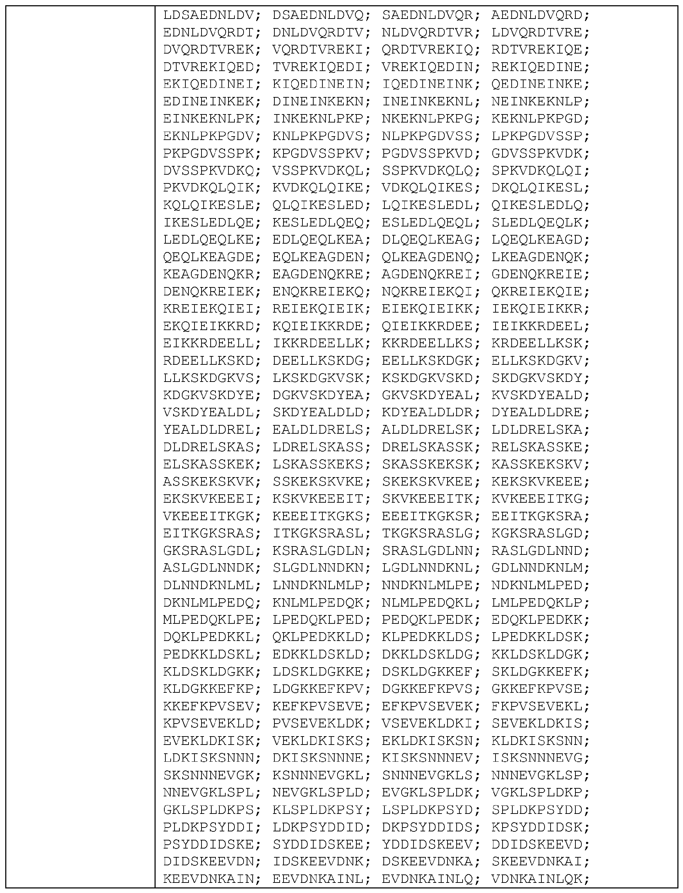

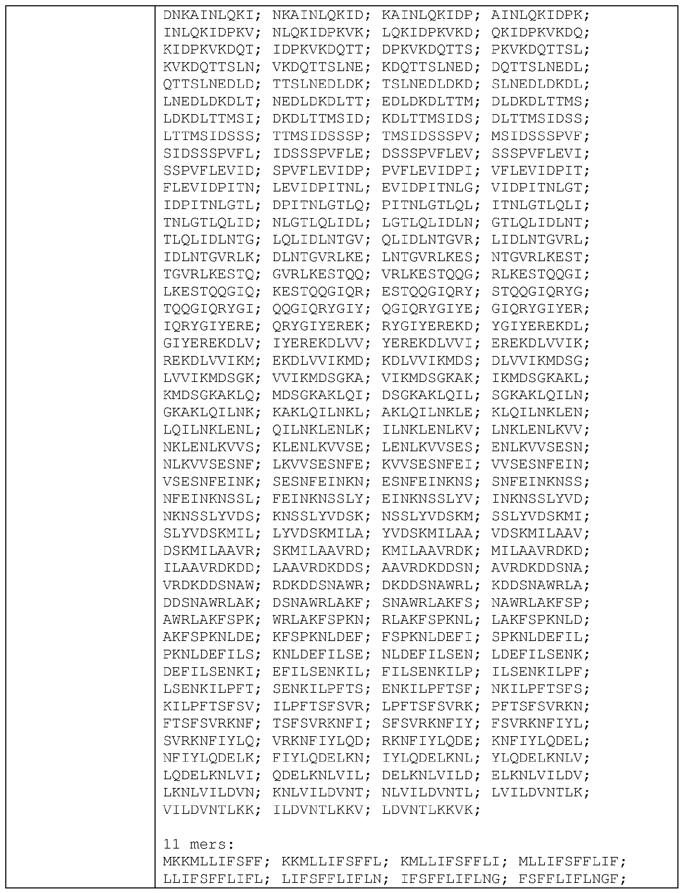

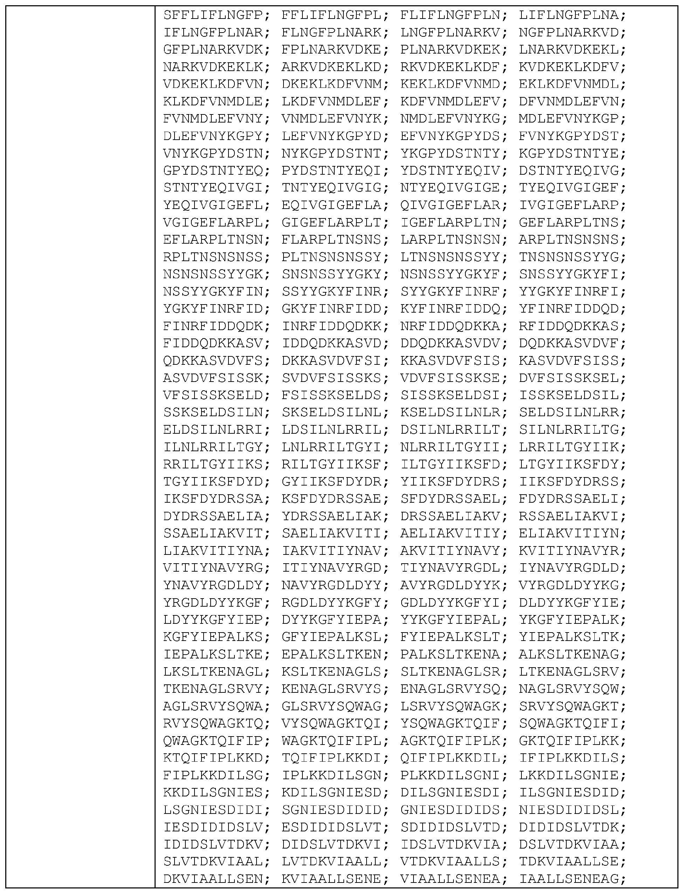

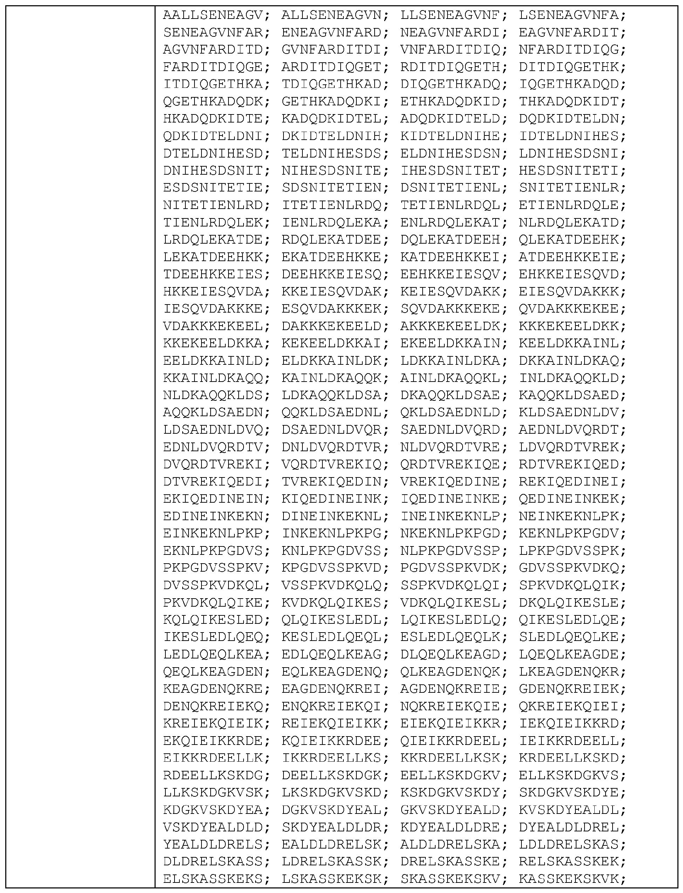

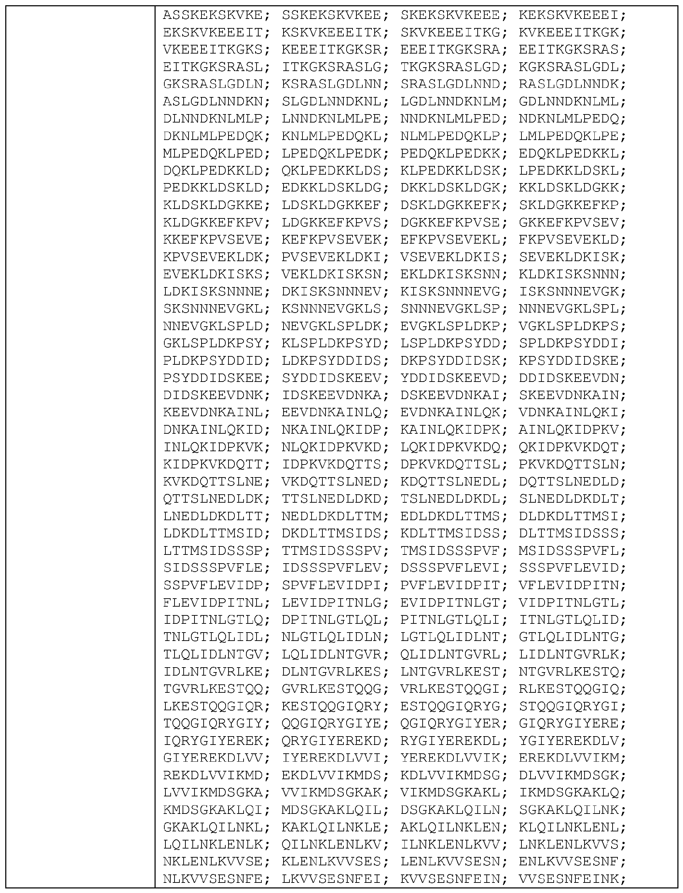

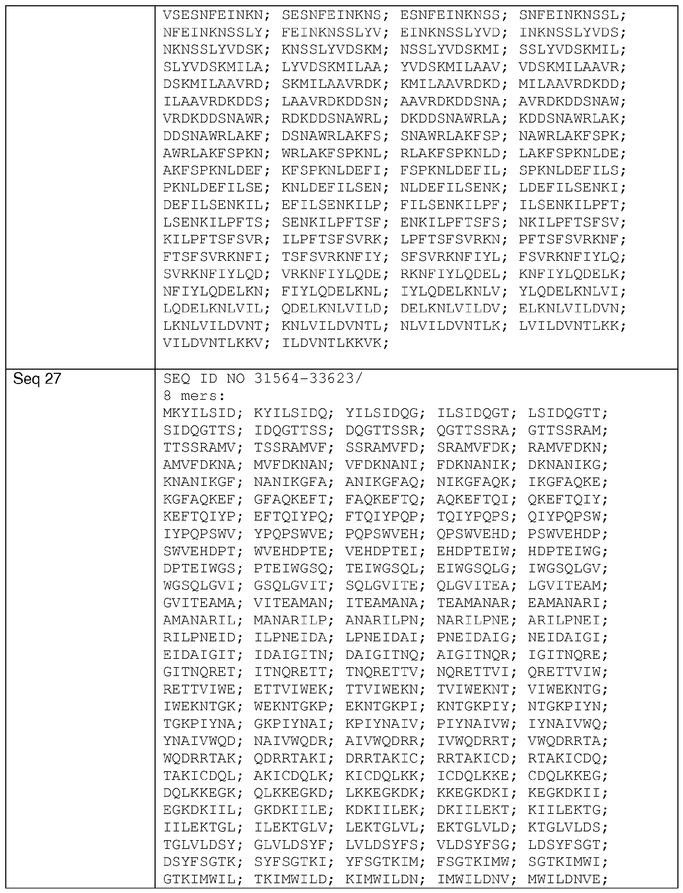

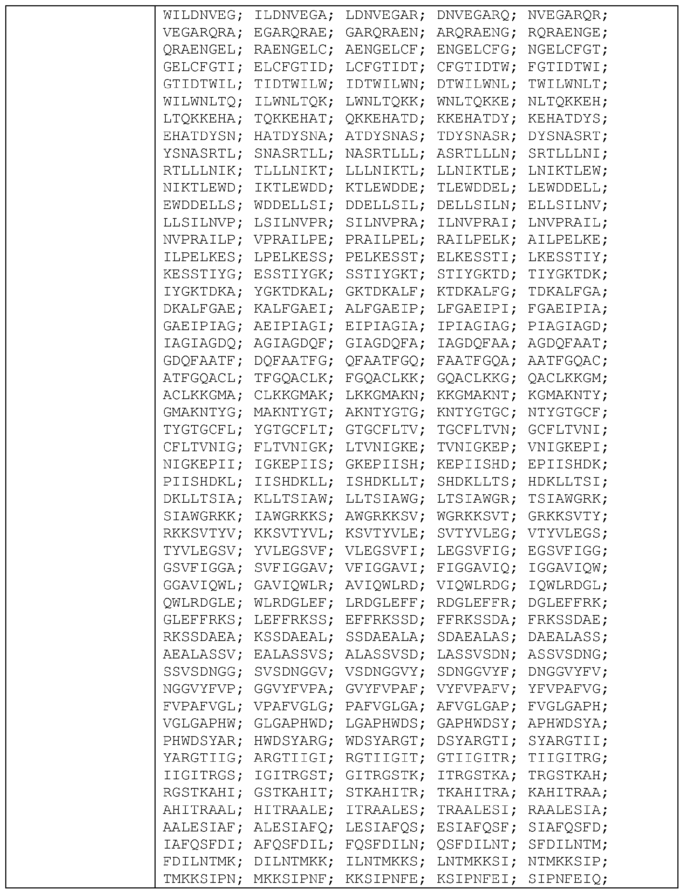

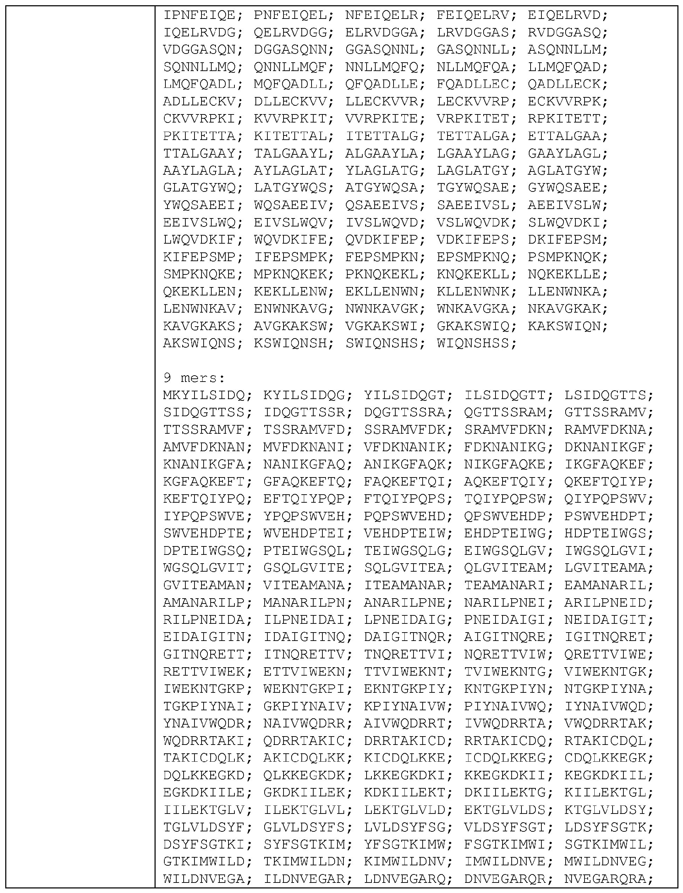

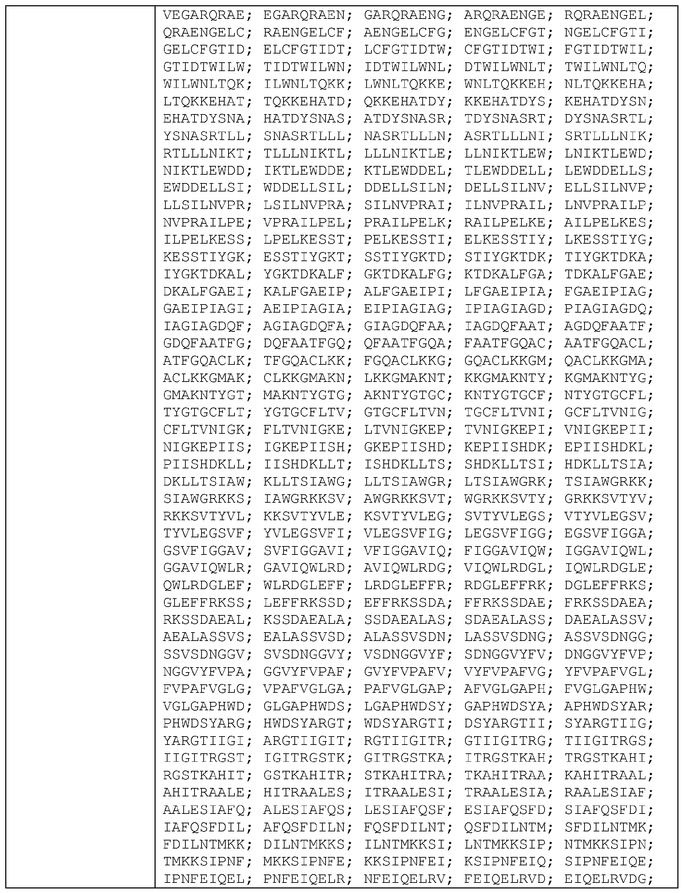

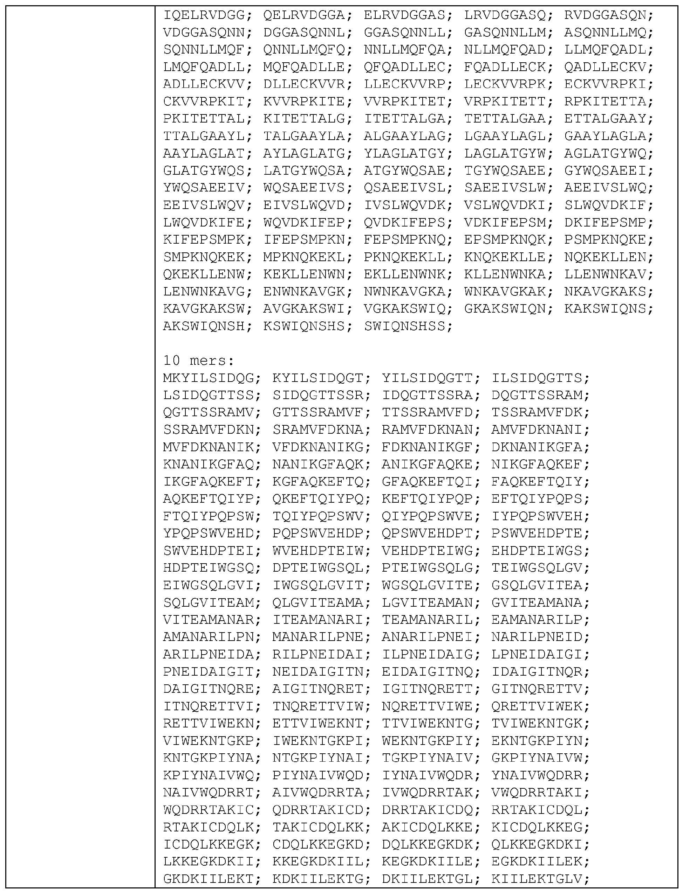

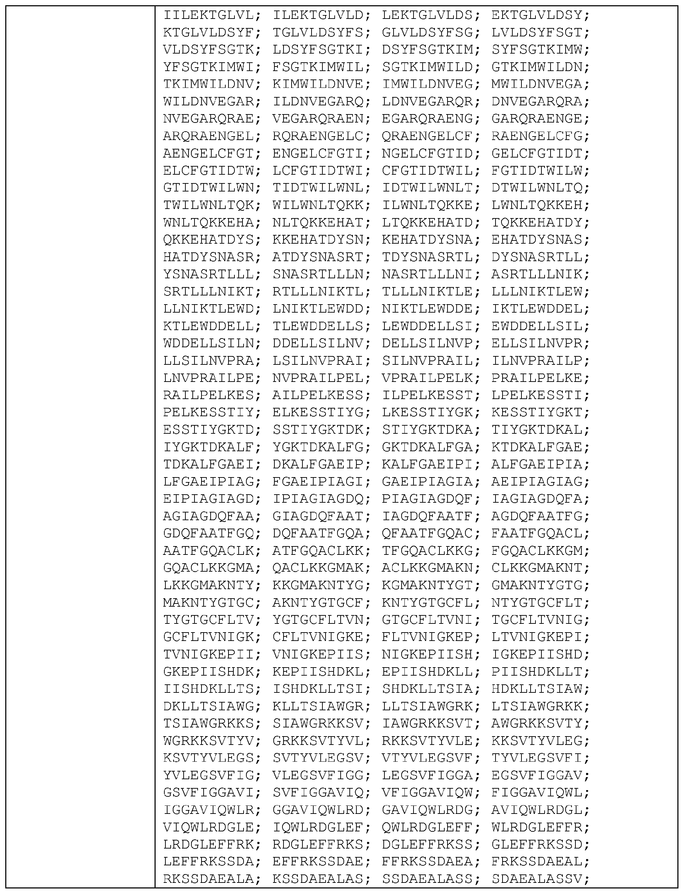

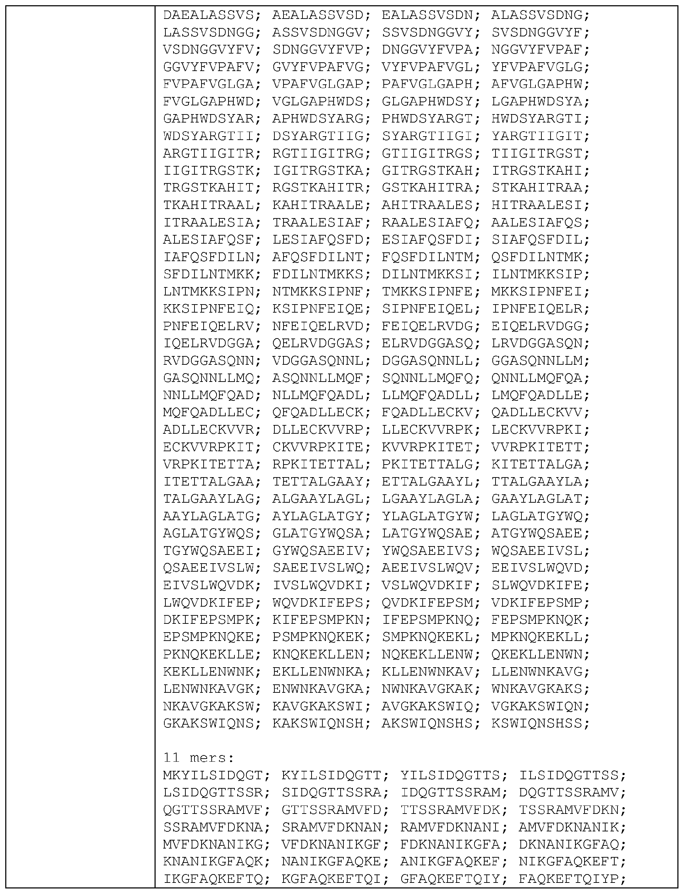

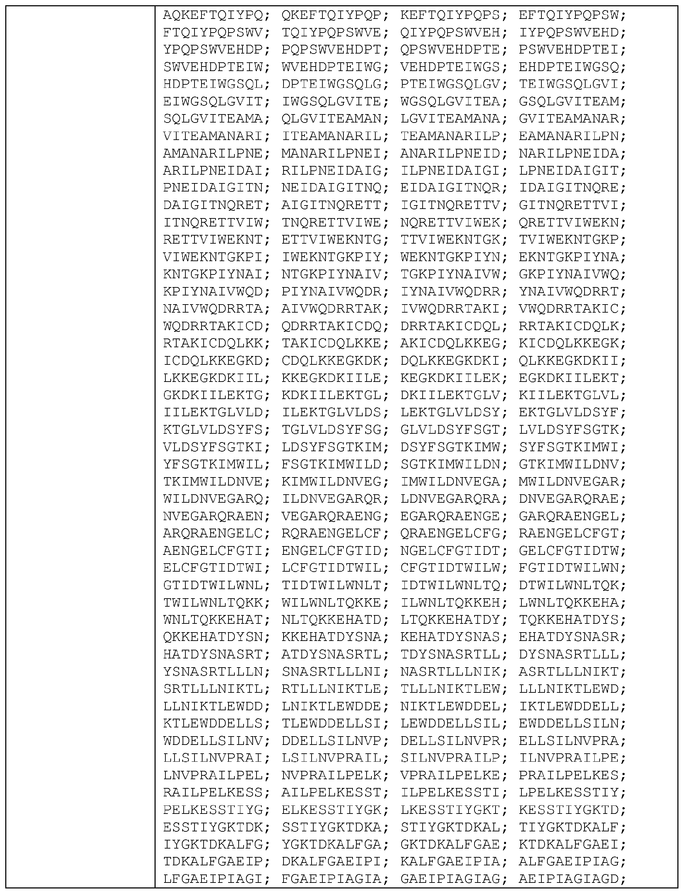

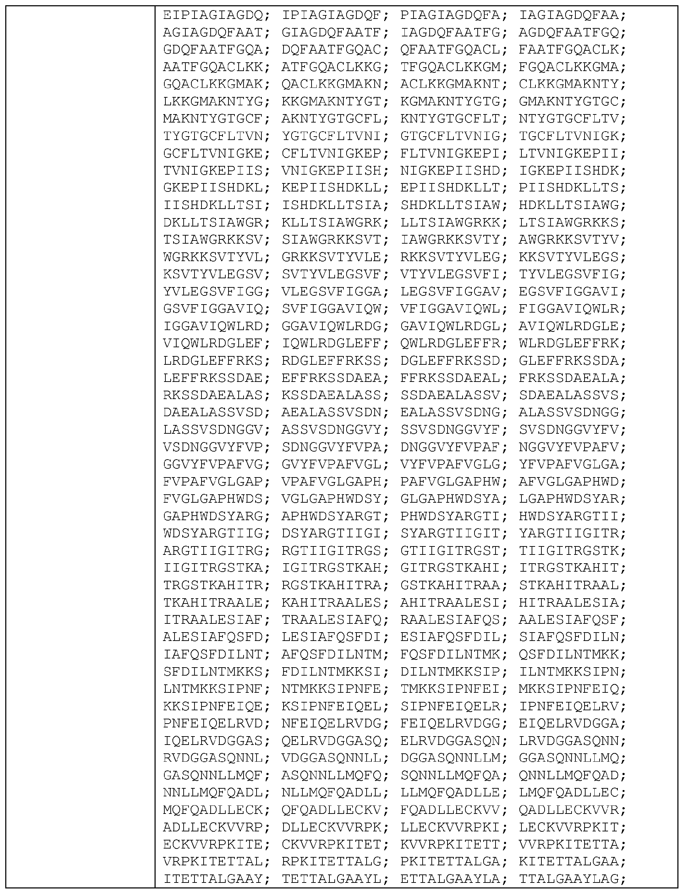

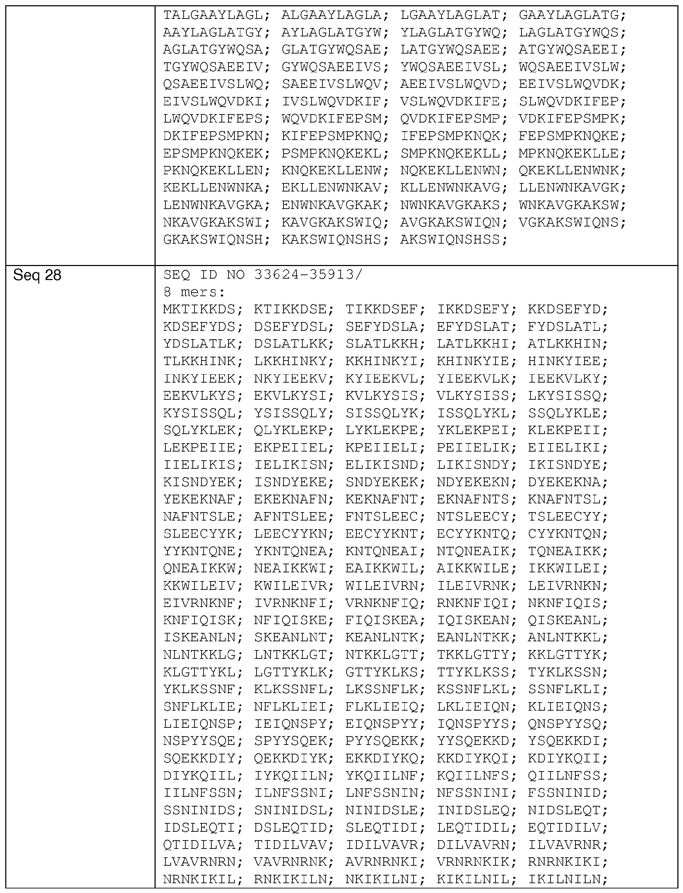

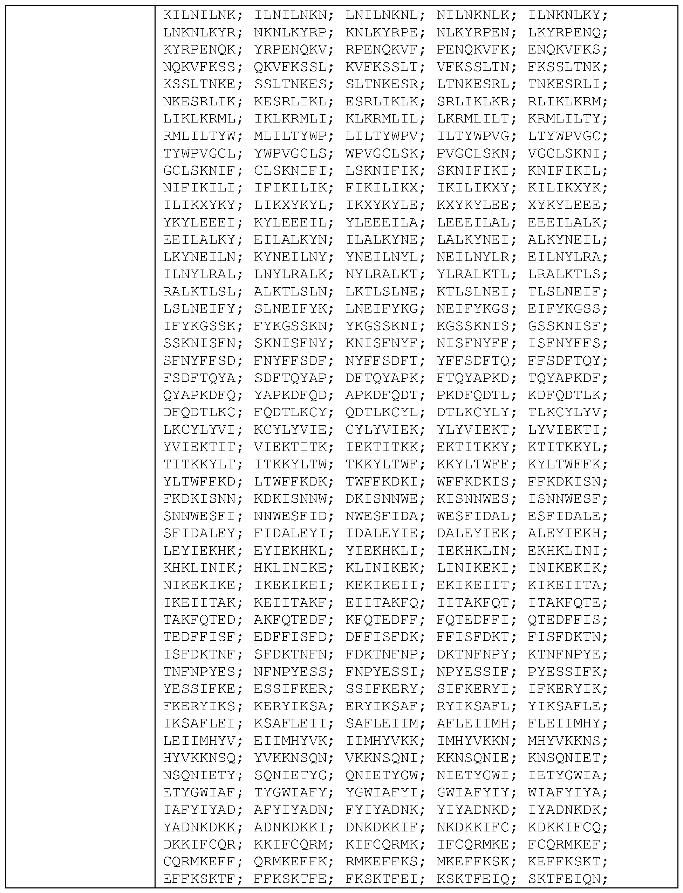

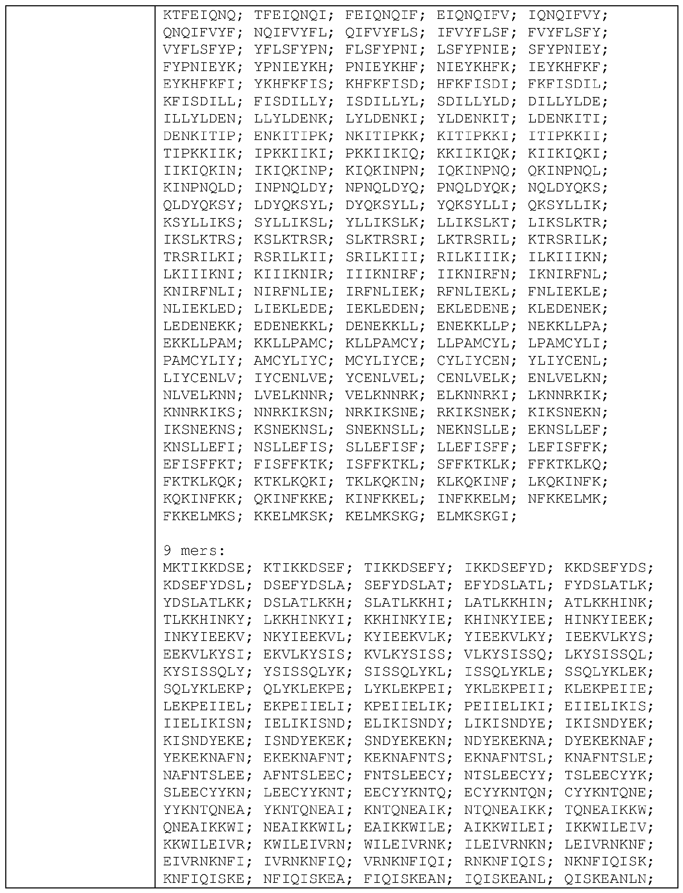

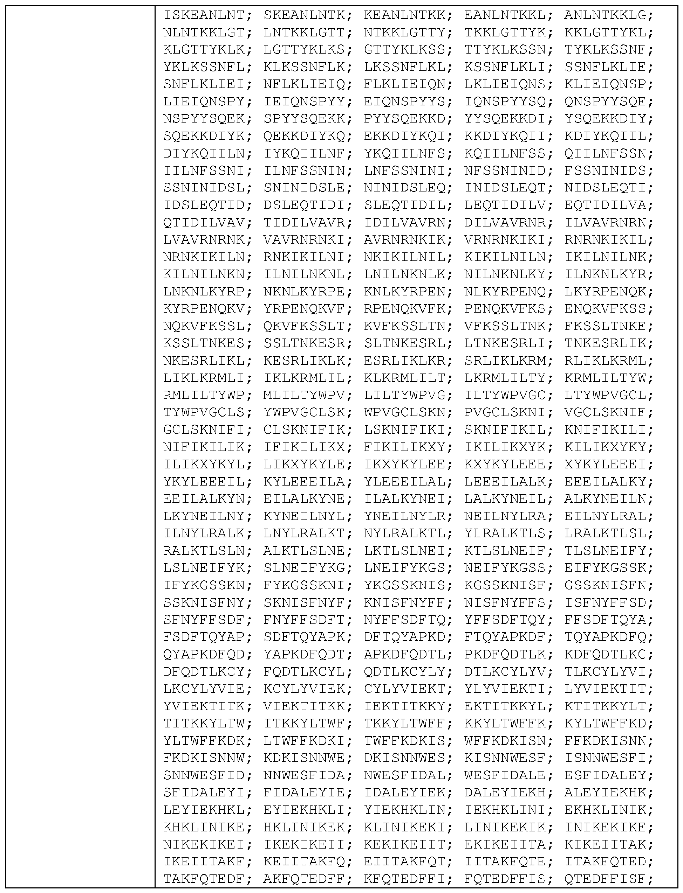

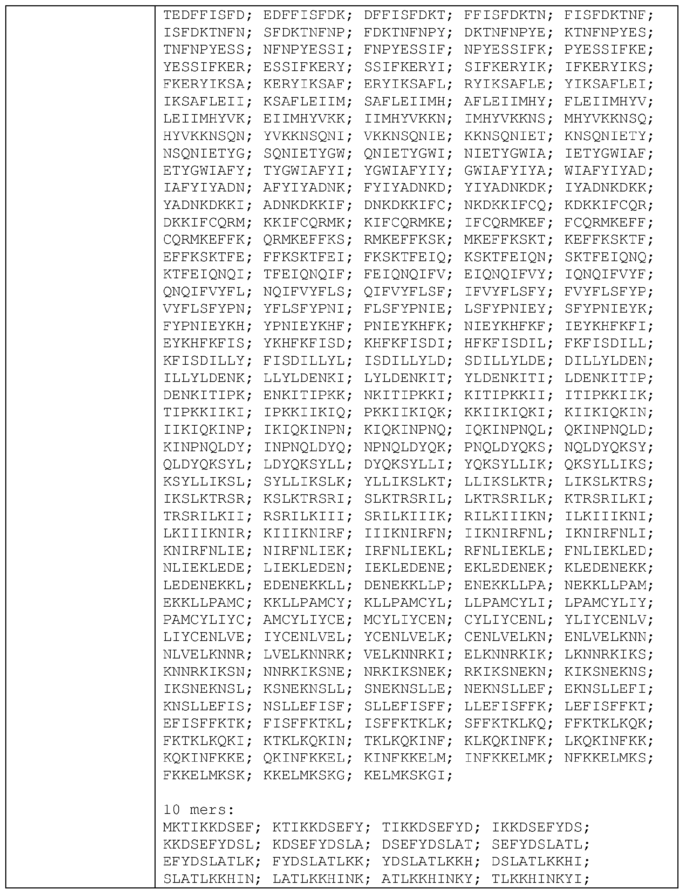

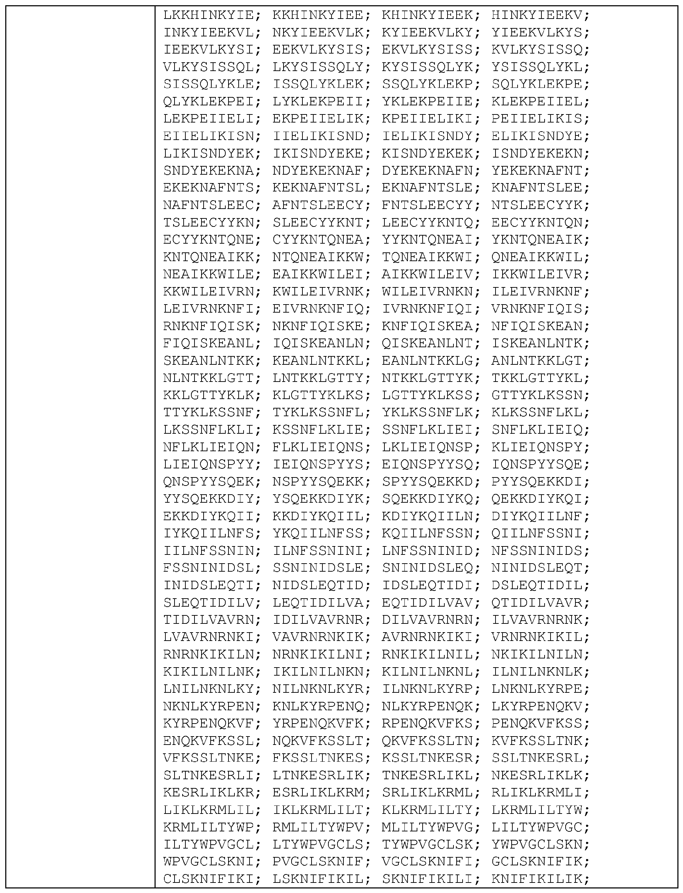

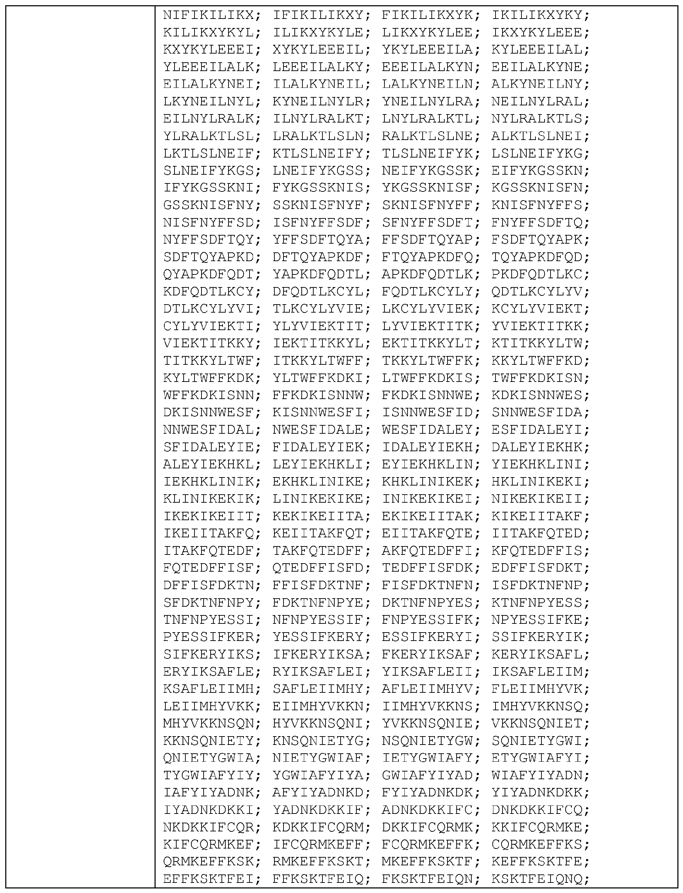

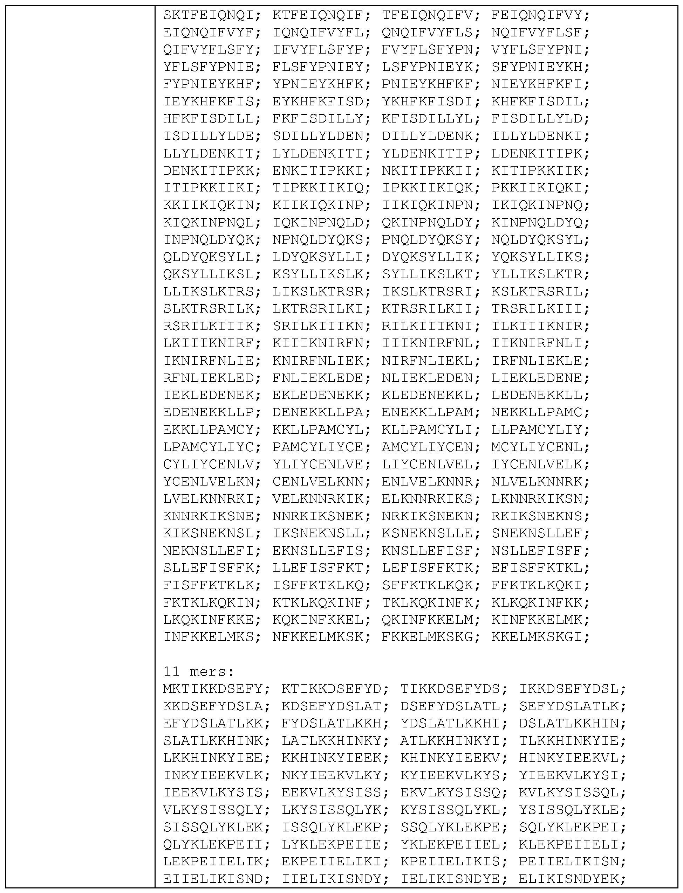

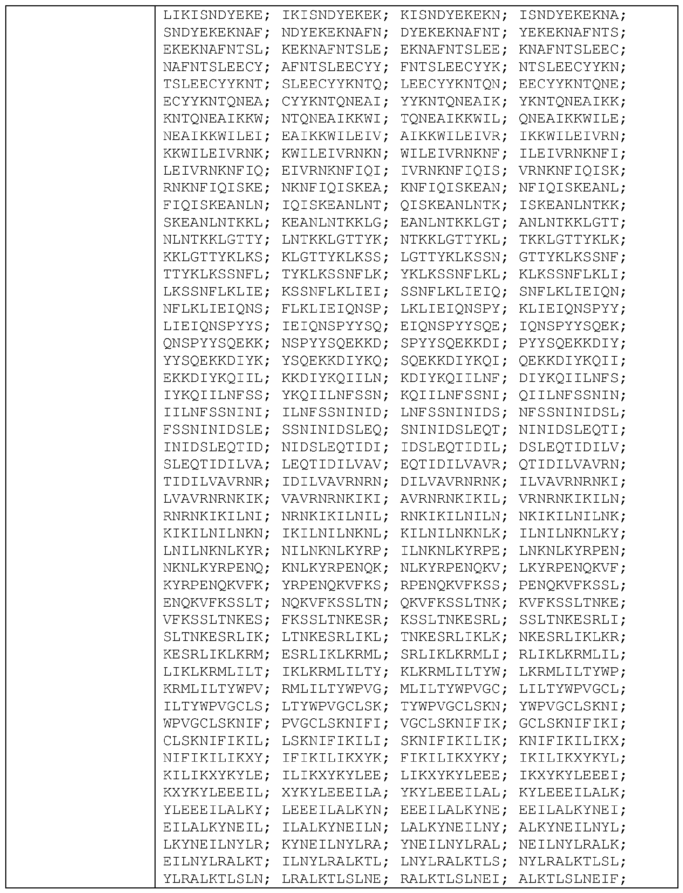

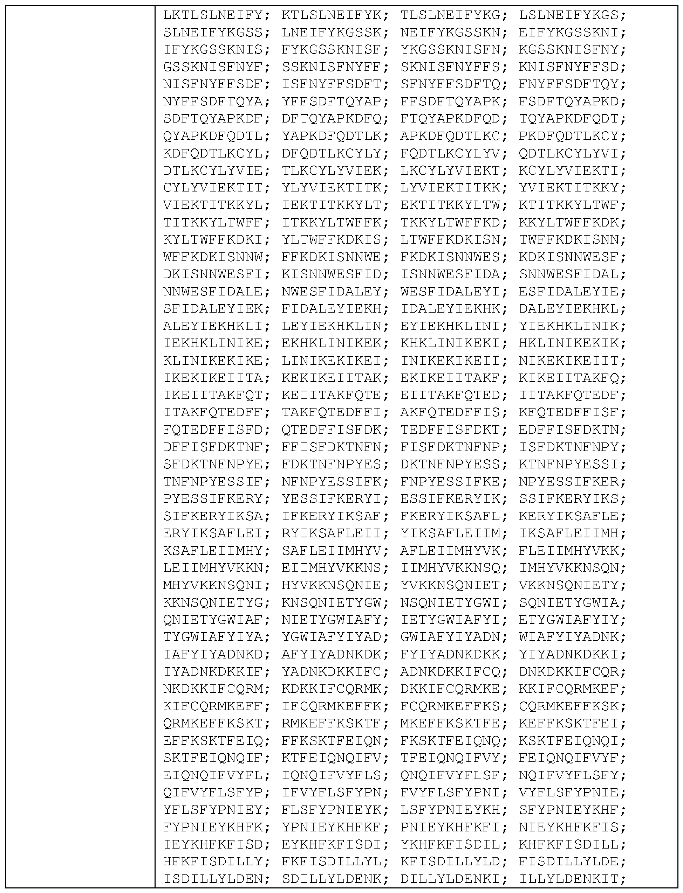

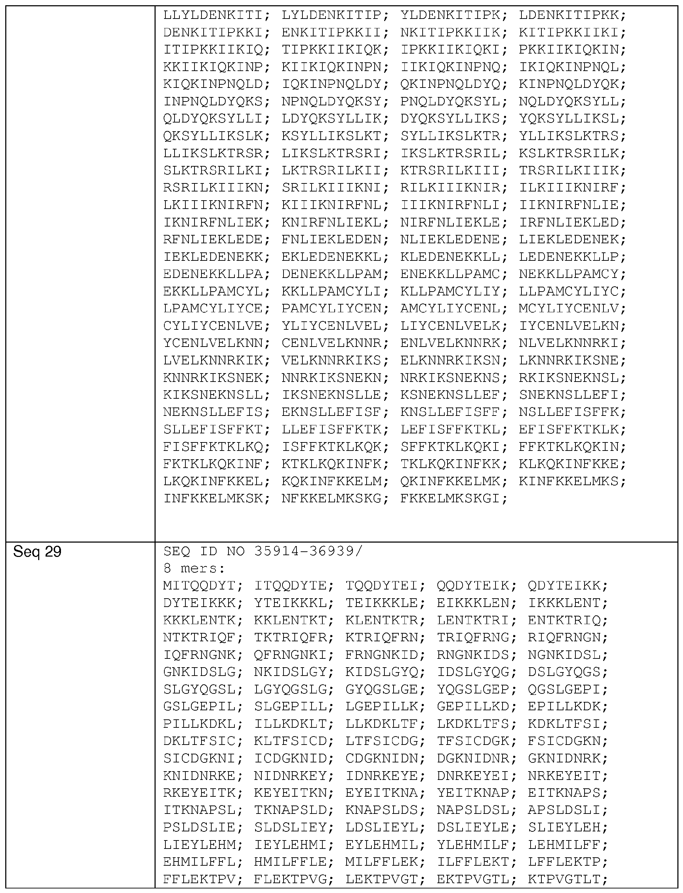

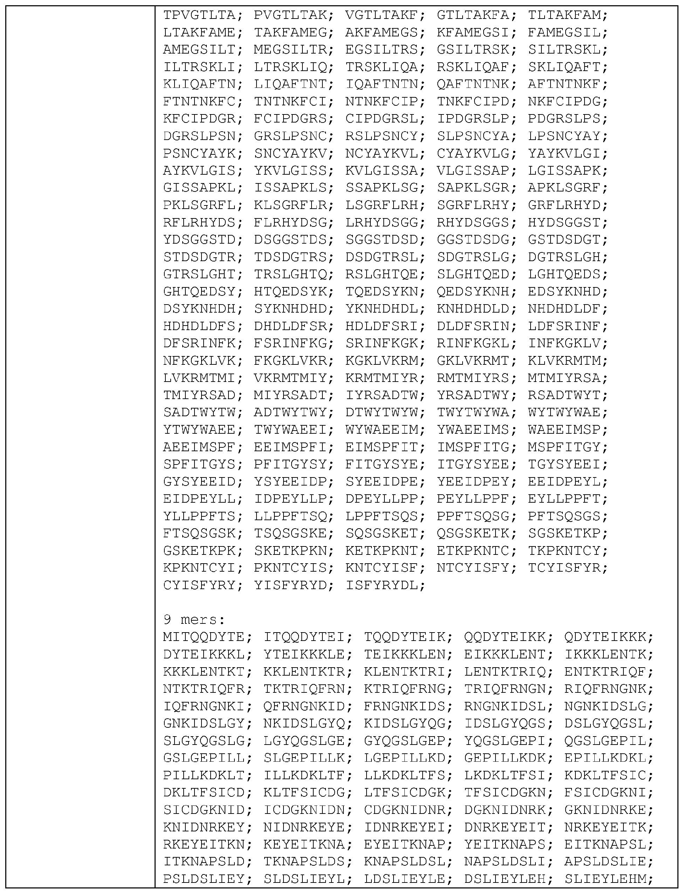

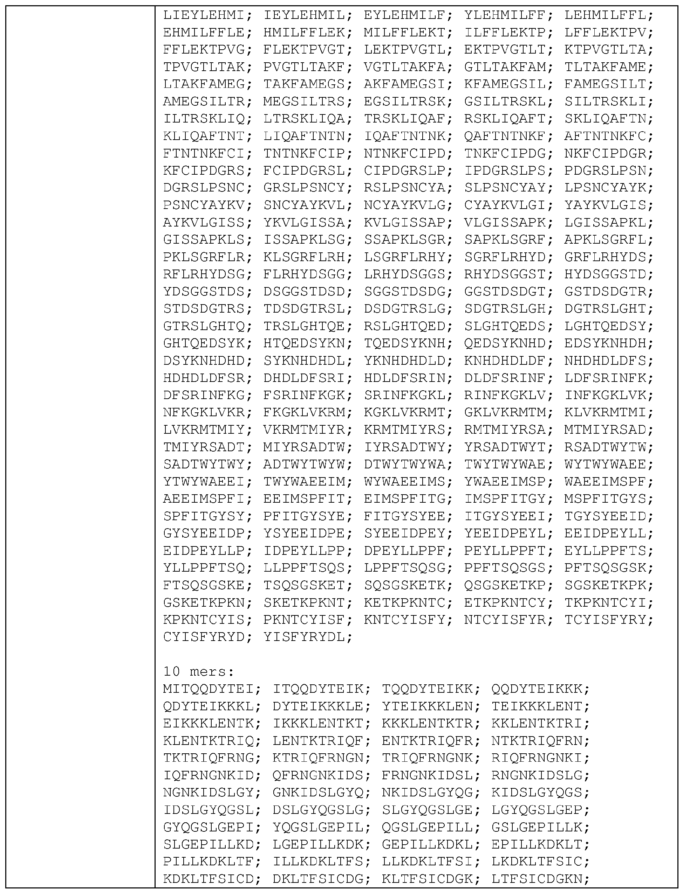

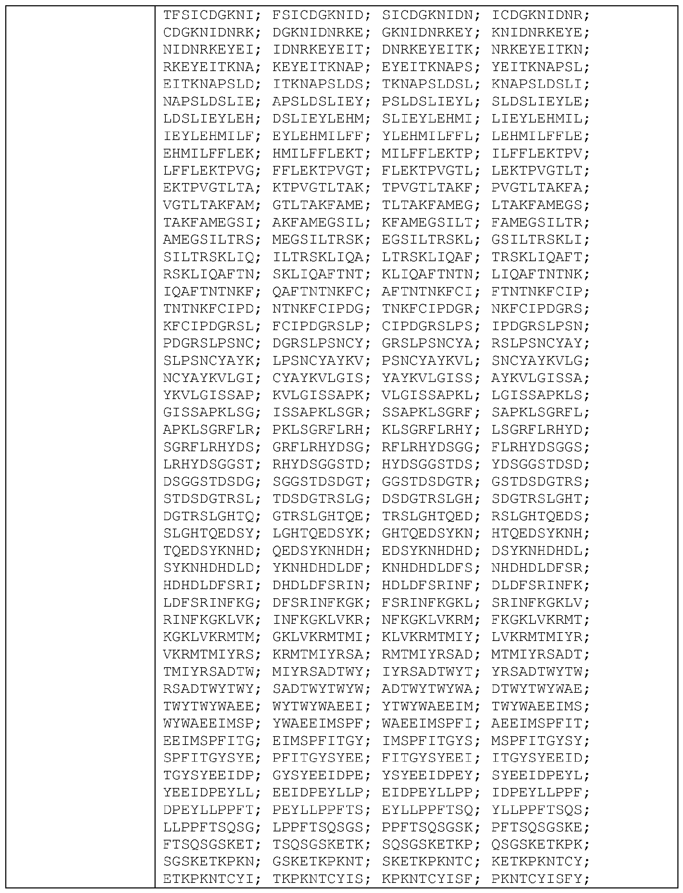

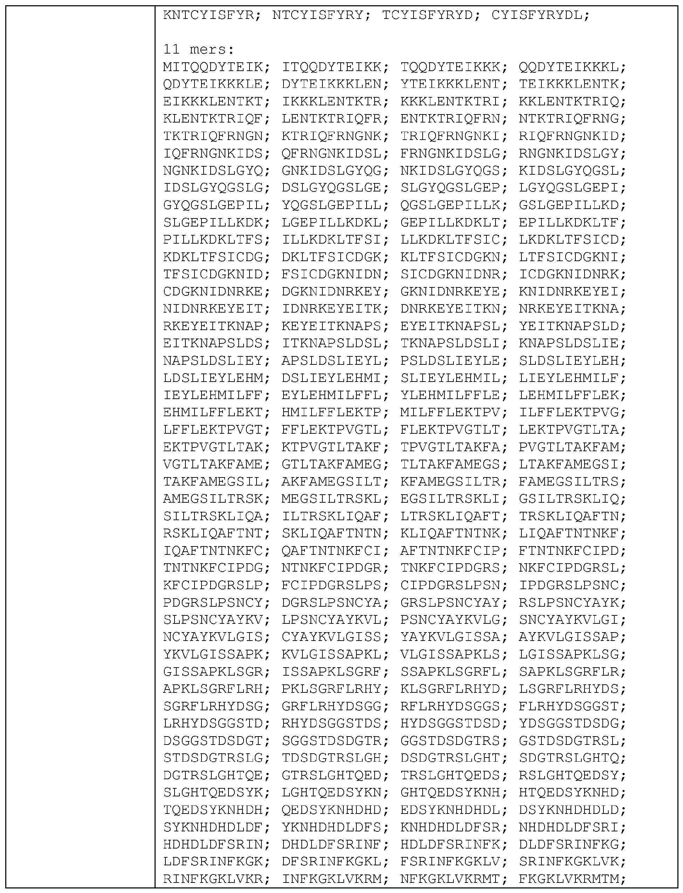

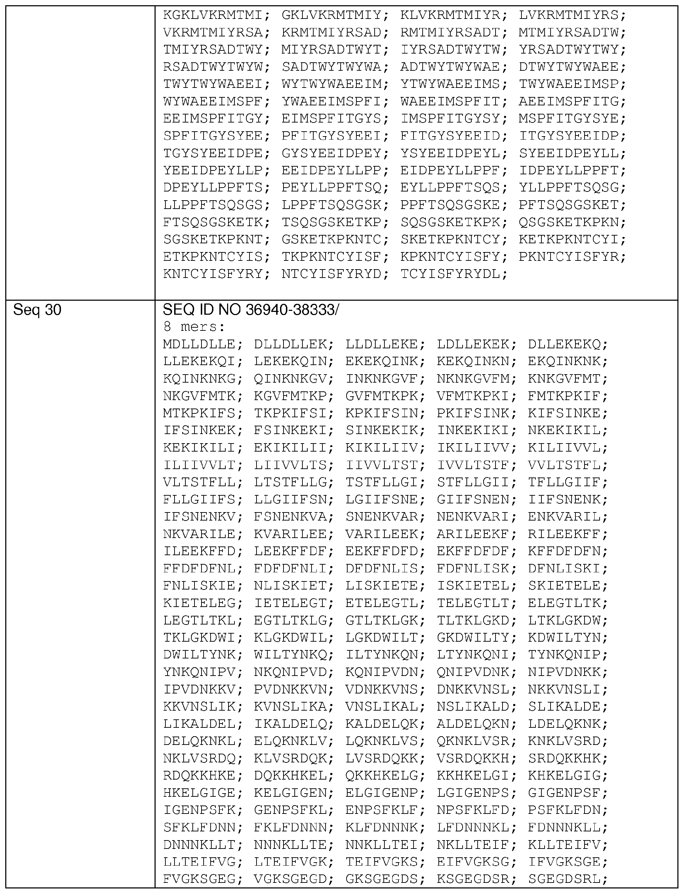

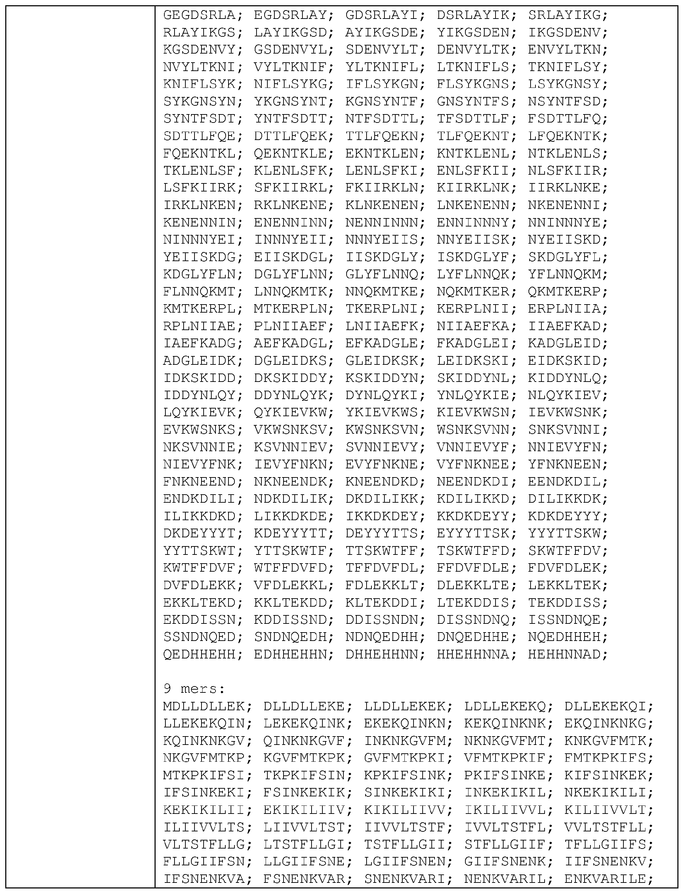

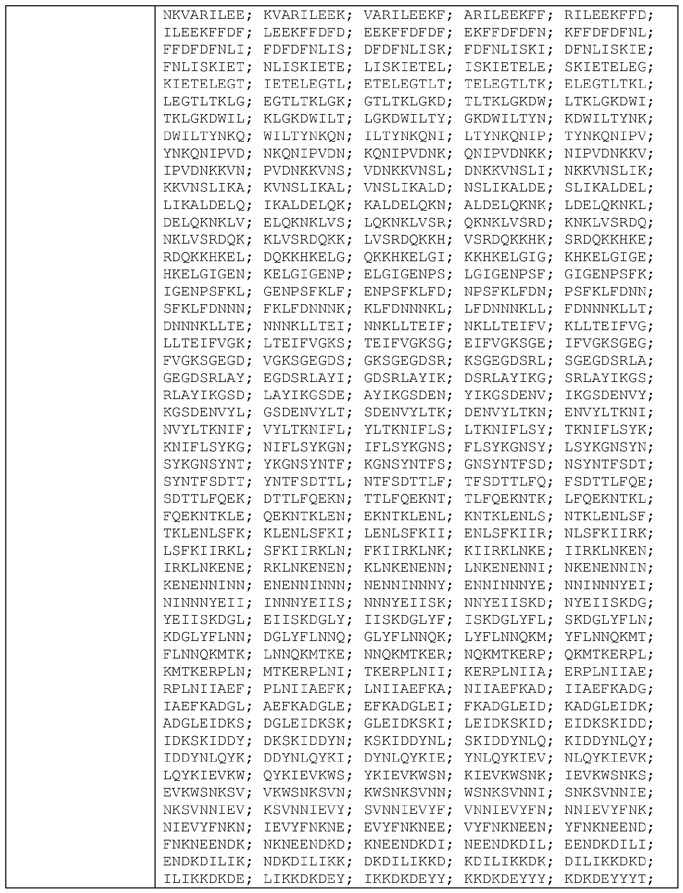

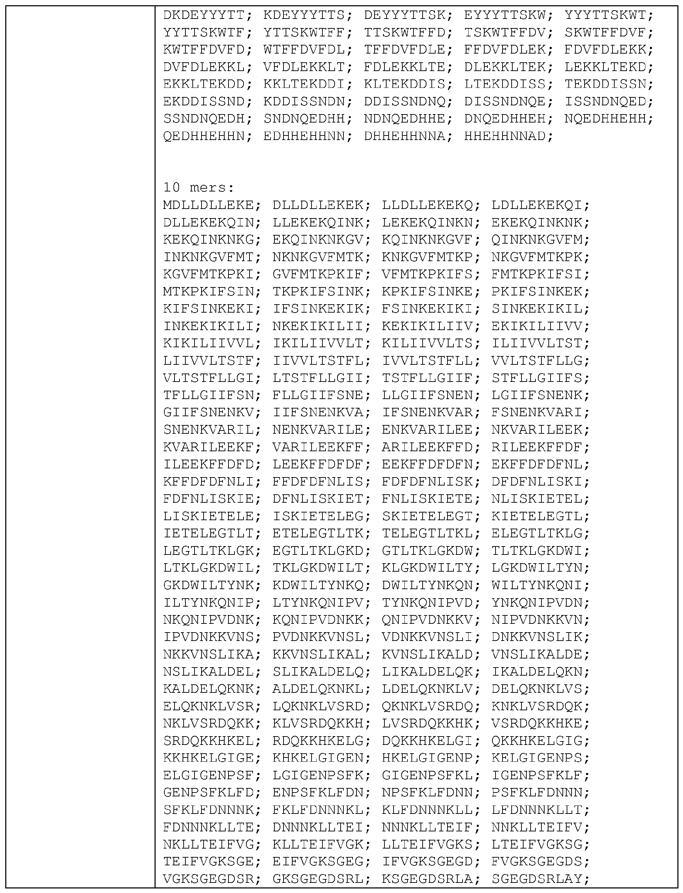

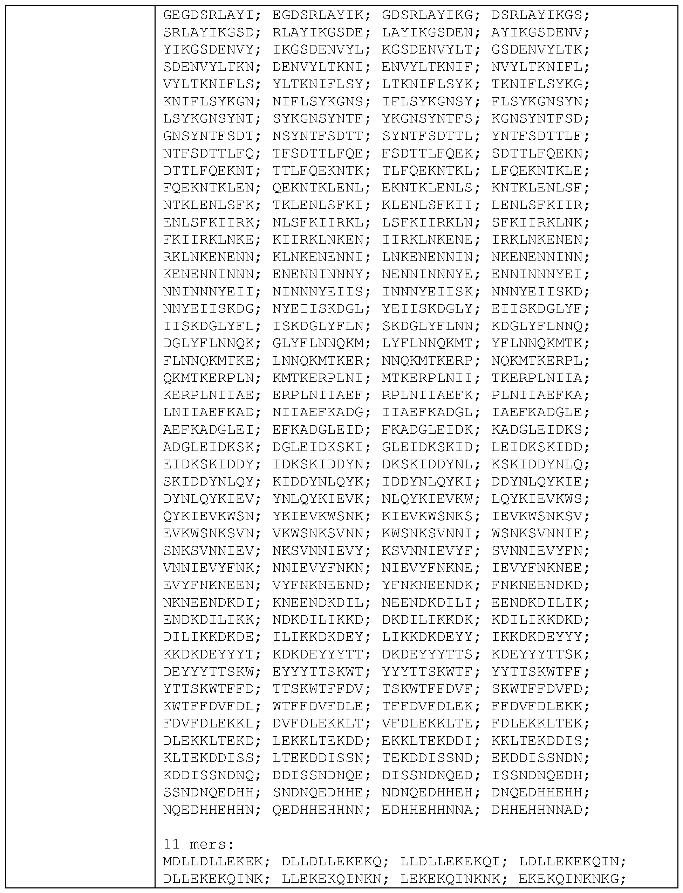

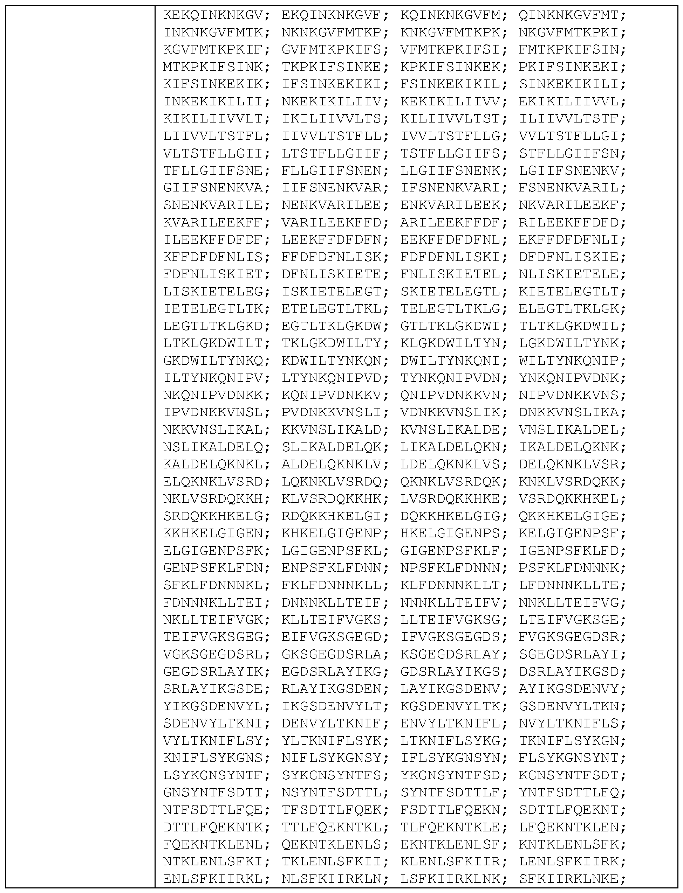

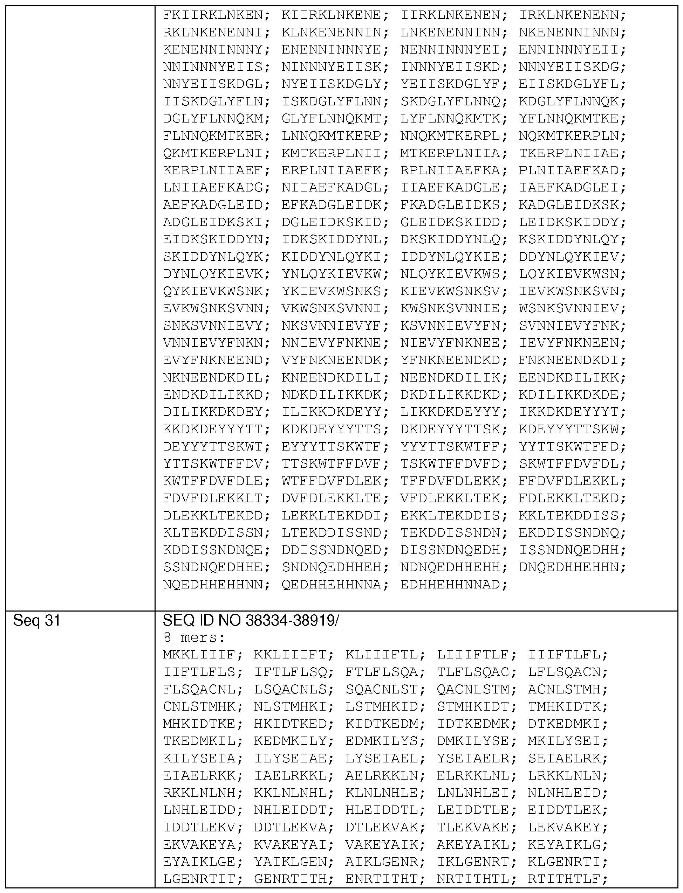

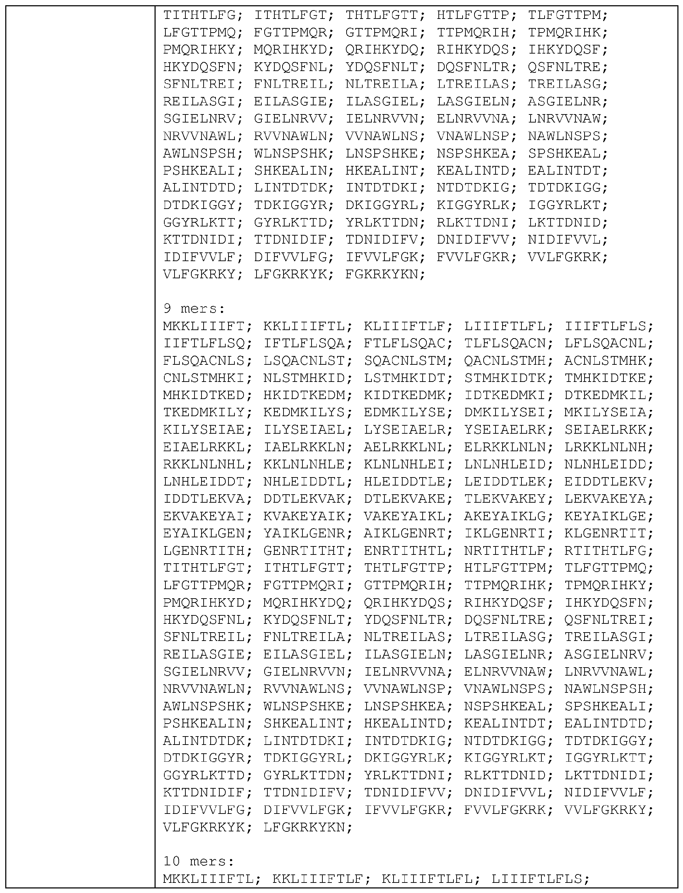

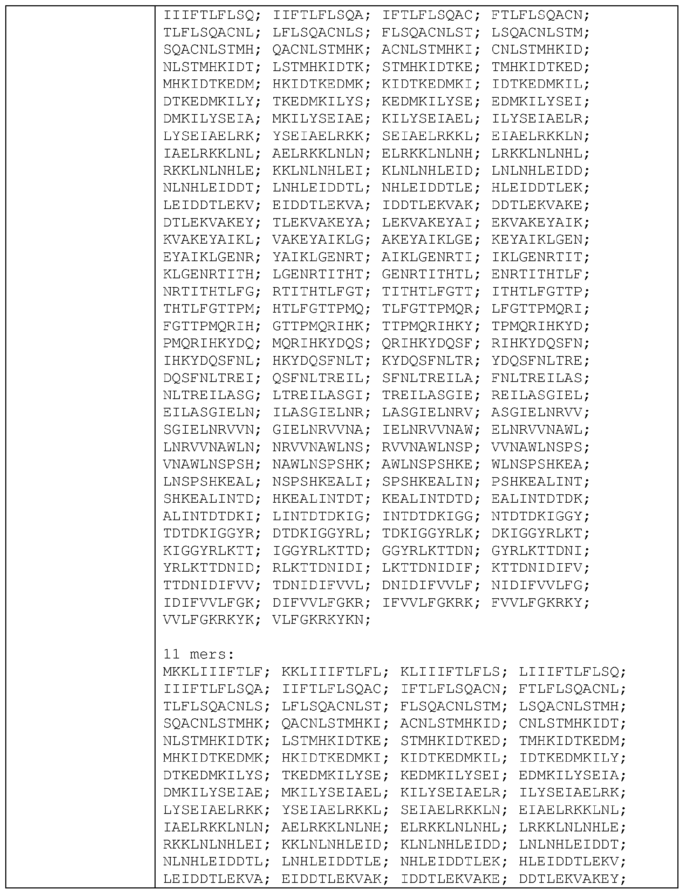

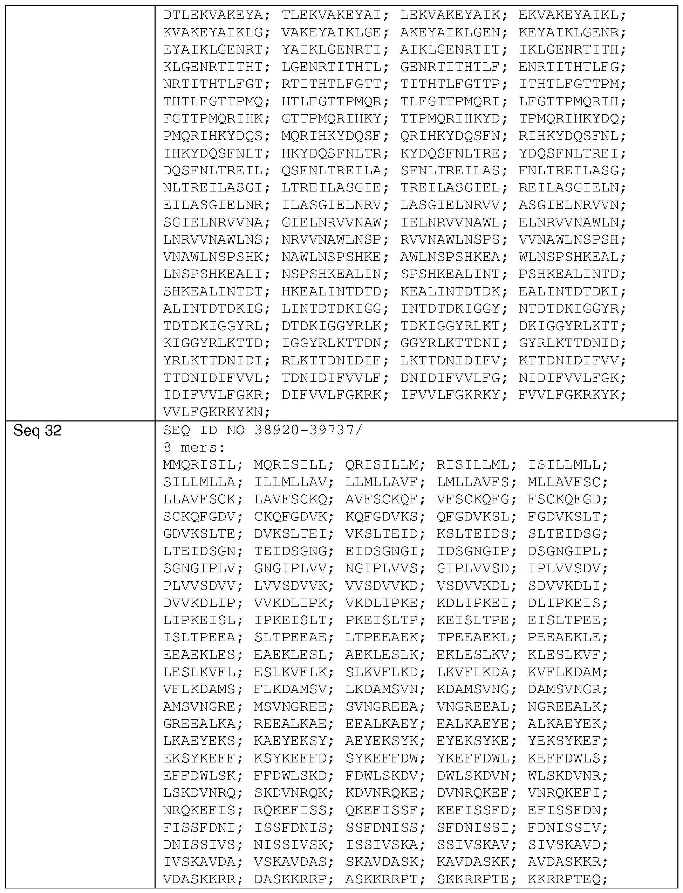

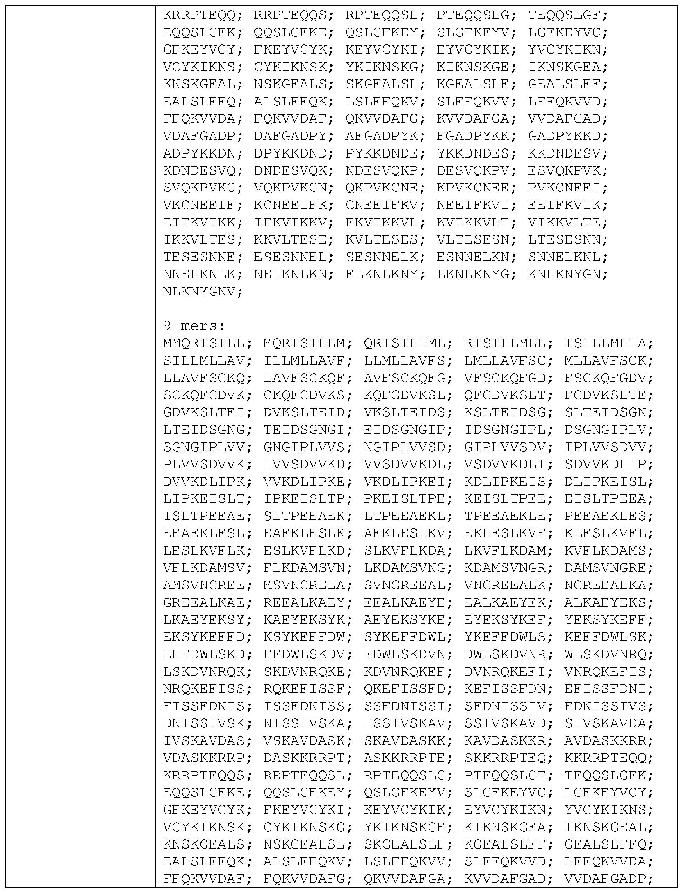

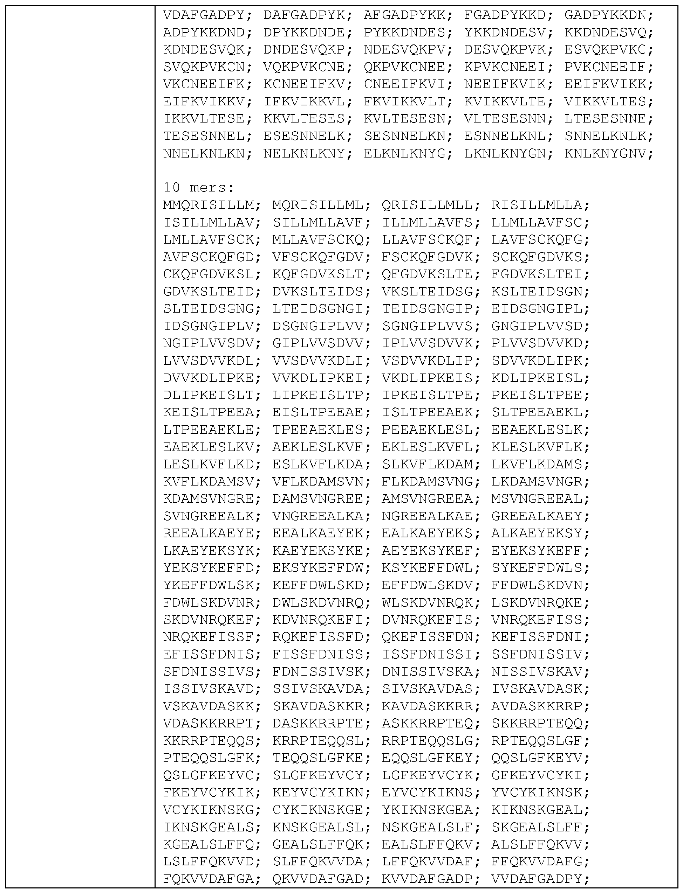

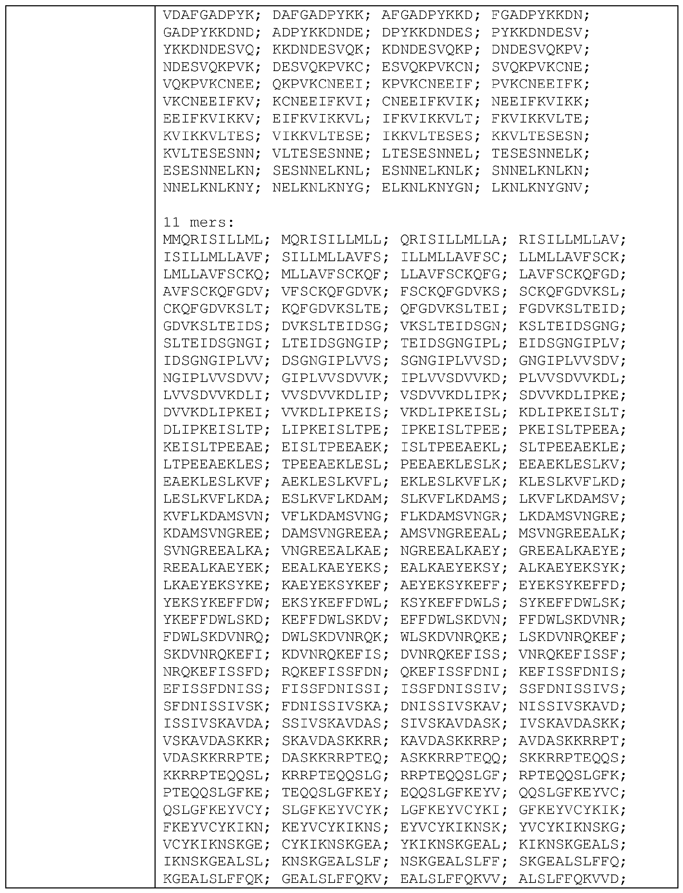

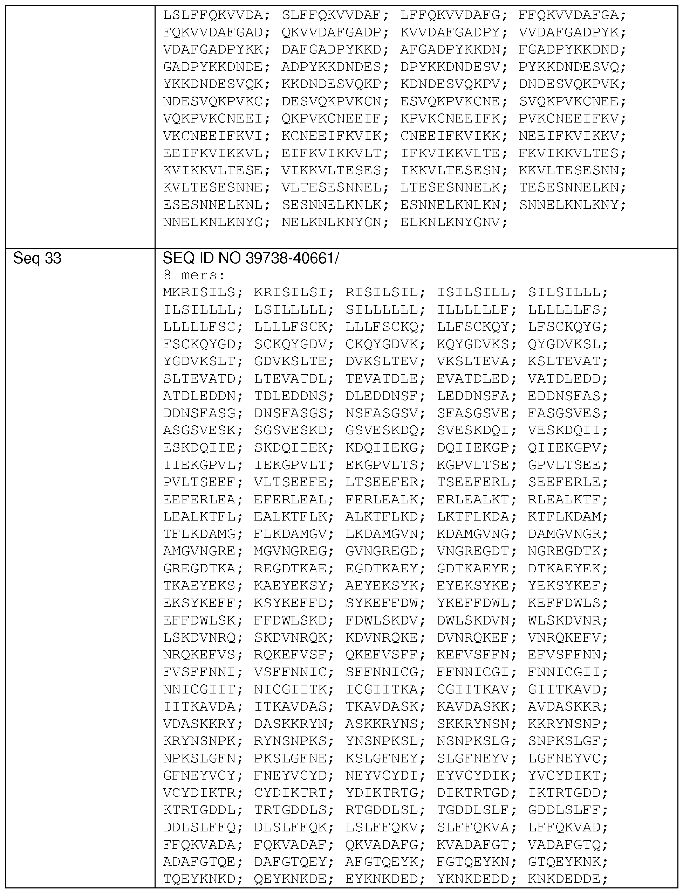

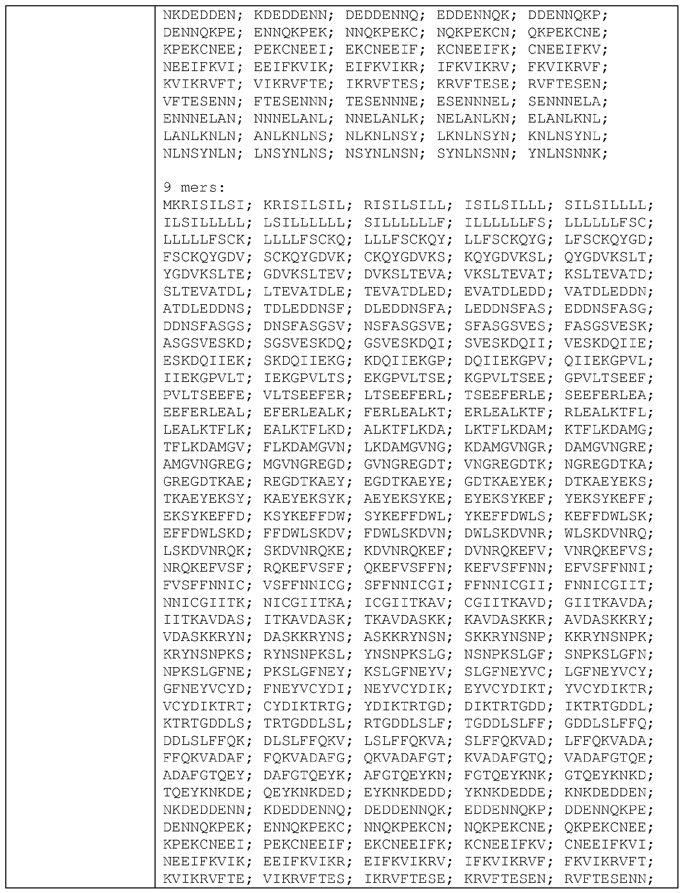

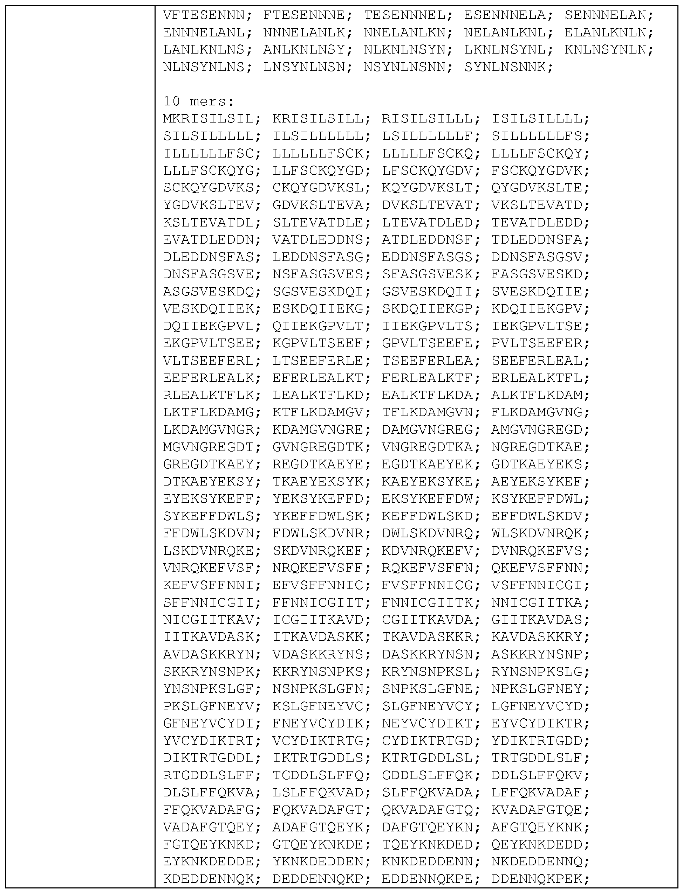

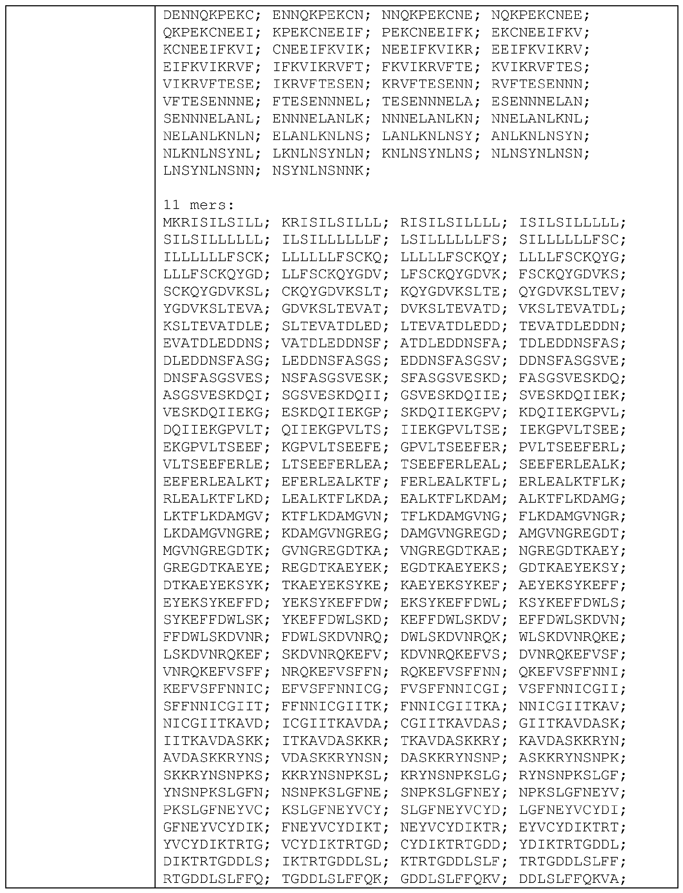

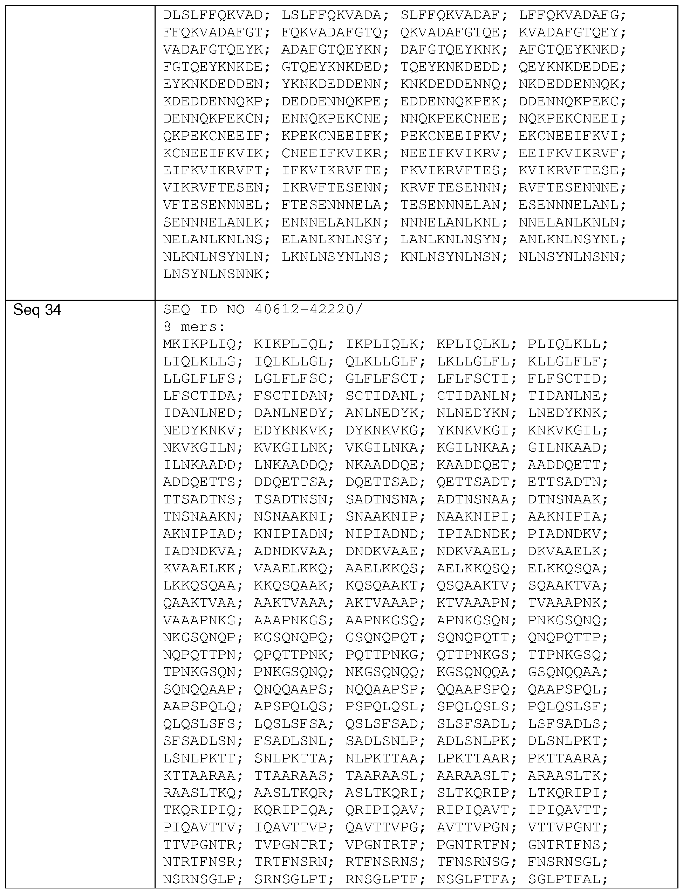

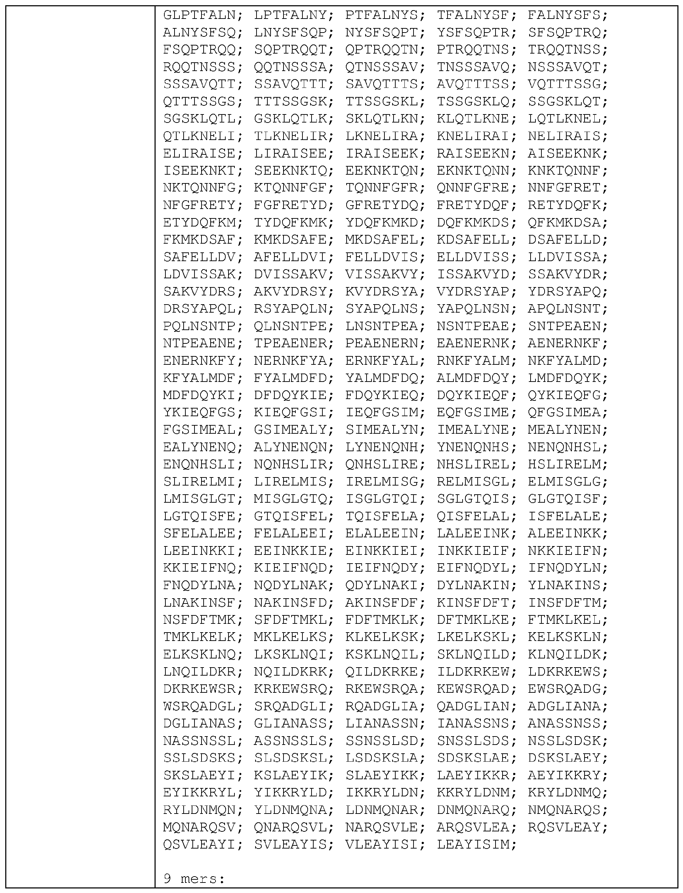

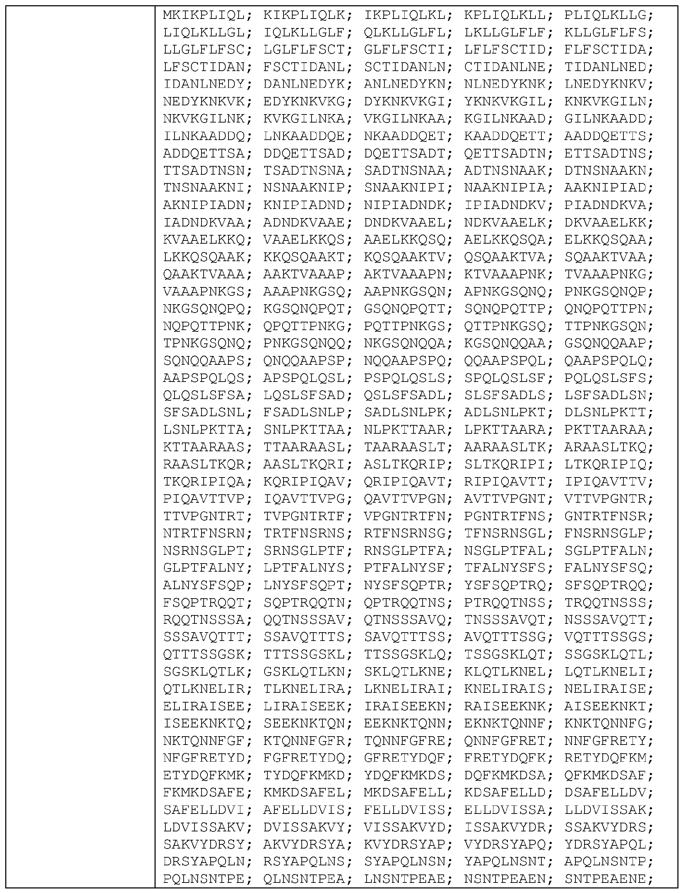

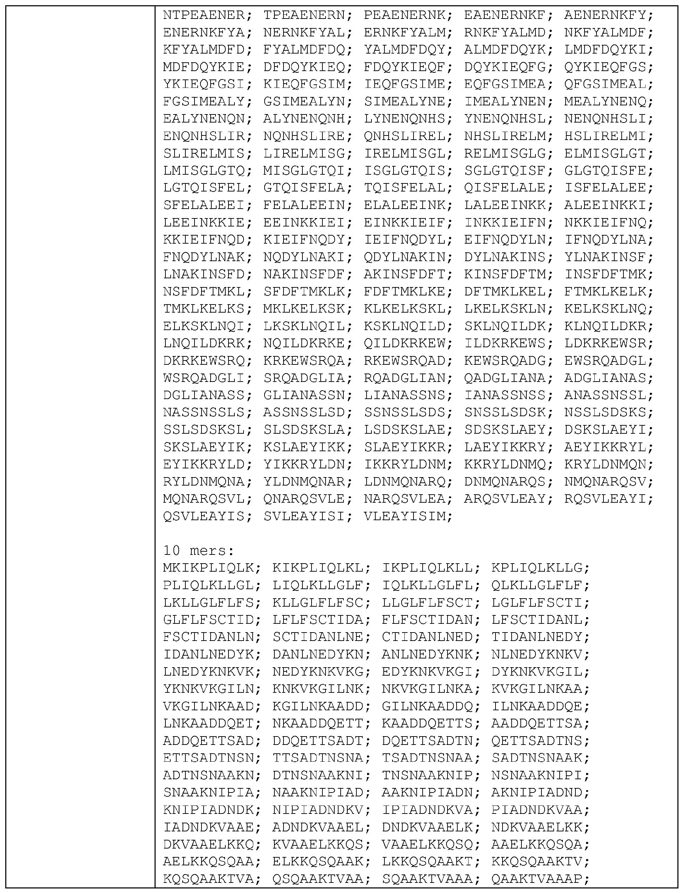

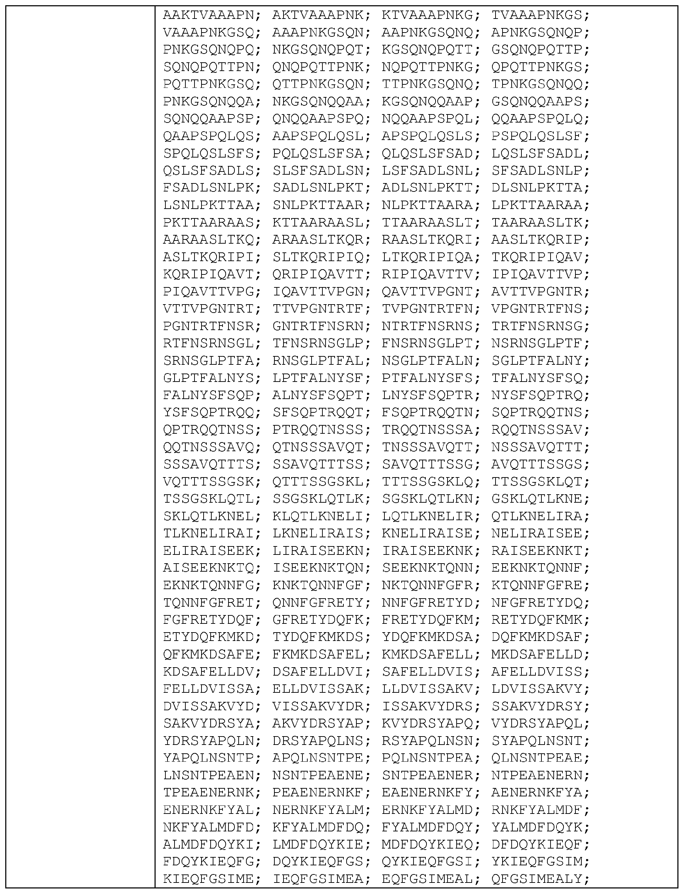

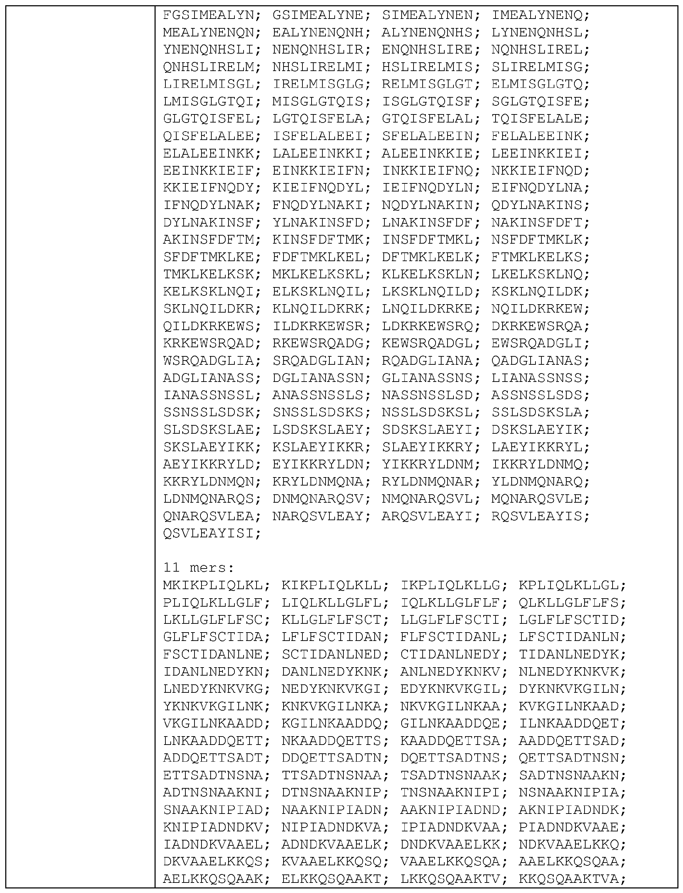

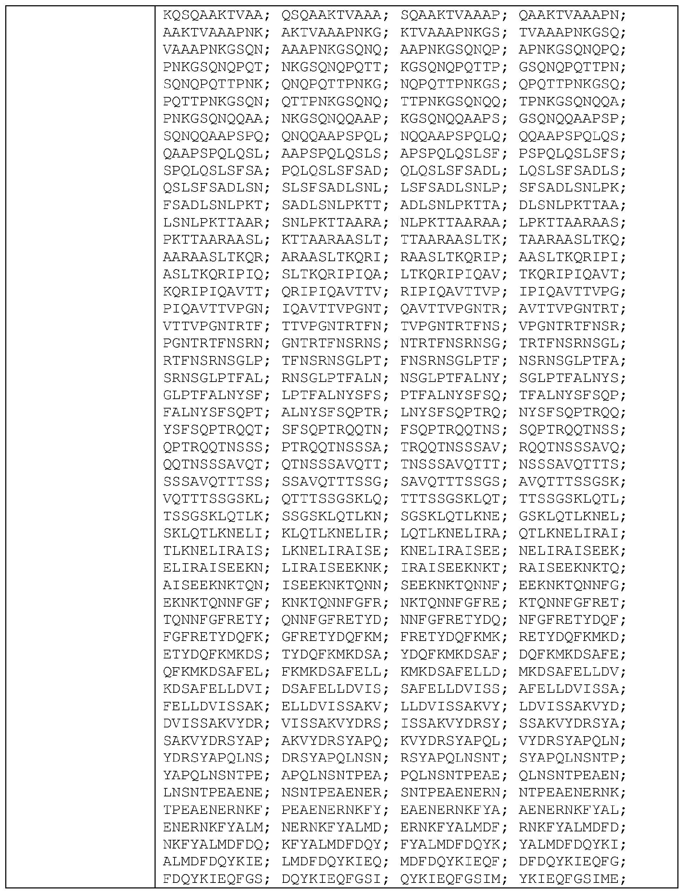

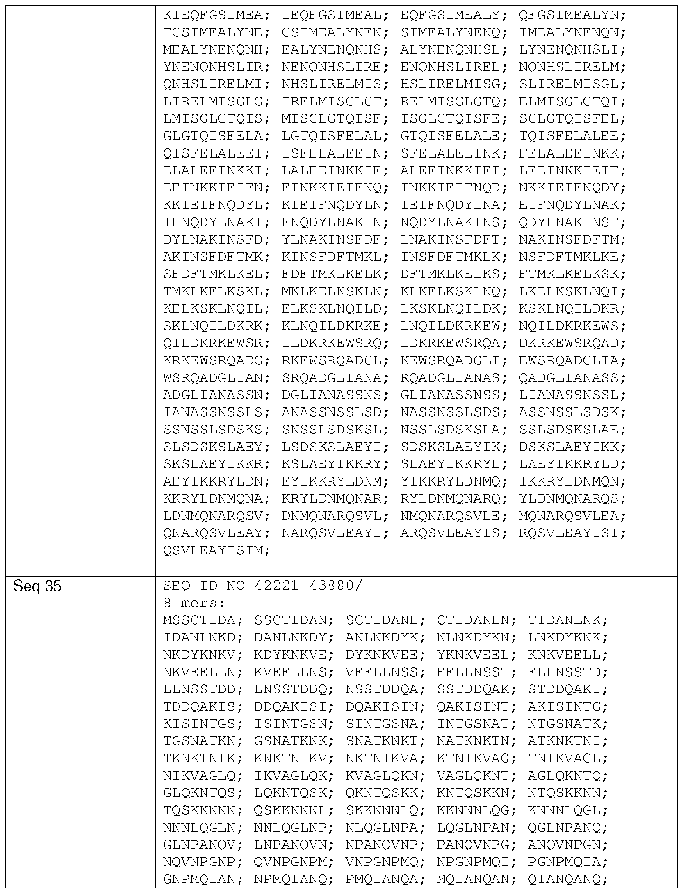

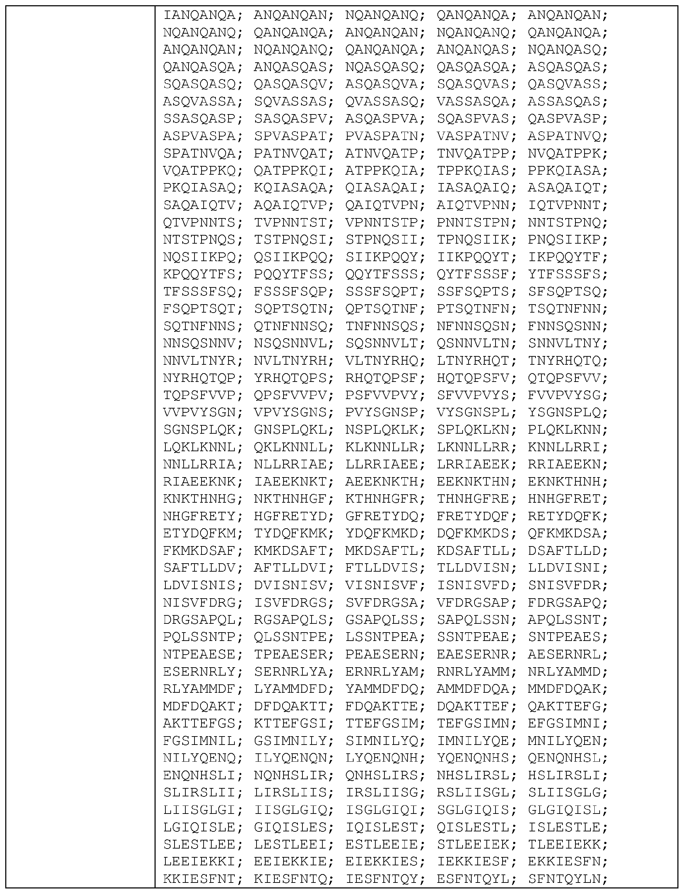

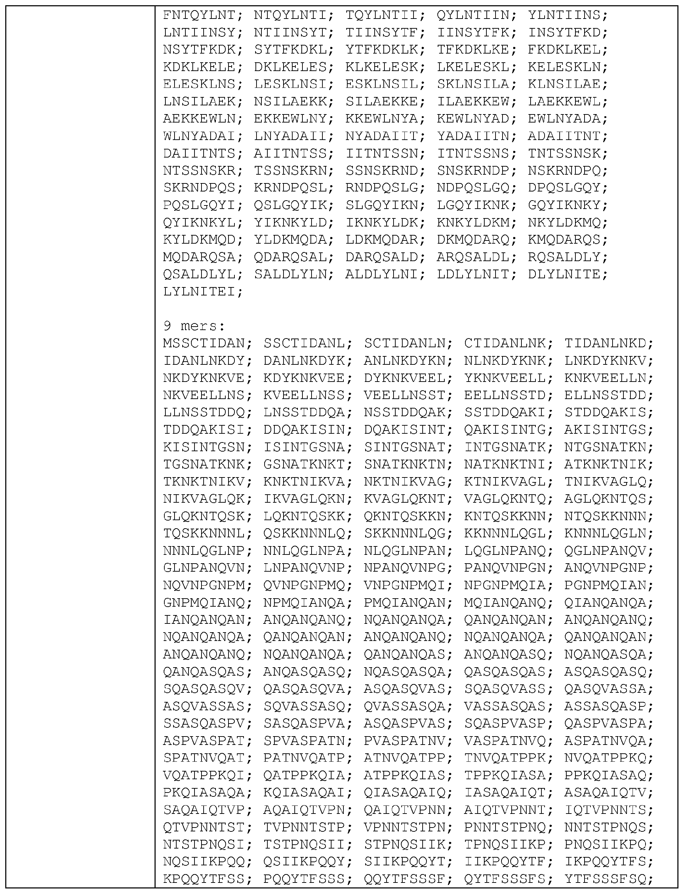

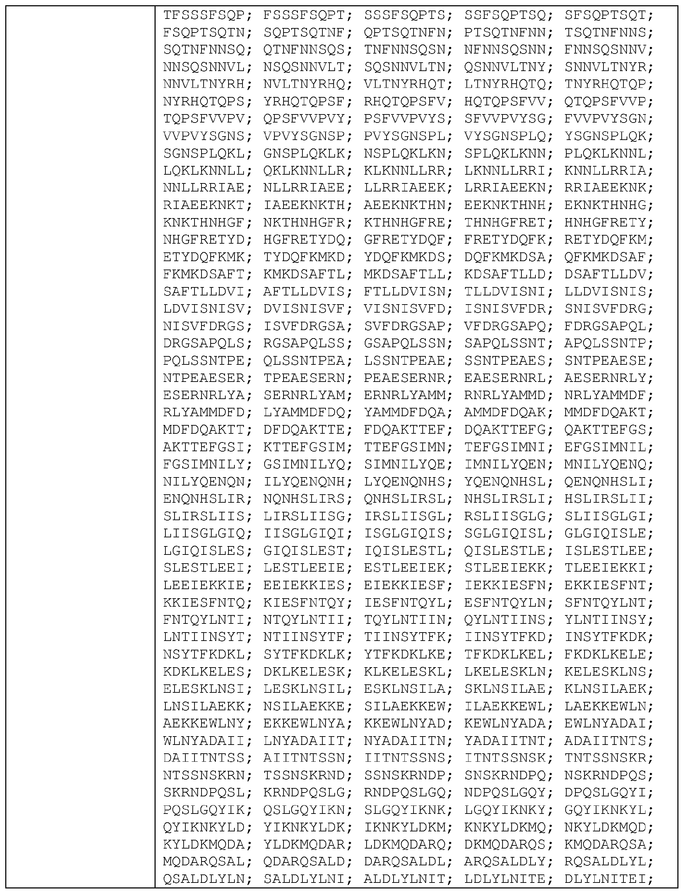

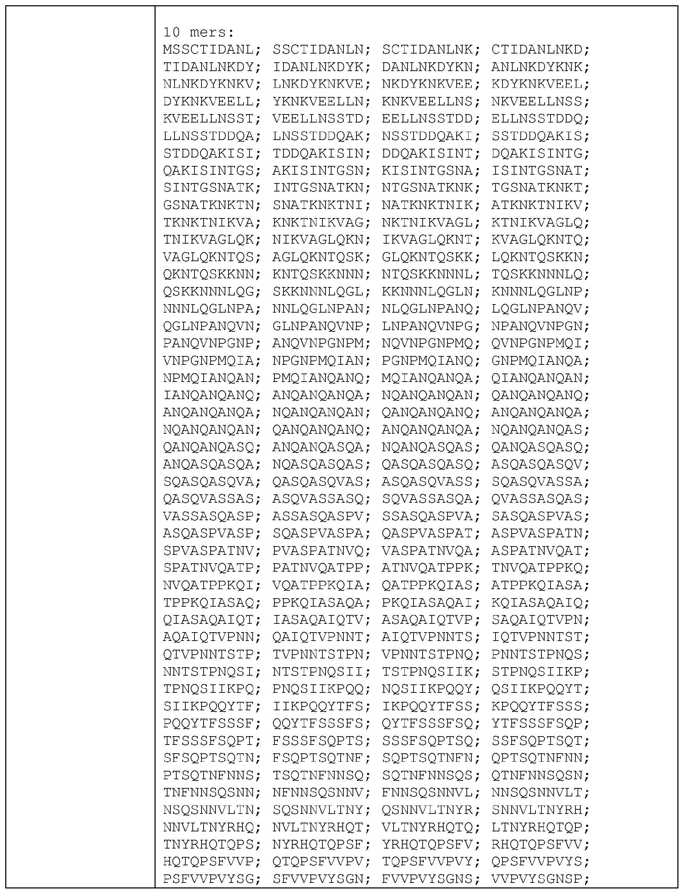

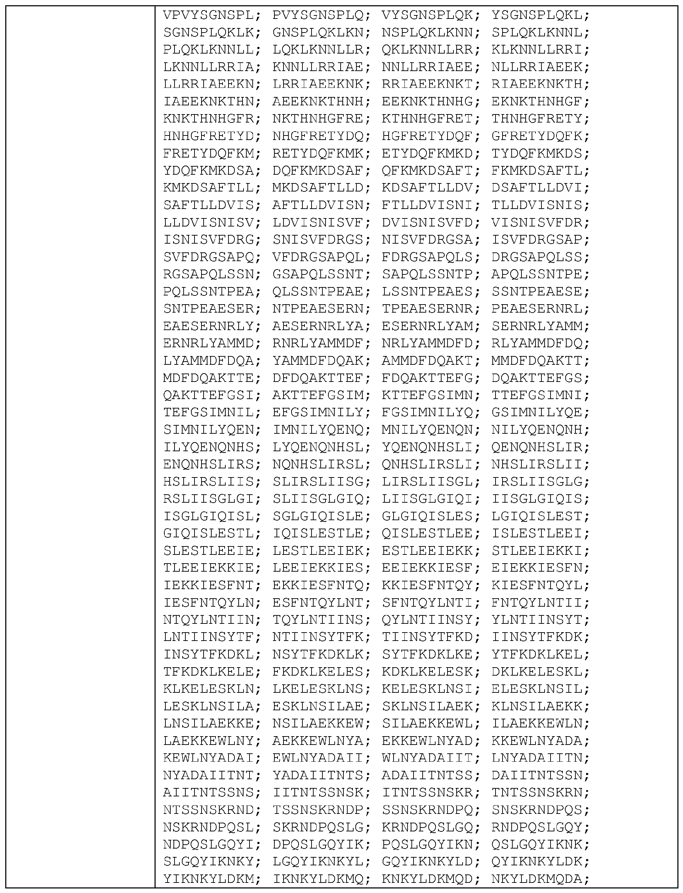

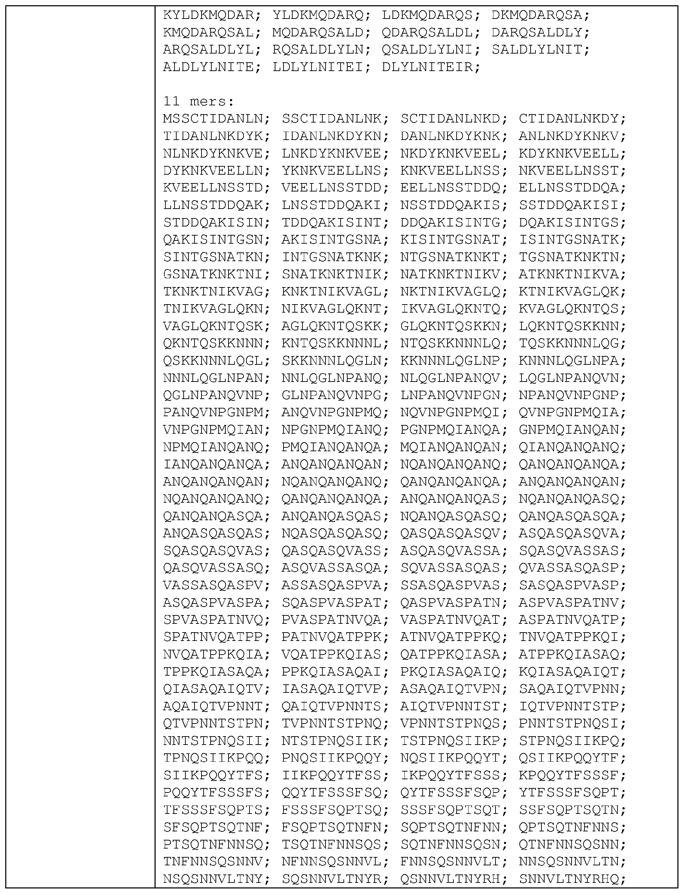

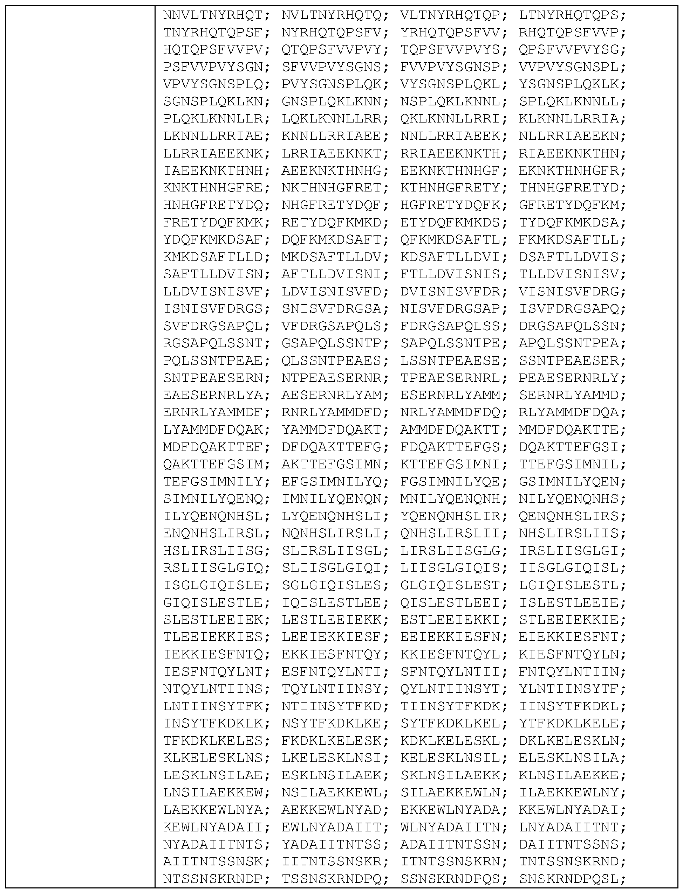

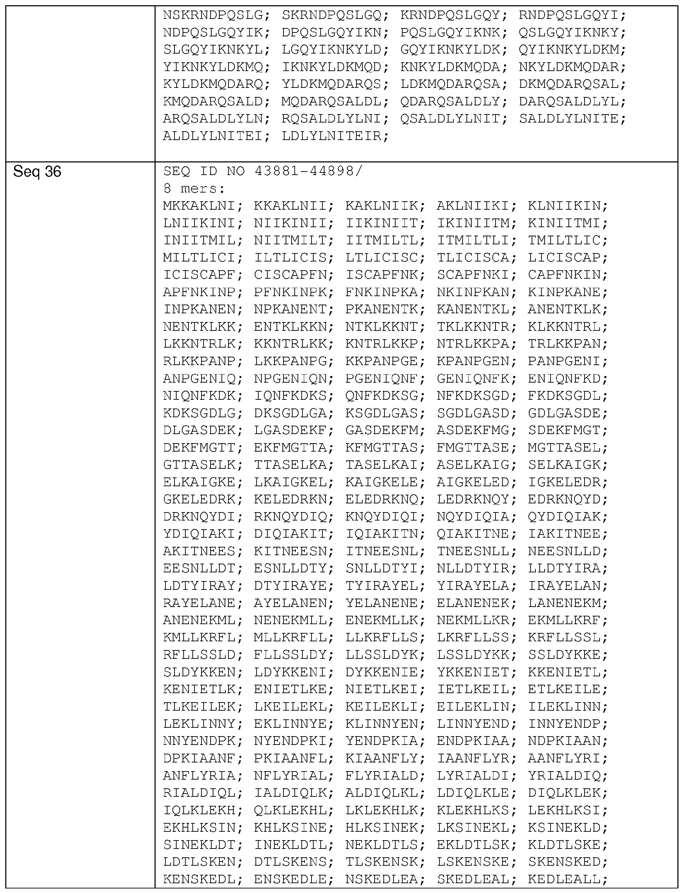

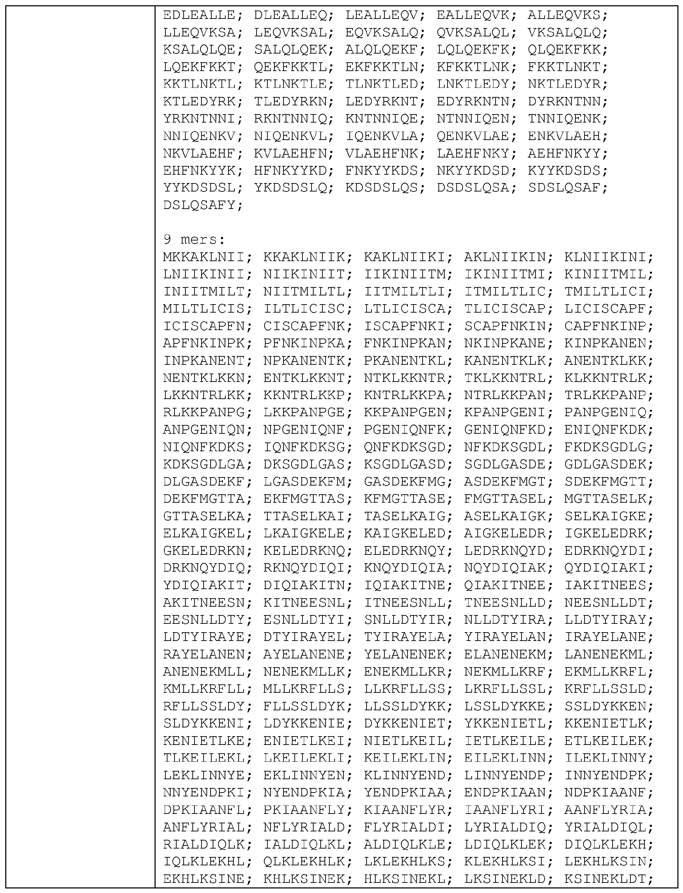

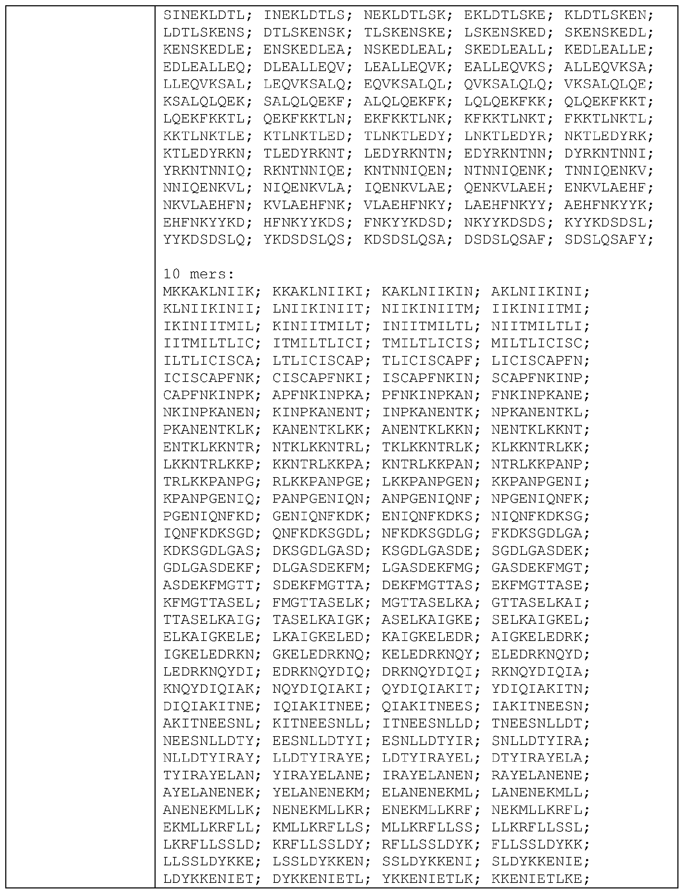

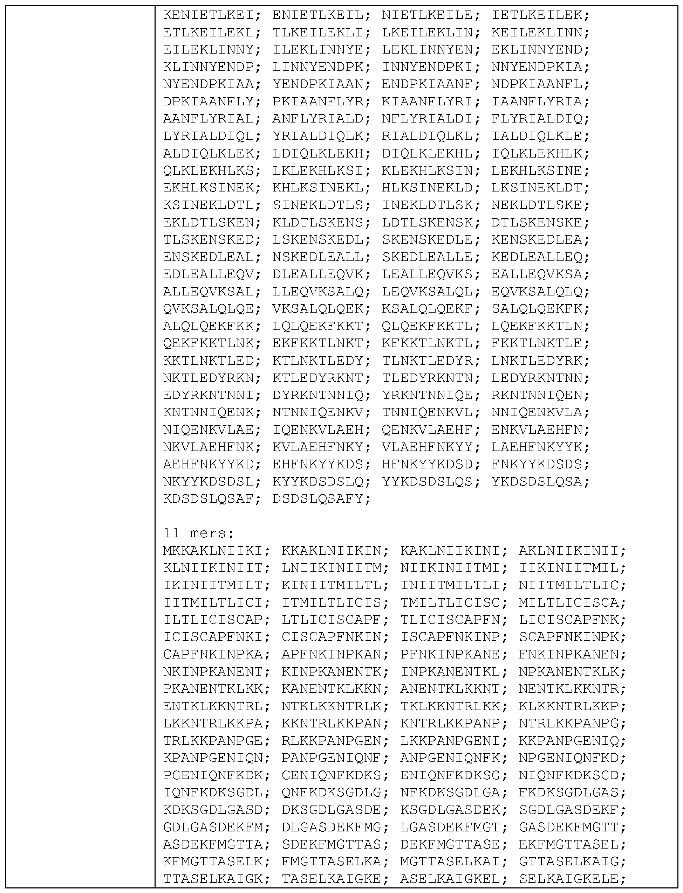

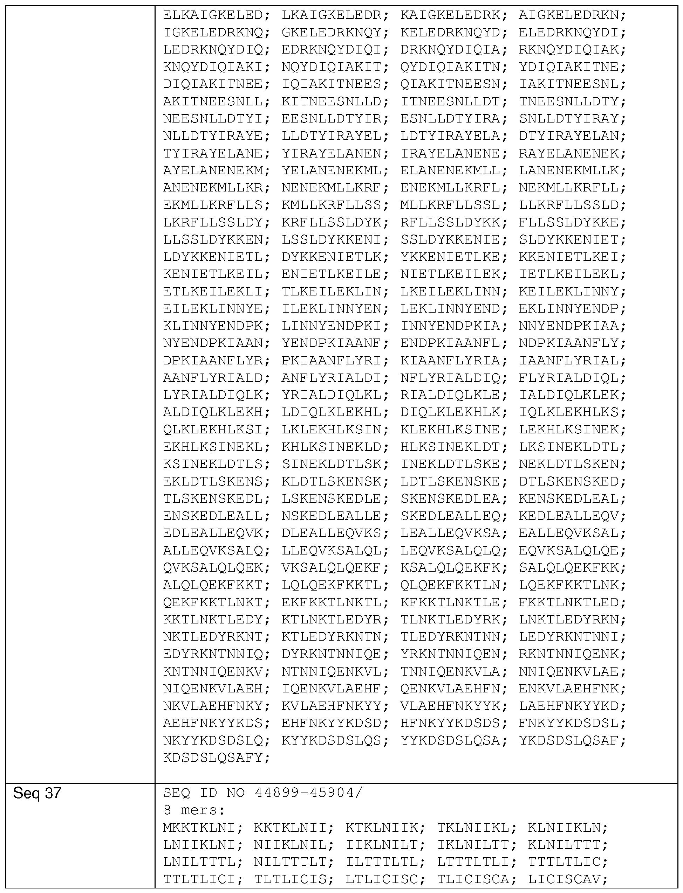

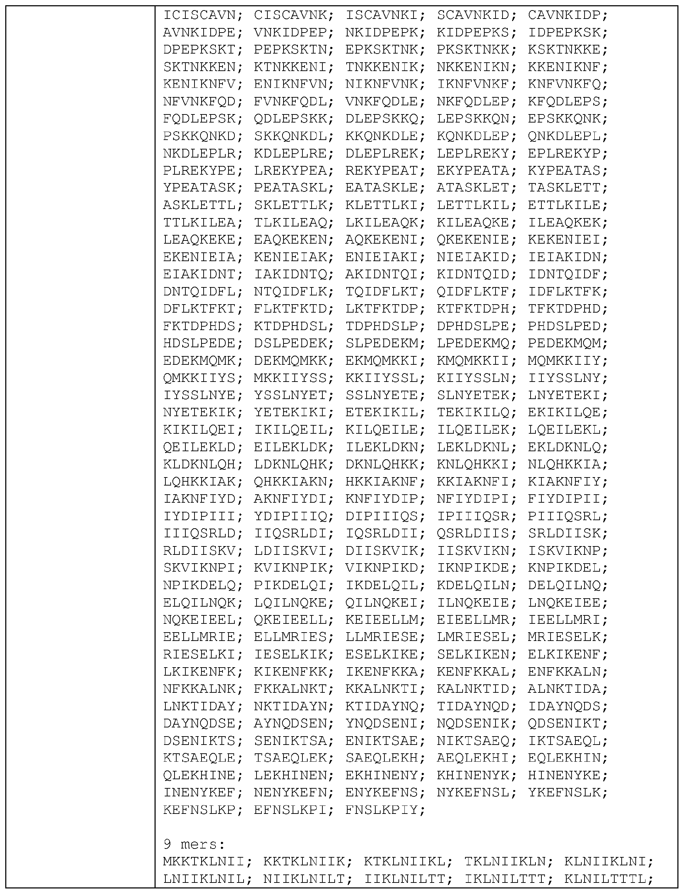

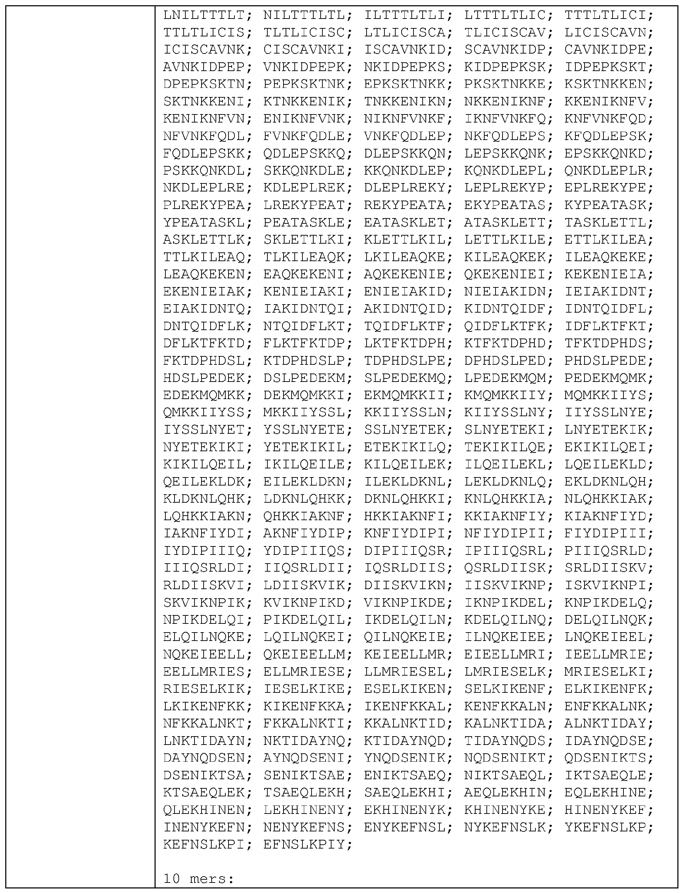

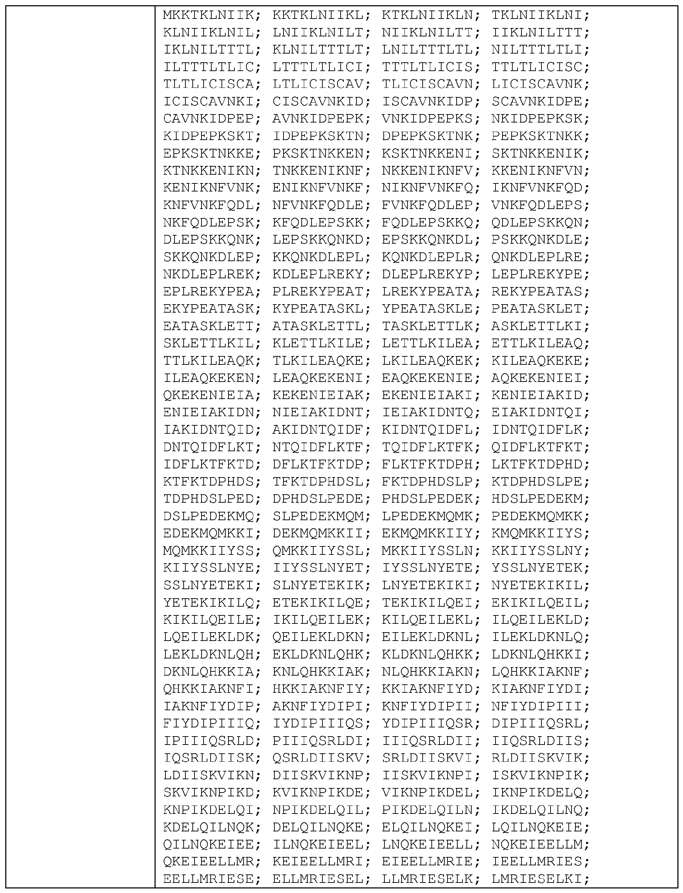

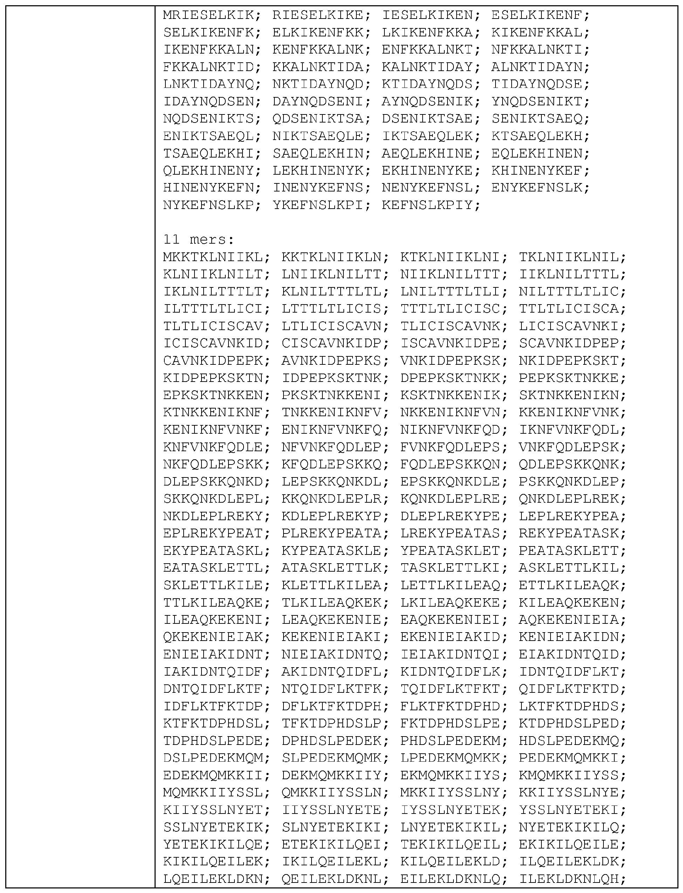

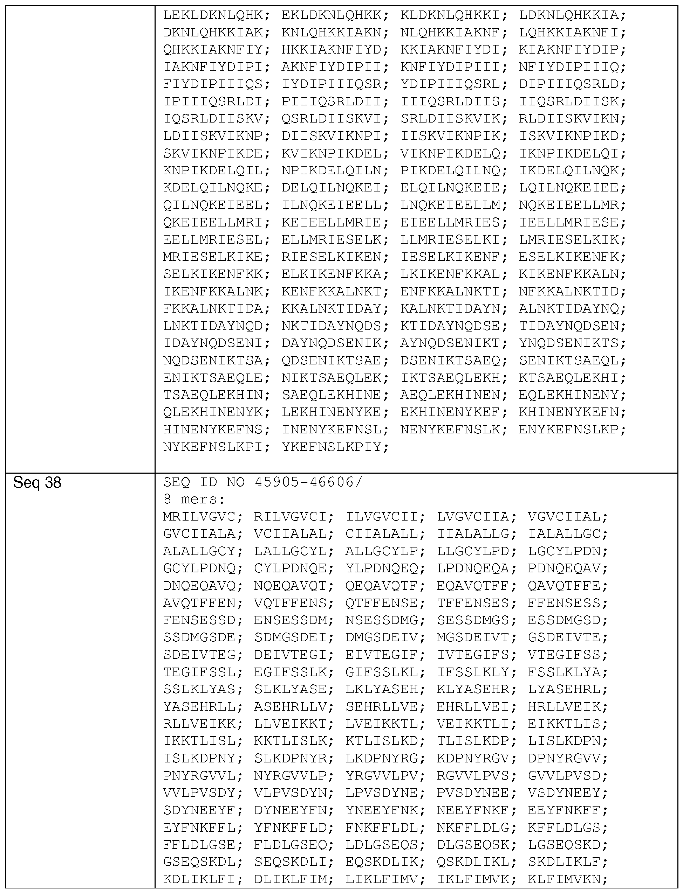

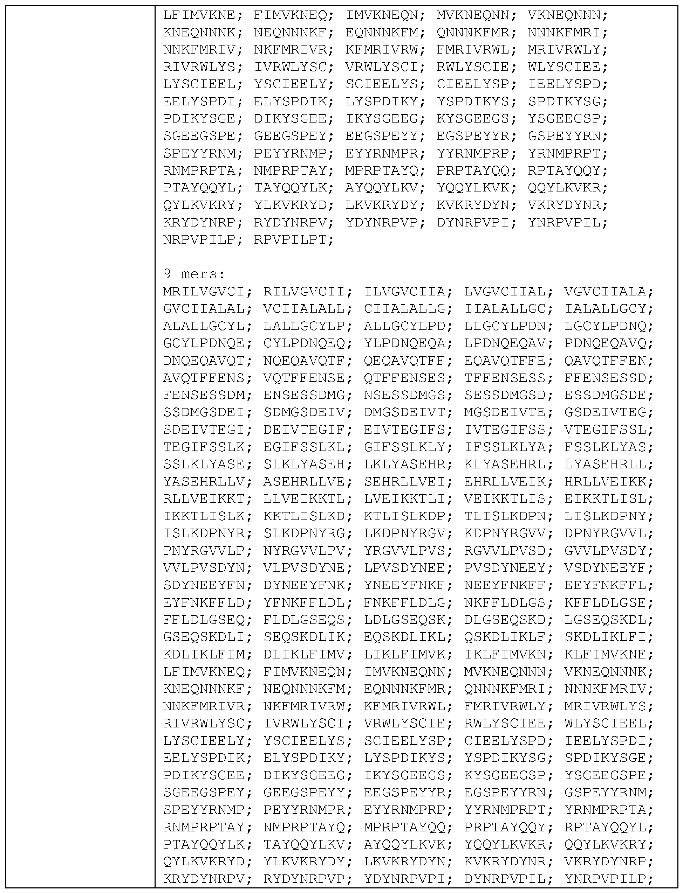

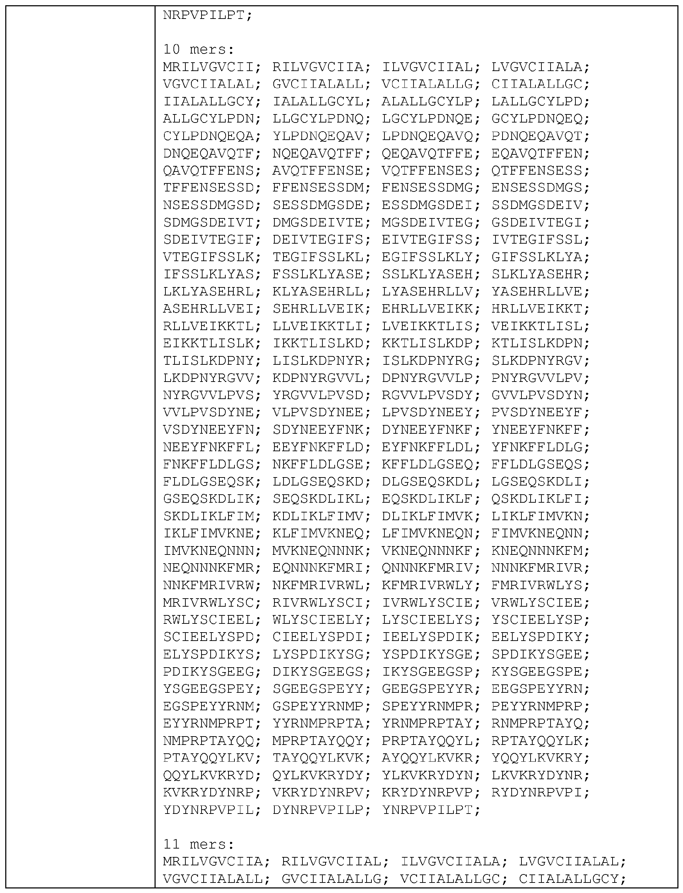

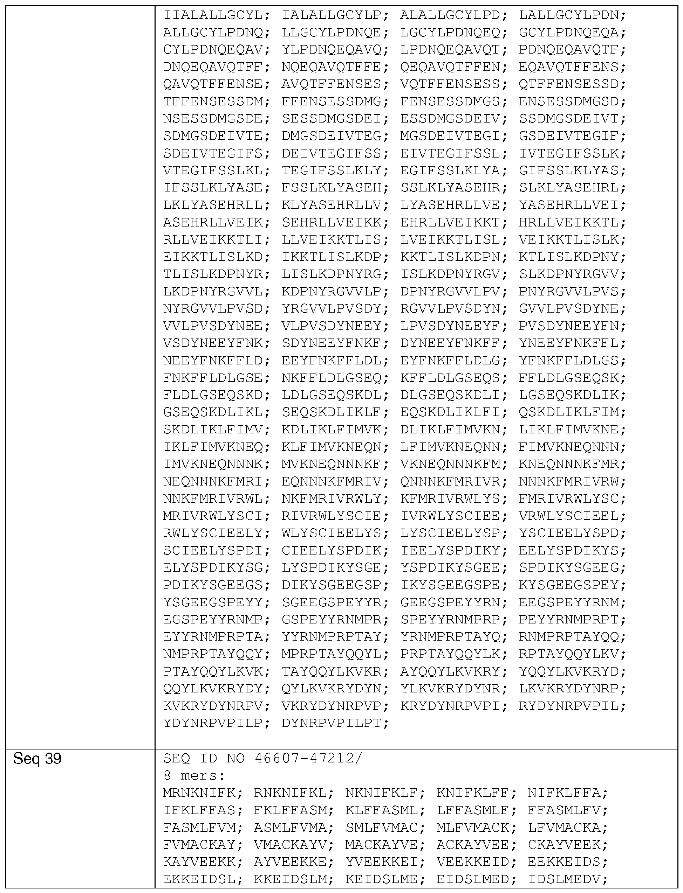

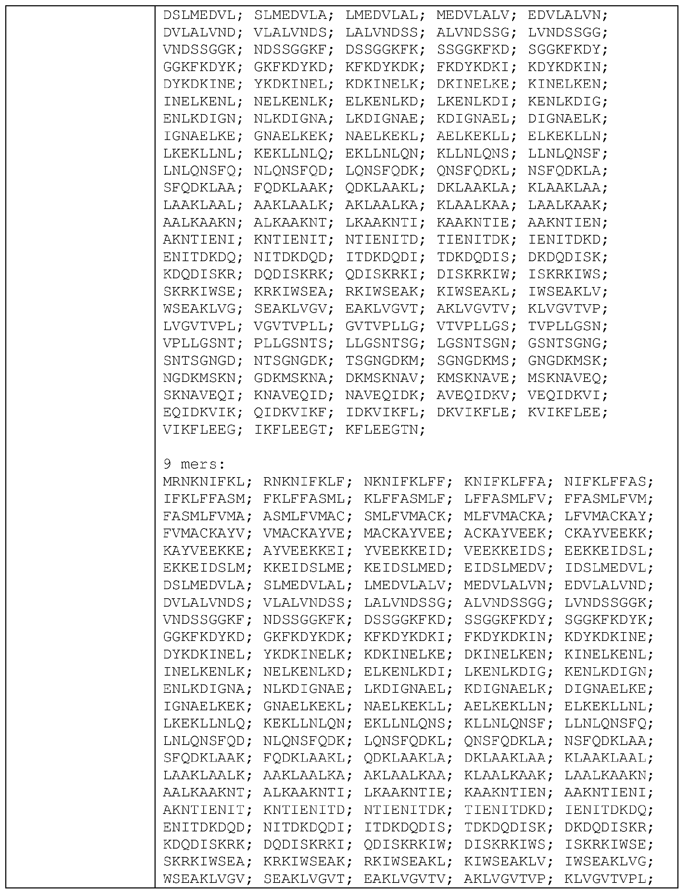

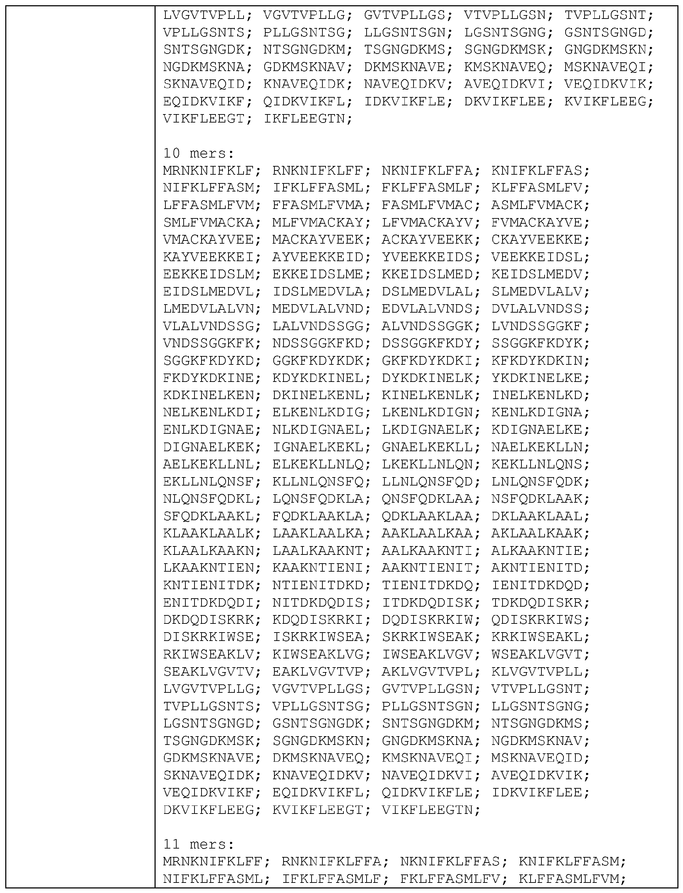

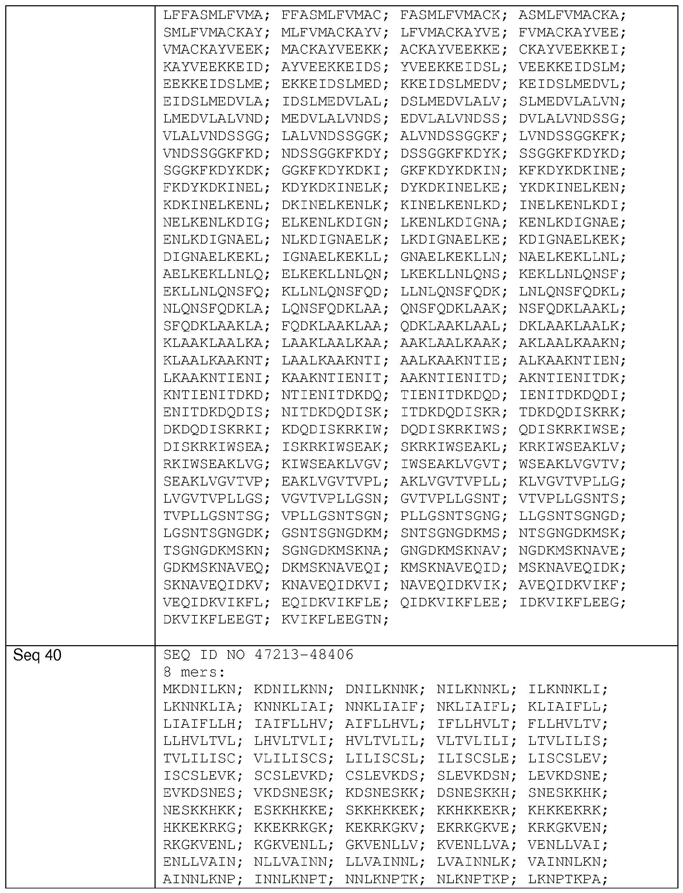

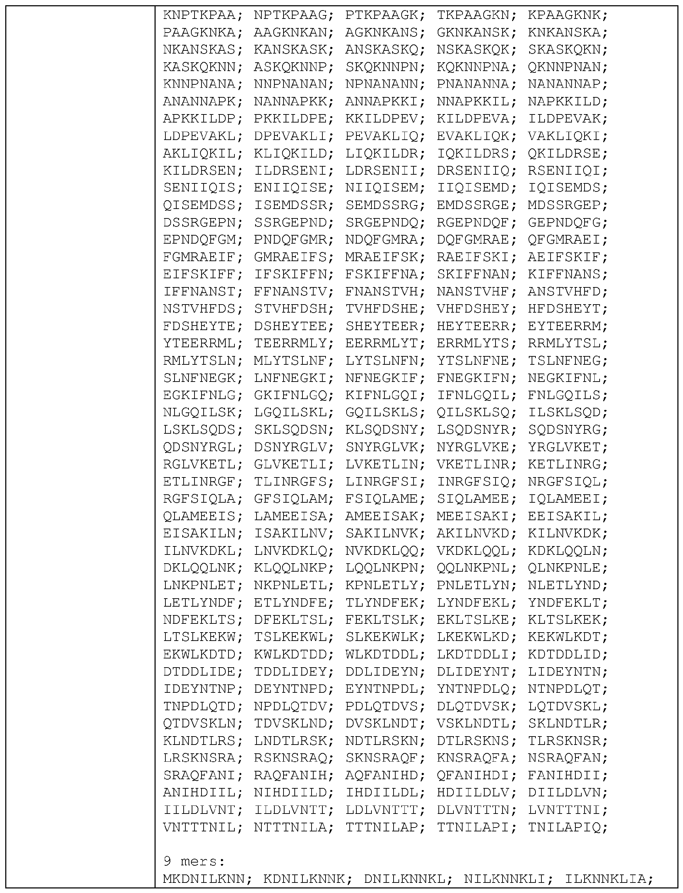

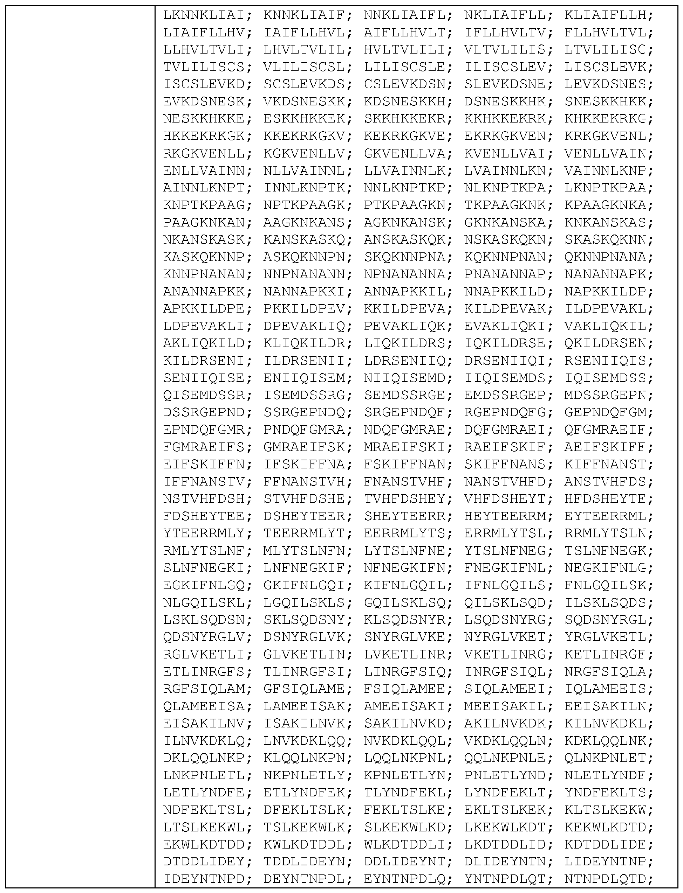

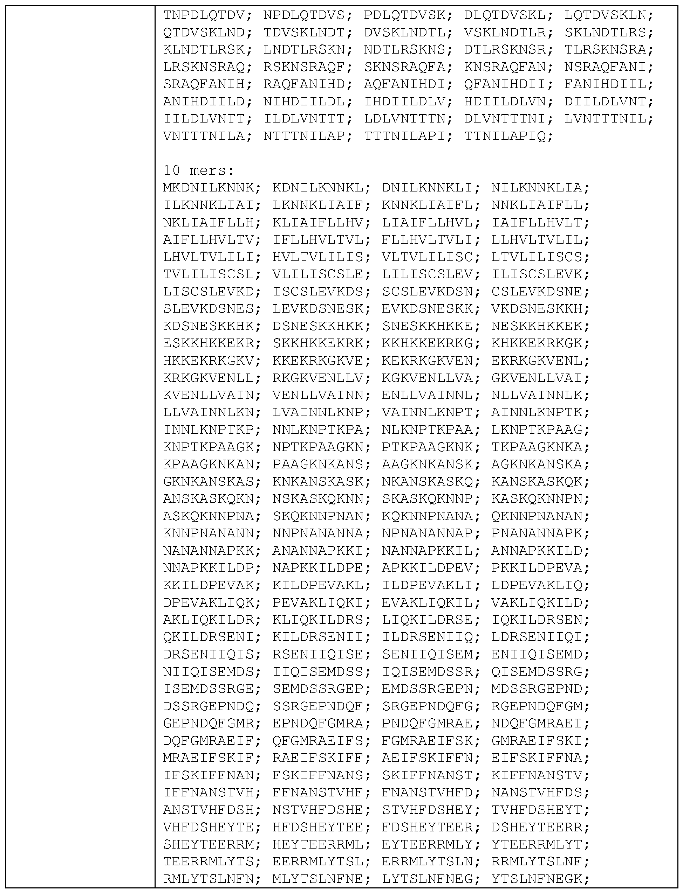

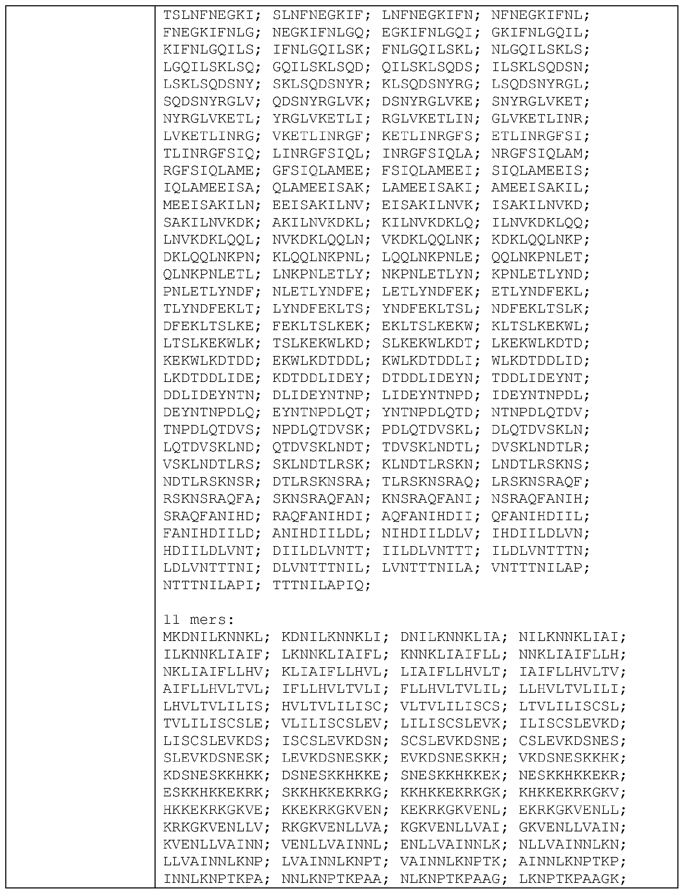

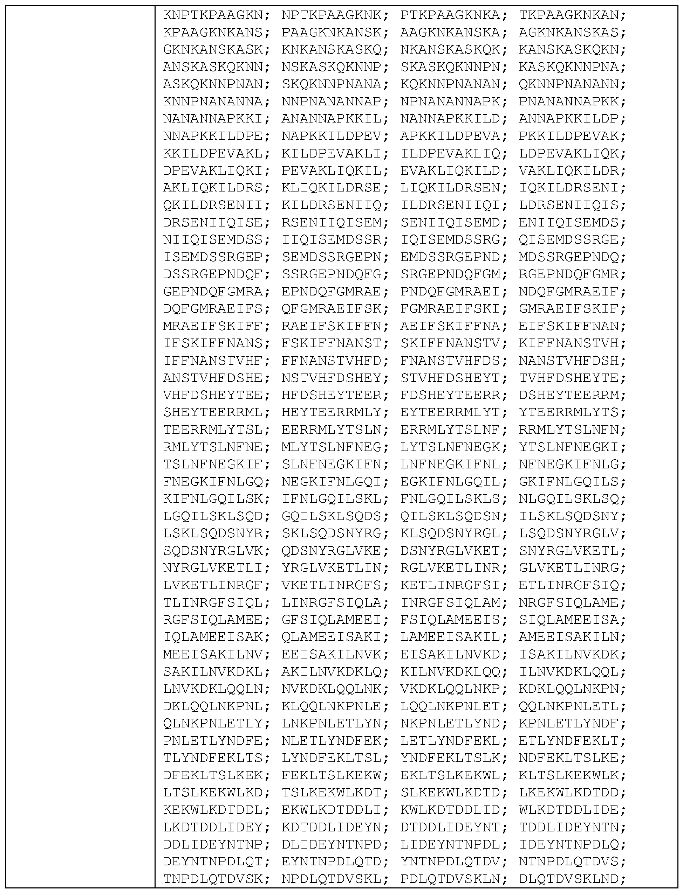

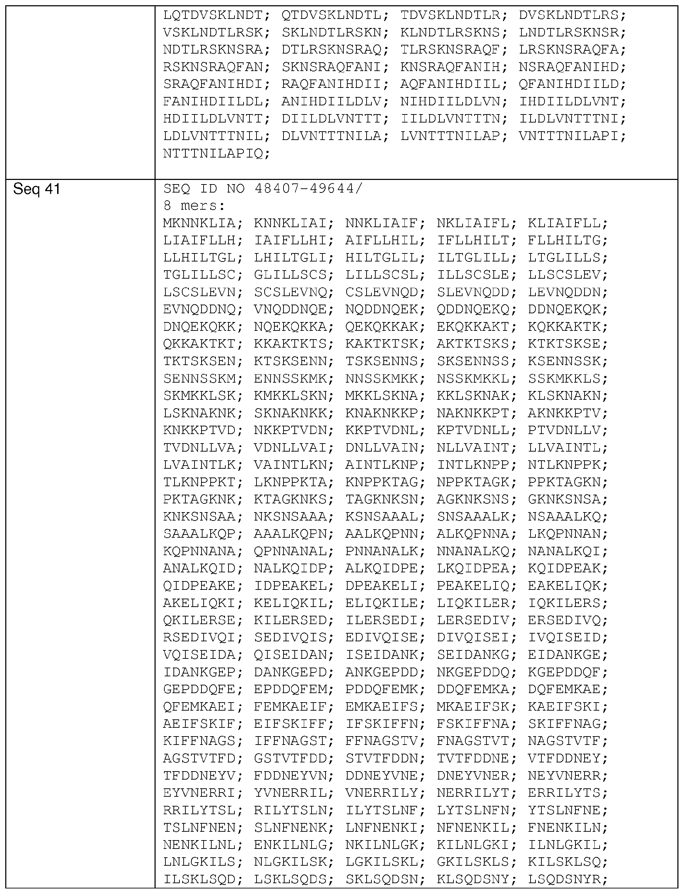

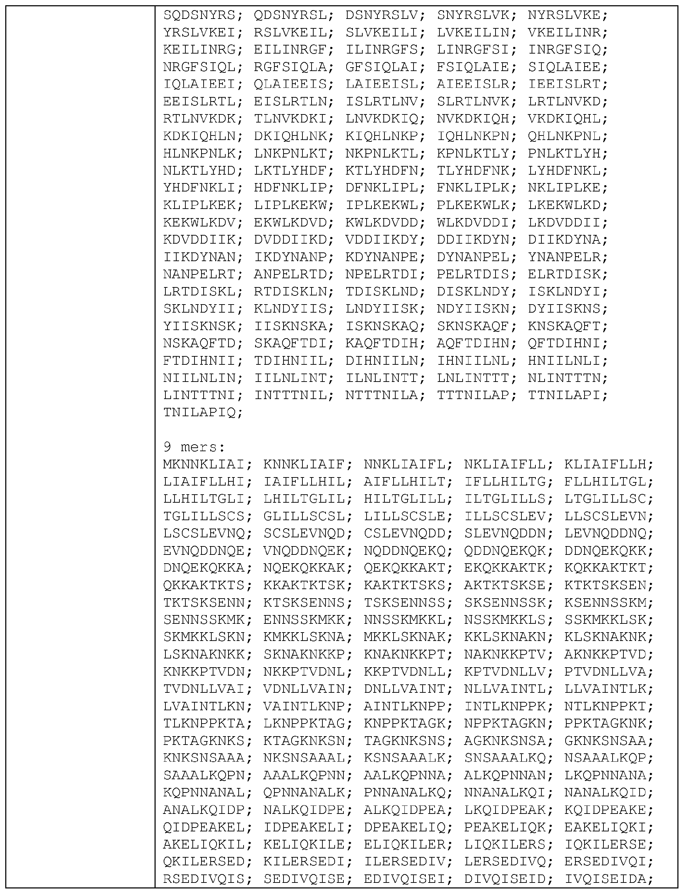

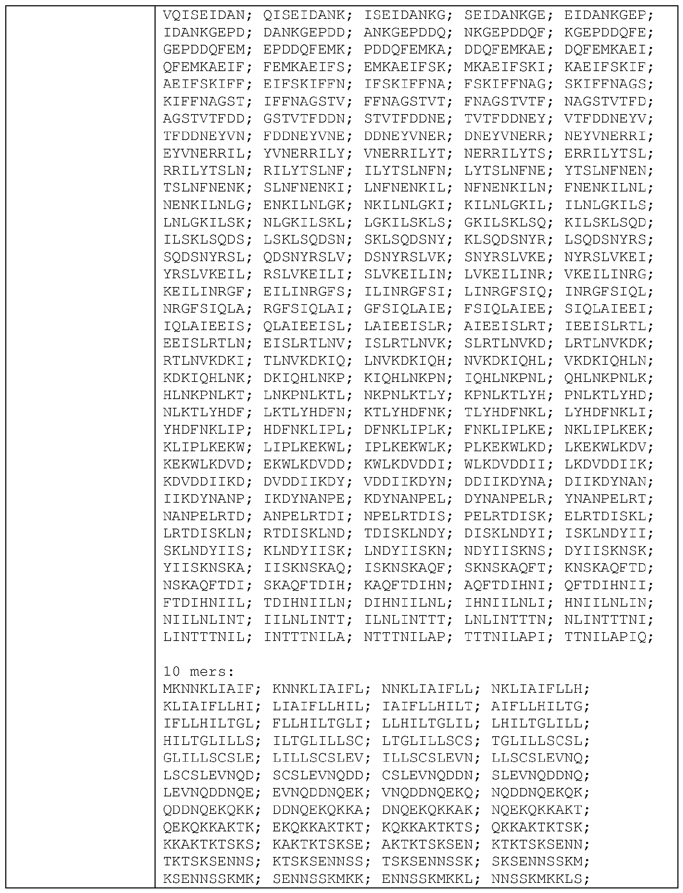

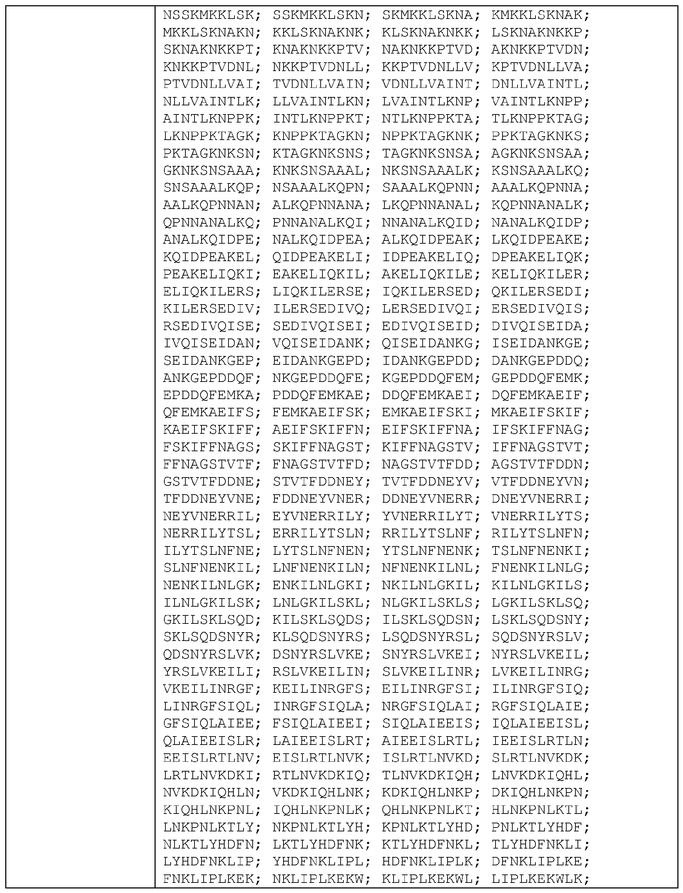

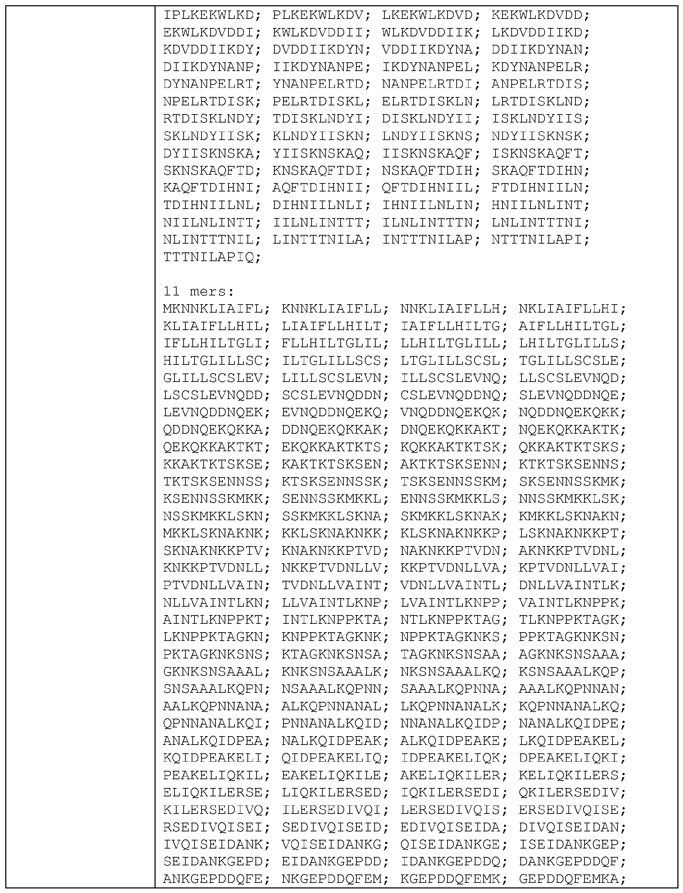

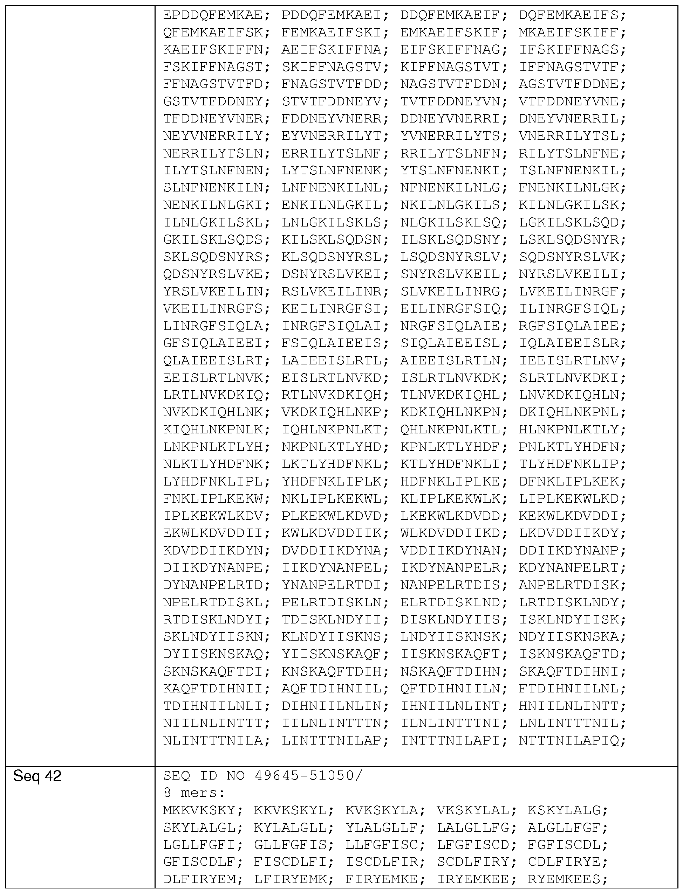

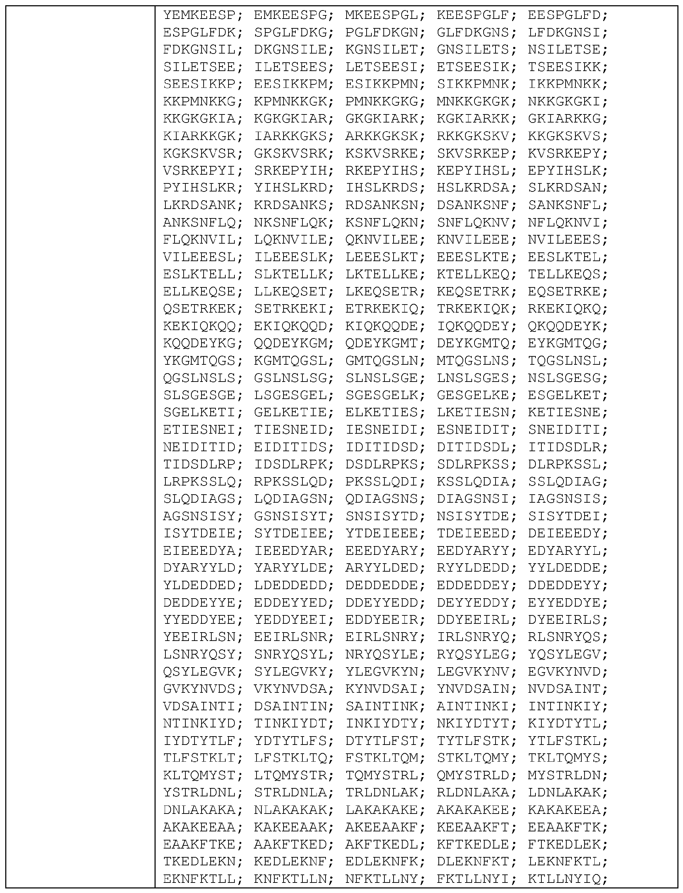

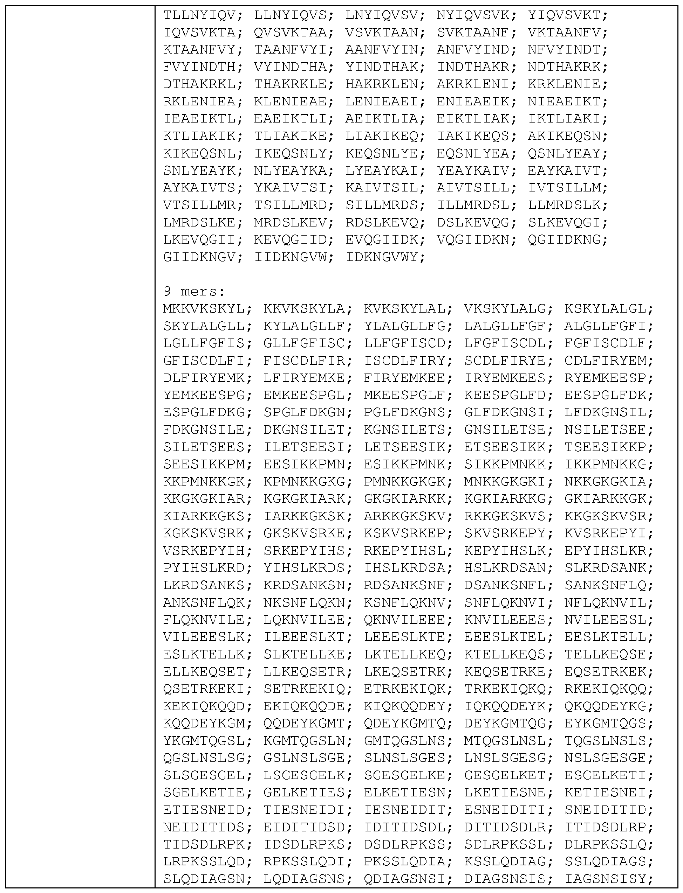

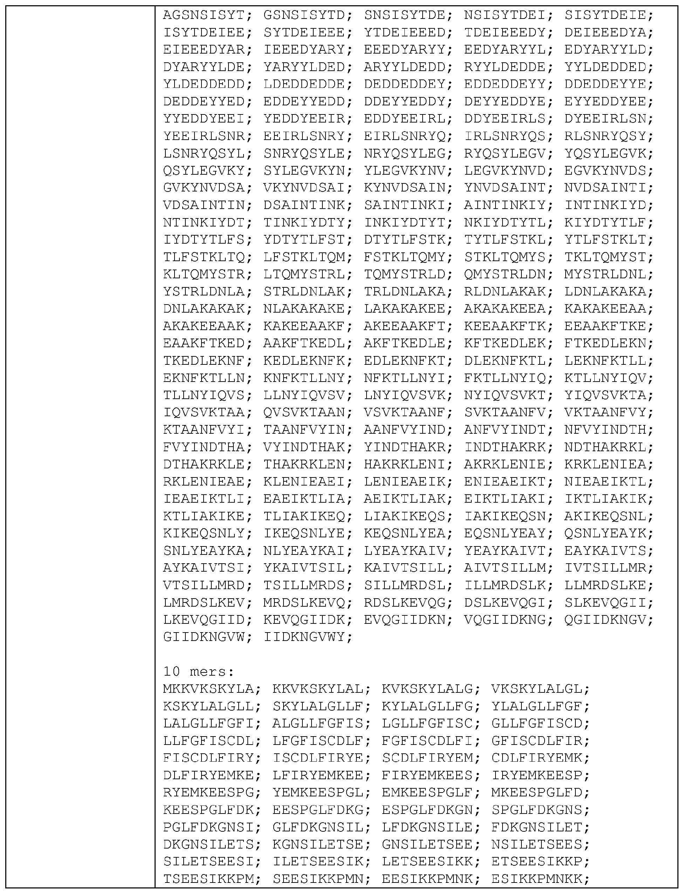

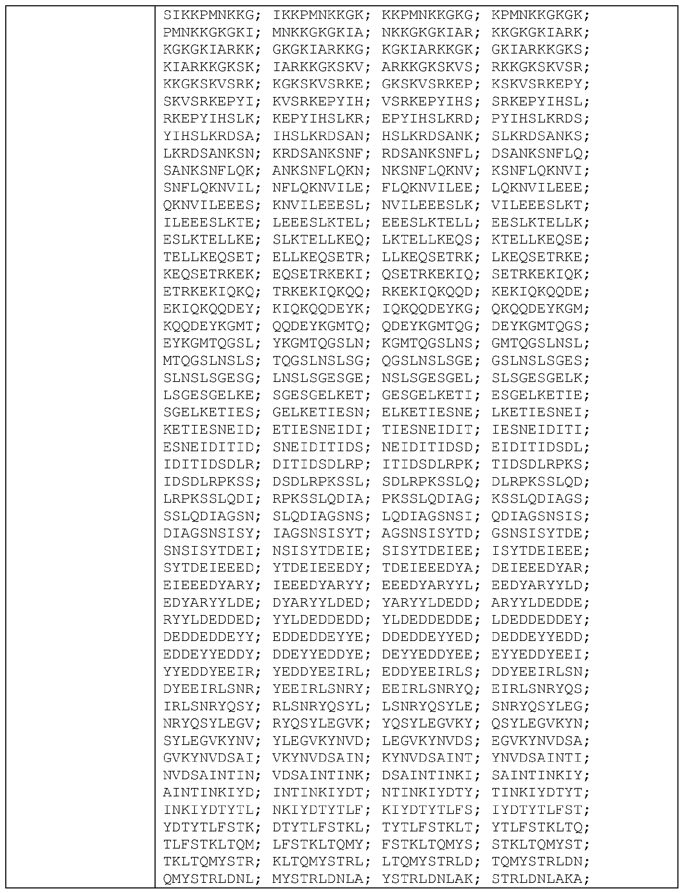

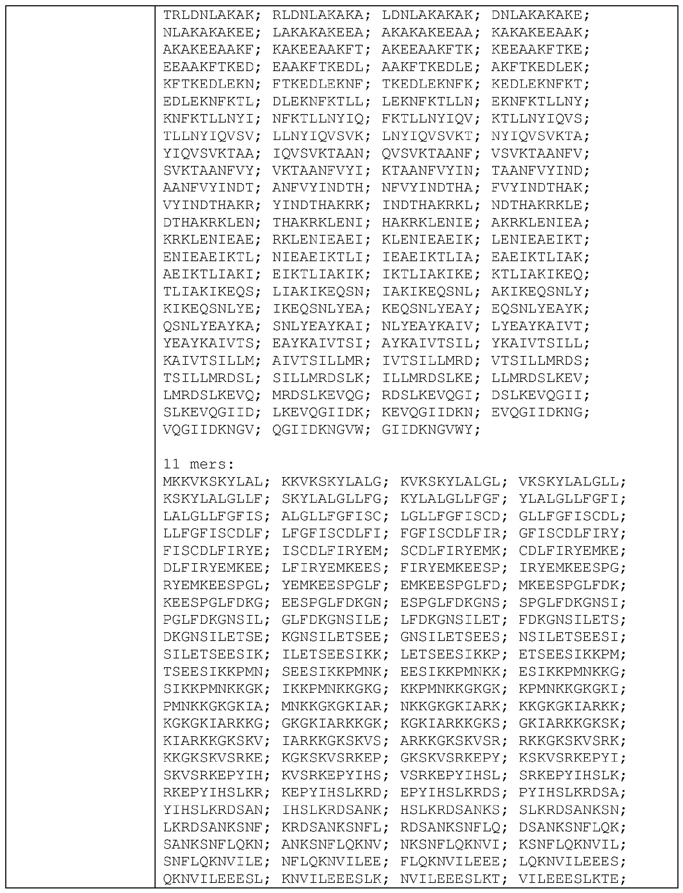

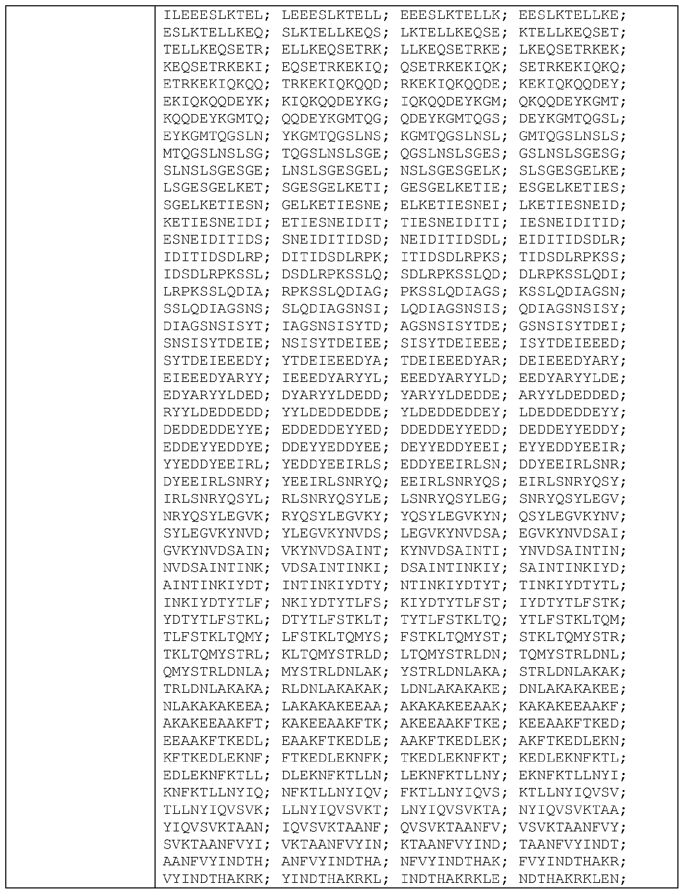

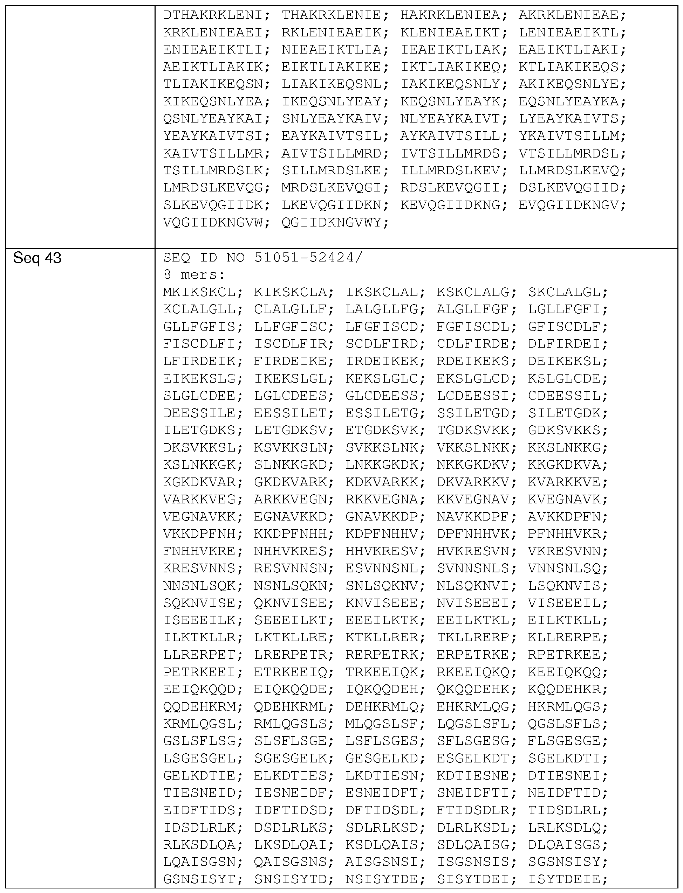

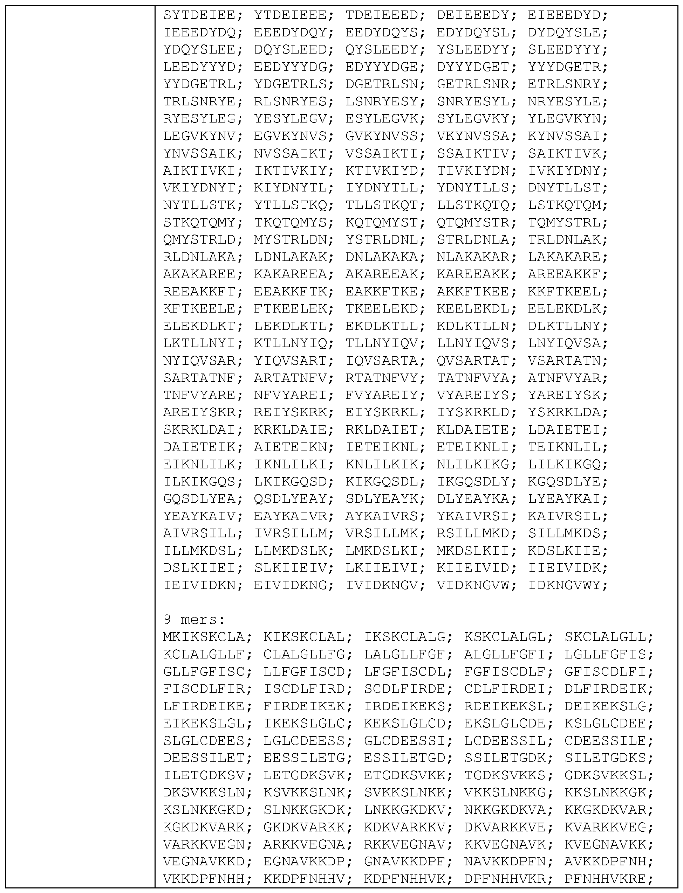

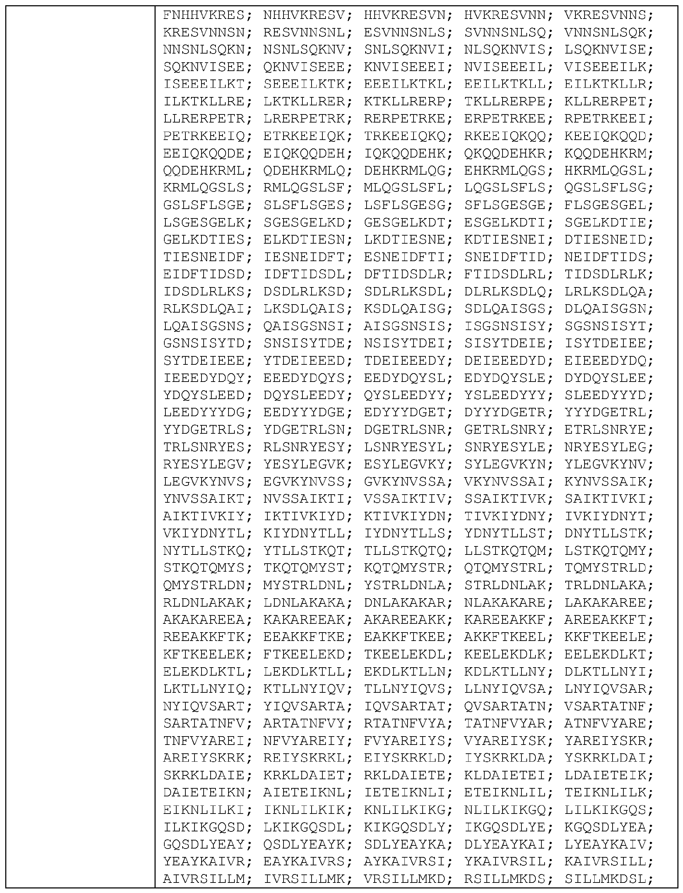

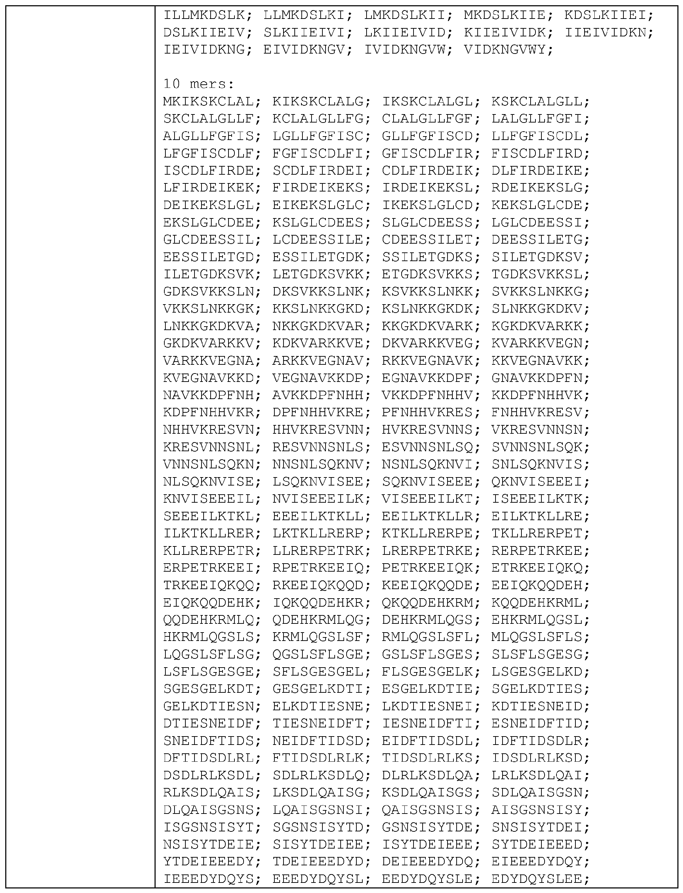

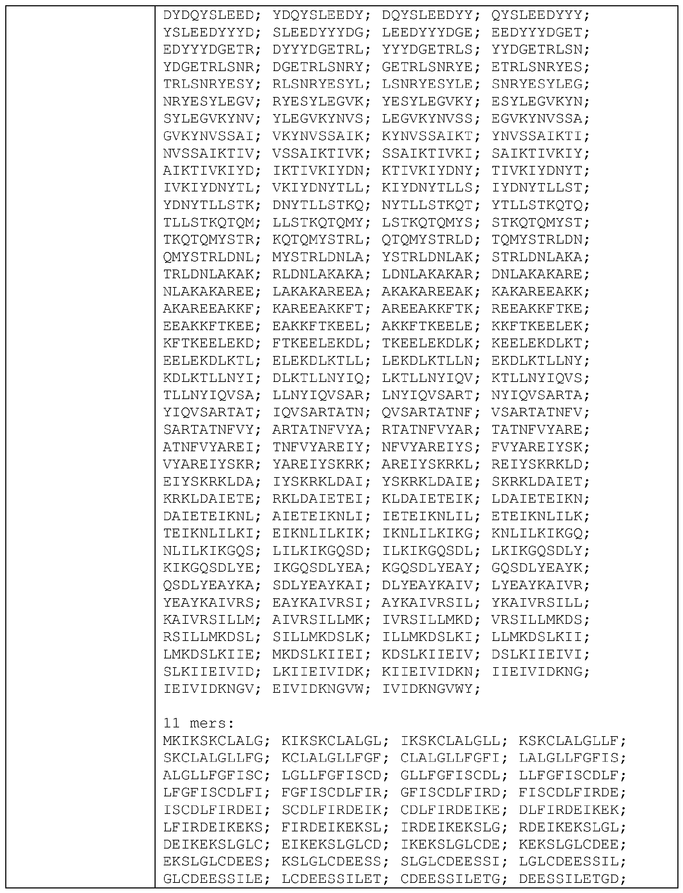

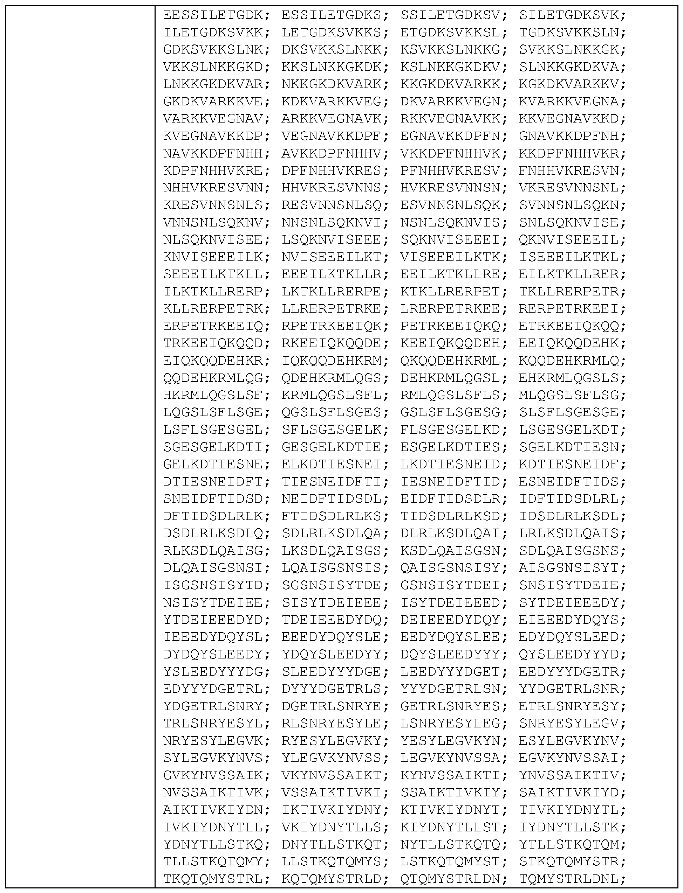

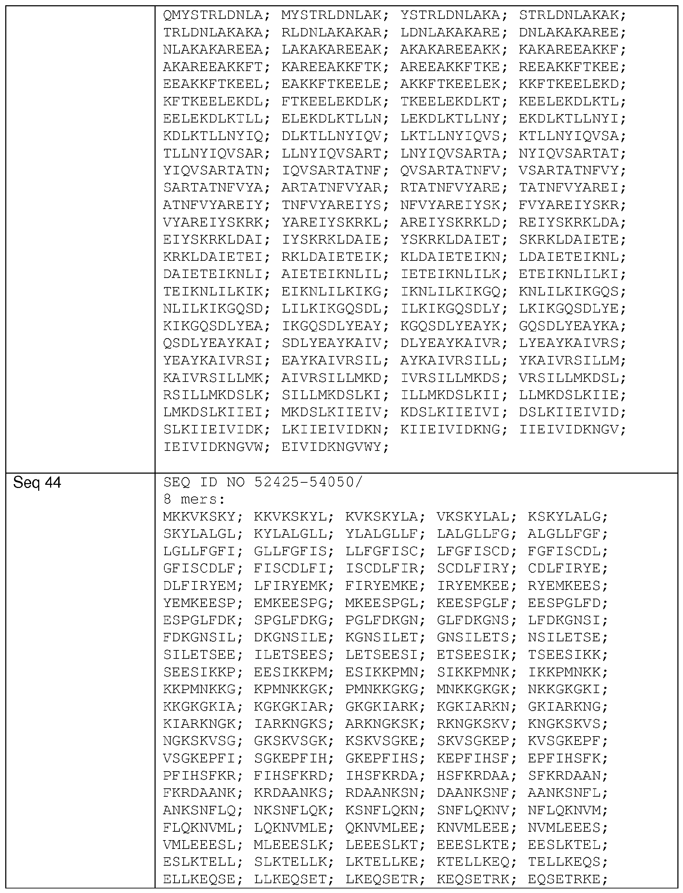

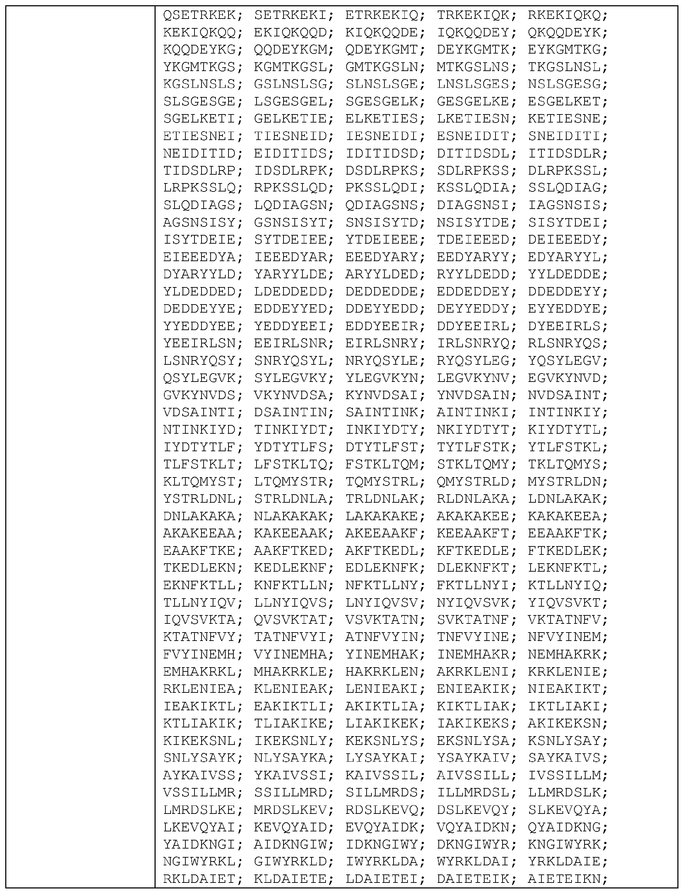

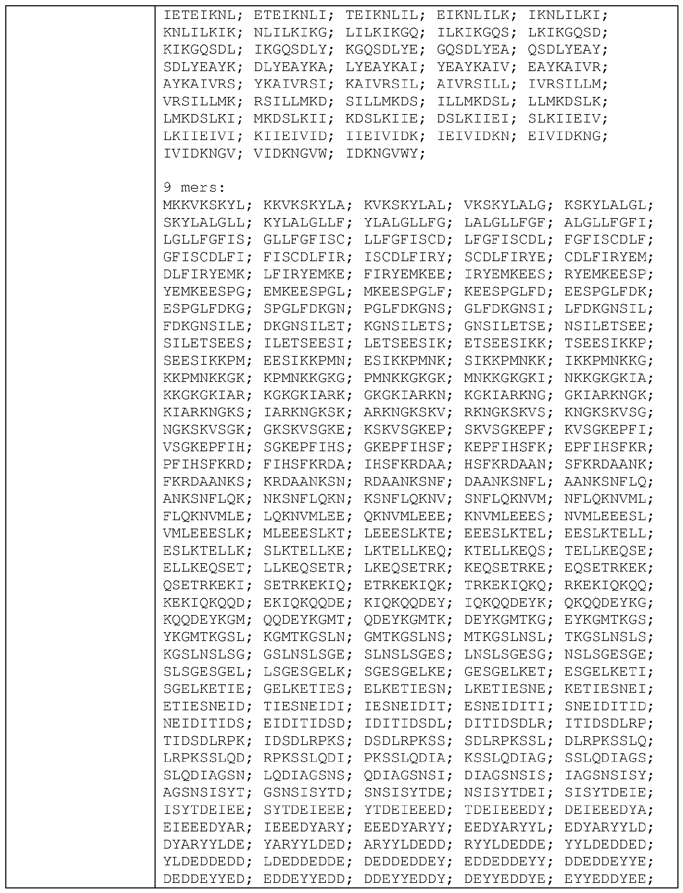

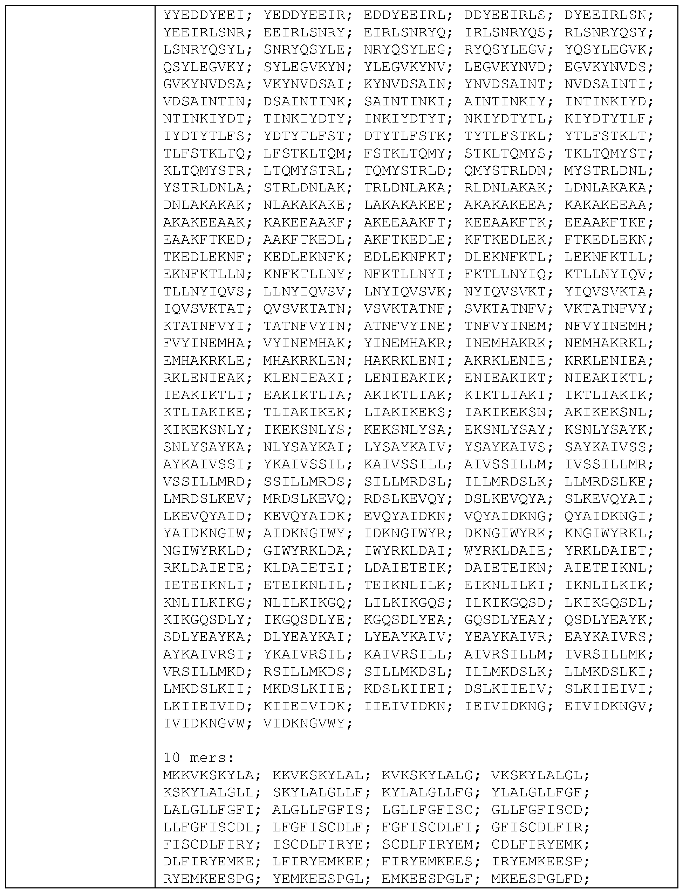

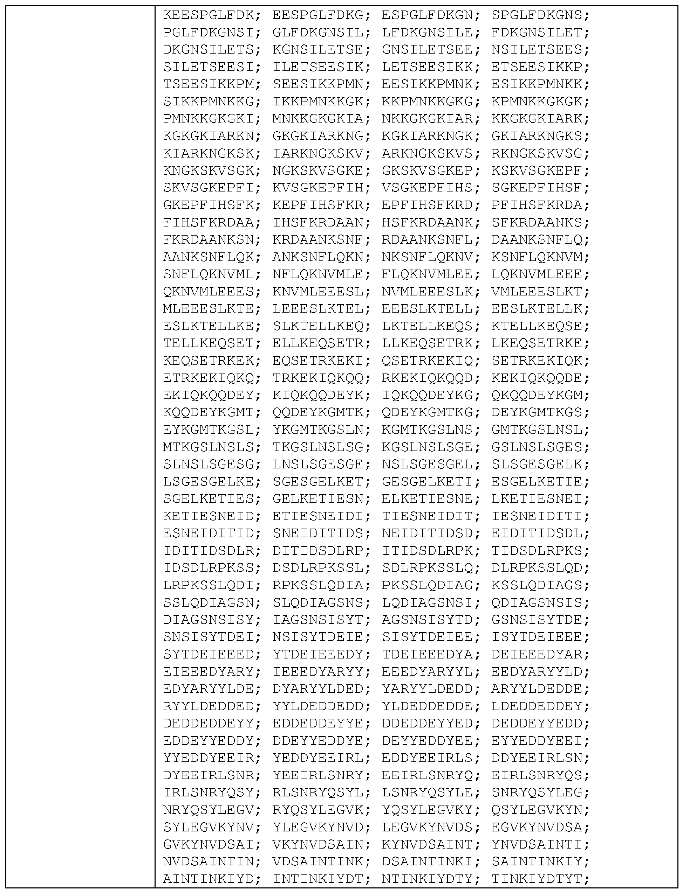

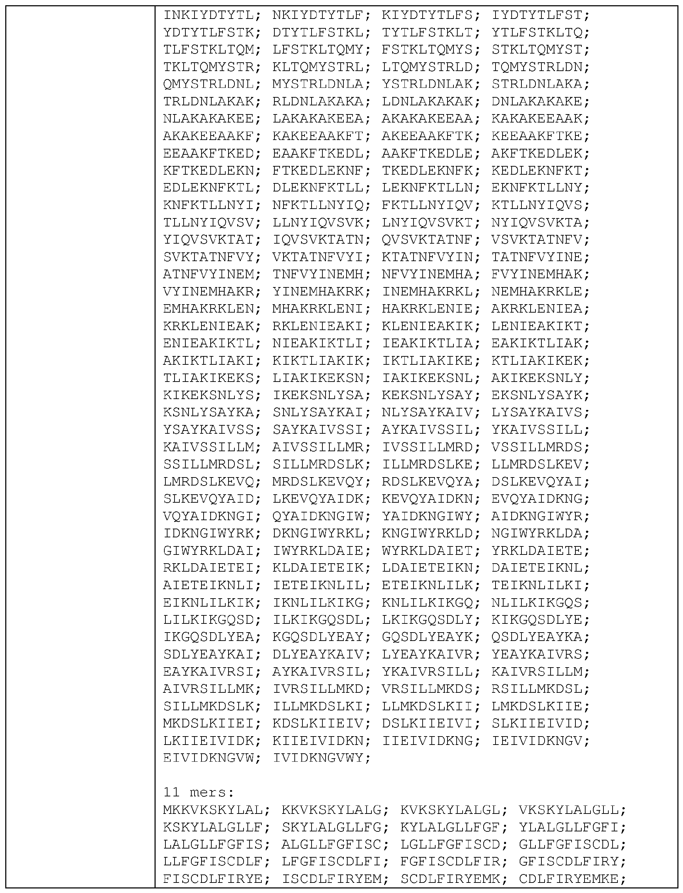

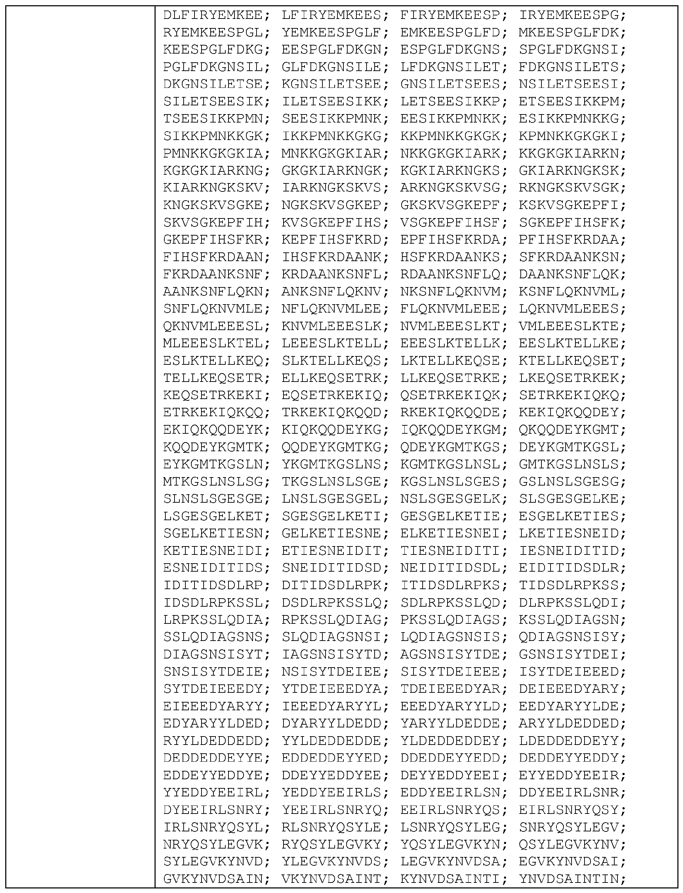

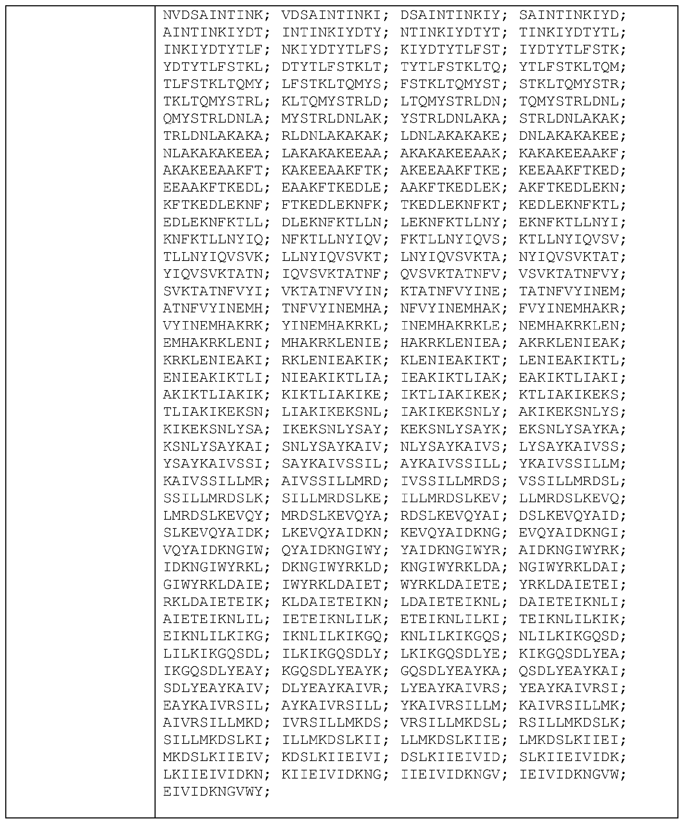

In another preferred embodiment the present disclosure relates to one or more MHC-peptide complexes or MHC multimers comprising one or more borrelia class I antigen peptide fragments or to the borrelia class I antigenic peptide fragments themselves, where the peptide fragments are selected from the peptides listed in Figure 31, Figure 32, Figure 39, Figure 40, Figure 41, Figure 42, Table A, Table B and Table C.

Borrelia class II antigen peptide fragments

In one preferred embodiment the present disclosure relates to one or more MHC-peptide complexes or MHC multimers comprising one or more borrelia class II antigen peptide fragments, or to the borrelia class II antigen peptide fragments themselves.

In one preferred embodiment the present disclosure relates to one or more MHC-peptide complexes or MHC multimers comprising one or more borrelia class II antigen peptide fragments, or to the borrelia class II antigen peptide fragments themselves, wherein said peptide fragments are 13 mers, 14 mers, 15 mers and/or 16 mers.

In one preferred embodiment the present disclosure relates to one or more MHC-peptide complexes or MHC multimers comprising one or more borrelia class II antigen peptide fragments, or to the borrelia class II antigen peptide fragments themselves, wherein said peptide fragments are 13 mers, 14 mers, 15 mers and/or 16 mers of a protein from or encoded by Borrelia burgdorferi such as Borrelia burgdorferi B31.

In one preferred embodiment the present disclosure relates to one or more MHC-peptide complexes or MHC multimers comprising one or more borrelia class II antigen peptide fragments, or to the borrelia class II antigen peptide fragments themselves, wherein said peptide fragments are 13 mers, 14 mers, 15 mers and/or 16 mers of a protein from or encoded by Borrelia afzelii such as Borrelia afzelii PKo.

In one preferred embodiment the present disclosure relates to one or more MHC-peptide complexes or MHC multimers comprising one or more borrelia class II antigen peptide fragments, or to the borrelia class II antigen peptide fragments themselves, wherein said peptide fragments are 13 mers, 14 mers, 15 mers and/or 16 mers of a protein from or encoded by Borrelia garinii such as Borrelia garinii PBi.

In one preferred embodiment the present disclosure relates to one or more MHC-peptide complexes or MHC multimers comprising one or more borrelia class II antigen peptide fragments, or to the borrelia class II antigen peptide fragments themselves, wherein said peptide fragments are 13 mers, 14 mers, 15 mers and/or 16 mers of a protein from or encoded by Borrelia anserina, Borrelia barbouri, Borrelia afzelii, Borrelia afzelii ACA-1, Borrelia afzelii K78, Borrelia afzelii PKo, Borrelia andersonii, Borrelia bissettii, Borrelia burgdorferi, Borrelia burgdorferi 118a, Borrelia burgdorferi 156a, Borrelia burgdorferi 29805, Borrelia burgdorferi 64b, Borrelia burgdorferi 72a, Borrelia burgdorferi 80a, Borrelia burgdorferi 94a, Borrelia burgdorferi B31, Borrelia burgdorferi Bol26, Borrelia burgdorferi CA-11.2a, Borrelia burgdorferi Borrelia burgdorferi ZS7, Borrelia californiensis, Borrelia garinii, Borrelia garinii PBi, Borrelia garinii PBr, Borrelia genomosp. 1, Borrelia genomosp. 2, Borrelia japonica, Borrelia lusitaniae, Borrelia spielmanii, Borrelia spielmanii A14S, Borrelia tanukii, Borrelia turdi, Borrelia valaisiana, Borrelia valaisiana VS116, Candidatus Borrelia texasensis, Borrelia sp. AA4Pool, Borrelia sp. AI-1, Borrelia sp. B31, Borrelia sp. BC-1, Borrelia sp. CA1133, Borrelia sp. CA1176, Borrelia sp. CA128, Borrelia sp. CA13, Borrelia sp. CA134, Borrelia sp. CA142, Borrelia sp. CA20, Borrelia sp. CA22, Borrelia sp.

CA27, Borrelia sp. CA28, Borrelia sp. CA29, Borrelia sp. CA31, Borrelia sp. CA33, Borrelia sp. CA370, Borrelia sp. CA372, Borrelia sp. CA378, Borrelia sp. CA388, Borrelia sp. CA393, Borrelia sp. CA394, Borrelia sp. CA395, Borrelia sp. CA399, Borrelia sp. CA400, Borrelia sp. CA401, Borrelia sp. CA402, Borrelia sp. CA404, Borrelia sp. CA411, Borrelia sp. CA426, Borrelia sp. CA443, Borrelia sp. CA446, Borrelia sp. CA448, Borrelia sp. CA462, Borrelia sp. CA468, Borrelia sp. CA502, Borrelia sp. CA504, Borrelia sp. CA507, Borrelia sp. CA547, Borrelia sp. CA552, Borrelia sp. CA8, Borrelia sp. D22, Borrelia sp. D35, Borrelia sp. FD-1, Borrelia sp. FL18, Borrelia sp. FL27, Borrelia sp. FL35, Borrelia sp. FL42, Borrelia sp. HN6, Borrelia sp. HN7, Borrelia sp. HN8, Borrelia sp. HNM13, Borrelia sp. HNM14, Borrelia sp. HNM19, Borrelia sp. IA1, Borrelia sp. Ir-3519, Borrelia sp. Ir-4721, Borrelia sp. Ir-4812, Borrelia sp. Ir-5215, Borrelia sp. LV5, Borrelia sp. MI-2, Borrelia sp. MI-5, Borrelia sp. MI-6, Borrelia sp. MI-8, Borrelia sp. MI-9, Borrelia sp. MOD-1, Borrelia sp. MOD-5, Borrelia sp. MOK-3a, Borrelia sp. MOS-1b, Borrelia sp. NE49, Borrelia sp. NE581, Borrelia sp. PHaP, Borrelia sp. PSigII, Borrelia sp. SCGT-10, Borrelia sp. SCGT-8a, Borrelia sp. SCI-2, Borrelia sp. SCW-30h, Borrelia sp. SI-1, Borrelia sp. SI-10, Borrelia sp. SM-1, Borrelia sp. SV1, Borrelia sp. W97F51, Borrelia sp. Z41293, Borrelia sp. Z41493, Borrelia coriaceae, Borrelia crocidurae, Borrelia duttonii, Borrelia duttonii Ly, Borrelia hermsii, Borrelia hermsii DAH, Borrelia hispanica, Borrelia lonestari, Borrelia miyamotoi, Borrelia parkeri, Borrelia persica, Borrelia recurrentis, Borrelia recurrentis A1, Borrelia sinica, Borrelia theileri, Borrelia turcica, Borrelia turicatae, Borrelia turicatae 91E135, Borrelia sp., Borrelia sp. 'Lake Gaillard', Borrelia sp. 000133, Borrelia sp. 010298, Borrelia sp. 10MT, Borrelia sp. 5145, Borrelia sp. 57Nsk, Borrelia sp. 5MT, Borrelia sp. 6T04-2, Borrelia sp. BR, Borrelia sp. BR 2007, Borrelia sp. C5-N52, Borrelia sp. CB-A1, Borrelia sp. CB-A11, Borrelia sp. CB-A3, Borrelia sp. EFL-S0100110, Borrelia sp. IK/23, Borrelia sp. IM/16, Borrelia sp. IM/19, Borrelia sp. KR1, Borrelia sp. KR3, Borrelia sp. LB-2001, Borrelia sp. LB-M56, Borrelia sp. LB-W100, Borrelia sp. MK-N61, Borrelia sp. NR-N8, Borrelia sp. OkME1, Borrelia sp. PAnz, Borrelia sp. PJes, Borrelia sp. PMai, Borrelia sp. PMew, Borrelia sp. R57, Borrelia sp. strain Spain, Borrelia sp. TA1, Borrelia sp. TM, Borrelia sp. TM1 and/or Borrelia sp. TM2.

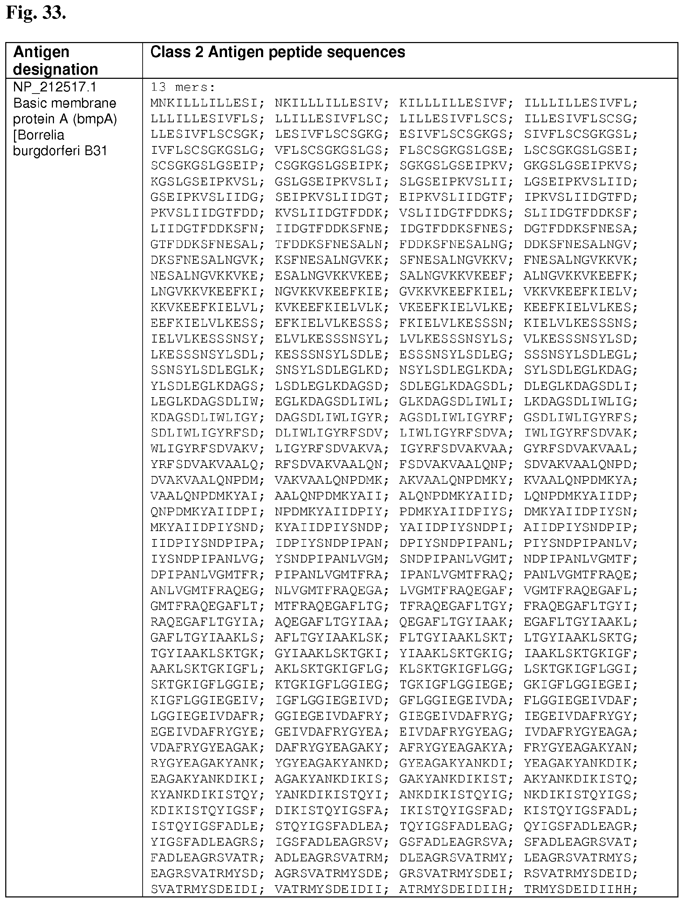

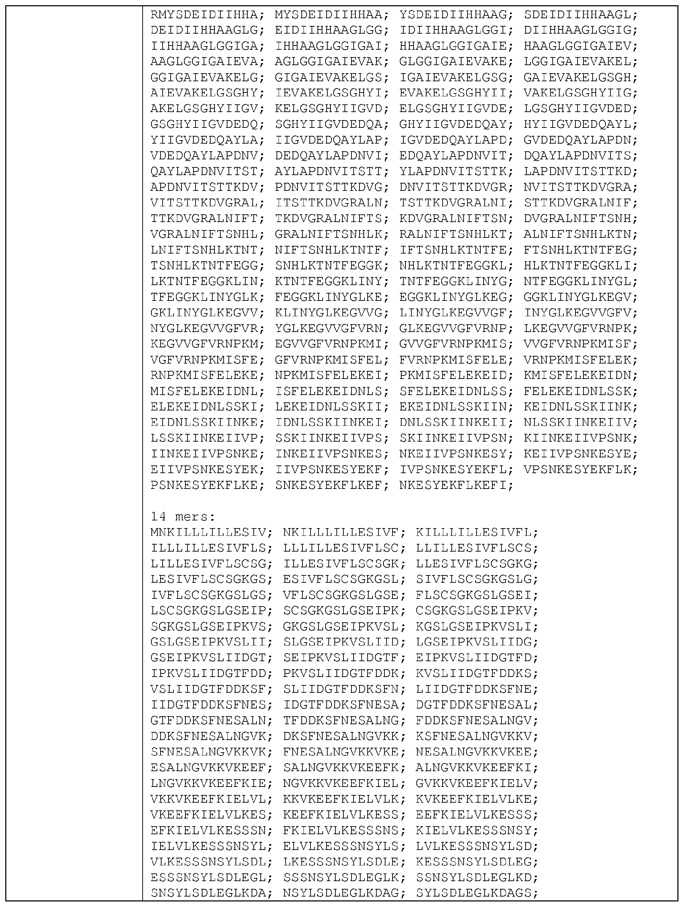

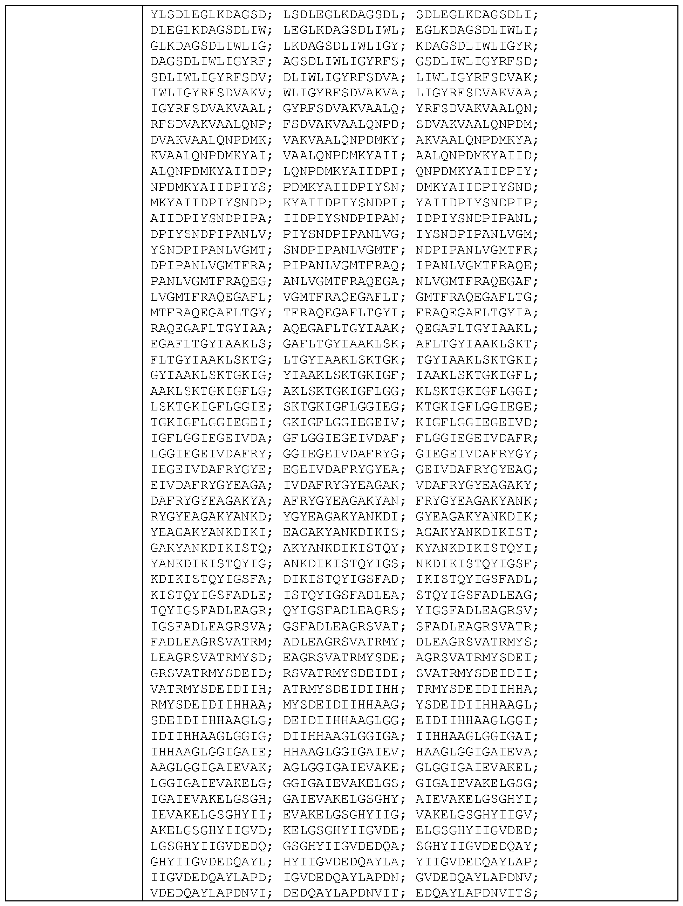

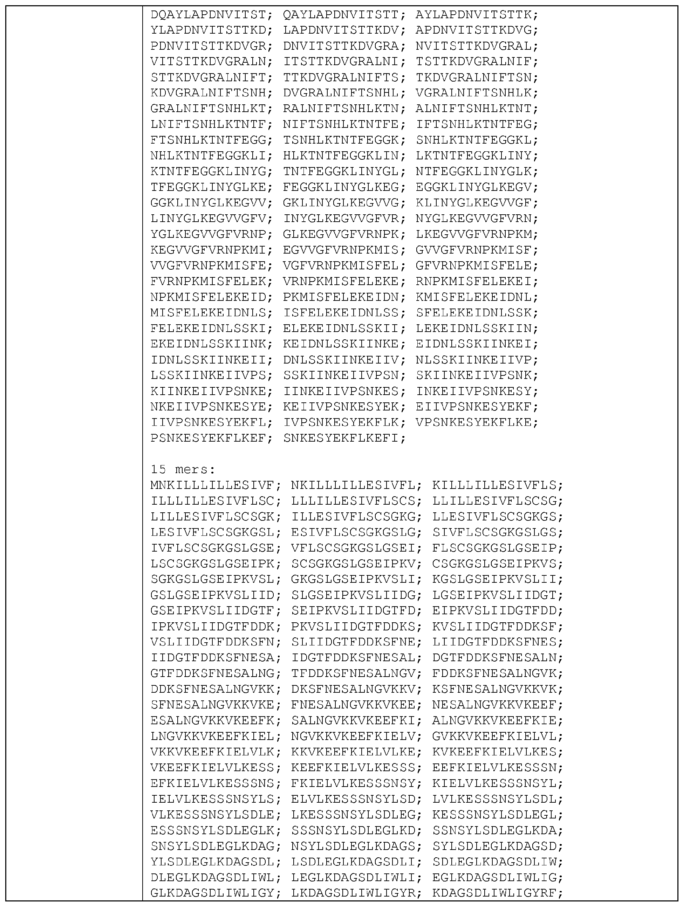

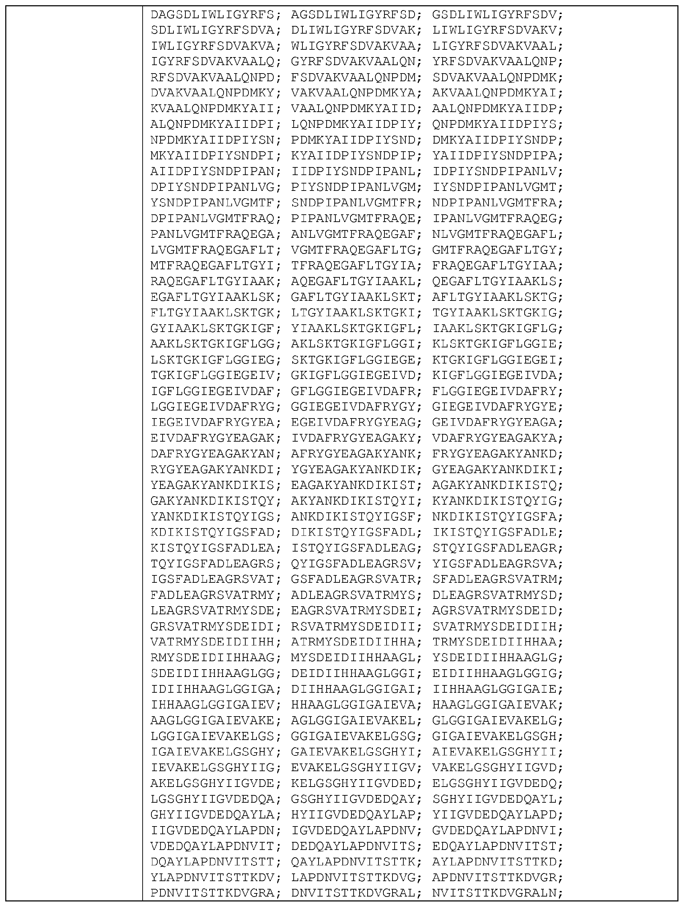

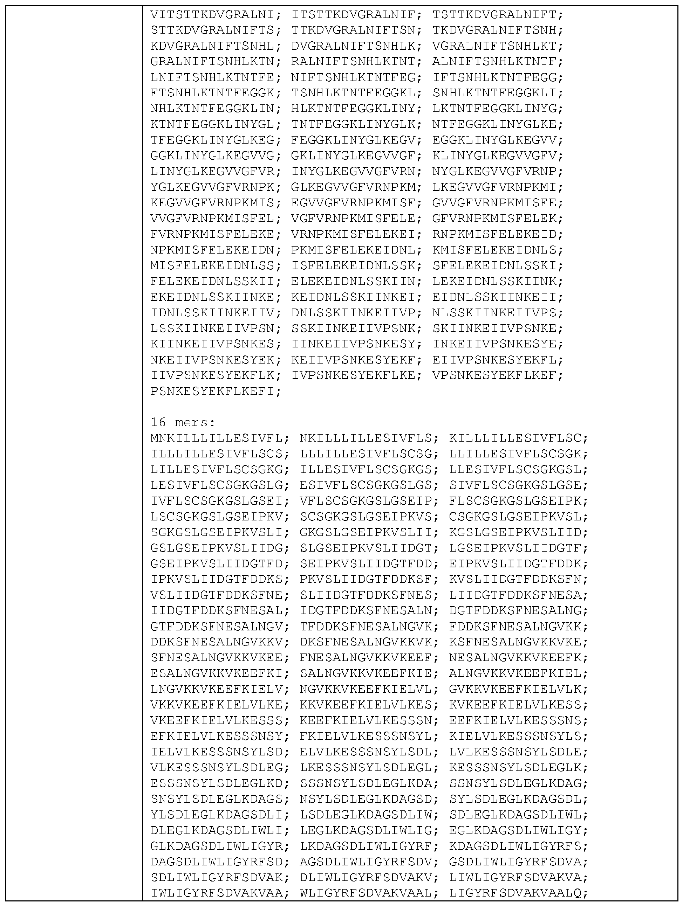

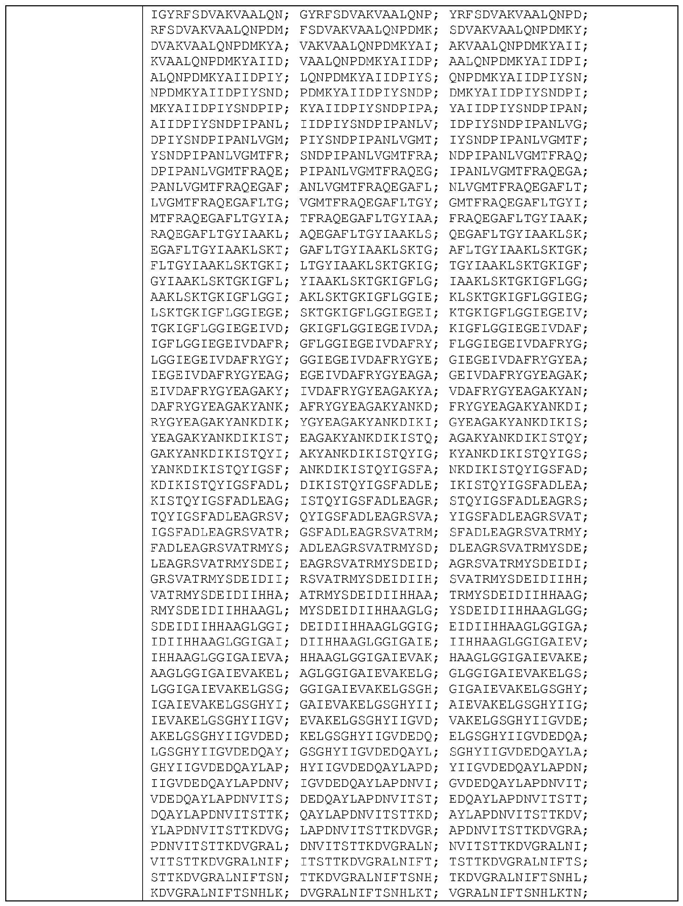

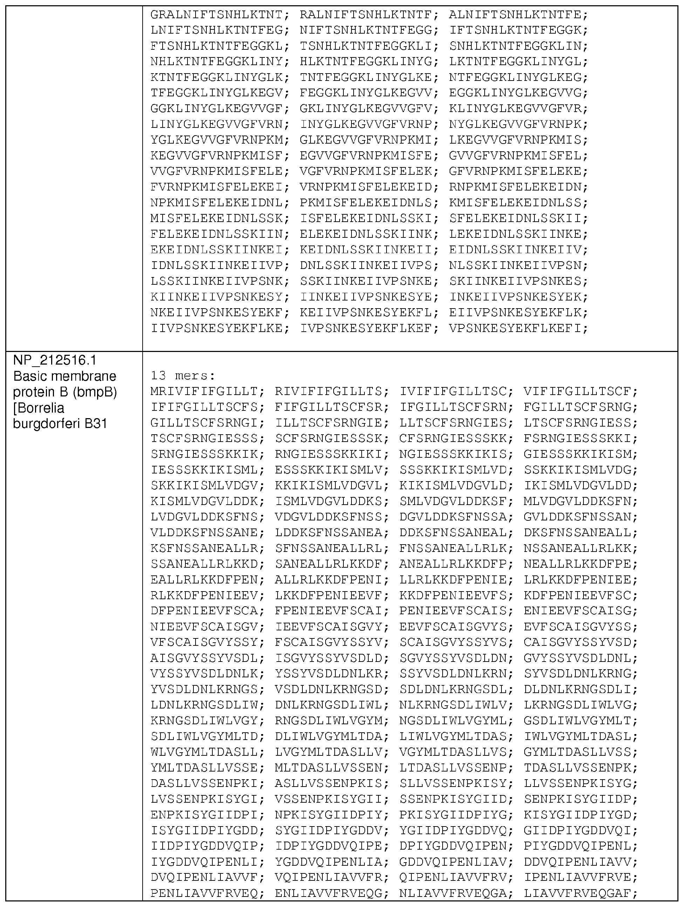

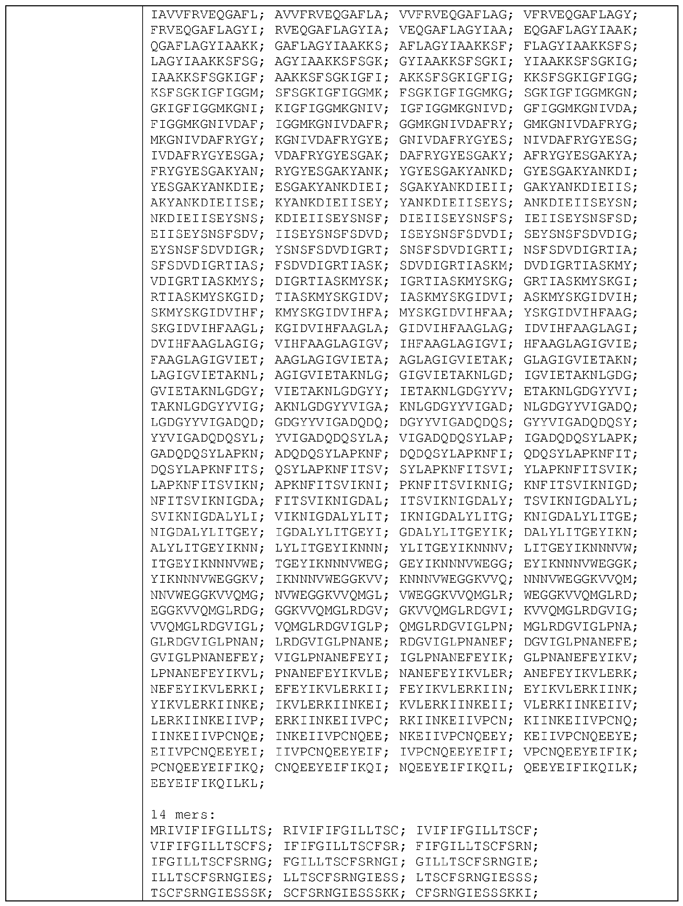

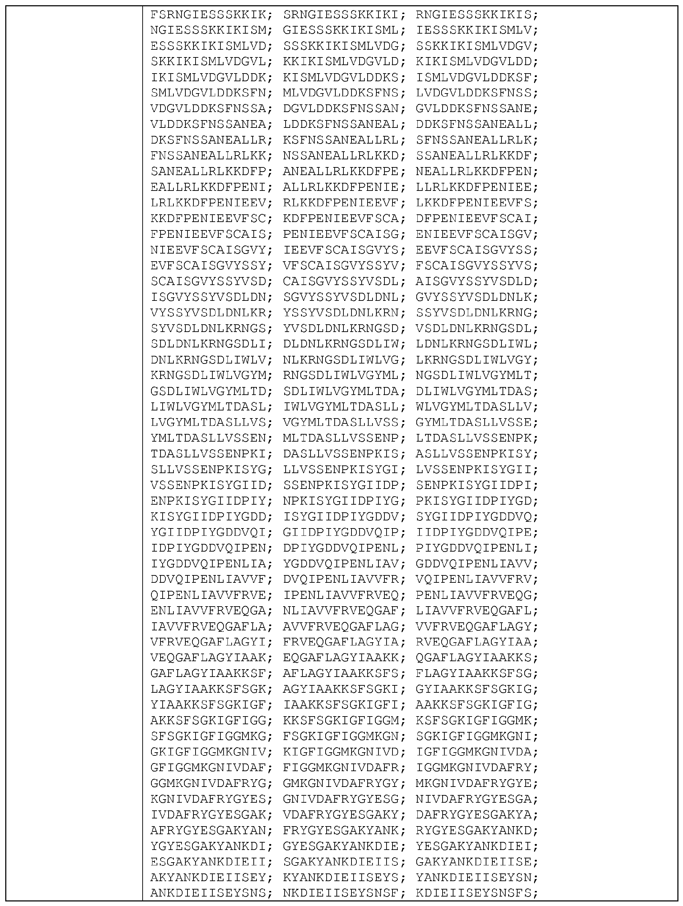

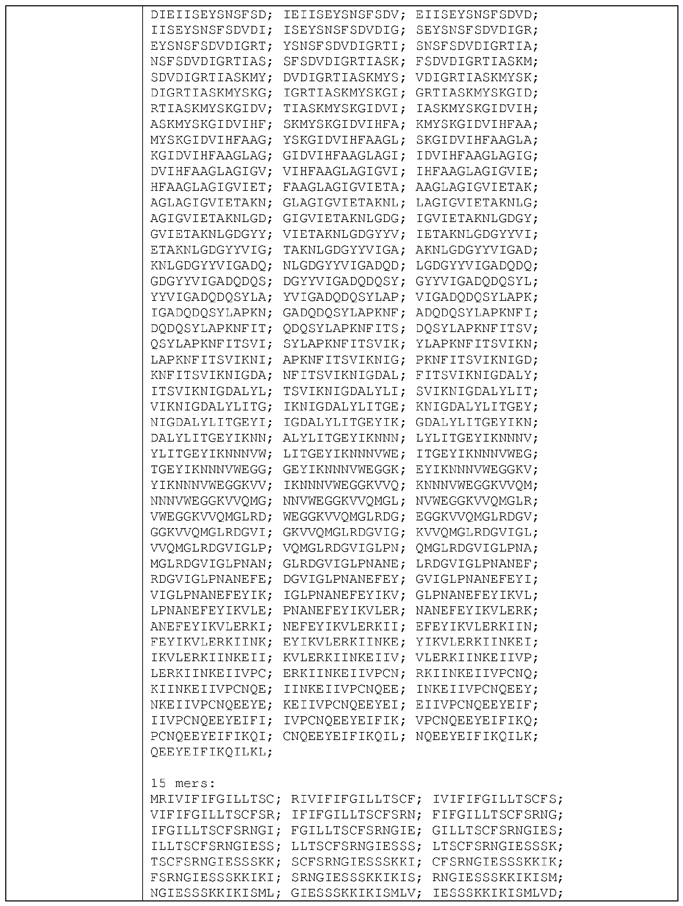

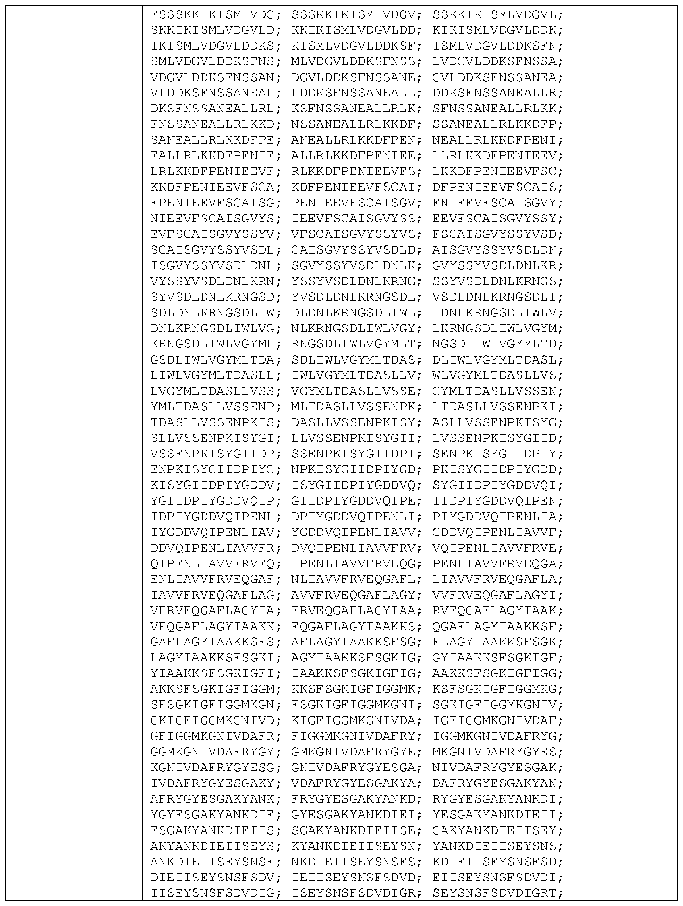

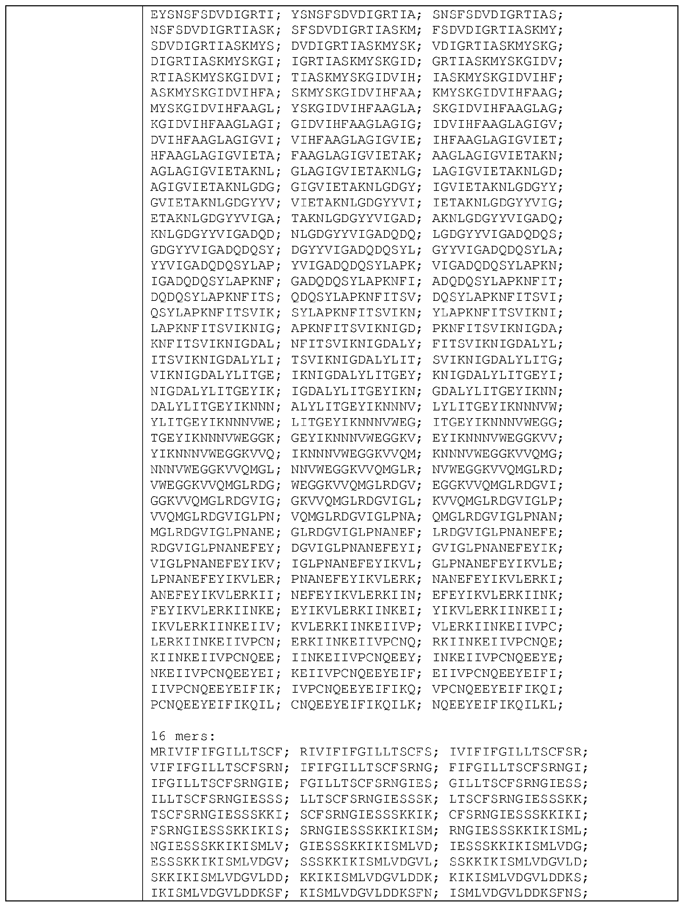

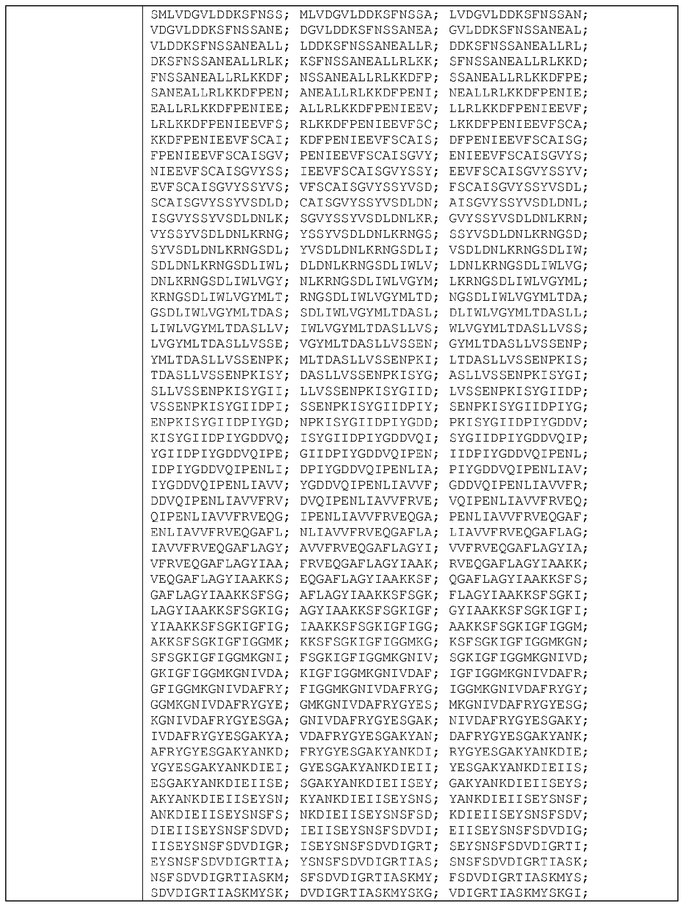

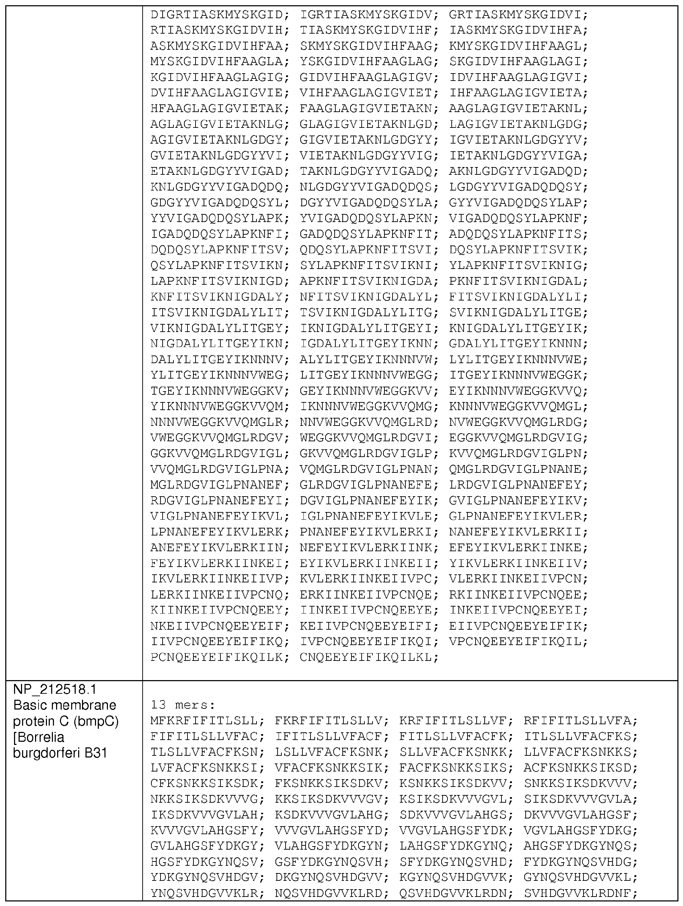

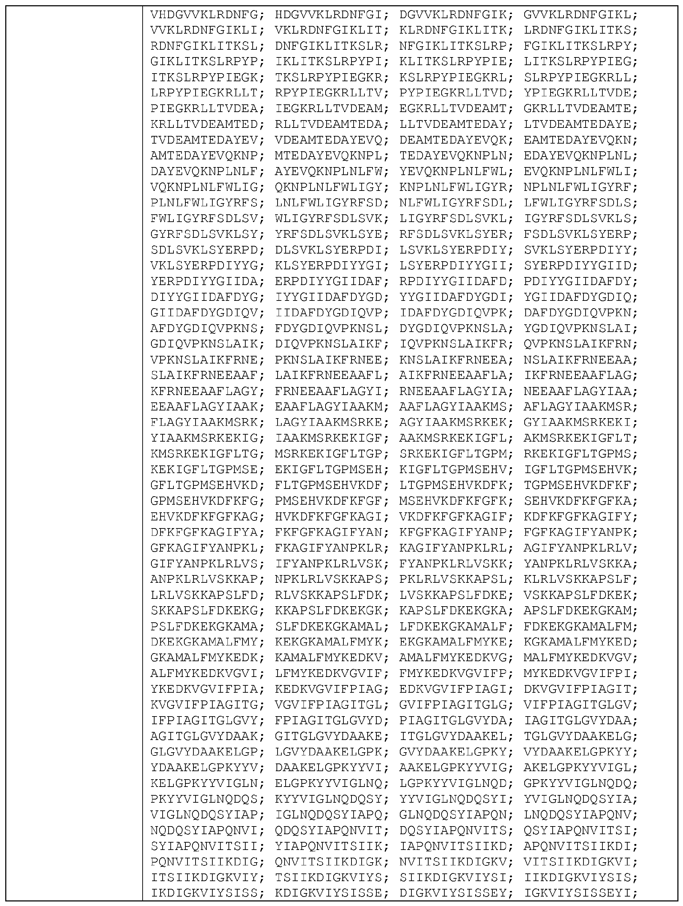

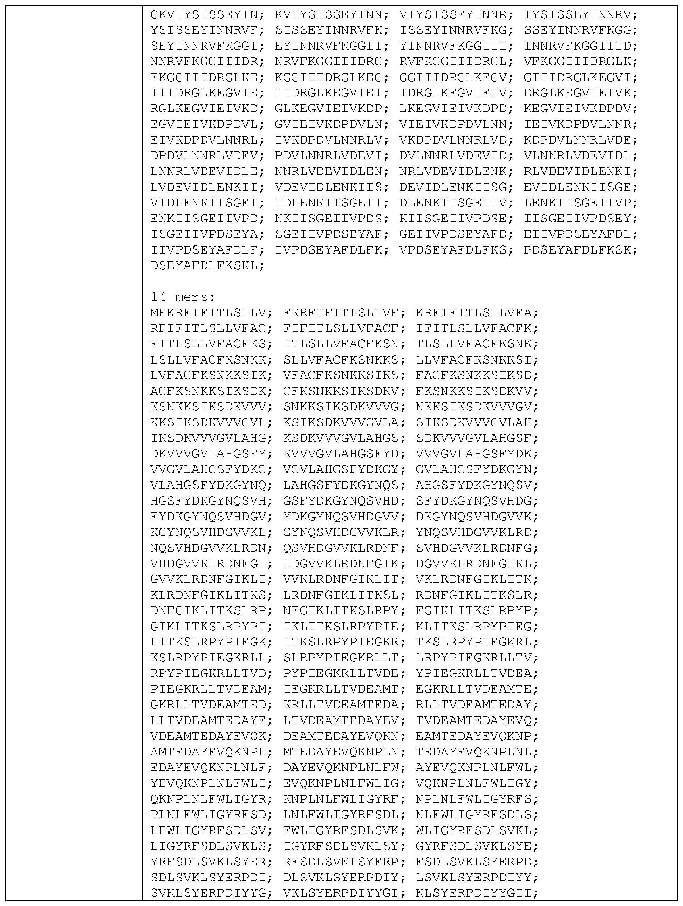

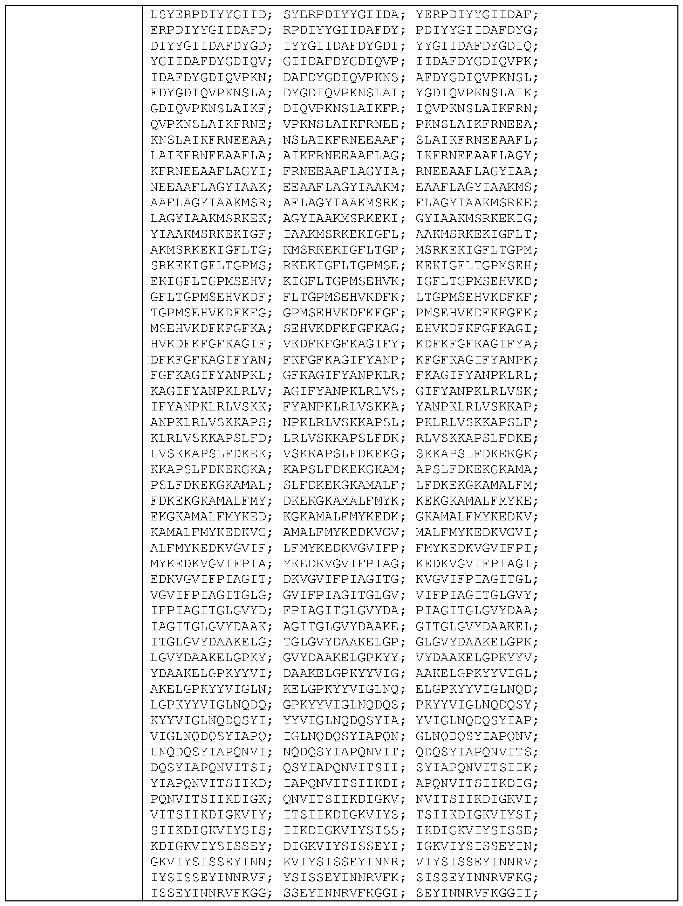

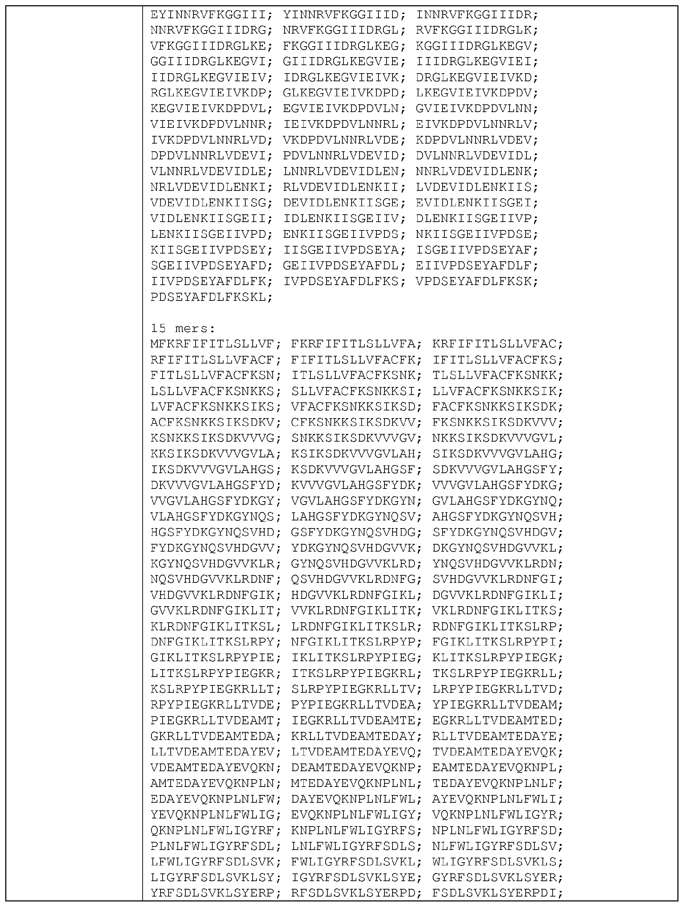

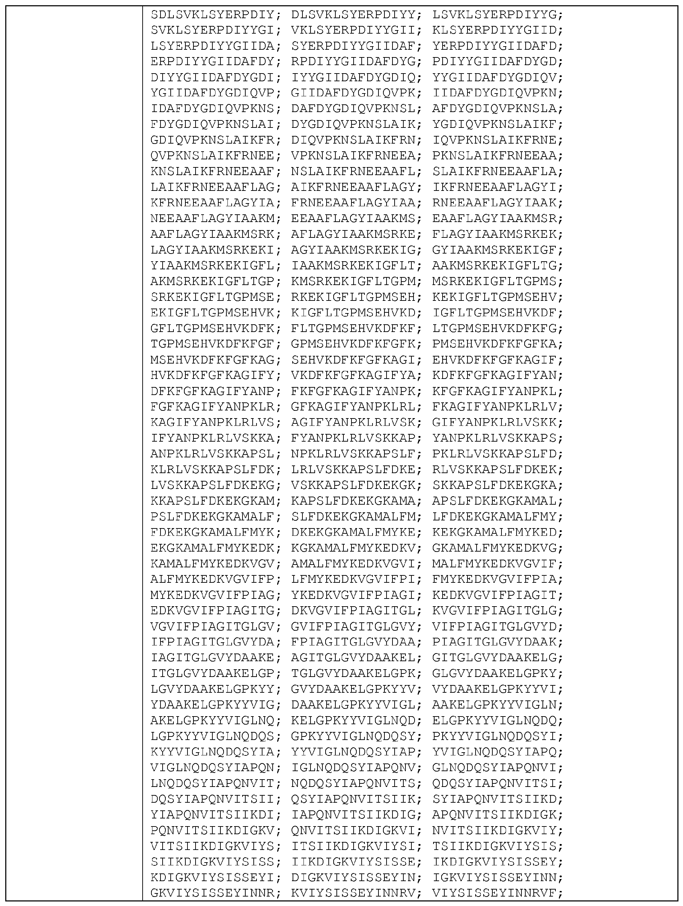

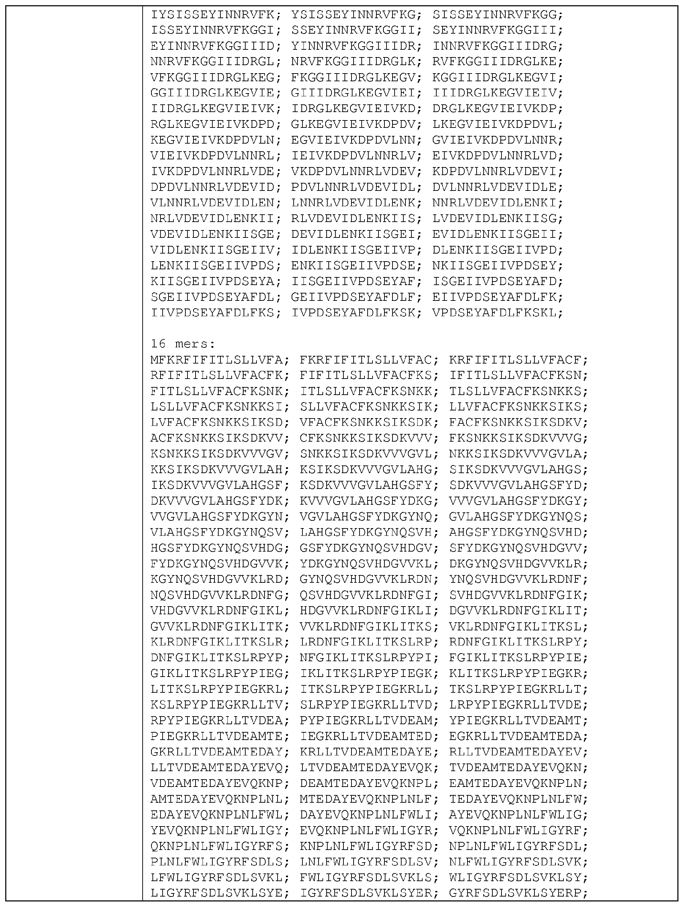

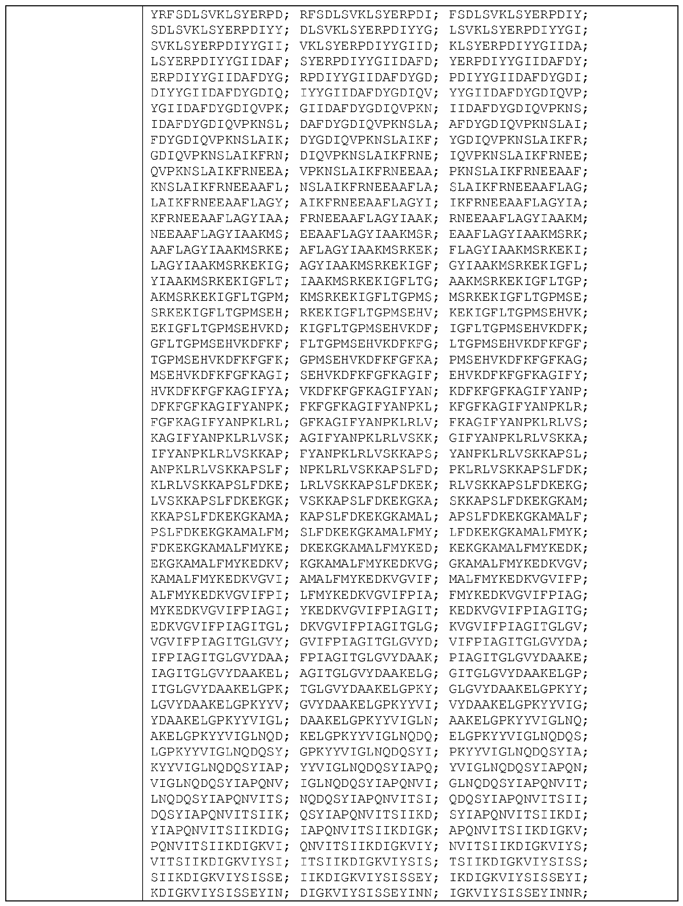

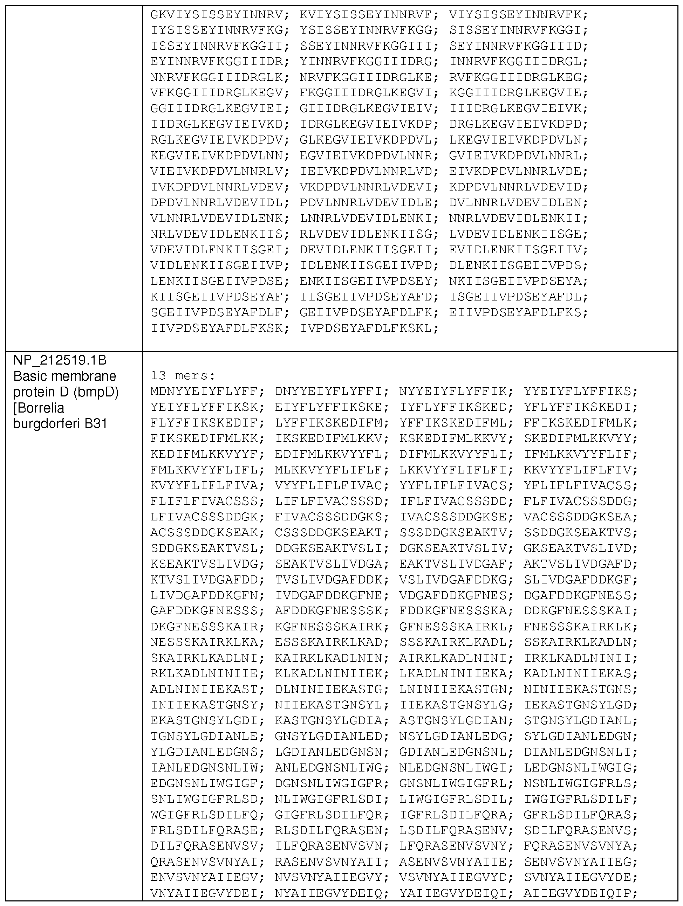

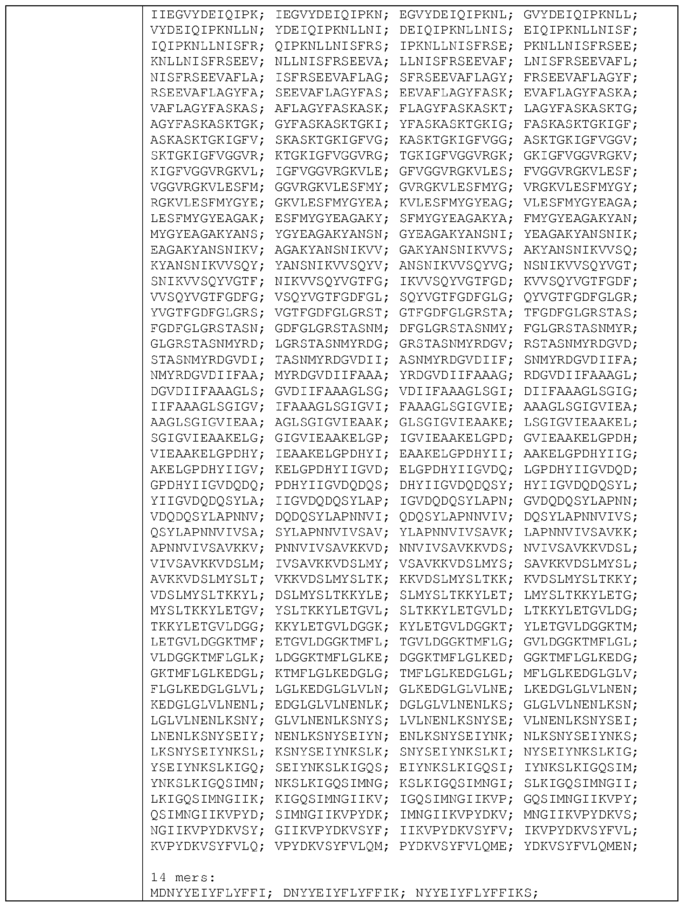

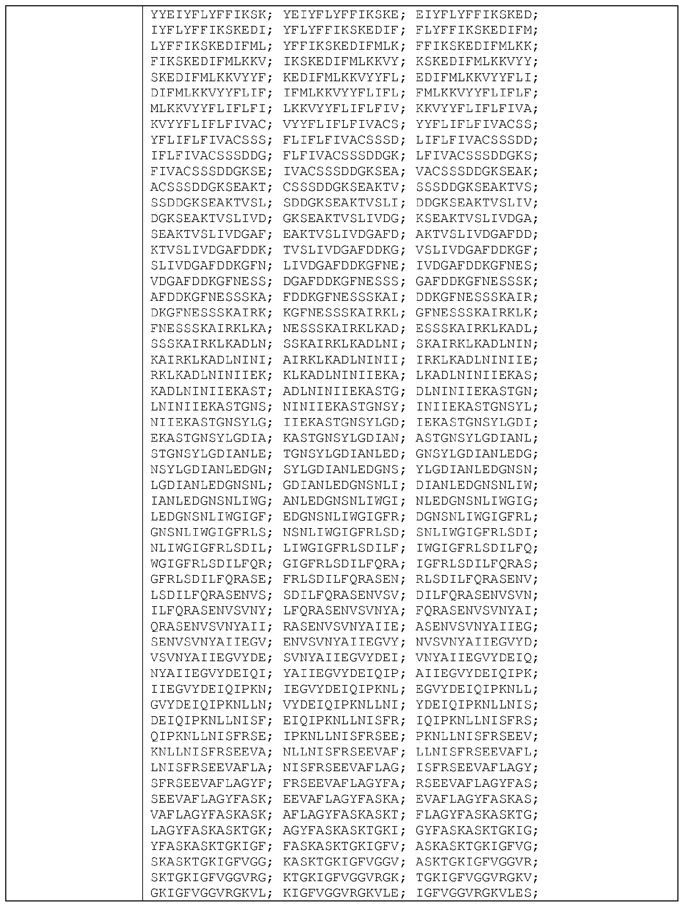

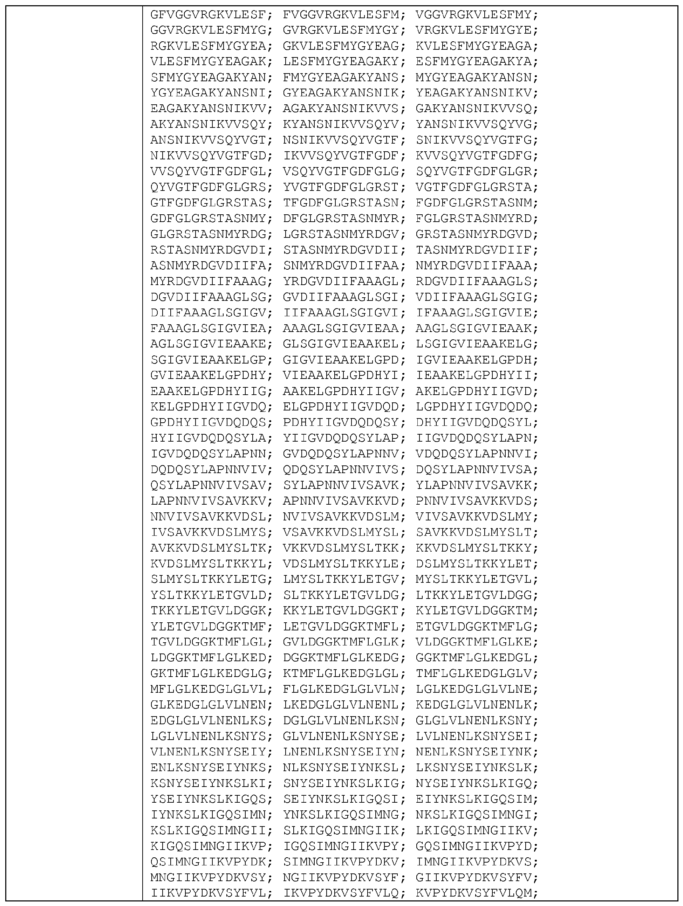

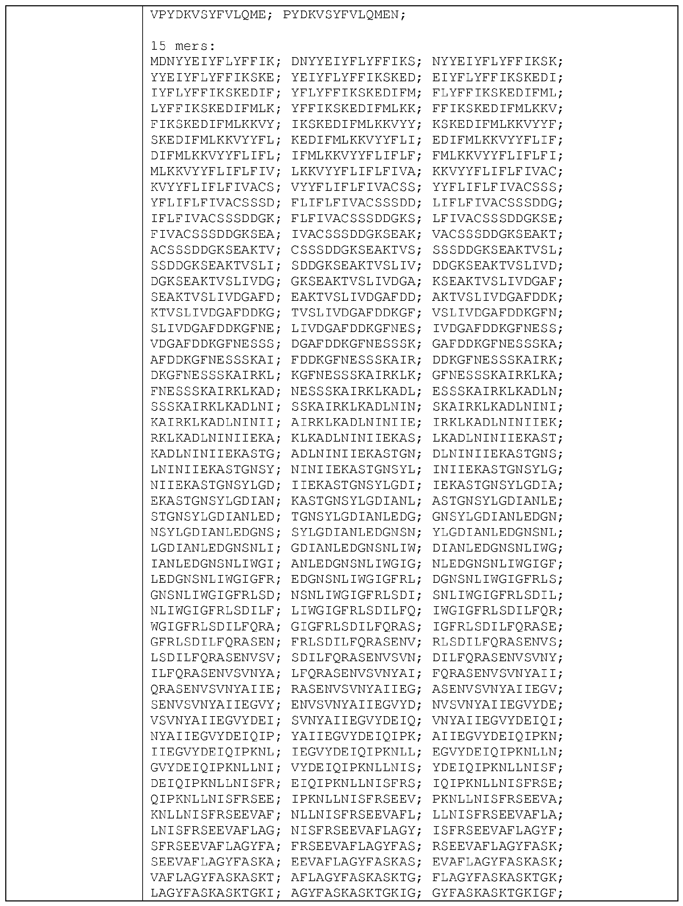

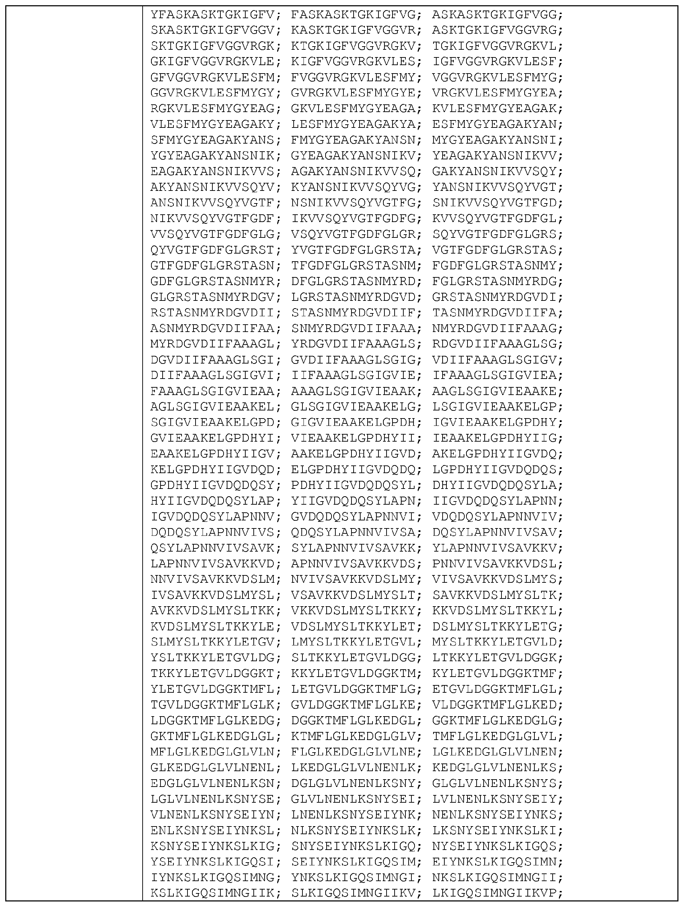

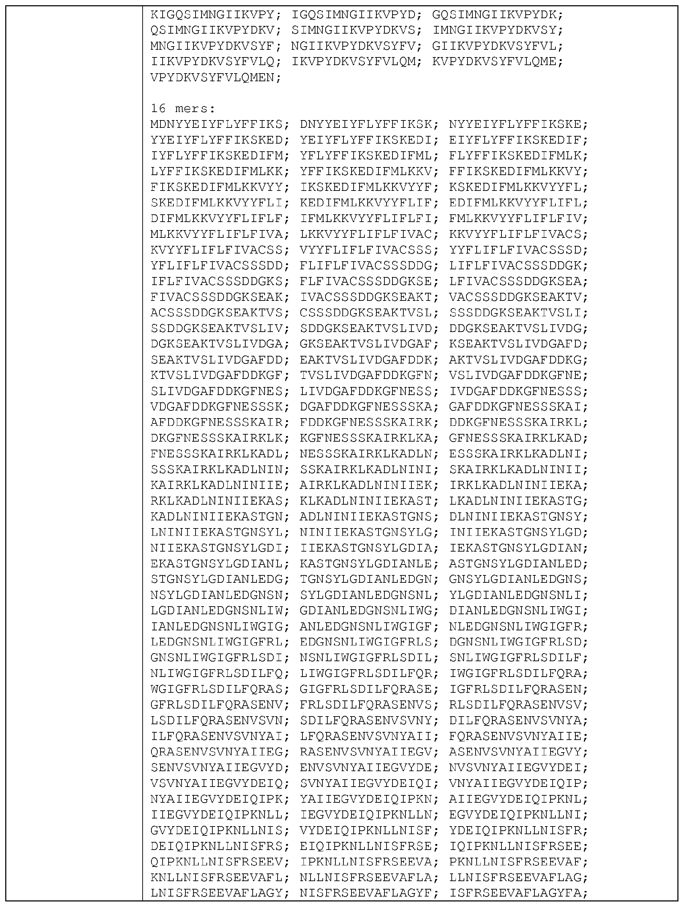

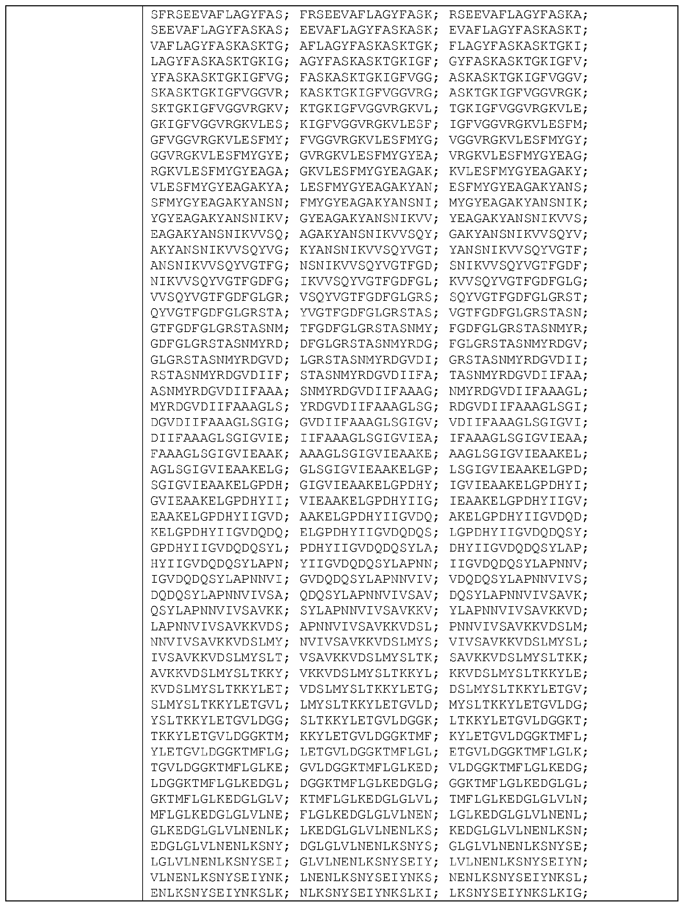

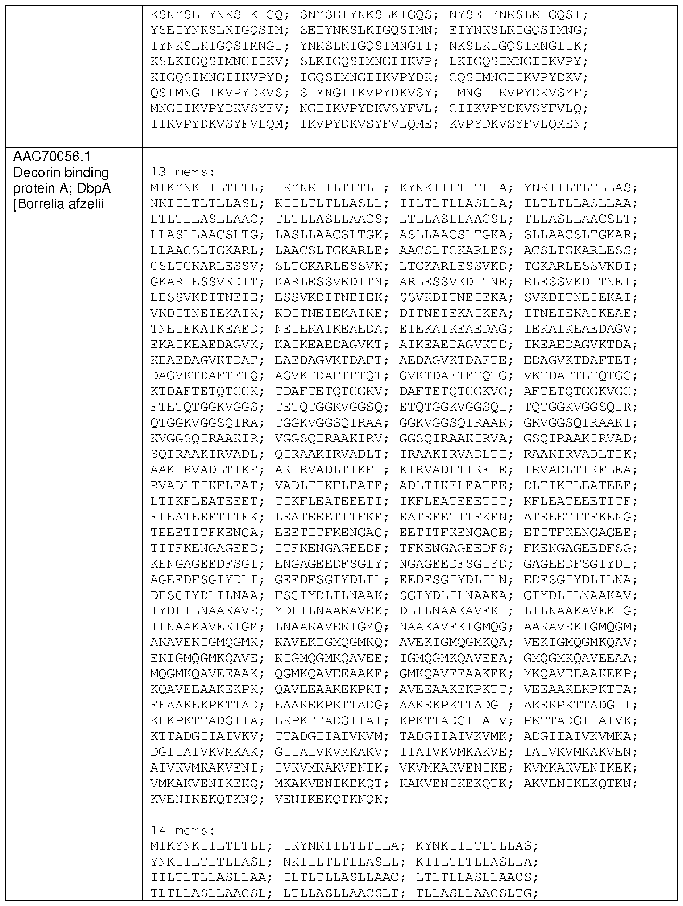

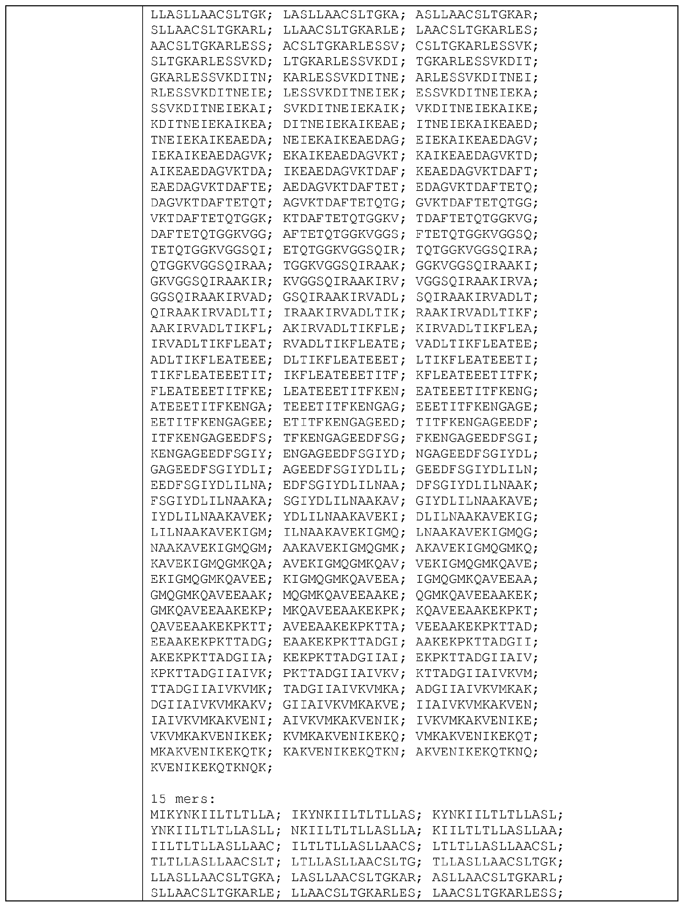

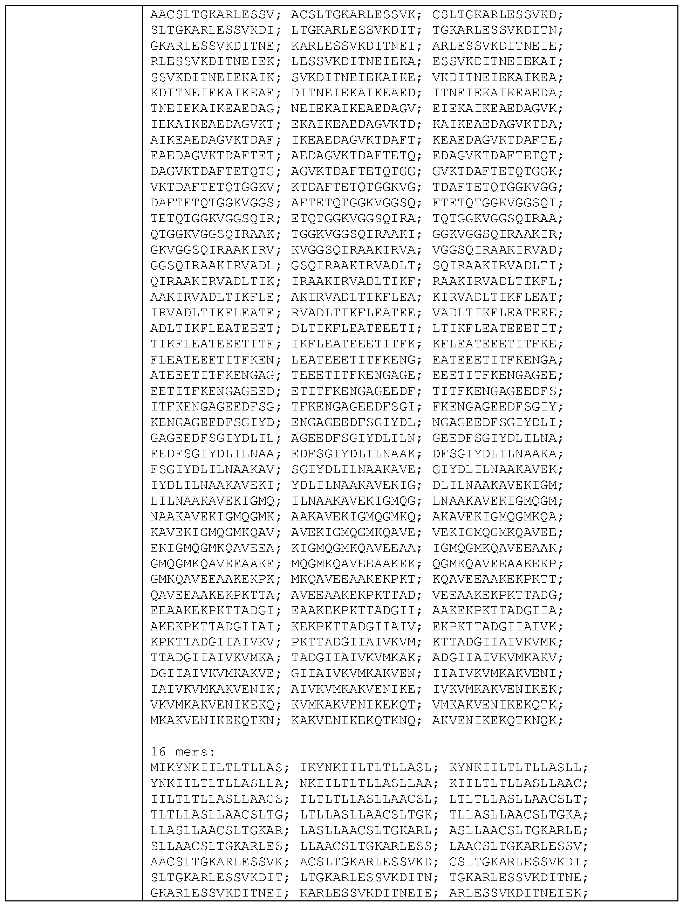

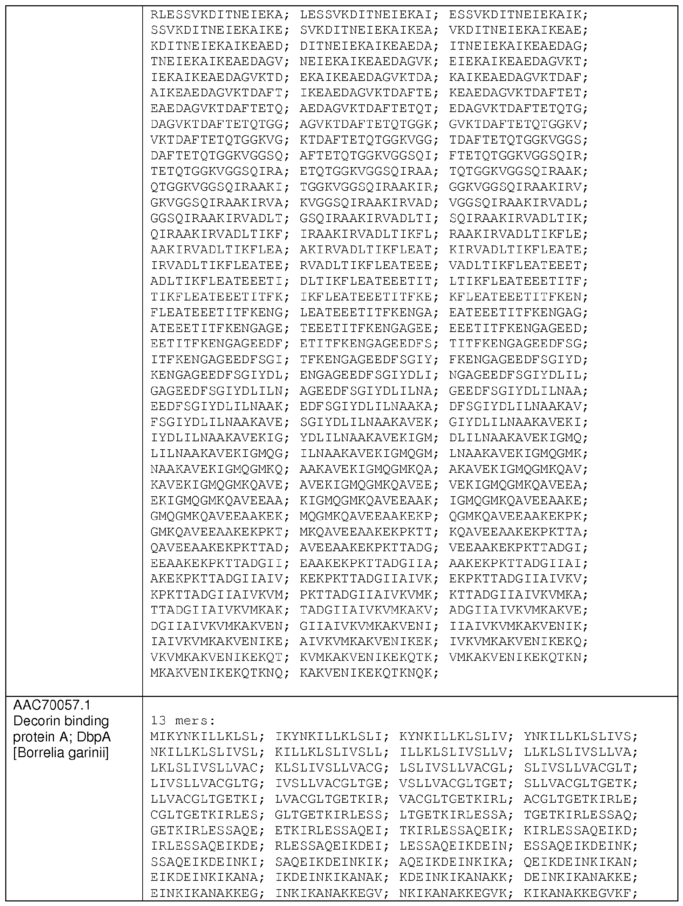

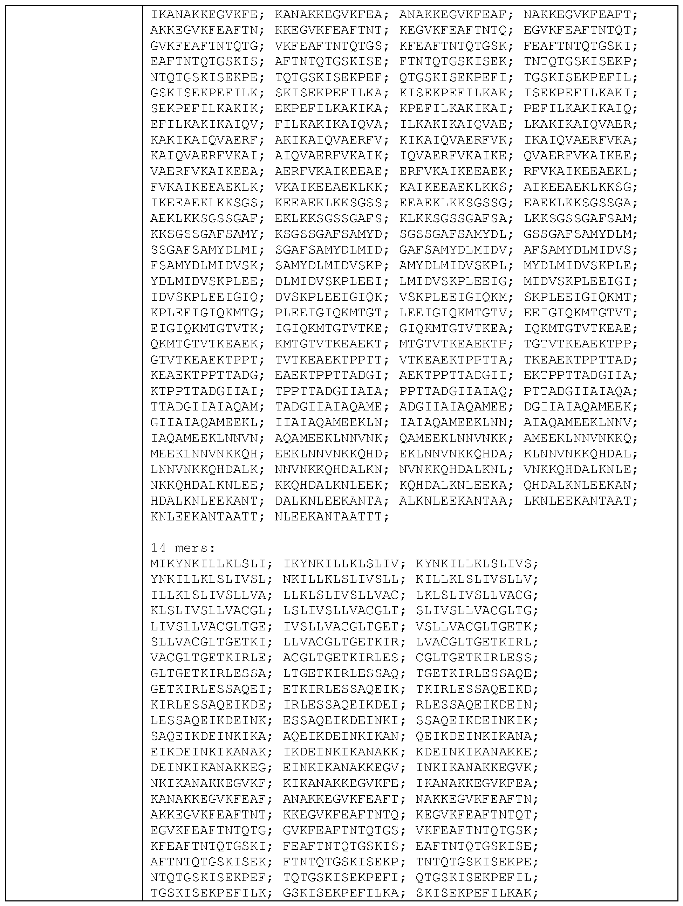

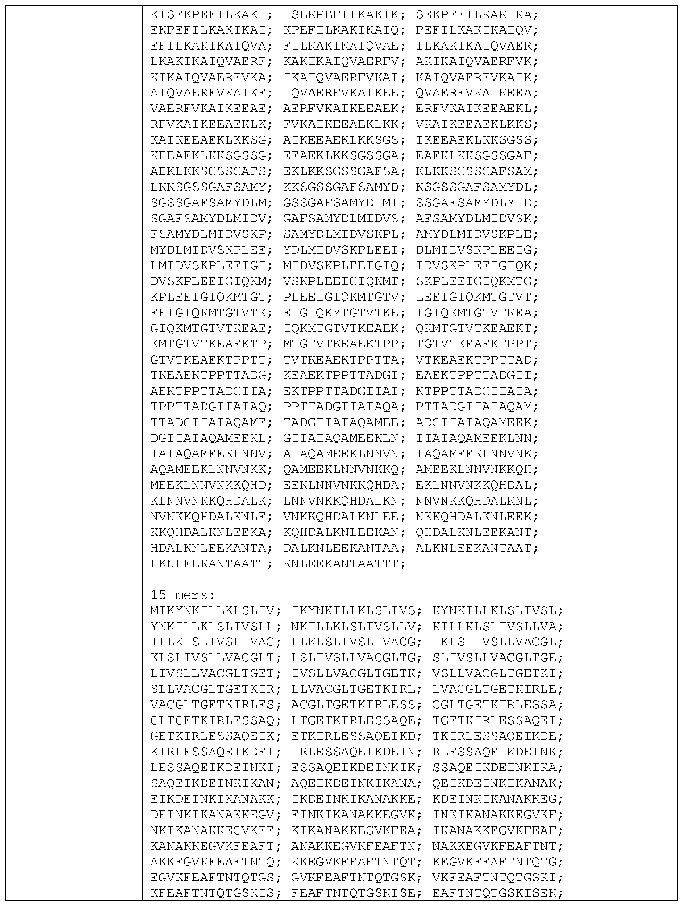

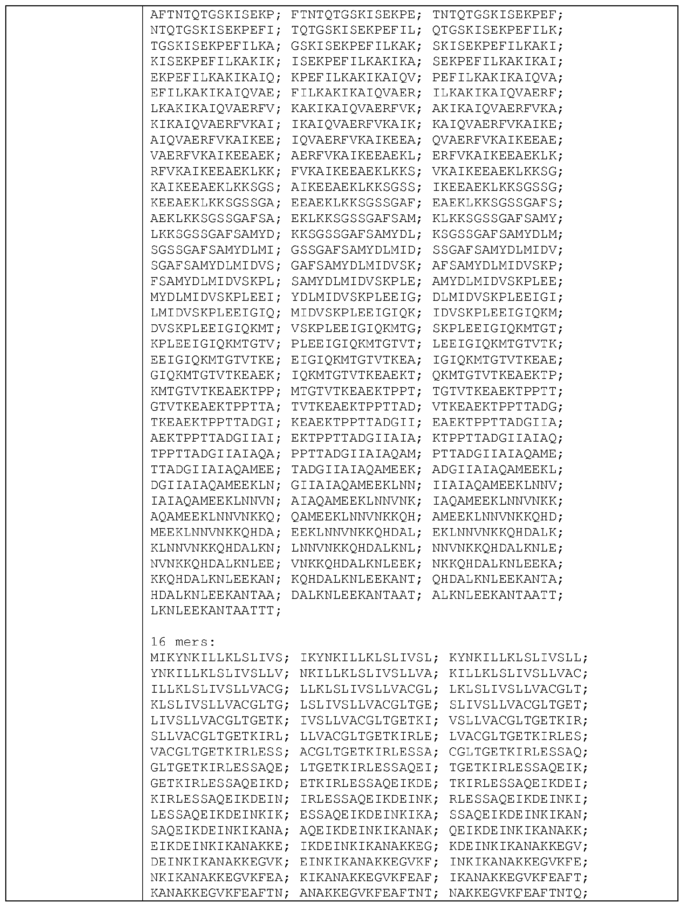

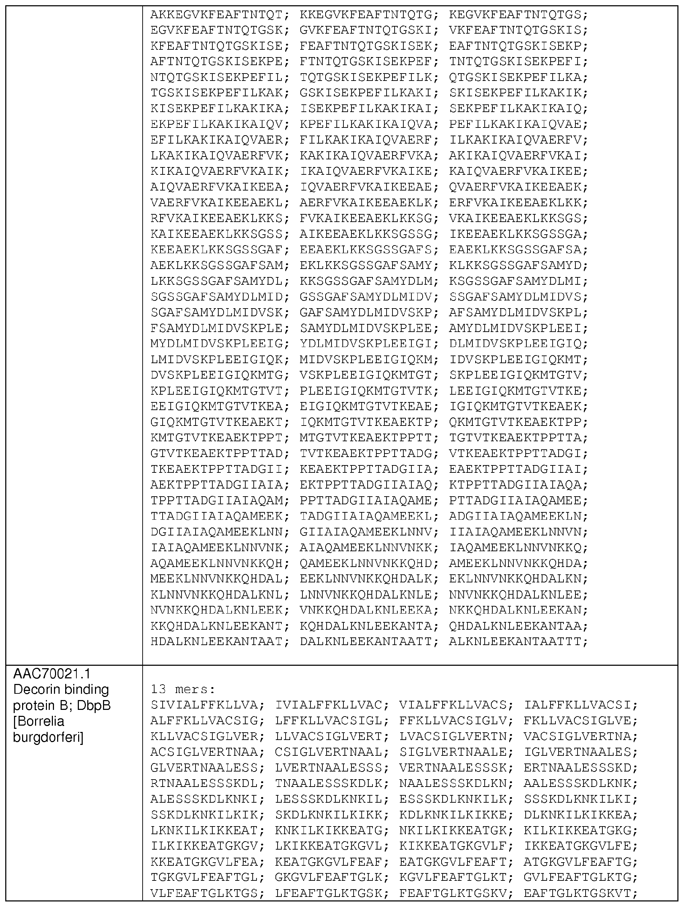

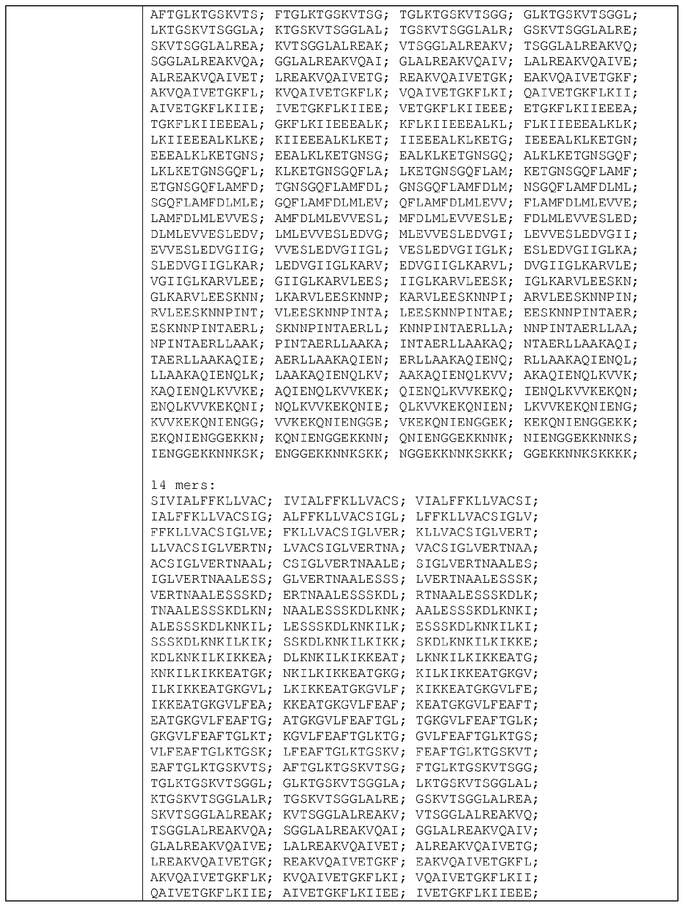

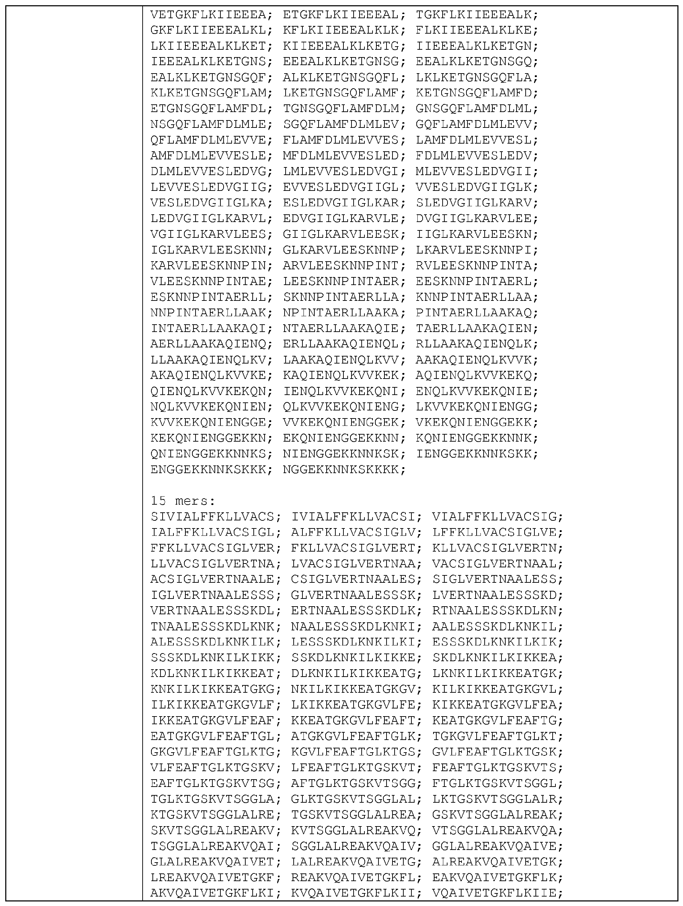

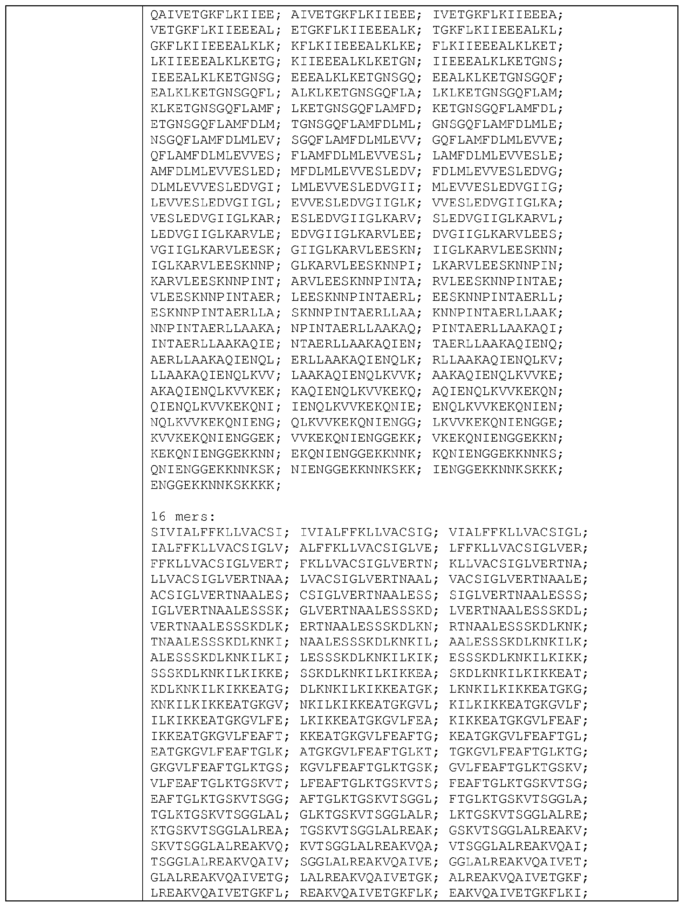

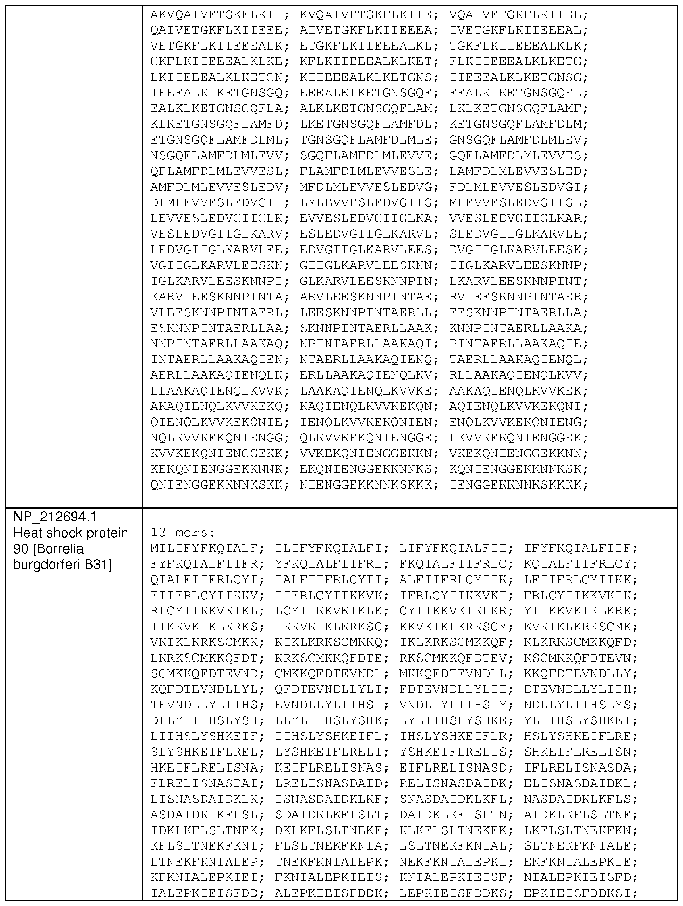

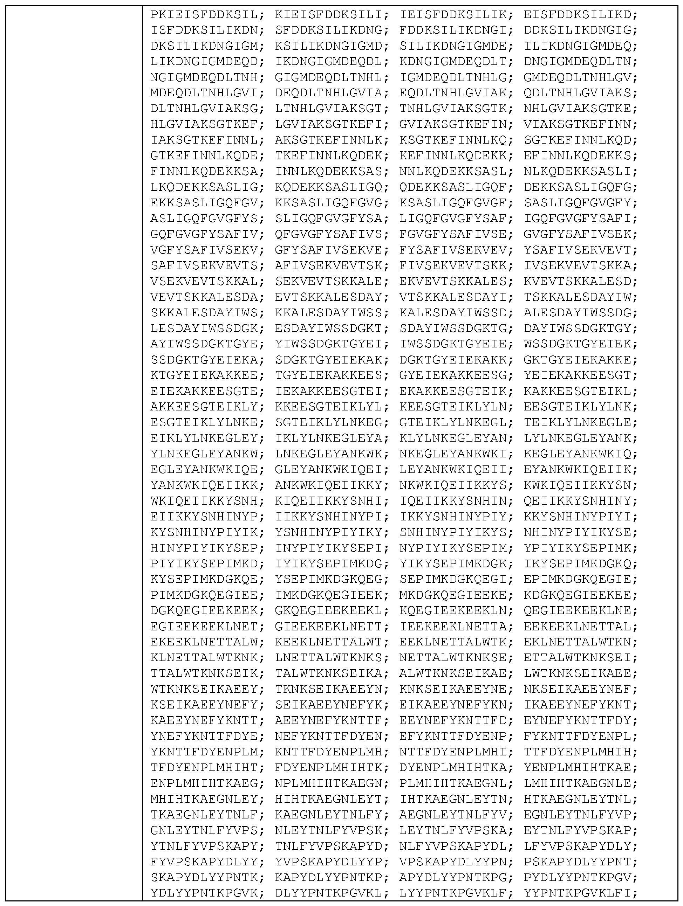

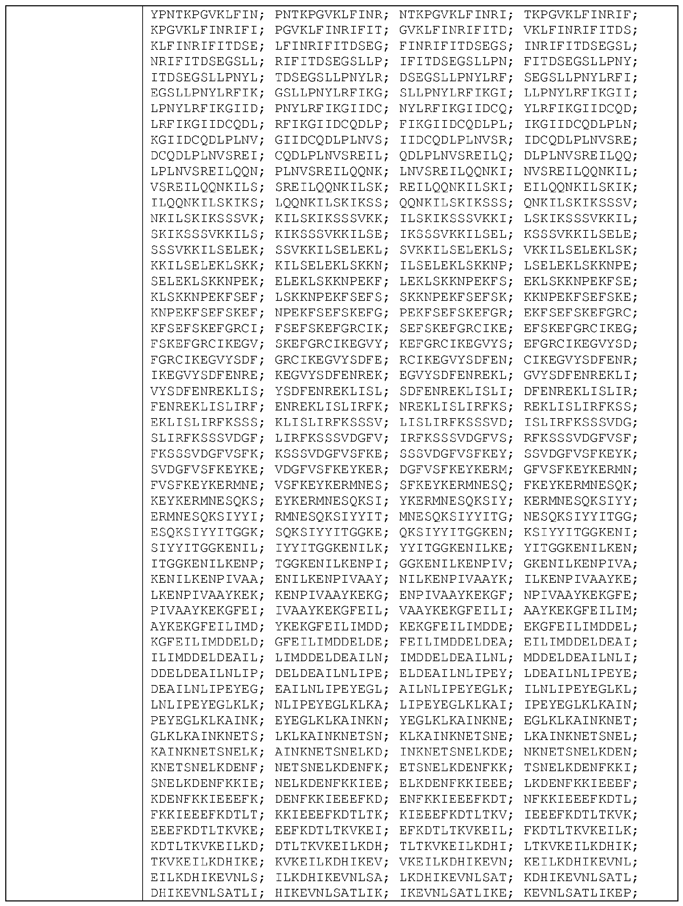

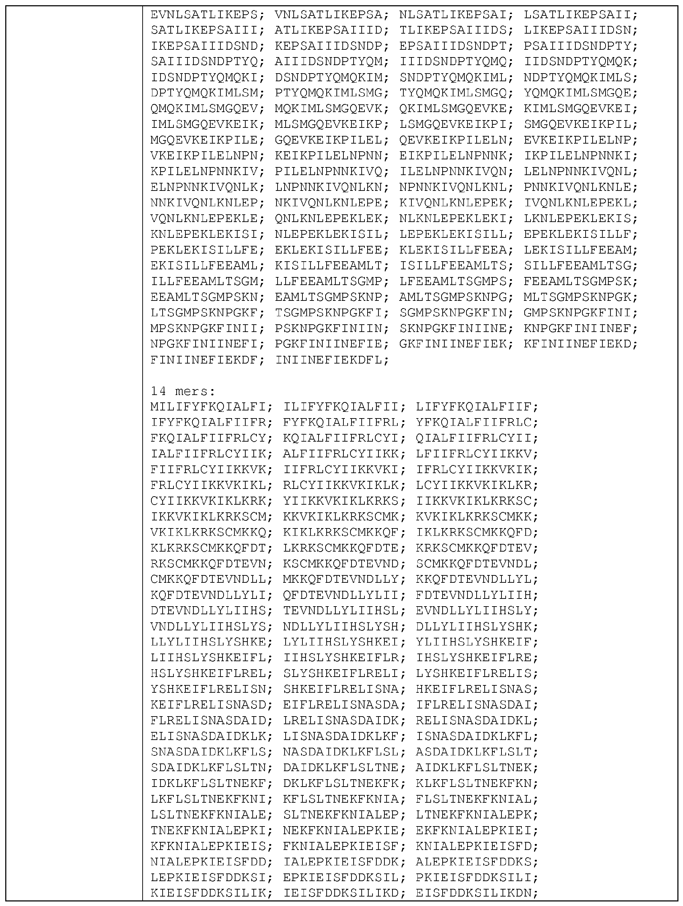

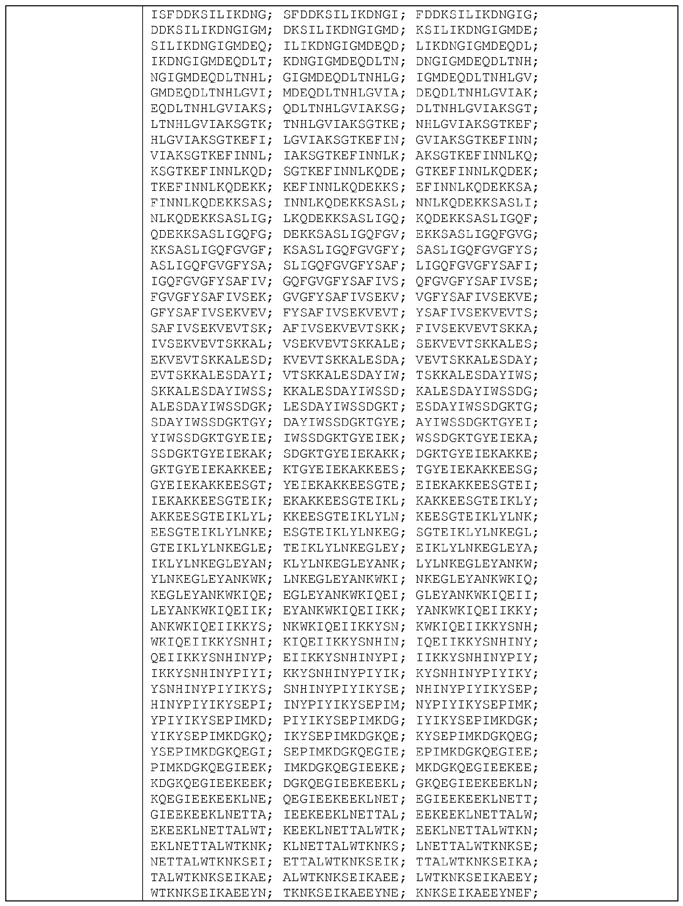

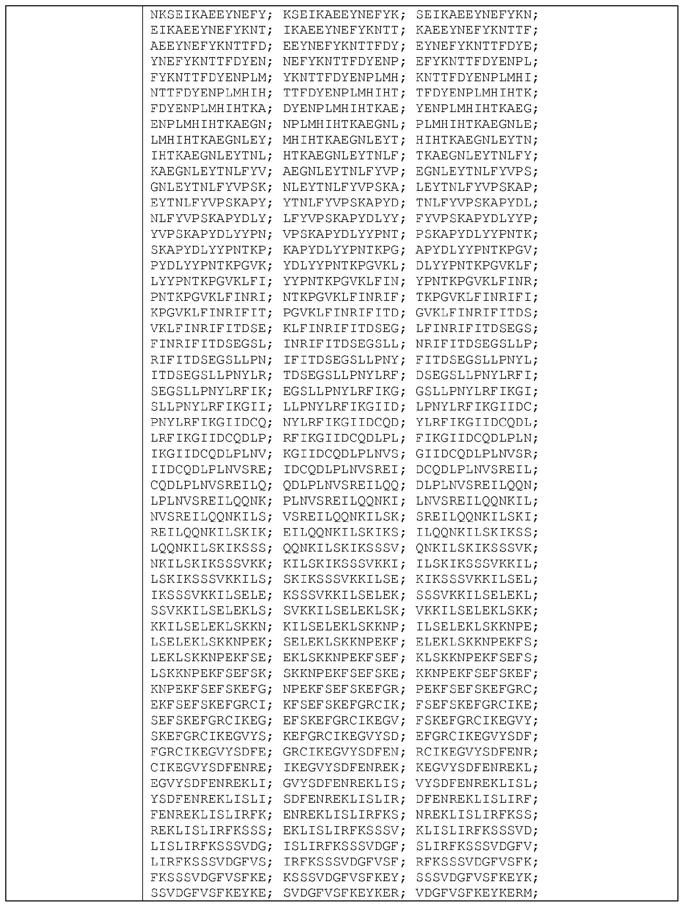

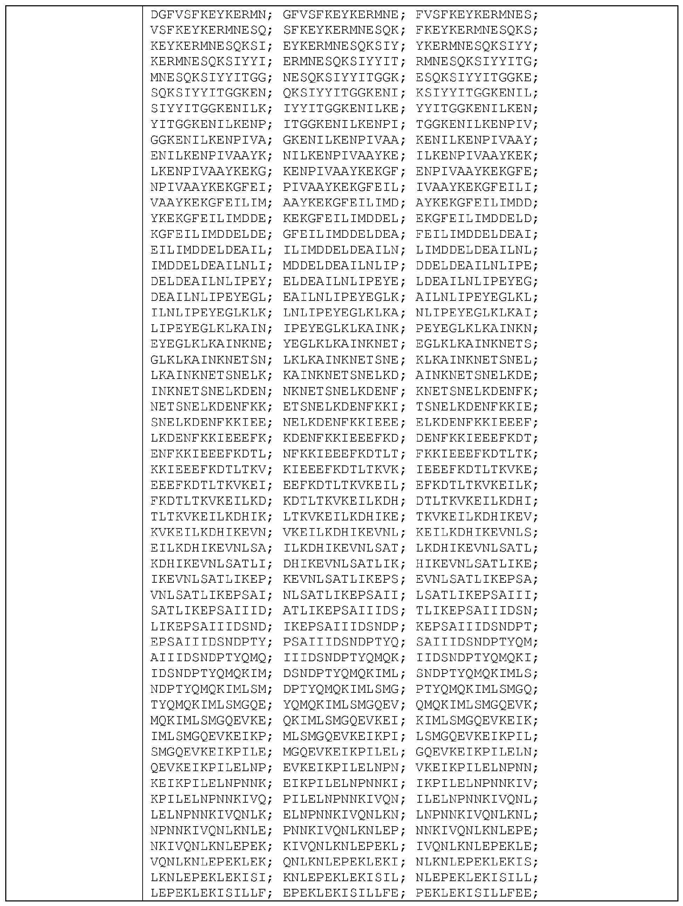

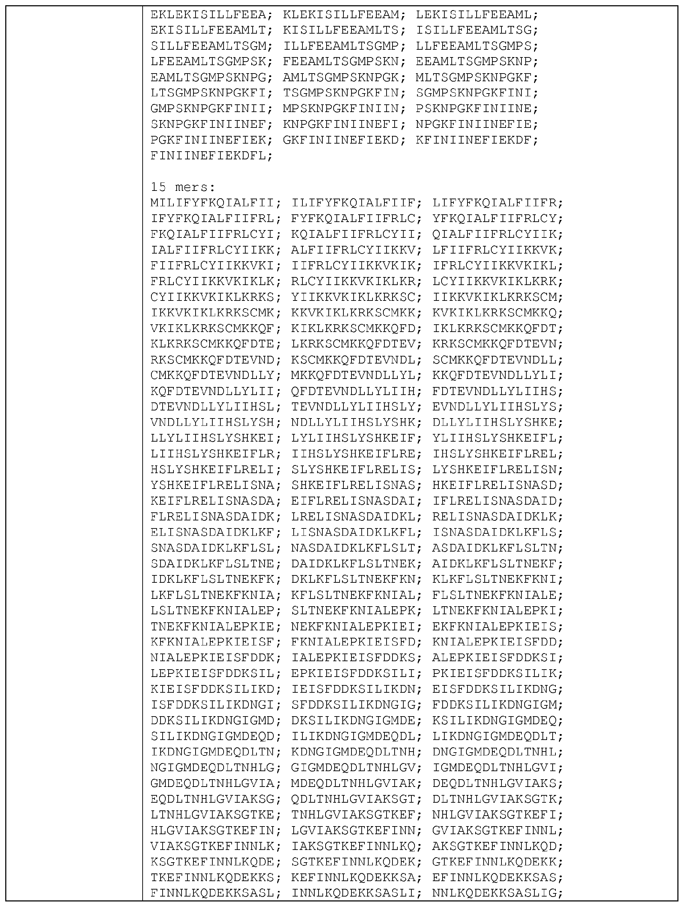

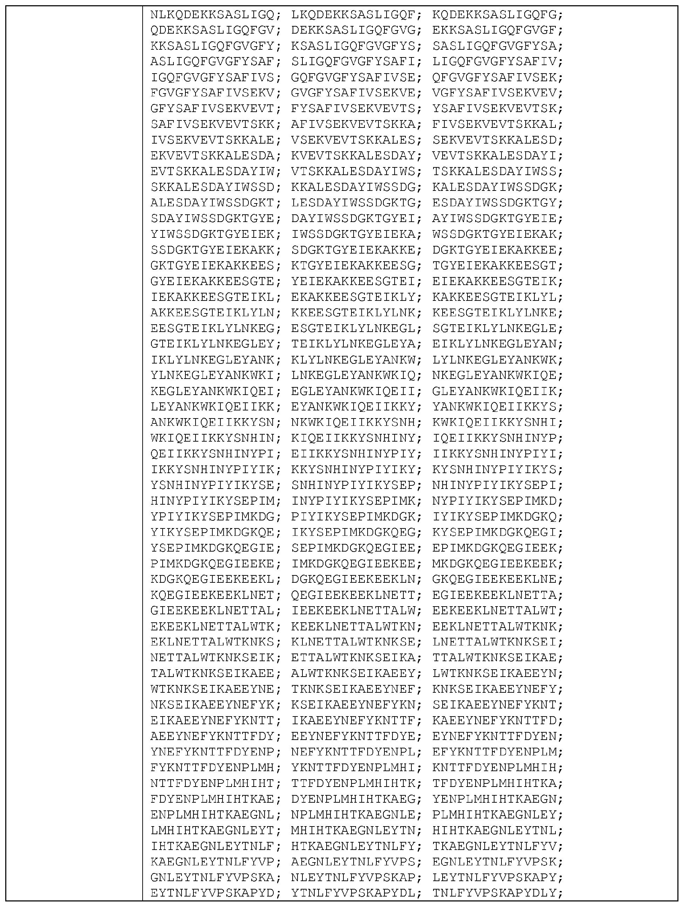

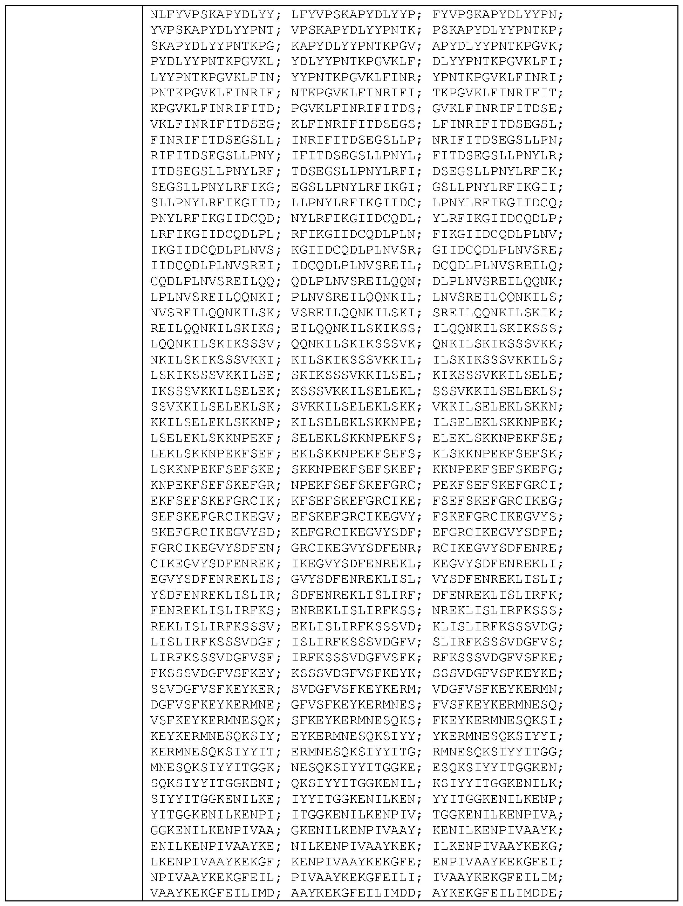

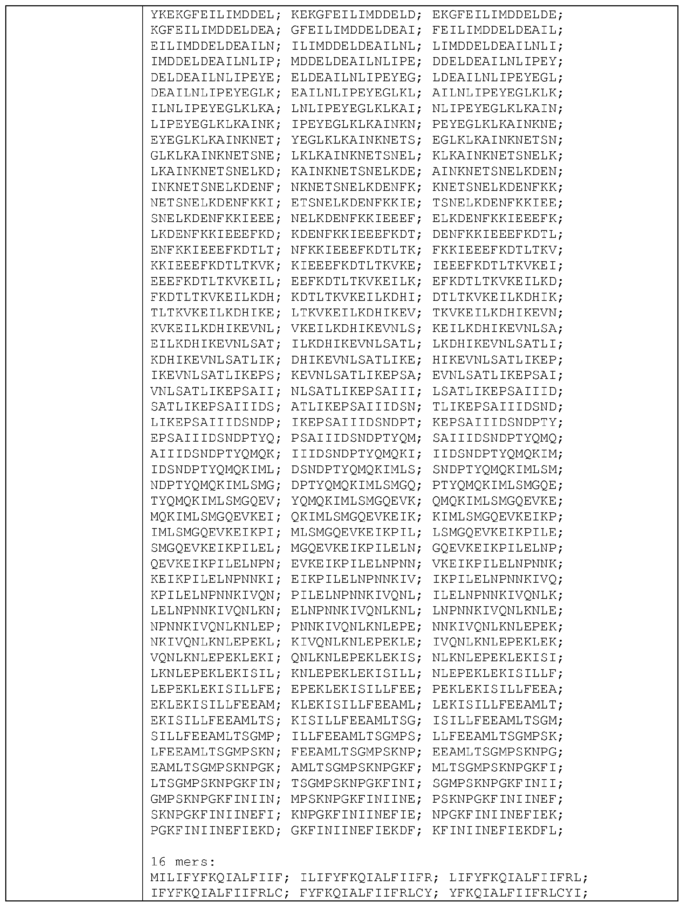

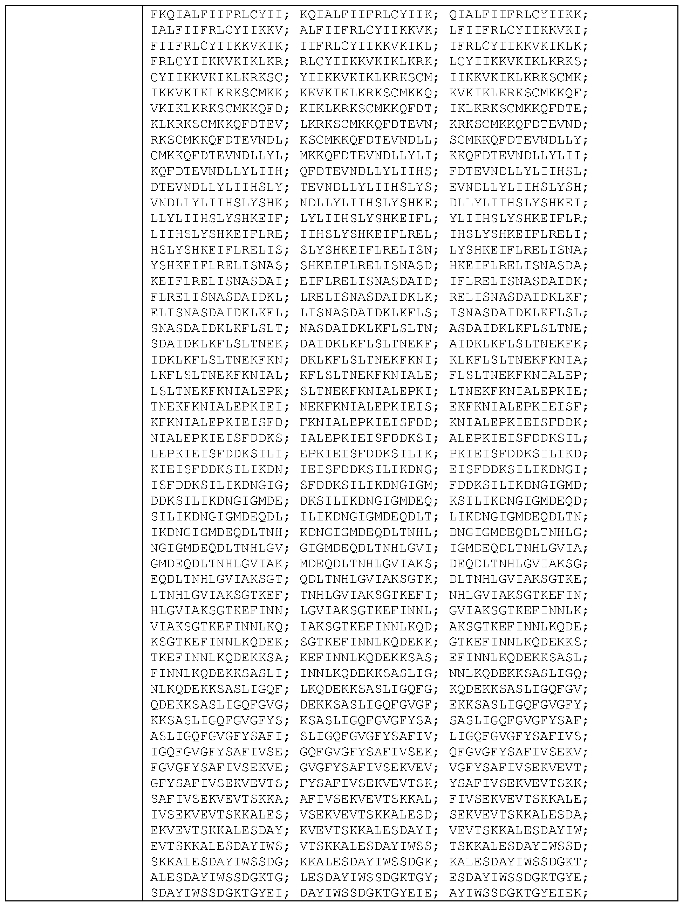

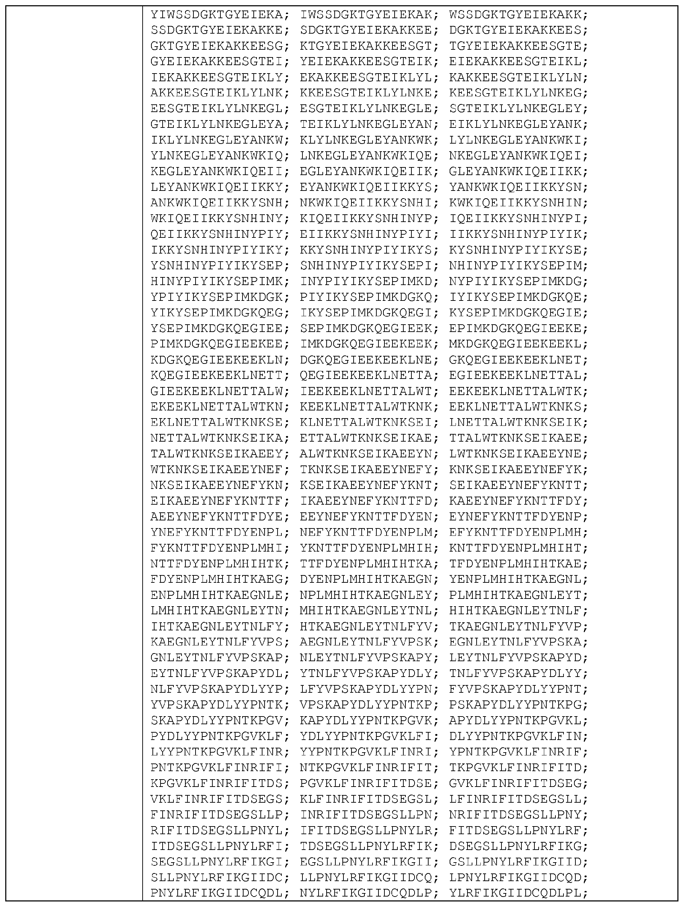

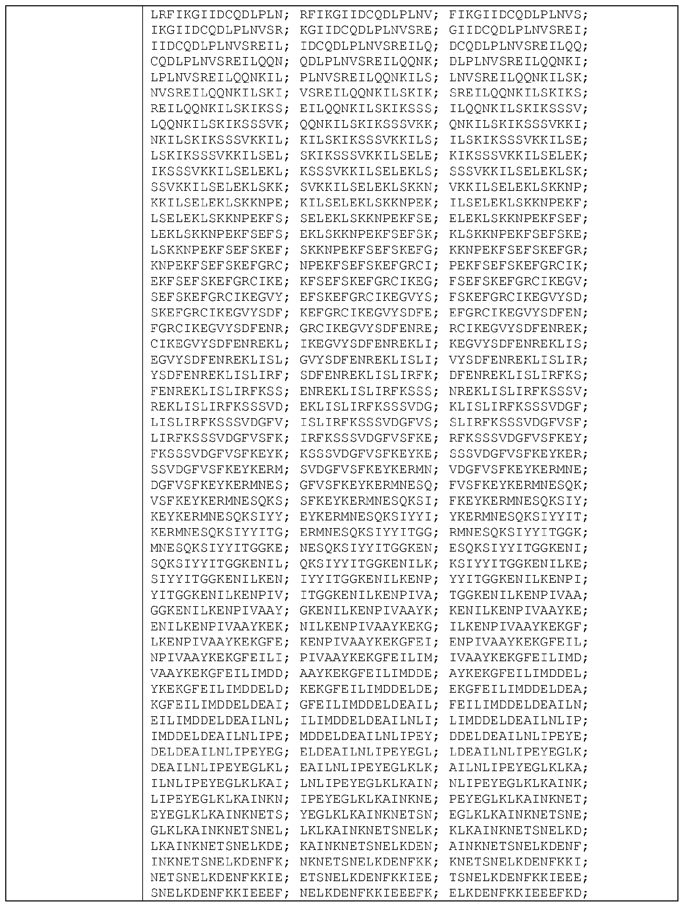

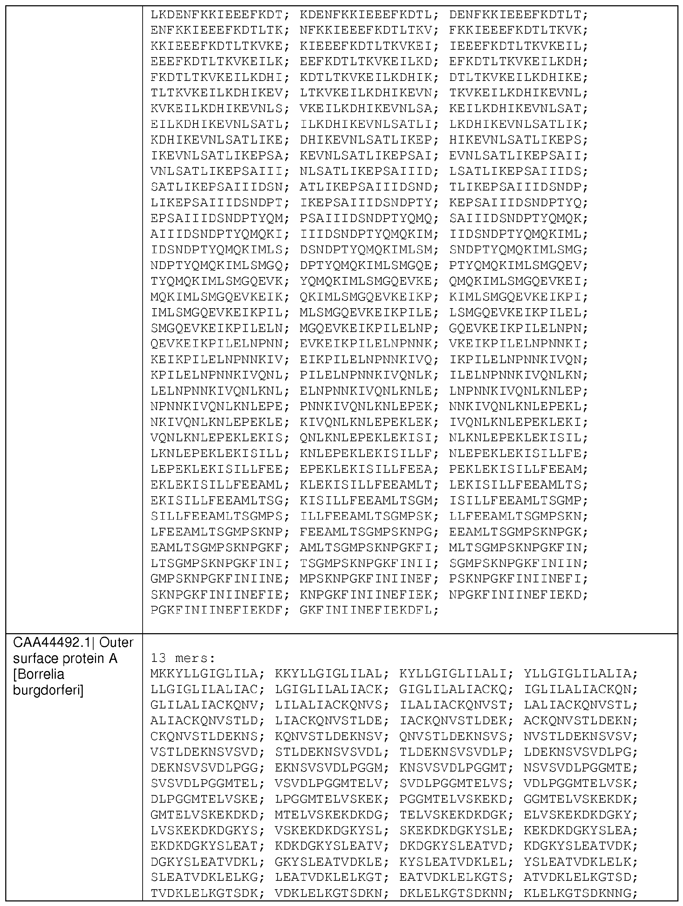

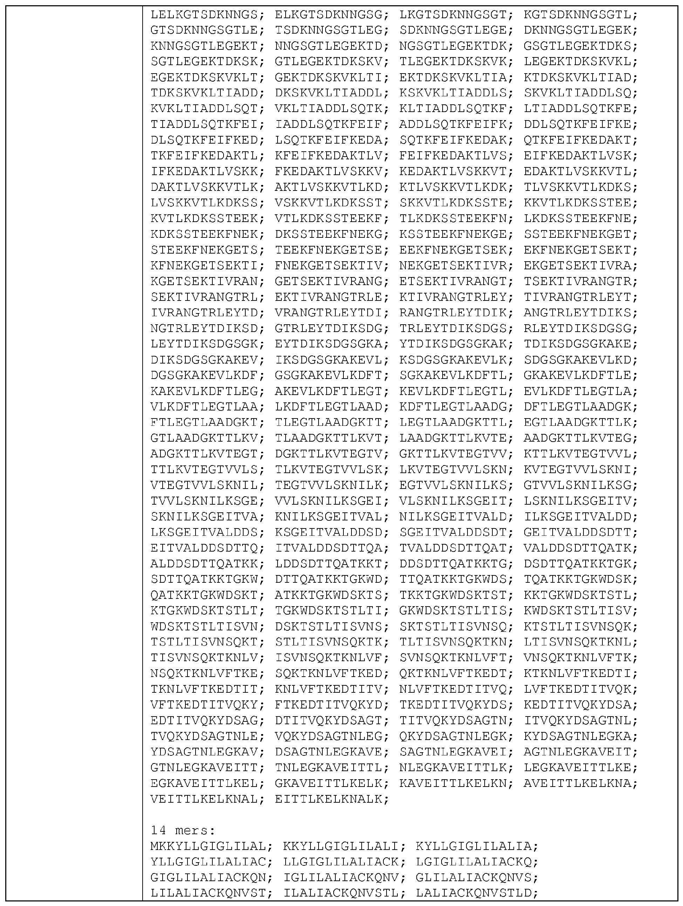

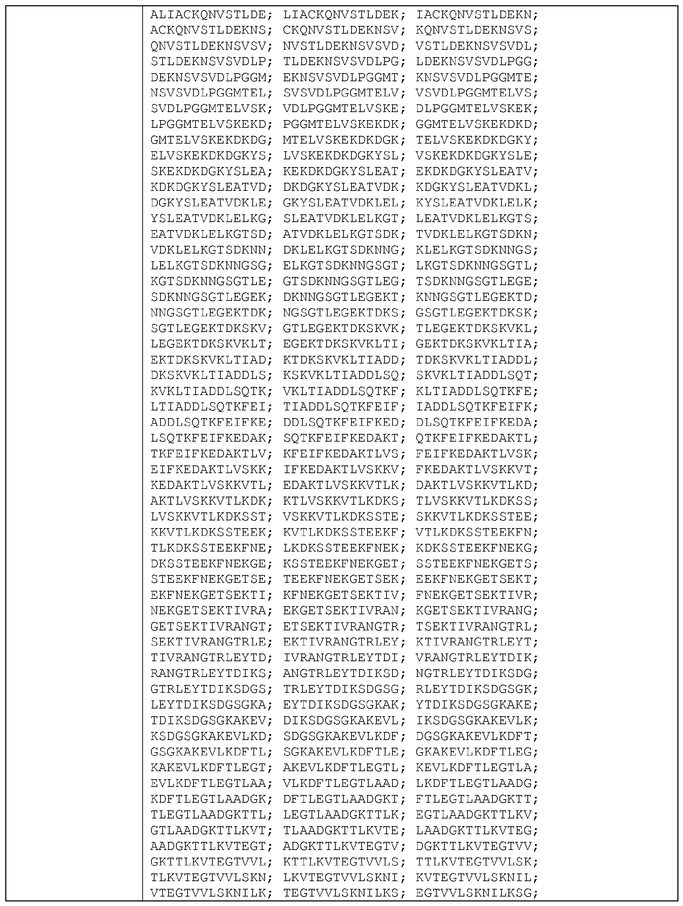

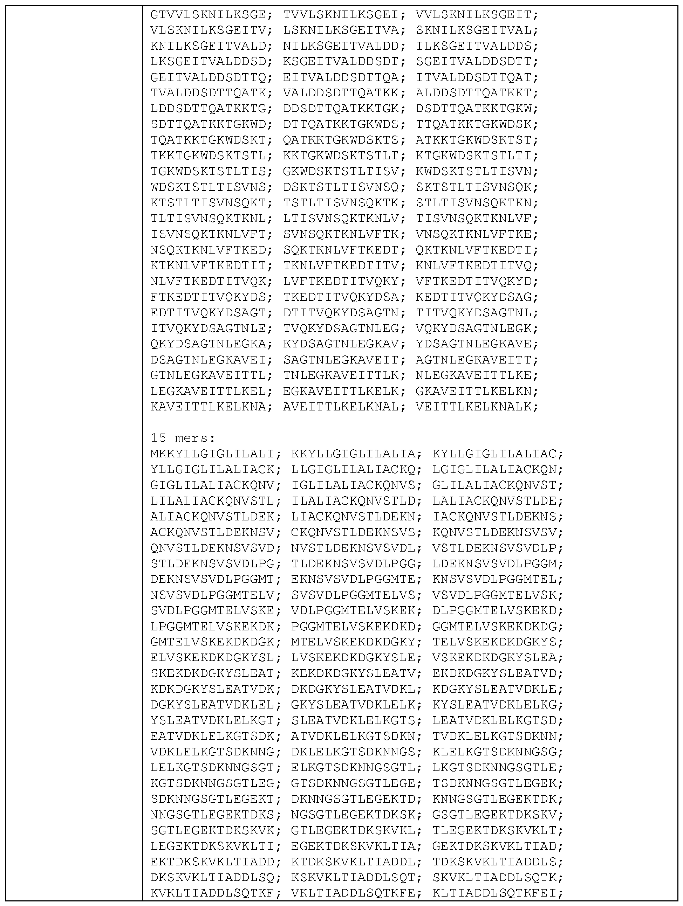

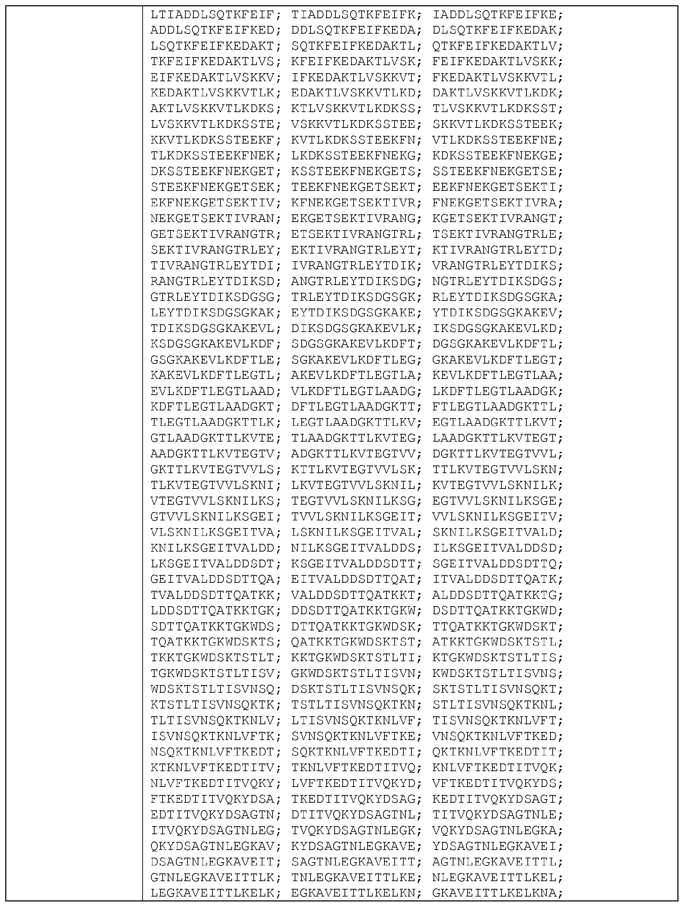

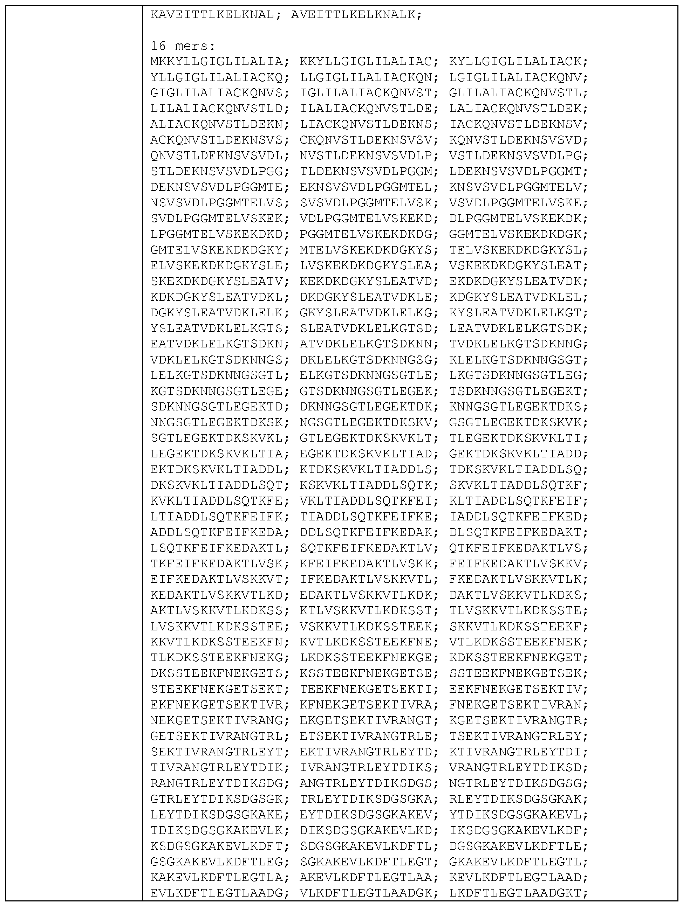

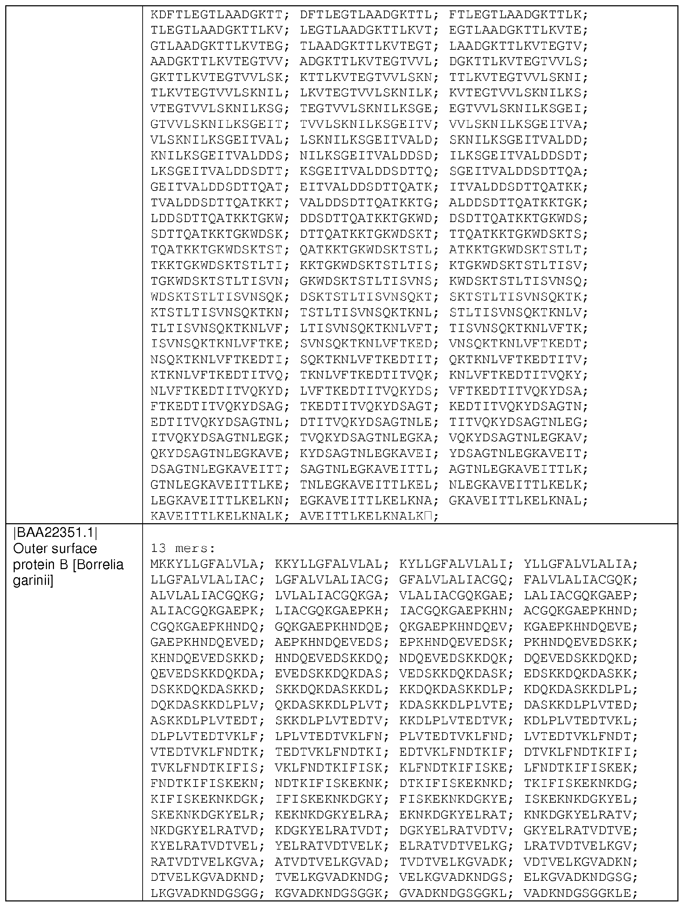

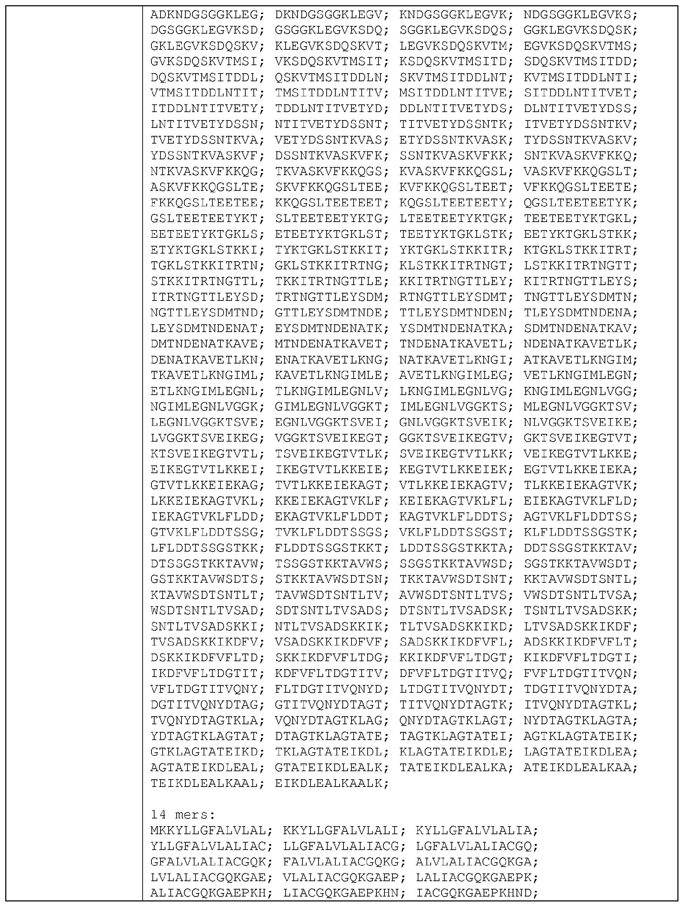

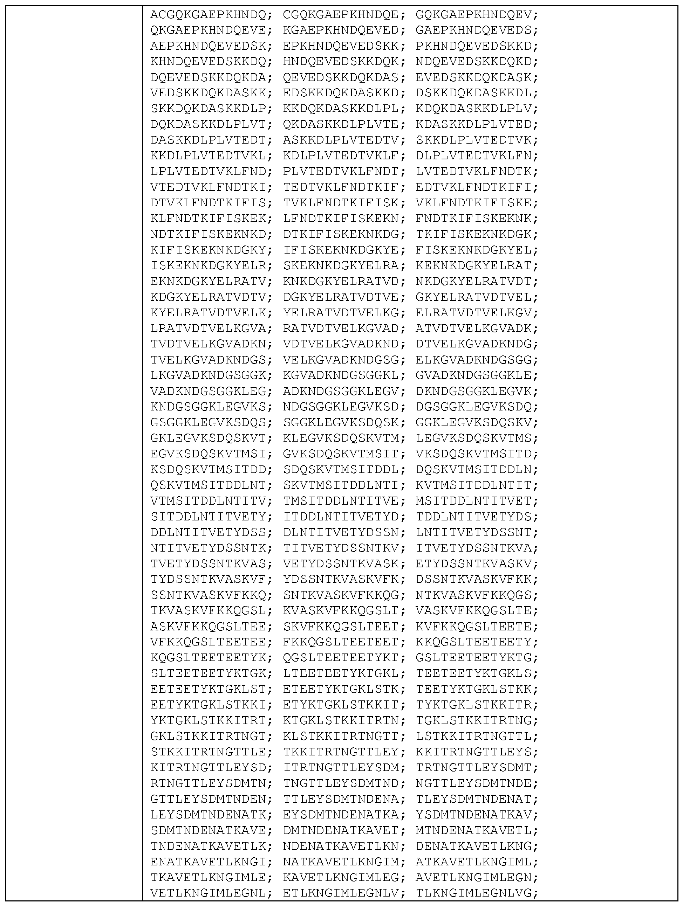

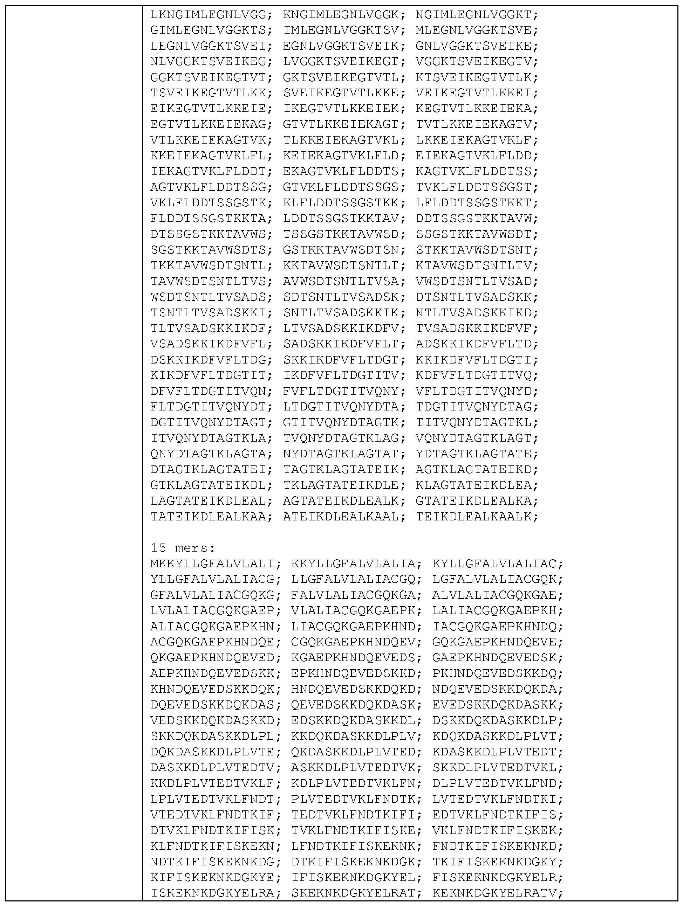

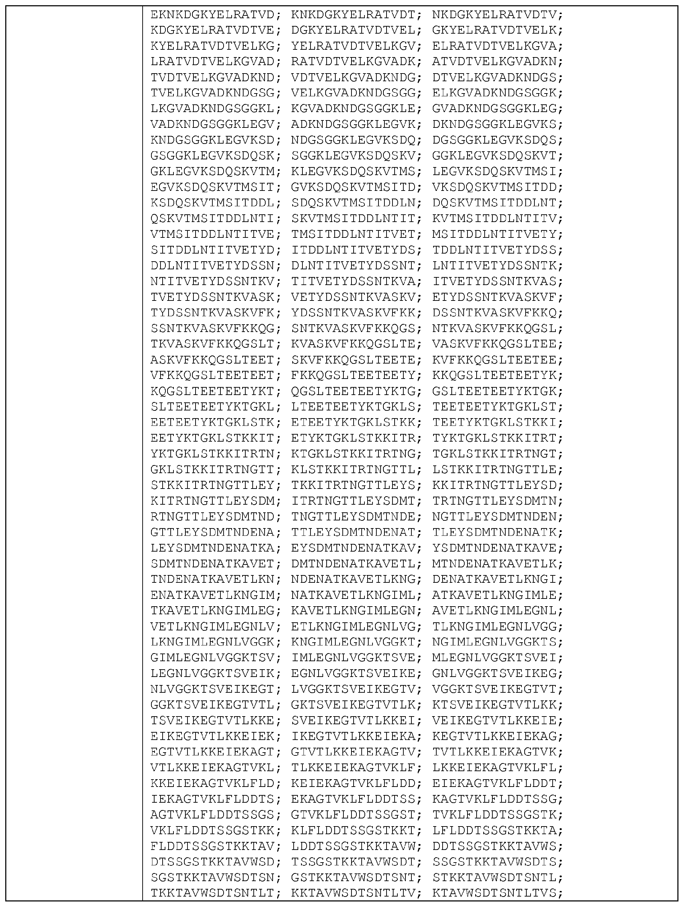

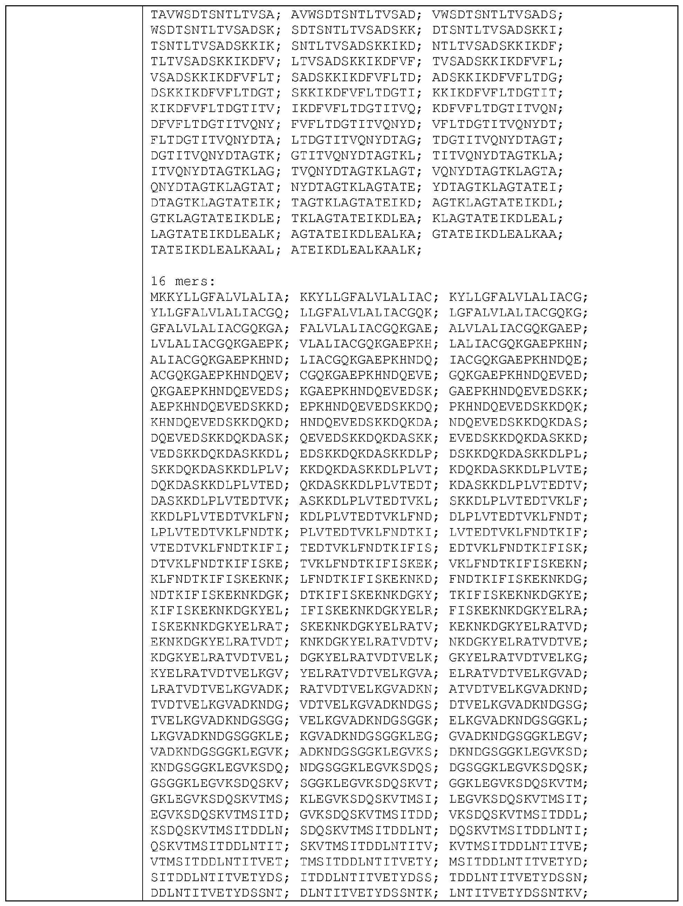

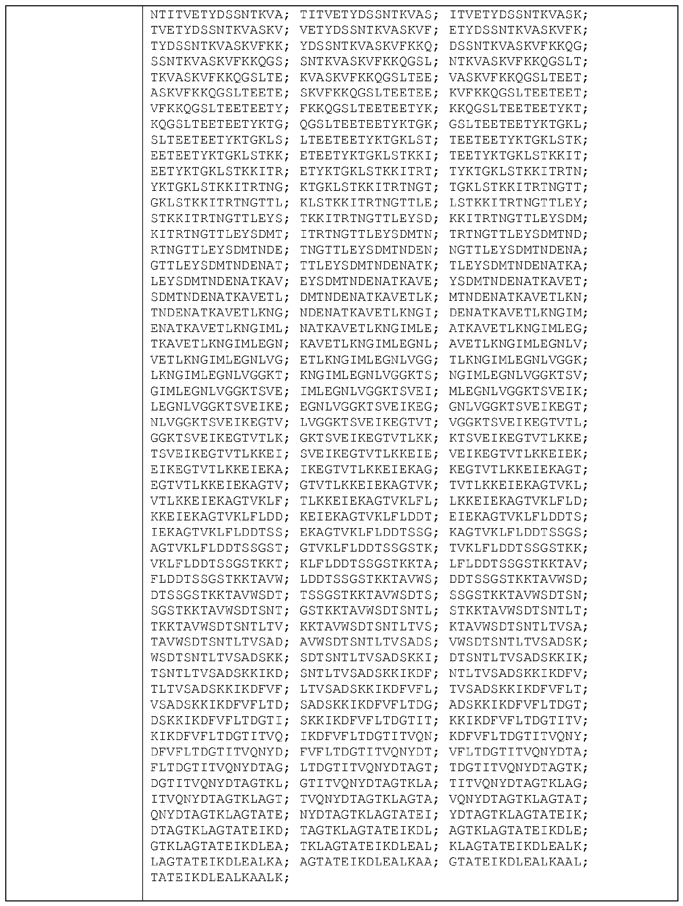

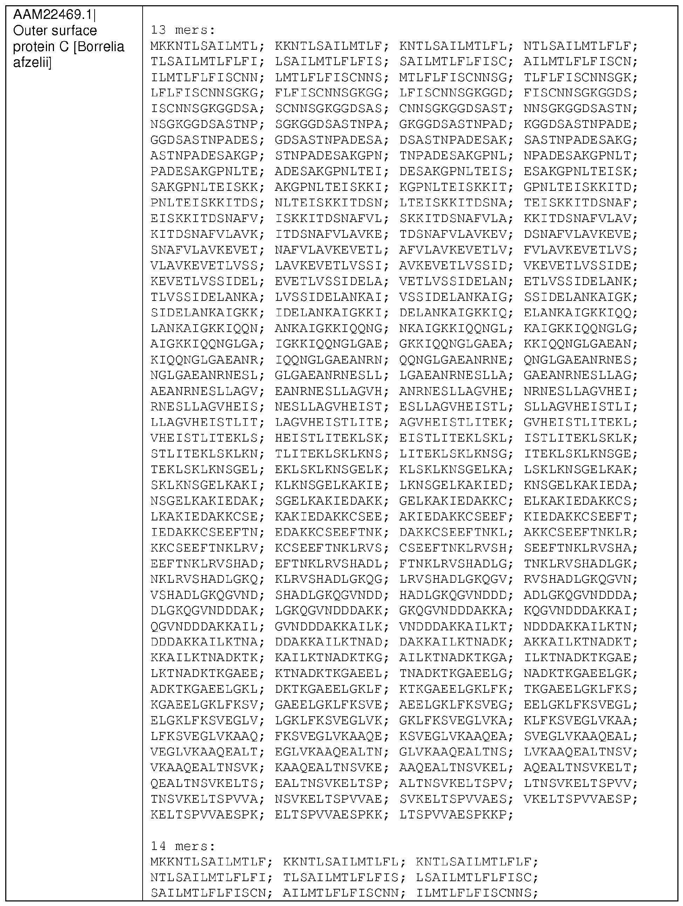

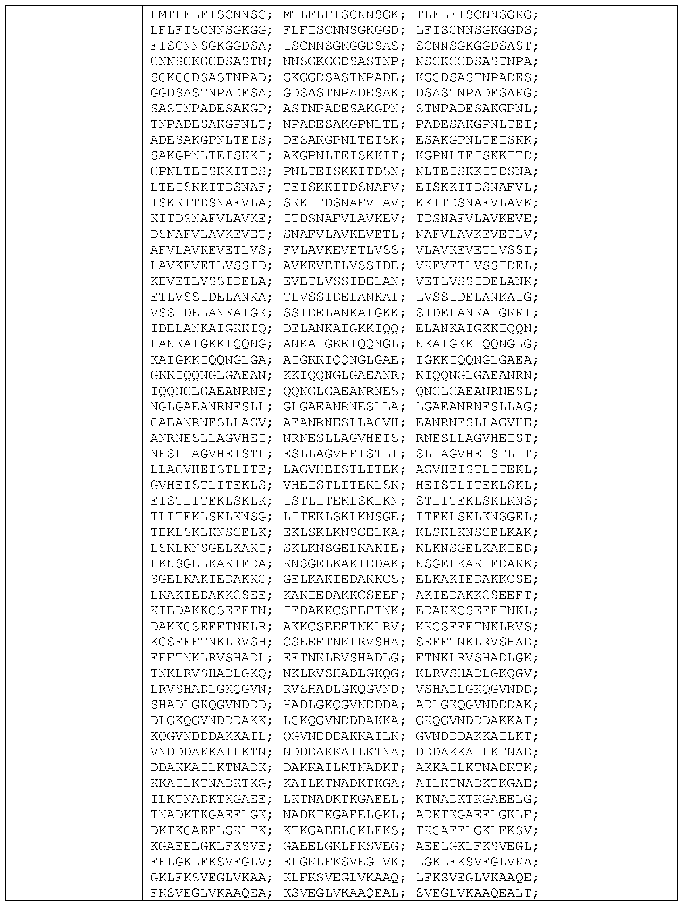

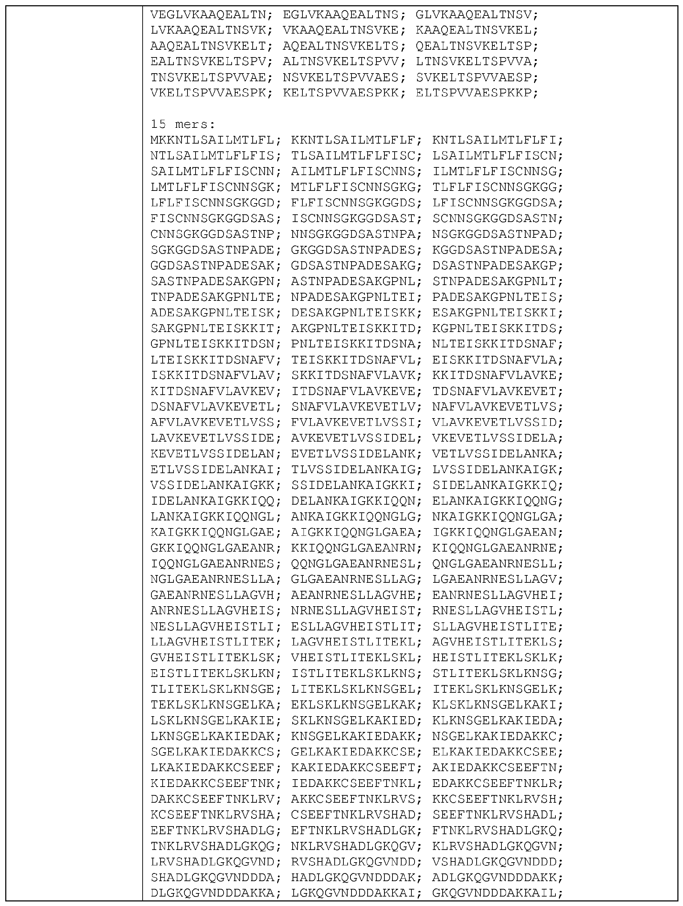

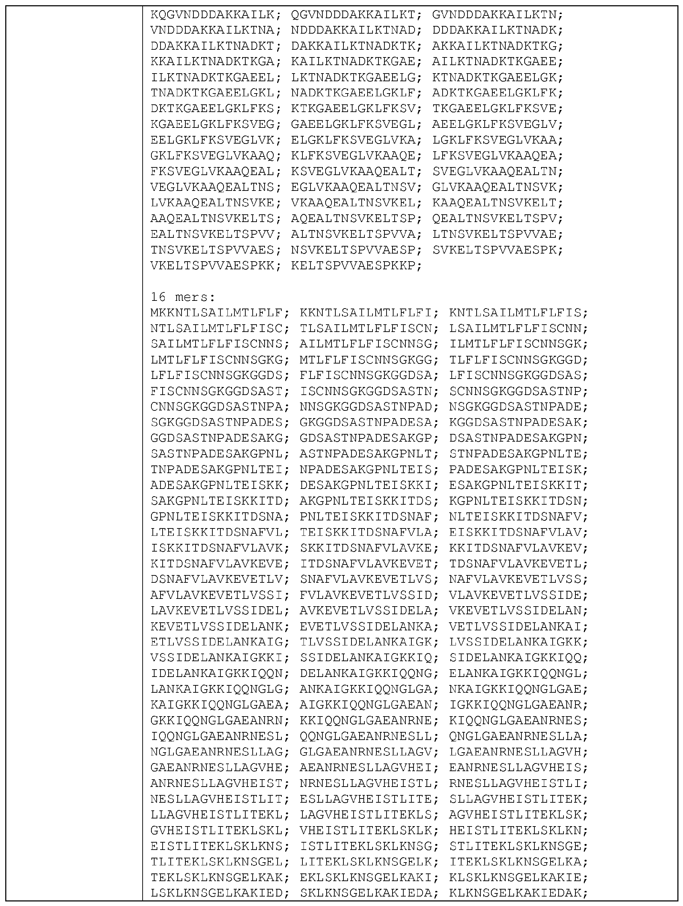

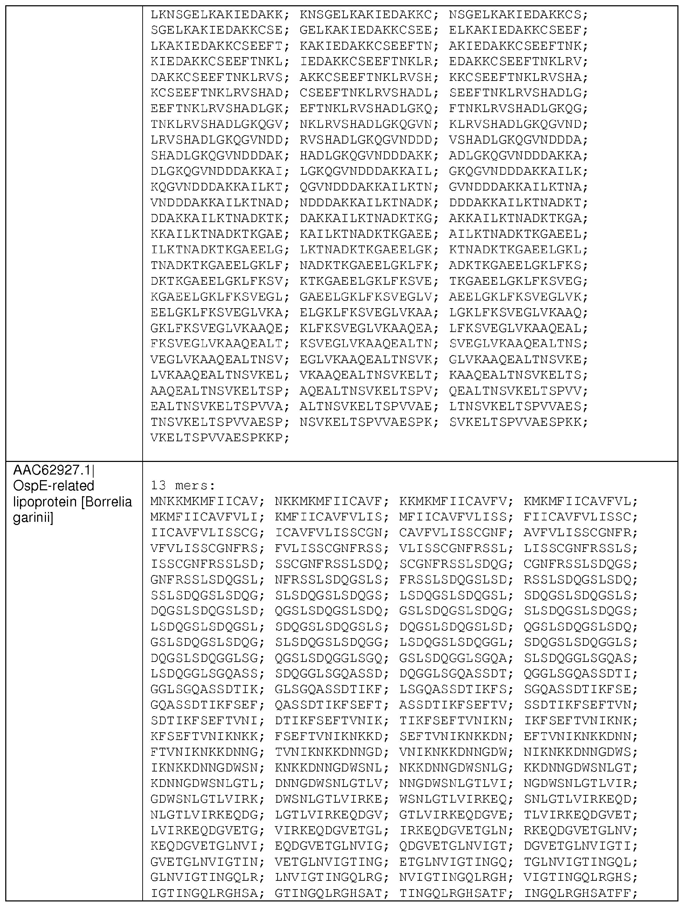

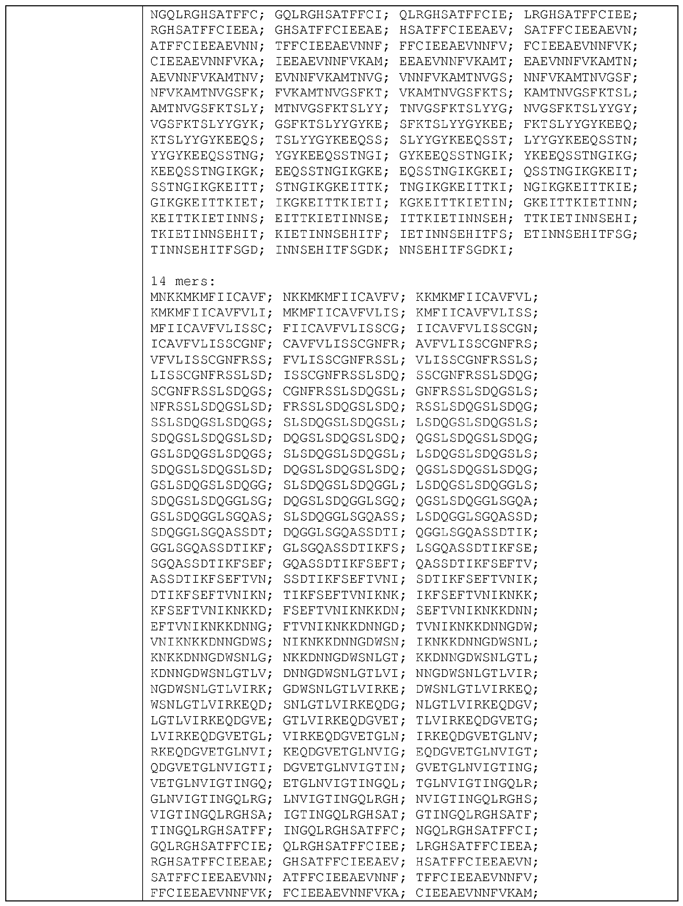

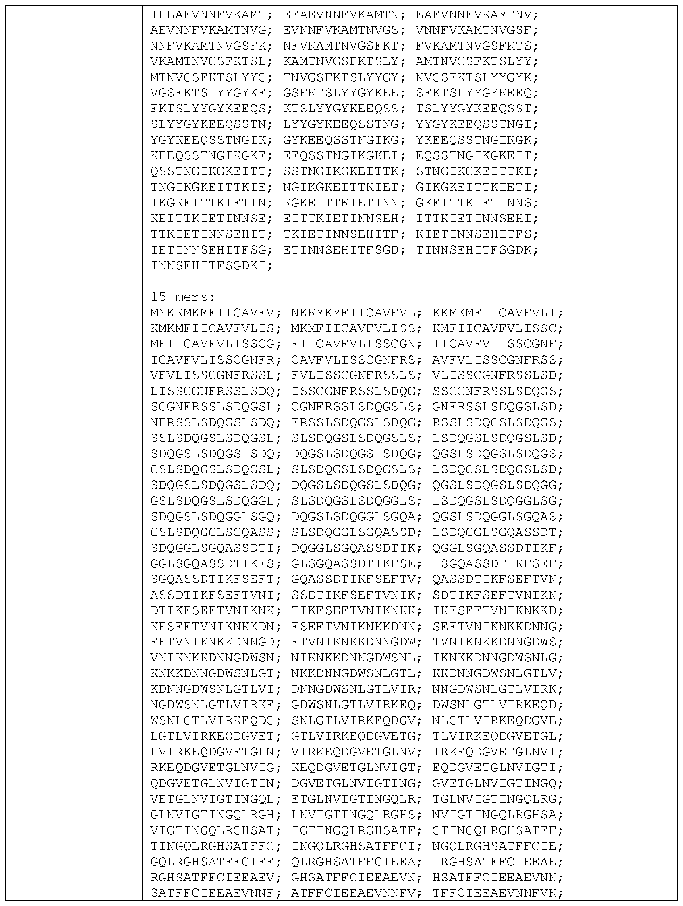

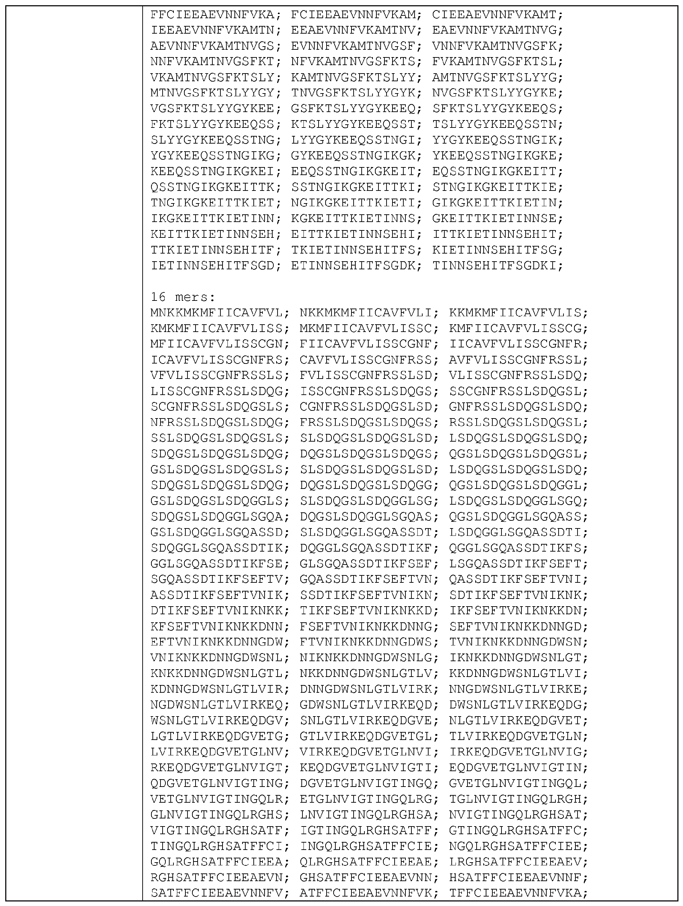

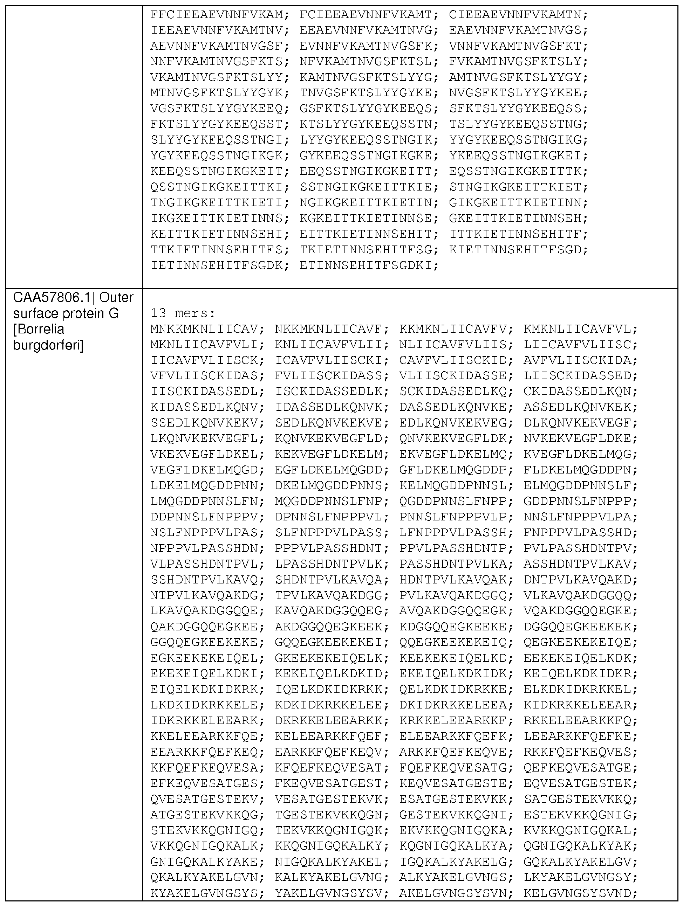

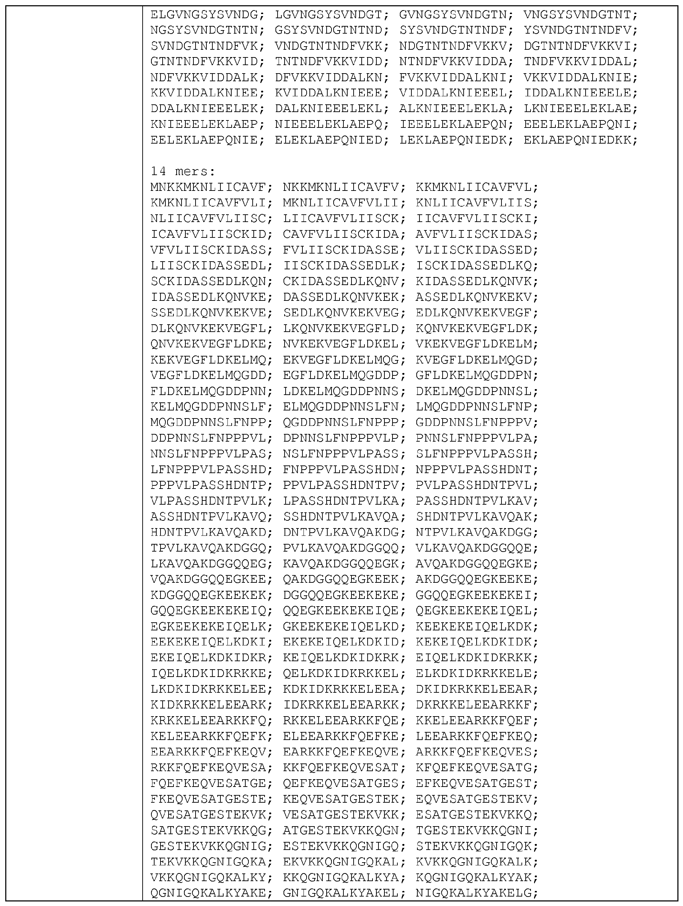

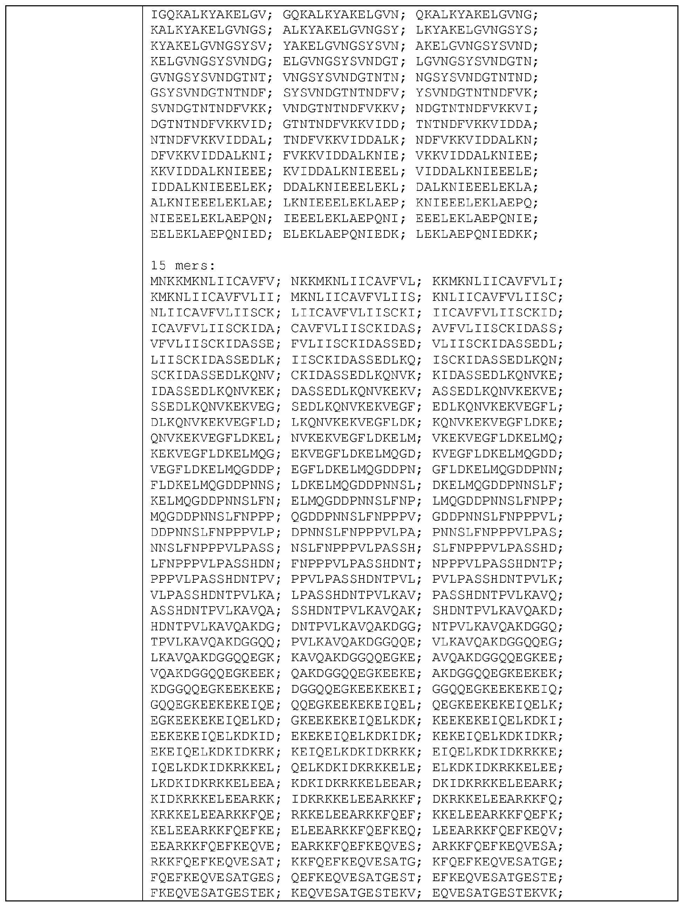

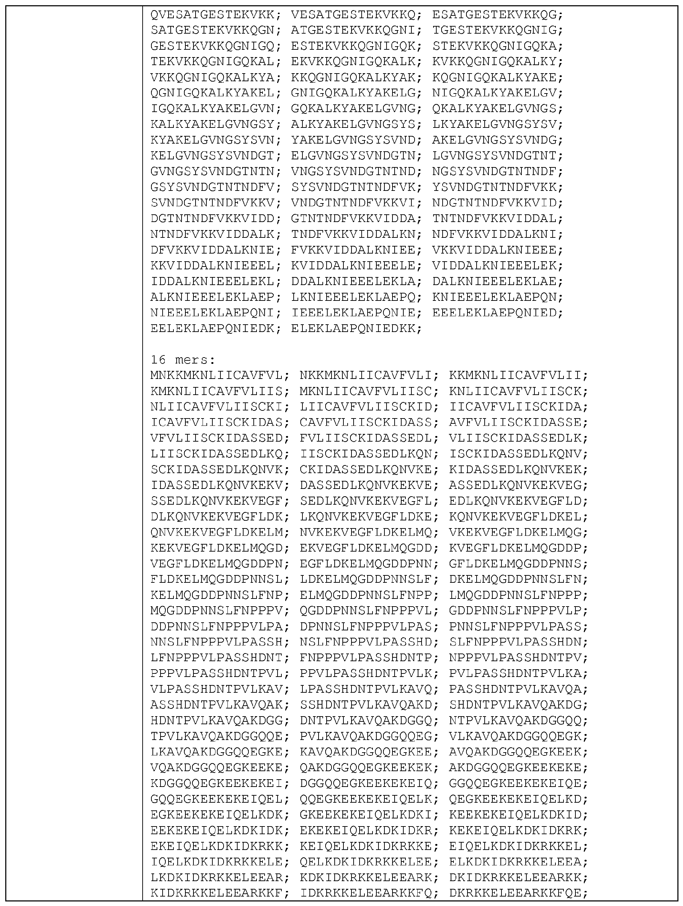

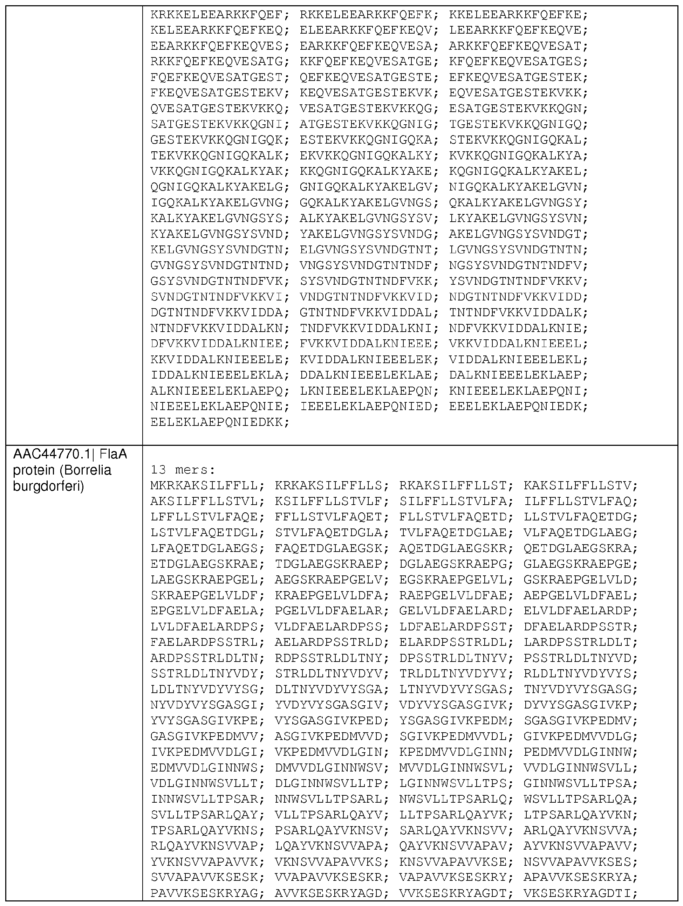

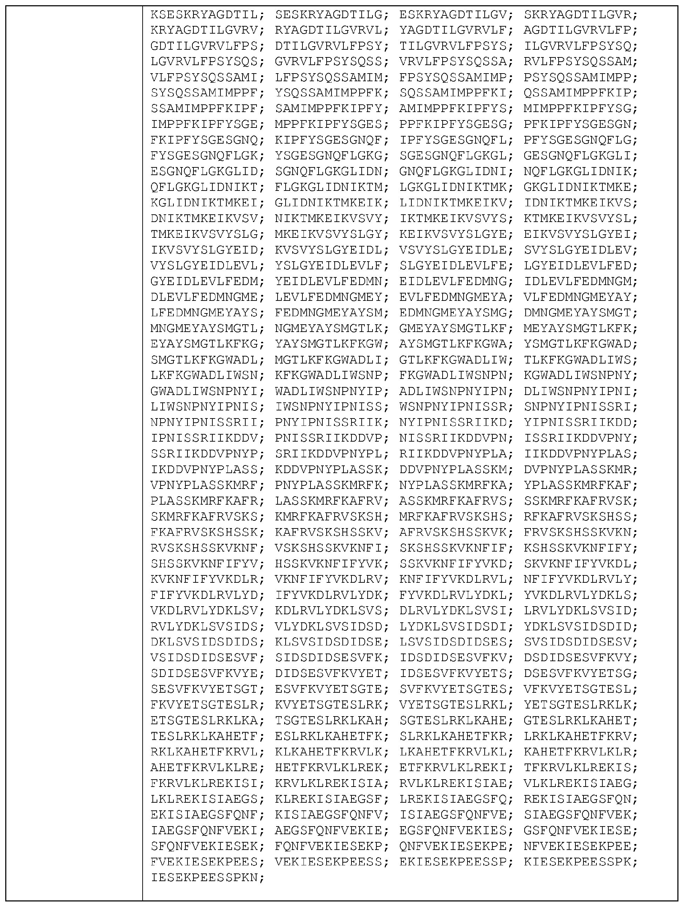

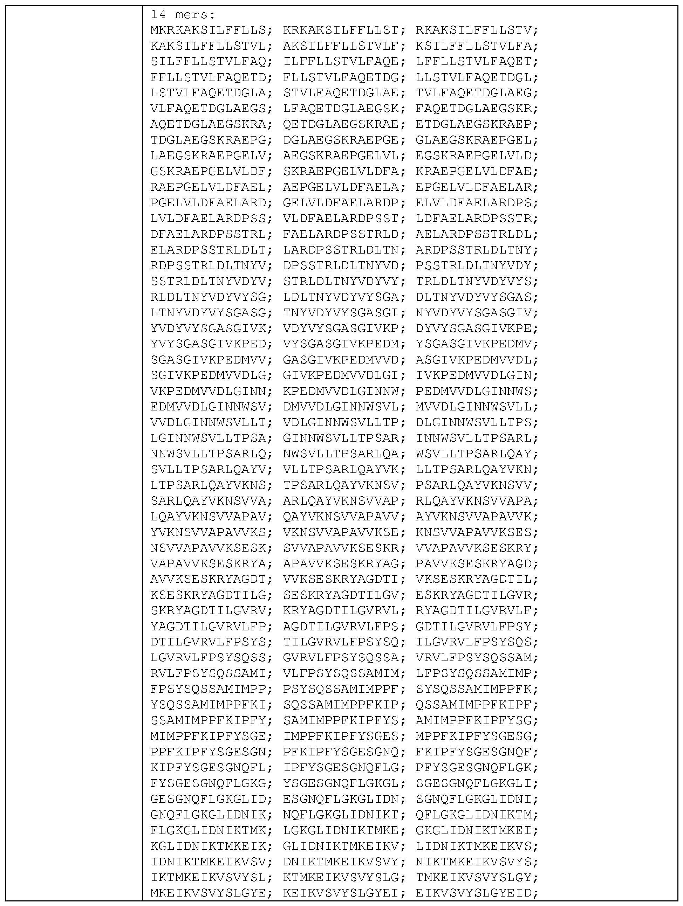

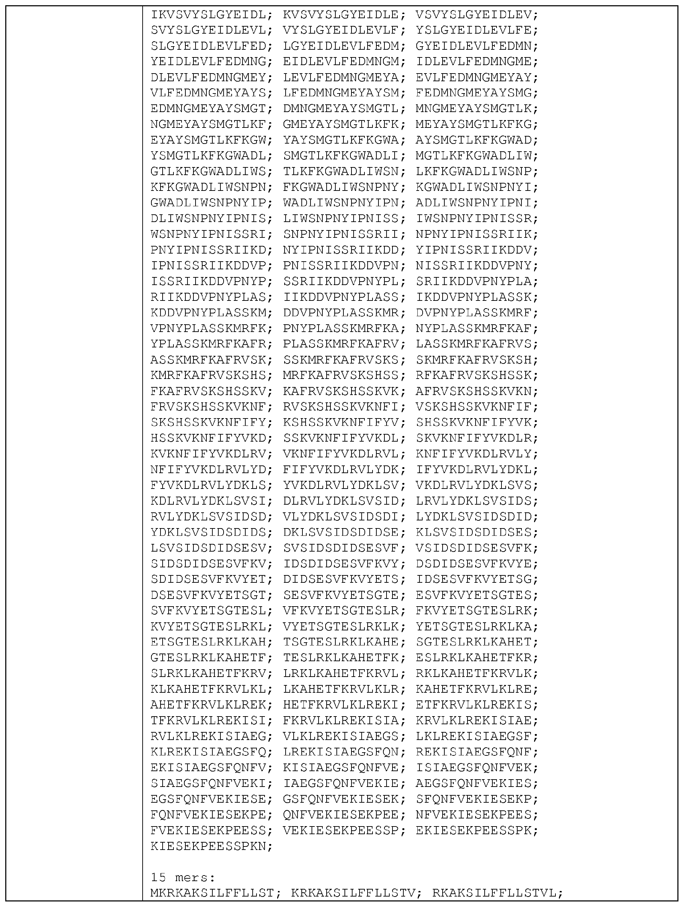

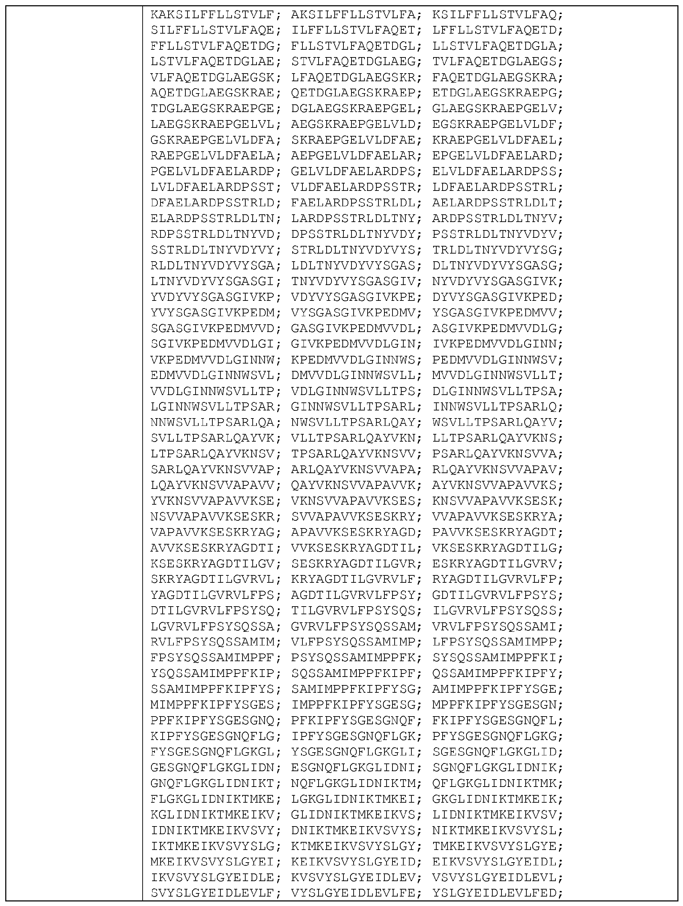

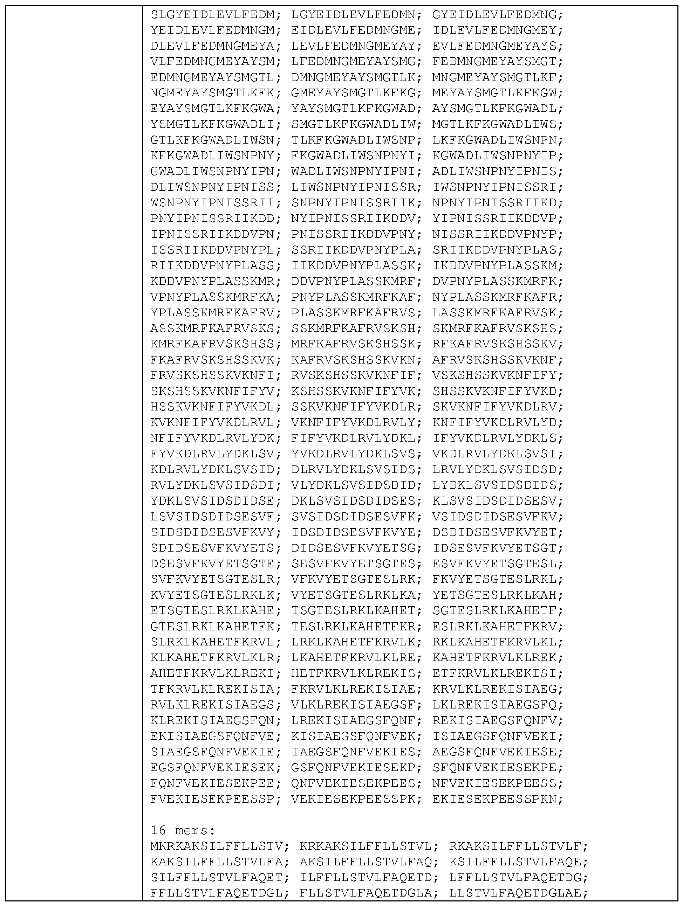

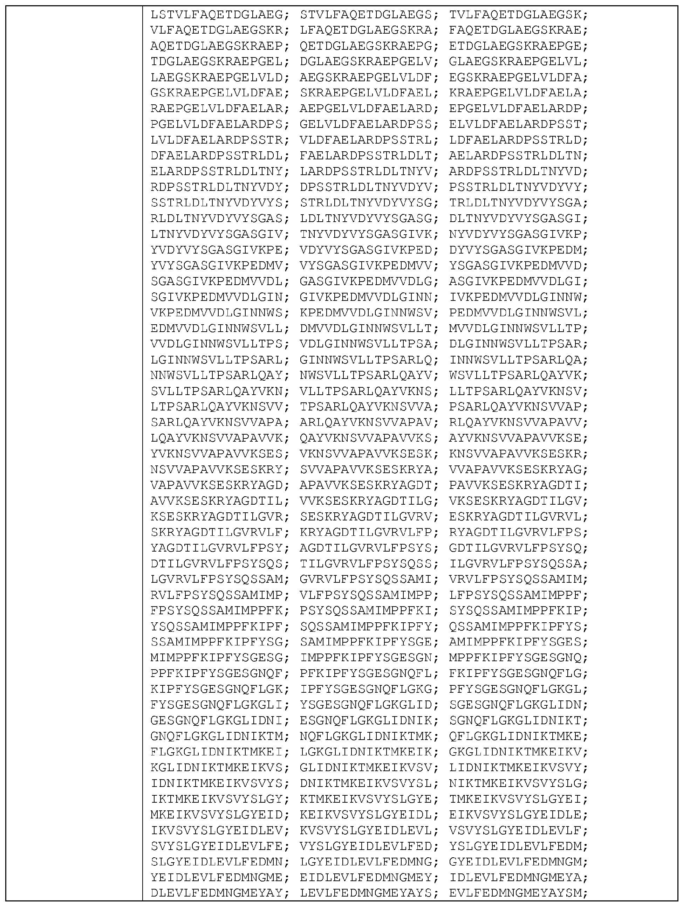

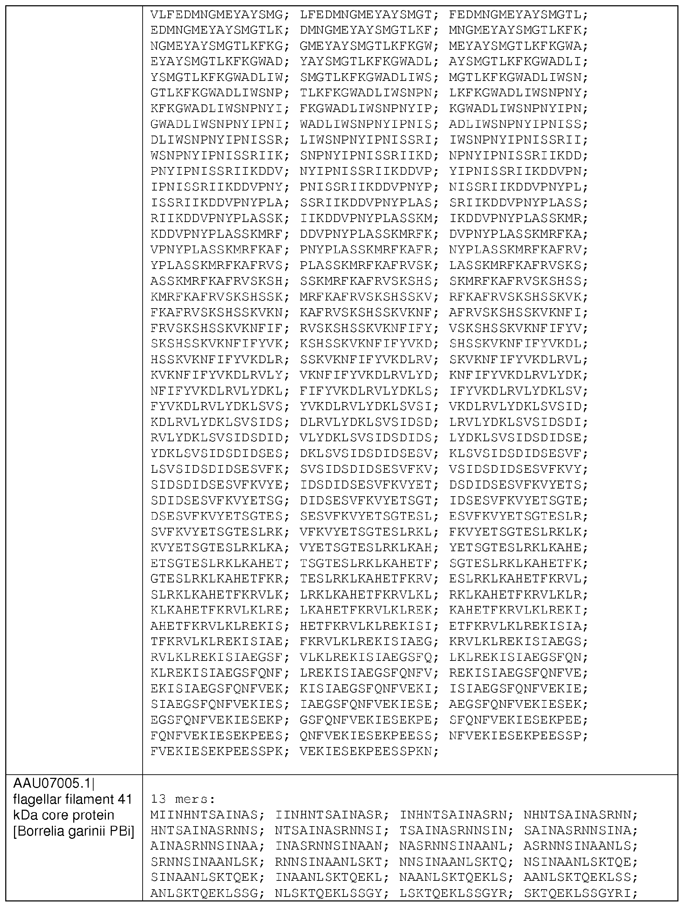

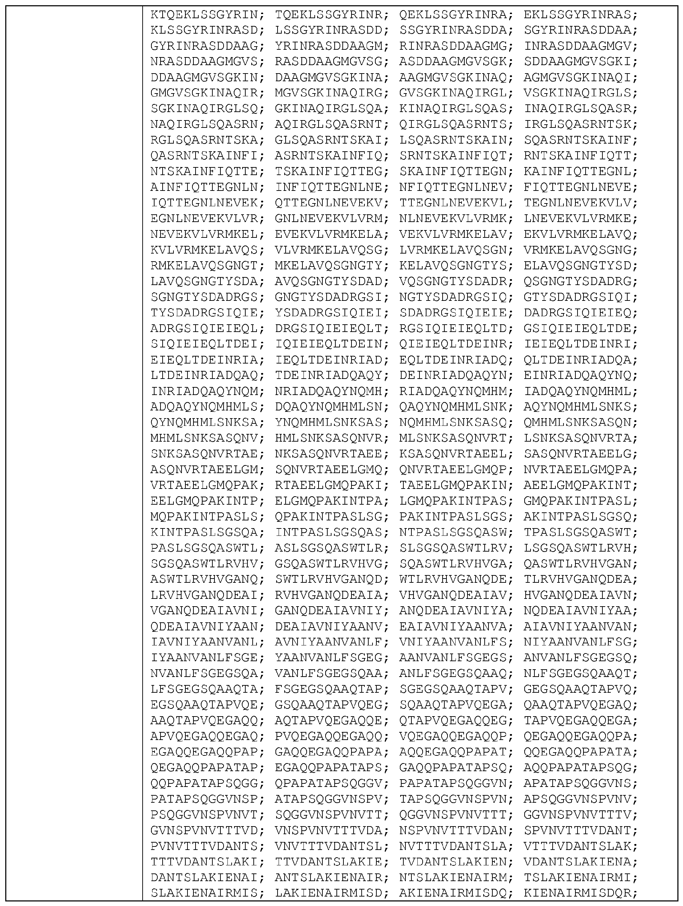

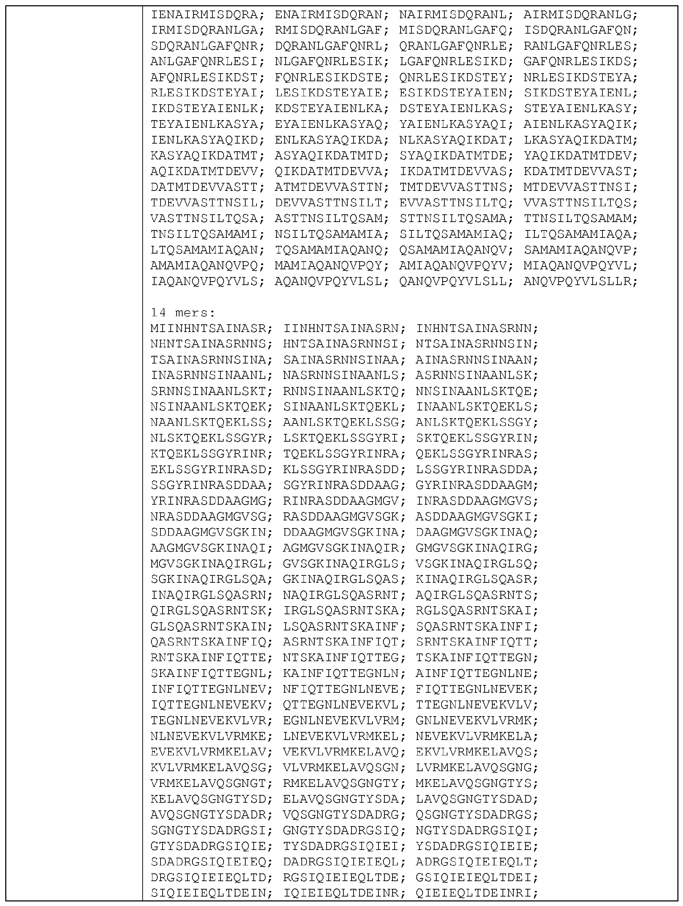

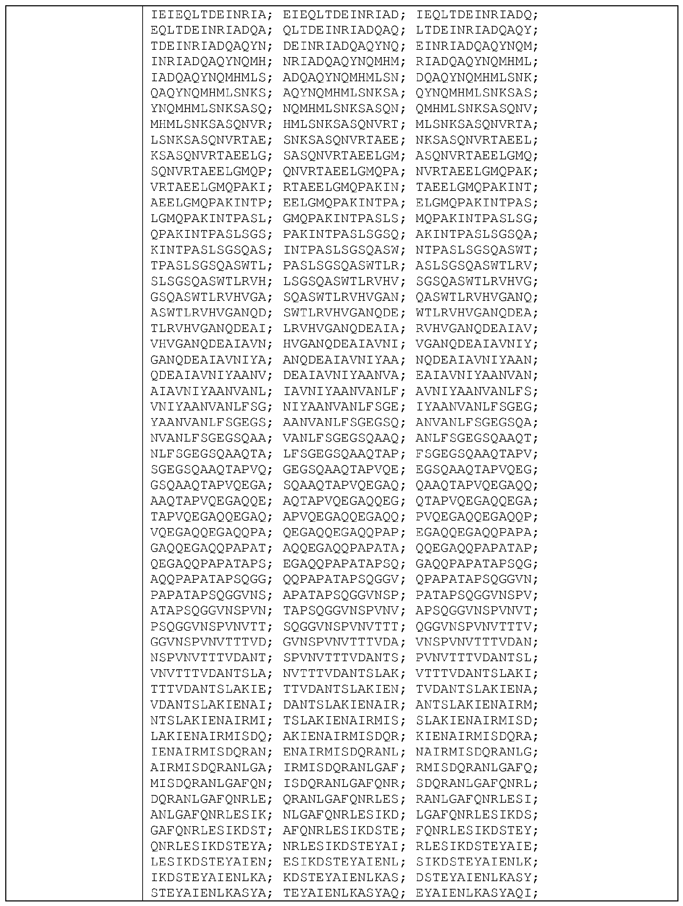

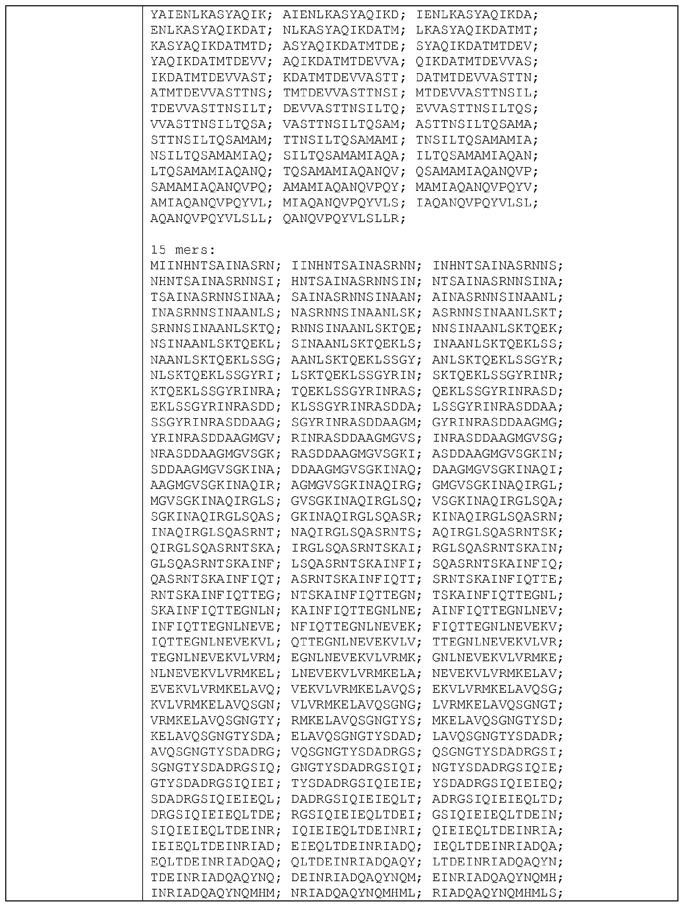

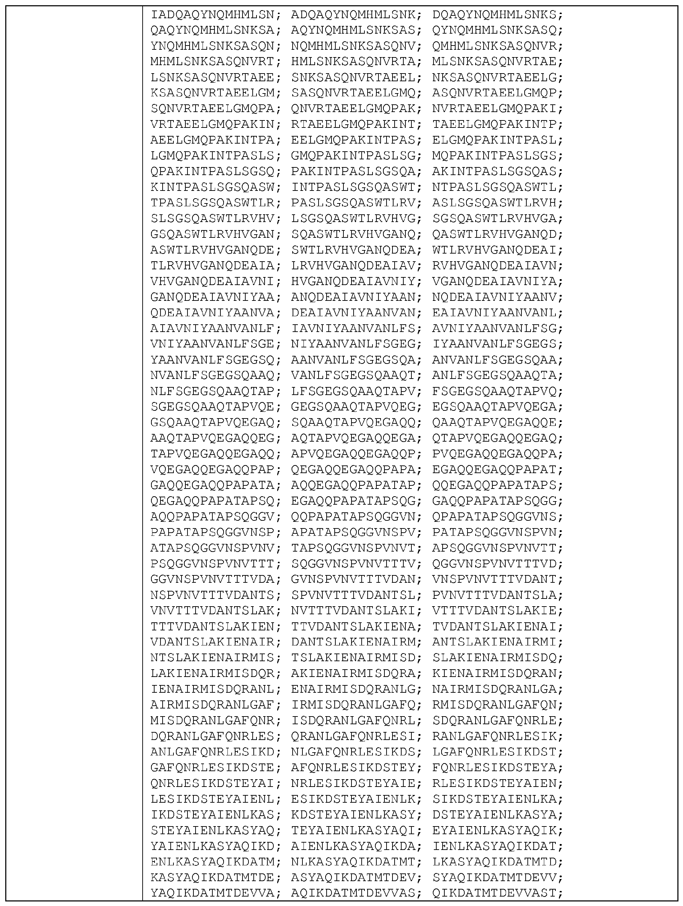

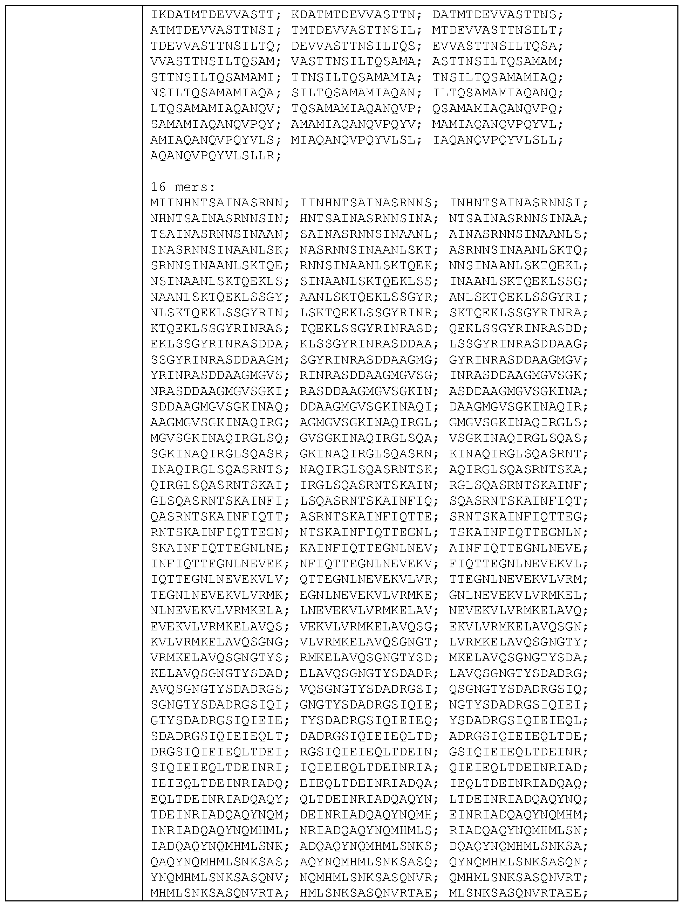

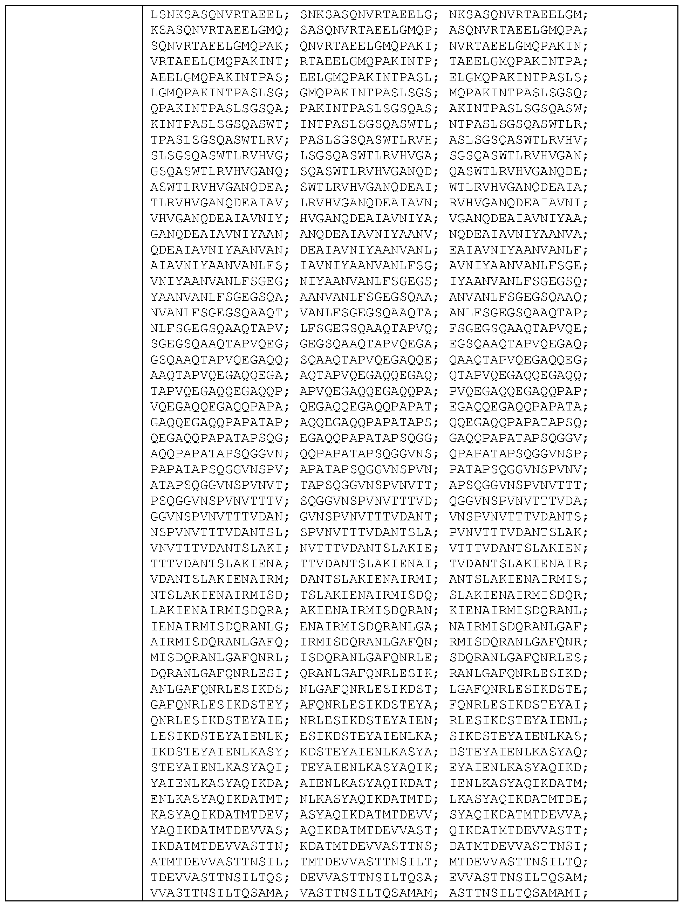

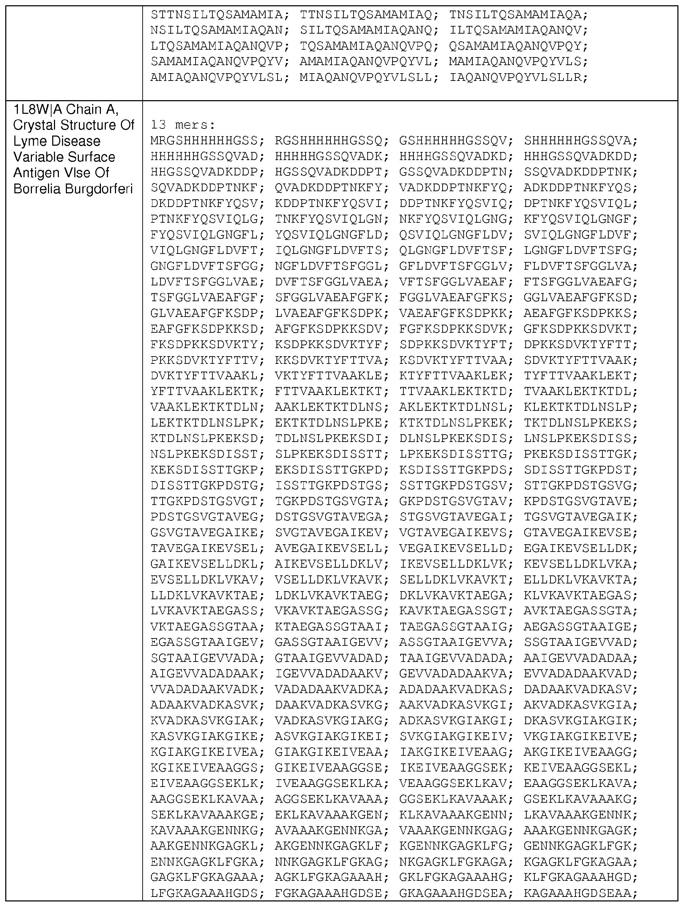

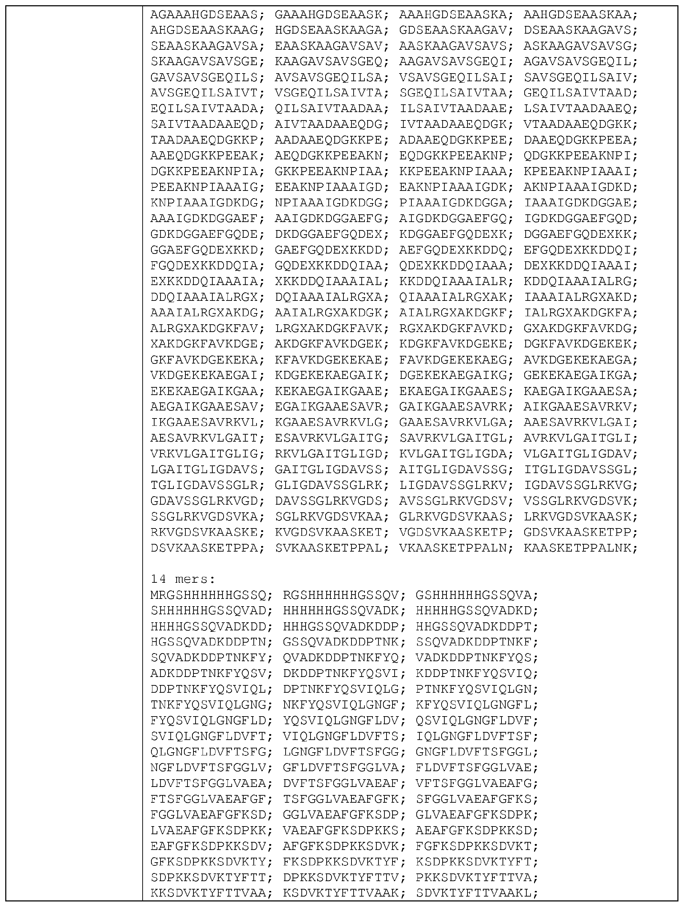

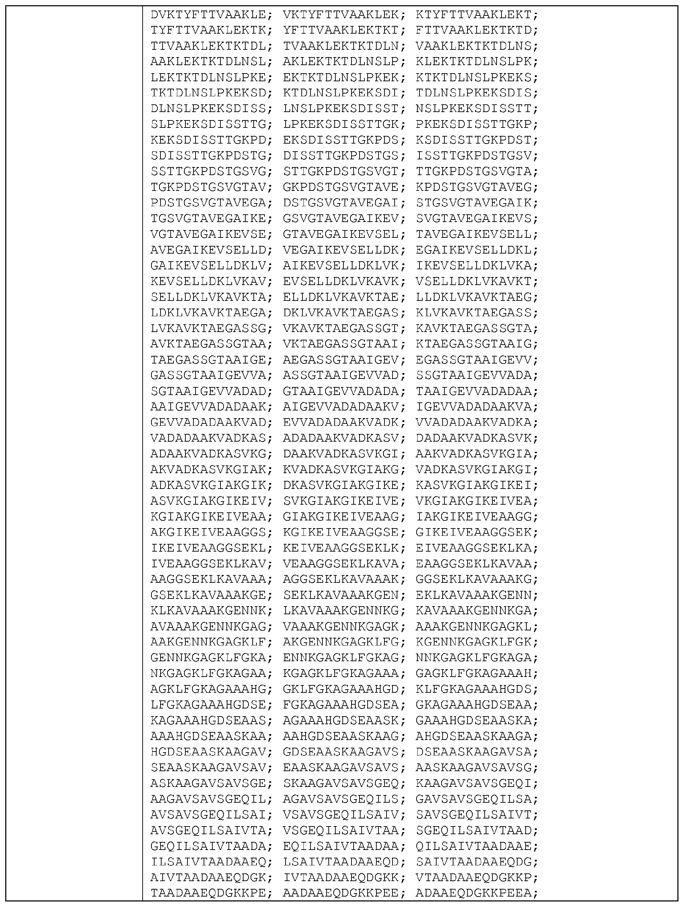

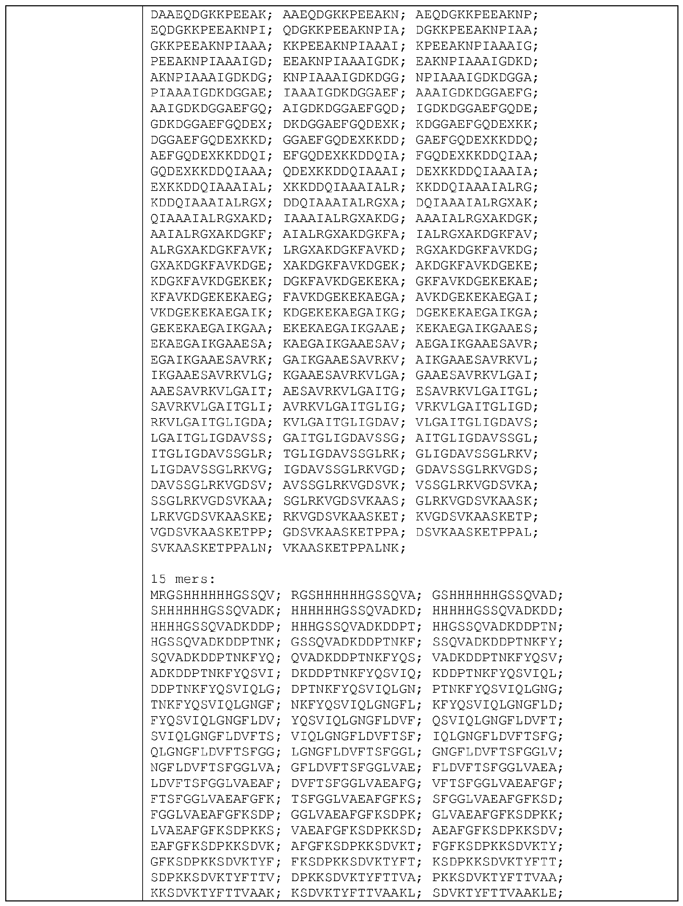

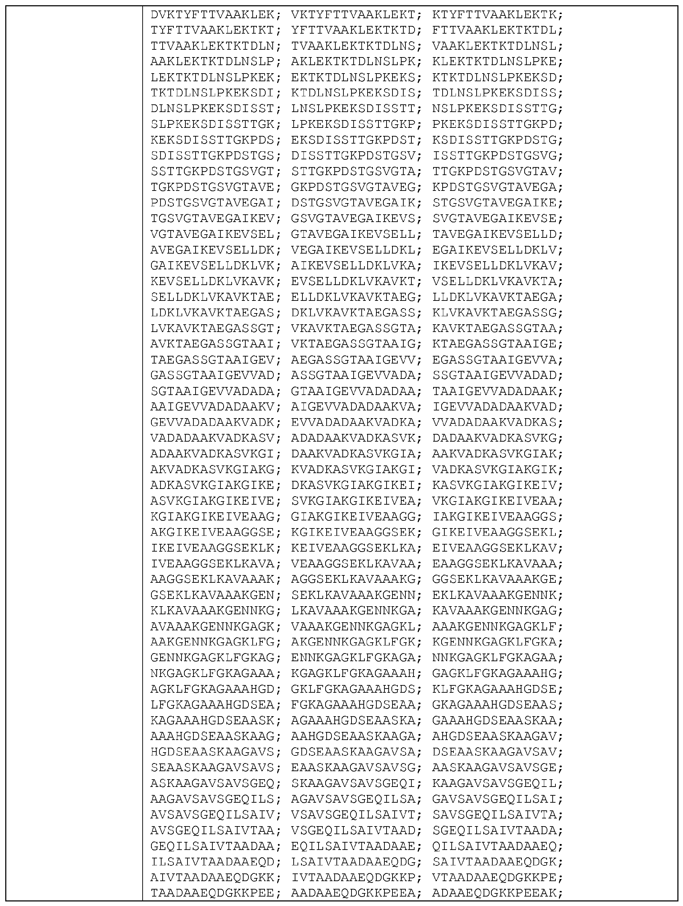

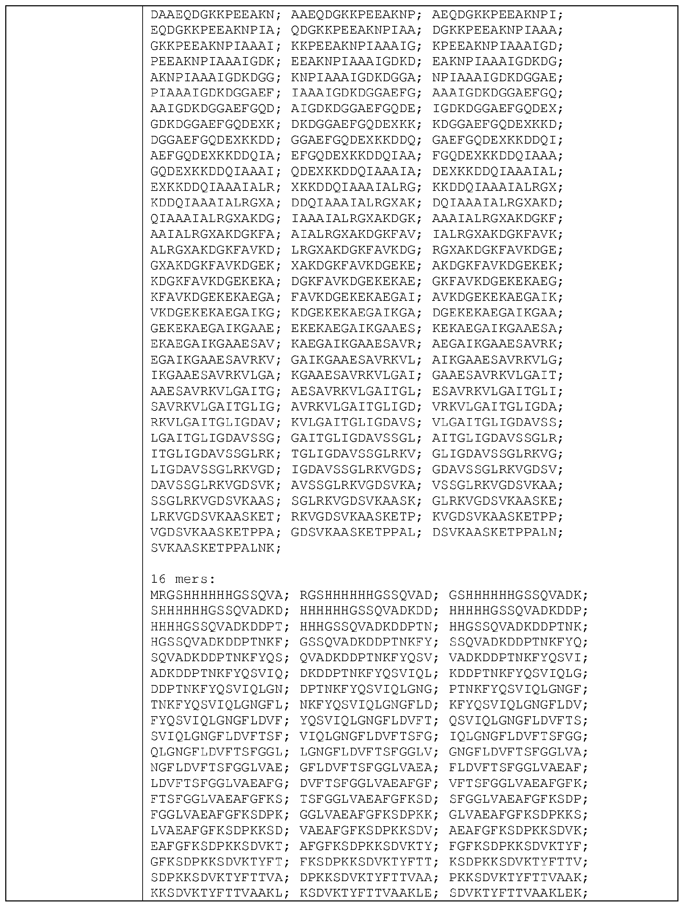

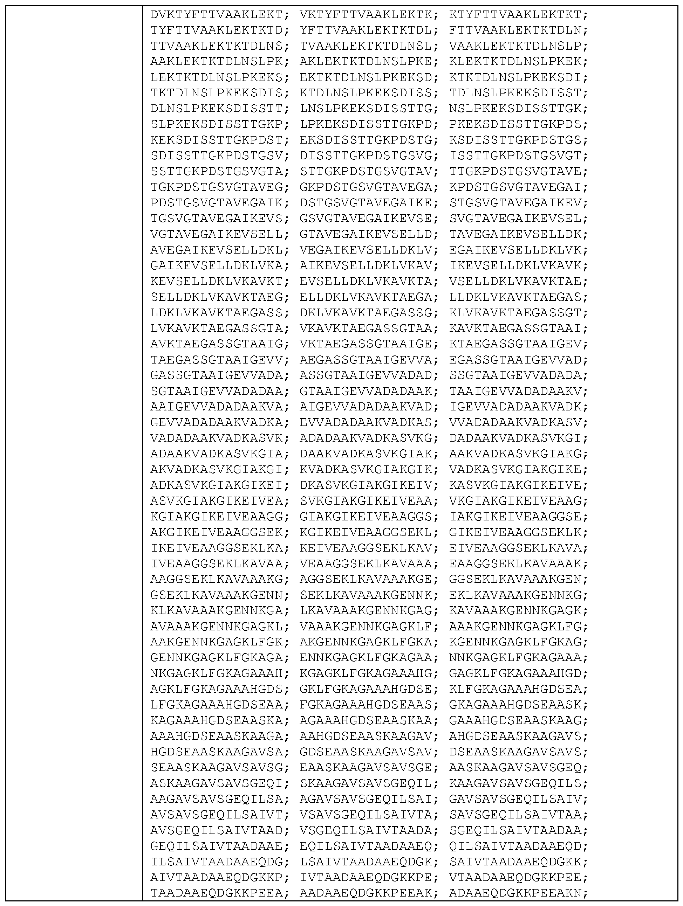

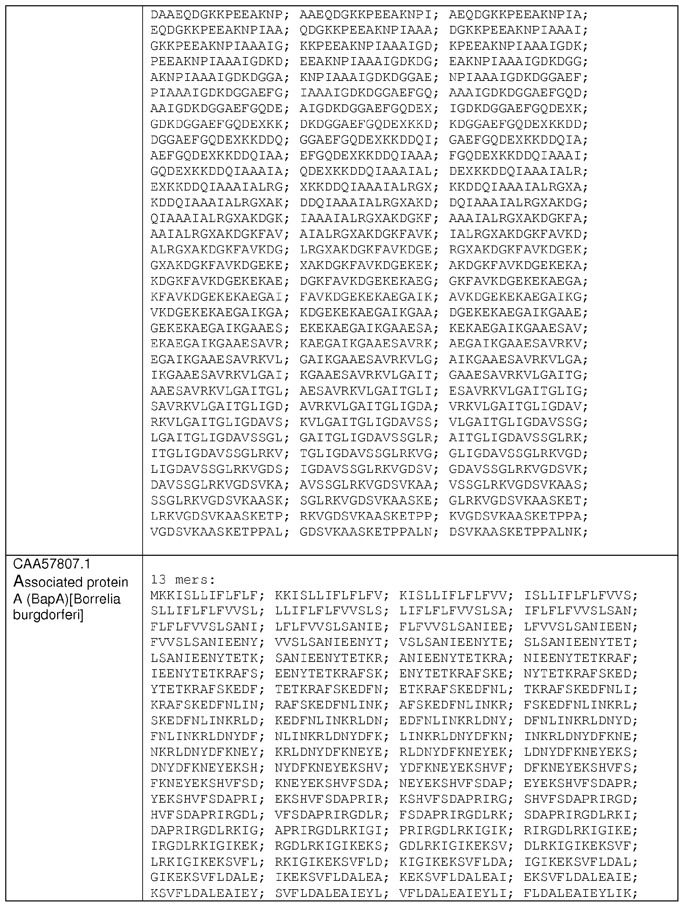

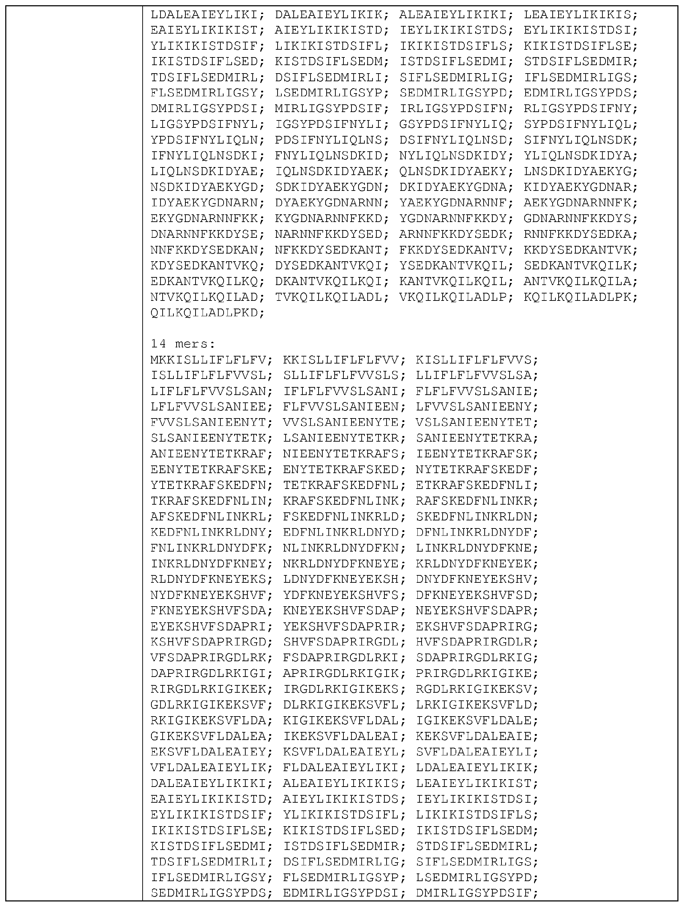

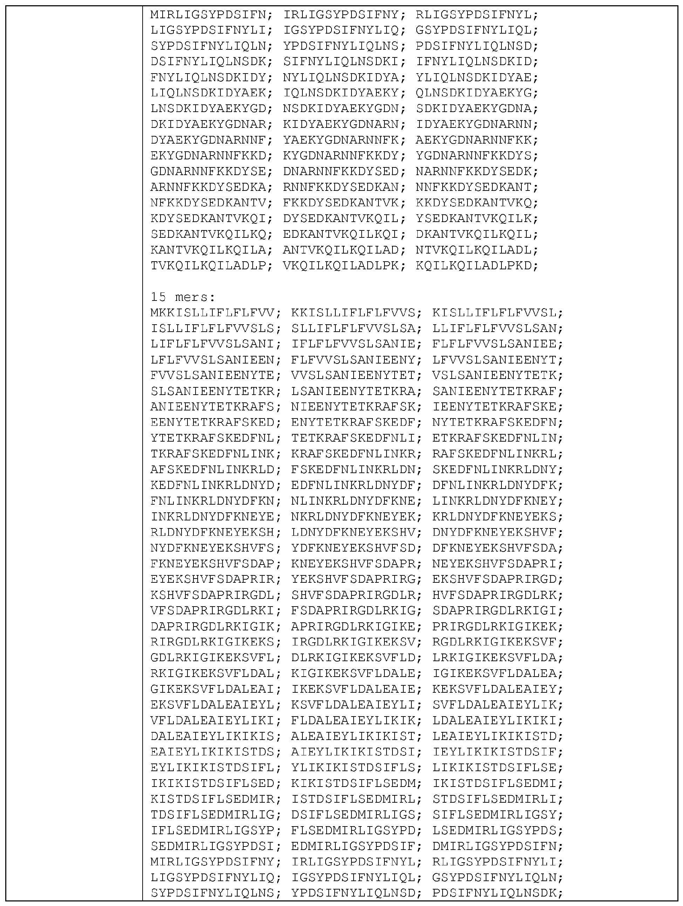

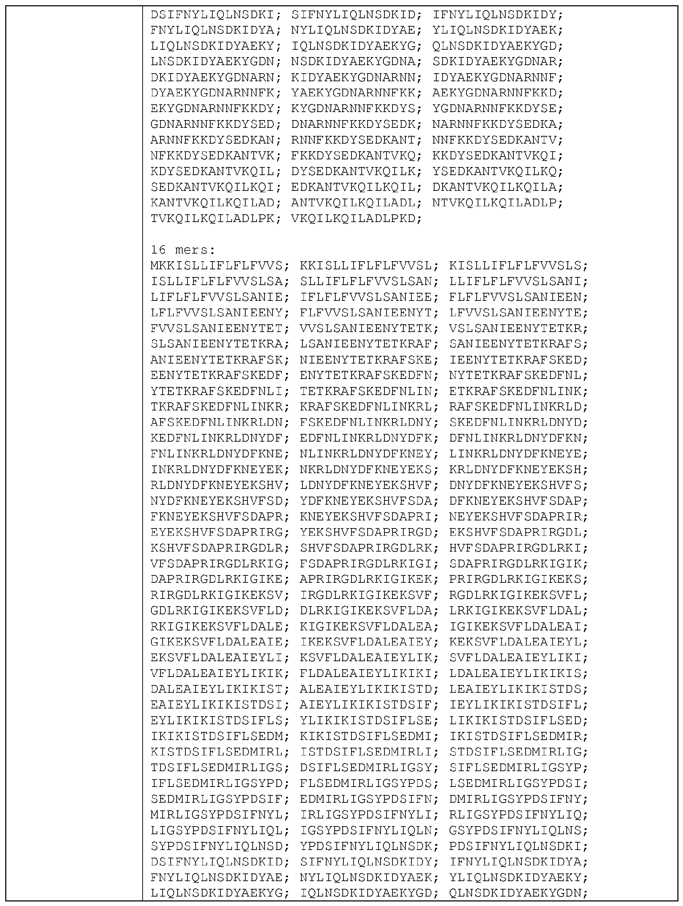

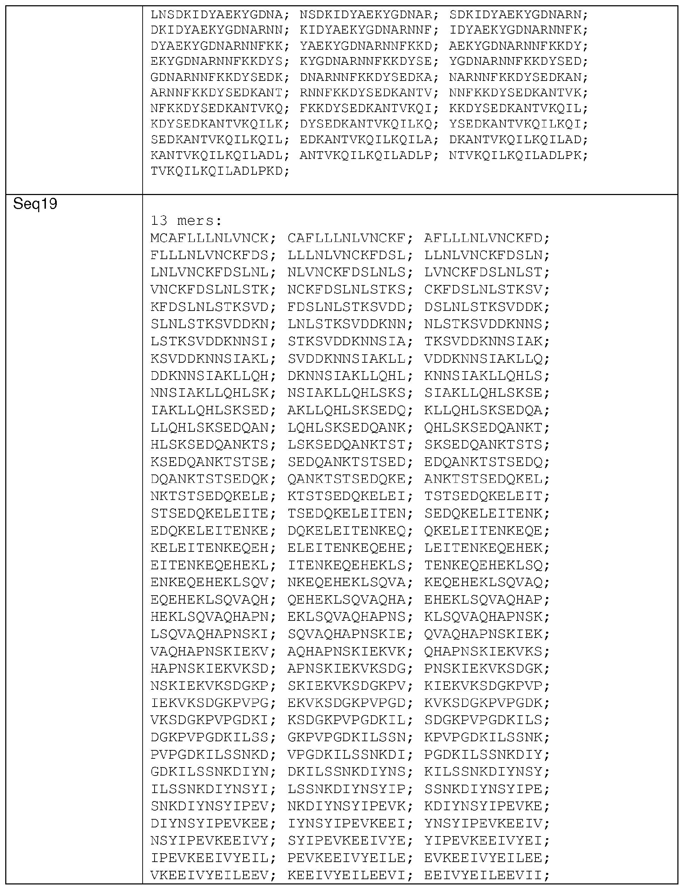

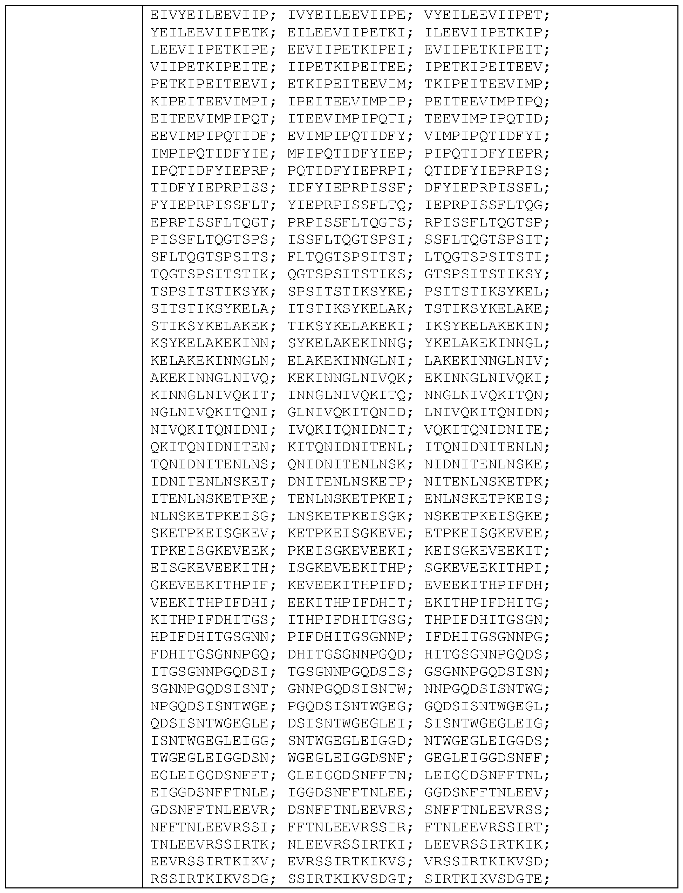

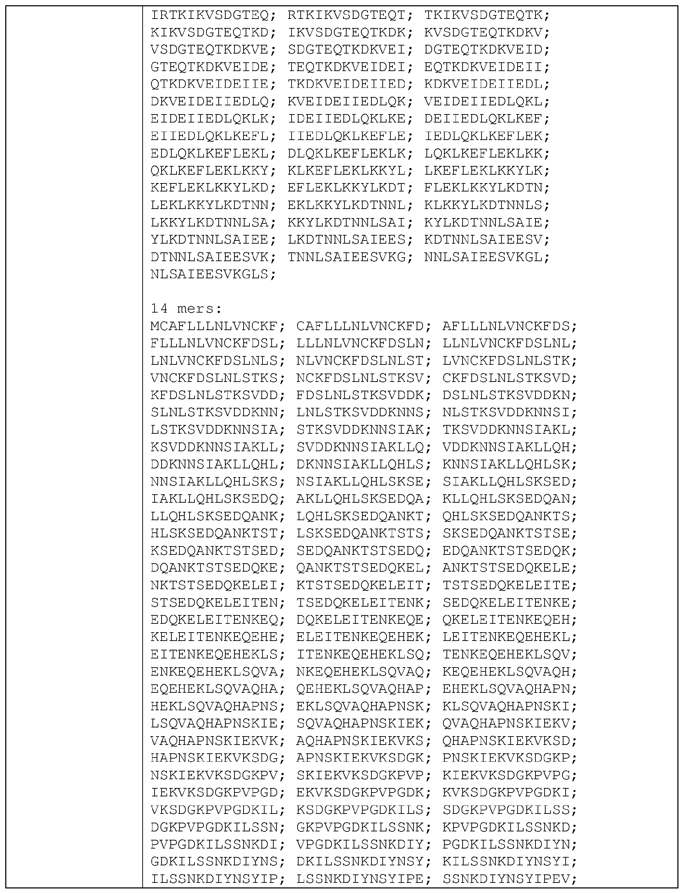

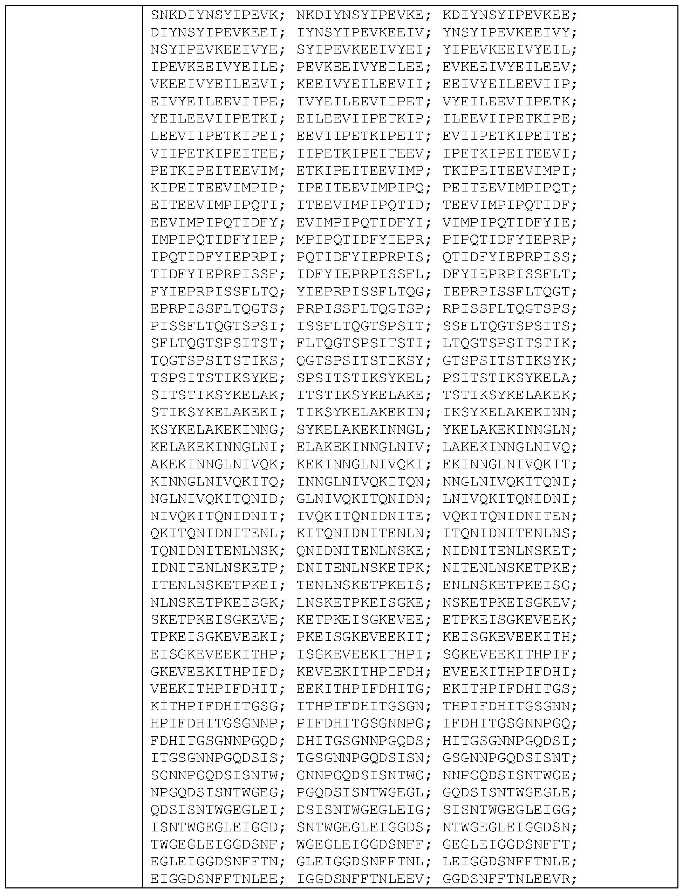

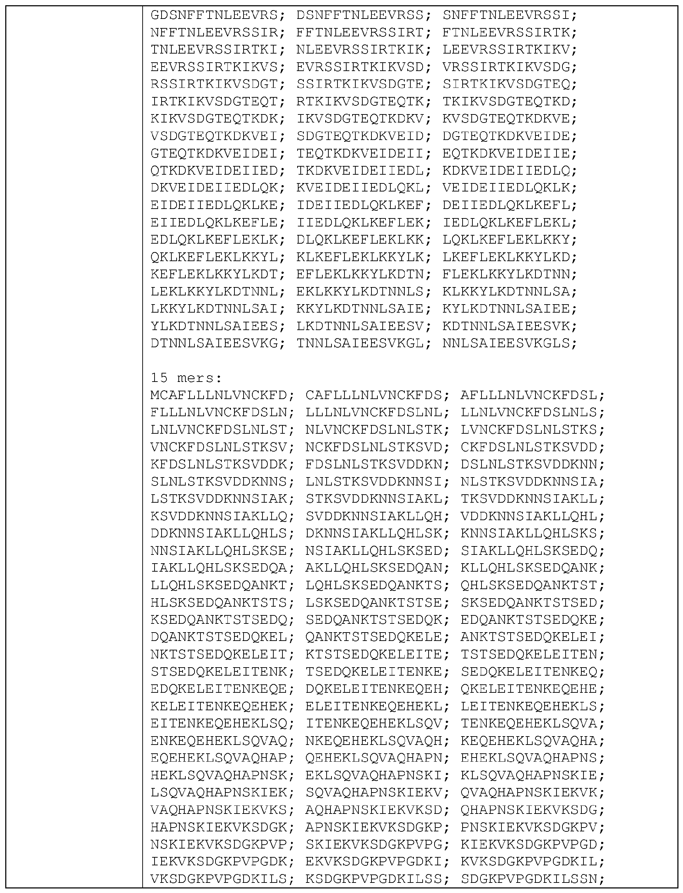

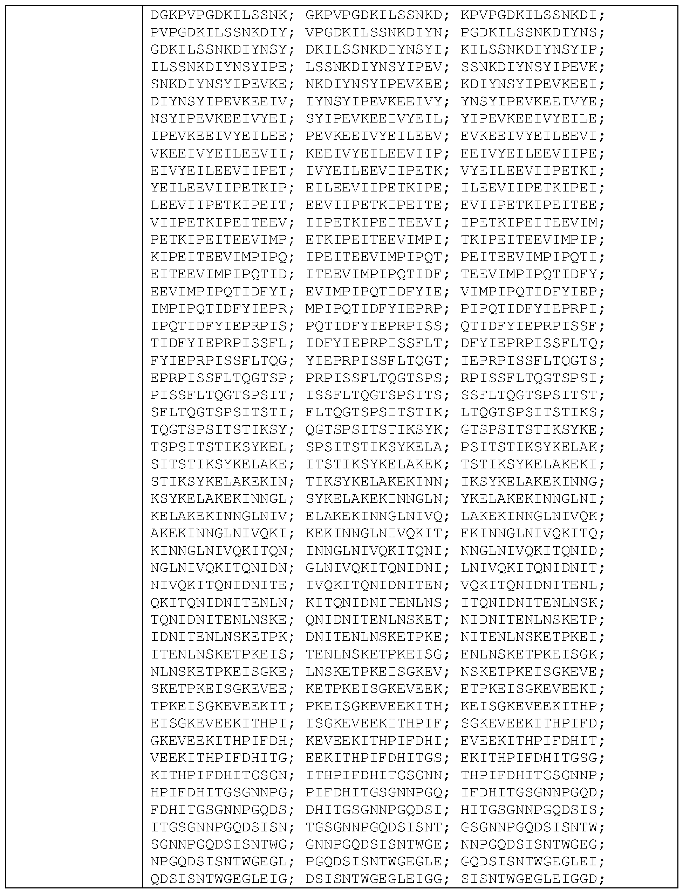

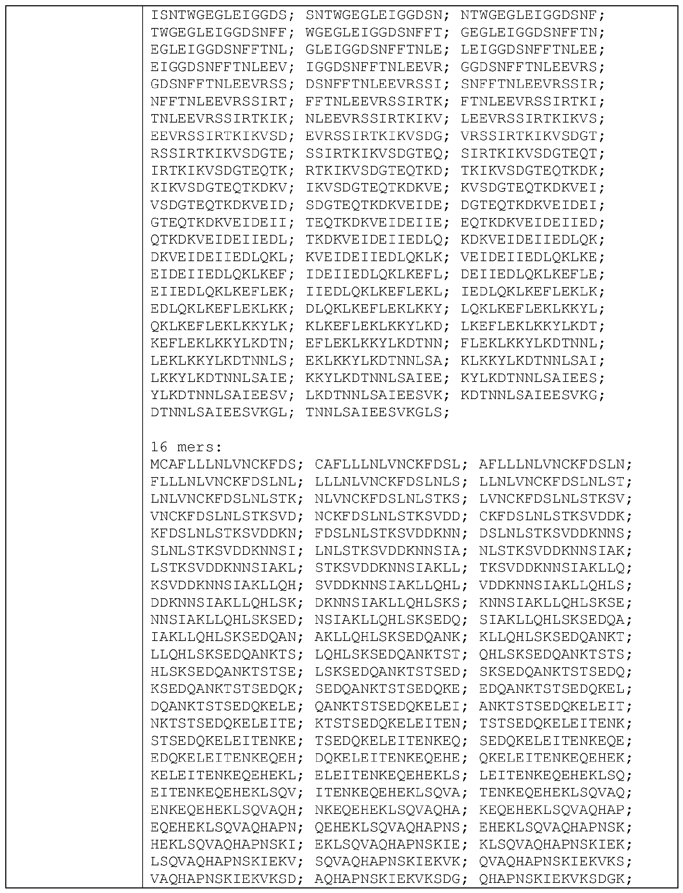

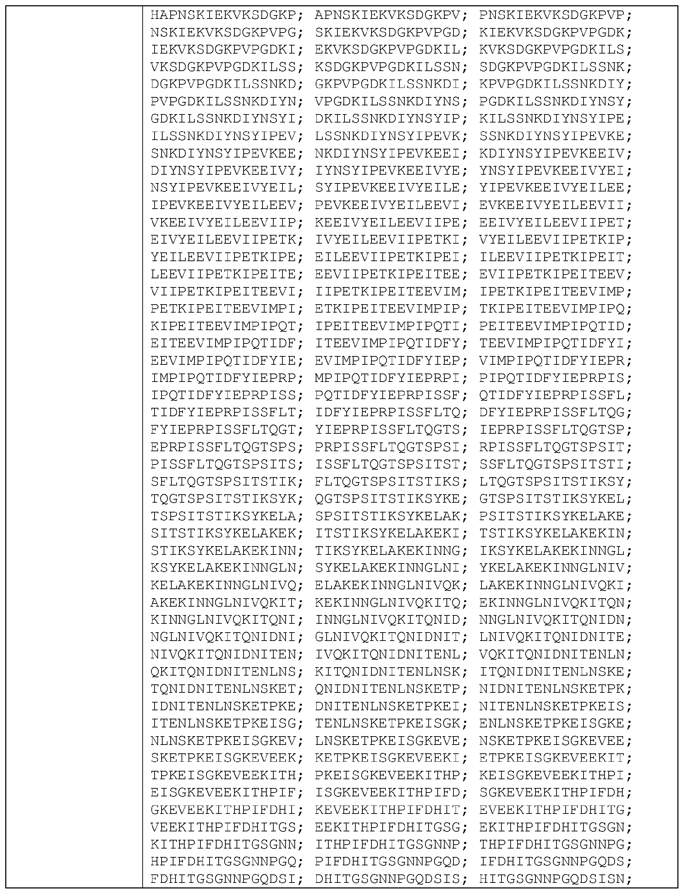

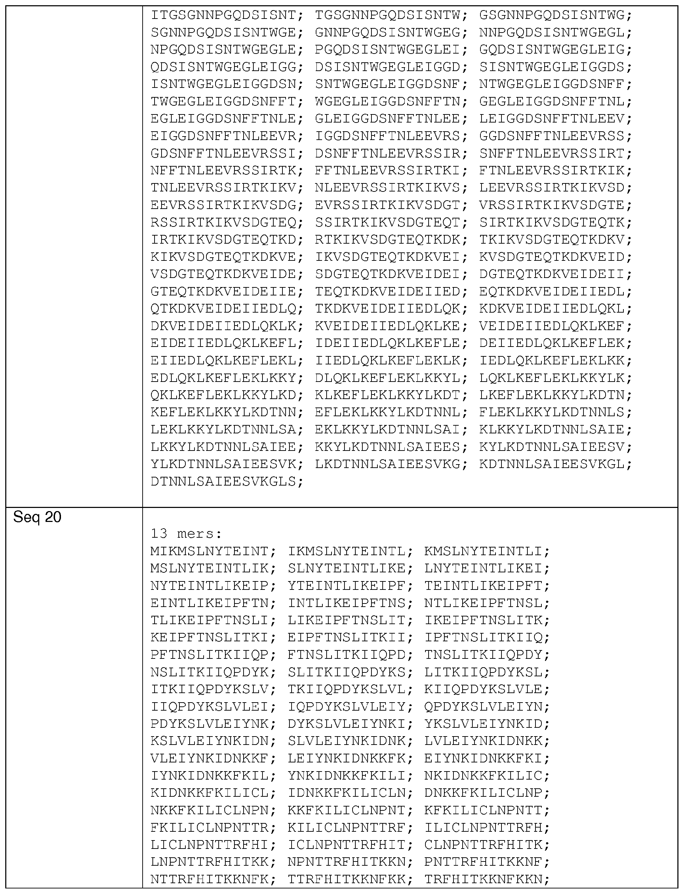

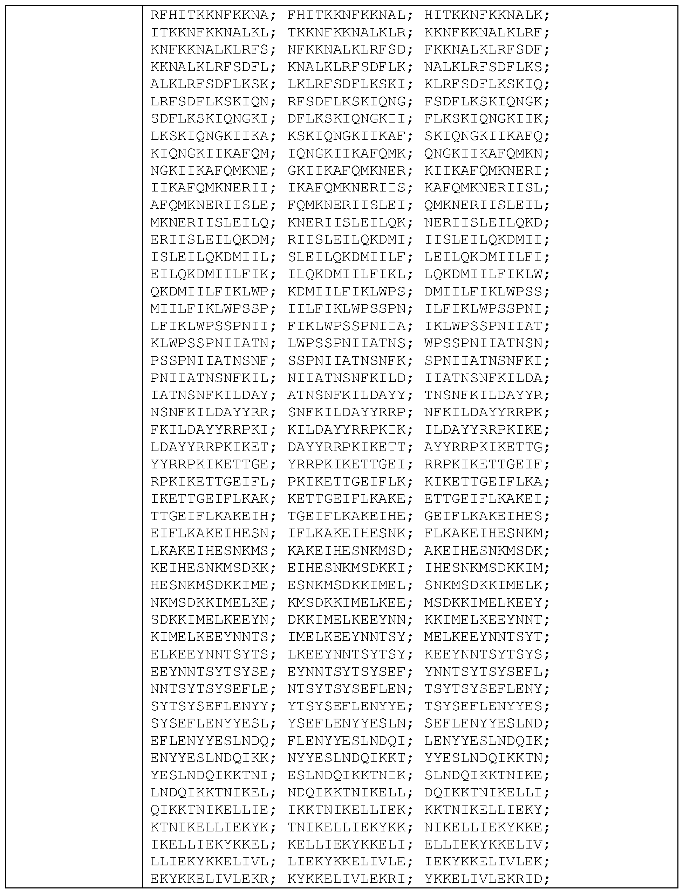

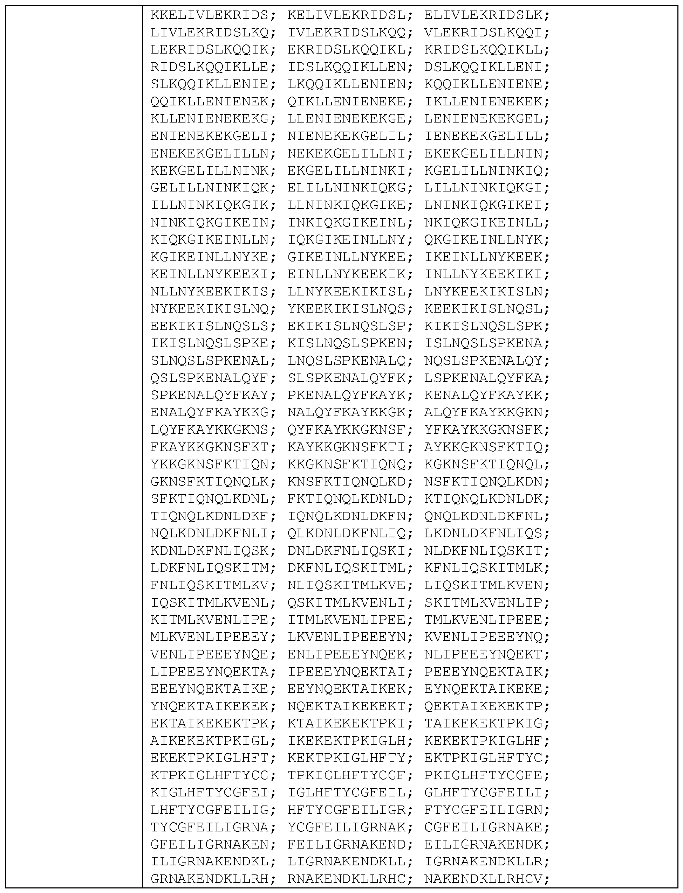

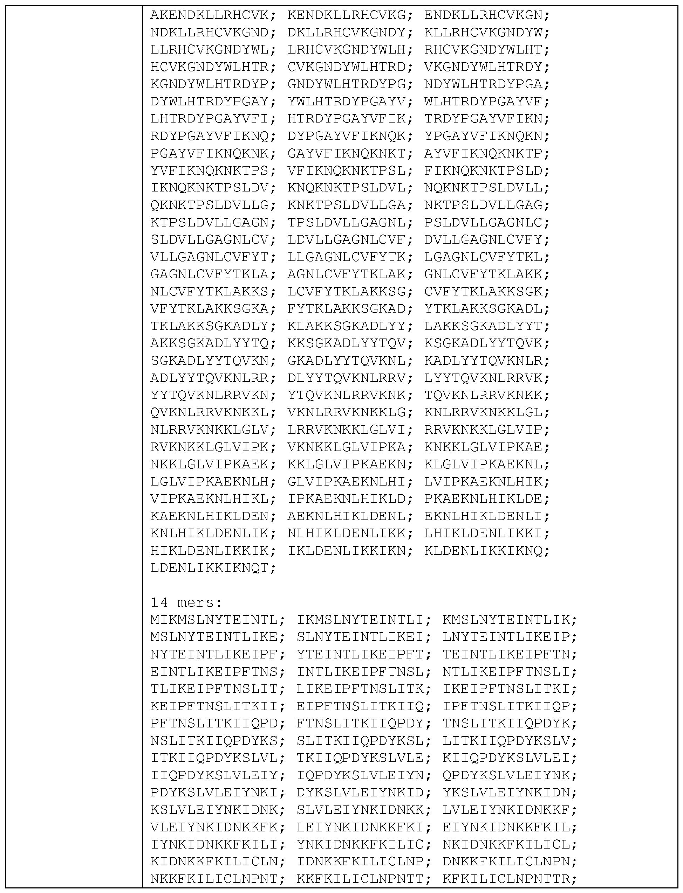

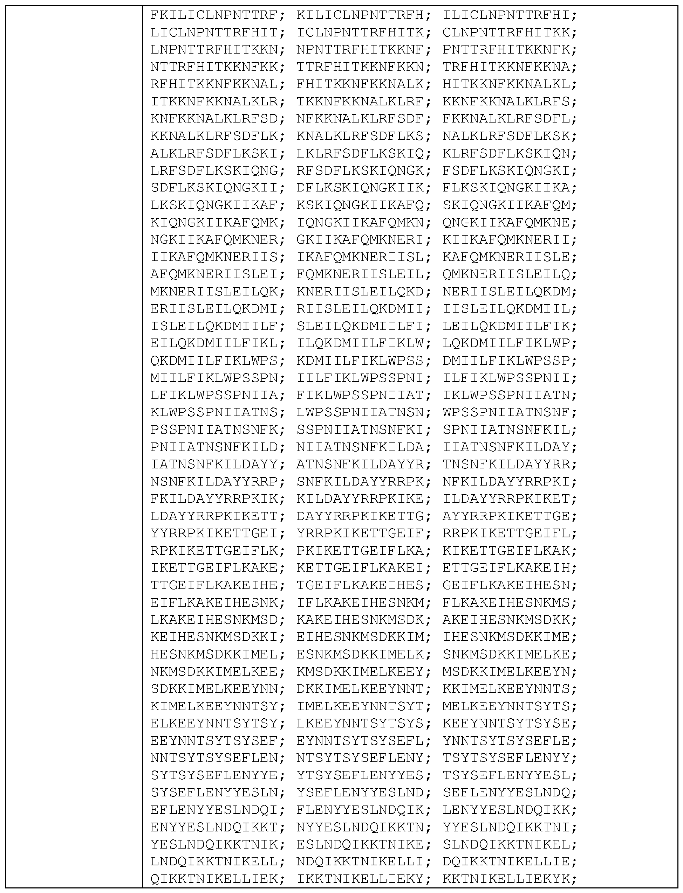

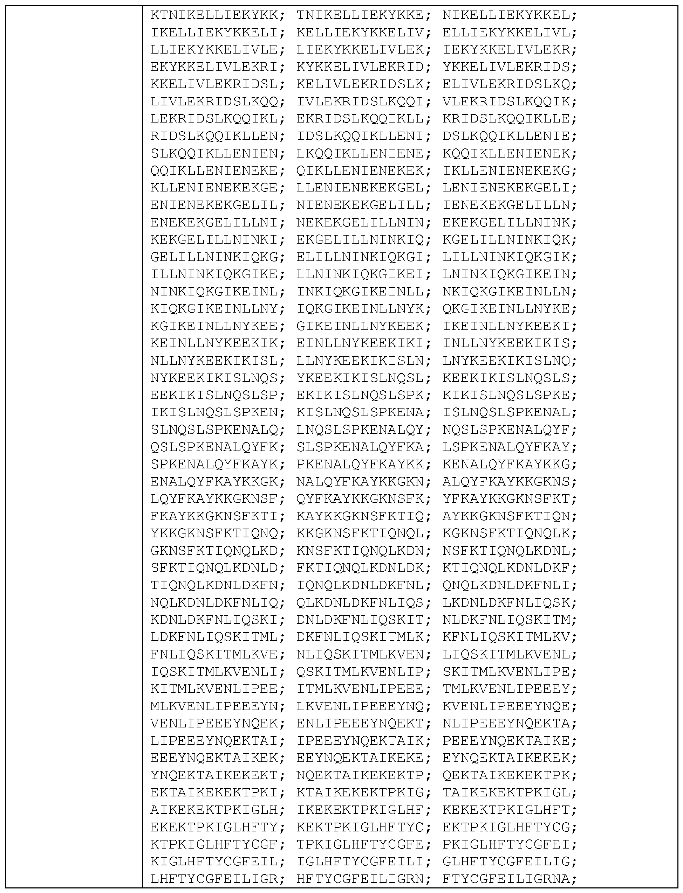

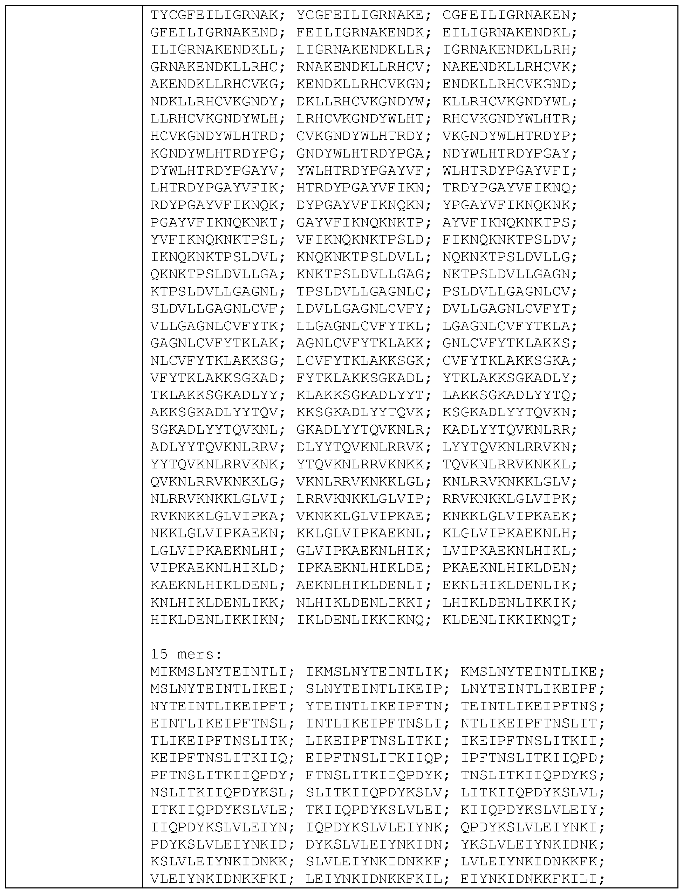

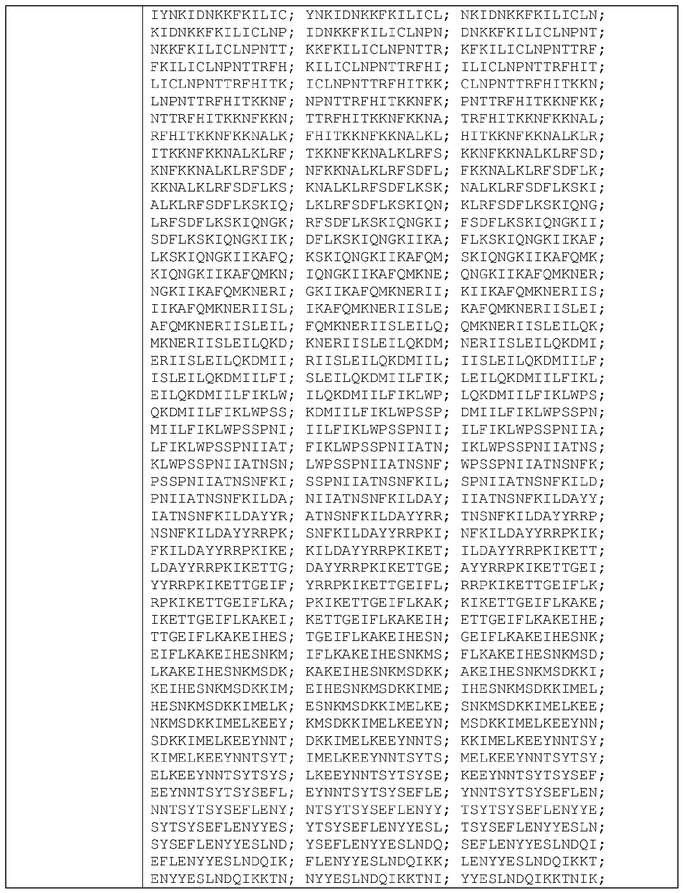

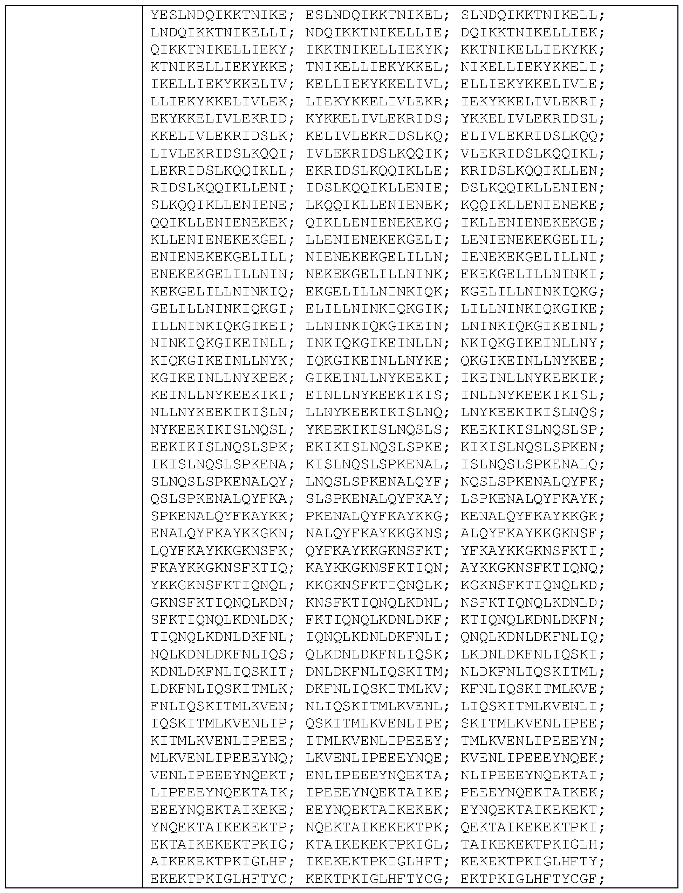

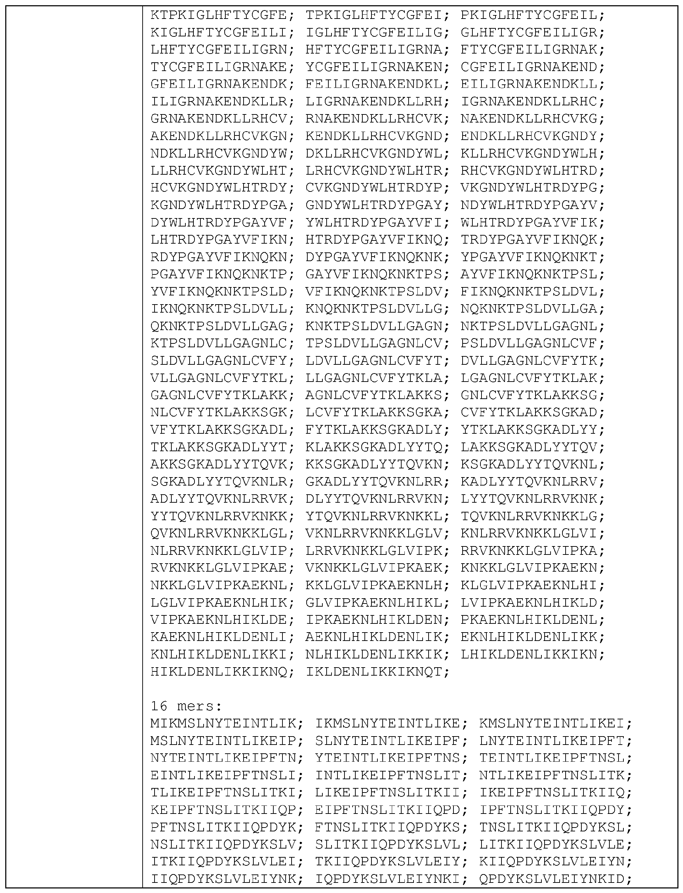

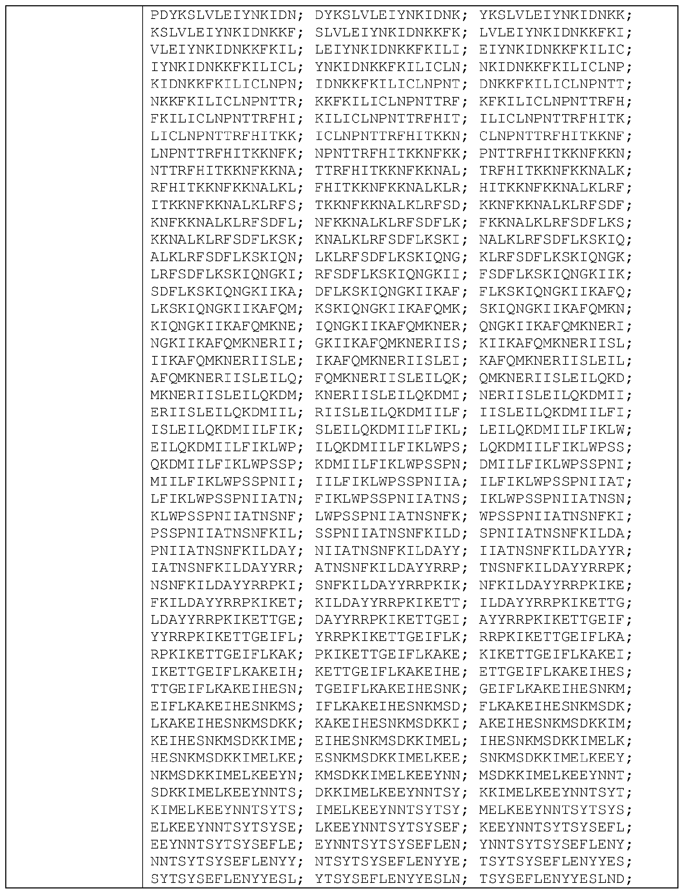

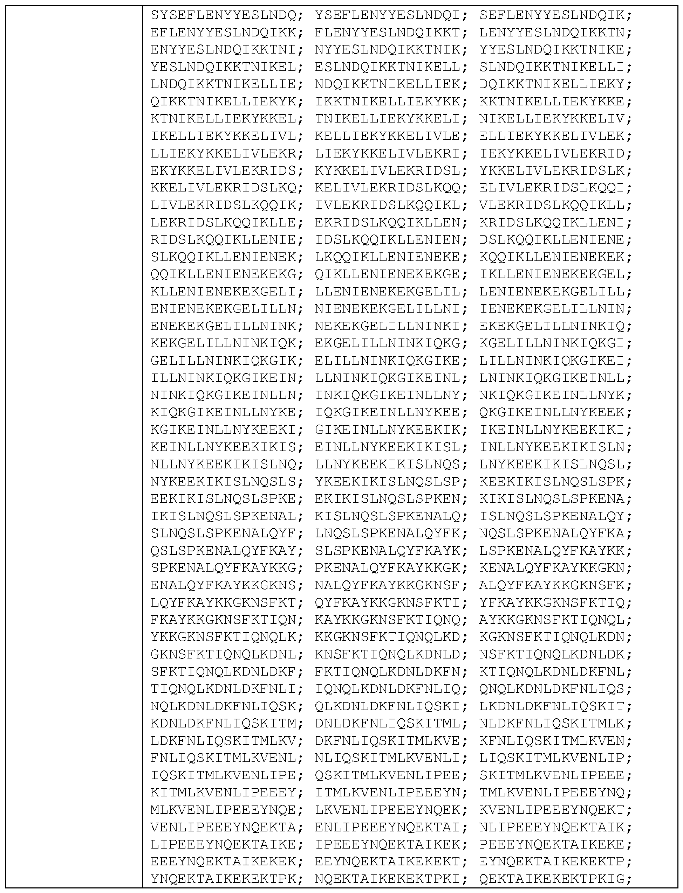

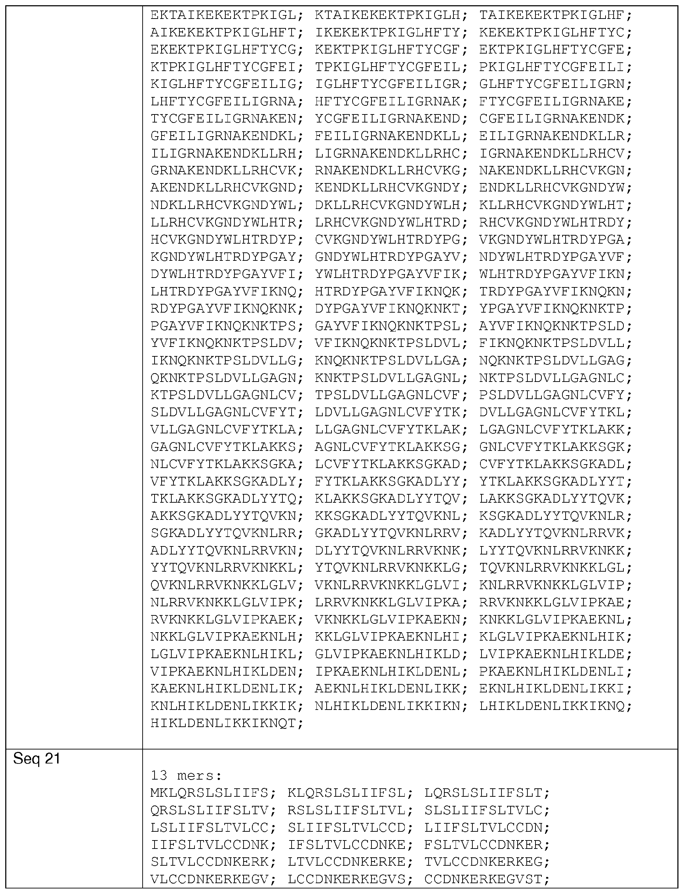

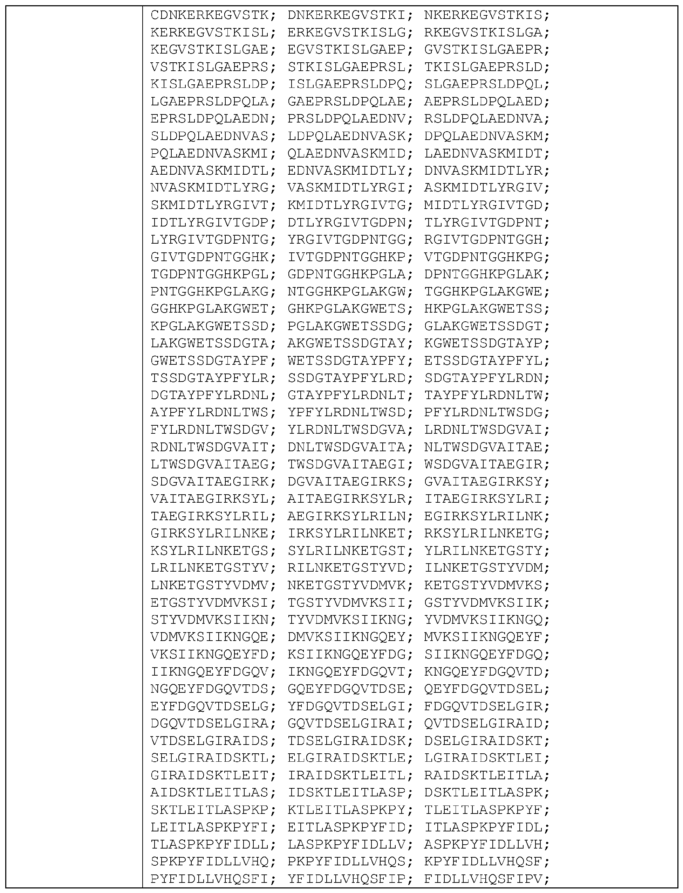

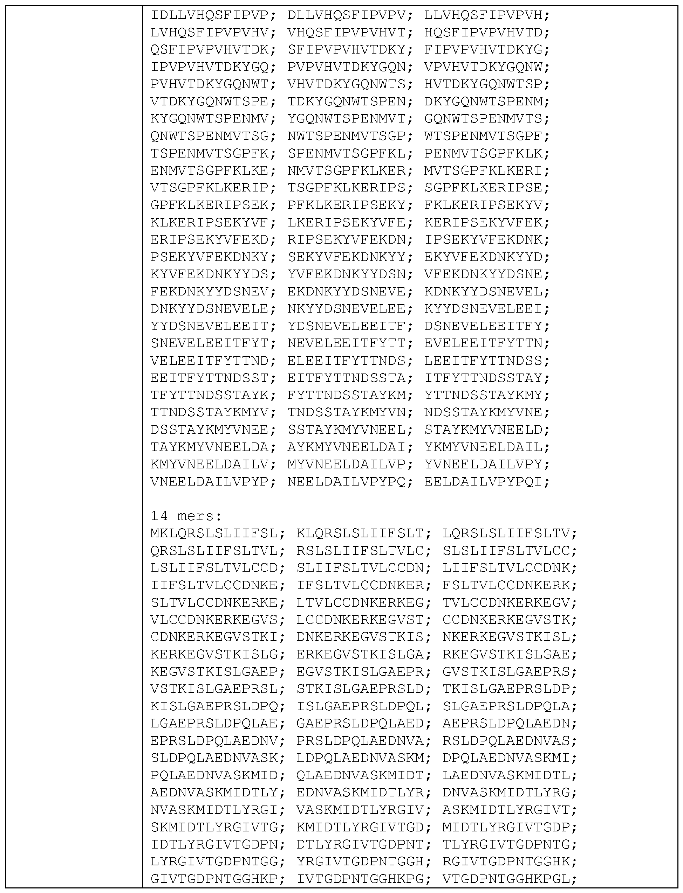

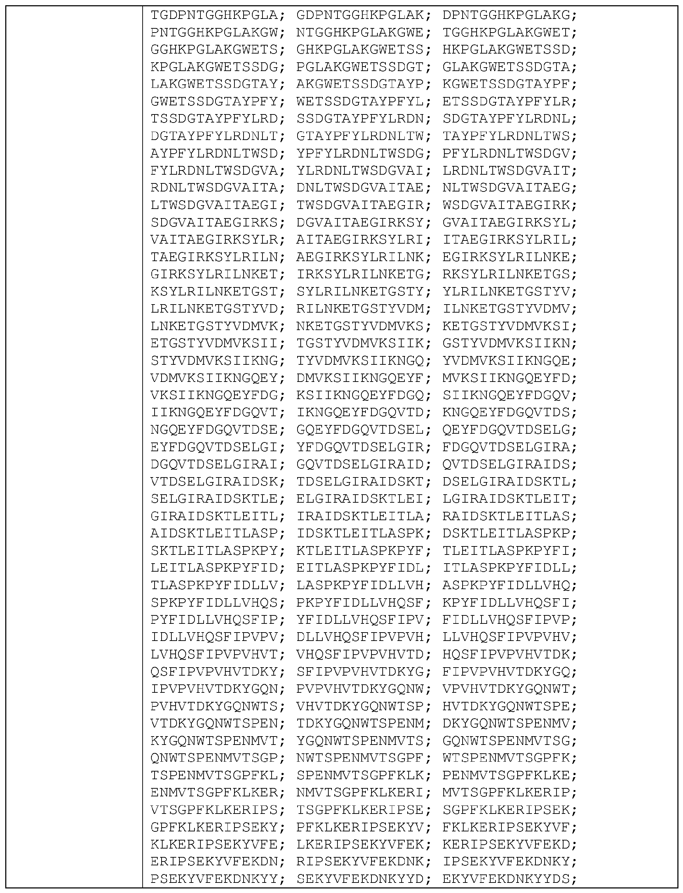

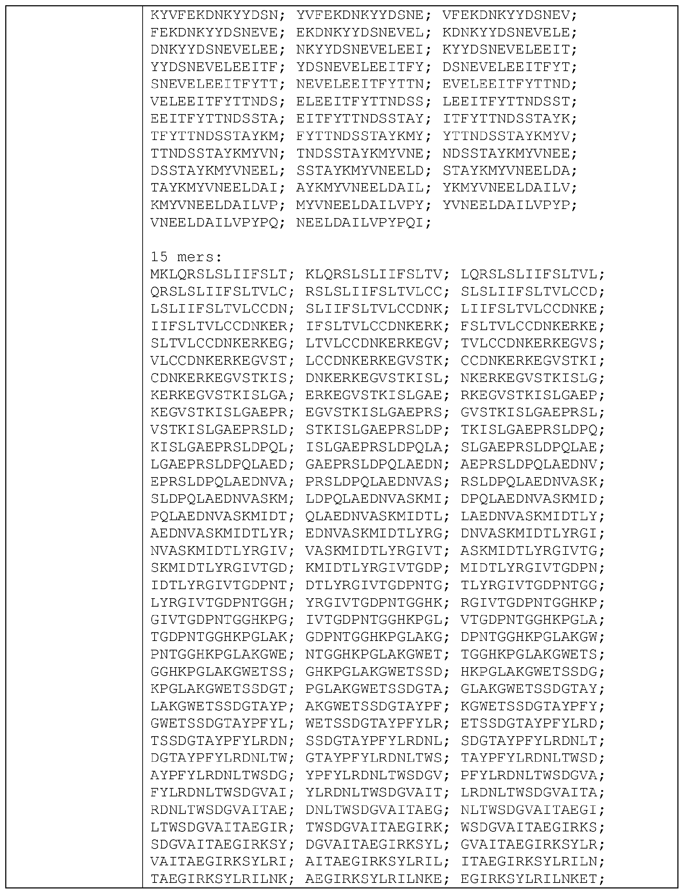

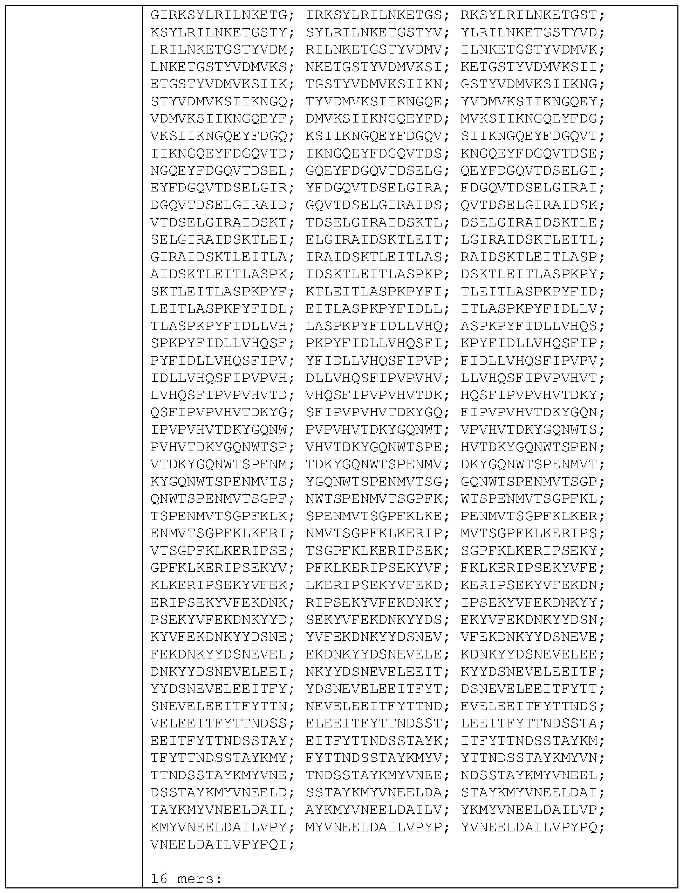

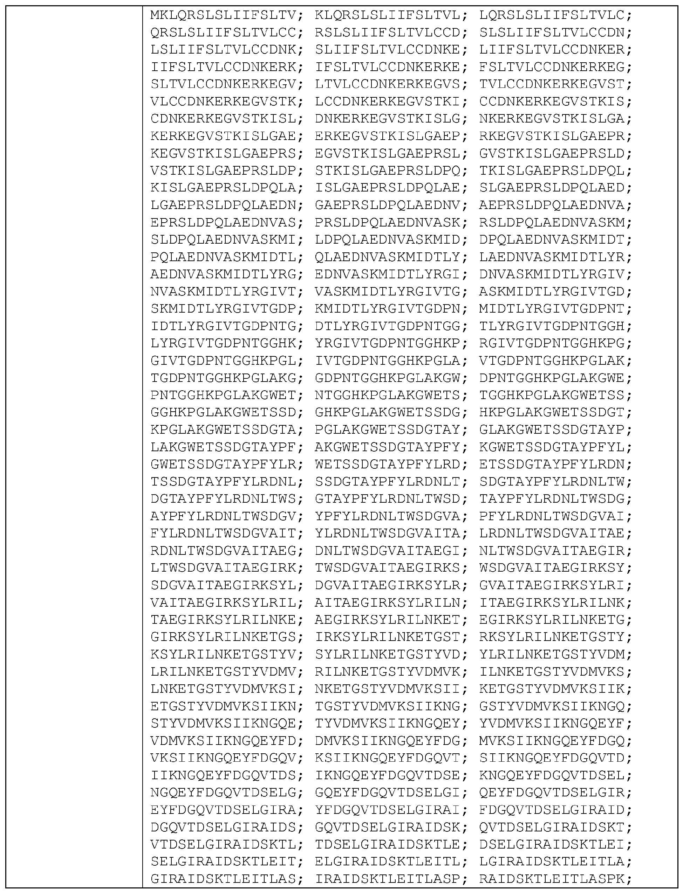

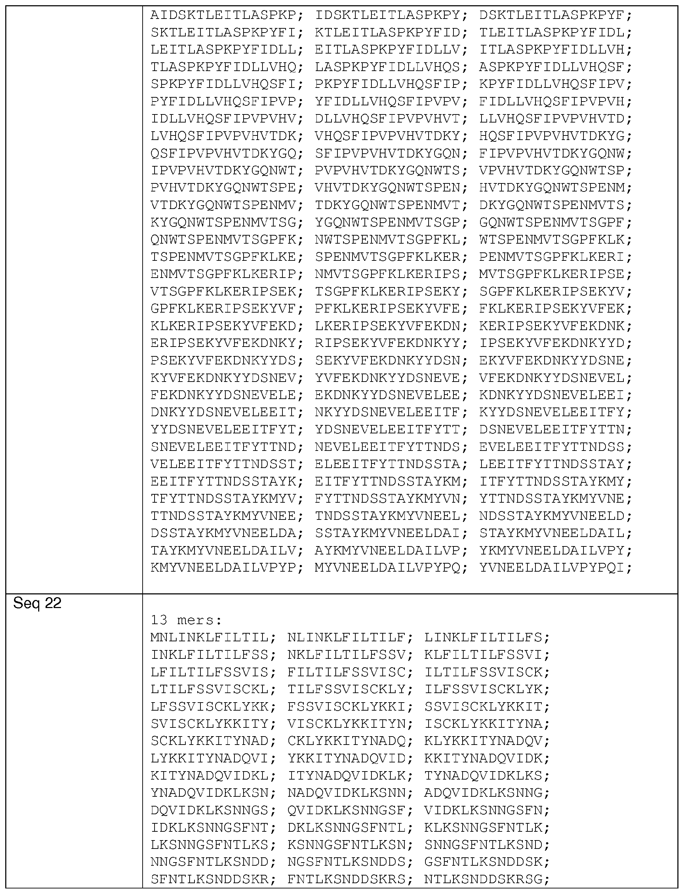

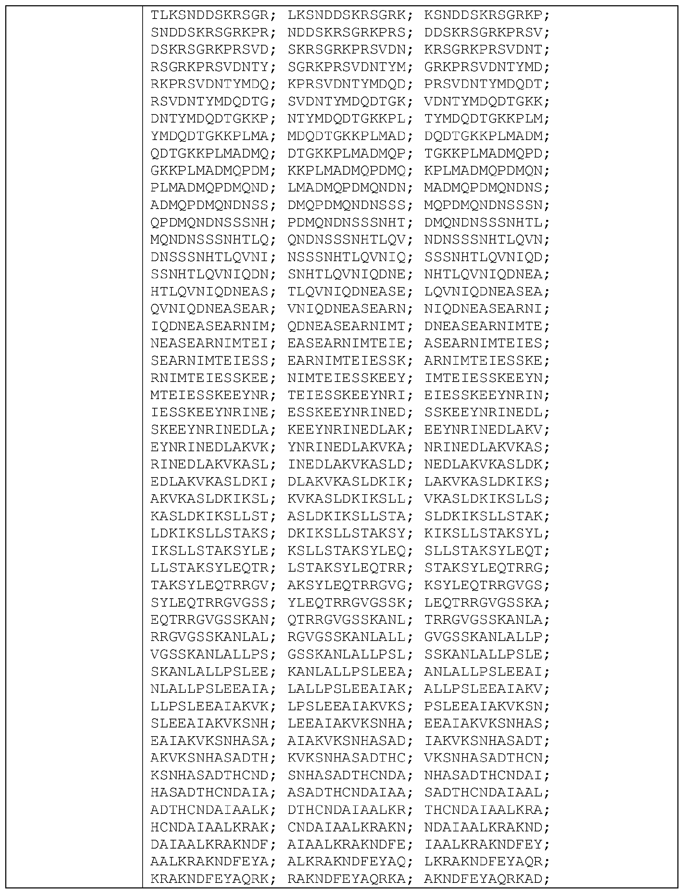

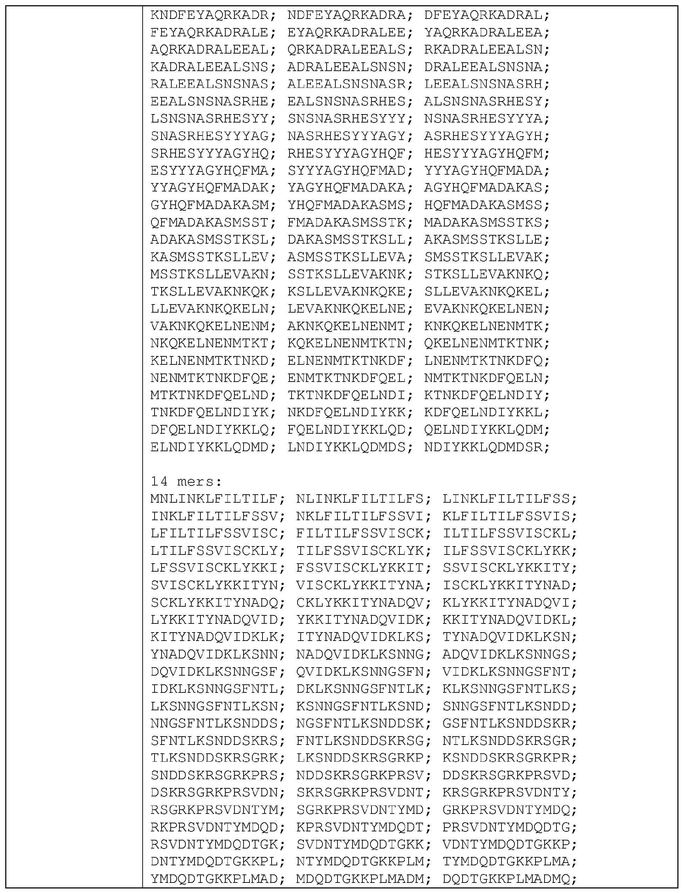

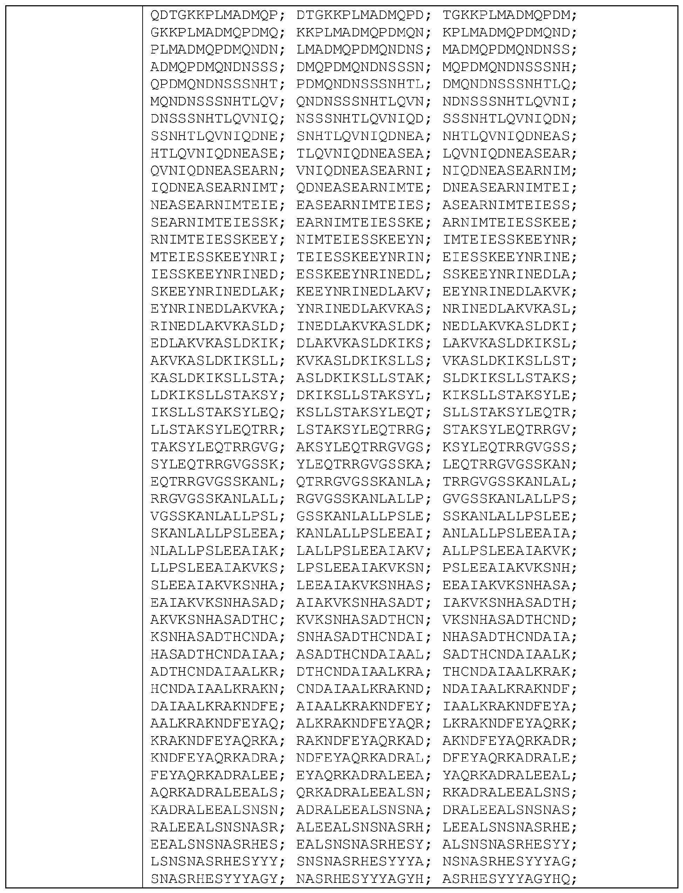

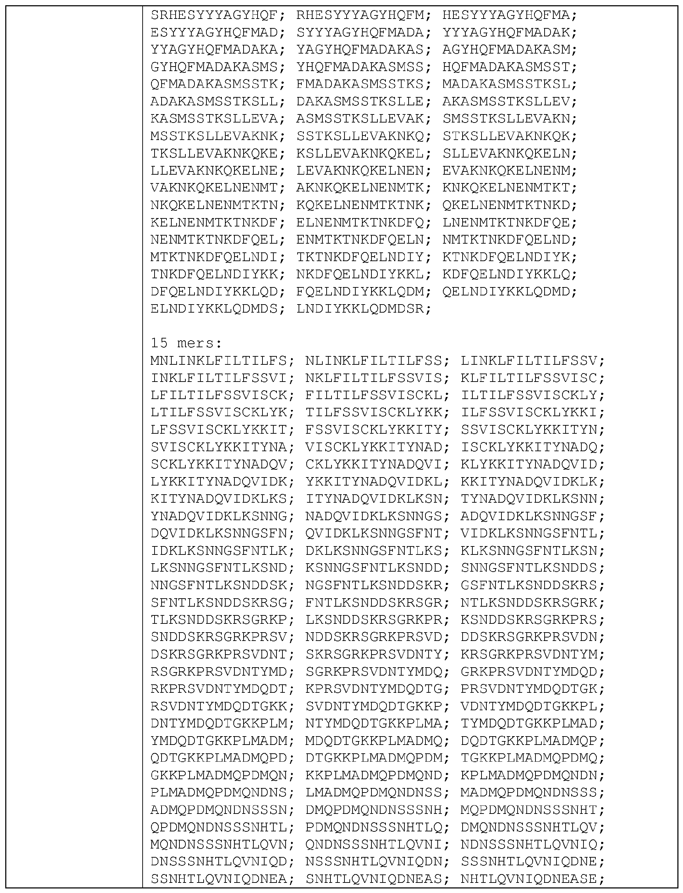

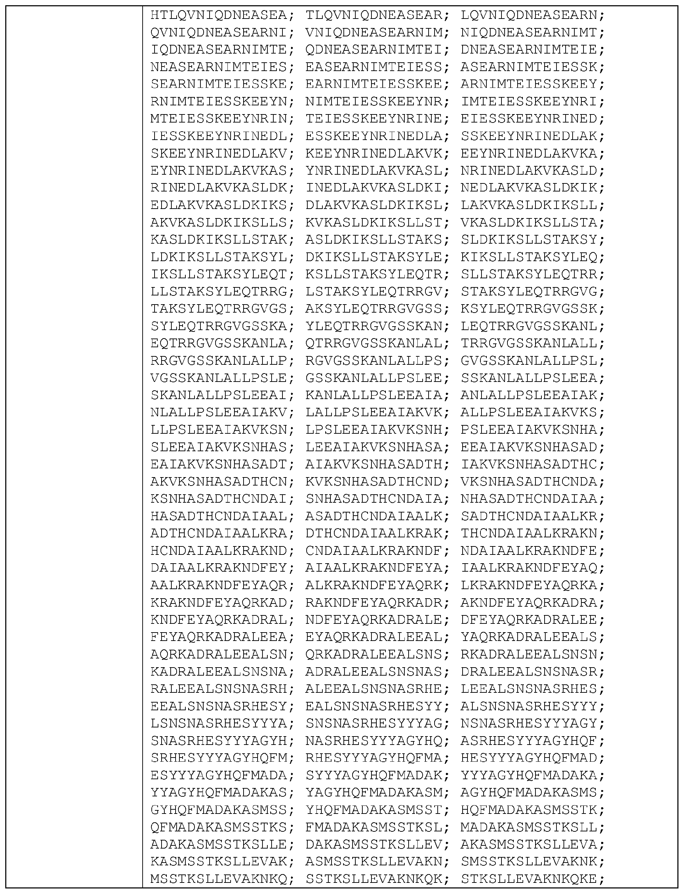

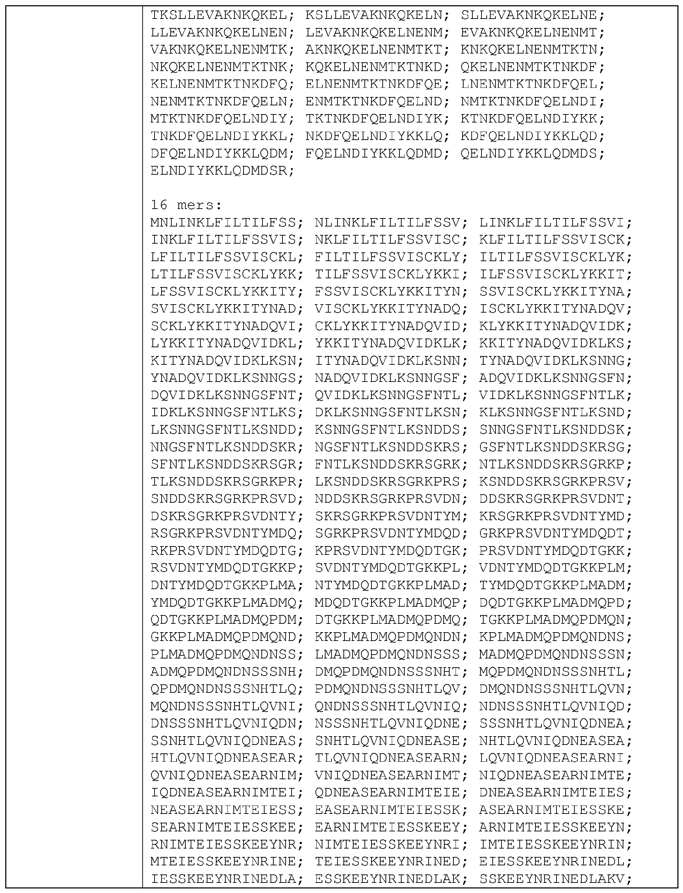

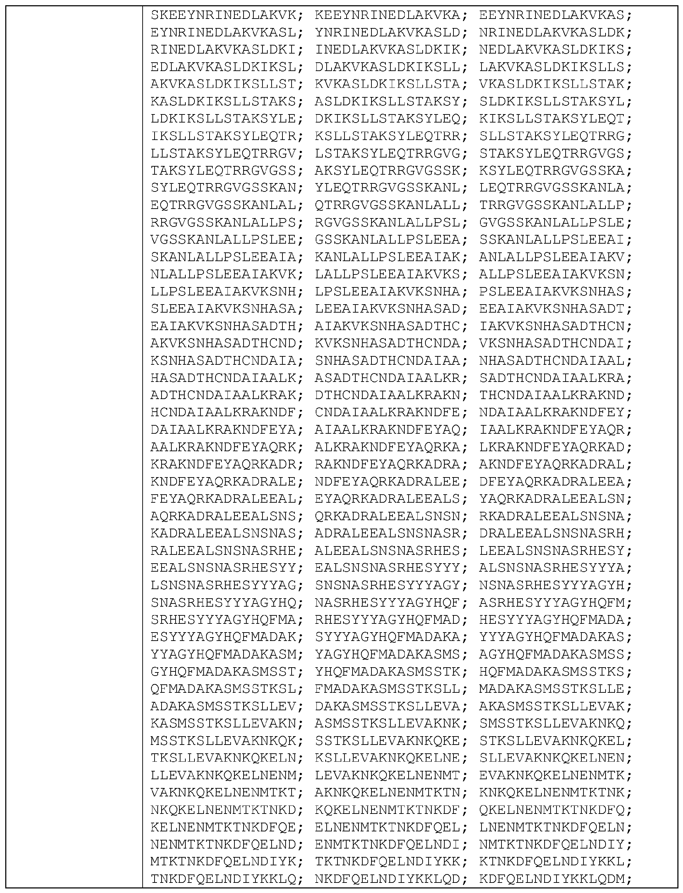

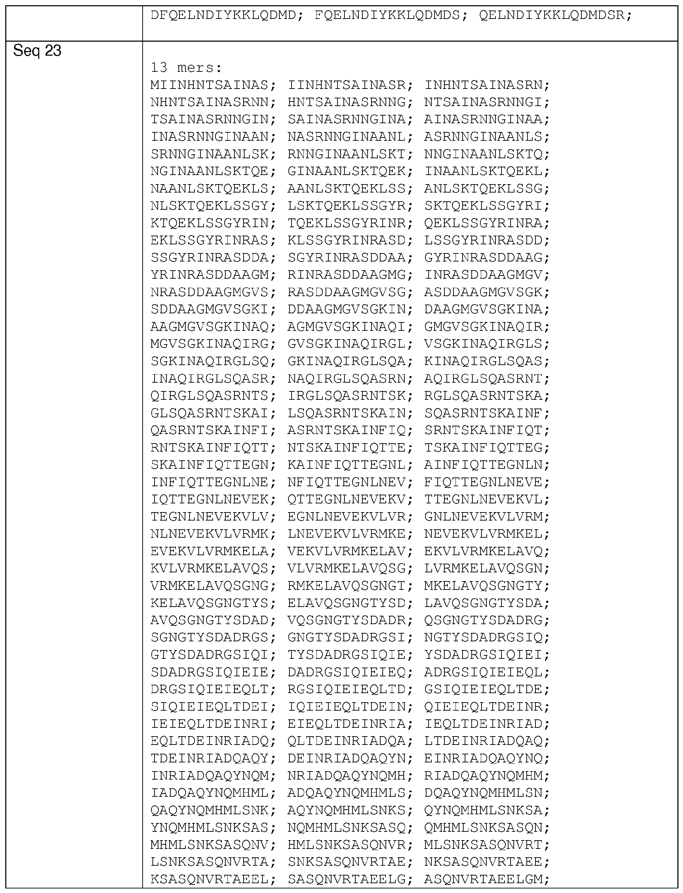

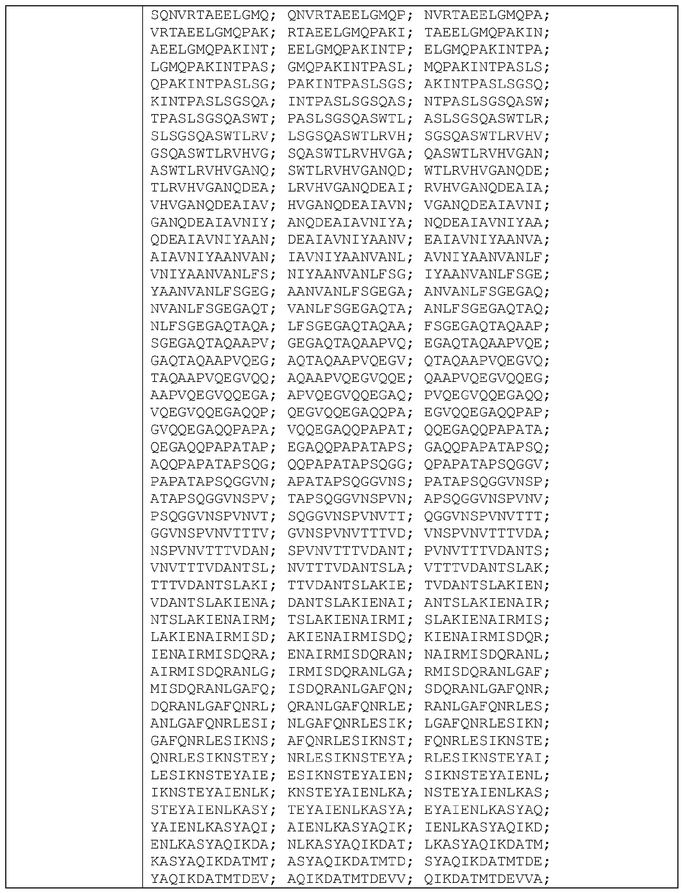

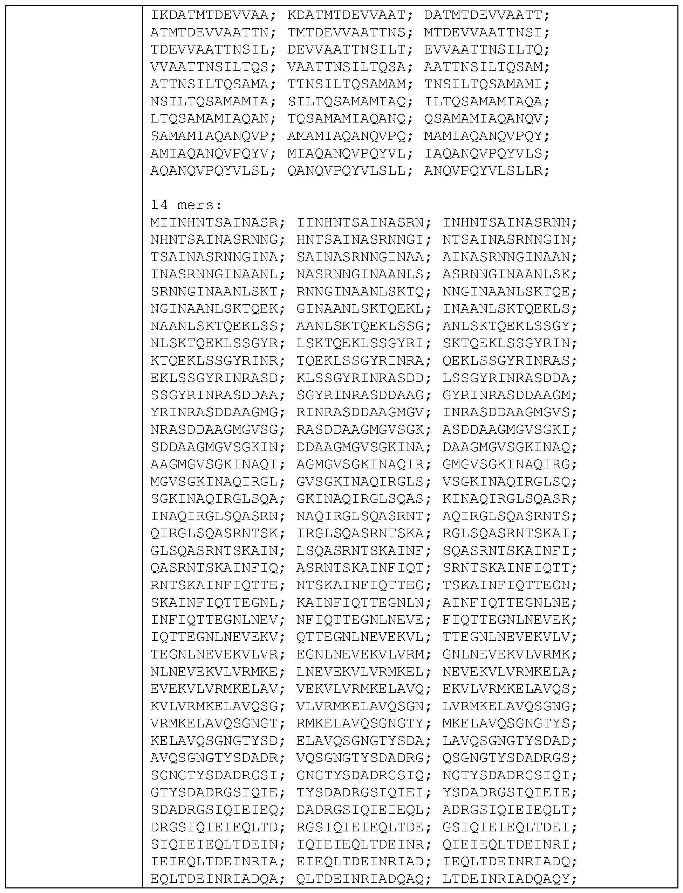

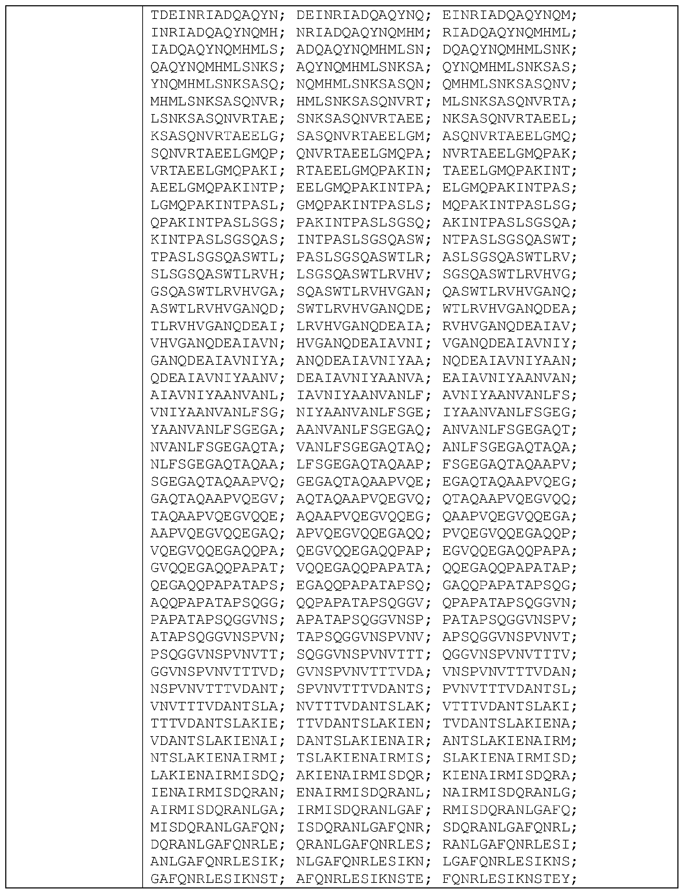

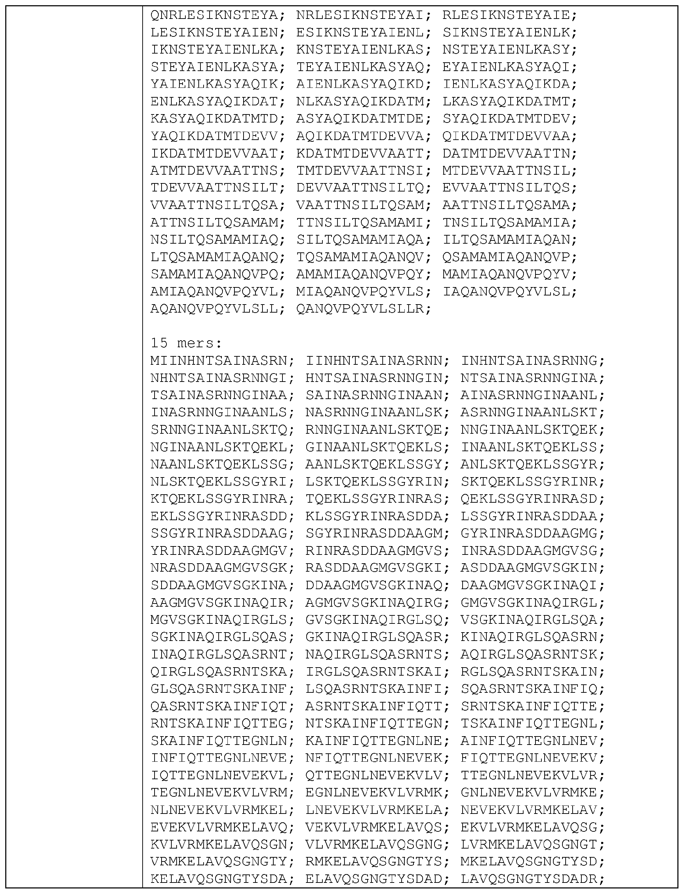

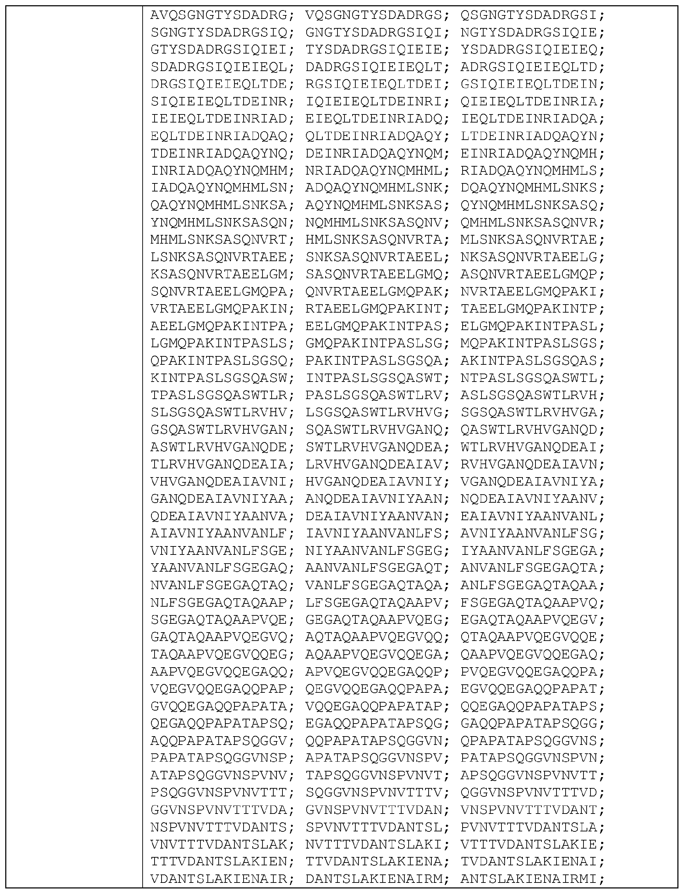

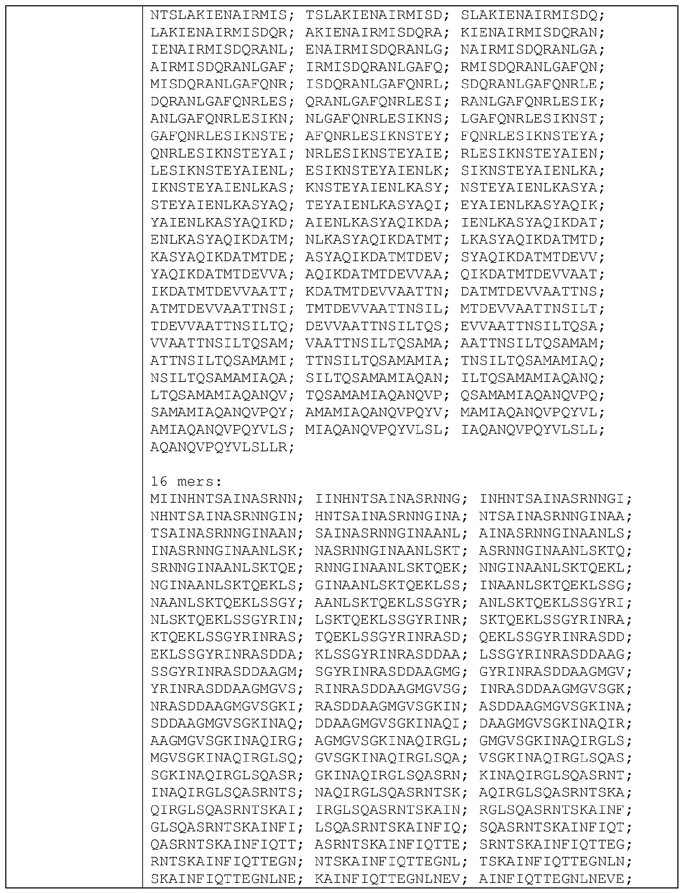

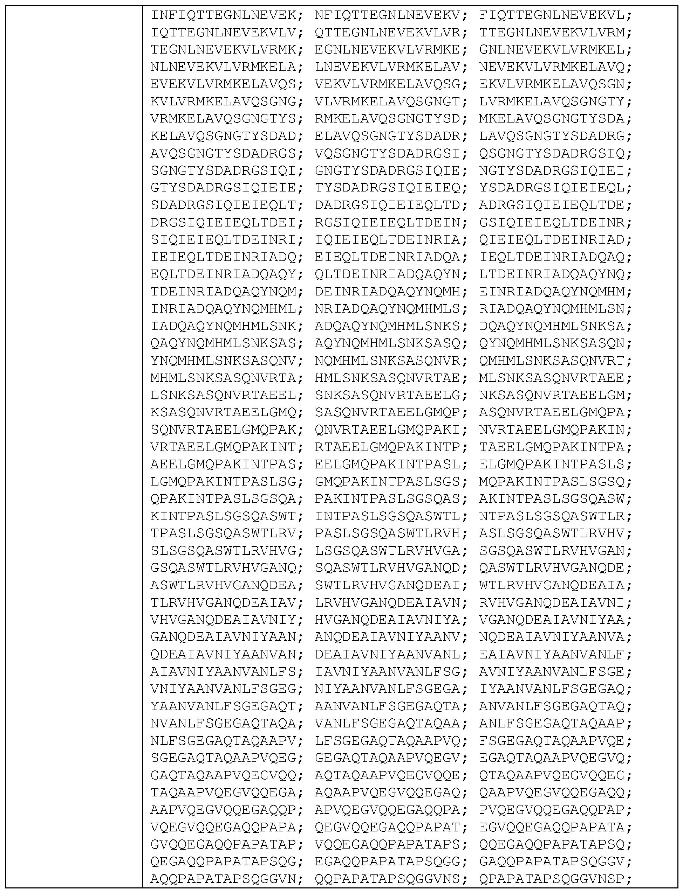

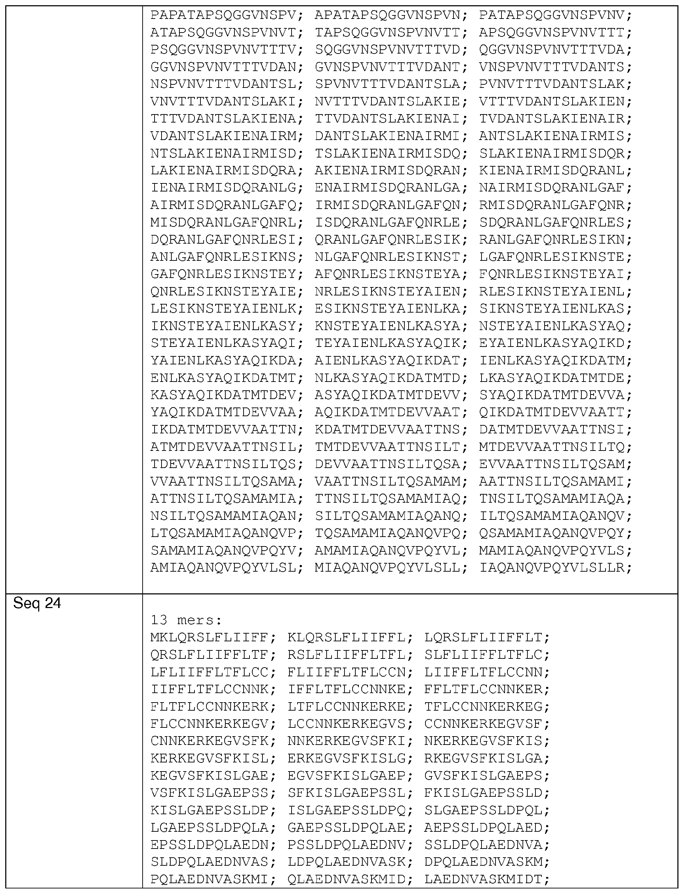

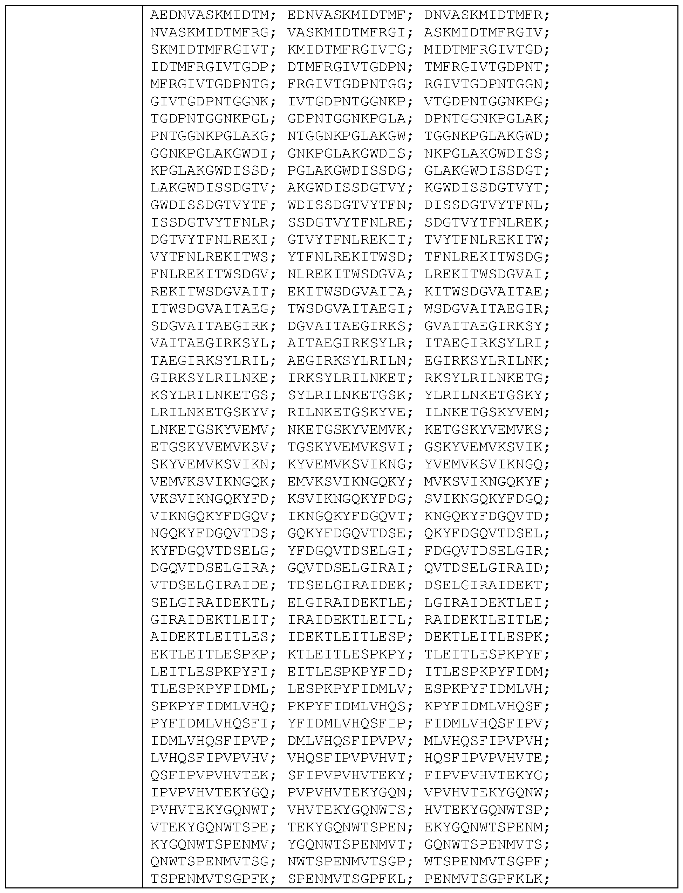

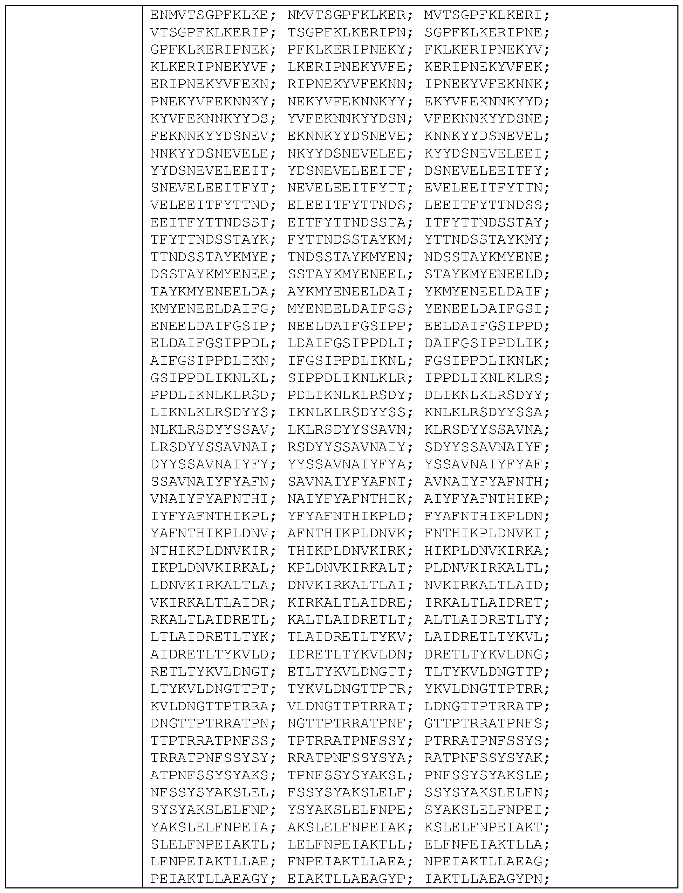

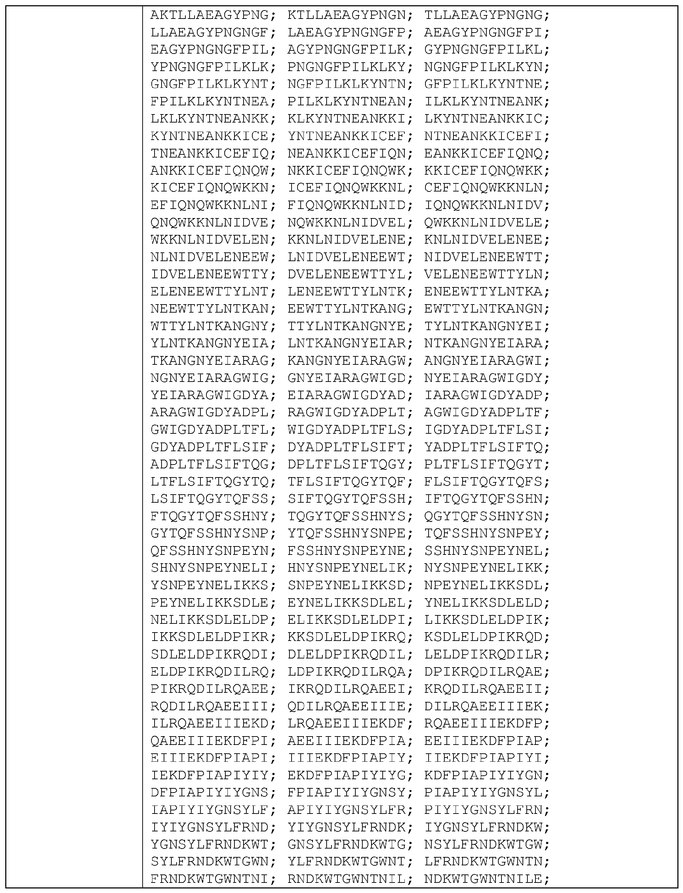

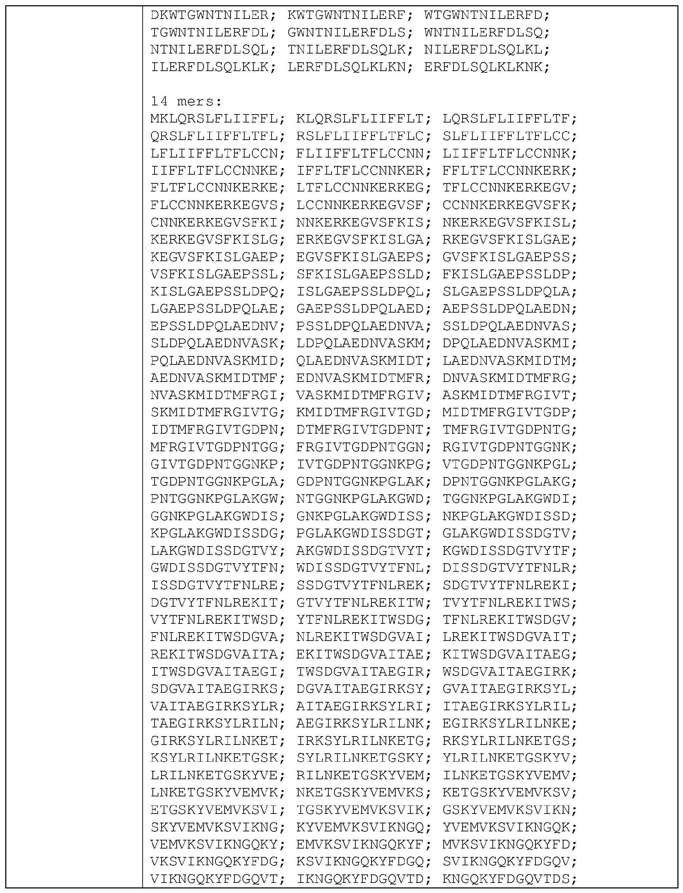

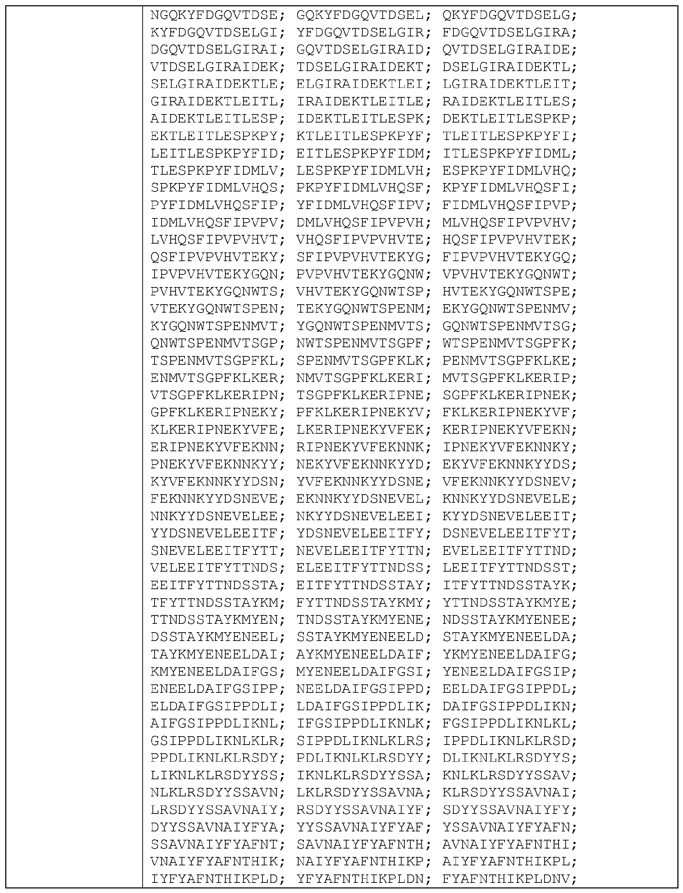

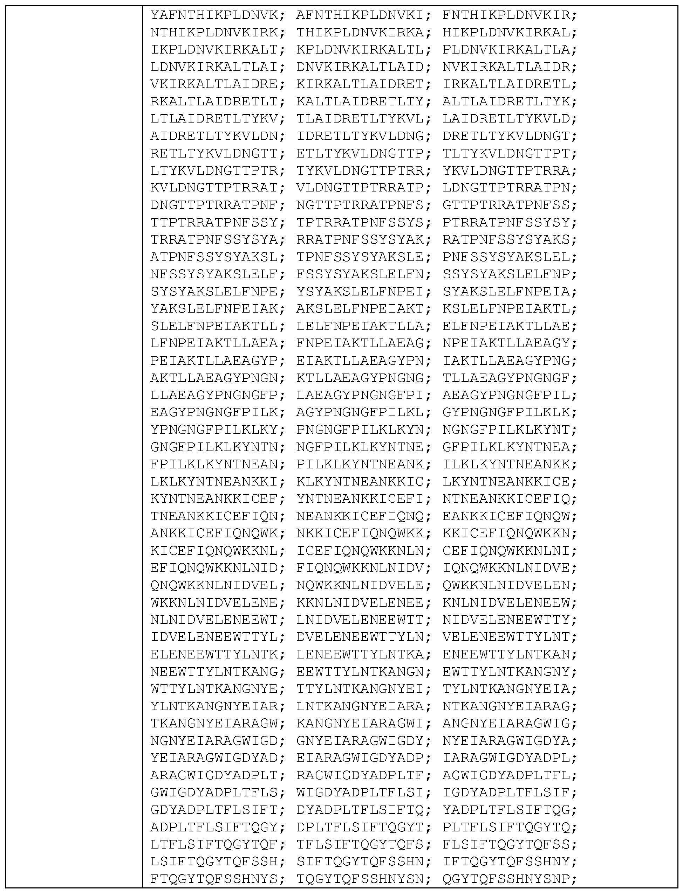

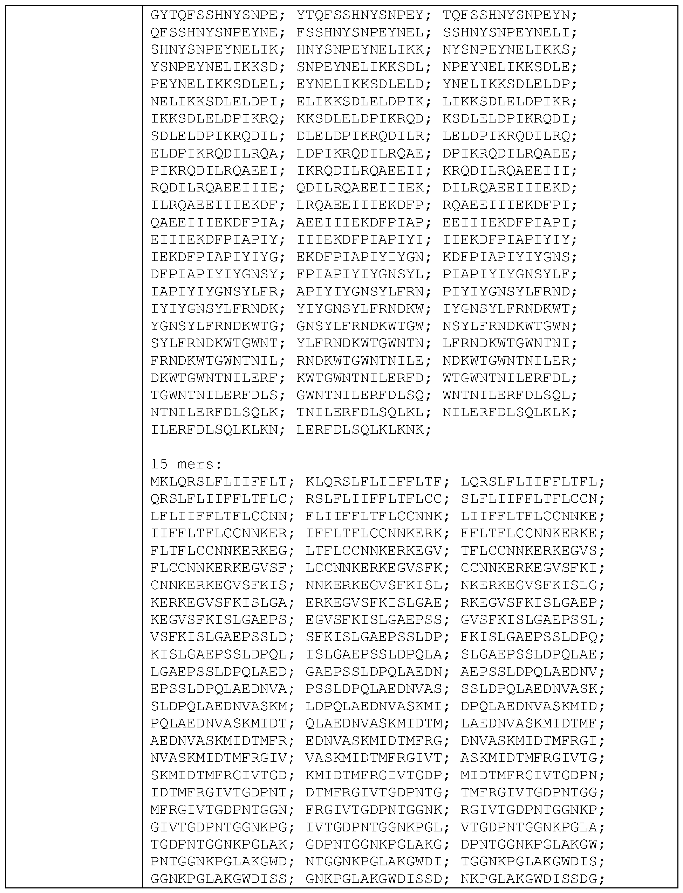

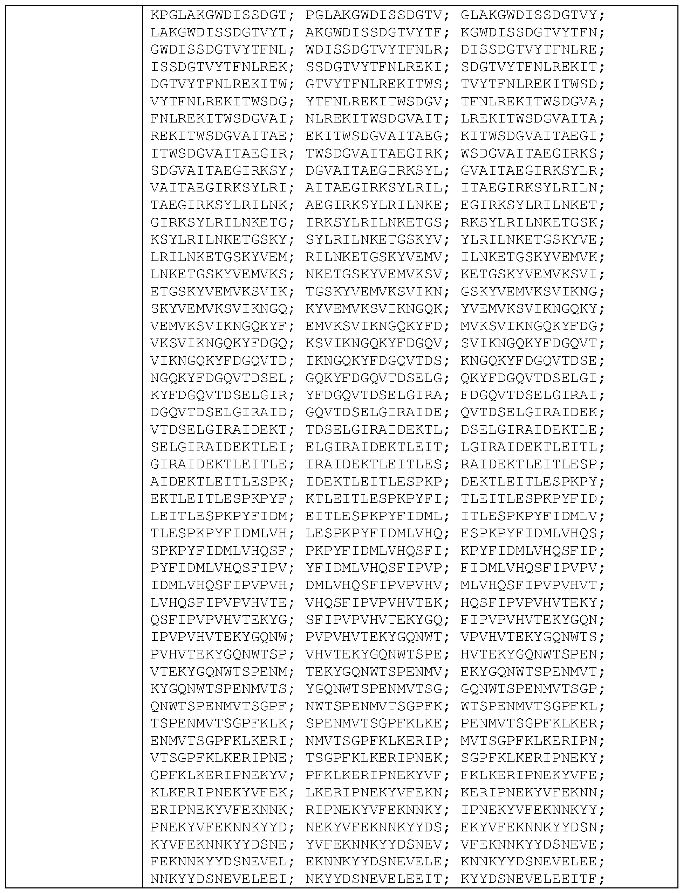

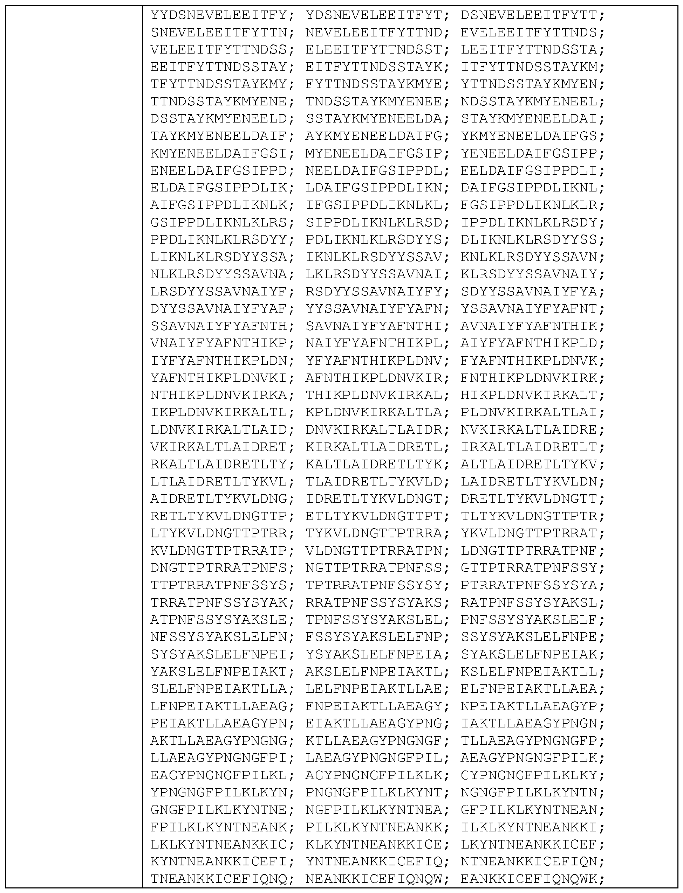

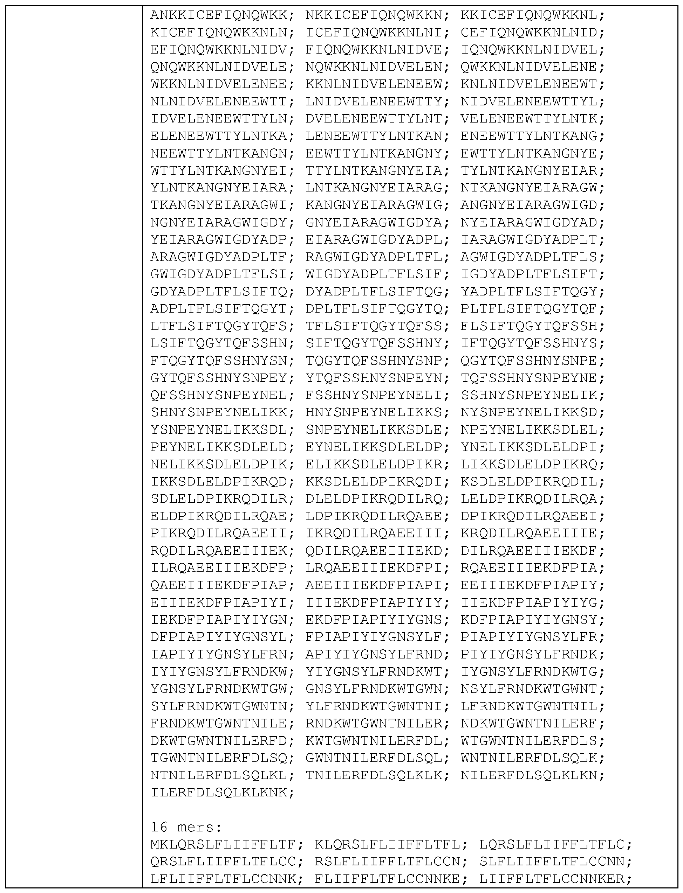

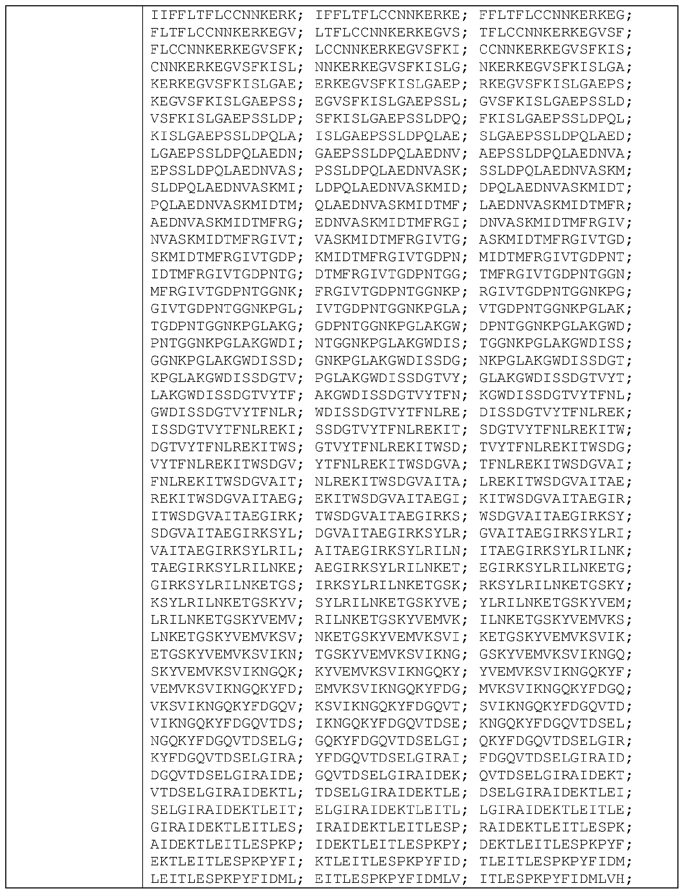

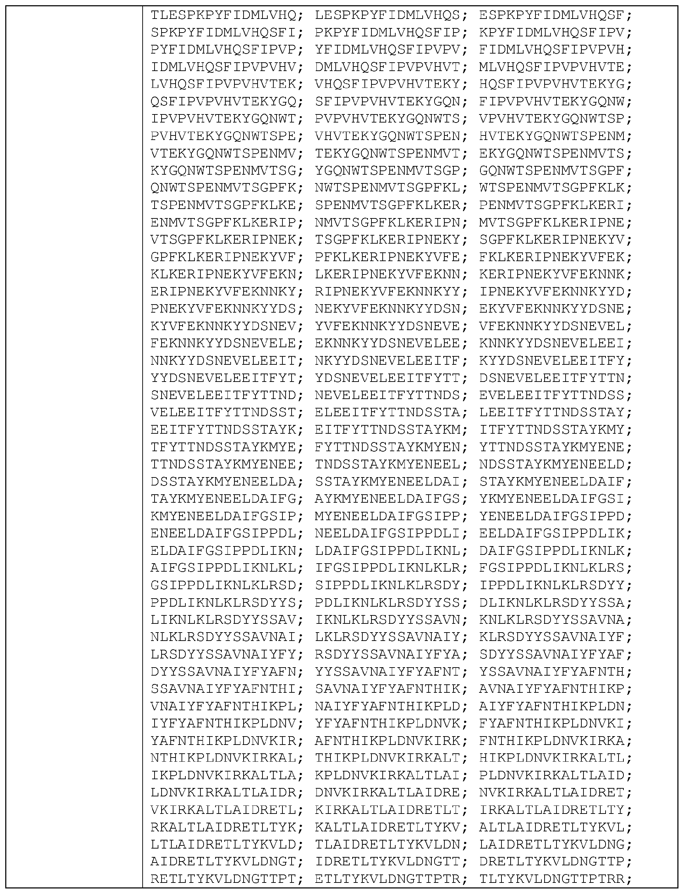

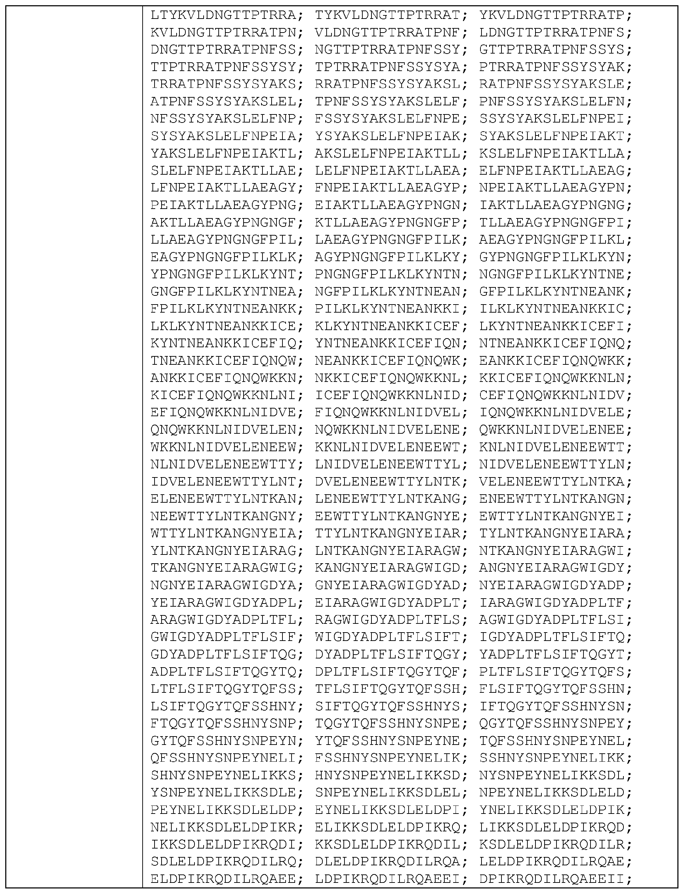

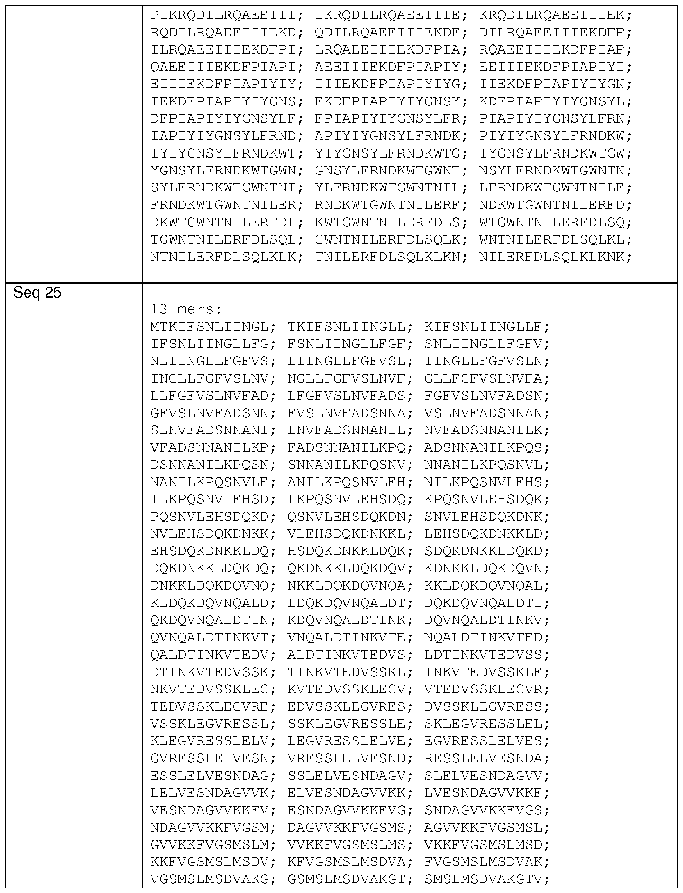

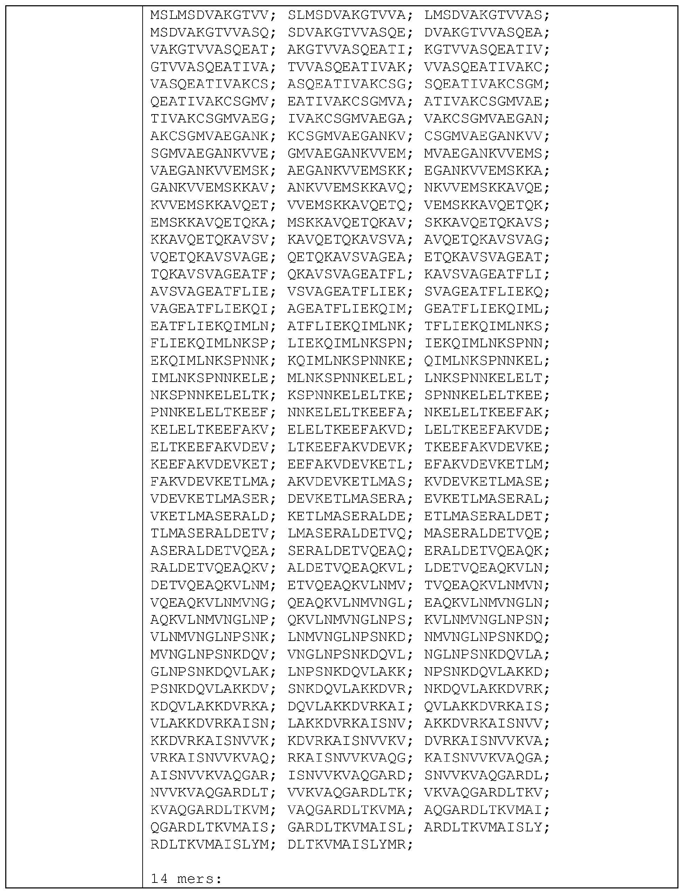

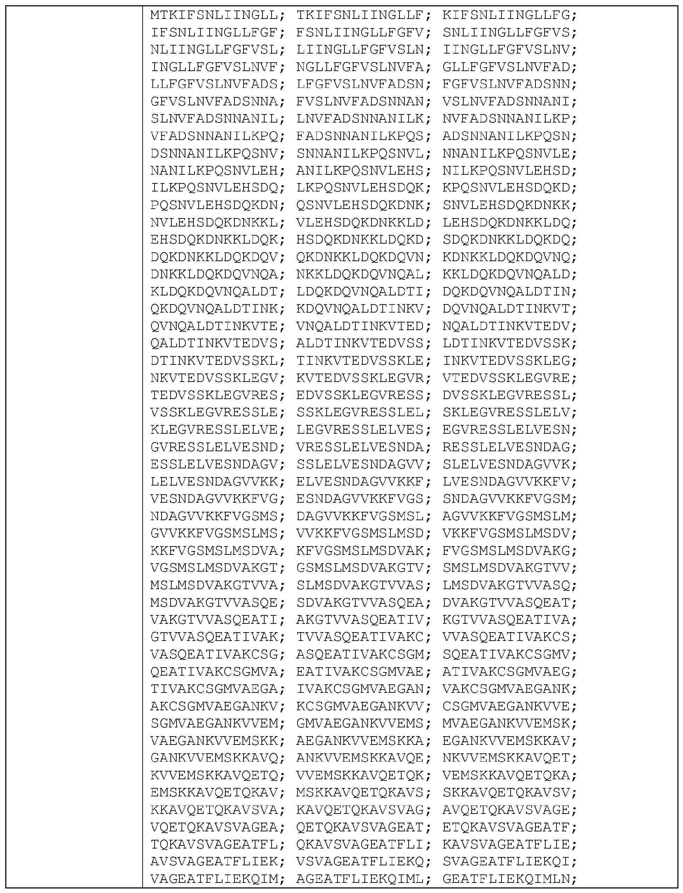

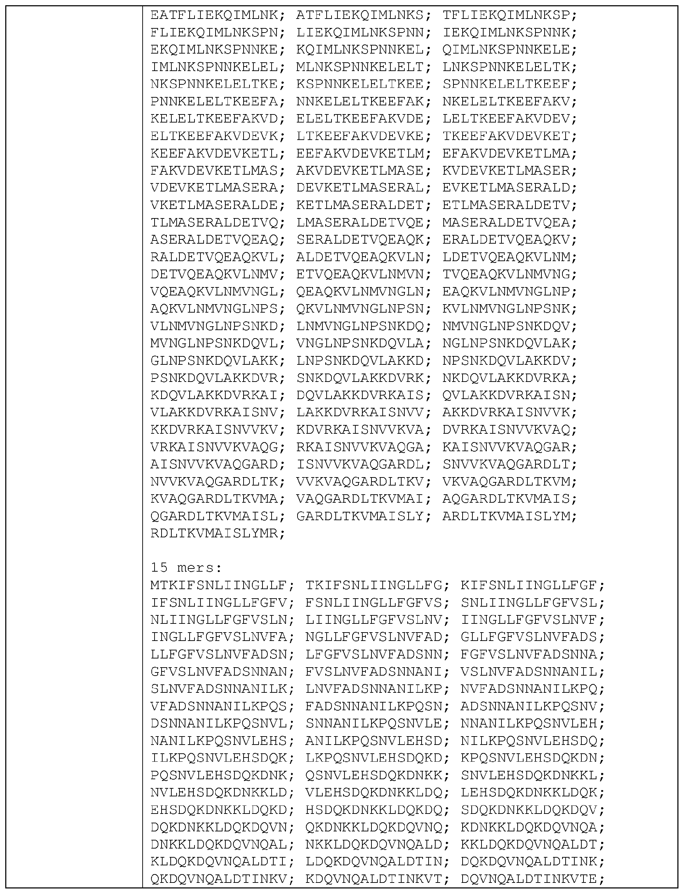

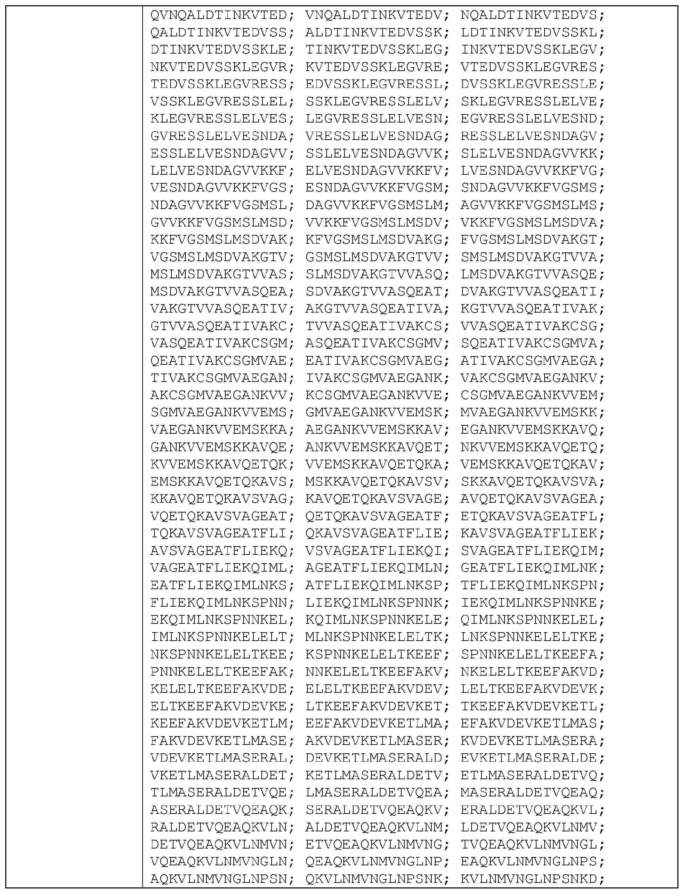

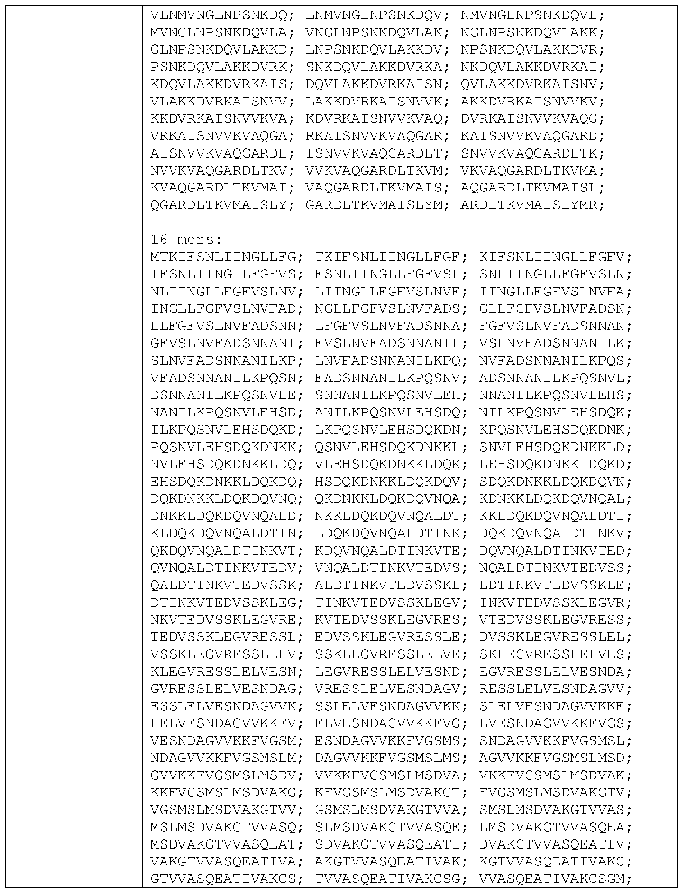

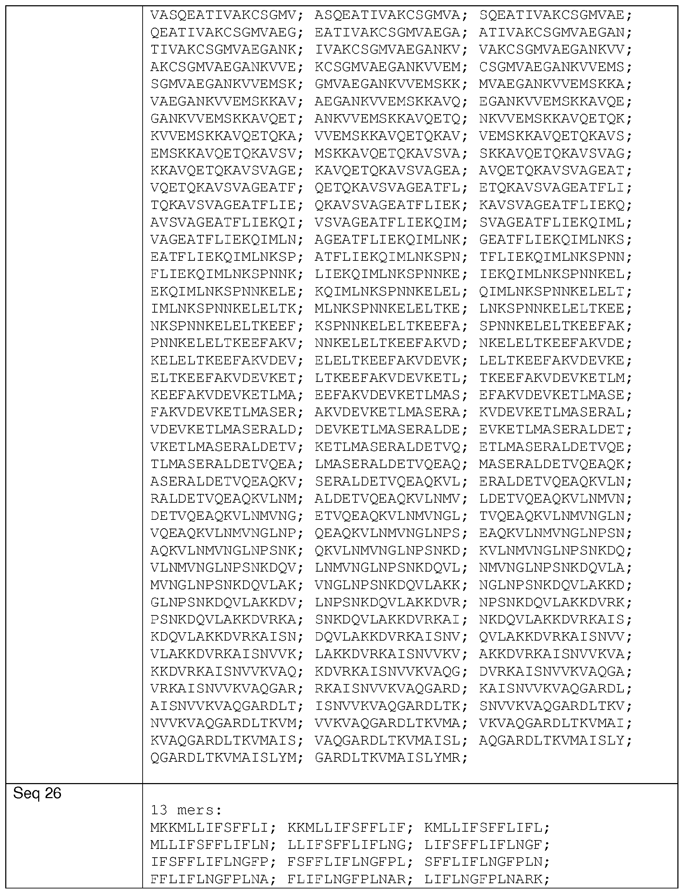

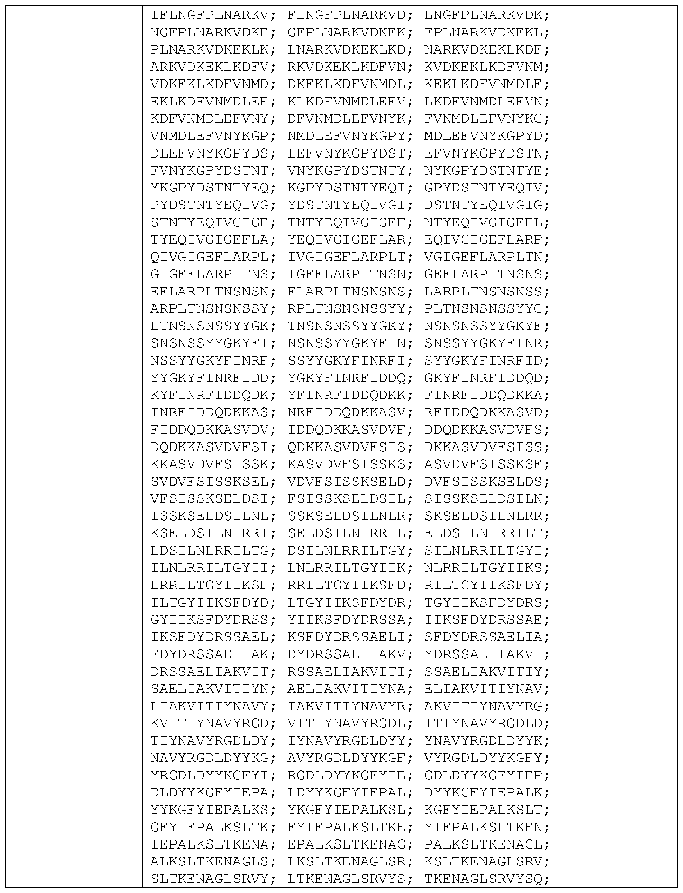

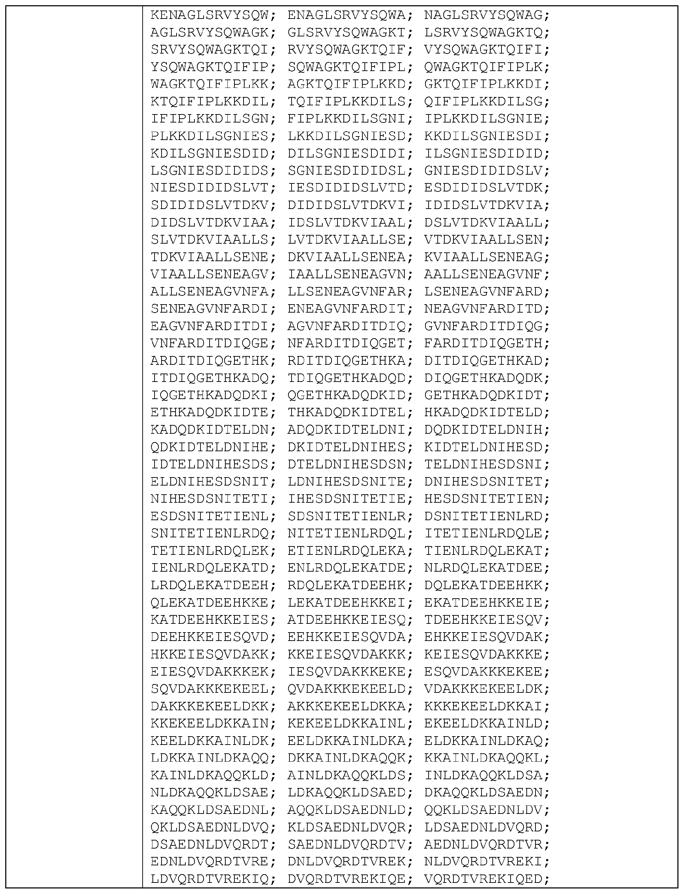

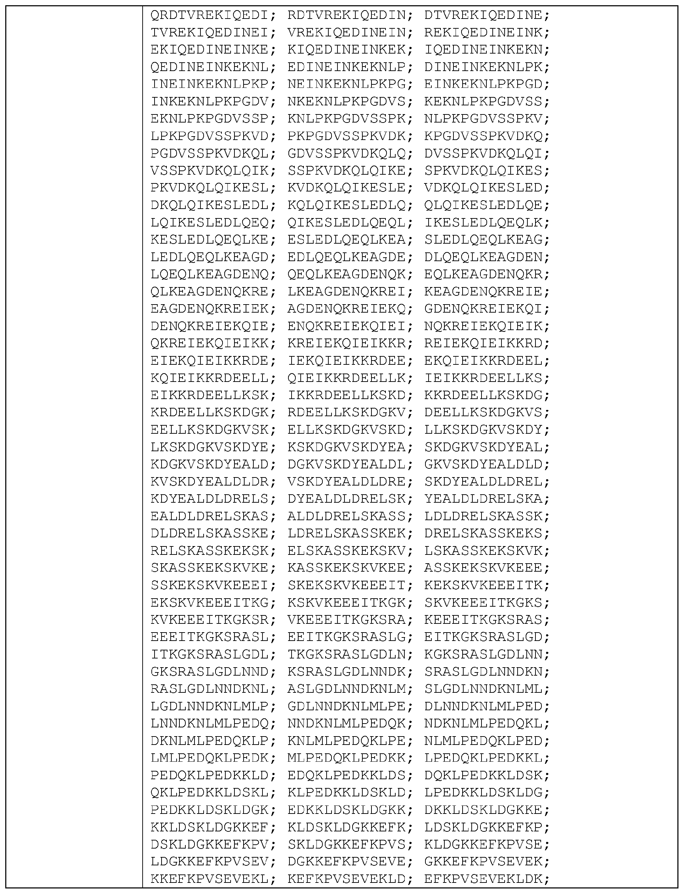

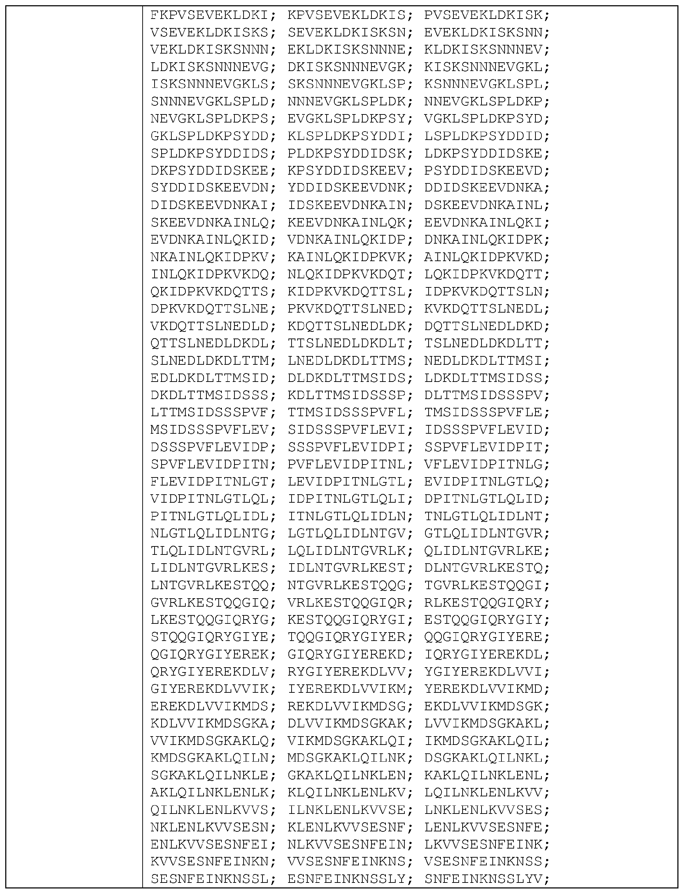

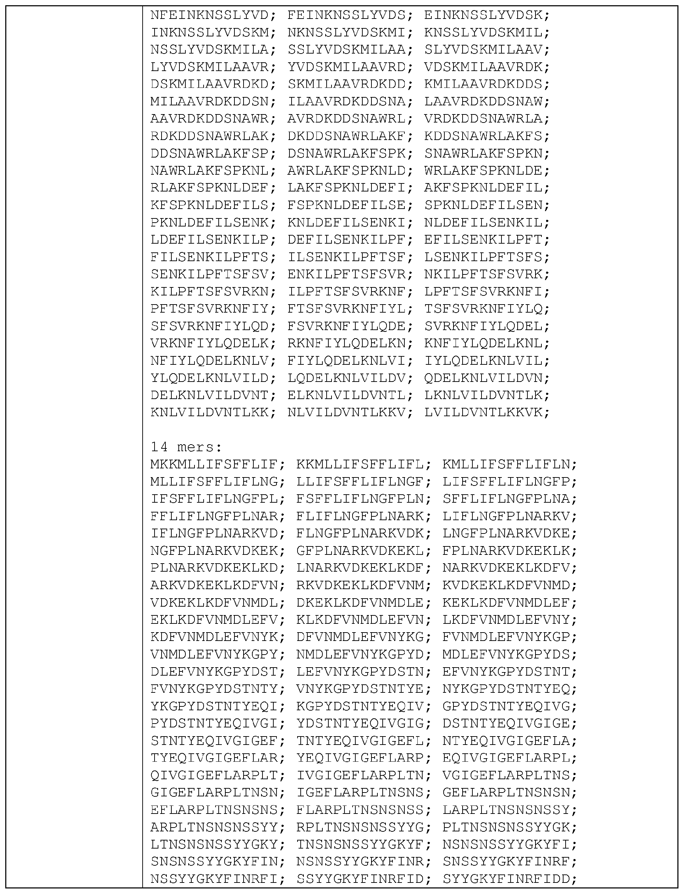

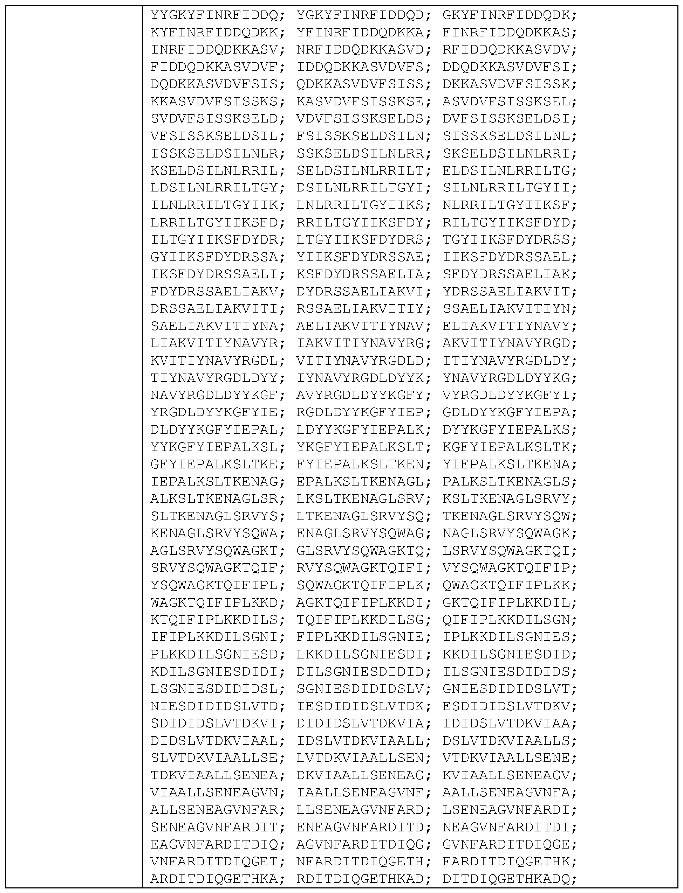

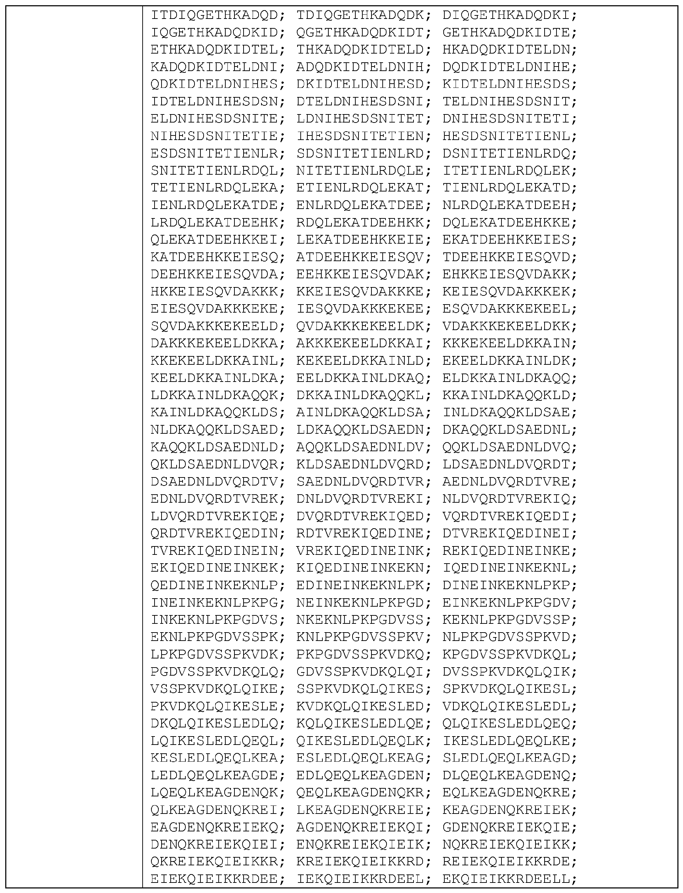

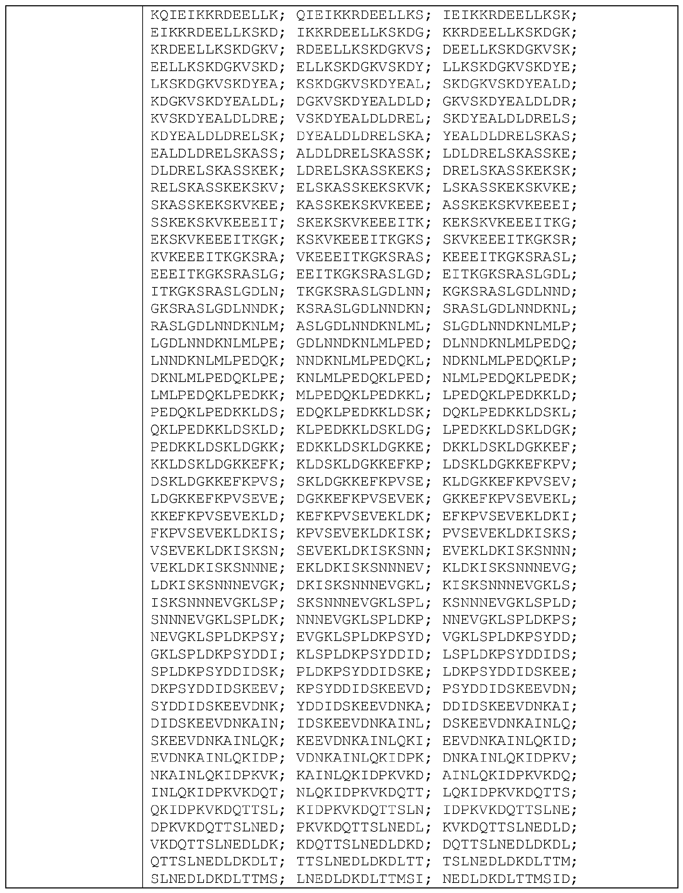

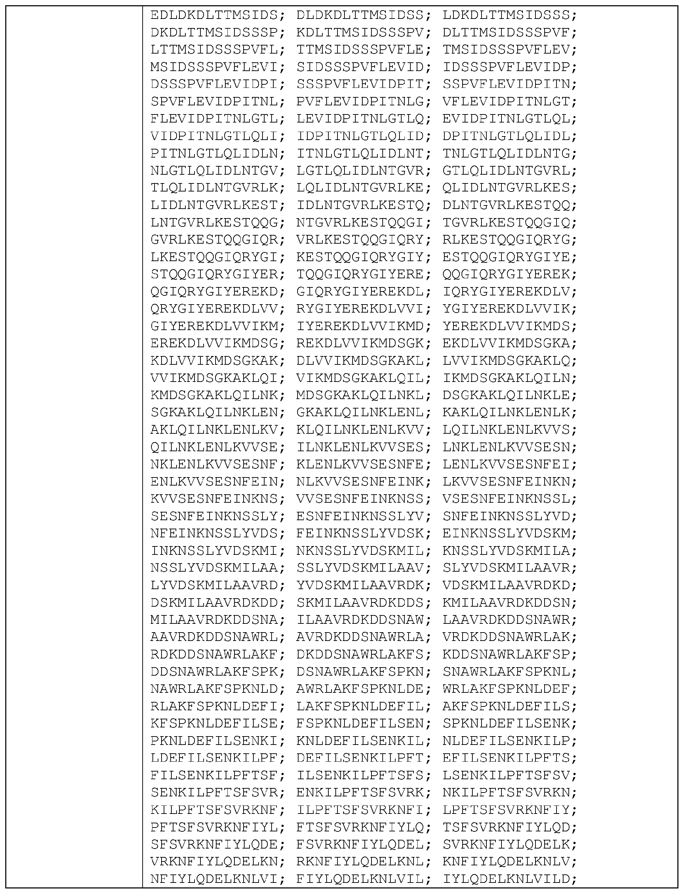

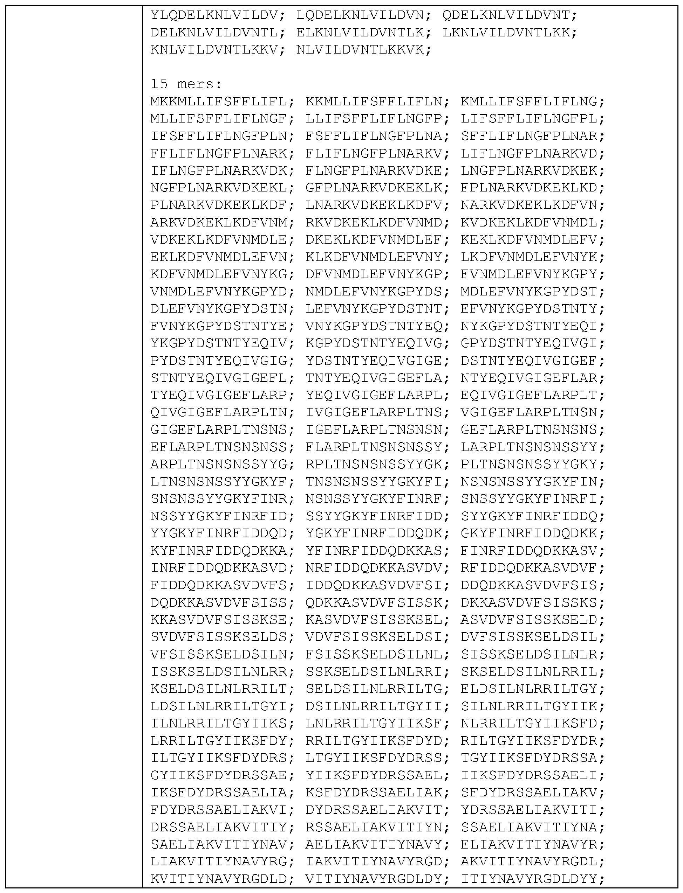

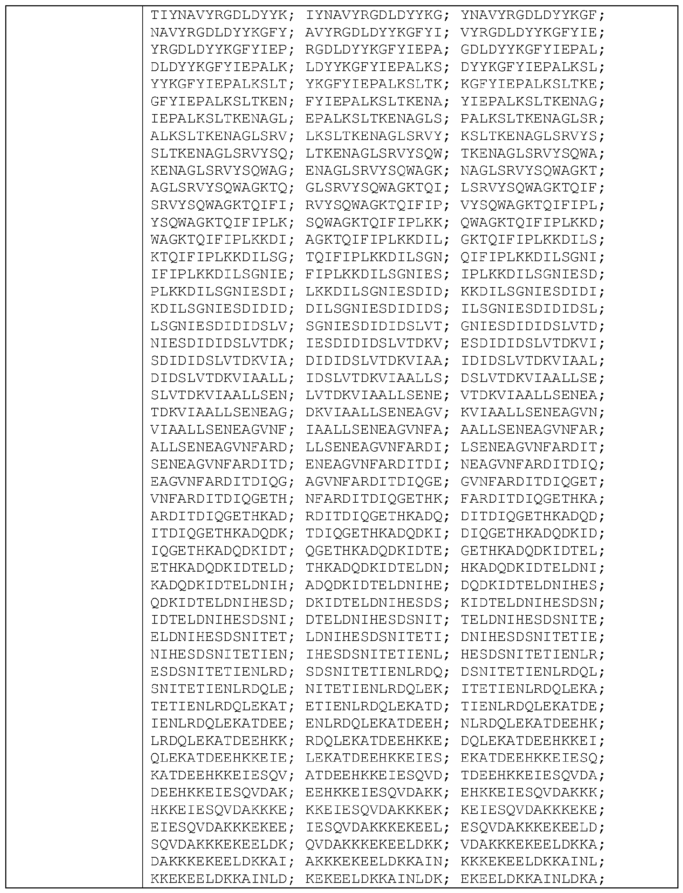

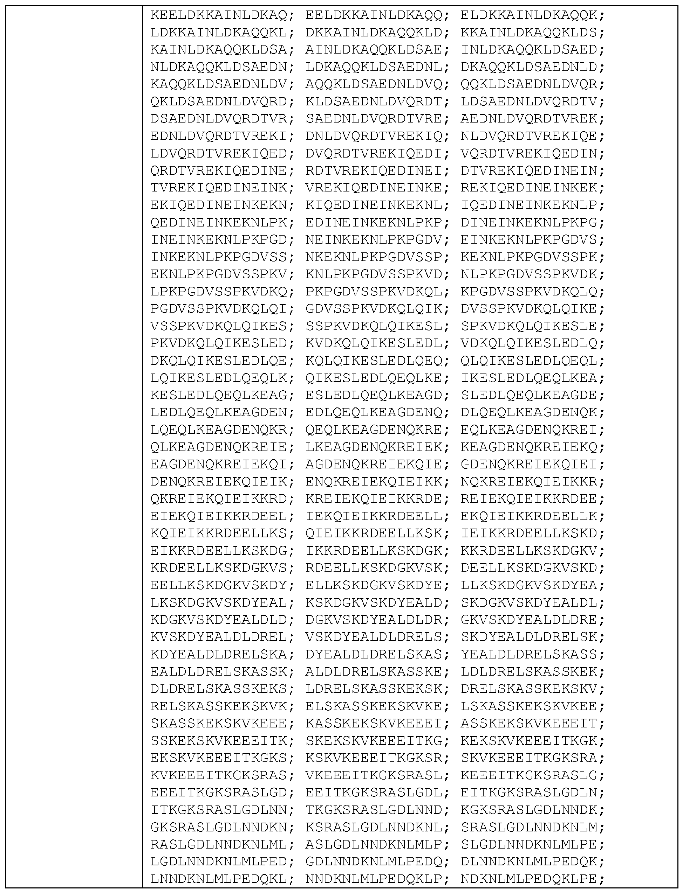

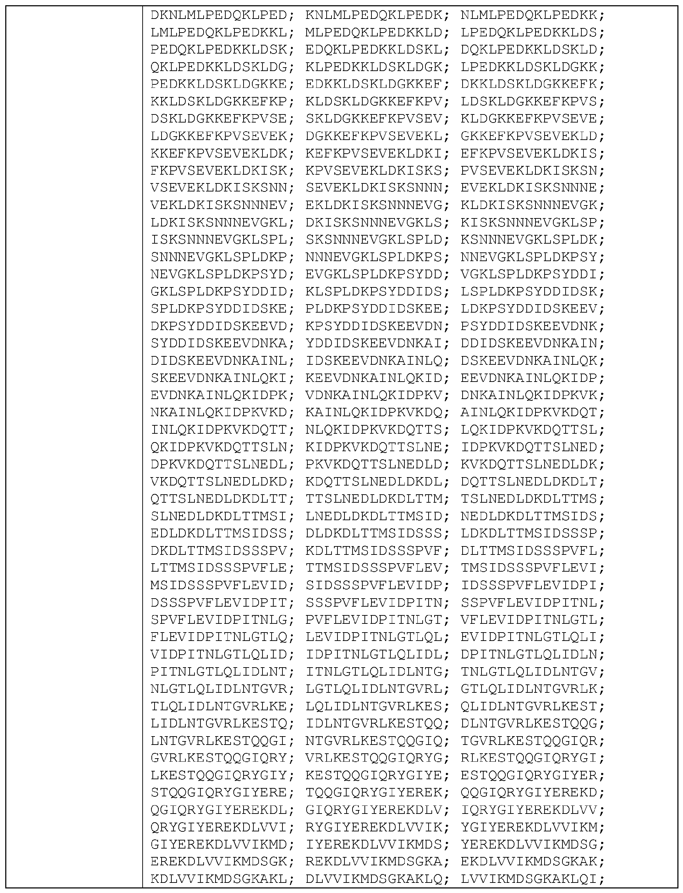

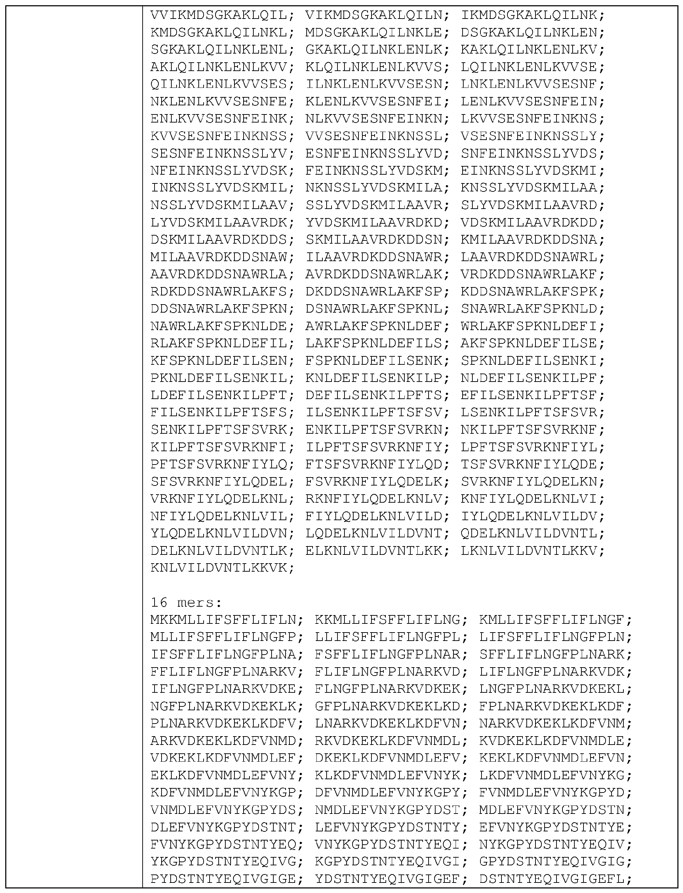

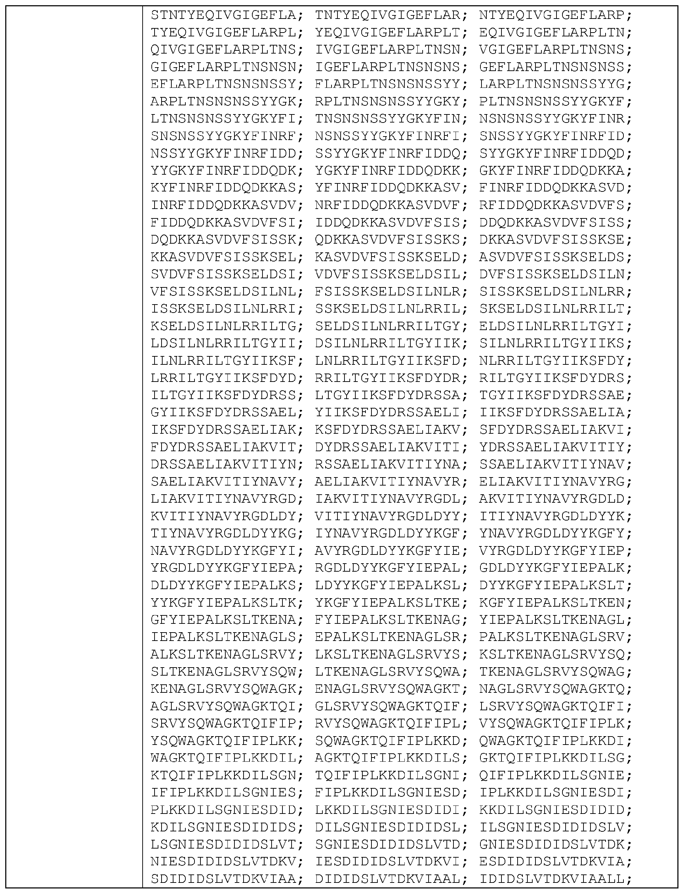

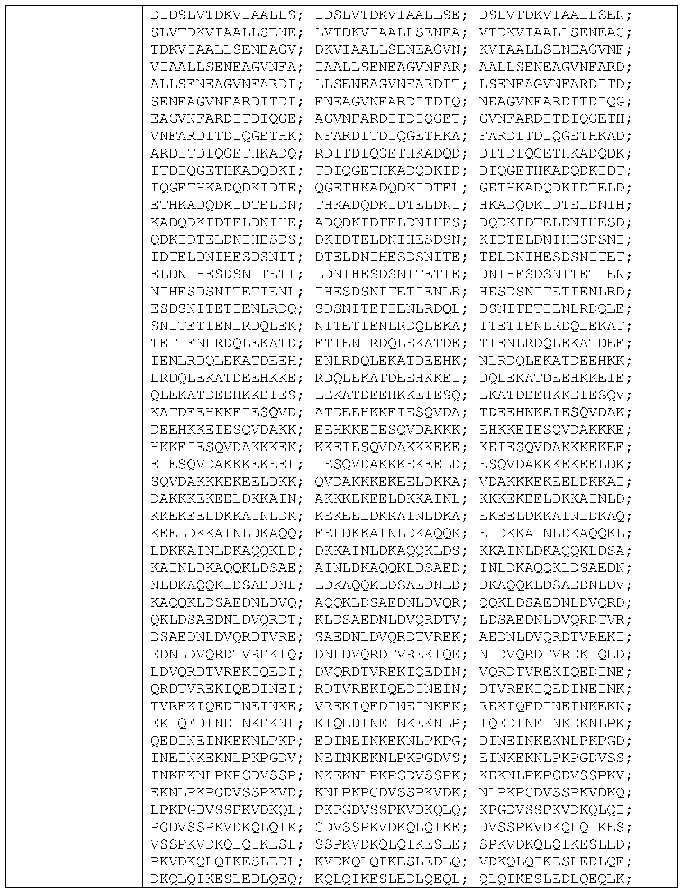

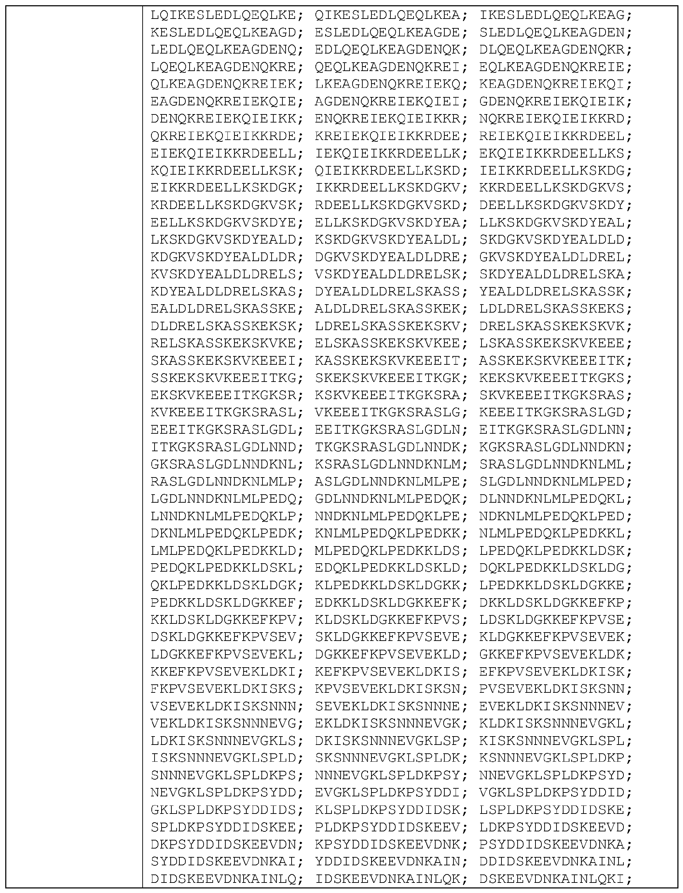

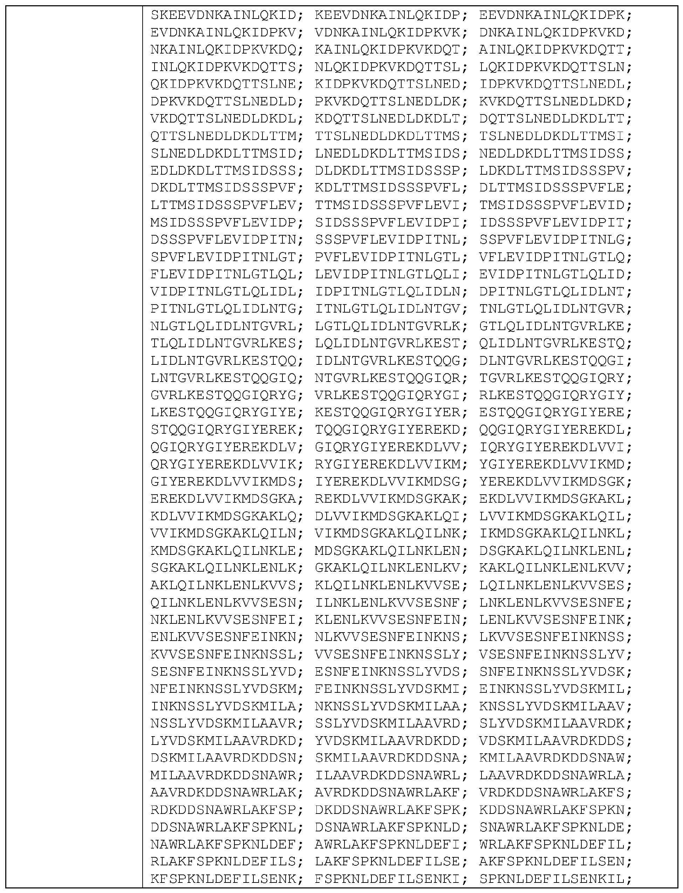

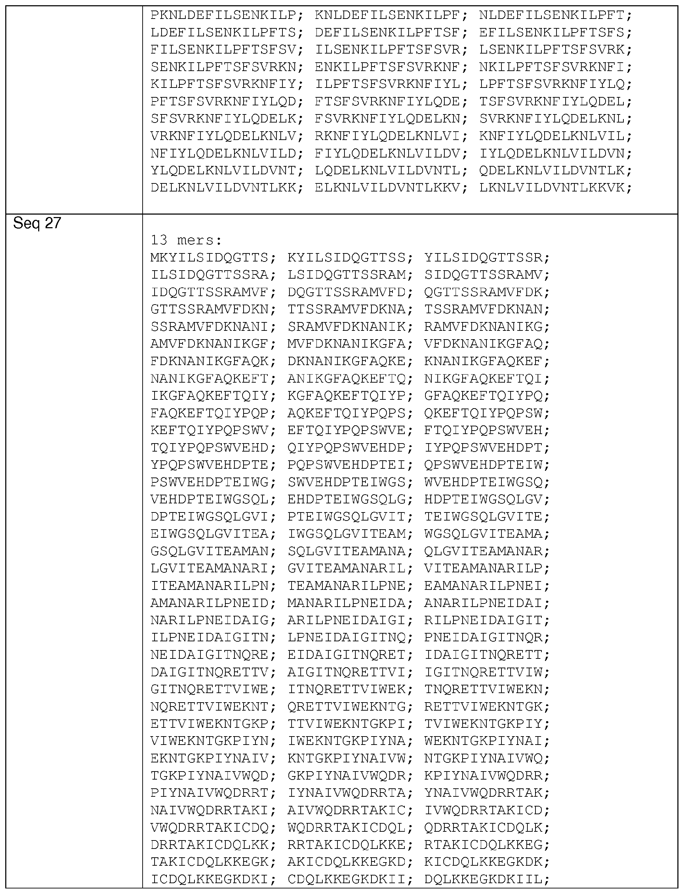

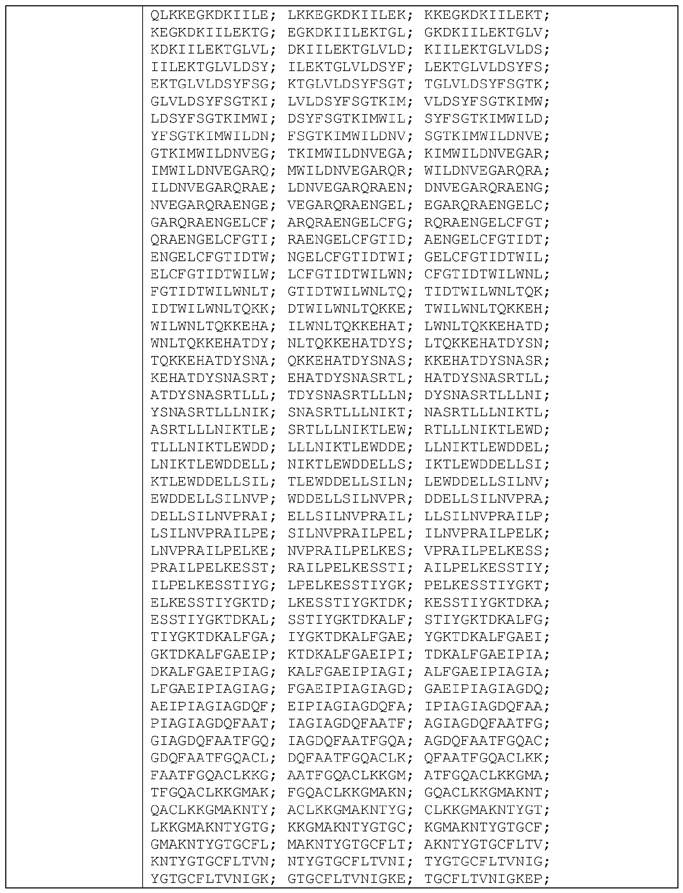

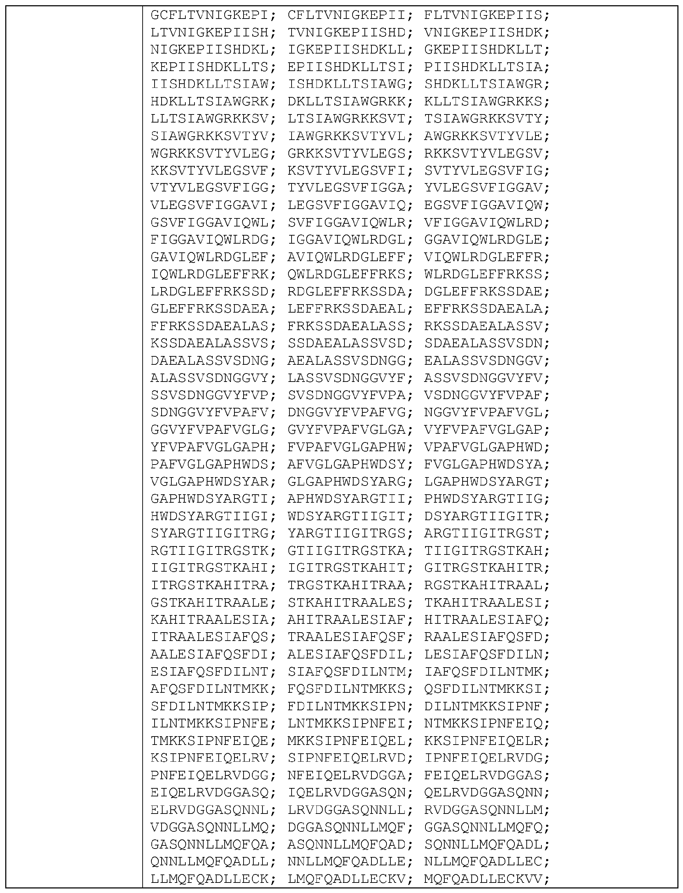

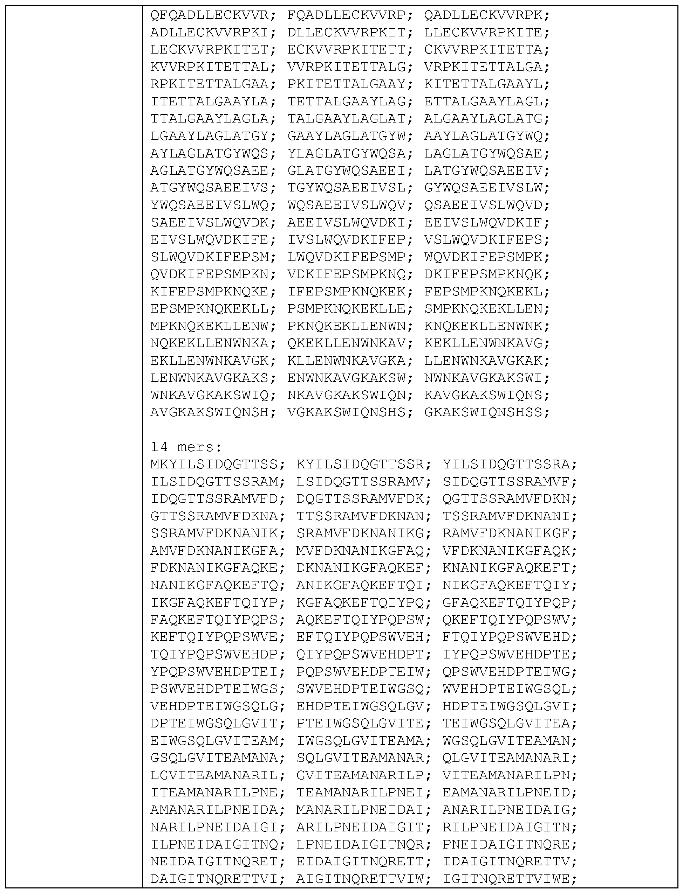

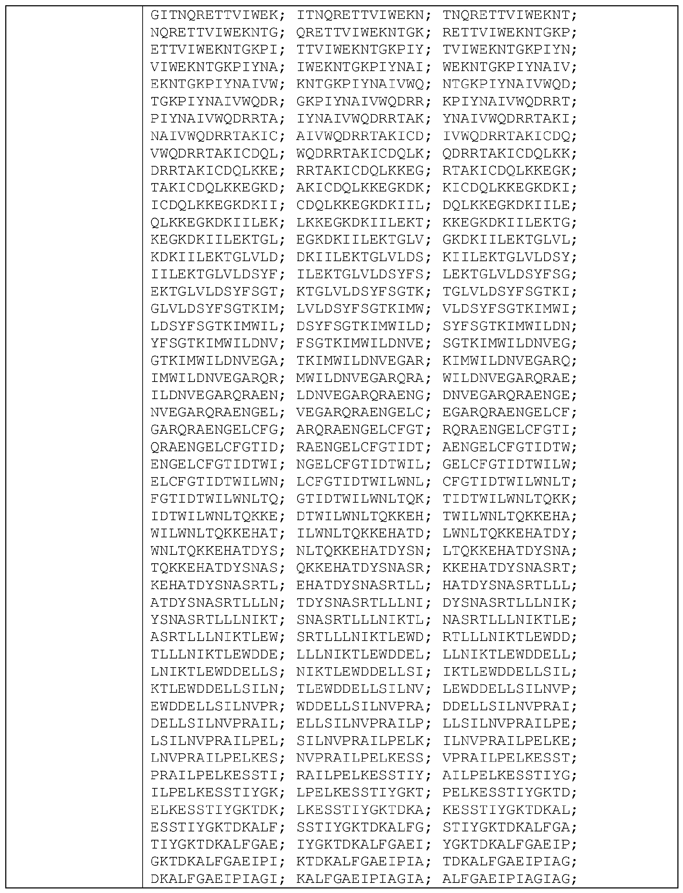

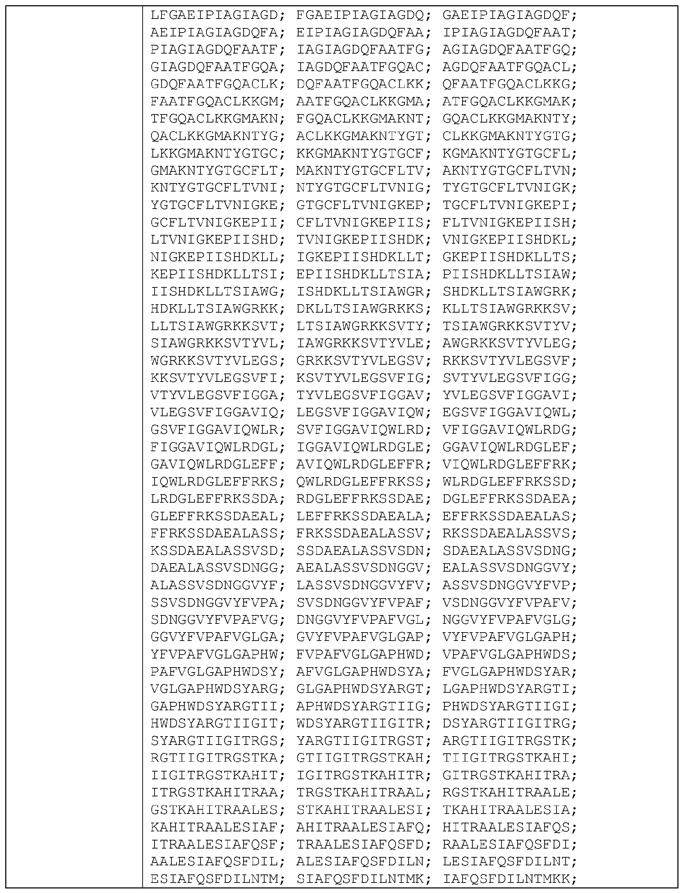

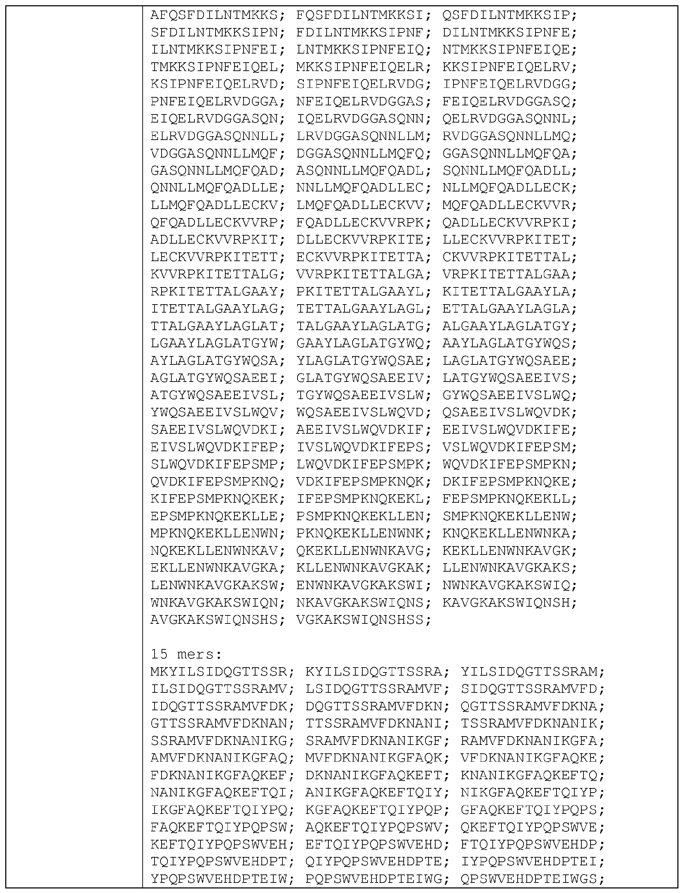

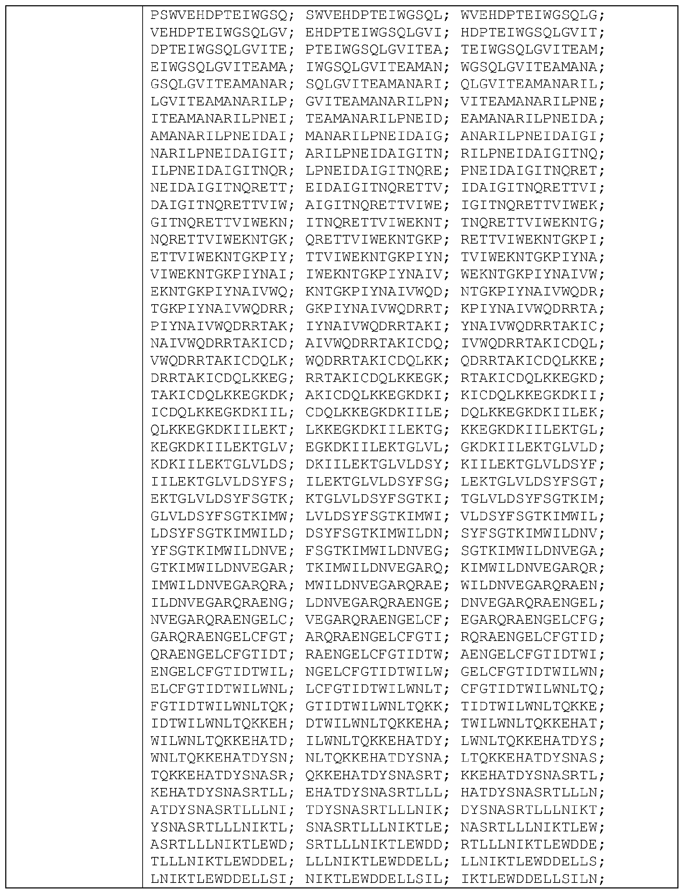

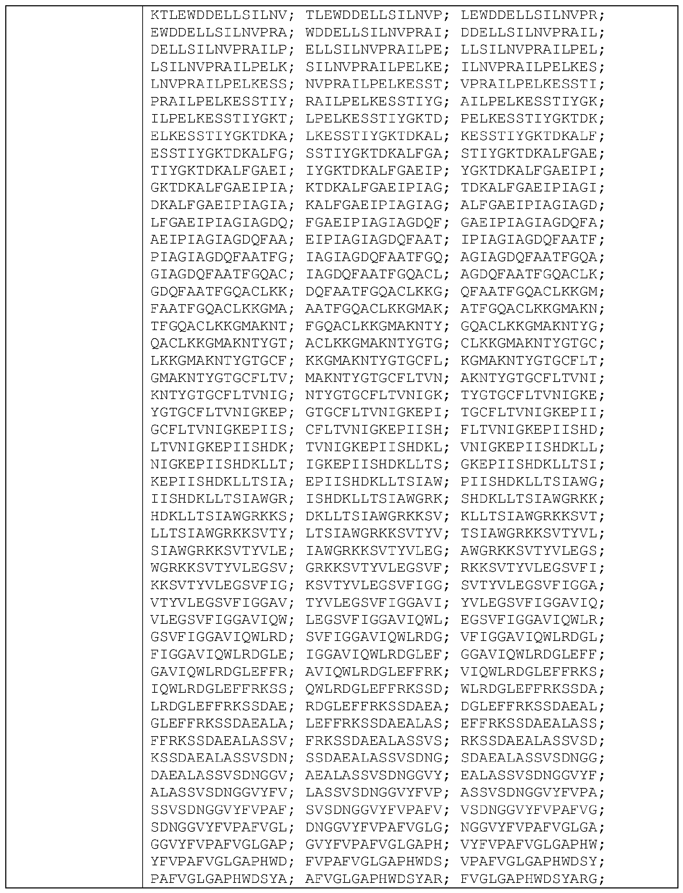

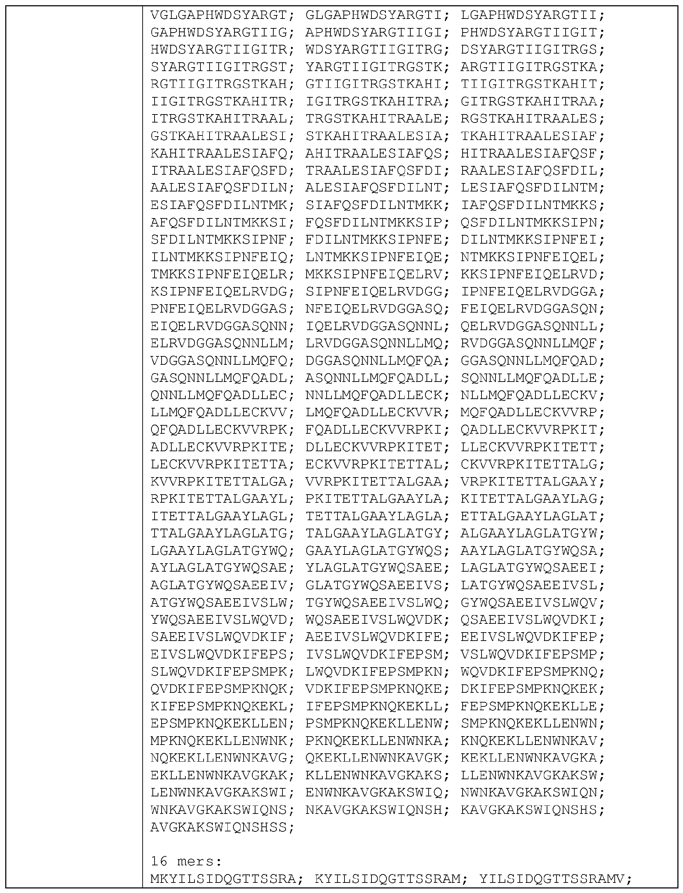

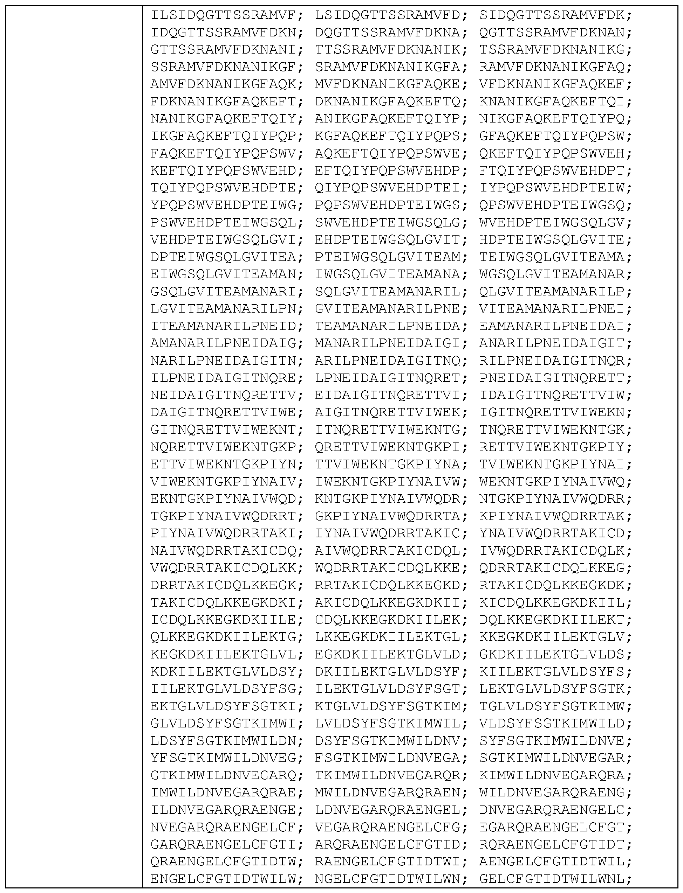

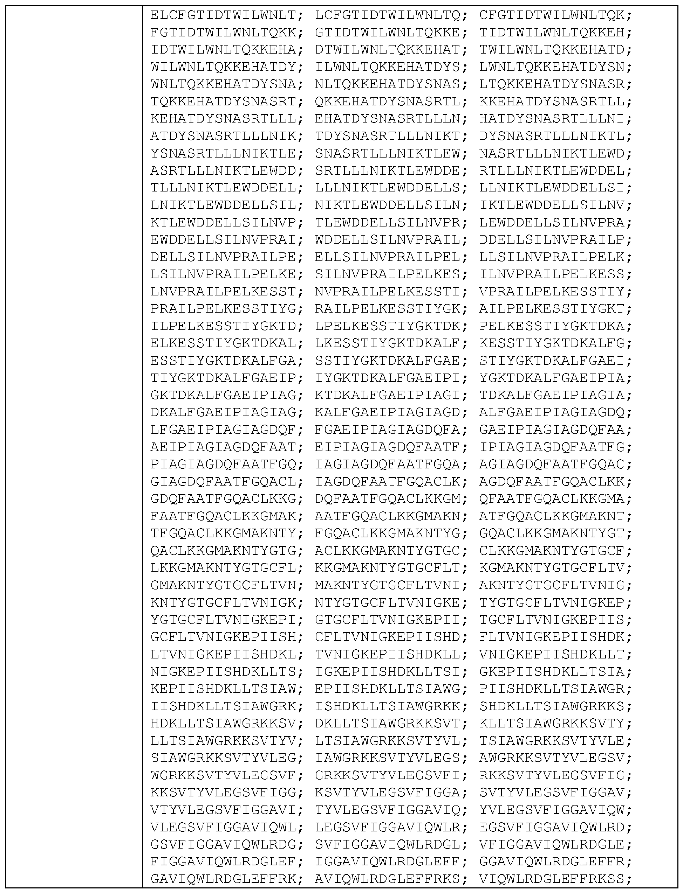

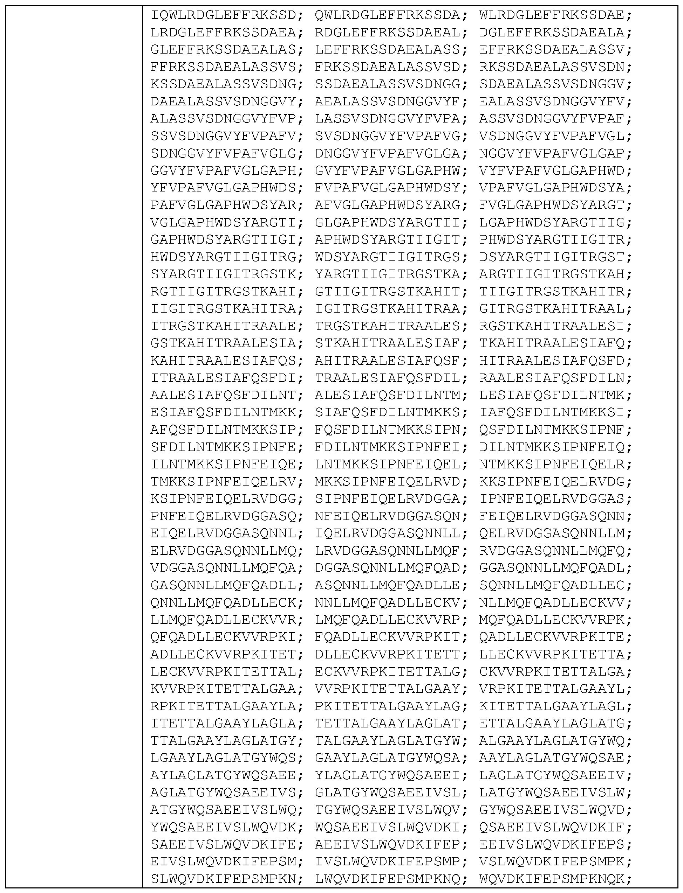

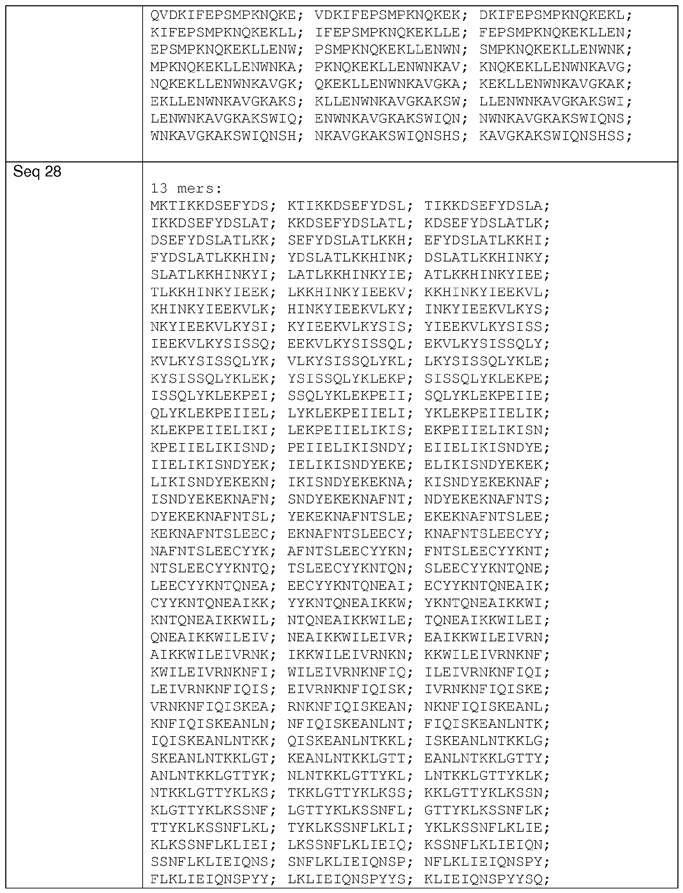

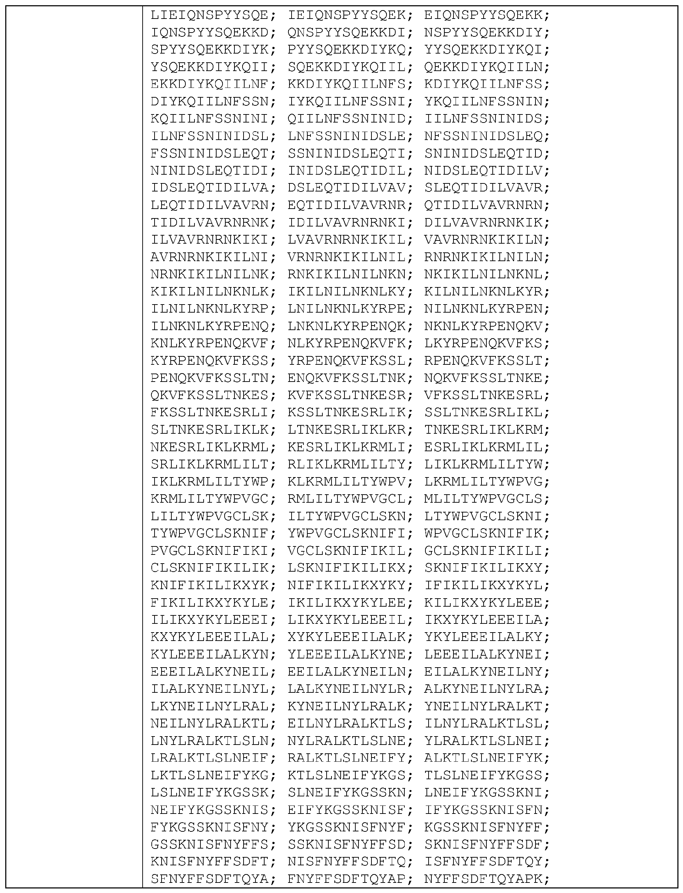

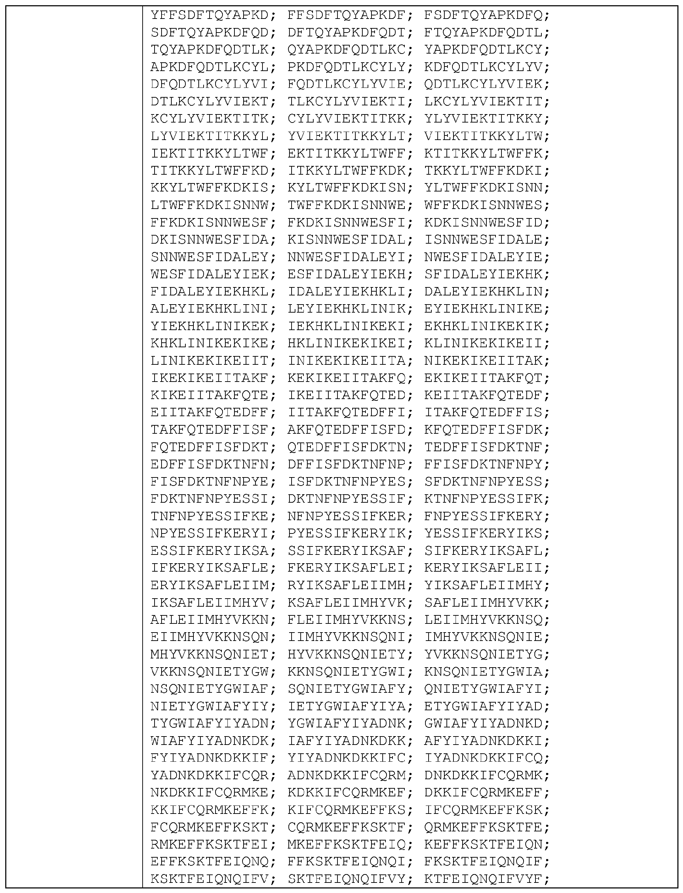

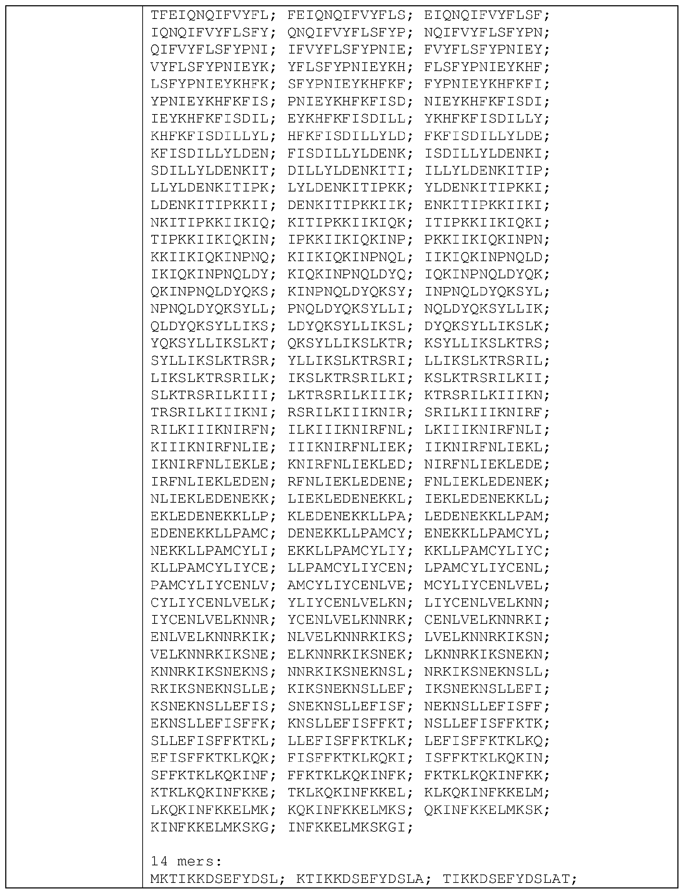

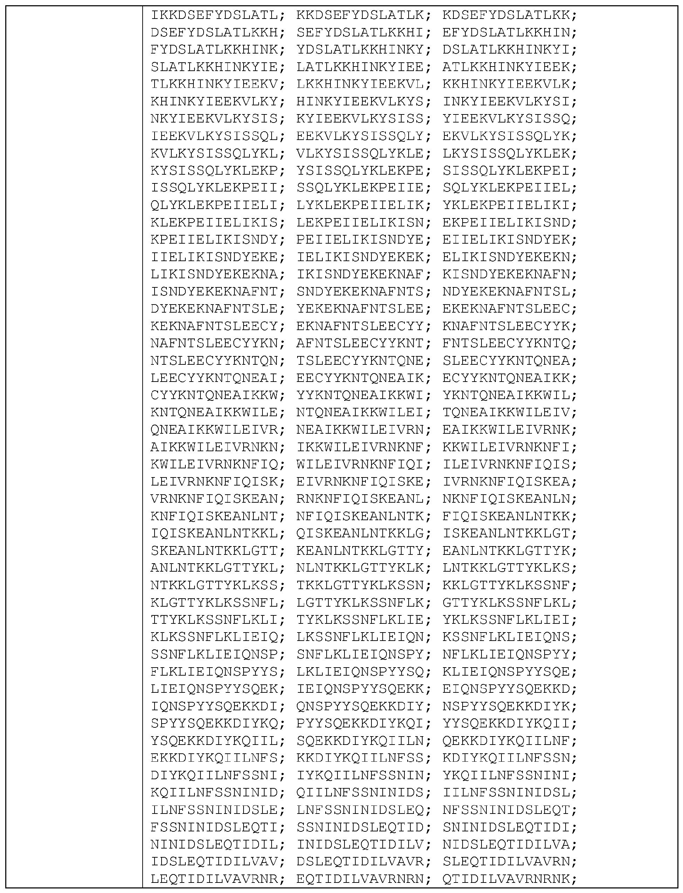

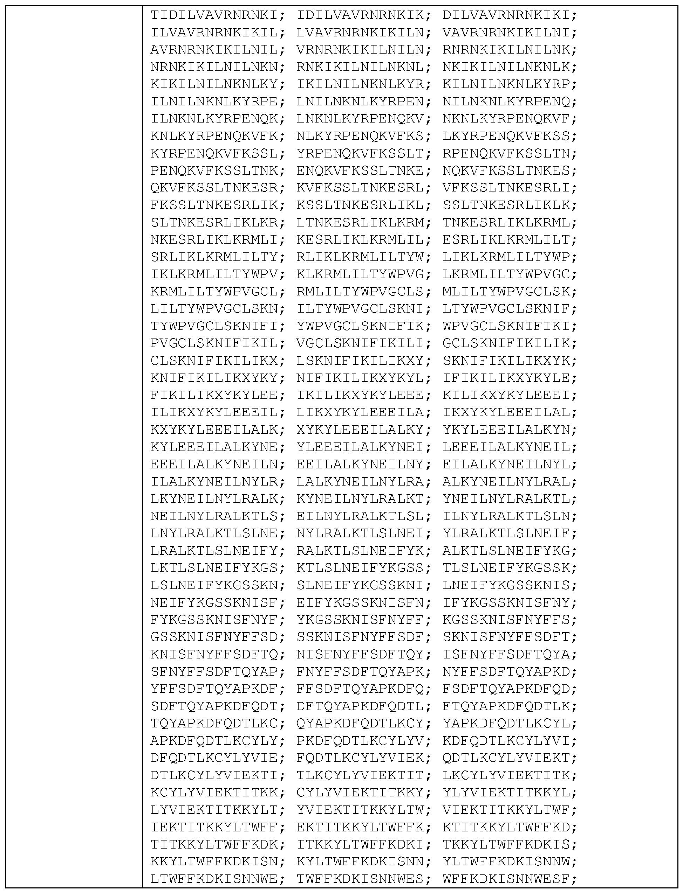

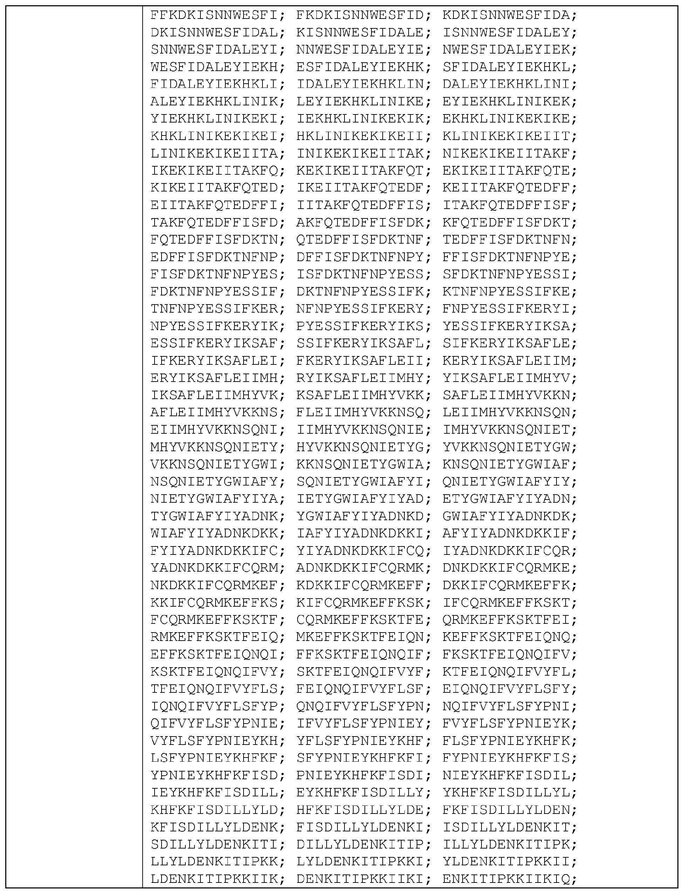

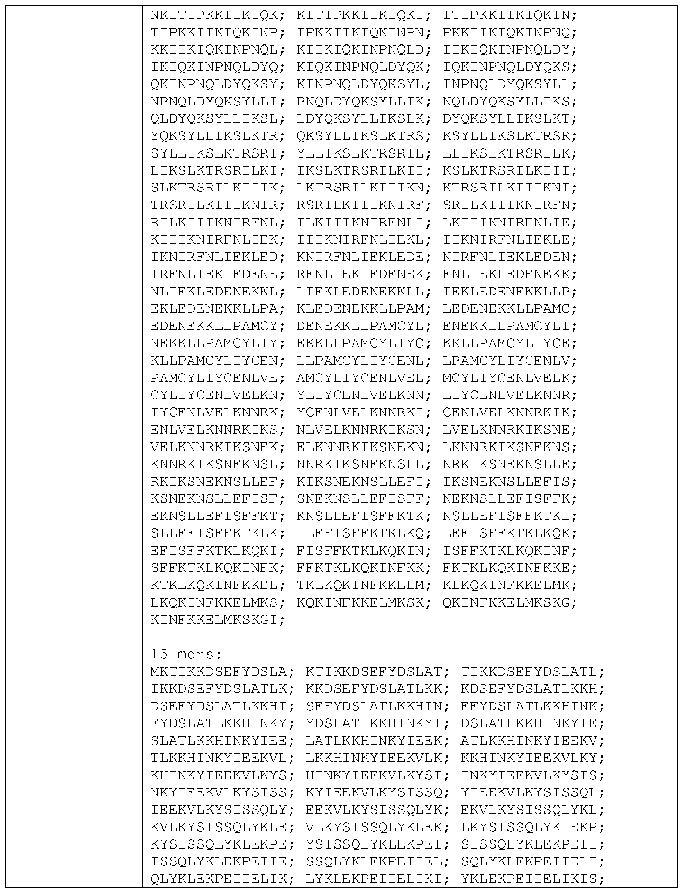

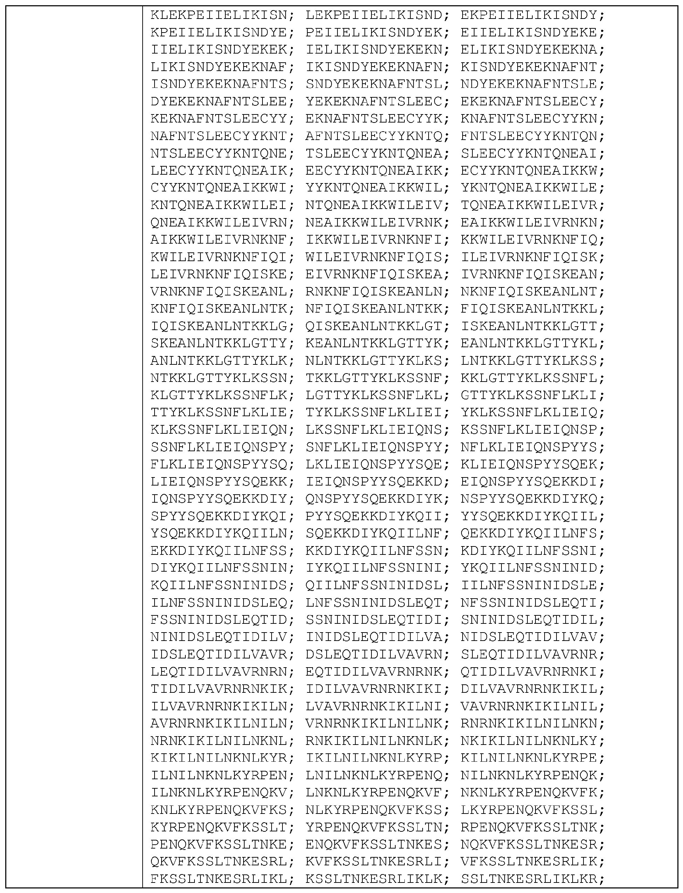

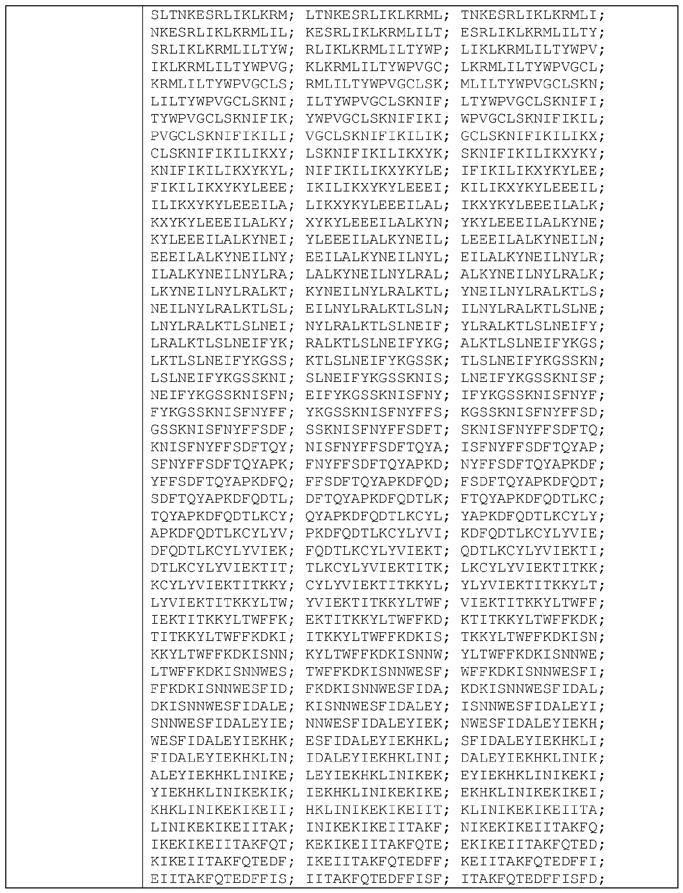

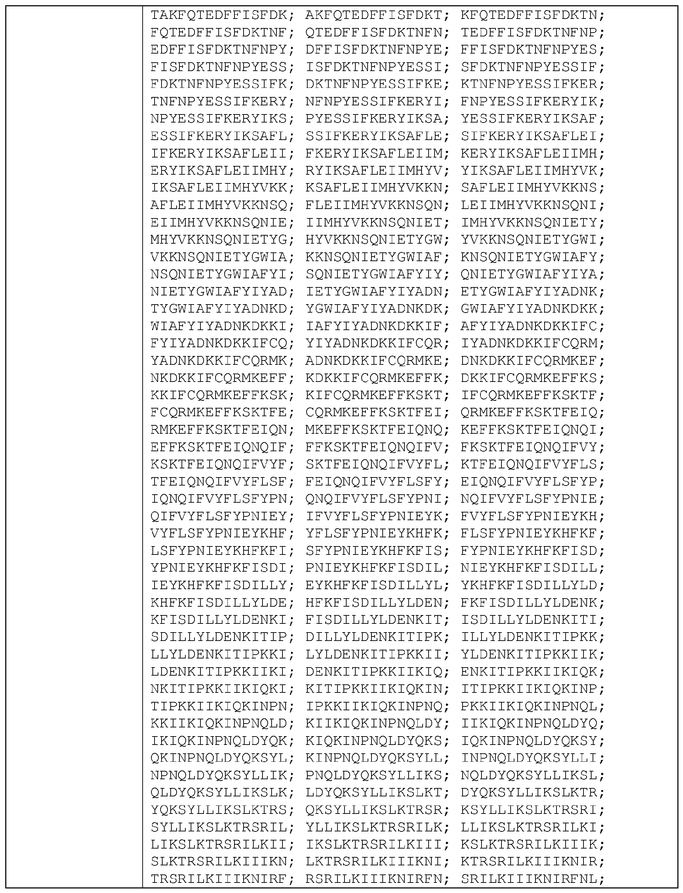

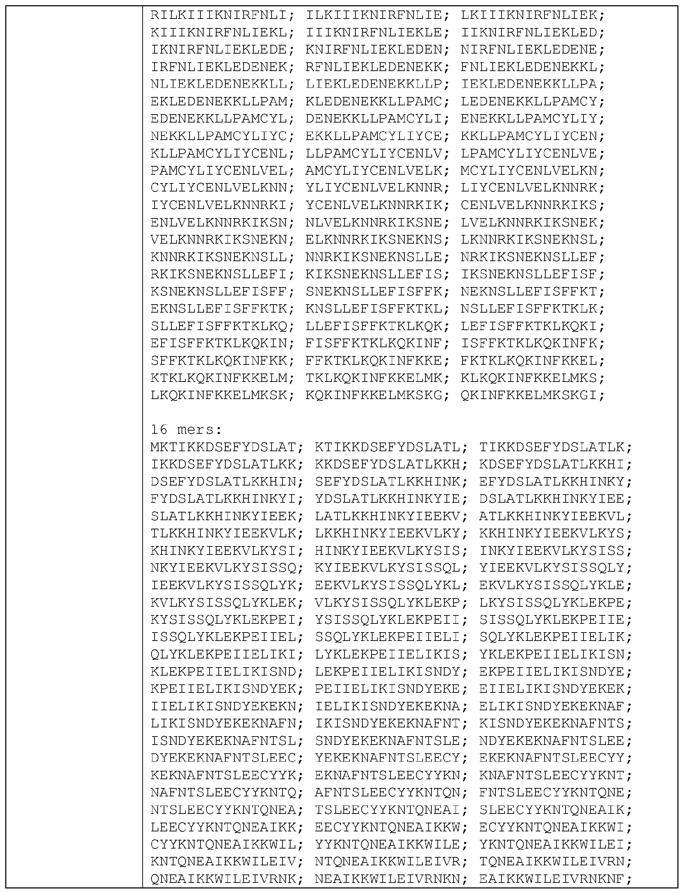

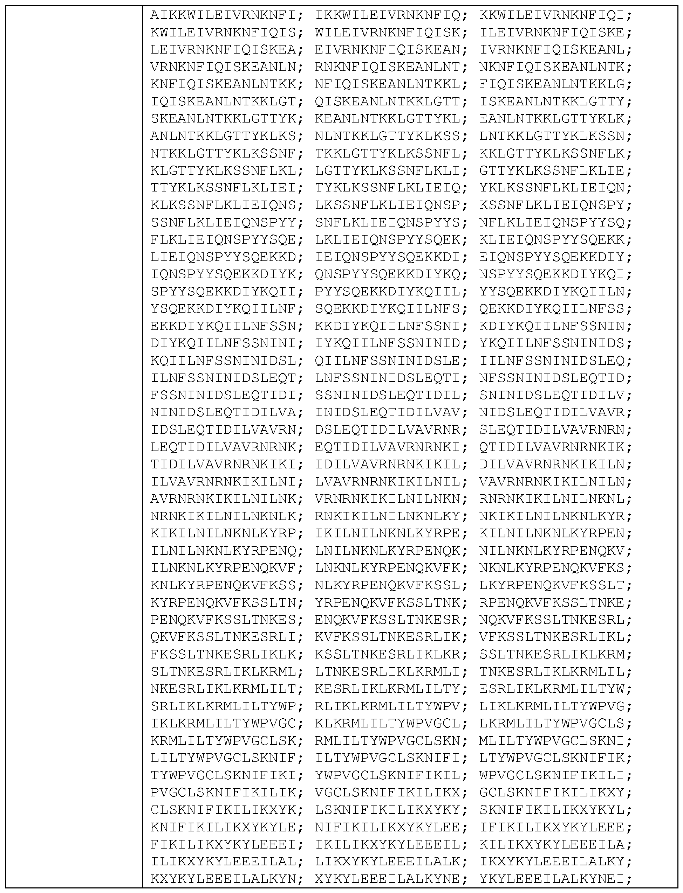

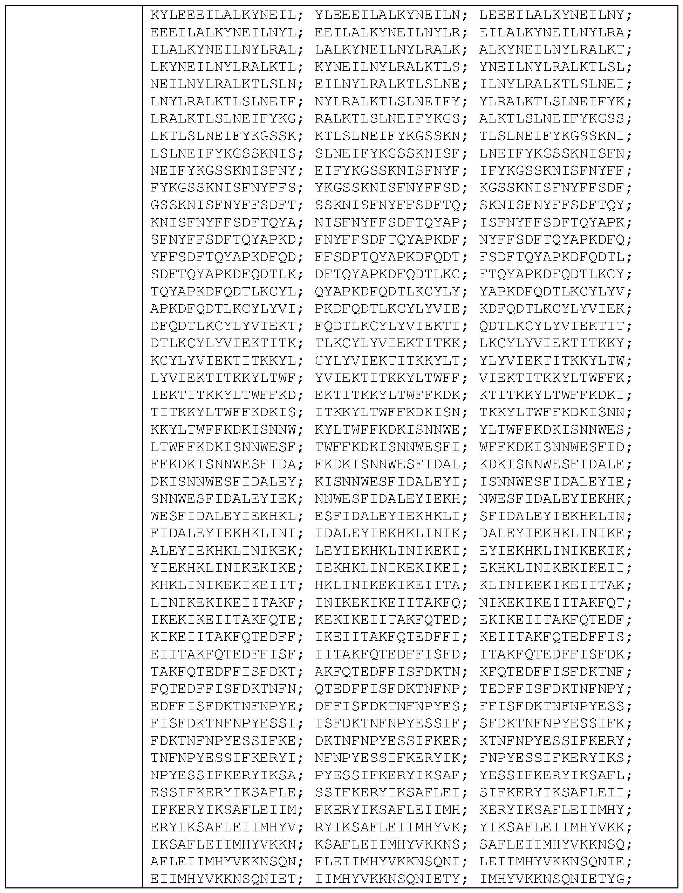

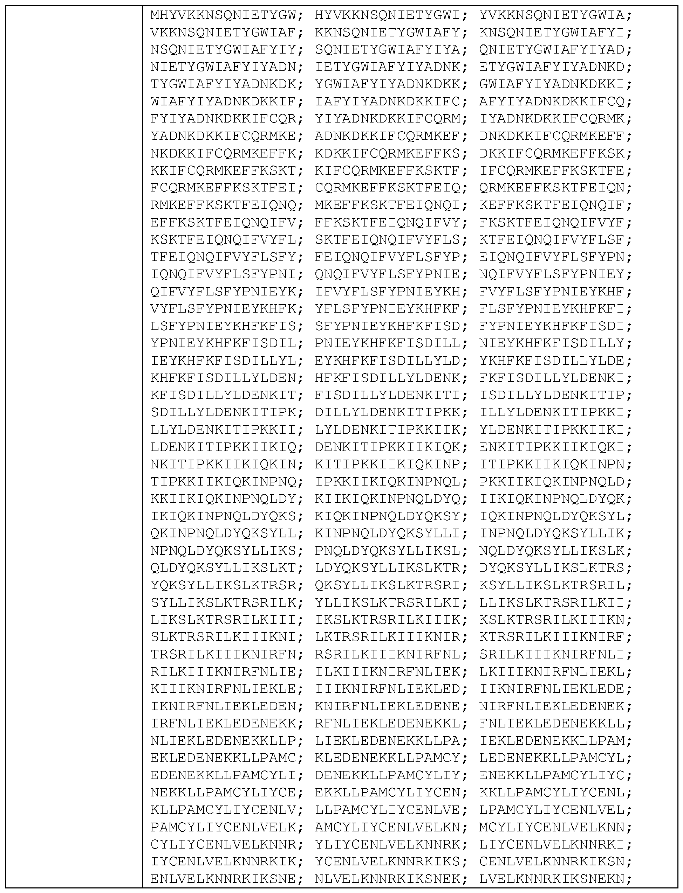

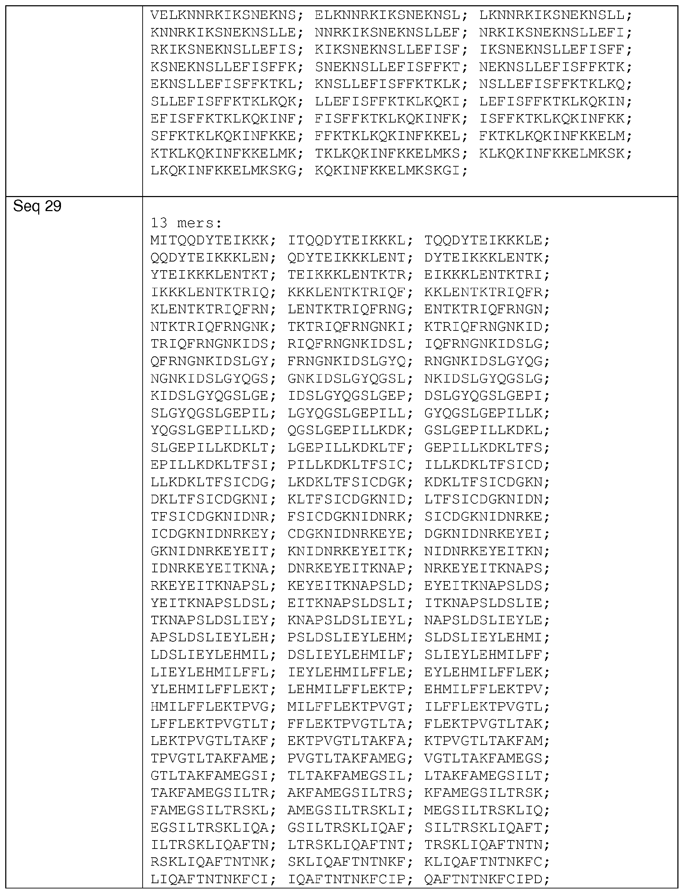

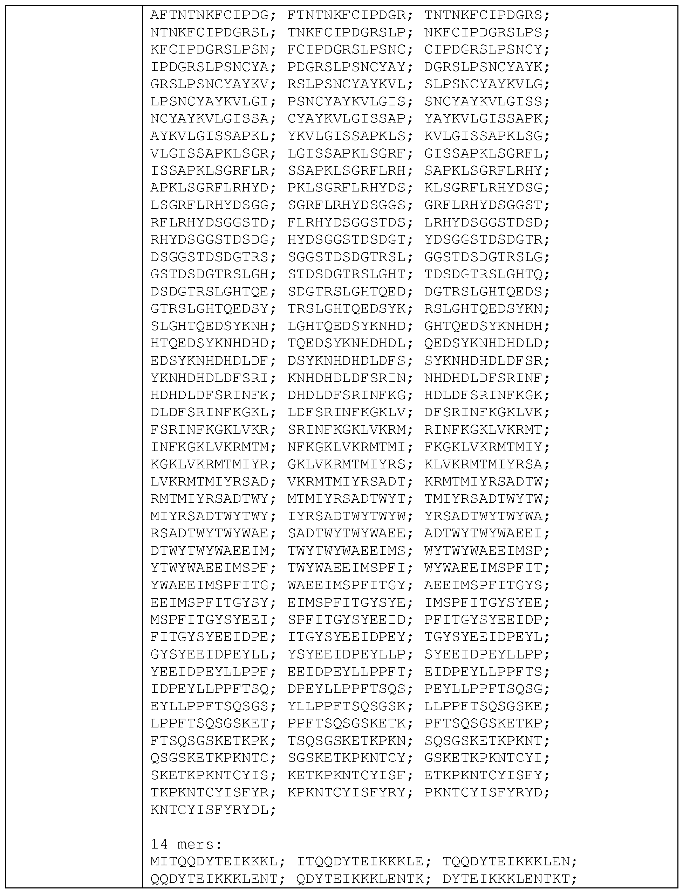

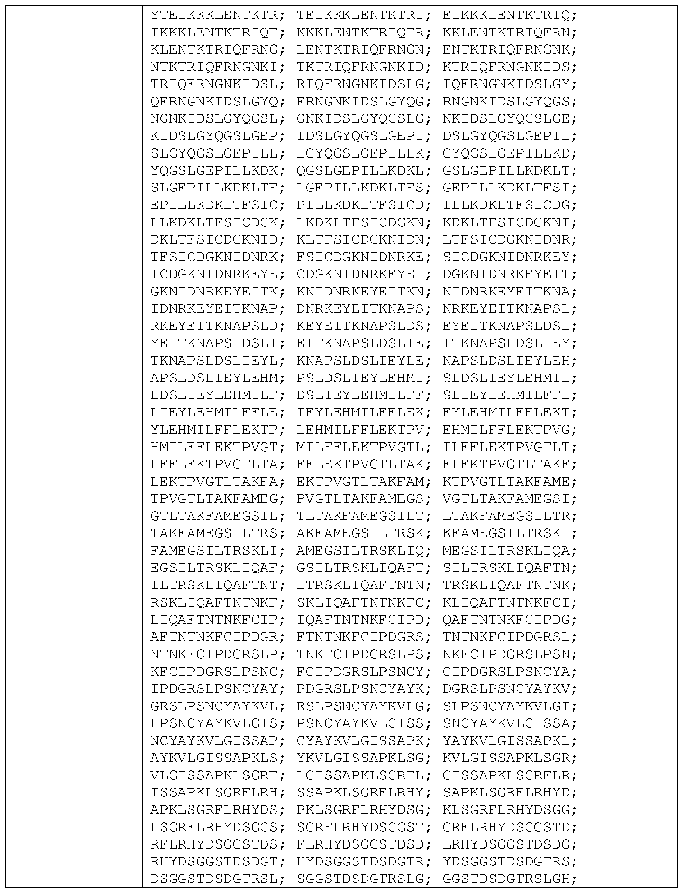

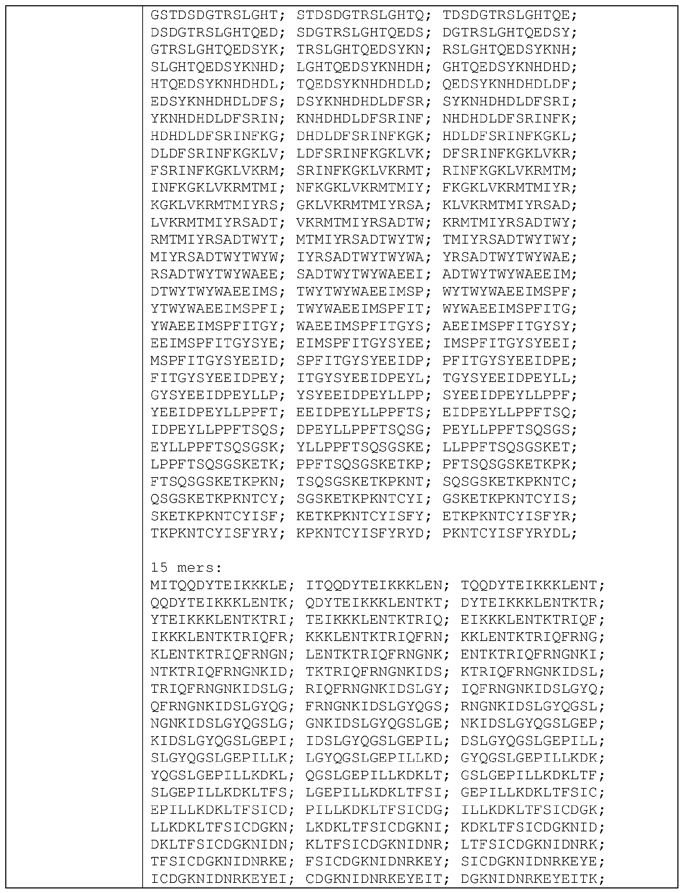

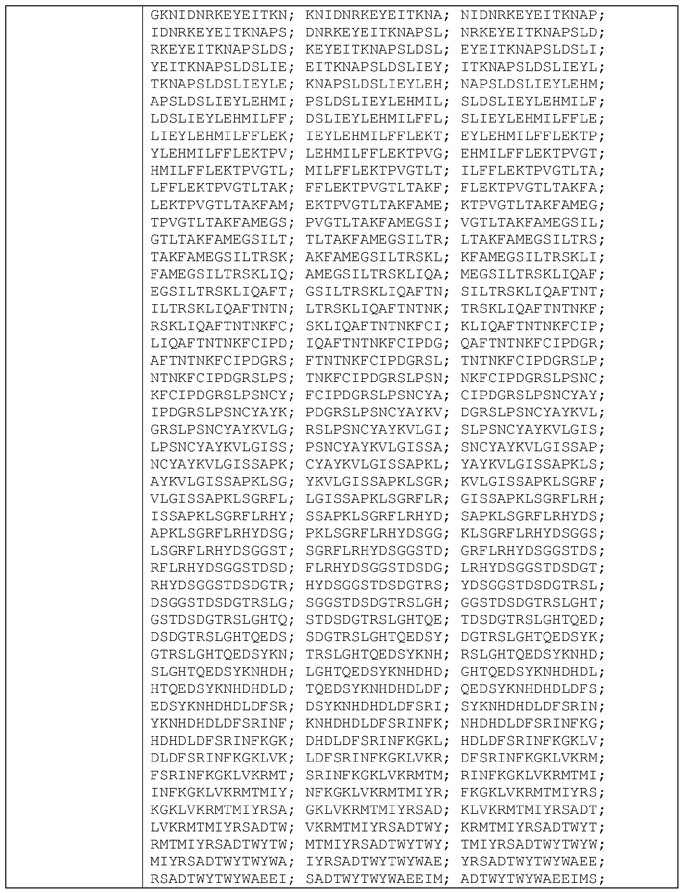

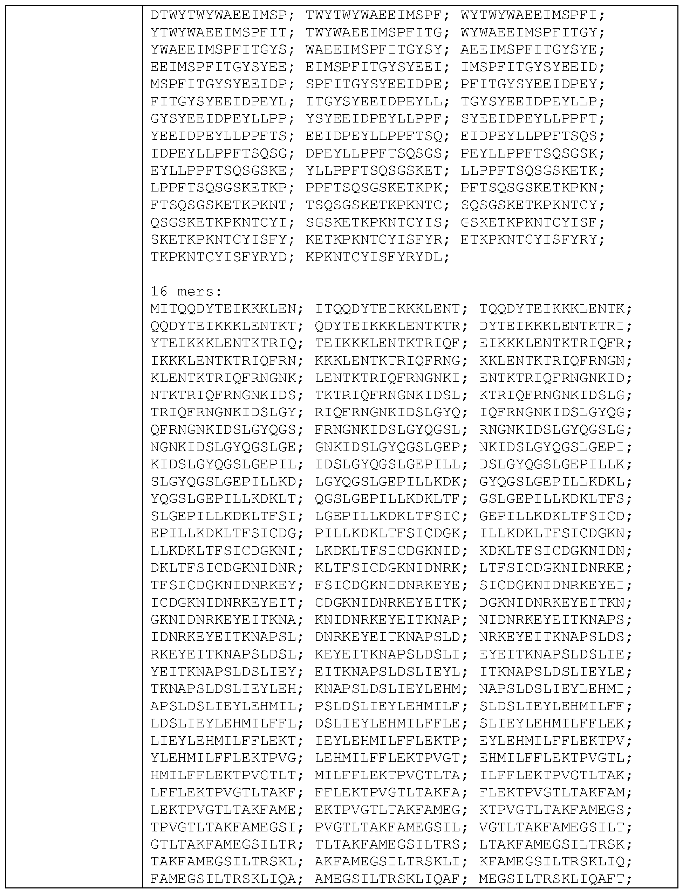

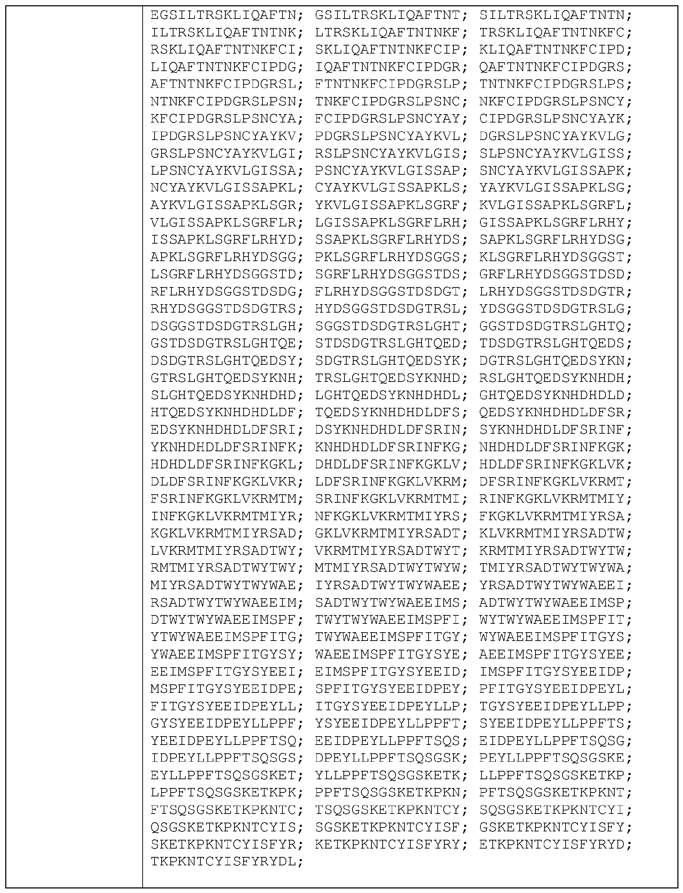

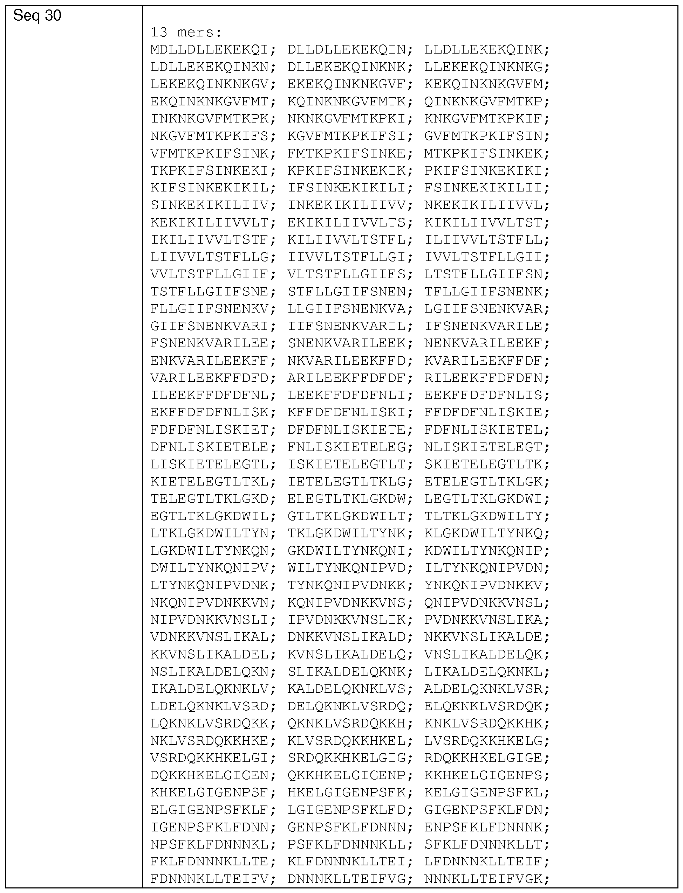

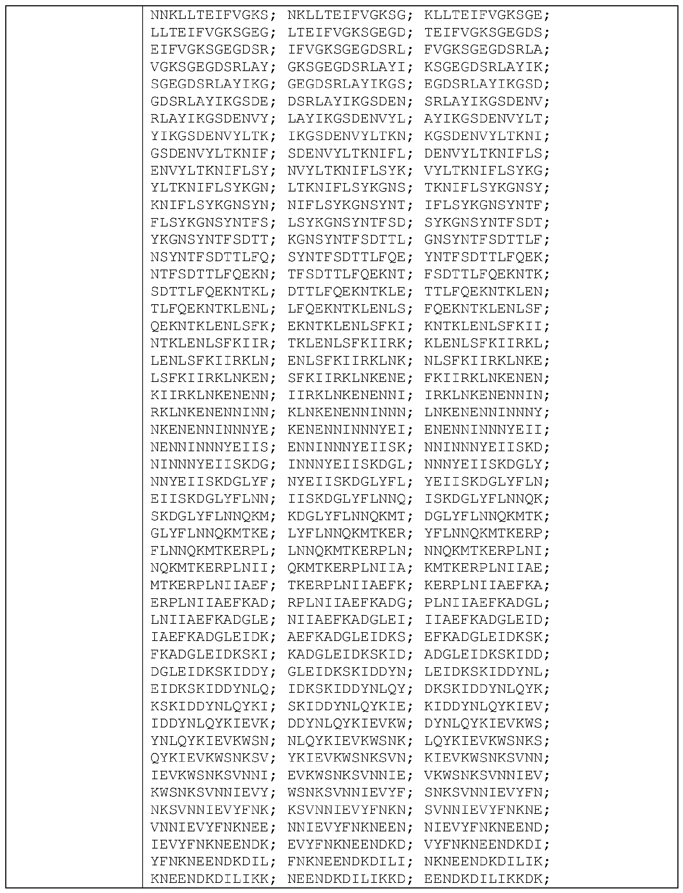

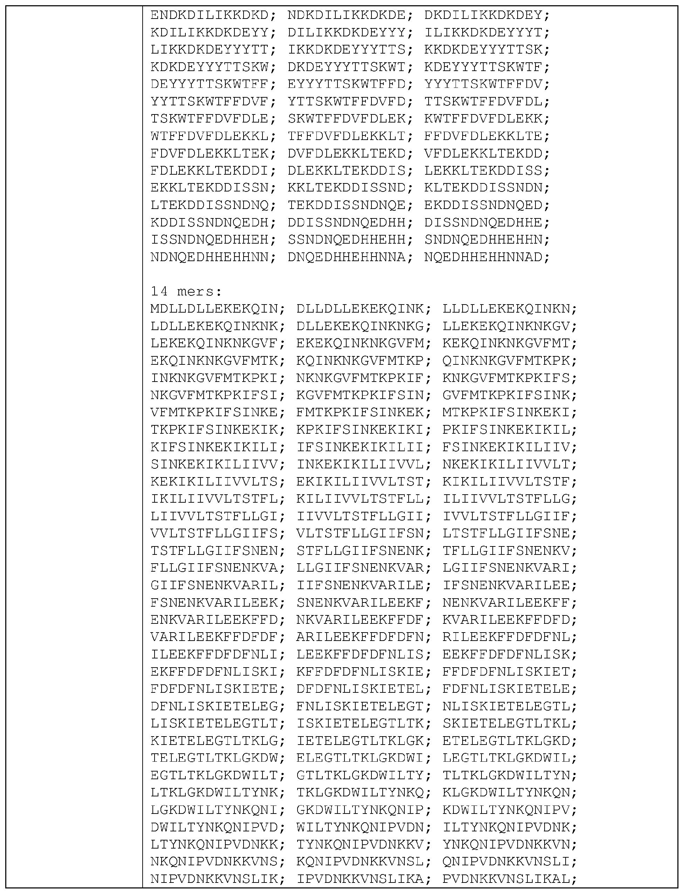

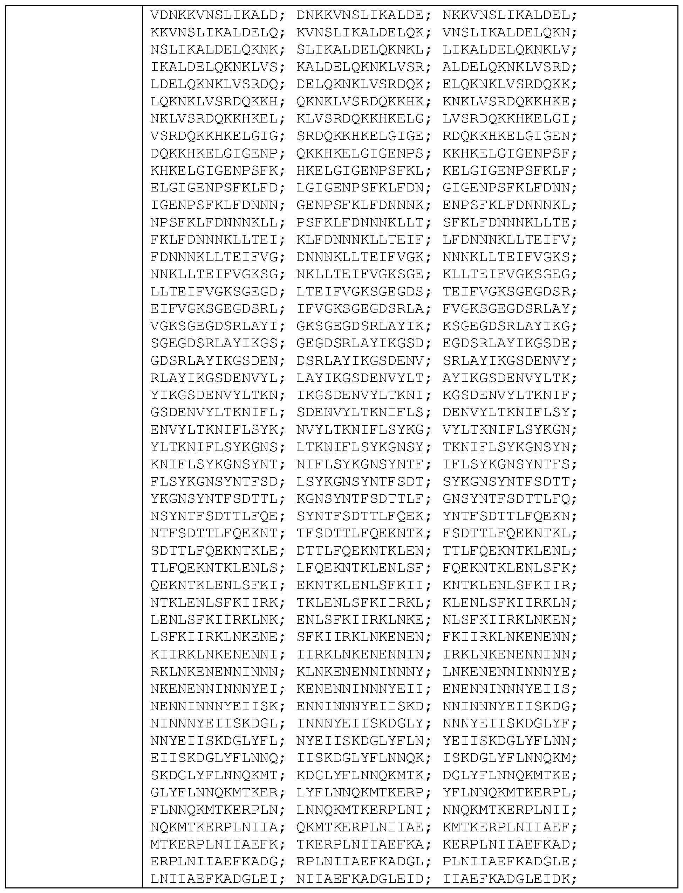

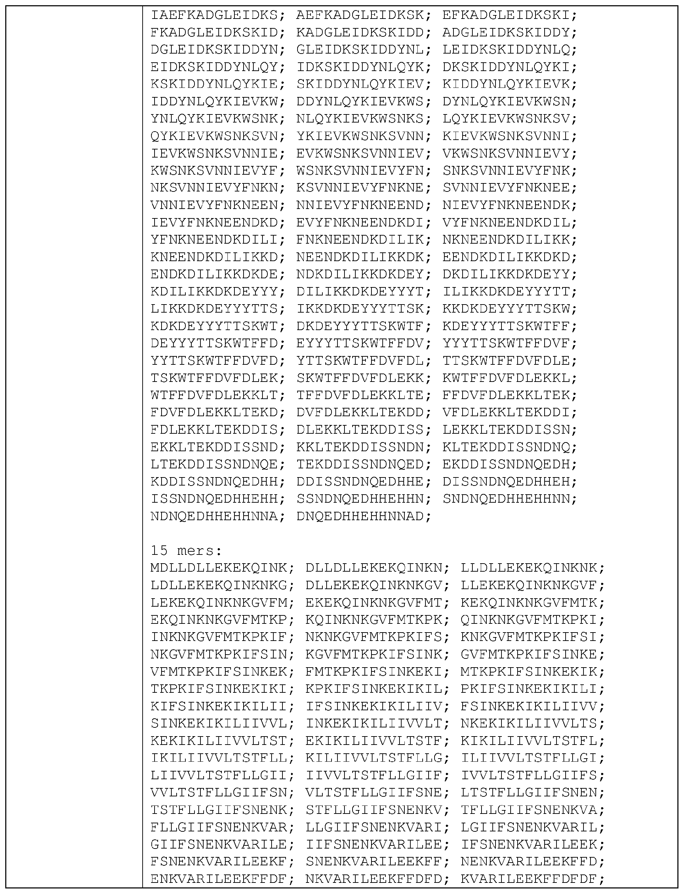

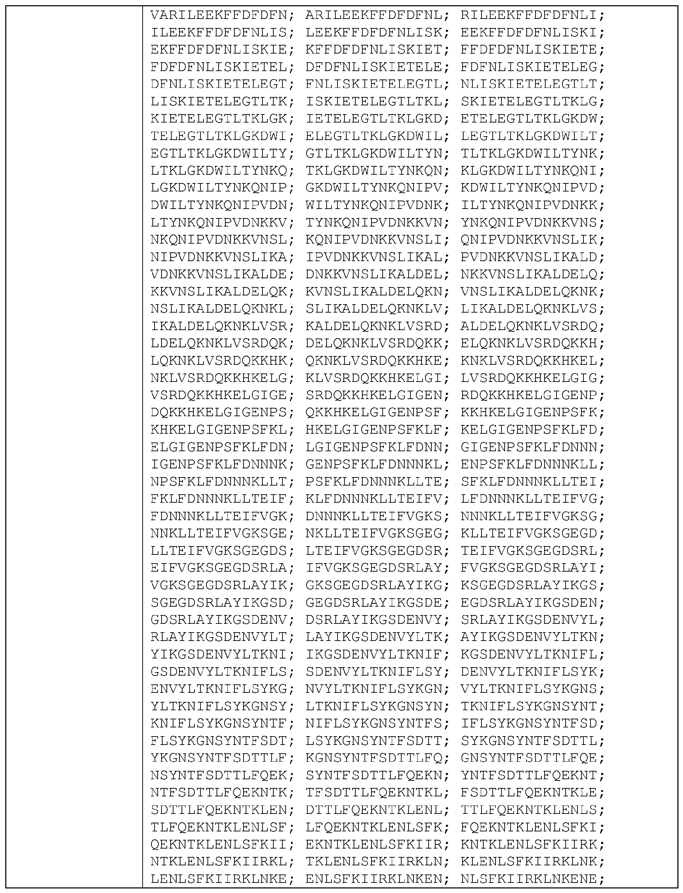

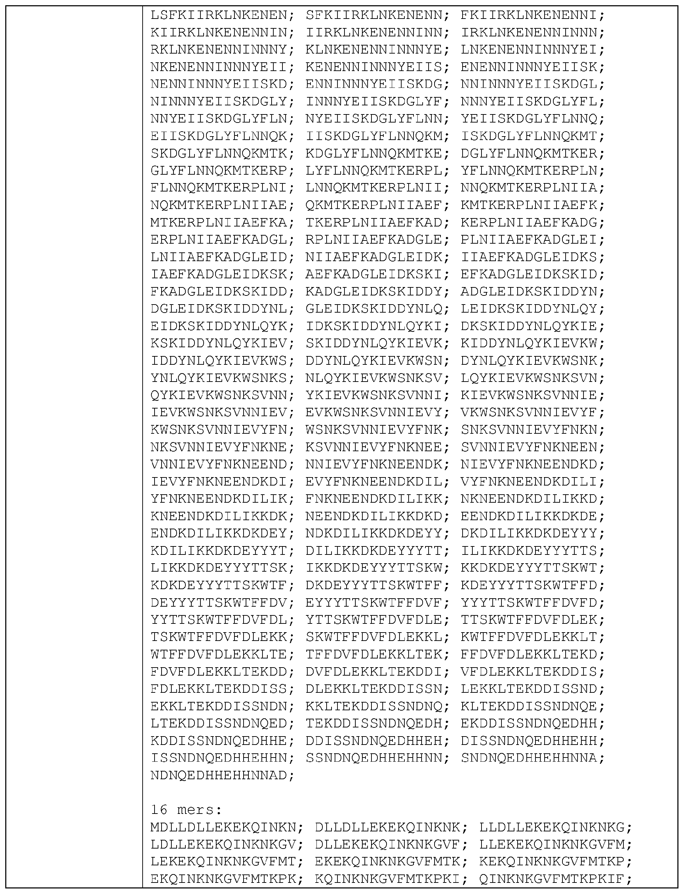

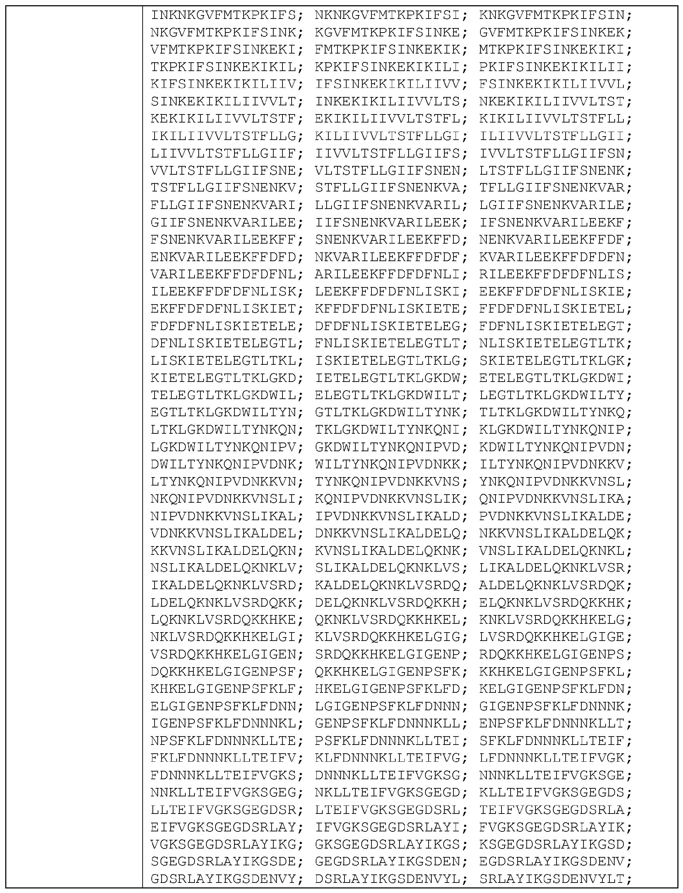

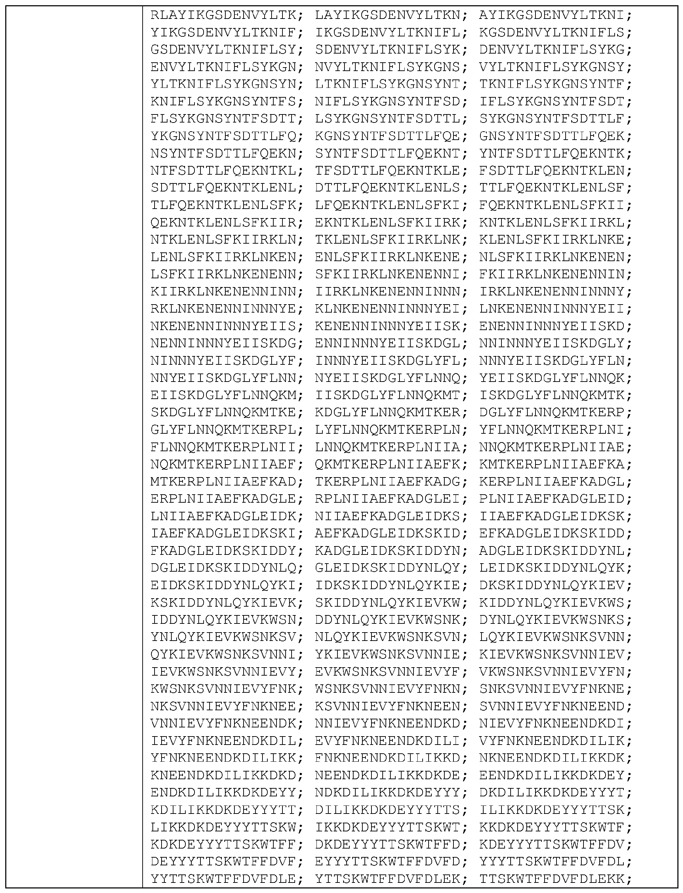

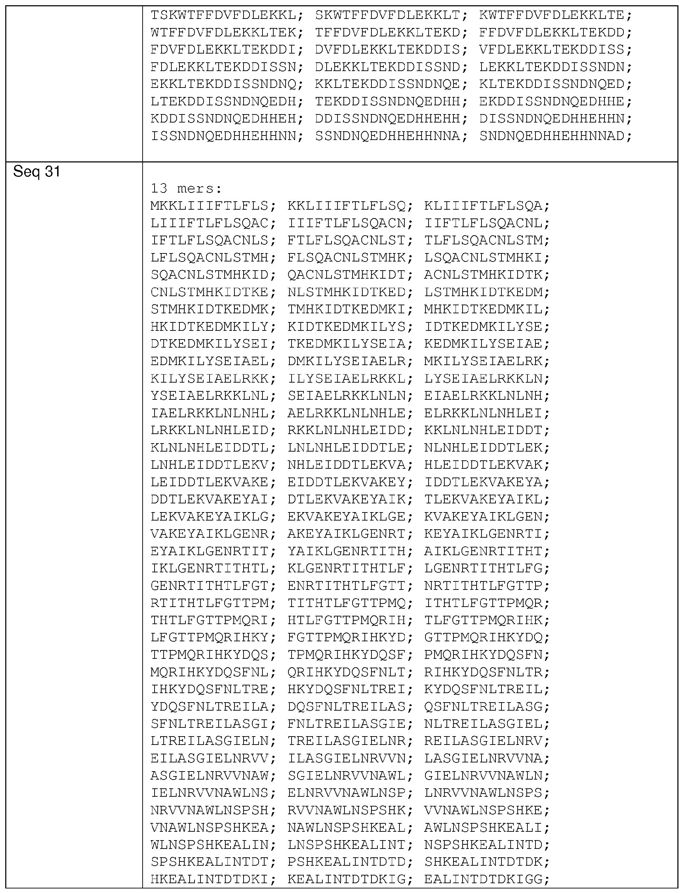

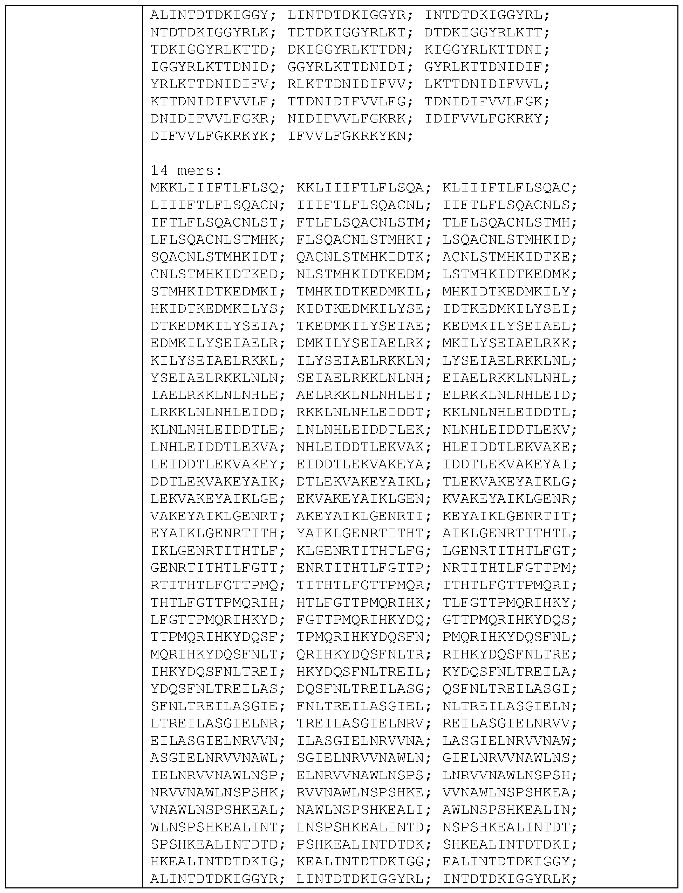

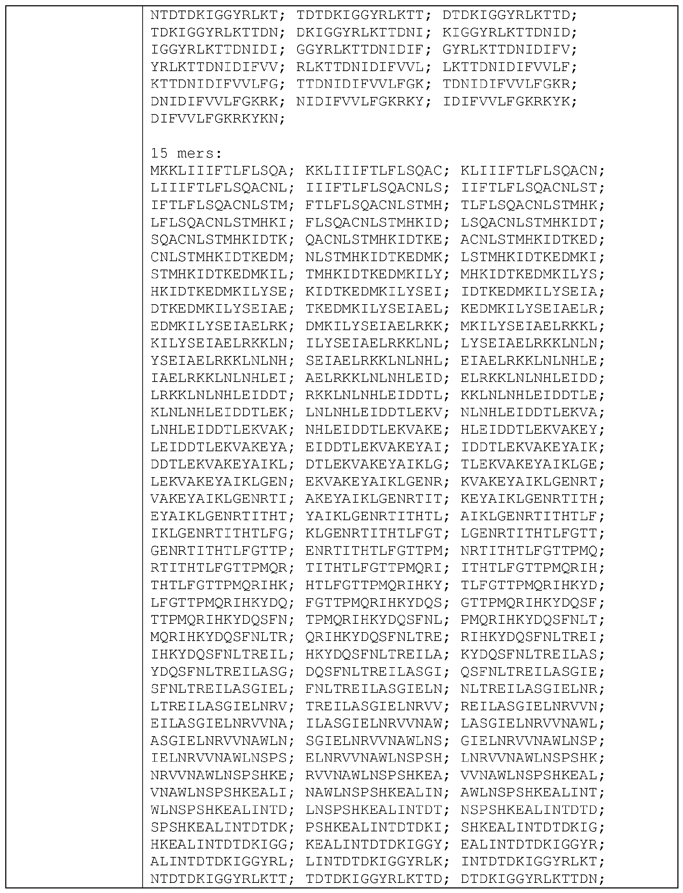

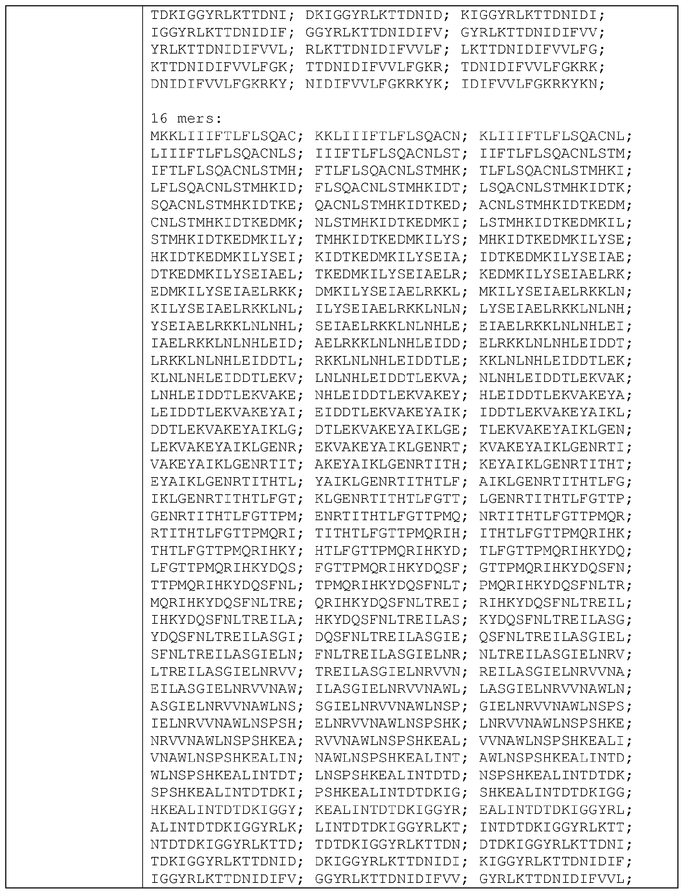

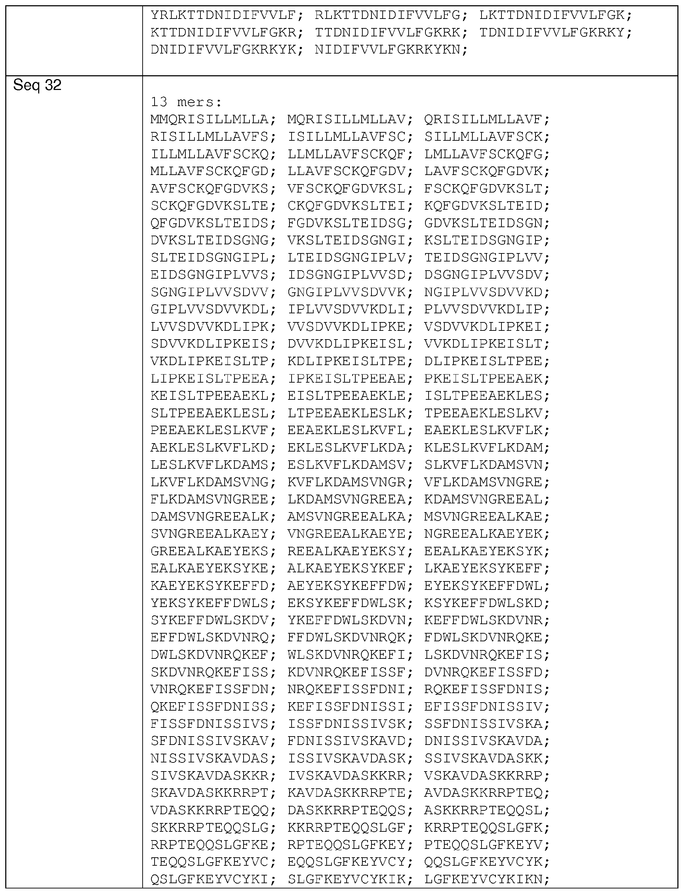

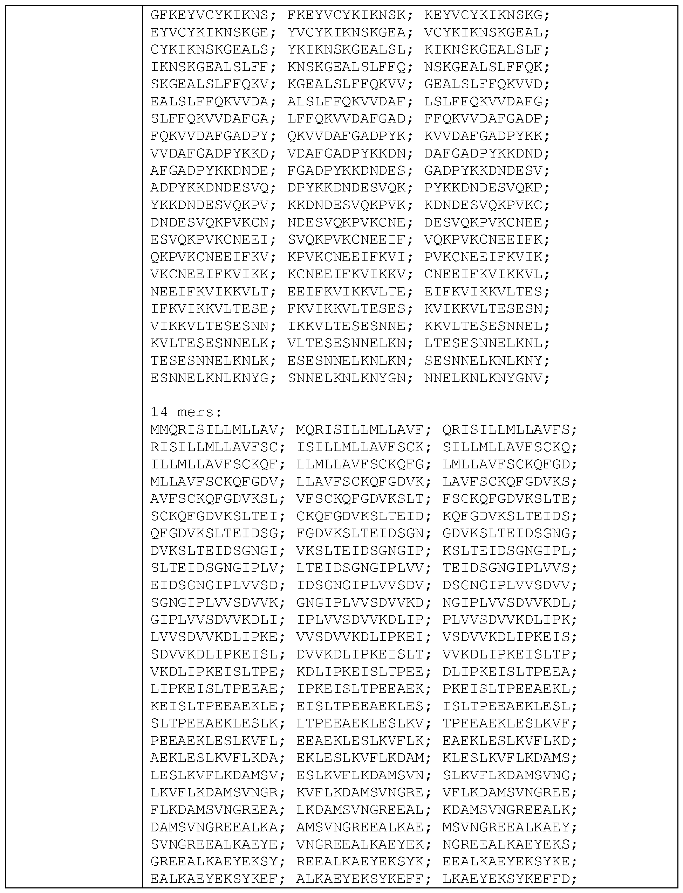

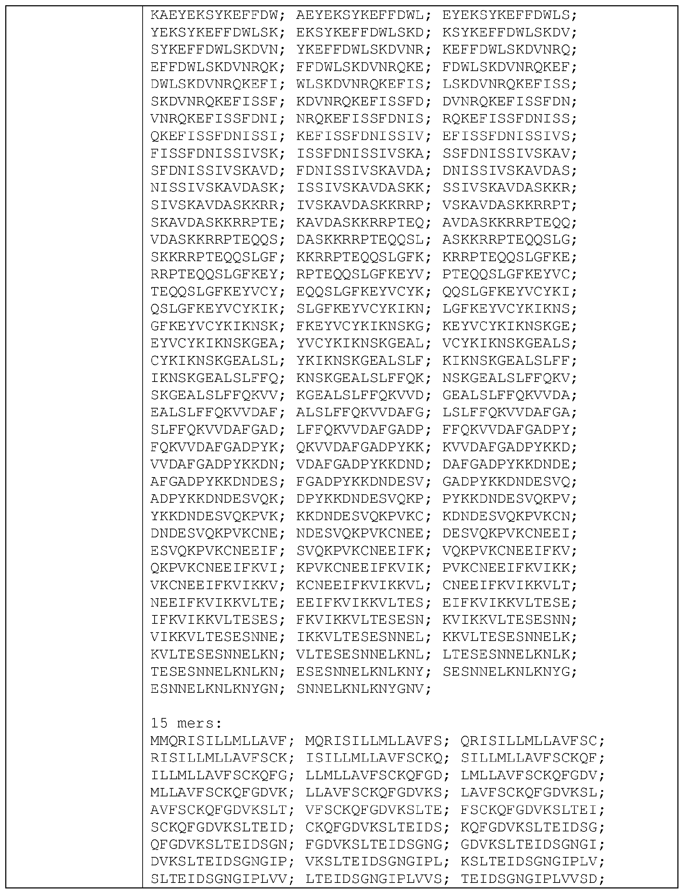

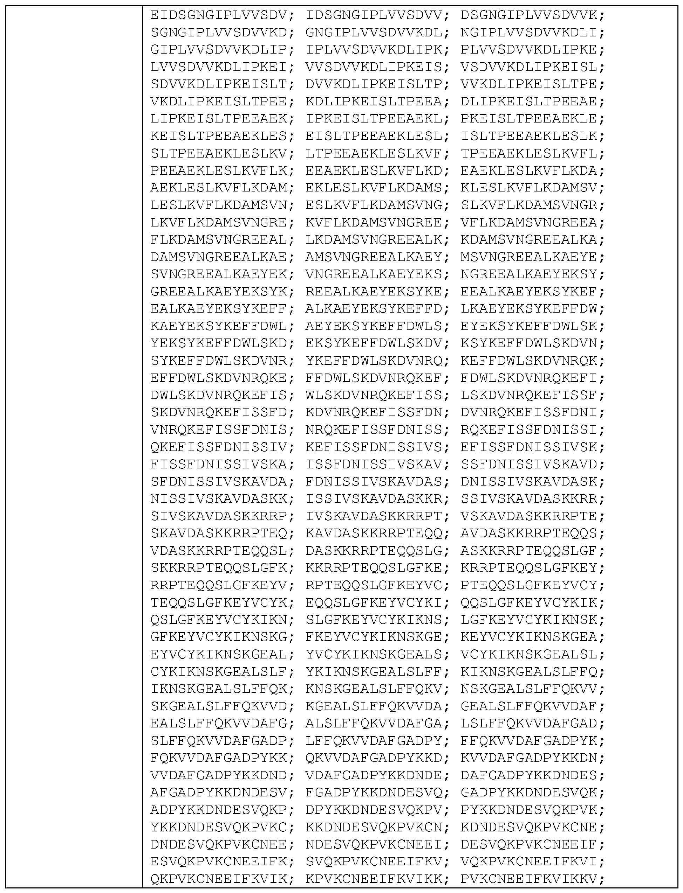

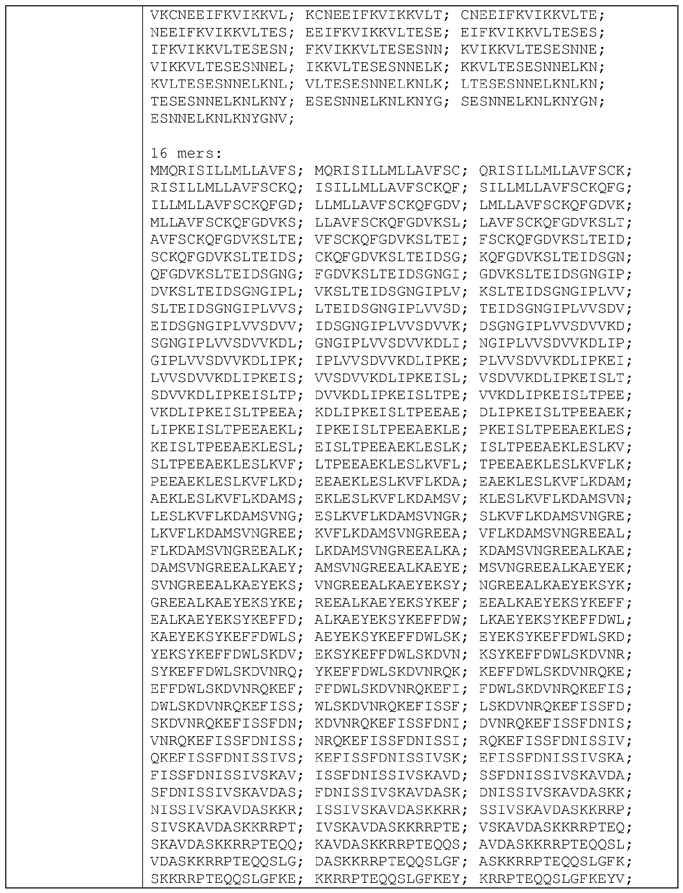

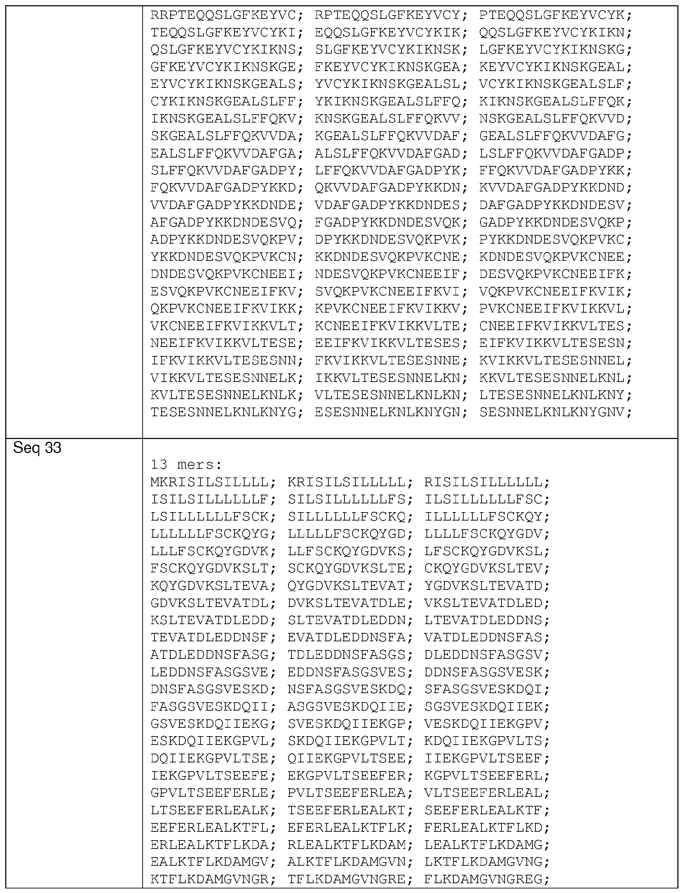

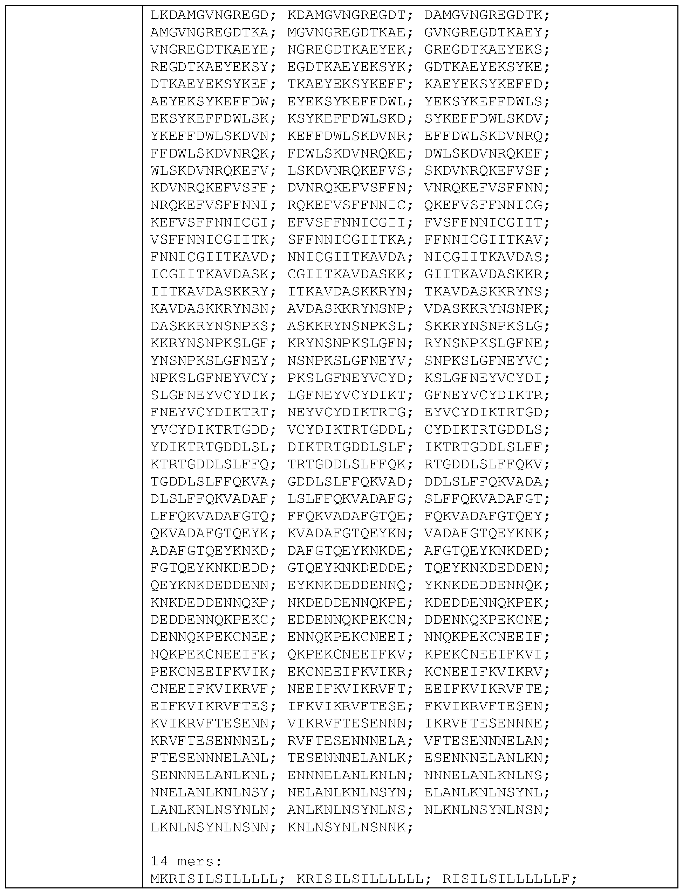

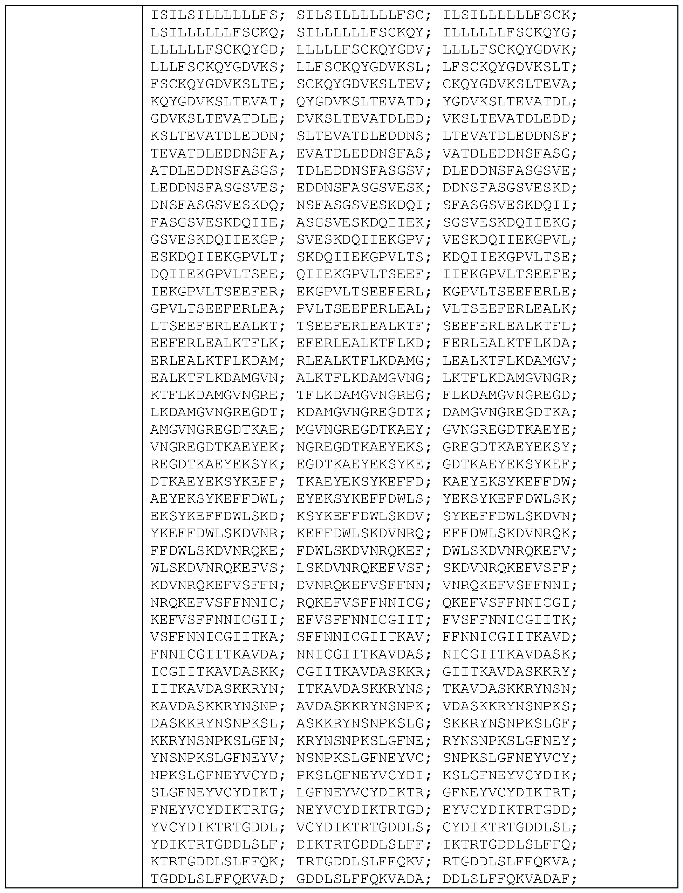

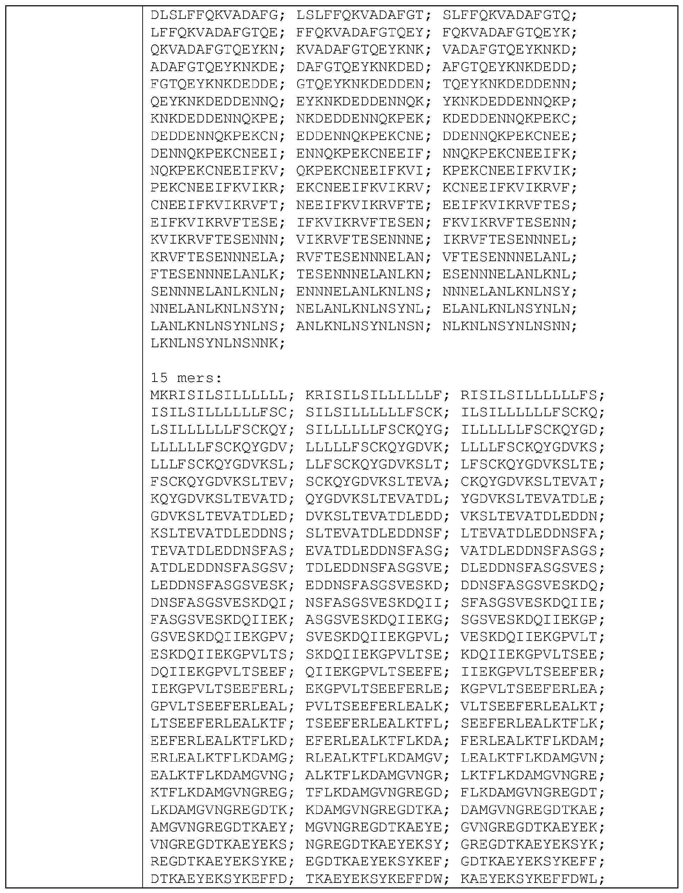

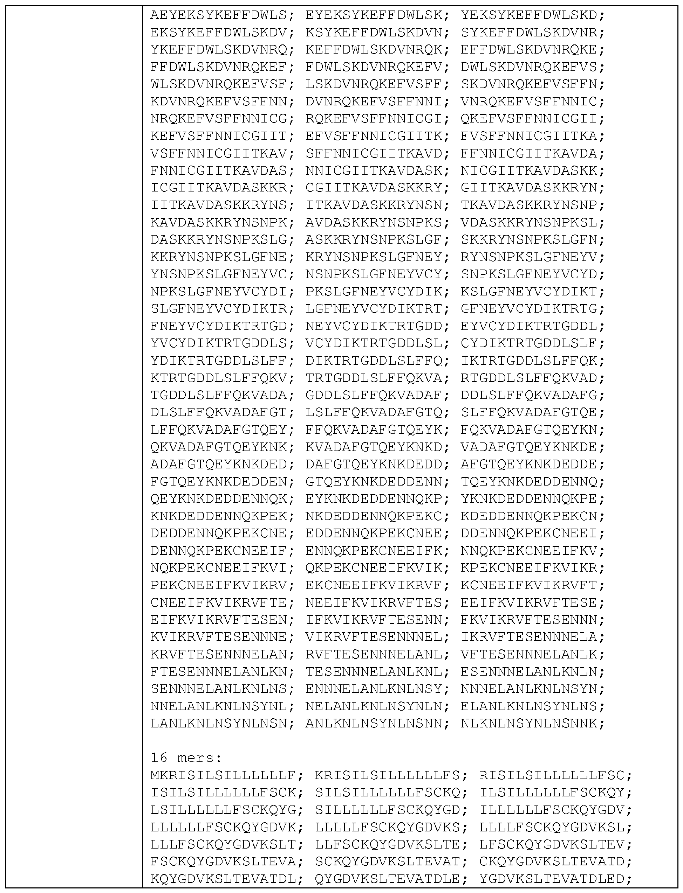

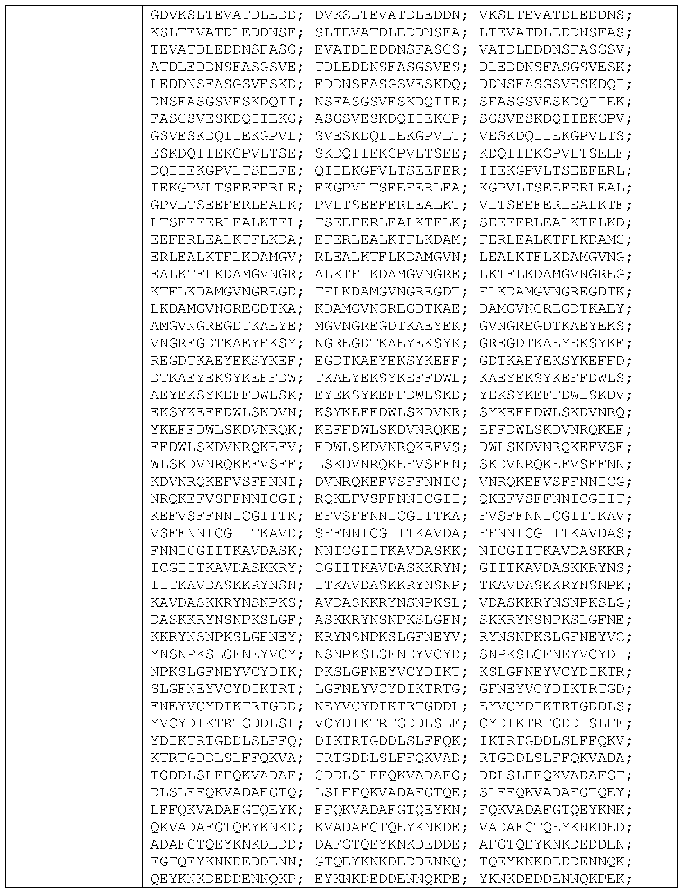

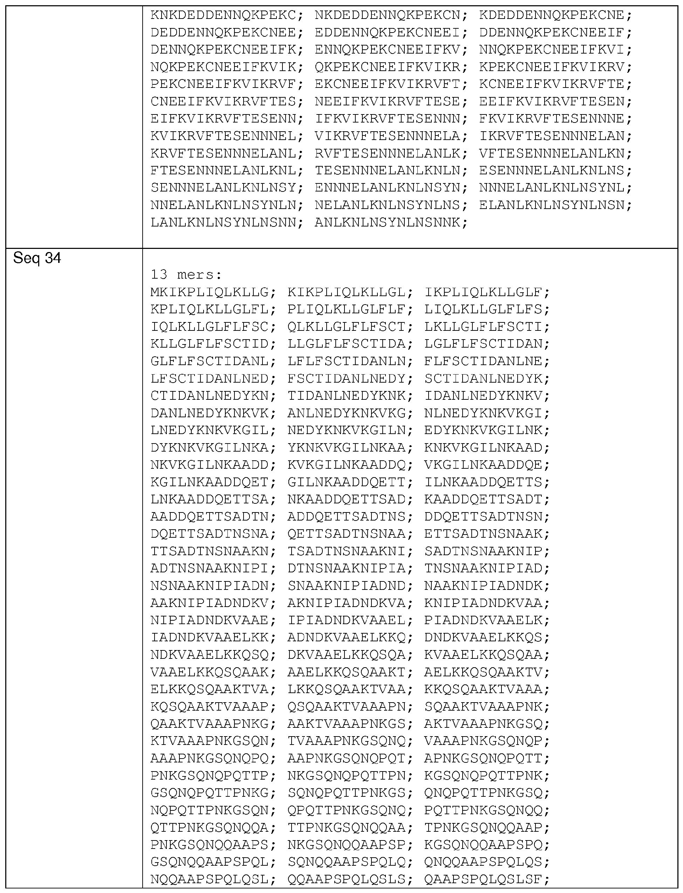

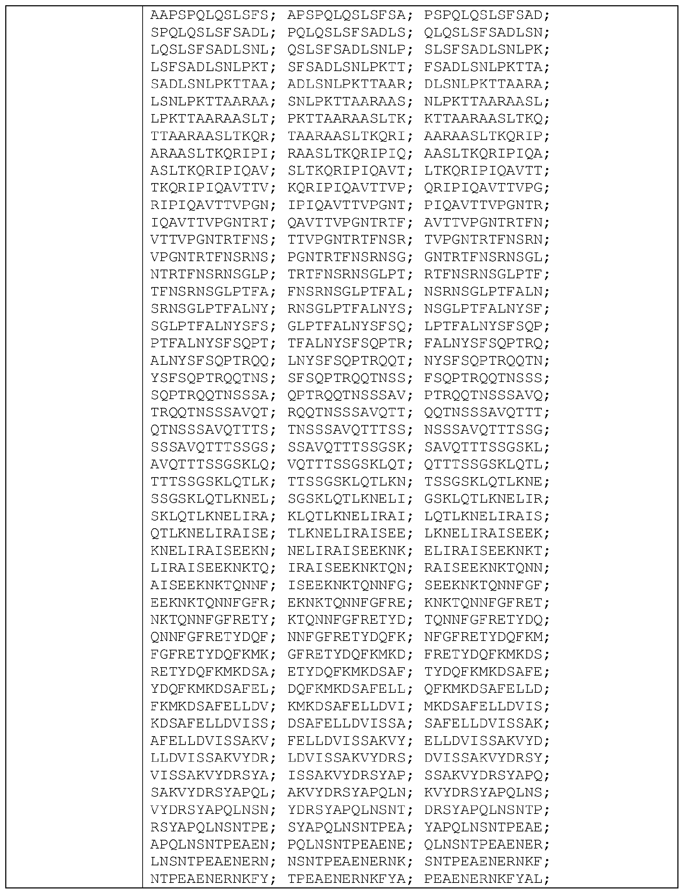

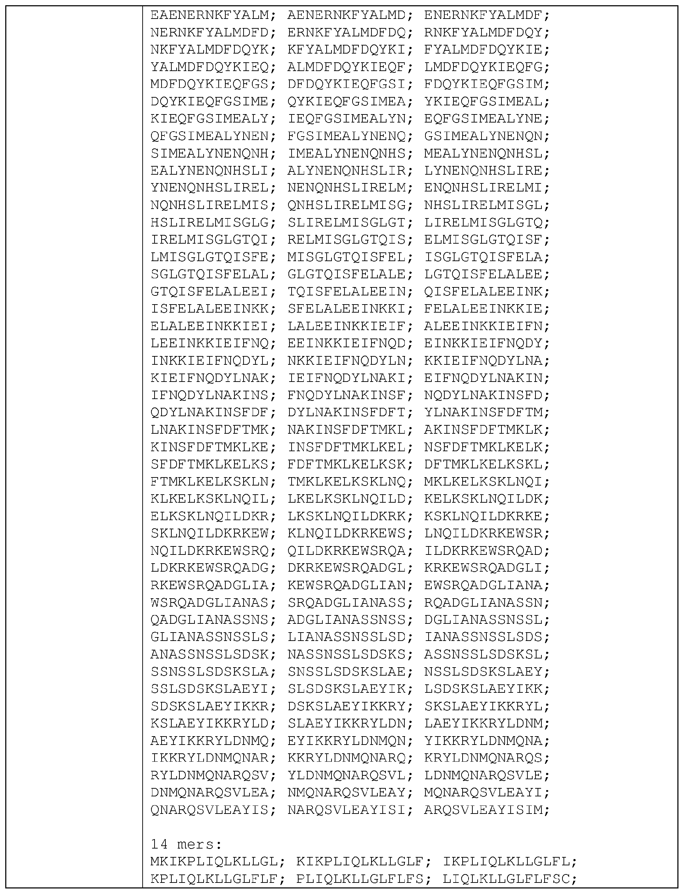

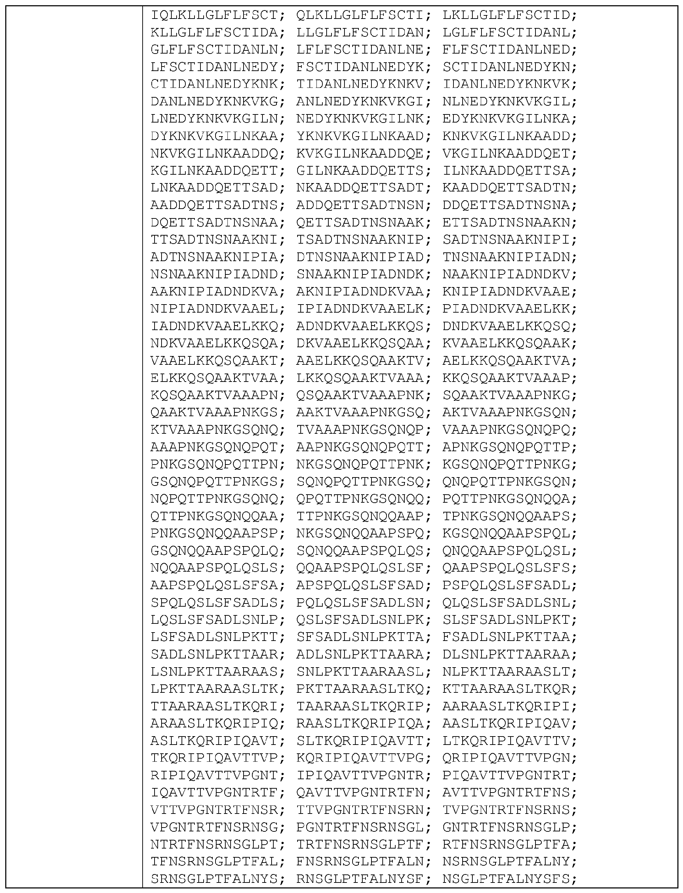

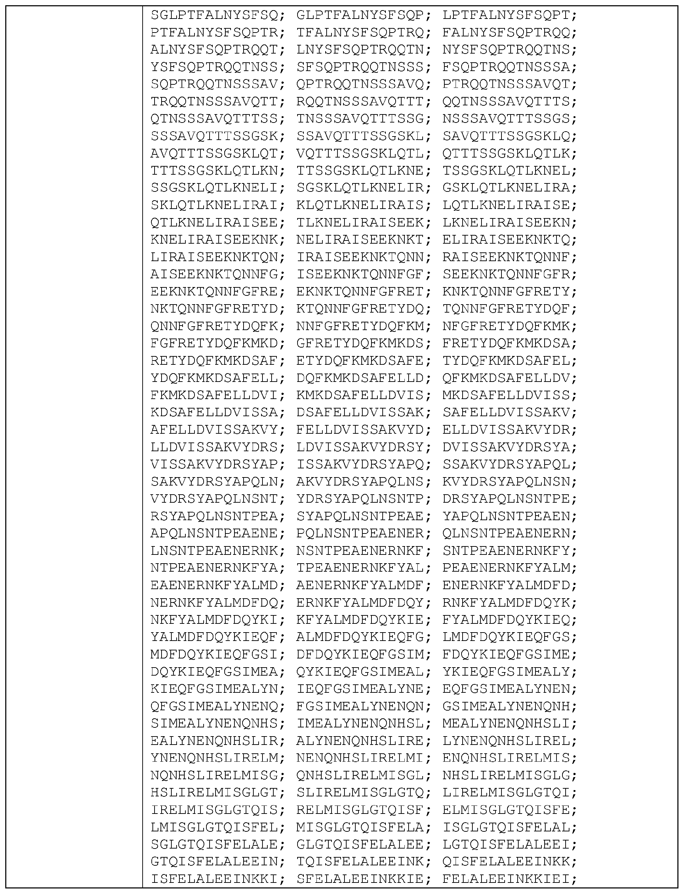

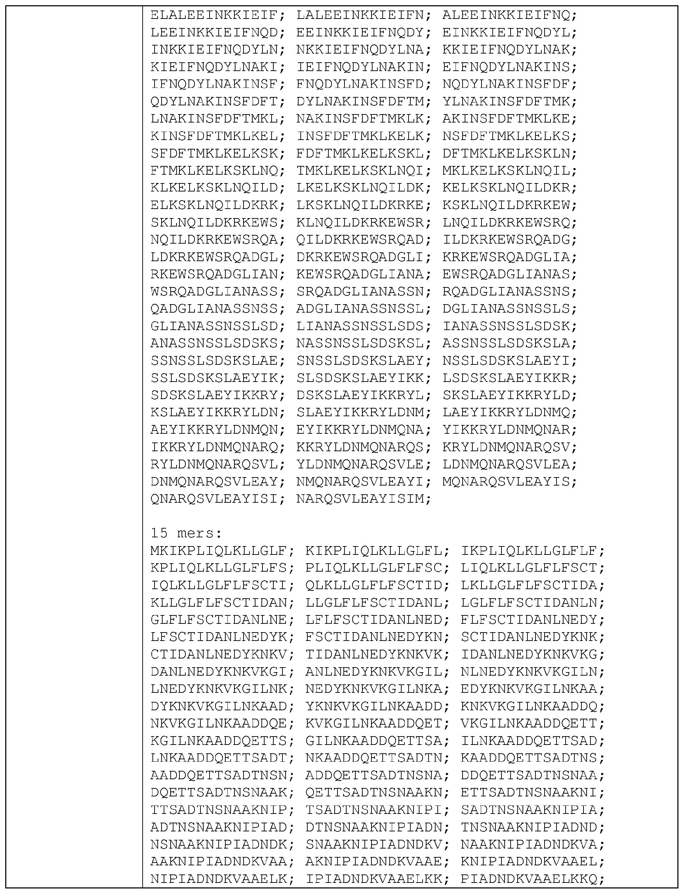

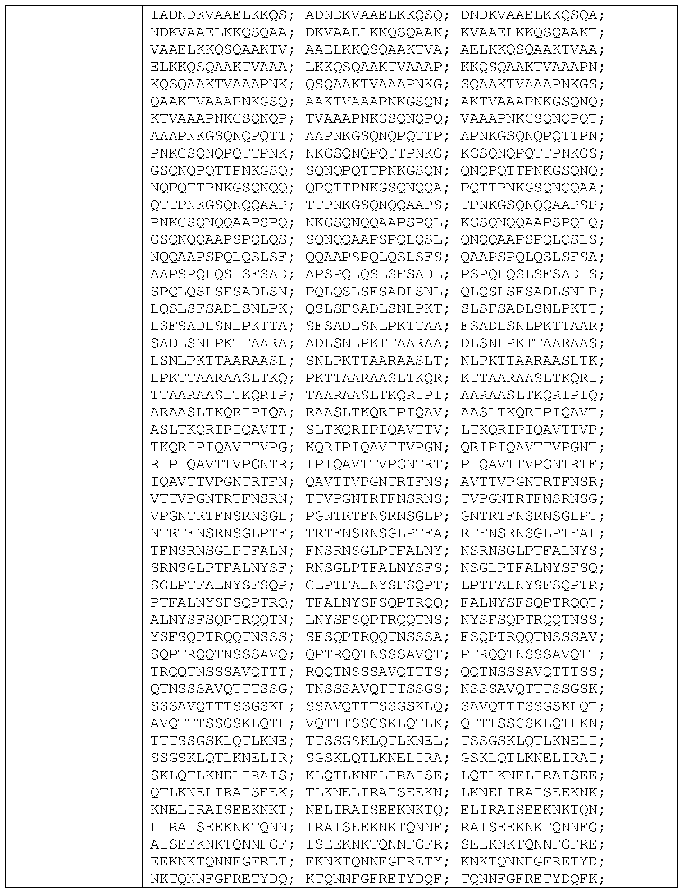

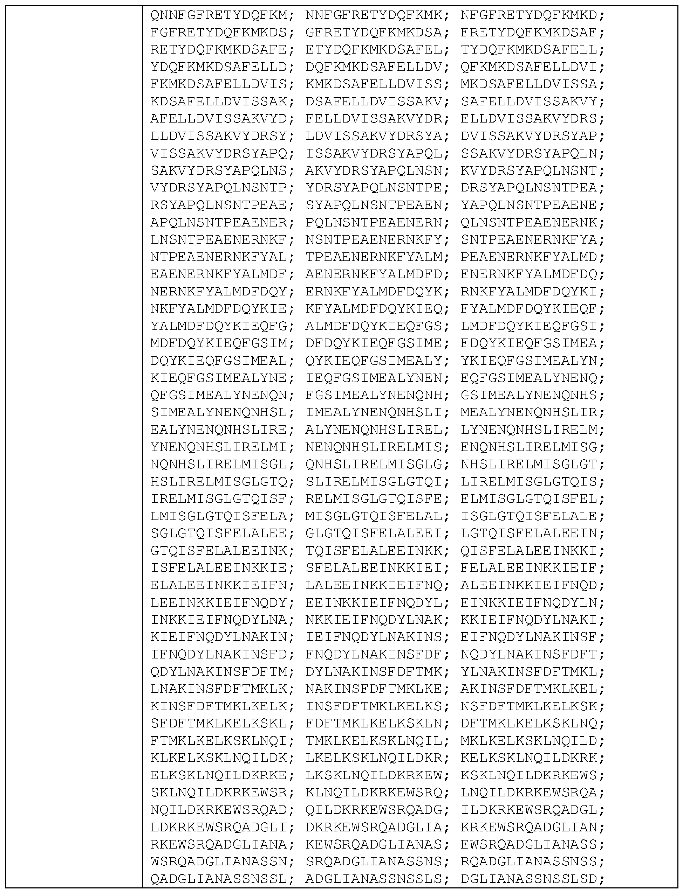

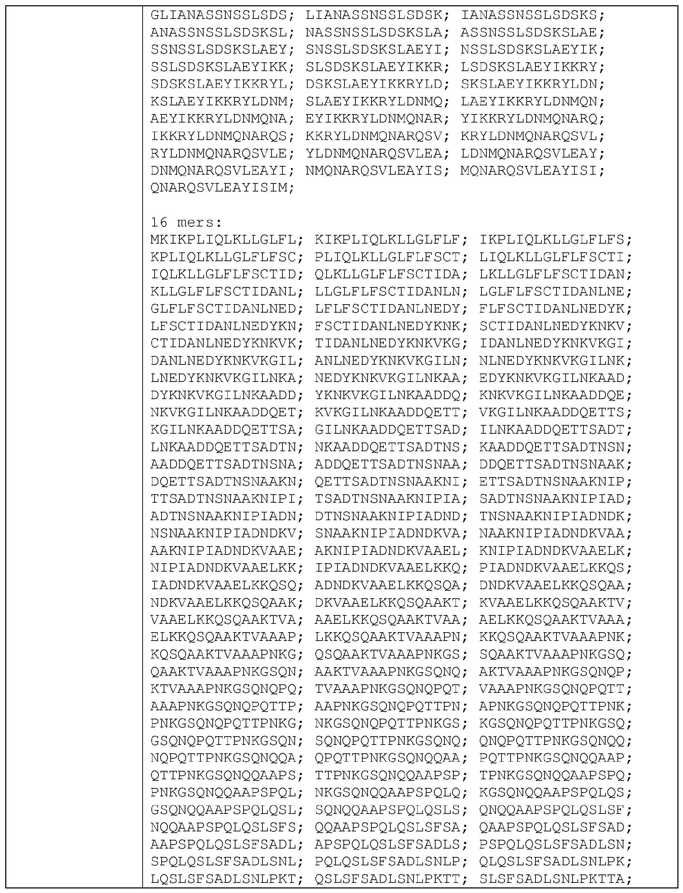

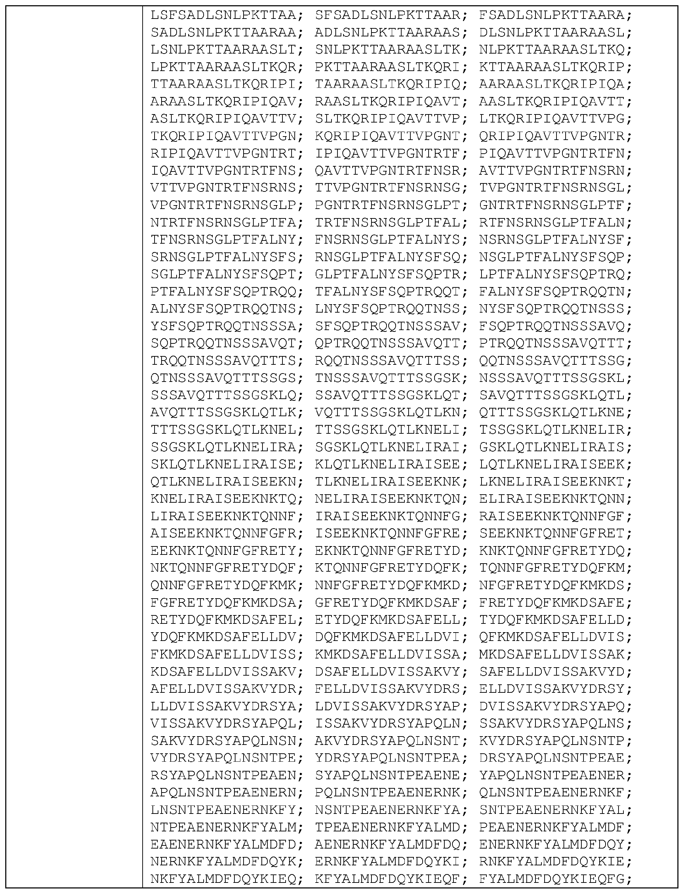

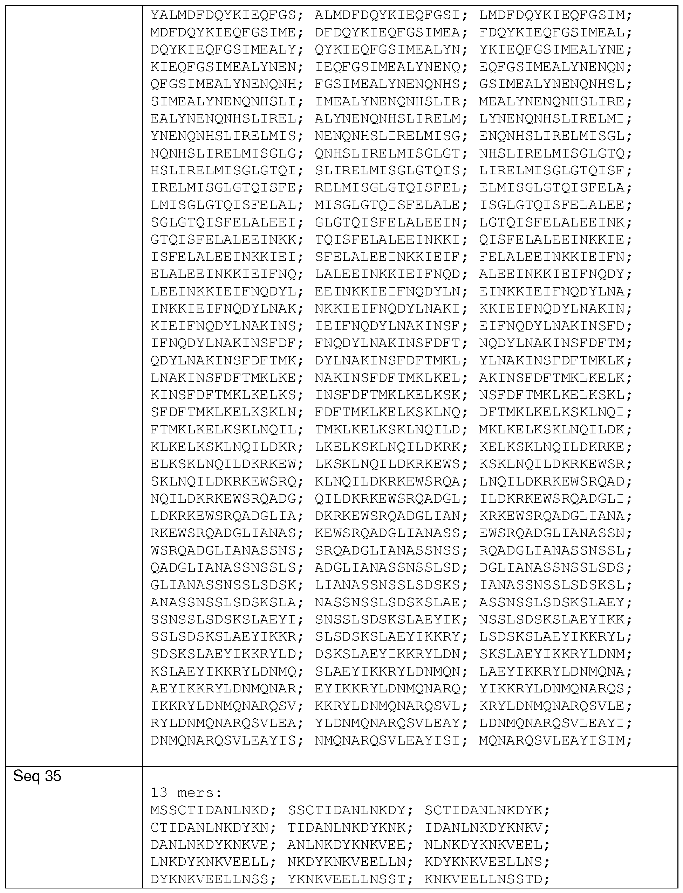

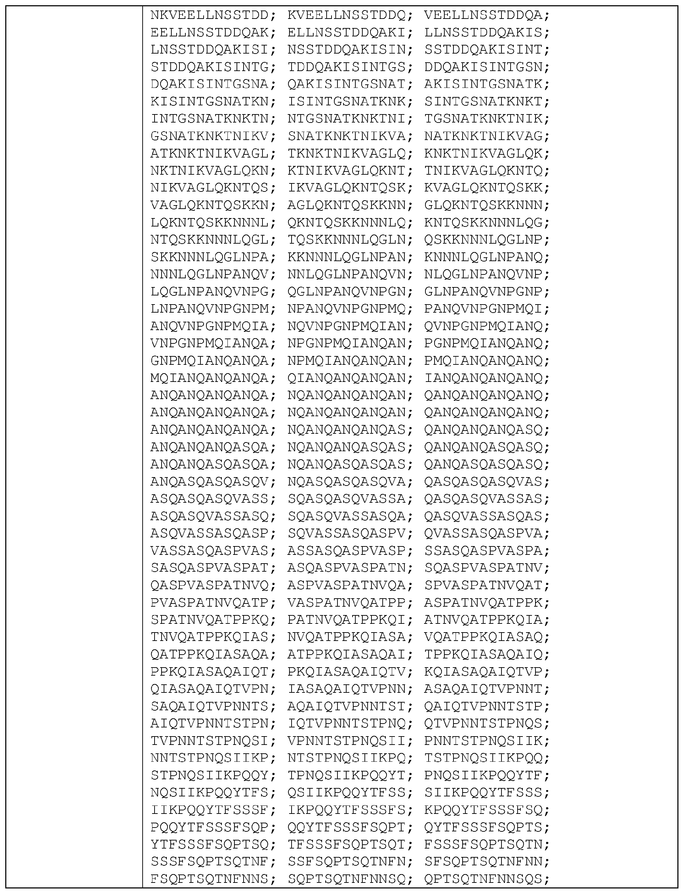

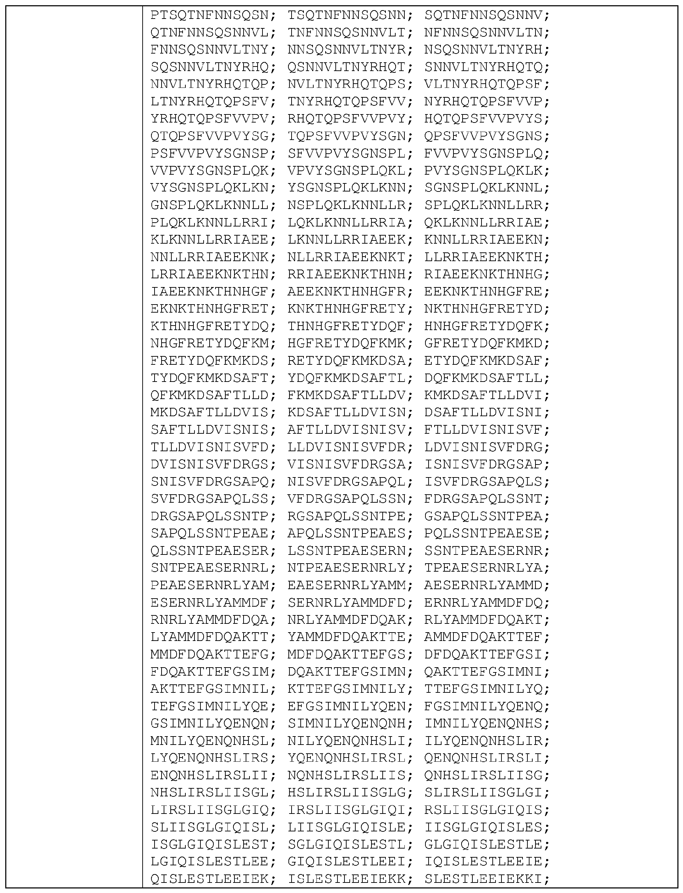

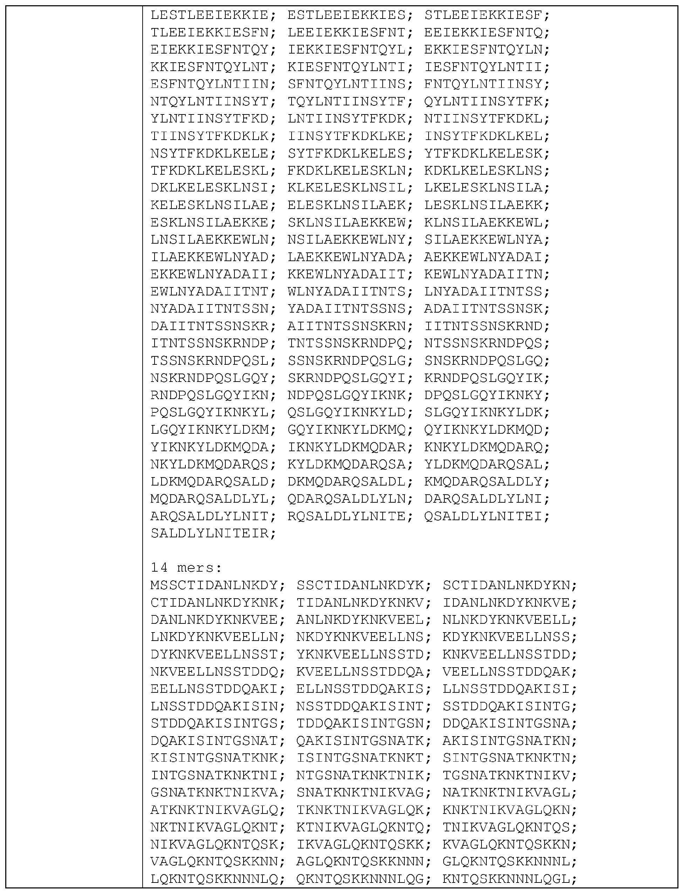

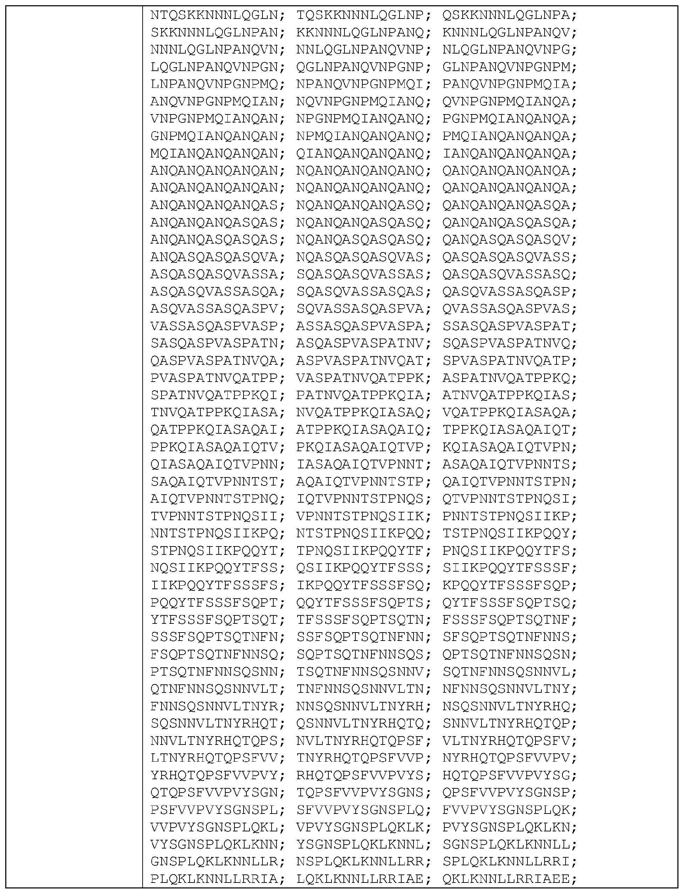

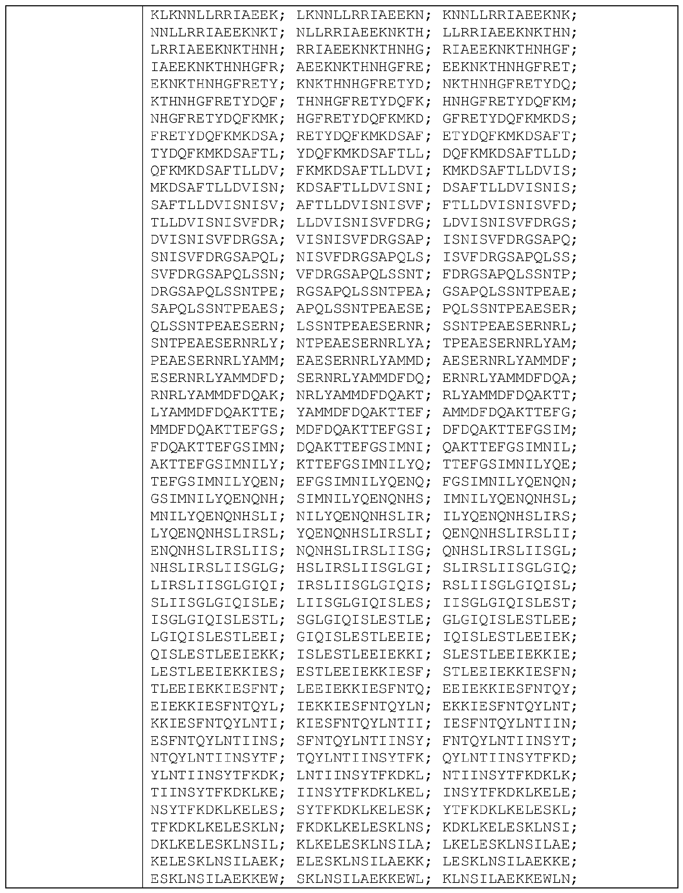

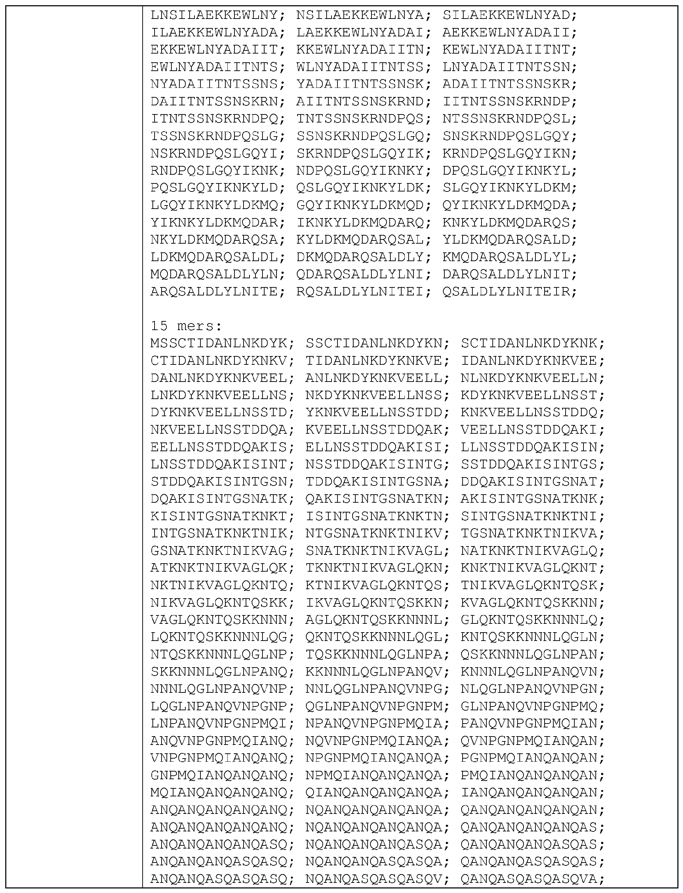

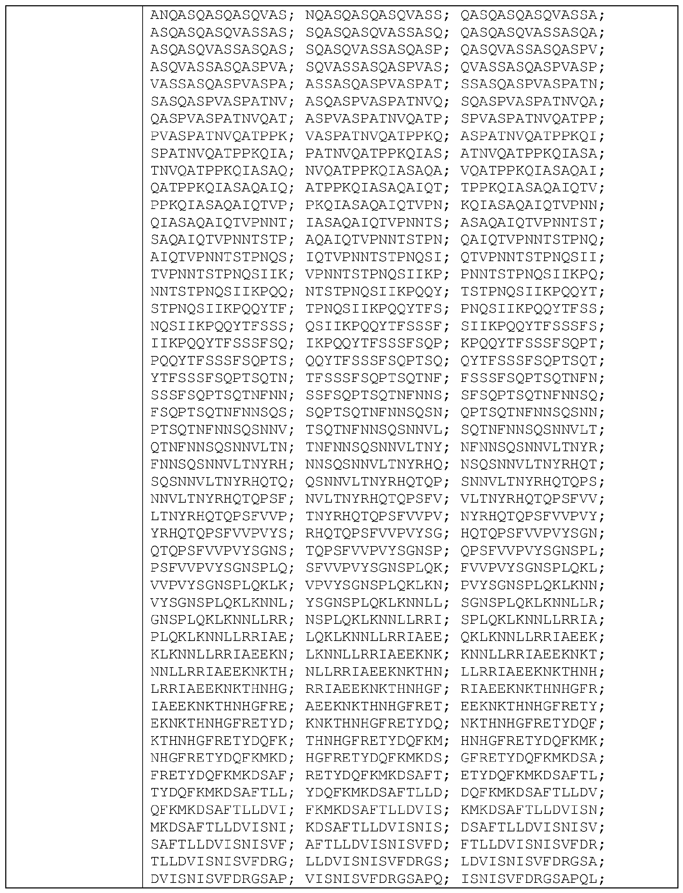

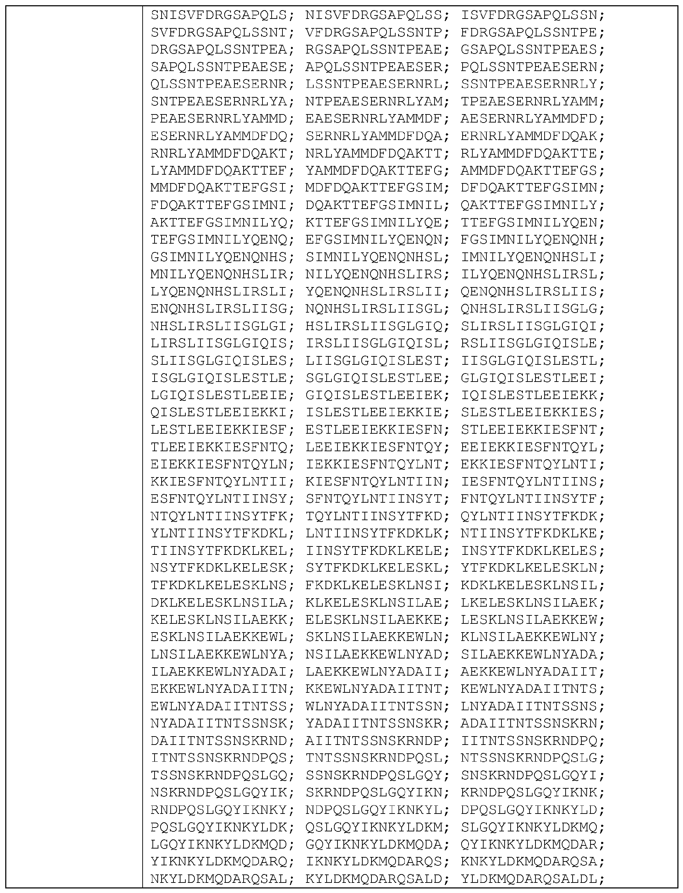

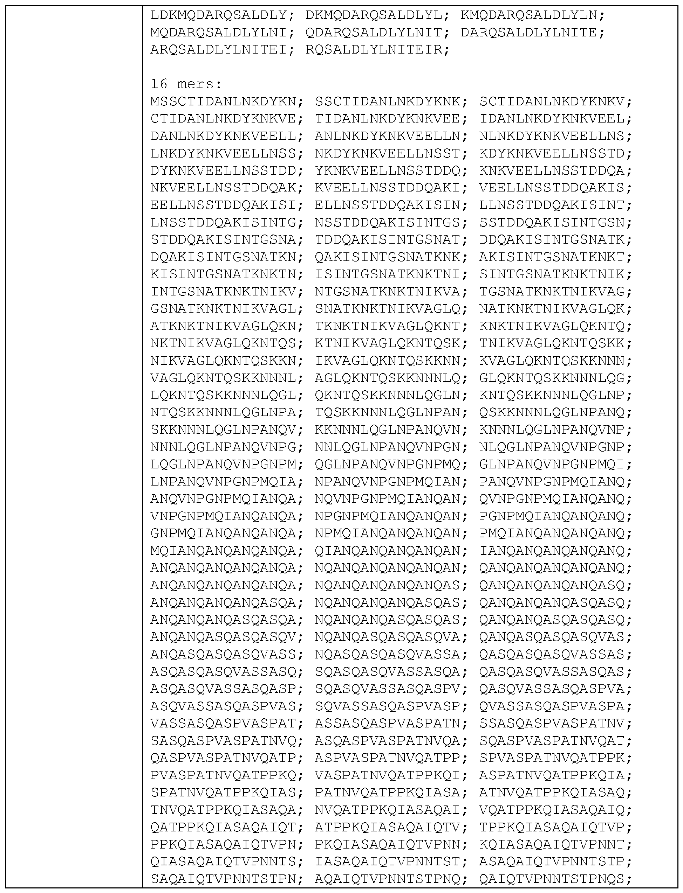

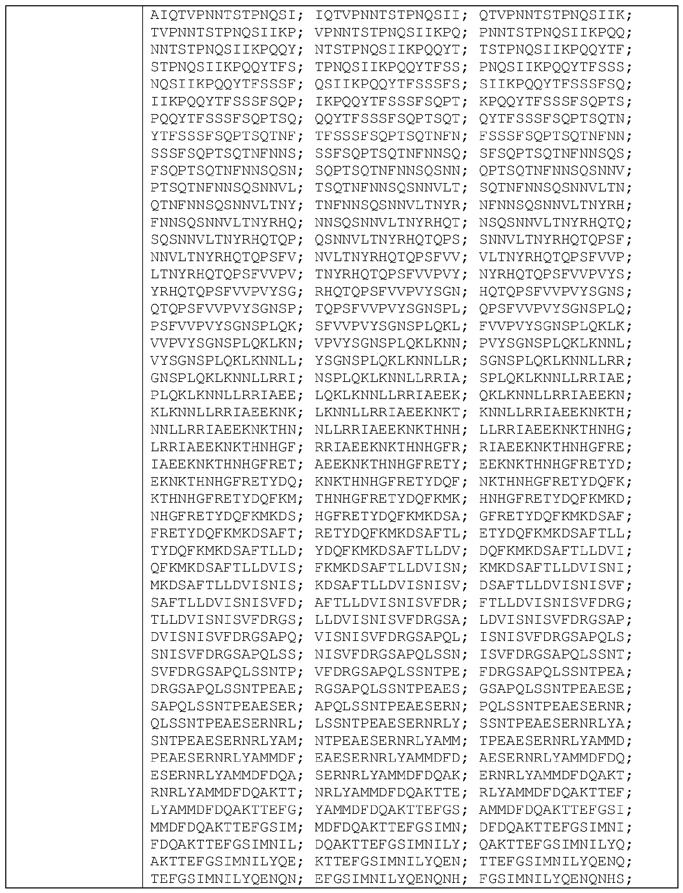

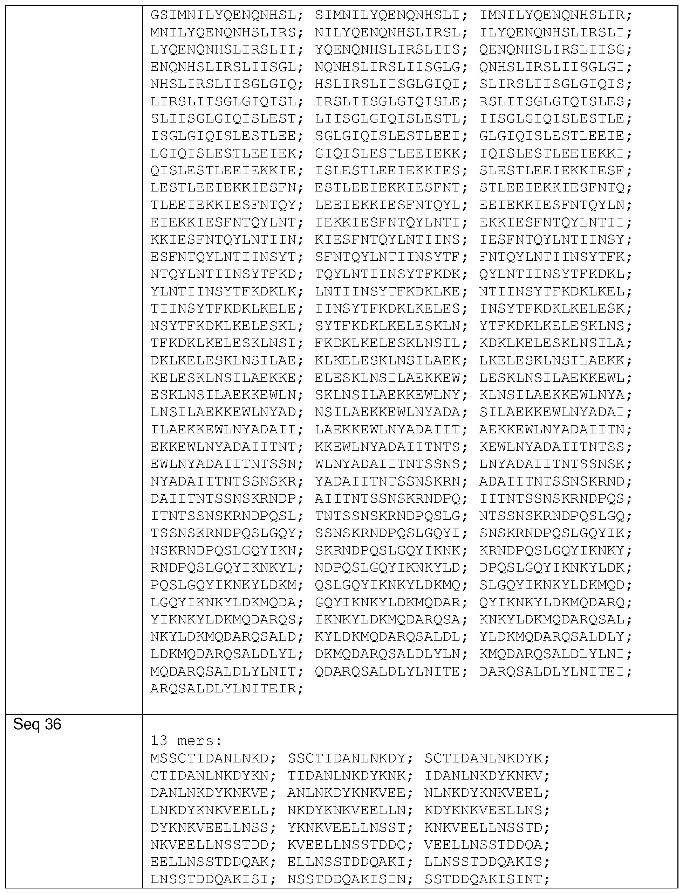

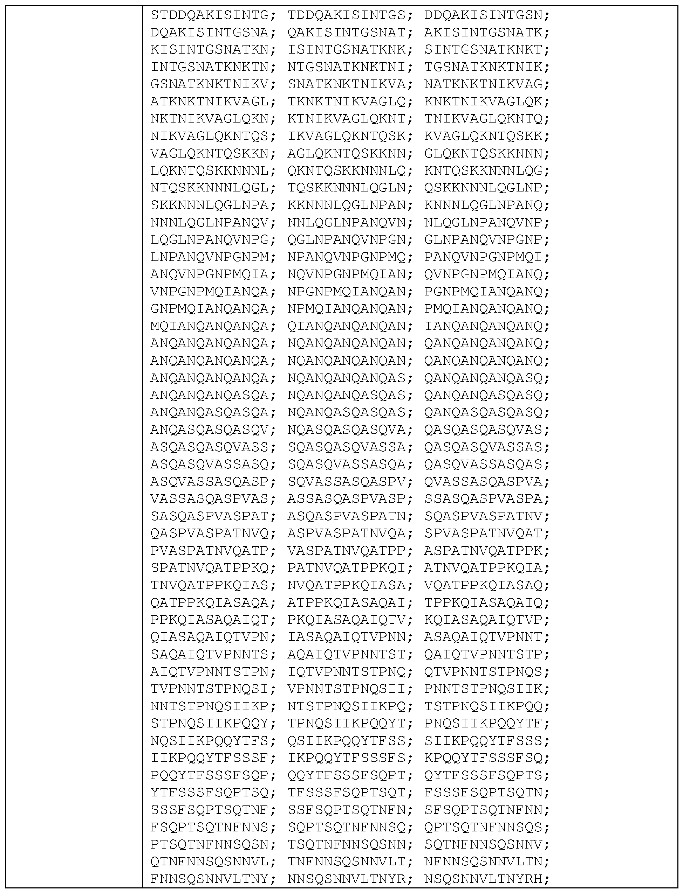

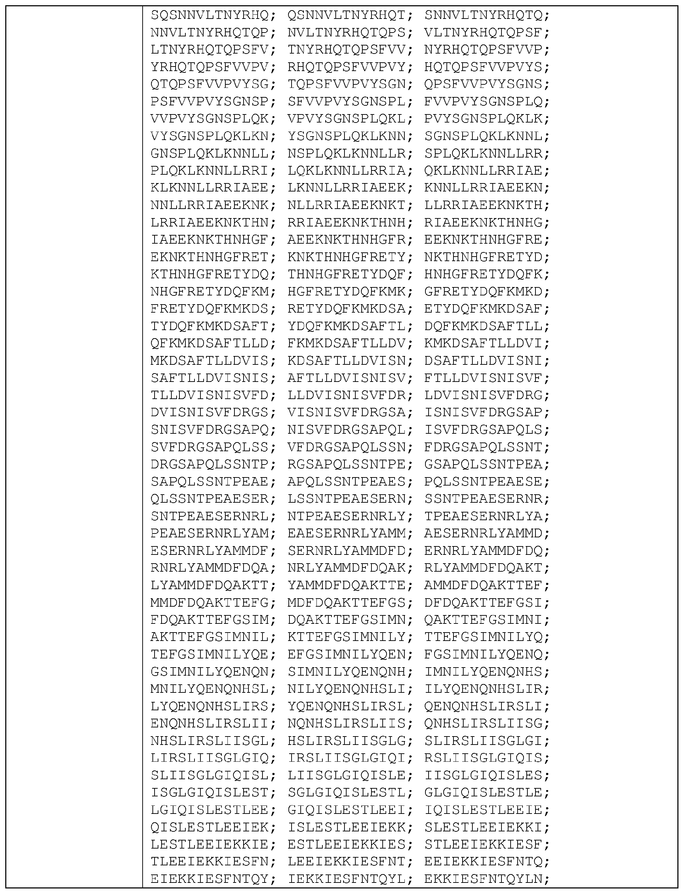

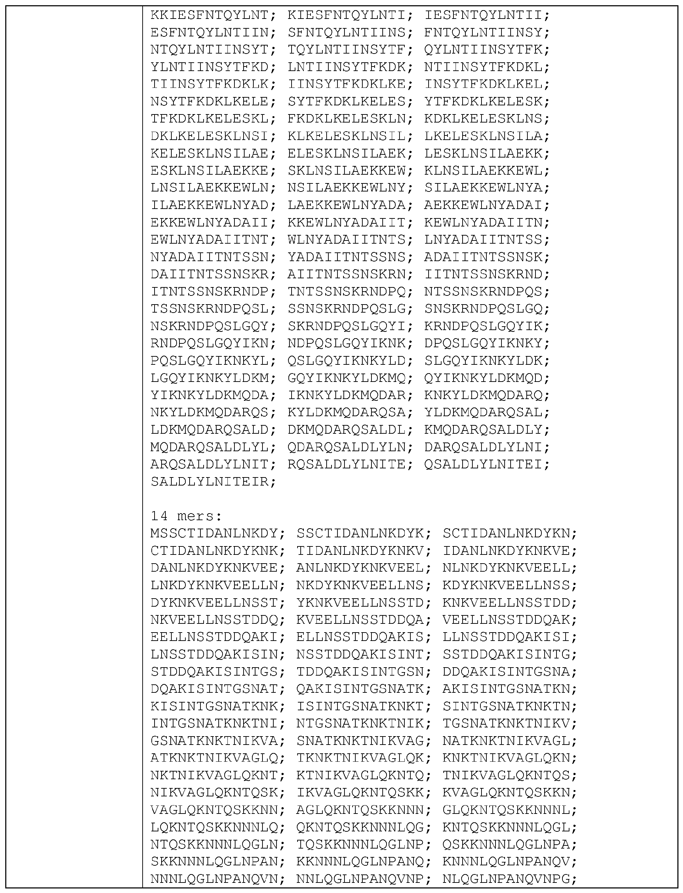

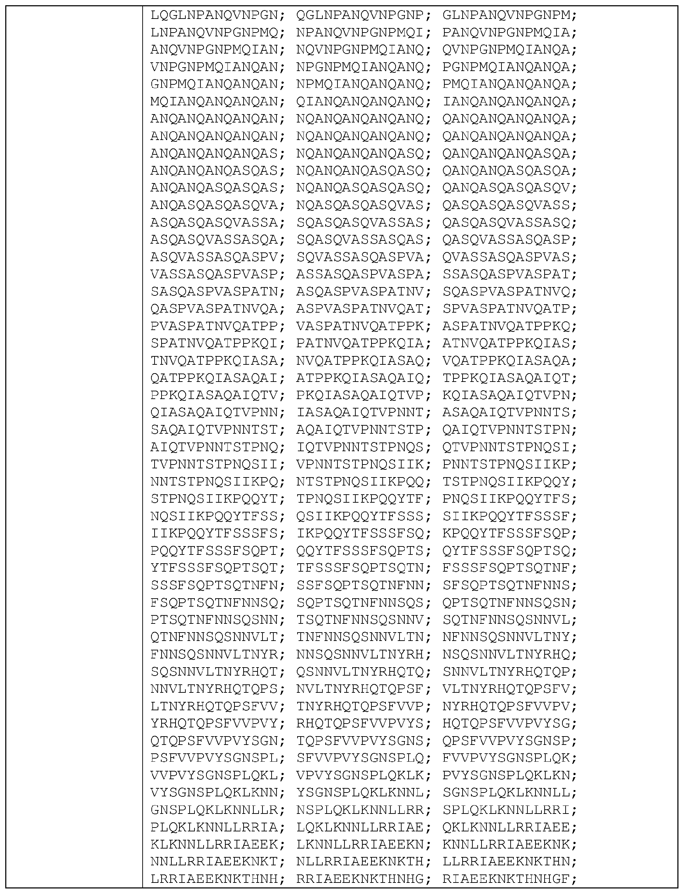

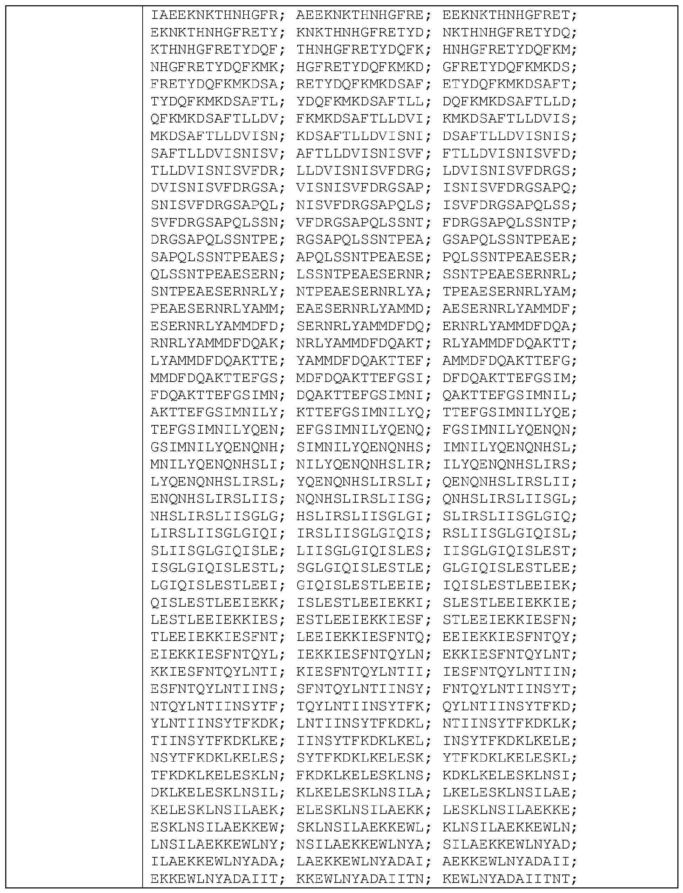

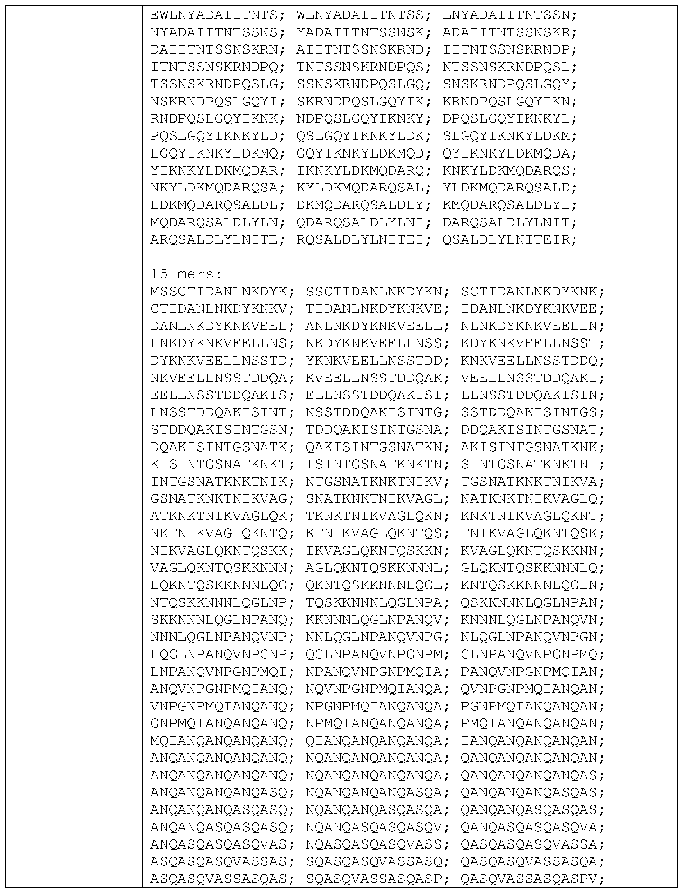

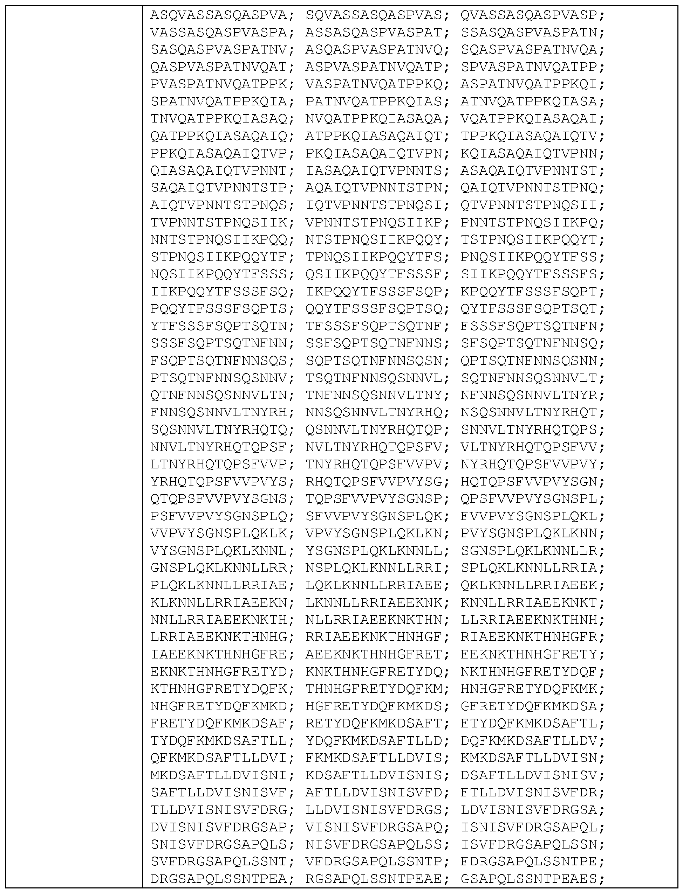

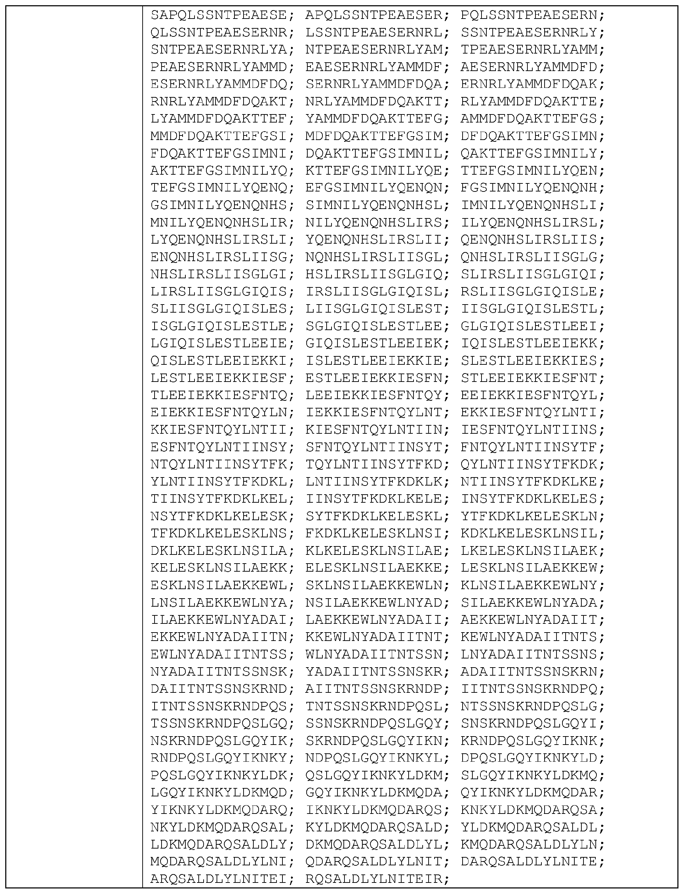

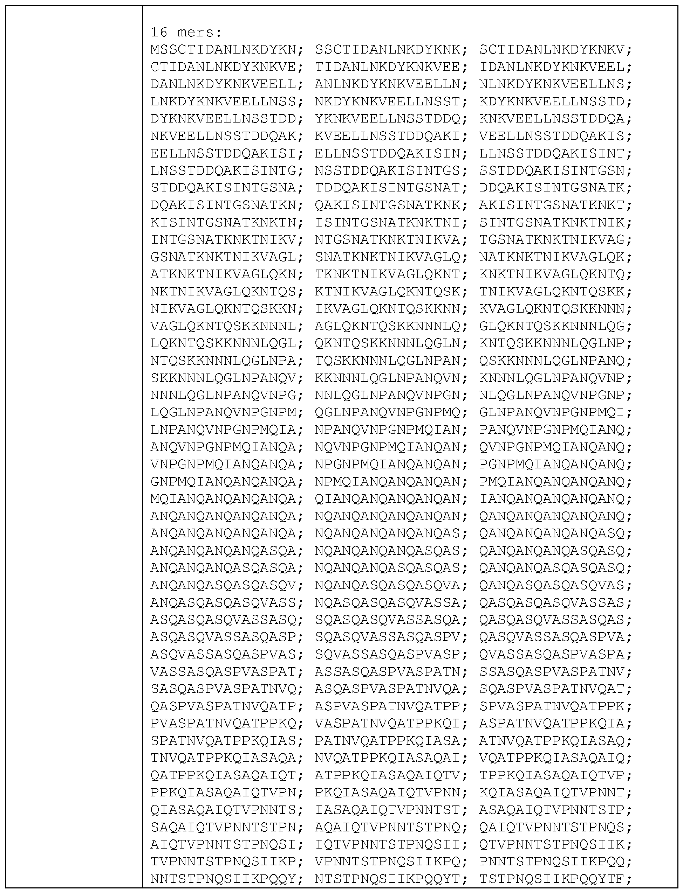

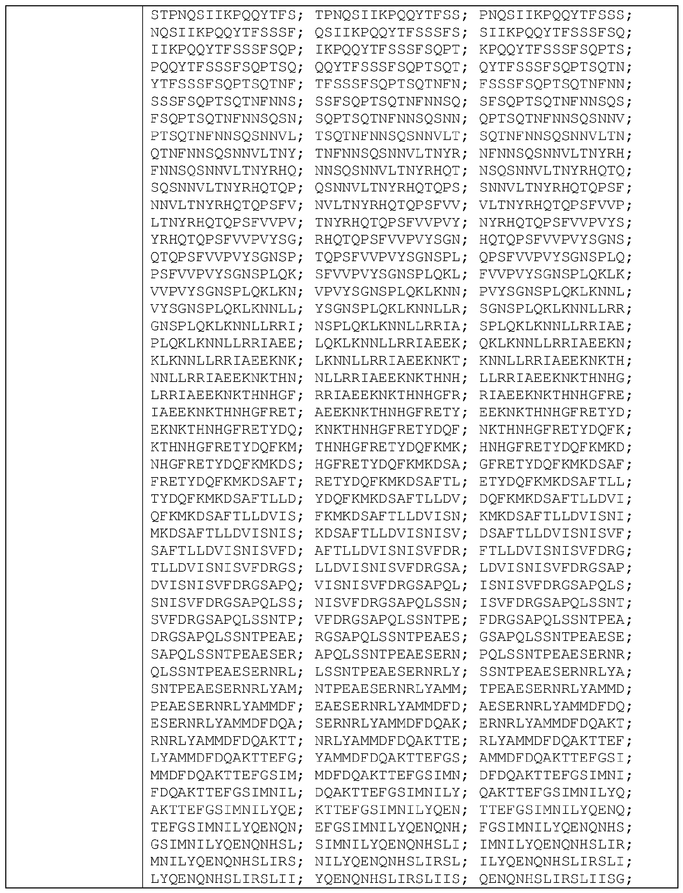

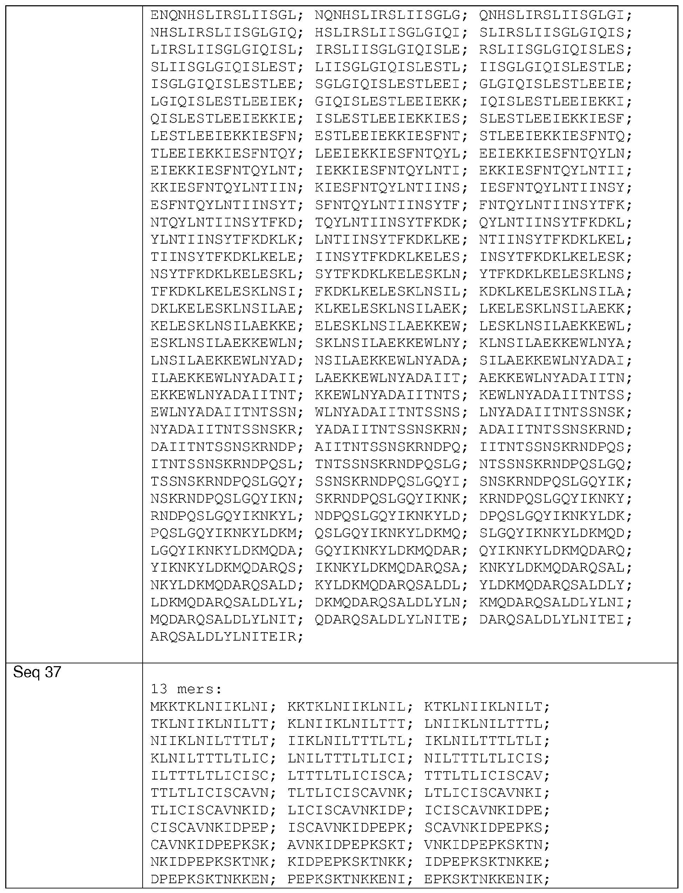

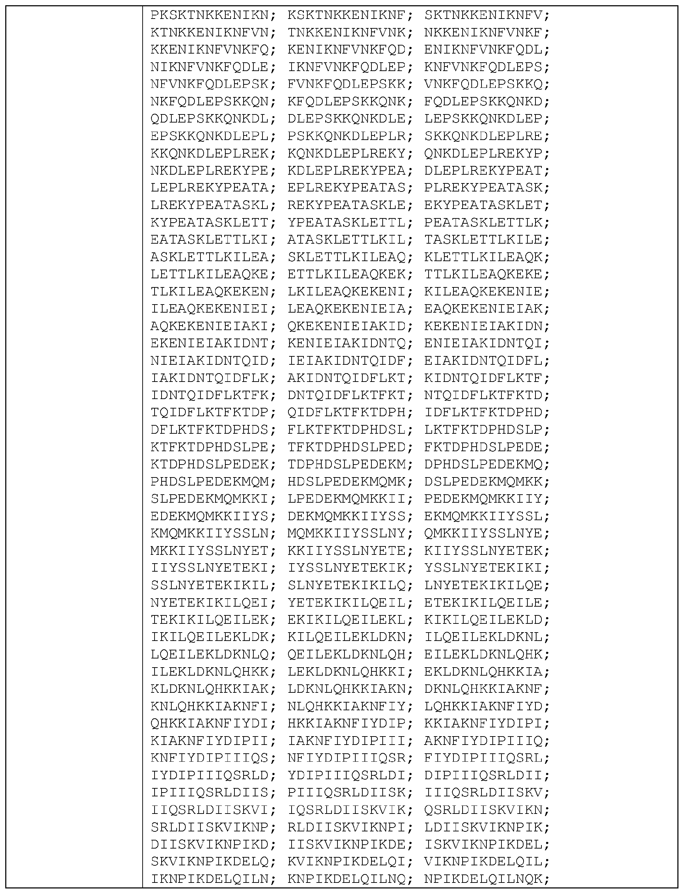

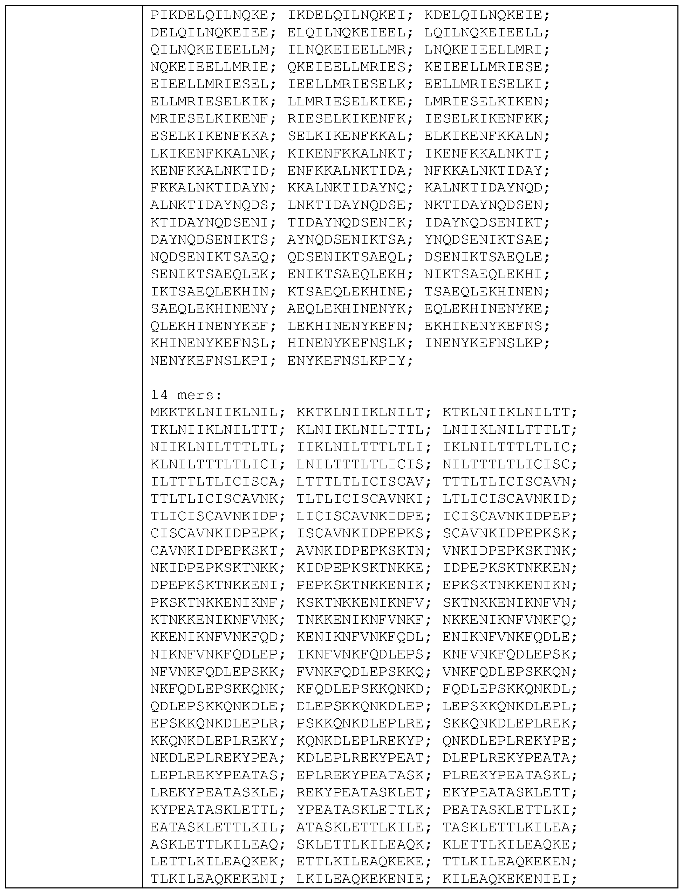

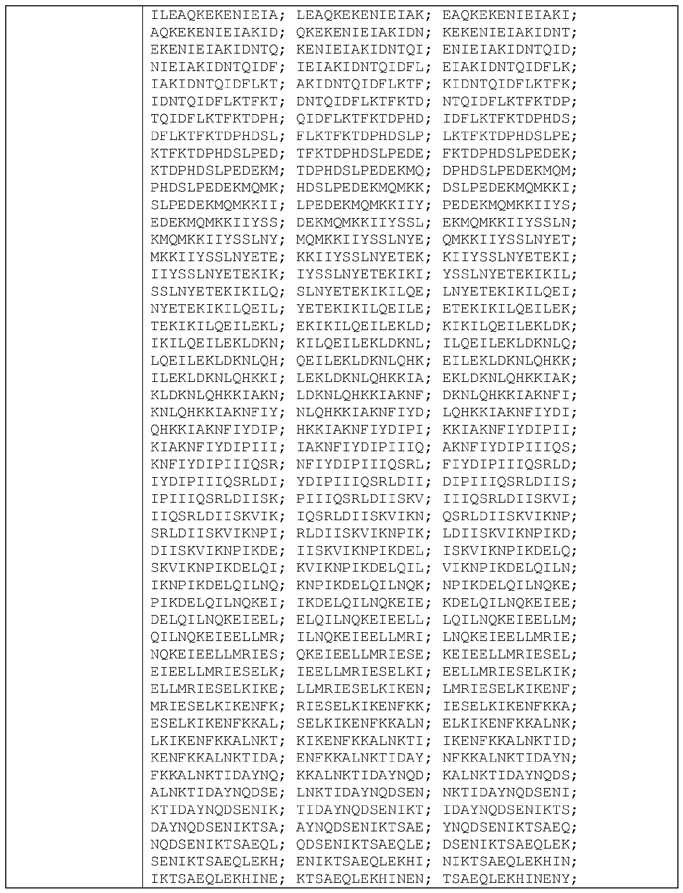

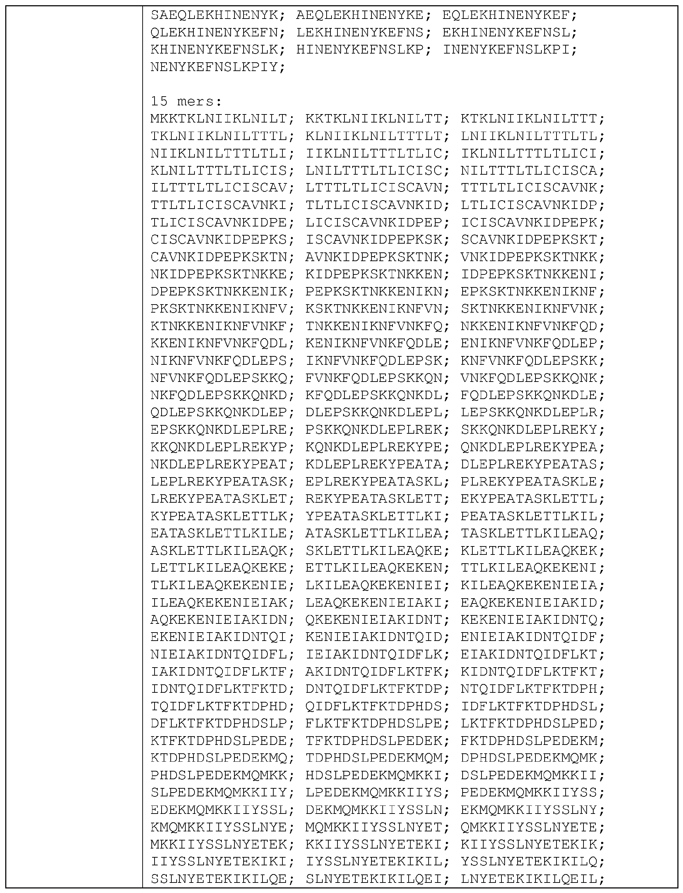

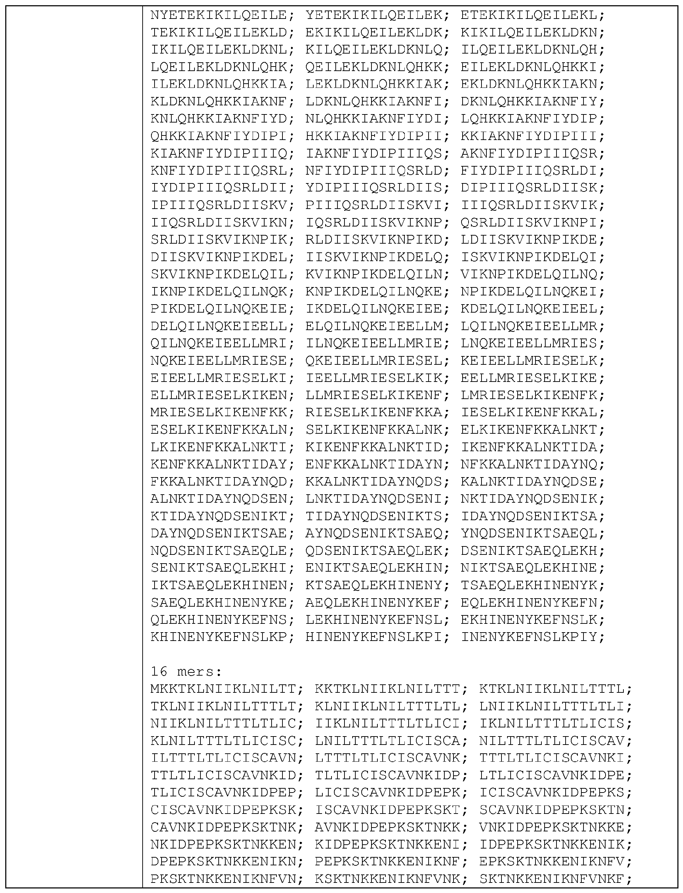

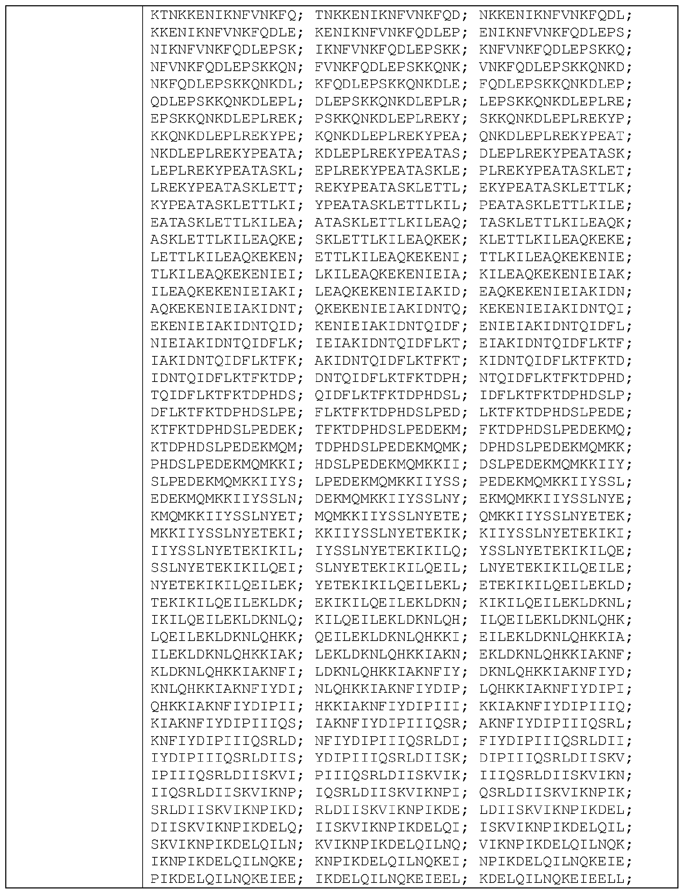

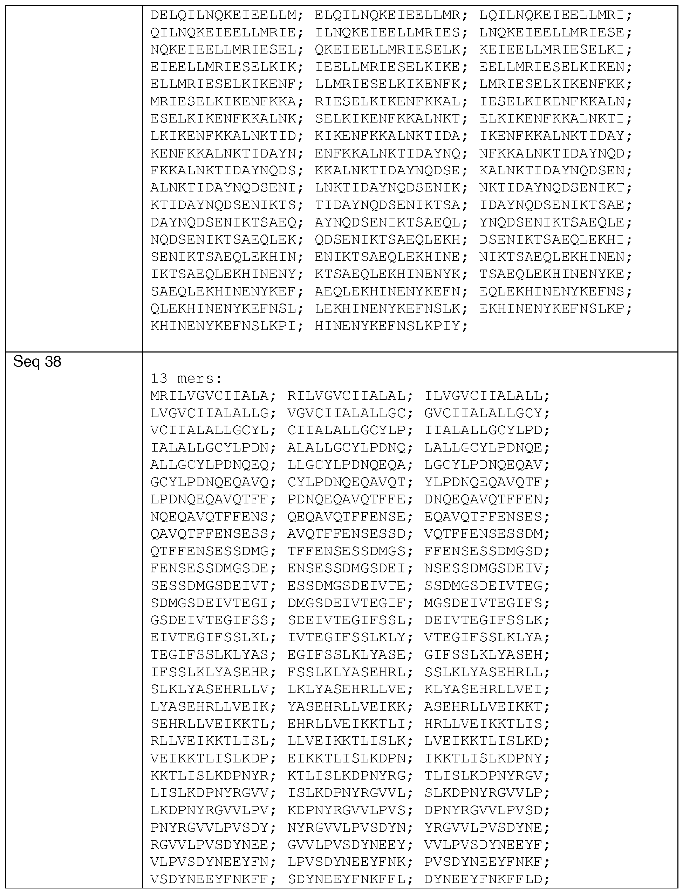

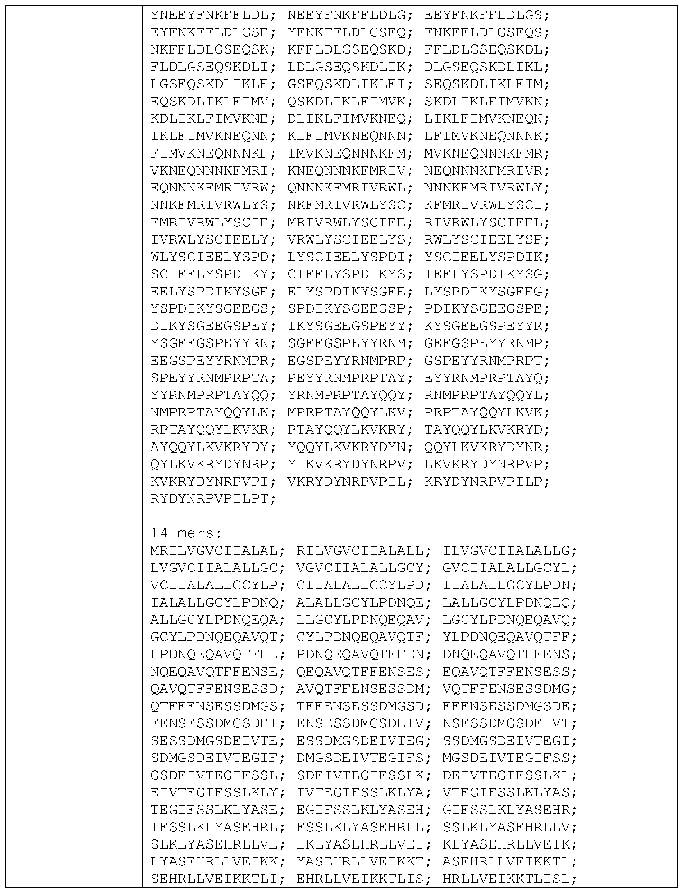

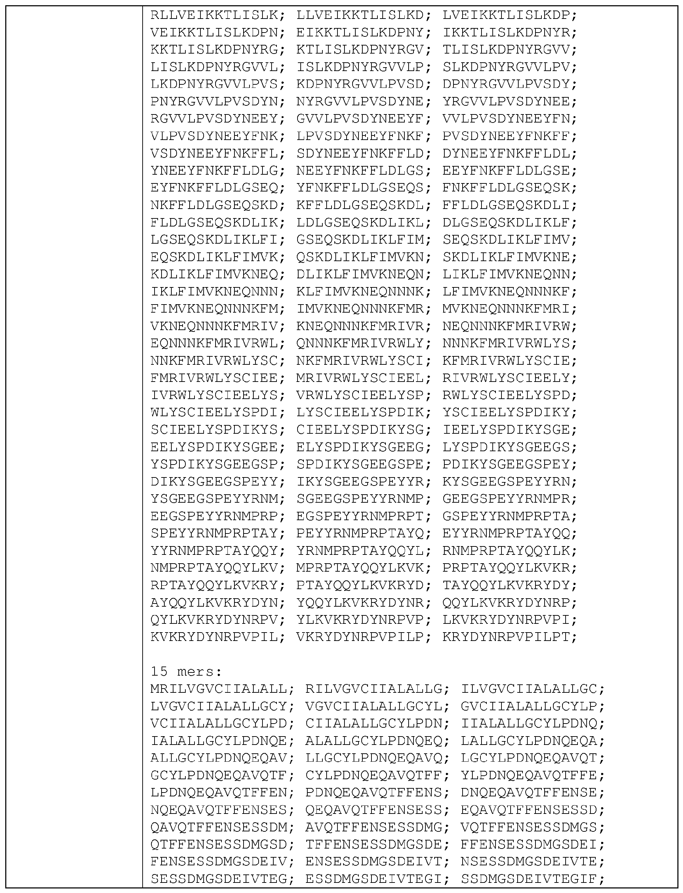

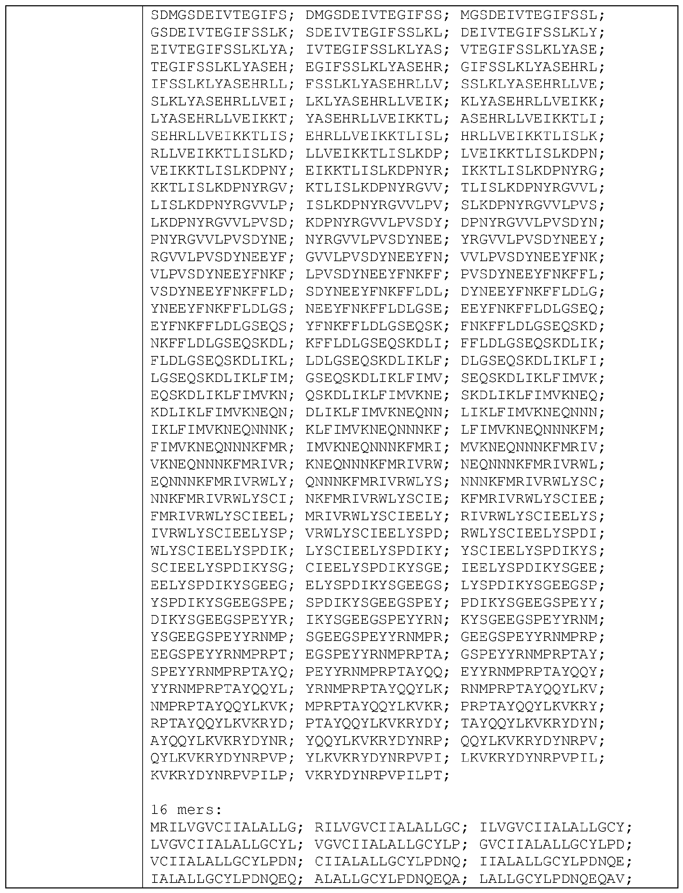

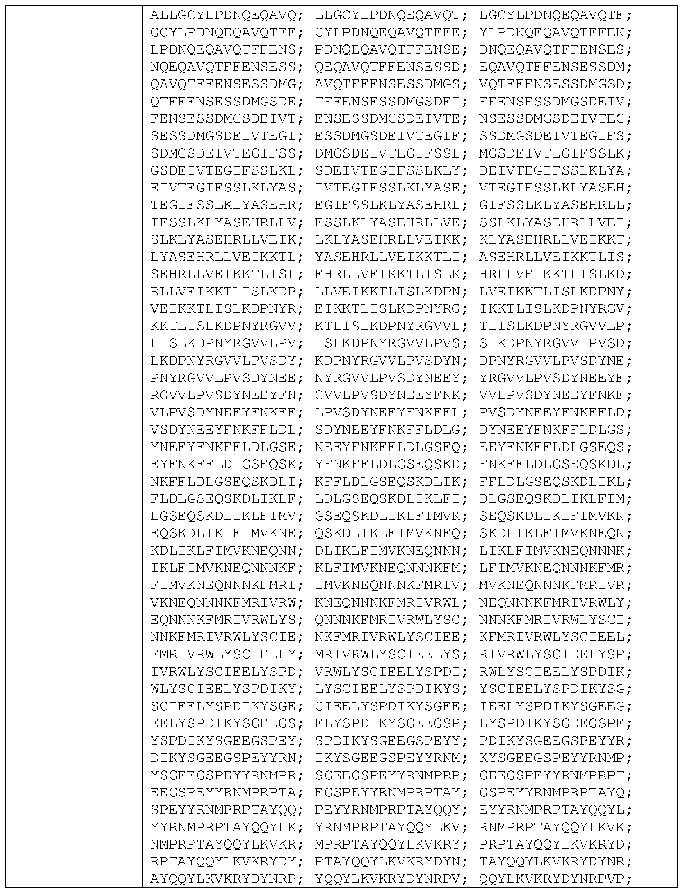

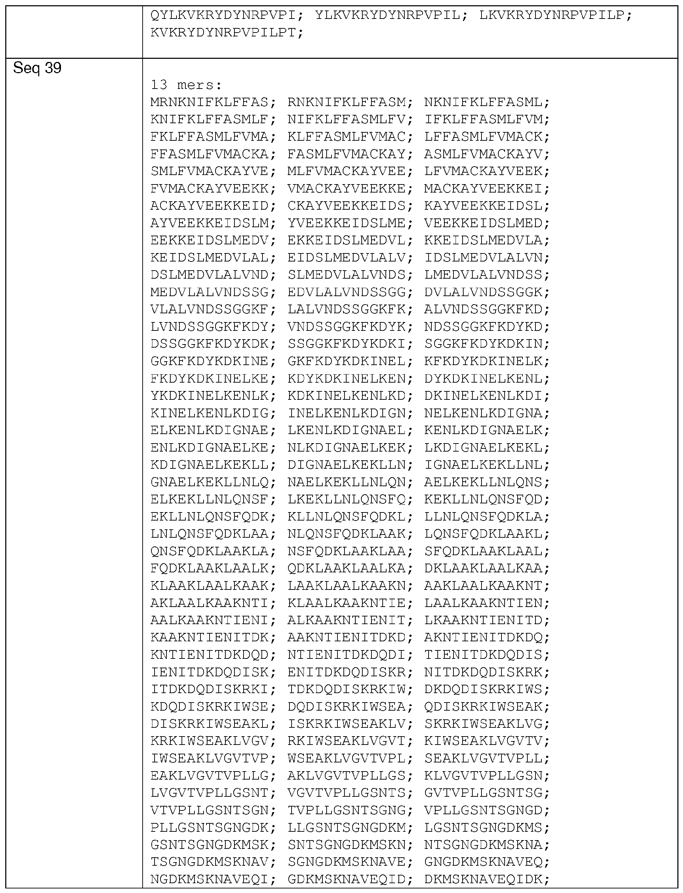

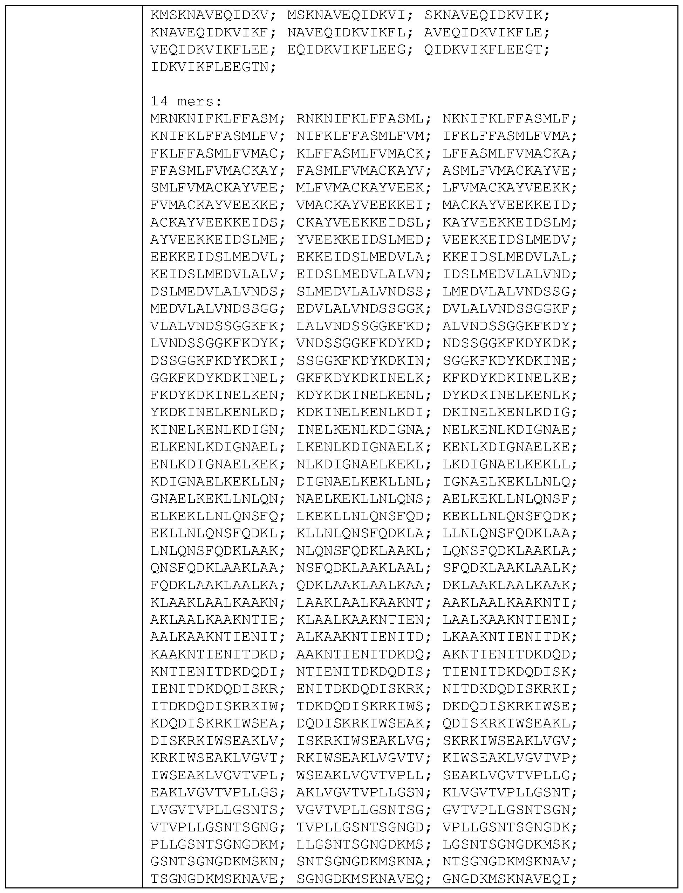

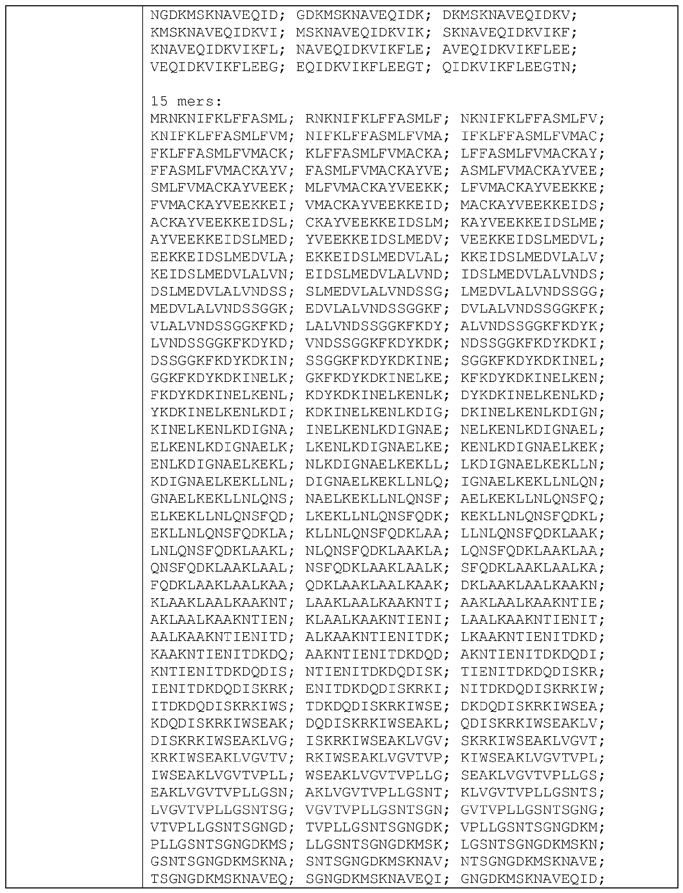

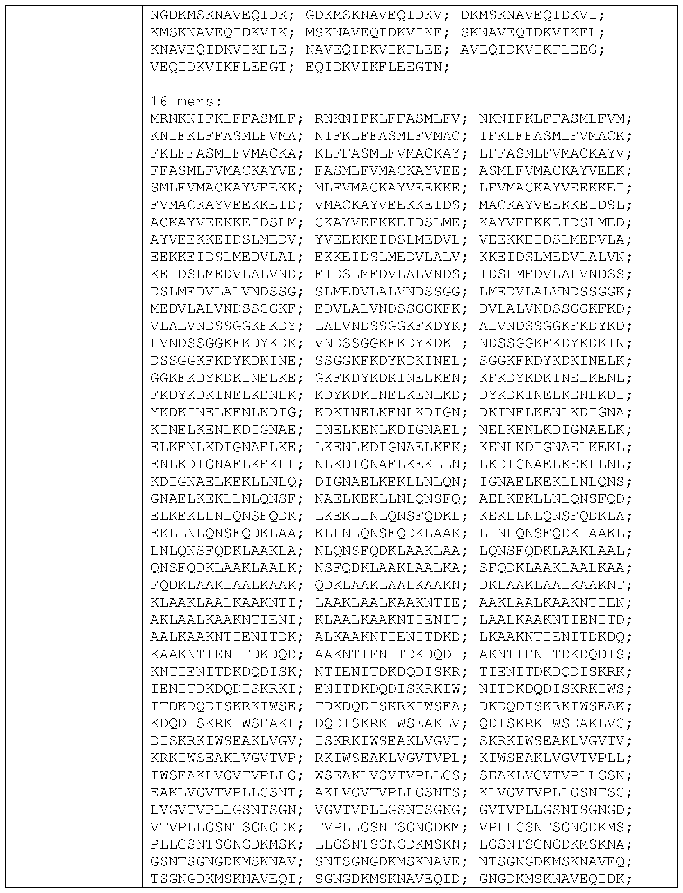

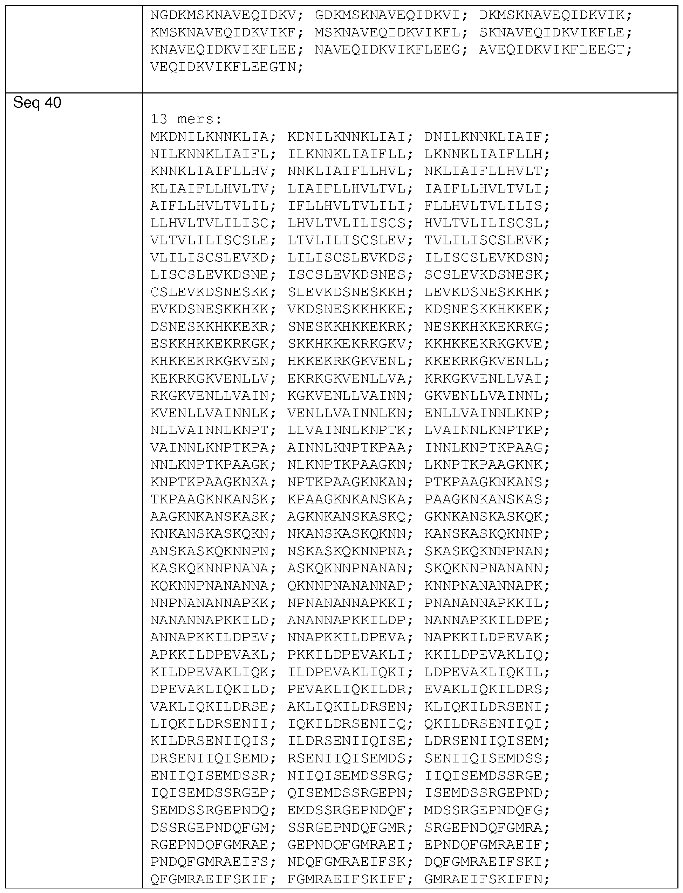

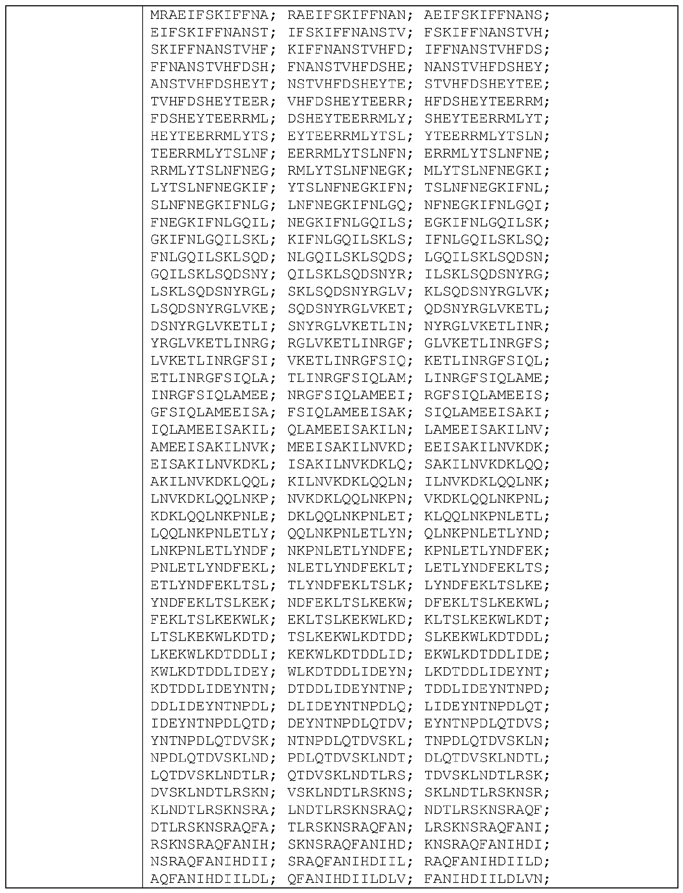

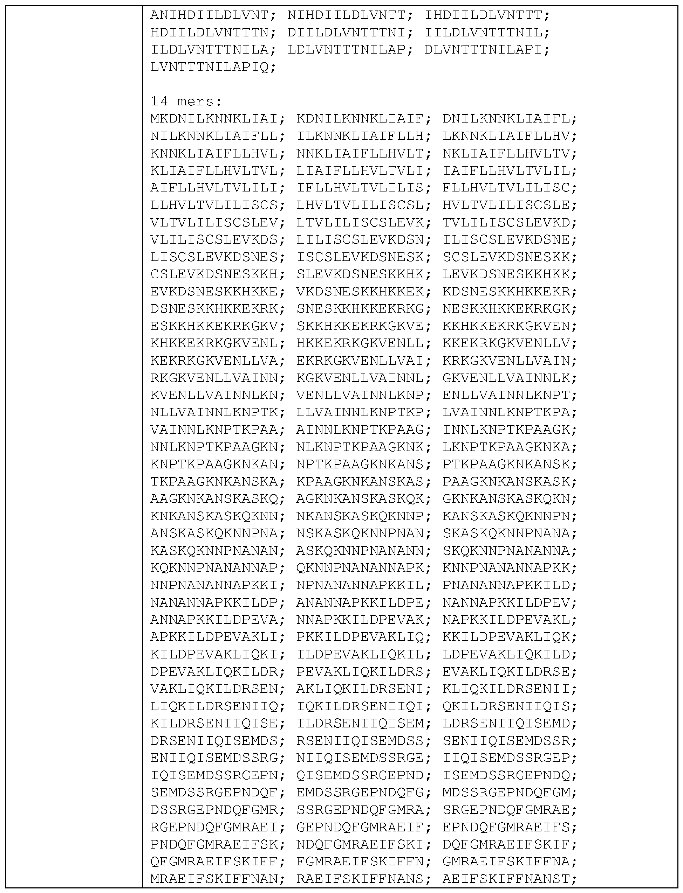

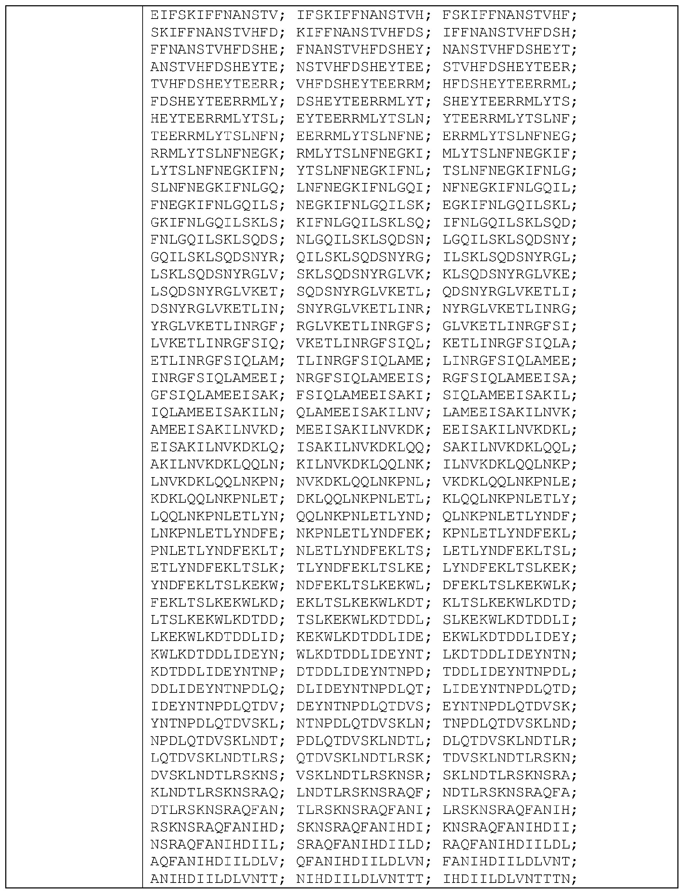

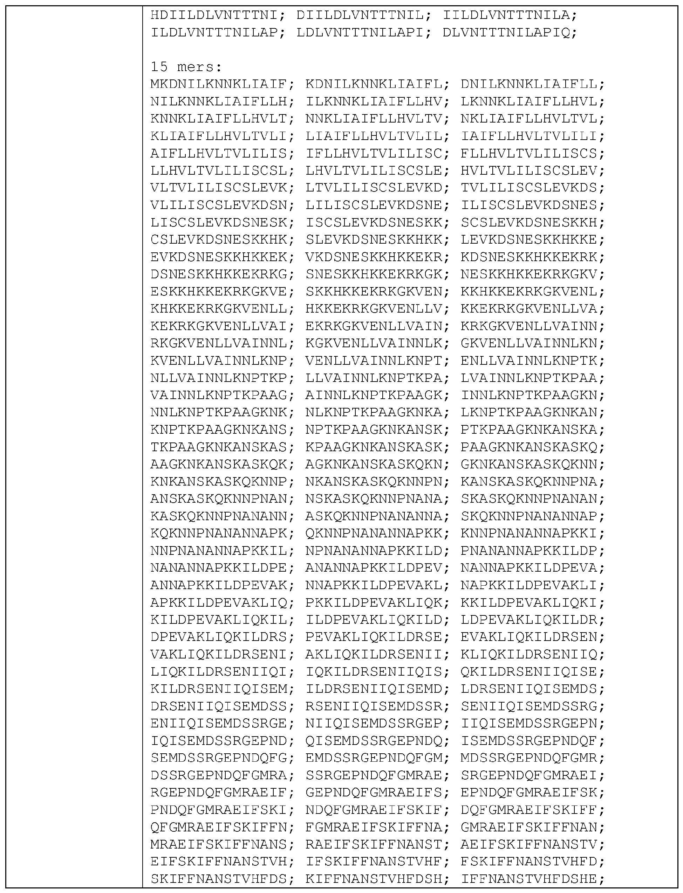

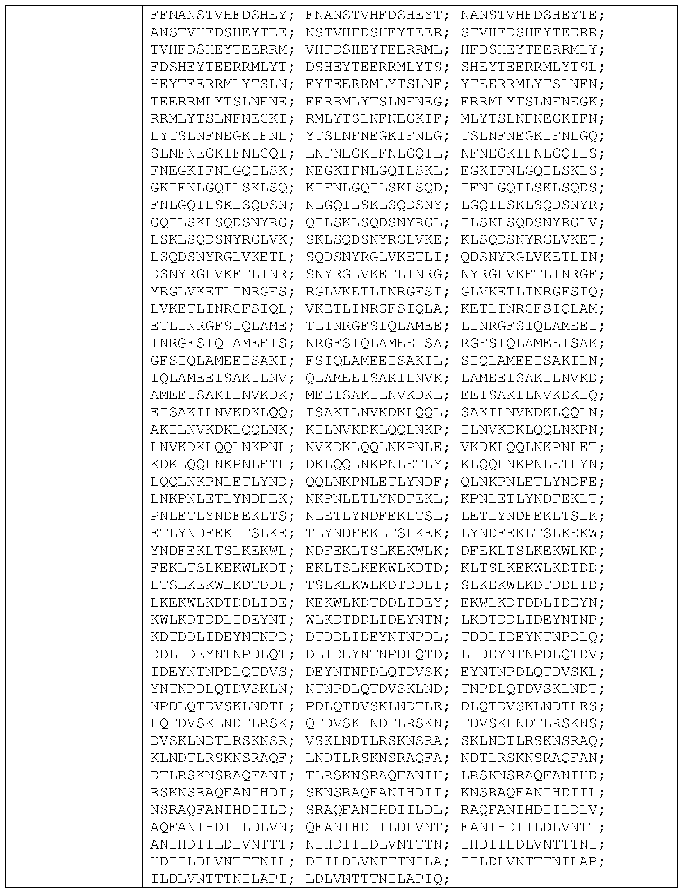

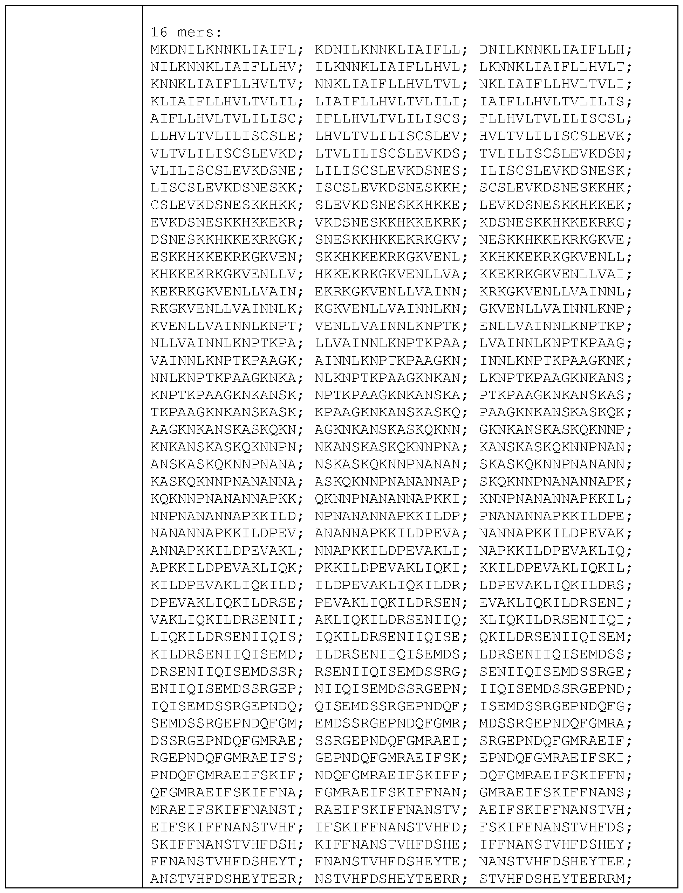

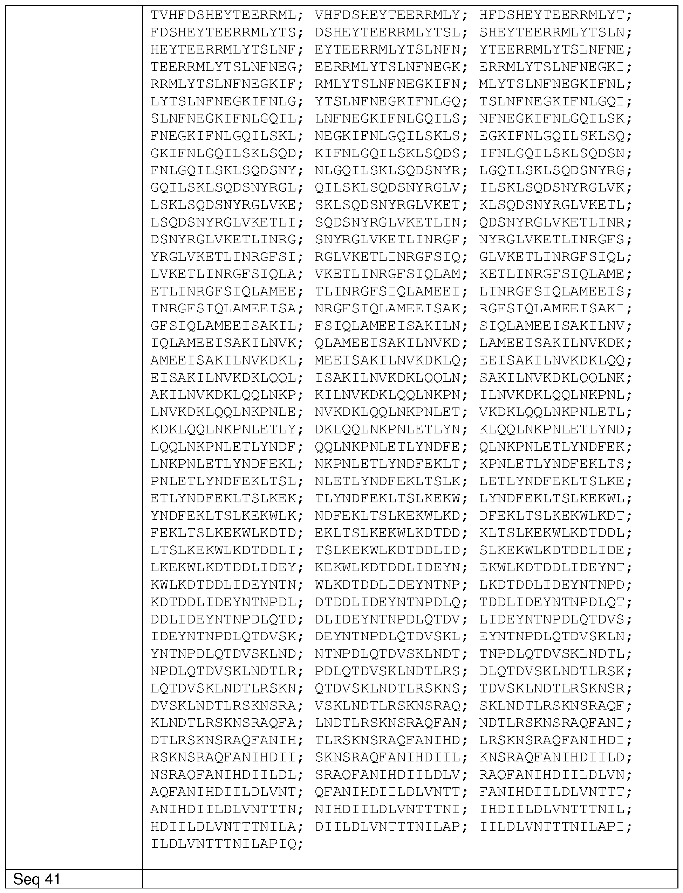

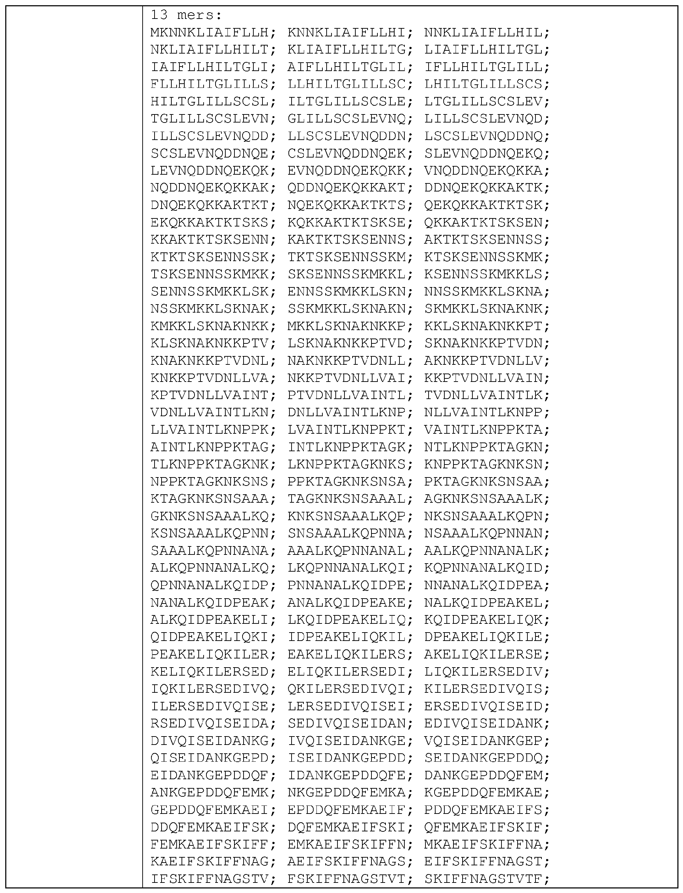

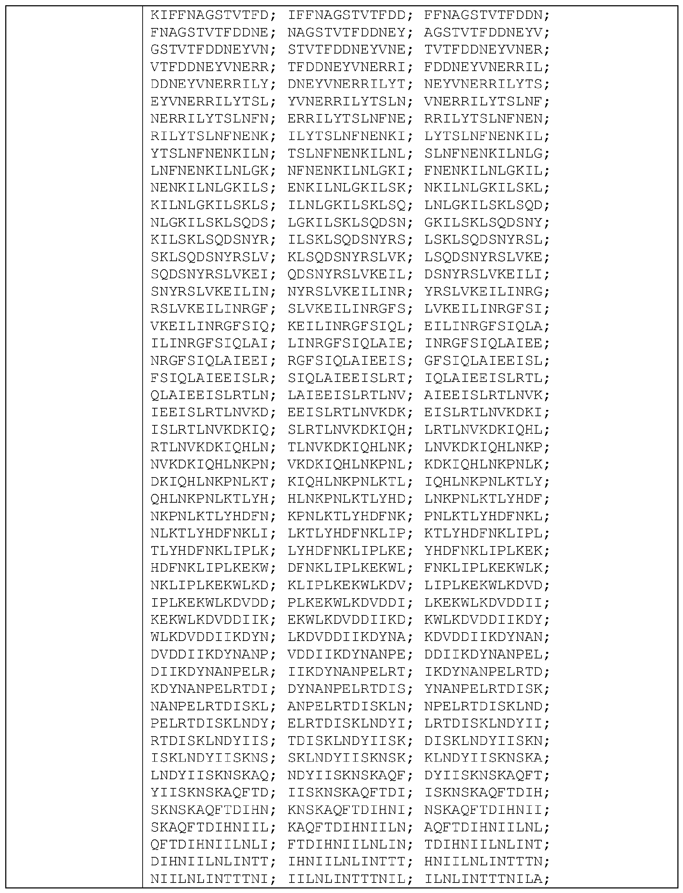

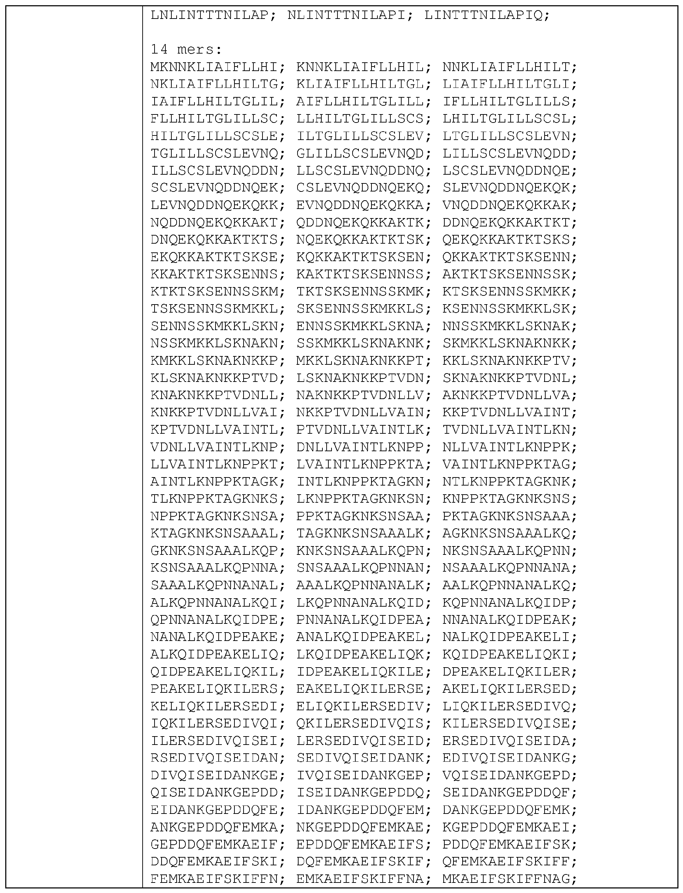

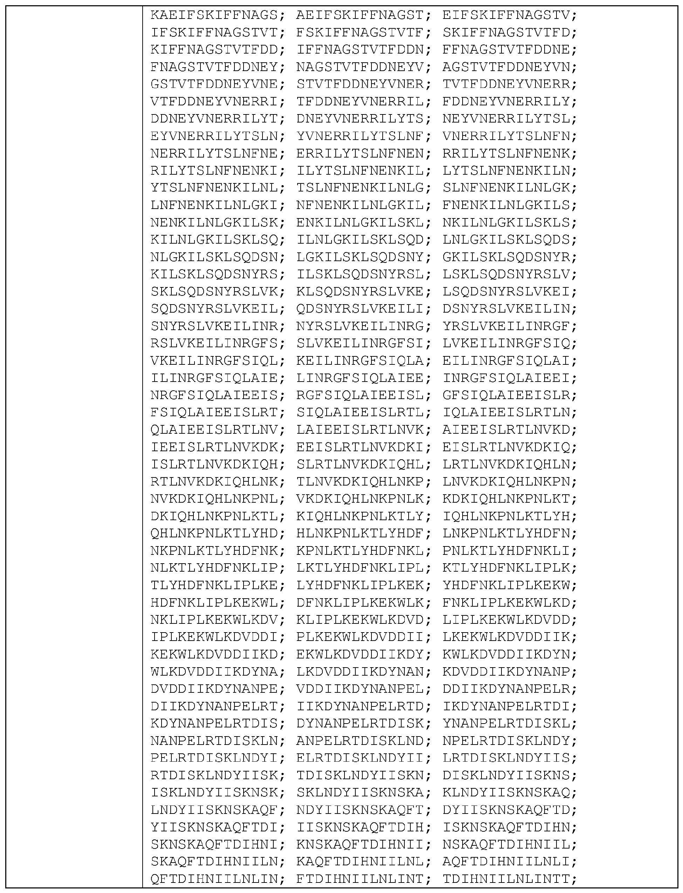

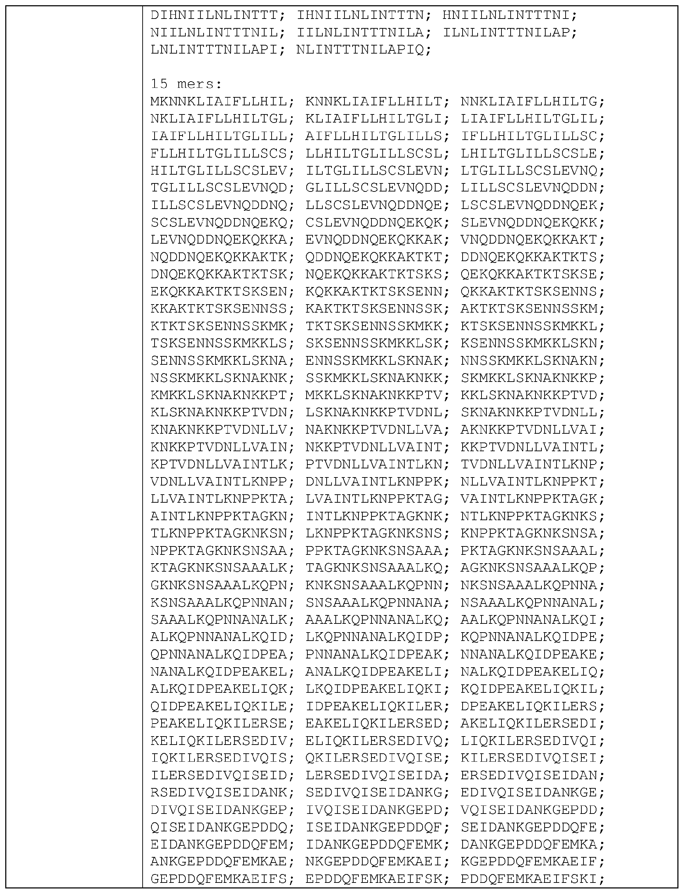

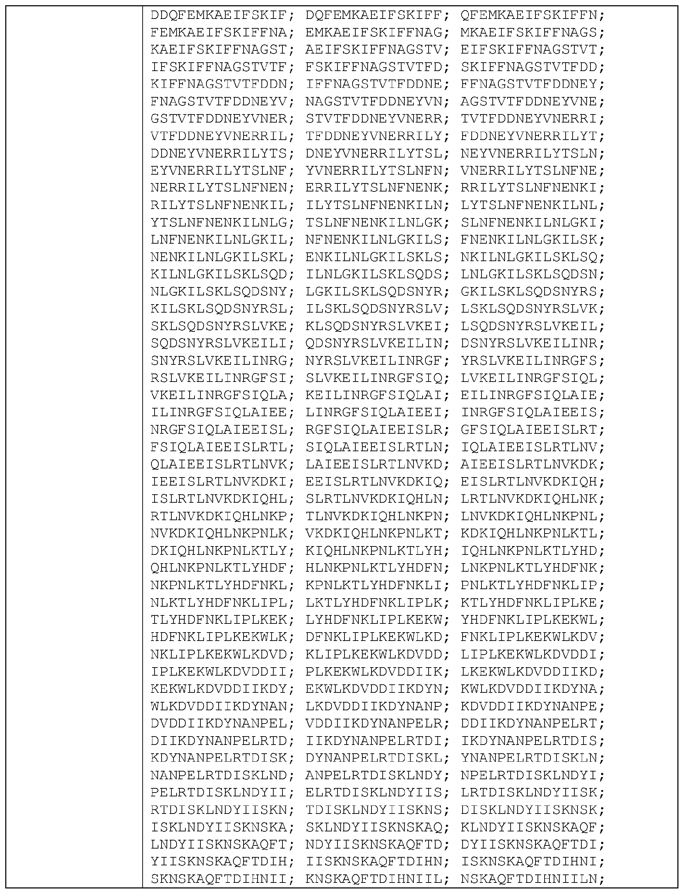

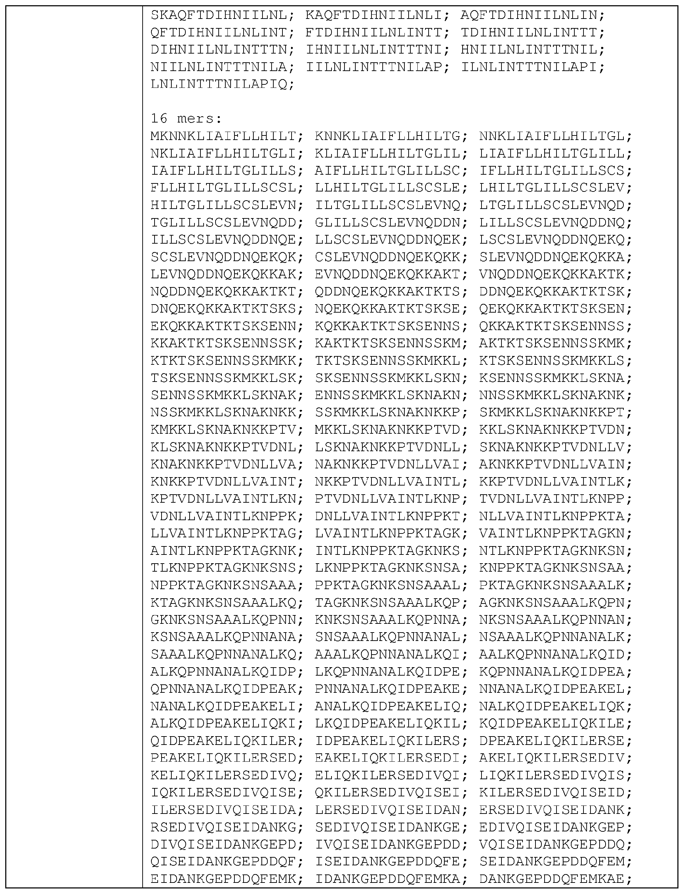

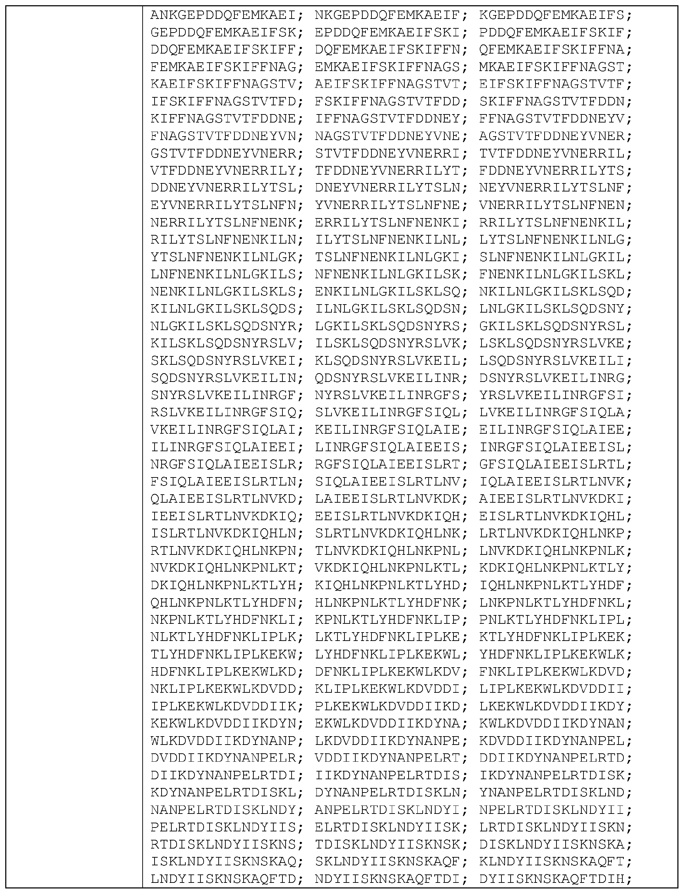

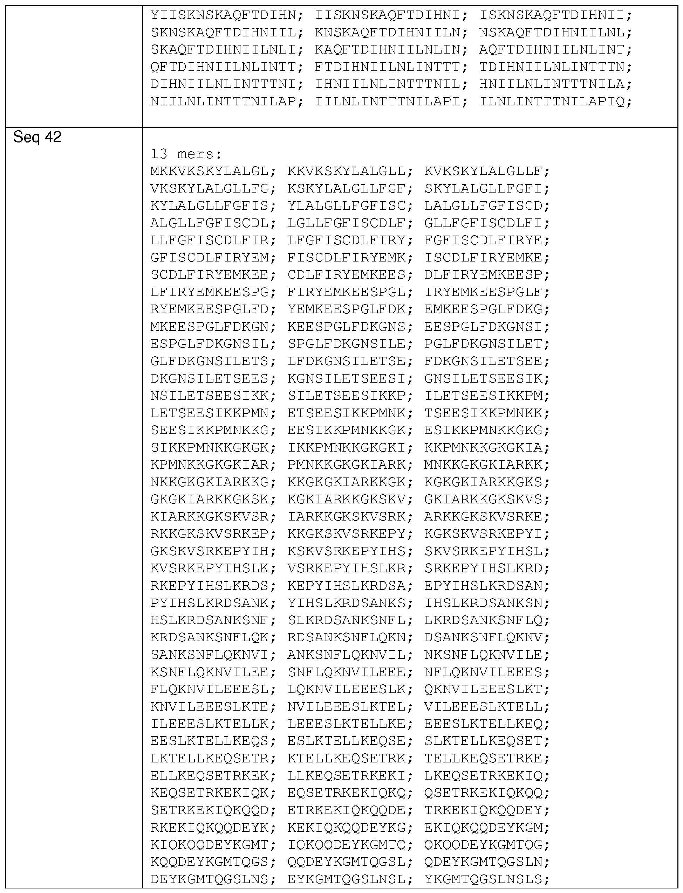

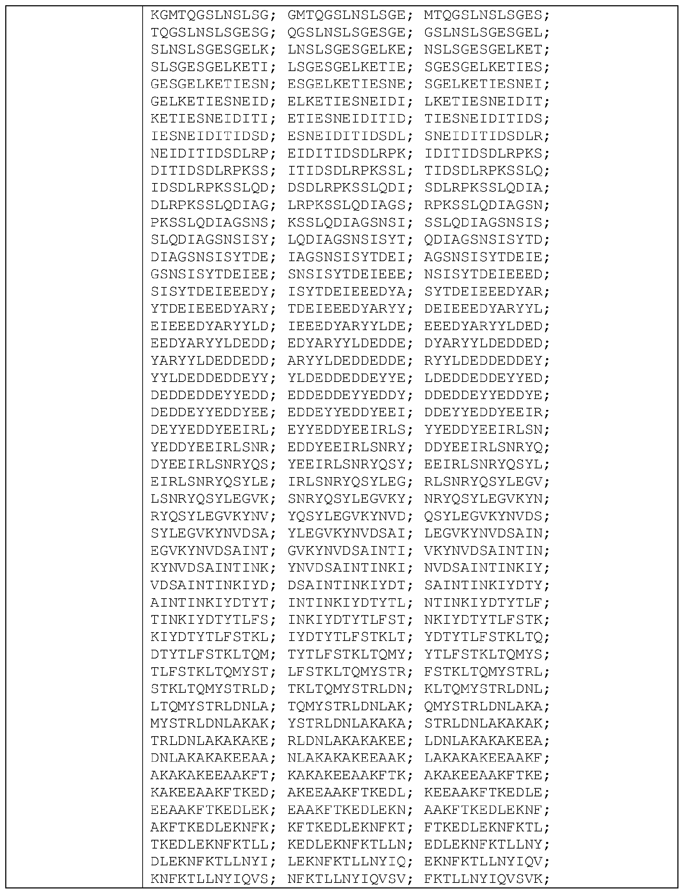

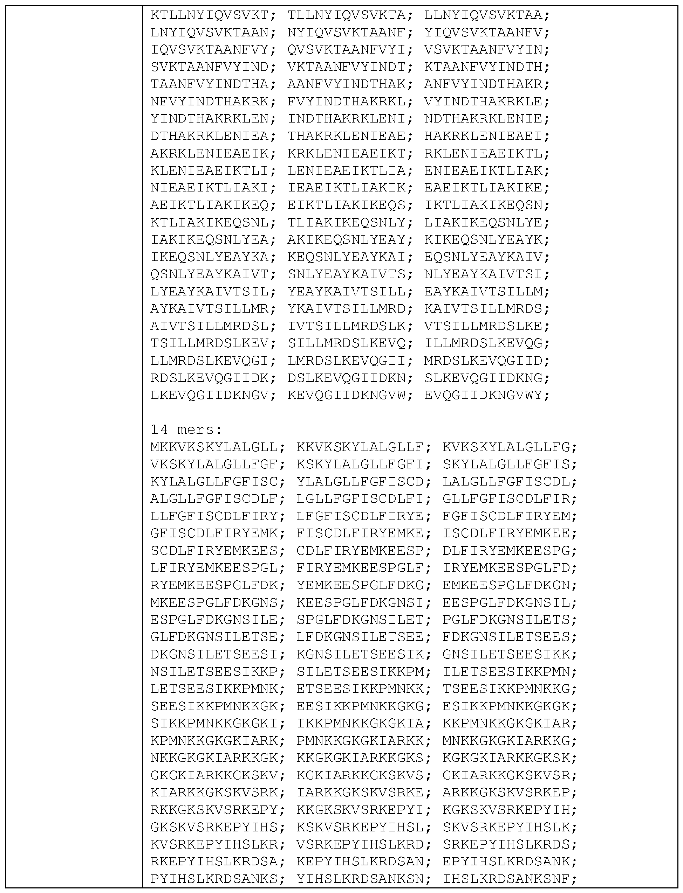

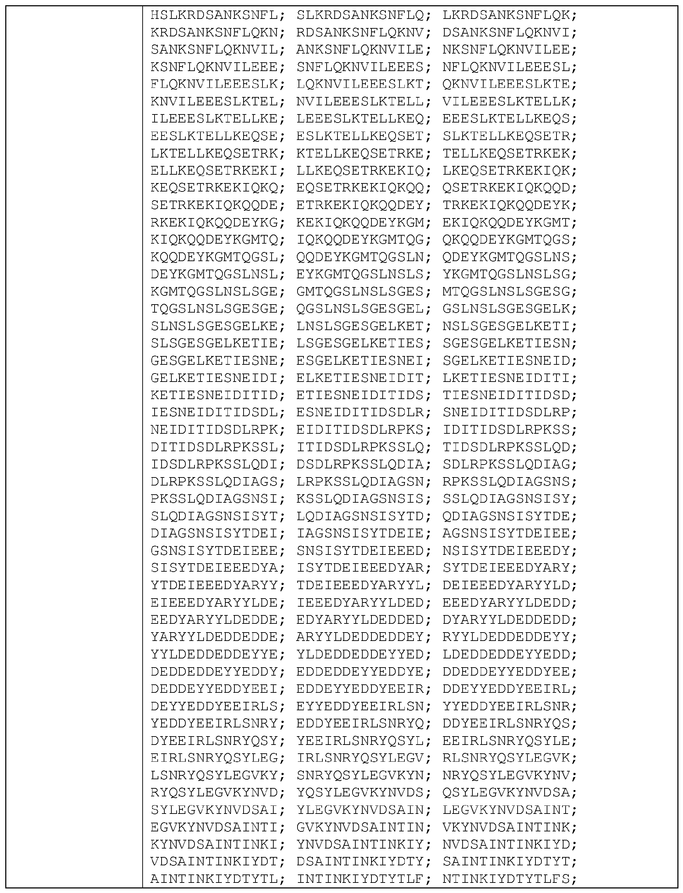

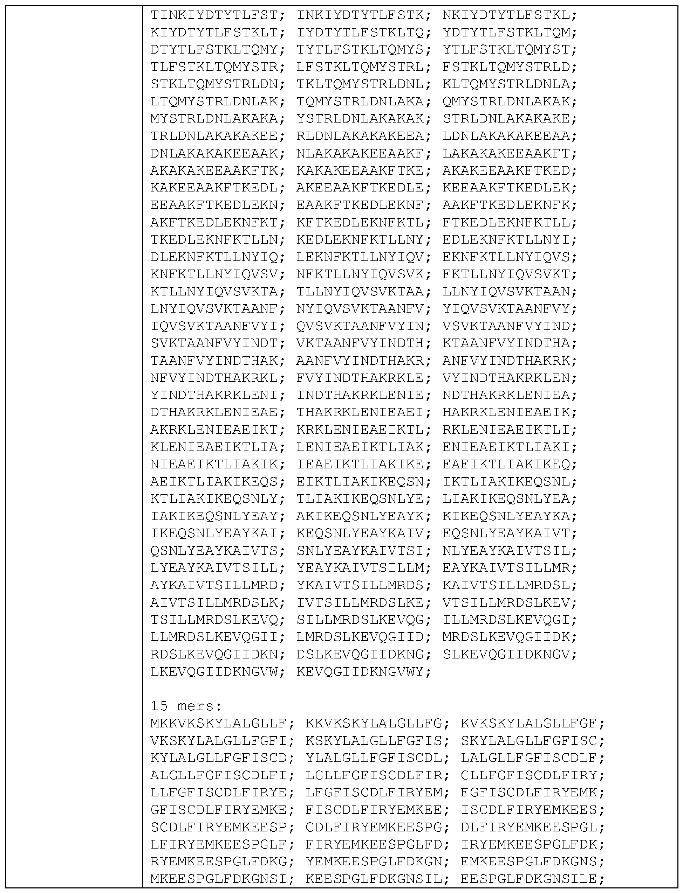

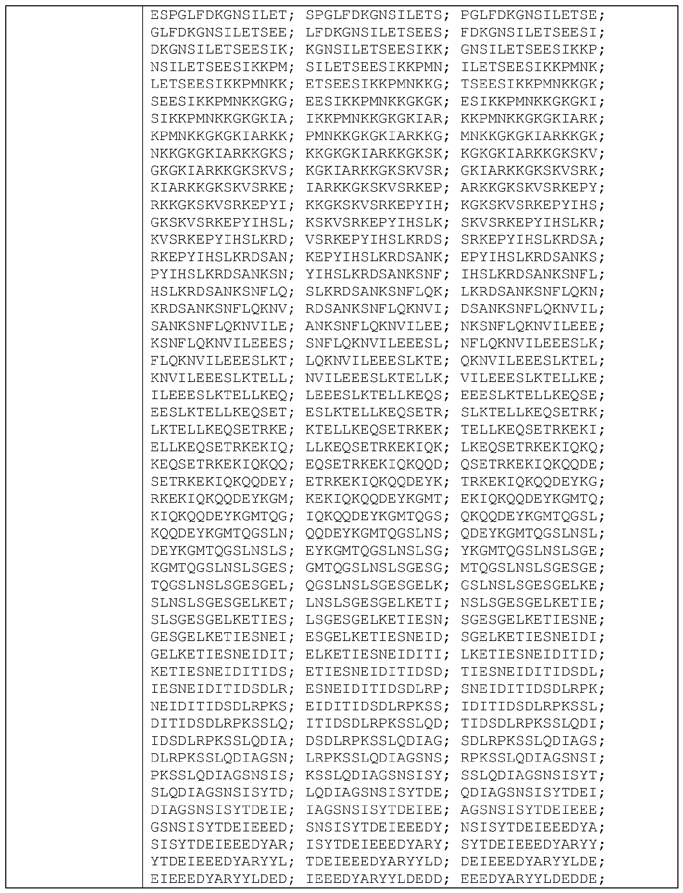

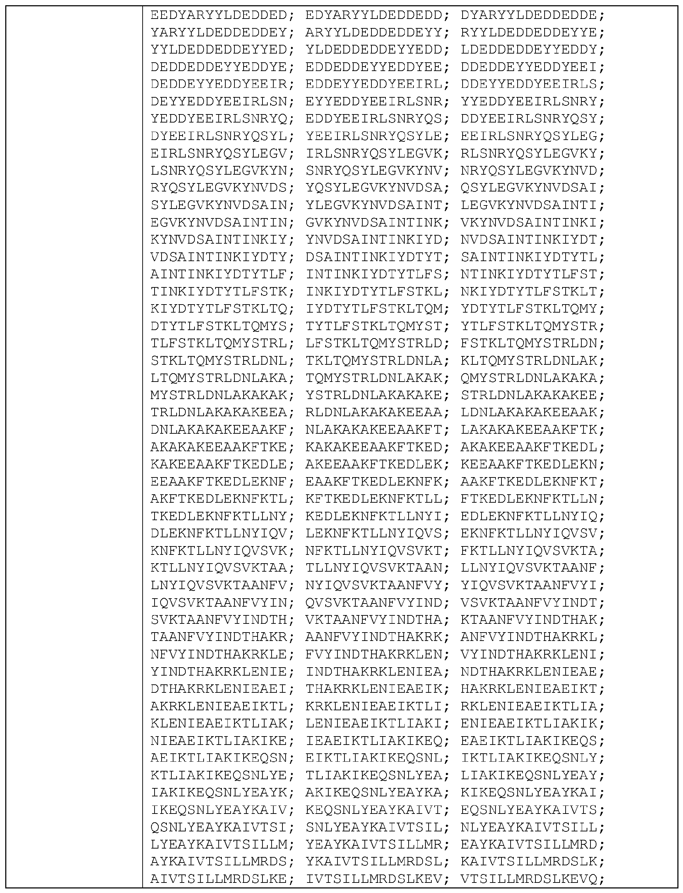

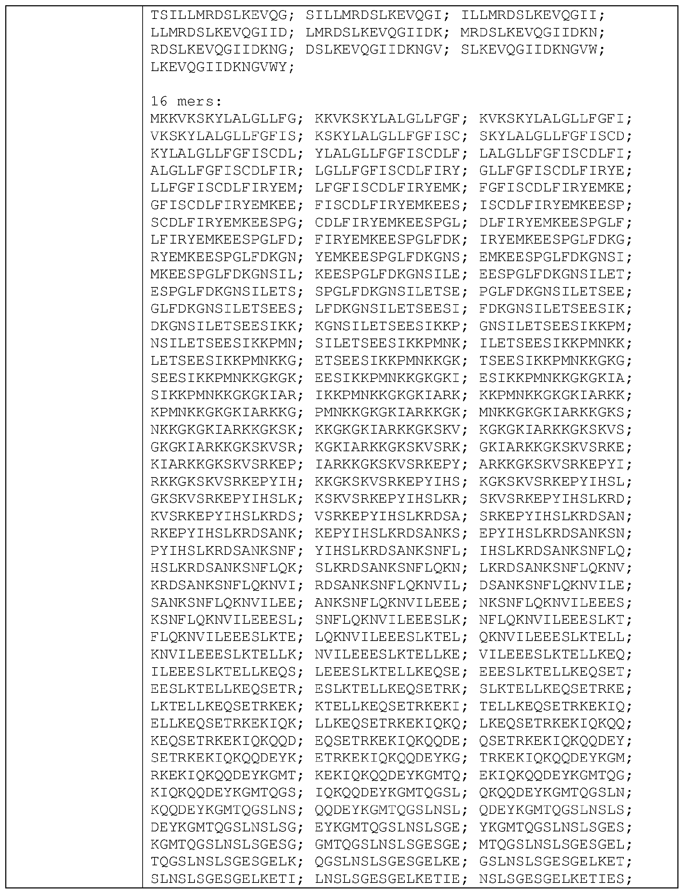

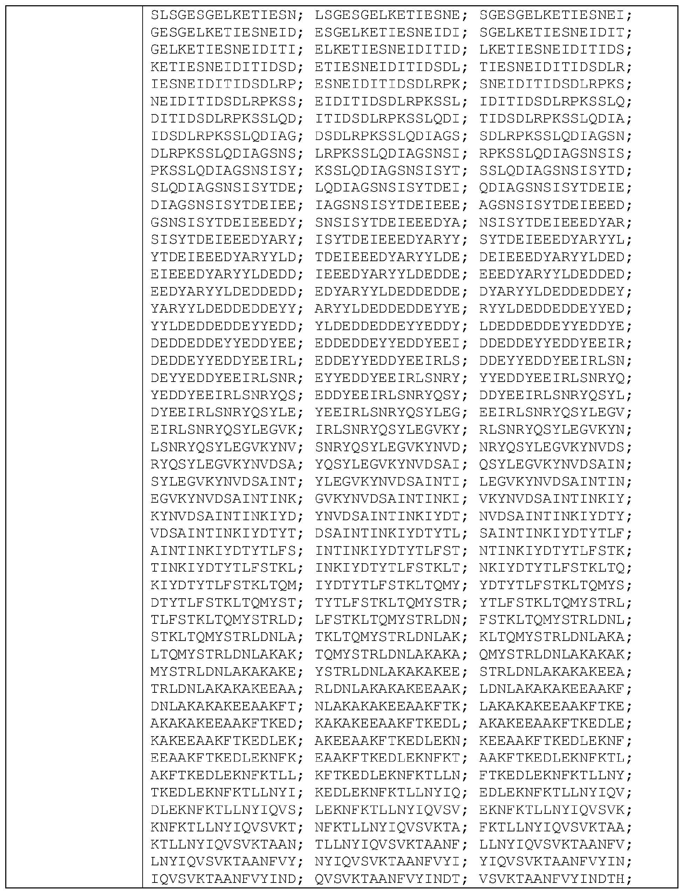

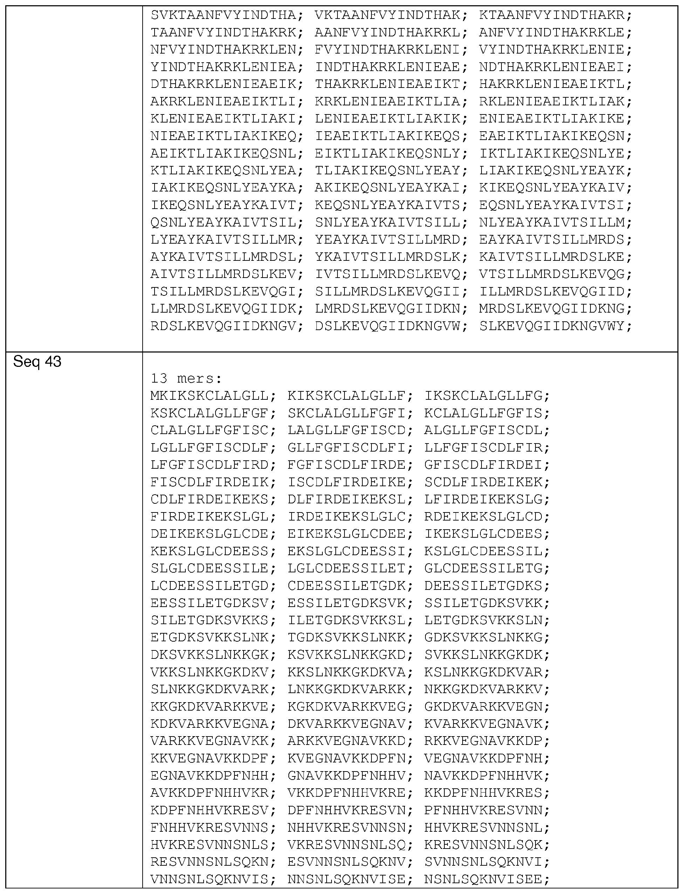

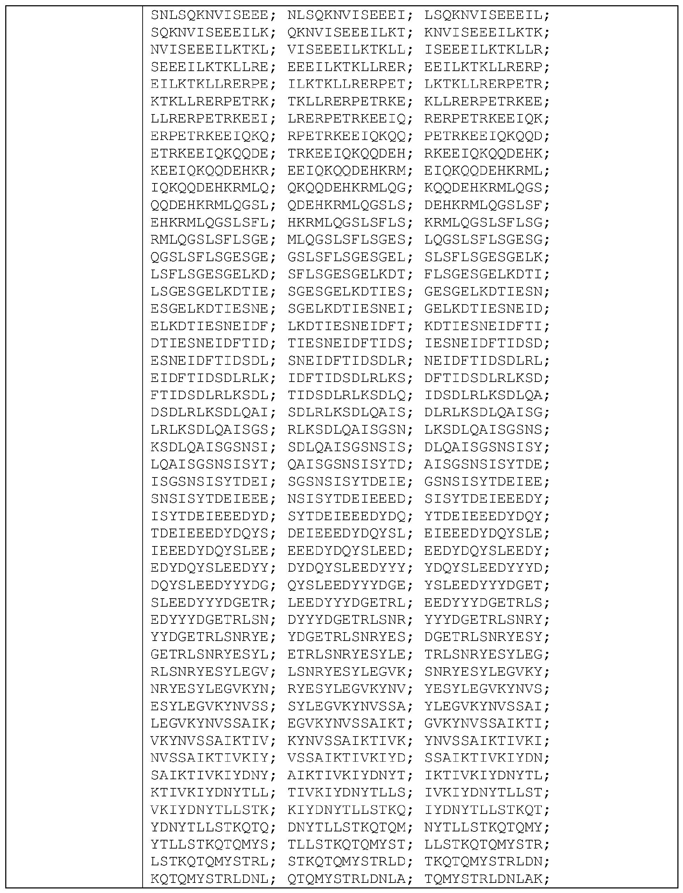

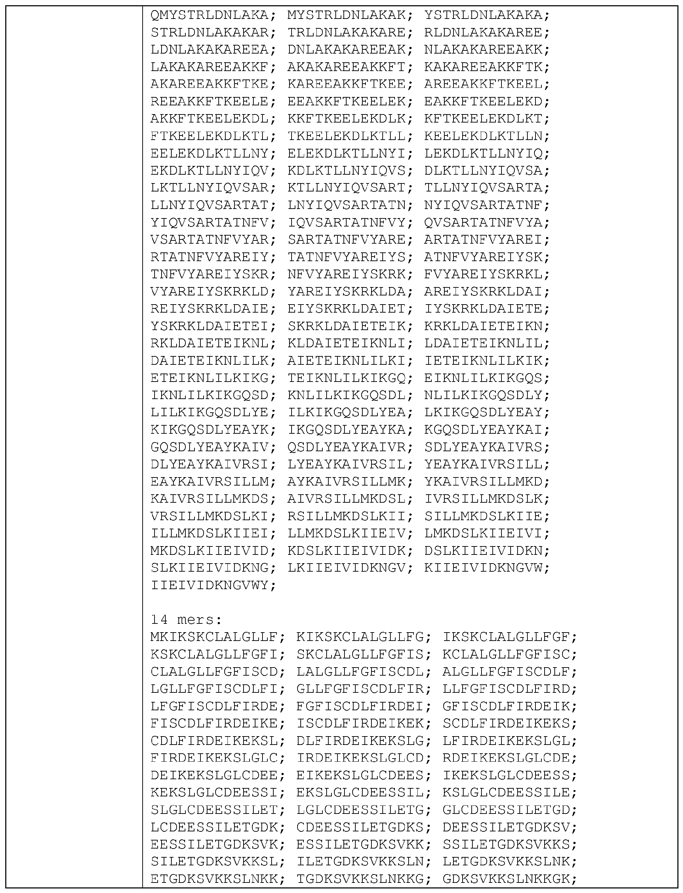

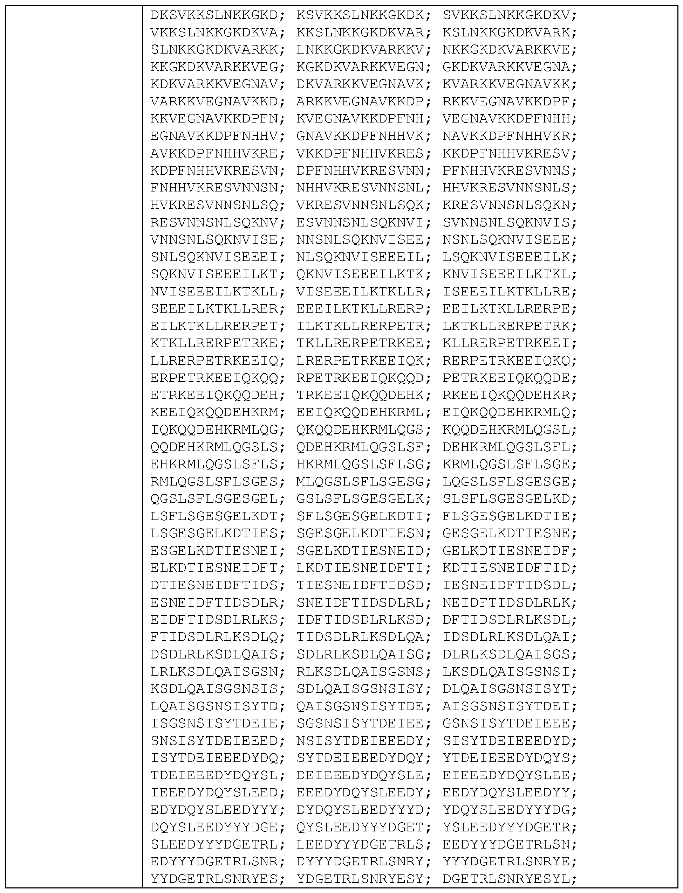

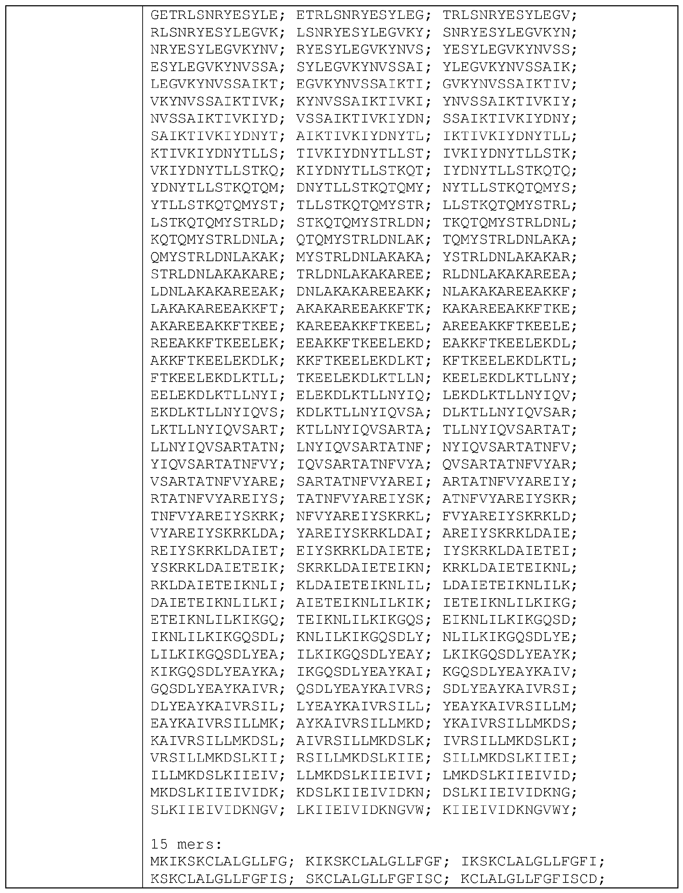

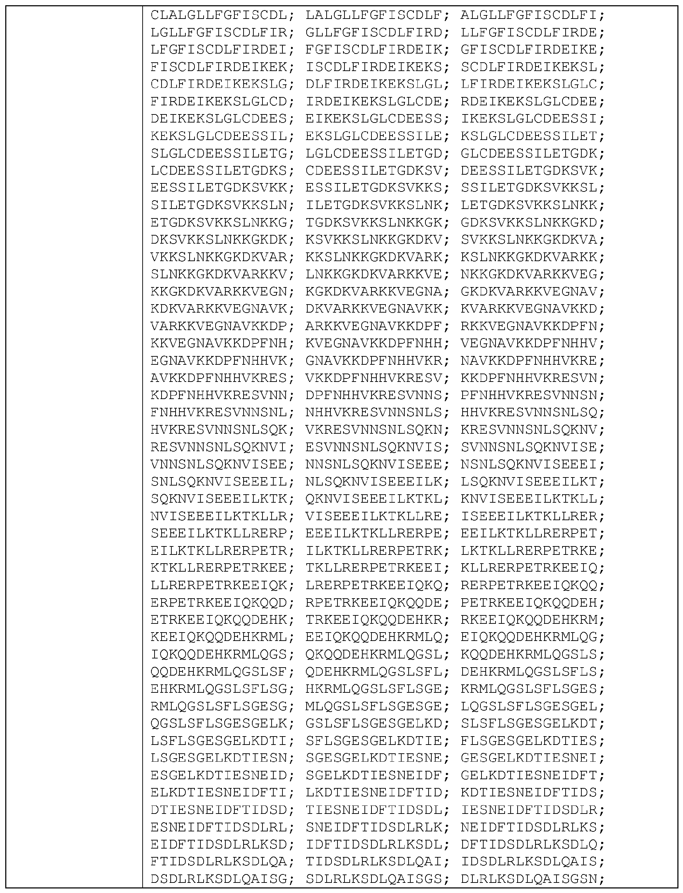

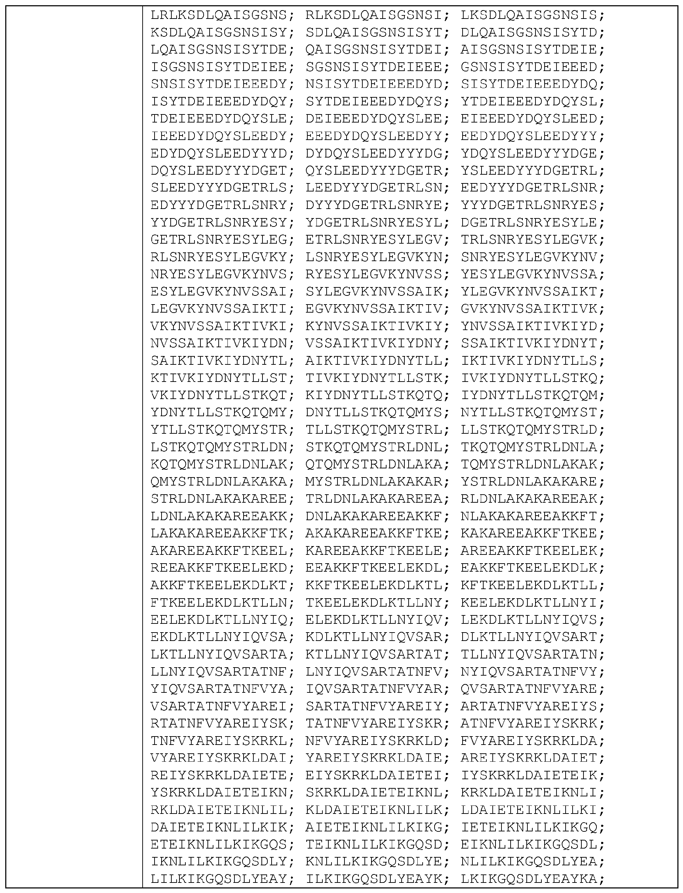

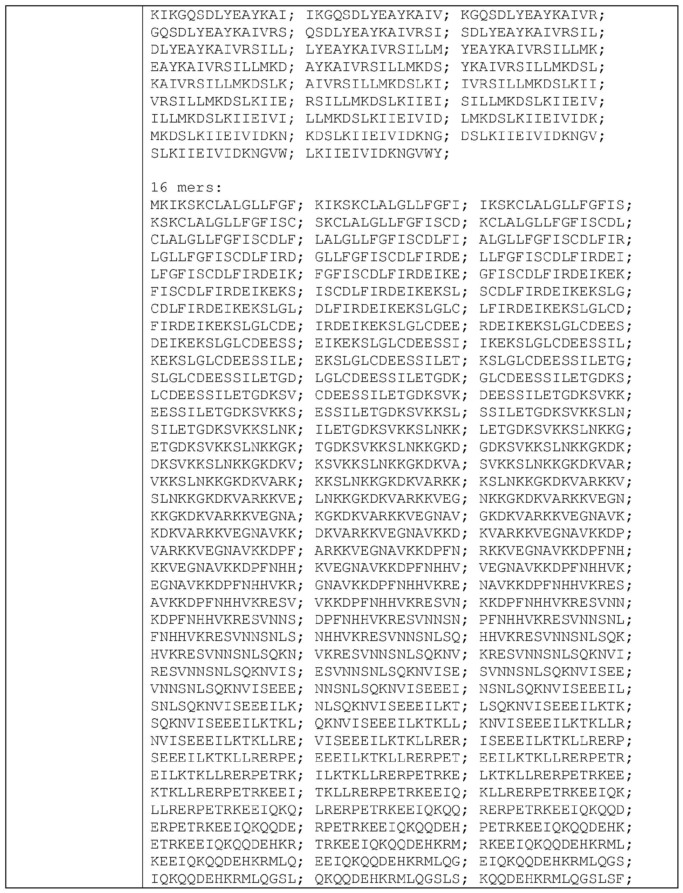

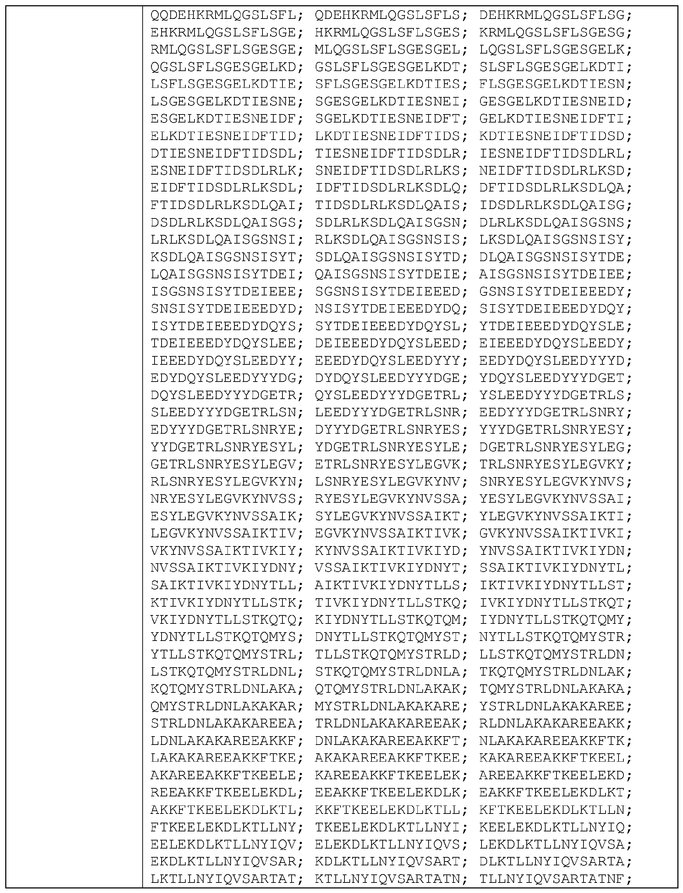

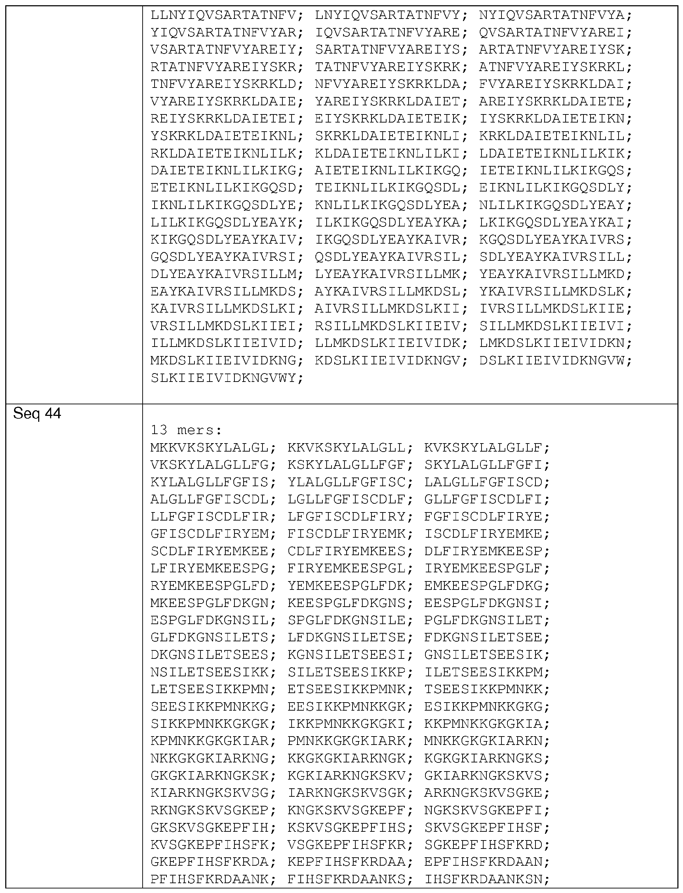

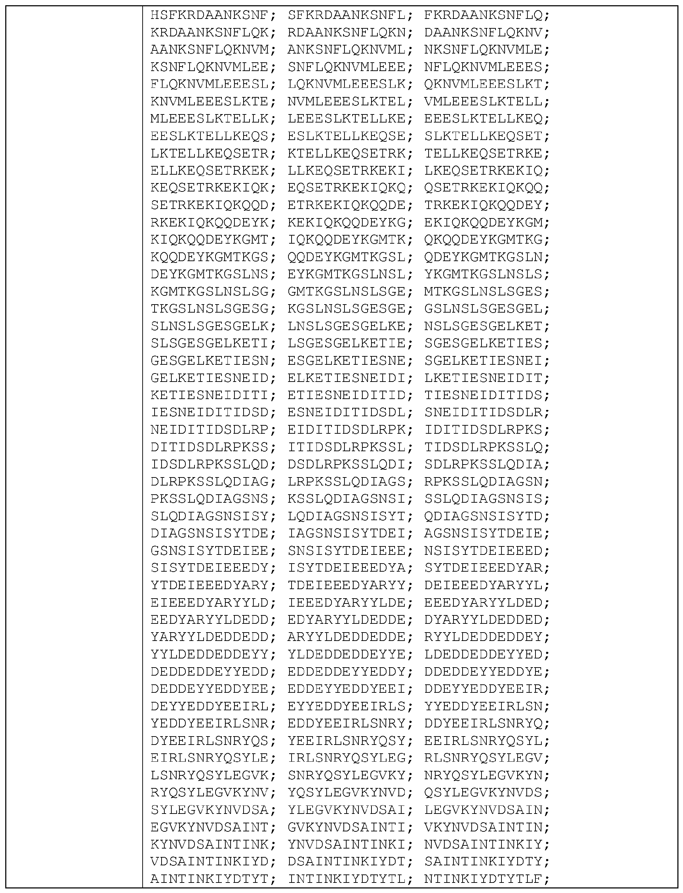

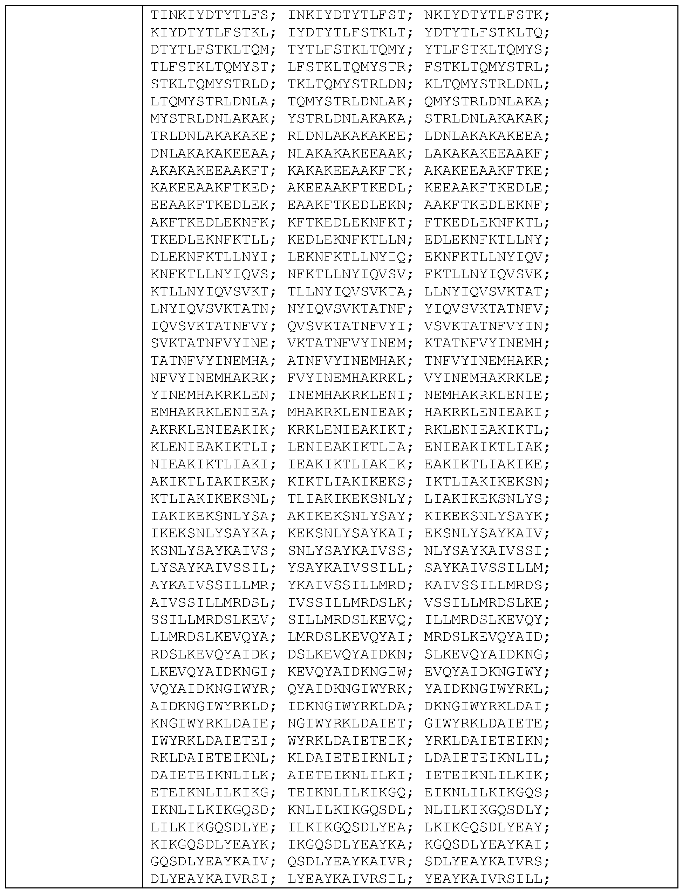

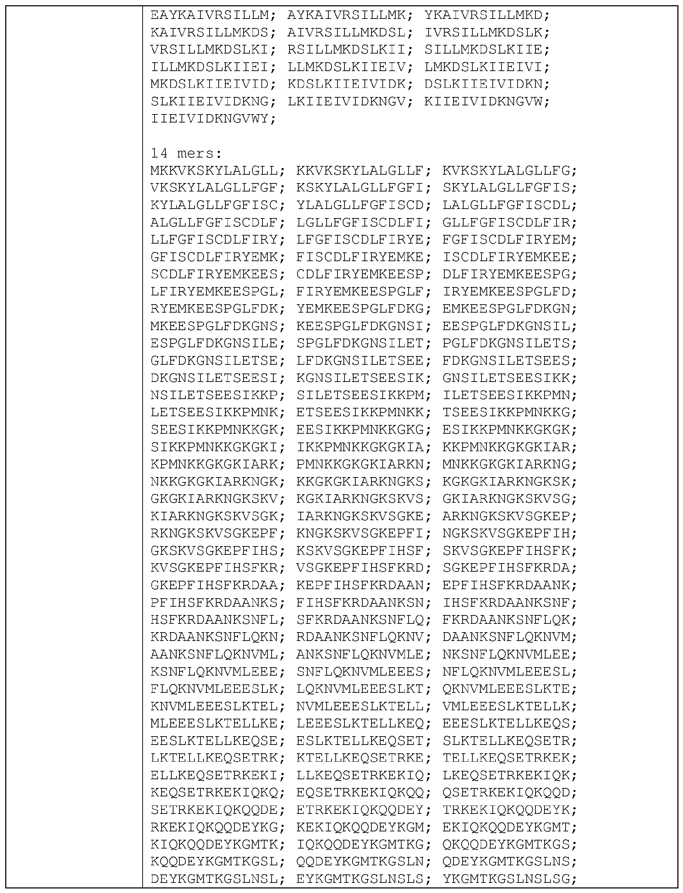

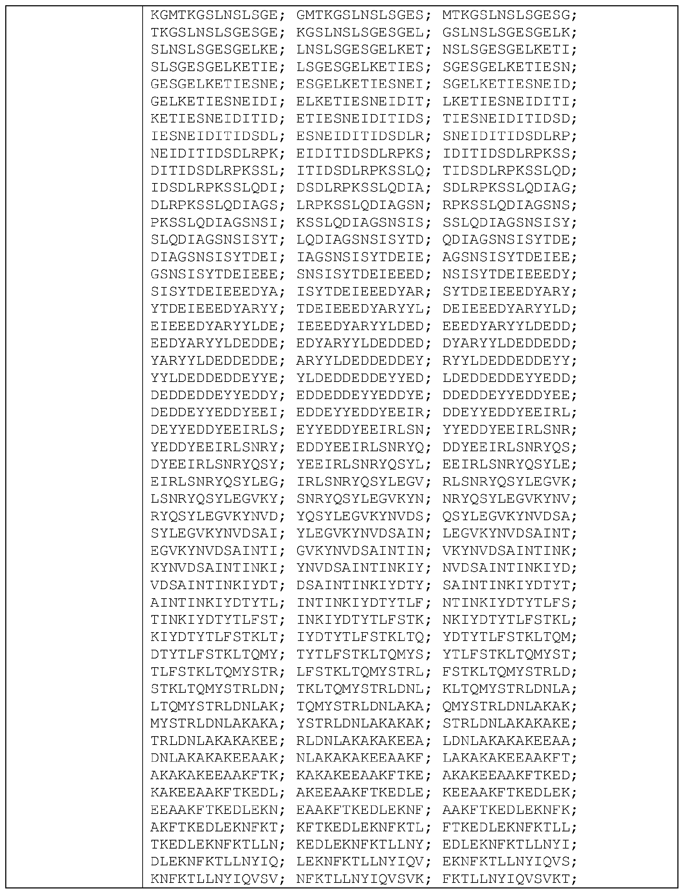

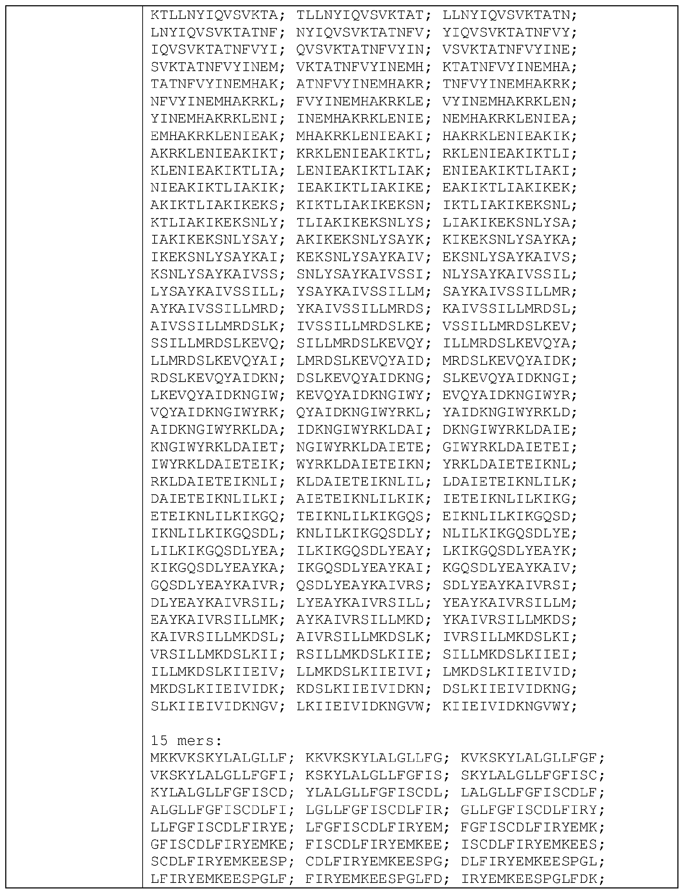

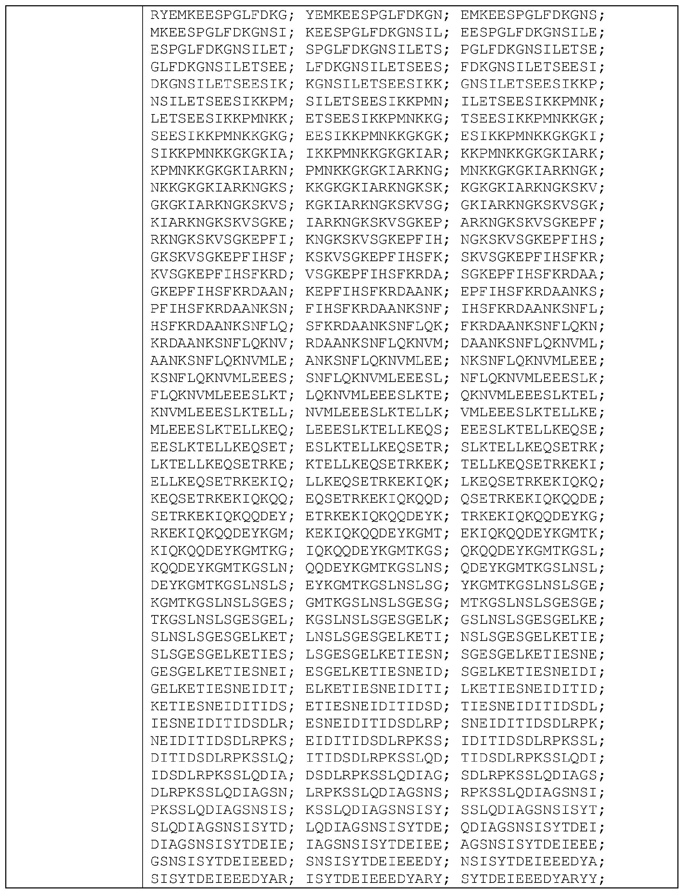

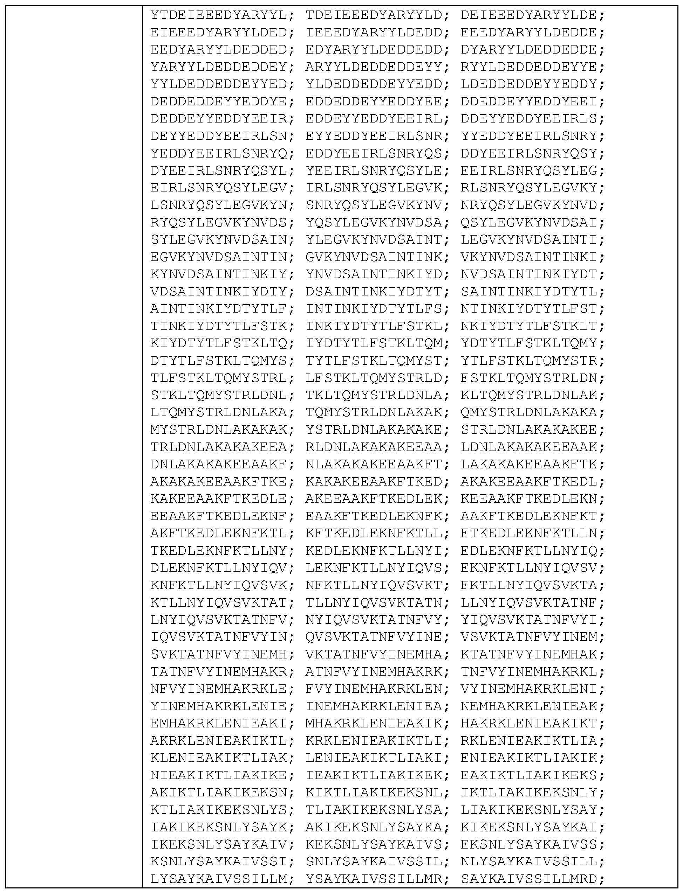

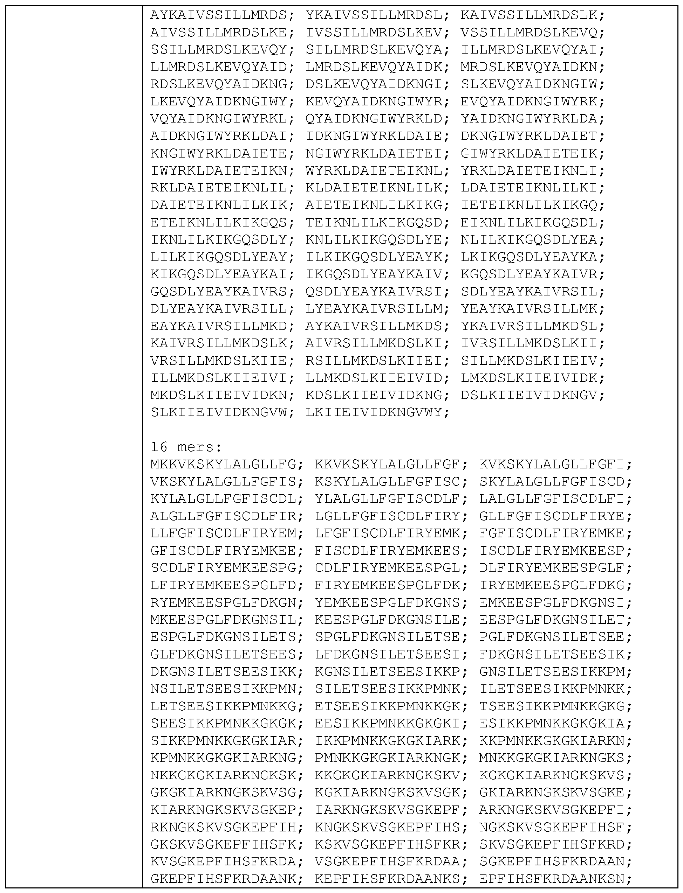

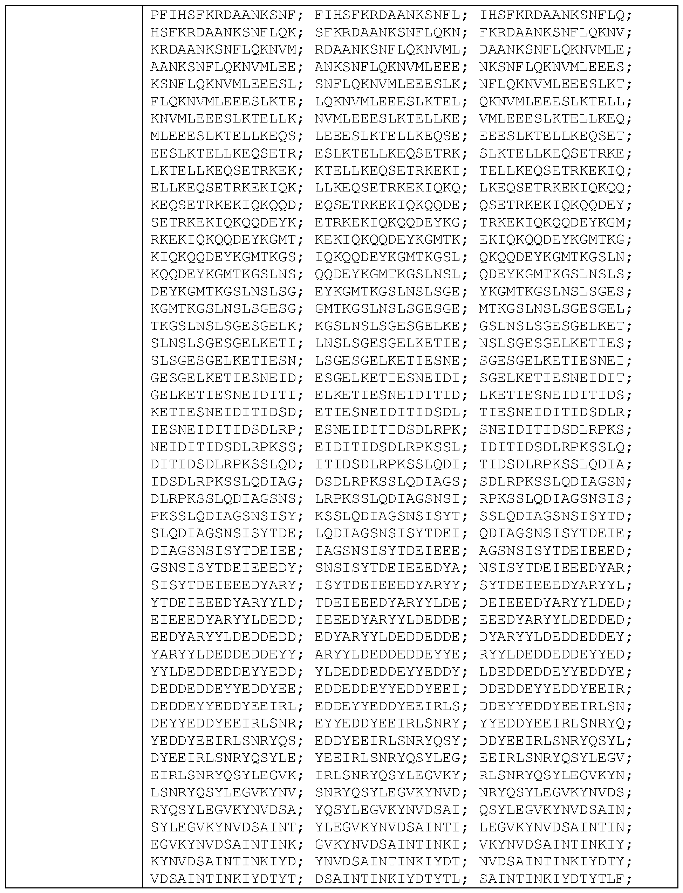

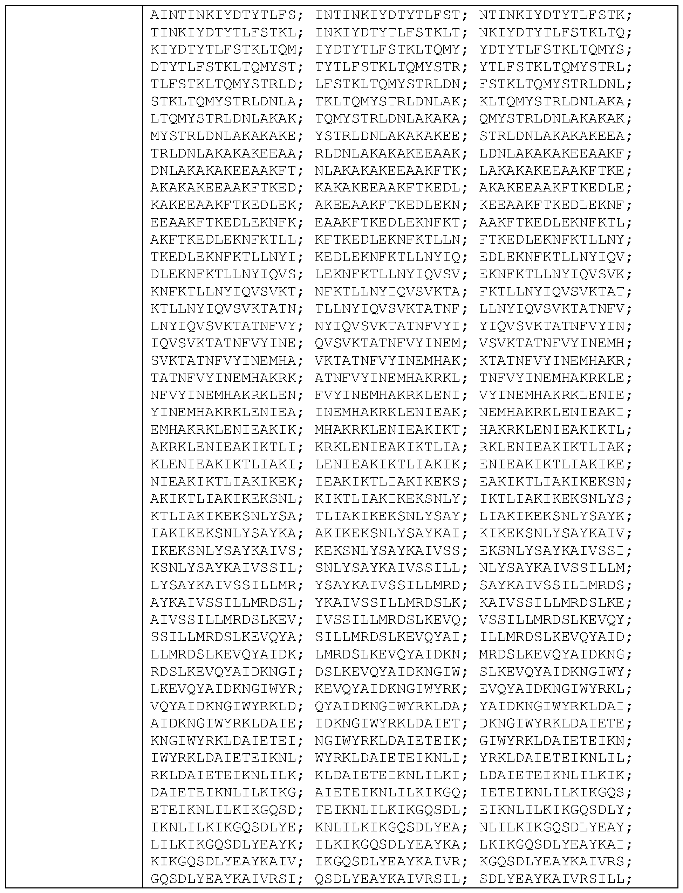

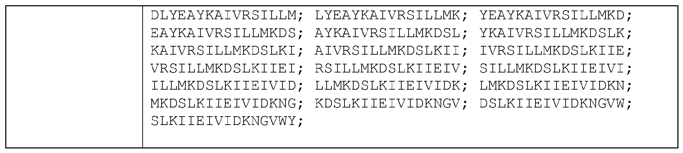

In another preferred embodiment the present disclosure relates to one or more MHC-peptide complexes or MHC multimers comprising one or more borrelia class II antigen peptide fragments selected from the peptides listed in Figure 33, Figure 34, Figure 39, Figure 40, Figure 41, Figure 42, Table A, Table B and Table C.

In another preferred embodiment the present disclosure relates to one or more borrelia class II antigenic peptide fragments selected from the peptides listed in Figure 33, Figure 34, Figure 39, Figure 40, Figure 41, Figure 42, Table A, Table B and Table C.

Borrelia class II antigen peptide fragments comprising a conserved core peptide. MHC class 2 proteins typically bind peptides with a total length of 13-18 amino acids, comprising a 9'-mer core motif containing the important amino acid anchor residues. However the total length is not strictly defined, as opposed to most MHC class 1 molecules.

The putative binding peptide sequences only describe the central part of the peptide including the 9-mer core motif; in other words, the peptide sequences shown represent the core of the binding peptide with a few important flanking amino acids, which in some cases may be of considerably length generating binding peptides longer than the 13-16 amino acids.

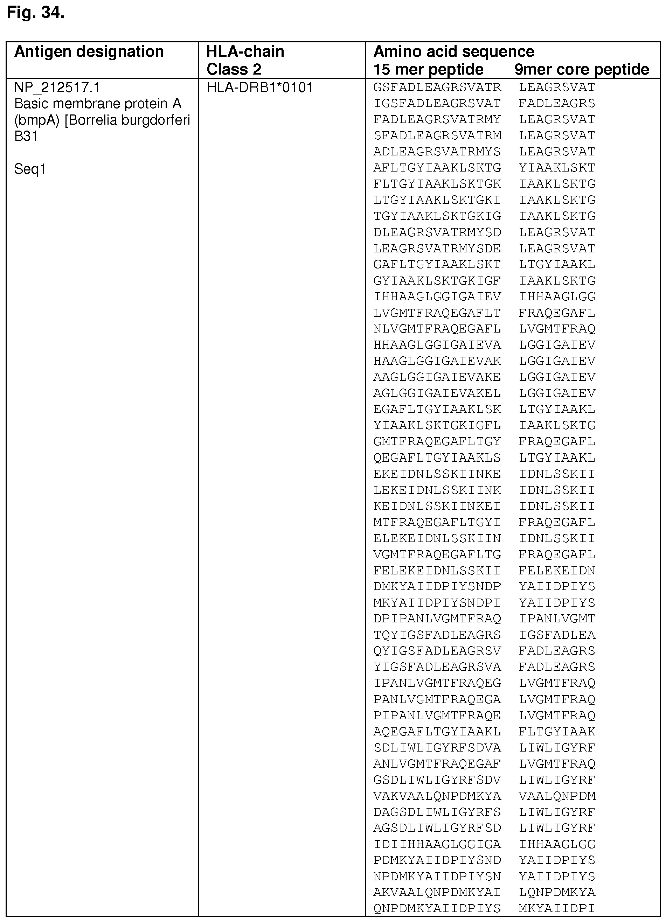

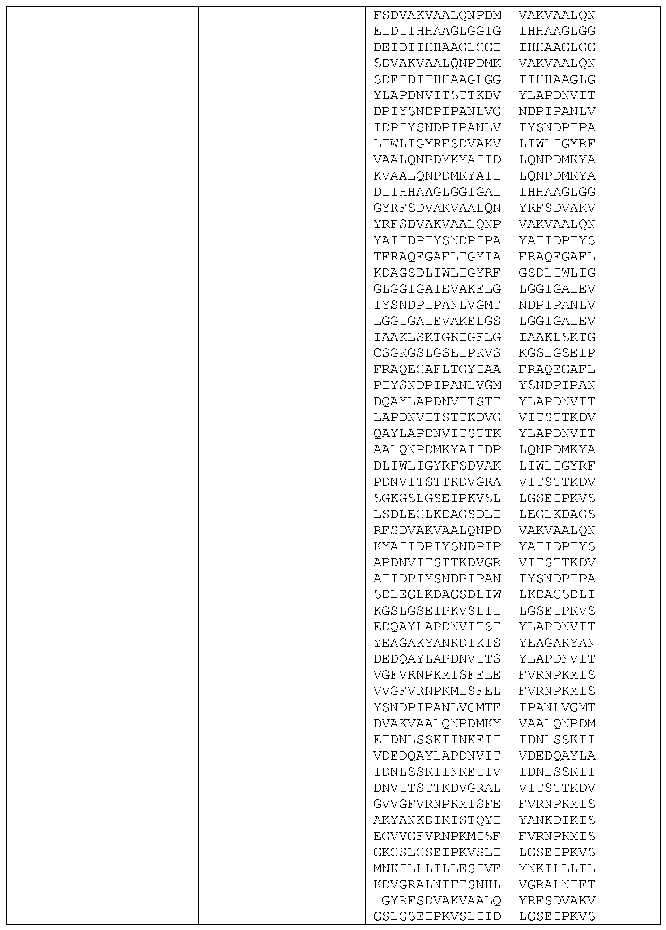

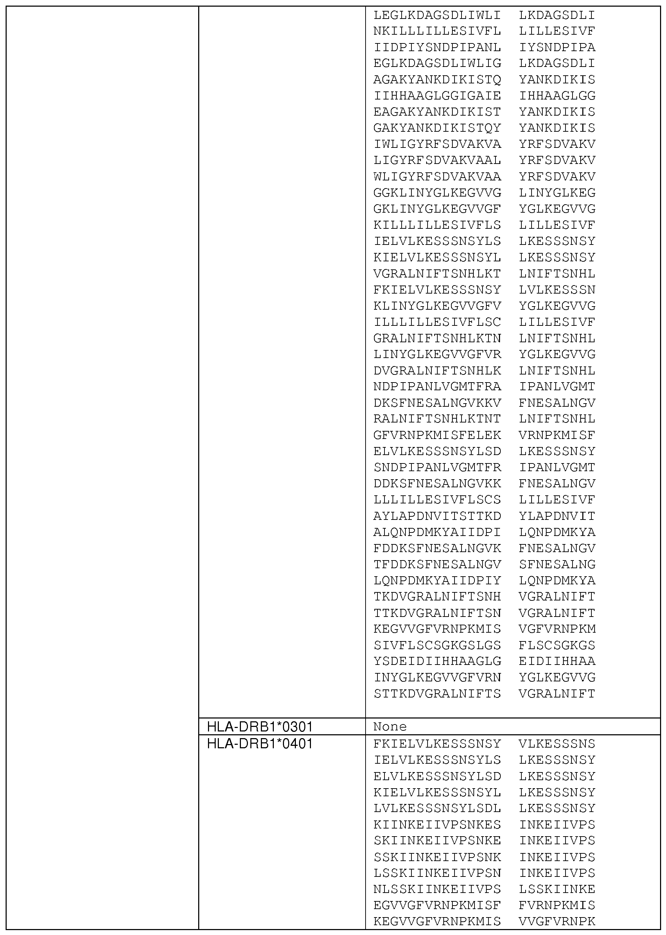

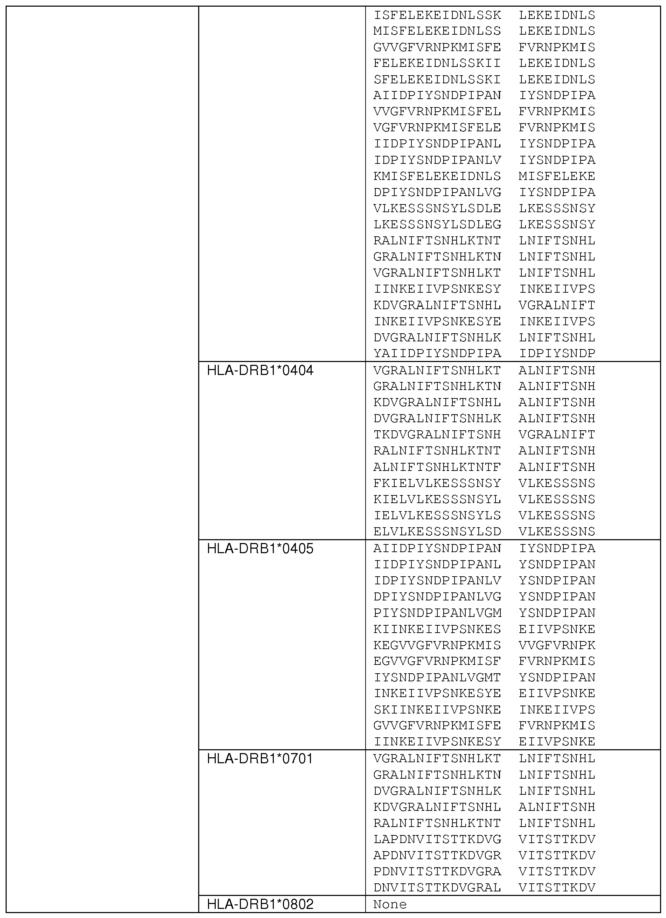

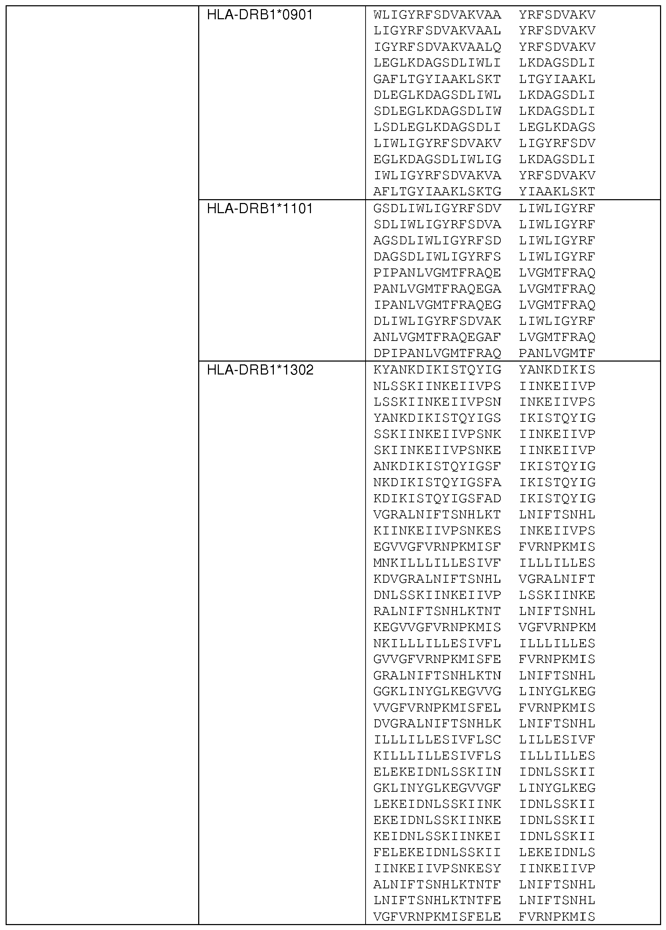

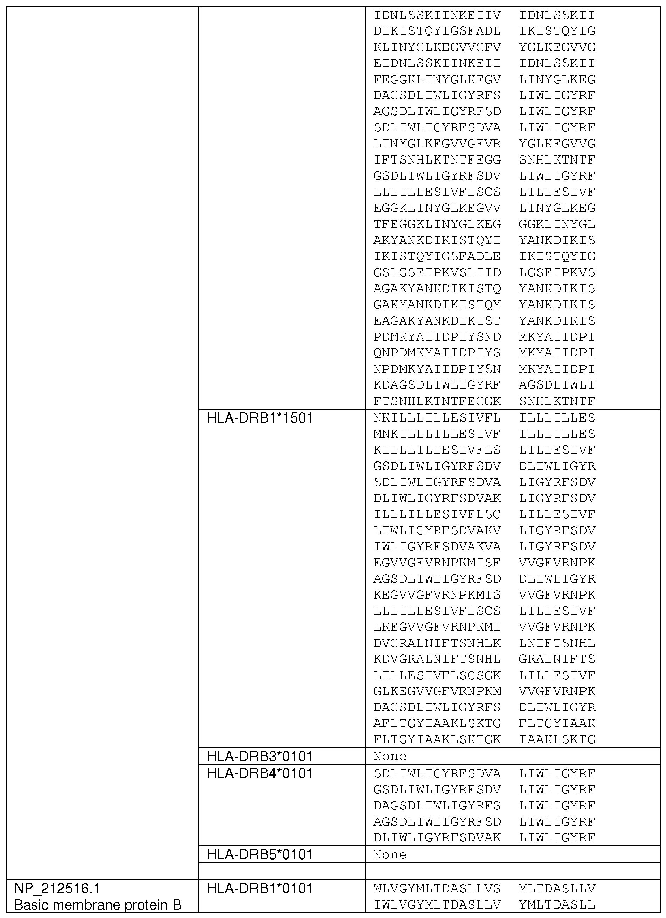

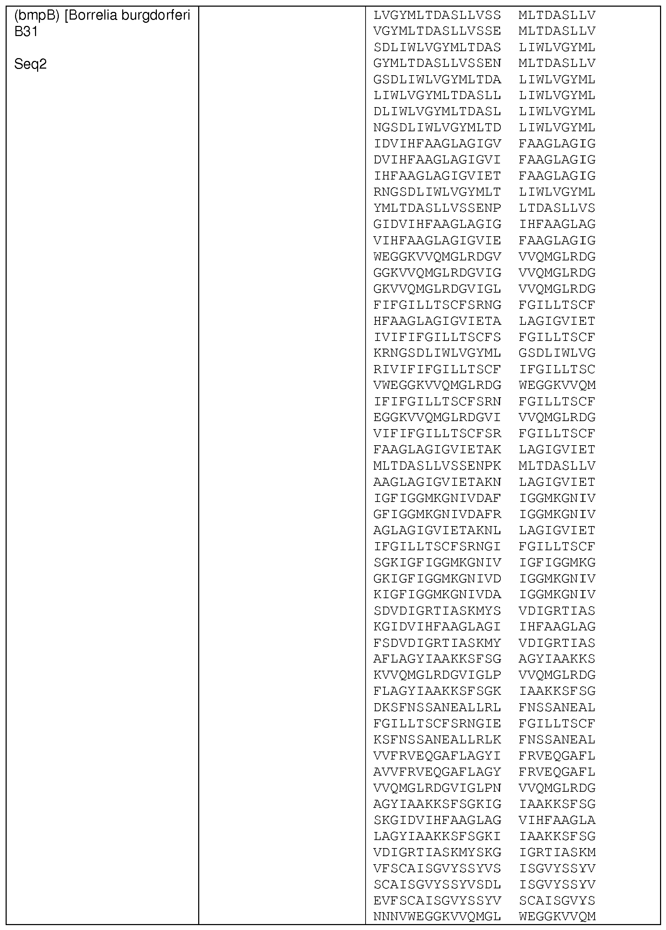

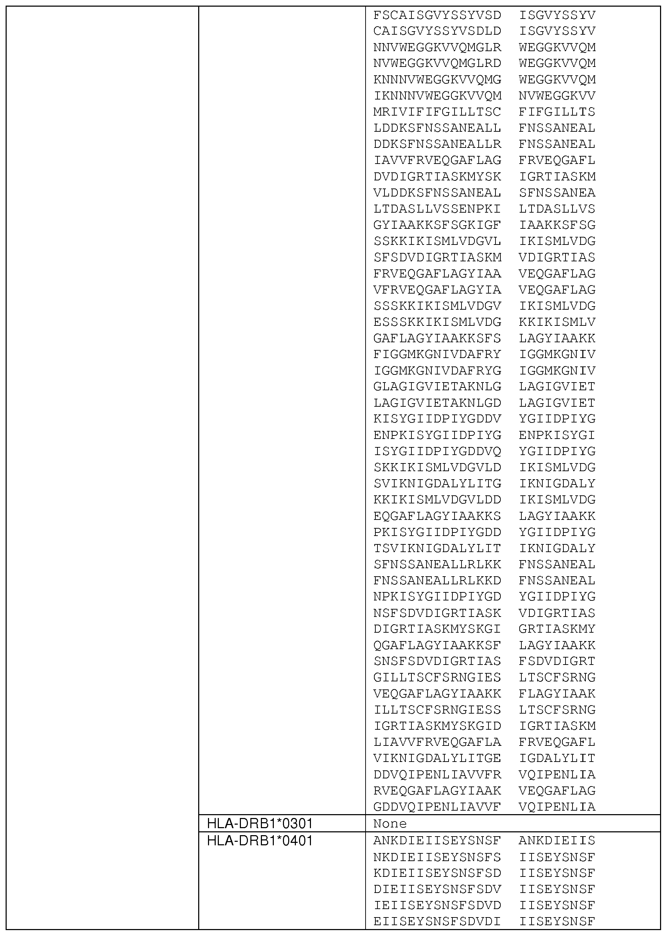

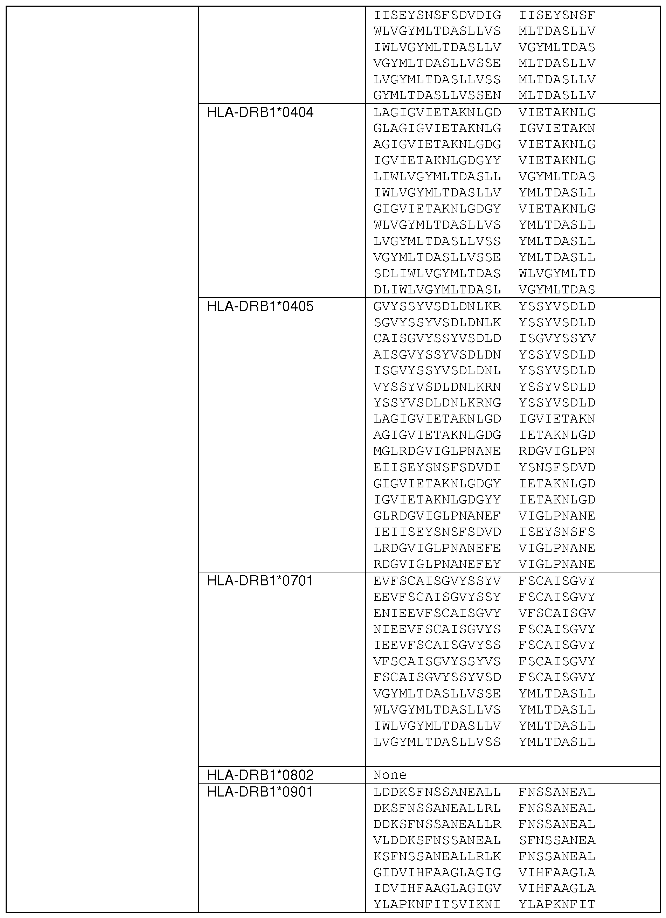

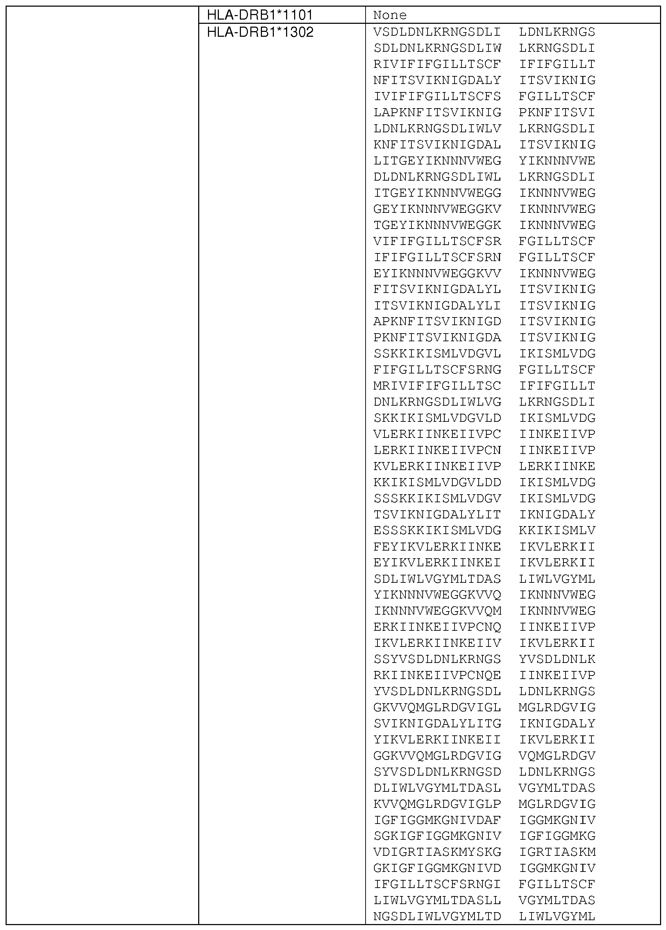

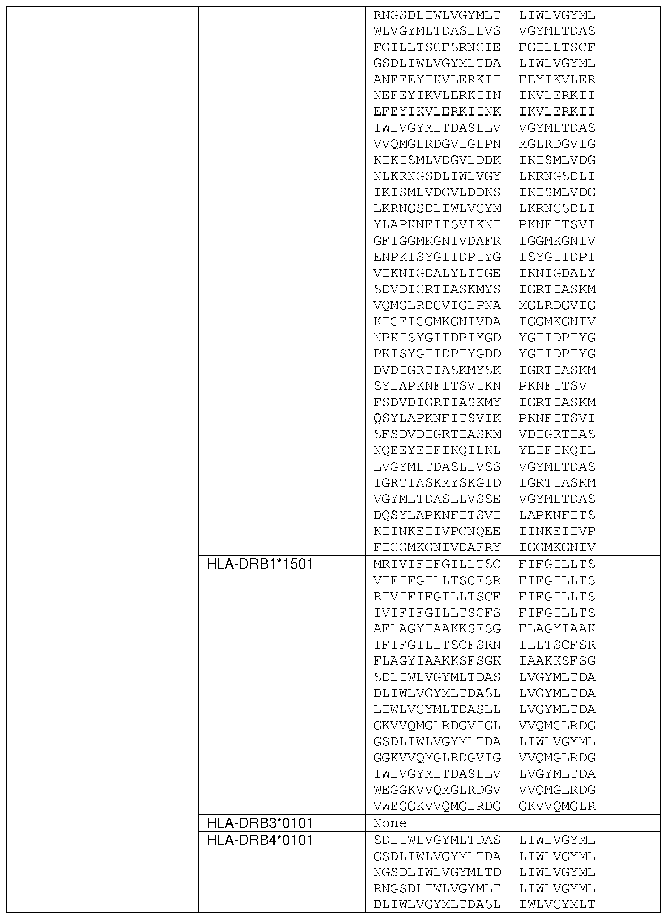

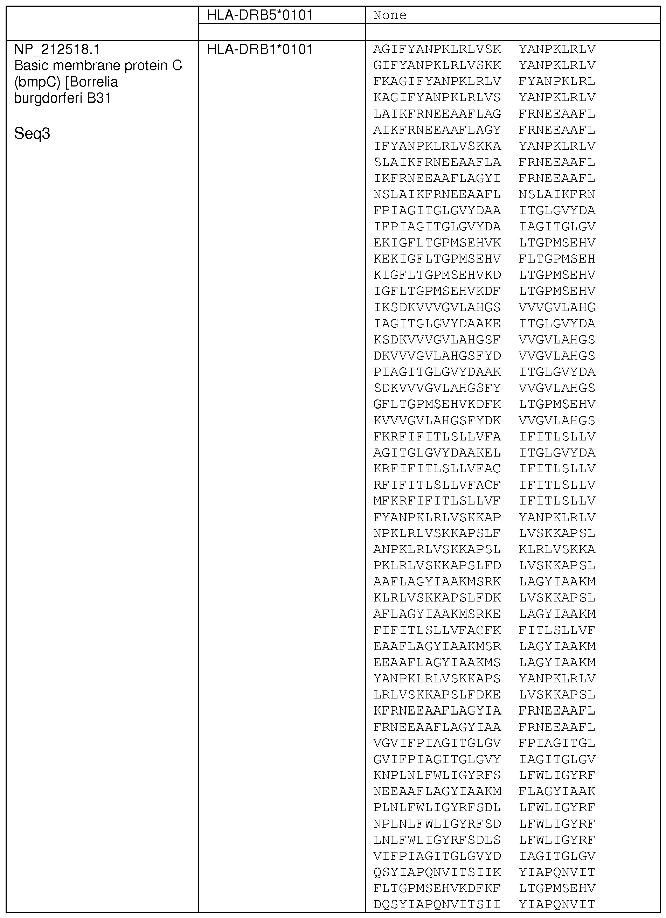

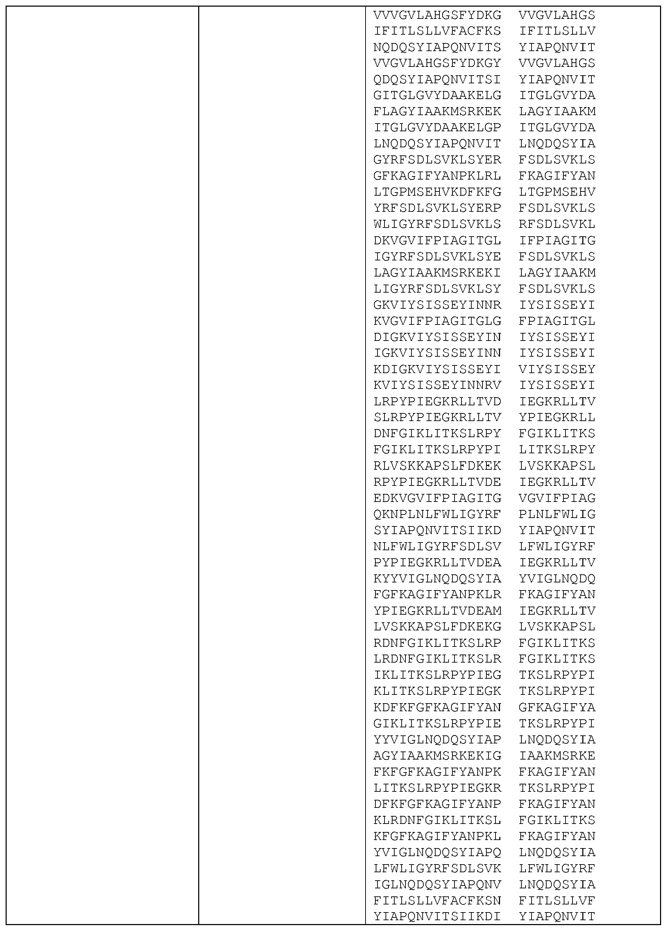

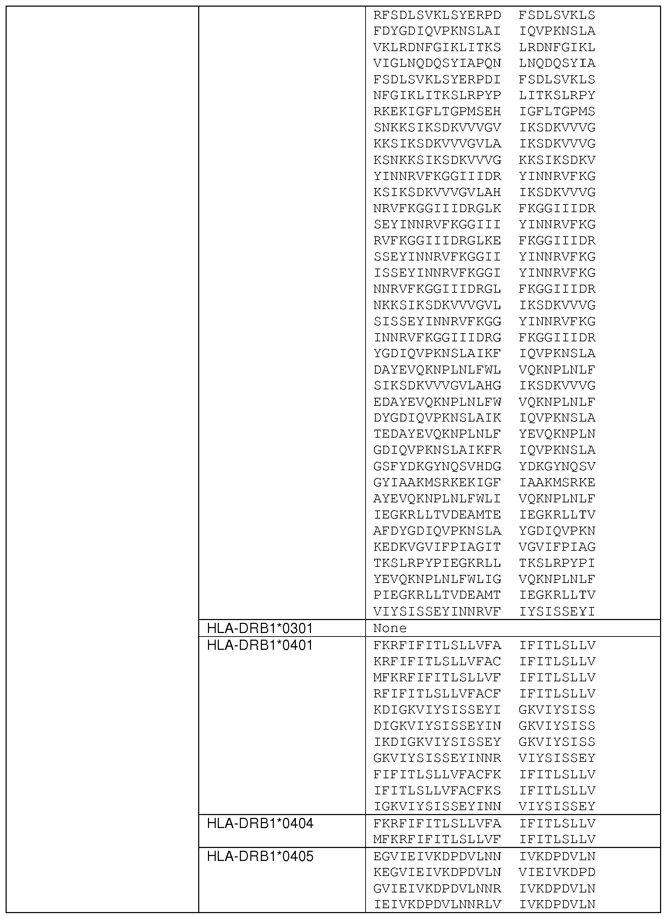

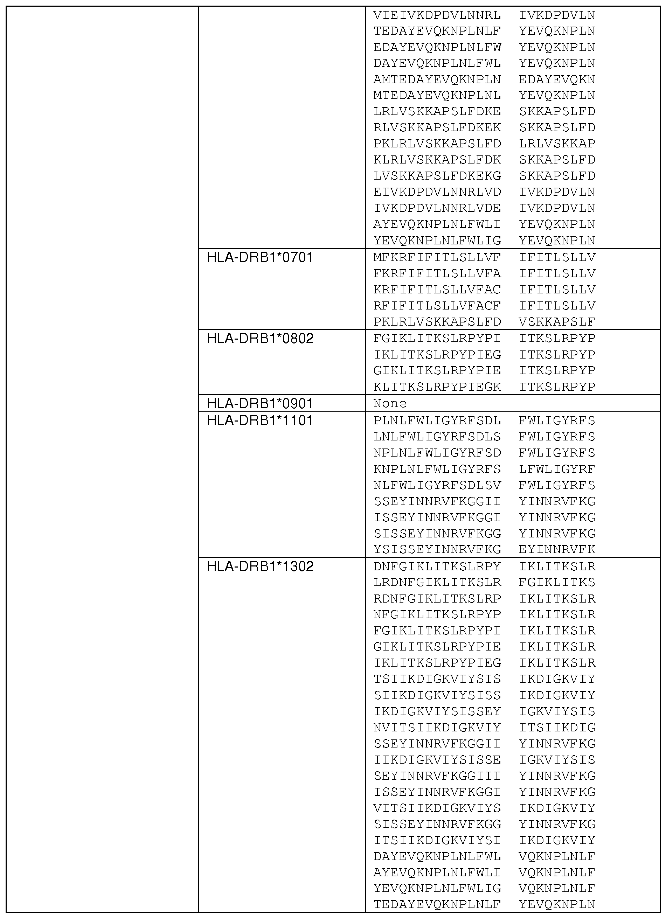

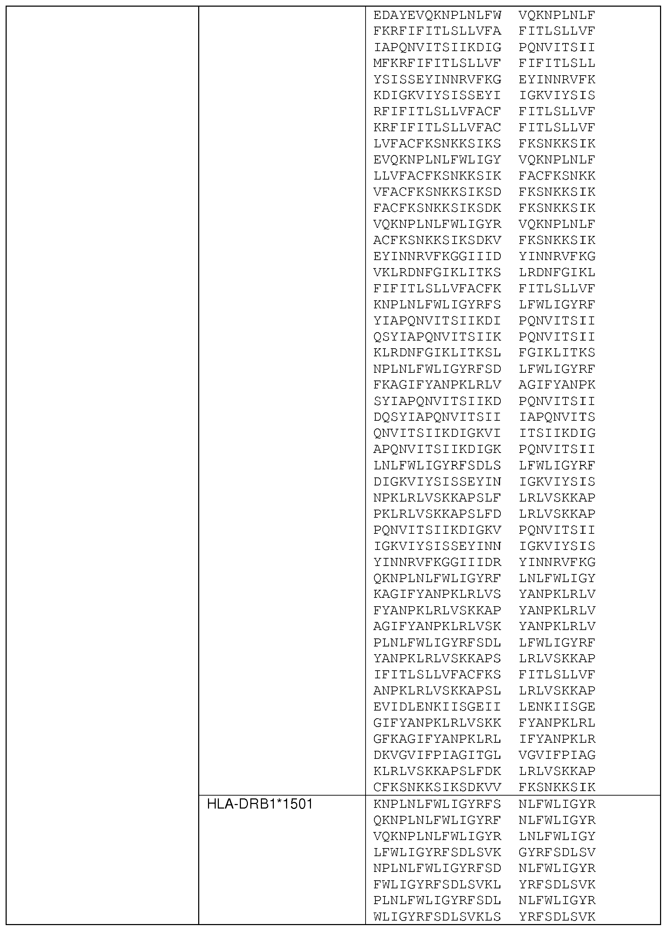

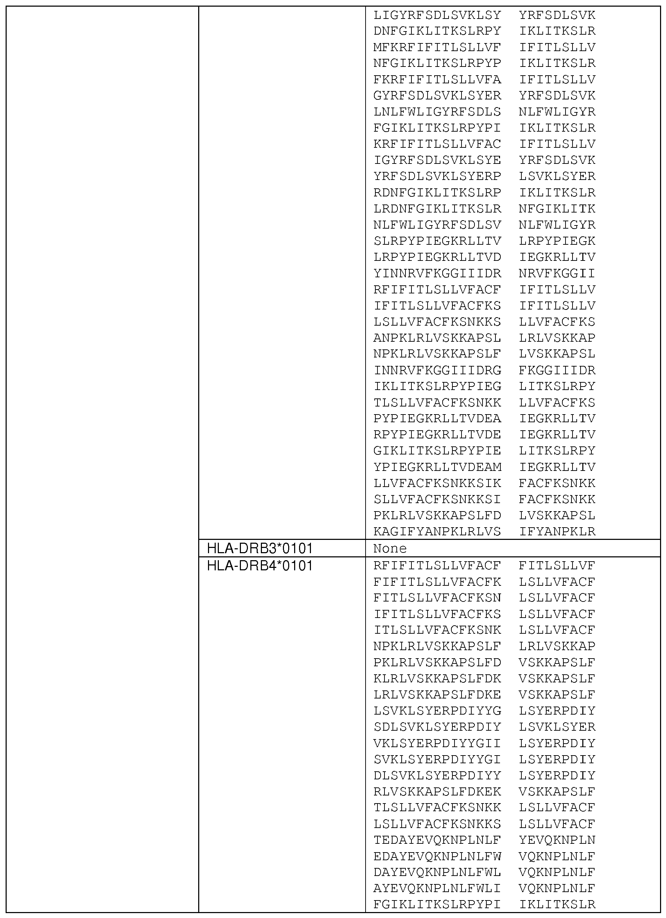

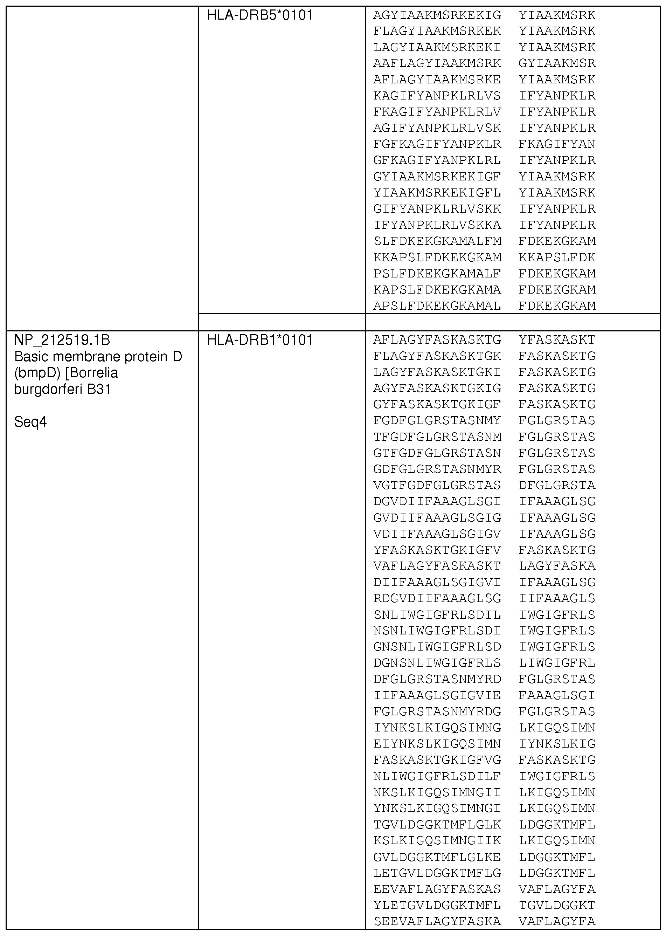

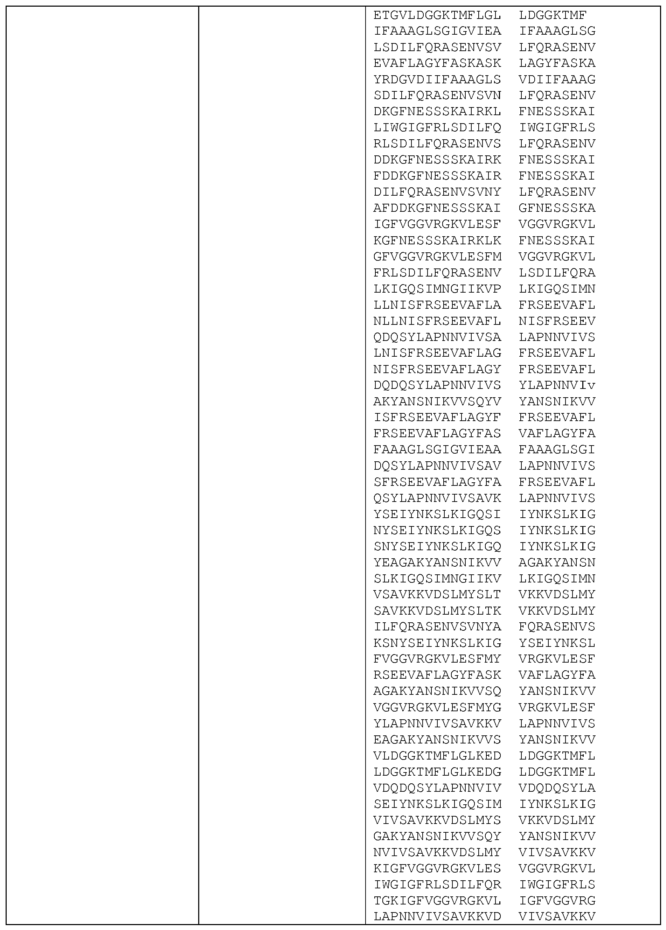

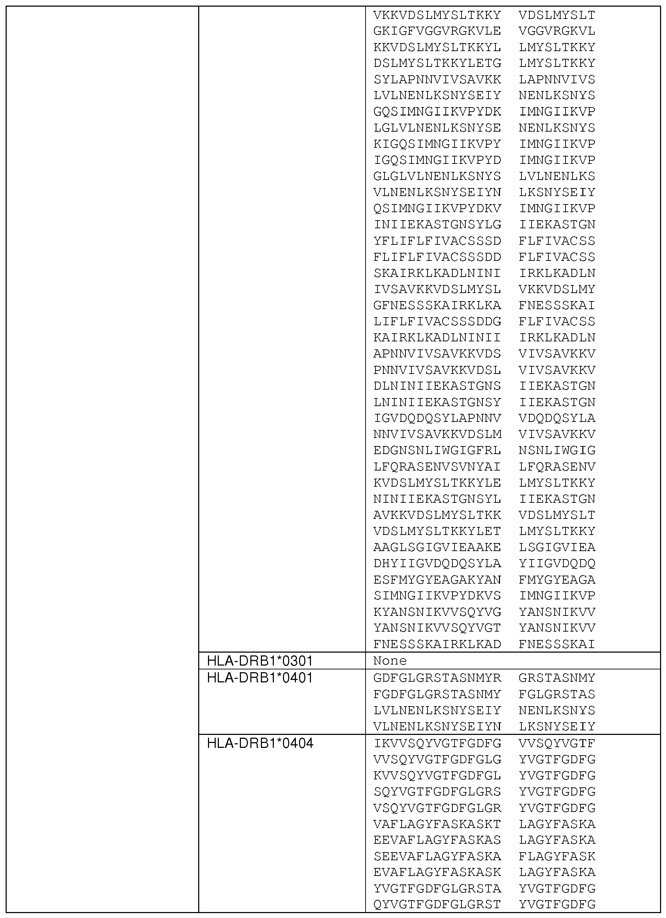

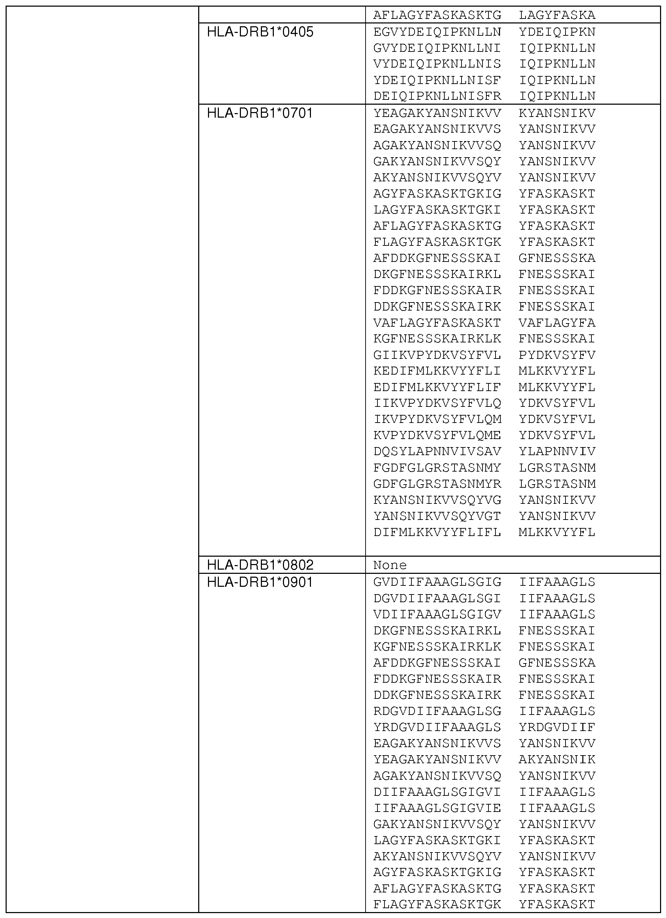

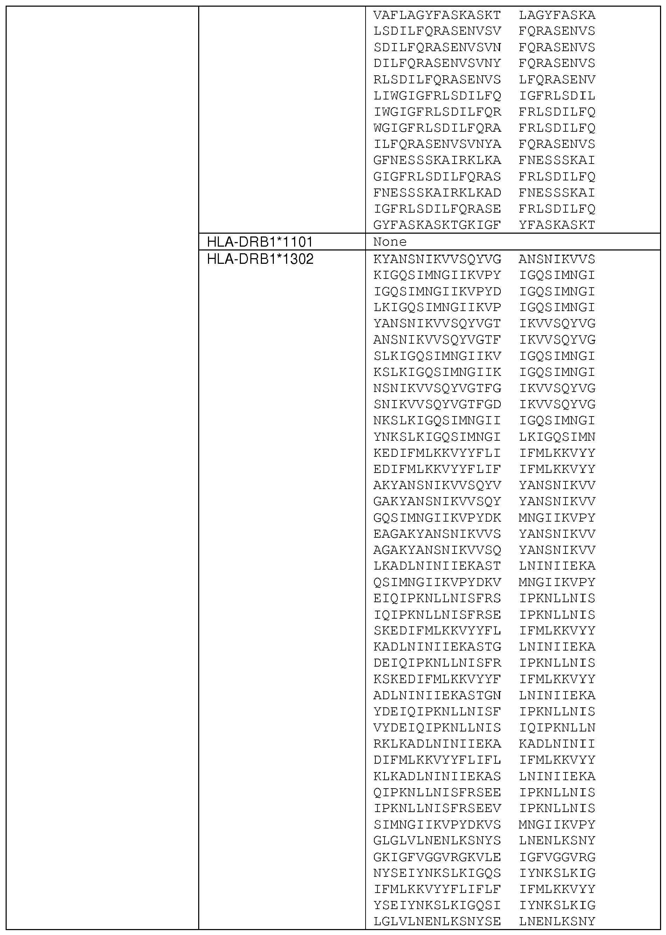

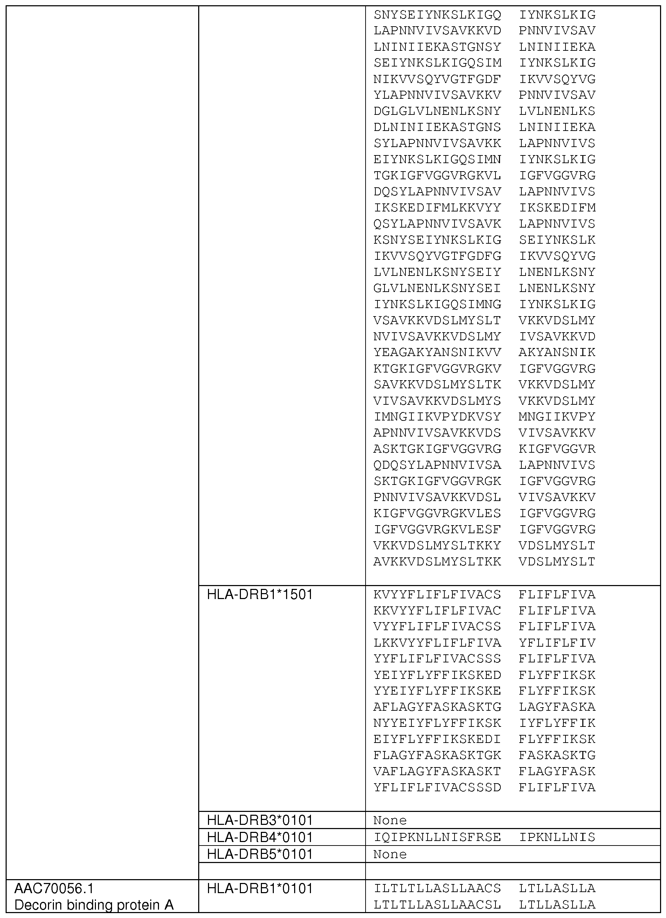

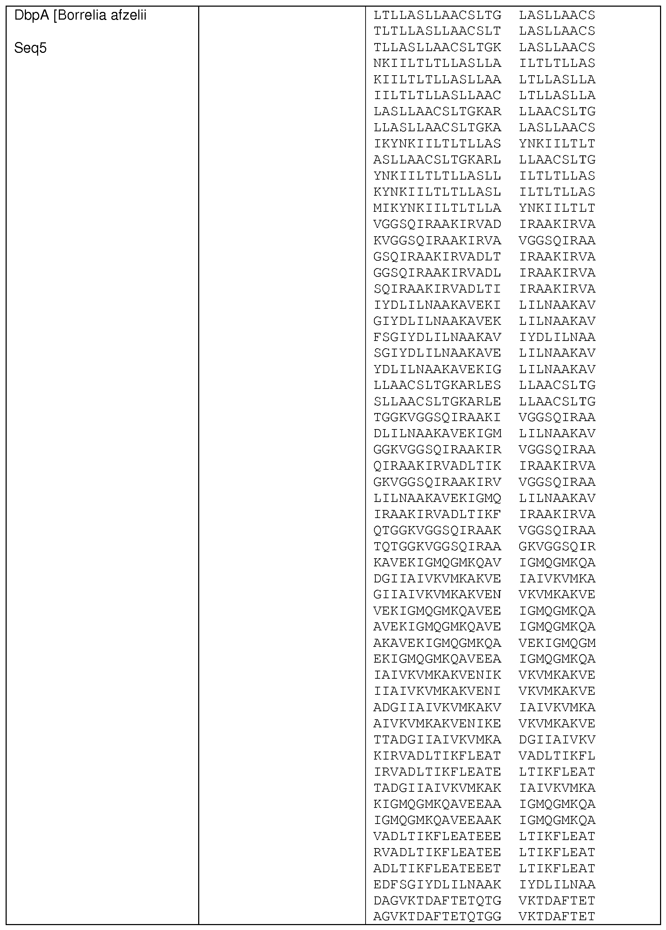

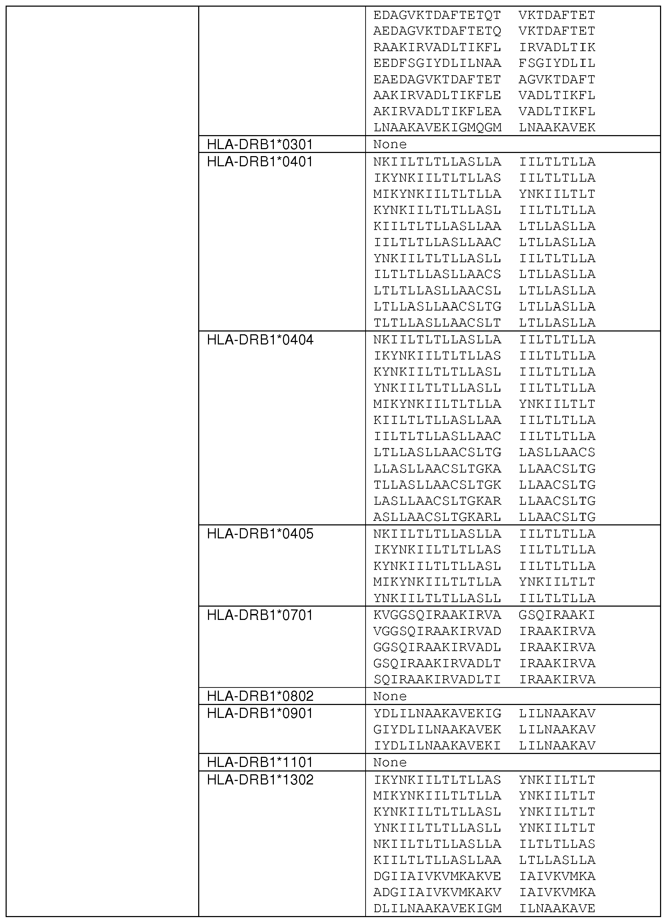

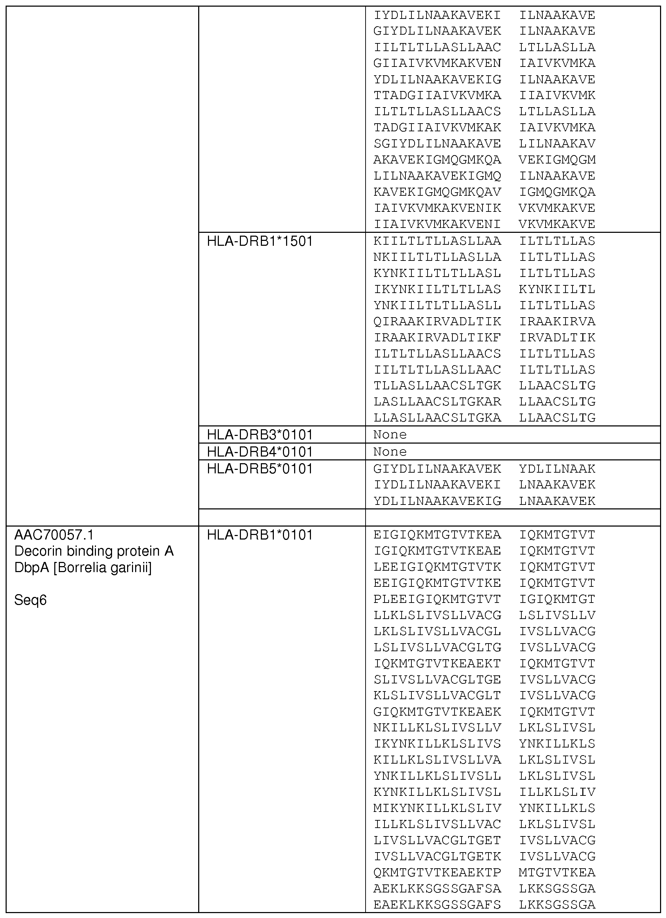

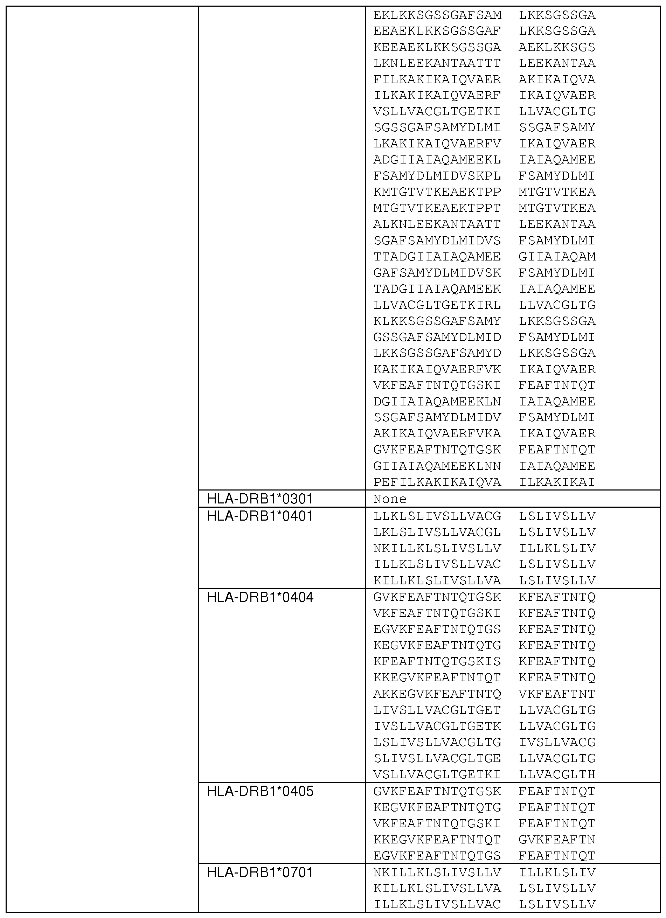

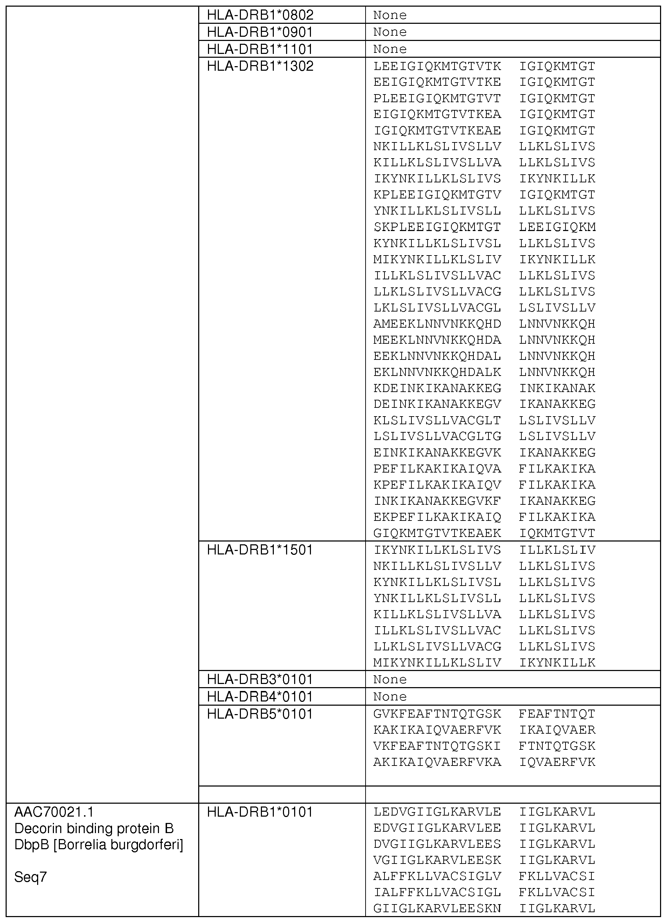

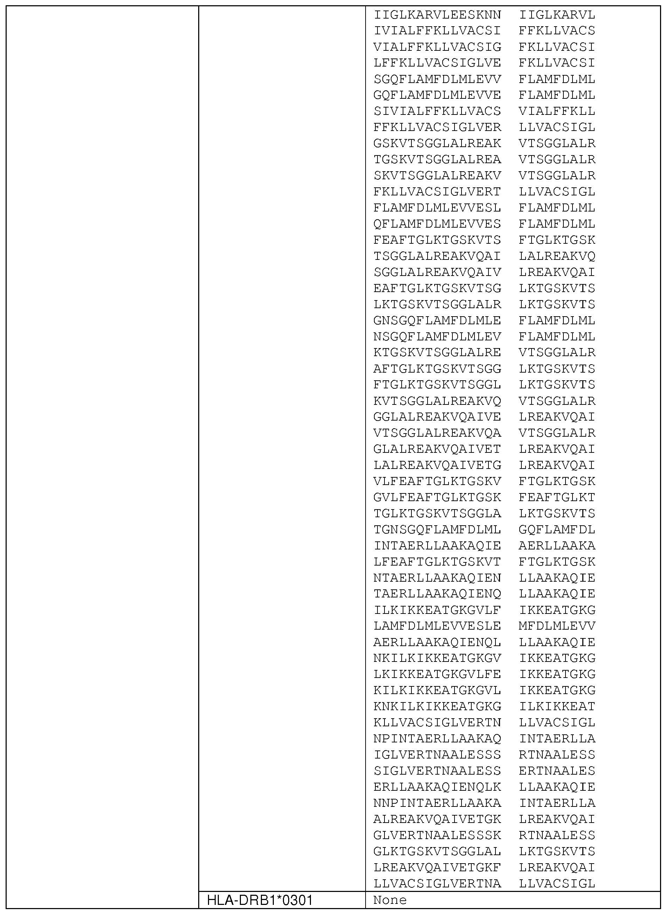

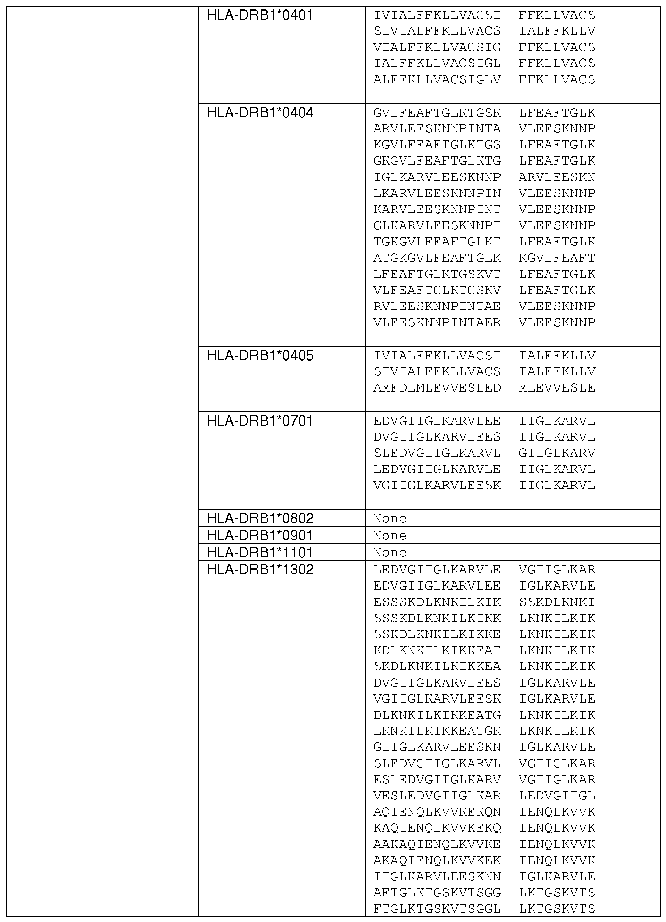

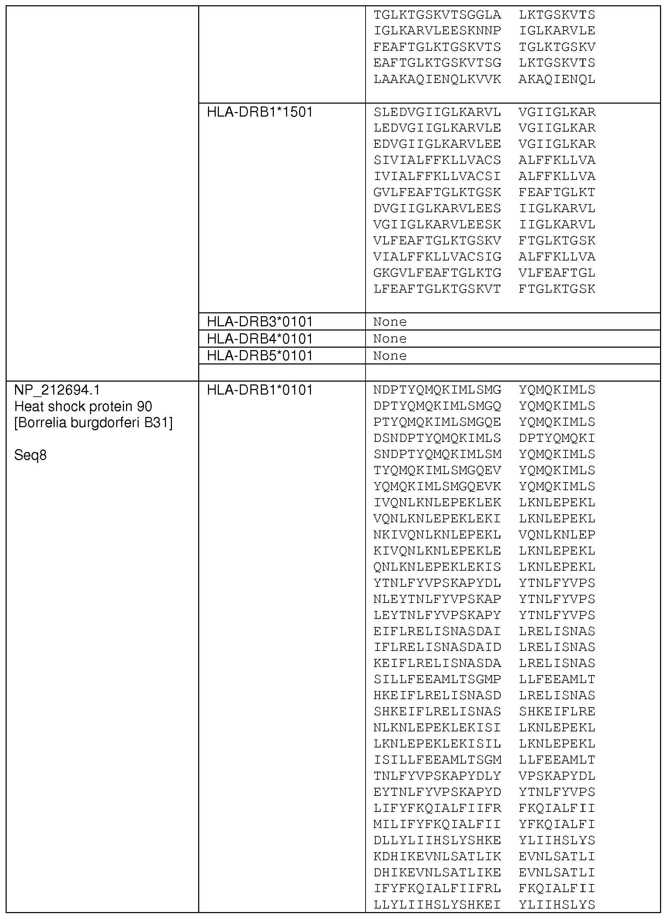

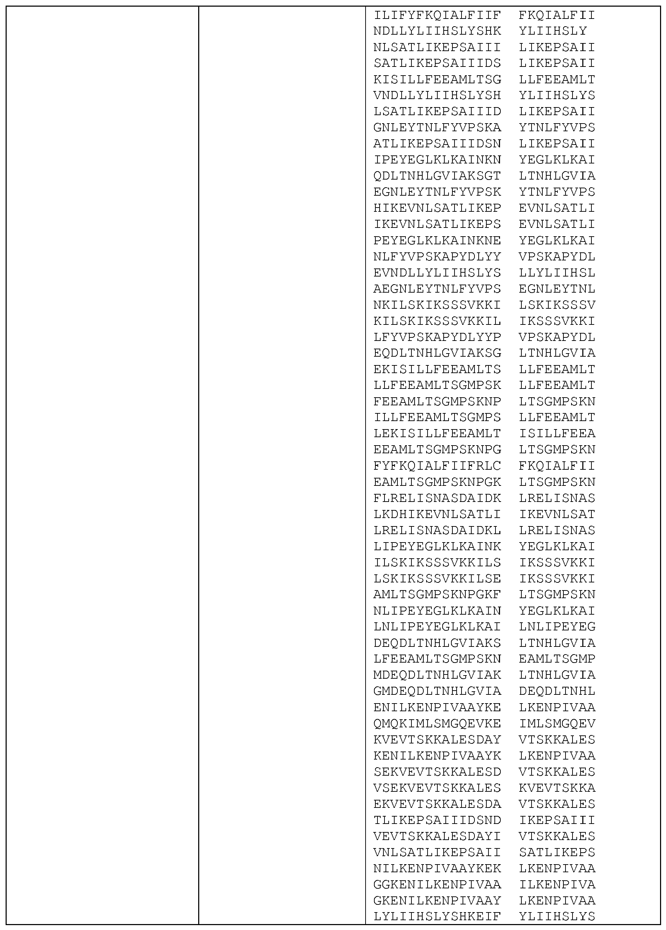

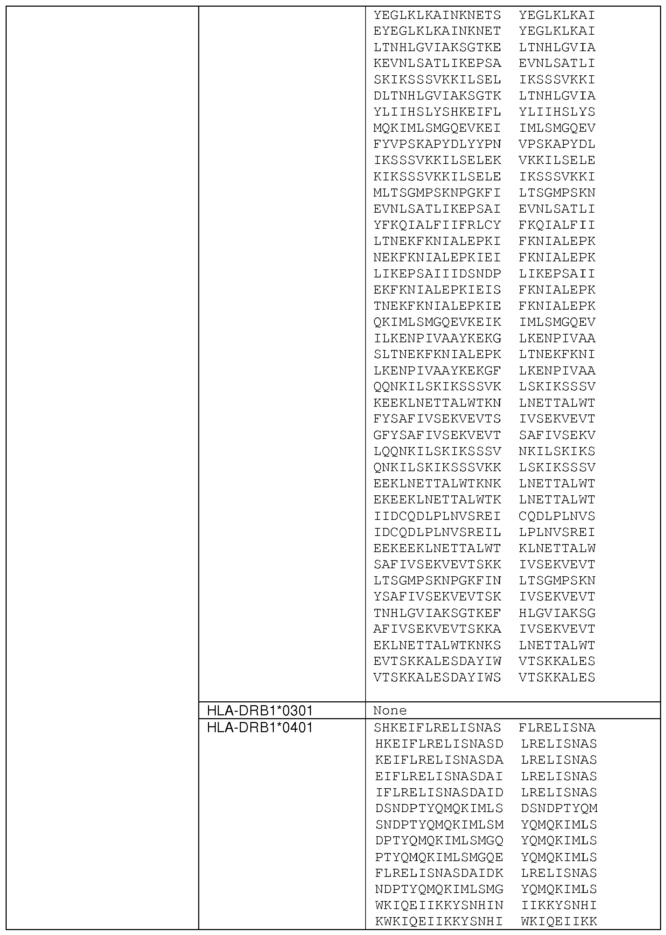

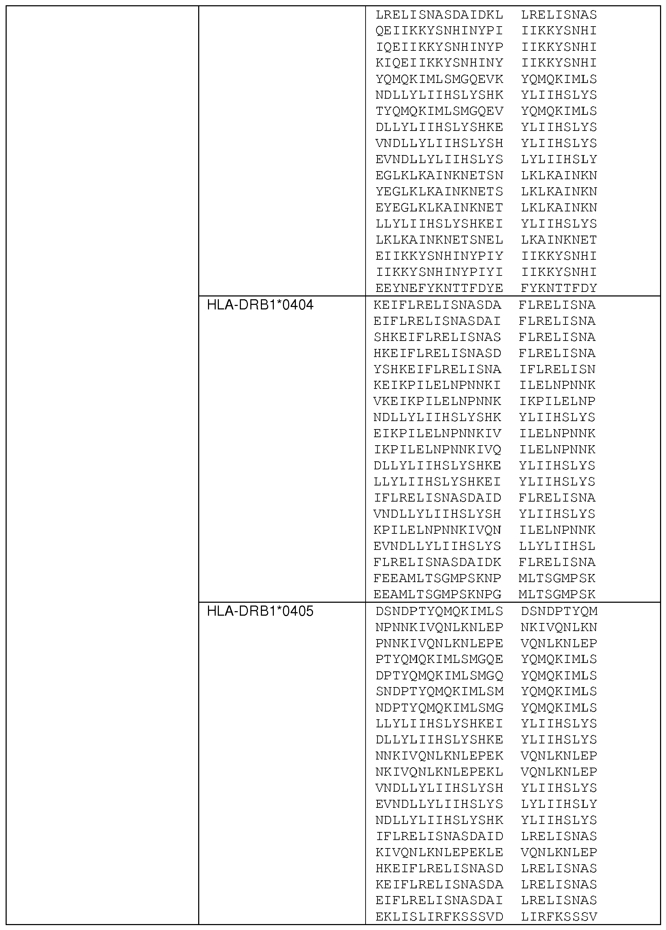

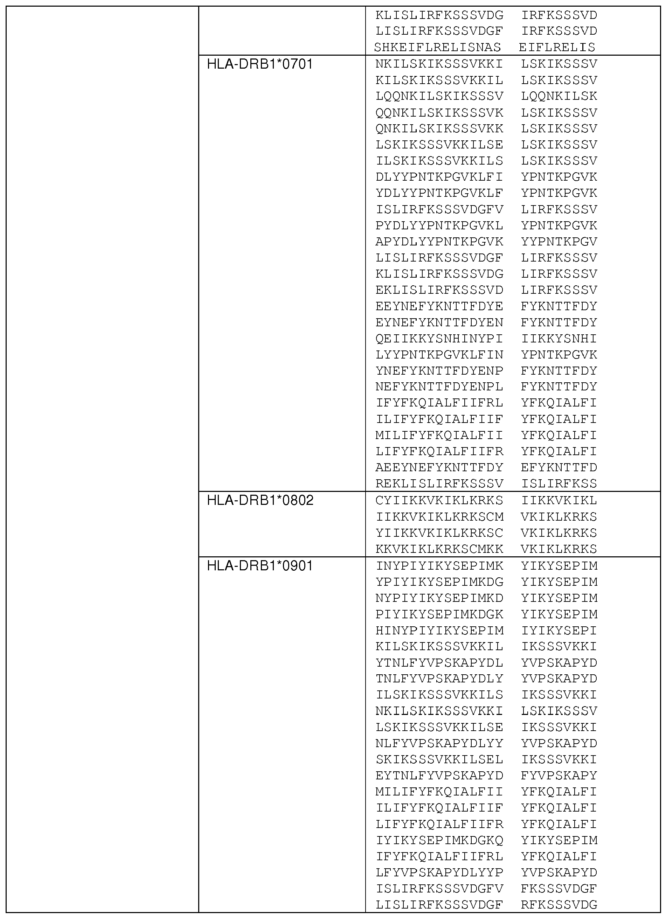

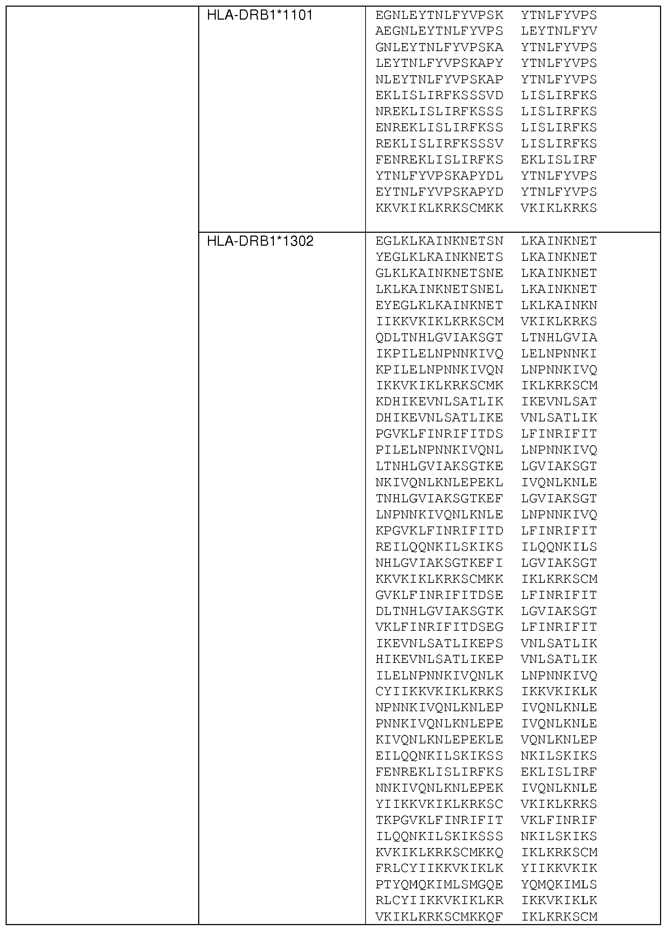

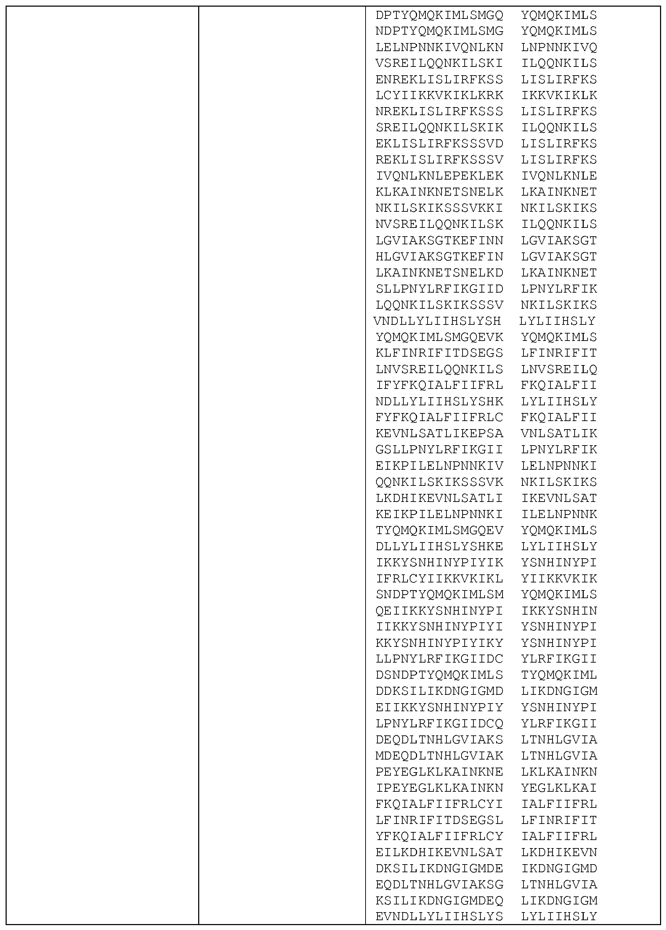

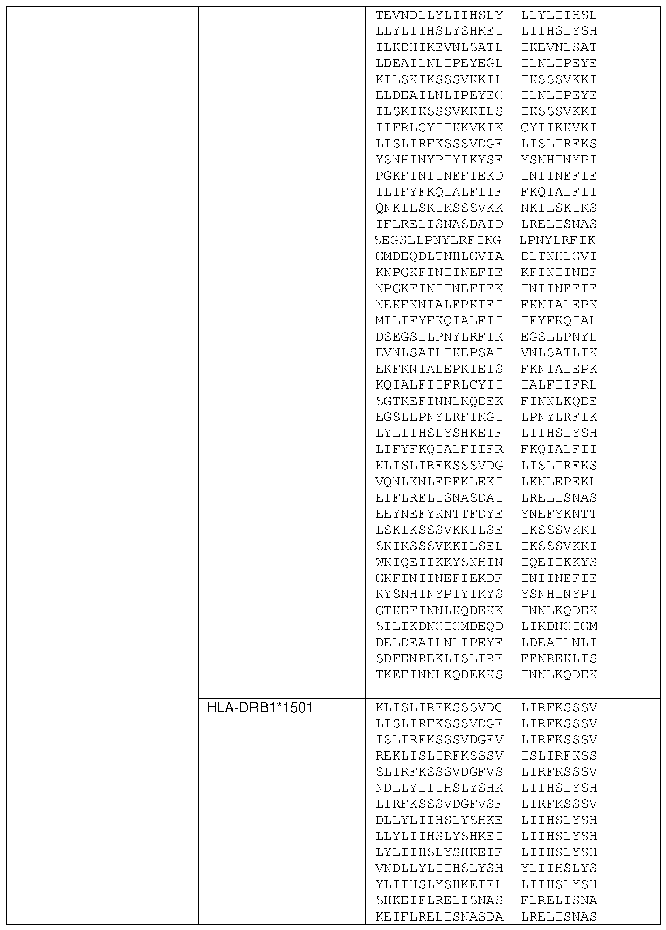

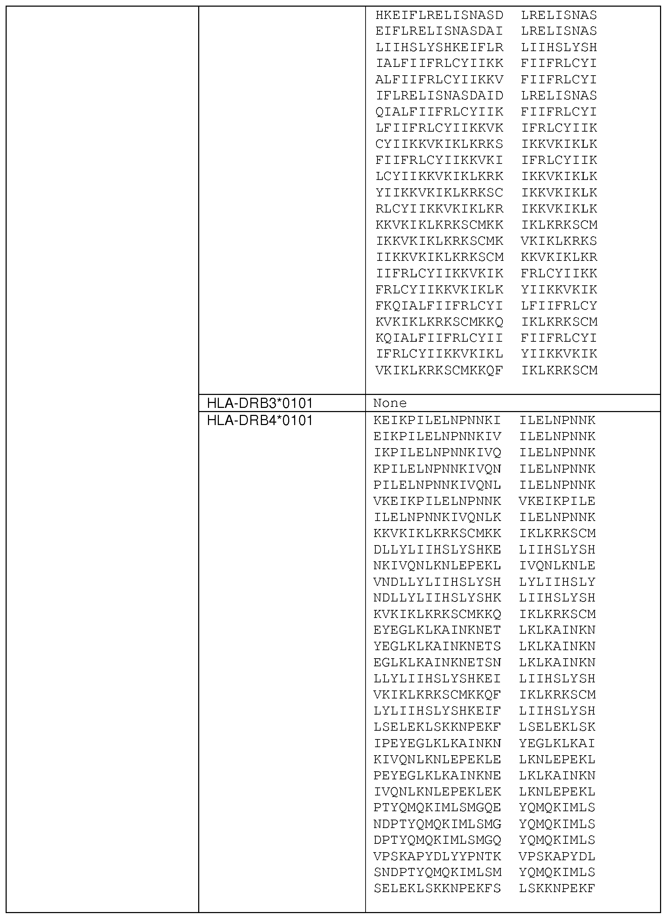

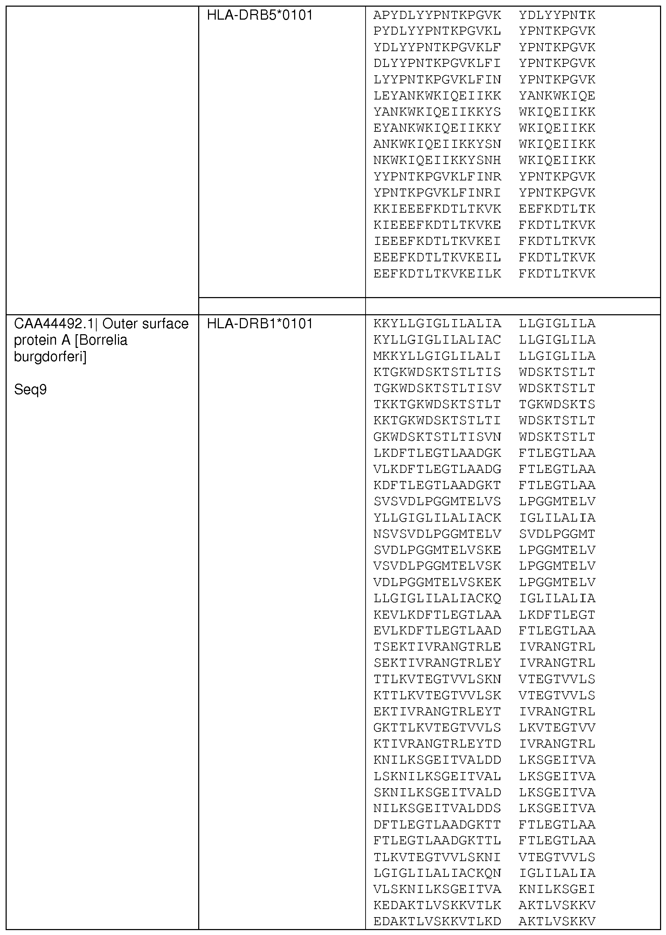

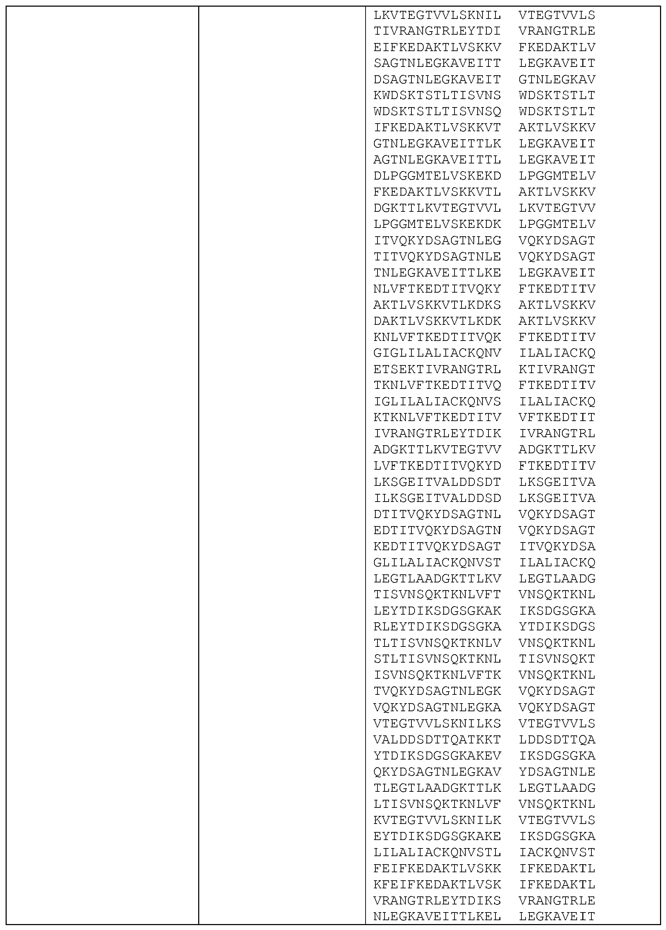

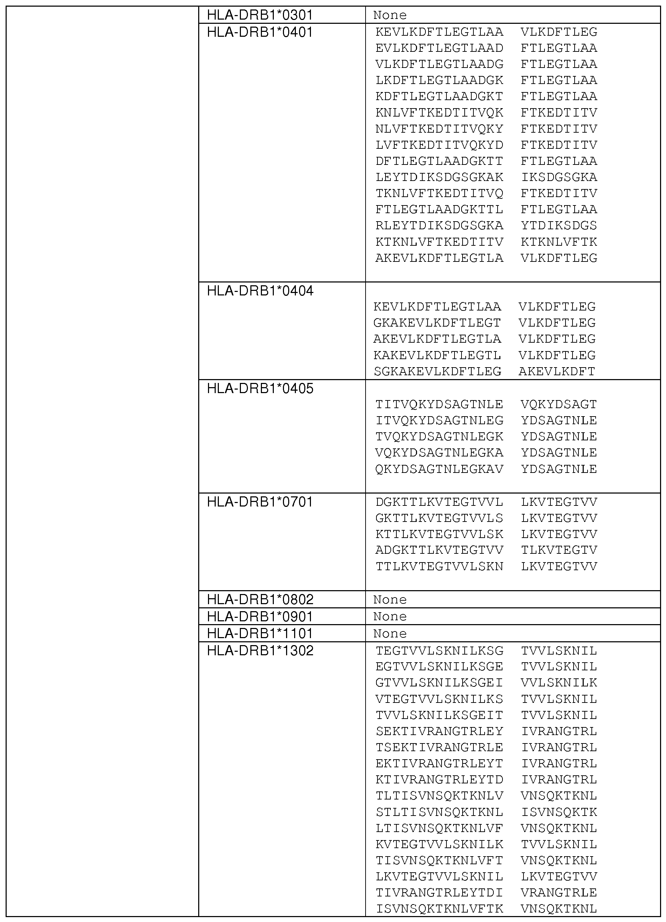

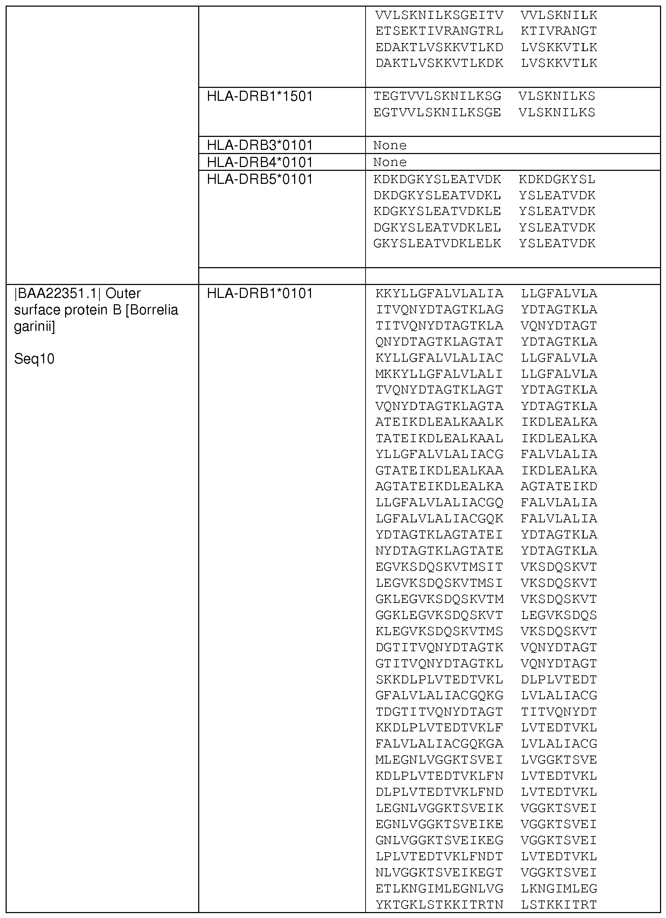

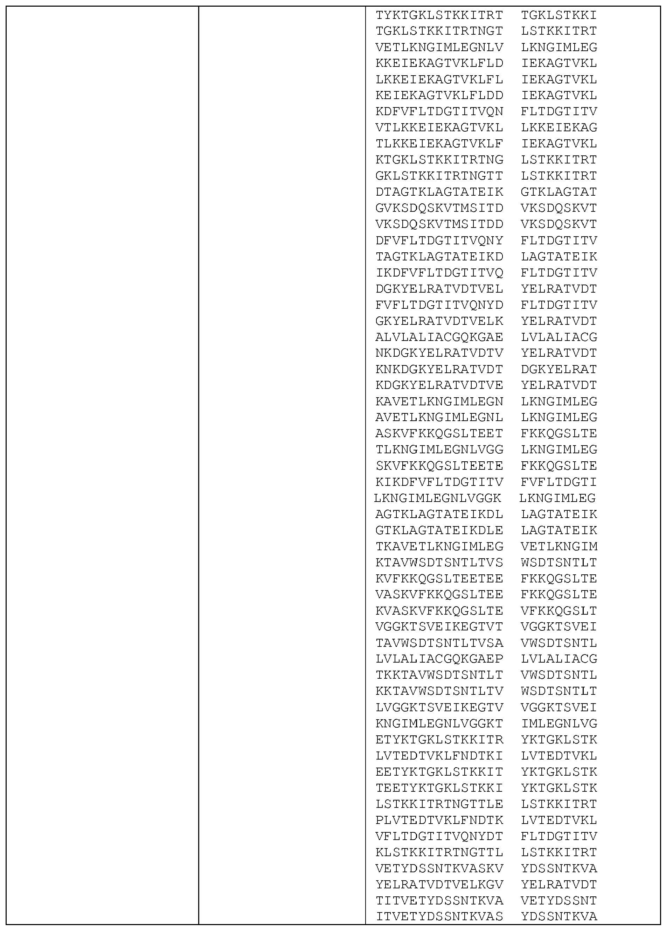

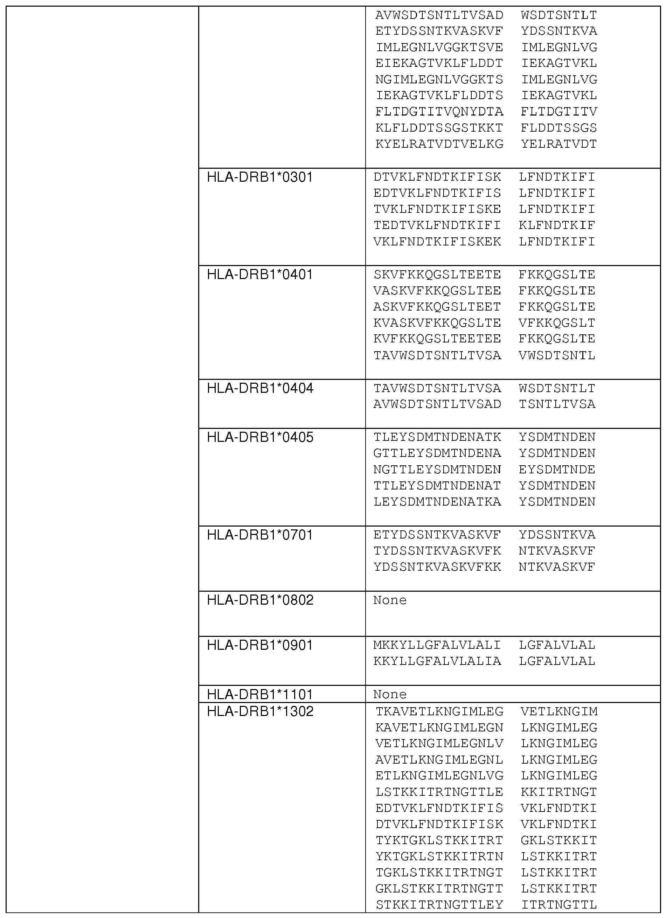

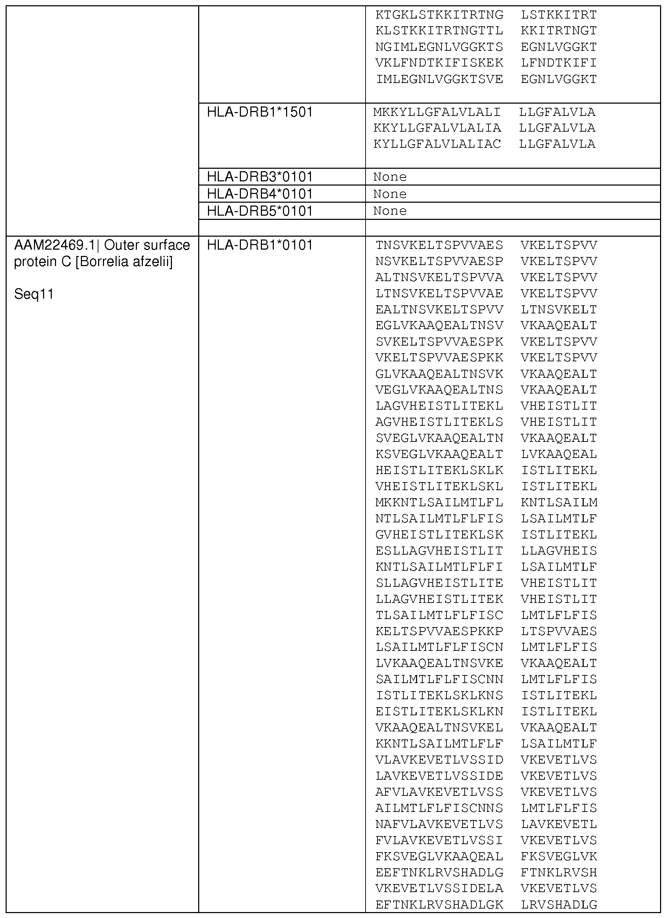

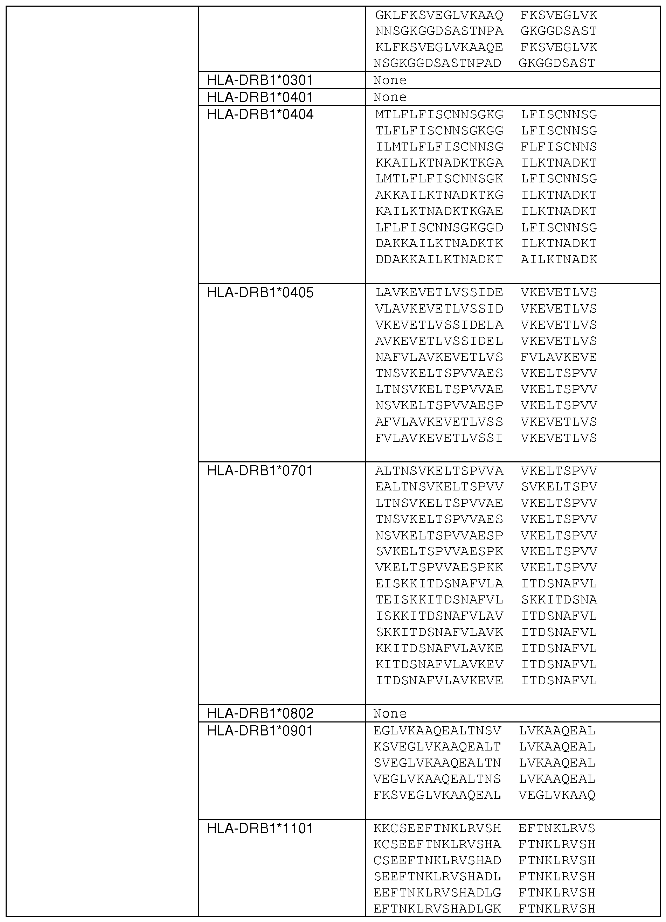

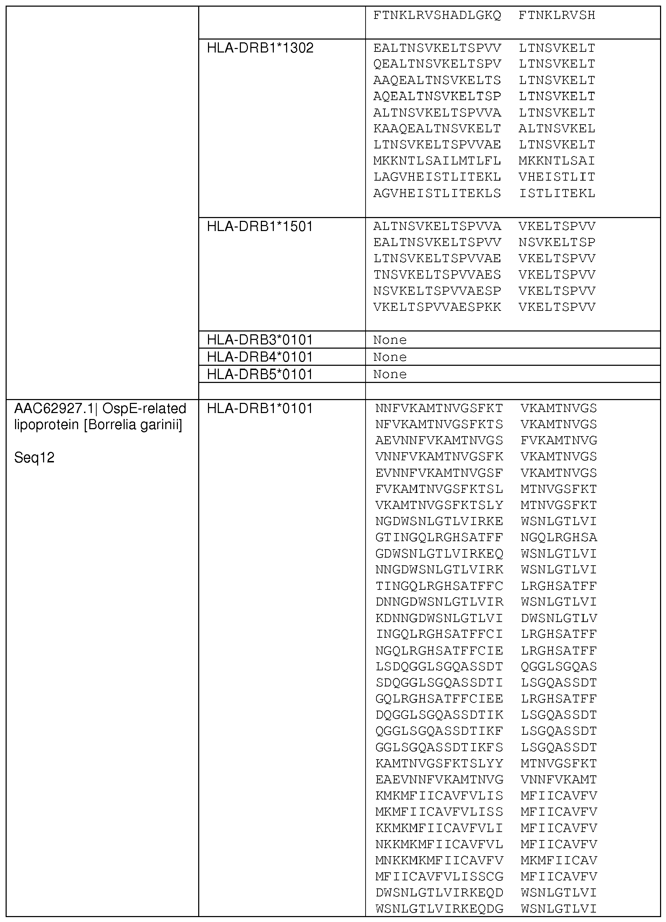

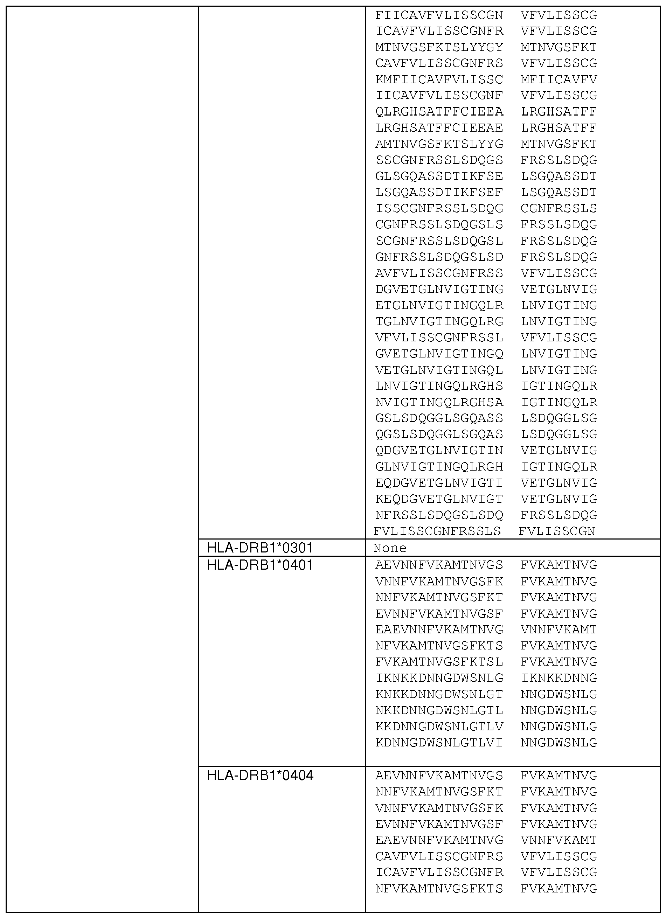

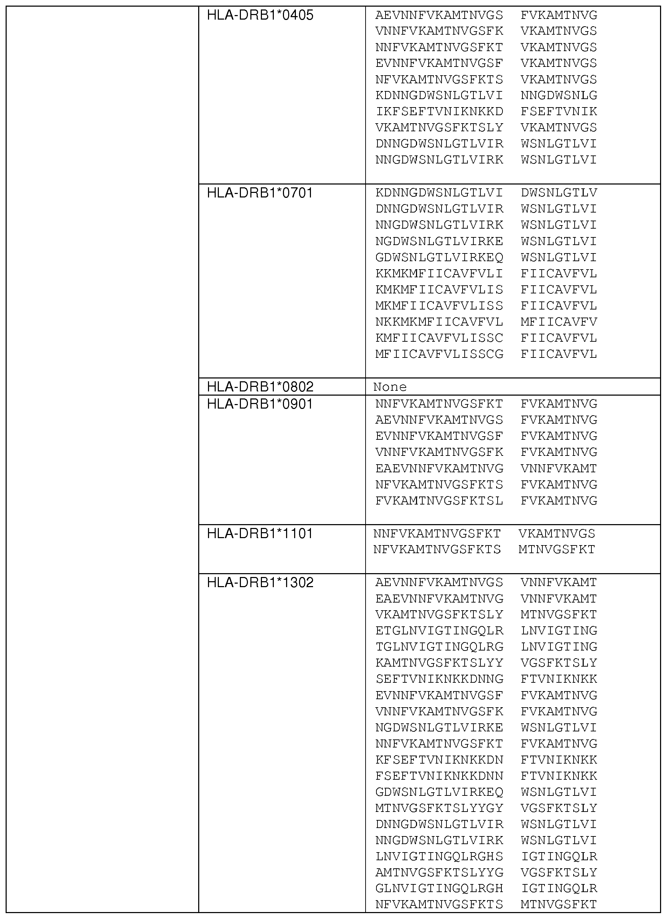

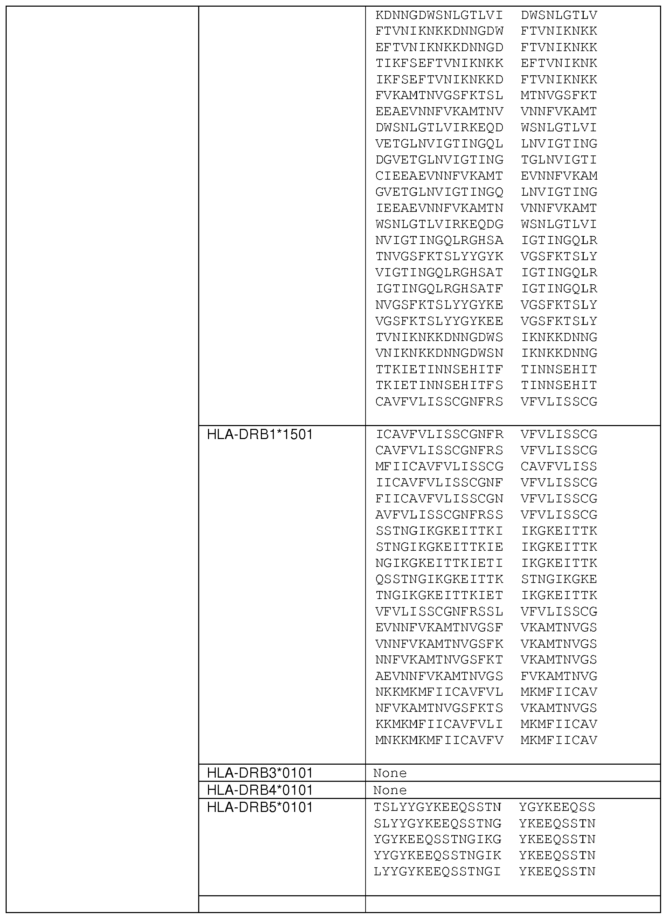

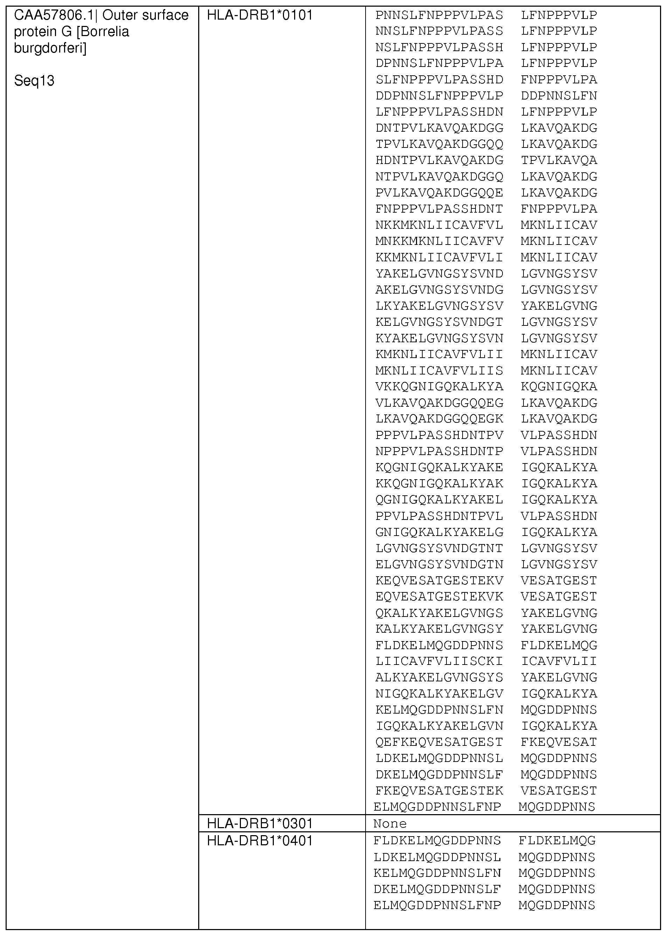

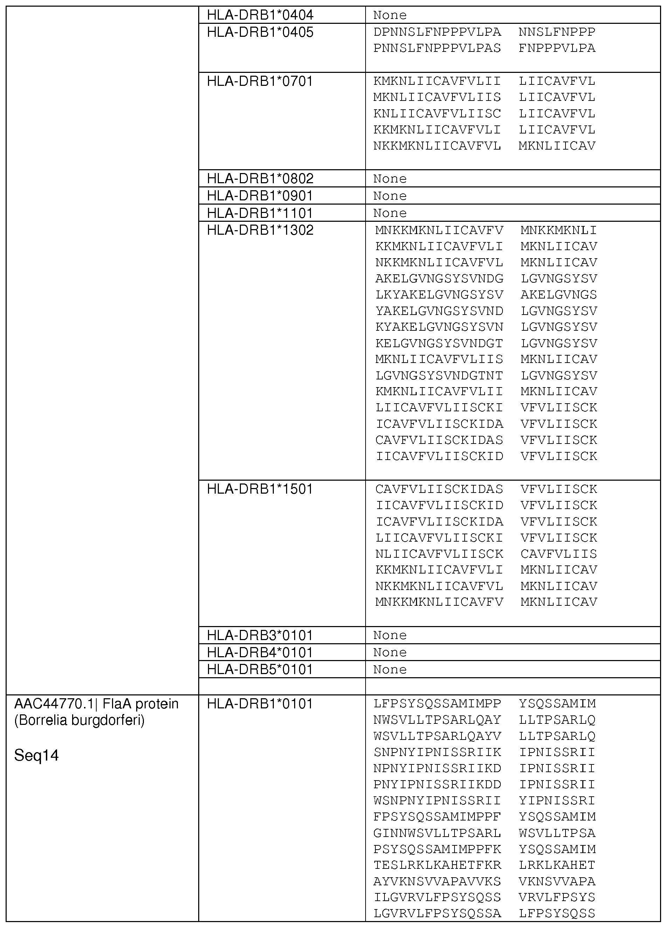

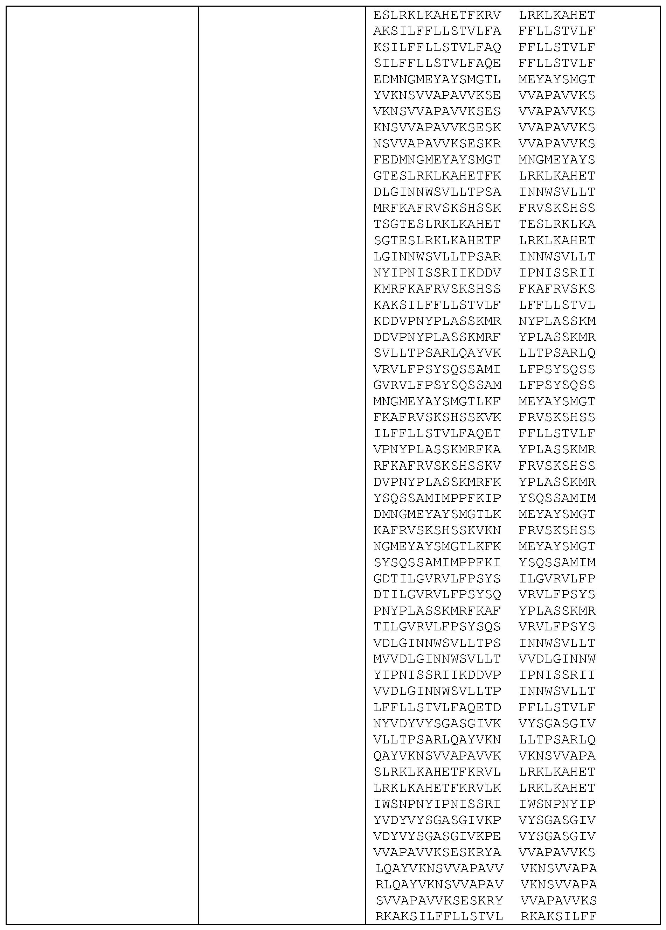

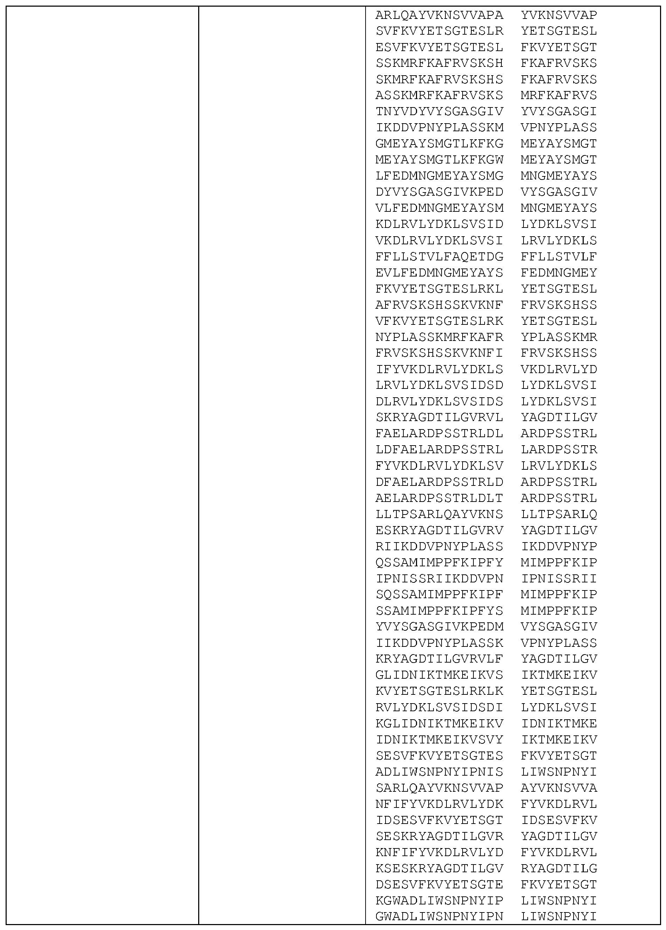

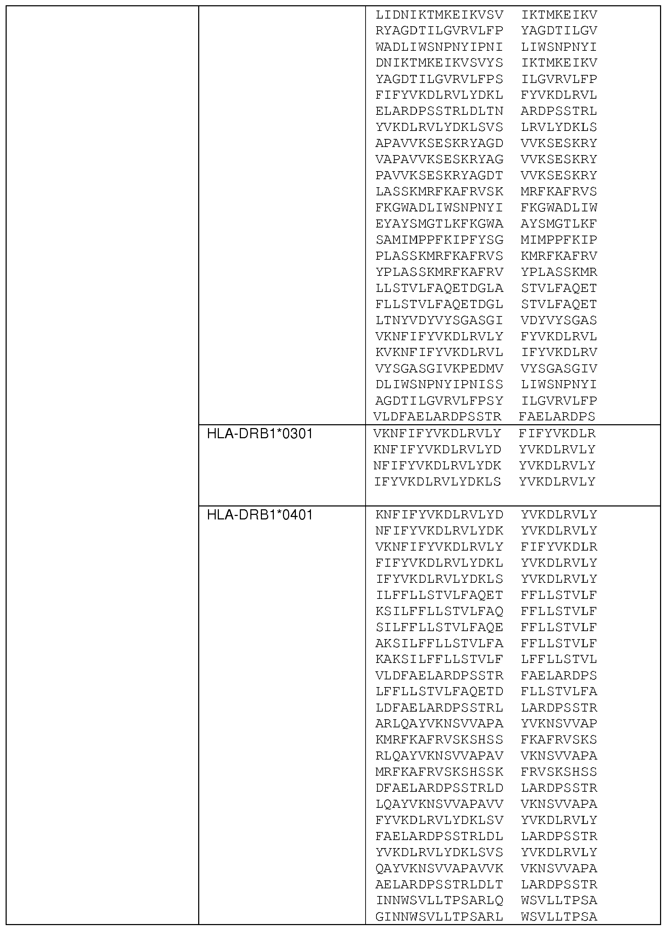

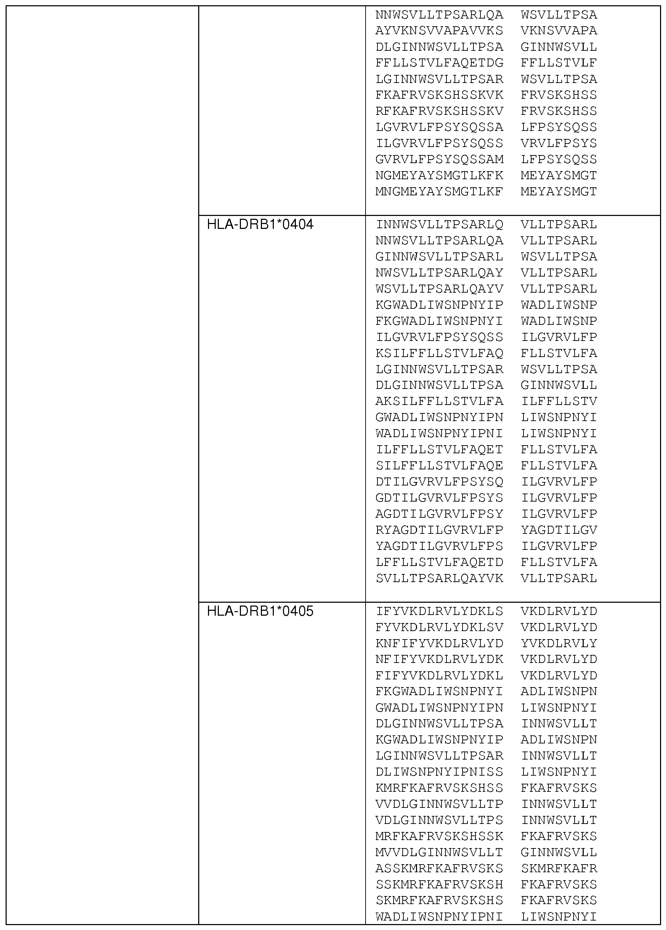

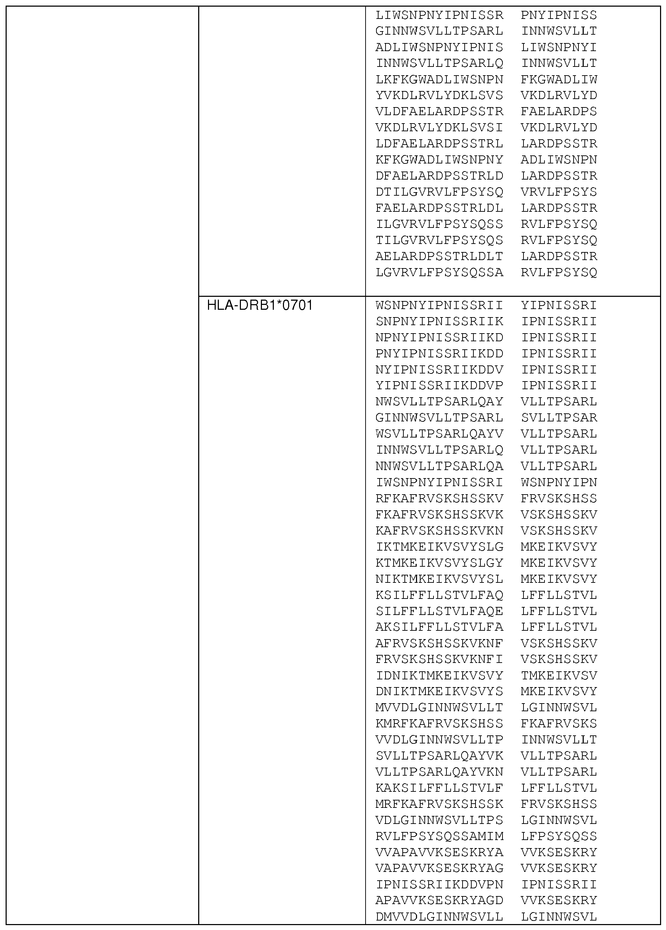

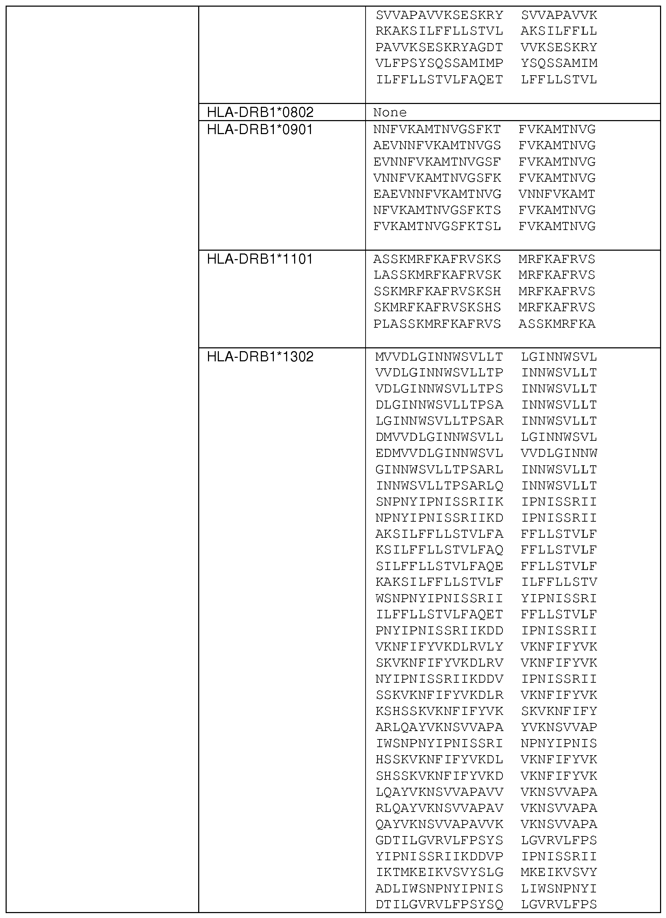

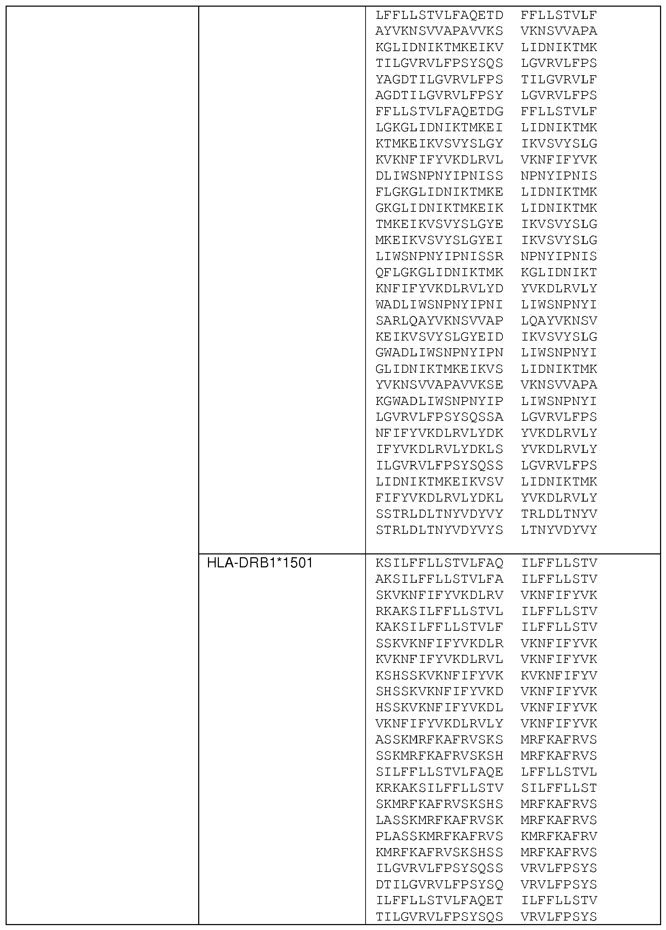

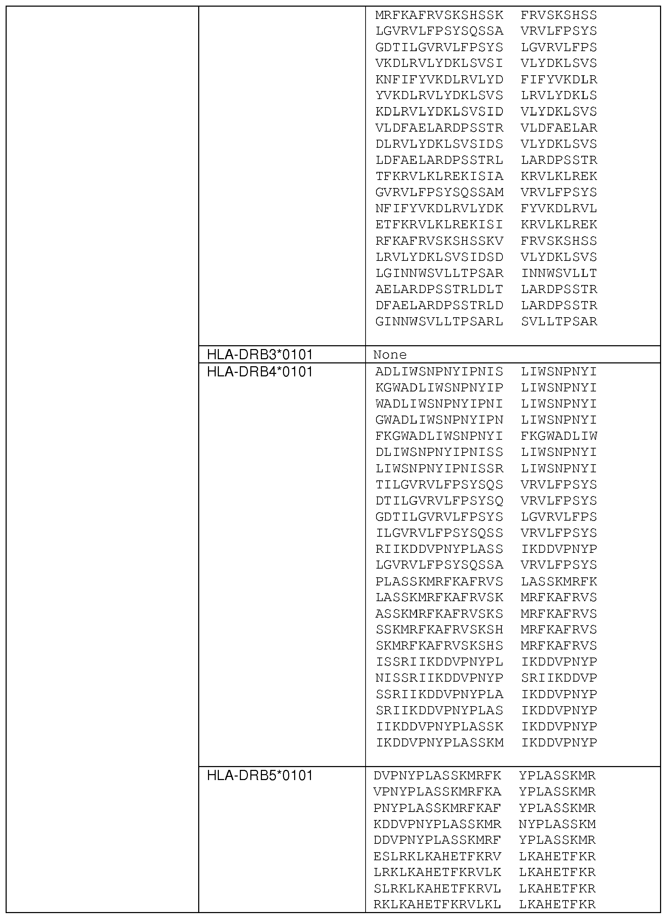

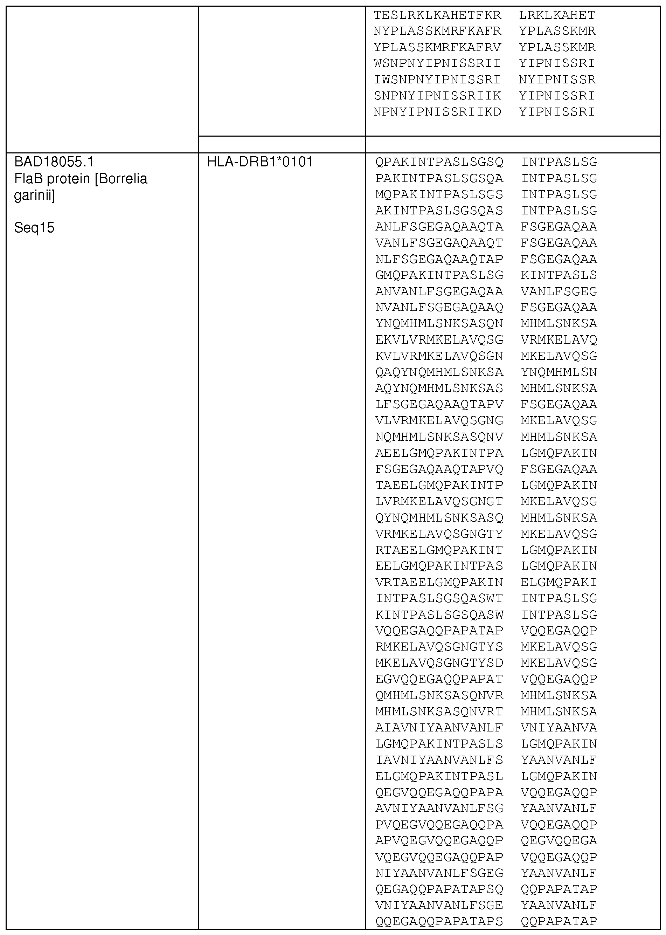

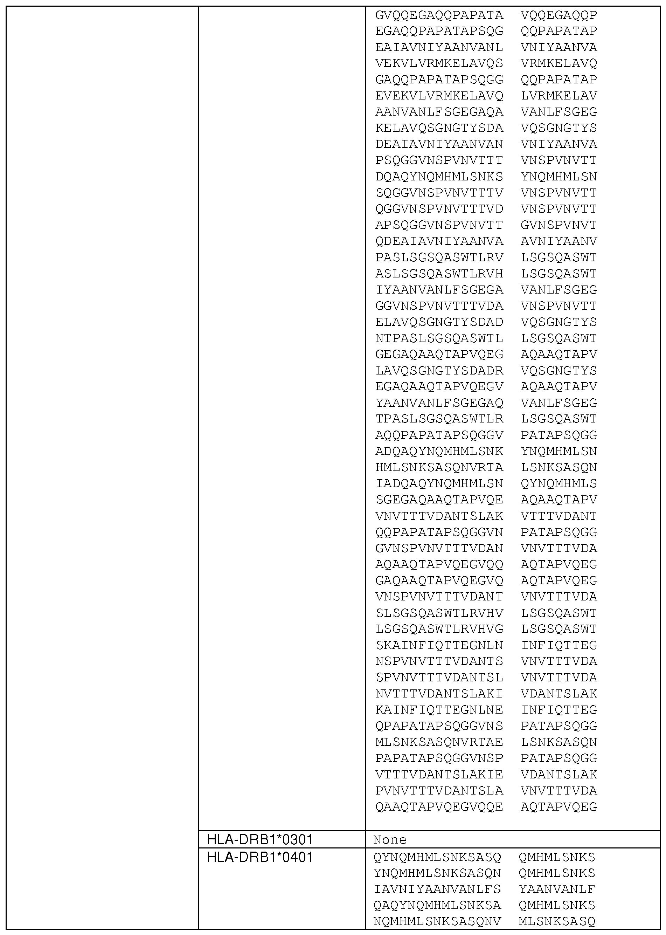

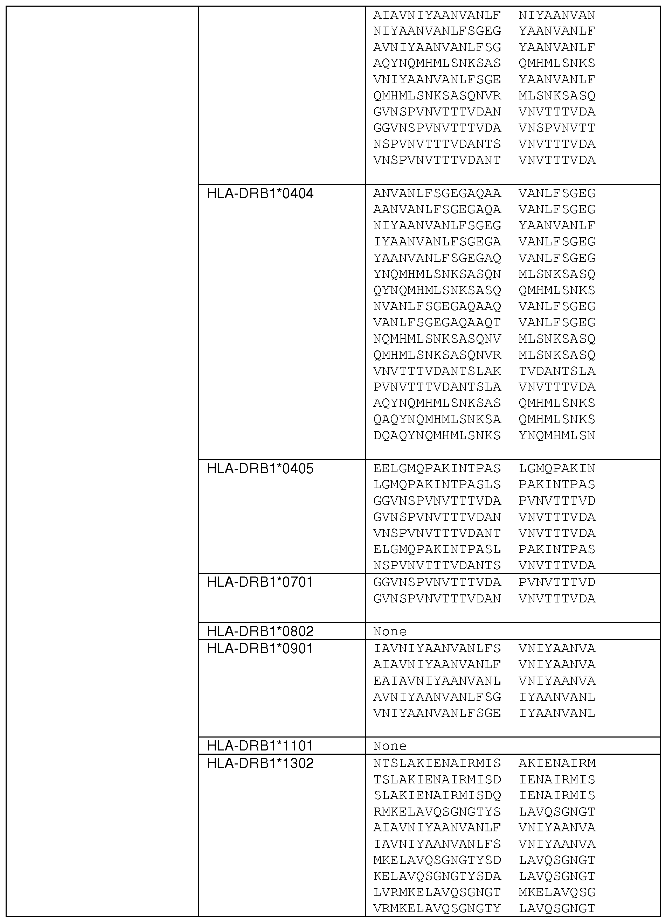

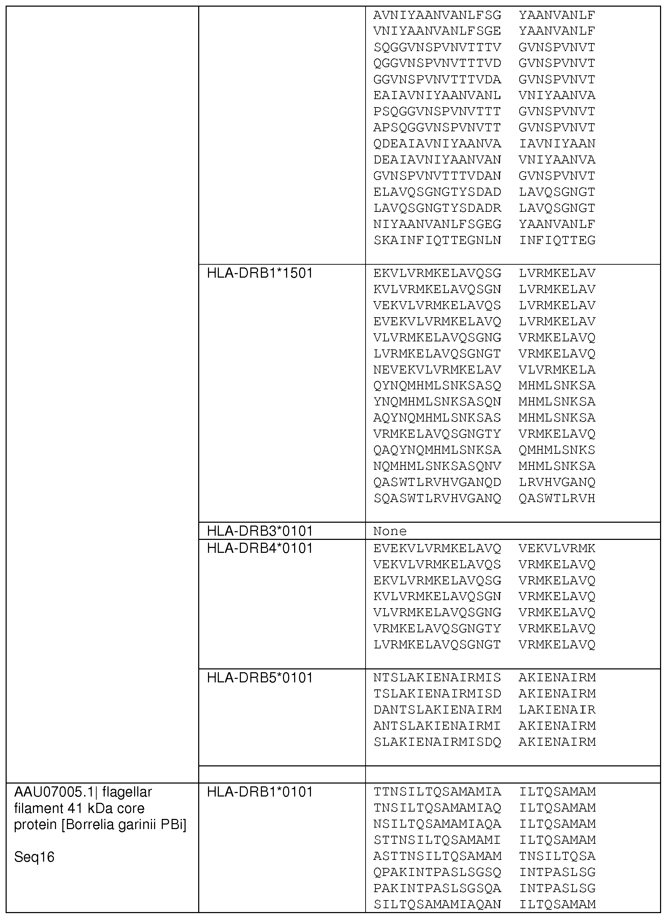

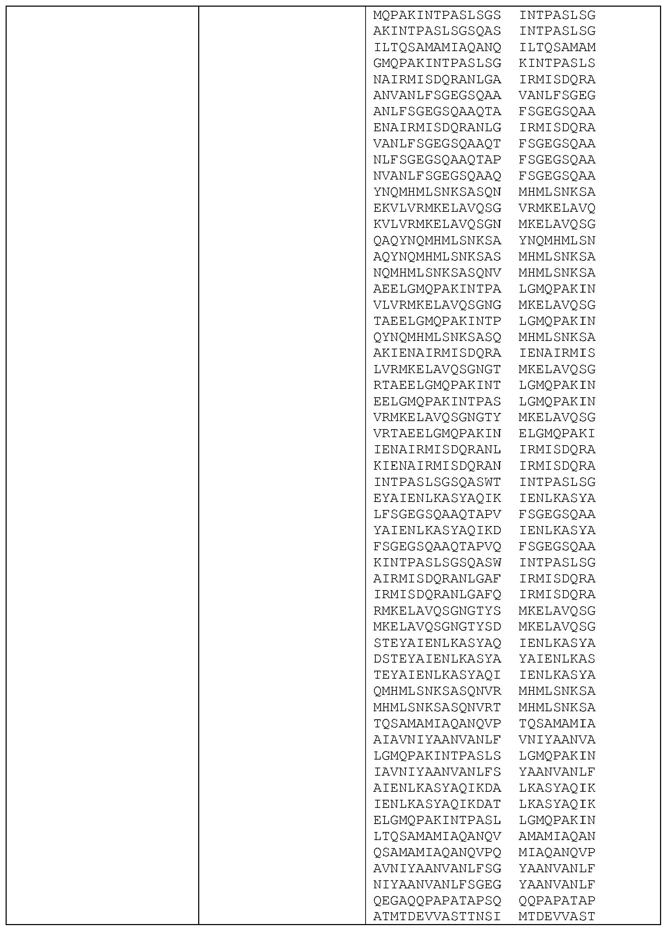

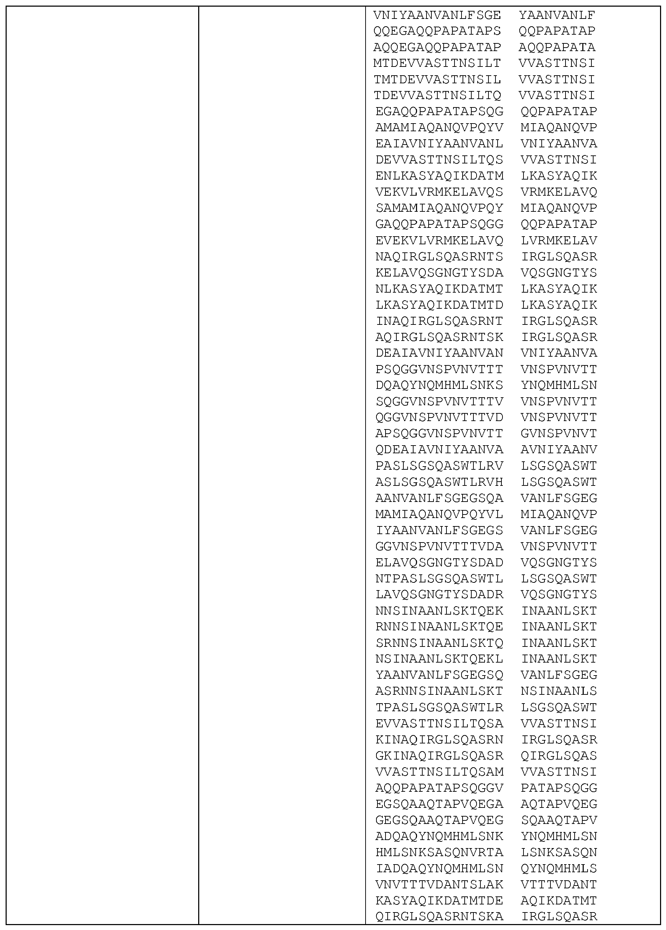

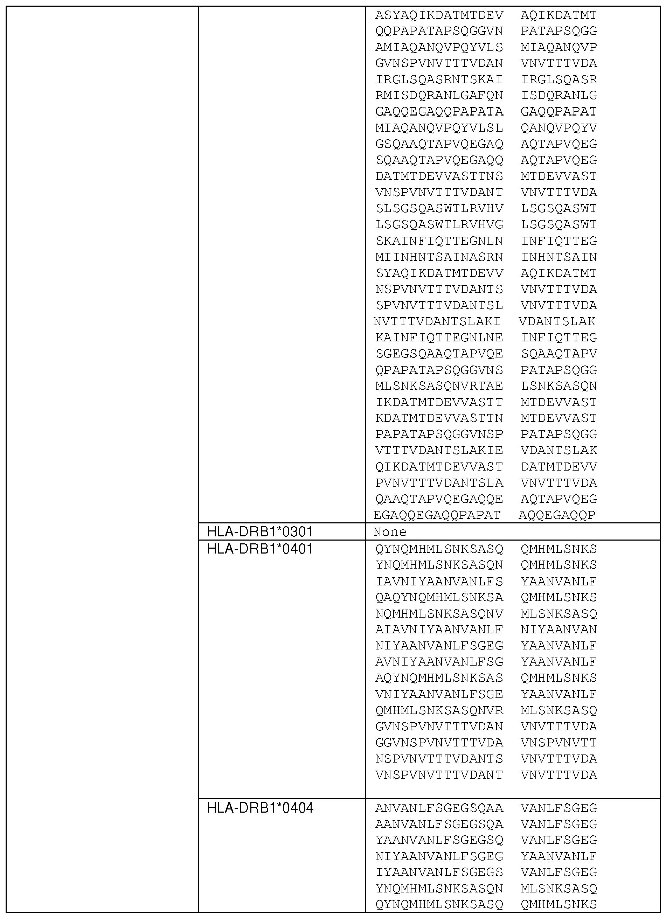

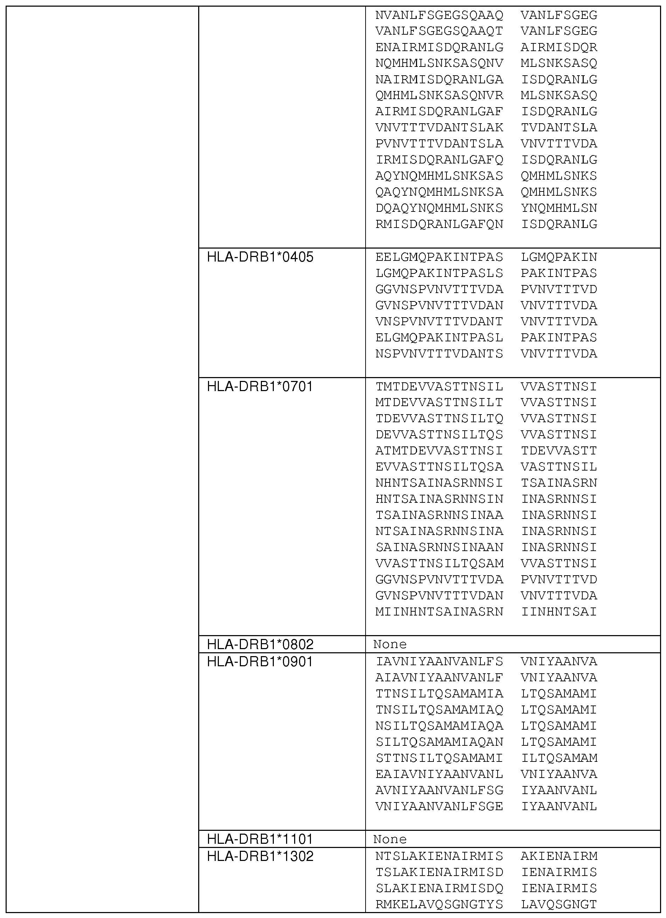

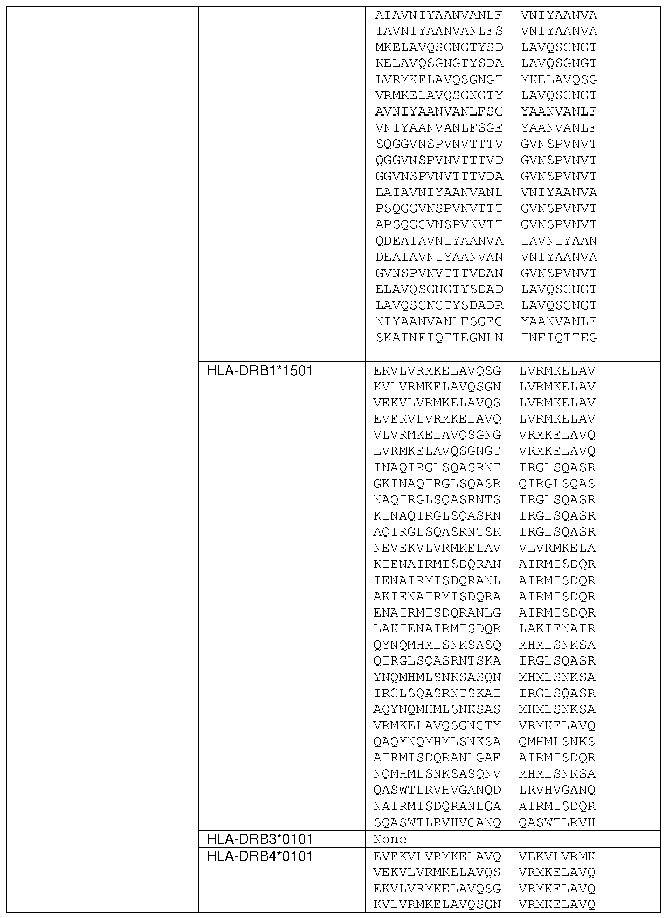

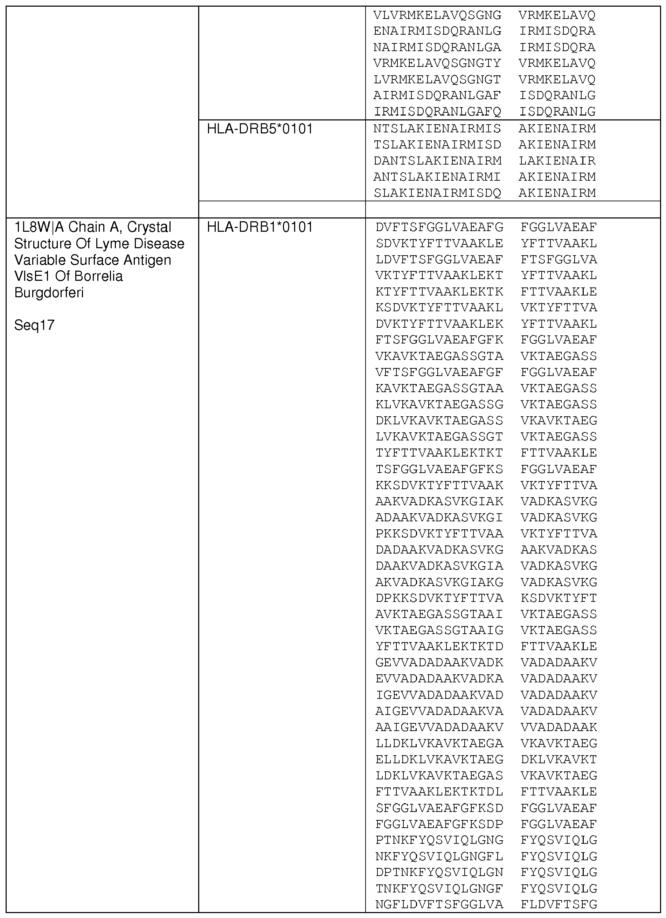

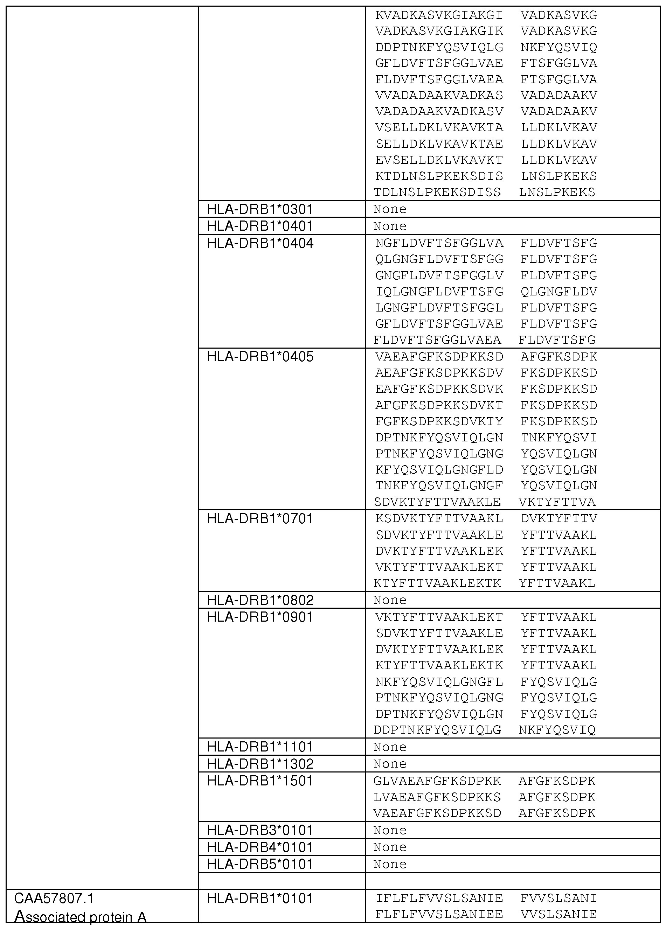

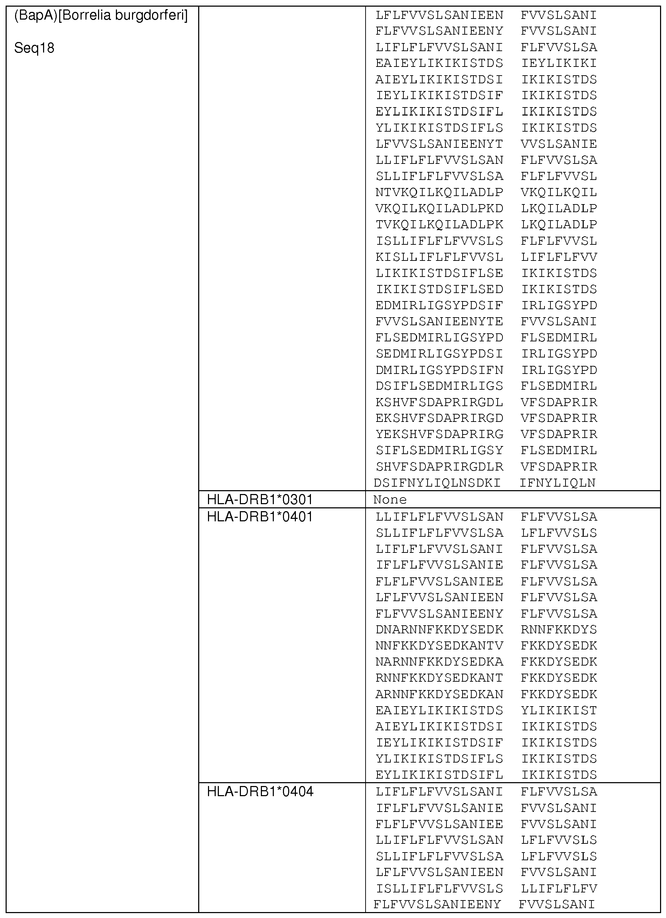

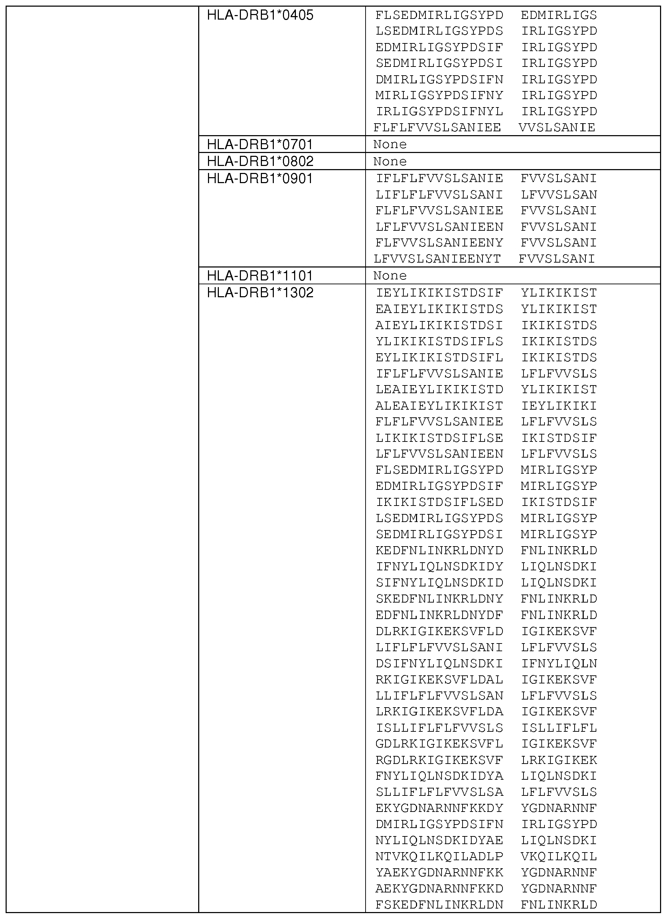

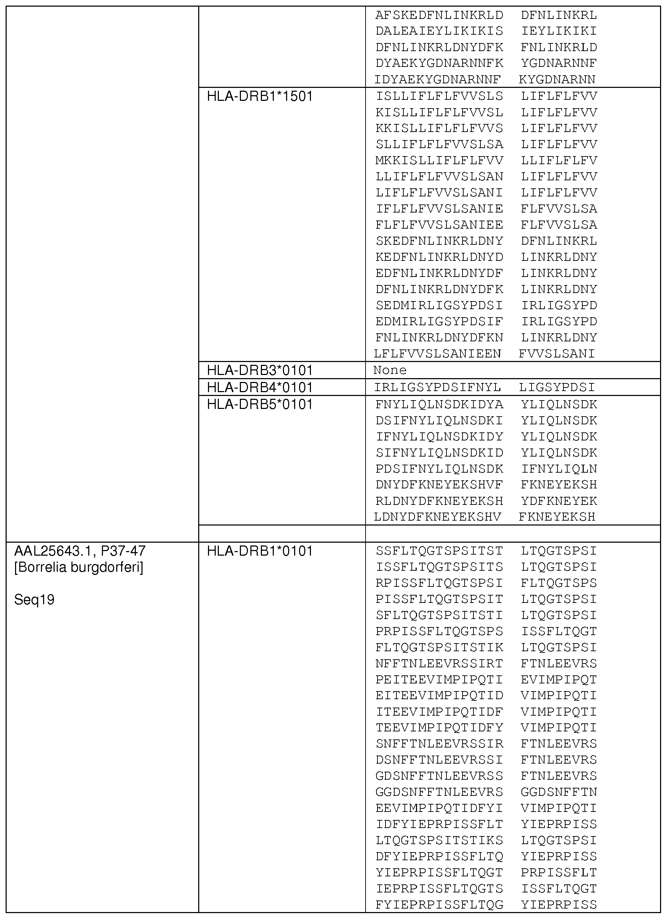

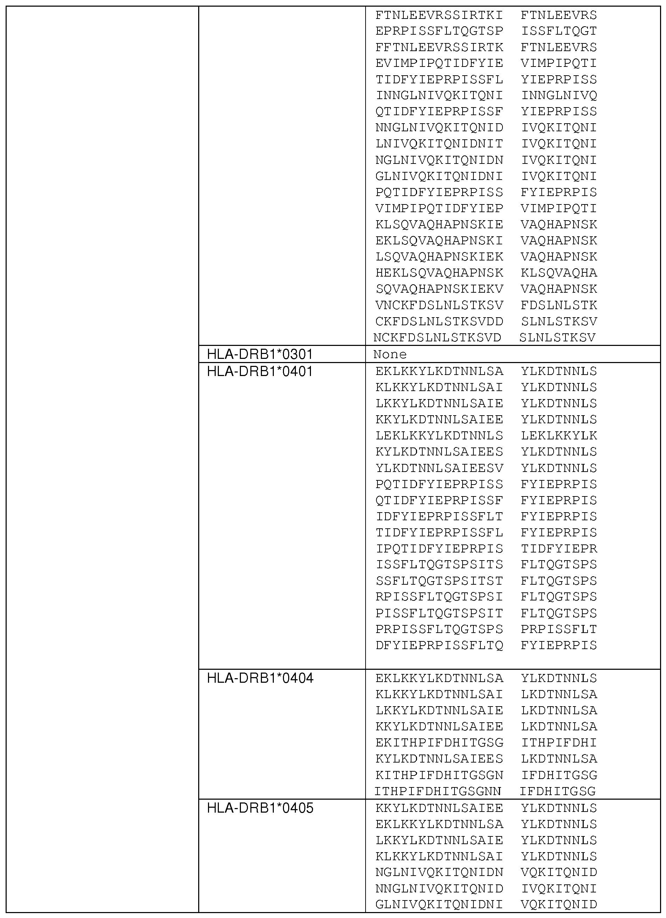

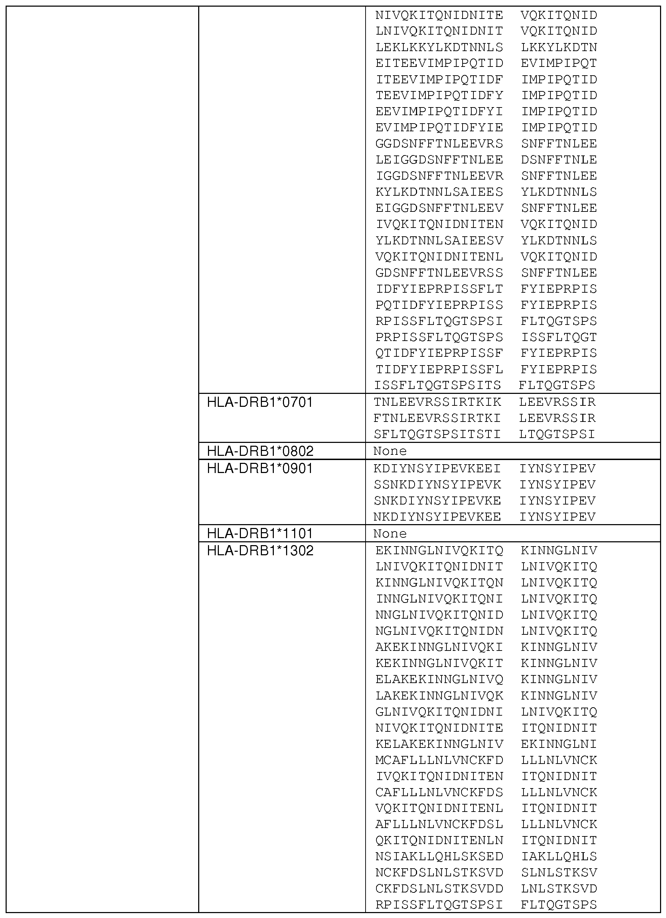

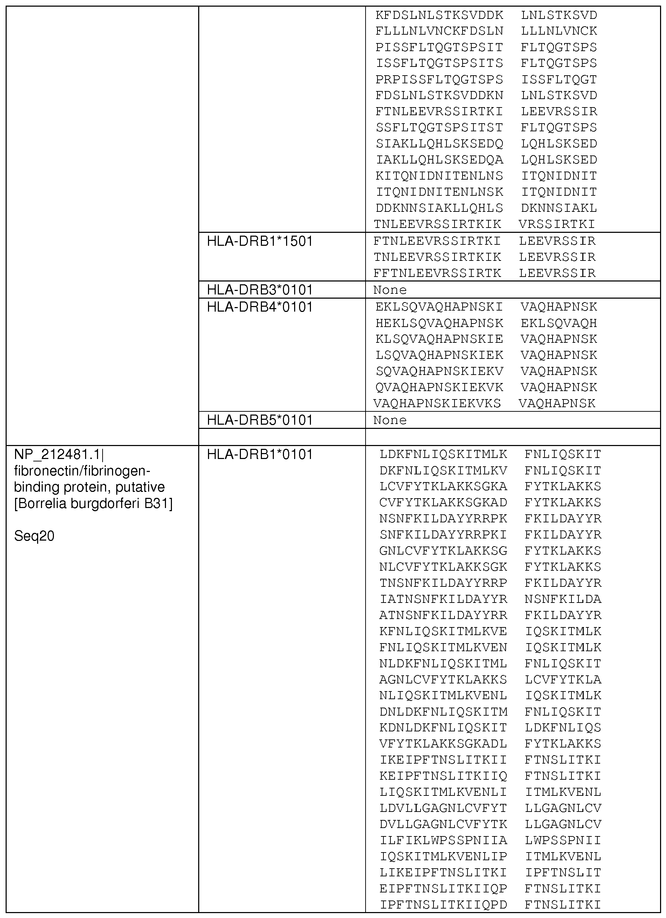

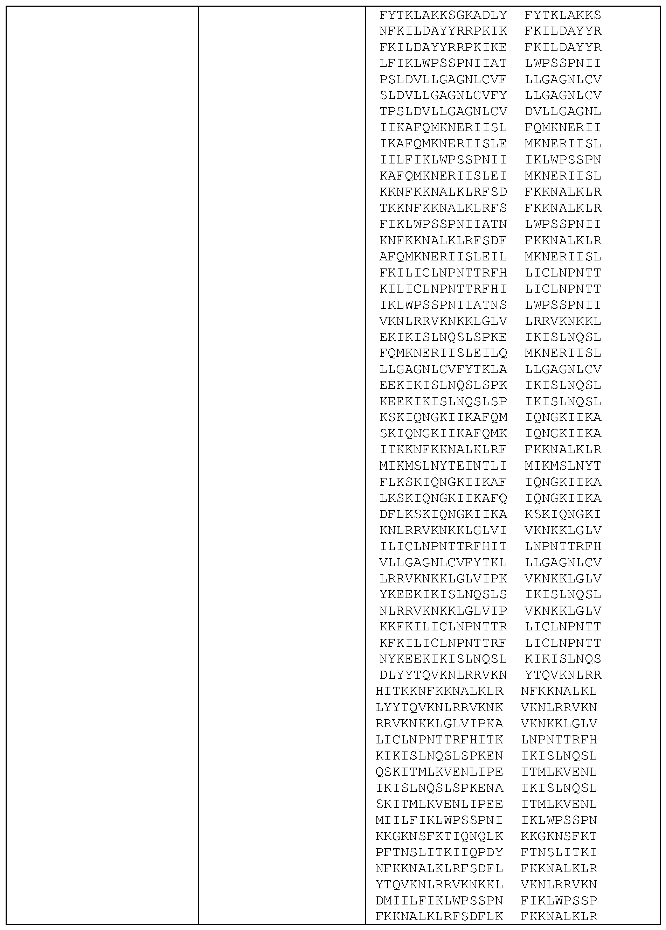

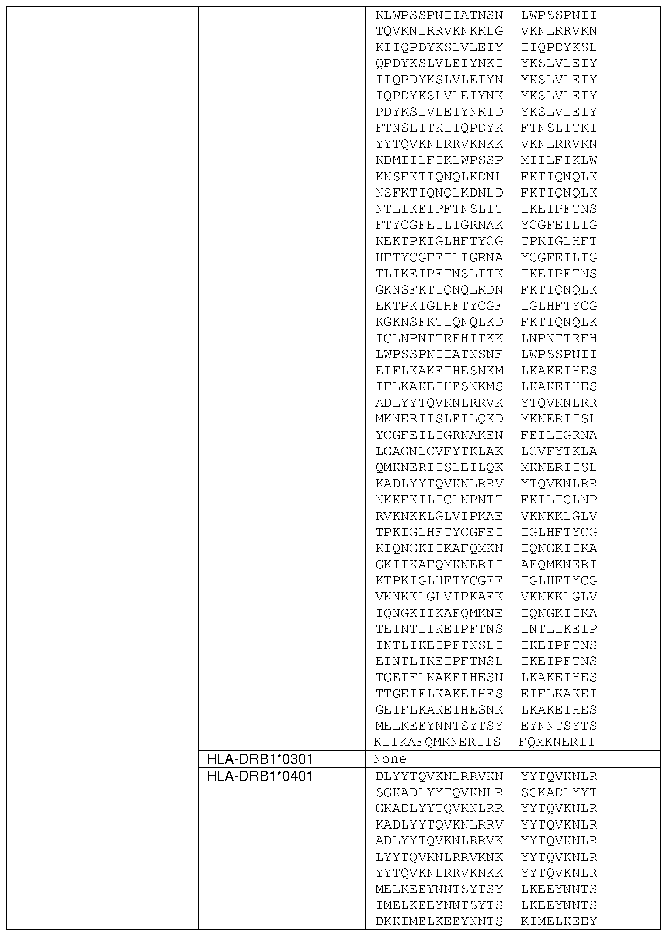

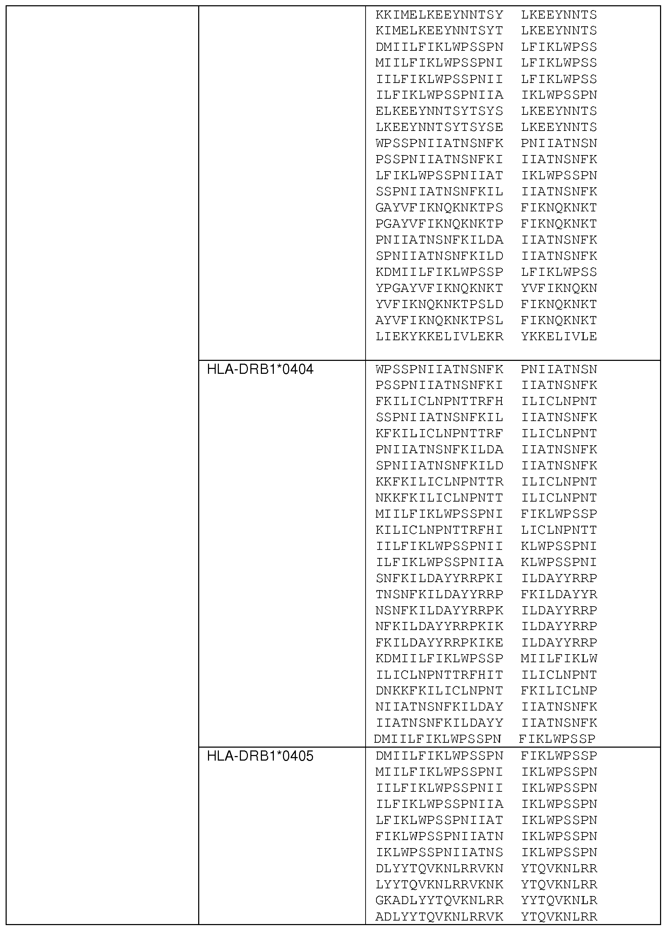

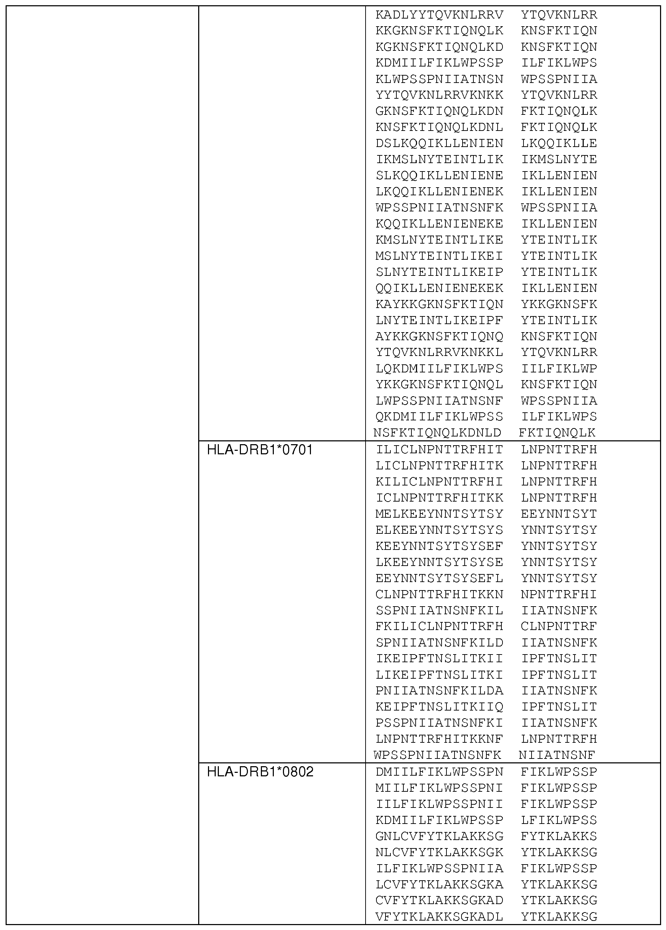

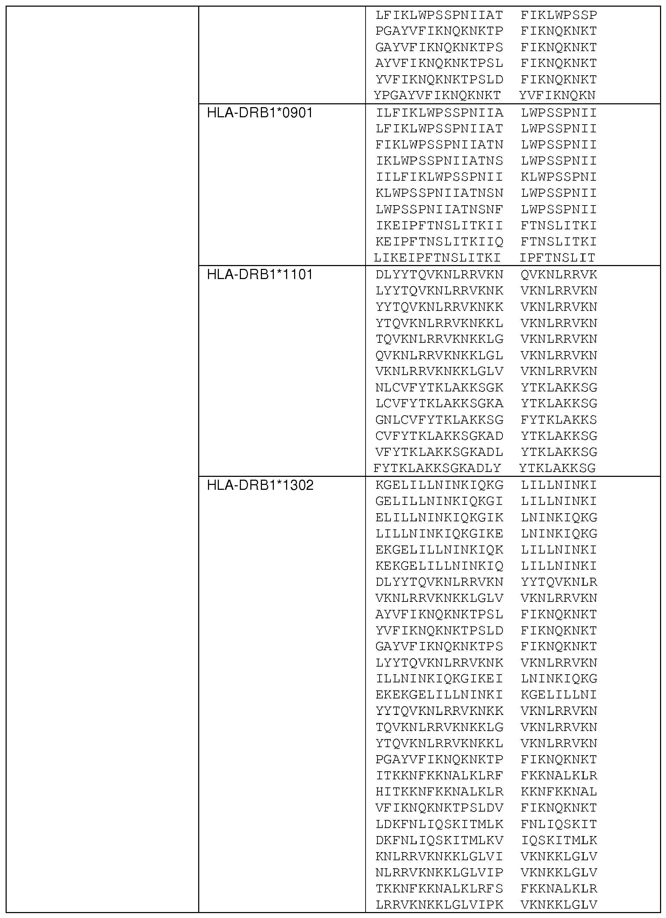

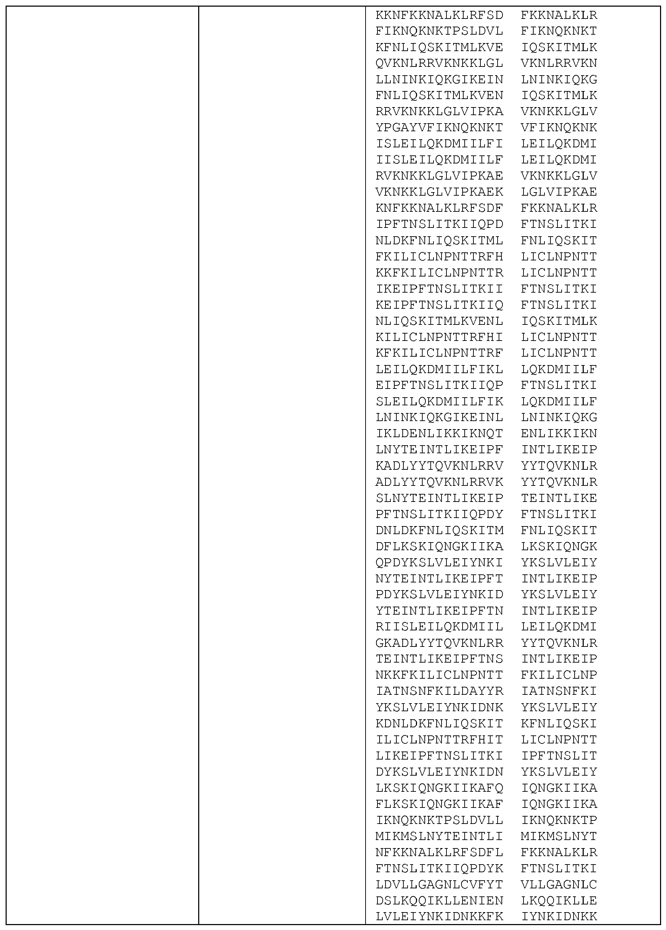

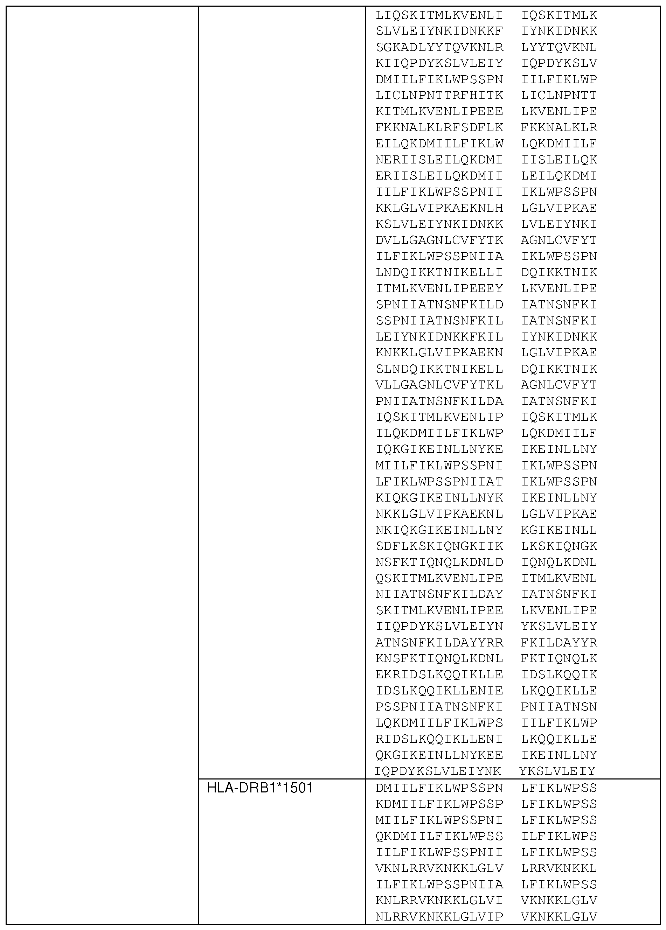

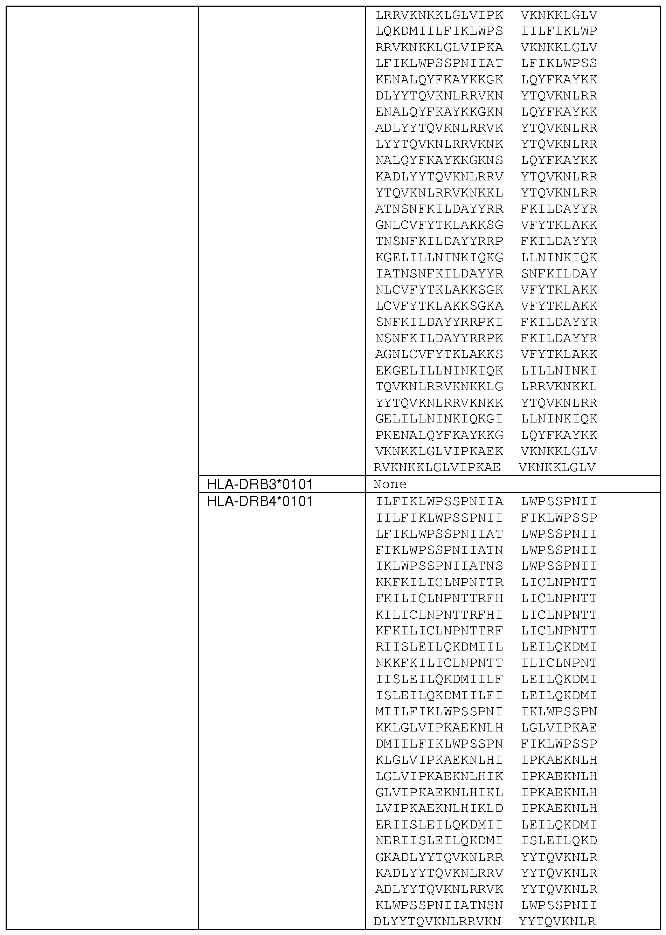

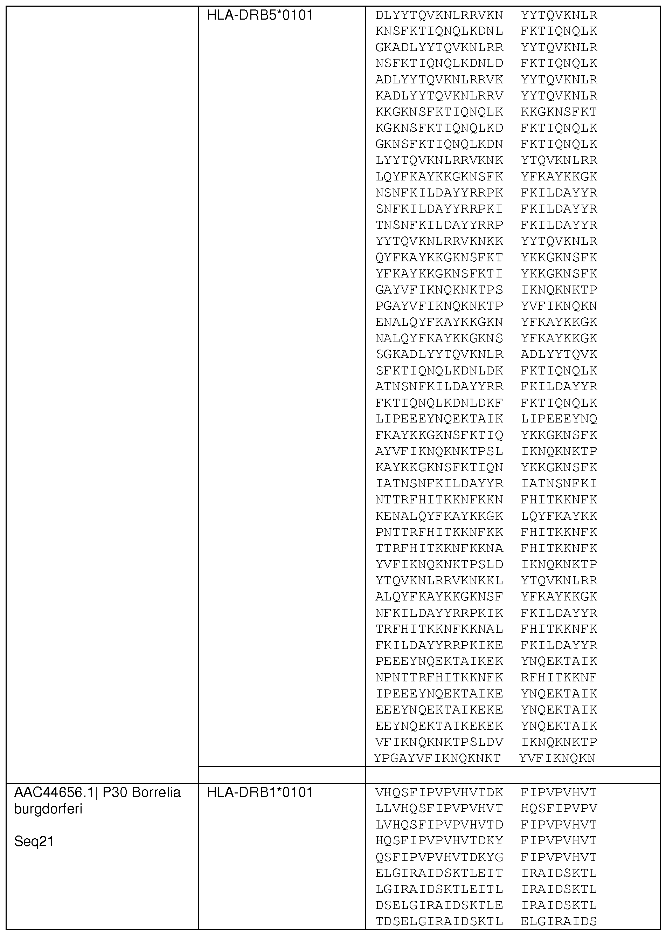

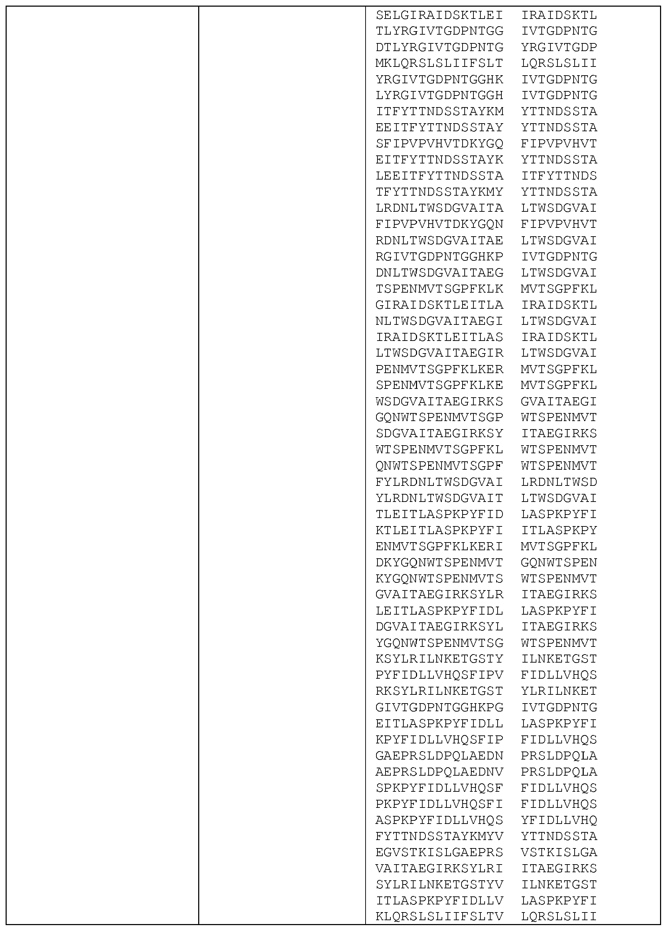

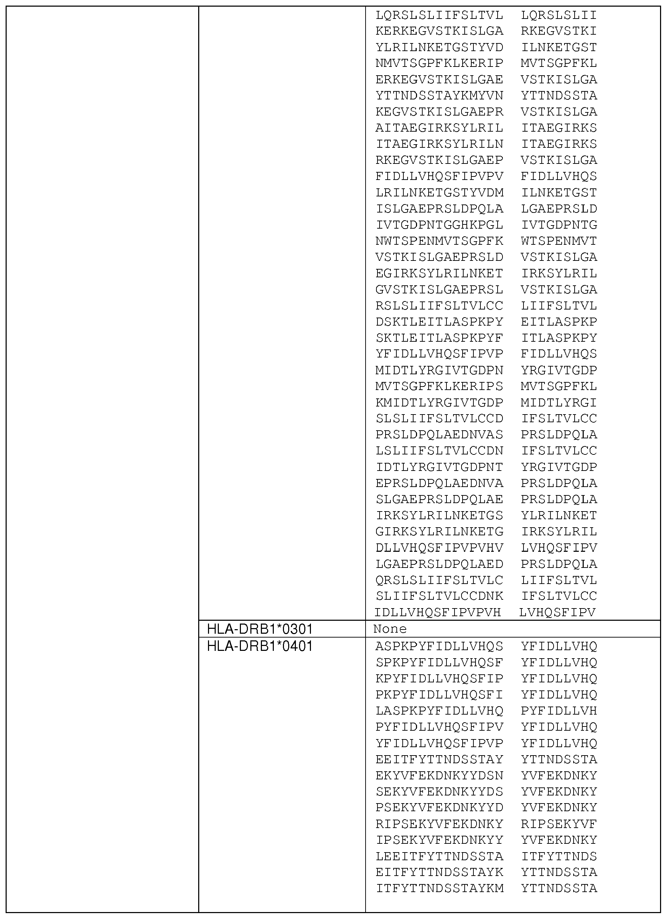

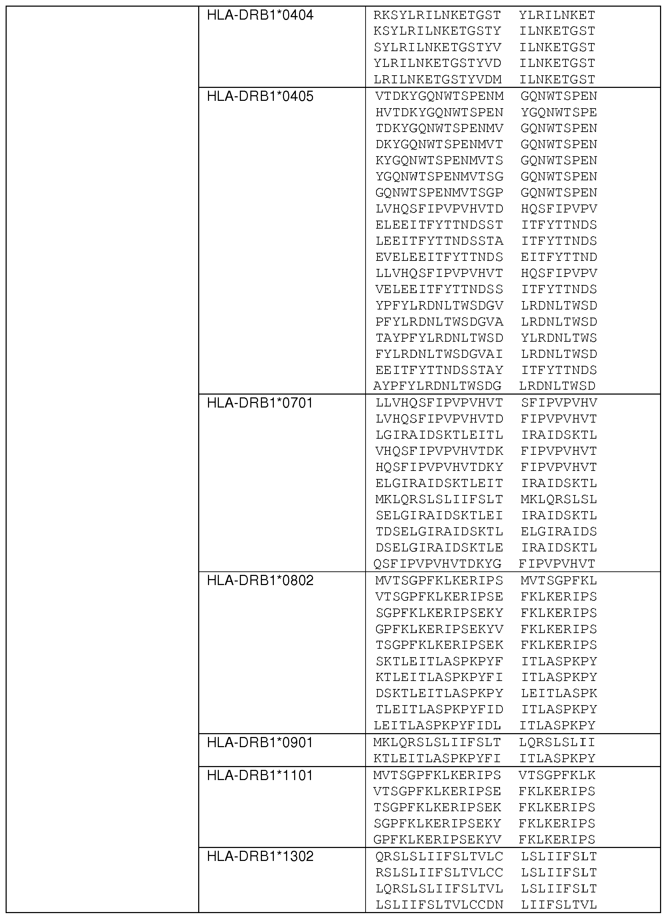

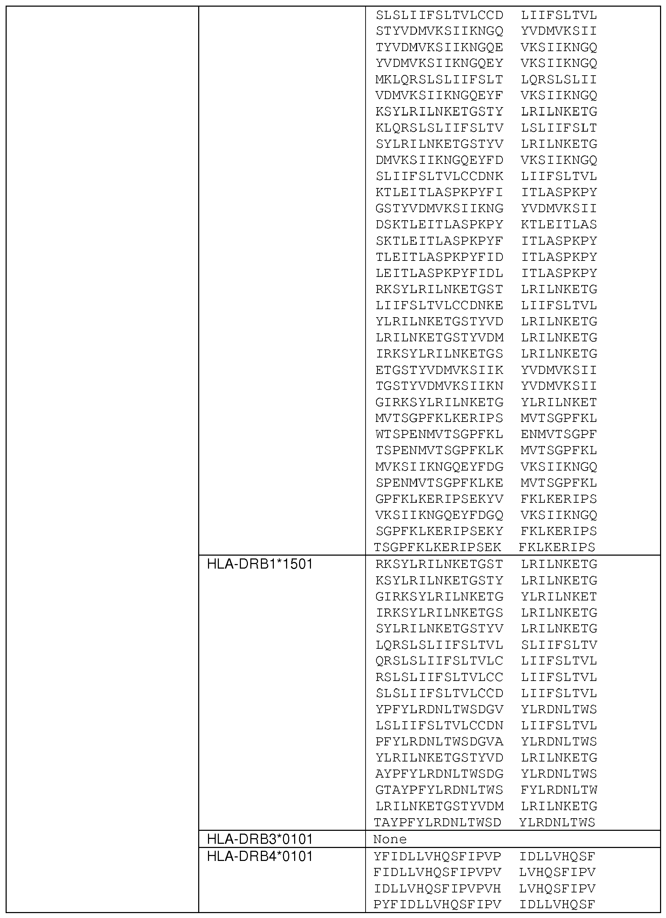

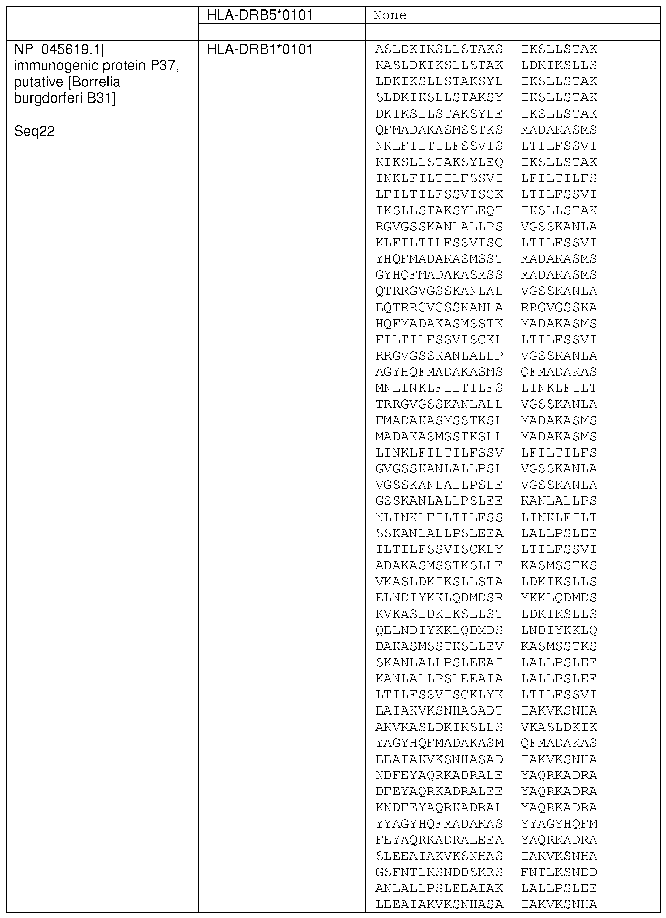

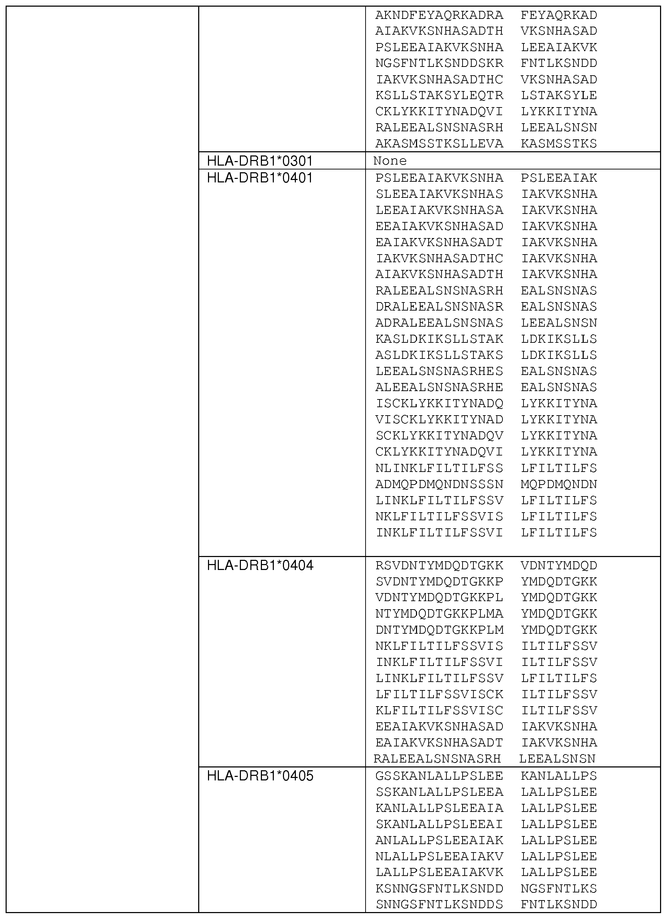

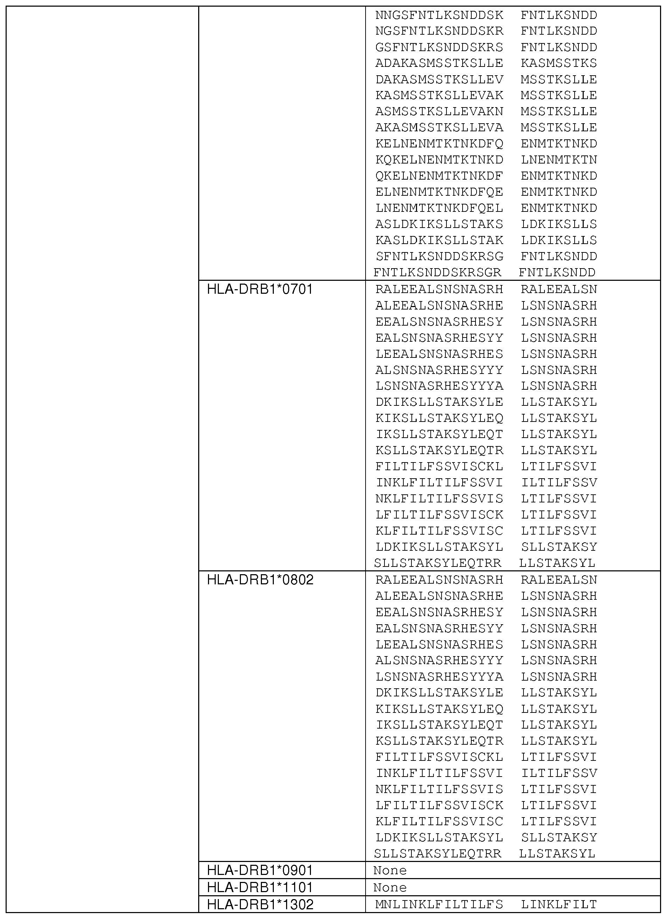

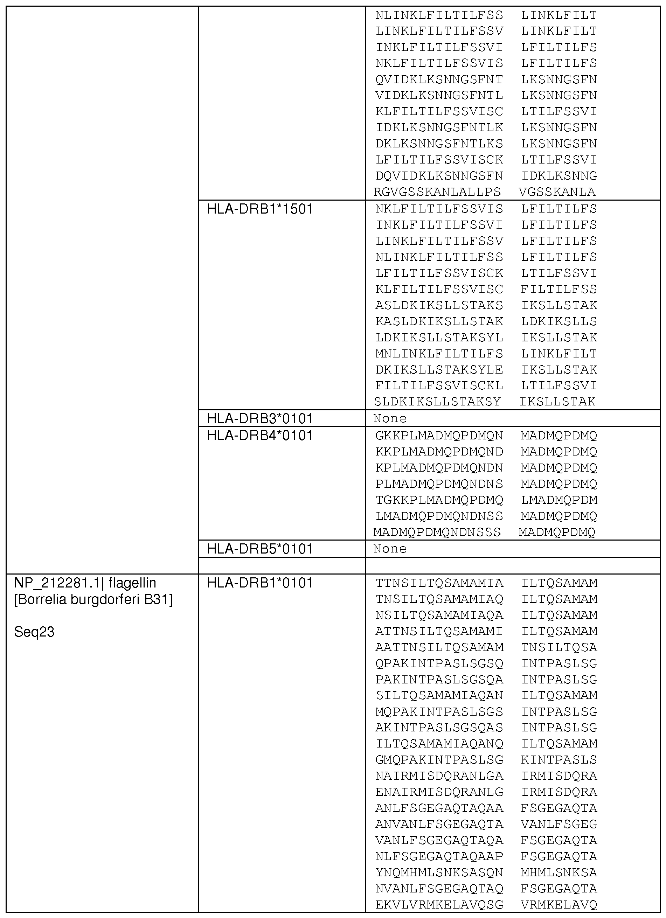

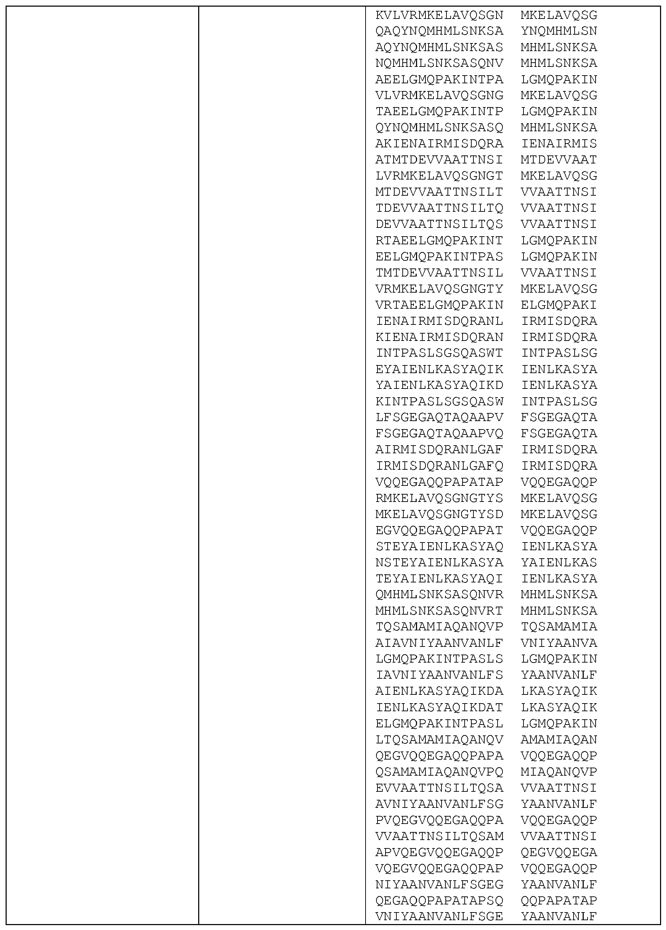

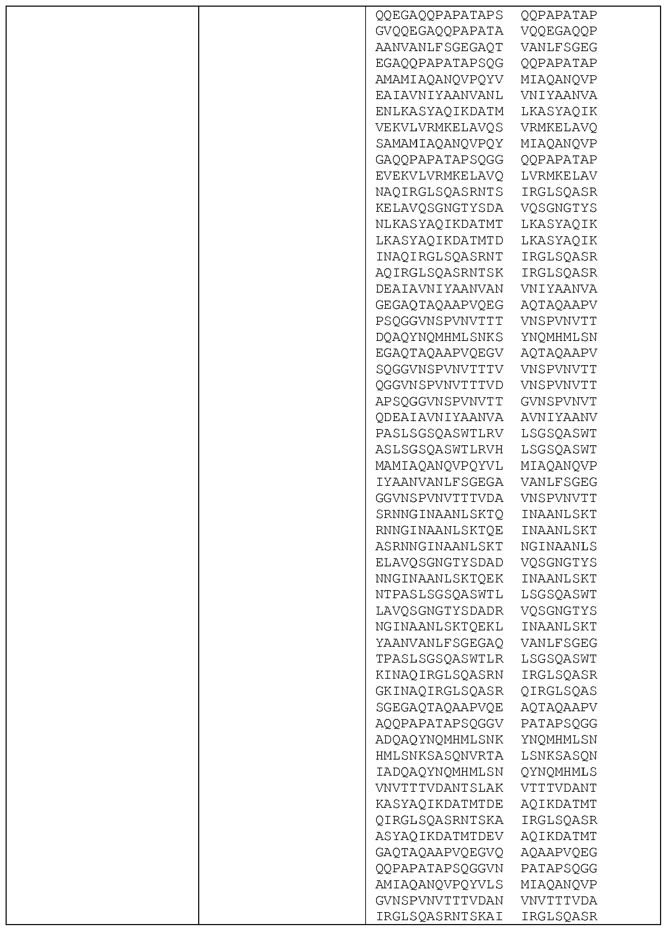

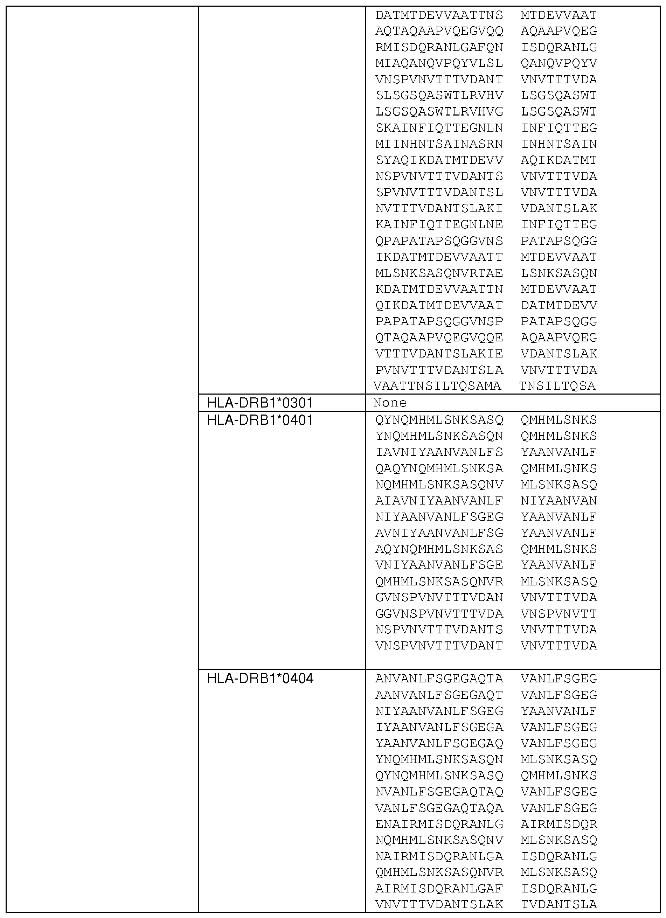

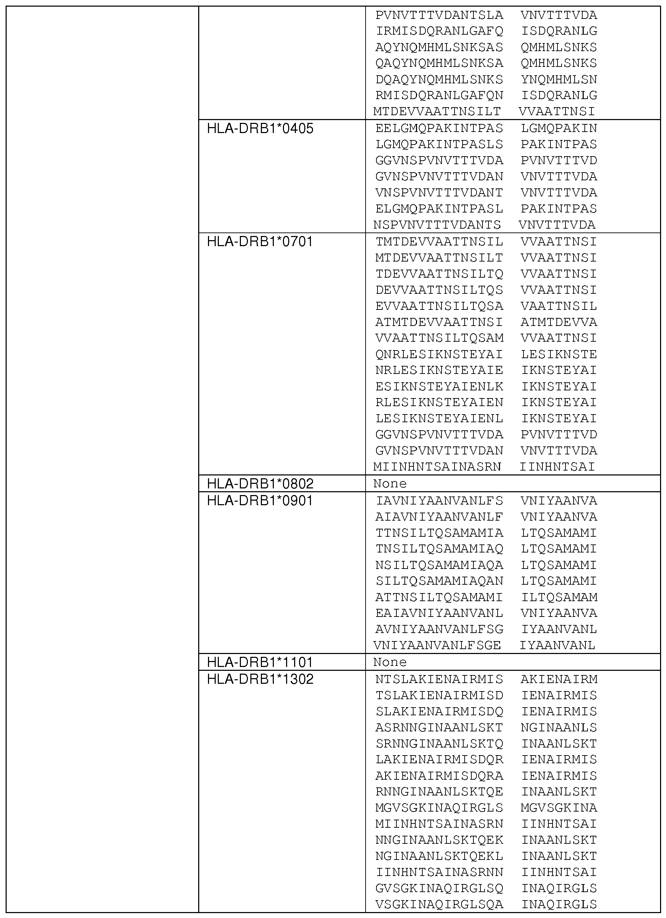

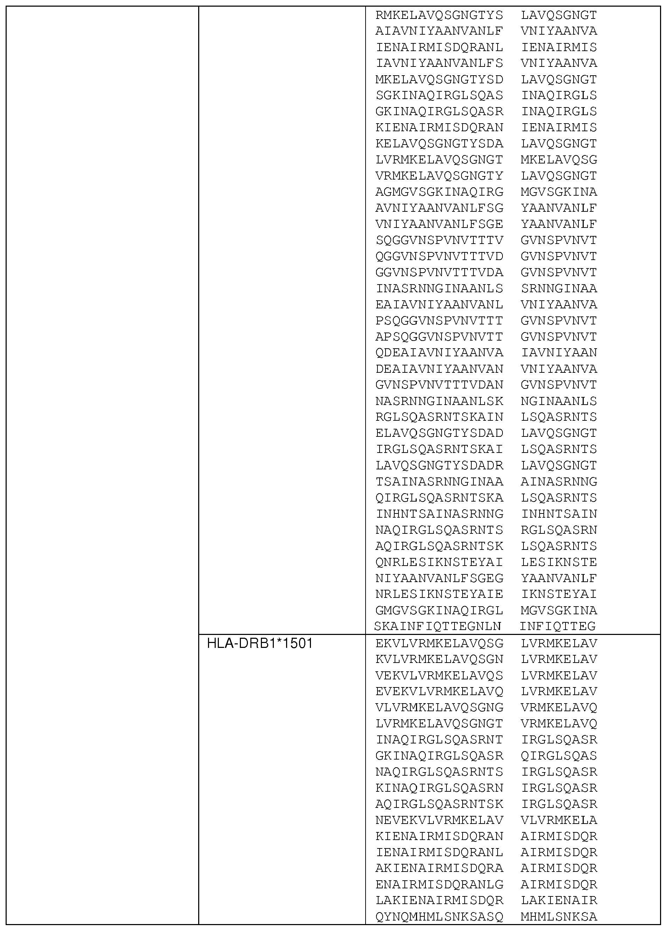

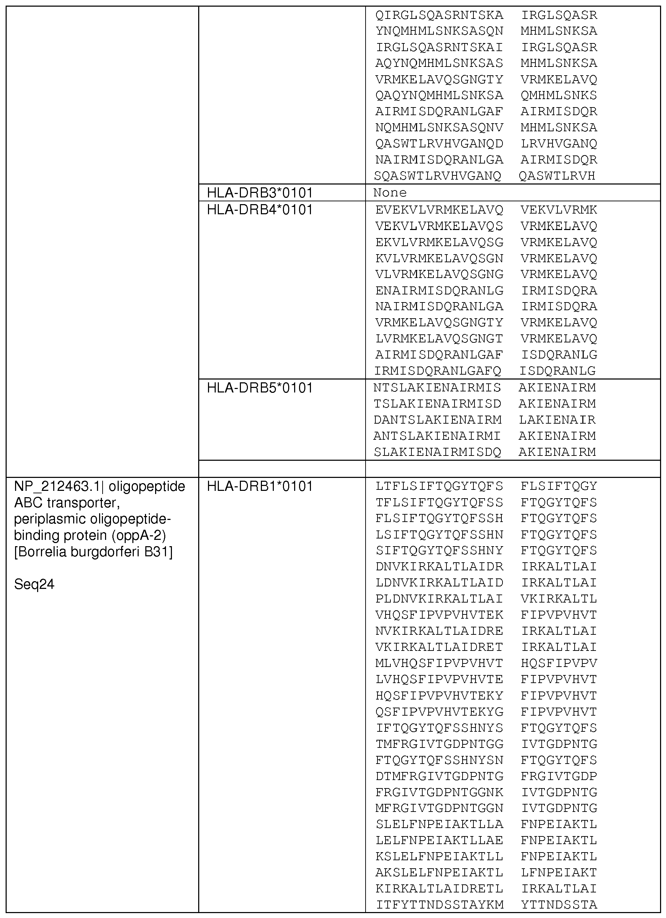

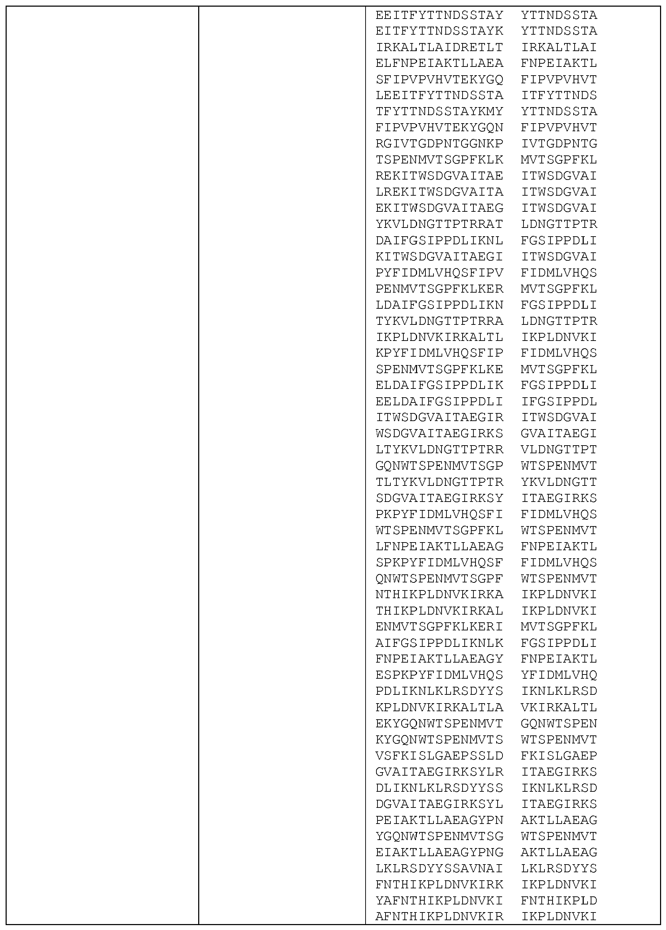

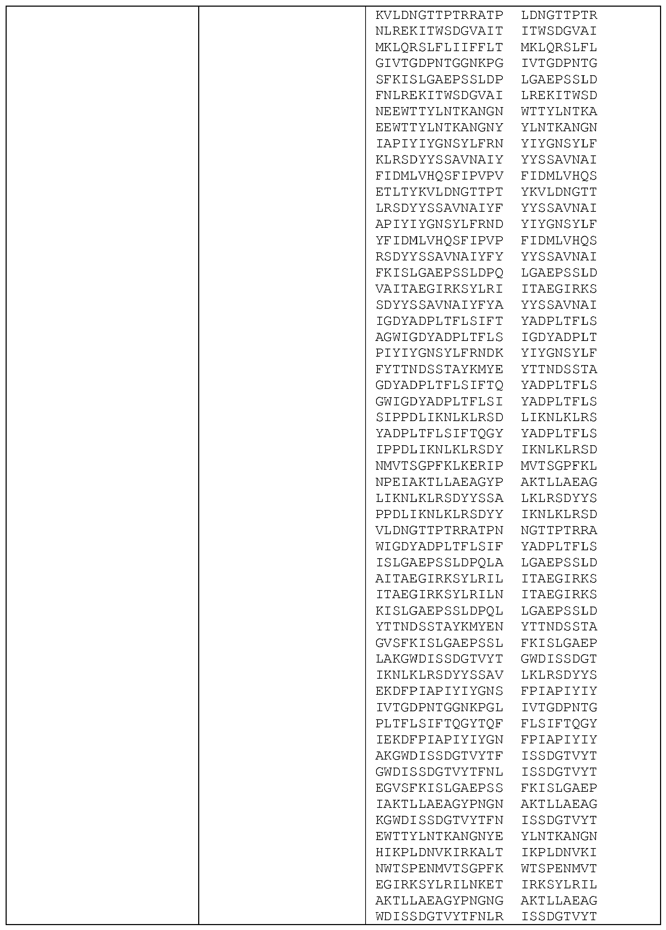

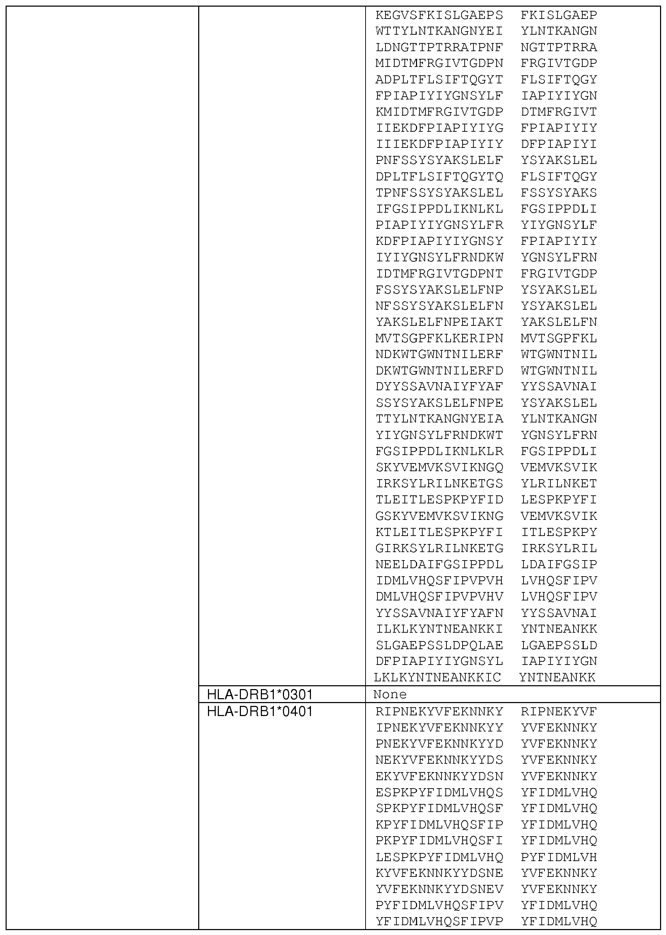

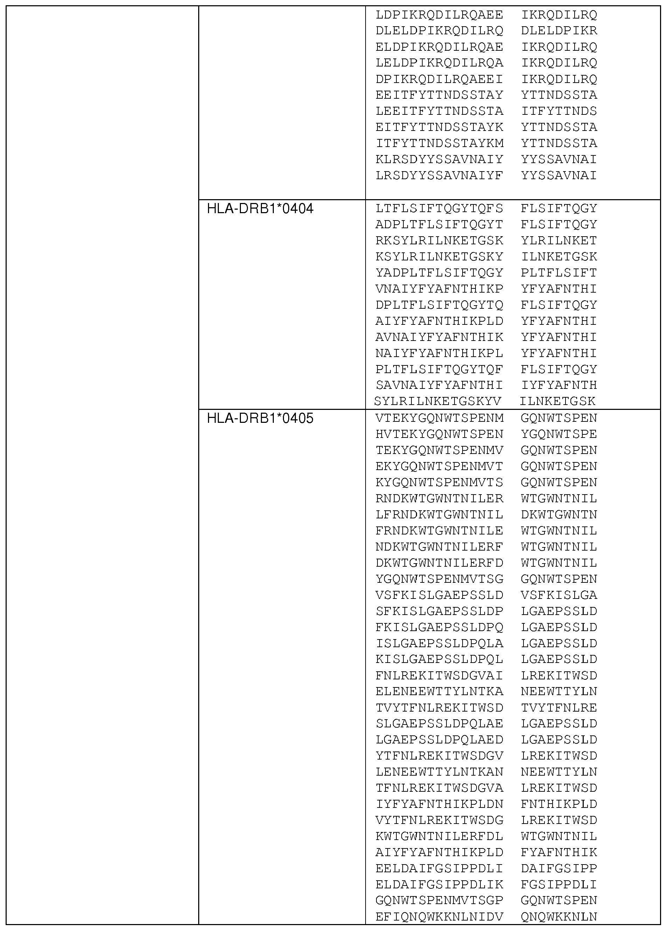

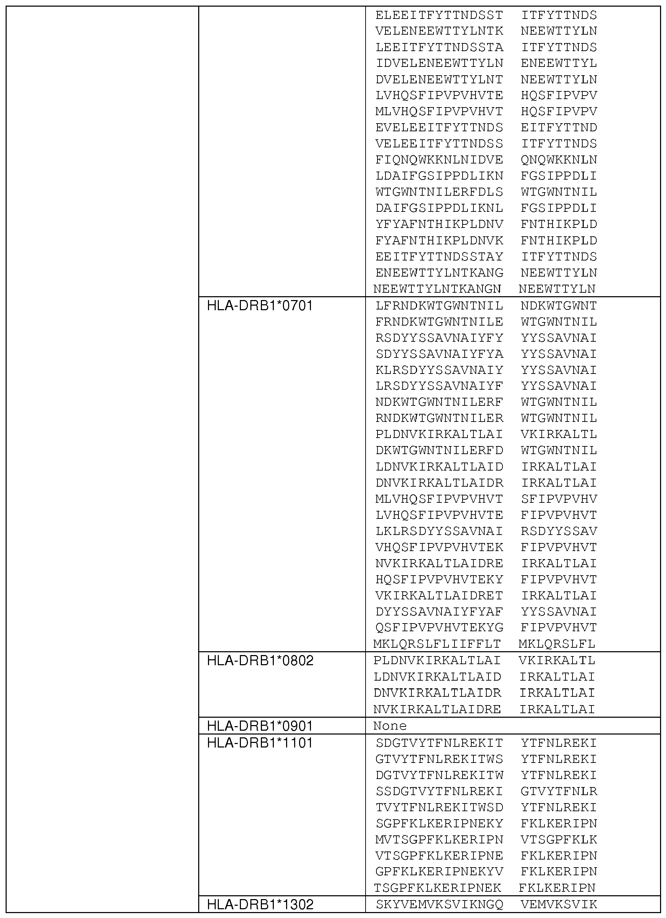

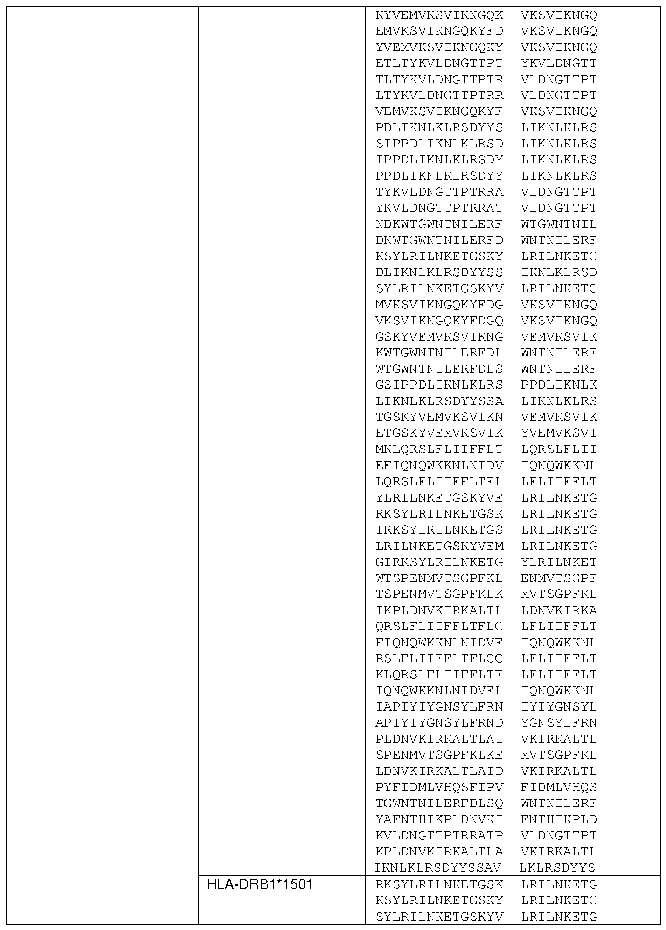

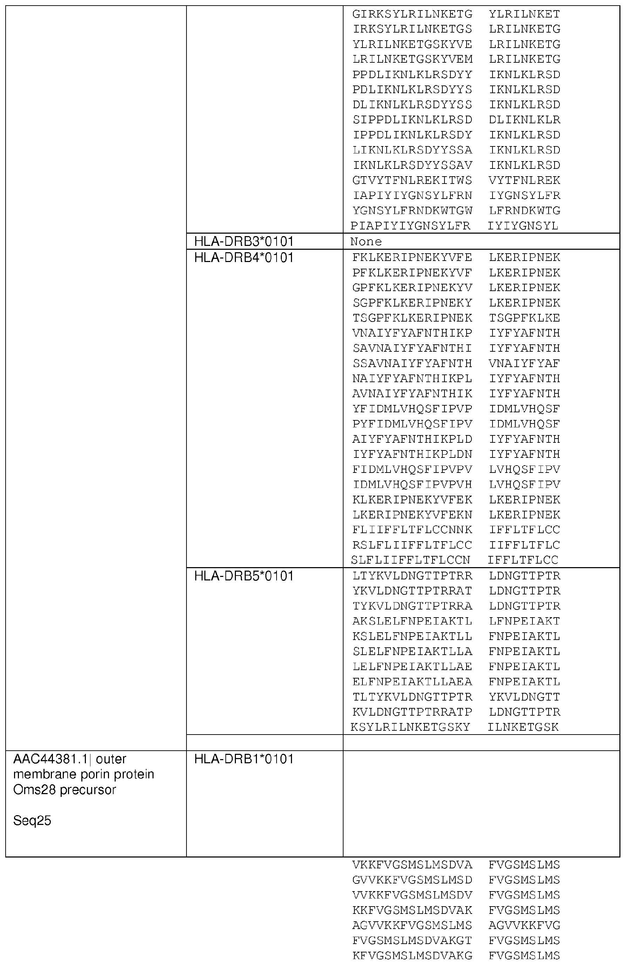

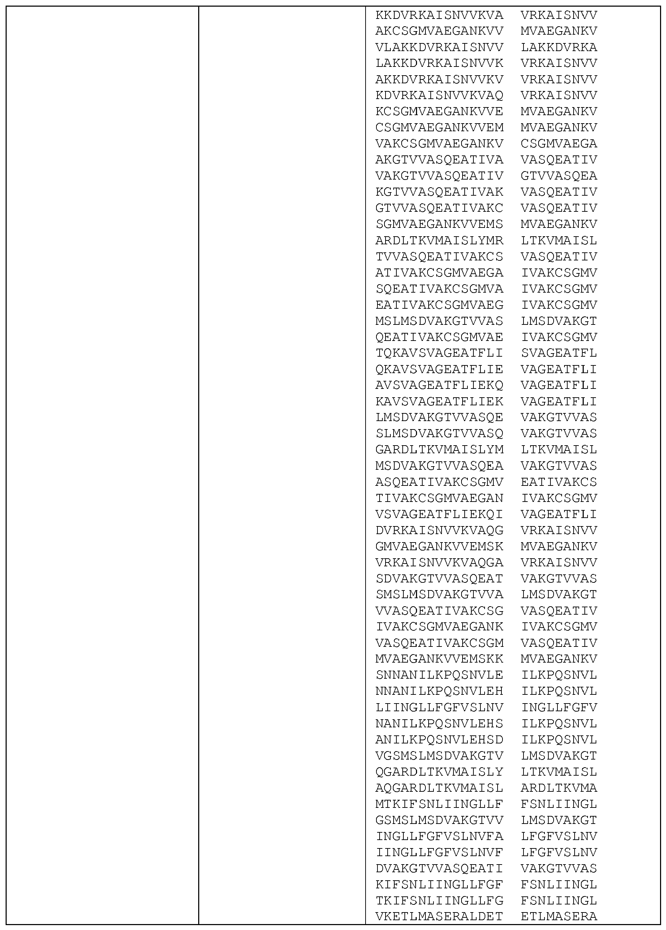

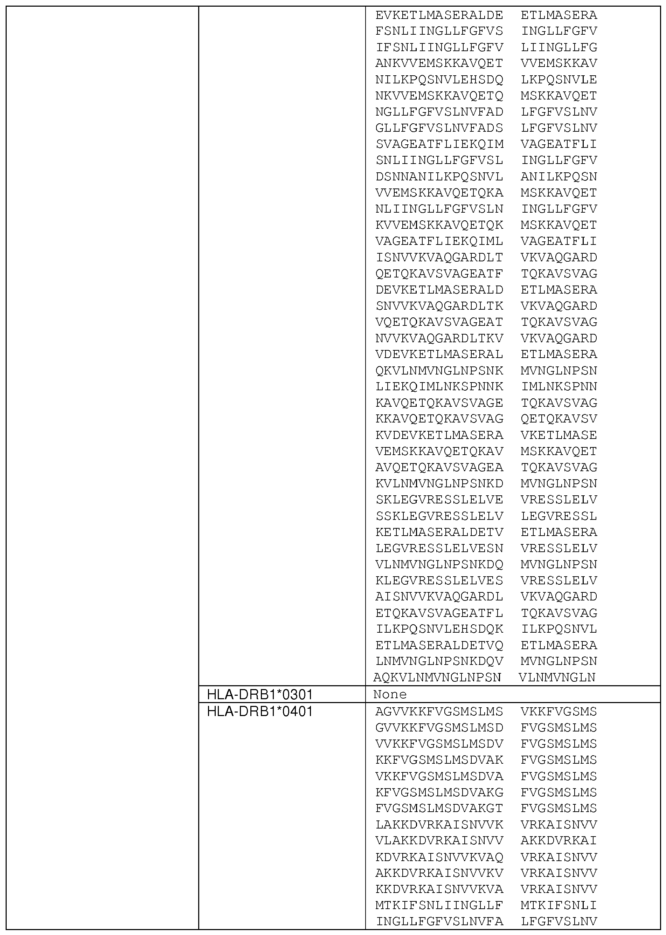

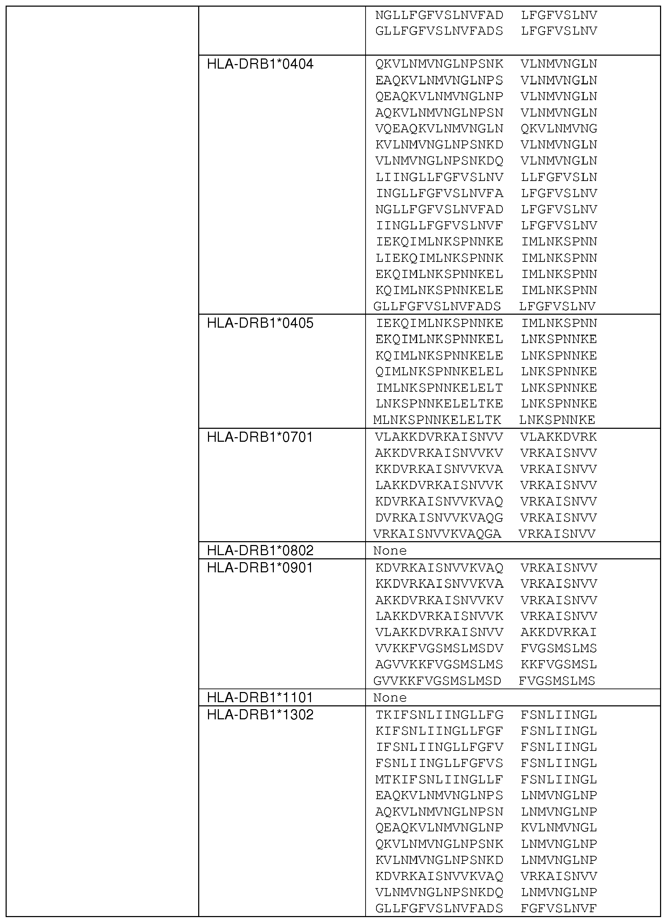

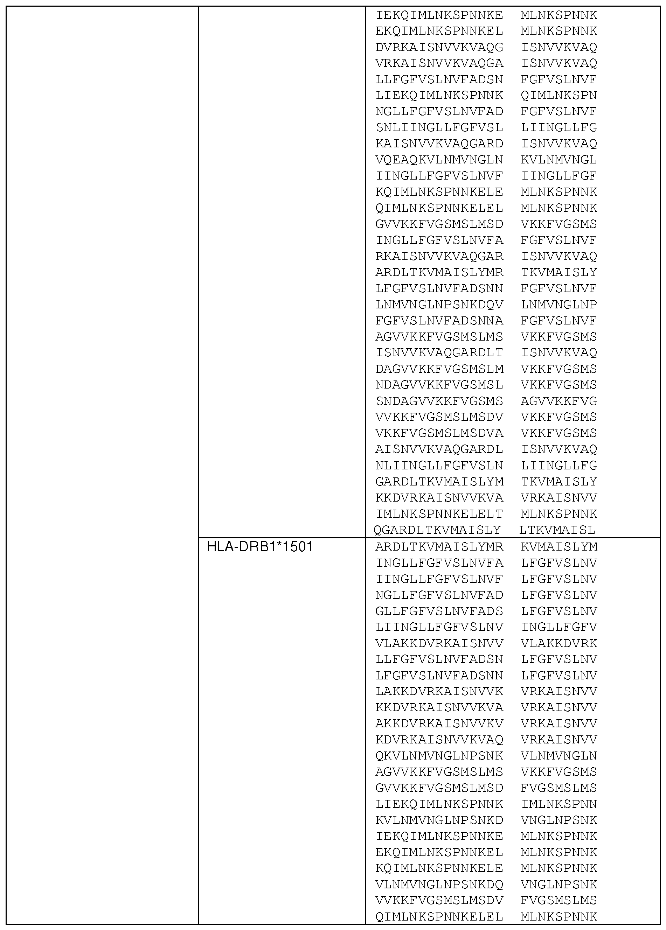

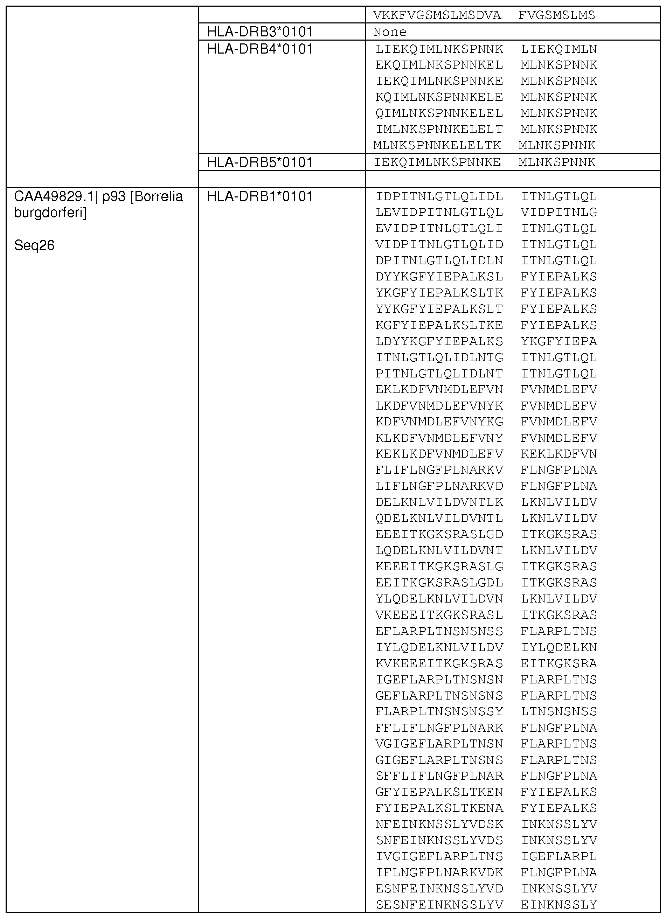

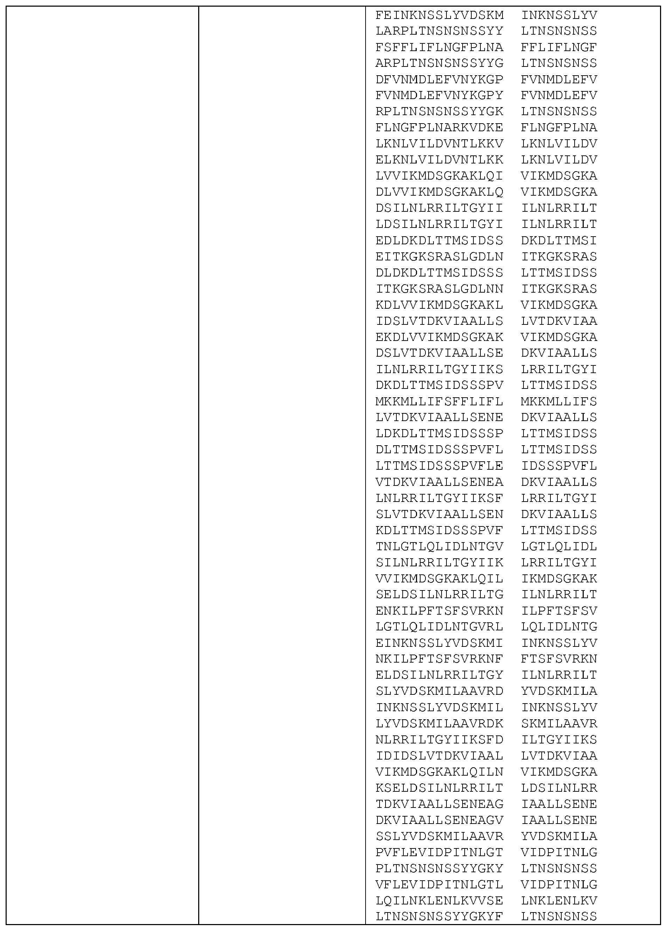

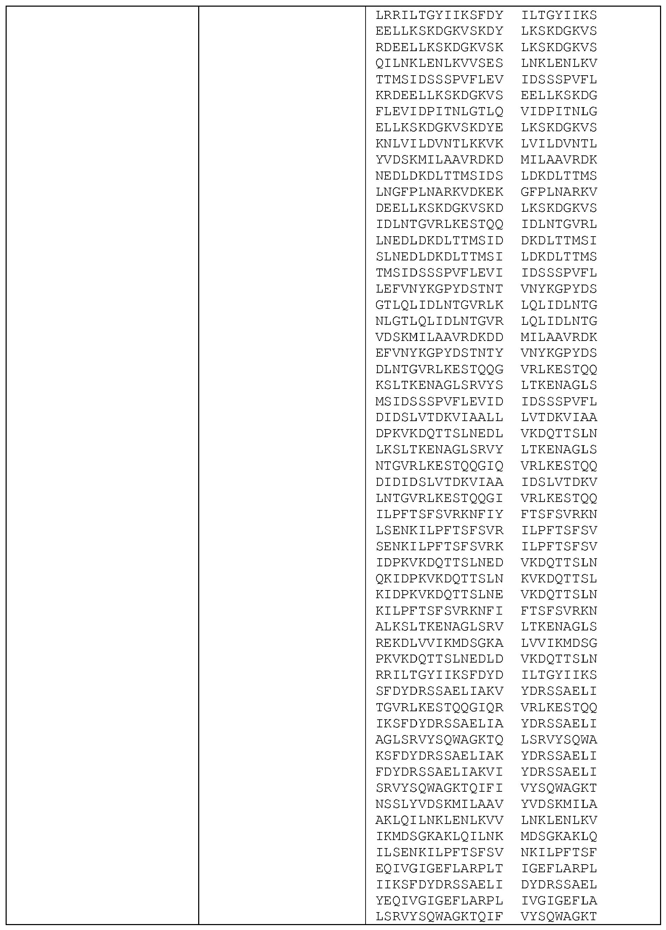

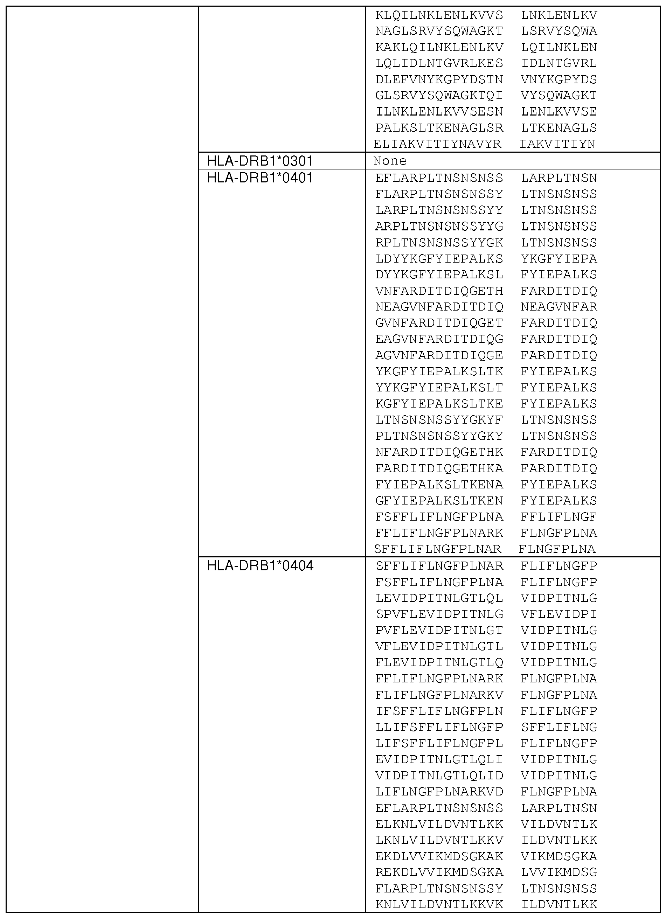

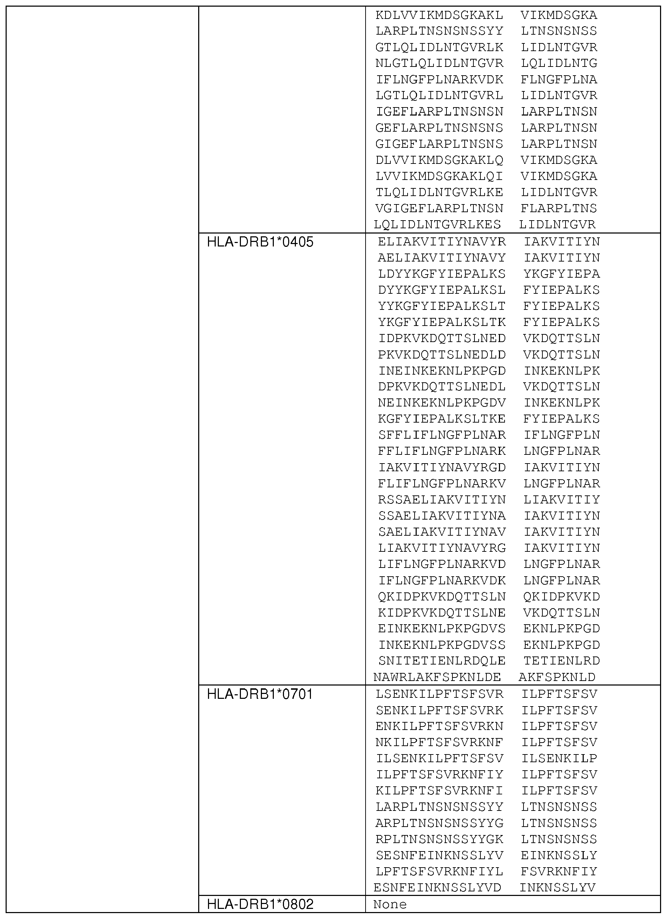

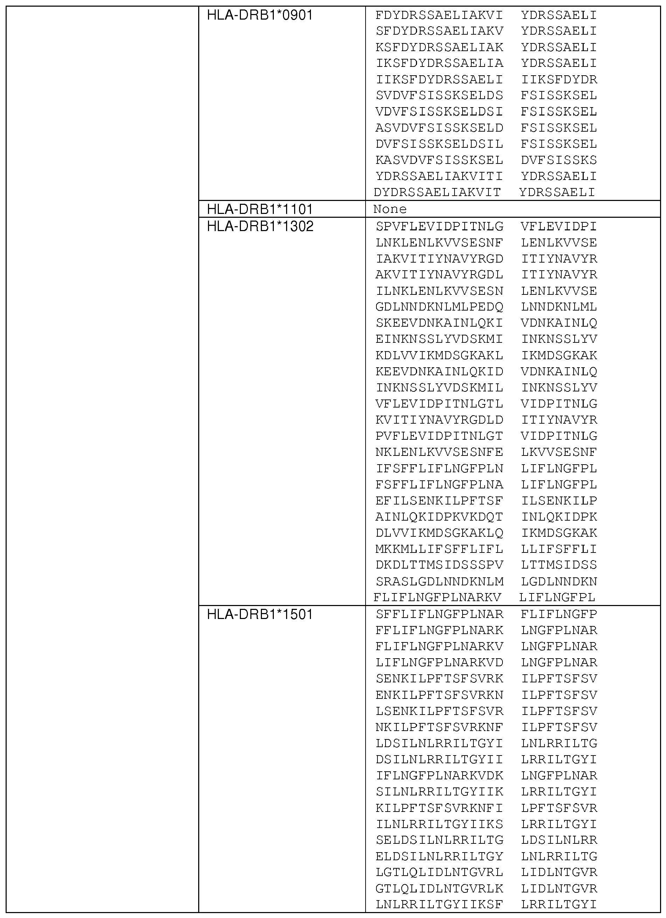

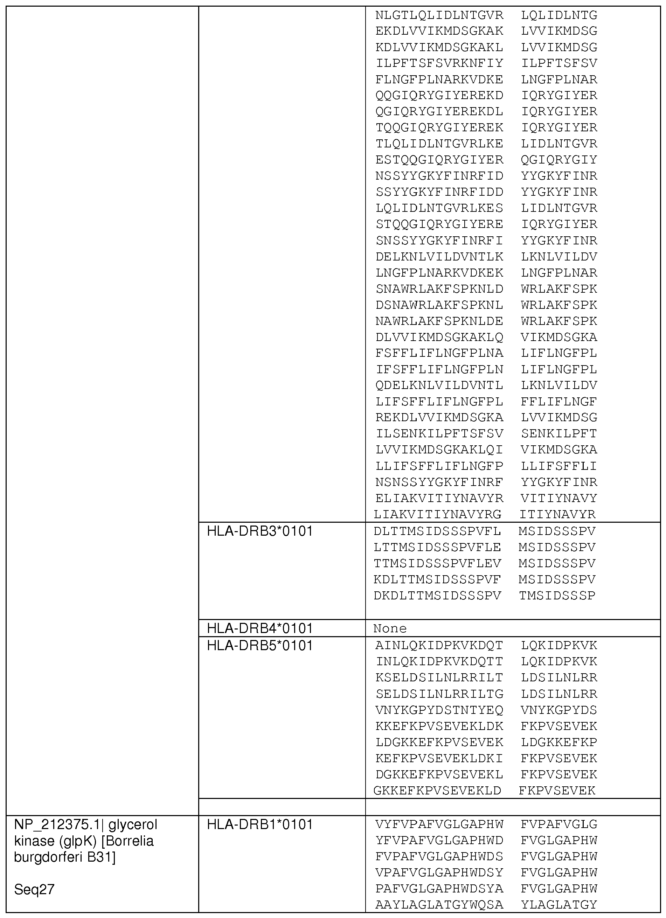

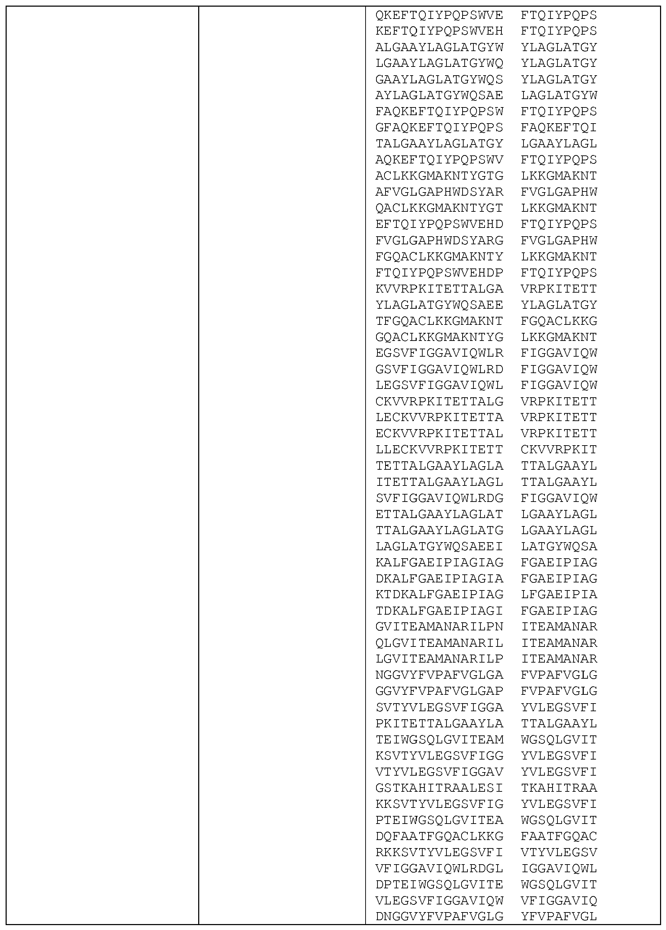

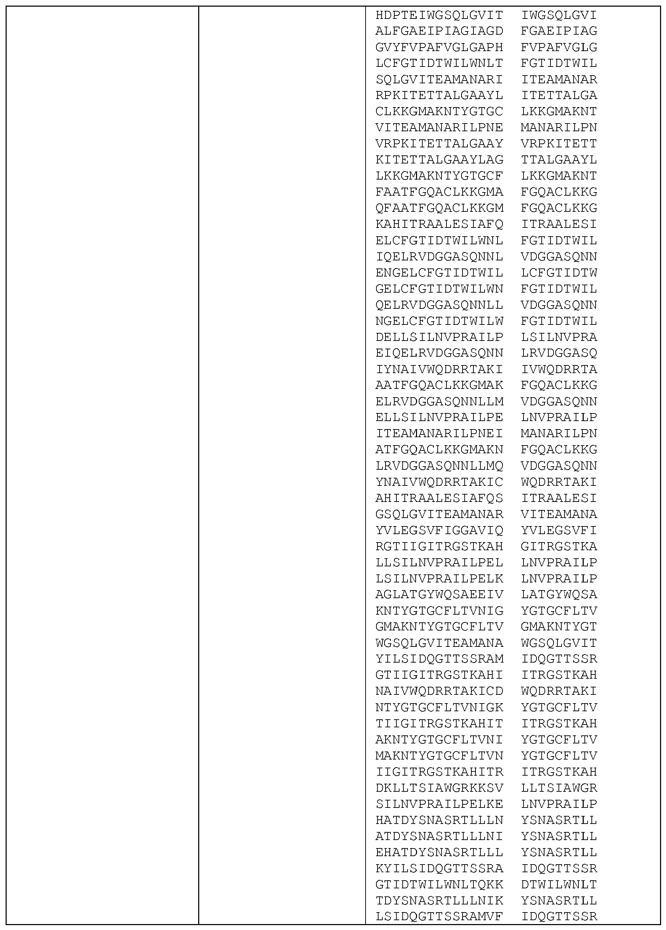

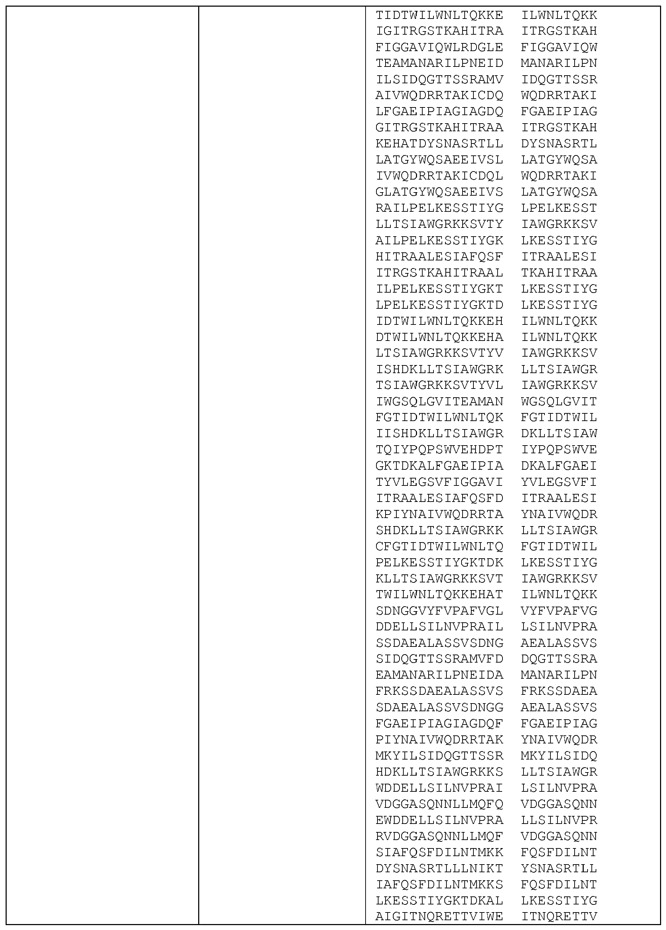

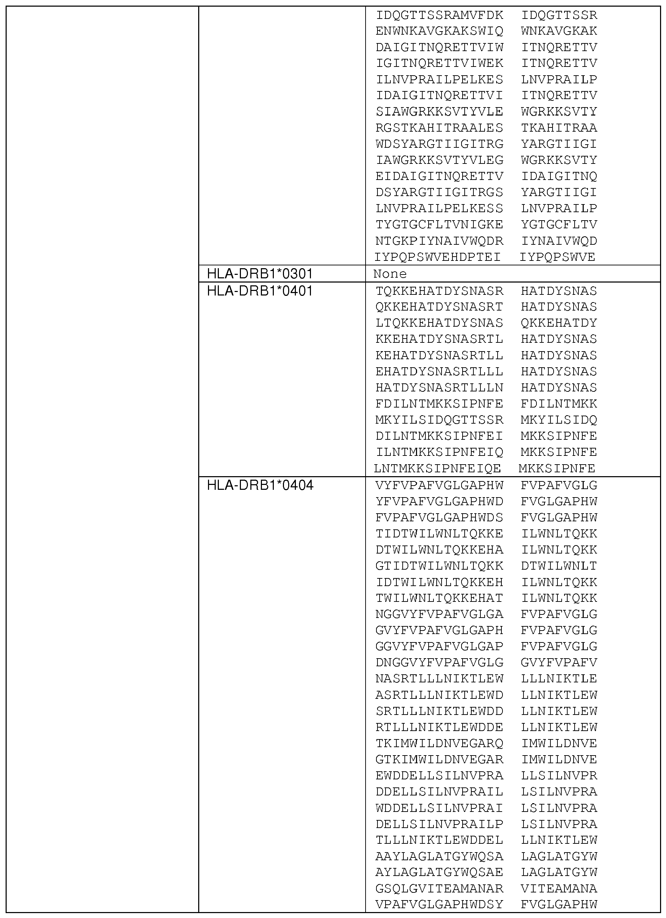

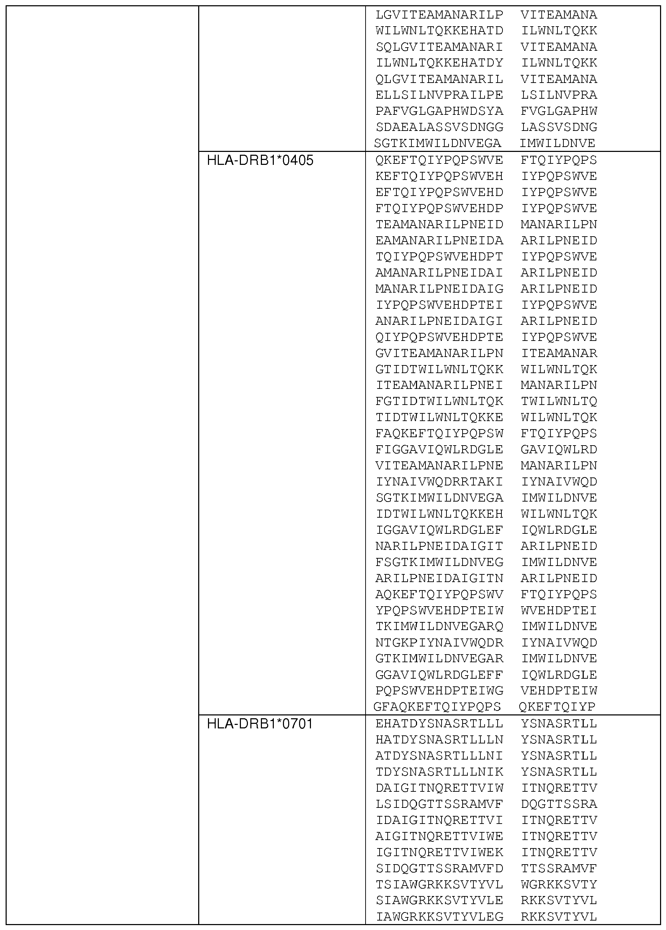

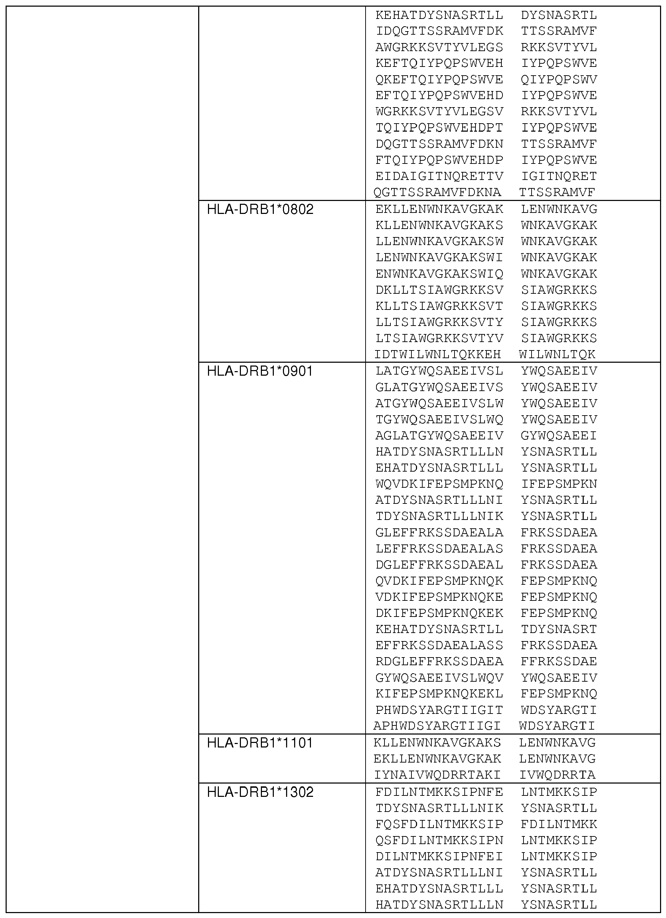

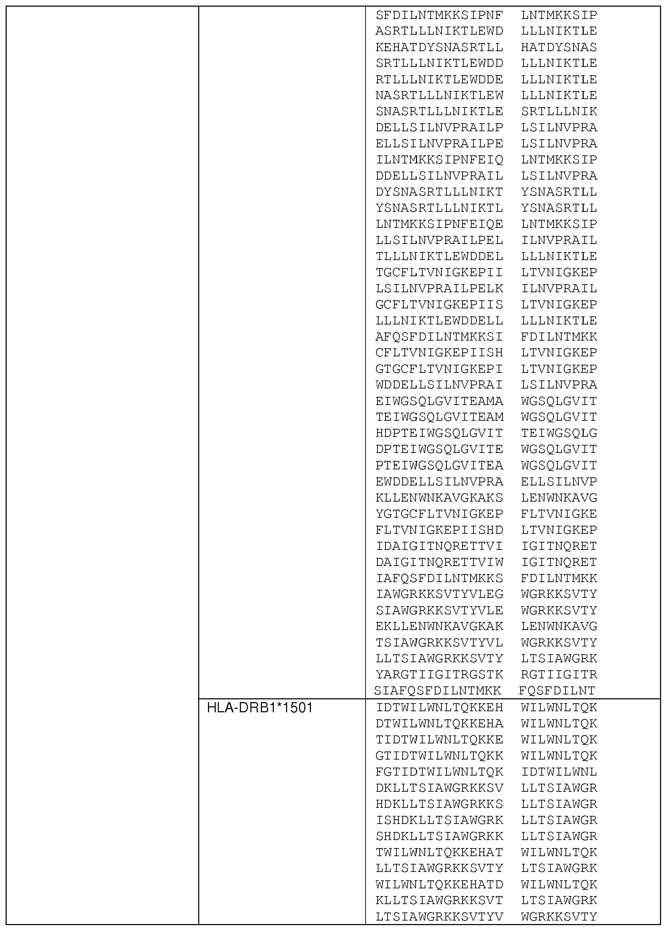

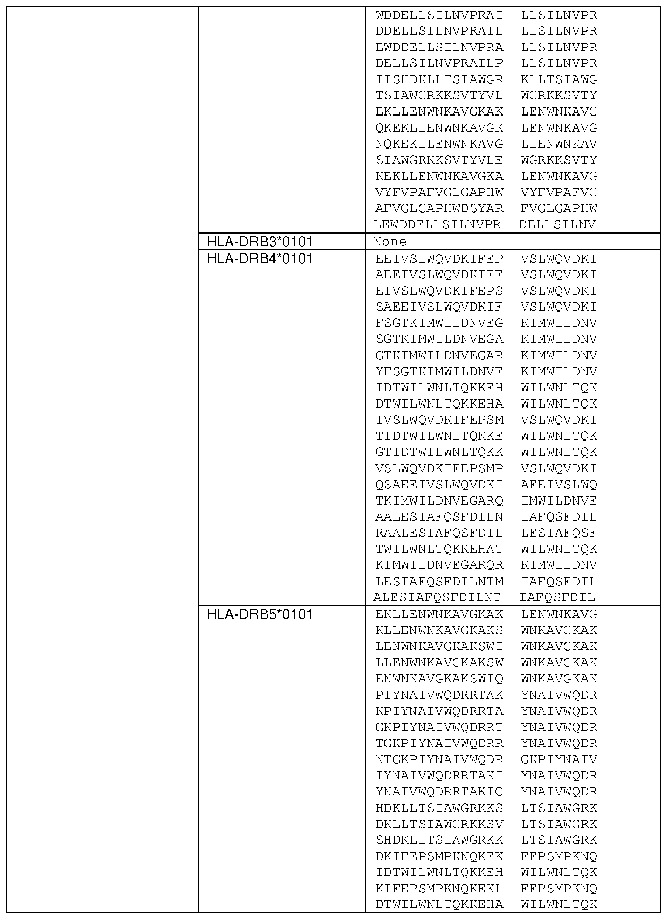

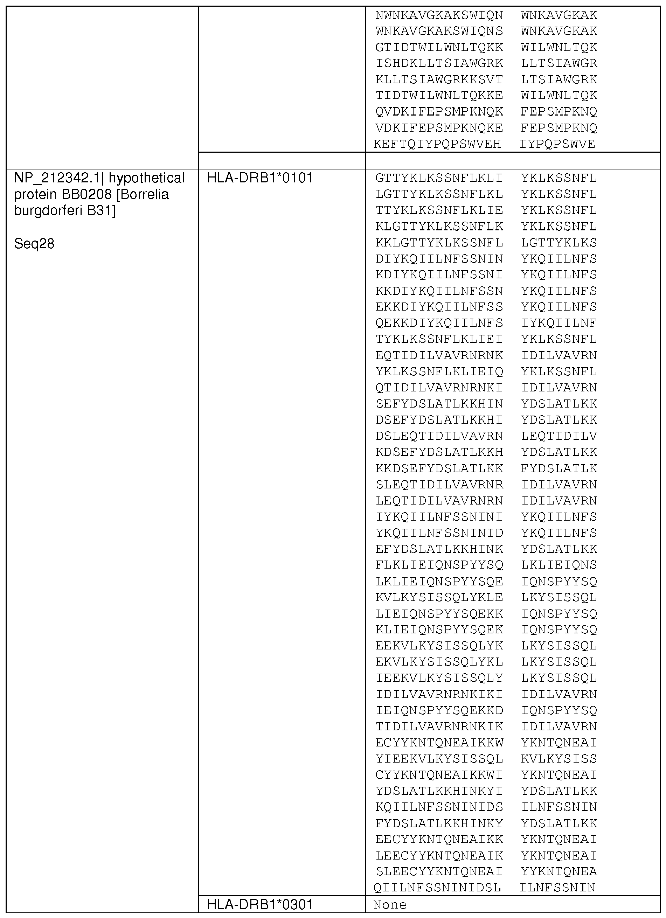

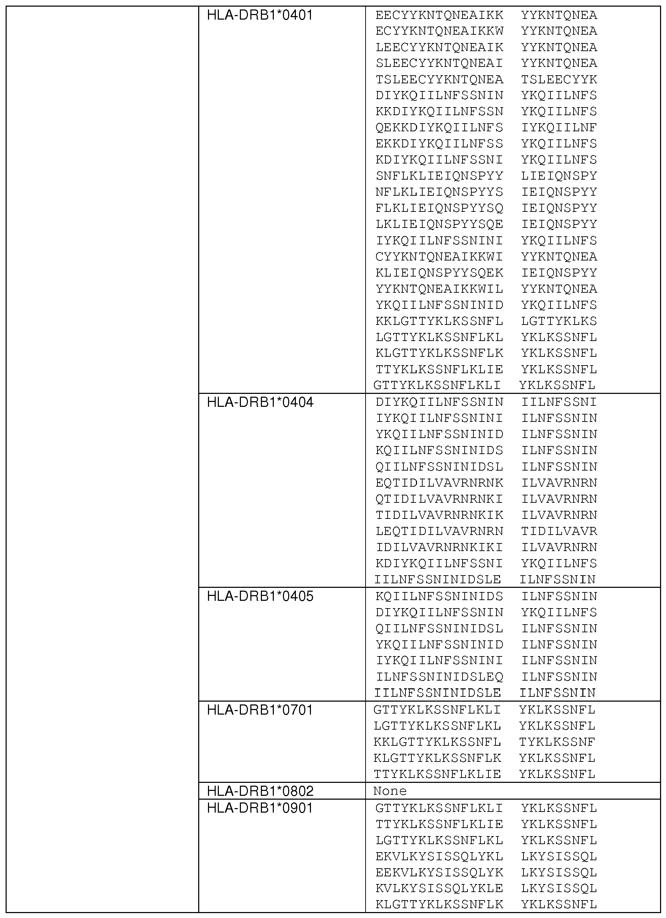

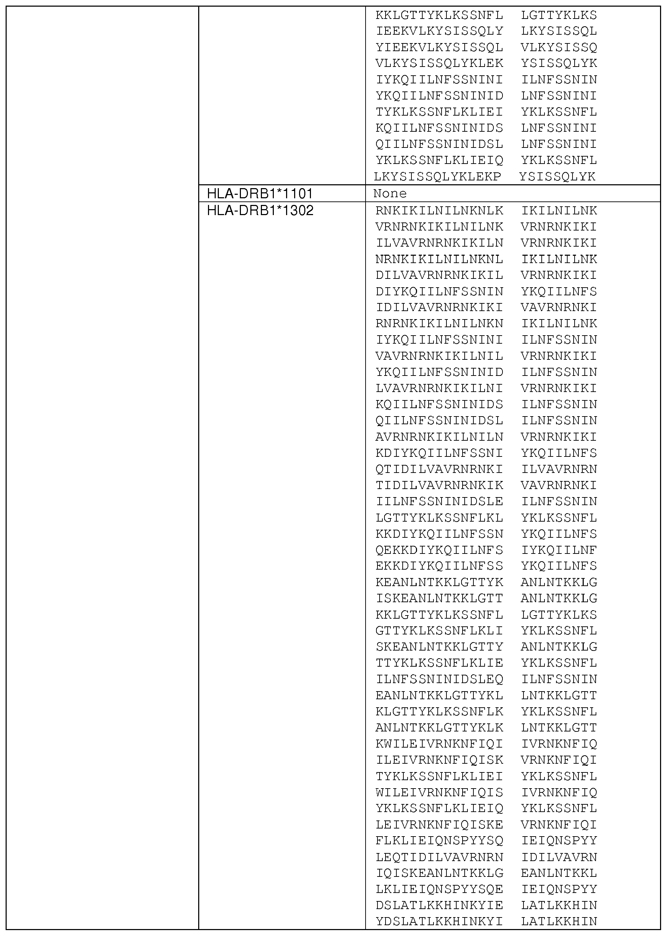

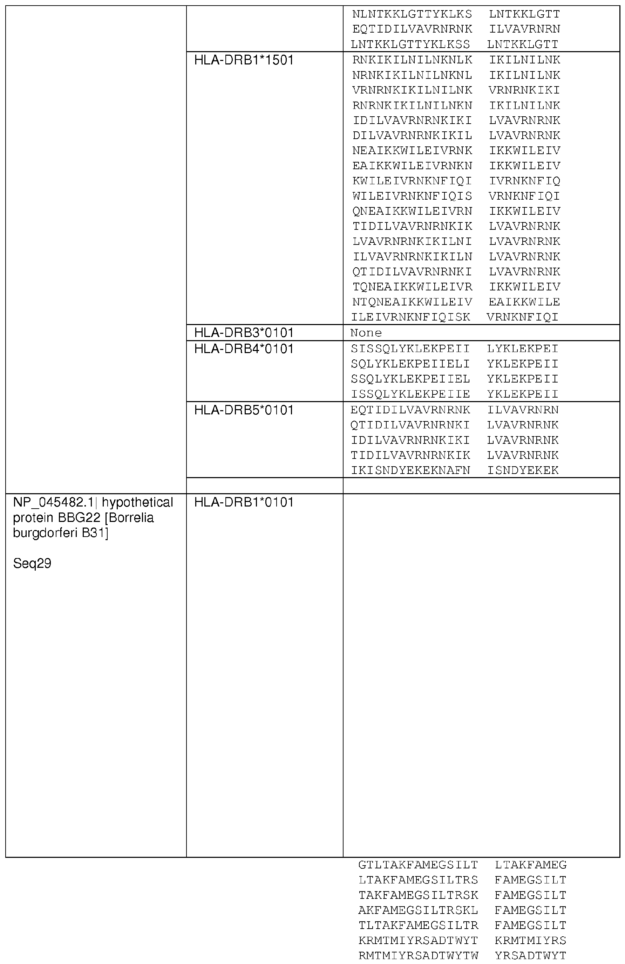

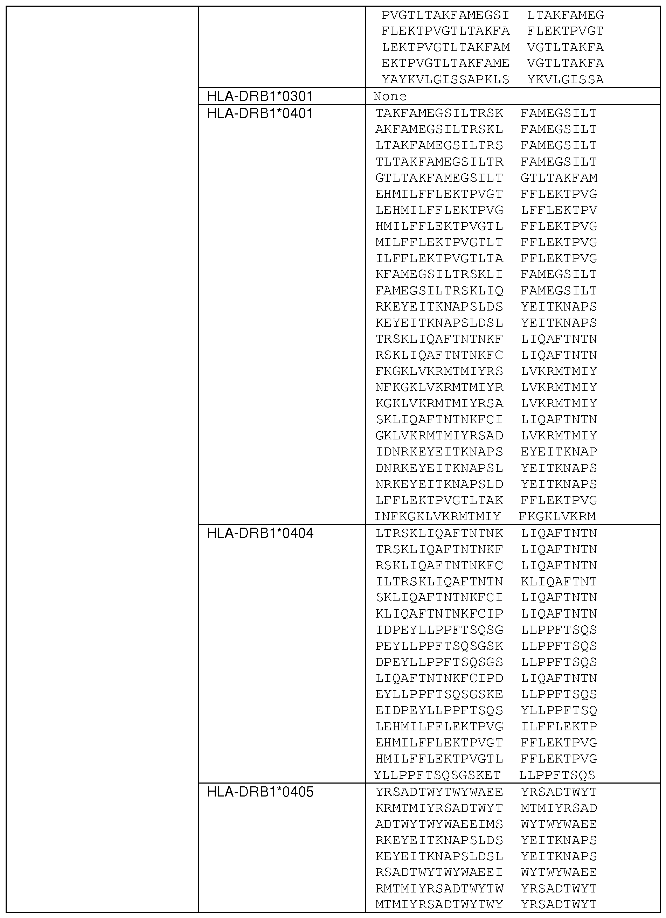

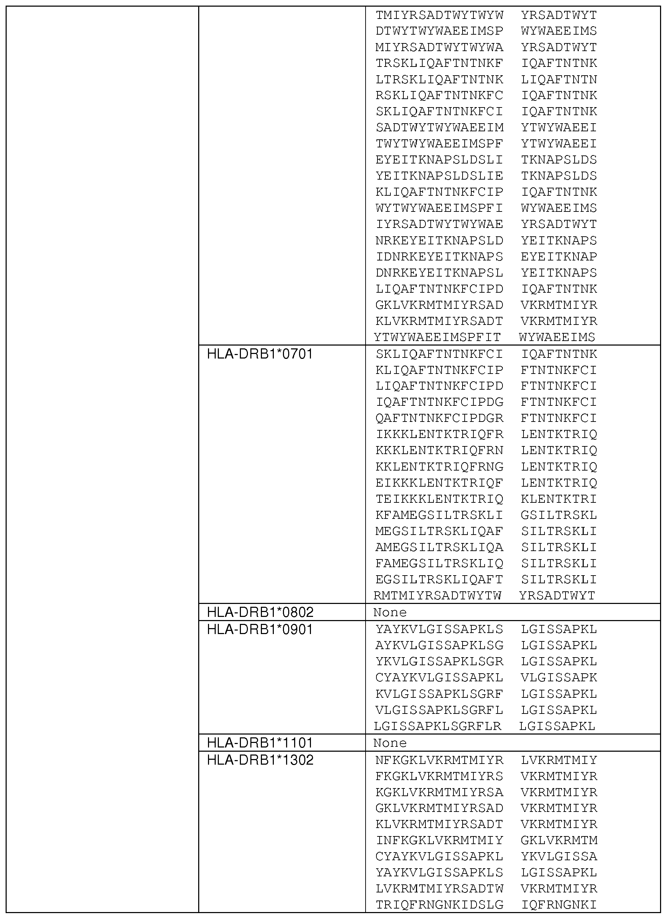

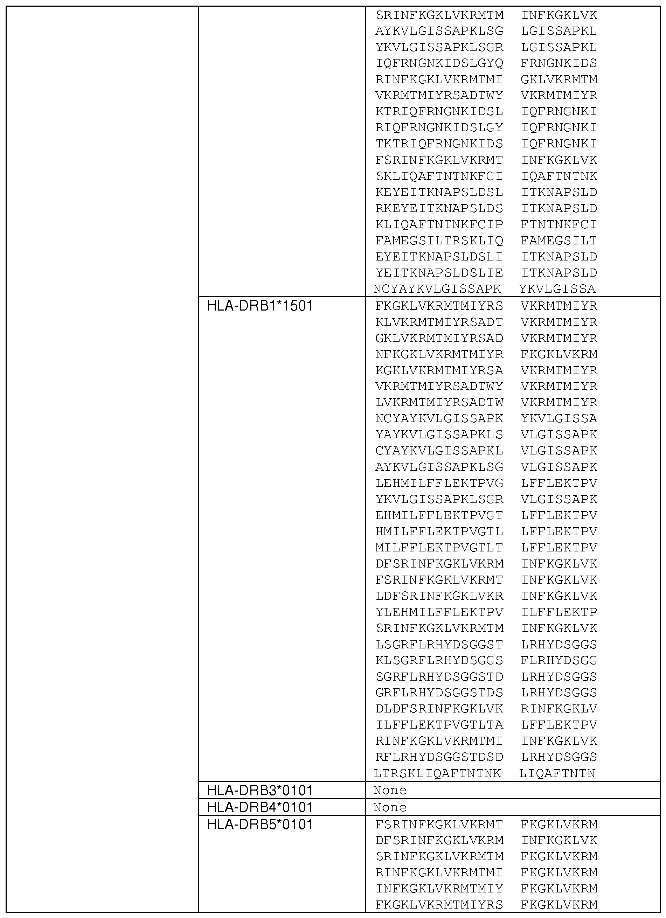

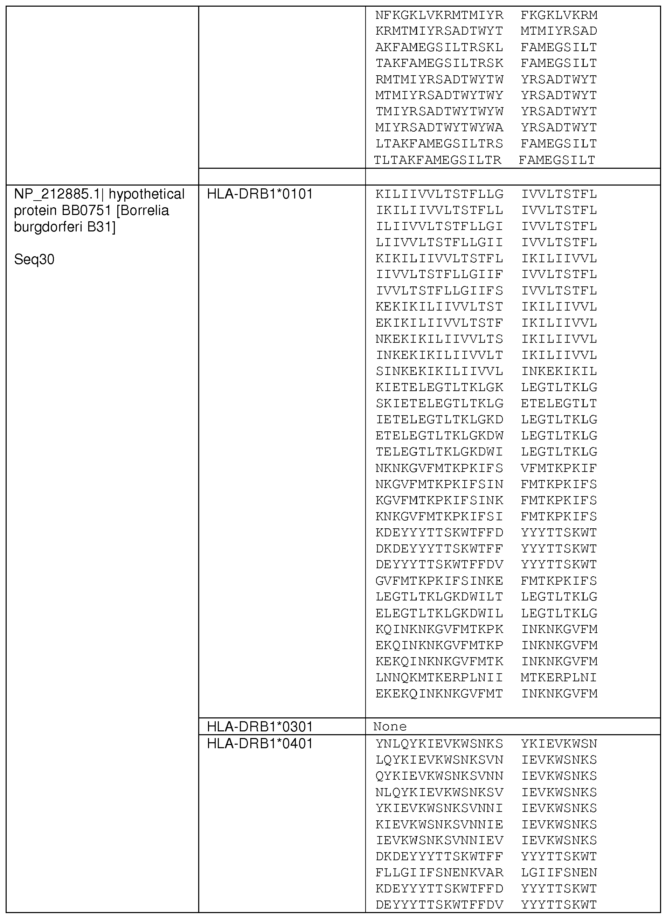

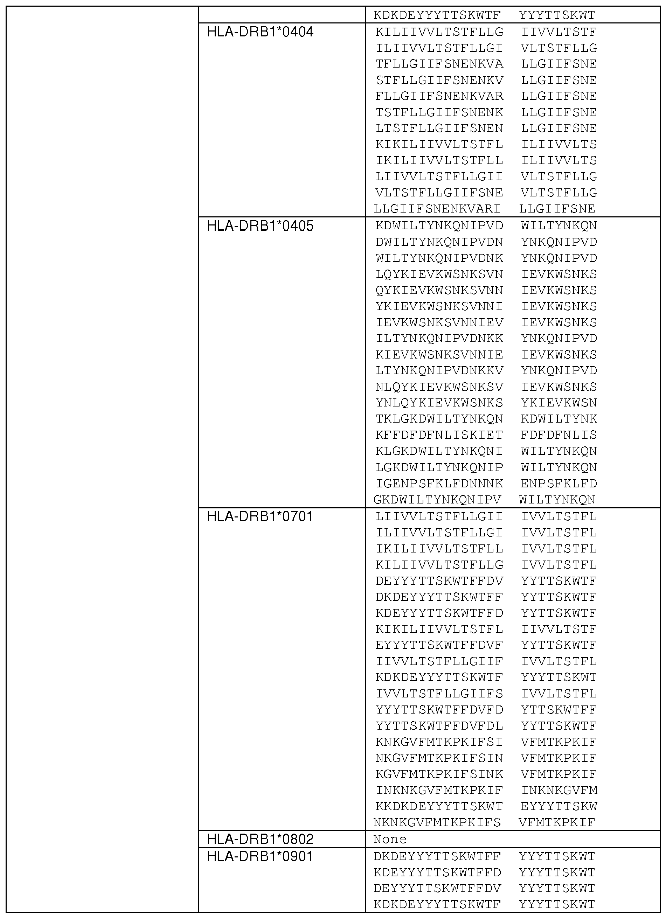

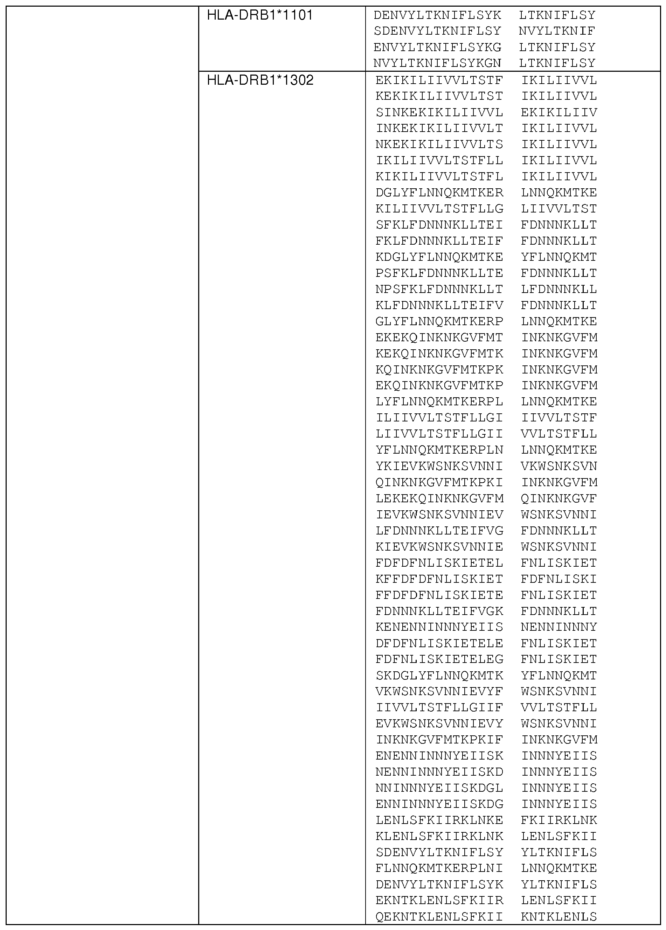

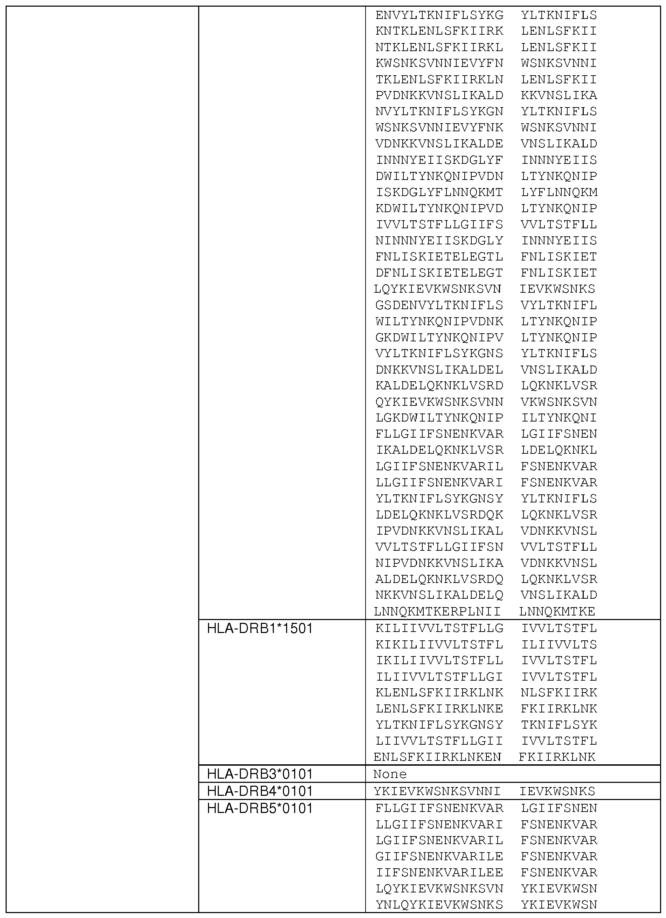

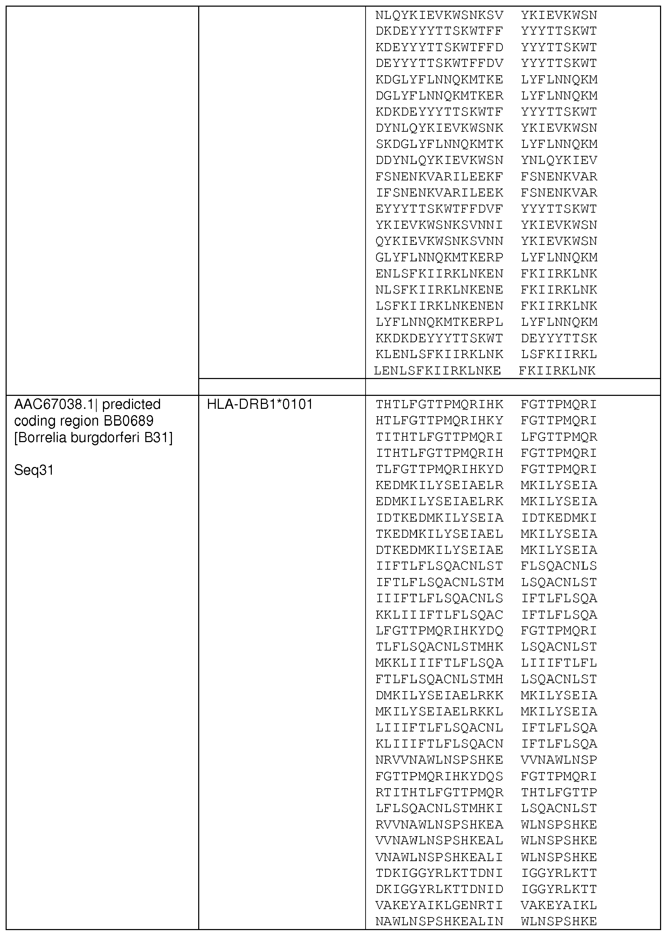

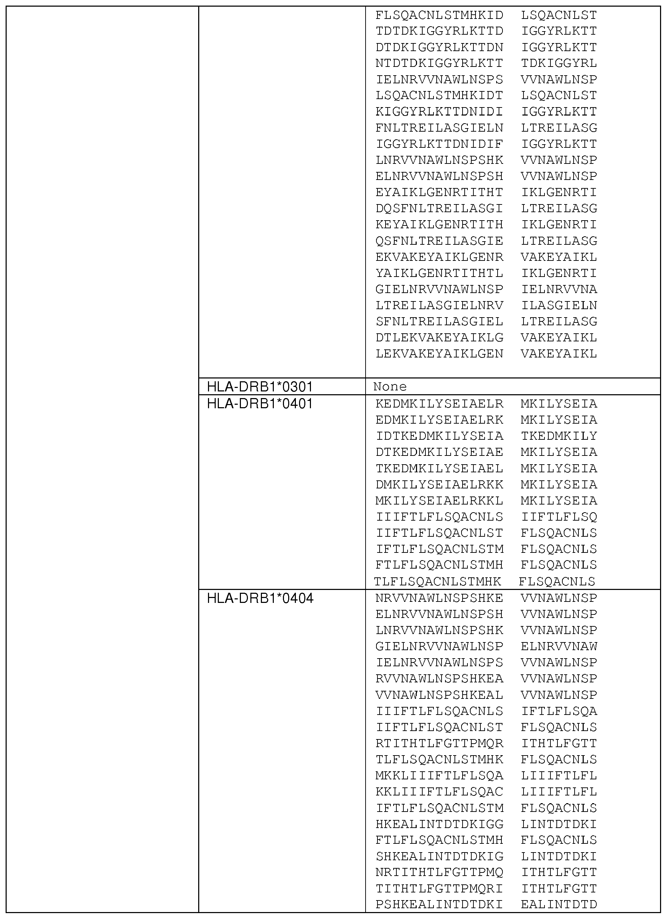

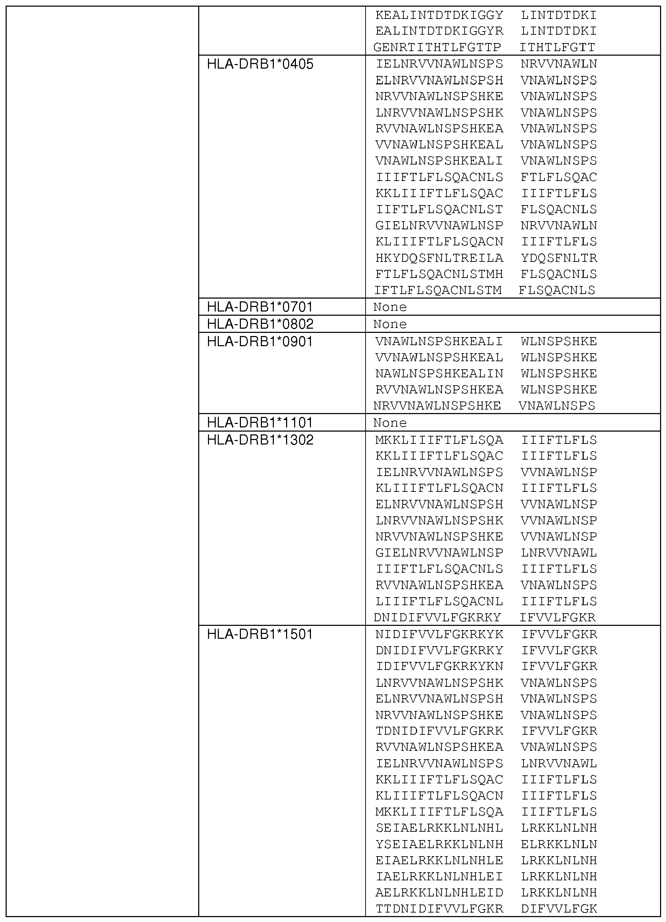

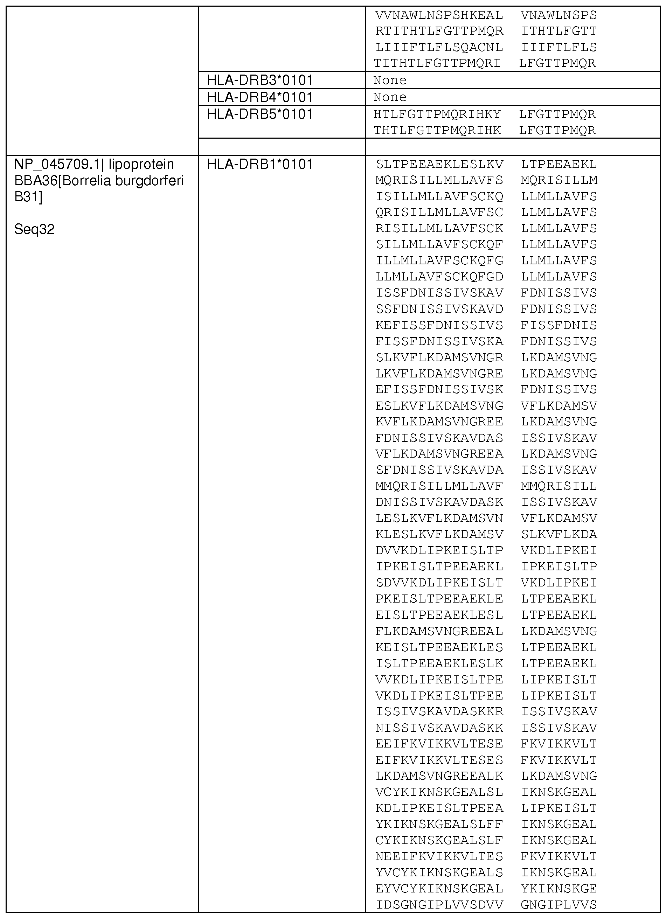

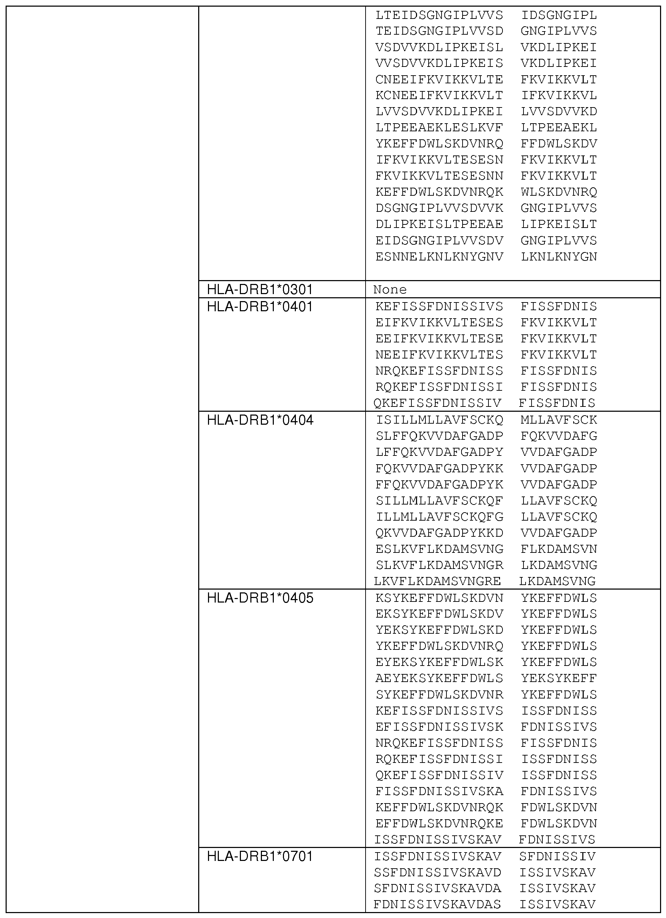

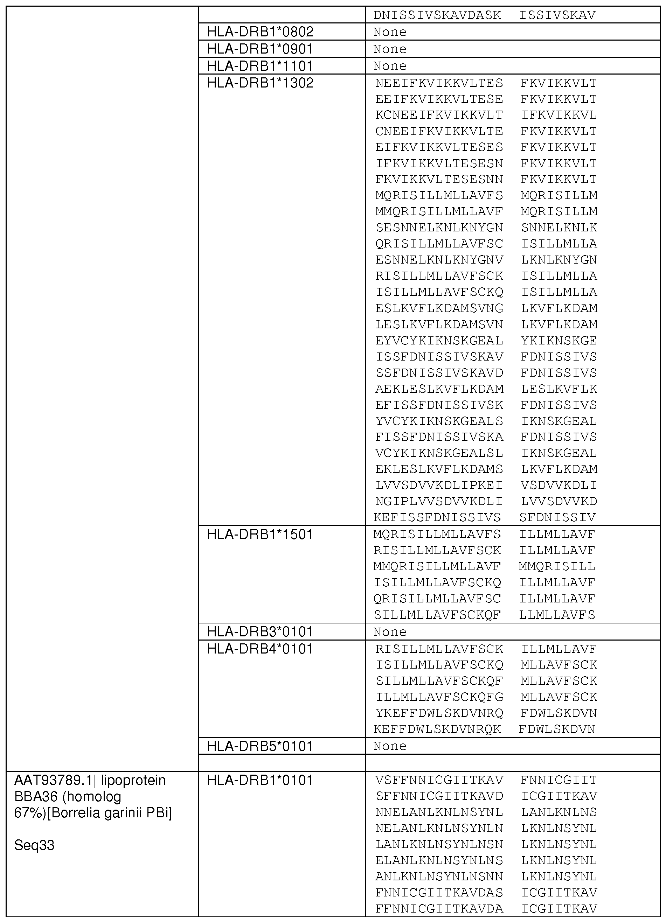

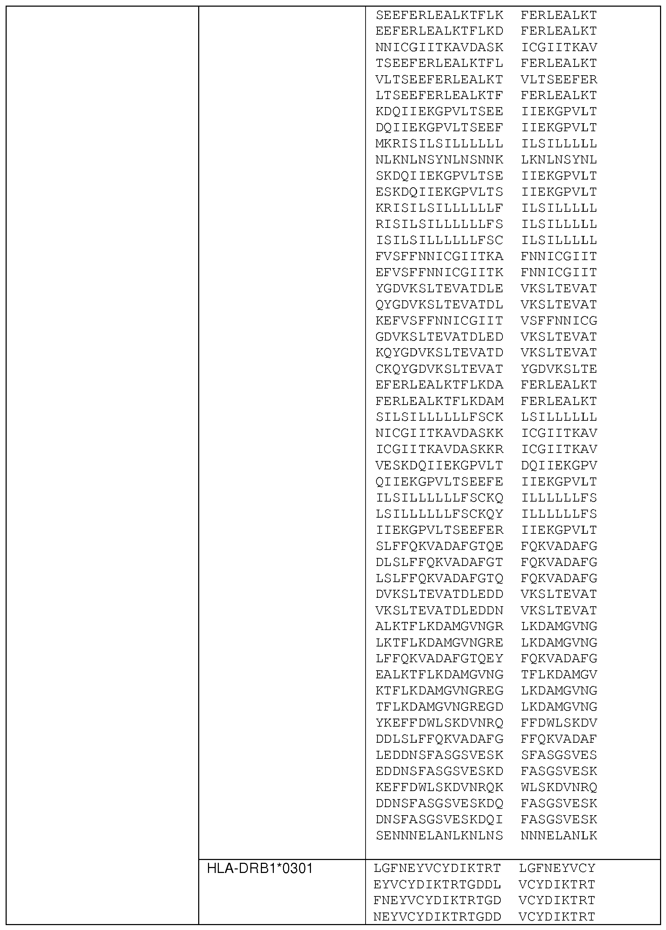

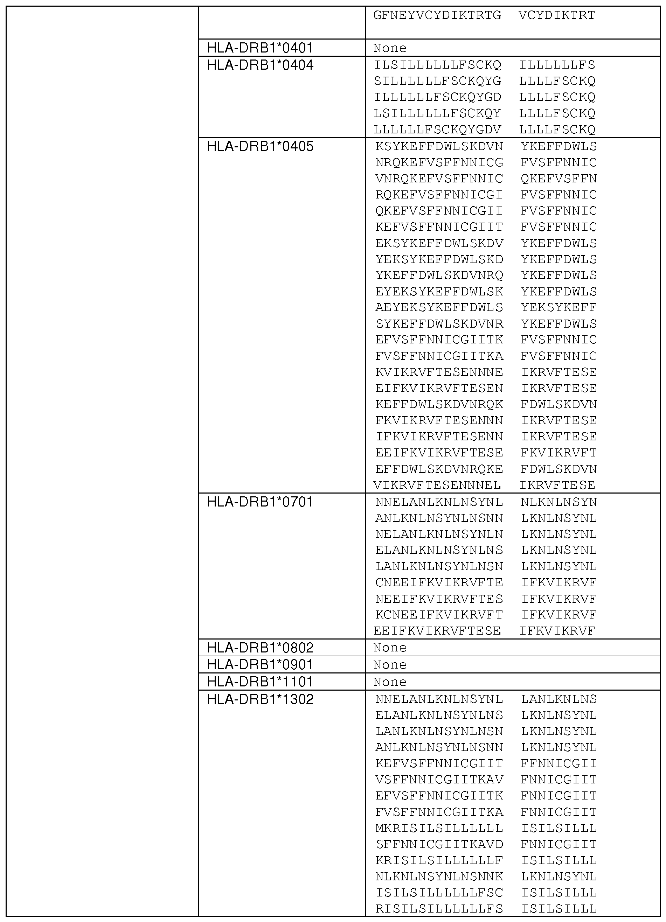

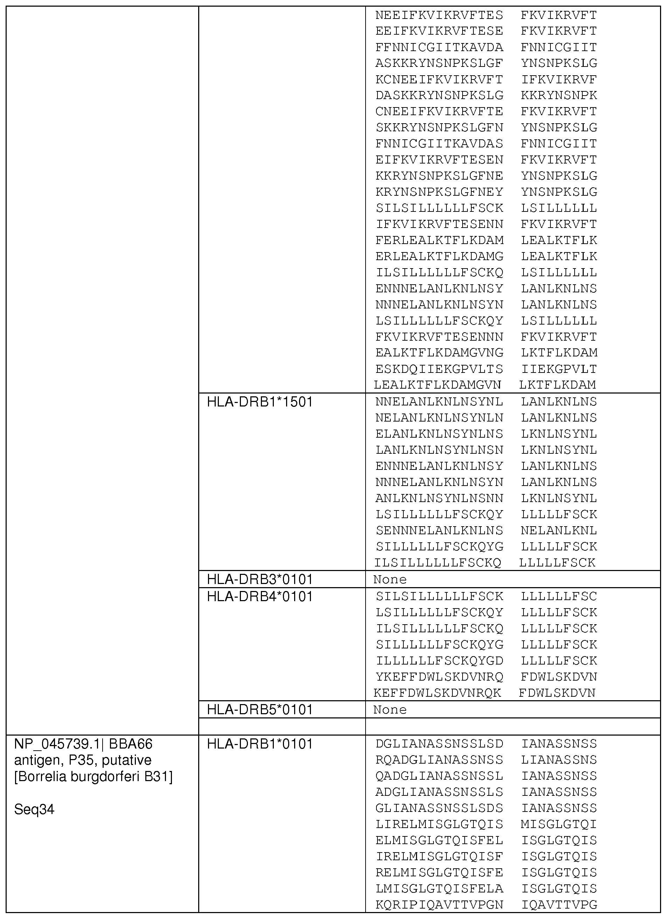

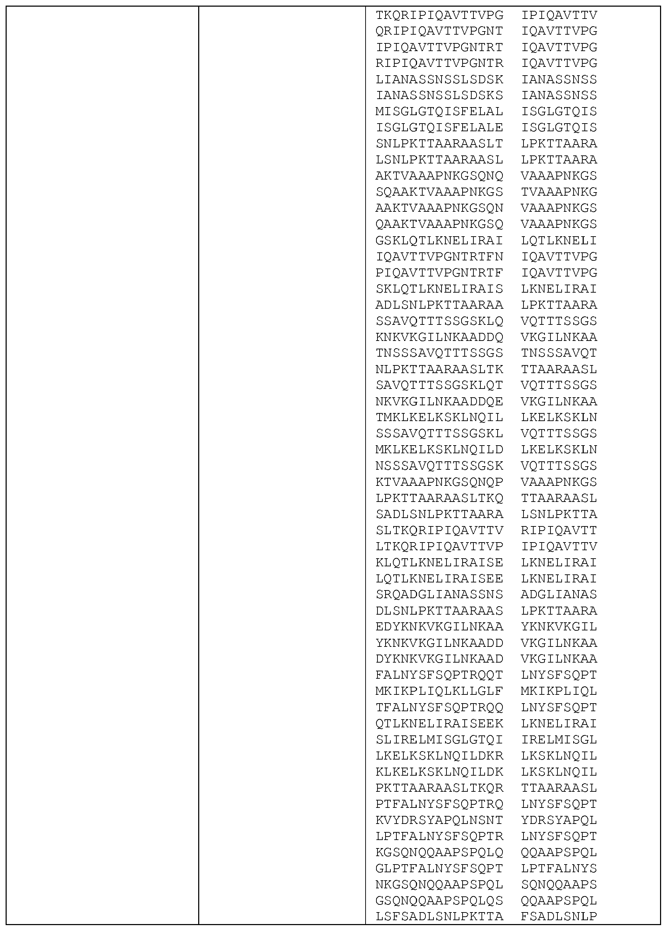

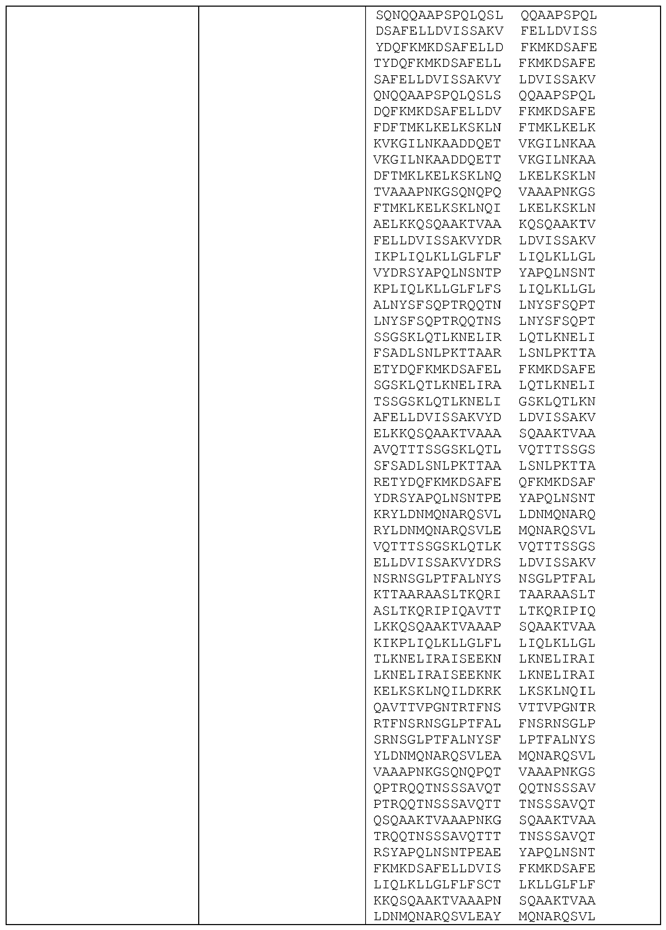

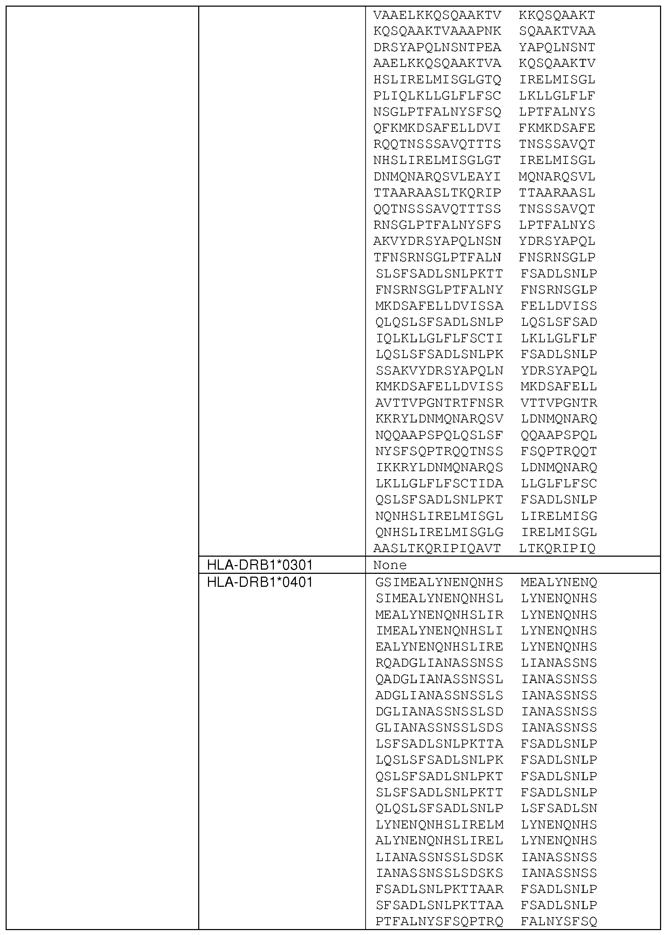

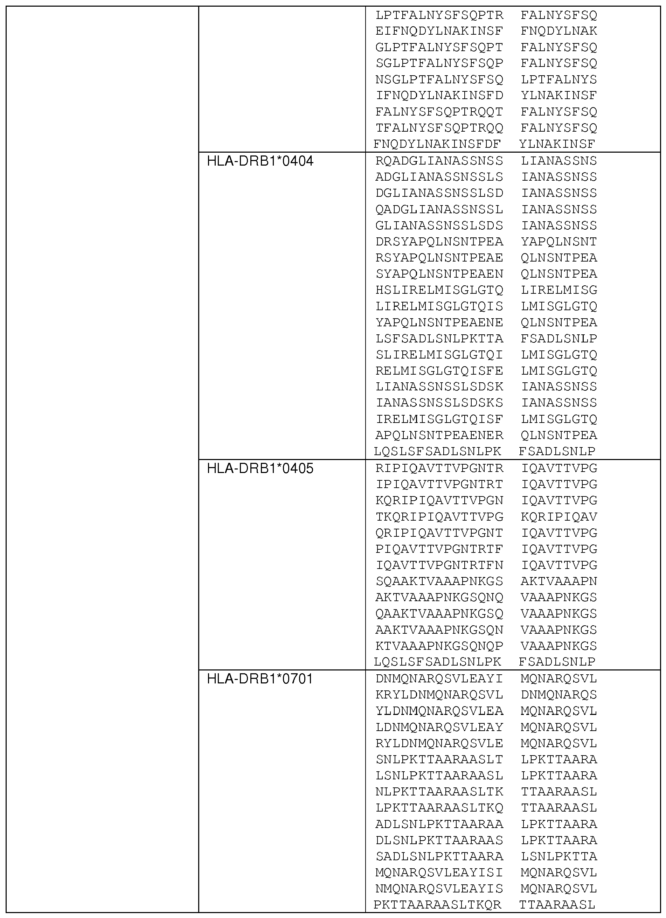

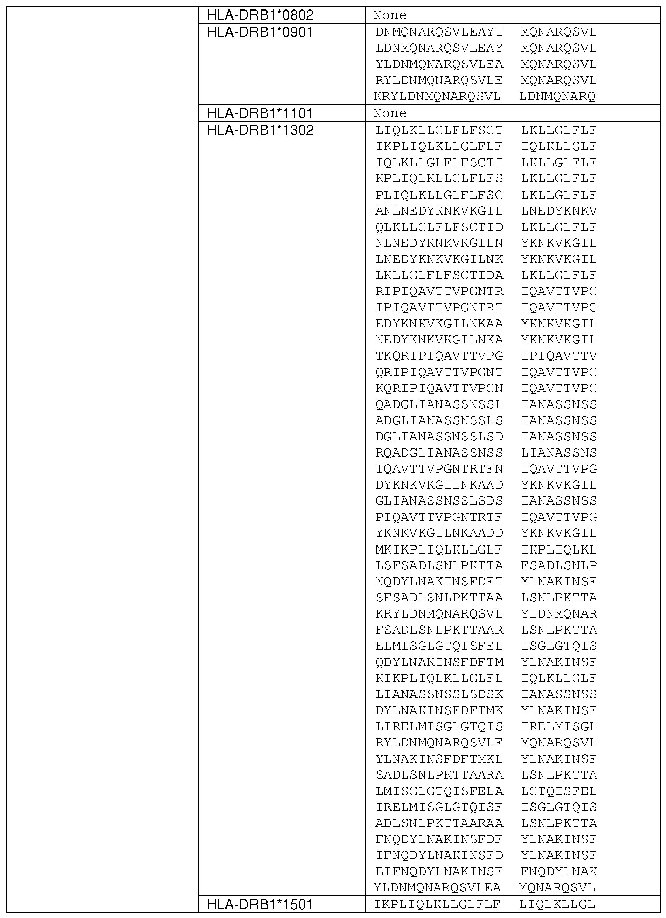

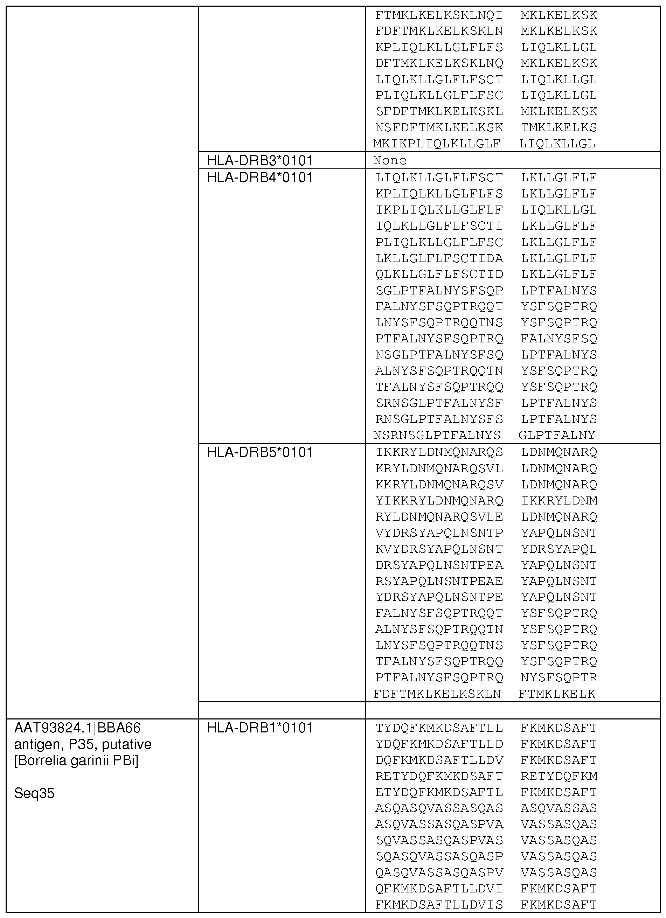

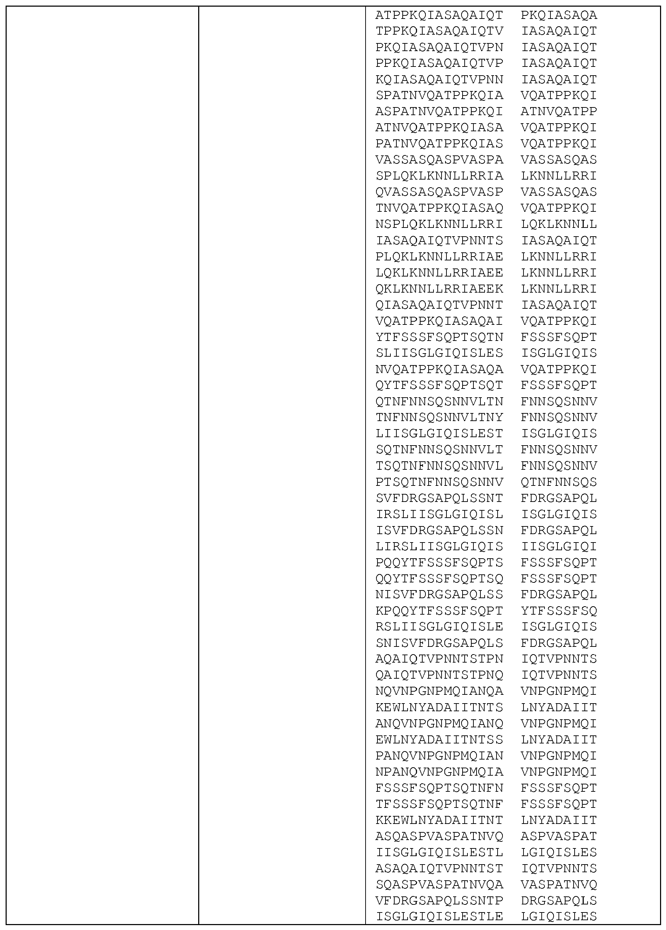

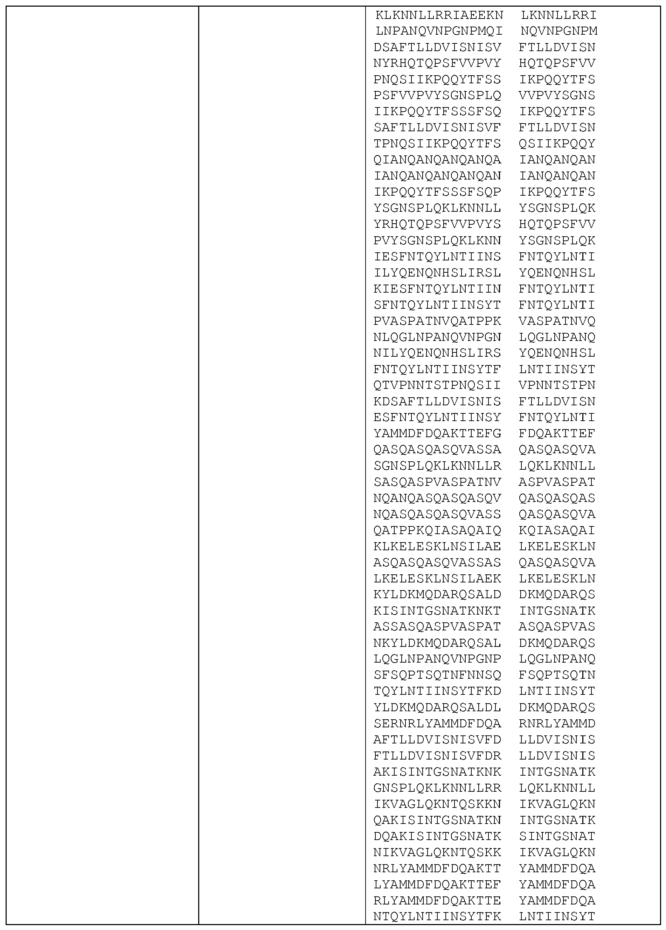

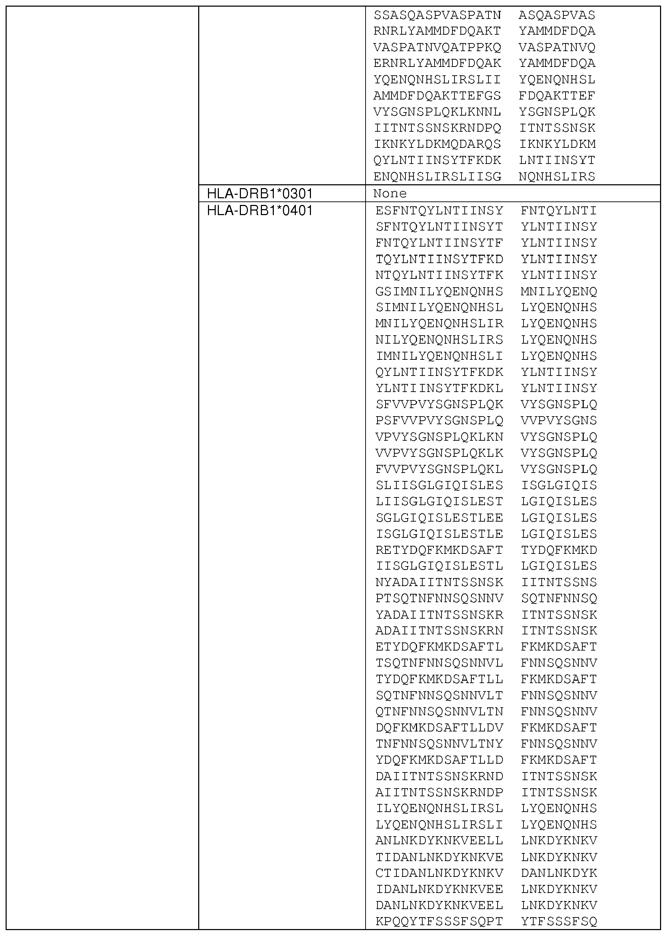

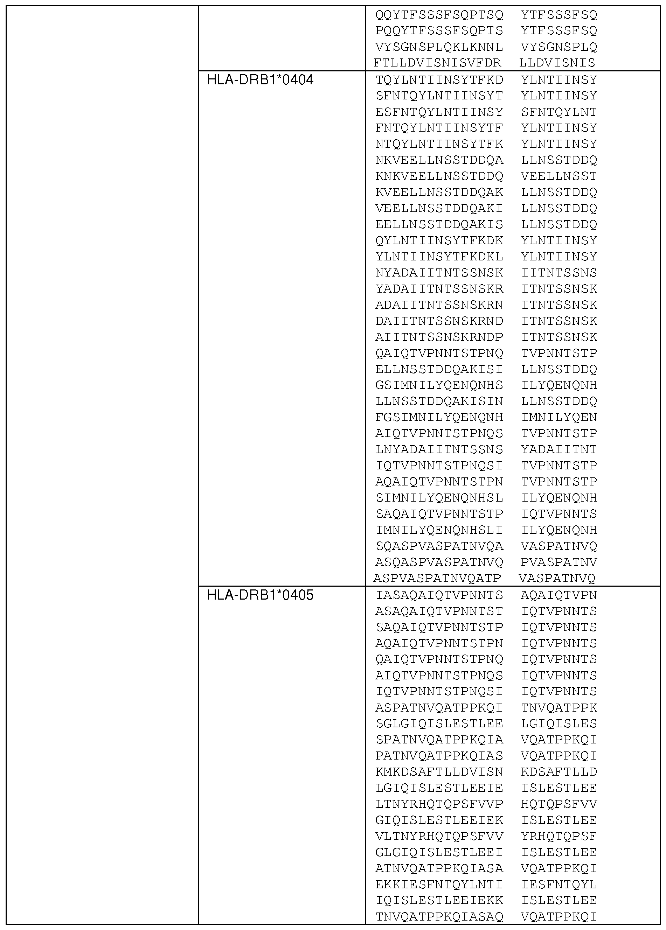

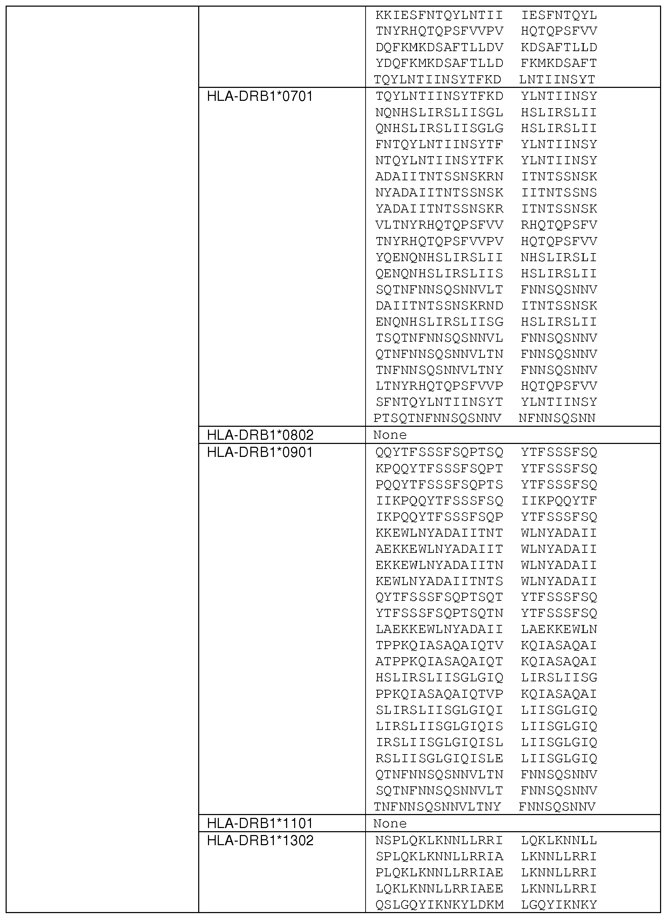

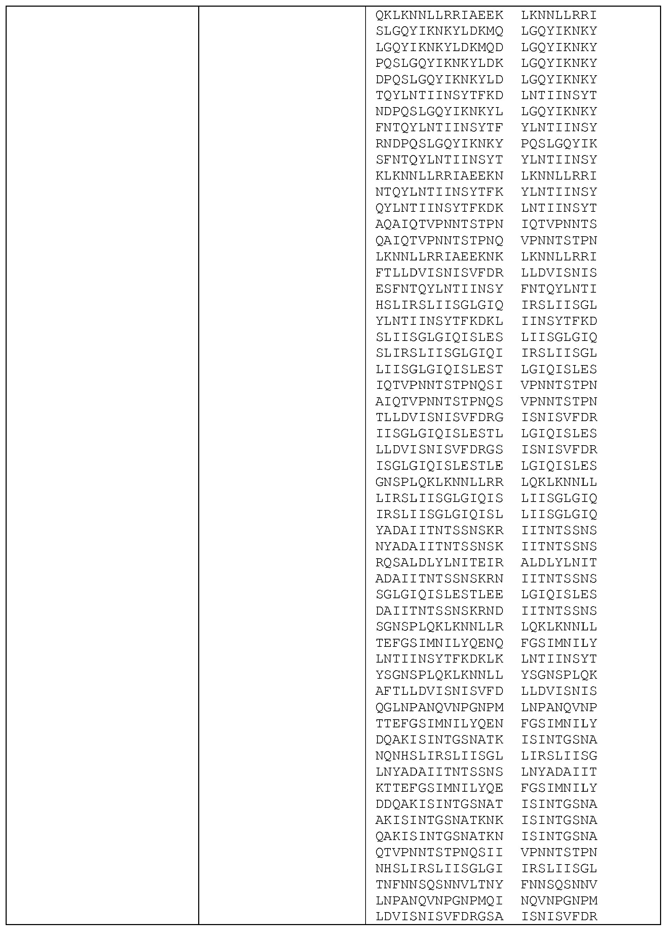

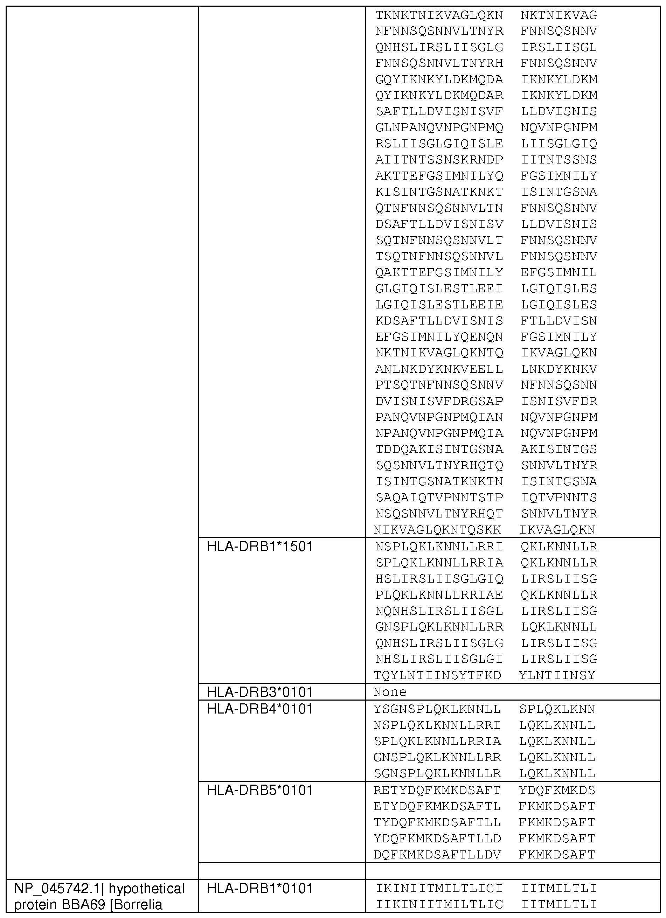

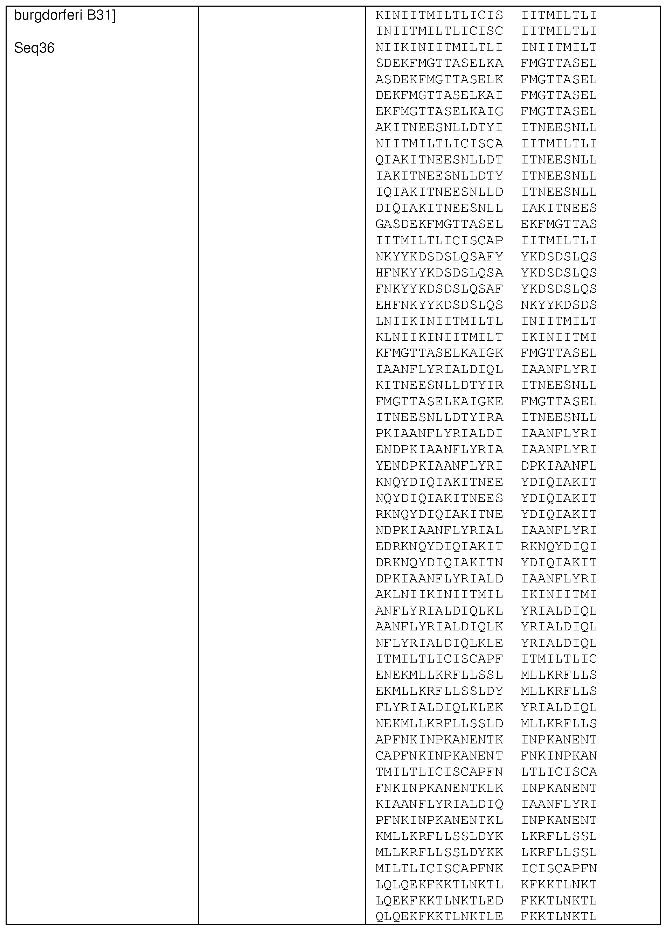

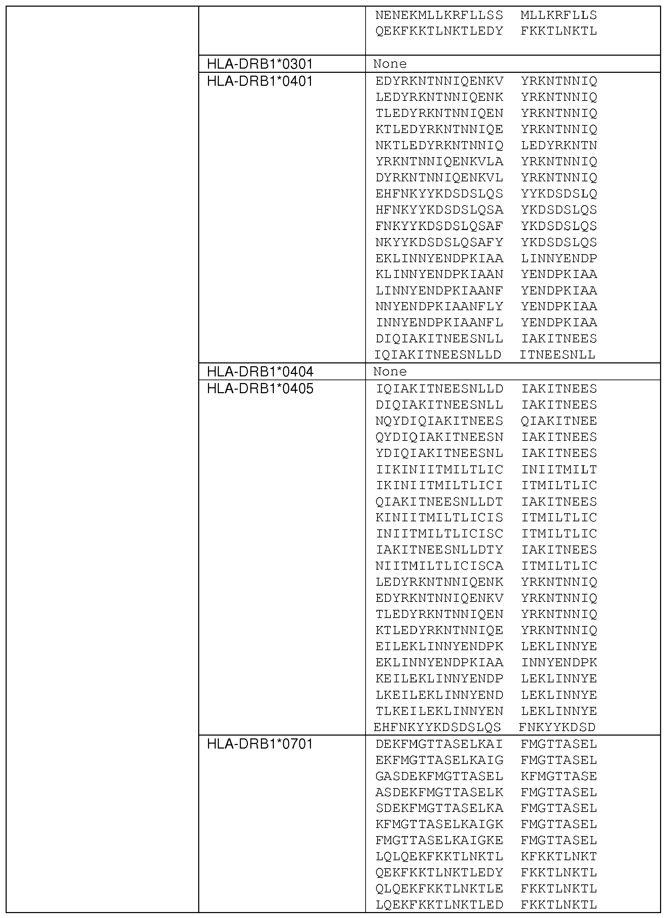

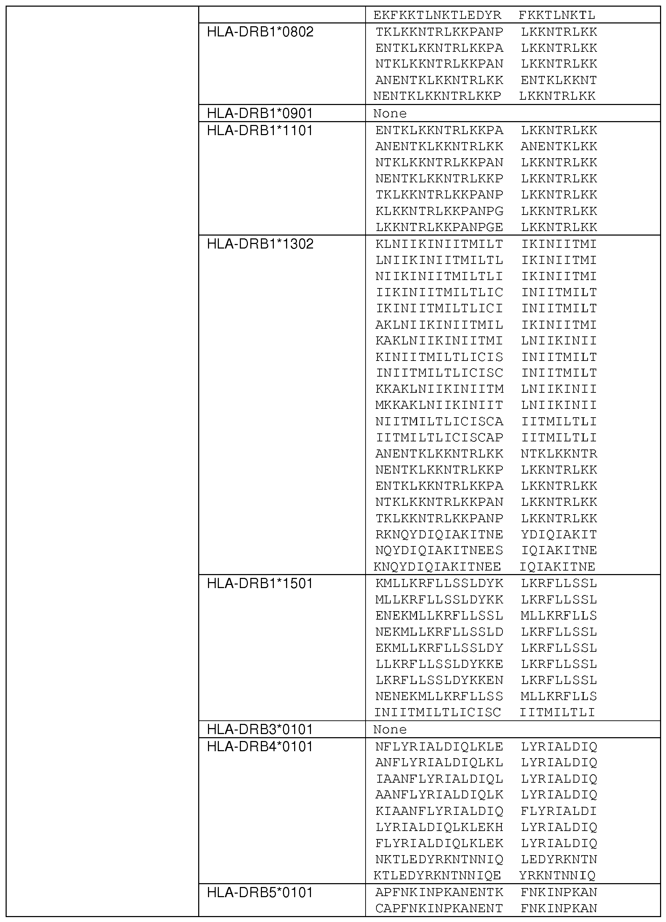

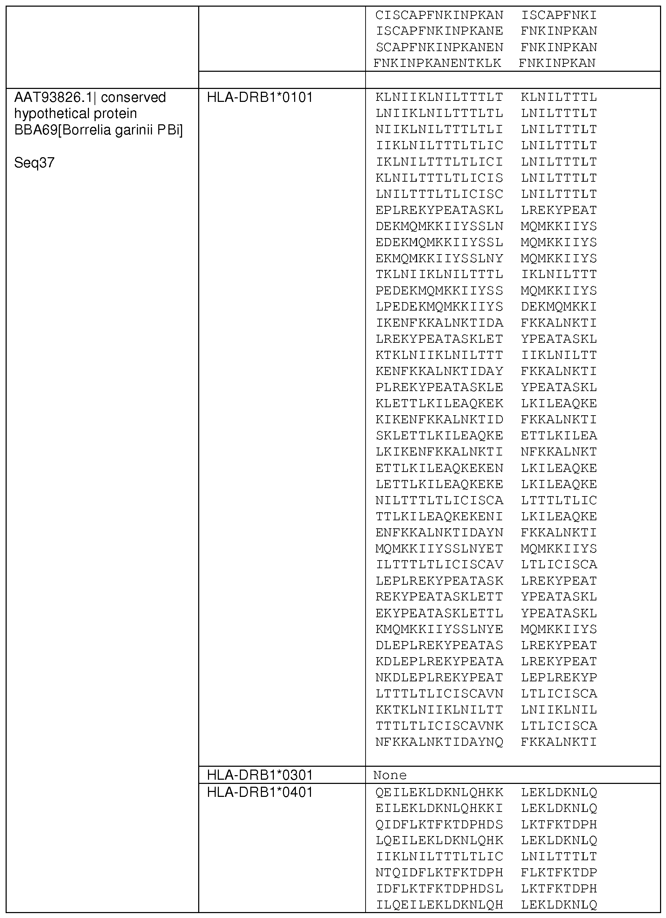

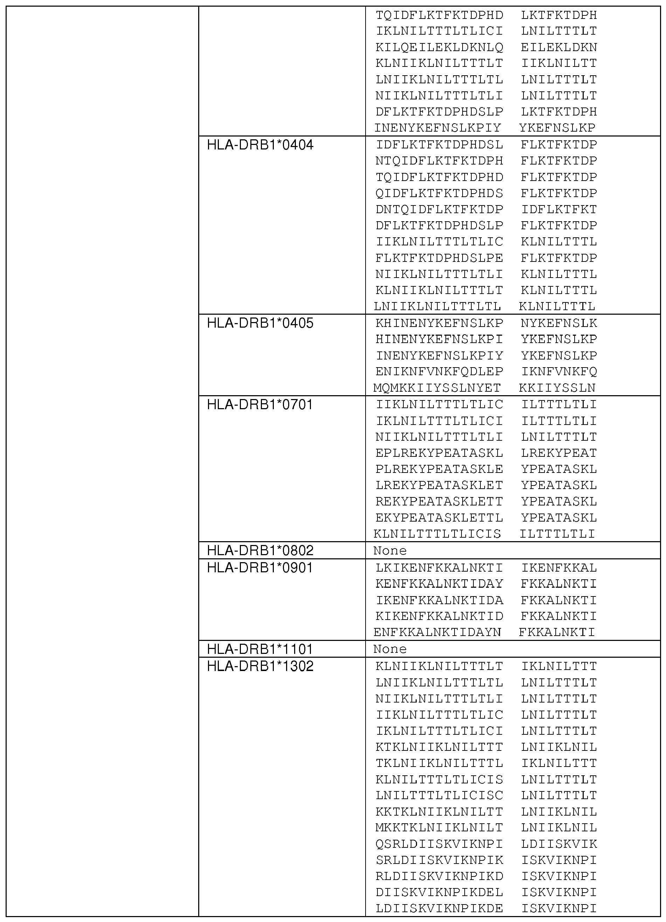

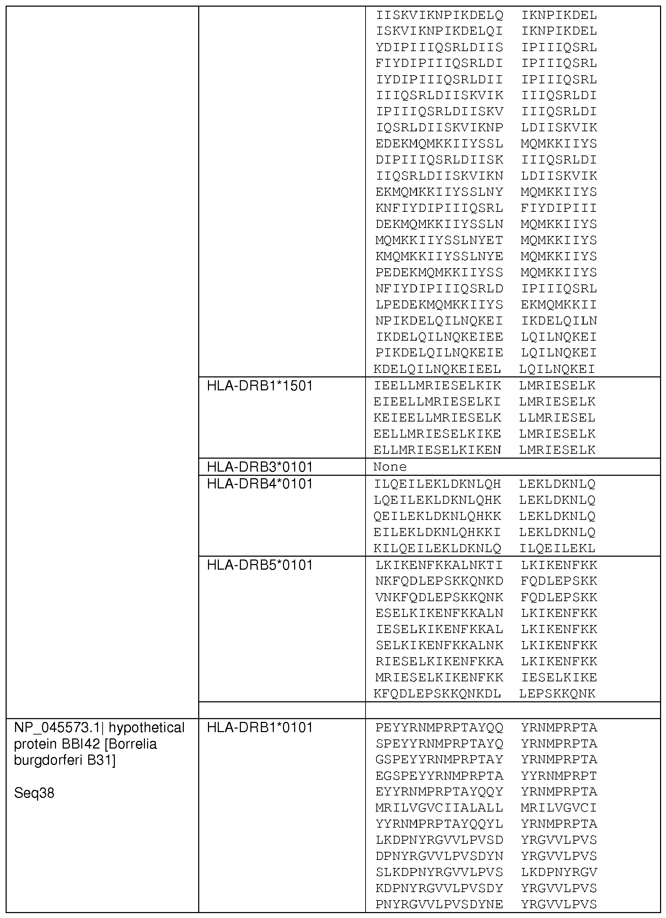

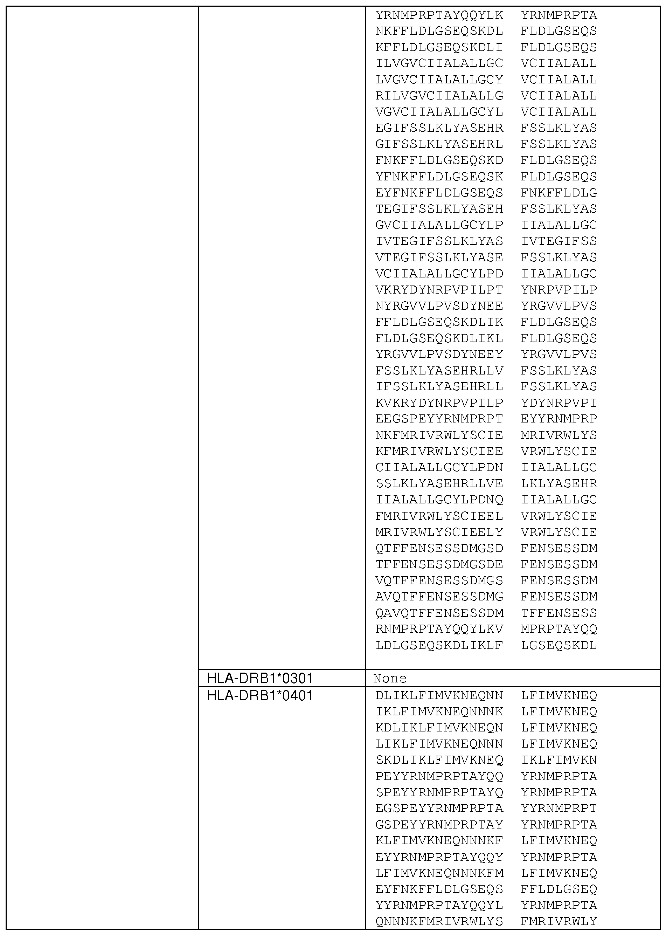

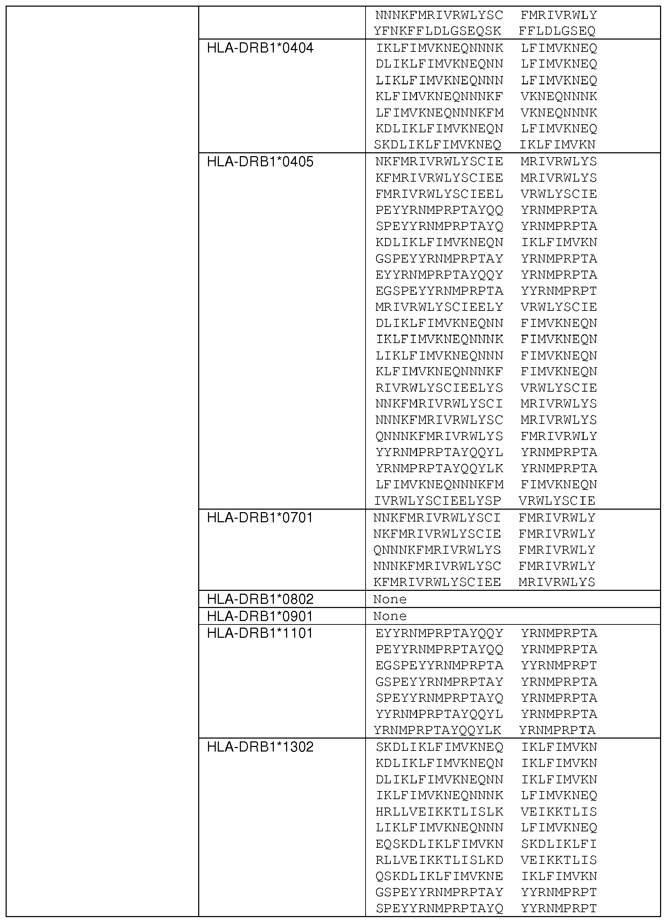

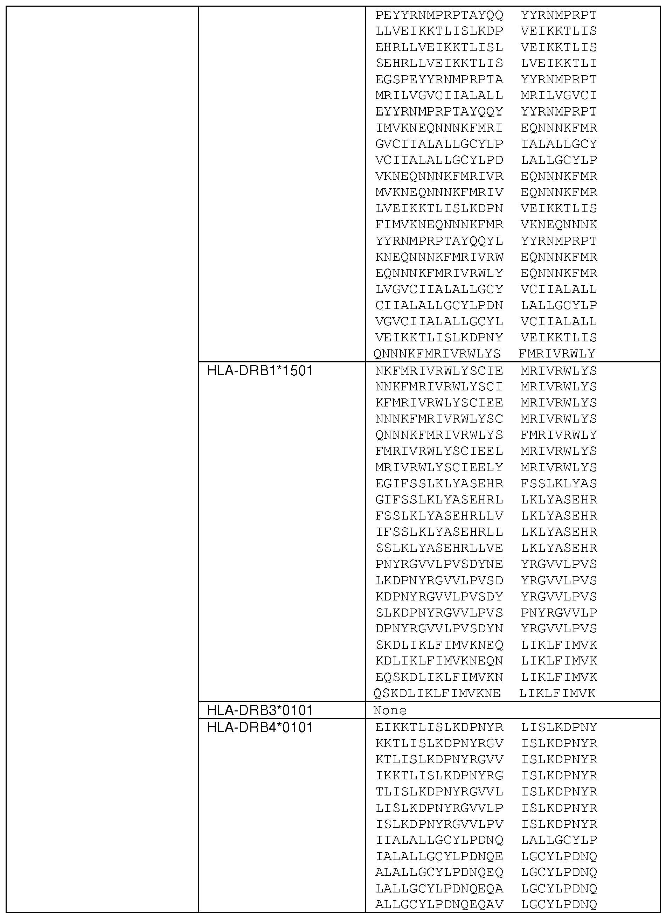

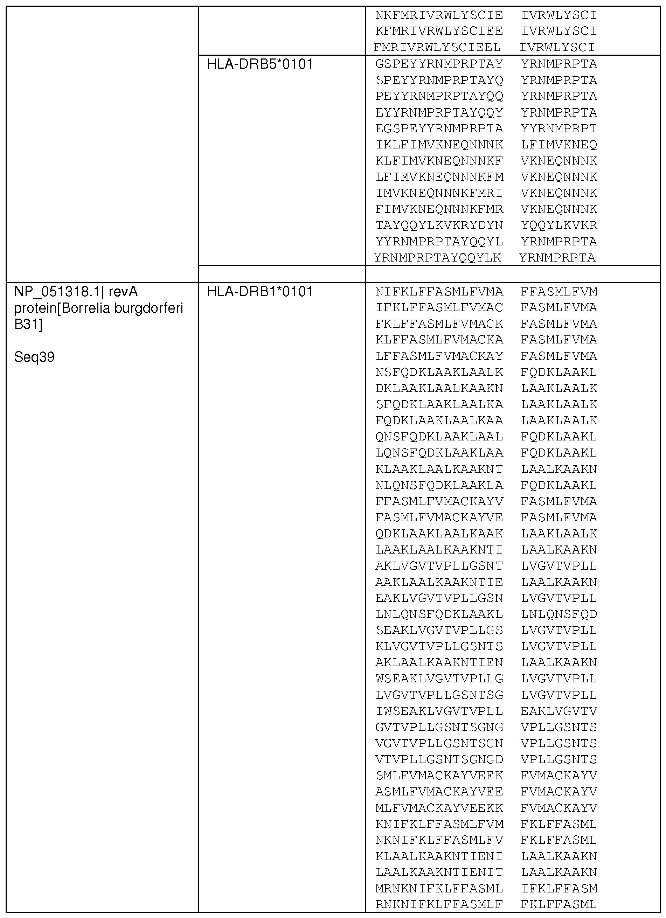

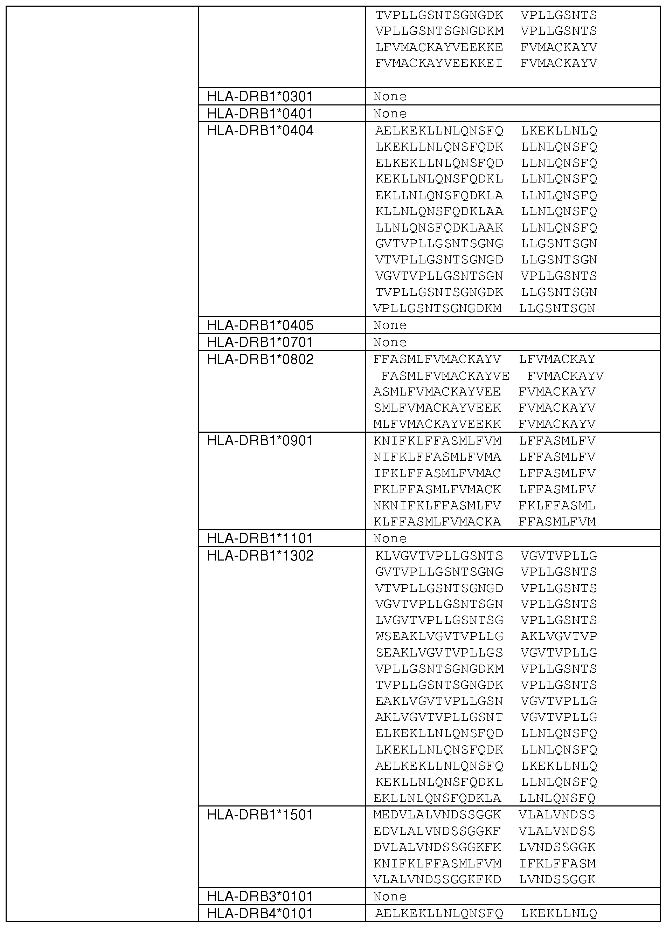

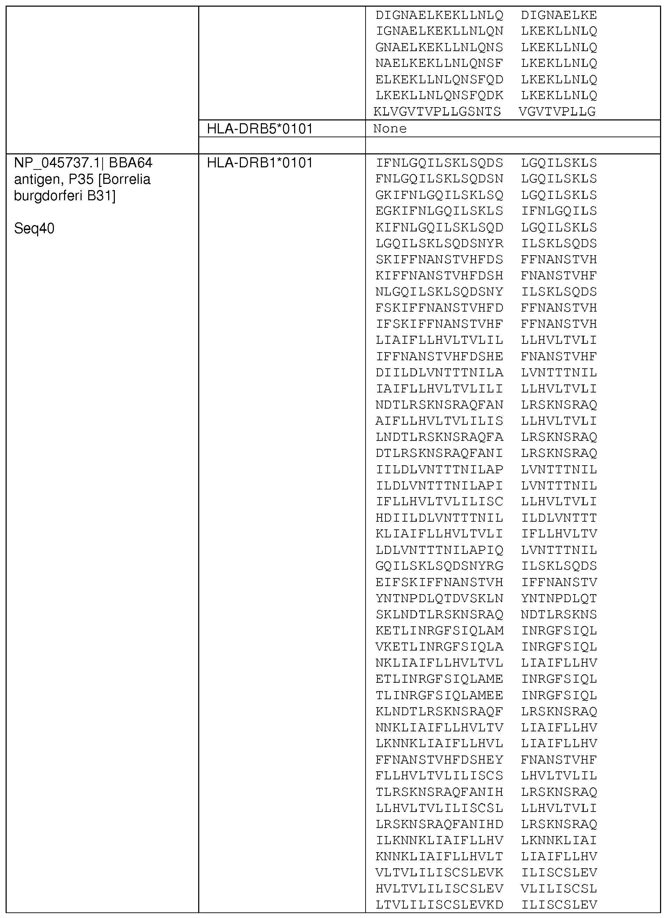

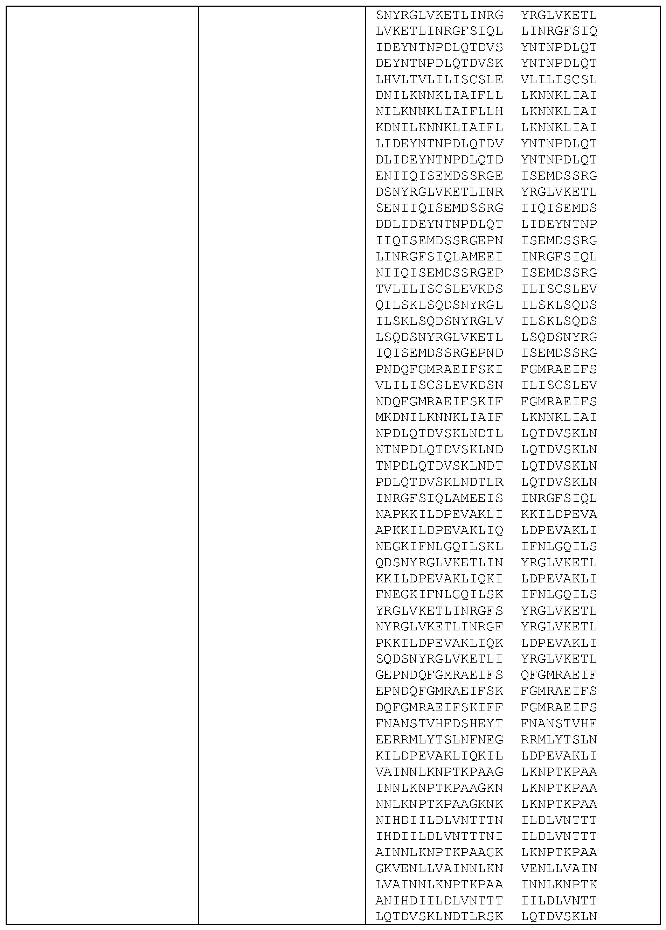

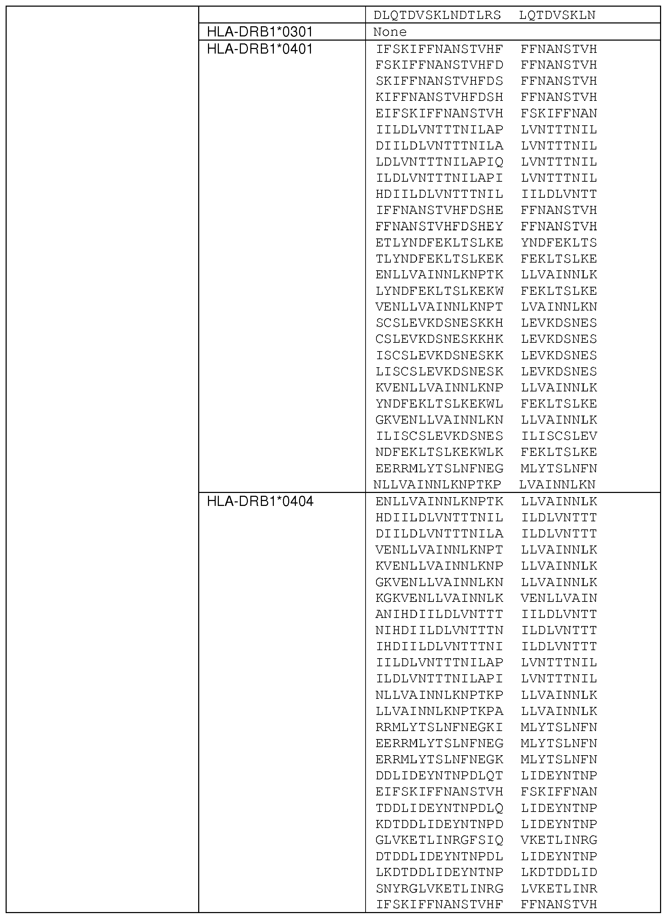

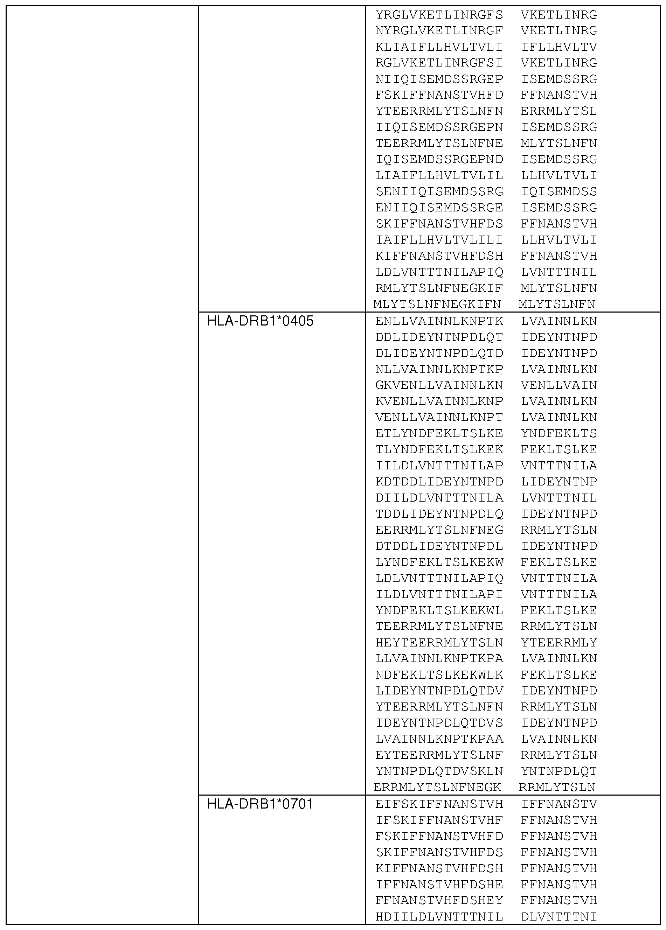

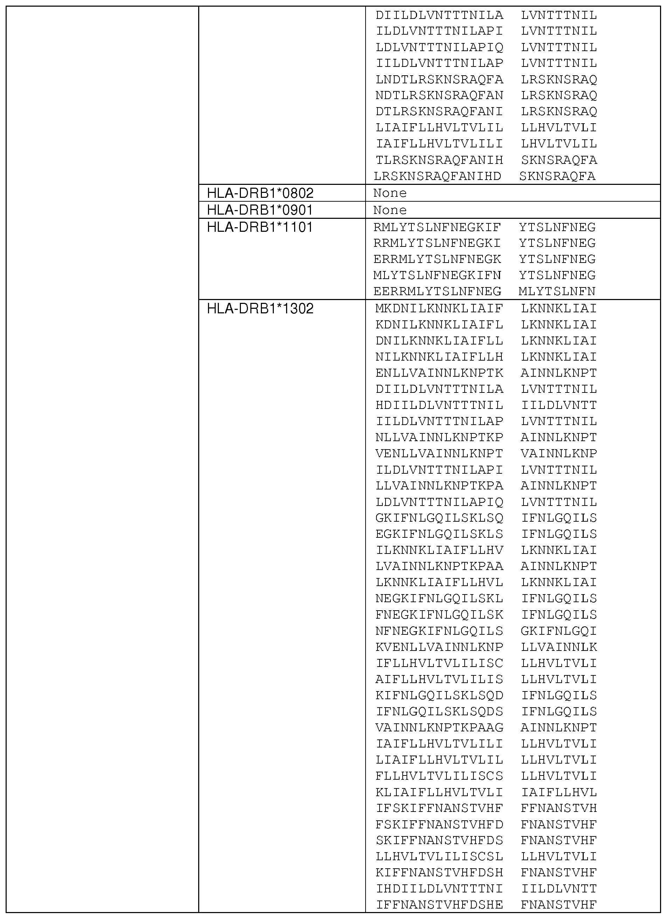

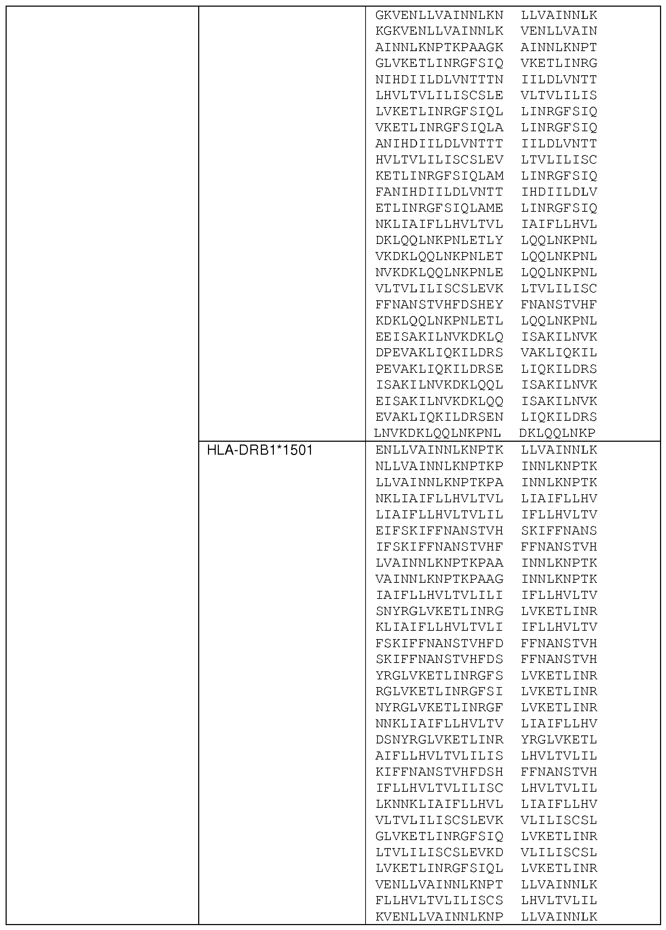

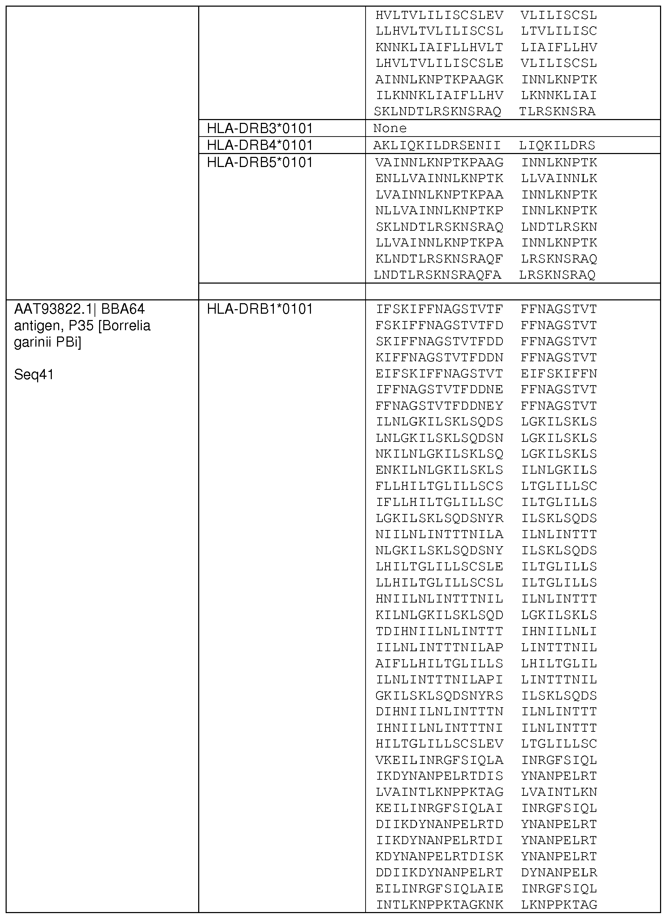

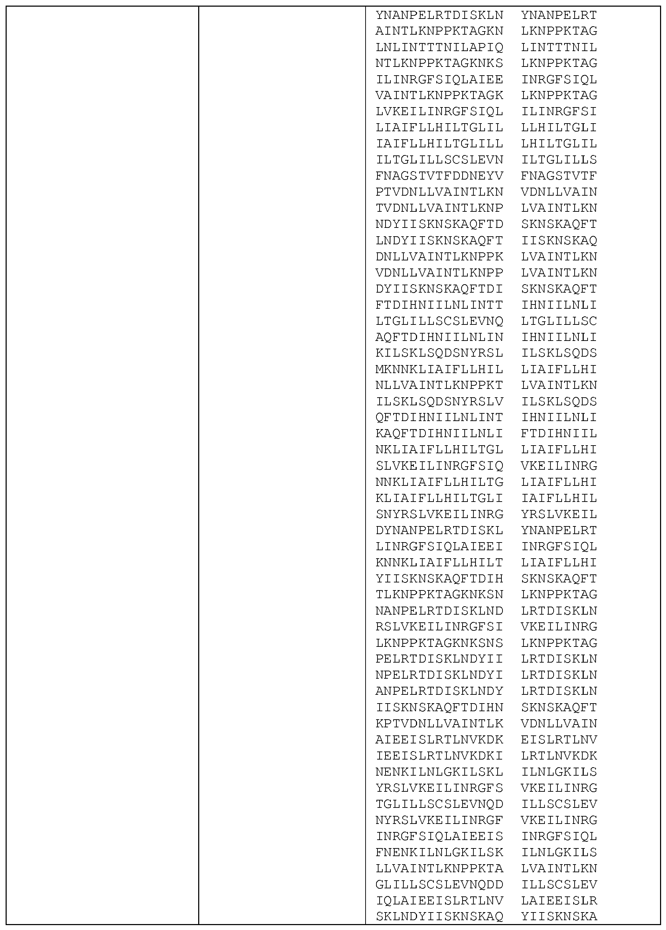

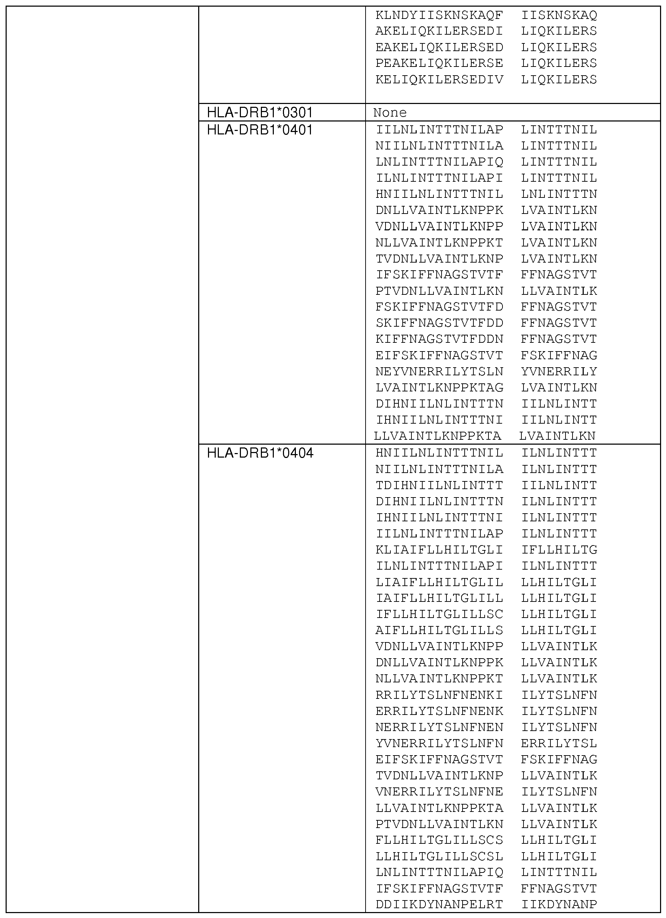

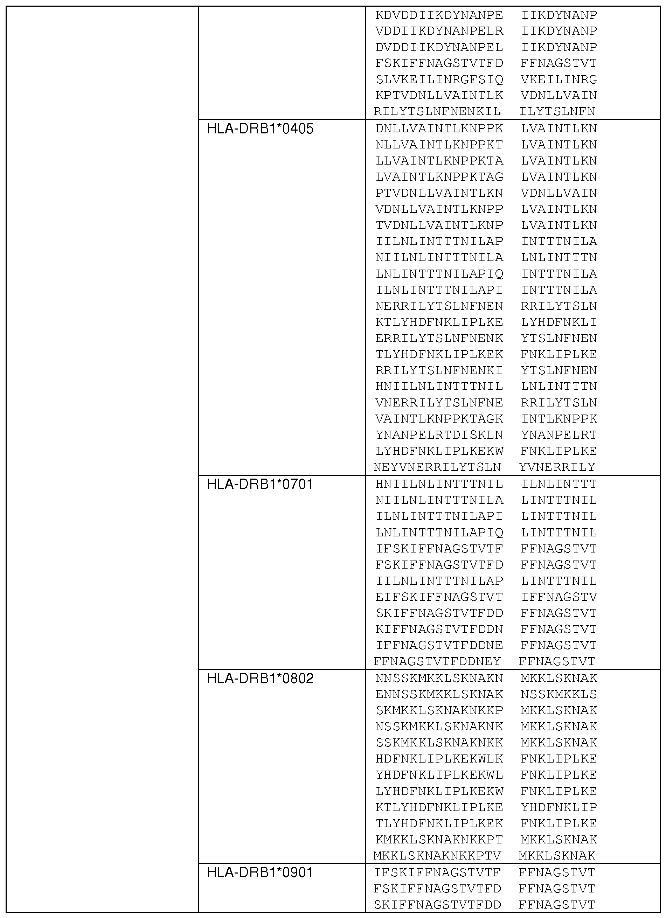

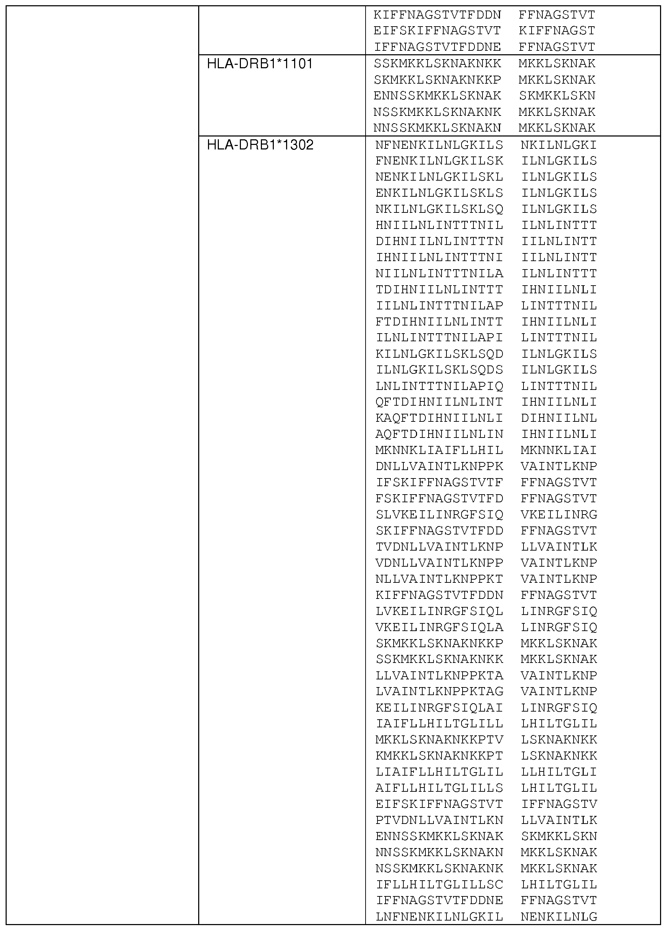

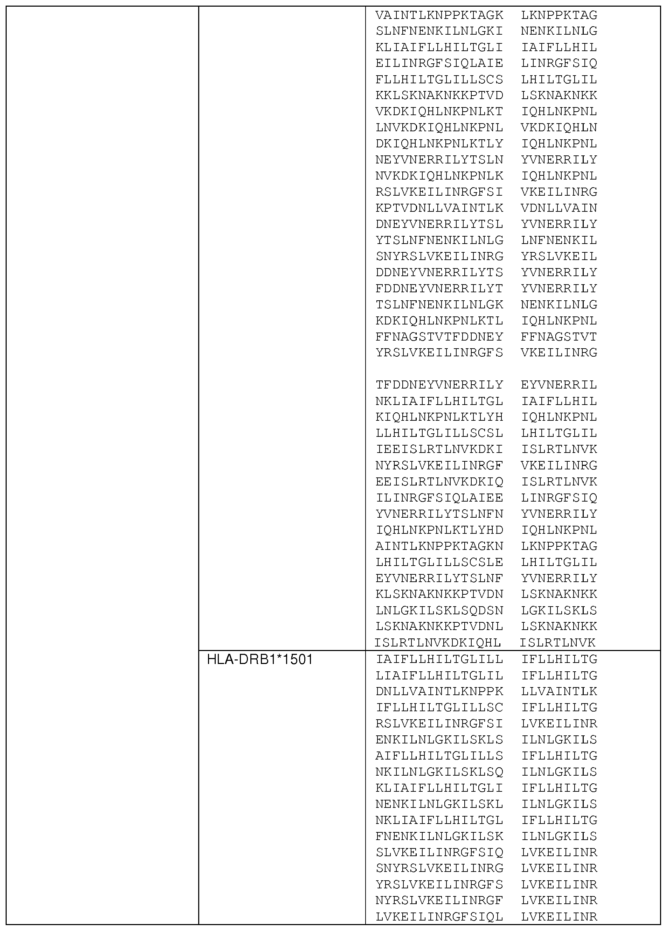

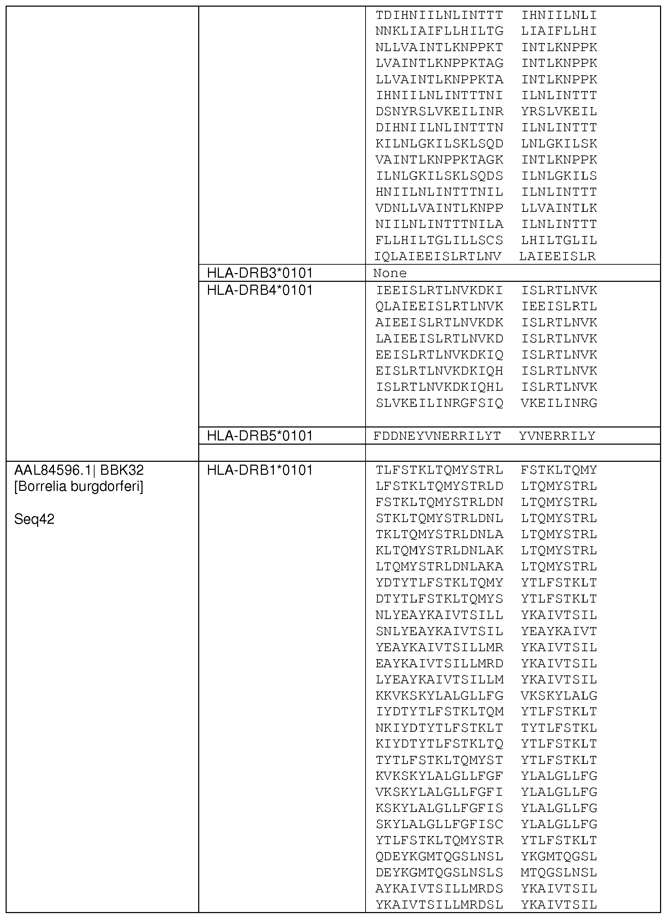

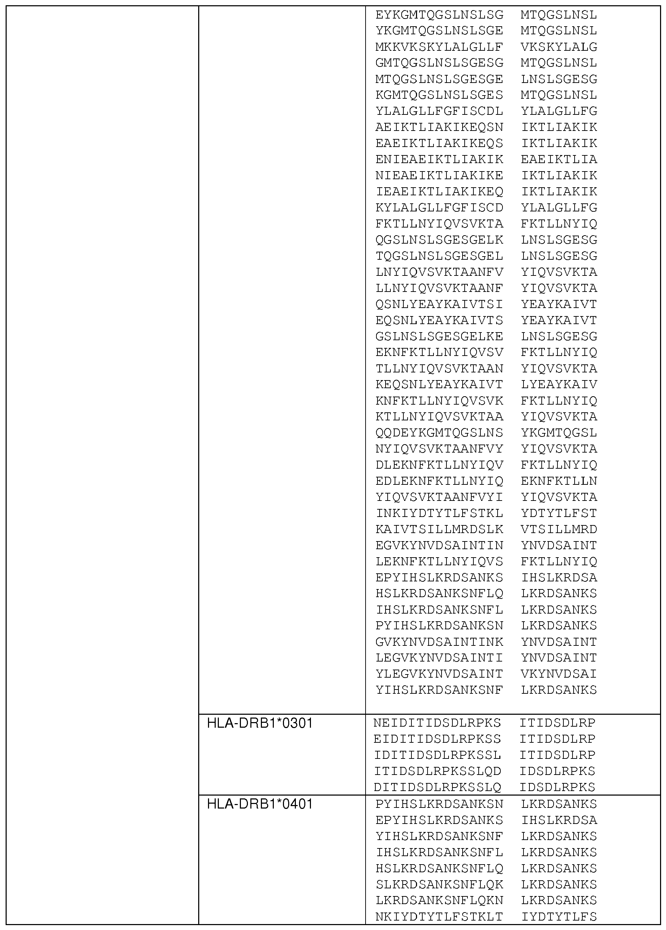

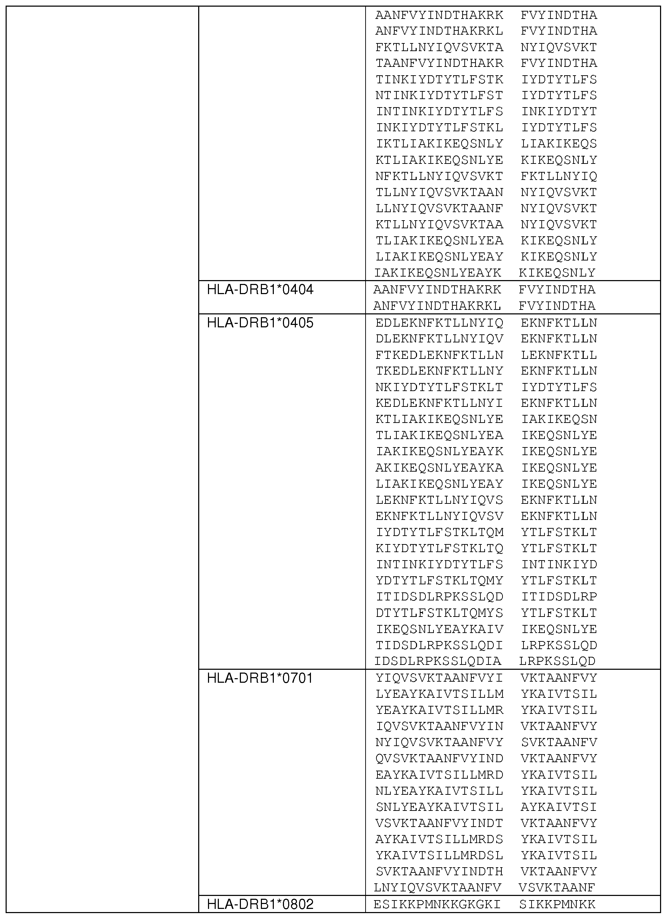

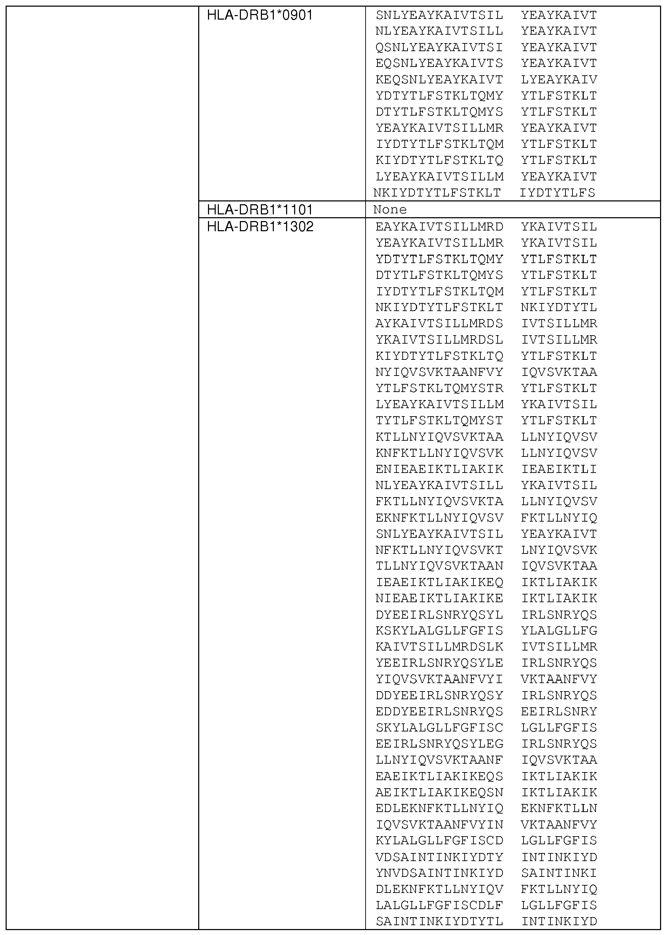

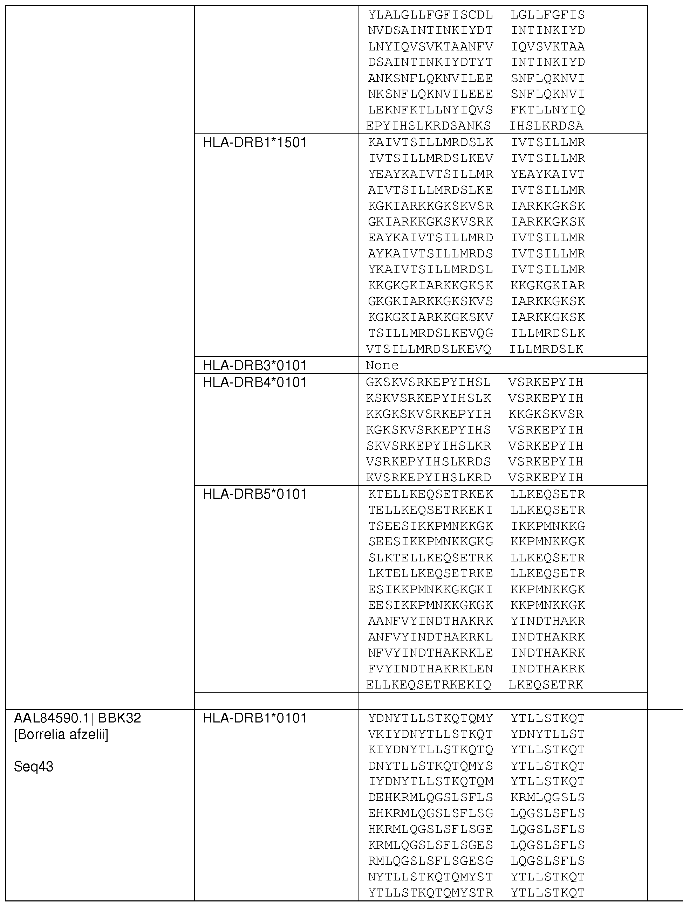

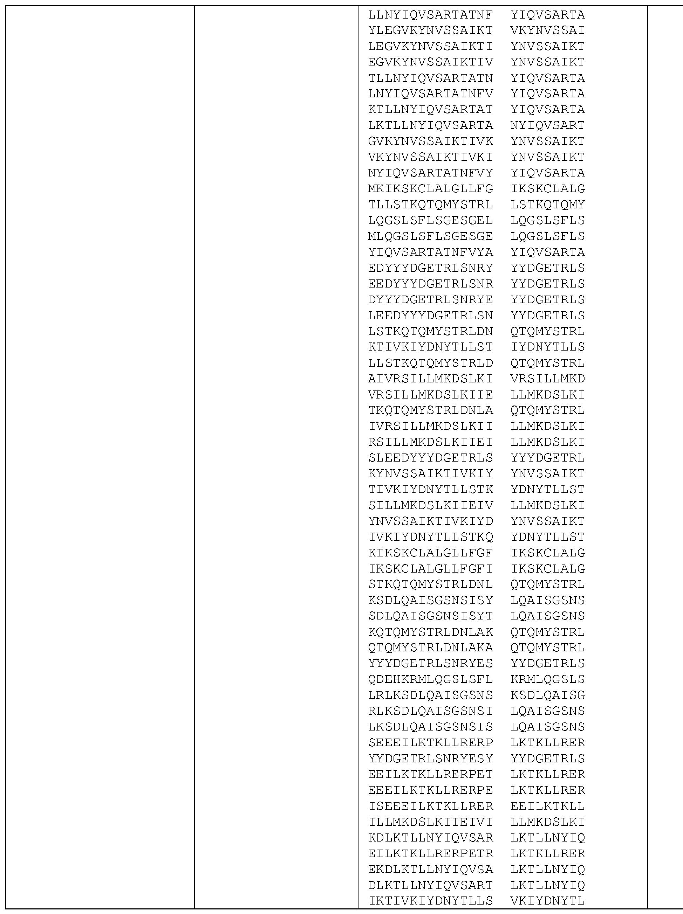

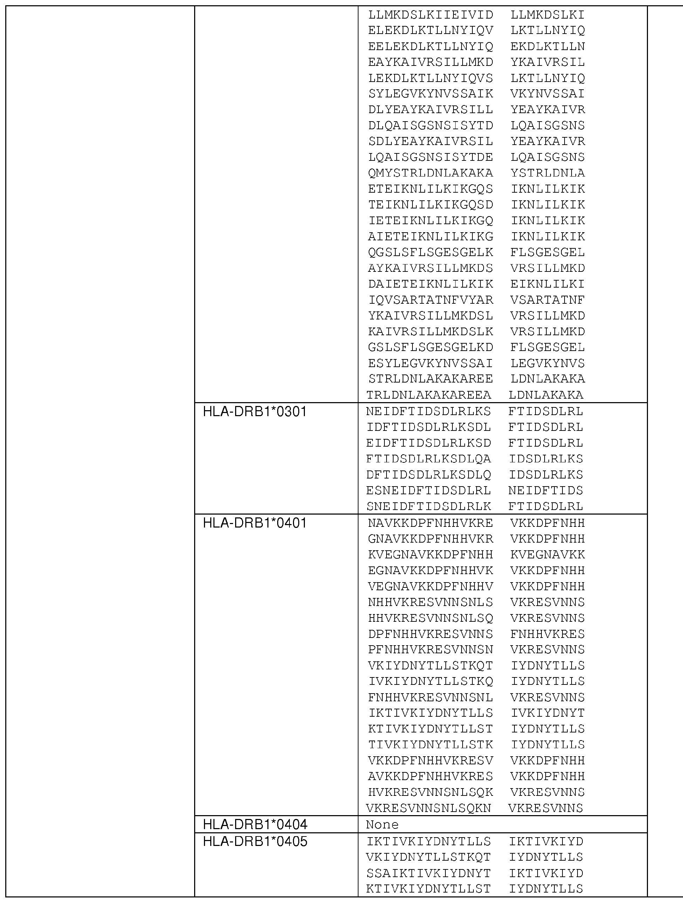

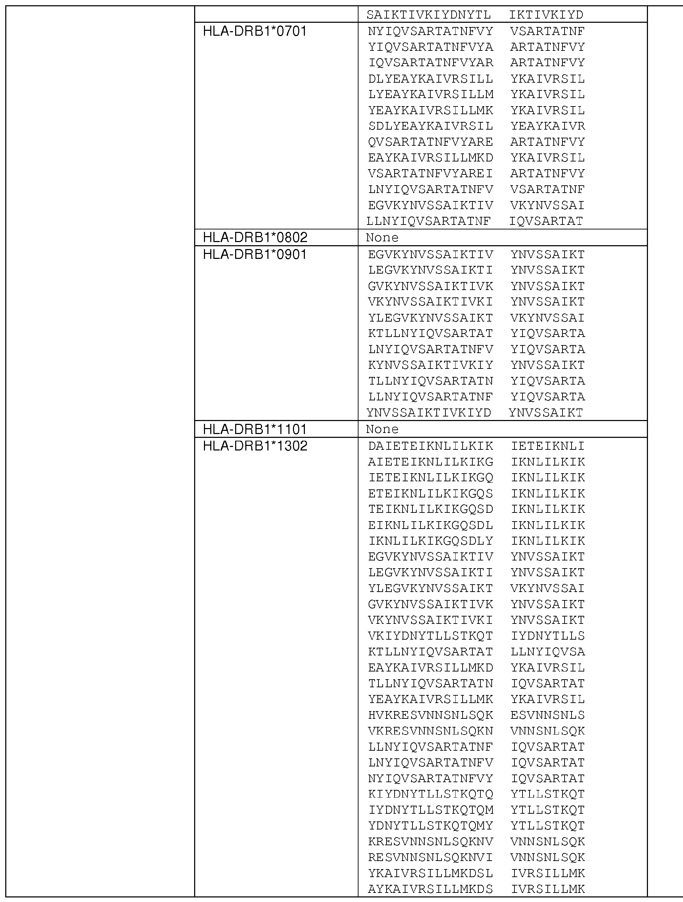

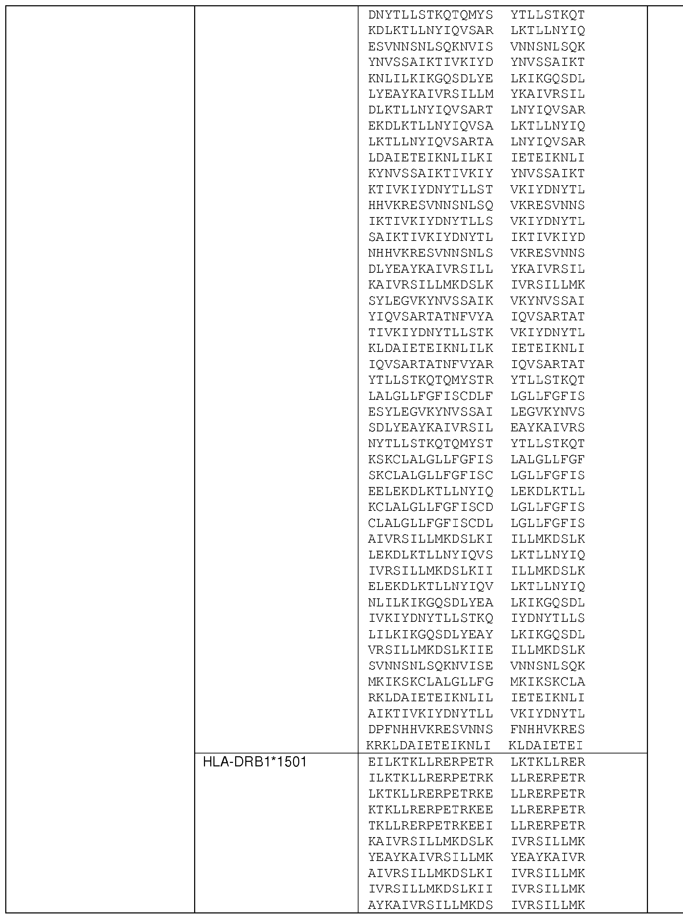

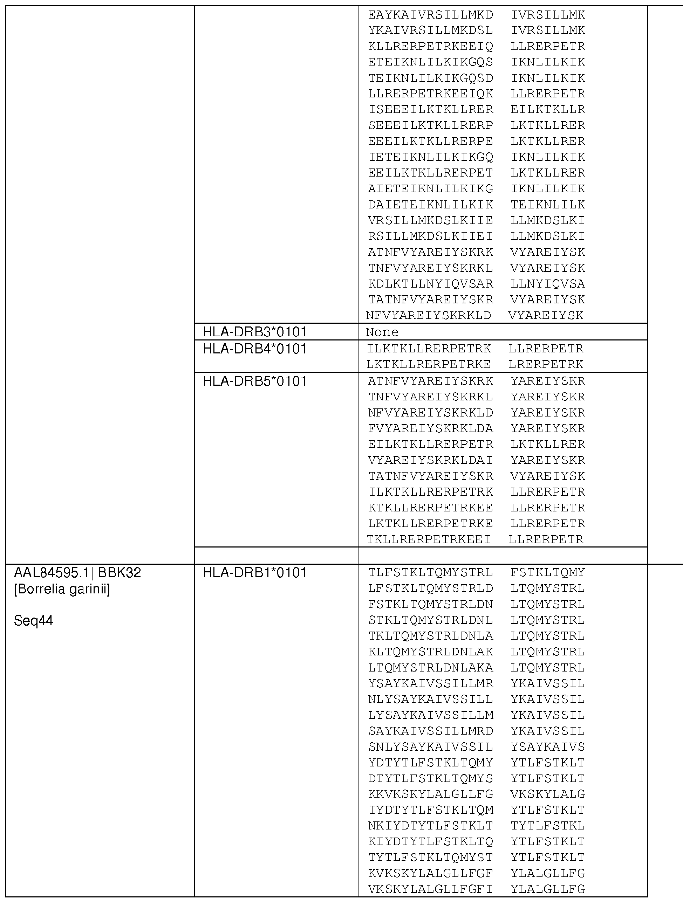

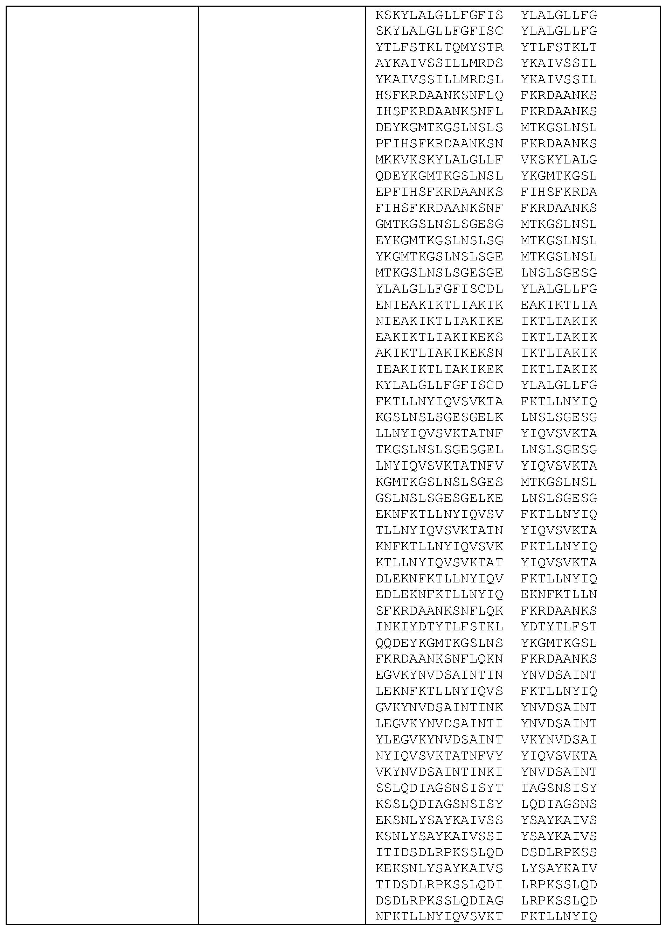

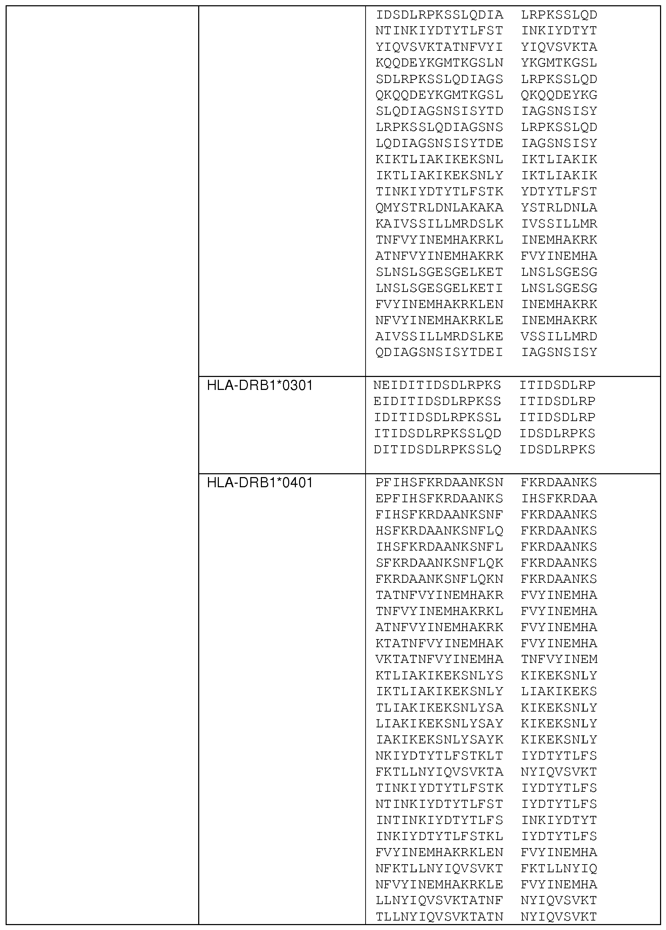

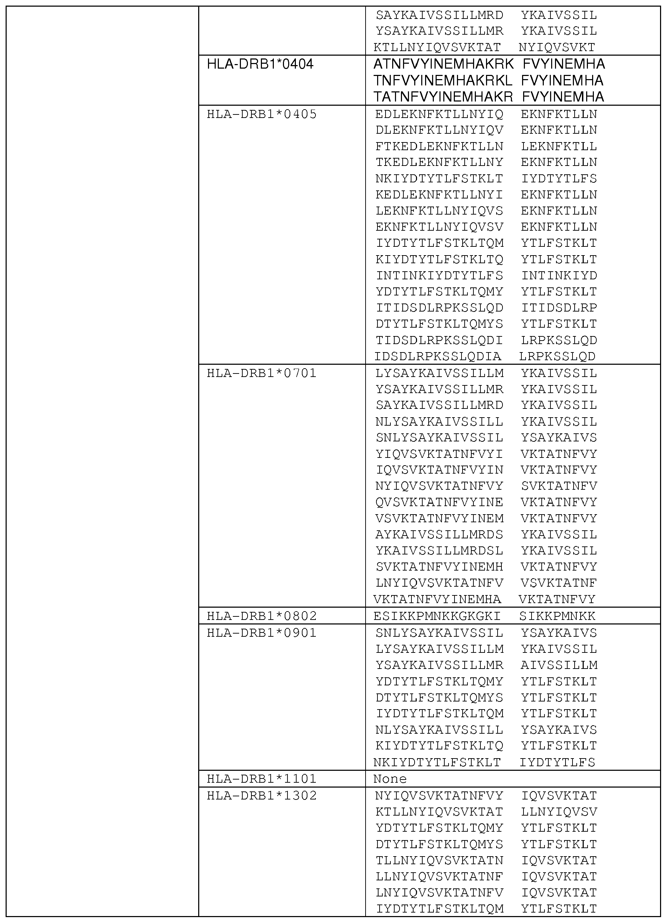

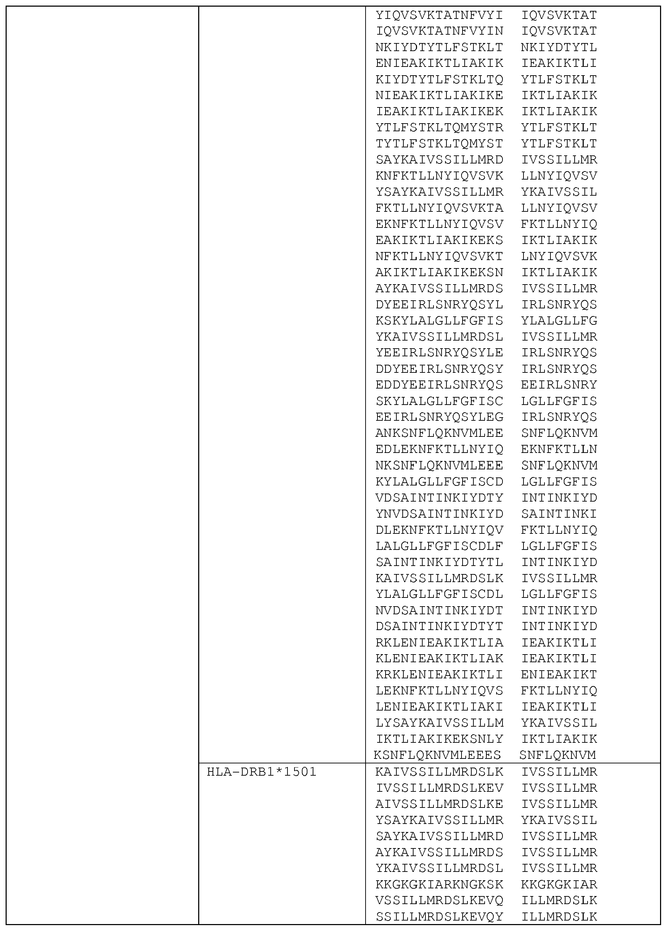

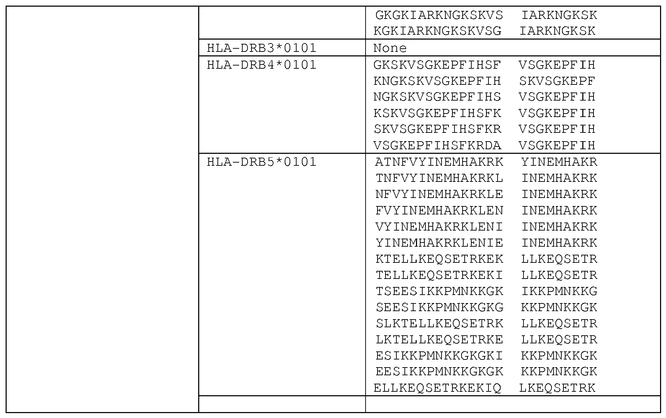

In another preferred embodiment the present disclosure relates to one or more MHC-peptide complexes or MHC multimers comprising one or more borrelia class II antigen peptide fragments comprising one or more of the 9 mer core peptides listed in Figure 34.

In another preferred embodiment the present disclosure relates to one or more borrelia class II antigen peptide fragments comprising one or more of the 9 mer core peptides listed in Figure 34.

The above mentioned 9 mer core peptides can be part of one or more 13 mers, 14 mers, 15 mers, 16 mers, 17 mers, 18 mers, 19 mers, 20 mers, 21 mers, 22 mers, 23,mers, 24 mers or 25 mer.

The 9 mer core peptides can have different positions in the 13 mers, 14 mers, 15 mers, 16 mers, 17 mers, 18 mers, 19 mers, 20 mers, 21 mers, 22 mers, 23,mers, 24 mers or 25 mer.

In one preferred embodiment of the present disclosure the 9 mer starts at position 1 from the N-terminus of the 13 mer.

In one preferred embodiment of the present disclosure the 9 mer starts at position 2 from the N-terminus of the 13 mer.

In one preferred embodiment of the present disclosure the 9 mer starts at position 3 from the N-terminus of the 13 mer.

In one preferred embodiment of the present disclosure the 9 mer starts at position 4 from the N-terminus of the 13 mer.

In one preferred embodiment of the present disclosure the 9 mer starts at position 5 from the N-terminus of the 13 mer.

In one preferred embodiment of the present disclosure the 9 mer starts at position 1 from the N-terminus of the 14 mer.

In one preferred embodiment of the present disclosure the 9 mer starts at position 2 from the N-terminus of the 14 mer.

In one preferred embodiment of the present disclosure the 9 mer starts at position 3 from the N-terminus of the 14 mer.

In one preferred embodiment of the present disclosure the 9 mer starts at position 4 from the N-terminus of the 14 mer.

In one preferred embodiment of the present disclosure the 9 mer starts at position 5 from the N-terminus of the 14 mer.

In one preferred embodiment of the present disclosure the 9 mer starts at position 6 from the N-terminus of the 14 mer.

In one preferred embodiment of the present disclosure the 9 mer starts at position 1 from the N-terminus of the 15 mer.

In one preferred embodiment of the present disclosure the 9 mer starts at position 2 from the N-terminus of the 15 mer.

In one preferred embodiment of the present disclosure the 9 mer starts at position 3 from the N-terminus of the 15 mer.

In one preferred embodiment of the present disclosure the 9 mer starts at position 4 from the N-terminus of the 15 mer.

In one preferred embodiment of the present disclosure the 9 mer starts at position 5 from the N-terminus of the 15 mer.

In one preferred embodiment of the present disclosure the 9 mer starts at position 6 from the N-terminus of the 15 mer.

In one preferred embodiment of the present disclosure the 9 mer starts at position 7 from the N-terminus of the 15 mer.

In one preferred embodiment of the present disclosure the 9 mer starts at position 1 from the N-terminus of the 16 mer.

In one preferred embodiment of the present disclosure the 9 mer starts at position 2 from the N-terminus of the 16 mer.

In one preferred embodiment of the present disclosure the 9 mer starts at position 3 from the N-terminus of the 16 mer.

In one preferred embodiment of the present disclosure the 9 mer starts at position 4 from the N-terminus of the 16 mer.

In one preferred embodiment of the present disclosure the 9 mer starts at position 5 from the N-terminus of the 16 mer.

In one preferred embodiment of the present disclosure the 9 mer starts at position 6 from the N-terminus of the 16 mer.

In one preferred embodiment of the present disclosure the 9 mer starts at position 7 from the N-terminus of the 16 mer.

In one preferred embodiment of the present disclosure the 9 mer starts at position 8 from the N-terminus of the 16 mer.

In one preferred embodiment of the present disclosure the 9 mer starts at position 1 from the N-terminus of the 17 mer.

In one preferred embodiment of the present disclosure the 9 mer starts at position 2 from the N-terminus of the 17 mer.

In one preferred embodiment of the present disclosure the 9 mer starts at position 3 from the N-terminus of the 17 mer.

In one preferred embodiment of the present disclosure the 9 mer starts at position 4 from the N-terminus of the 17 mer.

In one preferred embodiment of the present disclosure the 9 mer starts at position 5 from the N-terminus of the 17 mer.

In one preferred embodiment of the present disclosure the 9 mer starts at position 6 from the N-terminus of the 17 mer.

In one preferred embodiment of the present disclosure the 9 mer starts at position 7 from the N-terminus of the 17 mer.

In one preferred embodiment of the present disclosure the 9 mer starts at position 8 from the N-terminus of the 17 mer.

In one preferred embodiment of the present disclosure the 9 mer starts at position 9 from the N-terminus of the 17 mer.

In one preferred embodiment of the present disclosure the 9 mer starts at position 1 from the N-terminus of the 18 mer.

In one preferred embodiment of the present disclosure the 9 mer starts at position 2 from the N-terminus of the 18 mer.

In one preferred embodiment of the present disclosure the 9 mer starts at position 3 from the N-terminus of the 18 mer.

In one preferred embodiment of the present disclosure the 9 mer starts at position 4 from the N-terminus of the 18 mer.

In one preferred embodiment of the present disclosure the 9 mer starts at position 5 from the N-terminus of the 18 mer.

In one preferred embodiment of the present disclosure the 9 mer starts at position 6 from the N-terminus of the 18 mer.

In one preferred embodiment of the present disclosure the 9 mer starts at position 7 from the N-terminus of the 18 mer.

In one preferred embodiment of the present disclosure the 9 mer starts at position 8 from the N-terminus of the 18 mer.

In one preferred embodiment of the present disclosure the 9 mer starts at position 9 from the N-terminus of the 18 mer.

In one preferred embodiment of the present disclosure the 9 mer starts at position 10 from the N-terminus of the 18 mer.

In one preferred embodiment of the present disclosure the 9 mer starts at position 1 from the N-terminus of the 19 mer.

In one preferred embodiment of the present disclosure the 9 mer starts at position 2 from the N-terminus of the 19 mer.

In one preferred embodiment of the present disclosure the 9 mer starts at position 3 from the N-terminus of the 19 mer.

In one preferred embodiment of the present disclosure the 9 mer starts at position 4 from the N-terminus of the 19 mer.

In one preferred embodiment of the present disclosure the 9 mer starts at position 5 from the N-terminus of the 19 mer.

In one preferred embodiment of the present disclosure the 9 mer starts at position 6 from the N-terminus of the 19 mer.

In one preferred embodiment of the present disclosure the 9 mer starts at position 7 from the N-terminus of the 19 mer.

In one preferred embodiment of the present disclosure the 9 mer starts at position 8 from the N-terminus of the 19 mer.

In one preferred embodiment of the present disclosure the 9 mer starts at position 9 from the N-terminus of the 19 mer.

In one preferred embodiment of the present disclosure the 9 mer starts at position 10 from the N-terminus of the 19 mer.

In one preferred embodiment of the present disclosure the 9 mer starts at position 11 from the N-terminus of the 19 mer.

In one preferred embodiment of the present disclosure the 9 mer starts at position 1 from the N-terminus of the 20 mer.

In one preferred embodiment of the present disclosure the 9 mer starts at position 2 from the N-terminus of the 20 mer.

In one preferred embodiment of the present disclosure the 9 mer starts at position 3 from the N-terminus of the 20 mer.

In one preferred embodiment of the present disclosure the 9 mer starts at position 4 from the N-terminus of the 20 mer.

In one preferred embodiment of the present disclosure the 9 mer starts at position 5 from the N-terminus of the 20 mer.

In one preferred embodiment of the present disclosure the 9 mer starts at position 6 from the N-terminus of the 20 mer.

In one preferred embodiment of the present disclosure the 9 mer starts at position 7 from the N-terminus of the 20 mer.

In one preferred embodiment of the present disclosure the 9 mer starts at position 8 from the N-terminus of the 20 mer.

In one preferred embodiment of the present disclosure the 9 mer starts at position 9 from the N-terminus of the 20 mer.

In one preferred embodiment of the present disclosure the 9 mer starts at position 10 from the N-terminus of the 20 mer.

In one preferred embodiment of the present disclosure the 9 mer starts at position 11 from the N-terminus of the 20 mer.

In one preferred embodiment of the present disclosure the 9 mer starts at position 12 from the N-terminus of the 20 mer.

In one preferred embodiment of the present disclosure the 9 mer starts at position 1 from the N-terminus of the 21 mer, 22 mer, 23 mer, 24 mer or 25 mer.

In one preferred embodiment of the present disclosure the 9 mer starts at position 2 from the N-terminus of the 21 mer, 22 mer, 23 mer, 24 mer or 25 mer.

In one preferred embodiment of the present disclosure the 9 mer starts at position 3 from the N-terminus of the 21 mer, 22 mer, 23 mer, 24 mer or 25 mer.

In one preferred embodiment of the present disclosure the 9 mer starts at position 4 from the N-terminus of the 21 mer, 22 mer, 23 mer, 24 mer or 25 mer.

In one preferred embodiment of the present disclosure the 9 mer starts at position 5 from the N-terminus of the 21 mer, 22 mer, 23 mer, 24 mer or 25 mer.

In one preferred embodiment of the present disclosure the 9 mer starts at position 6 from the N-terminus of the 21 mer, 22 mer, 23 mer, 24 mer or 25 mer.

In one preferred embodiment of the present disclosure the 9 mer starts at position 7 from the N-terminus of the 21 mer, 22 mer, 23 mer, 24 mer or 25 mer.

In one preferred embodiment of the present disclosure the 9 mer starts at position 8 from the N-terminus of the 21 mer, 22 mer, 23 mer, 24 mer or 25 mer.

In one preferred embodiment of the present disclosure the 9 mer starts at position 9 from the N-terminus of the 21 mer, 22 mer, 23 mer, 24 mer or 25 mer.

In one preferred embodiment of the present disclosure the 9 mer starts at position 10 from the N-terminus of the 21 mer, 22 mer, 23 mer, 24 mer or 25 mer.

In one preferred embodiment of the present disclosure the 9 mer starts at position 11 from the N-terminus of the 21 mer, 22 mer, 23 mer, 24 mer or 25 mer.

In one preferred embodiment of the present disclosure the 9 mer starts at position 12 from the N-terminus of the 21 mer, 22 mer, 23 mer, 24 mer or 25 mer.

In one preferred embodiment of the present disclosure the 9 mer starts at position 13 from the N-terminus of the 21 mer, 22 mer, 23 mer, 24 mer or 25 mer.

In one preferred embodiment of the present disclosure the 9 mer starts at position 14 from the N-terminus of the 22 mer, 23 mer, 24 mer or 25 mer.

In one preferred embodiment of the present disclosure the 9 mer starts at position 15 from the N-terminus of the 23 mer, 24 mer or 25 mer.

In one preferred embodiment of the present disclosure the 9 mer starts at position 16 from the N-terminus of the 24 mer or 25 mer.

In one preferred embodiment of the present disclosure the 9 mer starts at position 17 from the N-terminus of the 25 mer.

The amino acids surrounding the 9 mer core peptides in the 13 mers, 14 mers, 15 mers, 16 mers, 17 mers, 18 mers, 19 mers, 20 mers, 21 mers, 22 mers, 23 mers, 24 mers and/or 25 mers can be any amino acids.

Peptide fragments with amino acid substitutions

The present disclosure further relates to one or more antigenic Borrelia peptides that have one or more amino acid substitutions such as 1, 2, 3, 4, 5, 6, 7, or 8.

The present disclosure also relates to one or more MHC-peptide complexes or MHC multimers, wherein the one or more antigenic Borrelia peptides have one or more amino acid substitutions such as 1, 2, 3, 4, 5, 6, 7, or 8.

In one embodiment of the present disclosure the one or more amino acid substitutions are within the amino acid anchor motif. In another embodiment of the present disclosure the one or more amino acid substitutions are outside the amino acid anchor motif. In one embodiment of the present disclosure the one or more amino acid substitutions are within the 9 mer core motif. In another embodiment of the present disclosure the one or more amino acid substitutions are outside the 9 mer core motif.

In a preferred embodiment of the present disclosure these amino acid substitutions comprise substitution with an "equivalent amino acid residue". An "equivalent amino acid residue" refers to an amino acid residue capable of replacing another amino acid residue in a polypeptide without substantially altering the structure and/or functionality of the polypeptide. Equivalent amino acids thus have similar properties such as bulkiness of the side-chain, side chain polarity (polar or non-polar), hydrophobicity (hydrophobic or hydrophilic), pH (acidic, neutral or basic) and side chain organization of carbon molecules (aromatic/aliphatic). As such, "equivalent amino acid residues" can be regarded as "conservative amino acid substitutions".

The classification of equivalent amino acids refers in one embodiment of the present disclosure to the following classes: 1) HRK, 2) DENQ, 3) C, 4) STPAG, 5) MILV and 6) FYW.

Within the meaning of the term "equivivalent amino acid substitution" as applied herein, one amino acid may be substituted for another, in one embodiment of the present disclosure, within the groups of amino acids indicated herein below:

Amino acids having polar side chains (Asp, Glu, Lys, Arg, His, Asn, Gin, Ser, Thr, Tyr, and Cys,)
Amino acids having non-polar side chains (Gly, Ala, Val, Leu, Ile, Phe, Trp, Pro, and Met)
Amino acids having aliphatic side chains (Gly, Ala Val, Leu, Ile)
Amino acids having cyclic side chains (Phe, Tyr, Trp, His, Pro)
Amino acids having aromatic side chains (Phe, Tyr, Trp)
Amino acids having acidic side chains (Asp, Glu)
Amino acids having basic side chains (Lys, Arg, His)
Amino acids having amide side chains (Asn, Gin)
Amino acids having hydroxy side chains (Ser, Thr)
Amino acids having sulphor-containing side chains (Cys, Met),
Neutral, weakly hydrophobic amino acids (Pro, Ala, Gly, Ser, Thr)
Hydrophilic, acidic amino acids (Gin, Asn, Glu, Asp), and
Hydrophobic amino acids (Leu, Ile, Val)

A Venn diagram is another method for grouping of amino acids according to their properties (Livingstone & Barton, CABIOS, 9, 745-756, 1993). In another preferred embodiment of the present disclosure one or more amino acids may be substituted with another within the same Venn diagram group.

In another preferred embodiment of the present disclosure these amino acid substitutions comprise substitution with a "non-equivalent amino acid residue". Non-equivalent amino acid residues are amino acid residues with dissimilar properties to the properties of the amino acid they substitute according to the groupings described above.

In one preferred embodiment of the present disclosure the amino acid substitutions increases the affinity of the peptide for the MHC molecule and thereby increase the stability of the MHC-peptide complex.

In another preferred embodiment of the present disclosure the amino acid substitutions decreases the affinity of the peptide for the MHC molecule and thereby increase the stability of the MHC-peptide complex.

In one preferred embodiment of the present disclosure the amino acid substitutions increases the overall affinity of one or more T-cell receptors for the MHC-peptide complex containing the modified antigenic peptide.

In another preferred embodiment of the present disclosure the amino acid substitutions decreases the overall affinity of one or more T-cell receptors for the MHC-peptide complex containing the modified antigenic peptide.

Gene variants

The present disclosure further relates to one or more MHC-peptide complexes or MHC multimers, wherein the one or more antigenic Borrelia peptides are encoded by one or more gene variants.

The present disclosure also relates to one or more antigenic Borrelia peptides that are encoded by one or more gene variants.

The term "variant gene" refers to nucleic acid molecules that encode a polypeptide having an amino acid sequence that is a modification of the polypeptides according to the present disclosure. Such variants include naturally-occurring polymorphisms of genes according to the present disclosure, as well as synthetic genes that contain conservative amino acid substitutions of the amino acid sequence of a polypeptide according to the present disclosure. Additional variant forms of genes are nucleic acid molecules that contain insertions or deletions of the nucleotide sequences described herein. A variant gene according to the present disclosure can be identified by determining whether the gene hybridizes with a nucleic acid molecule having the nucleotide sequence of a polypeptide according to the present disclosure, or its complement, under stringent conditions.

**Antigenic peptides**

The present disclosure relates in one embodiment to antigenic peptides derived from Borrelia antigens. The one or more antigenic peptides can in one embodiment of the present disclosure comprise one or more fragments from one or more Borrelia antigens capable of interacting with one or more MHC class 1 molecules. The one or more antigenic peptides can in another embodiment of the present disclosure comprise one or more fragments from one or more Borrelia antigens capable of interacting with one or more MHC class 2 molecules.

The antigenic peptides can be generated from any Borrelia antigen such as the Borrelia antigens listed in this application including the Borrelia antigens listed in figure 27, figure 28, figure 29, figure 30, figure 38 and figure 43.

MHC Class I and MHC Class II molecules have different structures, as described above, and therefore have different restrictions on the size of the peptide which may be accommodated. In general, MHC Class I molecules will accommodate peptides of from about 8 amino acids in length to about 11 amino acids. MHC Class II molecules will in general accommodate peptides of from about 13 amino acids in length to about 16 amino acids but may also accommodate longer peptides.

The antigenic peptides can in one embodiment of the present disclosure be generated by computational prediction e.g. using NetMHC (www.cbs.dtu.dk/services/NetMHC/) or by selection of specific 8, 9, 10, 11, 13, 14, 15 or 16 amino acid sequences. Figure 31, figure 32, figure 33, figure 34, figure 36, figure 37, figure 39, figure 40, figure 41, figure 42, figure 43, figure 44, figure 45, table A, table B and table C comprise antigen peptides including Borrelia antigenic peptides.

The present disclosure relates to one or more antigenic peptides and/or one or more MHC multimers, MHC-peptide complexes and/or one or more antigenic polypeptides comprising one or more antigenic peptides such as the antigenic peptides listed in the figures and/or tables in this application (comprising SEQ ID NO 1 to SEQ ID NO 217791) and/or the antigenic peptides characterized by item 1 to 735 herein below.

The one or more antigenic peptides can in one embodiment of the present disclosure comprise or consist of a fragment of one or more antigenic peptides listed in the figures and tables of this application (SEQ ID NO 1 to SEQ ID NO 217791) and/or the antigenic peptides characterized by item 1 to 735 herein below, such as a fragment consisting of 1, 2, 3, 4, 5, 6, 7, 8, 9, 10, 11, 12, 13, 14, 15 or 16 amino acids. It is to be understood that said items are not meant to be limiting to the peptide according to the present disclosure in that said peptide may consist of more than said 8 to 16 amino acids, but at least comprising said 8 to 16 amino acids.

Thus, in another embodiment of the present disclosure the antigenic peptide listed in figures and/or tables in this application (including SEQ ID NO 1 to SEQ ID NO 217791) and/or the antigenic peptides characterized by item 1 to 735 herein below can be a fragment or part of a larger antigenic polypeptide, wherein the larger antigenic polypeptide may be of a total length of 9, such as 10, for example 11, such as 12, for example 13, such as 14, for example 15, such as 16, for example 17, such as 18, for example 19, such as 20, for example 21, such as 22, for example 23, such as 24, for example 25, such as 26, for example 27, such as 28, for example 29, such as 30, for example 31, such as 32, for example 33, such as 34, for example 35, such as 36, for example 37, such as 38, for example 39, such as 40 amino acids, wherein 8 to 16 of said amino acids are defined in the items below. In another embodiment of the present disclosure, the larger polypeptide may be of a total length of between 20 to 30, such as 30-40, for example 40-50, such as 50-60, for example 60-70, such as 70-80, for example 80-90, such as 90-100, for example 100-150, such as 150-200, for example 200-250, such as 250-300, for example 300-500, such as 500-1000, for example 1000-2000, such as 2000-3000, for example 3000-4000, such as 4000-5000, for example 5000-10,000, such as 10,000-20,000, for example 20,000-30,000, such as 30,000-40,000, for example 40,000-50,000, such as 50,000-75,000, for example 75,000-100,000, such as 100,000-250,000, for example 250,000-,500,000, such as 500,000-1,000,000 amino acids.

In one embodiment of the present disclosure the antigenic peptides listed in the figures and/or tables of this application (SEQ ID NO 1 to SEQ ID NO 217791) are modified by one or more type(s) of post-translational modifications such as one or more of the post-translational modifications listed in the items (item 1 to 735) herein below. The same or different types of post-translational modification can occur on one or more amino acids in the antigenic peptide. Thus, in one embodiment of the present disclosure, any one amino acid may be modified once, twice or three times with the same or different types of modifications. Furthermore, said identical and/or different modification may be present on 1, 2, 3, 4, 5, 6, 7, 8, 9, 10, 11, 12, 13, 14, 15, 16, 17 or 18 of the amino acid residues of the peptide according to the present disclosure as defined in the items below. In addition, modifications may also be present on amino acid residues outside said 8 to 16 amino acids, in case the peptide is part of a larger protein.

Preferred Borrelia afzelii fragments of Osp C capable of interacting with one or more MHC class 1 and/or MHC class 2 molecules are listed in Table A.

**Table A: Prediction of MHC class 1 and 2 Borrelia afzelii OspC peptide binders. Prediction of 8-, 9-, 10-, 11-, 13-, 14-, 15-, 16-mer peptides were generated using the program displayed in figure 2.**

```
8 mers:

MKKNTLSA; KKNTLSAI; KNTLSAIL; NTLSAILM; TLSAILMT; LSAILMTL;
SAILMTLF; AILMTLFL; ILMTLFLF; LMTLFLFI; MTLFLFIS; TLFLFISC;
LFLFISCN; FLFISCNN; LFISCNNS; FISCNNSG; ISCNNSGK; SCNNSGKG;
CNNSGKGG; NNSGKGGD; NSGKGGDS; SGKGGDSA; GKGGDSAS; KGGDSAST;
GGDSASTN; GDSASTNP; DSASTNPA; SASTNPAD; ASTNPADE; STNPADES;
TNPADESA; NPADESAK; PADESAKG; ADESAKGP; DESAKGPN; ESAKGPNL;
```

SAKGPNLT; AKGPNLTE; KGPNLTEI; GPNLTEIS; PNLTEISK; NLTEISKK;
LTEISKKI; TEISKKIT; EISKKITD; ISKKITDS; SKKITDSN; KKITDSNA;
KITDSNAF; ITDSNAFV; TDSNAFVL; DSNAFVLA; SNAFVLAV; NAFVLAVK;
AFVLAVKE; FVLAVKEV; VLAVKEVE; LAVKEVET; AVKEVETL; VKEVETLV;
KEVETLVS; EVETLVSS; VETLVSSI; ETLVSSID; TLVSSIDE; LVSSIDEL;
VSSIDELA; SSIDELAN; SIDELANK; IDELANKA; DELANKAI; ELANKAIG;
LANKAIGK; ANKAIGKK; NKAIGKKI; KAIGKKIQ; AIGKKIQQ; IGKKIQQN;
GKKIQQNG; KKIQQNGL; KIQQNGLG; IQQNGLGA; QQNGLGAE; QNGLGAEA;
NGLGAEAN; GLGAEANR; LGAEANRN; GAEANRNE; AEANRNES; EANRNESL;
ANRNESLL; NRNESLLA; RNESLLAG; NESLLAGV; ESLLAGVH; SLLAGVHE;
LLAGVHEI; LAGVHEIS; AGVHEIST; GVHEISTL; VHEISTLI; HEISTLIT;
EISTLITE; ISTLITEK; STLITEKL; TLITEKLS; LITEKLSK; ITEKLSKL;
TEKLSKLK; EKLSKLKN; KLSKLKNS; LSKLKNSG; SKLKNSGE; KLKNSGEL;
LKNSGELK; KNSGELKA; NSGELKAK; SGELKAKI; GELKAKIE; ELKAKIED;
LKAKIEDA; KAKIEDAK; AKIEDAKK; KIEDAKKC; IEDAKKCS; EDAKKCSE;
DAKKCSEE; AKKCSEEF; KKCSEEFT; KCSEEFTN; CSEEFTNK; SEEFTNKL;
EEFTNKLR; EFTNKLRV; FTNKLRVS; TNKLRVSH; NKLRVSHA; KLRVSHAD;
LRVSHADL; RVSHADLG; VSHADLGK; SHADLGKQ; HADLGKQG; ADLGKQGV;
DLGKQGVN; LGKQGVND; GKQGVNDD; KQGVNDDD; QGVNDDDA; GVNDDDAK;
VNDDDAKK; NDDDAKKA; DDDAKKAI; DDAKKAIL; DAKKAILK; AKKAILKT;
KKAILKTN; KAILKTNA; AILKTNAD; ILKTNADK; LKTNADKT; KTNADKTK;
TNADKTKG; NADKTKGA; ADKTKGAE; DKTKGAEE; KTKGAEEL; TKGAEELG;
KGAEELGK; GAEELGKL; AEELGKLF; EELGKLFK; ELGKLFKS; LGKLFKSV;
GKLFKSVE; KLFKSVEG; LFKSVEGL; FKSVEGLV; KSVEGLVK; SVEGLVKA;
VEGLVKAA; EGLVKAAQ; GLVKAAQE; LVKAAQEA; VKAAQEAL; KAAQEALT;
AAQEALTN; AQEALTNS; QEALTNSV; EALTNSVK; ALTNSVKE; LTNSVKEL;
TNSVKELT; NSVKELTS; SVKELTSP; VKELTSPV; KELTSPVV; ELTSPVVA;
LTSPVVAE; TSPVVAES; SPVVAESP; PVVAESPK; VVAESPKK; VAESPKKP

*9 mers:*

MKKNTLSAI; KKNTLSAIL; KNTLSAILM; NTLSAILMT; TLSAILMTL; LSAILMTLF;
SAILMTLFL; AILMTLFLF; ILMTLFLFI; LMTLFLFIS; MTLFLFISC; TLFLFISCN;
LFLFISCNN; FLFISCNNS; LFISCNNSG; FISCNNSGK; ISCNNSGKG; SCNNSGKGG;
CNNSGKGGD; NNSGKGGDS; NSGKGGDSA; SGKGGDSAS; GKGGDSAST; KGGDSASTN;
GGDSASTNP; GDSASTNPA; DSASTNPAD; SASTNPADE; ASTNPADES; STNPADESA;
TNPADESAK; NPADESAKG; PADESAKGP; ADESAKGPN; DESAKGPNL; ESAKGPNLT;
SAKGPNLTE; AKGPNLTEI; KGPNLTEIS; GPNLTEISK; PNLTEISKK; NLTEISKKI;
LTEISKKIT; TEISKKITD; EISKKITDS; ISKKITDSN; SKKITDSNA; KKITDSNAF;
KITDSNAFV; ITDSNAFVL; TDSNAFVLA; DSNAFVLAV; SNAFVLAVK; NAFVLAVKE;
AFVLAVKEV; FVLAVKEVE; VLAVKEVET; LAVKEVETL; AVKEVETLV; VKEVETLVS;
KEVETLVSS; EVETLVSSI; VETLVSSID; ETLVSSIDE; TLVSSIDEL; LVSSIDELA;
VSSIDELAN; SSIDELANK; SIDELANKA; IDELANKAI; DELANKAIG; ELANKAIGK;
LANKAIGKK; ANKAIGKKI; NKAIGKKIQ; KAIGKKIQQ; AIGKKIQQN; IGKKIQQNG;
GKKIQQNGL; KKIQQNGLG; KIQQNGLGA; IQQNGLGAE; QQNGLGAEA; QNGLGAEAN;
NGLGAEANR; GLGAEANRN; LGAEANRNE; GAEANRNES; AEANRNESL; EANRNESLL;
ANRNESLLA; NRNESLLAG; RNESLLAGV; NESLLAGVH; ESLLAGVHE; SLLAGVHEI;
LLAGVHEIS; LAGVHEIST; AGVHEISTL; GVHEISTLI; VHEISTLIT; HEISTLITE;
EISTLITEK; ISTLITEKL; STLITEKLS; TLITEKLSK; LITEKLSKL; ITEKLSKLK;
TEKLSKLKN; EKLSKLKNS; KLSKLKNSG; LSKLKNSGE; SKLKNSGEL; KLKNSGELK;
LKNSGELKA; KNSGELKAK; NSGELKAKI; SGELKAKIE; GELKAKIED; ELKAKIEDA;
LKAKIEDAK; KAKIEDAKK; AKIEDAKKC; KIEDAKKCS; IEDAKKCSE; EDAKKCSEE;
DAKKCSEEF; AKKCSEEFT; KKCSEEFTN; KCSEEFTNK; CSEEFTNKL; SEEFTNKLR;
EEFTNKLRV; EFTNKLRVS; FTNKLRVSH; TNKLRVSHA; NKLRVSHAD; KLRVSHADL;
LRVSHADLG; RVSHADLGK; VSHADLGKQ; SHADLGKQG; HADLGKQGV; ADLGKQGVN;
DLGKQGVND; LGKQGVNDD; GKQGVNDDD; KQGVNDDDA; QGVNDDDAK; GVNDDDAKK;
VNDDDAKKA; NDDDAKKAI; DDDAKKAIL; DDAKKAILK; DAKKAILKT; AKKAILKTN;
KKAILKTNA; KAILKTNAD; AILKTNADK; ILKTNADKT; LKTNADKTK; KTNADKTKG;
TNADKTKGA; NADKTKGAE; ADKTKGAEE; DKTKGAEEL; KTKGAEELG; TKGAEELGK;

KGAEELGKL; GAEELGKLF; AEELGKLFK; EELGKLFKS; ELGKLFKSV; LGKLFKSVE;
GKLFKSVEG; KLFKSVEGL; LFKSVEGLV; FKSVEGLVK; KSVEGLVKA; SVEGLVKAA;
VEGLVKAAQ; EGLVKAAQE; GLVKAAQEA; LVKAAQEAL; VKAAQEALT; KAAQEALTN;
AAQEALTNS; AQEALTNSV; QEALTNSVK; EALTNSVKE; ALTNSVKEL; LTNSVKELT;
TNSVKELTS; NSVKELTSP; SVKELTSPV; VKELTSPVV; KELTSPVVA; ELTSPVVAE;
LTSPVVAES; TSPVVAESP; SPVVAESPK; PVVAESPKK; VVAESPKKP

---

*10 mers:*

MKKNTLSAIL; KKNTLSAILM; KNTLSAILMT; NTLSAILMTL; TLSAILMTLF;
LSAILMTLFL; SAILMTLFLF; AILMTLFLFI; ILMTLFLFIS; LMTLFLFISC;
MTLFLFISCN; TLFLFISCNN; LFLFISCNNS; FLFISCNNSG; LFISCNNSGK;
FISCNNSGKG; ISCNNSGKGG; SCNNSGKGGD; CNNSGKGGDS; NNSGKGGDSA;
NSGKGGDSAS; SGKGGDSAST; GKGGDSASTN; KGGDSASTNP; GGDSASTNPA;
GDSASTNPAD; DSASTNPADE; SASTNPADES; ASTNPADESA; STNPADESAK;
TNPADESAKG; NPADESAKGP; PADESAKGPN; ADESAKGPNL; DESAKGPNLT;
ESAKGPNLTE; SAKGPNLTEI; AKGPNLTEIS; KGPNLTEISK; GPNLTEISKK;
PNLTEISKKI; NLTEISKKIT; LTEISKKITD; TEISKKITDS; EISKKITDSN;
ISKKITDSNA; SKKITDSNAF; KKITDSNAFV; KITDSNAFVL; ITDSNAFVLA;
TDSNAFVLAV; DSNAFVLAVK; SNAFVLAVKE; NAFVLAVKEV; AFVLAVKEVE;
FVLAVKEVET; VLAVKEVETL; LAVKEVETLV; AVKEVETLVS; VKEVETLVSS;
KEVETLVSSI; EVETLVSSID; VETLVSSIDE; ETLVSSIDEL; TLVSSIDELA;
LVSSIDELAN; VSSIDELANK; SSIDELANKA; SIDELANKAI; IDELANKAIG;
DELANKAIGK; ELANKAIGKK; LANKAIGKKI; ANKAIGKKIQ; NKAIGKKIQQ;
KAIGKKIQQN; AIGKKIQQNG; IGKKIQQNGL; GKKIQQNGLG; KKIQQNGLGA;
KIQQNGLGAE; IQQNGLGAEA; QQNGLGAEAN; QNGLGAEANR; NGLGAEANRN;
GLGAEANRNE; LGAEANRNES; GAEANRNESL; AEANRNESLL; EANRNESLLA;
ANRNESLLAG; NRNESLLAGV; RNESLLAGVH; NESLLAGVHE; ESLLAGVHEI;
SLLAGVHEIS; LLAGVHEIST; LAGVHEISTL; AGVHEISTLI; GVHEISTLIT;
VHEISTLITE; HEISTLITEK; EISTLITEKL; ISTLITEKLS; STLITEKLSK;
TLITEKLSKL; LITEKLSKLK; ITEKLSKLKN; TEKLSKLKNS; EKLSKLKNSG;
KLSKLKNSGE; LSKLKNSGEL; SKLKNSGELK; KLKNSGELKA; LKNSGELKAK;
KNSGELKAKI; NSGELKAKIE; SGELKAKIED; GELKAKIEDA; ELKAKIEDAK;
LKAKIEDAKK; KAKIEDAKKC; AKIEDAKKCS; KIEDAKKCSE; IEDAKKCSEE;
EDAKKCSEEF; DAKKCSEEFT; AKKCSEEFTN; KKCSEEFTNK; KCSEEFTNKL;
CSEEFTNKLR; SEEFTNKLRV; EEFTNKLRVS; EFTNKLRVSH; FTNKLRVSHA;
TNKLRVSHAD; NKLRVSHADL; KLRVSHADLG; LRVSHADLGK; RVSHADLGKQ;
VSHADLGKQG; SHADLGKQGV; HADLGKQGVN; ADLGKQGVND; DLGKQGVNDD;
LGKQGVNDDD; GKQGVNDDDA; KQGVNDDDAK; QGVNDDDAKK; GVNDDDAKKA;
VNDDDAKKAI; NDDDAKKAIL; DDDAKKAILK; DDAKKAILKT; DAKKAILKTN;
AKKAILKTNA; KKAILKTNAD; KAILKTNADK; AILKTNADKT; ILKTNADKTK;
LKTNADKTKG; KTNADKTKGA; TNADKTKGAE; NADKTKGAEE; ADKTKGAEEL;
DKTKGAEELG; KTKGAEELGK; TKGAEELGKL; KGAEELGKLF; GAEELGKLFK;
AEELGKLFKS; EELGKLFKSV; ELGKLFKSVE; LGKLFKSVEG; GKLFKSVEGL;
KLFKSVEGLV; LFKSVEGLVK; FKSVEGLVKA; KSVEGLVKAA; SVEGLVKAAQ;
VEGLVKAAQE; EGLVKAAQEA; GLVKAAQEAL; LVKAAQEALT; VKAAQEALTN;
KAAQEALTNS; AAQEALTNSV; AQEALTNSVK; QEALTNSVKE; EALTNSVKEL;
ALTNSVKELT; LTNSVKELTS; TNSVKELTSP; NSVKELTSPV; SVKELTSPVV;
VKELTSPVVA; KELTSPVVAE; ELTSPVVAES; LTSPVVAESP; TSPVVAESPK;
SPVVAESPKK; PVVAESPKKP

---

*11 mers:*

MKKNTLSAILM; KKNTLSAILMT; KNTLSAILMTL; NTLSAILMTLF; TLSAILMTLFL;
LSAILMTLFLF; SAILMTLFLFI; AILMTLFLFIS; ILMTLFLFISC; LMTLFLFISCN;
MTLFLFISCNN; TLFLFISCNNS; LFLFISCNNSG; FLFISCNNSGK; LFISCNNSGKG;
FISCNNSGKGG; ISCNNSGKGGD; SCNNSGKGGDS; CNNSGKGGDSA; NNSGKGGDSAS;
NSGKGGDSAST; SGKGGDSASTN; GKGGDSASTNP; KGGDSASTNPA; GGDSASTNPAD;
GDSASTNPADE; DSASTNPADES; SASTNPADESA; ASTNPADESAK; STNPADESAKG;

TNPADESAKGP; NPADESAKGPN; PADESAKGPNL; ADESAKGPNLT; DESAKGPNLTE;
ESAKGPNLTEI; SAKGPNLTEIS; AKGPNLTEISK; KGPNLTEISKK; GPNLTEISKKI;
PNLTEISKKIT; NLTEISKKITD; LTEISKKITDS; TEISKKITDSN; EISKKITDSNA;
ISKKITDSNAF; SKKITDSNAFV; KKITDSNAFVL; KITDSNAFVLA; ITDSNAFVLAV;
TDSNAFVLAVK; DSNAFVLAVKE; SNAFVLAVKEV; NAFVLAVKEVE; AFVLAVKEVET;
FVLAVKEVETL; VLAVKEVETLV; LAVKEVETLVS; AVKEVETLVSS; VKEVETLVSSI;
KEVETLVSSID; EVETLVSSIDE; VETLVSSIDEL; ETLVSSIDELA; TLVSSIDELAN;
LVSSIDELANK; VSSIDELANKA; SSIDELANKAI; SIDELANKAIG; IDELANKAIGK;
DELANKAIGKK; ELANKAIGKKI; LANKAIGKKIQ; ANKAIGKKIQQ; NKAIGKKIQQN;
KAIGKKIQQNG; AIGKKIQQNGL; IGKKIQQNGLG; GKKIQQNGLGA; KKIQQNGLGAE;
KIQQNGLGAEA; IQQNGLGAEAN; QQNGLGAEANR; QNGLGAEANRN; NGLGAEANRNE;
GLGAEANRNES; LGAEANRNESL; GAEANRNESLL; AEANRNESLLA; EANRNESLLAG;
ANRNESLLAGV; NRNESLLAGVH; RNESLLAGVHE; NESLLAGVHEI; ESLLAGVHEIS;
SLLAGVHEIST; LLAGVHEISTL; LAGVHEISTLI; AGVHEISTLIT; GVHEISTLITE;
VHEISTLITEK; HEISTLITEKL; EISTLITEKLS; ISTLITEKLSK; STLITEKLSKL;
TLITEKLSKLK; LITEKLSKLKN; ITEKLSKLKNS; TEKLSKLKNSG; EKLSKLKNSGE;
KLSKLKNSGEL; LSKLKNSGELK; SKLKNSGELKA; KLKNSGELKAK; LKNSGELKAKI;
KNSGELKAKIE; NSGELKAKIED; SGELKAKIEDA; GELKAKIEDAK; ELKAKIEDAKK;
LKAKIEDAKKC; KAKIEDAKKCS; AKIEDAKKCSE; KIEDAKKCSEE; IEDAKKCSEEF;
EDAKKCSEEFT; DAKKCSEEFTN; AKKCSEEFTNK; KKCSEEFTNKL; KCSEEFTNKLR;
CSEEFTNKLRV; SEEFTNKLRVS; EEFTNKLRVSH; EFTNKLRVSHA; FTNKLRVSHAD;
TNKLRVSHADL; NKLRVSHADLG; KLRVSHADLGK; LRVSHADLGKQ; RVSHADLGKQG;
VSHADLGKQGV; SHADLGKQGVN; HADLGKQGVND; ADLGKQGVNDD; DLGKQGVNDDD;
LGKQGVNDDDA; GKQGVNDDDAK; KQGVNDDDAKK; QGVNDDDAKKA; GVNDDDAKKAI;
VNDDDAKKAIL; NDDDAKKAILK; DDDAKKAILKT; DDAKKAILKTN; DAKKAILKTNA;
AKKAILKTNAD; KKAILKTNADK; KAILKTNADKT; AILKTNADKTK; ILKTNADKTKG;
LKTNADKTKGA; KTNADKTKGAE; TNADKTKGAEE; NADKTKGAEEL; ADKTKGAEELG;
DKTKGAEELGK; KTKGAEELGKL; TKGAEELGKLF; KGAEELGKLFK; GAEELGKLFKS;
AEELGKLFKSV; EELGKLFKSVE; ELGKLFKSVEG; LGKLFKSVEGL; GKLFKSVEGLV;
KLFKSVEGLVK; LFKSVEGLVKA; FKSVEGLVKAA; KSVEGLVKAAQ; SVEGLVKAAQE;
VEGLVKAAQEA; EGLVKAAQEAL; GLVKAAQEALT; LVKAAQEALTN; VKAAQEALTNS;
KAAQEALTNSV; AAQEALTNSVK; AQEALTNSVKE; QEALTNSVKEL; EALTNSVKELT;
ALTNSVKELTS; LTNSVKELTSP; TNSVKELTSPV; NSVKELTSPVV; SVKELTSPVVA;
VKELTSPVVAE; KELTSPVVAES; ELTSPVVAESP; LTSPVVAESPK; TSPVVAESPKK;
SPVVAESPKKP;

---

*13 mers:*

MKKNTLSAILMTL; KKNTLSAILMTLF; KNTLSAILMTLFL; NTLSAILMTLFLF;
TLSAILMTLFLFI; LSAILMTLFLFIS; SAILMTLFLFISC; AILMTLFLFISCN;
ILMTLFLFISCNN; LMTLFLFISCNNS; MTLFLFISCNNSG; TLFLFISCNNSGK;
LFLFISCNNSGKG; FLFISCNNSGKGG; LFISCNNSGKGGD; FISCNNSGKGGDS;
ISCNNSGKGGDSA; SCNNSGKGGDSAS; CNNSGKGGDSAST; NNSGKGGDSASTN;
NSGKGGDSASTNP; SGKGGDSASTNPA; GKGGDSASTNPAD; KGGDSASTNPADE;
GGDSASTNPADES; GDSASTNPADESA; DSASTNPADESAK; SASTNPADESAKG;
ASTNPADESAKGP; STNPADESAKGPN; TNPADESAKGPNL; NPADESAKGPNLT;
PADESAKGPNLTE; ADESAKGPNLTEI; DESAKGPNLTEIS; ESAKGPNLTEISK;
SAKGPNLTEISKK; AKGPNLTEISKKI; KGPNLTEISKKIT; GPNLTEISKKITD;
PNLTEISKKITDS; NLTEISKKITDSN; LTEISKKITDSNA; TEISKKITDSNAF;
EISKKITDSNAFV; ISKKITDSNAFVL; SKKITDSNAFVLA; KKITDSNAFVLAV;
KITDSNAFVLAVK; ITDSNAFVLAVKE; TDSNAFVLAVKEV; DSNAFVLAVKEVE;
SNAFVLAVKEVET; NAFVLAVKEVETL; AFVLAVKEVETLV; FVLAVKEVETLVS;
VLAVKEVETLVSS; LAVKEVETLVSSI; AVKEVETLVSSID; VKEVETLVSSIDE;
KEVETLVSSIDEL; EVETLVSSIDELA; VETLVSSIDELAN; ETLVSSIDELANK;
TLVSSIDELANKA; LVSSIDELANKAI; VSSIDELANKAIG; SSIDELANKAIGK;
SIDELANKAIGKK; IDELANKAIGKKI; DELANKAIGKKIQ; ELANKAIGKKIQQ;
LANKAIGKKIQQN; ANKAIGKKIQQNG; NKAIGKKIQQNGL; KAIGKKIQQNGLG;
AIGKKIQQNGLGA; IGKKIQQNGLGAE; GKKIQQNGLGAEA; KKIQQNGLGAEAN;
KIQQNGLGAEANR; IQQNGLGAEANRN; QQNGLGAEANRNE; QNGLGAEANRNES;

NGLGAEANRNESL; GLGAEANRNESLL; LGAEANRNESLLA; GAEANRNESLLAG;
AEANRNESLLAGV; EANRNESLLAGVH; ANRNESLLAGVHE; NRNESLLAGVHEI;
RNESLLAGVHEIS; NESLLAGVHEIST; ESLLAGVHEISTL; SLLAGVHEISTLI;
LLAGVHEISTLIT; LAGVHEISTLITE; AGVHEISTLITEK; GVHEISTLITEKL;
VHEISTLITEKLS; HEISTLITEKLSK; EISTLITEKLSKL; ISTLITEKLSKLK;
STLITEKLSKLKN; TLITEKLSKLKNS; LITEKLSKLKNSG; ITEKLSKLKNSGE;
TEKLSKLKNSGEL; EKLSKLKNSGELK; KLSKLKNSGELKA; LSKLKNSGELKAK;
SKLKNSGELKAKI; KLKNSGELKAKIE; LKNSGELKAKIED; KNSGELKAKIEDA;
NSGELKAKIEDAK; SGELKAKIEDAKK; GELKAKIEDAKKC; ELKAKIEDAKKCS;
LKAKIEDAKKCSE; KAKIEDAKKCSEE; AKIEDAKKCSEEF; KIEDAKKCSEEFT;
IEDAKKCSEEFTN; EDAKKCSEEFTNK; DAKKCSEEFTNKL; AKKCSEEFTNKLR;
KKCSEEFTNKLRV; KCSEEFTNKLRVS; CSEEFTNKLRVSH; SEEFTNKLRVSHA;
EEFTNKLRVSHAD; EFTNKLRVSHADL; FTNKLRVSHADLG; TNKLRVSHADLGK;
NKLRVSHADLGKQ; KLRVSHADLGKQG; LRVSHADLGKQGV; RVSHADLGKQGVN;
VSHADLGKQGVND; SHADLGKQGVNDD; HADLGKQGVNDDD; ADLGKQGVNDDDA;
DLGKQGVNDDDAK; LGKQGVNDDDAKK; GKQGVNDDDAKKA; KQGVNDDDAKKAI;
QGVNDDDAKKAIL; GVNDDDAKKAILK; VNDDDAKKAILKT; NDDDAKKAILKTN;
DDDAKKAILKTNA; DDAKKAILKTNAD; DAKKAILKTNADK; AKKAILKTNADKT;
KKAILKTNADKTK; KAILKTNADKTKG; AILKTNADKTKGA; ILKTNADKTKGAE;
LKTNADKTKGAEE; KTNADKTKGAEEL; TNADKTKGAEELG; NADKTKGAEELGK;
ADKTKGAEELGKL; DKTKGAEELGKLF; KTKGAEELGKLFK; TKGAEELGKLFKS;
KGAEELGKLFKSV; GAEELGKLFKSVE; AEELGKLFKSVEG; EELGKLFKSVEGL;
ELGKLFKSVEGLV; LGKLFKSVEGLVK; GKLFKSVEGLVKA; KLFKSVEGLVKAA;
LFKSVEGLVKAAQ; FKSVEGLVKAAQE; KSVEGLVKAAQEA; SVEGLVKAAQEAL;
VEGLVKAAQEALT; EGLVKAAQEALTN; GLVKAAQEALTNS; LVKAAQEALTNSV;
VKAAQEALTNSVK; KAAQEALTNSVKE; AAQEALTNSVKEL; AQEALTNSVKELT;
QEALTNSVKELTS; EALTNSVKELTSP; ALTNSVKELTSPV; LTNSVKELTSPVV;
TNSVKELTSPVVA; NSVKELTSPVVAE; SVKELTSPVVAES; VKELTSPVVAESP;
KELTSPVVAESPK; ELTSPVVAESPKK; LTSPVVAESPKKP

*14 mers:*

MKKNTLSAILMTLF; KKNTLSAILMTLFL; KNTLSAILMTLFLF; NTLSAILMTLFLFI;
TLSAILMTLFLFIS; LSAILMTLFLFISC; SAILMTLFLFISCN; AILMTLFLFISCNN;
ILMTLFLFISCNNS; LMTLFLFISCNNSG; MTLFLFISCNNSGK; TLFLFISCNNSGKG;
LFLFISCNNSGKGG; FLFISCNNSGKGGD; LFISCNNSGKGGDS; FISCNNSGKGGDSA;
ISCNNSGKGGDSAS; SCNNSGKGGDSAST; CNNSGKGGDSASTN; NNSGKGGDSASTNP;
NSGKGGDSASTNPA; SGKGGDSASTNPAD; GKGGDSASTNPADE; KGGDSASTNPADES;
GGDSASTNPADESA; GDSASTNPADESAK; DSASTNPADESAKG; SASTNPADESAKGP;
ASTNPADESAKGPN; STNPADESAKGPNL; TNPADESAKGPNLT; NPADESAKGPNLTE;
PADESAKGPNLTEI; ADESAKGPNLTEIS; DESAKGPNLTEISK; ESAKGPNLTEISKK;
SAKGPNLTEISKKI; AKGPNLTEISKKIT; KGPNLTEISKKITD; GPNLTEISKKITDS;
PNLTEISKKITDSN; NLTEISKKITDSNA; LTEISKKITDSNAF; TEISKKITDSNAFV;
EISKKITDSNAFVL; ISKKITDSNAFVLA; SKKITDSNAFVLAV; KKITDSNAFVLAVK;
KITDSNAFVLAVKE; ITDSNAFVLAVKEV; TDSNAFVLAVKEVE; DSNAFVLAVKEVET;
SNAFVLAVKEVETL; NAFVLAVKEVETLV; AFVLAVKEVETLVS; FVLAVKEVETLVSS;
VLAVKEVETLVSSI; LAVKEVETLVSSID; AVKEVETLVSSIDE; VKEVETLVSSIDEL;
KEVETLVSSIDELA; EVETLVSSIDELAN; VETLVSSIDELANK; ETLVSSIDELANKA;
TLVSSIDELANKAI; LVSSIDELANKAIG; VSSIDELANKAIGK; SSIDELANKAIGKK;
SIDELANKAIGKKI; IDELANKAIGKKIQ; DELANKAIGKKIQQ; ELANKAIGKKIQQN;
LANKAIGKKIQQNG; ANKAIGKKIQQNGL; NKAIGKKIQQNGLG; KAIGKKIQQNGLGA;
AIGKKIQQNGLGAE; IGKKIQQNGLGAEA; GKKIQQNGLGAEAN; KKIQQNGLGAEANR;
KIQQNGLGAEANRN; IQQNGLGAEANRNE; QQNGLGAEANRNES; QNGLGAEANRNESL;
NGLGAEANRNESLL; GLGAEANRNESLLA; LGAEANRNESLLAG; GAEANRNESLLAGV;
AEANRNESLLAGVH; EANRNESLLAGVHE; ANRNESLLAGVHEI; NRNESLLAGVHEIS;
RNESLLAGVHEIST; NESLLAGVHEISTL; ESLLAGVHEISTLI; SLLAGVHEISTLIT;
LLAGVHEISTLITE; LAGVHEISTLITEK; AGVHEISTLITEKL; GVHEISTLITEKLS;
VHEISTLITEKLSK; HEISTLITEKLSKL; EISTLITEKLSKLK; ISTLITEKLSKLKN;
STLITEKLSKLKNS; TLITEKLSKLKNSG; LITEKLSKLKNSGE; ITEKLSKLKNSGEL;
TEKLSKLKNSGELK; EKLSKLKNSGELKA; KLSKLKNSGELKAK; LSKLKNSGELKAKI;

SKLKNSGELKAKIE; KLKNSGELKAKIED; LKNSGELKAKIEDA; KNSGELKAKIEDAK;
NSGELKAKIEDAKK; SGELKAKIEDAKKC; GELKAKIEDAKKCS; ELKAKIEDAKKCSE;
LKAKIEDAKKCSEE; KAKIEDAKKCSEEF; AKIEDAKKCSEEFT; KIEDAKKCSEEFTN;
IEDAKKCSEEFTNK; EDAKKCSEEFTNKL; DAKKCSEEFTNKLR; AKKCSEEFTNKLRV;
KKCSEEFTNKLRVS; KCSEEFTNKLRVSH; CSEEFTNKLRVSHA; SEEFTNKLRVSHAD;
EEFTNKLRVSHADL; EFTNKLRVSHADLG; FTNKLRVSHADLGK; TNKLRVSHADLGKQ;
NKLRVSHADLGKQG; KLRVSHADLGKQGV; LRVSHADLGKQGVN; RVSHADLGKQGVND;
VSHADLGKQGVNDD; SHADLGKQGVNDDD; HADLGKQGVNDDDA; ADLGKQGVNDDDAK;
DLGKQGVNDDDAKK; LGKQGVNDDDAKKA; GKQGVNDDDAKKAI; KQGVNDDDAKKAIL;
QGVNDDDAKKAILK; GVNDDDAKKAILKT; VNDDDAKKAILKTN; NDDDAKKAILKTNA;
DDDAKKAILKTNAD; DDAKKAILKTNADK; DAKKAILKTNADKT; AKKAILKTNADKTK;
KKAILKTNADKTKG; KAILKTNADKTKGA; AILKTNADKTKGAE; ILKTNADKTKGAEE;
LKTNADKTKGAEEL; KTNADKTKGAEELG; TNADKTKGAEELGK; NADKTKGAEELGKL;
ADKTKGAEELGKLF; DKTKGAEELGKLFK; KTKGAEELGKLFKS; TKGAEELGKLFKSV;
KGAEELGKLFKSVE; GAEELGKLFKSVEG; AEELGKLFKSVEGL; EELGKLFKSVEGLV;
ELGKLFKSVEGLVK; LGKLFKSVEGLVKA; GKLFKSVEGLVKAA; KLFKSVEGLVKAAQ;
LFKSVEGLVKAAQE; FKSVEGLVKAAQEA; KSVEGLVKAAQEAL; SVEGLVKAAQEALT;
VEGLVKAAQEALTN; EGLVKAAQEALTNS; GLVKAAQEALTNSV; LVKAAQEALTNSVK;
VKAAQEALTNSVKE; KAAQEALTNSVKEL; AAQEALTNSVKELT; AQEALTNSVKELTS;
QEALTNSVKELTSP; EALTNSVKELTSPV; ALTNSVKELTSPVV; LTNSVKELTSPVVA;
TNSVKELTSPVVAE; NSVKELTSPVVAES; SVKELTSPVVAESP; VKELTSPVVAESPK;
KELTSPVVAESPKK; ELTSPVVAESPKKP

_15 mers:_

MKKNTLSAILMTLFL; KKNTLSAILMTLFLF; KNTLSAILMTLFLFI; NTLSAILMTLFLFIS;
TLSAILMTLFLFISC; LSAILMTLFLFISCN; SAILMTLFLFISCNN; AILMTLFLFISCNNS;
ILMTLFLFISCNNSG; LMTLFLFISCNNSGK; MTLFLFISCNNSGKG; TLFLFISCNNSGKGG;
LFLFISCNNSGKGGD; FLFISCNNSGKGGDS; LFISCNNSGKGGDSA; FISCNNSGKGGDSAS;
ISCNNSGKGGDSAST; SCNNSGKGGDSASTN; CNNSGKGGDSASTNP; NNSGKGGDSASTNPA;
NSGKGGDSASTNPAD; SGKGGDSASTNPADE; GKGGDSASTNPADES; KGGDSASTNPADESA;
GGDSASTNPADESAK; GDSASTNPADESAKG; DSASTNPADESAKGP; SASTNPADESAKGPN;
ASTNPADESAKGPNL; STNPADESAKGPNLT; TNPADESAKGPNLTE; NPADESAKGPNLTEI;
PADESAKGPNLTEIS; ADESAKGPNLTEISK; DESAKGPNLTEISKK; ESAKGPNLTEISKKI;
SAKGPNLTEISKKIT; AKGPNLTEISKKITD; KGPNLTEISKKITDS; GPNLTEISKKITDSN;
PNLTEISKKITDSNA; NLTEISKKITDSNAF; LTEISKKITDSNAFV; TEISKKITDSNAFVL;
EISKKITDSNAFVLA; ISKKITDSNAFVLAV; SKKITDSNAFVLAVK; KKITDSNAFVLAVKE;
KITDSNAFVLAVKEV; ITDSNAFVLAVKEVE; TDSNAFVLAVKEVET; DSNAFVLAVKEVETL;
SNAFVLAVKEVETLV; NAFVLAVKEVETLVS; AFVLAVKEVETLVSS; FVLAVKEVETLVSSI;
VLAVKEVETLVSSID; LAVKEVETLVSSIDE; AVKEVETLVSSIDEL; VKEVETLVSSIDELA;
KEVETLVSSIDELAN; EVETLVSSIDELANK; VETLVSSIDELANKA; ETLVSSIDELANKAI;
TLVSSIDELANKAIG; LVSSIDELANKAIGK; VSSIDELANKAIGKK; SSIDELANKAIGKKI;
SIDELANKAIGKKIQ; IDELANKAIGKKIQQ; DELANKAIGKKIQQN; ELANKAIGKKIQQNG;
LANKAIGKKIQQNGL; ANKAIGKKIQQNGLG; NKAIGKKIQQNGLGA; KAIGKKIQQNGLGAE;
AIGKKIQQNGLGAEA; IGKKIQQNGLGAEAN; GKKIQQNGLGAEANR; KKIQQNGLGAEANRN;
KIQQNGLGAEANRNE; IQQNGLGAEANRNES; QQNGLGAEANRNESL; QNGLGAEANRNESLL;
NGLGAEANRNESLLA; GLGAEANRNESLLAG; LGAEANRNESLLAGV; GAEANRNESLLAGVH;
AEANRNESLLAGVHE; EANRNESLLAGVHEI; ANRNESLLAGVHEIS; NRNESLLAGVHEIST;
RNESLLAGVHEISTL; NESLLAGVHEISTLI; ESLLAGVHEISTLIT; SLLAGVHEISTLITE;
LLAGVHEISTLITEK; LAGVHEISTLITEKL; AGVHEISTLITEKLS; GVHEISTLITEKLSK;
VHEISTLITEKLSKL; HEISTLITEKLSKLK; EISTLITEKLSKLKN; ISTLITEKLSKLKNS;
STLITEKLSKLKNSG; TLITEKLSKLKNSGE; LITEKLSKLKNSGEL; ITEKLSKLKNSGELK;
TEKLSKLKNSGELKA; EKLSKLKNSGELKAK; KLSKLKNSGELKAKI; LSKLKNSGELKAKIE;
SKLKNSGELKAKIED; KLKNSGELKAKIEDA; LKNSGELKAKIEDAK; KNSGELKAKIEDAKK;
NSGELKAKIEDAKKC; SGELKAKIEDAKKCS; GELKAKIEDAKKCSE; ELKAKIEDAKKCSEE;
LKAKIEDAKKCSEEF; KAKIEDAKKCSEEFT; AKIEDAKKCSEEFTN; KIEDAKKCSEEFTNK;
IEDAKKCSEEFTNKL; EDAKKCSEEFTNKLR; DAKKCSEEFTNKLRV; AKKCSEEFTNKLRVS;
KKCSEEFTNKLRVSH; KCSEEFTNKLRVSHA; CSEEFTNKLRVSHAD; SEEFTNKLRVSHADL;
EEFTNKLRVSHADLG; EFTNKLRVSHADLGK; FTNKLRVSHADLGKQ; TNKLRVSHADLGKQG;
NKLRVSHADLGKQGV; KLRVSHADLGKQGVN; LRVSHADLGKQGVND; RVSHADLGKQGVNDD;

*VSHADLGKQGVNDDD; SHADLGKQGVNDDDA; HADLGKQGVNDDDAK; ADLGKQGVNDDDAKK;*
*DLGKQGVNDDDAKKA; LGKQGVNDDDAKKAI; GKQGVNDDDAKKAIL; KQGVNDDDAKKAILK;*
*QGVNDDDAKKAILKT; GVNDDDAKKAILKTN; VNDDDAKKAILKTNA; NDDDAKKAILKTNAD;*
*DDDAKKAILKTNADK; DDAKKAILKTNADKT; DAKKAILKTNADKTK; AKKAILKTNADKTKG;*
*KKAILKTNADKTKGA; KAILKTNADKTKGAE; AILKTNADKTKGAEE; ILKTNADKTKGAEEL;*
*LKTNADKTKGAEELG; KTNADKTKGAEELGK; TNADKTKGAEELGKL; NADKTKGAEELGKLF;*
*ADKTKGAEELGKLFK; DKTKGAEELGKLFKS; KTKGAEELGKLFKSV; TKGAEELGKLFKSVE;*
*KGAEELGKLFKSVEG; GAEELGKLFKSVEGL; AEELGKLFKSVEGLV; EELGKLFKSVEGLVK;*
*ELGKLFKSVEGLVKA; LGKLFKSVEGLVKAA; GKLFKSVEGLVKAAQ; KLFKSVEGLVKAAQE;*
*LFKSVEGLVKAAQEA; FKSVEGLVKAAQEAL; KSVEGLVKAAQEALT; SVEGLVKAAQEALTN;*
*VEGLVKAAQEALTNS; EGLVKAAQEALTNSV; GLVKAAQEALTNSVK; LVKAAQEALTNSVKE;*
*VKAAQEALTNSVKEL; KAAQEALTNSVKELT; AAQEALTNSVKELTS; AQEALTNSVKELTSP;*
*QEALTNSVKELTSPV; EALTNSVKELTSPVV; ALTNSVKELTSPVVA; LTNSVKELTSPVVAE;*
*TNSVKELTSPVVAES; NSVKELTSPVVAESP; SVKELTSPVVAESPK; VKELTSPVVAESPKK;*
*KELTSPVVAESPKKP*

*16 mers:*

*MKKNTLSAILMTLFLF; KKNTLSAILMTLFLFI; KNTLSAILMTLFLFIS;*
*NTLSAILMTLFLFISC; TLSAILMTLFLFISCN; LSAILMTLFLFISCNN;*
*SAILMTLFLFISCNNS; AILMTLFLFISCNNSG; ILMTLFLFISCNNSGK;*
*LMTLFLFISCNNSGKG; MTLFLFISCNNSGKGG; TLFLFISCNNSGKGGD;*
*LFLFISCNNSGKGGDS; FLFISCNNSGKGGDSA; LFISCNNSGKGGDSAS;*
*FISCNNSGKGGDSAST; ISCNNSGKGGDSASTN; SCNNSGKGGDSASTNP;*
*CNNSGKGGDSASTNPA; NNSGKGGDSASTNPAD; NSGKGGDSASTNPADE;*
*SGKGGDSASTNPADES; GKGGDSASTNPADESA; KGGDSASTNPADESAK;*
*GGDSASTNPADESAKG; GDSASTNPADESAKGP; DSASTNPADESAKGPN;*
*SASTNPADESAKGPNL; ASTNPADESAKGPNLT; STNPADESAKGPNLTE;*
*TNPADESAKGPNLTEI; NPADESAKGPNLTEIS; PADESAKGPNLTEISK;*
*ADESAKGPNLTEISKK; DESAKGPNLTEISKKI; ESAKGPNLTEISKKIT;*
*SAKGPNLTEISKKITD; AKGPNLTEISKKITDS; KGPNLTEISKKITDSN;*
*GPNLTEISKKITDSNA; PNLTEISKKITDSNAF; NLTEISKKITDSNAFV;*
*LTEISKKITDSNAFVL; TEISKKITDSNAFVLA; EISKKITDSNAFVLAV;*
*ISKKITDSNAFVLAVK; SKKITDSNAFVLAVKE; KKITDSNAFVLAVKEV;*
*KITDSNAFVLAVKEVE; ITDSNAFVLAVKEVET; TDSNAFVLAVKEVETL;*
*DSNAFVLAVKEVETLV; SNAFVLAVKEVETLVS; NAFVLAVKEVETLVSS;*
*AFVLAVKEVETLVSSI; FVLAVKEVETLVSSID; VLAVKEVETLVSSIDE;*
*LAVKEVETLVSSIDEL; AVKEVETLVSSIDELA; VKEVETLVSSIDELAN;*
*KEVETLVSSIDELANK; EVETLVSSIDELANKA; VETLVSSIDELANKAI;*
*ETLVSSIDELANKAIG; TLVSSIDELANKAIGK; LVSSIDELANKAIGKK;*
*VSSIDELANKAIGKKI; SSIDELANKAIGKKIQ; SIDELANKAIGKKIQQ;*
*IDELANKAIGKKIQQN; DELANKAIGKKIQQNG; ELANKAIGKKIQQNGL;*
*LANKAIGKKIQQNGLG; ANKAIGKKIQQNGLGA; NKAIGKKIQQNGLGAE;*
*KAIGKKIQQNGLGAEA; AIGKKIQQNGLGAEAN; IGKKIQQNGLGAEANR;*
*GKKIQQNGLGAEANRN; KKIQQNGLGAEANRNE; KIQQNGLGAEANRNES;*
*IQQNGLGAEANRNESL; QQNGLGAEANRNESLL; QNGLGAEANRNESLLA;*
*NGLGAEANRNESLLAG; GLGAEANRNESLLAGV; LGAEANRNESLLAGVH;*
*GAEANRNESLLAGVHE; AEANRNESLLAGVHEI; EANRNESLLAGVHEIS;*
*ANRNESLLAGVHEIST; NRNESLLAGVHEISTL; RNESLLAGVHEISTLI;*
*NESLLAGVHEISTLIT; ESLLAGVHEISTLITE; SLLAGVHEISTLITEK;*
*LLAGVHEISTLITEKL; LAGVHEISTLITEKLS; AGVHEISTLITEKLSK;*
*GVHEISTLITEKLSKL; VHEISTLITEKLSKLK; HEISTLITEKLSKLKN;*
*EISTLITEKLSKLKNS; ISTLITEKLSKLKNSG; STLITEKLSKLKNSGE;*
*TLITEKLSKLKNSGEL; LITEKLSKLKNSGELK; ITEKLSKLKNSGELKA;*
*TEKLSKLKNSGELKAK; EKLSKLKNSGELKAKI; KLSKLKNSGELKAKIE;*
*LSKLKNSGELKAKIED; SKLKNSGELKAKIEDA; KLKNSGELKAKIEDAK;*
*LKNSGELKAKIEDAKK; KNSGELKAKIEDAKKC; NSGELKAKIEDAKKCS;*
*SGELKAKIEDAKKCSE; GELKAKIEDAKKCSEE; ELKAKIEDAKKCSEEF;*
*LKAKIEDAKKCSEEFT; KAKIEDAKKCSEEFTN; AKIEDAKKCSEEFTNK;*
*KIEDAKKCSEEFTNKL; IEDAKKCSEEFTNKLR; EDAKKCSEEFTNKLRV;*

*DAKKCSEEFTNKLRVS; AKKCSEEFTNKLRVSH; KKCSEEFTNKLRVSHA;*
*KCSEEFTNKLRVSHAD; CSEEFTNKLRVSHADL; SEEFTNKLRVSHADLG;*
*EEFTNKLRVSHADLGK; EFTNKLRVSHADLGKQ; FTNKLRVSHADLGKQG;*
*TNKLRVSHADLGKQGV; NKLRVSHADLGKQGVN; KLRVSHADLGKQGVND;*
*LRVSHADLGKQGVNDD; RVSHADLGKQGVNDDD; VSHADLGKQGVNDDDA;*
*SHADLGKQGVNDDDAK; HADLGKQGVNDDDAKK; ADLGKQGVNDDDAKKA;*
*DLGKQGVNDDDAKKAI; LGKQGVNDDDAKKAIL; GKQGVNDDDAKKAILK;*
*KQGVNDDDAKKAILKT; QGVNDDDAKKAILKTN; GVNDDDAKKAILKTNA;*
*VNDDDAKKAILKTNAD; NDDDAKKAILKTNADK; DDDAKKAILKTNADKT;*
*DDAKKAILKTNADKTK; DAKKAILKTNADKTKG; AKKAILKTNADKTKGA;*
*KKAILKTNADKTKGAE; KAILKTNADKTKGAEE; AILKTNADKTKGAEEL;*
*ILKTNADKTKGAEELG; LKTNADKTKGAEELGK; KTNADKTKGAEELGKL;*
*TNADKTKGAEELGKLF; NADKTKGAEELGKLFK; ADKTKGAEELGKLFKS;*
*DKTKGAEELGKLFKSV; KTKGAEELGKLFKSVE; TKGAEELGKLFKSVEG;*
*KGAEELGKLFKSVEGL; GAEELGKLFKSVEGLV; AEELGKLFKSVEGLVK;*
*EELGKLFKSVEGLVKA; ELGKLFKSVEGLVKAA; LGKLFKSVEGLVKAAQ;*
*GKLFKSVEGLVKAAQE; KLFKSVEGLVKAAQEA; LFKSVEGLVKAAQEAL;*
*FKSVEGLVKAAQEALT; KSVEGLVKAAQEALTN; SVEGLVKAAQEALTNS;*
*VEGLVKAAQEALTNSV; EGLVKAAQEALTNSVK; GLVKAAQEALTNSVKE;*
*LVKAAQEALTNSVKEL; VKAAQEALTNSVKELT; KAAQEALTNSVKELTS;*
*AAQEALTNSVKELTSP; AQEALTNSVKELTSPV; QEALTNSVKELTSPVV;*
*EALTNSVKELTSPVVA; ALTNSVKELTSPVVAE; LTNSVKELTSPVVAES;*
*TNSVKELTSPVVAESP; NSVKELTSPVVAESPK; SVKELTSPVVAESPKK;*
*VKELTSPVVAESPKKP*

Preferred Borrelia burgdorferi fragments of Osp A capable of interacting with one or more MHC molecules are listed in Table B.

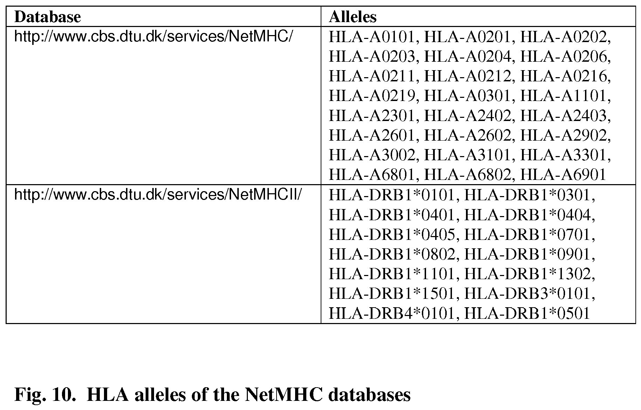

**Table B: Prediction of Borrelia burgdorferi OspA protein specific MHC class 1,  8- , 9- ,10- , 11-mer peptide binders for 24 MHC class 1 alleles (see figure 10) using the http://www.cbs.dtu.dk/services/NetMHC/ database. The MHC class 1 molecules for which no binders were found are not listed.**

| CAA44492.1\| Outer surface protein A [Borrelia burgdorferi]<br><br>Seq9 | HLA-A0101 | |
|---|---|---|
| | HLA-A0201 | YLLGIGLIL |
| | | FTLEGTLAA |
| | | KTSTLTISV |
| | | FTKEDTITV |
| | | ALIACKQNV |
| | | LLGIGLILA |
| | | SLEATVDKL |
| | | ILKSGEITV |
| | | KVTEGTVVL |
| | | STLDEKNSV |
| | | TLVSKKVTL |
| | | GIGLILALI |
| | | YLLGIGLILA |
| | | TLDEKNSVSV |
| | | ALDDSDTTQA |
| | | LLGIGLILAL |
| | | NILKSGEITV |
| | | TLAADGKTTL |
| | | VLKDFTLEGT |
| | | YSLEATVDKL |
| | | YLLGIGLILAL |

| | | |
|---|---|---|
| | | LVFTKEDTITV |
| | | LLGIGLILALI |
| | | ILALIACKQNV |
| | | STLDEKNSVSV |
| | | ALDDSDTTQAT |
| | | VLKDFTLEGTL |
| | | SLEATVDKLEL |
| | | ILKSGEITVAL |
| | | ALIACKQNVST |
| | | TTLKVTEGTVV |
| | | LAADGKTTLKV |
| | | LIACKQNVSTL |
| | HLA-A0301 | VVLSKNILK |
| | | KTKNLVFTK |
| | | LVSKKVTLK |
| | | LILALIACK |
| | | LTISVNSQK |
| | | KAVEITTLK |
| | | AADGKTTLK |
| | | ISVNSQKTK |
| | | RANGTRLEY |
| | | SQTKFEIFK |
| | | KSDGSGKAK |
| | | TQATKKTGK |
| | | MTELVSKEK |
| | | TLVSKKVTLK |
| | | TLTISVNSQK |
| | | TVVLSKNILK |
| | | GLILALIACK |
| | | TLKELKNALK |
| | | LSQTKFEIFK |
| | | LAADGKTTLK |
| | | VTEGTVVLSK |
| | | KLELKGTSDK |
| | | TTQATKKTGK |
| | | GMTELVSKEK |
| | | KYSLEATVDK |
| | | KQNVSTLDEK |
| | | TIADDLSQTK |
| | | TISVNSQKTK |
| | | KEDAKTLVSK |
| | | GSGKAKEVLK |
| | | KTIVRANGTR |
| | | GTLEGEKTDK |
| | | GKAVEITTLK |
| | | GTRLEYTDIK |
| | | VSKKVTLKDK |
| | | IKSDGSGKAK |
| | | TLAADGKTTLK |
| | | STLTISVNSQK |
| | | KTLVSKKVTLK |
| | | KVTEGTVVLSK |
| | | TTLKELKNALK |
| | | LTISVNSQKTK |
| | | GTVVLSKNILK |
| | | FTKEDTITVQK |
| | | IVRANGTRLEY |
| | | TLKDKSSTEEK |
| | | LVSKKVTLKDK |

| | | KFNEKGETSEK |
| | | LTIADDLSQTK |
| | | SQKTKNLVFTK |
| | | KSSTEEKFNEK |
| | | ATKKTGKWDSK |
| | HLA-A1101 | VVLSKNILK |
| | | KTKNLVFTK |
| | | SQTKFEIFK |
| | | LTISVNSQK |
| | | ATVDKLELK |
| | | LILALIACK |
| | | STEEKFNEK |
| | | KAVEITTLK |
| | | LVSKKVTLK |
| | | MTELVSKEK |
| | | AADGKTTLK |
| | | SAGTNLEGK |
| | | YSLEATVDK |
| | | GSGTLEGEK |
| | | TQATKKTGK |
| | | RANGTRLEY |
| | | IADDLSQTK |
| | | ISVNSQKTK |
| | | KSDGSGKAK |
| | | GGMTELVSK |
| | | TEGTVVLSK |
| | | EITTLKELK |
| | | TIVRANGTR |
| | | TVVLSKNILK |
| | | TTQATKKTGK |
| | | TIADDLSQTK |
| | | VTEGTVVLSK |
| | | LSQTKFEIFK |
| | | SSTEEKFNEK |
| | | GLILALIACK |
| | | KQNVSTLDEK |
| | | TLTISVNSQK |
| | | TLVSKKVTLK |
| | | TISVNSQKTK |
| | | LAADGKTTLK |
| | | GMTELVSKEK |
| | | TLKELKNALK |
| | | KTIVRANGTR |
| | | GSGKAKEVLK |
| | | GTLEGEKTDK |
| | | VSKKVTLKDK |
| | | GTRLEYTDIK |
| | | KLELKGTSDK |
| | | DSAGTNLEGK |
| | | STLTISVNSQK |
| | | TTLKELKNALK |
| | | KVTEGTVVLSK |
| | | KTLVSKKVTLK |
| | | GTVVLSKNILK |
| | | LTIADDLSQTK |
| | | TLAADGKTTLK |
| | | AVEITTLKELK |
| | | LTISVNSQKTK |
| | | SQKTKNLVFTK |

| | | KSSTEEKFNEK |
| | | ATKKTGKWDSK |
| | | TLKDKSSTEEK |
| | | FTKEDTITVQK |
| | | TLEGTLAADGK |
| | | LVSKKVTLKDK |
| | | MTELVSKEKDK |
| | | DLSQTKFEIFK |
| | | YTDIKSDGSGK |
| | | LVSKEKDKDGK |
| | | KFNEKGETSEK |
| | | IGLILALIACK |
| HLA-A2402 | KYLLGIGLI |
| | | KWDSKTSTL |
| | | KYDSAGTNL |
| | | KYLLGIGLIL |
| | | KWDSKTSTLTI |
| | | KYSLEATVDKL |
| HLA-A2902 | IVRANGTRLEY |
| | | TIADDLSQTKF |
| HLA-A6801 | LTISVNSQK |
| | | ETSEKTIVR |
| | | MTELVSKEK |
| | | EITTLKELK |
| | | TIVRANGTR |
| | | KAVEITTLK |
| | | DAKTLVSKK |
| | | KTKNLVFTK |
| | | STEEKFNEK |
| | | ATVDKLELK |
| | | LVSKKVTLK |
| | | SQTKFEIFK |
| | | YSLEATVDK |
| | | EGTLAADGK |
| | | ISVNSQKTK |
| | | LILALIACK |
| | | DIKSDGSGK |
| | | VVLSKNILK |
| | | EDAKTLVSK |
| | | DSDTTQATK |
| | | SAGTNLEGK |
| | | TVVLSKNILK |
| | | TLTISVNSQK |
| | | EATVDKLELK |
| | | TIADDLSQTK |
| | | DSAGTNLEGK |
| | | TTQATKKTGK |
| | | LAADGKTTLK |
| | | KTIVRANGTR |
| | | TISVNSQKTK |
| | | TLVSKKVTLK |
| | | SSTEEKFNEK |
| | | TLKELKNALK |
| | | LSQTKFEIFK |
| | | VTEGTVVLSK |
| | | EDAKTLVSKK |
| | | DSDTTQATKK |
| | | GLILALIACK |
| HLA- | IVRANGTRL |

| | B0702 | KVTEGTVVL |
| | | LAADGKTTL |
| | | LPGGMTELV |
| | | TVVLSKNIL |
| | | KAVEITTLKEL |
| | HLA-B0801 | TLKELKNAL |
| | | ILKSGEITVAL |
| | | YLLGIGLILAL |
| | | TLKVTEGTVVL |
| | HLA-B1501 | SQKTKNLVF |
| | | RANGTRLEY |
| | | YLLGIGLIL |
| | | KVTEGTVVL |
| | | TLKELKNAL |
| | | KAKEVLKDF |
| | | IVRANGTRL |
| | | LGIGLILAL |
| | | TLAADGKTTL |
| | | IVRANGTRLEY |
| | | YLLGIGLILAL |
| | | VQKYDSAGTNL |
| | | ILKSGEITVAL |
| | | VSKEKDKDGKY |
| | | VLKDFTLEGTL |
| | | TLKVTEGTVVL |
| | | VNSQKTKNLVF |
| | HLA-B2705 | KKYLLGIGL |
| | | VRANGTRLEY |
| | | GKWDSKTSTL |
| | | KKYLLGIGLIL |
| | HLA-B3501 | LAADGKTTL |
| | | RANGTRLEY |
| | | FTLEGTLAA |
| | | TVVLSKNIL |
| | | VALDDSDTT |
| | | YLLGIGLIL |
| | | LPGGMTELV |
| | | IADDLSQTKF |
| | | LPGGMTELVS |
| | | LKVTEGTVVL |
| | | YSLEATVDKL |
| | | YLLGIGLILAL |
| | | NSVSVDLPGGM |
| | | IVRANGTRLEY |
| | | TIADDLSQTKF |
| | | KAVEITTLKEL |
| | HLA-B4403 | KEVLKDFTL |
| | | GEITVALDD |
| | | KEKDKDGKY |
| | | KEDAKTLVS |
| | | GEITVALDDS |
| | | KEDTITVQKY |
| | | GEITVALDDSD |
| | | KEVLKDFTLEG |
| | HLA-B5101 | LPGGMTELV |
| | HLA-B5701 | TTQATKKTGKW |

Preferred Borrelia garinii fragments of FlaB capable of interacting with one or more MHC molecules are listed in Table C.

**Table C: Prediction of Borrelia garinii FlaB protein specific MHC class 2, 15-mer peptide binders for 14 MHC class 2 alleles (see figure 10) using the http://www.cbs.dtu.dk/services/NetMHCII/ database. The MHC class 2 molecules for which no binders were found are not listed.**

| BAD18055.1 FlaB protein [Borrelia garinii] Seq15 | HLA-A0101 | NQDEAIAVNIY ELAVQSGNGTY |
|---|---|---|
| | HLA-A0201 | SQASWTLRV QLTDEINRI AQAAQTAPV AIAVNIYAA SLAKIENAI AVNIYAANV AQYNQMHML TTVDANTSL SQGGVNSPV QTAPVQEGV NIYAANVANL NLNEVEKVLV VLVRMKELAV QLTDEINRIA NLFSGEGAQA IAVNIYAANV SLSGSQASWTL MLSNKSASQNV AIAVNIYAANV SQASWTLRVHV GMQPAKINTPA KVLVRMKELAV SLAKIENAIRM NLFSGEGAQAA |
| | HLA-A0301 | NQMHMLSNK GSQASWTLR TVDANTSLAK YNQMHMLSNK LSGSQASWTLR TTVDANTSLAK LSNKSASQNVR |
| | HLA-A1101 | NQMHMLSNK GSQASWTLR AVQSGNGTY TVDANTSLAK YNQMHMLSNK EVEKVLVRMK TTVDANTSLAK TTEGNLNEVEK TAEELGMQPAK TSLAKIENAIR QYNQMHMLSNK LSGSQASWTLR LSNKSASQNVR |
| | HLA-A2402 | IYAANVANL |

| | | YAANVANLF |
| | | IYAANVANLF |
| | | TYSDADRGSI |
| | | IYAANVANLFS |
| | HLA-A2902 | AVQSGNGTY |
| | | YAANVANLF |
| | | ELAVQSGNGTY |
| | | EINRIADQAQY |
| | HLA-A6801 | LAKIENAIR |
| | | EGNLNEVEK |
| | | YAANVANLF |
| | | NGTYSDADR |
| | | GSQASWTLR |
| | | NQMHMLSNK |
| | | TSKAINFIQ |
| | | NKSASQNVR |
| | | EVEKVLVRMK |
| | | SLAKIENAIR |
| | | TVDANTSLAK |
| | | NTSKAINFIQ |
| | | SGSQASWTLR |
| | | TTVDANTSLA |
| | | TTVDANTSLAK |
| | | TTEGNLNEVEK |
| | | EIEQLTDEINR |
| | | LSNKSASQNVR |
| | | TSLAKIENAIR |
| | | EINRIADQAQY |
| | | NLNEVEKVLVR |
| | | LSGSQASWTLR |
| | | TAEELGMQPAK |
| | | QYNQMHMLSNK |
| | | NIYAANVANLF |
| | | QTAPVQEGVQQ |
| | HLA-B0702 | SPVNVTTTV |
| | | QPAKINTPA |
| | | QPAPATAPS |
| | | LVRMKELAV |
| | | TPASLSGSQ |
| | | APSQGGVNS |
| | | APATAPSQG |
| | | APVQEGVQQ |
| | | TPASLSGSQA |
| | | SPVNVTTTVD |
| | | APSQGGVNSP |
| | | QPAKINTPAS |
| | | APSQGGVNSPV |
| | | QPAKINTPASL |
| | | APATAPSQGGV |
| | | SPVNVTTTVDA |
| | | TPASLSGSQAS |
| | HLA-B0801 | KVLVRMKEL |
| | | VEKVLVRMKEL |
| | HLA-B1501 | YAANVANLF |
| | | AVQSGNGTY |
| | | AQAAQTAPV |
| | | AQYNQMHML |
| | | SQGGVNSPV |

| | | SLSGSQASW |
|---|---|---|
| | | SQASWTLRV |
| | | IQIEIEQLT |
| | | LAVQSGNGTY |
| | | SQASWTLRVH |
| | | SQNVRTAEEL |
| | | VQQEGAQQPA |
| | | INRIADQAQY |
| | | AQAAQTAPVQ |
| | | MQPAKINTPA |
| | | ELAVQSGNGTY |
| | | NIYAANVANLF |
| | | NQDEAIAVNIY |
| | | SLSGSQASWTL |
| | HLA-B2705 | NRIADQAQY |
| | HLA-B3501 | YAANVANLF |
| | | QPAPATAPS |
| | | SPVNVTTTV |
| | | DEAIAVNIY |
| | | TTVDANTSL |
| | | LAVQSGNGT |
| | | QPAKINTPA |
| | | AVQSGNGTY |
| | | QASWTLRVH |
| | | IAVNIYAAN |
| | | LAVQSGNGTY |
| | | IADQAYNQM |
| | | QPAKINTPAS |
| | | LAKIENAIRM |
| | | QPAPATAPSQ |
| | | YAANVANLFS |
| | | TAEELGMQPA |
| | | EAIAVNIYAA |
| | | QAQYNQMHML |
| | | TPASLSGSQA |
| | | SPVNVTTTVD |
| | | TPASLSGSQAS |
| | | ELAVQSGNGTY |
| | | NQDEAIAVNIY |
| | | QPAKINTPASL |
| | | IAVNIYAANVA |
| | | NIYAANVANLF |
| | | SPVNVTTTVDA |
| | | EINRIADQAQY |
| | | IADQAYNQMH |
| | | APSQGGVNSPV |
| | | LAVQSGNGTYS |
| | HLA-B4403 | EELGMQPAKI |
| | | NEVEKVLVRM |
| | | DEINRIADQA |
| | | DEINRIADQAQ |
| | HLA-B5101 | SPVNVTTTV |
| | | APATAPSQGGV |
| | | APSQGGVNSPV |
| | HLA-B5701 | ASLSGSQASW |

**Choice of MHC allele for generation of MHC monomers and MHC multimers**

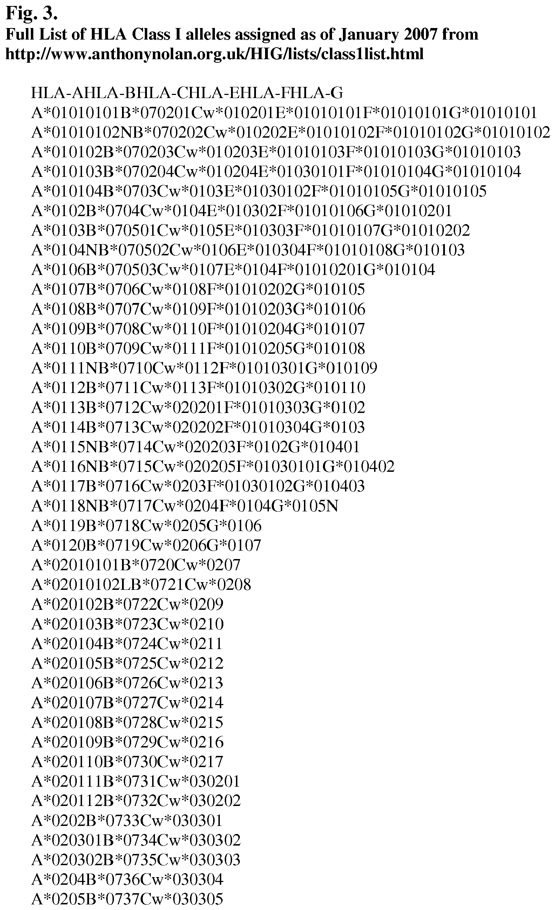

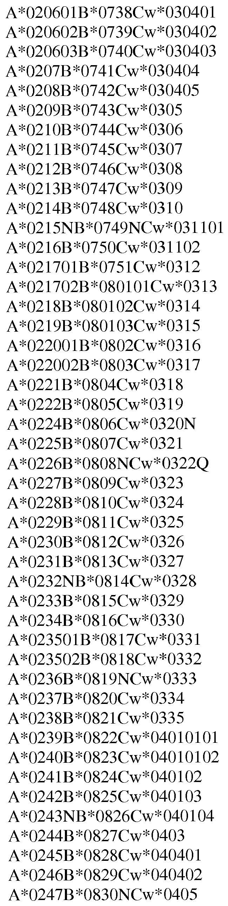

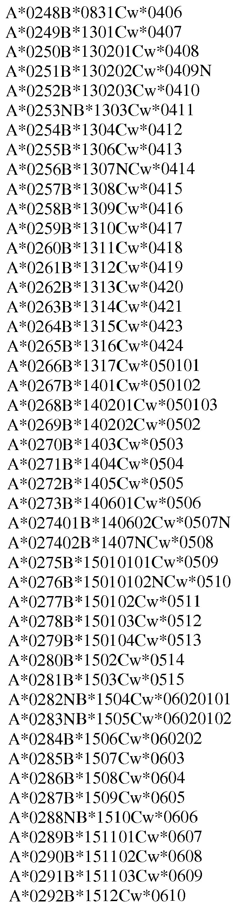

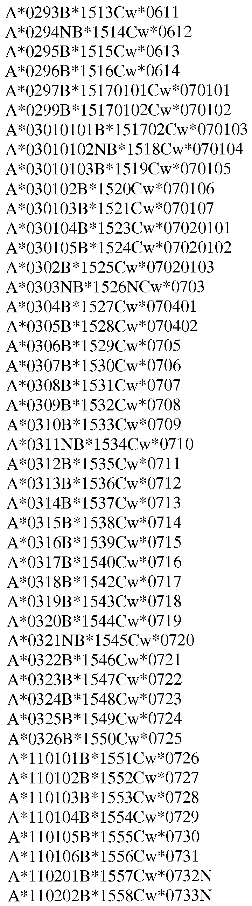

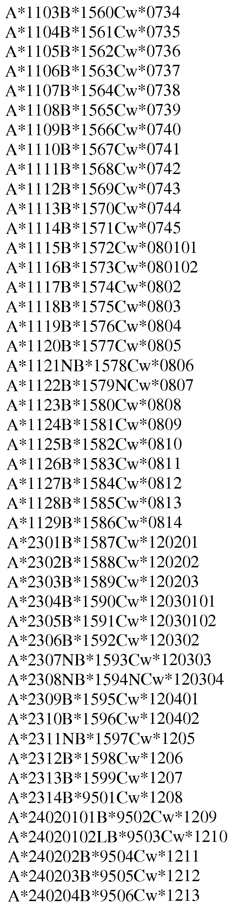

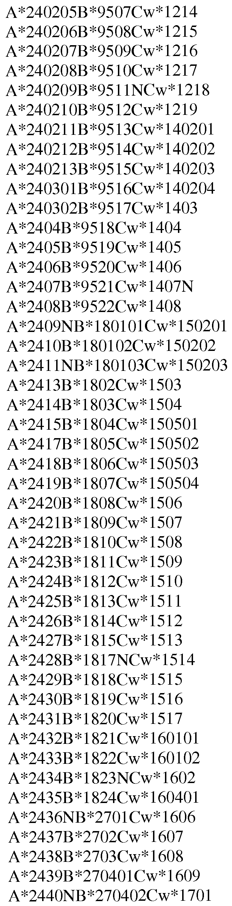

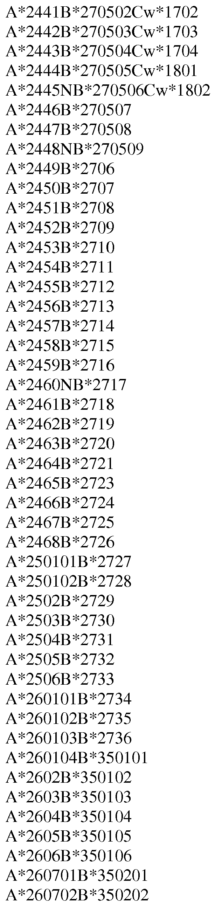

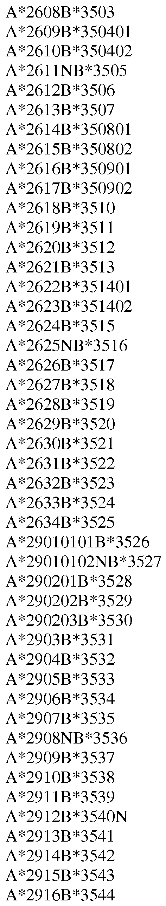

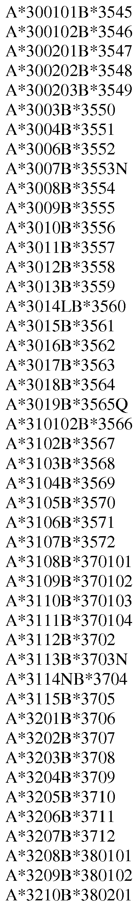

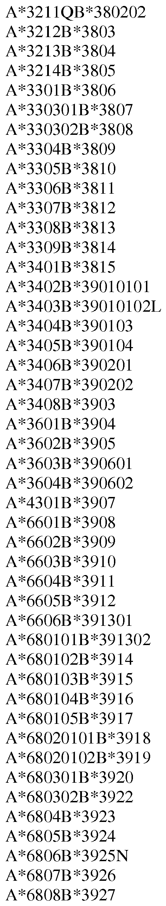

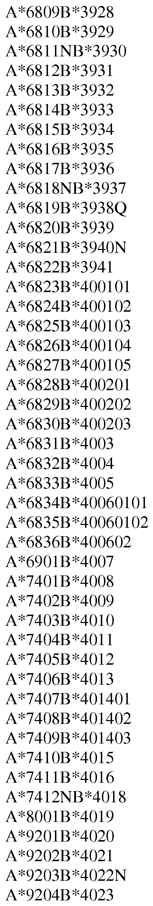

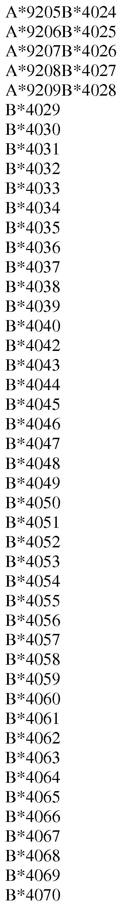

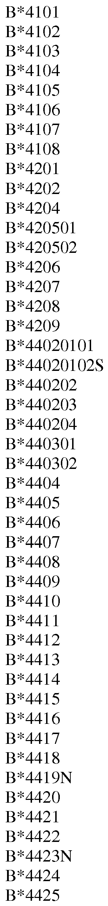

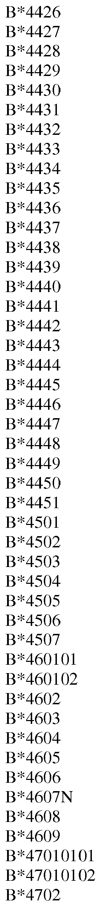

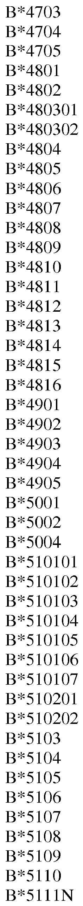

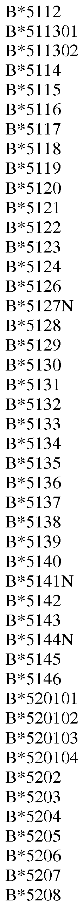

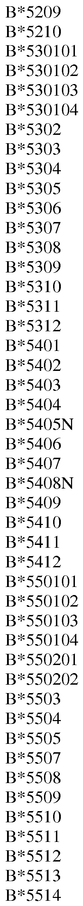

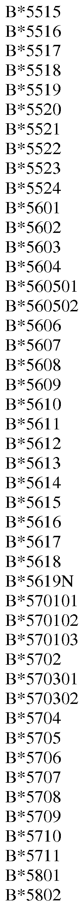

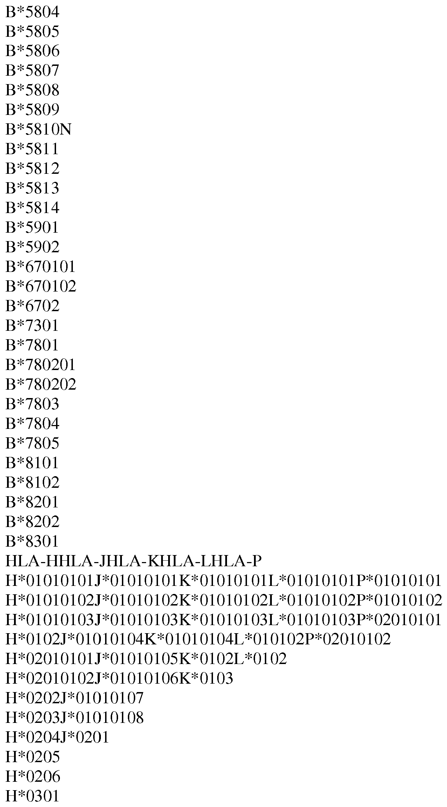

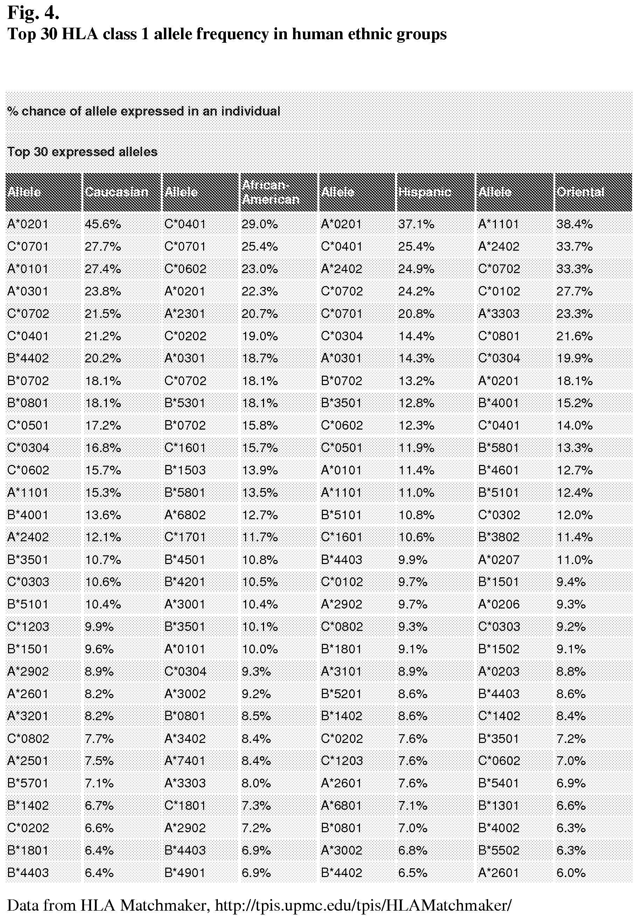

More than 600 MHC alleles (class 1 and 2) are known in humans; for many of these, the peptide binding characteristics are known. Figure 3 presents an updated list of the HLA class 1 alleles. The frequency of the different HLA alleles varies considerably, also between different ethnic groups (Figure 4). Thus it is of outmost importance to carefully select the MHC alleles that corresponds to the population that one wish to study.

**The combined choice of peptide, MHC and carrier.**

Above it has been described how to generate binding peptides, and which MHC alleles are available. Below it is further described how one may modify the binding peptides in order to increase the stability, affinity, specificity and other features of the MHC-peptide complex or the MHC multimer. In the following it is described what characteristics of binding peptides and MHC alleles are important when using the MHC-peptide complex or MHC-multimer for different purposes.

A first preferred embodiment of the present disclosure employs binding peptides of particularly high affinity for the MHC proteins. This may be done in order to increase the stability of the MHC-peptide complex. A higher affinity of the binding peptide for the MHC proteins may in some instances also result in increased rigidity of the MHC-peptide complex, which in turn often will result in higher affinity and/or specificity of the MHC-peptide complex for the T-cell receptor. A higher affinity and specificity will in turn have consequences for the immunogenicity and allergenicity, as well as possible side-effects of the MHC-peptide complex in e.g. the body.

Binding peptides of particularly high affinity for the MHC proteins may be identified by several means, including the following.

- Incubation of candidate binding peptides and MHC proteins, followed by analysis of the resulting complexes to identify those binding peptides that have most frequently been associated with MHC proteins. The binding peptides that have most frequently been associated with MHC proteins typically will represent high-affinity binding peptides. The identification of binding peptides with particularly high-affinity may involve enrichment of binding peptides, e.g. incubation of candidate peptides with immobilized MHC molecules, removal of non-binding peptides by e.g. washing, elution of binding peptides. This pool of peptides enriched for binding to the chosen MHC molecules may then be identified e.g. by mass spectrometry or HPLC and amino acid sequencing or the pool can be further enriched by another round of incubation with immobilized MHC.
- Candidate binding peptides may be compared to consensus sequences for the binding to a specific MHC allele. Thus, for a given class 1 allele, the consensus 8'mer sequence may be given by the sequence "X1-X2-X3-X4-X5-X6-X7-X8", where each of the X1-X8 amino acids can be chosen from a specific subset of amino acids, as described above.

  Those binding peptides that correlate the best with the consensus sequence are expected to have particularly high affinity for the MHC allele in question.
- Based on a large data set of affinities of binding peptides for specific MHC alleles, software programs (often involving neural networks) have been developed that allow a relatively accurate prediction of the affinity of a given candidate binding peptide for a given MHC allele. By examining candidate binding peptides using such software programs, one can identify binding peptides of expected high-affinity for the MHC molecule.

A second preferred embodiment of the present disclosure employs binding peptides with medium affinity for the MHC molecule. A medium affinity of the peptide for the MHC protein will often lead to lower physical and chemical stability of the MHC-peptide complex, which can be an advantage for certain applications. As an example, it is often desirable to administer a drug on a daily basis due to convenience. An MHC-peptide complex-based drug with high stability in the body would not allow this. In contrast a binding peptide with medium or low affinity for the MHC protein can be an advantage for such applications, since these functional MHC-peptide molecules will be cleared more rapidly from the body due to their lower stability.

For some applications where some level of cross-talk is desired, e.g. in applications where the target is a number of T cell clones that interact with a number of structurally related MHC-peptide complexes, e.g. MHC-peptide complexes containing binding peptides from different strains of a given species, a medium or low affinity of the binding peptide for the MHC protein can be an advantage. Thus, these MHC-peptide complexes are often more structurally flexible, allowing the MHC-peptide complexes to interact with several structurally related TCRs.

The affinity of a given peptide for a MHC protein, predicted by a software program or by its similarity to a consensus sequence, should only be considered a guideline to its real affinity. Moreover, the affinity can vary a lot depending on the conditions in the environment, e.g. the affinity in blood may be very different from the affinity in a biochemical assay. Further, in the context of a MHC multimer, the flexibility of the MHC-peptide complex can sometimes be an important parameter for overall avidity.

In summary, a lot of factors must be considered for the choice of binding peptides in a certain application. Some applications benefit from the use of all possible binding peptides for an antigen ("total approach"), other applications benefit from the selective choice of just a few binding peptides. Depending on the application, the affinity of the binding peptide for MHC protein is preferably high, medium, or low; the physical and/or chemical stability of the MHC-peptide complex is preferably high, medium or low; the binding peptide is preferably a very common or very rare epitope in a given population; etc.

It is obvious from the above preferred embodiments of the present disclosure that most or all of the binding peptides generated by the total approach have important applications. In other words, in order to make relevant MHC multimers that suit the different applications with regard to e.g. personalized or general targeting, or with regard to affinity, avidity, specificity, immunogenicity, stimulatory efficiency, or stability, one must be able to choose from the whole set of binding peptides generated by the total approach

**Loading of the peptide into the MHC multimer**

Loading of the peptides into the MHCmer being either MHC class 1 or class 2 can be performed in a number of ways depending on the source of the peptide and the MHC, and depending on the application. MHC class 2 molecules can in principle be loaded with peptides in similar ways as MHC class 1. However, due to complex instability the most successful approach have been to make the complexes recombinant *in toto* in eukaryotic cells from a gene construct encoding the following form β chain-flexible linker-a chain-flexible linker-antigenic peptide.

The antigenic peptide may be added to the other peptide chain(s) at different times and in different forms, as follows.

**a) Loading of antigenic peptide during MHC complex folding**

**a1) Antigenic peptide is added as a free peptide**

MHC class I molecules are most often loaded with peptide during assembly in vitro by the individual components in a folding reaction i.e. consisting of purified recombinant heavy chain $\alpha$ with the purified recombinant $\beta 2$ microglobulin and a peptide or a peptide mix.

**a2) Antigenic peptide is part of a recombinant protein construct**

Alternatively the peptide to be folded into the binding groove can be encoded together with e.g. the $\alpha$ heavy chain or fragment hereof by a gene construct having the structure, heavy chain-flexible linker- peptide. This recombinant molecule is then folded in vitro with $\beta 2$-microglobulin.

**b) Antigenic peptide replaces another antigenic peptide by an exchange reaction.**

**b1) Exchange reaction "in solution"**

Loading of desired peptide can also be made by an in vitro exchange reaction where a peptide already in place in the binding groove are being exchanged by another peptide species.

**b2) Exchange reaction "in situ"**

Peptide exchange reactions can also take place when the parent molecule is attached to other molecules, structures, surfaces, artificial or natural membranes and nanoparticles.

**b3) Aided exchange reaction.**

This method can be refined by making the parent construct with a peptide containing a meta-stable amino acid analog that is split by either light or chemically induction thereby leaving the parent structure free for access of the desired peptide in the binding groove.

**b4) Display by in vivo loading**

Loading of MHC class I and II molecules expressed on the cell surface with the desired peptides can be performed by an exchange reaction. Alternatively cells can be transfected by the peptides themselves or by the mother proteins that are then being processed leading to an *in vivo* analogous situation where the peptides are bound in the groove during the natural cause of MHC expression by the transfected cells. In the case of professional antigen presenting cells e.g. dendritic cells, macrophages, Langerhans cells, the proteins and peptides can be taken up by the cells themselves by phagocytosis and then bound to the MHC complexes the natural way and expressed on the cell surface in the correct MHC context.

**Other features of product**

In one preferred embodiment of the present disclosure the MHC multimer is between 50,000 Da and 1,000,000 Da, such as from 50,000 Da to 980,000; for example from 50,000 Da to 960,000; such as from 50,000 Da to 940,000; for example from 50,000 Da to 920,000; such as from 50,000 Da to 900,000; for example from 50,000 Da to 880,000; such as from 50,000 Da to 860,000; for example from 50,000 Da to 840,000; such as from 50,000 Da to 820,000; for example from 50,000 Da to 800,000; such as from 50,000 Da to 780,000; for example from 50,000 Da to 760,000; such as from 50,000 Da to 740,000; for example from 50,000 Da to 720,000; such as from 50,000 Da to 700,000; for example from 50,000 Da to 680,000; such as from 50,000 Da to 660,000; for example from 50,000 Da to 640,000; such as from 50,000 Da to 620,000; for example from 50,000 Da to 600,000; such as from 50,000 Da to 580,000; for example from 50,000 Da to 560,000; such as from 50,000 Da to 540,000; for example from 50,000 Da to 520,000; such as from 50,000 Da to 500,000; for example from 50,000 Da to 480,000; such as from 50,000 Da to 460,000; for example from 50,000 Da to 440,000; such as from 50,000 Da to 420,000; for example from 50,000 Da to 400,000; such as from 50,000 Da to 380,000; for example from 50,000 Da to 360,000; such as from 50,000 Da to 340,000; for example from 50,000 Da to 320,000; such as from 50,000 Da to 300,000; for example from 50,000 Da to 280,000; such as from 50,000 Da to 260,000; for example from 50,000 Da to 240,000; such as from 50,000 Da to 220,000; for example from 50,000 Da to 200,000; such as from 50,000 Da to 180,000; for example from 50,000 Da to 160,000; such as from 50,000 Da to 140,000; for example from 50,000 Da to 120,000; such as from 50,000 Da to 100,000; for example from 50,000 Da to 80,000; such as from 50,000 Da to 60,000; such as from 100,000 Da to 980,000; for example from 100,000 Da to 960,000; such as from 100,000 Da to 940,000; for example from 100,000 Da to 920,000; such as from 100,000 Da to 900,000; for example from 100,000 Da to 880,000; such as from 100,000 Da to 860,000; for example from 100,000 Da to 840,000; such as from 100,000 Da to 820,000; for example from 100,000 Da to 800,000; such as from 100,000 Da to 780,000; for example from 100,000 Da to 760,000; such as from 100,000 Da to 740,000; for example from 100,000 Da to 720,000; such as from 100,000 Da to 700,000; for example from 100,000 Da to 680,000; such as from 100,000 Da to 660,000; for example from 100,000 Da to 640,000; such as from 100,000 Da to 620,000; for example from 100,000 Da to 600,000; such as from 100,000 Da to 580,000; for example from 100,000 Da to 560,000; such as from 100,000 Da to 540,000; for example from 100,000 Da to 520,000; such as from 100,000 Da to 500,000; for example from 100,000 Da to 480,000; such as from 100,000 Da to 460,000; for example from 100,000 Da to 440,000; such as from 100,000 Da to 420,000; for example from 100,000 Da to 400,000; such as from 100,000 Da to 380,000; for example from 100,000 Da to 360,000; such as from 100,000 Da to 340,000; for example from 100,000 Da to 320,000; such as from 100,000 Da to 300,000; for example from 100,000 Da to 280,000; such as from 100,000 Da to 260,000; for example from 100,000 Da to 240,000; such as from 100,000 Da to 220,000; for example from 100,000 Da to 200,000; such as from 100,000 Da to 180,000; for example from 100,000 Da to 160,000; such as from 100,000 Da to 140,000; for example from 100,000 Da to 120,000; such as from 150,000 Da to 980,000; for example from 150,000 Da to 960,000; such as from 150,000 Da to 940,000; for example from 150,000 Da to 920,000; such as from 150,000 Da to 900,000; for example from 150,000 Da to 880,000; such as from 150,000 Da to 860,000; for example from 150,000 Da to 840,000; such as from 150,000 Da to 820,000; for example from 150,000 Da to 800,000; such as from 150,000 Da to 780,000; for example from 150,000 Da to 760,000; such as from 150,000 Da to 740,000; for example from 150,000 Da to 720,000; such as from 150,000 Da to 700,000; for example from 150,000 Da to 680,000; such as from 150,000 Da to 660,000; for example from 150,000 Da to 640,000; such as from 150,000 Da to 620,000; for example from 150,000 Da to 600,000; such as from 150,000 Da to 580,000; for example from 150,000 Da to 560,000; such as from 150,000 Da to 540,000; for example from 150,000 Da to 520,000; such as from 150,000 Da to 500,000; for example from 150,000 Da to 480,000; such as from 150,000 Da to 460,000; for example from 150,000 Da to 440,000; such as from 150,000 Da to 420,000; for example from 150,000 Da to 400,000; such as from 150,000 Da to 380,000; for example from 150,000 Da to 360,000; such as from 150,000 Da to 340,000; for example from 150,000 Da to 320,000; such as from 150,000 Da to 300,000; for example from 150,000 Da to 280,000; such as from 150,000 Da to 260,000; for example from 150,000 Da to 240,000; such as from 150,000 Da to 220,000; for example from 150,000 Da to 200,000; such as from 150,000 Da to 180,000; for example from 150,000 Da to 160,000.

In another preferred embodiment of the present disclosure the MHC multimer is between 1,000,000 Da and 3,000,000 Da, such as from 1,000,000 Da to 2,800,000; for example from 1,000,000 Da to 2,600,000; such as from 1,000,000 Da to 2,400,000; for example from 1,000,000 Da to 2,200,000; such as from 1,000,000 Da to 2,000,000; for example from 1,000,000 Da to 1,800,000; such as from 1,000,000 Da to 1,600,000; for example from 1,000,000 Da to 1,400,000.

Above it was described how to design and produce the key components of the MHC multimers, i.e. the MHC-peptide complex. In the following it is described how to generate the MHC monomer or MHC multimer products of the present disclosure.

## Number of MHC complexes pr multimer

A non-exhaustive list of possible MHC mono- and multimers illustrates the possibilities. 'n' indicates the number of MHC complexes comprised in the multimer:

| | | |
|---|---|---|
| a) n=1, | Monomers | |
| b) n=2, | Dimers, multimerization can be based on IgG scaffold, streptavidin with two MHC's, coiled-coil dimerization e.g. Fos.Jun dimerization | |
| c) n=3, | Trimers, multimerization can be based on streptavidin as scaffold with three MHC's, TNFalpha-MHC hybrids, triplex DNA-MHC konjugates or other trimer structures | |
| d) n=4, | Tetramers, multimerization can be based on streptavidin with all four binding sites occupied by MHC molecules or based on dimeric IgA | |
| e) n=5, | Pentamers, multimerization can take place around a pentameric coil-coil structure | |
| f) n=6, | Hexamers | |
| g) n=7, | Heptamers | |
| h) n=8-12, | Octa- dodecamers, multimerization can take place using Streptactin | |
| i) n=10, | Decamers, multimerization can take place using IgM | |
| j) 1<n<100, | Dextramers, as multimerization domain polymers such as polypeptide, polysaccharides and Dextrans can be used. | |
| k) 1<n<1000, | Multimerization can make use of dendritic cells (DC), antigen-presenting cells (APC), micelles, liposomes, beads, surfaces e.g. microtiterplate, tubes, microarray devices, micro-fluidic systems | |
| l) 1<n, | n in billions or trillions or higher, multimerization take place on beads, and surfaces e.g. microtiterplate, tubes, microarray devices, micro-fluidic systems | |

MHC multimers thus include MHC-dimers, MHC-trimers, MHC-tetramers, MHC-pentamers, MHC-hexamers, as well as organic molecules, cells, membranes, polymers and particles that comprise two or more MHC-peptide complexes. Example organic molecule-based multimers include functionalized cyclic structures such as benzene rings where e.g. a benzene ring is functionalized and covalently linked to e.g. three MHC complexes; example cell-based MHC multimers include dendritic cells and antigen presenting cells (APCs); example membrane-based MHC multimers include liposomes and micelles carrying MHC-peptide complexes in their membranes; example polymer-based MHC multimers include MHC-dextramers (dextran to which a number of MHC-peptide complexes are covalently or non-covalently attached) and example particles include beads or other solid supports with MHC complexes immobilized on the surface. Obviously, any kind of multimerization domain can be used, including any kind of cell, polymer, protein or other molecular structure, or particles and solid supports.

## MHC origin

Any of the three components of a MHC complex can be of any of the below mentioned origins. The list is non-exhaustive. A complete list would encompass all Chordate species. By origin is meant that the sequence is identical or highly homologous to a naturally occurring sequence of the specific species.

List of origins:

- Human
- Mouse
- Primate

  ◦ Chimpansee
  ◦ Gorilla
  ◦ Orang Utan

- Monkey

  ○ Macaques

- Porcine (Swine/Pig)
- Bovine (Cattle/Antilopes)
- Equine (Horse)
- Camelides (Camels)
- Ruminants (Deears)
- Canine (Dog)
- Feline (Cat)
- Bird

  ○ Chicken
  ○ Turkey

- Fish
- Reptiles
- Amphibians

**Generation of MHC multimers**

Different approaches to the generation of various types of MHC multimers are described in US patent 5,635,363 (Altmann et al.), patent application WO 02/072631 A2 (Winther et al.), patent application WO 99/42597, US patent 2004209295, US patent 5,635,363, and is described elsewhere in the present disclosure as well. In brief, MHC multimers can be generated by first expressing and purifying the individual protein components of the MHC protein, and then combining the MHC protein components and the peptide, to form the MHC-peptide complex. Then an appropriate number of MHC-peptide complexes are linked together by covalent or non-covalent bonds to a multimerization domain. This can be done by chemical reactions between reactive groups of the multimerization domain (e.g. vinyl sulfone functionalities on a dextran polymer) and reactive groups on the MHC protein (e.g. amino groups on the protein surface), or by non-covalent interaction between a part of the MHC protein (e.g. a biotinylated peptide component) and the multimerization domain (e.g. four binding sites for biotin on the strepavidin tetrameric protein). As an alternative, the MHC multimer can be formed by the non-covalent association of amino acid helices fused to one component of the MHC protein, to form a pentameric MHC multimer, held together by five helices in a coiled-coil structure making up the multimerization domain.

Appropriate chemical reactions for the covalent coupling of MHC and the multimerization domain include nucleophilic substitution by activation of electrophiles (e.g. acylation such as amide formation, pyrazolone formation, isoxazolone formation; alkylation; vinylation; disulfide formation), addition to carbon-hetero multiple bonds (e.g. alkene formation by reaction of phosphonates with aldehydes or ketones; arylation; alkylation of arenes/hetarenes by reaction with alkyl boronates or enolethers), nucleophilic substitution using activation of nucleophiles (e.g. condensations; alkylation of aliphatic halides or tosylates with enolethers or enamines), and cycloadditions.

Appropriate molecules, capable of providing non-covalent interactions between the multimerization domain and the MHC-peptide complex, involve the following molecule pairs and molecules: streptavidin/biotin, avidin/biotin, antibody/antigen, DNA/DNA, DNA/PNA, DNA/RNA, PNA/PNA, LNA/DNA, leucine zipper e.g. Fos/Jun, IgG dimeric protein, IgM multivalent protein, acid/base coiled-coil helices, chelate/metal ion-bound chelate, streptavidin (SA) and avidin and derivatives thereof, biotin, immunoglobulins, antibodies (monoclonal, polyclonal, and recombinant), antibody fragments and derivatives thereof, leucine zipper domain of AP-1 (jun and fos), hexa-his (metal chelate moiety), hexa-hat GST (glutathione S-transferase) glutathione affinity, Calmodulin-binding peptide (CBP), Strep-tag, Cellulose Binding Domain, Maltose Binding Protein, S-Peptide Tag, Chitin Binding Tag, Immuno-reactive Epitopes, Epitope Tags, E2Tag, HA Epitope Tag, Myc Epitope, FLAG Epitope, AU1 and AU5 Epitopes, Glu-Glu Epitope, KT3 Epitope, IRS Epitope, Btag Epitope, Protein Kinase-C Epitope, VSV Epitope, lectins that mediate binding to a diversity of compounds, including carbohydrates, lipids and proteins, e.g. Con A (*Canavalia ensiformis*) or WGA (wheat germ agglutinin) and tetranectin or Protein A or G (antibody affinity). Combinations of such binding entities are also comprised. In particular, when the MHC complex is tagged, the binding entity can be an "anti-tag". By "anti-tag" is meant an antibody binding to the tag and any other molecule capable of binding to such tag.

**Generation of components of MHC**

When employing MHC multimers for diagnostic purposes, it is preferable to use a MHC allele that corresponds to the tissue type of the person or animal to be diagnosed. Once the MHC allele has been chosen, a peptide derived from the antigenic protein may be chosen. The choice will depend on factors such as known or expected binding affinity of the MHC protein and the various possible peptide fragments that may be derived from the full sequence of the antigenic peptide, and will depend on the expected or known binding affinity and specificity of the MHC-peptide complex for the TCR. Preferably, the affinity of the peptide for the MHC molecule, and the affinity and specificity of the MHC-peptide complex for the TCR, should be high.

Similar considerations apply to the choice of MHC allele and peptide for therapeutic and vaccine purposes. In addition, for some of these applications the effect of binding the MHC multimer to the TCR is also important. Thus, in these cases the effect on the T-cell's general state must be considered, e.g. it must be decided whether the desired end result is apoptosis or proliferation of the T-cell.

Likewise, it must be decided whether stability is important. For some applications low stability may be an advantage, e.g. when a short-term effect is desired; in other instances, a long-term effect is desired and MHC multimers of high stability is desired. Stabilities of the MHC protein and of the MHC-peptide complex may be modified as described elsewhere herein.

Finally, modifications to the protein structure may be advantageous for some diagnostics purposes, because of e.g. increased stability, while in for vaccine purposes modifications to the MHC protein structure may induce undesired allergenic responses.

**Generation of protein chains of MHC**

Generation of MHC class I heavy chain and β2-Microglobulin

MHC class I heavy chain (HC) and β2-mircroglobulin (β2m) can be obtained from a variety of sources.

   **a)** Natural sources by means of purification from eukaryotic cells naturally expressing the MHC class 1 or β2m molecules in question.
   **b)** The molecules can be obtained by recombinant means e.g. using.

      a. *in vitro* translation of mRNA obtained from cells naturally expressing the MHC or β2m molecules in question
      b. by expression and purification of HC and/or β2m gene transfected cells of mammalian, yeast, bacterial or other origin. This last method will normally be the method of choise.The genetic material used for transfection/transformation can be:

         i. of natural origin isolated from cells, tissue or organisms
         ii. of synthetical origin i.e. synthetic genes identical to the natural DNA sequence or it could be modified to introduce molecular changes or to ease recombinant expression.
         The genetic material can encode all or only a fragment of β2m, all or only a fragment of MHC class 1 heavy chain. Of special interest are MHC class 1 heavy chain fragments consisting of, the complete chain minus the intramembrane domain, a chain consisting of only the extracellular α1 and α2 class 1 heavy chain domains, or any of the mentioned β2m and heavy chain fragments containing modified or added designer domain(s) or sequence(s).

Generation of MHC class 2 α- and β-chains

MHC class 2 α- and β-chains can be obtained from a variety of sources:

   **a)** Natural sources by means of purification from eukaryotic cells naturally expressing the MHC class 2 molecules in question.
   **b)** By recombinant means e.g. using:

      a. *in vitro* translation of mRNA obtained from cells naturally expressing the MHC class 2 molecules in question
      b. By purification from MHC class 2 gene transfected cells of mammalian, yeast, bacterial or other origin. This last method will normally be the method of choise.The genetic material used for transfection/transformation can be

         i. of natural origin isolated from cells, tissue or organisms
         ii. of synthetical origin i.e. synthetic genes identical to the natural DNA sequence or it could be modified to introduce molecular changes or to ease recombinant expression.

The genetic material can encode all or only a fragment of MHC class 2 α- and β-chains. Of special interest are MHC class 2 α- and β-chain_fragments consisting of, the complete α- and β-chains minus the intramembrane domains of either or both chains; and α- and β-chains consisting of only the extracellular domains of either or both, i.e α1 plus α2 and β1 plus β2 domains, respectively. The genetic material can be modified to encode the interesting MHC class 2 molecule fragments consisting of domains starting from the amino terminal in consecutive order, MHC class 2 β1 plus MHC class 2 α1 plus MHC class 1 α3 domains or in alternative order, MHC class 2 α1 plus MHC class 2 β1 plus MHC class 1 α3 domains.

Lastly, the gentic material can encode any of the above mentioned MHC class 2 α- and β-chain molecules or fragments containing modified or added designer domain(s) or sequence(s).

**c)** The MHC material may also be of exclusively synthetic origin manufactured by solid phase protein synthesis. Any of the above mentioned molecules can be made this way.

Modified MHC I or MHC II complexes

MHC I and MHC II complexes modified in any way as described above, can bind TCR. Modifications include mutations (substitutions, deletions or insertions of natural or non-natural amino acids, or any other organic molecule. The mutations are not limited to those that increase the stability of the MHC complex, and could be introduced anywhere in the MHC complex. One example of special interest is mutations introduced in the α3 subunit of MHC I heavy chain. The α3-subunit interacts with CD8 molecules on the surface of T cells. To minimize binding of MHC multimer to CD8 molecules on the surface of non-specific T cells, amino acids in α3 domain involved in the interaction with CD8 can be mutated. Such a mutation can result in altered or abrogated binding of MHC to CD8 molecules. Another example of special interest is mutations in areas of the β2-domain of MHC II molecules responsible for binding CD4 molecules.

Another embodiment of the present disclosure is chemically modified MHC complexes where the chemical modification could be introduced anywhere in the complex, e.g. a MHC complex where the peptide in the peptide-binding cleft has a dinitrophenyl group attached.

Modified MHC complexes could also be MHC I or MHC II fusion proteins where the fusion protein is not necessarily more stable than the native protein. Of special interest is MHC complexes fused with genes encoding an amino acid sequence capable of being biotinylated with a Bir A enzyme (Schatz, P. J.,(1993), Biotechnology 11(10):1138-1143). This biotinylation sequence could be fused with the COOH-terminal of β2m or the heavy chain of MHC I molecules or the COOH-terminal of either the α-chain or β-chain of MHC II. Similarly, other sequences capable of being enzymatically or chemically modified can be fused to the $NH_2$ or COOH-terminal ends of the MHC complex.

**Stabilization of empty MHC complexes and MHC-peptide complexes**

Classical MHC complexes are in nature embedded in the membrane. A preferred embodiment of the present disclosure includes multimers comprising a soluble form of MHC II or I where the transmembrane and cytosolic domains of the membrane-anchored MHC complexes are removed. The removal of the membrane-anchoring parts of the molecules can influence the stability of the MHC complexes. The stability of MHC complexes is an important parameter when generating and using MHC multimers.

MHC I complexes consist of a single membrane-anchored heavy chain that contains the complete peptide binding groove and is stable in the soluble form when complexed with β2m. The long-term stability is dependent on the binding of peptide in the peptide-binding groove. Without a peptide in the peptide binding groove the heavy chain and β2m tend to dissociate. Similarly, peptides with high affinity for binding in the peptide-binding groove will typically stabilize the soluble form of the MHC complex while peptides with low affinity for the peptide-binding groove will typically have a smaller stabilizing effect.

In contrast, MHC II complexes consist of two membrane-anchored chains of almost equal size. When not attached to the cell membrane the two chains tend to dissociate and are therefore not stable in the soluble form unless a high affinity peptide is bound in the peptide-binding groove or the two chains are held together in another way.

In nature MHC I molecules consist of a heavy chain combined with β2m, and a peptide of typically 8-11 amino acids. Herein, MHC I molecules also include molecules consisting of a heavy chain and β2m (empty MHC), or a heavy chain combined with a peptide or a truncated heavy chain comprising α1 and α2 subunits combined with a peptide, or a full-length or truncated heavy chain combined with a full-length or truncated β2m chain. These MHC I molecules can be produced in *E.coli* as recombinant proteins, purified and refolded *in vitro* (Garboczi et al., (1992), Proc. Natl. Acad. Sci. 89, 3429-33). Alternatively, insect cell systems or mammalian cell systems can be used. To produce stable MHC I complexes and thereby generate reliable MHC I multimers several strategies can be followed. Stabilization strategies for MHC I complexes are described in the following.

Stabilization strategies for MHC I complexes

◦ *Generation of covalent protein-fusions*
MHC I molecules can be stabilized by introduction of one or more linkers between the individual components of the MHC I complex. This could be a complex consisting of a heavy chain fused with β2m through a linker and a soluble peptide, a heavy chain fused to β2m through a linker, a heavy chain /β2m dimer covalently linked to a peptide through a linker to either heavy chain or β2m, and where there can or can not be a linker between the heavy chain and β2m, a heavy chain fused to a peptide through a linker, or the α1 and α2 subunits of the heavy chain fused to a peptide through a linker. In all of these example protein-fusions, each of the heavy chain, β2m and the peptide can be truncated.

The linker could be a flexible linker, e.g. made of glycine and serine and e.g. between 5-20 residues long. The linker could also be rigid with a defined structure, e.g. made of amino acids like glutamate, alanine, lysine, and leucine creating e.g. a more rigid structure.

In heavy chain-β2m fusion proteins the COOH terminus of β2m can be covalently linked to the NH$_2$ terminus of the heavy chain, or the NH$_2$ terminus of β2m can be linked to the COOH terminus of the heavy chain. The fusion-protein can also comprise a β2m domain, or a truncated β2m domain, inserted into the heavy chain, to form a fusion- protein of the form "heavy chain (first part)-β2m-heavy chain (last part)".

Likewise, the fusion-protein can comprise a heavy chain domain, or a truncated heavy chain, inserted into the β2m chain, to form a fusion-protein of the form "β2m(first part)-heavy chain-β2m(last part)".

In peptide-β2m fusion proteins the COOH terminus of the peptide is preferable linked to the NH$_2$ terminus of β2m but the peptide can also be linked to the COOH terminal of β2m via its NH$_2$ terminus. In heavy chain-peptide fusion proteins it is preferred to fuse the NH$_2$ terminus of the heavy chain to the COOH terminus of the peptide, but the fusion can also be between the COOH terminus of the heavy chain and the NH$_2$ terminus of the peptide. In heavy chain-β2m-peptide fusion proteins the NH$_2$ terminus of the heavy chain can be fused to the COOH terminus of β2m and the NH$_2$ terminus of β2m can be fused to the COOH terminus of the peptide.

◦ *Non-covalent stabilization by binding to an unnatural component*
Non-covalent binding of unnatural components to the MHC I complexes can lead to increased stability. The unnatural component can bind to both the heavy chain and the β2m, and in this way promote the assemble of the complex, and/or stabilize the formed complex. Alternatively, the unnatural component can bind to either β2m or heavy chain, and in this way stabilize the polypeptide in its correct conformation, and in this way increase the affinity of the heavy chain for β2m and/or peptide, or increase the affinity of β2m for peptide.

Here, unnatural components mean antibodies, peptides, aptamers or any other molecule with the ability to bind peptides stretches of the MHC complex. Antibody is here to be understood as truncated or full-length antibodies (of isotype IgG, IgM, IgA, IgE), Fab, scFv or bi-Fab fragments or diabodies.

An example of special interest is an antibody binding the MHC I molecule by interaction with the heavy chain as well as β2m. The antibody can be a bispecific antibody that binds with one arm to the heavy chain and the other arm to the β2m of the MHC complex. Alternatively the antibody can be monospecific, and bind at the interface between heavy chain and β2m.

Another example of special interest is an antibody binding the heavy chain but only when the heavy chain is correct folded. Correct folded is here a conformation where the MHC complex is able to bind and present peptide in such a way that a restricted T cell can recognize the MHC-peptide complex and be activated. This type of antibody can be an antibody like the one produced by the clone W6/32 (M0736 from Dako, Denmark) that recognizes a conformational epitope on intact human and some monkey MHC complexes containing β2m, heavy chain and peptide.

◦ *Generation of modified proteins or protein components*
One way to improve stability of a MHC I complex am to increase the affinity of the binding peptide for the MHC complex. This can be done by mutation/substitution of amino acids at relevant positions in the peptide, by chemical modifications of amino acids at relevant positions in the peptide or introduction by synthesis of non-natural amino acids at relevant positions in the peptide. Alternatively, mutations, chemical modifications, insertion of natural or non-natural amino acids or deletions could be introduced in the peptide binding cleft, i.e. in the binding pockets that accommodate peptide side chains responsible for anchoring the peptide to the peptide binding cleft. Moreover, reactive groups can be introduced into the antigenic peptide;

before, during or upon binding of the peptide, the reactive groups can react with amino acid residues of the peptide binding cleft, thus covalently linking the peptide to the binding pocket.

Mutations/substitutions, chemical modifications, insertion of natural or non-natural amino acids or deletions could also be introduced in the heavy chain and/or β2m at positions outside the peptide-binding cleft. By example, it has been shown that substitution of XX with YY in position nn of human β$_2$m enhance the biochemical stability of MHC Class I molecule complexes and thus may lead to more efficient antigen presentation of subdominant peptide epitopes.

A preferred embodiment of the present disclosure is removal of "unwanted cysteine residues" in the heavy chain by mutation, chemical modification, amino acid exchange or deletion. "Unwanted cysteine residues" is here to be understood as cysteines not involved in the correct folding of the final MHC I molecule. The presence of cysteine not directly involved in the formation of correctly folded MHC I molecules can lead to formation of intra molecular disulfide bridges resulting in a non correct folded MHC complex during *in vitro* refolding.

Another method for covalent stabilization of MHC I complex am to covalently attach a linker between two of the subunits of the MHC complex. This can be a linker between peptide and heavy chain or between heavy chain and beta2microglobulin.

Stabilization strategies for MHC II complexes

MHC II molecules as used herein are defined as classical MHC II molecule consisting of a $\alpha$-chain and a $\beta$-chain combined with a peptide. It could also be a molecule only consisting of $\alpha$-chain and $\beta$-chain ($\alpha/\beta$ dimer or empty MHC II), a truncated $\alpha$-chain (e.g. $\alpha 1$ domain alone) combined with full-length $\beta$-chain either empty or loaded with a peptide, a truncated $\beta$-chain (e.g. $\beta 1$ domain alone) combined with a full-length $\alpha$-chain either empty or loaded with a peptide or a truncated $\alpha$-chain combined with a truncated $\beta$-chain (e.g. $\alpha 1$ and $\beta 1$ domain) either empty or loaded with a peptide.

In contrast to MHC I molecules MHC II molecules are not easily refolded *in vitro*. Only some MHC II alleles may be produced in *E.colifollowed* by refolding *in vitro*. Therefore preferred expression systems for production of MHC II molecules are eukaryotic systems where refolding after expression of protein is not necessary. Such expression systems could be stable Drosophila cell transfectants, baculovirus infected insect cells, CHO cells or other mammalian cell lines suitable for expression of proteins.

Stabilization of soluble MHC II molecules is even more important than for MHC I molecules since both $\alpha$- and $\beta$-chain are participants in formation of the peptide binding groove and tend to dissociate when not embedded in the cell membrane.

  ∘ *Generation of covalent protein-fusions.*

MHC II complexes can be stabilized by introduction of one or more linkers between the individual components of the MHC II complex. This can be a $\alpha/\beta$ dimer with a linker between $\alpha$-chain and $\beta$-chain; a $\alpha/\beta$ dimer covalently linked to the peptide via a linker to either the $\alpha$-chain or $\beta$-chain; a $\alpha/\beta$ dimer, covalently linked by a linker between the $\alpha$-chain and $\beta$-chain, and where the dimer is covalently linked to the peptide; a $\alpha/\beta$ dimer with a linker between $\alpha$-chain and $\beta$-chain, where the dimer is combined with a peptide covalently linked to either $\alpha$-chain or $\beta$-chain.

The linker can be a flexible linker, e.g. made of glycine and serine, and is typically between 5-20 residues long, but can be shorter or longer. The linker can also be more rigid with a more defined structure, e.g. made of amino acids like glutamate, alanine, lysine, and leucine.

The peptides can be linked to the $NH_2$- or COOH-terminus of either $\alpha$-chain or $\beta$-chain. Of special interest are peptides linked to the $NH_2$-terminus of the $\beta$-chain via their COOH-terminus, since the linker required is shorter than if the peptide is linked to the COOH-terminus of the $\beta$-chain.

Linkage of $\alpha$-chain to $\beta$-chain can be via the COOH-terminus of the $\beta$-chain to the $NH_2$-terminus of the $\alpha$-chain or from the COOH-terminus of the $\alpha$-chain to the $NH_2$-terminus of the $\beta$-chain.

In a three-molecule fusion protein consisting of $\alpha$-chain, $\beta$-chain and peptide a preferred construct is where one linker connect the COOH-terminus of the $\beta$-chain with the $NH_2$-terminus of the $\alpha$-chain and another linker connects the COOH-terminal of the peptide with the $NH_2$-terminal of the $\beta$-chain. Alternatively one linker joins the COOH-terminus of the $\alpha$-chain with the $NH_2$-terminus of the $\beta$-chain and the second linker joins the $NH_2$-terminus of the peptide with the COOH-terminus of the $\beta$-chain. The three peptides of the MHC complex can further be linked as described above for the three peptides of the MHC complex, including internal fusion points for the proteins.

  ∘ *Non-covalent stabilization by binding ligand.*

Non-covalent binding of ligands to the MHC II complex can promote assembly of $\alpha$- and $\beta$-chain by bridging the two chains, or by binding to either of the $\alpha$- or $\beta$-chains, and in this way stabilize the conformation of $\alpha$ or $\beta$, that binds $\beta$ or $\alpha$, respectively, and/or that binds the peptide. Ligands here mean antibodies, peptides, aptamers or any other molecules with the ability to bind proteins.

A particular interesting example is an antibody binding the MHC complex distal to the interaction site with TCR, i.e. distal to the peptide-binding cleft. An antibody in this example can be any truncated or full length antibody of any isotype (e.g. IgG, IgM, IgA or IgE), a bi-Fab fragment or a diabody. The antibody could be bispecific with one arm binding to the $\alpha$-chain and the other arm binding to the $\beta$-chain. Alternatively the antibody could be monospecific and directed to a sequence fused to the $\alpha$-chain as well as to the $\beta$-chain.

Another example of interest is an antibody binding more central in the MHC II molecule, but still interacting with both α- and β-chain. Preferable the antibody binds a conformational epitope, thereby forcing the MHC molecule into a correct folded configuration. The antibody can be bispecific binding with one arm to the α-chain and the other arm to the β-chain. Alternatively the antibody is monospecific and binds to a surface of the complex that involves both the α- and β-chain, e.g. both the α2- and β2- domain or both the α1- and β1- domain.

The antibodies described above can be substituted with any other ligand that binds at the α-/β-chain interface, e.g. peptides and aptamers. The ligand can also bind the peptide, although, in this case it is important that the ligand does not interfere with the interaction of the peptide or binding cleft with the TCR.

◦ *Non-covalent stabilization by induced multimerization.*

In nature the anchoring of the α- and β-chains in the cell membrane stabilizes the MHC II complexes considerably. As mentioned above, a similar concept for stabilization of the α/β-dimer was employed by attachment of the MHC II chains to the Fc regions of an antibody, leading to a stable α/β-dimer, where α and β are held together by the tight interactions between two Fc domains of an antibody. Other dimerization domains can be used as well.

In one other example of special interest MHC II molecules are incorporated into artificial membrane spheres like liposomes or lipospheres. MHC II molecules can be incorporated as monomers in the membrane or as dimers like the MHC II-antibody constructs describes above. In addition to stabilization of the MHC II complex an increased avidity is obtained. The stabilization of the dimer will in most cases also stabilize the trimeric MHC-peptide complex. Induced multimerization can also be achieved by biotinylation of α- as well as β-chain and the two chains brought together by binding to streptavidin. Long flexible linkers such as extended glycine-serine tracts can be used to extend both chains, and the chains can be biotinylated at the end of such extended linkers. Then streptavidin can be used as a scaffold to bring the chains together in the presence of the peptide, while the flexible linkers still allow the chains to orientate properly.

◦ *Generation of modified proteins or protein components*

Stability of MHC II complexes can be increased by covalent modifications of the protein. One method is to increase the affinity of the peptide for the MHC complex. This can be done by exchange of the natural amino acids with other natural or non-natural amino acids at relevant positions in the peptide or by chemical modifications of amino acids at relevant positions in the peptide. Alternatively, mutations, chemical modifications, insertion of natural or non-natural amino acids or deletions can be introduced in the peptide-binding cleft.

Mutations, chemical modifications, insertion of natural or non-natural amino acids or deletions can alternatively be introduced in α- and/or β- chain at positions outside the peptide-binding cleft.

In this respect a preferred embodiment of the present disclosure is to replace the hydrophobic transmembrane regions of α-chain and β-chain by leucine zipper dimerisation domains (e.g. Fos-Jun leucine zipper; acid-base coiled-coil structure) to promote assembly of α-chain and β-chain.

Another preferred embodiment of the present disclosure is to introduce one or more cysteine residues by amino acid exchange at the COOH-terminal of both α-chain and β-chain, to create disulfide bridges between the two chains upon assembly of the MHC complex.

Another embodiment of the present disclosure is removal of "unwanted cysteine residues" in either of the chains by mutation, chemical modification, amino acid exchange or deletion. "Unwanted cysteine residues" is here to be understood as cysteines not involved in correct folding of the MHC II-peptide complex. The presence of cysteines not directly involved in the formation of correctly folded MHC II complexes can lead to formation of intra molecular disulfide bridges and incorrectly folded MHC complexes.

MHC II complexes can also be stabilized by chemically linking together the subunits and the peptide. That can be a linker between peptide and α-chain, between peptide and β-chain, between α-chain and β-chain, and combination thereof.

Such linkages can be introduced prior to folding by linking two of the complex constituents together, then folding this covalent hetero-dimer in the presence of the third constituent. An advantage of this method is that it only requires complex formation between two, rather than three species.

Another possibility is to allow all three constituents to fold, and then to introduce covalent cross-links on the folded MHC-complex, stabilizing the structure. An advantage of this method is that the two chains and the peptide will be correctly positioned relatively to each other when the cross linkages are introduced.

## Other stabilization of MHC I and/or MHC II complexes

◦ *Stabilization with soluble additives.*

The stability of proteins in aqueous solution depends on the composition of the solution. Addition of salts, detergents organic solvent, polymers ect. can influence the stability. Salts, detergents, organic solvent, polymers and any other soluble additives can be added to increase the stability of MHC complexes. Of special interest are additives that increase surface tension of the MHC molecule without binding the molecule. Examples are sucrose, mannose, glycine, betaine, alanine, glutamine, glutamic acid and ammoniumsulfate. Glycerol, mannitol and sorbitol are also included in this group even though they are able to bind polar regions.

Another group of additives of special interest are able to increase surface tension of the MHC molecule and simultaneously interact with charged groups in the protein. Examples are $MgSO_4$, NaCl, polyethylenglycol, 2-methyl-2,4-pentandiol and guanidiniumsulfate.

Correct formation of MHC complexes is dependent on binding of peptide in the peptide-binding cleft; the bound peptide appears to stabilize the complex in its correct conformation. Addition of molar excess of peptide will force the equilibrium towards correctly folded MHC-peptide complexes. Likewise is excess $\beta$2m also expected to drive the folding process in direction of correct folded MHC I complexes. Therefore peptide identical to the peptide bound in the peptide-binding cleft and/or $\beta$2m are included as stabilizing soluble additives.

Other additives of special interest for stabilization of MHC II molecules are BSA, fetal and bovine calf serum or individual protein components in serum with a protein stabilizing effect.

All of the above mentioned soluble additives can be added to any solution containing MHC complexes in order to increase the stability of the molecule. This can be during the refolding process, to the formed MHC complex, to the soluble MHC monomer, to a solution of MHC multimers comprising one or more MHC complexes or to solutions used during analysis of MHC specific T cells with MHC multimers.

Other additives of special interest for stabilization of MHC molecules are BSA, fetal and bovine calf serum or individual protein components in serum with a protein stabilizing effect.

∘ *Chemically modified MHC I and II complexes*

There are a number of amino acids that are particularly reactive towards chemical cross linkers. In the following, chemical reactions are described that are particularly preferable for the cross-linking or modification of MHC I or MHC II complexes. The amino group at the N-terminal of both chains and of the peptide, as well as amino groups of lysine side chains, are nucleophilic and can be used in a number of chemical reactions, including nucleophilic substitution by activation of electrophiles (e.g. acylation such as amide formation, pyrazolone formation, isoxazolone formation; alkylation; vinylation; disulfide formation), addition to carbon-hetero multiple bonds (e.g. alkene formation by reaction of phosphonates with aldehydes or ketones; arylation; alkylation of arenes/hetarenes by reaction with alkyl boronates or enolethers), nucleophilic substitution using activation of nucleophiles (e.g. condensations; alkylation of aliphatic halides or tosylates with enolethers or enamines), and cycloadditions. Example reagents that can be used in a reaction with the amino groups are activated carboxylic acids such as NHS-ester, tetra and pentafluoro phenolic esters, anhydrides, acid chlorides and fluorides, to form stable amide bonds. Likewise, sulphonyl chlorides can react with these amino groups to form stable sulphone-amides. Iso-Cyanates can also react with amino groups to form stable ureas, and isothiocyanates can be used to introduce thiourea linkages.

Aldehydes, such as formaldehyde and glutardialdehyde will react with amino groups to form shiff's bases, than can be further reduced to secondary amines. The guanidino group on the side chain of arginine will undergo similar reactions with the same type of reagents.

Another very useful amino acid is cysteine. The thiol on the side chain is readily alkylated by maleimides, vinyl sulphones and halides to form stable thioethers, and reaction with other thiols will give rise to disulphides.

Carboxylic acids at the C-terminal of both chains and peptide, as well as on the side chains of glutamic and aspartic acid, can also be used to introduce cross-links. They will require activation with reagents such as carbodiimides, and can then react with amino groups to give stable amides.

Thus, a large number of chemistries can be employed to form covalent cross-links. The crucial point is that the chemical reagents are bi-functional, being capable of reacting with two amino acid residues.

They can be either homo bi-functional, possessing two identical reactive moieties, such as glutardialdehyde or can be hetero bi-functional with two different reactive moieties, such as GMBS (MaleimidoButyryloxy-Succinimide ester). Alternatively, two or more reagents can be used; i.e. GMBS can be used to introduce maleimides on the $\alpha$-chain, and iminothiolane can be used to introduce thiols on the $\beta$-chain; the malemide and thiol can then form a thioether link between the two chains.

For the present disclosure some types of cross-links are particularly useful. The folded MHC-complex can be reacted with dextrans possessing a large number (up to many hundreds) of vinyl sulphones. These can react with lysine residues on both the $\alpha$ and $\beta$ chains as well as with lysine residues on the peptide protruding from the binding site, effectively cross linking the entire MHC-complex. Such cross linking is indeed a favored reaction because as the first lysine residue reacts with the dextran, the MHC-complex becomes anchored to the dextran favoring further reactions between the MHC complex and the dextran multimerization domain. Another great advantage of this dextran chemistry is that it can be combined with fluorochrome labelling; i.e. the dextran is reacted both with one or several MHC-complexes and one or more fluorescent protein such as APC.

Another valuable approach is to combine the molecular biological tools described above with chemical cross linkers.

As an example, one or more lysine residues can be inserted into the $\alpha$-chain, juxtaposed with glutamic acids in the $\beta$-chain, where after the introduced amino groups and carboxylic acids are reacted by addition of carbodiimide. Such reactions are usually not very effective in water, unless as in this case, the groups are well positioned towards reaction. This implies that one avoids excessive reactions that could otherwise end up denaturing or changing the conformation of the MHC-complex.

Likewise a dextran multimerization domain can be cross-linked with appropriately modified MHC-complexes; i.e. one or both chains of the MHC complex can be enriched with lysine residues, increasing reactivity towards the vinylsulphone dextran. The lysine's can be inserted at positions opposite the peptide binding cleft, orienting the MHC-complexes favorably for T-cell recognition.

Another valuable chemical tool is to use extended and flexible cross-linkers. An extended linker will allow the two chains to interact with little or no strain resulting from the linker that connects them, while keeping the chains in the vicinity of each other should the complex dissociate. An excess of peptide should further favor reformation of dissociated MHC-complex.

**Other TCR binding molecules**

MHC I and MHC II complexes bind to TCRs. However, other molecules also bind TCR. Some TCR-binding molecules are described in the following. MHC I and MHC II complexes binding to TCRs may be substituted with other molecules capable of binding TCR or molecules that have homology to the classical MHC molecules and therefore potentially could be TCR binding molecules. These other TCR binding or MHC like molecules include:

Non-classical MHC complexes and other MHC-like molecules

Non-classical MHC complexes include protein products of MHC Ib and MHC IIb genes. MHC Ib genes encode $\beta$2m-associated cell-surface molecules but show little polymorphism in contrast to classical MHC class I genes. Protein products of MHC class Ib genes include HLA-E, HLA-G, HLA-F, HLA-H, MIC A, MIC B, ULBP-1, ULBP-2, ULBP-3 in humans and H2-M, H2-Q, H2-T and Rae1 in mice.

Non-classical MHC II molecules (protein products of MHC IIb genes) include HLA-DM, HLA-DO in humans and H2-DM and H2-DO in mice that are involved in regulation of peptide loading into MHC II molecules.

Another MHC-like molecule of special interest is the MHC I-like molecule CD1. CD1 is similar to MHC I molecules in its organization of subunits and association with $\beta$2m but presents glycolipids and lipids instead of peptides.

Artificial molecules capable of binding specific TCRs

Of special interest are antibodies that bind TCRs. Antibodies herein include full length antibodies of isotype IgG, IgM, IgE, IgA and truncated versions of these, antibody fragments like Fab fragments and scFv. Antibodies also include antibodies of antibody fragments displayed on various supramolecular structures or solid supports, including filamentous phages, yeast, mammalian cells, fungi, artificial cells or micelles, and beads with various surface chemistries.

Peptide binding TCR

Another embodiment of the present disclosure of special interest is peptides that bind TCRs. Peptides herein include peptides composed of natural, non-natural and/or chemically modified amino acids with a length of 8-20 amino acid. The peptides could also be longer than 20 amino acids or shorter than 8 amino acids. The peptides can or can not have a defined tertiary structure.

Aptamers

Aptamers are another preferred group of TCR ligands. Aptamers are herein understood as natural nucleic acids (e.g. RNA and DNA) or unnatural nucleic acids (e.g. PNA, LNA, morpholinos) capable of binding TCR. The aptamer molecules consist of natural or modified nucleotides in various lengths.

Other TCR-binding molecules can be ankyrin repeat proteins or other repeat proteins, Avimers, or small chemical molecules, as long as they are capable of binding TCR with a dissociation constant smaller than $10^{-3}$ M.

**Verification of correctly folded MHC-peptide complexes**

Quantitative ELISA and other techniques to quantify correctly folded MHC complexes

When producing MHC multimers, it is desirable to determine the degree of correctly folded MHC.

The fraction or amount of functional and/or correctly folded MHC can be tested in a number of different ways, including:

- Measurement of correctly folded MHC in a quantitative ELISA, e.g. where the MHC bind to immobilized molecules recognizing the correctly folded complex.

- Measurement of functional MHC in an assay where the total protein concentration is measured before functional MHC is captured, by binding to e.g. immobilized TCR, and the excess, non-bound protein are measured. If the dissociation constant for the interaction is known, the amount of total and the amount of non-bound protein can be determined. From these numbers, the fraction of functional MHC complex can be determined.

- Measurement of functional MHC complex by a non-denaturing gel-shift assay, where functional MHC complexes bind to TCR (or another molecule that recognize correctly folded MHC complex), and thereby shifts the TCR to another position in the gel.

**Multimerization domain**

A number of MHC complexes associate with a multimerization domain to form a MHC multimer. The size of the multimerization domain spans a wide range, from multimerisation domains based on small organic molecule scaffolds to large multimers based on a cellular structure or solid support. The multimerization domain may thus be based on different types of carriers or scaffolds, and likewise, the attachment of MHC complexes to the multimerization domain may involve covalent or non-covalent linkers. Characteristics of different kinds of multimerization domains are described below.

Molecular weight of multimerization domain.

- In one embodiment of the present disclosure the multimerization domain(s) in the present disclosure is preferably less than 1,000 Da (small molecule scaffold). Examples include short peptides (e.g. comprising 10 amino acids), and various small molecule scaffolds (e.g. aromatic ring structures).
- In another embodiment of the present disclosure the multimerization domain(s) is preferably between 1,000 Da and 10,000 Da (small molecule scaffold, small peptides, small polymers). Examples include polycyclic structures of both aliphatic and aromatic compounds, peptides comprising e.g. 10-100 amino acids, and other polymers such as dextran, polyethylenglycol, and polyureas.
- In another embodiment of the present disclosure the multimerization domain(s) is between 10,000 Da and 100,000 Da (Small molecule scaffold, polymers e.g. dextran, streptavidin, IgG, pentamer structure). Examples include proteins and large polypeptides, small molecule scaffolds such as steroids, dextran, dimeric streptavidin, and multi-subunit proteins such as used in Pentamers.
- In another embodiment of the present disclosure the multimerization domain(s) is preferably between 100,000 Da and 1,000,000 Da (Small molecule scaffold, polymers e.g. dextran, streptavidin, IgG, pentamer structure). Typical examples include larger polymers such as dextran (used in e.g. Dextramers), and streptavidin tetramers.
- In another embodiment of the present disclosure the multimerization domain(s) is preferably larger than 1,000,000 Da (Small molecule scaffold, polymers e.g. dextran, streptavidin, IgG, pentamer structure, cells, liposomes, artificial lipid bilayers, polystyrene beads and other beads. Most examples of this size involve cells or cell-based structures such as micelles and liposomes, as well as beads and other solid supports.

As mentioned elsewhere herein multimerisation domains can comprise carrier molecules, scaffolds or combinations of the two.

Type of multimerization domain.

In principle any kind of carrier or scaffold can be used as multimerization domain, including any kind of cell, polymer, protein or other molecular structure, or particles and solid supports. Below different types and specific examples of multimerization domains are listed.

- Cell. Cells can be used as carriers. Cells can be either alive and mitotic active, alive and mitotic inactive as a result of irradiation or chemically treatment, or the cells may be dead. The MHC expression may be natural (i.e. not stimulated) or may be induced/stimulated by e.g. Inf-$\gamma$. Of special interest are natural antigen presenting cells (APCs) such as dendritic cells, macrophages, Kupfer cells, Langerhans cells, B-cells and any MHC expressing cell either naturally expressing, being transfected or being a hybridoma.
- Cell-like structures. Cell-like carriers include membrane-based structures carrying MHC-peptide complexes in their membranes such as micelles, liposomes, and other structures of membranes, and phages such as filamentous phages.
- Solid support. Solid support includes beads, particulate matters and other surfaces. A preferred embodiment of the present disclosure include beads (magnetic or non-magnetic beads) that carry electrophilic groups e.g. divinyl sulfone activated polysaccharide, polystyrene beads that have been functionalized with tosyl-activated esters, magnetic polystyrene beads functionalized with tosyl-activated esters), and where MHC complexes may be covalently immobilized to these by reaction of nucleophiles comprised within the MHC complex with the electrophiles of the beads. Beads may be made of sepharose, sephacryl, polystyrene, agarose, polysaccharide, polycarbamate or any other kind of beads that can be suspended in aqueous buffer. Another embodiment of the present disclosure includes surfaces, i.e. solid supports and particles carrying immobilized MHC complexes on the surface. Of special interest are wells of a microtiter plate or other plate formats, reagent tubes, glass slides or other supports for use in microarray analysis, tubings or channels of micro fluidic chambers or devices, Biacore chips and beads
- Molecule. Multimerization domains may also be molecules or complexes of molecules held together by non-covalent bonds. The molecules constituting the multimerization domain can be small organic molecules or large polymers, and may be flexible linear molecules or rigid, globular structures such as e.g. proteins. Different kinds of molecules used in multimerization domains are described below.

  ○ Small organic molecules. Small organic molecules here includes steroids, peptides, linear or cyclic structures, and aromatic or aliphatic structures, and many others. The prototypical small organic scaffold is a functionalized benzene ring, i.e. a benzene ring functionalized with a number of reactive groups such as amines, to which a number of MHC molecules may be covalently linked. However, the types of reactive groups constituting the linker connecting the MHC complex and the multimerization domain, as well as the type of scaffold structure, can be chosen from a long list of chemical structures. A non-comprehensive list of scaffold structures are listed below.
  Typical scaffolds include aromatic structures, benzodiazepines, hydantoins, piperazines, indoles, furans, thiazoles, steroids, diketopiperazines, morpholines, tropanes, coumarines, qinolines, pyrroles, oxazoles, amino acid precursors, cyclic or aromatic ring structures, and many others.
  Typical carriers include linear and branched polymers such as peptides, polysaccharides, nucleic acids, and many others. Multimerization domains based on small organic or polymer molecules thus include a wealth of different structures, including small compact molecules, linear structures, polymers, polypeptides, polyureas, polycarbamates, cyclic structures, natural compound derivatives, alpha-, beta-, gamma-, and omega-peptides, mono-, di- and tri-substituted peptides, L- and D-form peptides, cyclohexane- and cyclopentane-backbone modified beta-peptides, vinylogous polypeptides, glycopolypeptides, polyamides, vinylogous sulfonamide peptide, Polysulfonamide-conjugated peptide (i.e., having prosthetic groups), Polyesters, Polysaccharides such as dextran and aminodextran, polycarbamates, polycarbonates, polyureas, poly-peptidylphosphonates, Azatides, peptoids (oligo N-substituted glycines), Polyethers, ethoxyformacetal oligomers, poly-thioethers, polyethylene, glycols (PEG), polyethylenes, polydisulfides, polyarylene sulfides, Polynucleotides, PNAs, LNAs, Morpholinos, oligo pyrrolinone, polyoximes, Polyimines, Polyethyleneimine, Polyacetates, Polystyrenes, Polyacetylene, Polyvinyl, Lipids, Phospholipids, Glycolipids, polycycles, (aliphatic), polycycles (aromatic), polyheterocycles, Proteoglycan, Polysiloxanes, Polyisocyanides, Polyisocyanates, polymethacrylates, Monofunctional, Difunctional, Trifunctional and Oligofunctional open-chain hydrocarbons, Monofunctional, Difunctional, Trifunctional and Oligofunctional Nonaromat Carbocycles, Monocyclic, Bicyclic, Tricyclic and Polycyclic Hydrocarbons, Bridged Polycyclic Hydrocarbones, Monofunctional, Difunctional, Trifunctional and Oligofunctional Nonaromatic, Heterocycles, Monocyclic, Bicyclic, Tricyclic and Polycyclic Heterocycles, bridged Polycyclic Heterocycles, Monofunctional, Difunctional, Trifunctional and Oligofunctional Aromatic Carbocycles, Monocyclic, Bicyclic, Tricyclic and Polycyclic Aromatic Carbocycles, Monofunctional, Difunctional, Trifunctional and Oligofunctional Aromatic Hetero-cycles. Monocyclic, Bicyclic, Tricyclic and Polycyclic Heterocycles. Chelates, fullerenes, and any combination of the above and many others.
  ○ Biological polymers. Biological molecules here include peptides, proteins (including antibodies, coiled-coil helices, streptavidin and many others), nucleic acids such as DNA and RNA, and polysaccharides such as dextran. The biological polymers may be reacted with MHC complexes (e.g. a number of MHC complexes chemically coupled to e.g. the amino groups of a protein), or may be linked through e.g. DNA duplex formation between a carrier DNA molecule and a number of DNA oligonucleotides each coupled to a MHC complex. Another type of multimerization domain based on a biological polymer is the streptavidin-based tetramer, where a streptavidin binds up to four biotinylated MHC complexes, as described above (see Background).

∘ Self-assembling multimeric structures. Several examples of commercial MHC multimers exist where the multimer is formed through self-assembling. Thus, the Pentamers are formed through formation of a coiled-coil structure that holds together 5 MHC complexes in an apparently planar structure. In a similar way, the Streptamers are based on the Streptactin protein which oligomerizes to form a MHC multimer comprising several MHC complexes (see Background).

In the following, alternative ways to make MHC multimers based on a molecule multimerization domain are described. They involve one or more of the abovementoned types of multimerization domains.

MHC dextramers can be made by coupling MHC complexes to dextran via a streptavidin-biotin interaction. In principle, biotin-streptavdin can be replaced by any dimerization domain, where one half of the dimerization domain is coupled to the MHC-peptide complex and the other half is coupled to dextran. For example, an acidic helix (one half of a coiled-coil dimer) is coupled or fused to MHC, and a basic helix (other half of a coiled-coil dimmer) is coupled to dextran. Mixing the two results in MHC binding to dextran by forming the acid/base coiled-coil structure.

Antibodies can be used as scaffolds by using their capacity to bind to a carefully selected antigen found naturally or added as a tag to a part of the MHC molecule not involved in peptide binding. For example, IgG and IgE will be able to bind two MHC molecules, IgM having a pentameric structure will be able to bind 10 MHC molecules. The antibodies can be full-length or truncated; a standard antibody-fragment includes the Fab2 fragment.

Peptides involved in coiled-coil structures can act as scaffold by making stable dimeric, trimeric, tetrameric and pentameric interactions. Examples hereof are the Fos-Jun heterodimeric coiled coil, the E. coli homo-trimeric coiled-coil domain Lpp-56, the engineered Trp-zipper protein forming a discrete, stable, $\alpha$-helical pentamer in water at physiological pH.

Further examples of suitable scaffolds, carriers and linkers are streptavidin (SA) and avidin and derivatives thereof, biotin, immunoglobulins, antibodies (monoclonal, polyclonal, and recombinant), antibody fragments and derivatives thereof, leucine zipper domain of AP-1 (jun and fos), hexa-his (metal chelate moiety), hexa-hat GST (glutathione S-tranferase), glutathione, Calmodulin-binding peptide (CBP), Strep-tag, Cellulose Binding Domain, Maltose Binding Protein, S-Peptide Tag, Chitin Binding Tag, Immuno-reactive Epitopes, Epitope Tags, E2Tag, HA Epitope Tag, Myc Epitope, FLAG Epitope, AU1 and AU5 Epitopes, Glu-Glu Epitope, KT3 Epitope, IRS Epitope, Btag Epitope, Protein Kinase-C Epitope, VSV Epitope, lectins that mediate binding to a diversity of compounds, including carbohydrates, lipids and proteins, e.g. Con A (*Canavalia ensiformis*) or WGA (wheat germ agglutinin) and tetranectin or Protein A or G (antibody affinity). Combinations of such binding entities are also comprised. Nonlimiting examples are streptavidin-biotin and jun-fos. In particular, when the MHC molecule is tagged, the binding entity may be an "anti-tag". By "anti-tag" is meant an antibody binding to the tag, or any other molecule capable of binding to such tag.

MHC complexes can be multimerized by other means than coupling or binding to a multimerization domain. Thus, the multimerization domain may be formed during the multimerization of MHCs. One such method is to extend the bound antigenic peptide with dimerization domains. One end of the antigenic peptide is extended with dimerization domain A (e.g. acidic helix, half of a coiled-coil dimer) and the other end is extended with dimerization domain B (e.g. basic helix, other half of a coiled-coil dimer).

When MHC complexes are loaded/mixed with these extended peptides the following multimer structure will be formed: A-MHC-BA-MHC-BA-MHC-B etc. The antigenic peptides in the mixture can either be identical or a mixture of peptides with comparable extended dimerization domains. Alternatively both ends of a peptide are extended with the same dimerization domain A and another peptide (same amino acid sequence or a different amino acid sequence) is extended with dimerization domain B. When MHC and peptides are mixed the following structures are formed: A-MHC-AB-MHC-BA-MHC-AB-MHC-B etc. Multimerization of MHC complexes by extension of peptides are restricted to MHC II molecules since the peptide binding groove of MHC I molecules is typically closed in both ends thereby limiting the size of peptide that can be embedded in the groove, and therefore preventing the peptide from extending out of the groove.

Another multimerization approach applicable to both MHC I and MHC II complexes is based on extension of N- and C-terminal of the MHC complex. For example the N-terminal of the MHC complex is extended with dimerization domain A and the C-terminal is extended with dimerization domain B. When MHC complexes are incubated together they pair with each other and form multimers like: A-MHC-BA-MHC-BA-MHC-BA-MHC-B etc. Alternatively the N-terminal and the C-terminal of a MHC complex are both extended with dimerization domain A and the N-terminal and C-terminal of another preparation of MHC complex (either the same or a different MHC) are extended with dimerization domain B. When these two types of MHC complexes are incubated together multimers will be formed: A-MHC-AB-MHC-BA-MHC-AB-MHC-B etc.

In all the above-described examples the extension can be either chemically coupled to the peptide/MHC complex or introduced as extension by gene fusion.

Dimerization domain AB can be any molecule pair able to bind to each other, such as acid/base coiled-coil helices, antibody-antigen, DNA-DNA, PNA-PNA, DNA-PNA, DNA-RNA, LNA-DNA, leucine zipper e.g. Fos/Jun, streptavidin-biotin and other molecule pairs as described elsewhere herein.

**Linker molecules**

A number of MHC complexes associate with a multimerization domain to form a MHC multimer. The attachment of MHC complexes to the multimerization domain may involve covalent or non-covalent linkers, and may involve small reactive groups as well as large protein-protein interactions.

The coupling of multimerization domains and MHC complexes involve the association of an entity X (attached to or part of the multimerization domain) and an entity Y (attached to or part of the MHC complex). Thus, the linker that connects the multimerization domain and the MHC complex comprises an XY portion.

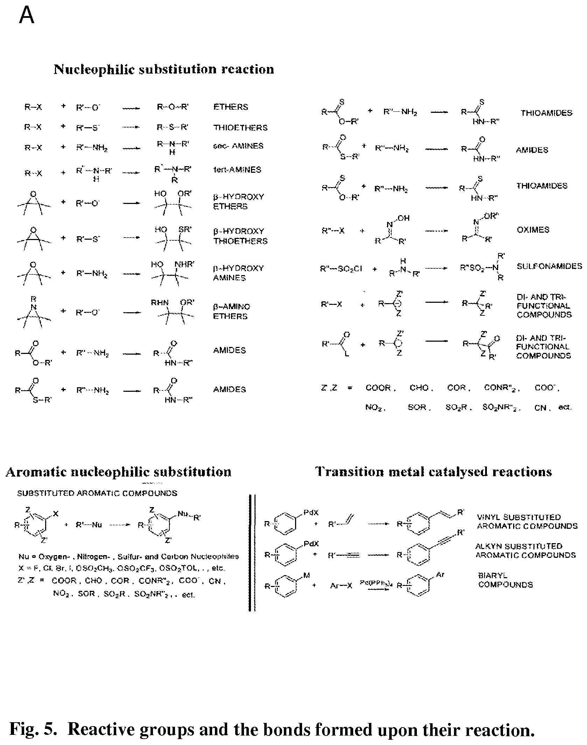

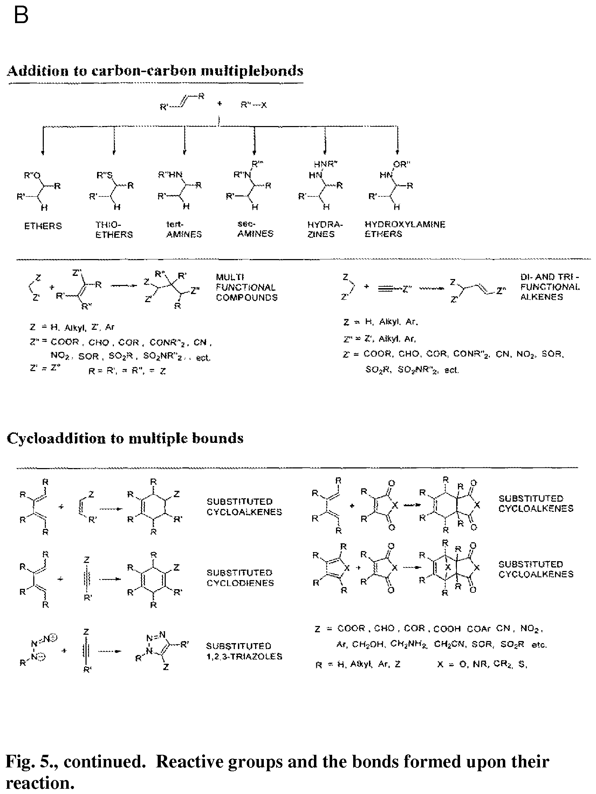

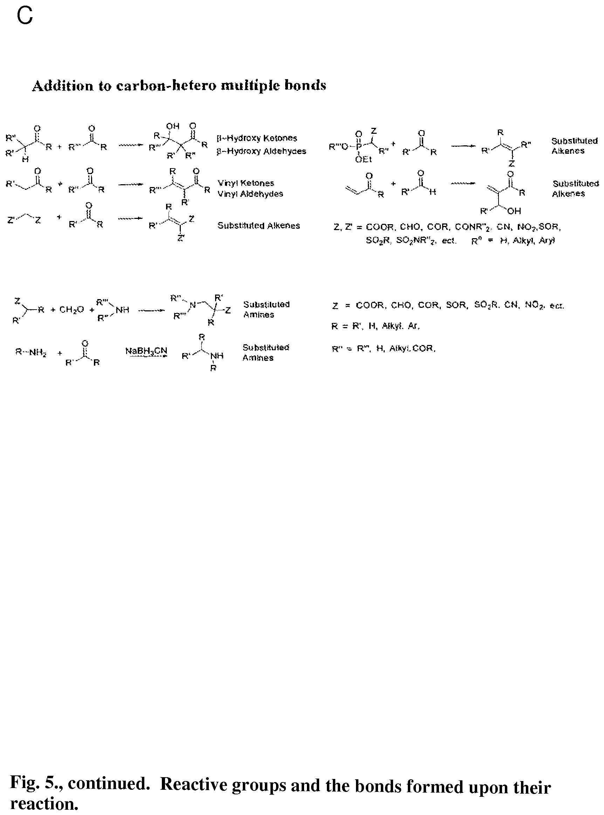

- **Covalent linker.** The XY linkage can be covalent, in which case X and Y are reactive groups. In this case, X can be a nucleophilic group (such as $-NH_2$, $-OH$, $-SH$, $-NH-NH_2$), and Y an electrophilic group (such as CHO, COOH, CO) that react to form a covalent bond XY; or Y can be a nucleophilic group and X an electrophilic group that react to form a covalent bond XY. Other possibilities exist, e.g either of the reactive groups can be a radical, capable of reacting with the other reactive group. A number of reactive groups X and Y, and the bonds that are formed upon reaction of X and Y, are shown in figure 5.

  X and Y can be reactive groups naturally comprised within the multimerization domain and/or the MHC complex, or they can be artificially added reactive groups. Thus, linkers containing reactive groups can be linked to either of the multimerization domain and MHC complex; subsequently the introduced reactive group(s) can be used to covalently link the multimerization domain and MHC complex.

  Example natural reactive groups of MHC complexes include amino acid side chains comprising $-NH_2$, $-OH$, $-SH$, and $-NH-$. Example natural reactive groups of multimerization domains include hydroxyls of polysaccharides such as dextrans, but also include amino acid side chains comprising $-NH_2$, $-OH$, $-SH$, and $-NH-$ of polypeptides, when the polypeptide is used as a multimerization domain. In some MHC multimers, one of the polypeptides of the MHC complex (i.e. the $\beta$2M, heavy chain or the antigenic peptide) is linked by a protein fusion to the multimerization domain. Thus, during the translation of the fusion protein, an acyl group (reactive group X or Y) and an amino group (reactive group Y or X) react to form an amide bond. Example MHC multimers where the bond between the multimerization domain and the MHC complex is covalent and results from reaction between natural reactive groups, include MHC-pentamers (described in US patent 2004209295) and MHC-dimers, where the linkage between multimerization domain and MHC complex is in both cases generated during the translation of the fusion protein.

  Example artificial reactive groups include reactive groups that are attached to the multimerization domain or MHC complex, through association of a linker molecule comprising the reactive group. The activation of dextran by reaction of the dextran hydroxyls with divinyl sulfone, introduces a reactive vinyl group that can react with e.g. amines of the MHC complex, to form an amine that now links the multimerization domain (the dextran polymer) and the MHC complex. An alternative activation of the dextran multimerization domain involves a multistep reaction that results in the decoration of the dextran with maleimide groups, as described in the patent Siiman et al. US 6,387,622. In this approach, the amino groups of MHC complexes are converted to $-SH$ groups, capable of reacting with the maleimide groups of the activated dextran. Thus, in the latter example, both the reactive group of the multimerization domain (the maleimide) and the reactive group of the MHC complex (the thiol) are artificially introduced.

  Sometimes activating reagents are used in order to make the reactive groups more reactive. For example, acids such as glutamate or aspartate can be converted to activated esters by addition of e.g. carbodiimid and NHS or nitrophenol, or by converting the acid moiety to a tosyl-activated ester. The activated ester reacts efficiently with a nucleophile such as $-NH_2$, $-SH$, $-OH$, etc.

  For the purpose of this disclosure, the multimerization domains (including small organic scaffold molecules, proteins, protein complexes, polymers, beads, liposomes, micelles, cells) that form a covalent bond with the MHC complexes can be divided into separate groups, depending on the nature of the reactive group that the multimerization domain contains. One group comprise multimerization domains that carry nucleophilic groups (e.g. $-NH_2$, $-OH$, $-SH$, $-CN$, $-NH-NH_2$), exemplified by polysaccharides, polypeptides containing e.g. lysine, serine, and cysteine; another group of multimerization domains carry electrophilic groups (e.g. $-COOH$, $-CHO$, $-CO$, NHS-ester, tosyl-activated ester, and other activated esters, acid-anhydrides), exemplified by polypeptides containing e.g. glutamate and aspartate, or vinyl sulfone activated dextran; yet another group of multimerization domains carry radicals or conjugated double bonds.

The multimerization domains appropriate for this disclosure thus include those that contain any of the reactive groups shown in Figure 5 or that can react with other reactive groups to form the bonds shown in Figure 5.

Likewise, MHC complexes can be divided into separate groups, depending on the nature of the reactive group comprised within the MHC complex. One group comprise MHCs that carry nucleophilic groups (e.g. $-NH_2$, $-OH$, $-SH$, $-CN$, $-NH-NH_2$), e.g. lysine, serine, and cysteine; another group of MHCs carry electrophilic groups (e.g. $-COOH$, $-CHO$, $-CO$, NHS-ester, tosyl-activated ester, and other activated esters, acid-anhydrides), exemplified by e.g. glutamate and aspartate; yet another group of MHCs carry radicals or conjugated double bonds.

The reactive groups of the MHC complex are either carried by the amino acids of the MHC-peptide complex (and may be comprised by any of the peptides of the MHC-peptide complex, including the antigenic peptide), or alternatively, the reactive group of the MHC complex has been introduced by covalent or non-covalent attachment of a molecule containing the appropriate reactive group.

Preferred reactive groups in this regard include $-CSO_2OH$, phenylchloride, $-SH$, $-SS$, aldehydes, hydroxyls, isocyanate, thiols, amines, esters, thioesters, carboxylic acids, triple bonds, double bonds, ethers, acid chlorides, phosphates, imidazoles, halogenated aromatic rings, any precursors thereof, or any protected reactive groups, and many others. Example pairs of reactive groups, and the resulting bonds formed, are shown in figure 5.

Reactions that may be employed include acylation (formation of amide, pyrazolone, isoxazolone, pyrimidine, comarine, quinolinon, phthalhydrazide, diketopiperazine, benzodiazepinone, and hydantoin), alkylation, vinylation, disulfide formation, Wittig reaction, Horner-Wittig-Emmans reaction, arylation (formation of biaryl or vinylarene), condensation reactions, cycloadditions ((2+4), (3+2)), addition to carbon-carbon multiplebonds, cycloaddition to multiple bonds, addition to carbon-hetero multiple bonds, nucleophilic aromatic substitution, transition metal catalyzed reactions, and may involve formation of ethers, thioethers, secondary amines, tertiary amines, beta-hydroxy ethers, beta-hydroxy thioethers, beta-hydroxy amines, beta-amino ethers, amides, thioamides, oximes, sulfonamides, di- and tri-functional compounds, substituted aromatic compounds, vinyl substituted aromatic compounds, alkyn substituted aromatic compounds, biaryl compounds, hydrazines, hydroxylamine ethers, substituted cycloalkenes, substituted cyclodienes, substituted 1, 2, 3 triazoles, substituted cycloalkenes, beta-hydroxy ketones, beta-hydroxy aldehydes, vinyl ketones, vinyl aldehydes, substituted alkenes, substituted alkenes, substituted amines, and many others.

MHC dextramers can be made by covalent coupling of MHC complexes to the dextran backbone, e.g. by chemical coupling of MHC complexes to dextran backbones. The MHC complexes can be coupled through either heavy chain or β2-microglobulin if the MHC complexes are MHC I or through α-chain or β-chain if the MHC complexes are MHC II. MHC complexes can be coupled as folded complexes comprising heavy chain/beta2microglobulin or α-chain/β-chain or either combination together with peptide in the peptide-binding cleft. Alternatively either of the protein chains can be coupled to dextran and then folded in vitro together with the other chain of the MHC complex not coupled to dextran and together with peptide. Direct coupling of MHC complexes to dextran multimerization domain can be via an amino group or via a sulphide group. Either group can be a natural component of the MHC complex or attached to the MHC complex chemically. Alternatively, a cysteine may be introduced into the genes of either chain of the MHC complex.

Another way to covalently link MHC complexes to dextran multimerization domains is to use the antigenic peptide as a linker between MHC and dextran. Linker containing antigenic peptide at one end is coupled to dextran. Antigenic peptide here means a peptide able to bind MHC complexes in the peptide-binding cleft. As an example, 10 or more antigenic peptides may be coupled to one dextran molecule. When MHC complexes are added to such peptide-dextran construct the MHC complexes will bind the antigenic peptides and thereby MHC-peptide complexes are displayed around the dextran multimerization domain. The antigenic peptides can be identical or different from each other. Similarly MHC complexes can be either identical or different from each other as long as they are capable of binding one or more of the peptides on the dextran multimerization domain.

- **Non-covalent linker.** The linker that connects the multimerization domain and the MHC complex comprises an XY portion. Above different kinds of covalent linkages XY were described. However, the XY linkage can also be non-covalent.

Non-covalent XY linkages can comprise natural dimerization pairs such as antigen-antibody pairs, DNA-DNA interactions, or can include natural interactions between small molecules and proteins, e.g. between biotin and streptavidin. Artificial XY examples include XY pairs such as His$_6$ tag (X) interacting with Ni-NTA (Y) and PNA-PNA interations.

Protein-protein interactions. The non-covalent linker may comprise a complex of two or more polypeptides or proteins, held together by non-covalent interactions. Example polypeptides and proteins belonging to this group include Fos/Jun, Acid/Base coiled coil structure, antibody/antigen (where the antigen is a peptide), and many others.

A preferred embodiment of the present disclosure involving non-covalent interactions between polypeptides and/or proteins are represented by the Pentamer structure described in US patent 2004209295.

Another preferred embodiment of the present disclosure involves the use of antibodies, with affinity for the surface of MHC opposite to the peptide-binding groove. Thus, an anti-MHC antibody, with its two binding site, will bind two MHC complexes and in this way generate a bivalent MHC multimer. In addition, the antibody can stabilize the MHC complex through the binding interactions. This is particularly relevant for MHC class II complexes, as these are less stable than class I MHC complexes.

Polynucleotide-polynucleotide interactions. The non-covalent linker may comprise nucleotides that interact non-covalently. Example interactions include PNA/PNA, DNA/DNA, RNA/RNA, LNA/DNA, and any other nucleic acid duplex structure, and any combination of such natural and unnatural polynucleotides such as DNA/PNA, RNA/DNA, and PNA/LNA.

Protein-small molecule interactions. The non-covalent linker may comprise a macromolecule (e.g. protein, polynucleotide) and a small molecule ligand of the macromolecule. The interaction may be natural (i.e., found in Nature, such as the Streptavidin/biotin interaction) or non-natural (e.g. His-tag peptide/Ni-NTA interaction). Example interactions include Streptavidin/biotin and anti-biotin antibody/biotin.

Combinations - non-covalent linker molecules. Other combinations of proteins, polynucleotides, small organic molecules, and other molecules, may be used to link the MHC to the multimerization domain. These other combinations include protein-DNA interactions (e.g. DNA binding protein such as the gene regulatory protein CRP interacting with its DNA recognition sequence), RNA aptamer-protein interactions (e.g. RNA aptamer specific for growth hormone interacting with growth hormone)

Synthetic molecule-synthetic molecule interaction. The non-covalent linker may comprise a complex of two or more organic molecules, held together by non-covalent interactions. Example interactions are two chelate molecules binding to the same metal ion (e.g. EDTA-$Ni^{++}$-NTA), or a short polyhistidine peptide (e.g. $His_6$) bound to NTA-$Ni^{++}$.

In another preferred embodiment of the present disclosure the multimerization domain is a bead. The bead is covalently or non-covalently coated with MHC multimers or single MHC complexes, through non-cleavable or cleavable linkers. As an example, the bead can be coated with streptavidin monomers, which in turn are associated with biotinylated MHC complexes; or the bead can be coated with streptavidin tetramers, each of which are associated with 0, 1, 2, 3, or 4 biotinylated MHC complexes; or the bead can be coated with MHC-dextramers where e.g. the reactive groups of the MHC-dextramer (e.g. the divinyl sulfone-activated dextran backbone) has reacted with nucleophilic groups on the bead, to form a covalent linkage between the dextran of the dextramer and the beads.

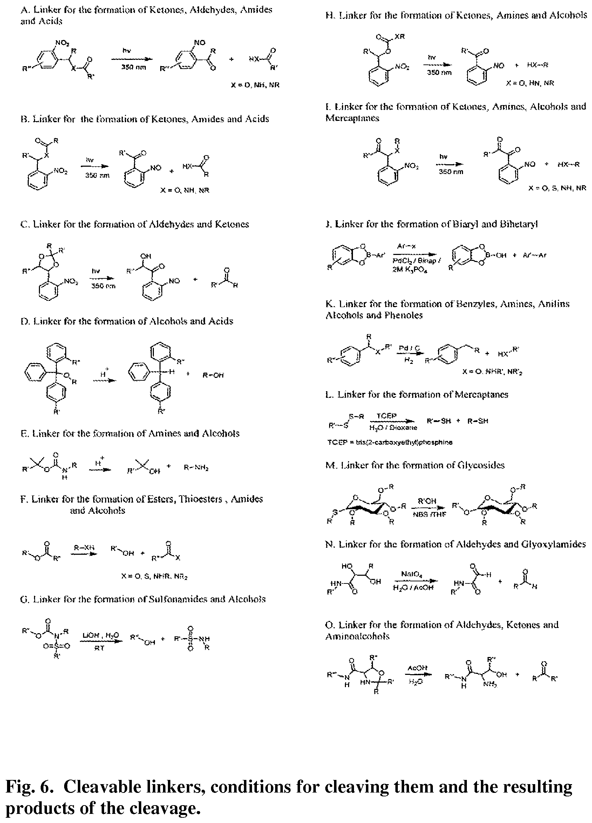

In another preferred embodiment of the present disclosure, the MHC multimers described above (e.g. where the multimerization domain is a bead) further contains a flexible or rigid, and water soluble, linker that allows for the immobilized MHC complexes to interact efficiently with cells, such as T-cells with affinity for the MHC complexes. In yet another embodiment of the present disclosure, the linker is cleavable, allowing for release of the MHC complexes from the bead. If T-cells have been immobilized, by binding to the MHC complexes, the T-cells can very gently be released by cleavage of this cleavable linker. Appropriate cleavable linkers are shown in Figure 6. Most preferably, the linker is cleaved at physiological conditions, allowing for the integrity of the isolated cells.

Further examples of linker molecules that may be employed in the present disclosure include Calmodulin-binding peptide (CBP), 6xHIS, Protein A, Protein G, biotin, Avidine, Streptavidine, Strep-tag, Cellulose Binding Domain, Maltose Binding Protein, S-Peptide Tag, Chitin Binding Tag, Immuno-reactive Epitopes, Epitope Tags, GST tagged proteins, E2Tag, HA Epitope Tag, Myc Epitope, FLAG Epitope, AU1 and AU5 Epitopes, Glu-Glu Epitope, KT3 Epitope, IRS Epitope, Btag Epitope, Protein Kinase-C Epitope, VSV Epitope.

The list of dimerization- and multimerization domains, described elsewhere in this document, define alternative non-covalent linkers between the multimerization domain and the MHC complex.

The abovementioned dimerization- and multimerization domains represent specific binding interactions. Another type of non-covalent interactions involves the non-specific adsorption of e.g. proteins onto surfaces. As an example, the non-covalent adsorption of proteins onto glass beads represents this class of XY interactions. Likewise, the interaction of MHC complexes (comprising full-length polypeptide chains, including the transmembrane portion) with the cell membrane of for example dendritic cells is an example of a non-covalent, primarily non-specific XY interaction.

In some of the abovementioned embodiments of the present disclosure, several multimerization domains (e.g. streptavidin tetramers bound to biotinylated MHC complexes) are linked to another multimerization domain (e.g. the bead). For the purpose of this disclosure we shall call both the smaller and the bigger multimerization domain, as well as the combined multimerization domain, for multimerization domain.

**Additional features of MHC multimer, antigenic peptide or antigenic polypeptide product**

Additional components may be coupled to carrier or added as individual components not coupled to carrier.

Attachment of biologically active molecules to MHC multimers

Engagement of MHC complex to the specific T cell receptor leads to a signaling cascade in the T cell. However, T-cells normally respond to a single signal stimulus by going into apoptosis. T cells needs a second signal in order to become activated and start development into a specific activation state e.g. become an active cytotoxic T cell, helper T cell or regulatory T cell.

It is to be understood that the MHC multimer of the disclosure may further comprise one or more additional substituents. The definition of the terms "one or more", "a plurality", "a", "an", and "the" also apply here. Such biologically active molecules may be attached to the construct in order to affect the characteristics of the constructs, e.g. with respect to binding properties, effects, MHC molecule specificities, solubility, stability, or detectability. For instance, spacing could be provided between the MHC complexes, one or both chromophores of a Fluorescence Resonance Energy Transfer (FRET) donor/acceptor pair could be inserted, functional groups could be attached, or groups having a biological activity could be attached.

MHC multimers can be covalently or non-covalently associated with various molecules: having adjuvant effects; being immune targets e.g. antigens; having biological activity e.g. enzymes, regulators of receptor activity, receptor ligands, immune potentiators, drugs, toxins, co-receptors, proteins and peptides in general; sugar moieties; lipid groups; nucleic acids including siRNA; nano particles; small molecules. In the following these molecules are collectively called biologically active molecules. Such molecules can be attached to the MHC multimer using the same principles as those described for attachment of MHC complexes to multimerisation domains as described elsewhere herein. In brief, attachment can be done by chemical reactions between reactive groups on the biologically active molecule and reactive groups of the multimerisation domain and/or between reactive groups on the biologically active molecule and reactive groups of the MHC-peptide complex. Alternatively, attachment is done by non-covalent interaction between part of the multimerisation domain and part of the biological active molecule or between part of the MHC-peptide complex and part of the biological active molecule. In both covalent and non-covalent attachment of the biologically molecule to the multimerisation domain a linker molecule can connect the two. The linker molecule can be covalent or non-covalent attached to both molecules. Examples of linker molecules are described elsewhere herein. Some of the MHCmer structures better allows these kind of modifications than others.

Biological active molecules can be attached repetitively aiding to recognition by and stimulation of the innate immune system via Toll or other receptors.

MHC multimers carrying one or more additional groups can be used as therapeutic or vaccine reagents.

In particular, the biologically active molecule may be selected from:

- proteins such as MHC Class I-like proteins like MIC A, MIC B, CD1d, HLA E, HLA F, HLA G, HLA H, ULBP-1, ULBP-2, and ULBP-3,

- co-stimulatory molecules such as CD2, CD3, CD4, CD5, CD8, CD9, CD27, CD28, CD30, CD69, CD134 (OX40), CD137 (4-1BB), CD147, CDw150 (SLAM), CD152 (CTLA-4), CD153 (CD30L), CD40L (CD154), NKG2D, ICOS, HVEM, HLA Class II, PD-1, Fas (CD95), FasL expressed on T and/or NK cells, CD40, CD48, CD58, CD70, CD72, B7.1 (CD80), B7.2 (CD86), B7RP-1, B7-H3, PD-L1, PD-L2, CD134L, CD137L, ICOSL, LIGHT expressed on APC and/or tumour cells,

- cell modulating molecules such as CD16, NKp30, NKp44, NKp46, NKp80, 2B4, KIR, LIR, CD94/NKG2A, CD94/NKG2C expressed on NK cells, IFN-alpha, IFN-beta, IFN-gamma, IL-1, IL-2, IL-3, IL-4, IL-6, IL-7, IL-8, IL-10, IL-11, IL-12, IL-15, CSFs (colony-stimulating factors), vitamin D3, IL-2 toxins, cyclosporin, FK-506, rapamycin, TGF-beta, clotrimazole, nitrendipine, and charybdotoxin,

- accessory molecules such as LFA-1, CD11a/18, CD54 (ICAM-1), CD106 (VCAM), and CD49a,b,c,d,e,f/CD29 (VLA-4),

- adhesion molecules such as ICAM-1, ICAM-2, GlyCAM-1, CD34, anti-LFA-1, anti-CD44, anti-beta7, chemokines, CXCR4, CCR5, anti-selectin L, anti-selectin E, and anti-selectin P,

- toxic molecules selected from toxins, enzymes, antibodies, radioisotopes, chemiluminescent substances, bioluminescent substances, polymers, metal particles, and haptens, such as cyclophosphamide, methrotrexate, Azathioprine, mizoribine, 15-deoxuspergualin, neomycin, staurosporine, genestein, herbimycin A, Pseudomonas exotoxin A, saporin, Rituxan, Ricin, gemtuzumab ozogamicin, Shiga toxin, heavy metals like inorganic and organic mercurials, and FN18-CRM9, radioisotopes such as incorporated isotopes of iodide, cobalt, selenium, tritium, and phosphor, and haptens such as DNP, and digoxiginin,

and combinations of any of the foregoing, as well as antibodies (monoclonal, polyclonal, and recombinant) to the foregoing, where relevant. Antibody derivatives or fragments thereof may also be used.

Biological active molecules as described above may also be attached to antigenic peptide products or antigenic polypeptide products using same principles for attachment.

## DESIGN AND GENERATION OF PRODUCT TO BE USED FOR IMMUNE MONITORING, DIAGNOSIS, THERAPY OR VACCINATION

The product of the present disclosure may be used for_immune monitoring, diagnosis, therapy and/or vaccination. The generation of product may follow some or all of the following general steps.

1. Design of antigenic peptides
2. Choise of MHC allele
3. Generation of product
4. Validation and optimization of product

**Production of a MHC multimer, antigenic peptide or antigenic polypeptide diagnostic or immune monitoring reagent may follow some or all of the following steps.**

1. Identify disease of interest. Most relevant diseases in this regard are infectious-, auto immune-related diseases.
2. Identify relevant protein antigen(s). This may be individual proteins, a group of proteins from a given tissue or subgroups of proteins from an organism.
3. Identify the protein sequence. Amino acid sequences can be directly found in databases or deduced from gene- or mRNA sequence e.g. using the following link http://www.ncbi.nlm.nih.gov/Genbank/index.html. If not in databases relevant proteins or genes encoding relevant proteins may be isolated and sequenced. In some cases only DNA sequences will be available without knowing which part of the sequence is protein coding. Then the DNA sequence is translated into amino acid sequence in all reading frames.
4. Choose MHC allele(s). Decide on needed MHC allele population coverage. If a broad coverage of a given population is needed (i.e. when a generally applicable reagents are sought) the most frequently expressed MHC alleles by the population of interest may be chosen e.g. using the database http://www.allelefrequencies.net/test/default1.asp or http://epitope.liai.org:8080/tools/population/iedb_input.
   In case of personalized medicine the patient is tissue typed (HLA type) and then MHC alleles may be selected according to that.
5. Run the general peptide epitope generator program described elsewhere herein on all selected amino acid sequences from step 3, thereby generating all possible epitopes of defined length (8'-, 9'-, 10'-, 11'-, 13'-, 14'-, 15' and/or 16'-mers). This procedure is particularly useful when the amino acid sequence is derived from a DNA sequence not knowing the protein encoding areas.
6. If searching for broadly applicable epitope sequences, a good alternative to step 5 is to run the "intelligent" peptide epitope prediction programs on the selected amino acid sequences of step 3 using the selected MHC alleles from step 4 e.g. using epitope prediction programs like http://www.syfpeithi.de/, http://www.cbs.dtu.dk/services/NetMHC/, and http://www.cbs.dtu.dk/services/NetMHCII/.
   This step can also be used supplementary to step 5 by running selected or all epitopes from the general peptide epitope generator program through one or more of the intelligent peptide epitope prediction programs.
7. If searching for broadly applicable epitope sequences, one may choose to select the epitopes with highest binding score, or the most likely proteolytic products of the species in question for the chosen MHC alleles and run them through the BLAST program (http://www.ncbi.nlm.nih.gov/blast/Blast.cgi) to validate the uniqueness of the peptides. If the peptide sequences are present in other species, evaluate the potential risk of disease states caused by the non-relevant species in relation to causing false positive results. If considered being a potential problem for evaluating the future analysis outcome, leave out the peptide. Preferably, choose unique peptide sequences only present in the selected protein.
8. Produce selected peptides as described elsewhere herein, e.g. by standard organic synthesis, and optionally test for binding to the desired MHC alleles by e.g *in vitro* folding, peptide exchange of already preloaded MHC complexes or another method able to test for peptide binding to MHC I or II molecules.
9. Generate desired MHC multimer by covalently or non-covalently attaching MHC-peptide complex(es) to multimerization domain, and optionally attach a fluorophore to the MHC multimer, as described elsewhere herein. Optionally, test efficacy in detecting specific T-cells using e.g. the methods described in the section "Detection".

The MHC multimer reagents may be used in a diagnostic procedure or kit for testing patient and control samples e.g. by flow cytometry, immune histochemistry, Elispot or other methods as described herein.

In some applications it is desirable to identify epitopes that covers several species and/or strains. E.g. the protein Bap A from Borrelia bacteria is very heterologous among Borrelia strains meaning that the amino acid sequence of this protein isolated from Borrelia different Borrelia isolates are varies. In such cases the amino acid sequence from several starins can be aligned e.g. using protein alignment programs (e.g. Vector NTI from Invitrogen) and a homologous sequence for all strains can be identified. This homologous sequence can be run through the general peptide epitope generator program and/or the "intelligent" peptide epitope prediction programs as described above to identify epitopes able to bind selected MHC alleles. The identified epitope may not nescessarily have 100% homology to amino acid stretches in any of the proteins selected for alignment. The selected epitopes can be used for generation of MHC multimer diagnostic, immune monitoring or therapeutic reagents.

**Production of a MHC multimer vaccine or therapeutic reagent may follow some or all of the following steps.**

    1. As step 1 - 8 above for diagnostic reagent.
    9. Select additional molecules (e.g. biologically active molecules, toxins) to attach to the MHC multimer as described elsewhere herein. The additional molecules can have different functionalities as e.g. adjuvants, specific activators, toxins etc.
    10. Test the therapeutic reagent following general guidelines
    11. Use for therapy

**PROCESSES INVOLVING MHC MULTIMERS, ANTIGENIC PEPTIDES AND/OR ANTIGENIC POLYPEPTIDES.**

The present disclosure relates to methods for detecting the presence of MHC-peptide recognising cells in a sample comprising the steps of

    (a) providing a sample suspected of comprising MHC-peptide recognising cells,
    (b) contacting the sample with a MHC multimer as defined above, and
    (c) determining any binding of the MHC multimer

Binding indicates the presence of MHC-peptide recognising cells.

or

    (a) providing a sample suspected of comprising MHC-peptide recognising cells,
    (b) contacting the sample with an antigenic peptide or antigenic polypepitde, and
    (c) determining any binding of the MHC multimer generatede as a consequence of addition of antigenic peptide or antigenic polypeptide.

Binding indicates the presence of MHC-peptide recognising cells.

Such methods are a powerful tool in diagnosing various diseases. Establishing a diagnosis is important in several ways. A diagnosis provides information about the disease, thus the patient can be offered a suitable treatment regime. Also, establishing a more specific diagnosis may give important information about a subtype of a disease for which a particular treatment will be beneficial (i.e. various subtypes of diseases may involve display of different peptides which are recognised by MHC-peptide recognising cells, and thus treatment can be targeted effectively against a particular subtype). In this way, it may also be possible to gain information about aberrant cells, which emerge through the progress of the disease or condition, or to investigate whether and how T-cell specificity is affected. The binding of the MHC multimer makes possible these options, since the binding is indicative for the presence of the MHC-peptide recognising cells in the sample, and accordingly the presence of MHC multimers displaying the peptide.

The present disclosure also relates to methods for monitoring MHC-peptide recognising cells comprising the steps of

    (a) providing a sample suspected of comprising MHC-peptide recognising cells,
    (b) contacting the sample with a MHC multimer as defined above, and
    (c) determining any binding of the MHC multimer, thereby monitoring MHC-peptide recognising cells.

Or

(a) providing a sample suspected of comprising MHC-peptide recognising cells,
(b) contacting the sample with an antigenic peptide or antigenic polypeptide as defined above, and
(c) determining any binding of the MHC multimer generated as a consequence of contacting sample with antigenic peptide or antigenic polypeptide, thereby monitoring MHC-peptide recognising cells.

Such methods are a powerful tool in monitoring the progress of a disease, e.g. to closely follow the effect of a treatment. The method can i.a. be used to manage or control the disease in a better way, to ensure the patient receives the optimum treatment regime, to adjust the treatment, to confirm remission or recurrence, and to ensure the patient is not treated with a medicament which does not cure or alleviate the disease. In this way, it may also be possible to monitor aberrant cells, which emerge through the progress of the disease or condition, or to investigate whether and how T-cell specificity is affected during treatment. The binding of the MHC multimer makes possible these options, since the binding is indicative for the presence of the MHC-peptide recognising cells in the sample, and accordingly the presence of MHC multimers displaying the peptide.

The present disclosure also relates to methods for establishing a prognosis of a disease involving MHC-peptide recognising cells comprising the steps of

(a) providing a sample suspected of comprising MHC-peptide recognising cells,
(b) contacting the sample with a MHC multimer as defined above, and
(c) determining any binding of the MHC multimer, thereby establishing a prognosis of a disease involving MHC-peptide recognising cells.

Or

(a) providing a sample suspected of comprising MHC-peptide recognising cells,
(b) contacting the sample with an antigenic peptide or antigenic polypeptide as defined above, and
(c) determining any binding of the MHC multimer generated as a consequence of contacting sample with antigenic peptide or antigenic polypeptide, thereby establishing a prognosis of a disease involving MHC-peptide recognising cells.

Such methods are a valuable tool in order to manage diseases, i.a. to ensure the patient is not treated without effect, to ensure the disease is treated in the optimum way, and to predict the chances of survival or cure. In this way, it may also be possible to gain information about aberrant cells, which emerge through the progress of the disease or condition, or to investigate whether and how T-cell specificity is affected, thereby being able to establish a prognosis. The binding of the MHC multimer makes possible these options, since the binding is indicative for the presence of the MHC-peptide recognising cells in the sample, and accordingly the presence of MHC-peptide complexs displaying the peptide.

The present disclosure also relates to methods for determining the status of a disease involving MHC-peptide recognising cells comprising the steps of

(a) providing a sample suspected of comprising MHC-peptide recognising cells,
(b) contacting the sample with a MHC multimer as defined above, and
(c) determining any binding of the MHC multimer, thereby determining the status of a disease involving MHC-peptide recognising cells.

Or

(a) providing a sample suspected of comprising MHC-peptide recognising cells,
(b) contacting the sample with an antigenic peptide or antigenic polypeptide as defined above, and
(c) determining any binding of the MHC multimer generated as a consequence of contacting sample with antigenic peptide or antigenic polypeptide, thereby establishing a prognosis of a disease involving MHC-peptide recognising cells.

Such methods are a valuable tool in managing and controlling various diseases. A disease could, e.g. change from one stage to another, and thus it is important to be able to determine the disease status. In this way, it may also be possible to gain information about aberrant cells which emerge through the progress of the disease or condition, or to investigate whether and how T-cell specificity is affected, thereby determining the status of a disease or condition.

The binding of the MHC-peptide complex makes possible these options, since the binding is indicative for the presence of the MHC-peptide recognising cells in the sample, and accordingly the presence of MHC-peptide complexs displaying the peptide.

The present disclosure also relates to methods for the diagnosis of a disease involving MHC-peptide recognising cells comprising the steps of

(a) providing a sample suspected of comprising MHC-peptide recognising cells,
(b) contacting the sample with a MHC multimer as defined above, and
(c) determining any binding of the MHC multimer, thereby diagnosing a disease involving MHC-peptide recognising cells.

Or

(a) providing a sample suspected of comprising MHC-peptide recognising cells,
(b) contacting the sample with an antigenic peptide or antigenic polypeptide as defined above, and
(c) determining any binding of the MHC multimer generated as a consequence of contacting sample with antigenic peptide or antigenic polypeptide, thereby establishing a prognosis of a disease involving MHC-peptide recognising cells.

Such diagnostic methods are a powerful tool in the diagnosis of various diseases. Establishing a diagnosis is important in several ways. A diagnosis gives information about the disease, thus the patient can be offered a suitable treatment regime. Also, establishing a more specific diagnosis may give important information about a subtype of a disease for which a particular treatment will be beneficial (i.e. various subtypes of diseases may involve display of different peptides which are recognised by MHC-peptide recognising cells, and thus treatment can be targeted effectively against a particular subtype). Valuable information may also be obtained about aberrant cells emerging through the progress of the disease or condition as well as whether and how T-cell specificity is affected. The binding of the MHC multimer makes possible these options, since the binding is indicative for the presence of the MHC-peptide recognising cells in the sample, and accordingly the presence of MHC multimers displaying the peptide.

The present disclosure also relates to methods of correlating cellular morphology with the presence of MHC-peptide recognising cells in a sample comprising the steps of

(a) providing a sample suspected of comprising MHC-peptide recognising cells,
(b) contacting the sample with a MHC multimer as defined above, and
(c) determining any binding of the MHC multimer, thereby correlating the binding of the MHC-peptide multimer with the cellular morphology.

Such methods are especially valuable as applied in the field of histochemical methods, as the binding pattern and distribution of the MHC multimers can be observed directly. In such methods, the sample is treated so as to preserve the morphology of the individual cells of the sample. The information gained is important i.a. in diagnostic procedures as sites affected can be observed directly.

The present disclosure also relates to methods for determining the effectiveness of a medicament against a disease involving MHC-peptide recognising cells comprising the steps of

(a) providing a sample from a subject receiving treatment with a medicament,
(b) contacting the sample with a MHC multimer as defined herein, and
(c) determining any binding of the MHC multimer, thereby determining the effectiveness of the medicament.

Or

(a) providing a sample from a subject receiving treatment with a medicament,
(b) contacting the sample with an antigenic peptide or antigenic polypeptide as defined herein, and
(c) determining any binding of the MHC multimer generated as a consequence of contacting sample with antigenic peptide, thereby determining the effectiveness of the medicament.

Such methods are a valuable tool in several ways. The methods may be used to determine whether a treatment is effectively combating the disease. The method may also provide information about aberrant cells which emerge through the progress of the disease or condition as well as whether and how T-cell specificity is affected, thereby providing information of the effectiveness of a medicament in question. The binding of the MHC multimer makes possible these options, since the binding is indicative for the presence of the MHC-peptide recognising cells in the sample, and accordingly the presence of MHC multimers displaying the peptide.

The present disclosure also relates to methods for manipulating MHC-peptide recognising cells populations comprising the steps of

(a) providing a sample comprising MHC-peptide recognising cells,
(b) contacting the sample with a MHC multimer immobilised onto a solid support as defined above,
(c) isolating the relevant MHC-peptide recognising cells, and
(d) expanding such cells to a clinically relevant number, with or without further manipulation.

Such ex vivo methods are a powerful tool to generate antigen-specific, long-lived human effector T-cell populations that, when re-introduced to the subject, enable killing of target cells and has a great potential for use in immunotherapy applications against various types of cancer and infectious diseases.

As used everywhere herein, the term "MHC-peptide recognising cells" are intended to mean such which are able to recognise and bind to MHC multimers. The intended meaning of "MHC multimers" is given above. Such MHC-peptide recognising cells may also be called MHC-peptide recognising cell clones, target cells, target MHC-peptide recognising cells, target MHC molecule recognising cells, MHC molecule receptors, MHC receptors, MHC peptide specific receptors, or peptide-specific cells. The term "MHC-peptide recognising cells" is intended to include all subsets of normal, abnormal and defect cells, which recognise and bind to the MHC molecule. Actually, it is the receptor on the MHC-peptide recognising cell that binds to the MHC molecule.

As described above, in diseases and various conditions, peptides are displayed by means of MHC multimers, which are recognised by the immune system, and cells targeting such MHC multimers are produced (MHC-peptide recognising cells). Thus, the presence of such MHC protein recognising cells is a direct indication of the presence of MHC multimers displaying the peptides recognised by the MHC protein recognising cells. The peptides displayed are indicative and may involved in various diseases and conditions.

For instance, such MHC-peptide recognising cells may be involved in diseases of inflammatory, auto-immune, allergic, viral, cancerous, infectious, allo- or xenogene (graft versus host and host versus graft) origin.

The MHC multimers, antigenic polypeptides and antigenic peptides of the present disclosure have numerous uses and are a valuable and powerful tools e.g. in the fields of therapy, diagnosis, prognosis, monitoring, stratification, and determining the status of diseases or conditions. Thus, the MHC multimers, antigenic polypeptides and/or antigenic peptides may be applied in the various methods involving the detection of MHC-peptide recognising cells.

Furthermore, the present disclosure relates to compositions comprising the MHC multimers, antigenic polypeptides and/or antigenic peptides in a solubilising medium. The present disclosure also relates to compositions comprising the MHC multimers, antigenic polypeptides and/or antigenic peptides immobilised onto a solid or semi-solid support.

The MHC multimers, antigenic polypeptides and/or antigenic peptides can be used in a number of applications, including analyses such as flow cytometry, immunohistochemistry (IHC), and ELISA-like analyses, and can be used for diagnostic, prognostic or therapeutic purposes including autologous cancer therapy or vaccines such as HIV vaccine or cancer vaccine.

The MHC multimers are very suitable as detection systems. Thus, the present disclosure relates to the use of the MHC multimers as defined herein as detection systems.

In another aspect the present disclosure relates to the general use of antigenic, antigenic polypeptides peptides, MHC-peptide complexes and multimers of such MHC-peptide complexes in various methods. These methods include therapeutic methods, diagnostic methods, prognostic methods, methods for determining the progress and status of a disease or condition, and methods for the stratification of a patient.

The MHC multimers, antigenic polypeptides and/or antigenic peptides of the present disclosure are also of value in testing the expected efficacy of medicaments against or for the treatment of various diseases. Thus, the present disclosure relates to methods of testing the effect of medicaments or treatments, the methods comprising detecting the binding of the MHC multimers to MHC-peptide recognising cells and establishing the effectiveness of the medicament or the treatment in question based on the specificity of the MHC-peptide recognising cells.

As mentioned above, the present disclosure also relates generally to the field of therapy. Thus, the present disclosure relates per se to the antigenic peptides, antigenic polypeptides and/or the MHC multimer as defined herein for use as medicaments, and to the antigenic peptides, antigenic polypeptides and the MHC multimers for use in in vivo and ex vivo therapy.

The present disclosure relates to therapeutic compositions comprising as active ingredients the MHC multimers, antigenic polypeptides and/or the antigenic peptides as defined herein.

An important aspect of the present disclosure is therapeutic compositions comprising as active ingredients effective amounts of MHC-peptide recognising cells obtained using the MHC multimers as defined herein to isolate relevant MHC-peptide recognising cells, and expanding such cells to a clinically relevant number.

The present disclosure further relates to methods for treating, preventing or alleviating diseases, methods for inducing anergy of cells, as well as to methods for up-regulating, down-regulating, modulating, stimulating, inhibiting, restoring, enhancing and/or otherwise manipulating immune responses.

The disclosure also relates to methods for obtaining MHC-peptide recognising cells by using the MHC multimers as described herein.

Also encompassed by the present disclosure are methods for preparing the therapeutic compositions of the disclosure.

The present disclosure is also directed to generating MHC multimers for detecting and analysing receptors on MHC-peptide recognising cells, such as epitope specific T-cell clones or other immune competent effector cells.

It is a further object of the present disclosure to provide new and powerful strategies for the development of curative vaccines. This in turn will improve the possibilities for directed and efficient immune manipulations against diseases caused by tumour genesis or infection by pathogenic agent like viruses and bacteria. HIV is an important example. The ability to generate and optionally attach recombinant MHC multimers to multimerization domains, such as scaffolds and/or carrier molecules, will enable the development of a novel analytical and therapeutical tool for monitoring immune responses and contribute to a rational platform for novel therapy and "vaccine" applications.

Therapeutic compositions (e.g. "therapeutical vaccines") that stimulate specific T-cell proliferation by peptide-specific stimulation is indeed a possibility within the present disclosure. Thus, quantitative analysis and ligand-based detection of specific T-cells that proliferate by the peptide specific stimulation should be performed simultaneously to monitoring the generated response.

## Application of MHC multimer, antigenic peptides and antigenic polypeptides in immune monitoring, diagnostics, therapy, vaccine

MHC multimers, antigenic polypeptides and antigenic peptides as described herein can be used to identify and isolate specific T cells in a wide array of applications. In principle all kind of samples possessing T cells can be analyzed using MHC multimers, antigenic peptides and/or antigenic polypeptides creating one or more MHC multimers in sample.

MHC multimers detect antigen-specific T cells of the various T cell subsets. T cells are pivotal for mounting an adaptive immune response. It is therefore of importance to be able to measure the number of specific T cells when performing a monitoring of a given immune response. Typically, the adaptive immune response is monitored by measuring the specific antibody response, which is only one of the effector arms of the immune system. This can lead to miss-interpretation of the actual clinical immune status.

In many cases intruders of the organism can hide away inside the cells, which can not provoke a humoral response. In other cases, e.g. in the case of certain viruses the intruder mutates fast, particularly in the genes encoding the proteins that are targets for the humoral response. Examples include the influenza and HIV viruses. The high rate of mutagenesis renders the humoral response unable to cope with the infection. In these cases the immune system relies on the cellular immune response. When developing vaccines against such targets one needs to provoke the cellular response in order to get an efficient vaccine.

MHC multimers, antigenic peptides and/or antigenic polypeptides compricing antigenic peptides can be used for monitoring immune responses elicited by vaccines. One preferred embodiment of the present disclosure is monitoring the effect of vaccines against infectious disease, e.g. vaccines against Lyme Borreliosis. Lyme Borreliosis is a dangerous, multisystem and multiorgan disease caused by infection with the bacterial spirochete Borrelia Burgdorferi senso lato and therefore the possibilities of prophylaxis are of great importance. Many Borrelia vaccines aim at eliciting an antibody response, but since the Borrelia bacteria have proven to be able to go inside cells a vaccine eliciting a cellular cytotoxic response is desirable. MHC multimers can be used to monitor the effectiveness of such a vaccine, where MHC multimers are any MHC multimer that can be added to a sample or one or more MHC multimers generated in sample by addition of antigenic peptide or antigenic polypeptide.

MHC multimers, antigenic peptides and/or antigenic polypeptides themself can also be valuable as a Borrelia vaccine in order to elicit a cellular immune response.

In one embodiment of the present disclosure MHC multimers having many MHC molecules attached to the multimerisation domain are used as vaccine. Such MHC multimers are able to bind several TCR simultaneously thereby crosslinking the receptors resulting in activation of the T cell in question. Example MHC multimers usefull as vaccine are MHC dextramers where many MHC molecules are coupled to dextran. In principle any MHC multimer as described elsewhere herein that are able to bind several TCR's simultaneously can be used.

In another embodiment of the present disclosure antigenic peptides and/or antigenic polypeptides are used as a vaccine eliciting a T cell responses directed against the peptide(s). Following administration of such vaccine antigenic peptides and/or antigenic polypeptides are taken up by antigen presenting cells, processed inside cell and displayed as MHC-peptide complexes on the surface of the antigen presenting cells thereby generating cell-based MHC multimers in sample. Such antigen presenting cells displaying antigenic peptide by MHC molecules may then bind TCR on antigen specific T cells and elicit a specific T cell immune response against the antigenic peptides.

In a Borrelia vaccine the antigenic peptides used as vaccine themselves, comprised in antigenic polypeptides or bound in the peptide binding cleft of MHC molecules in MHC multimers are derived from antigenic borrelia proteins. In vaccines against other infectious agents peptides derived from antigenic proteins of the relevant pathogen are used. To further enhance the MHC-peptide specific stimulation of the T cell bound, T cell stimulatory molecules can be coupled to the multimerisation domain together with MHC or may be added as soluble adjuvant together with the MHC multimer, antigenic peptide and/or antigenic polypeptide. Example T cell stimulatory molecules include but are not limited to IL-2, CD80 (B7.1), CD86 (B7.2), anti-CD28 antibody, CD40, CD37ligand (4-1BBL), IL-6, IL-15,IL-21, IFN-$\gamma$, IFN-$\alpha$, IFN-$\beta$, CD27 ligand, CD30 ligand, IL-23, IL-1$\alpha$ and IL-1$\beta$.

One or more T cell stimulatory molecules may be added together with or coupled to MHC multimer, antigenic peptide and/or antigenic polypeptide. Likewise other adjuvants or molecules enhancing or otherwise affecting the cellular, humoral or innate immune response may be coupled to or added together with the MHC multimer, antigenic peptide and/or antigenic polypeptide vaccine.

The number of antigen-specific cytotoxic T cells can be used as surrogate markers for the overall wellness of the immune system. The immune system can be compromised severely by natural causes such as HIV infections or big traumas or by immuno suppressive therapy in relation to transplantation. The efficacy of an anti HIV treatment can be evaluated by studying the number of common antigen-specific cytotoxic T cells, specific against for example Cytomegalovirus (CMV) and Epstein- Barr virus. In this case the measured T cells can be conceived as surrogate markers. The treatment can then be corrected accordingly and a prognosis can be made.

A similar situation is found for patients undergoing transplantation as they are severely immune compromised due to pharmaceutical immune suppression to avoid organ rejection. The suppression can lead to outbreak of opportunistic infections caused by reactivation of otherwise dormant viruses residing in the transplanted patients or the grafts. This can be the case for CMV and EBV viruses. Therefore measurement of the number of virus specific T cells can be used to give a prognosis for the outcome of the transplantation and adjustment of the immune suppressive treatment. Similarly, the BK virus has been implied as a causative reagent for kidney rejection. Therefore measurement of BK-virus specific T cells can have prognostic value. Measurement of borrelia bacteria specific T cells or T cells specific for other latent bacterial infcetions can also have a prognostic value.

MHC multimers, antigenic peptide and/or antigenic polypeptide can be of importance in diagnosis of infections caused by bacteria, virus and parasites that hide away inside cells. Serum titers can be very low and direct measurement of the disease-causing organisms by PCR can be very difficult because the host cells are not identified or are inaccessible. Other clinical symptoms of a chronical infection can be unrecognizable in an otherwise healthy individuals, even though such persons still are disease-carriers and at risk of becoming spontaneously ill if being compromised by other diseases or stress.

MHC multimers, antigenic peptide and/or antigenic polypeptide can be of importance in diagnosis of infections caused by bacteria, virus and parasites especially those hiding inside cells. Clinical symptoms of a chronic infection can be unrecognizable in otherwise healthy individuals, even though such persons still are disease-carriers and at risk of becoming spontaneously ill if being compromised by other diseases or stress.

In some acute infections the clinical symptoms are similar to clinical symptoms of completely unrelated diseases and therefore diagnosis of the disease is difficult based on clinical symptoms alone. Many infectious agents can be detected directly eg by measurement in serum. However, serum titers of the infectious agent can for other infectious agents be very low and therefore hard to measure. For intracellular pathogens direct measurement of the disease-causing organisms by e.g. PCR can be very difficult because the host cells are not identified or are inaccessible. Instead of detecting the infectious agent directly the immune response elicited by the infections agent may be measured.

Infections caused by extracellular bacteria and parasites often mediate a humoral immune response that can be monitored by measuring the specific antibody response. An alternative to measure antibodies could be measurement of CD4+ T cells which are important for establishing a humoral response.

Likewise infections caused by virus, intracellular bacteria or other infectious agents able to go inside cells can be detected by measurement of CD8+ T cells specific for the infectious agent.

An example of an infectious disease where MHC multimers, antigenic peptide and/or antigenic polypeptide could be usefull is in diagnosis of Borreliosis also called Lyme disease caused by Borrelia bacteria. Borrelia bacteria are very difficult to detect directly due to low serum titers and their ability to stay inside cells of unknown origin. Clinical symptoms caused by infection with borrelia bacteria depend on the type of bacteria and are similar to clinical symptoms of other diseases. Borrelia specific CD4+ and CD8+ T cells can be measured in serum, cerebrospinal fluid and/or joint fluid from infected individuals. The presence of a Borrelia infection can be measured using MHC multimers able to detect Borrelia specific T cells in liquid samples, preferably blood, from patients suspected to have borreliosis. Such MHC multimers may be added directly to sample or alternatively antigenic peptide and/or antigenic polypeptide may be added to sample and then cell-based MHC multimer generated in sample as described elsewhere herein.

Other bacterial infections where MHC multimers could be usefull for diagnosis is infections caused by intracellular bacteria like Brucella, Bartonella, Mycobacterium, Listeria, Rickettsia, Chlamydia and Salmonella.

MHC multimers can in principle be applied to diagnosis of any infection caused by an infectious agent eliciting a cellular immune response by measurement of antigen-specific T cells or changes in the amount antigen-specific T cells in the circulation.

Antigen-specific T helper cells and regulatory T cells have been implicated in the development of autoimmune disorders. In most cases the timing of events leading to autoimmune disease is unknown and the exact role of the immune cells not clear. Use of MHC multimers to study these diseases will lead to greater understanding of the disease-causing scenario and make provisions for development of therapies and vaccines for these diseases.

Therapeutic use of MHC multimers, antigenic peptide and/or antigenic polypeptide is possible, either directly or as part of therapeutic vaccines. In therapies involving T cells, e.g. treatment with *in vitro* amplified antigen-specific effector T cells, the T cells often do not home effectively to the correct target sites but ends up in undesired parts of the body. If the molecules responsible for interaction with the correct homing receptor can be identified these can be added to the MHC multimer making a dual, triple or multiple molecular structure that are able to aid the antigen-specific T cells home to the correct target, as the MHC multimer will bind to the specific T cell and the additional molecules will mediate binding to the target cells.

In a preferable embodiment of the present disclosure, MHC multimers bound to other functional molecules are employed to directly block, regulate or kill the targeted cells.

In a preferable embodiment of the present disclosure, MHC multimers bound to other functional molecules are employed to directly block, regulate or kill these cells.

In another aspect of the present disclosure modulation of regulatory T cells could be part of a treatment. In diseases where the function of regulatory T cells is understood it may be possible to directly block, regulate or kill these regulatory cells by means of MHC multimers that besides MHC-peptide complexes also features other functional molecules. The MHC multimers specifically recognize the target regulatory T cells and direct the action of the other functional molecules to this target T cell.

### Diseases

MHC multimers, antigenic peptides and/or antigenic polypeptides can be used in immune monitoring, diagnostics, prognostics, therapy and vaccines for many different diseases, including but not limited to the diseases listed in the following.

*a)* **Infectious diseases caused by virus** such as,
Adenovirus (subgropus A-F), BK-virus, CMV (Cytomegalo virus, HHV-5), EBV (Epstein Barr Virus, HHV-4), HBV (Hepatitis B Virus), HCV (Hepatitis C virus), HHV-6a and b (Human Herpes Virus-6a and b), HHV-7, HHV-8, HSV-1 (Herpes simplex virus-1, HHV-1), HSV-2 (HHV-2), JC-virus, SV-40 (Simian virus 40), VZV (Varizella-Zoster-Virus, HHV-3), Parvovirus B19, Haemophilus influenza, HIV-1 (Human immunodeficiency Virus-1), HTLV-1 (Human T-lymphotrophic virus-1), HPV (Human Papillomavirus giving rise to clinical manifestions such as Hepatitis, AIDS, Measles, Pox, Chicken pox, Rubella, Herpes and others.

*b)* **Infectious diseases** *caused by bacteria such as,*
Gram positive bacteria, gram negative bacteria, intracellular bacterium, extracellular bacterium, Mycobacterium tuberculosis, Mycobacterium bovis, Mycobacterium avium subsp. Paratuberculosis, Borrelia burgdorferi, Borrelia Garinii, Borrelia Afzelii, other spirochetes, Helicobacter pylori, Streptococcus pneumoniae, Listeria monocytogenes, Histoplasma capsulatum, Bartonella henselae, Bartonella quintana giving rise to clinical manifestations such as Tuberculosis, Pneumonia, Stomach ulcers, Paratuberculosis and others.

*c)* **Infectious diseases** *caused by fungus such as,*
Aspergillus fumigatus, Candida albicans, Cryptococcus neoformans, Pneumocystis carinii giving rise to clinical manifestations such as skin-, nail-, and mucosal infections, Meningitis, Sepsis and others.

*d)* **Parasitic diseases** caused by parasites such as,
Plasmodium falciparum, Plasmodium vivax, Plasmodium malariae, Schistosoma mansoni, Schistosoma japonicum, Schistosoma haematobium, Trypanosoma cruzi, Trypanosoma rhodesiense, Trypanosoma gambiense, Leishmania donovani, and Leishmania tropica.

*e)* **Allergic diseases** caused by allergens such as,
Birch, Hazel, Elm, Ragweed, Wormwood, Grass, Mould, Dust Mite giving rise to clinical manifestations such as Asthma.

**f) Transplantation-related diseases** caused by
reactions to minor histocompatibility antigens such as HA-1, HA-8, USP9Y, SMCY, TPR-protein, HB-1Y and other antigens in relation to, Graft-versus-host-related disease, allo- or xenogene reactions i.e. graft-versus-host and host-versus-graft disease.

**g) Cancerous diseases** associated with antigens such as
Survivin, Survivin-2B, Livin/ML-IAP, Bcl-2, Mcl-1, Bcl-X(L), Mucin-1, NY-ESO-1, Telomerase, CEA, MART-1, HER-2/neu, bcr-abl, PSA, PSCA, Tyrosinase, p53, hTRT, Leukocyte Proteinase-3, hTRT, gp100, MAGE antigens, GASC, JMJD2C, JARD2 (JMJ), JHDM3a, WT-1,CA 9, Protein kinases, where the cancerous diseases include malignant melanoma, renal carcinoma, breast cancer, lung cancer, cancer of the uterus, cervical cancer, prostatic cancer, pancreatic cancer, brain cancer, head and neck cancer, leukemia, cutaneous lymphoma, hepatic carcinoma, colorectal cancer, bladder cancer.

**h) Autoimmune and inflammatory diseases**, associated with antigens such as GAD64, Collagen, human cartilage glycoprotein 39, $\beta$-amyloid, A$\beta$42, APP, Presenilin 1, where the autoimmune and inflammatory diseases include Diabetes type 1, Rheumatoid arthritis, Alzheimer, chronic inflammatory bowel disease, Crohn's disease, ulcerative colitis uterosa, Multiple Sclerosis, Psoriasis

**Approaches to the analysis or treatment of diseases**

For each application of a MHC multimer, antigenic peptide or antigenic polypeptide, a number of choices must be made. These include:

A. Disease (to be e.g. treated, prevented, diagnosed, monitored).
B. Application (e.g. analyze by flow cytometry, isolate specific cells, induce an immune response)
C. Label (e.g. should the MHC multimer be labelled with a fluorophore or a chromophore)
D. Biologically active molecule (e.g. should a biologically active molecule such as an interleukin be added or chemically linked to the complex)
E. Peptide sequence (e.g. decide on the sequence of one or more antigenic peptide to be complexed with MHC, encoded in antigenic polypeptide or used as product itself)
F. MHC (e.g. use a MHC allele that does not interfere with the patient's immune system in an undesired way. This step is directly relevant for use of MHC multimers but also indirectly applicable when using antigenic peptide or polypeptide since these will following addition to assay/individual bind MHC molecules in sample/individual generating MHC multimer in sample/individual as described elsewhere herein).

A number of diseases $A_1$-$A_n$, relevant in connection with MHC multimers, antigenic peptide or antigenic polypeptide, have been described herein; a number of applications $B_1$-$B_n$, relevant in connection with MHC multimers, antigenic peptide or antigenic polypeptide, have been described herein; a number of Labels $C_1$-$C_n$, relevant in connection with MHC multimers, have been described herein; a number of biologically active molecules $D_1$-$D_n$, relevant in connection with MHC multimers antigenic peptide or antigenic polypeptide, have been described herein; a number of peptide sequences $E_1$-$E_n$, relevant in connection with MHC multimers, antigenic peptide or antigenic polypeptide, have been described herein; and a number of MHC molecules $F_1$-$F_n$, relevant in connection with MHC multimers, have been described herein.

Thus, each approach involves a choice to be made regarding all or some of the parameters A-F. A given application and the choices it involves can thus be described as follows:
Ai x Bi x Ci x Di x Ei x Fi

Where i specifies a number between 1 and n. n is different for different choices A, B, C, D, E, or F. Consequently, the present disclosure describes a large number of approaches to the diagnosis, monitoring, prognosis, therapeutic or vaccine treatment of diseases. The total number of approaches, as defined by these parameters, are
n(A) x n(B) x n(C) x n(D) x n(E) x n(F),
where n(A) describes the number of different diseases A described herein, n(B) describes the number of different applications B described herein, etc.

**Detection**

Diagnostic procedures, immune monitoring and some therapeutic processes of the present disclosure all involve identification and/or enumeration and/or isolation of antigen-specific T cells. Identification and enumeration of antigen-specific T cells may be done in a number of ways, and several assays are currently employed to provide this information.

In the following it is described how MHC multimers, antigenic peptides and/or antigenic polypeptides as described in the present disclosure can be used to detect specific T cell receptors (TCRs) and thereby antigen-specific T cells in a variety of methods and assays. In the present disclosure detection includes detection of the presence of antigen-specific TCR/ T cells in a sample, detection of and isolation of cells or entities with antigen-specific TCR from a sample and detection and enrichment of cells or entities with antigen-specific TCR in a sample.

The sample may be a biological sample including solid tissue, solid tissue section and fluid samples such as, but not limited to, whole blood, serum, plasma, nasal secretions, sputum, urine, sweat, saliva, transdermal exudates, pharyngeal exudates, bronchoalveolar lavage, tracheal aspirations, cerebrospinal fluid, synovial fluid, fluid from joints, vitreous fluid, vaginal or urethral secretions, seemen, or the like. Herein, disaggregated cellular tissues such as, for example, hair, skin, synovial tissue, tissue biopsies and nail scrapings are also considered as biological samples.

Many of the assays and methods described in the present inevntion are particularly useful for assaying T-cells in blood samples. Blood samples includes but is not limited to whole blood samples or blood processed to remove erythrocytes and platelets (e.g., by Ficoll density centrifugation or other such methods known to one of skill in the art) and the remaining PBMC sample, which includes the T-cells of interest, as well as B-cells, macrophages and dendritic cells, is used directly. Also included are blood samples processed in other ways e.g. isolating various subsets of blood cells by selecting or deselecting cells or entities in blood.

In order to be able to detect specific T cells by MHC multimers, labels and marker molecules can be used.

## Marker molecules

Marker molecules are molecules or complexes of molecules that bind to other molecules. Marker molecules thus may bind to molecules on entities, including the desired entities as well as undesired entities. Labeling molecules are molecules that may be detected in a certain analysis, i.e. the labeling molecules provide a signal detectable by the used method. Marker molecules, linked to labeling molecules, constitute detection molecules. Likewise labeling molecules linked to MHC multimers also constitute detection molecules but in contrast to detection molecules made up of marker and lebelling molecule labeled MHC multimers are specific for TCR. Sometimes a marker molecule in itself provides a detectable signal, wherefore attachment to a labeling molecule is not necessary.

Marker molecules are typically antibodies or antibodyfragments but can also be aptamers, proteins, peptides, small organic molecules, natural compounds (e.g. steroids), non-peptide polymers, or any other molecules that specifically and efficiently bind to other molecules are also marker molecules.

## Labelling molecules

Labelling molecules are molecules that can be detected in a certain analysis, i.e. the labelling molecules provide a signal detectable by the used method. The amount of labelling molecules can be quantified.

The labelling molecule is preferably such which is directly or indirectly detectable.

The labelling molecule may be any labelling molecule suitable for direct or indirect detection. By the term "direct" is meant that the labelling molecule can be detected per se without the need for a secondary molecule, i.e. is a "primary" labelling molecule. By the term "indirect" is meant that the labelling molecule can be detected by using one or more "secondary" molecules, i.e. the detection is performed by the detection of the binding of the secondary molecule(s) to the primary molecule.

The labelling molecule may further be attached via a suitable linker. Linkers suitable for attachment to labelling molecules would be readily known by the person skilled in the art and as described elsewhere herein for attachment of MHC molecules to multimerisation domains.

Examples of such suitable labelling compounds are fluorescent labels, enzyme labels, radioisotopes, chemiluminescent labels, bioluminescent labels, polymers, metal particles, haptens, antibodies, and dyes.

The labelling compound may suitably be selected:

from fluorescent labels such as 5-(and 6)-carboxyfluorescein, 5- or 6-carboxyfluorescein, 6-(fluorescein)-5-(and 6)-carboxamido hexanoic acid, fluorescein isothiocyanate (FITC), rhodamine, tetramethylrhodamine, and dyes such as Cy2, Cy3, and Cy5, optionally substituted coumarin including AMCA, PerCP, phycobiliproteins including R-phycoerythrin (RPE) and allophycoerythrin (APC), Texas Red, Princeston Red, Green fluorescent protein (GFP) and analogues thereof, and conjugates of R-phycoerythrin or allophycoerythrin and e.g. Cy5 or Texas Red, and inorganic fluorescent labels based on semiconductor nanocrystals (like quantum dot and Qdot™ nanocrystals), and time-resolved fluorescent labels based on lanthanides like Eu3+ and Sm3+,

from haptens such as DNP, biotin, and digoxiginin,

from enzymic labels such as horse radish peroxidase (HRP), alkaline phosphatase (AP), beta-galactosidase (GAL), glucose-6-phosphate dehydrogenase, beta-N-acetyl-glucosaminidase, β-glucuronidase, invertase, Xanthine Oxidase, firefly luciferase and glucose oxidase (GO),

from luminiscence labels such as luminol, isoluminol, acridinium esters, 1,2-dioxetanes and pyridopyridazines, and

from radioactivity labels such as incorporated isotopes of iodide, cobalt, selenium, tritium, and phosphor.

Radioactive labels may in particular be interesting in connection with labelling of the peptides harboured by the MHC multimers.

Different principles of labelling and detection exist, based on the specific property of the labelling molecule. Examples of different types of labelling are emission of radioactive radiation (radionuclide, isotopes), absorption of light (e.g. dyes, chromophores), emission of light after excitation (fluorescence from fluorochromes), NMR (nuclear magnetic resonance form paramagnetic molecules) and reflection of light (scatter from e.g. such as gold-, plastic- or glass-beads/particles of various sizes and shapes). Alternatively, the labelling molecules can have an enzymatic activity, by which they catalyze a reaction between chemicals in the near environment of the labelling molecules, producing a signal, which include production of light (chemi-luminescence), precipitation of chromophor dyes, or precipitates that can be detected by an additional layer of detection molecules. The enzymatic product can deposit at the location of the enzyme or, in a cell based analysis system, react with the membrane of the cell or diffuse into the cell to which it is attached. Examples of labelling molecules and associated detection principles are shown in table 2 below.

**Table 2: Examples of labelling molecules and associated detection principles.**

| Labelling substance | Effect | Assay-principle |
|---|---|---|
| Fluorochromes | emission of light having a specific spectra | ¤Photometry, Microscopy, spectroscopy PMT, photographic film, CCD's (Color-Capture Device or Charge-coupled device). |
| Radionuclide | irradiation, $\alpha$, $\beta$ or gamma □rays | Scintillation counting, GM-tube, photographic film, excitation of phosphor-imager screen |
| Enzyme; HRP,(horse reddish peroxidase), peroxidases in general | catalysis of $H_2O_2$ reduction using luminol as Oxygen acceptor, resulting in oxidized luminal + light | ¤Photometry, Microscopy, spectroscopy PMT, photographic film, CCD's (Colour-Capture Device or Charge-coupled device), Secondary label linked antibody |
| | catalysis of $H_2O_2$ reduction using a soluble dye, or molecule containing a hapten, such as a biotin residue as Oxygen acceptor, resulting in precipitation. The habten can be recognized by a detection molecule. | |
| Particles; gold, polystyrene beads, pollen and other particles | Change of scatter, reflection and transparency of the associated entity | Microscopy, cytometry, electron microscopy PMT's, light detecting devices, flowcytometry scatter |
| AP (Alkaline Phosphatase) | Catalyze a chemical conversion of a non-detectable to a precipitated detectable molecule, such as a dye or a hapten | ¤Photometry, Microscopy, spectroscopy Secondary label linked antibody |
| Ionophores or chelating chemical compounds binding to specific ions, e.g. $Ca^{2+}$ | Change in absorption and emission spectrums when binding. Change in intensity | ¤Photometry, Cytometry, spectroscopy |

(continued)

| Labelling substance | Effect | Assay-principle |
|---|---|---|
| Lanthanides | Fluorescence | ¤photometry, cytometry, spectroscopy |
| | Phosphorescence | |
| | Paramagnetic | NMR (Nuclear magnetic resonance) |
| DNA fluorescing stains | Propidium iodide Hoechst stain DAPI AMC DraQ5™ Acridine orange 7-AAD | ¤Photometry, cytometry, spectroscopy |

¤Photometry; is to be understood as any method that can be applied to detect the intensity, analyze the wavelength spectra, and or measure the accumulation of light derived form a source emitting light of one or multiple wavelength or spectra.

Labelling molecules can be used to label MHC multimers as well as other reagents used together with MHC multimers, e.g. antibodies, aptamers or other proteins or molecules able to bind specific structures in another protein, in sugars, in DNA or in other molecules. In the following molecules able to bind a specific structure in another molecule are named a marker.

Labelling molecules can be attached to a given MHC multimer or any other protein marker by covalent linkage as described for attachment of MHC multimers to multimerization domains elsewhere herein. The attachment can be directly between reactive groups in the labelling molecule and reactive groups in the marker molecule or the attachment can be through a linker covalently attached to labelling molecule and marker, both as described elsewhere herein. When labelling MHC multimers the label can be attached either to the MHC complex (heavy chain, $\beta$2m or peptide) or to the multimerization domain.

In particular,

one or more labelling molecules may be attached to the carrier molecule, or one or more labelling molecules may be attached to one or more of the scaffolds, or one or more labelling compounds may be attached to one or more of the MHC complexes, or one or more labelling compounds may be attached to the carrier molecule and/or one or more of the scaffolds and/or one or more of the MHC complexes, or one or more labelling compounds may be attached to the peptide harboured by the MHC molecule.

A single labelling molecule on a marker does not always generate sufficient signal intensity. The signal intensity can be improved by assembling single label molecules into large multi-labelling compounds, containing two or more label molecule residues. Generation of multi-label compounds can be achived by covalent or non-covalent, association of labelling molecules with a major structural molecule. Examples of such structures are synthetic or natural polymers (e.g. dextramers), proteins (e.g. streptavidin), or polymers. The labelling molecules in a multi-labelling compound can all be of the same type or can be a mixture of different labelling molecules.

In some applications, it may be advantageous to apply different MHC complexs, either as a combination or in individual steps. Such different MHC multimers can be differently labelled (i.e. by labelling with different labelling compounds) enabling visualisation of different target MHC-peptide recognising cells. Thus, if several different MHC multimers with different labelling compounds are present, it is possible simultaneously to identify more than one specific receptor, if each of the MHC multimers presents a different peptide.

Detection principles, such as listed in Table 2, can be applied to flow cytometry, stationary cytometry, and batch-based analysis. Most batch-based approaches can use any of the labelling substances depending on the purpose of the assay. Flow cytometry primarily employs fluorescence, whereas stationary cytometry primarily employs light absorption, e.g. dyes or chromophore deposit from enzymatic activity. In the following section, principles involving fluorescence detection will be exemplified for flow cytometry, and principles involving chromophore detection will be exemplified in the context of stationary cytometry. However, the labelling molecules can be applied to any of the analyses described in this disclosure.

**Labelling molecules of particular utility in Flow Cytometry:**

In flowcytometry the typical label is detected by its fluorescence. Most often a positive detection is based on the presents of light from a single fluorochrome, but in other techniques the signal is detected by a shift in wavelength of emitted light; as in FRET based techniques, where the exited fluorochrome transfer its energy to an adjacent bound fluorochrome that emits light, or when using $Ca^{2+}$ chelating fluorescent props, which change the emission (and absorption) spectra upon binding to calcium. Preferably labelling molecules employed in flowcytometry are illustrated in Table 3 and 4 and described in the following.

Simple fluorescent labels:

• Fluor dyes, Pacific Blue™, Pacific Orange™, Cascade Yellow™,

• AlexaFluor®(AF);

  ◦ AF405, AF488,AF500, AF514, AF532, AF546, AF555, AF568, AF594, AF610, AF633, AF635, AF647, AF680, AF700, AF710, AF750, AF800

• Quantum Dot based dyes, QDot® Nanocrystals (Invitrogen, MolecularProbs)

  ◦ Qdot®525, Qdot®565, Qdot®585, Qdot®605, Qdot®655, Qdot®705, Qdot®800

• DyLight™ Dyes (Pierce) (DL);

  ◦ DL549, DL649, DL680, DL800

• Fluorescein (Flu) or any derivate of that, ex. FITC

• Cy-Dyes

  ◦ Cy2, Cy3, Cy3.5, Cy5, Cy5.5, Cy7

• Fluorescent Proteins;

  ◦ RPE, PerCp, APC

  ◦ Green fluorescent proteins;

    ▪ GFP and GFP-derived mutant proteins; BFP,CFP, YFP, DsRed, T1, Dimer2, mRFP1,MBanana, mOrange, dTomato, tdTomato, mTangerine, mStrawberry, mCherry

• Tandem dyes:

  ◦ RPE-Cy5, RPE-Cy5.5, RPE-Cy7, RPE-AlexaFluor® tandem conjugates; RPE-Alexa610, RPE-TxRed

  ◦ APC-Aleca600, APC-Alexa610, APC-Alexa750, APC-Cy5, APC-Cy5.5

• Ionophors; ion chelating fluorescent props

  ◦ Props that change wavelength when binding a specific ion, such as Calcium
  Props that change intensity when binding to a specific ion, such as Calcium

• Combinations of fluorochromes on the same marker. Thus, the marker is not identified by a single fluorochrome but by a code of identification being a specific combination of fluorochromes, as well as inter related ratio of intensities. Example: Antibody Ab1 and Ab2, are conjugated to both. FITC and BP but Ab1 have 1 FITC to 1 BP whereas Ab2 have 2 FITC to 1 BP. Each antibodymay then be identified individually by the relative intensity of each fluorochrome. Any such combinations of n fluorochromes with m different ratios can be generated.

**Table 3: Examples of preferable fluorochromes**

| Fluorofor / Fluorochrome | Excitation nm | Emission nm |
|---|---|---|
| 2-(4'-maleimidylanilino)naphthalene-6-sulfonic acid, sodium salt | 322 | 417 |
| 5-((((2-iodoacetyl)amino)ethyl)amino) naphthalene-1-sulfonic acid | 336 | 490 |
| Pyrene-1-butanoic acid | 340 | 376 |
| AlexaFluor 350 (7-amino-6-sulfonic acid-4-methyl coumarin-3-acetic acid) | 346 | 442 |
| AMCA (7-amino-4-methyl coumarin-3-acetic acid) | 353 | 442 |
| 7-hydroxy-4-methyl coumarin-3-acetic acid | 360 | 455 |
| Marina Blue (6,8-difluoro-7-hydroxy-4-methyl coumarin-3-acetic acid) | 362 | 459 |
| 7-dimethylamino-coumarin-4-acetic acid | 370 | 459 |
| Fluorescamin-N-butyl amine adduct | 380 | 464 |
| 7-hydroxy-coumarine-3-carboxylic acid | 386 | 448 |
| CascadeBlue (pyrene-trisulphonic acid acetyl azide) | 396 | 410 |
| Cascade Yellow | 409 | 558 |
| Pacific Blue (6,8 difluoro-7-hydroxy coumarin-3-carboxylic acid) | 416 | 451 |
| 7-diethylamino-coumarin-3-carboxylic acid | 420 | 468 |
| N-(((4-azidobenzoyl)amino)ethyl)-4-amino-3,6-disulfo-1,8-naphthalimide, dipotassium salt | 426 | 534 |
| Alexa Fluor 430 | 434 | 539 |
| 3-perylenedodecanoic acid | 440 | 448 |
| 8-hydroxypyrene-1,3,6-trisulfonic acid, trisodium salt | 454 | 511 |
| 12-(N-(7-nitrobenz-2-oxa-1,3-diazol-4-yl)amino)dodecanoic acid | 467 | 536 |
| N,N'-dimethyl-N-(iodoacetyl)-N'-(7-nitrobenz-2- oxa-1,3-diazol-4-yl) ethylenediamine | 478 | 541 |
| Oregon Green 488 (difluoro carboxy fluorescein) | 488 | 518 |
| 5-iodoacetamidofluorescein | 492 | 515 |
| Propidium iodide-DNA adduct | 493 | 636 |
| Carboxy fluorescein | 495 | 519 |

**Table 4: Examples of preferable fluorochrome families**

| Fluorochrome family | Example fluorochrome |
|---|---|
| AlexaFluor®(AF) | AF®350, AF405, AF430, AF488,AF500, AF514, AF532, AF546, AF555, AF568, AF594, AF610, AF633, AF635, AF647, AF680, AF700, AF710, AF750, AF800 |
| Quantum Dot (Qdot®) based dyes | Qdot®525, Qdot®565, Qdot®585, Qdot®605, Qdot®655, Qdot®705, Qdot®800 |
| DyLight™ Dyes (DL) | DL549, DL649, DL680, DL800 |
| Small fluorescing dyes | FITC, Pacific Blue™, Pacific Orange™, Cascade Yellow™, Marina blue™, DSred, DSred-2, 7-AAD, TO-Pro-3, |
| Cy-Dyes | Cy2, Cy3, Cy3.5, Cy5, Cy5.5, Cy7 |
| Phycobili Proteins: | R-Phycoerythrin (RPE), PerCP, Allophycocyanin (APC), B-Phycoerythrin, C-Phycocyanin |
| Fluorescent Proteins | (E)GFP and GFP ((enhanced) green fluorescent protein) derived mutant proteins; BFP, CFP, YFP, DsRed, T1, Dimer2, mRFP1,MBanana, mOrange, dTomato, tdTomato, mTangerine, mStrawberry, mCherry |

(continued)

| Fluorochrome family | Example fluorochrome |
|---|---|
| Tandem dyes with RPE | RPE-Cy5, RPE-Cy5.5, RPE-Cy7, RPE-AlexaFluor® tandem conjugates; RPE-Alexa610, RPE-TxRed |
| Tandem dyes with APC | APC-Aleca600, APC-Alexa610, APC-Alexa750, APC-Cy5, APC-Cy5.5 |
| Calcium dyes | Indo-1-Ca2+ Indo-2-Ca2+ |

**Preferably labelling molecules employed in stationary cytometry and IHC**

- Enzymatic labelling, as exemplified in Table 5:

  ○ Horse radish peroxidase; reduces peroxides ($H_2O_2$), and the signal is generated by the Oxygen acceptor when being oxidized.

    ■ Precipitating dyes; Dyes that when they are reduced they are soluble, and precipitate when oxidized, generating a coloured deposit at the site of the reaction.

    ■ Precipitating agent, carrying a chemical residue, a hapten, for second layer binding of marker molecules, for amplification of the primary signal.

    ■ Luminol reaction, generating a light signal at the site of reaction.

  ○ Other enzymes, such as Alkaline Phosphatase, capable of converting a chemical compound from a non-detectable molecule to a precipitated detectable molecule, which can be coloured, or carries a hapten as described above.

- Fluorescent labels, as exemplified in Table 3 and 4; as those described for Flow cytometry are likewise important for used in stationary cytometry, such as in fluorescent microscopy.

**Table 5: Examples of preferable labels for stationary cytometry**

| Label | Enzyme substrate, Oxygen acceptor Chromogen / precipitating agent | Precipitate or Residue, hapten* for secondary detection layer | Binding partner to hapten |
|---|---|---|---|
| HRP | diaminobenzidine (DAB) | Colored precipitate | - |
| HRP | 3-amino-9-ethylcarbazole (AEC+) | Colored precipitate | - |
| AP | Fast red dye | Red precipitate | - |
| HRP | biotinyl tyramide | Exposed Biotin residue | Streptavidin, avidine |
| HRP | fluorescein tyramide | Exposed Fluorescein residue | Anti-Fluorecein Antibody |
| "Enzyme" | Substrate that when reacted precipitate | Primary label; being a dye, chemiluminescence's, or exposure of a hapten | Secondary label in case the primary label is a hapten |

**Detection methods and principles**

MHC multimers can be used to detect T-cell receptors (TCR) e.g. T-cells carrying specific TCR. For example, the MHC multimers can be labelled with fluorophores and used in flow cytometer, the MHC multimers can be labelled with chromophores, in order to specifically stain specific T-cells carrying TCRs that specifically bind the MHC multimer in question in e.g. sections of solid tissues using IHC.

ELISA and ELISA-like analyses can be performed with MHC multimers that are labelled with e.g. chromophores, fluorophores or enzymes.

MHC multimers can be used in a wide range os other methods using various principles. In the following methods and principles using MHC multimers for detection of TCR are outlined.

Detection of TCRs with MHC multimers may be direct or indirect.

## Direct detection

Direct detection of TCRs is detection directly of the binding interaction between the specific T cell receptor and the MHC multimer. The MHC multimer may be generated and then added to sample or alternatively MHC multimers are generated in sample by addition of antigenic peptide and MHC molecules attached to multimerization domain to sample.

Direct detection includes detection of TCR when TCR is attached to lipid bilayer (e.g. T cells), when TCR is attached to or in a solid medium or when TCR is in solution.

### Direct detection of TCR attached to lipid bilayer

One type of TCRs to detect and measure are TCRs attached to lipid bilayer including but not limited to naturally occurring T cells (from blood, spleen, lymphnode, brain or any other tissue containing T cells), TCR transfected cells, T cell hybridomas, TCRs embedded in liposomes or any other membrane structure. In the following methods for direct detection of entities of TCRs attached to lipid bilayer will be described and any entity consisting of TCR attached to lipid bilayer will be referred to as T cells.

T cells can be directly detected either when in a fluid solution or when immobilized to a solid support.

Direct detection of T cells in fluid sample.

T cells can be detected in fluid samples using the methods described below including but not limited to detection of T cells in culture media, in buffers, in water or in other liquids and also suspensions of disrupted tissues e.g. homogenized tissue resuspended in the fluids described above. T cells in fluid samples can be detected individually or detected as populations of T cells. In the following different methods for direct detction of T cells in fluid samples are described.

*Direct detection of individual T cells*

◦ *Direct detection of individual T cells using flow cytometry.*
An example of direct detection of individual T cells by flow cytometry is measurement of antigen specific T cells using MHC multimers like Tetramers, Pentamers, Dextramers or similar types of reagents.
Briefly, a suspension of T cells are added MHC multimers, the sample washed and then the amount of MHC multimer bound to each cell are measured. Bound MHC multimers may be labelled directly or measured through addition and binding of labelled marker molecules. The sample is analyzed using a flow cytometer, able to detect and count individual cells passing in a stream through a laser beam. For identification of specific T cells using MHC multimers, cells are stained with fluorescently labeled MHC multimer by incubating cells with fluorochrome labelled MHC multimer and then forcing the cells with a large volume of liquid through a nozzle creating a stream of spaced cells. Each cell passes through a laser beam and any fluorochrome bound to the cell is excited and thereby fluoresces. Sensitive photomultipliers detect emitted fluorescence, providing information about the amount of MHC multimer bound to the cell. By this method MHC multimers can be used to identify specific T cell populations in liquid samples such as blood, CSF, synovial fluid, cell cultures or any other liquid sample containing T cells..
When analyzing blood samples whole blood can be used with or without lysis of red blood cells. Alternatively lymphocytes can be purified from blood before flow cytometry analysis e.g. using a standard procedure like a Ficoll-Hypaque gradient. Another possibility is to isolate lymphocytes, subgroups of lymphocytes, T cells or subgroups of T cells from the blood sample for example by affinity purification like binding to antibody coated surfaces, followed by elution of bound cells. This purified lymphocyte or T cell population can then be used for flow cytometry analysis together with MHC multimers.
Instead of actively isolating T cells or subgroups of lymphocytes unwanted cells like B cells, NK cells or any other unwanted cells or substances can be removed prior to the analysis. One way to do this is by affinity purification e.g. using columns or beads or other surfaces coated with antibodies specific for the unwanted cells. Alternatively, specific antibodies recognizing the unwanted cells can be added to the blood sample together with complement proteins, thereby killing cells recognized by the antibodies.
Various gating reagents can be included in the analysis. Gating reagents here means labeled antibodies or other labeled marker molecules identifying subsets of cells by binding to unique surface proteins. Preferred gating reagents when using MHC multimers are antibodies or other marker molecules directed against CD3, CD4, and CD8 identifying major subsets of T cells. Other preferred gating reagents are antibodies or marker molecules specifically binding CD14, CD15, CD19, CD25, CD56, CD27, CD28, CD45, CD45RA, CD45RO, CCR7, CCR5, CD62L, Foxp3, CD95, CD127, CD7, CD57, CD154 or other specific proteins or molecules unique for different lymphocytes of the immune system.

Following labelling with MHC multimers and before analysis on a flow cytometer stained cells can be treated with a fixation reagent e.g. formaldehyde to crosslink bound MHC multimer to the cell surface. Stained cells can also be analyzed directly without fixation.

The number of cells in a sample can vary. When the target cells are rare, it is preferable to analyze large amounts of cells. In contrast, fewer cells are required when looking at T cell lines or samples containing many cells of the target cell type.

The flow cytometer can be equipped to separate and collect particular types of cells. This is called cell sorting. MHC multimers in combination with sorting on a flowcytometer can be used to isolate specific T cell populations. Isolated specific T cell populations can then be further manipulated as described elsewhere herein, e.g. expanded *in vitro*. This can be useful in autologous cancer therapy.

Amounts of MHC-peptide specific T cells in a blood sample can be determined by flow cytometry by calculating the amount of MHC multimer labeled cells in a given volumen of sample with a given cell density and then back calculate. Exact enumeration of specific T cells is better achieved by incubating sample with MHC multimers (and optionally gating reagents) together with an exact amount of counting beads followed by flow cytometry analysis. Counting beads is here to be understood as any fluorescent bead with a size that can be visualized in a sample containing T cells by flow cytometry. The beads could e.g. be made of polystyrene with a size of about 1-10 $\mu$m. They could also be made of agarose, polyacrylamide, silica, or any other material, and have any size between 0,1 $\mu$m and 100 $\mu$m. The counting beads are used as reference population to measure the exact volume of analyzed sample. The sample are analyzed on a flow cytometer and the amount of MHC-specific T cell detected can then be correlated with the amount of counting beads in the same volume of the sample and an exact number of MHC-peptide specific T cells determined using the following equation:

Concentration of MHC-specific T-cell in sample = (number of MHC-peptide specific T cells counted/number of counting beads counted) x concentration of counting beads in sample

Alternatively MHC multimers are added to one tube (below denoted tube 1) together with sample and counting beads are added to a separate tube (below denoted tube 2) containing the same sample but no MHC multimers. To both tubes one or more gating reagents are added able to identify other cell subsets in sample e.g. CD3+, CD4+, CD8+, CD19+, CD56+ cells. The exact amount of one of the cell subsets for which gating reagents are included are then calculated from the tube containing counting beads. For example if CD8+ cells are measured in both tubes the following equation can be used to determine the exact concentration of CD8+ cells in the sample:

(((number of CD8+ cells counted (tube 2)) / (number of counting beads counted (tube 2)) ) x (concentration of counting beads in sample) = exact concentration of CD8+ cells in sample

The exact concentration of CD8+ cells in sample are then used to determine the exact concentration of MHC-specific T cells in sample using the equation:

(Calculated exact concentration of CD8+ cells in sample (calculated from tube 2)) x (MHC-specific T cells counted as percentage of CD8+ events counted in sample (tube 1)) = concentration of MHC-specific T-cell in sample

◦ *Direct detection of individual T cells in fluid sample by microscopy*

A suspension of T cells are added MHC multimers, the sample washed and then the amount of MHC multimer bound to each cell are measured. Bound MHC multimers may be labelled directly or labelled through addition of labelled marker molecules. The sample is then spread out on a slide or similar in a thin layer able to distinguish individual cells and labelled cells identified using a microscope. Depending on the type of label different types of microscopes may be used, e.g. if fluorescent labels are used a fluorescent microscope is used for the analysis. For example MHC multimers can be labeled with a flourochrome or bound MHC multimer detected with a fluorescent antibody. Cells with bound fluorescent MHC multimers can then be visualized using an immunofluorescence microscope or a confocal fluorescence microscope.

*Direct detection of populations of T cells*

∘ Cell suspensions are added labeled MHC multimer, samples are washed and then total signal from label are measured. The MHC multimers may be labeled themselves or detected through a labeled marker molecule.

∘ Cell suspensions are added labeled MHC multimer, samples are washed and then signal from label are amplified and then total signal from label and/or amplifier are measured.

Direct detection of immobilized T cells.

T cells may be immobilized and then detected directly. Immobilization can be on solid support, in solid tissue or in fixator (e.g. paraffin, a sugar matrix or another medium fixing the T cells).

*Direct detection of T cells immobilized on solid support.*

In a number of applications, it may be advantageous to immobilize the T cell onto a solid or semi-solid support. Such support may be any which is suited for immobilisation, separation etc. Non-limiting examples include particles, beads, biodegradable particles, sheets, gels, filters, membranes (e. g. nylon membranes), fibres, capillaries, needles, microtitre strips, tubes, plates or wells, combs, pipette tips, micro arrays, chips, slides, or indeed any solid surface material. The solid or semi-solid support may be labelled, if this is desired. The support may also have scattering properties or sizes, which enable discrimination among supports of the same nature, e.g. particles of different sizes or scattering properties, colour or intensities.

Conveniently the support may be made of glass, silica, latex, plastic or any polymeric material. The support may also be made from a biodegradable material.

Generally speaking, the nature of the support is not critical and a variety of materials may be used. The surface of support may be hydrophobic or hydrophilic.

Preferred are materials presenting a high surface area for binding of the T cells. Such supports may for example be porous or particulate e.g. particles, beads, fibres, webs, sinters or sieves. Particulate materials like particles and beads are generally preferred due to their greater binding capacity. Particularly polymeric beads and particles may be of interest.

Conveniently, a particulate support (e.g. beads or particles) may be substantially spherical. The size of the particulate support is not critical, but it may for example have a diameter of at least 1 $\mu$m and preferably at least 2 $\mu$m, and have a maximum diameter of preferably not more than 10 $\mu$m and more preferably not more than 6 $\mu$m. For example, particulate supports having diameters of 2.8 $\mu$m and 4.5 $\mu$m will work well.

An example of a particulate support is monodisperse particles, i.e. such which are substantially uniform in size (e. g. size having a diameter standard deviation of less than 5%). Such have the advantage that they provide very uniform reproducibility of reaction. Monodisperse particles, e.g. made of a polymeric material, produced by the technique described in US 4,336,173 (ref. 25) are especially suitable.

Non-magnetic polymer beads may also be applicable. Such are available from a wide range of manufactures, e.g. Dynal Particles AS, Qiagen, Amersham Biosciences, Serotec, Seradyne, Merck, Nippon Paint, Chemagen, Promega, Prolabo, Polysciences, Agowa, and Bangs Laboratories.

Another example of a suitable support is magnetic beads or particles. The term "magnetic" as used everywhere herein is intended to mean that the support is capable of having a magnetic moment imparted to it when placed in a magnetic field, and thus is displaceable under the action of that magnetic field. In other words, a support comprising magnetic beads or particles may readily be removed by magnetic aggregation, which provides a quick, simple and efficient way of separating out the beads or particles from a solution. Magnetic beads and particles may suitably be paramagnetic or superparamagnetic. Superparamagnetic beads and particles are e.g. described in EP 0 106 873 (Sintef, ref. 26). Magnetic beads and particles are available from several manufacturers, e.g. Dynal Biotech ASA (Oslo, Norway, previously Dynal AS, e.g. Dynabeads®).

The support may suitably have a functionalised surface. Different types of functionalisation include making the surface of the support positively or negatively charged, or hydrophilic or hydrophobic. This applies in particular to beads and particles. Various methods therefore are e.g. described in US 4,336,173 (ref. 25), US 4,459,378 (ref. 27) and US 4,654,267 (ref. 28).

Immobilized T cells may be detected in several ways including:

◦ *Direct detection of T cells directly immobilized on solid support.*

T cells may be directly immobilized on solid support e.g. by non-specific adhesion. Then MHC multimers are added to the immobilized T cells thereby allowing specific T cells to bind the MHC multimers. Bound MHC multimer may be measured through label directly attached to the multimer or through labeled marker molecules. Individual T cells may be detected if the method for analysis is able to distinguish individual labelled cells, e.g. cells are immobilized in a monolayer on a cell culture well or a glass slide. Following staining with labelled multimer a digital picture is taken and labelled cells identified and counted. Alternatively a population of T cells is detected by measurement of total signal from all labelled T cells, e.g. cells are plated to wells of a microtiter plate, stained with labelled MHC multimer and total signal from each well are measured.

◦ *Direct detection of T cells immobilized on solid support through linker molecule*

T cells can also be immobilized to solid support through a linker molecule. The linker molecule can be an antibody specific for the T cell, a MHC multimer, or any molecule capable of binding T cells. In any case the linker may be attached directly to the solid support, to the solid support through another linker, or the linker molecule may be embedded in a matrix, e.g. a sugar matrix.

Then MHC multimers are added to the immobilized T cells thereby allowing specific T cells to bind the MHC multimers. Bound MHC multimer may be measured through label directly attached to the multimer or through labeled marker molecules. Individual T cells may be detected if the method for analysis is able to distinguish individual labelled cells, e.g. a digital picture is taken and labelled cells identified and counted.

By using a specific MHC multimer both for the immobilization of the T-cells and for the labelling of immobilized cells (e.g. by labelling immobilized cells with chromophore- or fluorophore-labelled MHC multimer), a very high analytical specificity may be achieved because of the low background noise that results.

Alternatively a population of T cells is detected by measurement of total signal from all labeled T cells.

◦ *Immuno profiling: Phenotyping T cell sample using MHC multimer beads or arrays.*

Different MHC multimers are immobilized to different beads with different characteristics (e.g. different size, different fluorophores or different fluorescence intensities) where each kind of bead has a specific type of MHC multimer molecule immobilized. The immobilization may be direct or through a linker molecule as described above. The amount of bound T cells to a specific population of beads can be analyzed, thereby phenotyping the sample. The TCR on the T cell is defined by the MHC multimer and hence the bead to which it binds.

Likewise, MHC multimers can be immobilized in an array, e.g. on a glass plate or pin array so that the position in the array specifies the identity of the MHC multimer. Again, the immobilization may be direct or through a linker molecule as described above. After addition of T cells, the amount of bound T cells at a specified position in the array can be determined by addition of a label or labelled marker that binds to cells in general, or that binds specifically to the cells of interest. For example, the cells may be generally labelled by the addition of a labelled molecule that binds to all kinds of cells, or specific cell types, e.g. CD4+ T-cells, may be labelled with anti-CD4 antibodies that are labelled with e.g. a chromophore or fluorophore. Either of these approaches allow a phenotyping of the sample. An example for the use of immuno profiling is given below.

• Profiling of an individual's disease-specific T-cell repertoire.

Mass profiling of the T-cells of an individual may be done by first immobilizing specific MHC multimers (e.g. $10$-$10^6$ different MHC multimers, each comprising a specific MHC-peptide combination) in an array (e.g. a glass plate), adding e.g. a blood sample from the individual, and then after washing the unbound cells off, label the immobilized cells. Positions in the array of particularly high staining indicate MHC-peptide combinations that recognize specific T-cells of particularly high abundance or affinity. Thus, an immuno profiling of the individual with regard to the tested MHC-peptide combinations is achieved. A similar profiling of an individuals disease may be made using MHC multimers immobilized to different beads as described above.

Whether the profiling is performed using beads or arrays, the profiling may entail a number of diseases, a specific disease, a set of specific antigens implicated in one or more diseases, or a specific antigen (e.g. implicated in a specific disease or set of diseases).

In a preferred embodiment of the present disclosure, an individual's immuno profile for a particular antigen is obtained. Thus, peptides corresponding to all possible 8'-, 9'- 10'- and 11'-mer peptide sequences derived from the peptide antigen sequence are generated, for example by standard organic synthesis or combinatorial chemistry, and the corresponding MHC multimers are produced, using one or more of the class I MHC-alleles of the individual in question. Further, peptides of e.g. 13, 14, 15, 16 and upto 25 amino acids length may be generated, for example by organic synthesis or combinatorial chemistry, corresponding to all 13', 14', 15', 16' and upto 25'-mers of the antigen, and the corresponding class II MHC multimers are produced, using one or more of the class II MHC-alleles of the individual in question. For a complete profiling for this particular antigen, all of the HLA-alleles of the individual in question should be used for the generation of the array; i.e., if the HLA class I haplotype of the individual is HLA-A*02, HLA-A*03, HLA-B*08 and HLA-B*07, all these HLA class I alleles should be combined with every tested peptide and similarly for all HLA class II alleles of the given individual.

Based on the profile, a personalized drug, -vaccine or -diagnostic test may be produced.

The principle described above may also be employed to distinguish between the immune response raised against a disease (e.g. an infection with a bacterium or the formation of a tumour), and the immune response raised against a vaccine for the same disease (in the example, a vaccine against the bacterium or the tumour). Most vaccines consists of subcomponents of the pathogen /tumour they are directed against and/or are designed to elicit an immune response different from the natural occuring immune response i.e. the T cell epitopes of the two immune reponses differs. Thus, by establishing the immuno profile, using a comprehensive array (i.e. an array that comprises all possible epitopes from one or more antigen(s)) or a subset of these epitopes, it is possible to deduce whether the immune response has been generated against the disease or the vaccine, or against both the disease and the vaccine. If the vaccine raises a response against a particular epitope or a particular set of epitopes, the corresponding positions in the array will give rise to high signals (compared to the remaining positions). Similarly a natural generated immune response will be directed against other and/or more particular epitopes and therefore give rise to high signals in other positions and/or more positions in the array. When an individual is vaccinated the immuno profile will reflect the effect of the vaccination on the immune response, and even if the individual has encountered the disease before and has generated a general immune response towards this disease, it will still be possible to deduce from the profiling whether this individual also has generated a specific response against the vaccine.

In another preferred embodiment of the present disclosure, an individual's immuno profile for a set of antigens implicated in a specific disease is obtained. A subset of epitopes from a number of antigens is used. Thus, this is not a comprehensive profiling of this individual with regard to these antigens, but careful selection of the epitopes used may ensure that the profiling data can be used afterwards to choose between e.g. a limited set of vaccines available, or the data can be used to evaluate the immune response of the individual following an infection, where the epitopes used have been selected in order to avoid interference from related infectious diseases.

As above, a personalized drug, -vaccine or -diagnostic test may be produced. based on the information obtained from the immuno profiling.

In yet another preferred embodiment of the present disclosure, the array comprising all possible 8'-, 9'- 10'- and 11'-mer peptide sequences derived from a given peptide antigen, and all 13, 14, 15 and 16'-mers of the same antigen, are synthesized and assembled in MHC multimers, and immobilized in an array. Then, the ability of the individual peptide to form a complex with MHC is tested. As an example, one may add labelled W6/32 antibody, an antibody that binds correctly folded MHC I heavy chain, when this heavy chain is assembled together with antigenic peptide and beta2microglobulin, and which therefore can be used to detect formation of MHC-peptide complex, as binding of W6/32 antibody is usually considered a strong indication that the MHC-peptide complex has been formed. The ability of different peptides to enter into a MHC-peptide complex may also be promoted by the addition to the array of T-cells. Specific T-cells will drive the formation of the corresponding specific MHC-peptide complexes. Thus, after addition of T-cells to the array, the MHC-peptide complex integrity can be examined by addition of the labelled W6/32 antibody or other antibodies specific for correct conformation. Positions on the array that have strong signals indicate that the peptide that was added to MHC and immobilized at this position, was capable of forming the MHC-peptide complex in the presence of specific T-cells. Alternatively, the binding of the specific T-cells to the corresponding MHC-peptide complexes may be detected directly through a labbelled antibody specific for the T cell.

*Direct detection of immobilized T cells followed by sorting*

Specific T cells or specific T cell subsets can be isolated from a sample containing other T cells, T cell subsets and/or other cells by immobilization of the wanted specific T cells in sample to solid support as described above followed by washing and elution. For example, MHC multimers are immobilized to a support e.g. beads, immunotubes, wells of a microtiterplate, CD, mircrochip or similar as decribed elsewhere herein, then a suspension of T cells (the sample) are added allowing specific T cells to bind MHC multimer molecules. Following washing bound T cells are recovered/eluted (e.g. using acid or competition with one or more competitor molecules) and counted.

Specific T-cells can e.g.be isolated through the use of bead-based MHC multimers. Bead-based MHC multimers are beads whereto monomer MHC-peptide complexes or MHC multimers are immobilized.

The isolated T cells can following elution optionally be manipulated further before final use. For example the isolated cells can be activated (to differentiate or proliferate), they can undergo induced apoptosis, or undesired cells of the isolated cell population can be removed. Then, the manipulated cell population can be re-introduced into the patient from which the sample originate, or can be introduced into another patient.

A typical cell sorting experiment, based on bead-based MHC multimers, would follow some of the steps of the general procedure outlined in general terms in the following: Acquire the sample, e.g. a cell sample from the blood or bone marrow of a cancer patient.

Block the sample with a protein solution, e.g. BSA or skim milk.

Block the beads coated with MHC complexes or MHC multimers, with BSA or skim milk.

Mix MHC-coated beads and the cell sample, and incubate.

Wash the beads with washing buffer, to remove unbound cells and non-specifically bound cells.

Isolate the immobilized cells, by either cleavage of the linker that connects MHC complex/MHC multimer and bead; or alternatively, release the cells by a change in pH, salt-concentration addition of competitive binding molecule or the like. Preferably, the cells are released under conditions that do not disrupt the integrity of the cells.

Manipulate the isolated cells (e.g. induce apoptosis, proliferation or differentiation)

*Direct detection of T cells in solid tissue.*

◦ *Direct detection of T cells in solid tissue in vitro.*
Example direct detection of T cells in solid tissue *in vitro* include but is not limited til Immunohsitochemistry (IHC). IHC is here referred to as the detection of antigens in solid tissue by antibodies or other marker molecules labelled with a labelling molecule as described elsewhere herein.
For *in vitro* methods of the present disclosure solid tissue includes tissue, tissue biopsies, frozen tissue or tissue biopsies, paraffin embedded tissue or tissue biopsies and sections of either of the above mentioned. In a preferred method of this disclosure sections of fixed or frozen tissues are incubated with MHC multimer, allowing MHC multimer to bind to specific T cells in the tissue section.
The MHC multimer may be labeled directly or through a labeled marker molecule. As an example, the MHC multimer can be labeled with a tag that can be recognized by e.g. a secondary antibody, optionally labeled with HRP or another label. The bound MHC multimer is then detected by its fluorescence or absorbance (for fluorophore or chromophore), or by addition of an enzyme-labeled antibody directed against this tag, or another component of the MHC multimer (e.g. one of the protein chains, a label on the multimerization domain).
The enzyme can be Horse Raddish Peroxidase (HRP) or Alkaline Phosphatase (AP), both of which convert a colorless substrate into a colored reaction product *in situ.* This colored deposit identifies the binding site of the MHC multimer, and can be visualized under a light microscope. The MHC multimer can also be directly labeled with e.g. HRP or AP, and used in IHC without an additional antibody.
The tissue sections may derive from blocks of tissue or tissue biopsies embedded in paraffin, and tissue sections from this paraffin-tissue block fixed in formalin before staining. This procedure may influence the structure of the TCR in the fixed T cells and thereby influence the ability to recognize specific MHC complexes. In this case, the native structure of TCR needs to be at least partly preserved in the fixed tissue. Fixation of tissue therefore should be gentle. Alternatively, the staining is performed on frozen tissue sections, and the fixation is done after MHC multimer staining.
◦ *Direct detection of T cells in solid tissue in vivo*
For in vivo detection of T cells labeled MHC multimers are injected in to the body of the individual to be investigated. The MHC multimers may be labeled with e.g. a paramagnetic isotope. Using a magnetic resonance imaging (MRI) scanner or electron spin resonance (ESR) scanner MHC multimer binding T cells can then be measured and localized. In general, any conventional method for diagnostic imaging visualization can be utilized. Usually gamma and positron emitting radioisotopes are used for camera and paramagnetic isotopes for MRI.

The methods described above for direct detection of TCR embedded in lipid bilayers collectively called T cells using MHC multimers also applies to detection of TCR in solution and detection of TCR attached to or in a solid medium. Though detection of individual TCRs may not be possible when TCR is in solution.

**Indirect detection of TCR**

Indirect detection of TCR is primarily usefull for detection of TCRs embedded in lipid bilayer, preferably natural occurring T cells, T cell hybridomas or transfected T cells. MHC multimers used for the indirect detection may be generated and then added to sample. Alternatively MHC multimers are generated in sample by addition of antigenic peptide and/or antigenic polypeptide to sample and optionally also addition of MHC molecules, components of MHC molecules or MHC molecules coupled to carrier. For example when antigenic peptide and/or antigenic polypeptide is added to a sample containing antigen presenting cells, the antigenic peptide and/or antigenic polypeptide are taken up by antigen presenting cells in sample, processed inside cells and displayed on their surface by binding MHC molecules, or the antigenic peptides added are bound directly to MHC molecules displayed on the surface of antigen presenting cells by exchange with peptide already present in the peptide binding cleft of the MHC molecules.

In indirect detection, the number or activity of T cells are measured, by detection of events that are the result of TCR-MHC-peptide complex interaction. Interaction between MHC multimer and T cell may stimulate the T cell resulting in activation of T cells, in cell division and proliferation of T cell populations or alternatively result in inactivation of T cells. All these mechanism can be measured using various detection methods.

**Indirect detection of T cells by measurement of activation.**

MHC multimers, e.g. antigen presenting cells, can stimulate T cells resulting in activation of the stimulated T cells. Activation of T cell can be detected by measurement of production of specific soluble factor from the stimulated T cell, e.g. production of cytokines like INFγ and IL2. Stimulation of T cells can also be detected by measurement of changes in expression of specific surface receptors, or by measurement of T cell effector functions.

Measurement of activation of T cells involves the following steps:

a) Antigenic peptide is added to a sample of T cells containing antigen presenting cells, preferably a suspension of cells e.g. blood. The antigenic peptide have to be able to bind MHC I or MHC II molecules of one or more antigen presenting cells in the sample thereby generating one or more cell based MHC multimer(s) in sample. Alternatively, antigenic polypeptide protein or protein fragment containing one or more antigenic peptides sequences is added to such sampe. The protein/protein fragmentsantigenic polypeptide is then taken up by antigen presenting cells in sample, processed into antigenic peptides and presented by MHC I or MHC II molecules on the surface of antigen presenting cells thereby creating cell based MHC multimers in the sample. Several different antigenic peptides or antigenic proteins polypeptides may be added to the sample. The peptide-loaded antigen presenting cells (the cell based MHC multimers) can then stimulate specific T cells in sample, and thereby induce the production of soluble factor, up- or down- regulation of surface receptors, or mediate other changes in the T cell, e.g. enhancing effector functions.

Alternatively, one or more MHC multimer(s) containing one or more antigenic peptide(s) are added to a sample containing T cells, preferably a suspension of cells, to stimulate MHC multimer specific T cells in sample and thereby induce production of soluble factor, up- or down-regulation of surface receptor and/or other changes in the T cell.

Following addition of antigenic peptide, antigenic proteinantigenic polypeptide or MHC multimer to sample, a second soluble factor, e.g. cytokine and/or growth factor(s) may optionally be added to facilitate continued activation and expansion of antigen-specific T cells

b) Detection of the presence of produced soluble factor, the presence/ absence of surface receptor or detection of effector function.

Correlate the measured result with presence of T cells. The measured signal/response indicates the presence of specific T cells that have been stimulated with particular MHC multimer. The signal/response of a T lymphocyte population is a measure of the overall response in sample.

The frequency of specific T cells able to respond to a given MHC multimer can be determined by including a limiting-dilution culture in the assay also called a Limiting dilution assay.

The limiting-dilution culture method involves the following steps:

i. Sample of T cells in suspension are plated into culture wells at increasing dilutions.

ii. Antigen presenting cells are provided into the sample if not already in sample and then antigenic peptide or protein polypeptide containing one or more antigenic peptide sequence(s) is added to the sample as described above thereby creating cell based MHC multimers in sample able to stimulate antigen-specific T cells in the sample. Alternatively, already generated MHC multimers are added to sample to stimulate specific T cells. Optionally growth factors, cytokines or other factors helping T cells to proliferate are added.

iii. Cells are allowed to grow and proliferate (½- several days). Each well that initially contained a specific T cell will make a response to the MHC multimer and divide.

iv. Wells are tested for a specific response e.g. production of soluble factors, cell proliferation, cytotoxicity or other effector functions.

The assay is replicated with different numbers of T cells in the sample, and each well that originally contained a specific T cell will make a response to the MHC multimer. The frequency of specific T cells in the sample equals the reciprocal of the number of cells added to each well when 37% of the wells are negative, because due to Poisson distrubtion each well then on average contained one specific T cell at the beginning of the culture. Optionally step i) and ii) from above maybe reversed, e.g. adding sample containing T cells in various dilutions to wells or containers containing MHC multimer, antigenic peptide, antigenic peptide + antigen presenting cells, antigenic polypeptide or antigenic polypeptide+ antigen presenting cells or MHC multimer..

In the following various methods to measure production of specific soluble factor, expression of surface receptors, effector functions or proliferation is described.

Indirect detection of T cells by measurement of production of soluble factors.

*Indirect detection of T cells by measurement of secreted soluble factors.*

Secreted soluble factors can be measured directly in fluid suspension or the soluble factor captured by immobilization on solid support and then detected or an effect of the secreted soluble factor can be detected.

Examples of such detection methods are interferon gamma release assays (IGRA's) like Quantiferon, enzyme-linked immunospot (ELISPOT) and cytokine flow cytometry (CFC), where INF-γ released from antigen stimulated T cells are measured. Principles of the various and alternative assays are described in more details below.

○ *Indirect detection of T cells by measurement of secreted soluble factor directly in fluid sample.*
A sample of T cells are added MHC multimer or antigenic peptideMHC multimer and/or antigenic peptide and/or antigenic polypeptide as described above to induce production and secretion of soluble factors from antigen-specific T cells. The secreted soluble factors can be measured directly in the supernatant using e.g. mass spectrometry.
○ *Indirect detection of T cells by capture of secreted soluble factor on solid support.*
A sample of T cells are added MHC multimer and/or, antigenic peptide and/or antigenic proteinantigenic polypeptide as described above to induce production and secretion of soluble factors from antigen-specific T cells. Secreted soluble factors in the supernatant are then immobilized on a solid support either directly or through a linker as described for immobilization of T cells elsewhere herein. Then immobilized soluble factors can be detected using labeled marker molecules.

Soluble factors secreted from **individual** T cells can be detected using ELISPOT assays or related techniques. The principle is capturing of the secreted soluble factors locally by marker molecules, e.g antibodies specific for the soluble factor. Soluble factor recognised by marker molecules are immobilised on a solid support together with T cells and soluble factors secreted by individual T cells are thereby captured in the proximity of each T cell. Bound soluble factor can then be measured using labelled marker molecules specific for the captured soluble factor. The number of T cells that has given rise to labelled spots on solid support can then be enumerated and these spots indicate the presence of specific T cells that have been stimulated with particular MHC multimer and/or antigenic peptide and/or antigenic polypeptide.

Soluble factors secreted from **a population** of T cells are detected by capture and detection of soluble factor secreted from the entire population of specific T cells. In this case soluble factor do not have to be captured locally close to each T cell but the secreted soluble factors my be captured and detected in the same well or container as where the T cells are, or supernatant containing secreted soluble factor transferred to another solid support with marker molecules for capture e.g. beads or wells of ELISA plate. An example of such an assay is QuantiFERON or QuantiFERON like assays measuring secretion of INF-γ from antigen stimulated T cells.
○ *Indirect detection of T cells immobilized to solid support in a defined pattern.*
Different MHC multimers, or MHC-peptide complexes are immobilized to a support to form a spatial array in a defined pattern, where the position specifies the identity of the MHC multimer/MHC-peptide complex immobilized at this position. Marker molecules able to bind T cell secreted soluble factors are co-spotted together with MHC multimer/MHC-peptide complex. Such marker molecules can e.g. be antibodies specific for cytokines like INFγ or IL-2. The immobilization may be direct or through a linker molecule as described above.

Then a suspension of labeled T cells are added or passed over the array of MHC multimers/MHC-peptide complexes and specific T cells will bind to the immobilized MHC multimers/MHC-peptide complexes and upon binding be stimulated to secrete soluble factors e.g. cytokines like INFγ ord IL-2. Soluble factors secreted by individual T cells are then captured in the proximity of each T cell and bound soluble factor can be measured using labelled marker molecule specific for the soluble factor. The number and position of different specific T cells that has given rise to labelled spots on solid support can then be identified and enumerated. In this way T cells bound to defined areas of the support are analyzed, thereby, phenotyping the sample. Each individual T cell is defined by the TCR it expose and depending on these TCRs each entity will bind to different types of MHC multimers/MHC-peptide complexes immobilized at defined positions on the solid support.

Alternatively to MHC multimers or MHC-peptide comlexes antigenic peptides or antigenic polypeptides can be immobilized to a support to form a spatial array in a defined pattern, where the position specifies the identity of the antigenic peptide/antigenic polypeptide immobilized. As described above marker molecules able to bind T cell secreted soluble factors are co-spotted together with antigenic peptide/antigenic polypeptide. Before or together with addition of the suspension of T cells, MHC molecules, components of MHC molecules or MHC molecules attached to carrier are added to the array thereby generating MHC multimers. Then antigen specific T cells in sample are detected as described above.

◦ *Indirect detection of T cells by measurement of secreted soluble factor on surface of T cell*

An alternative way to detect secretion of soluble factor from individual cells is to use soluble factor capture on the surface of the T cell secreting the soluble factor. This can be done by using a bispecific capture molecule able to bind a component on the surface of the T cell with one part of the capture molecule and bind the secreted soluble factor by another part of the capture molecule. Example useful capture molecules are bispecific antibodies in which two different heavy- and light chain pairs from different antibodies are combined in one antibody resulting in an antibody molecule with the two antigen-binding sites recognizing different ligands.

Activated T cells in a sample can then be detected by adding the bispecific capture molecules to the sample. These molecules will then bind all T cells with on part of the molecule. T cells secreting soluble factor (due to activation) will then capture the secreted soluble factor on their surface by the soluble factor binding part of the capture molecule. Bound soluble factor can then be detected by addition of a labelled marker molecule specific for the soluble factor in question.

◦ *Indirect detection of T cells by measurement of effect of secreted soluble factor.*

Secreted soluble factors can be measured and quantified indirectly by measurement of the effect of the soluble factor on other cell systems. Briefly, a sample of T cells are added MHC multimer or antigenic peptideMHC multimer and/or antigenic peptide and/or antigenic polypeptide as described above to induce secretion of soluble factors from antigen-specific T cells. The supernatant containing secreted soluble factor are transferred to another cell system and the effect measured. The soluble factor may induce proliferation, secretion of other soluble factors, expression/downregulation of receptors, or the soluble factor may have cytotoxic effects on these other cells,. All effects can be measured as described elsewhere herein.

*Indirect detection of T cells by measurement of produced soluble factors intracellularly*

Production of soluble factors can be measured intracellularly using flow cytometry. Often cytokines are measured and the method is therefore referred to as cytokine flow cytometry (CFC). The principles are described below.

Soluble factor production by stimulated T cells can also be measured intracellular by e.g. flow cytometry. This can be done using block of secretion of soluble factor (e.g. by monensin), permeabilization of cell (by e.g. saponine) followed by immunofluorescent staining. The method involves the following steps: 1) Stimulation of T cells e.g. by binding specific MHC multimers: The MHC multimers may be generated and added to sample containing T cells or antigenic peptid or protein polypeptide containing antigenic peptide sequences can be added to sample and MHC multimers generated in sample as described elsewhere herein. An example of usefull MHC multimers for stimulation of specific T cells is antigen presenting cells displaying MHC molrecules containing antigenic peptide. A reagent able to block extracellular secretion of cytokine is added during stimulation, e.g. monensin that interrupt intracellular transport processes leading to accumulation of produced soluble factor, e.g. cytokine in the Golgi complex. Other soluble factors may be added to the T cell sample during stimulation to enhance activation and/or expansion .This other soluble factor can be cytokine and or growth factors. 2) addition of one or more labelled marker molecules able to detect special surface receptors (e.g. marker molecules able to bind CD8, CD4, CD3, CD27, CD28, CD2). 3) Fixation of cell membrane using mild fixator followed by permeabilization of cell membrane e.g. by saponine. 4) Addition of labelled marker specific for the produced soluble factor to be determined, e.g. INFγ, IL-2, IL-4, IL-10. 5) Measurement of labelled cells using a flow cytometer.

An alternative to this procedure is to trap secreted soluble factors on the surface of the secreting T cell as described elsewhere herein or as described by Manz, R. et al., Proc. Natl. Acad. Sci. USA 92:1921 (1995).

Indirect detection of T cells by measurement of expression of receptors

Activation of T cells can be detected by measurement of expression and/or down regulation of specific surface receptors. The method includes the following steps. A sample of T cells are added MHC multimer, antigenic peptide or protein containinng antigenic peptide as described elsewhere hereinantigenic polypeptide to stimulate T cell and thereby induce expression or downregulation of specific surface receptors on antigen-specific T cells.These receptors include but are not limited to CD28, CD27, CCR7, CD45RO, CD45RA, IL2-receptor, CD62L, CCR5. Their expression level can be detected by addition of labelled marker specific for the desired receptor and then measure the amount of labelt cells using flow cytometry, microscopy, immobilization of activated T cell on solid support or any other method like those decribed for direct detection of TCR.

## Indirect detection of T cells by measurement of effector function

Activation of T cells can be detected indirectly by measurement of effector functions. A sample of T cells are added MHC multimer, antigenic peptide or antigenic polypeptide protein containing antigenic peptide as described elsewhere herein to stimulate T cell and thereby induce one or more effector functions of the antigen-specific T cells. The one or more effector function(s) are then measured. For example activation of antigen-specific CD8 positive T cells can be determined by measurement of killing of target cells, i.e. cells displaying specific MHC-peptide complexes recognized by the activated antigen-specific CD8 positive T cell. This method is often referred to as cytotoxicity assays or CTL killing assays and involves the following steps:
1) Sample containing antigen-specific CD8 positive cells are stimulated by addition of MHC multimer, antigenic peptide or antigenic polypeptideprotein containing antigenic peptide as described elsewhere herein. 2) Another sampe containing live target cells displaying MHC I molecules containing specific antigenic peptide are added labelled molecules that can be taken up by live cells but that are not spontaneously released by the target cells following uptake e.g. radioactive labelled compounds. 3) Stimulated and activated T cells from step 1 are then added to target cells of step 2. target cells displaying the MHC complexes containing specific antigenic peptide(s) are then killed releasing labelled compound from the target cells and the presence of this labelled compound may be detected in the supernatant of mixtures of target and cytoxic cells. Alternatively, amount of labelled compound in cells that are not killed by the CD8 positive T cells are measured, by removing labelled compound released by killed target cells followed by measurement of label inside remaining cells either directly or by release of the labelled compund from these remaining cells.

## Indirect detection of T cells by measurement of proliferation

T cells can be stimulated to proliferate upon binding specific MHC multimers. Proliferation of T cells can be measured several ways including but not limited to:

◦ Detection of mRNA

Proliferation of T cells can be detected by measurement of mRNA inside cell. Cell division and proliferation requires production of new protein in each cell which as an initial step requires production of mRNA encoding the proteins to be synthesized.

A sample of T cells are added MHC multimer or antigenic peptideMHC multimer and/or antigenic peptide and/or antigenic polypeptide as described above to induce proliferation of antigen-specific T cells. Detection of levels of mRNA inside the proliferating T cells can be done by quantitative PCR and indirectly measure activation of a T cell population as a result of interaction with MHC multimer. An example is measurement of cytokine mRNA by in sity hybridization.

◦ Detection of incorporation of thymidine

The proliferative capacity of T cells in response to stimulation by MHC multimer can be determined by a radioactive assay based on incorporation of [3H]thymidine ([3H]TdR) into newly generated DNA followed by measurement of radioactive signal.

◦ Detection of incorporation of BrdU

T cell proliferation can also be detected by of incorporation of bromo-2'-deoxyuridine (BrdU) followed by measurement of incorporated BrdU using a labeled anti-BrdU antibody in an ELISA based analysis.

Viability of cells may be measured by measurement ATP in a cell culture.

## Indirect detection of T cells by measurement of inactivation

Not all MHC multimers will lead to activation of the T cells they bind. Under certain circumstances some MHC multimers may rather inactivate the T cells they bind to.

## Indirect detection of T cells by measurement of effect of blockade of TCR

Inactivation of T cells by MHC multimers may be measured be measuring the effect of blocking TCR on antigen-specific T cells. MHC multimers, e.g. MHC-peptide complexes coupled to IgG scaffold can block the TCR of an antigen-specific T cell by binding the TCR, thereby prevent the blocked T cell receptor interacting with e.g. antigen presenting cells. Blockade of TCRs of a T cell can be detected in any of the above described medthods for detection of TCR by addition of an unlabeled blocking MHC multimer together with the labelled MHC multimer and then measuring the effect of the blockade on the readout.

Indirect detection of T cells by measurement of induction of apoptosis

Inactivation of T cells by MHC multimers may be measured be measuring apoptosis of the antigen-specific T cell. Binding of some MHC multimers to specific T cells may lead to induction of apoptosis. Inactivation of T cells by binding MHC multimer may therefore be detected by measuring apoptosis in the T cell population. Methods to measure apoptosis in T cells include but are not limited to measurement of the following:

- DNA fragmentation
- Alterations in membrane asymmetry (phosphatidylserine translocation)
- Activation of apoptotic caspases
- Release of cytochrome C and AIF from mitochondria into the cytoplasm

**Positive control experiments for the use of MHC multimers in flow cytometry and related techniques**

When performing flow cytometry experiments, or when using similar technologies, it is important to include appropriate positive and negative controls. In addition to establishing proper conditions for the experiments, positive and negative control reagents can also be used to evaluate the quality (e.g. specificity and affinity) and stability (e.g. shelf life) of produced MHC multimers.

The quality and stability of a given MHC multimer can be tested in a number of different ways, including:

- Measurement of specific MHC multimer binding to beads, other types of solid support, or micelles and liposomes, to which TCR's have been immobilized. Other kinds of molecules that recognize specifically the MHC-peptide complex can be immobilized and used as well. Depending on the nature of the solid support or membrane structure to which the TCR is immobilized, the TCR can be full-length (i.e. comprise the intracellular- and intra-membrane domains), or can be truncated (e.g. only comprise the extracellular domains). Likewise, the TCR can be recombinant, and can be chemically or enzymatically modified.

- Measurement of MHC multimer binding to beads, other types of solid support, or micelles and liposomes, to which aptamers, antibodies or other kinds of molecules that recognize correctly folded MHC-peptide complexes have been immobilized.

- Measurement of specific MHC multimer binding to specific cell lines (e.g. T-cell lines) displaying MHC multimer-binding molecules, e.g. displaying TCRs with appropriate specificity and affinity for the MHC multimer in question.

- Measurement of specific MHC multimer binding to cells in blood samples, preparations of purified lymphocytes (HPBMCs), or other bodily fluids that contain cells carrying receptor molecules specific for the MHC multimer in question.

- Measurement of specific MHC multimer binding to soluble TCRs, aptamers, antibodies, or other soluble MHC-peptide complex-binding molecules, by density-gradient centrifugation (e.g. in CsCl) or by size exclusion chromatography, PAGE or other type of chromatographic method.

Measurement of specific MHC binding to TCRs, aptamers, antibodies, streptavidin, or other MHC-peptide complex-binding molecules immobilized on a solid surface (e.g. a microtiter plate). The degree of MHC multimer binding can be visualized with a secondary component that binds the MHC multimer, e.g. a biotinylated fluorophore in cases where the MHC multimer contains streptavidin proteins, not fully loaded with biotin. Alternatively, the secondary component is unlabelled, and a labelled second component-specific compound is employed (e.g. EnVision System, Dako) for visualization. This solid surface can be beads, immunotubes, microtiterplates act. The principle for purification are basically the same I.e. T cells are added to the solid with immobilized MHC'mer, non-binding T cells are washed away and MHC-peptide specific T cells can be retrieved by elution with mild acid or a competitive binding reagent.

- Measurement of specific MHC multimer binding to TCRs, aptamers, antibodies, streptavidin, or other MHC-peptide complex-binding molecules immobilized on a solid surface (e.g. a microtiter plate) visualized with a secondary component specific to MHC multimer (e.g. TCRs, aptamers, antibodies, streptavidin, or other MHC-peptide binding complex-binding molecules). Alternatively the secondary receptor is unlabelled, and a labelled second receptor-specific compound is employed (e.g. EnVision System, Dako) before visualization.

In the above mentioned approaches, positive control reagents include MHC multimers comprising correctly folded MHC, complexed with an appropriate peptide that allows the MHC multimer to interact specifically and efficiently with its cognate TCR. Negative control reagents include empty MHC multimers, or correctly folded MHC multimers complexed with so-called nonsense peptides that support a correct conformation of the MHC-peptide complex, but that do not efficiently bind TCRs through the peptide-binding site of the MHC complex.

**Negative control reagents and negative control experiments for the use of MHC multimers in flow cytometry and related techniques**

Experiments with MHC multimers require a negative control in order to determine background staining with MHC multimer. Background staining can be due to unwanted binding of any of the individual components of the MHC multimer, e.g., MHC complex or individual components of the MHC complex, multimerization domain or label molecules. The unwanted binding can be to any surface or intracellular protein or other cellular structure of any cell in the test sample, e.g. undesired binding to B cells, NK cells or T cells. Unwanted binding to certain cells or certain components on cells can normally be corrected for during the analysis, by staining with antibodies that bind to unique surface markers of these specific cells, and thus identifies these as false positives, or alternatively, that bind to other components of the target cells, and thus identifies these cells as true positives. A negative control reagent can be used in any experiment involving MHC multimers, e.g. flow cytometry analysis, other cytometric methods, immunohistochemistry (IHC) and ELISA. Negative control reagents include the following:

- MHC complexes or MHC multimers comprising MHC complexes carrying nonsense peptides. A nonsense peptide is here to be understood as a peptide that binds the MHC protein efficiently, but that does not support binding of the resultant MHC-peptide complex to the desired TCR. An example nonsense peptide is a peptide with an amino acid sequence different from the linear sequence of any peptide derived from any known protein. When choosing an appropriate nonsense peptide the following points are taken into consideration. The peptide should ideally have appropriate amino acids at relevant positions that can anchor the peptide to the peptide-binding groove of the MHC. The remaining amino acids should ideally be chosen in such a way that possible binding to TCR (through interactions with the peptide or peptide-binding site of MHC) are minimized. The peptide should ideally be soluble in water to make proper folding with MHC alpha chain and β2m possible in aqueous buffer. The length of the peptide should ideally match the type and allele of MHC complex. The final peptide sequence should ideally be taken through a blast search or similar analysis, to ensure that it is not identical with any peptide sequence found in any known naturally occurring proteins.

- MHC complexes or MHC multimers comprising MHC complexes carrying a chemically modified peptide in the peptide-binding groove. The modification should ideally allow proper conformation of the MHC-peptide structure, yet should not allow efficient interaction of the peptide or peptide-binding site of MHC with the TCR.

- MHC complexes or MHC multimers comprising MHC complexes carrying a naturally occurring peptide different from the peptide used for analysis of specific T cells in the sample. When choosing the appropriate natural peptide the following should be taken into consideration. The peptide in complex with the MHC protein should ideally not be likely to bind a TCR of any T cell in the sample with such an affinity that it can be detected with the applied analysis method. The peptide should ideally be soluble in water to make proper folding with MHC alpha chain and β2m possible in aqueous buffer. The length of the peptide should match the type and allele of MHC complex.

- Empty MHC complexes or MHC multimers comprising empty MHC complexes, meaning any correctly folded MHC complex without a peptide in the peptide-binding groove.

- MHC heavy chain or MHC multimers comprising MHC heavy chain, where MHC heavy chain should be understood as full-length MHC I or MHC II heavy chain or any truncated version of MHC I or MHC II heavy chain. The MHC heavy chains can be either folded or unfolded. Of special interest is MHC I alpha chains containing the α3 domain that binds CD8 molecules on cytotoxic T cells. Another embodiment of the present disclosure of special interest is MHC II β chains containing the β2 domain that binds CD4 on the surface of helper T cells.

- Beta2microglobulin or subunits of beta2microglobulin, or MHC multimers comprising Beta2microglobulin or subunits of beta2microglobulin, folded or unfolded.

- MHC-like complexes or MHC multimers comprising MHC-like complexes, folded or unfolded. An example could be CD1 molecules that are able to bind peptides in a peptide-binding groove that can be recognized by T cells (Russano et al. (2007). CD1-restricted recognition of exogenous and self-lipid antigens by duodenal gammadelta+ T lymphocytes. J Immunol. 178(6):3620-6)

- Multimerization domains without MHC or MHC-like molecules, e.g. dextran, streptavidin, IgG, coiled-coil-domain liposomes.

- Labels, e.g. FITC, PE, APC, pacific blue, cascade yellow, or any other label listed elsewhere herein.

Negative controls 1-4 can provide information about potentially undesired binding of the MHC multimer, through interaction of a surface of the MHC-peptide complex different from the peptide-binding groove and its surroundings. Negative control 5 and 6 can provide information about binding through interactions through the MHC I or MHC II proteins (in the absence of peptide). Negative control 7 can provide information about binding through surfaces of the MHC complex that is not unique to the MHC complex. Negative controls 8 and 9 provide information about pontential undesired interactions between non-MHC-peptide complex components of the MHC multimer and cell constituents.

## Minimization of undesired binding of the MHC multimer

Identification of MHC-peptide specific T cells can give rise to background signals due to unwanted binding to cells that do not carry TCRs. This undesired binding can result from binding to cells or other material, by various components of the MHC multimer, e.g. the dextran in a MHC dextramer construct, the labelling molecule (e.g. FITC), or surface regions of the MHC-peptide complex that do not include the peptide and the peptide-binding cleft. MHC-peptide complexes bind to specific T cells through interaction with at least two receptors in the cell membrane of the T-cell. These two receptors are the T-cell receptor (TCR) and CD8 for MHC I-peptide complexes and TCR and CD4 receptor protein for MHC II-peptide complexes. Therefore, a particularly interesting example of undesired binding of a MHC multimer is its binding to the CD8 or CD4 molecules of T cells that do not carry a TCR specific for the actual MHC-peptide complex. The interaction of CD8 or CD4 molecules with the MHC is not very strong; however, because of the avidity gained from the binding of several MHC complexes of a MHC multimer, the interaction between the MHC multimer and several CD8 or CD4 receptors potentially can result in undesired but efficient binding of the MHC multimer to these T cells. In an analytical experiment this would give rise to an unwanted background signal; in a cell sorting experiment undesired cells might become isolated. Other particular interesting examples of undesired binding is binding to lymphoid cells different from T cells, e.g. NK-cells, B-cells, monocytes, dendritic cells, and granulocytes like eosinophils, neutrophils and basophiles.
Apart from the MHC complex, other components in the MHC multimer can give rise to unspecific binding. Of special interest are the multimerization domain, multimerization domain molecules, and labelling molecules.

One way to overcome the problem with unwanted binding is to include negative controls in the experiment and subtract this signal from signals derived from the analyzed sample, as described elsewhere in the disclosure.

Alternatively, unwanted binding could be minimized or eliminated during the experiment. Methods to minimize or eliminate background signals include:

- Mutations in areas of the MHC complex responsible for binding to unwanted cells can be introduced. Mutations here mean substitution, insertion, or deletion of natural or non-natural amino acids. Sub-domains in the MHC complex can be responsible for unwanted binding of the MHC multimer to cells without a TCR specific for the MHC-peptide complex contained in the MHC multimer. One example of special interest is a small region in the α3-domain of the α-chain of MHC I molecules that is responsible for binding to CD8 on all cytotoxic T cells. Mutations in this area can alter or completely abolish the interaction between CD8 on cytotoxic T cells and MHC multimer (Neveu et al. (2006) Int Immunol. 18, 1139-45). Similarly a sub domain in the β2 domain of the β-chain of MHC II molecules is responsible for binding CD4 molecules on all CD4 positive T cells. Mutations in this sub domain can alter or completely abolish the interaction between MHC II and CD4.
Another embodiment of the present disclosure is to mutate other areas of MHC I /MHC II complexes that are involved in interactions with T cell surface receptors different from TCR, CD8 and CD4, or that bind surface receptors on B cells, NK cells, Eosiniophils, Neutrophils, Basophiles, Dendritic cells or monocytes.

- Chemical alterations in areas of the MHC complex responsible for binding to unwanted cells can be employed in order to minimize unwanted binding of MHC multimer to irrelevant cells. Chemical alteration here means any chemical modification of one or more amino acids. Regions in MHC complexes that are of special interest are as mentioned above the α3 domain of the α-chain in MHC I molecules and β2 domains in the β-chain of MHC II molecules. Other regions in MHC I /MHC II molecules that can be chemically modified to decrease the extent of undesired binding are regions involved in interaction with T cell surface receptors different from TCR, CD8 and CD4, or that bind surface receptors on B cells, NK cells, Eosiniophils, Neutrophils, Basophiles, Dendritic cells or monocytes.

- Another method to minimize undesired binding involves the addition of one or more components of a MHC multimer, predicted to be responsible for the unwanted binding. The added component is not labeled, or carries a label different from the label of the MHC multimer used for analysis. Of special interest is addition of MHC multimers that contain nonsense peptides, i.e. peptides that interact efficiently with the MHC protein, but that expectably do not support specific binding of the MHC multimer to the TCR in question. Another example of interest is addition of soluble MHC complexes not coupled to a multimerization domain, and with or without peptide bound in the peptide binding cleft. In another embodiment of the present disclosure, individual components of the MHC complex can be added to the sample, e.g. I $\alpha$-chain or subunits of MHC I $\alpha$-chain either folded or unfolded, beta2microglobulin or subunits thereof either folded or unfolded, $\alpha/\beta$-chain of MHC II or subunits thereof either folded or unfolded. Any of the above mentioned individual components can also be attached to a multimerization domain identical or different from the one used in the MHC multimer employed in the analysis.

  Of special interest is also addition of multimerization domain similar or identical to the multimerization domain used in the MHC multimer or individual components of the multimerization domain.

- Reagents able to identify specific cell types either by selection or exclusion can be included in the analysis to help identify the population of T cells of interest, and in this way deselect the signal arising from binding of the MHC multimer to undesired cells.

  Of special interest is the use of appropriate gating reagents in flow cytometry experiments. Thus, fluorescent antibodies directed against specific surface markers can be used for identification of specific subpopulations of cells, and in this way help to deselect signals resulting from MHC multimers binding to undesired cells.

  Gating reagents of special interest that helps identify the subset of T cells of interest when using MHC I multimers are reagents binding to CD3 and CD8 identifying all cytotoxic T cells. These reagents are preferably antibodies but can be any labeled molecule capable of binding CD3 or CD8. Gating reagents directed against CD3 and CD8 are preferably used together. As they stain overlapping cell populations they are preferably labeled with distinct fluorochromes. However, they can also be used individually in separate samples. In experiments with MHC II multimers reagents binding to CD3 and CD4 identifying T helper cells can be used.

  These reagents are preferably antibodies but can be any labeled molecule capable of binding CD3 or CD4. Gating reagents directed against CD3 and CD4 are preferable used together. As they stain overlapping cell populations they are preferably labeled with distinct fluorochromes. However, they can also be used individually in separate samples.

Other gating reagents of special interest in experiments with any MHC multimer, are reagents binding to the cell surface markers CD2, CD27, CD28, CD45RA, CD45RO, CD62L and CCR7. These surface markers are unique to T cells in various differentiation states. Co staining with either of these reagents or combinations thereof together with MHC multimers helps to select MHC multimer binding T cells expressing a correct TCR. These reagents can also be combined with reagents directed against CD3, CD4 and/or CD8.

Another flow cytometric method of special interest to remove signals from MHC multimer stained cells not expressing the specific TCR, is to introduce an exclusion gate. Antibodies or other reagents specific for surface markers unique to the unwanted cells are labeled with a fluorochrome and added to the test sample together with the MHC multimer. The number of antibodies or surface marker specific reagents are not limited to one but can be two, three, four, five, six, seven, eight, nine, ten or more individual reagents recognizing different surface markers, all of which are unique to the unwanted cells. During or after collection of data all events representing cells labeled with these antibodies are dumped in the same gate and removed from the dataset.

This is possible because all the antibodies/reagents that bind to the wrong cells are labeled with the same fluorochrome.

Reagents of special interest that exclude irrelevant cells include reagents against CD45 expressed on red blood cells, CD19 expressed on B cells, CD56 expressed on NK cells, CD4 expressed on T helper cells and CD8 expressed on cytotoxic T cells, CD14 expressed on monocytes and CD15 expressed on granulocytes and monocytes.

## Vaccine treatment

For the purpose of making vaccines it can be desirable to employ MHC multimers that comprise a polymer such as dextran, or that are cell-based (e.g. specialized dendritic cells such as described by Banchereau and Palucka, Nature Reviews, Immunology, 2005, vol. 5, p. 296-306).

□ Preventive vaccination leading to prophylaxis/sterile immunity by inducing memory in the immune system may be obtained by immunizing/vaccinating an individual or animal with MHC alone, or with MHC in combination with other molecules as mentioned elsewhere in the patent.

◦ Vaccine antigens can be administered alone
◦ Vaccine can be administered in combination with adjuvant(s).

□ Adjuvant can be mixed with vaccine component or administered alone, simultaneously or in any order.
□ Adjuvant can be administered by the same route as the other vaccine components

◦ Vaccine administered more than once may change composition from 1st administration to the 2nd, 3rd, etc.
◦ Vaccine administered more than once can be administered by alternating routes
◦ Vaccine components can be administered alone or in combinations by the same route or by alternating/mixed routes
◦ Vaccine can be administered by the following routes

□ Cutaneously
□ Subcutaneously (SC)
□ Intramuscular (IM)
□ Intravenous (IV)
□ Per-oral (PO)
□ Inter peritoneally
□ Pulmonally
□ Vaginally
□ Rectally
□

□ Therapeutic vaccination i.e. vaccination "teaching" the immune system to fight an existing infection or disease, may be obtained by immunizing/vaccinating an individual or animal with MHC alone, or with MHC in combination with other molecules as mentioned elsewhere in the patent.

◦ Vaccine antigens can be administered alone
◦ Vaccine can be administered in combination with adjuvant(s).

□ Adjuvant can be mixed with vaccine component or administered alone, simultaneously or in any order.
□ Adjuvant can be administered by the same route as the other vaccine components

◦ Vaccine administered more than once may change composition from 1st administration to the 2nd, 3rd, etc.
◦ Vaccine administered more than once can be administered by alternating routes
◦ Vaccine components can be administered alone or in combinations by the same route or by alternating/mixed routes
◦ Vaccine can be administered by the following routes

□ Cutaneously
□ Subcutaneously (SC)
□ Intramuscular (IM)
□ Intravenous (IV)

Per-oral (PO)
Inter peritoneally
Pulmonally
Vaginally
Rectally

## Therapeutic treatment

Therapeutic treatment includes the use of MHC molecules alone or in any molecular combination mentioned elsewhere in the present disclosure for the purpose of treating a disease in any state. Treatment may be in the form of

◦ Per-orally intake

Pills
Capsules

◦ Injections

Systemic
Local

◦ Jet-infusion (micro-drops, micro-spheres, micro-beads) through skin
◦ Drinking solution, suspension or gel
◦ Inhalation
◦ Nose-drops
◦ Eye-drops
◦ Ear-drops
◦ Skin application as ointment, gel or creme
◦ Vaginal application as ointment, gel, creme or washing
◦ Gastro-Intestinal flushing
◦ Rectal washings or by use of suppositories

Treatment can be performed as

◦ Single intake, injection, application, washing
◦ Multiple intake, injection, application, washing

On single day basis
Over prolonged time as days, month, years

Treatment dose and regimen can be modified during the course

**The variation in peptide epitope usage among individuals must be considered when developing personalized medicine based on antigenic peptides and/or MHC complexes.**

The immune system is very complex. Each individual has a very large repertoire of specific T cells (on the order of $10^6$-$10^9$ different T cell specificities, differing in the identity of the T cell receptor), which again is only a small subset of the total T cell repertoire of a population of individuals. It is estimated that the Caucasian population represents a T cell diversity of $10^{10}$-$10^{12}$.
The T cell receptor recognizes MHC peptide complexes, embedded in the cell membrane. Each individual has between 3 and 6 MHC I alleles and 3 and 8 MHC II alleles. Each of these MHC alleles forms complexes with short antigenic peptides generated by proteolytic degradation and prematurely terminated protein synthesis. Individuals of a population differ in their pattern of peptide degradation. The MHC allele diversity described above combined with this variation among individuals' proteolytic metabolism further enhances the variation among different individuals' immune responses. As a result, each individual has its own characteristic immune response profile, comprising its unique set of alleles and peptide combinations.
This is important when designing an antigenic peptide-based or a MHC multimer-based immune monitoring reagent or immunotherapeutic agent. If an agent is sought that should be generally applicable to the majority of individuals in a population, one should try to identify peptide epitopes and MHC alleles that are common to the majority of individuals of a population. As described elsewhere in this disclosure, such peptide epitopes can be identified through computerized search algorithms and/or experimental testing of a large set of individuals.
This approach will be advantageous in many cases, but because of the variability among immune response profiles of different individuals, is likely to be inefficient in certain individuals, because of these individuals' non-average profile. In these latter cases one may have to turn to personalized medicine. In the case of immune monitoring and immunotherapy, this may involve testing a large number of different epitopes from a given antigen, in order to find peptide epitopes that applies to the given individual.
When considering the patient population as a whole, a large fraction of the epitopes that theoretically may be generated from a given antigen, for use as a free antigenic peptide agent or to be included in a MHC I or MHC II multimer reagent, are therefore of relevance in personalized medicine. For the individual patient only a small fraction of these will be efficient; and in order to make generally applicable diagnostics, vaccines or therapeutics, even less epitopes are of relevance. Only in the case where one wants to generate a therapeutic agent or diagnostic reagent that is applicable to the majority of individuals of a population can the large majority of epitope sequences be said to be irrelevant, and those identified by computerized search algorithms and experimental testing be said to be the only epitopes of value. For the odd individual with the odd immune response these disregarded peptide epitopes may be the epitopes that provide an efficient diagnostic reagent or cures that individual from a deadly disease.

In conclusion, a large fraction of the theoretical epitopes that can be generated from an antigen are of great practical value for use in personalized diagnostics, vaccines and therapeutics.

## FIGURE LEGENDS

Figure 1. Schematic representation of MHC multimer. A MHC multimer consist of a multimerization domain whereto one or more MHC-peptide complexes are attached through one or more linkers. The multimerization domain comprice one or more carriers and/or one or more scaffolds. The MHC-peptide complexes comprice a peptide and a MHC molecule

Figure 2. Program for peptide sequence motifs prediction inhere called random prediction software.

Figure 3. Full List of HLA Class I alleles assigned as of January 2007 from http://www.anthonynolan.org.uk/HIG/lists/class1list.html

Figure 4. List of top 30 HLA class 1 alleles in different human ethnic groups

Figure 5. Reactive groups and the bonds formed upon their reaction

Figure 6. Cleavable linkers, conditions for cleaving them and the resulting products of the cleavage. Shown are cleavable linkers relevant for the present disclosure, as well as the conditions that lead to their cleavage, and the products of the cleavage reaction.

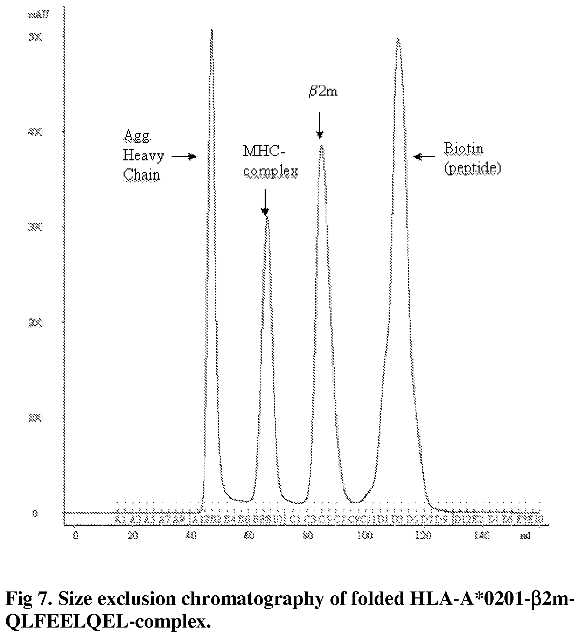

Figure 7. Size exclusion chromatography of folded HLA-A*0201-$\beta$2m -QLFEELQEL (SEQ ID NO 217775) peptide-complex. Purification of HLA-A*0201-$\beta$2m - QLFEELQEL (SEQ ID NO 217775) peptide-complex by size exclusion chromatography on a HiLoad 16/60 Superdex 75 column. Eluted protein was followed by measurement of the absorbance at 280 nm. The elution profile consisted of 4 peaks, corresponding to aggregated Heavy Chain, correctly folded MHC-complex, $\beta$2m and excess biotin and peptide.

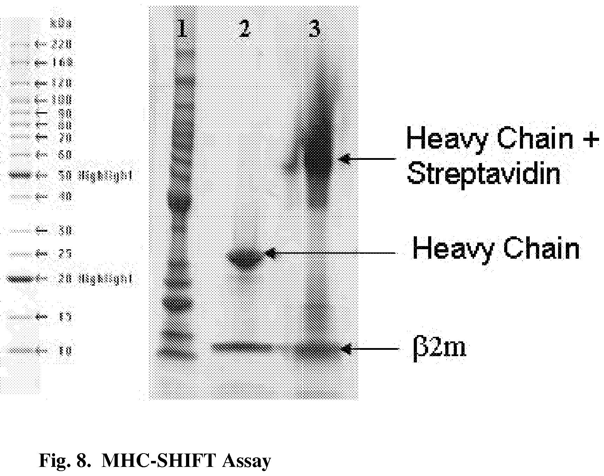

Figure 8. MHC-SHIFT Assay. The SHIFT Assay shows that heavy chain is efficiently biotinylated, since the band corresponding to biotinylated heavy chain (lane 2) is shifted up-wards upon incubation with streptavidin.

Lane 1: Benchmark protein-ladder
Lane 2: Folded HLA-A*0201-$\beta$2m -QLFEELQEL (SEQ ID NO 217775) peptide-complex
Lane 3: Folded HLA-A*0201-$\beta$2m -QLFEELQEL (SEQ ID NO 217775) peptide-complex incubated with molar excess Streptavidin.

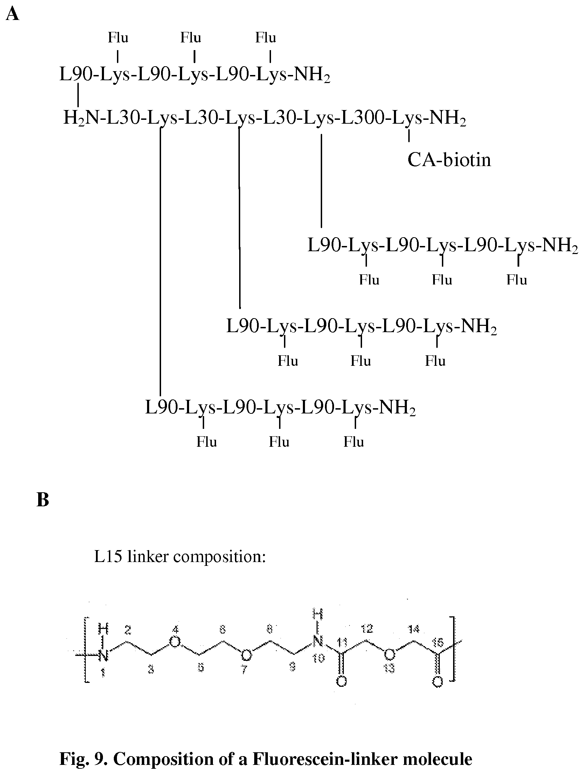

Figure 9. Composition of Fluorescein-linker molecule. (A) Schematic representation of an example of a Fluorescein-linker molecule. (B) Composition of a L15 linker.

Figure 10. List of the 24 MHC class 1 alleles used for peptide prediction by the database http://www.cbs.dtu.dk/services/NetMHC/ and the 14 MHC class 2 alleles used for peptide prediction by the database http://www.cbs.dtu.dk/services/NetMHCII/

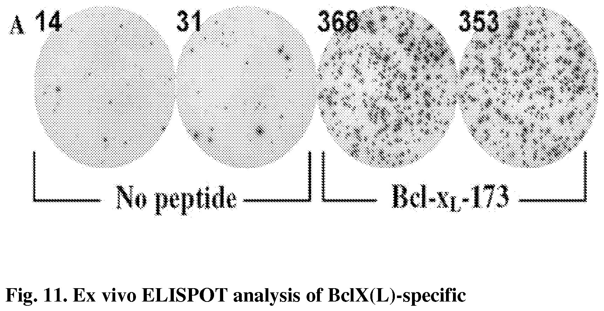

Figure 11. Ex vivo ELISPOT analysis of BclX(L)-specific. CD8 positive T cells in PBL from a breast cancer patient either with or without the BclX(L) YLNDHLEPWI peptide (SEQ ID NO 217776). Analysis were performed in doublets and number of IFN-gamma producing T-cells are presented. (Reference: Sorensen RB, Hadrup SR, Kollgaard T, Svane IM, Thor Straten P, Andersen MH (2006) Efficient tumor cell lysis mediated by a Bcl-X(L) specific T cell clone isolated from a breast cancer patient.Cancer Immunol Immunother Apr;56(4)527-33)

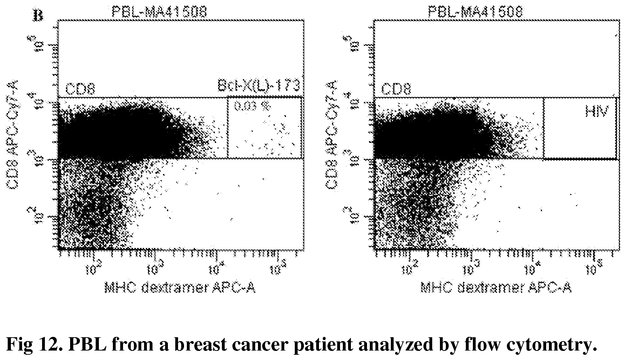

Figure 12. PBL from a breast cancer patient analyzed by flow cytometry. PBL from a breast cancer patient was analyzed by flow cytometry to identify Bcl-X(L)173-182 (peptide YLNDHLEPWI (SEQ ID NO 217776)) specific CD8 T cells using the dextramer complex HLA-A2/Bcl-X(L)173-182-APC, 7-AAD-PerCP, CD3-FITC, and CD8-APC-Cy7. The dextramer complex HLA-A2/HIV-1 pol476-484-APC was used as negative control.
(Reference: Sorensen RB, Hadrup SR, Kollgaard T, Svane IM, Thor Straten P, Andersen MH (2006) Efficient tumor cell lysis mediated by a Bcl-X(L) specific T cell clone isolated from a breast cancer patient.Cancer Immunol Immunother Apr;56(4)527-33)

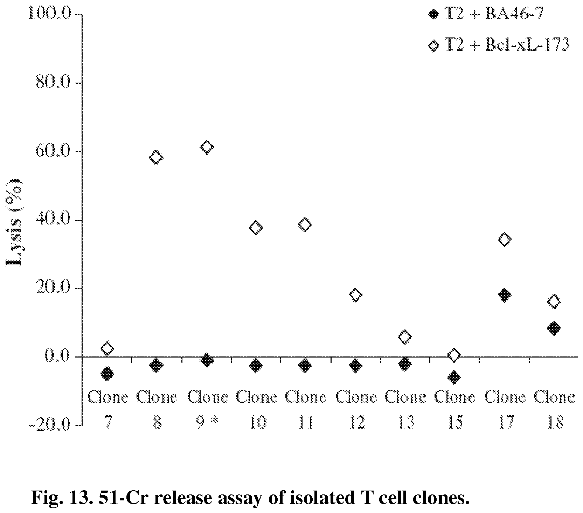

Figure 13. 51-Cr release assay of isolated T cell clones. Ten expanded T cell clones isolated by Flow sorting and then expanded were tested for their specificity by analysis in a standard 51-Cr release assay. For this purpose, T2 cells loaded with either Bcl-X(L)173-182, YLNDHLEPWI (SEQ ID NO 217776) peptide or an irrelevant peptide (BA4697-105, GLQHWVPEL (SEQ ID NO 217777)) were used as target cells.
(Reference: Sorensen RB, Hadrup SR, Kollgaard T, Svane IM, Thor Straten P, Andersen MH (2006) Efficient tumor cell lysis mediated by a Bcl-X(L) specific T cell clone isolated from a breast cancer patient.Cancer Immunol Immunother Apr;56(4)527-33)

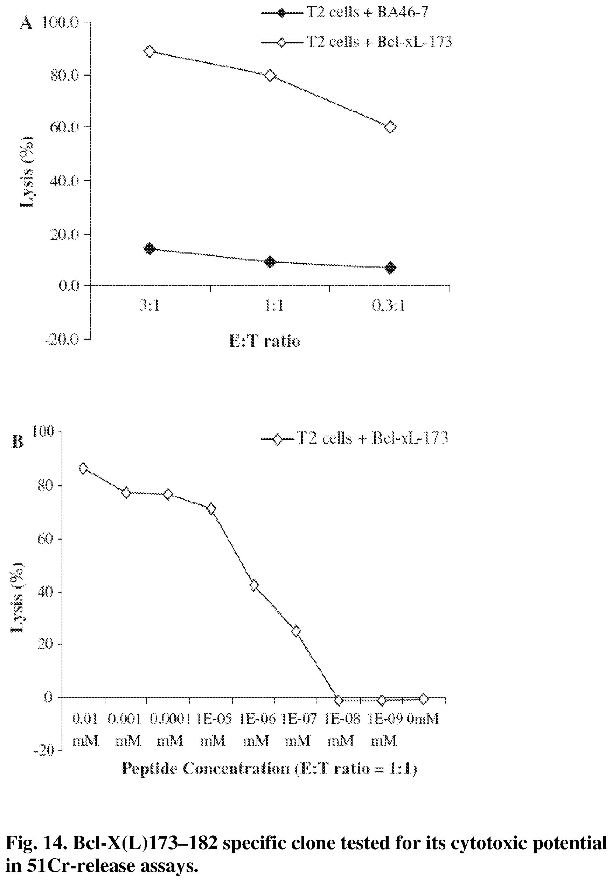

Figure 14. Bcl-X(L)173-182 specific clone tested for its cytotoxic potential in 51 Cr-release assays. A) Bcl-X(L)173-182 specific clone was tested for its cytotoxic potential in 51 Cr-release assays. Two assays were performed a Cell lysis of T2 cells pulsed with Bcl-X(L)173-182 peptide or an irrelevant peptide (BA4697-105, GLQHWVPEL (SEQ ID NO 217777)) in three E:T ratios. B) Cell lysis of T2 cells pulsed with different concentrations of Bcl-X(L)173-182 peptide at the E:T ratio 1:1. (Reference: Sorensen RB, Hadrup SR, Kollgaard T, Svane IM, Thor Straten P, Andersen MH (2006) Efficient tumor cell lysis mediated by a Bcl-X(L) specific T cell clone isolated from a breast cancer patient.Cancer Immunol Immunother Apr;56(4)527-33).

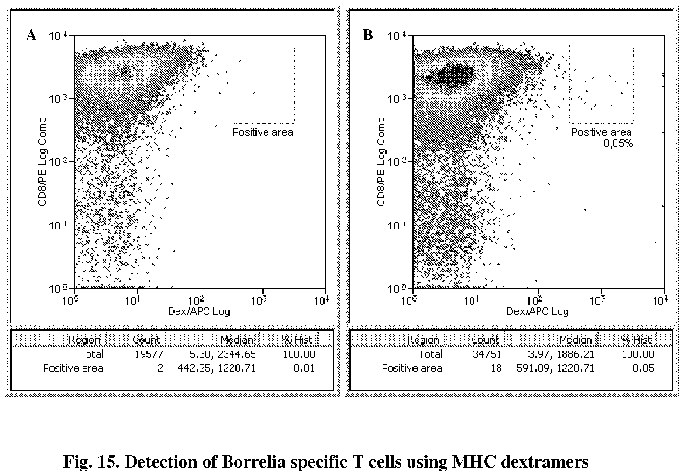

Figure 15. Detection of Borrelia specific T cells using MHC dextramers. Dot plots showing live gated CD3$^+$/CD4$^-$ lymphocytes from Borrelia patient stained with (A) Negative Control MHC Dextramer (HLA-A*0201(GLAGDVSAV) (SEQ ID NO 217778) or (B) pool of MHC Dextramers containing peptides from Borrelia antigen Osp A and Fla B. pool of MHC Dextramers containing peptides from Borrelia antigen. 0,05% of the live gated CD3$^+$/CD4$^-$ lymphocytes are positive for one or more of the MHC Dextramers in the pool.

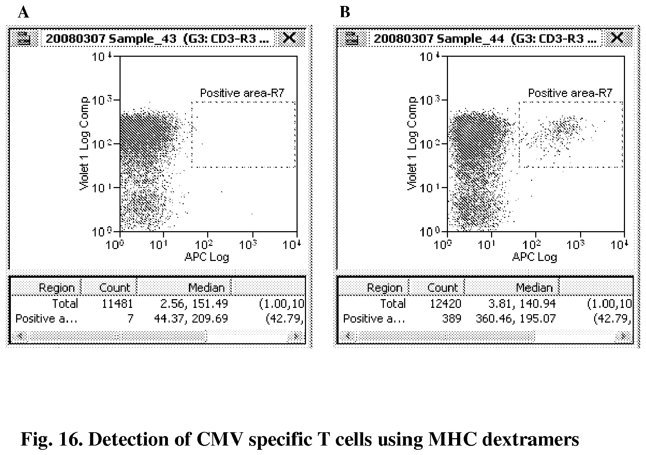

Figure 16. Detection of CMV specific T cells using MHC dextramers Dot plots showing live gated CD3$^+$/CD4$^-$ lymphocytes from CMV infected patient stained with (A) Negative Control MHC Dextramers (HLA-A*0201(GLAGDVSAV)) (SEQ ID NO 217778) or (B) MHC Dextramers containing peptides from CMV pp65 antigen (HLA-A*0201(NLVPMVATV)) (SEQ ID NO 217779).

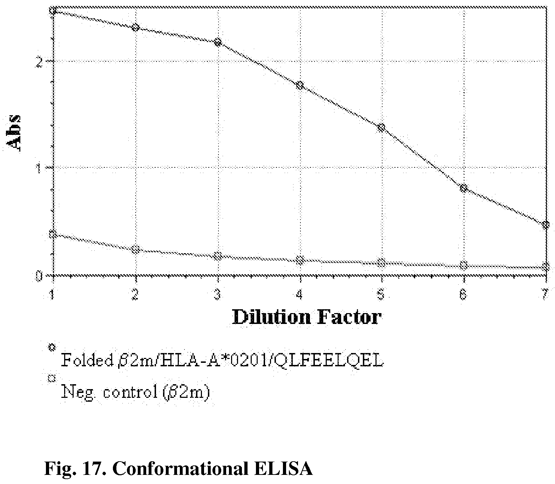

Figure 17. Conformational ELISA. The ELISA is carried out as a sandwich-ELISA. The ELISA-plate was coated with W6/32 mouse-anti-hHLA-ABC (DAKO M0736) antibody, which recognizes a conformational epitope on correctly folded MHC-complex. Then MHC complex in various concentrations was added. β2m in various concentrations was used as negative control. HRP-conjugated rabbit anti-β2m (DAKO PO174) was used for detection of bound MHC complex. TMB One-step substrate system (Dako) was used as a substrate for HRP, and color formation was followed by measurement of absorbance at 450 nm.

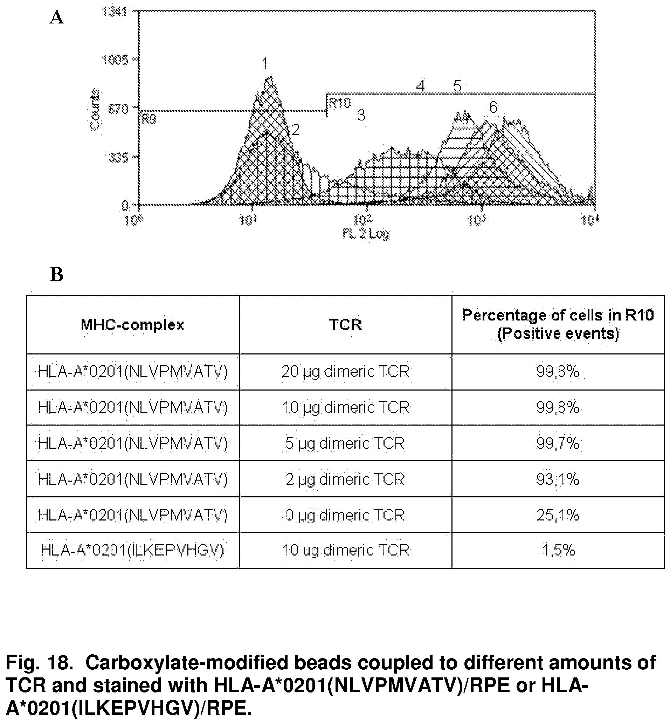

Figure 18. Carboxylate-modified beads coupled to TCR and stained with HLA-A*0201(NLVPMVATV)/RPE (SEQ ID NO 217779) or HLA-A*0201(ILKEPVHGV)/RPE (SEQ ID NO 217780) dextramers. TCR in various concentrations were coupled to carboxylate- modified beads and then stained with HLA-A*0201(NLVPMVATV)/RPE (SEQ ID NO 217779) or HLA-A*0201(ILKEPVHGV)/RPE (SEQ ID NO 217780) dextramers in a flow cytometry experiment.

A) Histogram showing x-axis: Fluorescence intensity measured in the RPE channel (FL2), y-axis: events counted. Events measured in the Region R9 are regarded as negative, and events measured in Region R10 are regarded as positive.
B) Percentage of positively stained beads is shown for each preparation of beads. Negative control samples:

1) Beads coupled with 10 μg TCR stained with HLA-A*0201(ILKEPVHGV)/RPE (SEQ ID NO 217780)
2) Beads coupled with 0 μg TCR stained with HLA-A*0201(NLVPMVATV)/RPE (SEQ ID NO 217779)

Positive control samples:

3) Beads coupled with 2 μg TCR stained with HLA-A*0201(NLVPMVATV)/RPE (SEQ ID NO 217779)

4) Beads coupled with 5 μg TCR stained with HLA-A*0201(NLVPMVATV)/RPE (SEQ ID NO 217779)
5) Beads coupled with 10 μg TCR stained with HLA-A*0201(NLVPMVATV)/RPE (SEQ ID NO 217779)
6) Beads coupled with 20 μg TCR stained with HLA-A*0201(NLVPMVATV)/RPE (SEQ ID NO 217779)

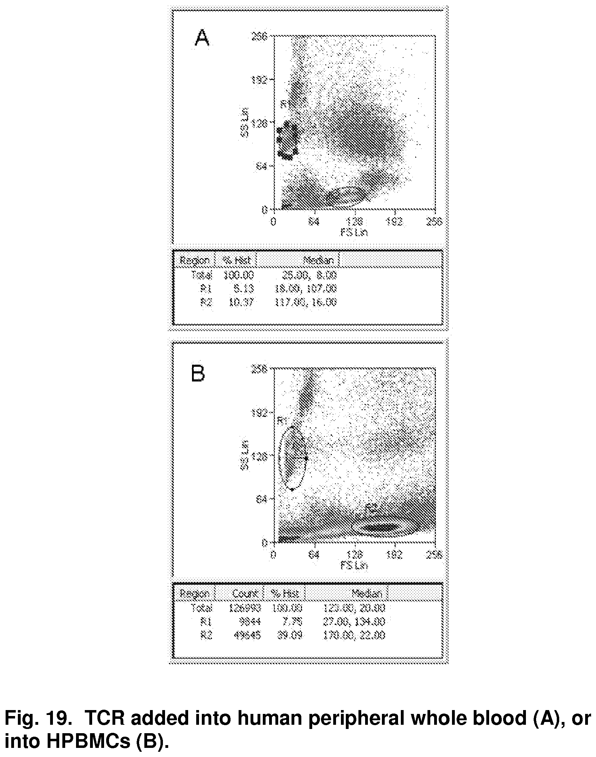

Figure 19. Flow cytometry analysis of human cell samples added TCR-coated beads. TCR-beads were added into human peripheral whole blood (A) or HPBMC (B) and then the samples were analysed by flow cytometry. Region R1 represents TCR-beads; region R2 represents lymphocyte cell population of interest.

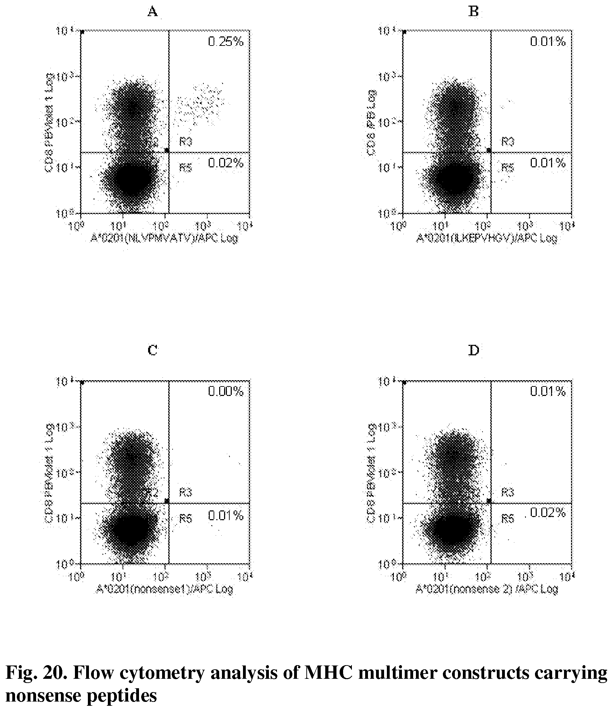

Figure 20. Flow cytometry analysis of MHC multimer constructs carrying nonsense peptides. Human Peripheral Blood Lymphocytes were ficoll purified from blood from a human donor and stained with mouse anti-human CD3/PE antibody and mouse anti-human CD8/PB antibody together with either of the MHC Dextramer molecule constructs A) HLA-A*0201(NLVPMVATV)/APC (SEQ ID NO 217779), B) HLA-A*0201(ILKEPVHGV)/APC (SEQ ID NO 217780), C) HLA-A*0201(nonsense peptide 1)/APC or D) HLA-A*0201 (nonsense peptide 2)/APC. The staining was analysed on a CyAn ADP flow cytometer. Live-gated and CD3 positive lymphocytes are shown.

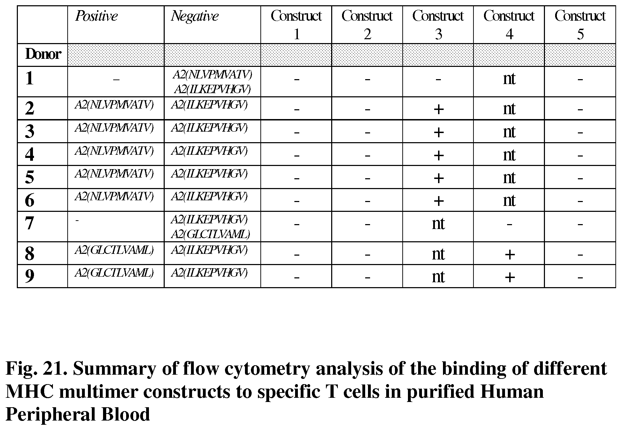

Figure 21. Summary of flow cytometry analysis of the binding of different MHC multimer constructs to specific T cells in purified Human Peripheral Blood. Mononuclear Cell samples. Purified HPBMC were stained with different MHC(peptide) molecules attached to APC labeled dextran270 multimerization domain and analyzed by flow cytometry . See example 58 for details on experimental procedures. 5 different MHC(peptide) molecules were investigated. Construct 1: HLA-A*0201(GLAGDVSAV) (SEQ ID NO 217778), construct 2: HLA-A*0201 (ALIAPVHAV) (SEQ ID NO 217781), construct 3: HLA-A*0201(NLVPMVATV) (SEQ ID NO 217779), construct 4: HLA-A*0201(GLCTLVAML) (SEQ ID NO 217782) and construct 5: HLA-A*0201(ILKEPVHGV) (SEQ ID NO 217780). A positive staining is symbolized with a (+) and is here defined as the identification of a distinct CD8 positive and MHC (peptide) positive population when visualized in a dot plot (se Figure 20). Negative staining is symbolized wit a (-) and is defined as absence of a distinct CD8 positive and MHC (peptide) positive population when visualized in a dot plot. Nt means not determined. All samples have previously been analyzed for the presence of T-cells restricted by HLA-A*0201(NLVPMVATV) (SEQ ID NO 217779), HLA-A*0201(GLCTLVAML) (SEQ ID NO 217782) and HLA-A*0201(ILKEPVHGV) (SEQ ID NO 217780) and these results are shown in italics in the figure (column 2 and 3).

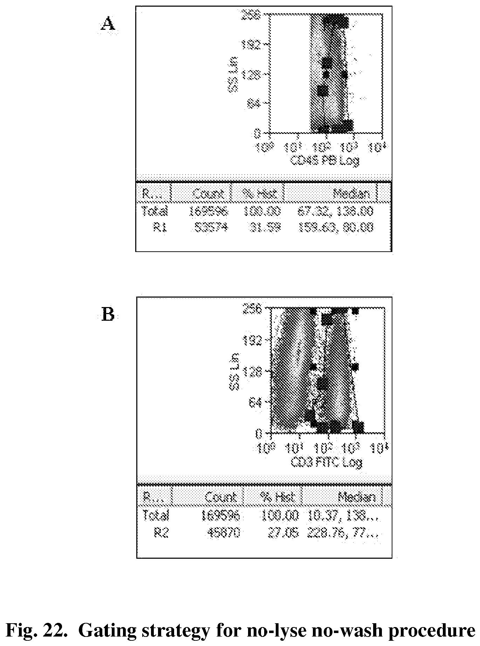

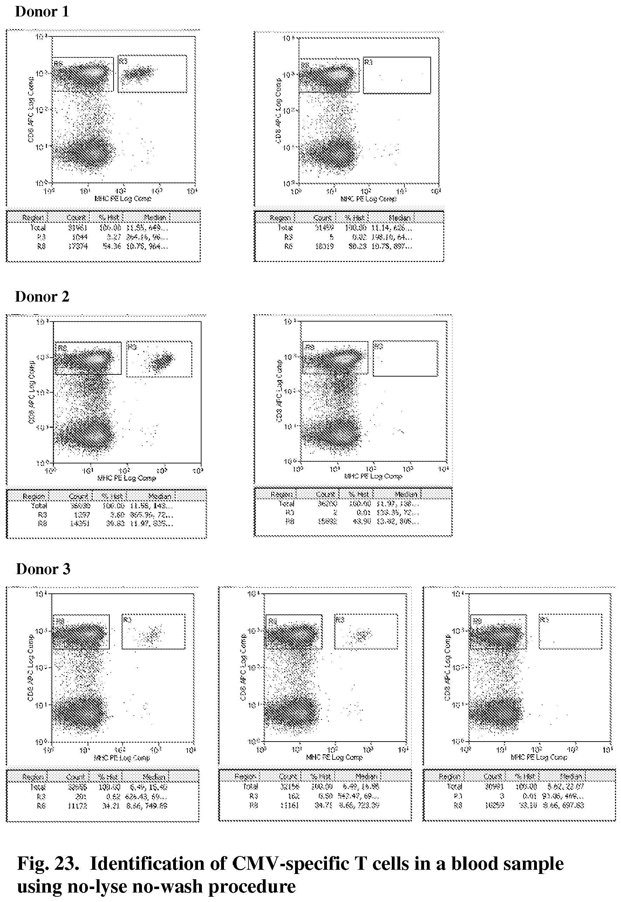

Figure 22. Gating strategy for no-lyse no-wash procedure. Whole blood was stained with MHC multimer, anti-CD8/APC, anti-CD3/PB and CD45/CY antibody in a no-lyse no-wash procedure. For further details see text in example 65. During analysis of data the following gating strategy was used: CD45/PB antibody was used to set a trigger discriminator to allow the flow cytometer to distinguish between red blood cells and stained white blood cells. This was done during data collection by gating on CD45/PB positive cells in a CD45/PB vs. side scatter dot plot as shown in A. After data collection and during data analysis CD3 positive cells were selected by gating CD3/FITC positive cells in a CD3/FITC vs side scatter plot as shown in B. The final data was illustrated in a MHC multimer/PE vs CD8/APC plot (see Figure 23).

Figure 23. Identification of CMV-specific T cells in a blood sample using no-lyse no-wash procedure. Whole blood from three different donors were analysed for the presence of CMV-specific T cells by flow cytometry using a no-lyse no-wash procedure. Donor 1 was stained with a MHC multimer consisting of PE-conjugated 270 kDa dextran coupled with HLA-A*0201 in complex with beta2microglobulin and the peptide NLVPMVATV (SEQ ID NO 217779) derived from Human Cytomegalo Virus (HCMV) (left panel) and with a negative control MHC multimer consisting of PE conjugated 270 kDa dextran coupled with HLA-A*0201 in complex with beta2microglobulin and the peptide ILKEPVHGV (SEQ ID NO 217780) derived from Human Immunodeficiency Virus (HIV) (right panel). Donor 2 was stained with a MHC multimer consisting of PE-conjugated 270 kDa dextran coupled with HLA-A*0101 in complex with beta2microglobulin and the peptide VTEHDTLLY (SEQ ID NO 217783) derived from Human Cytomegalo Virus (HCMV) (left panel) and a negative control MHC multimer consisting of PE-conjugated 270 kDa dextran coupled with HLA-A*0101 in complex with beta2microglobulin and the peptide IVDCLTEMY (SEQ ID NO 217784) derived from ubiquitin specific peptidase 9 (USP9) (right panel). Donor 3 was stained with twoMHC multimers consisting of PE conjugated 270 kDa dextran coupled with HLA-B*0207 in complex with beta2microglobulin and either of the peptides TPRVTGGGAM (SEQ ID NO 217785) (left panel) or RPHERNGFTVL (SEQ ID NO 217786) (center panel) both derived from Human Cytomegalo Virus (HCMV) and with a negative control MHC multimer consisting of PE-conjugated 270 kDa dextran coupled with HLA- B*0207 in complex with beta2microglobulin and the peptide TPGPGVRYPL (SEQ ID NO 217787) derived from Human Immunodeficiency Virus (HIV) (right panel). All samples were also added Anti-CD45/PB, anti-CD3/FITC and anti-CD8/APC antibodies. The samples were gated as shown in Figure 22.

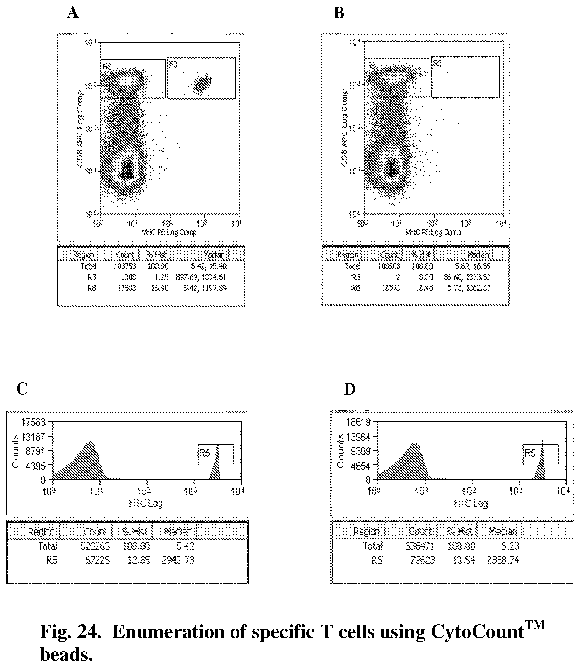

Figure 24. Enumeration of specific T cells using CytoCount™ beads. Whole blood from a human donor were analysed for the presence of CMV-specific T cells with MHC multimers by flow cytometry using a no-lyse no-wash procedure. 2 x 100 μl donor blood was analysed with two different MHC multimers: A) PE-conjugated 270 kDa dextran coupled with HLA-A*0101 in complex with beta2microglobulin and the peptide VTEHDTLLY (SEQ ID NO 217783) derived from Human Cytomegalo Virus (HCMV) and a negative control construct B) consisting of PE-conjugated 270 kDa dextran coupled with HLA-A*0101 in complex with beta2microglobulin and the peptide IVDCLTEMY (SEQ ID NO 217784) derived from ubiquitin specific peptidase 9 (USP9). To each sample Anti-CD45/CY, anti-CD3/APC and anti-CD8/PB antibody was added together with 50 μl CytoCount beads (1028 beads/μl). Following staining for 15 minutes PBS was added to 1 ml and the samples analysed on a CyAn flow cytometer. During analysis CD45/CY antibody was used to set a trigger discriminator to allow the flow cytometer to distinguish between red blood cells and stained white blood cells and CD3/APC antibody was used to gate for CD3 positive T lymphocytes. Amount of counted beads in sample A are shown in the histogram C and amount of beads counted in the negative control sample B are show in histogram D.

Concentration of HLA-A*0101 (VTEHDTLLY) (SEQ ID NO 217783) specific T cells in the blood sample were determined as follows:

$$((\text{count of MHC multimer+ CD8+ cells in A x concentration of beads x dilution factor of beads}) / \text{counted beads C})- ((\text{counted MHC multimer+ CD8+ cells in B x concentration of beads x dilution factor of beads}) / \text{counted beads D}) = ((1300 \text{ cells x } 1028 \text{ beads/}\mu l \text{ x } 0{,}05) / 67225 \text{ beads}) - ((2 \text{ cells x } 1028 \text{ beads/}\mu l \text{ x } 0{,}05) / 72623 \text{ beads}) = 0{,}9926 \text{ cells/}\mu l = 992{,}6 \text{ celler/ml}$$

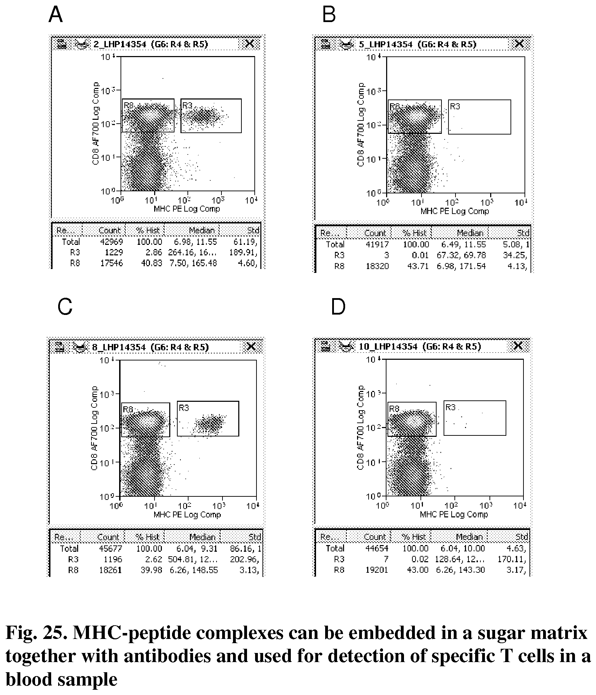

Figure 25. MHC dextramers can be embedded in a sugar matrix together with antibodies and used for detection of specific T cells in a blood sample. MHC dextramer constructs was embedded in a sugar matrix together with relevant gating reagents (anti-CD3/Pacific Blue, anti-CD8/Alexa700 and anti-CD45/Cascade Yellow antibodies) and the matrix dried. Then EDTA stabilized blood from a human donor were added and the samples analyzed by flow cytometry. Two different MHC construct were used HLA-A*0101(VTEHDTLLY)/PE dextramer (SEQ ID NO 217783) (A) and the negative control construct HLA-A*0101(IVDCLTEMY)/PE (SEQ ID NO 217784) (B). As a control antibodies and MHC dextramer constructs were used to stain blood from the same donor following a general staining procedure without embedding the antibodies and MHC dextramers in a sugar matrix as described elsewhere herein. (C) Staining with HLA-A*0101(VTEHDTLLY)/PE (SEQ ID NO 217783) dextramer following a normal staining procedure and (D) Staining with HLA-A*0101(IVDCLTEMY)/PE (SEQ ID NO 217784) dextramer following a normal staining procedure.

Figure 26. Borrelia genome survey for the three Borrelia species, B. burgdorferi, B. afzelii and B.garinii. Listed are the database accession numbers for the individual genomes and the known plasmids or variable plasmid segments of the three species. The specific strains of the individual species are denoted.

Figure 27. Complete list of all known and putative proteins encoded by the genome and plasmids of Borrelia burgdorferi (strain B31). The proteins are identified by their names or designations. Position of start and end of the gene, coding strand, amino acid length of the encoded protein, and the accession numbers and gene ID's are given.

Figure 28. Complete list of all known and putative proteins encoded by the genome and plasmids of Borrelia afzelii (strain PKo). The proteins are identified by their names or designations. Position of start and end of the gene, coding strand, amino acid length of the encoded protein, and the accession numbers and gene ID's are given.

Figure 29. Complete list of all known and putative proteins encoded by the genome, plasmids and known variable plasmid segments of Borrelia garinii (strain PBi). The proteins are identified by their names or designations. Position of start and end of the gene, coding strand, amino acid length of the encoded protein, and the accession numbers and gene ID's are given.

Figure 30. The amino acid sequences of Borrelia antigens of specific interest as sources for MHC binding peptides. Name and protein bank accession numbers are shown.

Figure 31. Borrelia MHC class 1 antigen peptides of 8-, 9-, 10- and 11 amino acids in length generated from the chosen protein sequences by random prediction software (see figure 2). Name and protein bank accession numbers are shown.

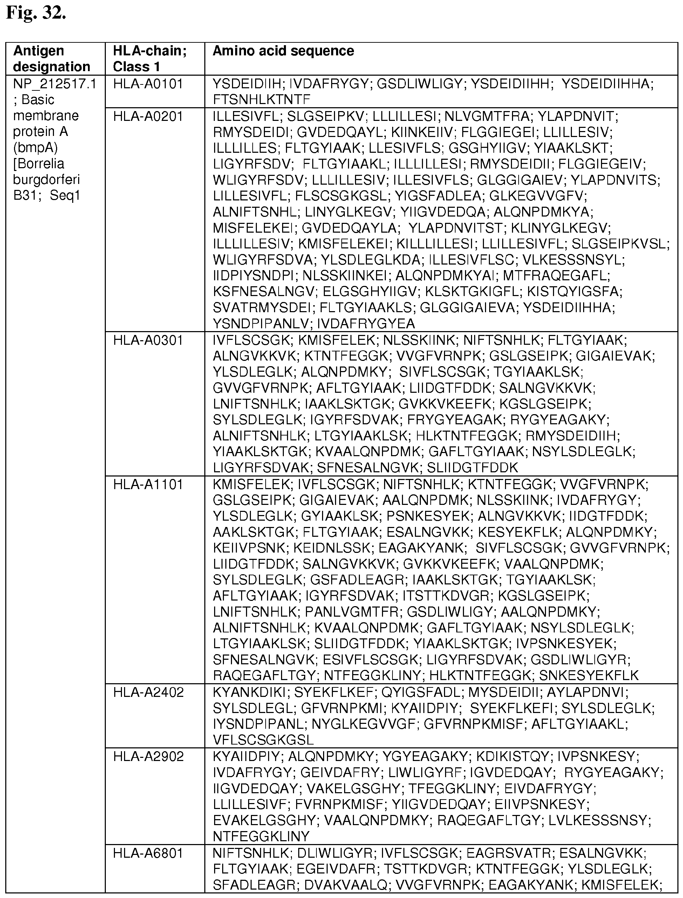

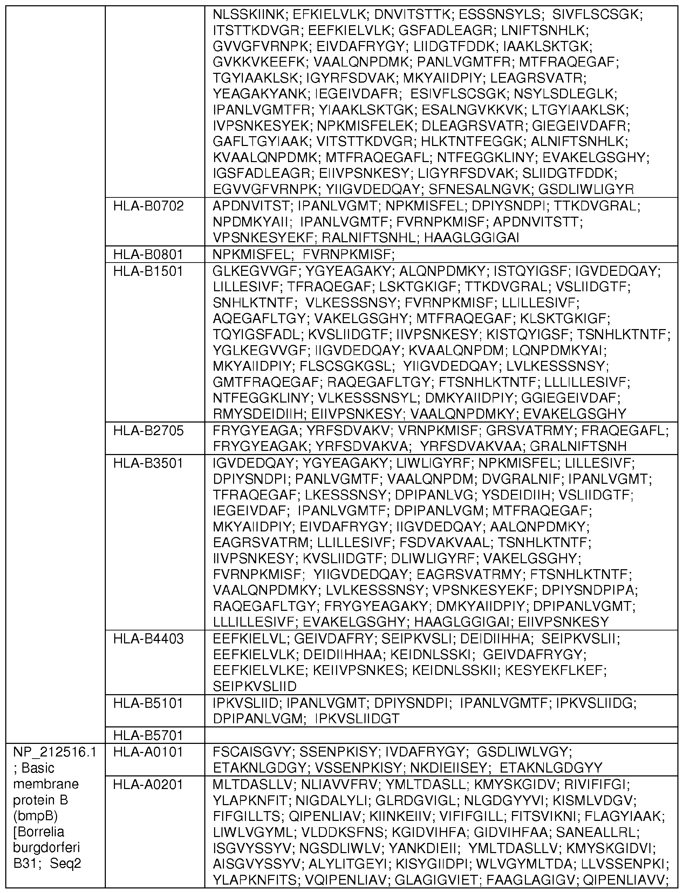

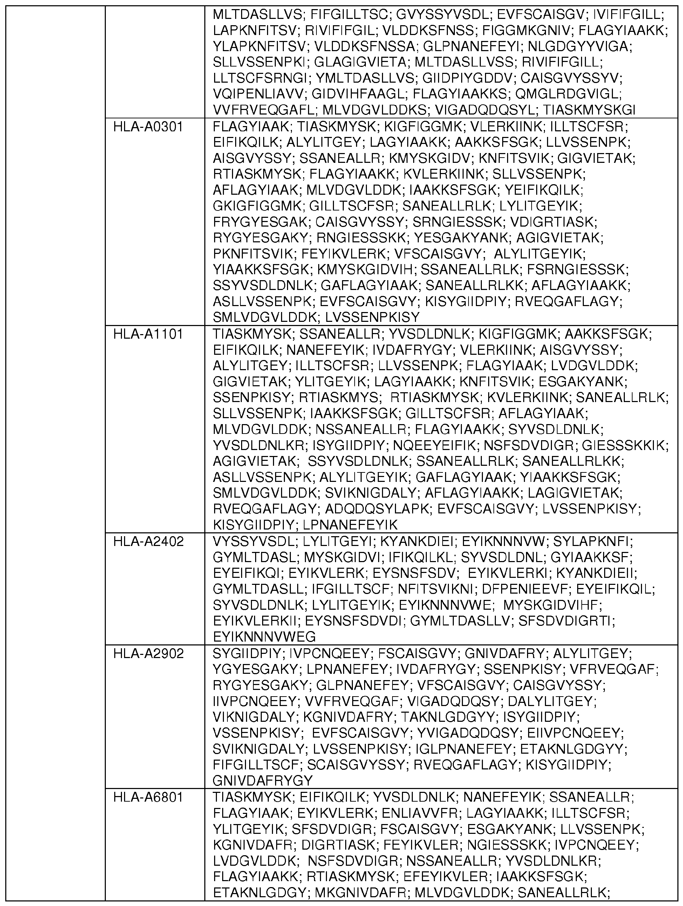

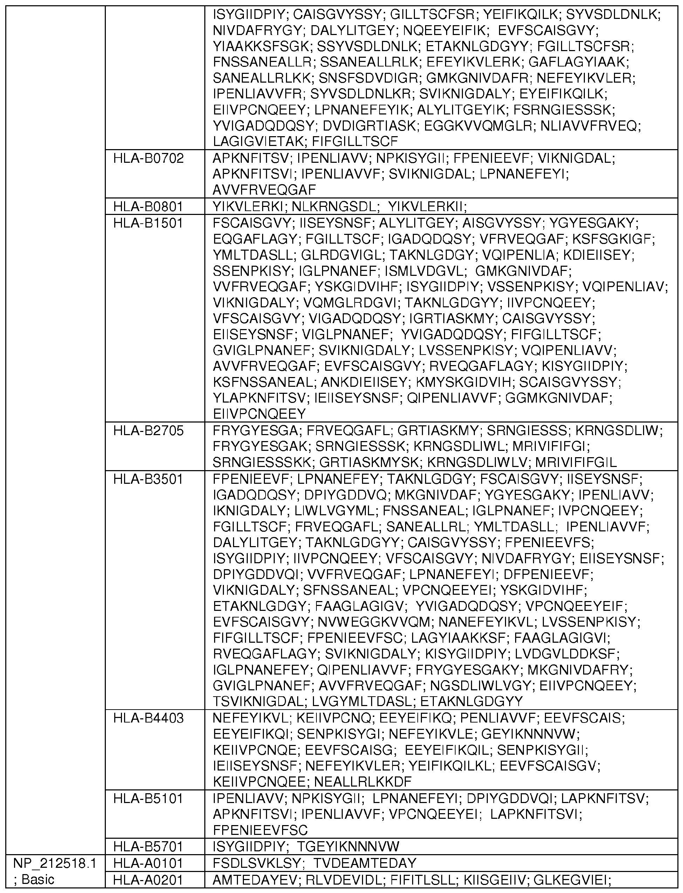

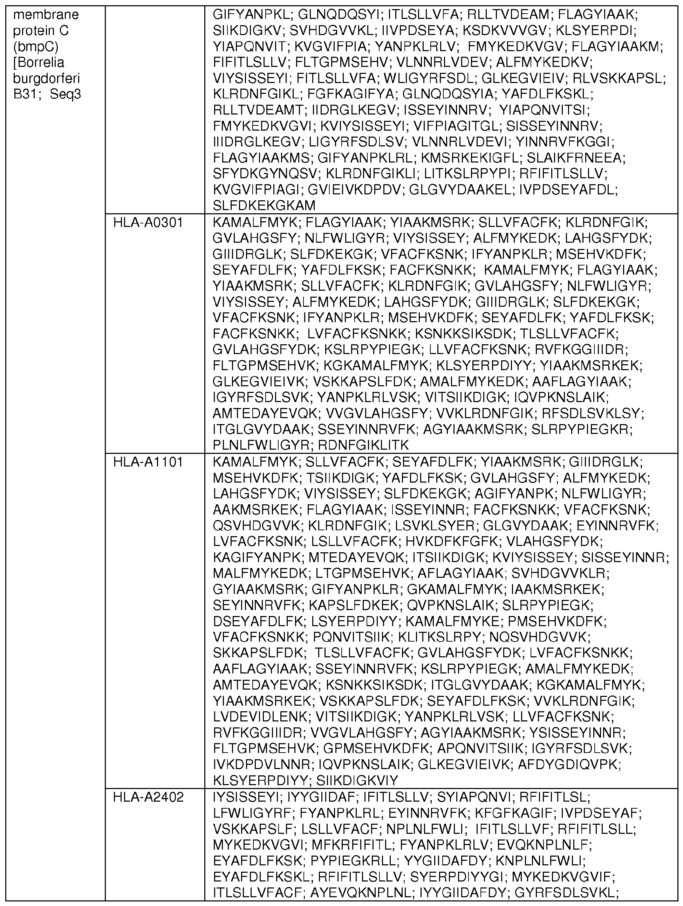

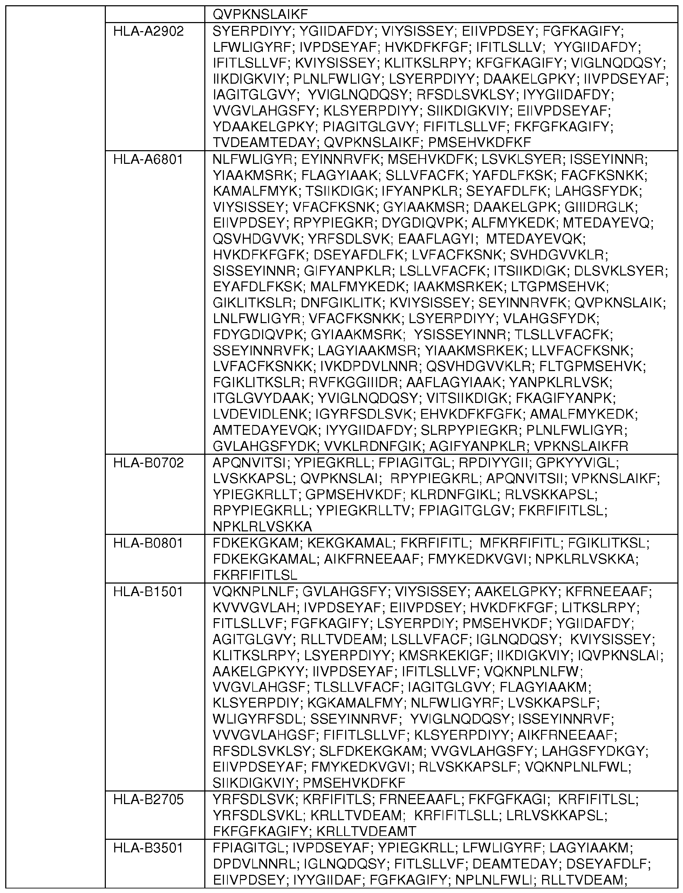

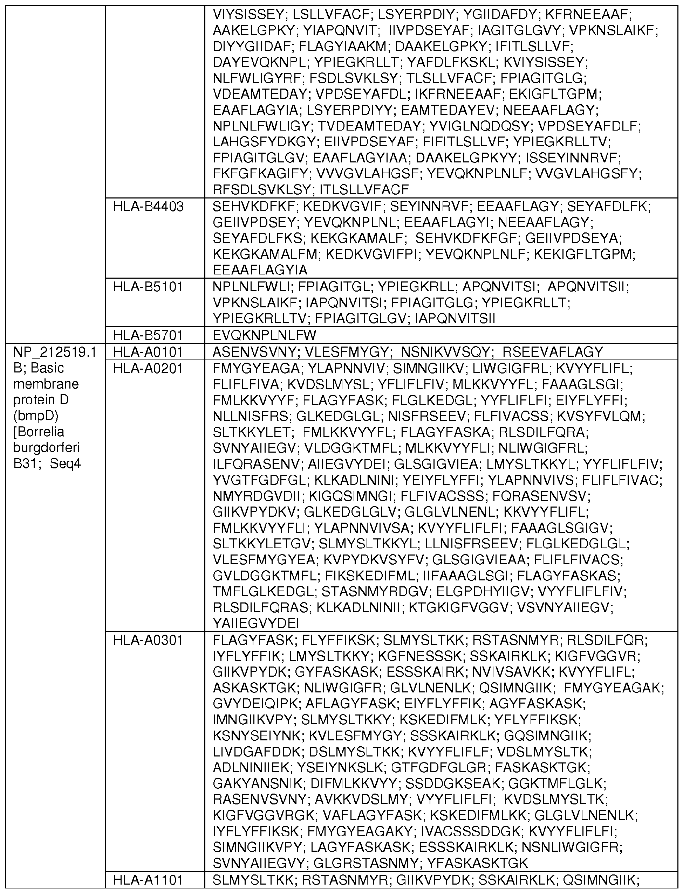

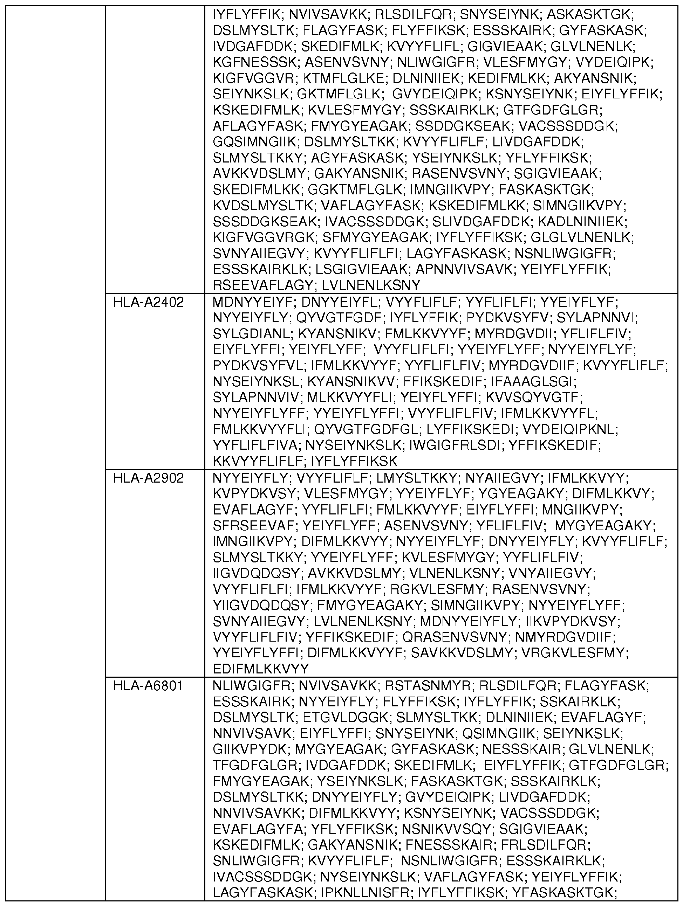

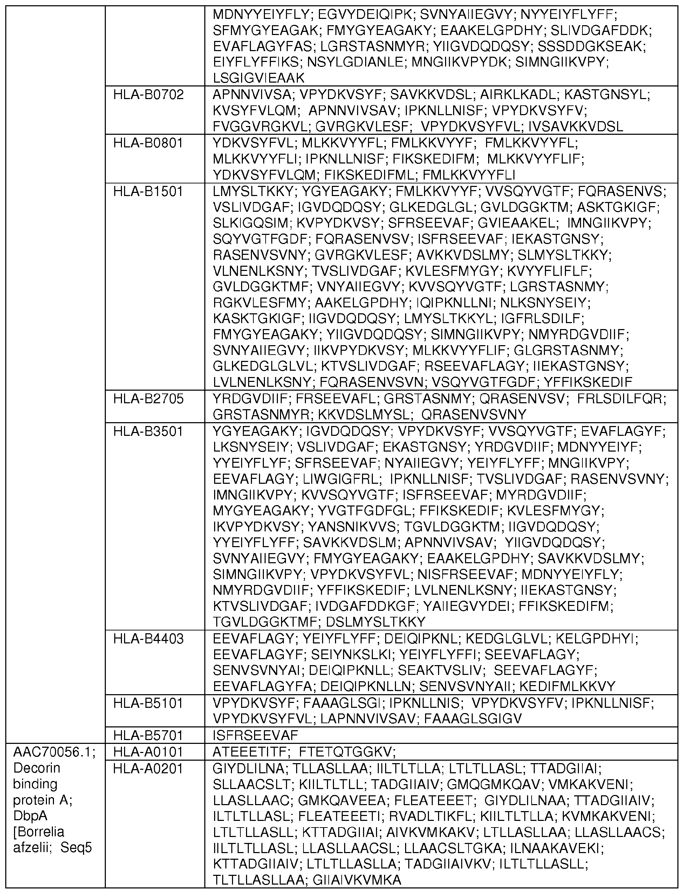

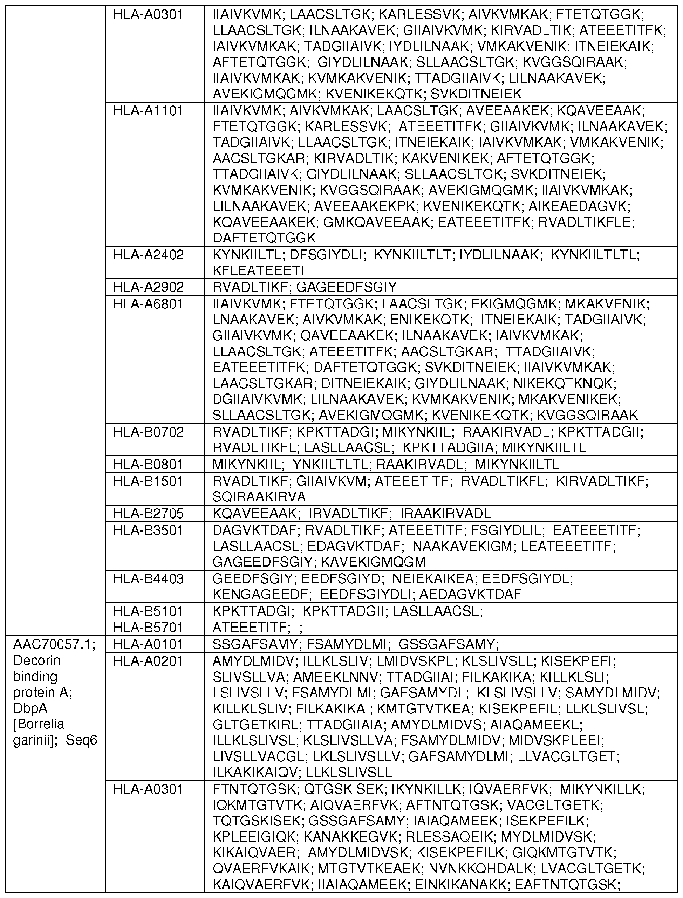

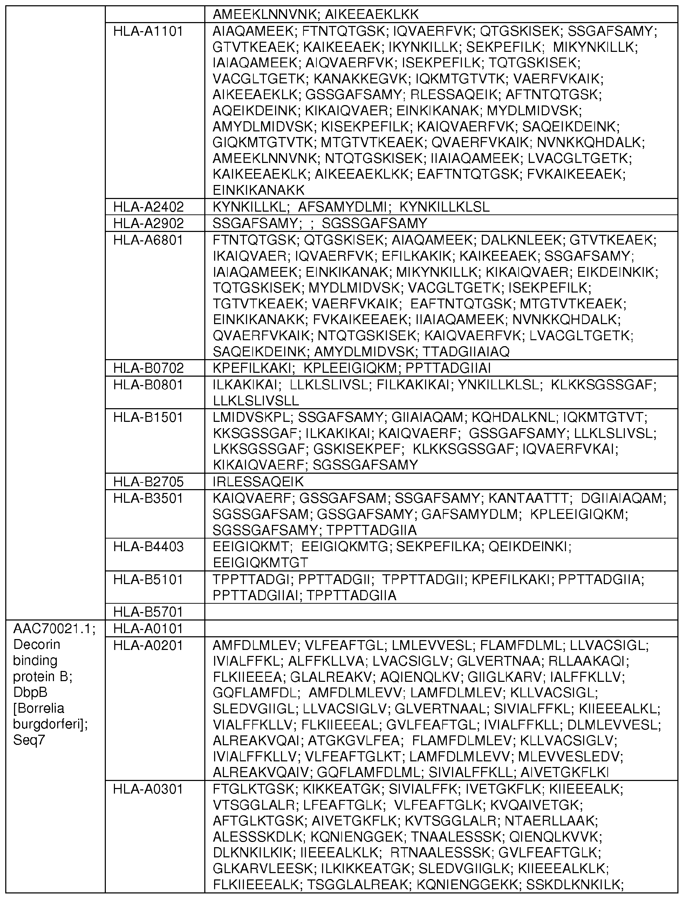

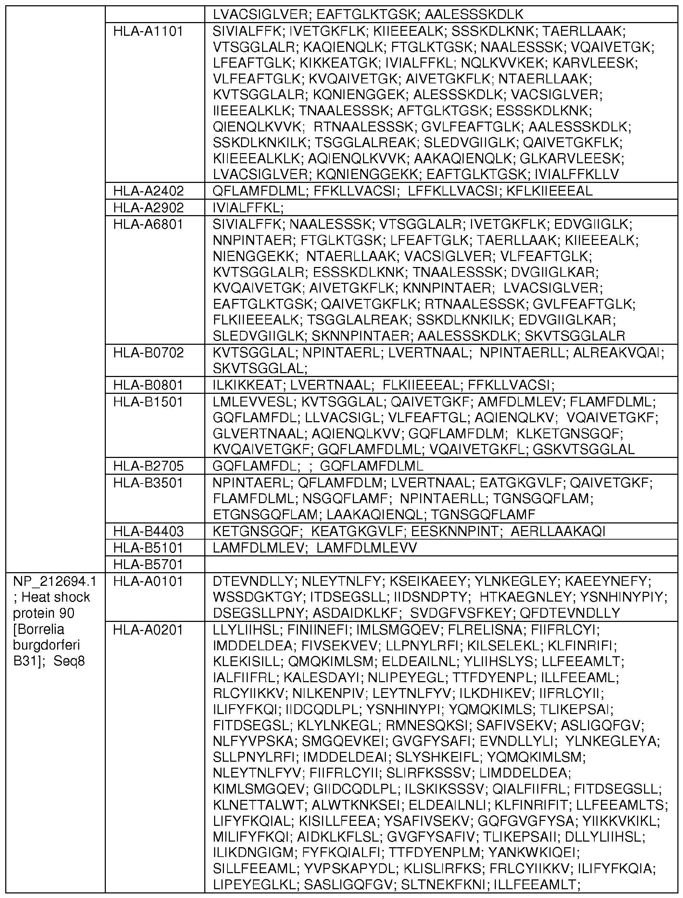

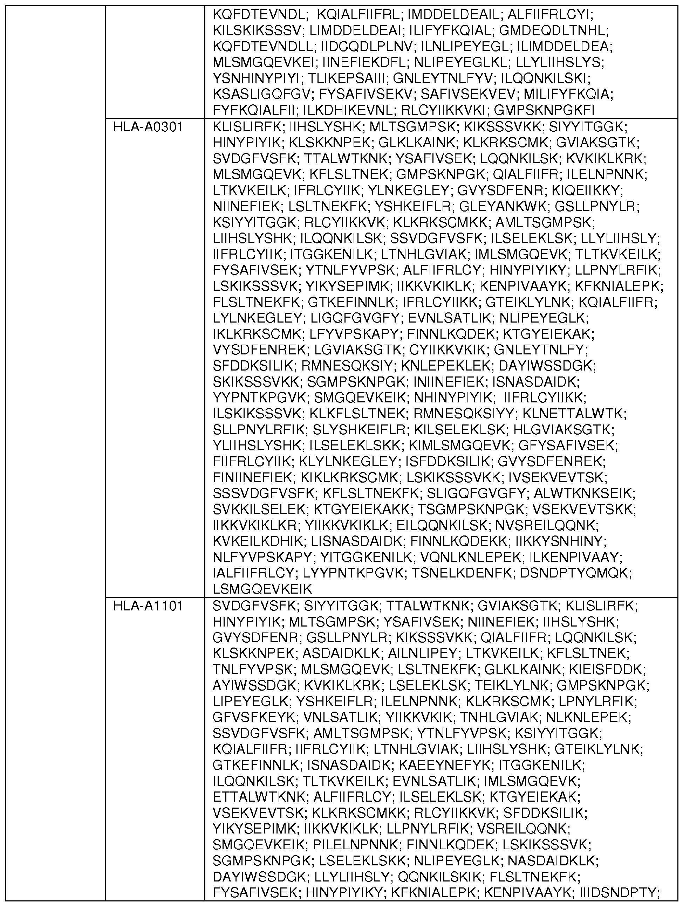

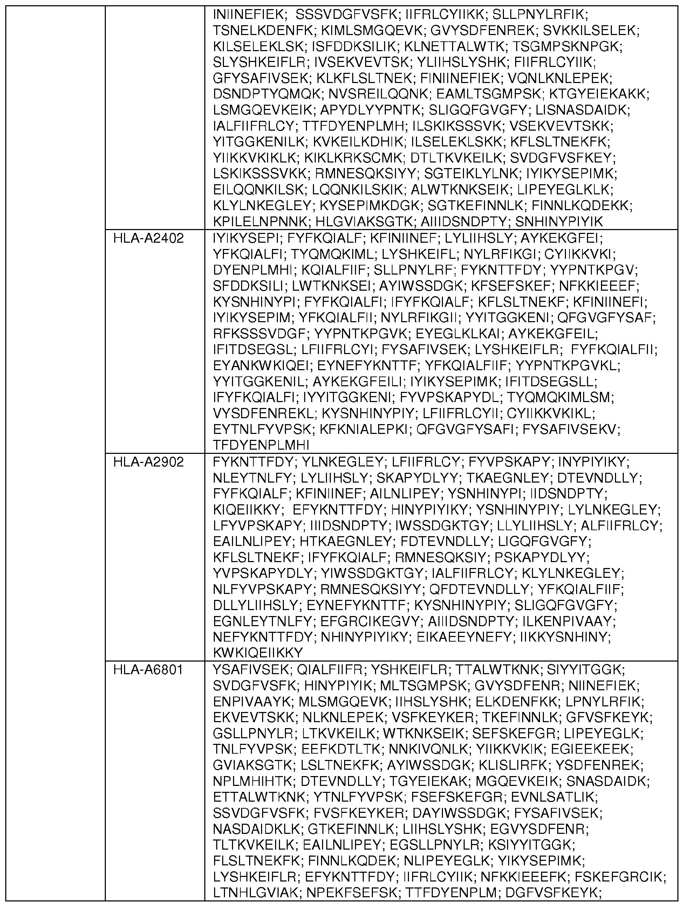

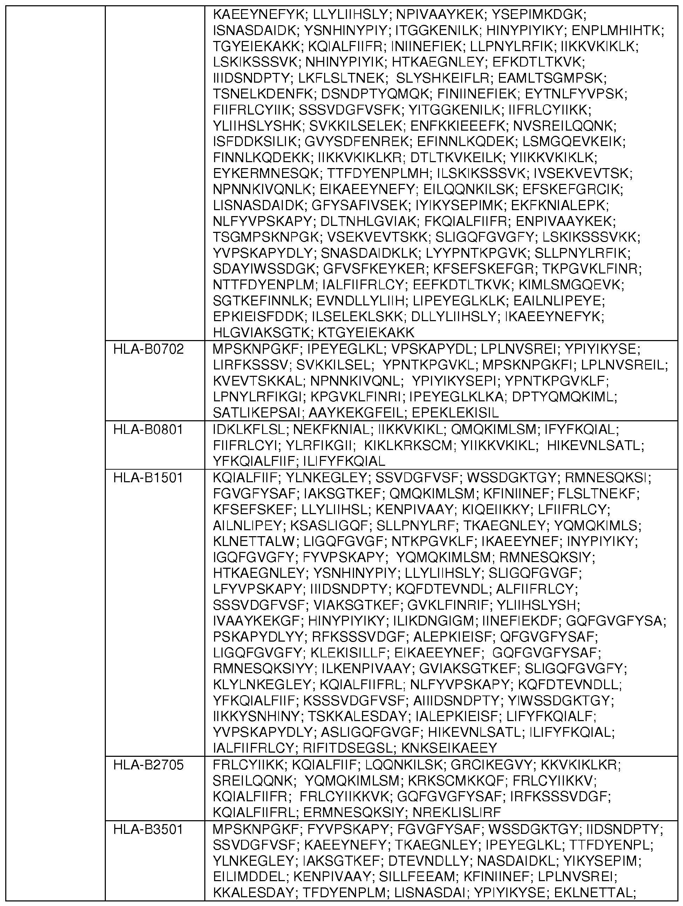

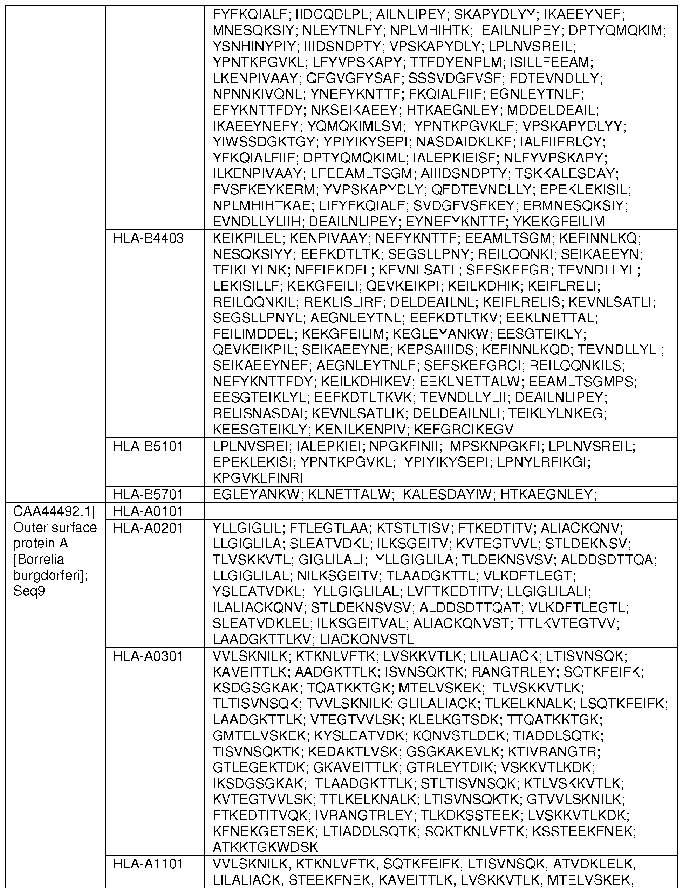

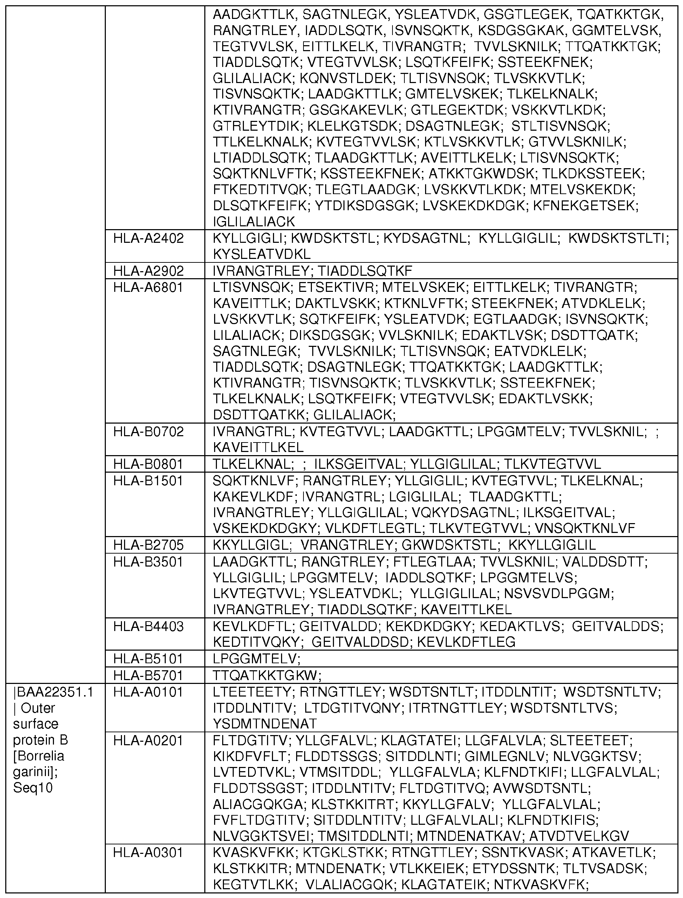

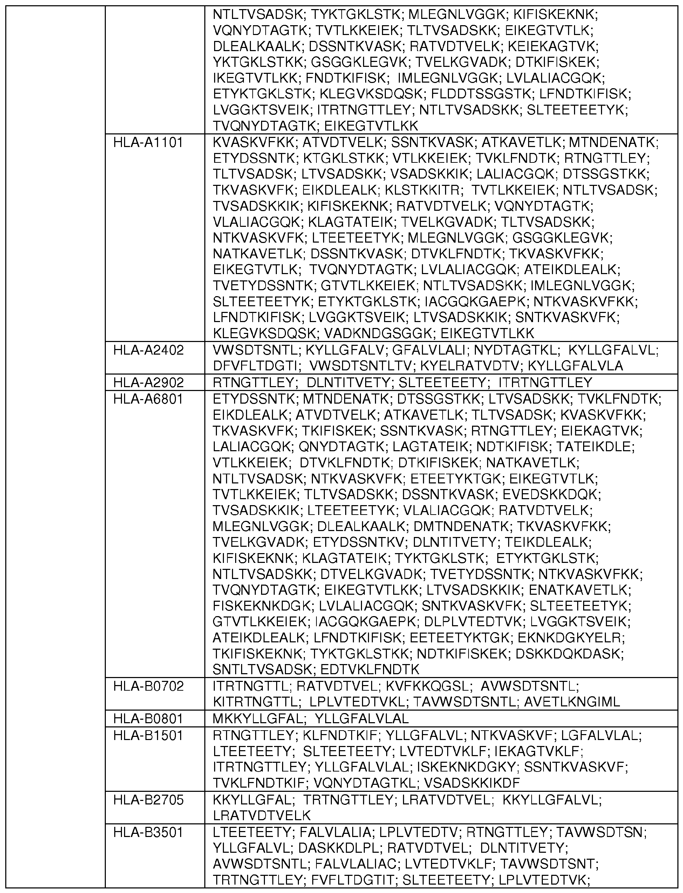

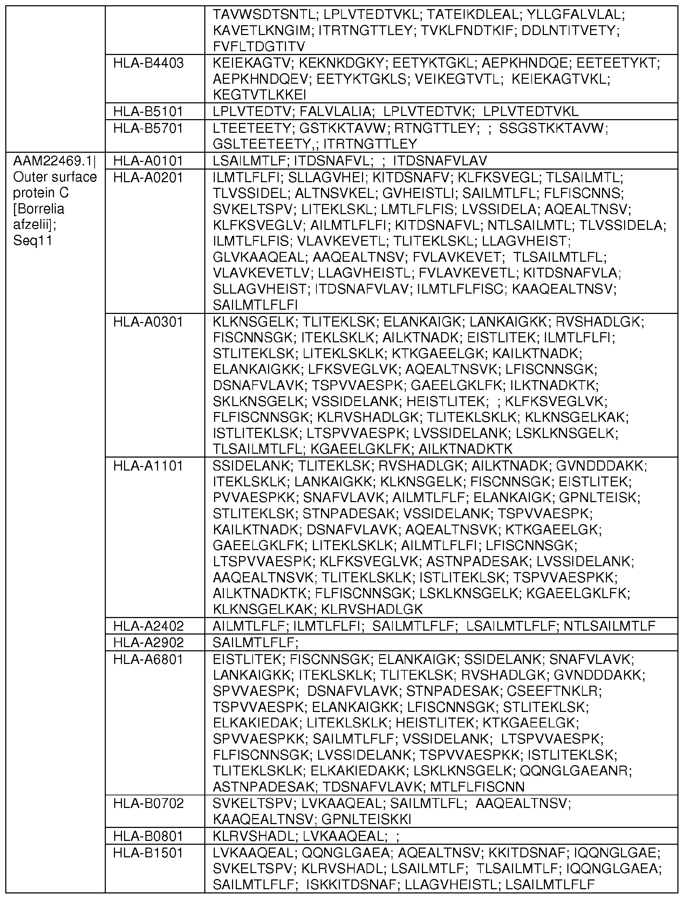

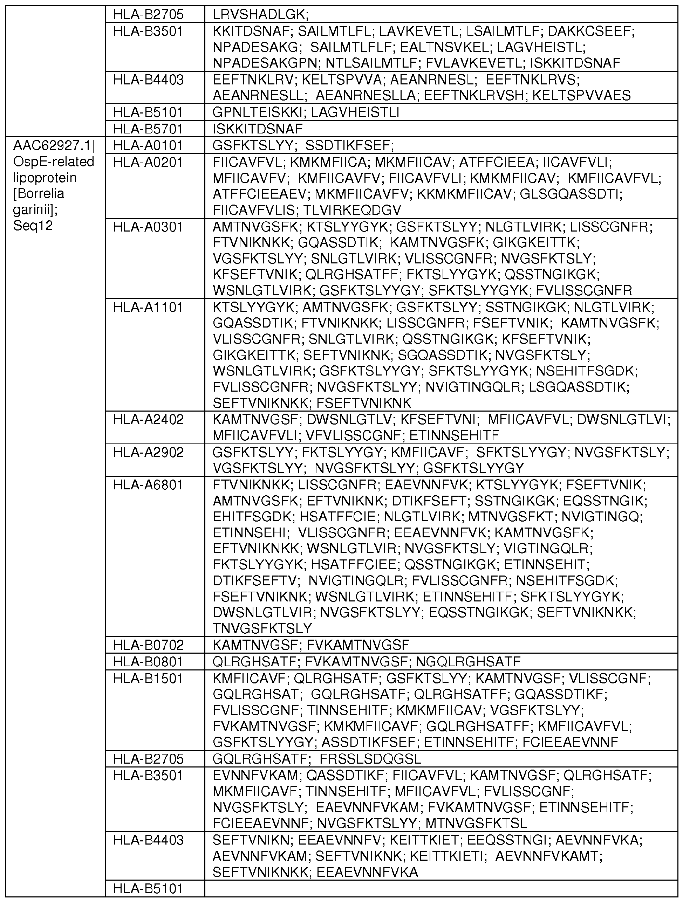

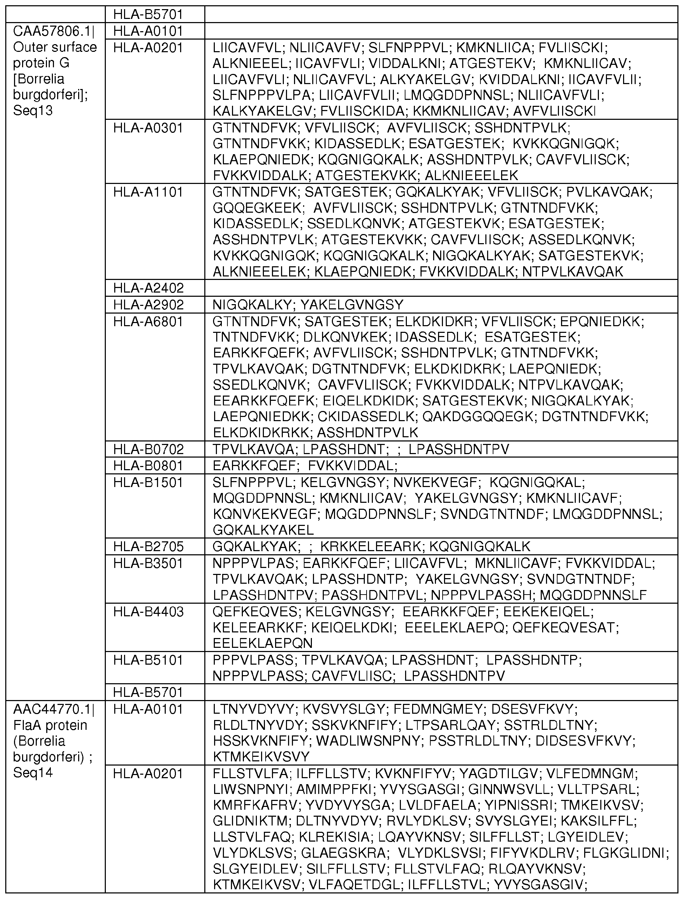

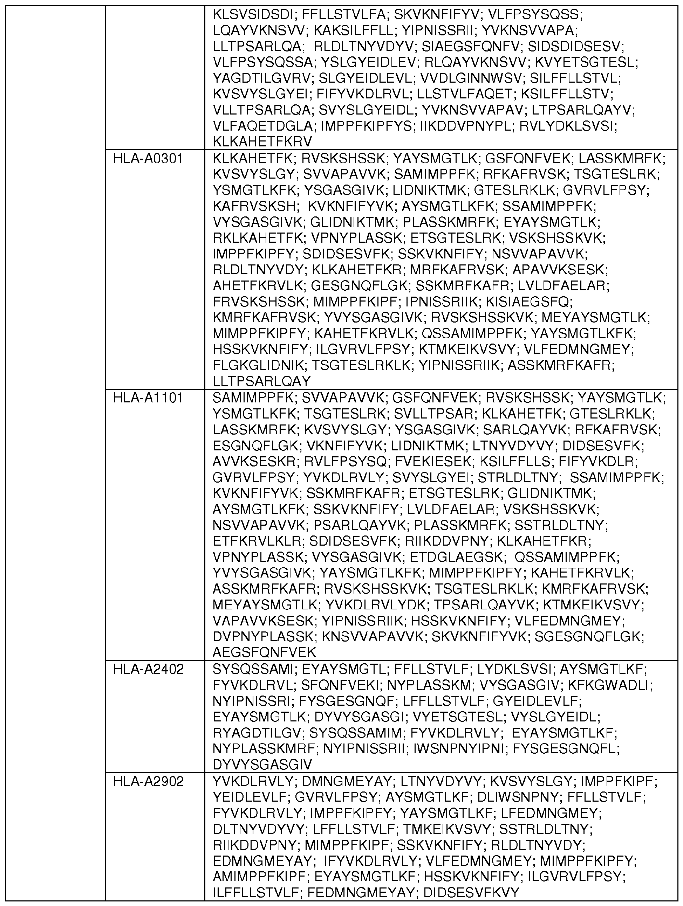

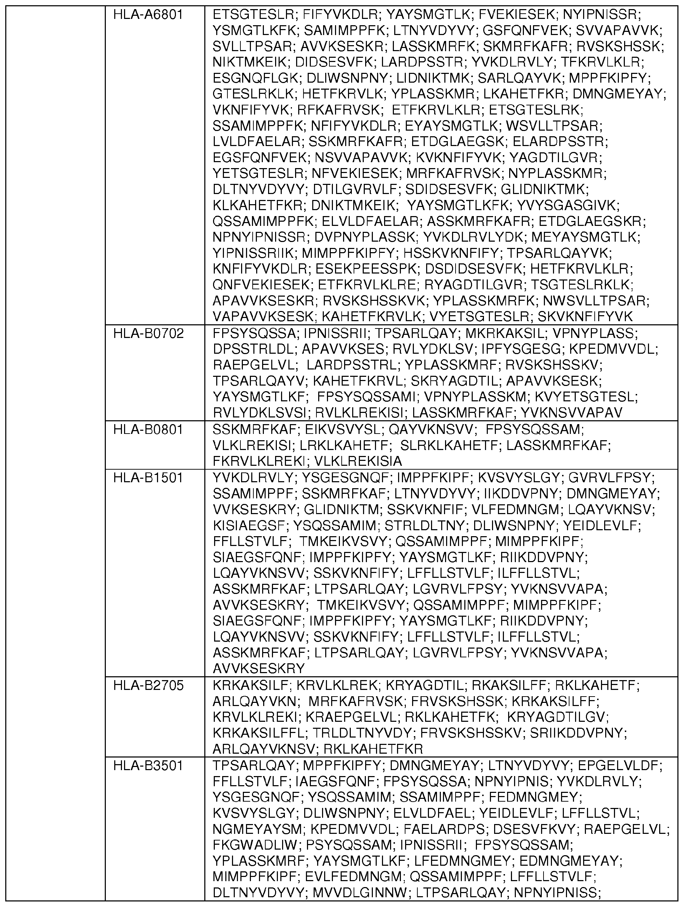

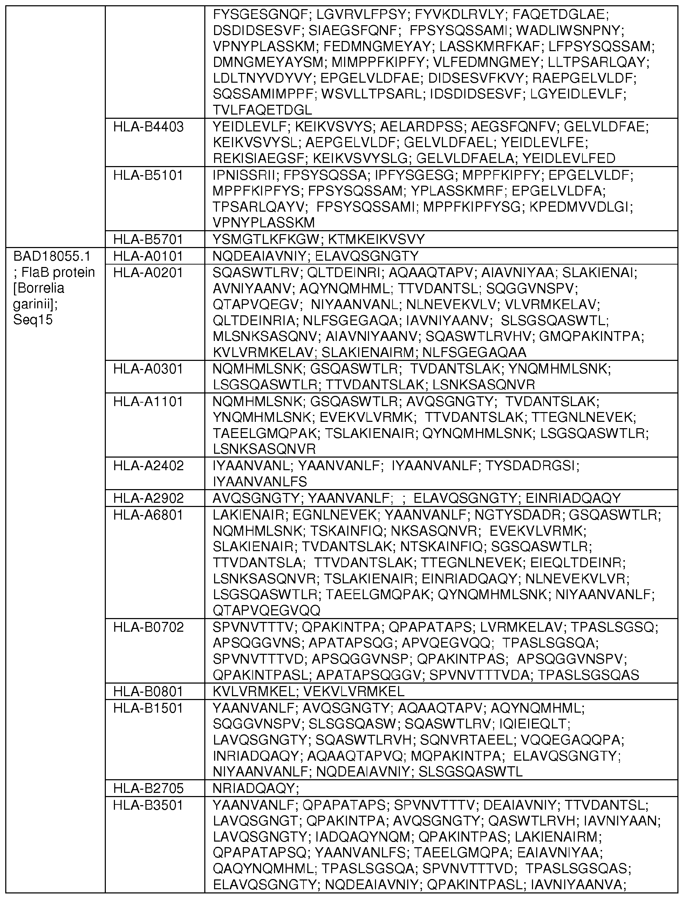

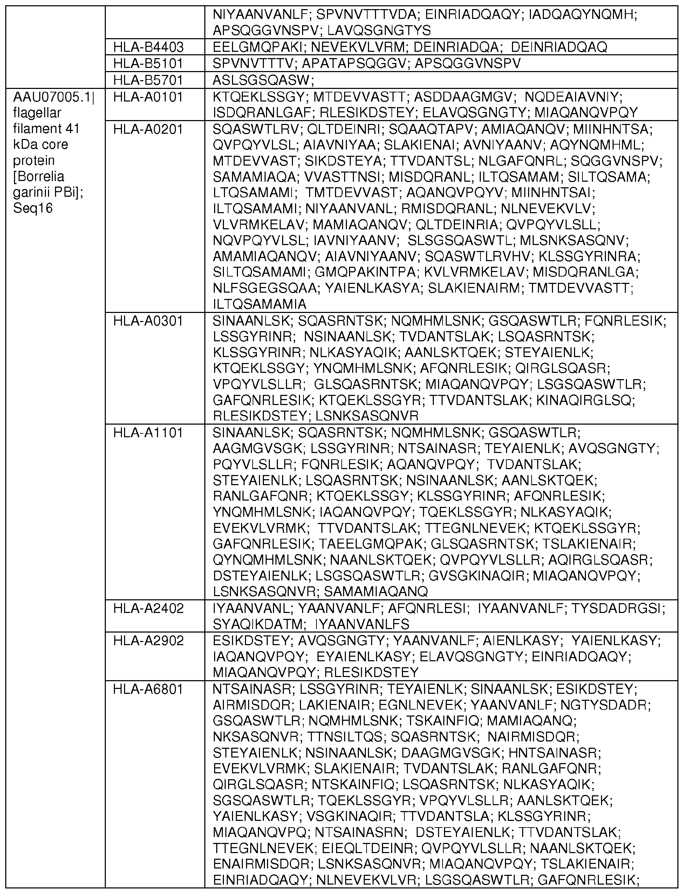

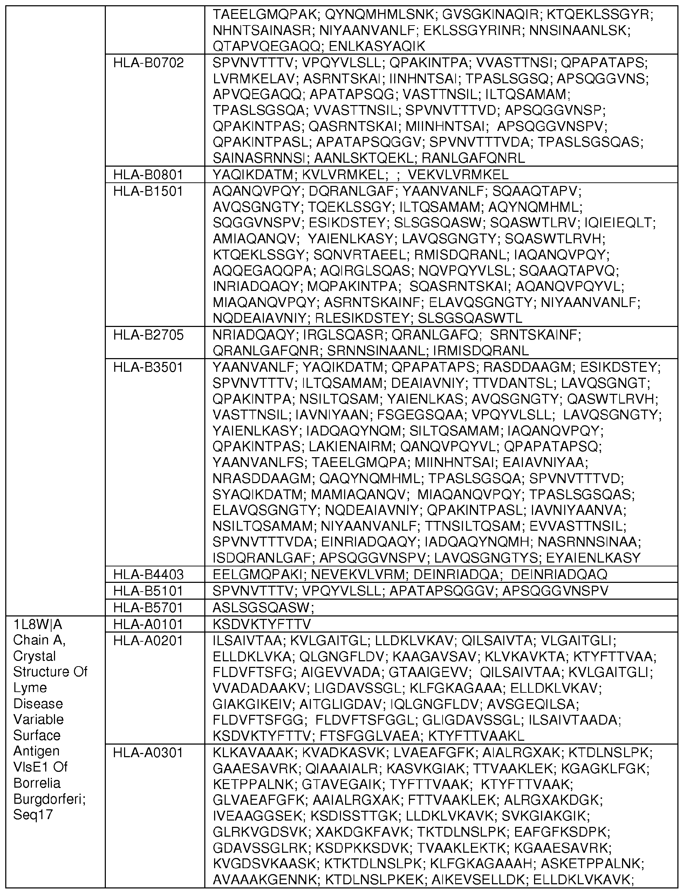

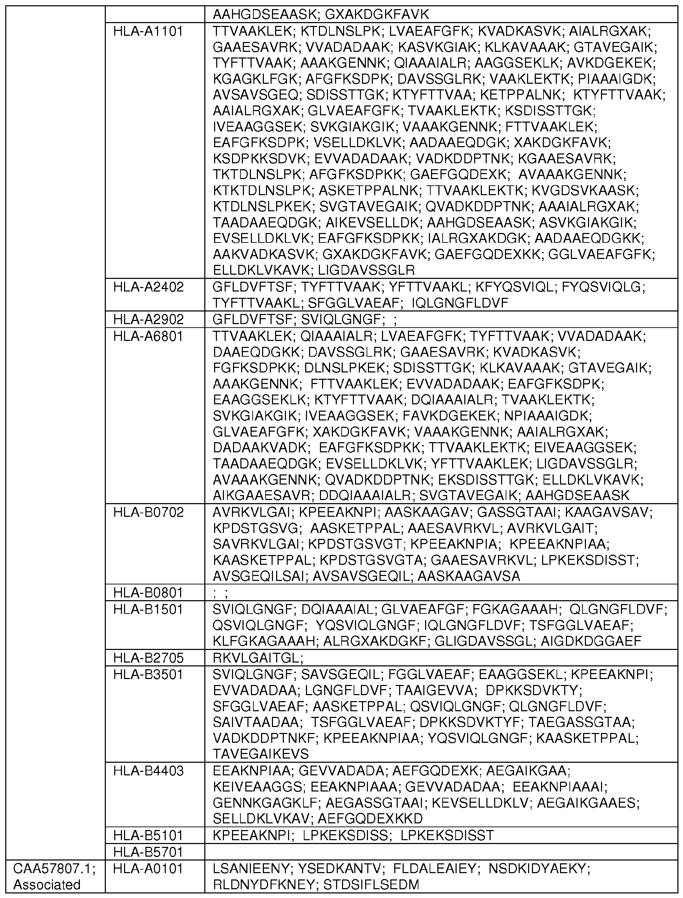

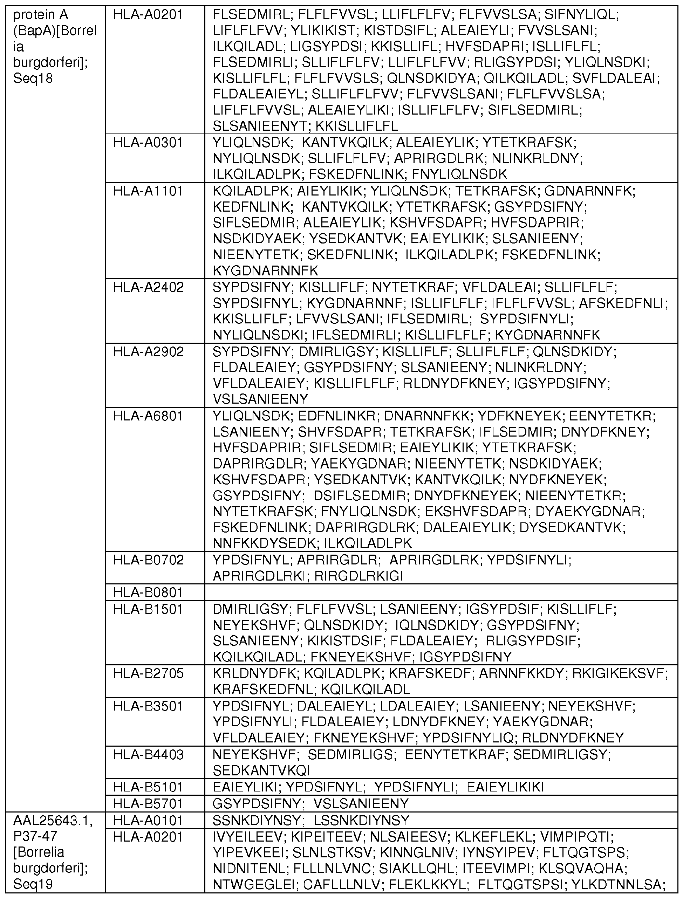

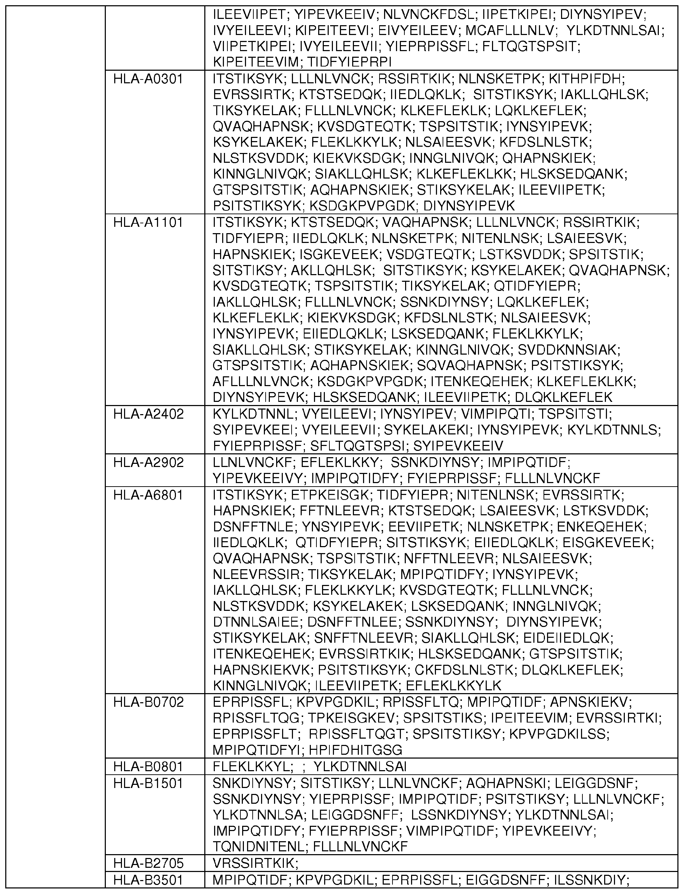

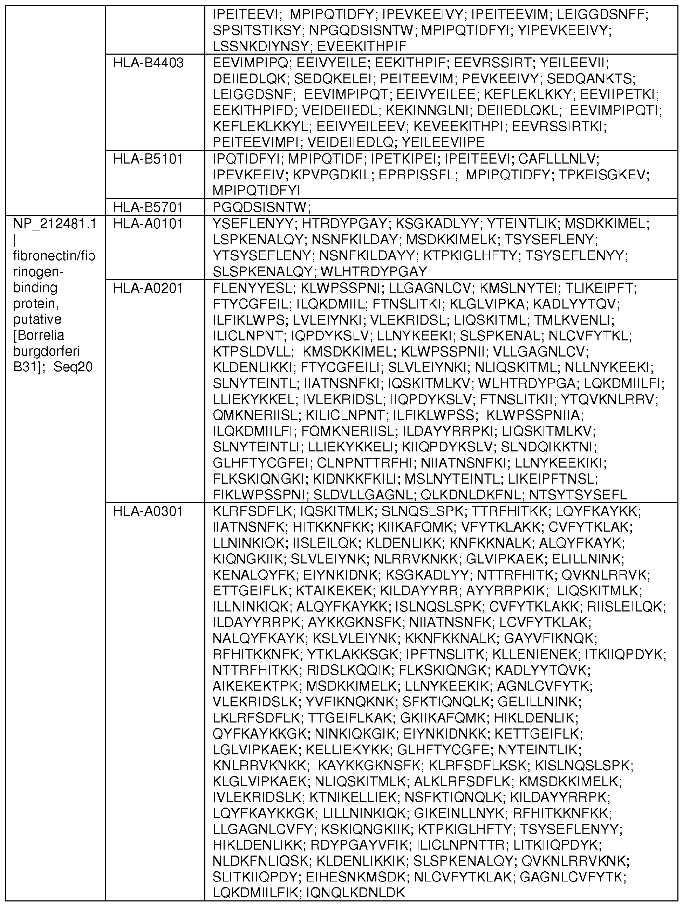

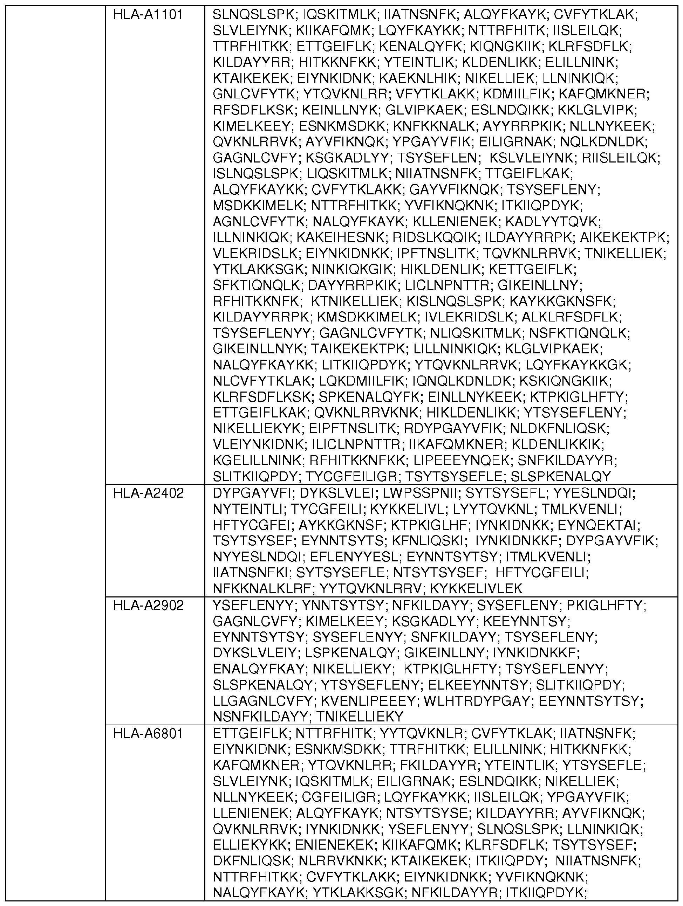

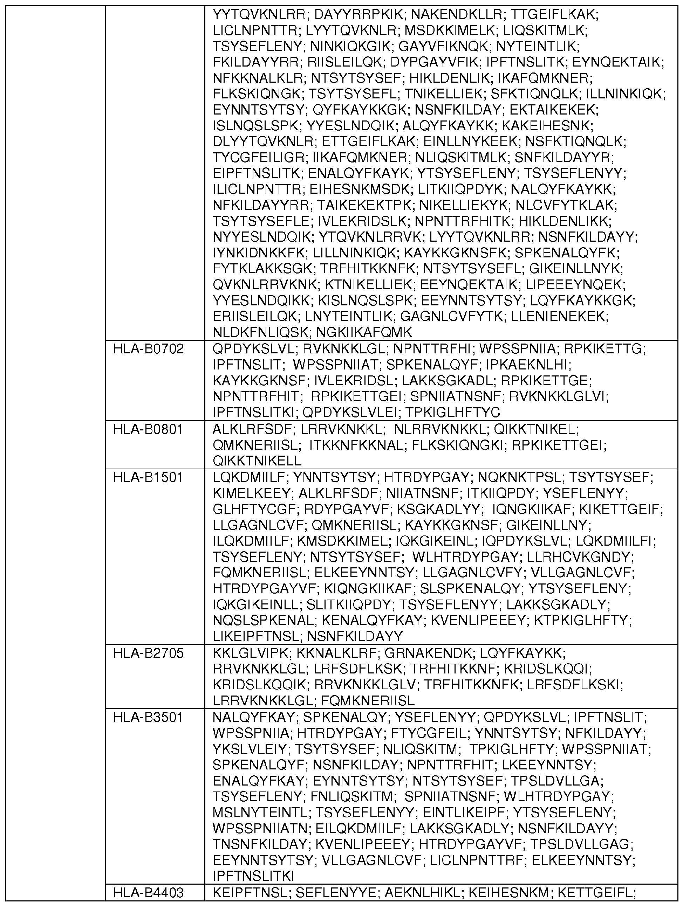

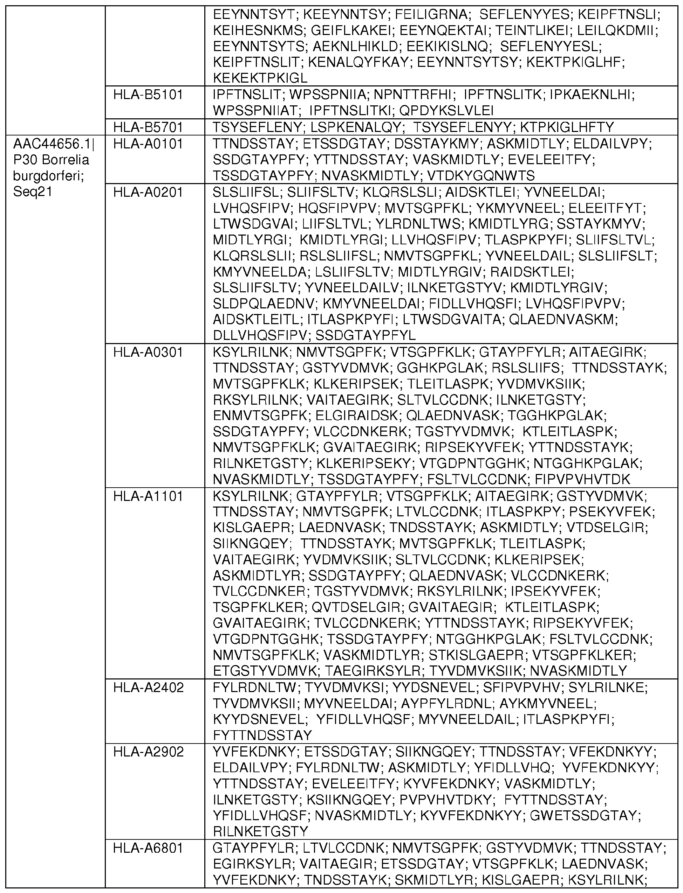

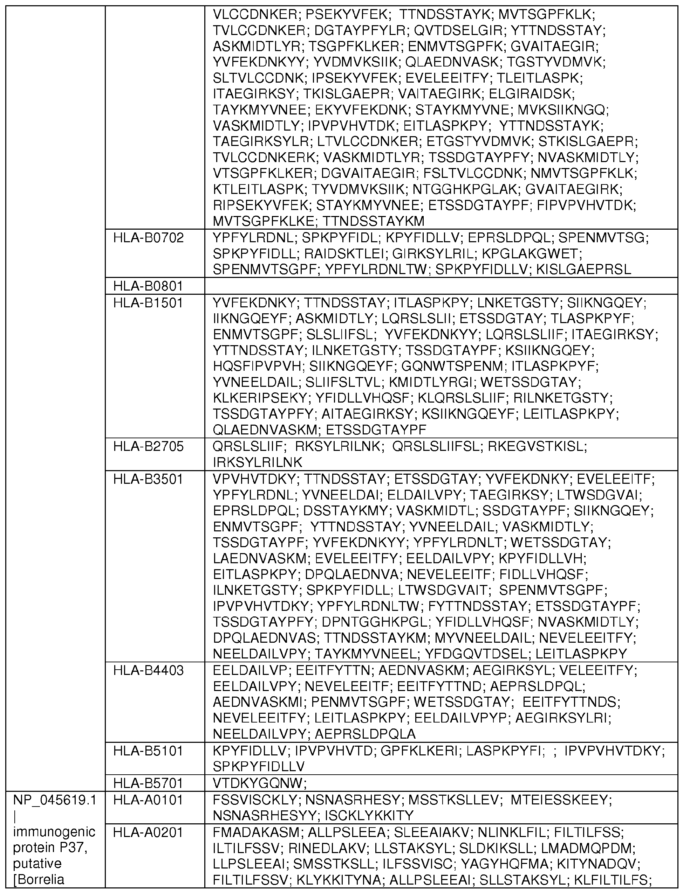

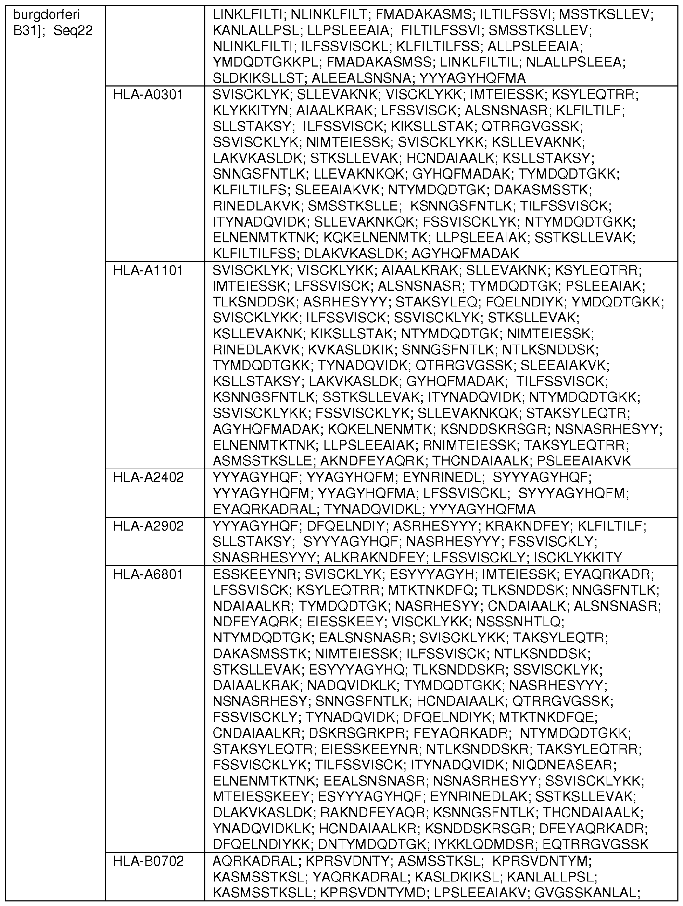

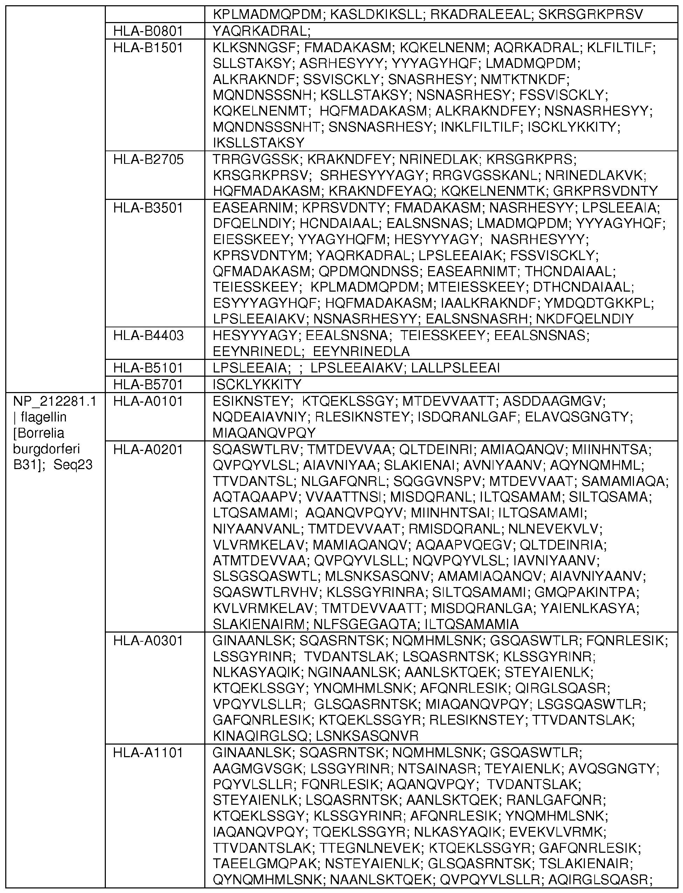

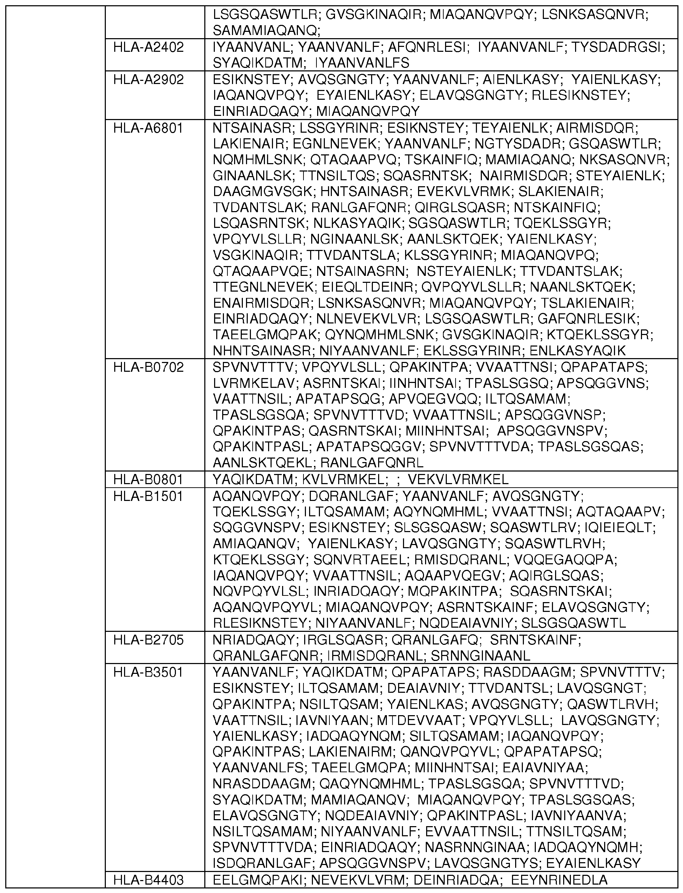

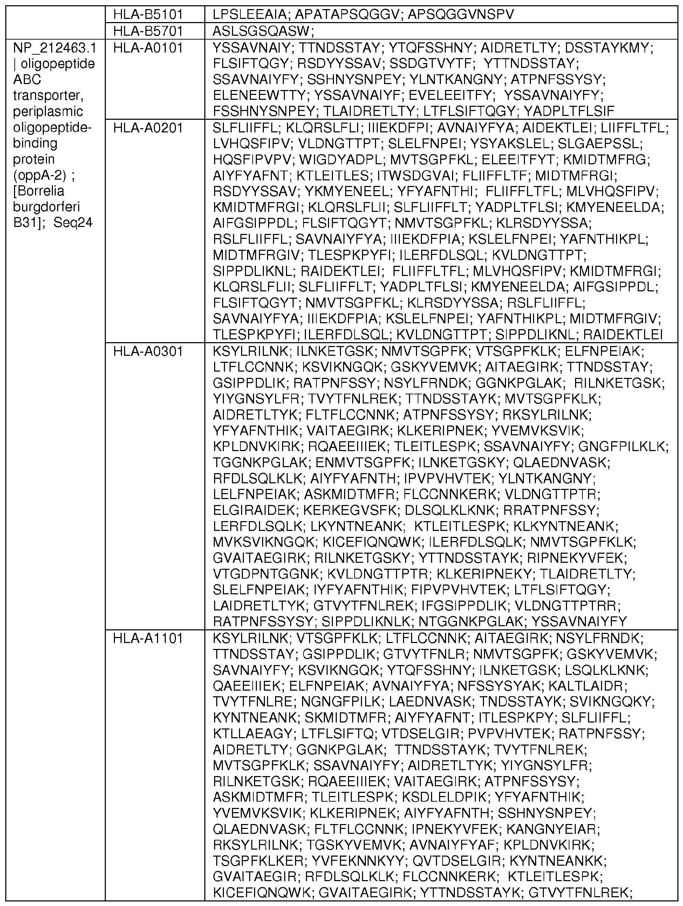

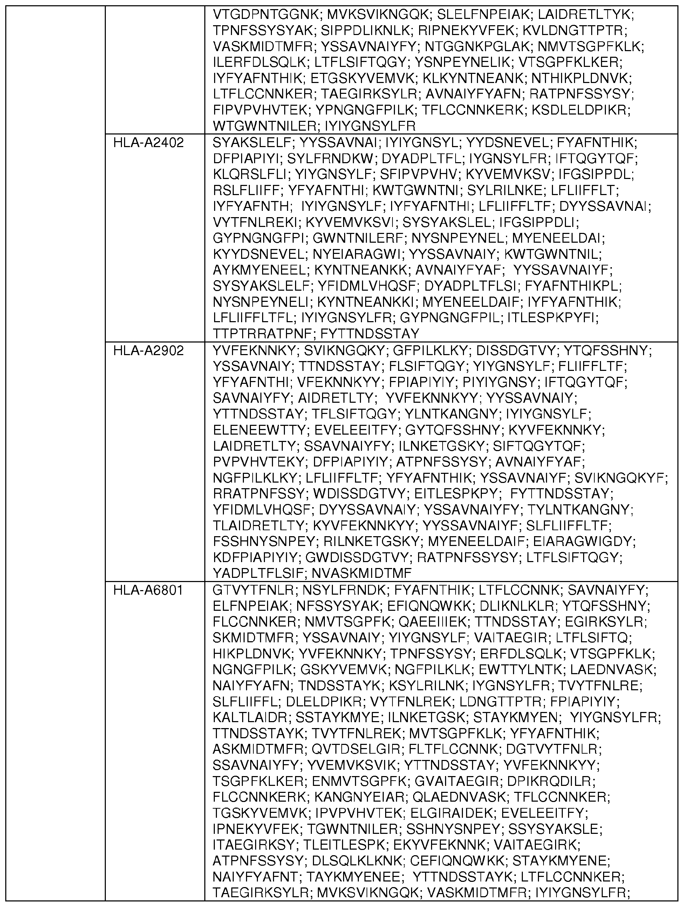

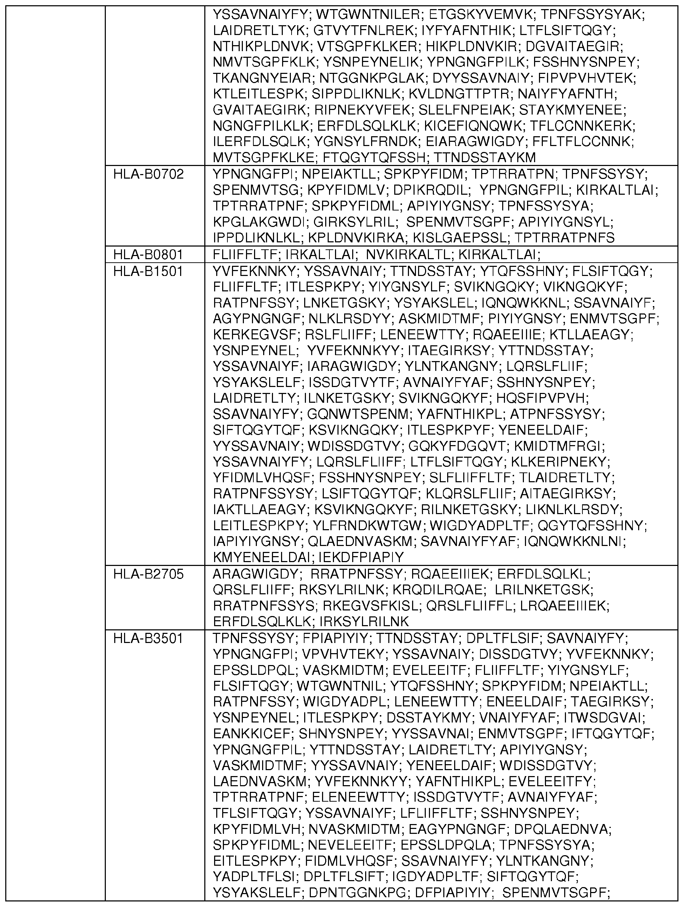

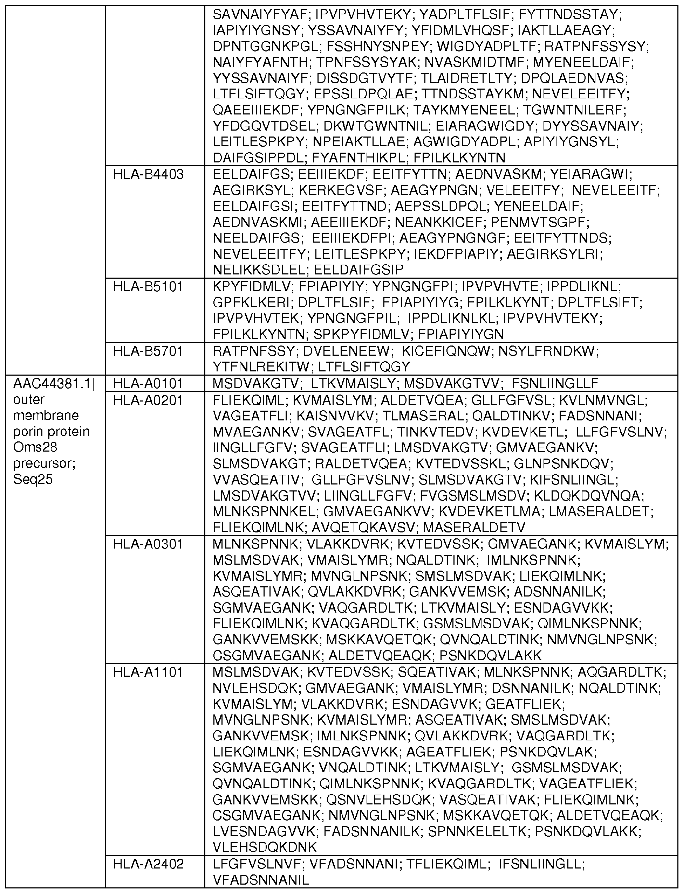

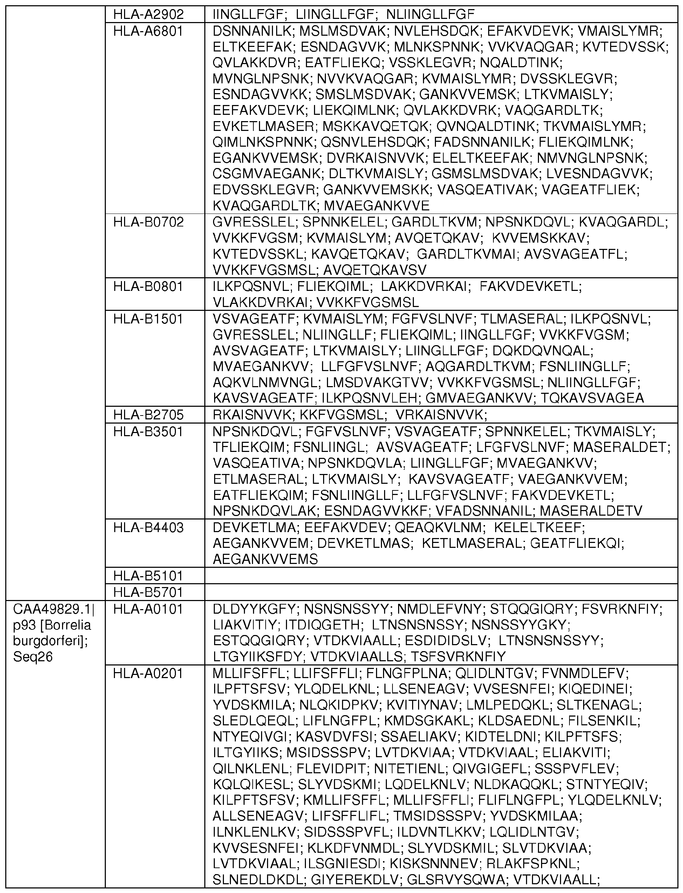

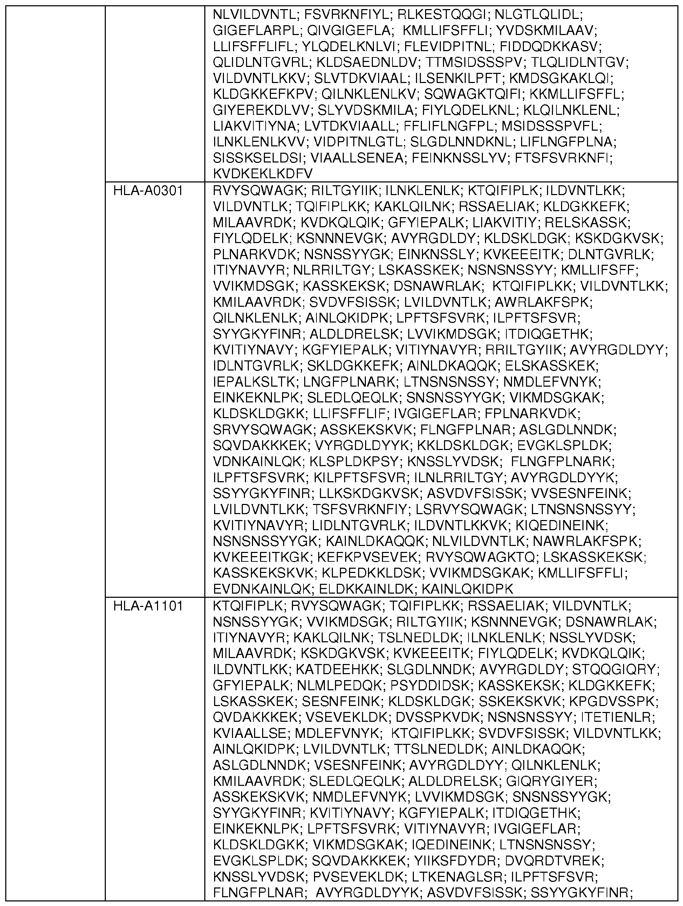

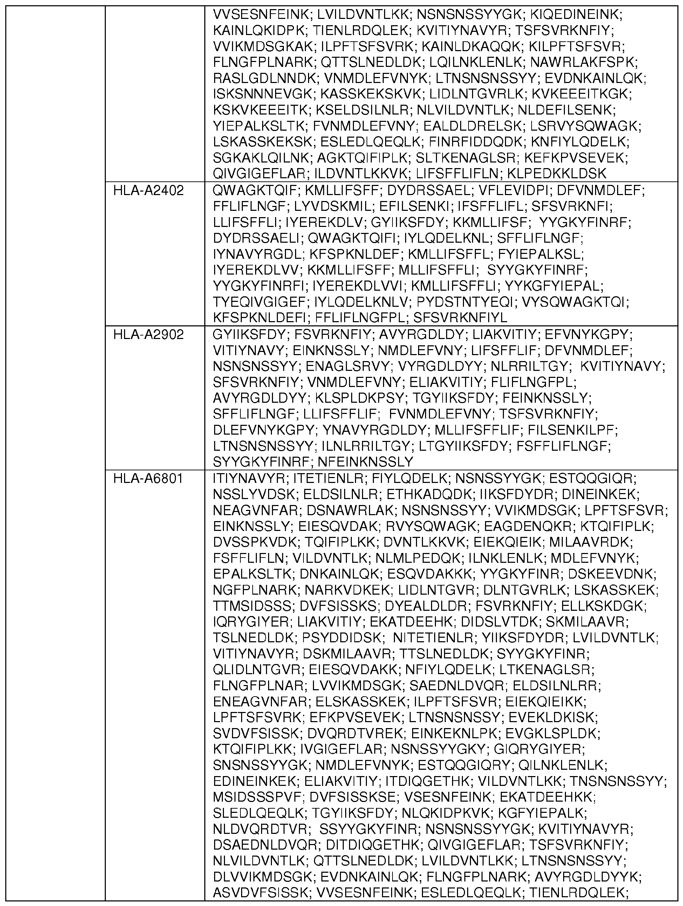

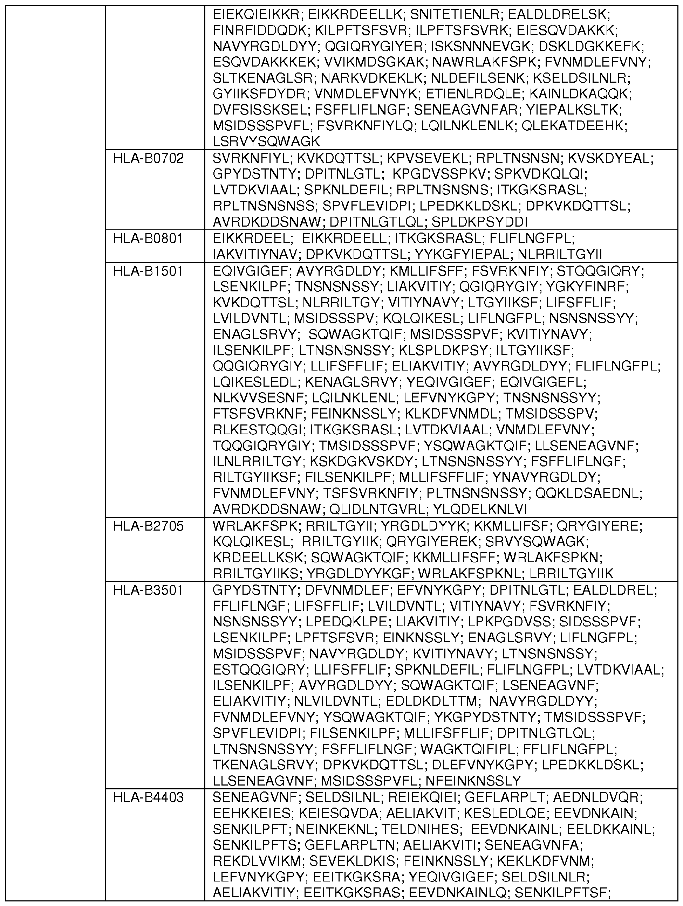

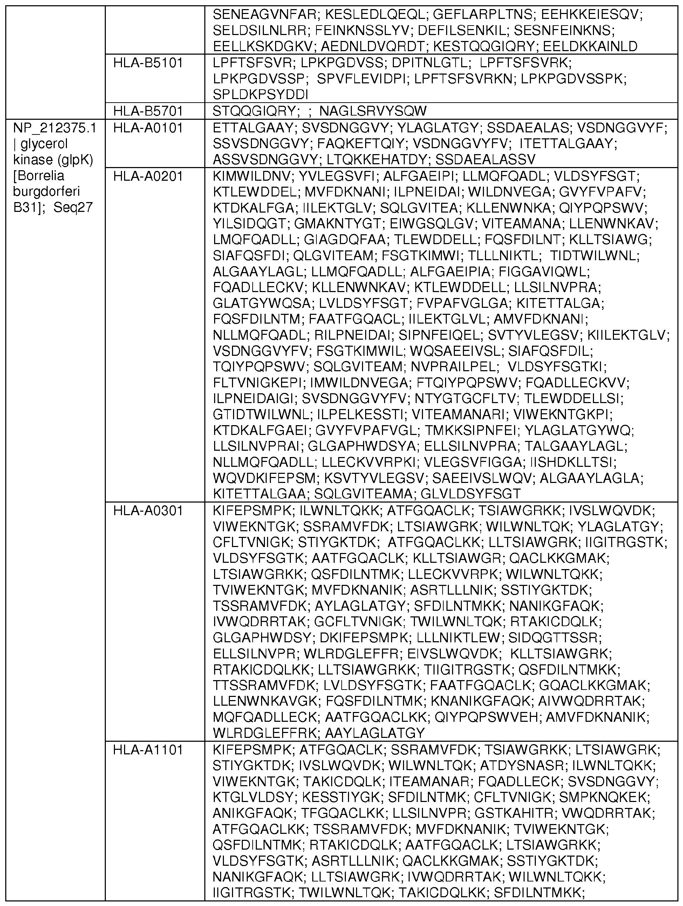

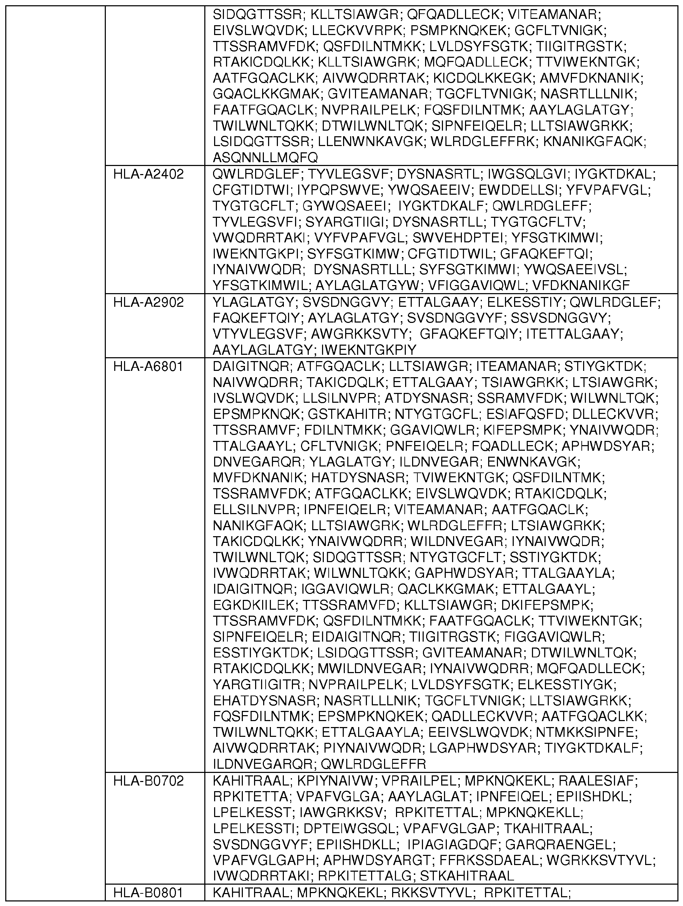

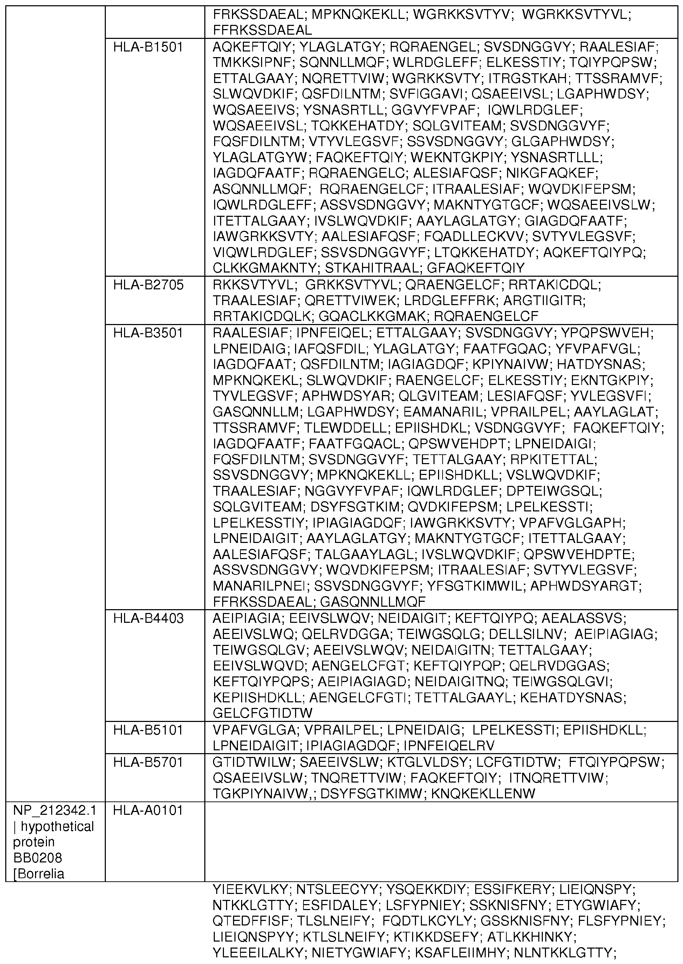

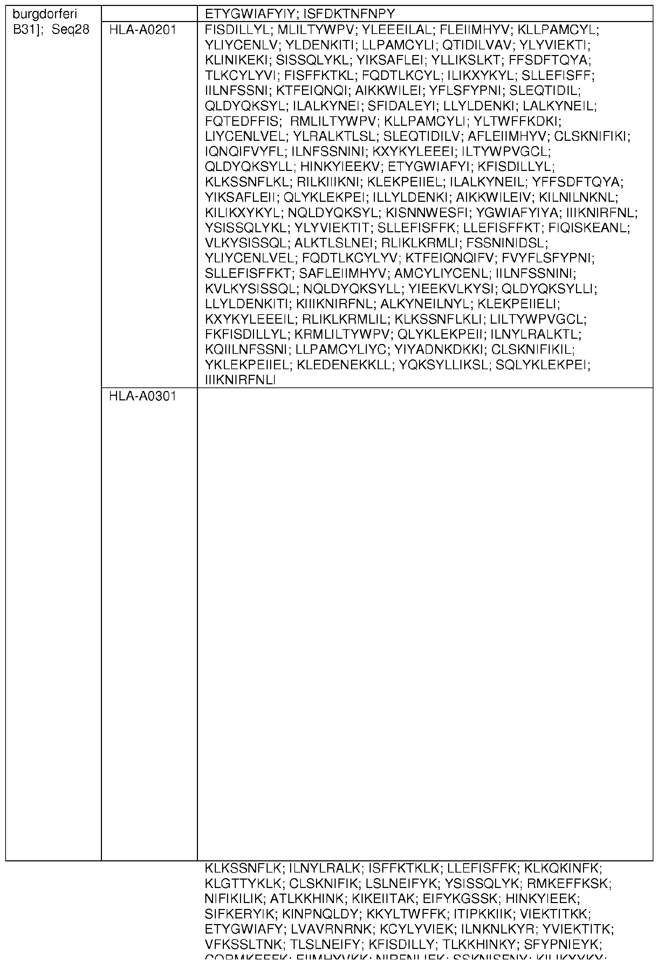

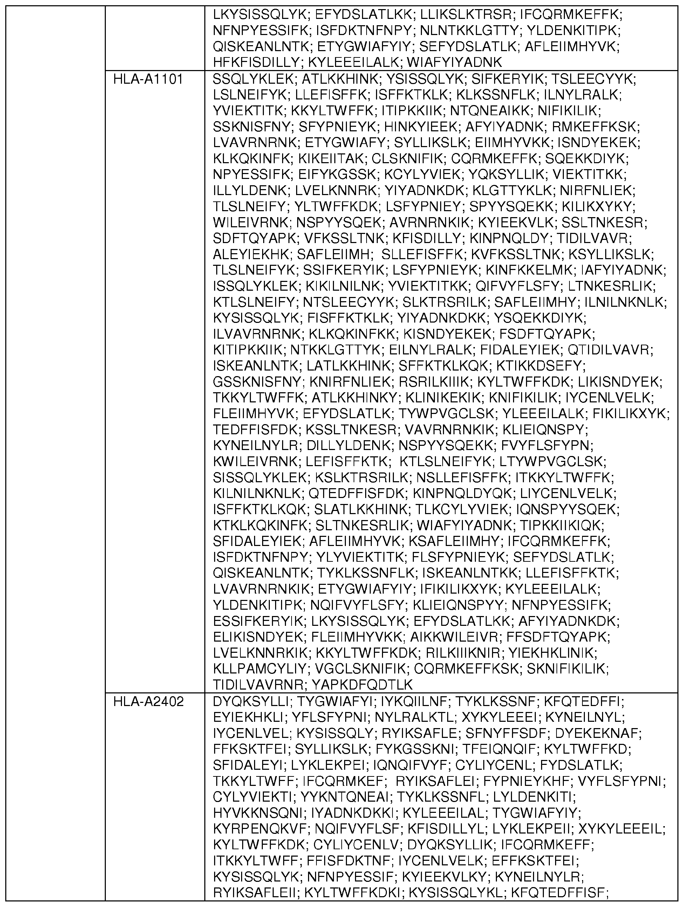

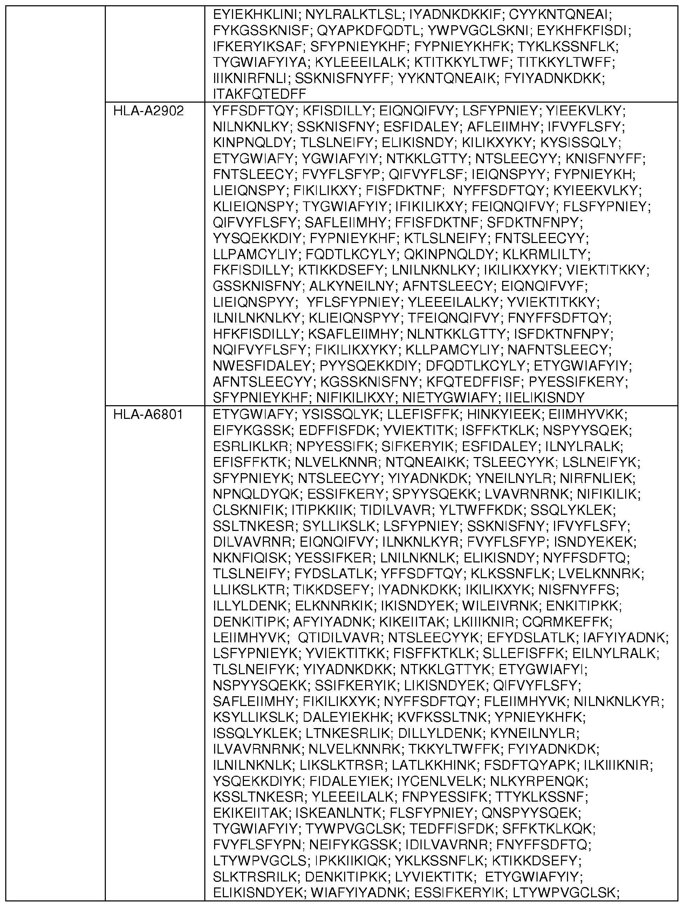

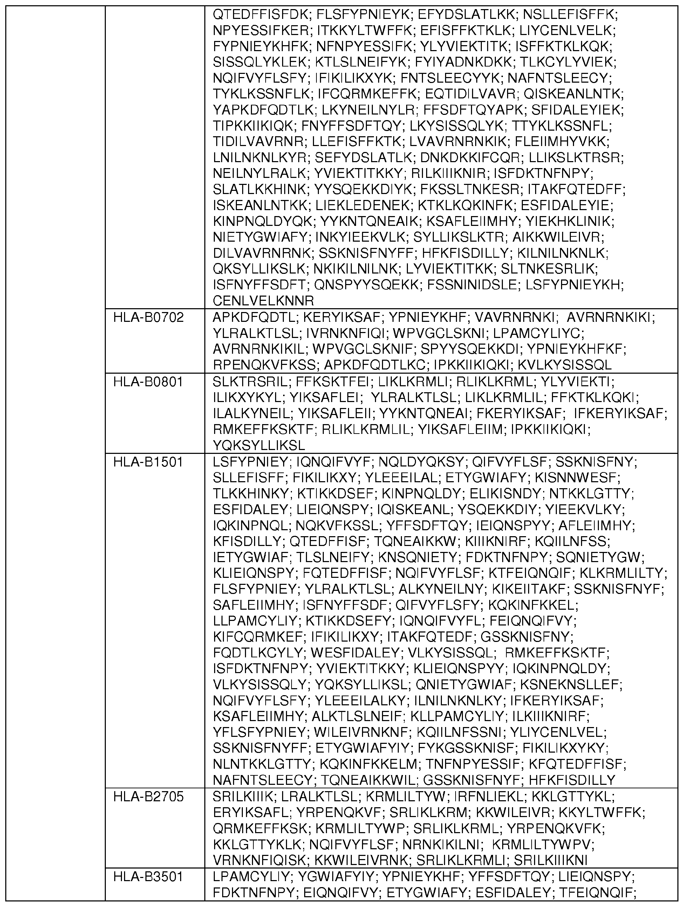

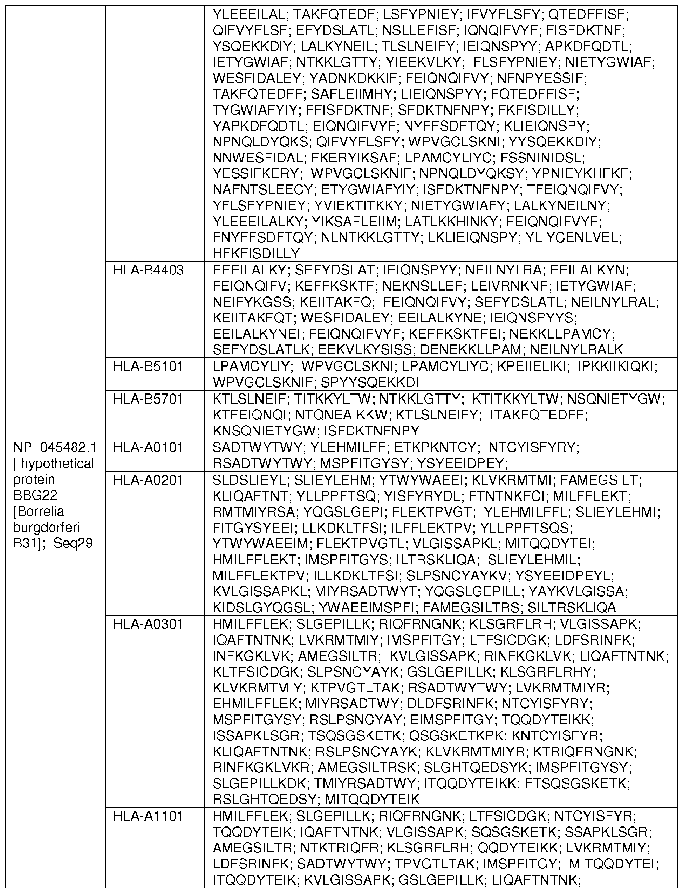

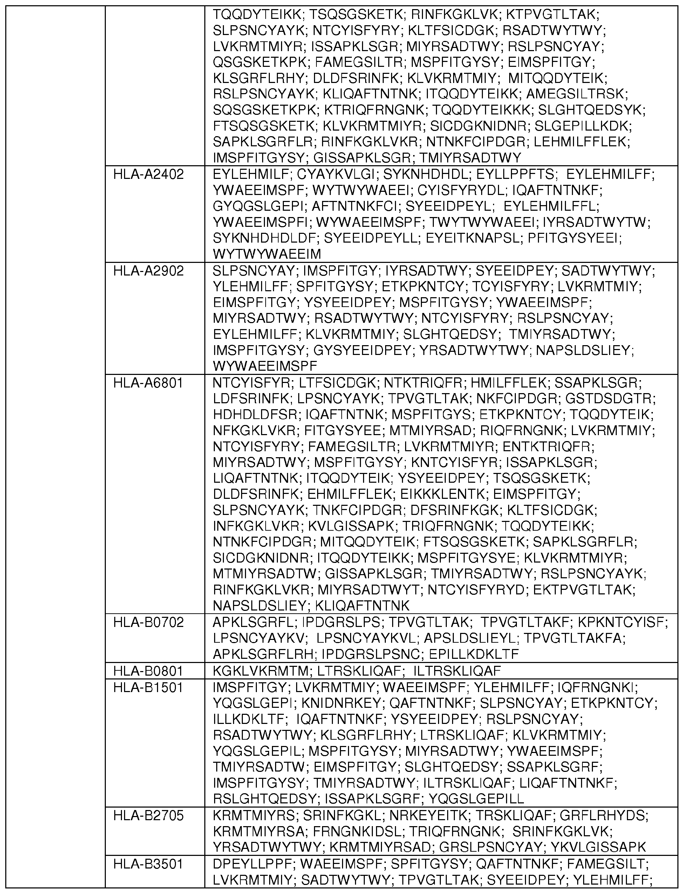

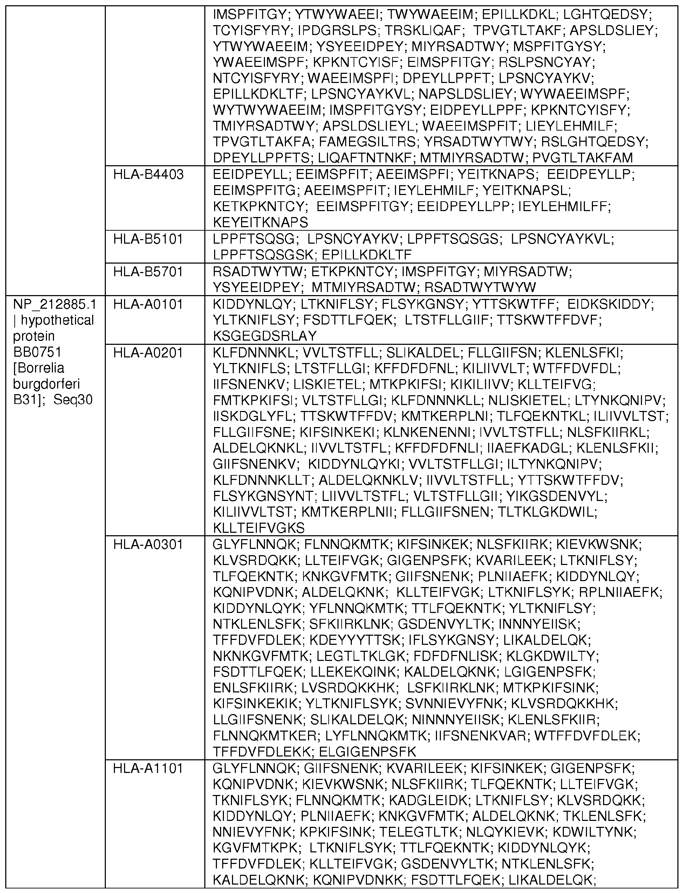

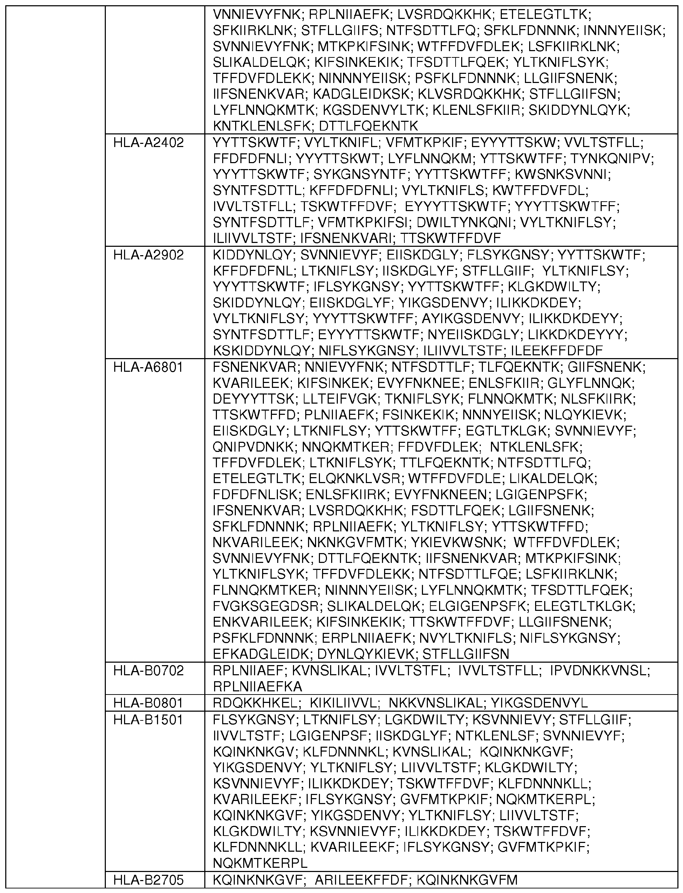

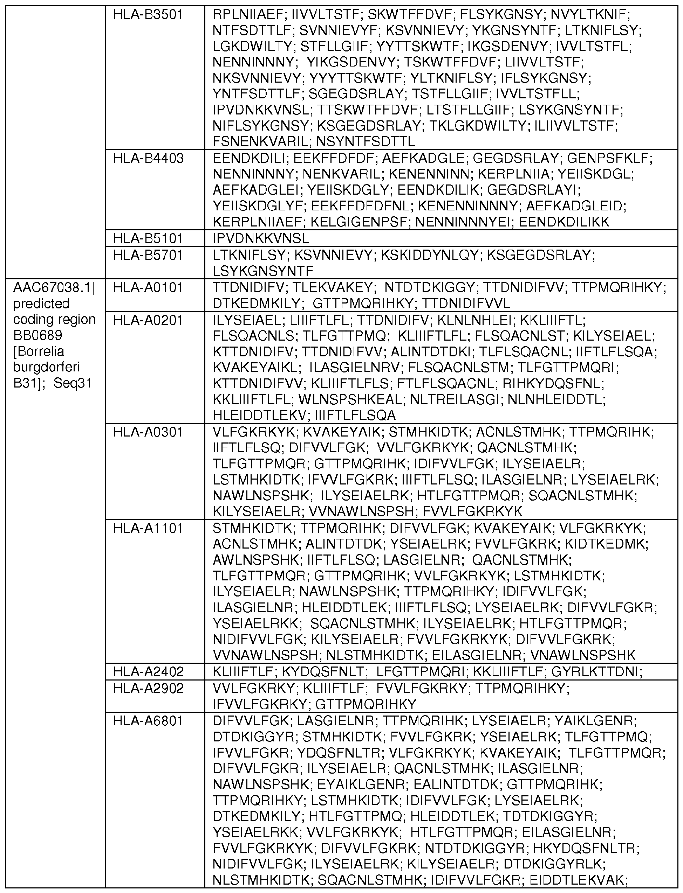

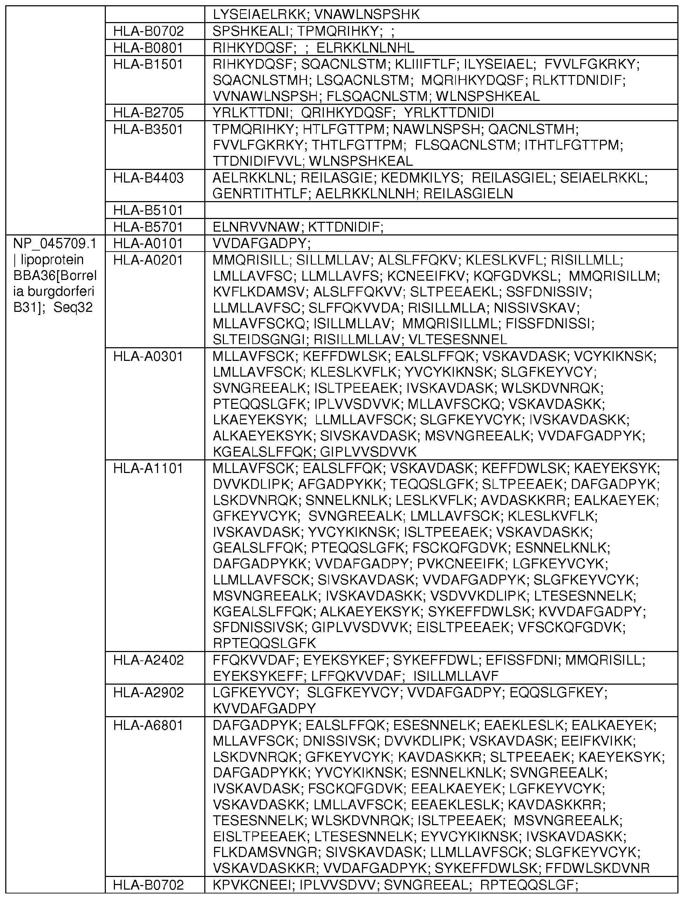

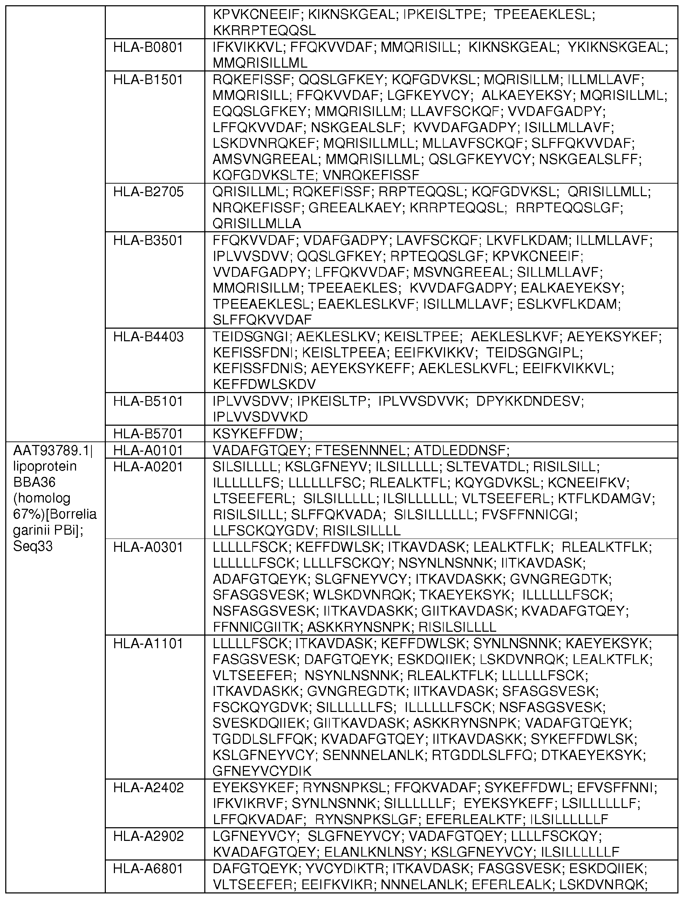

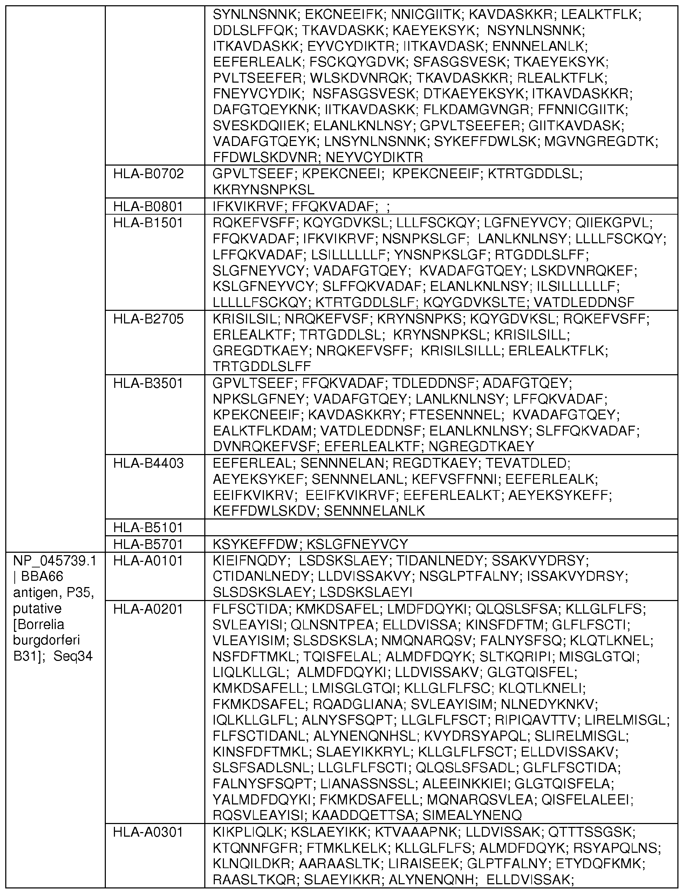

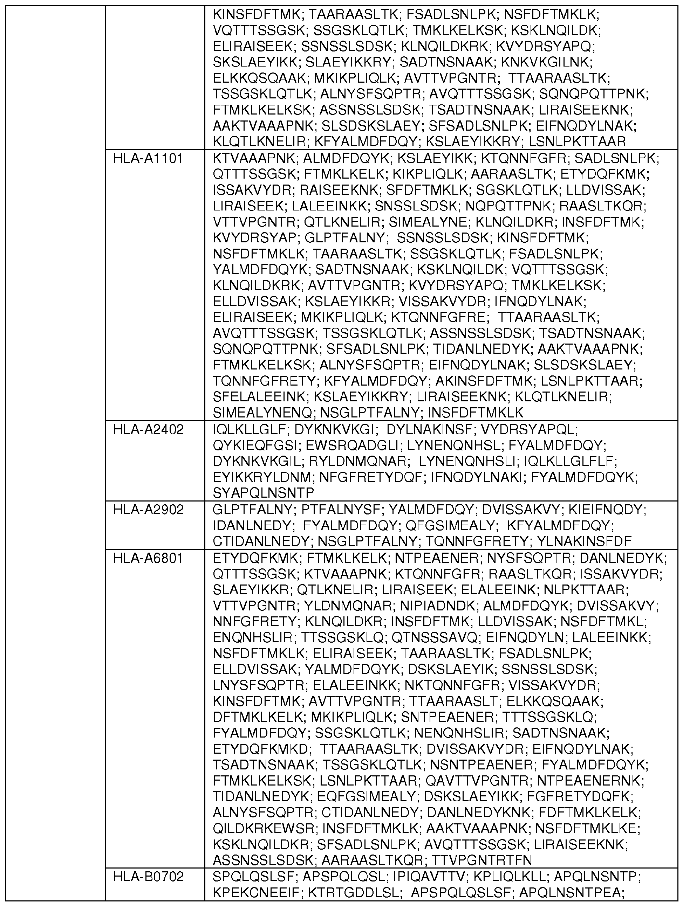

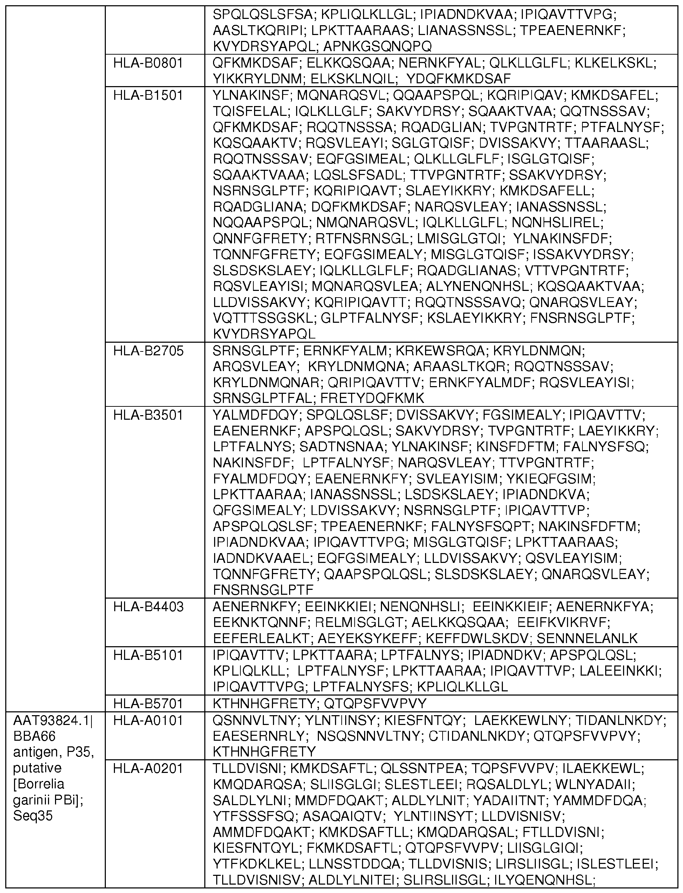

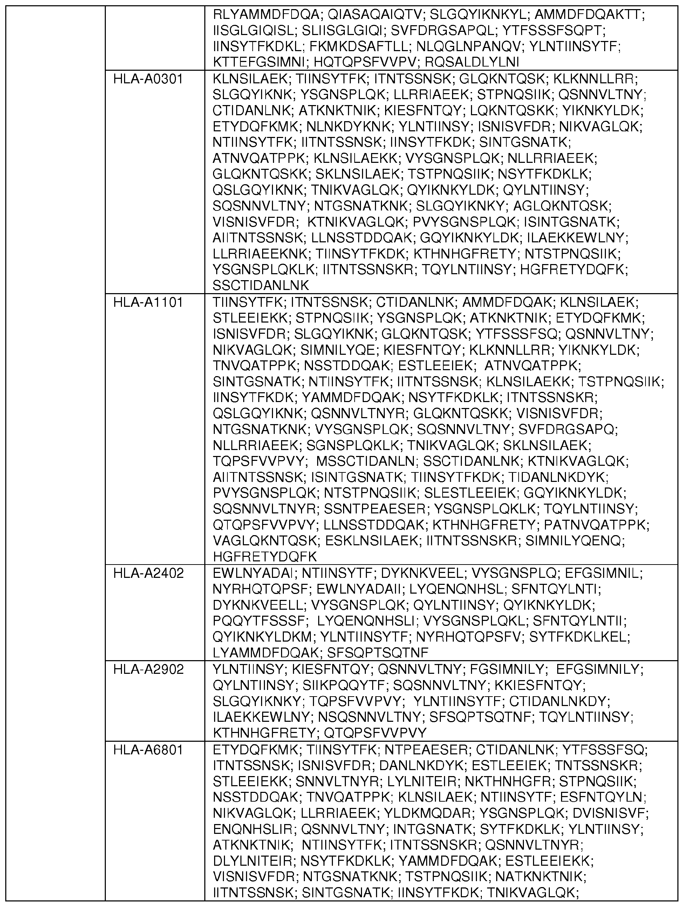

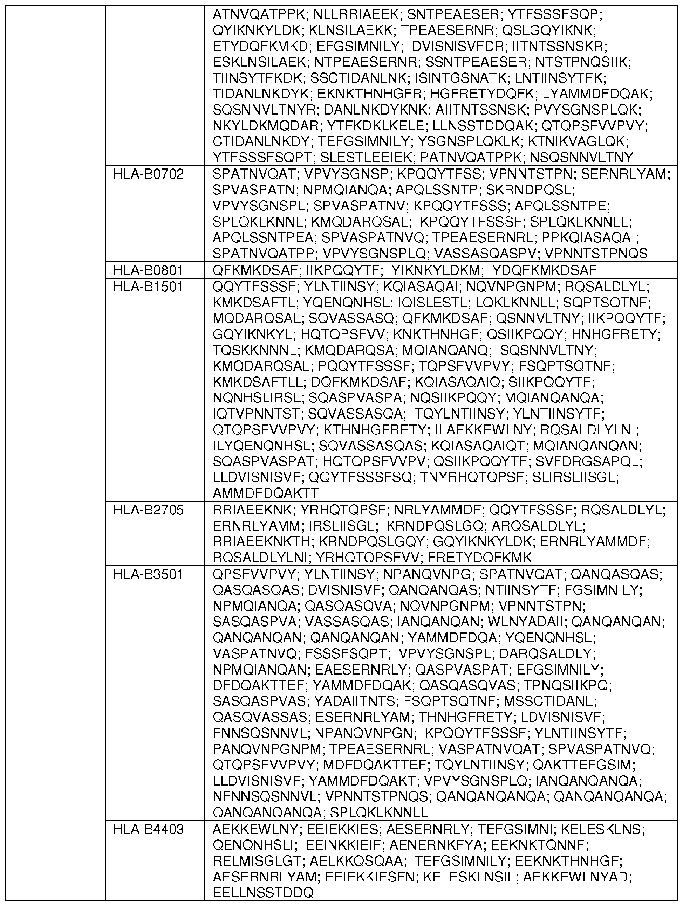

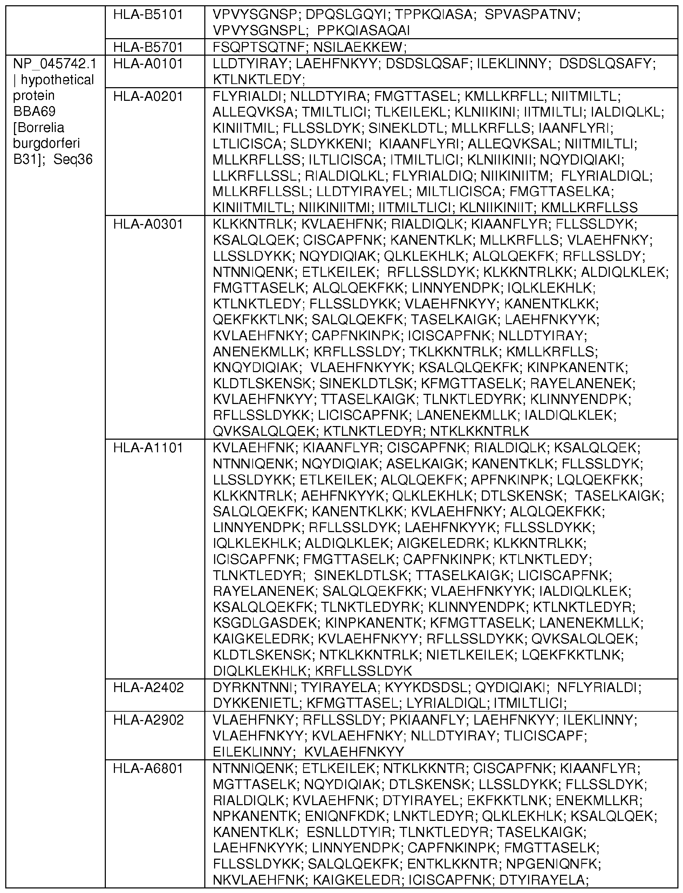

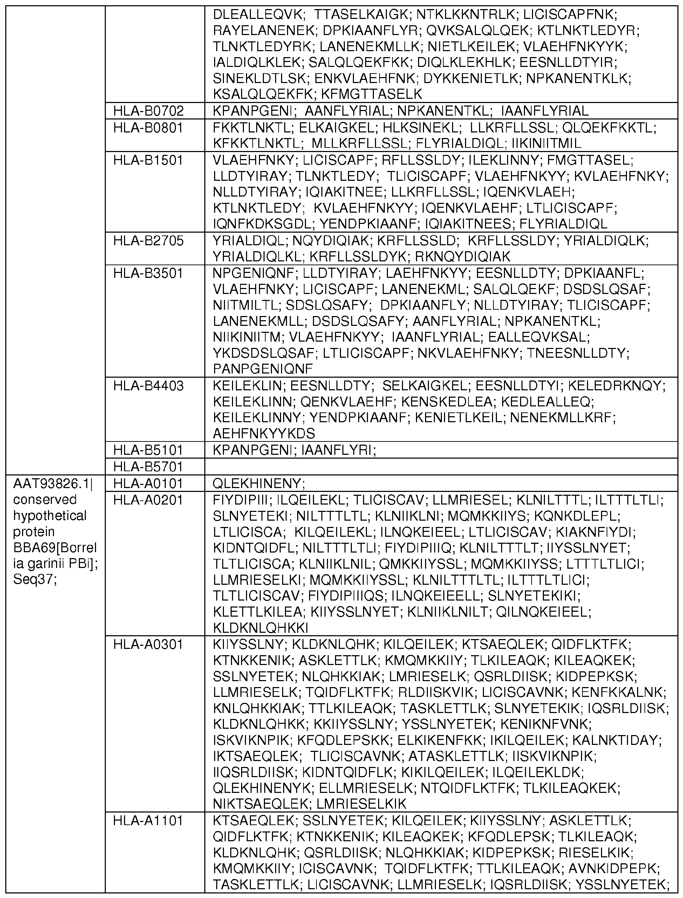

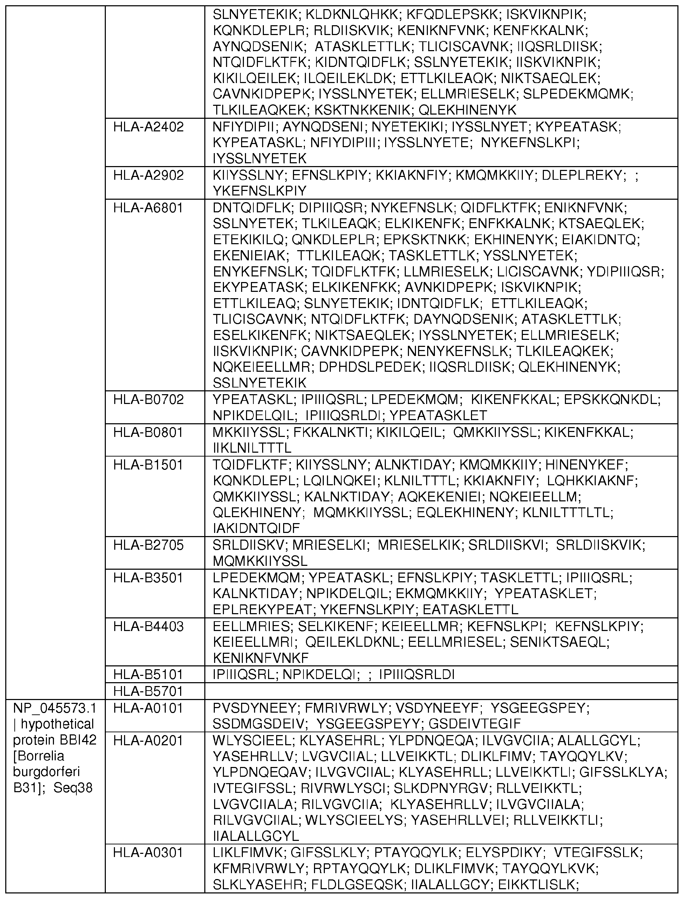

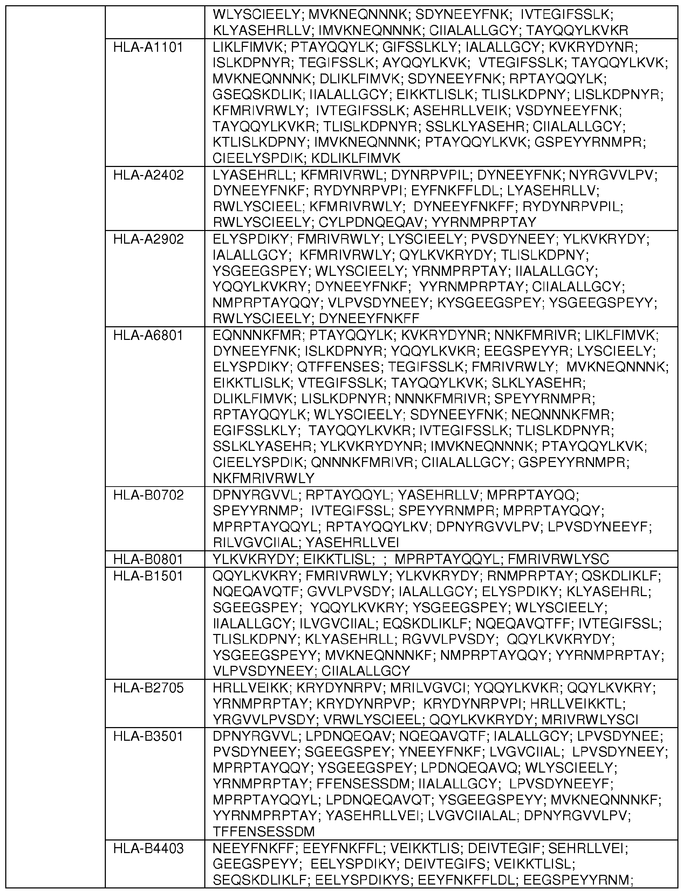

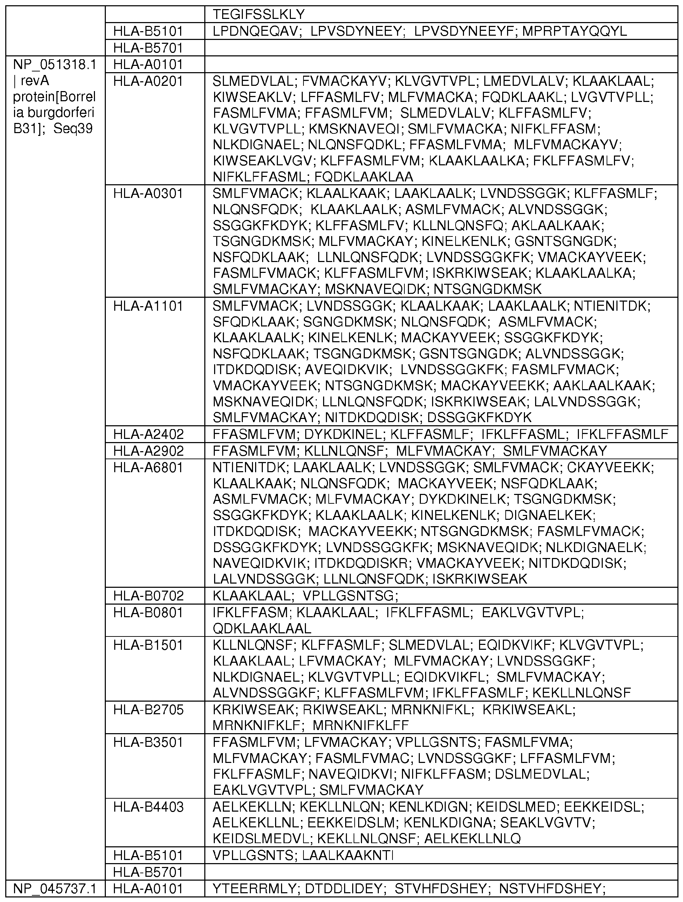

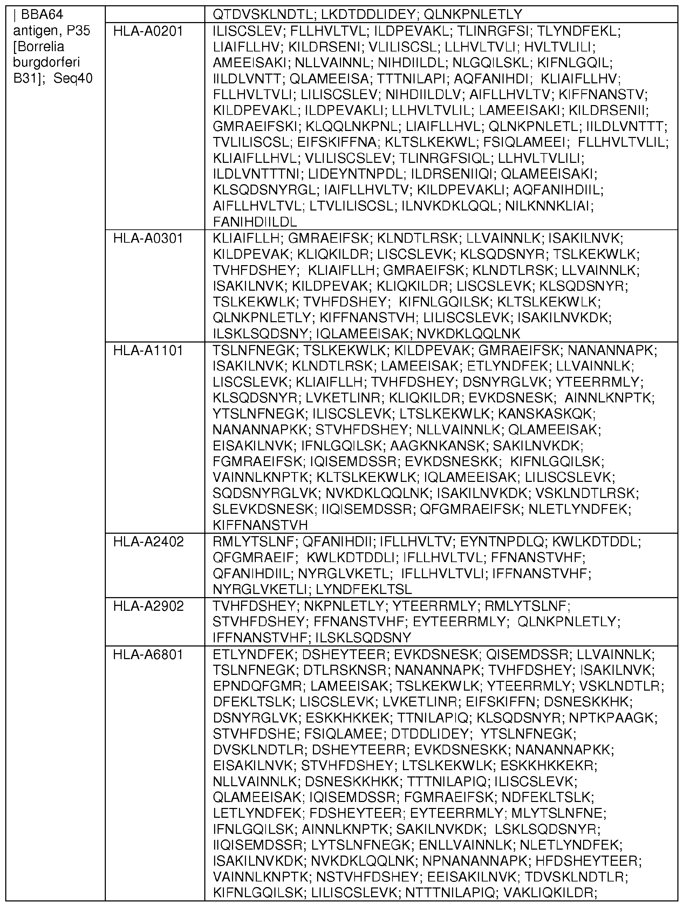

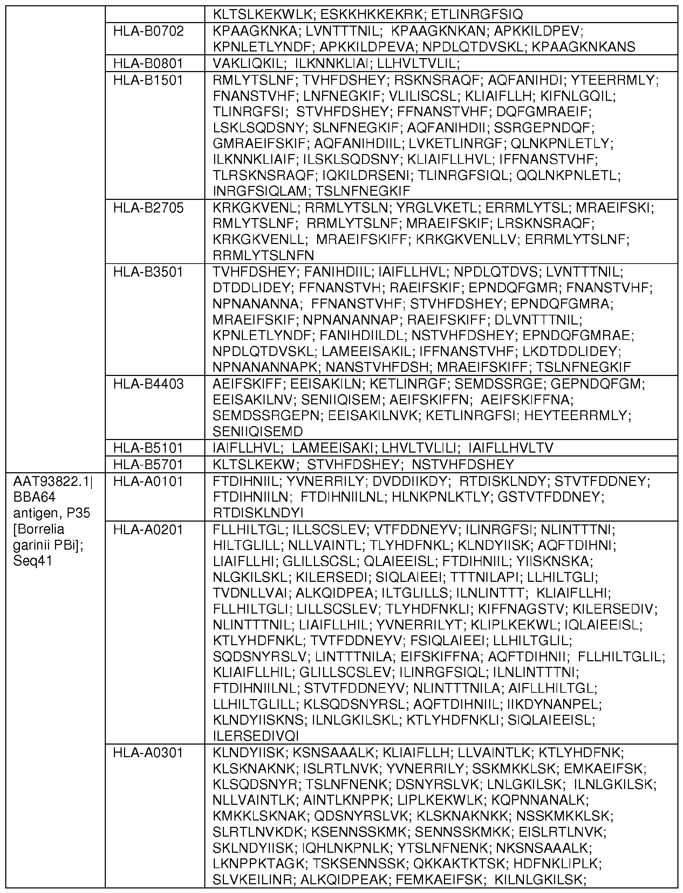

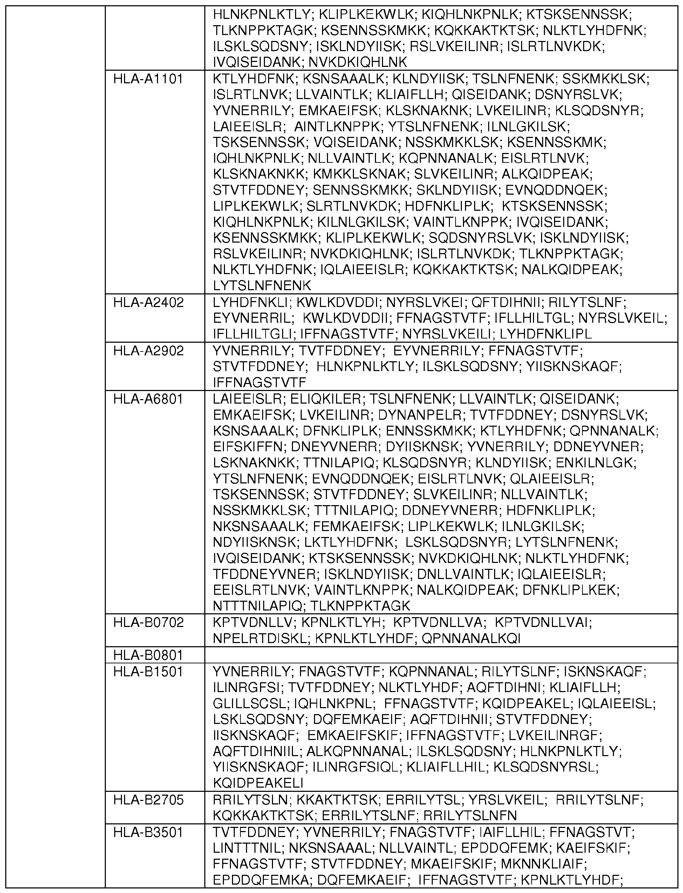

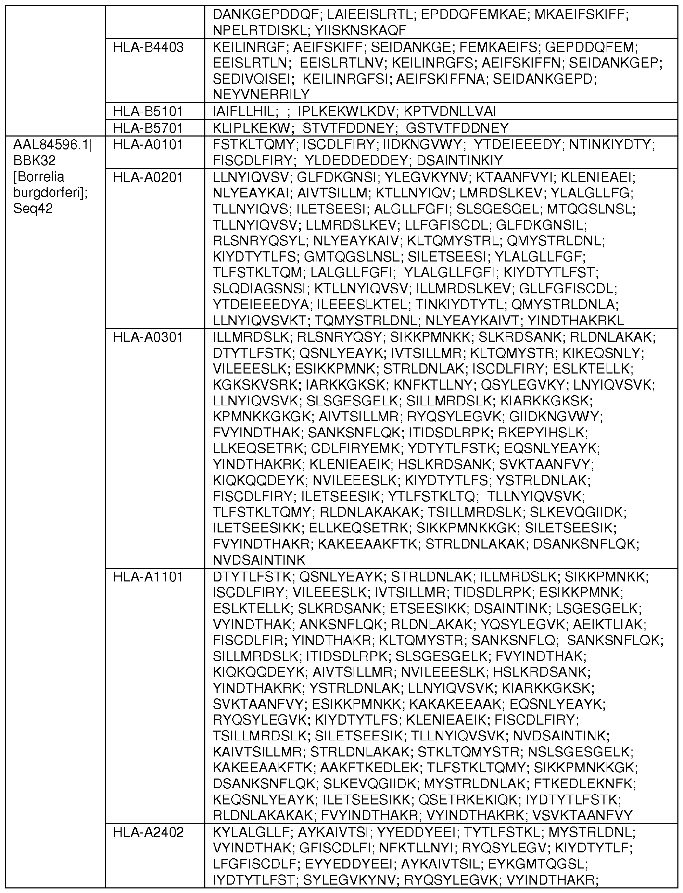

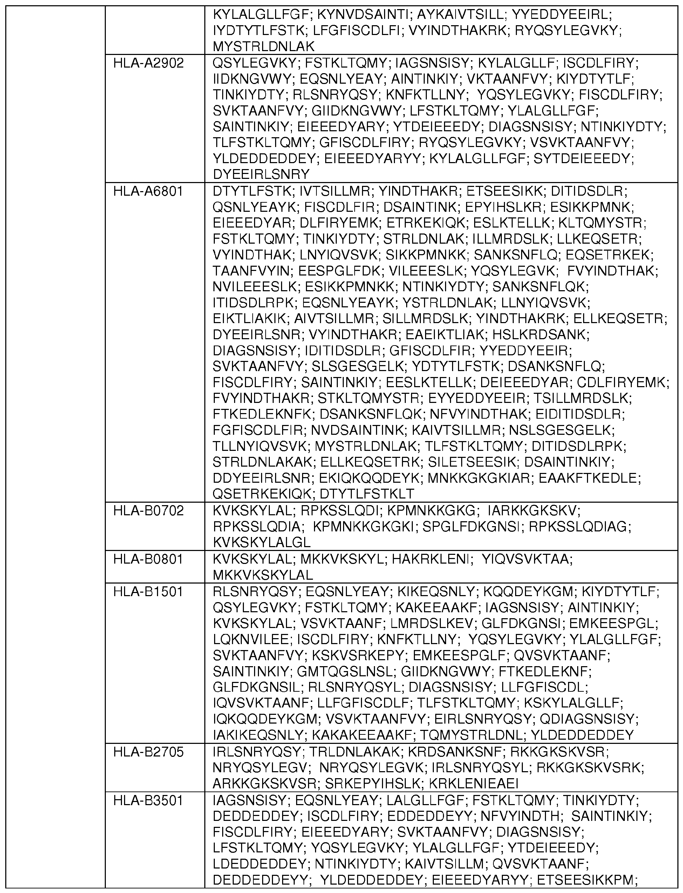

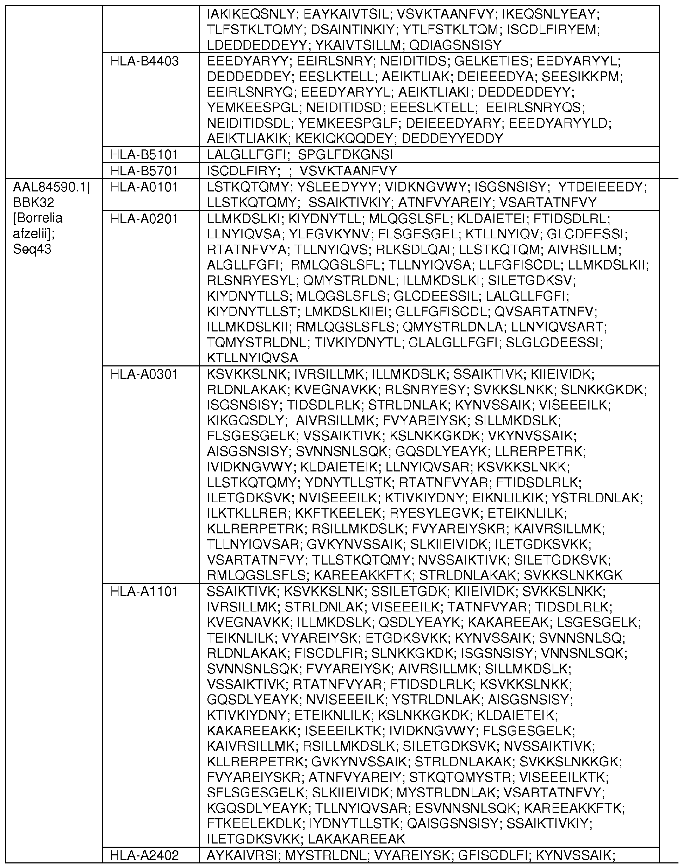

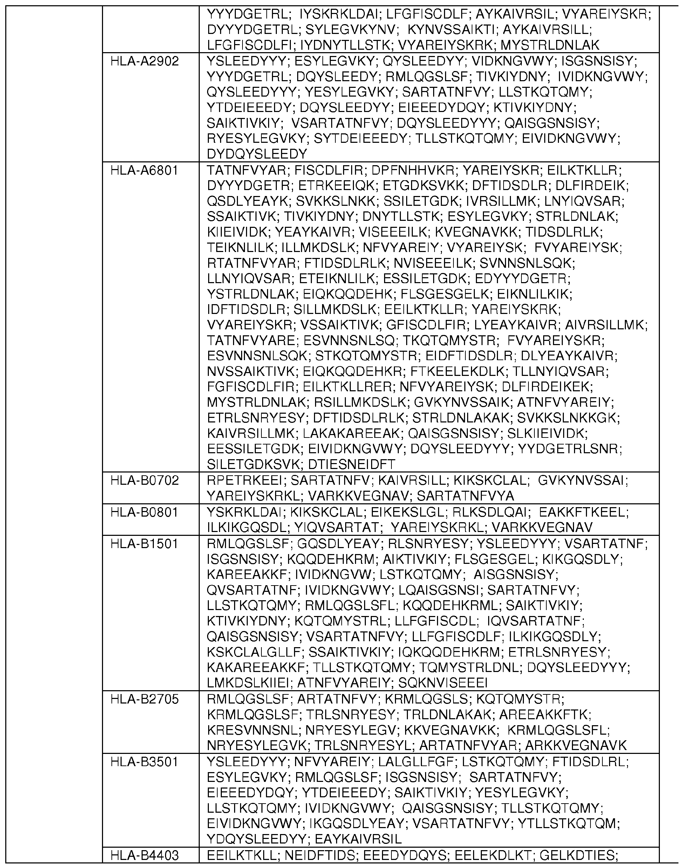

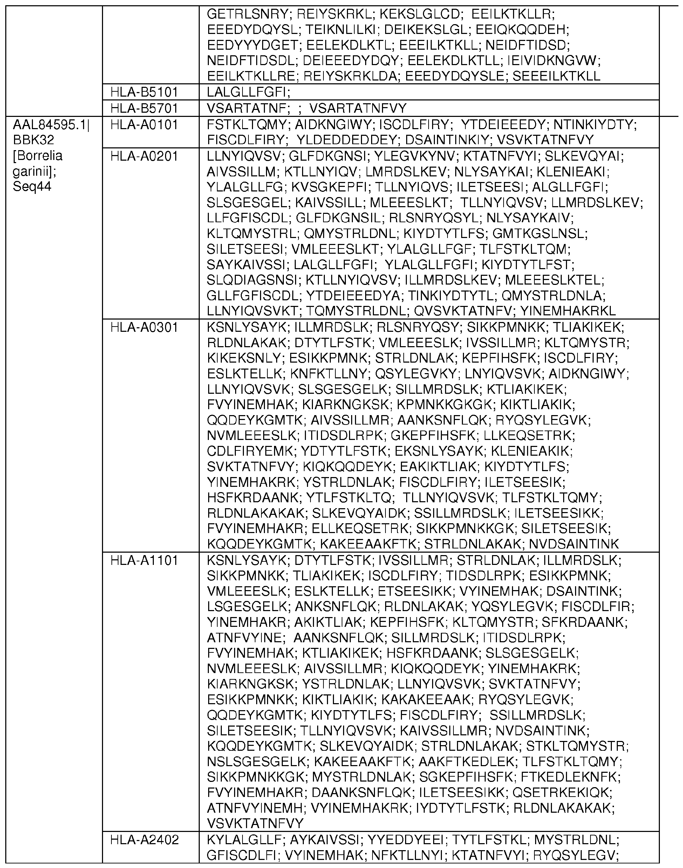

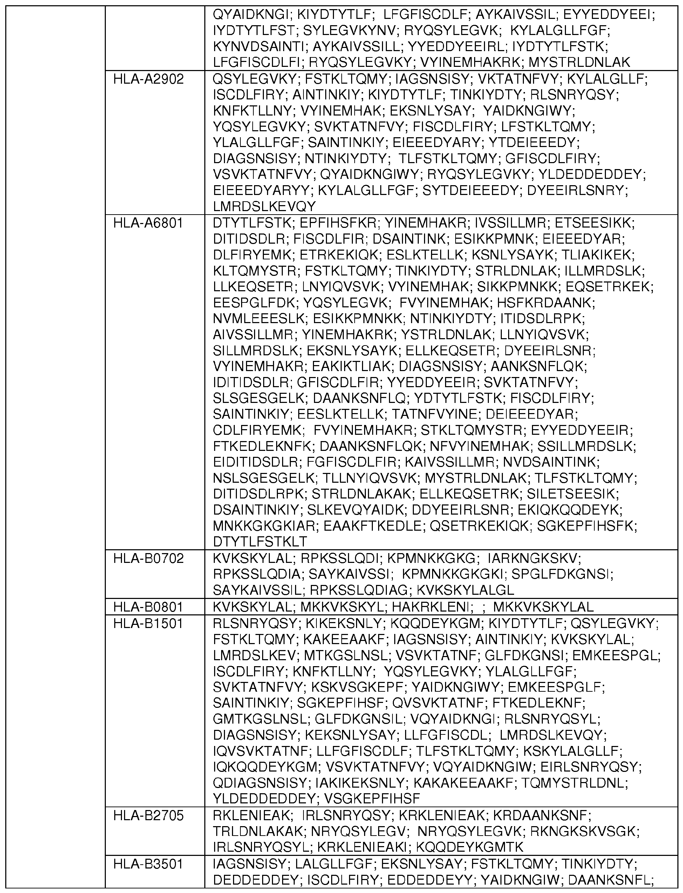

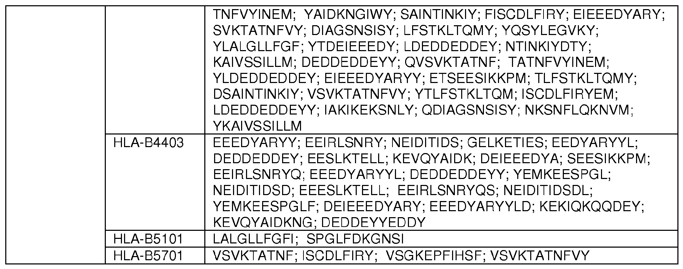

Figure 32. Borrelia MHC class 1 antigen peptides of 8-, 9-, 10- and 11 amino acids in length generated from the chosen protein sequences by use of software based on neural prediction of MHC binding peptides. Peptide sequences are grouped according to HLA binding alleles and sub-grouped according to peptide length.

Figure 33. Borrelia MHC class 2 antigen peptides of 13-, 14-, 15-, and 16 amino acids in length generated from the chosen protein sequences by random prediction software (see figure 2).

Figure 34. Borrelia MHC class 2 antigen peptides of 15 amino acids in length generated from the chosen protein sequences by use of software based on neural prediction of MHC binding peptides. Peptide sequences are grouped according to HLA binding alleles and sub-grouped according to peptide length. For each 15-mer binding peptide the essential 9-mer core sequence is given.

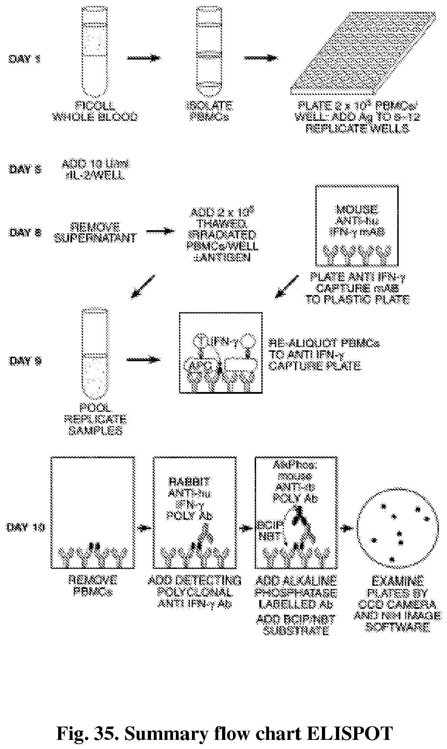

Figure 35. Summary flow chart ELISPOT. Summary flow chart showing measurement of antigen reactive T-Cells by IFN-$\gamma$ capture in blood samples by ELISPOT. See example 31 for more detailed information.

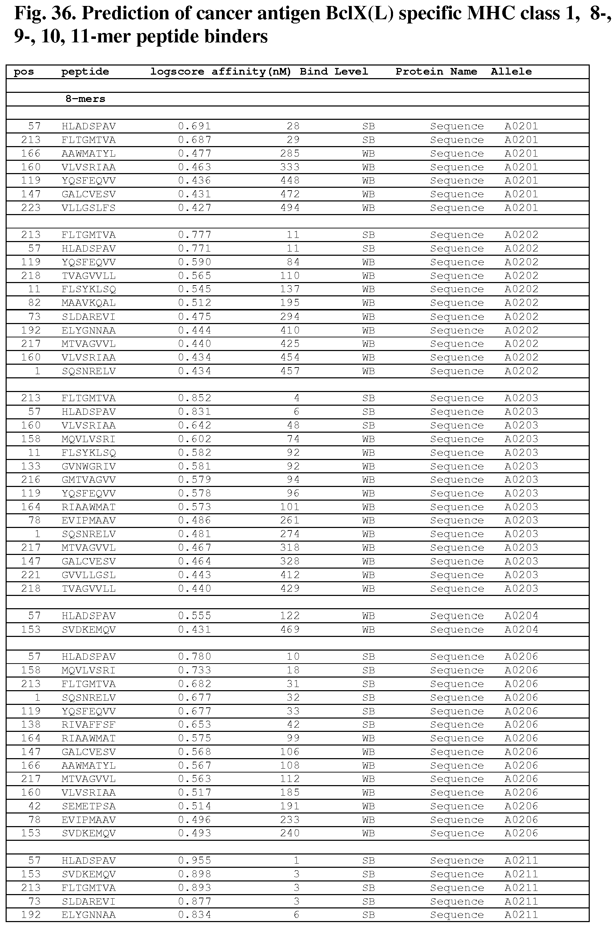

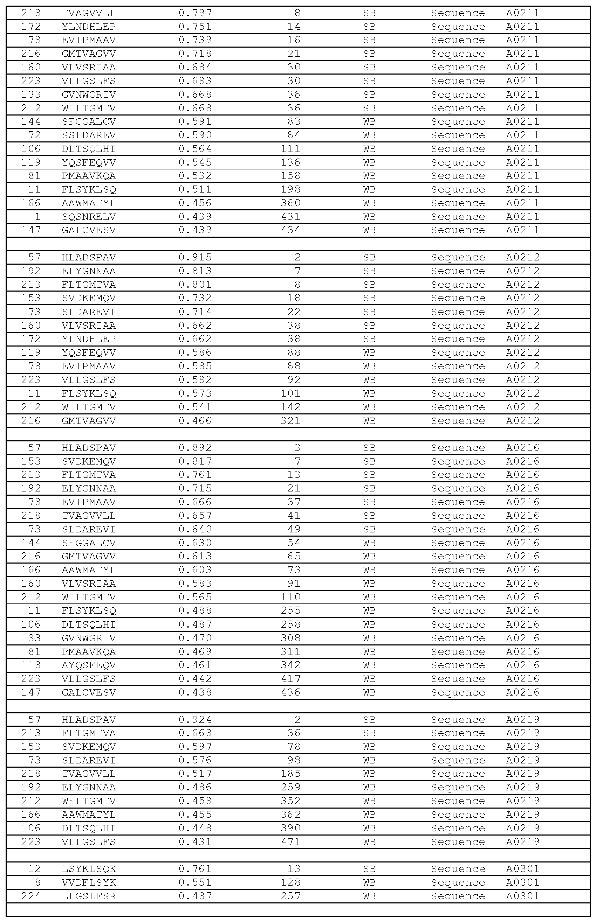

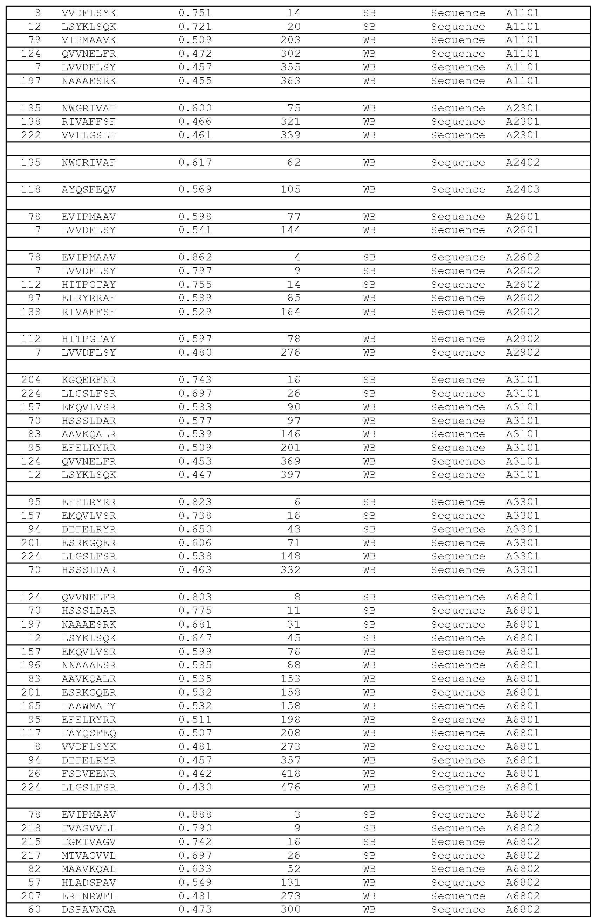

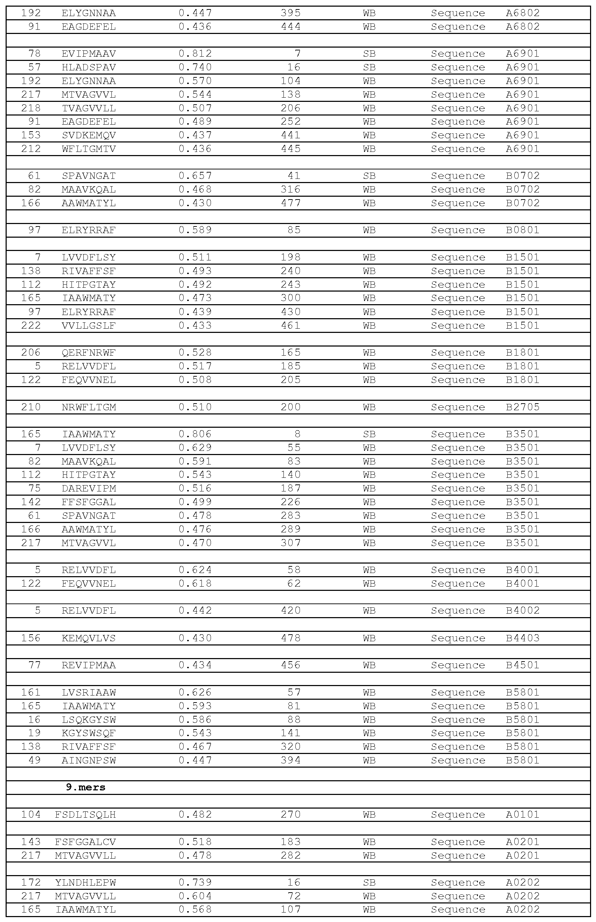

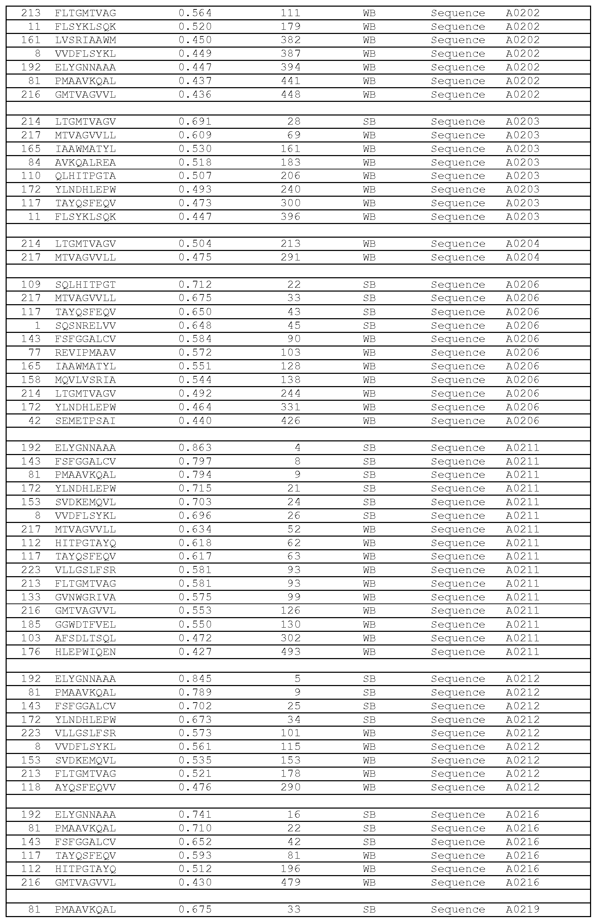

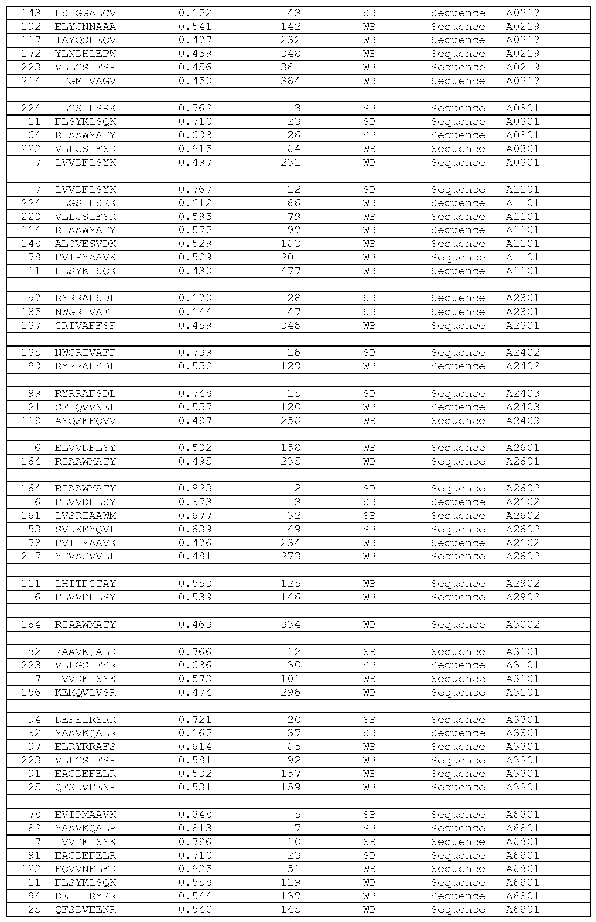

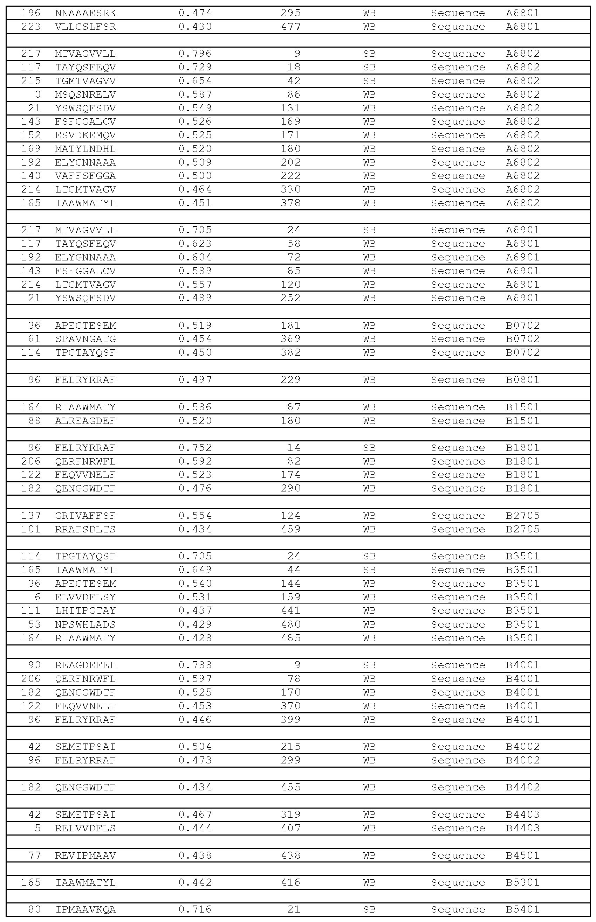

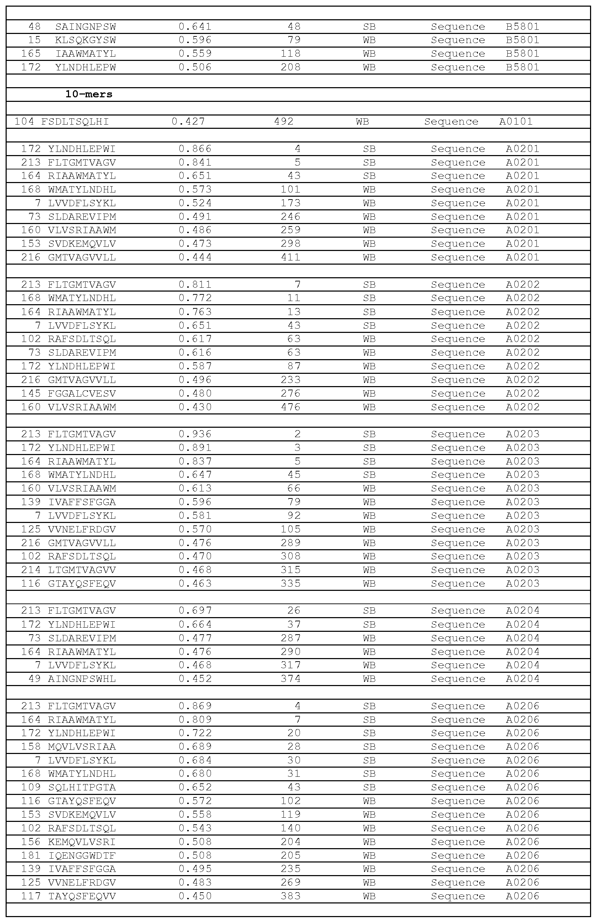

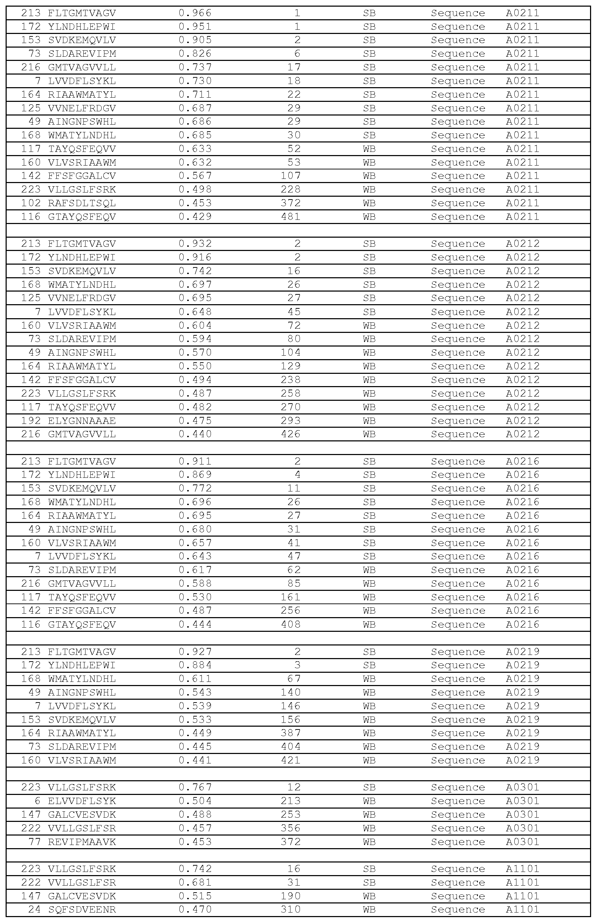

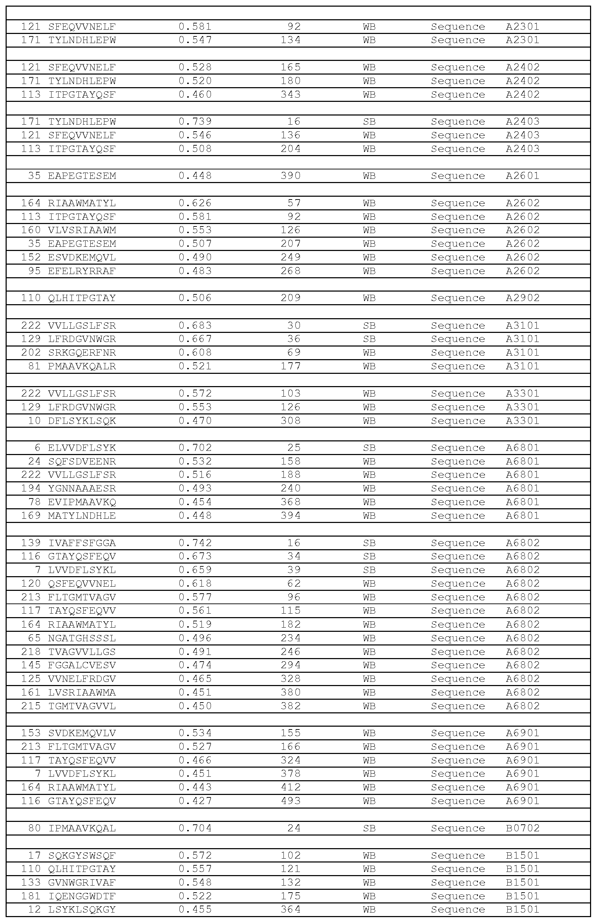

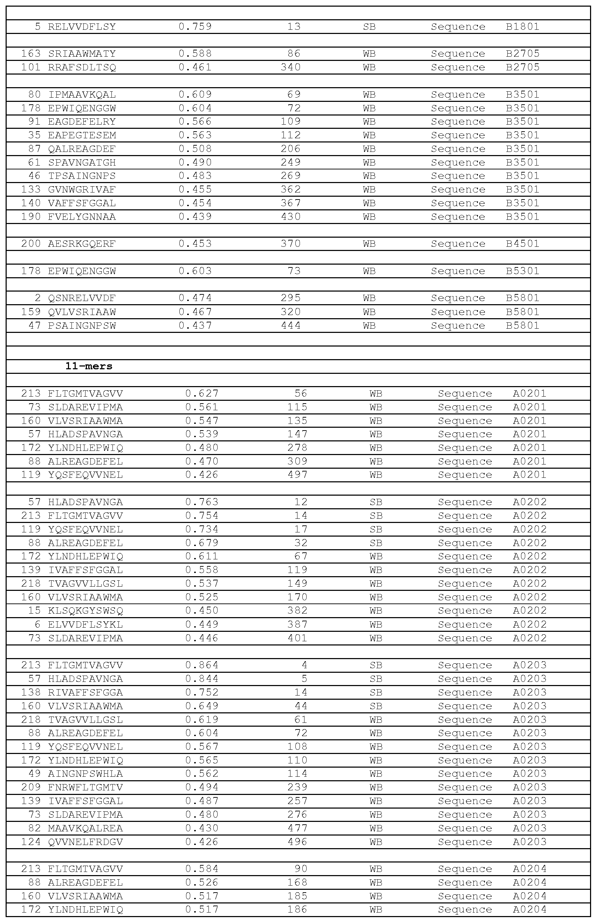

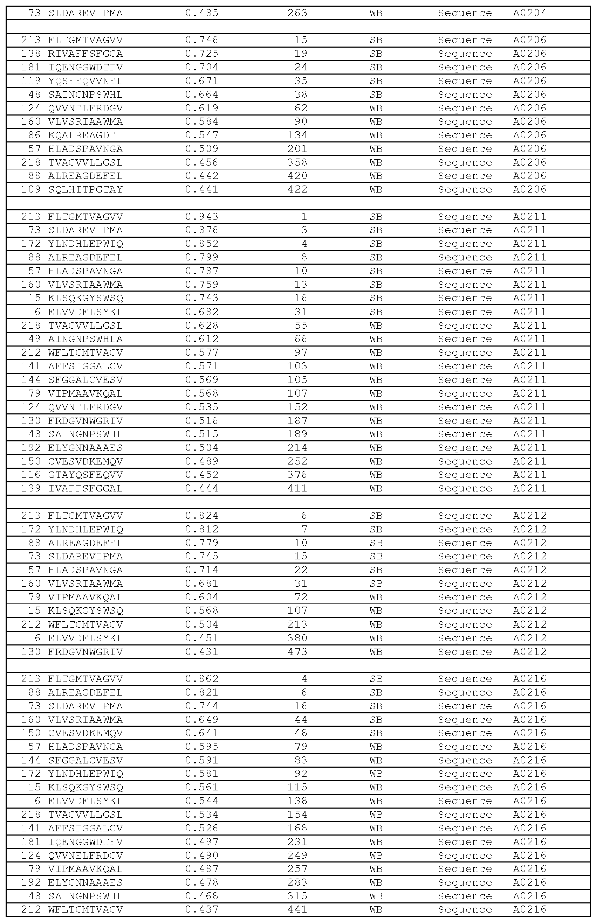

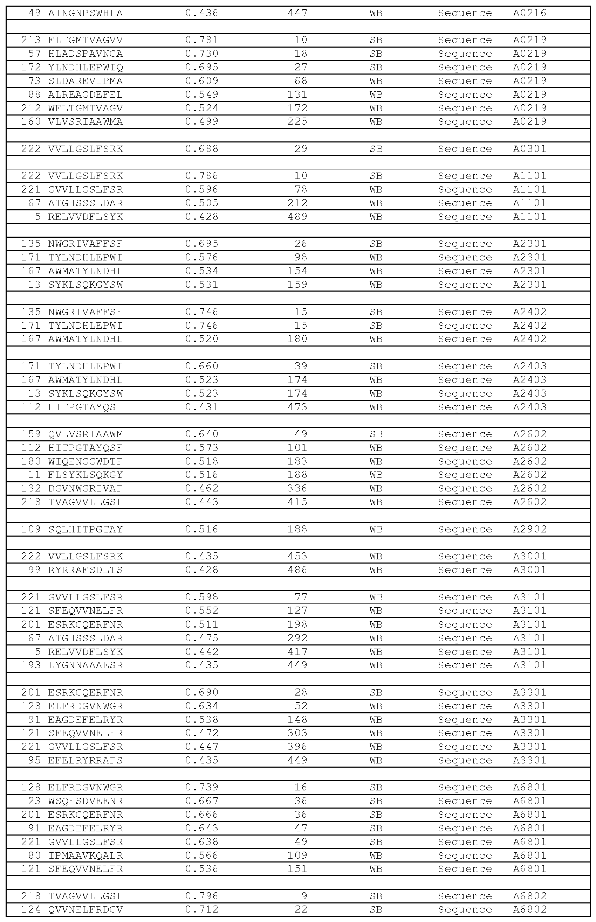

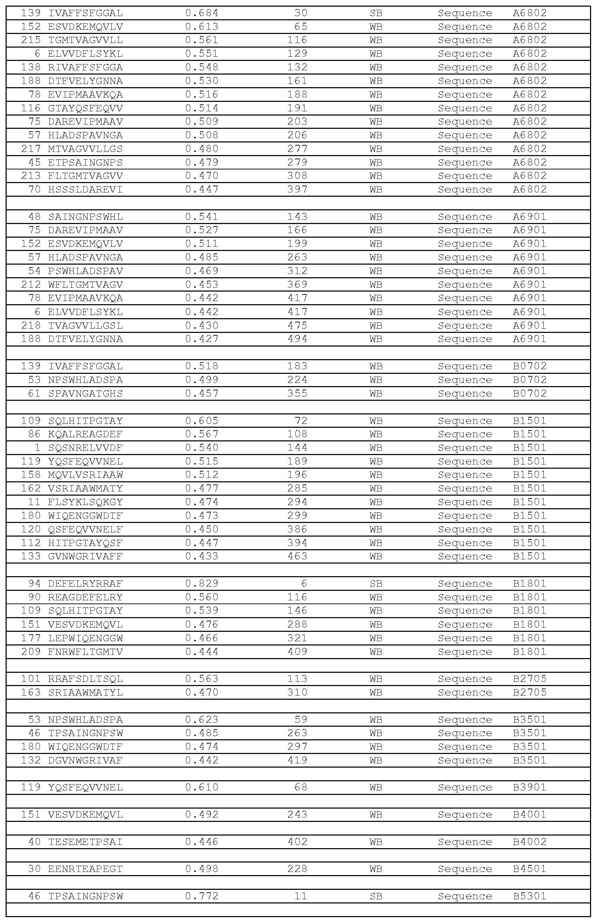

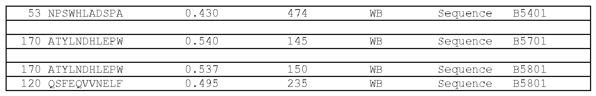

Figure 36. Prediction of cancer antigen BclX(L) specific MHC class1, 8-, 9- ,10- ,11-mer peptide binders. Prediction of cancer antigen BclX(L) specific MHC class 1, 8-, 9, 10-,11-mer peptide binders for 24 MHC class 1 alleles (see figure 10) using the http://www.cbs.dtu.dk/services/NetMHC/ database. The MHC class 1 molecules for which no binders were found are not listed. The peptides listed in figure 36 correspond to SEQ ID NO 216470-217262 in the sequence listing.

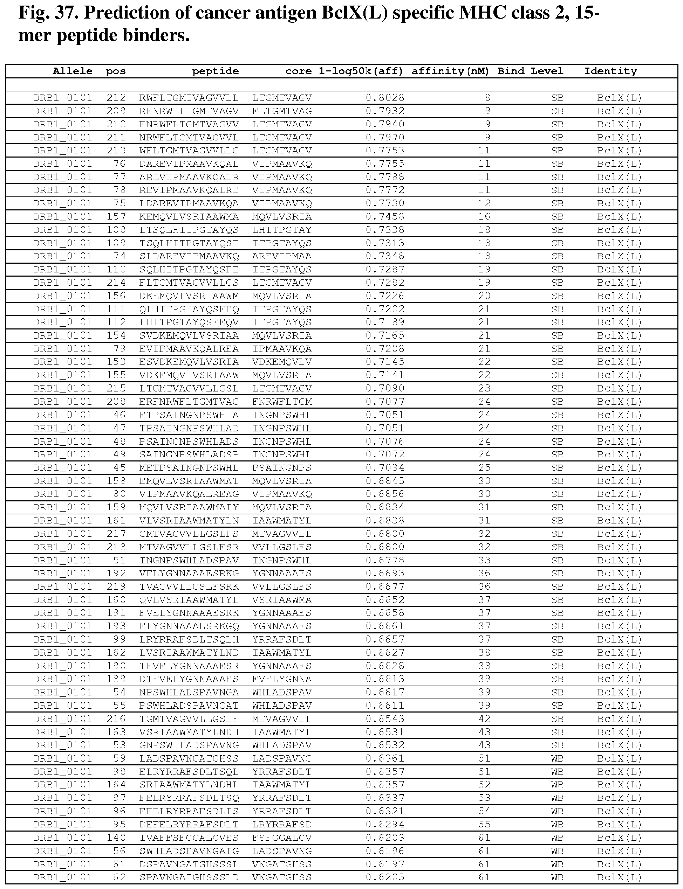

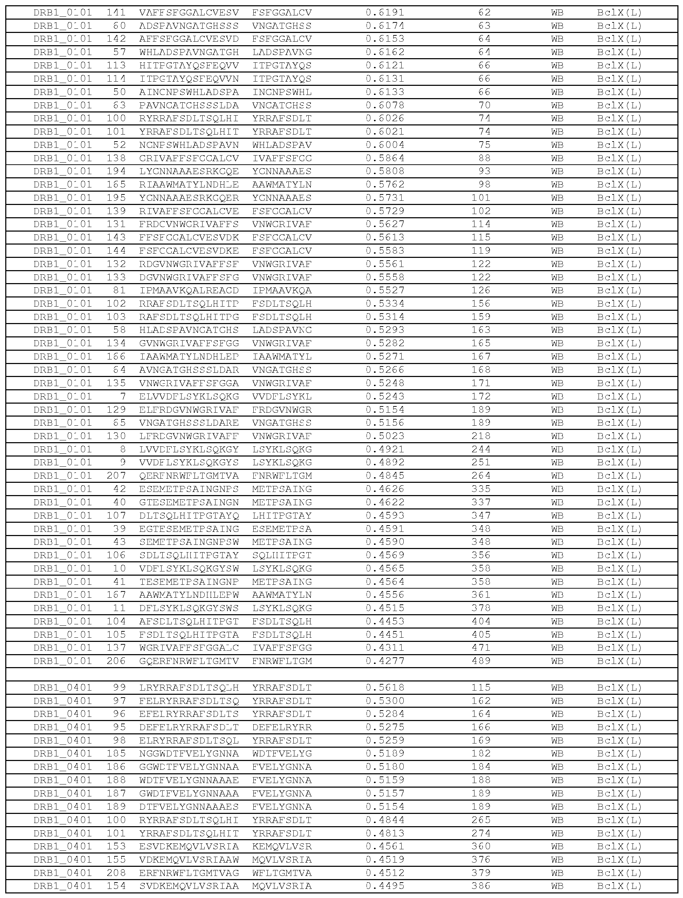

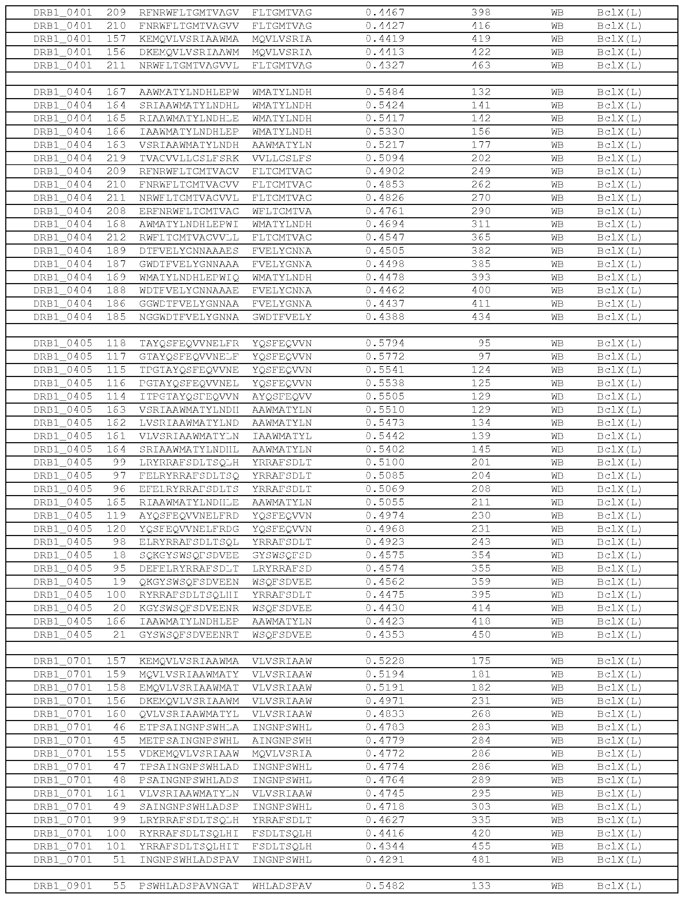

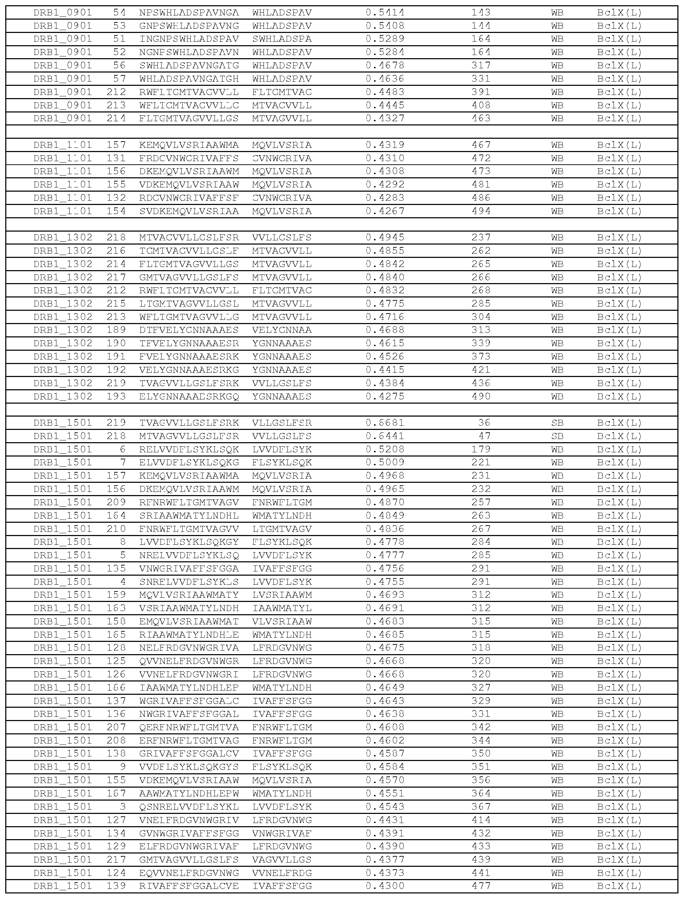

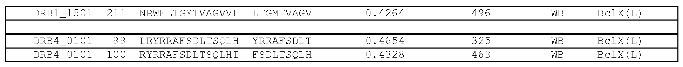

Figure 37. Prediction of cancer antigen BclX(L) specific MHC class 2, 15-mer peptide binders. Prediction of cancer antigen BclX(L) specific MHC class 2, 15-mer peptide binders for 14 MHC class 2 alleles (see figure 10) using the http://www.cbs.dtu.dk/services/NetMHCII / database. The MHC class 2 molecules for which no binders were found are not listed. The peptides listed in Fig 37 correspond to SEQ ID NO 217263-217774 in the sequence listing.

Figure 38. The amino acid sequences of Borrelia antigens of specific interest as sources for MHC binding peptides. Name and protein bank accession numbers are shown.

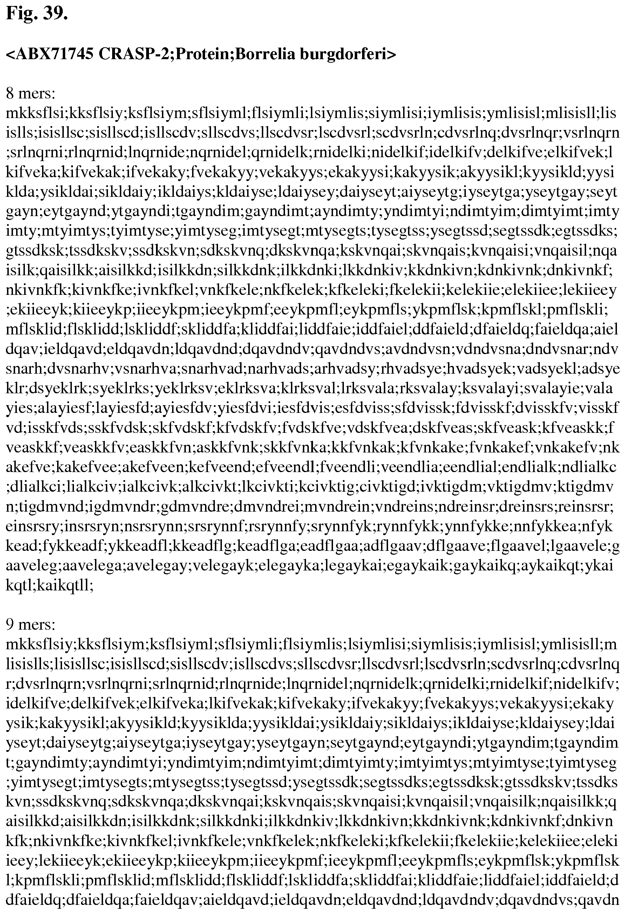

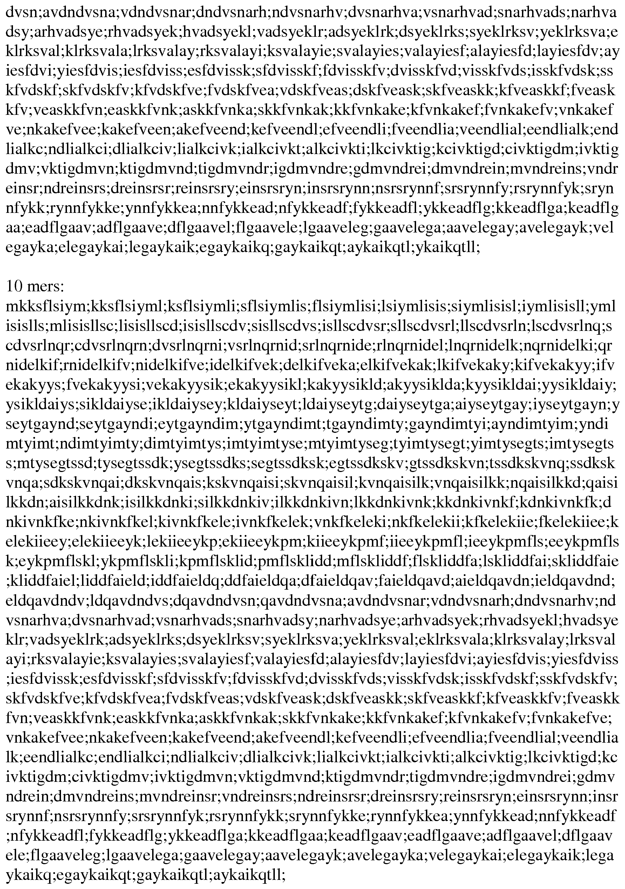

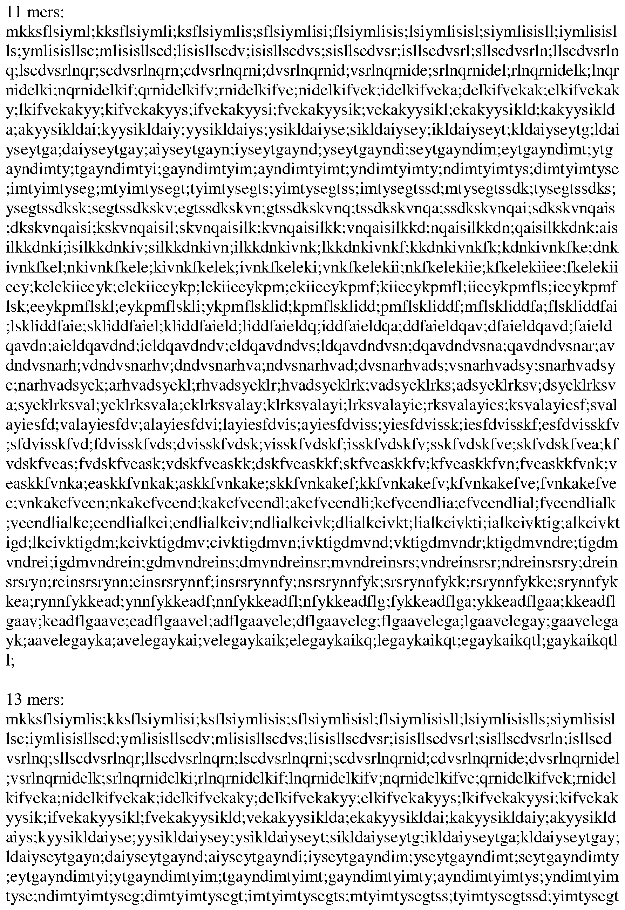

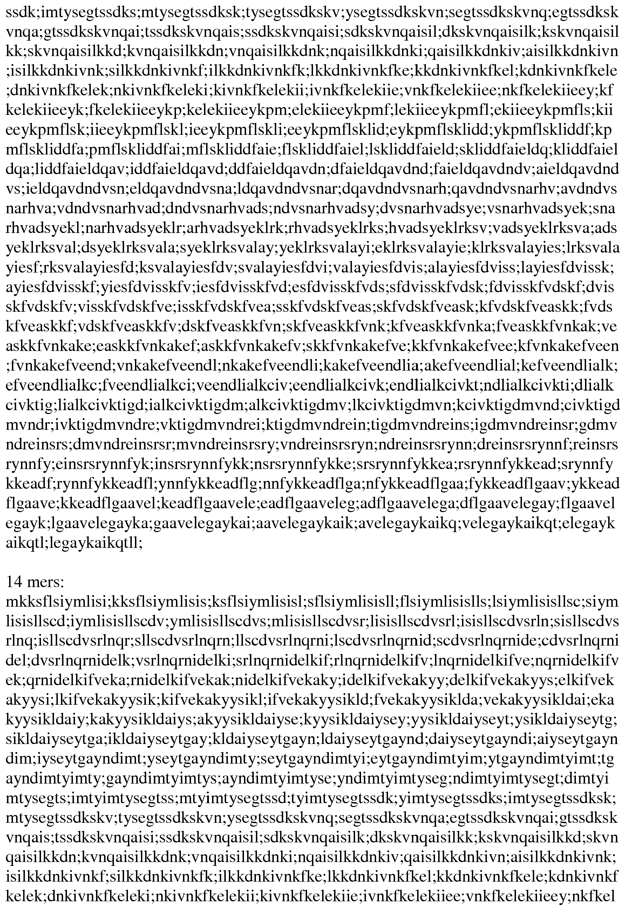

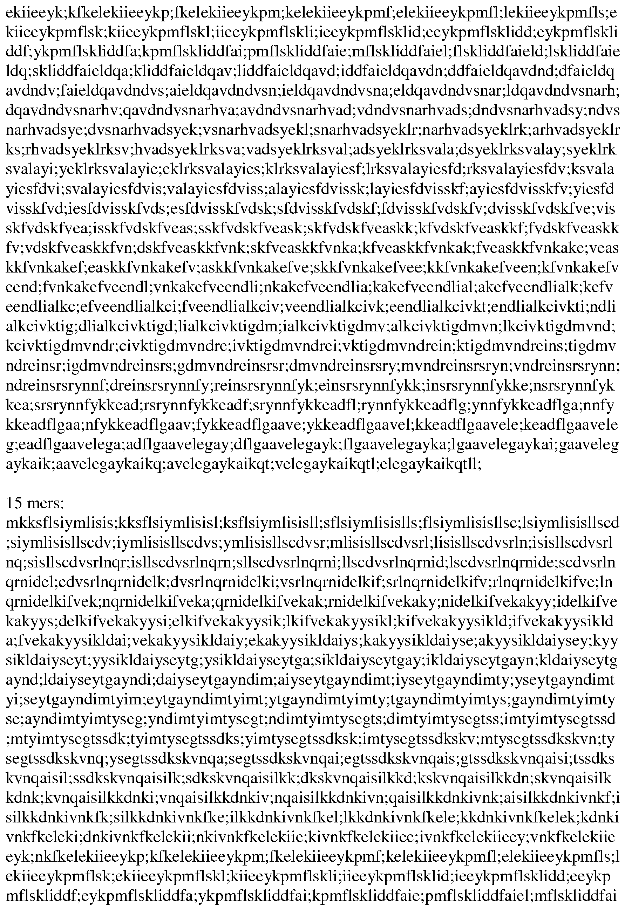

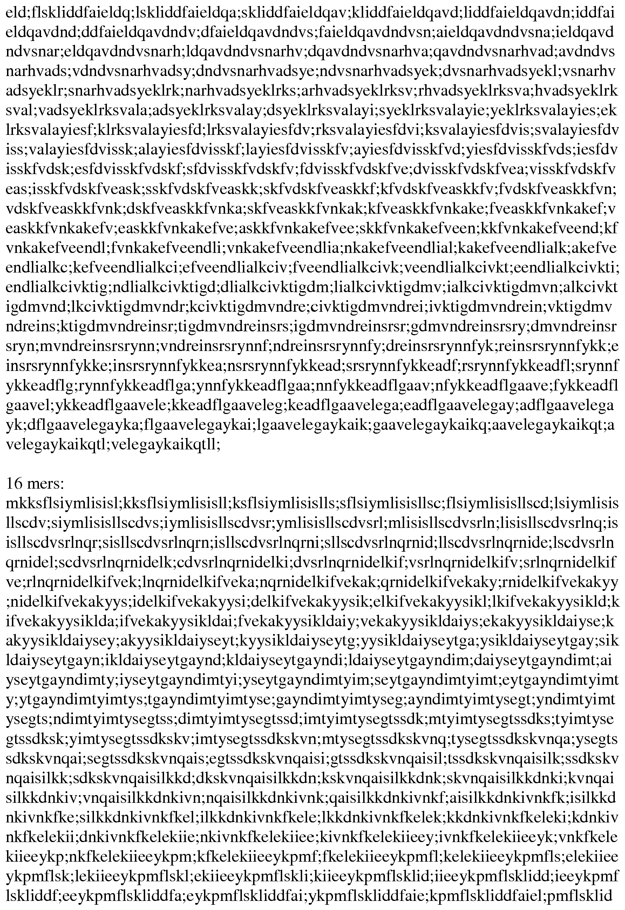

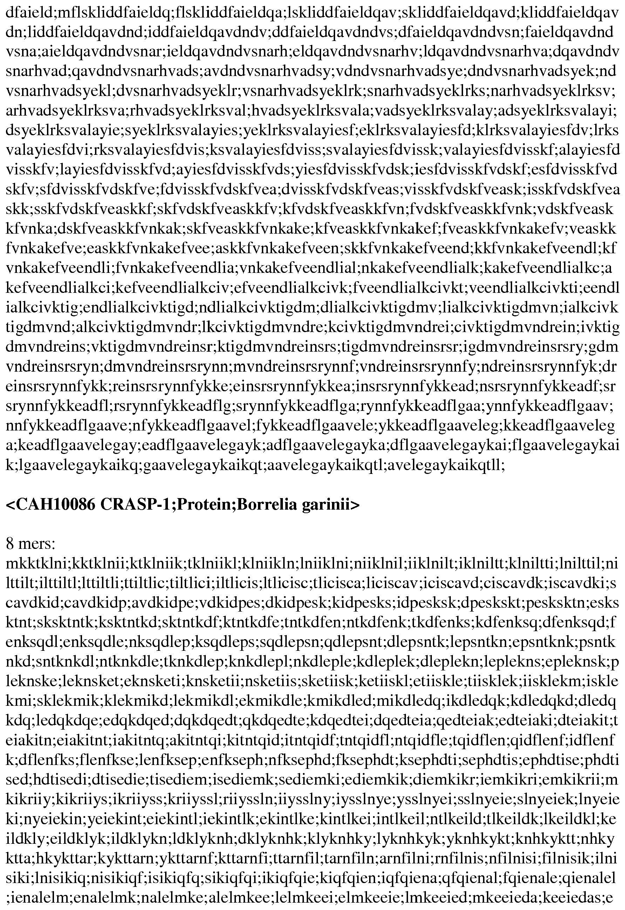

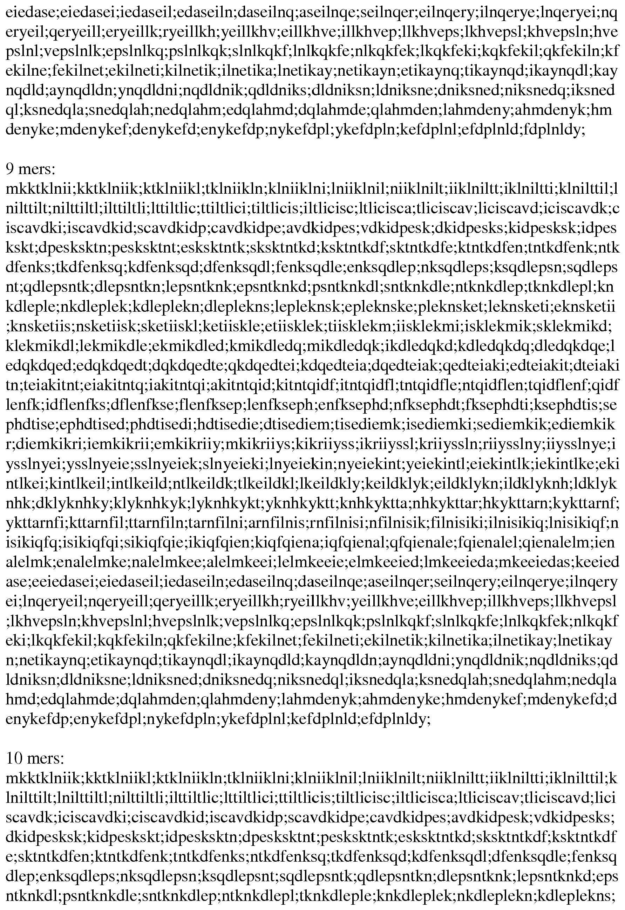

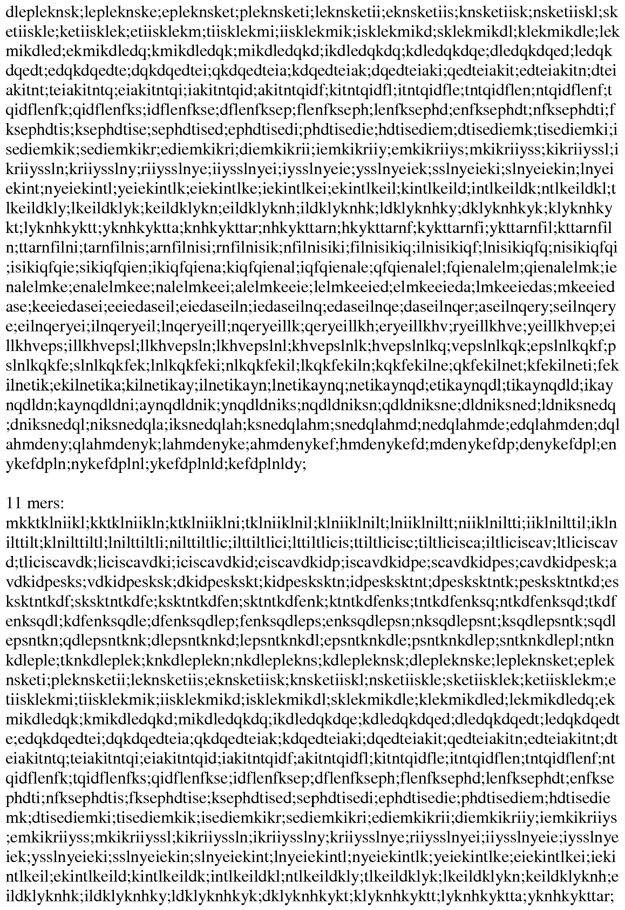

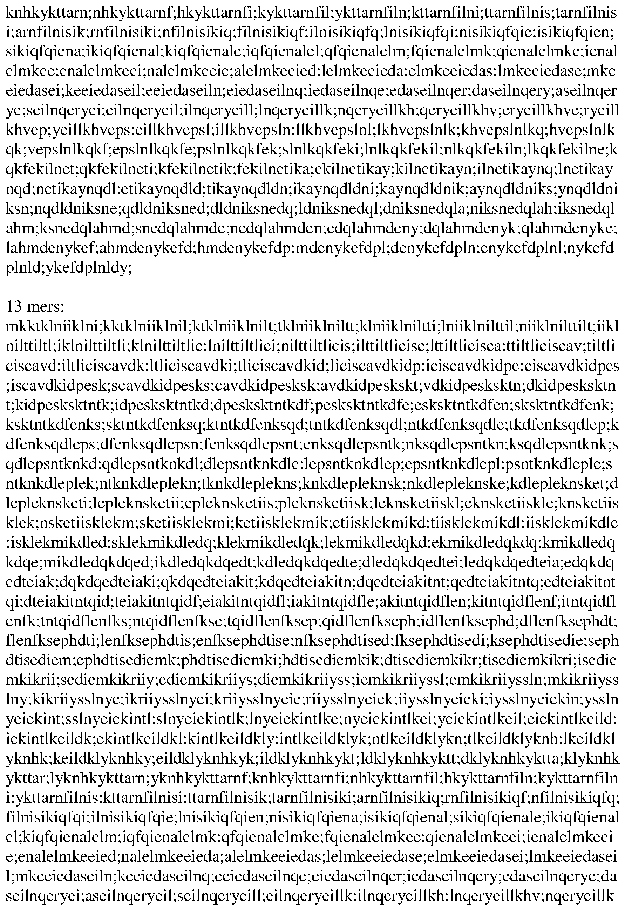

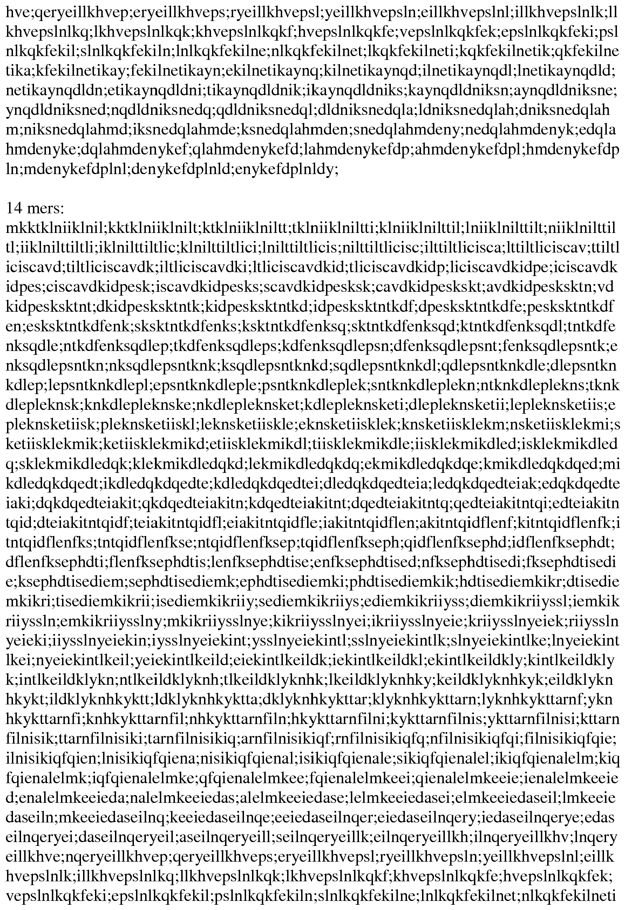

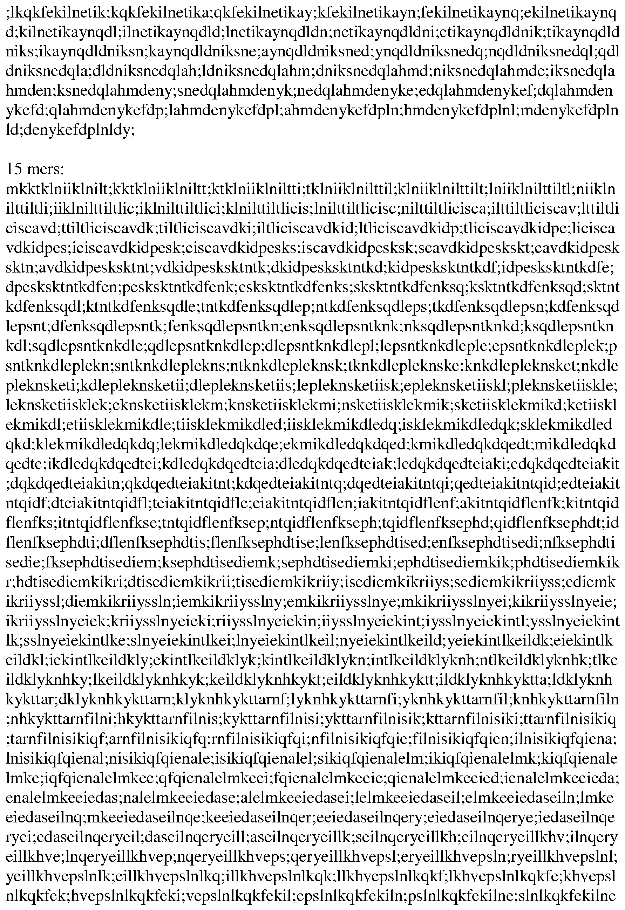

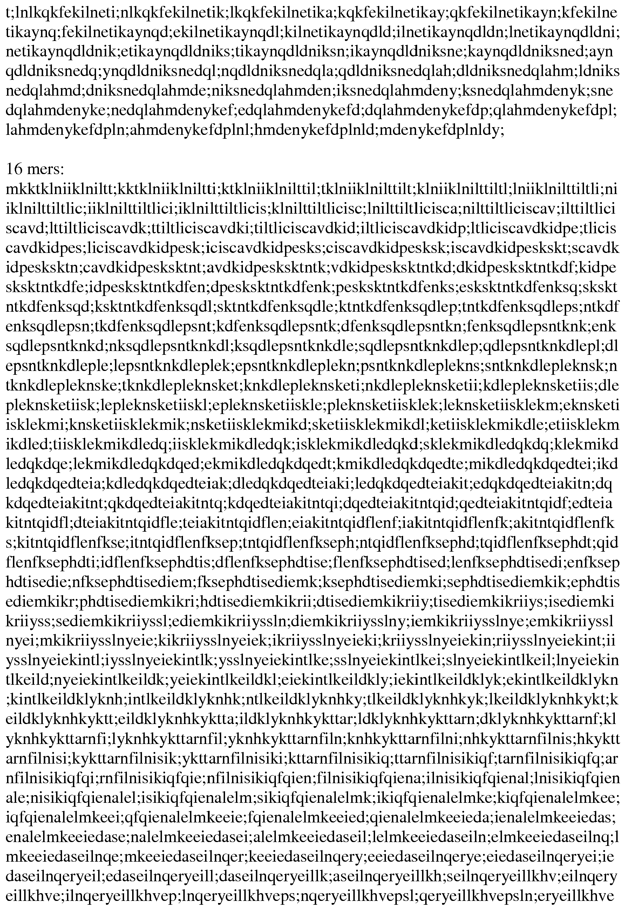

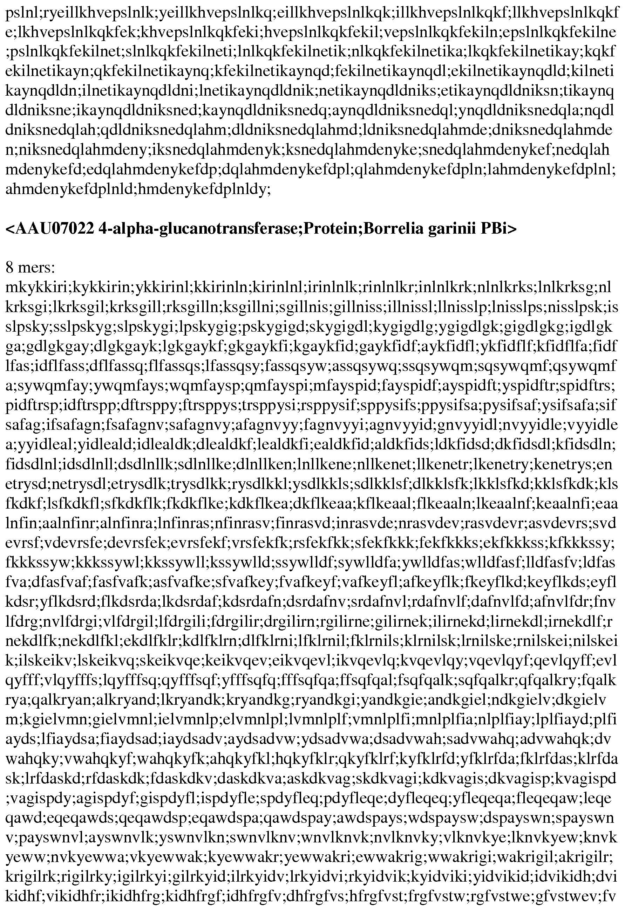

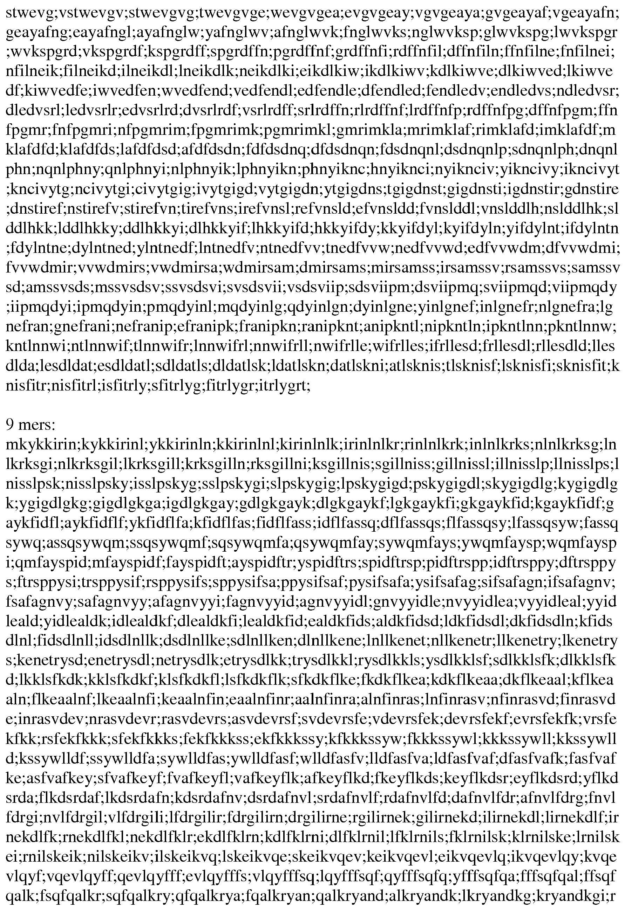

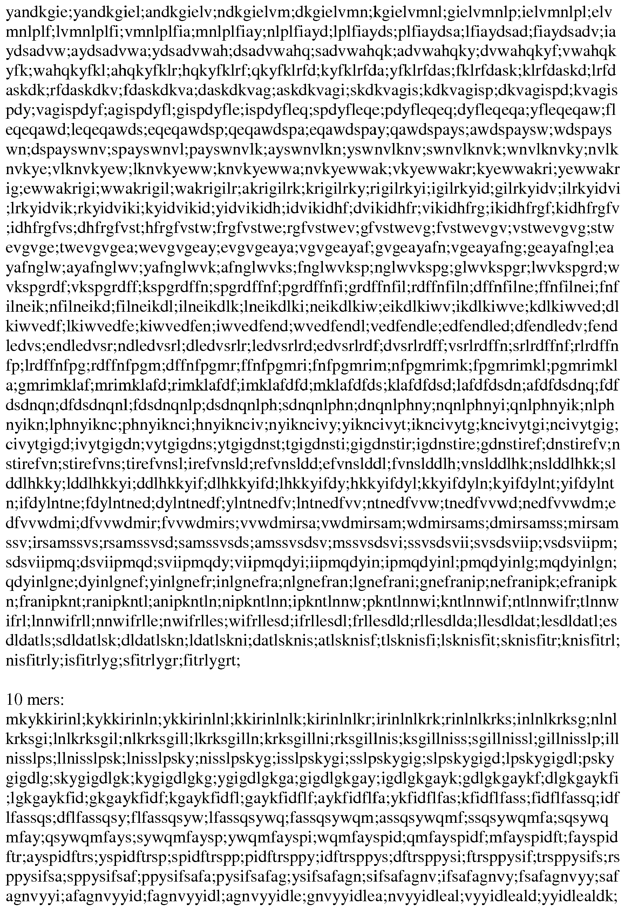

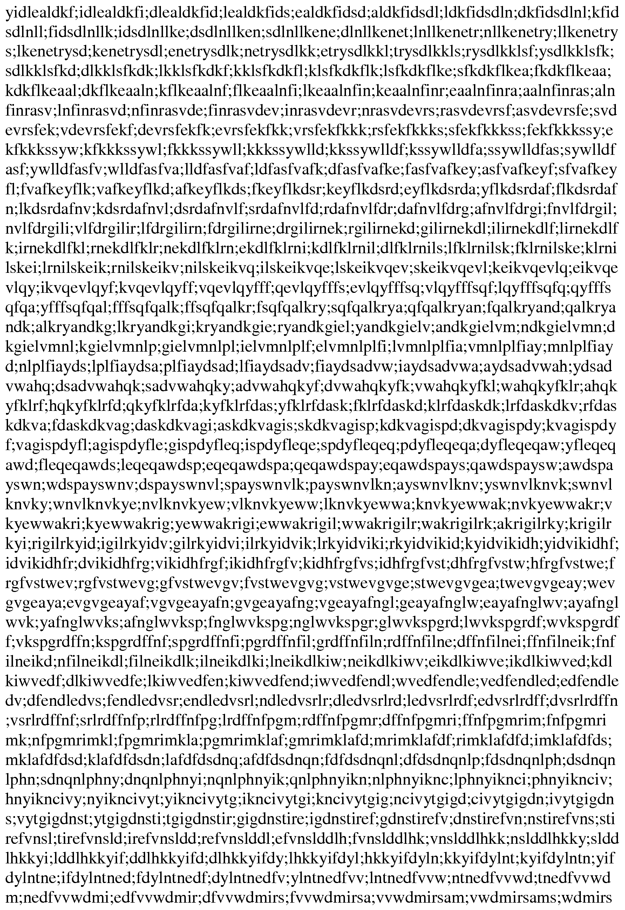

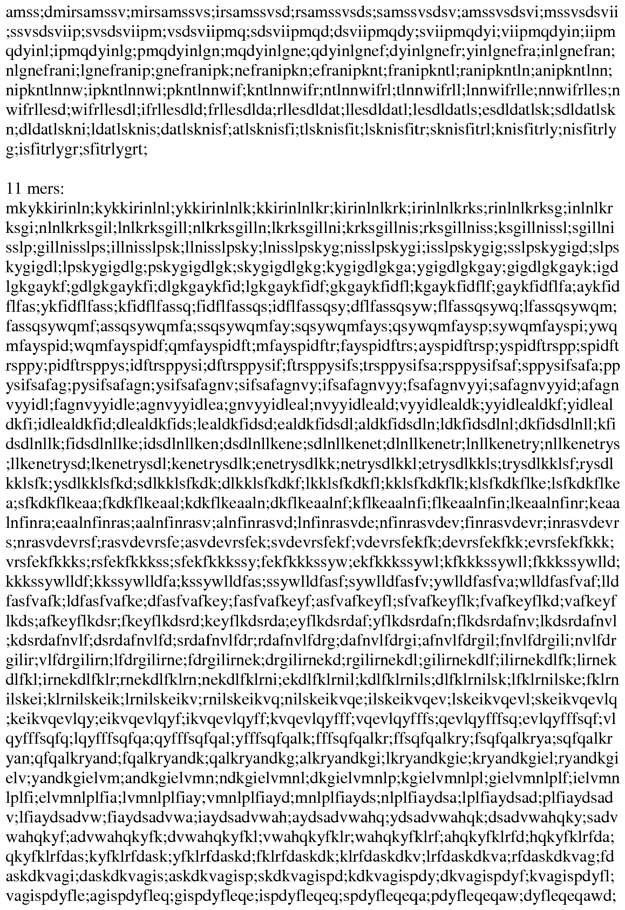

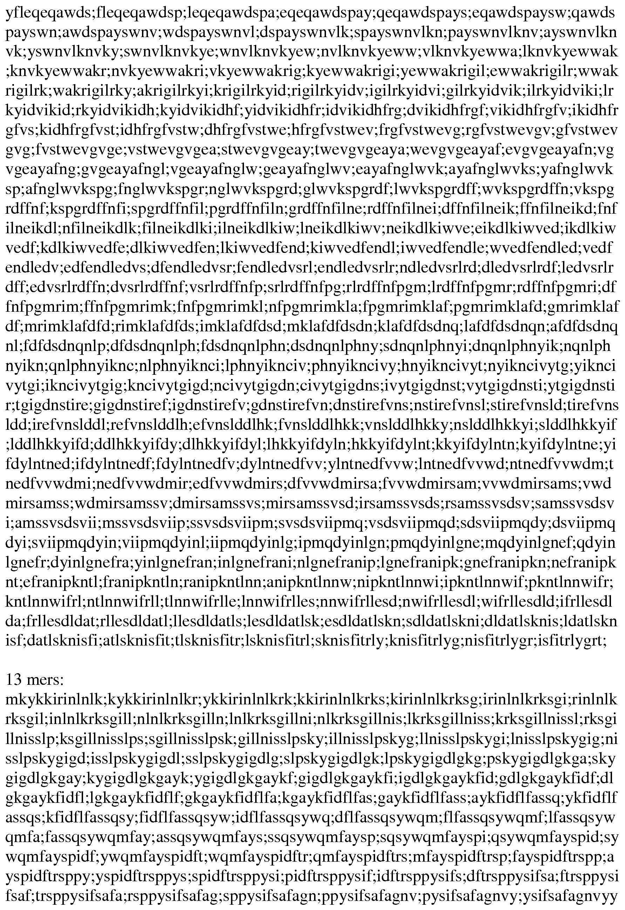

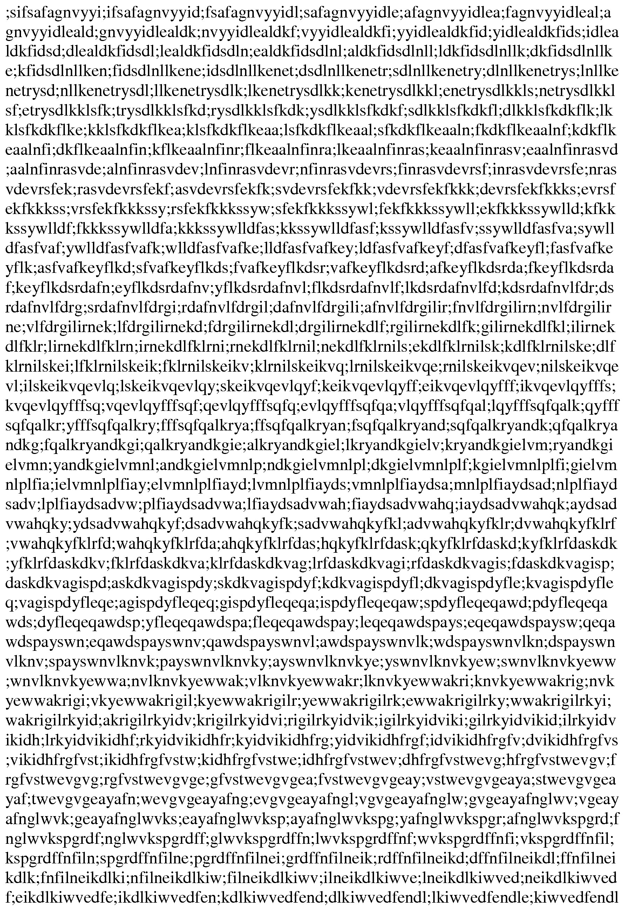

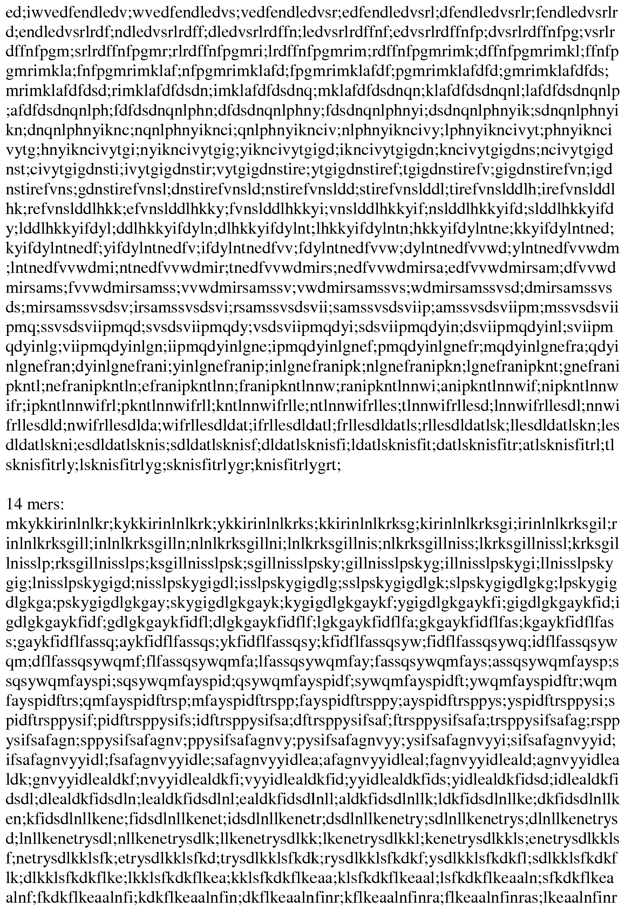

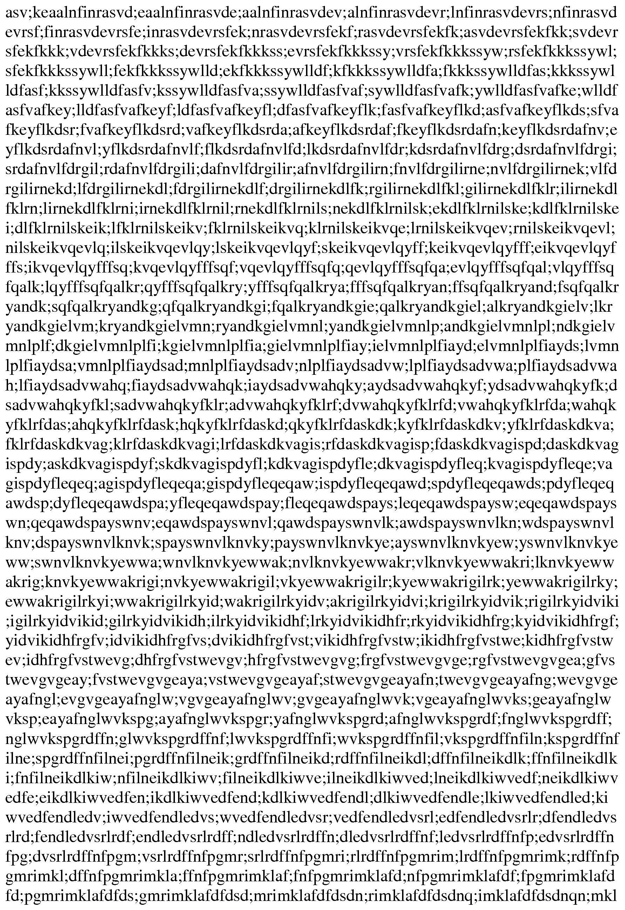

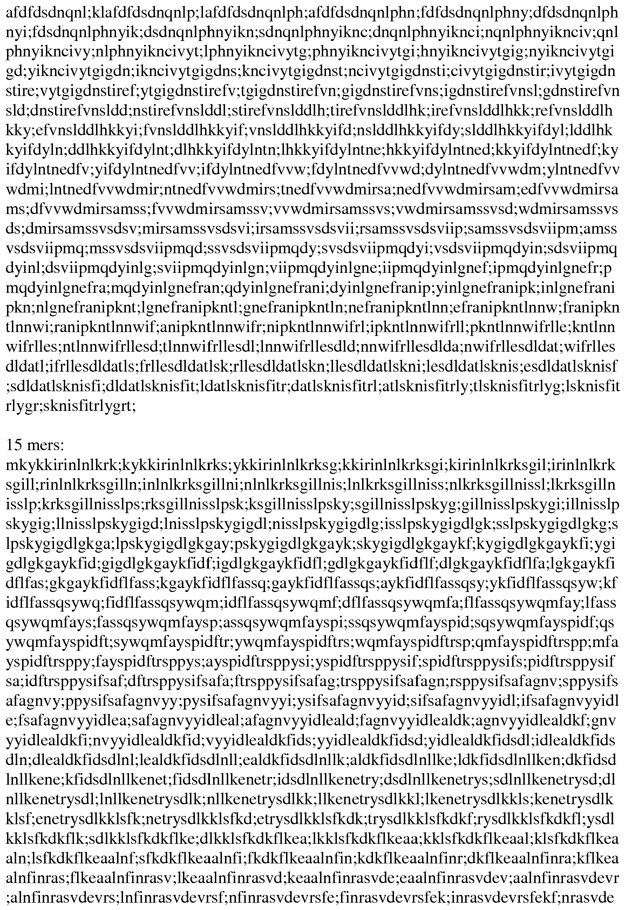

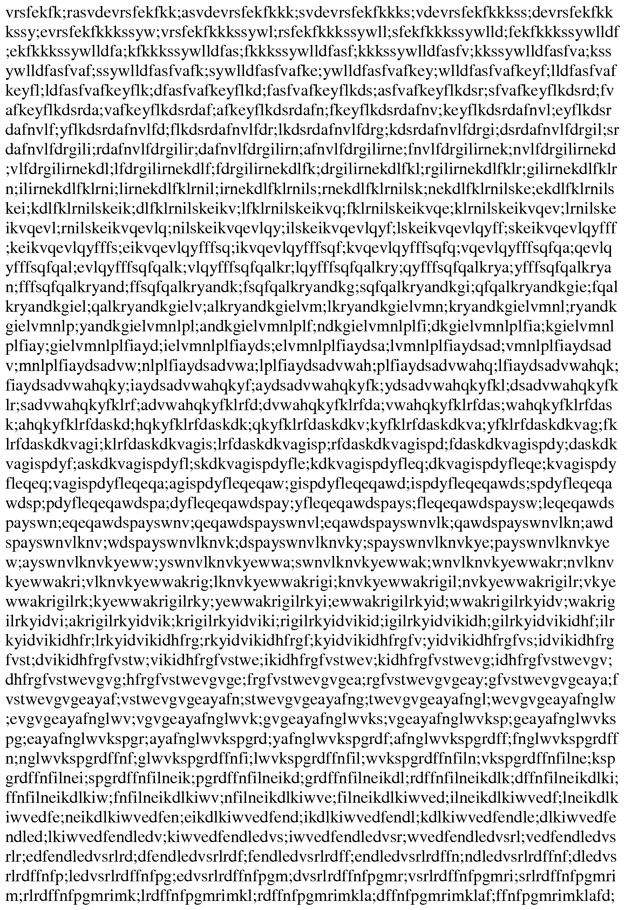

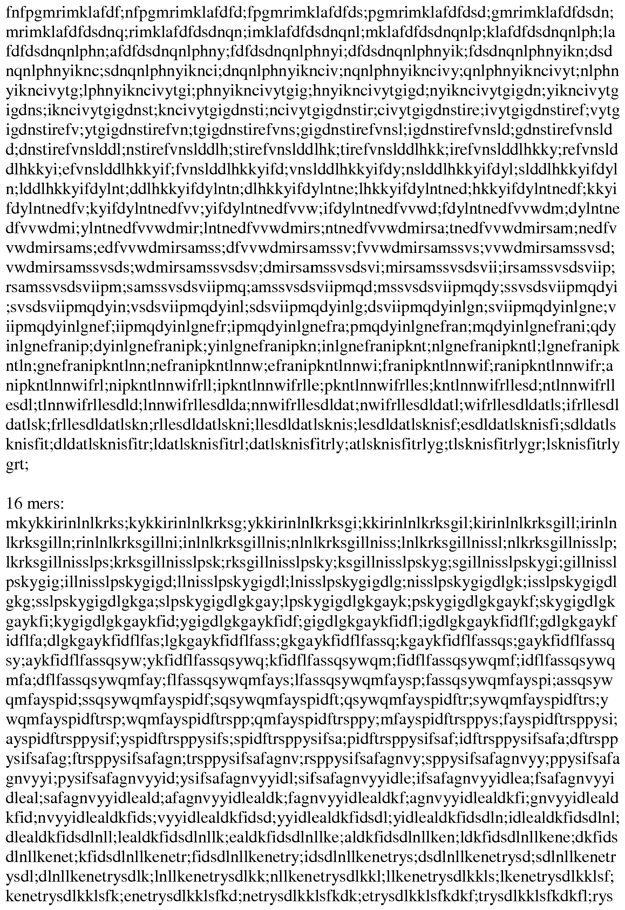

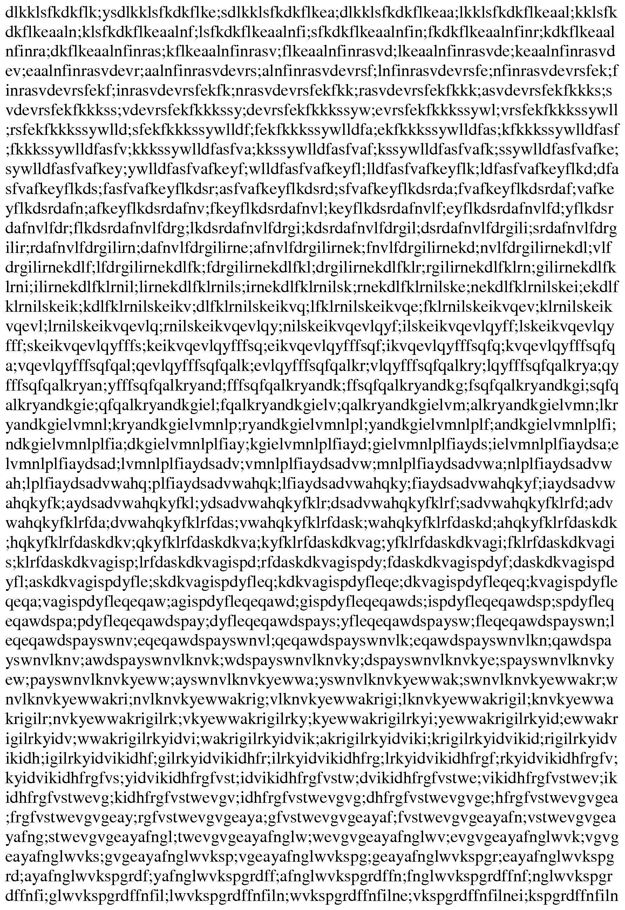

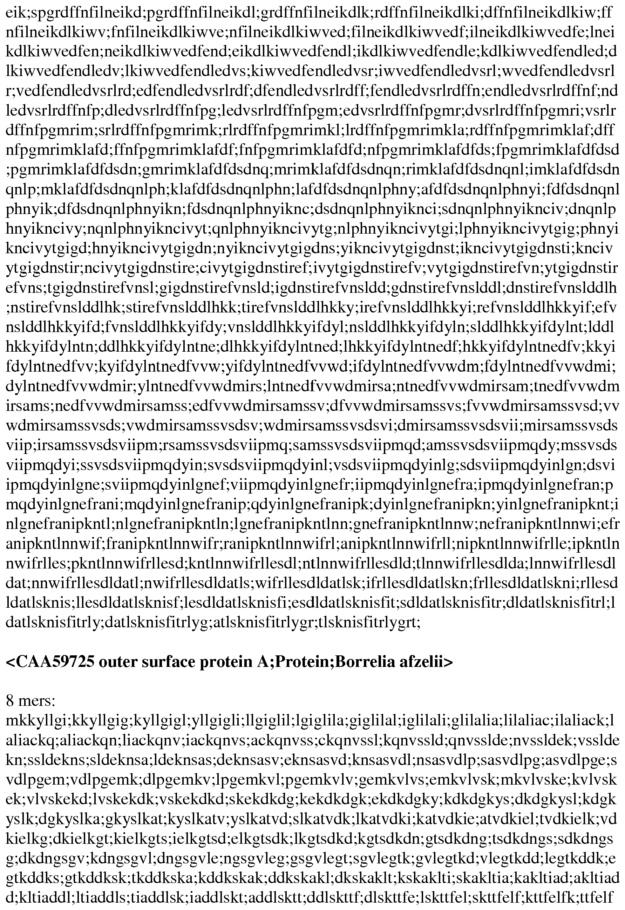

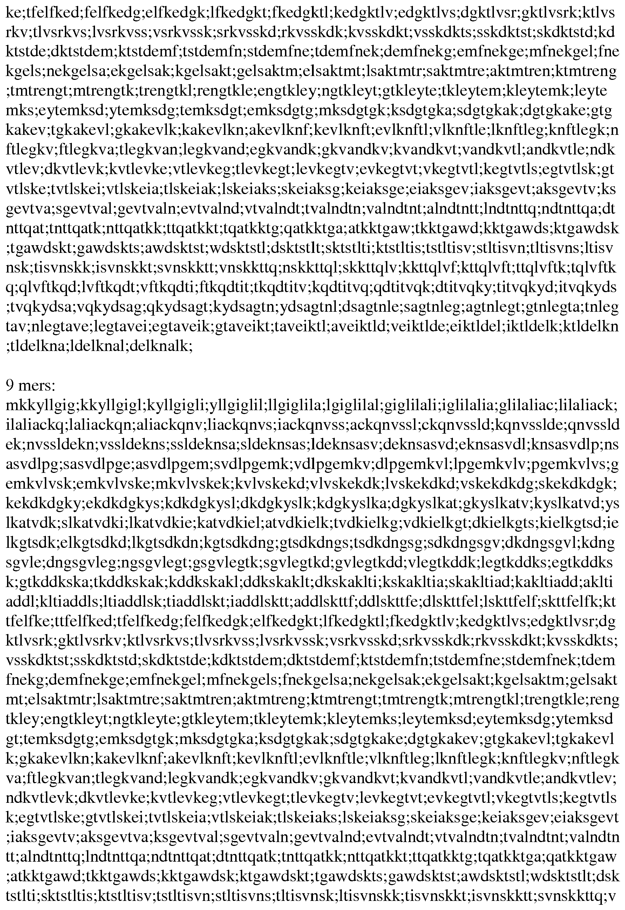

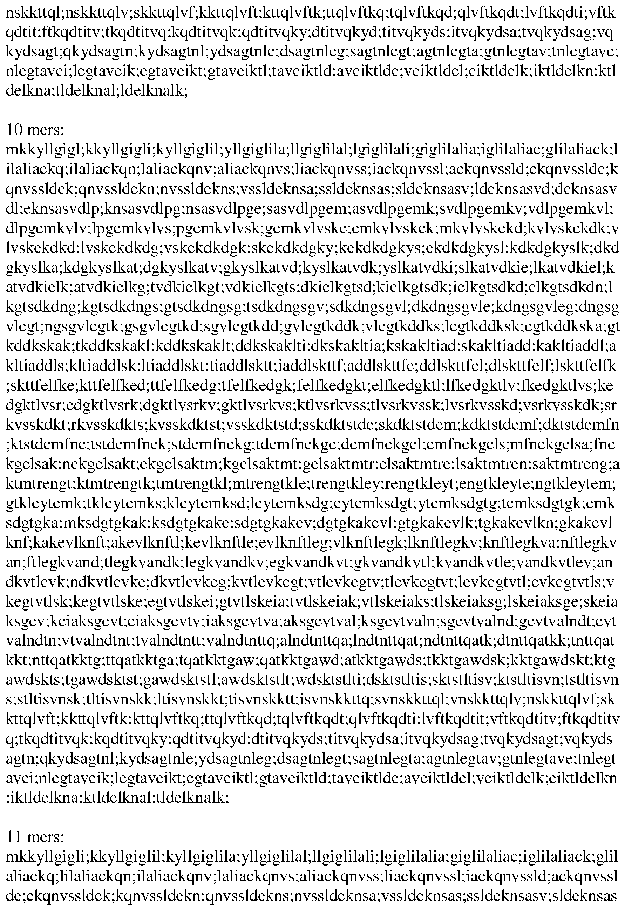

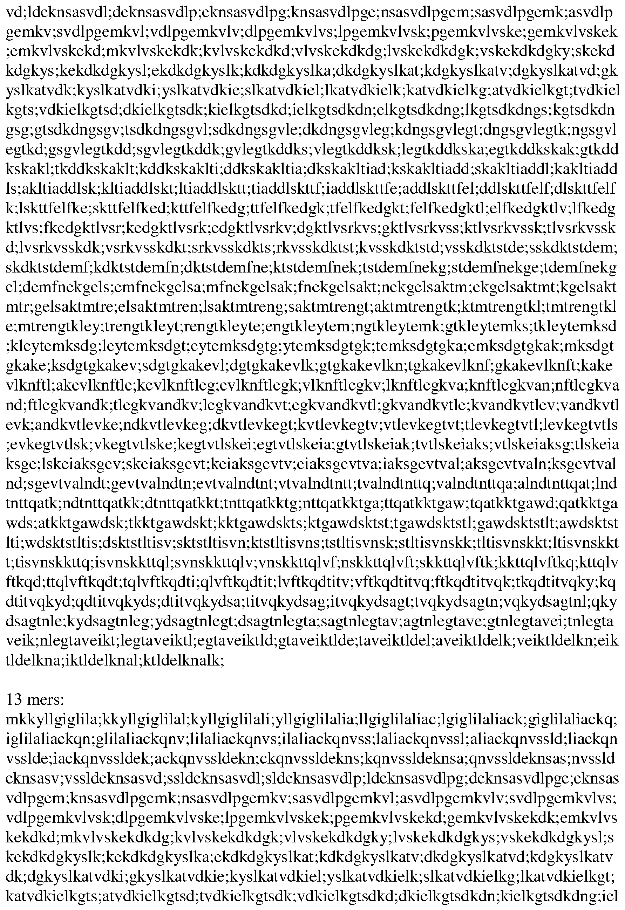

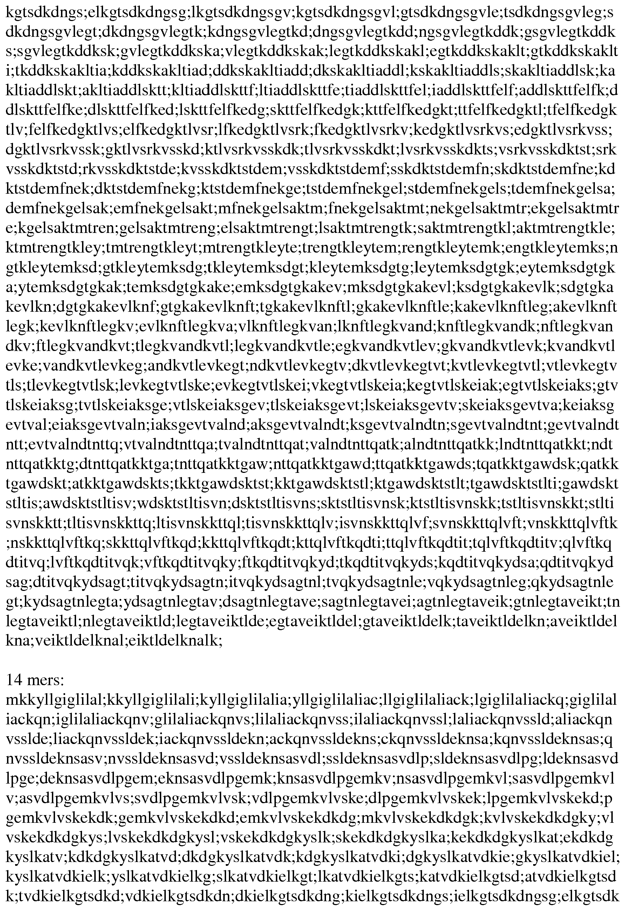

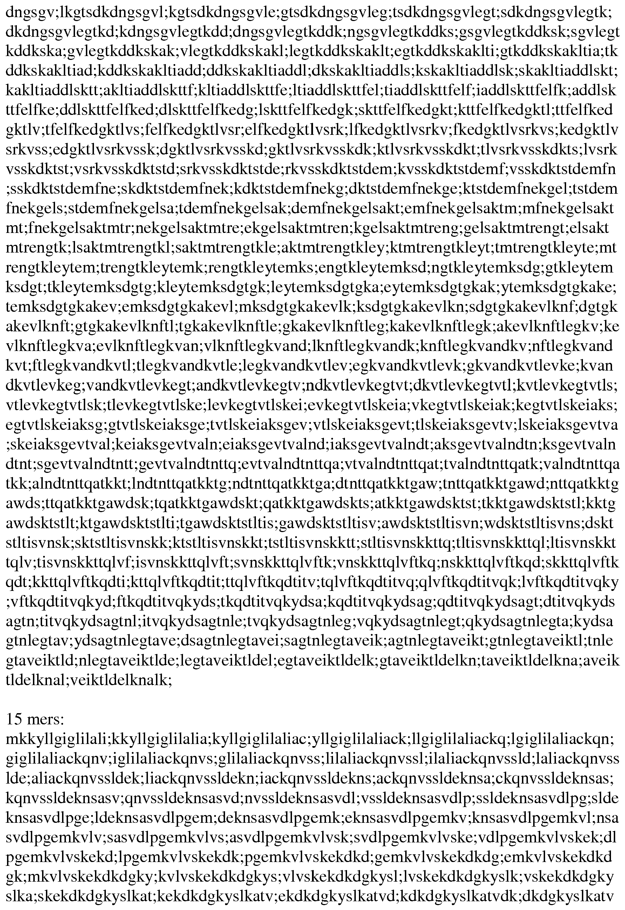

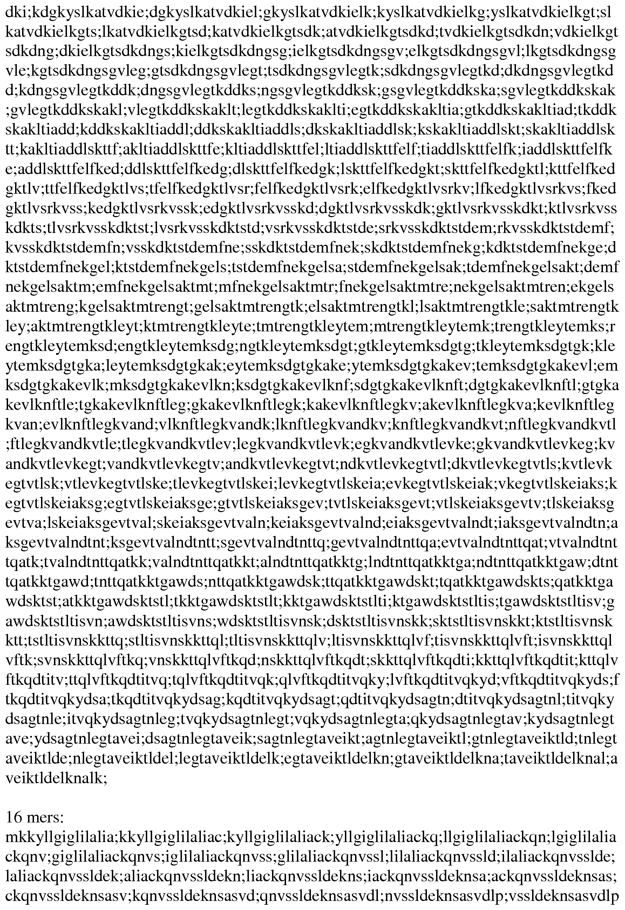

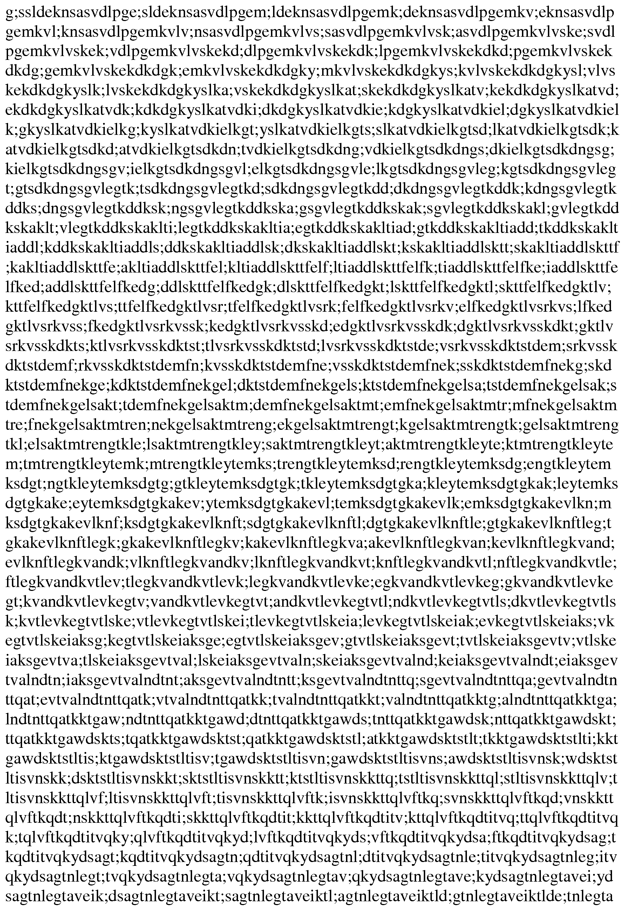

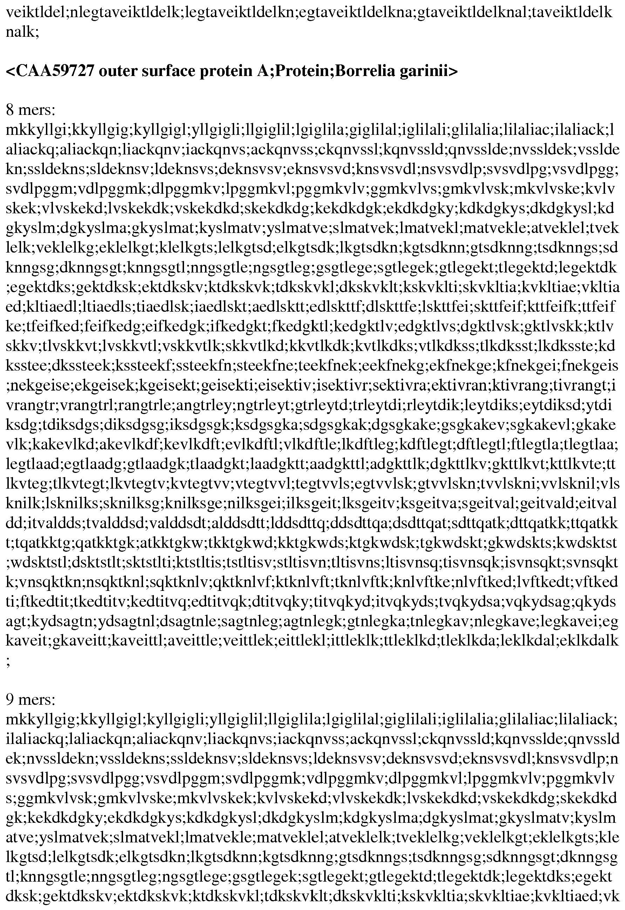

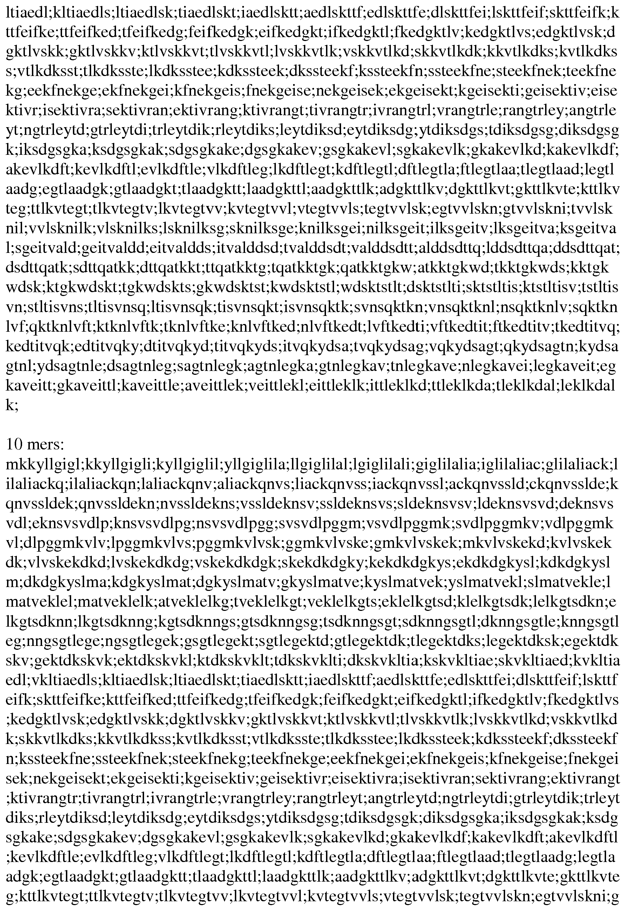

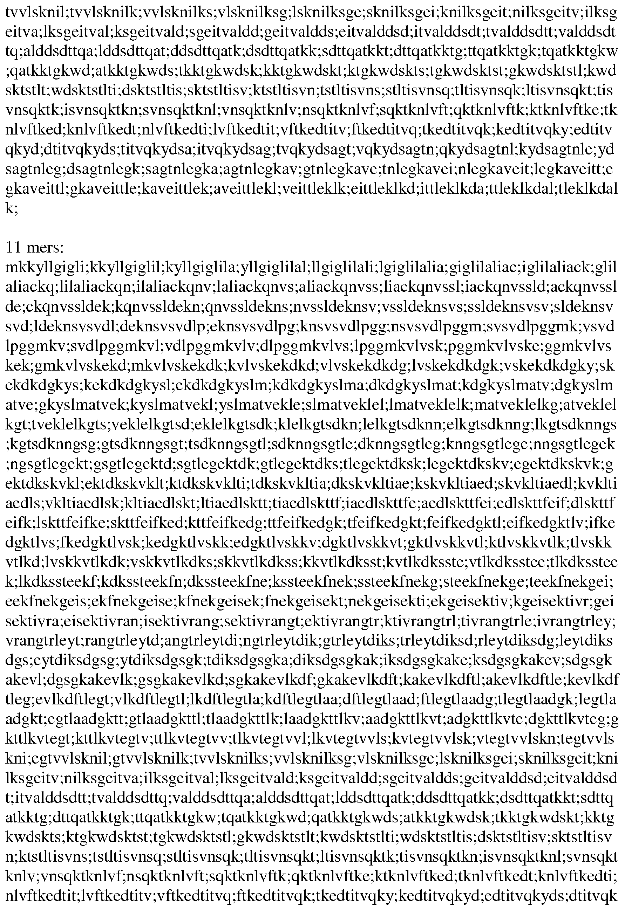

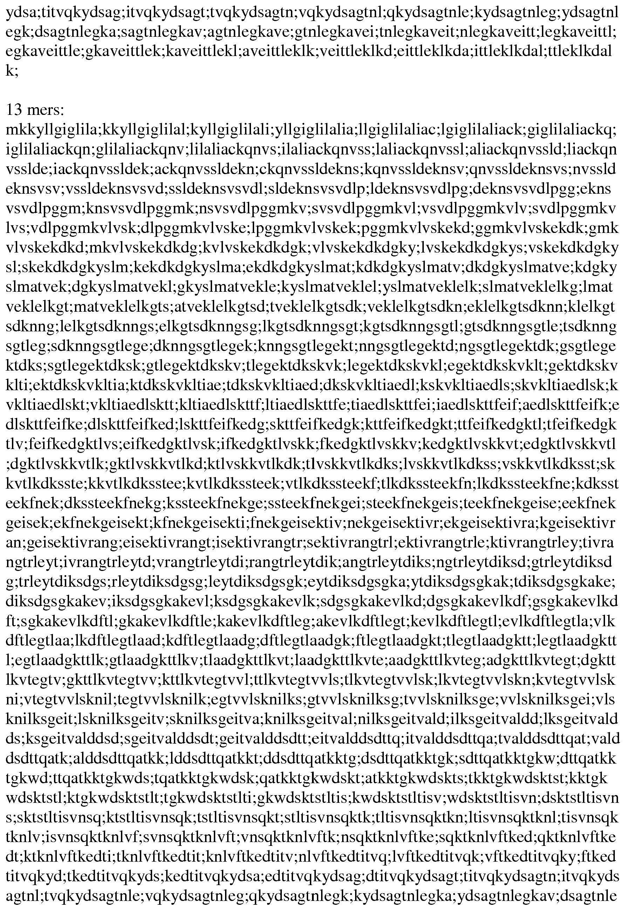

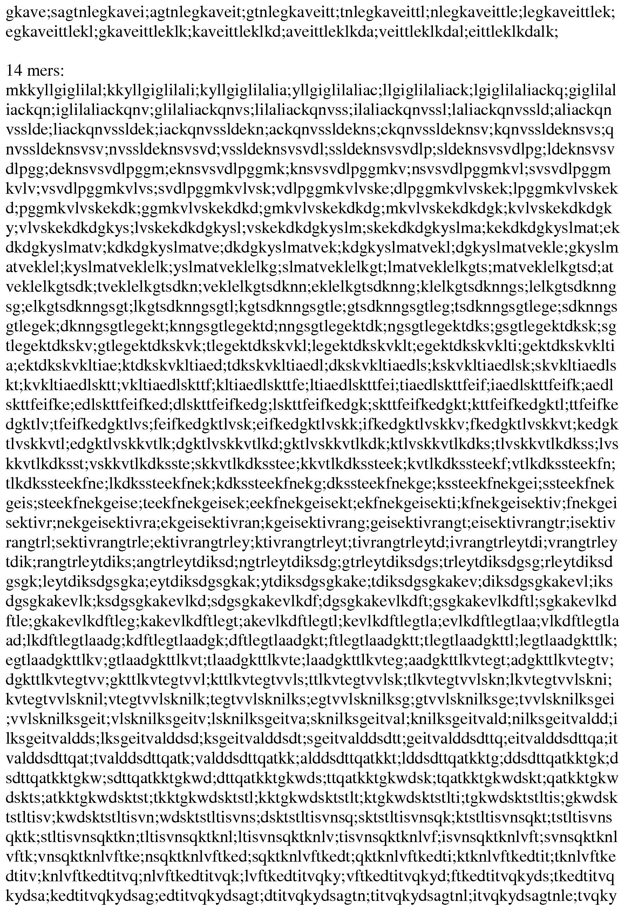

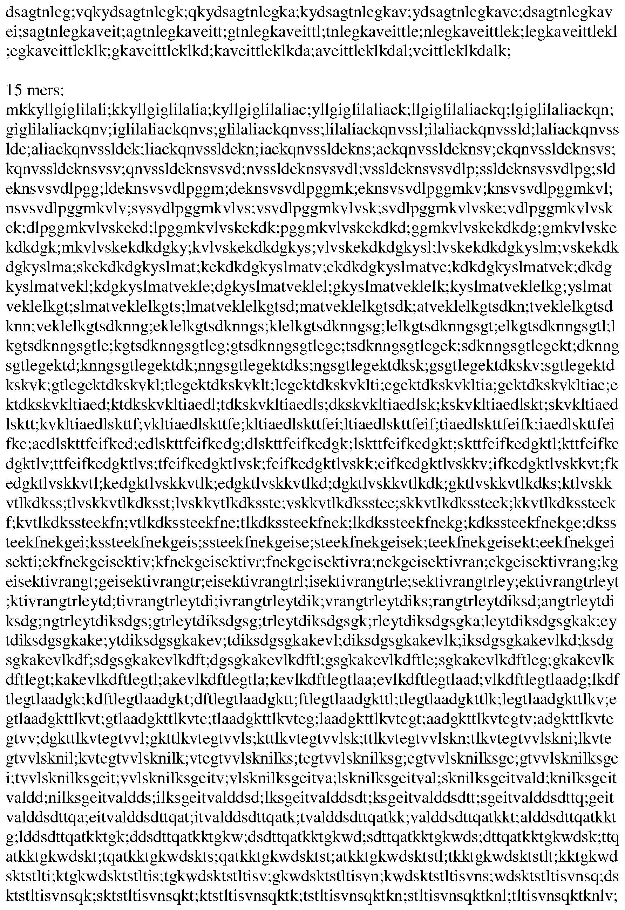

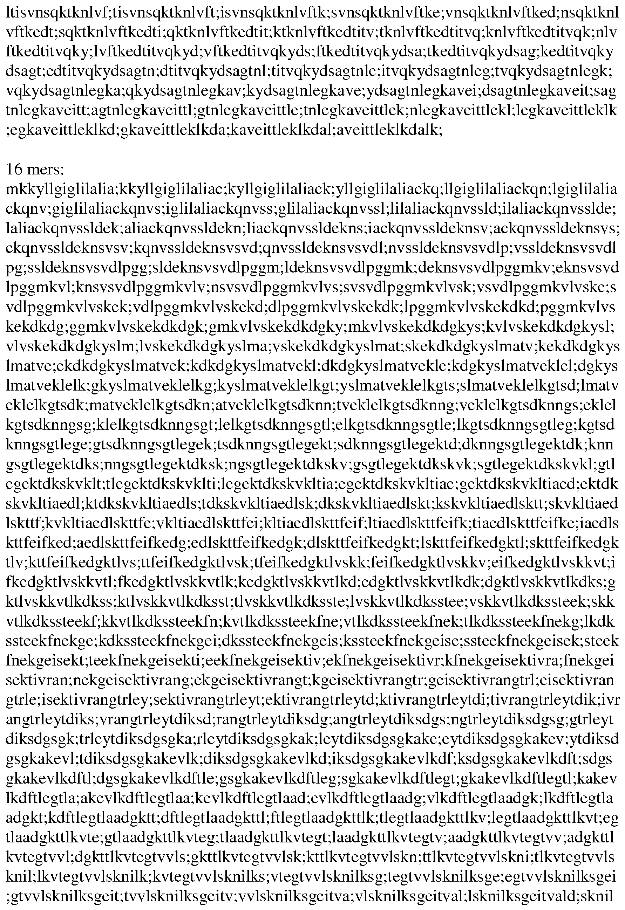

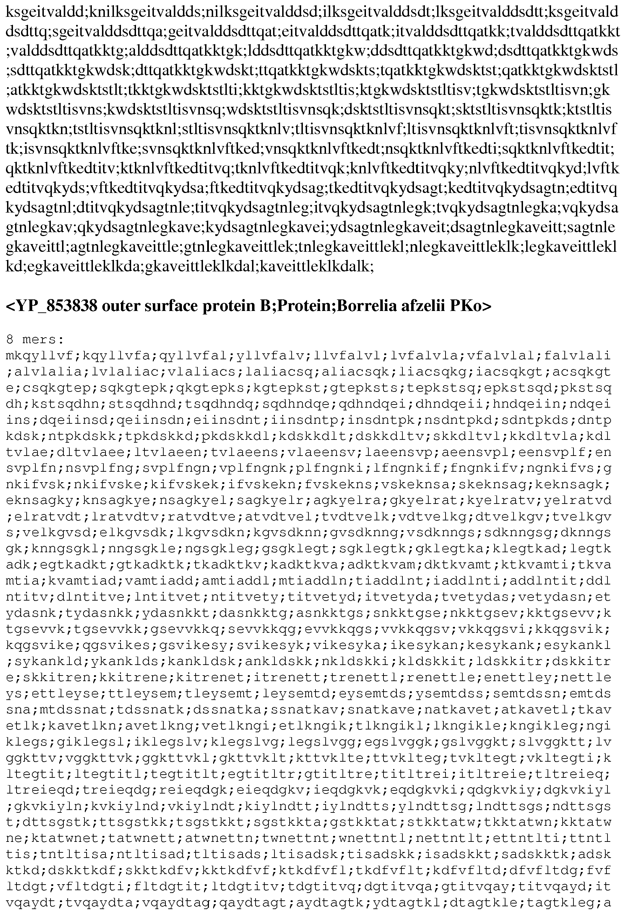

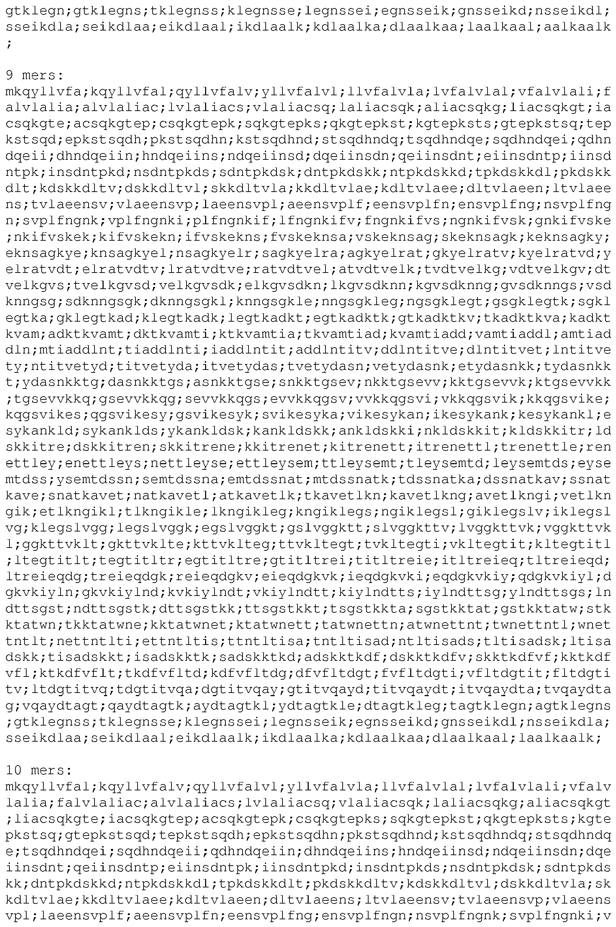

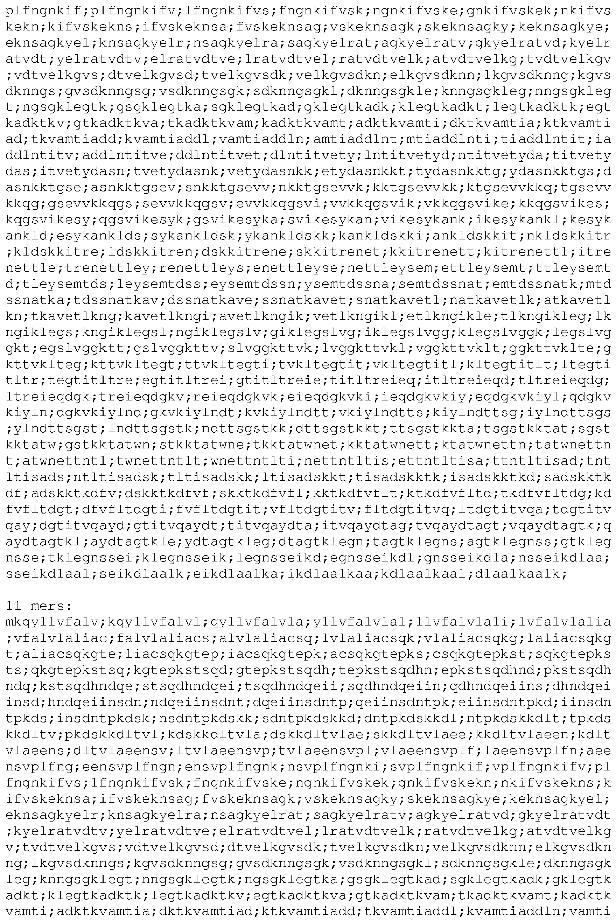

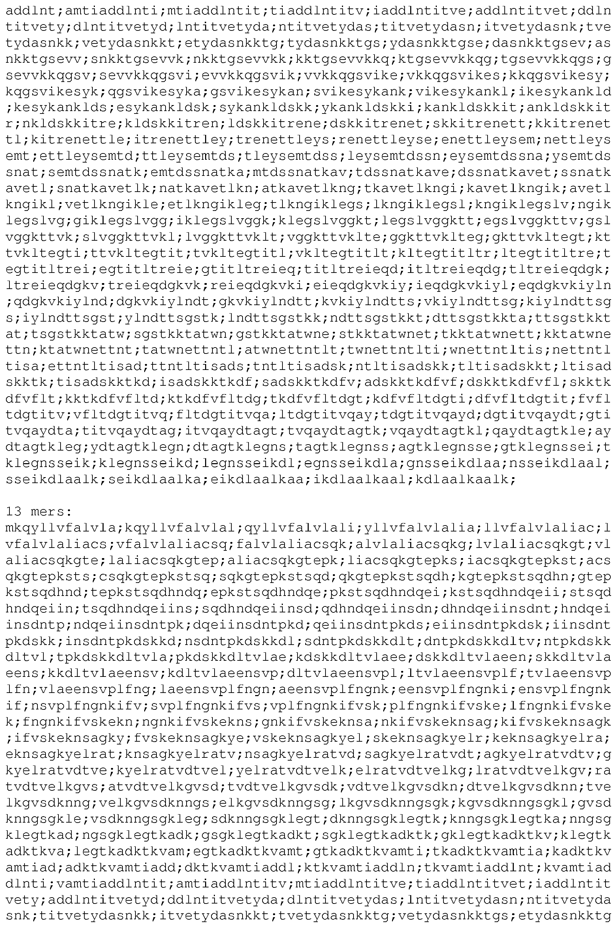

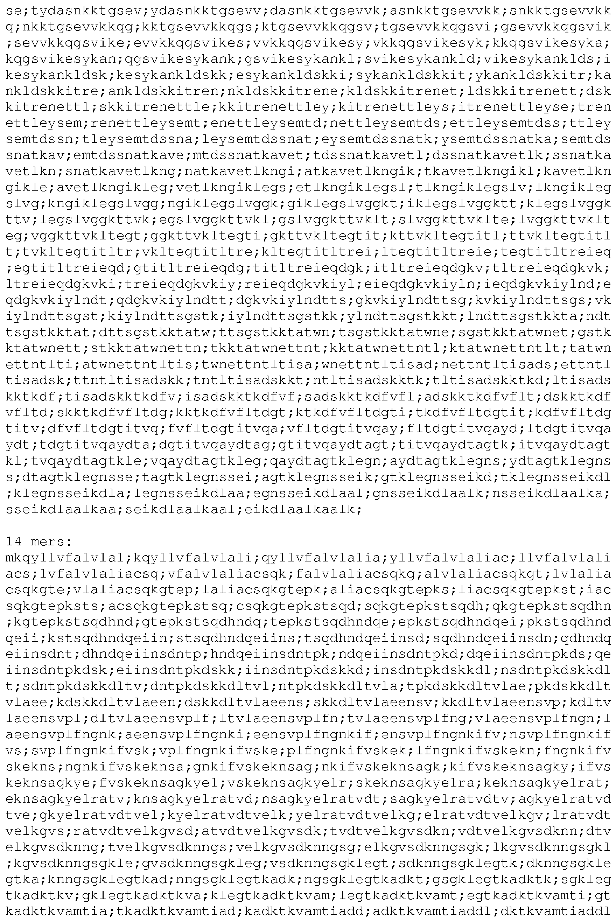

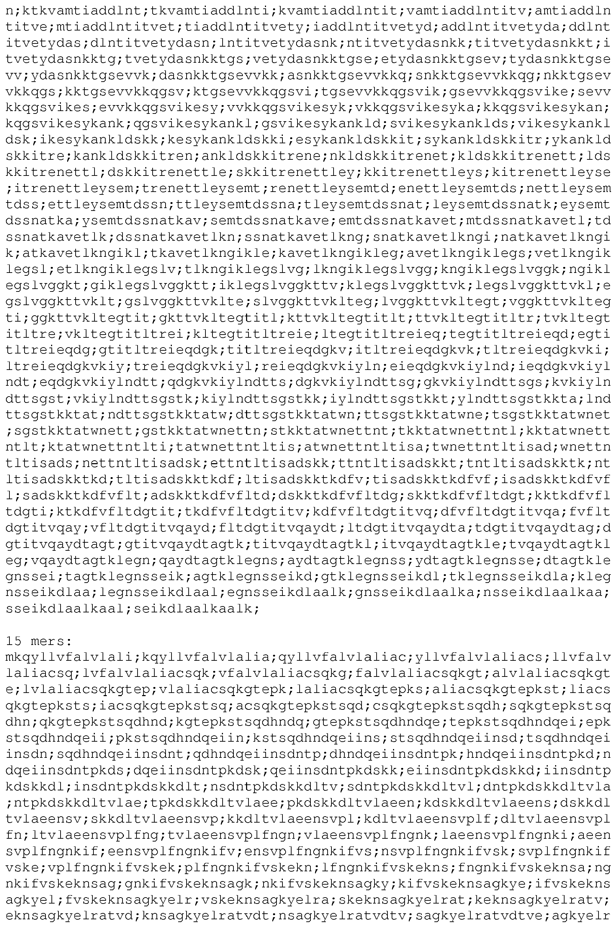

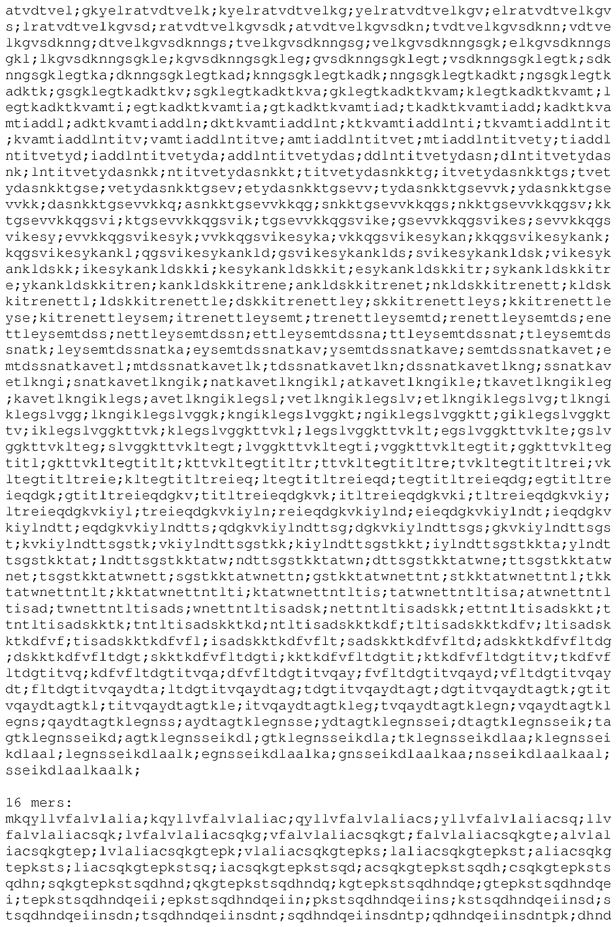

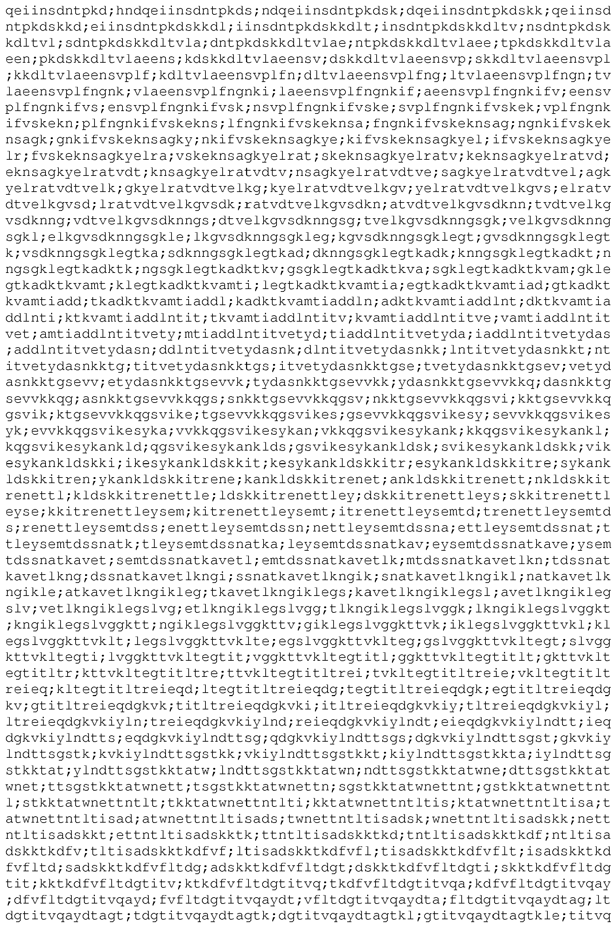

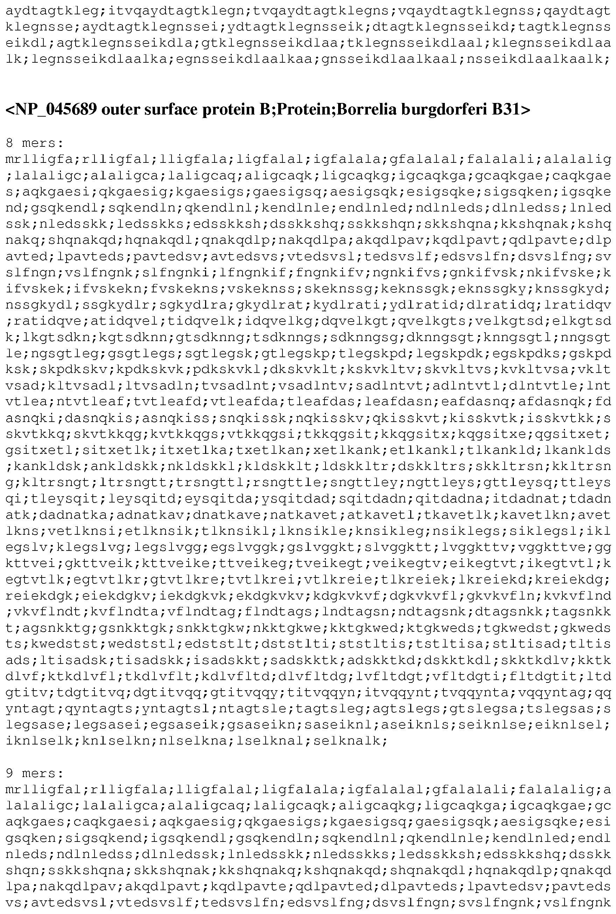

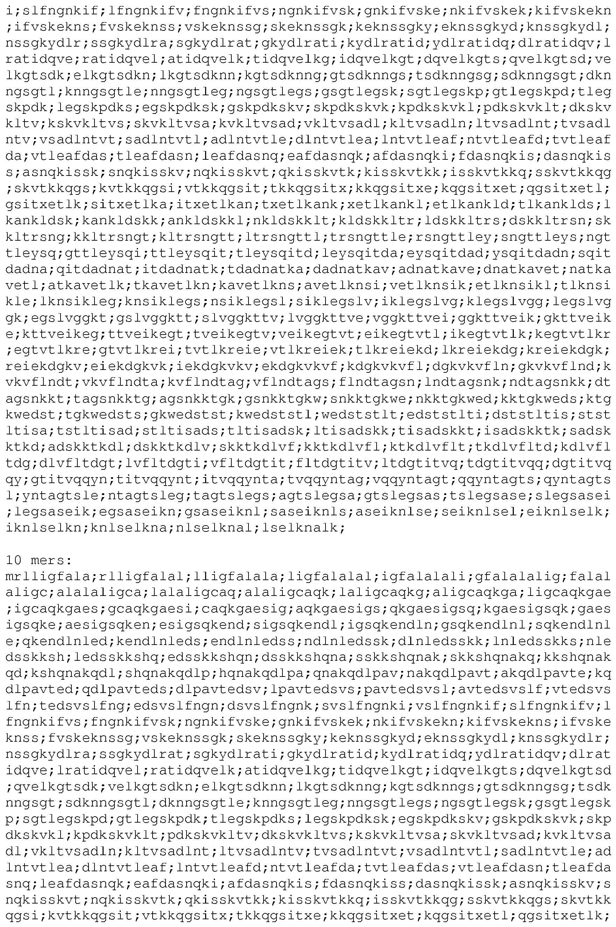

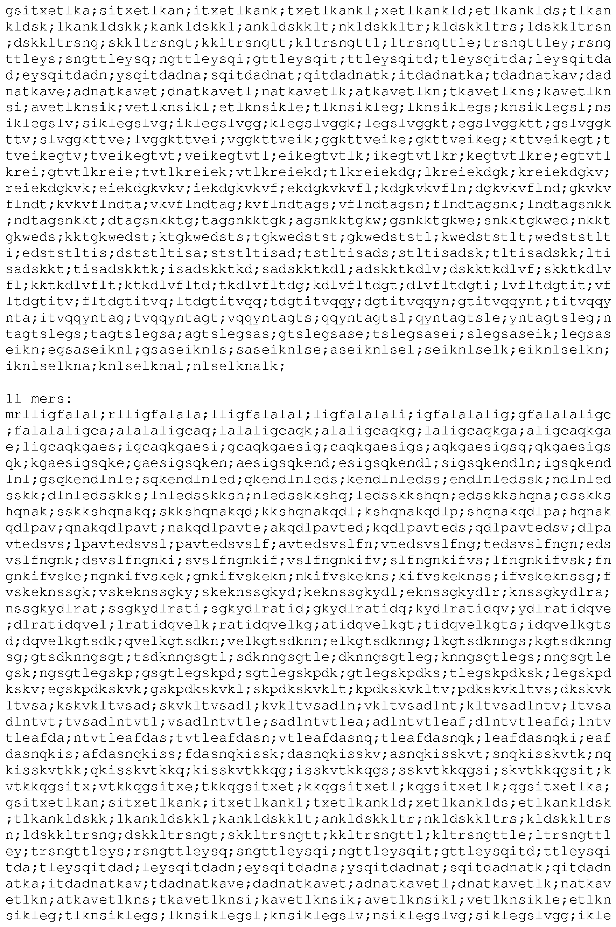

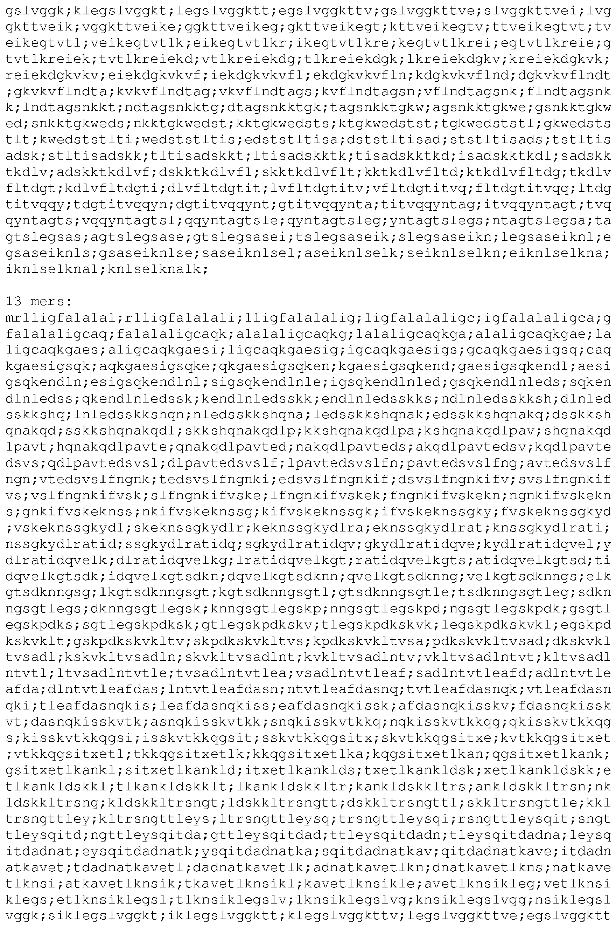

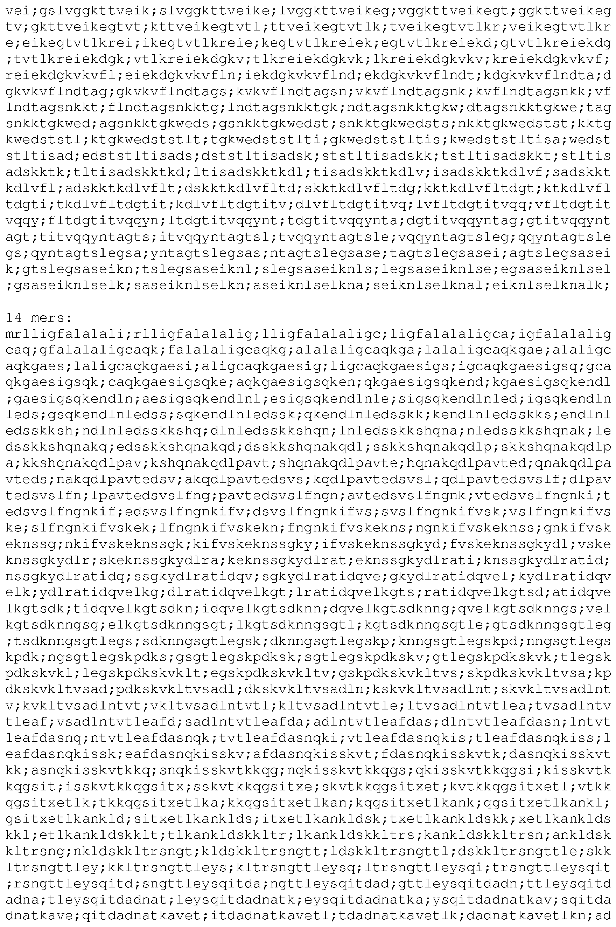

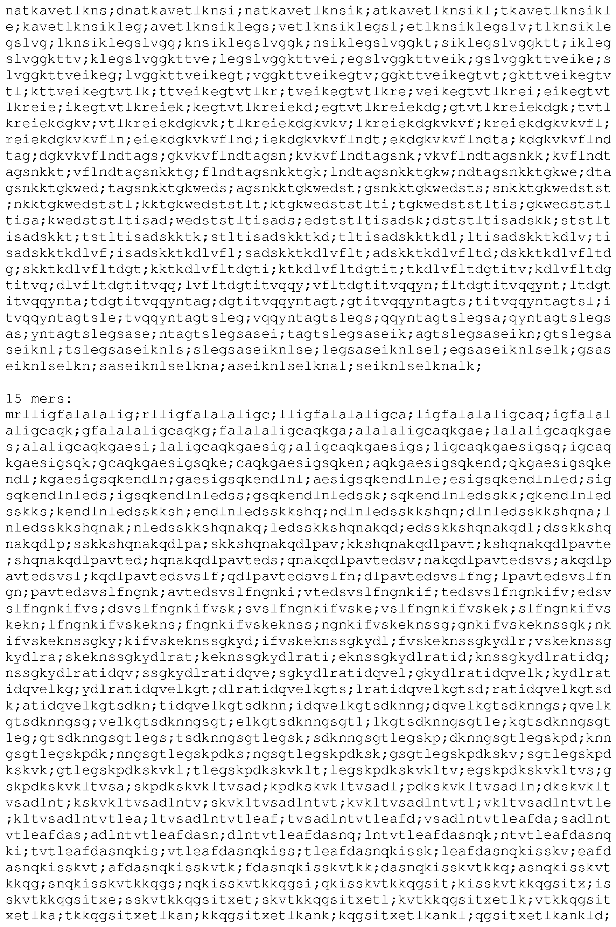

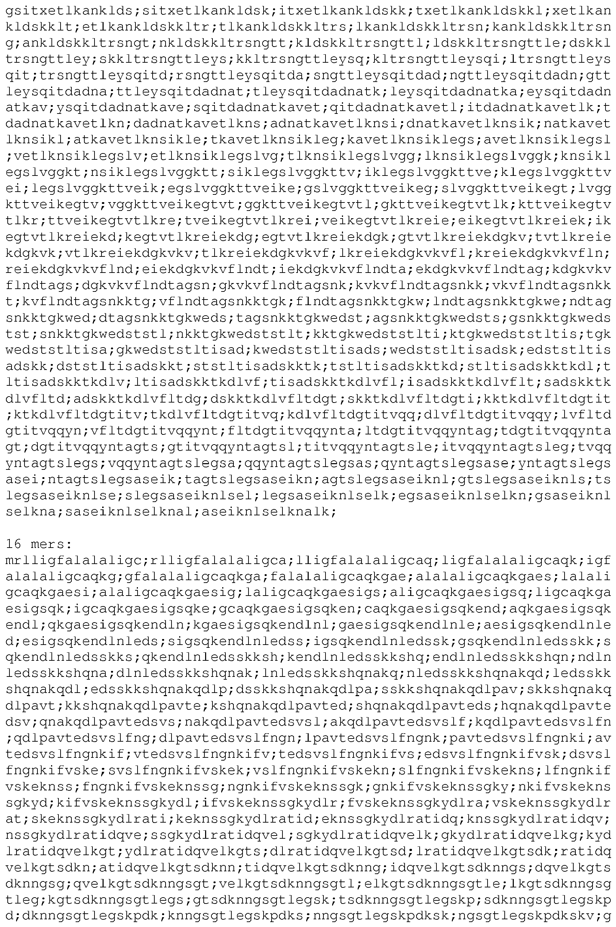

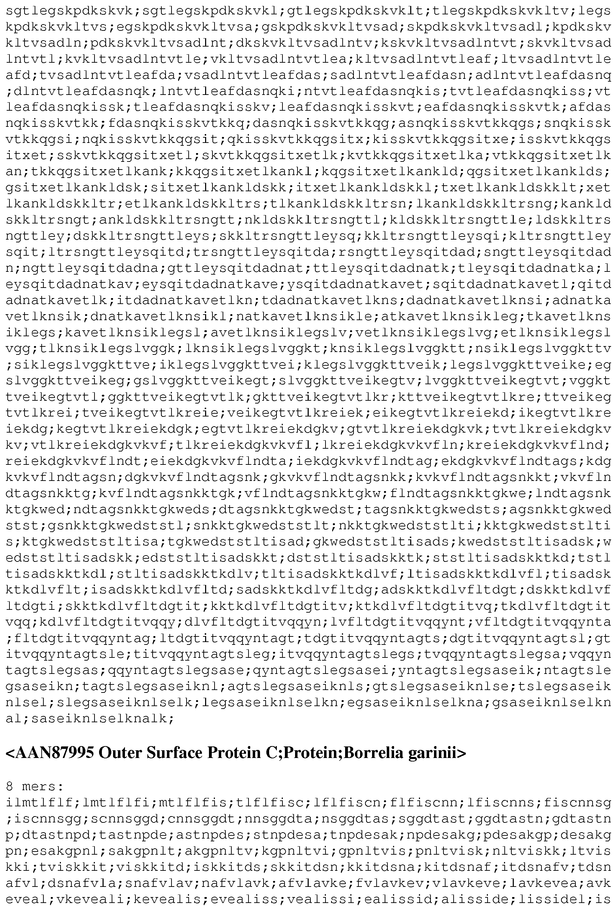

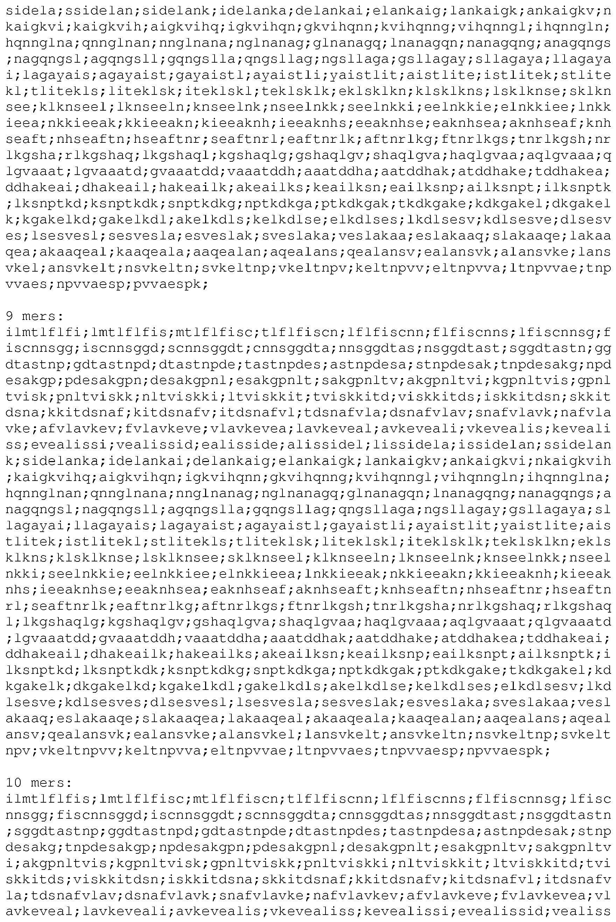

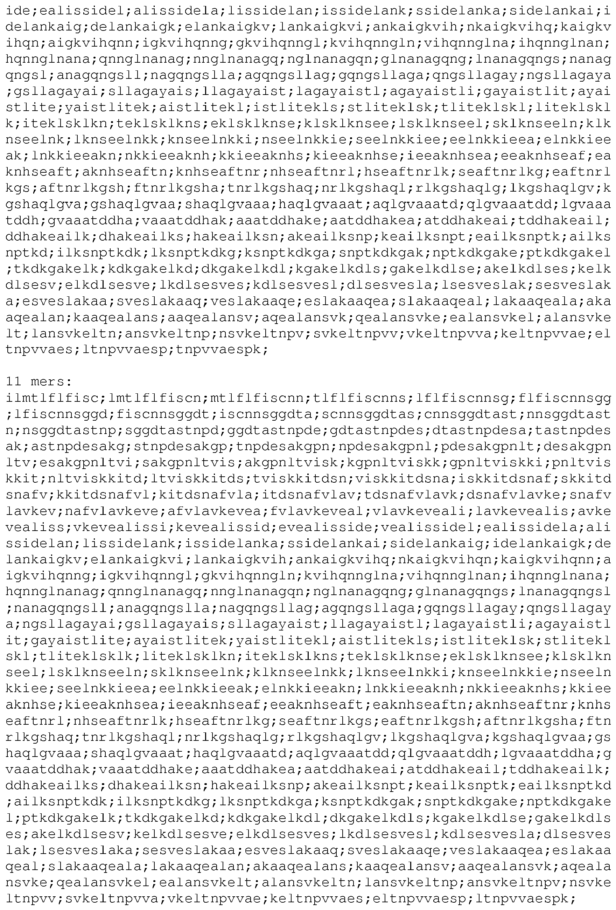

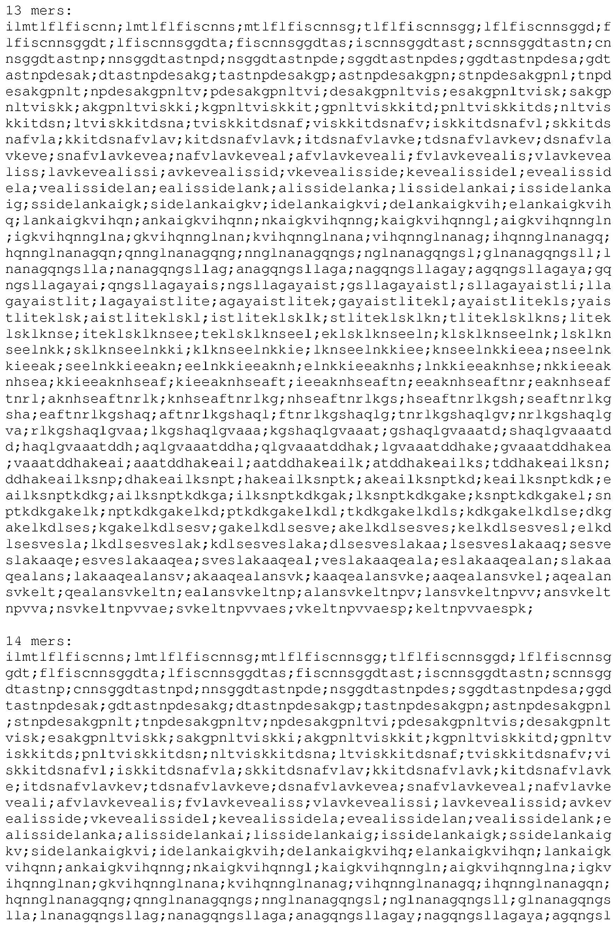

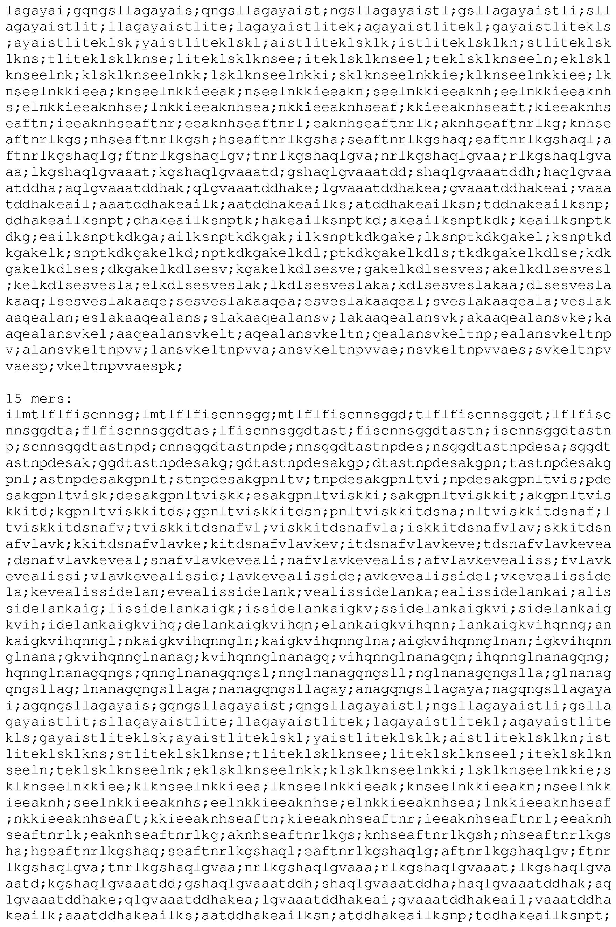

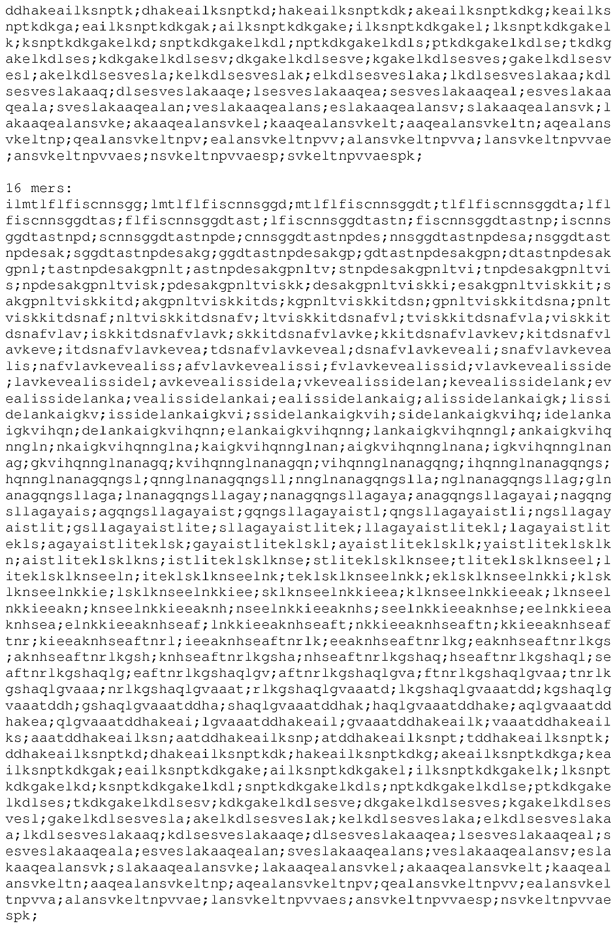

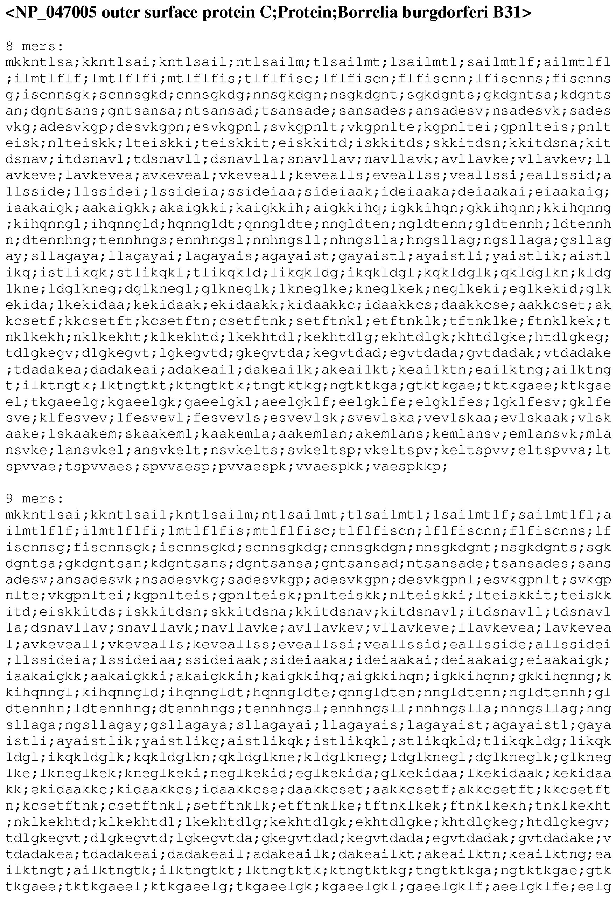

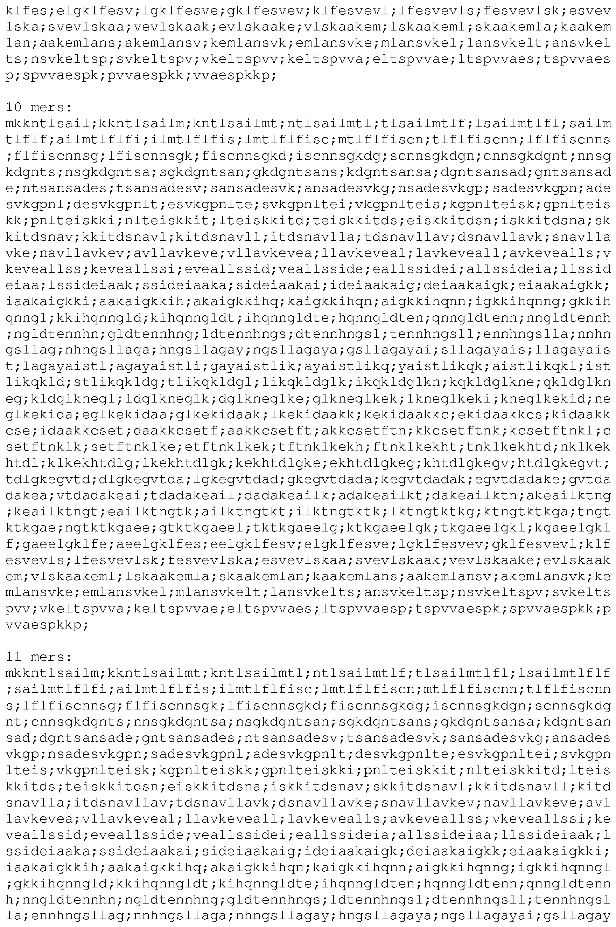

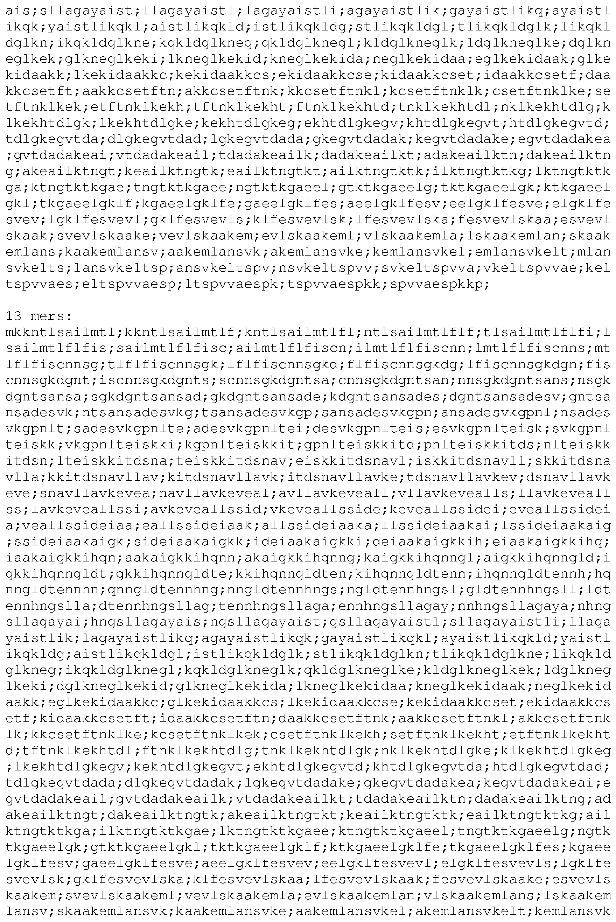

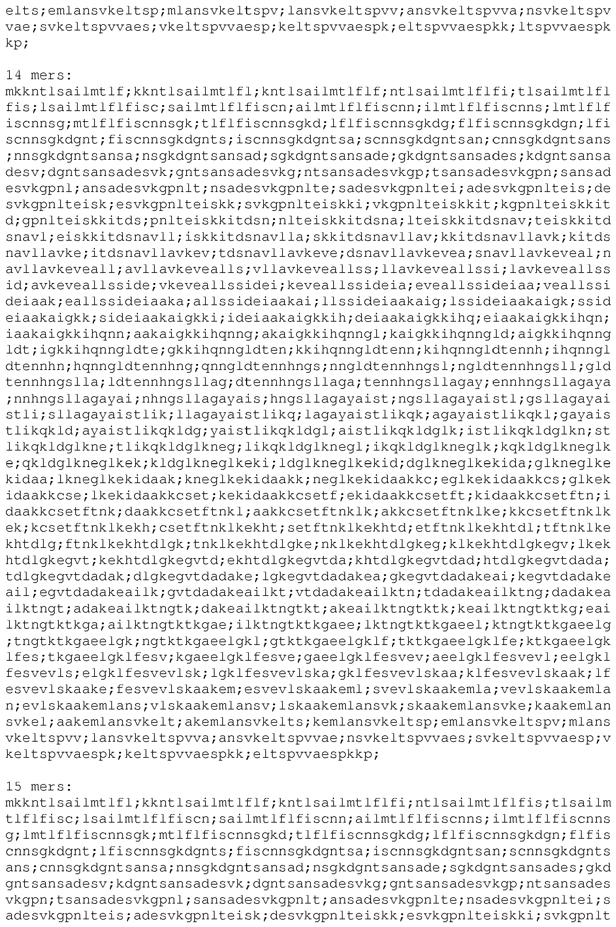

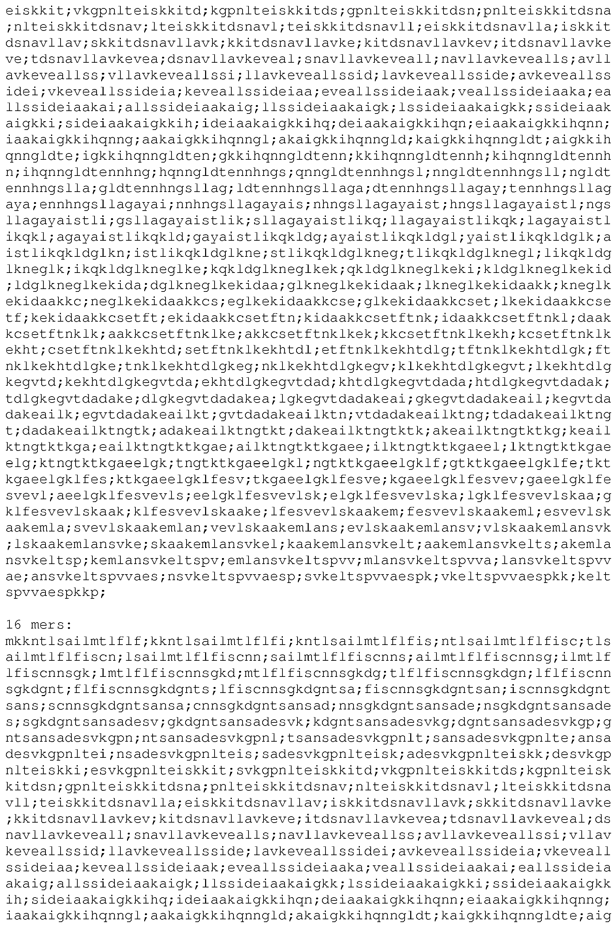

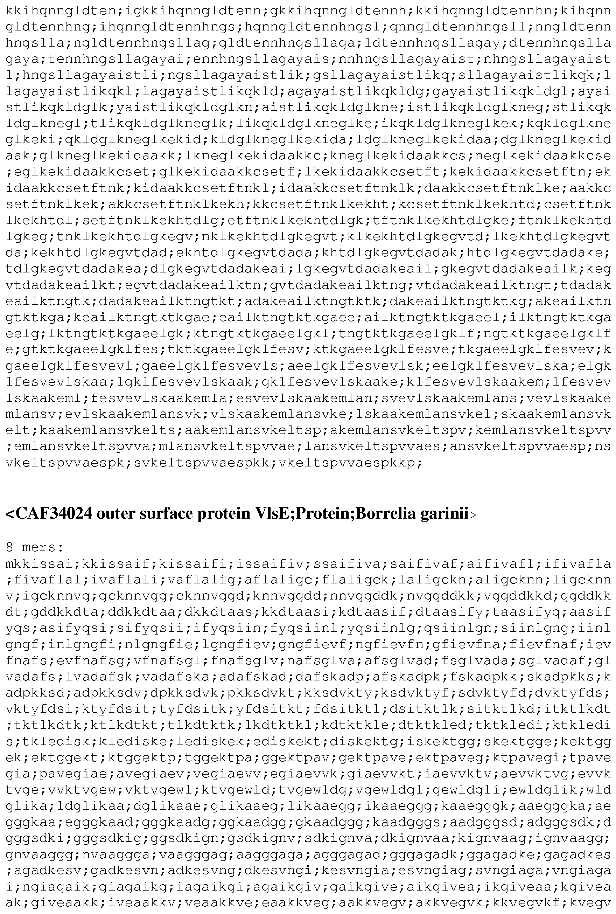

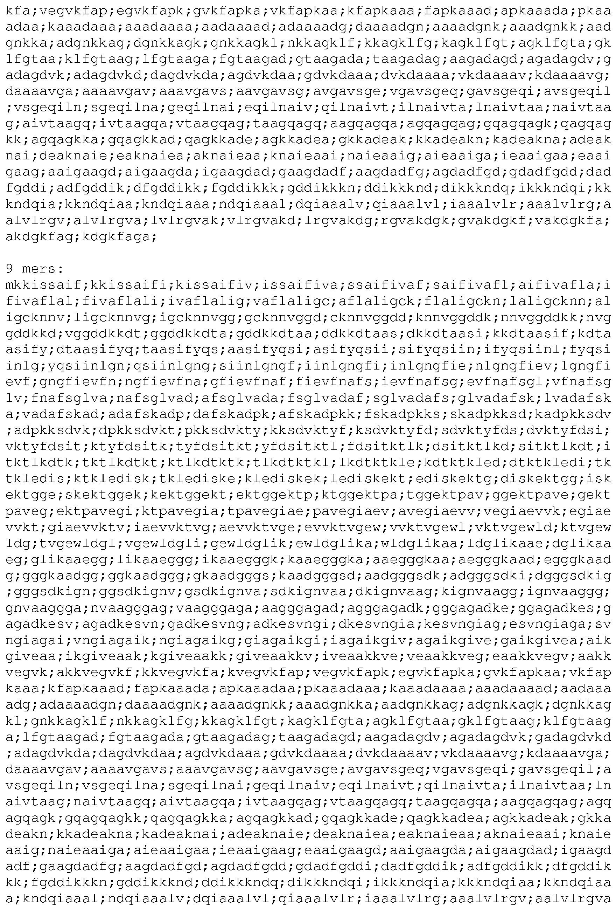

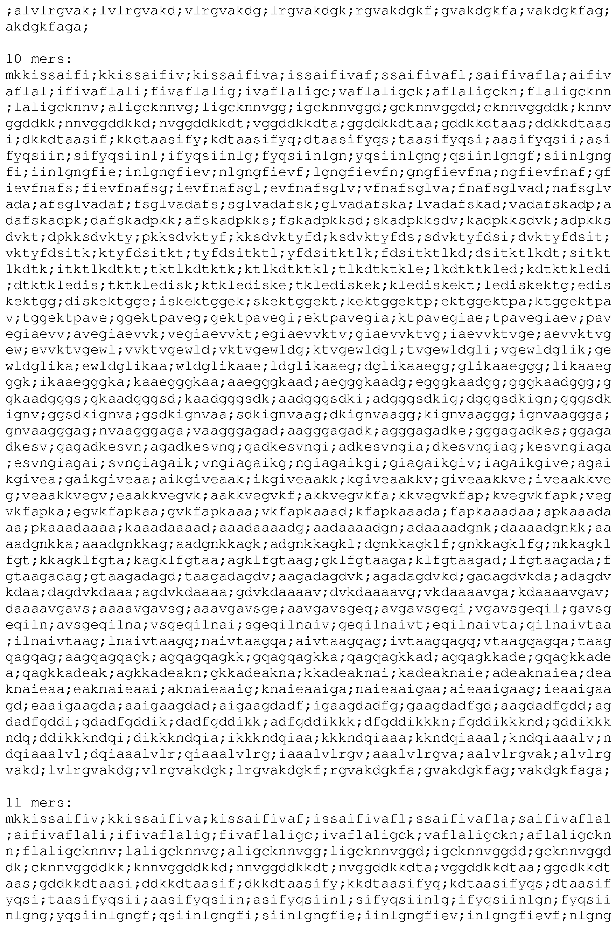

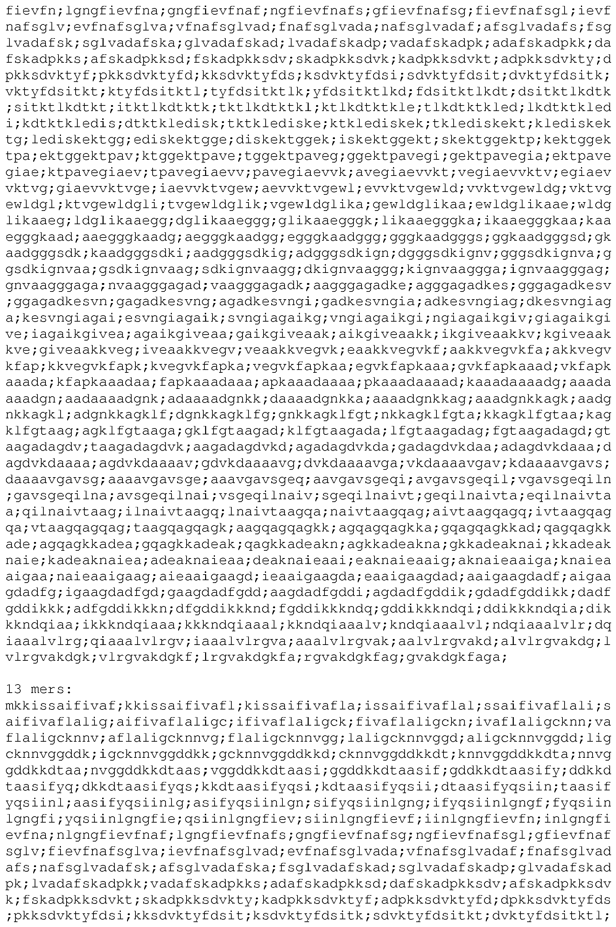

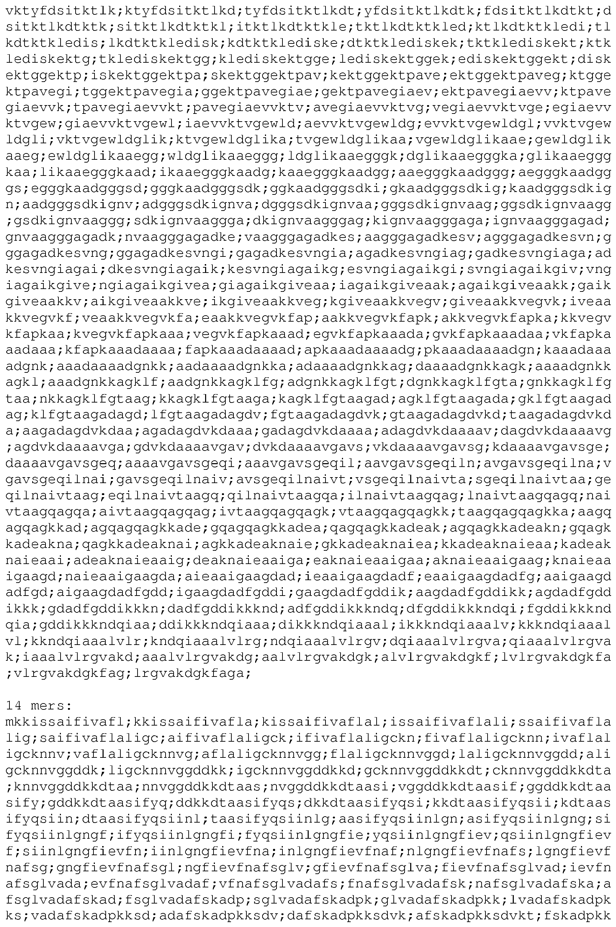

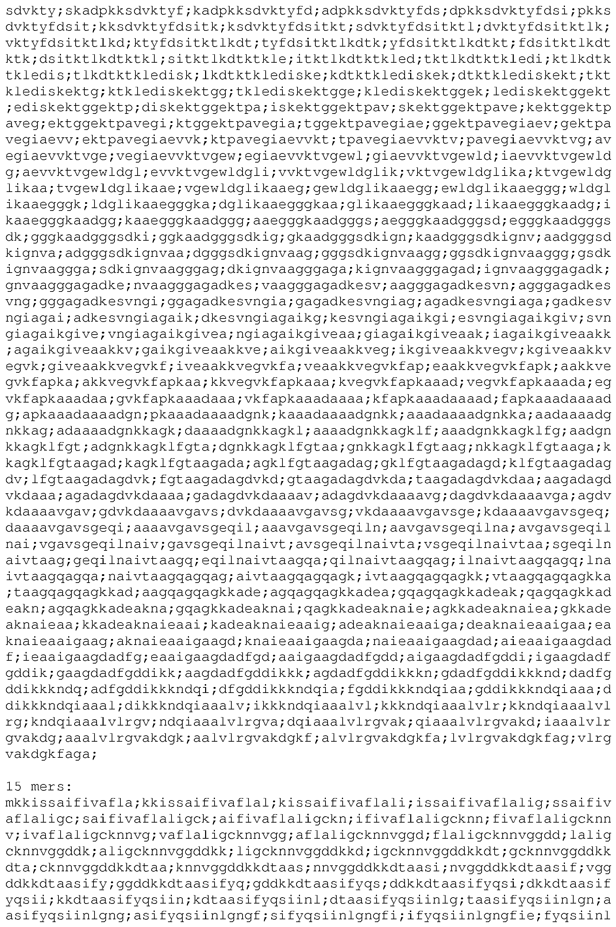

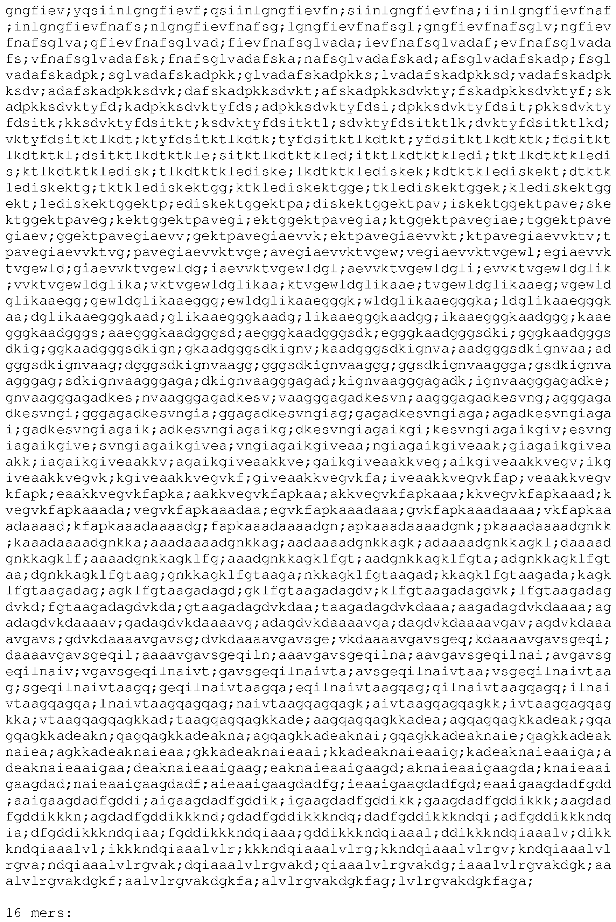

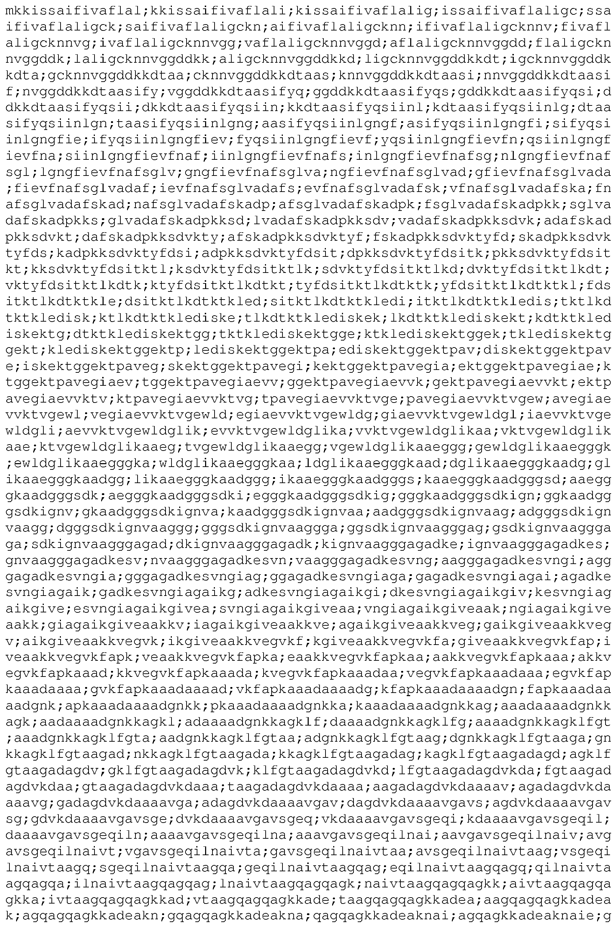

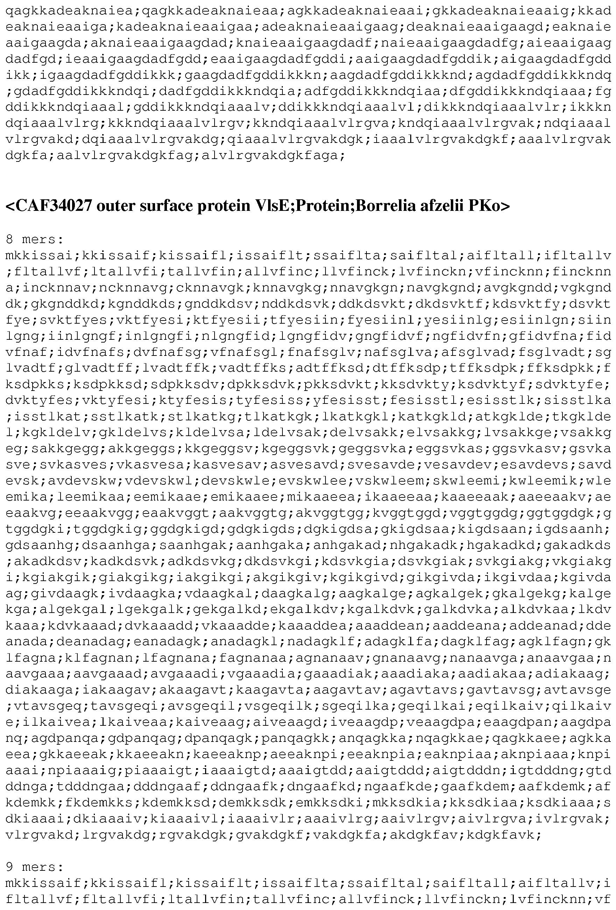

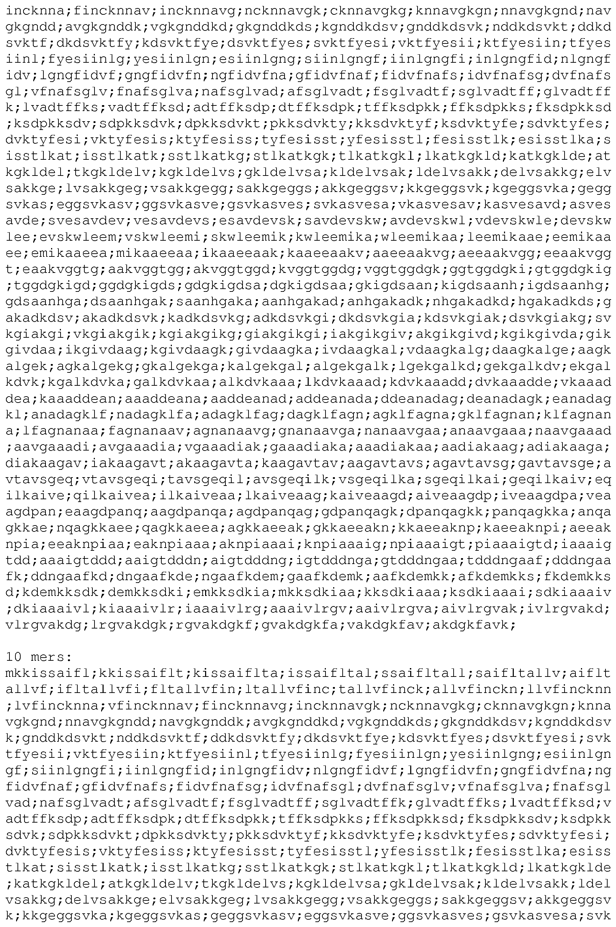

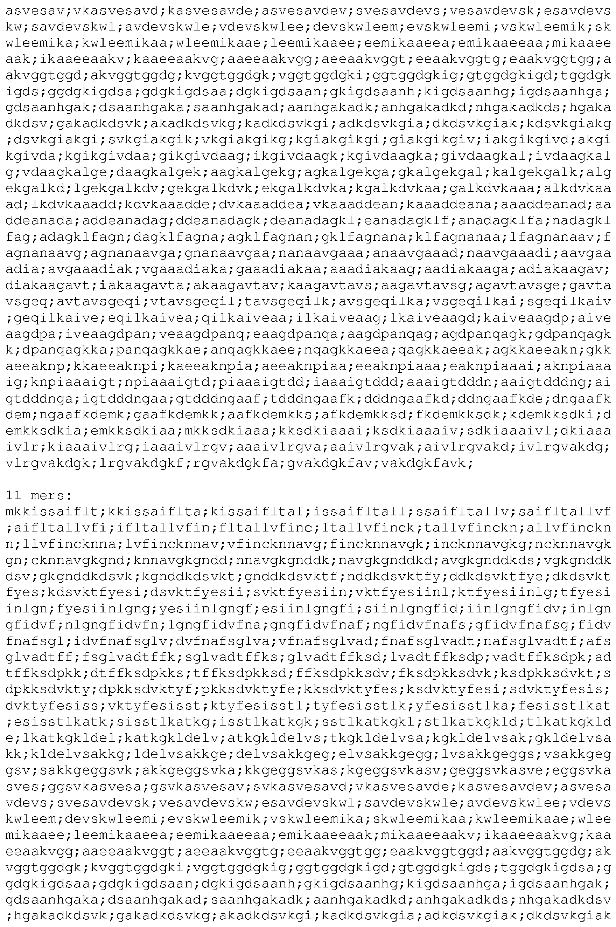

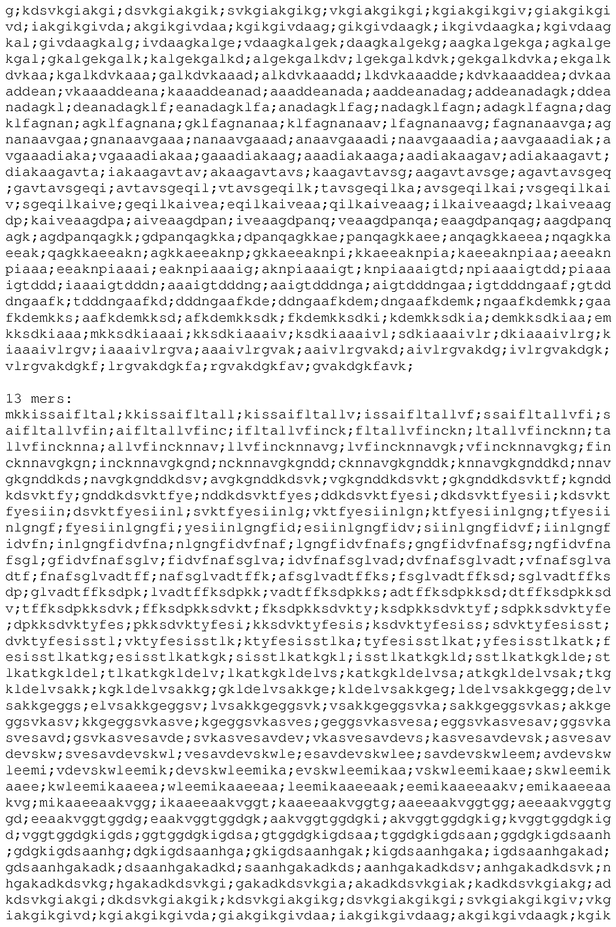

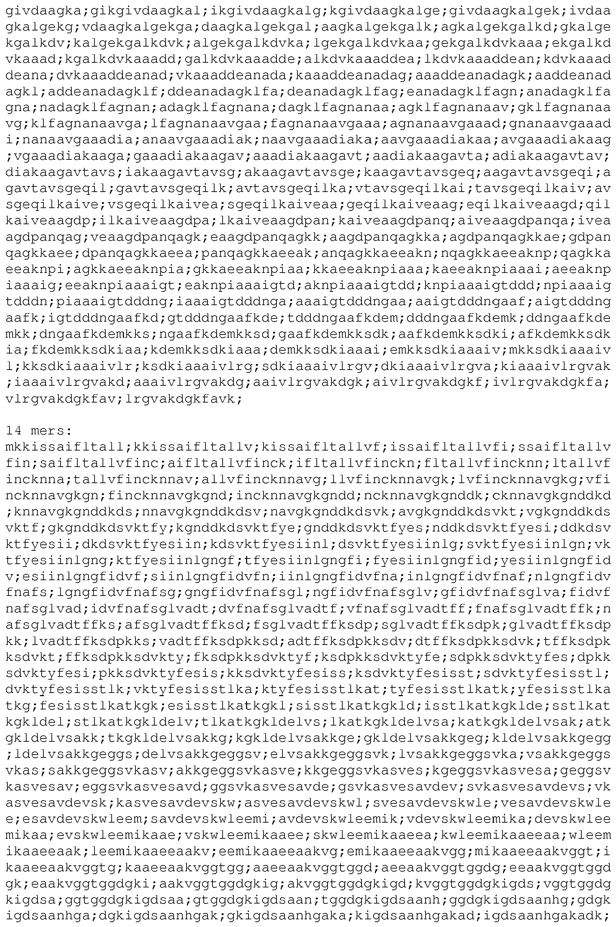

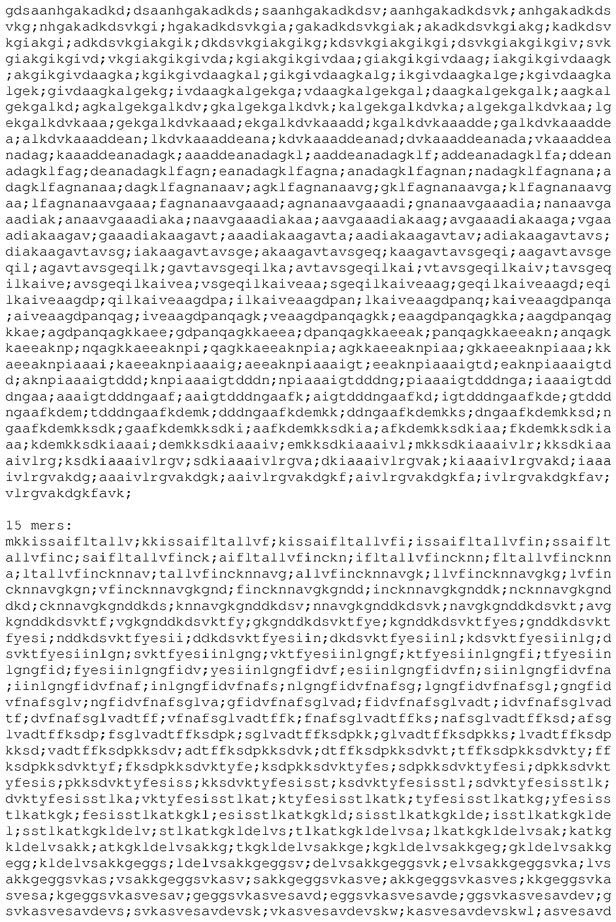

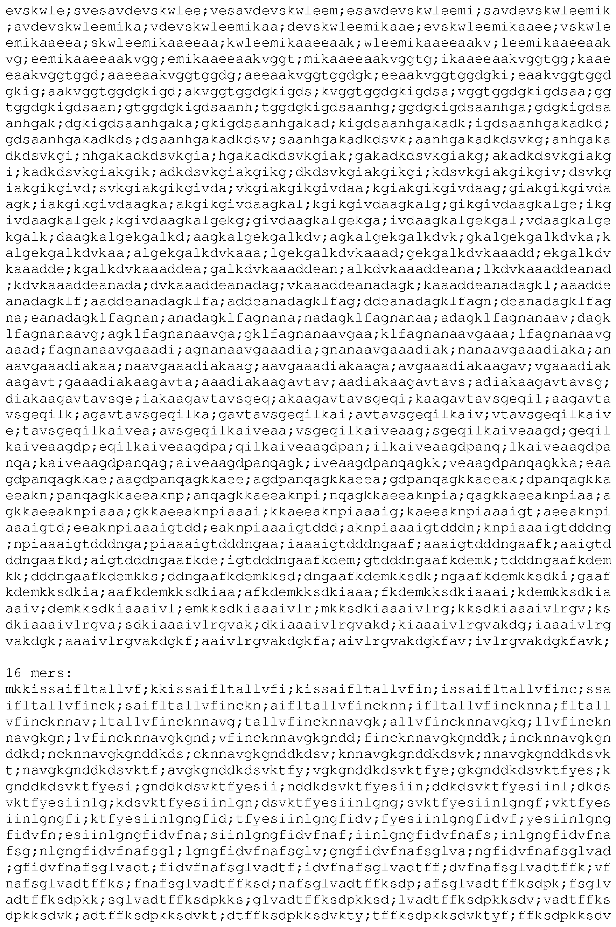

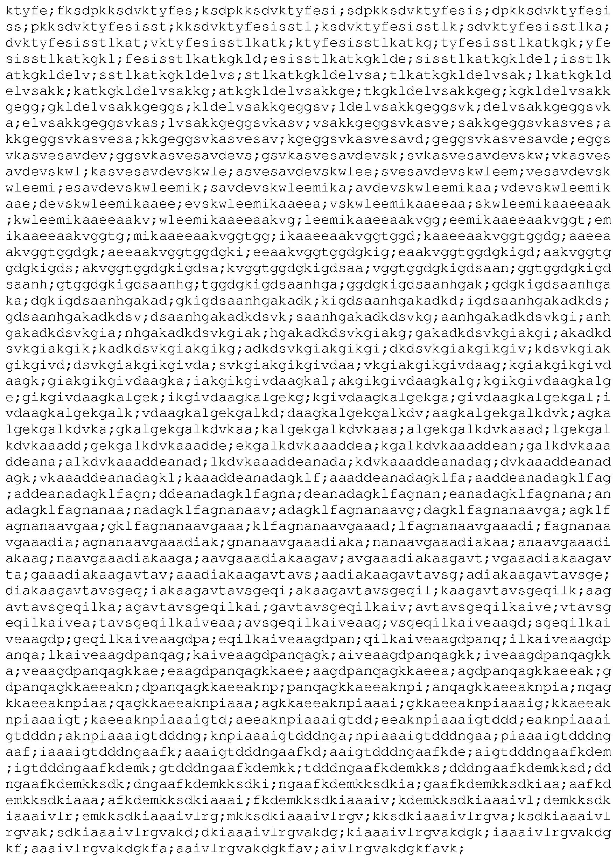

Figure 39: Borrelia MHC class 1 and 2 antigen peptides of 8-, 9-, 10- and 11 amino acids and 13-, 14-, 15- and 16 amino acids in length generated from the chosen protein sequences by random prediction software. Name and protein bank accession numbers are shown.

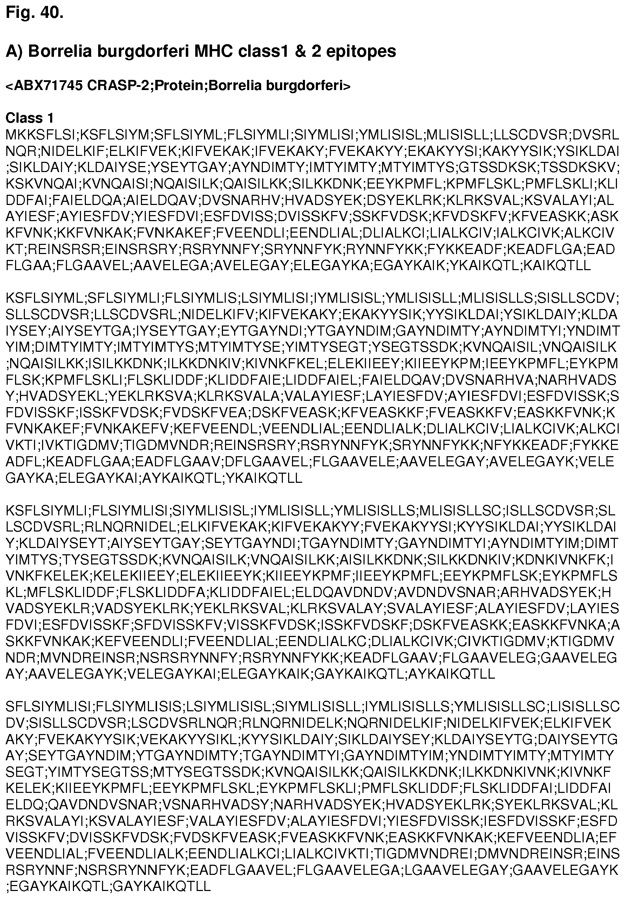

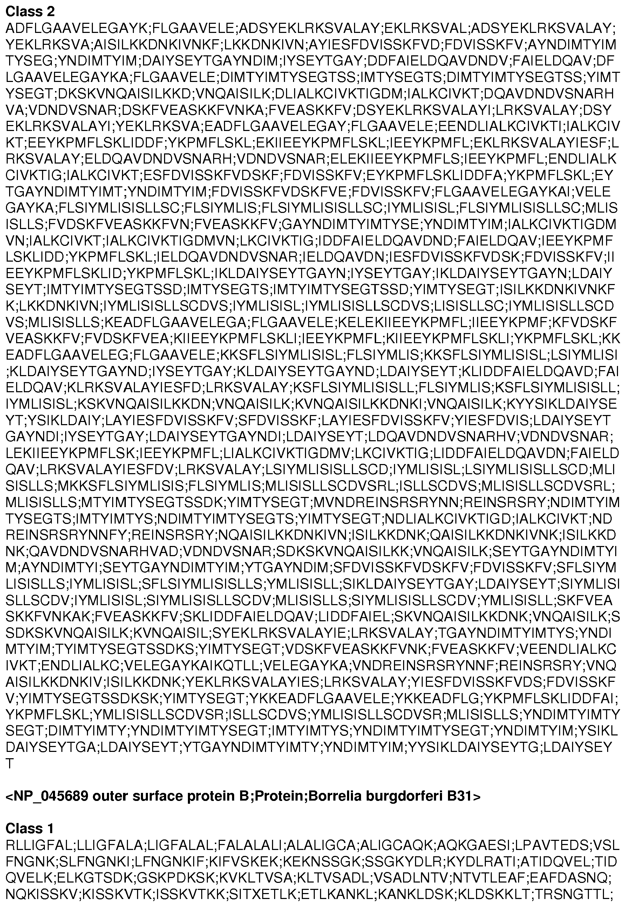

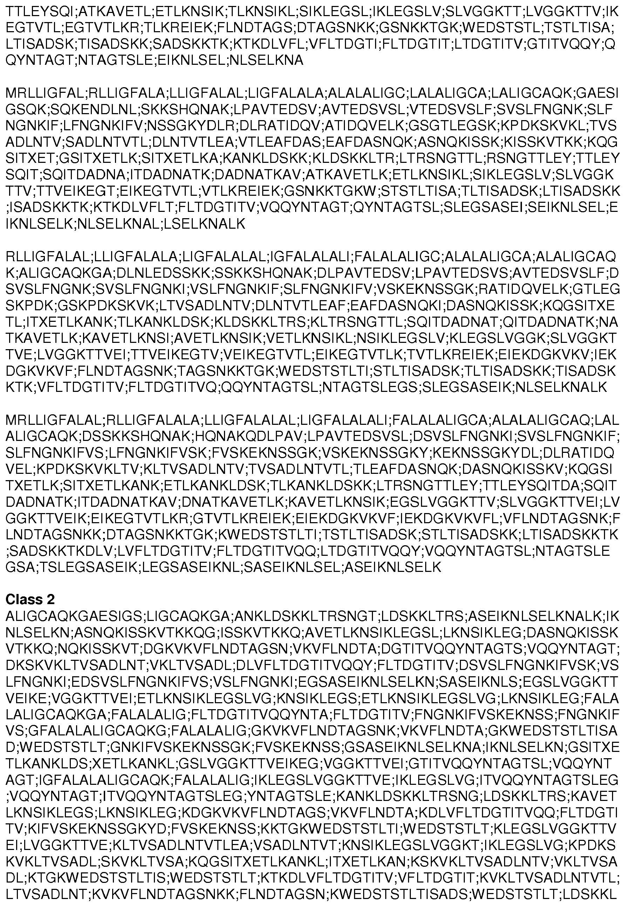

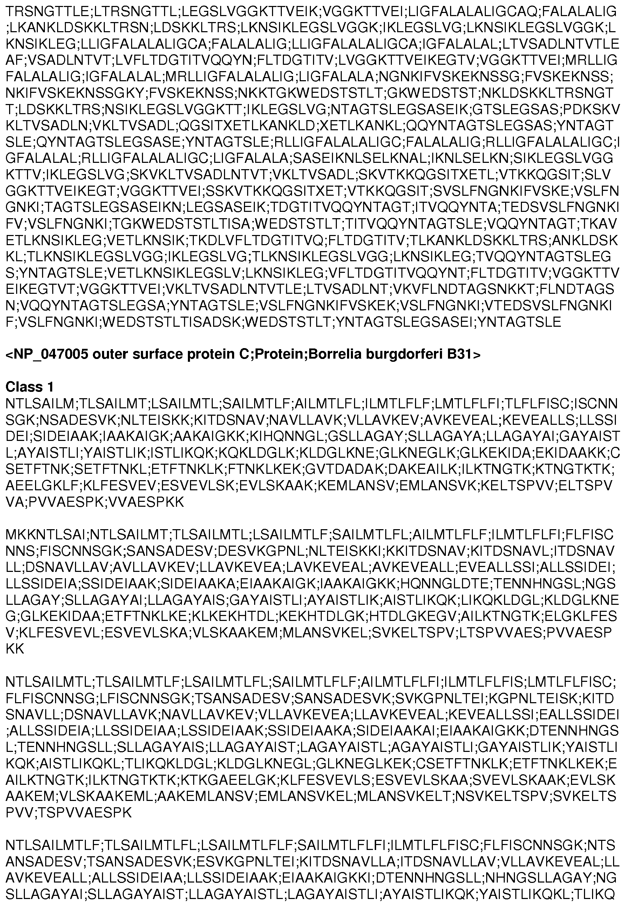

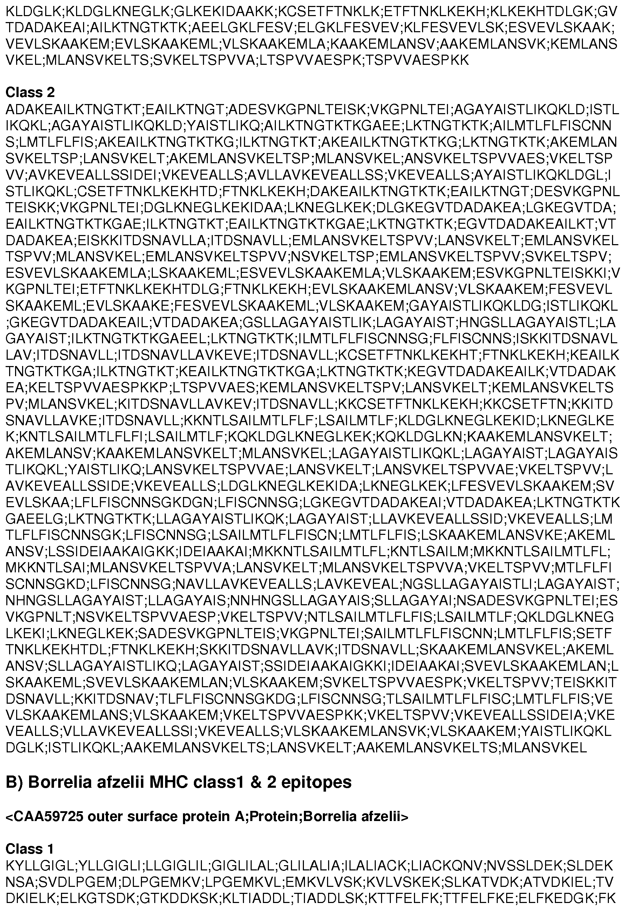

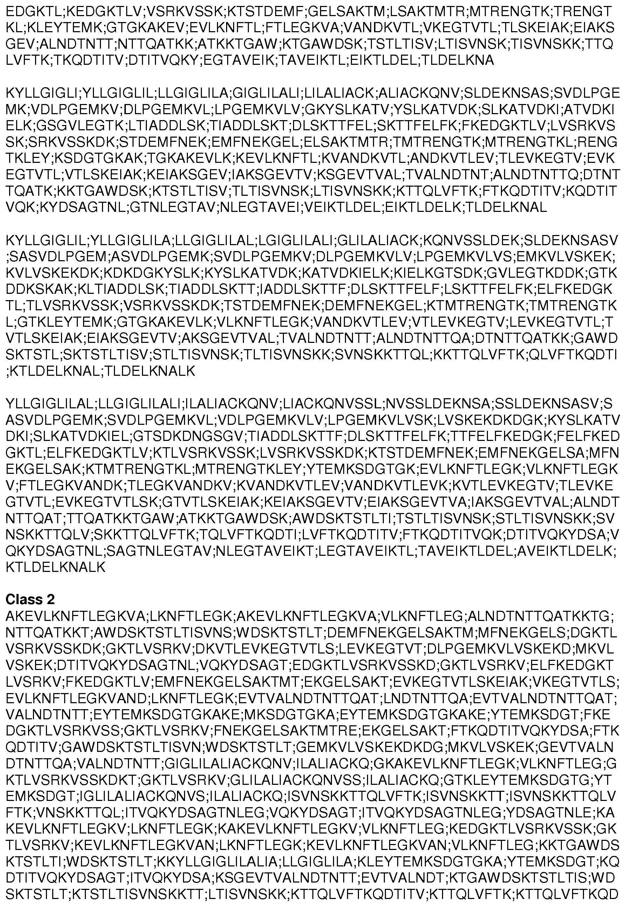

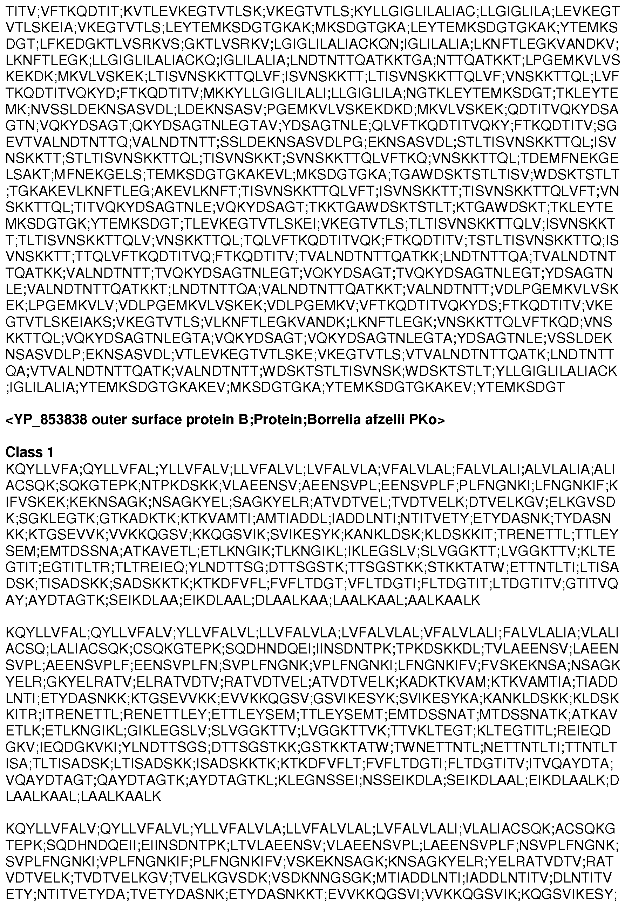

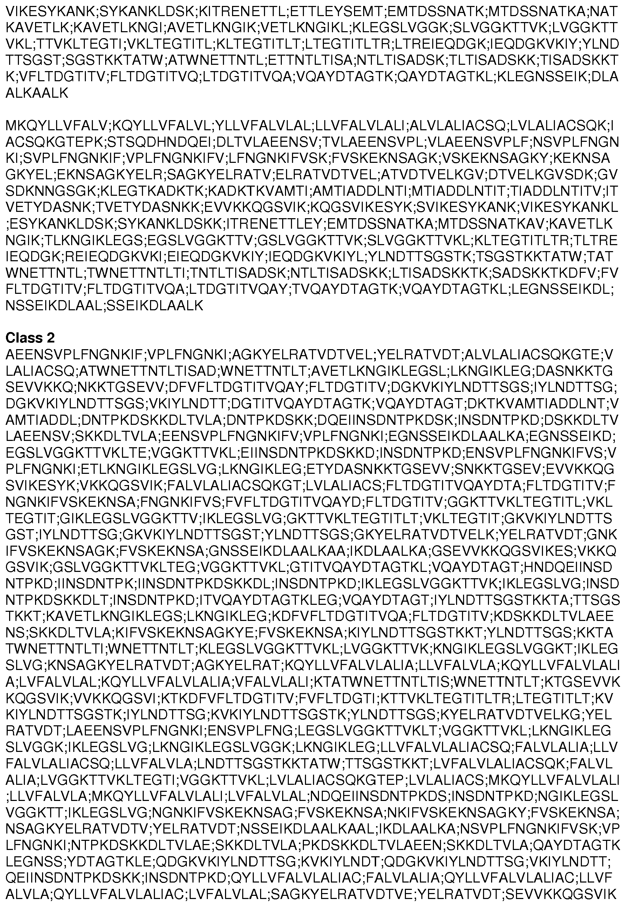

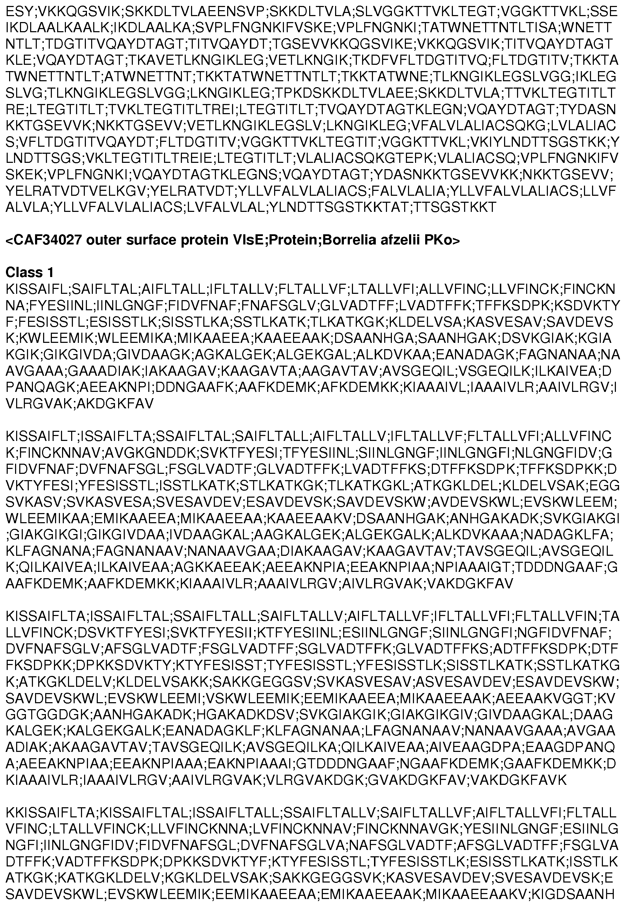

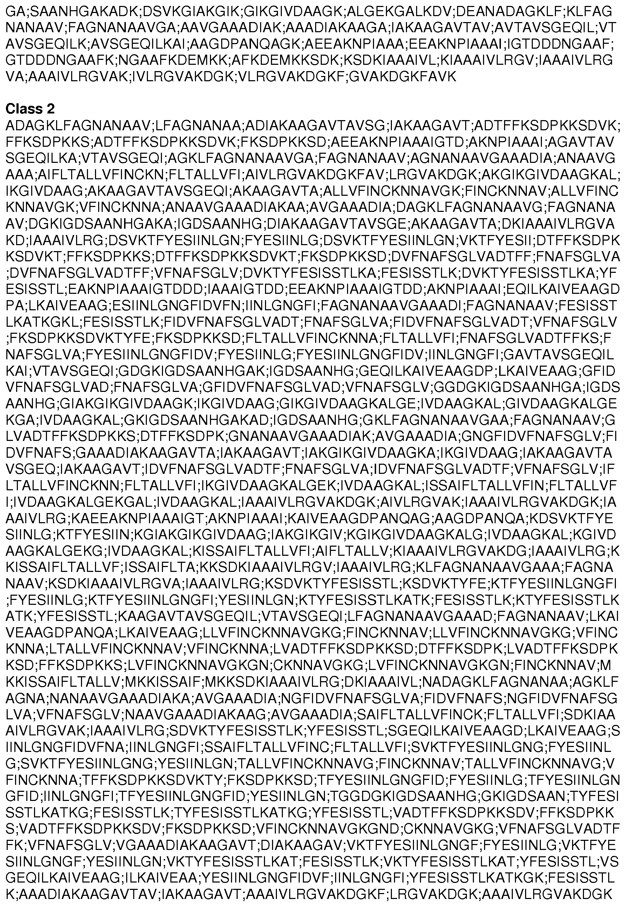

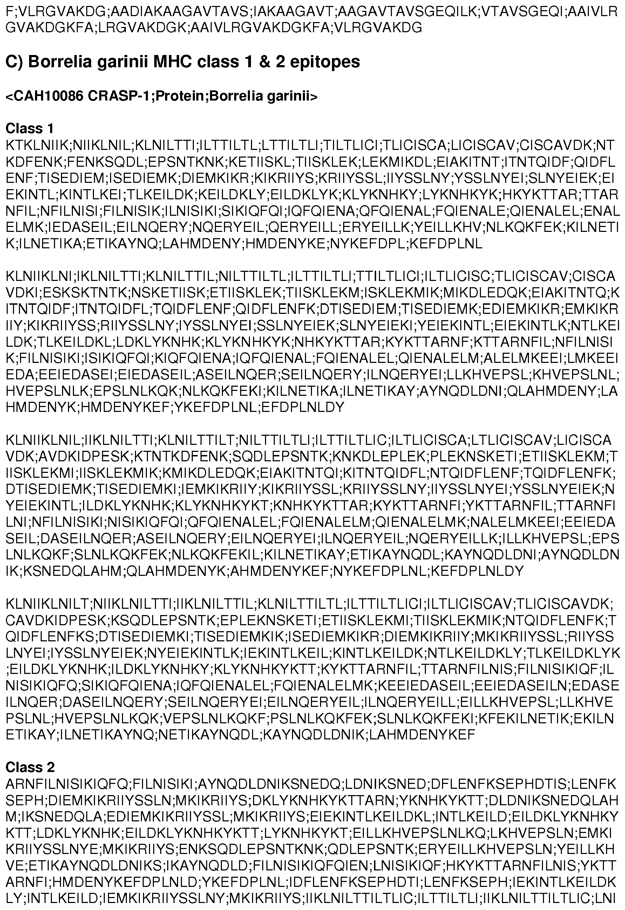

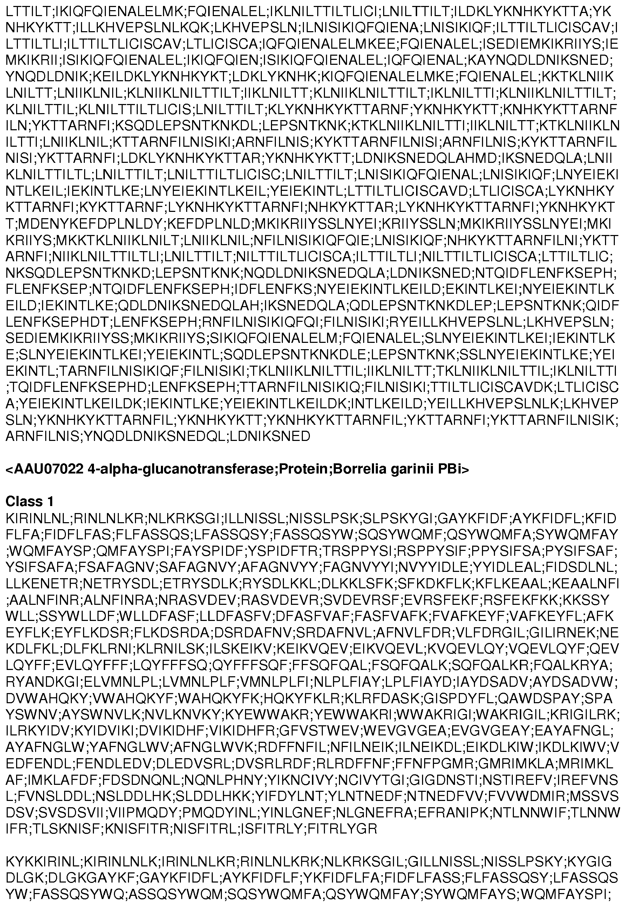

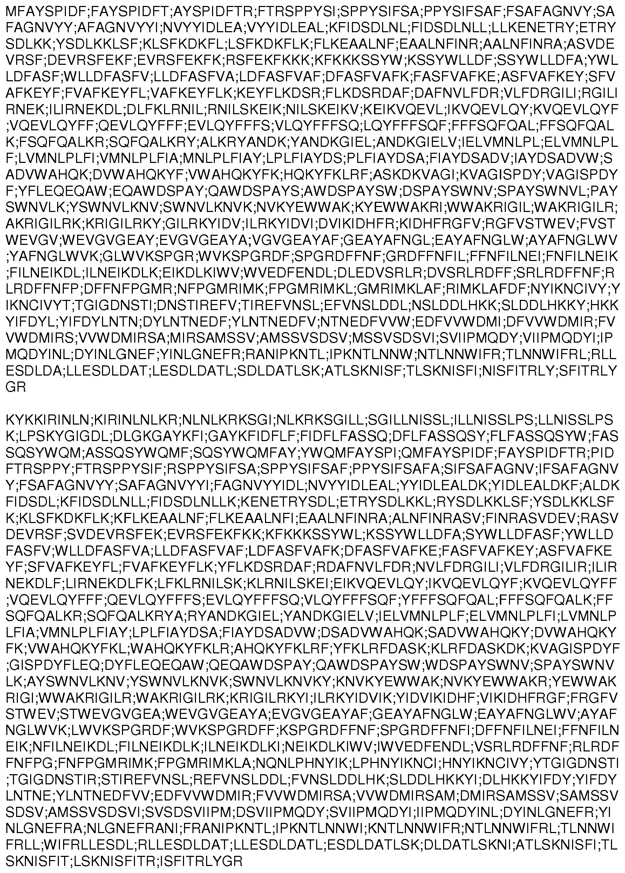

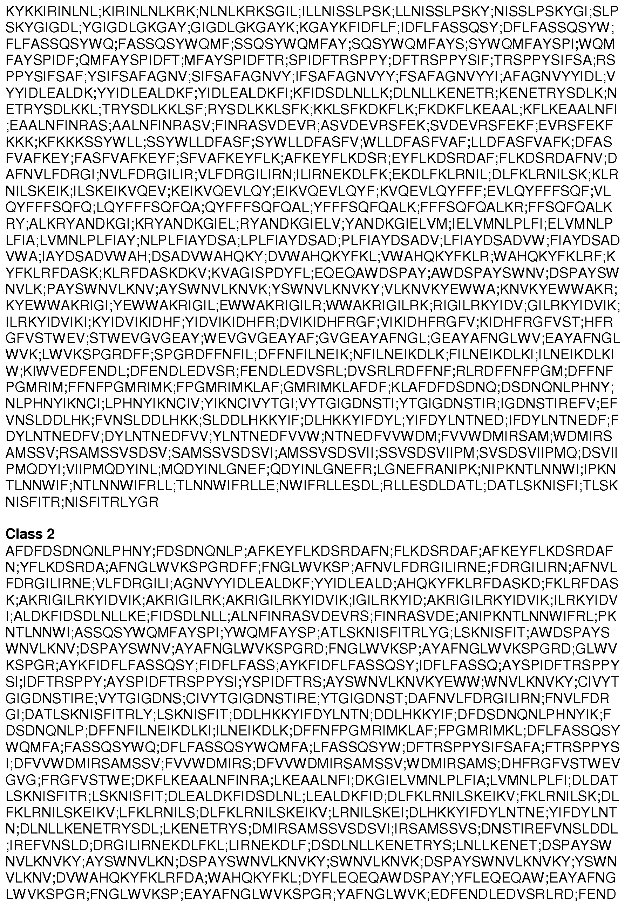

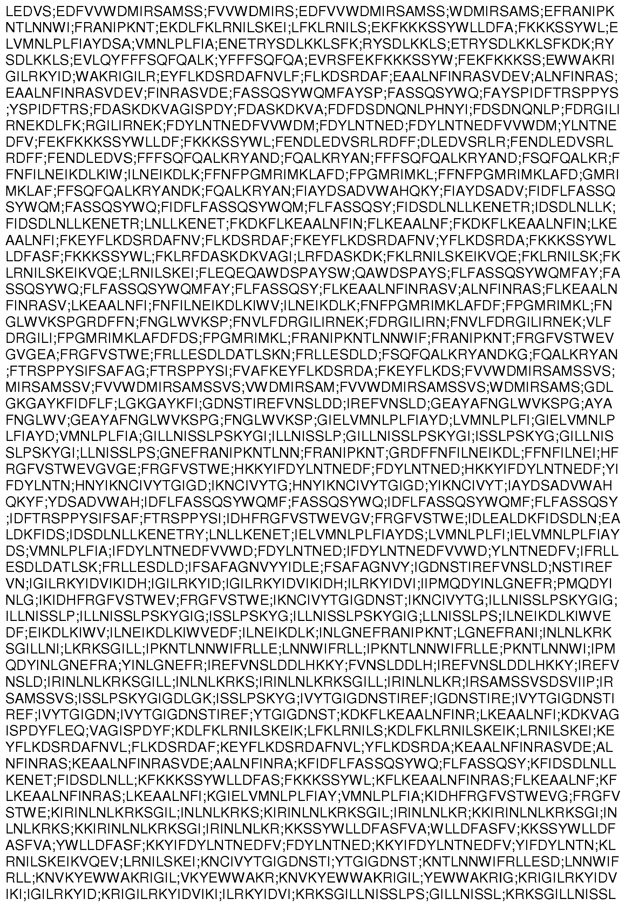

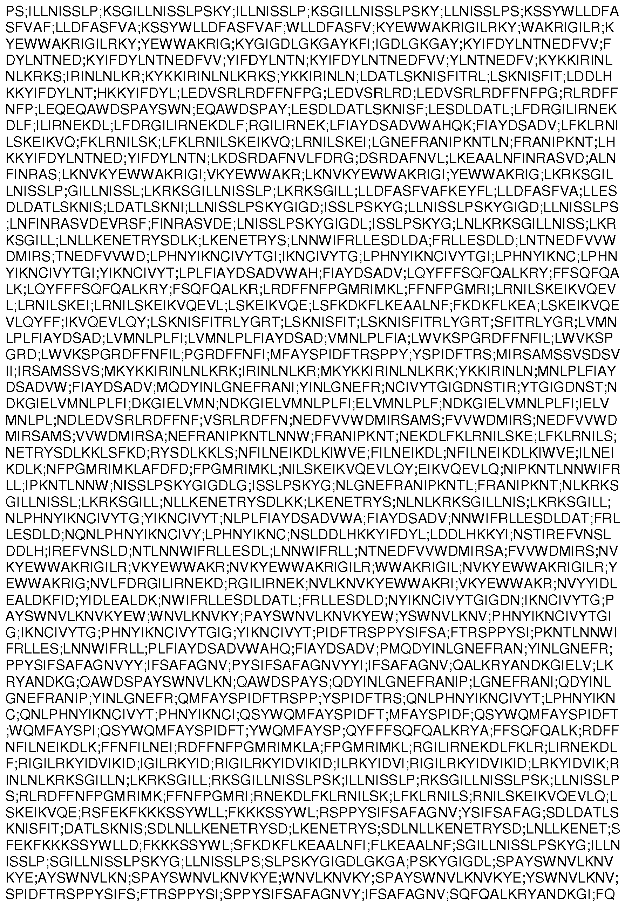

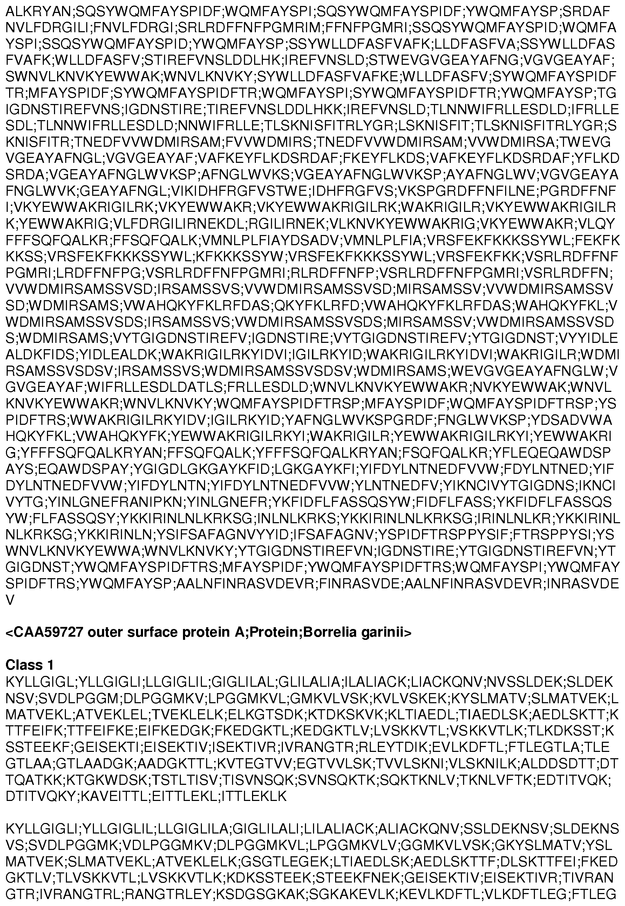

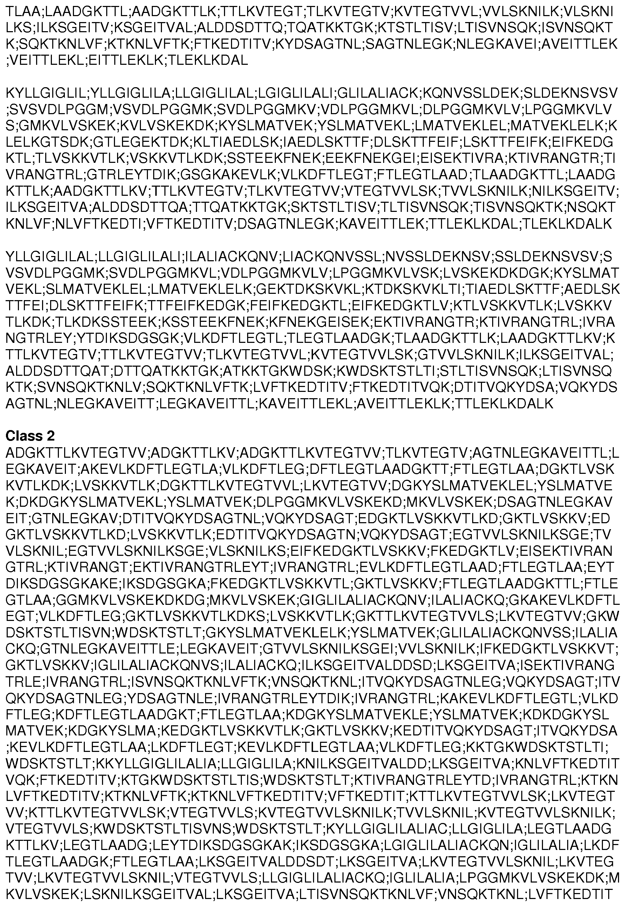

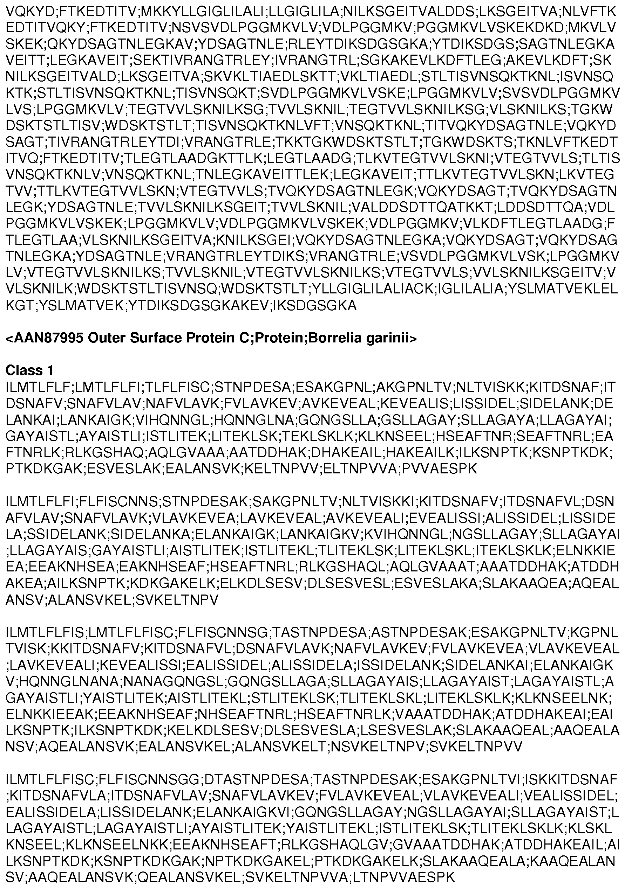

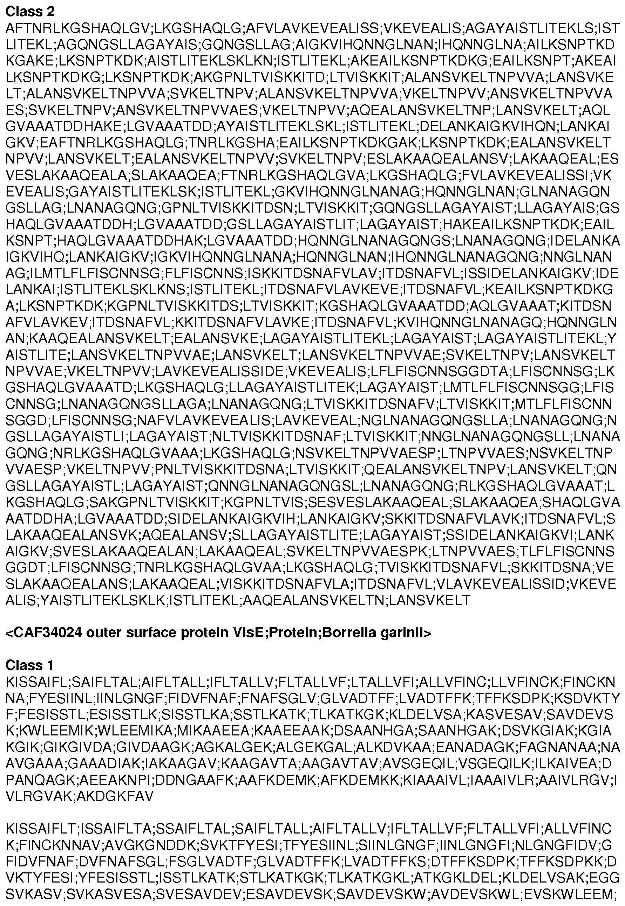

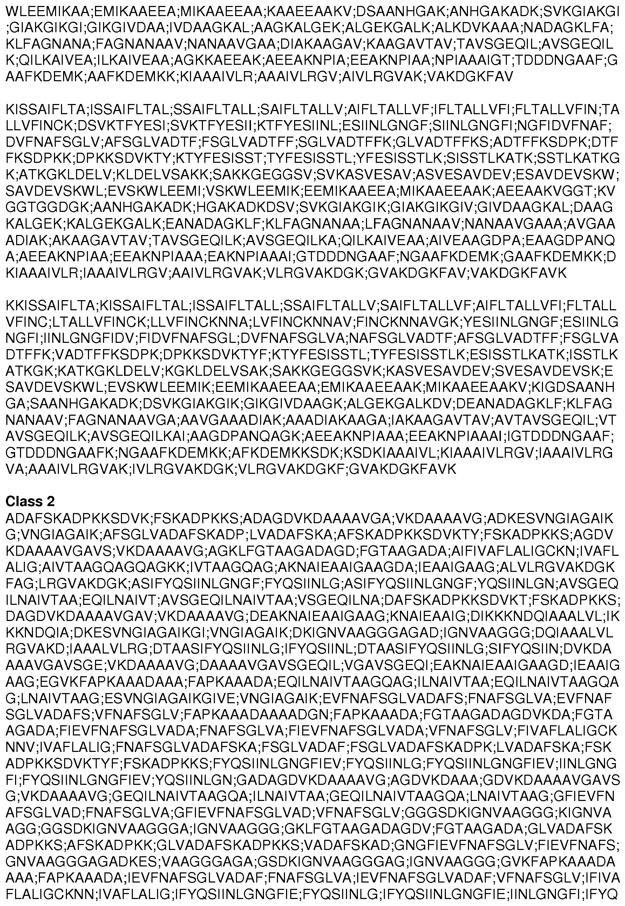

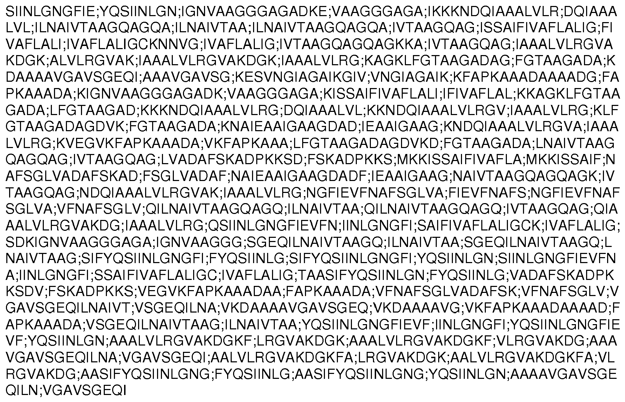

Figure 40: NetMHC peptides. A) Borrelia burgdorferi MHC class1 & 2 epitopes, B) Borrelia afzelii MHC class1 & 2 epitopes, C) Borrelia garinii MHC class 1 & 2 epitopes. The NetMHC peptides are predicted by the http://www.cbs.dtu.dk/services/NetMHC/ database.

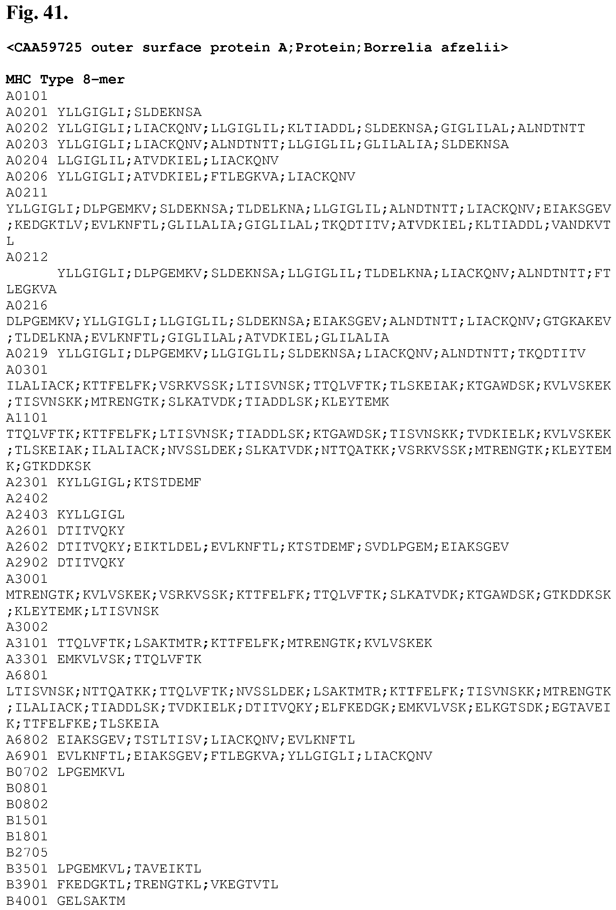

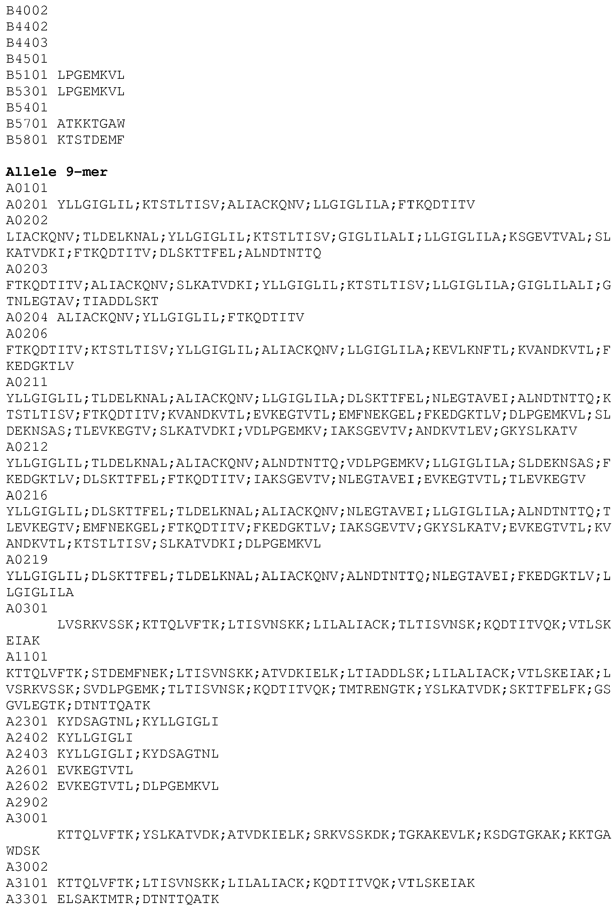

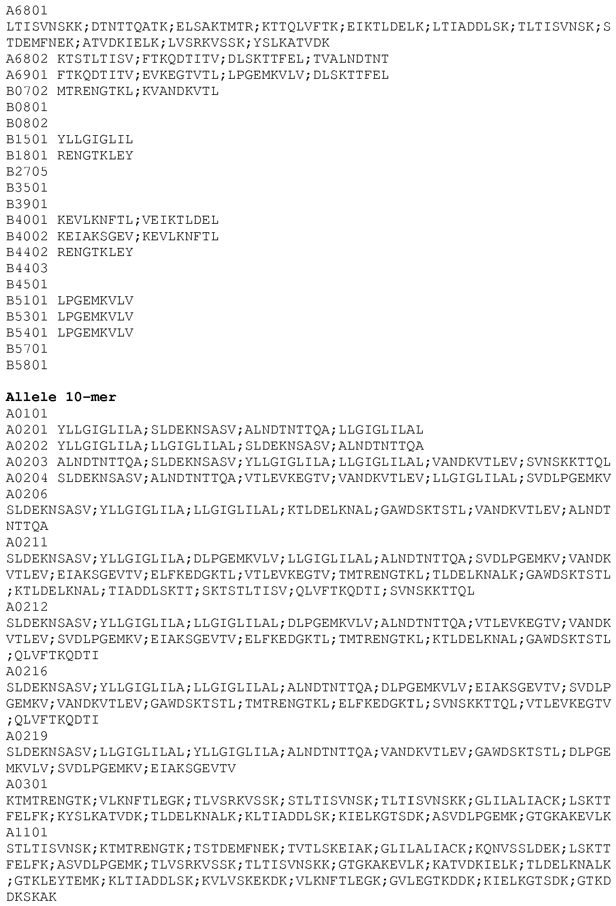

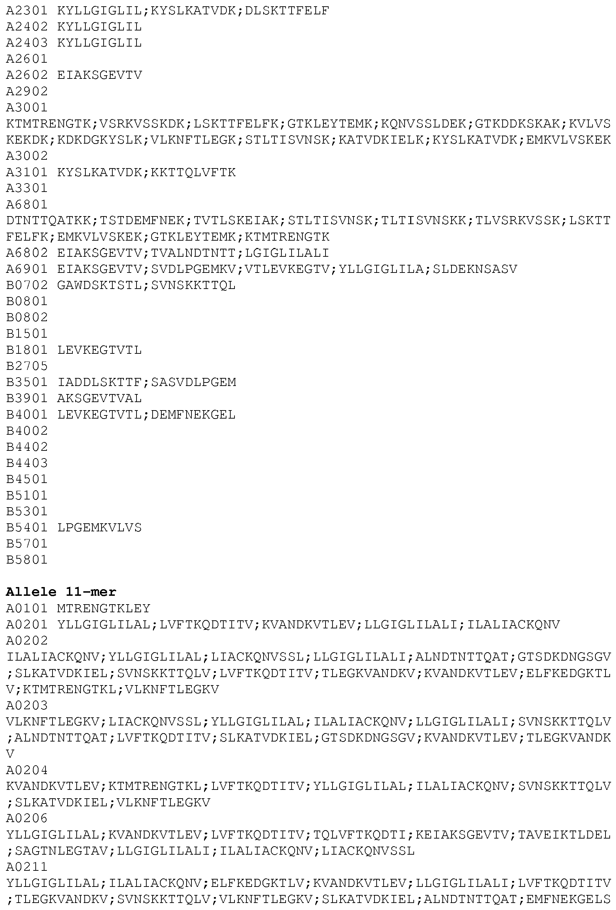

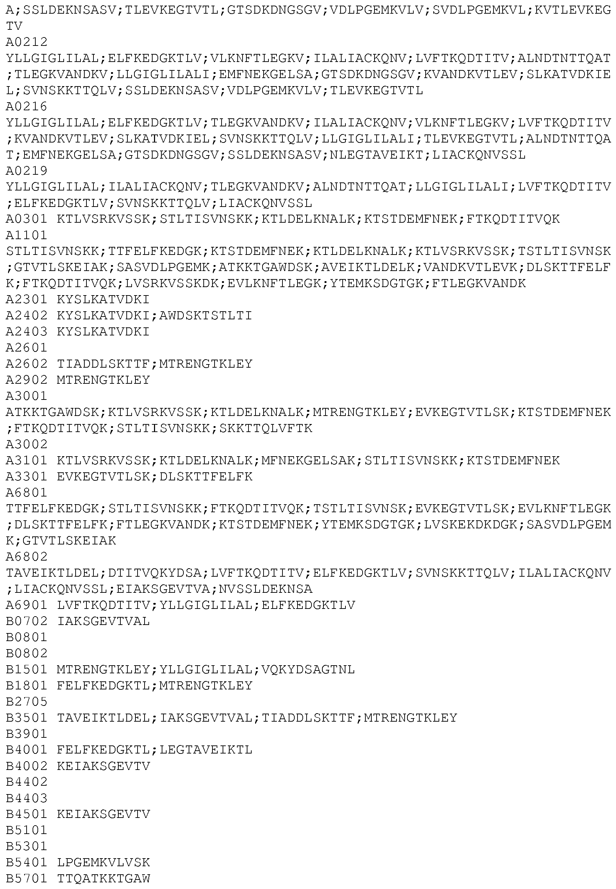

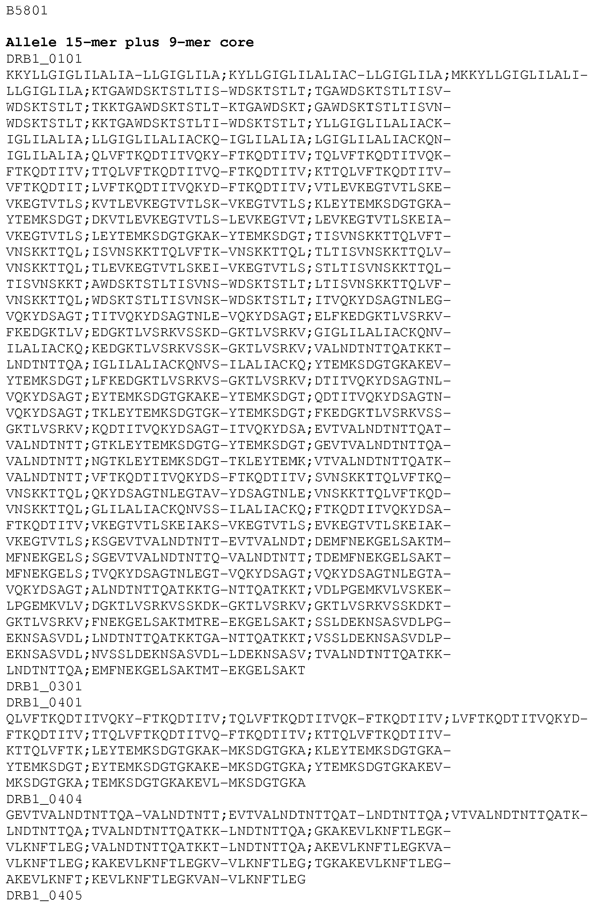

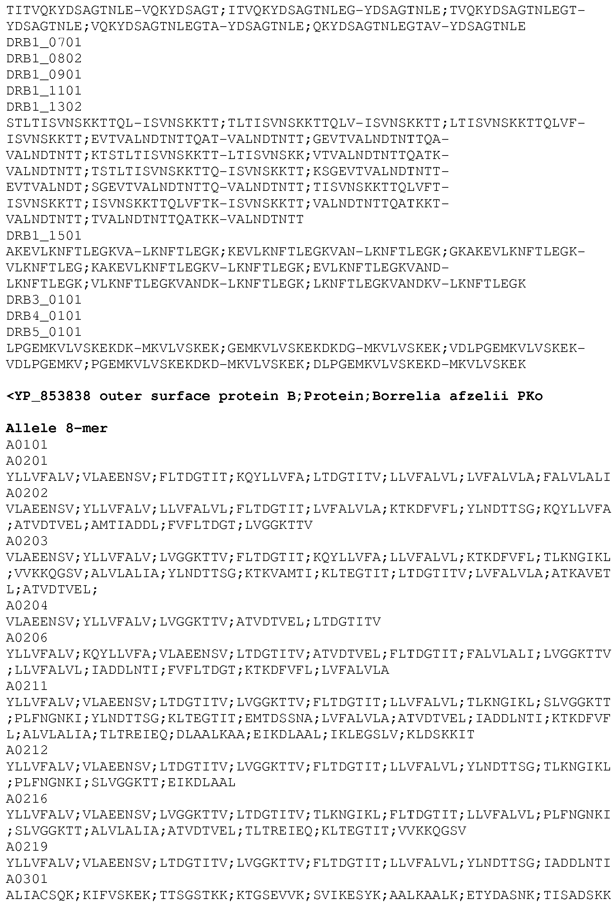

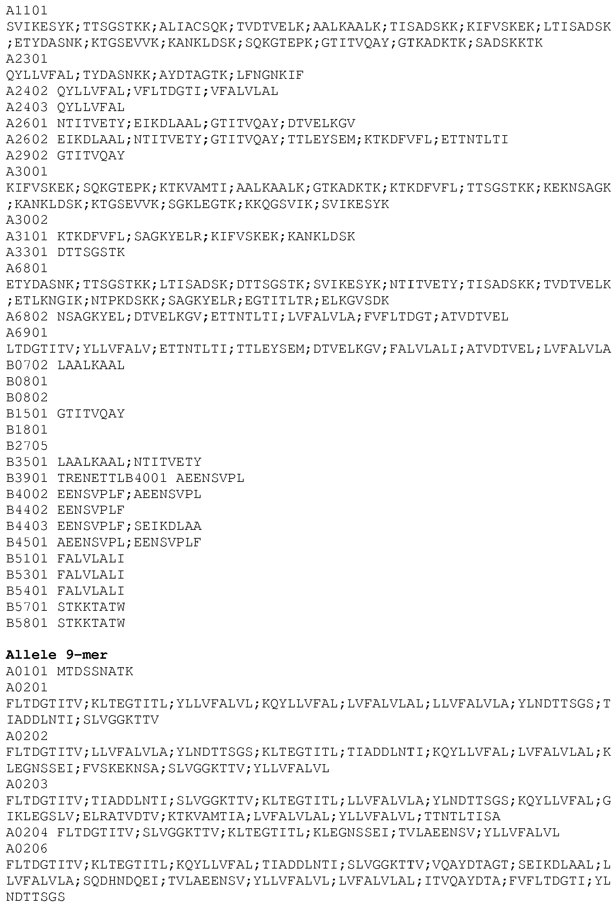

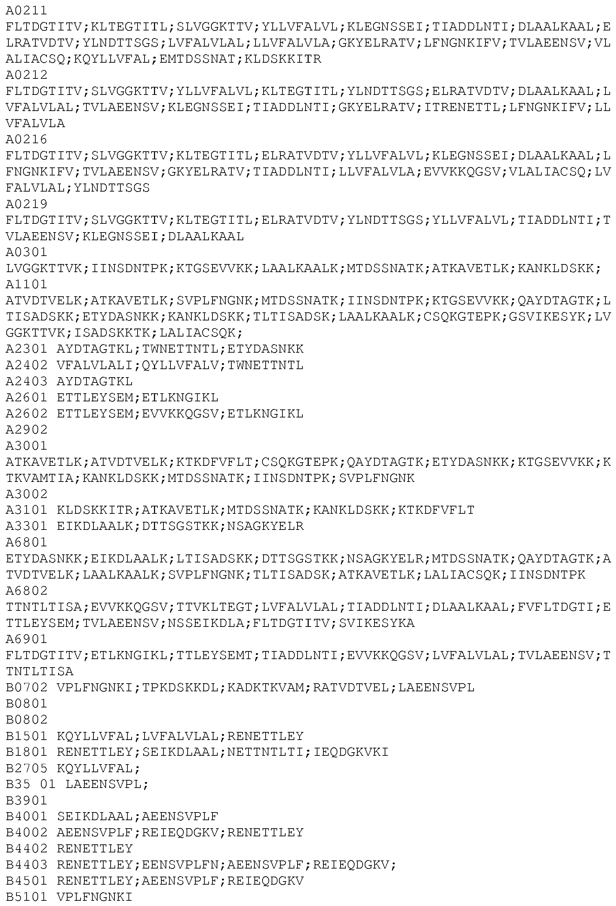

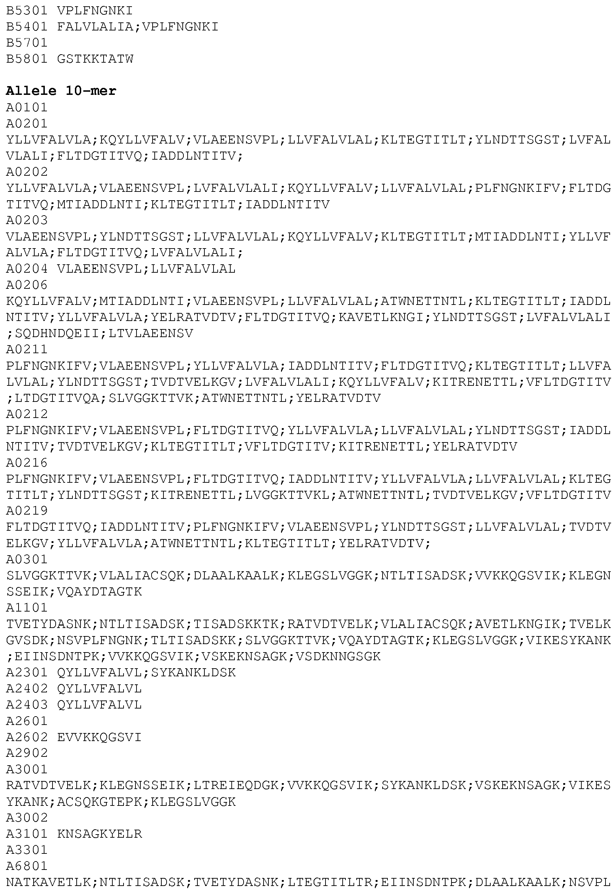

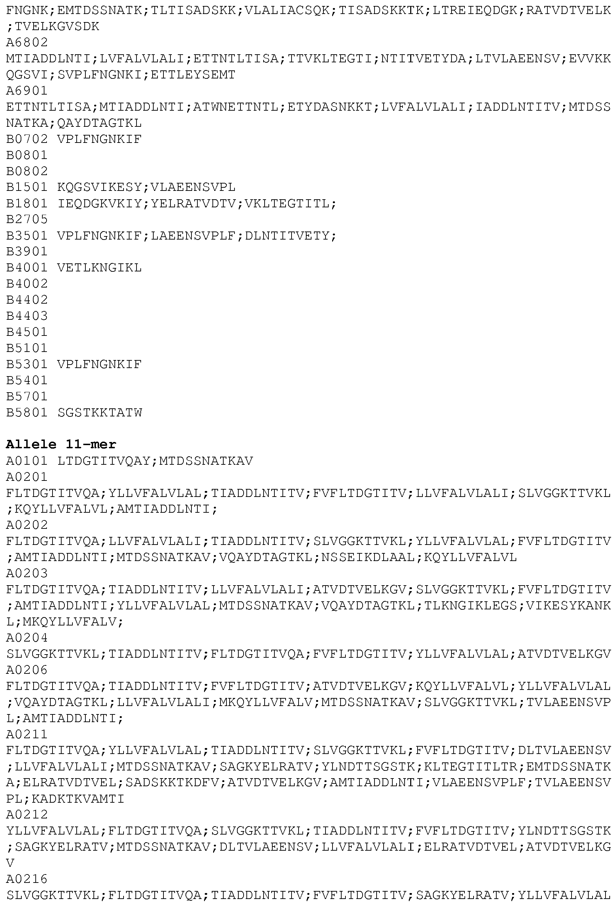

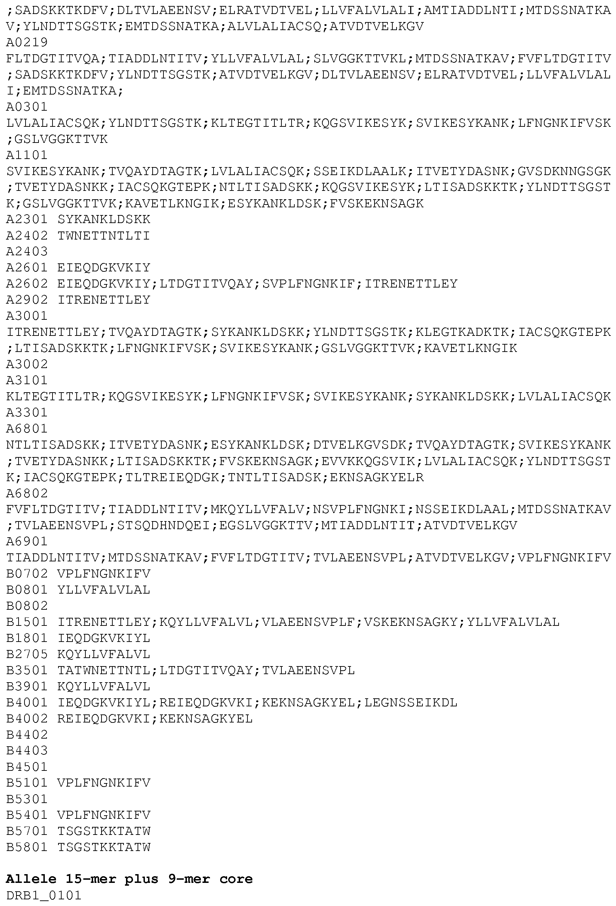

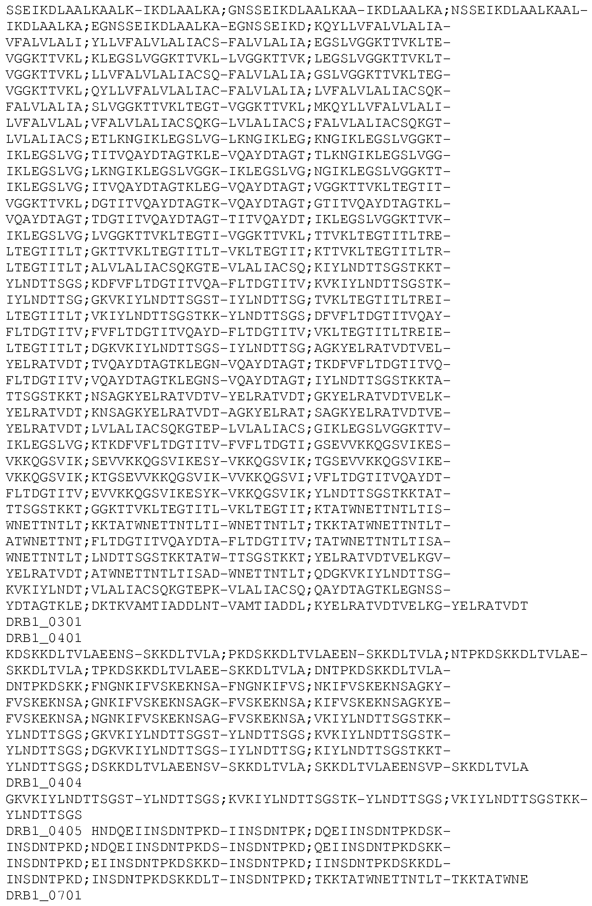

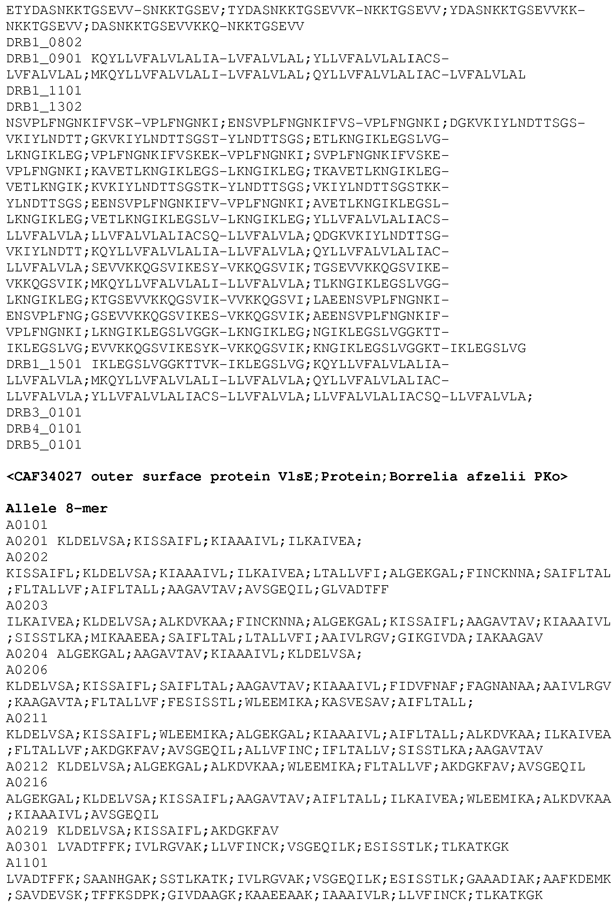

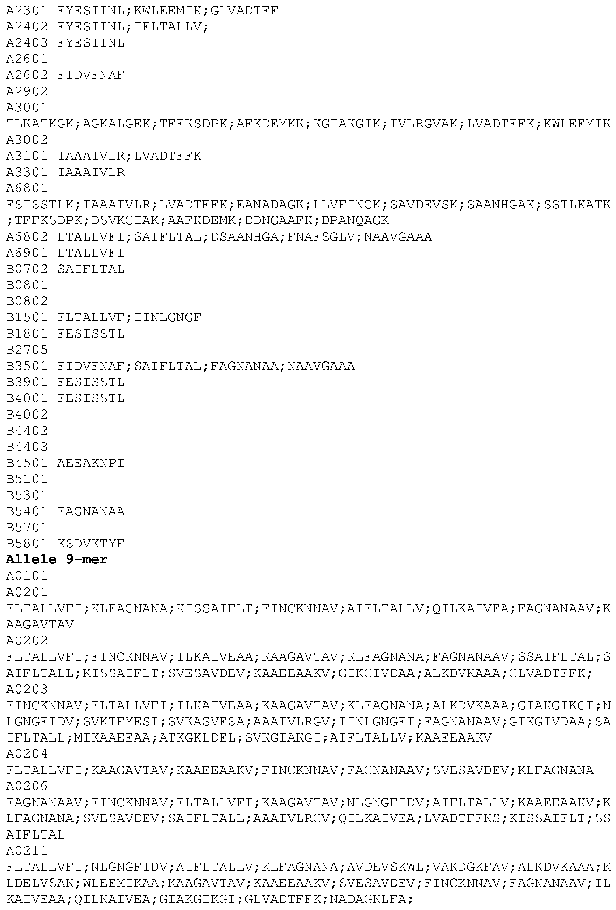

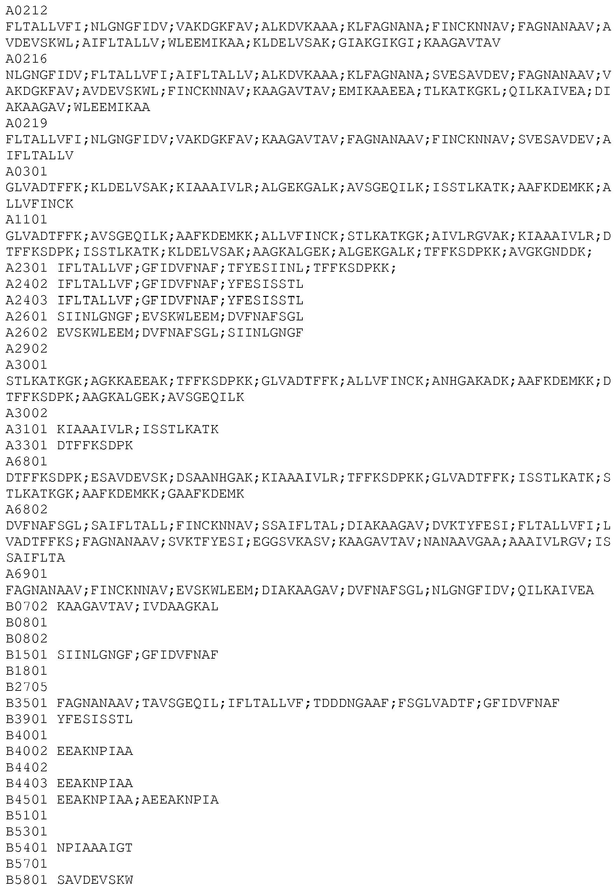

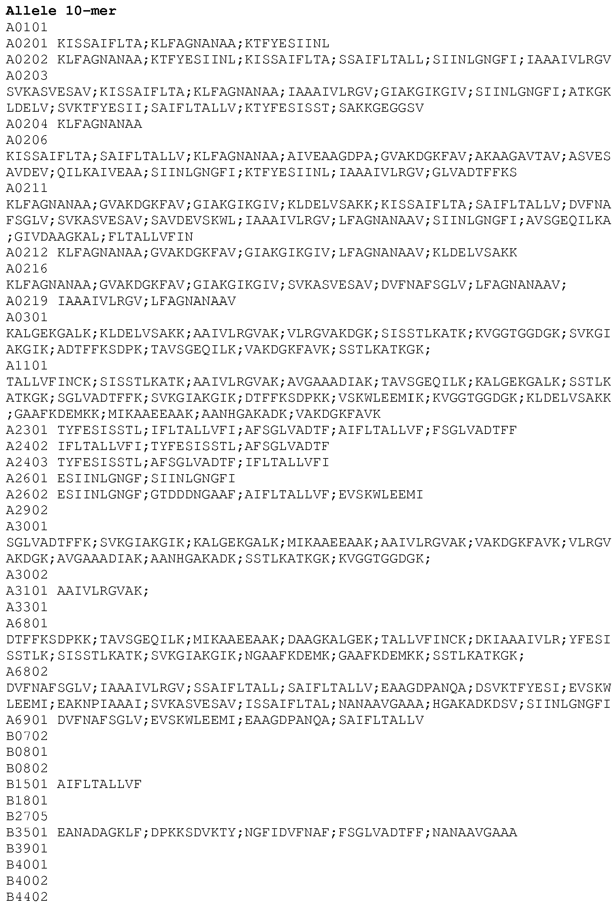

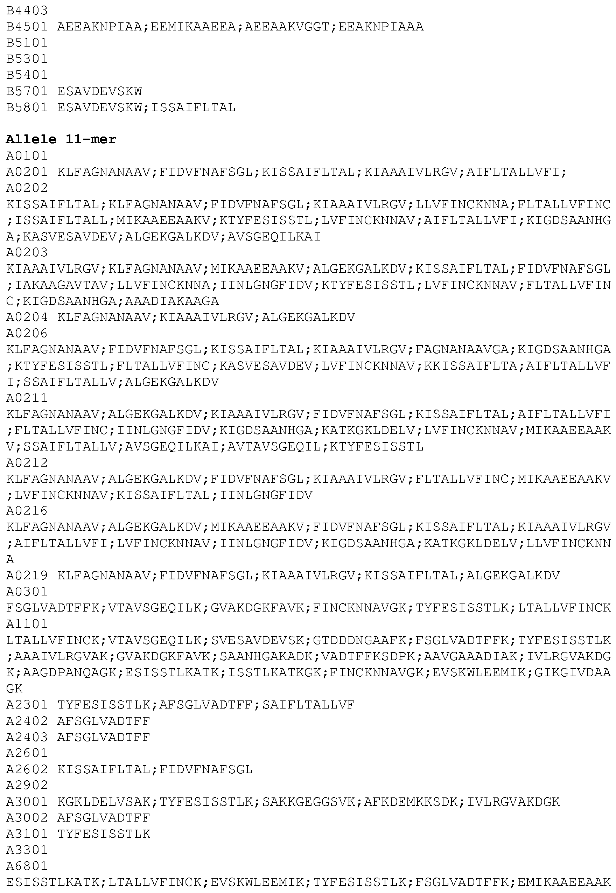

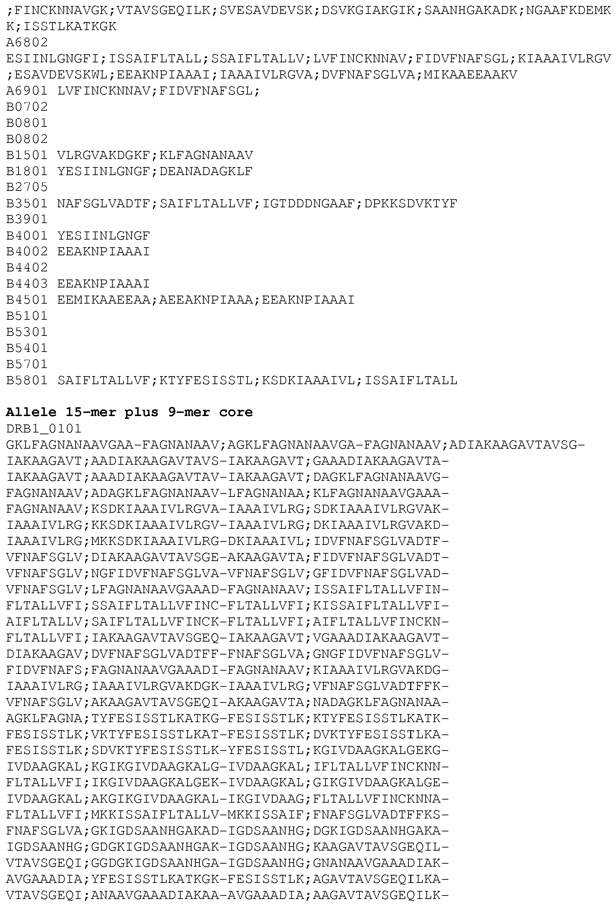

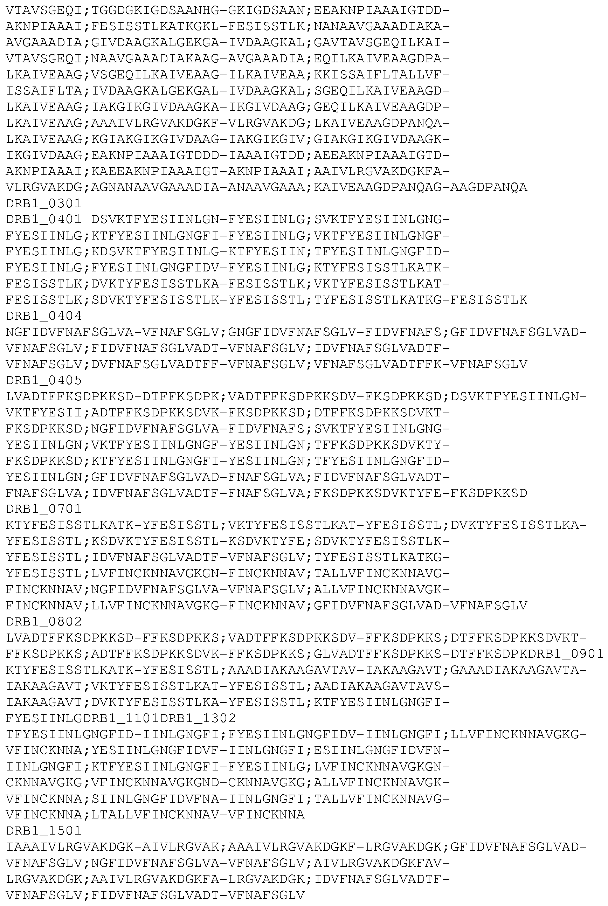

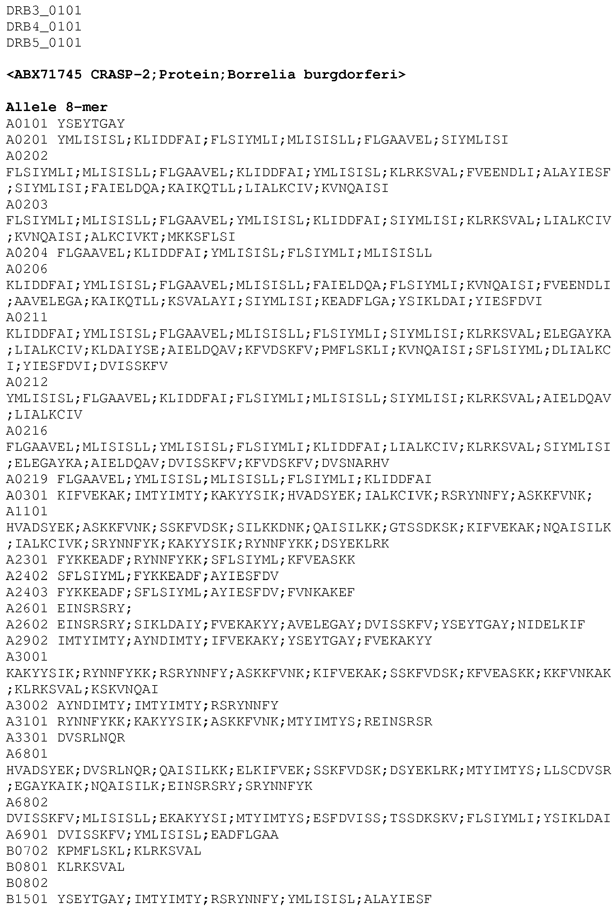

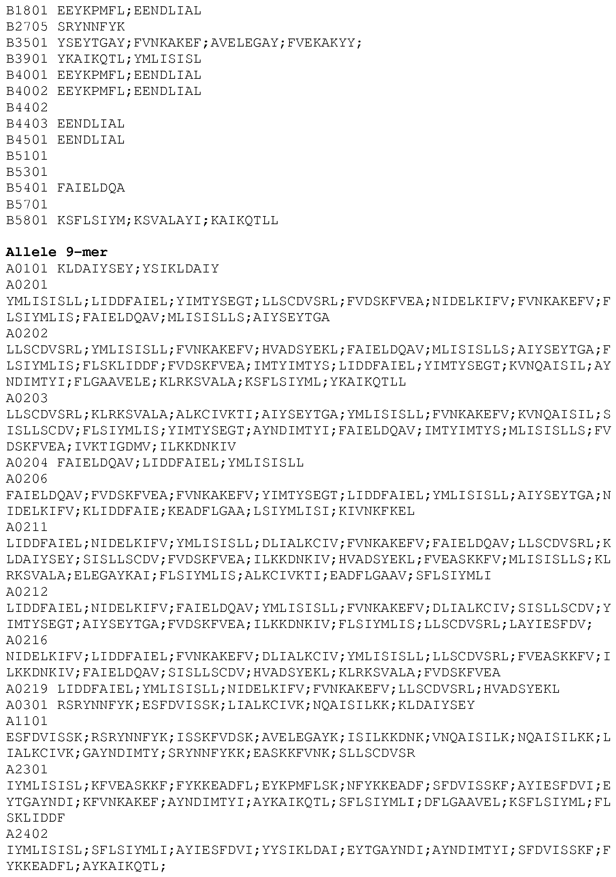

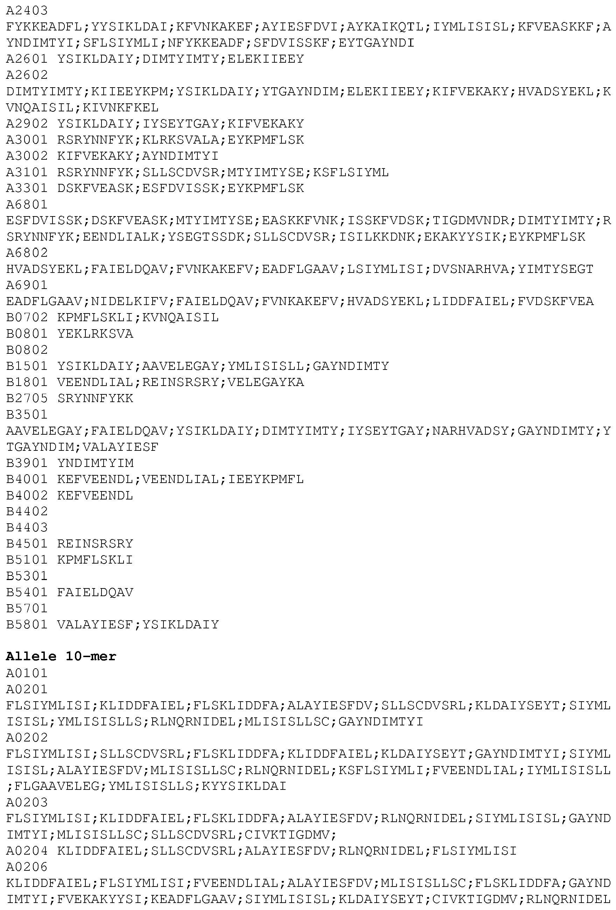

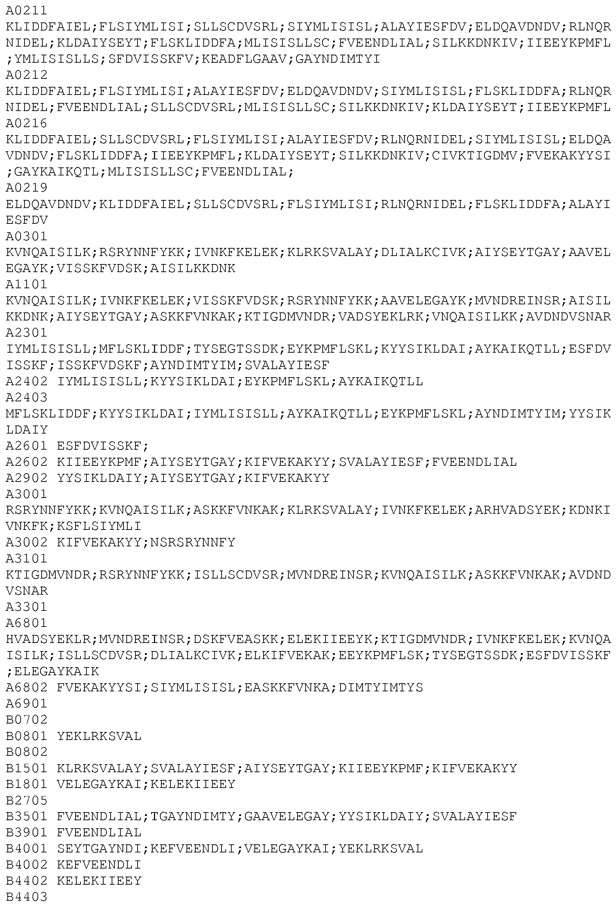

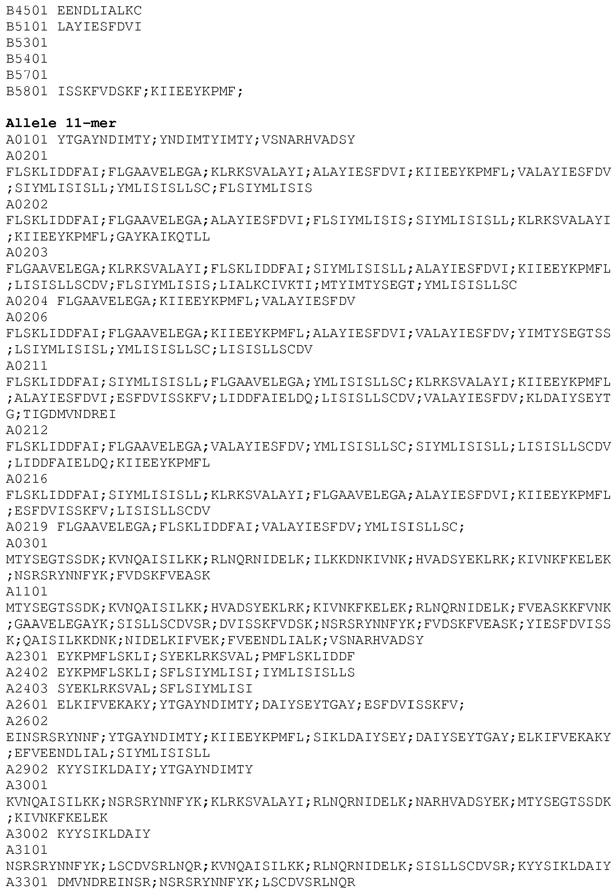

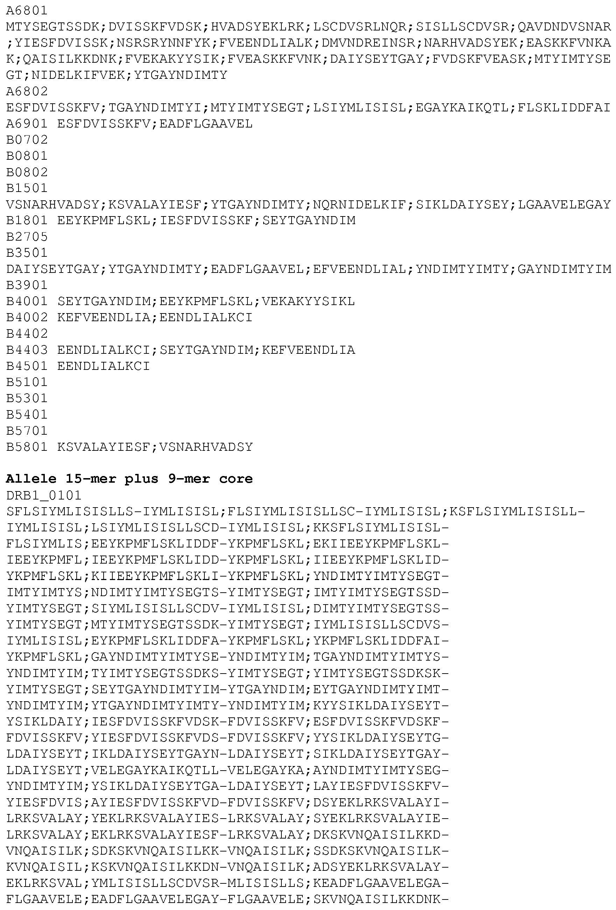

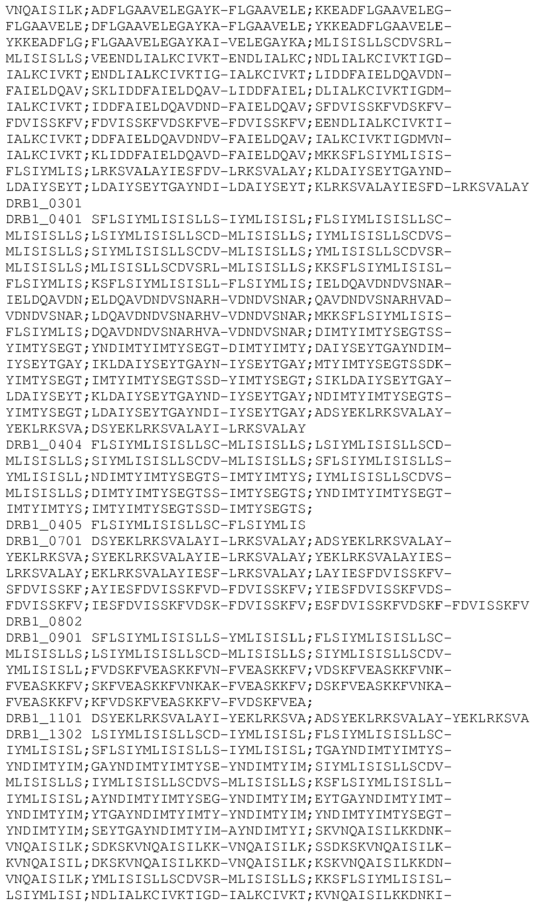

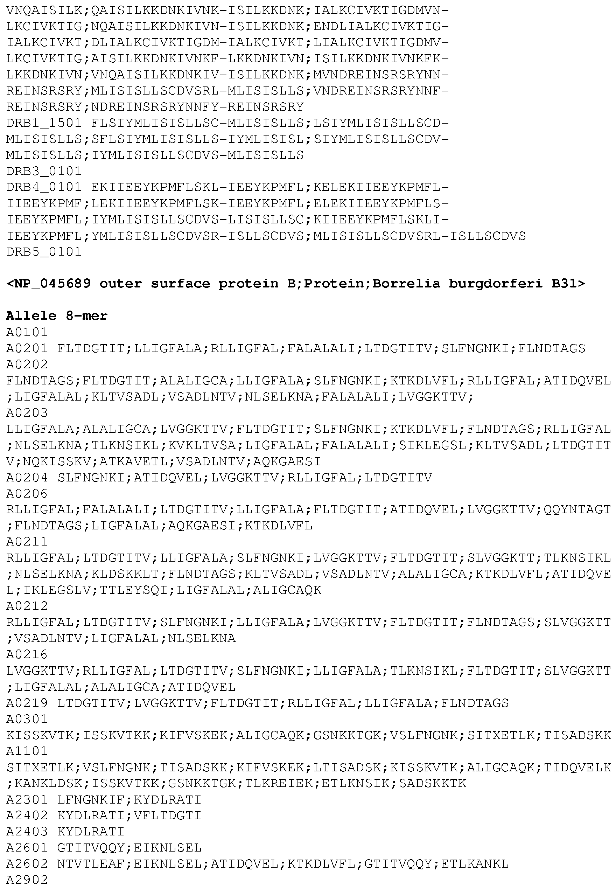

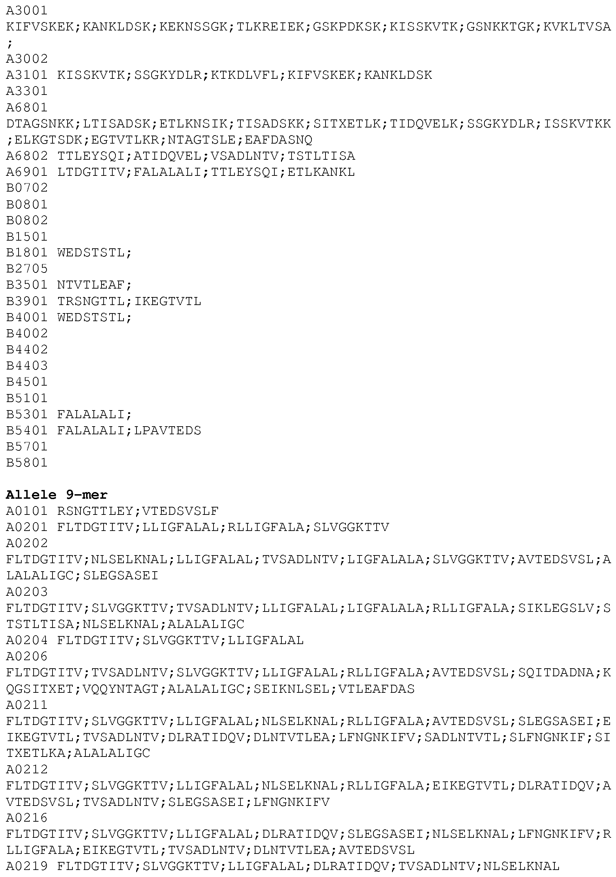

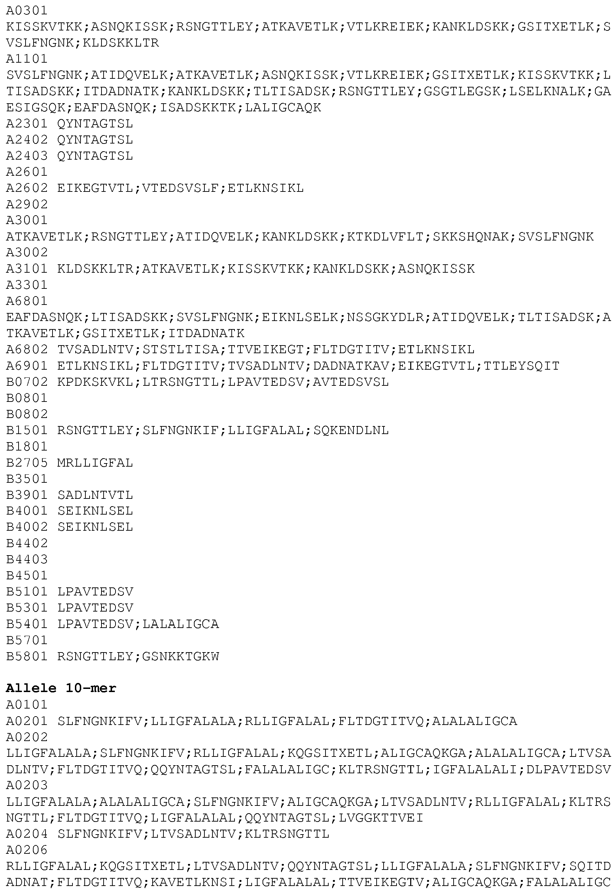

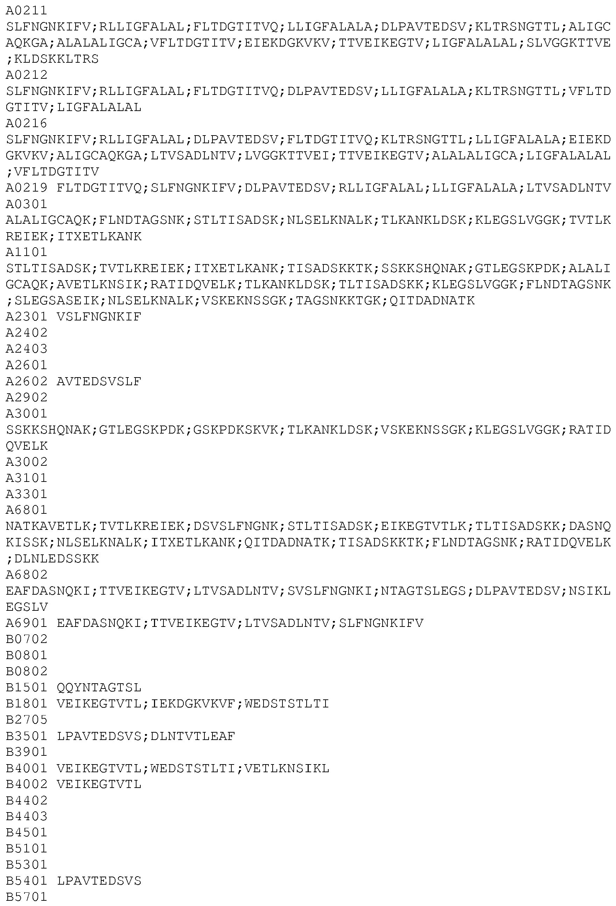

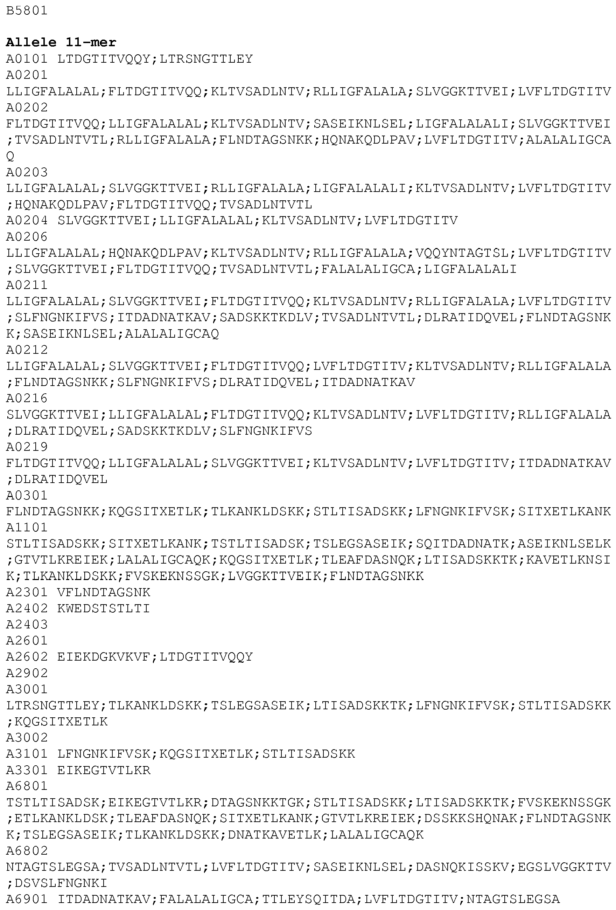

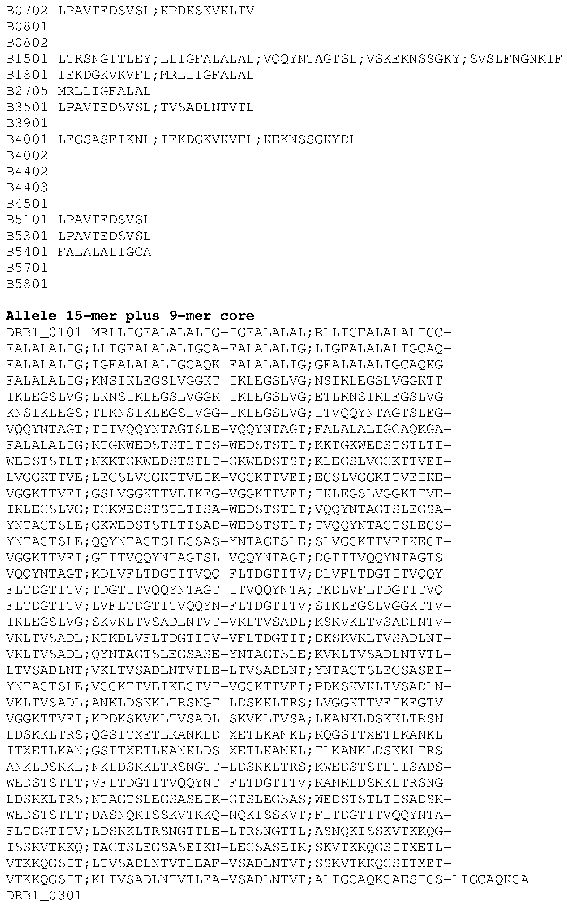

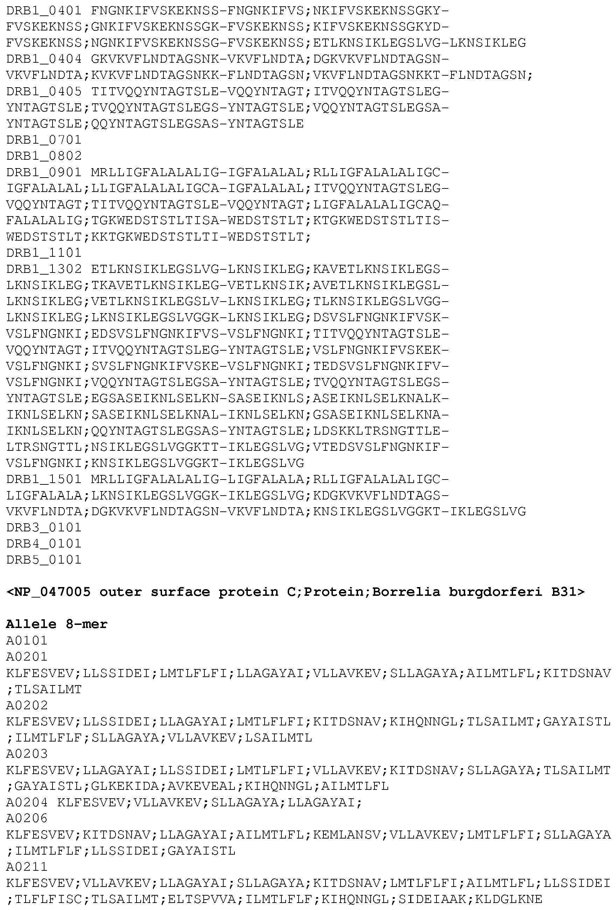

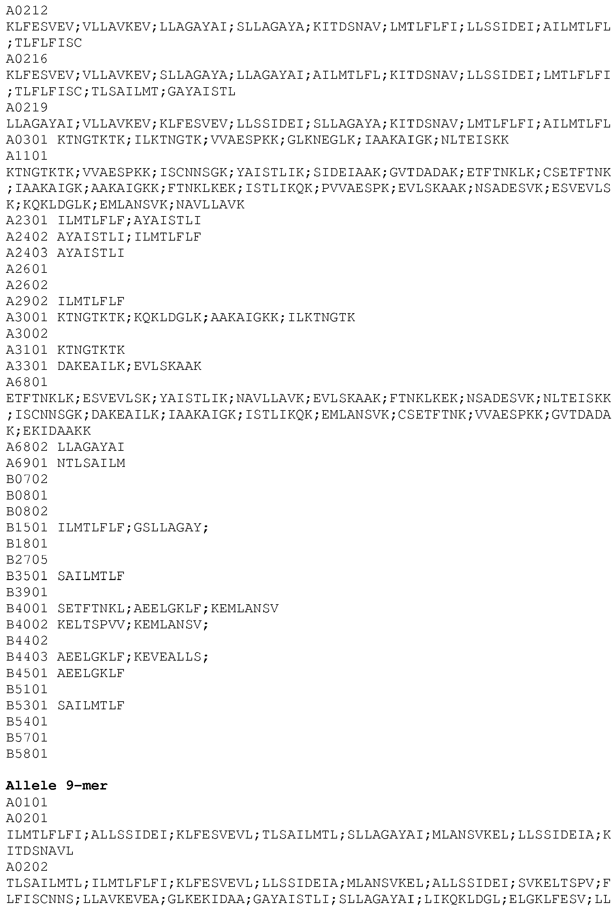

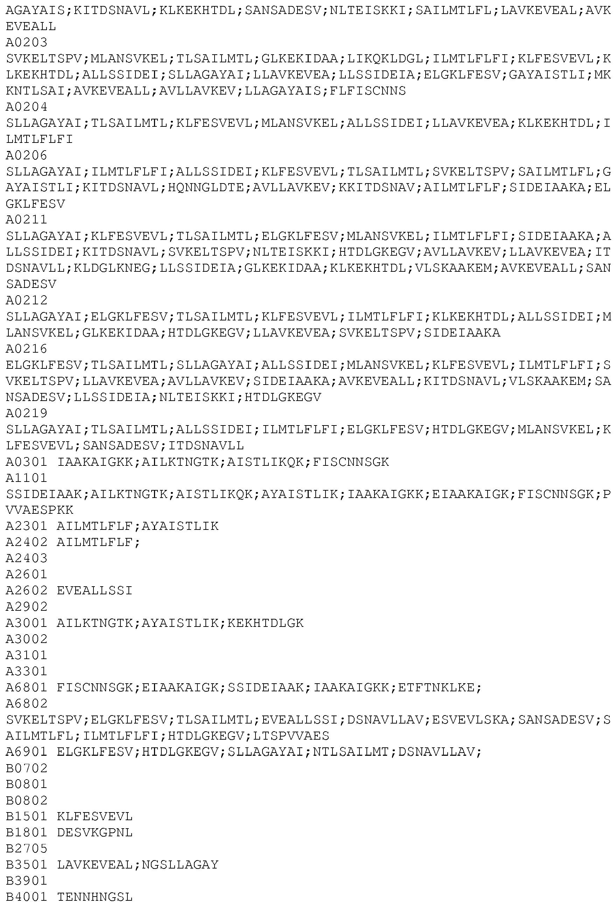

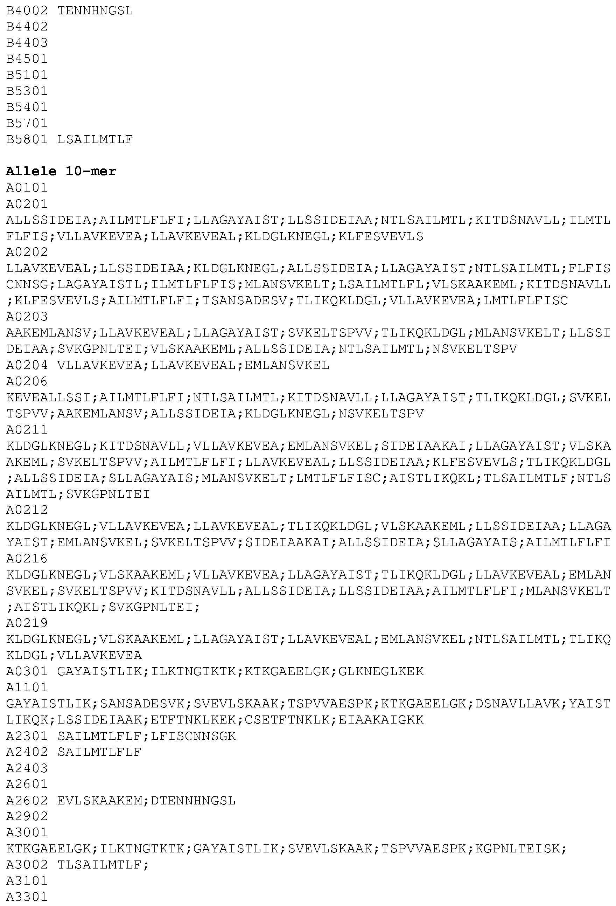

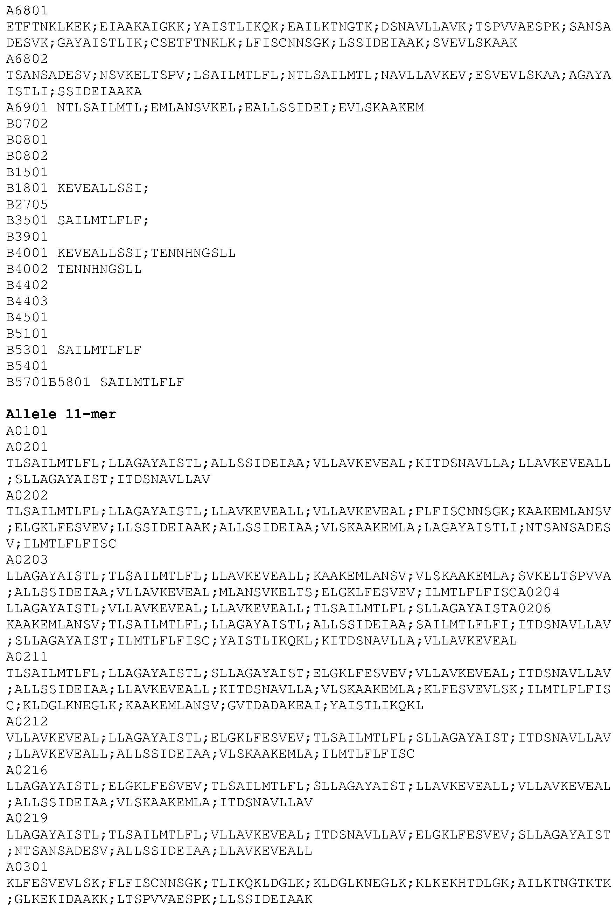

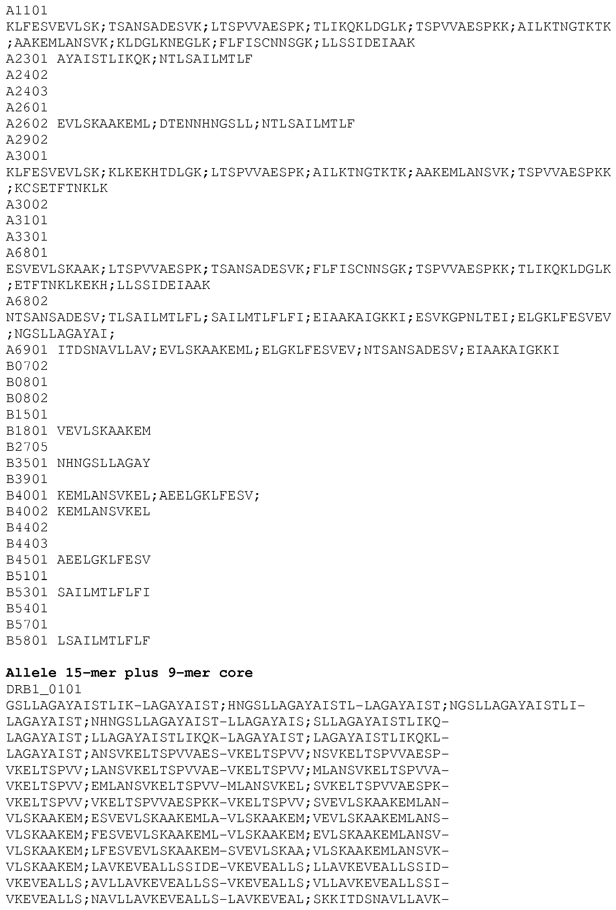

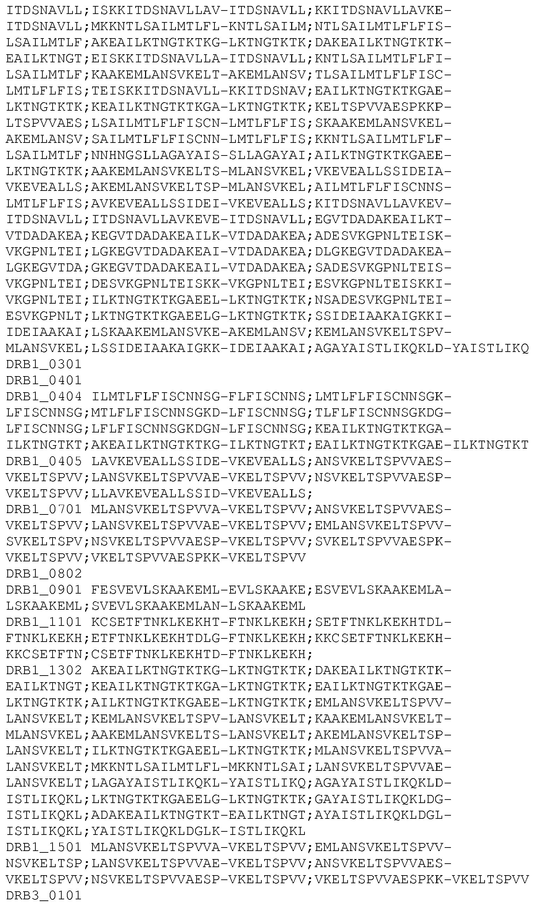

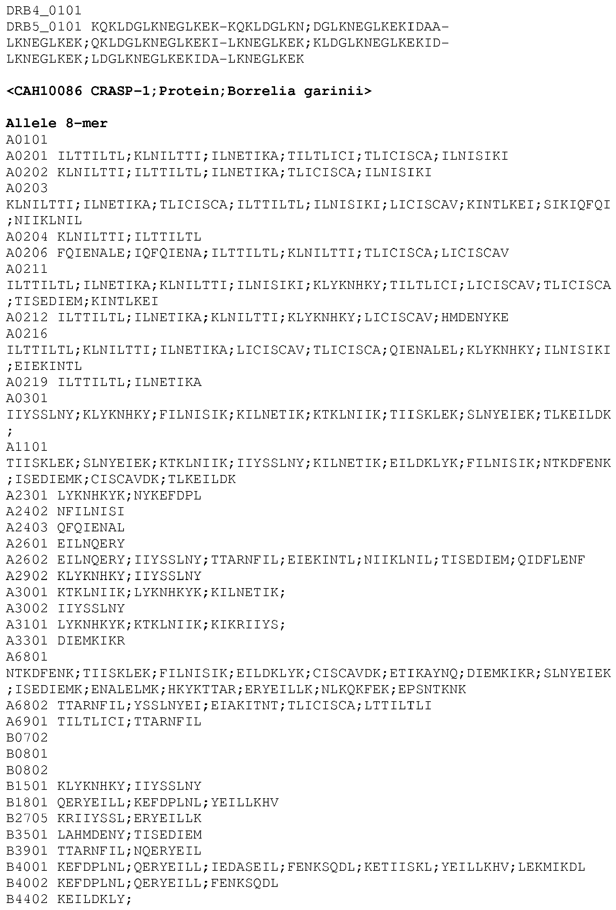

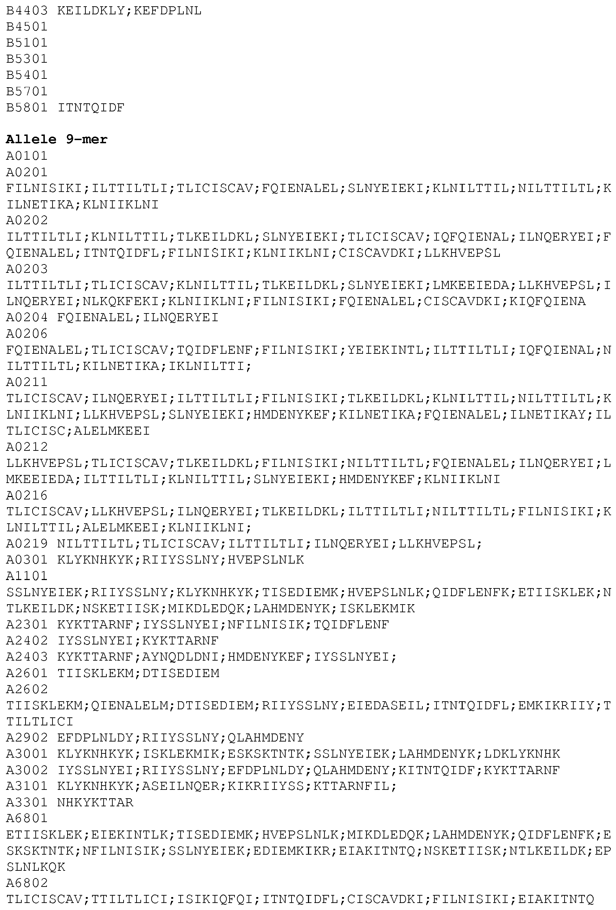

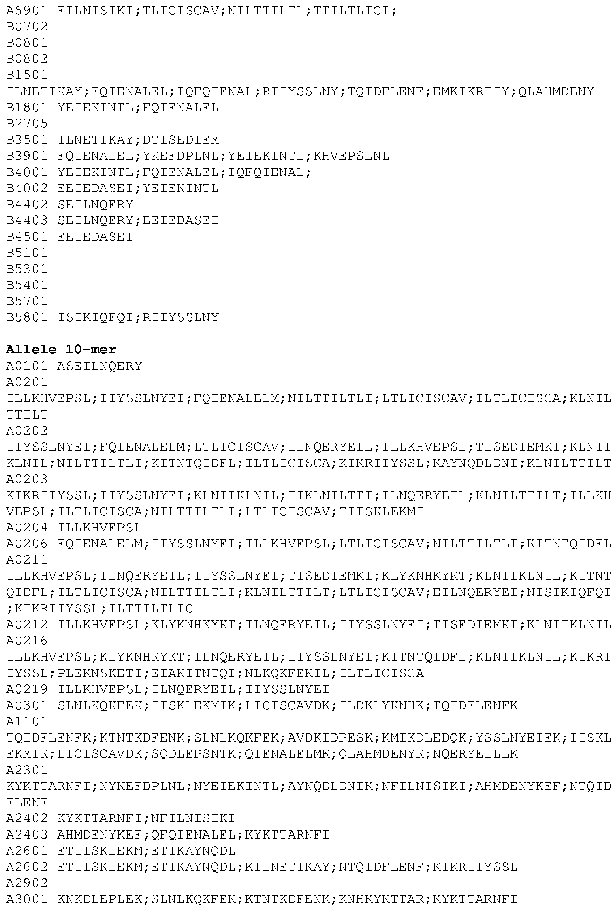

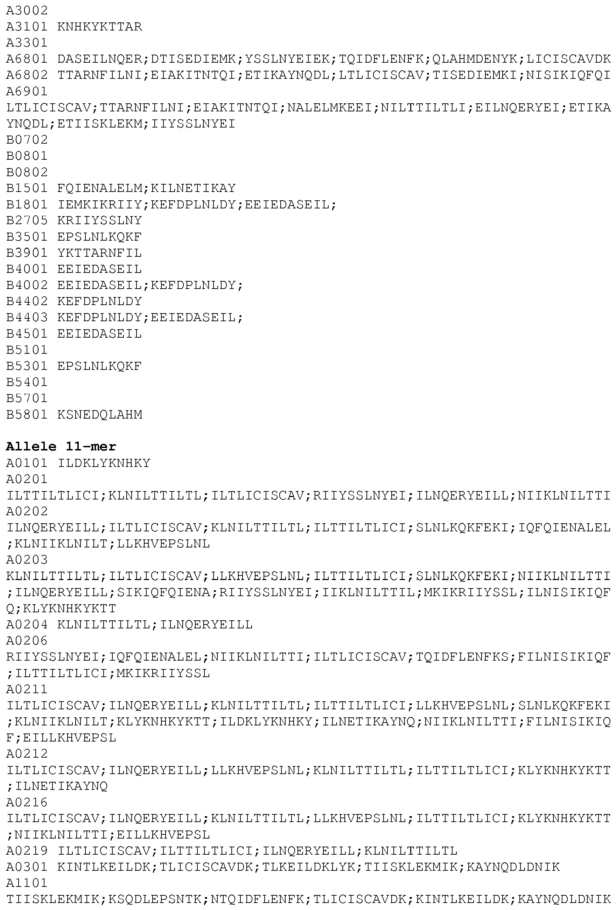

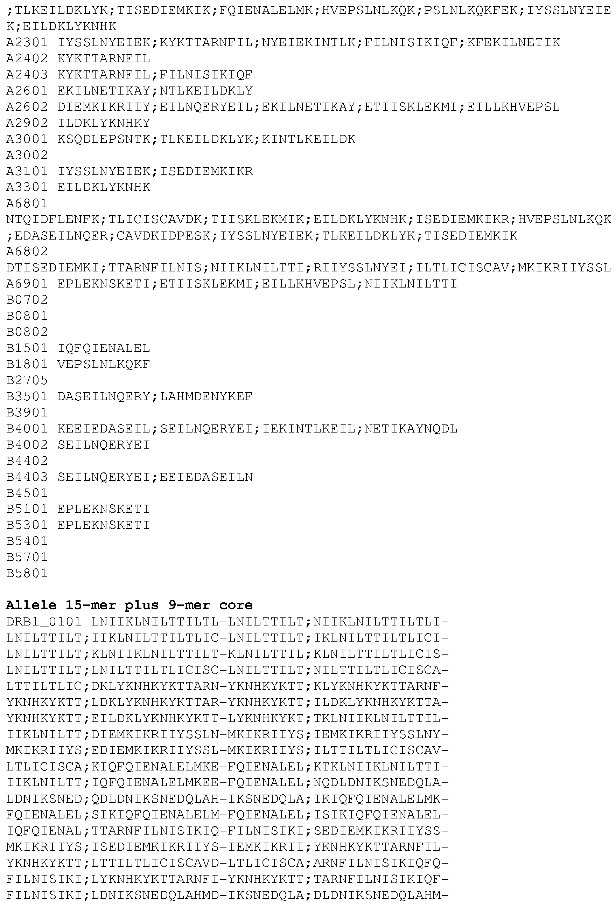

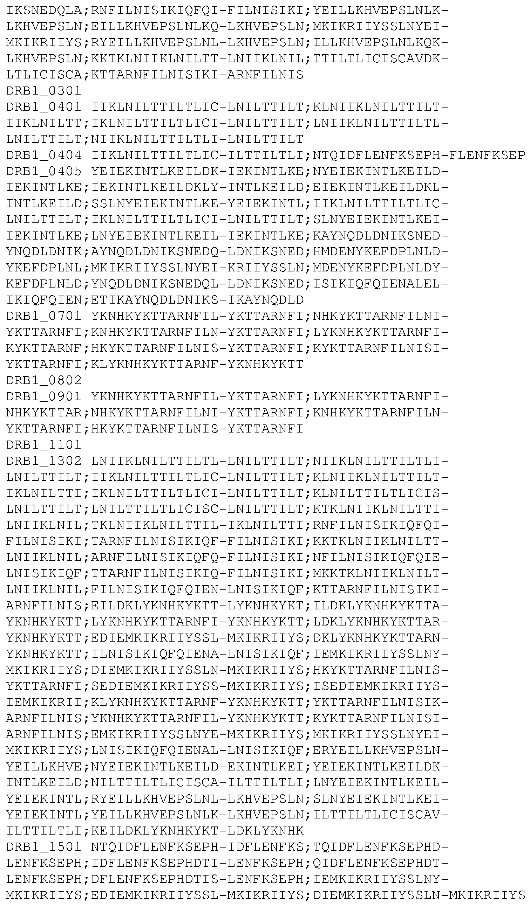

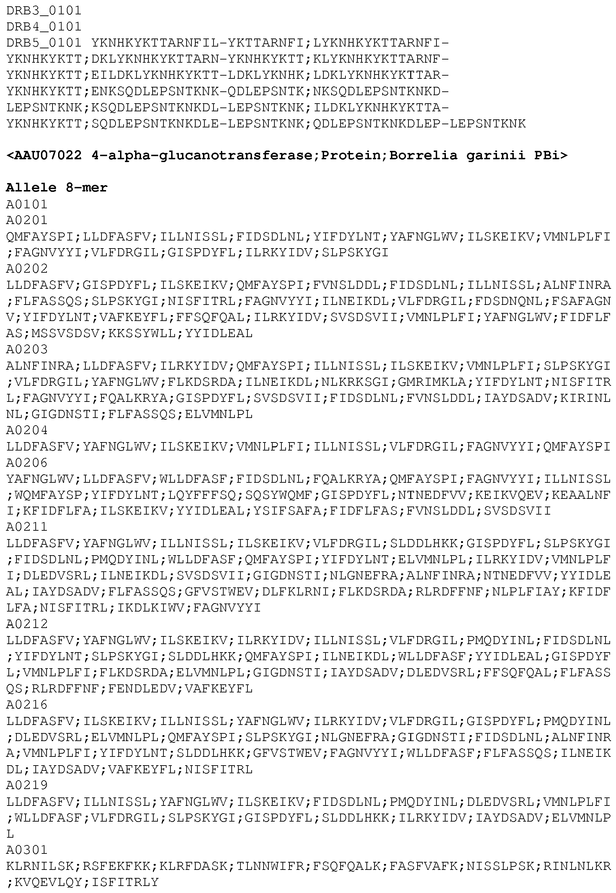

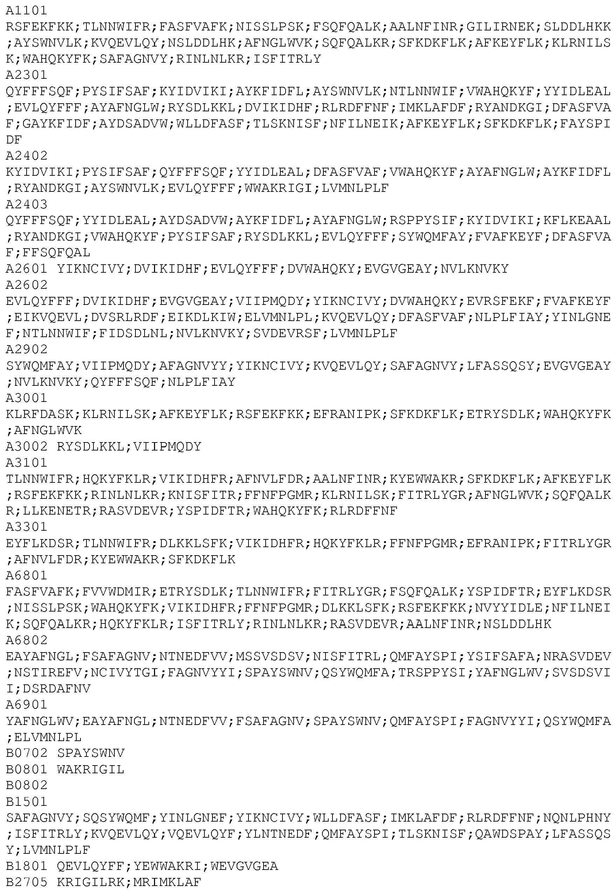

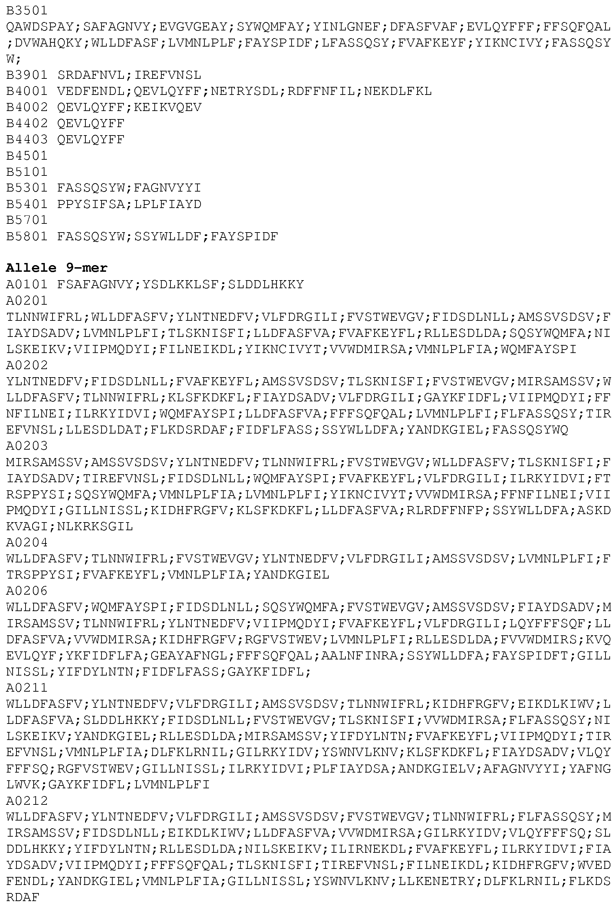

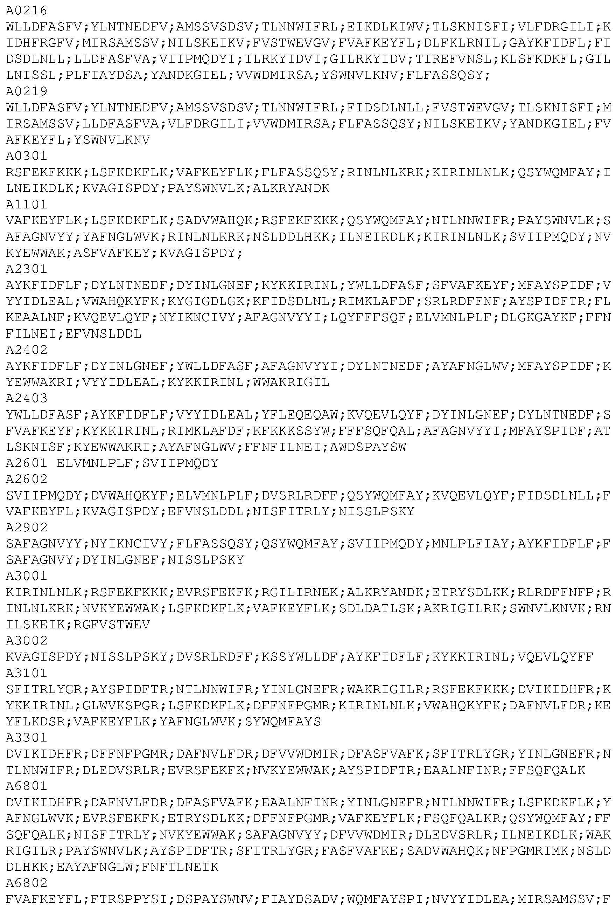

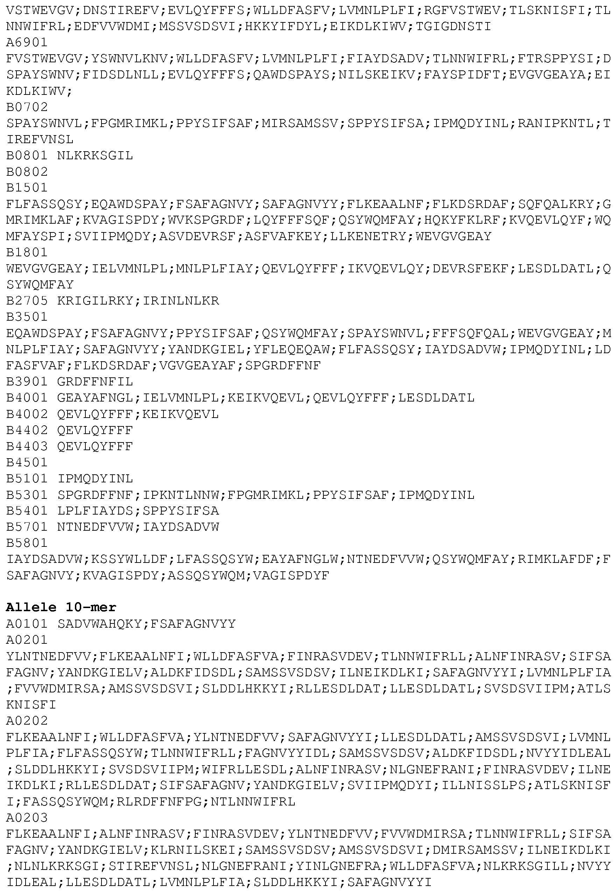

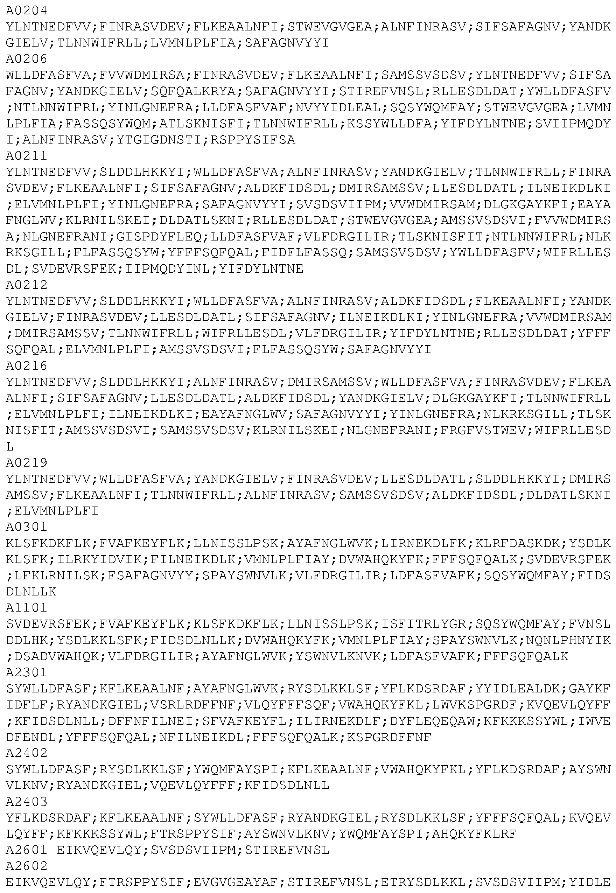

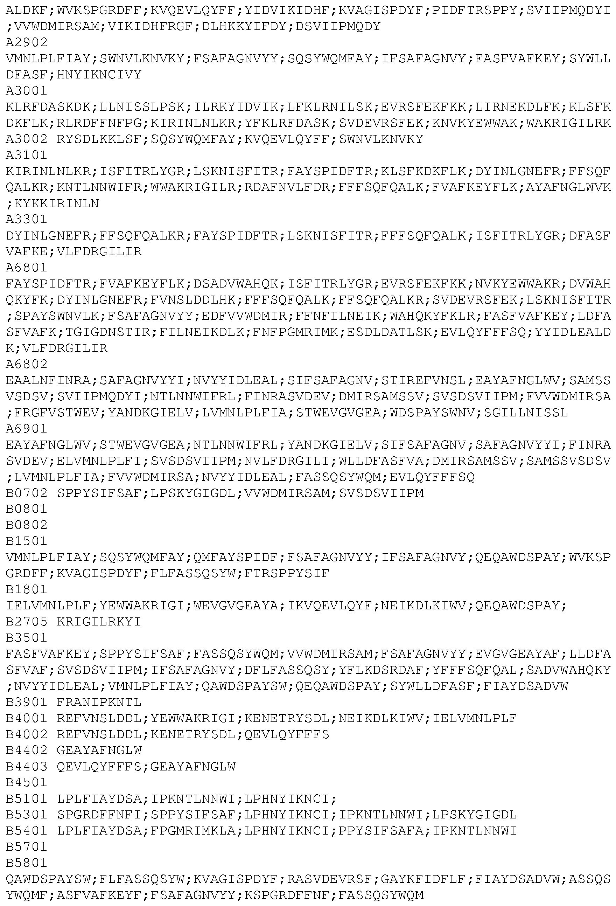

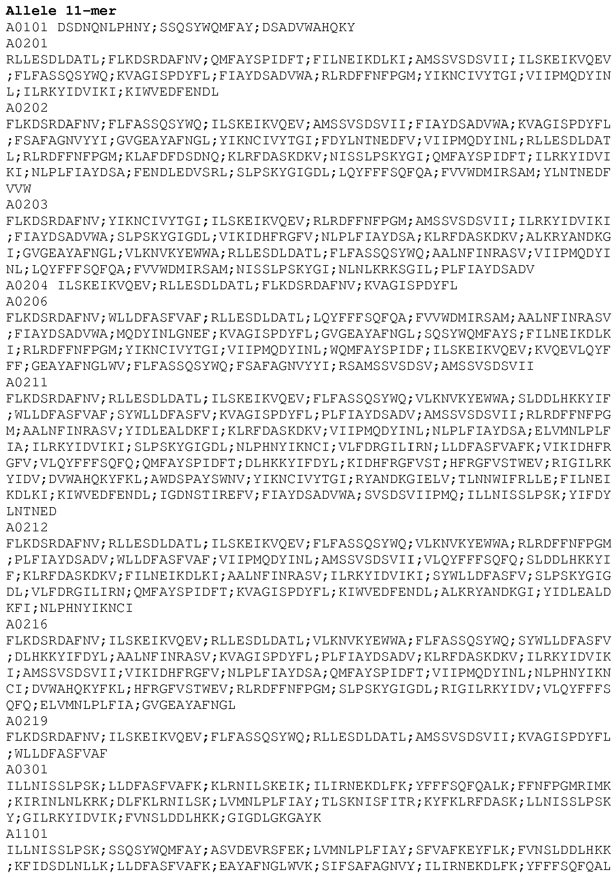

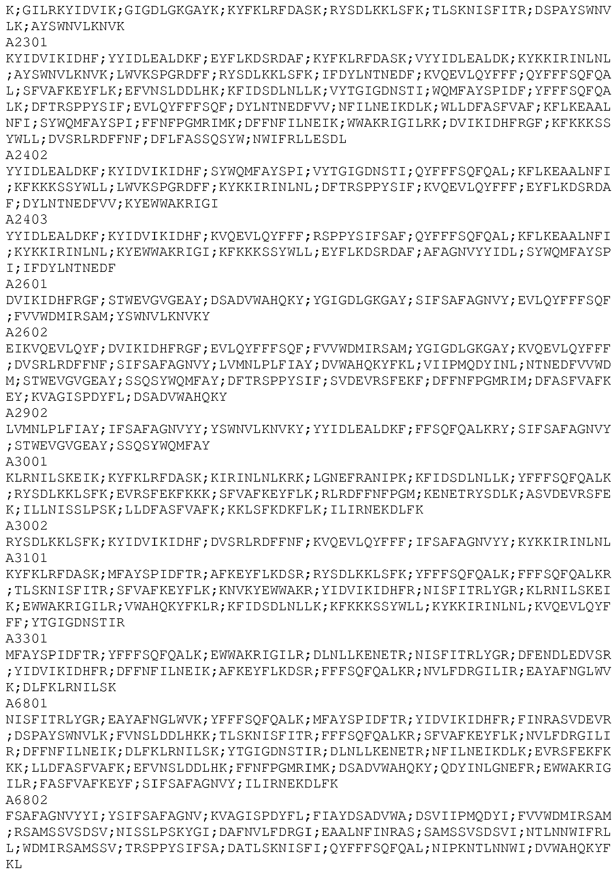

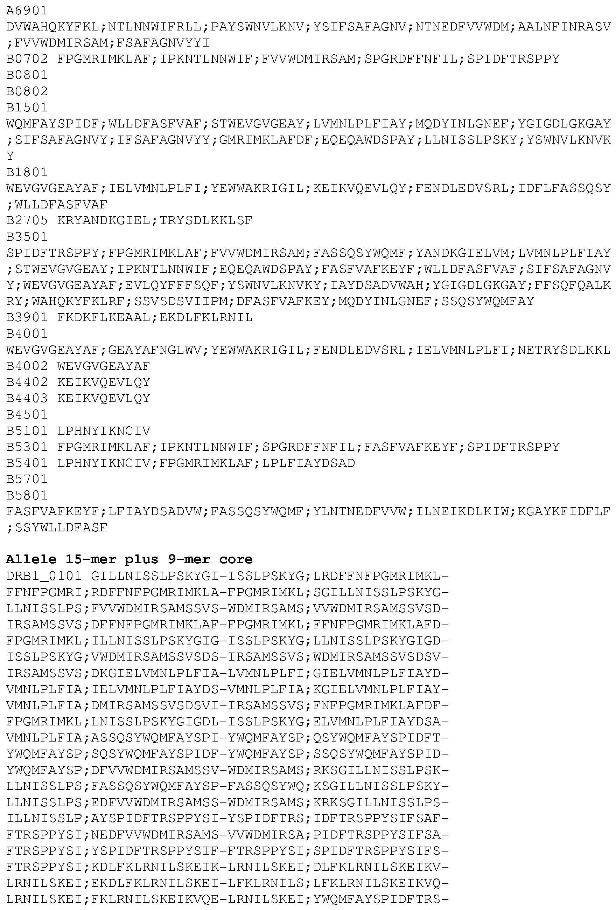

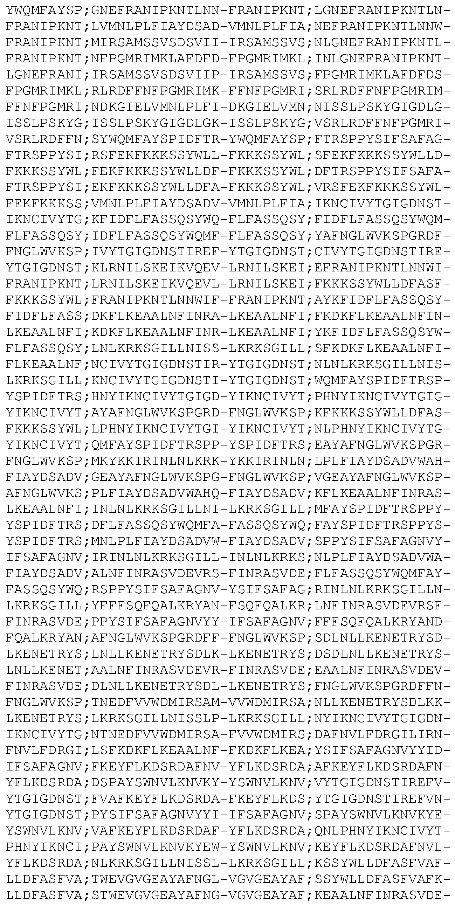

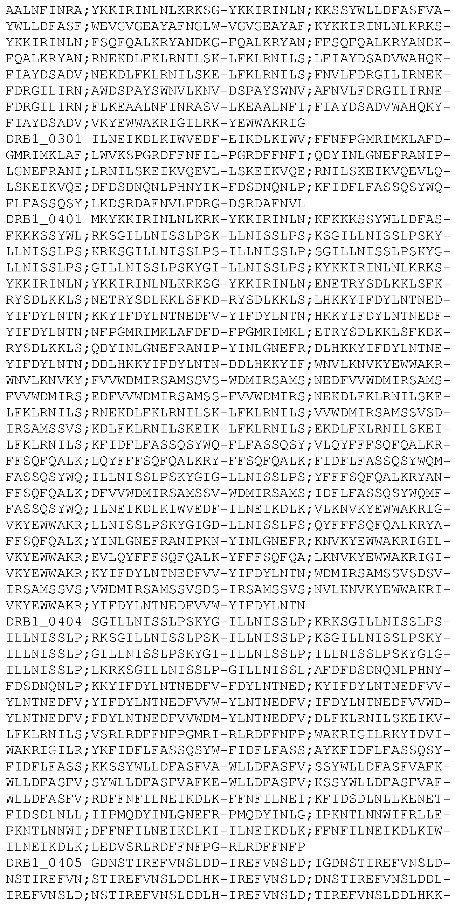

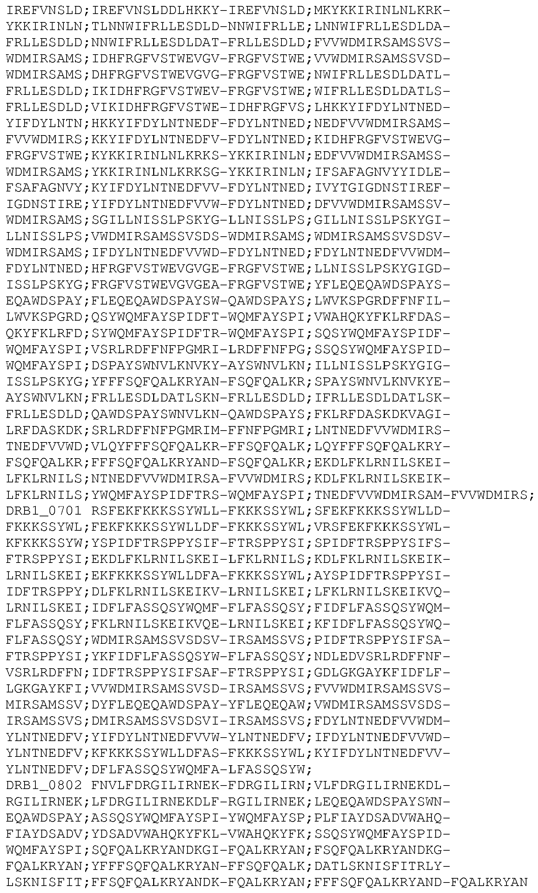

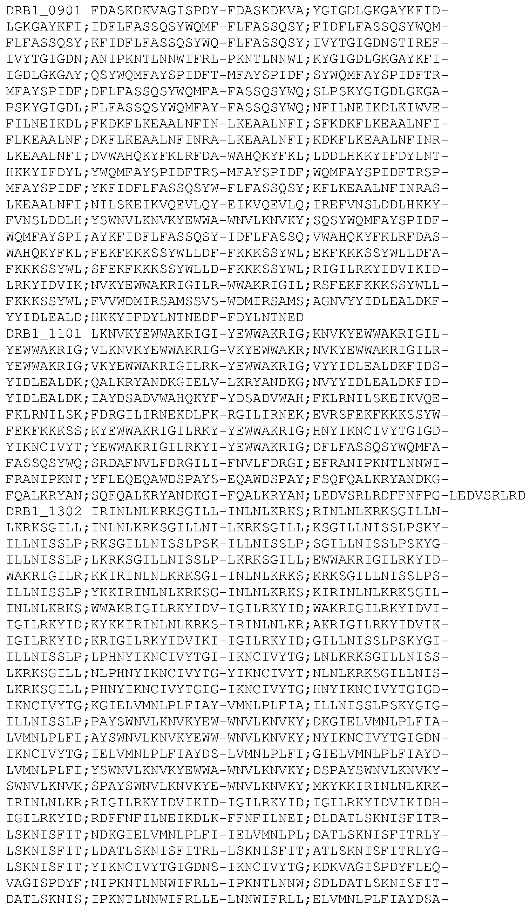

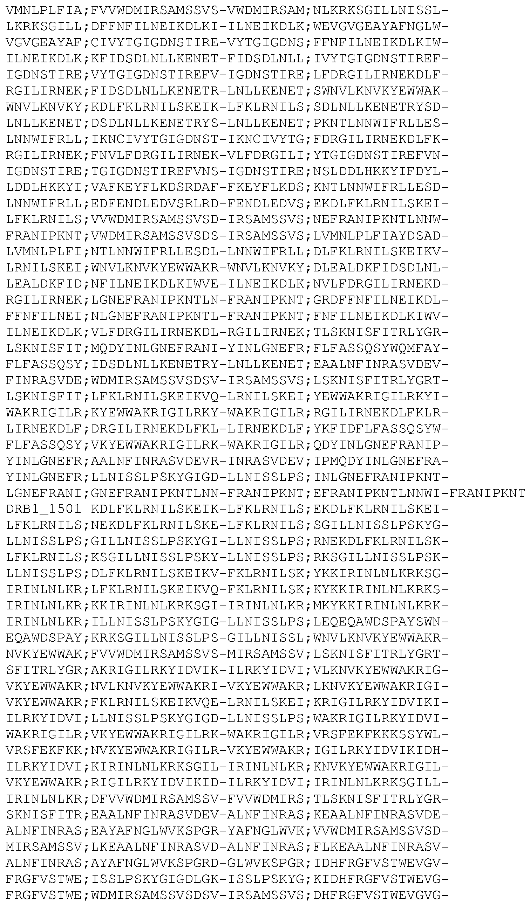

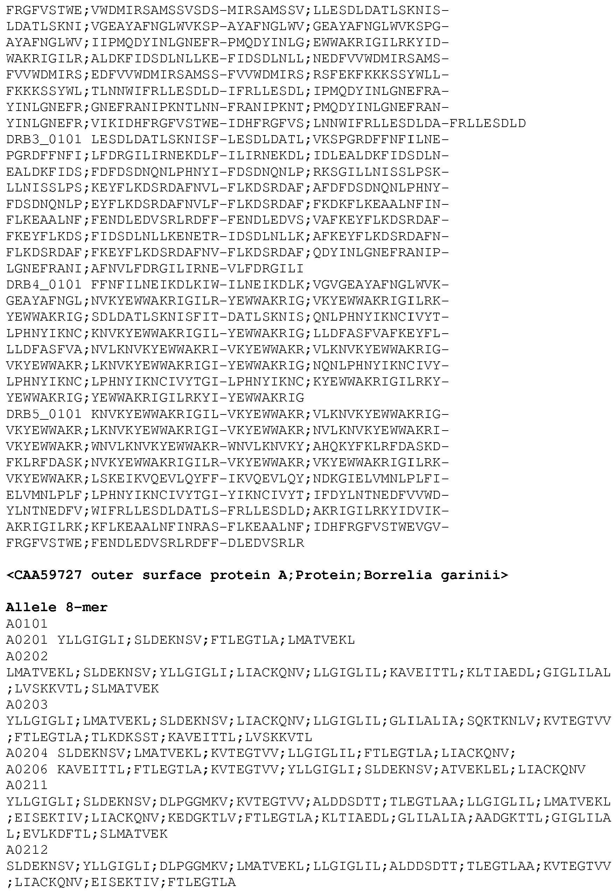

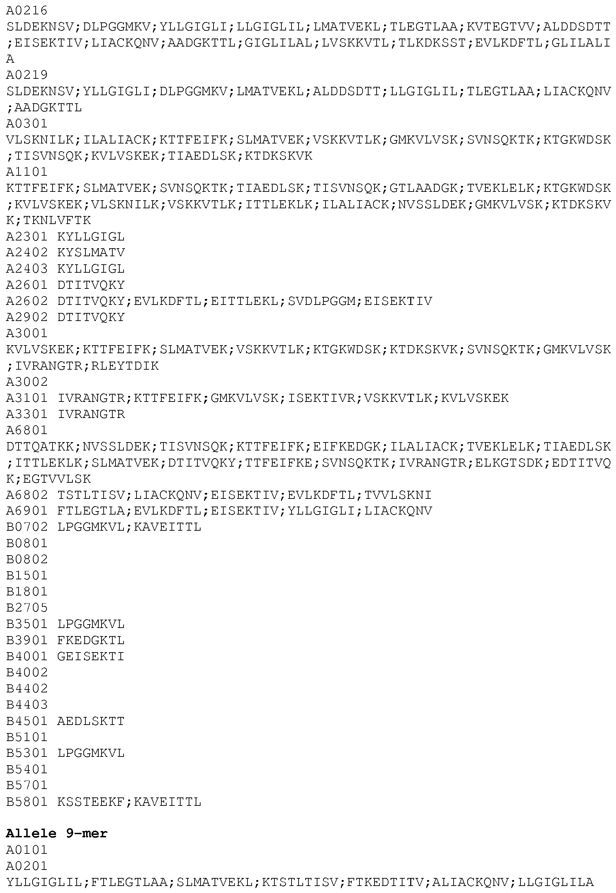

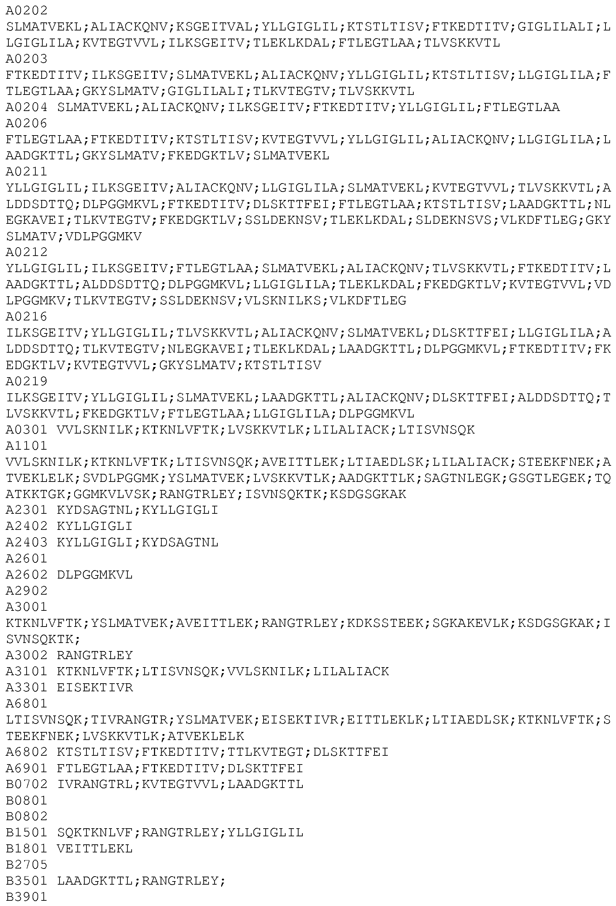

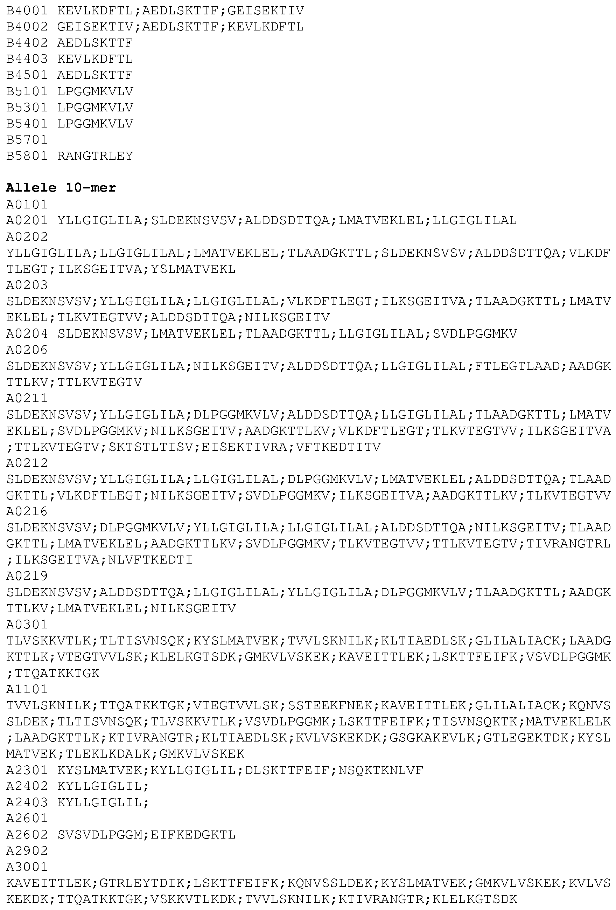

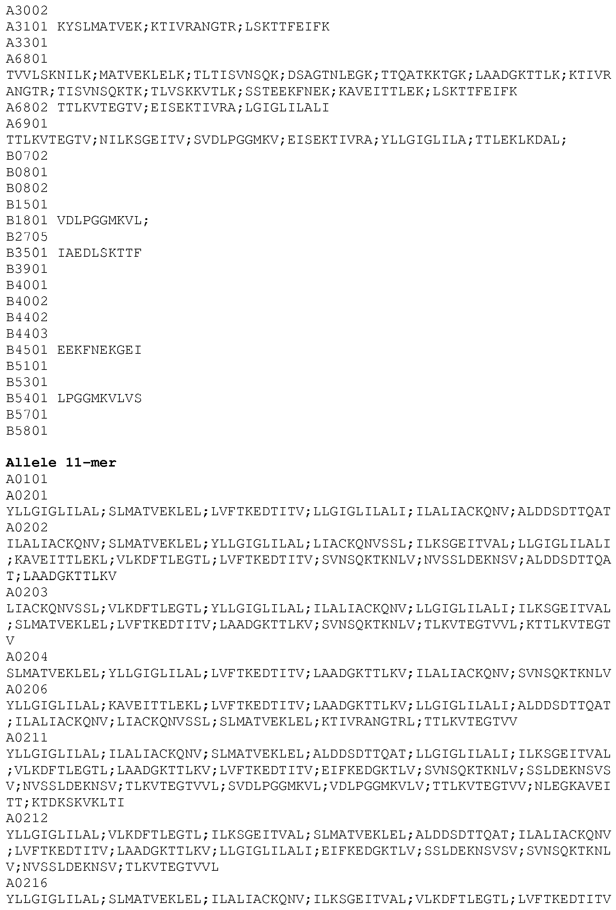

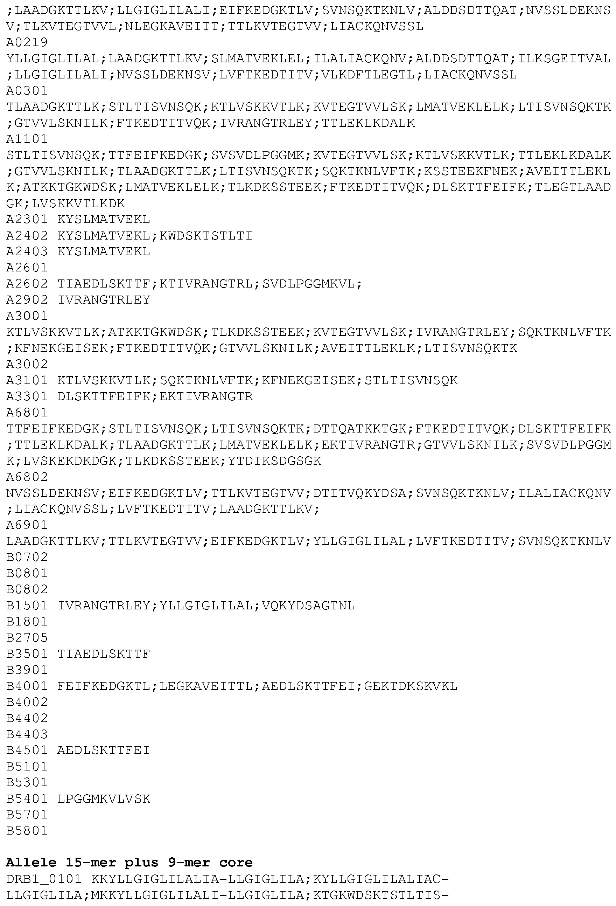

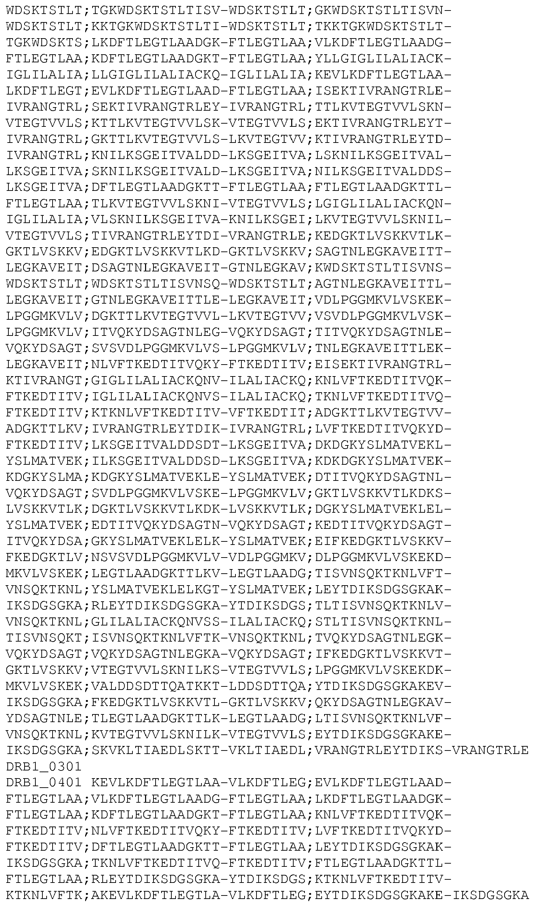

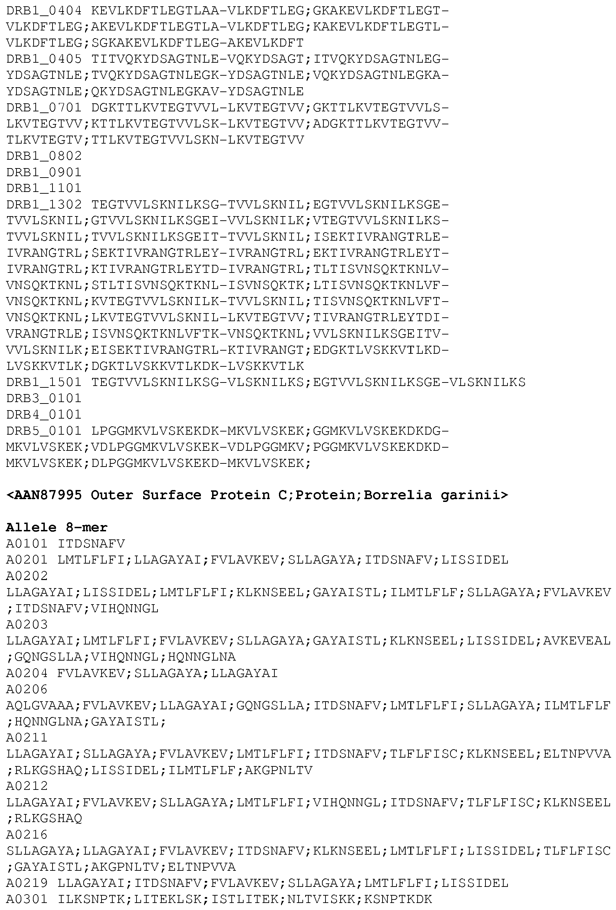

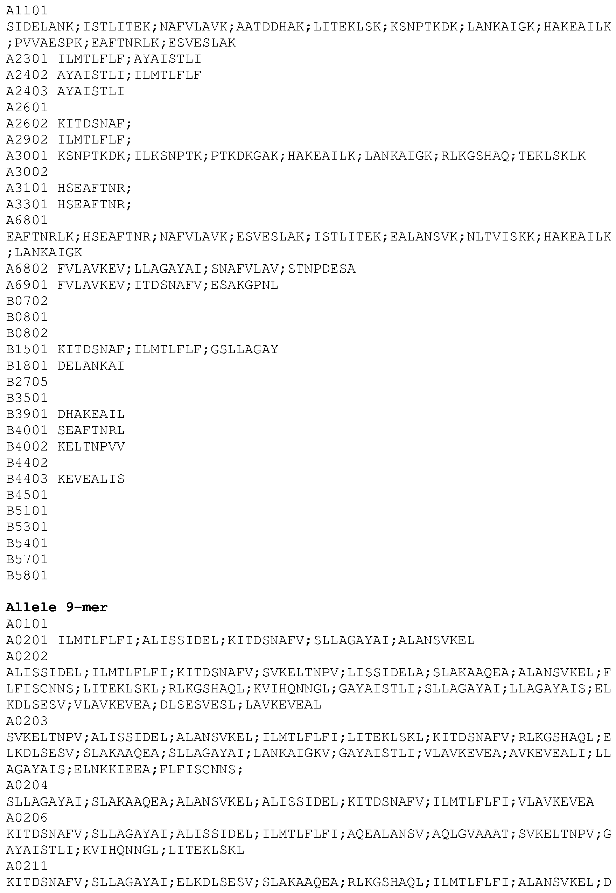

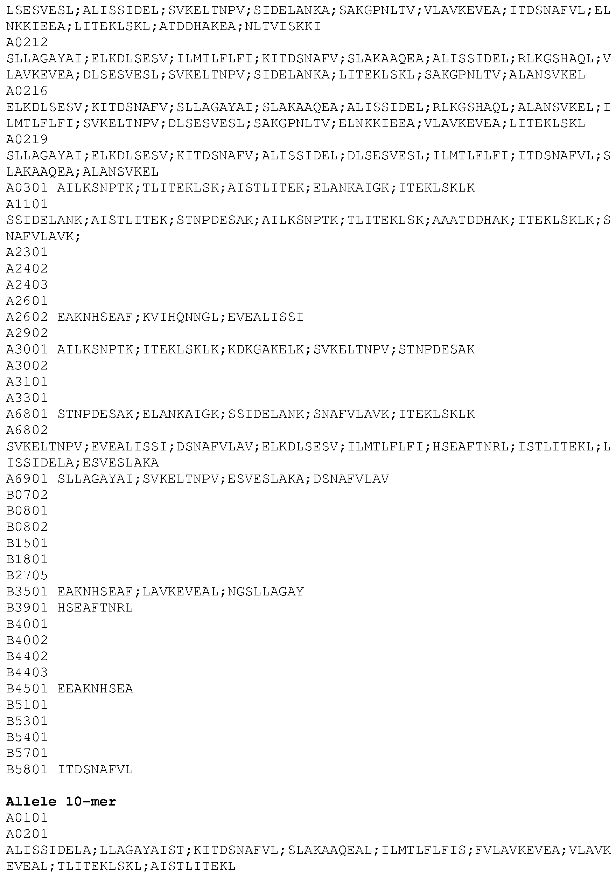

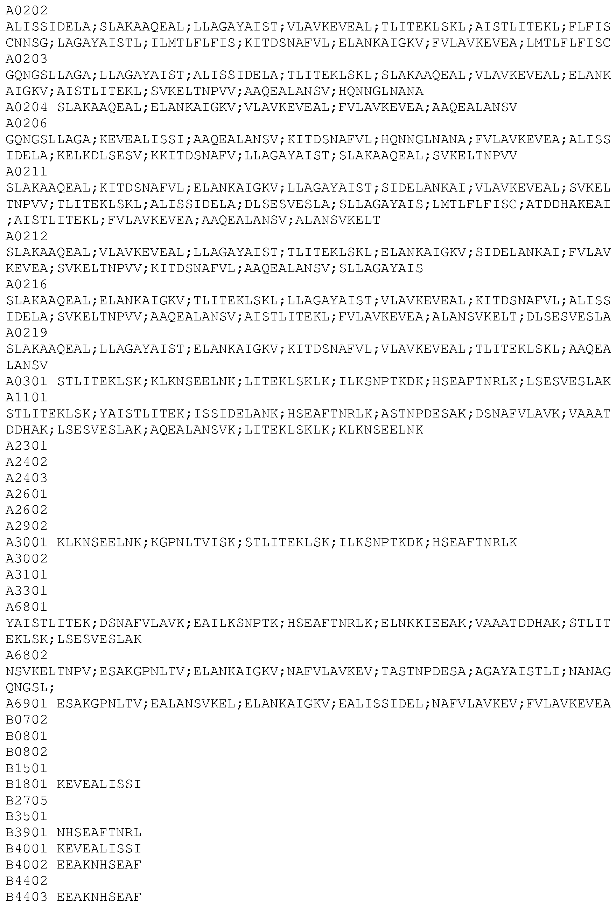

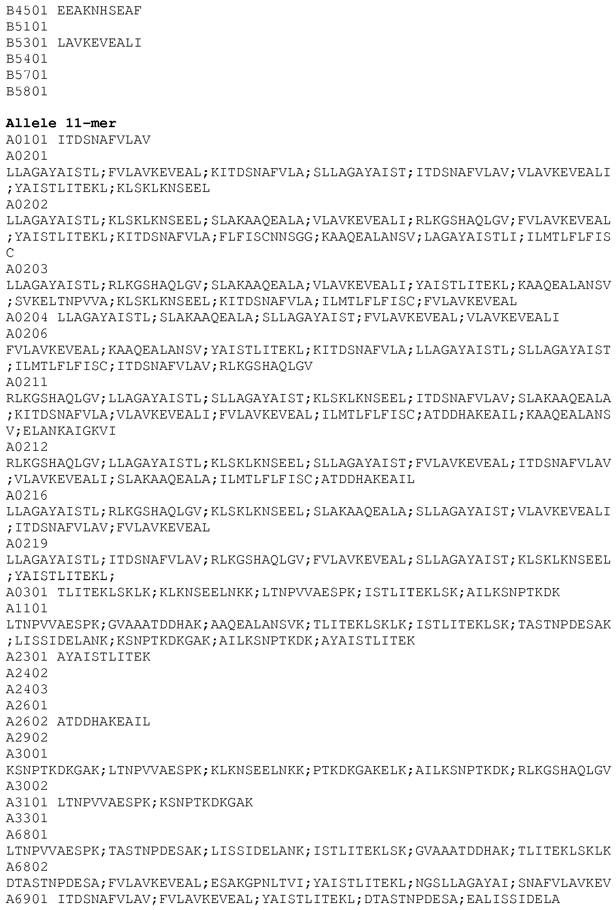

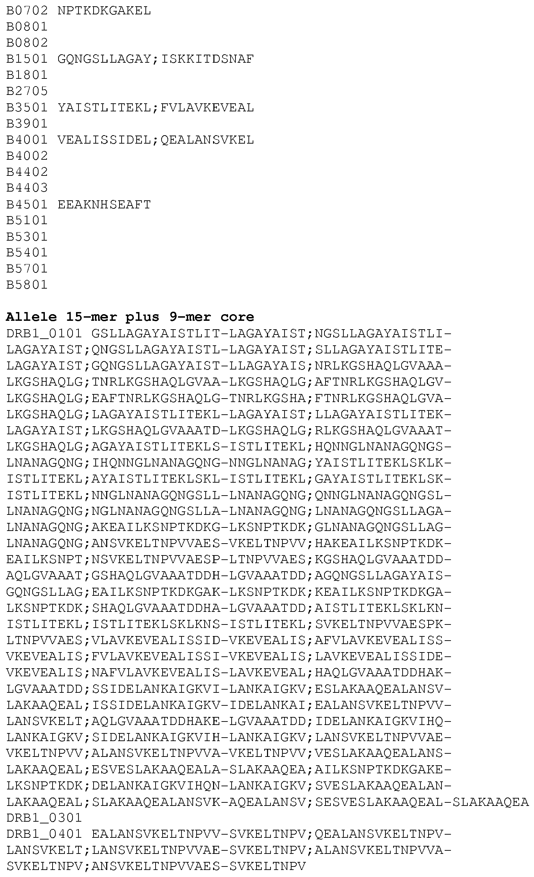

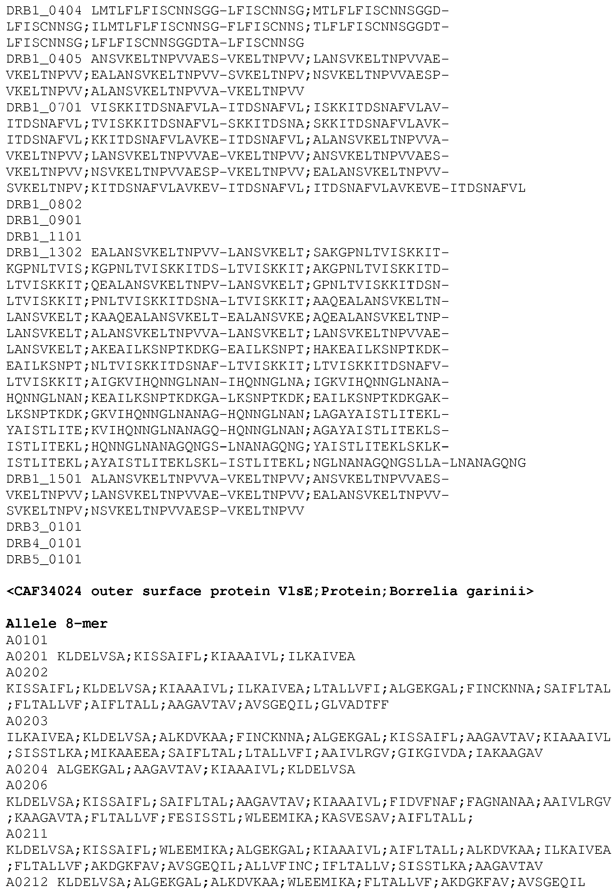

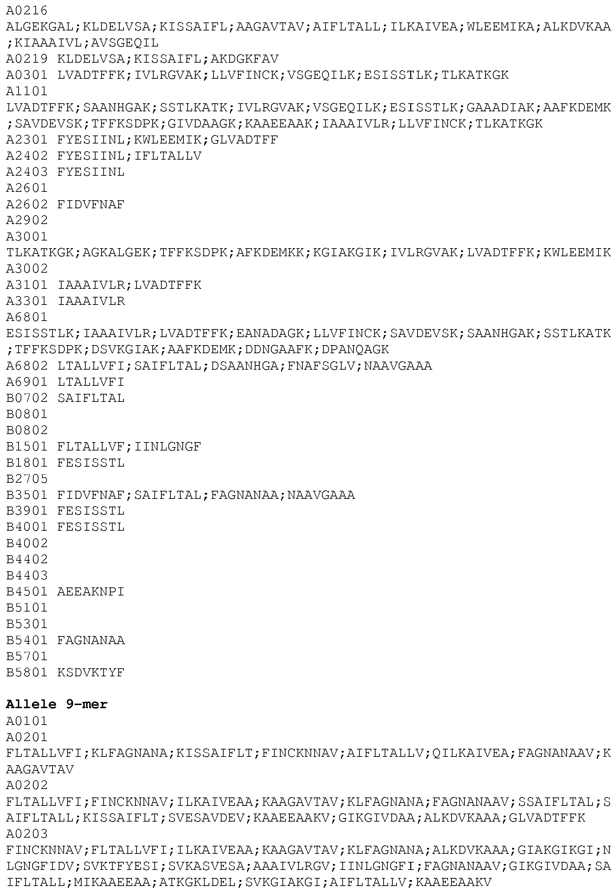

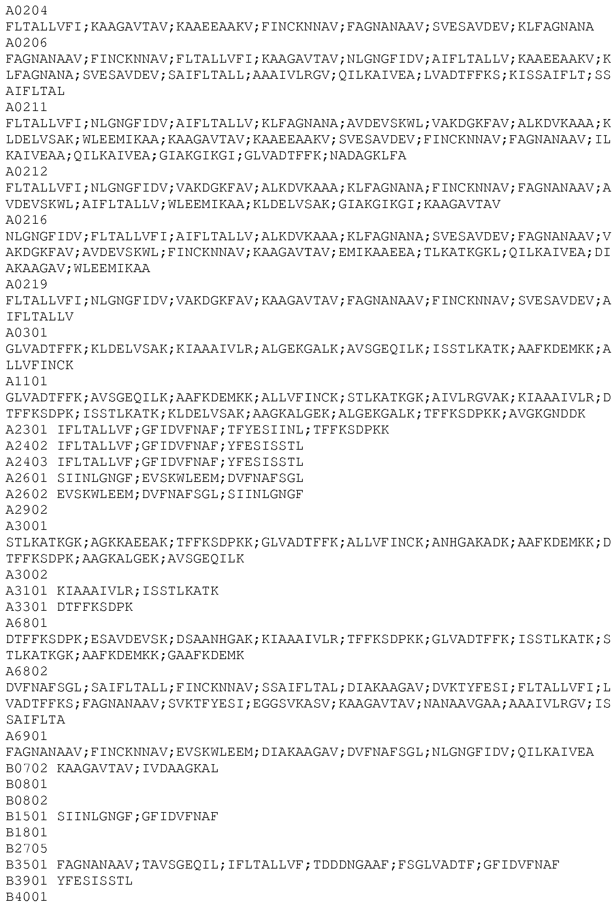

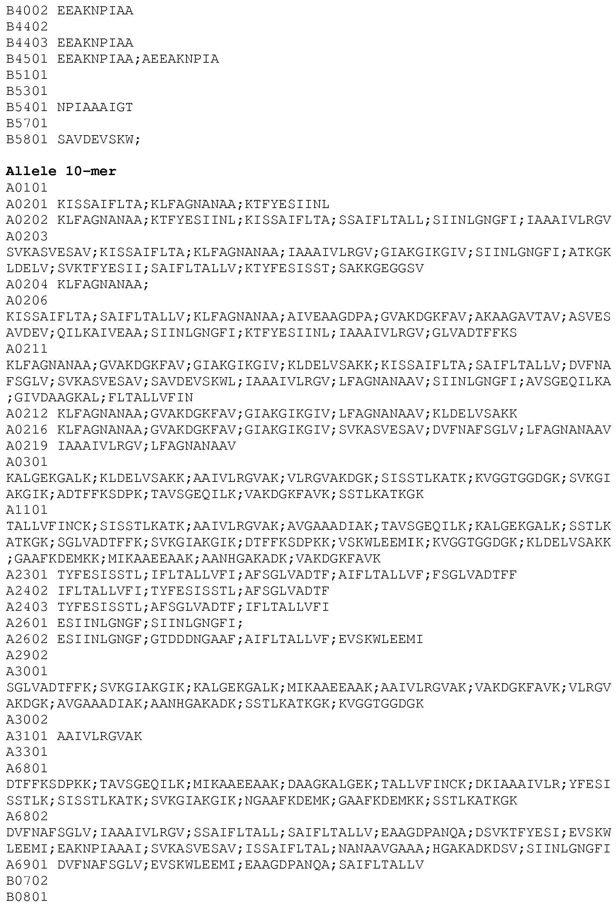

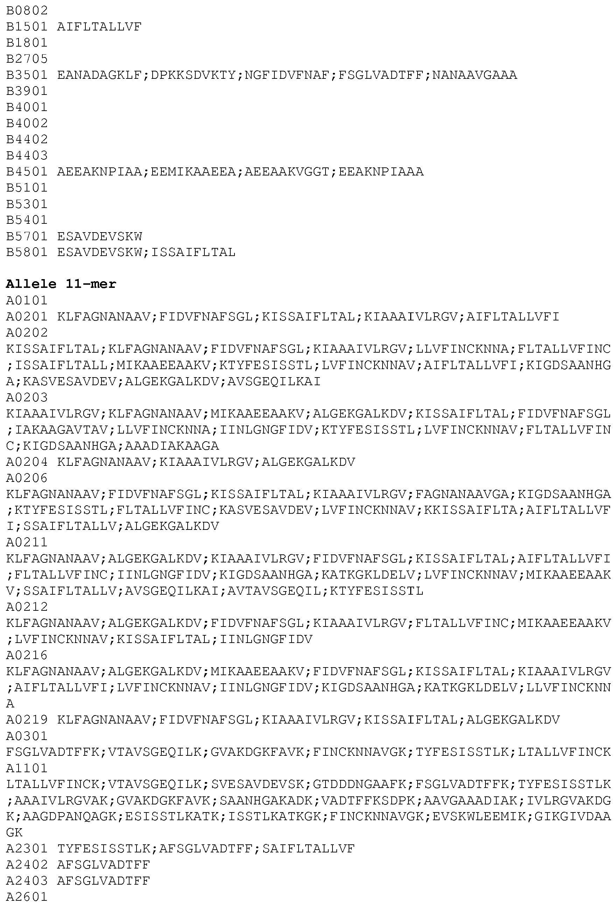

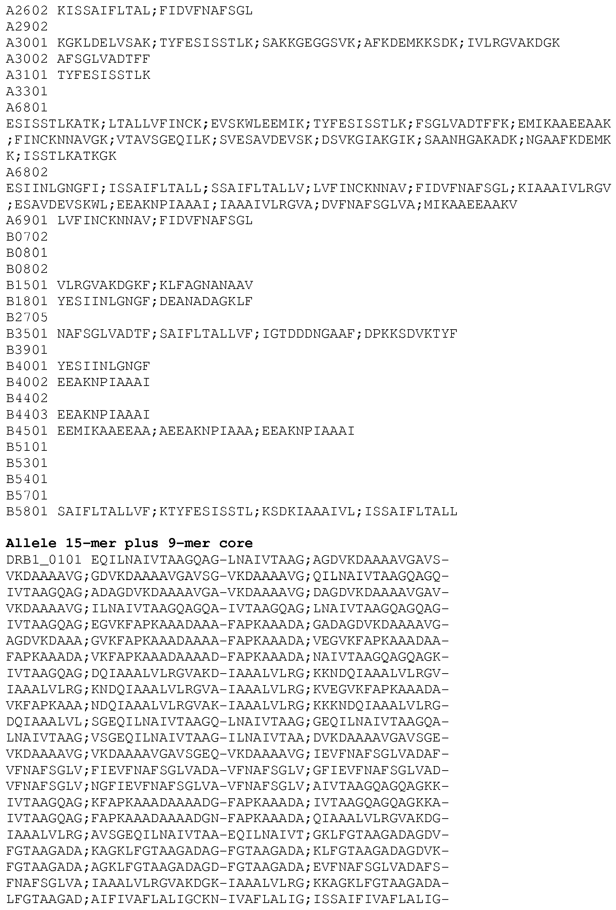

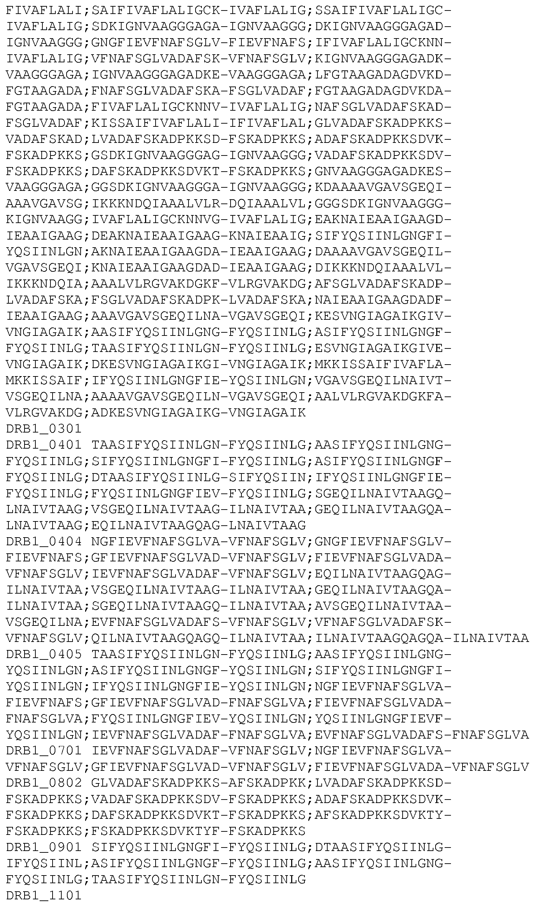

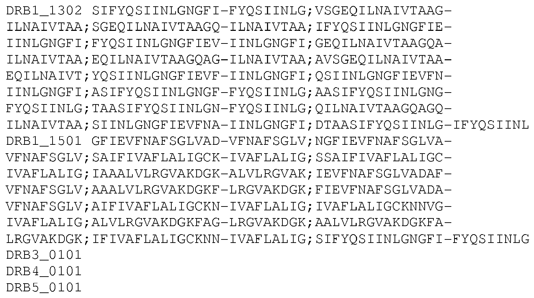

Figure 41: NetMHC peptide sequences from figure 40 are grouped according to HLA binding alleles and sub-grouped according to peptide length.

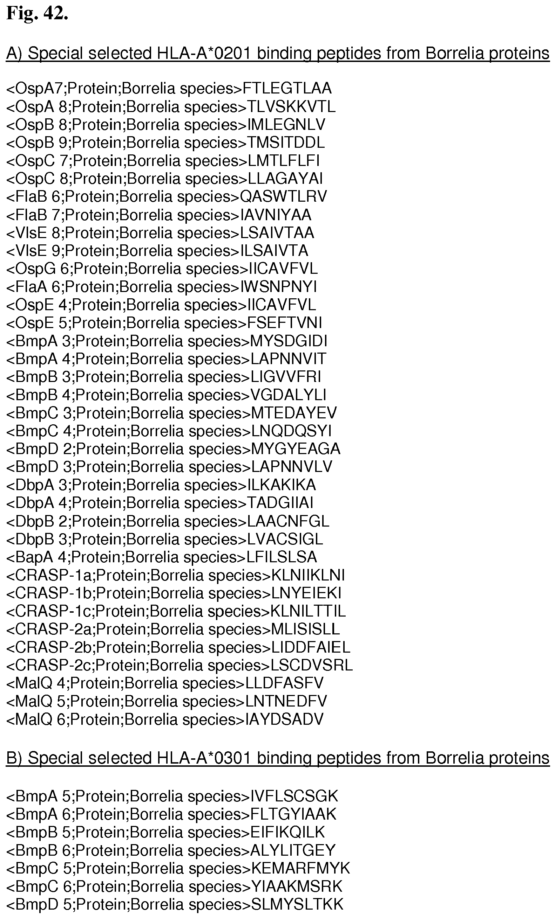

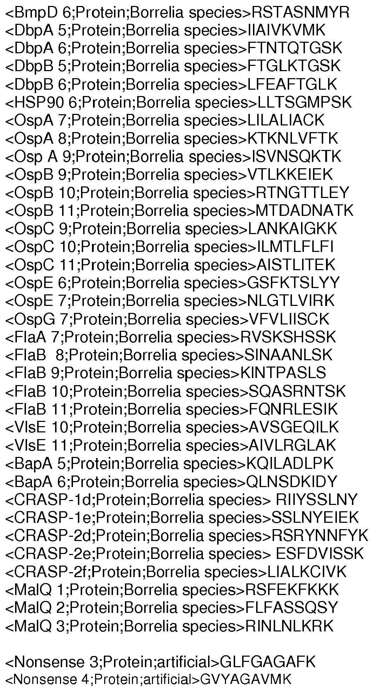

Figure 42: Selected peptides. The amino acid sequence from one or more Borrelia strains were aligned using the protein alignment programs Vector NTI from Invitrogen, and a homologous sequence for all analyzed strains were be identified. This homologous sequence were run through the "intelligent" peptide epitope prediction program NetMHC as described elsewhere herein to identify epitopes able to bind HLA-A*02 or HLA-A*03. The identified epitopes are shown in this figure. A) Special selected HLA-A*0201 binding peptides from Borrelia proteins; B) Special selected HLA-A*0301 binding peptides from Borrelia proteins.

Figure 43: Borrelia MHC class 1 and 2 antigen peptides of 8-, 9-, 10- and 11 amino acids and 13-, 14-, 15- and 16 amino acids in length generated from the chosen protein sequences by random prediction software (SEQ ID NO 180858 to SEQ ID NO 215925). NetMHC peptides predicted by the http://www.cbs.dtu.dk/services/NetMHC/ database (SEQ ID NO 215926 to SEQ ID NO216339). Name and protein bank accession numbers are shown.

Figure 44: Special selected HLA-A*0201 epitopes from Borrelia species. Some peptides may be identical to sequences in either Borrelia afzelii. B. garinii or B. burgdorferi but some will be optimized to show possible fit to more than one species ie. some amino acid position have been altered compared to the individual parent sequences.

Figure 45: Special selected HLA-A*03 epitopes from Borrelia species. Some peptides may be identical to sequences in either Borrelia afzelii. B. garinii or B. burgdorferi but some will be optimized to show possible fit to more than one species ie. some amino acid position have been altered compared to the individual parent sequences.

**INDEX TO SEQUENCE LIST**

The sequences in the figures and tables are included in the sequence list. The sequences have the SEQ ID NO's indicated below - the sequences in each figure or table are consecutively numbered.
Figure 30:
SEQ ID NO 1-44
Figure 31:

| Antigen designation in Table 19 | SEQ ID NO |
|---|---|
| NP_212517.1 Basic membrane protein A (bmpA) [Borrelia burgdorferi B31] | 45-1366 |
| NP_212516.1 Basic membrane protein B (bmpB) [Borrelia burgdorferi B31 | 1367-2696 |
| NP_212518.1 Basic membrane protein C (bmpC) [Borrelia burgdorferi B31 | 2697-4074 |
| NP_212519.1B Basic membrane protein D (bmpD) [Borrelia burgdorferi B31 | 4075-5480 |
| AAC70056.1 Decorin binding protein A; DbpA [Borrelia afzelii | 5481-6126 |
| AAC70057.1 Decorin binding protein A; DbpA [Borrelia garinii] | 6127-6836 |
| AAC70021.1 Decorin binding protein B; DbpB [Borrelia burgdorferi] | 6837-7522 |
| NP_212694.1 Heat shock protein 90 [Borrelia burgdorferi B31] | 7523-10087 |
| CAA44492.1| Outer surface protein A [Borrelia burgdorferi] | 10088-11149 |
| |BAA22351.1| Outer surface protein B [Borrelia garinii] | 11150-12307 |
| AAM22469.1| Outer surface protein C [Borrelia afzelii] | 12308-13117 |
| AAC62927.1| OspE-related lipoprotein [Borrelia garinii] | 13118-13799 |
| CAA57806.1| Outer surface protein G [Borrelia burgdorferi] | 13800-14549 |
| AAC44770.1| FlaA protein (Borrelia burgdorferi) | 14550-15879 |
| AAU07005.1| flagellar filament 41 kDa core protein [Borrelia garinii PBi] | 15880-17189 |
| 1L8W|A Chain A, Crystal Structure Of Lyme Disease Variable Surface Antigen Vise Of Borrelia Burgdorferi | 17190-18547 |
| CAA57807.1 Associated protein A (BapA)[Borrelia burgdorferi] | 18548-19189 |
| Seq 19 | 19190-20455 |
| Seq 20 | 20456-22321 |
| Seq 21 | 22322-23355 |
| Seq 22 | 23356-24473 |
| Seq 23 | 24474-25783 |
| Seq 24 | 25784-27861 |
| Seq 25 | 27862-28855 |
| Seq 26 | 28856-31653 |
| Seq 27 | 31564-33623 |
| Seq 28 | 33624-35913 |
| Seq 29 | 35914-36939 |
| Seq 30 | 36940-38333 |

(continued)

| Antigen designation in Table 19 | SEQ ID NO |
|---|---|
| Seq 31 | 38334-38919 |
| Seq 32 | 38920-39737 |
| Seq 33 | 39738-40611 |
| Seq 34 | 40612-42220 |
| Seq 35 | 42221-43880 |
| Seq 36 | 43881-44898 |
| Seq 37 | 44899-45904 |
| Seq 38 | 45905-46606 |
| Seq 39 | 46607-47212 |
| Seq 40 | 47213-48406 |
| Seq 41 | 48407-49644 |
| Seq 42 | 49645-51050 |
| Seq 43 | 51051-52424 |
| Seq 44 | 52425-54050 |

Figure 32

| Antigen designation | SEQ ID NO |
|---|---|
| NP_212517.1; Basic membrane protein A (bmpA) [Borrelia burgdorferi B31; Seq1 | 54051-54446 |
| NP_212516.1; Basic membrane protein B (bmpB) [Borrelia burgdorferi B31; Seq2 | 54447-54934 |
| NP_212518.1; Basic membrane protein C (bmpC) [Borrelia burgdorferi B31; Seq3 | 54935-55486 |
| NP_212519.1B; Basic membrane protein D (bmpD) [Borrelia burgdorferi B31; Seq4 | 55487-56052 |
| AAC70056.1; Decorin binding protein A; DbpA [Borrelia afzelii; Seq5 | 56053-56237 |
| AAC70057.1; Decorin binding protein A; DbpA [Borrelia garinii]; Seq6 | 56238-56436 |
| AAC70021.1; Decorin binding protein B; DbpB [Borrelia burgdorferi]; Seq7 | 56437-56639 |
| NP_212694.1; Heat shock protein 90 [Borrelia burgdorferi B31]; Seq8 | 56640-57579 |
| CAA44492.1| Outer surface protein A [Borrelia burgdorferi]; Seq9 | 57580-57833 |
| |BAA22351.1| Outer surface protein B [Borrelia garinii]; Seq10 | 57834-58137 |
| AAM22469.1| Outer surface protein C [Borrelia afzelii]; Seq11 | 58138-58345 |
| AAC62927.1| OspE-related lipoprotein [Borrelia garinii]; Seq12 | 58346-58523 |
| CAA57806.1| Outer surface protein G [Borrelia burgdorferi]; Seq13 | 58524-58669 |
| AAC44770.1| FlaA protein (Borrelia burgdorferi) ; Seq14 | 58670-59232 |
| BAD18055.1 ; FlaB protein [Borrelia garinii]; Seq15 | 59233-59392 |
| AAU07005.1| flagellar filament 41 kDa core protein [Borrelia garinii PBi]; Seq16 | 59393-59741 |
| 1L8W|A Chain A, Crystal Structure Of Lyme Disease | 59742-60008 |
| Variable Surface Antigen VlsE1 Of Borrelia Burgdorferi; Seq17 | |
| CAA57807.1; Associated protein A (BapA)[Borrelia burgdorferi]; Seq18 | 60009-60203 |
| AAL25643.1, P37-47 [Borrelia burgdorferi]; Seq19 | 60204-60500 |

(continued)

| Antigen designation | SEQ ID NO |
|---|---|
| NP_212481.1| fibronectin/fibrinogen-binding protein, putative [Borrelia burgdorferi B31]; Seq20 | 60501-61184 |
| AAC44656.1| P30 Borrelia burgdorferi; Seq21 | 61185-61566 |
| NP_045619.1| immunogenic protein P37, putative [Borrelia burgdorferi B31]; Seq22 | 61567-61910 |
| NP_212281.1| flagellin [Borrelia burgdorferi B31]; Seq23 | 61911-62261 |
| NP_212463.1| oligopeptide ABC transporter, periplasmic oligopeptide-binding protein (oppA-2) ; [Borrelia burgdorferi B31]; Seq24 | 62262-63060 |
| AAC44381.1| outer membrane porin protein Oms28 precursor; Seq25 | 63061-63322 |
| CAA49829.1| p93 [Borrelia burgdorferi]; Seq26 | 63323-64135 |
| NP_212375.1| glycerol kinase (glpK) [Borrelia burgdorferi B31]; Seq27 | 64136-64773 |
| NP_212342.1| hypothetical protein BB0208 [Borrelia burgdorferi B31]; Seq28 | 64774-65881 |
| NP_045482.1| hypothetical protein BBG22 [Borrelia burgdorferi B31]; Seq29 | 65882-66290 |
| NP_212885.1| hypothetical protein BB0751 [Borrelia burgdorferi B31]; Seq30 | 66291-66734 |
| AAC67038.1| predicted coding region BB0689 [Borrelia burgdorferi B31]; Seq31 | 66735-66934 |
| NP_045709.1| lipoprotein BBA36[Borrelia burgdorferi B31]; Seq32 | 66935-67186 |
| AAT93789.1| lipoprotein BBA36 (homolog 67%)[Borrelia garinii PBi]; Seq33 | 67187-67422 |
| NP_045739.1| BBA66 antigen, P35, putative [Borrelia burgdorferi B31]; Seq34 | 67423-67916 |
| AAT93824.1|BBA66 antigen, P35, putative [Borrelia garinii PBi]; Seq35 | 67917-68425 |
| NP_045742.1| hypothetical protein BBA69 [Borrelia burgdorferi B31]; Seq36 | 68426-68757 |
| AAT93826.1| conserved hypothetical protein BBA69[Borrelia garinii PBi]; Seq37; | 68758-69026 |
| NP_045573.1| hypothetical protein BBI42 [Borrelia burgdorferi B31]; Seq38 | 69027-69299 |
| NP_051318.1| revA protein[Borrelia burgdorferi B31]; Seq39 | 69300-69486 |
| NP_045737.1| BBA64 antigen, P35 [Borrelia burgdorferi B31]; Seq40 | 69487-69835 |
| AAT93822.1| BBA64 antigen, P35 [Borrelia garinii PBi]; Seq41 | 69836-70189 |
| AAL84596.1| BBK32 [Borrelia burgdorferi]; Seq42 | 70190-70651 |
| AAL84590.1| BBK32 [Borrelia afzelii]; Seq43 | 70652-71087 |
| AAL84595.1| BBK32 [Borrelia garinii]; Seq44 | 71088-71575 |

Figure 33

| Antigen designation | SEQ ID NO |
|---|---|
| NP_212517.1 Basic membrane protein A (bmpA) [Borrelia burgdorferi B31 | 71576-72877 |
| NP_212516.1 Basic membrane protein B (bmpB) [Borrelia burgdorferi B31 | 72848-74187 |
| NP_212518.1 Basic membrane protein C (bmpC) [Borrelia burgdorferi B31 | 74188-75545 |
| NP_212519.1B Basic membrane protein D (bmpD) [Borrelia burgdorferi B31 | 75546-76931 |
| AAC70056.1 Decorin binding protein A; DbpA [Borrelia afzelii | 76932-77557 |
| AAC70057.1 Decorin binding protein A; DbpA [Borrelia garinii] | 77558-78247 |
| AAC70021.1 Decorin binding protein B; DbpB [Borrelia burgdorferi] | 78248-78913 |
| NP_212694.1 Heat shock protein 90 [Borrelia burgdorferi B31] | 78914-81459 |
| CAA44492.1| Outer surface protein A [Borrelia burgdorferi] | 81460-82502 |

(continued)

| Antigen designation | SEQ ID NO |
|---|---|
| \|BAA22351.1\| Outer surface protein B [Borrelia garinii] | 82503-83640 |
| AAM22469.1\| Outer surface protein C [Borrelia afzelii] | 83641-84430 |
| AAC62927.1\| OspE-related lipoprotein [Borrelia garinii] | 84431-85092 |
| CAA57806.1\| Outer surface protein G [Borrelia burgdorferi] | 85093-85822 |
| AAC44770.1\| FlaA protein (Borrelia burgdorferi) | 85823-87132 |
| AAU07005.1\| flagellar filament 41 kDa core protein [Borrelia garinii PBi] | 87133-88422 |
| 1L8W\|A Chain A, Crystal Structure Of Lyme Disease Variable Surface Antigen Vise Of Borrelia Burgdorferi | 88423-89760 |
| CAA57807.1 Associated protein A (BapA)[Borrelia burgdorferi] | 89761-90382 |
| AAL25643.1, P37-47 [Borrelia burgdorferi]; Seq19 | 90383-91628 |
| NP_212481.11 fibronectin/fibrinogen-binding protein, putative [Borrelia burgdorferi B31]; Seq20 | 91629-93474 |
| AAC44656.1\| P30 Borrelia burgdorferi; Seq21 | 93475-94488 |
| NP_045619.1\| immunogenic protein P37, putative [Borrelia burgdorferi B31]; Seq22 | 94489-95586 |
| NP_212281.1\| flagellin [Borrelia burgdorferi B31]; Seq23 | 95587-96876 |
| NP_212463.1\| oligopeptide ABC transporter, periplasmic oligopeptide-binding protein (oppA-2) ; [Borrelia burgdorferi B31]; Seq24 | 96877-98934 |
| AAC44381.1\| outer membrane porin protein Oms28 precursor; Seq25 | 98935-99908 |
| CAA49829.1\| p93 [Borrelia burgdorferi]; Seq26 | 99909-102686 |
| NP_212375.1\| glycerol kinase (glpK) [Borrelia burgdorferi B31]; Seq27 | 102687-104636 |
| NP_212342.1\| hypothetical protein BB0208 [Borrelia burgdorferi B31]; Seq28 | 104637-106906 |
| NP_045482.1\| hypothetical protein BBG22 [Borrelia burgdorferi B31]; Seq29 | 106907-107912 |
| NP_212885.1\| hypothetical protein BB0751 [Borrelia burgdorferi B31]; Seq30 | 107913-109286 |
| AAC67038.1\| predicted coding region BB0689 [Borrelia burgdorferi B31]; Seq31 | 109287-109852 |
| NP_045709.1\| lipoprotein BBA36[Borrelia burgdorferi B31]; Seq32 | 109853-110650 |
| AAT93789.1\| lipoprotein BBA36 (homolog 67%)[Borrelia garinii PBi]; Seq33 | 110651-111504 |
| NP_045739.1\| BBA66 antigen, P35, putative [Borrelia burgdorferi B31]; Seq34 | 111505-113094 |
| AAT93824.1\|BBA66 antigen, P35, putative [Borrelia garinii PBi]; Seq35 | 113095-114736 |
| NP_045742.1\| hypothetical protein BBA69 [Borrelia burgdorferi B31]; Seq36 | 114737-116378 |
| AAT93826.1\| conserved hypothetical protein BBA69[Borrelia garinii PBi]; Seq37; | 116379-117364 |
| NP_045573.1\| hypothetical protein BBI42 [Borrelia burgdorferi B31]; Seq38 | 117365-118046 |
| NP_051318.1\| revA protein[Borrelia burgdorferi B31]; Seq39 | 118047-118632 |
| NP_045737.1\| BBA64 antigen, P35 [Borrelia burgdorferi B31]; Seq40 | 118633-119806 |
| AAT93822.1\| BBA64 antigen, P35 [Borrelia garinii PBi]; Seq41 | 119807-121024 |
| AAL84596.1\| BBK32 [Borrelia burgdorferi]; Seq42 | 121025-122410 |
| AAL84590.1\| BBK32 [Borrelia afzelii]; Seq43 | 122411-123764 |
| AAL84595.1\| BBK32 [Borrelia garinii]; Seq44 | 123765-125370 |

Figure 34

| Antigen designation | SEQ ID NO |
|---|---|
| NP_212517.1 Basic membrane protein A (bmpA) [Borrelia burgdorferi B31 Seq1 | 125371-126024 |
| NP_212516.1 Basic membrane protein B (bmpB) [Borrelia burgdorferi B31 Seq2 | 126025-126584 |
| NP_212518.1 Basic membrane protein C (bmpC) [Borrelia burgdorferi B31 Seq3 | 126585-127288 |
| NP_212519.1B Basic membrane protein D (bmpD) [Borrelia burgdorferi B31 Seq4 | 127289-127904 |
| AAC70056.1 Decorin binding protein A DbpA [Borrelia afzelii Seq5 | 127905-128186 |
| AAC70057.1 Decorin binding protein A DbpA [Borrelia garinii] Seq6 | 128187-128428 |
| AAC70021.1 Decorin binding protein B DbpB [Borrelia burgdorferi] Seq7 | 128429-128684 |
| NP_212694.1 Heat shock protein 90 [Borrelia burgdorferi B31] Seq8 | 128685-129678 |
| CAA44492.1| Outer surface protein A [Borrelia burgdorferi] Seq9 | 129679-129984 |
| |BAA22351.1| Outer surface protein B [Borrelia garinii] Seq10 | 129985-130282 |
| AAM22469.1| Outer surface protein C [Borrelia afzelii] Seq11 | 130283-130498 |
| AAC62927.1| OspE-related lipoprotein [Borrelia garinii] Seq12 | 130499-130870 |
| CAA57806.1| Outer surface protein G [Borrelia burgdorferi] Seq13 | 130871-131040 |
| AAC44770.1| FlaA protein (Borrelia burgdorferi) Seq14 | 131041-131966 |
| BAD18055.1 FlaB protein [Borrelia garinii] Seq15 | 131967-132356 |
| AAU07005.1| flagellar filament 41 kDa core protein [Borrelia garinii PBi] Seq16 | 132357-132940 |
| 1L8W|A Chain A, Crystal Structure Of Lyme Disease Variable Surface Antigen VlsE1 Of Borrelia Burgdorferi Seq17 | 132941-133116 |
| CAA57807.1 Associated protein A (BapA)[Borrelia burgdorferi] Seq18 | 133117-133398 |
| AAL25643.1, P37-47 [Borrelia burgdorferi] Seq19 | 133399-133708 |
| NP_212481.11 fibronectin/fibrinogen-binding protein, putative [Borrelia burgdorferi B31] Seq20 | 133709-134764 |
| AAC44656.1| P30 Borrelia burgdorferi Seq21 | 134765-135226 |
| NP_045619.1| immunogenic protein P37, putative [Borrelia burgdorferi B31] Seq22 | 135227-135614 |
| NP-212281.1| flagellin [Borrelia burgdorferi B31] Seq23 | 135615-136268 |
| NP_212463.1| oligopeptide ABC transporter, periplasmic oligopeptide-binding protein (oppA-2) [Borrelia burgdorferi B31] Seq24 | 136269-137092 |
| AAC44381.1| outer membrane porin protein Oms28 precursor Seq25 | 137093-137570 |
| CAA49829.1| p93 [Borrelia burgdorferi] Seq26 | 137571-138312 |
| NP_212375.1| glycerol kinase (glpK) [Borrelia burgdorferi B31] Seq27 | 138313-139244 |
| NP_212342.1| hypothetical protein BB0208 [Borrelia burgdorferi B31] Seq28 | 139245-139616 |
| NP_045482.1| hypothetical protein BBG22 [Borrelia burgdorferi B31] Seq29 | 139617-140010 |
| NP_212885.1| hypothetical protein BB0751 [Borrelia burgdorferi B31] Seq30 | 140011-140476 |
| AAC67038.1| predicted coding region BB0689 [Borrelia burgdorferi B31] Seq31 | 140477-140768 |
| NP_045709.1| lipoprotein BBA36[Borrelia burgdorferi B31] Seq32 | 140769-141052 |
| AAT93789.1| lipoprotein BBA36 (homolog 67%)[Borrelia garinii PBi] Seq33 | 141053-141368 |
| NP_045739.1| BBA66 antigen, P35, putative [Borrelia burgdorferi B31] Seq34 | 141369-142034 |
| AAT93824.1|BBA66 antigen, P35, putative [Borrelia garinii PBi] Seq35 | 142035-142838 |
| NP_045742.1| hypothetical protein BBA69 [Borrelia burgdorferi B31] Seq36 | 142839-143178 |
| AAT93826.1| conserved hypothetical protein | 143179-143468 |

(continued)

| Antigen designation | SEQ ID NO |
|---|---|
| BBA69[Borrelia garinii PBi] Seq37 | |
| NP_045573.1| hypothetical protein BBI42 [Borrelia burgdorferi B31] Seq38 | 143469-143852 |
| NP_051318.1| revA protein[Borrelia burgdorferi B31] Seq39 | 143853-144042 |
| NP_045737.1| BBA64 antigen, P35 [Borrelia burgdorferi B31] Seq40 | 144043-144728 |
| AAT93822.1| BBA64 antigen, P35 [Borrelia garinii PBi] Seq41 | 144729-145414 |
| AAL84596.1| BBK32 [Borrelia burgdorferi] Seq42 | 145415-145890 |
| AAL84590.1| BBK32 [Borrelia afzelii] Seq43 | 145891-146426 |
| AAL84595.1| BBK32 [Borrelia garinii] Seq44 | 146427-146962 |

Figure 38

| Antigen | SEQ ID NO |
|---|---|
| <ABX71745 CRASP-2,Borrelia Burgdorferi> | 146963 |
| <CAH10086 CRASP-1,Borrelia garinii> | 146964 |
| <AAU07022 4-alpha-glucanotransferase,Borrelia garinii PBi> | 146965 |
| <CAA59725 outer surface protein A,Borrelia afzelii> | 146966 |
| <CAA59727 outer surface protein A,Borrelia garinii> | 146967 |
| <YP_853838 outer surface protein B,Borrelia afzelii PKo> | 146968 |
| <NP_045689 outer surface protein B,Borrelia burgdorferi B31> | 146969 |
| AAN87995 OspC,Borrelia garinii> | 146970 |
| <NP_047005 outer surface protein C, Borrelia burgdorferi > | 146971 |
| <CAF34024 outer surface protein VlsE,Borrelia garinii> | 146972 |
| <CAF34027 outer surface protein VlsE,Borrelia afzelii PKo> | 146973 |

Figure 39

| Antigen | SEQ ID NO |
|---|---|
| <ABX71745 CRASP-2,Borrelia Burgdorferi> | 146974-148805 |
| <CAH10086 CRASP-1,Borrelia garinii> | 148806-150757 |
| <AAU07022 4-alpha-glucanotransferase,Borrelia garinii PBi> | 150758-154717 |
| <CAA59727 outer surface protein A,Borrelia garinii> | 154718-156821 |
| <CAA59725 outer surface protein A,Borrelia afzelii> | 156822-158917 |
| <YP_853838 outer surface protein B,Borrelia afzelii PKo> | 158918-161221 |
| <NP_045689 outer surface protein B,Borrelia burgdorferi > | 161222-163501 |
| AAN87995 OspC,Borrelia garinii> | 163502-165005 |
| <NP_047005 outer surface protein C, Borrelia burgdorferi > | 165006-166597 |
| <CAF34024 outer surface protein VlsE,Borrelia garinii> | 166598-168853 |
| <CAF34027 outer surface protein VlsE,Borrelia afzelii PKo> | 168854-171149 |

Figure 40

| Antigen | SEQ ID NO |
|---|---|
| \<ABX71745 CRASP-2;Protein;Borrelia burgdorferi> Class 1 and 2 | 171150-171695 |
| \<NP_045689 outer surface protein B;Protein;Borrelia burgdorferi B31> Class 1 and 2 | 171696-172134 |
| \<NP_047005 outer surface protein C;Protein;Borrelia burgdorferi B31> Class 1 and 2 | 172135-172552 |
| \<CAA59725 outer surface protein A;Protein;Borrelia afzelii> Class 1 and 2 | 172553-172996 |
| \<YP_853838 outer surface protein B;Protein;Borrelia afzelii PKo> Class 1 and 2 | 172997-173522 |
| \<CAF34027 outer surface protein VlsE;Protein;Borrelia afzelii PKo> Class 1 and 2 | 173523-174053 |
| \<CAH10086 CRASP-1 ;Protein;Borrelia garinii> Class 1 and 2 | 174054-174491 |
| \<AAU07022 4-alpha-glucanotransferase;Protein;Borrelia garinii PBi> Class 1 and 2 | 174492-176082 |
| \<CAA59727 outer surface protein A;Protein;Borrelia garinii> Class 1 and 2 | 176083-176568 |
| \<AAN87995 Outer Surface Protein C;Protein;Borrelia garinii> Class 1 and 2 | 176569-176943 |
| \<CAF34024 outer surface protein VlsE;Protein;Borrelia garinii> Class 1 and 2 | 176944-177458 |

Figure 41
The peptides listed in Figure 41 are identical to the ones listed in Figure 40.
Figure 42

| Peptide | SEQ ID NO |
|---|---|
| \<OspA7;Protein;Borrelia species>FTLEGTLAA | 177459 |
| \<OspA 8;Protein;Borrelia species>TLVSKKVTL | 177460 |
| \<OspB 8;Protein;Borrelia species>IMLEGNLV | 177461 |
| \<OspB 9;Protein;Borrelia species>TMSITDDL | 177462 |
| \<OspC 7;Protein;Borrelia species>LMTLFLFI | 177463 |
| \<OspC 8;Protein;Borrelia species>LLAGAYAI | 177464 |
| \<FlaB 6;Protein;Borrelia species>QASWTLRV | 177465 |
| \<FlaB 7;Protein;Borrelia species>IAVNIYAA | 177466 |
| \<VlsE 8;Protein;Borrelia species>LSAIVTAA | 177467 |
| \<VlsE 9;Protein;Borrelia species>ILSAIVTA | 177468 |
| \<OspG 6;Protein;Borrelia species>IICAVFVL | 177469 |
| \<FlaA 6;Protein;Borrelia species>IWSNPNYI | 177470 |
| \<OspE 4;Protein;Borrelia species>IICAVFVL | 177471 |
| \<OspE 5;Protein;Borrelia species>FSEFTVNI | 177472 |
| \<BmpA 3;Protein;Borrelia species>MYSDGIDI | 177473 |
| \<BmpA 4;Protein;Borrelia species>LAPNNVIT | 177474 |
| \<BmpB 3;Protein;Borrelia species>LIGWFRI | 177475 |
| \<BmpB 4;Protein;Borrelia species>VGDALYLI | 177476 |
| \<BmpC 3;Protein;Borrelia species>MTEDAYEV | 177477 |
| \<BmpC 4;Protein;Borrelia species>LNQDQSYI | 177478 |
| \<BmpD 2;Protein;Borrelia species>MYGYEAGA | 177479 |
| \<BmpD 3;Protein;Borrelia species>LAPNNVLV | 177480 |

(continued)

| Peptide | SEQ ID NO |
|---------|-----------|
| <DbpA 3;Protein;Borrelia species>ILKAKIKA | 177481 |
| <DbpA 4;Protein;Borrelia species>TADGIIAI | 177482 |
| <DbpB 2;Protein;Borrelia species>LAACNFGL | 177483 |
| <DbpB 3;Protein;Borrelia species>LVACSIGL | 177484 |
| <BapA 4;Protein;Borrelia species>LFILSLSA | 177485 |
| <CRASP-1a;Protein;Borrelia species>KLNIIKLNI | 177486 |
| <CRASP-1b;Protein;Borrelia species>LNYEIEKI | 177487 |
| <CRASP-1c;Protein;Borrelia species>KLNILTTIL | 177488 |
| <CRASP-2a;Protein;Borrelia species>MLISISLL | 177489 |
| <CRASP-2b;Protein;Borrelia species>LIDDFAIEL | 177490 |
| <CRASP-2c;Protein;Borrelia species>LSCDVSRL | 177491 |
| <MalQ 4;Protein;Borrelia species>LLDFASFV | 177492 |
| <MalQ 5;Protein;Borrelia species>LNTNEDFV | 177493 |
| <MalQ 6;Protein;Borrelia species>IAYDSADV | 177494 |
| <BmpA 5;Protein;Borrelia species>IVFLSCSGK | 177495 |
| <BmpA 6;Protein;Borrelia species>FLTGYIAAK | 177496 |
| <BmpB 5;Protein;Borrelia species>EIFIKQILK | 177497 |
| <BmpB 6;Protein;Borrelia species>ALYLITGEY | 177498 |
| <BmpC 5;Protein;Borrelia species>KEMARFMYK | 177499 |
| <BmpC 6;Protein;Borrelia species>YIAAKMSRK | 177500 |
| <BmpD 5;Protein;Borrelia species>SLMYSLTKK | 177501 |
| <BmpD 6;Protein;Borrelia species>RSTASNMYR | 177502 |
| <DbpA 5;Protein;Borrelia species>IIAIVKVMK | 177503 |
| <DbpA 6;Protein;Borrelia species>FTNTQTGSK | 177504 |
| <DbpB 5;Protein;Borrelia species>FTGLKTGSK | 177505 |
| <DbpB 6;Protein;Borrelia species>LFEAFTGLK | 177506 |
| <HSP90 6;Protein;Borrelia species>LLTSGMPSK | 177507 |
| <OspA 7;Protein;Borrelia species>LILALIACK | 177508 |
| <OspA 8;Protein;Borrelia species>KTKNLVFTK | 177509 |
| <OspA 9;Protein;Borrelia species>ISVNSQKTK | 177510 |
| <OspB 9;Protein;Borrelia species>VTLKKEIEK | 177511 |
| <OspB 10;Protein;Borrelia species>RTNGTTLEY | 177512 |
| <OspB 11;Protein;Borrelia species>MTDADNATK | 177513 |
| <OspC 9;Protein;Borrelia species>LANKAIGKK | 177514 |
| <OspC 10;Protein;Borrelia species>ILMTLFLFI | 177515 |
| <OspC 11;Protein;Borrelia species>AISTLITEK | 177516 |
| <OspE 6;Protein;Borrelia species>GSFKTSLYY | 177517 |
| <OspE 7;Protein;Borrelia species>NLGTLVIRK | 177518 |

(continued)

| Peptide | SEQ ID NO |
|---|---|
| <OspG 7;Protein;Borrelia species>VFVLIISCK | 177519 |
| <FlaA 7;Protein;Borrelia species>RVSKSHSSK | 177520 |
| <FlaB 8;Protein;Borrelia species>SINAANLSK | 177521 |
| <FlaB 9;Protein;Borrelia species>KINTPASLS | 177522 |
| <FlaB 10;Protein;Borrelia species>SQASRNTSK | 177523 |
| <FlaB 11;Protein;Borrelia species>FQNRLESIK | 177524 |
| <VlsE 10;Protein;Borrelia species>AVSGEQILK | 177525 |
| <VlsE 11;Protein;Borrelia species>AIVLRGLAK | 177526 |
| <BapA 5;Protein;Borrelia species>KQILADLPK | 177527 |
| <BapA 6;Protein;Borrelia species>QLNSDKIDY | 177528 |
| <CRASP-1d;Protein;Borrelia species> RIIYSSLNY | 177529 |
| <CRASP-1e;Protein;Borrelia species>SSLNYEIEK | 177530 |
| <CRASP-2d;Protein;Borrelia species>RSRYNNFYK | 177531 |
| <CRASP-2e;Protein;Borrelia species> ESFDVISSK | 177532 |
| <CRASP-2f;Protein;Borrelia species>LIALKCIVK | 177533 |
| <MalQ 1;Protein;Borrelia species>RSFEKFKKK | 177534 |
| <MalQ 2;Protein;Borrelia species>FLFASSQSY | 177535 |
| <MalQ 3;Protein;Borrelia species>RINLNLKRK | 177536 |
| <Artificial;Protein> | 177537 |
| <Artificial;Protein> | 177538 |

Table A: SEQ ID NO 177539-179138
Table B SEQ ID NO 179139-179392
Table C SEQ ID NO 179393-179552
Figure 36 SEQ ID NO 179553-180345
Figure 37 SEQ ID NO 180346-180857
Figure 43 SEQ ID NO 180858-216339
Figure 44 SEQ ID NO 216340-216404
Figure 45 SEQ ID NO 216405-216469
Fig 36 SEQ ID NO 216470-217262
Fig 37 SEQ ID NO 217263-217774

## EXAMPLES

Examples not falling under the scope of the appended claims do not form part of the invention.

### Example 1

This example describes how to make a MHC class I complex with a peptide in the peptide binding-groove using *in vitro refolding.* The MHC-complex in this example consisted of light chain β2m, the MHC class I Heavy Chain allele HLA-A*0201 (a truncated version in which the intracellular and transmembrane domains have been deleted) and the peptide QLFEELQEL (SEQ ID NO 217775).

MHC I-complexes consists of 3 components; Light Chain (β2m), Heavy Chain and a peptide of typically 8-10 amino acids. In this example MHC-complexes was generated by *in vitro* refolding of heavy chain, β2m and peptide in a buffer containing reduced and oxidized glutathione. By incubation in this buffer a non-covalent complex between Heavy Chain, β2m and peptide was formed. Heavy chain and β2m was expressed as inclusion bodies in *E.coli* prior to *in vitro* refolding following standard procedures as described in Garboczi et al., (1996), Nature 384, 134-141. Following refolding the MHC complexes was biotinylated using BirA enzyme able to biotinylate a specific amino acid residue in a recognition sequence fused to the C-terminal of the Heavy Chain by genetic fusion. Monomer MHC complexes was then purified by size exclusion chromatography.

1. 200 ml of refolding buffer (100 mM Tris, 400 mM L-arginin-HCL, 2 mM NaEDTA, 0.5 mM oxidized Gluthathione, 5 mM reduced Glutathione, pH 8.0) was supplied with protease inhibitors PMSF (phenylmethylsulphonyl fluoride), Pepstatin A and Leupeptin (to a final concentration of 1 mM, 1 mg/l and 1 mg/l, respectively). The refolding buffer was placed at 10 °C on a stirrer.

2. 12 mg of peptide QLFEELQEL (SEQ ID NO 217775) was dissolved in DMSO or another suitable solvent (300-500 μl), and added drop-wise to the refolding buffer at vigorous stirring.

3. 4.4 mg of human Light Chain β2m was added drop-wise to the refolding buffer at vigorous stirring.

4. 6.2 mg of Heavy Chain HLA-A*0201 (supplied with DTT to a concentration of 0.1 mM) was added drop-wise to the refolding buffer at vigorous stirring.

5. The folding reaction was placed at 10°C at slow stirring for 4-8 hours.

6. After 4-8 hours, step 3 and 4 was repeated and the folding reaction is placed at 10°C at slow stirring O/N.

7. Step 3 and 4 was repeated, and the folding reaction is placed at 10°C at slow stirring for 6-8 hours.

Optionally, steps 5-7 may be done in less time, e.g. a total of 0.5-5 hours.

8. After 6-8 hours the folding reaction was filtrated through a 0.2 μm filter to remove aggregates.

9. The folding reaction was concentrated O/N at 10°C shaking gently in a suitable concentrator with a 5000 mw cut-off filter. The folding reaction was concentrated to approximately 5-10 ml. (Optionally the filtrate can be stored at 4°C and reused for another folding with the same peptide and heavy chain.)

10. The concentrated folding reaction was buffer-exchanged at least 8 times, into a MHC-buffer (20 mM Tris-HCl, 50 mM NaCl, pH 8.0) and concentrated (at 10°C in a suitable concentrator with a 5000 mw cut-off filter) down to approximately 1 ml.

11. The heavy chain part of the MHC-complex was biotinylated by mixing the following components: approximately 1000 μl folded MHC-complex, 100 μl each of Biomix-A, Biomix-B and d-Biotin (all 3 from Biotin Protein Ligase Kit from Avidity, 10 μl birA enzyme (3 mg/ml, from Biotin Protein Ligase Kit from Avidity, 0.5 μl Pepstatin A (2 mg/ml) and 0.5 μl Leupeptin (2 mg/ml). The above was gently mixed and incubated O/N at room temperature.

12. The biotinylated and folded MHC-complex solution was centrifuged for 5 min. at 1700x g, room temperature.

13. Correctly folded MHC-complex was separated and purified from excess biotin, excess β2m, excesss heavy chain and aggregates thereoff, by size exclusion chromatography on a column that separates proteins in the 10-100 kDa range. Correctly folded monomer MHC-complex was eluted with a MHC-buffer (20 mM Tris-HCl, 50 mM NaCl, pH 8.0). The elution profile consisted of 4 peaks, corresponding to aggregated Heavy Chain, correctly folded monomer MHC-complex, β2m and excess biotin and peptide (See figure 7).

14. Fractions containing the folded MHC-complex were pooled and concentrated to approximately 1 ml in a suitable concentrator with a 5000 mw cut-off filter. The protein-concentration was estimated from its abosorption at 280 nm.

15. Folded MHC-complex can optionally be stored stored at -170°C before further use.

16. The grade of biotinylation was analyzed by a SDS PAGE SHIFT-assay with Streptavidin (figure 8) and correct folding was confirmed by ELISA, using the antibody W6/32 that recognizes correctly folded MHC-peptide complex.

17. The above procedure may be used for folding any MHC I compexes consisting of any β2m, any heavy chain and any peptide approx. 8-11 amino acids long. Either of the components can be truncated or otherwise modified. The above procedure can also be used for generation of "empty" MHC I complexes consisting of β2m and heavy chain without peptide.

### Example 2

This example describes how to generate soluble biotinylated MHC II complexes using a baculovirus expression system, where the MHC II complex was DR4 consisting of the α-chain DRA1*0101 and the β-chain DRB1*0401 as described by Svendsen et al., (2004), J. Immunol. 173(11):7037-45. Briefly, the hydrophobic transmembrane regions of the DRα and DRβ chains of DR4 were replaced by leucine zipper dimerization domains from the transcription factors Fos and Jun to promote DR α/β assembly. This was done by ligating cytoplasmic cDNA sequences of *DRA1*0101* and *ORB1*0401* to fos- and jun-encoding sequences. A *birA* site GLNDIFEAQKIEWH (SEQ ID NO 217788)was added to the 3' end of the *DRA1*0101-fos* template. Covalently bound peptide AGFKGEQGPKGEP (SEQ ID NO 217789) derived from collagen II amino acid 261-273 were genetically attached by a flexible linker peptide to the N terminus of the DRβ-chain. Finally, the modified DRA1*0101 and DRB1*0401 inserts were cloned into the expression vector pAcAb3. The pAcAB3-DRA1*0101/DRB1*0401 plasmids were cotransfected with linearized baculovirus DNA (BD Pharmingen; BaculoGold kit) into Sf9 insect cells, according to the manufacturer's instructions. Following two rounds of plaque purification, clonal virus isolates were further amplified three times before preparation of high-titervirus ($10^8$-$10^{10}$/ml). These stocks were used to infect High Five or serum-free Sf21 insect cells (Invitrogen Life Technologies, Carlsbad, CA) for protein production. Spinner cultures (2-3 x $10^6$ cells/ml) were infected at a multiplicity of infection of 1-3 in a volume of 150 ml per 2 L spinner flask. Supernatants were harvested and proteinase inhibitor tablets (Roche, Basel, Switzerland) were added before affinity purification on MiniLeak-Low columns (Kem-En-Tec) coupled with the anti-HLA-DR monoclonal antibody L243. HLA-DR4 complexes were eluted with diethylamine (pH 11) into neutralization buffer (2 M Tris, pH 6.5) and immediately buffer exchanged and concentrated in PBS, 0.01% NaN$_3$, using Millipore (Bedford, MA) concentrators. The purity of protein was confirmed by SDS-PAGE. The purified DR4 complexes were biotinylated *in vitro* as described for MHC I complexes elsewhere herein. These complexes may now be used for coupling to any dimerization domain, e.g. divynylsulfone activated dextran 270coupled with SA and a fluorochrome.

## Example 3

This example describes how to generate empty biotinylated MHC II complexes using a baculovirus expression system , where the MHC II complex consist of any α-chain and any β-chain, including truncated and otherwise modified versions of the two. Briefly, The hydrophobic transmembrane regions of the DRα and DRβ chains of MHC II are replaced by leucine zipper dimerization domains from the transcription factors Fos and Jun to promote DR α/β assembly. This is done by ligating cytoplasmic cDNA sequences of DRα and DRβ to fos- and *jun*-encoding sequences. A *birA* site GLNDIFEAQKIEWH (SEQ ID NO 217788) is added to the 3' end of either the DRα-*fos*/ DRα-*jun* or the DRβ-*jun*/ DRβ-*fos* template. The modified DRα and DRβ inserts is cloned into the expression vector pAcAb3 and cotransfected with linearized baculovirus DNA into Sf9 insect cells, according to the manufacturer's instructions. Following rounds of plaque purification, clonal virus isolates is further amplified before preparation of high-titer virus. These stocks are used to infect High Five or serum-free Sf21 insect cells (Invitrogen Life Technologies, Carlsbad, CA) for protein production, e.g. as Spinner cultures. Supernatants are harvested and proteinase inhibitors added before affinity purification, e.g. using a MiniLeak-Low columns (Kem-En-Tec) coupled with anti-MHC II antibody. The purified MHC II complexes is biotinylated *in vitro* as described for MHC I complexes elsewhere herein. These biotinylated MHC II complexes may now be used for coupling to any dimerization domain, e.g. divynylsulfone activated dextran 270coupled with SA and a fluorochrome.

## Example 4

This example describes how to generate biotinylated MHC II complexes using a cell based protein expression system , where the MHC II complex consist of any α-chain and any β-chain, including truncated and otherwise modified versions of the two. The MHC II complex may also have a peptide bound in the peptide binding cleft.

The hydrophobic transmembrane regions of the MHC II α-chain and MHC II β-chain are replaced by leucine zipper dimerization domains from the transcription factors Fos and Jun to promote α/β chain assembly. This is done by ligating cytoplasmic cDNA sequences of α-chain and β-chain to *fos*- and *jun*-encoding sequences. A *birA* site GLNDIFEAQKIEWH (SEQ ID NO 217788) is added to the 3' end of the DRα-*fos* template. Optionally covalently bound peptide is genetically attached by a flexible linker peptide to the N terminus of the DRβ-chain. The modified DRα and DRβ inserts is cloned into a suitable expression vector and transfected into a cell line capable of protein expression, e.g. insect cells, CHO cells or similar. Transfected cells are grown in culture, supernatants are harvested and proteinase inhibitors added before affinity purification, e.g. using a MiniLeak-Low columns (Kem-En-Tec) coupled with anti-MHC II antibody. Alternatively the expressed MHC II complexes may be purified by anion- or cation-exchange chromatography. The purified MHC II complexes is biotinylated *in vitro* as described for MHC I complexes elsewhere herein. These biotinylated MHC II complexes may now be used for coupling to any dimerization domain, e.g. divynylsulfone activated dextran 270coupled with SA and a fluorochrome.

## Example 5

This is an example of how to make a MHC multimer that is a tetramer and where the MHC are attached to the multimerization domain through a non-covalent interaction The multimerization domain consist of Streptavidin. The MHC molecule was biotinylated DR4 consisting of the α-chain DRA1*0101 and the β-chain DRB1*0401 and the peptide AGFKGEQGPKGEP (SEQ ID NO 217789) derived from collagen II amino acid 261-273. The biotinylated MHC-peptide complexes was generated as described in a previous example herein.

Fluorescent DR4-peptide tetramer complexes were assembled by addition of ultra-avidin-R-PE (Leinco Technologies, St. Louis, MO) at a final molar ratio of biotinylated to DR4-peptide ultra-avidin-R-PE of 6:1. The resulting DR4-peptide multimer complexes were subjected to size exclusion on a Superdex-200 column to separate the tetramer complexes from protein aggregates and lower molecular weight complexes and excess fre DR4-peptide. The tetramer complexes were concentrated using Centicon-30 concentrators and stored at 0.1-0.3 mg/ml in a mixture of protease inhibitors.

These complexes could be used to detect specific T cells in a flow cytometry assay as described by Svendsen et al.(2004) Tracking of Proinflammatory Collagen-Specific T cells in Early and Late Collagen-Induced Arthritis in Humanized mice. J. Immunol. 173:7037-7045.

## Example 6

This is and example of generation of a multimerization domain. The multimerization domain is an activated divinylsylfone-dextran(270kDa)(VS-dex270) was coupled with streptavidin (SA) and labelled with Allophycocyanin (APC).

1. Streptavidin (approx. 100 mg SA/ml in 10 mM HEPES, 0,1M NaCl, pH 7.85) was dialysed with gentle stirring for 2 days against 10 mM HEPES, 0.1M NaCl, pH 7.85 (20 fold excess volume) at 2-8°C with 1 buffer change/day.

2. 5 ml of APC from a homogen suspension (approx. 10 mg/ml) was centrifuged 40 min. at 3000 rpm. The supernatant was discharged and the precipitate dissolved in 5 ml of 10 mM HEPES, 0,1M NaCl, pH 7.85. This APC solution was dialysed with gentle stirring in the dark for 2 days against 10 mM HEPES, 0.1M NaCl, pH 7.85 (20 fold excess volume) at 2-8°C with 1 buffer change/day.

3. The APC-solution was concentrated to 1 ml and the concentration measured to 47 g/L at UV 650nm. The A650/A278-ratio was measured to 4.2.

4. The SA-solution was filtrated through a 0.45 $\mu$m filter and the protein concentration was measured to 61.8 g SA/L at UV 278nm .

5. Conjugation: The reagents was mixed to a total volume of 500 $\mu$l in the following order with 8.1 mol SA/mol Dex and 27 mol APC/mol Dex.:

a) 90 $\mu$l water
b) 160 $\mu$l activated VS-dex270
c) 23 $\mu$l SA (61,8 g/L) ~ 8.1 equivalents,
d) 177 $\mu$l APC (47 g/L) ~ 27 equivalents,
e) 50 $\mu$l of 100 mM HEPES, 1M NaCl, pH 8

The reaction was placed in a water bath with stirring at 30°C in the dark for 18 hours.

6. The coupling was stopped by adding 50 $\mu$l 0,1M ethanolamine, pH 8.0.

7. The conjugate was purified on a Sephacryl S-200 column with 10 mM HEPES, 0,1M NaCl buffer, pH 7.2.

8. 3 peaks were collected (peak 1: APC-SA-dex270; peak 2: Free APC; peak 3: Free SA). Volume, UV A650 and UV A278 were measured.

9. The concentration of dextran270, APC/Dex and SA/Dex were calculated to $22.4 \times 10^{-8}$ M; 3.48 and 9.54 respectively.

10. The conjugate were added $NaN_3$ and BSA to a final concentration of 15 mM and 1% respectively. The volume was adjusted with 10 mM HEPES, 0.1 M NaCl, pH 7.2 to a final concentration of $16 \times 10^{-8}$ M Dex270.

11. The conjugate were kept at 2-8°C in dark until further use.

## Example 7

This is and example of generation of a multimerization domain. The multimerization domain is an activated divinylsylfone-dextran(270kDa)(VS-dex270) was coupled with streptavidin (SA) and the label is R-phycoerythrin (RPE).

The coupling procedure described for coupling of SA and APC to VS-dex270 (as described elsewhere herein) were followed with the exception that APC were replaced with RPE

## Example 8

This is an example of how to couple MHC-peptide complexes to a multimerization domain, where the multimerization domain is dextran.

This example describes how to couple an empty MHC or a MHC-complex to a dextran multimerization domain through a non-covalent coupling, to generate a MHC-dextramer. The MHC-dextramer in this example consisted of APC-streptavidin (APC-SA)-conjugated 270kDA dextran and a biotinylated, folded MHC-complex composed of $\beta$2m, HLA-A*0201 heavy chain and the peptide NLVPMVATV (SEQ ID NO 217779). The APC-SA conjugated 270kDA dextran contained 3,7 molecules of SA per dextran (each SA can bind 3 MHC-complexes) and the concentration was $16 \times 10^{-8}$ M. The concentration of the HLA-A*0201/ NLVPMVATV-complex (SEQ ID NO 217779) was 4 mg/ml (1 $\mu$g = 20,663 pmol). The molecular concentration of the MHC-complex was 8,27x10$^{-5}$M.

The MHC-complex was attached to the dextran by a non-covalent Biotin-Streptavidin interaction between the biotinylated Heavy Chain part of the MHC-complex and the SA, conjugated to dextran.

Here follows a protocol for how to produce 1000 µl of a MHC-dextramer solution with a final concentration of approximately 32x10$^{-9}$M :

1. 200 µL 270 kDA vinylsulfone-activated dextran, corresponding to 3,2x10$^{-11}$ mol, and 4 µl MHC-complex, corresponding to 3,55x10$^{-10}$ mol was mixed and incubated at room temperature in the dark for 30 min.
2. A buffer of 0,05M Tris-HCl, 15 mM NaN$_3$, 1% BSA, pH 7,2 was added to a total volume of 1000 µl.
3. The resulting MHC-dextramer preparation may now be used in flow cytometry eksperiments.

**Example 9**

This is an example of how to make and use MHC multimers that are trimers consisting of a streptavidin multimerization domain with 3 biotinylated MHC complexs and 1 flourophore molecule attached to the biotin binding pockets of streptavidin.

MHC complexes consisting of HLA-A*0201 heavy chain, beta2microglobulin and NLVPMVATV (SEQ ID NO 217779) peptide or the negative control peptide GLAGDVSAV (SEQ ID NO 217778) were generated as described elsewhere herein. The fluorophore in this example was Fluorescein-linker molecules as shown in figure 9. Each of these molecules consist of a linker-biotin molecule mounted with 4 trippel fluorescein-linker molecules. The linker-biotin molecule was here H-L30-Lys(NH$_2$)-L30-Lys(NH$_2$)-L30-Lys(NH$_2$)L300Lys(caproylamidobiotin)-NH$_2$ where L30 was a 30 atom large linker and L300 was a 300 atom large linker. Both L30 and L300 was composed of multiple L15 linkers with the structure shown in figure 9B. Linker-biotin molecules were generated as follows: Downloaded Boc-L300-Lys(Fmoc) resin (100 mg) was deprotected and subjected to coupling with Boc-Lys(2CIZ)-OH, Boc-L30-OH, Boc-Lys(2CIZ)-OH, Boc-L30-OH, Boc-Lys(2CIZ)-OH then Boc-L30-OH. The resin was Fmoc deprotected and reacted twice (2x 2 h) with caproylamido biotin NHS ester (25 mg in 0.5 mL NMP + 25 microL DIPEA). The resin was washed with TFA and the product cleaved off with TFA:TFMSA:mCresol:thioanisol (6:2:1:1), 1 mL, precipitated with diethyl ether and purified by RP-HPLC. MS calculated for C$_{300}$H$_{544}$N$_{64}$O$_{137}$S is 7272.009 Da, found 7271.19 Da.

Alternatively linker-biotin molecule was H-L60-Lys(NH$_2$)-L60-Lys(NH$_2$)-L60-Lys(NH$_2$)L300Lys(caproylamidobiotin)-NH$_2$ and made from downloaded Boc-L300-Lys(Fmoc) resin (100 mg), and then prepared analogously to H-L30-Lys(NH$_2$)-L30-Lys(NH$_2$)-L30-Lys(NH$_2$)L300Lys(caproylamidobiotin)-NH$_2$. MS calculated for C$_{360}$H$_{652}$N$_{76}$O$_{167}$S is 8749.5848 Da and was found to be 7271.19 Da. Yield 3 mg.

The trippel fluorescein-linker molecules was here betaalanin-L90-Lys(Flu)-L90-Lys(Flu)-L90-Lys(Flu)-NH$_2$ where Lys = Lysine, Flu = Fluorescein and L90 is a 90 atom linker (se figure 9 for further details). The trippel-fluorescein-linker molecule was generated as follows: Downloaded Boc-Lys(Fmoc) resin, 2 g,was Boc deprotected and subjected to 3 x coupling with Boc-L30-OH, Boc-Lys(Fmoc)-OH, 3 x Boc-L30-OH, Boc-Lys(Fmoc)-OH, 3 x Boc-L30-OH. The three Fmoc groups were removed and carboxyfluorescein, 301 mg, activated with HATU, 274 mg, and DIPEA, 139 µL, in 8 mL NMP, was added to the resin twice for 30 min. The resin was Boc deprotected and subjected to 2 x 30 min coupling with beta-alanine-N,N-diacetic acid benzyl ester, followed by 5 min treatment with 20 % piperidine in NMP. The resin was washed with DCM, then TFA and the product was cleaved off the resin, precipitated with diethyl ether and purified by RP-HPLC. Yield was 621 mg. MS calculated for C268H402N44O116 is 6096.384 Da, while MS found was 6096 Da.

Biotin-linker molecule were coupled together with 4 trippel fluorescein-linker molecules as follows: (500 nmol) was dissolved in 88 microliter NMP + 2 µl pyridine and activated for 10 min at room temperature (conversion to cyclic anhydride) by addition of 10 µl N,N' diisopropylcarbodiimide. Following activation the trippel fluorescein-linker was precipitated with diethyl ether and redissolved in 100 microliter NMP containing 10 nmol biotin-linker. Once dissolved the coupling was initiated by addition of 5 µl diisopropyl ethyl amine, and was complete after 30 min.

Streptavidin and Fluorescein-linker molecules are then mixed in a molar ration of 1:1 and incubated for ½ hour. Then MHC complexes are added in 3-fold molar excess in respect to streptavidin and incubated for another ½ hour. Alternatively, MHC complexes are added first, then Fluorescein-linker molecules or MHC complexes are mixed with Fluorescein-linker molecules before addition to Streptavidin.

These MHC multimers are then used to stain CMV specific T cells in a flow Cytometry experiment. 1x10$^6$ purified HPBMC from a donor with T cells specific for HLA-A*0201 (NLVPMVATV) (SEQ ID NO 217779) are incubated with 10 µl of each of the two HLA-A*0201(peptide)/Fluorescein constructs described above for 10 minutes in the dark at room temperature with a cell concentration of 2x10$^7$ cells/ml. 10 µl of mouse-anti-human CD8/PB (clone DK25 from Dako) are added and the incubation continued for another 20 minutes at 4°C in the dark. The samples are then washed by adding 2 ml PBS; pH =7.2 followed by centrifugation for 5 minutes at 200xg and the supernatant removed. The cells are resuspended in 400-500 µl PBS; pH=7.2 and analyzed on a flowcytometer.

In the above described example the Fluorescein-linker is as shown in figure 9 but the linker molecule can be any linker molecule as described in patent application WO 2007/015168 A2 (Lohse (2007)) or alternatively chemical biotinylated fluorochrom can be used instead of Fluorescein-linker molecules. The MHC complexes described in this example is a MHC I molecule composed of HLA-A*0201 heavy chain, beta2microglobulin and NLVPMVATV (SEQ ID NO 217779) peptide but can in principle be any MHC complex or MHC like molecule as described elsewhere herein.

## Example 10

This is an example of how to make and use MHC multimers that are trimers .

This is an example of how to make MHC multimers consisting of a streptavidin multimerization domain with 3 biotinylated MHC complexs attached to the biotin binding pockets of streptavidin and how to use such trimer MHC complexs to detect specific T cells by direct detection of individual cells in a flow cytometry experiment by addition of a biotinylated flourophore molecule. In this example the fluorophore is Fluorescein linker molecules constructed as described elsewhere herein. MHC complexs consisting of HLA-A*0201 heavy chain, beta2microglobulin and peptide are generated as described elsewhere. MHC complexs are incubated with streptavidin in a molar ratio of 3:1 for ½ hour.

These trimer MHC multimers are then used to stain CMV specific T cells in a flow Cytometry experiment. $1x10^6$ purified HPBMC from a donor with T cells specific for HLA-A*0201(NLVPMVATV) (SEQ ID NO 217779) are incubated with 10 $\mu$l HLA-A*0201 (peptide) multimer construct for 10 minutes in the dark at room temperature with a cell concentration of $2x10^7$ cells/ml. Then Fluorescein linker molecules (as described in Example 9) are added and incubation continued for 5 minutes. 10 $\mu$l mouse-anti-human CD8/PB antibody (clone DK25 from Dako) is added and the incubation continued for another 20 minutes at 4°C in the dark. The samples are then washed by addition of 2 ml PBS; pH =7.2 followed by centrifugation for 5 minutes at 200xg and the supernatant removed. Cells are resuspended in 400-500 $\mu$l PBS; pH=7.2 and analyzed on a flowcytometer.

In this example the Fluorescein-linker is as shown in figure 9 but the linker molecule can be any linker molecule as described in Lohse, Jesper, (2007), WO 2007/015168 A2 or alternative chemically biotinylated fluorochrome may be used. The MHC complexse described in this example is a MHC I molecule composed of HLA-A*0201 heavy chain, beta2microglobulin and NLVPMVATV (SEQ ID NO 217779) peptide but can in principle be any MHC complex or MHC like molecule as described elsewhere herein.

## Example 11

This is an example of how to make MHC multimers where the multimerization domain is dextran and the MHC complexs are chemically conjugated to the dextran multimerization domain.

MHC complexs consisting of HLA-A*0201 heavy chain, beta2microglobulin and NLVPMVATV (SEQ ID NO 217779) peptide or the negative control peptide GLAGDVSAV (SEQ ID NO 217778) are generated as described elsewhere herein.

Dextran with a molecular weight of 270 kDa is activated with divinylsulfone. Activated Dextran is then incubated with MHC and RPE in a 0.05 M $NaCHO_3$ buffer; pH = 9.5 with a molar ratio between MHC and Dextran of 30-60 and a molar ratio between RPE and dextran of 3-7 :1 The mixture is placed in a water bath at 30°C for 16 hours. Excess flourochrome, MHC and dextran are removed by FPLC using a sephacryl S-300 column.

These MHC/RPE dextramers are then used to stain CMV specific T cells in a flow Cytometry experiment. Briefly, $1x10^6$ purified HPBMC from a donor with T cells specific for HLA-A*0201(NLVPMVATV) (SEQ ID NO 217779) are incubated with 10 $\mu$l of each of the two HLA-A*0201 (peptide)/RPE constructs described above for 10 minutes in the dark at room temperature with a cell concentration of $2x10^7$ cells/ml. 10 $\mu$l mouse-anti-human CD8/PB antibody (clone DK25 from Dako) are added and the incubation continued for another 20 minutes at 4°C in the dark. The samples are then washed by adding 2 ml PBS; pH =7.2 followed by centrifugation for 5 minutes at 200xg and the supernatant removed. The cells are then resuspended in 400-500 $\mu$l PBS; pH=7.2 and analyzed on a flow cytometer.

## Example 12

This is an example of how to make MHC multimers where the multimerization domain is dextran and MHC complexs are MHC I molecules chemically conjugated to dextran multimerization domain and the dextran multimerization domain also have fluorochrome chemically coupled.

Human beta2microglobulin is coupled to dextran as follows. Dextran with a molecular weight of 270 kDa is activated with divinylsulfone. Activated dextran is incubated with human beta2microglobulin and RPE in a 0.05 M $NaCHO_3$ buffer; pH = 9.5 with a molar ratio between beta2microglobulin and Dextran of 30-60 and a molar ratio between RPE and dextran of 3-7:1. The molar ratio of the final product is preferable 4-6 RPE and 15-24 beta2microglobulin per dextran. The mixture is placed in a water bath at 30°C for 16 hours. Excess flourochrome, beta2microglobulin and dextran are removed by FPLC using a sephacryl S-300 column. The beta2microglobulin-RPE-dextran construct is then refolded in vitro together with heavy chain and peptide using the following procedure. 200 ml refolding buffer (100 mM Tris, 400 mM L-arginin-HCL, 2 mM NaEDTA, 0,5 mM oxidized Gluthathione, 5 mM reduced Glutathione, pH 8.0) supplied with protease inhibitors PMSF, Pepstatin A and Leupeptin (to a final concentration of 1 mM, 1 mg/l and 1 mg/l, respectively) is made and cooled to 10°C. 12 mg NLVPMVATV (SEQ ID NO 217779) peptide is dissolved in DMSO and added to the refolding buffer together with 20-30 mg beta2microglobulin-RPE-dex and 6 mg HLA-A*0201 heavy chain. Incubation at 10°C for 4-8 hours, then 20-30 mg beta2microglobulin-RPE-dex and 6 mg HLA-A*0201 heavy chain is added and incubation continued for 4-8 hours. Another 20-30 mg beta2microglobulin-RPE-dex and 6 mg HLA-A*0201 heavy chain is added and incubation continued for 6-8 hours. The folding reaction is filtrated through a 0,2 $\mu$m filter to remove larger aggregates and then buffer exchanged into a buffer containing 20 mM Tris-HCl, 50 nM NaCl; pH = 8.0 followed by concentration to 1-2 ml sample. Dextran-RPE-MHC complexs are then separated from excess heavy chain and peptide by size exclusion chromatography using a sephacryl S-300, S-400 or sephacryl S-500 column.

These MHC/RPE dextramers may be used to stain CMV specific T cells in a flow Cytometry experiment. Briefly, $1 \times 10^6$ purified HPBMC from a donor with T cells specific for HLA-A*0201(NLVPMVATV) (SEQ ID NO 217779) are incubated with 10 $\mu$l of each of the two HLA-A*0201 (peptide)/RPE constructs described above for 10 minutes in the dark at room temperature with a cell concentration of $2 \times 10^7$ cells/ml. 10 $\mu$l of mouse-anti-human CD8/PB antibody (clone DK25 from Dako) are added and the incubation continued for another 20 minutes at 4°C in the dark. The samples are then washed by adding 2 ml PBS; pH =7.2 followed by centrifugation for 5 minutes at 200xg and the supernatant removed. The cells are then resuspended in 400-500 $\mu$l PBS; pH=7.2 and analyzed on a flowcytometer.

**Example 13**

The preparation of a Pentamer is described in e.g. (United States Patent application 20040209295). Briefly, the following steps lead to a fluorescent Pentamer reagent:

The following is a detailed example for cloning, expressing, and purifying a pentameric class I MHC complex, which comprises a chimeric fusion of .beta.2m with COMP. The chimeric .beta.2m-COMP protein is expressed in insoluble inclusion bodies in E. coli and subsequently assembled as pentameric .beta.2m-COMP in vitro. The pentameric class I MHC peptide complex is then formed in a second refolding reaction by combining .beta.2m-COMP pentamers and the human MHC class I .alpha. molecule known as HLA-A*0201, in the presence of an appropriate synthetic binding peptide representing the T cell antigen. In this example, a well characterized antigen derived from Epstein-Barr virus BMLF1 protein, GLCTLVAML (SEQ ID NO 217782) (a.a. 289-297), is used. The resultant complex is labelled with a fluorescent entity and used as a staining reagent for detecting antigen-specific T cells from a mixed lymphocyte population, in a flow cytometry application.

The strategy involves the sequential cloning into pET-24c vector of .beta.2m, yielding a construct referred to as pETBMC01, followed by the insertion of the oligomerisation domain of cartilage oligomeric matrix protein (COMP) with a biotin acceptor sequence (BP) for site-specific biotinylation with the biotin-protein ligase BirA, yielding a construct referred to as pETBMC02. Thirdly a polyglycine linker is cloned in between .beta.2m and COMP, yielding a construct referred to as pETBMC03, and finally, a serine-residue is removed by site-directed mutagenesis, which serine residue precedes the poly-glycine linker, to give the final .beta.2m-COMP/pET-24c construct, referred to as pETBMC04 (see also FIG. 3). Removal of the serine residue is carried out to avoid steric hindrance when the .beta.2m molecule is associated with the MHC class I chain protein.

The extracellular portion of .beta.2m comprises of 99 amino acids (equivalent to Ile1-Met99 of the mature protein) encoded by 74 bp-370 bp of the DNA sequence. This region of the .beta.2m sequence is amplified from a normal human lymphocyte cDNA library, by polymerase chain reaction (PCR)

beta.2m PCR product is purified from the above reaction mix using a QIAquick.RTM. PCR purification kit according to the manufacturer's instructions (Qiagen). 200 ng of purified PCR product and 1 .mu.g pET-24c vector (Novagen) are each digested with BamH I (10 U) and Nde I (10 U) restriction enzymes (New England Biolabs, NEB) for 4 h at 37.degree. C., in accordance with the manufacturer's instructions, and purified.

The gel-purified insert and vector DNA are ligated at a 1:3 molar ratio (vector:insert, 50 ng: 7.5 ng) using T4 DNA ligase (5 U; Bioline), in T4 DNA ligase buffer (as supplied) for 16 hrs at 16.degree. C.

The ligation mixtures and appropriate controls are subsequently transformed into XL1-Blue strain competent E. coli cells, according to the manufacturer's instructions (Stratagene). Successful transformants are selected by plating the cells on Luria-Bertani (LB) agar plates containing 30 .mu.g/ml kanamycin, and incubating overnight at 37.degree. C.

A selection of single colonies from the bacterial transformation plates are screened by PCR with T7 promoter [SEQ ID NO: 4] (1 .mu.M) and T7 terminator [SEQ ID NO: 5] (1 .mu.M) primers (Sigma Genosys, see Appendix I for primer sequences), which are complementary to regions of the pET vector flanking the cloning site. Amplification is carried out using Taq DNA polymerase (1 U, Bioline) in Taq reaction buffer (as supplied), supplemented with 2 mM MgSO.sub.4 and 0.2 mM dNTPs, using 25 thermal-cycling reactions as detailed above. Successful transformants generated a DNA fragment of approximately 500 bp, ascertained by 1.5% agarose gel electrophoresis.

Bacterial transformants that generated the correct size of PCR products are inoculated into 6 ml of sterile LB-kanamycin medium and incubated overnight at 37.degree. C. with 200 rpm shaking. pETBMC01 plasmid DNA is recovered from the bacterial cultures using a QIAprep.RTM. Spin Mini-prep kit according to the manufacturer's instructions (Qiagen). The presence of the .beta.2m fragment in these plasmids is further verified by automated DNA sequencing.

The sequence of the oligomerisation domain of COMP is obtained from the Genbank database (accession #1705995) and a region encoding the coiled-coil domain (amino acids 21-85) is selected based on self-association experiments of COMP (Efinov et al., FEBS Letters 341:54-58 (1994)). A biotin acceptor sequence 'BP': SLNDIFEAQKIEWHE [SEQ ID NO: 6] is incorporated at the C terminus and an additional 14 amino acid linker, PQPQPKPQPKPEPET [SEQ ID NO:7] is included to provide a physical separation between the COMP oligomerising domain and BP.

The whole region is synthesized using the overlapping complementary oligonucleotides, and purified COMP-BP and 1 .mu.g pETBMC01 vector are digested for 4 hrs at 37.degree. C. using Hind III (10 U) and Xho I (10 U) restriction enzymes (NEB), as described in Section 1.1. The digestion products are purified, ligated, transformed and PCR screened as in Section 1.1. Plasmids positive from the screen are purified and sequenced as described in Section 1.1.

The poly-glycine linker is synthesized by annealing overlapping oligonucleotides. Since the nucleotide sequence of the polyGlycine linker only incorporates the 5' overhang of the cut BamH I restriction site, and the 3' overhang of the cut Hind III nucleotide recognition motifs, there is no need to digest the annealed product to produce the complementary single-stranded overhangs suitable for subsequent ligation. The oligonucleotides are phosphorylated and annealed as described in Section 1.2.

pETBMC02 is digested with BamH I (10 U) and Hind III (10 U). Ligation of the annealed poly-glycine linker into pETBMC02 was as described previously (Section 1.1), assuming 96 fmoles of annealed oligonucleotide/.mu.l. The transformation and PCR-screening reactions are as described in Section 1.1, but in addition, the presence of an inserted linker is verified by a restriction enzyme digestion of the PCR screen product to ascertain the presence or absence of a Sal I restriction site. Successful transformants are not susceptible to Sal I digestion, given the removal of the site from the plasmid vector backbone. Purification of pETBMC03 and automated sequencing is as described in Section 1.1.

Analysis of X-ray crystallography models of MHC class I molecules reveal that the C terminus of .beta.2m closely abuts the .alpha.3 domain of the .alpha. chain. It is therefore desirable to achieve maximum flexibility at the start of the poly-glycine linker.

The extracellular portion of HLA-A*0201 .alpha. chain (EMBL M84379) comprises of 276 amino acids (equivalent to Gly1-Pro276 of the mature protein) encoded by bases 73-900 of the messenger RNA sequence. This region of the A*0201 sequence is amplified from a normal human lymphocyte cDNA library by PCR, using the primers A2S#1 [SEQ ID NO: 20] and A2S#2 [SEQ ID NO: 21] which incorporated NcoI and BamHI restriction sites respectively. The procedure for cloning the A*0201 insert into Nco I/BamH I-digested pET-11d vector (Novagen) is essentially as described for .beta.2m in Section 1.1.

An identical procedure is carried out to produce either .beta.2m-COMP or A*0201 .alpha. chain proteins. Plasmid DNA is transformed into an E. coli expression host strain in preparation for a large scale bacterial prep. Protein is produced as insoluble inclusion bodies within the bacterial cells, and is recovered by sonication. Purified inclusion bodies are solubilised in denaturing buffer and stored at -80.degree. C. until required.

Purified plasmid DNA is transformed into the BL21 (DE3)pLysS E. coli strain, which carries a chromosomal copy of the T7 RNA polymerase required to drive protein expression from pET-based constructs. Transformations into BL21 (DE3)pLysS competent cells (Stratagene) are carried out with appropriate controls.

A single bacterial transformant colony is innoculated into 60 ml sterile LB medium, containing appropriate antibiotics for selection, and left to stand overnight in a warm room (.about.24.degree. C.) The resulting overnight culture is added to 6 litres of LB and grown at 37.degree. C. with shaking (.about.240 rpm), up to mid-log phase (OD.sub.600=0.3-0.4). Protein expression is induced at this stage by addition of 1.0 ml of 1M IPTG to each flask. The cultures are left for a further 4 h at 37.degree. C. with shaking, after which the cells are harvested by centrifugation and the supernatant discarded.

The bacterial cell pellet is resuspended in ice-cold balanced salt solution and sonicated (XL series sonicator; Misonix Inc., USA) in a small glass beaker on ice in order to lyse the cells and release the protein inclusion bodies. Once the cells are completely lysed the inclusion bodies are spun down in 50 ml polycarbonate Oak Ridge centrifuge tubes in a Beckman high-speed centrifuge (J2 series) at 15,000 rpm for 10 min. The inclusion bodies are then washed three times in chilled Triton.RTM. wash This is followed by a final wash in detergent-free wash buffer.

The resultant purified protein preparation is solubilised in 20-50 ml of 8 M urea buffer, containing 50 mM MES, pH 6.5, 0.1 mM EDTA and 1 mM DTT, and left on an end-over-end rotator overnight at 4.degree. C. Insoluble particles are removed by centrifugation and the protein yield is determined using Bradford's protein assay reagent (Bio-Rad Laboratories) and by comparison with known standards. Urea-solubilised protein is dispensed in 10 mg aliquots and stored at -80.degree. C. for future use.

Assembly of .beta.2m-COMP from the urea-solubilised inclusion bodies is performed by diluting the protein into 20 mM CAPS buffer, pH 11.0, containing 0.2 M sodium chloride and 1 mM EDTA, to give a final protein concentration of 1.5 mg/ml. The protein is oxidised at room temperature by addition of oxidised and reduced glutathione to final concentrations of 20 mM and 2 mM, respectively. Following an overnight incubation, disulphide bond formation is analysed by non-reducing SDS-PAGE on 10% bis-tricine gels (Invitrogen).

The protein mixture is subsequently buffer exchanged into 20 mM Tris, pH 8.0, 50 mM sodium chloride ('S200 buffer'), and concentrated to a final volume of 4.5 ml, in preparation for enzymatic biotinylation with BirA (Affinity, Denver, Colo.). 0.5 ml of 10.times. BirA reaction buffer (as supplied) is added, and recombinant BirA enzyme at 10 .mu.M final concentration, supplemented with 10 mM ATP, pH 7.0. A selection of protease inhibitors is also used to preserve the proteins: 0.2 mM PMSF, 2 .mu.g/ml pepstatin and 2 .mu.g/ml leupeptin. The reaction is left for 4 hours at room temperature.

Biotinylated .beta.2m-COMP is purified by size exclusion chromatography (SEC) on a Superdex.RTM.200 HR 26/60 column (Amersham Biosciences), running S200 buffer.

500 ml of refolding buffer is prepared as follows: 100 mM Tris, pH 8.0, 400 mM Larginine hydrochloride, 2 mM EDTA, 5 mM reduced glutathione and 0.5 mM oxidised glutathione, dissolved in deionised water and left stirring at 4.degree. C. 15 mg of lyophilised synthetic peptide GLCTLVAML (SEQ ID NO 217782) is dissolved in 0.5 ml dimethylsulfoxide and added to the refolding buffer whilst stirring. 50 mg of biotinylated pentameric .beta.2m-COMP and 30 mg of A*0201 .alpha. chain is added sequentially, injected through a 23gauge hypodermic needle directly into the vigorously-stirred buffer, to ensure adequate dispersion. The refolding mixture is then left stirring gently for 16 hours at 4.degree. C.

The protein refolding mixture is subsequently concentrated from 500 ml to 20 ml using a MiniKros hollow fibre ultrafiltration cartridge (Spectrum Labs, Rancho Dominguez, Calif.) with a 30 kD molecular weight cutoff. Further concentration of the complex from 20 ml to 5 ml is carried out in Centricon Plus-20 centrifugal concentrators (30 kD molecular weight cut-off) according to the manufacturers instructions, followed by buffer exchange into S200 buffer using disposable PD10 desalting columns (Amersham Biosciences), according to the manufacturer's instructions. Final volume is 7.5 ml. The concentrated protein refold mixture is first purified by SEC on a Superdex.RTM. 200 HR 26/60 gel filtration chromatography column, as in Section 4.2. Fractions containing protein complexes in the region of 310 kD is collected.

Fractions collected from SEC are pooled and subjected to further purification by anion exchange chromatography on a MonoQ.RTM. HR 5/5 column (Amersham Biosciences), running a salt gradient from 0-0.5 M sodium chloride in 20 mM Tris over 15 column volumes. The dominant peak is collected. Protein recovery is determined using the Bradford assay.

Since each streptavidin molecule is able to bind up to 4 biotin entities, final labelling with phycoerythrin (PE)-conjugated streptavidin is carried out in a molar ratio of 1:0.8, streptavidin to biotinylated pentamer complex respectively, taking into account the initial biotinylation efficiency measurement made for .beta.2m-COMP in Section 4.2. The total required amount of pentamer complex is subdivided (e.g. into 5 equal amounts) and titrated successively into streptavidin-PE. The concentration of A*0201 pentamer-streptavidin complex is adjusted to 1 mg/ml with phosphate buffered saline (PBS), supplemented with 0.01% azide and 1% BSA.

This resultant fluorescent Pentamer reagent is stored at 4.degree. C.

**Example 14**

**Prediction of MHC class 1 peptide binders for human cancer protein BclX(L) using directed approach**

This example describes the directed approach, applied to a known protein sequence, the cancer protein BclX(L) encoded by the human genome. The purpose is to predict BclX(L) peptide sequences that binds to MHC class 1 molecules for use in construction of MHC'mers designed to be used for analytical, diagnostic, prognostic, therapeutic and vaccine purposes, through the interaction of the MHC'mers with human BclX(L) specific T-cells. Prediction is carried out using the known preferences of the 42 HLA class 1 alleles included in the http://www.cbs.dtu.dk/services/NetMHC/ database (figure 10).

The result of the prediction software is used to find all strong and weak 8-, 9-, 10- and 11-mer peptide binders of the 42 HLA class 1 alleles. The result can be seen in figure 36. The MHC class 1 alleles for whom no binders are predicted are omitted from the list. The listed peptides are ranked according to decreased binding affinity for the individual MHC alleles. Strong binders are defined as binders with an affinity value of less than 50 nM and weak binders with a value of less than 500nM. Only peptides defined as weak or strong binders are shown.

**Example 15**

**Prediction of MHC class 2 peptide binders for human cancer protein BclX(L) using directed approach**

This example describes the directed approach, applied to a known protein sequence, the cancer protein BclX(L) encoded by the human genome. The purpose is to predict BclX(L) peptide sequences that binds to MHC class 2 molecules for use in construction of MHC'mers designed to be used for analytical, diagnostic, prognostic, therapeutic and vaccine purposes, through the interaction of the MHC'mers with human BclX(L) specific T-cells. Prediction is carried out using the known preferences of the 14 HLA class 2 alleles included in the http://www.cbs.dtu.dk/services/NetMHCII/ database (figure 10).

The result of the prediction software is used to find all strong and weak 15-mer peptide binders of the 14 HLA class 2 alleles. It also finds the important central nonamer core peptide sequence of each binding peptide. The result can be seen in figure 37. The MHC class 2 alleles for whom no binders are predicted are omitted from the list. The listed peptides are ranked according to decreased binding affinity for the individual MHC alleles. Strong binders are defined as binders with an affinity value of less than 50 nM and weak binders with a value of less than 500nM. Only peptides defined as weak or strong binders are shown.

## Example 16

### Prediction of MHC class 1 and 2 Borrelia afzelii OspC peptide binders

This example describes the prediction of MHC class 1 and 2 *Borrelia afzelii OspC* peptide sequences for use in construction of MHC'mers designed to be used for analytical, diagnostic, prognostic, therapeutic and vaccine purposes, through the interaction of the MHC'mers with *Borrelia afzelii OspC*-specific T-cells. The amino acid sequence of Borrelia afzelii OspC protein was found and retrieved from NCBI protein database (http://www.ncbi.nlm.nih.gov). Prediction of the 8-, 9-, 10-, 11-, 13-, 14-, 15- and 16-mer peptide sequences are carried out using the peptide generation software program described in figure 2. The outcome is shown in table A.

## Example 17

### Prediction of MHC class 1 Borrelia burgdorferi OspA peptide binders

This example describes the directed approach, applied to a known protein sequence, the Borrelia burgdorferi protein OspA encoded by the Borrelia genome. The purpose was to predict OspA peptide sequences that binds to MHC class 1 molecules for use in construction of MHC'mers designed to be used for analytical, diagnostic, prognostic, therapeutic and vaccine purposes, through the interaction of the MHC'mers with human OspA specific T-cells. The amino acid sequence of Borrelia burgdorferi OspA protein was found and retrieved from NCBI protein database (http://www.ncbi.nlm.nih.gov) Prediction was carried out using the known preferences of the 42 HLA class 1 alleles included in the http://www.cbs.dtu.dk/services/NetMHC/ database (figure 10).

The result of the prediction software was used to find all strong and weak 8-, 9-, 10- and 11-mer peptide binders of the 42 HLA class 1 alleles. The result can be seen in table B. The MHC class 1 alleles for whom no binders are predicted are omitted from the list. The listed peptides are ranked according to decreased binding affinity for the individual MHC alleles. Strong binders are defined as binders with an affinity value of less than 50 nM and weak binders with a value of less than 500nM. Only peptides defined as weak or strong binders are shown.

## Example 18

### Prediction of MHC class 1 Borrelia garinii FlaB peptide binders

This example describes the directed approach, applied to a known protein sequence, the Borrelia garinii protein FlaB encoded by the Borrelia genome. The purpose was to predict FlaB peptide sequences that binds to MHC class 1 molecules for use in construction of MHC'mers designed to be used for analytical, diagnostic, prognostic, therapeutic and vaccine purposes, through the interaction of the MHC'mers with human FlaB specific T-cells. The amino acid sequence of Borrelia burgdorferi OspA protein was found and retrieved from NCBI protein database (http://www.ncbi.nlm.nih.gov). Prediction was carried out using the known preferences of the 42 HLA class 1 alleles included in the http://www.cbs.dtu.dk/services/NetMHC/ database (figure 10).

The result of the prediction software was used to find all strong and weak 8-, 9-, 10- and 11-mer peptide binders of the 42 HLA class 1 alleles. The result can be seen in table C. The MHC class 1 alleles for whom no binders are predicted are omitted from the list. The listed peptides are ranked according to decreased binding affinity for the individual MHC alleles. Strong binders are defined as binders with an affinity value of less than 50 nM and weak binders with a value of less than 500nM. Only peptides defined as weak or strong binders are shown.

**Example 19**

**Test of predicted BclX(L) 10-mer binding peptide functionality in ELISPOT**

In example 14 the best binding BclX(L) 10-mer peptide for HLA-A*0201 was identified to be YLNDHLEPWI. This peptide has then been tested in ELISPOT to see if it were able to detect the presence Bcl-X(L)-specific, CD8 positive T cells in PBL (Peripheral Blood Lymphocytes) from a breast cancer patient. PBL from a breast cancer patient was analyzed by ELISPOT ex vivo either with or without the Bcl-X(L)173-182 peptide (YLNDHLEPWII), 106 PBL/well in doublets. The number of spots was counted using the Immunospot Series 2.0 Analyzer (CTL Analysers). The result is given as number of spots above the pictures of the result as shown in Figure 11 and it clearly shows the presence of BclX(L) specific T-cells and thereby the functionality of the peptide as compared to the absence of added peptide.
*This example is from* Cancer Immunol Immunother Apr;56(4)527-33.

**Example 20**

**Test of predicted BclX(L) 10-mer binding peptide functionality in Flow cytometry**

In example 14 the best binding BclX(L) 10-mer peptide for HLA-A*0201 was identified to be YLNDHLEPWI. In the present example the functionality of the peptide is shown in a flow cytometric analysis of PBL from the patient was analyzed ex vivo by Flow cytometry to identify Bcl-X(L)173-182 specific CD8 T cells using the dextramer complex HLA-A2/Bcl-X(L)173-182-APC, 7-AAD-PerCP, CD3-FITC, and CD8-APC-Cy7. The dextramer complex HLA-A2/HIV-1 pol476-484-APC was used as negative control. The result (figure 12) clearly demonstrate that a MHC Dextramer HLA-A*0201/YLNRHLHTWI complex detects BclX(L) antigen-specific CD-8 cells in the patient sample at a level of 0.03% as compared with the negative control using HIV specific MHC Dextramer.
*This example is from* Cancer Immunol Immunother Apr;56(4)527-33.

**Example 21**

**Use of BclX(L) specific MHC Dextramer for sorting of antigen-specific CD8 T cells from patient sample**

The antigen-specific CD8 positive T-cells of example 20 were sorted out during the flow cytometric analysis using the MHC Dextramer HLA-A*0201/YLNDHLEPWI. The detectable population of dextramer positive CD8 T cells was sorted as single cells into 96 well plates using the following protocol:
Small lymphocytes were gated by forward and side scatter profile, before cloning according to CD8/MHC-multimer double staining. CD8/MHC-multimer double-positive cells were sorted as single cells into 96 well plates (Nunc) already containing $10^5$ cloning mix cells/well. The cloning mix was prepared containing $10^6$ irradiated (20 Gy) lymphocytes from three healthy donors per ml in X-vivo with 5% heat-inactivated human serum, 25 mM HEPES buffer (GibcoBRL), 1 $\mu$g/ml phytohemagglutinin (PHA) (Peprotech) and 120 U/ml IL-2. The cloning mix was incubated for two hours at 37°C/5 %$CO_2$ prior to cloning. After cloning, the plates were incubated at 37°C/5 %$CO_2$. Every 3 - 4 days 50 $\mu$l fresh media were added containing IL-2 to a final concentration of 120U/ml. Following 10 - 14 days of incubation, growing clones were further expanded using cloning mix cells. Consequently, each of the growing clones were transferred (split) into two or three wells (depending on the number of growing cells) of a new 96 well plate containing 5 x $10^4$ cloning mix cells/well. Clones that were not growing at this time were incubated for another week with IL-2, and then expanded. Subsequently, the specificity of the growing clones was tested in a $^{51}$Cr-release assay or by FACS.
Out of twenty isolated dextramer positive CD8 T cells, ten were able to be expanded into T-cell clones.
*This example is from* Cancer Immunol Immunother Apr,56(4)527-33.

**Example 22**

**Demonstration of specific cytolytic activity of isolated BclX(L) specific CD8 T-cells.**

This is an example of a CTL killing assay.
The ten expanded T cell clones isolated by Flow sorting as shown in example 21 were tested for their specificity by analysis in a standard 51-Cr release assay. For this purpose, T2 cells loaded with either Bcl-X(L)173-182 peptide or an irrelevant peptide (BA4697-105, GLQHWVPEL (SEQ ID NO 217777)) were used as target cells. Five CD8 T-cell clones (Clone 8, 9, 10, 11, and 12) effectively lysed T2 cells pulsed with Bcl-X(L)173-182 without killing of T2 cells pulsed with an irrelevant peptide (Figure 13). One of these BclX(L)173-182 specific CD8 T-cell clones [Clone 9] were expanded for further analyses. The remaining five expanded clones (Clone 7, 13, 15, 17, and 18) did not show specific lysis against T2 cells pulsed with Bcl-X(L)173-182 peptide (figure 13).

*This example is from* Cancer Immunol Immunother Apr;56(4)527-33.

## Example 23

**Demonstration of the cytotoxic capacity of a BclX(L)173-182 specific CD8 T cell clone isolated by flow aided sorting of antigen (HLA-A\*0201/YLNRHLHTWI) specific T cells.**

The Bcl-X(L)173-182 specific clone 9 from example 22 was expanded for additional 2 weeks before the cytotoxic potential was examined further in 51 Cr-release assays. Two assays were performed **a** Cell lysis of T2 cells pulsed with Bcl-X(L)173-182 peptide or an irrelevant peptide (BA4697-105, GLQHWVPEL (SEQ ID NO 217777)) in three E:T ratios. **b** Cell lysis of T2 cells pulsed with different concentrations of Bcl-X(L)173-182 peptide at the E:T ratio 1:1 The result is given in figure 14. As can be seen the presence of the specific peptide is necessary to get killing of the target cell and the effect of the peptide is significant even at low concentrations.

*This example is from* Cancer Immunol Immunother Apr;56(4)527-33.

## Example 24

**Synthesis of a comprehensive library of antigenic peptides of variable size derived from a full-length antigen sequence.**

In this example it is described how virtually all of the possible 8'- to 20'-mer peptide epitopes of an antigen may be synthetically prepared by modification of the standard Fmoc peptide synthesis protocol.
N-⟨-amino acids are incorporated into a peptide of the desired sequence with one end of the sequence remaining attached to a solid support matrix. All soluble reagents can be removed from the peptide-solid support matrix by filtration and washed away at the end of each coupling step. After each of the coupling steps, and after the removal of reagents, a fraction of the generated peptides are removed and recovered from the polymeric support by cleavage of the cleavable linker that links the growing peptide to solid support.
The solid support can be a synthetic polymer that bears reactive groups such as -OH. These groups are made so that they can react easily with the carboxyl group of an N-⟨-protected amino acid, thereby covalently binding it to the polymer. The amino protecting group can then be removed and a second N-⟨-protected amino acid can be coupled to the attached amino acid. These steps are repeated until the desired sequence is obtained. At the end of the synthesis, a different reagent is applied to cleave the bond between the C-terminal amino acid and the polymer support; the peptide then goes into solution and can be obtained from the solution.

Initially, the first Fmoc amino acid (starting at the C-terminal end of the antigen sequence) is coupled to a precursor molecule on an insoluble support resin via an acid labile linker. Deprotection of Fmoc is accomplished by treatment of the amino acid with a base, usually piperidine. Before coupling the next amino acid, a fraction of the synthesized peptide (for example 0.1%) is detached from the solid support, and recovered. Then additional beads carrying only the precursor molecule including the linker (for example corresponding to 0.1% of the total amount of solid support in the reaction) is added. Then the next Fmoc amino acid is coupled utilizing a pre-activated species or in situ activation.
This cycle of amino acid coupling, removal of reagents, detachment of a small fraction of synthesized peptide and recovery of these, and activation of the immobilized peptide to prepare for the next round of coupling, goes on until the entire antigen sequence has been processed.
The recovered peptides thus represent different fragments of the antigen, with varying lengths. The peptide pool thus contains most or all of the possible peptide epitopes of the antigen, and may be used in the preparation of MHC multimers as a pool.

The entire process, including the detachment of a fraction of the peptides after each round of coupling, follows standard Fmoc peptide synthesis protocols, and involves weak acids such as TFA or TMSBr, typical scavengers such as thiol compounds, phenol and water, and involves standard protecting groups.

## Example 25

This is an example of how MHC multimers may be used for diagnosis of Lyme Disease in blood samples from humans infected with Borrelia bacteria.
In this example the MHC multimer used are MHC complexes coupled to fluorophor-labelled dextran (Dextramers). The dextramers are used for direct detection of TCR in flow Cytometry. The antigen origin is Borrelia, thus, immune monitoring of a Borrelia infection.

Lyme disease is caused by infection by Borrelia bacteria. During acute infection Borrelia specific activated T cells will be present in increased amounts in an activated state compared to healthy individuals. The presences of an increased amount of activated Borrelia specific T cells may thereby act as a surrogate marker for infection with Borrelia bacterium. MHC multimers carrying borrelia specific antigenic peptides is in this example used to detect the presence of Borrelia specific T cells in the blood of patients infected with Borrelia.

Purified MHC-peptide complexes consisting of HLA-A*0201 heavy chain, human beta2microglobulin and peptide derived from regions in Outer surface protein A (Table B) or Flagellin B (Table C) conserved among the three species Borrelia Burgdorferi, Borrelia Garinii and Borrelia Afzelii or a negative control peptide are generated by in vitro refolding, purified and biotinylated as described elsewhere herein. Biotinylated MHC-peptide complexes are then coupled to a 270 kDa dextran multimerization domain labelled with APC by interaction with streptavidin (SA) on the dextran multimerization domain. The dextran-APC-SA multimerization domain is generated as described elsewhere herein. MHC-peptide complexes are added in an amount corresponding to a ratio of three MHC- peptide molecules per SA molecule and each molecule dextran contains 3.7 SA molecule and 8.95 molecules APC. The final concentration of dextran is 3.8x10e-8 M. The following MHC(peptide)/APC dextran constructs are made:

1. APC-SA conjugated 270 kDa dextran coupled with HLA-A*0201 in complex with beta2microglobulin and the peptide ALIACKQNV derived from OspA.
2. APC-SA conjugated 270 kDa dextran coupled with HLA-A*0201 in complex with beta2microglobulin and the peptide FTKEDTIT derived from OspA.
3. APC-SA conjugated 270 kDa dextran coupled with HLA-A*0201 in complex with beta2microglobulin and the peptide SIQIEIEQL derived from Fla B
4. APC-SA conjugated 270 kDa dextran coupled with HLA-A*0201 in complex with beta2microglobulin and the peptide NLNEVEKVL derived from Fla B
5. APC-SA conjugated 270 kDa dextran coupled with HLA-A*0201 in complex with beta2microglobulin and the peptide SLAKIENAI derived from Fla B
6. APC-SA conjugated 270 kDa dextran coupled with HLA-A*0201 in complex with beta2microglobulin and the nonsense peptide GLAGDVSAV (SEQ ID NO 217778).

The binding of the above described MHC(peptide)/APC dextran is used to determine the presence of Osp A or Fla B specific T cells in the blood from Borrelia infected individuals by flow cytometry following a standard flow cytometry protocol.

Blood from a patient with Lyme disease is isolated and 100 ul of this blood is incubated with 10 $\mu$l of each of the MHC(peptide)/APC dextran constructs described above for 10 minutes in the dark at room temperature. 5 $\mu$l of each of each of the antibodies mouse-anti-human CD3/PB (clone UCHT1 from Dako), mouse-anti-human CD4/FITC (clone MT310 from Dako) and mouse-anti-human CD8/PE (clone DK25 from Dako) are added and the incubation continues for another 20 minutes at 4°C in the dark. The samples are then washed by adding 2 ml PBS; pH =7.2 followed by centrifugation for 5 minutes at 300xg and the supernatant removed. The washing step is repeated twice. The washed cells are resuspended in 400-500 $\mu$l PBS + 1% BSA; pH=7.2 and analyzed on flowcytometer.

The presence of cells labeled with anti-CD3/PB, anti-CD8/PE and the MHC(peptide)/APC dextran constructs 1, 2, 3, 4 and 5 described above and thereby the presence of Borrelia specific T cells indicate that the patient are infected with Borrelia bacteria. Blood analysed with MHC(peptide)/APC dextran construct 6 show no staining of CD3 and CD8 positive cells with this MHC(peptide)/APC dextran construct. The result is shown in figure 15.

The sensitivity of the above described diagnostic test may be enhanced by addition of labeled antibodies specific for activation markers expressed in or on the surface of the Borrelia specific T cells.

We conclude that the MHC(peptide)/APC dextran constructs can be used to detect the presence of Borrelia specific T cells in the blood of patients infected with Borrelia.

**Example 26**

This is an example of how MHC multimers may be used for diagnosis of Lyme Disease in blood samples from humans infected with Borrelia bacteria.

In this example the MHC multimer used are MHC complexes coupled to the fluorophor-labelled multimerisation domain Streptavidin (SA), used for direct detection of TCR in flow Cytometry. The antigen origin is Borrelia, thus, immune monitoring of a Borrelia infection.

Lyme disease is caused by infection by Borrelia bacteria. During acute infection Borrelia specific activated T cells will be present in increased amounts in an activated state compared to healthy individuals. The presences of an increased amount of activated Borrelia specific T cells may thereby act as a surrogate marker for infection with Borrelia bacterium. MHC multimers carrying borrelia specific antigenic peptides is in this example used to detect the presence of Borrelia specific T cells in the blood of patients infected with Borrelia.

Purified MHC-peptide complexes consisting of HLA-A*0201 heavy chain, human beta2microglobulin and peptide derived from regions in Outer surface protein A (Table B) or Flagellin B (Table C) conserved among the three species Borrelia Burgdorferi, Borrelia Garinii and Borrelia Afzelii or a negative control peptide were generated by in vitro refolding, purified and biotinylated as described elsewhere herein. Biotinylated MHC-peptide complexes are then coupled SA labelled with APC. MHC-peptide complexes were added in an amount corresponding to a ratio of 5 MHC-peptide molecules per SA molecule. Then SA/APC carrying four MHC complexes were purified from free SA, free monomeric MHC complex, SA carrying three, two and one MHC complexes.

The following SA- MHC(peptide)/APC tetramers are made:

7. APC-SA coupled with HLA-A*0201 in complex with beta2microglobulin and the peptide ALIACKQNV derived from OspA.

8. APC-SA coupled with HLA-A*0201 in complex with beta2microglobulin and the peptide FTKEDTIT derived from OspA.

9. APC-SA coupled with HLA-A*0201 in complex with beta2microglobulin and the peptide SIQIEIEQL derived from Fla B.

10. APC-SA coupled with HLA-A*0201 in complex with beta2microglobulin and the peptide NLNEVEKVL derived from Fla B.

11. APC-SA coupled with HLA-A*0201 in complex with beta2microglobulin and the peptide SLAKIENAI derived from Fla B.

12. APC-SA coupled with HLA-A*0201 in complex with beta2microglobulin and the non-sense peptide GLAGDVSAV (SEQ ID NO 217778).

The binding of the above described MHC(peptide)/APC dextran can be used to determine the presence of Osp A or Fla B specific T cells in the blood from Borrelia infected individuals by flow cytometry following a standard flow cytometry protocol. Blood from a patient with Lyme disease is isolated and 100 ul of this blood is incubated with either of the four SA- MHC(peptide)/APC tetramers described above for 10 minutes in the dark at room temperature. 5 $\mu$l of each of each of the antibodies mouse-anti-human CD3/PB (clone UCHT1 from Dako) and mouse-anti-human CD8/PE (clone DK25 from Dako) are added and the incubation continued for another 20 minutes at 4°C in the dark. The samples are then washed by adding 2 ml PBS; pH =7.2 followed by centrifugation for 5 minutes at 200xg and the supernatant removed. The washing step is repeated. The washed cells are resuspended in 400-500 $\mu$l PBS; pH=7.2 and analyzed on flowcytometer.

The presence of cells labeled with anti-CD3/PB, anti-CD8/PE and either of the SA-MHC(peptide)/APC tetramers 7, 8, 9, 10 or 11 described above and thereby the presence of Borrelia specific T cells will indicate that the patient are infected with Borrelia bacteria. Blood analysed with SA- MHC(peptide)/APC tetramers 12 should show no staining of CD3 and CD8 positive cells with this SA- MHC(peptide)/APC tetramer.

The sensitivity of the above described diagnostic test may be enhanced by addition of labeled antibodies specific for activation markers expressed in or on the surface of the Borrelia specific T cells.

We conclude that the APC-SA coupled MHC(peptide) constructs may be used to detect the presence of Borrelia specific T cells in the blood of patients infected with Borrelia.

**Example 27**

This is an example of how MHC multimers may be used for diagnosis of Lyme Disease in blood samples from humans infected with Borrelia bacteria.

In this example the MHC multimer used are MHC complexes coupled to any fluorophor-labelled multimerisation as described elsewhere herein. The MHC multimers are used for direct detection of TCR in flow cytometry. The antigen origin is Borrelia, thus, immune monitoring of a Borrelia infection.

Lyme disease is caused by infection by Borrelia bacteria. During acute infection Borrelia specific activated T cells will be present in increased amounts in an activated state compared to healthy individuals. The presences of an increased amount of activated Borrelia specific T cells may thereby act as a surrogate marker for infection with Borrelia bacterium. MHC multimers carrying borrelia specific antigenic peptides is in this example used to detect the presence of Borrelia specific T cells in the blood of patients infected with Borrelia.

Purified MHC-peptide complexes consisting of HLA-A*0201 heavy chain, human beta2microglobulin and peptide derived from regions in Outer surface protein A (Table B) or Flagellin B (Table C) conserved among the three species Borrelia Burgdorferi, Borrelia Garinii and Borrelia Afzelii or a negative control peptide were generated by in vitro refolding and purified or purified from antigen presenting cells. MHC-peptide complexes are then coupled to a multimerisation domain together with APC.

The following MHC(peptide)/APC multimers are made:

13. APC-multimerisation domain coupled with HLA-A*0201 in complex with beta2microglobulin and the peptide ALIACKQNV derived from OspA.

14. APC-multimerisation domain coupled coupled with HLA-A*0201 in complex with beta2microglobulin and the peptide FTKEDTIT derived from OspA.

15. APC-multimerisation domain coupled coupled with HLA-A*0201 in complex with beta2microglobulin and the peptide SIQIEIEQL derived from Fla B.

16. APC-multimerisation domain coupled coupled with HLA-A*0201 in complex with beta2microglobulin and the peptide NLNEVEKVL derived from Fla B.

17. APC-multimerisation domain coupled coupled with HLA-A*0201 in complex with beta2microglobulin and the peptide SLAKIENAI derived from Fla B.

18. APC-multimerisation domain coupled with HLA-A*0201 in complex with beta2microglobulin and the non-sense peptide GLAGDVSAV (SEQ ID NO 217778).

The binding of the above described MHC(peptide)/APC multimers can be used to determine the presence of Osp A or Fla B specific T cells in the blood from Borrelia infected individuals by flow cytometry following a standard flow cytometry protocol. Blood from a patient with Lyme disease is isolated and 100 ul of this blood is incubated with either of the four MHC(peptide)/APC multimers described above for 10 minutes in the dark at room temperature. 5 $\mu$l of each of each of the antibodies mouse-anti-human CD3/PB (clone UCHT1 from Dako) and mouse-anti- human CD8/PE (clone DK25 from Dako) are added and the incubation continued for another 20 minutes at 4°C in the dark. The samples are then washed by adding 2 ml PBS; pH =7.2 followed by centrifugation for 5 minutes at 200xg and the supernatant removed. The washing step is repeated. The washed cells are resuspended in 400-500 $\mu$l PBS; pH=7.2 and analyzed on flowcytometer.

The presence of cells labeled with anti-CD3/PB, anti-CD8/PE and either of the MHC(peptide)/APC multimers 13, 14, 15, 16 or 17 described above and thereby the presence of Borrelia specific T cells will indicate that the patient are infected with Borrelia bacteria. Blood analysed with MHC(peptide)/APC multimer 18 should show no staining of CD3 and CD8 positive cells with this SA- MHC(peptide)/APC multimer.

The sensitivity of the above described diagnostic test may be enhanced by addition of labeled antibodies specific for activation markers expressed in or on the surface of the Borrelia specific T cells.

We conclude that the APC-multimerisation domain coupled MHC(peptide) constructs may be used to detect the presence of Borrelia specific T cells in the blood of patients infected with Borrelia.

## Example 28

This is an example of how MHC multimers may be used for diagnosis of Lyme Disease in blood samples from humans infected with Borrelia bacteria.

In this example the MHC multimer used are MHC complexes coupled to fluorophor-labelled dextran (Dextramers). The dextramers are used for direct detection of TCR in flow Cytometry. The antigen origin is Borrelia, thus, immune monitoring of a Borrelia infection.

Lyme disease is caused by infection by Borrelia bacteria. During acute infection Borrelia specific activated T cells will be present in increased amounts in an activated state compared to healthy individuals. The presences of an increased amount of activated Borrelia specific T cells may thereby act as a surrogate marker for infection with Borrelia bacterium. MHC multimers carrying borrelia specific antigenic peptides is in this example used to detect the presence of Borrelia specific T cells in the blood of patients infected with Borrelia.

Purified MHC-peptide complexes consisting of HLA-A*0301 heavy chain, human beta2microglobulin and peptide derived from regions in Outer surface protein C (Table A) conserved among the three species Borrelia Burgdorferi, Borrelia Garinii and Borrelia Afzelii or a negative control peptide were generated by in vitro refolding, purified and biotinylated as described elsewhere herein. Biotinylated MHC-peptide complexes were then coupled to a 270 kDa dextran multimerization domain labelled with APC by interaction with streptavidin (SA) on the dextran multimerization domain. The dextran-APC-SA multimerization domain was generated as described elsewhere herein. MHC-peptide complexes were added in an amount corresponding to a ratio of three MHC- peptide molecules per SA molecule and each molecule dextran contained 3.7 SA molecule and 8.95 molecules APC. The final concentration of dextran was 3.8x10e-8 M. The following MHC(peptide)/APC dextran constructs were made:

19. APC-SA conjugated 270 kDa dextran coupled with HLA-A*0301 in complex with beta2microglobulin and the peptide TLITEKLSK derived from OspC.

20. APC-SA conjugated 270 kDa dextran coupled with HLA-A*0301 in complex with beta2microglobulin and the peptide ELANKAIGK derived from OspC.

21. APC-SA conjugated 270 kDa dextran coupled with HLA-A*0301 in complex with beta2microglobulin and the HIV peptide QVPLRPMTYK.

The binding of the above described MHC(peptide)/APC dextran can be used to determine the presence of Osp C specific T cells in the blood from Borrelia infected individuals by flow cytometry following a standard flow cytometry protocol.

Blood from a patient with Lyme disease is isolated and 100 ul of this blood is incubated with 10 $\mu$l of one of the MHC(peptide)/APC dextran constructs described above for 10 minutes in the dark at room temperature. 5 $\mu$l of each of each of the antibodies mouse-anti-human CD3/PB (clone UCHT1 from Dako) and mouse-anti-human CD8/PE (clone DK25 from Dako) are added and the incubation continued for another 20 minutes at 4°C in the dark. The samples are then washed by adding 2 ml PBS; pH=7.2 followed by centrifugation for 5 minutes at 200xg and the supernatant removed. The washing step is repeated. The washed cells are resuspended in 400-500 $\mu$l PBS; pH=7.2 and analyzed on flowcytometer.

The presence of cells labeled with anti-CD3/PB, anti-CD8/PE and either of the MHC(peptide)/APC dextran constructs 19 or 20 described above and thereby the presence of Borrelia specific T cells will indicate that the patient are infected with Borrelia bacteria. Blood analysed with MHC(peptide)/APC dextran construct 21 should show no staining of CD3 and CD8 positive cells with this MHC(peptide)/APC dextran construct.

The sensitivity of the above described diagnostic test may be enhanced by addition of labeled antibodies specific for activation markers expressed in or on the surface of the Borrelia specific T cells.

We conclude that the APC-SA conjugated 270 kDa dextran coupled MHC(peptide) constructs may be used to detect the presence of Borrelia specific T cells in the blood of patients infected with Borrelia.

## Example 29

This is an example of how MHC multimers may be used for diagnosis of Lyme Disease in blood samples from humans infected with Borrelia bacteria.

In this example the MHC multimer used are MHC complexes coupled to the fluorophor-labelled multimerisation domain Streptavidin (SA), used for direct detection of TCR in flow Cytometry. The antigen origin is Borrelia, thus, immune monitoring of a Borrelia infection.

Lyme disease is caused by infection by Borrelia bacteria. During acute infection Borrelia specific activated T cells will be present in increased amounts in an activated state compared to healthy individuals. The presences of an increased amount of activated Borrelia specific T cells may thereby act as a surrogate marker for infection with Borrelia bacterium. MHC multimers carrying borrelia specific antigenic peptides is in this example used to detect the presence of Borrelia specific T cells in the blood of patients infected with Borrelia.

Purified MHC-peptide complexes consisting of HLA-A*0301 heavy chain, human beta2microglobulin and peptide derived from regions in Outer surface protein C (Table A) conserved among the three species Borrelia Burgdorferi, Borrelia Garinii and Borrelia Afzelii or a negative control peptide were generated by in vitro refolding, purified and biotinylated as described elsewhere herein. Biotinylated MHC-peptide complexes are then coupled SA labelled with APC. MHC-peptide complexes were added in an amount corresponding to a ratio of 5 MHC-peptide molecules per SA molecule. Then SA/APC carrying four MHC complexes were purified from free SA, free monomeric MHC complex, SA carrying three, two and one MHC complexes.

The following SA- MHC(peptide)/APC tetramers are made:

22. APC-SA coupled with HLA-A*0301 in complex with beta2microglobulin and the peptide TLITEKLSK derived from OspC.

23. APC-SA coupled with HLA-A*0301 in complex with beta2microglobulin and the peptide ELANKAIGK derived from OspC.

24. APC-SA coupled with HLA-A*0201 in complex with beta2microglobulin and the HIV peptide QVPLRPMTYK.

The binding of the above described MHC(peptide)/APC dextran can be used to determine the presence of Osp C specific T cells in the blood from Borrelia infected individuals by flow cytometry following a standard flow cytometry protocol.

Blood from a patient with Lyme disease is isolated and 100 ul of this blood is incubated with either of the four SA-MHC(peptide)/APC tetramers described above for 10 minutes in the dark at room temperature. 5 $\mu$l of each of each of the antibodies mouse-anti-human CD3/PB (clone UCHT1 from Dako) and mouse-anti-human CD8/PE (clone DK25 from Dako) are added and the incubation continued for another 20 minutes at 4°C in the dark. The samples are then washed by adding 2 ml PBS; pH =7.2 followed by centrifugation for 5 minutes at 200xg and the supernatant removed. The washing step is repeated. The washed cells are resuspended in 400-500 $\mu$l PBS; pH=7.2 and analyzed on flowcytometer.

The presence of cells labeled with anti-CD3/PB, anti-CD8/PE and either of the SA-MHC(peptide)/APC tetramers 22 or 23 described above and thereby the presence of Borrelia specific T cells will indicate that the patient are infected with Borrelia bacteria. Blood analysed with SA- MHC(peptide)/APC tetramers 24 should show no staining of CD3 and CD8 positive cells with this SA- MHC(peptide)/APC tetramer.

The sensitivity of the above described diagnostic test may be enhanced by addition of labeled antibodies specific for activation markers expressed in or on the surface of the Borrelia specific T cells.

We conclude that the APC-SA coupled MHC(peptide) constructs may be used to detect the presence of Borrelia specific T cells in the blood of patients infected with Borrelia.

**Example 30**

This is an example of how MHC multimers may be used for diagnosis of Lyme Disease in blood samples from humans infected with Borrelia bacteria.

In this example the MHC multimer used are MHC complexes coupled to any fluorophor-labelled multimerisation as described elsewhere herein. The MHC multimers are used for direct detection of TCR in flow cytometry. The antigen origin is Borrelia, thus, immune monitoring of a Borrelia infection.

Lyme disease is caused by infection by Borrelia bacteria. During acute infection Borrelia specific activated T cells will be present in increased amounts in an activated state compared to healthy individuals. The presences of an increased amount of activated Borrelia specific T cells may thereby act as a surrogate marker for infection with Borrelia bacterium. MHC multimers carrying borrelia specific antigenic peptides is in this example used to detect the presence of Borrelia specific T cells in the blood of patients infected with Borrelia.

Purified MHC-peptide complexes consisting of HLA-A*0201 heavy chain, human beta2microglobulin and peptide derived from regions in Outer surface protein C (Table A) conserved among the three species Borrelia Burgdorferi, Borrelia Garinii and Borrelia Afzelii or a negative control peptide were generated by in vitro refolding and purified or purified from antigen presenting cells. MHC-peptide complexes are then coupled to a multimerisation domain together with APC.

The following MHC(peptide)/APC multimers are made:

25. APC-multimerisation domain coupled with HLA-A*0201 in complex with beta2microglobulin and the peptide TLITEKLSK derived from OspC.
26. APC-multimerisation domain coupled with HLA-A*0201 in complex with beta2microglobulin and the peptide ELANKAIG derived from OspC.
27. APC-multimerisation domain coupled with HLA-A*0201 in complex with beta2microglobulin and the HIV peptide QVPLRPMTYK.

The binding of the above described MHC(peptide)/APC multimers can be used to determine the presence of Osp C specific T cells in the blood from Borrelia infected individuals by flow cytometry following a standard flow cytometry protocol.

Blood from a patient with Lyme disease is isolated and 100 ul of this blood is incubated with either of the four MHC(peptide)/APC multimers described above for 10 minutes in the dark at room temperature. 5 $\mu$l of each of each of the antibodies mouse-anti-human CD3/PB (clone UCHT1 from Dako) and mouse-anti- human CD8/PE (clone DK25 from Dako) are added and the incubation continued for another 20 minutes at 4°C in the dark. The samples are then washed by adding 2 ml PBS; pH =7.2 followed by centrifugation for 5 minutes at 200xg and the supernatant removed. The washing step is repeated. The washed cells are resuspended in 400-500 $\mu$l PBS; pH=7.2 and analyzed on flowcytometer.

The presence of cells labeled with anti-CD3/PB, anti-CD8/PE and either of the MHC(peptide)/APC multimers 25 or 26 described above and thereby the presence of Borrelia specific T cells will indicate that the patient are infected with Borrelia bacteria. Blood analysed with MHC(peptide)/APC multimer 27 should show no staining of CD3 and CD8 positive cells with this SA- MHC(peptide)/APC multimer.

The sensitivity of the above described diagnostic test may be enhanced by addition of labeled antibodies specific for activation markers expressed in or on the surface of the Borrelia specific T cells.

We conclude that the APC-multimerisation domain coupled MHC(peptide) constructs may be used to detect the presence of Borrelia specific T cells in the blood of patients infected with Borrelia.

**Example 31**

This is an example of measurement of antigen reactive T-Cells by IFN-$\gamma$ capture in blood samples by ELISPOT. This is an example of indirect detection of TCR, where individual cells are immobilized and measured by a chromogen assay.

The example provides a sensitive assay for the detection of T-cells reactive to an antigen by detecting a soluble factor whose secretion is induced by stimulation of the T-cell by the antigen.

A summary flow chart of the method is shown in FIG. 35. In brief, peripheral blood is diluted threefold in Dulbecco's phosphate buffered saline (DPBS), underlain with 15 ml of Ficoll (Pharmacia Ficoll-Paque #17-0840-02, Piscataway, N.J.) per 40 ml diluted blood in a 50 ml polypropylene centrifuge tube, and spun at 2000 RPM for 20 minutes in a Beckman CS-6R centrifuge (Beckman Inc., Palo Alto, Calif.). The buffy layer at the DPBS/Ficoll interface is removed, washed twice with DPBS and once with human tissue culture medium (hTCM: $\alpha$MEM+5% heat inactivated human AB serum (Ultraserum, BioWhittaker, Walkersville, Md.), penicillin/streptomycin, 1-glutamine) at low RCF to remove platelets. Sixty percent of the PBMCs are resuspended in freezing medium (10% dimethyl sulfoxide(Sigma Chenical Co., St. Louis, Mo.), 90% fetal bovine serum to a concentration of 5x10$^6$ cells/ml, frozen in a programmable Cryo-Med (New Baltimore, Mich.) cell freezer, and stored under liquid nitrogen until needed.

The purified PBMCs are plated at 2x10$^5$ cells/well at a volume of 0.1 ml in 96 well Costar cell culture plates. An equal volume of antigen at 10 $\mu$g/ml is added to triplicate or sextuplet sets of wells and the plate is incubated in a 37°C., 5% $CO_2$ incubator. On day five, 10 $\mu$l/well of 100 U/ml stock recombinant IL-2 (Advanced Biotechnologies Inc., Columbia, Md.) is added to each well. On day 8, frozen PBMCs are thawed, washed in DPBS+0.5% bovine serum albumin (BSA) to remove DMSO, resuspended to a concentration of 4x10$^6$ cells/ml in hTCM, and $\gamma$-irradiated (3,000 RADS). Fifty microliters/well are dispensed along with 50 $\mu$l of the appropriate antigen at a stock concentration of 40 $\mu$l/ml to give a final antigen concentration of 10 $\mu$g/ml.

To prepare a capture plate, IFN-$\gamma$ capture antibody (monoclonal mouse anti-human IFN-g, Endogen #M700A, Cambridge, Mass.) is diluted to 10 $\mu$g/ml in sterile 0.1 M Na(CO$_3$)$_2$ pH 8.2 buffer, aliquotted at 50 $\mu$l/well in flat bottomed 96 well sterile microtiter plates (Corning Costar Corp.), and incubated at 4°C for a minimum of 24 hours. Prior to use, excess antibody is removed and wells are washed twice with dPBS+1% Tween 20 (PBST). To block further nonspecific protein binding, plates are incubated with 250 $\mu$l/well of PBS+5% BSA at room temperature for 1 hour. After discarding the blocking solution, wells are washed once with PBST (0.1% Tween), followed by hTCM in preparation for the antigen stimulated cells.

On day 9 of the assay, twenty four hours after the second antigen stimulation, the stimulation plate is spun for 5 minutes at 1500 RPM in a Beckman CS-6R centrifuge and 90 $\mu$l of supernatant is carefully removed from each well with a micropipette. The pelleted cells are resuspended in 100 $\mu$l of hTCM, pooled in sterile tubes (Corning Costar corp sterile ClusterTAb #4411, Cambridge, Mass.), mixed and transferred into an equal number of wells of an anti IFN-$\gamma$ capture plate. Capture plates are incubated undisturbed at 37°C for 16-20 hours. At the end of the IFN-$\gamma$ secretion phase, the cells are discarded and the plates are washed three times with 0.1% PBST. A final aliquot of PBST is added to the wells for ten minutes, removed, and 100 $\mu$l of a 1:500 dilution of rabbit anti-human IFN-$\gamma$ polyclonal antibody (Endogen #P700, Cambridge, Mass.) in PBST+1% BSA is added to each well for 3.5 hours at room temperature with gentle rocking. Unbound anti-IFN-$\gamma$ polyclonal antibody is removed by three washes with PBST, followed by a wash with 250 $\mu$l of 1X Tris-buffered saline+0.05% Tween 20 (TBST). Next, a 100 $\mu$l aliquot of 1:5000 alkaline phosphatase-conjugated mouse anti-rabbit polyclonal antibody (Jackson Immunological #211-055-109, West Grove, Pa.) diluted in TBST is added to each well and incubated at room temperature for 1.5-2 hours with gentle rocking. Excess enzyme-conjugated antibody is removed by three washes with PBST and two washes with alkaline phosphatase buffer (APB=0.1 M NaCl, 0.05 M MgCl.sub.2, 0.1 M Tris HCl, pH 9.5) followed by addition of the substrate mix of p-Toluidine salt and nitroblue tetrazolium chloride (BCIP/NBT, GIBCO BRL #18280-016, Gaithersburg, Md.). To stop the calorimetric reaction, plates were washed three times in dH$_2$O, inverted to minimize deposition of dust in the wells, and dried overnight at 28°C in a dust free drying oven.

Images of the spots corresponding to the lymphokine secreted by individual antigen-stimulated T cells are captured with a CCD video camera and the image is analyzed by NIH image software. Captured images are enhanced using the Look Up Table which contrasts the images. Thresholding is then applied to every image and a wand tool is used to highlight the border to effectively subtract the edge of the well so that background counts won't be high and artificial. Density slicing over a narrow range is then used to highlight the spots produced from secreting cells. Pixel limits are set to subtract out small debris and large particles, and the number of spots falling within the prescribed pixel range are counted by the software program. Totals from each well are then manually recorded for future analysis. Alternatively, spots can be counted by other commercially available or customized software applications, or may be quantitated manually by a technician using standard light microscopy. Spots can also be counted manually under a light microscope.

We conclude that the protocol detailed above can be used for the enumeration of single IFN-$\gamma$ secreting T cells.

**Example 32**

This is an example of measurement of antigen reactive T-Cells by IFN-$\gamma$ capture in blood samples by ELISPOT. This is an example of indirect detection of TCR, where individual cells are immobilized and measured by a chromogen assay. The antigenic peptide origin is a library of antigens.

The example provides a sensitive assay for the detection of T-cells reactive to the antigen of a library generated as described in example 24, by detecting a soluble factor whose secretion is induced by stimulation of the T-cell by the antigen.

This example is similar to the experiment above. PMBC are isolated, prepared and stored as described in the example above.

The purified PBMCs are plated at $2 \times 10^5$ cells/well at a volume of 0.1 ml in 96 well Costar cell culture plates. An equal volume of antigens from the library, at 10 $\mu$g/ml is added to triplicate or sextuplet sets of wells and the plate is incubated in a 37°C, 5% $CO_2$ incubator. On day five, 10 $\mu$l/well of 100 U/ml stock recombinant IL-2 is added to each well. On day 8, frozen PBMCs are thawed, washed in DPBS+0.5% BSA to remove DMSO, resuspended to a concentration of $4 \times 10^6$ cells/ml in hTCM, and $\gamma$-irradiated (3,000 RADS). 50 $\mu$l/well are dispensed along with 50 $\mu$l of the appropriate antigen at a stock concentration of 40 $\mu$l/ml to give a final antigen concentration of 10 $\mu$g/ml.

A capture plate with IFN-$\gamma$ antibody is prepared, washed and blocked as described in the example above.

On day 9 of the assay, twenty four hours after the second antigen stimulation, the stimulation plate is spun for 5 minutes at 1500 RPM and 90 $\mu$l of supernatant is carefully removed from each well with a micropipette. The pelleted cells are resuspended in 100 $\mu$l of hTCM, pooled in sterile tubes, mixed and transferred into an equal number of wells of an anti IFN- $\gamma$ capture plate. Capture plates are incubated undisturbed at 37°C for 16-20 hours. At the end of the IFN-$\gamma$ secretion phase, the cells are discarded and the plates are washed three times with 0.1% PBST. A final aliquot of PBST is added to the wells for ten minutes, removed, and 100 $\mu$l of a 1:500 dilution of rabbit anti-human IFN-$\gamma$ polyclonal antibody in PBST+1% BSA is added to each well for 3.5 hours at room temperature with gentle rocking. Unbound anti-IFN-$\gamma$ polyclonal antibody is removed by three washes with PBST, followed by a wash with 250 $\mu$l of 1X Tris-buffered saline+0.05% Tween 20 (TBST). Next, a 100 $\mu$l aliquot of 1:5000 alkaline phosphatase-conjugated mouse anti-rabbit polyclonal antibody diluted in TBST is added to each well and incubated at room temperature for 1.5-2 hours with gentle rocking. Excess enzyme-conjugated antibody is removed by three washes with PBST and two washes with alkaline phosphatase followed by addition of the substrate mix of p-Toluidine salt and nitroblue tetrazolium chloride. To stop the calorimetric reaction, plates were washed three times in $dH_2O$, inverted to minimize deposition of dust in the wells, and dried overnight at 28°C in a dust free drying oven.

Images of the spots corresponding to the lymphokine secreted by individual antigen-stimulated T cells are captured with a CCD video camera and the image is analyzed as described in the example above

We conclude that the experiment detailed above can be used for the enumeration of single IFN-$\gamma$ secreting T cells in blood.

**Example 33**

This is an example of measurement of antigen reactive T-Cells by IFN-$\gamma$ capture in blood samples from Borrelia patients by ELISPOT.

This is an example of indirect detection of TCR, where individual cells are immobilized and measured by a chromogen assay. The antigenic peptide origin is Borrelia, thus, immune monitoring of Borrelia.

The example provides a sensitive assay for the detection of T-cells reactive to the antigen OspC by detecting a soluble factor whose secretion is induced by stimulation of the T-cell by the antigen.

This example is similar to the experiment above. PBMCs from Borrelia patients are isolated, prepared and stored as described in the example above.

The purified PBMCs are plated at $2 \times 10^5$ cells/well at a volume of 0.1 ml in 96 well Costar cell culture plates. An equal volume of a mix of antigenic peptides from OspC protein, at 10 $\mu$g/ml is added to triplicate or sextuplet sets of wells and the plate is incubated in a 37°C, 5% $CO_2$ incubator. On day five, 10 $\mu$l/well of 100 U/ml stock recombinant IL-2 is added to each well. On day 8, frozen PBMCs are thawed, washed in DPBS+0.5% BSA to remove DMSO, resuspended to a concentration of $4 \times 10^6$ cells/ml in hTCM, and $\gamma$-irradiated (3,000 RADS). 50 $\mu$l/well are dispensed along with 50 $\mu$l of the appropriate antigen at a stock concentration of 40 $\mu$l/ml to give a final antigen concentration of 10 $\mu$g/ml.

A capture plate with IFN-$\gamma$ antibody is prepared, washed and blocked as described in the example above.

On day 9 of the assay, twenty four hours after the second antigen stimulation, the stimulation plate is spun for 5 minutes at 1500 RPM and 90 $\mu$l of supernatant is carefully removed from each well with a micropipette. The pelleted cells are resuspended in 100 $\mu$l of hTCM, pooled in sterile tubes, mixed and transferred into an equal number of wells of an anti IFN- $\gamma$ capture plate. Capture plates are incubated undisturbed at 37°C for 16-20 hours. At the end of the IFN-$\gamma$ secretion phase, the cells are discarded and the plates are washed three times with 0.1% PBST. A final aliquot of PBST is added to the wells for ten minutes, removed, and 100 $\mu$l of a 1:500 dilution of rabbit anti-human IFN-$\gamma$ polyclonal antibody in PBST+1% BSA is added to each well for 3.5 hours at room temperature with gentle rocking. Unbound anti-IFN-$\gamma$ polyclonal antibody is removed by three washes with PBST, followed by a wash with 250 $\mu$l of 1X Tris-buffered saline+0.05% Tween 20 (TBST). Next, a 100 $\mu$l aliquot of 1:5000 alkaline phosphatase-conjugated mouse anti-rabbit polyclonal antibody diluted in TBST is added to each well and incubated at room temperature for 1.5-2 hours with gentle rocking. Excess enzyme-conjugated antibody is removed by three washes with PBST and two washes with alkaline phosphatase followed by addition of the substrate mix of p-Toluidine salt and nitroblue tetrazolium chloride. To stop the calorimetric reaction, plates were washed three times in $dH_2O$, inverted to minimize deposition of dust in the wells, and dried overnight at 28°C in a dust free drying oven.

Images of the spots corresponding to the lymphokine secreted by individual antigen-stimulated T cells are captured with a CCD video camera and the image is analyzed as described in the example above

We conclude that the experiment detailed above can be used for the enumeration of single IFN-γ secreting T cells in blood from Borrelia patients.

## Example 34

This is an example of how antigen-specific T-cells can be detected using a direct detection method detecting T cell immobilized in solid tissue. In this example MHC dextramers are used to detect antigen-specific T cells on frozen tissue sections using enzymatic chromogenic precipitation detection.

Equilibrate the cryosection tissue (e.g. section of spleen from transgenic mice) to - 20°C in the cryostate. Cut 5 μm sections and then dry sections on slides at room temperature. Store slides frozen until use at -20°C.

Equilibrate frozen sections to room temperature. Fix with acetone for 5 min. Immediately after fixation transfer slides to TBS buffer (50 mM Tris-HCL pH 7,6, 150 mM NaCl) for 10 min.

Incubate slides with FITC-conjugated MHC-dextramers at appropriate dilution (1:40-1:80) and incubate for 30 min at room temperature. Other dilution ranges, as well as incubation time and temperature, may be desirable.

Decant solution and gently tap slides against filter paper, submerge in TBS buffer. Decant and wash for 10 min in TBS buffer.

Incubate with rabbit polyclonal anti-FITC antibody (Dako P5100) at 1:100 dilution in TBS at room temperature for 30 min. Repeat step 5 and 6.

Incubate with Envision anti-Rabbit HRP (Dako K4003) at room temperature for 30 min. Other visualization systems may be used.

Repeat step 5 and 6.

Develop with DAB+ (Dako K3468) in fume hood for 10 min. Other substrates may be used

Rinse slides in tap-water for 5 min.

Counterstain with hematoxylin (Dako S3309) for 2 min.

Repeat step 12, mount slides.

The slides stained with MHC-Dextramers can now be evaluated by microscopy.

## Example 35

This is an example of how antigen-specific T-cells can be detected using a direct detection method detecting T cell immobilized in solid tissue. In this example MHC dextramers are used to detect antigen-specific T cells on paraffin embedded tissue sections using enzymatic chromogenic precipitation detection.

Formaldehyde fixed paraffin-embedded tissue are cut in section and mounted on the glass slice, for subsequent IHC staining with MHC-dextramers. Tissue fixed and prepared according to other protocols may be used as well. E.g. fresh tissue, lightly fixed tissue section (e.g. tissue fixed in 2% formaldehyde) or formalin-fixed, paraffin-embedded tissue section.

Optimal staining may require target retrieval treatment with enzymes as well as heating in a suitable buffer before incubation with antibodies and MHC-dextramer.

The sample is stained for DNA using DAPI stain, followed by incubated with an antigen-specific MHCdex/FITC reagent, followed by addition of anti-FITC antibody labeled with HRP.

Then the substrate for HRP, "DAP" is added and the reaction allows to progress.

The sample is analyzed by light microscopy for the present of a colored precipitate on the cells (DAPI stained nucleus) positive for the specific MHC/dex reagent.

A digital image of the stained sample is obtained, and this can be analyzed manually in the same way as by microscopy. However, a digital image may be used for automatic determination of where and how many cells that are positive, related to the total amount of cells, determined by the DAPI staining, or other criteria or stainings.

## Example 36

This example describes how the quality of a MHC multimer can be tested. The MHC multimer is in this example a MHC-dextramer, and the test involves specific binding of the MHC-dextramer to TCRs immobilized on beads.

Recombinant TCRs (CMV3 TCRs; Soluble CMVpp65(NLVPMVATV)-specific (SEQ ID NO 217779) TCR protein) specific for the MHC-peptide complex HLA-A*0201(NLVPMVATV) (SEQ ID NO 217779), where the letters in parenthesis denote the peptide complexed to the MHC-allel HLA-A*0201, were obtained from Altor Biosciences. The TCRs were dimers linked together via an IgG framework.

The purity of the TCRs was verified by SDS PAGE and was between 95-100% pure. The quality of the TCRs was verified by their ability to recognize the relevant MHC-dextramer and not irrelevant MHC dextramers in ELISA experiments (data not shown).

Carboxylate-modified beads were coupled with dimeric TCR (CMV3 TCRs; Soluble CMVpp65(NLVPMVATV)-specific TCR protein (SEQ ID NO 217779)), incubated with fluorescently labeled MHC-dextramers and the extend of cell staining analysed by flow cytometry, as follows:

Immobilization of TCR on carboxylate beads:

$3\times10^9$ Carboxylate-modified beads, Duke Scientific Corporation, XPR-1536, $4\mu m$, lot:4394 were washed in 2x 500$\mu$l Wash buffer 1 (0,05% Tetronic 1307, 0,1M MES-buffer (2-[N-morpholino]ethanesulfonic acid), pH 6,0), centrifuged 4 min at 15000 g, and the supernatant was discarded.
125 $\mu$l EDAC/Sulfo-NHS (50mM EDAC (1-ethyl-3-(3-dimethylaminopropyl)carbodiimide), 50mM Sulfo-NHS, in Wash buffer 1) was added to the beads, and the suspension incubated at room temperature for 20 min.
Beads were washed in 2x 250$\mu$l Wash buffer 1 and centrifuged 2 min at 15000 g, and the supernatant was discarded.
TCR was added in various concentrations from 0 $\mu$g to 20 $\mu$g, and incubated with slow shaking overnight at 4°C.
Beads were centrifuged 4 min at 15000 g, and the supernatant discarded.
Beads were washed in 2x 500$\mu$l Wash buffer 1 and centrifuged 4 min at 1500 g, and the supernatant was discarded.
125$\mu$l 20mM Glycin in Wash buffer 1 was added, and resuspended beads incubated for 1 hour at room temperature.
Beads were washed in 2x 500$\mu$l phosphate-buffered saline (PBS) pH 7.2, 0.5 % Tetronic 1307, and centrifuged 2 min at 15000 g, and the supernatant was discarded. Beads were resuspended in 250$\mu$l PBS pH 7.2, 0,05% Tetronic 1307.
Bead concentration after resuspension was $1,2\times10^7$ beads/$\mu$l. Beads coated with TCR were stored at 2-8°C until further use.

Flow cytometry analysis:

20$\mu$l beads ($1,2\times10^7$ beads/$\mu$l) coated with 0-20$\mu$g TCRs, as described above were washed in 200$\mu$l Wash buffer 2 (5% FCS, PBS, pH 7.4).
Beads were centrifuged 3 min at 12000 g, and the supernatant was discarded, and beads resuspended in 50$\mu$l Wash buffer 2.
10$\mu$l MHC-dextramers were added, and samples were incubated 15 min. at room temperature in the dark.
Samples were washed in 1 ml Wash buffer 2, centrifuged at 300 g for 5 min. The supernatant was discarded, and pellet resuspended in 0.4 ml PBS pH 7.4, and kept at 4°C in the dark until analysis on flow cytometer.
Samples were analysed by flow cytometry on a CyAn instrument.
The results are shown in figure 18. Beads coated with 2-20$\mu$g TCR all showed positive staining with the specific HLA-A*0201(NLVPMVATV)/RPE (SEQ ID NO 217779) and not with an irrelevant HLA-A*0201(ILKEPVHGV)/RPE (SEQ ID NO 217780) dextramer. It can be concluded that carboxylate beads coated with dimeric TCRs can be used to test the quality of the MHC-dextramers.

**Example 37**

This example describes how the quality of a MHC multimer can be tested. The MHC multimer was in this example a MHC-dextramer, and the test involved specific binding of the MHC-dextramer to monomeric and dimeric TCRs immobilized to different kind of beads.
Binding of MHC-dextramer to carboxylated beads coated with monomeric TCR: Recombinant monomeric TCRs (CMV3 TCRs; Soluble CMVpp65(NLVPMVATV)-specific TCR protein (SEQ ID NO 217779)) specific for the MHC-peptide complex HLA-A*0201(NLVPMVATV) (SEQ ID NO 217779), were obtained from Altor Biosciences. The purity of the TCRs were verified by SDS PAGE. The quality of the TCRs were verified by their ability to recognize the relevant MHC-dextramer and not irrelevant MHC dextramers in ELISA experiments (data not shown).
Carboxylate modified beads were coupled with monomeric TCR (CMV3 TCRs; Soluble CMVpp65(NLVPMVATV)-specific TCR protein (SEQ ID NO 217779)), incubated with fluorescently labeled MHC-dextramers and the extend of cell staining analysed by flow cytometry, as described in Example 8. 0-20 $\mu$g of monomeric TCRs were coupled to Carboxylate modified beads.

Flow cytometry analysis of beads coupled with 0-20 μg of monomeric TCRs showed a slightly stronger signal when stained with the relevant HLA-A*0201(NLVPMVATV) (SEQ ID NO 217779) dextramer than with an irrelevant MHC-dextramer (data not shown). It might be desirable to coat the beads with larger amounts of monomeric TCRs in order to increase the signal difference between relevant and irrelevant MHC-dextramer.

Beads couples with 0 μg of monomeric TCRs showed identical signal when stained with relevant and irrelevant MHC-dextramers.

We conclude that the monomeric TCRs coupled to Carboxylate modified beads can be used as positive control for the MHC-dextramer.

Binding of MHC-dextramer to streptavidin beads coupled with biotinylated monomeric TCR:

Recombinant monomeric, biotinylated TCRs (CMV3 TCRs; Soluble CMVpp65(NLVPMVATV)-specific TCR protein (SEQ ID NO 217779)) specific for the MHC-peptide complex HLA-A*0201(NLVPMVATV) (SEQ ID NO 217779), were obtained from Altor Biosciences.

The purity of the TCRs were verified by SDS PAGE. The quality of the TCRs were verified by their ability to recognize the relevant MHC-dextramer and not irrelevant MHC dextramers in ELISA experiments (data not shown).

Streptavidin beads were coupled with monomeric biotinylated TCR (CMV3 TCRs; Soluble CMVpp65(NLVPMVATV)-specific TCR protein (SEQ ID NO 217779)), incubated with fluorescently labeled MHC-dextramers and the extend of cell staining analysed by flow cytometry.

Immobilization of TCR on streptavidin beads:

$2x10^6$ Streptavidin Coated Compel Magnetic beads, Bangs laboratories, CM01N, lot: 6998, were washed in 2x 500μl phosphate-buffered saline (PBS) pH 7.2 centrifuged 5 min at 15000 g, and then the supernatant was discarded. Beads were resuspended in 50μl PBS pH 7.2.

0-4μg TCRs was added. Incubated at room temperature for 30 min.

Beads were washed in 2x 500μl PBS, pH 7.2. Centrifuged 5 min at 15000 g, and the supernatant was discarded.

Beads were resuspended in 100μl PBS pH 7.2.

Bead concentration after resuspension was $2x10^4$ beads/μl. Beads coated with TCRs were stored at 2-8°C until further use.

Flow cytometry analysis:

50μl streptavidin beads (~$1x10^6$ beads) coated with 0-4μg TCRs were added 10μl MHC-dextramers, and samples were incubated at 4°C for 1 hour.

2x 500μl PBS pH 7.2 was added, and samples were centrifuged for 5 min at 15000 g, and supernatant discarded.

Beads were resuspended in 500μl PBS pH 7.2 and kept at 4°C in the dark until analysis on a flow cytometer.

Samples were analysed by flow cytometry on a CyAn instrument.

Beads were stained with HLA-A*0201(NLVPMVATV (SEQ ID NO 217779))-dextramers specific for the TCR and with irrelevant MHC-dextramers not able to bind the TCR. Flow cytometry analysis of beads coupled with >0 μg TCRs showed a slightly stronger signal when stained with the relevant MHC-dextramer than with the irrelevant MHC-dextramer (data not shown). The staining intensity was identical when beads conjugated with 0 μg TCR were stained with either specific or non-specific MHC-dextramers. We conclude that the monomeric biotinylated TCRs bound to streptavidin coated beads can be used as positive control for the MHC-dextramer.

Binding of MHC-dextramers toamine-modified beads coupled with monomeric or dimeric TCRs:

Recombinant monomeric or dimeric TCRs (CMV3 TCRs; Soluble CMVpp65(NLVPMVATV (SEQ ID NO 217779))-specific TCR protein) specific for the MHC-peptide complex HLA-A*0201(NLVPMVATV(SEQ ID NO 217779)), were obtained from Altor Biosciences.

The purity of the TCRs were verified by SDS PAGE. The quality of the TCRs were verified by their ability to recognize the relevant MHC-dextramer and not irrelevant MHC dextramers in ELISA experiments (data not shown).

Amine-modified beads were coupled with dimeric TCR (CMV3 TCRs; Soluble CMVpp65(NLVPMVATV (SEQ ID NO 217779))-specific TCR protein).

Immobilization of TCR on amine modified beads:

$3x10^9$ Amine modified beads, Duke Scentific XPR-1536, 4μm, lot:4393 were washed in 2x500μl phosphate-

buffered saline (PBS) pH 7.5 centrifuged 4 min at 15000 g, and then the supernatant was discarded.

Resuspend beads in 0.3ml PBS pH 7.5

25$\mu$l 20mM solution of SPDP (N-Succinimidyl 3-(2-pyridyldithio) propionate) in DMSO was added to the beads, and the suspension incubated at room temperature for 30 min.

Beads were washed in 2x1ml PBS containing 1 mM EDTA, pH 7,5. Centrifuged 4 min at 15000 g, and the supernatant was discarded.

Resuspend beads in 0,3ml PBS containing 1 mM EDTA, pH 7.5.

10mg dithiothritol (DTT) was added, and suspension incubated at room temperature for 30 min.

Beads were washed in 2x 1ml PBS, pH 7.5. Centrifuged 4 min at 15000 g, and the supernatant was discarded.

Sedimented beads were added a freshly prepared SPDP-derivatized protein prepared according to the following outlines (step 14-15):

Treat protein (in this example dimeric TCR) 2-5mg/ml, in 0,1ml carbonate buffer, pH 8 with 10$\mu$l 20mM solution of SPDP in DMSO at room temperature for 30 min.

Remove excess reagent by passing through a small desalting column in PBS pH 7.5, or by rapid dialyse against PBS pH 7.5.

Add >1mg SPDS-protein per $10^9$ beads, to the SPDP derivatized and reduced bead preparation from paragraph 13. 0-1mg of SPDS-protein per $10^9$ beads might be desirable.

Resuspend beads in the SPDP-protein solution. Incubate with slow shaking overnight at 4°C.

Beads were washed in 2x 1ml PBS pH 7.5. Centrifuged 4 min at 15000 g, and the supernatant was discarded.

Resuspend beads in 250$\mu$l PBS pH 7.5.

Bead concentration after resuspension was $1.2 \times 10^7$ beads/$\mu$l. Beads coated with TCRs were stored at 2-8°C. The quality of MHC-dextramer can now be analysed by examining the degree of binding of specific MHC-dextramer to the TCR-coated beads, versus the binding of irrelevant MHC-dextramer to the TCR coated beads.

Various reaction conditions (e.g various protein:bead ratios) and assay for optimal coupling yield can be explored. The latter can be done by using an ELISA technique (incubate protein/bead conjugated with an appropiate anti-protein enzyme conjugated (e.g. HRP Peroxidase) followed by washing and colour development with a suitable substrate (e.g. TMB/Peroxide)) or by flow cytometry (e.g. a fluorescence labelled anti-protein (in this example MHC multimer HLA-A*0201(NLVPMVATV (SEQ ID NO 217779))) to assess level of covalently bound protein to amine-modified beads.

We conclude that the TCRs coupled to amine-modified beads coupled can be used as positive controls for the MHC Dextramer as described in Example 38 and 39.

## Example 38

This example describes how TCR-coated beads can be used as internal, positive controls when analysing suspensions of Human Peripheral Blood Mononuclear Cells (HPBMCs), whole blood samples or any other cell sample of interest. The MHC multimer employed in this example is a MHC-dextramer.

In this example TCR-coated carboxylated beads generated as described in Example 8 were added to a sample containing either HPBMCs or whole peripheral blood.

HPBMCs and TCR-beads were incubated with fluorescently labelled MHC-dextramers and the extent of cell staining analysed by flow cytometry according to this general staining procedure:

Transfer 1-3 x $10^6$ lymphoid cells (PBMC or splenocytes) to a 12 x 75 mm polystyrene test tube. Other cells of interest can be used. Allocate only 2-5 x $10^5$ cells per tube when staining T-cell clones or cell lines due to the high frequency of antigen-specific T cells

Add 2 ml 0,01 mol/L PBS containing 5% fetal calf serum and centrifuge at 300x g for 5 minutes. Remove supernatant and resuspend cells in remaining liquid.

Add 10 $\mu$l of MHC Dextramer and mix gently with a vortex mixer. Incubate in the dark at room temperature for 10 minutes.

Add an optimally titrated amount of anti-CD8 antibody conjugated with a relevant flourochrome (e.g. Dako clone DK25 for human lymphocytes or clone YTS169.4/KT15 for mouse lymphocytes). Incubate in the dark at 2-8°C for 20 min.

Add 2 ml of 0,01 mol/L PBS containing 5% fetal calf serum and centrifuge at 300x g for 5 minutes.

Resuspend pellet in an appropriate fluid for flow cytometry, e.g. 0.4 ml PBS. Analyse on a flow cytometer or store at 2-8°C in the dark until analysis. Do not store longer than 2 hours before analysis.

Human peripheral whole blood and TCR-beads were incubated with fluorescently labelled MHC-dextramers and the extent of cell staining analysed by flow cytometry as follows:

Transfer 100 $\mu$L whole blood to a 12 x 75 mm polystyrene test tube.

Add 10 $\mu$l of MHC Dextramer and mix with a vortex mixer. Incubate in the dark at room temperature for 10 minutes.

Add an optimally titrated amount of anti-CD8 antibody (e.g. Dako clone DK25) conjugated with a relevant fluorochromes and mix well. Continue incubation at 2-8 °C in the dark for 20 minutes.

Add 2 mL EasyLyse™ working solution (Code No. S2364) and incubate for 10 minutes. Centrifuge for 5 minutes at 300 x g and aspirate supernatant.

Add 2 mL 0.01 mol/L PBS and centrifuge for 5 minutes at 300 x g and aspirate supernatant.

Resuspend pellet in an appropriate fluid for flow cytometry, e.g. 0.4 mL PBS, and analyze on a flow cytometer or store at 2-8 °C in the dark until analysis. Do not store longer than 2 hours before analysis.

Figure 19 shows examples of TCR-beads added into whole blood or HPBMC samples. In both experiments it is possible, by forward- vs. side-scatter measurements, to distinguish TCR-beads from cell populations in the sample. Region R1 is TCR-beads, and region R2 is lymphocyte cell population of interest in the analysis of MHC positive T cells.

The size and conditions of coating of beads might be optimized. The size of beads or labelling of beads (e.g. flourescent labelling) can be optimized to allow separation of cells of interest in the sample. In this example the forward-vs. side- scatter dot plot has been used for gating of cell populations of interest. Other parameters (e.g. fluorescence intensity) for cell populations of interest can be used.

Human peripheral whole blood and other cells (e.g. HPBMCs) can be stained with MHC Dextramers simultaneously with immuno-phenotyping of relevant antigens. The staining procedure describes the use of labelled CD8 antibody together with MHC dextramers; additional antibodies for detection of other extracellular antigens can be added. Likewise, detection of intracellular antigens can be performed simultaneously with MHC-detection (for protocol, see IntraStain procedure, cat no. K2311, Dako. Additional washing step prior to IntraStain Reagent A is essential for good results using MHC Dextramers together with this IntraStain procedure).

**Example 39**

This example describes how TCR-coated beads can be used as internal, positive controls when analysing suspensions of Human Peripheral Blood Mononuclear Cells (HPBMCs), whole blood samples or any cell sample of interest. The MHC multimer employed in this example is a MHC-tetramer.

In this example TCR-coated beads as described in Example 37 and 38 are added to a sample containing either HPBMCs or whole peripheral blood.

HPBMCs/whole peripheral blood and TCR-beads are incubated with fluorescently labelled MHC-tetramers and the extent of cell staining is analysed by flow cytometry according to staining procedures as described for Tetramers in the product insert by Beckman Coulter.

**Example 40**

This example describes how TCR-coated beads can be used as internal, positive controls when analysing suspensions of Human Peripheral Blood Mononuclear Cells (HPBMCs), blood samples (red blood cell depleted) or any cell sample of interest. The MHC multimer employed in this example is a MHC-pentamer.

In this example TCR-coated beads as described in Example 37 and 38 are added to a sample containing either HPBMCs or blood.

HPBMCs/blood sample and TCR-beads are incubated with fluorescently labelled MHC-pentamers and the extent of cell staining is analysed by flow cytometry according to staining procedures as described for Petramers in the product insert by ProImmune.

**Example 41**

This example describes how it can be examined whether a MHC multimer is correctly folded. The MHC multimer is in this example a MHC-dextramer, and the test involves specific binding of the MHC-dextramer to antibodies immobilized on beads.

Beads were coated with the antibody clone W6/32. W6/32 is an antibody recognizing all human MHC I HLA-A, B and, C alleles but only when they are in the correct conformation and properly loaded with antigenic peptide. The protocol for immobilization of proteins on carboxylate beads described elsewhere herein was followed. In the following these W6/32 antibody coated carboxylated beads are referred to as W/32-beads.

W6/32-beads were incubated with fluorescently labeled MHC-dextramers and the extent of cell staining analyzed by flow cytometry. The staining procedure described in example 37 was followed.

W6/32-beads incubated with correctly folded MHC-dextramers showed efficient staining. Experiments with W6/32-beads incubated with unfolded heavy chain attached to fluorescently labelled dextran, or a fluorescently labelled dextran without MHC complex attached, showed less fluorescence intensity compared to W6/32-beads incubated with correct folded MHC-dextramer.

We conclude that beads coupled with the antibody clone W6/32 can be used as positive control for all MHC alleles recognized by this antibody.

Other antibodies, or other types of molecules such as DNA aptamers recognizing correctly folded MHCs or parts of MHC could be used in similar experiments.

**Example 42**

This example describes how the quality of the MHC multimer can be tested. The example also describes how MHC multimers can be used for detection of TCR immobilized to solid support.

The MHC multimer in this example is a MHC-dextramer, and the test involves specific binding of the MHC-dextramer to TCRs or other MHC-recognizing molecules. Experiments can be performed with any kind of TCRs or MHC-recognizing molecules immobilized on a bead or other solid support.

Procedures as described in examples elsewhere herein can be used, depending on the chemistry of the MHC recognizing molecules and type of solid support. Procedures for coupling of molecules and type of solid support can be chosen and optimized according to the chemistry of the molecules and solid support.

Alternatively, the experiments could be performed without including solid supports, e.g. by performing immunoprecipitation of formed MHC multimer-TCR complexes.

**Example 43**

This example describes how the quality of a MHC multimer can be tested. The example also describes how MHC multimers can be used for detection of a specific T-cell line.

The MHC multimer in this example is a MHC-dextramer, and the test involves specific binding of the MHC-dextramer to a cell line that expresses specific TCRs and displays these on the cell surface.

A transfected Jurkat T cell line (JT3A) from Altor Biosciences specific for the MHC complex HLA-A*0201(NLVPMVATV (SEQ ID NO 217779)) was evaluated as positive control for the MHC-dextramer HLA-A*0201(NLVPMVATV (SEQ ID NO 217779)). The cells were cultured and treated to express TCR just before evaluation. Under the conditions used, 20-50% of the cells were expected to express and display TCR. After stimulation the cells were incubated with fluorescently labeled MHC-dextramers and the extent of cell staining analyzed by flow cytometry, as follows:

JT3A cells growing in log phase were incubated at room temperature for 2-3 hours to express TCRs (The TCRs are not stable expressed at 37°C).

After 3 hours cells were centrifuged for 5 min at 400 g, and the supernatant was discarded.

Cells were washed in PBS pH 7.4 + 5% FCS, and centrifuged for 5 min at 400 g. The supernatant was discarded, and cells resuspended in proper volume PBS pH 7.4 + 5% FCS for counting in a Bürker chamber.

$1 \times 10^6$ cells per sample in 100$\mu$l PBS pH 7.4 + 5% FCS were added to each sample tube.

10$\mu$l MHC-dextramers were added. Incubation for 30 min at 4°C in the dark.

5$\mu$l anti-CD3 was added to each sample. Further incubation for 30 min at 4°C in the dark.

Samples were washed in 2 ml PBS, centrifuged for 5 min at 300 g. Supernatant discarded and sample resuspended in 0.4 ml PBS pH 7.4.

Samples were kept at 2-8°C in the dark until analysis on flow cytometer.

Samples were analyzed by flow cytometry on a CyAn instrument.

Data were analyzed by the Summit software. Stimulated JT3A cells were stained with the specific MHC-dextramer HLA-A*0201(NLVPMVATV (SEQ ID NO 217779)) and anti-CD3. Another sample of cells were stained with the irrelevant MHC-dextramer HLA-A*0201(GILGFVFTL) and anti-CD3. The cells stained with HLA-A*0201 (GILGFVFTL) had weak signals (low fluorescent intensity), and therefore regarded as the negative population. A boundary was introduced in the dot plot, to mark the negative population. Cells with fluorescence higher than the negative boundary were hereafter regarded positive. 19% and 0.25% of the cells were regarded positive when stained with the relevant and irrelevant MHC-dextramer, respectively. See table below.

| MHC-complex | Percentage of positive cells |
| --- | --- |
| HLA-A*0201 (NLVPMVATV) (SEQ ID NO 217779) | 19% |
| HLA-A*0201 (GILGFVFTL) | 0,25% |

The results thus correlate well with the expected 20-50% HLA-A*0201(NLVPMVATV) (SEQ ID NO 217779) positive JT3A cells after stimulation. We conclude that the transfected Jurkat cell line (JT3A) can be used as positive control for the MHC-dextramer.

**Example 44**

This example describes how the quality of a MHC multimer can be tested. The example also describes how MHC multimers can be used for detection of specific T-cell lines.

The MHC multimer in this example is a MHC-dextramer, and the test involves specific binding of the MHC-dextramer to cell preparations expressing TCRs.

Three different peptide specific T-cell preparations of Human cytotoxic T lymphocyte lines specific for viral peptides were incubated with fluorescently labeled MHC-dextramers and the extent of cell staining analyzed by flow cytometry. The following T-cell preparations were examined: (NLV) specific for MHC-dextramer HLA-A*0201(NLVPMVATV) (SEQ ID NO 217779), (IPSI) specific for MHC-dextramer B*3501(IPSINVHHY) and (GLC) specific for MHC-dextramer A*0201 (GLCLVALM).

Cells were added 1ml RPMI and then transfer to a tube with 9ml RPMI. Cells were centrifuged for 5 min at 300 g, and the supernatant was discarded.

Cells were washed in 10ml PBS pH 7.4 + 5% FCS, and centrifuged for 5 min at 300 g, and the supernatant was discarded.

$1\times10^6$ cells per sample in 100$\mu$l PBS pH 7.4 + 5% FCS were added to sample tubes. 10$\mu$l MHC Dextramers were added, and incubated at room temperature in the dark for 10 min.

5$\mu$l anti-CD3 and anti-CD8 were added to each sample. Further incubation for 20 min at 4°C in the dark.

Samples were washed in 2ml PBS pH 7.4 + 5% FCS and centrifuged for 5 min at 300 g, and the supernatant was discarded. Pellets were resuspended in 0.4 ml PBS pH 7.4.

Samples were kept in the dark at 2-8°C until analysis on a flow cytometer.

Samples were analyzed by flow cytometry on a CyAn instrument.

Data were analyzed by the Summit software. The cell preparations were stained with anti-CD3, anti-CD8, the respective specific MHC-dextramer, or an irrelevant MHC-dextramer. Anti-CD3 positive cells were positively gated and anti-CD8 vs. MHC-dextramer were depicted in a dot plot. The main population of anti-CD8 positive cells stained with the irrelevant MHC-dextramer was regarded as negative, and a boundary was introduced in the dot plot to mark the negative population. Anti-CD8 positive cells with fluorescence higher than the negative boundary were regarded positive. In the NLV and IPSI cell preparations, approximately 95% of the CD8$^+$ cells were positive for the relevant MHC dextramer. 45% of the CD8$^+$ GLC cells were positive for relevant MHC Dextramers, see table below. Cell preparations were not stained by the irrelevant MHC-dextramer.

We conclude that the different peptide specific T-cell preparations can be used as positive controls for the relevant MHC-dextramer.

| Cell preparation | MHC-complex | Percentage of positive cells |
|---|---|---|
| NLV | HLA-A*0201(NLVPMVATV) (SEQ ID NO 217779) | 97% |
| | HLA-B*3501(IPSINVHHY) | 0,02% |
| IPSI | HLA-B*3501(IPSINVHHY) | 95% |
| | HLA-A*0201(NLVPMVATV) (SEQ ID NO 217779) | 0,01% |
| GLC | HLA-A*0201(GLCLVALM) | 45% |
| | HLA-A*0201(ILKEPVHGV) (SEQ ID NO 217780) | 0,1% |

**Example 45**

This example describes how the quality of the MHC multimer can be tested. The example also describes how MHC multimers can be used for detection of specific T-cell lines.

The MHC multimer in this example is a MHC-dextramer, and the test involves specific binding of the MHC-dextramer to cell lines or other preparations expressing TCRs or MHC-recognizing molecules.

Experiments can be performed with any kind of TCRs or MHC-recognizing molecules displayed on cells (e.g. a cell line or a cell preparation).

Procedures as described in Example 43 and 44 can be used with any kind of TCRs or MHC-recognizing molecules displayed on cells. Stimulation of cells has to be optimized for the specific experiments (e.g. stimulation with chemicals and/or temperature to express the TCR prior to analysis).

Results can be analyzed as in Example 43 and 44 and cell preparations stained with relevant MHC-dextramers will extent higher signal intensity than when stained with irrevant MHC-dextramers.

**Example 46**

This example describes how the quality of the MHC multimer can be tested. The example also describes how MHC multimers can be used for detection of TCR immobilized to solid support or attached to other molecule.

The MHC multimer in this example is a MHC-dextramer, and the test involves specific binding of the MHC-dextramer to TCRs or MHC recognizing motifs immobilized or expressed on molecules detectable in flow cytometry.

The experiments can be performed with any kind of molecule detectable in flow cytometry. MHC-recognizing molecules can be displayed on beads, cells, or other entities that are amenable to flow cytometry analysis.

MHC-recognizing molecules can be displayed on entities amenable in flow cytometry either naturally or artificially (e.g. chemical coupling). Procedures as described in the above examples can be applied, depending on the nature of the molecules.

Alternatively procedures optimized for the specific molecule can be applied.

Molecules can be stained with flourescently labeled MHC-dextramers and the extent of cell staining can be analyzed by flow cytometry. Molecules stained with relevant MHC-dextramers will extent higher signal intensity than when stained with irrelevant MHC-dextramers.

**Example 47**

This example describes how the quantity of correctly folded MHC can be determined by ELISA assay. The test involves specific binding of the MHC to anti-HLA-ABC antibody, clone W6/32. W6/32 is an antibody recognizing all human MHC I HLA-A, B and C alleles but only when they are in the correct conformation and properly loaded with antigenic peptide.

The ELISA can be carried out as follows. A preparation of MHC complexes is incubated in wells of a microtiter plate pre-coated with W6/32 antibody following standard ELISA procedure regarding washes, blocking etc. A secondary antibody recognizing MHC (e.g. anti-$\beta_2$m) is used for visualization. The secondary antibody can be labbelled, e.g. with Horse raddish Peroxidase or it can be unlabelled, and a labeled compound specific for the secondary antibody is then employed (e.g. EnVision System) before visualization.. A compound for visualization is added, e.g. TMB One-Substrate System when Peroxidase enzyme is the label. The chromogenic intensity is measured and the result from the ELISA assay is evaluated. In parallel a standard curve of is generated, e.g. consisting of various concentrations of correctly folded MHC complexes. The chromogenic intensity of the tested MHC sample can now be converted to actual concentrations of correctly folded MHC using the standard curve. We conclude that the ELISA assay together with a standard curve can be used to quantify correctly folded MHC.

**Example 48**

This example describes how the quantity of correctly folded MHC can be determined by ELISA assay. The test involves specific binding of MHC complexes to a molecule recognizing correctly folded MHC (e.g. antibody, TCR, aptamers, or other MHC-peptide complex binding molecules).

As described elsewhere the quantity of correctly folded MHC can be measured in an ELISA assay using a molecule specific for correctly folded MHC complexes either for catching of the testet MHC complex preparation or for detection of it. The result obtained from bound MHC complex in the sample tested is correlated to a standard curve for determination of concentration. The ELISA assay can be conducted and optimized by various measures depending on the reagent and enzyme used.

**Example 49**

This example describes how the quantity of correctly folded MHC can be determined by competitive ELISA assay. The example also describes how MHC multimers can be used for detection of TCR immobilized to solid support.

The test involves specific binding of MHC complexes to a molecule recognizing correctly folded MHC (e.g. antibody, TCR, aptamers, or other MHC-peptide complex binding molecules). The analysis is carried out as follows. A preparation of correctly folded MHC of known concentration is immobilized in wells of a microtiter plate. A mixture of a molecule recognizing correctly folded MHC (e.g. the antibody W6/32) and soluble MHC complex, containing a mixture of correctly and non-correctly folded MHC, is added to the microtiter plate. The molecules recognizing correctly folded MHC will now attach to either the immobilized MHC or the correctly folded MHC in the soluble fraction. The amount of molecules recognizing correctly folded MHC bound to the immobilized MHC in the microtiter plate is measured, e.g. by addition of a secondary labeled or unlabeled antibody specific for the molecule recognizing correctly folded MHC. Depending on the characteristic of the secondary antibody visualization is performed. The result of the ELISA assay is evaluated. The amount of bound molecules specific for correctly folded MHC complexs is inversely proportional to the quantity of correctly folded MHC in the soluble fraction. The quantity of correctly folded MHC in the soluble fraction can be measured using a standard curve, obtained using known amounts of a pure preparation of MHC complexes and constant amount of molecules recognizing correctly folded MHC.

We conclude that an indirect competitive ELISA can be used to quantify correctly folded MHC.

## Example 50

This example describes how the quantity of correctly folded MHC can be determined by competitive ELISA assay. The example also describes how MHC multimers can be used for detection of TCR immobilized to solid support.

The test involves specific binding of MHC complexes to a molecule recognizing correctly folded MHC (e.g. antibody, TCR, aptamers, or other MHC-peptide complex binding molecules).

The analysis is carried out as follows. Molecules recognizing correctly folded MHC are immobilized in wells of a microtiter plate. A preparation of in vitro folded MHC complexes is mixed with a known amount of labeled correctly folded MHC and the mixure added to the microtiter plate. The correctly folded MHC in the mixture will now compete for binding to the molecules recognizing correctly folded MHC immobilized in the microtiter plate. The amount of bound labeled MHC can now be visualized and measured. The result is inversely proportional to the quantity of correctly folded MHC in the mixture. The quantity of correctly folded MHC in the mixture can be measured using a standard curve, obtained using known amounts of a pure preparation of MHC mixed with a constant amount of labeled MHC.

## Example 51

This example describes how the quantity of correctly folded MHC can be determined by competitive ELISA assay. The example also describes how MHC multimers can be used for detection of TCR immobilized to solid support.

The test involves specific binding of the MHC to a molecule recognizing correctly folded MHC (e.g. antibody, TCR, aptamers, or other MHC-peptide complex binding molecules) or to molecules recognizing MHC, correctly as well as non-correctly folded (e.g. antibody, TCR, aptamers, or other MHC-peptide complex binding molecules).

A preparation of MHC complexes are immobilized in a microtiter plate and incubated with a labeled molecule recognizing MHC, correctly as well as non-correctly folded (in the following referred to as recognizing all MHC complexes). Then an unlabeled molecule recognizing correctly folded MHC is added in major excess. These molecules will displace all the MHC recognizing molecules added in step 1 that binds correctly folded MHC complexes.

The amount of bound labeled molecule recognizing all MHC can now be visualized and measured.

The result is inversely proportional to the quantity of correctly folded MHC. The quantity of correctly folded MHC can be measured using a standard curve, obtained using known amounts of a MHC incubated with labeled molecule recognizing all MHC.

## Example 52

This example describes how the quantity of correctly folded MHC can be determined. The test involves specific binding of MHC complexes to anti-HLA-ABC antibody, clone W6/32. In this example W6/32 antibody is immobilized on magnetic beads.

The protein concentration in a purified preparation of MHC complexes is determined by spectofotometric measurement of $OD_{280}$. Other techniques can be used as well, e.g. BCA (bicinchoninic acid) assay or SDS-PAGE gel.

To measure the proportion of correctly folded MHC, a sample of the MHC complex preparation is incubated with W6/32 antibody coated magnetic beads.

After incubation the beads are sedimented or captured by a magnet and excess supernatant is withdrawn.

The concentration of protein in the supernatant is measured as in step 1.

The proportion of correctly folded MHC complex is calculated as follows:

X: Total protein concentration (step 1)
Y: Protein concentration after capture of correctly folded MHC (step 4)

$$\% \text{ of correct folded MHC} = (1 - (Y/X)) * 100\ \%$$

Ex. X = 10 mg/ml
Y = 2 mg/ml

$$\% \text{ of correct folded MHC} = (1- (2\ mg/ml\ /\ 10\ mg/ml))*\ 100\ \%$$

$$= 80\%$$

The proportion of correctly folded MHC can be used as quality parameter, to measure the variation from production to production of a specific MHC-peptide complex.

**Example 53**

This example describes how the quantity of correctly folded MHC can be determined. The example also describes how MHC multimers can be used for detection of TCR immobilized to solid support.
The test involves specific binding of MHC complexes to a molecule recognizing correctly folded MHC (e.g. antibody, TCR, aptamers, or other MHC-peptide complex binding molecules). The MHC recognizing molecules can be immobilized to a solid support (e.g. beads, microtiter plate or gel material).

The protein concentration in a purified preparation of MHC complexes is determined by spectofotometric measurement of $OD_{280}$. Other techniques can be used as well, e.g. BCA (bicinchoninic acid) assay or SDS-PAGE gel.
To measure the proportion of correctly folded MHC, a sample of the MHC complex preparation is incubated with a molecule recognizing correctly folded MHC complex immobilized to a solid support.
After incubation supernatant is withdrawn.
The concentration of protein in the supernatant is measured as in step 1.
The proportion of correctly folded MHC complex is calculated as follows:

X: Total protein concentration (step 1)
Y: Protein concentration after capture of correctly folded MHC (step 4)

$$\% \text{ of correct folded MHC} = (1- (Y/X))*\ 100\ \%$$

Ex. X = 10 mg/ml
Y = 2 mg/ml

$$\% \text{ of correct folded MHC} = (1- (2\ mg/ml\ /\ 10\ mg/ml))*\ 100\ \%$$

$$= 80\%$$

The proportion of correctly folded MHC can be used as quality parameter, to measure the variation from production to production of a specific MHC-peptide complex.

**Example 54**

This example describes how the quantity of correctly folded MHC can be determined. The test involves specific binding of MHC complexes to anti-HLA-ABC antibody, clone W6/32. In this example W6/32 antibody is immobilized on magnetic beads.

A preparation of MHC complexes is incubated with W6/32 antibody coated magnetic beads and all correctly folded MHCs are thereby captured.
After incubation the beads are sedimented or captured by magnet, d the supernatant withdrawn and the amount of non-bound MHC complex (equal to not correctly folded MHC complex) is determined by ELISA.
To measure the quantity of MHC complexes in solution before and after incubation with W6/32 antibody coated magnetic beads an ELISA assay is applied. A microtiter plate is coated with an antibody recognizing all MHC complexes correct as well as non-correct folded.
Samples of MHC complex before and after incubation with W6/32 antibody coated magnetic beads are added to the coated microtiter plate.
A labeled secondary antibody recognizing MHC (e.g. anti-$\beta_2$m) is used for visualization. Alternatively the secondary antibody is unlabelled, and a labeled molcule specific for the secondary antibody is employed (e.g. EnVision System) before visualization.
The proportion between results from the sample after incubation with W6/32 antibody coated magnetic beads and the sample before incubation with W6/32 antibody coated magnetic beads is a measure of the amount of non correct folded MHC complex and thereby an indirect measure of the amount of correct folded MHC complex . To obtain exact values for the amount of MHC complex in each sample a standard curve can be generated.

**Example 55**

This example describes how the quantity of correctly folded MHC in a sample can be determined. The example also describes how MHC multimers can be used for detection of TCR immobilized to solid support.

The test involves specific binding of MHC complexes to a molecule recognizing correctly folded MHC complex (e.g. antibody, TCR, aptamers, or other MHC-peptide complex binding molecules). The MHC recognizing molecules can be immobilized to any solid support (e.g. beads, microtiter plate or gel material). Correctly folded MHC complex in a sample is captured by addition of the sample to the immobilized MHC-binding molecule, then non-bound MHC-complex (equal to not corretly folded MHC complex) is removed. The proportion of MHC complex in the two fractions (before and after incubation with immobilized MHC-specific molecule) can be evaluated by any method capable of determination of the total amount of MHC complex (folded or undfodled).

**Example 56**

This example describes how the quantity of correctly folded MHC in a sample can be determined. The test involves specific binding of MHC complex to a molecule recognizing correctly folded MHC (e.g. antibody, TCR, aptamers, or other MHC-peptide complex binding molecules). The specific binding is evaluated by native protein gel electrophoresis.

A preparation of in vitro folded and purified MHC complex is incubated with a molecule recognizing correctly folded MHC, e.g. the antibody W6/32.

The product is analysed by native protein gel electrophoresis (lane 1) together with a sample of in vitro folded and purified MHC complex not incubated with MHC recognizing molecule (lane 2), and a sample of the molecule recognizing correctly folded MHC (lane 3). In the protein gel the different proteins are separated according to size. The various protein products are visualized in the gel e.g. by Comassie blue staining, isotope or chemiluminiscence. In the sample with MHC complex and MHC-binding molecule correctly folded MHC complex will bind the MHC-binding molecule and appear in the protein gel as one or more large bands in the top of the gel. In contrast non-correctly folded MHC complex will appear as monomer lower in the gel.

The fraction of not correctly folded MHC (lane 1) can now be compared with the total amount of MHC in the sample (lane 2) and the difference between the two correlates with the amount of correctly folded MHC complex in the sample. The results can be evaluated in different manners to determine the quantity of correctly folded MHC, e.g. comparison of intensity to the intensity of products of known concentration.

**Example 57**

This example describes how to verify that a MHC-complex is correctly folded by a sandwich-ELISA assay. W6/32 mouse-anti-HLA-ABC antibody (Dako M0736), that recognizes a conformational epitope on correctly folded MHC-complex, was used as coating-antibody. HRP-conjugated rabbit anti-$\beta$2m (Dako PO174) was used for visualization.

Wells of a microtiter plate was pre-coated with W6/32 antibody (Dako M0736, 5 $\mu$g/ml in 0.1M NaHCO$_3$, 1 mM MgCl$_2$, pH 9.8, 50 $\mu$l/well) following a standard ELISA procedure regarding washes and blocking ect.

After addition of 50 $\mu$l of 0.5M Tris-HCl, 0.1 M NaCl, 0.1% Tween 20, 0.01% Bronidox, pH 7.2 to each well, 50 $\mu$l of a sample of purified folded MHC-complex (in a concentration of approx. 0.4 mg/ml) was added to two wells in to columns in the microtiter plate, diluted 2-fold down the column and incubated 2 hours at 4°C. Light chain $\beta$2m (0.15 mg/ml in 0.5M Tris-HCl, 0.1 M NaCl, 0.1% Tween 20, 0.01% Bronidox, pH 7.2) was used as a negative control and the cell-line KG-1a, expressing HLA-A*30, HLA-A*31 and HLA-B*35 heavy chains, was used as positive control ($10^6$ cells/well).

After a standard ELISA wash, 50 $\mu$l of the detecting antibody; HRP-conjugated rabbit anti-$\beta$2m (Dako PO174), diluted 1:2500 in 1% Skimmed Milk in 0.5M Tris-HCl, 0.1 M NaCl, 0.1% Tween 20, 0.01% Bronidox, pH 7.2 was added to each well. The plate wass incubated 1 hour at 4°C.

After a standard ELISA wash, 50 $\mu$l of an amplifying antibody; HRP-Dextran500-conjugated goat anti-rabbit (Dako DM0106), diluted 1:2000 in 1% Skimmed Milk in 0.5M Tris-HCl, 0.1 M NaCl, 0.1% Tween 20, 0.01% Bronidox, 1% mouse serum (Dako X0190) pH 7.2 was added. The plate was incubated 30 min. at 20°C.

After a standard ELISA wash, 50 $\mu$l of Dako S1599 (TMB+Substrat Chromogen) was added to each well for visualization. After 10 min. the visualization reaction was stopped with 50 $\mu$l 0,5M H$_2$SO$_4$/well.

The chromogenic intensity was measured at OD$_{450}$ and the result from the ELISA assay evaluated.

As shown in figure 17 the OD$_{450}$ values from wells with MHC complex was more than 6 times higher than OD$_{450}$ values from wells with the negative control $\beta$2m. This ELISA procedure can be used to verify the presence of correctly folded MHC-peptide complexes in a preparation of MHC complexes.

## Example 58

This example describes the generation and application of negative controls, where the MHC complex is HLA-A*0201 loaded with either of the nonsense peptides GLAGDVSAV (SEQ ID NO 217778) or ALIAPVHAV (SEQ ID NO 217781) and these MHC complexes are coupled to a 270 kDa dextran multimerization domain. The nonsense peptides have an amino acid sequence different from the linear sequence of any peptide derived from any known naturally occurring protein. This was analyzed by a blast search. The amino acids at position 2 and 9 can serve as anchor residues when binding to HLA-A*0201 molecules.

Purified MHC(peptide) molecules consisting of the allele HLA-A*0201, human beta2microglobulin and peptide was generated by in vitro refolding, purified and biotinylated as described elsewhere herin. Biotinylated HLA-A*0201(peptide) was mixed with APC-SA-conjugated 270 kDa dextran in an amount corresponding to a ratio of three biotinylated HLA-A*0201(peptide) molecules per SA molecule and incubated for 30 minutes in the dark at room temperature. The APC-SA-conjugated 270 kDa dextran contained 9 molecules APC and 3,7 molecules SA per dextran molecule. Following incubation the mixture was diluted into a buffer containing 0,05M Tris/HCl, 15 nM NaN$_3$ and 1% BSA to a final concentration of $3,8 \times 10^{-8}$ M dextran.

By this procedure the following MHC multimer constructs were made:

A negative control construct comprising APC-SA-conjugated 270 kDa dextran and biotinylated HLA-A*0201 in complex with beta2microglobulin and the nonsense peptide GLAGDVSAV ((SEQ ID NO 217778) nonsense peptide 1).

A negative control construct comprising APC-SA-conjugated 270 kDa dextran and biotinylated HLA-A*0201 in complex with beta2microglobulin and the nonsense peptide ALIAPVHAV ((SEQ ID NO 217781) nonsense peptide 2).

A construct comprising APC-SA-conjugated 270 kDa dextran and biotinylated HLA-A*0201 in complex with beta2microglobulin and the peptide NLVPMVATV (SEQ ID NO 217779) derived from pp65 protein from human cytomegalovirus (HCMV).

A construct comprising APC-SA-conjugated 270 kDa dextran and biotinylated HLA-A*0201 in complex with beta2microglobulin and the peptide GLCTLVAML (SEQ ID NO 217782) derived from BMLF-1 protein from Epstein Barr virus (EBV).

A construct comprising APC-SA-conjugated 270 kDa dextran and biotinylated HLA-A*0201 in complex with beta2microglobulin and the peptide ILKEPVHGV (SEQ ID NO 217780) Reverse Transcriptase from Human Immunodeficiency Virus (HIV).

The binding of the HLA-A*0201(peptide)/APC dextran constructs to Human Peripheral Blood Mononuclear Cells (HPBMC) from various donors was analyzed by flow cytometry following a standard flow cytometry protocol. Briefly, HPBMC from the blood of 9 individual donors were isolated, by a standard protocol using Ficoll-Hypaque. $1 \times 10^6$ purified HPBMC at a concentration of $2 \times 10^7$ cells/ml were incubated with 10 μl of one of the HLA-A*0201(peptide)/APC dextran constructs described above for 10 minutes in the dark at room temperature. 10 μl of each of the antibodies mouse-anti-human CD3/PE (clone UCHT1 from Dako) and mouse-anti-human CD8/PB (clone DK25 from Dako) were added and the incubation continued for another 20 minutes at 4°C in the dark. The samples were then washed by adding 2 ml PBS; pH =7.2 followed by centrifugation for 5 minutes at 200xg and the supernatant removed. The cells were then resuspended in 400-500 μl PBS; pH=7.2 and analyzed on a CYAN ADP flowcytometer.

Donor 1-5 were known to have detectable T cells specific for HLA-A*0201(NLVPMVATV) (SEQ ID NO 217779) and no detectable T cells specific for HLA-A*0201(ILKEPVHGV) (SEQ ID NO 217780) while donor 6 were known not to have detectable specific T cells for either HLA-A*0201(NLVPMVATV) (SEQ ID NO 217779) nor HLA-A*0201(ILKEPVHGV) (SEQ ID NO 217780). Lymphocytes from these 6 donors were stained with MHC multimer construct 1, 2, 3, and 5. Donor 1-5 showed positive staining with MHC multimer construct 3 as expected while no staining was observed with the either of the negative control MHC complex constructs 1 and 2 or with MHC complex construct 5. An example showing the staining patterns for donor 2 is shown in figure 20. No specific staining was observed of lymphocytes from donor 6 with either of the MHC multimer constructs.

Donor 7-8 known to have detectable T cells specific for HLA-A*0201(GLCTLVAML) (SEQ ID NO 217782) and no detectable T cells recognizing HLA-A*0201(ILKEPVHGV ) (SEQ ID NO 217780) and donor 9 having no detectable T cells specific for either HLA-A*0201(GLCTLVAML) (SEQ ID NO 217782) nor HLA-A*0201(ILKEPVHGV) (SEQ ID NO 217780) were all stained with MHC multimer construct 1, 2, 4, and 5. Donor 7 and 8 demonstrated efficient staining with MHC multimer construct 4 as expected while no staining was observed with the other MHC multimer constructs tested. No staining was observed of lymphocytes from donor 9 with either of the MHC multimer constructs tested. A summary of the results is shown in figure 21.

In conclusion this experiment demonstrates that the negative MHC multimer constructs 1 and 2 did not stain any specific T cells in lymphocyte preparations from 10 different donors. Donors known to have specific T cells for either HLA-A*0201(GLCTLVAML) (SEQ ID NO 217782) or HLA-A*0201(NLVPMVATV) (SEQ ID NO 217779) also demonstrated positive staining with the corresponding MHC multimer constructs 3 and 4. None of the 10 donors were infected with HIV and as expected did not appear to have T cells specific for HLA-A*0201 in complex with the HIV derived peptide ILKEPVHGV (SEQ ID NO 217780), and as expected none of these donors showed staining with MHC multimere construct 5. MHC multimer construct 1 and 2 are therefore suitable negative controls when using HLA-A*0201(peptide) multimers for detection of specific T cells in Flow Cytometry.

**Example 59**

This is an example of how to generate and use negative controls, where the MHC complex is any MHC I or MHC II complex of human, mouse, rabbit, rat, swine or monkey origin loaded with a nonsense peptide. A nonsense peptide is here to be understood as a peptide having an amino acid sequence different from any peptide derived from any known naturally occurring protein, and preferably is not recognized by any T cell when presented by a MHC complex. The nonsense peptide has amino acid residues at relevant positions that anchor the peptide to the peptide-binding groove of the MHC complex. The MHC(nonsense peptide) complex is coupled to a 270 kDa dextran multimerization domain.

Purified MHC(peptide) molecules consisting of the alpha chain, human beta2microglobulin and peptide is generated by in vitro refolding, purified and biotinylated as described elsewhere. Biotinylated MHC(peptide) is mixed with APC-SA-conjugated 270 kDa dextran in amounts corresponding to a ratio of three biotinylated MHC(peptide) molecules per SA molecule and incubated for 30 minutes in the dark at room temperature. The APC-SA-conjugated 270 kDa dextran contains 9 molecules APC and 3,7 molecules SA per dextran molecule. Following incubation the mixture is diluted into a buffer containing 0,05M Tris/HCl, 15 nM $NaN_3$ and 1% BSA to a final concentration of $3,8\times10^{-8}$ M dextran. By this procedure the following MHC complex constructs are made:

A negative control construct comprising APC-SA-conjugated 270 kDa dextran and biotinylated alpha chain in complex with beta2microglobulin and a corresponding nonsense peptide. A nonsense peptide is here to be understood as a peptide with an amino acid sequence different from any peptide derived from any known naturally occurring protein and the nonsense peptide is not recognized by any T cell when presented by a MHC complex.

A construct comprising APC-SA-conjugated 270 kDa dextran and biotinylated alpha chain in complex with beta2microglobulin and a peptide derived from a known protein.

The binding of the MHC(peptide)/APC dextran constructs to Human Peripheral Blood Mononuclear Cells (HPBMC) from various donors is analyzed by flow cytometry following a standard flow cytometry protocol. Briefly, HPBMC from the blood of 9 individual donors are isolated by a standard protocol using Ficoll-Hypaque. $1\times10^6$ purified HPBMC at a concentration of $2\times10^7$ cells/ml is incubated with 10 µl of one of the MHC(peptide)/APC dextran constructs described above for 10 minutes in the dark at room temperature. 10 µl of each of each of the antibodies mouse-anti-human CD3/PE (clone UCHT1 from Dako) and mouse-anti-human CD8/PB (clone DK25 from Dako) are added and the incubation continued for another 20 minutes at 4°C in the dark. The samples are then washed by adding 2 ml PBS; pH =7.2 followed by centrifugation for 5 minutes at 200xg and the supernatant removed. The washed cells are resuspended in 400-500 µl PBS; pH=7.2 and analyzed on a CYAN ADP flowcytometer.

The staining patterns of flow cytometry analysis with the two MHC(peptide)/APC constructs 1 and 2 are compared. There will be no staining observed with construct 1.

**Example 60**

This is an example of how to generate negative controls, where the MHC complexes is any MHC I or MHC II molecule of human, mouse, rabbit, rat, swine, monkey or any other origin loaded with a nonsense peptide and where the MHC(nonsense peptide) complexes are coupled to any multimerization domain. A nonsense peptide is here to be understood as a peptide that have an amino acid sequence different from any peptide derived from any known naturally occurring protein and cannot be recognized by any T cell when presented by a MHC complex. The nonsense peptide carries amino acid residues at relevant positions that anchor the peptide to the peptide-binding groove of the MHC complex.

The MHC(nonsense peptide) complex can be made as described elsewhere herein, and can then be coupled to a relevant multimerization domain. The labelling of the multimerization domain can be optimized depending on later use of the negative control e.g. in flow cytometry analysis, IHC, ELISA or similar.

**Example 61**

This example describes the generation of a negative control, where the MHC complex is HLA-A*0201 coupled to a 270 kDa dextran, and where the MHC is loaded with the peptide ILAKFLHWL that have pivaloyl coupled to Lysine at position 4. ILAKFLHWL is a peptide derived from telomerase and is known to bind HLA-A*0201. Pivaloyl is a small molecule that confers high sterical hindrance. Because pivaloyl is placed at a central position in the peptide it is likely to inhibit or completely abrogate the interaction with a specific TCR, because TCR-recognition is normally directed to the middle of the peptide when bound in the peptide-binding cleft. In the following the pivaloyl-modified peptide will be designated ILAKpFLHWL.

Purified HLA-A*0201(ILAKpFLHWL) molecules consisting of the HLA-A*0201 heavy chain, human beta2microglobulin and ILAKpFLHWL peptide is generated by in vitro refolding, purified and biotinylated as described elsewhere herein. Biotinylated HLA-A*0201(ILAKpFLHWL) molecules are mixed with flourochrome-SA-conjugated 270 kDa dextran molecules. The resulting HLA-A*0201(ILAKpFLHWL)/flourochrome-carrying dextran molecules can be used as negative controls in e.g. flow cytometric analysis.

**Example 62**

This example describes the generation of a negative control, where the MHC complex is any MHC I or MHC II molecule of human, mouse, rabbit, rat, swine, monkey or any other origin loaded with the peptide ILAKpFLHWL and coupled to any multimerization domain labeled with fluorochrome, HRP or any other label. Purified MHC(ILAKpFLHWL) complexes consisting of the heavy chain, human beta2microglobulin and ILAKpFLHWL peptide is generated by in vitro refolding, purified and biotinylated as described elsewhere herein. Biotinylated MHC(ILAKpFLHWL) complexes are mixed with labeled multimerization domain, thereby generating MHC(ILAKpFLHWL) multimers. The MHC(ILAKpFLHWL) multimers mayn be used as negative controls in e.g. flow cytometric analysis, IHC, ELISA or similar.

**Example 63**

This example describes the generation of a negative control where the MHC is HLA-A*0201 loaded with the peptide ILKEPVHGV (SEQ ID NO 217780) and coupled to a 270 kDa dextran multimerization domain. The peptide ILKEPVHGV (SEQ ID NO 217780) is derived from HIV. HLA-A*0201(ILKEPVHGV) (SEQ ID NO 217780) complexes are generated, biotinylated and coupled with a labeled dextran multimerization domain as described elsewhere herein. Then these HLA-A*0201 (ILKEPVHGV) (SEQ ID NO 217780)-dextran conjugates are used as negative controls in experiments with samples from humans that are HLA-A*0201 positive and not infected by HIV, because it is not likely that they have T cell's with a TCR specific for HLA-A*0201(ILKEPVHGV) (SEQ ID NO 217780) in amounts that can be detected by MHC multimer reagents. The conjugates can also be used as negative control in samples from humans that are HLA-A*0201 negative or in samples from other species.

**Example 64**

This example describes the generation of a negative control. CD8 molecules on the surface of cytotoxic T-cells bind MHC I complexes by interaction with amino acids in the $\alpha$3 domain of the heavy chain. When using MHC multimers this interaction can result in MHC multimer binding to CD8 positive T-cells that are not restricted by the MHC(peptide) complex in the MHC multimer. In this example a proper negative control showing these false positive cells is described. Such a negative control can consist of MHC I heavy chain or truncated versions of MHC I heavy chain bound to any multimerization domain. The heavy chain can be folded or unfolded, and can have a peptide bound in the peptide binding groove but does not have to comprise a peptide. A MHC I heavy chain-multimerization domain can be made in a way similar to what is described for the generation of MHC I-multimerization domains. Briefly, purified MHC I heavy chain is expressed as inclusion bodies in E.coli and purified by general procedures. The heavy chain is refolded in vitro, purified and biotinylated, or the heavy chain can be biotinylated directly without refolding. Biotinylated heavy chain is coupled to any multimerization domain. The multimerization domain can be labeled with any suitable label or can be unlabeled depending on later use. Such a negative control can be used in experiments with MHC I multimers, e.g. in flow cytometry analysis, IHC, or ELISA.

**Example 65**

This example describes how to identify specific T cells in a blood sample with MHC multimers using flow cytometry analysis without lysis of red blood cells and without washing the cells after staining. MHC complexes in this example consisted of HLA-A*0201 heavy chain, human beta2microglobulin and different peptides, and the MHC complexes were coupled to a 270 kDa dextran multimerization domain.

Purified MHC-peptide complexes consisting of human heavy chain, human beta2microglobulin and peptide were generated by in vitro refolding, purified and biotinylated as described elsewhere herein. Biotinylated MHC-peptide complexes were then coupled to a 270 kDa dextran multimerization domain labelled with PE by interaction with streptavidin (SA) on the dextran multimerization domain. The SA-PE-dextran was made as described elsewhere herein. MHC-peptide complexes was added in an amount corresponding to a ratio of three MHC-peptide moleculess per SA molecule and each molecule dextran contained 6.1 SA molecule and 3.9 molecules PE. The final concentration of dextran was 3.8x10e-8 M. The following constructs were made:

PE conjugated 270 kDa dextran coupled with HLA-A*0101 in complex with beta2microglobulin and the peptide VTEHDTLLY (SEQ ID NO 217783) derived from Human Cytomegalo Virus (HCMV).

PE conjugated 270 kDa dextran coupled with HLA-A*0101 in complex with beta2microglobulin and the peptide IVDCLTEMY (SEQ ID NO 217784) derived from ubiquitin specific peptidase 9 (USP9).

PE conjugated 270 kDa dextran coupled with HLA-A*0201 in complex with beta2microglobulin and the peptide NLVPMVATV (SEQ ID NO 217779) derived from Human Cytomegalo Virus (HCMV).

PE conjugated 270 kDa dextran coupled with HLA-A*0201 in complex with beta2microglobulin and the peptide ILKEPVHGV (SEQ ID NO 217780) derived from Human Immunodeficiency Virus (HIV).

PE/SA conjugated 270 kDa dextran coupled with HLA-B*0207 in complex with beta2microglobulin and the peptide TPRVTGGGAM (SEQ ID NO 217785) derived from Human Cytomegalo Virus (HCMV).

PE conjugated 270 kDa dextran coupled with HLA-B*0207 in complex with beta2microglobulin and the peptide RPHERNGFTVL (SEQ ID NO 217786) derived from Human Cytomegalo Virus (HCMV).

PE conjugated 270 kDa dextran coupled with HLA- B*0207 in complex with beta2microglobulin and the peptide TPGPGVRYPL (SEQ ID NO 217787) derived from Human Immunodeficiency Virus (HIV).

These seven MHC multimer constructs were used for detection of specific T cells in flow cytometry analysis using a no-lyse no-wash procedure. Blood samples from three individual donors were analyzed. The donors had previously been screened for the presence of specific T cells using a general staining procedure including lysis and wash of the cell sample, and donor one turned out to be positive for HLA*0201 in complex with the peptide NLVPMVATV (SEQ ID NO 217779), donor two were positive for HLA*0101 in complex with the peptide VTEHDTLLY (SEQ ID NO 217783) and donor three were positive for HLA-B*0207 in complex with the peptides TPRVTGGGAM (SEQ ID NO 217785) and RPHERNGFTVL (SEQ ID NO 217786). In this experiment blood from each donor were analyzed with the MHC multimer construct they were supposed to have specific T cells restricted for and with MHC multimers of same haplotype but carrying a negative control peptide. The negative control peptides were either derived from HIV or the self-protein USP 9. Self-protein here means a naturally occurring protein in normal cells of a human individual. Normal healthy donors not infected with HIV are not expected to have specific T cells recognizing HIV derived peptides or peptides derived from self-proteins in complex with any HLA molecule in an amount detectable with this analysis method.

The blood was stained as follows:

100 μl EDTA stabilized blood were incubated with 5 μl MHC(peptide)/PE dextran for 5 minutes at room temperature. Anti-CD45/PB, anti-CD3/FITC and anti-CD8/APC antibody in an amount of 0.4-1.2 μg/sample was added to each tube and the incubation continued for another 15 minutes. 850 μl PBS; pH = 7.2 was added and the sample analyzed on a CyAn ADP flowcytometry instrument with a speed of 150 μl/minute. A total of 20.000 CD8 positive cells were acquired. During analysis CD45/PB antibody was used to set a trigger discriminator to allow the flow cytometer to distinguish between red blood cells and stained white blood cells (see figure 22A). Furthermore CD3/FITC antibody was used to select CD3 positive cells in a second gating strategy (see figure 22B).

Blood from donor one showed specific staining with HLA-A*0201(NLVPMVATV) (SEQ ID NO 217779) multimer (construct 3) while no staining of specific T cells was observed with the negative control HLA-A*0201(ILKEPVHGV) (SEQ ID NO 217780) multimer (construct 4). Donor two showed specific staining with HLA-A*0101(VTEHDTLLY) (SEQ ID NO 217783) multimer (construct 1) and no staining was observed with the negative control HLA-A*0101(IVDCLTEMY) (SEQ ID NO 217784) multimer (construct 2). In blood from donor three a population of T cells were stained with HLA-B*0207(TPRVTGGGAM) (SEQ ID NO 217785) multimer (construct 5) and another population with HLA-B*0207(RPHERNGFTVL) (SEQ ID NO 217786) multimer (construct 6) while no specific staining was observed with the negative control HLA-B*0207(TPGPGVRYPL) (SEQ ID NO 217787) multimer (construct 7). The results are shown in figure 23.

We have shown that MHC multimers of three different haplotypes can be used to identify specific T cells in blood samples from three different donors using an approach without lysing red blood cells and without wash following staining with MHC multimer. This method is simple, fast and interfere as little as possible with cells in the blood sample.

**Example 66**

This example describes how to identify specific T cells in a blood sample with MHC multimers using flow cytometry analysis without lysis of red blood cells and without washing cells upon staining. The MHC complex is here any MHC I or MHC II molecule of human, rodent, bovine, monkey or any other origin loaded with any peptide able to bind the peptide-binding cleft of the MHC complex and where the MHC-peptide complexes are coupled to any multimerization domain.

Purified MHC-peptide complexes is generated as described elsewhere herein and coupled to any multimerization domain labelled with a fluorescent dye, preferable FITC, PE, APC, pacific blue, cascade yellow or any other flour chrome. These MHC multimers are used for detection of specific T cells by flow cytometry using the following procedure: EDTA stabilized blood are incubated with MHC multimer at room temperature. The amount of blood analyzed is preferable 50-150 μl but could be any volume ranging from 1 -1000 μl. The amount of MHC multimer depends on the multimer construct and the volumen of blood and should be determined by titration prior to this type of experiment. The incubation time with MHC multimer is preferably 5-20 minutes but could be anything between 0 minutes and 1 hour. Then anti-CD45/CY, anti-CD3/APC and anti-CD8/PB antibody is added and the incubation continued. The incubation time is preferably 5-20 minutes but can be anything between 1 minute and 1 hour. The amount of antibody is preferable 0.4-1.2 μg /100 μl blood but these limits can be extended and should be determined by titration prior to this kind of experiments. The antibodies can be labelled with any fluorochrome as long as the fluorochrome is different from the fluorochrome on the MHC multimer. Next PBS; pH = 7.0-8.0 is added and the sample analyzed by a flowcytometer. The amount of PBS added is preferable 500-1000 μl but can also be more than 1000 μl and less than 500 μl. During analysis anti-CD45 antibody is used to set a trigger discriminator to allow the flow cytometer to distinguish between red blood cells and stained white blood cells. Different gating strategies can then be applied to analyse data. Preferably cells are first gated on CD3 positive cells and then for CD8 positive cells, but can also be gated only for CD8 positive cells of only for CD3 positive cells. Alternatively "dump" gates can be applied excluding unwanted cells, e.g. B-cells, CD4-positive cells, NK-cells.

In the above example MHC multimers are added prior to antibodies but MHC multimers and antibodies can also be added simultaneously to the blood sample and incubated for preferably 5-30 minutes but the incubation time can be anything between 1 minute and 2 hours.

This method can be used to identify specific T cells in blood samples fromfdifferent donors using an approach without lysing red blood cells and without wash following staining with MHC multimer. This method is simple, fast and interfere as little as possible with cells in the blood sample

## Example 67

This example illustrates how MHC multimers together with counting beads was used for exact numeration of MHC-peptide specific T cells in a flow cytometry analysis whit no lyses of red blood cells and no washing steps during or after staining. Counting beads in this example was CytoCount™, Count Control Beads from Dako that are polystyrene Fluorospheres with a diameter of 5.2 μm. The MHC multimer consisted of HLA-A*0101 heavy chain complexed with human beta2microgloblin and a peptide and the MHC-peptide complexes were coupled to a 270 kDa dextran multimerization domain labelled with PE. MHC multimers were generated as described elsewhere herein and the following two constructs were made:

PE conjugated 270 kDa dextran coupled with HLA-A*0101 in complex with beta2microglobulin and the peptide VTEHDTLLY (SEQ ID NO 217783) derived from Human Cytomegalo Virus (HCMV).
PE conjugated 270 kDa dextran coupled with HLA-A*0101 in complex with beta2microglobulin and the peptide IVDCLTEMY (SEQ ID NO 217784) derived from ubiquitin specific peptidase 9 (USP9).

Construct 2 is a negative control for construct 1 in this example and both were used for detection of specific T cells by flow cytometry using a no-lyse no-wash procedure:
100 μl of EDTA stabilized blood from a donor positive for HLA*0101 in complex with the peptide VTEHDLLY were incubated with 5 μl MHC multimer for 5 minutes at room temperature. Anti-CD45/CY, anti-CD3/PB and anti-CD8/APC antibody in an amount of 0.4-1.2 μg/sample was added and the incubation continued for another 15 minutes.

850 μl PBS; pH = 7.2 was added together with precise 50 μl CytoCount beads 1028 bead/μl and the sample analyzed on a CyAn ADP flowcytometry instrument with a speed of 150 μl/minute. A total of 20.000 CD8 positive cells were acquired. During analysis CD45/CY antibody was used to set a trigger discriminator to allow the flow cytometer to distinguish between red blood cells and stained white blood cells.

A dot plot was made for each sample showing MHC multimer vs CD8 positive events (se figure 24A and B). Based on the negative control a gate containing events representing CD8 positive T cells specific for MHC multimer was defined. Similarly histogram plots for each sample were made showing FITC signal vs counts (figure 24 C and D). In these histograms the amount of beads in the analyzed sample were identified since the beads in contrast to the cells emit light in the FITC channel. In principle the beads could be visualized in any fluorochrome channel because they emit light in all channels but it was important to visualize the beads in a channel where there was no interfering signal from labelled cells.

The concentration of T cells specific for HLA-A*0101 (VTEHDTLLY) (SEQ ID NO 217783) multimer (construct 1) in the blood sample were determined using the counting beads as an internal standard. Events obtained from staining with the negative control MHC multimer, construct 2, were defined as background signals and subtracted from the result obtained from staining with construct 1.

Concentration of HLA-A*0101(VTEHDTLLY) (SEQ ID NO 217783) specific T cells in

the blood sample =

((Count of MHC multimer+ CD8+ positive cells, construct 1 x concentration of beads x

dilution factor of beads) /counted beads))- ((Counted MHC multimer+ CD8+ cells,

construct 2 x concentration of beads x dilution factor of beads) /counted beads) = 992,6

cells/ml

For details see figure 24.

This experiment demonstrated how CytoCount™ counting beads together with MHC multimers could be used to determine the exact concentration of MHC-peptide specific T cells in a blood sample using a no-lyse no-wash method.

**Example 68**

This example describes an analysis of specific T cells in blood using MHC multimers where MHC multimers together with antibodies are pre-mixed and embedded in a matrix material to retain and immobilize the reagents prior to use. In this example the matrix was composed of Trehalose and Fructose and the MHC complex consisted of HLA-A*0101 heavy chain complexed with human beta2microglobulin and peptide. The MHC-peptide complexes were coupled to a 270 kDa dextran multimerization domain.

Purified MHC-peptide complexes consisting of heavy chain, human beta2microglobulin and peptide were generated by in vitro refolding, purified and biotinylated as described elsewhere herein. Biotinylated MHC(peptide) complexes were coupled to a 270 kDa dextran multimerization domain labelled with PE, thereby generating PE labelled MHC multimers. The following MHC multimer constructs were made:

PE conjugated 270 kDa dextran coupled with HLA-A*0101 in complex with beta2microglobulin and the peptide VTEHDTLLY (SEQ ID NO 217783) derived from Human Cytomegalo Virus (HCMV).
PE conjugated 270 kDa dextran coupled with HLA-A*0101 in complex with beta2microglobulin and the negative control peptide IVDCLTEMY (SEQ ID NO 217784) derived from ubiquitin specific peptidase 9 (USP9).

Tubes with a matrix material to retain and immobilize the above described MHC multimer constructs together with antibodies relevant for later flow cytometer analysis were made. The matrix material was made to retain MHC multimer and antibody in the container when dry but release them into the sample medium when a sample containing cells of interest was added to the tube.
Experimentally, solutions of 20% Fructose in water and 20% Trehalose in water were made and mixed in a 1:1 ratio. 15 μl of this mixture were transferred to two 5 ml Falcon tubes. A premix of antibodies were made consisting of 40 μl anti-CD8 Alexa700 labelled antibody in a concentration of 25 μg/ml + 40 μl anti-CD3 Pacific Blue labelled antibody in a concentration of 100 μg/ml + 160 μl anti-CD45 Cascade Yellow labelled antibody in a concentration of 200 μg/ml. 12 μl of this mixture were added to each Falcon tube together with 3 μl of either of the two MHC multimer constructs. 100 μl butylated hydroxytoluen (BHT) with a concentration of 99 mg/L were added. The mixtures were dried under vacuum a 2-8°C over night. 100 μl EDTA stabilized blood from a donor with T cells specific for HLA-A*0101 complexed with the peptide VTEHDTLLY (SEQ ID NO 217783) were added to each of the two tubes. As a control experiment 6 μl of the antibody premix described above were transferred to two empty 5 ml Falcon tubes together with 3 μl of either of the MHC multimer constructs and 100 μl blood from the same donor. All four tubes were incubated for 15 minutes at room temperature. Then 900 μl PBS; pH = 7.2 was added and the sample analyzed on a CyAn ADP flowcytometer instrument. A total of 20.000 CD8 positive cells were acquired for each sample. During analysis CD45/CY antibody was used to set a trigger discriminator to allow the flow cytometer to distinguish between red blood cells and stained white blood cells. As expected and shown in figure 25 a population of CD8 positive and HLA-A*0101(VTEHDTLLY) (SEQ ID NO 217783) multimer positive cells were observed in the two samples stained with construct 1. The amount of specific T cells detected in the matrix sample was comparable to the amount of specific T cells detected in the control sample without matrix material. No HLA-A*0101(IVDCLTEMY) (SEQ ID NO 217784) multimer specific CD8 positive cells were observed in the two samples stained with the negative control MHC multimer construct 2.
This experiment demonstrates that the MHC multimer constructs used in this experiment can be embedded in a sugar matrix and later used for analysis of specific T cells in a blood sample and that this method gives results comparable to results obtained from a no-lyse no-wash staining procedure.

**Example 69**

This example describes an analysis of specific T cells in blood or other samples with cells in solution using MHC multimers where MHC multimers together with antibodies are pre-mixed and embedded in a matrix material to retain and immobilize the reagents prior to use. In this example the matrix is composed of Trehalose and Fructose and the MHC multimer is any MHC I, MHC II or MHC like molecule.

Tubes with a matrix material to retain and immobilize MHC multimer constructs together with antibodies relevant for later flow cytometer analysis are made. The matrix material is made to retain MHC multimer and antibody in the container when dry but release them into the sample medium when a sample containing cells of interest is added to the tube. The matrix is preferable water-soluble sugar mixtures but can be any contiguous mass releasing its components upon addition of aqueous solution. The matrix embedding medium can comprise one or more compounds including carbohydrates, polymers, small proteins etc. Examples of carbohydrates for use in the matrix include saccharose, arabinose, ribulose, fructose, sorbose, glucose, mannose,gulose, galactose, sucrose, lactose, maltose, trehalose, raffinose and melizitose. Examples of suitable polymers for use in matrix include polyvinyl alcohols, polyethylene glycols, polyethylene imines, polyacryl amides, polyaziridines, glycols, polyacrylic acids, esters or derivatives thereof. Examples of small proteins include BSA, other albumins or protein fragments.
The matrix-embedding medium is transferred to tubes preferable 5 ml tubes or other tubes usable in flow cytometry. Fluorochrome antibodies are added and here means any antibody useable for gating when analysing samples with T lymphocytes. Preferable antibodies are directed against CD8, CD4, CD3, CD45, CD27, CD28, CD45RA, CD45RO and CD62L. Then MHC multimer constructs are added but can also be added simultaneously with the antibodies or before the antibodies. Addition of MHC multimer is not restricted to one type of MHC multimer but several different MHC multimers can be added to the same tube and thereby embedded in the same matrix sample. Optionally scavengers for oxygen-derived radicals can be added. Examples of such radical scavengers are ascorbic acid, beta-carotene, bilirubin, butylated hydroxytoluene, butylated hydroxyanisol, tert-butylhydroquinone, d-alpha-tocopherol, trolox and hydroxyanisol. The matrix mixtures are then dried under vacuum a 2-8°C over night.

Cell samples in solution are added to the dry or semidry matrix tubes. Cell samples here means any sample containing specific T cells. That is preferable whole blood, homogenized spleen, lymph nodes, tumors or similar or purified lymphocytes from any of the above. The samples are incubated at room temperature for 1 minute to 2 hours, preferable 10-30 minutes. They can also be incubated at 4°C or 37°C or any temperature in between those two. The samples are analyzed on a flowcytometry instrument.

As an alternative to the above described method MHC multimers can be added to the sample after addition of cell sample thereby only embedding antibodies and not MHC multimer in the matrix. Similarly only MHC multimer are embedded in the matrix and antibodies added after addition of cell sample.

For enumeration of specific T cells in the samples counting beads can be embedded in the matrix medium. The beads are then added before, together with or after adding antibodies and MHC multimer. Alternatively counting beads are added following incubation with cell sample. The exact amount of specific T cells are determined as described elsewhere herein.

**Example 70**

This is an example of how to make and use MHC multimers where the multimerization domain is dextran and the MHC complexs are chemically conjugated to the dextran multimerization domain.

MHC complexes consisting of HLA-A*0201 heavy chain, beta2microglobulin and NLVPMVATV (SEQ ID NO 217779) peptide or the negative control peptide GLAGDVSAV (SEQ ID NO 217778) are generated as described elsewhere herein. Dextran with a molecular weight of 270 kDa is activated with divinylsulfone. Activated Dextran is then incubated with MHC and RPE in a 0.05 M $NaCHO_3$ buffer; pH = 9.5 with a molar ratio between MHC and Dextran of 30-60 and a molar ratio between RPE and dextran of 3-7 :1 The mixture is placed in a water bath at 30°C for 16 hours. Excess flourochrome, MHC and dextran are removed by FPLC using a sephacryl S-300 column.

These MHC/RPE dextramers are then used to stain CMV specific T cells in a flow Cytometry experiment. Briefly, $1x10^6$ purified HPBMC from a donor with T cells specific for HLA-A*0201(NLVPMVATV) (SEQ ID NO 217779) are incubated with 10 $\mu$l of each of the two HLA-A*0201(peptide)/RPE constructs described above for 10 minutes in the dark at room temperature with a cell concentration of $2x10^7$ cells/ml. 10 $\mu$l mouse-anti-human CD8/PB antibody (clone DK25 from Dako) are added and the incubation continued for another 20 minutes at 4°C in the dark. The samples are then washed by adding 2 ml PBS; pH =7.2 followed by centrifugation for 5 minutes at 200xg and the supernatant removed. The cells are then resuspended in 400-500 $\mu$l PBS; pH=7.2 and analyzed on a flow cytometer.

**Example 71**

This is an example of how to make and use MHC multimers where the multimerization domain is dextran and MHC complexs are MHC I molecules chemically conjugated to dextran multimerization domain and the dextran multimerization domain also have fluorochrome chemically coupled.

Human beta2microglobulin is coupled to dextran as follows. Dextran with a molecular weight of 270 kDa is activated with divinylsulfone. Activated dextran is incubated with human beta2microglobulin and RPE in a 0.05 M NaCHO$_3$ buffer; pH = 9.5 with a molar ratio between beta2microglobulin and Dextran of 30-60 and a molar ratio between RPE and dextran of 3-7:1. The molar ratio of the final product is preferable 4-6 RPE and 15-24 beta2microglobulin per dextran. The mixture is placed in a water bath at 30°C for 16 hours. Excess flourochrome, beta2microglobulin and dextran are removed by FPLC using a sephacryl S-300 column. The beta2microglobulin-RPE-dextran construct is then refolded in vitro together with heavy chain and peptide using the following procedure. 200 ml refolding buffer (100 mM Tris, 400 mM L-arginin-HCL, 2 mM NaEDTA, 0,5 mM oxidized Gluthathione, 5 mM reduced Glutathione, pH 8.0) supplied with protease inhibitors PMSF, Pepstatin A and Leupeptin (to a final concentration of 1 mM, 1 mg/l and 1 mg/l, respectively) is made and cooled to 10°C. 12 mg NLVPMVATV (SEQ ID NO 217779) peptide is dissolved in DMSO and added to the refolding buffer together with 20-30 mg beta2microglobulin-RPE-dex and 6 mg HLA-A*0201 heavy chain. Incubation at 10°C for 4-8 hours, then 20-30 mg beta2microglobulin-RPE-dex and 6 mg HLA-A*0201 heavy chain is added and incubation continued for 4-8 hours.

Another 20-30 mg beta2microglobulin-RPE-dex and 6 mg HLA-A*0201 heavy chain is added and incubation continued for 6-8 hours. The folding reaction is filtrated through a 0,2 μm filter to remove larger aggregates and then buffer exchanged into a buffer containing 20 mM Tris-HCl, 50 nM NaCl; pH = 8.0 followed by concentration to 1-2 ml sample. Dextran-RPE-MHC complexes are then separated from excess heavy chain and peptide by size exclusion chromatography using a sephacryl S-300, S-400 or sephacryl S-500 column.

These MHC/RPE dextramers can be used to stain CMV specific T cells in a flow Cytometry experiment. Briefly, $1x10^6$ purified HPBMC from a donor with T cells specific for HLA-A*0201(NLVPMVATV (SEQ ID NO 217779)) are incubated with 10 μl of each of the two HLA-A*0201(peptide)/RPE constructs described above for 10 minutes in the dark at room temperature with a cell concentration of $2x10^7$ cells/ml. 10 μl of mouse-anti-human CD8/PB antibody (clone DK25 from Dako) are added and the incubation continued for another 20 minutes at 4°C in the dark. The samples are then washed by adding 2 ml PBS; pH =7.2 followed by centrifugation for 5 minutes at 200xg and the supernatant removed. The cells are then resuspended in 400-500 μl PBS; pH=7.2 and analyzed on a flowcytometer.

**Example 72**

This is an example of how MHC multimers may be used for diagnosis of Lyme Disease in blood samples from humans infected with Borrelia bacteria. The MHC multimer used are MHC complexes coupled to labeled dextran. Lyme disease is caused by infection by Borrelia bacteria. During acute infection Borrelia specific activated T cells will be present in increased amounts in an activated state compared to healthy individuals. The presences of an increased amount of activated Borrelia specific T cells may thereby act as a surrogate marker for infection with Borrelia bacterium. MHC multimers carrying borrelia specific antigenic peptides is in this example used to detect the presence of Borrelia specific T cells in the blood of a patient infected with Borrelia.

Purified MHC-peptide complexes consisting of HLA-A*0201 heavy chain, human beta2microglobulin and peptide derived from regions in Outer surface protein B conserved among the three species Borrelia Burgdorferi, Borrelia Garinii and Borrelia Afzelii or a negative control peptide were generated by in vitro refolding, purified and biotinylated as described elsewhere herein. Biotinylated MHC-peptide complexes were then coupled to a 270 kDa dextran multimerization domain labelled with APC by interaction with streptavidin (SA) on the dextran multimerization domain. The dextran-APC-SA multimerization domain was generated as described elsewhere herein. MHC-peptide complexes were added in an amount corresponding to a ratio of three MHC-peptide molecules per SA molecule and each molecule dextran contained 3.7 SA molecule and 8.95 molecules APC. The final concentration of dextran was 3.8x10e-8 M. The following MHC(peptide)/APC dextran constructs were made:

APC-SA conjugated 270 kDa dextran coupled with HLA-A*0201 in complex with beta2microglobulin and the peptide FLTDGTITV derived from OspB.
APC-SA conjugated 270 kDa dextran coupled with HLA-A*0201 in complex with beta2microglobulin and the peptide SITDDLNTI derived from OspB.
APC-SA conjugated 270 kDa dextran coupled with HLA-A*0201 in complex with beta2microglobulin and the peptide KIKDFVFLT derived from OspB
APC-SA conjugated 270 kDa dextran coupled with HLA-A*0201 in complex with beta2microglobulin and the nonsense peptide GLAGDVSAV (SEQ ID NO 217778).

The binding of the above described MHC(peptide)/APC dextran can be used to determine the presence of Osp B specific T cells in the blood from Borrelia infected individuals by flow cytometry following a standard flow cytometry protocol.

Blood from a patient with Lyme disease is isolated and 100 ul of this blood is incubated with 10 μl of one of the MHC(peptide)/APC dextran constructs described above for 10 minutes in the dark at room temperature. 5 μl of each of each of the antibodies mouse-anti-human CD3/PB (clone UCHT1 from Dako) and mouse-anti-human CD8/PE (clone DK25 from Dako) are added and the incubation continued for another 20 minutes at 4°C in the dark. The samples are then washed by adding 2 ml PBS; pH =7.2 followed by centrifugation for 5 minutes at 200xg and the supernatant removed. The washing step is repeated. The washed cells are resuspended in 400-500 μl PBS; pH=7.2 and analyzed on flowcytometer.

The presence of cells labeled with anti-CD3/PB, anti-CD8/PE and either of the MHC(peptide)/APC dextran constructs 1, 2 or 3 described above and thereby the presence of Borrelia specific T cells will indicate that the patient are infected with Borrelia bacteria. Blood analysed with MHC(peptide)/APC dextran construct 4 should show no staining of CD3 and CD8 positive cells with this MHC(peptide)/APC dextran construct.

The sensitivity of the above described diagnostic test may be enhanced by addition of labeled antibodies specific for activation markers expressed in or on the surface of the Borrelia specific T cells.

## Example 73

This is an example of how MHC multimers may be used for diagnosis of Lyme Disease in blood samples from humans infected with Borrelia bacteria. The MHC multimer used are MHC complexes coupled to the multimerisation domain Streptavidin (SA).

Lyme disease is caused by infection by Borrelia bacteria. During acute infection Borrelia specific activated T cells will be present in increased amounts in an activated state compared to healthy individuals. The presences of an increased amount of activated Borrelia specific T cells may thereby act as a surrogate marker for infection with Borrelia bacterium. MHC multimers carrying borrelia specific antigenic peptides is in this example used to detect the presence of Borrelia specific T cells in the blood of patients infected with Borrelia.

Purified MHC-peptide complexes consisting of HLA-A*0201 heavy chain, human beta2microglobulin and peptide derived from regions in Outer surface protein B conserved among the three species Borrelia Burgdorferi, Borrelia Garinii and Borrelia Afzelii or a negative control peptide were generated by in vitro refolding, purified and biotinylated as described elsewhere herein. Biotinylated MHC-peptide complexes are then coupled SA labelled with APC. MHC-peptide complexes were added in an amount corresponding to a ratio of 5 MHC-peptide molecules per SA molecule. Then SA/APC carrying four MHC complexes were purified from free SA, free monomeric MHC complex, SA carrying three, two and one MHC complexes.

The following SA- MHC(peptide)/APC tetramers are made:

APC-SA coupled with HLA-A*0201 in complex with beta2microglobulin and the peptide FLTDGTITV derived from OspB.

APC-SA coupled with HLA-A*0201 in complex with beta2microglobulin and the peptide SITDDLNTI derived from OspB.

APC-SA coupled with HLA-A*0201 in complex with beta2microglobulin and the peptide KIKDFVFLT derived from OspB

APC-SA coupled with HLA-A*0201 in complex with beta2microglobulin and the non-sense peptide GLAGDVSAV (SEQ ID NO 217778).

The binding of the above described MHC(peptide)/APC dextran can be used to determine the presence of Osp B specific T cells in the blood from Borrelia infected individuals by flow cytometry following a standard flow cytometry protocol.

Blood from a patient with Lyme disease is isolated and 100 ul of this blood is incubated with either of the four SA- MHC(peptide)/APC tetramers described above for 10 minutes in the dark at room temperature. 5 μl of each of each of the antibodies mouse-anti-human CD3/PB (clone UCHT1 from Dako) and mouse-anti-human CD8/PE (clone DK25 from Dako) are added and the incubation continued for another 20 minutes at 4°C in the dark. The samples are then washed by adding 2 ml PBS; pH =7.2 followed by centrifugation for 5 minutes at 200xg and the supernatant removed. The washing step is repeated. The washed cells are resuspended in 400-500 μl PBS; pH=7.2 and analyzed on flowcytometer.

The presence of cells labeled with anti-CD3/PB, anti-CD8/PE and either of the SA-MHC(peptide)/APC tetramers 1, 2 or 3 described above and thereby the presence of Borrelia specific T cells will indicate that the patient are infected with Borrelia bacteria. Blood analysed with SA- MHC(peptide)/APC tetramers 4 should show no staining of CD3 and CD8 positive cells with this SA- MHC(peptide)/APC tetramer.

The sensitivity of the above described diagnostic test may be enhanced by addition of labeled antibodies specific for activation markers expressed in or on the surface of the Borrelia specific T cells.

**Example 74**

This is an example of how MHC multimers may be used for diagnosis of Lyme Disease in blood samples from humans infected with Borrelia bacteria. The MHC multimer used MHC complexes coupled to any multimerisation as described elsewhere herein. Lyme disease is caused by infection by Borrelia bacteria. During acute infection Borrelia specific activated T cells will be present in increased amounts in an activated state compared to healthy individuals. The presences of an increased amount of activated Borrelia specific T cells may thereby act as a surrogate marker for infection with Borrelia bacterium. MHC multimers carrying borrelia specific antigenic peptides is in this example used to detect the presence of Borrelia specific T cells in the blood of a patient infected with Borrelia.

Purified MHC-peptide complexes consisting of HLA-A*0201 heavy chain, human beta2microglobulin and peptide derived from regions in Outer surface protein B conserved among the three species Borrelia Burgdorferi, Borrelia Garinii and Borrelia Afzelii or a negative control peptide were generated by in vitro refolding and purified or purified from antigen presenting cells. MHC-peptide complexes are then coupled to a multimerisation domain together with APC. The following MHC(peptide)/APC multimers are made:

APC-multimerisation domian coupled with HLA-A*0201 in complex with beta2microglobulin and the peptide FLTDGTITV derived from OspB.

APC-multimerisation domian coupled coupled with HLA-A*0201 in complex with beta2microglobulin and the peptide SITDDLNTI derived from OspB.

APC-multimerisation domian coupled coupled with HLA-A*0201 in complex with beta2microglobulin and the peptide KIKDFVFLT derived from OspB

APC-multimerisation domian coupled coupled with HLA-A*0201 in complex with beta2microglobulin and the non-sense peptide GLAGDVSAV (SEQ ID NO 217778).

The binding of the above described MHC(peptide)/APC multimers can be used to determine the presence of Osp B specific T cells in the blood from Borrelia infected individuals by flow cytometry following a standard flow cytometry protocol.

Blood from a patient with Lyme disease is isolated and 100 ul of this blood is incubated with either of the four MHC(peptide)/APC multimers described above for 10 minutes in the dark at room temperature. 5 $\mu$l of each of each of the antibodies mouse-anti-human CD3/PB (clone UCHT1 from Dako) and mouse-anti-human CD8/PE (clone DK25 from Dako) are added and the incubation continued for another 20 minutes at 4°C in the dark. The samples are then washed by adding 2 ml PBS; pH =7.2 followed by centrifugation for 5 minutes at 200xg and the supernatant removed. The washing step is repeated. The washed cells are resuspended in 400-500 $\mu$l PBS; pH=7.2 and analyzed on flowcytometer.

The presence of cells labeled with anti-CD3/PB, anti-CD8/PE and either of the MHC(peptide)/APC multimers 1, 2 or 3 described above and thereby the presence of Borrelia specific T cells will indicate that the patient are infected with Borrelia bacteria. Blood analysed with MHC(peptide)/APC multimer 4 should show no staining of CD3 and CD8 positive cells with this SA- MHC(peptide)/APC tetramer.

The sensitivity of the above described diagnostic test may be enhanced by addition of labeled antibodies specific for activation markers expressed in or on the surface of the Borrelia specific T cells.

**Example 75**

This is an example of how MHC multimers may be used for diagnosis of Lyme Disease in blood samples from humans infected with Borrelia bacteria. This is an example of indirect detection of individual specific T cells by measurement of produced soluble factor.

The MHC multimer used are antigen presenting cells expressing MHC molecules complexed with peptides derived from Borrelia antigen.

Lyme disease is caused by infection by Borrelia bacteria. During acute infection Borrelia specific activated T cells will be present in increased amounts in an activated state compared to healthy individuals. The presences of an increased amount of activated Borrelia specific T cells may thereby act as a surrogate marker for infection with Borrelia bacterium. MHC multimers carrying borrelia specific antigenic peptides is in this example used to detect the presence of Borrelia specific T cells in the blood of a patient infected with Borrelia.

Activated effector T cells can be detected directly, via their secretion of cytokines, when presented with specific antigens. In this example the activation of individual effector T cells is detected by measurement of cytokines secreted from each individual T cell by capturing of the secreted cytokines in the proximity of each cell. The effector T cells secrete specific cytokine in response to stimulation by peptides from the Borrelia antigen OspB when these peptides are presented by antigen presenting cells, briefly:

A patient's blood sample is collected ficoll purified using aVacutainer® Cell Preparation Tubes™, which are spun to separate white blood cells, known as peripheral blood mononuclear cells (PBMCs)

PBMCs are washed in culture media to remove any background interference, counted to correct for a patient's immune status, and added in even amounts to the wells of a 96-well microtiter plate. These plate wells are pre-coated with a monoclonal antibody specific for the cytokine INFγ released by effector T cells in response to contact with specific antigens presented on antigen presenting cells (e.g. B cells that are a population in the PBMC pool)

The peptides FLTDGTITV, SITDDLNTI and KIKDFVFLT all derived from OspB from Borrelia bacteria are added to the appropriate wells to stimulate cytokine release (positive and nil controls are used as internal assay controls). A negative control is a non-sense peptide unable to stimulate antigen-specific T cells.

The assay plate is placed in a $CO_2$ incubator overnight to allow the effector T cells to encounter the antigen.

The plates are washed, removing both the T cells and the antigen from the wells and leaving any T cell secreted cytokine captured by the antibodies lining the wells.

An enzyme-conjugated secondary antibody that binds to another epitope on the captured cytokine is then added and incubated for 1 hour at room temperature.

The plates are washed to remove unbound secondary antibody.

Coloured spots are generated by the conjugated enzyme upon application of a colorimetric substrate. These spots reveal a footprint of the antigen-specific effector T cells in the sample.

The spots are counted to determine the number of T cells in the sample that reacted to the antigen relative to the negative control, thereby identifying infection.

**Example 76**

This is an example of how MHC multimers may be used for diagnosis of Lyme Disease in blood samples from humans infected with Borrelia bacteria. This is an example of indirect detection of a population of specific T cells by measurement of produced soluble factor.

The MHC multimer used are antigen presenting cells expressing MHC molecules complexed with peptides derived from Borrelia antigen.

Lyme disease is caused by infection by Borrelia bacteria. During acute infection Borrelia specific activated T cells will be present in increased amounts in an activated state compared to healthy individuals. The presences of an increased amount of activated Borrelia specific T cells may thereby act as a surrogate marker for infection with Borrelia bacterium. MHC multimers carrying borrelia specific antigenic peptides is in this example used to detect the presence of Borrelia specific T cells in the blood of a patient infected with Borrelia.

Activated effector T cells can be detected directly, via their secretion of cytokines, when presented with specific antigens. In this example the activation of a pool of effector T cells is detected by measurement of cytokines secreted from the activated T cells in the analysed sample. In this example the effector T cells secrete the cytokine INFγ in response to stimulation by peptides derived from the Borrelia antigen OspB when these peptides are presented by antigen presenting cells.

Briefly, the procedure is as follows: Blood is collected in a heparin-coontaining tube, then the sample is aliquotated into the wells of a microtiterplate. Appropriate wells are incubated with the peptides FLTDGTITV, SITDDLNTI and KIKDFVFLT all derived from OspB from Borrelia bacteria within 12 hours of blood collection. Mix on a shaker, incubate for a day-ish. The supernatant plasma, is transferred to wells of another microtiterplate in various dilutions. The wells of the microtiterplate are previously coated with antibody specific for INFγ. An IFN-gamma standard dilution series is included as positive control. Mix, incubate, wash six times. An enzyme-conjugated secondary antibody that binds to another epitope on the captured INF is then added and incubated for 1 hour at room temperature Add enzyme substrate solution, incubate, add enzyme stopping solution. The OD of each well is determined. The OD value may be correlated to the amount of INF-gamma in the sample which is a surrogate marker for the presence of activated Borrelia specific T cells in the sample. The presence of activated Borrelia specific T cells in the sample is a surrogate marker for infection

**Example 77**

This is an example of how MHC multimers may be used for diagnosis of Lyme Disease in blood samples from humans infected with Borrelia bacteria. The MHC multimer used are MHC complexes coupled to labeled dextran, where theMHC molecules are complexed with peptides derived from Borrelia antigen.

Lyme disease is caused by infection by Borrelia bacteria. During acute infection Borrelia specific activated T cells will be present in increased amounts in an activated state compared to healthy individuals. The presences of an increased amount of activated Borrelia specific T cells may thereby act as a surrogate marker for infection with Borrelia bacterium. MHC multimers carrying borrelia specific antigenic peptides is in this example used to detect the presence of Borrelia specific T cells in the blood of a patient infected with Borrelia.

Activated effector T cells can be detected directly, via their production of cytokines, when presented with specific antigens. In this example the activation of individual effector T cells is detected by measurement of the intracellular production of cytokines in individual T cell in response to stimulation by peptides from the Borrelia antigen OspB when these peptides are presented by antigen presenting cells.

Briefly, a patient's blood sample is collected ficoll purified using aVacutainer® Cell Preparation Tubes™, which are spun to separate white blood cells, known as peripheral blood mononuclear cells (PBMCs)

PBMCs are washed in culture media to remove any background interference, counted to correct for a patient's immune status, and added in even amounts to the wells of cell culture plate.

The peptides FLTDGTITV, SITDDLNTI and KIKDFVFLT all derived from OspB from Borrelia bacteria are added to the appropriate wells to stimulate cytokine release (positive and nil controls are used as internal assay controls). A negative control is a non-sense peptide unable to stimulate antigen-specific T cells.

The assay plate is placed in a $CO_2$ incubator overnight to allow the effector T cells to encounter the antigen.

T cells from each well of the plate is transferred to 12 x 75 mm polystyrene test tubes and then analysed by flow cytometry using a procedure allowing for intracellular staining:

5 $\mu$l of each of each of the antibodies mouse-anti-human CD3/PB (clone UCHT1 from Dako) and mouse-anti-human CD8/PE (clone DK25 from Dako) are added to each sample and incubatied for 20 minutes at room temperature in the dark. The samples are then washed by adding 2 ml PBS; pH =7.2 followed by centrifugation for 5 minutes at 200xg and the supernatant removed.

5 $\mu$l of each of each of the antibodies mouse-anti-human CD3/PB (clone UCHT1 from Dako) and mouse-anti-human CD8/PE (clone DK25 from Dako) are added to each sample and incubatied for 20 minutes at room temperature in the dark. The samples are then washed by adding 2 ml PBS; pH =7.2 followed by centrifugation for 5 minutes at 200xg and the supernatant removed.

Fixative is added. Mix gently with a vortex mixer to ensure that the cells are in suspension.

Incubate in the dark at room temperature for 15 minutes.

The samples are then washed by adding 2 ml PBS; pH =7.2 followed by centrifugation for 5 minutes at 200xg and then aspirate the supernatant, leaving approximately 50 $\mu$L of fluid. Mix thoroughly to ensure that the cells are in suspension.

Permeabilization reagent is added, to each test tube. Add an appropriate volume of fluorochrome-conjugated antibody specific for the intracellular antigen to be stained. Mix gently to ensure that the cells are in suspension.

Incubate in the dark at room temperature for 15 minutes.

The samples are then washed by adding 2 ml PBS; pH =7.2 followed by centrifugation for 5 minutes at 200xg and the supernatant removed.

Resuspend the pellet in an appropriate fluid for flow cytometric analysis.

Analyze on a flow cytometer

The presence of cells labeled with anti-CD3/PB, anti-CD8/PE and the antibody used for intracellular staining are cells that have been activated by Borrelia antigens and are thereby Borrelia specific T cells. The presence of activated Borrelia specific T cells in the sample is a surrogate marker for infection.

The sensitivity of the above described diagnostic test may be enhanced by addition of flourochrome labeled MHC multimers able to measure antigen-specific T cells and/or by addition of labeled antibodies specific for activation markers expressed in or on the surface of the Borrelia specific T cells.

## Example 78

This is an example of a CTL killing assay.

T cell clones can be tested for specificity for a given borrelia derived antigenic peptide able to bind a given HLA molecule using a CTL killing assay. In this example expanded T cell clones are tested for specificity for the borrelia derived Outer surface protein C (OspC) by analysis in a standard 51-Cr release assay. For this purpose, T2 cells expressing HLA-A*0201 molecules on their surface are loaded with either TLVSSIDEL peptide derived from OspC and able to bind HLA-A*0201 (se figure 32) or an irrelevant peptide GLAGDVSAV and used as target cells. CD8 T-cell clones that effectively lyse T2 cells pulsed with TLVSSIDEL without killing of T2 cells pulsed with the irrelevant peptide GLAGDVSAV are T cell clones specific for the OspC derived epitope TLVSSIDEL. Clones that do not show specific lysis against T2 cells pulsed with TLVSSIDEL peptide are not specific for OspC.

## Example 79

This is an example of a CTL killing assay.

T cell clones can be tested for specificity for a given borrelia derived antigenic peptide able to bind a given HLA molecule using a CTL killing assay. In this example expanded T cell clones are tested for specificity for any borrelia derived protein by analysis in a standard 51-Cr release assay. For this purpose, T2 cells expressing HLA-A*0201 or any other cell type expressing specific HLA molecules are loaded with either antigenic peptides derived from borrelia proteins and able to bind HLA molecules in question or an irrelevant peptide and used as target cells. CD8 T-cell clones that effectively lyse T2 cells pulsed with antigenic peptides derived from borrelia proteins without killing of T2 cells pulsed with the irrelevant peptide are T cell clones specific for the tested borrelia protein. Clones that do not show specific lysis against T2 cells pulsed with borrelia derived antigenic peptide are not specific for OspC.

## Example 80

This is an example of indirect detection of a population of T cells, where cells in suspension are induced to produce soluble factor. The soluble factor produced is a cytokine (IFN-γ) and is detected by a chromogen assay using anti-cytokine antibodies. The antigenic peptides origin is Borrelia bacteria.

Blood from patients suspected to have Borrelia infection are withdrawn and the presence of IFN-γ releasing T cells are detected as described in the following.

The procedure used in this example is a whole blood IFN-γ assay (QuantiFERON [QFT]; Cellestis, Carnegie, Australia) and involves two stages: (*1*) overnight incubation of whole blood with antigens and (*2*) measurement of IFN-γ production in harvested plasma samples by ELISA.

Briefly, the procedure is as follows:

Within 12 hours of collection, 1-ml aliquots of blood samples are dispensed into 24-well tissue culture plates and antigens are added to appropriate wells. Three drops of saline (nil control) or phytohemagglutinin (5 μg/ml; mitogen-positive control), and 100 μl of a peptide cocktail, are added to separate wells to give a final peptide concentration of 1 μg/ml. The peptide cocktail contain 10 antigenic peptides selected from the Borrelia antigen OspB. The 10 peptides are able to bind different HLA clas 2 molecules and have the following sequences:

KKYLLGFALVLALIA; DTVKLFNDTKIFISK; SKVFKKQGSLTEETE; TAVWSDTSNTLTVSA; ETYDSSNTKVASKVF; MKKYLLGFALVLALI; TKAVETLKNGIMLEG; KAVETLKNGIMLEGN; TLEYSDMTNDENATK and ITVQNYDTAGTKLAG (see figure 34).

Blood samples were incubated with antigens for 16 to 24 hours at 37°C before harvesting about 300 μl of plasma from above the settled blood cells.

The concentration of IFN-γ produced in the four plasma samples from each subject, as a result of stimulation of specific T cells with antigen presenting cells displaying the γ above listed peptides, is determined by QuantiFERON-CMI ELISA or another IFN-measuring ELISA assay following the manufacturer's instructions.

Samples from up to 16 subjects are tested in each ELISA run, which also included a set of standards that are measured in duplicate. For an ELISA run to be valid, strict performance criteria (coefficient of variation less than 15% and correlation coefficient for the standard curve greater than 0.98) had to be met. ELISA data for the *Borrelia*-specific antigen OspB and the nil and mitogen controls are converted to international units per milliliter on the basis of the IFN-γ standard curve generated for each ELISA plate. For an individual's test to be deemed valid, their response to at least one antigen (OspB or mitogen) has to be at least 0.25 IU of IFN-γ per milliliter above that of their nil control (five times the limit of detection for the ELISA). Results for OspB are expressed as the concentration of IFN-γ detected minus the concentration of IFN-γ in the respective nil control plasma.

The presence of IFN-γ in blood of the tested individual indicates the presence of activated T cells specific for one or more of the investigated peptide epitopes from the Borrelia antigen OspB tested and can be regarded as a surrogate marker for infection with Borrelia bacteria.

## Example 81

This is an example of indirect detection of a population of T cells, where cells in suspension are induced to produce soluble factor. The soluble factor produced is a cytokine (IFN-γ) and is detected by a chromogen assay using anti-cytokine antibodies. The antigenic peptides origin is Borrelia bacteria.

Blood from patients suspected to have Borrelia infection are withdrawn and the presence of IFN-γ releasing T cells are detected as described in the following.

The procedure used in this example is a whole blood IFN-γ assay (QuantiFERON [QFT]; Cellestis, Carnegie, Australia) and involves two stages: (*1*) overnight incubation of whole blood with antigens and (*2*) measurement of IFN-γ production in harvested plasma samples by ELISA.

Briefly, the procedure is as follows:

Within 12 hours of collection, 1-ml aliquots of blood samples are dispensed into 24-well tissue culture plates and antigens are added to appropriate wells. Three drops of saline (nil control) or phytohemagglutinin (5 μg/ml; mitogen-positive

control), and 100 μl of a peptide cocktail, are added to separate wells to give a final peptide concentration of 1 μg/ml. The peptide cocktail contain 10-20 antigenic peptides selected from one or more Borrelia antigen(s). The 10-20 peptides are able to bind different HLA class 1 and/or 2 molecules and have sequences selected from the lists of borrelia derived antigenic peptide sequences enclosed in this disclosure.

Blood samples were incubated with antigens for 16 to 24 hours at 37 °C before harvesting about 300 μl of plasma from above the settled blood cells.

The concentration of IFN-γ produced in the four plasma samples from each subject, as a result of stimulation of specific T cells with antigen presenting cells displaying the above listed peptides, is determined by QuantiFERON-CMI ELISA or another IFN-γ measuring ELISA assay following the manufacturer's instructions.

Samples from up to 16 subjects are tested in each ELISA run, which also included a set of standards that are measured in duplicate. For an ELISA run to be valid, strict performance criteria (coefficient of variation less than 15% and correlation coefficient for the standard curve greater than 0.98) had to be met. ELISA data for the *Borrelia*-specific antigen(s) and the nil and mitogen controls are converted to international units per milliliter on the basis of the IFN-γ standard curve generated for each ELISA plate. For an individual's test to be deemed valid, their response to at least one antigen (Borrelia antigen or mitogen) has to be at least 0.25 IU of IFN-γ per milliliter above that of their nil control (five times the limit of detection for the ELISA). Results for Borrelia antigen(s) are expressed as the concentration of IFN-γ detected minus the concentration of IFN-γ in the respective nil control plasma.

The presence of IFN-γ in blood of the tested individual indicates the presence of activated T cells specific for one or more of the investigated antigenic peptide epitopes from the Borrelia antigen(s) tested and can be regarded as a surrogate marker for infection with Borrelia bacteria.

**Example 82**

This is an example of indirect detection of a population of T cells, where cells in suspension are induced to produce soluble factor. The soluble factor produced is a cytokine (IFN-γ) and is detected by a chromogen assay using anti-cytokine antibodies. The antigenic peptides origin is Borrelia bacteria.

Blood from patients suspected to have Borrelia infection are withdrawn and the presence of IFN-γ releasing T cells are detected as described in the following.

The procedure used in this example is a whole blood IFN-γ assay (QuantiFERON [QFT]; Cellestis, Carnegie, Australia) and involves two stages: (*1*) overnight incubation of whole blood with antigens and (*2*) measurement of IFN-γ production in harvested plasma samples by ELISA.

Briefly, the procedure is as follows:

Within 12 hours of collection, 1-ml aliquots of blood samples are dispensed into 24-well tissue culture plates and antigens are added to appropriate wells. Three drops of saline (nil control) or phytohemagglutinin (5 μg/ml; mitogen-positive control), and 100 μl of a peptide cocktail, are added to separate wells to give a final peptide concentration of 1 μg/ml. The peptide cocktail contain 10-20 antigenic polypeptides selected from one or more Borrelia antigen(s). The 10-20 peptides comprises antigenic peptides able to bind different HLA class 1 and/or 2 molecules and these antigenic peptides have sequences selected from the lists of borrelia derived antigenic peptide sequences enclosed in this disclosure.

Blood samples were incubated with antigens for 16 to 24 hours at 37 °C before harvesting about 300 μl of plasma from above the settled blood cells.

The concentration of IFN-γ produced in the four plasma samples from each subject, as a result of stimulation of specific T cells with antigen presenting cells displaying the above listed peptides, is determined by QuantiFERON-CMI ELISA or another IFN-γ measuring ELISA assay following the manufacturer's instructions.

Samples from up to 16 subjects are tested in each ELISA run, which also included a set of standards that are measured in duplicate. For an ELISA run to be valid, strict performance criteria (coefficient of variation less than 15% and correlation coefficient for the standard curve greater than 0.98) had to be met. ELISA data for the *Borrelia*-specific antigen(s) and the nil and mitogen controls are converted to international units per milliliter on the basis of the IFN-γ standard curve generated for each ELISA plate. For an individual's test to be deemed valid, their response to at least one antigen (Borrelia antigen or mitogen) has to be at least 0.25 IU of IFN-γ per milliliter above that of their nil control (five times the limit of detection for the ELISA). Results for Borrelia antigen(s) are expressed as the concentration of IFN-γ detected minus the concentration of IFN-γ in the respective nil control plasma.

The presence of IFN-γ in blood of the tested individual indicates the presence of activated T cells specific for one or more of the investigated antigenic polypeptide epitopes from the Borrelia antigen(s) tested and can be regarded as a surrogate marker for infection with Borrelia bacteria.

**Example 83**

This is an example of how borrelia derived antigenic polypeptides can be used for vaccination of Gerbils.

To ascertain whether cross-protection between different OspC families was possible, OspC proteins were purified from B. burgdorferi strain ZS7 (OspC family 1), B. afzelii strain PKO (OspC family 7) and B. garinii strain W (OspC family 10) and used as immunogens in the gerbil model of Lyme borreliosis against a challenge with B. afzelii strain Orth (OspC family 5). To test protection within a family, the OspC from strain H7 (OspC family 5) was used as an immunogen against strain Orth. The OspC from strain H7 belongs to the same serovar and RFLP-type as the OspC from strain Orth.

Gerbils were given either a single, subcutaneous immunization of purified OspC (20 .mu.g protein/200 .mu.l, adjuvanted with TiterMax #R-1 (CytRx), that is, were prepared as a water-in-oil (squalene) emulsion with a synthetic immunomodulator (copolymer CRL89-41) or two intraperitoneal injections of OspC (10 .mu.g protein/500 .mu.l) adjuvanted with aluminium hydroxide. The purified antigens were prepared from strains by methods described in U.S. patent6,221,363. Three and a half weeks after the first immunization, blood samples were taken from the eye and plasma prepared so that the antibody response to the immunogen at the time of the challenge could be ascertained (unimmunized control animals were likewise treated). Four weeks after the first immunization, the animals were challenged intraperitoneally with 10.sup.4 cells (25-100 ID.sub.50) of strain Orth, as were a group of unimmunized control animals. The animals. The challenge suspension was also titrated in unimmunized gerbils to determine the dose required to infect 50% of the animals. After a further two weeks, the animals challenged with the 10.sup.4 dose were killed and the bladder, heart, kidneys and spleens cultured in BSK medium. Cultures were inspected for spirochetes at weekly intervals, from the second to the sixth week post-inoculation, by dark-field microscopy. Blood also was taken and the resultant plasma analyzed by western blotting for sero-conversion, i.e. the development of antibodies post-challenge to antigens from strain Orth other than the immunogen. There was good agreement between the cultural and serological tests used to ascertain which animals were infected. Only serological testing was used for the ID(50) determinations but in this instance the animals were bled three weeks post-challenge, the extra time being given to ensure that the antibody response in infected gerbils was sufficiently strong to be easily detected.

There were no signs of cross-protection between species i.e. OspC proteins from OspC families 1 (B. burgdorferi) and (B. garinii) were ineffective as immunogens against the challenge strain Orth (B. afzelii). Likewise there was no sign of cross-protection between different OspC families of the same species i.e. the OspC protein from an OspC family 7 isolate (B. afzelii strain PKO) was ineffective as an immunogen against a challenge with strain Orth which expresses an OspC protein from OspC family 5. By contrast, immunization with the OspC protein from strain H7 (OspC family 5) was effective against a challenge strain Orth (OspC family 5). These data indicate that cross-protection between the OspC families is unlikely and that protection within a family is possible. A multivalent vaccine comprising one or more types of OspC proteins from each of the OspC families should be sufficient to protect against most Lyme disease Borrelia strains.

*This example is modified from patent* WO94/25596.

## Example 84

This is an example of how a borrelia derived antigenic polypeptide can be used for vaccination of Gerbils.

In this example the antigenic polypeptide is the first 86 amino acids of the Borrelia Outer surface protein G (OspG). The amino acids sequences used is

MNKKMKNLIICAVFVLIISCKIDASSEDLKQNVKEKVEGFLDKELMQGDDPNNSLFNPPPVLPASSHDNTPVLKAVQAK-DGGQQEG and is derived from Borrelia burgdorferi strain ZS7. Genes encoding the antigenic polypeptide with this sequence are synthesized e.g. using Gene Assembler™ gene synthesis platform (made by GeneArt) and cloned into a suitable expression vector. Then this plasmid is transformed into E.coli, expressed and purified using standard procedures known by persons skilled in the art.

This OspG(1-86) antigenic polypeptide are used as immunogens in the gerbil model of Lyme borreliosis against a challenge with Borrelia burgdorferi.

Gerbils were given either a single, subcutaneous immunization of purified OspG(1-86) (20 .mu.g protein/200 .mu.l, adjuvanted with TiterMax #R-1 (CytRx), that is, were prepared as a water-in-oil (squalene) emulsion with a synthetic immunomodulator (copolymer CRL89-41) or two intraperitoneal injections of OspG(1-86) (10 .mu.g protein/500 .mu.l) adjuvanted with aluminium hydroxide.

Three and a half weeks after the first immunization, blood samples are taken from the eye and plasma prepared so that the antibody response to the immunogen at the time of the challenge can be ascertained (unimmunized control animals were likewise treated). Four weeks after the first immunization, the animals are challenged intraperitoneally with 10.sup.4 cells (25-100 ID.sub.50) of a Borreli burgdorferi strain (e.g. ZS7 or B31). The challenge suspension is also titrated in unimmunized gerbils to determine the dose required to infect 50% of the animals. After a further two weeks, the animals challenged with the 10.sup.4 dose are killed and the bladder, heart, kidneys and spleens cultured in BSK medium. Cultures are inspected for spirochetes at weekly intervals, from the second to the sixth week post-inoculation, by dark-field microscopy. Only serological testing was used for the ID(50) determinations but in this instance the animals are bled three weeks post-challenge, the extra time being given to ensure that the antibody response in infected gerbils is sufficiently strong to be easily detected.

It is expected that vaccination with the OspG(1-86) protein is effective against a challenge strain ZS7 and may also be effective against challenge with other strains since these strains have homologous sequences of the first 86 amino acids of the OspG proteins.

## Example 85

This is an example of how several borrelia derived antigenic polypeptide can be used for vaccination of Gerbils.

In this example 10 different antigenic polypeptides with sequences derived from 1-10 different borrelia proteins are used to generate a vaccine against Lyme disease. Each sequence is selected to be homologous among many Borrelia species and strains. Genes encoding the antigenic polypeptides with these sequences are synthesized e.g. using Gene Assembler™ gene synthesis platform (made by GeneArt) and cloned into a suitable expression vectors. Then these plasmids are transformed into *E.coli*, expressed and purified using standard procedures known by persons skilled in the art. The antigenic polypeptides are used as immunogens in the gerbil model of Lyme borreliosis against a challenge with Borrelia burgdorferi, Borrelia Garinii and/or Borrelia afzelii.

Gerbils were given either a single, subcutaneous immunization of purified antigenic polypeptides (each 20 .mu.g protein/200 .mu.l, adjuvanted with TiterMax #R-1 (CytRx), that is, were prepared as a water-in-oil (squalene) emulsion with a synthetic immunomodulator (copolymer CRL89-41) or two intraperitoneal injections of antigenic polypeptide (each 10 .mu.g protein/500 .mu.l) adjuvanted with aluminium hydroxide. Three and a half weeks after the first immunization, blood samples are taken from the eye and plasma prepared so that the antibody response to the immunogen at the time of the challenge can be ascertained (unimmunized control animals were likewise treated). Four weeks after the first immunization, the animals are challenged intraperitoneally with 10.sup.4 cells (25-100 ID.sub.50) of a Borreli burgdorferi, Borrelia Garinii and/or Borrelia afzelii strain(s). The challenge suspension is also titrated in unimmunized gerbils to determine the dose required to infect 50% of the animals. After a further two weeks, the animals challenged with the 10.sup.4 dose are killed and the bladder, heart, kidneys and spleens cultured in BSK medium. Cultures are inspected for spirochetes at weekly intervals, from the second to the sixth week post-inoculation, by dark-field microscopy.

Only serological testing was used for the ID(50) determinations but in this instance the animals are bled three weeks post-challenge, the extra time being given to ensure that the antibody response in infected gerbils is sufficiently strong to be easily detected.

It is expected that vaccination with these antigenic polypeptides are effective against a challenge a wide range of Borrelia species and strain since these strains have homologous sequences of the amino acid sequence of the selected antigenic polypeptides.

A multivalent vaccine comprising one or more types of Borrelia derived proteins s should be sufficient to protect against most Lyme disease Borrelia strains. In general antigenic polypeptides comprising one/or more antigenic peptides sequence(s) as described herein can be used.

## Example 86

This is an example of how several borrelia derived antigenic polypeptide can be used for vaccination of humans against Lyme disease.

In this example 10-20 different antigenic polypeptides with sequences derived from 1-20 different borrelia proteins are used to generate a vaccine against Lyme disease. Each sequence is selected to be homologous among many Borrelia species and strains.

Genes encoding the antigenic polypeptides with these sequences are synthesized , expressed and produced.

The antigenic polypeptides are used as vaccine components in humans To protect against Borrelia burgdorferi, Borrelia Garinii and/or Borrelia afzelii strains.

The antigenic polypeptides are given in dosis suitable for humans and together with an adjuvant usefull in humans. The vaccine is given either as a single dose or as multiple dosis.

It is expected that vaccination with these antigenic polypeptides are effective against a challenge a wide range of Borrelia species and strain since these strains have homologous sequences of the amino acid sequence of the selected antigenic polypeptides.

A multivalent vaccine comprising one or more types of Borrelia derived proteins s should be sufficient to protect humans against most Lyme disease Borrelia strains. In general antigenic polypeptides comprising one/or more Borrelia derived antigenic peptide sequences as described in the present disclosure can be used.

### Example 87

This is an example of how several borrelia derived antigenic peptides can be used for vaccination of humans against Lyme disease.
In this example 10-20 different antigenic peptides with sequences derived from 1-20 different borrelia proteins are used to generate a vaccine against Lyme disease. Each sequence is selected to be homologous among many Borrelia species and strains.

Genes encoding the antigenic peptides with these sequences are synthesized , expressed and produced. The antigenic peptides are used as vaccine components in humans To protect against Borrelia burgdorferi, Borrelia Garinii and/or Borrelia afzelii strains.

The antigenic peptides are given in dosis suitable for humans and together with an adjuvant usefull in humans. The vaccine is given either as a single dose or as multiple dosis.
It is expected that vaccination with these antigenic peptides are effective against a challenge a wide range of Borrelia species and strain since these strains have homologous sequences of the amino acid sequence of the selected antigenic peptides. A multivalent vaccine comprising one or more types of Borrelia derived proteins s should be sufficient to protect humans against most Lyme disease Borrelia strains. In general any antigenic peptides comprising Borrelia derived amino acid sequences as described in the present dislcosure can be used.

### Example 88

This is an example of how MHC multimers can be used as a borrelia vaccine for monkeys.
The vaccine is composed of a Dextran (270 kDa) carrier that has been derivatized by attaching Strepavidin (on average 8.6 Strepavidin molecules/Dextran molecule). Attached to the carrier are human HLA class 1 and 2 molecules containing antigenic peptides derived from the Borrelia antigen Basic membrane protein A (BmpA). The attached HLA-peptide complexes are:
HLA-A*0201(YLAPDNVIT), HLA-A*0101(YSDEIDIIH), HLA-B*0702(APDNVITST), HLA-DRB1*0401(IELVLKESSSNSYLS), HLA-BRB1*1501(MNKILLLILLESIVF) and HLA-DRB1*1101(SDLIWLIGYRFSDVA).

A single full dose of the final vaccine corresponds to, 100 $\mu$g of each MHC class 1; 113,5 g of HLA class 2; 3x25.8 $\mu$g HSP70 and 3x162.2 $\mu$g dextran (corresponding to a total of approx 300 $\mu$g MHC multimer/kg body weight for a monkey of 3.5 kg) and consist of a 1:1:1 mixture of the following three MHC multimers:

MHC multimer 1:
HLA-A*0101(YSDEIDIIH) + HLA-DR*0401(IELVLKESSSNSYLS) + HSP70 + Dextran carrier, in the molar ratio10/10/4/1

MHC multimer 2:
HLA-A*0201(YLAPDNVIT) + HLA-DR*1501(MNKILLLILLESIVF) + HSP70 + Dextran carrier, in the molar ratio10/10/4/1

MHC multimer 2:
HLA-B*0702(APDNVITST) + HLA-DRB1*1101(SDLIWLIGYRFSDVA) + HSP70 + Dextran carrier, in the molar ratio10/10/4/1

Administration:

The MHC multimer mix is emulsified with the adjuvant Titermax Gold as specified by the

manufacturer and administered as intramuscular injections at time points 0, 4, 8, and 16 weeks such that a full dose is given at 0 weeks and half-full dose at 4, 8, and 16 5 weeks. This vaccine may give protection against infection with Borrelia bacteria.

### Example 89

This is an example of how MHC multimers can be used as a borrelia vaccine for monkeys.

The vaccine is composed of an antigen presenting cell (APC) e.g. a Dendritic cell expressing the HLA class 1 molecules HLA-A*0101 and HLA-B*0702. The APC is loaded with antigenic peptides derived from the Borrelia protein Basic membrane protein A (BmpA) by incubation with the peptides for 2-5 hours at 37°C. The amino acid sequence of the peptides are YSDEIDIIH and APDNVITST (se figure 32).

Administration:

The peptide loaded APC are added the adjuvant Titermax Gold as specified by the manufacturer and administered as intramuscular injections at time points 0, 4, 8, and 16 weeks. This vaccine may give protection against infection with Borrelia bacteria.

## Claims

1. An MHC class I multimer comprising $(a-b-P)_n$, wherein n > 1,
   wherein a and b together form a functional MHC class I protein capable of binding the antigenic peptide P,
   wherein (a-b-P) is the MHC class I peptide complex formed when the antigenic peptide P binds to the functional MHC class I protein,
   wherein each MHC class I peptide complex of a MHC class I multimer is associated with one or more multimerization domains,
   wherein P comprises SEQ ID NO:5751.

2. An MHC class II multimer comprising $(a-b-P)_n$, wherein n > 1,
   wherein a and b together form a functional MHC class II protein capable of binding the antigenic peptide P,
   wherein (a-b-P) is the MHC class II peptide complex formed when the antigenic peptide P binds to the functional MHC class II protein,
   wherein each MHC class II peptide complex of a MHC class II multimer is associated with one or more multimerization domains,
   wherein P comprises SEQ ID NO:5751.

3. The MHC multimer according to any of the preceding claims, wherein said one or more multimerization domains comprise a polysaccharide carrier.

4. A method for immune monitoring or diagnosing one or more diseases comprising the following steps

   i) providing the MHC multimer according to any one of claims 1-3, or the individual components thereof,
   ii) providing a population of T cells, and
   iii) measuring the number, activity or state of T cells specific for said MHC multimer, thereby immune monitoring or diagnosing said one or more diseases.

5. A method for isolation of one or more antigen-specific T cells, said method comprising the steps of

   i) providing the MHC multimer according to any one of claims 1-3, or individual components thereof,
   ii) providing a population of T cells, and
   iii) isolating T cells specific for said MHC multimer.

6. A composition comprising a plurality of MHC Multimers according to any of claims 1-3, and a carrier.

7. A method for detection of antigen-specific T cells, said method comprising the steps of 1) providing a MHC multimer according to claims 1-3, 2) providing a population of antigen-specific T cells, and 3) detecting antigen-specific T cells specific for the peptide P of the MHC multimer.

## Patentansprüche

1. Multimer der MHC-Klasse I, umfassend $(a-b-P)_n$, wobei n > 1 ist,
   wobei a und b zusammen ein funktionelles Protein der MHC-Klasse I bilden, das in der Lage ist, das Antigenpeptid P zu binden,
   wobei (a-b-P) der Peptidkomplex der MHC-Klasse I ist, der gebildet wird, wenn das Antigenpeptid P an das funktionelle Protein der MHC-Klasse I bindet,
   wobei jeder Peptidkomplex der MHC-Klasse I eines Multimers der MHC-Klasse I mit einer oder mehreren Multimerisierungsdomänen assoziiert ist,
   wobei P die SEQ ID NO:5751 umfasst.

**2.** Multimer der MHC-Klasse II, umfassend $(a\text{-}b\text{-}P)_n$, wobei n > 1 ist,
wobei a und b zusammen ein funktionelles Protein der MHC-Klasse II bilden, das in der Lage ist, das Antigenpeptid P zu binden,
wobei (a-b-P) der Peptidkomplex der MHC-Klasse II ist, der gebildet wird, wenn das Antigenpeptid P an das funktionelle Protein der MHC-Klasse II bindet,
wobei jeder Peptidkomplex der MHC-Klasse II eines Multimers der MHC-Klasse II mit einer oder mehreren Multimerisierungsdomänen assoziiert ist,
wobei P die SEQ ID NO:5751 umfasst.

**3.** MHC-Multimer nach einem der vorstehenden Ansprüche, wobei die eine oder die mehreren Multimerisierungsdomänen einen Polysaccharidträger umfassen.

**4.** Verfahren zur Immunüberwachung oder zum Diagnostizieren einer oder mehrerer Erkrankungen, umfassend die folgenden Schritte:

i) Bereitstellen des MHC-Multimers nach einem der Ansprüche 1-3 oder der einzelnen Komponenten davon,
ii) Bereitstellen eines Bestands von T-Zellen, und
iii) Messen der Anzahl, Aktivität oder des Zustands der T-Zellen, die für das MHC-Multimer spezifisch sind, und somit die Immunüberwachung oder das Diagnostizieren der einen oder der mehreren Erkrankungen.

**5.** Verfahren zum Isolieren einer oder mehrerer antigenspezifischer T-Zellen, wobei das Verfahren die folgenden Schritte umfasst:

i) Bereitstellen des MHC-Multimers nach einem der Ansprüche 1-3 oder der einzelnen Komponenten davon,
ii) Bereitstellen eines Bestands von T-Zellen, und
iii) Isolieren der T-Zellen, die für das MHC-Multimer spezifisch sind.

**6.** Zusammensetzung, umfassend eine Vielzahl von MHC-Multimeren nach einem der Ansprüche 1-3, und einen Träger.

**7.** Verfahren zum Erfassen von antigenspezifischen T-Zellen, wobei das Verfahren die folgenden Schritte umfasst: 1) Bereitstellen MHC-Multimers nach einem der Ansprüche 1-3, 2) Bereitstellen eines Bestands aus antigenspezifischen T-Zellen, und 3) Erfassen von antigenspezifischen T-Zellen, die für das Peptid P des MHC-Multimers spezifisch sind.

**Revendications**

**1.** Multimère de MHC de classe I comprenant $(a\text{-}b\text{-}P)_n$, dans lequel n > 1,
dans lequel a et b forment ensemble une protéine de MHC de classe I fonctionnelle capable de se lier au peptide antigénique P,
dans lequel (a-b-P) est le complexe peptidique de MHC de classe I formé lorsque le peptide antigénique P se lie à la protéine de MHC de classe I fonctionnelle,
dans lequel chaque complexe peptidique de MHC de classe I d'un multimère de MHC de classe I est associé à un ou plusieurs domaines de multimérisation,
dans lequel P comprend la SEQ ID NO: 5751.

**2.** Multimère de MHC de classe II comprenant $(a\text{-}b\text{-}P)_n$, dans lequel n > 1,
dans lequel a et b forment ensemble une protéine de MHC de classe II fonctionnelle capable de se lier au peptide antigénique P,
dans lequel (a-b-P) est le complexe peptidique de MHC de classe II formé lorsque le peptide antigénique P se lie à la protéine de MHC de classe II fonctionnelle,
dans lequel chaque complexe peptidique de MHC de classe II d'un multimère de MHC de classe II est associé à un ou plusieurs domaines de multimérisation,
dans lequel P comprend la SEQ ID NO: 5751.

**3.** Multimère de MHC selon l'une quelconque des revendications précédentes, dans lequel lesdits un ou plusieurs domaines de multimérisation comprennent un transporteur polysaccharide.

4. Procédé de surveillance immunitaire ou de diagnostic d'une ou de plusieurs maladies comprenant les étapes suivantes

   i) la fourniture du multimère de MHC selon l'une quelconque des revendications 1 à 3, ou des composants individuels de celui-ci,
   ii) la fourniture d'une population de lymphocytes T, et
   iii) la mesure du nombre, de l'activité ou de l'état de lymphocytes T spécifiques audit multimère de MHC, permettant ainsi la surveillance immunitaire ou le diagnostic desdites une ou plusieurs maladies.

5. Procédé d'isolement d'un ou de plusieurs lymphocytes T spécifiques d'un antigène, ledit procédé comprenant les étapes de :

   i) fourniture du multimère de MHC selon l'une quelconque des revendications 1 à 3, ou des composants individuels de celui-ci,
   ii) fourniture d'une population de lymphocytes T, et
   iii) isolement de lymphocytes T spécifiques audit multimère de MHC.

6. Composition comprenant une pluralité de multimères de MHC selon l'une quelconque des revendications 1 à 3 et un transporteur.

7. Procédé de détection de lymphocytes T spécifiques d'un antigène, ledit procédé comprenant les étapes de 1) fourniture d'un multimère de MHC selon les revendications 1 à 3, 2) fourniture d'une population de lymphocytes T spécifiques d'un antigène et 3) détection de lymphocytes T spécifiques d'un antigène spécifiques au peptide P du multimère de MHC.

**Fig 1. Schematic representation of MHC multimer**

## Fig. 2.
## Program for peptide sequence motifs prediction

Imports System.IO

Public Class Form1

```
    Dim TRACE_LOG = ""
    Dim CR = "-"
    Dim BACKSLASH = "\"
    Dim COLON = ":"
    Dim SPACE = " "

    Private Sub Button_valgmappe_Click(ByVal sender As System.Object, ByVal e As
System.EventArgs) Handles Button_valgmappe.Click
        Dim fdlg As FolderBrowserDialog = New FolderBrowserDialog()

        If fdlg.ShowDialog() = Windows.Forms.DialogResult.OK Then
            Txt_mappe.Text = fdlg.SelectedPath
        End If
    End Sub

    Private Sub Button_gem_Click(ByVal sender As System.Object, ByVal e As
System.EventArgs) Handles Button_gem.Click
        If Txt_sekvens.Text = Nothing Then
            MessageBox.Show("Indtast sekvens")
            Exit Sub
        End If
        If Txt_fil.Text = Nothing Then
            MessageBox.Show("Indtast filnavn")
            Exit Sub
        End If
        If Txt_mappe.Text = Nothing Then
            MessageBox.Show("Vælg mappe")
            Exit Sub
        End If

        TRACE_LOG = Txt_mappe.Text + "/" + Txt_fil.Text + ".txt"
        If File.Exists(TRACE_LOG) Then
            MessageBox.Show("Filen findes allerede og kan ikke overskrives")
            Exit Sub
        End If

        Dim n = Txt_sekvens.Text.Length
        Trace_skriv("Sequence length: " & n)
        Trace_skriv("CR")
```

```
Trace_skriv("CR")

Dim min = CInt(Txt_min.Text)
Dim max = CInt(Txt_max.Text)
Dim j As Integer
Dim i As Integer
Dim tmptxt As String
Dim peptid As String

For j = min To max
   Trace_skriv(j & " mers:")
   Trace_skriv("CR")
   tmptxt = ""
   For i = 0 To n - j
      peptid = Txt_sekvens.Text.Substring(i, j)
      If CheckBox_validering.Checked Then
         If valideret(peptid) Then
            tmptxt = tmptxt + peptid & CR
         End If
      Else
         tmptxt = tmptxt + peptid & CR
      End If
   Next
   Trace_skriv(tmptxt)
   Trace_skriv("CR")
   Trace_skriv("CR")
Next

End Sub

Private Function valideret(ByVal peptid As String)
   If CheckBox_validering_stopkodon.Checked Then
      If InStr(peptid, "*") Then
         Return False
      Else
         Return True
      End If
   Else
      Return True
   End If
End Function

Private Sub Form1_Load(ByVal sender As System.Object, ByVal e As
System.EventArgs) Handles MyBase.Load
   Txt_min.Text = 8
   Txt_max.Text = 11
```

```
End Sub

Friend Sub Trace_skriv(ByVal texttoadd As String)
    Dim logtext() As String
    Dim fileline() As String
    Dim fs As StreamWriter
    Dim strace As New StackTrace(True)
    Try
        If Not File.Exists(TRACE_LOG) Then
            fs = File.CreateText(TRACE_LOG)
            'fs.Write("Trace Log " & Format(Now) & CR & CR)
            fs.Flush()
            fs.Close()
        End If
        logtext = strace.GetFrame(1).ToString.Split(Space)
        fileline = logtext(6).Split(BACKSLASH)
        Dim i As Integer = fileline.GetUpperBound(0)
        fs = File.AppendText(TRACE_LOG)
        If texttoadd = "CR" Then
            fs.WriteLine()
        Else
            Dim tmp = Split(texttoadd, CR)
            If UBound(tmp) = 0 Then
                fs.Write(tmp(0))
            Else
                For i = LBound(tmp) To UBound(tmp) - 1
                    fs.Write(tmp(i))
                    If Me.CheckBox_semikolon.Checked Then
                        fs.Write("; ")
                    End If
                    If Me.CheckBox_linie.Checked And i < UBound(tmp) - 1 Then
                        fs.WriteLine()
                    End If
                Next
            End If
        End If
        fs.Flush()
        fs.Close()
    Catch ex As Exception
        MsgBox(ex.ToString)
    End Try
End Sub
End Class
```

## Fig. 3.

**Full List of HLA Class I alleles assigned as of January 2007 from http://www.anthonynolan.org.uk/HIG/lists/class1list.html**

HLA-AHLA-BHLA-CHLA-EHLA-FHLA-G
A\*01010101B\*070201Cw\*010201E\*01010101F\*01010101G\*01010101
A\*01010102NB\*070202Cw\*010202E\*01010102F\*01010102G\*01010102
A\*010102B\*070203Cw\*010203E\*01010103F\*01010103G\*01010103
A\*010103B\*070204Cw\*010204E\*01030101F\*01010104G\*01010104
A\*010104B\*0703Cw\*0103E\*01030102F\*01010105G\*01010105
A\*0102B\*0704Cw\*0104E\*010302F\*01010106G\*01010201
A\*0103B\*070501Cw\*0105E\*010303F\*01010107G\*01010202
A\*0104NB\*070502Cw\*0106E\*010304F\*01010108G\*010103
A\*0106B\*070503Cw\*0107E\*0104F\*01010201G\*010104
A\*0107B\*0706Cw\*0108F\*01010202G\*010105
A\*0108B\*0707Cw\*0109F\*01010203G\*010106
A\*0109B\*0708Cw\*0110F\*01010204G\*010107
A\*0110B\*0709Cw\*0111F\*01010205G\*010108
A\*0111NB\*0710Cw\*0112F\*01010301G\*010109
A\*0112B\*0711Cw\*0113F\*01010302G\*010110
A\*0113B\*0712Cw\*020201F\*01010303G\*0102
A\*0114B\*0713Cw\*020202F\*01010304G\*0103
A\*0115NB\*0714Cw\*020203F\*0102G\*010401
A\*0116NB\*0715Cw\*020205F\*01030101G\*010402
A\*0117B\*0716Cw\*0203F\*01030102G\*010403
A\*0118NB\*0717Cw\*0204F\*0104G\*0105N
A\*0119B\*0718Cw\*0205G\*0106
A\*0120B\*0719Cw\*0206G\*0107
A\*02010101B\*0720Cw\*0207
A\*02010102LB\*0721Cw\*0208
A\*020102B\*0722Cw\*0209
A\*020103B\*0723Cw\*0210
A\*020104B\*0724Cw\*0211
A\*020105B\*0725Cw\*0212
A\*020106B\*0726Cw\*0213
A\*020107B\*0727Cw\*0214
A\*020108B\*0728Cw\*0215
A\*020109B\*0729Cw\*0216
A\*020110B\*0730Cw\*0217
A\*020111B\*0731Cw\*030201
A\*020112B\*0732Cw\*030202
A\*0202B\*0733Cw\*030301
A\*020301B\*0734Cw\*030302
A\*020302B\*0735Cw\*030303
A\*0204B\*0736Cw\*030304
A\*0205B\*0737Cw\*030305

A*020601B*0738Cw*030401
A*020602B*0739Cw*030402
A*020603B*0740Cw*030403
A*0207B*0741Cw*030404
A*0208B*0742Cw*030405
A*0209B*0743Cw*0305
A*0210B*0744Cw*0306
A*0211B*0745Cw*0307
A*0212B*0746Cw*0308
A*0213B*0747Cw*0309
A*0214B*0748Cw*0310
A*0215NB*0749NCw*031101
A*0216B*0750Cw*031102
A*021701B*0751Cw*0312
A*021702B*080101Cw*0313
A*0218B*080102Cw*0314
A*0219B*080103Cw*0315
A*022001B*0802Cw*0316
A*022002B*0803Cw*0317
A*0221B*0804Cw*0318
A*0222B*0805Cw*0319
A*0224B*0806Cw*0320N
A*0225B*0807Cw*0321
A*0226B*0808NCw*0322Q
A*0227B*0809Cw*0323
A*0228B*0810Cw*0324
A*0229B*0811Cw*0325
A*0230B*0812Cw*0326
A*0231B*0813Cw*0327
A*0232NB*0814Cw*0328
A*0233B*0815Cw*0329
A*0234B*0816Cw*0330
A*023501B*0817Cw*0331
A*023502B*0818Cw*0332
A*0236B*0819NCw*0333
A*0237B*0820Cw*0334
A*0238B*0821Cw*0335
A*0239B*0822Cw*04010101
A*0240B*0823Cw*04010102
A*0241B*0824Cw*040102
A*0242B*0825Cw*040103
A*0243NB*0826Cw*040104
A*0244B*0827Cw*0403
A*0245B*0828Cw*040401
A*0246B*0829Cw*040402
A*0247B*0830NCw*0405

A*0248B*0831Cw*0406
A*0249B*1301Cw*0407
A*0250B*130201Cw*0408
A*0251B*130202Cw*0409N
A*0252B*130203Cw*0410
A*0253NB*1303Cw*0411
A*0254B*1304Cw*0412
A*0255B*1306Cw*0413
A*0256B*1307NCw*0414
A*0257B*1308Cw*0415
A*0258B*1309Cw*0416
A*0259B*1310Cw*0417
A*0260B*1311Cw*0418
A*0261B*1312Cw*0419
A*0262B*1313Cw*0420
A*0263B*1314Cw*0421
A*0264B*1315Cw*0423
A*0265B*1316Cw*0424
A*0266B*1317Cw*050101
A*0267B*1401Cw*050102
A*0268B*140201Cw*050103
A*0269B*140202Cw*0502
A*0270B*1403Cw*0503
A*0271B*1404Cw*0504
A*0272B*1405Cw*0505
A*0273B*140601Cw*0506
A*027401B*140602Cw*0507N
A*027402B*1407NCw*0508
A*0275B*15010101Cw*0509
A*0276B*15010102NCw*0510
A*0277B*150102Cw*0511
A*0278B*150103Cw*0512
A*0279B*150104Cw*0513
A*0280B*1502Cw*0514
A*0281B*1503Cw*0515
A*0282NB*1504Cw*06020101
A*0283NB*1505Cw*06020102
A*0284B*1506Cw*060202
A*0285B*1507Cw*0603
A*0286B*1508Cw*0604
A*0287B*1509Cw*0605
A*0288NB*1510Cw*0606
A*0289B*151101Cw*0607
A*0290B*151102Cw*0608
A*0291B*151103Cw*0609
A*0292B*1512Cw*0610

A*0293B*1513Cw*0611
A*0294NB*1514Cw*0612
A*0295B*1515Cw*0613
A*0296B*1516Cw*0614
A*0297B*15170101Cw*070101
A*0299B*15170102Cw*070102
A*03010101B*151702Cw*070103
A*03010102NB*1518Cw*070104
A*03010103B*1519Cw*070105
A*030102B*1520Cw*070106
A*030103B*1521Cw*070107
A*030104B*1523Cw*07020101
A*030105B*1524Cw*07020102
A*0302B*1525Cw*07020103
A*0303NB*1526NCw*0703
A*0304B*1527Cw*070401
A*0305B*1528Cw*070402
A*0306B*1529Cw*0705
A*0307B*1530Cw*0706
A*0308B*1531Cw*0707
A*0309B*1532Cw*0708
A*0310B*1533Cw*0709
A*0311NB*1534Cw*0710
A*0312B*1535Cw*0711
A*0313B*1536Cw*0712
A*0314B*1537Cw*0713
A*0315B*1538Cw*0714
A*0316B*1539Cw*0715
A*0317B*1540Cw*0716
A*0318B*1542Cw*0717
A*0319B*1543Cw*0718
A*0320B*1544Cw*0719
A*0321NB*1545Cw*0720
A*0322B*1546Cw*0721
A*0323B*1547Cw*0722
A*0324B*1548Cw*0723
A*0325B*1549Cw*0724
A*0326B*1550Cw*0725
A*110101B*1551Cw*0726
A*110102B*1552Cw*0727
A*110103B*1553Cw*0728
A*110104B*1554Cw*0729
A*110105B*1555Cw*0730
A*110106B*1556Cw*0731
A*110201B*1557Cw*0732N
A*110202B*1558Cw*0733N

A*1103B*1560Cw*0734
A*1104B*1561Cw*0735
A*1105B*1562Cw*0736
A*1106B*1563Cw*0737
A*1107B*1564Cw*0738
A*1108B*1565Cw*0739
A*1109B*1566Cw*0740
A*1110B*1567Cw*0741
A*1111B*1568Cw*0742
A*1112B*1569Cw*0743
A*1113B*1570Cw*0744
A*1114B*1571Cw*0745
A*1115B*1572Cw*080101
A*1116B*1573Cw*080102
A*1117B*1574Cw*0802
A*1118B*1575Cw*0803
A*1119B*1576Cw*0804
A*1120B*1577Cw*0805
A*1121NB*1578Cw*0806
A*1122B*1579NCw*0807
A*1123B*1580Cw*0808
A*1124B*1581Cw*0809
A*1125B*1582Cw*0810
A*1126B*1583Cw*0811
A*1127B*1584Cw*0812
A*1128B*1585Cw*0813
A*1129B*1586Cw*0814
A*2301B*1587Cw*120201
A*2302B*1588Cw*120202
A*2303B*1589Cw*120203
A*2304B*1590Cw*12030101
A*2305B*1591Cw*12030102
A*2306B*1592Cw*120302
A*2307NB*1593Cw*120303
A*2308NB*1594NCw*120304
A*2309B*1595Cw*120401
A*2310B*1596Cw*120402
A*2311NB*1597Cw*1205
A*2312B*1598Cw*1206
A*2313B*1599Cw*1207
A*2314B*9501Cw*1208
A*24020101B*9502Cw*1209
A*24020102LB*9503Cw*1210
A*240202B*9504Cw*1211
A*240203B*9505Cw*1212
A*240204B*9506Cw*1213

A*240205B*9507Cw*1214
A*240206B*9508Cw*1215
A*240207B*9509Cw*1216
A*240208B*9510Cw*1217
A*240209B*9511NCw*1218
A*240210B*9512Cw*1219
A*240211B*9513Cw*140201
A*240212B*9514Cw*140202
A*240213B*9515Cw*140203
A*240301B*9516Cw*140204
A*240302B*9517Cw*1403
A*2404B*9518Cw*1404
A*2405B*9519Cw*1405
A*2406B*9520Cw*1406
A*2407B*9521Cw*1407N
A*2408B*9522Cw*1408
A*2409NB*180101Cw*150201
A*2410B*180102Cw*150202
A*2411NB*180103Cw*150203
A*2413B*1802Cw*1503
A*2414B*1803Cw*1504
A*2415B*1804Cw*150501
A*2417B*1805Cw*150502
A*2418B*1806Cw*150503
A*2419B*1807Cw*150504
A*2420B*1808Cw*1506
A*2421B*1809Cw*1507
A*2422B*1810Cw*1508
A*2423B*1811Cw*1509
A*2424B*1812Cw*1510
A*2425B*1813Cw*1511
A*2426B*1814Cw*1512
A*2427B*1815Cw*1513
A*2428B*1817NCw*1514
A*2429B*1818Cw*1515
A*2430B*1819Cw*1516
A*2431B*1820Cw*1517
A*2432B*1821Cw*160101
A*2433B*1822Cw*160102
A*2434B*1823NCw*1602
A*2435B*1824Cw*160401
A*2436NB*2701Cw*1606
A*2437B*2702Cw*1607
A*2438B*2703Cw*1608
A*2439B*270401Cw*1609
A*2440NB*270402Cw*1701

A*2441B*270502Cw*1702
A*2442B*270503Cw*1703
A*2443B*270504Cw*1704
A*2444B*270505Cw*1801
A*2445NB*270506Cw*1802
A*2446B*270507
A*2447B*270508
A*2448NB*270509
A*2449B*2706
A*2450B*2707
A*2451B*2708
A*2452B*2709
A*2453B*2710
A*2454B*2711
A*2455B*2712
A*2456B*2713
A*2457B*2714
A*2458B*2715
A*2459B*2716
A*2460NB*2717
A*2461B*2718
A*2462B*2719
A*2463B*2720
A*2464B*2721
A*2465B*2723
A*2466B*2724
A*2467B*2725
A*2468B*2726
A*250101B*2727
A*250102B*2728
A*2502B*2729
A*2503B*2730
A*2504B*2731
A*2505B*2732
A*2506B*2733
A*260101B*2734
A*260102B*2735
A*260103B*2736
A*260104B*350101
A*2602B*350102
A*2603B*350103
A*2604B*350104
A*2605B*350105
A*2606B*350106
A*260701B*350201
A*260702B*350202

A*2608B*3503
A*2609B*350401
A*2610B*350402
A*2611NB*3505
A*2612B*3506
A*2613B*3507
A*2614B*350801
A*2615B*350802
A*2616B*350901
A*2617B*350902
A*2618B*3510
A*2619B*3511
A*2620B*3512
A*2621B*3513
A*2622B*351401
A*2623B*351402
A*2624B*3515
A*2625NB*3516
A*2626B*3517
A*2627B*3518
A*2628B*3519
A*2629B*3520
A*2630B*3521
A*2631B*3522
A*2632B*3523
A*2633B*3524
A*2634B*3525
A*29010101B*3526
A*29010102NB*3527
A*290201B*3528
A*290202B*3529
A*290203B*3530
A*2903B*3531
A*2904B*3532
A*2905B*3533
A*2906B*3534
A*2907B*3535
A*2908NB*3536
A*2909B*3537
A*2910B*3538
A*2911B*3539
A*2912B*3540N
A*2913B*3541
A*2914B*3542
A*2915B*3543
A*2916B*3544

A*300101B*3545
A*300102B*3546
A*300201B*3547
A*300202B*3548
A*300203B*3549
A*3003B*3550
A*3004B*3551
A*3006B*3552
A*3007B*3553N
A*3008B*3554
A*3009B*3555
A*3010B*3556
A*3011B*3557
A*3012B*3558
A*3013B*3559
A*3014LB*3560
A*3015B*3561
A*3016B*3562
A*3017B*3563
A*3018B*3564
A*3019B*3565Q
A*310102B*3566
A*3102B*3567
A*3103B*3568
A*3104B*3569
A*3105B*3570
A*3106B*3571
A*3107B*3572
A*3108B*370101
A*3109B*370102
A*3110B*370103
A*3111B*370104
A*3112B*3702
A*3113B*3703N
A*3114NB*3704
A*3115B*3705
A*3201B*3706
A*3202B*3707
A*3203B*3708
A*3204B*3709
A*3205B*3710
A*3206B*3711
A*3207B*3712
A*3208B*380101
A*3209B*380102
A*3210B*380201

A*3211QB*380202
A*3212B*3803
A*3213B*3804
A*3214B*3805
A*3301B*3806
A*330301B*3807
A*330302B*3808
A*3304B*3809
A*3305B*3810
A*3306B*3811
A*3307B*3812
A*3308B*3813
A*3309B*3814
A*3401B*3815
A*3402B*39010101
A*3403B*39010102L
A*3404B*390103
A*3405B*390104
A*3406B*390201
A*3407B*390202
A*3408B*3903
A*3601B*3904
A*3602B*3905
A*3603B*390601
A*3604B*390602
A*4301B*3907
A*6601B*3908
A*6602B*3909
A*6603B*3910
A*6604B*3911
A*6605B*3912
A*6606B*391301
A*680101B*391302
A*680102B*3914
A*680103B*3915
A*680104B*3916
A*680105B*3917
A*68020101B*3918
A*68020102B*3919
A*680301B*3920
A*680302B*3922
A*6804B*3923
A*6805B*3924
A*6806B*3925N
A*6807B*3926
A*6808B*3927

A*6809B*3928
A*6810B*3929
A*6811NB*3930
A*6812B*3931
A*6813B*3932
A*6814B*3933
A*6815B*3934
A*6816B*3935
A*6817B*3936
A*6818NB*3937
A*6819B*3938Q
A*6820B*3939
A*6821B*3940N
A*6822B*3941
A*6823B*400101
A*6824B*400102
A*6825B*400103
A*6826B*400104
A*6827B*400105
A*6828B*400201
A*6829B*400202
A*6830B*400203
A*6831B*4003
A*6832B*4004
A*6833B*4005
A*6834B*40060101
A*6835B*40060102
A*6836B*400602
A*6901B*4007
A*7401B*4008
A*7402B*4009
A*7403B*4010
A*7404B*4011
A*7405B*4012
A*7406B*4013
A*7407B*401401
A*7408B*401402
A*7409B*401403
A*7410B*4015
A*7411B*4016
A*7412NB*4018
A*8001B*4019
A*9201B*4020
A*9202B*4021
A*9203B*4022N
A*9204B*4023

A*9205B*4024
A*9206B*4025
A*9207B*4026
A*9208B*4027
A*9209B*4028
B*4029
B*4030
B*4031
B*4032
B*4033
B*4034
B*4035
B*4036
B*4037
B*4038
B*4039
B*4040
B*4042
B*4043
B*4044
B*4045
B*4046
B*4047
B*4048
B*4049
B*4050
B*4051
B*4052
B*4053
B*4054
B*4055
B*4056
B*4057
B*4058
B*4059
B*4060
B*4061
B*4062
B*4063
B*4064
B*4065
B*4066
B*4067
B*4068
B*4069
B*4070

B*4101
B*4102
B*4103
B*4104
B*4105
B*4106
B*4107
B*4108
B*4201
B*4202
B*4204
B*420501
B*420502
B*4206
B*4207
B*4208
B*4209
B*44020101
B*44020102S
B*440202
B*440203
B*440204
B*440301
B*440302
B*4404
B*4405
B*4406
B*4407
B*4408
B*4409
B*4410
B*4411
B*4412
B*4413
B*4414
B*4415
B*4416
B*4417
B*4418
B*4419N
B*4420
B*4421
B*4422
B*4423N
B*4424
B*4425

B*4426
B*4427
B*4428
B*4429
B*4430
B*4431
B*4432
B*4433
B*4434
B*4435
B*4436
B*4437
B*4438
B*4439
B*4440
B*4441
B*4442
B*4443
B*4444
B*4445
B*4446
B*4447
B*4448
B*4449
B*4450
B*4451
B*4501
B*4502
B*4503
B*4504
B*4505
B*4506
B*4507
B*460101
B*460102
B*4602
B*4603
B*4604
B*4605
B*4606
B*4607N
B*4608
B*4609
B*47010101
B*47010102
B*4702

B*4703
B*4704
B*4705
B*4801
B*4802
B*480301
B*480302
B*4804
B*4805
B*4806
B*4807
B*4808
B*4809
B*4810
B*4811
B*4812
B*4813
B*4814
B*4815
B*4816
B*4901
B*4902
B*4903
B*4904
B*4905
B*5001
B*5002
B*5004
B*510101
B*510102
B*510103
B*510104
B*510105
B*510106
B*510107
B*510201
B*510202
B*5103
B*5104
B*5105
B*5106
B*5107
B*5108
B*5109
B*5110
B*5111N

B*5112
B*511301
B*511302
B*5114
B*5115
B*5116
B*5117
B*5118
B*5119
B*5120
B*5121
B*5122
B*5123
B*5124
B*5126
B*5127N
B*5128
B*5129
B*5130
B*5131
B*5132
B*5133
B*5134
B*5135
B*5136
B*5137
B*5138
B*5139
B*5140
B*5141N
B*5142
B*5143
B*5144N
B*5145
B*5146
B*520101
B*520102
B*520103
B*520104
B*5202
B*5203
B*5204
B*5205
B*5206
B*5207
B*5208

B*5209
B*5210
B*530101
B*530102
B*530103
B*530104
B*5302
B*5303
B*5304
B*5305
B*5306
B*5307
B*5308
B*5309
B*5310
B*5311
B*5312
B*5401
B*5402
B*5403
B*5404
B*5405N
B*5406
B*5407
B*5408N
B*5409
B*5410
B*5411
B*5412
B*550101
B*550102
B*550103
B*550104
B*550201
B*550202
B*5503
B*5504
B*5505
B*5507
B*5508
B*5509
B*5510
B*5511
B*5512
B*5513
B*5514

B*5515
B*5516
B*5517
B*5518
B*5519
B*5520
B*5521
B*5522
B*5523
B*5524
B*5601
B*5602
B*5603
B*5604
B*560501
B*560502
B*5606
B*5607
B*5608
B*5609
B*5610
B*5611
B*5612
B*5613
B*5614
B*5615
B*5616
B*5617
B*5618
B*5619N
B*570101
B*570102
B*570103
B*5702
B*570301
B*570302
B*5704
B*5705
B*5706
B*5707
B*5708
B*5709
B*5710
B*5711
B*5801
B*5802

B*5804
B*5805
B*5806
B*5807
B*5808
B*5809
B*5810N
B*5811
B*5812
B*5813
B*5814
B*5901
B*5902
B*670101
B*670102
B*6702
B*7301
B*7801
B*780201
B*780202
B*7803
B*7804
B*7805
B*8101
B*8102
B*8201
B*8202
B*8301
HLA-HHLA-JHLA-KHLA-LHLA-P
H*01010101J*01010101K*01010101L*01010101P*01010101
H*01010102J*01010102K*01010102L*01010102P*01010102
H*01010103J*01010103K*01010103L*01010103P*02010101
H*0102J*01010104K*01010104L*010102P*02010102
H*02010101J*01010105K*0102L*0102
H*02010102J*01010106K*0103
H*0202J*01010107
H*0203J*01010108
H*0204J*0201
H*0205
H*0206
H*0301

# Fig. 4.
## Top 30 HLA class 1 allele frequency in human ethnic groups

% chance of allele expressed in an individual

Top 30 expressed alleles

| Allele | Caucasian | Allele | African American | Allele | Hispanic | Allele | Oriental |
|--------|-----------|--------|------------------|--------|----------|--------|----------|
| A*0201 | 45.6% | C*0401 | 29.0% | A*0201 | 37.1% | A*1101 | 38.4% |
| C*0701 | 27.7% | C*0701 | 25.4% | C*0401 | 25.4% | A*2402 | 33.7% |
| A*0101 | 27.4% | C*0602 | 23.0% | A*2402 | 24.9% | C*0702 | 33.3% |
| A*0301 | 23.8% | A*0201 | 22.3% | C*0702 | 24.2% | C*0102 | 27.7% |
| C*0702 | 21.5% | A*2301 | 20.7% | C*0701 | 20.8% | A*3303 | 23.3% |
| C*0401 | 21.2% | C*0202 | 19.0% | C*0304 | 14.4% | C*0801 | 21.6% |
| B*4402 | 20.2% | A*0301 | 18.7% | A*0301 | 14.3% | C*0304 | 19.9% |
| B*0702 | 18.1% | C*0702 | 18.1% | B*0702 | 13.2% | A*0201 | 18.1% |
| B*0801 | 18.1% | B*5301 | 18.1% | B*3501 | 12.8% | B*4001 | 15.2% |
| C*0501 | 17.2% | B*0702 | 15.8% | C*0602 | 12.3% | C*0401 | 14.0% |
| C*0304 | 16.8% | C*1601 | 15.7% | C*0501 | 11.9% | B*5801 | 13.3% |
| C*0602 | 15.7% | B*1503 | 13.9% | A*0101 | 11.4% | B*4601 | 12.7% |
| A*1101 | 15.3% | B*5801 | 13.5% | A*1101 | 11.0% | B*5101 | 12.4% |
| B*4001 | 13.6% | A*6802 | 12.7% | B*5101 | 10.8% | C*0302 | 12.0% |
| A*2402 | 12.1% | C*1701 | 11.7% | C*1601 | 10.6% | B*3802 | 11.4% |
| B*3501 | 10.7% | B*4501 | 10.8% | B*4403 | 9.9% | A*0207 | 11.0% |
| C*0303 | 10.6% | B*4201 | 10.5% | C*0102 | 9.7% | B*1501 | 9.4% |
| B*5101 | 10.4% | A*3001 | 10.4% | A*2902 | 9.7% | A*0206 | 9.3% |
| C*1203 | 9.9% | B*3501 | 10.1% | C*0802 | 9.3% | C*0303 | 9.2% |
| B*1501 | 9.6% | A*0101 | 10.0% | B*1801 | 9.1% | B*1502 | 9.1% |
| A*2902 | 8.9% | C*0304 | 9.3% | A*3101 | 8.9% | A*0203 | 8.8% |
| A*2601 | 8.2% | A*3002 | 9.2% | B*5201 | 8.6% | B*4403 | 8.6% |
| A*3201 | 8.2% | B*0801 | 8.5% | B*1402 | 8.6% | C*1402 | 8.4% |
| C*0802 | 7.7% | A*3402 | 8.4% | C*0202 | 7.6% | B*3501 | 7.2% |
| A*2501 | 7.5% | A*7401 | 8.4% | C*1203 | 7.6% | C*0602 | 7.0% |
| B*5701 | 7.1% | A*3303 | 8.0% | A*2601 | 7.6% | B*5401 | 6.9% |
| B*1402 | 6.7% | C*1801 | 7.3% | A*6801 | 7.1% | B*1301 | 6.6% |
| C*0202 | 6.6% | A*2902 | 7.2% | B*0801 | 7.0% | B*4002 | 6.3% |
| B*1801 | 6.4% | B*4403 | 6.9% | A*3002 | 6.8% | B*5502 | 6.3% |
| B*4403 | 6.4% | B*4901 | 6.9% | B*4402 | 6.5% | A*2601 | 6.0% |

Data from HLA Matchmaker, http://tpis.upmc.edu/tpis/HLAMatchmaker/

A

## Nucleophilic substitution reaction

R–X + R'–O⁻ ⟶ R–O–R'    ETHERS

R–X + R'–S⁻ ⟶ R–S–R'    THIOETHERS

R–X + R'–NH₂ ⟶ R–N–R' (H)   sec- AMINES

R–X + R'–N–R' (H) ⟶ R'–N–R' (R)   tert-AMINES

epoxide + R'–O⁻ ⟶ β-HYDROXY ETHERS

epoxide + R'–S⁻ ⟶ β-HYDROXY THIOETHERS

epoxide + R'–NH₂ ⟶ β-HYDROXY AMINES

aziridine + R'–O⁻ ⟶ β-AMINO ETHERS

R–C(O)–O–R' + R''–NH₂ ⟶ R–C(O)–HN–R''   AMIDES

R–C(O)–S–R' + R''–NH₂ ⟶ R–C(O)–HN–R''   AMIDES

R–C(S)–O–R' + R''–NH₂ ⟶ R–C(S)–HN–R''   THIOAMIDES

R–C(O)–S–R' + R''–NH₂ ⟶ R–C(O)–HN–R''   AMIDES

R–C(S)–O–R' + R''–NH₂ ⟶ R–C(S)–HN–R''   THIOAMIDES

R''–X + R–C(=N–OH)–R' ⟶ R–C(=N–OR'')–R'   OXIMES

R''–SO₂Cl + R–N(H)–R' ⟶ R''SO₂–N(R')(R)   SULFONAMIDES

R'–X + R–⦵–Z (Z') ⟶ R–(Z')–R' (Z)   DI- AND TRI- FUNCTIONAL COMPOUNDS

R'–C(O)–L + R–(Z')–Z ⟶ DI- AND TRI- FUNCTIONAL COMPOUNDS

Z ,Z' = COOR , CHO , COR , CONR''₂ , COO⁻ ,

NO₂ , SOR , SO₂R , SO₂NR''₂ , CN , ect.

## Aromatic nucleophilic substitution

SUBSTITUTED AROMATIC COMPOUNDS

R–Ar(Z)(Z')–X + R'–Nu ⟶ R–Ar(Z)(Z')–Nu–R'

Nu = Oxygen- , Nitrogen- , Sulfur- and Carbon Nucleophiles

X = F, Cl, Br, I, OSO₂CH₃, OSO₂CF₃, OSO₂TOL, ., etc.

Z' ,Z = COOR , CHO , COR , CONR''₂ , COO⁻ , CN ,

NO₂ , SOR , SO₂R , SO₂NR''₂ .. ect.

## Transition metal catalysed reactions

R–Ar(PdX) + R'–(vinyl) ⟶ R–Ar–CH=CH–R'   VINYL SUBSTITUTED AROMATIC COMPOUNDS

R–Ar(PdX) + R'–(alkyne) ⟶ R–Ar–C≡C–R'   ALKYN SUBSTITUTED AROMATIC COMPOUNDS

R–Ar(M) + Ar–X —Pd(PPh₃)₄→ R–Ar–Ar   BIARYL COMPOUNDS

# Fig. 5. Reactive groups and the bonds formed upon their reaction.

## B

### Addition to carbon-carbon multiplebonds

ETHERS   THIO-ETHERS   tert-AMINES   sec-AMINES   HYDRA-ZINES   HYDROXYLAMINE ETHERS

MULTI FUNCTIONAL COMPOUNDS

Z = H, Alkyl, Z', Ar
Z" = COOR, CHO, COR, CONR"$_2$, CN, NO$_2$, SOR, SO$_2$R, SO$_2$NR"$_2$, ect.
Z' = Z"   R = R', = R", = Z

DI- AND TRI- FUNCTIONAL ALKENES

Z = H, Alkyl, Ar,
Z" = Z', Alkyl, Ar,
Z = COOR, CHO, COR, CONR"$_2$, CN, NO$_2$, SOR, SO$_2$R, SO$_2$NR"$_2$, ect.

### Cycloaddition to multiple bounds

SUBSTITUTED CYCLOALKENES

SUBSTITUTED CYCLODIENES

SUBSTITUTED 1,2,3-TRIAZOLES

SUBSTITUTED CYCLOALKENES

SUBSTITUTED CYCLOALKENES

Z = COOR, CHO, COR, COOH COAr CN, NO$_2$, Ar, CH$_2$OH, CH$_2$NH$_2$, CH$_2$CN, SOR, SO$_2$R etc.

R = H, Alkyl, Ar, Z        X = O, NR, CR$_2$, S,

**Fig. 5., continued. Reactive groups and the bonds formed upon their reaction.**

C

**Addition to carbon-hetero multiple bonds**

Z, Z' = COOR, CHO, COR, CONR"$_2$, CN, NO$_2$,SOR, SO$_2$R, SO$_2$NR"$_2$, ect.   R$^n$ = H, Alkyl, Aryl

Z = COOR, CHO, COR, SOR, SO$_2$R, CN, NO$_2$, ect.

R = R', H, Alkyl, Ar,

R" = R''', H, Alkyl,COR,

**Fig. 5., continued. Reactive groups and the bonds formed upon their reaction.**

EP 2 254 592 B1

A. Linker for the formation of Ketones, Aldehydes, Amides and Acids

B. Linker for the formation of Ketones, Amides and Acids

C. Linker for the formation of Aldehydes and Ketones

D. Linker for the formation of Alcohols and Acids

E. Linker for the formation of Amines and Alcohols

F. Linker for the formation of Esters, Thioesters, Amides and Alcohols

G. Linker for the formation of Sulfonamides and Alcohols

H. Linker for the formation of Ketones, Amines and Alcohols

I. Linker for the formation of Ketones, Amines, Alcohols and Mercaptanes

J. Linker for the formation of Biaryl and Bihetaryl

K. Linker for the formation of Benzyles, Amines, Anilins Alcohols and Phenoles

L. Linker for the formation of Mercaptanes

M. Linker for the formation of Glycosides

N. Linker for the formation of Aldehydes and Glyoxylamides

O. Linker for the formation of Aldehydes, Ketones and Aminoalcohols

**Fig. 6. Cleavable linkers, conditions for cleaving them and the resulting products of the cleavage.**

**Fig 7. Size exclusion chromatography of folded HLA-A\*0201-β2m-QLFEELQEL-complex.**

Fig. 8.  MHC-SHIFT Assay

**A**

Flu      Flu      Flu
|         |         |
L90-Lys-L90-Lys-L90-Lys-$NH_2$

$H_2N$-L30-Lys-L30-Lys-L30-Lys-L300-Lys-$NH_2$

CA-biotin

L90-Lys-L90-Lys-L90-Lys-$NH_2$
Flu      Flu      Flu

L90-Lys-L90-Lys-L90-Lys-$NH_2$
Flu      Flu      Flu

L90-Lys-L90-Lys-L90-Lys-$NH_2$
Flu      Flu      Flu

**B**

L15 linker composition:

**Fig. 9. Composition of a Fluorescein-linker molecule**

| Database | Alleles |
|---|---|
| http://www.cbs.dtu.dk/services/NetMHC/ | HLA-A0101, HLA-A0201, HLA-A0202, HLA-A0203, HLA-A0204, HLA-A0206, HLA-A0211, HLA-A0212, HLA-A0216, HLA-A0219, HLA-A0301, HLA-A1101, HLA-A2301, HLA-A2402, HLA-A2403, HLA-A2601, HLA-A2602, HLA-A2902, HLA-A3002, HLA-A3101, HLA-A3301, HLA-A6801, HLA-A6802, HLA-A6901 |
| http://www.cbs.dtu.dk/services/NetMHCII/ | HLA-DRB1*0101, HLA-DRB1*0301, HLA-DRB1*0401, HLA-DRB1*0404, HLA-DRB1*0405, HLA-DRB1*0701, HLA-DRB1*0802, HLA-DRB1*0901, HLA-DRB1*1101, HLA-DRB1*1302, HLA-DRB1*1501, HLA-DRB3*0101, HLA-DRB4*0101, HLA-DRB1*0501 |

**Fig. 10.  HLA alleles of the NetMHC databases**

Fig. 11. Ex vivo ELISPOT analysis of BclX(L)-specific

**Fig 12. PBL from a breast cancer patient analyzed by flow cytometry.**

**Fig. 13. 51-Cr release assay of isolated T cell clones.**

**Fig. 14. Bcl-X(L)173–182 specific clone tested for its cytotoxic potential in 51Cr-release assays.**

| Region | Count | Median | % Hist |
|---|---|---|---|
| Total | 19577 | 5.30, 2344.65 | 100.00 |
| Positive area | 2 | 442.25, 1220.71 | 0.01 |

| Region | Count | Median | % Hist |
|---|---|---|---|
| Total | 34751 | 3.97, 1886.21 | 100.00 |
| Positive area | 18 | 591.09, 1220.71 | 0.05 |

**Fig. 15. Detection of Borrelia specific T cells using MHC dextramers**

**Fig. 16. Detection of CMV specific T cells using MHC dextramers**

Fig. 17. Conformational ELISA

A

B

| MHC-complex | TCR | Percentage of cells in R10 (Positive events) |
|---|---|---|
| HLA-A*0201(NLVPMVATV) | 20 µg dimeric TCR | 99,8% |
| HLA-A*0201(NLVPMVATV) | 10 µg dimeric TCR | 99,8% |
| HLA-A*0201(NLVPMVATV) | 5 µg dimeric TCR | 99,7% |
| HLA-A*0201(NLVPMVATV) | 2 µg dimeric TCR | 93,1% |
| HLA-A*0201(NLVPMVATV) | 0 µg dimeric TCR | 25,1% |
| HLA-A*0201(ILKEPVHGV) | 10 ug dimeric TCR | 1,5% |

**Fig. 18. Carboxylate-modified beads coupled to different amounts of TCR and stained with HLA-A*0201(NLVPMVATV)/RPE or HLA-A*0201(ILKEPVHGV)/RPE.**

Fig. 19.  TCR added into human peripheral whole blood (A), or into HPBMCs (B).

**Fig. 20. Flow cytometry analysis of MHC multimer constructs carrying nonsense peptides**

| | *Positive* | *Negative* | Construct 1 | Construct 2 | Construct 3 | Construct 4 | Construct 5 |
|---|---|---|---|---|---|---|---|
| **Donor** | | | | | | | |
| **1** | – | A2(NLVPMVATV) A2(ILKEPVHGV) | – | – | – | nt | – |
| **2** | A2(NLVPMVATV) | A2(ILKEPVHGV) | – | – | + | nt | – |
| **3** | A2(NLVPMVATV) | A2(ILKEPVHGV) | – | – | + | nt | – |
| **4** | A2(NLVPMVATV) | A2(ILKEPVHGV) | – | – | + | nt | – |
| **5** | A2(NLVPMVATV) | A2(ILKEPVHGV) | – | – | + | nt | – |
| **6** | A2(NLVPMVATV) | A2(ILKEPVHGV) | – | – | + | nt | – |
| **7** | - | A2(ILKEPVHGV) A2(GLCTLVAML) | – | – | nt | - | – |
| **8** | A2(GLCTLVAML) | A2(ILKEPVHGV) | – | – | nt | + | – |
| **9** | A2(GLCTLVAML) | A2(ILKEPVHGV) | – | – | nt | + | – |

**Fig. 21. Summary of flow cytometry analysis of the binding of different MHC multimer constructs to specific T cells in purified Human Peripheral Blood**

**Fig. 22. Gating strategy for no-lyse no-wash procedure**

Donor 1

Donor 2

Donor 3

**Fig. 23.  Identification of CMV-specific T cells in a blood sample using no-lyse no-wash procedure**

**Fig. 24. Enumeration of specific T cells using CytoCount™ beads.**

**Fig. 25. MHC-peptide complexes can be embedded in a sugar matrix together with antibodies and used for detection of specific T cells in a blood sample**

| Borrelia | burgdorferi B31 | afzelii PKo | garini PBi |
|---|---|---|---|
| Genome | NC_001318    851 proteins | NC_008277 855 proteins | NC_006156 832 proteins |
| Plasmids | lp5    NC_000957 | lp25    NC_008569 | lp54    NC_006129 |
| | lp 17    NC_001849 | lp28    NC_0085698 | cp26    NC_006128 |
| | lp21    NC_000955 | lp32    NC_008567 | Variable plasmid segments not finally assembled to whole plasmids NT_108239; NT_108263; NT_108262; NT_108261 NT_108260; NT_108259 NT_108258; NT_108257 NT_108256; NT_108255 NT_108254; NT_108253 NT_108252; NT_108251 NT_108250; NT_108249 NT_108248; NT_108247 NT_108246, NT_108245 NT_108244, NT_108243 NT_108242, NT_108241 NT_108238, NT_108237 NT_108236, NT_108235 NT_108234, NT_108233 NT_108232, NT_108231 NT_108230, NT_108229 NT_108228, NT_108227 |
| | lp25    NC_001850 | lp34    NC_008566 | |
| | lp28-1    NC_001851 | lp60    NC_008564 | |
| | lp28-2    NC_001852 | lp60-2    NC_008565 | |
| | lp28-3    NC_001853 | cp27    NC_008274 | |
| | lp28-4    NC_001854 | cp30    NC_008273 | |
| | lp36    NC_001855 | | |
| | lp38    NC_001856 | | |
| | lp56    NC_000956 | | |
| | cp9    NC_001904 | | |
| | cp26    NC_001903 | | |
| | cp32-1    NC_000948 | | |
| | cp32-3    NC_000949 | | |
| | cp32-4    NC_000950 | | |
| | cp32-6    NC_000951 | | |
| | cp32-7    NC_000952 | | |
| | cp32-8    NC_000953 | | |
| | cp32-9    NC_000954 | | |
| | | | |

## Fig. 26.

# Fig. 27. Borrelia bulgdorferi

| Plasmid or genome | Product Name | Start | End | Strand | Length | Gi | GeneID |
|---|---|---|---|---|---|---|---|
| Plasmid cp32-1(Accession number NC_000948) | hypothetical protein BB_P01 | 66 | 1289 | + | 407 | 11497090 | 1194468 |
| Plasmid cp32-1(Accession number NC_000948) | hypothetical protein BB_P02 | 1306 | 1998 | + | 230 | 11497091 | 1194469 |
| Plasmid cp32-1(Accession number NC_000948) | hypothetical protein BB_P03 | 2011 | 2568 | + | 185 | 11497092 | 1194470 |
| Plasmid cp32-1(Accession number NC_000948) | hypothetical protein BB_P04 | 2575 | 3339 | + | 254 | 11497093 | 1194471 |
| Plasmid cp32-1(Accession number NC_000948) | hypothetical protein BB_P05 | 3369 | 3941 | + | 190 | 11497094 | 1194472 |
| Plasmid cp32-1(Accession number NC_000948) | hypothetical protein BB_P06 | 3948 | 4922 | + | 324 | 11497064 | 1194442 |
| Plasmid cp32-1(Accession number NC_000948) | hypothetical protein BB_P07 | 4936 | 5397 | + | 153 | 11497065 | 1194443 |
| Plasmid cp32-1(Accession number NC_000948) | hypothetical protein BB_P08 | 5379 | 5780 | + | 133 | 11497066 | 1194444 |
| Plasmid cp32-1(Accession number NC_000948) | hypothetical protein BB_P09 | 5768 | 6157 | + | 129 | 11497067 | 1194445 |
| Plasmid cp32-1(Accession number NC_000948) | hypothetical protein BB_P10 | 6154 | 6720 | + | 188 | 11497068 | 1194446 |
| Plasmid cp32-1(Accession number NC_000948) | hypothetical protein BB_P11 | 6701 | 7813 | + | 370 | 11497095 | 1194473 |
| Plasmid cp32-1(Accession number NC_000948) | hypothetical protein BB_P12 | 7828 | 8256 | + | 142 | 11497096 | 1194474 |
| Plasmid cp32-1(Accession number NC_000948) | hypothetical protein BB_P13 | 8272 | 8727 | + | 151 | 11497097 | 1194475 |
| Plasmid cp32-1(Accession number NC_000948) | hypothetical protein BB_P14 | 8724 | 8960 | + | 78 | 11497098 | 1194476 |
| Plasmid cp32-1(Accession number NC_000948) | hypothetical protein BB_P15 | 8968 | 10242 | + | 424 | 11497099 | 1194477 |
| Plasmid cp32-1(Accession number NC_000948) | hypothetical protein BB_P16 | 10265 | 10948 | + | 227 | 11497100 | 1194478 |

| | | | | | | | |
|---|---|---|---|---|---|---|---|
| Plasmid cp32-1(Accession number NC_000948) | hypothetical protein BB_P17 | 10952 | 11902 | + | 316 | 11497101 | 1194479 |
| Plasmid cp32-1(Accession number NC_000948) | hypothetical protein BB_P18 | 11920 | 12465 | + | 181 | 11497102 | 1194480 |
| Plasmid cp32-1(Accession number NC_000948) | hypothetical protein BB_P19 | 12495 | 12827 | + | 110 | 11497069 | 1194447 |
| Plasmid cp32-1(Accession number NC_000948) | hypothetical protein BB_P20 | 12824 | 13699 | + | 291 | 11497070 | 1194448 |
| Plasmid cp32-1(Accession number NC_000948) | hypothetical protein BB_P21 | 13709 | 14314 | + | 201 | 11497071 | 1194449 |
| Plasmid cp32-1(Accession number NC_000948) | hypothetical protein BB_P22 | 14324 | 15139 | + | 271 | 11497072 | 1194450 |
| Plasmid cp32-1(Accession number NC_000948) | pore-forming hemolysin | 15215 | 15418 | + | 67 | 11497073 | 1194451 |
| Plasmid cp32-1(Accession number NC_000948) | hemolysin accessory protein | 15422 | 15769 | + | 115 | 11497074 | 1194452 |
| Plasmid cp32-1(Accession number NC_000948) | hypothetical protein BB_P25 | 15759 | 16094 | + | 111 | 11497075 | 1194453 |
| Plasmid cp32-1(Accession number NC_000948) | hypothetical protein BB_P26 | 16081 | 16440 | + | 119 | 11497076 | 1194454 |
| Plasmid cp32-1(Accession number NC_000948) | rev protein | 16578 | 17060 | - | 160 | 11497077 | 1194455 |
| Plasmid cp32-1(Accession number NC_000948) | lipoprotein | 17232 | 17678 | + | 148 | 11497078 | 1194456 |
| Plasmid cp32-1(Accession number NC_000948) | hypothetical protein BB_P29 | 17715 | 18728 | - | 337 | 11497079 | 1194457 |
| Plasmid cp32-1(Accession number NC_000948) | hypothetical protein BB_P30 | 19114 | 20214 | + | 366 | 11497080 | 1194458 |
| Plasmid cp32-1(Accession number NC_000948) | hypothetical protein BB_P31 | 20224 | 20790 | + | 188 | 11497081 | 1194459 |
| Plasmid cp32-1(Accession number NC_000948) | plasmid partition protein, putative | 20766 | 21506 | + | 246 | 11497082 | 1194460 |
| Plasmid cp32-1(Accession number NC_000948) | hypothetical protein BB_P33 | 21510 | 22118 | + | 202 | 11497083 | 1194461 |
| Plasmid cp32-1(Accession number | BdrA | 22131 | 22763 | + | 210 | 11497084 | 1194462 |

| | | | | | | | |
|---|---|---|---|---|---|---|---|
| NC_000948) | | | | | | | |
| Plasmid cp32-1(Accession number NC_000948) | BppA | 2323 1 | 2455 6 | + | 441 | 11497085 | 1194463 |
| Plasmid cp32-1(Accession number NC_000948) | BppB | 2460 9 | 2503 4 | + | 141 | 11497086 | 1194464 |
| Plasmid cp32-1(Accession number NC_000948) | BppC | 2504 0 | 2581 6 | - | 258 | 11497087 | 1194465 |
| Plasmid cp32-1(Accession number NC_000948) | ErpA | 2624 7 | 2676 8 | + | 173 | 11497061 | 1194439 |
| Plasmid cp32-1(Accession number NC_000948) | ErpB | 2679 6 | 2793 2 | + | 378 | 11497062 | 1194440 |
| Plasmid cp32-1(Accession number NC_000948) | hypothetical protein BB_P40 | 2807 4 | 2865 5 | + | 193 | 11497088 | 1194466 |
| Plasmid cp32-1(Accession number NC_000948) | hypothetical protein BB_P41 | 2883 5 | 2940 1 | + | 188 | 11497089 | 1194467 |
| Plasmid cp32-1(Accession number NC_000948) | hypothetical protein BB_P42 | 2939 8 | 3075 0 | + | 450 | 11497063 | 1194441 |
| Plasmid cp32-3 (Accession number NC_000949) | hypothetical protein BB_S01 | 66 | 1289 | + | 407 | 11497136 | 1194513 |
| Plasmid cp32-3 (Accession number NC_000949) | hypothetical protein BB_S02 | 1306 | 1998 | + | 230 | 11497137 | 1194514 |
| Plasmid cp32-3 (Accession number NC_000949) | hypothetical protein BB_S03 | 2011 | 2568 | + | 185 | 11497138 | 1194515 |
| Plasmid cp32-3 (Accession number NC_000949) | hypothetical protein BB_S04 | 2575 | 3339 | + | 254 | 11497139 | 1194516 |
| Plasmid cp32-3 (Accession number NC_000949) | hypothetical protein BB_S05 | 3369 | 3941 | + | 190 | 11497140 | 1194517 |
| Plasmid cp32-3 (Accession number NC_000949) | hypothetical protein BB_S06 | 3963 | 4922 | + | 319 | 11497125 | 1194502 |
| Plasmid cp32-3 (Accession number NC_000949) | hypothetical protein BB_S07 | 4936 | 5397 | + | 153 | 11497126 | 1194503 |
| Plasmid cp32-3 (Accession number NC_000949) | hypothetical protein BB_S08 | 5379 | 5780 | + | 133 | 11497127 | 1194504 |
| Plasmid cp32-3 (Accession number NC_000949) | hypothetical protein BB_S09 | 5768 | 6157 | + | 129 | 11497128 | 1194505 |

| Plasmid cp32-3 (Accession number NC_000949) | hypothetical protein BB_S10 | 6154 | 6720 | + | 188 | 11497129 | 1194506 |
|---|---|---|---|---|---|---|---|
| Plasmid cp32-3 (Accession number NC_000949) | hypothetical protein BB_S11 | 6701 | 7813 | + | 370 | 11497141 | 1194518 |
| Plasmid cp32-3 (Accession number NC_000949) | hypothetical protein BB_S12 | 7828 | 8256 | + | 142 | 11497142 | 1194519 |
| Plasmid cp32-3 (Accession number NC_000949) | hypothetical protein BB_S13 | 8272 | 8727 | + | 151 | 11497143 | 1194520 |
| Plasmid cp32-3 (Accession number NC_000949) | hypothetical protein BB_S14 | 8724 | 8960 | + | 78 | 11497144 | 1194521 |
| Plasmid cp32-3 (Accession number NC_000949) | hypothetical protein BB_S15 | 8968 | 1024 2 | + | 424 | 11497145 | 1194522 |
| Plasmid cp32-3 (Accession number NC_000949) | hypothetical protein BB_S16 | 1026 5 | 1094 8 | + | 227 | 11497146 | 1194523 |
| Plasmid cp32-3 (Accession number NC_000949) | hypothetical protein BB_S17 | 1095 2 | 1190 2 | + | 316 | 11497147 | 1194524 |
| Plasmid cp32-3 (Accession number NC_000949) | hypothetical protein BB_S18 | 1192 0 | 1246 5 | + | 181 | 11497148 | 1194525 |
| Plasmid cp32-3 (Accession number NC_000949) | hypothetical protein BB_S19 | 1249 5 | 1282 7 | + | 110 | 11497130 | 1194507 |
| Plasmid cp32-3 (Accession number NC_000949) | hypothetical protein BB_S20 | 1282 4 | 1369 9 | + | 291 | 11497131 | 1194508 |
| Plasmid cp32-3 (Accession number NC_000949) | hypothetical protein BB_S21 | 1378 4 | 1431 4 | + | 176 | 11497104 | 1194481 |
| Plasmid cp32-3 (Accession number NC_000949) | hypothetical protein BB_S22 | 1432 4 | 1513 6 | + | 270 | 11497105 | 1194482 |
| Plasmid cp32-3 (Accession number NC_000949) | pore-forming hemolysin | 1521 2 | 1541 5 | + | 67 | 11497106 | 1194483 |
| Plasmid cp32-3 (Accession number NC_000949) | hemolysin accessory protein | 1541 9 | 1576 6 | + | 115 | 11497107 | 1194484 |
| Plasmid cp32-3 (Accession number NC_000949) | hypothetical protein BB_S25 | 1575 6 | 1609 1 | + | 111 | 11497108 | 1194485 |
| Plasmid cp32-3 (Accession number NC_000949) | hypothetical protein BB_S26 | 1607 8 | 1643 7 | + | 119 | 11497109 | 1194486 |
| Plasmid cp32-3 (Accession number NC_000949) | hypothetical protein BB_S27 | 1658 6 | 1690 3 | + | 105 | 11497132 | 1194509 |

| Plasmid cp32-3 (Accession number NC_000949) | hypothetical protein BB_S28 | 1691 5 | 1704 9 | + | 44 | 11497133 | 1194510 |
|---|---|---|---|---|---|---|---|
| Plasmid cp32-3 (Accession number NC_000949) | BdrF | 1706 8 | 1769 7 | + | 209 | 11497110 | 1194487 |
| Plasmid cp32-3 (Accession number NC_000949) | lipoprotein | 1780 3 | 1824 9 | + | 148 | 11497111 | 1194488 |
| Plasmid cp32-3 (Accession number NC_000949) | hypothetical protein BB_S31 | 1828 7 | 1915 9 | - | 290 | 11497112 | 1194489 |
| Plasmid cp32-3 (Accession number NC_000949) | hypothetical protein BB_S32 | 1919 8 | 1939 5 | + | 65 | 11497113 | 1194490 |
| Plasmid cp32-3 (Accession number NC_000949) | hypothetical protein BB_S33 | 1960 5 | 2070 5 | + | 366 | 11497114 | 1194491 |
| Plasmid cp32-3 (Accession number NC_000949) | hypothetical protein BB_S34 | 2071 5 | 2128 1 | + | 188 | 11497115 | 1194492 |
| Plasmid cp32-3 (Accession number NC_000949) | plasmid partition protein, putative | 2125 7 | 2199 7 | + | 246 | 11497116 | 1194493 |
| Plasmid cp32-3 (Accession number NC_000949) | hypothetical protein BB_S36 | 2203 8 | 2258 0 | + | 180 | 11497117 | 1194494 |
| Plasmid cp32-3 (Accession number NC_000949) | BdrE | 2259 3 | 2318 3 | + | 196 | 11497118 | 1194495 |
| Plasmid cp32-3 (Accession number NC_000949) | BppA | 2364 9 | 2501 6 | + | 455 | 11497119 | 1194496 |
| Plasmid cp32-3 (Accession number NC_000949) | BppB | 2506 9 | 2549 4 | + | 141 | 11497120 | 1194497 |
| Plasmid cp32-3 (Accession number NC_000949) | BppC | 2550 0 | 2627 6 | - | 258 | 11497121 | 1194498 |
| Plasmid cp32-3 (Accession number NC_000949) | ErpG | 2670 8 | 2729 8 | + | 196 | 11497122 | 1194499 |
| Plasmid cp32-3 (Accession number NC_000949) | BapA | 2741 0 | 2791 9 | + | 169 | 11497123 | 1194500 |
| Plasmid cp32-3 (Accession number NC_000949) | hypothetical protein BB_S43 | 2806 7 | 2824 9 | + | 60 | 11497134 | 1194511 |
| Plasmid cp32-3 (Accession number NC_000949) | hypothetical protein BB_S44 | 2823 6 | 2887 4 | + | 212 | 11497135 | 1194512 |
| Plasmid cp32-3 (Accession number NC_000949) | hypothetical protein BB_S45 | 2887 1 | 3022 3 | + | 450 | 11497124 | 1194501 |

| | | | | | | | |
|---|---|---|---|---|---|---|---|
| Plasmid cp32-4 (Accession number NC_000950) | hypothetical protein BB_R01 | 66 | 1289 | + | 407 | 11497179 | 1194556 |
| Plasmid cp32-4 (Accession number NC_000950) | hypothetical protein BB_R03 | 2013 | 2576 | + | 187 | 11497180 | 1194557 |
| Plasmid cp32-4 (Accession number NC_000950) | hypothetical protein BB_R04 | 2580 | 3347 | + | 255 | 11497181 | 1194558 |
| Plasmid cp32-4 (Accession number NC_000950) | hypothetical protein BB_R05 | 3340 | 3951 | + | 203 | 11497182 | 1194559 |
| Plasmid cp32-4 (Accession number NC_000950) | hypothetical protein BB_R06 | 3958 | 4932 | + | 324 | 11497153 | 1194529 |
| Plasmid cp32-4 (Accession number NC_000950) | hypothetical protein BB_R07 | 4952 | 5407 | + | 151 | 11497154 | 1194530 |
| Plasmid cp32-4 (Accession number NC_000950) | hypothetical protein BB_R08 | 5389 | 5790 | + | 133 | 11497155 | 1194531 |
| Plasmid cp32-4 (Accession number NC_000950) | hypothetical protein BB_R09 | 5778 | 6167 | + | 129 | 11497156 | 1194532 |
| Plasmid cp32-4 (Accession number NC_000950) | hypothetical protein BB_R10 | 6164 | 6730 | + | 188 | 11497157 | 1194533 |
| Plasmid cp32-4 (Accession number NC_000950) | hypothetical protein BB_R11 | 6711 | 7823 | + | 370 | 11497183 | 1194560 |
| Plasmid cp32-4 (Accession number NC_000950) | hypothetical protein BB_R12 | 7838 | 8266 | + | 142 | 11497184 | 1194561 |
| Plasmid cp32-4 (Accession number NC_000950) | hypothetical protein BB_R13 | 8282 | 8737 | + | 151 | 11497185 | 1194562 |
| Plasmid cp32-4 (Accession number NC_000950) | hypothetical protein BB_R14 | 8734 | 8970 | + | 78 | 11497186 | 1194563 |
| Plasmid cp32-4 (Accession number NC_000950) | hypothetical protein BB_R15 | 8978 | 10273 | + | 431 | 11497187 | 1194564 |
| Plasmid cp32-4 (Accession number NC_000950) | hypothetical protein BB_R16 | 10296 | 10892 | + | 198 | 11497188 | 1194565 |
| Plasmid cp32-4 (Accession number NC_000950) | hypothetical protein BB_R17 | 10896 | 11846 | + | 316 | 11497189 | 1194566 |
| Plasmid cp32-4 (Accession number NC_000950) | hypothetical protein BB_R18 | 11864 | 12418 | + | 184 | 11497190 | 1194567 |
| Plasmid cp32-4 (Accession number NC_000950) | hypothetical protein BB_R19 | 12448 | 12780 | + | 110 | 11497158 | 1194534 |

| Plasmid cp32-4 (Accession number NC_000950) | hypothetical protein BB_R20 | 1277 7 | 1365 2 | + | 291 | 11497159 | 1194535 |
|---|---|---|---|---|---|---|---|
| Plasmid cp32-4 (Accession number NC_000950) | hypothetical protein BB_R21 | 1366 2 | 1426 7 | + | 201 | 11497160 | 1194536 |
| Plasmid cp32-4 (Accession number NC_000950) | hypothetical protein BB_R22 | 1427 7 | 1509 2 | + | 271 | 11497161 | 1194537 |
| Plasmid cp32-4 (Accession number NC_000950) | pore-forming hemolysin | 1516 7 | 1537 0 | + | 67 | 11497162 | 1194538 |
| Plasmid cp32-4 (Accession number NC_000950) | hemolysin accessory protein | 1537 4 | 1572 1 | + | 115 | 11497163 | 1194539 |
| Plasmid cp32-4 (Accession number NC_000950) | hypothetical protein BB_R25 | 1571 1 | 1604 6 | + | 111 | 11497164 | 1194540 |
| Plasmid cp32-4 (Accession number NC_000950) | hypothetical protein BB_R26 | 1603 3 | 1639 2 | + | 119 | 11497165 | 1194541 |
| Plasmid cp32-4 (Accession number NC_000950) | BdrH | 1646 7 | 1699 7 | + | 176 | 11497166 | 1194542 |
| Plasmid cp32-4 (Accession number NC_000950) | lipoprotein | 1710 3 | 1752 5 | + | 140 | 11497167 | 1194543 |
| Plasmid cp32-4 (Accession number NC_000950) | hypothetical protein BB_R29 | 1757 3 | 1866 4 | - | 363 | 11497168 | 1194544 |
| Plasmid cp32-4 (Accession number NC_000950) | hypothetical protein BB_R30 | 1873 4 | 1882 9 | - | 31 | 11497191 | 1194568 |
| Plasmid cp32-4 (Accession number NC_000950) | hypothetical protein BB_R31 | 1896 0 | 2005 7 | + | 365 | 11497169 | 1194545 |
| Plasmid cp32-4 (Accession number NC_000950) | hypothetical protein BB_R32 | 2006 7 | 2063 3 | + | 188 | 11497170 | 1194546 |
| Plasmid cp32-4 (Accession number NC_000950) | plasmid partition protein, putative | 2060 9 | 2136 4 | + | 251 | 11497171 | 1194547 |
| Plasmid cp32-4 (Accession number NC_000950) | hypothetical protein BB_R34 | 2141 5 | 2196 0 | + | 181 | 11497172 | 1194548 |
| Plasmid cp32-4 (Accession number NC_000950) | BdrG | 2197 4 | 2225 2 | + | 92 | 21538986 | 1194549 |
| Plasmid cp32-4 (Accession number NC_000950) | BppA | 2283 1 | 2415 6 | + | 441 | 11497173 | 1194550 |
| Plasmid cp32-4 (Accession number | BppB | 2421 0 | 2463 5 | + | 141 | 11497174 | 1194551 |

| NC_000950) | | | | | | | |
|---|---|---|---|---|---|---|---|
| Plasmid cp32-4 (Accession number NC_000950) | BppC | 2464 1 | 2543 5 | - | 264 | 11497175 | 1194552 |
| Plasmid cp32-4 (Accession number NC_000950) | hypothetical protein BB_R39 | 2553 5 | 2563 6 | - | 33 | 11497192 | 1194569 |
| Plasmid cp32-4 (Accession number NC_000950) | ErpH | 2586 5 | 2596 9 | + | 34 | 11497176 | 1194553 |
| Plasmid cp32-4 (Accession number NC_000950) | hypothetical protein BB_R41 | 2607 7 | 2682 0 | + | 247 | 11497150 | 1194526 |
| Plasmid cp32-4 (Accession number NC_000950) | ErpY | 2685 3 | 2752 7 | + | 224 | 11497151 | 1194527 |
| Plasmid cp32-4 (Accession number NC_000950) | hypothetical protein BB_R43 | 2763 4 | 2820 3 | + | 189 | 11497177 | 1194554 |
| Plasmid cp32-4 (Accession number NC_000950) | hypothetical protein BB_R44 | 2838 4 | 2895 0 | + | 188 | 11497178 | 1194555 |
| Plasmid cp32-4 (Accession number NC_000950) | hypothetical protein BB_R45 | 2894 7 | 3029 9 | + | 450 | 11497152 | 1194528 |
| Plasmid cp32-6 (Accession number NC_000951) | hypothetical protein BB_M01 | 66 | 1289 | + | 407 | 11497225 | 1194601 |
| Plasmid cp32-6 (Accession number NC_000951) | hypothetical protein BB_M02 | 1306 | 1998 | + | 230 | 11497226 | 1194602 |
| Plasmid cp32-6 (Accession number NC_000951) | hypothetical protein BB_M03 | 2010 | 2573 | + | 187 | 11497227 | 1194603 |
| Plasmid cp32-6 (Accession number NC_000951) | hypothetical protein BB_M04 | 2577 | 3344 | + | 255 | 11497228 | 1194604 |
| Plasmid cp32-6 (Accession number NC_000951) | hypothetical protein BB_M05 | 3337 | 3948 | + | 203 | 11497229 | 1194605 |
| Plasmid cp32-6 (Accession number NC_000951) | hypothetical protein BB_M06 | 3955 | 4929 | + | 324 | 11497215 | 1194591 |
| Plasmid cp32-6 (Accession number NC_000951) | hypothetical protein BB_M07 | 4949 | 5404 | + | 151 | 11497216 | 1194592 |
| Plasmid cp32-6 (Accession number NC_000951) | hypothetical protein BB_M08 | 5386 | 5787 | + | 133 | 11497217 | 1194593 |
| Plasmid cp32-6 (Accession number NC_000951) | hypothetical protein BB_M09 | 5775 | 6164 | + | 129 | 11497218 | 1194594 |

| | | | | | | | |
|---|---|---|---|---|---|---|---|
| Plasmid cp32-6 (Accession number NC_000951) | hypothetical protein BB_M10 | 6161 | 6730 | + | 189 | 11497219 | 1194595 |
| Plasmid cp32-6 (Accession number NC_000951) | hypothetical protein BB_M11 | 6711 | 7823 | + | 370 | 11497230 | 1194606 |
| Plasmid cp32-6 (Accession number NC_000951) | hypothetical protein BB_M12 | 7838 | 8266 | + | 142 | 11497231 | 1194607 |
| Plasmid cp32-6 (Accession number NC_000951) | hypothetical protein BB_M13 | 8282 | 8737 | + | 151 | 11497232 | 1194608 |
| Plasmid cp32-6 (Accession number NC_000951) | hypothetical protein BB_M14 | 8734 | 8970 | + | 78 | 11497233 | 1194609 |
| Plasmid cp32-6 (Accession number NC_000951) | hypothetical protein BB_M15 | 8978 | 10252 | + | 424 | 11497234 | 1194610 |
| Plasmid cp32-6 (Accession number NC_000951) | hypothetical protein BB_M16 | 10275 | 10958 | + | 227 | 11497235 | 1194611 |
| Plasmid cp32-6 (Accession number NC_000951) | hypothetical protein BB_M17 | 10962 | 11912 | + | 316 | 11497220 | 1194596 |
| Plasmid cp32-6 (Accession number NC_000951) | hypothetical protein BB_M18 | 11930 | 12484 | + | 184 | 11497221 | 1194597 |
| Plasmid cp32-6 (Accession number NC_000951) | hypothetical protein BB_M19 | 12514 | 12846 | + | 110 | 11497194 | 1194570 |
| Plasmid cp32-6 (Accession number NC_000951) | hypothetical protein BB_M20 | 12843 | 13718 | + | 291 | 11497195 | 1194571 |
| Plasmid cp32-6 (Accession number NC_000951) | hypothetical protein BB_M21 | 13728 | 14333 | + | 201 | 11497196 | 1194572 |
| Plasmid cp32-6 (Accession number NC_000951) | hypothetical protein BB_M22 | 14343 | 15155 | + | 270 | 11497197 | 1194573 |
| Plasmid cp32-6 (Accession number NC_000951) | pore-forming hemolysin | 15231 | 15434 | + | 67 | 11497198 | 1194574 |
| Plasmid cp32-6 (Accession number NC_000951) | hemolysin accessory protein | 15438 | 15785 | + | 115 | 11497199 | 1194575 |
| Plasmid cp32-6 (Accession number NC_000951) | hypothetical protein BB_M25 | 15775 | 16110 | + | 111 | 11497200 | 1194576 |
| Plasmid cp32-6 (Accession number NC_000951) | hypothetical protein BB_M26 | 16097 | 16456 | + | 119 | 11497201 | 1194577 |
| Plasmid cp32-6 (Accession number NC_000951) | rev protein | 16593 | 17075 | - | 160 | 11497202 | 1194578 |

| | | | | | | | |
|---|---|---|---|---|---|---|---|
| Plasmid cp32-6 (Accession number NC_000951) | lipoprotein | 1724 7 | 1769 6 | + | 149 | 11497203 | 1194579 |
| Plasmid cp32-6 (Accession number NC_000951) | hypothetical protein BB_M29 | 1773 3 | 1868 0 | - | 315 | 11497204 | 1194580 |
| Plasmid cp32-6 (Accession number NC_000951) | hypothetical protein BB_M30 | 1906 9 | 2016 9 | + | 366 | 11497205 | 1194581 |
| Plasmid cp32-6 (Accession number NC_000951) | hypothetical protein BB_M31 | 2017 9 | 2074 5 | + | 188 | 11497206 | 1194582 |
| Plasmid cp32-6 (Accession number NC_000951) | plasmid partition protein, putative | 2072 1 | 2147 0 | + | 249 | 11497207 | 1194583 |
| Plasmid cp32-6 (Accession number NC_000951) | hypothetical protein BB_M33 | 2152 0 | 2209 8 | + | 192 | 11497208 | 1194584 |
| Plasmid cp32-6 (Accession number NC_000951) | BdrK | 2210 2 | 2277 0 | + | 222 | 11497209 | 1194585 |
| Plasmid cp32-6 (Accession number NC_000951) | BppA | 2324 1 | 2456 6 | + | 441 | 11497210 | 1194586 |
| Plasmid cp32-6 (Accession number NC_000951) | BppB | 2461 9 | 2504 4 | + | 141 | 11497211 | 1194587 |
| Plasmid cp32-6 (Accession number NC_000951) | BppC | 2505 0 | 2582 0 | - | 256 | 11497212 | 1194588 |
| Plasmid cp32-6 (Accession number NC_000951) | ErpK | 2624 5 | 2701 5 | + | 256 | 11497213 | 1194589 |
| Plasmid cp32-6 (Accession number NC_000951) | hypothetical protein BB_M39 | 2707 7 | 2774 5 | - | 222 | 11497223 | 1194599 |
| Plasmid cp32-6 (Accession number NC_000951) | hypothetical protein BB_M40 | 2773 1 | 2785 3 | + | 40 | 11497222 | 1194598 |
| Plasmid cp32-6 (Accession number NC_000951) | hypothetical protein BB_M41 | 2792 3 | 2848 9 | + | 188 | 11497224 | 1194600 |
| Plasmid cp32-6 (Accession number NC_000951) | hypothetical protein BB_M42 | 2848 6 | 2983 8 | + | 450 | 11497214 | 1194590 |
| Plasmid cp32-7 (Accession number NC_000952) | hypothetical protein BB_O01 | 65 | 1288 | + | 407 | 11497267 | 1194642 |
| Plasmid cp32-7 (Accession number NC_000952) | hypothetical protein BB_O02 | 1305 | 1997 | + | 230 | 11497268 | 1194643 |

| | | | | | | | |
|---|---|---|---|---|---|---|---|
| Plasmid cp32-7 (Accession number NC_000952) | hypothetical protein BB_O03 | 2010 | 2567 | + | 185 | 11497269 | 1194644 |
| Plasmid cp32-7 (Accession number NC_000952) | hypothetical protein BB_O04 | 2574 | 3338 | + | 254 | 11497270 | 1194645 |
| Plasmid cp32-7 (Accession number NC_000952) | hypothetical protein BB_O05 | 3368 | 3940 | + | 190 | 11497271 | 1194646 |
| Plasmid cp32-7 (Accession number NC_000952) | hypothetical protein BB_O06 | 3962 | 4921 | + | 319 | 11497248 | 1194623 |
| Plasmid cp32-7 (Accession number NC_000952) | hypothetical protein BB_O07 | 4935 | 5396 | + | 153 | 11497249 | 1194624 |
| Plasmid cp32-7 (Accession number NC_000952) | hypothetical protein BB_O08 | 5378 | 5779 | + | 133 | 11497250 | 1194625 |
| Plasmid cp32-7 (Accession number NC_000952) | hypothetical protein BB_O09 | 5767 | 6156 | + | 129 | 11497251 | 1194626 |
| Plasmid cp32-7 (Accession number NC_000952) | hypothetical protein BB_O10 | 6153 | 6722 | + | 189 | 11497252 | 1194627 |
| Plasmid cp32-7 (Accession number NC_000952) | hypothetical protein BB_O11 | 6703 | 7815 | + | 370 | 11497272 | 1194647 |
| Plasmid cp32-7 (Accession number NC_000952) | hypothetical protein BB_O12 | 7830 | 8258 | + | 142 | 11497273 | 1194648 |
| Plasmid cp32-7 (Accession number NC_000952) | hypothetical protein BB_O13 | 8274 | 8729 | + | 151 | 11497274 | 1194649 |
| Plasmid cp32-7 (Accession number NC_000952) | hypothetical protein BB_O14 | 8726 | 8962 | + | 78 | 11497275 | 1194650 |
| Plasmid cp32-7 (Accession number NC_000952) | hypothetical protein BB_O15 | 8970 | 10304 | + | 444 | 11497276 | 1194651 |
| Plasmid cp32-7 (Accession number NC_000952) | hypothetical protein BB_O16 | 10317 | 10958 | + | 213 | 11497277 | 1194652 |
| Plasmid cp32-7 (Accession number NC_000952) | hypothetical protein BB_O17 | 10962 | 11903 | + | 313 | 11497278 | 1194653 |
| Plasmid cp32-7 (Accession number NC_000952) | hypothetical protein BB_O18 | 11904 | 12473 | + | 189 | 11497279 | 1194654 |
| Plasmid cp32-7 (Accession number NC_000952) | hypothetical protein BB_O19 | 12503 | 12835 | + | 110 | 11497253 | 1194628 |

| | | | | | | | |
|---|---|---|---|---|---|---|---|
| Plasmid cp32-7 (Accession number NC_000952) | hypothetical protein BB_O20 | 1283 2 | 1371 0 | + | 292 | 11497254 | 1194629 |
| Plasmid cp32-7 (Accession number NC_000952) | hypothetical protein BB_O21 | 1371 6 | 1432 1 | + | 201 | 11497255 | 1194630 |
| Plasmid cp32-7 (Accession number NC_000952) | hypothetical protein BB_O22 | 1433 1 | 1514 6 | + | 271 | 11497256 | 1194631 |
| Plasmid cp32-7 (Accession number NC_000952) | pore-forming hemolysin | 1522 2 | 1542 5 | + | 67 | 11497257 | 1194632 |
| Plasmid cp32-7 (Accession number NC_000952) | hemolysin accessory protein | 1542 9 | 1578 5 | + | 118 | 11497258 | 1194633 |
| Plasmid cp32-7 (Accession number NC_000952) | hypothetical protein BB_O25 | 1576 6 | 1610 1 | + | 111 | 11497259 | 1194634 |
| Plasmid cp32-7 (Accession number NC_000952) | hypothetical protein BB_O26 | 1608 8 | 1644 7 | + | 119 | 11497260 | 1194635 |
| Plasmid cp32-7 (Accession number NC_000952) | BdrN | 1652 2 | 1713 9 | + | 205 | 11497261 | 1194636 |
| Plasmid cp32-7 (Accession number NC_000952) | lipoprotein | 1724 5 | 1766 7 | + | 140 | 11497262 | 1194637 |
| Plasmid cp32-7 (Accession number NC_000952) | hypothetical protein BB_O29 | 1771 2 | 1877 0 | - | 352 | 11497280 | 1194655 |
| Plasmid cp32-7 (Accession number NC_000952) | hypothetical protein BB_O30 | 1911 7 | 2021 4 | + | 365 | 11497237 | 1194612 |
| Plasmid cp32-7 (Accession number NC_000952) | hypothetical protein BB_O31 | 2022 4 | 2079 0 | + | 188 | 11497238 | 1194613 |
| Plasmid cp32-7 (Accession number NC_000952) | plasmid partition protein, putative | 2076 6 | 2151 5 | + | 249 | 11497239 | 1194614 |
| Plasmid cp32-7 (Accession number NC_000952) | hypothetical protein BB_O33 | 2152 2 | 2207 6 | + | 184 | 11497240 | 1194615 |
| Plasmid cp32-7 (Accession number NC_000952) | BdrM | 2208 8 | 2266 0 | + | 190 | 11497241 | 1194616 |
| Plasmid cp32-7 (Accession number NC_000952) | hypothetical protein BB_O35 | 2262 7 | 2275 5 | - | 42 | 11497263 | 1194638 |
| Plasmid cp32-7 (Accession number NC_000952) | BppA | 2309 3 | 2446 0 | + | 455 | 11497242 | 1194617 |
| Plasmid cp32-7 (Accession number | BppB | 2451 3 | 2493 8 | + | 141 | 11497243 | 1194618 |

| | | | | | | | |
|---|---|---|---|---|---|---|---|
| NC_000952) | | | | | | | |
| Plasmid cp32-7 (Accession number NC_000952) | BppC | 2494 4 | 2572 0 | - | 258 | 11497244 | 1194619 |
| Plasmid cp32-7 (Accession number NC_000952) | ErpL | 2615 2 | 2684 1 | + | 229 | 11497245 | 1194620 |
| Plasmid cp32-7 (Accession number NC_000952) | ErpM | 2689 3 | 2798 4 | + | 363 | 11497246 | 1194621 |
| Plasmid cp32-7 (Accession number NC_000952) | hypothetical protein BB_O41 | 2800 4 | 2811 7 | - | 37 | 11497264 | 1194639 |
| Plasmid cp32-7 (Accession number NC_000952) | hypothetical protein BB_O42 | 2813 4 | 2870 3 | + | 189 | 11497265 | 1194640 |
| Plasmid cp32-7 (Accession number NC_000952) | hypothetical protein BB_O43 | 2888 5 | 2945 1 | + | 188 | 11497266 | 1194641 |
| Plasmid cp32-7 (Accession number NC_000952) | hypothetical protein BB_O44 | 2944 8 | 3080 0 | + | 450 | 11497247 | 1194622 |
| Plasmid cp32-8 (Accession number NC_000953) | hypothetical protein BB_L01 | 66 | 1289 | + | 407 | 11497312 | 1194686 |
| Plasmid cp32-8 (Accession number NC_000953) | hypothetical protein BB_L02 | 1306 | 1998 | + | 230 | 11497313 | 1194687 |
| Plasmid cp32-8 (Accession number NC_000953) | hypothetical protein BB_L03 | 2011 | 2568 | + | 185 | 11497314 | 1194688 |
| Plasmid cp32-8 (Accession number NC_000953) | hypothetical protein BB_L04 | 2575 | 3339 | + | 254 | 11497315 | 1194689 |
| Plasmid cp32-8 (Accession number NC_000953) | hypothetical protein BB_L05 | 3369 | 3941 | + | 190 | 11497316 | 1194690 |
| Plasmid cp32-8 (Accession number NC_000953) | hypothetical protein BB_L06 | 3948 | 4922 | + | 324 | 11497293 | 1194667 |
| Plasmid cp32-8 (Accession number NC_000953) | hypothetical protein BB_L07 | 4936 | 5397 | + | 153 | 11497294 | 1194668 |

| Plasmid cp32-8 (Accession number NC_000953) | hypothetical protein BB_L08 | 5379 | 5780 | + | 133 | 11497295 | 1194669 |
|---|---|---|---|---|---|---|---|
| Plasmid cp32-8 (Accession number NC_000953) | hypothetical protein BB_L09 | 5768 | 6157 | + | 129 | 11497296 | 1194670 |
| Plasmid cp32-8 (Accession number NC_000953) | hypothetical protein BB_L10 | 6154 | 6720 | + | 188 | 11497297 | 1194671 |
| Plasmid cp32-8 (Accession number NC_000953) | hypothetical protein BB_L11 | 6701 | 7813 | + | 370 | 11497317 | 1194691 |
| Plasmid cp32-8 (Accession number NC_000953) | hypothetical protein BB_L12 | 7828 | 8256 | + | 142 | 11497318 | 1194692 |
| Plasmid cp32-8 (Accession number NC_000953) | hypothetical protein BB_L13 | 8272 | 8727 | + | 151 | 11497319 | 1194693 |
| Plasmid cp32-8 (Accession number NC_000953) | hypothetical protein BB_L14 | 8724 | 8960 | + | 78 | 11497320 | 1194694 |
| Plasmid cp32-8 (Accession number NC_000953) | hypothetical protein BB_L15 | 8968 | 10242 | + | 424 | 11497321 | 1194695 |
| Plasmid cp32-8 (Accession number NC_000953) | hypothetical protein BB_L16 | 10265 | 10948 | + | 227 | 11497322 | 1194696 |
| Plasmid cp32-8 (Accession number NC_000953) | hypothetical protein BB_L17 | 10952 | 11902 | + | 316 | 11497323 | 1194697 |
| Plasmid cp32-8 (Accession number NC_000953) | hypothetical protein BB_L18 | 11920 | 12465 | + | 181 | 11497324 | 1194698 |
| Plasmid cp32-8 (Accession number NC_000953) | hypothetical protein BB_L19 | 12495 | 12827 | + | 110 | 11497298 | 1194672 |

| | | | | | | | |
|---|---|---|---|---|---|---|---|
| Plasmid cp32-8 (Accession number NC_000953) | hypothetical protein BB_L20 | 12824 | 13699 | + | 291 | 11497299 | 1194673 |
| Plasmid cp32-8 (Accession number NC_000953) | hypothetical protein BB_L21 | 13709 | 14314 | + | 201 | 11497300 | 1194674 |
| Plasmid cp32-8 (Accession number NC_000953) | hypothetical protein BB_L22 | 14324 | 15139 | + | 271 | 11497301 | 1194675 |
| Plasmid cp32-8 (Accession number NC_000953) | pore forming hemolysin | 15215 | 15418 | + | 67 | 11497302 | 1194676 |
| Plasmid cp32-8 (Accession number NC_000953) | hemolysin accessory protein | 15422 | 15769 | + | 115 | 11497303 | 1194677 |
| Plasmid cp32-8 (Accession number NC_000953) | hypothetical protein BB_L25 | 15759 | 16094 | + | 111 | 11497304 | 1194678 |
| Plasmid cp32-8 (Accession number NC_000953) | hypothetical protein BB_L26 | 16081 | 16440 | + | 119 | 11497305 | 1194679 |
| Plasmid cp32-8 (Accession number NC_000953) | BdrP | 16515 | 17099 | + | 194 | 11497306 | 1194680 |
| Plasmid cp32-8 (Accession number NC_000953) | lipoprotein | 17205 | 17651 | + | 148 | 11497307 | 1194681 |
| Plasmid cp32-8 (Accession number NC_000953) | hypothetical protein BB_L29 | 17688 | 18761 | - | 357 | 11497308 | 1194682 |
| Plasmid cp32-8 (Accession number NC_000953) | hypothetical protein BB_L30 | 19091 | 20188 | + | 365 | 11497282 | 1194656 |
| Plasmid cp32-8 (Accession number NC_000953) | hypothetical protein BB_L31 | 20198 | 20764 | + | 188 | 11497283 | 1194657 |
| Plasmid cp32-8 (Accession number NC_000953) | plasmid partition protein, putative | 20740 | 21480 | + | 246 | 11497284 | 1194658 |

| Plasmid cp32-8 (Accession number NC_000953) | hypothetical protein BB_L33 | 2146 7 | 2155 9 | + | 30 | 11497309 | 1194683 |
|---|---|---|---|---|---|---|---|
| Plasmid cp32-8 (Accession number NC_000953) | hypothetical protein BB_L34 | 2154 0 | 2210 0 | + | 186 | 11497285 | 1194659 |
| Plasmid cp32-8 (Accession number NC_000953) | BdrO | 2211 3 | 2269 1 | + | 192 | 11497286 | 1194660 |
| Plasmid cp32-8 (Accession number NC_000953) | BppA | 2330 6 | 2469 1 | + | 461 | 11497287 | 1194661 |
| Plasmid cp32-8 (Accession number NC_000953) | BppB | 2474 4 | 2516 9 | + | 141 | 11497288 | 1194662 |
| Plasmid cp32-8 (Accession number NC_000953) | BppC | 2517 5 | 2595 1 | - | 258 | 11497289 | 1194663 |
| Plasmid cp32-8 (Accession number NC_000953) | ErpN | 2637 0 | 2690 3 | + | 177 | 11497290 | 1194664 |
| Plasmid cp32-8 (Accession number NC_000953) | ErpO | 2693 1 | 2806 7 | + | 378 | 11497291 | 1194665 |
| Plasmid cp32-8 (Accession number NC_000953) | hypothetical protein BB_L41 | 2820 9 | 2879 0 | + | 193 | 11497310 | 1194684 |
| Plasmid cp32-8 (Accession number NC_000953) | hypothetical protein BB_L42 | 2897 0 | 2953 6 | + | 188 | 11497311 | 1194685 |
| Plasmid cp32-8 (Accession number NC_000953) | hypothetical protein BB_L43 | 2953 3 | 3088 5 | + | 450 | 11497292 | 1194666 |
| Plasmid cp32-9 (Accession number NC_000954) | hypothetical protein BBN01 | 66 | 1292 | + | 408 | 11497348 | 1194725 |
| Plasmid cp32-9 (Accession number NC_000954) | hypothetical protein BBN02 | 1309 | 2001 | + | 230 | 11497349 | 1194726 |
| Plasmid cp32-9 (Accession number NC_000954) | hypothetical protein BBN03 | 2013 | 2579 | + | 188 | 11497350 | 1194727 |

| | | | | | | | |
|---|---|---|---|---|---|---|---|
| Plasmid cp32-9 (Accession number NC_000954) | hypothetical protein BBN04 | 2583 | 3350 | + | 255 | 11497351 | 1194728 |
| Plasmid cp32-9 (Accession number NC_000954) | hypothetical protein BBN07 | 4958 | 5413 | + | 151 | 11497326 | 1194699 |
| Plasmid cp32-9 (Accession number NC_000954) | hypothetical protein BBN08 | 5395 | 5796 | + | 133 | 11497327 | 1194700 |
| Plasmid cp32-9 (Accession number NC_000954) | hypothetical protein BBN09 | 5784 | 6173 | + | 129 | 11497328 | 1194701 |
| Plasmid cp32-9 (Accession number NC_000954) | hypothetical protein BBN10 | 6170 | 6736 | + | 188 | 11497329 | 1194702 |
| Plasmid cp32-9 (Accession number NC_000954) | hypothetical protein BBN11 | 6717 | 7805 | + | 362 | 11497352 | 1194729 |
| Plasmid cp32-9 (Accession number NC_000954) | hypothetical protein BBN12 | 7845 | 8273 | + | 142 | 11497353 | 1194730 |
| Plasmid cp32-9 (Accession number NC_000954) | hypothetical protein BBN14 | 8742 | 8978 | + | 78 | 11497354 | 1194731 |
| Plasmid cp32-9 (Accession number NC_000954) | hypothetical protein BBN15 | 8986 | 1026 0 | + | 424 | 11497355 | 1194732 |
| Plasmid cp32-9 (Accession number NC_000954) | hypothetical protein BBN17 | 1104 1 | 1199 1 | + | 316 | 11497356 | 1194733 |
| Plasmid cp32-9 (Accession number NC_000954) | hypothetical protein BBN19 | 1259 3 | 1292 5 | + | 110 | 11497330 | 1194703 |
| Plasmid cp32-9 (Accession number NC_000954) | hypothetical protein BBN20 | 1292 2 | 1379 7 | + | 291 | 11497331 | 1194704 |

| Plasmid cp32-9 (Accession number NC_000954) | pore-forming hemolysin | 1531 2 | 1551 5 | + | 67 | 11497332 | 1194707 |
|---|---|---|---|---|---|---|---|
| Plasmid cp32-9 (Accession number NC_000954) | hemolysin accessory protein | 1551 9 | 1586 6 | + | 115 | 11497333 | 1194708 |
| Plasmid cp32-9 (Accession number NC_000954) | hypothetical protein BBN25 | 1585 6 | 1619 1 | + | 111 | 11497334 | 1194709 |
| Plasmid cp32-9 (Accession number NC_000954) | outer surface protein, putative | 1617 8 | 1653 7 | + | 119 | 11497335 | 1194710 |
| Plasmid cp32-9 (Accession number NC_000954) | BdrR | 1661 2 | 1719 6 | + | 194 | 11497336 | 1194711 |
| Plasmid cp32-9 (Accession number NC_000954) | lipoprotein | 1730 2 | 1773 0 | + | 142 | 11497337 | 1194712 |
| Plasmid cp32-9 (Accession number NC_000954) | hypothetical protein BBN30 | 1916 4 | 2026 4 | + | 366 | 11497338 | 1194714 |
| Plasmid cp32-9 (Accession number NC_000954) | hypothetical protein BBN31 | 2027 5 | 2084 1 | + | 188 | 11497339 | 1194715 |
| Plasmid cp32-9 (Accession number NC_000954) | plasmid partition protein, putative | 2081 7 | 2157 2 | + | 251 | 11497340 | 1194716 |
| Plasmid cp32-9 (Accession number NC_000954) | hypothetical protein BBN33 | 2161 4 | 2217 4 | + | 186 | 11497341 | 1194717 |
| Plasmid cp32-9 (Accession number NC_000954) | BdrQ | 2218 4 | 2272 3 | + | 179 | 11497342 | 1194718 |
| Plasmid cp32-9 (Accession number NC_000954) | BppA | 2319 4 | 2451 9 | + | 441 | 11497343 | 1194719 |
| Plasmid cp32-9 (Accession number NC_000954) | BppB | 2457 2 | 2499 7 | + | 141 | 11497344 | 1194720 |
| Plasmid cp32-9 (Accession number NC_000954) | BppC | 2555 8 | 2577 9 | - | 73 | 21538987 | 1194721 |
| Plasmid cp32-9 (Accession number | ErpP | 2621 0 | 2677 0 | + | 186 | 11497345 | 1194722 |

| NC_000954) | | | | | | | |
|---|---|---|---|---|---|---|---|
| Plasmid cp32-9 (Accession number NC_000954) | ErpQ | 2679 8 | 2782 9 | + | 343 | 11497346 | 1194723 |
| Plasmid cp32-9 (Accession number NC_000954) | hypothetical protein BBN40 | 2788 1 | 2799 1 | - | 36 | 11497358 | 1194735 |
| Plasmid cp32-9 (Accession number NC_000954) | hypothetical protein BBN41 | 2798 4 | 2854 4 | + | 186 | 11497357 | 1194734 |
| Plasmid cp32-9 (Accession number NC_000954) | hypothetical protein BBN42 | 2873 6 | 2930 2 | + | 188 | 11497359 | 1194736 |
| Plasmid cp32-9 (Accession number NC_000954) | hypothetical protein BBN43 | 2929 9 | 3065 1 | + | 450 | 11497347 | 1194724 |
| Plasmid lp21 (Accession number NC_000955) | hypothetical protein BBU01 | 184 | 618 | + | 144 | 11497367 | 1194744 |
| Plasmid lp21 (Accession number NC_000955) | hypothetical protein BBU02 | 746 | 1114 | + | 122 | 11497368 | 1194745 |
| Plasmid lp21 (Accession number NC_000955) | hypothetical protein BBU03 | 1238 | 1357 | - | 39 | 11497369 | 1194746 |
| Plasmid lp21 (Accession number NC_000955) | hypothetical protein BBU04 | 1486 | 2628 | + | 380 | 11497361 | 1194737 |
| Plasmid lp21 (Accession number NC_000955) | plasmid partition protein, putative | 2868 | 3656 | + | 262 | 11497362 | 1194738 |
| Plasmid lp21 (Accession number NC_000955) | hypothetical protein BBU06 | 1463 3 | 1523 5 | + | 200 | 11497363 | 1194739 |
| Plasmid lp21 (Accession number NC_000955) | hypothetical protein BBU07 | 1534 9 | 1581 3 | + | 154 | 11497364 | 1194740 |

| Plasmid lp21 (Accession number NC_000955) | hypothetical protein BBU08 | 15791 | 16084 | + | 97 | 11497370 | 1194747 |
|---|---|---|---|---|---|---|---|
| Plasmid lp21 (Accession number NC_000955) | hypothetical protein BBU09 | 16228 | 16548 | - | 106 | 11497365 | 1194741 |
| Plasmid lp21 (Accession number NC_000955) | hypothetical protein BBU10 | 16603 | 16800 | + | 65 | 11497371 | 1194748 |
| Plasmid lp21 (Accession number NC_000955) | hypothetical protein BBU11 | 16886 | 17878 | + | 330 | 11497366 | 1194742 |
| Plasmid lp56 (Accession number NC_000956) | hypothetical protein BB_Q01 | 279 | 548 | + | 89 | 11497373 | 1194749 |
| Plasmid lp56 (Accession number NC_000956) | hypothetical protein BB_Q02 | 710 | 802 | + | 30 | 11497407 | 1194793 |
| Plasmid lp56 (Accession number NC_000956) | outer membrane protein, putative | 856 | 1407 | + | 183 | 11497374 | 1194750 |
| Plasmid lp56 (Accession number NC_000956) | antigen, P35, putative | 2744 | 3496 | + | 250 | 11497375 | 1194752 |
| Plasmid lp56 (Accession number NC_000956) | hypothetical protein BB_Q06 | 3623 | 4108 | + | 161 | 11497376 | 1194753 |
| Plasmid lp56 (Accession number NC_000956) | hypothetical protein BB_Q07 | 4249 | 4986 | - | 245 | 11497377 | 1194754 |
| Plasmid lp56 (Accession number NC_000956) | plasmid partition protein, putative | 5069 | 5830 | - | 253 | 11497378 | 1194755 |
| Plasmid lp56 (Accession number NC_000956) | hypothetical protein BB_Q09 | 5803 | 6339 | - | 178 | 11497379 | 1194756 |

| Plasmid lp56 (Accession number NC_000956) | hypothetical protein BB_Q10 | 6336 | 6674 | - | 112 | 11497408 | 1194794 |
|---|---|---|---|---|---|---|---|
| Plasmid lp56 (Accession number NC_000956) | hypothetical protein BB_Q12 | 6800 | 7411 | + | 203 | 11497409 | 1194795 |
| Plasmid lp56 (Accession number NC_000956) | hypothetical protein BB_Q13 | 7418 | 8392 | + | 324 | 11497380 | 1194757 |
| Plasmid lp56 (Accession number NC_000956) | hypothetical protein BB_Q14 | 8406 | 8867 | + | 153 | 11497381 | 1194758 |
| Plasmid lp56 (Accession number NC_000956) | hypothetical protein BB_Q15 | 8849 | 9250 | + | 133 | 11497382 | 1194759 |
| Plasmid lp56 (Accession number NC_000956) | hypothetical protein BB_Q17 | 9624 | 10190 | + | 188 | 11497383 | 1194761 |
| Plasmid lp56 (Accession number NC_000956) | hypothetical protein BB_Q18 | 10171 | 11283 | + | 370 | 11497410 | 1194796 |
| Plasmid lp56 (Accession number NC_000956) | hypothetical protein BB_Q19 | 11276 | 11725 | + | 149 | 11497411 | 1194797 |
| Plasmid lp56 (Accession number NC_000956) | hypothetical protein BB_Q20 | 11741 | 12196 | + | 151 | 11497412 | 1194798 |
| Plasmid lp56 (Accession number NC_000956) | hypothetical protein BB_Q21 | 12193 | 12429 | + | 78 | 11497413 | 1194799 |
| Plasmid lp56 (Accession number NC_000956) | hypothetical protein BB_Q22 | 12437 | 13732 | + | 431 | 11497414 | 1194800 |
| Plasmid lp56 (Accession number NC_000956) | hypothetical protein BB_Q23 | 13755 | 14438 | + | 227 | 11497415 | 1194801 |

| Plasmid lp56 (Accession number NC_000956) | hypothetical protein BB_Q24 | 1444 2 | 1539 2 | + | 316 | 11497416 | 1194802 |
|---|---|---|---|---|---|---|---|
| Plasmid lp56 (Accession number NC_000956) | hypothetical protein BB_Q25 | 1541 0 | 1596 4 | + | 184 | 11497417 | 1194803 |
| Plasmid lp56 (Accession number NC_000956) | hypothetical protein BB_Q26 | 1599 4 | 1632 6 | + | 110 | 11497384 | 1194762 |
| Plasmid lp56 (Accession number NC_000956) | hypothetical protein BB_Q27 | 1632 3 | 1719 8 | + | 291 | 11497385 | 1194763 |
| Plasmid lp56 (Accession number NC_000956) | hypothetical protein BB_Q28 | 1720 8 | 1781 3 | + | 201 | 11497386 | 1194764 |
| Plasmid lp56 (Accession number NC_000956) | hypothetical protein BB_Q29 | 1782 3 | 1863 8 | + | 271 | 11497387 | 1194765 |
| Plasmid lp56 (Accession number NC_000956) | pore-forming hemolysin | 1871 3 | 1891 6 | + | 67 | 11497388 | 1194766 |
| Plasmid lp56 (Accession number NC_000956) | hemolysin accessory protein | 1892 0 | 1926 7 | + | 115 | 11497389 | 1194767 |
| Plasmid lp56 (Accession number NC_000956) | hypothetical protein BB_Q32 | 1925 7 | 1959 2 | + | 111 | 11497390 | 1194768 |
| Plasmid lp56 (Accession number NC_000956) | hypothetical protein BB_Q33 | 1957 9 | 1993 8 | + | 119 | 11497391 | 1194769 |
| Plasmid lp56 (Accession number NC_000956) | BdrW | 2002 2 | 2073 8 | + | 238 | 11497392 | 1194770 |
| Plasmid lp56 (Accession number NC_000956) | MlpJ | 2084 4 | 2145 5 | + | 203 | 11497393 | 1194771 |
| Plasmid lp56 (Accession number NC_000956) | hypothetical protein BB_Q36 | 2142 4 | 2151 6 | + | 30 | 11497418 | 1194804 |

| | | | | | | | |
|---|---|---|---|---|---|---|---|
| Plasmid lp56 (Accession number NC_000956) | hypothetical protein BB_Q37 | 2153 2 | 2247 9 | - | 315 | 11497394 | 1194772 |
| Plasmid lp56 (Accession number NC_000956) | hypothetical protein BB_Q38 | 2286 9 | 2396 6 | + | 365 | 11497395 | 1194773 |
| Plasmid lp56 (Accession number NC_000956) | hypothetical protein BB_Q39 | 2397 6 | 2454 2 | + | 188 | 11497396 | 1194774 |
| Plasmid lp56 (Accession number NC_000956) | plasmid partition protein, putative | 2451 8 | 2527 3 | + | 251 | 11497397 | 1194775 |
| Plasmid lp56 (Accession number NC_000956) | hypothetical protein BB_Q41 | 2531 7 | 2587 7 | + | 186 | 11497398 | 1194776 |
| Plasmid lp56 (Accession number NC_000956) | BdrV | 2589 0 | 2642 6 | + | 178 | 11497399 | 1194777 |
| Plasmid lp56 (Accession number NC_000956) | BppA | 2687 9 | 2824 6 | + | 455 | 11497400 | 1194778 |
| Plasmid lp56 (Accession number NC_000956) | BppB | 2829 9 | 2872 4 | + | 141 | 11497401 | 1194779 |
| Plasmid lp56 (Accession number NC_000956) | BppC | 2873 0 | 2950 6 | - | 258 | 11497402 | 1194780 |
| Plasmid lp56 (Accession number NC_000956) | hypothetical protein BB_Q46 | 2955 9 | 2965 4 | + | 31 | 11497419 | 1194805 |
| Plasmid lp56 (Accession number NC_000956) | ErpX | 2993 7 | 3097 4 | + | 345 | 11497403 | 1194781 |
| Plasmid lp56 (Accession number NC_000956) | hypothetical protein BB_Q48 | 3111 7 | 3171 0 | + | 197 | 11497420 | 1194806 |
| Plasmid lp56 (Accession number NC_000956) | hypothetical protein BB_Q49 | 3189 2 | 3245 8 | + | 188 | 11497421 | 1194807 |
| Plasmid lp56 (Accession number NC_000956) | hypothetical protein BB_Q50 | 3245 5 | 3380 7 | + | 450 | 11497404 | 1194782 |

| | | | | | | | |
|---|---|---|---|---|---|---|---|
| Plasmid lp56 (Accession number NC_000956) | hypothetical protein BB_Q52 | 3511 5 | 3580 7 | + | 230 | 11497422 | 1194808 |
| Plasmid lp56 (Accession number NC_000956) | hypothetical protein BB_Q53 | 3581 9 | 3638 5 | + | 188 | 11497423 | 1194809 |
| Plasmid lp56 (Accession number NC_000956) | hypothetical protein BB_Q54 | 3638 9 | 3713 5 | + | 248 | 11497424 | 1194810 |
| Plasmid lp56 (Accession number NC_000956) | hypothetical protein BB_Q56 | 3766 9 | 3780 9 | - | 46 | 11497425 | 1194811 |
| Plasmid lp56 (Accession number NC_000956) | hypothetical protein BB_Q57 | 3781 6 | 3822 3 | - | 135 | 11497426 | 1194812 |
| Plasmid lp56 (Accession number NC_000956) | hypothetical protein BB_Q58 | 3841 6 | 3851 4 | - | 32 | 11497427 | 1194813 |
| Plasmid lp56 (Accession number NC_000956) | hypothetical protein BB_Q59 | 3856 5 | 3907 7 | - | 170 | 11497428 | 1194814 |
| Plasmid lp56 (Accession number NC_000956) | hypothetical protein BB_Q61 | 3935 7 | 3948 2 | - | 41 | 11497429 | 1194815 |
| Plasmid lp56 (Accession number NC_000956) | hypothetical protein BB_Q62 | 3953 1 | 3990 5 | + | 124 | 11497430 | 1194816 |
| Plasmid lp56 (Accession number NC_000956) | hypothetical protein BB_Q64 | 3995 9 | 4018 6 | - | 75 | 11497431 | 1194817 |
| Plasmid lp56 (Accession number NC_000956) | hypothetical protein BB_Q66 | 4031 7 | 4041 2 | + | 31 | 11497432 | 1194818 |
| Plasmid lp56 (Accession number NC_000956) | adenine specific DNA methyltransferase | 4043 6 | 4373 2 | - | 1098 | 11497405 | 1194785 |

| Plasmid lp56 (Accession number NC_000956) | hypothetical protein BB_Q68 | 4391 5 | 4423 2 | - | 105 | 11497433 | 1194819 |
|---|---|---|---|---|---|---|---|
| Plasmid lp56 (Accession number NC_000956) | hypothetical protein BB_Q70 | 4450 8 | 4461 2 | - | 34 | 11497434 | 1194820 |
| Plasmid lp56 (Accession number NC_000956) | hypothetical protein BB_Q72 | 4531 4 | 4543 0 | + | 38 | 11497435 | 1194821 |
| Plasmid lp56 (Accession number NC_000956) | hypothetical protein BB_Q76 | 4698 2 | 4708 0 | + | 32 | 11497436 | 1194822 |
| Plasmid lp56 (Accession number NC_000956) | hypothetical protein BB_Q78 | 4729 5 | 4739 6 | + | 33 | 11497437 | 1194823 |
| Plasmid lp56 (Accession number NC_000956) | hypothetical protein BB_Q82 | 4934 4 | 4953 8 | - | 64 | 11497438 | 1194824 |
| Plasmid lp56 (Accession number NC_000956) | hypothetical protein BB_Q83 | 4975 2 | 4986 8 | - | 38 | 11497439 | 1194825 |
| Plasmid lp56 (Accession number NC_000956) | hypothetical protein BB_Q84 | 5039 5 | 5055 0 | - | 51 | 11497440 | 1194826 |
| Plasmid lp56 (Accession number NC_000956) | hypothetical protein BB_Q85 | 5117 2 | 5152 8 | - | 118 | 11497406 | 1194792 |
| Plasmid lp56 (Accession number NC_000956) | hypothetical protein BB_Q86 | 5171 9 | 5182 3 | - | 34 | 11497441 | 1194827 |
| Plasmid lp56 (Accession number NC_000956) | hypothetical protein BB_Q87 | 5191 8 | 5206 7 | - | 49 | 11497442 | 1194828 |
| Plasmid lp56 (Accession number NC_000956) | hypothetical protein BB_Q88 | 5213 5 | 5260 8 | - | 157 | 11497443 | 1194829 |

| Plasmid lp56 (Accession number NC_000956) | hypothetical protein BB_Q89 | 52532 | 52726 | - | 64 | 11497444 | 1194830 |
|---|---|---|---|---|---|---|---|
| Plasmid lp5 (Accession number NC_000957) | hypothetical protein BB_T01 | 195 | 638 | + | 147 | 11497450 | 1194835 |
| Plasmid lp5 (Accession number NC_000957) | hypothetical protein BB_T02 | 744 | 1097 | + | 117 | 11497446 | 1194831 |
| Plasmid lp5 (Accession number NC_000957) | hypothetical protein BB_T03 | 1208 | 1576 | + | 122 | 11497451 | 1194836 |
| Plasmid lp5 (Accession number NC_000957) | hypothetical protein BB_T04 | 2148 | 3254 | + | 368 | 11497447 | 1194832 |
| Plasmid lp5 (Accession number NC_000957) | hypothetical protein BB_T06 | 3340 | 4332 | + | 330 | 11497448 | 1194833 |
| Plasmid lp5 (Accession number NC_000957) | outer membrane protein | 4388 | 4819 | + | 143 | 11497449 | 1194834 |
| Plasmid lp17 (Accession number NC_001849) | hypothetical protein BBD01 | 332 | 805 | + | 157 | 11496586 | 1194013 |
| Plasmid lp17 (Accession number NC_001849) | hypothetical protein BBD02 | 873 | 1022 | + | 49 | 11496599 | 1194028 |
| Plasmid lp17 (Accession number NC_001849) | hypothetical protein BBD03 | 1117 | 1221 | + | 34 | 11496598 | 1194027 |
| Plasmid lp17 (Accession number NC_001849) | hypothetical protein BBD04 | 1412 | 1768 | + | 118 | 11496585 | 1194012 |
| Plasmid lp17 (Accession number NC_001849) | hypothetical protein BBD05 | 2389 | 2544 | + | 51 | 11496584 | 1194011 |

| | | | | | | | |
|---|---|---|---|---|---|---|---|
| Plasmid lp17 (Accession number NC_001849) | hypothetical protein BBD06 | 3143 | 3607 | + | 154 | 11496596 | 1194024 |
| Plasmid lp17 (Accession number NC_001849) | hypothetical protein BBD07 | 4257 | 4373 | - | 38 | 11496606 | 1194035 |
| Plasmid lp17 (Accession number NC_001849) | hypothetical protein BBD08 | 4707 | 4805 | + | 32 | 11496605 | 1194034 |
| Plasmid lp17 (Accession number NC_001849) | hypothetical protein BBD09 | 5058 | 5741 | + | 227 | 11496595 | 1194023 |
| Plasmid lp17 (Accession number NC_001849) | hypothetical protein BBD10 | 5876 | 6454 | - | 192 | 11496594 | 1194022 |
| Plasmid lp17 (Accession number NC_001849) | hypothetical protein BBD11 | 6681 | 7634 | + | 317 | 11496593 | 1194021 |
| Plasmid lp17 (Accession number NC_001849) | hypothetical protein BBD12 | 7621 | 7752 | - | 43 | 11496604 | 1194033 |
| Plasmid lp17 (Accession number NC_001849) | hypothetical protein BBD13 | 7787 | 8113 | + | 108 | 11496592 | 1194020 |
| Plasmid lp17 (Accession number NC_001849) | hypothetical protein BBD14 | 8269 | 9381 | + | 370 | 11496591 | 1194019 |
| Plasmid lp17 (Accession number NC_001849) | hypothetical protein BBD15 | 9593 | 10015 | - | 140 | 11496590 | 1194018 |
| Plasmid lp17 (Accession number NC_001849) | hypothetical protein BBD16 | 10425 | 10520 | - | 31 | 11496603 | 1194032 |
| Plasmid lp17 (Accession number NC_001849) | hypothetical protein BBD17 | 10591 | 10686 | + | 31 | 11496601 | 1194030 |

| Plasmid lp17 (Accession number NC_001849) | hypothetical protein BBD18 | 1098 6 | 1164 8 | - | 220 | 11496588 | 1194015 |
|---|---|---|---|---|---|---|---|
| Plasmid lp17 (Accession number NC_001849) | hypothetical protein BBD19 | 1205 7 | 1217 0 | + | 37 | 11496600 | 1194029 |
| Plasmid lp17 (Accession number NC_001849) | hypothetical protein BBD21 | 1334 1 | 1408 1 | + | 246 | 11496587 | 1194014 |
| Plasmid lp17 (Accession number NC_001849) | hypothetical protein BBD22 | 1407 2 | 1434 1 | + | 89 | 11496589 | 1194017 |
| Plasmid lp17 (Accession number NC_001849) | hypothetical protein BBD24 | 1589 1 | 1612 1 | - | 76 | 11496597 | 1194026 |
| Plasmid lp17 (Accession number NC_001849) | hypothetical protein BBD25 | 1621 2 | 1637 0 | + | 52 | 11496602 | 1194031 |
| Plasmid lp25 (Accession number NC_001850) | hypothetical protein BBE01 | 154 | 255 | - | 33 | 11496632 | 1194063 |
| Plasmid lp25 (Accession number NC_001850) | hypothetical protein BBE02 | 323 | 4156 | - | 1277 | 11496616 | 1194047 |
| Plasmid lp25 (Accession number NC_001850) | hypothetical protein BBE03 | 4419 | 4613 | - | 64 | 11496617 | 1194048 |
| Plasmid lp25 (Accession number NC_001850) | hypothetical protein BBE04 | 4719 | 4859 | + | 46 | 11496618 | 1194049 |
| Plasmid lp25 (Accession number NC_001850) | hypothetical protein BBE05 | 5377 | 5529 | + | 50 | 11496615 | 1194046 |
| Plasmid lp25 (Accession number NC_001850) | hypothetical protein BBE06 | 5757 | 5906 | + | 49 | 11496625 | 1194056 |

250

| | | | | | | | |
|---|---|---|---|---|---|---|---|
| Plasmid lp25 (Accession number NC_001850) | hypothetical protein BBE07 | 6182 | 6355 | - | 57 | 11496626 | 1194057 |
| Plasmid lp25 (Accession number NC_001850) | hypothetical protein BBE08 | 6555 | 6701 | - | 48 | 11496627 | 1194058 |
| Plasmid lp25 (Accession number NC_001850) | protein p23 | 6898 | 7761 | + | 287 | 11496612 | 1194043 |
| Plasmid lp25 (Accession number NC_001850) | hypothetical protein BBE10 | 7874 | 7972 | - | 32 | 11496628 | 1194059 |
| Plasmid lp25 (Accession number NC_001850) | hypothetical protein BBE11 | 8312 | 8446 | - | 44 | 11496629 | 1194060 |
| Plasmid lp25 (Accession number NC_001850) | hypothetical protein BBE12 | 8521 | 8646 | - | 41 | 11496630 | 1194061 |
| Plasmid lp25 (Accession number NC_001850) | hypothetical protein BBE13 | 8863 | 8958 | + | 31 | 11496631 | 1194062 |
| Plasmid lp25 (Accession number NC_001850) | hypothetical protein BBE14 | 9163 | 9378 | + | 71 | 11496633 | 1194064 |
| Plasmid lp25 (Accession number NC_001850) | hypothetical protein BBE15 | 9353 | 9490 | - | 45 | 11496634 | 1194065 |
| Plasmid lp25 (Accession number NC_001850) | hypothetical protein BBE16 | 9567 | 10187 | - | 206 | 11496635 | 1194066 |
| Plasmid lp25 (Accession number NC_001850) | hypothetical protein BBE17 | 10200 | 10709 | - | 169 | 11496636 | 1194067 |
| Plasmid lp25 (Accession number NC_001850) | hypothetical protein BBE18 | 11498 | 12079 | - | 193 | 11496613 | 1194044 |
| Plasmid lp25 (Accession number NC_001850) | hypothetical protein BBE19 | 12096 | 12854 | - | 252 | 11496614 | 1194045 |

EP 2 254 592 B1

| Plasmid lp25 (Accession number NC_001850) | hypothetical protein BBE20 | 1283 0 | 1339 3 | - | 187 | 11496608 | 1194036 |
|---|---|---|---|---|---|---|---|
| Plasmid lp25 (Accession number NC_001850) | hypothetical protein BBE21 | 1340 3 | 1453 0 | - | 375 | 11496609 | 1194037 |
| Plasmid lp25 (Accession number NC_001850) | pyrazinamidase/ni cotinamidase (pncA) | 1504 2 | 1557 8 | - | 178 | 11496610 | 1194038 |
| Plasmid lp25 (Accession number NC_001850) | hypothetical protein BBE23 | 1597 3 | 1615 8 | + | 61 | 11496620 | 1194051 |
| Plasmid lp25 (Accession number NC_001850) | hypothetical protein BBE25 | 1850 2 | 1860 6 | - | 34 | 11496621 | 1194052 |
| Plasmid lp25 (Accession number NC_001850) | hypothetical protein BBE26 | 1858 6 | 1871 4 | + | 42 | 11496619 | 1194050 |
| Plasmid lp25 (Accession number NC_001850) | hypothetical protein BBE27 | 1905 5 | 1919 8 | + | 47 | 11496622 | 1194053 |
| Plasmid lp25 (Accession number NC_001850) | hypothetical protein BBE28 | 1933 7 | 1948 9 | - | 50 | 11496623 | 1194054 |
| Plasmid lp25 (Accession number NC_001850) | hypothetical protein BBE30 | 2155 8 | 2170 4 | + | 48 | 11496624 | 1194055 |
| Plasmid lp25 (Accession number NC_001850) | antigen, P35, putative | 2194 6 | 2267 7 | - | 243 | 11496611 | 1194040 |
| Plasmid lp28-1 (Accession number NC_001851) | erpD protein, putative | 467 | 1465 | + | 332 | 11496643 | 1194073 |
| Plasmid lp28-1 (Accession number NC_001851) | hypothetical protein BBF02 | 1720 | 2076 | + | 118 | 11496642 | 1194072 |

| | | | | | | | |
|---|---|---|---|---|---|---|---|
| Plasmid lp28-1 (Accession number NC_001851) | hypothetical protein BBF03 | 2098 | 2619 | - | 173 | 11496641 | 1194071 |
| Plasmid lp28-1 (Accession number NC_001851) | hypothetical protein BBF04 | 2658 | 2807 | + | 49 | 11496658 | 1194094 |
| Plasmid lp28-1 (Accession number NC_001851) | hypothetical protein BBF05 | 2777 | 3076 | + | 99 | 11496657 | 1194093 |
| Plasmid lp28-1 (Accession number NC_001851) | hypothetical protein BBF06 | 3201 | 3380 | + | 59 | 11496640 | 1194070 |
| Plasmid lp28-1 (Accession number NC_001851) | hypothetical protein BBF07 | 3410 | 3529 | - | 39 | 11496656 | 1194092 |
| Plasmid lp28-1 (Accession number NC_001851) | hypothetical protein BBF08 | 3682 | 3849 | - | 55 | 11496655 | 1194091 |
| Plasmid lp28-1 (Accession number NC_001851) | hypothetical protein BBF09 | 4027 | 4182 | + | 51 | 11496654 | 1194090 |
| Plasmid lp28-1 (Accession number NC_001851) | hypothetical protein BBF10 | 4488 | 4985 | + | 165 | 11496653 | 1194089 |
| Plasmid lp28-1 (Accession number NC_001851) | hypothetical protein BBF11 | 5435 | 5542 | + | 35 | 11496652 | 1194088 |
| Plasmid lp28-1 (Accession number NC_001851) | hypothetical protein BBF13 | 6632 | 7381 | - | 249 | 11496639 | 1194069 |
| Plasmid lp28-1 (Accession number NC_001851) | hypothetical protein BBF14 | 7354 | 7911 | - | 185 | 11496638 | 1194068 |
| Plasmid lp28-1 (Accession number NC_001851) | hypothetical protein BBF15 | 8239 | 8370 | + | 43 | 11496651 | 1194087 |

| | | | | | | | |
|---|---|---|---|---|---|---|---|
| Plasmid lp28-1 (Accession number NC_001851) | hypothetical protein BBF16 | 8389 | 8574 | + | 61 | 11496650 | 1194086 |
| Plasmid lp28-1 (Accession number NC_001851) | hypothetical protein BBF17 | 8772 | 9029 | + | 85 | 11496660 | 1194096 |
| Plasmid lp28-1 (Accession number NC_001851) | hypothetical protein BBF20 | 1069 8 | 1099 1 | - | 97 | 11496649 | 1194079 |
| Plasmid lp28-1 (Accession number NC_001851) | hypothetical protein BBF21 | 1144 6 | 1155 0 | - | 34 | 11496659 | 1194095 |
| Plasmid lp28-1 (Accession number NC_001851) | protein p23, putative | 1179 1 | 1201 8 | - | 75 | 11496648 | 1194078 |
| Plasmid lp28-1 (Accession number NC_001851) | hypothetical protein BBF23 | 1244 1 | 1299 2 | - | 183 | 11496647 | 1194077 |
| Plasmid lp28-1 (Accession number NC_001851) | hypothetical protein BBF24 | 1302 9 | 1379 3 | - | 254 | 11496646 | 1194076 |
| Plasmid lp28-1 (Accession number NC_001851) | hypothetical protein BBF25 | 1376 9 | 1432 9 | - | 186 | 11496645 | 1194075 |
| Plasmid lp28-1 (Accession number NC_001851) | hypothetical protein BBF26 | 1435 1 | 1545 1 | - | 366 | 11496644 | 1194074 |
| Plasmid lp28-1 (Accession number NC_001851) | hypothetical protein BBF27 | 1575 5 | 1592 5 | - | 56 | 11496663 | 1194099 |
| Plasmid lp28-1 (Accession number NC_001851) | hypothetical protein BBF28 | 1599 8 | 1612 9 | - | 43 | 11496662 | 1194098 |
| Plasmid lp28-1 (Accession number NC_001851) | hypothetical protein BBF30 | 1716 7 | 1741 5 | - | 82 | 11496661 | 1194097 |

| | | | | | | | |
|---|---|---|---|---|---|---|---|
| Plasmid lp28-2 (Accession number NC_001852) | hypothetical protein BBG01 | 116 | 1009 | + | 297 | 11496686 | 1194123 |
| Plasmid lp28-2 (Accession number NC_001852) | hypothetical protein BBG02 | 1047 | 1928 | + | 293 | 11496666 | 1194101 |
| Plasmid lp28-2 (Accession number NC_001852) | hypothetical protein BBG04 | 2750 | 2857 | - | 35 | 11496687 | 1194124 |
| Plasmid lp28-2 (Accession number NC_001852) | hypothetical protein BBG06 | 4208 | 5368 | + | 386 | 11496667 | 1194102 |
| Plasmid lp28-2 (Accession number NC_001852) | hypothetical protein BBG07 | 5378 | 5950 | + | 190 | 11496668 | 1194103 |
| Plasmid lp28-2 (Accession number NC_001852) | stage 0 sporulation protein J (spoOJ) | 5911 | 6678 | + | 255 | 11496669 | 1194104 |
| Plasmid lp28-2 (Accession number NC_001852) | hypothetical protein BBG09 | 6737 | 7288 | + | 183 | 11496670 | 1194105 |
| Plasmid lp28-2 (Accession number NC_001852) | hypothetical protein BBG10 | 7483 | 1077 9 | - | 1098 | 11496688 | 1194125 |
| Plasmid lp28-2 (Accession number NC_001852) | hypothetical protein BBG11 | 1077 6 | 1101 5 | - | 79 | 11496689 | 1194126 |
| Plasmid lp28-2 (Accession number NC_001852) | hypothetical protein BBG12 | 1106 3 | 1149 1 | - | 142 | 11496690 | 1194127 |
| Plasmid lp28-2 (Accession number NC_001852) | hypothetical protein BBG13 | 1150 1 | 1235 5 | - | 284 | 11496691 | 1194128 |
| Plasmid lp28-2 (Accession number NC_001852) | hypothetical protein BBG14 | 1230 9 | 1275 2 | - | 147 | 11496676 | 1194113 |

| Plasmid lp28-2 (Accession number NC_001852) | hypothetical protein BBG15 | 1268 1 | 1316 9 | + | 162 | 11496692 | 1194129 |
|---|---|---|---|---|---|---|---|
| Plasmid lp28-2 (Accession number NC_001852) | hypothetical protein BBG16 | 1315 7 | 1349 5 | - | 112 | 11496677 | 1194114 |
| Plasmid lp28-2 (Accession number NC_001852) | hypothetical protein BBG17 | 1350 8 | 1434 1 | - | 277 | 11496678 | 1194115 |
| Plasmid lp28-2 (Accession number NC_001852) | hypothetical protein BBG18 | 1437 6 | 1488 5 | - | 169 | 11496679 | 1194116 |
| Plasmid lp28-2 (Accession number NC_001852) | hypothetical protein BBG19 | 1488 6 | 1543 1 | - | 181 | 11496680 | 1194117 |
| Plasmid lp28-2 (Accession number NC_001852) | hypothetical protein BBG20 | 1545 7 | 1648 2 | - | 341 | 11496681 | 1194118 |
| Plasmid lp28-2 (Accession number NC_001852) | hypothetical protein BBG21 | 1649 4 | 1768 4 | - | 396 | 11496682 | 1194119 |
| Plasmid lp28-2 (Accession number NC_001852) | hypothetical protein BBG22 | 1803 0 | 1882 7 | - | 265 | 11496683 | 1194120 |
| Plasmid lp28-2 (Accession number NC_001852) | hypothetical protein BBG23 | 1883 7 | 1961 9 | - | 260 | 11496684 | 1194121 |
| Plasmid lp28-2 (Accession number NC_001852) | hypothetical protein BBG24 | 1962 0 | 2231 0 | - | 896 | 11496685 | 1194122 |
| Plasmid lp28-2 (Accession number NC_001852) | hypothetical protein BBG25 | 2227 3 | 2265 9 | - | 128 | 11496665 | 1194100 |
| Plasmid lp28-2 (Accession number NC_001852) | hypothetical protein BBG26 | 2265 9 | 2303 3 | - | 124 | 11496693 | 1194130 |

| Plasmid lp28-2 (Accession number NC_001852) | hypothetical protein BBG27 | 2303 3 | 2372 5 | - | 230 | 11496671 | 1194106 |
|---|---|---|---|---|---|---|---|
| Plasmid lp28-2 (Accession number NC_001852) | hypothetical protein BBG28 | 2372 2 | 2410 8 | - | 128 | 11496694 | 1194131 |
| Plasmid lp28-2 (Accession number NC_001852) | hypothetical protein BBG29 | 2448 9 | 2595 5 | + | 488 | 11496672 | 1194107 |
| Plasmid lp28-2 (Accession number NC_001852) | hypothetical protein BBG30 | 2596 2 | 2639 0 | + | 142 | 11496695 | 1194132 |
| Plasmid lp28-2 (Accession number NC_001852) | hypothetical protein BBG31 | 2656 7 | 2708 5 | + | 172 | 11496673 | 1194108 |
| Plasmid lp28-2 (Accession number NC_001852) | replicative DNA helicase, putative | 2711 3 | 2794 0 | + | 275 | 11496674 | 1194109 |
| Plasmid lp28-2 (Accession number NC_001852) | hypothetical protein BBG33 | 2803 1 | 2883 1 | + | 266 | 11496675 | 1194110 |
| Plasmid lp28-2 (Accession number NC_001852) | hypothetical protein BBG34 | 2885 4 | 2961 8 | - | 254 | 11496696 | 1194133 |
| Plasmid lp28-3 (Accession number NC_001853) | hypothetical protein BBH01 | 273 | 467 | + | 64 | 11496702 | 1194139 |
| Plasmid lp28-3 (Accession number NC_001853) | hypothetical protein BBH02 | 391 | 858 | + | 155 | 11496704 | 1194141 |
| Plasmid lp28-3 (Accession number NC_001853) | hypothetical protein BBH03 | 926 | 1075 | + | 49 | 11496709 | 1194148 |
| Plasmid lp28-3 (Accession number NC_001853) | hypothetical protein BBH04 | 1045 | 1368 | + | 107 | 11496698 | 1194134 |

| Plasmid lp28-3 (Accession number NC_001853) | hypothetical protein BBH05 | 1498 | 1680 | + | 60 | 11496703 | 1194140 |
|---|---|---|---|---|---|---|---|
| Plasmid lp28-3 (Accession number NC_001853) | hypothetical protein BBH06 | 2260 | 2970 | - | 236 | 11496710 | 1194149 |
| Plasmid lp28-3 (Accession number NC_001853) | hypothetical protein BBH08 | 3590 | 3730 | - | 46 | 11496711 | 1194150 |
| Plasmid lp28-3 (Accession number NC_001853) | hypothetical protein BBH09 | 3892 | 7728 | - | 1278 | 11496712 | 1194151 |
| Plasmid lp28-3 (Accession number NC_001853) | hypothetical protein BBH10 | 8000 | 8203 | - | 67 | 11496713 | 1194152 |
| Plasmid lp28-3 (Accession number NC_001853) | hypothetical protein BBH11 | 8701 | 8796 | - | 31 | 11496714 | 1194153 |
| Plasmid lp28-3 (Accession number NC_001853) | hypothetical protein BBH12 | 9455 | 9592 | + | 45 | 11496715 | 1194154 |
| Plasmid lp28-3 (Accession number NC_001853) | hypothetical protein BBH13 | 9848 | 1051 6 | - | 222 | 11496699 | 1194136 |
| Plasmid lp28-3 (Accession number NC_001853) | hypothetical protein BBH14 | 1081 8 | 1093 4 | - | 38 | 11496716 | 1194155 |
| Plasmid lp28-3 (Accession number NC_001853) | hypothetical protein BBH15 | 1091 0 | 1100 5 | - | 31 | 11496717 | 1194156 |
| Plasmid lp28-3 (Accession number NC_001853) | hypothetical protein BBH16 | 1106 8 | 1119 0 | + | 40 | 11496732 | 1194171 |
| Plasmid lp28-3 (Accession number NC_001853) | hypothetical protein BBH17 | 1183 7 | 1202 8 | + | 63 | 11496733 | 1194172 |

| Plasmid lp28-3 (Accession number NC_001853) | hypothetical protein BBH18 | 1210 5 | 1322 0 | + | 371 | 11496734 | 1194173 |
|---|---|---|---|---|---|---|---|
| Plasmid lp28-3 (Accession number NC_001853) | hypothetical protein BBH19 | 1359 0 | 1371 2 | + | 40 | 11496718 | 1194157 |
| Plasmid lp28-3 (Accession number NC_001853) | hypothetical protein BBH22 | 1476 3 | 1487 0 | - | 35 | 11496719 | 1194158 |
| Plasmid lp28-3 (Accession number NC_001853) | hypothetical protein BBH23 | 1505 1 | 1516 1 | + | 36 | 11496720 | 1194159 |
| Plasmid lp28-3 (Accession number NC_001853) | hypothetical protein BBH24 | 1513 6 | 1534 5 | + | 69 | 11496721 | 1194160 |
| Plasmid lp28-3 (Accession number NC_001853) | hypothetical protein BBH25 | 1556 5 | 1581 0 | - | 81 | 11496722 | 1194161 |
| Plasmid lp28-3 (Accession number NC_001853) | hypothetical protein BBH26 | 1651 9 | 1741 5 | + | 298 | 11496723 | 1194162 |
| Plasmid lp28-3 (Accession number NC_001853) | hypothetical protein BBH27 | 1740 8 | 1797 4 | + | 188 | 11496705 | 1194142 |
| Plasmid lp28-3 (Accession number NC_001853) | hypothetical protein BBH28 | 1794 7 | 1870 2 | + | 251 | 11496706 | 1194143 |
| Plasmid lp28-3 (Accession number NC_001853) | hypothetical protein BBH29 | 1879 8 | 1942 7 | + | 209 | 11496707 | 1194144 |
| Plasmid lp28-3 (Accession number NC_001853) | hypothetical protein BBH31 | 2099 7 | 2110 7 | + | 36 | 11496725 | 1194164 |
| Plasmid lp28-3 (Accession number NC_001853) | antigen, P35, putative | 2147 0 | 2221 9 | + | 249 | 11496708 | 1194146 |

| | | | | | | | |
|---|---|---|---|---|---|---|---|
| Plasmid lp28-3 (Accession number NC_001853) | hypothetical protein BBH33 | 2267 8 | 2295 3 | + | 91 | 11496726 | 1194165 |
| Plasmid lp28-3 (Accession number NC_001853) | hypothetical protein BBH34 | 2318 9 | 2338 3 | - | 64 | 11496727 | 1194166 |
| Plasmid lp28-3 (Accession number NC_001853) | hypothetical protein BBH35 | 2344 7 | 2356 3 | + | 38 | 11496728 | 1194167 |
| Plasmid lp28-3 (Accession number NC_001853) | hypothetical protein BBH36 | 2402 8 | 2418 0 | - | 50 | 11496729 | 1194168 |
| Plasmid lp28-3 (Accession number NC_001853) | hypothetical protein BBH37 | 2543 3 | 2637 1 | - | 312 | 11496730 | 1194169 |
| Plasmid lp28-3 (Accession number NC_001853) | hypothetical protein BBH38 | 2649 5 | 2661 4 | - | 39 | 11496731 | 1194170 |
| Plasmid lp28-3 (Accession number NC_001853) | hypothetical protein BBH39 | 2675 4 | 2685 8 | + | 34 | 11496724 | 1194163 |
| Plasmid lp28-3 (Accession number NC_001853) | transposase-like protein, putative | 2697 8 | 2744 5 | - | 155 | 11496700 | 1194137 |
| Plasmid lp28-3 (Accession number NC_001853) | hypothetical protein BBH41 | 2762 5 | 2819 7 | - | 190 | 11496701 | 1194138 |
| Plasmid lp28-4 (Accession number NC_001854) | hypothetical protein BBI01 | 174 | 608 | + | 144 | 11496764 | 1194202 |
| Plasmid lp28-4 (Accession number NC_001854) | hypothetical protein BBI02 | 736 | 1104 | + | 122 | 11496742 | 1194180 |
| Plasmid lp28-4 (Accession number NC_001854) | hypothetical protein BBI03 | 1847 | 1972 | - | 41 | 11496765 | 1194203 |

| Plasmid lp28-4 (Accession number NC_001854) | hypothetical protein BBI04 | 2124 | 2219 | - | 31 | 11496766 | 1194204 |
|---|---|---|---|---|---|---|---|
| Plasmid lp28-4 (Accession number NC_001854) | hypothetical protein BBI05 | 2188 | 2310 | - | 40 | 11496767 | 1194205 |
| Plasmid lp28-4 (Accession number NC_001854) | pfs protein (pfs) | 2536 | 3351 | + | 271 | 11496743 | 1194181 |
| Plasmid lp28-4 (Accession number NC_001854) | hypothetical protein BBI07 | 3343 | 3441 | - | 32 | 11496768 | 1194206 |
| Plasmid lp28-4 (Accession number NC_001854) | hypothetical protein BBI08 | 3576 | 3755 | + | 59 | 11496749 | 1194187 |
| Plasmid lp28-4 (Accession number NC_001854) | hypothetical protein BBI09 | 3745 | 3882 | + | 45 | 11496750 | 1194188 |
| Plasmid lp28-4 (Accession number NC_001854) | hypothetical protein BBI10 | 3911 | 4315 | + | 134 | 11496751 | 1194189 |
| Plasmid lp28-4 (Accession number NC_001854) | hypothetical protein BBI11 | 4623 | 4721 | - | 32 | 11496752 | 1194190 |
| Plasmid lp28-4 (Accession number NC_001854) | hypothetical protein BBI12 | 5128 | 5346 | + | 72 | 11496753 | 1194191 |
| Plasmid lp28-4 (Accession number NC_001854) | hypothetical protein BBI13 | 5609 | 5707 | + | 32 | 11496736 | 1194174 |
| Plasmid lp28-4 (Accession number NC_001854) | hypothetical protein BBI14 | 6159 | 6272 | + | 37 | 11496754 | 1194192 |
| Plasmid lp28-4 (Accession number NC_001854) | hypothetical protein BBI15 | 6603 | 6833 | + | 76 | 11496755 | 1194193 |

| Plasmid lp28-4 (Accession number NC_001854) | hypothetical protein BBI16 | 7183 | 8538 | + | 451 | 11496756 | 1194194 |
|---|---|---|---|---|---|---|---|
| Plasmid lp28-4 (Accession number NC_001854) | hypothetical protein BBI17 | 8821 | 8967 | - | 48 | 11496757 | 1194195 |
| Plasmid lp28-4 (Accession number NC_001854) | hypothetical protein BBI18 | 1049 5 | 1064 7 | - | 50 | 11496758 | 1194196 |
| Plasmid lp28-4 (Accession number NC_001854) | hypothetical protein BBI19 | 1074 9 | 1192 7 | + | 392 | 11496737 | 1194175 |
| Plasmid lp28-4 (Accession number NC_001854) | hypothetical protein BBI20 | 1192 4 | 1247 8 | + | 184 | 11496738 | 1194176 |
| Plasmid lp28-4 (Accession number NC_001854) | hypothetical protein BBI21 | 1245 4 | 1320 6 | + | 250 | 11496739 | 1194177 |
| Plasmid lp28-4 (Accession number NC_001854) | conserved hypothetical protein, | 1326 5 | 1383 7 | + | 190 | 11496740 | 1194178 |
| Plasmid lp28-4 (Accession number NC_001854) | hypothetical protein BBI23 | 1398 9 | 1409 3 | + | 34 | 11496759 | 1194197 |
| Plasmid lp28-4 (Accession number NC_001854) | hypothetical protein BBI24 | 1433 4 | 1444 1 | + | 35 | 11496760 | 1194198 |
| Plasmid lp28-4 (Accession number NC_001854) | hypothetical protein BBI25 | 1521 1 | 1534 2 | + | 43 | 11496769 | 1194207 |
| Plasmid lp28-4 (Accession number NC_001854) | multidrug-efflux transporter | 1535 2 | 1653 0 | + | 392 | 11496744 | 1194182 |
| Plasmid lp28-4 (Accession number NC_001854) | hypothetical protein BBI27 | 1709 3 | 1727 2 | - | 59 | 11496770 | 1194208 |

| | | | | | | | |
|---|---|---|---|---|---|---|---|
| Plasmid lp28-4 (Accession number NC_001854) | hypothetical protein BBI28 | 1730 2 | 1787 4 | - | 190 | 11496771 | 1194209 |
| Plasmid lp28-4 (Accession number NC_001854) | hypothetical protein BBI29 | 1851 8 | 1918 3 | - | 221 | 11496772 | 1194210 |
| Plasmid lp28-4 (Accession number NC_001854) | hypothetical protein BBI30 | 1940 3 | 1951 0 | + | 35 | 11496773 | 1194211 |
| Plasmid lp28-4 (Accession number NC_001854) | hypothetical protein BBI31 | 1961 5 | 2012 7 | - | 170 | 11496747 | 1194185 |
| Plasmid lp28-4 (Accession number NC_001854) | hypothetical protein BBI32 | 2027 0 | 2047 9 | - | 69 | 11496774 | 1194212 |
| Plasmid lp28-4 (Accession number NC_001854) | hypothetical protein BBI33 | 2048 2 | 2059 2 | + | 36 | 11496775 | 1194213 |
| Plasmid lp28-4 (Accession number NC_001854) | hypothetical protein BBI34 | 2077 1 | 2156 2 | - | 263 | 11496776 | 1194214 |
| Plasmid lp28-4 (Accession number NC_001854) | hypothetical protein BBI35 | 2199 2 | 2209 3 | + | 33 | 11496777 | 1194215 |
| Plasmid lp28-4 (Accession number NC_001854) | antigen, P35, putative | 2209 5 | 2293 1 | - | 278 | 11496748 | 1194186 |
| Plasmid lp28-4 (Accession number NC_001854) | hypothetical protein BBI37 | 2305 6 | 2315 7 | + | 33 | 11496778 | 1194216 |
| Plasmid lp28-4 (Accession number NC_001854) | hypothetical protein BBI38 | 2315 9 | 2399 5 | - | 278 | 11496761 | 1194199 |
| Plasmid lp28-4 (Accession number NC_001854) | hypothetical protein BBI39 | 2422 3 | 2508 9 | - | 288 | 11496762 | 1194200 |

| Plasmid lp28-4 (Accession number NC_001854) | hypothetical protein BBI40 | 2551 8 | 2580 5 | + | 95 | 11496741 | 1194179 |
|---|---|---|---|---|---|---|---|
| Plasmid lp28-4 (Accession number NC_001854) | hypothetical protein BBI41 | 2579 4 | 2603 6 | - | 80 | 11496763 | 1194201 |
| Plasmid lp28-4 (Accession number NC_001854) | hypothetical protein BBI42 | 2636 0 | 2691 4 | + | 184 | 11496745 | 1194183 |
| Plasmid lp28-4 (Accession number NC_001854) | hypothetical protein BBI43 | 2688 1 | 2706 9 | - | 62 | 11496746 | 1194184 |
| Plasmid lp36 (Accession number NC_001855) | hypothetical protein BBK01 | 188 | 1081 | + | 297 | 11496795 | 1194235 |
| Plasmid lp36 (Accession number NC_001855) | hypothetical protein BBK02 | 1210 | 2478 | - | 422 | 11496796 | 1194236 |
| Plasmid lp36 (Accession number NC_001855) | hypothetical protein BBK03 | 2818 | 3222 | - | 134 | 11496797 | 1194237 |
| Plasmid lp36 (Accession number NC_001855) | hypothetical protein BBK04 | 3416 | 3595 | - | 59 | 11496798 | 1194238 |
| Plasmid lp36 (Accession number NC_001855) | hypothetical protein BBK05 | 4902 | 5096 | - | 64 | 11496799 | 1194239 |
| Plasmid lp36 (Accession number NC_001855) | hypothetical protein BBK06 | 5126 | 5236 | + | 36 | 11496800 | 1194240 |
| Plasmid lp36 (Accession number NC_001855) | hypothetical protein BBK07 | 5288 | 6040 | - | 250 | 11496801 | 1194241 |
| Plasmid lp36 (Accession number NC_001855) | hypothetical protein BBK08 | 6177 | 6281 | - | 34 | 11496802 | 1194242 |

| | | | | | | | |
|---|---|---|---|---|---|---|---|
| Plasmid lp36 (Accession number NC_001855) | hypothetical protein BBK09 | 6366 | 6650 | + | 94 | 11496803 | 1194243 |
| Plasmid lp36 (Accession number NC_001855) | hypothetical protein BBK10 | 6804 | 6983 | - | 59 | 11496805 | 1194245 |
| Plasmid lp36 (Accession number NC_001855) | hypothetical protein BBK11 | 6956 | 7063 | + | 35 | 11496804 | 1194244 |
| Plasmid lp36 (Accession number NC_001855) | hypothetical protein BBK12 | 7335 | 8033 | + | 232 | 11496806 | 1194246 |
| Plasmid lp36 (Accession number NC_001855) | hypothetical protein BBK13 | 8164 | 8880 | - | 238 | 11496780 | 1194217 |
| Plasmid lp36 (Accession number NC_001855) | hypothetical protein BBK14 | 8921 | 9016 | + | 31 | 11496807 | 1194247 |
| Plasmid lp36 (Accession number NC_001855) | antigen, P35, putative | 9373 | 9999 | + | 208 | 11496781 | 1194218 |
| Plasmid lp36 (Accession number NC_001855) | hypothetical protein BBK16 | 10098 | 10223 | - | 41 | 11496819 | 1194259 |
| Plasmid lp36 (Accession number NC_001855) | adenine deaminase (adeC) | 10301 | 11947 | + | 548 | 11496787 | 1194225 |
| Plasmid lp36 (Accession number NC_001855) | hypothetical protein BBK18 | 12051 | 12143 | - | 30 | 11496788 | 1194226 |
| Plasmid lp36 (Accession number NC_001855) | hypothetical protein BBK19 | 12602 | 13237 | + | 211 | 11496820 | 1194260 |
| Plasmid lp36 (Accession number NC_001855) | hypothetical protein BBK20 | 13212 | 13304 | + | 30 | 11496821 | 1194261 |
| Plasmid lp36 (Accession number NC_001855) | hypothetical protein BBK21 | 13577 | 14326 | - | 249 | 11496789 | 1194227 |

| Plasmid lp36 (Accession number NC_001855) | hypothetical protein BBK22 | 1430 2 | 1484 1 | - | 179 | 11496790 | 1194228 |
|---|---|---|---|---|---|---|---|
| Plasmid lp36 (Accession number NC_001855) | hypothetical protein BBK23 | 1483 4 | 1576 0 | - | 308 | 11496822 | 1194262 |
| Plasmid lp36 (Accession number NC_001855) | hypothetical protein BBK24 | 1627 5 | 1688 6 | + | 203 | 11496791 | 1194229 |
| Plasmid lp36 (Accession number NC_001855) | hypothetical protein BBK26 | 1834 6 | 1846 5 | + | 39 | 11496823 | 1194263 |
| Plasmid lp36 (Accession number NC_001855) | hypothetical protein BBK27 | 1880 4 | 1909 4 | - | 96 | 11496809 | 1194249 |
| Plasmid lp36 (Accession number NC_001855) | hypothetical protein BBK28 | 1923 2 | 1935 1 | + | 39 | 11496810 | 1194250 |
| Plasmid lp36 (Accession number NC_001855) | hypothetical protein BBK29 | 1971 5 | 1980 7 | - | 30 | 11496811 | 1194251 |
| Plasmid lp36 (Accession number NC_001855) | hypothetical protein BBK30 | 1993 5 | 2003 6 | + | 33 | 11496812 | 1194252 |
| Plasmid lp36 (Accession number NC_001855) | hypothetical protein BBK31 | 2002 6 | 2016 9 | + | 47 | 11496813 | 1194253 |
| Plasmid lp36 (Accession number NC_001855) | immunogenic protein P35 | 2038 9 | 2145 3 | + | 354 | 11496782 | 1194219 |
| Plasmid lp36 (Accession number NC_001855) | hypothetical protein BBK33 | 2172 0 | 2189 3 | + | 57 | 11496808 | 1194248 |
| Plasmid lp36 (Accession number NC_001855) | hypothetical protein BBK34 | 2191 2 | 2213 3 | + | 73 | 11496814 | 1194254 |

| Plasmid lp36 (Accession number NC_001855) | hypothetical protein BBK35 | 2229 4 | 2246 7 | + | 57 | 11496815 | 1194255 |
|---|---|---|---|---|---|---|---|
| Plasmid lp36 (Accession number NC_001855) | hypothetical protein BBK36 | 2254 5 | 2264 9 | + | 34 | 11496816 | 1194256 |
| Plasmid lp36 (Accession number NC_001855) | hypothetical protein BBK38 | 2391 9 | 2414 6 | - | 75 | 11496824 | 1194264 |
| Plasmid lp36 (Accession number NC_001855) | hypothetical protein BBK39 | 2429 3 | 2467 0 | + | 125 | 11496825 | 1194265 |
| Plasmid lp36 (Accession number NC_001855) | hypothetical protein BBK40 | 2510 3 | 2565 7 | + | 184 | 11496792 | 1194231 |
| Plasmid lp36 (Accession number NC_001855) | hypothetical protein BBK41 | 2581 3 | 2637 9 | - | 188 | 11496826 | 1194266 |
| Plasmid lp36 (Accession number NC_001855) | hypothetical protein BBK42 | 2658 5 | 2680 3 | - | 72 | 11496827 | 1194267 |
| Plasmid lp36 (Accession number NC_001855) | hypothetical protein BBK43 | 2693 7 | 2704 4 | + | 35 | 11496828 | 1194268 |
| Plasmid lp36 (Accession number NC_001855) | hypothetical protein BBK44 | 2723 4 | 2735 3 | + | 39 | 11496829 | 1194269 |
| Plasmid lp36 (Accession number NC_001855) | immunogenic protein P37, putative | 2738 3 | 2831 5 | - | 310 | 11496793 | 1194232 |
| Plasmid lp36 (Accession number NC_001855) | hypothetical protein BBK47 | 2947 7 | 3046 3 | - | 328 | 11496830 | 1194270 |
| Plasmid lp36 (Accession number NC_001855) | immunogenic protein P37, putative | 3071 9 | 3158 5 | - | 288 | 11496794 | 1194233 |

| | | | | | | | |
|---|---|---|---|---|---|---|---|
| Plasmid lp36 (Accession number NC_001855) | hypothetical protein BBK49 | 3182 7 | 3282 2 | - | 331 | 11496817 | 1194257 |
| Plasmid lp36 (Accession number NC_001855) | immunogenic protein P37 | 3308 1 | 3407 9 | - | 332 | 11496783 | 1194221 |
| Plasmid lp36 (Accession number NC_001855) | hypothetical protein BBK51 | 3423 2 | 3433 0 | + | 32 | 11496818 | 1194258 |
| Plasmid lp36 (Accession number NC_001855) | protein p23 | 3459 5 | 3544 3 | - | 282 | 11496784 | 1194222 |
| Plasmid lp36 (Accession number NC_001855) | outer membrane protein | 3586 8 | 3642 2 | + | 184 | 11496785 | 1194223 |
| Plasmid lp36 (Accession number NC_001855) | hypothetical protein BBK54 | 3638 9 | 3657 7 | - | 62 | 11496786 | 1194224 |
| Plasmid lp38 (Accession number NC_001856) | hypothetical protein BBJ01 | 482 | 667 | + | 61 | 11496862 | 1194305 |
| Plasmid lp38 (Accession number NC_001856) | hypothetical protein BBJ02 | 927 | 1211 | + | 94 | 11496863 | 1194306 |
| Plasmid lp38 (Accession number NC_001856) | hypothetical protein BBJ03 | 1593 | 1745 | + | 50 | 11496840 | 1194283 |
| Plasmid lp38 (Accession number NC_001856) | hypothetical protein BBJ04 | 2268 | 2381 | - | 37 | 11496841 | 1194284 |
| Plasmid lp38 (Accession number NC_001856) | hypothetical protein BBJ06 | 3486 | 3632 | + | 48 | 11496842 | 1194285 |
| Plasmid lp38 (Accession number NC_001856) | hypothetical protein BBJ07 | 4164 | 4307 | - | 47 | 11496843 | 1194286 |
| Plasmid lp38 (Accession number NC_001856) | hypothetical protein BBJ08 | 4576 | 5496 | + | 306 | 11496844 | 1194287 |

| | | | | | | | |
|---|---|---|---|---|---|---|---|
| Plasmid lp38 (Accession number NC_001856) | outer surface protein D (ospD) | 6089 | 6862 | + | 257 | 11496832 | 1194272 |
| Plasmid lp38 (Accession number NC_001856) | hypothetical protein BBJ10 | 7270 | 7476 | + | 68 | 11496845 | 1194288 |
| Plasmid lp38 (Accession number NC_001856) | hypothetical protein BBJ11 | 7780 | 7965 | - | 61 | 11496846 | 1194289 |
| Plasmid lp38 (Accession number NC_001856) | hypothetical protein BBJ12 | 8633 | 8725 | - | 30 | 11496847 | 1194290 |
| Plasmid lp38 (Accession number NC_001856) | hypothetical protein BBJ13 | 8877 | 9155 | - | 92 | 11496848 | 1194291 |
| Plasmid lp38 (Accession number NC_001856) | hypothetical protein BBJ14 | 9125 | 9286 | + | 53 | 11496833 | 1194273 |
| Plasmid lp38 (Accession number NC_001856) | hypothetical protein BBJ15 | 1004 0 | 1016 8 | - | 42 | 11496849 | 1194292 |
| Plasmid lp38 (Accession number NC_001856) | hypothetical protein BBJ16 | 1051 8 | 1110 5 | - | 195 | 11496834 | 1194274 |
| Plasmid lp38 (Accession number NC_001856) | hypothetical protein BBJ17 | 1115 2 | 1188 9 | - | 245 | 11496835 | 1194275 |
| Plasmid lp38 (Accession number NC_001856) | hypothetical protein BBJ18 | 1186 2 | 1245 2 | - | 196 | 11496836 | 1194276 |
| Plasmid lp38 (Accession number NC_001856) | hypothetical protein BBJ19 | 1244 5 | 1344 0 | - | 331 | 11496837 | 1194277 |
| Plasmid lp38 (Accession number NC_001856) | hypothetical protein BBJ20 | 1377 2 | 1393 6 | - | 54 | 11496864 | 1194307 |

| | | | | | | | |
|---|---|---|---|---|---|---|---|
| Plasmid lp38 (Accession number NC_001856) | hypothetical protein BBJ21 | 1551 4 | 1566 0 | + | 48 | 11496865 | 1194308 |
| Plasmid lp38 (Accession number NC_001856) | hypothetical protein BBJ22 | 1590 2 | 1600 3 | - | 33 | 11496866 | 1194309 |
| Plasmid lp38 (Accession number NC_001856) | hypothetical protein BBJ23 | 1650 5 | 1732 9 | + | 274 | 11496867 | 1194310 |
| Plasmid lp38 (Accession number NC_001856) | hypothetical protein BBJ24 | 1738 8 | 1817 0 | + | 260 | 11496868 | 1194311 |
| Plasmid lp38 (Accession number NC_001856) | hypothetical protein BBJ25 | 1820 2 | 1925 4 | + | 350 | 11496869 | 1194312 |
| Plasmid lp38 (Accession number NC_001856) | ABC transporter, ATP-binding protein | 1931 3 | 2000 8 | + | 231 | 11496838 | 1194278 |
| Plasmid lp38 (Accession number NC_001856) | hypothetical protein BBJ27 | 1999 5 | 2123 0 | + | 411 | 11496870 | 1194313 |
| Plasmid lp38 (Accession number NC_001856) | hypothetical protein BBJ28 | 2122 0 | 2194 8 | + | 242 | 11496850 | 1194293 |
| Plasmid lp38 (Accession number NC_001856) | hypothetical protein BBJ29 | 2197 1 | 2300 8 | + | 345 | 11496851 | 1194294 |
| Plasmid lp38 (Accession number NC_001856) | hypothetical protein BBJ30 | 2301 8 | 2312 2 | + | 34 | 11496852 | 1194295 |
| Plasmid lp38 (Accession number NC_001856) | hypothetical protein BBJ31 | 2336 6 | 2408 8 | + | 240 | 11496853 | 1194296 |
| Plasmid lp38 (Accession number NC_001856) | hypothetical protein BBJ32 | 2438 6 | 2451 7 | - | 43 | 11496854 | 1194297 |

| | | | | | | | |
|---|---|---|---|---|---|---|---|
| Plasmid lp38 (Accession number NC_001856) | hypothetical protein BBJ33 | 2468 1 | 2479 4 | + | 37 | 11496855 | 1194298 |
| Plasmid lp38 (Accession number NC_001856) | hypothetical protein BBJ34 | 2533 7 | 2640 7 | - | 356 | 11496856 | 1194299 |
| Plasmid lp38 (Accession number NC_001856) | hypothetical protein BBJ35 | 2685 8 | 2696 2 | + | 34 | 11496857 | 1194300 |
| Plasmid lp38 (Accession number NC_001856) | hypothetical protein BBJ36 | 2699 5 | 2805 3 | - | 352 | 11496858 | 1194301 |
| Plasmid lp38 (Accession number NC_001856) | hypothetical protein BBJ37 | 2827 8 | 2844 2 | - | 54 | 11496859 | 1194302 |
| Plasmid lp38 (Accession number NC_001856) | hypothetical protein BBJ38 | 2860 5 | 2870 3 | - | 32 | 11496860 | 1194303 |
| Plasmid lp38 (Accession number NC_001856) | hypothetical protein BBJ39 | 2926 4 | 2940 1 | - | 45 | 11496861 | 1194304 |
| Plasmid lp38 (Accession number NC_001856) | hypothetical protein BBJ40 | 2982 7 | 2992 2 | + | 31 | 11496871 | 1194314 |
| Plasmid lp38 (Accession number NC_001856) | antigen, P35, putative | 2990 5 | 3077 1 | - | 288 | 11496839 | 1194279 |
| Plasmid lp38 (Accession number NC_001856) | hypothetical protein BBJ42 | 3094 2 | 3113 3 | - | 63 | 11496872 | 1194315 |
| Plasmid lp38 (Accession number NC_001856) | hypothetical protein BBJ43 | 3122 0 | 3214 0 | + | 306 | 11496873 | 1194316 |
| Plasmid lp38 (Accession number NC_001856) | hypothetical protein BBJ44 | 3215 0 | 3225 4 | + | 34 | 11496874 | 1194317 |

| Plasmid lp38 (Accession number NC_001856) | hypothetical protein BBJ45 | 32498 | 33220 | + | 240 | 11496875 | 1194318 |
|---|---|---|---|---|---|---|---|
| Plasmid lp38 (Accession number NC_001856) | hypothetical protein BBJ46 | 34372 | 34668 | - | 98 | 11496876 | 1194319 |
| Plasmid lp38 (Accession number NC_001856) | hypothetical protein BBJ47 | 34905 | 35591 | - | 228 | 11496877 | 1194320 |
| Plasmid lp38 (Accession number NC_001856) | hypothetical protein BBJ48 | 35634 | 36272 | - | 212 | 11496878 | 1194321 |
| Plasmid lp38 (Accession number NC_001856) | hypothetical protein BBJ49 | 36312 | 36455 | - | 47 | 11496879 | 1194322 |
| Plasmid cp26 (Accession number NC_001903) | hypothetical protein BBB01 | 16 | 324 | + | 102 | 11497020 | 1194411 |
| Plasmid cp26 (Accession number NC_001903) | hypothetical protein BBB02 | 308 | 751 | - | 147 | 11497029 | 1194420 |
| Plasmid cp26 (Accession number NC_001903) | hypothetical protein BBB03 | 837 | 2186 | - | 449 | 11497028 | 1194419 |
| Plasmid cp26 (Accession number NC_001903) | PTS system, cellobiose-specific IIC component (celB) | 2476 | 3807 | - | 443 | 11497019 | 1194410 |
| Plasmid cp26 (Accession number NC_001903) | PTS system, cellobiose-specific IIA component (celC) | 4084 | 4431 | + | 115 | 11497018 | 1194409 |

| Plasmid cp26 (Accession number NC_001903) | PTS system, cellobiose-specific IIB component (celA) | 4440 | 4757 | + | 105 | 11497017 | 1194408 |
|---|---|---|---|---|---|---|---|
| Plasmid cp26 (Accession number NC_001903) | outer surface protein, putative | 4769 | 5866 | + | 365 | 11497016 | 1194407 |
| Plasmid cp26 (Accession number NC_001903) | hypothetical protein BBB08 | 5888 | 6517 | - | 209 | 11497027 | 1194418 |
| Plasmid cp26 (Accession number NC_001903) | hypothetical protein BBB09 | 6677 | 7714 | + | 345 | 11497026 | 1194417 |
| Plasmid cp26 (Accession number NC_001903) | hypothetical protein BBB10 | 7836 | 8765 | + | 309 | 11497015 | 1194406 |
| Plasmid cp26 (Accession number NC_001903) | hypothetical protein BBB11 | 8781 | 9299 | + | 172 | 11497014 | 1194405 |
| Plasmid cp26 (Accession number NC_001903) | hypothetical protein BBB12 | 9275 | 1003 6 | + | 253 | 11497013 | 1194404 |
| Plasmid cp26 (Accession number NC_001903) | hypothetical protein BBB13 | 1010 4 | 1065 2 | + | 182 | 11497012 | 1194403 |
| Plasmid cp26 (Accession number NC_001903) | hypothetical protein BBB14 | 1092 0 | 1141 7 | - | 165 | 11497011 | 1194402 |
| Plasmid cp26 (Accession number NC_001903) | hypothetical protein BBB15 | 1163 6 | 1174 0 | + | 34 | 11497025 | 1194416 |

| | | | | | | | |
|---|---|---|---|---|---|---|---|
| Plasmid cp26 (Accession number NC_001903) | oligopeptide ABC transporter, periplasmic oligopeptide-binding protein (oppAIV) | 1201 4 | 1360 6 | + | 530 | 11497010 | 1194401 |
| Plasmid cp26 (Accession number NC_001903) | inositol-5-monophosphate dehydrogenase | 1389 3 | 1510 7 | - | 404 | 11497009 | 1194400 |
| Plasmid cp26 (Accession number NC_001903) | bifunctional GMP synthase/glutamine amidotransferase protein | 1513 2 | 1671 8 | - | 528 | 11497008 | 1194399 |
| Plasmid cp26 (Accession number NC_001903) | outer surface protein C (ospC) | 1690 3 | 1753 5 | + | 210 | 11497024 | 1194415 |
| Plasmid cp26 (Accession number NC_001903) | hypothetical protein BBB20 | 1762 3 | 1773 3 | - | 36 | 11497036 | 1194427 |
| Plasmid cp26 (Accession number NC_001903) | hypothetical protein BBB21 | 1775 0 | 1784 5 | + | 31 | 11497035 | 1194426 |
| Plasmid cp26 (Accession number NC_001903) | hypothetical protein BBB22 | 1796 6 | 1932 1 | - | 451 | 11497023 | 1194414 |
| Plasmid cp26 (Accession number NC_001903) | hypothetical protein BBB23 | 1943 1 | 2082 2 | - | 463 | 11497022 | 1194413 |
| Plasmid cp26 (Accession number NC_001903) | hypothetical protein BBB24 | 2085 8 | 2136 4 | - | 168 | 11497034 | 1194425 |
| Plasmid cp26 (Accession number NC_001903) | hypothetical protein BBB25 | 2133 9 | 2185 1 | - | 170 | 11497033 | 1194424 |

| | | | | | | | |
|---|---|---|---|---|---|---|---|
| Plasmid cp26 (Accession number NC_001903) | hypothetical protein BBB26 | 2189 8 | 2259 3 | + | 231 | 11497032 | 1194423 |
| Plasmid cp26 (Accession number NC_001903) | hypothetical protein BBB27 | 2260 3 | 2315 4 | - | 183 | 11497031 | 1194422 |
| Plasmid cp26 (Accession number NC_001903) | hypothetical protein BBB28 | 2325 5 | 2449 9 | + | 414 | 11497030 | 1194421 |
| Plasmid cp26 (Accession number NC_001903) | PTS system, maltose and glucose-specific IIABC component (malX) | 2482 5 | 2645 3 | + | 542 | 11497021 | 1194412 |
| Plasmid cp9 (Accession number NC_001904) | hypothetical protein BBC01 | 163 | 1272 | + | 369 | 11497057 | 1194436 |
| Plasmid cp9 (Accession number NC_001904) | hypothetical protein BBC02 | 1282 | 1839 | + | 185 | 11497049 | 1194428 |
| Plasmid cp9 (Accession number NC_001904) | hypothetical protein BBC03 | 1892 | 2452 | + | 186 | 11497050 | 1194429 |
| Plasmid cp9 (Accession number NC_001904) | hypothetical protein BBC04 | 2590 | 2700 | - | 36 | 11497058 | 1194437 |
| Plasmid cp9 (Accession number NC_001904) | hypothetical protein BBC05 | 2804 | 3712 | + | 302 | 11497051 | 1194430 |
| Plasmid cp9 (Accession number NC_001904) | exported protein A (eppA) | 3853 | 4377 | - | 174 | 11497052 | 1194431 |
| Plasmid cp9 (Accession number NC_001904) | hypothetical protein BBC07 | 4504 | 4788 | - | 94 | 11497059 | 1194438 |

| | | | | | | | |
|---|---|---|---|---|---|---|---|
| Plasmid cp9 (Accession number NC_001904) | hypothetical protein BBC08 | 5534 | 5980 | + | 148 | 11497053 | 1194432 |
| Plasmid cp9 (Accession number NC_001904) | rev protein (rev) | 6281 | 6808 | - | 175 | 11497054 | 1194433 |
| Plasmid cp9 (Accession number NC_001904) | hypothetical protein BBC11 | 6974 | 7771 | + | 265 | 11497055 | 1194434 |
| Plasmid cp9 (Accession number NC_001904) | hypothetical protein BBC12 | 7911 | 9203 | - | 430 | 11497056 | 1194435 |
| Complete genome (Accession number NC_001318) | hypothetical protein BB0001 | 105 | 677 | + | | 15594347 | 1194837 |
| Complete genome (Accession number NC_001318) | beta-N-acetylhexosaminidase, putative | 768 | 1796 | - | | 15594348 | 1194838 |
| Complete genome (Accession number NC_001318) | hypothetical protein BB0003 | 1784 | 3148 | - | | 15594349 | 1194839 |
| Complete genome (Accession number NC_001318) | phosphoglucomutase (femD) | 3395 | 5188 | + | | 15594350 | 1194840 |
| Complete genome (Accession number NC_001318) | tryptophanyl-tRNA synthetase | 5251 | 6312 | - | | 15594351 | 1194841 |
| Complete genome (Accession number NC_001318) | hypothetical protein BB0006 | 6309 | 7433 | - | | 15594352 | 1194842 |
| Complete genome (Accession number NC_001318) | hypothetical protein BB0007 | 7458 | 8315 | - | | 15594353 | 1194843 |
| Complete genome (Accession number NC_001318) | hypothetical protein BB0008 | 8412 | 9197 | + | | 15594354 | 1194844 |

| | | | | | | | |
|---|---|---|---|---|---|---|---|
| Complete genome (Accession number NC_001318) | hypothetical protein BB0009 | 9202 | 1020 6 | + | | 15594355 | 1194845 |
| Complete genome (Accession number NC_001318) | holo-acyl-carrier protein synthase, putative | 1020 3 | 1057 7 | + | | 15594356 | 1194846 |
| Complete genome (Accession number NC_001318) | hypothetical protein BB0011 | 1058 1 | 1142 0 | + | | 15594357 | 1194847 |
| Complete genome (Accession number NC_001318) | tRNA pseudouridine synthase A | 1142 1 | 1216 1 | + | | 15594358 | 1194848 |
| Complete genome (Accession number NC_001318) | hypothetical protein BB0013 | 1215 4 | 1275 3 | + | | 15594359 | 1194849 |
| Complete genome (Accession number NC_001318) | primosomal protein N (priA) | 1274 6 | 1472 8 | + | | 15594360 | 1194850 |
| Complete genome (Accession number NC_001318) | uridine kinase | 1472 5 | 1534 8 | - | | 15594361 | 1194851 |
| Complete genome (Accession number NC_001318) | glpE protein (glpE) | 1534 5 | 1572 2 | - | | 15594362 | 1194852 |
| Complete genome (Accession number NC_001318) | hypothetical protein BB0017 | 1584 5 | 1680 4 | + | | 15594363 | 1194853 |
| Complete genome (Accession number NC_001318) | hypothetical protein BB0018 | 1680 7 | 1781 7 | - | | 15594364 | 1194854 |
| Complete genome (Accession number NC_001318) | hypothetical protein BB0019 | 1779 2 | 1830 4 | - | | 15594365 | 1194855 |
| Complete genome (Accession number NC_001318) | diphosphate--fructose-6-phosphate 1-phosphotransfera se | 1831 2 | 1997 9 | - | | 15594366 | 1194856 |

| Complete genome (Accession number NC_001318) | S-adenosylmethionine: tRNA ribosyltransferase-isomerase | 2044 5 | 2109 2 | - | | 15594367 | 1194857 |
|---|---|---|---|---|---|---|---|
| Complete genome (Accession number NC_001318) | Holliday junction DNA helicase B | 2105 8 | 2210 1 | - | | 15594368 | 1194858 |
| Complete genome (Accession number NC_001318) | Holliday junction DNA helicase (ruvA) | 2214 6 | 2273 9 | - | | 15594369 | 1194859 |
| Complete genome (Accession number NC_001318) | hypothetical protein BB0024 | 2281 9 | 2395 8 | + | | 15594370 | 1194860 |
| Complete genome (Accession number NC_001318) | hypothetical protein BB0025 | 2396 5 | 2469 6 | - | | 15594371 | 1194861 |
| Complete genome (Accession number NC_001318) | methylenetetrahydrofolate dehydrogenase (folD) | 2469 7 | 2562 3 | - | | 15594372 | 1194862 |
| Complete genome (Accession number NC_001318) | hypothetical protein BB0027 | 2575 3 | 2639 1 | + | | 15594373 | 1194863 |
| Complete genome (Accession number NC_001318) | hypothetical protein BB0028 | 2639 9 | 2744 8 | + | | 15594374 | 1194864 |
| Complete genome (Accession number NC_001318) | hypothetical protein BB0029 | 2743 4 | 2786 5 | - | | 15594375 | 1194865 |
| Complete genome (Accession number NC_001318) | signal peptidase I (lepB-1) | 2785 5 | 2849 0 | - | | 15594376 | 1194866 |
| Complete genome (Accession number NC_001318) | signal peptidase I (lepB-2) | 2849 2 | 2947 2 | - | | 15594377 | 1194867 |

| | | | | | | | |
|---|---|---|---|---|---|---|---|
| Complete genome (Accession number NC_001318) | hypothetical protein BB0032 | 2954 3 | 3101 5 | + | | 15594378 | 1194868 |
| Complete genome (Accession number NC_001318) | SsrA-binding protein | 3102 7 | 3147 9 | + | | 15594379 | 1194869 |
| Complete genome (Accession number NC_001318) | hypothetical protein BB0034 | 3155 0 | 3208 9 | - | | 15594380 | 1194870 |
| Complete genome (Accession number NC_001318) | DNA topoisomerase IV subunit A | 3215 7 | 3403 7 | - | | 15594381 | 1194871 |
| Complete genome (Accession number NC_001318) | DNA topoisomerase IV subunit B | 3403 7 | 3583 6 | - | | 15594382 | 1194872 |
| Complete genome (Accession number NC_001318) | 1-acyl-sn-glycerol-3-phosphate acetyltransferase (plsC) | 3585 7 | 3660 9 | - | | 15594383 | 1194873 |
| Complete genome (Accession number NC_001318) | hypothetical protein BB0038 | 3660 6 | 3812 3 | - | | 15594384 | 1194874 |
| Complete genome (Accession number NC_001318) | hypothetical protein BB0039 | 3813 6 | 3963 8 | - | | 15594385 | 1194875 |
| Complete genome (Accession number NC_001318) | chemotaxis protein methyltransferase (cheR-1) | 3982 6 | 4067 7 | + | | 15594386 | 1194876 |
| Complete genome (Accession number NC_001318) | hypothetical protein BB0041 | 4067 7 | 4076 9 | + | | 15594387 | 1194877 |
| Complete genome (Accession number NC_001318) | phosphate transport system regulatory protein (phoU) | 4076 1 | 4143 2 | - | | 15594388 | 1194878 |

| Complete genome (Accession number NC_001318) | hypothetical protein BB0043 | 4144 3 | 4248 3 | - | | 15594389 | 1194879 |
|---|---|---|---|---|---|---|---|
| Complete genome (Accession number NC_001318) | hypothetical protein BB0044 | 4248 0 | 4288 1 | - | | 15594390 | 1194880 |
| Complete genome (Accession number NC_001318) | P115 protein | 4290 7 | 4536 6 | - | | 15594391 | 1194881 |
| Complete genome (Accession number NC_001318) | ribonuclease H (rnhB) | 4544 6 | 4599 1 | + | | 15594392 | 1194882 |
| Complete genome (Accession number NC_001318) | hypothetical protein BB0047 | 4601 1 | 4639 4 | - | | 15594393 | 1194883 |
| Complete genome (Accession number NC_001318) | hypothetical protein BB0048 | 4660 1 | 4677 1 | - | | 15594394 | 1194884 |
| Complete genome (Accession number NC_001318) | hypothetical protein BB0049 | 4692 1 | 4704 0 | - | | 15594395 | 1194885 |
| Complete genome (Accession number NC_001318) | hypothetical protein BB0050 | 4716 7 | 4784 1 | + | | 15594396 | 1194886 |
| Complete genome (Accession number NC_001318) | hypothetical protein BB0051 | 4790 5 | 4860 6 | + | | 15594397 | 1194887 |
| Complete genome (Accession number NC_001318) | spoU protein (spoU) | 4860 3 | 4925 9 | - | | 15594398 | 1194888 |
| Complete genome (Accession number NC_001318) | uracil-DNA glycosylase | 4934 1 | 5001 2 | + | | 15594399 | 1194889 |
| Complete genome (Accession number NC_001318) | preprotein translocase subunit SecG | 5011 2 | 5048 9 | - | | 15594400 | 1194890 |
| Complete genome (Accession number NC_001318) | triosephosphate isomerase | 5049 0 | 5125 1 | - | | 15594401 | 1194891 |

| | | | | | | | |
|---|---|---|---|---|---|---|---|
| Complete genome (Accession number NC_001318) | phosphoglycerate kinase | 5125 3 | 5243 4 | - | | 15594402 | 1194892 |
| Complete genome (Accession number NC_001318) | glyceraldehyde 3-phosphate dehydrogenase (gap) | 5245 4 | 5346 1 | - | | 15594403 | 1194893 |
| Complete genome (Accession number NC_001318) | hypothetical protein BB0058 | 5353 4 | 5550 4 | - | | 15594404 | 1194894 |
| Complete genome (Accession number NC_001318) | hemolysin (tlyC) | 5550 1 | 5628 0 | - | | 15594405 | 1194895 |
| Complete genome (Accession number NC_001318) | hypothetical protein BB0060 | 5628 1 | 5674 8 | - | | 15594406 | 1194896 |
| Complete genome (Accession number NC_001318) | thioredoxin (trxA) | 5683 2 | 5718 5 | - | | 15594407 | 1194897 |
| Complete genome (Accession number NC_001318) | hypothetical protein BB0062 | 5725 9 | 5797 5 | + | | 15594408 | 1194898 |
| Complete genome (Accession number NC_001318) | hypothetical protein BB0063 | 5802 9 | 5903 6 | - | | 15594409 | 1194899 |
| Complete genome (Accession number NC_001318) | methionyl-tRNA formyltransferase (fmt) | 5910 1 | 6003 9 | - | | 15594410 | 1194900 |
| Complete genome (Accession number NC_001318) | polypeptide deformylase (def) | 6003 6 | 6055 4 | - | | 15594411 | 1194901 |
| Complete genome (Accession number NC_001318) | hypothetical protein BB0066 | 6056 8 | 6125 7 | - | | 15594412 | 1194902 |
| Complete genome (Accession number NC_001318) | peptidase, putative | 6125 4 | 6303 2 | - | | 15594413 | 1194903 |

| | | | | | | | |
|---|---|---|---|---|---|---|---|
| Complete genome (Accession number NC_001318) | hypothetical protein BB0068 | 6314 7 | 6402 8 | + | | 15594414 | 1194904 |
| Complete genome (Accession number NC_001318) | aminopeptidase II | 6403 8 | 6527 6 | + | | 15594415 | 1194905 |
| Complete genome (Accession number NC_001318) | hypothetical protein BB0070 | 6529 1 | 6575 2 | + | | 15594416 | 1194906 |
| Complete genome (Accession number NC_001318) | hypothetical protein BB0071 | 6576 8 | 6724 0 | + | | 15594417 | 1194907 |
| Complete genome (Accession number NC_001318) | hypothetical protein BB0072 | 6724 8 | 6956 9 | + | | 15594418 | 1194908 |
| Complete genome (Accession number NC_001318) | hypothetical protein BB0073 | 6956 6 | 7012 6 | + | | 15594419 | 1194909 |
| Complete genome (Accession number NC_001318) | peptide chain release factor 2 | 7017 3 | 7121 9 | + | | 15594420 | 1194910 |
| Complete genome (Accession number NC_001318) | hypothetical protein BB0075 | 7120 1 | 7261 0 | + | | 15594421 | 1194911 |
| Complete genome (Accession number NC_001318) | cell division protein, putative | 7264 7 | 7349 2 | + | | 15594422 | 1194912 |
| Complete genome (Accession number NC_001318) | hypothetical protein BB0077 | 7348 9 | 7451 7 | + | | 15594423 | 1194913 |
| Complete genome (Accession number NC_001318) | hypothetical protein BB0078 | 7451 8 | 7503 9 | + | | 15594424 | 1194914 |
| Complete genome (Accession number NC_001318) | hypothetical protein BB0079 | 7511 0 | 7577 2 | + | | 15594425 | 1194915 |

| | | | | | | | |
|---|---|---|---|---|---|---|---|
| Complete genome (Accession number NC_001318) | ABC transporter, ATP-binding protein | 7573 2 | 7645 4 | + | | 15594426 | 1194916 |
| Complete genome (Accession number NC_001318) | hypothetical protein BB0081 | 7645 1 | 7770 1 | + | | 15594427 | 1194917 |
| Complete genome (Accession number NC_001318) | hypothetical protein BB0082 | 7771 1 | 7900 9 | - | | 15594428 | 1194918 |
| Complete genome (Accession number NC_001318) | hypothetical protein BB0083 | 7900 2 | 7937 6 | - | | 15594429 | 1194919 |
| Complete genome (Accession number NC_001318) | aminotransferase (nifS) | 7950 5 | 8077 3 | + | | 15594430 | 1194920 |
| Complete genome (Accession number NC_001318) | hypothetical protein BB0085 | 8098 3 | 8140 8 | + | | 15594431 | 1194921 |
| Complete genome (Accession number NC_001318) | hypothetical protein BB0086 | 8140 5 | 8294 0 | + | | 15594432 | 1194922 |
| Complete genome (Accession number NC_001318) | L-lactate dehydrogenase (ldh) | 8297 0 | 8392 0 | - | | 15594433 | 1194923 |
| Complete genome (Accession number NC_001318) | GTP-binding protein LepA | 8404 1 | 8572 0 | - | | 15594434 | 1194924 |
| Complete genome (Accession number NC_001318) | hypothetical protein BB0089 | 8588 0 | 8685 4 | - | | 15594435 | 1194925 |
| Complete genome (Accession number NC_001318) | V-type ATP synthase subunit K | 8692 6 | 8736 0 | - | | 15594436 | 1194926 |
| Complete genome (Accession number NC_001318) | V-type ATP synthase subunit I | 8737 7 | 8920 3 | - | | 15594437 | 1194927 |

| Complete genome (Accession number NC_001318) | V-type ATP synthase subunit D | 8920 0 | 8981 4 | - | | 15594438 | 1194928 |
|---|---|---|---|---|---|---|---|
| Complete genome (Accession number NC_001318) | V-type ATP synthase subunit B | 8981 1 | 9111 5 | - | | 15594439 | 1194929 |
| Complete genome (Accession number NC_001318) | V-type ATP synthase subunit A | 9113 7 | 9286 4 | - | | 1,62E+08 | 1194930 |
| Complete genome (Accession number NC_001318) | hypothetical protein BB0095 | 9287 8 | 9342 3 | - | | 15594441 | 1194931 |
| Complete genome (Accession number NC_001318) | V-type ATP synthase subunit E | 9343 3 | 9403 5 | - | | 15594442 | 1194932 |
| Complete genome (Accession number NC_001318) | hypothetical protein BB0097 | 9421 9 | 9460 5 | - | | 15594443 | 1194933 |
| Complete genome (Accession number NC_001318) | recombination and DNA strand exchange inhibitor protein | 9461 1 | 9695 3 | - | | 15594444 | 1194934 |
| Complete genome (Accession number NC_001318) | hypothetical protein BB0099 | 9694 3 | 9786 6 | - | | 15594445 | 1194935 |
| Complete genome (Accession number NC_001318) | glutamate racemase (murI) | 9786 3 | 9864 8 | - | | 15594446 | 1194936 |
| Complete genome (Accession number NC_001318) | asparaginyl-tRNA synthetase | 9880 5 | 1002 05 | + | | 15594447 | 1194937 |
| Complete genome (Accession number NC_001318) | hypothetical protein BB0102 | 1002 28 | 1007 49 | + | | 15594448 | 1194938 |
| Complete genome (Accession number NC_001318) | hypothetical protein BB0103 | 1007 46 | 1013 39 | - | | 15594449 | 1194939 |

| | | | | | | | |
|---|---|---|---|---|---|---|---|
| Complete genome (Accession number NC_001318) | periplasmic serine protease DO (htrA) | 1015 25 | 1029 76 | - | | 15594450 | 1194940 |
| Complete genome (Accession number NC_001318) | methionine aminopeptidase (map) | 1030 16 | 1037 71 | - | | 15594451 | 1194941 |
| Complete genome (Accession number NC_001318) | hypothetical protein BB0106 | 1037 64 | 1054 73 | - | | 15594452 | 1194942 |
| Complete genome (Accession number NC_001318) | transcription antitermination protein NusB | 1054 18 | 1058 55 | - | | 15594453 | 1194943 |
| Complete genome (Accession number NC_001318) | basic membrane protein | 1058 84 | 1068 94 | - | | 15594454 | 1194944 |
| Complete genome (Accession number NC_001318) | acetyl-CoA C-acetyltransferase (fadA) | 1069 74 | 1081 70 | - | | 15594455 | 1194945 |
| Complete genome (Accession number NC_001318) | hypothetical protein BB0110 | 1083 07 | 1096 71 | + | | 15594456 | 1194946 |
| Complete genome (Accession number NC_001318) | replicative DNA helicase (dnaB) | 1096 75 | 1110 42 | - | | 15594457 | 1194947 |
| Complete genome (Accession number NC_001318) | 50S ribosomal protein L9 | 1110 46 | 1115 67 | - | | 15594458 | 1194948 |
| Complete genome (Accession number NC_001318) | 30S ribosomal protein S18 | 1115 85 | 1118 75 | - | | 15594459 | 1194949 |
| Complete genome (Accession number NC_001318) | single-stranded DNA-binding protein (ssb) | 1118 89 | 1123 38 | - | | 15594460 | 1194950 |

| | | | | | | | |
|---|---|---|---|---|---|---|---|
| Complete genome (Accession number NC_001318) | 30S ribosomal protein S6 | 1123 50 | 1127 69 | - | | 15594461 | 1194951 |
| Complete genome (Accession number NC_001318) | PTS system, maltose and glucose-specific IIABC component (malX) | 1129 09 | 1146 33 | - | | 15594462 | 1194952 |
| Complete genome (Accession number NC_001318) | hemolysin III (yplQ) | 1148 07 | 1155 08 | + | | 15594463 | 1194953 |
| Complete genome (Accession number NC_001318) | zinc protease, putative | 1155 05 | 1168 18 | - | | 15594464 | 1194954 |
| Complete genome (Accession number NC_001318) | phosphatidate cytidylyltransferas e | 1168 25 | 1177 63 | - | | 15595197 | 1195711 |
| Complete genome (Accession number NC_001318) | hypothetical protein BB0120 | 1176 78 | 1183 70 | - | | 15594465 | 1194955 |
| Complete genome (Accession number NC_001318) | ribosome releasing factor (frr) | 1183 75 | 1189 29 | - | | 15594466 | 1194956 |
| Complete genome (Accession number NC_001318) | elongation factor Ts | 1189 64 | 1198 03 | - | | 15594467 | 1194957 |
| Complete genome (Accession number NC_001318) | 30S ribosomal protein S2 | 1198 07 | 1205 89 | - | | 15594468 | 1194958 |
| Complete genome (Accession number NC_001318) | hypothetical protein BB0124 | 1208 04 | 1210 82 | - | | 15594469 | 1194959 |
| Complete genome (Accession number NC_001318) | hypothetical protein BB0125 | 1211 01 | 1218 23 | - | | 15594470 | 1194960 |
| Complete genome (Accession number NC_001318) | hypothetical protein BB0126 | 1218 80 | 1224 91 | - | | 15594471 | 1194961 |

| | | | | | | | |
|---|---|---|---|---|---|---|---|
| Complete genome (Accession number NC_001318) | 30S ribosomal protein S1 | 1224 91 | 1241 52 | - | | 15594472 | 1194962 |
| Complete genome (Accession number NC_001318) | cytidylate kinase (cmk-1) | 1241 49 | 1248 14 | - | | 15594473 | 1194963 |
| Complete genome (Accession number NC_001318) | hypothetical protein BB0129 | 1247 98 | 1255 47 | - | | 15594474 | 1194964 |
| Complete genome (Accession number NC_001318) | hypothetical protein BB0130 | 1255 34 | 1263 01 | - | | 15594475 | 1194965 |
| Complete genome (Accession number NC_001318) | recombinase A | 1263 16 | 1274 13 | - | | 15594476 | 1194966 |
| Complete genome (Accession number NC_001318) | transcript cleavage factor/unknown domain fusion protein | 1274 27 | 1301 32 | - | | 15594477 | 1194967 |
| Complete genome (Accession number NC_001318) | hypothetical protein BB0133 | 1301 70 | 1311 14 | - | | 15594478 | 1194968 |
| Complete genome (Accession number NC_001318) | hypothetical protein BB0134 | 1312 77 | 1324 25 | + | | 15594479 | 1194969 |
| Complete genome (Accession number NC_001318) | histidyl-tRNA synthetase | 1324 72 | 1338 45 | - | | 15594480 | 1194970 |
| Complete genome (Accession number NC_001318) | penicillin-binding protein (pbp-1) | 1339 75 | 1358 64 | + | | 15594481 | 1194971 |
| Complete genome (Accession number NC_001318) | long-chain-fatty-acid CoA ligase | 1358 74 | 1377 66 | - | | 15594482 | 1194972 |
| Complete genome (Accession number NC_001318) | hypothetical protein BB0138 | 1378 80 | 1380 89 | - | | 15594483 | 1194973 |

| | | | | | | | |
|---|---|---|---|---|---|---|---|
| Complete genome (Accession number NC_001318) | hypothetical protein BB0139 | 1384 34 | 1388 23 | - | | 15594484 | 1194974 |
| Complete genome (Accession number NC_001318) | acriflavine resistance protein (acrB) | 1388 50 | 1419 60 | - | | 15594485 | 1194975 |
| Complete genome (Accession number NC_001318) | membrane fusion protein (mtrC) | 1419 79 | 1429 35 | - | | 15594486 | 1194976 |
| Complete genome (Accession number NC_001318) | hypothetical protein BB0142 | 1429 44 | 1442 66 | - | | 15594487 | 1194977 |
| Complete genome (Accession number NC_001318) | alpha-hemolysin (hlyA) | 1443 07 | 1445 97 | + | | 15594488 | 1194978 |
| Complete genome (Accession number NC_001318) | glycine betaine, L-proline ABC transporter, glycine/betaine/L-proline-binding protein (proX) | 1445 86 | 1454 58 | - | | 15594489 | 1194979 |
| Complete genome (Accession number NC_001318) | glycine betaine, L-proline ABC transporter, permease protein (proW) | 1454 71 | 1463 70 | - | | 15594490 | 1194980 |
| Complete genome (Accession number NC_001318) | glycine betaine, L-proline ABC transporter, ATP-binding protein (proV) | 1463 77 | 1474 95 | - | | 15594491 | 1194981 |
| Complete genome (Accession number | flagellin | 1476 49 | 1486 59 | - | | 15594492 | 1194982 |

| NC_001318) | | | | | | | |
|---|---|---|---|---|---|---|---|
| Complete genome (Accession number NC_001318) | hypothetical protein BB0148 | 1487 10 | 1488 05 | + | | 15594493 | 1194983 |
| Complete genome (Accession number NC_001318) | flagellar hook-associated protein FliD | 1487 86 | 1507 83 | - | | 15594494 | 1194984 |
| Complete genome (Accession number NC_001318) | hypothetical protein BB0150 | 1508 05 | 1508 97 | - | | 15594495 | 1194985 |
| Complete genome (Accession number NC_001318) | N-acetylglucosamine-6-phosphate deacetylase (nagA) | 1509 74 | 1521 79 | + | | 15594496 | 1194986 |
| Complete genome (Accession number NC_001318) | glucosamine-6-phosphate deaminase | 1522 06 | 1530 12 | + | | 15594497 | 1194987 |
| Complete genome (Accession number NC_001318) | superoxide dismutase (sodA) | 1530 75 | 1537 19 | - | | 15594498 | 1194988 |
| Complete genome (Accession number NC_001318) | preprotein translocase subunit SecA | 1537 01 | 1564 00 | - | | 15594499 | 1194989 |
| Complete genome (Accession number NC_001318) | hypothetical protein BB0155 | 1564 62 | 1575 98 | + | | 15594500 | 1194990 |
| Complete genome (Accession number NC_001318) | hypothetical protein BB0156 | 1576 27 | 1580 61 | + | | 15594501 | 1194991 |
| Complete genome (Accession number NC_001318) | hypothetical protein BB0157 | 1580 58 | 1584 74 | + | | 15594502 | 1194992 |

| | | | | | | | |
|---|---|---|---|---|---|---|---|
| Complete genome (Accession number NC_001318) | antigen, S2, putative | 1586 28 | 1593 44 | + | | 15594503 | 1194993 |
| Complete genome (Accession number NC_001318) | hypothetical protein BB0159 | 1593 68 | 1600 42 | + | | 15594504 | 1194994 |
| Complete genome (Accession number NC_001318) | alanine racemase (alr) | 1600 90 | 1612 08 | + | | 15594505 | 1194995 |
| Complete genome (Accession number NC_001318) | hypothetical protein BB0161 | 1612 00 | 1628 64 | - | | 15594506 | 1194996 |
| Complete genome (Accession number NC_001318) | hypothetical protein BB0162 | 1628 78 | 1630 45 | + | | 15594507 | 1194997 |
| Complete genome (Accession number NC_001318) | hypothetical protein BB0163 | 1630 72 | 1648 20 | - | | 15594508 | 1194998 |
| Complete genome (Accession number NC_001318) | Na+/Ca+ exchange protein, putative | 1649 01 | 1659 14 | - | | 15594509 | 1194999 |
| Complete genome (Accession number NC_001318) | hypothetical protein BB0165 | 1659 15 | 1677 59 | - | | 15594510 | 1195000 |
| Complete genome (Accession number NC_001318) | 4-alpha-glucanotransferas e (malQ) | 1678 79 | 1693 99 | - | | 15594511 | 1195001 |
| Complete genome (Accession number NC_001318) | outer membrane protein (tpn50) | 1695 39 | 1707 08 | + | | 15594512 | 1195002 |
| Complete genome (Accession number NC_001318) | dnaK suppressor, putative | 1707 20 | 1710 97 | - | | 15594513 | 1195003 |
| Complete genome (Accession number NC_001318) | translation initiation factor IF-1 | 1712 12 | 1714 84 | + | | 15594514 | 1195004 |

| | | | | | | | |
|---|---|---|---|---|---|---|---|
| Complete genome (Accession number NC_001318) | hypothetical protein BB0170 | 1715 32 | 1735 80 | - | | 15594515 | 1195005 |
| Complete genome (Accession number NC_001318) | hypothetical protein BB0171 | 1735 29 | 1741 16 | - | | 15594516 | 1195006 |
| Complete genome (Accession number NC_001318) | hypothetical protein BB0172 | 1740 98 | 1751 38 | - | | 15594517 | 1195007 |
| Complete genome (Accession number NC_001318) | hypothetical protein BB0173 | 1750 81 | 1761 06 | - | | 15594518 | 1195008 |
| Complete genome (Accession number NC_001318) | hypothetical protein BB0174 | 1760 69 | 1769 74 | - | | 15594519 | 1195009 |
| Complete genome (Accession number NC_001318) | hypothetical protein BB0175 | 1769 78 | 1778 53 | - | | 15594520 | 1195010 |
| Complete genome (Accession number NC_001318) | methanol dehydrogenase regulator (moxR) | 1778 68 | 1788 81 | - | | 15594521 | 1195011 |
| Complete genome (Accession number NC_001318) | glucose inhibited division protein B (gidB) | 1789 17 | 1795 43 | - | | 15594522 | 1195012 |
| Complete genome (Accession number NC_001318) | tRNA uridine 5-carboxymethylami nomethyl modification enzyme GidA | 1795 40 | 1814 23 | - | | 15594523 | 1195013 |
| Complete genome (Accession number NC_001318) | tRNA modification GTPase TrmE | 1814 08 | 1828 02 | - | | 15594524 | 1195014 |

| | | | | | | | |
|---|---|---|---|---|---|---|---|
| Complete genome (Accession number NC_001318) | flagellar protein, putative | 1828 86 | 1833 80 | + | | 15594525 | 1195015 |
| Complete genome (Accession number NC_001318) | flagellar hook-associated protein FlgK | 1833 92 | 1852 75 | + | | 15594526 | 1195016 |
| Complete genome (Accession number NC_001318) | flagellar hook-associated protein FlgL | 1852 78 | 1865 52 | + | | 15594527 | 1195017 |
| Complete genome (Accession number NC_001318) | flagellar assembly protein FliW | 1865 54 | 1869 46 | + | | 15594528 | 1195018 |
| Complete genome (Accession number NC_001318) | carbon storage regulator (csrA) | 1869 49 | 1871 94 | + | | 15594529 | 1195019 |
| Complete genome (Accession number NC_001318) | hypothetical protein BB0185 | 1871 78 | 1878 31 | - | | 15594530 | 1195020 |
| Complete genome (Accession number NC_001318) | hypothetical protein BB0186 | 1878 24 | 1882 37 | - | | 15594531 | 1195021 |
| Complete genome (Accession number NC_001318) | hypothetical protein BB0187 | 1882 34 | 1884 94 | - | | 15594532 | 1195022 |
| Complete genome (Accession number NC_001318) | 50S ribosomal protein L20 | 1887 08 | 1890 55 | - | | 15594533 | 1195023 |
| Complete genome (Accession number NC_001318) | 50S ribosomal protein L35 | 1890 76 | 1892 76 | - | | 15594534 | 1195024 |
| Complete genome (Accession number NC_001318) | translation initiation factor IF-3 | 1892 99 | 1898 59 | - | | 15594535 | 1195025 |
| Complete genome (Accession number NC_001318) | hypothetical protein BB0191 | 1899 49 | 1900 44 | + | | 15594536 | 1195026 |

| Complete genome (Accession number NC_001318) | hypothetical protein BB0192 | 1900 64 | 1907 17 | + | | 15594537 | 1195027 |
|---|---|---|---|---|---|---|---|
| Complete genome (Accession number NC_001318) | hypothetical protein BB0193 | 1907 19 | 1914 59 | + | | 15594538 | 1195028 |
| Complete genome (Accession number NC_001318) | hypothetical protein BB0194 | 1915 18 | 1923 27 | - | | 15594539 | 1195029 |
| Complete genome (Accession number NC_001318) | cell division control protein 27, putative | 1923 24 | 1934 63 | - | | 15594540 | 1195030 |
| Complete genome (Accession number NC_001318) | peptide chain release factor 1 | 1938 00 | 1948 73 | + | | 15594541 | 1195031 |
| Complete genome (Accession number NC_001318) | protoporphyrinoge n oxidase, putative | 1948 76 | 1957 36 | + | | 15594542 | 1195032 |
| Complete genome (Accession number NC_001318) | guanosine-3',5'-bis(diphosphate) 3'-pyrophosphohydr olase (spoT) | 1956 93 | 1976 96 | + | | 15594543 | 1195033 |
| Complete genome (Accession number NC_001318) | hypothetical protein BB0199 | 1976 86 | 1999 50 | + | | 15594544 | 1195034 |
| Complete genome (Accession number NC_001318) | D-alanine--D-alanine ligase (ddlA) | 1999 65 | 2010 35 | - | | 15594545 | 1195035 |

| | | | | | | | |
|---|---|---|---|---|---|---|---|
| Complete genome (Accession number NC_001318) | UDP-N-acetylmuramoylalanyl-D-glutamate--2,6-diaminopimelate ligase (murE) | 2010 52 | 2025 78 | - | | 15594546 | 1195036 |
| Complete genome (Accession number NC_001318) | hemolysin, putative | 2028 50 | 2040 88 | + | | 15594547 | 1195038 |
| Complete genome (Accession number NC_001318) | Lambda CII stability-governing protein (hflK) | 2041 37 | 2050 72 | + | | 15594548 | 1195039 |
| Complete genome (Accession number NC_001318) | Lambda CII stability-governing protein (hflC) | 2050 73 | 2060 44 | + | | 15594549 | 1195040 |
| Complete genome (Accession number NC_001318) | hypothetical protein BB0205 | 2065 29 | 2081 06 | + | | 15594550 | 1195042 |
| Complete genome (Accession number NC_001318) | hypothetical protein BB0206 | 2080 84 | 2086 53 | - | | 15594551 | 1195043 |
| Complete genome (Accession number NC_001318) | UTP--glucose-1-phosphate uridylyltransferase (gtaB) | 2087 06 | 2095 42 | + | | 15594552 | 1195044 |
| Complete genome (Accession number NC_001318) | hypothetical protein BB0208 | 2095 29 | 2112 74 | + | | 15594553 | 1195045 |
| Complete genome (Accession number NC_001318) | hypothetical protein BB0209 | 2113 01 | 2120 68 | + | | 15594554 | 1195046 |

| | | | | | | | |
|---|---|---|---|---|---|---|---|
| Complete genome (Accession number NC_001318) | surface-located membrane protein 1 (lmp1) | 2120 61 | 2154 20 | + | | 15594555 | 1195047 |
| Complete genome (Accession number NC_001318) | DNA mismatch repair protein (mutL) | 2154 27 | 2172 59 | + | | 15594556 | 1195048 |
| Complete genome (Accession number NC_001318) | hypothetical protein BB0212 | 2172 78 | 2183 12 | + | | 15594557 | 1195049 |
| Complete genome (Accession number NC_001318) | hypothetical protein BB0213 | 2184 25 | 2190 78 | - | | 15594558 | 1195050 |
| Complete genome (Accession number NC_001318) | translation elongation factor P (efp) | 2190 56 | 2196 34 | - | | 15594559 | 1195051 |
| Complete genome (Accession number NC_001318) | phosphate ABC transporter, periplasmic phosphate-binding protein (pstS) | 2197 97 | 2206 39 | + | | 15594560 | 1195052 |
| Complete genome (Accession number NC_001318) | phosphate ABC transporter, permease protein (pstC) | 2207 29 | 2216 37 | + | | 15594561 | 1195053 |
| Complete genome (Accession number NC_001318) | phosphate ABC transporter, permease protein (pstA) | 2221 92 | 2231 78 | + | | 15594562 | 1195054 |

| | | | | | | | |
|---|---|---|---|---|---|---|---|
| Complete genome (Accession number NC_001318) | phosphate ABC transporter, ATP-binding protein (pstB) | 2231 79 | 2239 61 | + | | 15594563 | 1195055 |
| Complete genome (Accession number NC_001318) | gufA protein | 2239 64 | 2247 85 | - | | 15594564 | 1195056 |
| Complete genome (Accession number NC_001318) | alanyl-tRNA synthetase | 2248 26 | 2266 10 | - | | 15594565 | 1195057 |
| Complete genome (Accession number NC_001318) | flagellar motor switch protein (fliG-1) | 2266 10 | 2278 36 | - | | 15594566 | 1195058 |
| Complete genome (Accession number NC_001318) | glucose-6-phosphate 1-dehydrogenase, putative | 2278 14 | 2285 21 | - | | 15594567 | 1195059 |
| Complete genome (Accession number NC_001318) | hypothetical protein BB0223 | 2285 42 | 2288 95 | - | | 15594568 | 1195060 |
| Complete genome (Accession number NC_001318) | hypothetical protein BB0224 | 2288 64 | 2291 48 | - | | 15594569 | 1195061 |
| Complete genome (Accession number NC_001318) | tRNA-dihydrouridine synthase A | 2292 17 | 2302 24 | + | | 15594570 | 1195062 |
| Complete genome (Accession number NC_001318) | seryl-tRNA synthetase | 2302 14 | 2314 91 | - | | 15594571 | 1195063 |
| Complete genome (Accession number NC_001318) | hypothetical protein BB0227 | 2316 21 | 2323 22 | + | | 15594572 | 1195064 |
| Complete genome (Accession number NC_001318) | hypothetical protein BB0228 | 2323 38 | 2352 53 | + | | 15594573 | 1195065 |

| | | | | | | | |
|---|---|---|---|---|---|---|---|
| Complete genome (Accession number NC_001318) | 50S ribosomal protein L31 type B | 2352 89 | 2355 34 | - | | 15594574 | 1195066 |
| Complete genome (Accession number NC_001318) | transcription termination factor Rho | 2355 95 | 2371 42 | - | | 15594575 | 1195067 |
| Complete genome (Accession number NC_001318) | hypothetical protein BB0231 | 2372 22 | 2376 29 | - | | 15594576 | 1195068 |
| Complete genome (Accession number NC_001318) | hbbU protein | 2376 30 | 2379 56 | - | | 15594577 | 1195069 |
| Complete genome (Accession number NC_001318) | 30S ribosomal protein S20 | 2379 69 | 2382 26 | - | | 1,62E+08 | 1195070 |
| Complete genome (Accession number NC_001318) | hypothetical protein BB0234 | 2383 01 | 2391 28 | - | | 15594579 | 1195071 |
| Complete genome (Accession number NC_001318) | translation-associated GTPase | 2391 44 | 2402 50 | - | | 15594580 | 1195072 |
| Complete genome (Accession number NC_001318) | hypothetical protein BB0236 | 2402 83 | 2422 89 | - | | 15594581 | 1195073 |
| Complete genome (Accession number NC_001318) | apolipoprotein N-acyltransferase | 2424 38 | 2440 03 | + | | 15594582 | 1195074 |
| Complete genome (Accession number NC_001318) | hypothetical protein BB0238 | 2439 43 | 2447 31 | - | | 15594583 | 1195075 |
| Complete genome (Accession number NC_001318) | deoxyguanosine/deoxyadenosine kinase(I) subunit 2 (dck) | 2447 77 | 2453 94 | - | | 15594584 | 1195076 |

| | | | | | | | |
|---|---|---|---|---|---|---|---|
| Complete genome (Accession number NC_001318) | glycerol uptake facilitator (glpF) | 2457 90 | 2465 54 | + | | 15594585 | 1195077 |
| Complete genome (Accession number NC_001318) | glycerol kinase (glpK) | 2465 97 | 2481 02 | + | | 15594586 | 1195078 |
| Complete genome (Accession number NC_001318) | hypothetical protein BB0242 | 2481 56 | 2484 34 | + | | 15594587 | 1195079 |
| Complete genome (Accession number NC_001318) | glycerol-3-phosphate dehydrogenase, anaerobic (glpA) | 2485 06 | 2500 89 | + | | 15594588 | 1195080 |
| Complete genome (Accession number NC_001318) | hypothetical protein BB0244 | 2501 76 | 2506 70 | - | | 15594589 | 1195081 |
| Complete genome (Accession number NC_001318) | hypothetical protein BB0245 | 2506 57 | 2512 11 | - | | 15594590 | 1195082 |
| Complete genome (Accession number NC_001318) | hypothetical protein BB0246 | 2512 12 | 2522 37 | - | | 15594591 | 1195083 |
| Complete genome (Accession number NC_001318) | hypothetical protein BB0247 | 2524 34 | 2530 39 | + | | 15594592 | 1195084 |
| Complete genome (Accession number NC_001318) | oligoendopeptidase F (pepF) | 2531 59 | 2549 31 | + | | 15594593 | 1195085 |
| Complete genome (Accession number NC_001318) | phosphatidyltransferase | 2549 45 | 2556 49 | + | | 15594594 | 1195086 |
| Complete genome (Accession number NC_001318) | dedA protein (dedA) | 2556 46 | 2562 60 | + | | 15594595 | 1195087 |
| Complete genome (Accession number NC_001318) | leucyl-tRNA synthetase | 2564 63 | 2589 85 | + | | 15594596 | 1195088 |

| | | | | | | | |
|---|---|---|---|---|---|---|---|
| Complete genome (Accession number NC_001318) | hypothetical protein BB0252 | 2590 00 | 2613 03 | + | | 15594597 | 1195089 |
| Complete genome (Accession number NC_001318) | ATP-dependent protease LA (lon-1) | 2612 92 | 2637 12 | - | | 15594598 | 1195090 |
| Complete genome (Accession number NC_001318) | single-stranded-DNA-specific exonuclease (recJ) | 2639 88 | 2661 14 | + | | 15594599 | 1195091 |
| Complete genome (Accession number NC_001318) | hypothetical protein BB0255 | 2660 95 | 2670 51 | + | | 15594600 | 1195092 |
| Complete genome (Accession number NC_001318) | 30S ribosomal protein S21 | 2670 66 | 2672 75 | + | | 15594601 | 1195093 |
| Complete genome (Accession number NC_001318) | cell division protein, putative | 2673 13 | 2696 76 | - | | 15594602 | 1195094 |
| Complete genome (Accession number NC_001318) | undecaprenyl pyrophosphate phosphatase | 2696 73 | 2704 73 | - | | 15594603 | 1195095 |
| Complete genome (Accession number NC_001318) | hypothetical protein BB0259 | 2704 88 | 2727 01 | - | | 15594604 | 1195096 |
| Complete genome (Accession number NC_001318) | hypothetical protein BB0260 | 2726 34 | 2736 47 | - | | 15594605 | 1195097 |
| Complete genome (Accession number NC_001318) | hypothetical protein BB0261 | 2738 54 | 2752 36 | + | | 15594606 | 1195098 |
| Complete genome (Accession number NC_001318) | hypothetical protein BB0262 | 2752 43 | 2764 96 | - | | 15594607 | 1195099 |

| Complete genome (Accession number NC_001318) | signal peptidase I (lepB-3) | 2765 58 | 2770 64 | + | | 15594608 | 1195100 |
|---|---|---|---|---|---|---|---|
| Complete genome (Accession number NC_001318) | heat shock protein 70 (dnaK-1) | 2770 90 | 2785 65 | - | | 15594609 | 1195101 |
| Complete genome (Accession number NC_001318) | hypothetical protein BB0265 | 2785 37 | 2790 58 | - | | 15594610 | 1195102 |
| Complete genome (Accession number NC_001318) | hypothetical protein BB0266 | 2790 78 | 2793 86 | - | | 15594611 | 1195103 |
| Complete genome (Accession number NC_001318) | hypothetical protein BB0267 | 2794 07 | 2813 11 | - | | 15594612 | 1195104 |
| Complete genome (Accession number NC_001318) | hypothetical protein BB0268 | 2813 14 | 2817 93 | - | | 15594613 | 1195105 |
| Complete genome (Accession number NC_001318) | ATP-binding protein (ylxH-1) | 2818 00 | 2826 87 | - | | 15594614 | 1195106 |
| Complete genome (Accession number NC_001318) | flagellar biosynthesis regulator FlhF | 2826 99 | 2838 65 | - | | 15594615 | 1195107 |
| Complete genome (Accession number NC_001318) | flagellar biosynthesis protein A | 2838 69 | 2859 62 | - | | 15594616 | 1195108 |
| Complete genome (Accession number NC_001318) | flagellar biosynthesis protein FlhB | 2859 71 | 2870 89 | - | | 15594617 | 1195109 |
| Complete genome (Accession number NC_001318) | flagellar biosynthesis protein (fliR) | 2870 89 | 2878 98 | - | | 15594618 | 1195110 |

| Complete genome (Accession number NC_001318) | flagellar biosynthesis protein (fliQ) | 2879 10 | 2881 73 | - | | 15594619 | 1195111 |
|---|---|---|---|---|---|---|---|
| Complete genome (Accession number NC_001318) | flagellar biosynthesis protein FliP | 2881 82 | 2889 46 | - | | 15594620 | 1195112 |
| Complete genome (Accession number NC_001318) | flagellar biosynthesis protein (fliZ) | 2889 57 | 2895 83 | - | | 15594621 | 1195113 |
| Complete genome (Accession number NC_001318) | flagellar motor switch protein (fliN) | 2895 76 | 2899 17 | - | | 15594622 | 1195114 |
| Complete genome (Accession number NC_001318) | flagellar motor switch protein FliM | 2899 63 | 2910 21 | - | | 15594623 | 1195115 |
| Complete genome (Accession number NC_001318) | flagellar basal body-associated protein FliL | 2910 59 | 2915 95 | - | | 15594624 | 1195116 |
| Complete genome (Accession number NC_001318) | flagellar motor protein MotB | 2916 44 | 2924 26 | - | | 15594625 | 1195117 |
| Complete genome (Accession number NC_001318) | flagellar motor rotation protein A (motA) | 2924 26 | 2932 08 | - | | 15594626 | 1195118 |
| Complete genome (Accession number NC_001318) | flagellar protein (flbD) | 2932 05 | 2934 29 | - | | 15594627 | 1195119 |
| Complete genome (Accession number NC_001318) | flagellar hook protein FlgE | 2934 52 | 2947 80 | - | | 15594628 | 1195120 |

| | | | | | | | |
|---|---|---|---|---|---|---|---|
| Complete genome (Accession number NC_001318) | flagellar basal body rod modification protein | 2947 85 | 2952 28 | - | | 15594629 | 1195121 |
| Complete genome (Accession number NC_001318) | flagellar protein (flbC) | 2952 42 | 2964 26 | - | | 15594630 | 1195122 |
| Complete genome (Accession number NC_001318) | flagellar protein (flbB) | 2964 28 | 2970 51 | - | | 15594631 | 1195123 |
| Complete genome (Accession number NC_001318) | flagellar protein (flbA) | 2970 38 | 2974 69 | - | | 15594632 | 1195124 |
| Complete genome (Accession number NC_001318) | flagellum-specific ATP synthase (fliI) | 2974 66 | 2987 76 | - | | 15594633 | 1195125 |
| Complete genome (Accession number NC_001318) | flagellar assembly protein H | 2987 95 | 2997 15 | - | | 15594634 | 1195126 |
| Complete genome (Accession number NC_001318) | flagellar motor switch protein G | 2997 30 | 3007 64 | - | | 15594635 | 1195127 |
| Complete genome (Accession number NC_001318) | flagellar MS-ring protein | 3007 80 | 3024 89 | - | | 15594636 | 1195128 |
| Complete genome (Accession number NC_001318) | flagellar hook-basal body protein FliE | 3025 04 | 3028 39 | - | | 15594637 | 1195129 |
| Complete genome (Accession number NC_001318) | flagellar basal body rod protein FlgC | 3028 51 | 3033 09 | - | | 15594638 | 1195130 |
| Complete genome (Accession number NC_001318) | flagellar basal body rod protein FlgB | 3033 33 | 3037 40 | - | | 15594639 | 1195131 |

302

| | | | | | | | |
|---|---|---|---|---|---|---|---|
| Complete genome (Accession number NC_001318) | ATP-dependent protease ATP-binding subunit | 3037 74 | 3051 41 | - | | 15594640 | 1195132 |
| Complete genome (Accession number NC_001318) | ATP-dependent protease peptidase subunit | 3051 13 | 3056 61 | - | | 15594641 | 1195133 |
| Complete genome (Accession number NC_001318) | smg protein | 3056 71 | 3066 18 | - | | 15594642 | 1195134 |
| Complete genome (Accession number NC_001318) | hypothetical protein BB0298 | 3066 25 | 3073 17 | - | | 15594643 | 1195135 |
| Complete genome (Accession number NC_001318) | cell division protein FtsZ | 3073 18 | 3085 32 | - | | 15594644 | 1195136 |
| Complete genome (Accession number NC_001318) | cell division protein (ftsA) | 3085 39 | 3097 80 | - | | 15594645 | 1195137 |
| Complete genome (Accession number NC_001318) | cell division protein (divIB) | 3097 80 | 3105 23 | - | | 15594646 | 1195138 |
| Complete genome (Accession number NC_001318) | cell division protein (ftsW) | 3105 59 | 3116 53 | - | | 15594647 | 1195139 |
| Complete genome (Accession number NC_001318) | phospho-N-acetylmuramoyl-pentapeptide-transferase (mraY) | 3116 60 | 3127 15 | - | | 15594648 | 1195140 |

| | | | | | | | |
|---|---|---|---|---|---|---|---|
| Complete genome (Accession number NC_001318) | UDP-N-acetylmuramoylal anyl-D-glutamyl-2,6-diaminopimelate--D-alanyl-D-alanine ligase (murF) | 3127 29 | 3141 23 | - | | 15594649 | 1195141 |
| Complete genome (Accession number NC_001318) | hypothetical protein BB0305 | 3141 46 | 3144 27 | - | | 15594650 | 1195142 |
| Complete genome (Accession number NC_001318) | hypothetical protein BB0306 | 3144 24 | 3153 14 | - | | 15594651 | 1195143 |
| Complete genome (Accession number NC_001318) | hypothetical protein BB0307 | 3153 07 | 3161 43 | - | | 15594652 | 1195144 |
| Complete genome (Accession number NC_001318) | hypothetical protein BB0308 | 3161 40 | 3172 16 | - | | 15594653 | 1195145 |
| Complete genome (Accession number NC_001318) | hypothetical protein BB0309 | 3172 47 | 3180 26 | - | | 15594654 | 1195146 |
| Complete genome (Accession number NC_001318) | hypothetical protein BB0310 | 3181 19 | 3182 56 | - | | 15594655 | 1195147 |
| Complete genome (Accession number NC_001318) | hypothetical protein BB0311 | 3182 77 | 3191 58 | - | | 15594656 | 1195148 |
| Complete genome (Accession number NC_001318) | purine-binding chemotaxis protein (cheW-1) | 3191 06 | 3196 36 | - | | 15594657 | 1195149 |
| Complete genome (Accession number NC_001318) | cell division protein (ftsJ) | 3196 80 | 3202 58 | - | | 15594658 | 1195150 |

| Complete genome (Accession number NC_001318) | octaprenyl-diphosphate synthase (ispB) | 3203 09 | 3213 52 | + | | 15594659 | 1195151 |
|---|---|---|---|---|---|---|---|
| Complete genome (Accession number NC_001318) | hypothetical protein BB0315 | 3213 52 | 3220 38 | + | | 15594660 | 1195152 |
| Complete genome (Accession number NC_001318) | hypothetical protein BB0316 | 3220 40 | 3228 46 | - | | 15594661 | 1195153 |
| Complete genome (Accession number NC_001318) | hypothetical protein BB0317 | 3228 47 | 3237 79 | - | | 15594662 | 1195154 |
| Complete genome (Accession number NC_001318) | methylgalactoside ABC transporter, ATP-binding protein (mglA) | 3237 76 | 3252 36 | - | | 15594663 | 1195155 |
| Complete genome (Accession number NC_001318) | exported protein (tpn38b) | 3252 36 | 3262 88 | - | | 15594664 | 1195156 |
| Complete genome (Accession number NC_001318) | hypothetical protein BB0320 | 3263 25 | 3264 32 | - | | 15594665 | 1195157 |
| Complete genome (Accession number NC_001318) | hypothetical protein BB0321 | 3264 69 | 3266 66 | - | | 15594666 | 1195158 |
| Complete genome (Accession number NC_001318) | hypothetical protein BB0322 | 3266 99 | 3277 57 | - | | 15594667 | 1195159 |
| Complete genome (Accession number NC_001318) | hypothetical protein BB0323 | 3280 32 | 3291 65 | + | | 15594668 | 1195160 |
| Complete genome (Accession number NC_001318) | hypothetical protein BB0324 | 3292 62 | 3296 21 | + | | 15594669 | 1195161 |

| Complete genome (Accession number NC_001318) | hypothetical protein BB0325 | 3296 43 | 3307 52 | - | | 15594670 | 1195162 |
|---|---|---|---|---|---|---|---|
| Complete genome (Accession number NC_001318) | hypothetical protein BB0326 | 3308 03 | 3335 98 | - | | 15594671 | 1195163 |
| Complete genome (Accession number NC_001318) | glycerol-3-phosphate O-acyltransferase, putative | 3336 19 | 3345 15 | - | | 15594672 | 1195164 |
| Complete genome (Accession number NC_001318) | oligopeptide ABC transporter, periplasmic oligopeptide-binding protein (oppA-1) | 3349 19 | 3365 08 | + | | 15594673 | 1195165 |
| Complete genome (Accession number NC_001318) | oligopeptide ABC transporter, periplasmic oligopeptide-binding protein (oppA-2) | 3366 29 | 3382 15 | + | | 15594674 | 1195166 |
| Complete genome (Accession number NC_001318) | oligopeptide ABC transporter, periplasmic oligopeptide-binding protein (oppA-3) | 3383 58 | 3399 83 | + | | 15594675 | 1195167 |
| Complete genome (Accession number NC_001318) | hypothetical protein BB0331 | 3400 62 | 3402 17 | - | | 15594676 | 1195168 |

| Complete genome (Accession number NC_001318) | oligopeptide ABC transporter, permease protein (oppB-1) | 3403 41 | 3412 61 | + | | 15594677 | 1195169 |
|---|---|---|---|---|---|---|---|
| Complete genome (Accession number NC_001318) | oligopeptide ABC transporter, permease protein (oppC-1) | 3412 74 | 3423 23 | + | | 15594678 | 1195170 |
| Complete genome (Accession number NC_001318) | oligopeptide ABC transporter, ATP-binding protein (oppD) | 3423 35 | 3432 07 | + | | 15594679 | 1195171 |
| Complete genome (Accession number NC_001318) | oligopeptide ABC transporter, ATP-binding protein (oppF) | 3432 08 | 3441 79 | + | | 15594680 | 1195172 |
| Complete genome (Accession number NC_001318) | P26 | 3441 92 | 3449 47 | - | | 15594681 | 1195173 |
| Complete genome (Accession number NC_001318) | enolase (eno) | 3450 63 | 3463 64 | + | | 15594682 | 1195174 |
| Complete genome (Accession number NC_001318) | 30S ribosomal protein S9 | 3464 31 | 3468 41 | - | | 15594683 | 1195175 |
| Complete genome (Accession number NC_001318) | 50S ribosomal protein L13 | 3468 61 | 3473 01 | - | | 15594684 | 1195176 |
| Complete genome (Accession number NC_001318) | hypothetical protein BB0340 | 3473 51 | 3481 60 | - | | 15594685 | 1195177 |

| | | | | | | | |
|---|---|---|---|---|---|---|---|
| Complete genome (Accession number NC_001318) | aspartyl/glutamyl-tRNA amidotransferase subunit B | 3481 53 | 3496 10 | - | | 15594686 | 1195178 |
| Complete genome (Accession number NC_001318) | glu-tRNA amidotransferase, subunit A (gluA) | 3496 00 | 3510 90 | - | | 15594687 | 1195179 |
| Complete genome (Accession number NC_001318) | aspartyl/glutamyl-tRNA amidotransferase subunit C | 3510 56 | 3513 31 | - | | 15594688 | 1195180 |
| Complete genome (Accession number NC_001318) | DNA helicase (uvrD) | 3513 41 | 3534 40 | - | | 15594689 | 1195181 |
| Complete genome (Accession number NC_001318) | hypothetical protein BB0345 | 3534 40 | 3546 36 | - | | 15594690 | 1195182 |
| Complete genome (Accession number NC_001318) | hypothetical protein BB0346 | 3546 48 | 3552 98 | - | | 15594691 | 1195183 |
| Complete genome (Accession number NC_001318) | fibronectin/fibrinogen-binding protein, putative | 3554 82 | 3569 09 | + | | 15594692 | 1195184 |
| Complete genome (Accession number NC_001318) | pyruvate kinase | 3570 07 | 3584 40 | + | | 15594693 | 1195185 |
| Complete genome (Accession number NC_001318) | hypothetical protein BB0349 | 3584 58 | 3591 98 | + | | 15594694 | 1195186 |
| Complete genome (Accession number NC_001318) | 50S ribosomal protein L28 | 3592 17 | 3594 95 | - | | 15594695 | 1195187 |
| Complete genome (Accession number NC_001318) | hypothetical protein BB0351 | 3595 13 | 3610 81 | - | | 15594696 | 1195188 |

| | | | | | | | |
|---|---|---|---|---|---|---|---|
| Complete genome (Accession number NC_001318) | hypothetical protein BB0352 | 3611 88 | 3623 21 | + | | 15594697 | 1195189 |
| Complete genome (Accession number NC_001318) | hypothetical protein BB0353 | 3623 18 | 3641 17 | - | | 15594698 | 1195190 |
| Complete genome (Accession number NC_001318) | hypothetical protein BB0354 | 3641 30 | 3651 70 | - | | 15594699 | 1195191 |
| Complete genome (Accession number NC_001318) | transcription factor, putative | 3651 15 | 3656 03 | - | | 15594700 | 1195192 |
| Complete genome (Accession number NC_001318) | hypothetical protein BB0356 | 3656 43 | 3661 37 | - | | 15594701 | 1195193 |
| Complete genome (Accession number NC_001318) | hypothetical protein BB0357 | 3661 44 | 3662 36 | - | | 15594702 | 1195194 |
| Complete genome (Accession number NC_001318) | hypothetical protein BB0358 | 3662 29 | 3669 60 | - | | 15594703 | 1195195 |
| Complete genome (Accession number NC_001318) | carboxyl-terminal protease (ctp) | 3669 64 | 3683 91 | - | | 15594704 | 1195196 |
| Complete genome (Accession number NC_001318) | hypothetical protein BB0360 | 3684 56 | 3688 81 | + | | 15594705 | 1195197 |
| Complete genome (Accession number NC_001318) | ATP-binding protein (ylxH-2) | 3688 85 | 3700 27 | + | | 15594706 | 1195198 |
| Complete genome (Accession number NC_001318) | prolipoprotein diacylglyceryl transferase | 3700 27 | 3710 13 | + | | 15594707 | 1195199 |
| Complete genome (Accession number NC_001318) | periplasmic protein | 3710 34 | 3730 46 | + | | 15594708 | 1195200 |
| Complete genome (Accession number NC_001318) | methylglyoxal synthase | 3731 40 | 3735 20 | + | | 15594709 | 1195201 |

| Complete genome (Accession number NC_001318) | lipoprotein LA7 | 3735 67 | 3741 51 | - | | 15594710 | 1195202 |
|---|---|---|---|---|---|---|---|
| Complete genome (Accession number NC_001318) | putative aminopeptidase 1 | 3742 77 | 3756 53 | + | | 15594711 | 1195203 |
| Complete genome (Accession number NC_001318) | hypothetical protein BB0367 | 3756 61 | 3759 39 | + | | 15594712 | 1195204 |
| Complete genome (Accession number NC_001318) | NAD(P)H-dependent glycerol-3-phosphate dehydrogenase | 3759 61 | 3770 52 | - | | 15594713 | 1195205 |
| Complete genome (Accession number NC_001318) | ATP-dependent Clp protease, subunit A (clpA) | 3770 39 | 3793 30 | - | | 15594714 | 1195206 |
| Complete genome (Accession number NC_001318) | tyrosyl-tRNA synthetase (tyrS) | 3793 41 | 3805 58 | - | | 15594715 | 1195207 |
| Complete genome (Accession number NC_001318) | glycyl-tRNA synthetase | 3805 55 | 3818 92 | - | | 15594716 | 1195208 |
| Complete genome (Accession number NC_001318) | glutamyl-tRNA synthetase | 3819 10 | 3833 82 | - | | 15594717 | 1195209 |
| Complete genome (Accession number NC_001318) | hypothetical protein BB0373 | 3833 95 | 3841 62 | - | | 15594718 | 1195210 |
| Complete genome (Accession number NC_001318) | hypothetical protein BB0374 | 3842 87 | 3854 26 | + | | 15594719 | 1195211 |
| Complete genome (Accession number NC_001318) | pfs protein (pfs-1) | 3854 49 | 3861 62 | + | | 15594720 | 1195212 |

EP 2 254 592 B1

| | | | | | | | |
|---|---|---|---|---|---|---|---|
| Complete genome (Accession number NC_001318) | S-adenosylmethionine synthetase | 3861 59 | 3873 37 | + | | 15594721 | 1195213 |
| Complete genome (Accession number NC_001318) | S-ribosylhomocysteinase | 3873 34 | 3878 07 | + | | 1,62E+08 | 1195214 |
| Complete genome (Accession number NC_001318) | hypothetical protein BB0378 | 3878 18 | 3884 80 | - | | 15594723 | 1195215 |
| Complete genome (Accession number NC_001318) | protein kinase C1 inhibitor (pkcI) | 3885 07 | 3889 56 | - | | 15594724 | 1195216 |
| Complete genome (Accession number NC_001318) | Mg2+ transport protein (mgtE) | 3889 77 | 3903 41 | - | | 15594725 | 1195217 |
| Complete genome (Accession number NC_001318) | hypothetical protein BB0381 | 3903 92 | 3918 19 | - | | 15594726 | 1195218 |
| Complete genome (Accession number NC_001318) | basic membrane protein B (bmpB) | 3919 32 | 3929 57 | - | | 15594727 | 1195219 |
| Complete genome (Accession number NC_001318) | basic membrane protein A (bmpA) | 3930 44 | 3940 63 | - | | 15594728 | 1195220 |
| Complete genome (Accession number NC_001318) | basic membrane protein C (bmpC) | 3941 05 | 3951 66 | - | | 15594729 | 1195221 |
| Complete genome (Accession number NC_001318) | basic membrane protein D (bmpD) | 3954 81 | 3965 63 | - | | 15594730 | 1195222 |
| Complete genome (Accession number NC_001318) | 30S ribosomal protein S7 | 3966 04 | 3970 77 | - | | 15594731 | 1195223 |

| Complete genome (Accession number NC_001318) | 30S ribosomal protein S12 | 3971 02 | 3974 76 | - | | 15594732 | 1195224 |
|---|---|---|---|---|---|---|---|
| Complete genome (Accession number NC_001318) | DNA-directed RNA polymerase subunit beta' | 3975 44 | 4016 77 | - | | 15594733 | 1195225 |
| Complete genome (Accession number NC_001318) | DNA-directed RNA polymerase subunit beta | 4016 93 | 4051 60 | - | | 15594734 | 1195226 |
| Complete genome (Accession number NC_001318) | 50S ribosomal protein L7/L12 | 4052 43 | 4056 17 | - | | 15594735 | 1195227 |
| Complete genome (Accession number NC_001318) | 50S ribosomal protein L10 | 4056 88 | 4061 94 | - | | 15594736 | 1195228 |
| Complete genome (Accession number NC_001318) | 50S ribosomal protein L1 | 4061 81 | 4068 61 | - | | 15594737 | 1195229 |
| Complete genome (Accession number NC_001318) | 50S ribosomal protein L11 | 4068 61 | 4072 92 | - | | 15594738 | 1195230 |
| Complete genome (Accession number NC_001318) | transcription antitermination factor (nusG) | 4073 44 | 4078 98 | - | | 15594739 | 1195231 |
| Complete genome (Accession number NC_001318) | preprotein translocase subunit SecE | 4079 22 | 4080 92 | - | | 15594740 | 1195232 |
| Complete genome (Accession number NC_001318) | 50S ribosomal protein L33 | 4082 01 | 4083 80 | - | | 15594741 | 1195234 |

| | | | | | | | |
|---|---|---|---|---|---|---|---|
| Complete genome (Accession number NC_001318) | hypothetical protein BB0397 | 4085 54 | 4094 02 | - | | 15594742 | 1195235 |
| Complete genome (Accession number NC_001318) | hypothetical protein BB0398 | 4094 16 | 4104 47 | - | | 15594743 | 1195236 |
| Complete genome (Accession number NC_001318) | hypothetical protein BB0399 | 4107 87 | 4114 46 | + | | 15594744 | 1195237 |
| Complete genome (Accession number NC_001318) | hypothetical protein BB0400 | 4114 56 | 4130 06 | - | | 15594745 | 1195238 |
| Complete genome (Accession number NC_001318) | glutamate transporter, putative | 4131 13 | 4143 15 | + | | 15594746 | 1195239 |
| Complete genome (Accession number NC_001318) | prolyl-tRNA synthetase | 4143 27 | 4157 93 | - | | 15594747 | 1195240 |
| Complete genome (Accession number NC_001318) | hypothetical protein BB0403 | 4158 46 | 4165 23 | - | | 15594748 | 1195241 |
| Complete genome (Accession number NC_001318) | hypothetical protein BB0404 | 4166 89 | 4171 05 | + | | 15594749 | 1195242 |
| Complete genome (Accession number NC_001318) | hypothetical protein BB0405 | 4171 02 | 4177 13 | + | | 15594750 | 1195243 |
| Complete genome (Accession number NC_001318) | hypothetical protein BB0406 | 4177 24 | 4183 35 | + | | 15594751 | 1195244 |
| Complete genome (Accession number NC_001318) | mannose-6-phosphate isomerase (manA) | 4183 88 | 4195 06 | - | | 15594752 | 1195245 |

| | | | | | | | |
|---|---|---|---|---|---|---|---|
| Complete genome (Accession number NC_001318) | PTS system, fructose-specific IIABC component (fruA-1) | 4195 03 | 4213 80 | - | | 15594753 | 1195246 |
| Complete genome (Accession number NC_001318) | hypothetical protein BB0409 | 4215 54 | 4221 83 | + | | 15594754 | 1195247 |
| Complete genome (Accession number NC_001318) | hypothetical protein BB0410 | 4221 80 | 4222 99 | + | | 15594755 | 1195248 |
| Complete genome (Accession number NC_001318) | endonuclease precursor (nucA) | 4224 71 | 4230 58 | + | | 15594756 | 1195249 |
| Complete genome (Accession number NC_001318) | hypothetical protein BB0412 | 4230 55 | 4238 34 | + | | 15594757 | 1195250 |
| Complete genome (Accession number NC_001318) | hypothetical protein BB0413 | 4238 22 | 4244 45 | - | | 15594758 | 1195251 |
| Complete genome (Accession number NC_001318) | chemotaxis protein methyltransferase (cheR-2) | 4246 94 | 4255 51 | + | | 15594759 | 1195252 |
| Complete genome (Accession number NC_001318) | protein-glutamate methylesterase (cheB-1) | 4255 84 | 4267 11 | + | | 15594760 | 1195253 |
| Complete genome (Accession number NC_001318) | pheromone shutdown protein (traB) | 4267 36 | 4279 50 | + | | 15594761 | 1195254 |
| Complete genome (Accession number NC_001318) | adenylate kinase (adk) | 4279 87 | 4286 22 | + | | 15594762 | 1195255 |
| Complete genome (Accession number NC_001318) | hypothetical protein BB0418 | 4286 46 | 4296 74 | + | | 15594763 | 1195256 |

| | | | | | | | |
|---|---|---|---|---|---|---|---|
| Complete genome (Accession number NC_001318) | response regulatory protein (rrp-1) | 4296 93 | 4306 16 | - | | 15594764 | 1195257 |
| Complete genome (Accession number NC_001318) | sensory transduction histidine kinase, putative | 4306 19 | 4351 03 | - | | 15594765 | 1195258 |
| Complete genome (Accession number NC_001318) | hydrolase | 4417 13 | 4425 52 | - | | 15594766 | 1195263 |
| Complete genome (Accession number NC_001318) | 3-methyladenine DNA glycosylase (mag) | 4426 88 | 4432 48 | - | | 15594767 | 1195264 |
| Complete genome (Accession number NC_001318) | hypothetical protein BB0423 | 4437 63 | 4440 53 | - | | 15594768 | 1195266 |
| Complete genome (Accession number NC_001318) | hypothetical protein BB0424 | 4441 50 | 4442 99 | + | | 15594769 | 1195267 |
| Complete genome (Accession number NC_001318) | hypothetical protein BB0425 | 4461 13 | 4462 05 | - | | 15594770 | 1195270 |
| Complete genome (Accession number NC_001318) | hypothetical protein BB0426 | 4463 27 | 4468 90 | - | | 15594771 | 1195271 |
| Complete genome (Accession number NC_001318) | hypothetical protein BB0427 | 4469 69 | 4477 60 | - | | 15594772 | 1195272 |
| Complete genome (Accession number NC_001318) | hypothetical protein BB0428 | 4478 55 | 4481 72 | + | | 15594773 | 1195273 |
| Complete genome (Accession number NC_001318) | hypothetical protein BB0429 | 4482 02 | 4485 91 | - | | 15594774 | 1195274 |
| Complete genome (Accession number NC_001318) | proline dipeptidase (pepQ) | 4487 05 | 4493 10 | + | | 15594775 | 1195275 |

| | | | | | | | |
|---|---|---|---|---|---|---|---|
| Complete genome (Accession number NC_001318) | hypothetical protein BB0431 | 4494 19 | 4501 71 | + | | 15594776 | 1195276 |
| Complete genome (Accession number NC_001318) | hypothetical protein BB0432 | 4501 74 | 4508 87 | + | | 15594777 | 1195277 |
| Complete genome (Accession number NC_001318) | hypothetical protein BB0433 | 4509 72 | 4510 70 | - | | 15594778 | 1195278 |
| Complete genome (Accession number NC_001318) | stage 0 sporulation protein J (spo0J) | 4511 39 | 4519 21 | - | | 15594779 | 1195279 |
| Complete genome (Accession number NC_001318) | DNA gyrase, subunit A (gyrA) | 4520 40 | 4544 72 | - | | 15594780 | 1195280 |
| Complete genome (Accession number NC_001318) | DNA gyrase, subunit B (gyrB) | 4544 84 | 4564 03 | - | | 15594781 | 1195281 |
| Complete genome (Accession number NC_001318) | chromosomal replication initiator protein (dnaA) | 4565 76 | 4580 36 | + | | 15594782 | 1195282 |
| Complete genome (Accession number NC_001318) | DNA polymerase III, subunit beta (dnaN) | 4582 77 | 4594 34 | + | | 15594783 | 1195283 |
| Complete genome (Accession number NC_001318) | hypothetical protein BB0439 | 4595 25 | 4598 24 | + | | 15594784 | 1195284 |
| Complete genome (Accession number NC_001318) | 50S ribosomal protein L34 | 4599 16 | 4600 71 | + | | 15594785 | 1195285 |
| Complete genome (Accession number NC_001318) | ribonuclease P protein component (rnpA) | 4600 52 | 4604 11 | + | | 15594786 | 1195286 |

| | | | | | | | |
|---|---|---|---|---|---|---|---|
| Complete genome (Accession number NC_001318) | putative inner membrane protein translocase component YidC | 4603 98 | 4620 32 | + | | 15594787 | 1195287 |
| Complete genome (Accession number NC_001318) | spoIIIJ-associtated protein (jag) | 4620 45 | 4627 73 | + | | 15594788 | 1195288 |
| Complete genome (Accession number NC_001318) | nucleotide sugar epimerase | 4628 19 | 4638 86 | + | | 15594789 | 1195289 |
| Complete genome (Accession number NC_001318) | fructose-bisphosphate aldolase | 4640 53 | 4651 32 | + | | 15594790 | 1195290 |
| Complete genome (Accession number NC_001318) | aspartyl-tRNA synthetase | 4655 18 | 4672 78 | - | | 1,62E+08 | 1195291 |
| Complete genome (Accession number NC_001318) | Na+/H+ antiporter (napA) | 4672 82 | 4693 87 | - | | 15594792 | 1195292 |
| Complete genome (Accession number NC_001318) | phosphocarrier protein HPr (ptsH-1) | 4693 90 | 4696 65 | - | | 15594793 | 1195293 |
| Complete genome (Accession number NC_001318) | hypothetical protein BB0449 | 4696 78 | 4699 71 | - | | 15594794 | 1195294 |
| Complete genome (Accession number NC_001318) | RNA polymerase sigma-54 factor (ntrA) | 4699 61 | 4712 20 | - | | 15594795 | 1195295 |
| Complete genome (Accession number NC_001318) | chromate transport protein, putative | 4712 23 | 4717 56 | - | | 15594796 | 1195296 |
| Complete genome (Accession number NC_001318) | hypothetical protein BB0452 | 4717 71 | 4724 75 | - | | 15594797 | 1195297 |

| | | | | | | | |
|---|---|---|---|---|---|---|---|
| Complete genome (Accession number NC_001318) | hypothetical protein BB0453 | 4725 66 | 4734 08 | + | | 15594798 | 1195298 |
| Complete genome (Accession number NC_001318) | lipopolysaccharide biosynthesis-related protein | 4734 05 | 4745 56 | - | | 15594799 | 1195299 |
| Complete genome (Accession number NC_001318) | hypothetical protein BB0455 | 4746 13 | 4756 02 | + | | 15594800 | 1195300 |
| Complete genome (Accession number NC_001318) | hypothetical protein BB0456 | 4756 06 | 4762 11 | + | | 15594801 | 1195301 |
| Complete genome (Accession number NC_001318) | excinuclease ABC, subunit C (uvrC) | 4762 63 | 4780 74 | - | | 15594802 | 1195302 |
| Complete genome (Accession number NC_001318) | hypothetical protein BB0458 | 4781 39 | 4797 16 | + | | 15594803 | 1195303 |
| Complete genome (Accession number NC_001318) | hypothetical protein BB0459 | 4797 09 | 4810 19 | + | | 15594804 | 1195304 |
| Complete genome (Accession number NC_001318) | hypothetical protein BB0460 | 4813 44 | 4820 57 | - | | 15594805 | 1195305 |
| Complete genome (Accession number NC_001318) | DNA polymerase III subunits gamma and tau | 4826 00 | 4842 82 | + | | 15594806 | 1195307 |
| Complete genome (Accession number NC_001318) | hypothetical protein BB0462 | 4842 54 | 4845 86 | + | | 15594807 | 1195308 |
| Complete genome (Accession number NC_001318) | nucleoside-diphosphate kinase (ndk) | 4847 57 | 4852 66 | + | | 15594808 | 1195309 |

| | | | | | | | |
|---|---|---|---|---|---|---|---|
| Complete genome (Accession number NC_001318) | hypothetical protein BB0464 | 4855 50 | 4860 77 | + | | 15594809 | 1195310 |
| Complete genome (Accession number NC_001318) | hypothetical protein BB0465 | 4860 74 | 4867 69 | + | | 15594810 | 1195311 |
| Complete genome (Accession number NC_001318) | ABC transporter, ATP-binding protein | 4867 05 | 4875 38 | + | | 15594811 | 1195312 |
| Complete genome (Accession number NC_001318) | hypothetical protein BB0467 | 4875 35 | 4882 24 | + | | 15594812 | 1195313 |
| Complete genome (Accession number NC_001318) | hypothetical protein BB0468 | 4882 26 | 4889 72 | + | | 15594813 | 1195314 |
| Complete genome (Accession number NC_001318) | signal peptidase II | 4889 83 | 4894 95 | + | | 15594814 | 1195315 |
| Complete genome (Accession number NC_001318) | hypothetical protein BB0470 | 4894 92 | 4897 19 | + | | 15594815 | 1195316 |
| Complete genome (Accession number NC_001318) | hypothetical protein BB0471 | 4897 33 | 4905 54 | + | | 15594816 | 1195317 |
| Complete genome (Accession number NC_001318) | UDP-N-acetylglucosamine 1-carboxyvinyltransf erase | 4905 99 | 4919 27 | + | | 15594817 | 1195318 |
| Complete genome (Accession number NC_001318) | hypothetical protein BB0473 | 4925 88 | 4939 52 | + | | 15594818 | 1195319 |
| Complete genome (Accession number NC_001318) | hypothetical protein BB0474 | 4939 91 | 4940 92 | + | | 15594819 | 1195320 |

| | | | | | | | |
|---|---|---|---|---|---|---|---|
| Complete genome (Accession number NC_001318) | hypothetical protein BB0475 | 4941 50 | 4945 42 | - | | 15594820 | 1195321 |
| Complete genome (Accession number NC_001318) | elongation factor Tu | 4950 33 | 4962 17 | + | | 1,62E+08 | 1195322 |
| Complete genome (Accession number NC_001318) | 30S ribosomal protein S10 | 4962 68 | 4965 79 | + | | 15594822 | 1195323 |
| Complete genome (Accession number NC_001318) | 50S ribosomal protein L3 | 4966 15 | 4972 35 | + | | 15594823 | 1195324 |
| Complete genome (Accession number NC_001318) | 50S ribosomal protein L4 | 4972 45 | 4978 74 | + | | 15594824 | 1195325 |
| Complete genome (Accession number NC_001318) | 50S ribosomal protein L23 | 4978 80 | 4981 91 | + | | 15594825 | 1195326 |
| Complete genome (Accession number NC_001318) | 50S ribosomal protein L2 | 4982 13 | 4990 46 | + | | 15594826 | 1195327 |
| Complete genome (Accession number NC_001318) | 30S ribosomal protein S19 | 4990 56 | 4993 34 | + | | 15594827 | 1195328 |
| Complete genome (Accession number NC_001318) | 50S ribosomal protein L22 | 4993 41 | 4997 03 | + | | 15594828 | 1195329 |
| Complete genome (Accession number NC_001318) | 30S ribosomal protein S3 | 4997 07 | 5005 88 | + | | 15594829 | 1195330 |
| Complete genome (Accession number NC_001318) | 50S ribosomal protein L16 | 5005 93 | 5010 09 | + | | 15594830 | 1195331 |
| Complete genome (Accession number NC_001318) | 50S ribosomal protein L29 | 5010 12 | 5012 12 | + | | 15594831 | 1195332 |
| Complete genome (Accession number NC_001318) | 30S ribosomal protein S17 | 5012 15 | 5014 69 | + | | 15594832 | 1195333 |

| | | | | | | | |
|---|---|---|---|---|---|---|---|
| Complete genome (Accession number NC_001318) | 50S ribosomal protein L14 | 5014 97 | 5018 65 | + | | 1,62E+08 | 1195334 |
| Complete genome (Accession number NC_001318) | 50S ribosomal protein L24 | 5018 80 | 5021 85 | + | | 15594834 | 1195335 |
| Complete genome (Accession number NC_001318) | 50S ribosomal protein L5 | 5021 91 | 5027 39 | + | | 15594835 | 1195336 |
| Complete genome (Accession number NC_001318) | 30S ribosomal protein S14 | 5027 56 | 5029 41 | + | | 1,62E+08 | 1195337 |
| Complete genome (Accession number NC_001318) | 30S ribosomal protein S8 | 5029 51 | 5033 49 | + | | 15594837 | 1195338 |
| Complete genome (Accession number NC_001318) | 50S ribosomal protein L6 | 5033 66 | 5039 08 | + | | 15594838 | 1195339 |
| Complete genome (Accession number NC_001318) | 50S ribosomal protein L18 | 5039 26 | 5042 85 | + | | 15594839 | 1195340 |
| Complete genome (Accession number NC_001318) | 30S ribosomal protein S5 | 5042 98 | 5047 95 | + | | 15594840 | 1195341 |
| Complete genome (Accession number NC_001318) | ribosomal protein L30 (rpmD) | 5047 99 | 5051 04 | + | | 15594841 | 1195342 |
| Complete genome (Accession number NC_001318) | 50S ribosomal protein L15 | 5051 04 | 5055 41 | + | | 15594842 | 1195343 |
| Complete genome (Accession number NC_001318) | preprotein translocase subunit SecY | 5055 54 | 5068 58 | + | | 15594843 | 1195344 |
| Complete genome (Accession number NC_001318) | 50S ribosomal protein L36 | 5068 65 | 5069 84 | + | | 15594844 | 1195345 |

| | | | | | | | |
|---|---|---|---|---|---|---|---|
| Complete genome (Accession number NC_001318) | 30S ribosomal protein S13 | 5070 02 | 5073 79 | + | | 15594845 | 1195346 |
| Complete genome (Accession number NC_001318) | 30S ribosomal protein S11 | 5073 94 | 5077 98 | + | | 15594846 | 1195347 |
| Complete genome (Accession number NC_001318) | DNA-directed RNA polymerase subunit alpha | 5078 11 | 5088 48 | + | | 15594847 | 1195348 |
| Complete genome (Accession number NC_001318) | 50S ribosomal protein L17 | 5088 70 | 5092 41 | + | | 15594848 | 1195349 |
| Complete genome (Accession number NC_001318) | hypothetical protein BB0504 | 5093 14 | 5108 46 | + | | 15594849 | 1195350 |
| Complete genome (Accession number NC_001318) | hypothetical protein BB0505 | 5108 36 | 5116 24 | + | | 15594850 | 1195351 |
| Complete genome (Accession number NC_001318) | hemolysin (tlyA) | 5116 05 | 5123 96 | + | | 15594851 | 1195352 |
| Complete genome (Accession number NC_001318) | hypothetical protein BB0507 | 5123 93 | 5131 48 | - | | 15594852 | 1195353 |
| Complete genome (Accession number NC_001318) | GTP-binding protein EngA | 5132 26 | 5145 27 | + | | 15594853 | 1195354 |
| Complete genome (Accession number NC_001318) | hypothetical protein BB0509 | 5145 24 | 5157 83 | + | | 15594854 | 1195355 |
| Complete genome (Accession number NC_001318) | hypothetical protein BB0510 | 5157 80 | 5159 98 | + | | 15594855 | 1195356 |
| Complete genome (Accession number NC_001318) | hypothetical protein BB0511 | 5160 90 | 5164 73 | + | | 15594856 | 1195357 |

| | | | | | | | |
|---|---|---|---|---|---|---|---|
| Complete genome (Accession number NC_001318) | hypothetical protein BB0512 | 5164 93 | 5229 93 | + | | 15594857 | 1195358 |
| Complete genome (Accession number NC_001318) | phenylalanyl-tRNA synthetase subunit alpha | 5229 90 | 5245 76 | + | | 15594858 | 1195359 |
| Complete genome (Accession number NC_001318) | phenylalanyl-tRNA synthetase subunit beta | 5245 54 | 5262 54 | + | | 15594859 | 1195360 |
| Complete genome (Accession number NC_001318) | thioredoxin reductase (trxB) | 5263 25 | 5273 05 | + | | 15594860 | 1195361 |
| Complete genome (Accession number NC_001318) | rRNA methylase (yacO) | 5273 61 | 5280 47 | - | | 15594861 | 1195362 |
| Complete genome (Accession number NC_001318) | heat shock protein (dnaJ-1) | 5281 04 | 5291 98 | - | | 15594862 | 1195363 |
| Complete genome (Accession number NC_001318) | molecular chaperone DnaK | 5291 98 | 5311 05 | - | | 15594863 | 1195364 |
| Complete genome (Accession number NC_001318) | grpE protein (grpE) | 5311 29 | 5316 92 | - | | 15594864 | 1195365 |
| Complete genome (Accession number NC_001318) | hypothetical protein BB0520 | 5318 51 | 5319 67 | - | | 15594865 | 1195366 |
| Complete genome (Accession number NC_001318) | hypothetical protein BB0521 | 5320 69 | 5325 21 | + | | 15594866 | 1195367 |
| Complete genome (Accession number NC_001318) | NH(3)-dependent NAD+ synthetase | 5329 38 | 5336 06 | + | | 15594867 | 1195368 |
| Complete genome (Accession number NC_001318) | hypothetical protein BB0523 | 5339 80 | 5340 72 | + | | 15594868 | 1195369 |

| Complete genome (Accession number NC_001318) | inositol monophosphatase | 5343 01 | 5351 55 | + | | 15594869 | 1195370 |
|---|---|---|---|---|---|---|---|
| Complete genome (Accession number NC_001318) | hypothetical protein BB0525 | 5351 78 | 5356 00 | - | | 15594870 | 1195371 |
| Complete genome (Accession number NC_001318) | hypothetical protein BB0526 | 5357 04 | 5375 27 | + | | 15594871 | 1195372 |
| Complete genome (Accession number NC_001318) | pantothenate kinase | 5375 20 | 5383 08 | + | | 15594872 | 1195373 |
| Complete genome (Accession number NC_001318) | aldose reductase, putative | 5383 28 | 5392 75 | - | | 15594873 | 1195374 |
| Complete genome (Accession number NC_001318) | hypothetical protein BB0529 | 5392 56 | 5393 72 | + | | 15594874 | 1195375 |
| Complete genome (Accession number NC_001318) | hypothetical protein BB0530 | 5393 67 | 5400 20 | - | | 15594875 | 1195376 |
| Complete genome (Accession number NC_001318) | hypothetical protein BB0531 | 5401 59 | 5407 73 | - | | 15594876 | 1195377 |
| Complete genome (Accession number NC_001318) | hypothetical protein BB0532 | 5411 82 | 5420 69 | - | | 15594877 | 1195378 |
| Complete genome (Accession number NC_001318) | phnP protein (phnP) | 5422 63 | 5430 24 | + | | 15594878 | 1195379 |
| Complete genome (Accession number NC_001318) | exodeoxyribonucl ease III (exoA) | 5430 49 | 5438 16 | + | | 15594879 | 1195380 |
| Complete genome (Accession number NC_001318) | hypothetical protein BB0535 | 5438 32 | 5446 05 | + | | 15594880 | 1195381 |
| Complete genome (Accession number NC_001318) | zinc protease, putative | 5445 86 | 5473 87 | + | | 15594881 | 1195382 |

| | | | | | | | |
|---|---|---|---|---|---|---|---|
| Complete genome (Accession number NC_001318) | hypothetical protein BB0537 | 5478 46 | 5484 90 | - | | 15594882 | 1195384 |
| Complete genome (Accession number NC_001318) | hypothetical protein BB0538 | 5484 87 | 5488 85 | - | | 15594883 | 1195385 |
| Complete genome (Accession number NC_001318) | hypothetical protein BB0539 | 5489 20 | 5496 18 | - | | 15594884 | 1195386 |
| Complete genome (Accession number NC_001318) | elongation factor G | 5496 42 | 5517 23 | - | | 15594885 | 1195387 |
| Complete genome (Accession number NC_001318) | hypothetical protein BB0541 | 5520 07 | 5522 25 | + | | 15594886 | 1195388 |
| Complete genome (Accession number NC_001318) | hypothetical protein BB0542 | 5524 40 | 5530 18 | + | | 15594887 | 1195389 |
| Complete genome (Accession number NC_001318) | hypothetical protein BB0543 | 5531 33 | 5537 89 | + | | 15594888 | 1195390 |
| Complete genome (Accession number NC_001318) | phosphoribosylpyr ophosphate synthetase | 5539 92 | 5552 12 | + | | 15594889 | 1195391 |
| Complete genome (Accession number NC_001318) | xylulokinase (xylB) | 5552 16 | 5565 80 | + | | 15594890 | 1195392 |
| Complete genome (Accession number NC_001318) | hypothetical protein BB0546 | 5565 63 | 5574 23 | - | | 15594891 | 1195393 |
| Complete genome (Accession number NC_001318) | dephospho-CoA kinase | 5574 07 | 5580 24 | - | | 15594892 | 1195394 |
| Complete genome (Accession number NC_001318) | DNA polymerase I (polA) | 5580 06 | 5607 32 | - | | 15594893 | 1195395 |
| Complete genome (Accession number NC_001318) | hypothetical protein BB0549 | 5607 29 | 5611 27 | - | | 15594894 | 1195396 |

| | | | | | | | |
|---|---|---|---|---|---|---|---|
| Complete genome (Accession number NC_001318) | flagellar protein (flaJ) | 5611 17 | 5615 54 | - | | 15594895 | 1195397 |
| Complete genome (Accession number NC_001318) | chemotaxis response regulator (cheY-1) | 5615 63 | 5619 64 | - | | 15594896 | 1195398 |
| Complete genome (Accession number NC_001318) | DNA ligase (lig) | 5621 85 | 5641 67 | + | | 15594897 | 1195399 |
| Complete genome (Accession number NC_001318) | hypothetical protein BB0553 | 5641 88 | 5656 81 | - | | 15594898 | 1195400 |
| Complete genome (Accession number NC_001318) | hypothetical protein BB0554 | 5659 43 | 5678 23 | + | | 15594899 | 1195401 |
| Complete genome (Accession number NC_001318) | hypothetical protein BB0555 | 5678 10 | 5682 77 | + | | 15594900 | 1195402 |
| Complete genome (Accession number NC_001318) | hypothetical protein BB0556 | 5682 70 | 5691 39 | + | | 15594901 | 1195403 |
| Complete genome (Accession number NC_001318) | phosphocarrier protein HPr (ptsH-2) | 5692 78 | 5695 38 | + | | 15594902 | 1195404 |
| Complete genome (Accession number NC_001318) | phosphoenolpyruvate-protein phosphatase (ptsI) | 5695 59 | 5712 80 | + | | 15594903 | 1195405 |
| Complete genome (Accession number NC_001318) | glucose-specific PTS system component | 5712 83 | 5718 52 | + | | 15594904 | 1195406 |
| Complete genome (Accession number NC_001318) | heat shock protein 90 | 5718 90 | 5738 42 | - | | 15594905 | 1195407 |

| Complete genome (Accession number NC_001318) | 6-phosphogluconate dehydrogenase | 5738 53 | 5752 47 | + | | 15594906 | 1195408 |
|---|---|---|---|---|---|---|---|
| Complete genome (Accession number NC_001318) | hypothetical protein BB0562 | 5752 74 | 5758 16 | - | | 15594907 | 1195409 |
| Complete genome (Accession number NC_001318) | hypothetical protein BB0563 | 5759 81 | 5765 26 | - | | 15594908 | 1195410 |
| Complete genome (Accession number NC_001318) | hypothetical protein BB0564 | 5765 82 | 5771 87 | - | | 15594909 | 1195411 |
| Complete genome (Accession number NC_001318) | purine-binding chemotaxis protein (cheW-2) | 5773 95 | 5779 37 | + | | 15594910 | 1195412 |
| Complete genome (Accession number NC_001318) | hypothetical protein BB0566 | 5779 50 | 5782 61 | + | | 15594911 | 1195413 |
| Complete genome (Accession number NC_001318) | chemotaxis histidine kinase (cheA-1) | 5782 77 | 5804 21 | + | | 15594912 | 1195414 |
| Complete genome (Accession number NC_001318) | protein-glutamate methylesterase (cheB-2) | 5804 86 | 5816 43 | + | | 15594913 | 1195415 |
| Complete genome (Accession number NC_001318) | hypothetical protein BB0569 | 5816 45 | 5834 17 | + | | 15594914 | 1195416 |
| Complete genome (Accession number NC_001318) | chemotaxis response regulator (cheY-2) | 5834 58 | 5838 32 | + | | 15594915 | 1195417 |
| Complete genome (Accession number NC_001318) | uridylate kinase | 5841 91 | 5848 80 | - | | 15594916 | 1195418 |
| Complete genome (Accession number NC_001318) | glycosyl transferase (lgtD) | 5850 80 | 5861 56 | + | | 15594917 | 1195419 |

| | | | | | | | |
|---|---|---|---|---|---|---|---|
| Complete genome (Accession number NC_001318) | ABC transporter, ATP-binding protein | 5862 12 | 5870 24 | + | | 15594918 | 1195420 |
| Complete genome (Accession number NC_001318) | hypothetical protein BB0574 | 5870 31 | 5878 82 | + | | 15594919 | 1195421 |
| Complete genome (Accession number NC_001318) | CTP synthetase | 5880 66 | 5896 67 | + | | 15594920 | 1195422 |
| Complete genome (Accession number NC_001318) | hypothetical protein BB0576 | 5896 84 | 5902 32 | - | | 15594921 | 1195423 |
| Complete genome (Accession number NC_001318) | hypothetical protein BB0577 | 5903 48 | 5909 35 | + | | 15594922 | 1195424 |
| Complete genome (Accession number NC_001318) | methyl-accepting chemotaxis protein (mcp-1) | 5911 88 | 5923 57 | + | | 15594923 | 1195425 |
| Complete genome (Accession number NC_001318) | DNA polymerase III subunit alpha | 5924 46 | 5959 31 | + | | 15594924 | 1195426 |
| Complete genome (Accession number NC_001318) | hypothetical protein BB0580 | 5959 43 | 5965 21 | + | | 15594925 | 1195427 |
| Complete genome (Accession number NC_001318) | DNA recombinase (recG) | 5965 31 | 5985 91 | + | | 15594926 | 1195428 |
| Complete genome (Accession number NC_001318) | carboxypeptidase, putative | 5985 88 | 5993 76 | - | | 15594927 | 1195429 |
| Complete genome (Accession number NC_001318) | hypothetical protein BB0583 | 5994 95 | 6008 44 | + | | 15594928 | 1195430 |
| Complete genome (Accession number NC_001318) | hypothetical protein BB0584 | 6009 98 | 6023 44 | + | | 15594929 | 1195431 |

| Complete genome (Accession number NC_001318) | UDP-N-acetylmuramoylalanine--D-glutamate ligase (murD) | 6023 74 | 6037 29 | + | | 15594930 | 1195432 |
|---|---|---|---|---|---|---|---|
| Complete genome (Accession number NC_001318) | femA protein (femA) | 6037 26 | 6047 69 | - | | 15594931 | 1195433 |
| Complete genome (Accession number NC_001318) | methionyl-tRNA synthetase | 6047 79 | 6069 83 | - | | 15594932 | 1195434 |
| Complete genome (Accession number NC_001318) | pfs protein (pfs-2) | 6071 38 | 6079 35 | + | | 15594933 | 1195435 |
| Complete genome (Accession number NC_001318) | phosphate acetyltransferase (pta) | 6080 20 | 6090 78 | + | | 15594934 | 1195436 |
| Complete genome (Accession number NC_001318) | dimethyladenosine transferase | 6090 61 | 6099 06 | - | | 15594935 | 1195437 |
| Complete genome (Accession number NC_001318) | competence locus E, putative | 6099 43 | 6111 93 | - | | 15594936 | 1195438 |
| Complete genome (Accession number NC_001318) | hypothetical protein BB0592 | 6113 09 | 6120 22 | + | | 15594937 | 1195439 |
| Complete genome (Accession number NC_001318) | long-chain-fatty-acid CoA ligase | 6120 48 | 6139 85 | + | | 15594938 | 1195440 |
| Complete genome (Accession number NC_001318) | arginyl-tRNA synthetase | 6140 14 | 6157 89 | - | | 15594939 | 1195441 |
| Complete genome (Accession number NC_001318) | hypothetical protein BB0595 | 6157 64 | 6163 90 | - | | 15594940 | 1195442 |

| | | | | | | | |
|---|---|---|---|---|---|---|---|
| Complete genome (Accession number NC_001318) | methyl-accepting chemotaxis protein (mcp-2) | 6164 12 | 6185 59 | - | | 15594941 | 1195443 |
| Complete genome (Accession number NC_001318) | methyl-accepting chemotaxis protein (mcp-3) | 6188 28 | 6210 35 | + | | 15594942 | 1195444 |
| Complete genome (Accession number NC_001318) | UDP-N-acetylmuramate dehydrogenase (murB) | 6210 45 | 6219 53 | - | | 15594943 | 1195445 |
| Complete genome (Accession number NC_001318) | cysteinyl-tRNA synthetase (cysS) | 6220 05 | 6234 47 | + | | 15594944 | 1195446 |
| Complete genome (Accession number NC_001318) | hypothetical protein BB0600 | 6234 51 | 6250 07 | + | | 15594945 | 1195447 |
| Complete genome (Accession number NC_001318) | serine hydroxymethyltransferase (glyA) | 6250 07 | 6262 60 | + | | 15594946 | 1195448 |
| Complete genome (Accession number NC_001318) | chaperonin, putative | 6262 86 | 6270 38 | + | | 15594947 | 1195449 |
| Complete genome (Accession number NC_001318) | membrane-associated protein p66 | 6270 92 | 6289 48 | - | | 15594948 | 1195450 |
| Complete genome (Accession number NC_001318) | L-lactate permease (lctP) | 6291 96 | 6306 98 | - | | 15594949 | 1195451 |
| Complete genome (Accession number NC_001318) | serine-type D-Ala-D-Ala carboxypeptidase (dacA) | 6307 69 | 6319 86 | + | | 15594950 | 1195452 |
| Complete genome (Accession number NC_001318) | hypothetical protein BB0606 | 6319 91 | 6324 82 | + | | 15594951 | 1195453 |

| | | | | | | | |
|---|---|---|---|---|---|---|---|
| Complete genome (Accession number NC_001318) | rep helicase, single-stranded DNA-dependent ATPase (rep) | 6324 85 | 6344 64 | + | | 15594952 | 1195454 |
| Complete genome (Accession number NC_001318) | aminoacyl-histidine dipeptidase (pepD) | 6345 81 | 6360 11 | + | | 15594953 | 1195455 |
| Complete genome (Accession number NC_001318) | hypothetical protein BB0609 | 6360 24 | 6361 34 | - | | 15594954 | 1195456 |
| Complete genome (Accession number NC_001318) | trigger factor | 6365 93 | 6379 57 | + | | 15594955 | 1195459 |
| Complete genome (Accession number NC_001318) | ATP-dependent Clp protease proteolytic component (clpP-1) | 6379 63 | 6385 56 | + | | 15594956 | 1195460 |
| Complete genome (Accession number NC_001318) | ATP-dependent protease ATP-binding subunit | 6385 80 | 6398 72 | + | | 15594957 | 1195461 |
| Complete genome (Accession number NC_001318) | ATP-dependent protease LA (lon-2) | 6398 08 | 6422 49 | + | | 15594958 | 1195462 |
| Complete genome (Accession number NC_001318) | hypothetical protein BB0614 | 6422 89 | 6424 41 | + | | 15594959 | 1195463 |
| Complete genome (Accession number NC_001318) | 30S ribosomal protein S4 | 6425 00 | 6431 29 | - | | 15594960 | 1195464 |

| | | | | | | | |
|---|---|---|---|---|---|---|---|
| Complete genome (Accession number NC_001318) | hypothetical protein BB0616 | 6432 95 | 6446 50 | - | | 15594961 | 1195465 |
| Complete genome (Accession number NC_001318) | hypothetical protein BB0617 | 6447 72 | 6451 40 | + | | 15594962 | 1195466 |
| Complete genome (Accession number NC_001318) | cytidine deaminase (cdd) | 6451 37 | 6456 01 | - | | 15594963 | 1195467 |
| Complete genome (Accession number NC_001318) | hypothetical protein BB0619 | 6456 89 | 6466 81 | + | | 15594964 | 1195468 |
| Complete genome (Accession number NC_001318) | beta-glucosidase, putative | 6466 59 | 6483 26 | - | | 15594965 | 1195469 |
| Complete genome (Accession number NC_001318) | 4-methyl-5(b-hydroxyethyl)-thiazole monophosphate biosynthesis protein (thiJ) | 6485 53 | 6491 07 | + | | 15594966 | 1195471 |
| Complete genome (Accession number NC_001318) | acetate kinase (ackA) | 6491 70 | 6504 11 | - | | 15594967 | 1195472 |
| Complete genome (Accession number NC_001318) | transcription-repair coupling factor (mfd) | 6504 13 | 6537 90 | - | | 15594968 | 1195473 |
| Complete genome (Accession number NC_001318) | hypothetical protein BB0624 | 6538 87 | 6548 49 | + | | 15594969 | 1195474 |
| Complete genome (Accession number NC_001318) | N-acetylmuramoyl-L-alanine amidase, putative | 6548 09 | 6569 02 | + | | 15594970 | 1195475 |

| | | | | | | | |
|---|---|---|---|---|---|---|---|
| Complete genome (Accession number NC_001318) | hypothetical protein BB0626 | 6569 98 | 6575 43 | - | | 15594971 | 1195477 |
| Complete genome (Accession number NC_001318) | putative aminopeptidase 2 | 6575 46 | 6588 17 | - | | 15594972 | 1195478 |
| Complete genome (Accession number NC_001318) | hypothetical protein BB0628 | 6588 98 | 6596 23 | + | | 15594973 | 1195479 |
| Complete genome (Accession number NC_001318) | PTS system, fructose-specific IIABC component (fruA-2) | 6596 44 | 6615 24 | - | | 15594974 | 1195480 |
| Complete genome (Accession number NC_001318) | 1-phosphofructokina se (fruK) | 6616 06 | 6625 29 | + | | 15594975 | 1195481 |
| Complete genome (Accession number NC_001318) | hypothetical protein BB0631 | 6626 03 | 6629 17 | - | | 15594976 | 1195482 |
| Complete genome (Accession number NC_001318) | exodeoxyribonucl ease V, alpha chain (recD) | 6631 79 | 6650 11 | - | | 15594977 | 1195484 |
| Complete genome (Accession number NC_001318) | exodeoxyribonucl ease V, beta chain (recB) | 6650 08 | 6685 17 | - | | 15594978 | 1195485 |
| Complete genome (Accession number NC_001318) | exodeoxyribonucl ease V, gamma chain (recC) | 6688 48 | 6717 63 | - | | 15594979 | 1195486 |
| Complete genome (Accession number NC_001318) | nicotinate phosphoribosyltra nsferase | 6717 67 | 6732 12 | - | | 15594980 | 1195487 |

| Complete genome (Accession number NC_001318) | glucose-6-phosphate 1-dehydrogenase | 6733 42 | 6747 78 | + | | 15594981 | 1195488 |
|---|---|---|---|---|---|---|---|
| Complete genome (Accession number NC_001318) | Na+/H+ antiporter (nhaC-1) | 6750 29 | 6763 78 | + | | 15594982 | 1195489 |
| Complete genome (Accession number NC_001318) | Na+/H+ antiporter (nhaC-2) | 6764 72 | 6778 60 | + | | 15594983 | 1195490 |
| Complete genome (Accession number NC_001318) | spermidine/putres cine ABC transporter, spermidine/putres cine-binding periplasmic protein (potD) | 6778 92 | 6789 38 | - | | 15594984 | 1195491 |
| Complete genome (Accession number NC_001318) | spermidine/putres cine ABC transporter, permease protein (potC) | 6789 59 | 6797 50 | - | | 15594985 | 1195492 |
| Complete genome (Accession number NC_001318) | spermidine/putres cine ABC transporter, permease protein (potB) | 6797 54 | 6805 63 | - | | 15594986 | 1195493 |
| Complete genome (Accession number NC_001318) | spermidine/putres cine ABC transporter, ATP-binding protein (potA) | 6805 63 | 6816 06 | - | | 15594987 | 1195494 |
| Complete genome (Accession number NC_001318) | ribosomal biogenesis GTPase | 6816 85 | 6825 24 | - | | 15594988 | 1195495 |

| Complete genome (Accession number NC_001318) | N-acetylmannosamine-6-phosphate 2-epimerase | 6826 79 | 6833 77 | + | | 15594989 | 1195496 |
|---|---|---|---|---|---|---|---|
| Complete genome (Accession number NC_001318) | PTS system, glucose-specific IIBC component (ptsG) | 6834 23 | 6849 67 | + | | 15594990 | 1195497 |
| Complete genome (Accession number NC_001318) | hypothetical protein BB0646 | 6849 97 | 6859 80 | - | | 15594991 | 1195498 |
| Complete genome (Accession number NC_001318) | ferric uptake regulation protein (fur) | 6859 77 | 6865 07 | - | | 15594992 | 1195499 |
| Complete genome (Accession number NC_001318) | serine/threonine kinase, putative | 6866 07 | 6882 95 | - | | 15594993 | 1195500 |
| Complete genome (Accession number NC_001318) | chaperonin GroEL | 6884 90 | 6901 27 | + | | 15594994 | 1195501 |
| Complete genome (Accession number NC_001318) | hypothetical protein BB0650 | 6901 51 | 6904 89 | + | | 15594995 | 1195502 |
| Complete genome (Accession number NC_001318) | hypothetical protein BB0651 | 6904 82 | 6907 99 | + | | 15594996 | 1195503 |
| Complete genome (Accession number NC_001318) | preprotein translocase subunit SecD | 6908 85 | 6926 45 | + | | 15594997 | 1195504 |
| Complete genome (Accession number NC_001318) | preprotein translocase subunit SecF | 6926 29 | 6935 28 | + | | 15594998 | 1195505 |

| | | | | | | | |
|---|---|---|---|---|---|---|---|
| Complete genome (Accession number NC_001318) | hypothetical protein BB0654 | 6935 54 | 6946 96 | + | | 15594999 | 1195506 |
| Complete genome (Accession number NC_001318) | heat shock protein (dnaJ-2) | 6946 93 | 6955 23 | - | | 15595000 | 1195507 |
| Complete genome (Accession number NC_001318) | oxygen-independent coproporphyrinog en III oxidase, putative | 6956 31 | 6967 64 | + | | 15595001 | 1195508 |
| Complete genome (Accession number NC_001318) | ribose 5-phosphate isomerase (rpi) | 6967 51 | 6974 37 | - | | 15595002 | 1195509 |
| Complete genome (Accession number NC_001318) | phosphoglycerate mutase (gpmA) | 6975 71 | 6983 32 | + | | 15595003 | 1195510 |
| Complete genome (Accession number NC_001318) | lysyl-tRNA synthetase | 6984 00 | 6999 65 | - | | 15595004 | 1195511 |
| Complete genome (Accession number NC_001318) | GTP-binding protein Era | 7000 52 | 7009 24 | - | | 15595005 | 1195512 |
| Complete genome (Accession number NC_001318) | hypothetical protein BB0661 | 7010 14 | 7013 85 | + | | 15595006 | 1195513 |
| Complete genome (Accession number NC_001318) | hypothetical protein BB0662 | 7013 95 | 7018 05 | + | | 15595007 | 1195514 |
| Complete genome (Accession number NC_001318) | hypothetical protein BB0663 | 7018 25 | 7022 74 | + | | 15595008 | 1195515 |
| Complete genome (Accession number NC_001318) | hypothetical protein BB0664 | 7022 83 | 7029 66 | - | | 15595009 | 1195516 |

| | | | | | | | |
|---|---|---|---|---|---|---|---|
| Complete genome (Accession number NC_001318) | hypothetical protein BB0665 | 7030 09 | 7039 68 | + | | 15595010 | 1195517 |
| Complete genome (Accession number NC_001318) | hypothetical protein BB0666 | 7039 31 | 7049 59 | + | | 15595011 | 1195518 |
| Complete genome (Accession number NC_001318) | hypothetical protein BB0667 | 7049 49 | 7055 00 | + | | 15595012 | 1195519 |
| Complete genome (Accession number NC_001318) | flagellar filament outer layer protein (flaA) | 7055 71 | 7066 05 | + | | 15595013 | 1195520 |
| Complete genome (Accession number NC_001318) | chemotaxis histidine kinase (cheA-2) | 7066 73 | 7092 67 | + | | 15595014 | 1195521 |
| Complete genome (Accession number NC_001318) | purine-binding chemotaxis protein (cheW-3) | 7092 74 | 7106 74 | + | | 15595015 | 1195522 |
| Complete genome (Accession number NC_001318) | chemotaxis operon protein (cheX) | 7106 86 | 7111 71 | + | | 15595016 | 1195523 |
| Complete genome (Accession number NC_001318) | chemotaxis response regulator (cheY-3) | 7112 11 | 7116 51 | + | | 15595017 | 1195524 |
| Complete genome (Accession number NC_001318) | hypothetical protein BB0673 | 7116 92 | 7122 07 | - | | 15595018 | 1195525 |
| Complete genome (Accession number NC_001318) | hypothetical protein BB0674 | 7122 04 | 7132 50 | - | | 15595019 | 1195526 |
| Complete genome (Accession number NC_001318) | hypothetical protein BB0675 | 7132 44 | 7141 07 | - | | 15595020 | 1195527 |

| | | | | | | | |
|---|---|---|---|---|---|---|---|
| Complete genome (Accession number NC_001318) | phosphoglycolate phosphatase (gph) | 7141 04 | 7147 66 | - | | 15595021 | 1195528 |
| Complete genome (Accession number NC_001318) | ribose/galactose ABC transporter, ATP-binding protein (mglA) | 7150 09 | 7166 19 | + | | 15595022 | 1195529 |
| Complete genome (Accession number NC_001318) | ribose/galactose ABC transporter, permease protein (rbsC-1) | 7166 20 | 7177 71 | + | | 15595023 | 1195530 |
| Complete genome (Accession number NC_001318) | ribose/galactose ABC transporter, permease protein (rbsC-2) | 7177 34 | 7186 69 | + | | 15595024 | 1195531 |
| Complete genome (Accession number NC_001318) | methyl-accepting chemotaxis protein (mcp-4) | 7188 27 | 7210 88 | + | | 15595025 | 1195532 |
| Complete genome (Accession number NC_001318) | methyl-accepting chemotaxis protein (mcp-5) | 7211 10 | 7230 20 | + | | 15595026 | 1195533 |
| Complete genome (Accession number NC_001318) | tRNA (5-methylaminomethyl-2-thiouridylate)-methyltransferase | 7230 63 | 7241 30 | - | | 15595027 | 1195534 |
| Complete genome (Accession number NC_001318) | 3-hydroxy-3-methylglutaryl-CoA synthase | 7242 24 | 7254 47 | + | | 15595028 | 1195535 |

| Complete genome (Accession number NC_001318) | isopentenyl pyrophosphate isomerase | 7254 13 | 7264 95 | + | | 15595029 | 1195536 |
|---|---|---|---|---|---|---|---|
| Complete genome (Accession number NC_001318) | 3-hydroxy-3-methylglutaryl-CoA reductase (mvaA) | 7264 58 | 7277 53 | + | | 15595030 | 1195537 |
| Complete genome (Accession number NC_001318) | mevalonate pyrophosphate decarboxylase | 7277 37 | 7286 75 | + | | 15595031 | 1195538 |
| Complete genome (Accession number NC_001318) | phosphomevalonate kinase, putative | 7286 66 | 7296 19 | + | | 15595032 | 1195539 |
| Complete genome (Accession number NC_001318) | mevalonate kinase | 7296 13 | 7305 06 | + | | 15595033 | 1195540 |
| Complete genome (Accession number NC_001318) | hypothetical protein BB0689 | 7306 32 | 7310 99 | - | | 15595034 | 1195542 |
| Complete genome (Accession number NC_001318) | neutrophil activating protein (napA) | 7311 51 | 7317 20 | + | | 15595035 | 1195543 |
| Complete genome (Accession number NC_001318) | elongation factor G | 7317 94 | 7338 03 | + | | 15595036 | 1195544 |
| Complete genome (Accession number NC_001318) | hypothetical protein BB0692 | 7338 09 | 7339 22 | + | | 15595037 | 1195545 |
| Complete genome (Accession number NC_001318) | xylose operon regulatory protein (xylR-1) | 7340 81 | 7352 89 | + | | 15595038 | 1195546 |
| Complete genome (Accession number NC_001318) | signal recognition particle protein (ffh) | 7353 43 | 7366 86 | + | | 15595039 | 1195547 |

**EP 2 254 592 B1**

| | | | | | | | |
|---|---|---|---|---|---|---|---|
| Complete genome (Accession number NC_001318) | 30S ribosomal protein S16 | 7366 99 | 7369 59 | + | | 15595040 | 1195548 |
| Complete genome (Accession number NC_001318) | hypothetical protein BB0696 | 7369 60 | 7372 08 | + | | 15595041 | 1195549 |
| Complete genome (Accession number NC_001318) | hypothetical protein BB0697 | 7372 12 | 7377 12 | + | | 15595042 | 1195550 |
| Complete genome (Accession number NC_001318) | tRNA (guanine-N(1)-)-methyltransferase | 7377 09 | 7384 28 | + | | 15595043 | 1195551 |
| Complete genome (Accession number NC_001318) | 50S ribosomal protein L19 | 7384 06 | 7387 71 | + | | 15595044 | 1195552 |
| Complete genome (Accession number NC_001318) | hypothetical protein BB0700 | 7388 51 | 7389 64 | + | | 15595045 | 1195553 |
| Complete genome (Accession number NC_001318) | hypothetical protein BB0701 | 7391 44 | 7396 83 | + | | 15595046 | 1195554 |
| Complete genome (Accession number NC_001318) | lipopolysaccharide biosynthesis-related protein (kdtB) | 7396 85 | 7401 76 | + | | 15595047 | 1195555 |
| Complete genome (Accession number NC_001318) | 50S ribosomal protein L32 | 7402 40 | 7404 22 | + | | 15595048 | 1195556 |
| Complete genome (Accession number NC_001318) | acyl carrier protein | 7404 46 | 7406 88 | + | | 15595049 | 1195557 |
| Complete genome (Accession number NC_001318) | ribonuclease III (rnc) | 7406 85 | 7414 25 | + | | 15595050 | 1195558 |

| | | | | | | | |
|---|---|---|---|---|---|---|---|
| Complete genome (Accession number NC_001318) | polynucleotide adenylyltransferase (papS) | 7413 96 | 7426 28 | - | | 15595051 | 1195559 |
| Complete genome (Accession number NC_001318) | hypothetical protein BB0707 | 7426 90 | 7445 16 | + | | 15595052 | 1195560 |
| Complete genome (Accession number NC_001318) | hypothetical protein BB0708 | 7445 22 | 7448 45 | + | | 15595053 | 1195561 |
| Complete genome (Accession number NC_001318) | hypothetical protein BB0709 | 7453 02 | 7463 33 | + | | 15595054 | 1195563 |
| Complete genome (Accession number NC_001318) | DNA primase (dnaG) | 7463 39 | 7479 10 | + | | 15595055 | 1195564 |
| Complete genome (Accession number NC_001318) | hypothetical protein BB0711 | 7479 25 | 7481 19 | + | | 15595056 | 1195565 |
| Complete genome (Accession number NC_001318) | RNA polymerase sigma factor RpoD | 7481 23 | 7500 18 | + | | 15595057 | 1195566 |
| Complete genome (Accession number NC_001318) | hypothetical protein BB0713 | 7500 28 | 7507 89 | + | | 15595058 | 1195567 |
| Complete genome (Accession number NC_001318) | hypothetical protein BB0714 | 7511 91 | 7521 59 | + | | 15595059 | 1195568 |
| Complete genome (Accession number NC_001318) | rod shape-determining protein (mreB-1) | 7521 34 | 7532 19 | + | | 15595060 | 1195569 |
| Complete genome (Accession number NC_001318) | rod shape-determining protein (mreC) | 7532 23 | 7540 68 | + | | 15595061 | 1195570 |
| Complete genome (Accession number NC_001318) | hypothetical protein BB0717 | 7540 68 | 7545 50 | + | | 15595062 | 1195571 |

| | | | | | | | |
|---|---|---|---|---|---|---|---|
| Complete genome (Accession number NC_001318) | penicillin-binding protein (pbp-2) | 7545 50 | 7563 49 | + | | 15595063 | 1195572 |
| Complete genome (Accession number NC_001318) | rod shape-determining protein (mreB-2) | 7562 91 | 7576 70 | + | | 15595064 | 1195573 |
| Complete genome (Accession number NC_001318) | threonyl-tRNA synthetase (thrZ) | 7578 36 | 7595 81 | + | | 15595065 | 1195574 |
| Complete genome (Accession number NC_001318) | CDP-diacylglycerol--glycerol-3-phosphate 3-phosphatidyltransf erase | 7595 86 | 7602 15 | + | | 15595066 | 1195575 |
| Complete genome (Accession number NC_001318) | hypothetical protein BB0722 | 7602 02 | 7616 47 | + | | 15595067 | 1195576 |
| Complete genome (Accession number NC_001318) | hypothetical protein BB0723 | 7616 21 | 7621 54 | - | | 15595068 | 1195577 |
| Complete genome (Accession number NC_001318) | K+ transport protein (ntpJ) | 7622 42 | 7635 73 | + | | 15595069 | 1195578 |
| Complete genome (Accession number NC_001318) | hypothetical protein BB0725 | 7635 70 | 7643 61 | + | | 15595070 | 1195579 |
| Complete genome (Accession number NC_001318) | ATP-binding protein (ylxH-3) | 7643 71 | 7653 42 | + | | 15595071 | 1195580 |
| Complete genome (Accession number NC_001318) | diphosphate--fructose-6-phosphate 1-phosphotransfera se | 7653 70 | 7667 16 | + | | 15595072 | 1195581 |

| | | | | | | | |
|---|---|---|---|---|---|---|---|
| Complete genome (Accession number NC_001318) | coenzyme A disulfide reductase | 7667 33 | 7680 67 | - | | 15595073 | 1195582 |
| Complete genome (Accession number NC_001318) | glutamate transporter (gltP) | 7680 79 | 7694 70 | - | | 15595074 | 1195583 |
| Complete genome (Accession number NC_001318) | glucose-6-phosphate isomerase | 7695 47 | 7711 45 | - | | 15595075 | 1195584 |
| Complete genome (Accession number NC_001318) | hypothetical protein BB0731 | 7711 62 | 7715 63 | - | | 15595076 | 1195585 |
| Complete genome (Accession number NC_001318) | penicillin-binding protein (pbp-3) | 7715 70 | 7743 68 | - | | 15595077 | 1195586 |
| Complete genome (Accession number NC_001318) | hypothetical protein BB0733 | 7746 32 | 7754 62 | + | | 15595078 | 1195587 |
| Complete genome (Accession number NC_001318) | hypothetical protein BB0734 | 7756 66 | 7766 79 | - | | 15595079 | 1195588 |
| Complete genome (Accession number NC_001318) | rare lipoprotein A (rlpA) | 7767 46 | 7775 73 | + | | 15595080 | 1195589 |
| Complete genome (Accession number NC_001318) | hypothetical protein BB0736 | 7776 25 | 7777 68 | + | | 15595081 | 1195590 |
| Complete genome (Accession number NC_001318) | histidine phosphokinase/ph ophatase, putative | 7777 47 | 7787 54 | + | | 15595082 | 1195591 |
| Complete genome (Accession number NC_001318) | valyl-tRNA synthetase | 7787 58 | 7813 85 | + | | 15595083 | 1195592 |
| Complete genome (Accession number NC_001318) | hypothetical protein BB0739 | 7814 86 | 7820 73 | - | | 15595084 | 1195593 |

| | | | | | | | |
|---|---|---|---|---|---|---|---|
| Complete genome (Accession number NC_001318) | hypothetical protein BB0740 | 7821 52 | 7828 29 | - | | 15595085 | 1195594 |
| Complete genome (Accession number NC_001318) | co-chaperonin GroES | 7829 76 | 7832 57 | - | | 15595086 | 1195595 |
| Complete genome (Accession number NC_001318) | ABC transporter, ATP-binding protein | 7833 24 | 7850 21 | + | | 15595087 | 1195596 |
| Complete genome (Accession number NC_001318) | hypothetical protein BB0743 | 7850 57 | 7867 54 | + | | 15595088 | 1195597 |
| Complete genome (Accession number NC_001318) | antigen, p83/100 | 7868 60 | 7889 62 | + | | 15595089 | 1195598 |
| Complete genome (Accession number NC_001318) | endonuclease III (nth) | 7892 70 | 7899 38 | + | | 15595090 | 1195601 |
| Complete genome (Accession number NC_001318) | oligopeptide ABC transporter, permease protein (oppC-2) | 7899 47 | 7908 07 | - | | 15595091 | 1195602 |
| Complete genome (Accession number NC_001318) | oligopeptide ABC transporter, permease protein (oppB-2) | 7908 07 | 7917 87 | - | | 15595092 | 1195603 |
| Complete genome (Accession number NC_001318) | hypothetical protein BB0748 | 7917 94 | 7922 52 | - | | 15595093 | 1195604 |
| Complete genome (Accession number NC_001318) | hypothetical protein BB0749 | 7923 33 | 7936 25 | + | | 15595094 | 1195605 |
| Complete genome (Accession number NC_001318) | hypothetical protein BB0750 | 7936 93 | 7938 36 | + | | 15595095 | 1195606 |

| | | | | | | | |
|---|---|---|---|---|---|---|---|
| Complete genome (Accession number NC_001318) | hypothetical protein BB0751 | 7938 42 | 7949 15 | - | | 15595096 | 1195607 |
| Complete genome (Accession number NC_001318) | hypothetical protein BB0752 | 7948 75 | 7963 83 | - | | 15595097 | 1195608 |
| Complete genome (Accession number NC_001318) | membrane spanning protein, putative | 7963 88 | 7971 22 | - | | 15595098 | 1195609 |
| Complete genome (Accession number NC_001318) | ABC transporter, ATP-binding protein | 7971 19 | 7980 48 | - | | 15595099 | 1195610 |
| Complete genome (Accession number NC_001318) | ribonuclease Z | 7980 57 | 7990 16 | - | | 15595100 | 1195611 |
| Complete genome (Accession number NC_001318) | hypothetical protein BB0756 | 7992 08 | 8002 75 | - | | 15595101 | 1195612 |
| Complete genome (Accession number NC_001318) | ATP-dependent Clp protease proteolytic subunit | 8004 52 | 8010 72 | + | | 15595102 | 1195613 |
| Complete genome (Accession number NC_001318) | hypothetical protein BB0758 | 8009 96 | 8017 78 | + | | 15595103 | 1195614 |
| Complete genome (Accession number NC_001318) | hypothetical protein BB0759 | 8019 41 | 8027 98 | - | | 15595104 | 1195616 |
| Complete genome (Accession number NC_001318) | hypothetical protein BB0760 | 8028 38 | 8032 12 | - | | 15595105 | 1195617 |
| Complete genome (Accession number NC_001318) | hypothetical protein BB0761 | 8032 78 | 8041 65 | + | | 15595106 | 1195618 |
| Complete genome (Accession number NC_001318) | hypothetical protein BB0762 | 8042 40 | 8043 53 | - | | 15595107 | 1195619 |

| Complete genome (Accession number NC_001318) | response regulatory protein (rrp-2) | 8044 17 | 8057 78 | - | | 15595108 | 1195620 |
|---|---|---|---|---|---|---|---|
| Complete genome (Accession number NC_001318) | sensory transduction histidine kinase, putative | 8057 75 | 8069 23 | - | | 15595109 | 1195621 |
| Complete genome (Accession number NC_001318) | hypothetical protein BB0765 | 8069 20 | 8079 66 | - | | 15595110 | 1195622 |
| Complete genome (Accession number NC_001318) | colicin V production protein, putative | 8079 76 | 8084 64 | - | | 15595111 | 1195623 |
| Complete genome (Accession number NC_001318) | UDP-N-acetylglucosamine --N-acetylmuramyl-(pentapeptide) pyrophosphoryl-undecaprenol N-acetylglucosamine transferase (murG) | 8084 61 | 8095 52 | - | | 15595112 | 1195624 |
| Complete genome (Accession number NC_001318) | pyridoxal kinase (pdxK) | 8097 01 | 8104 95 | - | | 15595113 | 1195625 |
| Complete genome (Accession number NC_001318) | O-sialoglycoprotein endopeptidase | 8104 70 | 8115 10 | - | | 15595114 | 1195626 |
| Complete genome (Accession number NC_001318) | hypothetical protein BB0770 | 8115 07 | 8123 88 | - | | 15595115 | 1195627 |
| Complete genome (Accession number NC_001318) | RNA polymerase sigma factor (rpoS) | 8124 39 | 8132 39 | - | | 15595116 | 1195628 |

| | | | | | | | |
|---|---|---|---|---|---|---|---|
| Complete genome (Accession number NC_001318) | flagellar basal body P-ring protein | 8136 90 | 8146 97 | - | | 15595117 | 1195629 |
| Complete genome (Accession number NC_001318) | hypothetical protein BB0773 | 8148 68 | 8153 02 | - | | 15595118 | 1195630 |
| Complete genome (Accession number NC_001318) | flagellar basal body rod protein FlgG | 8155 02 | 8162 99 | - | | 15595119 | 1195631 |
| Complete genome (Accession number NC_001318) | flagellar hook-basal body complex protein (flhO) | 8163 12 | 8172 14 | - | | 15595120 | 1195632 |
| Complete genome (Accession number NC_001318) | hypothetical protein BB0776 | 8173 93 | 8179 56 | + | | 15595121 | 1195633 |
| Complete genome (Accession number NC_001318) | adenine phosphoribosyltra nsferase | 8180 18 | 8185 48 | + | | 15595122 | 1195634 |
| Complete genome (Accession number NC_001318) | 50S ribosomal protein L21 | 8186 05 | 8189 16 | + | | 15595123 | 1195635 |
| Complete genome (Accession number NC_001318) | hypothetical protein BB0779 | 8189 22 | 8192 42 | + | | 15595124 | 1195636 |
| Complete genome (Accession number NC_001318) | 50S ribosomal protein L27 | 8192 42 | 8194 87 | + | | 15595125 | 1195637 |
| Complete genome (Accession number NC_001318) | GTPase ObgE | 8195 42 | 8205 28 | + | | 15595126 | 1195638 |
| Complete genome (Accession number NC_001318) | hypothetical protein BB0782 | 8205 68 | 8211 88 | + | | 15595127 | 1195639 |

| | | | | | | | |
|---|---|---|---|---|---|---|---|
| Complete genome (Accession number NC_001318) | hypothetical protein BB0783 | 8211 85 | 8223 69 | + | | 15595128 | 1195640 |
| Complete genome (Accession number NC_001318) | hypothetical protein BB0784 | 8223 35 | 8226 94 | + | | 15595129 | 1195641 |
| Complete genome (Accession number NC_001318) | regulatory protein SpoVG | 8228 10 | 8231 03 | + | | 15595130 | 1195642 |
| Complete genome (Accession number NC_001318) | 50S ribosomal protein L25/general stress protein Ctc | 8232 63 | 8238 11 | + | | 1,62E+08 | 1195643 |
| Complete genome (Accession number NC_001318) | peptidyl-tRNA hydrolase | 8238 16 | 8243 82 | + | | 15595132 | 1195645 |
| Complete genome (Accession number NC_001318) | hypothetical protein BB0788 | 8243 82 | 8257 04 | + | | 15595133 | 1195646 |
| Complete genome (Accession number NC_001318) | cell division protein (ftsH) | 8257 01 | 8276 20 | + | | 15595134 | 1195647 |
| Complete genome (Accession number NC_001318) | hypothetical protein BB0790 | 8276 25 | 8291 12 | + | | 15595135 | 1195648 |
| Complete genome (Accession number NC_001318) | thymidine kinase (tdk) | 8292 18 | 8303 21 | + | | 15595136 | 1195649 |
| Complete genome (Accession number NC_001318) | hypothetical protein BB0792 | 8303 18 | 8309 41 | - | | 15595137 | 1195650 |
| Complete genome (Accession number NC_001318) | thymidylate kinase (tmk) | 8309 55 | 8317 28 | - | | 15595138 | 1195651 |
| Complete genome (Accession number NC_001318) | hypothetical protein BB0794 | 8316 46 | 8360 43 | + | | 15595139 | 1195652 |
| Complete genome (Accession number NC_001318) | outer membrane protein | 8360 61 | 8385 26 | + | | 15595140 | 1195653 |

| | | | | | | | |
|---|---|---|---|---|---|---|---|
| Complete genome (Accession number NC_001318) | hypothetical protein BB0796 | 8385 23 | 8390 80 | + | | 15595141 | 1195654 |
| Complete genome (Accession number NC_001318) | DNA mismatch repair protein | 8392 44 | 8418 32 | + | | 15595142 | 1195655 |
| Complete genome (Accession number NC_001318) | competence protein F, putative | 8418 34 | 8424 51 | + | | 15595143 | 1195656 |
| Complete genome (Accession number NC_001318) | hypothetical protein BB0799 | 8425 01 | 8429 89 | + | | 15595144 | 1195657 |
| Complete genome (Accession number NC_001318) | transcription elongation factor NusA | 8430 03 | 8444 51 | + | | 15595145 | 1195658 |
| Complete genome (Accession number NC_001318) | translation initiation factor IF-2 | 8444 54 | 8471 02 | + | | 15595146 | 1195659 |
| Complete genome (Accession number NC_001318) | ribosome-binding factor A (rbfA) | 8471 05 | 8474 88 | + | | 15595147 | 1195660 |
| Complete genome (Accession number NC_001318) | tRNA pseudouridine 55 synthase (truB) | 8474 91 | 8483 39 | + | | 15595148 | 1195661 |
| Complete genome (Accession number NC_001318) | 30S ribosomal protein S15 | 8485 38 | 8488 04 | + | | 15595149 | 1195662 |
| Complete genome (Accession number NC_001318) | polynucleotide phosphorylase/pol yadenylase | 8488 19 | 8509 87 | + | | 15595150 | 1195663 |
| Complete genome (Accession number NC_001318) | hypothetical protein BB0806 | 8509 94 | 8525 14 | + | | 15595151 | 1195664 |

| | | | | | | | |
|---|---|---|---|---|---|---|---|
| Complete genome (Accession number NC_001318) | hypothetical protein BB0807 | 8524 89 | 8537 78 | + | | 15595152 | 1195665 |
| Complete genome (Accession number NC_001318) | hypothetical protein BB0808 | 8537 75 | 8548 42 | + | | 15595153 | 1195666 |
| Complete genome (Accession number NC_001318) | tRNA-guanine transglycosylase (tgt) | 8548 69 | 8559 96 | + | | 15595154 | 1195667 |
| Complete genome (Accession number NC_001318) | virulence factor mviN protein (mviN) (SP:P37169) | 8559 71 | 8575 09 | + | | 15595155 | 1195668 |
| Complete genome (Accession number NC_001318) | hypothetical protein BB0811 | 8575 06 | 8589 15 | + | | 15595156 | 1195669 |
| Complete genome (Accession number NC_001318) | pantothenate metabolism flavoprotein (dfp) | 8589 39 | 8601 11 | - | | 15595157 | 1195670 |
| Complete genome (Accession number NC_001318) | hypothetical protein BB0813 | 8601 86 | 8603 62 | + | | 15595158 | 1195671 |
| Complete genome (Accession number NC_001318) | sodium/panthothe nate symporter | 8604 40 | 8617 74 | + | | 15595159 | 1195672 |
| Complete genome (Accession number NC_001318) | hypothetical protein BB0815 | 8617 84 | 8626 77 | + | | 15595160 | 1195673 |
| Complete genome (Accession number NC_001318) | hypothetical protein BB0816 | 8626 96 | 8636 46 | + | | 15595161 | 1195674 |

| | | | | | | | |
|---|---|---|---|---|---|---|---|
| Complete genome (Accession number NC_001318) | UDP-N-acetylmuramate--L-alanine ligase | 8636 36 | 8650 42 | + | | 15595162 | 1195675 |
| Complete genome (Accession number NC_001318) | hypothetical protein BB0818 | 8650 53 | 8659 19 | + | | 15595163 | 1195676 |
| Complete genome (Accession number NC_001318) | cytidylate kinase | 8659 16 | 8664 58 | + | | 15595164 | 1195677 |
| Complete genome (Accession number NC_001318) | hypothetical protein BB0820 | 8664 94 | 8666 94 | + | | 15595165 | 1195678 |
| Complete genome (Accession number NC_001318) | 2-methylthio-N6-isopentyladenosine tRNA modification enzyme (miaA) | 8666 81 | 8676 01 | + | | 15595166 | 1195679 |
| Complete genome (Accession number NC_001318) | hypothetical protein BB0822 | 8676 05 | 8677 00 | + | | 15595167 | 1195680 |
| Complete genome (Accession number NC_001318) | hypothetical protein BB0823 | 8677 43 | 8681 14 | - | | 15595168 | 1195681 |
| Complete genome (Accession number NC_001318) | hypothetical protein BB0824 | 8681 89 | 8687 40 | + | | 15595169 | 1195682 |
| Complete genome (Accession number NC_001318) | hypothetical protein BB0825 | 8687 33 | 8695 15 | - | | 15595170 | 1195683 |
| Complete genome (Accession number NC_001318) | hypothetical protein BB0826 | 8695 08 | 8700 23 | - | | 15595171 | 1195684 |
| Complete genome (Accession number NC_001318) | ATP-dependent helicase (hrpA) | 8701 12 | 8725 83 | - | | 15595172 | 1195685 |

| | | | | | | | |
|---|---|---|---|---|---|---|---|
| Complete genome (Accession number NC_001318) | DNA topoisomerase I (topA) | 8725 86 | 8751 32 | - | | 15595173 | 1195686 |
| Complete genome (Accession number NC_001318) | exonuclease SbcD (sbcD) | 8752 07 | 8764 48 | + | | 15595174 | 1195687 |
| Complete genome (Accession number NC_001318) | exonuclease SbcC (sbcC) | 8764 29 | 8792 81 | + | | 15595175 | 1195688 |
| Complete genome (Accession number NC_001318) | xylose operon regulatory protein (xylR-2) | 8793 03 | 8802 50 | + | | 15595176 | 1195689 |
| Complete genome (Accession number NC_001318) | hypothetical protein BB0832 | 8802 67 | 8810 91 | - | | 15595177 | 1195690 |
| Complete genome (Accession number NC_001318) | isoleucyl-tRNA synthetase | 8810 85 | 8842 13 | - | | 15595178 | 1195691 |
| Complete genome (Accession number NC_001318) | ATP-dependent Clp protease, subunit C (clpC) | 8842 28 | 8864 47 | - | | 15595179 | 1195692 |
| Complete genome (Accession number NC_001318) | phosphomannom utase (cpsG) | 8864 70 | 8881 82 | - | | 15595180 | 1195693 |
| Complete genome (Accession number NC_001318) | excinuclease ABC subunit B | 8882 97 | 8903 18 | + | | 15595181 | 1195694 |
| Complete genome (Accession number NC_001318) | excinuclease ABC, subunit A (uvrA) | 8903 22 | 8931 74 | + | | 15595182 | 1195695 |
| Complete genome (Accession number NC_001318) | hypothetical protein BB0838 | 8931 29 | 8965 69 | + | | 15595183 | 1195696 |
| Complete genome (Accession number NC_001318) | hypothetical protein BB0839 | 8970 96 | 8977 40 | - | | 15595184 | 1195697 |

| | | | | | | | |
|---|---|---|---|---|---|---|---|
| Complete genome (Accession number NC_001318) | hypothetical protein BB0840 | 8977 04 | 8993 20 | - | | 15595185 | 1195698 |
| Complete genome (Accession number NC_001318) | arginine deiminase (arcA) | 8994 99 | 9007 31 | + | | 15595186 | 1195699 |
| Complete genome (Accession number NC_001318) | ornithine carbamoyltransfer ase, catabolic (arcB) | 9007 96 | 9017 82 | + | | 15595187 | 1195700 |
| Complete genome (Accession number NC_001318) | hypothetical protein BB0843 | 9018 10 | 9032 58 | + | | 15595188 | 1195701 |
| Complete genome (Accession number NC_001318) | hypothetical protein BB0844 | 9039 29 | 9049 00 | - | | 15595189 | 1195702 |
| Complete genome (Accession number NC_001318) | hypothetical protein BB0845 | 9051 20 | 9052 27 | + | | 15595190 | 1195703 |
| Complete genome (Accession number NC_001318) | hypothetical protein BB0846 | 9057 52 | 9058 65 | - | | 15595191 | 1195704 |
| Complete genome (Accession number NC_001318) | hypothetical protein BB0847 | 9058 39 | 9059 46 | + | | 15595192 | 1195705 |
| Complete genome (Accession number NC_001318) | hypothetical protein BB0848 | 9059 28 | 9060 32 | + | | 15595193 | 1195706 |
| Complete genome (Accession number NC_001318) | hypothetical protein BB0849 | 9061 62 | 9062 63 | + | | 15595194 | 1195707 |
| Complete genome (Accession number NC_001318) | hypothetical protein BB0851 | 9079 35 | 9080 81 | + | | 15595195 | 1195708 |
| Complete genome (Accession number NC_001318) | hypothetical protein BB0852 | 9084 07 | 9095 91 | + | | 15595196 | 1195709 |

# Fig. 28. Borrelia afzelli

| Plasmid or genome | Product Name | Start | End | Strand | Length | Gi | GeneID |
|---|---|---|---|---|---|---|---|
| Plasmid cp30 (Accession number NC_008273) | hypothetical protein BAPKO_4000 | 63 | 632 | + | 189 | 111074075 | 4227522 |
| Plasmid cp30 (Accession number NC_008273) | hypothetical protein BAPKO_4001 | 671 | 916 | - | 81 | 111074076 | 4227523 |
| Plasmid cp30 (Accession number NC_008273) | hypothetical protein BAPKO_4002 | 1038 | 1367 | - | 109 | 111074077 | 4227524 |
| Plasmid cp30 (Accession number NC_008273) | hypothetical protein BAPKO_4003 | 1371 | 1568 | - | 65 | 111074078 | 4227525 |
| Plasmid cp30 (Accession number NC_008273) | hypothetical protein BAPKO_4004 | 1642 | 2457 | - | 271 | 111074079 | 4227485 |
| Plasmid cp30 (Accession number NC_008273) | hypothetical protein BAPKO_4005 | 2569 | 3081 | - | 170 | 111074080 | 4227486 |
| Plasmid cp30 (Accession number NC_008273) | hypothetical protein BAPKO_4006 | 3078 | 3956 | - | 292 | 111074081 | 4227487 |
| Plasmid cp30 (Accession number NC_008273) | hypothetical protein BAPKO_4007 | 3953 | 4285 | - | 110 | 111074082 | 4227488 |
| Plasmid cp30 (Accession number NC_008273) | hypothetical protein BAPKO_4008 | 4403 | 4564 | + | 53 | 111074083 | 4227489 |

| Plasmid cp30 (Accession number NC_008273) | hypothetical protein BAPKO_4009 | 4795 | 4875 | - | 26 | 111074084 | 4227490 |
|---|---|---|---|---|---|---|---|
| Plasmid cp30 (Accession number NC_008273) | hypothetical protein BAPKO_4010 | 4877 | 4996 | - | 39 | 111074085 | 4227491 |
| Plasmid cp30 (Accession number NC_008273) | hypothetical protein BAPKO_4011 | 5038 | 5883 | - | 281 | 111074086 | 4227492 |
| Plasmid cp30 (Accession number NC_008273) | hypothetical protein BAPKO_4012 | 5887 | 6519 | - | 210 | 111074087 | 4227493 |
| Plasmid cp30 (Accession number NC_008273) | hypothetical protein BAPKO_4013 | 6513 | 7817 | - | 434 | 111074088 | 4227494 |
| Plasmid cp30 (Accession number NC_008273) | hypothetical protein BAPKO_4014 | 7825 | 8061 | - | 78 | 111074089 | 4227495 |
| Plasmid cp30 (Accession number NC_008273) | hypothetical protein BAPKO_4015 | 8058 | 8180 | - | 40 | 111074090 | 4227496 |
| Plasmid cp30 (Accession number NC_008273) | hypothetical protein BAPKO_4016 | 8186 | 8503 | - | 105 | 111074091 | 4227497 |
| Plasmid cp30 (Accession number NC_008273) | hypothetical protein BAPKO_4017 | 8519 | 8947 | - | 142 | 111074092 | 4227498 |
| Plasmid cp30 (Accession number NC_008273) | hypothetical protein BAPKO_4018 | 8961 | 1007 3 | - | 370 | 111074093 | 4227499 |

| | | | | | | | |
|---|---|---|---|---|---|---|---|
| Plasmid cp30 (Accession number NC_008273) | hypothetical protein BAPKO_4019 | 1005 4 | 1062 3 | - | 189 | 111074094 | 4227500 |
| Plasmid cp30 (Accession number NC_008273) | hypothetical protein BAPKO_4020 | 1062 0 | 1100 6 | - | 128 | 111074095 | 4227501 |
| Plasmid cp30 (Accession number NC_008273) | hypothetical protein BAPKO_4021 | 1099 4 | 1137 7 | - | 127 | 111074096 | 4227502 |
| Plasmid cp30 (Accession number NC_008273) | hypothetical protein BAPKO_4022 | 1137 7 | 1183 2 | - | 151 | 111074097 | 4227503 |
| Plasmid cp30 (Accession number NC_008273) | hypothetical protein BAPKO_4023 | 1185 1 | 1281 0 | - | 319 | 111074098 | 4227504 |
| Plasmid cp30 (Accession number NC_008273) | hypothetical protein BAPKO_4024 | 1283 2 | 1340 4 | - | 190 | 111074099 | 4227505 |
| Plasmid cp30 (Accession number NC_008273) | hypothetical protein BAPKO_4025 | 1342 9 | 1418 7 | - | 252 | 111074100 | 4227506 |
| Plasmid cp30 (Accession number NC_008273) | hypothetical protein BAPKO_4026 | 1419 5 | 1475 8 | - | 187 | 111074101 | 4227507 |
| Plasmid cp30 (Accession number NC_008273) | hypothetical protein BAPKO_4027 | 1477 0 | 1546 5 | - | 231 | 111074102 | 4227508 |
| Plasmid cp30 (Accession number NC_008273) | hypothetical protein BAPKO_4028 | 1548 2 | 1670 5 | - | 407 | 111074103 | 4227266 |

| Plasmid cp30 (Accession number NC_008273) | hypothetical protein BAPKO_4029 | 1677 1 | 1812 3 | - | 450 | 111074104 | 4227267 |
|---|---|---|---|---|---|---|---|
| Plasmid cp30 (Accession number NC_008273) | hypothetical protein BAPKO_4030 | 1815 4 | 1863 9 | - | 161 | 111074105 | 4227509 |
| Plasmid cp30 (Accession number NC_008273) | hypothetical protein BAPKO_4031 | 1880 9 | 1952 5 | + | 238 | 111074106 | 4227510 |
| Plasmid cp30 (Accession number NC_008273) | hypothetical protein BAPKO_4032 | 1966 0 | 2016 9 | - | 169 | 111074107 | 4227511 |
| Plasmid cp30 (Accession number NC_008273) | hypothetical protein BAPKO_4033 | 2028 1 | 2090 1 | - | 206 | 111074108 | 4227512 |
| Plasmid cp30 (Accession number NC_008273) | hypothetical protein BAPKO_4034 | 2092 9 | 2147 7 | - | 182 | 111074109 | 4227513 |
| Plasmid cp30 (Accession number NC_008273) | hypothetical protein BAPKO_4035 | 2191 0 | 2268 6 | + | 258 | 111074110 | 4227514 |
| Plasmid cp30 (Accession number NC_008273) | hypothetical protein BAPKO_4036 | 2269 1 | 2303 8 | - | 115 | 111074111 | 4227515 |
| Plasmid cp30 (Accession number NC_008273) | hypothetical protein BAPKO_4037 | 2316 8 | 2449 6 | - | 442 | 111074112 | 4227516 |
| Plasmid cp30 (Accession number NC_008273) | hypothetical protein BAPKO_4038 | 2523 6 | 2576 0 | - | 174 | 111074113 | 4227517 |

| | | | | | | | |
|---|---|---|---|---|---|---|---|
| Plasmid cp30 (Accession number NC_008273) | hypothetical protein BAPKO_4039 | 2577 3 | 2633 0 | - | 185 | 111074114 | 4227518 |
| Plasmid cp30 (Accession number NC_008273) | hypothetical protein BAPKO_4040 | 2637 2 | 2712 7 | - | 251 | 111074115 | 4227519 |
| Plasmid cp30 (Accession number NC_008273) | hypothetical protein BAPKO_4041 | 2710 3 | 2768 1 | - | 192 | 111074116 | 4227520 |
| Plasmid cp30 (Accession number NC_008273) | hypothetical protein BAPKO_4042 | 2769 1 | 2878 5 | - | 364 | 111074117 | 4227521 |
| Plasmid cp27 (Accession number NC_008274) | hypothetical protein BAPKO_5000 | 46 | 327 | + | 93 | 111074119 | 4227268 |
| Plasmid cp27 (Accession number NC_008274) | hypothetical protein BAPKO_5001 | 311 | 757 | - | 148 | 111074120 | 4227269 |
| Plasmid cp27 (Accession number NC_008274) | hypothetical protein BAPKO_5002 | 843 | 2192 | - | 449 | 111074121 | 4227270 |
| Plasmid cp27 (Accession number NC_008274) | PTS system, cellobiose-specific IIC component | 2436 | 3758 | - | 440 | 111074122 | 4227271 |
| Plasmid cp27 (Accession number NC_008274) | PTS system, cellobiose-specific IIA component | 3961 | 4308 | + | 115 | 111074123 | 4227272 |

| | | | | | | | |
|---|---|---|---|---|---|---|---|
| Plasmid cp27 (Accession number NC_008274) | PTS system, cellobiose-specific IIB component | 4317 | 4643 | + | 108 | 111074124 | 4227273 |
| Plasmid cp27 (Accession number NC_008274) | hypothetical protein BAPKO_5006 | 4645 | 5742 | + | 365 | 111074125 | 4227274 |
| Plasmid cp27 (Accession number NC_008274) | hypothetical protein BAPKO_5007 | 5764 | 6393 | - | 209 | 111074126 | 4227275 |
| Plasmid cp27 (Accession number NC_008274) | hypothetical protein BAPKO_5008 | 6535 | 7563 | + | 342 | 111074127 | 4227276 |
| Plasmid cp27 (Accession number NC_008274) | hypothetical protein BAPKO_5009 | 7695 | 8633 | + | 312 | 111074128 | 4227277 |
| Plasmid cp27 (Accession number NC_008274) | hypothetical protein BAPKO_5010 | 8633 | 9151 | + | 172 | 111074129 | 4227278 |
| Plasmid cp27 (Accession number NC_008274) | hypothetical protein BAPKO_5011 | 9127 | 9888 | + | 253 | 111074130 | 4227279 |
| Plasmid cp27 (Accession number NC_008274) | hypothetical protein BAPKO_5012 | 9954 | 10523 | + | 189 | 111074131 | 4227280 |
| Plasmid cp27 (Accession number NC_008274) | hypothetical protein BAPKO_5013 | 10773 | 11270 | - | 165 | 111074132 | 4227281 |

| | | | | | | | |
|---|---|---|---|---|---|---|---|
| Plasmid cp27 (Accession number NC_008274) | oligopeptide ABC transporter, periplasmic oligopeptide-binding protein | 1202 8 | 1362 0 | + | 530 | 111074133 | 4227282 |
| Plasmid cp27 (Accession number NC_008274) | hypothetical protein BAPKO_5015 | 1367 2 | 1380 0 | - | 42 | 111074134 | 4227283 |
| Plasmid cp27 (Accession number NC_008274) | inositol-5-monophosphate dehydrogenase | 1390 1 | 1511 2 | - | 403 | 111074135 | 4227284 |
| Plasmid cp27 (Accession number NC_008274) | bifunctional GMP synthase/glutamine amidotransferase protein | 1513 7 | 1667 2 | - | 511 | 111074136 | 4227285 |
| Plasmid cp27 (Accession number NC_008274) | outer surface protein C | 1692 5 | 1756 3 | + | 212 | 111074137 | 4227286 |
| Plasmid cp27 (Accession number NC_008274) | hypothetical protein BAPKO_5019 | 1799 7 | 1935 2 | - | 451 | 111074138 | 4227287 |
| Plasmid cp27 (Accession number NC_008274) | hypothetical protein BAPKO_5020 | 1946 1 | 2081 6 | - | 451 | 111074139 | 4227288 |
| Plasmid cp27 (Accession number NC_008274) | hypothetical protein BAPKO_5021 | 2089 1 | 2139 4 | - | 167 | 111074140 | 4227289 |
| Plasmid cp27 (Accession number NC_008274) | hypothetical protein BAPKO_5022 | 2136 9 | 2188 1 | - | 170 | 111074141 | 4227290 |

| Plasmid cp27 (Accession number NC_008274) | hypothetical protein BAPKO_5023 | 21928 | 22623 | + | 231 | 111074142 | 4227291 |
|---|---|---|---|---|---|---|---|
| Plasmid cp27 (Accession number NC_008274) | hypothetical protein BAPKO_5024 | 22633 | 23184 | - | 183 | 111074143 | 4227292 |
| Plasmid cp27 (Accession number NC_008274) | hypothetical protein BAPKO_5025 | 23286 | 24530 | + | 414 | 111074144 | 4227293 |
| Plasmid cp27 (Accession number NC_008274) | hypothetical protein BAPKO_5026 | 24831 | 24926 | - | 31 | 111074145 | 4227294 |
| Plasmid cp27 (Accession number NC_008274) | PTS system, maltose and glucose-specific IIABC component | 24925 | 26487 | + | 520 | 111074146 | 4227295 |
| Plasmid lp60 (Accession number NC_008564) | hypothetical protein BAPKO_2000 | 782 | 1336 | - | 184 | 117621571 | 4491022 |
| Plasmid lp60 (Accession number NC_008564) | hypothetical protein BAPKO_2001 | 1691 | 2500 | + | 269 | 117621572 | 4491023 |
| Plasmid lp60 (Accession number NC_008564) | hypothetical protein BAPKO_2002 | 2785 | 2973 | + | 62 | 117621573 | 4491024 |
| Plasmid lp60 (Accession number NC_008564) | hypothetical protein BAPKO_2003 | 3222 | 3707 | + | 161 | 117621574 | 4491025 |
| Plasmid lp60 (Accession number NC_008564) | outer membrane protein | 3949 | 4467 | + | 172 | 117621575 | 4491026 |
| Plasmid lp60 (Accession number NC_008564) | antigen, S2 | 4545 | 5387 | - | 280 | 117621576 | 4491027 |
| Plasmid lp60 (Accession number NC_008564) | antigen, S1 | 5488 | 6735 | - | 415 | 117621577 | 4491028 |

| | | | | | | | |
|---|---|---|---|---|---|---|---|
| Plasmid lp60 (Accession number NC_008564) | hypothetical protein BAPKO_2007 | 6967 | 7086 | - | 39 | 117621578 | 4491029 |
| Plasmid lp60 (Accession number NC_008564) | chpAI protein, putative | 7147 | 7617 | - | 156 | 117621579 | 4491050 |
| Plasmid lp60 (Accession number NC_008564) | hypothetical protein BAPKO_2009 | 7788 | 8123 | + | 111 | 117621580 | 4491051 |
| Plasmid lp60 (Accession number NC_008564) | hypothetical protein BAPKO_2010 | 8120 | 8992 | + | 290 | 117621581 | 4491052 |
| Plasmid lp60 (Accession number NC_008564) | hypothetical protein BAPKO_2011 | 8995 | 9621 | + | 208 | 117621582 | 4491053 |
| Plasmid lp60 (Accession number NC_008564) | hypothetical protein BAPKO_2012 | 9677 | 10711 | + | 344 | 117621583 | 4491054 |
| Plasmid lp60 (Accession number NC_008564) | hypothetical protein BAPKO_2013 | 10735 | 10914 | + | 59 | 117621584 | 4491055 |
| Plasmid lp60 (Accession number NC_008564) | hypothetical protein BAPKO_2014 | 10911 | 11330 | + | 139 | 117621585 | 4491056 |
| Plasmid lp60 (Accession number NC_008564) | hypothetical protein BAPKO_2015 | 11320 | 11685 | + | 121 | 117621586 | 4491057 |
| Plasmid lp60 (Accession number NC_008564) | outer surface protein A | 11891 | 12712 | + | 273 | 117621587 | 4491058 |
| Plasmid lp60 (Accession number NC_008564) | outer surface protein B | 12726 | 13625 | + | 299 | 117621588 | 4491059 |
| Plasmid lp60 (Accession number NC_008564) | hypothetical protein BAPKO_2018 | 14200 | 15399 | + | 399 | 117621589 | 4491060 |
| Plasmid lp60 (Accession number NC_008564) | hypothetical protein BAPKO_2019 | 15447 | 16031 | + | 194 | 117621590 | 4491061 |
| Plasmid lp60 (Accession number NC_008564) | hypothetical protein BAPKO_2020 | 16007 | 16759 | + | 250 | 117621591 | 4491062 |

| | | | | | | | |
|---|---|---|---|---|---|---|---|
| Plasmid lp60 (Accession number NC_008564) | hypothetical protein BAPKO_2021 | 16789 | 17334 | + | 181 | 117621592 | 4491063 |
| Plasmid lp60 (Accession number NC_008564) | hypothetical protein BAPKO_2022 | 17599 | 17718 | + | 39 | 117621593 | 4491064 |
| Plasmid lp60 (Accession number NC_008564) | hypothetical protein BAPKO_2023 | 18044 | 18475 | + | 143 | 117621594 | 4491065 |
| Plasmid lp60 (Accession number NC_008564) | decorin binding protein A | 18591 | 19103 | - | 170 | 117621595 | 4491066 |
| Plasmid lp60 (Accession number NC_008564) | decorin binding protein B | 19219 | 19770 | - | 183 | 117621596 | 4491067 |
| Plasmid lp60 (Accession number NC_008564) | hypothetical protein BAPKO_2026 | 20199 | 20516 | - | 105 | 117621597 | 4491068 |
| Plasmid lp60 (Accession number NC_008564) | hypothetical protein BAPKO_2027 | 20638 | 21231 | + | 197 | 117621598 | 4491069 |
| Plasmid lp60 (Accession number NC_008564) | hypothetical protein BAPKO_2028 | 21235 | 22587 | + | 450 | 117621599 | 4491070 |
| Plasmid lp60 (Accession number NC_008564) | hypothetical protein BAPKO_2029 | 22676 | 22804 | + | 42 | 117621600 | 4491071 |
| Plasmid lp60 (Accession number NC_008564) | hypothetical protein BAPKO_2030 | 22886 | 23311 | + | 141 | 117621601 | 4491072 |
| Plasmid lp60 (Accession number NC_008564) | hypothetical protein BAPKO_2031 | 23444 | 23638 | + | 64 | 117621602 | 4491073 |
| Plasmid lp60 (Accession number NC_008564) | hypothetical protein BAPKO_2032 | 23801 | 24340 | + | 179 | 117621603 | 4491074 |
| Plasmid lp60 (Accession number NC_008564) | oligopeptide ABC transporter, periplasmic | 24430 | 26016 | - | 528 | 117621604 | 4491075 |
| Plasmid lp60 (Accession number NC_008564) | lipoprotein | 26531 | 27181 | + | 216 | 117621605 | 4491076 |

| Plasmid lp60 (Accession number NC_008564) | hypothetical protein BAPKO_2035 | 2728 9 | 2789 7 | + | 202 | 117621606 | 4491077 |
|---|---|---|---|---|---|---|---|
| Plasmid lp60 (Accession number NC_008564) | hypothetical protein BAPKO_2036 | 2826 3 | 2949 2 | + | 409 | 117621607 | 4491078 |
| Plasmid lp60 (Accession number NC_008564) | hypothetical protein BAPKO_2037 | 2950 4 | 3010 3 | + | 199 | 117621608 | 4491079 |
| Plasmid lp60 (Accession number NC_008564) | hypothetical protein BAPKO_2038 | 3015 8 | 3076 9 | + | 203 | 117621609 | 4491080 |
| Plasmid lp60 (Accession number NC_008564) | hypothetical protein BAPKO_2039 | 3078 6 | 3170 3 | + | 305 | 117621610 | 4491081 |
| Plasmid lp60 (Accession number NC_008564) | hypothetical protein BAPKO_2040 | 3170 6 | 3216 1 | + | 151 | 117621611 | 4491082 |
| Plasmid lp60 (Accession number NC_008564) | hypothetical protein BAPKO_2041 | 3215 8 | 3254 7 | + | 129 | 117621612 | 4491083 |
| Plasmid lp60 (Accession number NC_008564) | hypothetical protein BAPKO_2042 | 3254 4 | 3289 7 | + | 117 | 117621613 | 4491084 |
| Plasmid lp60 (Accession number NC_008564) | hypothetical protein BAPKO_2043 | 3289 4 | 3343 3 | + | 179 | 117621614 | 4491085 |
| Plasmid lp60 (Accession number NC_008564) | hypothetical protein BAPKO_2044 | 3349 2 | 3456 2 | + | 356 | 117621615 | 4491086 |
| Plasmid lp60 (Accession number NC_008564) | hypothetical protein BAPKO_2045 | 3456 2 | 3498 4 | + | 140 | 117621616 | 4491087 |
| Plasmid lp60 (Accession number NC_008564) | hypothetical protein BAPKO_2046 | 3502 6 | 3551 7 | + | 163 | 117621617 | 4491088 |
| Plasmid lp60 (Accession number NC_008564) | hypothetical protein BAPKO_2047 | 3551 0 | 3572 8 | + | 72 | 117621618 | 4491030 |
| Plasmid lp60 (Accession number NC_008564) | hypothetical protein BAPKO_2048 | 3573 7 | 3711 6 | + | 459 | 117621619 | 4491031 |

| | | | | | | | |
|---|---|---|---|---|---|---|---|
| Plasmid lp60 (Accession number NC_008564) | hypothetical protein BAPKO_2049 | 3714 7 | 3771 0 | + | 187 | 117621620 | 4491032 |
| Plasmid lp60 (Accession number NC_008564) | outer membrane protein | 3775 0 | 3866 7 | + | 305 | 117621621 | 4491033 |
| Plasmid lp60 (Accession number NC_008564) | hypothetical protein BAPKO_2051 | 3874 8 | 3902 6 | + | 92 | 117621622 | 4491034 |
| Plasmid lp60 (Accession number NC_008564) | hypothetical protein BAPKO_2052 | 3905 6 | 3932 8 | + | 90 | 117621623 | 4491035 |
| Plasmid lp60 (Accession number NC_008564) | hypothetical protein BAPKO_2053 | 3939 1 | 4032 9 | + | 312 | 117621624 | 4491036 |
| Plasmid lp60 (Accession number NC_008564) | hypothetical protein BAPKO_2054 | 4031 9 | 4088 2 | + | 187 | 117621625 | 4491037 |
| Plasmid lp60 (Accession number NC_008564) | hypothetical protein BAPKO_2055 | 4094 6 | 4214 5 | - | 399 | 117621626 | 4491038 |
| Plasmid lp60 (Accession number NC_008564) | lipoprotein | 4262 8 | 4286 1 | - | 77 | 117621627 | 4491039 |
| Plasmid lp60 (Accession number NC_008564) | surface lipoprotein P27 | 4295 8 | 4373 4 | - | 258 | 117621628 | 4491040 |
| Plasmid lp60 (Accession number NC_008564) | hypothetical protein BAPKO_2058 | 4411 2 | 4473 8 | - | 208 | 117621629 | 4491041 |
| Plasmid lp60 (Accession number NC_008564) | lipoprotein | 4498 7 | 4516 6 | + | 59 | 117621630 | 4491042 |
| Plasmid lp60 (Accession number NC_008564) | antigen, P35 | 4537 1 | 4634 5 | - | 324 | 117621631 | 4491043 |
| Plasmid lp60 (Accession number NC_008564) | hypothetical protein BAPKO_2061 | 4651 1 | 4736 2 | - | 283 | 117621632 | 4491044 |
| Plasmid lp60 (Accession number NC_008564) | antigen, P35, putative | 4752 9 | 4877 6 | - | 415 | 117621633 | 4491045 |

| Plasmid lp60 (Accession number NC_008564) | hypothetical protein BAPKO_2063 | 4893 4 | 4962 9 | - | 231 | 117621634 | 4491046 |
|---|---|---|---|---|---|---|---|
| Plasmid lp60 (Accession number NC_008564) | hypothetical protein BAPKO_2064 | 4970 7 | 5042 6 | - | 239 | 117621635 | 4491047 |
| Plasmid lp60 (Accession number NC_008564) | hypothetical protein BAPKO_2065 | 5066 1 | 5143 7 | - | 258 | 117621636 | 4491048 |
| Plasmid lp60 (Accession number NC_008564) | hypothetical protein BAPKO_2066 | 5163 8 | 5245 0 | - | 270 | 117621637 | 4491049 |
| Plasmid lp60 (Accession number NC_008564) | hypothetical protein BAPKO_2067 | 5265 1 | 5339 7 | - | 248 | 117621638 | 4491014 |
| Plasmid lp60 (Accession number NC_008564) | hypothetical protein BAPKO_2068 | 5367 3 | 5439 8 | - | 241 | 117621639 | 4491015 |
| Plasmid lp60 (Accession number NC_008564) | hypothetical protein BAPKO_2069 | 5467 0 | 5542 5 | - | 251 | 117621640 | 4491016 |
| Plasmid lp60 (Accession number NC_008564) | hypothetical protein BAPKO_2070 | 5573 6 | 5643 4 | - | 232 | 117621641 | 4491017 |
| Plasmid lp60 (Accession number NC_008564) | antigen, P35, putative | 5666 0 | 5754 1 | - | 293 | 117621642 | 4491018 |
| Plasmid lp60 (Accession number NC_008564) | hypothetical protein BAPKO_2072 | 5769 9 | 5783 6 | - | 45 | 117621643 | 4491019 |
| Plasmid lp60 (Accession number NC_008564) | outer membrane porin | 5807 6 | 5889 7 | + | 273 | 117621644 | 4491020 |
| Plasmid lp60 (Accession number NC_008564) | FAD-dependent thymidylate synthase | 5910 5 | 5990 8 | + | 267 | 117621645 | 4491021 |
| Plasmid lp60-2 (Accession number NC_008565) | hypothetical protein BAPKO_2500 | 120 | 683 | + | 187 | 117621647 | 4491089 |
| Plasmid lp60-2 (Accession number NC_008565) | hypothetical protein BAPKO_2501 | 866 | 1363 | + | 165 | 117621648 | 4491090 |

| | | | | | | | |
|---|---|---|---|---|---|---|---|
| Plasmid lp60-2 (Accession number NC_008565) | hypothetical protein BAPKO_2502 | 1360 | 1545 | - | 61 | 117621649 | 4491091 |
| Plasmid lp60-2 (Accession number NC_008565) | hypothetical protein BAPKO_2503 | 1777 | 1989 | + | 70 | 117621650 | 4491092 |
| Plasmid lp60-2 (Accession number NC_008565) | hypothetical protein BAPKO_2504 | 2513 | 3337 | + | 274 | 117621651 | 4491093 |
| Plasmid lp60-2 (Accession number NC_008565) | hypothetical protein BAPKO_2505 | 3901 | 4251 | + | 116 | 117621652 | 4491094 |
| Plasmid lp60-2 (Accession number NC_008565) | hypothetical protein BAPKO_2506 | 4400 | 5080 | + | 226 | 117621653 | 4491095 |
| Plasmid lp60-2 (Accession number NC_008565) | hypothetical protein BAPKO_2507 | 5952 | 6845 | + | 297 | 117621654 | 4491096 |
| Plasmid lp60-2 (Accession number NC_008565) | hypothetical protein BAPKO_2508 | 7447 | 11277 | - | 1276 | 117621655 | 4491097 |
| Plasmid lp60-2 (Accession number NC_008565) | hypothetical protein BAPKO_2509 | 11397 | 11627 | - | 76 | 117621656 | 4491098 |
| Plasmid lp60-2 (Accession number NC_008565) | hypothetical protein BAPKO_2510 | 11700 | 11903 | - | 67 | 117621657 | 4491099 |
| Plasmid lp60-2 (Accession number NC_008565) | hypothetical protein BAPKO_2511 | 11818 | 11961 | - | 47 | 117621658 | 4491100 |
| Plasmid lp60-2 (Accession number NC_008565) | hypothetical protein BAPKO_2512 | 12703 | 13620 | + | 305 | 117621659 | 4491101 |
| Plasmid lp60-2 (Accession number NC_008565) | hypothetical protein BAPKO_2513 | 13613 | 14179 | + | 188 | 117621660 | 4491102 |
| Plasmid lp60-2 (Accession number NC_008565) | hypothetical protein BAPKO_2514 | 14152 | 14907 | + | 251 | 117621661 | 4491103 |
| Plasmid lp60-2 (Accession number NC_008565) | hypothetical protein BAPKO_2515 | 15003 | 15632 | + | 209 | 117621662 | 4491104 |

| Plasmid lp60-2 (Accession number NC_008565) | hypothetical protein BAPKO_2516 | 1579 1 | 1613 5 | + | 114 | 117621663 | 4491105 |
|---|---|---|---|---|---|---|---|
| Plasmid lp60-2 (Accession number NC_008565) | hypothetical protein BAPKO_2517 | 1642 7 | 1653 4 | + | 35 | 117621664 | 4491106 |
| Plasmid lp60-2 (Accession number NC_008565) | hypothetical protein BAPKO_2518 | 1691 4 | 1717 4 | - | 86 | 117621665 | 4491107 |
| Plasmid lp60-2 (Accession number NC_008565) | hypothetical protein BAPKO_2519 | 1762 8 | 1861 1 | - | 327 | 117621666 | 4491108 |
| Plasmid lp60-2 (Accession number NC_008565) | hypothetical protein BAPKO_2520 | 1929 1 | 2014 5 | + | 284 | 117621667 | 4491109 |
| Plasmid lp60-2 (Accession number NC_008565) | hypothetical protein BAPKO_2521 | 2089 2 | 2190 2 | - | 336 | 117621668 | 4491110 |
| Plasmid lp60-2 (Accession number NC_008565) | hypothetical protein BAPKO_2522 | 2259 8 | 2314 6 | + | 182 | 117621669 | 4491111 |
| Plasmid lp60-2 (Accession number NC_008565) | hypothetical protein BAPKO_2523 | 2354 6 | 2402 8 | - | 160 | 117621670 | 4491112 |
| Plasmid lp60-2 (Accession number NC_008565) | hypothetical protein BAPKO_2524 | 2460 5 | 2492 8 | - | 107 | 117621671 | 4491113 |
| Plasmid lp60-2 (Accession number NC_008565) | hypothetical protein BAPKO_2525 | 2562 1 | 2679 6 | - | 391 | 117621672 | 4491114 |
| Plasmid lp60-2 (Accession number NC_008565) | hypothetical protein BAPKO_2526 | 2691 9 | 2710 4 | - | 61 | 117621673 | 4491115 |
| Plasmid lp60-2 (Accession number NC_008565) | hypothetical protein BAPKO_2527 | 2719 4 | 3102 7 | - | 1277 | 117621674 | 4491116 |
| Plasmid lp60-2 (Accession number NC_008565) | hypothetical protein BAPKO_2528 | 3261 3 | 3278 6 | + | 57 | 117621675 | 4491117 |
| Plasmid lp60-2 (Accession number NC_008565) | hypothetical protein BAPKO_2529 | 3287 5 | 3303 3 | - | 52 | 117621676 | 4491118 |

| Plasmid lp60-2 (Accession number NC_008565) | hypothetical protein BAPKO_2530 | 3407 8 | 3421 8 | + | 46 | 117621677 | 4491119 |
|---|---|---|---|---|---|---|---|
| Plasmid lp60-2 (Accession number NC_008565) | hypothetical protein BAPKO_2531 | 3470 8 | 3494 1 | + | 77 | 117621678 | 4491120 |
| Plasmid lp60-2 (Accession number NC_008565) | hypothetical protein BAPKO_2532 | 3646 6 | 3656 1 | - | 31 | 117621679 | 4491121 |
| Plasmid lp60-2 (Accession number NC_008565) | hypothetical protein BAPKO_2533 | 3648 5 | 3657 7 | - | 30 | 117621680 | 4491122 |
| Plasmid lp60-2 (Accession number NC_008565) | hypothetical protein BAPKO_2534 | 3681 1 | 3695 7 | - | 48 | 117621681 | 4491123 |
| Plasmid lp60-2 (Accession number NC_008565) | hypothetical protein BAPKO_2535 | 3701 6 | 3706 9 | - | 17 | 117621682 | 4491124 |
| Plasmid lp60-2 (Accession number NC_008565) | hypothetical protein BAPKO_2536 | 3712 8 | 3788 0 | - | 250 | 117621683 | 4491125 |
| Plasmid lp60-2 (Accession number NC_008565) | hypothetical protein BAPKO_2537 | 3785 6 | 3841 0 | - | 184 | 117621684 | 4491126 |
| Plasmid lp60-2 (Accession number NC_008565) | hypothetical protein BAPKO_2538 | 3845 2 | 3952 2 | - | 356 | 117621685 | 4491127 |
| Plasmid lp60-2 (Accession number NC_008565) | hypothetical protein BAPKO_2539 | 3969 1 | 3988 5 | + | 64 | 117621686 | 4491128 |
| Plasmid lp60-2 (Accession number NC_008565) | hypothetical protein BAPKO_2540 | 4072 3 | 4089 6 | + | 57 | 117621687 | 4491129 |
| Plasmid lp60-2 (Accession number NC_008565) | hypothetical protein BAPKO_2541 | 4118 8 | 4179 6 | - | 202 | 117621688 | 4491130 |
| Plasmid lp60-2 (Accession number NC_008565) | hypothetical protein BAPKO_2542 | 4329 7 | 4407 9 | - | 260 | 117621689 | 4491131 |
| Plasmid lp60-2 (Accession number NC_008565) | hypothetical protein BAPKO_2543 | 4452 9 | 4460 6 | - | 25 | 117621690 | 4491132 |

| | | | | | | | |
|---|---|---|---|---|---|---|---|
| Plasmid lp60-2 (Accession number NC_008565) | hypothetical protein BAPKO_2544 | 4508 7 | 4577 0 | - | 227 | 117621691 | 4491133 |
| Plasmid lp60-2 (Accession number NC_008565) | hypothetical protein BAPKO_2545 | 4666 0 | 4697 1 | + | 103 | 117621692 | 4491134 |
| Plasmid lp60-2 (Accession number NC_008565) | hypothetical protein BAPKO_2546 | 4703 5 | 4725 6 | - | 73 | 117621693 | 4491135 |
| Plasmid lp60-2 (Accession number NC_008565) | hypothetical protein BAPKO_2547 | 4785 4 | 4804 2 | + | 62 | 117621694 | 4491136 |
| Plasmid lp60-2 (Accession number NC_008565) | hypothetical protein BAPKO_2548 | 4807 2 | 4829 0 | + | 72 | 117621695 | 4491137 |
| Plasmid lp60-2 (Accession number NC_008565) | hypothetical protein BAPKO_2549 | 4844 2 | 4936 8 | - | 308 | 117621696 | 4491138 |
| Plasmid lp60-2 (Accession number NC_008565) | hypothetical protein BAPKO_2550 | 4936 1 | 4981 9 | - | 152 | 117621697 | 4491139 |
| Plasmid lp60-2 (Accession number NC_008565) | hypothetical protein BAPKO_2551 | 5012 5 | 5042 7 | - | 100 | 117621698 | 4491140 |
| Plasmid lp60-2 (Accession number NC_008565) | hypothetical protein BAPKO_2552 | 5112 7 | 5167 8 | + | 183 | 117621699 | 4491141 |
| Plasmid lp60-2 (Accession number NC_008565) | hypothetical protein BAPKO_2553 | 5183 7 | 5242 4 | - | 195 | 117621700 | 4491142 |
| Plasmid lp60-2 (Accession number NC_008565) | hypothetical protein BAPKO_2554 | 5594 8 | 5705 4 | + | 368 | 117621701 | 4491143 |
| Plasmid lp60-2 (Accession number NC_008565) | hypothetical protein BAPKO_2555 | 5706 4 | 5730 0 | + | 78 | 117621702 | 4491144 |
| Plasmid lp60-2 (Accession number NC_008565) | hypothetical protein BAPKO_2556 | 5729 3 | 5804 5 | + | 250 | 117621703 | 4491145 |
| Plasmid lp60-2 (Accession number NC_008565) | hypothetical protein BAPKO_2557 | 5834 0 | 5868 4 | + | 114 | 117621704 | 4491146 |

| | | | | | | | |
|---|---|---|---|---|---|---|---|
| Plasmid lp60-2 (Accession number NC_008565) | hypothetical protein BAPKO_2558 | 5887 0 | 5932 8 | + | 152 | 117621705 | 4491147 |
| Plasmid lp34 (Accession number NC_008566) | hypothetical protein BAPKO_3000 | 921 | 1748 | + | 275 | 117621707 | 4491157 |
| Plasmid lp34 (Accession number NC_008566) | hypothetical protein BAPKO_3001 | 2473 | 2664 | - | 63 | 117621708 | 4491158 |
| Plasmid lp34 (Accession number NC_008566) | hypothetical protein BAPKO_3002 | 3113 | 3391 | - | 92 | 117621709 | 4491159 |
| Plasmid lp34 (Accession number NC_008566) | hypothetical protein BAPKO_3003 | 3345 | 3806 | - | 153 | 117621710 | 4491160 |
| Plasmid lp34 (Accession number NC_008566) | hypothetical protein BAPKO_3004 | 3796 | 3912 | - | 38 | 117621711 | 4491161 |
| Plasmid lp34 (Accession number NC_008566) | hypothetical protein BAPKO_3005 | 4259 | 4783 | - | 174 | 117621712 | 4491162 |
| Plasmid lp34 (Accession number NC_008566) | hypothetical protein BAPKO_3006 | 5150 | 5473 | - | 107 | 117621713 | 4491163 |
| Plasmid lp34 (Accession number NC_008566) | hypothetical protein BAPKO_3007 | 5657 | 5908 | - | 83 | 117621714 | 4491164 |
| Plasmid lp34 (Accession number NC_008566) | hypothetical protein BAPKO_3008 | 6922 | 7803 | - | 293 | 117621715 | 4491165 |
| Plasmid lp34 (Accession number NC_008566) | hypothetical protein BAPKO_3009 | 8618 | 8731 | + | 37 | 117621716 | 4491166 |
| Plasmid lp34 (Accession number NC_008566) | hypothetical protein BAPKO_3010 | 8863 | 9375 | + | 170 | 117621717 | 4491167 |
| Plasmid lp34 (Accession number NC_008566) | hypothetical protein BAPKO_3011 | 9826 | 1014 3 | - | 105 | 117621718 | 4491168 |
| Plasmid lp34 (Accession number NC_008566) | hypothetical protein BAPKO_3012 | 1142 5 | 1240 2 | + | 325 | 117621719 | 4491169 |

| Plasmid lp34 (Accession number NC_008566) | hypothetical protein BAPKO_3013 | 1239 5 | 1287 4 | + | 159 | 117621720 | 4491170 |
|---|---|---|---|---|---|---|---|
| Plasmid lp34 (Accession number NC_008566) | hypothetical protein BAPKO_3014 | 1295 6 | 1369 3 | + | 245 | 117621721 | 4491171 |
| Plasmid lp34 (Accession number NC_008566) | hypothetical protein BAPKO_3015 | 1374 0 | 1436 0 | + | 206 | 117621722 | 4491172 |
| Plasmid lp34 (Accession number NC_008566) | hypothetical protein BAPKO_3016 | 1578 5 | 1654 0 | - | 251 | 117621723 | 4491173 |
| Plasmid lp34 (Accession number NC_008566) | hypothetical protein BAPKO_3017 | 1787 0 | 1802 8 | + | 52 | 117621724 | 4491174 |
| Plasmid lp34 (Accession number NC_008566) | hypothetical protein BAPKO_3018 | 1823 7 | 1841 0 | + | 57 | 117621725 | 4491175 |
| Plasmid lp34 (Accession number NC_008566) | hypothetical protein BAPKO_3019 | 1870 5 | 1890 5 | + | 66 | 117621726 | 4491176 |
| Plasmid lp34 (Accession number NC_008566) | hypothetical protein BAPKO_3020 | 1948 2 | 1967 9 | - | 65 | 117621727 | 4491177 |
| Plasmid lp34 (Accession number NC_008566) | hypothetical protein BAPKO_3021 | 1980 1 | 2000 7 | - | 68 | 117621728 | 4491178 |
| Plasmid lp34 (Accession number NC_008566) | hypothetical protein BAPKO_3022 | 2067 3 | 2078 0 | - | 35 | 117621729 | 4491179 |
| Plasmid lp34 (Accession number NC_008566) | hypothetical protein BAPKO_3023 | 2084 4 | 2108 0 | - | 78 | 117621730 | 4491180 |
| Plasmid lp34 (Accession number NC_008566) | hypothetical protein BAPKO_3024 | 2217 6 | 2233 7 | - | 53 | 117621731 | 4491181 |
| Plasmid lp34 (Accession number NC_008566) | hypothetical protein BAPKO_3025 | 2408 7 | 2516 3 | - | 358 | 117621732 | 4491182 |
| Plasmid lp34 (Accession number NC_008566) | hypothetical protein BAPKO_3026 | 2551 1 | 2632 6 | + | 271 | 117621733 | 4491183 |

| Plasmid lp34 (Accession number NC_008566) | hypothetical protein BAPKO_3027 | 2625 4 | 2634 3 | - | 29 | 117621734 | 4491148 |
|---|---|---|---|---|---|---|---|
| Plasmid lp34 (Accession number NC_008566) | hypothetical protein BAPKO_3028 | 2635 8 | 2648 9 | + | 43 | 117621735 | 4491149 |
| Plasmid lp34 (Accession number NC_008566) | hypothetical protein BAPKO_3029 | 2650 0 | 2674 8 | + | 82 | 117621736 | 4491150 |
| Plasmid lp34 (Accession number NC_008566) | hypothetical protein BAPKO_3030 | 2700 1 | 2712 3 | + | 40 | 117621737 | 4491151 |
| Plasmid lp34 (Accession number NC_008566) | hypothetical protein BAPKO_3031 | 2847 0 | 2857 1 | + | 33 | 117621738 | 4491152 |
| Plasmid lp34 (Accession number NC_008566) | hypothetical protein BAPKO_3032 | 2851 6 | 2926 2 | + | 248 | 117621739 | 4491153 |
| Plasmid lp34 (Accession number NC_008566) | hypothetical protein BAPKO_3033 | 2984 7 | 3149 3 | + | 548 | 117621740 | 4491154 |
| Plasmid lp34 (Accession number NC_008566) | hypothetical protein BAPKO_3034 | 3239 0 | 3308 5 | - | 231 | 117621741 | 4491155 |
| Plasmid lp34 (Accession number NC_008566) | hypothetical protein BAPKO_3035 | 3348 8 | 3415 3 | + | 221 | 117621742 | 4491156 |
| Plasmid lp32 (Accession number NC_008567) | hypothetical protein BAPKO_3500 | 598 | 1212 | - | 204 | 117621744 | 4491204 |
| Plasmid lp32 (Accession number NC_008567) | hypothetical protein BAPKO_3501 | 1714 | 1890 | + | 58 | 117621745 | 4491205 |
| Plasmid lp32 (Accession number NC_008567) | hypothetical protein BAPKO_3502 | 1863 | 2105 | + | 80 | 117621746 | 4491206 |
| Plasmid lp32 (Accession number NC_008567) | hypothetical protein BAPKO_3503 | 2247 | 2402 | - | 51 | 117621747 | 4491207 |
| Plasmid lp32 (Accession number NC_008567) | hypothetical protein BAPKO_3504 | 2426 | 2809 | + | 127 | 117621748 | 4491208 |

| | | | | | | | |
|---|---|---|---|---|---|---|---|
| Plasmid lp32 (Accession number NC_008567) | hypothetical protein BAPKO_3505 | 2877 | 3035 | + | 52 | 117621749 | 4491209 |
| Plasmid lp32 (Accession number NC_008567) | hypothetical protein BAPKO_3506 | 3342 | 3674 | + | 110 | 117621750 | 4491210 |
| Plasmid lp32 (Accession number NC_008567) | hypothetical protein BAPKO_3507 | 3722 | 4102 | + | 126 | 117621751 | 4491211 |
| Plasmid lp32 (Accession number NC_008567) | hypothetical protein BAPKO_3508 | 4166 | 4321 | + | 51 | 117621752 | 4491212 |
| Plasmid lp32 (Accession number NC_008567) | hypothetical protein BAPKO_3509 | 4522 | 4776 | + | 84 | 117621753 | 4491213 |
| Plasmid lp32 (Accession number NC_008567) | hypothetical protein BAPKO_3510 | 5340 | 5537 | + | 65 | 117621754 | 4491214 |
| Plasmid lp32 (Accession number NC_008567) | hypothetical protein BAPKO_3511 | 5719 | 6273 | + | 184 | 117621755 | 4491215 |
| Plasmid lp32 (Accession number NC_008567) | hypothetical protein BAPKO_3512 | 6397 | 6852 | + | 151 | 117621756 | 4491216 |
| Plasmid lp32 (Accession number NC_008567) | hypothetical protein BAPKO_3513 | 7298 | 7624 | + | 108 | 117621757 | 4491217 |
| Plasmid lp32 (Accession number NC_008567) | hypothetical protein BAPKO_3514 | 7706 | 8302 | + | 198 | 117621758 | 4491218 |
| Plasmid lp32 (Accession number NC_008567) | hypothetical protein BAPKO_3515 | 8264 | 8374 | - | 36 | 117621759 | 4491219 |
| Plasmid lp32 (Accession number NC_008567) | hypothetical protein BAPKO_3516 | 8394 | 8510 | + | 38 | 117621760 | 4491220 |
| Plasmid lp32 (Accession number NC_008567) | hypothetical protein BAPKO_3517 | 9142 | 9468 | - | 108 | 117621761 | 4491221 |
| Plasmid lp32 (Accession number NC_008567) | hypothetical protein BAPKO_3518 | 9888 | 13100 | + | 1070 | 117621762 | 4491222 |

| | | | | | | | |
|---|---|---|---|---|---|---|---|
| Plasmid lp32 (Accession number NC_008567) | hypothetical protein BAPKO_3519 | 1380 3 | 1418 0 | + | 125 | 117621763 | 4491223 |
| Plasmid lp32 (Accession number NC_008567) | hypothetical protein BAPKO_3520 | 1418 3 | 1470 1 | + | 172 | 117621764 | 4491224 |
| Plasmid lp32 (Accession number NC_008567) | hypothetical protein BAPKO_3521 | 1469 1 | 1491 2 | + | 73 | 117621765 | 4491225 |
| Plasmid lp32 (Accession number NC_008567) | hypothetical protein BAPKO_3522 | 1492 8 | 1538 0 | + | 150 | 117621766 | 4491226 |
| Plasmid lp32 (Accession number NC_008567) | hypothetical protein BAPKO_3523 | 1549 6 | 1565 4 | + | 52 | 117621767 | 4491227 |
| Plasmid lp32 (Accession number NC_008567) | hypothetical protein BAPKO_3524 | 1598 3 | 1605 1 | + | 22 | 117621768 | 4491228 |
| Plasmid lp32 (Accession number NC_008567) | hypothetical protein BAPKO_3525 | 1604 4 | 1639 7 | + | 117 | 117621769 | 4491229 |
| Plasmid lp32 (Accession number NC_008567) | hypothetical protein BAPKO_3526 | 1657 1 | 1712 2 | + | 183 | 117621770 | 4491230 |
| Plasmid lp32 (Accession number NC_008567) | hypothetical protein BAPKO_3527 | 1698 4 | 1718 7 | - | 67 | 117621771 | 4491184 |
| Plasmid lp32 (Accession number NC_008567) | hypothetical protein BAPKO_3528 | 1724 0 | 1732 9 | + | 29 | 117621772 | 4491185 |
| Plasmid lp32 (Accession number NC_008567) | hypothetical protein BAPKO_3529 | 1727 4 | 1743 5 | + | 53 | 117621773 | 4491186 |
| Plasmid lp32 (Accession number NC_008567) | hypothetical protein BAPKO_3530 | 1775 6 | 1792 6 | - | 56 | 117621774 | 4491187 |
| Plasmid lp32 (Accession number NC_008567) | hypothetical protein BAPKO_3531 | 1787 8 | 1856 1 | - | 227 | 117621775 | 4491188 |
| Plasmid lp32 (Accession number NC_008567) | hypothetical protein BAPKO_3532 | 1863 7 | 1879 5 | - | 52 | 117621776 | 4491189 |

| | | | | | | | |
|---|---|---|---|---|---|---|---|
| Plasmid lp32 (Accession number NC_008567) | hypothetical protein BAPKO_3533 | 1918 7 | 2027 8 | + | 363 | 117621777 | 4491190 |
| Plasmid lp32 (Accession number NC_008567) | hypothetical protein BAPKO_3534 | 2028 8 | 2084 5 | + | 185 | 117621778 | 4491191 |
| Plasmid lp32 (Accession number NC_008567) | hypothetical protein BAPKO_3535 | 2082 1 | 2157 6 | + | 251 | 117621779 | 4491192 |
| Plasmid lp32 (Accession number NC_008567) | hypothetical protein BAPKO_3536 | 2162 0 | 2218 0 | + | 186 | 117621780 | 4491193 |
| Plasmid lp32 (Accession number NC_008567) | hypothetical protein BAPKO_3537 | 2219 3 | 2244 4 | + | 83 | 117621781 | 4491194 |
| Plasmid lp32 (Accession number NC_008567) | hypothetical protein BAPKO_3538 | 2296 5 | 2308 7 | + | 40 | 117621782 | 4491195 |
| Plasmid lp32 (Accession number NC_008567) | hypothetical protein BAPKO_3539 | 2355 5 | 2383 6 | - | 93 | 117621783 | 4491196 |
| Plasmid lp32 (Accession number NC_008567) | hypothetical protein BAPKO_3540 | 2384 8 | 2429 1 | - | 147 | 117621784 | 4491197 |
| Plasmid lp32 (Accession number NC_008567) | hypothetical protein BAPKO_3541 | 2430 3 | 2452 1 | - | 72 | 117621785 | 4491198 |
| Plasmid lp32 (Accession number NC_008567) | outer surface protein D | 2555 5 | 2603 4 | + | 159 | 117621786 | 4491199 |
| Plasmid lp32 (Accession number NC_008567) | hypothetical protein BAPKO_3543 | 2614 8 | 2628 2 | + | 44 | 117621787 | 4491200 |
| Plasmid lp32 (Accession number NC_008567) | hypothetical protein BAPKO_3544 | 2653 0 | 2659 5 | - | 21 | 117621788 | 4491201 |
| Plasmid lp32 (Accession number NC_008567) | hypothetical protein BAPKO_3545 | 2659 8 | 2684 0 | - | 80 | 117621789 | 4491202 |
| Plasmid lp32 (Accession number NC_008567) | hypothetical protein BAPKO_3546 | 2755 1 | 2768 5 | - | 44 | 117621790 | 4491203 |

| | | | | | | | |
|---|---|---|---|---|---|---|---|
| Plasmid lp32 (Accession number NC_008567) | hypothetical protein BAPKO_3547 | 2809 9 | 2893 5 | + | 278 | 117621791 | 4491231 |
| Plasmid lp32 (Accession number NC_008567) | hypothetical protein BAPKO_3548 | 2933 1 | 2985 2 | - | 173 | 117621792 | 4491232 |
| Plasmid lp32 (Accession number NC_008567) | hypothetical protein BAPKO_3549 | 2989 5 | 3008 6 | - | 63 | 117621793 | 4491233 |
| Plasmid lp32 (Accession number NC_008567) | hypothetical protein BAPKO_3550 | 3022 8 | 3045 2 | + | 74 | 117621794 | 4491234 |
| Plasmid lp32 (Accession number NC_008567) | hypothetical protein BAPKO_3551 | 3044 5 | 3049 8 | + | 17 | 117621795 | 4491235 |
| Plasmid lp32 (Accession number NC_008567) | hypothetical protein BAPKO_3552 | 3052 1 | 3064 0 | + | 39 | 117621796 | 4491236 |
| Plasmid lp32 (Accession number NC_008567) | hypothetical protein BAPKO_3553 | 3080 9 | 3115 6 | + | 115 | 117621797 | 4491237 |
| Plasmid lp32 (Accession number NC_008567) | hypothetical protein BAPKO_3554 | 3121 5 | 3184 1 | + | 208 | 117621798 | 4491238 |
| Plasmid lp28 (Accession number NC_008568) | hypothetical protein BAPKO_4500 | 351 | 542 | - | 63 | 117621800 | 4491239 |
| Plasmid lp28 (Accession number NC_008568) | hypothetical protein BAPKO_4501 | 699 | 926 | - | 75 | 117621801 | 4491240 |
| Plasmid lp28 (Accession number NC_008568) | hypothetical protein BAPKO_4502 | 1398 | 1670 | - | 90 | 117621802 | 4491241 |
| Plasmid lp28 (Accession number NC_008568) | hypothetical protein BAPKO_4503 | 1661 | 2401 | - | 246 | 117621803 | 4491242 |
| Plasmid lp28 (Accession number NC_008568) | hypothetical protein BAPKO_4504 | 4910 | 5569 | + | 219 | 117621804 | 4491243 |
| Plasmid lp28 (Accession number NC_008568) | hypothetical protein BAPKO_4505 | 6396 | 7079 | + | 227 | 117621805 | 4491244 |

| | | | | | | | |
|---|---|---|---|---|---|---|---|
| Plasmid lp28 (Accession number NC_008568) | hypothetical protein BAPKO_4506 | 7231 | 7809 | - | 192 | 117621806 | 4491245 |
| Plasmid lp28 (Accession number NC_008568) | hypothetical protein BAPKO_4507 | 7986 | 8990 | + | 334 | 117621807 | 4491246 |
| Plasmid lp28 (Accession number NC_008568) | hypothetical protein BAPKO_4508 | 9140 | 9466 | + | 108 | 117621808 | 4491254 |
| Plasmid lp28 (Accession number NC_008568) | hypothetical protein BAPKO_4509 | 9622 | 1072 8 | + | 368 | 117621809 | 4491255 |
| Plasmid lp28 (Accession number NC_008568) | hypothetical protein BAPKO_4510 | 1060 0 | 1083 3 | - | 77 | 117621810 | 4491256 |
| Plasmid lp28 (Accession number NC_008568) | hypothetical protein BAPKO_4511 | 1168 1 | 1185 7 | + | 58 | 117621811 | 4491257 |
| Plasmid lp28 (Accession number NC_008568) | hypothetical protein BAPKO_4512 | 1191 6 | 1297 4 | + | 352 | 117621812 | 4491258 |
| Plasmid lp28 (Accession number NC_008568) | hypothetical protein BAPKO_4513 | 1391 9 | 1421 8 | + | 99 | 117621813 | 4491259 |
| Plasmid lp28 (Accession number NC_008568) | hypothetical protein BAPKO_4514 | 1460 0 | 1538 5 | - | 261 | 117621814 | 4491260 |
| Plasmid lp28 (Accession number NC_008568) | hypothetical protein BAPKO_4515 | 1640 0 | 1733 8 | - | 312 | 117621815 | 4491261 |
| Plasmid lp28 (Accession number NC_008568) | hypothetical protein BAPKO_4516 | 1746 7 | 1759 2 | + | 41 | 117621816 | 4491262 |
| Plasmid lp28 (Accession number NC_008568) | hypothetical protein BAPKO_4517 | 1806 8 | 1858 6 | - | 172 | 117621817 | 4491263 |
| Plasmid lp28 (Accession number NC_008568) | hypothetical protein BAPKO_4518 | 1886 6 | 1895 5 | + | 29 | 117621818 | 4491264 |
| Plasmid lp28 (Accession number NC_008568) | hypothetical protein BAPKO_4519 | 1904 5 | 1921 2 | + | 55 | 117621819 | 4491265 |

| | | | | | | | |
|---|---|---|---|---|---|---|---|
| Plasmid lp28 (Accession number NC_008568) | hypothetical protein BAPKO_4520 | 1940 6 | 2005 3 | + | 215 | 117621820 | 4491266 |
| Plasmid lp28 (Accession number NC_008568) | hypothetical protein BAPKO_4521 | 2023 8 | 2057 0 | - | 110 | 117621821 | 4491267 |
| Plasmid lp28 (Accession number NC_008568) | hypothetical protein BAPKO_4522 | 2090 5 | 2204 1 | + | 378 | 117621822 | 4491268 |
| Plasmid lp28 (Accession number NC_008568) | hypothetical protein BAPKO_4523 | 2212 7 | 2234 5 | - | 72 | 117621823 | 4491269 |
| Plasmid lp28 (Accession number NC_008568) | hypothetical protein BAPKO_4524 | 2233 8 | 2245 7 | - | 39 | 117621824 | 4491270 |
| Plasmid lp28 (Accession number NC_008568) | hypothetical protein BAPKO_4525 | 2282 8 | 2290 8 | + | 26 | 117621825 | 4491271 |
| Plasmid lp28 (Accession number NC_008568) | hypothetical protein BAPKO_4526 | 2290 3 | 2346 9 | - | 188 | 117621826 | 4491272 |
| Plasmid lp28 (Accession number NC_008568) | hypothetical protein BAPKO_4527 | 2347 5 | 2370 8 | - | 77 | 117621827 | 4491247 |
| Plasmid lp28 (Accession number NC_008568) | hypothetical protein BAPKO_4528 | 2395 3 | 2472 9 | + | 258 | 117621828 | 4491248 |
| Plasmid lp28 (Accession number NC_008568) | hypothetical protein BAPKO_4529 | 2475 7 | 2507 4 | + | 105 | 117621829 | 4491249 |
| Plasmid lp28 (Accession number NC_008568) | hypothetical protein BAPKO_4530 | 2509 1 | 2644 6 | - | 451 | 117621830 | 4491250 |
| Plasmid lp28 (Accession number NC_008568) | hypothetical protein BAPKO_4531 | 2642 7 | 2775 5 | - | 442 | 117621831 | 4491251 |
| Plasmid lp28 (Accession number NC_008568) | hypothetical protein BAPKO_4532 | 2797 4 | 2815 6 | - | 60 | 117621832 | 4491252 |
| Plasmid lp28 (Accession number NC_008568) | hypothetical protein BAPKO_4533 | 2838 9 | 2852 6 | - | 45 | 117621833 | 4491253 |

| | | | | | | | |
|---|---|---|---|---|---|---|---|
| Plasmid lp25 (Accession number NC_008569) | hypothetical protein BAPKO_5500 | 685 | 1254 | - | 189 | 117621835 | 4491275 |
| Plasmid lp25 (Accession number NC_008569) | hypothetical protein BAPKO_5501 | 1351 | 2727 | - | 458 | 117621836 | 4491276 |
| Plasmid lp25 (Accession number NC_008569) | hypothetical protein BAPKO_5502 | 2747 | 3385 | - | 212 | 117621837 | 4491277 |
| Plasmid lp25 (Accession number NC_008569) | hypothetical protein BAPKO_5503 | 3469 | 3903 | - | 144 | 117621838 | 4491278 |
| Plasmid lp25 (Accession number NC_008569) | hypothetical protein BAPKO_5504 | 3928 | 5292 | - | 454 | 117621839 | 4491279 |
| Plasmid lp25 (Accession number NC_008569) | hypothetical protein BAPKO_5505 | 5658 | 6032 | + | 124 | 117621840 | 4491280 |
| Plasmid lp25 (Accession number NC_008569) | hypothetical protein BAPKO_5506 | 6025 | 6471 | + | 148 | 117621841 | 4491281 |
| Plasmid lp25 (Accession number NC_008569) | hypothetical protein BAPKO_5507 | 6497 | 6829 | + | 110 | 117621842 | 4491282 |
| Plasmid lp25 (Accession number NC_008569) | hypothetical protein BAPKO_5508 | 6842 | 9544 | + | 900 | 117621843 | 4491283 |
| Plasmid lp25 (Accession number NC_008569) | hypothetical protein BAPKO_5509 | 9544 | 1037 4 | + | 276 | 117621844 | 4491284 |
| Plasmid lp25 (Accession number NC_008569) | hypothetical protein BAPKO_5510 | 1038 3 | 1072 1 | + | 112 | 117621845 | 4491285 |
| Plasmid lp25 (Accession number NC_008569) | hypothetical protein BAPKO_5511 | 1073 8 | 1113 0 | + | 130 | 117621846 | 4491286 |
| Plasmid lp25 (Accession number NC_008569) | hypothetical protein BAPKO_5512 | 1112 7 | 1157 0 | + | 147 | 117621847 | 4491287 |
| Plasmid lp25 (Accession number NC_008569) | hypothetical protein BAPKO_5513 | 1156 0 | 1237 8 | + | 272 | 117621848 | 4491288 |

| | | | | | | | |
|---|---|---|---|---|---|---|---|
| Plasmid lp25 (Accession number NC_008569) | hypothetical protein BAPKO_5514 | 1239 9 | 1282 7 | + | 142 | 117621849 | 4491289 |
| Plasmid lp25 (Accession number NC_008569) | hypothetical protein BAPKO_5515 | 1291 7 | 1311 4 | + | 65 | 117621850 | 4491290 |
| Plasmid lp25 (Accession number NC_008569) | hypothetical protein BAPKO_5516 | 1311 1 | 1498 8 | + | 625 | 117621851 | 4491291 |
| Plasmid lp25 (Accession number NC_008569) | hypothetical protein BAPKO_5517 | 1500 6 | 1545 8 | + | 150 | 117621852 | 4491292 |
| Plasmid lp25 (Accession number NC_008569) | hypothetical protein BAPKO_5518 | 1548 7 | 1556 4 | + | 25 | 117621853 | 4491293 |
| Plasmid lp25 (Accession number NC_008569) | hypothetical protein BAPKO_5519 | 1556 5 | 1611 6 | + | 183 | 117621854 | 4491294 |
| Plasmid lp25 (Accession number NC_008569) | hypothetical protein BAPKO_5520 | 1651 4 | 1708 6 | + | 190 | 117621855 | 4491295 |
| Plasmid lp25 (Accession number NC_008569) | hypothetical protein BAPKO_5521 | 1723 6 | 1777 5 | + | 179 | 117621856 | 4491296 |
| Plasmid lp25 (Accession number NC_008569) | hypothetical protein BAPKO_5522 | 1775 1 | 1851 2 | + | 253 | 117621857 | 4491297 |
| Plasmid lp25 (Accession number NC_008569) | hypothetical protein BAPKO_5523 | 1858 4 | 1914 1 | + | 185 | 117621858 | 4491298 |
| Plasmid lp25 (Accession number NC_008569) | hypothetical protein BAPKO_5524 | 1929 6 | 1983 8 | - | 180 | 117621859 | 4491299 |
| Plasmid lp25 (Accession number NC_008569) | hypothetical protein BAPKO_5525 | 1986 9 | 2041 1 | - | 180 | 117621860 | 4491300 |
| Plasmid lp25 (Accession number NC_008569) | hypothetical protein BAPKO_5526 | 2054 5 | 2272 2 | + | 725 | 117621861 | 4491301 |
| Plasmid lp25 (Accession number NC_008569) | hypothetical protein BAPKO_5527 | 2265 0 | 2378 0 | + | 376 | 117621862 | 4491273 |

| | | | | | | | |
|---|---|---|---|---|---|---|---|
| Plasmid lp25 (Accession number NC_008569) | hypothetical protein BAPKO_5528 | 2384 4 | 2434 7 | - | 167 | 117621863 | 4491274 |
| Complete genome (Accession number NC_008277) | hypothetical protein BAPKO_0001 | 531 | 1550 | - | 339 | 111114824 | 4227028 |
| Complete genome (Accession number NC_008277) | hypothetical protein BAPKO_0002 | 1538 | 3013 | - | 491 | 111114825 | 4227029 |
| Complete genome (Accession number NC_008277) | phosphogluco mutase | 3156 | 4946 | + | 596 | 111114826 | 4227030 |
| Complete genome (Accession number NC_008277) | tryptophanyl-tRNA synthetase | 5023 | 6069 | - | 348 | 111114827 | 4227031 |
| Complete genome (Accession number NC_008277) | hypothetical protein BAPKO_0005 | 6072 | 7160 | - | 362 | 111114828 | 4227032 |
| Complete genome (Accession number NC_008277) | hypothetical protein BAPKO_0006 | 7218 | 8075 | - | 285 | 111114829 | 4227033 |
| Complete genome (Accession number NC_008277) | hypothetical protein BAPKO_0007 | 8184 | 8954 | + | 256 | 111114830 | 4227034 |
| Complete genome (Accession number NC_008277) | hypothetical protein BAPKO_0008 | 8959 | 9963 | + | 334 | 111114831 | 4227174 |
| Complete genome (Accession number NC_008277) | holo-acyl-carrier protein synthase, putative | 9960 | 1033 4 | + | 124 | 111114832 | 4227175 |
| Complete genome (Accession number NC_008277) | hypothetical protein BAPKO_0010 | 1033 8 | 1117 7 | + | 279 | 111114833 | 4227176 |
| Complete genome (Accession | tRNA pseudouridine synthase A | 1117 8 | 1191 8 | + | 246 | 111114834 | 4227177 |

| | | | | | | | |
|---|---|---|---|---|---|---|---|
| number NC_008277) | | | | | | | |
| Complete genome (Accession number NC_008277) | hypothetical protein BAPKO_0012 | 1191 1 | 1251 0 | + | 199 | 111114835 | 4227178 |
| Complete genome (Accession number NC_008277) | primosomal protein N | 1250 3 | 1449 4 | + | 663 | 111114836 | 4227179 |
| Complete genome (Accession number NC_008277) | uridine kinase | 1449 1 | 1511 4 | - | 207 | 111114837 | 4227180 |
| Complete genome (Accession number NC_008277) | glpE protein | 1511 4 | 1548 8 | - | 124 | 111114838 | 4227181 |
| Complete genome (Accession number NC_008277) | hypothetical protein BAPKO_0016 | 1561 6 | 1659 0 | + | 324 | 111114839 | 4227182 |
| Complete genome (Accession number NC_008277) | hypothetical protein BAPKO_0017 | 1658 7 | 1756 7 | - | 326 | 111114840 | 4227183 |
| Complete genome (Accession number NC_008277) | hypothetical protein BAPKO_0018 | 1757 4 | 1808 6 | - | 170 | 111114841 | 4227184 |
| Complete genome (Accession number NC_008277) | diphosphate-- fructose-6- phosphate 1- phosphotransfe rase | 1809 4 | 1976 1 | - | 555 | 111114842 | 4227185 |
| Complete genome (Accession number NC_008277) | S- adenosylmethio nine: tRNA ribosyltransfera se- isomerase | 1984 1 | 2088 1 | - | 346 | 111114843 | 4227186 |
| Complete genome (Accession number NC_008277) | Holliday junction DNA helicase B | 2085 6 | 2189 9 | - | 347 | 111114844 | 4227187 |
| Complete genome (Accession number | Holliday junction DNA helicase | 2195 9 | 2254 9 | - | 196 | 111114845 | 4227188 |

| NC_008277) | | | | | | | |
|---|---|---|---|---|---|---|---|
| Complete genome (Accession number NC_008277) | hypothetical protein BAPKO_0023 | 2261 9 | 2375 8 | + | 379 | 111114846 | 4227189 |
| Complete genome (Accession number NC_008277) | hypothetical protein BAPKO_0024 | 2377 3 | 2450 4 | - | 243 | 111114847 | 4227190 |
| Complete genome (Accession number NC_008277) | methylenetetra hydrofolate dehydrogenase | 2450 5 | 2541 0 | - | 301 | 111114848 | 4227191 |
| Complete genome (Accession number NC_008277) | hypothetical protein BAPKO_0026 | 2555 7 | 2619 2 | + | 211 | 111114849 | 4227192 |
| Complete genome (Accession number NC_008277) | lipoprotein, putative | 2620 0 | 2724 9 | + | 349 | 111114850 | 4227035 |
| Complete genome (Accession number NC_008277) | hypothetical protein BAPKO_0028 | 2723 5 | 2766 6 | - | 143 | 111114851 | 4227036 |
| Complete genome (Accession number NC_008277) | signal peptidase I | 2765 6 | 2829 1 | - | 211 | 111114852 | 4227037 |
| Complete genome (Accession number NC_008277) | signal peptidase I | 2829 3 | 2927 3 | - | 326 | 111114853 | 4227038 |
| Complete genome (Accession number NC_008277) | hypothetical protein BAPKO_0031 | 2934 4 | 3080 7 | + | 487 | 111114854 | 4227039 |
| Complete genome (Accession number NC_008277) | SsrA-binding protein | 3081 0 | 3126 2 | + | 150 | 111114855 | 4227040 |
| Complete genome (Accession number | hypothetical protein BAPKO_0033 | 3113 3 | 3128 5 | - | 50 | 111114856 | 4227041 |

| | | | | | | | |
|---|---|---|---|---|---|---|---|
| NC_008277) | | | | | | | |
| Complete genome (Accession number NC_008277) | hypothetical protein BAPKO_0034 | 3134 4 | 3188 0 | - | 178 | 111114857 | 4227042 |
| Complete genome (Accession number NC_008277) | DNA topoisomerase IV subunit A | 3194 8 | 3382 8 | - | 626 | 111114858 | 4227043 |
| Complete genome (Accession number NC_008277) | DNA topoisomerase IV subunit B | 3382 8 | 3562 7 | - | 599 | 111114859 | 4227044 |
| Complete genome (Accession number NC_008277) | 1-acyl-sn-glycerol-3-phosphate acyltransferase | 3564 8 | 3638 5 | - | 245 | 111114860 | 4227045 |
| Complete genome (Accession number NC_008277) | hypothetical protein BAPKO_0038 | 3639 4 | 3791 7 | - | 507 | 111114861 | 4227046 |
| Complete genome (Accession number NC_008277) | hypothetical protein BAPKO_0039 | 3793 0 | 3943 2 | - | 500 | 111114862 | 4227047 |
| Complete genome (Accession number NC_008277) | chemotaxis protein methyltransfera se | 3962 2 | 4047 9 | + | 285 | 111114863 | 4227048 |
| Complete genome (Accession number NC_008277) | phosphate transport system regulatory protein | 4054 4 | 4121 5 | - | 223 | 111114864 | 4227049 |
| Complete genome (Accession number NC_008277) | hypothetical protein BAPKO_0042 | 4122 6 | 4226 0 | - | 344 | 111114865 | 4227050 |
| Complete genome (Accession number NC_008277) | hypothetical protein BAPKO_0043 | 4225 7 | 4265 8 | - | 133 | 111114866 | 4227051 |
| Complete genome (Accession number | P115 protein | 4268 4 | 4513 4 | - | 816 | 111114867 | 4227052 |

| NC_008277) | | | | | | | |
|---|---|---|---|---|---|---|---|
| Complete genome (Accession number NC_008277) | ribonuclease H | 4522 7 | 4577 2 | + | 181 | 111114868 | 4227053 |
| Complete genome (Accession number NC_008277) | hypothetical protein BAPKO_0047 | 4579 0 | 4616 1 | - | 123 | 111114869 | 4227054 |
| Complete genome (Accession number NC_008277) | hypothetical protein BAPKO_0049 | 4669 5 | 4681 4 | - | 39 | 111114870 | 4227592 |
| Complete genome (Accession number NC_008277) | hypothetical protein BAPKO_0050 | 4693 3 | 4760 7 | + | 224 | 111114871 | 4227593 |
| Complete genome (Accession number NC_008277) | hypothetical protein BAPKO_0051 | 4765 4 | 4837 3 | + | 239 | 111114872 | 4227594 |
| Complete genome (Accession number NC_008277) | spoU protein | 4837 0 | 4903 8 | - | 222 | 111114873 | 4227595 |
| Complete genome (Accession number NC_008277) | uracil-DNA glycosylase | 4910 8 | 4977 9 | + | 223 | 111114874 | 4227596 |
| Complete genome (Accession number NC_008277) | preprotein translocase subunit SecG | 4985 3 | 5021 5 | - | 120 | 111114875 | 4227597 |
| Complete genome (Accession number NC_008277) | triosephosphat e isomerase | 5023 1 | 5099 2 | - | 253 | 111114876 | 4227598 |
| Complete genome (Accession number NC_008277) | phosphoglycer ate kinase | 5099 4 | 5217 5 | - | 393 | 111114877 | 4227599 |
| Complete genome (Accession number NC_008277) | glyceraldehyde 3-phosphate dehydrogenase | 5220 0 | 5320 7 | - | 335 | 111114878 | 4227600 |

| | | | | | | | |
|---|---|---|---|---|---|---|---|
| Complete genome (Accession number NC_008277) | hypothetical protein BAPKO_0058 | 5328 2 | 5525 2 | - | 656 | 111114879 | 4227601 |
| Complete genome (Accession number NC_008277) | hemolysin | 5524 9 | 5602 8 | - | 259 | 111114880 | 4227602 |
| Complete genome (Accession number NC_008277) | hypothetical protein BAPKO_0060 | 5602 9 | 5648 7 | - | 152 | 111114881 | 4227603 |
| Complete genome (Accession number NC_008277) | thioredoxin | 5657 0 | 5692 3 | - | 117 | 111114882 | 4227604 |
| Complete genome (Accession number NC_008277) | hypothetical protein BAPKO_0062 | 5699 7 | 5771 3 | + | 238 | 111114883 | 4227605 |
| Complete genome (Accession number NC_008277) | hypothetical protein BAPKO_0063 | 5776 4 | 5877 1 | - | 335 | 111114884 | 4227606 |
| Complete genome (Accession number NC_008277) | methionyl-tRNA formyltransferase | 5882 1 | 5976 8 | - | 315 | 111114885 | 4227607 |
| Complete genome (Accession number NC_008277) | polypeptide deformylase | 5976 5 | 6026 2 | - | 165 | 111114886 | 4227608 |
| Complete genome (Accession number NC_008277) | hypothetical protein BAPKO_0066 | 6026 9 | 6032 2 | - | 17 | 111114887 | 4227722 |
| Complete genome (Accession number NC_008277) | hypothetical protein BAPKO_0067 | 6029 5 | 6098 1 | - | 228 | 111114888 | 4227723 |
| Complete genome (Accession number NC_008277) | peptidase, putative | 6097 8 | 6275 6 | - | 592 | 111114889 | 4227724 |

| | | | | | | | |
|---|---|---|---|---|---|---|---|
| Complete genome (Accession number NC_008277) | hypothetical protein BAPKO_0069 | 6287 1 | 6377 9 | + | 302 | 111114890 | 4227725 |
| Complete genome (Accession number NC_008277) | aminopeptidase II | 6376 4 | 6500 2 | + | 412 | 111114891 | 4227726 |
| Complete genome (Accession number NC_008277) | hypothetical protein BAPKO_0071 | 6501 7 | 6547 8 | + | 153 | 111114892 | 4227727 |
| Complete genome (Accession number NC_008277) | hypothetical protein BAPKO_0072 | 6549 5 | 6696 7 | + | 490 | 111114893 | 4227728 |
| Complete genome (Accession number NC_008277) | hypothetical protein BAPKO_0073 | 6697 5 | 6929 3 | + | 772 | 111114894 | 4227729 |
| Complete genome (Accession number NC_008277) | hypothetical protein BAPKO_0074 | 6929 0 | 6985 0 | + | 186 | 111114895 | 4227730 |
| Complete genome (Accession number NC_008277) | peptide chain release factor 2 | 7013 4 | 7094 3 | + | 269 | 111114896 | 4227731 |
| Complete genome (Accession number NC_008277) | hypothetical protein BAPKO_0076 | 7092 5 | 7233 4 | + | 469 | 111114897 | 4227732 |
| Complete genome (Accession number NC_008277) | signal recognition particle-docking protein FtsY | 7237 1 | 7321 6 | + | 281 | 111114898 | 4227733 |
| Complete genome (Accession number NC_008277) | hypothetical protein BAPKO_0078 | 7321 3 | 7424 1 | + | 342 | 111114899 | 4227734 |
| Complete genome (Accession number NC_008277) | hypothetical protein BAPKO_0079 | 7424 2 | 7549 5 | + | 417 | 111114900 | 4227735 |

| | | | | | | | |
|---|---|---|---|---|---|---|---|
| Complete genome (Accession number NC_008277) | ABC transporter, ATP-binding protein | 7548 5 | 7617 7 | + | 230 | 111114901 | 4227736 |
| Complete genome (Accession number NC_008277) | hypothetical protein BAPKO_0081 | 7617 4 | 7742 7 | + | 417 | 111114902 | 4227737 |
| Complete genome (Accession number NC_008277) | hypothetical protein BAPKO_0082 | 7743 8 | 7873 3 | - | 431 | 111114903 | 4227738 |
| Complete genome (Accession number NC_008277) | hypothetical protein BAPKO_0083 | 7872 6 | 7904 6 | - | 106 | 111114904 | 4227829 |
| Complete genome (Accession number NC_008277) | nifS protein | 7923 1 | 8049 9 | + | 422 | 111114905 | 4227424 |
| Complete genome (Accession number NC_008277) | hypothetical protein BAPKO_0085 | 8070 9 | 8113 7 | + | 142 | 111114906 | 4227425 |
| Complete genome (Accession number NC_008277) | hypothetical protein BAPKO_0086 | 8113 4 | 8266 9 | + | 511 | 111114907 | 4227333 |
| Complete genome (Accession number NC_008277) | hypothetical protein BAPKO_0087 | 8250 7 | 8269 8 | - | 63 | 111114908 | 4227334 |
| Complete genome (Accession number NC_008277) | L-lactate dehydrogenase | 8269 9 | 8364 9 | - | 316 | 111114909 | 4227335 |
| Complete genome (Accession number NC_008277) | GTP-binding protein LepA | 8377 0 | 8557 5 | - | 601 | 111114910 | 4227336 |
| Complete genome (Accession number NC_008277) | hypothetical protein BAPKO_0090 | 8560 9 | 8657 1 | - | 320 | 111114911 | 4227337 |

| | | | | | | | |
|---|---|---|---|---|---|---|---|
| Complete genome (Accession number NC_008277) | V-type ATP synthase subunit K | 8665 8 | 8709 2 | - | 144 | 111114912 | 4227338 |
| Complete genome (Accession number NC_008277) | V-type ATP synthase subunit I | 8710 9 | 8896 2 | - | 617 | 111114913 | 4227339 |
| Complete genome (Accession number NC_008277) | V-type ATP synthase subunit D | 8894 6 | 8954 5 | - | 199 | 111114914 | 4227340 |
| Complete genome (Accession number NC_008277) | V-type ATP synthase subunit B | 8954 8 | 9085 2 | - | 434 | 111114915 | 4227341 |
| Complete genome (Accession number NC_008277) | V-type ATP synthase subunit A | 9087 4 | 9262 8 | - | 584 | 111114916 | 4227342 |
| Complete genome (Accession number NC_008277) | hypothetical protein BAPKO_0096 | 9261 5 | 9315 7 | - | 180 | 111114917 | 4227343 |
| Complete genome (Accession number NC_008277) | V-type ATP synthase subunit E | 9317 0 | 9376 9 | - | 199 | 111114918 | 4227344 |
| Complete genome (Accession number NC_008277) | hypothetical protein BAPKO_0098 | 9395 5 | 9434 1 | - | 128 | 111114919 | 4227345 |
| Complete genome (Accession number NC_008277) | DNA mismatch repair protein, putative | 9434 7 | 9668 3 | - | 778 | 111114920 | 4227346 |
| Complete genome (Accession number NC_008277) | hypothetical protein BAPKO_0100 | 9667 3 | 9759 6 | - | 307 | 111114921 | 4227347 |
| Complete genome (Accession number NC_008277) | glutamate racemase | 9759 3 | 9837 8 | - | 261 | 111114922 | 4227348 |

| | | | | | | | |
|---|---|---|---|---|---|---|---|
| Complete genome (Accession number NC_008277) | asparaginyl-tRNA synthetase | 9854 0 | 9992 8 | + | 462 | 111114923 | 4227349 |
| Complete genome (Accession number NC_008277) | hypothetical protein BAPKO_0103 | 9995 1 | 1004 72 | + | 173 | 111114924 | 4227350 |
| Complete genome (Accession number NC_008277) | hypothetical protein BAPKO_0104 | 1004 69 | 1010 62 | - | 197 | 111114925 | 4227351 |
| Complete genome (Accession number NC_008277) | periplasmic serine protease DO | 1011 93 | 1026 17 | - | 474 | 111114926 | 4227385 |
| Complete genome (Accession number NC_008277) | methionine aminopeptidas e | 1026 84 | 1034 39 | - | 251 | 111114927 | 4227386 |
| Complete genome (Accession number NC_008277) | hypothetical protein BAPKO_0107 | 1034 32 | 1050 99 | - | 555 | 111114928 | 4227387 |
| Complete genome (Accession number NC_008277) | transcription antitermination protein NusB | 1051 01 | 1055 14 | - | 137 | 111114929 | 4227388 |
| Complete genome (Accession number NC_008277) | basic membrane protein | 1055 51 | 1065 61 | - | 336 | 111114930 | 4227389 |
| Complete genome (Accession number NC_008277) | acetyl-CoA C-acetyltransfera se | 1066 39 | 1078 32 | - | 397 | 111114931 | 4227390 |
| Complete genome (Accession number NC_008277) | hypothetical protein BAPKO_0111 | 1079 47 | 1093 11 | + | 454 | 111114932 | 4227391 |
| Complete genome (Accession number NC_008277) | replicative DNA helicase | 1093 28 | 1106 95 | - | 455 | 111114933 | 4227392 |

| | | | | | | | |
|---|---|---|---|---|---|---|---|
| Complete genome (Accession number NC_008277) | 50S ribosomal protein L9 | 1106 99 | 1112 20 | - | 173 | 111114934 | 4227393 |
| Complete genome (Accession number NC_008277) | 30S ribosomal protein S18 | 1112 37 | 1115 27 | - | 96 | 111114935 | 4227394 |
| Complete genome (Accession number NC_008277) | single-stranded DNA-binding protein | 1115 42 | 1119 91 | - | 149 | 111114936 | 4227395 |
| Complete genome (Accession number NC_008277) | 30S ribosomal protein S6 | 1120 03 | 1124 22 | - | 139 | 111114937 | 4227396 |
| Complete genome (Accession number NC_008277) | PTS system, maltose and glucose-specific IIABC component | 1125 61 | 1142 55 | - | 564 | 111114938 | 4227397 |
| Complete genome (Accession number NC_008277) | hemolysin III | 1144 57 | 1151 58 | + | 233 | 111114939 | 4227398 |
| Complete genome (Accession number NC_008277) | zinc protease, putative | 1151 55 | 1164 56 | - | 433 | 111114940 | 4227399 |
| Complete genome (Accession number NC_008277) | phosphatidate cytidylyltransfer ase | 1164 78 | 1173 20 | - | 280 | 111114941 | 4227400 |
| Complete genome (Accession number NC_008277) | undecaprenyl diphosphate synthase | 1173 25 | 1180 17 | - | 230 | 111114942 | 4227401 |
| Complete genome (Accession number NC_008277) | ribosome releasing factor | 1180 23 | 1185 77 | - | 184 | 111114943 | 4227402 |
| Complete genome (Accession number NC_008277) | elongation factor Ts | 1186 15 | 1194 54 | - | 279 | 111114944 | 4227403 |

| | | | | | | | |
|---|---|---|---|---|---|---|---|
| Complete genome (Accession number NC_008277) | 30S ribosomal protein S2 | 1194 51 | 1202 24 | - | 257 | 111114945 | 4227404 |
| Complete genome (Accession number NC_008277) | hypothetical protein BAPKO_0125 | 1204 52 | 1207 30 | - | 92 | 111114946 | 4227366 |
| Complete genome (Accession number NC_008277) | hypothetical protein BAPKO_0126 | 1205 61 | 1207 40 | - | 59 | 111114947 | 4227367 |
| Complete genome (Accession number NC_008277) | hypothetical protein BAPKO_0127 | 1207 49 | 1213 90 | - | 213 | 111114948 | 4227368 |
| Complete genome (Accession number NC_008277) | hypothetical protein BAPKO_0128 | 1215 31 | 1221 42 | - | 203 | 111114949 | 4227369 |
| Complete genome (Accession number NC_008277) | 30S ribosomal protein S1 | 1221 42 | 1237 97 | - | 551 | 111114950 | 4227370 |
| Complete genome (Accession number NC_008277) | cytidylate kinase | 1238 00 | 1244 65 | - | 221 | 111114951 | 4227371 |
| Complete genome (Accession number NC_008277) | hypothetical protein BAPKO_0131 | 1244 49 | 1251 98 | - | 249 | 111114952 | 4227372 |
| Complete genome (Accession number NC_008277) | hypothetical protein BAPKO_0132 | 1251 85 | 1259 49 | - | 254 | 111114953 | 4227373 |
| Complete genome (Accession number NC_008277) | recombinase A | 1259 64 | 1270 61 | - | 365 | 111114954 | 4227374 |
| Complete genome (Accession number NC_008277) | transcript cleavage factor/unknown domain fusion protein | 1270 75 | 1297 77 | - | 900 | 111114955 | 4227375 |

| | | | | | | | |
|---|---|---|---|---|---|---|---|
| Complete genome (Accession number NC_008277) | hypothetical protein BAPKO_0135 | 1298 14 | 1307 58 | - | 314 | 111114956 | 4227376 |
| Complete genome (Accession number NC_008277) | hypothetical protein BAPKO_0136 | 1309 43 | 1320 70 | + | 375 | 111114957 | 4227377 |
| Complete genome (Accession number NC_008277) | histidyl-tRNA synthetase | 1321 15 | 1334 85 | - | 456 | 111114958 | 4227378 |
| Complete genome (Accession number NC_008277) | penicillin-binding protein | 1336 18 | 1355 04 | + | 628 | 111114959 | 4227379 |
| Complete genome (Accession number NC_008277) | long-chain-fatty-acid CoA ligase | 1355 05 | 1373 97 | - | 630 | 111114960 | 4227380 |
| Complete genome (Accession number NC_008277) | hypothetical protein BAPKO_0140 | 1377 47 | 1378 75 | - | 42 | 111114961 | 4227381 |
| Complete genome (Accession number NC_008277) | hypothetical protein BAPKO_0141 | 1378 75 | 1382 61 | - | 128 | 111114962 | 4227382 |
| Complete genome (Accession number NC_008277) | acriflavine resistance protein | 1381 85 | 1413 97 | - | 1070 | 111114963 | 4227383 |
| Complete genome (Accession number NC_008277) | membrane fusion protein | 1414 16 | 1423 69 | - | 317 | 111114964 | 4227384 |
| Complete genome (Accession number NC_008277) | hypothetical protein BAPKO_0144 | 1423 81 | 1436 16 | - | 411 | 111114965 | 4227426 |
| Complete genome (Accession number NC_008277) | hypothetical protein BAPKO_0145 | 1437 45 | 1440 35 | + | 96 | 111114966 | 4227427 |

| | | | | | | | |
|---|---|---|---|---|---|---|---|
| Complete genome (Accession number NC_008277) | glycine betaine, L-proline ABC transporter, binding protein | 1440 24 | 1449 08 | - | 294 | 111114967 | 4227428 |
| Complete genome (Accession number NC_008277) | glycine betaine, L-proline ABC transporter, permease protein | 1449 23 | 1458 22 | - | 299 | 111114968 | 4227429 |
| Complete genome (Accession number NC_008277) | glycine betaine, L-proline ABC transporter, ATP- binding protein | 1458 29 | 1469 47 | - | 372 | 111114969 | 4227430 |
| Complete genome (Accession number NC_008277) | flagellin | 1471 00 | 1481 10 | - | 336 | 111114970 | 4227431 |
| Complete genome (Accession number NC_008277) | flagellar hook- associated protein FliD | 1482 33 | 1502 30 | - | 665 | 111114971 | 4227432 |
| Complete genome (Accession number NC_008277) | N-acetylglucosam ine-6-phosphate deacetylase | 1504 13 | 1516 18 | + | 401 | 111114972 | 4227433 |
| Complete genome (Accession number NC_008277) | glucosamine-6-phosphate deaminase | 1516 45 | 1524 51 | + | 268 | 111114973 | 4227434 |
| Complete genome (Accession number NC_008277) | superoxide dismutase | 1525 14 | 1531 25 | - | 203 | 111114974 | 4227435 |
| Complete genome (Accession number NC_008277) | preprotein translocase subunit SecA | 1531 40 | 1558 39 | - | 899 | 111114975 | 4227436 |
| Complete genome (Accession number NC_008277) | lipoprotein, putative | 1559 09 | 1570 45 | + | 378 | 111114976 | 4227437 |
| Complete genome (Accession number NC_008277) | hypothetical protein BAPKO_0157 | 1570 74 | 1575 02 | + | 142 | 111114977 | 4227438 |

| Complete genome (Accession number NC_008277) | hypothetical protein BAPKO_0158 | 1575 06 | 1579 22 | + | 138 | 111114978 | 4227439 |
|---|---|---|---|---|---|---|---|
| Complete genome (Accession number NC_008277) | antigen, S2, putative | 1580 71 | 1588 05 | + | 244 | 111114979 | 4227440 |
| Complete genome (Accession number NC_008277) | antigen S2-related protein | 1588 32 | 1595 06 | + | 224 | 111114980 | 4227441 |
| Complete genome (Accession number NC_008277) | alanine racemase | 1595 71 | 1606 71 | + | 366 | 111114981 | 4227442 |
| Complete genome (Accession number NC_008277) | hypothetical protein BAPKO_0162 | 1606 63 | 1622 64 | - | 533 | 111114982 | 4227443 |
| Complete genome (Accession number NC_008277) | hypothetical protein BAPKO_0163 | 1623 40 | 1625 07 | + | 55 | 111114983 | 4227444 |
| Complete genome (Accession number NC_008277) | hypothetical protein BAPKO_0164 | 1625 35 | 1642 83 | - | 582 | 111114984 | 4227445 |
| Complete genome (Accession number NC_008277) | Na+/Ca+ exchange protein, putative | 1643 63 | 1653 49 | - | 328 | 111114985 | 4227092 |
| Complete genome (Accession number NC_008277) | hypothetical protein BAPKO_0166 | 1653 77 | 1672 15 | - | 612 | 111114986 | 4227093 |
| Complete genome (Accession number NC_008277) | 4-alpha-glucanotransferase | 1673 41 | 1688 61 | - | 506 | 111114987 | 4227094 |
| Complete genome (Accession number NC_008277) | outer membrane protein | 1690 02 | 1701 71 | + | 389 | 111114988 | 4227095 |

| Complete genome (Accession number NC_008277) | hypothetical protein BAPKO_0169 | 1701 60 | 1702 37 | - | 25 | 111114989 | 4227096 |
|---|---|---|---|---|---|---|---|
| Complete genome (Accession number NC_008277) | dnaK suppressor, putative | 1701 82 | 1705 59 | - | 125 | 111114990 | 4227097 |
| Complete genome (Accession number NC_008277) | translation initiation factor IF-1 | 1707 25 | 1709 46 | + | 73 | 111114991 | 4227098 |
| Complete genome (Accession number NC_008277) | hypothetical protein BAPKO_0172 | 1709 94 | 1730 00 | - | 668 | 111114992 | 4227099 |
| Complete genome (Accession number NC_008277) | hypothetical protein BAPKO_0173 | 1729 94 | 1735 81 | - | 195 | 111114993 | 4227100 |
| Complete genome (Accession number NC_008277) | hypothetical protein BAPKO_0174 | 1735 63 | 1746 03 | - | 346 | 111114994 | 4227101 |
| Complete genome (Accession number NC_008277) | hypothetical protein BAPKO_0175 | 1745 46 | 1755 47 | - | 333 | 111114995 | 4227102 |
| Complete genome (Accession number NC_008277) | hypothetical protein BAPKO_0176 | 1755 34 | 1764 33 | - | 299 | 111114996 | 4227103 |
| Complete genome (Accession number NC_008277) | hypothetical protein BAPKO_0177 | 1764 37 | 1773 12 | - | 291 | 111114997 | 4227104 |
| Complete genome (Accession number NC_008277) | MoxR-related protein | 1773 26 | 1783 18 | - | 330 | 111114998 | 4227105 |
| Complete genome (Accession number NC_008277) | glucose inhibited division protein B | 1783 75 | 1790 01 | - | 208 | 111114999 | 4227106 |

| | | | | | | | |
|---|---|---|---|---|---|---|---|
| Complete genome (Accession number NC_008277) | tRNA uridine 5-carboxymethyla minomethyl modification enzyme GidA | 1789 98 | 1808 63 | - | 621 | 111115000 | 4227617 |
| Complete genome (Accession number NC_008277) | tRNA modification GTPase TrmE | 1808 66 | 1822 60 | - | 464 | 111115001 | 4227959 |
| Complete genome (Accession number NC_008277) | flagellar protein, putative | 1823 43 | 1828 37 | + | 164 | 111115002 | 4227960 |
| Complete genome (Accession number NC_008277) | flagellar hook-associated protein FlgK | 1828 49 | 1847 32 | + | 627 | 111115003 | 4227961 |
| Complete genome (Accession number NC_008277) | flagellar hook-associated protein FlgL | 1847 35 | 1860 09 | + | 424 | 111115004 | 4227783 |
| Complete genome (Accession number NC_008277) | flagellar assembly protein FliW | 1860 11 | 1864 09 | + | 132 | 111115005 | 4227784 |
| Complete genome (Accession number NC_008277) | carbon storage regulator | 1864 12 | 1866 57 | + | 81 | 111115006 | 4227785 |
| Complete genome (Accession number NC_008277) | hypothetical protein BAPKO_0187 | 1866 41 | 1872 94 | - | 217 | 111115007 | 4227786 |
| Complete genome (Accession number NC_008277) | hypothetical protein BAPKO_0188 | 1872 87 | 1877 00 | - | 137 | 111115008 | 4227787 |
| Complete genome (Accession number NC_008277) | hypothetical protein BAPKO_0189 | 1876 97 | 1879 57 | - | 86 | 111115009 | 4227788 |
| Complete genome (Accession number NC_008277) | hypothetical protein BAPKO_0190 | 1881 64 | 1882 95 | + | 43 | 111115010 | 4227789 |

| | | | | | | | |
|---|---|---|---|---|---|---|---|
| Complete genome (Accession number NC_008277) | 50S ribosomal protein L20 | 1881 72 | 1885 19 | - | 115 | 111115011 | 4227790 |
| Complete genome (Accession number NC_008277) | 50S ribosomal protein L35 | 1885 40 | 1887 40 | - | 66 | 111115012 | 4227791 |
| Complete genome (Accession number NC_008277) | translation initiation factor IF-3 | 1887 63 | 1893 23 | - | 186 | 111115013 | 4227792 |
| Complete genome (Accession number NC_008277) | hypothetical protein BAPKO_0194 | 1895 17 | 1901 70 | + | 217 | 111115014 | 4227793 |
| Complete genome (Accession number NC_008277) | lipoprotein, putative | 1901 57 | 1909 12 | + | 251 | 111115015 | 4227794 |
| Complete genome (Accession number NC_008277) | hypothetical protein BAPKO_0196 | 1909 68 | 1917 74 | - | 268 | 111115016 | 4227795 |
| Complete genome (Accession number NC_008277) | hypothetical protein BAPKO_0197 | 1917 74 | 1929 13 | - | 379 | 111115017 | 4227796 |
| Complete genome (Accession number NC_008277) | peptide chain release factor 1 | 1932 51 | 1943 24 | + | 357 | 111115018 | 4227797 |
| Complete genome (Accession number NC_008277) | HemK family methylase, putative | 1943 27 | 1951 48 | + | 273 | 111115019 | 4227798 |
| Complete genome (Accession number NC_008277) | guanosine-3,5-bis(diphosphate) 3-pyrophosphohydrolase | 1951 45 | 1971 48 | + | 667 | 111115020 | 4227799 |
| Complete genome (Accession number NC_008277) | hypothetical protein BAPKO_0201 | 1971 38 | 1993 99 | + | 753 | 111115021 | 4227800 |

| | | | | | | | |
|---|---|---|---|---|---|---|---|
| Complete genome (Accession number NC_008277) | hypothetical protein BAPKO_0202 | 1993 44 | 1994 54 | - | 36 | 111115022 | 4227801 |
| Complete genome (Accession number NC_008277) | D-alanine--D-alanine ligase | 1993 96 | 2004 81 | - | 361 | 111115023 | 4227802 |
| Complete genome (Accession number NC_008277) | UDP-N-acetylmuramoyl alanyl-D-glutamate--2, 6-diaminopimelat e ligase | 2004 83 | 2020 00 | - | 505 | 111115024 | 4227686 |
| Complete genome (Accession number NC_008277) | hypothetical protein BAPKO_0206 | 2022 73 | 2026 47 | + | 124 | 111115025 | 4227688 |
| Complete genome (Accession number NC_008277) | hemolysin, putative | 2027 43 | 2035 10 | + | 255 | 111115026 | 4227689 |
| Complete genome (Accession number NC_008277) | Lambda CII stability-governing protein | 2035 63 | 2044 98 | + | 311 | 111115027 | 4227690 |
| Complete genome (Accession number NC_008277) | Lambda CII stability-governing protein | 2044 99 | 2054 70 | + | 323 | 111115028 | 4227691 |
| Complete genome (Accession number NC_008277) | hypothetical protein BAPKO_0212 | 2059 30 | 2074 80 | + | 516 | 111115029 | 4227694 |
| Complete genome (Accession number NC_008277) | hypothetical protein BAPKO_0213 | 2074 85 | 2080 54 | - | 189 | 111115030 | 4227695 |
| Complete genome (Accession number NC_008277) | UTP--glucose-1-phosphate uridylyltransfer ase | 2081 07 | 2089 43 | + | 278 | 111115031 | 4227696 |
| Complete genome (Accession number NC_008277) | hypothetical protein BAPKO_0215 | 2089 30 | 2106 69 | + | 579 | 111115032 | 4227697 |

| | | | | | | | |
|---|---|---|---|---|---|---|---|
| Complete genome (Accession number NC_008277) | hypothetical protein BAPKO_0216 | 2107 06 | 2114 73 | + | 255 | 111115033 | 4227698 |
| Complete genome (Accession number NC_008277) | surface-located membrane protein 1 | 2114 66 | 2145 07 | + | 1013 | 111115034 | 4227699 |
| Complete genome (Accession number NC_008277) | DNA mismatch repair protein | 2145 14 | 2163 49 | + | 611 | 111115035 | 4227700 |
| Complete genome (Accession number NC_008277) | hypothetical protein BAPKO_0219 | 2163 68 | 2174 02 | + | 344 | 111115036 | 4227701 |
| Complete genome (Accession number NC_008277) | lipoprotein, putative | 2175 15 | 2181 68 | - | 217 | 111115037 | 4227831 |
| Complete genome (Accession number NC_008277) | translation elongation factor P | 2181 46 | 2187 24 | - | 192 | 111115038 | 4227832 |
| Complete genome (Accession number NC_008277) | phosphate ABC transporter, periplasmic phosphate-binding protein | 2188 90 | 2197 29 | + | 279 | 111115039 | 4227833 |
| Complete genome (Accession number NC_008277) | phosphate ABC transporter, permease protein | 2198 19 | 2207 27 | + | 302 | 111115040 | 4227834 |
| Complete genome (Accession number NC_008277) | phosphate ABC transporter, permease protein | 2207 24 | 2222 68 | + | 514 | 111115041 | 4227835 |
| Complete genome (Accession number NC_008277) | phosphate ABC transporter, ATP-binding protein | 2222 69 | 2230 51 | + | 260 | 111115042 | 4227836 |
| Complete genome (Accession number NC_008277) | gufA protein | 2230 54 | 2238 75 | - | 273 | 111115043 | 4227837 |
| Complete genome | alanyl-tRNA synthetase | 2239 16 | 2257 00 | - | 594 | 111115044 | 4227838 |

| | | | | | | | |
|---|---|---|---|---|---|---|---|
| (Accession number NC_008277) | | | | | | | |
| Complete genome (Accession number NC_008277) | flagellar motor switch protein | 2257 00 | 2269 17 | - | 405 | 111115045 | 4227839 |
| Complete genome (Accession number NC_008277) | 6-phosphogluconolactonase | 2269 04 | 2276 11 | - | 235 | 111115046 | 4227840 |
| Complete genome (Accession number NC_008277) | hypothetical protein BAPKO_0231 | 2277 16 | 2278 53 | - | 45 | 111115047 | 4227841 |
| Complete genome (Accession number NC_008277) | hypothetical protein BAPKO_0232 | 2278 74 | 2280 41 | - | 55 | 111115048 | 4227842 |
| Complete genome (Accession number NC_008277) | tRNA-dihydrouridine synthase A | 2281 18 | 2291 19 | + | 333 | 111115049 | 4227843 |
| Complete genome (Accession number NC_008277) | seryl-tRNA synthetase | 2291 09 | 2303 86 | - | 425 | 111115050 | 4227844 |
| Complete genome (Accession number NC_008277) | hypothetical protein BAPKO_0235 | 2305 26 | 2312 42 | + | 238 | 111115051 | 4227845 |
| Complete genome (Accession number NC_008277) | hypothetical protein BAPKO_0236 | 2312 43 | 2341 61 | + | 972 | 111115052 | 4227846 |
| Complete genome (Accession number NC_008277) | hypothetical protein BAPKO_0237 | 2341 58 | 2342 32 | - | 24 | 111115053 | 4227847 |
| Complete genome (Accession number NC_008277) | 50S ribosomal protein L31 type B | 2341 98 | 2344 43 | - | 81 | 111115054 | 4227702 |
| Complete genome (Accession number | transcription termination factor Rho | 2345 04 | 2360 51 | - | 515 | 111115055 | 4227703 |

| | | | | | | | |
|---|---|---|---|---|---|---|---|
| NC_008277) | | | | | | | |
| Complete genome (Accession number NC_008277) | hypothetical protein BAPKO_0240 | 2361 31 | 2365 38 | - | 135 | 111115056 | 4227704 |
| Complete genome (Accession number NC_008277) | hbbU protein | 2365 39 | 2368 65 | - | 108 | 111115057 | 4227705 |
| Complete genome (Accession number NC_008277) | 30S ribosomal protein S20 | 2368 78 | 2371 35 | - | 85 | 111115058 | 4227706 |
| Complete genome (Accession number NC_008277) | hypothetical protein BAPKO_0243 | 2372 06 | 2380 33 | - | 275 | 111115059 | 4227707 |
| Complete genome (Accession number NC_008277) | translation-associated GTPase | 2380 43 | 2391 49 | - | 368 | 111115060 | 4227708 |
| Complete genome (Accession number NC_008277) | hypothetical protein BAPKO_0245 | 2391 85 | 2411 91 | - | 668 | 111115061 | 4227709 |
| Complete genome (Accession number NC_008277) | apolipoprotein N-acyltransferase | 2413 52 | 2429 20 | + | 522 | 111115062 | 4227710 |
| Complete genome (Accession number NC_008277) | hypothetical protein BAPKO_0247 | 2428 57 | 2436 30 | - | 257 | 111115063 | 4227711 |
| Complete genome (Accession number NC_008277) | deoxyguanosine/deoxyadenosine kinase(I) subunit 2 | 2436 95 | 2442 97 | - | 200 | 111115064 | 4227712 |
| Complete genome (Accession number NC_008277) | glycerol uptake facilitator | 2447 05 | 2454 69 | + | 254 | 111115065 | 4227713 |
| Complete genome (Accession number | glycerol kinase | 2455 07 | 2470 12 | + | 501 | 111115066 | 4227714 |

| NC_008277) | | | | | | | |
|---|---|---|---|---|---|---|---|
| Complete genome (Accession number NC_008277) | hypothetical protein BAPKO_0251 | 2470 67 | 2473 06 | + | 79 | 111115067 | 4227715 |
| Complete genome (Accession number NC_008277) | glycerol-3-phosphate dehydrogenase , anaerobic | 2474 40 | 2490 08 | + | 522 | 111115068 | 4227716 |
| Complete genome (Accession number NC_008277) | hypothetical protein BAPKO_0253 | 2490 95 | 2495 89 | - | 164 | 111115069 | 4227717 |
| Complete genome (Accession number NC_008277) | hypothetical protein BAPKO_0254 | 2495 76 | 2501 30 | - | 184 | 111115070 | 4227718 |
| Complete genome (Accession number NC_008277) | hypothetical protein BAPKO_0255 | 2501 31 | 2511 56 | - | 341 | 111115071 | 4227719 |
| Complete genome (Accession number NC_008277) | hypothetical protein BAPKO_0256 | 2513 54 | 2519 59 | + | 201 | 111115072 | 4227720 |
| Complete genome (Accession number NC_008277) | oligoendopepti dase F | 2520 79 | 2538 51 | + | 590 | 111115073 | 4227721 |
| Complete genome (Accession number NC_008277) | phosphatidyltra nsferase | 2538 65 | 2545 69 | + | 234 | 111115074 | 4227466 |
| Complete genome (Accession number NC_008277) | dedA protein | 2545 66 | 2551 80 | + | 204 | 111115075 | 4227467 |
| Complete genome (Accession number NC_008277) | leucyl-tRNA synthetase | 2553 76 | 2578 98 | + | 840 | 111115076 | 4227469 |
| Complete genome (Accession number NC_008277) | hypothetical protein BAPKO_0262 | 2579 13 | 2602 16 | + | 767 | 111115077 | 4227470 |

| | | | | | | | |
|---|---|---|---|---|---|---|---|
| Complete genome (Accession number NC_008277) | ATP-dependent protease LA | 2602 05 | 2626 25 | - | 806 | 111115078 | 4227471 |
| Complete genome (Accession number NC_008277) | single-stranded-DNA-specific exonuclease | 2628 98 | 2650 18 | + | 706 | 111115079 | 4227472 |
| Complete genome (Accession number NC_008277) | hypothetical protein BAPKO_0265 | 2650 11 | 2659 55 | + | 314 | 111115080 | 4227473 |
| Complete genome (Accession number NC_008277) | 30S ribosomal protein S21 | 2659 70 | 2661 79 | + | 69 | 111115081 | 4227474 |
| Complete genome (Accession number NC_008277) | cell division protein, putative | 2662 18 | 2685 69 | - | 783 | 111115082 | 4227475 |
| Complete genome (Accession number NC_008277) | undecaprenyl pyrophosphate phosphatase | 2685 73 | 2693 67 | - | 264 | 111115083 | 4227476 |
| Complete genome (Accession number NC_008277) | hypothetical protein BAPKO_0269 | 2693 82 | 2715 50 | - | 722 | 111115084 | 4227477 |
| Complete genome (Accession number NC_008277) | hypothetical protein BAPKO_0270 | 2715 28 | 2725 38 | - | 336 | 111115085 | 4227478 |
| Complete genome (Accession number NC_008277) | hypothetical protein BAPKO_0271 | 2727 41 | 2741 23 | + | 460 | 111115086 | 4227479 |
| Complete genome (Accession number NC_008277) | hypothetical protein BAPKO_0272 | 2741 30 | 2753 83 | - | 417 | 111115087 | 4227480 |
| Complete genome (Accession number NC_008277) | signal peptidase I | 2755 59 | 2759 51 | + | 130 | 111115088 | 4227481 |
| Complete genome | heat shock protein 70 | 2759 75 | 2774 44 | - | 489 | 111115089 | 4227482 |

| | | | | | | | |
|---|---|---|---|---|---|---|---|
| (Accession number NC_008277) | | | | | | | |
| Complete genome (Accession number NC_008277) | hypothetical protein BAPKO_0275 | 2774 22 | 2779 43 | - | 173 | 111115090 | 4227483 |
| Complete genome (Accession number NC_008277) | hypothetical protein BAPKO_0276 | 2779 63 | 2782 65 | - | 100 | 111115091 | 4227260 |
| Complete genome (Accession number NC_008277) | hypothetical protein BAPKO_0277 | 2782 92 | 2801 96 | - | 634 | 111115092 | 4227261 |
| Complete genome (Accession number NC_008277) | hypothetical protein BAPKO_0278 | 2801 99 | 2806 78 | - | 159 | 111115093 | 4227262 |
| Complete genome (Accession number NC_008277) | minD-related ATP-binding protein | 2806 88 | 2815 75 | - | 295 | 111115094 | 4227263 |
| Complete genome (Accession number NC_008277) | flagellar biosynthesis regulator FlhF | 2815 87 | 2827 53 | - | 388 | 111115095 | 4227264 |
| Complete genome (Accession number NC_008277) | flagellar biosynthesis protein A | 2827 58 | 2848 48 | - | 696 | 111115096 | 4227265 |
| Complete genome (Accession number NC_008277) | flagellar biosynthesis protein FlhB | 2848 60 | 2859 78 | - | 372 | 111115097 | 4227618 |
| Complete genome (Accession number NC_008277) | flagellar biosynthesis protein | 2859 78 | 2867 66 | - | 262 | 111115098 | 4227619 |
| Complete genome (Accession number NC_008277) | flagellar biosynthesis protein | 2868 02 | 2870 65 | - | 87 | 111115099 | 4227620 |
| Complete genome (Accession number | flagellar biosynthesis protein FliP | 2870 73 | 2878 37 | - | 254 | 111115100 | 4227621 |

| | | | | | | | |
|---|---|---|---|---|---|---|---|
| NC_008277) | | | | | | | |
| Complete genome (Accession number NC_008277) | flagellar biosynthesis protein | 2878 48 | 2884 74 | - | 208 | 111115101 | 4227622 |
| Complete genome (Accession number NC_008277) | flagellar motor switch protein | 2884 67 | 2888 08 | - | 113 | 111115102 | 4227623 |
| Complete genome (Accession number NC_008277) | flagellar motor switch protein FliM | 2888 51 | 2899 09 | - | 352 | 111115103 | 4227624 |
| Complete genome (Accession number NC_008277) | flagellar basal body- associated protein FliL | 2899 30 | 2904 66 | - | 178 | 111115104 | 4227625 |
| Complete genome (Accession number NC_008277) | flagellar motor protein MotB | 2905 14 | 2912 96 | - | 260 | 111115105 | 4227626 |
| Complete genome (Accession number NC_008277) | flagellar motor rotation protein A | 2912 96 | 2920 78 | - | 260 | 111115106 | 4227627 |
| Complete genome (Accession number NC_008277) | flagellar protein | 2920 75 | 2922 99 | - | 74 | 111115107 | 4227628 |
| Complete genome (Accession number NC_008277) | flagellar hook protein FlgE | 2923 20 | 2936 48 | - | 442 | 111115108 | 4227629 |
| Complete genome (Accession number NC_008277) | flagellar basal body rod modification protein | 2936 53 | 2940 96 | - | 147 | 111115109 | 4227630 |
| Complete genome (Accession number NC_008277) | flagellar protein | 2941 10 | 2952 94 | - | 394 | 111115110 | 4227631 |
| Complete genome (Accession number | flagellar protein | 2953 02 | 2959 19 | - | 205 | 111115111 | 4227213 |

| NC_008277) | | | | | | | |
|---|---|---|---|---|---|---|---|
| Complete genome (Accession number NC_008277) | flagellar protein | 2959 12 | 2963 43 | - | 143 | 111115112 | 4227214 |
| Complete genome (Accession number NC_008277) | flagellum-specific ATP synthase | 2963 40 | 2976 50 | - | 436 | 111115113 | 4227215 |
| Complete genome (Accession number NC_008277) | flagellar assembly protein H | 2976 69 | 2985 89 | - | 306 | 111115114 | 4227216 |
| Complete genome (Accession number NC_008277) | flagellar motor switch protein G | 2986 04 | 2996 38 | - | 344 | 111115115 | 4227217 |
| Complete genome (Accession number NC_008277) | flagellar MS-ring protein | 2996 54 | 3013 63 | - | 569 | 111115116 | 4227218 |
| Complete genome (Accession number NC_008277) | flagellar hook-basal body protein FliE | 3013 78 | 3017 13 | - | 111 | 111115117 | 4227219 |
| Complete genome (Accession number NC_008277) | flagellar basal body rod protein FlgC | 3017 25 | 3021 83 | - | 152 | 111115118 | 4227220 |
| Complete genome (Accession number NC_008277) | flagellar basal body rod protein FlgB | 3022 07 | 3026 14 | - | 135 | 111115119 | 4227221 |
| Complete genome (Accession number NC_008277) | ATP-dependent protease ATP-binding subunit | 3026 48 | 3039 94 | - | 448 | 111115120 | 4227222 |
| Complete genome (Accession number NC_008277) | ATP-dependent protease peptidase subunit | 3039 87 | 3045 35 | - | 182 | 111115121 | 4227223 |
| Complete genome (Accession number NC_008277) | smf protein | 3045 44 | 3054 91 | - | 315 | 111115122 | 4227224 |

| | | | | | | | |
|---|---|---|---|---|---|---|---|
| Complete genome (Accession number NC_008277) | hypothetical protein BAPKO_0308 | 3054 98 | 3061 90 | - | 230 | 111115123 | 4227225 |
| Complete genome (Accession number NC_008277) | cell division protein FtsZ | 3061 91 | 3073 90 | - | 399 | 111115124 | 4227226 |
| Complete genome (Accession number NC_008277) | cell division protein | 3074 12 | 3086 53 | - | 413 | 111115125 | 4227227 |
| Complete genome (Accession number NC_008277) | cell division protein | 3086 53 | 3093 96 | - | 247 | 111115126 | 4227228 |
| Complete genome (Accession number NC_008277) | cell division protein | 3094 32 | 3099 89 | - | 185 | 111115127 | 4227229 |
| Complete genome (Accession number NC_008277) | hypothetical protein BAPKO_0313 | 3100 62 | 3104 90 | - | 142 | 111115128 | 4227230 |
| Complete genome (Accession number NC_008277) | phospho-N-acetylmuramoyl -pentapeptide-transferase | 3105 33 | 3115 88 | - | 351 | 111115129 | 4227231 |
| Complete genome (Accession number NC_008277) | UDP-N-acetylmuramoyl alanyl-D-glutamyl-2, 6-diaminopimelat e--D-alanyl-D-alanine | 3116 06 | 3129 97 | - | 463 | 111115130 | 4227405 |
| Complete genome (Accession number NC_008277) | hypothetical protein BAPKO_0316 | 3130 20 | 3133 01 | - | 93 | 111115131 | 4227406 |
| Complete genome (Accession number NC_008277) | hypothetical protein BAPKO_0317 | 3132 98 | 3141 88 | - | 296 | 111115132 | 4227407 |
| Complete genome (Accession number NC_008277) | hypothetical protein BAPKO_0318 | 3141 81 | 3150 17 | - | 278 | 111115133 | 4227408 |

| | | | | | | | |
|---|---|---|---|---|---|---|---|
| Complete genome (Accession number NC_008277) | hypothetical protein BAPKO_0319 | 3150 14 | 3160 90 | - | 358 | 111115134 | 4227409 |
| Complete genome (Accession number NC_008277) | hypothetical protein BAPKO_0320 | 3161 20 | 3168 78 | - | 252 | 111115135 | 4227410 |
| Complete genome (Accession number NC_008277) | hypothetical protein BAPKO_0321 | 3171 94 | 3180 33 | - | 279 | 111115136 | 4227411 |
| Complete genome (Accession number NC_008277) | purine-binding chemotaxis protein | 3180 23 | 3185 53 | - | 176 | 111115137 | 4227412 |
| Complete genome (Accession number NC_008277) | cell division protein | 3185 99 | 3191 68 | - | 189 | 111115138 | 4227413 |
| Complete genome (Accession number NC_008277) | octaprenyl-diphosphate synthase | 3192 28 | 3202 71 | + | 347 | 111115139 | 4227414 |
| Complete genome (Accession number NC_008277) | hypothetical protein BAPKO_0325 | 3202 71 | 3209 54 | + | 227 | 111115140 | 4227415 |
| Complete genome (Accession number NC_008277) | hypothetical protein BAPKO_0326 | 3209 56 | 3217 62 | - | 268 | 111115141 | 4227416 |
| Complete genome (Accession number NC_008277) | hypothetical protein BAPKO_0327 | 3217 63 | 3226 95 | - | 310 | 111115142 | 4227417 |
| Complete genome (Accession number NC_008277) | methylgalactoside ABC transporter, ATP-binding protein | 3226 92 | 3241 52 | - | 486 | 111115143 | 4227418 |
| Complete genome (Accession number NC_008277) | exported protein | 3241 52 | 3251 89 | - | 345 | 111115144 | 4227419 |

| Complete genome (Accession number NC_008277) | hypothetical protein BAPKO_0330 | 3253 23 | 3255 74 | - | 83 | 111115145 | 4227420 |
|---|---|---|---|---|---|---|---|
| Complete genome (Accession number NC_008277) | hypothetical protein BAPKO_0331 | 3256 09 | 3266 67 | - | 352 | 111115146 | 4227421 |
| Complete genome (Accession number NC_008277) | hypothetical protein BAPKO_0332 | 3269 41 | 3280 80 | + | 379 | 111115147 | 4227422 |
| Complete genome (Accession number NC_008277) | hypothetical protein BAPKO_0333 | 3281 76 | 3285 41 | + | 121 | 111115148 | 4227423 |
| Complete genome (Accession number NC_008277) | hypothetical protein BAPKO_0334 | 3285 48 | 3296 57 | - | 369 | 111115149 | 4227647 |
| Complete genome (Accession number NC_008277) | hypothetical protein BAPKO_0335 | 3297 08 | 3325 12 | - | 934 | 111115150 | 4227667 |
| Complete genome (Accession number NC_008277) | glycerol-3-phosphate O-acyltransferase, putative | 3325 36 | 3334 29 | - | 297 | 111115151 | 4227668 |
| Complete genome (Accession number NC_008277) | oligopeptide ABC transporter, periplasmic oligopeptide-binding protein | 3338 29 | 3354 00 | + | 523 | 111115152 | 4227669 |
| Complete genome (Accession number NC_008277) | oligopeptide ABC transporter, periplasmic oligopeptide-binding protein | 3354 99 | 3370 82 | + | 527 | 111115153 | 4227670 |
| Complete genome (Accession number NC_008277) | oligopeptide ABC transporter, periplasmic oligopeptide-binding protein | 3372 27 | 3388 52 | + | 541 | 111115154 | 4227671 |
| Complete genome (Accession number | oligopeptide ABC transporter, permease | 3391 48 | 3400 68 | + | 306 | 111115155 | 4227672 |

| | | | | | | | |
|---|---|---|---|---|---|---|---|
| NC_008277) | protein | | | | | | |
| Complete genome (Accession number NC_008277) | oligopeptide ABC transporter, permease protein | 3400 81 | 3411 30 | + | 349 | 111115156 | 4227673 |
| Complete genome (Accession number NC_008277) | hypothetical protein BAPKO_0342 | 3411 02 | 3411 85 | + | 27 | 111115157 | 4227674 |
| Complete genome (Accession number NC_008277) | oligopeptide ABC transporter, ATP-binding protein | 3411 42 | 3420 17 | + | 291 | 111115158 | 4227675 |
| Complete genome (Accession number NC_008277) | oligopeptide ABC transporter, ATP-binding protein | 3420 18 | 3429 89 | + | 323 | 111115159 | 4227676 |
| Complete genome (Accession number NC_008277) | hypothetical protein BAPKO_0345 | 3430 03 | 3437 34 | - | 243 | 111115160 | 4227677 |
| Complete genome (Accession number NC_008277) | enolase | 3438 68 | 3451 69 | + | 433 | 111115161 | 4227678 |
| Complete genome (Accession number NC_008277) | 30S ribosomal protein S9 | 3452 34 | 3456 44 | - | 136 | 111115162 | 4227679 |
| Complete genome (Accession number NC_008277) | 50S ribosomal protein L13 | 3456 46 | 3461 04 | - | 152 | 111115163 | 4227680 |
| Complete genome (Accession number NC_008277) | hypothetical protein BAPKO_0349 | 3461 57 | 3469 66 | - | 269 | 111115164 | 4227681 |
| Complete genome (Accession number NC_008277) | aspartyl/glutam yl-tRNA amidotransfera se subunit B | 3469 59 | 3484 16 | - | 485 | 111115165 | 4227682 |
| Complete genome (Accession | Glu-tRNA(Gln) amidotransfera se, subunit A | 3484 06 | 3498 51 | - | 481 | 111115166 | 4227683 |

| number NC_008277) | | | | | | | |
|---|---|---|---|---|---|---|---|
| Complete genome (Accession number NC_008277) | aspartyl/glutamyl-tRNA amidotransferase subunit C | 3498 62 | 3501 37 | - | 91 | 111115167 | 4227684 |
| Complete genome (Accession number NC_008277) | DNA helicase | 3501 47 | 3522 43 | - | 698 | 111115168 | 4227685 |
| Complete genome (Accession number NC_008277) | hypothetical protein BAPKO_0354 | 3522 46 | 3534 45 | - | 399 | 111115169 | 4227446 |
| Complete genome (Accession number NC_008277) | hypothetical protein BAPKO_0355 | 3534 56 | 3541 06 | - | 216 | 111115170 | 4227447 |
| Complete genome (Accession number NC_008277) | fibronectin/fibrinogen-binding protein, putative | 3542 98 | 3557 16 | + | 472 | 111115171 | 4227448 |
| Complete genome (Accession number NC_008277) | pyruvate kinase | 3558 12 | 3572 45 | + | 477 | 111115172 | 4227449 |
| Complete genome (Accession number NC_008277) | hypothetical protein BAPKO_0358 | 3573 14 | 3580 03 | + | 229 | 111115173 | 4227450 |
| Complete genome (Accession number NC_008277) | 50S ribosomal protein L28 | 3580 22 | 3583 00 | - | 92 | 111115174 | 4227451 |
| Complete genome (Accession number NC_008277) | hypothetical protein BAPKO_0360 | 3583 22 | 3598 84 | - | 520 | 111115175 | 4227452 |
| Complete genome (Accession number NC_008277) | hypothetical protein BAPKO_0361 | 3599 94 | 3611 27 | + | 377 | 111115176 | 4227453 |
| Complete genome (Accession | hypothetical protein BAPKO_0362 | 3611 24 | 3629 23 | - | 599 | 111115177 | 4227454 |

| number NC_008277) | | | | | | | |
|---|---|---|---|---|---|---|---|
| Complete genome (Accession number NC_008277) | hypothetical protein BAPKO_0363 | 3629 36 | 3638 86 | - | 316 | 111115178 | 4227455 |
| Complete genome (Accession number NC_008277) | transcription factor, putative | 3639 20 | 3644 08 | - | 162 | 111115179 | 4227456 |
| Complete genome (Accession number NC_008277) | hypothetical protein BAPKO_0365 | 3644 48 | 3650 41 | - | 197 | 111115180 | 4227457 |
| Complete genome (Accession number NC_008277) | hypothetical protein BAPKO_0366 | 3650 34 | 3657 65 | - | 243 | 111115181 | 4227458 |
| Complete genome (Accession number NC_008277) | carboxyl-terminal protease | 3657 69 | 3672 02 | - | 477 | 111115182 | 4227459 |
| Complete genome (Accession number NC_008277) | minD-related ATP-binding protein | 3677 02 | 3688 44 | + | 380 | 111115183 | 4227460 |
| Complete genome (Accession number NC_008277) | prolipoprotein diacylglyceryl transferase | 3688 44 | 3698 15 | + | 323 | 111115184 | 4227461 |
| Complete genome (Accession number NC_008277) | hypothetical protein BAPKO_0370 | 3698 50 | 3718 62 | + | 670 | 111115185 | 4227462 |
| Complete genome (Accession number NC_008277) | methylglyoxal synthase | 3719 58 | 3723 41 | + | 127 | 111115186 | 4227463 |
| Complete genome (Accession number NC_008277) | lipoprotein LA7 | 3723 84 | 3729 71 | - | 195 | 111115187 | 4227464 |
| Complete genome (Accession number | putative aminopeptidas e 1 | 3731 08 | 3744 84 | + | 458 | 111115188 | 4227465 |

| | | | | | | | |
|---|---|---|---|---|---|---|---|
| NC_008277) | | | | | | | |
| Complete genome (Accession number NC_008277) | hypothetical protein BAPKO_0375 | 3744 35 | 3747 70 | + | 111 | 111115189 | 4227554 |
| Complete genome (Accession number NC_008277) | hypothetical protein BAPKO_0376 | 3747 54 | 3752 15 | + | 153 | 111115190 | 4227555 |
| Complete genome (Accession number NC_008277) | NAD(P)H-dependent glycerol-3-phosphate dehydrogenase | 3747 92 | 3758 50 | - | 352 | 111115191 | 4227556 |
| Complete genome (Accession number NC_008277) | ATP-dependent Clp protease, subunit A | 3758 68 | 3781 65 | - | 765 | 111115192 | 4227557 |
| Complete genome (Accession number NC_008277) | tyrosyl-tRNA synthetase | 3781 76 | 3793 93 | - | 405 | 111115193 | 4227558 |
| Complete genome (Accession number NC_008277) | glycyl-tRNA synthetase | 3793 90 | 3807 27 | - | 445 | 111115194 | 4227559 |
| Complete genome (Accession number NC_008277) | glutamyl-tRNA synthetase | 3807 45 | 3822 17 | - | 490 | 111115195 | 4227560 |
| Complete genome (Accession number NC_008277) | hypothetical protein BAPKO_0382 | 3822 30 | 3829 97 | - | 255 | 111115196 | 4227561 |
| Complete genome (Accession number NC_008277) | hypothetical protein BAPKO_0383 | 3831 27 | 3842 63 | + | 378 | 111115197 | 4227562 |
| Complete genome (Accession number NC_008277) | 5-methylthioadenosine/S-adenosylhomocysteine nucleosidase, putative | 3842 85 | 3849 98 | + | 237 | 111115198 | 4227563 |
| Complete genome | S-adenosylmethio | 3849 95 | 3861 73 | + | 392 | 111115199 | 4227564 |

| | | | | | | | |
|---|---|---|---|---|---|---|---|
| (Accession number NC_008277) | nine synthetase | | | | | | |
| Complete genome (Accession number NC_008277) | S-ribosylhomocys teinase | 3861 70 | 3866 43 | + | 157 | 111115200 | 4227565 |
| Complete genome (Accession number NC_008277) | hypothetical protein BAPKO_0387 | 3866 54 | 3873 16 | - | 220 | 111115201 | 4227566 |
| Complete genome (Accession number NC_008277) | protein kinase C1 inhibitor | 3873 43 | 3877 62 | - | 139 | 111115202 | 4227567 |
| Complete genome (Accession number NC_008277) | Mg2+ transport protein | 3878 11 | 3891 75 | - | 454 | 111115203 | 4227568 |
| Complete genome (Accession number NC_008277) | hypothetical protein BAPKO_0390 | 3892 29 | 3906 53 | - | 474 | 111115204 | 4227569 |
| Complete genome (Accession number NC_008277) | basic membrane protein B | 3907 67 | 3917 92 | - | 341 | 111115205 | 4227570 |
| Complete genome (Accession number NC_008277) | basic membrane protein A | 3918 79 | 3928 98 | - | 339 | 111115206 | 4227571 |
| Complete genome (Accession number NC_008277) | hypothetical protein BAPKO_0393 | 3929 42 | 3931 00 | - | 52 | 111115207 | 4227572 |
| Complete genome (Accession number NC_008277) | hypothetical protein BAPKO_0394 | 3932 99 | 3933 85 | - | 28 | 111115208 | 4227803 |
| Complete genome (Accession number NC_008277) | hypothetical protein BAPKO_0395 | 3935 63 | 3945 82 | - | 339 | 111115209 | 4227804 |
| Complete genome (Accession number | hypothetical protein BAPKO_0396 | 3946 26 | 3949 04 | - | 92 | 111115210 | 4227805 |

| | | | | | | | |
|---|---|---|---|---|---|---|---|
| NC_008277) | | | | | | | |
| Complete genome (Accession number NC_008277) | hypothetical protein BAPKO_0397 | 3949 80 | 3950 66 | - | 28 | 111115211 | 4227806 |
| Complete genome (Accession number NC_008277) | hypothetical protein BAPKO_0398 | 3952 44 | 3962 63 | - | 339 | 111115212 | 4227807 |
| Complete genome (Accession number NC_008277) | basic membrane protein C | 3963 07 | 3973 68 | - | 353 | 111115213 | 4227808 |
| Complete genome (Accession number NC_008277) | basic membrane protein D | 3976 82 | 3987 07 | - | 341 | 111115214 | 4227809 |
| Complete genome (Accession number NC_008277) | 30S ribosomal protein S7 | 3988 08 | 3992 81 | - | 157 | 111115215 | 4227810 |
| Complete genome (Accession number NC_008277) | 30S ribosomal protein S12 | 3993 06 | 3996 80 | - | 124 | 111115216 | 4227811 |
| Complete genome (Accession number NC_008277) | DNA-directed RNA polymerase subunit beta' | 3997 48 | 4038 81 | - | 1377 | 111115217 | 4227812 |
| Complete genome (Accession number NC_008277) | DNA-directed RNA polymerase subunit beta | 4038 97 | 4073 64 | - | 1155 | 111115218 | 4227813 |
| Complete genome (Accession number NC_008277) | 50S ribosomal protein L7/L12 | 4074 47 | 4078 21 | - | 124 | 111115219 | 4227814 |
| Complete genome (Accession number NC_008277) | 50S ribosomal protein L10 | 4078 92 | 4083 80 | - | 162 | 111115220 | 4227815 |
| Complete genome (Accession number | 50S ribosomal protein L1 | 4083 85 | 4090 65 | - | 226 | 111115221 | 4227816 |

| NC_008277) | | | | | | | |
|---|---|---|---|---|---|---|---|
| Complete genome (Accession number NC_008277) | 50S ribosomal protein L11 | 4090 65 | 4094 96 | - | 143 | 111115222 | 4227817 |
| Complete genome (Accession number NC_008277) | transcription antitermination factor | 4095 50 | 4101 04 | - | 184 | 111115223 | 4227818 |
| Complete genome (Accession number NC_008277) | preprotein translocase subunit SecE | 4101 28 | 4102 98 | - | 56 | 111115224 | 4227819 |
| Complete genome (Accession number NC_008277) | 50S ribosomal protein L33 | 4104 06 | 4105 85 | - | 59 | 111115225 | 4227821 |
| Complete genome (Accession number NC_008277) | hypothetical protein BAPKO_0414 | 4107 50 | 4116 07 | - | 285 | 111115226 | 4227610 |
| Complete genome (Accession number NC_008277) | lipoprotein, putative | 4116 21 | 4126 43 | - | 340 | 111115227 | 4227611 |
| Complete genome (Accession number NC_008277) | hypothetical protein BAPKO_0416 | 4129 90 | 4136 01 | + | 203 | 111115228 | 4227612 |
| Complete genome (Accession number NC_008277) | hypothetical protein BAPKO_0417 | 4136 58 | 4153 13 | - | 551 | 111115229 | 4227613 |
| Complete genome (Accession number NC_008277) | glutamate transporter, putative | 4153 14 | 4165 16 | + | 400 | 111115230 | 4227614 |
| Complete genome (Accession number NC_008277) | prolyl-tRNA synthetase | 4165 28 | 4179 94 | - | 488 | 111115231 | 4227615 |
| Complete genome (Accession number | hypothetical protein BAPKO_0420 | 4180 47 | 4187 24 | - | 225 | 111115232 | 4227616 |

| | | | | | | | |
|---|---|---|---|---|---|---|---|
| NC_008277) | | | | | | | |
| Complete genome (Accession number NC_008277) | hypothetical protein BAPKO_0421 | 4188 88 | 4193 04 | + | 138 | 111115233 | 4227739 |
| Complete genome (Accession number NC_008277) | hypothetical protein BAPKO_0422 | 4193 01 | 4199 06 | + | 201 | 111115234 | 4227740 |
| Complete genome (Accession number NC_008277) | hypothetical protein BAPKO_0423 | 4199 17 | 4205 28 | + | 203 | 111115235 | 4227741 |
| Complete genome (Accession number NC_008277) | mannose-6-phosphate isomerase | 4205 81 | 4216 96 | - | 371 | 111115236 | 4227742 |
| Complete genome (Accession number NC_008277) | PTS system, fructose-specific IIABC component | 4216 97 | 4235 74 | - | 625 | 111115237 | 4227743 |
| Complete genome (Accession number NC_008277) | hypothetical protein BAPKO_0426 | 4237 46 | 4243 75 | + | 209 | 111115238 | 4227744 |
| Complete genome (Accession number NC_008277) | endonuclease precursor | 4243 72 | 4252 53 | + | 293 | 111115239 | 4227745 |
| Complete genome (Accession number NC_008277) | hypothetical protein BAPKO_0428 | 4252 50 | 4260 29 | + | 259 | 111115240 | 4227746 |
| Complete genome (Accession number NC_008277) | hypothetical protein BAPKO_0429 | 4260 17 | 4266 37 | - | 206 | 111115241 | 4227747 |
| Complete genome (Accession number NC_008277) | chemotaxis protein methyltransferase | 4268 83 | 4277 40 | + | 285 | 111115242 | 4227748 |
| Complete genome (Accession number | protein-glutamate methylesterase | 4277 73 | 4289 00 | + | 375 | 111115243 | 4227749 |

| | | | | | | | |
|---|---|---|---|---|---|---|---|
| NC_008277) | | | | | | | |
| Complete genome (Accession number NC_008277) | pheromone shutdown protein | 4289 26 | 4301 40 | + | 404 | 111115244 | 4227750 |
| Complete genome (Accession number NC_008277) | adenylate kinase | 4301 79 | 4308 14 | + | 211 | 111115245 | 4227751 |
| Complete genome (Accession number NC_008277) | hypothetical protein BAPKO_0434 | 4308 15 | 4318 64 | + | 349 | 111115246 | 4227752 |
| Complete genome (Accession number NC_008277) | response regulatory protein | 4318 96 | 4328 19 | - | 307 | 111115247 | 4227753 |
| Complete genome (Accession number NC_008277) | sensory transduction histidine kinase/respons e regulator | 4328 22 | 4373 03 | - | 1493 | 111115248 | 4227754 |
| Complete genome (Accession number NC_008277) | hydrolase | 4439 02 | 4447 44 | - | 280 | 111115249 | 4227759 |
| Complete genome (Accession number NC_008277) | 3-methyladenine DNA glycosylase | 4448 80 | 4454 40 | - | 186 | 111115250 | 4227760 |
| Complete genome (Accession number NC_008277) | hypothetical protein BAPKO_0443 | 4459 66 | 4460 49 | + | 27 | 111115251 | 4227761 |
| Complete genome (Accession number NC_008277) | hypothetical protein BAPKO_0448 | 4501 81 | 4507 29 | - | 182 | 111115253 | 4227575 |
| Complete genome (Accession number NC_008277) | hypothetical protein BAPKO_0449 | 4508 19 | 4514 96 | - | 225 | 111115254 | 4227576 |
| Complete genome (Accession number | hypothetical protein BAPKO_0450 | 4517 09 | 4520 26 | + | 105 | 111115255 | 4227577 |

| | | | | | | | |
|---|---|---|---|---|---|---|---|
| NC_008277) | | | | | | | |
| Complete genome (Accession number NC_008277) | hypothetical protein BAPKO_0451 | 4520 57 | 4524 46 | - | 129 | 111115256 | 4227578 |
| Complete genome (Accession number NC_008277) | hypothetical protein BAPKO_0452 | 4525 73 | 4531 69 | + | 198 | 111115257 | 4227579 |
| Complete genome (Accession number NC_008277) | chromosome segregation protein, putative | 4532 78 | 4540 30 | + | 250 | 111115258 | 4227580 |
| Complete genome (Accession number NC_008277) | hypothetical protein BAPKO_0454 | 4540 33 | 4547 46 | + | 237 | 111115259 | 4227581 |
| Complete genome (Accession number NC_008277) | stage 0 sporulation protein J | 4550 34 | 4558 16 | - | 260 | 111115260 | 4227582 |
| Complete genome (Accession number NC_008277) | hypothetical protein BAPKO_0456 | 4558 62 | 4559 51 | + | 29 | 111115261 | 4227583 |
| Complete genome (Accession number NC_008277) | DNA gyrase, subunit A | 4559 34 | 4583 66 | - | 810 | 111115262 | 4227584 |
| Complete genome (Accession number NC_008277) | DNA gyrase, subunit B | 4583 78 | 4602 82 | - | 634 | 111115263 | 4227585 |
| Complete genome (Accession number NC_008277) | chromosomal replication initiator protein | 4604 69 | 4619 26 | + | 485 | 111115264 | 4227586 |
| Complete genome (Accession number NC_008277) | DNA polymerase III, subunit beta | 4621 59 | 4633 19 | + | 386 | 111115265 | 4227587 |
| Complete genome (Accession number | hypothetical protein BAPKO_0461 | 4634 11 | 4637 10 | + | 99 | 111115266 | 4227588 |

| | | | | | | | |
|---|---|---|---|---|---|---|---|
| NC_008277) | | | | | | | |
| Complete genome (Accession number NC_008277) | 50S ribosomal protein L34 | 4638 02 | 4639 57 | + | 51 | 111115267 | 4227589 |
| Complete genome (Accession number NC_008277) | ribonuclease P protein component | 4639 38 | 4642 73 | + | 111 | 111115268 | 4227590 |
| Complete genome (Accession number NC_008277) | putative inner membrane protein translocase component YidC | 4642 84 | 4659 18 | + | 544 | 111115269 | 4227764 |
| Complete genome (Accession number NC_008277) | spoIIIJ-associtated protein | 4659 32 | 4666 60 | + | 242 | 111115270 | 4227765 |
| Complete genome (Accession number NC_008277) | nucleotide sugar epimerase | 4667 04 | 4677 71 | + | 355 | 111115271 | 4227766 |
| Complete genome (Accession number NC_008277) | fructose-bisphosphate aldolase | 4679 33 | 4690 12 | + | 359 | 111115272 | 4227767 |
| Complete genome (Accession number NC_008277) | aspartyl-tRNA synthetase | 4694 19 | 4711 79 | - | 586 | 111115273 | 4227768 |
| Complete genome (Accession number NC_008277) | Na+/H+ antiporter | 4711 83 | 4732 88 | - | 701 | 111115274 | 4227769 |
| Complete genome (Accession number NC_008277) | phosphocarrier protein HPr | 4732 91 | 4735 66 | - | 91 | 111115275 | 4227770 |
| Complete genome (Accession number NC_008277) | hypothetical protein BAPKO_0471 | 4735 79 | 4738 72 | - | 97 | 111115276 | 4227771 |
| Complete genome (Accession | RNA polymerase sigma-54 factor | 4738 62 | 4751 21 | - | 419 | 111115277 | 4227772 |

| | | | | | | | |
|---|---|---|---|---|---|---|---|
| number NC_008277) | | | | | | | |
| Complete genome (Accession number NC_008277) | chromate transport protein, putative | 4751 24 | 4756 57 | - | 177 | 111115278 | 4227773 |
| Complete genome (Accession number NC_008277) | hypothetical protein BAPKO_0474 | 4756 54 | 4762 59 | - | 201 | 111115279 | 4227774 |
| Complete genome (Accession number NC_008277) | hypothetical protein BAPKO_0475 | 4764 71 | 4773 13 | + | 280 | 111115280 | 4227775 |
| Complete genome (Accession number NC_008277) | hypothetical protein BAPKO_0476 | 4772 88 | 4774 04 | - | 38 | 111115281 | 4227776 |
| Complete genome (Accession number NC_008277) | lipopolysacchar ide biosynthesis-related protein | 4773 10 | 4784 58 | - | 382 | 111115282 | 4227777 |
| Complete genome (Accession number NC_008277) | hypothetical protein BAPKO_0478 | 4785 33 | 4795 04 | + | 323 | 111115283 | 4227778 |
| Complete genome (Accession number NC_008277) | hypothetical protein BAPKO_0479 | 4795 08 | 4801 46 | + | 212 | 111115284 | 4227779 |
| Complete genome (Accession number NC_008277) | hypothetical protein BAPKO_0480 | 4802 09 | 4803 52 | + | 47 | 111115285 | 4227780 |
| Complete genome (Accession number NC_008277) | excinuclease ABC, subunit C | 4802 35 | 4819 62 | - | 575 | 111115286 | 4227781 |
| Complete genome (Accession number NC_008277) | hypothetical protein BAPKO_0482 | 4821 53 | 4836 70 | + | 505 | 111115287 | 4227782 |
| Complete genome (Accession number | hypothetical protein BAPKO_0483 | 4836 63 | 4849 73 | + | 436 | 111115288 | 4227352 |

| NC_008277) | | | | | | | |
|---|---|---|---|---|---|---|---|
| Complete genome (Accession number NC_008277) | lipoprotein, putative | 4852 99 | 4860 24 | - | 241 | 111115289 | 4227641 |
| Complete genome (Accession number NC_008277) | hypothetical protein BAPKO_0489 | 4862 25 | 4865 36 | + | 103 | 111115290 | 4227644 |
| Complete genome (Accession number NC_008277) | DNA polymerase III subunits gamma and tau | 4865 87 | 4882 87 | + | 566 | 111115291 | 4227645 |
| Complete genome (Accession number NC_008277) | hypothetical protein BAPKO_0491 | 4882 92 | 4885 91 | + | 99 | 111115292 | 4227646 |
| Complete genome (Accession number NC_008277) | nucleoside-diphosphate kinase | 4887 61 | 4892 70 | + | 169 | 111115293 | 4227254 |
| Complete genome (Accession number NC_008277) | hypothetical protein BAPKO_0493 | 4895 66 | 4900 93 | + | 175 | 111115294 | 4227255 |
| Complete genome (Accession number NC_008277) | hypothetical protein BAPKO_0494 | 4900 90 | 4907 85 | + | 231 | 111115295 | 4227256 |
| Complete genome (Accession number NC_008277) | ABC transporter, ATP-binding protein | 4907 54 | 4915 54 | + | 266 | 111115296 | 4227257 |
| Complete genome (Accession number NC_008277) | hypothetical protein BAPKO_0496 | 4915 51 | 4922 40 | + | 229 | 111115297 | 4227258 |
| Complete genome (Accession number NC_008277) | hypothetical protein BAPKO_0497 | 4922 42 | 4929 88 | + | 248 | 111115298 | 4227259 |
| Complete genome (Accession number | signal peptidase II | 4929 99 | 4935 11 | + | 170 | 111115299 | 4227949 |

| | | | | | | | |
|---|---|---|---|---|---|---|---|
| NC_008277) | | | | | | | |
| Complete genome (Accession number NC_008277) | hypothetical protein BAPKO_0499 | 4935 08 | 4937 35 | + | 75 | 111115300 | 4227950 |
| Complete genome (Accession number NC_008277) | hypothetical protein BAPKO_0500 | 4937 49 | 4945 70 | + | 273 | 111115301 | 4227951 |
| Complete genome (Accession number NC_008277) | UDP-N-acetylglucosam ine 1-carboxyvinyltra nsferase | 4946 76 | 4959 59 | + | 427 | 111115302 | 4227952 |
| Complete genome (Accession number NC_008277) | hypothetical protein BAPKO_0502 | 4964 49 | 4978 13 | + | 454 | 111115303 | 4227953 |
| Complete genome (Accession number NC_008277) | lipoprotein, putative | 4979 02 | 4982 91 | - | 129 | 111115304 | 4227954 |
| Complete genome (Accession number NC_008277) | elongation factor Tu | 4987 82 | 4999 66 | + | 394 | 111115305 | 4227955 |
| Complete genome (Accession number NC_008277) | 30S ribosomal protein S10 | 5000 18 | 5003 29 | + | 103 | 111115306 | 4227956 |
| Complete genome (Accession number NC_008277) | 50S ribosomal protein L3 | 5003 65 | 5009 85 | + | 206 | 111115307 | 4227957 |
| Complete genome (Accession number NC_008277) | 50S ribosomal protein L4 | 5009 95 | 5016 24 | + | 209 | 111115308 | 4227958 |
| Complete genome (Accession number NC_008277) | 50S ribosomal protein L23 | 5016 45 | 5019 41 | + | 98 | 111115309 | 4227164 |
| Complete genome (Accession number NC_008277) | 50S ribosomal protein L2 | 5019 63 | 5027 96 | + | 277 | 111115310 | 4227165 |

| | | | | | | | |
|---|---|---|---|---|---|---|---|
| Complete genome (Accession number NC_008277) | 30S ribosomal protein S19 | 5028 06 | 5030 84 | + | 92 | 111115311 | 4227166 |
| Complete genome (Accession number NC_008277) | 50S ribosomal protein L22 | 5030 92 | 5034 54 | + | 120 | 111115312 | 4227167 |
| Complete genome (Accession number NC_008277) | 30S ribosomal protein S3 | 5034 58 | 5043 33 | + | 291 | 111115313 | 4227168 |
| Complete genome (Accession number NC_008277) | 50S ribosomal protein L16 | 5043 38 | 5047 54 | + | 138 | 111115314 | 4227169 |
| Complete genome (Accession number NC_008277) | 50S ribosomal protein L29 | 5047 57 | 5049 57 | + | 66 | 111115315 | 4227170 |
| Complete genome (Accession number NC_008277) | 30S ribosomal protein S17 | 5049 60 | 5052 14 | + | 84 | 111115316 | 4227171 |
| Complete genome (Accession number NC_008277) | 50S ribosomal protein L14 | 5052 42 | 5056 10 | + | 122 | 111115317 | 4227172 |
| Complete genome (Accession number NC_008277) | 50S ribosomal protein L24 | 5056 25 | 5059 30 | + | 101 | 111115318 | 4227173 |
| Complete genome (Accession number NC_008277) | 50S ribosomal protein L5 | 5059 36 | 5064 84 | + | 182 | 111115319 | 4227895 |
| Complete genome (Accession number NC_008277) | 30S ribosomal protein S14 | 5065 02 | 5066 87 | + | 61 | 111115320 | 4227896 |
| Complete genome (Accession number NC_008277) | 30S ribosomal protein S8 | 5066 97 | 5070 95 | + | 132 | 111115321 | 4227897 |

| | | | | | | | |
|---|---|---|---|---|---|---|---|
| Complete genome (Accession number NC_008277) | 50S ribosomal protein L6 | 5071 12 | 5076 54 | + | 180 | 111115322 | 4227898 |
| Complete genome (Accession number NC_008277) | 50S ribosomal protein L18 | 5076 72 | 5080 31 | + | 119 | 111115323 | 4227899 |
| Complete genome (Accession number NC_008277) | 30S ribosomal protein S5 | 5080 44 | 5085 41 | + | 165 | 111115324 | 4227900 |
| Complete genome (Accession number NC_008277) | ribosomal protein L30 | 5085 45 | 5088 56 | + | 103 | 111115325 | 4227901 |
| Complete genome (Accession number NC_008277) | 50S ribosomal protein L15 | 5088 49 | 5092 86 | + | 145 | 111115326 | 4227902 |
| Complete genome (Accession number NC_008277) | preprotein translocase subunit SecY | 5092 99 | 5106 03 | + | 434 | 111115327 | 4227903 |
| Complete genome (Accession number NC_008277) | 50S ribosomal protein L36 | 5106 16 | 5107 29 | + | 37 | 111115328 | 4227904 |
| Complete genome (Accession number NC_008277) | 30S ribosomal protein S13 | 5107 47 | 5111 24 | + | 125 | 111115329 | 4227905 |
| Complete genome (Accession number NC_008277) | 30S ribosomal protein S11 | 5111 52 | 5115 44 | + | 130 | 111115330 | 4227906 |
| Complete genome (Accession number NC_008277) | DNA-directed RNA polymerase subunit alpha | 5115 60 | 5125 94 | + | 344 | 111115331 | 4227907 |
| Complete genome (Accession number NC_008277) | 50S ribosomal protein L17 | 5126 16 | 5129 87 | + | 123 | 111115332 | 4227908 |

| | | | | | | | |
|---|---|---|---|---|---|---|---|
| Complete genome (Accession number NC_008277) | hypothetical protein BAPKO_0532 | 5130 64 | 5145 93 | + | 509 | 111115333 | 4227909 |
| Complete genome (Accession number NC_008277) | hypothetical protein BAPKO_0533 | 5145 83 | 5153 71 | + | 262 | 111115334 | 4227910 |
| Complete genome (Accession number NC_008277) | hemolysin | 5153 52 | 5161 43 | + | 263 | 111115335 | 4227911 |
| Complete genome (Accession number NC_008277) | hypothetical protein BAPKO_0535 | 5161 40 | 5168 86 | - | 248 | 111115336 | 4227912 |
| Complete genome (Accession number NC_008277) | GTP-binding protein EngA | 5169 73 | 5182 74 | + | 433 | 111115337 | 4227913 |
| Complete genome (Accession number NC_008277) | hypothetical protein BAPKO_0537 | 5182 71 | 5195 30 | + | 419 | 111115338 | 4227193 |
| Complete genome (Accession number NC_008277) | hypothetical protein BAPKO_0538 | 5195 27 | 5202 13 | + | 228 | 111115339 | 4227194 |
| Complete genome (Accession number NC_008277) | hypothetical protein BAPKO_0539 | 5202 33 | 5267 21 | + | 2162 | 111115340 | 4227195 |
| Complete genome (Accession number NC_008277) | phenylalanyl-tRNA synthetase subunit alpha | 5267 33 | 5282 95 | + | 520 | 111115341 | 4227196 |
| Complete genome (Accession number NC_008277) | phenylalanyl-tRNA synthetase subunit beta | 5282 83 | 5299 86 | + | 567 | 111115342 | 4227197 |
| Complete genome (Accession number NC_008277) | thioredoxin reductase | 5300 55 | 5310 35 | + | 326 | 111115343 | 4227198 |
| Complete genome | rRNA methylase | 5310 56 | 5317 96 | - | 246 | 111115344 | 4227199 |

| | | | | | | | |
|---|---|---|---|---|---|---|---|
| (Accession number NC_008277) | | | | | | | |
| Complete genome (Accession number NC_008277) | heat shock protein | 5317 98 | 5328 92 | - | 364 | 111115345 | 4227200 |
| Complete genome (Accession number NC_008277) | heat shock protein 70 | 5328 92 | 5347 99 | - | 635 | 111115346 | 4227201 |
| Complete genome (Accession number NC_008277) | grpE protein | 5348 23 | 5353 86 | - | 187 | 111115347 | 4227202 |
| Complete genome (Accession number NC_008277) | NH(3)- dependent NAD+ synthetase | 5357 64 | 5373 02 | + | 512 | 111115348 | 4227203 |
| Complete genome (Accession number NC_008277) | hypothetical protein BAPKO_0548 | 5375 45 | 5377 24 | + | 59 | 111115349 | 4227204 |
| Complete genome (Accession number NC_008277) | hypothetical protein BAPKO_0549 | 5380 68 | 5381 54 | + | 28 | 111115350 | 4227205 |
| Complete genome (Accession number NC_008277) | hypothetical protein BAPKO_0550 | 5382 08 | 5390 62 | + | 284 | 111115351 | 4227206 |
| Complete genome (Accession number NC_008277) | hypothetical protein BAPKO_0551 | 5390 89 | 5395 11 | - | 140 | 111115352 | 4227207 |
| Complete genome (Accession number NC_008277) | hypothetical protein BAPKO_0552 | 5396 16 | 5414 27 | + | 603 | 111115353 | 4227208 |
| Complete genome (Accession number NC_008277) | pantothenate kinase | 5414 20 | 5421 96 | + | 258 | 111115354 | 4227209 |
| Complete genome (Accession number | aldose reductase, putative | 5422 31 | 5431 78 | - | 315 | 111115355 | 4227210 |

| | | | | | | | |
|---|---|---|---|---|---|---|---|
| NC_008277) | | | | | | | |
| Complete genome (Accession number NC_008277) | hypothetical protein BAPKO_0555 | 5432 64 | 5440 73 | - | 269 | 111115356 | 4227211 |
| Complete genome (Accession number NC_008277) | hypothetical protein BAPKO_0556 | 5440 57 | 5446 71 | - | 204 | 111115357 | 4227212 |
| Complete genome (Accession number NC_008277) | hypothetical protein BAPKO_0557 | 5446 74 | 5459 81 | - | 435 | 111115358 | 4227128 |
| Complete genome (Accession number NC_008277) | phnP protein | 5461 77 | 5469 38 | + | 253 | 111115359 | 4227129 |
| Complete genome (Accession number NC_008277) | exodeoxyribon uclease III | 5469 59 | 5477 26 | + | 255 | 111115360 | 4227130 |
| Complete genome (Accession number NC_008277) | hypothetical protein BAPKO_0560 | 5477 42 | 5485 12 | + | 256 | 111115361 | 4227131 |
| Complete genome (Accession number NC_008277) | zinc protease, putative | 5484 93 | 5512 94 | + | 933 | 111115362 | 4227132 |
| Complete genome (Accession number NC_008277) | hypothetical protein BAPKO_0565 | 5517 46 | 5523 90 | - | 214 | 111115363 | 4227136 |
| Complete genome (Accession number NC_008277) | hypothetical protein BAPKO_0566 | 5523 87 | 5527 43 | - | 118 | 111115364 | 4227137 |
| Complete genome (Accession number NC_008277) | hypothetical protein BAPKO_0567 | 5528 28 | 5535 26 | - | 232 | 111115365 | 4227138 |
| Complete genome (Accession number | elongation factor G | 5535 61 | 5556 42 | - | 693 | 111115366 | 4227139 |

| | | | | | | | |
|---|---|---|---|---|---|---|---|
| NC_008277) | | | | | | | |
| Complete genome (Accession number NC_008277) | hypothetical protein BAPKO_0569 | 5559 41 | 5561 68 | + | 75 | 111115367 | 4227140 |
| Complete genome (Accession number NC_008277) | hypothetical protein BAPKO_0570 | 5564 00 | 5569 27 | + | 175 | 111115368 | 4227141 |
| Complete genome (Accession number NC_008277) | hypothetical protein BAPKO_0571 | 5570 48 | 5576 98 | + | 216 | 111115369 | 4227142 |
| Complete genome (Accession number NC_008277) | hypothetical protein BAPKO_0572 | 5577 71 | 5579 05 | + | 44 | 111115370 | 4227143 |
| Complete genome (Accession number NC_008277) | phosphoribosyl pyrophosphate synthetase | 5578 92 | 5591 12 | + | 406 | 111115371 | 4227108 |
| Complete genome (Accession number NC_008277) | xylulokinase | 5591 16 | 5604 80 | + | 454 | 111115372 | 4227109 |
| Complete genome (Accession number NC_008277) | hypothetical protein BAPKO_0575 | 5604 63 | 5612 09 | - | 248 | 111115373 | 4227110 |
| Complete genome (Accession number NC_008277) | dephospho-CoA kinase | 5611 93 | 5618 10 | - | 205 | 111115374 | 4227111 |
| Complete genome (Accession number NC_008277) | DNA polymerase I | 5617 92 | 5644 61 | - | 889 | 111115375 | 4227112 |
| Complete genome (Accession number NC_008277) | hypothetical protein BAPKO_0578 | 5645 15 | 5649 25 | - | 136 | 111115376 | 4227113 |
| Complete genome (Accession number NC_008277) | flagellar protein | 5649 15 | 5653 34 | - | 139 | 111115377 | 4227114 |

| | | | | | | | |
|---|---|---|---|---|---|---|---|
| Complete genome (Accession number NC_008277) | chemotaxis response regulator | 5653 43 | 5657 23 | - | 126 | 111115378 | 4227115 |
| Complete genome (Accession number NC_008277) | DNA ligase | 5659 73 | 5679 55 | + | 660 | 111115379 | 4227116 |
| Complete genome (Accession number NC_008277) | hypothetical protein BAPKO_0582 | 5679 79 | 5694 84 | - | 501 | 111115380 | 4227117 |
| Complete genome (Accession number NC_008277) | hypothetical protein BAPKO_0583 | 5697 64 | 5716 26 | + | 620 | 111115381 | 4227118 |
| Complete genome (Accession number NC_008277) | hypothetical protein BAPKO_0584 | 5716 13 | 5719 84 | + | 123 | 111115382 | 4227119 |
| Complete genome (Accession number NC_008277) | hypothetical protein BAPKO_0585 | 5720 73 | 5729 42 | + | 289 | 111115383 | 4227120 |
| Complete genome (Accession number NC_008277) | phosphocarrier protein HPr | 5730 80 | 5733 40 | + | 86 | 111115384 | 4227121 |
| Complete genome (Accession number NC_008277) | phosphoenolpy ruvate-protein phosphatase | 5733 61 | 5750 82 | + | 573 | 111115385 | 4227122 |
| Complete genome (Accession number NC_008277) | glucose-specific PTS system component | 5750 85 | 5756 54 | + | 189 | 111115386 | 4227123 |
| Complete genome (Accession number NC_008277) | heat shock protein 90 | 5756 91 | 5775 41 | - | 616 | 111115387 | 4227124 |
| Complete genome (Accession number NC_008277) | 6-phosphoglucon ate dehydrogenase | 5776 54 | 5790 48 | + | 464 | 111115388 | 4227125 |

| | | | | | | | |
|---|---|---|---|---|---|---|---|
| Complete genome (Accession number NC_008277) | hypothetical protein BAPKO_0591 | 5790 79 | 5796 24 | - | 181 | 111115389 | 4227126 |
| Complete genome (Accession number NC_008277) | hypothetical protein BAPKO_0592 | 5797 27 | 5798 16 | + | 29 | 111115390 | 4227127 |
| Complete genome (Accession number NC_008277) | hypothetical protein BAPKO_0593 | 5798 09 | 5803 27 | - | 172 | 111115391 | 4227296 |
| Complete genome (Accession number NC_008277) | hypothetical protein BAPKO_0594 | 5804 10 | 5809 97 | - | 195 | 111115392 | 4227297 |
| Complete genome (Accession number NC_008277) | purine-binding chemotaxis protein | 5812 10 | 5817 46 | + | 178 | 111115393 | 4227298 |
| Complete genome (Accession number NC_008277) | hypothetical protein BAPKO_0596 | 5817 59 | 5820 70 | + | 103 | 111115394 | 4227299 |
| Complete genome (Accession number NC_008277) | chemotaxis histidine kinase | 5820 86 | 5842 27 | + | 713 | 111115395 | 4227300 |
| Complete genome (Accession number NC_008277) | protein-glutamate methylesterase | 5842 85 | 5854 42 | + | 385 | 111115396 | 4227301 |
| Complete genome (Accession number NC_008277) | hypothetical protein BAPKO_0599 | 5854 45 | 5872 17 | + | 590 | 111115397 | 4227302 |
| Complete genome (Accession number NC_008277) | chemotaxis response regulator | 5872 58 | 5876 32 | + | 124 | 111115398 | 4227303 |
| Complete genome (Accession number NC_008277) | uridylate kinase | 5879 59 | 5886 48 | - | 229 | 111115399 | 4227305 |
| Complete genome | glycosyl transferase | 5888 61 | 5899 25 | + | 354 | 111115400 | 4227306 |

| | | | | | | | |
|---|---|---|---|---|---|---|---|
| (Accession number NC_008277) | | | | | | | |
| Complete genome (Accession number NC_008277) | ABC transporter, ATP-binding protein | 5899 88 | 5907 94 | + | 268 | 111115401 | 4227307 |
| Complete genome (Accession number NC_008277) | hypothetical protein BAPKO_0605 | 5908 01 | 5916 52 | + | 283 | 111115402 | 4227308 |
| Complete genome (Accession number NC_008277) | CTP synthetase | 5918 39 | 5934 40 | + | 533 | 111115403 | 4227309 |
| Complete genome (Accession number NC_008277) | hypothetical protein BAPKO_0607 | 5934 57 | 5939 81 | - | 174 | 111115404 | 4227310 |
| Complete genome (Accession number NC_008277) | hypothetical protein BAPKO_0608 | 5941 19 | 5947 09 | + | 196 | 111115405 | 4227311 |
| Complete genome (Accession number NC_008277) | methyl-accepting chemotaxis protein | 5949 56 | 5961 28 | + | 390 | 111115406 | 4227312 |
| Complete genome (Accession number NC_008277) | DNA polymerase III subunit alpha | 5962 16 | 5997 01 | + | 1161 | 111115407 | 4227313 |
| Complete genome (Accession number NC_008277) | hypothetical protein BAPKO_0611 | 5997 10 | 6002 91 | + | 193 | 111115408 | 4227914 |
| Complete genome (Accession number NC_008277) | DNA recombinase | 6003 01 | 6023 61 | + | 686 | 111115409 | 4227915 |
| Complete genome (Accession number NC_008277) | hypothetical protein BAPKO_0613 | 6023 58 | 6031 46 | - | 262 | 111115410 | 4227916 |
| Complete genome (Accession number | hypothetical protein BAPKO_0614 | 6032 75 | 6046 24 | + | 449 | 111115411 | 4227917 |

| | | | | | | | |
|---|---|---|---|---|---|---|---|
| NC_008277) | | | | | | | |
| Complete genome (Accession number NC_008277) | hypothetical protein BAPKO_0615 | 6047 79 | 6061 25 | + | 448 | 111115412 | 4227918 |
| Complete genome (Accession number NC_008277) | UDP-N-acetylmuramoyl alanine--D-glutamateligase | 6061 44 | 6074 99 | + | 451 | 111115413 | 4227919 |
| Complete genome (Accession number NC_008277) | femA protein | 6074 96 | 6085 39 | - | 347 | 111115414 | 4227920 |
| Complete genome (Accession number NC_008277) | methionyl-tRNA synthetase | 6085 49 | 6107 23 | - | 724 | 111115415 | 4227921 |
| Complete genome (Accession number NC_008277) | 5-methylthioaden osine/S-adenosylhomoc ysteine nucleosidase, putative | 6108 82 | 6116 76 | + | 264 | 111115416 | 4227922 |
| Complete genome (Accession number NC_008277) | phosphate acetyltransfera se | 6117 68 | 6128 05 | + | 345 | 111115417 | 4227923 |
| Complete genome (Accession number NC_008277) | dimethyladenos ine transferase | 6127 88 | 6136 33 | - | 281 | 111115418 | 4227924 |
| Complete genome (Accession number NC_008277) | competence locus E, putative | 6136 66 | 6149 22 | - | 418 | 111115419 | 4227925 |
| Complete genome (Accession number NC_008277) | hypothetical protein BAPKO_0623 | 6150 78 | 6157 52 | + | 224 | 111115420 | 4227926 |
| Complete genome (Accession number NC_008277) | long-chain-fatty-acid CoA ligase | 6157 78 | 6177 15 | + | 645 | 111115421 | 4227927 |
| Complete genome | arginyl-tRNA synthetase | 6177 44 | 6195 01 | - | 585 | 111115422 | 4227928 |

| | | | | | | | |
|---|---|---|---|---|---|---|---|
| (Accession number NC_008277) | | | | | | | |
| Complete genome (Accession number NC_008277) | hypothetical protein BAPKO_0626 | 6194 94 | 6195 44 | - | 16 | 111115423 | 4227929 |
| Complete genome (Accession number NC_008277) | hypothetical protein BAPKO_0627 | 6195 47 | 6201 46 | - | 199 | 111115424 | 4227930 |
| Complete genome (Accession number NC_008277) | methyl-accepting chemotaxis protein | 6201 68 | 6223 15 | - | 715 | 111115425 | 4227931 |
| Complete genome (Accession number NC_008277) | methyl-accepting chemotaxis protein | 6225 83 | 6247 90 | + | 735 | 111115426 | 4227932 |
| Complete genome (Accession number NC_008277) | UDP-N-acetylmuramat e dehydrogenase | 6247 95 | 6257 06 | - | 303 | 111115427 | 4227933 |
| Complete genome (Accession number NC_008277) | cysteinyl-tRNA synthetase | 6257 58 | 6272 00 | + | 480 | 111115428 | 4227934 |
| Complete genome (Accession number NC_008277) | hypothetical protein BAPKO_0632 | 6270 97 | 6272 40 | + | 47 | 111115429 | 4227935 |
| Complete genome (Accession number NC_008277) | hypothetical protein BAPKO_0633 | 6272 04 | 6287 60 | + | 518 | 111115430 | 4227936 |
| Complete genome (Accession number NC_008277) | serine hydroxymethylt ransferase | 6287 60 | 6300 13 | + | 417 | 111115431 | 4227937 |
| Complete genome (Accession number NC_008277) | DnaJ domain containing protein | 6300 40 | 6307 92 | + | 250 | 111115432 | 4227938 |
| Complete genome (Accession number | membrane-associated protein p66 | 6308 53 | 6327 12 | - | 619 | 111115433 | 4227939 |

| | | | | | | | |
|---|---|---|---|---|---|---|---|
| NC_008277) | | | | | | | |
| Complete genome (Accession number NC_008277) | L-lactate permease | 6329 51 | 6344 53 | - | 500 | 111115434 | 4227940 |
| Complete genome (Accession number NC_008277) | serine-type D-Ala-D-Ala carboxypeptida se | 6345 38 | 6357 43 | + | 401 | 111115435 | 4227941 |
| Complete genome (Accession number NC_008277) | hypothetical protein BAPKO_0639 | 6357 60 | 6362 51 | + | 163 | 111115436 | 4227942 |
| Complete genome (Accession number NC_008277) | rep helicase, single-stranded DNA-dependent ATPase | 6362 54 | 6382 33 | + | 659 | 111115437 | 4227943 |
| Complete genome (Accession number NC_008277) | aminoacyl-histidine dipeptidase | 6383 46 | 6397 76 | + | 476 | 111115438 | 4227944 |
| Complete genome (Accession number NC_008277) | trigger factor | 6403 54 | 6417 12 | + | 452 | 111115439 | 4227232 |
| Complete genome (Accession number NC_008277) | ATP-dependent Clp protease proteolytic component | 6417 18 | 6423 11 | + | 197 | 111115440 | 4227233 |
| Complete genome (Accession number NC_008277) | ATP-dependent protease ATP-binding subunit | 6423 36 | 6436 28 | + | 430 | 111115441 | 4227234 |
| Complete genome (Accession number NC_008277) | ATP-dependent protease LA | 6435 97 | 6460 05 | + | 802 | 111115442 | 4227235 |
| Complete genome (Accession number NC_008277) | hypothetical protein BAPKO_0650 | 6460 45 | 6461 97 | + | 50 | 111115443 | 4227236 |
| Complete genome (Accession number | hypothetical protein BAPKO_0651 | 6461 42 | 6462 49 | + | 35 | 111115444 | 4227237 |

| | | | | | | | |
|---|---|---|---|---|---|---|---|
| NC_008277) | | | | | | | |
| Complete genome (Accession number NC_008277) | 30S ribosomal protein S4 | 6462 61 | 6468 87 | - | 208 | 111115445 | 4227238 |
| Complete genome (Accession number NC_008277) | hypothetical protein BAPKO_0654 | 6470 64 | 6484 25 | - | 453 | 111115446 | 4227240 |
| Complete genome (Accession number NC_008277) | hypothetical protein BAPKO_0655 | 6485 46 | 6489 14 | + | 122 | 111115447 | 4227241 |
| Complete genome (Accession number NC_008277) | cytidine deaminase | 6489 37 | 6493 77 | - | 146 | 111115448 | 4227242 |
| Complete genome (Accession number NC_008277) | hypothetical protein BAPKO_0657 | 6494 97 | 6504 59 | + | 320 | 111115449 | 4227243 |
| Complete genome (Accession number NC_008277) | hypothetical protein BAPKO_0658 | 6504 37 | 6520 32 | - | 531 | 111115450 | 4227244 |
| Complete genome (Accession number NC_008277) | 4-methyl-5(b-hydroxyethyl)-thiazole monophosphat e biosynthesis protein | 6523 31 | 6528 85 | + | 184 | 111115451 | 4227246 |
| Complete genome (Accession number NC_008277) | hypothetical protein BAPKO_0661 | 6528 82 | 6529 74 | + | 30 | 111115452 | 4227247 |
| Complete genome (Accession number NC_008277) | acetate kinase | 6529 47 | 6541 28 | - | 393 | 111115453 | 4227248 |
| Complete genome (Accession number NC_008277) | transcription-repair coupling factor | 6541 89 | 6575 63 | - | 1124 | 111115454 | 4227249 |
| Complete genome (Accession | hypothetical protein BAPKO_0664 | 6576 60 | 6586 22 | + | 320 | 111115455 | 4227250 |

| | | | | | | | |
|---|---|---|---|---|---|---|---|
| number NC_008277) | | | | | | | |
| Complete genome (Accession number NC_008277) | N-acetylmuramoyl-L-alanine amidase, putative | 6586 36 | 6606 78 | + | 680 | 111115456 | 4227251 |
| Complete genome (Accession number NC_008277) | small primase-like protein | 6607 72 | 6613 17 | - | 181 | 111115457 | 4227253 |
| Complete genome (Accession number NC_008277) | hypothetical protein BAPKO_0668 | 6612 86 | 6613 36 | - | 16 | 111115458 | 4227353 |
| Complete genome (Accession number NC_008277) | putative aminopeptidase 2 | 6613 20 | 6625 91 | - | 423 | 111115459 | 4227354 |
| Complete genome (Accession number NC_008277) | hypothetical protein BAPKO_0670 | 6626 76 | 6634 01 | + | 241 | 111115460 | 4227355 |
| Complete genome (Accession number NC_008277) | PTS system, fructose-specific IIABC component | 6634 29 | 6652 94 | - | 621 | 111115461 | 4227356 |
| Complete genome (Accession number NC_008277) | 1-phosphofructokinase | 6653 84 | 6663 07 | + | 307 | 111115462 | 4227357 |
| Complete genome (Accession number NC_008277) | hypothetical protein BAPKO_0673 | 6663 56 | 6666 73 | - | 105 | 111115463 | 4227358 |
| Complete genome (Accession number NC_008277) | exodeoxyribonuclease V, alpha chain | 6669 37 | 6687 69 | - | 610 | 111115464 | 4227360 |
| Complete genome (Accession number NC_008277) | exodeoxyribonuclease V, beta chain | 6687 66 | 6722 78 | - | 1170 | 111115465 | 4227361 |
| Complete genome (Accession number | exodeoxyribonuclease V, gamma chain | 6722 84 | 6755 23 | - | 1079 | 111115466 | 4227362 |

| | | | | | | | |
|---|---|---|---|---|---|---|---|
| NC_008277) | | | | | | | |
| Complete genome (Accession number NC_008277) | nicotinate phosphoribosylt ransferase | 6755 27 | 6769 72 | - | 481 | 111115467 | 4227363 |
| Complete genome (Accession number NC_008277) | glucose-6-phosphate 1-dehydrogenase | 6771 05 | 6785 41 | + | 478 | 111115468 | 4227364 |
| Complete genome (Accession number NC_008277) | Na+/H+ antiporter | 6788 42 | 6801 85 | + | 447 | 111115469 | 4227365 |
| Complete genome (Accession number NC_008277) | hypothetical protein BAPKO_0681 | 6801 33 | 6802 19 | - | 28 | 111115470 | 4227848 |
| Complete genome (Accession number NC_008277) | Na+/H+ antiporter | 6802 85 | 6816 40 | + | 451 | 111115471 | 4227849 |
| Complete genome (Accession number NC_008277) | spermidine/putr escine ABC transporter, binding periplasmic protein | 6816 86 | 6827 32 | - | 348 | 111115472 | 4227850 |
| Complete genome (Accession number NC_008277) | spermidine/putr escine ABC transporter, permease protein | 6827 52 | 6835 43 | - | 263 | 111115473 | 4227851 |
| Complete genome (Accession number NC_008277) | spermidine/putr escine ABC transporter, permease protein | 6835 47 | 6843 56 | - | 269 | 111115474 | 4227852 |
| Complete genome (Accession number NC_008277) | spermidine/putr escine ABC transporter, ATP- binding protein | 6843 56 | 6853 99 | - | 347 | 111115475 | 4227853 |
| Complete genome (Accession number NC_008277) | ribosomal biogenesis GTPase | 6854 78 | 6862 84 | - | 268 | 111115476 | 4227854 |
| Complete genome (Accession | N-acetylmannosa mine-6- | 6864 88 | 6871 86 | + | 232 | 111115477 | 4227855 |

| | | | | | | | |
|---|---|---|---|---|---|---|---|
| number NC_008277) | phosphate 2-epimerase | | | | | | |
| Complete genome (Accession number NC_008277) | PTS system, glucose-specific IIBC component | 6872 35 | 6887 79 | + | 514 | 111115478 | 4227856 |
| Complete genome (Accession number NC_008277) | hypothetical protein BAPKO_0690 | 6888 05 | 6897 88 | - | 327 | 111115479 | 4227857 |
| Complete genome (Accession number NC_008277) | ferric uptake regulation protein | 6897 85 | 6903 12 | - | 175 | 111115480 | 4227858 |
| Complete genome (Accession number NC_008277) | serine/threonine kinase, putative | 6904 12 | 6921 00 | - | 562 | 111115481 | 4227859 |
| Complete genome (Accession number NC_008277) | chaperonin GroEL | 6922 91 | 6939 28 | + | 545 | 111115482 | 4227860 |
| Complete genome (Accession number NC_008277) | hypothetical protein BAPKO_0694 | 6939 95 | 6942 91 | + | 98 | 111115483 | 4227861 |
| Complete genome (Accession number NC_008277) | hypothetical protein BAPKO_0695 | 6943 04 | 6946 18 | + | 104 | 111115484 | 4227862 |
| Complete genome (Accession number NC_008277) | preprotein translocase subunit SecD | 6946 84 | 6964 44 | + | 586 | 111115485 | 4227863 |
| Complete genome (Accession number NC_008277) | preprotein translocase subunit SecF | 6964 28 | 6973 27 | + | 299 | 111115486 | 4227864 |
| Complete genome (Accession number NC_008277) | hypothetical protein BAPKO_0698 | 6973 54 | 6984 96 | + | 380 | 111115487 | 4227865 |
| Complete genome (Accession number | heat shock protein | 6984 93 | 6993 23 | - | 276 | 111115488 | 4227866 |

| | | | | | | | |
|---|---|---|---|---|---|---|---|
| NC_008277) | | | | | | | |
| Complete genome (Accession number NC_008277) | HemN-related protein | 6994 32 | 7005 65 | + | 377 | 111115489 | 4227867 |
| Complete genome (Accession number NC_008277) | ribose 5-phosphate isomerase | 7005 52 | 7012 38 | - | 228 | 111115490 | 4227648 |
| Complete genome (Accession number NC_008277) | phosphoglycer ate mutase | 7013 83 | 7021 29 | + | 248 | 111115491 | 4227649 |
| Complete genome (Accession number NC_008277) | lysyl-tRNA synthetase | 7021 99 | 7037 64 | - | 521 | 111115492 | 4227650 |
| Complete genome (Accession number NC_008277) | GTP-binding protein Era | 7038 37 | 7047 09 | - | 290 | 111115493 | 4227651 |
| Complete genome (Accession number NC_008277) | hypothetical protein BAPKO_0705 | 7048 16 | 7051 72 | + | 118 | 111115494 | 4227652 |
| Complete genome (Accession number NC_008277) | hypothetical protein BAPKO_0706 | 7051 82 | 7055 92 | + | 136 | 111115495 | 4227653 |
| Complete genome (Accession number NC_008277) | hypothetical protein BAPKO_0707 | 7056 12 | 7060 61 | + | 149 | 111115496 | 4227654 |
| Complete genome (Accession number NC_008277) | hypothetical protein BAPKO_0708 | 7060 70 | 7067 59 | - | 229 | 111115497 | 4227655 |
| Complete genome (Accession number NC_008277) | hypothetical protein BAPKO_0709 | 7068 02 | 7077 61 | + | 319 | 111115498 | 4227656 |
| Complete genome (Accession number NC_008277) | hypothetical protein BAPKO_0710 | 7077 45 | 7087 52 | + | 335 | 111115499 | 4227657 |

| Complete genome (Accession number NC_008277) | hypothetical protein BAPKO_0711 | 7087 42 | 7092 93 | + | 183 | 111115500 | 4227658 |
|---|---|---|---|---|---|---|---|
| Complete genome (Accession number NC_008277) | flagellar filament outer layer protein | 7093 76 | 7104 01 | + | 341 | 111115501 | 4227659 |
| Complete genome (Accession number NC_008277) | chemotaxis histidine kinase | 7104 76 | 7130 70 | + | 864 | 111115502 | 4227660 |
| Complete genome (Accession number NC_008277) | purine-binding chemotaxis protein | 7130 78 | 7144 78 | + | 466 | 111115503 | 4227661 |
| Complete genome (Accession number NC_008277) | chemotaxis operon protein | 7144 90 | 7149 75 | + | 161 | 111115504 | 4227662 |
| Complete genome (Accession number NC_008277) | chemotaxis response regulator | 7150 15 | 7154 55 | + | 146 | 111115505 | 4227663 |
| Complete genome (Accession number NC_008277) | hypothetical protein BAPKO_0717 | 7155 04 | 7160 10 | - | 168 | 111115506 | 4227664 |
| Complete genome (Accession number NC_008277) | hypothetical protein BAPKO_0718 | 7160 07 | 7170 53 | - | 348 | 111115507 | 4227665 |
| Complete genome (Accession number NC_008277) | hypothetical protein BAPKO_0719 | 7170 47 | 7179 10 | - | 287 | 111115508 | 4227666 |
| Complete genome (Accession number NC_008277) | phosphoglycolate phosphatase | 7179 07 | 7185 69 | - | 220 | 111115509 | 4227962 |
| Complete genome (Accession number NC_008277) | ribose/galactose ABC transporter, ATP-binding protein | 7188 12 | 7204 22 | + | 536 | 111115510 | 4227963 |

| | | | | | | | |
|---|---|---|---|---|---|---|---|
| Complete genome (Accession number NC_008277) | ribose/galactose ABC transporter, permease protein | 7204 23 | 7215 74 | + | 383 | 111115511 | 4227964 |
| Complete genome (Accession number NC_008277) | ribose/galactose ABC transporter, permease protein | 7215 46 | 7224 72 | + | 308 | 111115512 | 4227965 |
| Complete genome (Accession number NC_008277) | methyl-accepting chemotaxis protein | 7226 29 | 7248 90 | + | 753 | 111115513 | 4227966 |
| Complete genome (Accession number NC_008277) | methyl-accepting chemotaxis protein | 7249 21 | 7268 22 | + | 633 | 111115514 | 4227967 |
| Complete genome (Accession number NC_008277) | tRNA (5-methylaminom ethyl-2-thiouridylate)-methyltransfera se | 7268 64 | 7279 31 | - | 355 | 111115515 | 4227968 |
| Complete genome (Accession number NC_008277) | 3-hydroxy-3-methylglutaryl-CoA synthase | 7280 27 | 7292 50 | + | 407 | 111115516 | 4227969 |
| Complete genome (Accession number NC_008277) | isopentenyl pyrophosphate isomerase | 7292 34 | 7302 98 | + | 354 | 111115517 | 4227970 |
| Complete genome (Accession number NC_008277) | 3-hydroxy-3-methylglutaryl-CoA reductase | 7302 88 | 7315 56 | + | 422 | 111115518 | 4227971 |
| Complete genome (Accession number NC_008277) | mevalonate pyrophosphate decarboxylase | 7315 40 | 7324 78 | + | 312 | 111115519 | 4227972 |
| Complete genome (Accession number NC_008277) | phosphomeval onate kinase, putative | 7324 69 | 7334 22 | + | 317 | 111115520 | 4227973 |
| Complete genome (Accession number NC_008277) | mevalonate kinase, putative | 7334 16 | 7343 09 | + | 297 | 111115521 | 4227974 |

| | | | | | | | |
|---|---|---|---|---|---|---|---|
| Complete genome (Accession number NC_008277) | hypothetical protein BAPKO_0734 | 7344 32 | 7348 99 | - | 155 | 111115522 | 4227976 |
| Complete genome (Accession number NC_008277) | neutrophil activating protein | 7349 84 | 7355 14 | + | 176 | 111115523 | 4227977 |
| Complete genome (Accession number NC_008277) | elongation factor G | 7355 91 | 7376 00 | + | 669 | 111115524 | 4227978 |
| Complete genome (Accession number NC_008277) | xylose operon regulatory protein | 7378 79 | 7390 87 | + | 402 | 111115525 | 4227107 |
| Complete genome (Accession number NC_008277) | signal recognition particle protein | 7391 50 | 7404 84 | + | 444 | 111115526 | 4227074 |
| Complete genome (Accession number NC_008277) | 30S ribosomal protein S16 | 7404 97 | 7407 57 | + | 86 | 111115527 | 4227075 |
| Complete genome (Accession number NC_008277) | hypothetical protein BAPKO_0740 | 7407 58 | 7410 06 | + | 82 | 111115528 | 4227076 |
| Complete genome (Accession number NC_008277) | hypothetical protein BAPKO_0741 | 7410 10 | 7415 10 | + | 166 | 111115529 | 4227077 |
| Complete genome (Accession number NC_008277) | tRNA (guanine-N(1)-)-methyltransfera se | 7415 07 | 7422 26 | + | 239 | 111115530 | 4227078 |
| Complete genome (Accession number NC_008277) | 50S ribosomal protein L19 | 7422 04 | 7425 63 | + | 119 | 111115531 | 4227079 |
| Complete genome (Accession number NC_008277) | hypothetical protein BAPKO_0744 | 7429 44 | 7434 83 | + | 179 | 111115532 | 4227080 |

| | | | | | | | |
|---|---|---|---|---|---|---|---|
| Complete genome (Accession number NC_008277) | lipopolysaccharide biosynthesis-related protein | 7434 85 | 7439 76 | + | 163 | 111115533 | 4227081 |
| Complete genome (Accession number NC_008277) | 50S ribosomal protein L32 | 7440 39 | 7442 21 | + | 60 | 111115534 | 4227082 |
| Complete genome (Accession number NC_008277) | acyl carrier protein | 7442 45 | 7444 87 | + | 80 | 111115535 | 4227083 |
| Complete genome (Accession number NC_008277) | ribonuclease III | 7444 87 | 7452 24 | + | 245 | 111115536 | 4227084 |
| Complete genome (Accession number NC_008277) | polynucleotide adenylyltransferase | 7451 95 | 7464 27 | - | 410 | 111115537 | 4227085 |
| Complete genome (Accession number NC_008277) | hypothetical protein BAPKO_0750 | 7465 45 | 7483 17 | + | 590 | 111115538 | 4227086 |
| Complete genome (Accession number NC_008277) | hypothetical protein BAPKO_0751 | 7483 23 | 7486 46 | + | 107 | 111115539 | 4227087 |
| Complete genome (Accession number NC_008277) | hypothetical protein BAPKO_0754 | 7491 04 | 7501 35 | + | 343 | 111115540 | 4227090 |
| Complete genome (Accession number NC_008277) | DNA primase | 7501 41 | 7519 22 | + | 593 | 111115541 | 4227091 |
| Complete genome (Accession number NC_008277) | RNA polymerase sigma factor RpoD | 7519 26 | 7538 21 | + | 631 | 111115542 | 4227055 |
| Complete genome (Accession number NC_008277) | hypothetical protein BAPKO_0757 | 7538 18 | 7545 94 | + | 258 | 111115543 | 4227056 |

| | | | | | | | |
|---|---|---|---|---|---|---|---|
| Complete genome (Accession number NC_008277) | hypothetical protein BAPKO_0758 | 7549 99 | 7559 67 | + | 322 | 111115544 | 4227057 |
| Complete genome (Accession number NC_008277) | rod shape-determining protein | 7559 42 | 7570 27 | + | 361 | 111115545 | 4227058 |
| Complete genome (Accession number NC_008277) | rod shape-determining protein | 7570 31 | 7578 76 | + | 281 | 111115546 | 4227059 |
| Complete genome (Accession number NC_008277) | hypothetical protein BAPKO_0761 | 7578 76 | 7583 58 | + | 160 | 111115547 | 4227060 |
| Complete genome (Accession number NC_008277) | penicillin-binding protein | 7583 58 | 7601 57 | + | 599 | 111115548 | 4227061 |
| Complete genome (Accession number NC_008277) | rod shape-determining protein | 7601 62 | 7614 78 | + | 438 | 111115549 | 4227062 |
| Complete genome (Accession number NC_008277) | threonyl-tRNA synthetase | 7616 45 | 7633 90 | + | 581 | 111115550 | 4227063 |
| Complete genome (Accession number NC_008277) | CDP-diacylglycerol--glycerol-3-phosphate 3-phosphatidyltransferase | 7633 95 | 7640 24 | + | 209 | 111115551 | 4227064 |
| Complete genome (Accession number NC_008277) | hypothetical protein BAPKO_0766 | 7640 11 | 7654 56 | + | 481 | 111115552 | 4227065 |
| Complete genome (Accession number NC_008277) | adenylyl cyclase, CyaB-type, putative | 7654 30 | 7659 63 | - | 177 | 111115553 | 4227066 |
| Complete genome (Accession number NC_008277) | K+ transport protein | 7660 63 | 7673 82 | + | 439 | 111115554 | 4227067 |

447

| | | | | | | | |
|---|---|---|---|---|---|---|---|
| Complete genome (Accession number NC_008277) | hypothetical protein BAPKO_0769 | 7673 86 | 7681 68 | + | 260 | 111115555 | 4227068 |
| Complete genome (Accession number NC_008277) | minD-related ATP-binding protein | 7681 78 | 7691 49 | + | 323 | 111115556 | 4227069 |
| Complete genome (Accession number NC_008277) | diphosphate--fructose-6-phosphate 1-phosphotransfe rase | 7691 80 | 7705 23 | + | 447 | 111115557 | 4227070 |
| Complete genome (Accession number NC_008277) | coenzyme A disulfide reductase | 7705 38 | 7718 69 | - | 443 | 111115558 | 4227071 |
| Complete genome (Accession number NC_008277) | glutamate transporter | 7718 84 | 7732 75 | - | 463 | 111115559 | 4227072 |
| Complete genome (Accession number NC_008277) | glucose-6-phosphate isomerase | 7733 40 | 7749 32 | - | 530 | 111115560 | 4227073 |
| Complete genome (Accession number NC_008277) | hypothetical protein BAPKO_0775 | 7749 55 | 7753 56 | - | 133 | 111115561 | 4227484 |
| Complete genome (Accession number NC_008277) | penicillin-binding protein | 7753 63 | 7781 34 | - | 923 | 111115562 | 4227526 |
| Complete genome (Accession number NC_008277) | hypothetical protein BAPKO_0777 | 7784 29 | 7792 14 | + | 261 | 111115563 | 4227527 |
| Complete genome (Accession number NC_008277) | hypothetical protein BAPKO_0778 | 7794 20 | 7804 24 | - | 334 | 111115564 | 4227528 |
| Complete genome (Accession number NC_008277) | rare lipoprotein A | 7805 04 | 7813 16 | + | 270 | 111115565 | 4227529 |

| | | | | | | | |
|---|---|---|---|---|---|---|---|
| Complete genome (Accession number NC_008277) | histidine phosphokinase/ phophatase, putative | 7815 12 | 7825 16 | + | 334 | 111115566 | 4227530 |
| Complete genome (Accession number NC_008277) | valyl-tRNA synthetase | 7825 06 | 7851 33 | + | 875 | 111115567 | 4227531 |
| Complete genome (Accession number NC_008277) | hypothetical protein BAPKO_0782 | 7852 24 | 7858 23 | - | 199 | 111115568 | 4227532 |
| Complete genome (Accession number NC_008277) | hypothetical protein BAPKO_0783 | 7858 97 | 7862 44 | - | 115 | 111115569 | 4227533 |
| Complete genome (Accession number NC_008277) | co-chaperonin GroES | 7867 33 | 7870 05 | - | 90 | 111115570 | 4227534 |
| Complete genome (Accession number NC_008277) | ABC transporter, ATP-binding protein | 7871 63 | 7887 94 | + | 543 | 111115571 | 4227535 |
| Complete genome (Accession number NC_008277) | hypothetical protein BAPKO_0786 | 7888 54 | 7905 09 | + | 551 | 111115572 | 4227536 |
| Complete genome (Accession number NC_008277) | antigen, p83/100 | 7906 13 | 7926 04 | + | 663 | 111115573 | 4227537 |
| Complete genome (Accession number NC_008277) | hypothetical protein BAPKO_0788 | 7926 37 | 7927 38 | - | 33 | 111115574 | 4227538 |
| Complete genome (Accession number NC_008277) | endonuclease III | 7929 21 | 7935 65 | + | 214 | 111115575 | 4227541 |
| Complete genome (Accession number NC_008277) | oligopeptide ABC transporter, permease protein | 7935 74 | 7944 34 | - | 286 | 111115576 | 4227542 |

| | | | | | | | |
|---|---|---|---|---|---|---|---|
| Complete genome (Accession number NC_008277) | oligopeptide ABC transporter, permease protein | 7944 34 | 7954 14 | - | 326 | 111115577 | 4227632 |
| Complete genome (Accession number NC_008277) | hypothetical protein BAPKO_0794 | 7954 21 | 7958 79 | - | 152 | 111115578 | 4227633 |
| Complete genome (Accession number NC_008277) | hypothetical protein BAPKO_0795 | 7959 78 | 7970 99 | + | 373 | 111115579 | 4227634 |
| Complete genome (Accession number NC_008277) | hypothetical protein BAPKO_0796 | 7973 34 | 7983 47 | - | 337 | 111115580 | 4227635 |
| Complete genome (Accession number NC_008277) | hypothetical protein BAPKO_0797 | 7983 64 | 7998 72 | - | 502 | 111115581 | 4227636 |
| Complete genome (Accession number NC_008277) | hypothetical protein BAPKO_0798 | 7998 77 | 8006 11 | - | 244 | 111115582 | 4227637 |
| Complete genome (Accession number NC_008277) | ABC transporter, ATP-binding protein | 8006 08 | 8015 37 | - | 309 | 111115583 | 4227638 |
| Complete genome (Accession number NC_008277) | ribonuclease Z | 8015 46 | 8025 05 | - | 319 | 111115584 | 4227639 |
| Complete genome (Accession number NC_008277) | hypothetical protein BAPKO_0801 | 8026 35 | 8027 39 | + | 34 | 111115585 | 4227640 |
| Complete genome (Accession number NC_008277) | hypothetical protein BAPKO_0802 | 8026 75 | 8027 88 | + | 37 | 111115586 | 4227868 |
| Complete genome (Accession number NC_008277) | hypothetical protein BAPKO_0803 | 8026 96 | 8037 63 | - | 355 | 111115587 | 4227869 |

| Complete genome (Accession number NC_008277) | ATP-dependent Clp protease proteolytic subunit | 8039 62 | 8045 58 | + | 198 | 111115588 | 4227870 |
|---|---|---|---|---|---|---|---|
| Complete genome (Accession number NC_008277) | hypothetical protein BAPKO_0805 | 8045 36 | 8052 64 | + | 242 | 111115589 | 4227871 |
| Complete genome (Accession number NC_008277) | hypothetical protein BAPKO_0807 | 8054 19 | 8062 76 | - | 285 | 111115590 | 4227873 |
| Complete genome (Accession number NC_008277) | hypothetical protein BAPKO_0808 | 8063 16 | 8066 45 | - | 109 | 111115591 | 4227874 |
| Complete genome (Accession number NC_008277) | hypothetical protein BAPKO_0809 | 8067 56 | 8076 43 | + | 295 | 111115592 | 4227875 |
| Complete genome (Accession number NC_008277) | response regulatory protein | 8078 90 | 8092 48 | - | 452 | 111115593 | 4227876 |
| Complete genome (Accession number NC_008277) | hypothetical protein BAPKO_0811 | 8092 51 | 8096 46 | - | 131 | 111115594 | 4227877 |
| Complete genome (Accession number NC_008277) | sensory transduction histidine kinase, putative | 8096 39 | 8104 00 | - | 253 | 111115595 | 4227878 |
| Complete genome (Accession number NC_008277) | hypothetical protein BAPKO_0813 | 8103 97 | 8114 43 | - | 348 | 111115596 | 4227879 |
| Complete genome (Accession number NC_008277) | colicin V production protein, putative | 8114 54 | 8119 42 | - | 162 | 111115597 | 4227880 |
| Complete genome (Accession number NC_008277) | UDP-N-acetylglucosam ine--N-acetylmuramyl-(pentapeptide) pyrophosphoryl -undecapr | 8119 39 | 8130 30 | - | 363 | 111115598 | 4227881 |

| Complete genome (Accession number NC_008277) | pyridoxal kinase | 8132 08 | 8140 02 | - | 264 | 111115599 | 4227882 |
|---|---|---|---|---|---|---|---|
| Complete genome (Accession number NC_008277) | O-sialoglycoprotein endopeptidase | 8139 77 | 8150 17 | - | 346 | 111115600 | 4227883 |
| Complete genome (Accession number NC_008277) | hypothetical protein BAPKO_0818 | 8150 14 | 8158 95 | - | 293 | 111115601 | 4227884 |
| Complete genome (Accession number NC_008277) | RNA polymerase sigma factor | 8159 46 | 8167 46 | - | 266 | 111115602 | 4227885 |
| Complete genome (Accession number NC_008277) | hypothetical protein BAPKO_0820 | 8168 86 | 8172 06 | - | 106 | 111115603 | 4227886 |
| Complete genome (Accession number NC_008277) | flagellar basal body P-ring protein | 8171 96 | 8182 06 | - | 336 | 111115604 | 4227887 |
| Complete genome (Accession number NC_008277) | hypothetical protein BAPKO_0822 | 8183 80 | 8189 43 | - | 187 | 111115605 | 4227888 |
| Complete genome (Accession number NC_008277) | flagellar basal body rod protein FlgG | 8190 14 | 8198 11 | - | 265 | 111115606 | 4227889 |
| Complete genome (Accession number NC_008277) | flagellar hook-basal body complex protein | 8198 24 | 8206 72 | - | 282 | 111115607 | 4227890 |
| Complete genome (Accession number NC_008277) | hypothetical protein BAPKO_0825 | 8209 21 | 8214 81 | + | 186 | 111115608 | 4227891 |
| Complete genome (Accession number NC_008277) | adenine phosphoribosyltransferase | 8215 46 | 8220 76 | + | 176 | 111115609 | 4227892 |

EP 2 254 592 B1

| Complete genome (Accession number NC_008277) | 50S ribosomal protein L21 | 8221 34 | 8224 45 | + | 103 | 111115610 | 4227893 |
|---|---|---|---|---|---|---|---|
| Complete genome (Accession number NC_008277) | hypothetical protein BAPKO_0828 | 8224 51 | 8227 71 | + | 106 | 111115611 | 4227894 |
| Complete genome (Accession number NC_008277) | 50S ribosomal protein L27 | 8227 71 | 8230 16 | + | 81 | 111115612 | 4227314 |
| Complete genome (Accession number NC_008277) | GTPase ObgE | 8230 58 | 8240 44 | + | 328 | 111115613 | 4227315 |
| Complete genome (Accession number NC_008277) | hypothetical protein BAPKO_0831 | 8240 01 | 8240 81 | + | 26 | 111115614 | 4227316 |
| Complete genome (Accession number NC_008277) | hypothetical protein BAPKO_0832 | 8241 27 | 8247 08 | + | 193 | 111115615 | 4227317 |
| Complete genome (Accession number NC_008277) | hypothetical protein BAPKO_0833 | 8247 05 | 8258 89 | + | 394 | 111115616 | 4227318 |
| Complete genome (Accession number NC_008277) | hypothetical protein BAPKO_0834 | 8258 64 | 8262 14 | + | 116 | 111115617 | 4227319 |
| Complete genome (Accession number NC_008277) | hypothetical protein BAPKO_0835 | 8261 68 | 8263 17 | + | 49 | 111115618 | 4227320 |
| Complete genome (Accession number NC_008277) | regulatory protein SpoVG | 8263 18 | 8266 11 | + | 97 | 111115619 | 4227321 |
| Complete genome (Accession number NC_008277) | 50S ribosomal protein L25/general stress protein Ctc | 8267 68 | 8273 16 | + | 182 | 111115620 | 4227323 |
| Complete genome | peptidyl-tRNA hydrolase | 8273 21 | 8278 87 | + | 188 | 111115621 | 4227324 |

| | | | | | | | |
|---|---|---|---|---|---|---|---|
| (Accession number NC_008277) | | | | | | | |
| Complete genome (Accession number NC_008277) | hypothetical protein BAPKO_0840 | 8278 87 | 8292 09 | + | 440 | 111115622 | 4227325 |
| Complete genome (Accession number NC_008277) | cell division protein | 8292 06 | 8311 25 | + | 639 | 111115623 | 4227326 |
| Complete genome (Accession number NC_008277) | hypothetical protein BAPKO_0842 | 8311 31 | 8326 15 | + | 494 | 111115624 | 4227327 |
| Complete genome (Accession number NC_008277) | thymidine kinase | 8327 20 | 8338 23 | + | 367 | 111115625 | 4227328 |
| Complete genome (Accession number NC_008277) | hypothetical protein BAPKO_0844 | 8337 53 | 8338 96 | - | 47 | 111115626 | 4227329 |
| Complete genome (Accession number NC_008277) | hypothetical protein BAPKO_0845 | 8338 20 | 8344 37 | - | 205 | 111115627 | 4227330 |
| Complete genome (Accession number NC_008277) | thymidylate kinase | 8344 63 | 8350 71 | - | 202 | 111115628 | 4227331 |
| Complete genome (Accession number NC_008277) | hypothetical protein BAPKO_0847 | 8351 57 | 8395 60 | + | 1467 | 111115629 | 4227332 |
| Complete genome (Accession number NC_008277) | outer membrane protein | 8395 78 | 8420 43 | + | 821 | 111115630 | 4227822 |
| Complete genome (Accession number NC_008277) | hypothetical protein BAPKO_0849 | 8420 64 | 8425 97 | + | 177 | 111115631 | 4227823 |
| Complete genome (Accession number | DNA mismatch repair protein | 8430 20 | 8456 08 | + | 862 | 111115632 | 4227824 |

| | | | | | | | |
|---|---|---|---|---|---|---|---|
| NC_008277) | | | | | | | |
| Complete genome (Accession number NC_008277) | competence protein F, putative | 8456 10 | 8462 27 | + | 205 | 111115633 | 4227825 |
| Complete genome (Accession number NC_008277) | hypothetical protein BAPKO_0852 | 8463 28 | 8467 65 | + | 145 | 111115634 | 4227826 |
| Complete genome (Accession number NC_008277) | transcription elongation factor NusA | 8467 79 | 8482 27 | + | 482 | 111115635 | 4227827 |
| Complete genome (Accession number NC_008277) | translation initiation factor IF-2 | 8482 30 | 8505 78 | + | 782 | 111115636 | 4227828 |
| Complete genome (Accession number NC_008277) | ribosome-binding factor A | 8506 01 | 8509 63 | + | 120 | 111115637 | 4227016 |
| Complete genome (Accession number NC_008277) | tRNA pseudouridine 55 synthase | 8509 66 | 8518 14 | + | 282 | 111115638 | 4227017 |
| Complete genome (Accession number NC_008277) | 30S ribosomal protein S15 | 8519 90 | 8522 56 | + | 88 | 111115639 | 4227018 |
| Complete genome (Accession number NC_008277) | polynucleotide phosphorylase/ polyadenylase | 8522 71 | 8544 39 | + | 722 | 111115640 | 4227019 |
| Complete genome (Accession number NC_008277) | hypothetical protein BAPKO_0859 | 8544 46 | 8559 87 | + | 513 | 111115641 | 4227020 |
| Complete genome (Accession number NC_008277) | hypothetical protein BAPKO_0860 | 8559 62 | 8572 51 | + | 429 | 111115642 | 4227021 |
| Complete genome (Accession number | hypothetical protein BAPKO_0861 | 8572 48 | 8583 15 | + | 355 | 111115643 | 4227022 |

| | | | | | | | |
|---|---|---|---|---|---|---|---|
| NC_008277) | | | | | | | |
| Complete genome (Accession number NC_008277) | tRNA-guanine transglycosylase | 8583 47 | 8594 74 | + | 375 | 111115644 | 4227023 |
| Complete genome (Accession number NC_008277) | virulence factor mviN protein | 8594 67 | 8609 87 | + | 506 | 111115645 | 4227024 |
| Complete genome (Accession number NC_008277) | hypothetical protein BAPKO_0864 | 8609 84 | 8623 90 | + | 468 | 111115646 | 4227025 |
| Complete genome (Accession number NC_008277) | pantothenate metabolism flavoprotein | 8624 15 | 8634 76 | - | 353 | 111115647 | 4227026 |
| Complete genome (Accession number NC_008277) | hypothetical protein BAPKO_0866 | 8637 30 | 8638 34 | - | 34 | 111115648 | 4227027 |
| Complete genome (Accession number NC_008277) | sodium/panthothenate symporter | 8639 16 | 8652 50 | + | 444 | 111115649 | 4227144 |
| Complete genome (Accession number NC_008277) | hypothetical protein BAPKO_0868 | 8652 63 | 8662 10 | + | 315 | 111115650 | 4227145 |
| Complete genome (Accession number NC_008277) | hypothetical protein BAPKO_0869 | 8661 79 | 8671 26 | + | 315 | 111115651 | 4227146 |
| Complete genome (Accession number NC_008277) | UDP-N-acetylmuramate--L-alanine ligase | 8671 16 | 8685 22 | + | 468 | 111115652 | 4227147 |
| Complete genome (Accession number NC_008277) | hypothetical protein BAPKO_0871 | 8685 33 | 8693 99 | + | 288 | 111115653 | 4227148 |
| Complete genome (Accession number | cytidylate kinase | 8693 96 | 8699 32 | + | 178 | 111115654 | 4227149 |

| | | | | | | | |
|---|---|---|---|---|---|---|---|
| NC_008277) | | | | | | | |
| Complete genome (Accession number NC_008277) | hypothetical protein BAPKO_0873 | 8699 72 | 8701 72 | + | 66 | 111115655 | 4227150 |
| Complete genome (Accession number NC_008277) | 2-methylthio-N6-isopentyladeno sine tRNA modification enzyme | 8701 59 | 8710 61 | + | 300 | 111115656 | 4227151 |
| Complete genome (Accession number NC_008277) | hypothetical protein BAPKO_0875 | 8710 83 | 8711 78 | + | 31 | 111115657 | 4227152 |
| Complete genome (Accession number NC_008277) | hypothetical protein BAPKO_0876 | 8712 22 | 8715 93 | - | 123 | 111115658 | 4227153 |
| Complete genome (Accession number NC_008277) | hypothetical protein BAPKO_0877 | 8716 74 | 8722 19 | + | 181 | 111115659 | 4227154 |
| Complete genome (Accession number NC_008277) | hypothetical protein BAPKO_0878 | 8722 12 | 8729 94 | - | 260 | 111115660 | 4227155 |
| Complete genome (Accession number NC_008277) | hypothetical protein BAPKO_0879 | 8729 87 | 8735 02 | - | 171 | 111115661 | 4227156 |
| Complete genome (Accession number NC_008277) | ATP-dependent helicase | 8736 29 | 8761 00 | - | 823 | 111115662 | 4227157 |
| Complete genome (Accession number NC_008277) | DNA topoisomerase I | 8761 03 | 8786 52 | - | 849 | 111115663 | 4227158 |
| Complete genome (Accession number NC_008277) | exonuclease SbcD | 8787 28 | 8799 69 | + | 413 | 111115664 | 4227159 |
| Complete genome (Accession number | exonuclease SbcC | 8799 56 | 8827 96 | + | 946 | 111115665 | 4227160 |

| | | | | | | | |
|---|---|---|---|---|---|---|---|
| NC_008277) | | | | | | | |
| Complete genome (Accession number NC_008277) | xylose operon regulatory protein | 8828 30 | 8837 68 | + | 312 | 111115666 | 4227161 |
| Complete genome (Accession number NC_008277) | lipoprotein, putative | 8837 82 | 8846 21 | - | 279 | 111115667 | 4227162 |
| Complete genome (Accession number NC_008277) | isoleucyl-tRNA synthetase | 8846 15 | 8877 43 | - | 1042 | 111115668 | 4227163 |
| Complete genome (Accession number NC_008277) | ATP-dependent Clp protease, subunit C | 8877 64 | 8899 11 | - | 715 | 111115669 | 4227543 |
| Complete genome (Accession number NC_008277) | phosphomanno mutase | 8900 06 | 8917 15 | - | 569 | 111115670 | 4227544 |
| Complete genome (Accession number NC_008277) | excinuclease ABC subunit B | 8918 58 | 8938 52 | + | 664 | 111115671 | 4227545 |
| Complete genome (Accession number NC_008277) | excinuclease ABC, subunit A | 8938 69 | 8967 21 | + | 950 | 111115672 | 4227546 |
| Complete genome (Accession number NC_008277) | hypothetical protein BAPKO_0891 | 8967 24 | 9001 25 | + | 1133 | 111115673 | 4227547 |
| Complete genome (Accession number NC_008277) | hypothetical protein BAPKO_0892 | 9000 91 | 9004 77 | + | 128 | 111115674 | 4227548 |
| Complete genome (Accession number NC_008277) | hypothetical protein BAPKO_0893 | 9001 35 | 9006 74 | - | 179 | 111115675 | 4227549 |
| Complete genome (Accession number NC_008277) | hypothetical protein BAPKO_0894 | 9006 52 | 9012 51 | - | 199 | 111115676 | 4227550 |

| | | | | | | | |
|---|---|---|---|---|---|---|---|
| Complete genome (Accession number NC_008277) | lipoprotein, putative | 9012 61 | 9028 47 | - | 528 | 111115677 | 4227551 |
| Complete genome (Accession number NC_008277) | arginine deiminase | 9030 57 | 9042 86 | + | 409 | 111115678 | 4227552 |
| Complete genome (Accession number NC_008277) | ornithine carbamoyltrans ferase, catabolic | 9043 51 | 9053 37 | + | 328 | 111115679 | 4227553 |

# Fig. 29. Borrelia garinii

| Plasmid or genome | Product Name | Start | End | Strand | Length | Gi | GeneID |
|---|---|---|---|---|---|---|---|
| Plasmid cp26 (Accession number NC_006128) | hypothetical protein BGB01 | 45 | 326 | + | 93 | 51038598 | 2942824 |
| Plasmid cp26 (Accession number NC_006128) | hypothetical protein BGB02 | 310 | 756 | - | 148 | 51038599 | 2942818 |
| Plasmid cp26 (Accession number NC_006128) | hypothetical protein BGB03 | 844 | 2193 | - | 449 | 51038600 | 2942810 |
| Plasmid cp26 (Accession number NC_006128) | PTS system, cellobiose-specific IIC component | 2471 | 3802 | - | 443 | 51038601 | 2942815 |
| Plasmid cp26 (Accession number NC_006128) | PTS system, cellobiose-specific IIA | 3993 | 4340 | + | 115 | 51038602 | 2942820 |
| Plasmid cp26 (Accession number NC_006128) | PTS system, cellobiose-specific IIB component | 4349 | 4666 | + | 105 | 51038603 | 2942809 |
| Plasmid cp26 (Accession number NC_006128) | outer surface protein, putative | 4678 | 5775 | + | 365 | 51038604 | 2942806 |
| Plasmid cp26 (Accession number NC_006128) | hypothetical protein BGB08 | 5796 | 6428 | - | 210 | 51038605 | 2942805 |
| Plasmid cp26 (Accession number NC_006128) | hypothetical protein BGB09 | 6570 | 7613 | + | 347 | 51038606 | 2942823 |

| | | | | | | | |
|---|---|---|---|---|---|---|---|
| Plasmid cp26 (Accession number NC_006128) | hypothetical protein BGB10 | 7729 | 8673 | + | 314 | 51038607 | 2942821 |
| Plasmid cp26 (Accession number NC_006128) | hypothetical protein BGB11 | 8663 | 9181 | + | 172 | 51038608 | 2942826 |
| Plasmid cp26 (Accession number NC_006128) | hypothetical protein BGB12 | 9157 | 9918 | + | 253 | 51038609 | 2942807 |
| Plasmid cp26 (Accession number NC_006128) | hypothetical protein BGB13 | 9986 | 1053 4 | + | 182 | 51038610 | 2942822 |
| Plasmid cp26 (Accession number NC_006128) | hypothetical protein BGB14 | 1080 4 | 1131 9 | - | 171 | 51038611 | 2942814 |
| Plasmid cp26 (Accession number NC_006128) | oligopeptide ABC transporter, periplasmic oligopeptide- binding protein | 1254 5 | 1414 6 | + | 533 | 51038612 | 2942812 |
| Plasmid cp26 (Accession number NC_006128) | inositol-5- monophosphate dehydrogenase | 1448 2 | 1569 6 | - | 404 | 51038613 | 2942827 |
| Plasmid cp26 (Accession number NC_006128) | bifunctional GMP synthase/glutamine amidotransferase protein | 1572 1 | 1730 7 | - | 528 | 51038614 | 2942828 |
| Plasmid cp26 (Accession number NC_006128) | outer surface protein C | 1753 1 | 1815 4 | + | 207 | 51038615 | 2942825 |
| Plasmid cp26 (Accession number NC_006128) | hypothetical protein BGB19 | 1859 6 | 1995 1 | - | 451 | 51038616 | 2942829 |

| Plasmid cp26 (Accession number NC_006128) | hypothetical protein BGB20 | 2006 1 | 2141 6 | - | 451 | 51038617 | 2942830 |
|---|---|---|---|---|---|---|---|
| Plasmid cp26 (Accession number NC_006128) | hypothetical protein BGB21 | 2148 4 | 2199 0 | - | 168 | 51038618 | 2942817 |
| Plasmid cp26 (Accession number NC_006128) | hypothetical protein BGB22 | 2196 5 | 2245 0 | - | 161 | 51038619 | 2942819 |
| Plasmid cp26 (Accession number NC_006128) | hypothetical protein BGB23 | 2252 4 | 2321 9 | + | 231 | 51038620 | 2942813 |
| Plasmid cp26 (Accession number NC_006128) | hypothetical protein BGB24 | 2322 9 | 2378 0 | - | 183 | 51038621 | 2942816 |
| Plasmid cp26 (Accession number NC_006128) | hypothetical protein BGB25 | 2409 6 | 2513 6 | + | 346 | 51038622 | 2942811 |
| Plasmid cp26 (Accession number NC_006128) | PTS system, maltose and glucose-specific IIABC component | 2543 7 | 2706 5 | + | 542 | 51038623 | 2942808 |
| Plasmid lp54 (Accession number NC_006129) | hypothetical protein BGA01 | 1024 | 1509 | + | 161 | 51038625 | 2942865 |
| Plasmid lp54 (Accession number NC_006129) | outer membrane protein | 1825 | 2343 | + | 172 | 51038626 | 2942881 |
| Plasmid lp54 (Accession number NC_006129) | antigen, S2 | 2434 | 3255 | - | 273 | 51038627 | 2942890 |
| Plasmid lp54 (Accession number NC_006129) | antigen, S1 | 3380 | 4573 | - | 397 | 51038628 | 2942835 |
| Plasmid lp54 (Accession number | chpAI protein, putative | 4982 | 5455 | - | 157 | 51038629 | 2942880 |

| | | | | | | | |
|---|---|---|---|---|---|---|---|
| NC_006129) | | | | | | | |
| Plasmid lp54 (Accession number NC_006129) | hypothetical protein BGA06 | 5599 | 5961 | + | 120 | 51038630 | 2942838 |
| Plasmid lp54 (Accession number NC_006129) | hypothetical protein BGA07 | 6163 | 6351 | + | 62 | 51038631 | 2942867 |
| Plasmid lp54 (Accession number NC_006129) | hypothetical protein BGA08 | 6606 | 6812 | + | 68 | 51038632 | 2942892 |
| Plasmid lp54 (Accession number NC_006129) | hypothetical protein BGA09 | 6825 | 7430 | + | 201 | 51038633 | 2942894 |
| Plasmid lp54 (Accession number NC_006129) | hypothetical protein BGA10 | 7885 | 8547 | + | 220 | 51038634 | 2942871 |
| Plasmid lp54 (Accession number NC_006129) | hypothetical protein BGA11 | 8640 | 8750 | + | 36 | 51038635 | 2942802 |
| Plasmid lp54 (Accession number NC_006129) | hypothetical protein BGA12 | 8747 | 9106 | + | 119 | 51038636 | 2942874 |
| Plasmid lp54 (Accession number NC_006129) | hypothetical protein BGA13 | 9302 | 9649 | + | 115 | 51038637 | 2942833 |
| Plasmid lp54 (Accession number NC_006129) | outer surface protein A | 9700 | 10521 | + | 273 | 51038638 | 2942836 |
| Plasmid lp54 (Accession number NC_006129) | outer surface protein B | 10531 | 11415 | + | 294 | 51038639 | 2942852 |
| Plasmid lp54 (Accession number NC_006129) | hypothetical protein BGA16 | 11967 | 13166 | + | 399 | 51038640 | 2942854 |
| Plasmid lp54 (Accession number NC_006129) | hypothetical protein BGA17 | 13172 | 13795 | + | 207 | 51038641 | 2942853 |

| | | | | | | | |
|---|---|---|---|---|---|---|---|
| Plasmid lp54 (Accession number NC_006129) | hypothetical protein BGA18 | 13771 | 14523 | + | 250 | 51038642 | 2942834 |
| Plasmid lp54 (Accession number NC_006129) | hypothetical protein BGA19 | 14524 | 15096 | + | 190 | 51038643 | 2942855 |
| Plasmid lp54 (Accession number NC_006129) | hypothetical protein BGA20 | 15991 | 16314 | + | 107 | 51038644 | 2942841 |
| Plasmid lp54 (Accession number NC_006129) | decorin binding protein A | 16429 | 16956 | - | 175 | 51038645 | 2942886 |
| Plasmid lp54 (Accession number NC_006129) | decorin binding protein B | 17077 | 17631 | - | 184 | 51038646 | 2942878 |
| Plasmid lp54 (Accession number NC_006129) | hypothetical protein BGA23 | 18024 | 18335 | - | 103 | 51038647 | 2942885 |
| Plasmid lp54 (Accession number NC_006129) | hypothetical protein BGA24 | 19210 | 19773 | + | 187 | 51038648 | 2942831 |
| Plasmid lp54 (Accession number NC_006129) | hypothetical protein BGA25 | 19900 | 20406 | + | 168 | 51038649 | 2942803 |
| Plasmid lp54 (Accession number NC_006129) | hypothetical protein BGA26 | 20749 | 21120 | + | 123 | 51038650 | 2942839 |
| Plasmid lp54 (Accession number NC_006129) | hypothetical protein BGA27 | 21685 | 22152 | + | 155 | 51038651 | 2942897 |
| Plasmid lp54 (Accession number NC_006129) | oligopeptide ABC transporter, periplasmic | 22316 | 23902 | - | 528 | 51038652 | 2942887 |
| Plasmid lp54 (Accession number NC_006129) | hypothetical protein BGA29 | 23865 | 24053 | + | 62 | 51038653 | 2942844 |

| | | | | | | | |
|---|---|---|---|---|---|---|---|
| Plasmid lp54 (Accession number NC_006129) | lipoprotein | 2437 7 | 2506 0 | + | 227 | 51038654 | 2942896 |
| Plasmid lp54 (Accession number NC_006129) | hypothetical protein BGA31 | 2518 1 | 2578 9 | + | 202 | 51038655 | 2942895 |
| Plasmid lp54 (Accession number NC_006129) | hypothetical protein BGA32 | 2638 2 | 2654 0 | + | 52 | 51038656 | 2942891 |
| Plasmid lp54 (Accession number NC_006129) | hypothetical protein BGA33 | 2671 8 | 2738 9 | + | 223 | 51038657 | 2942866 |
| Plasmid lp54 (Accession number NC_006129) | hypothetical protein BGA34 | 2740 1 | 2792 8 | + | 175 | 51038658 | 2942889 |
| Plasmid lp54 (Accession number NC_006129) | hypothetical protein BGA35 | 2805 7 | 2818 8 | + | 43 | 51038659 | 2942883 |
| Plasmid lp54 (Accession number NC_006129) | hypothetical protein BGA36 | 2818 8 | 2852 0 | + | 110 | 51038660 | 2942868 |
| Plasmid lp54 (Accession number NC_006129) | hypothetical protein BGA37 | 2855 1 | 2949 5 | + | 314 | 51038661 | 2942856 |
| Plasmid lp54 (Accession number NC_006129) | hypothetical protein BGA38 | 2949 9 | 2984 0 | + | 113 | 51038662 | 2942857 |
| Plasmid lp54 (Accession number NC_006129) | hypothetical protein BGA39 | 2995 6 | 3033 0 | + | 124 | 51038663 | 2942872 |
| Plasmid lp54 (Accession number NC_006129) | hypothetical protein BGA40 | 3041 6 | 3070 0 | + | 94 | 51038664 | 2942875 |
| Plasmid lp54 (Accession number NC_006129) | hypothetical protein BGA41 | 3068 1 | 3085 1 | + | 56 | 51038665 | 2942832 |

| Plasmid lp54 (Accession number NC_006129) | hypothetical protein BGA42 | 3100 4 | 3122 8 | + | 74 | 51038666 | 2942899 |
|---|---|---|---|---|---|---|---|
| Plasmid lp54 (Accession number NC_006129) | hypothetical protein BGA43 | 3126 4 | 3149 4 | + | 76 | 51038667 | 2942876 |
| Plasmid lp54 (Accession number NC_006129) | hypothetical protein BGA44 | 3150 1 | 3197 1 | + | 156 | 51038668 | 2942860 |
| Plasmid lp54 (Accession number NC_006129) | hypothetical protein BGA45 | 3233 4 | 3275 6 | + | 140 | 51038669 | 2942870 |
| Plasmid lp54 (Accession number NC_006129) | hypothetical protein BGA46 | 3279 9 | 3329 0 | + | 163 | 51038670 | 2942862 |
| Plasmid lp54 (Accession number NC_006129) | hypothetical protein BGA47 | 3351 5 | 3408 1 | + | 188 | 51038671 | 2942848 |
| Plasmid lp54 (Accession number NC_006129) | hypothetical protein BGA48 | 3410 3 | 3483 4 | + | 243 | 51038672 | 2942849 |
| Plasmid lp54 (Accession number NC_006129) | hypothetical protein BGA49 | 3492 3 | 3507 8 | + | 51 | 51038673 | 2942884 |
| Plasmid lp54 (Accession number NC_006129) | hypothetical protein BGA50 | 3526 3 | 3548 7 | + | 74 | 51038674 | 2942847 |
| Plasmid lp54 (Accession number NC_006129) | outer membrane protein | 3552 6 | 3629 3 | + | 255 | 51038675 | 2942843 |
| Plasmid lp54 (Accession number NC_006129) | hypothetical protein BGA52 | 3643 8 | 3666 8 | + | 76 | 51038676 | 2942846 |
| Plasmid lp54 (Accession number NC_006129) | hypothetical protein BGA53 | 3670 7 | 3697 0 | + | 87 | 51038677 | 2942804 |

| | | | | | | | |
|---|---|---|---|---|---|---|---|
| Plasmid lp54 (Accession number NC_006129) | hypothetical protein BGA54 | 3704 8 | 3725 1 | + | 67 | 51038678 | 2942893 |
| Plasmid lp54 (Accession number NC_006129) | hypothetical protein BGA55 | 3725 4 | 3801 5 | + | 253 | 51038679 | 2942901 |
| Plasmid lp54 (Accession number NC_006129) | hypothetical protein BGA56 | 3799 7 | 3834 4 | + | 115 | 51038680 | 2942879 |
| Plasmid lp54 (Accession number NC_006129) | hypothetical protein BGA57 | 3839 2 | 3854 7 | + | 51 | 51038681 | 2942873 |
| Plasmid lp54 (Accession number NC_006129) | hypothetical protein BGA58 | 3860 0 | 3973 9 | - | 379 | 51038682 | 2942851 |
| Plasmid lp54 (Accession number NC_006129) | lipoprotein | 4029 8 | 4051 0 | - | 70 | 51038683 | 2942877 |
| Plasmid lp54 (Accession number NC_006129) | surface lipoprotein P27 | 4061 0 | 4144 3 | - | 277 | 51038684 | 2942842 |
| Plasmid lp54 (Accession number NC_006129) | hypothetical protein BGA61 | 4179 8 | 4242 1 | - | 207 | 51038685 | 2942869 |
| Plasmid lp54 (Accession number NC_006129) | lipoprotein | 4268 9 | 4284 7 | + | 52 | 51038686 | 2942863 |
| Plasmid lp54 (Accession number NC_006129) | antigen, P35 | 4300 7 | 4396 3 | - | 318 | 51038687 | 2942861 |
| Plasmid lp54 (Accession number NC_006129) | hypothetical protein BGA64 | 4413 0 | 4496 0 | - | 276 | 51038688 | 2942859 |
| Plasmid lp54 (Accession number NC_006129) | antigen, P35, putative | 4512 9 | 4640 3 | - | 424 | 51038689 | 2942900 |
| Plasmid lp54 (Accession number NC_006129) | hypothetical protein BGA66 | 4674 7 | 4749 9 | - | 250 | 51038690 | 2942882 |

| | | | | | | | |
|---|---|---|---|---|---|---|---|
| Plasmid lp54 (Accession number NC_006129) | hypothetical protein BGA67 | 4782 4 | 4860 6 | - | 260 | 51038691 | 2942898 |
| Plasmid lp54 (Accession number NC_006129) | hypothetical protein BGA68 | 4883 9 | 4954 6 | - | 235 | 51038692 | 2942845 |
| Plasmid lp54 (Accession number NC_006129) | hypothetical protein BGA69 | 4987 2 | 5007 5 | - | 67 | 51038693 | 2942837 |
| Plasmid lp54 (Accession number NC_006129) | hypothetical protein BGA70 | 5020 5 | 5057 3 | - | 122 | 51038694 | 2942850 |
| Plasmid lp54 (Accession number NC_006129) | hypothetical protein BGA71 | 5084 5 | 5160 0 | - | 251 | 51038695 | 2942864 |
| Plasmid lp54 (Accession number NC_006129) | hypothetical protein BGA72 | 5191 1 | 5268 4 | - | 257 | 51038696 | 2942858 |
| Plasmid lp54 (Accession number NC_006129) | antigen, P35, putative | 5283 5 | 5369 2 | - | 285 | 51038697 | 2942888 |
| Plasmid lp54 (Accession number NC_006129) | outer membrane porin | 5423 0 | 5500 3 | + | 257 | 51038698 | 2942840 |
| Complete genome (Accession number NC_006156) | hypothetical protein BG0001 | 443 | 526 | - | 27 | 51598264 | 2957747 |
| Complete genome (Accession number NC_006156) | beta-N-acetylhexosaminida se, putative | 629 | 1648 | - | 339 | 51598265 | 2957836 |
| Complete genome (Accession number NC_006156) | hypothetical protein BG0003 | 1636 | 3114 | - | 492 | 51598266 | 2957388 |
| Complete genome (Accession number NC_006156) | phosphoglucomuta se | 3254 | 5041 | + | 595 | 51598267 | 2957341 |

| | | | | | | | |
|---|---|---|---|---|---|---|---|
| Complete genome (Accession number NC_006156) | tryptophanyl-tRNA synthetase | 5126 | 6163 | - | 345 | 51598268 | 2957736 |
| Complete genome (Accession number NC_006156) | hypothetical protein BG0006 | 6166 | 7254 | - | 362 | 51598269 | 2957926 |
| Complete genome (Accession number NC_006156) | hypothetical protein BG0007 | 7308 | 8168 | - | 286 | 51598270 | 2957823 |
| Complete genome (Accession number NC_006156) | hypothetical protein BG0008 | 8277 | 9047 | + | 256 | 51598271 | 2957719 |
| Complete genome (Accession number NC_006156) | hypothetical protein BG0009 | 9052 | 1005 6 | + | 334 | 51598272 | 2957763 |
| Complete genome (Accession number NC_006156) | holo-acyl-carrier protein synthase, putative | 1005 3 | 1042 7 | + | 124 | 51598273 | 2957930 |
| Complete genome (Accession number NC_006156) | hypothetical protein BG0011 | 1043 1 | 1127 0 | + | 279 | 51598274 | 2958006 |
| Complete genome (Accession number NC_006156) | tRNA pseudouridine synthase A | 1127 1 | 1201 1 | + | 246 | 51598275 | 2957468 |
| Complete genome (Accession number NC_006156) | hypothetical protein BG0013 | 1200 4 | 1259 4 | + | 196 | 51598276 | 2957813 |
| Complete genome (Accession number NC_006156) | primosomal protein N | 1258 7 | 1456 9 | + | 660 | 51598277 | 2957928 |
| Complete genome (Accession number NC_006156) | uridine kinase | 1456 6 | 1518 6 | - | 206 | 51598278 | 2957927 |

| Complete genome (Accession number NC_006156) | glpE protein | 1518 6 | 1546 7 | - | 93 | 51598279 | 2957998 |
|---|---|---|---|---|---|---|---|
| Complete genome (Accession number NC_006156) | hypothetical protein BG0017 | 1569 0 | 1665 5 | + | 321 | 51598280 | 2957916 |
| Complete genome (Accession number NC_006156) | hypothetical protein BG0018 | 1665 2 | 1763 5 | - | 327 | 51598281 | 2957605 |
| Complete genome (Accession number NC_006156) | hypothetical protein BG0019 | 1763 7 | 1814 9 | - | 170 | 51598282 | 2957659 |
| Complete genome (Accession number NC_006156) | diphosphate-- fructose-6- phosphate 1- phosphotransferas e | 1815 7 | 1982 7 | - | 556 | 51598283 | 2957509 |
| Complete genome (Accession number NC_006156) | S- adenosylmethionin e: tRNA ribosyltransferase- isomerase | 1990 5 | 2094 5 | - | 346 | 51598284 | 2957477 |
| Complete genome (Accession number NC_006156) | Holliday junction DNA helicase B | 2092 0 | 2196 3 | - | 347 | 51598285 | 2957993 |
| Complete genome (Accession number NC_006156) | Holliday junction DNA helicase | 2200 8 | 2259 8 | - | 196 | 51598286 | 2957957 |
| Complete genome (Accession number NC_006156) | hypothetical protein BG0024 | 2266 8 | 2380 7 | + | 379 | 51598287 | 2957981 |
| Complete genome (Accession number | hypothetical protein BG0025 | 2382 2 | 2455 3 | - | 243 | 51598288 | 2957984 |

| NC_006156) | | | | | | | |
|---|---|---|---|---|---|---|---|
| Complete genome (Accession number NC_006156) | methylenetetrahydr ofolate dehydrogenase | 2455 4 | 2545 9 | - | 301 | 51598289 | 2957612 |
| Complete genome (Accession number NC_006156) | hypothetical protein BG0027 | 2561 0 | 2624 8 | + | 212 | 51598290 | 2957660 |
| Complete genome (Accession number NC_006156) | lipoprotein, putative | 2625 6 | 2730 5 | + | 349 | 51598291 | 2957613 |
| Complete genome (Accession number NC_006156) | hypothetical protein BG0029 | 2729 1 | 2772 2 | - | 143 | 51598292 | 2957982 |
| Complete genome (Accession number NC_006156) | signal peptidase I | 2771 2 | 2834 7 | - | 211 | 51598293 | 2957611 |
| Complete genome (Accession number NC_006156) | signal peptidase I | 2834 9 | 2932 9 | - | 326 | 51598294 | 2957616 |
| Complete genome (Accession number NC_006156) | hypothetical protein BG0032 | 2940 0 | 3086 3 | + | 487 | 51598295 | 2957950 |
| Complete genome (Accession number NC_006156) | SsrA-binding protein | 3087 5 | 3132 7 | + | 150 | 51598296 | 2957961 |
| Complete genome (Accession number NC_006156) | hypothetical protein BG0034 | 3140 1 | 3193 4 | - | 177 | 51598297 | 2957962 |
| Complete genome (Accession number NC_006156) | DNA topoisomerase IV subunit A | 3200 2 | 3388 2 | - | 626 | 51598298 | 2957933 |

| | | | | | | | |
|---|---|---|---|---|---|---|---|
| Complete genome (Accession number NC_006156) | DNA topoisomerase IV subunit B | 3388 2 | 3568 1 | - | 599 | 51598299 | 2957619 |
| Complete genome (Accession number NC_006156) | 1-acyl-sn-glycerol-3-phosphate acyltransferase | 3570 2 | 3643 9 | - | 245 | 51598300 | 2957945 |
| Complete genome (Accession number NC_006156) | hypothetical protein BG0038 | 3644 8 | 3798 6 | - | 512 | 51598301 | 2957952 |
| Complete genome (Accession number NC_006156) | hypothetical protein BG0039 | 3799 8 | 3950 0 | - | 500 | 51598302 | 2957938 |
| Complete genome (Accession number NC_006156) | chemotaxis protein methyltransferase | 3969 2 | 4054 3 | + | 283 | 51598303 | 2957656 |
| Complete genome (Accession number NC_006156) | phosphate transport system regulatory protein | 4062 6 | 4129 7 | - | 223 | 51598304 | 2957623 |
| Complete genome (Accession number NC_006156) | hypothetical protein BG0042 | 4130 8 | 4234 2 | - | 344 | 51598305 | 2957939 |
| Complete genome (Accession number NC_006156) | hypothetical protein BG0043 | 4233 9 | 4274 0 | - | 133 | 51598306 | 2957624 |
| Complete genome (Accession number NC_006156) | P115 protein | 4276 6 | 4521 3 | - | 815 | 51598307 | 2957969 |
| Complete genome (Accession number NC_006156) | ribonuclease H | 4530 6 | 4585 1 | + | 181 | 51598308 | 2957615 |

| | | | | | | | |
|---|---|---|---|---|---|---|---|
| Complete genome (Accession number NC_006156) | hypothetical protein BG0046 | 4586 6 | 4622 8 | - | 120 | 51598309 | 2957992 |
| Complete genome (Accession number NC_006156) | hypothetical protein BG0048 | 4676 5 | 4688 4 | - | 39 | 51598310 | 2957882 |
| Complete genome (Accession number NC_006156) | hypothetical protein BG0049 | 4700 7 | 4768 1 | + | 224 | 51598311 | 2957860 |
| Complete genome (Accession number NC_006156) | hypothetical protein BG0050 | 4775 8 | 4844 7 | + | 229 | 51598312 | 2957631 |
| Complete genome (Accession number NC_006156) | spoU protein | 4844 4 | 4910 0 | - | 218 | 51598313 | 2957632 |
| Complete genome (Accession number NC_006156) | uracil-DNA glycosylase | 4918 3 | 4985 4 | + | 223 | 51598314 | 2957874 |
| Complete genome (Accession number NC_006156) | preprotein translocase subunit SecG | 4995 2 | 5031 4 | - | 120 | 51598315 | 2957870 |
| Complete genome (Accession number NC_006156) | triosephosphate isomerase | 5033 0 | 5109 1 | - | 253 | 51598316 | 2957633 |
| Complete genome (Accession number NC_006156) | phosphoglycerate kinase | 5109 3 | 5227 4 | - | 393 | 51598317 | 2957894 |
| Complete genome (Accession number NC_006156) | glyceraldehyde 3-phosphate dehydrogenase | 5230 0 | 5330 7 | - | 335 | 51598318 | 2957866 |
| Complete genome (Accession number | hypothetical protein BG0057 | 5338 2 | 5535 2 | - | 656 | 51598319 | 2957635 |

| | | | | | | | |
|---|---|---|---|---|---|---|---|
| NC_006156) | | | | | | | |
| Complete genome (Accession number NC_006156) | hemolysin | 5534 9 | 5612 8 | - | 259 | 51598320 | 2957634 |
| Complete genome (Accession number NC_006156) | hypothetical protein BG0059 | 5612 9 | 5658 7 | - | 152 | 51598321 | 2957642 |
| Complete genome (Accession number NC_006156) | thioredoxin | 5667 6 | 5702 9 | - | 117 | 51598322 | 2957639 |
| Complete genome (Accession number NC_006156) | hypothetical protein BG0061 | 5710 3 | 5781 9 | + | 238 | 51598323 | 2957637 |
| Complete genome (Accession number NC_006156) | hypothetical protein BG0062 | 5787 1 | 5881 5 | - | 314 | 51598324 | 2957640 |
| Complete genome (Accession number NC_006156) | methionyl-tRNA formyltransferase | 5892 8 | 5987 5 | - | 315 | 51598325 | 2957645 |
| Complete genome (Accession number NC_006156) | polypeptide deformylase | 5987 2 | 6043 2 | - | 186 | 51598326 | 2957647 |
| Complete genome (Accession number NC_006156) | hypothetical protein BG0065 | 6040 5 | 6109 1 | - | 228 | 51598327 | 2957646 |
| Complete genome (Accession number NC_006156) | peptidase, putative | 6108 8 | 6286 6 | - | 592 | 51598328 | 2957644 |
| Complete genome (Accession number NC_006156) | hypothetical protein BG0067 | 6297 9 | 6386 9 | + | 296 | 51598329 | 2957641 |
| Complete genome | aminopeptidase II | 6387 0 | 6510 8 | + | 412 | 51598330 | 2957650 |

| | | | | | | | |
|---|---|---|---|---|---|---|---|
| (Accession number NC_006156) | | | | | | | |
| Complete genome (Accession number NC_006156) | hypothetical protein BG0069 | 6512 3 | 6558 4 | + | 153 | 51598331 | 2957684 |
| Complete genome (Accession number NC_006156) | hypothetical protein BG0070 | 6560 1 | 6707 3 | + | 490 | 51598332 | 2957655 |
| Complete genome (Accession number NC_006156) | hypothetical protein BG0071 | 6708 1 | 6939 9 | + | 772 | 51598333 | 2957685 |
| Complete genome (Accession number NC_006156) | hypothetical protein BG0072 | 6939 6 | 6995 6 | + | 186 | 51598334 | 2957667 |
| Complete genome (Accession number NC_006156) | peptide chain release factor 2 | 6999 7 | 7104 9 | + | 350 | 51598335 | 2957657 |
| Complete genome (Accession number NC_006156) | hypothetical protein BG0074 | 7103 1 | 7244 0 | + | 469 | 51598336 | 2957675 |
| Complete genome (Accession number NC_006156) | signal recognition particle-docking protein FtsY | 7247 7 | 7332 2 | + | 281 | 51598337 | 2957669 |
| Complete genome (Accession number NC_006156) | hypothetical protein BG0076 | 7331 9 | 7434 7 | + | 342 | 51598338 | 2957700 |
| Complete genome (Accession number NC_006156) | hypothetical protein BG0077 | 7434 8 | 7560 1 | + | 417 | 51598339 | 2957676 |
| Complete genome (Accession number NC_006156) | ABC transporter, ATP-binding protein | 7560 3 | 7628 3 | + | 226 | 51598340 | 2957668 |

| | | | | | | | |
|---|---|---|---|---|---|---|---|
| Complete genome (Accession number NC_006156) | hypothetical protein BG0079 | 7628 0 | 7753 3 | + | 417 | 51598341 | 2957671 |
| Complete genome (Accession number NC_006156) | hypothetical protein BG0080 | 7754 5 | 7884 3 | - | 432 | 51598342 | 2957670 |
| Complete genome (Accession number NC_006156) | hypothetical protein BG0081 | 7883 6 | 7915 6 | - | 106 | 51598343 | 2957673 |
| Complete genome (Accession number NC_006156) | nifS protein | 7933 9 | 8060 7 | + | 422 | 51598344 | 2957672 |
| Complete genome (Accession number NC_006156) | hypothetical protein BG0083 | 8053 8 | 8069 0 | - | 50 | 51598345 | 2957677 |
| Complete genome (Accession number NC_006156) | hypothetical protein BG0084 | 8081 1 | 8123 9 | + | 142 | 51598346 | 2957674 |
| Complete genome (Accession number NC_006156) | hypothetical protein BG0085 | 8123 2 | 8149 2 | - | 86 | 51598347 | 2957678 |
| Complete genome (Accession number NC_006156) | hypothetical protein BG0086 | 8155 1 | 8234 2 | + | 263 | 51598348 | 2957680 |
| Complete genome (Accession number NC_006156) | hypothetical protein BG0087 | 8237 9 | 8277 1 | + | 130 | 51598349 | 2957679 |
| Complete genome (Accession number NC_006156) | L-lactate dehydrogenase | 8280 1 | 8375 1 | - | 316 | 51598350 | 2957681 |
| Complete genome (Accession number NC_006156) | GTP-binding protein LepA | 8387 4 | 8567 9 | - | 601 | 51598351 | 2957827 |

| Complete genome (Accession number NC_006156) | hypothetical protein BG0090 | 8571 3 | 8669 9 | - | 328 | 51598352 | 2957845 |
|---|---|---|---|---|---|---|---|
| Complete genome (Accession number NC_006156) | V-type ATP synthase subunit K | 8675 9 | 8719 3 | - | 144 | 51598353 | 2957887 |
| Complete genome (Accession number NC_006156) | V-type ATP synthase subunit I | 8721 0 | 8903 6 | - | 608 | 51598354 | 2957465 |
| Complete genome (Accession number NC_006156) | V-type ATP synthase subunit D | 8904 7 | 8964 3 | - | 198 | 51598355 | 2957466 |
| Complete genome (Accession number NC_006156) | V-type ATP synthase subunit B | 8964 6 | 9095 0 | - | 434 | 51598356 | 2957463 |
| Complete genome (Accession number NC_006156) | V-type ATP synthase subunit A | 9097 2 | 9269 9 | - | 575 | 51598357 | 2957476 |
| Complete genome (Accession number NC_006156) | hypothetical protein BG0096 | 9271 3 | 9325 8 | - | 181 | 51598358 | 2957174 |
| Complete genome (Accession number NC_006156) | V-type ATP synthase subunit E | 9326 8 | 9386 7 | - | 199 | 51598359 | 2957175 |
| Complete genome (Accession number NC_006156) | hypothetical protein BG0098 | 9405 3 | 9443 9 | - | 128 | 51598360 | 2957472 |
| Complete genome (Accession number NC_006156) | recombination and DNA strand exchange inhibitor protein | 9444 5 | 9678 1 | - | 778 | 51598361 | 2957607 |

| | | | | | | | |
|---|---|---|---|---|---|---|---|
| Complete genome (Accession number NC_006156) | hypothetical protein BG0100 | 9677 1 | 9769 4 | - | 307 | 51598362 | 2957453 |
| Complete genome (Accession number NC_006156) | glutamate racemase | 9769 1 | 9847 3 | - | 260 | 51598363 | 2957454 |
| Complete genome (Accession number NC_006156) | asparaginyl-tRNA synthetase | 9863 6 | 1000 24 | + | 462 | 51598364 | 2957176 |
| Complete genome (Accession number NC_006156) | hypothetical protein BG0103 | 1000 39 | 1005 60 | + | 173 | 51598365 | 2957386 |
| Complete genome (Accession number NC_006156) | hypothetical protein BG0104 | 1005 57 | 1011 53 | - | 198 | 51598366 | 2957447 |
| Complete genome (Accession number NC_006156) | periplasmic serine protease DO | 1013 41 | 1027 65 | - | 474 | 51598367 | 2957448 |
| Complete genome (Accession number NC_006156) | methionine aminopeptidase | 1028 36 | 1035 91 | - | 251 | 51598368 | 2957271 |
| Complete genome (Accession number NC_006156) | hypothetical protein BG0107 | 1035 84 | 1052 51 | - | 555 | 51598369 | 2957464 |
| Complete genome (Accession number NC_006156) | transcription antitermination protein NusB | 1052 38 | 1056 66 | - | 142 | 51598370 | 2957181 |
| Complete genome (Accession number NC_006156) | basic membrane protein | 1057 04 | 1067 14 | - | 336 | 51598371 | 2957182 |
| Complete genome (Accession number NC_006156) | acetyl-CoA C-acetyltransferase | 1067 94 | 1079 87 | - | 397 | 51598372 | 2957180 |

478

| | | | | | | | |
|---|---|---|---|---|---|---|---|
| Complete genome (Accession number NC_006156) | hypothetical protein BG0111 | 1081 30 | 1094 94 | + | 454 | 51598373 | 2957457 |
| Complete genome (Accession number NC_006156) | replicative DNA helicase | 1095 05 | 1108 72 | - | 455 | 51598374 | 2957189 |
| Complete genome (Accession number NC_006156) | 50S ribosomal protein L9 | 1108 97 | 1113 97 | - | 166 | 51598375 | 2957190 |
| Complete genome (Accession number NC_006156) | 30S ribosomal protein S18 | 1114 14 | 1117 04 | - | 96 | 51598376 | 2957348 |
| Complete genome (Accession number NC_006156) | single-stranded DNA-binding protein | 1117 19 | 1121 68 | - | 149 | 51598377 | 2957347 |
| Complete genome (Accession number NC_006156) | 30S ribosomal protein S6 | 1121 80 | 1125 99 | - | 139 | 51598378 | 2957314 |
| Complete genome (Accession number NC_006156) | PTS system, maltose and glucose-specific IIABC component | 1127 43 | 1144 37 | - | 564 | 51598379 | 2957315 |
| Complete genome (Accession number NC_006156) | hemolysin III | 1146 39 | 1153 40 | + | 233 | 51598380 | 2957387 |
| Complete genome (Accession number NC_006156) | zinc protease, putative | 1153 37 | 1166 38 | - | 433 | 51598381 | 2957585 |
| Complete genome (Accession number NC_006156) | phosphatidate cytidylyltransferase | 1166 60 | 1175 17 | - | 285 | 51598382 | 2957185 |

| | | | | | | | |
|---|---|---|---|---|---|---|---|
| Complete genome (Accession number NC_006156) | undecaprenyl diphosphate synthase | 1175 07 | 1181 99 | - | 230 | 51598383 | 2957186 |
| Complete genome (Accession number NC_006156) | ribosome releasing factor | 1182 04 | 1187 58 | - | 184 | 51598384 | 2957318 |
| Complete genome (Accession number NC_006156) | elongation factor Ts | 1187 96 | 1196 35 | - | 279 | 51598385 | 2957433 |
| Complete genome (Accession number NC_006156) | 30S ribosomal protein S2 | 1196 32 | 1204 20 | - | 262 | 51598386 | 2957197 |
| Complete genome (Accession number NC_006156) | hypothetical protein BG0125 | 1206 40 | 1209 18 | - | 92 | 51598387 | 2957198 |
| Complete genome (Accession number NC_006156) | hypothetical protein BG0126 | 1208 24 | 1209 37 | - | 37 | 51598388 | 2957431 |
| Complete genome (Accession number NC_006156) | hypothetical protein BG0127 | 1209 38 | 1217 20 | - | 260 | 51598389 | 2957536 |
| Complete genome (Accession number NC_006156) | hypothetical protein BG0128 | 1217 22 | 1223 33 | - | 203 | 51598390 | 2957195 |
| Complete genome (Accession number NC_006156) | 30S ribosomal protein S1 | 1223 33 | 1239 88 | - | 551 | 51598391 | 2957196 |
| Complete genome (Accession number NC_006156) | cytidylate kinase | 1239 91 | 1246 56 | - | 221 | 51598392 | 2957248 |
| Complete genome (Accession number NC_006156) | hypothetical protein BG0131 | 1246 40 | 1253 89 | - | 249 | 51598393 | 2957455 |

| | | | | | | | |
|---|---|---|---|---|---|---|---|
| Complete genome (Accession number NC_006156) | hypothetical protein BG0132 | 1253 76 | 1261 25 | - | 249 | 51598394 | 2957512 |
| Complete genome (Accession number NC_006156) | recombinase A | 1261 49 | 1272 37 | - | 362 | 51598395 | 2957513 |
| Complete genome (Accession number NC_006156) | transcript cleavage factor/unknown domain fusion protein | 1272 51 | 1299 53 | - | 900 | 51598396 | 2957425 |
| Complete genome (Accession number NC_006156) | hypothetical protein BG0135 | 1299 90 | 1309 34 | - | 314 | 51598397 | 2957531 |
| Complete genome (Accession number NC_006156) | hypothetical protein BG0136 | 1311 19 | 1322 46 | + | 375 | 51598398 | 2957206 |
| Complete genome (Accession number NC_006156) | histidyl-tRNA synthetase | 1322 93 | 1336 63 | - | 456 | 51598399 | 2957207 |
| Complete genome (Accession number NC_006156) | penicillin-binding protein | 1337 96 | 1356 85 | + | 629 | 51598400 | 2957421 |
| Complete genome (Accession number NC_006156) | long-chain-fatty-acid CoA ligase | 1356 86 | 1375 78 | - | 630 | 51598401 | 2957519 |
| Complete genome (Accession number NC_006156) | hypothetical protein BG0140 | 1380 21 | 1383 29 | - | 102 | 51598402 | 2957440 |
| Complete genome (Accession number NC_006156) | acriflavine resistance protein | 1383 31 | 1415 43 | - | 1070 | 51598403 | 2957441 |
| Complete genome (Accession | membrane fusion protein | 1415 62 | 1425 15 | - | 317 | 51598404 | 2957414 |

| | | | | | | | |
|---|---|---|---|---|---|---|---|
| number NC_006156) | | | | | | | |
| Complete genome (Accession number NC_006156) | hypothetical protein BG0143 | 1425 27 | 1437 62 | - | 411 | 51598405 | 2957479 |
| Complete genome (Accession number NC_006156) | glycine betaine, L-proline ABC transporter, binding protein | 1441 70 | 1450 54 | - | 294 | 51598406 | 2957481 |
| Complete genome (Accession number NC_006156) | glycine betaine, L-proline ABC transporter, permease protein | 1450 69 | 1459 68 | - | 299 | 51598407 | 2957482 |
| Complete genome (Accession number NC_006156) | glycine betaine, L-proline ABC transporter, ATP-binding protein | 1459 75 | 1470 93 | - | 372 | 51598408 | 2957400 |
| Complete genome (Accession number NC_006156) | flagellin | 1472 45 | 1482 55 | - | 336 | 51598409 | 2957384 |
| Complete genome (Accession number NC_006156) | flagellar hook-associated protein FliD | 1483 82 | 1503 79 | - | 665 | 51598410 | 2957361 |
| Complete genome (Accession number NC_006156) | N-acetylglucosamine-6-phosphate deacetylase | 1505 50 | 1517 55 | + | 401 | 51598411 | 2957362 |

| | | | | | | | |
|---|---|---|---|---|---|---|---|
| Complete genome (Accession number NC_006156) | glucosamine-6-phosphate deaminase | 1517 82 | 1525 88 | + | 268 | 51598412 | 2957532 |
| Complete genome (Accession number NC_006156) | superoxide dismutase | 1526 55 | 1532 66 | - | 203 | 51598413 | 2957282 |
| Complete genome (Accession number NC_006156) | preprotein translocase subunit SecA | 1532 81 | 1559 80 | - | 899 | 51598414 | 2957227 |
| Complete genome (Accession number NC_006156) | lipoprotein, putative | 1560 49 | 1571 82 | + | 377 | 51598415 | 2957228 |
| Complete genome (Accession number NC_006156) | hypothetical protein BG0154 | 1572 14 | 1576 42 | + | 142 | 51598416 | 2957377 |
| Complete genome (Accession number NC_006156) | hypothetical protein BG0155 | 1576 46 | 1580 62 | + | 138 | 51598417 | 2957373 |
| Complete genome (Accession number NC_006156) | antigen, S2, putative | 1582 08 | 1589 45 | + | 245 | 51598418 | 2957237 |
| Complete genome (Accession number NC_006156) | antigen S2-related protein | 1589 69 | 1596 43 | + | 224 | 51598419 | 2957238 |
| Complete genome (Accession number NC_006156) | alanine racemase | 1597 09 | 1608 09 | + | 366 | 51598420 | 2957370 |
| Complete genome (Accession number NC_006156) | hypothetical protein BG0159 | 1608 01 | 1624 02 | - | 533 | 51598421 | 2957252 |
| Complete genome (Accession number | hypothetical protein BG0160 | 1624 79 | 1626 46 | + | 55 | 51598422 | 2957217 |

| NC_006156) | | | | | | | |
|---|---|---|---|---|---|---|---|
| Complete genome (Accession number NC_006156) | hypothetical protein BG0161 | 1626 73 | 1644 21 | - | 582 | 51598423 | 2957218 |
| Complete genome (Accession number NC_006156) | Na+/Ca+ exchange protein, putative | 1645 02 | 1654 88 | - | 328 | 51598424 | 2957357 |
| Complete genome (Accession number NC_006156) | hypothetical protein BG0163 | 1655 16 | 1673 60 | - | 614 | 51598425 | 2957349 |
| Complete genome (Accession number NC_006156) | 4-alpha-glucanotransferase | 1674 72 | 1689 92 | - | 506 | 51598426 | 2957578 |
| Complete genome (Accession number NC_006156) | outer membrane protein | 1691 34 | 1703 03 | + | 389 | 51598427 | 2957579 |
| Complete genome (Accession number NC_006156) | hypothetical protein BG0166 | 1703 05 | 1703 70 | - | 21 | 51598428 | 2957296 |
| Complete genome (Accession number NC_006156) | dnaK suppressor, putative | 1703 15 | 1706 92 | - | 125 | 51598429 | 2957329 |
| Complete genome (Accession number NC_006156) | translation initiation factor IF-1 | 1708 58 | 1710 79 | + | 73 | 51598430 | 2957208 |
| Complete genome (Accession number NC_006156) | hypothetical protein BG0169 | 1711 26 | 1731 77 | - | 683 | 51598431 | 2957209 |
| Complete genome (Accession number NC_006156) | hypothetical protein BG0170 | 1731 26 | 1737 13 | - | 195 | 51598432 | 2957331 |

| | | | | | | | |
|---|---|---|---|---|---|---|---|
| Complete genome (Accession number NC_006156) | hypothetical protein BG0171 | 1736 95 | 1746 81 | - | 328 | 51598433 | 2957326 |
| Complete genome (Accession number NC_006156) | hypothetical protein BG0172 | 1746 78 | 1756 79 | - | 333 | 51598434 | 2957266 |
| Complete genome (Accession number NC_006156) | hypothetical protein BG0173 | 1756 66 | 1765 65 | - | 299 | 51598435 | 2957267 |
| Complete genome (Accession number NC_006156) | hypothetical protein BG0174 | 1765 72 | 1774 44 | - | 290 | 51598436 | 2957269 |
| Complete genome (Accession number NC_006156) | MoxR-related protein | 1774 62 | 1784 54 | - | 330 | 51598437 | 2957444 |
| Complete genome (Accession number NC_006156) | glucose inhibited division protein B | 1785 11 | 1791 37 | - | 208 | 51598438 | 2957279 |
| Complete genome (Accession number NC_006156) | tRNA uridine 5-carboxymethylamin omethyl modification enzyme GidA | 1791 34 | 1809 99 | - | 621 | 51598439 | 2957280 |
| Complete genome (Accession number NC_006156) | tRNA modification GTPase TrmE | 1810 02 | 1823 96 | - | 464 | 51598440 | 2957300 |
| Complete genome (Accession number NC_006156) | flagellar protein, putative | 1824 79 | 1829 73 | + | 164 | 51598441 | 2957214 |
| Complete genome (Accession number NC_006156) | flagellar hook-associated protein FlgK | 1829 85 | 1848 68 | + | 627 | 51598442 | 2957159 |

| | | | | | | | |
|---|---|---|---|---|---|---|---|
| Complete genome (Accession number NC_006156) | flagellar hook-associated protein FlgL | 1848 71 | 1861 45 | + | 424 | 51598443 | 2957160 |
| Complete genome (Accession number NC_006156) | flagellar assembly protein FliW | 1861 47 | 1865 39 | + | 130 | 51598444 | 2957533 |
| Complete genome (Accession number NC_006156) | carbon storage regulator | 1865 42 | 1867 87 | + | 81 | 51598445 | 2957289 |
| Complete genome (Accession number NC_006156) | hypothetical protein BG0184 | 1867 71 | 1874 24 | - | 217 | 51598446 | 2957285 |
| Complete genome (Accession number NC_006156) | hypothetical protein BG0185 | 1874 17 | 1878 30 | - | 137 | 51598447 | 2957286 |
| Complete genome (Accession number NC_006156) | hypothetical protein BG0186 | 1878 27 | 1880 87 | - | 86 | 51598448 | 2957262 |
| Complete genome (Accession number NC_006156) | 50S ribosomal protein L20 | 1883 01 | 1886 48 | - | 115 | 51598449 | 2957298 |
| Complete genome (Accession number NC_006156) | 50S ribosomal protein L35 | 1886 69 | 1888 69 | - | 66 | 51598450 | 2957283 |
| Complete genome (Accession number NC_006156) | translation initiation factor IF-3 | 1888 92 | 1894 52 | - | 186 | 51598451 | 2957284 |
| Complete genome (Accession number NC_006156) | hypothetical protein BG0190 | 1900 55 | 1902 97 | - | 80 | 51598452 | 2957529 |
| Complete genome (Accession number NC_006156) | lipoprotein, putative | 1903 18 | 1910 58 | + | 246 | 51598453 | 2957272 |

| Complete genome (Accession number NC_006156) | hypothetical protein BG0192 | 1911 11 | 1919 20 | - | 269 | 51598454 | 2957287 |
|---|---|---|---|---|---|---|---|
| Complete genome (Accession number NC_006156) | hypothetical protein BG0193 | 1919 17 | 1930 56 | - | 379 | 51598455 | 2957288 |
| Complete genome (Accession number NC_006156) | peptide chain release factor 1 | 1933 92 | 1944 65 | + | 357 | 51598456 | 2957534 |
| Complete genome (Accession number NC_006156) | HemK family methylase, putative | 1944 68 | 1952 89 | + | 273 | 51598457 | 2957216 |
| Complete genome (Accession number NC_006156) | guanosine-3,5-bis(diphosphate) 3-pyrophosphohydrol ase | 1952 86 | 1972 89 | + | 667 | 51598458 | 2957301 |
| Complete genome (Accession number NC_006156) | hypothetical protein BG0197 | 1972 79 | 1995 43 | + | 754 | 51598459 | 2957302 |
| Complete genome (Accession number NC_006156) | hypothetical protein BG0198 | 1994 88 | 1995 92 | - | 34 | 51598460 | 2957167 |
| Complete genome (Accession number NC_006156) | D-alanine--D-alanine ligase | 1995 40 | 2006 25 | - | 361 | 51598461 | 2957253 |
| Complete genome (Accession number NC_006156) | UDP-N-acetylmuramoylala nyl-D-glutamate--2, 6-diaminopimelate ligase | 2006 27 | 2021 53 | - | 508 | 51598462 | 2957178 |
| Complete genome (Accession | hemolysin, putative | 2024 26 | 2036 64 | + | 412 | 51598463 | 2957244 |

| | | | | | | | |
|---|---|---|---|---|---|---|---|
| number NC_006156) | | | | | | | |
| Complete genome (Accession number NC_006156) | Lambda CII stability-governing protein | 2037 17 | 2046 52 | + | 311 | 51598464 | 2957245 |
| Complete genome (Accession number NC_006156) | Lambda CII stability-governing protein | 2046 53 | 2056 24 | + | 323 | 51598465 | 2957303 |
| Complete genome (Accession number NC_006156) | hypothetical protein BG0207 | 2061 24 | 2077 01 | + | 525 | 51598466 | 2957310 |
| Complete genome (Accession number NC_006156) | hypothetical protein BG0208 | 2076 79 | 2082 18 | - | 179 | 51598467 | 2957304 |
| Complete genome (Accession number NC_006156) | UTP--glucose-1-phosphate uridylyltransferase | 2083 04 | 2091 40 | + | 278 | 51598468 | 2957219 |
| Complete genome (Accession number NC_006156) | hypothetical protein BG0210 | 2091 27 | 2108 75 | + | 582 | 51598469 | 2957164 |
| Complete genome (Accession number NC_006156) | hypothetical protein BG0211 | 2109 02 | 2116 57 | + | 251 | 51598470 | 2957165 |
| Complete genome (Accession number NC_006156) | surface-located membrane protein 1 | 2116 50 | 2143 70 | + | 906 | 51598471 | 2957308 |
| Complete genome (Accession number NC_006156) | DNA mismatch repair protein | 2143 77 | 2162 12 | + | 611 | 51598472 | 2957412 |
| Complete genome (Accession number NC_006156) | hypothetical protein BG0214 | 2162 31 | 2172 62 | + | 343 | 51598473 | 2957306 |

| | | | | | | | |
|---|---|---|---|---|---|---|---|
| Complete genome (Accession number NC_006156) | lipoprotein, putative | 2173 45 | 2179 98 | - | 217 | 51598474 | 2957183 |
| Complete genome (Accession number NC_006156) | translation elongation factor P | 2180 06 | 2185 84 | - | 192 | 51598475 | 2957184 |
| Complete genome (Accession number NC_006156) | phosphate ABC transporter, periplasmic phosphate-binding protein | 2187 50 | 2195 86 | + | 278 | 51598476 | 2957320 |
| Complete genome (Accession number NC_006156) | phosphate ABC transporter, permease protein | 2196 76 | 2205 84 | + | 302 | 51598477 | 2957311 |
| Complete genome (Accession number NC_006156) | phosphate ABC transporter, permease protein | 2207 55 | 2221 25 | + | 456 | 51598478 | 2957324 |
| Complete genome (Accession number NC_006156) | phosphate ABC transporter, ATP-binding protein | 2221 26 | 2229 08 | + | 260 | 51598479 | 2957325 |
| Complete genome (Accession number NC_006156) | gufA protein | 2229 11 | 2237 32 | - | 273 | 51598480 | 2957265 |
| Complete genome (Accession number NC_006156) | alanyl-tRNA synthetase | 2237 67 | 2255 51 | - | 594 | 51598481 | 2957270 |
| Complete genome (Accession number NC_006156) | flagellar motor switch protein | 2255 51 | 2267 68 | - | 405 | 51598482 | 2957169 |

| Complete genome (Accession number NC_006156) | 6-phosphogluconolac tonase | 2267 55 | 2274 62 | - | 235 | 51598483 | 2957170 |
|---|---|---|---|---|---|---|---|
| Complete genome (Accession number NC_006156) | hypothetical protein BG0226 | 2276 01 | 2277 47 | - | 48 | 51598484 | 2957249 |
| Complete genome (Accession number NC_006156) | lipoprotein, putative | 2278 65 | 2280 92 | - | 75 | 51598485 | 2957435 |
| Complete genome (Accession number NC_006156) | tRNA-dihydrouridine synthase A | 2281 69 | 2291 70 | + | 333 | 51598486 | 2957514 |
| Complete genome (Accession number NC_006156) | seryl-tRNA synthetase | 2291 60 | 2304 37 | - | 425 | 51598487 | 2957515 |
| Complete genome (Accession number NC_006156) | hypothetical protein BG0230 | 2305 67 | 2312 68 | + | 233 | 51598488 | 2957401 |
| Complete genome (Accession number NC_006156) | hypothetical protein BG0231 | 2312 84 | 2342 02 | + | 972 | 51598489 | 2957330 |
| Complete genome (Accession number NC_006156) | 50S ribosomal protein L31 type B | 2342 38 | 2344 83 | - | 81 | 51598490 | 2957332 |
| Complete genome (Accession number NC_006156) | transcription termination factor Rho | 2345 44 | 2360 91 | - | 515 | 51598491 | 2957333 |
| Complete genome (Accession number NC_006156) | hypothetical protein BG0234 | 2361 71 | 2365 78 | - | 135 | 51598492 | 2957268 |
| Complete genome (Accession number NC_006156) | hbbU protein | 2365 79 | 2369 05 | - | 108 | 51598493 | 2957489 |

| | | | | | | | |
|---|---|---|---|---|---|---|---|
| Complete genome (Accession number NC_006156) | 30S ribosomal protein S20 | 2369 18 | 2371 75 | - | 85 | 51598494 | 2957260 |
| Complete genome (Accession number NC_006156) | hypothetical protein BG0237 | 2372 46 | 2380 73 | - | 275 | 51598495 | 2957261 |
| Complete genome (Accession number NC_006156) | translation-associated GTPase | 2380 88 | 2391 94 | - | 368 | 51598496 | 2957275 |
| Complete genome (Accession number NC_006156) | hypothetical protein BG0239 | 2392 24 | 2412 30 | - | 668 | 51598497 | 2957254 |
| Complete genome (Accession number NC_006156) | apolipoprotein N-acyltransferase | 2413 77 | 2429 42 | + | 521 | 51598498 | 2957352 |
| Complete genome (Accession number NC_006156) | hypothetical protein BG0241 | 2428 82 | 2436 52 | - | 256 | 51598499 | 2957353 |
| Complete genome (Accession number NC_006156) | deoxyguanosine/deoxyadenosine kinase(I) subunit 2 | 2437 17 | 2443 19 | - | 200 | 51598500 | 2957355 |
| Complete genome (Accession number NC_006156) | glycerol uptake facilitator | 2447 31 | 2454 95 | + | 254 | 51598501 | 2957340 |
| Complete genome (Accession number NC_006156) | glycerol kinase | 2455 34 | 2470 39 | + | 501 | 51598502 | 2957503 |
| Complete genome (Accession number NC_006156) | glycerol-3-phosphate dehydrogenase, anaerobic | 2473 16 | 2488 84 | + | 522 | 51598503 | 2957504 |

| | | | | | | | |
|---|---|---|---|---|---|---|---|
| Complete genome (Accession number NC_006156) | hypothetical protein BG0246 | 2489 71 | 2494 65 | - | 164 | 51598504 | 2957259 |
| Complete genome (Accession number NC_006156) | hypothetical protein BG0247 | 2494 52 | 2500 06 | - | 184 | 51598505 | 2957356 |
| Complete genome (Accession number NC_006156) | hypothetical protein BG0248 | 2500 07 | 2510 32 | - | 341 | 51598506 | 2957473 |
| Complete genome (Accession number NC_006156) | hypothetical protein BG0249 | 2512 29 | 2518 34 | + | 201 | 51598507 | 2957474 |
| Complete genome (Accession number NC_006156) | oligoendopeptidase F | 2519 55 | 2537 27 | + | 590 | 51598508 | 2957490 |
| Complete genome (Accession number NC_006156) | phosphatidyltransfe rase | 2537 41 | 2544 45 | + | 234 | 51598509 | 2957246 |
| Complete genome (Accession number NC_006156) | dedA protein | 2544 42 | 2550 56 | + | 204 | 51598510 | 2957359 |
| Complete genome (Accession number NC_006156) | leucyl-tRNA synthetase | 2552 53 | 2577 75 | + | 840 | 51598511 | 2957256 |
| Complete genome (Accession number NC_006156) | hypothetical protein BG0255 | 2577 90 | 2600 90 | + | 766 | 51598512 | 2957257 |
| Complete genome (Accession number NC_006156) | ATP-dependent protease LA | 2600 87 | 2625 07 | - | 806 | 51598513 | 2957369 |

| | | | | | | | |
|---|---|---|---|---|---|---|---|
| Complete genome (Accession number NC_006156) | single-stranded-DNA-specific exonuclease | 2627 84 | 2649 10 | + | 708 | 51598514 | 2957309 |
| Complete genome (Accession number NC_006156) | hypothetical protein BG0258 | 2649 03 | 2658 47 | + | 314 | 51598515 | 2957415 |
| Complete genome (Accession number NC_006156) | 30S ribosomal protein S21 | 2658 62 | 2660 71 | + | 69 | 51598516 | 2957416 |
| Complete genome (Accession number NC_006156) | cell division protein, putative | 2661 07 | 2684 58 | - | 783 | 51598517 | 2957488 |
| Complete genome (Accession number NC_006156) | undecaprenyl pyrophosphate phosphatase | 2684 55 | 2692 55 | - | 266 | 51598518 | 2957365 |
| Complete genome (Accession number NC_006156) | hypothetical protein BG0262 | 2692 70 | 2714 17 | - | 715 | 51598519 | 2957242 |
| Complete genome (Accession number NC_006156) | hypothetical protein BG0263 | 2714 10 | 2724 26 | - | 338 | 51598520 | 2957243 |
| Complete genome (Accession number NC_006156) | hypothetical protein BG0264 | 2726 30 | 2740 12 | + | 460 | 51598521 | 2957297 |
| Complete genome (Accession number NC_006156) | hypothetical protein BG0265 | 2740 19 | 2752 72 | - | 417 | 51598522 | 2957291 |
| Complete genome (Accession number NC_006156) | signal peptidase I | 2754 48 | 2758 40 | + | 130 | 51598523 | 2957336 |
| Complete genome (Accession | heat shock protein 70 | 2758 65 | 2773 34 | - | 489 | 51598524 | 2957337 |

| | | | | | | | |
|---|---|---|---|---|---|---|---|
| number NC_006156) | | | | | | | |
| Complete genome (Accession number NC_006156) | hypothetical protein BG0268 | 2773 12 | 2778 33 | - | 173 | 51598525 | 2957344 |
| Complete genome (Accession number NC_006156) | hypothetical protein BG0269 | 2778 53 | 2781 55 | - | 100 | 51598526 | 2957234 |
| Complete genome (Accession number NC_006156) | hypothetical protein BG0270 | 2781 82 | 2800 86 | - | 634 | 51598527 | 2957350 |
| Complete genome (Accession number NC_006156) | hypothetical protein BG0271 | 2800 89 | 2805 68 | - | 159 | 51598528 | 2957351 |
| Complete genome (Accession number NC_006156) | minD-related ATP-binding protein | 2805 78 | 2814 65 | - | 295 | 51598529 | 2957478 |
| Complete genome (Accession number NC_006156) | flagellar biosynthesis regulator FlhF | 2814 77 | 2826 43 | - | 388 | 51598530 | 2957354 |
| Complete genome (Accession number NC_006156) | flagellar biosynthesis protein A | 2826 48 | 2847 38 | - | 696 | 51598531 | 2957235 |
| Complete genome (Accession number NC_006156) | flagellar biosynthesis protein FlhB | 2847 50 | 2858 68 | - | 372 | 51598532 | 2957236 |
| Complete genome (Accession number NC_006156) | flagellar biosynthesis protein | 2858 68 | 2866 56 | - | 262 | 51598533 | 2957430 |
| Complete genome (Accession number NC_006156) | flagellar biosynthesis protein | 2866 92 | 2869 55 | - | 87 | 51598534 | 2957371 |

| Complete genome (Accession number NC_006156) | flagellar biosynthesis protein FliP | 2869 64 | 2877 28 | - | 254 | 51598535 | 2957451 |
|---|---|---|---|---|---|---|---|
| Complete genome (Accession number NC_006156) | flagellar biosynthesis protein | 2877 39 | 2883 65 | - | 208 | 51598536 | 2957452 |
| Complete genome (Accession number NC_006156) | flagellar motor switch protein | 2883 58 | 2886 99 | - | 113 | 51598537 | 2957233 |
| Complete genome (Accession number NC_006156) | flagellar motor switch protein FliM | 2887 45 | 2898 03 | - | 352 | 51598538 | 2957229 |
| Complete genome (Accession number NC_006156) | flagellar basal body-associated protein FliL | 2898 28 | 2903 64 | - | 178 | 51598539 | 2957375 |
| Complete genome (Accession number NC_006156) | flagellar motor protein MotB | 2904 12 | 2911 94 | - | 260 | 51598540 | 2957376 |
| Complete genome (Accession number NC_006156) | flagellar motor rotation protein A | 2911 94 | 2919 76 | - | 260 | 51598541 | 2957338 |
| Complete genome (Accession number NC_006156) | flagellar protein | 2919 73 | 2921 97 | - | 74 | 51598542 | 2957417 |
| Complete genome (Accession number NC_006156) | flagellar hook protein FlgE | 2922 19 | 2935 47 | - | 442 | 51598543 | 2957199 |
| Complete genome (Accession number NC_006156) | flagellar basal body rod modification protein | 2935 52 | 2939 95 | - | 147 | 51598544 | 2957200 |
| Complete genome (Accession | flagellar protein | 2940 09 | 2951 96 | - | 395 | 51598545 | 2957379 |

| | | | | | | | |
|---|---|---|---|---|---|---|---|
| number NC_006156) | | | | | | | |
| Complete genome (Accession number NC_006156) | flagellar protein | 2952 04 | 2957 82 | - | 192 | 51598546 | 2957255 |
| Complete genome (Accession number NC_006156) | flagellar protein | 2958 14 | 2962 45 | - | 143 | 51598547 | 2957381 |
| Complete genome (Accession number NC_006156) | flagellum-specific ATP synthase | 2962 42 | 2975 52 | - | 436 | 51598548 | 2957382 |
| Complete genome (Accession number NC_006156) | flagellar assembly protein H | 2975 71 | 2984 91 | - | 306 | 51598549 | 2957396 |
| Complete genome (Accession number NC_006156) | flagellar motor switch protein G | 2985 06 | 2995 40 | - | 344 | 51598550 | 2957172 |
| Complete genome (Accession number NC_006156) | flagellar MS-ring protein | 2995 56 | 3012 65 | - | 569 | 51598551 | 2957154 |
| Complete genome (Accession number NC_006156) | flagellar hook-basal body protein FliE | 3012 80 | 3016 15 | - | 111 | 51598552 | 2957155 |
| Complete genome (Accession number NC_006156) | flagellar basal body rod protein FlgC | 3016 27 | 3020 85 | - | 152 | 51598553 | 2957191 |
| Complete genome (Accession number NC_006156) | flagellar basal body rod protein FlgB | 3021 09 | 3025 16 | - | 135 | 51598554 | 2957391 |

| | | | | | | | |
|---|---|---|---|---|---|---|---|
| Complete genome (Accession number NC_006156) | ATP-dependent protease ATP-binding subunit | 3025 50 | 3038 96 | - | 448 | 51598555 | 2957193 |
| Complete genome (Accession number NC_006156) | ATP-dependent protease peptidase subunit | 3038 89 | 3044 37 | - | 182 | 51598556 | 2957194 |
| Complete genome (Accession number NC_006156) | hypothetical protein BG0300 | 3044 47 | 3045 63 | - | 38 | 51598557 | 2957398 |
| Complete genome (Accession number NC_006156) | smg protein | 3045 38 | 3053 95 | - | 285 | 51598558 | 2957406 |
| Complete genome (Accession number NC_006156) | hypothetical protein BG0302 | 3054 02 | 3060 94 | - | 230 | 51598559 | 2957404 |
| Complete genome (Accession number NC_006156) | cell division protein FtsZ | 3060 95 | 3072 94 | - | 399 | 51598560 | 2957405 |
| Complete genome (Accession number NC_006156) | cell division protein | 3073 16 | 3085 57 | - | 413 | 51598561 | 2957222 |
| Complete genome (Accession number NC_006156) | cell division protein | 3085 57 | 3093 00 | - | 247 | 51598562 | 2957399 |
| Complete genome (Accession number NC_006156) | cell division protein | 3093 36 | 3103 94 | - | 352 | 51598563 | 2957520 |

| | | | | | | | |
|---|---|---|---|---|---|---|---|
| Complete genome (Accession number NC_006156) | phospho-N-acetylmuramoyl-pentapeptide-transferase | 3104 37 | 3114 92 | - | 351 | 51598564 | 2957521 |
| Complete genome (Accession number NC_006156) | UDP-N-acetylmuramoylala nyl-D-glutamyl-2, 6-diaminopimelate--D-alanyl-D-alanine ligase | 3115 10 | 3129 01 | - | 463 | 51598565 | 2957545 |
| Complete genome (Accession number NC_006156) | hypothetical protein BG0309 | 3129 23 | 3132 04 | - | 93 | 51598566 | 2957403 |
| Complete genome (Accession number NC_006156) | hypothetical protein BG0310 | 3132 01 | 3140 91 | - | 296 | 51598567 | 2957410 |
| Complete genome (Accession number NC_006156) | hypothetical protein BG0311 | 3140 84 | 3149 20 | - | 278 | 51598568 | 2957411 |
| Complete genome (Accession number NC_006156) | hypothetical protein BG0312 | 3149 17 | 3159 93 | - | 358 | 51598569 | 2957168 |
| Complete genome (Accession number NC_006156) | hypothetical protein BG0313 | 3160 22 | 3167 80 | - | 252 | 51598570 | 2957456 |
| Complete genome (Accession number NC_006156) | hypothetical protein BG0314 | 3170 96 | 3179 35 | - | 279 | 51598571 | 2957418 |
| Complete genome (Accession number NC_006156) | purine-binding chemotaxis protein | 3179 25 | 3184 55 | - | 176 | 51598572 | 2957419 |

| Complete genome (Accession number NC_006156) | cell division protein | 3185 01 | 3190 79 | - | 192 | 51598573 | 2957413 |
|---|---|---|---|---|---|---|---|
| Complete genome (Accession number NC_006156) | octaprenyl-diphosphate synthase | 3191 30 | 3201 73 | + | 347 | 51598574 | 2957201 |
| Complete genome (Accession number NC_006156) | hypothetical protein BG0318 | 3201 73 | 3208 56 | + | 227 | 51598575 | 2957203 |
| Complete genome (Accession number NC_006156) | hypothetical protein BG0319 | 3208 58 | 3216 64 | - | 268 | 51598576 | 2957204 |
| Complete genome (Accession number NC_006156) | hypothetical protein BG0320 | 3216 65 | 3225 97 | - | 310 | 51598577 | 2957526 |
| Complete genome (Accession number NC_006156) | methylgalactoside ABC transporter, ATP-binding protein | 3225 94 | 3240 54 | - | 486 | 51598578 | 2957527 |
| Complete genome (Accession number NC_006156) | exported protein | 3240 54 | 3251 06 | - | 350 | 51598579 | 2957140 |
| Complete genome (Accession number NC_006156) | hypothetical protein BG0323 | 3255 24 | 3265 64 | - | 346 | 51598580 | 2957141 |
| Complete genome (Accession number NC_006156) | hypothetical protein BG0324 | 3268 53 | 3279 83 | + | 376 | 51598581 | 2957205 |
| Complete genome (Accession number NC_006156) | hypothetical protein BG0325 | 3280 78 | 3284 43 | + | 121 | 51598582 | 2957220 |

| | | | | | | | |
|---|---|---|---|---|---|---|---|
| Complete genome (Accession number NC_006156) | hypothetical protein BG0326 | 3284 50 | 3295 59 | - | 369 | 51598583 | 2957423 |
| Complete genome (Accession number NC_006156) | hypothetical protein BG0327 | 3296 10 | 3324 14 | - | 934 | 51598584 | 2957424 |
| Complete genome (Accession number NC_006156) | glycerol-3-phosphate O-acyltransferase, putative | 3324 38 | 3333 31 | - | 297 | 51598585 | 2957420 |
| Complete genome (Accession number NC_006156) | oligopeptide ABC transporter, periplasmic oligopeptide-binding protein | 3337 22 | 3352 93 | + | 523 | 51598586 | 2957449 |
| Complete genome (Accession number NC_006156) | oligopeptide ABC transporter, periplasmic oligopeptide-binding protein | 3353 91 | 3369 74 | + | 527 | 51598587 | 2957540 |
| Complete genome (Accession number NC_006156) | oligopeptide ABC transporter, periplasmic oligopeptide-binding protein | 3371 16 | 3387 41 | + | 541 | 51598588 | 2957541 |
| Complete genome (Accession number NC_006156) | oligopeptide ABC transporter, permease protein | 3390 78 | 3399 98 | + | 306 | 51598589 | 2957496 |

| Complete genome (Accession number NC_006156) | oligopeptide ABC transporter, permease protein | 3400 11 | 3410 60 | + | 349 | 51598590 | 2957142 |
|---|---|---|---|---|---|---|---|
| Complete genome (Accession number NC_006156) | hypothetical protein BG0334 | 3410 68 | 3411 15 | + | 15 | 51598591 | 2957427 |
| Complete genome (Accession number NC_006156) | oligopeptide ABC transporter, ATP-binding protein | 3410 72 | 3419 44 | + | 290 | 51598592 | 2957428 |
| Complete genome (Accession number NC_006156) | oligopeptide ABC transporter, ATP-binding protein | 3419 45 | 3429 16 | + | 323 | 51598593 | 2957537 |
| Complete genome (Accession number NC_006156) | P26 | 3429 30 | 3436 85 | - | 251 | 51598594 | 2957439 |
| Complete genome (Accession number NC_006156) | enolase | 3438 04 | 3451 20 | + | 438 | 51598595 | 2957276 |
| Complete genome (Accession number NC_006156) | 30S ribosomal protein S9 | 3451 72 | 3455 82 | - | 136 | 51598596 | 2957277 |
| Complete genome (Accession number NC_006156) | 50S ribosomal protein L13 | 3455 79 | 3460 43 | - | 154 | 51598597 | 2957528 |
| Complete genome (Accession number NC_006156) | hypothetical protein BG0341 | 3460 96 | 3469 32 | - | 278 | 51598598 | 2957443 |
| Complete genome (Accession number NC_006156) | aspartyl/glutamyl-tRNA amidotransferase subunit B | 3468 98 | 3483 55 | - | 485 | 51598599 | 2957239 |

| | | | | | | | |
|---|---|---|---|---|---|---|---|
| Complete genome (Accession number NC_006156) | Glu-tRNA(Gln) amidotransferase, subunit A | 3483 45 | 3497 90 | - | 481 | 51598600 | 2957240 |
| Complete genome (Accession number NC_006156) | aspartyl/glutamyl-tRNA amidotransferase subunit C | 3498 01 | 3500 76 | - | 91 | 51598601 | 2957436 |
| Complete genome (Accession number NC_006156) | DNA helicase | 3500 86 | 3521 82 | - | 698 | 51598602 | 2957535 |
| Complete genome (Accession number NC_006156) | hypothetical protein BG0346 | 3521 85 | 3533 84 | - | 399 | 51598603 | 2957393 |
| Complete genome (Accession number NC_006156) | hypothetical protein BG0347 | 3533 95 | 3540 45 | - | 216 | 51598604 | 2957394 |
| Complete genome (Accession number NC_006156) | fibronectin/fibrinogen-binding protein, putative | 3542 39 | 3556 57 | + | 472 | 51598605 | 2957187 |
| Complete genome (Accession number NC_006156) | pyruvate kinase | 3557 55 | 3571 88 | + | 477 | 51598606 | 2957437 |
| Complete genome (Accession number NC_006156) | hypothetical protein BG0350 | 3572 06 | 3579 46 | + | 246 | 51598607 | 2957442 |
| Complete genome (Accession number NC_006156) | 50S ribosomal protein L28 | 3579 65 | 3582 43 | - | 92 | 51598608 | 2957273 |
| Complete genome (Accession number NC_006156) | hypothetical protein BG0352 | 3582 65 | 3598 27 | - | 520 | 51598609 | 2957274 |

| | | | | | | | |
|---|---|---|---|---|---|---|---|
| Complete genome (Accession number NC_006156) | hypothetical protein BG0353 | 3599 50 | 3610 71 | + | 373 | 51598610 | 2957480 |
| Complete genome (Accession number NC_006156) | hypothetical protein BG0354 | 3610 68 | 3628 67 | - | 599 | 51598611 | 2957471 |
| Complete genome (Accession number NC_006156) | hypothetical protein BG0355 | 3628 80 | 3638 30 | - | 316 | 51598612 | 2957334 |
| Complete genome (Accession number NC_006156) | transcription factor, putative | 3638 64 | 3643 52 | - | 162 | 51598613 | 2957499 |
| Complete genome (Accession number NC_006156) | hypothetical protein BG0357 | 3643 92 | 3649 85 | - | 197 | 51598614 | 2957500 |
| Complete genome (Accession number NC_006156) | hypothetical protein BG0358 | 3649 78 | 3657 09 | - | 243 | 51598615 | 2957475 |
| Complete genome (Accession number NC_006156) | carboxyl-terminal protease | 3657 13 | 3671 43 | - | 476 | 51598616 | 2957226 |
| Complete genome (Accession number NC_006156) | minD-related ATP-binding protein | 3676 37 | 3687 79 | + | 380 | 51598617 | 2957450 |
| Complete genome (Accession number NC_006156) | prolipoprotein diacylglyceryl transferase | 3687 79 | 3697 65 | + | 328 | 51598618 | 2957263 |
| Complete genome (Accession number NC_006156) | periplasmic protein | 3697 86 | 3717 98 | + | 670 | 51598619 | 2957264 |
| Complete genome (Accession number | methylglyoxal synthase | 3718 93 | 3722 73 | + | 126 | 51598620 | 2957171 |

| NC_006156) | | | | | | | |
|---|---|---|---|---|---|---|---|
| Complete genome (Accession number NC_006156) | lipoprotein LA7 | 3723 27 | 3729 14 | - | 195 | 51598621 | 2957422 |
| Complete genome (Accession number NC_006156) | putative aminopeptidase 1 | 3730 38 | 3744 14 | + | 458 | 51598622 | 2957317 |
| Complete genome (Accession number NC_006156) | hypothetical protein BG0366 | 3744 21 | 3746 99 | + | 92 | 51598623 | 2957445 |
| Complete genome (Accession number NC_006156) | NAD(P)H-dependent glycerol-3-phosphate dehydrogenase | 3747 25 | 3757 80 | - | 351 | 51598624 | 2957213 |
| Complete genome (Accession number NC_006156) | ATP-dependent Clp protease, subunit A | 3757 98 | 3780 65 | - | 755 | 51598625 | 2957467 |
| Complete genome (Accession number NC_006156) | tyrosyl-tRNA synthetase | 3781 03 | 3793 20 | - | 405 | 51598626 | 2957409 |
| Complete genome (Accession number NC_006156) | glycyl-tRNA synthetase | 3793 17 | 3806 54 | - | 445 | 51598627 | 2957156 |
| Complete genome (Accession number NC_006156) | glutamyl-tRNA synthetase | 3806 72 | 3821 44 | - | 490 | 51598628 | 2957485 |
| Complete genome (Accession number NC_006156) | hypothetical protein BG0372 | 3821 57 | 3829 24 | - | 255 | 51598629 | 2957511 |

| Complete genome (Accession number NC_006156) | hypothetical protein BG0373 | 3830 50 | 3841 86 | + | 378 | 51598630 | 2957544 |
|---|---|---|---|---|---|---|---|
| Complete genome (Accession number NC_006156) | 5-methylthioadenosine/S-adenosylhomocysteine nucleosidase, putative | 3842 09 | 3849 22 | + | 237 | 51598631 | 2957392 |
| Complete genome (Accession number NC_006156) | S-adenosylmethionine synthetase | 3849 19 | 3860 97 | + | 392 | 51598632 | 2957498 |
| Complete genome (Accession number NC_006156) | S-ribosylhomocysteinase | 3860 94 | 3865 67 | + | 157 | 51598633 | 2957157 |
| Complete genome (Accession number NC_006156) | hypothetical protein BG0377 | 3865 78 | 3872 37 | - | 219 | 51598634 | 2957158 |
| Complete genome (Accession number NC_006156) | protein kinase C1 inhibitor | 3872 64 | 3876 83 | - | 139 | 51598635 | 2957225 |
| Complete genome (Accession number NC_006156) | Mg2+ transport protein | 3877 34 | 3890 98 | - | 454 | 51598636 | 2957364 |
| Complete genome (Accession number NC_006156) | hypothetical protein BG0380 | 3891 52 | 3905 76 | - | 474 | 51598637 | 2957374 |
| Complete genome (Accession number NC_006156) | basic membrane protein B | 3906 89 | 3917 14 | - | 341 | 51598638 | 2957152 |

| | | | | | | | |
|---|---|---|---|---|---|---|---|
| Complete genome (Accession number NC_006156) | basic membrane protein A | 3918 11 | 3928 24 | - | 337 | 51598639 | 2957497 |
| Complete genome (Accession number NC_006156) | basic membrane protein A | 3936 89 | 3947 02 | - | 337 | 51598640 | 2957518 |
| Complete genome (Accession number NC_006156) | basic membrane protein C | 3947 43 | 3958 04 | - | 353 | 51598641 | 2957339 |
| Complete genome (Accession number NC_006156) | basic membrane protein D | 3961 27 | 3971 52 | - | 341 | 51598642 | 2957188 |
| Complete genome (Accession number NC_006156) | 30S ribosomal protein S7 | 3972 54 | 3977 27 | - | 157 | 51598643 | 2957395 |
| Complete genome (Accession number NC_006156) | 30S ribosomal protein S12 | 3977 52 | 3981 26 | - | 124 | 51598644 | 2957507 |
| Complete genome (Accession number NC_006156) | DNA-directed RNA polymerase subunit beta' | 3981 92 | 4023 25 | - | 1377 | 51598645 | 2957150 |
| Complete genome (Accession number NC_006156) | DNA-directed RNA polymerase subunit beta | 4023 41 | 4058 08 | - | 1155 | 51598646 | 2957177 |
| Complete genome (Accession number NC_006156) | 50S ribosomal protein L7/L12 | 4058 91 | 4062 65 | - | 124 | 51598647 | 2957147 |
| Complete genome (Accession number NC_006156) | 50S ribosomal protein L10 | 4063 36 | 4068 24 | - | 162 | 51598648 | 2957161 |

| | | | | | | | |
|---|---|---|---|---|---|---|---|
| Complete genome (Accession number NC_006156) | 50S ribosomal protein L1 | 4068 29 | 4075 09 | - | 226 | 51598649 | 2957163 |
| Complete genome (Accession number NC_006156) | 50S ribosomal protein L11 | 4075 09 | 4079 40 | - | 143 | 51598650 | 2957510 |
| Complete genome (Accession number NC_006156) | transcription antitermination factor | 4079 92 | 4085 46 | - | 184 | 51598651 | 2957539 |
| Complete genome (Accession number NC_006156) | preprotein translocase subunit SecE | 4085 70 | 4087 40 | - | 56 | 51598652 | 2957517 |
| Complete genome (Accession number NC_006156) | 50S ribosomal protein L33 | 4088 48 | 4090 27 | - | 59 | 51598653 | 2957146 |
| Complete genome (Accession number NC_006156) | hypothetical protein BG0400 | 4091 92 | 4100 49 | - | 285 | 51598654 | 2957293 |
| Complete genome (Accession number NC_006156) | lipoprotein, putative | 4100 63 | 4110 94 | - | 343 | 51598655 | 2957294 |
| Complete genome (Accession number NC_006156) | hypothetical protein BG0402 | 4114 18 | 4120 77 | + | 219 | 51598656 | 2957148 |
| Complete genome (Accession number NC_006156) | hypothetical protein BG0403 | 4120 87 | 4136 10 | - | 507 | 51598657 | 2957144 |
| Complete genome (Accession number NC_006156) | glutamate transporter, putative | 4137 44 | 4149 46 | + | 400 | 51598658 | 2957524 |
| Complete genome (Accession number NC_006156) | prolyl-tRNA synthetase | 4149 58 | 4164 24 | - | 488 | 51598659 | 2957143 |

| | | | | | | | |
|---|---|---|---|---|---|---|---|
| Complete genome (Accession number NC_006156) | hypothetical protein BG0406 | 4164 77 | 4170 46 | - | 189 | 51598660 | 2957880 |
| Complete genome (Accession number NC_006156) | hypothetical protein BG0407 | 4177 34 | 4183 45 | + | 203 | 51598661 | 2957426 |
| Complete genome (Accession number NC_006156) | hypothetical protein BG0408 | 4183 56 | 4189 67 | + | 203 | 51598662 | 2957865 |
| Complete genome (Accession number NC_006156) | mannose-6-phosphate isomerase | 4190 20 | 4201 35 | - | 371 | 51598663 | 2957881 |
| Complete genome (Accession number NC_006156) | PTS system, fructose-specific IIABC component | 4201 32 | 4220 09 | - | 625 | 51598664 | 2957530 |
| Complete genome (Accession number NC_006156) | hypothetical protein BG0411 | 4221 79 | 4228 08 | + | 209 | 51598665 | 2957868 |
| Complete genome (Accession number NC_006156) | endonuclease precursor | 4228 05 | 4236 86 | + | 293 | 51598666 | 2957869 |
| Complete genome (Accession number NC_006156) | hypothetical protein BG0413 | 4236 83 | 4244 62 | + | 259 | 51598667 | 2957542 |
| Complete genome (Accession number NC_006156) | hypothetical protein BG0414 | 4244 50 | 4250 70 | - | 206 | 51598668 | 2957543 |
| Complete genome (Accession number NC_006156) | hypothetical protein BG0415 | 4250 63 | 4251 25 | - | 20 | 51598669 | 2957871 |

| | | | | | | | |
|---|---|---|---|---|---|---|---|
| Complete genome (Accession number NC_006156) | chemotaxis protein methyltransferase | 4253 17 | 4261 74 | + | 285 | 51598670 | 2957886 |
| Complete genome (Accession number NC_006156) | hypothetical protein BG0417 | 4261 10 | 4261 93 | + | 27 | 51598671 | 2957385 |
| Complete genome (Accession number NC_006156) | protein-glutamate methylesterase | 4262 07 | 4273 34 | + | 375 | 51598672 | 2957875 |
| Complete genome (Accession number NC_006156) | pheromone shutdown protein | 4273 58 | 4285 72 | + | 404 | 51598673 | 2957888 |
| Complete genome (Accession number NC_006156) | adenylate kinase | 4286 11 | 4292 46 | + | 211 | 51598674 | 2957295 |
| Complete genome (Accession number NC_006156) | hypothetical protein BG0421 | 4292 69 | 4303 12 | + | 347 | 51598675 | 2957892 |
| Complete genome (Accession number NC_006156) | response regulatory protein | 4303 31 | 4312 54 | - | 307 | 51598676 | 2957915 |
| Complete genome (Accession number NC_006156) | sensory transduction histidine kinase/response regulator | 4312 57 | 4357 47 | - | 1496 | 51598677 | 2957342 |
| Complete genome (Accession number NC_006156) | hydrolase | 4423 57 | 4431 96 | - | 279 | 51598678 | 2957895 |
| Complete genome (Accession number NC_006156) | 3-methyladenine DNA glycosylase | 4433 30 | 4438 90 | - | 186 | 51598679 | 2957903 |

| | | | | | | | |
|---|---|---|---|---|---|---|---|
| Complete genome (Accession number NC_006156) | hypothetical protein BG0431 | 4456 10 | 4457 08 | - | 32 | 51598680 | 2957893 |
| Complete genome (Accession number NC_006156) | hypothetical protein BG0434 | 4485 22 | 4490 70 | - | 182 | 51598681 | 2957912 |
| Complete genome (Accession number NC_006156) | hypothetical protein BG0435 | 4491 65 | 4498 39 | - | 224 | 51598682 | 2957995 |
| Complete genome (Accession number NC_006156) | hypothetical protein BG0436 | 4500 48 | 4503 65 | + | 105 | 51598683 | 2957890 |
| Complete genome (Accession number NC_006156) | hypothetical protein BG0437 | 4503 95 | 4507 84 | - | 129 | 51598684 | 2957891 |
| Complete genome (Accession number NC_006156) | hypothetical protein BG0438 | 4509 02 | 4515 07 | + | 201 | 51598685 | 2957564 |
| Complete genome (Accession number NC_006156) | chromosome segregation protein, putative | 4516 16 | 4523 68 | + | 250 | 51598686 | 2957996 |
| Complete genome (Accession number NC_006156) | hypothetical protein BG0440 | 4523 71 | 4530 84 | + | 237 | 51598687 | 2957994 |
| Complete genome (Accession number NC_006156) | stage 0 sporulation protein J | 4533 53 | 4541 35 | - | 260 | 51598688 | 2957565 |
| Complete genome (Accession number NC_006156) | DNA gyrase, subunit A | 4542 54 | 4566 86 | - | 810 | 51598689 | 2957946 |
| Complete genome (Accession number NC_006156) | DNA gyrase, subunit B | 4566 98 | 4586 02 | - | 634 | 51598690 | 2957949 |

| | | | | | | | |
|---|---|---|---|---|---|---|---|
| Complete genome (Accession number NC_006156) | chromosomal replication initiator protein | 4587 89 | 4602 43 | + | 484 | 51598691 | 2957604 |
| Complete genome (Accession number NC_006156) | DNA polymerase III, subunit beta | 4604 84 | 4616 44 | + | 386 | 51598692 | 2957989 |
| Complete genome (Accession number NC_006156) | hypothetical protein BG0446 | 4617 37 | 4620 36 | + | 99 | 51598693 | 2957944 |
| Complete genome (Accession number NC_006156) | 50S ribosomal protein L34 | 4621 28 | 4622 83 | + | 51 | 51598694 | 2957566 |
| Complete genome (Accession number NC_006156) | ribonuclease P protein component | 4622 64 | 4625 99 | + | 111 | 51598695 | 2957951 |
| Complete genome (Accession number NC_006156) | putative inner membrane protein translocase component YidC | 4626 10 | 4642 44 | + | 544 | 51598696 | 2957947 |
| Complete genome (Accession number NC_006156) | spoIIIJ-associtated protein | 4642 57 | 4649 85 | + | 242 | 51598697 | 2957586 |
| Complete genome (Accession number NC_006156) | hypothetical protein BG0451 | 4649 12 | 4650 16 | + | 34 | 51598698 | 2957948 |
| Complete genome (Accession number NC_006156) | nucleotide sugar epimerase | 4650 31 | 4660 98 | + | 355 | 51598699 | 2957941 |
| Complete genome (Accession number NC_006156) | fructose-bisphosphate aldolase | 4662 60 | 4673 39 | + | 359 | 51598700 | 2957942 |

| | | | | | | | |
|---|---|---|---|---|---|---|---|
| Complete genome (Accession number NC_006156) | aspartyl-tRNA synthetase | 4677 93 | 4695 53 | - | 586 | 51598701 | 2957979 |
| Complete genome (Accession number NC_006156) | Na+/H+ antiporter | 4695 57 | 4716 62 | - | 701 | 51598702 | 2957954 |
| Complete genome (Accession number NC_006156) | phosphocarrier protein HPr | 4716 77 | 4719 40 | - | 87 | 51598703 | 2957583 |
| Complete genome (Accession number NC_006156) | hypothetical protein BG0457 | 4719 53 | 4722 46 | - | 97 | 51598704 | 2957970 |
| Complete genome (Accession number NC_006156) | RNA polymerase sigma-54 factor | 4722 36 | 4733 93 | - | 385 | 51598705 | 2957971 |
| Complete genome (Accession number NC_006156) | chromate transport protein, putative | 4734 98 | 4740 31 | - | 177 | 51598706 | 2957588 |
| Complete genome (Accession number NC_006156) | hypothetical protein BG0460 | 4740 28 | 4746 21 | - | 197 | 51598707 | 2957589 |
| Complete genome (Accession number NC_006156) | hypothetical protein BG0461 | 4745 90 | 4747 09 | - | 39 | 51598708 | 2957953 |
| Complete genome (Accession number NC_006156) | hypothetical protein BG0462 | 4748 40 | 4756 82 | + | 280 | 51598709 | 2957983 |
| Complete genome (Accession number NC_006156) | lipopolysaccharide biosynthesis-related protein | 4756 79 | 4768 27 | - | 382 | 51598710 | 2957584 |
| Complete genome (Accession number NC_006156) | hypothetical protein BG0464 | 4768 84 | 4778 73 | + | 329 | 51598711 | 2957976 |

| | | | | | | | |
|---|---|---|---|---|---|---|---|
| Complete genome (Accession number NC_006156) | hypothetical protein BG0465 | 4779 40 | 4785 18 | + | 192 | 51598712 | 2957937 |
| Complete genome (Accession number NC_006156) | excinuclease ABC, subunit C | 4785 90 | 4803 32 | - | 580 | 51598713 | 2957594 |
| Complete genome (Accession number NC_006156) | hypothetical protein BG0467 | 4805 23 | 4820 40 | + | 505 | 51598714 | 2957988 |
| Complete genome (Accession number NC_006156) | hypothetical protein BG0468 | 4820 33 | 4833 52 | + | 439 | 51598715 | 2957977 |
| Complete genome (Accession number NC_006156) | lipoprotein, putative | 4836 64 | 4843 68 | - | 234 | 51598716 | 2957602 |
| Complete genome (Accession number NC_006156) | DNA polymerase III subunits gamma and tau | 4849 51 | 4866 33 | + | 560 | 51598717 | 2957980 |
| Complete genome (Accession number NC_006156) | hypothetical protein BG0475 | 4866 38 | 4869 37 | + | 99 | 51598718 | 2957965 |
| Complete genome (Accession number NC_006156) | nucleoside-diphosphate kinase | 4871 09 | 4876 18 | + | 169 | 51598719 | 2957595 |
| Complete genome (Accession number NC_006156) | hypothetical protein BG0477 | 4878 89 | 4884 31 | + | 180 | 51598720 | 2957975 |
| Complete genome (Accession number NC_006156) | hypothetical protein BG0478 | 4884 28 | 4891 23 | + | 231 | 51598721 | 2957972 |

| | | | | | | | |
|---|---|---|---|---|---|---|---|
| Complete genome (Accession number NC_006156) | ABC transporter, ATP-binding protein | 4890 92 | 4898 92 | + | 266 | 51598722 | 2957603 |
| Complete genome (Accession number NC_006156) | hypothetical protein BG0480 | 4898 89 | 4905 78 | + | 229 | 51598723 | 2957955 |
| Complete genome (Accession number NC_006156) | hypothetical protein BG0481 | 4905 80 | 4913 26 | + | 248 | 51598724 | 2957973 |
| Complete genome (Accession number NC_006156) | signal peptidase II | 4913 37 | 4918 49 | + | 170 | 51598725 | 2957974 |
| Complete genome (Accession number NC_006156) | hypothetical protein BG0483 | 4920 85 | 4929 15 | + | 276 | 51598726 | 2957967 |
| Complete genome (Accession number NC_006156) | UDP-N-acetylglucosamine 1-carboxyvinyltransfe rase | 4930 43 | 4943 26 | + | 427 | 51598727 | 2957968 |
| Complete genome (Accession number NC_006156) | hypothetical protein BG0485 | 4949 22 | 4962 80 | + | 452 | 51598728 | 2957600 |
| Complete genome (Accession number NC_006156) | lipoprotein, putative | 4965 74 | 4967 05 | - | 43 | 51598729 | 2957601 |
| Complete genome (Accession number NC_006156) | elongation factor Tu | 4973 47 | 4985 31 | + | 394 | 51598730 | 2957964 |
| Complete genome (Accession number NC_006156) | 30S ribosomal protein S10 | 4985 83 | 4988 94 | + | 103 | 51598731 | 2957963 |
| Complete genome (Accession | 50S ribosomal protein L3 | 4989 35 | 4995 55 | + | 206 | 51598732 | 2957609 |

| | | | | | | | |
|---|---|---|---|---|---|---|---|
| number NC_006156) | | | | | | | |
| Complete genome (Accession number NC_006156) | 50S ribosomal protein L4 | 4995 65 | 5001 94 | + | 209 | 51598733 | 2957966 |
| Complete genome (Accession number NC_006156) | hypothetical protein BG0491 | 5001 84 | 5002 28 | + | 14 | 51598734 | 2957960 |
| Complete genome (Accession number NC_006156) | 50S ribosomal protein L23 | 5002 15 | 5005 11 | + | 98 | 51598735 | 2957617 |
| Complete genome (Accession number NC_006156) | 50S ribosomal protein L2 | 5005 33 | 5013 66 | + | 277 | 51598736 | 2957956 |
| Complete genome (Accession number NC_006156) | 30S ribosomal protein S19 | 5013 76 | 5016 54 | + | 92 | 51598737 | 2957742 |
| Complete genome (Accession number NC_006156) | 50S ribosomal protein L22 | 5016 23 | 5020 24 | + | 133 | 51598738 | 2957745 |
| Complete genome (Accession number NC_006156) | 30S ribosomal protein S3 | 5020 28 | 5029 06 | + | 292 | 51598739 | 2957824 |
| Complete genome (Accession number NC_006156) | 50S ribosomal protein L16 | 5029 11 | 5033 27 | + | 138 | 51598740 | 2957904 |
| Complete genome (Accession number NC_006156) | 50S ribosomal protein L29 | 5033 30 | 5035 30 | + | 66 | 51598741 | 2957898 |
| Complete genome (Accession number NC_006156) | 30S ribosomal protein S17 | 5035 33 | 5037 87 | + | 84 | 51598742 | 2957877 |

| | | | | | | | |
|---|---|---|---|---|---|---|---|
| Complete genome (Accession number NC_006156) | 50S ribosomal protein L14 | 5038 15 | 5041 83 | + | 122 | 51598743 | 2957221 |
| Complete genome (Accession number NC_006156) | 50S ribosomal protein L24 | 5041 98 | 5045 03 | + | 101 | 51598744 | 2957343 |
| Complete genome (Accession number NC_006156) | 50S ribosomal protein L5 | 5045 09 | 5050 57 | + | 182 | 51598745 | 2957434 |
| Complete genome (Accession number NC_006156) | 30S ribosomal protein S14 | 5050 75 | 5052 60 | + | 61 | 51598746 | 2957162 |
| Complete genome (Accession number NC_006156) | 30S ribosomal protein S8 | 5052 70 | 5056 68 | + | 132 | 51598747 | 2957577 |
| Complete genome (Accession number NC_006156) | 50S ribosomal protein L6 | 5056 86 | 5062 28 | + | 180 | 51598748 | 2957438 |
| Complete genome (Accession number NC_006156) | 50S ribosomal protein L18 | 5062 46 | 5066 05 | + | 119 | 51598749 | 2957548 |
| Complete genome (Accession number NC_006156) | 30S ribosomal protein S5 | 5066 18 | 5071 18 | + | 166 | 51598750 | 2957487 |
| Complete genome (Accession number NC_006156) | ribosomal protein L30 | 5071 22 | 5074 33 | + | 103 | 51598751 | 2957321 |
| Complete genome (Accession number NC_006156) | 50S ribosomal protein L15 | 5074 26 | 5078 63 | + | 145 | 51598752 | 2957597 |
| Complete genome (Accession number NC_006156) | preprotein translocase subunit SecY | 5078 76 | 5091 80 | + | 434 | 51598753 | 2957590 |

| | | | | | | | |
|---|---|---|---|---|---|---|---|
| Complete genome (Accession number NC_006156) | 50S ribosomal protein L36 | 5091 93 | 5093 06 | + | 37 | 51598754 | 2957550 |
| Complete genome (Accession number NC_006156) | 30S ribosomal protein S13 | 5093 24 | 5097 01 | + | 125 | 51598755 | 2957560 |
| Complete genome (Accession number NC_006156) | 30S ribosomal protein S11 | 5097 29 | 5101 21 | + | 130 | 51598756 | 2957610 |
| Complete genome (Accession number NC_006156) | DNA-directed RNA polymerase subunit alpha | 5101 37 | 5111 71 | + | 344 | 51598757 | 2957563 |
| Complete genome (Accession number NC_006156) | 50S ribosomal protein L17 | 5111 93 | 5115 64 | + | 123 | 51598758 | 2957618 |
| Complete genome (Accession number NC_006156) | hypothetical protein BG0516 | 5116 34 | 5131 66 | + | 510 | 51598759 | 2957599 |
| Complete genome (Accession number NC_006156) | hypothetical protein BG0517 | 5131 56 | 5139 44 | + | 262 | 51598760 | 2957627 |
| Complete genome (Accession number NC_006156) | hemolysin | 5139 97 | 5147 16 | + | 239 | 51598761 | 2957662 |
| Complete genome (Accession number NC_006156) | hypothetical protein BG0519 | 5147 13 | 5154 59 | - | 248 | 51598762 | 2957653 |
| Complete genome (Accession number NC_006156) | GTP-binding protein EngA | 5155 46 | 5168 47 | + | 433 | 51598763 | 2957651 |

| | | | | | | | |
|---|---|---|---|---|---|---|---|
| Complete genome (Accession number NC_006156) | hypothetical protein BG0521 | 5168 44 | 5181 03 | + | 419 | 51598764 | 2957690 |
| Complete genome (Accession number NC_006156) | hypothetical protein BG0522 | 5181 00 | 5187 86 | + | 228 | 51598765 | 2957691 |
| Complete genome (Accession number NC_006156) | hypothetical protein BG0523 | 5188 06 | 5252 94 | + | 2162 | 51598766 | 2957710 |
| Complete genome (Accession number NC_006156) | phenylalanyl-tRNA synthetase subunit alpha | 5253 06 | 5268 68 | + | 520 | 51598767 | 2957709 |
| Complete genome (Accession number NC_006156) | phenylalanyl-tRNA synthetase subunit beta | 5268 56 | 5285 56 | + | 566 | 51598768 | 2957705 |
| Complete genome (Accession number NC_006156) | thioredoxin reductase | 5286 27 | 5296 07 | + | 326 | 51598769 | 2957706 |
| Complete genome (Accession number NC_006156) | rRNA methylase | 5296 57 | 5303 97 | - | 246 | 51598770 | 2957718 |
| Complete genome (Accession number NC_006156) | heat shock protein | 5304 00 | 5314 94 | - | 364 | 51598771 | 2957722 |
| Complete genome (Accession number NC_006156) | molecular chaperone DnaK | 5314 94 | 5334 01 | - | 635 | 51598772 | 2957753 |
| Complete genome (Accession number NC_006156) | grpE protein | 5334 25 | 5339 88 | - | 187 | 51598773 | 2957794 |
| Complete genome (Accession number NC_006156) | NH(3)-dependent NAD+ synthetase | 5343 65 | 5359 03 | + | 512 | 51598774 | 2957720 |

| | | | | | | | |
|---|---|---|---|---|---|---|---|
| Complete genome (Accession number NC_006156) | hypothetical protein BG0532 | 5367 47 | 5368 42 | + | 31 | 51598775 | 2957783 |
| Complete genome (Accession number NC_006156) | inositol monophosphatase | 5371 67 | 5373 43 | + | 58 | 51598776 | 2957767 |
| Complete genome (Accession number NC_006156) | hypothetical protein BG0534 | 5373 70 | 5377 86 | - | 138 | 51598777 | 2957768 |
| Complete genome (Accession number NC_006156) | hypothetical protein BG0535 | 5379 27 | 5397 08 | + | 593 | 51598778 | 2957782 |
| Complete genome (Accession number NC_006156) | pantothenate kinase | 5397 01 | 5404 89 | + | 262 | 51598779 | 2957785 |
| Complete genome (Accession number NC_006156) | aldose reductase, putative | 5405 11 | 5414 58 | - | 315 | 51598780 | 2957784 |
| Complete genome (Accession number NC_006156) | hypothetical protein BG0538 | 5415 47 | 5423 56 | - | 269 | 51598781 | 2957786 |
| Complete genome (Accession number NC_006156) | hypothetical protein BG0539 | 5423 40 | 5429 54 | - | 204 | 51598782 | 2957788 |
| Complete genome (Accession number NC_006156) | hypothetical protein BG0540 | 5429 57 | 5442 52 | - | 431 | 51598783 | 2957789 |
| Complete genome (Accession number NC_006156) | phnP protein | 5444 46 | 5452 07 | + | 253 | 51598784 | 2957787 |
| Complete genome (Accession number NC_006156) | exodeoxyribonucle ase III | 5452 28 | 5459 95 | + | 255 | 51598785 | 2957798 |

| | | | | | | | |
|---|---|---|---|---|---|---|---|
| Complete genome (Accession number NC_006156) | hypothetical protein BG0543 | 5460 11 | 5467 84 | + | 257 | 51598786 | 2957793 |
| Complete genome (Accession number NC_006156) | zinc protease, putative | 5467 65 | 5495 66 | + | 933 | 51598787 | 2957799 |
| Complete genome (Accession number NC_006156) | hypothetical protein BG0547 | 5499 99 | 5506 43 | - | 214 | 51598788 | 2957837 |
| Complete genome (Accession number NC_006156) | hypothetical protein BG0548 | 5506 40 | 5509 96 | - | 118 | 51598789 | 2957809 |
| Complete genome (Accession number NC_006156) | hypothetical protein BG0549 | 5510 81 | 5517 79 | - | 232 | 51598790 | 2957812 |
| Complete genome (Accession number NC_006156) | elongation factor G | 5518 02 | 5538 83 | - | 693 | 51598791 | 2957985 |
| Complete genome (Accession number NC_006156) | hypothetical protein BG0551 | 5541 95 | 5544 22 | + | 75 | 51598792 | 2957990 |
| Complete genome (Accession number NC_006156) | hypothetical protein BG0552 | 5546 53 | 5551 80 | + | 175 | 51598793 | 2957991 |
| Complete genome (Accession number NC_006156) | hypothetical protein BG0553 | 5552 89 | 5559 39 | + | 216 | 51598794 | 2957935 |
| Complete genome (Accession number NC_006156) | phosphoribosylpyro phosphate synthetase | 5561 45 | 5573 65 | + | 406 | 51598795 | 2957913 |
| Complete genome (Accession number NC_006156) | xylulokinase | 5573 69 | 5587 36 | + | 455 | 51598796 | 2957906 |

| | | | | | | | |
|---|---|---|---|---|---|---|---|
| Complete genome (Accession number NC_006156) | hypothetical protein BG0556 | 5587 19 | 5594 02 | - | 227 | 51598797 | 2957909 |
| Complete genome (Accession number NC_006156) | dephospho-CoA kinase | 5593 86 | 5600 03 | - | 205 | 51598798 | 2957910 |
| Complete genome (Accession number NC_006156) | DNA polymerase I | 5599 85 | 5627 14 | - | 909 | 51598799 | 2957902 |
| Complete genome (Accession number NC_006156) | hypothetical protein BG0559 | 5627 11 | 5631 15 | - | 134 | 51598800 | 2957900 |
| Complete genome (Accession number NC_006156) | flagellar protein | 5631 05 | 5635 24 | - | 139 | 51598801 | 2957889 |
| Complete genome (Accession number NC_006156) | chemotaxis response regulator | 5635 33 | 5639 13 | - | 126 | 51598802 | 2957899 |
| Complete genome (Accession number NC_006156) | DNA ligase | 5641 60 | 5661 42 | + | 660 | 51598803 | 2957884 |
| Complete genome (Accession number NC_006156) | hypothetical protein BG0563 | 5661 67 | 5676 54 | - | 495 | 51598804 | 2957872 |
| Complete genome (Accession number NC_006156) | hypothetical protein BG0564 | 5679 38 | 5698 00 | + | 620 | 51598805 | 2957873 |
| Complete genome (Accession number NC_006156) | hypothetical protein BG0565 | 5697 87 | 5702 54 | + | 155 | 51598806 | 2957151 |
| Complete genome (Accession number NC_006156) | hypothetical protein BG0566 | 5702 47 | 5711 16 | + | 289 | 51598807 | 2957145 |

| Complete genome (Accession number NC_006156) | phosphocarrier protein HPr | 5712 54 | 5715 14 | + | 86 | 51598808 | 2957328 |
|---|---|---|---|---|---|---|---|
| Complete genome (Accession number NC_006156) | phosphoenolpyruvate-protein phosphatase | 5715 35 | 5732 56 | + | 573 | 51598809 | 2957389 |
| Complete genome (Accession number NC_006156) | glucose-specific PTS system component | 5732 59 | 5738 28 | + | 189 | 51598810 | 2957390 |
| Complete genome (Accession number NC_006156) | heat shock protein 90 | 5738 66 | 5757 16 | - | 616 | 51598811 | 2957327 |
| Complete genome (Accession number NC_006156) | 6-phosphogluconate dehydrogenase | 5758 29 | 5772 23 | + | 464 | 51598812 | 2957345 |
| Complete genome (Accession number NC_006156) | hypothetical protein BG0572 | 5772 54 | 5777 93 | - | 179 | 51598813 | 2957549 |
| Complete genome (Accession number NC_006156) | hypothetical protein BG0573 | 5779 47 | 5784 65 | - | 172 | 51598814 | 2957570 |
| Complete genome (Accession number NC_006156) | hypothetical protein BG0574 | 5785 47 | 5791 31 | - | 194 | 51598815 | 2957322 |
| Complete genome (Accession number NC_006156) | purine-binding chemotaxis protein | 5793 37 | 5798 79 | + | 180 | 51598816 | 2957323 |
| Complete genome (Accession number NC_006156) | hypothetical protein BG0576 | 5798 91 | 5802 02 | + | 103 | 51598817 | 2957551 |

| | | | | | | | |
|---|---|---|---|---|---|---|---|
| Complete genome (Accession number NC_006156) | chemotaxis histidine kinase | 5802 18 | 5823 59 | + | 713 | 51598818 | 2957620 |
| Complete genome (Accession number NC_006156) | protein-glutamate methylesterase | 5824 15 | 5835 72 | + | 385 | 51598819 | 2957569 |
| Complete genome (Accession number NC_006156) | hypothetical protein BG0579 | 5835 75 | 5853 47 | + | 590 | 51598820 | 2957652 |
| Complete genome (Accession number NC_006156) | chemotaxis response regulator | 5853 88 | 5857 62 | + | 124 | 51598821 | 2957571 |
| Complete genome (Accession number NC_006156) | uridylate kinase | 5861 19 | 5868 08 | - | 229 | 51598822 | 2957643 |
| Complete genome (Accession number NC_006156) | glycosyl transferase | 5870 26 | 5880 90 | + | 354 | 51598823 | 2957701 |
| Complete genome (Accession number NC_006156) | ABC transporter, ATP-binding protein | 5881 53 | 5889 59 | + | 268 | 51598824 | 2957614 |
| Complete genome (Accession number NC_006156) | hypothetical protein BG0585 | 5889 66 | 5898 17 | + | 283 | 51598825 | 2957694 |
| Complete genome (Accession number NC_006156) | CTP synthetase | 5900 07 | 5916 08 | + | 533 | 51598826 | 2957695 |
| Complete genome (Accession number NC_006156) | hypothetical protein BG0587 | 5916 25 | 5921 49 | - | 174 | 51598827 | 2957689 |
| Complete genome (Accession number NC_006156) | hypothetical protein BG0588 | 5922 85 | 5928 75 | + | 196 | 51598828 | 2957725 |

| | | | | | | | |
|---|---|---|---|---|---|---|---|
| Complete genome (Accession number NC_006156) | hypothetical protein BG0589 | 5931 07 | 5931 69 | + | 20 | 51598829 | 2957687 |
| Complete genome (Accession number NC_006156) | methyl-accepting chemotaxis protein | 5931 23 | 5942 95 | + | 390 | 51598830 | 2957726 |
| Complete genome (Accession number NC_006156) | DNA polymerase III subunit alpha | 5944 25 | 5978 68 | + | 1147 | 51598831 | 2957727 |
| Complete genome (Accession number NC_006156) | hypothetical protein BG0592 | 5978 77 | 5984 58 | + | 193 | 51598832 | 2957724 |
| Complete genome (Accession number NC_006156) | DNA recombinase | 5984 68 | 6005 28 | + | 686 | 51598833 | 2957735 |
| Complete genome (Accession number NC_006156) | carboxypeptidase, putative | 6005 25 | 6012 11 | - | 228 | 51598834 | 2957732 |
| Complete genome (Accession number NC_006156) | MATE efflux family protein | 6014 50 | 6027 87 | + | 445 | 51598835 | 2957728 |
| Complete genome (Accession number NC_006156) | hypothetical protein BG0596 | 6028 00 | 6029 97 | + | 65 | 51598836 | 2957733 |
| Complete genome (Accession number NC_006156) | hypothetical protein BG0597 | 6029 66 | 6043 21 | + | 451 | 51598837 | 2957734 |
| Complete genome (Accession number NC_006156) | UDP-N-acetylmuramoylalanine--D-glutamate ligase | 6043 34 | 6056 89 | + | 451 | 51598838 | 2957769 |
| Complete genome (Accession number | femA protein | 6056 86 | 6067 29 | - | 347 | 51598839 | 2957744 |

| | | | | | | | |
|---|---|---|---|---|---|---|---|
| NC_006156) | | | | | | | |
| Complete genome (Accession number NC_006156) | methionyl-tRNA synthetase | 6067 39 | 6089 13 | - | 724 | 51598840 | 2957737 |
| Complete genome (Accession number NC_006156) | 5-methylthioadenosine/S-adenosylhomocysteine nucleosidase, putative | 6090 72 | 6098 66 | + | 264 | 51598841 | 2957358 |
| Complete genome (Accession number NC_006156) | phosphate acetyltransferase | 6099 78 | 6110 15 | + | 345 | 51598842 | 2957781 |
| Complete genome (Accession number NC_006156) | dimethyladenosine transferase | 6109 98 | 6118 43 | - | 281 | 51598843 | 2957791 |
| Complete genome (Accession number NC_006156) | competence locus E, putative | 6125 18 | 6131 32 | - | 204 | 51598844 | 2957792 |
| Complete genome (Accession number NC_006156) | hypothetical protein BG0605 | 6132 49 | 6139 62 | + | 237 | 51598845 | 2957750 |
| Complete genome (Accession number NC_006156) | long-chain-fatty-acid CoA ligase | 6139 88 | 6159 25 | + | 645 | 51598846 | 2957801 |
| Complete genome (Accession number NC_006156) | arginyl-tRNA synthetase | 6159 54 | 6177 11 | - | 585 | 51598847 | 2957800 |
| Complete genome (Accession number NC_006156) | hypothetical protein BG0608 | 6177 77 | 6180 73 | + | 98 | 51598848 | 2957806 |

| Complete genome (Accession number NC_006156) | methyl-accepting chemotaxis protein | 6183 25 | 6204 72 | - | 715 | 51598849 | 2957807 |
|---|---|---|---|---|---|---|---|
| Complete genome (Accession number NC_006156) | methyl-accepting chemotaxis protein | 6207 41 | 6229 48 | + | 735 | 51598850 | 2957808 |
| Complete genome (Accession number NC_006156) | UDP-N-acetylmuramate dehydrogenase | 6229 45 | 6238 65 | - | 306 | 51598851 | 2957815 |
| Complete genome (Accession number NC_006156) | cysteinyl-tRNA synthetase | 6239 17 | 6253 59 | + | 480 | 51598852 | 2957814 |
| Complete genome (Accession number NC_006156) | hypothetical protein BG0613 | 6253 63 | 6269 34 | + | 523 | 51598853 | 2957817 |
| Complete genome (Accession number NC_006156) | serine hydroxymethyltrans ferase | 6269 34 | 6281 87 | + | 417 | 51598854 | 2957830 |
| Complete genome (Accession number NC_006156) | DnaJ domain containing protein | 6282 14 | 6289 66 | + | 250 | 51598855 | 2957828 |
| Complete genome (Accession number NC_006156) | membrane-associated protein p66 | 6290 26 | 6308 91 | - | 621 | 51598856 | 2957829 |
| Complete genome (Accession number NC_006156) | L-lactate permease | 6311 39 | 6326 47 | - | 502 | 51598857 | 2957833 |
| Complete genome (Accession number NC_006156) | serine-type D-Ala-D-Ala carboxypeptidase | 6327 19 | 6339 24 | + | 401 | 51598858 | 2957825 |
| Complete genome (Accession number NC_006156) | hypothetical protein BG0619 | 6339 41 | 6344 32 | + | 163 | 51598859 | 2957839 |

| | | | | | | | |
|---|---|---|---|---|---|---|---|
| Complete genome (Accession number NC_006156) | rep helicase, single-stranded DNA-dependent ATPase | 6344 35 | 6364 11 | + | 658 | 51598860 | 2957835 |
| Complete genome (Accession number NC_006156) | aminoacyl-histidine dipeptidase | 6365 26 | 6379 56 | + | 476 | 51598861 | 2957834 |
| Complete genome (Accession number NC_006156) | trigger factor | 6385 27 | 6398 85 | + | 452 | 51598862 | 2957844 |
| Complete genome (Accession number NC_006156) | ATP-dependent Clp protease proteolytic component | 6399 00 | 6404 84 | + | 194 | 51598863 | 2957855 |
| Complete genome (Accession number NC_006156) | ATP-dependent protease ATP-binding subunit | 6404 93 | 6418 00 | + | 435 | 51598864 | 2957854 |
| Complete genome (Accession number NC_006156) | ATP-dependent protease LA | 6417 69 | 6441 77 | + | 802 | 51598865 | 2957856 |
| Complete genome (Accession number NC_006156) | hypothetical protein BG0630 | 6442 11 | 6443 63 | + | 50 | 51598866 | 2957852 |
| Complete genome (Accession number NC_006156) | hypothetical protein BG0631 | 6442 93 | 6444 00 | + | 35 | 51598867 | 2957858 |
| Complete genome (Accession number NC_006156) | 30S ribosomal protein S4 | 6444 25 | 6450 51 | - | 208 | 51598868 | 2957863 |

| | | | | | | | |
|---|---|---|---|---|---|---|---|
| Complete genome (Accession number NC_006156) | hypothetical protein BG0634 | 6452 28 | 6465 89 | - | 453 | 51598869 | 2957843 |
| Complete genome (Accession number NC_006156) | hypothetical protein BG0635 | 6467 10 | 6470 78 | + | 122 | 51598870 | 2957846 |
| Complete genome (Accession number NC_006156) | cytidine deaminase | 6471 01 | 6475 41 | - | 146 | 51598871 | 2957847 |
| Complete genome (Accession number NC_006156) | hypothetical protein BG0637 | 6476 62 | 6486 24 | + | 320 | 51598872 | 2957816 |
| Complete genome (Accession number NC_006156) | beta-glucosidase, putative | 6486 02 | 6501 97 | - | 531 | 51598873 | 2957848 |
| Complete genome (Accession number NC_006156) | 4-methyl-5(b-hydroxyethyl)-thiazole monophosphate biosynthesis protein | 6504 96 | 6510 50 | + | 184 | 51598874 | 2957822 |
| Complete genome (Accession number NC_006156) | acetate kinase | 6511 05 | 6523 22 | - | 405 | 51598875 | 2957780 |
| Complete genome (Accession number NC_006156) | transcription-repair coupling factor | 6523 47 | 6557 21 | - | 1124 | 51598876 | 2957821 |
| Complete genome (Accession number NC_006156) | hypothetical protein BG0643 | 6558 18 | 6567 80 | + | 320 | 51598877 | 2957795 |

| | | | | | | | |
|---|---|---|---|---|---|---|---|
| Complete genome (Accession number NC_006156) | N-acetylmuramoyl-L-alanine amidase, putative | 6567 49 | 6588 36 | + | 695 | 51598878 | 2957778 |
| Complete genome (Accession number NC_006156) | small primase-like protein | 6589 19 | 6594 67 | - | 182 | 51598879 | 2957779 |
| Complete genome (Accession number NC_006156) | hypothetical protein BG0647 | 6594 36 | 6594 86 | - | 16 | 51598880 | 2957746 |
| Complete genome (Accession number NC_006156) | putative aminopeptidase 2 | 6594 70 | 6607 41 | - | 423 | 51598881 | 2957290 |
| Complete genome (Accession number NC_006156) | hypothetical protein BG0649 | 6608 25 | 6615 53 | + | 242 | 51598882 | 2957501 |
| Complete genome (Accession number NC_006156) | PTS system, fructose-specific IIABC component | 6615 81 | 6634 46 | - | 621 | 51598883 | 2957502 |
| Complete genome (Accession number NC_006156) | 1-phosphofructokinase | 6635 36 | 6644 59 | + | 307 | 51598884 | 2957749 |
| Complete genome (Accession number NC_006156) | hypothetical protein BG0652 | 6645 34 | 6648 48 | - | 104 | 51598885 | 2957372 |
| Complete genome (Accession number NC_006156) | exodeoxyribonuclease V, alpha chain | 6651 16 | 6669 48 | - | 610 | 51598886 | 2957522 |
| Complete genome (Accession number NC_006156) | exodeoxyribonuclease V, beta chain | 6669 45 | 6704 51 | - | 1168 | 51598887 | 2957523 |

| Complete genome (Accession number NC_006156) | exodeoxyribonucle ase V, gamma chain | 6704 57 | 6736 96 | - | 1079 | 51598888 | 2957299 |
|---|---|---|---|---|---|---|---|
| Complete genome (Accession number NC_006156) | nicotinate phosphoribosyltran sferase | 6737 00 | 6751 45 | - | 481 | 51598889 | 2957368 |
| Complete genome (Accession number NC_006156) | glucose-6-phosphate 1-dehydrogenase | 6752 58 | 6766 94 | + | 478 | 51598890 | 2957173 |
| Complete genome (Accession number NC_006156) | Na+/H+ antiporter | 6769 56 | 6782 99 | + | 447 | 51598891 | 2957305 |
| Complete genome (Accession number NC_006156) | hypothetical protein BG0660 | 6782 47 | 6783 33 | - | 28 | 51598892 | 2957483 |
| Complete genome (Accession number NC_006156) | Na+/H+ antiporter | 6783 99 | 6797 54 | + | 451 | 51598893 | 2957484 |
| Complete genome (Accession number NC_006156) | spermidine/putresci ne ABC transporter, binding periplasmic protein | 6798 03 | 6808 49 | - | 348 | 51598894 | 2957462 |
| Complete genome (Accession number NC_006156) | spermidine/putresci ne ABC transporter, permease protein | 6808 69 | 6816 60 | - | 263 | 51598895 | 2957491 |
| Complete genome (Accession number NC_006156) | spermidine/putresci ne ABC transporter, permease protein | 6816 63 | 6824 72 | - | 269 | 51598896 | 2957380 |

| | | | | | | | |
|---|---|---|---|---|---|---|---|
| Complete genome (Accession number NC_006156) | spermidine/putrescine ABC transporter, ATP-binding protein | 6824 72 | 6835 15 | - | 347 | 51598897 | 2957525 |
| Complete genome (Accession number NC_006156) | ribosomal biogenesis GTPase | 6835 94 | 6844 33 | - | 279 | 1,62E+08 | 2957366 |
| Complete genome (Accession number NC_006156) | N-acetylmannosamine-6-phosphate 2-epimerase | 6845 98 | 6852 96 | + | 232 | 51598899 | 2957774 |
| Complete genome (Accession number NC_006156) | PTS system, glucose-specific IIBC component | 6853 44 | 6868 88 | + | 514 | 51598900 | 2957775 |
| Complete genome (Accession number NC_006156) | hydrolase, alpha/beta fold family | 6869 16 | 6878 99 | - | 327 | 51598901 | 2957711 |
| Complete genome (Accession number NC_006156) | ferric uptake regulation protein | 6878 96 | 6884 23 | - | 175 | 51598902 | 2957776 |
| Complete genome (Accession number NC_006156) | serine/threonine kinase, putative | 6885 21 | 6902 09 | - | 562 | 51598903 | 2957224 |
| Complete genome (Accession number NC_006156) | chaperonin GroEL | 6904 00 | 6920 37 | + | 545 | 51598904 | 2957762 |
| Complete genome (Accession number NC_006156) | hypothetical protein BG0673 | 6921 05 | 6924 01 | + | 98 | 51598905 | 2957765 |

| | | | | | | | |
|---|---|---|---|---|---|---|---|
| Complete genome (Accession number NC_006156) | hypothetical protein BG0674 | 6924 16 | 6927 30 | + | 104 | 51598906 | 2957766 |
| Complete genome (Accession number NC_006156) | preprotein translocase subunit SecD | 6928 08 | 6945 68 | + | 586 | 1,62E+08 | 2957759 |
| Complete genome (Accession number NC_006156) | preprotein translocase subunit SecF | 6945 52 | 6954 51 | + | 299 | 51598908 | 2957773 |
| Complete genome (Accession number NC_006156) | hypothetical protein BG0677 | 6954 77 | 6966 19 | + | 380 | 51598909 | 2957755 |
| Complete genome (Accession number NC_006156) | heat shock protein | 6966 16 | 6974 46 | - | 276 | 51598910 | 2957721 |
| Complete genome (Accession number NC_006156) | HemN-related protein | 6975 55 | 6986 88 | + | 377 | 51598911 | 2957760 |
| Complete genome (Accession number NC_006156) | ribose 5-phosphate isomerase | 6986 75 | 6993 61 | - | 228 | 51598912 | 2957761 |
| Complete genome (Accession number NC_006156) | phosphoglycerate mutase | 6995 08 | 7002 54 | + | 248 | 51598913 | 2957699 |
| Complete genome (Accession number NC_006156) | lysyl-tRNA synthetase | 7003 21 | 7018 86 | - | 521 | 51598914 | 2957658 |
| Complete genome (Accession number NC_006156) | GTP-binding protein Era | 7019 59 | 7028 31 | - | 290 | 51598915 | 2957697 |
| Complete genome (Accession number NC_006156) | hypothetical protein BG0684 | 7029 37 | 7032 93 | + | 118 | 51598916 | 2957688 |

| | | | | | | | |
|---|---|---|---|---|---|---|---|
| Complete genome (Accession number NC_006156) | hypothetical protein BG0685 | 7033 03 | 7037 13 | + | 136 | 51598917 | 2957665 |
| Complete genome (Accession number NC_006156) | hypothetical protein BG0686 | 7037 75 | 7041 82 | + | 135 | 51598918 | 2957666 |
| Complete genome (Accession number NC_006156) | hypothetical protein BG0687 | 7041 91 | 7048 80 | - | 229 | 51598919 | 2957664 |
| Complete genome (Accession number NC_006156) | hypothetical protein BG0688 | 7049 23 | 7058 82 | + | 319 | 51598920 | 2957661 |
| Complete genome (Accession number NC_006156) | hypothetical protein BG0689 | 7058 66 | 7068 73 | + | 335 | 51598921 | 2957698 |
| Complete genome (Accession number NC_006156) | hypothetical protein BG0690 | 7068 63 | 7074 14 | + | 183 | 51598922 | 2958007 |
| Complete genome (Accession number NC_006156) | flagellar filament outer layer protein | 7074 92 | 7085 17 | + | 341 | 51598923 | 2958008 |
| Complete genome (Accession number NC_006156) | chemotaxis histidine kinase | 7085 60 | 7111 81 | + | 873 | 51598924 | 2957663 |
| Complete genome (Accession number NC_006156) | purine-binding chemotaxis protein | 7111 89 | 7125 92 | + | 467 | 51598925 | 2957408 |
| Complete genome (Accession number NC_006156) | chemotaxis operon protein | 7126 04 | 7130 89 | + | 161 | 51598926 | 2957625 |
| Complete genome (Accession number NC_006156) | chemotaxis response regulator | 7131 31 | 7135 71 | + | 146 | 51598927 | 2957598 |

| | | | | | | | |
|---|---|---|---|---|---|---|---|
| Complete genome (Accession number NC_006156) | hypothetical protein BG0696 | 7136 11 | 7141 23 | - | 170 | 51598928 | 2958001 |
| Complete genome (Accession number NC_006156) | hypothetical protein BG0697 | 7141 20 | 7151 66 | - | 348 | 51598929 | 2958002 |
| Complete genome (Accession number NC_006156) | hypothetical protein BG0698 | 7151 60 | 7160 23 | - | 287 | 51598930 | 2958003 |
| Complete genome (Accession number NC_006156) | phosphoglycolate phosphatase | 7160 20 | 7166 82 | - | 220 | 51598931 | 2958004 |
| Complete genome (Accession number NC_006156) | ribose/galactose ABC transporter, ATP-binding protein | 7169 30 | 7185 40 | + | 536 | 51598932 | 2958005 |
| Complete genome (Accession number NC_006156) | ribose/galactose ABC transporter, permease protein | 7185 41 | 7196 92 | + | 383 | 51598933 | 2957921 |
| Complete genome (Accession number NC_006156) | ribose/galactose ABC transporter, permease protein | 7196 64 | 7205 90 | + | 308 | 51598934 | 2957925 |
| Complete genome (Accession number NC_006156) | methyl-accepting chemotaxis protein | 7207 47 | 7230 08 | + | 753 | 51598935 | 2957919 |
| Complete genome (Accession number NC_006156) | methyl-accepting chemotaxis protein | 7230 38 | 7249 39 | + | 633 | 51598936 | 2957999 |

| | | | | | | | |
|---|---|---|---|---|---|---|---|
| Complete genome (Accession number NC_006156) | tRNA (5-methylaminomethyl-2-thiouridylate)-methyltransferase | 7249 82 | 7260 49 | - | 355 | 51598937 | 2958000 |
| Complete genome (Accession number NC_006156) | 3-hydroxy-3-methylglutaryl-CoA synthase | 7261 43 | 7273 66 | + | 407 | 51598938 | 2957920 |
| Complete genome (Accession number NC_006156) | isopentenyl pyrophosphate isomerase | 7273 50 | 7284 14 | + | 354 | 51598939 | 2957917 |
| Complete genome (Accession number NC_006156) | 3-hydroxy-3-methylglutaryl-CoA reductase | 7284 04 | 7296 78 | + | 424 | 51598940 | 2957918 |
| Complete genome (Accession number NC_006156) | mevalonate pyrophosphate decarboxylase | 7296 56 | 7305 94 | + | 312 | 51598941 | 2957923 |
| Complete genome (Accession number NC_006156) | phosphomevalonate kinase, putative | 7305 85 | 7315 38 | + | 317 | 51598942 | 2957924 |
| Complete genome (Accession number NC_006156) | mevalonate kinase, putative | 7315 32 | 7324 25 | + | 297 | 51598943 | 2957929 |
| Complete genome (Accession number NC_006156) | hypothetical protein BG0713 | 7325 49 | 7330 16 | - | 155 | 51598944 | 2957757 |
| Complete genome (Accession number NC_006156) | neutrophil activating protein | 7331 01 | 7336 31 | + | 176 | 51598945 | 2957758 |
| Complete genome (Accession | elongation factor G | 7337 12 | 7357 21 | + | 669 | 51598946 | 2957723 |

| | | | | | | | |
|---|---|---|---|---|---|---|---|
| number NC_006156) | | | | | | | |
| Complete genome (Accession number NC_006156) | xylose operon regulatory protein | 7359 98 | 7372 06 | + | 402 | 51598947 | 2957756 |
| Complete genome (Accession number NC_006156) | signal recognition particle protein | 7372 69 | 7386 03 | + | 444 | 51598948 | 2957764 |
| Complete genome (Accession number NC_006156) | 30S ribosomal protein S16 | 7386 16 | 7388 76 | + | 86 | 51598949 | 2957378 |
| Complete genome (Accession number NC_006156) | hypothetical protein BG0719 | 7388 77 | 7391 25 | + | 82 | 51598950 | 2957460 |
| Complete genome (Accession number NC_006156) | hypothetical protein BG0720 | 7391 29 | 7396 29 | + | 166 | 51598951 | 2957461 |
| Complete genome (Accession number NC_006156) | tRNA (guanine-N(1)-)-methyltransferase | 7396 26 | 7403 45 | + | 239 | 51598952 | 2957212 |
| Complete genome (Accession number NC_006156) | 50S ribosomal protein L19 | 7403 23 | 7406 88 | + | 121 | 51598953 | 2957247 |
| Complete genome (Accession number NC_006156) | hypothetical protein BG0723 | 7410 39 | 7415 78 | + | 179 | 51598954 | 2957166 |
| Complete genome (Accession number NC_006156) | lipopolysaccharide biosynthesis-related protein | 7415 80 | 7420 71 | + | 163 | 51598955 | 2957752 |
| Complete genome (Accession number NC_006156) | 50S ribosomal protein L32 | 7421 34 | 7423 16 | + | 60 | 51598956 | 2957446 |

| | | | | | | | |
|---|---|---|---|---|---|---|---|
| Complete genome (Accession number NC_006156) | acyl carrier protein | 7423 40 | 7425 82 | + | 80 | 51598957 | 2957804 |
| Complete genome (Accession number NC_006156) | ribonuclease III | 7425 82 | 7433 19 | + | 245 | 51598958 | 2957805 |
| Complete genome (Accession number NC_006156) | polynucleotide adenylyltransferase | 7432 90 | 7445 22 | - | 410 | 51598959 | 2957818 |
| Complete genome (Accession number NC_006156) | hypothetical protein BG0729 | 7446 39 | 7464 11 | + | 590 | 51598960 | 2957849 |
| Complete genome (Accession number NC_006156) | hypothetical protein BG0730 | 7464 17 | 7467 40 | + | 107 | 51598961 | 2957853 |
| Complete genome (Accession number NC_006156) | hypothetical protein BG0732 | 7471 99 | 7482 30 | + | 343 | 51598962 | 2957851 |
| Complete genome (Accession number NC_006156) | DNA primase | 7482 36 | 7500 17 | + | 593 | 51598963 | 2957838 |
| Complete genome (Accession number NC_006156) | RNA polymerase sigma factor RpoD | 7500 21 | 7519 16 | + | 631 | 51598964 | 2957841 |
| Complete genome (Accession number NC_006156) | hypothetical protein BG0735 | 7519 30 | 7526 88 | + | 252 | 51598965 | 2957826 |
| Complete genome (Accession number NC_006156) | hypothetical protein BG0736 | 7530 93 | 7540 61 | + | 322 | 51598966 | 2957819 |
| Complete genome (Accession number NC_006156) | rod shape-determining protein | 7540 78 | 7551 21 | + | 347 | 51598967 | 2957820 |

| | | | | | | | |
|---|---|---|---|---|---|---|---|
| Complete genome (Accession number NC_006156) | rod shape-determining protein | 7551 25 | 7559 70 | + | 281 | 51598968 | 2957810 |
| Complete genome (Accession number NC_006156) | hypothetical protein BG0739 | 7559 70 | 7564 52 | + | 160 | 51598969 | 2957802 |
| Complete genome (Accession number NC_006156) | penicillin-binding protein | 7564 52 | 7582 51 | + | 599 | 51598970 | 2957790 |
| Complete genome (Accession number NC_006156) | rod shape-determining protein | 7582 56 | 7595 72 | + | 438 | 51598971 | 2957748 |
| Complete genome (Accession number NC_006156) | threonyl-tRNA synthetase | 7597 39 | 7614 87 | + | 582 | 51598972 | 2957754 |
| Complete genome (Accession number NC_006156) | CDP-diacylglycerol-glycerol-3-phosphate 3-phosphatidyltransferase | 7614 89 | 7621 18 | + | 209 | 51598973 | 2957730 |
| Complete genome (Accession number NC_006156) | hypothetical protein BG0744 | 7621 05 | 7635 50 | + | 481 | 51598974 | 2957731 |
| Complete genome (Accession number NC_006156) | adenylyl cyclase, CyaB-type, putative | 7635 24 | 7640 54 | - | 176 | 51598975 | 2957714 |
| Complete genome (Accession number NC_006156) | K+ transport protein | 7641 54 | 7654 73 | + | 439 | 51598976 | 2957704 |
| Complete genome (Accession number NC_006156) | hypothetical protein BG0747 | 7654 70 | 7662 61 | + | 263 | 51598977 | 2957654 |

| Complete genome (Accession number NC_006156) | minD-related ATP-binding protein | 7662 71 | 7672 42 | + | 323 | 51598978 | 2957648 |
|---|---|---|---|---|---|---|---|
| Complete genome (Accession number NC_006156) | diphosphate--fructose-6-phosphate 1-phosphotransferas e | 7672 73 | 7686 16 | + | 447 | 51598979 | 2957649 |
| Complete genome (Accession number NC_006156) | coenzyme A disulfide reductase | 7686 33 | 7699 64 | - | 443 | 51598980 | 2957553 |
| Complete genome (Accession number NC_006156) | glutamate transporter | 7699 79 | 7713 70 | - | 463 | 51598981 | 2957580 |
| Complete genome (Accession number NC_006156) | glucose-6-phosphate isomerase | 7714 37 | 7730 29 | - | 530 | 51598982 | 2957575 |
| Complete genome (Accession number NC_006156) | hypothetical protein BG0753 | 7730 52 | 7734 53 | - | 133 | 51598983 | 2957508 |
| Complete genome (Accession number NC_006156) | penicillin-binding protein | 7734 60 | 7762 46 | - | 928 | 51598984 | 2957538 |
| Complete genome (Accession number NC_006156) | hypothetical protein BG0755 | 7765 43 | 7773 28 | + | 261 | 51598985 | 2957505 |
| Complete genome (Accession number NC_006156) | hypothetical protein BG0756 | 7775 35 | 7785 48 | - | 337 | 51598986 | 2957506 |
| Complete genome (Accession number NC_006156) | rare lipoprotein A | 7786 58 | 7794 46 | + | 262 | 51598987 | 2957494 |

| | | | | | | | |
|---|---|---|---|---|---|---|---|
| Complete genome (Accession number NC_006156) | histidine phosphokinase/pho phatase, putative | 7796 39 | 7806 28 | + | 329 | 51598988 | 2957335 |
| Complete genome (Accession number NC_006156) | valyl-tRNA synthetase | 7806 33 | 7832 60 | + | 875 | 51598989 | 2957319 |
| Complete genome (Accession number NC_006156) | hypothetical protein BG0760 | 7833 54 | 7839 50 | - | 198 | 51598990 | 2957281 |
| Complete genome (Accession number NC_006156) | hypothetical protein BG0761 | 7840 28 | 7847 05 | - | 225 | 51598991 | 2957231 |
| Complete genome (Accession number NC_006156) | co-chaperonin GroES | 7850 24 | 7852 96 | - | 90 | 51598992 | 2957232 |
| Complete genome (Accession number NC_006156) | ABC transporter, ATP-binding protein | 7854 40 | 7870 71 | + | 543 | 51598993 | 2957883 |
| Complete genome (Accession number NC_006156) | hypothetical protein BG0764 | 7871 31 | 7887 86 | + | 551 | 51598994 | 2957862 |
| Complete genome (Accession number NC_006156) | antigen, p83/100 | 7888 92 | 7909 73 | + | 693 | 51598995 | 2957934 |
| Complete genome (Accession number NC_006156) | endonuclease III | 7912 99 | 7919 16 | + | 205 | 51598996 | 2957958 |
| Complete genome (Accession number NC_006156) | oligopeptide ABC transporter, permease protein | 7919 26 | 7927 86 | - | 286 | 51598997 | 2957959 |

| | | | | | | | |
|---|---|---|---|---|---|---|---|
| Complete genome (Accession number NC_006156) | oligopeptide ABC transporter, permease protein | 7927 86 | 7937 66 | - | 326 | 51598998 | 2957987 |
| Complete genome (Accession number NC_006156) | hypothetical protein BG0771 | 7937 72 | 7942 30 | - | 152 | 51598999 | 2957936 |
| Complete genome (Accession number NC_006156) | hypothetical protein BG0772 | 7943 29 | 7954 80 | + | 383 | 51599000 | 2957911 |
| Complete genome (Accession number NC_006156) | hypothetical protein BG0773 | 7957 25 | 7967 38 | - | 337 | 51599001 | 2957907 |
| Complete genome (Accession number NC_006156) | hypothetical protein BG0774 | 7967 55 | 7982 63 | - | 502 | 51599002 | 2957908 |
| Complete genome (Accession number NC_006156) | membrane spanning protein, putative | 7982 68 | 7990 02 | - | 244 | 51599003 | 2957932 |
| Complete genome (Accession number NC_006156) | ABC transporter, ATP-binding protein | 7990 05 | 7999 28 | - | 307 | 51599004 | 2957905 |
| Complete genome (Accession number NC_006156) | ribonuclease Z | 7999 37 | 8008 96 | - | 319 | 51599005 | 2957857 |
| Complete genome (Accession number NC_006156) | hypothetical protein BG0778 | 8010 87 | 8021 54 | - | 355 | 51599006 | 2957878 |
| Complete genome (Accession number NC_006156) | ATP-dependent Clp protease proteolytic subunit | 8023 53 | 8029 49 | + | 198 | 51599007 | 2957879 |

EP 2 254 592 B1

| | | | | | | | |
|---|---|---|---|---|---|---|---|
| Complete genome (Accession number NC_006156) | hypothetical protein BG0780 | 8030 27 | 8034 04 | + | 125 | 51599008 | 2957885 |
| Complete genome (Accession number NC_006156) | lipoprotein, putative | 8033 74 | 8036 61 | + | 95 | 51599009 | 2957867 |
| Complete genome (Accession number NC_006156) | hypothetical protein BG0783 | 8038 24 | 8046 78 | - | 284 | 51599010 | 2957258 |
| Complete genome (Accession number NC_006156) | hypothetical protein BG0784 | 8047 18 | 8050 47 | - | 109 | 51599011 | 2957192 |
| Complete genome (Accession number NC_006156) | hypothetical protein BG0785 | 8051 59 | 8060 43 | + | 294 | 51599012 | 2957876 |
| Complete genome (Accession number NC_006156) | response regulatory protein | 8062 88 | 8076 46 | - | 452 | 51599013 | 2957250 |
| Complete genome (Accession number NC_006156) | sensory transduction histidine kinase, putative | 8076 49 | 8087 97 | - | 382 | 51599014 | 2957251 |
| Complete genome (Accession number NC_006156) | hypothetical protein BG0788 | 8087 94 | 8098 40 | - | 348 | 51599015 | 2957241 |
| Complete genome (Accession number NC_006156) | colicin V production protein, putative | 8098 51 | 8103 39 | - | 162 | 51599016 | 2957210 |

| Complete genome (Accession number NC_006156) | UDP-N-acetylglucosamine--N-acetylmuramyl-(pentapeptide) pyrophosphoryl-undecaprenol N-acetylglucosamine transferase | 8103 36 | 8114 27 | - | 363 | 51599017 | 2957493 |
|---|---|---|---|---|---|---|---|
| Complete genome (Accession number NC_006156) | pyridoxal kinase | 8115 80 | 8123 74 | - | 264 | 51599018 | 2957223 |
| Complete genome (Accession number NC_006156) | O-sialoglycoprotein endopeptidase | 8123 74 | 8133 90 | - | 338 | 51599019 | 2957312 |
| Complete genome (Accession number NC_006156) | hypothetical protein BG0793 | 8133 87 | 8142 53 | - | 288 | 51599020 | 2957313 |
| Complete genome (Accession number NC_006156) | RNA polymerase sigma factor | 8143 04 | 8151 04 | - | 266 | 51599021 | 2957292 |
| Complete genome (Accession number NC_006156) | hypothetical protein BG0795 | 8152 49 | 8155 69 | - | 106 | 51599022 | 2957316 |
| Complete genome (Accession number NC_006156) | flagellar basal body P-ring protein | 8155 59 | 8165 54 | - | 331 | 51599023 | 2957211 |
| Complete genome (Accession number NC_006156) | hypothetical protein BG0797 | 8167 24 | 8171 58 | - | 144 | 51599024 | 2957383 |

| Complete genome (Accession number NC_006156) | flagellar basal body rod protein FlgG | 8173 58 | 8181 55 | - | 265 | 51599025 | 2957469 |
|---|---|---|---|---|---|---|---|
| Complete genome (Accession number NC_006156) | flagellar hook-basal body complex protein | 8181 68 | 8190 16 | - | 282 | 51599026 | 2957470 |
| Complete genome (Accession number NC_006156) | hypothetical protein BG0800 | 8192 71 | 8198 31 | + | 186 | 51599027 | 2957346 |
| Complete genome (Accession number NC_006156) | adenine phosphoribosyltran sferase | 8198 93 | 8204 23 | + | 176 | 51599028 | 2957397 |
| Complete genome (Accession number NC_006156) | 50S ribosomal protein L21 | 8204 81 | 8207 92 | + | 103 | 51599029 | 2957581 |
| Complete genome (Accession number NC_006156) | hypothetical protein BG0803 | 8207 98 | 8211 18 | + | 106 | 51599030 | 2957402 |
| Complete genome (Accession number NC_006156) | 50S ribosomal protein L27 | 8211 18 | 8213 63 | + | 81 | 51599031 | 2957486 |
| Complete genome (Accession number NC_006156) | GTPase ObgE | 8214 08 | 8223 97 | + | 329 | 51599032 | 2957458 |
| Complete genome (Accession number NC_006156) | hypothetical protein BG0806 | 8224 84 | 8230 77 | + | 197 | 51599033 | 2957459 |
| Complete genome (Accession number NC_006156) | hypothetical protein BG0807 | 8230 62 | 8242 46 | + | 394 | 51599034 | 2957429 |
| Complete genome (Accession number | hypothetical protein BG0808 | 8242 21 | 8245 71 | + | 116 | 51599035 | 2957407 |

| | | | | | | | |
|---|---|---|---|---|---|---|---|
| NC_006156) | | | | | | | |
| Complete genome (Accession number NC_006156) | regulatory protein SpoVG | 8246 75 | 8249 68 | + | 97 | 51599036 | 2957432 |
| Complete genome (Accession number NC_006156) | 50S ribosomal protein L25/general stress protein Ctc | 8251 25 | 8256 73 | + | 182 | 51599037 | 2957552 |
| Complete genome (Accession number NC_006156) | peptidyl-tRNA hydrolase | 8256 87 | 8262 44 | + | 185 | 51599038 | 2957492 |
| Complete genome (Accession number NC_006156) | hypothetical protein BG0813 | 8262 44 | 8275 66 | + | 440 | 51599039 | 2957363 |
| Complete genome (Accession number NC_006156) | cell division protein | 8275 63 | 8294 82 | + | 639 | 51599040 | 2957561 |
| Complete genome (Accession number NC_006156) | hypothetical protein BG0815 | 8294 95 | 8309 76 | + | 493 | 51599041 | 2957562 |
| Complete genome (Accession number NC_006156) | thymidine kinase | 8310 84 | 8321 87 | + | 367 | 51599042 | 2957555 |
| Complete genome (Accession number NC_006156) | hypothetical protein BG0817 | 8321 17 | 8322 09 | - | 30 | 51599043 | 2957556 |
| Complete genome (Accession number NC_006156) | hypothetical protein BG0818 | 8321 84 | 8328 13 | - | 209 | 51599044 | 2957554 |
| Complete genome (Accession number | thymidylate kinase | 8328 27 | 8334 35 | - | 202 | 51599045 | 2957587 |

| | | | | | | | |
|---|---|---|---|---|---|---|---|
| NC_006156) | | | | | | | |
| Complete genome (Accession number NC_006156) | hypothetical protein BG0820 | 8335 41 | 8379 41 | + | 1466 | 51599046 | 2957557 |
| Complete genome (Accession number NC_006156) | outer membrane protein | 8379 59 | 8404 24 | + | 821 | 51599047 | 2957558 |
| Complete genome (Accession number NC_006156) | hypothetical protein BG0822 | 8404 46 | 8409 79 | + | 177 | 51599048 | 2957546 |
| Complete genome (Accession number NC_006156) | DNA mismatch repair protein | 8411 69 | 8437 57 | + | 862 | 51599049 | 2957568 |
| Complete genome (Accession number NC_006156) | competence protein F, putative | 8437 91 | 8443 03 | + | 170 | 51599050 | 2957559 |
| Complete genome (Accession number NC_006156) | hypothetical protein BG0825 | 8444 76 | 8449 13 | + | 145 | 51599051 | 2957572 |
| Complete genome (Accession number NC_006156) | transcription elongation factor NusA | 8449 27 | 8463 75 | + | 482 | 51599052 | 2957573 |
| Complete genome (Accession number NC_006156) | translation initiation factor IF-2 | 8463 78 | 8490 29 | + | 883 | 51599053 | 2957567 |
| Complete genome (Accession number NC_006156) | ribosome-binding factor A | 8490 53 | 8494 15 | + | 120 | 51599054 | 2957574 |
| Complete genome (Accession number NC_006156) | tRNA pseudouridine 55 synthase | 8494 18 | 8502 66 | + | 282 | 51599055 | 2957576 |

| | | | | | | | |
|---|---|---|---|---|---|---|---|
| Complete genome (Accession number NC_006156) | 30S ribosomal protein S15 | 8504 36 | 8507 02 | + | 88 | 51599056 | 2957593 |
| Complete genome (Accession number NC_006156) | polynucleotide phosphorylase/poly adenylase | 8507 35 | 8528 82 | + | 715 | 51599057 | 2957582 |
| Complete genome (Accession number NC_006156) | hypothetical protein BG0832 | 8528 54 | 8544 01 | + | 515 | 51599058 | 2957591 |
| Complete genome (Accession number NC_006156) | hypothetical protein BG0833 | 8543 76 | 8556 65 | + | 429 | 51599059 | 2957592 |
| Complete genome (Accession number NC_006156) | hypothetical protein BG0834 | 8556 62 | 8567 29 | + | 355 | 51599060 | 2957621 |
| Complete genome (Accession number NC_006156) | tRNA-guanine transglycosylase | 8567 59 | 8578 86 | + | 375 | 51599061 | 2957596 |
| Complete genome (Accession number NC_006156) | virulence factor mviN protein | 8578 79 | 8593 99 | + | 506 | 51599062 | 2957608 |
| Complete genome (Accession number NC_006156) | hypothetical protein BG0837 | 8593 96 | 8608 02 | + | 468 | 51599063 | 2957606 |
| Complete genome (Accession number NC_006156) | pantothenate metabolism flavoprotein | 8608 28 | 8618 89 | - | 353 | 51599064 | 2957628 |
| Complete genome (Accession number NC_006156) | sodium/panthothen ate symporter | 8623 27 | 8636 61 | + | 444 | 51599065 | 2957629 |

| | | | | | | | |
|---|---|---|---|---|---|---|---|
| Complete genome (Accession number NC_006156) | hypothetical protein BG0840 | 8636 74 | 8646 21 | + | 315 | 51599066 | 2957622 |
| Complete genome (Accession number NC_006156) | hypothetical protein BG0841 | 8645 96 | 8655 37 | + | 313 | 51599067 | 2957630 |
| Complete genome (Accession number NC_006156) | UDP-N-acetylmuramate--L-alanine ligase | 8655 27 | 8669 33 | + | 468 | 51599068 | 2957638 |
| Complete genome (Accession number NC_006156) | hypothetical protein BG0843 | 8669 44 | 8678 13 | + | 289 | 51599069 | 2957686 |
| Complete genome (Accession number NC_006156) | cytidylate kinase | 8678 10 | 8683 46 | + | 178 | 51599070 | 2957682 |
| Complete genome (Accession number NC_006156) | hypothetical protein BG0845 | 8683 86 | 8685 86 | + | 66 | 51599071 | 2957683 |
| Complete genome (Accession number NC_006156) | 2-methylthio-N6-isopentyladenosine tRNA modification enzyme | 8685 73 | 8694 93 | + | 306 | 51599072 | 2957636 |
| Complete genome (Accession number NC_006156) | hypothetical protein BG0847 | 8696 35 | 8700 06 | - | 123 | 51599073 | 2957696 |
| Complete genome (Accession number NC_006156) | hypothetical protein BG0848 | 8700 87 | 8706 32 | + | 181 | 51599074 | 2957707 |
| Complete genome (Accession number NC_006156) | hypothetical protein BG0849 | 8706 25 | 8712 72 | - | 215 | 51599075 | 2957702 |

| | | | | | | | |
|---|---|---|---|---|---|---|---|
| Complete genome (Accession number NC_006156) | hypothetical protein BG0850 | 8713 31 | 8714 53 | + | 40 | 51599076 | 2957703 |
| Complete genome (Accession number NC_006156) | hypothetical protein BG0851 | 8714 00 | 8718 10 | - | 136 | 51599077 | 2957693 |
| Complete genome (Accession number NC_006156) | ATP-dependent helicase | 8720 44 | 8745 18 | - | 824 | 51599078 | 2957708 |
| Complete genome (Accession number NC_006156) | DNA topoisomerase I | 8745 21 | 8770 70 | - | 849 | 51599079 | 2957712 |
| Complete genome (Accession number NC_006156) | exonuclease SbcD | 8771 45 | 8783 86 | + | 413 | 51599080 | 2957717 |
| Complete genome (Accession number NC_006156) | exonuclease SbcC | 8783 73 | 8812 19 | + | 948 | 51599081 | 2957715 |
| Complete genome (Accession number NC_006156) | xylose operon regulatory protein | 8812 53 | 8821 88 | + | 311 | 51599082 | 2957716 |
| Complete genome (Accession number NC_006156) | lipoprotein, putative | 8822 08 | 8830 32 | - | 274 | 51599083 | 2957713 |
| Complete genome (Accession number NC_006156) | isoleucyl-tRNA synthetase | 8830 26 | 8861 54 | - | 1042 | 51599084 | 2957738 |
| Complete genome (Accession number NC_006156) | ATP-dependent Clp protease, subunit C | 8861 69 | 8883 88 | - | 739 | 51599085 | 2957740 |
| Complete genome (Accession number NC_006156) | phosphomannomut ase | 8884 11 | 8901 20 | - | 569 | 51599086 | 2957729 |

| | | | | | | | |
|---|---|---|---|---|---|---|---|
| Complete genome (Accession number NC_006156) | excinuclease ABC subunit B | 8902 70 | 8922 64 | + | 664 | 51599087 | 2957739 |
| Complete genome (Accession number NC_006156) | excinuclease ABC, subunit A | 8922 81 | 8951 33 | + | 950 | 51599088 | 2957772 |
| Complete genome (Accession number NC_006156) | hypothetical protein BG0863 | 8951 36 | 8985 37 | + | 1133 | 51599089 | 2957770 |
| Complete genome (Accession number NC_006156) | hypothetical protein BG0864 | 8985 03 | 8985 59 | + | 18 | 51599090 | 2957771 |
| Complete genome (Accession number NC_006156) | hypothetical protein BG0865 | 8985 46 | 8990 91 | - | 181 | 51599091 | 2957741 |
| Complete genome (Accession number NC_006156) | hypothetical protein BG0866 | 8990 69 | 8996 68 | - | 199 | 51599092 | 2957743 |
| Complete genome (Accession number NC_006156) | lipoprotein, putative | 8996 78 | 9013 00 | - | 540 | 51599093 | 2957796 |
| Complete genome (Accession number NC_006156) | arginine deiminase | 9014 81 | 9027 10 | + | 409 | 51599094 | 2957797 |
| Complete genome (Accession number NC_006156) | ornithine carbamoyltransferase, catabolic | 9027 82 | 9037 65 | + | 327 | 51599095 | 2957777 |
| Variable plasmid segment G1a19c04.r1 (Accession number NT_108239) | hypothetical protein BGP227 | 431 | 874 | + | 147 | 56561105 | 3189711 |
| Variable plasmid segment G1a19c04.r1 (Accession number | hypothetical protein BGP228 | 1203 | 1631 | - | 142 | 56561106 | 3189707 |

| | | | | | | | |
|---|---|---|---|---|---|---|---|
| NT_108239) | | | | | | | |
| Variable plasmid segment G1a19c04.r1 (Accession number NT_108239) | hypothetical protein BGP229 | 1600 | 1689 | - | 29 | 56561107 | 3189708 |
| Variable plasmid segment G1a19c04.r1 (Accession number NT_108239) | hypothetical protein BGP230 | 1690 | 2106 | - | 138 | 56561108 | 3189710 |
| Variable plasmid segment G1a19c04.r1 (Accession number NT_108239) | hypothetical protein BGP231 | 2116 | 2316 | - | 66 | 56561109 | 3189714 |
| Variable plasmid segment G1a19c04.r1 (Accession number NT_108239) | hypothetical protein BGP232 | 2304 | 2771 | - | 155 | 56561110 | 3189704 |
| Variable plasmid segment G1a19c04.r1 (Accession number NT_108239) | hypothetical protein BGP233 | 2966 | 3055 | - | 29 | 56561111 | 3189705 |
| Variable plasmid segment G1a19c04.r1 (Accession number NT_108239) | hypothetical protein BGP234 | 3742 | 3858 | - | 38 | 56561112 | 3189709 |
| Variable plasmid segment G1a19c04.r1 (Accession number NT_108239) | hypothetical protein BGP235 | 3946 | 4299 | - | 117 | 56561113 | 3189706 |
| Variable plasmid segment G1a19c04.r1 (Accession number NT_108239) | hypothetical protein BGP236 | 4312 | 4710 | - | 132 | 56561114 | 3189715 |
| Variable plasmid segment G1a19c04.r1 (Accession | hypothetical protein BGP237 | 4921 | 5076 | - | 51 | 56561115 | 3189713 |

| | | | | | | | |
|---|---|---|---|---|---|---|---|
| number NT_108239) | | | | | | | |
| Variable plasmid segment G1a19c04.r1 (Accession number NT_108239) | hypothetical protein BGP238 | 5037 | 5762 | - | 241 | 56561116 | 3189712 |
| Variable plasmid segment G11a15f12.s1 (Accession number NT_108263) | hypothetical protein BGP334 | 74 | 286 | + | 70 | 56561236 | 3189985 |
| Variable plasmid segment G11a15f12.s1 (Accession number NT_108263) | hypothetical protein BGP335 | 635 | 1195 | - | 186 | 56561237 | 3189987 |
| Variable plasmid segment G11a15f12.s1 (Accession number NT_108263) | hypothetical protein BGP336 | 1210 | 1695 | - | 161 | 56561238 | 3189986 |
| Variable plasmid segment G11a15f12.s1 (Accession number NT_108263) | hypothetical protein BGP337 | 1902 | 1970 | - | 22 | 56561239 | 3189984 |
| Variable plasmid segment G1M1b11b03.s1( Accession number NT_108262) | hypothetical protein BGP332 | 1 | 365 | - | 122 | 56561233 | 3189982 |
| Variable plasmid segment G1M1b11b03.s1( Accession number NT_108262) | hypothetical protein BGP333 | 795 | 1928 | + | 377 | 56561234 | 3189983 |
| Variable plasmid segment G1M1b15c06.r1 (Accession number NT_108261) | hypothetical protein BGP329 | 3 | 488 | + | 161 | 56561229 | 3189981 |
| Variable plasmid segment G1M1b15c06.r1 (Accession | hypothetical protein BGP330 | 1652 | 1984 | + | 110 | 56561230 | 3189979 |

| | | | | | | | |
|---|---|---|---|---|---|---|---|
| number NT_108261) | | | | | | | |
| Variable plasmid segment G1M1b15c06.r1 (Accession number NT_108261) | hypothetical protein BGP331 | 2120 | 2242 | + | 40 | 56561231 | 3189980 |
| Variable plasmid segment G11a14d08.s1 (Accession number NT_108260) | hypothetical protein BGP326 | 1 | 852 | - | 284 | 56561225 | 3189978 |
| Variable plasmid segment G11a14d08.s1 (Accession number NT_108260) | hypothetical protein BGP327 | 849 | 1415 | - | 188 | 56561226 | 3189977 |
| Variable plasmid segment G11a14d08.s1 (Accession number NT_108260) | hypothetical protein BGP328 | 1604 | 1858 | - | 84 | 56561227 | 3189976 |
| Variable plasmid segment PBil1a03e01.s1 (Accession number NT_108259) | hypothetical protein BGP325 | 845 | 1096 | + | 83 | 56561223 | 3189975 |
| Variable plasmid segment G1a13d03.r1 (Accession number NT_108258) | hypothetical protein BGP321 | 1 | 203 | - | 67 | 56561218 | 3189973 |
| Variable plasmid segment G1a13d03.r1 (Accession number NT_108258) | hypothetical protein BGP322 | 370 | 990 | - | 206 | 56561219 | 3189974 |
| Variable plasmid segment G1a13d03.r1 (Accession number NT_108258) | hypothetical protein BGP323 | 1088 | 1843 | - | 251 | 56561220 | 3189972 |
| Variable plasmid segment G1a13d03.r1 (Accession | hypothetical protein BGP324 | 1816 | 1986 | - | 56 | 56561221 | 3189971 |

| | | | | | | | |
|---|---|---|---|---|---|---|---|
| number NT_108258) | | | | | | | |
| Variable plasmid segment G1Mb19g09.r1 (Accession number NT_108257) | hypothetical protein BGP320 | 1700 | 1819 | + | 39 | 56561216 | 3189970 |
| Variable plasmid segment G11a19d04.r1 (Accession number NT_108256) | hypothetical protein BGP317 | 531 | 644 | + | 37 | 56561212 | 3189967 |
| Variable plasmid segment G11a19d04.r1 (Accession number NT_108256) | hypothetical protein BGP318 | 960 | 1049 | + | 29 | 56561213 | 3189968 |
| Variable plasmid segment G11a19d04.r1 (Accession number NT_108256) | hypothetical protein BGP319 | 2065 | 2438 | + | 124 | 56561214 | 3189969 |
| Variable plasmid segment G1a13d06.s1 (Accession number NT_108255) | hypothetical protein BGP316 | 1 | 2417 | - | 806 | 56561210 | 3189966 |
| Variable plasmid segment G1a18e10.s1 (Accession number NT_108254) | hypothetical protein BGP312 | 60 | 719 | + | 219 | 56561205 | 3189965 |
| Variable plasmid segment G1a18e10.s1 (Accession number NT_108254) | hypothetical protein BGP313 | 759 | 1280 | + | 173 | 56561206 | 3189962 |
| Variable plasmid segment G1a18e10.s1 (Accession number NT_108254) | hypothetical protein BGP314 | 1292 | 1693 | + | 133 | 56561207 | 3189964 |
| Variable plasmid segment G1a18e10.s1 (Accession | hypothetical protein BGP315 | 2408 | 2543 | + | 45 | 56561208 | 3189963 |

| | | | | | | | |
|---|---|---|---|---|---|---|---|
| number NT_108254) | | | | | | | |
| Variable plasmid segment G1a22b07.r1 (Accession number NT_108253) | hypothetical protein BGP309 | 123 | 239 | + | 38 | 56561201 | 3189960 |
| Variable plasmid segment G1a22b07.r1 (Accession number NT_108253) | hypothetical protein BGP310 | 695 | 1249 | - | 184 | 56561202 | 3189961 |
| Variable plasmid segment G1a22b07.r1 (Accession number NT_108253) | hypothetical protein BGP311 | 1559 | 2380 | + | 273 | 56561203 | 3189959 |
| Variable plasmid segment G11a15h12.r1 (Accession number NT_108252) | hypothetical protein BGP303 | 405 | 1022 | - | 205 | 56561194 | 3189958 |
| Variable plasmid segment G11a15h12.r1 (Accession number NT_108252) | hypothetical protein BGP304 | 1035 | 1544 | - | 169 | 56561195 | 3189957 |
| Variable plasmid segment G11a15h12.r1 (Accession number NT_108252) | hypothetical protein BGP305 | 2190 | 2342 | - | 50 | 56561196 | 3189953 |
| Variable plasmid segment G11a15h12.r1 (Accession number NT_108252) | hypothetical protein BGP306 | 2348 | 2509 | - | 53 | 56561197 | 3189954 |
| Variable plasmid segment G11a15h12.r1 (Accession number NT_108252) | hypothetical protein BGP307 | 2532 | 2705 | - | 57 | 56561198 | 3189955 |
| Variable plasmid segment G11a15h12.r1 (Accession | hypothetical protein BGP308 | 3059 | 3140 | - | 26 | 56561199 | 3189956 |

| | | | | | | | |
|---|---|---|---|---|---|---|---|
| number NT_108252) | | | | | | | |
| Variable plasmid segment G1a18d09.s1 (Accession number NT_108251) | hypothetical protein BGP296 | 237 | 359 | + | 40 | 56561186 | 3189951 |
| Variable plasmid segment G1a18d09.s1 (Accession number NT_108251) | hypothetical protein BGP297 | 389 | 811 | + | 140 | 56561187 | 3189949 |
| Variable plasmid segment G1a18d09.s1 (Accession number NT_108251) | hypothetical protein BGP298 | 774 | 1088 | + | 104 | 56561188 | 3189788 |
| Variable plasmid segment G1a18d09.s1 (Accession number NT_108251) | hypothetical protein BGP299 | 1098 | 1511 | + | 137 | 56561189 | 3189948 |
| Variable plasmid segment G1a18d09.s1 (Accession number NT_108251) | hypothetical protein BGP300 | 1630 | 2370 | + | 246 | 56561190 | 3189787 |
| Variable plasmid segment G1a18d09.s1 (Accession number NT_108251) | hypothetical protein BGP301 | 2411 | 2953 | + | 180 | 56561191 | 3189950 |
| Variable plasmid segment G1a18d09.s1 (Accession number NT_108251) | hypothetical protein BGP302 | 2966 | 3140 | + | 58 | 56561192 | 3189952 |
| Variable plasmid segment G1a07g11.r1 (Accession number NT_108250) | hypothetical protein BGP294 | 435 | 2342 | - | 635 | 56561183 | 3189786 |
| Variable plasmid segment G1a07g11.r1 (Accession | hypothetical protein BGP295 | 2580 | 3029 | - | 149 | 56561184 | 3189785 |

| | | | | | | | |
|---|---|---|---|---|---|---|---|
| number NT_108250) | | | | | | | |
| Variable plasmid segment G1Mb28a10.r1 (Accession number NT_108249) | hypothetical protein BGP291 | 320 | 946 | + | 208 | 56561179 | 3189784 |
| Variable plasmid segment G1Mb28a10.r1 (Accession number NT_108249) | hypothetical protein BGP292 | 1023 | 1970 | - | 315 | 56561180 | 3189782 |
| Variable plasmid segment G1Mb28a10.r1 (Accession number NT_108249) | hypothetical protein BGP293 | 2358 | 3380 | + | 341 | 56561181 | 3189783 |
| Variable plasmid segment G1a23g09.r1 (Accession number NT_108248) | hypothetical protein BGP287 | 226 | 777 | + | 183 | 56561174 | 3189780 |
| Variable plasmid segment G1a23g09.r1 (Accession number NT_108248) | hypothetical protein BGP288 | 1065 | 1388 | - | 107 | 56561175 | 3189781 |
| Variable plasmid segment G1a23g09.r1 (Accession number NT_108248) | hypothetical protein BGP289 | 2136 | 2675 | - | 179 | 56561176 | 3189779 |
| Variable plasmid segment G1a23g09.r1 (Accession number NT_108248) | hypothetical protein BGP290 | 3009 | 3236 | - | 75 | 56561177 | 3189778 |
| Variable plasmid segment PBilM1b05c04.r1 (Accession number NT_108247) | hypothetical protein BGP283 | 392 | 547 | + | 51 | 56561169 | 3189763 |
| Variable plasmid segment PBilM1b05c04.r1 (Accession | hypothetical protein BGP284 | 630 | 1139 | + | 169 | 56561170 | 3189762 |

| | | | | | | | |
|---|---|---|---|---|---|---|---|
| number NT_108247) | | | | | | | |
| Variable plasmid segment PBiIM1b05c04.r1 (Accession number NT_108247) | hypothetical protein BGP285 | 2210 | 2392 | - | 60 | 56561171 | 3189761 |
| Variable plasmid segment PBiIM1b05c04.r1 (Accession number NT_108247) | hypothetical protein BGP286 | 2699 | 2860 | + | 53 | 56561172 | 3189760 |
| Variable plasmid segment G1M1b09a03.r1 (Accession number NT_108246) | hypothetical protein BGP278 | 1 | 187 | - | 62 | 56561163 | 3189759 |
| Variable plasmid segment G1M1b09a03.r1 (Accession number NT_108246) | hypothetical protein BGP279 | 191 | 388 | - | 65 | 56561164 | 3189758 |
| Variable plasmid segment G1M1b09a03.r1 (Accession number NT_108246) | hypothetical protein BGP280 | 731 | 1276 | - | 181 | 56561165 | 3189756 |
| Variable plasmid segment G1M1b09a03.r1 (Accession number NT_108246) | hypothetical protein BGP281 | 1286 | 1594 | - | 102 | 56561166 | 3189755 |
| Variable plasmid segment G1M1b09a03.r1 (Accession number NT_108246) | hypothetical protein BGP282 | 1563 | 2039 | - | 158 | 56561167 | 3189757 |
| Variable plasmid segment G1a10e03.r1 (Accession number NT_108245) | hypothetical protein BGP277 | 1 | 72 | + | 23 | 56561152 | 3189745 |
| Variable plasmid segment G1a10e03.r1 (Accession | hypothetical protein BGP276 | 38 | 682 | - | 214 | 56561153 | 3189752 |

| | | | | | | | |
|---|---|---|---|---|---|---|---|
| number NT_108245) | | | | | | | |
| Variable plasmid segment G1a10e03.r1 (Accession number NT_108245) | hypothetical protein BGP275 | 1246 | 1410 | + | 54 | 56561154 | 3189749 |
| Variable plasmid segment G1a10e03.r1 (Accession number NT_108245) | hypothetical protein BGP274 | 1420 | 1548 | + | 42 | 56561155 | 3189747 |
| Variable plasmid segment G1a10e03.r1 (Accession number NT_108245) | hypothetical protein BGP273 | 2070 | 2186 | + | 38 | 56561156 | 3189754 |
| Variable plasmid segment G1a10e03.r1 (Accession number NT_108245) | hypothetical protein BGP272 | 2137 | 2280 | - | 47 | 56561157 | 3189750 |
| Variable plasmid segment G1a10e03.r1 (Accession number NT_108245) | hypothetical protein BGP271 | 2228 | 2662 | - | 144 | 56561158 | 3189753 |
| Variable plasmid segment G1a10e03.r1 (Accession number NT_108245) | hypothetical protein BGP270 | 2824 | 3306 | + | 160 | 56561159 | 3189746 |
| Variable plasmid segment G1a10e03.r1 (Accession number NT_108245) | hypothetical protein BGP269 | 3592 | 3798 | - | 68 | 56561160 | 3189748 |
| Variable plasmid segment G1a10e03.r1 (Accession number NT_108245) | hypothetical protein BGP268 | 3791 | 4050 | - | 85 | 56561161 | 3189751 |
| Variable plasmid segment G1Mb12b07.s1 (Accession | hypothetical protein BGP262 | 98 | 433 | + | 111 | 56561145 | 3189742 |

| | | | | | | | |
|---|---|---|---|---|---|---|---|
| number NT_108244) | | | | | | | |
| Variable plasmid segment G1Mb12b07.s1 (Accession number NT_108244) | hypothetical protein BGP263 | 417 | 1067 | + | 216 | 56561146 | 3189739 |
| Variable plasmid segment G1Mb12b07.s1 (Accession number NT_108244) | hypothetical protein BGP264 | 1356 | 1541 | - | 61 | 56561147 | 3189743 |
| Variable plasmid segment G1Mb12b07.s1 (Accession number NT_108244) | hypothetical protein BGP265 | 1959 | 2210 | + | 83 | 56561148 | 3189744 |
| Variable plasmid segment G1Mb12b07.s1 (Accession number NT_108244) | hypothetical protein BGP266 | 2367 | 2861 | + | 164 | 56561149 | 3189741 |
| Variable plasmid segment G1Mb12b07.s1 (Accession number NT_108244) | hypothetical protein BGP267 | 3835 | 4017 | - | 60 | 56561150 | 3189740 |
| Variable plasmid segment G1a25a05.r1 (Accession number NT_108243) | hypothetical protein BGP257 | 84 | 1193 | + | 369 | 56561139 | 3189737 |
| Variable plasmid segment G1a25a05.r1 (Accession number NT_108243) | hypothetical protein BGP258 | 1246 | 1671 | + | 141 | 56561140 | 3189738 |
| Variable plasmid segment G1a25a05.r1 (Accession number NT_108243) | hypothetical protein BGP259 | 1677 | 2453 | - | 258 | 56561141 | 3189734 |
| Variable plasmid segment G1a25a05.r1 (Accession | hypothetical protein BGP260 | 2885 | 3568 | + | 227 | 56561142 | 3189735 |

| | | | | | | | |
|---|---|---|---|---|---|---|---|
| number NT_108243) | | | | | | | |
| Variable plasmid segment G1a25a05.r1 (Accession number NT_108243) | hypothetical protein BGP261 | 3620 | 4649 | + | 343 | 56561143 | 3189736 |
| Variable plasmid segment G11a16g12.s1 (Accession number NT_108242) | hypothetical protein BGP253 | 3193 | 3768 | - | 191 | 56561134 | 3189731 |
| Variable plasmid segment G11a16g12.s1 (Accession number NT_108242) | hypothetical protein BGP254 | 3791 | 4549 | - | 252 | 56561135 | 3189730 |
| Variable plasmid segment G11a16g12.s1 (Accession number NT_108242) | hypothetical protein BGP255 | 4525 | 4821 | - | 98 | 56561136 | 3189733 |
| Variable plasmid segment G11a16g12.s1 (Accession number NT_108242) | hypothetical protein BGP256 | 5002 | 5088 | - | 28 | 56561137 | 3189732 |
| Variable plasmid segment G11a14a06.r1 (Accession number NT_108241) | hypothetical protein BGP247 | 242 | 322 | - | 26 | 56561127 | 3189726 |
| Variable plasmid segment G11a14a06.r1 (Accession number NT_108241) | hypothetical protein BGP248 | 1108 | 1905 | + | 265 | 56561128 | 3189724 |
| Variable plasmid segment G11a14a06.r1 (Accession number NT_108241) | hypothetical protein BGP249 | 2017 | 2244 | + | 75 | 56561129 | 3189728 |
| Variable plasmid segment G11a14a06.r1 (Accession | hypothetical protein BGP250 | 2989 | 3123 | - | 44 | 56561130 | 3189729 |

| | | | | | | | |
|---|---|---|---|---|---|---|---|
| number NT_108241) | | | | | | | |
| Variable plasmid segment G11a14a06.r1 (Accession number NT_108241) | hypothetical protein BGP251 | 3473 | 4315 | - | 280 | 56561131 | 3189725 |
| Variable plasmid segment G11a14a06.r1 (Accession number NT_108241) | hypothetical protein BGP252 | 5334 | 5558 | + | 74 | 56561132 | 3189727 |
| Variable plasmid segment G1Mb03b12.r1 (Accession number NT_108238) | hypothetical protein BGP223 | 236 | 3274 | + | 1012 | 56561100 | 3189703 |
| Variable plasmid segment G1Mb03b12.r1 (Accession number NT_108238) | hypothetical protein BGP224 | 3315 | 4643 | + | 442 | 56561101 | 3189700 |
| Variable plasmid segment G1Mb03b12.r1 (Accession number NT_108238) | hypothetical protein BGP225 | 4633 | 4965 | + | 110 | 56561102 | 3189701 |
| Variable plasmid segment G1Mb03b12.r1 (Accession number NT_108238) | hypothetical protein BGP226 | 4943 | 5982 | + | 347 | 56561103 | 3189702 |
| Variable plasmid segment G11a16d07.r1 (Accession number NT_108237) | hypothetical protein BGP216 | 108 | 1019 | + | 303 | 56561092 | 3189697 |
| Variable plasmid segment G11a16d07.r1 (Accession number NT_108237) | hypothetical protein BGP217 | 1029 | 1583 | + | 184 | 56561093 | 3189699 |
| Variable plasmid segment G11a16d07.r1 (Accession | hypothetical protein BGP218 | 1559 | 2311 | + | 250 | 56561094 | 3189694 |

| | | | | | | | |
|---|---|---|---|---|---|---|---|
| number NT_108237) | | | | | | | |
| Variable plasmid segment G11a16d07.r1 (Accession number NT_108237) | hypothetical protein BGP219 | 2370 | 2939 | + | 189 | 56561095 | 3189693 |
| Variable plasmid segment G11a16d07.r1 (Accession number NT_108237) | hypothetical protein BGP220 | 3806 | 4621 | - | 271 | 56561096 | 3189696 |
| Variable plasmid segment G11a16d07.r1 (Accession number NT_108237) | hypothetical protein BGP221 | 5841 | 5978 | - | 45 | 56561097 | 3189695 |
| Variable plasmid segment G11a16d07.r1 (Accession number NT_108237) | hypothetical protein BGP222 | 6089 | 6265 | - | 58 | 56561098 | 3189698 |
| Variable plasmid segment G1a09h07.r1 (Accession number NT_108236) | hypothetical protein BGP207 | 69 | 530 | - | 153 | 56561082 | 3189685 |
| Variable plasmid segment G1a09h07.r1 (Accession number NT_108236) | hypothetical protein BGP208 | 686 | 1012 | - | 108 | 56561083 | 3189688 |
| Variable plasmid segment G1a09h07.r1 (Accession number NT_108236) | hypothetical protein BGP209 | 1081 | 1257 | + | 58 | 56561084 | 3189686 |
| Variable plasmid segment G1a09h07.r1 (Accession number NT_108236) | hypothetical protein BGP210 | 1164 | 2117 | - | 317 | 56561085 | 3189687 |
| Variable plasmid segment G1a09h07.r1 (Accession | hypothetical protein BGP211 | 2345 | 2923 | + | 192 | 56561086 | 3189684 |

| | | | | | | | |
|---|---|---|---|---|---|---|---|
| number NT_108236) | | | | | | | |
| Variable plasmid segment G1a09h07.r1 (Accession number NT_108236) | hypothetical protein BGP212 | 3069 | 3752 | - | 227 | 56561087 | 3189690 |
| Variable plasmid segment G1a09h07.r1 (Accession number NT_108236) | hypothetical protein BGP213 | 4580 | 5242 | - | 220 | 56561088 | 3189689 |
| Variable plasmid segment G1a09h07.r1 (Accession number NT_108236) | hypothetical protein BGP214 | 6192 | 6932 | + | 246 | 56561089 | 3189691 |
| Variable plasmid segment G1a09h07.r1 (Accession number NT_108236) | hypothetical protein BGP215 | 6923 | 7198 | + | 91 | 56561090 | 3189692 |
| Variable plasmid segment G1a07d05.r1 (Accession number NT_108235) | hypothetical protein BGP195 | 41 | 181 | + | 46 | 56561069 | 3189677 |
| Variable plasmid segment G1a07d05.r1 (Accession number NT_108235) | hypothetical protein BGP196 | 181 | 303 | + | 40 | 56561070 | 3189678 |
| Variable plasmid segment G1a07d05.r1 (Accession number NT_108235) | hypothetical protein BGP197 | 506 | 778 | + | 90 | 56561071 | 3189865 |
| Variable plasmid segment G1a07d05.r1 (Accession number NT_108235) | hypothetical protein BGP198 | 726 | 953 | + | 75 | 56561072 | 3189675 |
| Variable plasmid segment G1a07d05.r1 (Accession | hypothetical protein BGP199 | 2140 | 5724 | - | 1194 | 56561073 | 3189683 |

| number NT_108235) | | | | | | | |
|---|---|---|---|---|---|---|---|
| Variable plasmid segment G1a07d05.r1 (Accession number NT_108235) | hypothetical protein BGP200 | 5840 | 6196 | - | 118 | 56561074 | 3190011 |
| Variable plasmid segment G1a07d05.r1 (Accession number NT_108235) | hypothetical protein BGP201 | 6141 | 6332 | - | 63 | 56561075 | 3189674 |
| Variable plasmid segment G1a07d05.r1 (Accession number NT_108235) | hypothetical protein BGP202 | 6811 | 7422 | - | 203 | 56561076 | 3189682 |
| Variable plasmid segment G1a07d05.r1 (Accession number NT_108235) | hypothetical protein BGP203 | 7403 | 7480 | - | 25 | 56561077 | 3189681 |
| Variable plasmid segment G1a07d05.r1 (Accession number NT_108235) | hypothetical protein BGP204 | 8141 | 8314 | - | 57 | 56561078 | 3189679 |
| Variable plasmid segment G1a07d05.r1 (Accession number NT_108235) | hypothetical protein BGP205 | 8337 | 8864 | + | 175 | 56561079 | 3189676 |
| Variable plasmid segment G1a07d05.r1 (Accession number NT_108235) | hypothetical protein BGP206 | 8857 | 9174 | + | 106 | 56561080 | 3189680 |
| Variable plasmid segment G1M1b07c12.s1 (Accession number NT_108234) | hypothetical protein BGP175 | 39 | 734 | + | 231 | 56561048 | 3189849 |
| Variable plasmid segment G1M1b07c12.s1 (Accession | hypothetical protein BGP176 | 1588 | 1974 | + | 128 | 56561049 | 3189864 |

| number NT_108234) | | | | | | | |
|---|---|---|---|---|---|---|---|
| Variable plasmid segment G1M1b07c12.s1 (Accession number NT_108234) | hypothetical protein BGP177 | 1971 | 2540 | + | 189 | 56561050 | 3189863 |
| Variable plasmid segment G1M1b07c12.s1 (Accession number NT_108234) | hypothetical protein BGP178 | 2521 | 2841 | + | 106 | 56561051 | 3189854 |
| Variable plasmid segment G1M1b07c12.s1 (Accession number NT_108234) | hypothetical protein BGP179 | 2870 | 3217 | + | 115 | 56561052 | 3189853 |
| Variable plasmid segment G1M1b07c12.s1 (Accession number NT_108234) | hypothetical protein BGP180 | 3241 | 3876 | + | 211 | 56561053 | 3189851 |
| Variable plasmid segment G1M1b07c12.s1 (Accession number NT_108234) | hypothetical protein BGP181 | 3880 | 4827 | + | 315 | 56561054 | 3189850 |
| Variable plasmid segment G1M1b07c12.s1 (Accession number NT_108234) | hypothetical protein BGP182 | 4839 | 5384 | + | 181 | 56561055 | 3189862 |
| Variable plasmid segment G1M1b07c12.s1 (Accession number NT_108234) | hypothetical protein BGP183 | 5414 | 5614 | + | 66 | 56561056 | 3189858 |
| Variable plasmid segment G1M1b07c12.s1 (Accession number NT_108234) | hypothetical protein BGP184 | 5745 | 6623 | + | 292 | 56561057 | 3189847 |
| Variable plasmid segment G1M1b07c12.s1 (Accession | hypothetical protein BGP185 | 6626 | 7225 | + | 199 | 56561058 | 3189856 |

| | | | | | | | |
|---|---|---|---|---|---|---|---|
| number NT_108234) | | | | | | | |
| Variable plasmid segment G1M1b07c12.s1 (Accession number NT_108234) | hypothetical protein BGP186 | 7242 | 7292 | + | 16 | 56561059 | 3189860 |
| Variable plasmid segment G1M1b07c12.s1 (Accession number NT_108234) | hypothetical protein BGP187 | 7302 | 8057 | + | 251 | 56561060 | 3189848 |
| Variable plasmid segment G1M1b07c12.s1 (Accession number NT_108234) | hypothetical protein BGP188 | 8132 | 8326 | + | 64 | 56561061 | 3189852 |
| Variable plasmid segment G1M1b07c12.s1 (Accession number NT_108234) | hypothetical protein BGP189 | 8330 | 8668 | + | 112 | 56561062 | 3189861 |
| Variable plasmid segment G1M1b07c12.s1 (Accession number NT_108234) | hypothetical protein BGP190 | 8668 | 9003 | + | 111 | 56561063 | 3189859 |
| Variable plasmid segment G1M1b07c12.s1 (Accession number NT_108234) | hypothetical protein BGP191 | 8996 | 9349 | + | 117 | 56561064 | 3189855 |
| Variable plasmid segment G1M1b07c12.s1 (Accession number NT_108234) | hypothetical protein BGP192 | 9546 | 1018 4 | + | 212 | 56561065 | 3189846 |
| Variable plasmid segment G1M1b07c12.s1 (Accession number NT_108234) | hypothetical protein BGP193 | 1028 7 | 1118 9 | - | 300 | 56561066 | 3189845 |
| Variable plasmid segment G1M1b07c12.s1 (Accession | hypothetical protein BGP194 | 1156 2 | 1186 8 | + | 102 | 56561067 | 3189857 |

| | | | | | | | |
|---|---|---|---|---|---|---|---|
| number NT_108234) | | | | | | | |
| Variable plasmid segment G11a15e03.s1 (Accession number NT_108233) | hypothetical protein BGP157 | 1 | 721 | - | 240 | 56561029 | 3189837 |
| Variable plasmid segment G11a15e03.s1 (Accession number NT_108233) | hypothetical protein BGP158 | 731 | 1336 | - | 201 | 56561030 | 3189844 |
| Variable plasmid segment G11a15e03.s1 (Accession number NT_108233) | hypothetical protein BGP159 | 1346 | 2221 | - | 291 | 56561031 | 3189830 |
| Variable plasmid segment G11a15e03.s1 (Accession number NT_108233) | hypothetical protein BGP160 | 2218 | 2550 | - | 110 | 56561032 | 3189836 |
| Variable plasmid segment G11a15e03.s1 (Accession number NT_108233) | hypothetical protein BGP161 | 2580 | 3125 | - | 181 | 56561033 | 3189842 |
| Variable plasmid segment G11a15e03.s1 (Accession number NT_108233) | hypothetical protein BGP162 | 3150 | 4091 | - | 313 | 56561034 | 3189835 |
| Variable plasmid segment G11a15e03.s1 (Accession number NT_108233) | hypothetical protein BGP163 | 4095 | 4778 | - | 227 | 56561035 | 3189839 |
| Variable plasmid segment G11a15e03.s1 (Accession number NT_108233) | hypothetical protein BGP164 | 4800 | 6110 | - | 436 | 56561036 | 3189838 |
| Variable plasmid segment G11a15e03.s1 (Accession | hypothetical protein BGP165 | 6118 | 6354 | - | 78 | 56561037 | 3189833 |

| | | | | | | | |
|---|---|---|---|---|---|---|---|
| number NT_108233) | | | | | | | |
| Variable plasmid segment G11a15e03.s1 (Accession number NT_108233) | hypothetical protein BGP166 | 6351 | 6806 | - | 151 | 56561038 | 3189827 |
| Variable plasmid segment G11a15e03.s1 (Accession number NT_108233) | hypothetical protein BGP167 | 6822 | 7151 | - | 109 | 56561039 | 3189828 |
| Variable plasmid segment G11a15e03.s1 (Accession number NT_108233) | hypothetical protein BGP168 | 7264 | 8376 | - | 370 | 56561040 | 3189841 |
| Variable plasmid segment G11a15e03.s1 (Accession number NT_108233) | hypothetical protein BGP169 | 8357 | 8926 | - | 189 | 56561041 | 3189834 |
| Variable plasmid segment G11a15e03.s1 (Accession number NT_108233) | hypothetical protein BGP170 | 8923 | 9312 | - | 129 | 56561042 | 3189832 |
| Variable plasmid segment G11a15e03.s1 (Accession number NT_108233) | hypothetical protein BGP171 | 9300 | 9683 | - | 127 | 56561043 | 3189829 |
| Variable plasmid segment G11a15e03.s1 (Accession number NT_108233) | hypothetical protein BGP172 | 9683 | 1013 8 | - | 151 | 56561044 | 3189843 |
| Variable plasmid segment G11a15e03.s1 (Accession number NT_108233) | hypothetical protein BGP173 | 1015 8 | 1111 7 | - | 319 | 56561045 | 3189831 |
| Variable plasmid segment G11a15e03.s1 (Accession | hypothetical protein BGP174 | 1113 9 | 1164 8 | - | 169 | 56561046 | 3189840 |

| | | | | | | | |
|---|---|---|---|---|---|---|---|
| number NT_108233) | | | | | | | |
| Variable plasmid segment G1M1b09g01.s1 (Accession number NT_108232) | hypothetical protein BGP338 | 1 | 306 | + | 101 | 56561002 | 3189814 |
| Variable plasmid segment G1M1b09g01.s1 (Accession number NT_108232) | hypothetical protein BGP132 | 383 | 646 | - | 87 | 56561003 | 3189812 |
| Variable plasmid segment G1M1b09g01.s1 (Accession number NT_108232) | hypothetical protein BGP133 | 726 | 980 | - | 84 | 56561004 | 3189806 |
| Variable plasmid segment G1M1b09g01.s1 (Accession number NT_108232) | hypothetical protein BGP134 | 1203 | 2081 | - | 292 | 56561005 | 3189817 |
| Variable plasmid segment G1M1b09g01.s1 (Accession number NT_108232) | hypothetical protein BGP135 | 2501 | 2617 | + | 38 | 56561006 | 3189821 |
| Variable plasmid segment G1M1b09g01.s1 (Accession number NT_108232) | hypothetical protein BGP136 | 3506 | 3682 | + | 58 | 56561007 | 3189825 |
| Variable plasmid segment G1M1b09g01.s1 (Accession number NT_108232) | hypothetical protein BGP137 | 3691 | 3897 | + | 68 | 56561008 | 3189803 |
| Variable plasmid segment G1M1b09g01.s1 (Accession number NT_108232) | hypothetical protein BGP138 | 4472 | 5548 | + | 358 | 56561009 | 3189801 |
| Variable plasmid segment G1M1b09g01.s1 (Accession | hypothetical protein BGP139 | 6229 | 6387 | + | 52 | 56561010 | 3189805 |

| | | | | | | | |
|---|---|---|---|---|---|---|---|
| number NT_108232) | | | | | | | |
| Variable plasmid segment G1M1b09g01.s1 (Accession number NT_108232) | hypothetical protein BGP140 | 6428 | 6529 | + | 33 | 56561011 | 3189807 |
| Variable plasmid segment G1M1b09g01.s1 (Accession number NT_108232) | hypothetical protein BGP141 | 6456 | 6905 | - | 149 | 56561012 | 3189804 |
| Variable plasmid segment G1M1b09g01.s1 (Accession number NT_108232) | hypothetical protein BGP142 | 6902 | 7105 | - | 67 | 56561013 | 3189816 |
| Variable plasmid segment G1M1b09g01.s1 (Accession number NT_108232) | hypothetical protein BGP143 | 7102 | 7434 | - | 110 | 56561014 | 3189813 |
| Variable plasmid segment G1M1b09g01.s1 (Accession number NT_108232) | hypothetical protein BGP144 | 7701 | 7937 | + | 78 | 56561015 | 3189802 |
| Variable plasmid segment G1M1b09g01.s1 (Accession number NT_108232) | hypothetical protein BGP145 | 8062 | 9087 | + | 341 | 56561016 | 3189820 |
| Variable plasmid segment G1M1b09g01.s1 (Accession number NT_108232) | hypothetical protein BGP146 | 9157 | 9294 | + | 45 | 56561017 | 3189810 |
| Variable plasmid segment G1M1b09g01.s1 (Accession number NT_108232) | hypothetical protein BGP147 | 9312 | 9443 | + | 43 | 56561018 | 3189811 |
| Variable plasmid segment G1M1b09g01.s1 (Accession | hypothetical protein BGP148 | 9584 | 9799 | + | 71 | 56561019 | 3189808 |

| | | | | | | | |
|---|---|---|---|---|---|---|---|
| number NT_108232) | | | | | | | |
| Variable plasmid segment G1M1b09g01.s1 (Accession number NT_108232) | hypothetical protein BGP149 | 9880 | 1000 8 | + | 42 | 56561020 | 3189826 |
| Variable plasmid segment G1M1b09g01.s1 (Accession number NT_108232) | hypothetical protein BGP150 | 1002 0 | 1011 2 | + | 30 | 56561021 | 3189823 |
| Variable plasmid segment G1M1b09g01.s1 (Accession number NT_108232) | hypothetical protein BGP151 | 1086 9 | 1106 3 | - | 64 | 56561022 | 3189809 |
| Variable plasmid segment G1M1b09g01.s1 (Accession number NT_108232) | hypothetical protein BGP152 | 1107 6 | 1150 1 | - | 141 | 56561023 | 3189819 |
| Variable plasmid segment G1M1b09g01.s1 (Accession number NT_108232) | hypothetical protein BGP153 | 1150 7 | 1183 6 | - | 109 | 56561024 | 3189818 |
| Variable plasmid segment G1M1b09g01.s1 (Accession number NT_108232) | hypothetical protein BGP154 | 1202 2 | 1216 8 | + | 48 | 56561025 | 3189824 |
| Variable plasmid segment G1M1b09g01.s1 (Accession number NT_108232) | hypothetical protein BGP155 | 1232 7 | 1272 8 | + | 133 | 56561026 | 3189822 |
| Variable plasmid segment G1M1b09g01.s1 (Accession number NT_108232) | hypothetical protein BGP156 | 1279 4 | 1308 1 | - | 95 | 56561027 | 3189815 |
| Variable plasmid segment G1a34h09.s1 (Accession | hypothetical protein BGP109 | 1 | 572 | - | 190 | 56560978 | 3189793 |

| | | | | | | | |
|---|---|---|---|---|---|---|---|
| number NT_108231) | | | | | | | |
| Variable plasmid segment G1a34h09.s1 (Accession number NT_108231) | hypothetical protein BGP110 | 683 | 1504 | - | 273 | 56560979 | 3189940 |
| Variable plasmid segment G1a34h09.s1 (Accession number NT_108231) | hypothetical protein BGP111 | 1563 | 1754 | - | 63 | 56560980 | 3189944 |
| Variable plasmid segment G1a34h09.s1 (Accession number NT_108231) | hypothetical protein BGP112 | 1736 | 2113 | - | 125 | 56560981 | 3189799 |
| Variable plasmid segment G1a34h09.s1 (Accession number NT_108231) | hypothetical protein BGP113 | 2269 | 2481 | - | 70 | 56560982 | 3189945 |
| Variable plasmid segment G1a34h09.s1 (Accession number NT_108231) | hypothetical protein BGP114 | 2548 | 3318 | + | 256 | 56560983 | 3189792 |
| Variable plasmid segment G1a34h09.s1 (Accession number NT_108231) | hypothetical protein BGP115 | 3329 | 3676 | + | 115 | 56560984 | 3189789 |
| Variable plasmid segment G1a34h09.s1 (Accession number NT_108231) | hypothetical protein BGP116 | 3673 | 4683 | + | 336 | 56560985 | 3189798 |
| Variable plasmid segment G1a34h09.s1 (Accession number NT_108231) | hypothetical protein BGP117 | 4680 | 4865 | + | 61 | 56560986 | 3189942 |
| Variable plasmid segment G1a34h09.s1 (Accession | hypothetical protein BGP118 | 4876 | 5073 | + | 65 | 56560987 | 3189790 |

| number NT_108231) | | | | | | | |
|---|---|---|---|---|---|---|---|
| Variable plasmid segment G1a34h09.s1 (Accession number NT_108231) | hypothetical protein BGP119 | 5127 | 5903 | + | 258 | 56560988 | 3189791 |
| Variable plasmid segment G1a34h09.s1 (Accession number NT_108231) | hypothetical protein BGP120 | 5940 | 6488 | + | 182 | 56560989 | 3189794 |
| Variable plasmid segment G1a34h09.s1 (Accession number NT_108231) | hypothetical protein BGP121 | 6489 | 6998 | + | 169 | 56560990 | 3189937 |
| Variable plasmid segment G1a34h09.s1 (Accession number NT_108231) | hypothetical protein BGP122 | 7034 | 7864 | + | 276 | 56560991 | 3189800 |
| Variable plasmid segment G1a34h09.s1 (Accession number NT_108231) | hypothetical protein BGP123 | 7880 | 8218 | + | 112 | 56560992 | 3189946 |
| Variable plasmid segment G1a34h09.s1 (Accession number NT_108231) | hypothetical protein BGP124 | 8234 | 8626 | + | 130 | 56560993 | 3189943 |
| Variable plasmid segment G1a34h09.s1 (Accession number NT_108231) | hypothetical protein BGP125 | 8623 | 9066 | + | 147 | 56560994 | 3189796 |
| Variable plasmid segment G1a34h09.s1 (Accession number NT_108231) | hypothetical protein BGP126 | 9056 | 9874 | + | 272 | 56560995 | 3189939 |
| Variable plasmid segment G1a34h09.s1 (Accession | hypothetical protein BGP127 | 9884 | 1031 2 | + | 142 | 56560996 | 3189938 |

| | | | | | | | |
|---|---|---|---|---|---|---|---|
| number NT_108231) | | | | | | | |
| Variable plasmid segment G1a34h09.s1 (Accession number NT_108231) | hypothetical protein BGP128 | 1040 2 | 1059 9 | + | 65 | 56560997 | 3189936 |
| Variable plasmid segment G1a34h09.s1 (Accession number NT_108231) | hypothetical protein BGP129 | 1059 6 | 1258 4 | + | 662 | 56560998 | 3189941 |
| Variable plasmid segment G1a34h09.s1 (Accession number NT_108231) | hypothetical protein BGP130 | 1264 4 | 1384 6 | + | 400 | 56560999 | 3189797 |
| Variable plasmid segment G1a34h09.s1 (Accession number NT_108231) | hypothetical protein BGP131 | 1416 7 | 1453 1 | + | 121 | 56561000 | 3189795 |
| Variable plasmid segment G1a08d08.s1 (Accession number NT_108230) | hypothetical protein BGP085 | 117 | 590 | - | 157 | 56560953 | 3189927 |
| Variable plasmid segment G1a08d08.s1 (Accession number NT_108230) | hypothetical protein BGP086 | 916 | 1065 | - | 49 | 56560954 | 3189931 |
| Variable plasmid segment G1a08d08.s1 (Accession number NT_108230) | hypothetical protein BGP087 | 1076 | 1807 | - | 243 | 56560955 | 3189921 |
| Variable plasmid segment G1a08d08.s1 (Accession number NT_108230) | hypothetical protein BGP088 | 2048 | 3022 | + | 324 | 56560956 | 3189925 |
| Variable plasmid segment G1a08d08.s1 (Accession | hypothetical protein BGP089 | 3398 | 3535 | + | 45 | 56560957 | 3189920 |

| | | | | | | | |
|---|---|---|---|---|---|---|---|
| number NT_108230) | | | | | | | |
| Variable plasmid segment G1a08d08.s1 (Accession number NT_108230) | hypothetical protein BGP090 | 3453 | 3644 | - | 63 | 56560958 | 3189923 |
| Variable plasmid segment G1a08d08.s1 (Accession number NT_108230) | hypothetical protein BGP091 | 3664 | 3849 | + | 61 | 56560959 | 3189913 |
| Variable plasmid segment G1a08d08.s1 (Accession number NT_108230) | hypothetical protein BGP092 | 3926 | 4060 | - | 44 | 56560960 | 3189928 |
| Variable plasmid segment G1a08d08.s1 (Accession number NT_108230) | hypothetical protein BGP093 | 4358 | 4960 | + | 200 | 56560961 | 3189933 |
| Variable plasmid segment G1a08d08.s1 (Accession number NT_108230) | hypothetical protein BGP094 | 5333 | 5482 | - | 49 | 56560962 | 3189915 |
| Variable plasmid segment G1a08d08.s1 (Accession number NT_108230) | hypothetical protein BGP095 | 5569 | 5709 | - | 46 | 56560963 | 3189929 |
| Variable plasmid segment G1a08d08.s1 (Accession number NT_108230) | hypothetical protein BGP096 | 5773 | 6714 | + | 313 | 56560964 | 3189922 |
| Variable plasmid segment G1a08d08.s1 (Accession number NT_108230) | hypothetical protein BGP097 | 7435 | 7521 | - | 28 | 56560965 | 3189926 |
| Variable plasmid segment G1a08d08.s1 (Accession | hypothetical protein BGP098 | 7737 | 7904 | + | 55 | 56560966 | 3189935 |

| number NT_108230) | | | | | | | |
|---|---|---|---|---|---|---|---|
| Variable plasmid segment G1a08d08.s1 (Accession number NT_108230) | hypothetical protein BGP099 | 8066 | 8536 | + | 156 | 56560967 | 3189918 |
| Variable plasmid segment G1a08d08.s1 (Accession number NT_108230) | hypothetical protein BGP100 | 9297 | 1036 1 | + | 354 | 56560968 | 3189934 |
| Variable plasmid segment G1a08d08.s1 (Accession number NT_108230) | hypothetical protein BGP101 | 1071 5 | 1078 3 | - | 22 | 56560969 | 3189932 |
| Variable plasmid segment G1a08d08.s1 (Accession number NT_108230) | hypothetical protein BGP102 | 1112 4 | 1126 4 | - | 46 | 56560970 | 3189912 |
| Variable plasmid segment G1a08d08.s1 (Accession number NT_108230) | hypothetical protein BGP103 | 1126 6 | 1145 1 | - | 61 | 56560971 | 3189919 |
| Variable plasmid segment G1a08d08.s1 (Accession number NT_108230) | hypothetical protein BGP104 | 1147 5 | 1183 7 | - | 120 | 56560972 | 3189930 |
| Variable plasmid segment G1a08d08.s1 (Accession number NT_108230) | hypothetical protein BGP105 | 1239 7 | 1257 0 | + | 57 | 56560973 | 3189917 |
| Variable plasmid segment G1a08d08.s1 (Accession number NT_108230) | hypothetical protein BGP106 | 1296 7 | 1315 2 | + | 61 | 56560974 | 3189924 |
| Variable plasmid segment G1a08d08.s1 (Accession | hypothetical protein BGP107 | 1379 6 | 1408 9 | + | 97 | 56560975 | 3189916 |

| | | | | | | | |
|---|---|---|---|---|---|---|---|
| number NT_108230) | | | | | | | |
| Variable plasmid segment G1a08d08.s1 (Accession number NT_108230) | hypothetical protein BGP108 | 1552 0 | 1606 7 | - | 181 | 56560976 | 3189914 |
| Variable plasmid segment G1M1b13a11.r1 (Accession number NT_108229) | hypothetical protein BGP056 | 56 | 478 | + | 140 | 56560923 | 3189894 |
| Variable plasmid segment G1M1b13a11.r1 (Accession number NT_108229) | hypothetical protein BGP057 | 495 | 1190 | + | 231 | 56560924 | 3189906 |
| Variable plasmid segment G1M1b13a11.r1 (Accession number NT_108229) | hypothetical protein BGP058 | 1202 | 1765 | + | 187 | 56560925 | 3189910 |
| Variable plasmid segment G1M1b13a11.r1 (Accession number NT_108229) | hypothetical protein BGP059 | 1785 | 2744 | + | 319 | 56560926 | 3189907 |
| Variable plasmid segment G1M1b13a11.r1 (Accession number NT_108229) | hypothetical protein BGP060 | 2763 | 3215 | + | 150 | 56560927 | 3189889 |
| Variable plasmid segment G1M1b13a11.r1 (Accession number NT_108229) | hypothetical protein BGP061 | 3215 | 3598 | + | 127 | 56560928 | 3189902 |
| Variable plasmid segment G1M1b13a11.r1 (Accession number NT_108229) | hypothetical protein BGP062 | 3586 | 3972 | + | 128 | 56560929 | 3189898 |
| Variable plasmid segment G1M1b13a11.r1 (Accession | hypothetical protein BGP063 | 3975 | 4538 | + | 187 | 56560930 | 3189890 |

| | | | | | | | |
|---|---|---|---|---|---|---|---|
| number NT_108229) | | | | | | | |
| Variable plasmid segment G1M1b13a11.r1 (Accession number NT_108229) | hypothetical protein BGP064 | 4519 | 5640 | + | 373 | 56560931 | 3189911 |
| Variable plasmid segment G1M1b13a11.r1 (Accession number NT_108229) | hypothetical protein BGP065 | 5654 | 6082 | + | 142 | 56560932 | 3189900 |
| Variable plasmid segment G1M1b13a11.r1 (Accession number NT_108229) | hypothetical protein BGP066 | 6098 | 6553 | + | 151 | 56560933 | 3189909 |
| Variable plasmid segment G1M1b13a11.r1 (Accession number NT_108229) | hypothetical protein BGP067 | 6550 | 6786 | + | 78 | 56560934 | 3189896 |
| Variable plasmid segment G1M1b13a11.r1 (Accession number NT_108229) | hypothetical protein BGP068 | 6794 | 7042 | + | 82 | 56560935 | 3189908 |
| Variable plasmid segment G1M1b13a11.r1 (Accession number NT_108229) | hypothetical protein BGP069 | 7096 | 7875 | + | 259 | 56560936 | 3189888 |
| Variable plasmid segment G1M1b13a11.r1 (Accession number NT_108229) | hypothetical protein BGP070 | 7888 | 8076 | - | 62 | 56560937 | 3189891 |
| Variable plasmid segment G1M1b13a11.r1 (Accession number NT_108229) | hypothetical protein BGP071 | 8110 | 8796 | + | 228 | 56560938 | 3189883 |
| Variable plasmid segment G1M1b13a11.r1 (Accession | hypothetical protein BGP072 | 8800 | 9789 | + | 329 | 56560939 | 3189886 |

| | | | | | | | |
|---|---|---|---|---|---|---|---|
| number NT_108229) | | | | | | | |
| Variable plasmid segment G1M1b13a11.r1 (Accession number NT_108229) | hypothetical protein BGP073 | 9786 | 1033 1 | + | 181 | 56560940 | 3189884 |
| Variable plasmid segment G1M1b13a11.r1 (Accession number NT_108229) | hypothetical protein BGP074 | 1036 1 | 1069 3 | + | 110 | 56560941 | 3189892 |
| Variable plasmid segment G1M1b13a11.r1 (Accession number NT_108229) | hypothetical protein BGP075 | 1069 0 | 1156 8 | + | 292 | 56560942 | 3189904 |
| Variable plasmid segment G1M1b13a11.r1 (Accession number NT_108229) | hypothetical protein BGP076 | 1157 3 | 1217 5 | + | 200 | 56560943 | 3189903 |
| Variable plasmid segment G1M1b13a11.r1 (Accession number NT_108229) | hypothetical protein BGP077 | 1218 5 | 1299 7 | + | 270 | 56560944 | 3189895 |
| Variable plasmid segment G1M1b13a11.r1 (Accession number NT_108229) | hypothetical protein BGP078 | 1307 0 | 1326 7 | + | 65 | 56560945 | 3189893 |
| Variable plasmid segment G1M1b13a11.r1 (Accession number NT_108229) | hypothetical protein BGP079 | 1327 1 | 1350 7 | + | 78 | 56560946 | 3189901 |
| Variable plasmid segment G1M1b13a11.r1 (Accession number NT_108229) | hypothetical protein BGP080 | 1360 8 | 1394 3 | + | 111 | 56560947 | 3189897 |
| Variable plasmid segment G1M1b13a11.r1 (Accession | hypothetical protein BGP081 | 1393 6 | 1428 9 | + | 117 | 56560948 | 3189885 |

| | | | | | | | |
|---|---|---|---|---|---|---|---|
| number NT_108229) | | | | | | | |
| Variable plasmid segment G1M1b13a11.r1 (Accession number NT_108229) | hypothetical protein BGP082 | 14542 | 14976 | + | 144 | 56560949 | 3189905 |
| Variable plasmid segment G1M1b13a11.r1 (Accession number NT_108229) | hypothetical protein BGP083 | 14998 | 15426 | + | 142 | 56560950 | 3189899 |
| Variable plasmid segment G1M1b13a11.r1 (Accession number NT_108229) | hypothetical protein BGP084 | 15467 | 16033 | - | 188 | 56560951 | 3189887 |
| Variable plasmid segment G11a17h03.s1 (Accession number NT_108228) | hypothetical protein BGP033 | 360 | 458 | - | 32 | 56560899 | 3189874 |
| Variable plasmid segment G11a17h03.s1 (Accession number NT_108228) | hypothetical protein BGP034 | 680 | 853 | - | 57 | 56560900 | 3189867 |
| Variable plasmid segment G11a17h03.s1 (Accession number NT_108228) | hypothetical protein BGP035 | 1425 | 2240 | - | 271 | 56560901 | 3189773 |
| Variable plasmid segment G11a17h03.s1 (Accession number NT_108228) | hypothetical protein BGP036 | 2594 | 3760 | + | 388 | 56560902 | 3189873 |
| Variable plasmid segment G11a17h03.s1 (Accession number NT_108228) | hypothetical protein BGP037 | 5389 | 5583 | - | 64 | 56560903 | 3189878 |
| Variable plasmid segment G11a17h03.s1 (Accession | hypothetical protein BGP038 | 5974 | 6075 | - | 33 | 56560904 | 3189880 |

| | | | | | | | |
|---|---|---|---|---|---|---|---|
| number NT_108228) | | | | | | | |
| Variable plasmid segment G11a17h03.s1 (Accession number NT_108228) | hypothetical protein BGP039 | 6075 | 6530 | - | 151 | 56560905 | 3189882 |
| Variable plasmid segment G11a17h03.s1 (Accession number NT_108228) | hypothetical protein BGP040 | 6549 | 7508 | - | 319 | 56560906 | 3189876 |
| Variable plasmid segment G11a17h03.s1 (Accession number NT_108228) | hypothetical protein BGP041 | 7530 | 8099 | - | 189 | 56560907 | 3189777 |
| Variable plasmid segment G11a17h03.s1 (Accession number NT_108228) | hypothetical protein BGP042 | 8114 | 8884 | - | 256 | 56560908 | 3189875 |
| Variable plasmid segment G11a17h03.s1 (Accession number NT_108228) | hypothetical protein BGP043 | 8892 | 9449 | - | 185 | 56560909 | 3189775 |
| Variable plasmid segment G11a17h03.s1 (Accession number NT_108228) | hypothetical protein BGP044 | 9461 | 1015 0 | - | 229 | 56560910 | 3189879 |
| Variable plasmid segment G11a17h03.s1 (Accession number NT_108228) | hypothetical protein BGP045 | 1016 7 | 1139 0 | - | 407 | 56560911 | 3189871 |
| Variable plasmid segment G11a17h03.s1 (Accession number NT_108228) | hypothetical protein BGP046 | 1145 6 | 1280 8 | - | 450 | 56560912 | 3189869 |
| Variable plasmid segment G11a17h03.s1 (Accession | hypothetical protein BGP047 | 1280 5 | 1337 1 | - | 188 | 56560913 | 3189881 |

| | | | | | | | |
|---|---|---|---|---|---|---|---|
| number NT_108228) | | | | | | | |
| Variable plasmid segment G11a17h03.s1 (Accession number NT_108228) | hypothetical protein BGP048 | 13586 | 13831 | + | 81 | 56560914 | 3189866 |
| Variable plasmid segment G11a17h03.s1 (Accession number NT_108228) | hypothetical protein BGP049 | 13930 | 14991 | - | 353 | 56560915 | 3189872 |
| Variable plasmid segment G11a17h03.s1 (Accession number NT_108228) | hypothetical protein BGP050 | 15429 | 15635 | + | 68 | 56560916 | 3189870 |
| Variable plasmid segment G11a17h03.s1 (Accession number NT_108228) | hypothetical protein BGP051 | 15713 | 16204 | + | 163 | 56560917 | 3189877 |
| Variable plasmid segment G11a17h03.s1 (Accession number NT_108228) | hypothetical protein BGP052 | 16210 | 16635 | - | 141 | 56560918 | 3190010 |
| Variable plasmid segment G11a17h03.s1 (Accession number NT_108228) | hypothetical protein BGP053 | 16693 | 16872 | - | 59 | 56560919 | 3189776 |
| Variable plasmid segment G11a17h03.s1 (Accession number NT_108228) | hypothetical protein BGP054 | 16854 | 16970 | - | 38 | 56560920 | 3189774 |
| Variable plasmid segment G11a17h03.s1 (Accession number NT_108228) | hypothetical protein BGP055 | 17191 | 17640 | - | 149 | 56560921 | 3189868 |
| Variable plasmid segment Gla19d06.s1 (Accession | hypothetical protein BGP001 | 427 | 1122 | - | 231 | 56560866 | 3189995 |

| | | | | | | | |
|---|---|---|---|---|---|---|---|
| number NT_108227) | | | | | | | |
| Variable plasmid segment Gla19d06.s1 (Accession number NT_108227) | hypothetical protein BGP002 | 1181 | 1732 | - | 183 | 56560867 | 3190009 |
| Variable plasmid segment Gla19d06.s1 (Accession number NT_108227) | hypothetical protein BGP003 | 1872 | 2129 | - | 85 | 56560868 | 3189991 |
| Variable plasmid segment Gla19d06.s1 (Accession number NT_108227) | hypothetical protein BGP004 | 2192 | 2353 | + | 53 | 56560869 | 3189765 |
| Variable plasmid segment Gla19d06.s1 (Accession number NT_108227) | hypothetical protein BGP005 | 2350 | 2445 | - | 31 | 56560870 | 3189993 |
| Variable plasmid segment Gla19d06.s1 (Accession number NT_108227) | hypothetical protein BGP006 | 2442 | 3857 | - | 471 | 56560871 | 3189992 |
| Variable plasmid segment Gla19d06.s1 (Accession number NT_108227) | hypothetical protein BGP007 | 3981 | 4133 | + | 50 | 56560872 | 3189772 |
| Variable plasmid segment Gla19d06.s1 (Accession number NT_108227) | hypothetical protein BGP008 | 4232 | 4645 | + | 137 | 56560873 | 3189996 |
| Variable plasmid segment Gla19d06.s1 (Accession number NT_108227) | hypothetical protein BGP009 | 4638 | 5330 | + | 230 | 56560874 | 3189990 |
| Variable plasmid segment Gla19d06.s1 (Accession | hypothetical protein BGP010 | 5330 | 5701 | + | 123 | 56560875 | 3189947 |

| number NT_108227) | | | | | | | |
|---|---|---|---|---|---|---|---|
| Variable plasmid segment Gla19d06.s1 (Accession number NT_108227) | hypothetical protein BGP011 | 5704 | 6090 | + | 128 | 56560876 | 3189988 |
| Variable plasmid segment Gla19d06.s1 (Accession number NT_108227) | hypothetical protein BGP012 | 6102 | 8726 | + | 874 | 56560877 | 3189989 |
| Variable plasmid segment Gla19d06.s1 (Accession number NT_108227) | hypothetical protein BGP013 | 8727 | 9509 | + | 260 | 56560878 | 3190001 |
| Variable plasmid segment Gla19d06.s1 (Accession number NT_108227) | hypothetical protein BGP014 | 9522 | 1029 2 | + | 256 | 56560879 | 3189770 |
| Variable plasmid segment Gla19d06.s1 (Accession number NT_108227) | hypothetical protein BGP015 | 1030 3 | 1065 0 | + | 115 | 56560880 | 3189768 |
| Variable plasmid segment Gla19d06.s1 (Accession number NT_108227) | hypothetical protein BGP016 | 1064 7 | 1183 7 | + | 396 | 56560881 | 3190004 |
| Variable plasmid segment Gla19d06.s1 (Accession number NT_108227) | hypothetical protein BGP017 | 1184 9 | 1287 7 | + | 342 | 56560882 | 3189997 |
| Variable plasmid segment Gla19d06.s1 (Accession number NT_108227) | hypothetical protein BGP018 | 1291 1 | 1345 9 | + | 182 | 56560883 | 3189769 |
| Variable plasmid segment Gla19d06.s1 (Accession | hypothetical protein BGP019 | 1346 0 | 1396 9 | + | 169 | 56560884 | 3189998 |

| | | | | | | | |
|---|---|---|---|---|---|---|---|
| number NT_108227) | | | | | | | |
| Variable plasmid segment Gla19d06.s1 (Accession number NT_108227) | hypothetical protein BGP020 | 1400 5 | 1483 5 | + | 276 | 56560885 | 3189999 |
| Variable plasmid segment Gla19d06.s1 (Accession number NT_108227) | hypothetical protein BGP021 | 1485 1 | 1518 9 | + | 112 | 56560886 | 3190002 |
| Variable plasmid segment Gla19d06.s1 (Accession number NT_108227) | hypothetical protein BGP022 | 1520 5 | 1559 7 | + | 130 | 56560887 | 3190007 |
| Variable plasmid segment Gla19d06.s1 (Accession number NT_108227) | hypothetical protein BGP023 | 1559 4 | 1603 7 | + | 147 | 56560888 | 3190005 |
| Variable plasmid segment Gla19d06.s1 (Accession number NT_108227) | hypothetical protein BGP024 | 1602 7 | 1684 5 | + | 272 | 56560889 | 3189771 |
| Variable plasmid segment Gla19d06.s1 (Accession number NT_108227) | hypothetical protein BGP025 | 1685 5 | 1728 3 | + | 142 | 56560890 | 3189994 |
| Variable plasmid segment Gla19d06.s1 (Accession number NT_108227) | hypothetical protein BGP026 | 1737 3 | 1757 0 | + | 65 | 56560891 | 3189766 |
| Variable plasmid segment Gla19d06.s1 (Accession number NT_108227) | hypothetical protein BGP027 | 1756 7 | 2081 2 | + | 1081 | 56560892 | 3189767 |
| Variable plasmid segment Gla19d06.s1 (Accession | hypothetical protein BGP028 | 2113 3 | 2224 5 | + | 370 | 56560893 | 3190003 |

| number NT_108227) | | | | | | | |
|---|---|---|---|---|---|---|---|
| Variable plasmid segment Gla19d06.s1 (Accession number NT_108227) | hypothetical protein BGP029 | 2219 9 | 2225 2 | + | 17 | 56560894 | 3189764 |
| Variable plasmid segment Gla19d06.s1 (Accession number NT_108227) | hypothetical protein BGP030 | 2225 5 | 2281 5 | + | 186 | 56560895 | 3190006 |
| Variable plasmid segment Gla19d06.s1 (Accession number NT_108227) | hypothetical protein BGP031 | 2279 1 | 2355 5 | + | 254 | 56560896 | 3190008 |
| Variable plasmid segment Gla19d06.s1 (Accession number NT_108227) | hypothetical protein BGP032 | 2389 7 | 2416 3 | + | 88 | 56560897 | 3190000 |

## Fig. 30.

| Antigen designation | Amino acid sequence |
|---|---|
| NP_212517.1 Basic membrane protein A (bmpA) [Borrelia burgdorferi B31 SEQ ID NO 1 | MNKILLLILLESIVFLSCSGKGSLGSEIPKVSLIIDGTFDDKSFNESALNGVKKVKEEFK IELVLKESSSNSYLSDLEGLKDAGSDLIWLIGYRFSDVAKVAALQNPDMKYAIIDPIYS NDPIPANLVGMTFRAQEGAFLTGYIAAKLSKTGKIGFLGGIEGEIVDAFRYGYEAGAK YANKDIKISTQYIGSFADLEAGRSVATRMYSDEIDIIHHAAGLGGIGAIEVAKELGSGH YIIGVDEDQAYLAPDNVITSTTKDVGRALNIFTSNHLKTNTFEGGKLINYGLKEGVVG FVRNPKMISFELEKEIDNLSSKIINKEIIVPSNKESYEKFLKEFI |
| NP_212516.1 Basic membrane protein B (bmpB) [Borrelia burgdorferi B31 SEQ ID NO 2 | MRIVIFIFGILLTSCFSRNGIESSSKKIKISMLVDGVLDDKSFNSSANEALLRLKKDFPE NIEEVFSCAISGVYSSYVSDLDNLKRNGSDLIWLVGYMLTDASLLVSSENPKISYGII DPIYGDDVQIPENLIAVVFRVEQGAFLAGYIAAKKSFSGKIGFIGGMKGNIVDAFRYG YESGAKYANKDIEIISEYSNSFSDVDIGRTIASKMYSKGIDVIHFAAGLAGIGVIETAKN LGDGYYVIGADQDQSYLAPKNFITSVIKNIGDALYLITGEYIKNNNVWEGGKVVQMG LRDGVIGLPNANEFEYIKVLERKIINKEIIVPCNQEEYEIFIKQILKL |
| NP_212518.1 Basic membrane protein C (bmpC) [Borrelia burgdorferi B31 SEQ ID NO 3 | MFKRFIFITLSLLVFACFKSNKKSIKSDKVVVGVLAHGSFYDKGYNQSVHDGVVKLR DNFGIKLITKSLRPYPIEGKRLLTVDEAMTEDAYEVQKNPLNLFWLIGYRFSDLSVKL SYERPDIYYGIIDAFDYGDIQVPKNSLAIKFRNEEAAFLAGYIAAKMSRKEKIGFLTGP MSEHVKDFKFGFKAGIFYANPKLRLVSKKAPSLFDKEKGKAMALFMYKEDKVGVIF PIAGITGLGVYDAAKELGPKYYVIGLNQDQSYIAPQNVITSIIKDIGKVIYSISSEYINNR VFKGGIIIDRGLKEGVIEIVKDPDVLNNRLVDEVIDLENKIISGEIIVPDSEYAFDLFKSK L |
| NP_212519.1B Basic membrane protein D (bmpD) [Borrelia burgdorferi B31 SEQ ID NO 4 | MDNYYEIYFLYFFIKSKEDIFMLKKVYYFLIFLFIVACSSSDDGKSEAKTVSLIVDGAF DDKGFNESSSKAIRKLKADLNINIIEKASTGNSYLGDIANLEDGNSNLIWGIGFRLSDI LFQRASENVSVNYAIIEGVYDEIQIPKNLLNISFRSEEVAFLAGYFASKASKTGKIGFV GGVRGKVLESFMYGYEAGAKYANSNIKVVSQYVGTFGDFGLGRSTASNMYRDGV DIIFAAAGLSGIGVIEAAKELGPDHYIIGVDQDQSYLAPNNVIVSAVKKVDSLMYSLTK KYLETGVLDGGKTMFLGLKEDGLGLVLNENLKSNYSEIYNKSLKIGQSIMNGIIKVPY DKVSYFVLQMEN |
| AAC70056.1 Decorin binding protein A; DbpA [Borrelia afzelii SEQ ID NO 5 | MIKYNKIILTLTLLASLLAACSLTGKARLESSVKDITNEIEKAIKEAEDAGVKTDAFTET QTGGKVGGSQIRAAKIRVADLTIKFLEATEEETITFKENGAGEEDFSGIYDLILNAAKA VEKIGMQGMKQAVEEAAKEKPKTTADGIIAIVKVMKAKVENIKEKQTKNQK |
| AAC70057.1 Decorin binding protein A; DbpA [Borrelia garinii] SEQ ID NO 6 | MIKYNKILLKLSLIVSLLVACGLTGETKIRLESSAQEIKDEINKIKANAKKEGVKFEAFT NTQTGSKISEKPEFILKAKIKAIQVAERFVKAIKEEAEKLKKSGSSGAFSAMYDLMIDV SKPLEEIGIQKMTGTVTKEAEKTPPTTADGIIAIAQAMEEKLNNVNKKQHDALKNLEE KANTAATTT |
| AAC70021.1 Decorin binding protein B; DbpB [Borrelia burgdorferi] SEQ ID NO 7 | SIVIALFFKLLVACSIGLVERTNAALESSSKDLKNKILKIKKEATGKGVLFEAFTGLKTG SKVTSGGLALREAKVQAIVETGKFLKIIEEEALKLKETGNSGQFLAMFDLMLEVVESL EDVGIIGLKARVLEESKNNPINTAERLLAAKAQIENQLKVVKEKQNIENGGEKKNNKS KKKK |
| NP_212694.1 Heat shock protein 90 [Borrelia burgdorferi B31] SEQ ID NO 8 | MILIFYFKQIALFIIFRLCYIIKKVKIKLKRKSCMKKQFDTEVNDLLYLIIHSLYSHKEIFLR ELISNASDAIDKLKFLSLTNEKFKNIALEPKIEISFDDKSILIKDNGIGMDEQDLTNHLG VIAKSGTKEFINNLKQDEKKSASLIGQFGVGFYSAFIVSEKVEVTSKKALESDAYIWS SDGKTGYEIEKAKKEESGTEIKLYLNKEGLEYANKWKIQEIIKKYSNHINYPIYIKYSE PIMKDGKQEGIEEKEEKLNETTALWTKNKSEIKAEEYNEFYKNTTFDYENPLMHIHT KAEGNLEYTNLFYVPSKAPYDLYYPNTKPGVKLFINRIFITDSEGSLLPNYLRFIKGIID CQDLPLNVSREILQQNKILSKIKSSSVKKILSELEKLSKKNPEKFSEFSKEFGRCIKEG VYSDFENREKLISLIRFKSSSVDGFVSFKEYKERMNESQKSIYYITGGKENILKENPIV AAYKEKGFEILIMDDELDEAILNLIPEYEGLKLKAINKNETSNELKDENFKKIEEEFKDT LTKVKEILKDHIKEVNLSATLIKEPSAIIIDSNDPTYQMQKIMLSMGQEVKEIKPILELNP |

| | NNKIVQNLKNLEPEKLEKISILLFEEAMLTSGMPSKNPGKFINIINEFIEKDFL |
|---|---|
| CAA44492.1\| Outer surface protein A [Borrelia burgdorferi] SEQ ID NO 9 | MKKYLLGIGLILALIACKQNVSTLDEKNSVSVDLPGGMTELVSKEKDKDGKYSLEAT VDKLELKGTSDKNNGSGTLEGEKTDKSKVKLTIADDLSQTKFEIFKEDAKTLVSKKV TLKDKSSTEEKFNEKGETSEKTIVRANGTRLEYTDIKSDGSGKAKEVLKDFTLEGTL AADGKTTLKVTEGTVVLSKNILKSGEITVALDDSDTTQATKKTGKWDSKTSTLTISVN SQKTKNLVFTKEDTITVQKYDSAGTNLEGKAVEITTLKELKNALK |
| \|BAA22351.1\| Outer surface protein B [Borrelia garinii] SEQ ID NO 10 | MKKYLLGFALVLALIACGQKGAEPKHNDQEVEDSKKDQKDASKKDLPLVTEDTVKL FNDTKIFISKEKNKDGKYELRATVDTVELKGVADKNDGSGGKLEGVKSDQSKVTMS ITDDLNTITVETYDSSNTKVASKVFKKQGSLTEETEETYKTGKLSTKKITRTNGTTLE YSDMTNDENATKAVETLKNGIMLEGNLVGGKTSVEIKEGTVTLKKEIEKAGTVKLFL DDTSSGSTKKTAVWSDTSNTLTVSADSKKIKDFVFLTDGTITVQNYDTAGTKLAGTA TEIKDLEALKAALK |
| AAM22469.1\| Outer surface protein C [Borrelia afzelii] SEQ ID NO 11 | MKKNTLSAILMTLFLFISCNNSGKGGDSASTNPADESAKGPNLTEISKKITDSNAFVL AVKEVETLVSSIDELANKAIGKKIQQNGLGAEANRNESLLAGVHEISTLITEKLSKLKN SGELKAKIEDAKKCSEEFTNKLRVSHADLGKQGVNDDDAKKAILKTNADKTKGAEE LGKLFKSVEGLVKAAQEALTNSVKELTSPVVAESPKKP |
| AAC62927.1\| OspE-related lipoprotein [Borrelia garinii] SEQ ID NO 12 | MNKKMKMFIICAVFVLISSCGNFRSSLSDQGSLSDQGSLSDQGSLSDQGGLSGQA SSDTIKFSEFTVNIKNKKDNNGDWSNLGTLVIRKEQDGVETGLNVIGTINGQLRGHS ATFFCIEEAEVNNFVKAMTNVGSFKTSLYYGYKEEQSSTNGIKGKEITTKIETINNSE HITFSGDKI |
| CAA57806.1\| Outer surface protein G [Borrelia burgdorferi] SEQ ID NO 13 | MNKKMKNLIICAVFVLIISCKIDASSEDLKQNVKEKVEGFLDKELMQGDDPNNSLFNP PPVLPASSHDNTPVLKAVQAKDGGQQEGKEEKEKEIQELKDKIDKRKKELEEARKK FQEFKEQVESATGESTEKVKKQGNIGQKALKYAKELGVNGSYSVNDGTNTNDFVK KVIDDALKNIEEELEKLAEPQNIEDKK |
| AAC44770.1\| FlaA protein (Borrelia burgdorferi) **SEQ ID NO 14** | MKRKAKSILFFLLSTVLFAQETDGLAEGSKRAEPGELVLDFAELARDPSSTRLDLTN YVDYVYSGASGIVKPEDMVVDLGINNWSVLLTPSARLQAYVKNSVVAPAVVKSESK RYAGDTILGVRVLFPSYSQSSAMIMPPFKIPFYSGESGNQFLGKGLIDNIKTMKEIKV SVYSLGYEIDLEVLFEDMNGMEYAYSMGTLKFKGWADLIWSNPNYIPNISSRIIKDD VPNYPLASSKMRFKAFRVSKSHSSKVKNFIFYVKDLRVLYDKLSVSIDSDIDSESVFK VYETSGTESLRKLKAHETFKRVLKLREKISIAEGSFQNFVEKIESEKPEESSPKN |
| BAD18055.1 FlaB protein [Borrelia garinii] SEQ ID NO 15 | NTSKAINFIQTTEGNLNEVEKVLVRMKELAVQSGNGTYSDADRGSIQIEIEQLTDEIN RIADQAQYNQMHMLSNKSASQNVRTAEELGMQPAKINTPASLSGSQASWTLRVHV GANQDEAIAVNIYAANVANLFSGEGAQAAQTAPVQEGVQQEGAQQPAPATAPSQG GVNSPVNVTTTVDANTSLAKIENAIRMISDQ |
| AAU07005.1\| flagellar filament 41 kDa core protein [Borrelia garinii PBi] SEQ ID NO 16 | MIINHNTSAINASRNNSINAANLSKTQEKLSSGYRINRASDDAAGMGVSGKINAQIRG LSQASRNTSKAINFIQTTEGNLNEVEKVLVRMKELAVQSGNGTYSDADRGSIQIEIE QLTDEINRIADQAQYNQMHMLSNKSASQNVRTAEELGMQPAKINTPASLSGSQAS WTLRVHVGANQDEAIAVNIYAANVANLFSGEGSQAAQTAPVQEGAQQEGAQQPA PATAPSQGGVNSPVNVTTTVDANTSLAKIENAIRMISDQRANLGAFQNRLESIKDST EYAIENLKASYAQIKDATMTDEVVASTTNSILTQSAMAMIAQANQVPQYVLSLLR |
| 1L8W\|A Chain A, Crystal Structure Of Lyme Disease Variable Surface Antigen VlsE1 Of Borrelia Burgdorferi SEQ ID NO 17 | MRGSHHHHHHGSSQVADKDDPTNKFYQSVIQLGNGFLDVFTSFGGLVAEAFGFKS DPKKSDVKTYFTTVAAKLEKTKTDLNSLPKEKSDISSTTGKPDSTGSVGTAVEGAIK EVSELLDKLVKAVKTAEGASSGTAAIGEVVADADAAKVADKASVKGIAKGIKEIVEAA GGSEKLKAVAAAKGENNKGAGKLFGKAGAAAHGDSEAASKAAGAVSAVSGEQILS AIVTAADAAEQDGKKPEEAKNPIAAAIGDKDGGAEFGQDEXKKDDQIAAAIALRGXA KDGKFAVKDGEKEKAEGAIKGAAESAVRKVLGAITGLIGDAVSSGLRKVGDSVKAA SKETPPALNK |
| CAA57807.1 Associated protein A | MKKISLLIFLFLFVVSLSANIEENYTETKRAFSKEDFNLINKRLDNYDFKNEYEKSHVF SDAPRIRGDLRKIGIKEKSVFLDALEAIEYLIKIKISTDSIFLSEDMIRLIGSYPDSIFNYLI QLNSDKIDYAEKYGDNARNNFKKDYSEDKANTVKQILKQILADLPKD |

| | |
|---|---|
| (BapA)[Borrelia burgdorferi] SEQ ID NO 18 | |
| AAL25643.1, P37-47 [Borrelia burgdorferi] SEQ ID NO 19 | MCAFLLLNLVNCKFDSLNLSTKSVDDKNNSIAKLLQHLSKSEDQANKTSTSEDQKEL EITENKEQEHEKLSQVAQHAPNSKIEKVKSDGKPVPGDKILSSNKDIYNSYIPEVKEE IVYEILEEVIIPETKIPEITEEVIMPIPQTIDFYIEPRPISSFLTQGTSPSITSTIKSYKELAK EKINNGLNIVQKITQNIDNITENLNSKETPKEISGKEVEEKITHPIFDHITGSGNNPGQD SISNTWGEGLEIGGDSNFFTNLEEVRSSIRTKIKVSDGTEQTKDKVEIDEIIEDLQKLK EFLEKLKKYLKDTNNLSAIEESVKGLS |
| NP_212481.1\| fibronectin/fibrino gen-binding protein, putative [Borrelia burgdorferi B31] SEQ ID NO 20 | MIKMSLNYTEINTLIKEIPFTNSLITKIIQPDYKSLVLEIYNKIDNKKFKILICLNPNTTRFH ITKKNFKKNALKLRFSDFLKSKIQNGKIIKAFQMKNERIISLEILQKDMIILFIKLWPSSP NIIATNSNFKILDAYYRRPKIKETTGEIFLKAKEIHESNKMSDKKIMELKEEYNNTSYTS YSEFLENYYESLNDQIKKTNIKELLIEKYKKELIVLEKRIDSLKQQIKLLENIENEKEKG ELILLNINKIQKGIKEINLLNYKEEKIKISLNQSLSPKENALQYFKAYKKGKNSFKTIQN QLKDNLDKFNLIQSKITMLKVENLIPEEEYNQEKTAIKEKEKTPKIGLHFTYCGFEILIG RNAKENDKLLRHCVKGNDYWLHTRDYPGAYVFIKNQKNKTPSLDVLLGAGNLCVF YTKLAKKSGKADLYYTQVKNLRRVKNKKLGLVIPKAEKNLHIKLDENLIKKIKNQT |
| AAC44656.1\| P30 Borrelia burgdorferi SEQ ID NO 21 | MKLQRSLSLIIFSLTVLCCDNKERKEGVSTKISLGAEPRSLDPQLAEDNVASKMIDTL YRGIVTGDPNTGGHKPGLAKGWETSSDGTAYPFYLRDNLTWSDGVAITAEGIRKSY LRILNKETGSTYVDMVKSIIKNGQEYFDGQVTDSELGIRAIDSKTLEITLASPKPYFIDL LVHQSFIPVPVHVTDKYGQNWTSPENMVTSGPFKLKERIPSEKYVFEKDNKYYDSN EVELEEITFYTTNDSSTAYKMYVNEELDAILVPYPQI |
| NP_045619.1\| immunogenic protein P37, putative [Borrelia burgdorferi B31] SEQ ID NO 22 | MNLINKLFILTILFSSVISCKLYKKITYNADQVIDKLKSNNGSFNTLKSNDDSKRSGRK PRSVDNTYMDQDTGKKPLMADMQPDMQNDNSSSNHTLQVNIQDNEASEARNIMT EIESSKEEYNRINEDLAKVKASLDKIKSLLSTAKSYLEQTRRGVGSSKANLALLPSLE EAIAKVKSNHASADTHCNDAIAALKRAKNDFEYAQRKADRALEEALSNSNASRHES YYYAGYHQFMADAKASMSSTKSLLEVAKNKQKELNENMTKTNKDFQELNDIYKKL QDMDSR |
| NP_212281.1\| flagellin [Borrelia burgdorferi B31] SEQ ID NO 23 | MIINHNTSAINASRNNGINAANLSKTQEKLSSGYRINRASDDAAGMGVSGKINAQIR GLSQASRNTSKAINFIQTTEGNLNEVEKVLVRMKELAVQSGNGTYSDADRGSIQIEI EQLTDEINRIADQAQYNQMHMLSNKSASQNVRTAEELGMQPAKINTPASLSGSQAS WTLRVHVGANQDEAIAVNIYAANVANLFSGEGAQTAQAAPVQEGVQQEGAQQPA PATAPSQGGVNSPVNVTTTVDANTSLAKIENAIRMISDQRANLGAFQNRLESIKNST EYAIENLKASYAQIKDATMTDEVVAATTNSILTQSAMAMIAQANQVPQYVLSLLR |
| NP_212463.1\| oligopeptide ABC transporter, periplasmic oligopeptide- binding protein (oppA-2) [Borrelia burgdorferi B31] SEQ ID NO 24 | MKLQRSLFLIIFFLTFLCCNNKERKEGVSFKISLGAEPSSLDPQLAEDNVASKMIDTM FRGIVTGDPNTGGNKPGLAKGWDISSDGTVYTFNLREKITWSDGVAITAEGIRKSYL RILNKETGSKYVEMVKSVIKNGQKYFDGQVTDSELGIRAIDEKTLEITLESPKPYFID MLVHQSFIPVPVHVTEKYGQNWTSPENMVTSGPFKLKERIPNEKYVFEKNNKYYDS NEVELEEITFYTTNDSSTAYKMYENEELDAIFGSIPPDLIKNLKLRSDYYSSAVNAIYF YAFNTHIKPLDNVKIRKALTLAIDRETLTYKVLDNGTTPTRRATPNFSSYSYAKSLELF NPEIAKTLLAEAGYPNGNGFPILKLKYNTNEANKKICEFIQNQWKKNLNIDVELENEE WTTYLNTKANGNYEIARAGWIGDYADPLTFLSIFTQGYTQFSSHNYSNPEYNELIKK SDLELDPIKRQDILRQAEEIIIEKDFPIAPIYIYGNSYLFRNDKWTGWNTNILERFDLSQ LKLKNK |
| AAC44381.1\| outer membrane porin protein Oms28 precursor SEQ ID NO 25 | MTKIFSNLIINGLLFGFVSLNVFADSNNANILKPQSNVLEHSDQKDNKKLDQKDQVN QALDTINKVTEDVSSKLEGVRESSLELVESNDAGVVKKFVGSMSLMSDVAKGTVVA SQEATIVAKCSGMVAEGANKVVEMSKKAVQETQKAVSVAGEATFLIEKQIMLNKSP NNKELELTKEEFAKVDEVKETLMASERALDETVQEAQKVLNMVNGLNPSNKDQVL AKKDVRKAISNVVKVAQGARDLTKVMAISLYMR |
| CAA49829.1\| p93 [Borrelia burgdorferi] SEQ ID NO 26 | MKKMLLIFSFFLIFLNGFPLNARKVDKEKLKDFVNMDLEFVNYKGPYDSTNTYEQIV GIGEFLARPLTNSNSNSSYYGKYFINRFIDDQDKKASVDVFSISSKSELDSILNLRRIL TGYIIKSFDYDRSSAELIAKVITIYNAVYRGDLDYYKGFYIEPALKSLTKENAGLSRVY SQWAGKTQIFIPLKKDILSGNIESDIDIDSLVTDKVIAALLSENEAGVNFARDITDIQGE THKADQDKIDTELDNIHESDSNITETIENLRDQLEKATDEEHKKEIESQVDAKKKEKE ELDKKAINLDKAQQKLDSAEDNLDVQRDTVREKIQEDINEINKEKNLPKPGDVSSPK VDKQLQIKESLEDLQEQLKEAGDENQKREIEKQIEIKKRDEELLKSKDGKVSKDYEA |

| | |
|---|---|
| | LDLDRELSKASSKEKSKVKEEEITKGKSRASLGDLNNDKNLMLPEDQKLPEDKKLD SKLDGKKEFKPVSEVEKLDKISKSNNNEVGKLSPLDKPSYDDIDSKEEVDNKAINLQ KIDPKVKDQTTSLNEDLDKDLTTMSIDSSSPVFLEVIDPITNLGTLQLIDLNTGVRLKE STQQGIQRYGIYEREKDLVVIKMDSGKAKLQILNKLENLKVVSESNFEINKNSSLYVD SKMILAAVRDKDDSNAWRLAKFSPKNLDEFILSENKILPFTSFSVRKNFIYLQDELKN LVILDVNTLKKVK |
| NP_212375.1\| glycerol kinase (glpK) [Borrelia burgdorferi B31] SEQ ID NO 27 | MKYILSIDQGTTSSRAMVFDKNANIKGFAQKEFTQIYPQPSWVEHDPTEIWGSQLG VITEAMANARILPNEIDAIGITNQRETTVIWEKNTGKPIYNAIVWQDRRTAKICDQLKK EGKDKIILEKTGLVLDSYFSGTKIMWILDNVEGARQRAENGELCFGTIDTWILWNLTQ KKEHATDYSNASRTLLLNIKTLEWDDELLSILNVPRAILPELKESSTIYGKTDKALFGA EIPIAGIAGDQFAATFGQACLKKGMAKNTYGTGCFLTVNIGKEPIISHDKLLTSIAWG RKKSVTYVLEGSVFIGGAVIQWLRDGLEFFRKSSDAEALASSVSDNGGVYFVPAFV GLGAPHWDSYARGTIIGITRGSTKAHITRAALESIAFQSFDILNTMKKSIPNFEIQELR VDGGASQNNLLMQFQADLLECKVVRPKITETTALGAAYLAGLATGYWQSAEEIVSL WQVDKIFEPSMPKNQKEKLLENWNKAVGKAKSWIQNSHSS |
| NP_212342.1\| hypothetical protein BB0208 [Borrelia burgdorferi B31] SEQ ID NO 28 | MKTIKKDSEFYDSLATLKKHINKYIEEKVLKYSISSQLYKLEKPEIIELIKISNDYEKEKN AFNTSLEECYYKNTQNEAIKKWILEIVRNKNFIQISKEANLNTKKLGTTYKLKSSNFLK LIEIQNSPYYSQEKKDIYKQIILNFSSNINIDSLEQTIDILVAVRNRNKIKILNILNKNLKY RPENQKVFKSSLTNKESRLIKLKRMLILTYWPVGCLSKNIFIKILIKXYKYLEEEILALK YNEILNYLRALKTLSLNEIFYKGSSKNISFNYFFSDFTQYAPKDFQDTLKCYLYVIEKT ITKKYLTWFFKDKISNNWESFIDALEYIEKHKLINIKEKIKEIITAKFQTEDFFISFDKTNF NPYESSIFKERYIKSAFLEIIMHYVKKNSQNIETYGWIAFYIYADNKDKKIFCQRMKEF FKSKTFEIQNQIFVYFLSFYPNIEYKHFKFISDILLYLDENKITIPKKIIKIQKINPNQLDY QKSYLLIKSLKTRSRILKIIIKNIRFNLIEKLEDENEKKLLPAMCYLIYCENLVELKNNRKI KSNEKNSLLEFISFFKTKLKQKINFKKELMKSKGI |
| NP_045482.1\| hypothetical protein BBG22 [Borrelia burgdorferi B31] SEQ ID NO 29 | MITQQDYTEIKKKLENTKTRIQFRNGNKIDSLGYQGSLGEPILLKDKLTFSICDGKNID NRKEYEITKNAPSLDSLIEYLEHMILFFLEKTPVGTLTAKFAMEGSILTRSKLIQAFTN TNKFCIPDGRSLPSNCYAYKVLGISSAPKLSGRFLRHYDSGGSTDSDGTRSLGHTQ EDSYKNHDHDLDFSRINFKGKLVKRMTMIYRSADTWYTWYWAEEIMSPFITGYSYE EIDPEYLLPPFTSQSGSKETKPKNTCYISFYRYDL |
| NP_212885.1\| hypothetical protein BB0751 [Borrelia burgdorferi B31] SEQ ID NO 30 | MDLLDLLEKEKQINKNKGVFMTKPKIFSINKEKIKILIIVVLTSTFLLGIIFSNENKVARIL EEKFFDFDFNLISKIETELEGTLTKLGKDWILTYNKQNIPVDNKKVNSLIKALDELQKN KLVSRDQKKHKELGIGENPSFKLFDNNNKLLTEIFVGKSGEGDSRLAYIKGSDENVY LTKNIFLSYKGNSYNTFSDTTLFQEKNTKLENLSFKIIRKLNKENENNINNNYEIISKD GLYFLNNQKMTKERPLNIIAEFKADGLEIDKSKIDDYNLQYKIEVKWSNKSVNNIEVY FNKNEENDKDILIKKDKDEYYYTTSKWTFFDVFDLEKKLTEKDDISSNDNQEDHHEH HNNAD |
| AAC67038.1\| predicted coding region BB0689 [Borrelia burgdorferi B31] SEQ ID NO 31 | MKKLIIIFTLFLSQACNLSTMHKIDTKEDMKILYSEIAELRKKLNLNHLEIDDTLEKVAK EYAIKLGENRTITHTLFGTTPMQRIHKYDQSFNLTREILASGIELNRVVNAWLNSPSH KEALINTDTDKIGGYRLKTTDNIDIFVVLFGKRKYKN |
| NP_045709.1\| lipoprotein BBA36[Borrelia burgdorferi B31] SEQ ID NO 32 | MMQRISILLMLLAVFSCKQFGDVKSLTEIDSGNGIPLVVSDVVKDLIPKEISLTPEEAE KLESLKVFLKDAMSVNGREEALKAEYEKSYKEFFDWLSKDVNRQKEFISSFDNISSI VSKAVDASKKRRPTEQQSLGFKEYVCYKIKNSKGEALSLFFQKVVDAFGADPYKKD NDESVQKPVKCNEEIFKVIKKVLTESESNNELKNLKNYGNV |
| AAT93789.1\| lipoprotein BBA36 (homolog 67%)[Borrelia garinii PBi] SEQ ID NO 33 | MKRISILSILLLLLLFSCKQYGDVKSLTEVATDLEDDNSFASGSVESKDQIIEKGPVLT SEEFERLEALKTFLKDAMGVNGREGDTKAEYEKSYKEFFDWLSKDVNRQKEFVSF FNNICGIITKAVDASKKRYNSNPKSLGFNEYVCYDIKTRTGDDLSLFFQKVADAFGT QEYKNKDEDDENNQKPEKCNEEIFKVIKRVFTESENNNELANLKNLNSYNLNSNNK |
| NP_045739.1\| BBA66 antigen, | MKIKPLIQLKLLGLFLFSCTIDANLNEDYKNKVKGILNKAADDQETTSADTNSNAAKNI PIADNDKVAAELKKQSQAAKTVAAAPNKGSQNQPQTTPNKGSQNQQAAPSPQLQ |

| P35, putative [Borrelia burgdorferi B31]SEQ ID NO 34 | SLSFSADLSNLPKTTAARAASLTKQRIPIQAVTTVPGNTRTFNSRNSGLPTFALNYSF SQPTRQQTNSSSAVQTTTSSGSKLQTLKNELIRAISEEKNKTQNNFGFRETYDQFK MKDSAFELLDVISSAKVYDRSYAPQLNSNTPEAENERNKFYALMDFDQYKIEQFGSI MEALYNENQNHSLIRELMISGLGTQISFELALEEINKKIEIFNQDYLNAKINSFDFTMK LKELKSKLNQILDKRKEWSRQADGLIANASSNSSLSDSKSLAEYIKKRYLDNMQNAR QSVLEAYISIM |
|---|---|
| AAT93824.1\|BBA 66 antigen, P35, putative [Borrelia garinii PBi] SEQ ID NO 35 | MSSCTIDANLNKDYKNKVEELLNSSTDDQAKISINTGSNATKNKTNIKVAGLQKNTQ SKKNNNLQGLNPANQVNPGNPMQIANQANQANQANQANQASQASQASQVA SSASQASPVASPATNVQATPPKQIASAQAIQTVPNNTSTPNQSIIKPQQYTFSSSFS QPTSQTNFNNSQSNNVLTNYRHQTQPSFVVPVYSGNSPLQKLKNNLLRRIAEEKNK THNHGFRETYDQFKMKDSAFTLLDVISNISVFDRGSAPQLSSNTPEAESERNRLYA MMDFDQAKTTEFGSIMNILYQENQNHSLIRSLIISGLGIQISLESTLEEIEKKIESFNTQ YLNTIINSYTFKDKLKELESKLNSILAEKKEWLNYADAIITNTSSNSKRNDPQSLGQYI KNKYLDKMQDARQSALDLYLNITEIR |
| NP_045742.1\| hypothetical protein BBA69 [Borrelia burgdorferi B31] SEQ ID NO 36 | MKKAKLNIIKINIITMILTLICISCAPFNKINPKANENTKLKKNTRLKKPANPGENIQNFK DKSGDLGASDEKFMGTTASELKAIGKELEDRKNQYDIQIAKITNEESNLLDTYIRAYE LANENEKMLLKRFLLSSLDYKKENIETLKEILEKLINNYENDPKIAANFLYRIALDIQLK LEKHLKSINEKLDTLSKENSKEDLEALLEQVKSALQLQEKFKKTLNKTLEDYRKNTN NIQENKVLAEHFNKYYKDSDSLQSAFY |
| AAT93826.1\| conserved hypothetical protein BBA69[Borrelia garinii PBi] SEQ ID NO 37 | MKKTKLNIIKLNILTTTLTLICISCAVNKIDPEPKSKTNKKENIKNFVNKFQDLEPSKKQ NKDLEPLREKYPEATASKLETTLKILEAQKEKENIEIAKIDNTQIDFLKTFKTDPHDSL PEDEKMQMKKIIYSSLNYETEKIKILQEILEKLDKNLQHKKIAKNFIYDIPIIIQSRLDIISK VIKNPIKDELQILNQKEIEEELLMRIESELKIKENFKKALNKTIDAYNQDSENIKTSAEQL EKHINENYKEFNSLKPIY |
| NP_045573.1\| hypothetical protein BBI42 [Borrelia burgdorferi B31] SEQ ID NO 38 | MRILVGVCIIALALLGCYLPDNQEQAVQTFFENSESSDMGSDEIVTEGIFSSLKLYAS EHRLLVEIKKTLISLKDPNYRGVVLPVSDYNEEYFNKFFLDLGSEQSKDLIKLFIMVK NEQNNNKFMRIVRWLYSCIEELYSPDIKYSGEEGSPEYYRNMPRPTAYQQYLKVKR YDYNRPVPILPT |
| NP_051318.1\| revA protein[Borrelia burgdorferi B31] SEQ ID NO 39 | MRNKNIFKLFFASMLFVMACKAYVEEKKEIDSLMEDVLALVNDSSGGKFKDYKDKIN ELKENLKDIGNAELKEKLLNLQNSFQDKLAAKLAALKAAKNTIENITDKDQDISKRKI WSEAKLVGVTVPLLGSNTSGNGDKMSKNAVEQIDKVIKFLEEGTN |
| NP_045737.1\| BBA64 antigen, P35 [Borrelia burgdorferi B31] SEQ ID NO 40 | MKDNILKNNKLIAIFLLHVLTVLILISCSLEVKDSNESKKHKKEKRKGKVENLLVAINNL KNPTKPAAGKNKANSKASKQKNNPNANANNAPKKILDPEVAKLIQKILDRSENIIQIS EMDSSRGEPNDQFGMRAEIFSKIFFNANSTVHFDSHEYTEERRMLYTSLNFNEGKI FNLGQILSKLSQDSNYRGLVKETLINRGFSIQLAMEEISAKILNVKDKLQQLNKPNLE TLYNDFEKLTSLKEKWLKDTDDLIDEYNTNPDLQTDVSKLNDTLRSKNSRAQFANIH DIILDLVNTTTNILAPIQ |
| AAT93822.1\| BBA64 antigen, P35 [Borrelia garinii PBi] SEQ ID NO 41 | MKNNKLIAIFLLHILTGLILLSCSLEVNQDDNQEKQKKAKTKTSKSENNSSKMKKLSK NAKNKKPTVDNLLVAINTLKNPPKTAGKNKSNSAAALKQPNNANALKQIDPEAKELI QKILERSEDIVQISEIDANKGEPDDQFEMKAEIFSKIFFNAGSTVTFDDNEYVNERRIL YTSLNFNENKILNLGKILSKLSQDSNYRSLVKEILINRGFSIQLAIEEISLRTLNVKDKIQ HLNKPNLKTLYHDFNKLIPLKEKWLKDVDDIIKDYNANPELRTDISKLNDYIISKNSKA QFTDIHNIILNLINTTTNILAPIQ |
| AAL84596.1\| BBK32 [Borrelia burgdorferi] SEQ ID NO 42 | MKKVKSKYLALGLLFGFISCDLFIRYEMKEESPGLFDKGNSILETSEESIKKPMNKKG KGKIARKKGKSKVSRKEPYIHSLKRDSANKSNFLQKNVILEEESLKTELLKEQSETR KEKIQKQQDEYKGMTQGSLNSLSGESGELKETIESNEIDITIDSDLRPKSSLQDIAGS NSISYTDEIEEEDYARYYLDEDDEDDEYYEDDYEEIRLSNRYQSYLEGVKYNVDSAI NTINKIYDTYTLFSTKLTQMYSTRLDNLAKAKAKEEAAKFTKEDLEKNFKTLLNYIQV SVKTAANFVYINDTHAKRKLENIEAEIKTLIAKIKEQSNLYEAYKAIVTSILLMRDSLKE |

| | VQGIIDKNGVWY |
|---|---|
| AAL84590.1\| BBK32 [Borrelia afzelii] SEQ ID NO 43 | MKIKSKCLALGLLFGFISCDLFIRDEIKEKSLGLCDEESSILETGDKSVKKSLNKKGKD KVARKKVEGNAVKKDPFNHHVKRESVNNSNLSQKNVISEEEILKTKLLRERPETRK EEIQKQQDEHKRMLQGSLSFLSGESGELKDTIESNEIDFTIDSDLRLKSDLQAISGSN SISYTDEIEEEDYDQYSLEEDYYYDGETRLSNRYESYLEGVKYNVSSAIKTIVKIYDN YTLLSTKQTQMYSTRLDNLAKAKAREEAKKFTKEELEKDLKTLLNYIQVSARTATNF VYAREIYSKRKLDAIETEIKNLILKIKGQSDLYEAYKAIVRSILLMKDSLKIIEIVIDKNGV WY |
| AAL84595.1\| BBK32 [Borrelia garinii] SEQ ID NO 44 | MKKVKSKYLALGLLFGFISCDLFIRYEMKEESPGLFDKGNSILETSEESIKKPMNKKG KGKIARKNGKSKVSGKEPFIHSFKRDAANKSNFLQKNVMLEEESLKTELLKEQSET RKEKIQKQQDEYKGMTKGSLNSLSGESGELKETIESNEIDITIDSDLRPKSSLQDIAG SNSISYTDEIEEEDYARYYLDEDDEDDEYYEDDYEEIRLSNRYQSYLEGVKYNVDSA INTINKIYDTYTLFSTKLTQMYSTRLDNLAKAKAKEEAAKFTKEDLEKNFKTLLNYIQV SVKTATNFVYINEMHAKRKLENIEAKIKTLIAKIKEKSNLYSAYKAIVSSILLMRDSLKE VQYAIDKNGIWY |

## Fig. 31.

| Antigen designation | Class I Antigen peptide sequences SEQ ID NO/sequences |
|---|---|
| NP_212517.1 Basic membrane protein A (bmpA) [Borrelia burgdorferi B31 | SEQ ID NO 45-1366/<br>8 mers:<br>MNKILLLI; NKILLLIL; KILLLILL; ILLLILLE; LLLILLES; LLILLESI;<br>LILLESIV; ILLESIVF; LLESIVFL; LESIVFLS; ESIVFLSC; SIVFLSCS;<br>IVFLSCSG; VFLSCSGK; FLSCSGKG; LSCSGKGS; SCSGKGSL; CSGKGSLG;<br>SGKGSLGS; GKGSLGSE; KGSLGSEI; GSLGSEIP; SLGSEIPK; LGSEIPKV;<br>GSEIPKVS; SEIPKVSL; EIPKVSLI; IPKVSLII; PKVSLIID; KVSLIIDG;<br>VSLIIDGT; SLIIDGTF; LIIDGTFD; IIDGTFDD; IDGTFDDK; DGTFDDKS;<br>GTFDDKSF; TFDDKSFN; FDDKSFNE; DDKSFNES; DKSFNESA; KSFNESAL;<br>SFNESALN; FNESALNG; NESALNGV; ESALNGVK; SALNGVKK; ALNGVKKV;<br>LNGVKKVK; NGVKKVKE; GVKKVKEE; VKKVKEEF; KKVKEEFK; KVKEEFKI;<br>VKEEFKIE; KEEFKIEL; EEFKIELV; EFKIELVL; FKIELVLK; KIELVLKE;<br>IELVLKES; ELVLKESS; LVLKESSS; VLKESSSN; LKESSSNS; KESSSNSY;<br>ESSSNSYL; SSSNSYLS; SSNSYLSD; SNSYLSDL; NSYLSDLE; SYLSDLEG;<br>YLSDLEGL; LSDLEGLK; SDLEGLKD; DLEGLKDA; LEGLKDAG; EGLKDAGS;<br>GLKDAGSD; LKDAGSDL; KDAGSDLI; DAGSDLIW; AGSDLIWL; GSDLIWLI;<br>SDLIWLIG; DLIWLIGY; LIWLIGYR; IWLIGYRF; WLIGYRFS; LIGYRFSD;<br>IGYRFSDV; GYRFSDVA; YRFSDVAK; RFSDVAKV; FSDVAKVA; SDVAKVAA;<br>DVAKVAAL; VAKVAALQ; AKVAALQN; KVAALQNP; VAALQNPD; AALQNPDM;<br>ALQNPDMK; LQNPDMKY; QNPDMKYA; NPDMKYAI; PDMKYAII; DMKYAIID;<br>MKYAIIDP; KYAIIDPI; YAIIDPIY; AIIDPIYS; IIDPIYSN; IDPIYSND;<br>DPIYSNDP; PIYSNDPI; IYSNDPIP; YSNDPIPA; SNDPIPAN; NDPIPANL;<br>DPIPANLV; PIPANLVG; IPANLVGM; PANLVGMT; ANLVGMTF; NLVGMTFR;<br>LVGMTFRA; VGMTFRAQ; GMTFRAQE; MTFRAQEG; TFRAQEGA; FRAQEGAF;<br>RAQEGAFL; AQEGAFLT; QEGAFLTG; EGAFLTGY; GAFLTGYI; AFLTGYIA;<br>FLTGYIAA; LTGYIAAK; TGYIAAKL; GYIAAKLS; YIAAKLSK; IAAKLSKT;<br>AAKLSKTG; AKLSKTGK; KLSKTGKI; LSKTGKIG; SKTGKIGF; KTGKIGFL;<br>TGKIGFLG; GKIGFLGG; KIGFLGGI; IGFLGGIE; GFLGGIEG; FLGGIEGE;<br>LGGIEGEI; GGIEGEIV; GIEGEIVD; IEGEIVDA; EGEIVDAF; GEIVDAFR;<br>EIVDAFRY; IVDAFRYG; VDAFRYGY; DAFRYGYE; AFRYGYEA; FRYGYEAG;<br>RYGYEAGA; YGYEAGAK; GYEAGAKY; YEAGAKYA; EAGAKYAN; AGAKYANK;<br>GAKYANKD; AKYANKDI; KYANKDIK; YANKDIKI; ANKDIKIS; NKDIKIST;<br>KDIKISTQ; DIKISTQY; IKISTQYI; KISTQYIG; ISTQYIGS; STQYIGSF;<br>TQYIGSFA; QYIGSFAD; YIGSFADL; IGSFADLE; GSFADLEA; SFADLEAG;<br>FADLEAGR; ADLEAGRS; DLEAGRSV; LEAGRSVA; EAGRSVAT; AGRSVATR;<br>GRSVATRM; RSVATRMY; SVATRMYS; VATRMYSD; ATRMYSDE; TRMYSDEI;<br>RMYSDEID; MYSDEIDI; YSDEIDII; SDEIDIIH; DEIDIIHH; EIDIIHHA;<br>IDIIHHAA; DIIHHAAG; IIHHAAGL; IHHAAGLG; HHAAGLGG; HAAGLGGI;<br>AAGLGGIG; AGLGGIGA; GLGGIGAI; LGGIGAIE; GGIGAIEV; GIGAIEVA;<br>IGAIEVAK; GAIEVAKE; AIEVAKEL; IEVAKELG; EVAKELGS; VAKELGSG;<br>AKELGSGH; KELGSGHY; ELGSGHYI; LGSGHYII; GSGHYIIG; SGHYIIGV;<br>GHYIIGVD; HYIIGVDE; YIIGVDED; IIGVDEDQ; IGVDEDQA; GVDEDQAY;<br>VDEDQAYL; DEDQAYLA; EDQAYLAP; DQAYLAPD; QAYLAPDN; AYLAPDNV;<br>YLAPDNVI; LAPDNVIT; APDNVITS; PDNVITST; DNVITSTT; NVITSTTK;<br>VITSTTKD; ITSTTKDV; TSTTKDVG; STTKDVGR; TTKDVGRA; TKDVGRAL;<br>KDVGRALN; DVGRALNI; VGRALNIF; GRALNIFT; RALNIFTS; ALNIFTSN;<br>LNIFTSNH; NIFTSNHL; IFTSNHLK; FTSNHLKT; TSNHLKTN; SNHLKTNT;<br>NHLKTNTF; HLKTNTFE; LKTNTFEG; KTNTFEGG; TNTFEGGK; NTFEGGKL;<br>TFEGGKLI; FEGGKLIN; EGGKLINY; GGKLINYG; GKLINYGL; KLINYGLK;<br>LINYGLKE; INYGLKEG; NYGLKEGV; YGLKEGVV; GLKEGVVG; LKEGVVGF;<br>KEGVVGFV; EGVVGFVR; GVVGFVRN; VVGFVRNP; VGFVRNPK; GFVRNPKM;<br>FVRNPKMI; VRNPKMIS; RNPKMISF; NPKMISFE; PKMISFEL; KMISFELE; |

MISFELEK; ISFELEKE; SFELEKEI; FELEKEID; ELEKEIDN; LEKEIDNL;
EKEIDNLS; KEIDNLSS; EIDNLSSK; IDNLSSKI; DNLSSKII; NLSSKIIN;
LSSKIINK; SSKIINKE; SKIINKEI; KIINKEII; IINKEIIV; INKEIIVP;
NKEIIVPS; KEIIVPSN; EIIVPSNK; IIVPSNKE; IVPSNKES; VPSNKESY;
PSNKESYE; SNKESYEK; NKESYEKF; KESYEKFL; ESYEKFLK; SYEKFLKE;
YEKFLKEF; EKFLKEFI;

9 mers:
MNKILLLIL; NKILLLILL; KILLLILLE; ILLLILLES; LLLILLESI;
LLILLESIV; LILLESIVF; ILLESIVFL; LLESIVFLS; LESIVFLSC;
ESIVFLSCS; SIVFLSCSG; IVFLSCSGK; VFLSCSGKG; FLSCSGKGS;
LSCSGKGSL; SCSGKGSLG; CSGKGSLGS; SGKGSLGSE; GKGSLGSEI;
KGSLGSEIP; GSLGSEIPK; SLGSEIPKV; LGSEIPKVS; GSEIPKVSL;
SEIPKVSLI; EIPKVSLII; IPKVSLIID; PKVSLIIDG; KVSLIIDGT;
VSLIIDGTF; SLIIDGTFD; LIIDGTFDD; IIDGTFDDK; IDGTFDDKS;
DGTFDDKSF; GTFDDKSFN; TFDDKSFNE; FDDKSFNES; DDKSFNESA;
DKSFNESAL; KSFNESALN; SFNESALNG; FNESALNGV; NESALNGVK;
ESALNGVKK; SALNGVKKV; ALNGVKKVK; LNGVKKVKE; NGVKKVKEE;
GVKKVKEEF; VKKVKEEFK; KKVKEEFKI; KVKEEFKIE; VKEEFKIEL;
KEEFKIELV; EEFKIELVL; EFKIELVLK; FKIELVLKE; KIELVLKES;
IELVLKESS; ELVLKESSS; LVLKESSSN; VLKESSSNS; LKESSSNSY;
KESSSNSYL; ESSSNSYLS; SSSNSYLSD; SSNSYLSDL; SNSYLSDLE;
NSYLSDLEG; SYLSDLEGL; YLSDLEGLK; LSDLEGLKD; SDLEGLKDA;
DLEGLKDAG; LEGLKDAGS; EGLKDAGSD; GLKDAGSDL; LKDAGSDLI;
KDAGSDLIW; DAGSDLIWL; AGSDLIWLI; GSDLIWLIG; SDLIWLIGY;
DLIWLIGYR; LIWLIGYRF; IWLIGYRFS; WLIGYRFSD; LIGYRFSDV;
IGYRFSDVA; GYRFSDVAK; YRFSDVAKV; RFSDVAKVA; FSDVAKVAA;
SDVAKVAAL; DVAKVAALQ; VAKVAALQN; AKVAALQNP; KVAALQNPD;
VAALQNPDM; AALQNPDMK; ALQNPDMKY; LQNPDMKYA; QNPDMKYAI;
NPDMKYAII; PDMKYAIID; DMKYAIIDP; MKYAIIDPI; KYAIIDPIY;
YAIIDPIYS; AIIDPIYSN; IIDPIYSND; IDPIYSNDP; DPIYSNDPI;
PIYSNDPIP; IYSNDPIPA; YSNDPIPAN; SNDPIPANL; NDPIPANLV;
DPIPANLVG; PIPANLVGM; IPANLVGMT; PANLVGMTF; ANLVGMTFR;
NLVGMTFRA; LVGMTFRAQ; VGMTFRAQE; GMTFRAQEG; MTFRAQEGA;
TFRAQEGAF; FRAQEGAFL; RAQEGAFLT; AQEGAFLTG; QEGAFLTGY;
EGAFLTGYI; GAFLTGYIA; AFLTGYIAA; FLTGYIAAK; LTGYIAAKL;
TGYIAAKLS; GYIAAKLSK; YIAAKLSKT; IAAKLSKTG; AAKLSKTGK;
AKLSKTGKI; KLSKTGKIG; LSKTGKIGF; SKTGKIGFL; KTGKIGFLG;
TGKIGFLGG; GKIGFLGGI; KIGFLGGIE; IGFLGGIEG; GFLGGIEGE;
FLGGIEGEI; LGGIEGEIV; GGIEGEIVD; GIEGEIVDA; IEGEIVDAF;
EGEIVDAFR; GEIVDAFRY; EIVDAFRYG; IVDAFRYGY; VDAFRYGYE;
DAFRYGYEA; AFRYGYEAG; FRYGYEAGA; RYGYEAGAK; YGYEAGAKY;
GYEAGAKYA; YEAGAKYAN; EAGAKYANK; AGAKYANKD; GAKYANKDI;
AKYANKDIK; KYANKDIKI; YANKDIKIS; ANKDIKIST; NKDIKISTQ;
KDIKISTQY; DIKISTQYI; IKISTQYIG; KISTQYIGS; ISTQYIGSF;
STQYIGSFA; TQYIGSFAD; QYIGSFADL; YIGSFADLE; IGSFADLEA;
GSFADLEAG; SFADLEAGR; FADLEAGRS; ADLEAGRSV; DLEAGRSVA;
LEAGRSVAT; EAGRSVATR; AGRSVATRM; GRSVATRMY; RSVATRMYS;
SVATRMYSD; VATRMYSDE; ATRMYSDEI; TRMYSDEID; RMYSDEIDI;
MYSDEIDII; YSDEIDIIH; SDEIDIIHH; DEIDIIHHA; EIDIIHHAA;
IDIIHHAAG; DIIHHAAGL; IIHHAAGLG; IHHAAGLGG; HHAAGLGGI;
HAAGLGGIG; AAGLGGIGA; AGLGGIGAI; GLGGIGAIE; LGGIGAIEV;
GGIGAIEVA; GIGAIEVAK; IGAIEVAKE; GAIEVAKEL; AIEVAKELG;
IEVAKELGS; EVAKELGSG; VAKELGSGH; AKELGSGHY; KELGSGHYI;
ELGSGHYII; LGSGHYIIG; GSGHYIIGV; SGHYIIGVD; GHYIIGVDE;
HYIIGVDED; YIIGVDEDQ; IIGVDEDQA; IGVDEDQAY; GVDEDQAYL;
VDEDQAYLA; DEDQAYLAP; EDQAYLAPD; DQAYLAPDN; QAYLAPDNV;

EP 2 254 592 B1

AYLAPDNVI; YLAPDNVIT; LAPDNVITS; APDNVITST; PDNVITSTT;
DNVITSTTK; NVITSTTKD; VITSTTKDV; ITSTTKDVG; TSTTKDVGR;
STTKDVGRA; TTKDVGRAL; TKDVGRALN; KDVGRALNI; DVGRALNIF;
VGRALNIFT; GRALNIFTS; RALNIFTSN; ALNIFTSNH; LNIFTSNHL;
NIFTSNHLK; IFTSNHLKT; FTSNHLKTN; TSNHLKTNT; SNHLKTNTF;
NHLKTNTFE; HLKTNTFEG; LKTNTFEGG; KTNTFEGGK; TNTFEGGKL;
NTFEGGKLI; TFEGGKLIN; FEGGKLINY; EGGKLINYG; GGKLINYGL;
GKLINYGLK; KLINYGLKE; LINYGLKEG; INYGLKEGV; NYGLKEGVV;
YGLKEGVVG; GLKEGVVGF; LKEGVVGFV; KEGVVGFVR; EGVVGFVRN;
GVVGFVRNP; VVGFVRNPK; VGFVRNPKM; GFVRNPKMI; FVRNPKMIS;
VRNPKMISF; RNPKMISFE; NPKMISFEL; PKMISFELE; KMISFELEK;
MISFELEKE; ISFELEKEI; SFELEKEID; FELEKEIDN; ELEKEIDNL;
LEKEIDNLS; EKEIDNLSS; KEIDNLSSK; EIDNLSSKI; IDNLSSKII;
DNLSSKIIN; NLSSKIINK; LSSKIINKE; SSKIINKEI; SKIINKEII;
KIINKEIIV; IINKEIIVP; INKEIIVPS; NKEIIVPSN; KEIIVPSNK;
EIIVPSNKE; IIVPSNKES; IVPSNKESY; VPSNKESYE; PSNKESYEK;
SNKESYEKF; NKESYEKFL; KESYEKFLK; ESYEKFLKE; SYEKFLKEF;
YEKFLKEFI;

10 mers:
MNKILLLILL; NKILLLILLE; KILLLILLES; ILLLILLESI; LLLILLESIV;
LLILLESIVF; LILLESIVFL; ILLESIVFLS; LLESIVFLSC; LESIVFLSCS;
ESIVFLSCSG; SIVFLSCSGK; IVFLSCSGKG; VFLSCSGKGS; FLSCSGKGSL;
LSCSGKGSLG; SCSGKGSLGS; CSGKGSLGSE; SGKGSLGSEI; GKGSLGSEIP;
KGSLGSEIPK; GSLGSEIPKV; SLGSEIPKVS; LGSEIPKVSL; GSEIPKVSLI;
SEIPKVSLII; EIPKVSLIID; IPKVSLIIDG; PKVSLIIDGT; KVSLIIDGTF;
VSLIIDGTFD; SLIIDGTFDD; LIIDGTFDDK; IIDGTFDDKS; IDGTFDDKSF;
DGTFDDKSFN; GTFDDKSFNE; TFDDKSFNES; FDDKSFNESA; DDKSFNESAL;
DKSFNESALN; KSFNESALNG; SFNESALNGV; FNESALNGVK; NESALNGVKK;
ESALNGVKKV; SALNGVKKVK; ALNGVKKVKE; LNGVKKVKEE; NGVKKVKEEF;
GVKKVKEEFK; VKKVKEEFKI; KKVKEEFKIE; KVKEEFKIEL; VKEEFKIELV;
KEEFKIELVL; EEFKIELVLK; EFKIELVLKE; FKIELVLKES; KIELVLKESS;
IELVLKESSS; ELVLKESSSN; LVLKESSSNS; VLKESSSNSY; LKESSSNSYL;
KESSSNSYLS; ESSSNSYLSD; SSSNSYLSDL; SSNSYLSDLE; SNSYLSDLEG;
NSYLSDLEGL; SYLSDLEGLK; YLSDLEGLKD; LSDLEGLKDA; SDLEGLKDAG;
DLEGLKDAGS; LEGLKDAGSD; EGLKDAGSDL; GLKDAGSDLI; LKDAGSDLIW;
KDAGSDLIWL; DAGSDLIWLI; AGSDLIWLIG; GSDLIWLIGY; SDLIWLIGYR;
DLIWLIGYRF; LIWLIGYRFS; IWLIGYRFSD; WLIGYRFSDV; LIGYRFSDVA;
IGYRFSDVAK; GYRFSDVAKV; YRFSDVAKVA; RFSDVAKVAA; FSDVAKVAAL;
SDVAKVAALQ; DVAKVAALQN; VAKVAALQNP; AKVAALQNPD; KVAALQNPDM;
VAALQNPDMK; AALQNPDMKY; ALQNPDMKYA; LQNPDMKYAI; QNPDMKYAII;
NPDMKYAIID; PDMKYAIIDP; DMKYAIIDPI; MKYAIIDPIY; KYAIIDPIYS;
YAIIDPIYSN; AIIDPIYSND; IIDPIYSNDP; IDPIYSNDPI; DPIYSNDPIP;
PIYSNDPIPA; IYSNDPIPAN; YSNDPIPANL; SNDPIPANLV; NDPIPANLVG;
DPIPANLVGM; PIPANLVGMT; IPANLVGMTF; PANLVGMTFR; ANLVGMTFRA;
NLVGMTFRAQ; LVGMTFRAQE; VGMTFRAQEG; GMTFRAQEGA; MTFRAQEGAF;
TFRAQEGAFL; FRAQEGAFLT; RAQEGAFLTG; AQEGAFLTGY; QEGAFLTGYI;
EGAFLTGYIA; GAFLTGYIAA; AFLTGYIAAK; FLTGYIAAKL; LTGYIAAKLS;
TGYIAAKLSK; GYIAAKLSKT; YIAAKLSKTG; IAAKLSKTGK; AAKLSKTGKI;
AKLSKTGKIG; KLSKTGKIGF; LSKTGKIGFL; SKTGKIGFLG; KTGKIGFLGG;
TGKIGFLGGI; GKIGFLGGIE; KIGFLGGIEG; IGFLGGIEGE; GFLGGIEGEI;
FLGGIEGEIV; LGGIEGEIVD; GGIEGEIVDA; GIEGEIVDAF; IEGEIVDAFR;
EGEIVDAFRY; GEIVDAFRYG; EIVDAFRYGY; IVDAFRYGYE; VDAFRYGYEA;
DAFRYGYEAG; AFRYGYEAGA; FRYGYEAGAK; RYGYEAGAKY; YGYEAGAKYA;
GYEAGAKYAN; YEAGAKYANK; EAGAKYANKD; AGAKYANKDI; GAKYANKDIK;
AKYANKDIKI; KYANKDIKIS; YANKDIKIST; ANKDIKISTQ; NKDIKISTQY;
KDIKISTQYI; DIKISTQYIG; IKISTQYIGS; KISTQYIGSF; ISTQYIGSFA;

STQYIGSFAD; TQYIGSFADL; QYIGSFADLE; YIGSFADLEA; IGSFADLEAG;
GSFADLEAGR; SFADLEAGRS; FADLEAGRSV; ADLEAGRSVA; DLEAGRSVAT;
LEAGRSVATR; EAGRSVATRM; AGRSVATRMY; GRSVATRMYS; RSVATRMYSD;
SVATRMYSDE; VATRMYSDEI; ATRMYSDEID; TRMYSDEIDI; RMYSDEIDII;
MYSDEIDIIH; YSDEIDIIHH; SDEIDIIHHA; DEIDIIHHAA; EIDIIHHAAG;
IDIIHHAAGL; DIIHHAAGLG; IIHHAAGLGG; IHHAAGLGGI; HHAAGLGGIG;
HAAGLGGIGA; AAGLGGIGAI; AGLGGIGAIE; GLGGIGAIEV; LGGIGAIEVA;
GGIGAIEVAK; GIGAIEVAKE; IGAIEVAKEL; GAIEVAKELG; AIEVAKELGS;
IEVAKELGSG; EVAKELGSGH; VAKELGSGHY; AKELGSGHYI; KELGSGHYII;
ELGSGHYIIG; LGSGHYIIGV; GSGHYIIGVD; SGHYIIGVDE; GHYIIGVDED;
HYIIGVDEDQ; YIIGVDEDQA; IIGVDEDQAY; IGVDEDQAYL; GVDEDQAYLA;
VDEDQAYLAP; DEDQAYLAPD; EDQAYLAPDN; DQAYLAPDNV; QAYLAPDNVI;
AYLAPDNVIT; YLAPDNVITS; LAPDNVITST; APDNVITSTT; PDNVITSTTK;
DNVITSTTKD; NVITSTTKDV; VITSTTKDVG; ITSTTKDVGR; TSTTKDVGRA;
STTKDVGRAL; TTKDVGRALN; TKDVGRALNI; KDVGRALNIF; DVGRALNIFT;
VGRALNIFTS; GRALNIFTSN; RALNIFTSNH; ALNIFTSNHL; LNIFTSNHLK;
NIFTSNHLKT; IFTSNHLKTN; FTSNHLKTNT; TSNHLKTNTF; SNHLKTNTFE;
NHLKTNTFEG; HLKTNTFEGG; LKTNTFEGGK; KTNTFEGGKL; TNTFEGGKLI;
NTFEGGKLIN; TFEGGKLINY; FEGGKLINYG; EGGKLINYGL; GGKLINYGLK;
GKLINYGLKE; KLINYGLKEG; LINYGLKEGV; INYGLKEGVV; NYGLKEGVVG;
YGLKEGVVGF; GLKEGVVGFV; LKEGVVGFVR; KEGVVGFVRN; EGVVGFVRNP;
GVVGFVRNPK; VVGFVRNPKM; VGFVRNPKMI; GFVRNPKMIS; FVRNPKMISF;
VRNPKMISFE; RNPKMISFEL; NPKMISFELE; PKMISFELEK; KMISFELEKE;
MISFELEKEI; ISFELEKEID; SFELEKEIDN; FELEKEIDNL; ELEKEIDNLS;
LEKEIDNLSS; EKEIDNLSSK; KEIDNLSSKI; EIDNLSSKII; IDNLSSKIIN;
DNLSSKIINK; NLSSKIINKE; LSSKIINKEI; SSKIINKEII; SKIINKEIIV;
KIINKEIIVP; IINKEIIVPS; INKEIIVPSN; NKEIIVPSNK; KEIIVPSNKE;
EIIVPSNKES; IIVPSNKESY; IVPSNKESYE; VPSNKESYEK; PSNKESYEKF;
SNKESYEKFL; NKESYEKFLK; KESYEKFLKE; ESYEKFLKEF; SYEKFLKEFI;

11 mers:
MNKILLLILLE; NKILLLILLES; KILLLILLESI; ILLLILLESIV;
LLLILLESIVF; LLILLESIVFL; LILLESIVFLS; ILLESIVFLSC;
LLESIVFLSCS; LESIVFLSCSG; ESIVFLSCSGK; SIVFLSCSGKG;
IVFLSCSGKGS; VFLSCSGKGSL; FLSCSGKGSLG; LSCSGKGSLGS;
SCSGKGSLGSE; CSGKGSLGSEI; SGKGSLGSEIP; GKGSLGSEIPK;
KGSLGSEIPKV; GSLGSEIPKVS; SLGSEIPKVSL; LGSEIPKVSLI;
GSEIPKVSLII; SEIPKVSLIID; EIPKVSLIIDG; IPKVSLIIDGT;
PKVSLIIDGTF; KVSLIIDGTFD; VSLIIDGTFDD; SLIIDGTFDDK;
LIIDGTFDDKS; IIDGTFDDKSF; IDGTFDDKSFN; DGTFDDKSFNE;
GTFDDKSFNES; TFDDKSFNESA; FDDKSFNESAL; DDKSFNESALN;
DKSFNESALNG; KSFNESALNGV; SFNESALNGVK; FNESALNGVKK;
NESALNGVKKV; ESALNGVKKVK; SALNGVKKVKE; ALNGVKKVKEE;
LNGVKKVKEEF; NGVKKVKEEFK; GVKKVKEEFKI; VKKVKEEFKIE;
KKVKEEFKIEL; KVKEEFKIELV; VKEEFKIELVL; KEEFKIELVLK;
EEFKIELVLKE; EFKIELVLKES; FKIELVLKESS; KIELVLKESSS;
IELVLKESSSN; ELVLKESSSNS; LVLKESSSNSY; VLKESSSNSYL;
LKESSSNSYLS; KESSSNSYLSD; ESSSNSYLSDL; SSSNSYLSDLE;
SSNSYLSDLEG; SNSYLSDLEGL; NSYLSDLEGLK; SYLSDLEGLKD;
YLSDLEGLKDA; LSDLEGLKDAG; SDLEGLKDAGS; DLEGLKDAGSD;
LEGLKDAGSDL; EGLKDAGSDLI; GLKDAGSDLIW; LKDAGSDLIWL;
KDAGSDLIWLI; DAGSDLIWLIG; AGSDLIWLIGY; GSDLIWLIGYR;
SDLIWLIGYRF; DLIWLIGYRFS; LIWLIGYRFSD; IWLIGYRFSDV;
WLIGYRFSDVA; LIGYRFSDVAK; IGYRFSDVAKV; GYRFSDVAKVA;
YRFSDVAKVAA; RFSDVAKVAAL; FSDVAKVAALQ; SDVAKVAALQN;
DVAKVAALQNP; VAKVAALQNPD; AKVAALQNPDM; KVAALQNPDMK;
VAALQNPDMKY; AALQNPDMKYA; ALQNPDMKYAI; LQNPDMKYAII;

QNPDMKYAIID; NPDMKYAIIDP; PDMKYAIIDPI; DMKYAIIDPIY;
MKYAIIDPIYS; KYAIIDPIYSN; YAIIDPIYSND; AIIDPIYSNDP;
IIDPIYSNDPI; IDPIYSNDPIP; DPIYSNDPIPA; PIYSNDPIPAN;
IYSNDPIPANL; YSNDPIPANLV; SNDPIPANLVG; NDPIPANLVGM;
DPIPANLVGMT; PIPANLVGMTF; IPANLVGMTFR; PANLVGMTFRA;
ANLVGMTFRAQ; NLVGMTFRAQE; LVGMTFRAQEG; VGMTFRAQEGA;
GMTFRAQEGAF; MTFRAQEGAFL; TFRAQEGAFLT; FRAQEGAFLTG;
RAQEGAFLTGY; AQEGAFLTGYI; QEGAFLTGYIA; EGAFLTGYIAA;
GAFLTGYIAAK; AFLTGYIAAKL; FLTGYIAAKLS; LTGYIAAKLSK;
TGYIAAKLSKT; GYIAAKLSKTG; YIAAKLSKTGK; IAAKLSKTGKI;
AAKLSKTGKIG; AKLSKTGKIGF; KLSKTGKIGFL; LSKTGKIGFLG;
SKTGKIGFLGG; KTGKIGFLGGI; TGKIGFLGGIE; GKIGFLGGIEG;
KIGFLGGIEGE; IGFLGGIEGEI; GFLGGIEGEIV; FLGGIEGEIVD;
LGGIEGEIVDA; GGIEGEIVDAF; GIEGEIVDAFR; IEGEIVDAFRY;
EGEIVDAFRYG; GEIVDAFRYGY; EIVDAFRYGYE; IVDAFRYGYEA;
VDAFRYGYEAG; DAFRYGYEAGA; AFRYGYEAGAK; FRYGYEAGAKY;
RYGYEAGAKYA; YGYEAGAKYAN; GYEAGAKYANK; YEAGAKYANKD;
EAGAKYANKDI; AGAKYANKDIK; GAKYANKDIKI; AKYANKDIKIS;
KYANKDIKIST; YANKDIKISTQ; ANKDIKISTQY; NKDIKISTQYI;
KDIKISTQYIG; DIKISTQYIGS; IKISTQYIGSF; KISTQYIGSFA;
ISTQYIGSFAD; STQYIGSFADL; TQYIGSFADLE; QYIGSFADLEA;
YIGSFADLEAG; IGSFADLEAGR; GSFADLEAGRS; SFADLEAGRSV;
FADLEAGRSVA; ADLEAGRSVAT; DLEAGRSVATR; LEAGRSVATRM;
EAGRSVATRMY; AGRSVATRMYS; GRSVATRMYSD; RSVATRMYSDE;
SVATRMYSDEI; VATRMYSDEID; ATRMYSDEIDI; TRMYSDEIDII;
RMYSDEIDIIH; MYSDEIDIIHH; YSDEIDIIHHA; SDEIDIIHHAA;
DEIDIIHHAAG; EIDIIHHAAGL; IDIIHHAAGLG; DIIHHAAGLGG;
IIHHAAGLGGI; IHHAAGLGGIG; HHAAGLGGIGA; HAAGLGGIGAI;
AAGLGGIGAIE; AGLGGIGAIEV; GLGGIGAIEVA; LGGIGAIEVAK;
GGIGAIEVAKE; GIGAIEVAKEL; IGAIEVAKELG; GAIEVAKELGS;
AIEVAKELGSG; IEVAKELGSGH; EVAKELGSGHY; VAKELGSGHYI;
AKELGSGHYII; KELGSGHYIIG; ELGSGHYIIGV; LGSGHYIIGVD;
GSGHYIIGVDE; SGHYIIGVDED; GHYIIGVDEDQ; HYIIGVDEDQA;
YIIGVDEDQAY; IIGVDEDQAYL; IGVDEDQAYLA; GVDEDQAYLAP;
VDEDQAYLAPD; DEDQAYLAPDN; EDQAYLAPDNV; DQAYLAPDNVI;
QAYLAPDNVIT; AYLAPDNVITS; YLAPDNVITST; LAPDNVITSTT;
APDNVITSTTK; PDNVITSTTKD; DNVITSTTKDV; NVITSTTKDVG;
VITSTTKDVGR; ITSTTKDVGRA; TSTTKDVGRAL; STTKDVGRALN;
TTKDVGRALNI; TKDVGRALNIF; KDVGRALNIFT; DVGRALNIFTS;
VGRALNIFTSN; GRALNIFTSNH; RALNIFTSNHL; ALNIFTSNHLK;
LNIFTSNHLKT; NIFTSNHLKTN; IFTSNHLKTNT; FTSNHLKTNTF;
TSNHLKTNTFE; SNHLKTNTFEG; NHLKTNTFEGG; HLKTNTFEGGK;
LKTNTFEGGKL; KTNTFEGGKLI; TNTFEGGKLIN; NTFEGGKLINY;
TFEGGKLINYG; FEGGKLINYGL; EGGKLINYGLK; GGKLINYGLKE;
GKLINYGLKEG; KLINYGLKEGV; LINYGLKEGVV; INYGLKEGVVG;
NYGLKEGVVGF; YGLKEGVVGFV; GLKEGVVGFVR; LKEGVVGFVRN;
KEGVVGFVRNP; EGVVGFVRNPK; GVVGFVRNPKM; VVGFVRNPKMI;
VGFVRNPKMIS; GFVRNPKMISF; FVRNPKMISFE; VRNPKMISFEL;
RNPKMISFELE; NPKMISFELEK; PKMISFELEKE; KMISFELEKEI;
MISFELEKEID; ISFELEKEIDN; SFELEKEIDNL; FELEKEIDNLS;
ELEKEIDNLSS; LEKEIDNLSSK; EKEIDNLSSKI; KEIDNLSSKII;
EIDNLSSKIIN; IDNLSSKIINK; DNLSSKIINKE; NLSSKIINKEI;
LSSKIINKEII; SSKIINKEIIV; SKIINKEIIVP; KIINKEIIVPS;
IINKEIIVPSN; INKEIIVPSNK; NKEIIVPSNKE; KEIIVPSNKES;
EIIVPSNKESY; IIVPSNKESYE; IVPSNKESYEK; VPSNKESYEKF;
PSNKESYEKFL; SNKESYEKFLK; NKESYEKFLKE; KESYEKFLKEF;
ESYEKFLKEFI;

| NP_212516.1 Basic membrane protein B (bmpB) [Borrelia burgdorferi B31 | SEQ ID NO 1367-2696/ 8 mers: |
|---|---|

SEQ ID NO 1367-2696/
8 mers:
MRIVIFIF; RIVIFIFG; IVIFIFGI; VIFIFGIL; IFIFGILL; FIFGILLT;
IFGILLTS; FGILLTSC; GILLTSCF; ILLTSCFS; LLTSCFSR; LTSCFSRN;
TSCFSRNG; SCFSRNGI; CFSRNGIE; FSRNGIES; SRNGIESS; RNGIESSS;
NGIESSSK; GIESSSKK; IESSSKKI; ESSSKKIK; SSSKKIKI; SSKKIKIS;
SKKIKISM; KKIKISML; KIKISMLV; IKISMLVD; KISMLVDG; ISMLVDGV;
SMLVDGVL; MLVDGVLD; LVDGVLDD; VDGVLDDK; DGVLDDKS; GVLDDKSF;
VLDDKSFN; LDDKSFNS; DDKSFNSS; DKSFNSSA; KSFNSSAN; SFNSSANE;
FNSSANEA; NSSANEAL; SSANEALL; SANEALLR; ANEALLRL; NEALLRLK;
EALLRLKK; ALLRLKKD; LLRLKKDF; LRLKKDFP; RLKKDFPE; LKKDFPEN;
KKDFPENI; KDFPENIE; DFPENIEE; FPENIEEV; PENIEEVF; ENIEEVFS;
NIEEVFSC; IEEVFSCA; EEVFSCAI; EVFSCAIS; VFSCAISG; FSCAISGV;
SCAISGVY; CAISGVYS; AISGVYSS; ISGVYSSY; SGVYSSYV; GVYSSYVS;
VYSSYVSD; YSSYVSDL; SSYVSDLD; SYVSDLDN; YVSDLDNL; VSDLDNLK;
SDLDNLKR; DLDNLKRN; LDNLKRNG; DNLKRNGS; NLKRNGSD; LKRNGSDL;
KRNGSDLI; RNGSDLIW; NGSDLIWL; GSDLIWLV; SDLIWLVG; DLIWLVGY;
LIWLVGYM; IWLVGYML; WLVGYMLT; LVGYMLTD; VGYMLTDA; GYMLTDAS;
YMLTDASL; MLTDASLL; LTDASLLV; TDASLLVS; DASLLVSS; ASLLVSSE;
SLLVSSEN; LLVSSENP; LVSSENPK; VSSENPKI; SSENPKIS; SENPKISY;
ENPKISYG; NPKISYGI; PKISYGII; KISYGIID; ISYGIIDP; SYGIIDPI;
YGIIDPIY; GIIDPIYG; IIDPIYGD; IDPIYGDD; DPIYGDDV; PIYGDDVQ;
IYGDDVQI; YGDDVQIP; GDDVQIPE; DDVQIPEN; DVQIPENL; VQIPENLI;
QIPENLIA; IPENLIAV; PENLIAVV; ENLIAVVF; NLIAVVFR; LIAVVFRV;
IAVVFRVE; AVVFRVEQ; VVFRVEQG; VFRVEQGA; FRVEQGAF; RVEQGAFL;
VEQGAFLA; EQGAFLAG; QGAFLAGY; GAFLAGYI; AFLAGYIA; FLAGYIAA;
LAGYIAAK; AGYIAAKK; GYIAAKKS; YIAAKKSF; IAAKKSFS; AAKKSFSG;
AKKSFSGK; KKSFSGKI; KSFSGKIG; SFSGKIGF; FSGKIGFI; SGKIGFIG;
GKIGFIGG; KIGFIGGM; IGFIGGMK; GFIGGMKG; FIGGMKGN; IGGMKGNI;
GGMKGNIV; GMKGNIVD; MKGNIVDA; KGNIVDAF; GNIVDAFR; NIVDAFRY;
IVDAFRYG; VDAFRYGY; DAFRYGYE; AFRYGYES; FRYGYESG; RYGYESGA;
YGYESGAK; GYESGAKY; YESGAKYA; ESGAKYAN; SGAKYANK; GAKYANKD;
AKYANKDI; KYANKDIE; YANKDIEI; ANKDIEII; NKDIEIIS; KDIEIISE;
DIEIISEY; IEIISEYS; EIISEYSN; IISEYSNS; ISEYSNSF; SEYSNSFS;
EYSNSFSD; YSNSFSDV; SNSFSDVD; NSFSDVDI; SFSDVDIG; FSDVDIGR;
SDVDIGRT; DVDIGRTI; VDIGRTIA; DIGRTIAS; IGRTIASK; GRTIASKM;
RTIASKMY; TIASKMYS; IASKMYSK; ASKMYSKG; SKMYSKGI; KMYSKGID;
MYSKGIDV; YSKGIDVI; SKGIDVIH; KGIDVIHF; GIDVIHFA; IDVIHFAA;
DVIHFAAG; VIHFAAGL; IHFAAGLA; HFAAGLAG; FAAGLAGI; AAGLAGIG;
AGLAGIGV; GLAGIGVI; LAGIGVIE; AGIGVIET; GIGVIETA; IGVIETAK;
GVIETAKN; VIETAKNL; IETAKNLG; ETAKNLGD; TAKNLGDG; AKNLGDGY;
KNLGDGYY; NLGDGYYV; LGDGYYVI; GDGYYVIG; DGYYVIGA; GYYVIGAD;
YYVIGADQ; YVIGADQD; VIGADQDQ; IGADQDQS; GADQDQSY; ADQDQSYL;
DQDQSYLA; QDQSYLAP; DQSYLAPK; QSYLAPKN; SYLAPKNF; YLAPKNFI;
LAPKNFIT; APKNFITS; PKNFITSV; KNFITSVI; NFITSVIK; FITSVIKN;
ITSVIKNI; TSVIKNIG; SVIKNIGD; VIKNIGDA; IKNIGDAL; KNIGDALY;
NIGDALYL; IGDALYLI; GDALYLIT; DALYLITG; ALYLITGE; LYLITGEY;
YLITGEYI; LITGEYIK; ITGEYIKN; TGEYIKNN; GEYIKNNN; EYIKNNNV;
YIKNNNVW; IKNNNVWE; KNNNVWEG; NNNVWEGG; NNVWEGGK; NVWEGGKV;
VWEGGKVV; WEGGKVVQ; EGGKVVQM; GGKVVQMG; GKVVQMGL; KVVQMGLR;
VVQMGLRD; VQMGLRDG; QMGLRDGV; MGLRDGVI; GLRDGVIG; LRDGVIGL;
RDGVIGLP; DGVIGLPN; GVIGLPNA; VIGLPNAN; IGLPNANE; GLPNANEF;
LPNANEFE; PNANEFEY; NANEFEYI; ANEFEYIK; NEFEYIKV; EFEYIKVL;
FEYIKVLE; EYIKVLER; YIKVLERK; IKVLERKI; KVLERKII; VLERKIIN;
LERKIINK; ERKIINKE; RKIINKEI; KIINKEII; IINKEIIV; INKEIIVP;
NKEIIVPC; KEIIVPCN; EIIVPCNQ; IIVPCNQE; IVPCNQEE; VPCNQEEY;

PCNQEEYE; CNQEEYEI; NQEEYEIF; QEEYEIFI; EEYEIFIK; EYEIFIKQ;
YEIFIKQI; EIFIKQIL; IFIKQILK; FIKQILKL;

9 mers:
MRIVIFIFG; RIVIFIFGI; IVIFIFGIL; VIFIFGILL; IFIFGILLT;
FIFGILLTS; IFGILLTSC; FGILLTSCF; GILLTSCFS; ILLTSCFSR;
LLTSCFSRN; LTSCFSRNG; TSCFSRNGI; SCFSRNGIE; CFSRNGIES;
FSRNGIESS; SRNGIESSS; RNGIESSSK; NGIESSSKK; GIESSSKKI;
IESSSKKIK; ESSSKKIKI; SSSKKIKIS; SSKKIKISM; SKKIKISML;
KKIKISMLV; KIKISMLVD; IKISMLVDG; KISMLVDGV; ISMLVDGVL;
SMLVDGVLD; MLVDGVLDD; LVDGVLDDK; VDGVLDDKS; DGVLDDKSF;
GVLDDKSFN; VLDDKSFNS; LDDKSFNSS; DDKSFNSSA; DKSFNSSAN;
KSFNSSANE; SFNSSANEA; FNSSANEAL; NSSANEALL; SSANEALLR;
SANEALLRL; ANEALLRLK; NEALLRLKK; EALLRLKKD; ALLRLKKDF;
LLRLKKDFP; LRLKKDFPE; RLKKDFPEN; LKKDFPENI; KKDFPENIE;
KDFPENIEE; DFPENIEEV; FPENIEEVF; PENIEEVFS; ENIEEVFSC;
NIEEVFSCA; IEEVFSCAI; EEVFSCAIS; EVFSCAISG; VFSCAISGV;
FSCAISGVY; SCAISGVYS; CAISGVYSS; AISGVYSSY; ISGVYSSYV;
SGVYSSYVS; GVYSSYVSD; VYSSYVSDL; YSSYVSDLD; SSYVSDLDN;
SYVSDLDNL; YVSDLDNLK; VSDLDNLKR; SDLDNLKRN; DLDNLKRNG;
LDNLKRNGS; DNLKRNGSD; NLKRNGSDL; LKRNGSDLI; KRNGSDLIW;
RNGSDLIWL; NGSDLIWLV; GSDLIWLVG; SDLIWLVGY; DLIWLVGYM;
LIWLVGYML; IWLVGYMLT; WLVGYMLTD; LVGYMLTDA; VGYMLTDAS;
GYMLTDASL; YMLTDASLL; MLTDASLLV; LTDASLLVS; TDASLLVSS;
DASLLVSSE; ASLLVSSEN; SLLVSSENP; LLVSSENPK; LVSSENPKI;
VSSENPKIS; SSENPKISY; SENPKISYG; ENPKISYGI; NPKISYGII;
PKISYGIID; KISYGIIDP; ISYGIIDPI; SYGIIDPIY; YGIIDPIYG;
GIIDPIYGD; IIDPIYGDD; IDPIYGDDV; DPIYGDDVQ; PIYGDDVQI;
IYGDDVQIP; YGDDVQIPE; GDDVQIPEN; DDVQIPENL; DVQIPENLI;
VQIPENLIA; QIPENLIAV; IPENLIAVV; PENLIAVVF; ENLIAVVFR;
NLIAVVFRV; LIAVVFRVE; IAVVFRVEQ; AVVFRVEQG; VVFRVEQGA;
VFRVEQGAF; FRVEQGAFL; RVEQGAFLA; VEQGAFLAG; EQGAFLAGY;
QGAFLAGYI; GAFLAGYIA; AFLAGYIAA; FLAGYIAAK; LAGYIAAKK;
AGYIAAKKS; GYIAAKKSF; YIAAKKSFS; IAAKKSFSG; AAKKSFSGK;
AKKSFSGKI; KKSFSGKIG; KSFSGKIGF; SFSGKIGFI; FSGKIGFIG;
SGKIGFIGG; GKIGFIGGM; KIGFIGGMK; IGFIGGMKG; GFIGGMKGN;
FIGGMKGNI; IGGMKGNIV; GGMKGNIVD; GMKGNIVDA; MKGNIVDAF;
KGNIVDAFR; GNIVDAFRY; NIVDAFRYG; IVDAFRYGY; VDAFRYGYE;
DAFRYGYES; AFRYGYESG; FRYGYESGA; RYGYESGAK; YGYESGAKY;
GYESGAKYA; YESGAKYAN; ESGAKYANK; SGAKYANKD; GAKYANKDI;
AKYANKDIE; KYANKDIEI; YANKDIEII; ANKDIEIIS; NKDIEIISE;
KDIEIISEY; DIEIISEYS; IEIISEYSN; EIISEYSNS; IISEYSNSF;
ISEYSNSFS; SEYSNSFSD; EYSNSFSDV; YSNSFSDVD; SNSFSDVDI;
NSFSDVDIG; SFSDVDIGR; FSDVDIGRT; SDVDIGRTI; DVDIGRTIA;
VDIGRTIAS; DIGRTIASK; IGRTIASKM; GRTIASKMY; RTIASKMYS;
TIASKMYSK; IASKMYSKG; ASKMYSKGI; SKMYSKGID; KMYSKGIDV;
MYSKGIDVI; YSKGIDVIH; SKGIDVIHF; KGIDVIHFA; GIDVIHFAA;
IDVIHFAAG; DVIHFAAGL; VIHFAAGLA; IHFAAGLAG; HFAAGLAGI;
FAAGLAGIG; AAGLAGIGV; AGLAGIGVI; GLAGIGVIE; LAGIGVIET;
AGIGVIETA; GIGVIETAK; IGVIETAKN; GVIETAKNL; VIETAKNLG;
IETAKNLGD; ETAKNLGDG; TAKNLGDGY; AKNLGDGYY; KNLGDGYYV;
NLGDGYYVI; LGDGYYVIG; GDGYYVIGA; DGYYVIGAD; GYYVIGADQ;
YYVIGADQD; YVIGADQDQ; VIGADQDQS; IGADQDQSY; GADQDQSYL;
ADQDQSYLA; DQDQSYLAP; QDQSYLAPK; DQSYLAPKN; QSYLAPKNF;
SYLAPKNFI; YLAPKNFIT; LAPKNFITS; APKNFITSV; PKNFITSVI;
KNFITSVIK; NFITSVIKN; FITSVIKNI; ITSVIKNIG; TSVIKNIGD;
SVIKNIGDA; VIKNIGDAL; IKNIGDALY; KNIGDALYL; NIGDALYLI;

EP 2 254 592 B1

IGDALYLIT;  GDALYLITG;  DALYLITGE;  ALYLITGEY;  LYLITGEYI;
YLITGEYIK;  LITGEYIKN;  ITGEYIKNN;  TGEYIKNNN;  GEYIKNNNV;
EYIKNNNVW;  YIKNNNVWE;  IKNNNVWEG;  KNNNVWEGG;  NNNVWEGGK;
NNVWEGGKV;  NVWEGGKVV;  VWEGGKVVQ;  WEGGKVVQM;  EGGKVVQMG;
GGKVVQMGL;  GKVVQMGLR;  KVVQMGLRD;  VVQMGLRDG;  VQMGLRDGV;
QMGLRDGVI;  MGLRDGVIG;  GLRDGVIGL;  LRDGVIGLP;  RDGVIGLPN;
DGVIGLPNA;  GVIGLPNAN;  VIGLPNANE;  IGLPNANEF;  GLPNANEFE;
LPNANEFEY;  PNANEFEYI;  NANEFEYIK;  ANEFEYIKV;  NEFEYIKVL;
EFEYIKVLE;  FEYIKVLER;  EYIKVLERK;  YIKVLERKI;  IKVLERKII;
KVLERKIIN;  VLERKIINK;  LERKIINKE;  ERKIINKEI;  RKIINKEII;
KIINKEIIV;  IINKEIIVP;  INKEIIVPC;  NKEIIVPCN;  KEIIVPCNQ;
EIIVPCNQE;  IIVPCNQEE;  IVPCNQEEY;  VPCNQEEYE;  PCNQEEYEI;
CNQEEYEIF;  NQEEYEIFI;  QEEYEIFIK;  EEYEIFIKQ;  EYEIFIKQI;
YEIFIKQIL;  EIFIKQILK;  IFIKQILKL;

10 mers:
MRIVIFIFGI;  RIVIFIFGIL;  IVIFIFGILL;  VIFIFGILLT;  IFIFGILLTS;
FIFGILLTSC;  IFGILLTSCF;  FGILLTSCFS;  GILLTSCFSR;  ILLTSCFSRN;
LLTSCFSRNG;  LTSCFSRNGI;  TSCFSRNGIE;  SCFSRNGIES;  CFSRNGIESS;
FSRNGIESSS;  SRNGIESSSK;  RNGIESSSKK;  NGIESSSKKI;  GIESSSKKIK;
IESSSKKIKI;  ESSSKKIKIS;  SSSKKIKISM;  SSKKIKISML;  SKKIKISMLV;
KKIKISMLVD;  KIKISMLVDG;  IKISMLVDGV;  KISMLVDGVL;  ISMLVDGVLD;
SMLVDGVLDD;  MLVDGVLDDK;  LVDGVLDDKS;  VDGVLDDKSF;  DGVLDDKSFN;
GVLDDKSFNS;  VLDDKSFNSS;  LDDKSFNSSA;  DDKSFNSSAN;  DKSFNSSANE;
KSFNSSANEA;  SFNSSANEAL;  FNSSANEALL;  NSSANEALLR;  SSANEALLRL;
SANEALLRLK;  ANEALLRLKK;  NEALLRLKKD;  EALLRLKKDF;  ALLRLKKDFP;
LLRLKKDFPE;  LRLKKDFPEN;  RLKKDFPENI;  LKKDFPENIE;  KKDFPENIEE;
KDFPENIEEV;  DFPENIEEVF;  FPENIEEVFS;  PENIEEVFSC;  ENIEEVFSCA;
NIEEVFSCAI;  IEEVFSCAIS;  EEVFSCAISG;  EVFSCAISGV;  VFSCAISGVY;
FSCAISGVYS;  SCAISGVYSS;  CAISGVYSSY;  AISGVYSSYV;  ISGVYSSYVS;
SGVYSSYVSD;  GVYSSYVSDL;  VYSSYVSDLD;  YSSYVSDLDN;  SSYVSDLDNL;
SYVSDLDNLK;  YVSDLDNLKR;  VSDLDNLKRN;  SDLDNLKRNG;  DLDNLKRNGS;
LDNLKRNGSD;  DNLKRNGSDL;  NLKRNGSDLI;  LKRNGSDLIW;  KRNGSDLIWL;
RNGSDLIWLV;  NGSDLIWLVG;  GSDLIWLVGY;  SDLIWLVGYM;  DLIWLVGYML;
LIWLVGYMLT;  IWLVGYMLTD;  WLVGYMLTDA;  LVGYMLTDAS;  VGYMLTDASL;
GYMLTDASLL;  YMLTDASLLV;  MLTDASLLVS;  LTDASLLVSS;  TDASLLVSSE;
DASLLVSSEN;  ASLLVSSENP;  SLLVSSENPK;  LLVSSENPKI;  LVSSENPKIS;
VSSENPKISY;  SSENPKISYG;  SENPKISYGI;  ENPKISYGII;  NPKISYGIID;
PKISYGIIDP;  KISYGIIDPI;  ISYGIIDPIY;  SYGIIDPIYG;  YGIIDPIYGD;
GIIDPIYGDD;  IIDPIYGDDV;  IDPIYGDDVQ;  DPIYGDDVQI;  PIYGDDVQIP;
IYGDDVQIPE;  YGDDVQIPEN;  GDDVQIPENL;  DDVQIPENLI;  DVQIPENLIA;
VQIPENLIAV;  QIPENLIAVV;  IPENLIAVVF;  PENLIAVVFR;  ENLIAVVFRV;
NLIAVVFRVE;  LIAVVFRVEQ;  IAVVFRVEQG;  AVVFRVEQGA;  VVFRVEQGAF;
VFRVEQGAFL;  FRVEQGAFLA;  RVEQGAFLAG;  VEQGAFLAGY;  EQGAFLAGYI;
QGAFLAGYIA;  GAFLAGYIAA;  AFLAGYIAAK;  FLAGYIAAKK;  LAGYIAAKKS;
AGYIAAKKSF;  GYIAAKKSFS;  YIAAKKSFSG;  IAAKKSFSGK;  AAKKSFSGKI;
AKKSFSGKIG;  KKSFSGKIGF;  KSFSGKIGFI;  SFSGKIGFIG;  FSGKIGFIGG;
SGKIGFIGGM;  GKIGFIGGMK;  KIGFIGGMKG;  IGFIGGMKGN;  GFIGGMKGNI;
FIGGMKGNIV;  IGGMKGNIVD;  GGMKGNIVDA;  GMKGNIVDAF;  MKGNIVDAFR;
KGNIVDAFRY;  GNIVDAFRYG;  NIVDAFRYGY;  IVDAFRYGYE;  VDAFRYGYES;
DAFRYGYESG;  AFRYGYESGA;  FRYGYESGAK;  RYGYESGAKY;  YGYESGAKYA;
GYESGAKYAN;  YESGAKYANK;  ESGAKYANKD;  SGAKYANKDI;  GAKYANKDIE;
AKYANKDIEI;  KYANKDIEII;  YANKDIEIIS;  ANKDIEIISE;  NKDIEIISEY;
KDIEIISEYS;  DIEIISEYSN;  IEIISEYSNS;  EIISEYSNSF;  IISEYSNSFS;
ISEYSNSFSD;  SEYSNSFSDV;  EYSNSFSDVD;  YSNSFSDVDI;  SNSFSDVDIG;
NSFSDVDIGR;  SFSDVDIGRT;  FSDVDIGRTI;  SDVDIGRTIA;  DVDIGRTIAS;
VDIGRTIASK;  DIGRTIASKM;  IGRTIASKMY;  GRTIASKMYS;  RTIASKMYSK;

601

TIASKMYSKG; IASKMYSKGI; ASKMYSKGID; SKMYSKGIDV; KMYSKGIDVI;
MYSKGIDVIH; YSKGIDVIHF; SKGIDVIHFA; KGIDVIHFAA; GIDVIHFAAG;
IDVIHFAAGL; DVIHFAAGLA; VIHFAAGLAG; IHFAAGLAGI; HFAAGLAGIG;
FAAGLAGIGV; AAGLAGIGVI; AGLAGIGVIE; GLAGIGVIET; LAGIGVIETA;
AGIGVIETAK; GIGVIETAKN; IGVIETAKNL; GVIETAKNLG; VIETAKNLGD;
IETAKNLGDG; ETAKNLGDGY; TAKNLGDGYY; AKNLGDGYYV; KNLGDGYYVI;
NLGDGYYVIG; LGDGYYVIGA; GDGYYVIGAD; DGYYVIGADQ; GYYVIGADQD;
YYVIGADQDQ; YVIGADQDQS; VIGADQDQSY; IGADQDQSYL; GADQDQSYLA;
ADQDQSYLAP; DQDQSYLAPK; QDQSYLAPKN; DQSYLAPKNF; QSYLAPKNFI;
SYLAPKNFIT; YLAPKNFITS; LAPKNFITSV; APKNFITSVI; PKNFITSVIK;
KNFITSVIKN; NFITSVIKNI; FITSVIKNIG; ITSVIKNIGD; TSVIKNIGDA;
SVIKNIGDAL; VIKNIGDALY; IKNIGDALYL; KNIGDALYLI; NIGDALYLIT;
IGDALYLITG; GDALYLITGE; DALYLITGEY; ALYLITGEYI; LYLITGEYIK;
YLITGEYIKN; LITGEYIKNN; ITGEYIKNNN; TGEYIKNNNV; GEYIKNNNVW;
EYIKNNNVWE; YIKNNNVWEG; IKNNNVWEGG; KNNNVWEGGK; NNNVWEGGKV;
NNVWEGGKVV; NVWEGGKVVQ; VWEGGKVVQM; WEGGKVVQMG; EGGKVVQMGL;
GGKVVQMGLR; GKVVQMGLRD; KVVQMGLRDG; VVQMGLRDGV; VQMGLRDGVI;
QMGLRDGVIG; MGLRDGVIGL; GLRDGVIGLP; LRDGVIGLPN; RDGVIGLPNA;
DGVIGLPNAN; GVIGLPNANE; VIGLPNANEF; IGLPNANEFE; GLPNANEFEY;
LPNANEFEYI; PNANEFEYIK; NANEFEYIKV; ANEFEYIKVL; NEFEYIKVLE;
EFEYIKVLER; FEYIKVLERK; EYIKVLERKI; YIKVLERKII; IKVLERKIIN;
KVLERKIINK; VLERKIINKE; LERKIINKEI; ERKIINKEII; RKIINKEIIV;
KIINKEIIVP; IINKEIIVPC; INKEIIVPCN; NKEIIVPCNQ; KEIIVPCNQE;
EIIVPCNQEE; IIVPCNQEEY; IVPCNQEEYE; VPCNQEEYEI; PCNQEEYEIF;
CNQEEYEIFI; NQEEYEIFIK; QEEYEIFIKQ; EEYEIFIKQI; EYEIFIKQIL;
YEIFIKQILK; EIFIKQILKL;

11 mers:
MRIVIFIFGIL; RIVIFIFGILL; IVIFIFGILLT; VIFIFGILLTS;
IFIFGILLTSC; FIFGILLTSCF; IFGILLTSCFS; FGILLTSCFSR;
GILLTSCFSRN; ILLTSCFSRNG; LLTSCFSRNGI; LTSCFSRNGIE;
TSCFSRNGIES; SCFSRNGIESS; CFSRNGIESSS; FSRNGIESSSK;
SRNGIESSSKK; RNGIESSSKKI; NGIESSSKKIK; GIESSSKKIKI;
IESSSKKIKIS; ESSSKKIKISM; SSSKKIKISML; SSKKIKISMLV;
SKKIKISMLVD; KKIKISMLVDG; KIKISMLVDGV; IKISMLVDGVL;
KISMLVDGVLD; ISMLVDGVLDD; SMLVDGVLDDK; MLVDGVLDDKS;
LVDGVLDDKSF; VDGVLDDKSFN; DGVLDDKSFNS; GVLDDKSFNSS;
VLDDKSFNSSA; LDDKSFNSSAN; DDKSFNSSANE; DKSFNSSANEA;
KSFNSSANEAL; SFNSSANEALL; FNSSANEALLR; NSSANEALLRL;
SSANEALLRLK; SANEALLRLKK; ANEALLRLKKD; NEALLRLKKDF;
EALLRLKKDFP; ALLRLKKDFPE; LLRLKKDFPEN; LRLKKDFPENI;
RLKKDFPENIE; LKKDFPENIEE; KKDFPENIEEV; KDFPENIEEVF;
DFPENIEEVFS; FPENIEEVFSC; PENIEEVFSCA; ENIEEVFSCAI;
NIEEVFSCAIS; IEEVFSCAISG; EEVFSCAISGV; EVFSCAISGVY;
VFSCAISGVYS; FSCAISGVYSS; SCAISGVYSSY; CAISGVYSSYV;
AISGVYSSYVS; ISGVYSSYVSD; SGVYSSYVSDL; GVYSSYVSDLD;
VYSSYVSDLDN; YSSYVSDLDNL; SSYVSDLDNLK; SYVSDLDNLKR;
YVSDLDNLKRN; VSDLDNLKRNG; SDLDNLKRNGS; DLDNLKRNGSD;
LDNLKRNGSDL; DNLKRNGSDLI; NLKRNGSDLIW; LKRNGSDLIWL;
KRNGSDLIWLV; RNGSDLIWLVG; NGSDLIWLVGY; GSDLIWLVGYM;
SDLIWLVGYML; DLIWLVGYMLT; LIWLVGYMLTD; IWLVGYMLTDA;
WLVGYMLTDAS; LVGYMLTDASL; VGYMLTDASLL; GYMLTDASLLV;
YMLTDASLLVS; MLTDASLLVSS; LTDASLLVSSE; TDASLLVSSEN;
DASLLVSSENP; ASLLVSSENPK; SLLVSSENPKI; LLVSSENPKIS;
LVSSENPKISY; VSSENPKISYG; SSENPKISYGI; SENPKISYGII;
ENPKISYGIID; NPKISYGIIDP; PKISYGIIDPI; KISYGIIDPIY;
ISYGIIDPIYG; SYGIIDPIYGD; YGIIDPIYGDD; GIIDPIYGDDV;

| | |
|---|---|
| | IIDPIYGDDVQ; IDPIYGDDVQI; DPIYGDDVQIP; PIYGDDVQIPE;<br>IYGDDVQIPEN; YGDDVQIPENL; GDDVQIPENLI; DDVQIPENLIA;<br>DVQIPENLIAV; VQIPENLIAVV; QIPENLIAVVF; IPENLIAVVFR;<br>PENLIAVVFRV; ENLIAVVFRVE; NLIAVVFRVEQ; LIAVVFRVEQG;<br>IAVVFRVEQGA; AVVFRVEQGAF; VVFRVEQGAFL; VFRVEQGAFLA;<br>FRVEQGAFLAG; RVEQGAFLAGY; VEQGAFLAGYI; EQGAFLAGYIA;<br>QGAFLAGYIAA; GAFLAGYIAAK; AFLAGYIAAKK; FLAGYIAAKKS;<br>LAGYIAAKKSF; AGYIAAKKSFS; GYIAAKKSFSG; YIAAKKSFSGK;<br>IAAKKSFSGKI; AAKKSFSGKIG; AKKSFSGKIGF; KKSFSGKIGFI;<br>KSFSGKIGFIG; SFSGKIGFIGG; FSGKIGFIGGM; SGKIGFIGGMK;<br>GKIGFIGGMKG; KIGFIGGMKGN; IGFIGGMKGNI; GFIGGMKGNIV;<br>FIGGMKGNIVD; IGGMKGNIVDA; GGMKGNIVDAF; GMKGNIVDAFR;<br>MKGNIVDAFRY; KGNIVDAFRYG; GNIVDAFRYGY; NIVDAFRYGYE;<br>IVDAFRYGYES; VDAFRYGYESG; DAFRYGYESGA; AFRYGYESGAK;<br>FRYGYESGAKY; RYGYESGAKYA; YGYESGAKYAN; GYESGAKYANK;<br>YESGAKYANKD; ESGAKYANKDI; SGAKYANKDIE; GAKYANKDIEI;<br>AKYANKDIEII; KYANKDIEIIS; YANKDIEIISE; ANKDIEIISEY;<br>NKDIEIISEYS; KDIEIISEYSN; DIEIISEYSNS; IEIISEYSNSF;<br>EIISEYSNSFS; IISEYSNSFSD; ISEYSNSFSDV; SEYSNSFSDVD;<br>EYSNSFSDVDI; YSNSFSDVDIG; SNSFSDVDIGR; NSFSDVDIGRT;<br>SFSDVDIGRTI; FSDVDIGRTIA; SDVDIGRTIAS; DVDIGRTIASK;<br>VDIGRTIASKM; DIGRTIASKMY; IGRTIASKMYS; GRTIASKMYSK;<br>RTIASKMYSKG; TIASKMYSKGI; IASKMYSKGID; ASKMYSKGIDV;<br>SKMYSKGIDVI; KMYSKGIDVIH; MYSKGIDVIHF; YSKGIDVIHFA;<br>SKGIDVIHFAA; KGIDVIHFAAG; GIDVIHFAAGL; IDVIHFAAGLA;<br>DVIHFAAGLAG; VIHFAAGLAGI; IHFAAGLAGIG; HFAAGLAGIGV;<br>FAAGLAGIGVI; AAGLAGIGVIE; AGLAGIGVIET; GLAGIGVIETA;<br>LAGIGVIETAK; AGIGVIETAKN; GIGVIETAKNL; IGVIETAKNLG;<br>GVIETAKNLGD; VIETAKNLGDG; IETAKNLGDGY; ETAKNLGDGYY;<br>TAKNLGDGYYV; AKNLGDGYYVI; KNLGDGYYVIG; NLGDGYYVIGA;<br>LGDGYYVIGAD; GDGYYVIGADQ; DGYYVIGADQD; GYYVIGADQDQ;<br>YYVIGADQDQS; YVIGADQDQSY; VIGADQDQSYL; IGADQDQSYLA;<br>GADQDQSYLAP; ADQDQSYLAPK; DQDQSYLAPKN; QDQSYLAPKNF;<br>DQSYLAPKNFI; QSYLAPKNFIT; SYLAPKNFITS; YLAPKNFITSV;<br>LAPKNFITSVI; APKNFITSVIK; PKNFITSVIKN; KNFITSVIKNI;<br>NFITSVIKNIG; FITSVIKNIGD; ITSVIKNIGDA; TSVIKNIGDAL;<br>SVIKNIGDALY; VIKNIGDALYL; IKNIGDALYLI; KNIGDALYLIT;<br>NIGDALYLITG; IGDALYLITGE; GDALYLITGEY; DALYLITGEYI;<br>ALYLITGEYIK; LYLITGEYIKN; YLITGEYIKNN; LITGEYIKNNN;<br>ITGEYIKNNNV; TGEYIKNNNVW; GEYIKNNNVWE; EYIKNNNVWEG;<br>YIKNNNVWEGG; IKNNNVWEGGK; KNNNVWEGGKV; NNNVWEGGKVV;<br>NNVWEGGKVVQ; NVWEGGKVVQM; VWEGGKVVQMG; WEGGKVVQMGL;<br>EGGKVVQMGLR; GGKVVQMGLRD; GKVVQMGLRDG; KVVQMGLRDGV;<br>VVQMGLRDGVI; VQMGLRDGVIG; QMGLRDGVIGL; MGLRDGVIGLP;<br>GLRDGVIGLPN; LRDGVIGLPNA; RDGVIGLPNAN; DGVIGLPNANE;<br>GVIGLPNANEF; VIGLPNANEFE; IGLPNANEFEY; GLPNANEFEYI;<br>LPNANEFEYIK; PNANEFEYIKV; NANEFEYIKVL; ANEFEYIKVLE;<br>NEFEYIKVLER; EFEYIKVLERK; FEYIKVLERKI; EYIKVLERKII;<br>YIKVLERKIIN; IKVLERKIINK; KVLERKIINKE; VLERKIINKEI;<br>LERKIINKEII; ERKIINKEIIV; RKIINKEIIVP; KIINKEIIVPC;<br>IINKEIIVPCN; INKEIIVPCNQ; NKEIIVPCNQE; KEIIVPCNQEE;<br>EIIVPCNQEEY; IIVPCNQEEYE; IVPCNQEEYEI; VPCNQEEYEIF;<br>PCNQEEYEIFI; CNQEEYEIFIK; NQEEYEIFIKQ; QEEYEIFIKQI;<br>EEYEIFIKQIL; EYEIFIKQILK; YEIFIKQILKL; |
| NP_212518.1<br>Basic membrane | SEQ ID NO 2697-4074/<br>8 mers: |

| protein C (bmpC) [Borrelia burgdorferi B31 | MFKRFIFI; FKRFIFIT; KRFIFITL; RFIFITLS; FIFITLSL; IFITLSLL; FITLSLLV; ITLSLLVF; TLSLLVFA; LSLLVFAC; SLLVFACF; LLVFACFK; LVFACFKS; VFACFKSN; FACFKSNK; ACFKSNKK; CFKSNKKS; FKSNKKSI; KSNKKSIK; SNKKSIKS; NKKSIKSD; KKSIKSDK; KSIKSDKV; SIKSDKVV; IKSDKVVV; KSDKVVVG; SDKVVVGV; DKVVVGVL; KVVVGVLA; VVVGVLAH; VVGVLAHG; VGVLAHGS; GVLAHGSF; VLAHGSFY; LAHGSFYD; AHGSFYDK; HGSFYDKG; GSFYDKGY; SFYDKGYN; FYDKGYNQ; YDKGYNQS; DKGYNQSV; KGYNQSVH; GYNQSVHD; YNQSVHDG; NQSVHDGV; QSVHDGVV; SVHDGVVK; VHDGVVKL; HDGVVKLR; DGVVKLRD; GVVKLRDN; VVKLRDNF; VKLRDNFG; KLRDNFGI; LRDNFGIK; RDNFGIKL; DNFGIKLI; NFGIKLIT; FGIKLITK; GIKLITKS; IKLITKSL; KLITKSLR; LITKSLRP; ITKSLRPY; TKSLRPYP; KSLRPYPI; SLRPYPIE; LRPYPIEG; RPYPIEGK; PYPIEGKR; YPIEGKRL; PIEGKRLL; IEGKRLLT; EGKRLLTV; GKRLLTVD; KRLLTVDE; RLLTVDEA; LLTVDEAM; LTVDEAMT; TVDEAMTE; VDEAMTED; DEAMTEDA; EAMTEDAY; AMTEDAYE; MTEDAYEV; TEDAYEVQ; EDAYEVQK; DAYEVQKN; AYEVQKNP; YEVQKNPL; EVQKNPLN; VQKNPLNL; QKNPLNLF; KNPLNLFW; NPLNLFWL; PLNLFWLI; LNLFWLIG; NLFWLIGY; LFWLIGYR; FWLIGYRF; WLIGYRFS; LIGYRFSD; IGYRFSDL; GYRFSDLS; YRFSDLSV; RFSDLSVK; FSDLSVKL; SDLSVKLS; DLSVKLSY; LSVKLSYE; SVKLSYER; VKLSYERP; KLSYERPD; LSYERPDI; SYERPDIY; YERPDIYY; ERPDIYYG; RPDIYYGI; PDIYYGII; DIYYGIID; IYYGIIDA; YYGIIDAF; YGIIDAFD; GIIDAFDY; IIDAFDYG; IDAFDYGD; DAFDYGDI; AFDYGDIQ; FDYGDIQV; DYGDIQVP; YGDIQVPK; GDIQVPKN; DIQVPKNS; IQVPKNSL; QVPKNSLA; VPKNSLAI; PKNSLAIK; KNSLAIKF; NSLAIKFR; SLAIKFRN; LAIKFRNE; AIKFRNEE; IKFRNEEA; KFRNEEAA; FRNEEAAF; RNEEAAFL; NEEAAFLA; EEAAFLAG; EAAFLAGY; AAFLAGYI; AFLAGYIA; FLAGYIAA; LAGYIAAK; AGYIAAKM; GYIAAKMS; YIAAKMSR; IAAKMSRK; AAKMSRKE; AKMSRKEK; KMSRKEKI; MSRKEKIG; SRKEKIGF; RKEKIGFL; KEKIGFLT; EKIGFLTG; KIGFLTGP; IGFLTGPM; GFLTGPMS; FLTGPMSE; LTGPMSEH; TGPMSEHV; GPMSEHVK; PMSEHVKD; MSEHVKDF; SEHVKDFK; EHVKDFKF; HVKDFKFG; VKDFKFGF; KDFKFGFK; DFKFGFKA; FKFGFKAG; KFGFKAGI; FGFKAGIF; GFKAGIFY; FKAGIFYA; KAGIFYAN; AGIFYANP; GIFYANPK; IFYANPKL; FYANPKLR; YANPKLRL; ANPKLRLV; NPKLRLVS; PKLRLVSK; KLRLVSKK; LRLVSKKA; RLVSKKAP; LVSKKAPS; VSKKAPSL; SKKAPSLF; KKAPSLFD; KAPSLFDK; APSLFDKE; PSLFDKEK; SLFDKEKG; LFDKEKGK; FDKEKGKA; DKEKGKAM; KEKGKAMA; EKGKAMAL; KGKAMALF; GKAMALFM; KAMALFMY; AMALFMYK; MALFMYKE; ALFMYKED; LFMYKEDK; FMYKEDKV; MYKEDKVG; YKEDKVGV; KEDKVGVI; EDKVGVIF; DKVGVIFP; KVGVIFPI; VGVIFPIA; GVIFPIAG; VIFPIAGI; IFPIAGIT; FPIAGITG; PIAGITGL; IAGITGLG; AGITGLGV; GITGLGVY; ITGLGVYD; TGLGVYDA; GLGVYDAA; LGVYDAAK; GVYDAAKE; VYDAAKEL; YDAAKELG; DAAKELGP; AAKELGPK; AKELGPKY; KELGPKYY; ELGPKYYV; LGPKYYVI; GPKYYVIG; PKYYVIGL; KYYVIGLN; YYVIGLNQ; YVIGLNQD; VIGLNQDQ; IGLNQDQS; GLNQDQSY; LNQDQSYI; NQDQSYIA; QDQSYIAP; DQSYIAPQ; QSYIAPQN; SYIAPQNV; YIAPQNVI; IAPQNVIT; APQNVITS; PQNVITSI; QNVITSII; NVITSIIK; VITSIIKD; ITSIIKDI; TSIIKDIG; SIIKDIGK; IIKDIGKV; IKDIGKVI; KDIGKVIY; DIGKVIYS; IGKVIYSI; GKVIYSIS; KVIYSISS; VIYSISSE; IYSISSEY; YSISSEYI; SISSEYIN; ISSEYINN; SSEYINNR; SEYINNRV; EYINNRVF; YINNRVFK; INNRVFKG; NNRVFKGG; NRVFKGGI; RVFKGGII; VFKGGIII; FKGGIIID; KGGIIIDR; GGIIIDRG; GIIIDRGL; IIIDRGLK; IIDRGLKE; IDRGLKEG; DRGLKEGV; RGLKEGVI; GLKEGVIE; LKEGVIEI; KEGVIEIV; EGVIEIVK; GVIEIVKD; VIEIVKDP; IEIVKDPD; EIVKDPDV; IVKDPDVL; VKDPDVLN; KDPDVLNN; DPDVLNNR; PDVLNNRL; DVLNNRLV; VLNNRLVD; LNNRLVDE; NNRLVDEV; NRLVDEVI; RLVDEVID; LVDEVIDL; VDEVIDLE; DEVIDLEN; EVIDLENK; VIDLENKI; IDLENKII; DLENKIIS; LENKIISG; ENKIISGE; NKIISGEI; KIISGEII; IISGEIIV; ISGEIIVP; SGEIIVPD; GEIIVPDS; EIIVPDSE; IIVPDSEY; IVPDSEYA; VPDSEYAF; PDSEYAFD; DSEYAFDL; SEYAFDLF; |
|---|---|

EYAFDLFK; YAFDLFKS; AFDLFKSK; FDLFKSKL;

9 mers:
MFKRFIFIT; FKRFIFITL; KRFIFITLS; RFIFITLSL; FIFITLSLL;
IFITLSLLV; FITLSLLVF; ITLSLLVFA; TLSLLVFAC; LSLLVFACF;
SLLVFACFK; LLVFACFKS; LVFACFKSN; VFACFKSNK; FACFKSNKK;
ACFKSNKKS; CFKSNKKSI; FKSNKKSIK; KSNKKSIKS; SNKKSIKSD;
NKKSIKSDK; KKSIKSDKV; KSIKSDKVV; SIKSDKVVV; IKSDKVVVG;
KSDKVVVGV; SDKVVVGVL; DKVVVGVLA; KVVVGVLAH; VVVGVLAHG;
VVGVLAHGS; VGVLAHGSF; GVLAHGSFY; VLAHGSFYD; LAHGSFYDK;
AHGSFYDKG; HGSFYDKGY; GSFYDKGYN; SFYDKGYNQ; FYDKGYNQS;
YDKGYNQSV; DKGYNQSVH; KGYNQSVHD; GYNQSVHDG; YNQSVHDGV;
NQSVHDGVV; QSVHDGVVK; SVHDGVVKL; VHDGVVKLR; HDGVVKLRD;
DGVVKLRDN; GVVKLRDNF; VVKLRDNFG; VKLRDNFGI; KLRDNFGIK;
LRDNFGIKL; RDNFGIKLI; DNFGIKLIT; NFGIKLITK; FGIKLITKS;
GIKLITKSL; IKLITKSLR; KLITKSLRP; LITKSLRPY; ITKSLRPYP;
TKSLRPYPI; KSLRPYPIE; SLRPYPIEG; LRPYPIEGK; RPYPIEGKR;
PYPIEGKRL; YPIEGKRLL; PIEGKRLLT; IEGKRLLTV; EGKRLLTVD;
GKRLLTVDE; KRLLTVDEA; RLLTVDEAM; LLTVDEAMT; LTVDEAMTE;
TVDEAMTED; VDEAMTEDA; DEAMTEDAY; EAMTEDAYE; AMTEDAYEV;
MTEDAYEVQ; TEDAYEVQK; EDAYEVQKN; DAYEVQKNP; AYEVQKNPL;
YEVQKNPLN; EVQKNPLNL; VQKNPLNLF; QKNPLNLFW; KNPLNLFWL;
NPLNLFWLI; PLNLFWLIG; LNLFWLIGY; NLFWLIGYR; LFWLIGYRF;
FWLIGYRFS; WLIGYRFSD; LIGYRFSDL; IGYRFSDLS; GYRFSDLSV;
YRFSDLSVK; RFSDLSVKL; FSDLSVKLS; SDLSVKLSY; DLSVKLSYE;
LSVKLSYER; SVKLSYERP; VKLSYERPD; KLSYERPDI; LSYERPDIY;
SYERPDIYY; YERPDIYYG; ERPDIYYGI; RPDIYYGII; PDIYYGIID;
DIYYGIIDA; IYYGIIDAF; YYGIIDAFD; YGIIDAFDY; GIIDAFDYG;
IIDAFDYGD; IDAFDYGDI; DAFDYGDIQ; AFDYGDIQV; FDYGDIQVP;
DYGDIQVPK; YGDIQVPKN; GDIQVPKNS; DIQVPKNSL; IQVPKNSLA;
QVPKNSLAI; VPKNSLAIK; PKNSLAIKF; KNSLAIKFR; NSLAIKFRN;
SLAIKFRNE; LAIKFRNEE; AIKFRNEEA; IKFRNEEAA; KFRNEEAAF;
FRNEEAAFL; RNEEAAFLA; NEEAAFLAG; EEAAFLAGY; EAAFLAGYI;
AAFLAGYIA; AFLAGYIAA; FLAGYIAAK; LAGYIAAKM; AGYIAAKMS;
GYIAAKMSR; YIAAKMSRK; IAAKMSRKE; AAKMSRKEK; AKMSRKEKI;
KMSRKEKIG; MSRKEKIGF; SRKEKIGFL; RKEKIGFLT; KEKIGFLTG;
EKIGFLTGP; KIGFLTGPM; IGFLTGPMS; GFLTGPMSE; FLTGPMSEH;
LTGPMSEHV; TGPMSEHVK; GPMSEHVKD; PMSEHVKDF; MSEHVKDFK;
SEHVKDFKF; EHVKDFKFG; HVKDFKFGF; VKDFKFGFK; KDFKFGFKA;
DFKFGFKAG; FKFGFKAGI; KFGFKAGIF; FGFKAGIFY; GFKAGIFYA;
FKAGIFYAN; KAGIFYANP; AGIFYANPK; GIFYANPKL; IFYANPKLR;
FYANPKLRL; YANPKLRLV; ANPKLRLVS; NPKLRLVSK; PKLRLVSKK;
KLRLVSKKA; LRLVSKKAP; RLVSKKAPS; LVSKKAPSL; VSKKAPSLF;
SKKAPSLFD; KKAPSLFDK; KAPSLFDKE; APSLFDKEK; PSLFDKEKG;
SLFDKEKGK; LFDKEKGKA; FDKEKGKAM; DKEKGKAMA; KEKGKAMAL;
EKGKAMALF; KGKAMALFM; GKAMALFMY; KAMALFMYK; AMALFMYKE;
MALFMYKED; ALFMYKEDK; LFMYKEDKV; FMYKEDKVG; MYKEDKVGV;
YKEDKVGVI; KEDKVGVIF; EDKVGVIFP; DKVGVIFPI; KVGVIFPIA;
VGVIFPIAG; GVIFPIAGI; VIFPIAGIT; IFPIAGITG; FPIAGITGL;
PIAGITGLG; IAGITGLGV; AGITGLGVY; GITGLGVYD; ITGLGVYDA;
TGLGVYDAA; GLGVYDAAK; LGVYDAAKE; GVYDAAKEL; VYDAAKELG;
YDAAKELGP; DAAKELGPK; AAKELGPKY; AKELGPKYY; KELGPKYYV;
ELGPKYYVI; LGPKYYVIG; GPKYYVIGL; PKYYVIGLN; KYYVIGLNQ;
YYVIGLNQD; YVIGLNQDQ; VIGLNQDQS; IGLNQDQSY; GLNQDQSYI;
LNQDQSYIA; NQDQSYIAP; QDQSYIAPQ; DQSYIAPQN; QSYIAPQNV;
SYIAPQNVI; YIAPQNVIT; IAPQNVITS; APQNVITSI; PQNVITSII;
QNVITSIIK; NVITSIIKD; VITSIIKDI; ITSIIKDIG; TSIIKDIGK;

SIIKDIGKV; IIKDIGKVI; IKDIGKVIY; KDIGKVIYS; DIGKVIYSI;
IGKVIYSIS; GKVIYSISS; KVIYSISSE; VIYSISSEY; IYSISSEYI;
YSISSEYIN; SISSEYINN; ISSEYINNR; SSEYINNRV; SEYINNRVF;
EYINNRVFK; YINNRVFKG; INNRVFKGG; NNRVFKGGI; NRVFKGGII;
RVFKGGIII; VFKGGIIID; FKGGIIIDR; KGGIIIDRG; GGIIIDRGL;
GIIIDRGLK; IIIDRGLKE; IIDRGLKEG; IDRGLKEGV; DRGLKEGVI;
RGLKEGVIE; GLKEGVIEI; LKEGVIEIV; KEGVIEIVK; EGVIEIVKD;
GVIEIVKDP; VIEIVKDPD; IEIVKDPDV; EIVKDPDVL; IVKDPDVLN;
VKDPDVLNN; KDPDVLNNR; DPDVLNNRL; PDVLNNRLV; DVLNNRLVD;
VLNNRLVDE; LNNRLVDEV; NNRLVDEVI; NRLVDEVID; RLVDEVIDL;
LVDEVIDLE; VDEVIDLEN; DEVIDLENK; EVIDLENKI; VIDLENKII;
IDLENKIIS; DLENKIISG; LENKIISGE; ENKIISGEI; NKIISGEII;
KIISGEIIV; IISGEIIVP; ISGEIIVPD; SGEIIVPDS; GEIIVPDSE;
EIIVPDSEY; IIVPDSEYA; IVPDSEYAF; VPDSEYAFD; PDSEYAFDL;
DSEYAFDLF; SEYAFDLFK; EYAFDLFKS; YAFDLFKSK; AFDLFKSKL;

10 mers:
MFKRFIFITL; FKRFIFITLS; KRFIFITLSL; RFIFITLSLL; FIFITLSLLV;
IFITLSLLVF; FITLSLLVFA; ITLSLLVFAC; TLSLLVFACF; LSLLVFACFK;
SLLVFACFKS; LLVFACFKSN; LVFACFKSNK; VFACFKSNKK; FACFKSNKKS;
ACFKSNKKSI; CFKSNKKSIK; FKSNKKSIKS; KSNKKSIKSD; SNKKSIKSDK;
NKKSIKSDKV; KKSIKSDKVV; KSIKSDKVVV; SIKSDKVVVG; IKSDKVVVGV;
KSDKVVVGVL; SDKVVVGVLA; DKVVVGVLAH; KVVVGVLAHG; VVVGVLAHGS;
VVGVLAHGSF; VGVLAHGSFY; GVLAHGSFYD; VLAHGSFYDK; LAHGSFYDKG;
AHGSFYDKGY; HGSFYDKGYN; GSFYDKGYNQ; SFYDKGYNQS; FYDKGYNQSV;
YDKGYNQSVH; DKGYNQSVHD; KGYNQSVHDG; GYNQSVHDGV; YNQSVHDGVV;
NQSVHDGVVK; QSVHDGVVKL; SVHDGVVKLR; VHDGVVKLRD; HDGVVKLRDN;
DGVVKLRDNF; GVVKLRDNFG; VVKLRDNFGI; VKLRDNFGIK; KLRDNFGIKL;
LRDNFGIKLI; RDNFGIKLIT; DNFGIKLITK; NFGIKLITKS; FGIKLITKSL;
GIKLITKSLR; IKLITKSLRP; KLITKSLRPY; LITKSLRPYP; ITKSLRPYPI;
TKSLRPYPIE; KSLRPYPIEG; SLRPYPIEGK; LRPYPIEGKR; RPYPIEGKRL;
PYPIEGKRLL; YPIEGKRLLT; PIEGKRLLTV; IEGKRLLTVD; EGKRLLTVDE;
GKRLLTVDEA; KRLLTVDEAM; RLLTVDEAMT; LLTVDEAMTE; LTVDEAMTED;
TVDEAMTEDA; VDEAMTEDAY; DEAMTEDAYE; EAMTEDAYEV; AMTEDAYEVQ;
MTEDAYEVQK; TEDAYEVQKN; EDAYEVQKNP; DAYEVQKNPL; AYEVQKNPLN;
YEVQKNPLNL; EVQKNPLNLF; VQKNPLNLFW; QKNPLNLFWL; KNPLNLFWLI;
NPLNLFWLIG; PLNLFWLIGY; LNLFWLIGYR; NLFWLIGYRF; LFWLIGYRFS;
FWLIGYRFSD; WLIGYRFSDL; LIGYRFSDLS; IGYRFSDLSV; GYRFSDLSVK;
YRFSDLSVKL; RFSDLSVKLS; FSDLSVKLSY; SDLSVKLSYE; DLSVKLSYER;
LSVKLSYERP; SVKLSYERPD; VKLSYERPDI; KLSYERPDIY; LSYERPDIYY;
SYERPDIYYG; YERPDIYYGI; ERPDIYYGII; RPDIYYGIID; PDIYYGIIDA;
DIYYGIIDAF; IYYGIIDAFD; YYGIIDAFDY; YGIIDAFDYG; GIIDAFDYGD;
IIDAFDYGDI; IDAFDYGDIQ; DAFDYGDIQV; AFDYGDIQVP; FDYGDIQVPK;
DYGDIQVPKN; YGDIQVPKNS; GDIQVPKNSL; DIQVPKNSLA; IQVPKNSLAI;
QVPKNSLAIK; VPKNSLAIKF; PKNSLAIKFR; KNSLAIKFRN; NSLAIKFRNE;
SLAIKFRNEE; LAIKFRNEEA; AIKFRNEEAA; IKFRNEEAAF; KFRNEEAAFL;
FRNEEAAFLA; RNEEAAFLAG; NEEAAFLAGY; EEAAFLAGYI; EAAFLAGYIA;
AAFLAGYIAA; AFLAGYIAAK; FLAGYIAAKM; LAGYIAAKMS; AGYIAAKMSR;
GYIAAKMSRK; YIAAKMSRKE; IAAKMSRKEK; AAKMSRKEKI; AKMSRKEKIG;
KMSRKEKIGF; MSRKEKIGFL; SRKEKIGFLT; RKEKIGFLTG; KEKIGFLTGP;
EKIGFLTGPM; KIGFLTGPMS; IGFLTGPMSE; GFLTGPMSEH; FLTGPMSEHV;
LTGPMSEHVK; TGPMSEHVKD; GPMSEHVKDF; PMSEHVKDFK; MSEHVKDFKF;
SEHVKDFKFG; EHVKDFKFGF; HVKDFKFGFK; VKDFKFGFKA; KDFKFGFKAG;
DFKFGFKAGI; FKFGFKAGIF; KFGFKAGIFY; FGFKAGIFYA; GFKAGIFYAN;
FKAGIFYANP; KAGIFYANPK; AGIFYANPKL; GIFYANPKLR; IFYANPKLRL;
FYANPKLRLV; YANPKLRLVS; ANPKLRLVSK; NPKLRLVSKK; PKLRLVSKKA;
KLRLVSKKAP; LRLVSKKAPS; RLVSKKAPSL; LVSKKAPSLF; VSKKAPSLFD;

SKKAPSLFDK; KKAPSLFDKE; KAPSLFDKEK; APSLFDKEKG; PSLFDKEKGK;
SLFDKEKGKA; LFDKEKGKAM; FDKEKGKAMA; DKEKGKAMAL; KEKGKAMALF;
EKGKAMALFM; KGKAMALFMY; GKAMALFMYK; KAMALFMYKE; AMALFMYKED;
MALFMYKEDK; ALFMYKEDKV; LFMYKEDKVG; FMYKEDKVGV; MYKEDKVGVI;
YKEDKVGVIF; KEDKVGVIFP; EDKVGVIFPI; DKVGVIFPIA; KVGVIFPIAG;
VGVIFPIAGI; GVIFPIAGIT; VIFPIAGITG; IFPIAGITGL; FPIAGITGLG;
PIAGITGLGV; IAGITGLGVY; AGITGLGVYD; GITGLGVYDA; ITGLGVYDAA;
TGLGVYDAAK; GLGVYDAAKE; LGVYDAAKEL; GVYDAAKELG; VYDAAKELGP;
YDAAKELGPK; DAAKELGPKY; AAKELGPKYY; AKELGPKYYV; KELGPKYYVI;
ELGPKYYVIG; LGPKYYVIGL; GPKYYVIGLN; PKYYVIGLNQ; KYYVIGLNQD;
YYVIGLNQDQ; YVIGLNQDQS; VIGLNQDQSY; IGLNQDQSYI; GLNQDQSYIA;
LNQDQSYIAP; NQDQSYIAPQ; QDQSYIAPQN; DQSYIAPQNV; QSYIAPQNVI;
SYIAPQNVIT; YIAPQNVITS; IAPQNVITSI; APQNVITSII; PQNVITSIIK;
QNVITSIIKD; NVITSIIKDI; VITSIIKDIG; ITSIIKDIGK; TSIIKDIGKV;
SIIKDIGKVI; IIKDIGKVIY; IKDIGKVIYS; KDIGKVIYSI; DIGKVIYSIS;
IGKVIYSISS; GKVIYSISSE; KVIYSISSEY; VIYSISSEYI; IYSISSEYIN;
YSISSEYINN; SISSEYINNR; ISSEYINNRV; SSEYINNRVF; SEYINNRVFK;
EYINNRVFKG; YINNRVFKGG; INNRVFKGGI; NNRVFKGGII; NRVFKGGIII;
RVFKGGIIID; VFKGGIIIDR; FKGGIIIDRG; KGGIIIDRGL; GGIIIDRGLK;
GIIIDRGLKE; IIIDRGLKEG; IIDRGLKEGV; IDRGLKEGVI; DRGLKEGVIE;
RGLKEGVIEI; GLKEGVIEIV; LKEGVIEIVK; KEGVIEIVKD; EGVIEIVKDP;
GVIEIVKDPD; VIEIVKDPDV; IEIVKDPDVL; EIVKDPDVLN; IVKDPDVLNN;
VKDPDVLNNR; KDPDVLNNRL; DPDVLNNRLV; PDVLNNRLVD; DVLNNRLVDE;
VLNNRLVDEV; LNNRLVDEVI; NNRLVDEVID; NRLVDEVIDL; RLVDEVIDLE;
LVDEVIDLEN; VDEVIDLENK; DEVIDLENKI; EVIDLENKII; VIDLENKIIS;
IDLENKIISG; DLENKIISGE; LENKIISGEI; ENKIISGEII; NKIISGEIIV;
KIISGEIIVP; IISGEIIVPD; ISGEIIVPDS; SGEIIVPDSE; GEIIVPDSEY;
EIIVPDSEYA; IIVPDSEYAF; IVPDSEYAFD; VPDSEYAFDL; PDSEYAFDLF;
DSEYAFDLFK; SEYAFDLFKS; EYAFDLFKSK; YAFDLFKSKL;

11 mers:
MFKRFIFITLS; FKRFIFITLSL; KRFIFITLSLL; RFIFITLSLLV;
FIFITLSLLVF; IFITLSLLVFA; FITLSLLVFAC; ITLSLLVFACF;
TLSLLVFACFK; LSLLVFACFKS; SLLVFACFKSN; LLVFACFKSNK;
LVFACFKSNKK; VFACFKSNKKS; FACFKSNKKSI; ACFKSNKKSIK;
CFKSNKKSIKS; FKSNKKSIKSD; KSNKKSIKSDK; SNKKSIKSDKV;
NKKSIKSDKVV; KKSIKSDKVVV; KSIKSDKVVVG; SIKSDKVVVGV;
IKSDKVVVGVL; KSDKVVVGVLA; SDKVVVGVLAH; DKVVVGVLAHG;
KVVVGVLAHGS; VVVGVLAHGSF; VVGVLAHGSFY; VGVLAHGSFYD;
GVLAHGSFYDK; VLAHGSFYDKG; LAHGSFYDKGY; AHGSFYDKGYN;
HGSFYDKGYNQ; GSFYDKGYNQS; SFYDKGYNQSV; FYDKGYNQSVH;
YDKGYNQSVHD; DKGYNQSVHDG; KGYNQSVHDGV; GYNQSVHDGVV;
YNQSVHDGVVK; NQSVHDGVVKL; QSVHDGVVKLR; SVHDGVVKLRD;
VHDGVVKLRDN; HDGVVKLRDNF; DGVVKLRDNFG; GVVKLRDNFGI;
VVKLRDNFGIK; VKLRDNFGIKL; KLRDNFGIKLI; LRDNFGIKLIT;
RDNFGIKLITK; DNFGIKLITKS; NFGIKLITKSL; FGIKLITKSLR;
GIKLITKSLRP; IKLITKSLRPY; KLITKSLRPYP; LITKSLRPYPI;
ITKSLRPYPIE; TKSLRPYPIEG; KSLRPYPIEGK; SLRPYPIEGKR;
LRPYPIEGKRL; RPYPIEGKRLL; PYPIEGKRLLT; YPIEGKRLLTV;
PIEGKRLLTVD; IEGKRLLTVDE; EGKRLLTVDEA; GKRLLTVDEAM;
KRLLTVDEAMT; RLLTVDEAMTE; LLTVDEAMTED; LTVDEAMTEDA;
TVDEAMTEDAY; VDEAMTEDAYE; DEAMTEDAYEV; EAMTEDAYEVQ;
AMTEDAYEVQK; MTEDAYEVQKN; TEDAYEVQKNP; EDAYEVQKNPL;
DAYEVQKNPLN; AYEVQKNPLNL; YEVQKNPLNLF; EVQKNPLNLFW;
VQKNPLNLFWL; QKNPLNLFWLI; KNPLNLFWLIG; NPLNLFWLIGY;
PLNLFWLIGYR; LNLFWLIGYRF; NLFWLIGYRFS; LFWLIGYRFSD;
FWLIGYRFSDL; WLIGYRFSDLS; LIGYRFSDLSV; IGYRFSDLSVK;

GYRFSDLSVKL; YRFSDLSVKLS; RFSDLSVKLSY; FSDLSVKLSYE;
SDLSVKLSYER; DLSVKLSYERP; LSVKLSYERPD; SVKLSYERPDI;
VKLSYERPDIY; KLSYERPDIYY; LSYERPDIYYG; SYERPDIYYGI;
YERPDIYYGII; ERPDIYYGIID; RPDIYYGIIDA; PDIYYGIIDAF;
DIYYGIIDAFD; IYYGIIDAFDY; YYGIIDAFDYG; YGIIDAFDYGD;
GIIDAFDYGDI; IIDAFDYGDIQ; IDAFDYGDIQV; DAFDYGDIQVP;
AFDYGDIQVPK; FDYGDIQVPKN; DYGDIQVPKNS; YGDIQVPKNSL;
GDIQVPKNSLA; DIQVPKNSLAI; IQVPKNSLAIK; QVPKNSLAIKF;
VPKNSLAIKFR; PKNSLAIKFRN; KNSLAIKFRNE; NSLAIKFRNEE;
SLAIKFRNEEA; LAIKFRNEEAA; AIKFRNEEAAF; IKFRNEEAAFL;
KFRNEEAAFLA; FRNEEAAFLAG; RNEEAAFLAGY; NEEAAFLAGYI;
EEAAFLAGYIA; EAAFLAGYIAA; AAFLAGYIAAK; AFLAGYIAAKM;
FLAGYIAAKMS; LAGYIAAKMSR; AGYIAAKMSRK; GYIAAKMSRKE;
YIAAKMSRKEK; IAAKMSRKEKI; AAKMSRKEKIG; AKMSRKEKIGF;
KMSRKEKIGFL; MSRKEKIGFLT; SRKEKIGFLTG; RKEKIGFLTGP;
KEKIGFLTGPM; EKIGFLTGPMS; KIGFLTGPMSE; IGFLTGPMSEH;
GFLTGPMSEHV; FLTGPMSEHVK; LTGPMSEHVKD; TGPMSEHVKDF;
GPMSEHVKDFK; PMSEHVKDFKF; MSEHVKDFKFG; SEHVKDFKFGF;
EHVKDFKFGFK; HVKDFKFGFKA; VKDFKFGFKAG; KDFKFGFKAGI;
DFKFGFKAGIF; FKFGFKAGIFY; KFGFKAGIFYA; FGFKAGIFYAN;
GFKAGIFYANP; FKAGIFYANPK; KAGIFYANPKL; AGIFYANPKLR;
GIFYANPKLRL; IFYANPKLRLV; FYANPKLRLVS; YANPKLRLVSK;
ANPKLRLVSKK; NPKLRLVSKKA; PKLRLVSKKAP; KLRLVSKKAPS;
LRLVSKKAPSL; RLVSKKAPSLF; LVSKKAPSLFD; VSKKAPSLFDK;
SKKAPSLFDKE; KKAPSLFDKEK; KAPSLFDKEKG; APSLFDKEKGK;
PSLFDKEKGKA; SLFDKEKGKAM; LFDKEKGKAMA; FDKEKGKAMAL;
DKEKGKAMALF; KEKGKAMALFM; EKGKAMALFMY; KGKAMALFMYK;
GKAMALFMYKE; KAMALFMYKED; AMALFMYKEDK; MALFMYKEDKV;
ALFMYKEDKVG; LFMYKEDKVGV; FMYKEDKVGVI; MYKEDKVGVIF;
YKEDKVGVIFP; KEDKVGVIFPI; EDKVGVIFPIA; DKVGVIFPIAG;
KVGVIFPIAGI; VGVIFPIAGIT; GVIFPIAGITG; VIFPIAGITGL;
IFPIAGITGLG; FPIAGITGLGV; PIAGITGLGVY; IAGITGLGVYD;
AGITGLGVYDA; GITGLGVYDAA; ITGLGVYDAAK; TGLGVYDAAKE;
GLGVYDAAKEL; LGVYDAAKELG; GVYDAAKELGP; VYDAAKELGPK;
YDAAKELGPKY; DAAKELGPKYY; AAKELGPKYYV; AKELGPKYYVI;
KELGPKYYVIG; ELGPKYYVIGL; LGPKYYVIGLN; GPKYYVIGLNQ;
PKYYVIGLNQD; KYYVIGLNQDQ; YYVIGLNQDQS; YVIGLNQDQSY;
VIGLNQDQSYI; IGLNQDQSYIA; GLNQDQSYIAP; LNQDQSYIAPQ;
NQDQSYIAPQN; QDQSYIAPQNV; DQSYIAPQNVI; QSYIAPQNVIT;
SYIAPQNVITS; YIAPQNVITSI; IAPQNVITSII; APQNVITSIIK;
PQNVITSIIKD; QNVITSIIKDI; NVITSIIKDIG; VITSIIKDIGK;
ITSIIKDIGKV; TSIIKDIGKVI; SIIKDIGKVIY; IIKDIGKVIYS;
IKDIGKVIYSI; KDIGKVIYSIS; DIGKVIYSISS; IGKVIYSISSE;
GKVIYSISSEY; KVIYSISSEYI; VIYSISSEYIN; IYSISSEYINN;
YSISSEYINNR; SISSEYINNRV; ISSEYINNRVF; SSEYINNRVFK;
SEYINNRVFKG; EYINNRVFKGG; YINNRVFKGGI; INNRVFKGGII;
NNRVFKGGIII; NRVFKGGIIID; RVFKGGIIIDR; VFKGGIIIDRG;
FKGGIIIDRGL; KGGIIIDRGLK; GGIIIDRGLKE; GIIIDRGLKEG;
IIIDRGLKEGV; IIDRGLKEGVI; IDRGLKEGVIE; DRGLKEGVIEI;
RGLKEGVIEIV; GLKEGVIEIVK; LKEGVIEIVKD; KEGVIEIVKDP;
EGVIEIVKDPD; GVIEIVKDPDV; VIEIVKDPDVL; IEIVKDPDVLN;
EIVKDPDVLNN; IVKDPDVLNNR; VKDPDVLNNRL; KDPDVLNNRLV;
DPDVLNNRLVD; PDVLNNRLVDE; DVLNNRLVDEV; VLNNRLVDEVI;
LNNRLVDEVID; NNRLVDEVIDL; NRLVDEVIDLE; RLVDEVIDLEN;
LVDEVIDLENK; VDEVIDLENKI; DEVIDLENKII; EVIDLENKIIS;
VIDLENKIISG; IDLENKIISGE; DLENKIISGEI; LENKIISGEII;
ENKIISGEIIV; NKIISGEIIVP; KIISGEIIVPD; IISGEIIVPDS;

608

| | |
|---|---|
| | ISGEIIVPDSE; SGEIIVPDSEY; GEIIVPDSEYA; EIIVPDSEYAF;<br>IIVPDSEYAFD; IVPDSEYAFDL; VPDSEYAFDLF; PDSEYAFDLFK;<br>DSEYAFDLFKS; SEYAFDLFKSK; EYAFDLFKSKL; |
| NP_212519.1B<br>Basic membrane<br>protein D (bmpD)<br>[Borrelia burgdorferi<br>B31 | SEQ ID NO 4075-5480/<br>8 mers:<br>MDNYYEIY; DNYYEIYF; NYYEIYFL; YYEIYFLY; YEIYFLYF; EIYFLYFF;<br>IYFLYFFI; YFLYFFIK; FLYFFIKS; LYFFIKSK; YFFIKSKE; FFIKSKED;<br>FIKSKEDI; IKSKEDIF; KSKEDIFM; SKEDIFML; KEDIFMLK; EDIFMLKK;<br>DIFMLKKV; IFMLKKVY; FMLKKVYY; MLKKVYYF; LKKVYYFL; KKVYYFLI;<br>KVYYFLIF; VYYFLIFL; YYFLIFLF; YFLIFLFI; FLIFLFIV; LIFLFIVA;<br>IFLFIVAC; FLFIVACS; LFIVACSS; FIVACSSS; IVACSSSD; VACSSSDD;<br>ACSSSDDG; CSSSDDGK; SSSDDGKS; SSDDGKSE; SDDGKSEA; DDGKSEAK;<br>DGKSEAKT; GKSEAKTV; KSEAKTVS; SEAKTVSL; EAKTVSLI; AKTVSLIV;<br>KTVSLIVD; TVSLIVDG; VSLIVDGA; SLIVDGAF; LIVDGAFD; IVDGAFDD;<br>VDGAFDDK; DGAFDDKG; GAFDDKGF; AFDDKGFN; FDDKGFNE; DDKGFNES;<br>DKGFNESS; KGFNESSS; GFNESSSK; FNESSSKA; NESSSKAI; ESSSKAIR;<br>SSSKAIRK; SSKAIRKL; SKAIRKLK; KAIRKLKA; AIRKLKAD; IRKLKADL;<br>RKLKADLN; KLKADLNI; LKADLNIN; KADLNINI; ADLNINII; DLNINIIE;<br>LNINIIEK; NINIIEKA; INIIEKAS; NIIEKAST; IIEKASTG; IEKASTGN;<br>EKASTGNS; KASTGNSY; ASTGNSYL; STGNSYLG; TGNSYLGD; GNSYLGDI;<br>NSYLGDIA; SYLGDIAN; YLGDIANL; LGDIANLE; GDIANLED; DIANLEDG;<br>IANLEDGN; ANLEDGNS; NLEDGNSN; LEDGNSNL; EDGNSNLI; DGNSNLIW;<br>GNSNLIWG; NSNLIWGI; SNLIWGIG; NLIWGIGF; LIWGIGFR; IWGIGFRL;<br>WGIGFRLS; GIGFRLSD; IGFRLSDI; GFRLSDIL; FRLSDILF; RLSDILFQ;<br>LSDILFQR; SDILFQRA; DILFQRAS; ILFQRASE; LFQRASEN; FQRASENV;<br>QRASENVS; RASENVSV; ASENVSVN; SENVSVNY; ENVSVNYA; NVSVNYAI;<br>VSVNYAII; SVNYAIIE; VNYAIIEG; NYAIIEGV; YAIIEGVY; AIIEGVYD;<br>IIEGVYDE; IEGVYDEI; EGVYDEIQ; GVYDEIQI; VYDEIQIP; YDEIQIPK;<br>DEIQIPKN; EIQIPKNL; IQIPKNLL; QIPKNLLN; IPKNLLNI; PKNLLNIS;<br>KNLLNISF; NLLNISFR; LLNISFRS; LNISFRSE; NISFRSEE; ISFRSEEV;<br>SFRSEEVA; FRSEEVAF; RSEEVAFL; SEEVAFLA; EEVAFLAG; EVAFLAGY;<br>VAFLAGYF; AFLAGYFA; FLAGYFAS; LAGYFASK; AGYFASKA; GYFASKAS;<br>YFASKASK; FASKASKT; ASKASKTG; SKASKTGK; KASKTGKI; ASKTGKIG;<br>SKTGKIGF; KTGKIGFV; TGKIGFVG; GKIGFVGG; KIGFVGGV; IGFVGGVR;<br>GFVGGVRG; FVGGVRGK; VGGVRGKV; GGVRGKVL; GVRGKVLE; VRGKVLES;<br>RGKVLESF; GKVLESFM; KVLESFMY; VLESFMYG; LESFMYGY; ESFMYGYE;<br>SFMYGYEA; FMYGYEAG; MYGYEAGA; YGYEAGAK; GYEAGAKY; YEAGAKYA;<br>EAGAKYAN; AGAKYANS; GAKYANSN; AKYANSNI; KYANSNIK; YANSNIKV;<br>ANSNIKVV; NSNIKVVS; SNIKVVSQ; NIKVVSQY; IKVVSQYV; KVVSQYVG;<br>VVSQYVGT; VSQYVGTF; SQYVGTFG; QYVGTFGD; YVGTFGDF; VGTFGDFG;<br>GTFGDFGL; TFGDFGLG; FGDFGLGR; GDFGLGRS; DFGLGRST; FGLGRSTA;<br>GLGRSTAS; LGRSTASN; GRSTASNM; RSTASNMY; STASNMYR; TASNMYRD;<br>ASNMYRDG; SNMYRDGV; NMYRDGVD; MYRDGVDI; YRDGVDII; RDGVDIIF;<br>DGVDIIFA; GVDIIFAA; VDIIFAAA; DIIFAAAG; IIFAAAGL; IFAAAGLS;<br>FAAAGLSG; AAAGLSGI; AAGLSGIG; AGLSGIGV; GLSGIGVI; LSGIGVIE;<br>SGIGVIEA; GIGVIEAA; IGVIEAAK; GVIEAAKE; VIEAAKEL; IEAAKELG;<br>EAAKELGP; AAKELGPD; AKELGPDH; KELGPDHY; ELGPDHYI; LGPDHYII;<br>GPDHYIIG; PDHYIIGV; DHYIIGVD; HYIIGVDQ; YIIGVDQD; IIGVDQDQ;<br>IGVDQDQS; GVDQDQSY; VDQDQSYL; DQDQSYLA; QDQSYLAP; DQSYLAPN;<br>QSYLAPNN; SYLAPNNV; YLAPNNVI; LAPNNVIV; APNNVIVS; PNNVIVSA;<br>NNVIVSAV; NVIVSAVK; VIVSAVKK; IVSAVKKV; VSAVKKVD; SAVKKVDS;<br>AVKKVDSL; VKKVDSLM; KKVDSLMY; KVDSLMYS; VDSLMYSL; DSLMYSLT;<br>SLMYSLTK; LMYSLTKK; MYSLTKKY; YSLTKKYL; SLTKKYLE; LTKKYLET;<br>TKKYLETG; KKYLETGV; KYLETGVL; YLETGVLD; LETGVLDG; ETGVLDGG;<br>TGVLDGGK; GVLDGGKT; VLDGGKTM; LDGGKTMF; DGGKTMFL; GGKTMFLG;<br>GKTMFLGL; KTMFLGLK; TMFLGLKE; MFLGLKED; FLGLKEDG; LGLKEDGL; |

GLKEDGLG; LKEDGLGL; KEDGLGLV; EDGLGLVL; DGLGLVLN; GLGLVLNE;
LGLVLNEN; GLVLNENL; LVLNENLK; VLNENLKS; LNENLKSN; NENLKSNY;
ENLKSNYS; NLKSNYSE; LKSNYSEI; KSNYSEIY; SNYSEIYN; NYSEIYNK;
YSEIYNKS; SEIYNKSL; EIYNKSLK; IYNKSLKI; YNKSLKIG; NKSLKIGQ;
KSLKIGQS; SLKIGQSI; LKIGQSIM; KIGQSIMN; IGQSIMNG; GQSIMNGI;
QSIMNGII; SIMNGIIK; IMNGIIKV; MNGIIKVP; NGIIKVPY; GIIKVPYD;
IIKVPYDK; IKVPYDKV; KVPYDKVS; VPYDKVSY; PYDKVSYF; YDKVSYFV;
DKVSYFVL; KVSYFVLQ; VSYFVLQM; SYFVLQME; YFVLQMEN;

9 mers:
MDNYYEIYF; DNYYEIYFL; NYYEIYFLY; YYEIYFLYF; YEIYFLYFF;
EIYFLYFFI; IYFLYFFIK; YFLYFFIKS; FLYFFIKSK; LYFFIKSKE;
YFFIKSKED; FFIKSKEDI; FIKSKEDIF; IKSKEDIFM; KSKEDIFML;
SKEDIFMLK; KEDIFMLKK; EDIFMLKKV; DIFMLKKVY; IFMLKKVYY;
FMLKKVYYF; MLKKVYYFL; LKKVYYFLI; KKVYYFLIF; KVYYFLIFL;
VYYFLIFLF; YYFLIFLFI; YFLIFLFIV; FLIFLFIVA; LIFLFIVAC;
IFLFIVACS; FLFIVACSS; LFIVACSSS; FIVACSSSD; IVACSSSDD;
VACSSSDDG; ACSSSDDGK; CSSSDDGKS; SSSDDGKSE; SSDDGKSEA;
SDDGKSEAK; DDGKSEAKT; DGKSEAKTV; GKSEAKTVS; KSEAKTVSL;
SEAKTVSLI; EAKTVSLIV; AKTVSLIVD; KTVSLIVDG; TVSLIVDGA;
VSLIVDGAF; SLIVDGAFD; LIVDGAFDD; IVDGAFDDK; VDGAFDDKG;
DGAFDDKGF; GAFDDKGFN; AFDDKGFNE; FDDKGFNES; DDKGFNESS;
DKGFNESSS; KGFNESSSK; GFNESSSKA; FNESSSKAI; NESSSKAIR;
ESSSKAIRK; SSSKAIRKL; SSKAIRKLK; SKAIRKLKA; KAIRKLKAD;
AIRKLKADL; IRKLKADLN; RKLKADLNI; KLKADLNIN; LKADLNINI;
KADLNINII; ADLNINIIE; DLNINIIEK; LNINIIEKA; NINIIEKAS;
INIIEKAST; NIIEKASTG; IIEKASTGN; IEKASTGNS; EKASTGNSY;
KASTGNSYL; ASTGNSYLG; STGNSYLGD; TGNSYLGDI; GNSYLGDIA;
NSYLGDIAN; SYLGDIANL; YLGDIANLE; LGDIANLED; GDIANLEDG;
DIANLEDGN; IANLEDGNS; ANLEDGNSN; NLEDGNSNL; LEDGNSNLI;
EDGNSNLIW; DGNSNLIWG; GNSNLIWGI; NSNLIWGIG; SNLIWGIGF;
NLIWGIGFR; LIWGIGFRL; IWGIGFRLS; WGIGFRLSD; GIGFRLSDI;
IGFRLSDIL; GFRLSDILF; FRLSDILFQ; RLSDILFQR; LSDILFQRA;
SDILFQRAS; DILFQRASE; ILFQRASEN; LFQRASENV; FQRASENVS;
QRASENVSV; RASENVSVN; ASENVSVNY; SENVSVNYA; ENVSVNYAI;
NVSVNYAII; VSVNYAIIE; SVNYAIIEG; VNYAIIEGV; NYAIIEGVY;
YAIIEGVYD; AIIEGVYDE; IIEGVYDEI; IEGVYDEIQ; EGVYDEIQI;
GVYDEIQIP; VYDEIQIPK; YDEIQIPKN; DEIQIPKNL; EIQIPKNLL;
IQIPKNLLN; QIPKNLLNI; IPKNLLNIS; PKNLLNISF; KNLLNISFR;
NLLNISFRS; LLNISFRSE; LNISFRSEE; NISFRSEEV; ISFRSEEVA;
SFRSEEVAF; FRSEEVAFL; RSEEVAFLA; SEEVAFLAG; EEVAFLAGY;
EVAFLAGYF; VAFLAGYFA; AFLAGYFAS; FLAGYFASK; LAGYFASKA;
AGYFASKAS; GYFASKASK; YFASKASKT; FASKASKTG; ASKASKTGK;
SKASKTGKI; KASKTGKIG; ASKTGKIGF; SKTGKIGFV; KTGKIGFVG;
TGKIGFVGG; GKIGFVGGV; KIGFVGGVR; IGFVGGVRG; GFVGGVRGK;
FVGGVRGKV; VGGVRGKVL; GGVRGKVLE; GVRGKVLES; VRGKVLESF;
RGKVLESFM; GKVLESFMY; KVLESFMYG; VLESFMYGY; LESFMYGYE;
ESFMYGYEA; SFMYGYEAG; FMYGYEAGA; MYGYEAGAK; YGYEAGAKY;
GYEAGAKYA; YEAGAKYAN; EAGAKYANS; AGAKYANSN; GAKYANSNI;
AKYANSNIK; KYANSNIKV; YANSNIKVV; ANSNIKVVS; NSNIKVVSQ;
SNIKVVSQY; NIKVVSQYV; IKVVSQYVG; KVVSQYVGT; VVSQYVGTF;
VSQYVGTFG; SQYVGTFGD; QYVGTFGDF; YVGTFGDFG; VGTFGDFGL;
GTFGDFGLG; TFGDFGLGR; FGDFGLGRS; GDFGLGRST; DFGLGRSTA;
FGLGRSTAS; GLGRSTASN; LGRSTASNM; GRSTASNMY; RSTASNMYR;
STASNMYRD; TASNMYRDG; ASNMYRDGV; SNMYRDGVD; NMYRDGVDI;
MYRDGVDII; YRDGVDIIF; RDGVDIIFA; DGVDIIFAA; GVDIIFAAA;
VDIIFAAAG; DIIFAAAGL; IIFAAAGLS; IFAAAGLSG; FAAAGLSGI;

AAAGLSGIG; AAGLSGIGV; AGLSGIGVI; GLSGIGVIE; LSGIGVIEA;
SGIGVIEAA; GIGVIEAAK; IGVIEAAKE; GVIEAAKEL; VIEAAKELG;
IEAAKELGP; EAAKELGPD; AAKELGPDH; AKELGPDHY; KELGPDHYI;
ELGPDHYII; LGPDHYIIG; GPDHYIIGV; PDHYIIGVD; DHYIIGVDQ;
HYIIGVDQD; YIIGVDQDQ; IIGVDQDQS; IGVDQDQSY; GVDQDQSYL;
VDQDQSYLA; DQDQSYLAP; QDQSYLAPN; DQSYLAPNN; QSYLAPNNV;
SYLAPNNVI; YLAPNNVIV; LAPNNVIVS; APNNVIVSA; PNNVIVSAV;
NNVIVSAVK; NVIVSAVKK; VIVSAVKKV; IVSAVKKVD; VSAVKKVDS;
SAVKKVDSL; AVKKVDSLM; VKKVDSLMY; KKVDSLMYS; KVDSLMYSL;
VDSLMYSLT; DSLMYSLTK; SLMYSLTKK; LMYSLTKKY; MYSLTKKYL;
YSLTKKYLE; SLTKKYLET; LTKKYLETG; TKKYLETGV; KKYLETGVL;
KYLETGVLD; YLETGVLDG; LETGVLDGG; ETGVLDGGK; TGVLDGGKT;
GVLDGGKTM; VLDGGKTMF; LDGGKTMFL; DGGKTMFLG; GGKTMFLGL;
GKTMFLGLK; KTMFLGLKE; TMFLGLKED; MFLGLKEDG; FLGLKEDGL;
LGLKEDGLG; GLKEDGLGL; LKEDGLGLV; KEDGLGLVL; EDGLGLVLN;
DGLGLVLNE; GLGLVLNEN; LGLVLNENL; GLVLNENLK; LVLNENLKS;
VLNENLKSN; LNENLKSNY; NENLKSNYS; ENLKSNYSE; NLKSNYSEI;
LKSNYSEIY; KSNYSEIYN; SNYSEIYNK; NYSEIYNKS; YSEIYNKSL;
SEIYNKSLK; EIYNKSLKI; IYNKSLKIG; YNKSLKIGQ; NKSLKIGQS;
KSLKIGQSI; SLKIGQSIM; LKIGQSIMN; KIGQSIMNG; IGQSIMNGI;
GQSIMNGII; QSIMNGIIK; SIMNGIIKV; IMNGIIKVP; MNGIIKVPY;
NGIIKVPYD; GIIKVPYDK; IIKVPYDKV; IKVPYDKVS; KVPYDKVSY;
VPYDKVSYF; PYDKVSYFV; YDKVSYFVL; DKVSYFVLQ; KVSYFVLQM;
VSYFVLQME; SYFVLQMEN;

10 mers:
MDNYYEIYFL; DNYYEIYFLY; NYYEIYFLYF; YYEIYFLYFF; YEIYFLYFFI;
EIYFLYFFIK; IYFLYFFIKS; YFLYFFIKSK; FLYFFIKSKE; LYFFIKSKED;
YFFIKSKEDI; FFIKSKEDIF; FIKSKEDIFM; IKSKEDIFML; KSKEDIFMLK;
SKEDIFMLKK; KEDIFMLKKV; EDIFMLKKVY; DIFMLKKVYY; IFMLKKVYYF;
FMLKKVYYFL; MLKKVYYFLI; LKKVYYFLIF; KKVYYFLIFL; KVYYFLIFLF;
VYYFLIFLFI; YYFLIFLFIV; YFLIFLFIVA; FLIFLFIVAC; LIFLFIVACS;
IFLFIVACSS; FLFIVACSSS; LFIVACSSSD; FIVACSSSDD; IVACSSSDDG;
VACSSSDDGK; ACSSSDDGKS; CSSSDDGKSE; SSSDDGKSEA; SSDDGKSEAK;
SDDGKSEAKT; DDGKSEAKTV; DGKSEAKTVS; GKSEAKTVSL; KSEAKTVSLI;
SEAKTVSLIV; EAKTVSLIVD; AKTVSLIVDG; KTVSLIVDGA; TVSLIVDGAF;
VSLIVDGAFD; SLIVDGAFDD; LIVDGAFDDK; IVDGAFDDKG; VDGAFDDKGF;
DGAFDDKGFN; GAFDDKGFNE; AFDDKGFNES; FDDKGFNESS; DDKGFNESSS;
DKGFNESSSK; KGFNESSSKA; GFNESSSKAI; FNESSSKAIR; NESSSKAIRK;
ESSSKAIRKL; SSSKAIRKLK; SSKAIRKLKA; SKAIRKLKAD; KAIRKLKADL;
AIRKLKADLN; IRKLKADLNI; RKLKADLNIN; KLKADLNINI; LKADLNINII;
KADLNINIIE; ADLNINIIEK; DLNINIIEKA; LNINIIEKAS; NINIIEKAST;
INIIEKASTG; NIIEKASTGN; IIEKASTGNS; IEKASTGNSY; EKASTGNSYL;
KASTGNSYLG; ASTGNSYLGD; STGNSYLGDI; TGNSYLGDIA; GNSYLGDIAN;
NSYLGDIANL; SYLGDIANLE; YLGDIANLED; LGDIANLEDG; GDIANLEDGN;
DIANLEDGNS; IANLEDGNSN; ANLEDGNSNL; NLEDGNSNLI; LEDGNSNLIW;
EDGNSNLIWG; DGNSNLIWGI; GNSNLIWGIG; NSNLIWGIGF; SNLIWGIGFR;
NLIWGIGFRL; LIWGIGFRLS; IWGIGFRLSD; WGIGFRLSDI; GIGFRLSDIL;
IGFRLSDILF; GFRLSDILFQ; FRLSDILFQR; RLSDILFQRA; LSDILFQRAS;
SDILFQRASE; DILFQRASEN; ILFQRASENV; LFQRASENVS; FQRASENVSV;
QRASENVSVN; RASENVSVNY; ASENVSVNYA; SENVSVNYAI; ENVSVNYAII;
NVSVNYAIIE; VSVNYAIIEG; SVNYAIIEGV; VNYAIIEGVY; NYAIIEGVYD;
YAIIEGVYDE; AIIEGVYDEI; IIEGVYDEIQ; IEGVYDEIQI; EGVYDEIQIP;
GVYDEIQIPK; VYDEIQIPKN; YDEIQIPKNL; DEIQIPKNLL; EIQIPKNLLN;
IQIPKNLLNI; QIPKNLLNIS; IPKNLLNISF; PKNLLNISFR; KNLLNISFRS;
NLLNISFRSE; LLNISFRSEE; LNISFRSEEV; NISFRSEEVA; ISFRSEEVAF;
SFRSEEVAFL; FRSEEVAFLA; RSEEVAFLAG; SEEVAFLAGY; EEVAFLAGYF;

EVAFLAGYFA; VAFLAGYFAS; AFLAGYFASK; FLAGYFASKA; LAGYFASKAS;
AGYFASKASK; GYFASKASKT; YFASKASKTG; FASKASKTGK; ASKASKTGKI;
SKASKTGKIG; KASKTGKIGF; ASKTGKIGFV; SKTGKIGFVG; KTGKIGFVGG;
TGKIGFVGGV; GKIGFVGGVR; KIGFVGGVRG; IGFVGGVRGK; GFVGGVRGKV;
FVGGVRGKVL; VGGVRGKVLE; GGVRGKVLES; GVRGKVLESF; VRGKVLESFM;
RGKVLESFMY; GKVLESFMYG; KVLESFMYGY; VLESFMYGYE; LESFMYGYEA;
ESFMYGYEAG; SFMYGYEAGA; FMYGYEAGAK; MYGYEAGAKY; YGYEAGAKYA;
GYEAGAKYAN; YEAGAKYANS; EAGAKYANSN; AGAKYANSNI; GAKYANSNIK;
AKYANSNIKV; KYANSNIKVV; YANSNIKVVS; ANSNIKVVSQ; NSNIKVVSQY;
SNIKVVSQYV; NIKVVSQYVG; IKVVSQYVGT; KVVSQYVGTF; VVSQYVGTFG;
VSQYVGTFGD; SQYVGTFGDF; QYVGTFGDFG; YVGTFGDFGL; VGTFGDFGLG;
GTFGDFGLGR; TFGDFGLGRS; FGDFGLGRST; GDFGLGRSTA; DFGLGRSTAS;
FGLGRSTASN; GLGRSTASNM; LGRSTASNMY; GRSTASNMYR; RSTASNMYRD;
STASNMYRDG; TASNMYRDGV; ASNMYRDGVD; SNMYRDGVDI; NMYRDGVDII;
MYRDGVDIIF; YRDGVDIIFA; RDGVDIIFAA; DGVDIIFAAA; GVDIIFAAAG;
VDIIFAAAGL; DIIFAAAGLS; IIFAAAGLSG; IFAAAGLSGI; FAAAGLSGIG;
AAAGLSGIGV; AAGLSGIGVI; AGLSGIGVIE; GLSGIGVIEA; LSGIGVIEAA;
SGIGVIEAAK; GIGVIEAAKE; IGVIEAAKEL; GVIEAAKELG; VIEAAKELGP;
IEAAKELGPD; EAAKELGPDH; AAKELGPDHY; AKELGPDHYI; KELGPDHYII;
ELGPDHYIIG; LGPDHYIIGV; GPDHYIIGVD; PDHYIIGVDQ; DHYIIGVDQD;
HYIIGVDQDQ; YIIGVDQDQS; IIGVDQDQSY; IGVDQDQSYL; GVDQDQSYLA;
VDQDQSYLAP; DQDQSYLAPN; QDQSYLAPNN; DQSYLAPNNV; QSYLAPNNVI;
SYLAPNNVIV; YLAPNNVIVS; LAPNNVIVSA; APNNVIVSAV; PNNVIVSAVK;
NNVIVSAVKK; NVIVSAVKKV; VIVSAVKKVD; IVSAVKKVDS; VSAVKKVDSL;
SAVKKVDSLM; AVKKVDSLMY; VKKVDSLMYS; KKVDSLMYSL; KVDSLMYSLT;
VDSLMYSLTK; DSLMYSLTKK; SLMYSLTKKY; LMYSLTKKYL; MYSLTKKYLE;
YSLTKKYLET; SLTKKYLETG; LTKKYLETGV; TKKYLETGVL; KKYLETGVLD;
KYLETGVLDG; YLETGVLDGG; LETGVLDGGK; ETGVLDGGKT; TGVLDGGKTM;
GVLDGGKTMF; VLDGGKTMFL; LDGGKTMFLG; DGGKTMFLGL; GGKTMFLGLK;
GKTMFLGLKE; KTMFLGLKED; TMFLGLKEDG; MFLGLKEDGL; FLGLKEDGLG;
LGLKEDGLGL; GLKEDGLGLV; LKEDGLGLVL; KEDGLGLVLN; EDGLGLVLNE;
DGLGLVLNEN; GLGLVLNENL; LGLVLNENLK; GLVLNENLKS; LVLNENLKSN;
VLNENLKSNY; LNENLKSNYS; NENLKSNYSE; ENLKSNYSEI; NLKSNYSEIY;
LKSNYSEIYN; KSNYSEIYNK; SNYSEIYNKS; NYSEIYNKSL; YSEIYNKSLK;
SEIYNKSLKI; EIYNKSLKIG; IYNKSLKIGQ; YNKSLKIGQS; NKSLKIGQSI;
KSLKIGQSIM; SLKIGQSIMN; LKIGQSIMNG; KIGQSIMNGI; IGQSIMNGII;
GQSIMNGIIK; QSIMNGIIKV; SIMNGIIKVP; IMNGIIKVPY; MNGIIKVPYD;
NGIIKVPYDK; GIIKVPYDKV; IIKVPYDKVS; IKVPYDKVSY; KVPYDKVSYF;
VPYDKVSYFV; PYDKVSYFVL; YDKVSYFVLQ; DKVSYFVLQM; KVSYFVLQME;
VSYFVLQMEN;

11 mers:
MDNYYEIYFLY; DNYYEIYFLYF; NYYEIYFLYFF; YYEIYFLYFFI;
YEIYFLYFFIK; EIYFLYFFIKS; IYFLYFFIKSK; YFLYFFIKSKE;
FLYFFIKSKED; LYFFIKSKEDI; YFFIKSKEDIF; FFIKSKEDIFM;
FIKSKEDIFML; IKSKEDIFMLK; KSKEDIFMLKK; SKEDIFMLKKV;
KEDIFMLKKVY; EDIFMLKKVYY; DIFMLKKVYYF; IFMLKKVYYFL;
FMLKKVYYFLI; MLKKVYYFLIF; LKKVYYFLIFL; KKVYYFLIFLF;
KVYYFLIFLFI; VYYFLIFLFIV; YYFLIFLFIVA; YFLIFLFIVAC;
FLIFLFIVACS; LIFLFIVACSS; IFLFIVACSSS; FLFIVACSSSD;
LFIVACSSSDD; FIVACSSSDDG; IVACSSSDDGK; VACSSSDDGKS;
ACSSSDDGKSE; CSSSDDGKSEA; SSSDDGKSEAK; SSDDGKSEAKT;
SDDGKSEAKTV; DDGKSEAKTVS; DGKSEAKTVSL; GKSEAKTVSLI;
KSEAKTVSLIV; SEAKTVSLIVD; EAKTVSLIVDG; AKTVSLIVDGA;
KTVSLIVDGAF; TVSLIVDGAFD; VSLIVDGAFDD; SLIVDGAFDDK;
LIVDGAFDDKG; IVDGAFDDKGF; VDGAFDDKGFN; DGAFDDKGFNE;
GAFDDKGFNES; AFDDKGFNESS; FDDKGFNESSS; DDKGFNESSSK;

| | DKGFNESSSKA; KGFNESSSKAI; GFNESSSKAIR; FNESSSKAIRK; |
| | NESSSKAIRKL; ESSSKAIRKLK; SSSKAIRKLKA; SSKAIRKLKAD; |
| | SKAIRKLKADL; KAIRKLKADLN; AIRKLKADLNI; IRKLKADLNIN; |
| | RKLKADLNINI; KLKADLNINII; LKADLNINIIE; KADLNINIIEK; |
| | ADLNINIIEKA; DLNINIIEKAS; LNINIIEKAST; NINIIEKASTG; |
| | INIIEKASTGN; NIIEKASTGNS; IIEKASTGNSY; IEKASTGNSYL; |
| | EKASTGNSYLG; KASTGNSYLGD; ASTGNSYLGDI; STGNSYLGDIA; |
| | TGNSYLGDIAN; GNSYLGDIANL; NSYLGDIANLE; SYLGDIANLED; |
| | YLGDIANLEDG; LGDIANLEDGN; GDIANLEDGNS; DIANLEDGNSN; |
| | IANLEDGNSNL; ANLEDGNSNLI; NLEDGNSNLIW; LEDGNSNLIWG; |
| | EDGNSNLIWGI; DGNSNLIWGIG; GNSNLIWGIGF; NSNLIWGIGFR; |
| | SNLIWGIGFRL; NLIWGIGFRLS; LIWGIGFRLSD; IWGIGFRLSDI; |
| | WGIGFRLSDIL; GIGFRLSDILF; IGFRLSDILFQ; GFRLSDILFQR; |
| | FRLSDILFQRA; RLSDILFQRAS; LSDILFQRASE; SDILFQRASEN; |
| | DILFQRASENV; ILFQRASENVS; LFQRASENVSV; FQRASENVSVN; |
| | QRASENVSVNY; RASENVSVNYA; ASENVSVNYAI; SENVSVNYAII; |
| | ENVSVNYAIIE; NVSVNYAIIEG; VSVNYAIIEGV; SVNYAIIEGVY; |
| | VNYAIIEGVYD; NYAIIEGVYDE; YAIIEGVYDEI; AIIEGVYDEIQ; |
| | IIEGVYDEIQI; IEGVYDEIQIP; EGVYDEIQIPK; GVYDEIQIPKN; |
| | VYDEIQIPKNL; YDEIQIPKNLL; DEIQIPKNLLN; EIQIPKNLLNI; |
| | IQIPKNLLNIS; QIPKNLLNISF; IPKNLLNISFR; PKNLLNISFRS; |
| | KNLLNISFRSE; NLLNISFRSEE; LLNISFRSEEV; LNISFRSEEVA; |
| | NISFRSEEVAF; ISFRSEEVAFL; SFRSEEVAFLA; FRSEEVAFLAG; |
| | RSEEVAFLAGY; SEEVAFLAGYF; EEVAFLAGYFA; EVAFLAGYFAS; |
| | VAFLAGYFASK; AFLAGYFASKA; FLAGYFASKAS; LAGYFASKASK; |
| | AGYFASKASKT; GYFASKASKTG; YFASKASKTGK; FASKASKTGKI; |
| | ASKASKTGKIG; SKASKTGKIGF; KASKTGKIGFV; ASKTGKIGFVG; |
| | SKTGKIGFVGG; KTGKIGFVGGV; TGKIGFVGGVR; GKIGFVGGVRG; |
| | KIGFVGGVRGK; IGFVGGVRGKV; GFVGGVRGKVL; FVGGVRGKVLE; |
| | VGGVRGKVLES; GGVRGKVLESF; GVRGKVLESFM; VRGKVLESFMY; |
| | RGKVLESFMYG; GKVLESFMYGY; KVLESFMYGYE; VLESFMYGYEA; |
| | LESFMYGYEAG; ESFMYGYEAGA; SFMYGYEAGAK; FMYGYEAGAKY; |
| | MYGYEAGAKYA; YGYEAGAKYAN; GYEAGAKYANS; YEAGAKYANSN; |
| | EAGAKYANSNI; AGAKYANSNIK; GAKYANSNIKV; AKYANSNIKVV; |
| | KYANSNIKVVS; YANSNIKVVSQ; ANSNIKVVSQY; NSNIKVVSQYV; |
| | SNIKVVSQYVG; NIKVVSQYVGT; IKVVSQYVGTF; KVVSQYVGTFG; |
| | VVSQYVGTFGD; VSQYVGTFGDF; SQYVGTFGDFG; QYVGTFGDFGL; |
| | YVGTFGDFGLG; VGTFGDFGLGR; GTFGDFGLGRS; TFGDFGLGRST; |
| | FGDFGLGRSTA; GDFGLGRSTAS; DFGLGRSTASN; FGLGRSTASNM; |
| | GLGRSTASNMY; LGRSTASNMYR; GRSTASNMYRD; RSTASNMYRDG; |
| | STASNMYRDGV; TASNMYRDGVD; ASNMYRDGVDI; SNMYRDGVDII; |
| | NMYRDGVDIIF; MYRDGVDIIFA; YRDGVDIIFAA; RDGVDIIFAAA; |
| | DGVDIIFAAAG; GVDIIFAAAGL; VDIIFAAAGLS; DIIFAAAGLSG; |
| | IIFAAAGLSGI; IFAAAGLSGIG; FAAAGLSGIGV; AAAGLSGIGVI; |
| | AAGLSGIGVIE; AGLSGIGVIEA; GLSGIGVIEAA; LSGIGVIEAAK; |
| | SGIGVIEAAKE; GIGVIEAAKEL; IGVIEAAKELG; GVIEAAKELGP; |
| | VIEAAKELGPD; IEAAKELGPDH; EAAKELGPDHY; AAKELGPDHYI; |
| | AKELGPDHYII; KELGPDHYIIG; ELGPDHYIIGV; LGPDHYIIGVD; |
| | GPDHYIIGVDQ; PDHYIIGVDQD; DHYIIGVDQDQ; HYIIGVDQDQS; |
| | YIIGVDQDQSY; IIGVDQDQSYL; IGVDQDQSYLA; GVDQDQSYLAP; |
| | VDQDQSYLAPN; DQDQSYLAPNN; QDQSYLAPNNV; DQSYLAPNNVI; |
| | QSYLAPNNVIV; SYLAPNNVIVS; YLAPNNVIVSA; LAPNNVIVSAV; |
| | APNNVIVSAVK; PNNVIVSAVKK; NNVIVSAVKKV; NVIVSAVKKVD; |
| | VIVSAVKKVDS; IVSAVKKVDSL; VSAVKKVDSLM; SAVKKVDSLMY; |
| | AVKKVDSLMYS; VKKVDSLMYSL; KKVDSLMYSLT; KVDSLMYSLTK; |
| | VDSLMYSLTKK; DSLMYSLTKKY; SLMYSLTKKYL; LMYSLTKKYLE; |
| | MYSLTKKYLET; YSLTKKYLETG; SLTKKYLETGV; LTKKYLETGVL; |

| | |
|---|---|
| | TKKYLETGVLD; KKYLETGVLDG; KYLETGVLDGG; YLETGVLDGGK; LETGVLDGGKT; ETGVLDGGKTM; TGVLDGGKTMF; GVLDGGKTMFL; VLDGGKTMFLG; LDGGKTMFLGL; DGGKTMFLGLK; GGKTMFLGLKE; GKTMFLGLKED; KTMFLGLKEDG; TMFLGLKEDGL; MFLGLKEDGLG; FLGLKEDGLGL; LGLKEDGLGLV; GLKEDGLGLVL; LKEDGLGLVLN; KEDGLGLVLNE; EDGLGLVLNEN; DGLGLVLNENL; GLGLVLNENLK; LGLVLNENLKS; GLVLNENLKSN; LVLNENLKSNY; VLNENLKSNYS; LNENLKSNYSE; NENLKSNYSEI; ENLKSNYSEIY; NLKSNYSEIYN; LKSNYSEIYNK; KSNYSEIYNKS; SNYSEIYNKSL; NYSEIYNKSLK; YSEIYNKSLKI; SEIYNKSLKIG; EIYNKSLKIGQ; IYNKSLKIGQS; YNKSLKIGQSI; NKSLKIGQSIM; KSLKIGQSIMN; SLKIGQSIMNG; LKIGQSIMNGI; KIGQSIMNGII; IGQSIMNGIIK; GQSIMNGIIKV; QSIMNGIIKVP; SIMNGIIKVPY; IMNGIIKVPYD; MNGIIKVPYDK; NGIIKVPYDKV; GIIKVPYDKVS; IIKVPYDKVSY; IKVPYDKVSYF; KVPYDKVSYFV; VPYDKVSYFVL; PYDKVSYFVLQ; YDKVSYFVLQM; DKVSYFVLQME; KVSYFVLQMEN; |
| AAC70056.1 Decorin binding protein A; DbpA [Borrelia afzelii | SEQ ID NO 5481-6126/ 8 mers: MIKYNKII; IKYNKIIL; KYNKIILT; YNKIILTL; NKIILTLT; KIILTLTL; IILTLTLL; ILTLTLLA; LTLTLLAS; TLTLLASL; LTLLASLL; TLLASLLA; LLASLLAA; LASLLAAC; ASLLAACS; SLLAACSL; LLAACSLT; LAACSLTG; AACSLTGK; ACSLTGKA; CSLTGKAR; SLTGKARL; LTGKARLE; TGKARLES; GKARLESS; KARLESSV; ARLESSVK; RLESSVKD; LESSVKDI; ESSVKDIT; SSVKDITN; SVKDITNE; VKDITNEI; KDITNEIE; DITNEIEK; ITNEIEKA; TNEIEKAI; NEIEKAIK; EIEKAIKE; IEKAIKEA; EKAIKEAE; KAIKEAED; AIKEAEDA; IKEAEDAG; KEAEDAGV; EAEDAGVK; AEDAGVKT; EDAGVKTD; DAGVKTDA; AGVKTDAF; GVKTDAFT; VKTDAFTE; KTDAFTET; TDAFTETQ; DAFTETQT; AFTETQTG; FTETQTGG; TETQTGGK; ETQTGGKV; TQTGGKVG; QTGGKVGG; TGGKVGGS; GGKVGGSQ; GKVGGSQI; KVGGSQIR; VGGSQIRA; GGSQIRAA; GSQIRAAK; SQIRAAKI; QIRAAKIR; IRAAKIRV; RAAKIRVA; AAKIRVAD; AKIRVADL; KIRVADLT; IRVADLTI; RVADLTIK; VADLTIKF; ADLTIKFL; DLTIKFLE; LTIKFLEA; TIKFLEAT; IKFLEATE; KFLEATEE; FLEATEEE; LEATEEET; EATEEETI; ATEEETIT; TEEETITF; EEETITFK; EETITFKE; ETITFKEN; TITFKENG; ITFKENGA; TFKENGAG; FKENGAGE; KENGAGEE; ENGAGEED; NGAGEEDF; GAGEEDFS; AGEEDFSG; GEEDFSGI; EEDFSGIY; EDFSGIYD; DFSGIYDL; FSGIYDLI; SGIYDLIL; GIYDLILN; IYDLILNA; YDLILNAA; DLILNAAK; LILNAAKA; ILNAAKAV; LNAAKAVE; NAAKAVEK; AAKAVEKI; AKAVEKIG; KAVEKIGM; AVEKIGMQ; VEKIGMQG; EKIGMQGM; KIGMQGMK; IGMQGMKQ; GMQGMKQA; MQGMKQAV; QGMKQAVE; GMKQAVEE; MKQAVEEA; KQAVEEAA; QAVEEAAK; AVEEAAKE; VEEAAKEK; EEAAKEKP; EAAKEKPK; AAKEKPKT; AKEKPKTT; KEKPKTTA; EKPKTTAD; KPKTTADG; PKTTADGI; KTTADGII; TTADGIIA; TADGIIAI; ADGIIAIV; DGIIAIVK; GIIAIVKV; IIAIVKVM; IAIVKVMK; AIVKVMKA; IVKVMKAK; VKVMKAKV; KVMKAKVE; VMKAKVEN; MKAKVENI; KAKVENIK; AKVENIKE; KVENIKEK; VENIKEKQ; ENIKEKQT; NIKEKQTK; IKEKQTKN; KEKQTKNQ; EKQTKNQK; <br><br> 9 mers: MIKYNKIIL; IKYNKIILT; KYNKIILTL; YNKIILTLT; NKIILTLTL; KIILTLTLL; IILTLTLLA; ILTLTLLAS; LTLTLLASL; TLTLLASLL; LTLLASLLA; TLLASLLAA; LLASLLAAC; LASLLAACS; ASLLAACSL; SLLAACSLT; LLAACSLTG; LAACSLTGK; AACSLTGKA; ACSLTGKAR; CSLTGKARL; SLTGKARLE; LTGKARLES; TGKARLESS; GKARLESSV; KARLESSVK; ARLESSVKD; RLESSVKDI; LESSVKDIT; ESSVKDITN; SSVKDITNE; SVKDITNEI; VKDITNEIE; KDITNEIEK; DITNEIEKA; ITNEIEKAI; TNEIEKAIK; NEIEKAIKE; EIEKAIKEA; IEKAIKEAE; |

EKAIKEAED; KAIKEAEDA; AIKEAEDAG; IKEAEDAGV; KEAEDAGVK;
EAEDAGVKT; AEDAGVKTD; EDAGVKTDA; DAGVKTDAF; AGVKTDAFT;
GVKTDAFTE; VKTDAFTET; KTDAFTETQ; TDAFTETQT; DAFTETQTG;
AFTETQTGG; FTETQTGGK; TETQTGGKV; ETQTGGKVG; TQTGGKVGG;
QTGGKVGGS; TGGKVGGSQ; GGKVGGSQI; GKVGGSQIR; KVGGSQIRA;
VGGSQIRAA; GGSQIRAAK; GSQIRAAKI; SQIRAAKIR; QIRAAKIRV;
IRAAKIRVA; RAAKIRVAD; AAKIRVADL; AKIRVADLT; KIRVADLTI;
IRVADLTIK; RVADLTIKF; VADLTIKFL; ADLTIKFLE; DLTIKFLEA;
LTIKFLEAT; TIKFLEATE; IKFLEATEE; KFLEATEEE; FLEATEEET;
LEATEEETI; EATEEETIT; ATEEETITF; TEEETITFK; EEETITFKE;
EETITFKEN; ETITFKENG; TITFKENGA; ITFKENGAG; TFKENGAGE;
FKENGAGEE; KENGAGEED; ENGAGEEDF; NGAGEEDFS; GAGEEDFSG;
AGEEDFSGI; GEEDFSGIY; EEDFSGIYD; EDFSGIYDL; DFSGIYDLI;
FSGIYDLIL; SGIYDLILN; GIYDLILNA; IYDLILNAA; YDLILNAAK;
DLILNAAKA; LILNAAKAV; ILNAAKAVE; LNAAKAVEK; NAAKAVEKI;
AAKAVEKIG; AKAVEKIGM; KAVEKIGMQ; AVEKIGMQG; VEKIGMQGM;
EKIGMQGMK; KIGMQGMKQ; IGMQGMKQA; GMQGMKQAV; MQGMKQAVE;
QGMKQAVEE; GMKQAVEEA; MKQAVEEAA; KQAVEEAAK; QAVEEAAKE;
AVEEAAKEK; VEEAAKEKP; EEAAKEKPK; EAAKEKPKT; AAKEKPKTT;
AKEKPKTTA; KEKPKTTAD; EKPKTTADG; KPKTTADGI; PKTTADGII;
KTTADGIIA; TTADGIIAI; TADGIIAIV; ADGIIAIVK; DGIIAIVKV;
GIIAIVKVM; IIAIVKVMK; IAIVKVMKA; AIVKVMKAK; IVKVMKAKV;
VKVMKAKVE; KVMKAKVEN; VMKAKVENI; MKAKVENIK; KAKVENIKE;
AKVENIKEK; KVENIKEKQ; VENIKEKQT; ENIKEKQTK; NIKEKQTKN;
IKEKQTKNQ; KEKQTKNQK;

10 mers:
MIKYNKIILT; IKYNKIILTL; KYNKIILTLT; YNKIILTLTL; NKIILTLTLL;
KIILTLTLLA; IILTLTLLAS; ILTLTLLASL; LTLTLLASLL; TLTLLASLLA;
LTLLASLLAA; TLLASLLAAC; LLASLLAACS; LASLLAACSL; ASLLAACSLT;
SLLAACSLTG; LLAACSLTGK; LAACSLTGKA; AACSLTGKAR; ACSLTGKARL;
CSLTGKARLE; SLTGKARLES; LTGKARLESS; TGKARLESSV; GKARLESSVK;
KARLESSVKD; ARLESSVKDI; RLESSVKDIT; LESSVKDITN; ESSVKDITNE;
SSVKDITNEI; SVKDITNEIE; VKDITNEIEK; KDITNEIEKA; DITNEIEKAI;
ITNEIEKAIK; TNEIEKAIKE; NEIEKAIKEA; EIEKAIKEAE; IEKAIKEAED;
EKAIKEAEDA; KAIKEAEDAG; AIKEAEDAGV; IKEAEDAGVK; KEAEDAGVKT;
EAEDAGVKTD; AEDAGVKTDA; EDAGVKTDAF; DAGVKTDAFT; AGVKTDAFTE;
GVKTDAFTET; VKTDAFTETQ; KTDAFTETQT; TDAFTETQTG; DAFTETQTGG;
AFTETQTGGK; FTETQTGGKV; TETQTGGKVG; ETQTGGKVGG; TQTGGKVGGS;
QTGGKVGGSQ; TGGKVGGSQI; GGKVGGSQIR; GKVGGSQIRA; KVGGSQIRAA;
VGGSQIRAAK; GGSQIRAAKI; GSQIRAAKIR; SQIRAAKIRV; QIRAAKIRVA;
IRAAKIRVAD; RAAKIRVADL; AAKIRVADLT; AKIRVADLTI; KIRVADLTIK;
IRVADLTIKF; RVADLTIKFL; VADLTIKFLE; ADLTIKFLEA; DLTIKFLEAT;
LTIKFLEATE; TIKFLEATEE; IKFLEATEEE; KFLEATEEET; FLEATEEETI;
LEATEEETIT; EATEEETITF; ATEEETITFK; TEEETITFKE; EEETITFKEN;
EETITFKENG; ETITFKENGA; TITFKENGAG; ITFKENGAGE; TFKENGAGEE;
FKENGAGEED; KENGAGEEDF; ENGAGEEDFS; NGAGEEDFSG; GAGEEDFSGI;
AGEEDFSGIY; GEEDFSGIYD; EEDFSGIYDL; EDFSGIYDLI; DFSGIYDLIL;
FSGIYDLILN; SGIYDLILNA; GIYDLILNAA; IYDLILNAAK; YDLILNAAKA;
DLILNAAKAV; LILNAAKAVE; ILNAAKAVEK; LNAAKAVEKI; NAAKAVEKIG;
AAKAVEKIGM; AKAVEKIGMQ; KAVEKIGMQG; AVEKIGMQGM; VEKIGMQGMK;
EKIGMQGMKQ; KIGMQGMKQA; IGMQGMKQAV; GMQGMKQAVE; MQGMKQAVEE;
QGMKQAVEEA; GMKQAVEEAA; MKQAVEEAAK; KQAVEEAAKE; QAVEEAAKEK;
AVEEAAKEKP; VEEAAKEKPK; EEAAKEKPKT; EAAKEKPKTT; AAKEKPKTTA;
AKEKPKTTAD; KEKPKTTADG; EKPKTTADGI; KPKTTADGII; PKTTADGIIA;
KTTADGIIAI; TTADGIIAIV; TADGIIAIVK; ADGIIAIVKV; DGIIAIVKVM;
GIIAIVKVMK; IIAIVKVMKA; IAIVKVMKAK; AIVKVMKAKV; IVKVMKAKVE;

615

| | |
|---|---|
| | VKVMKAKVEN; KVMKAKVENI; VMKAKVENIK; MKAKVENIKE; KAKVENIKEK; AKVENIKEKQ; KVENIKEKQT; VENIKEKQTK; ENIKEKQTKN; NIKEKQTKNQ; IKEKQTKNQK;<br><br>11 mers:<br>MIKYNKIILTL; IKYNKIILTLT; KYNKIILTLTL; YNKIILTLTLL; NKIILTLTLLA; KIILTLTLLAS; IILTLTLLASL; ILTLTLLASLL; LTLTLLASLLA; TLTLLASLLAA; LTLLASLLAAC; TLLASLLAACS; LLASLLAACSL; LASLLAACSLT; ASLLAACSLTG; SLLAACSLTGK; LLAACSLTGKA; LAACSLTGKAR; AACSLTGKARL; ACSLTGKARLE; CSLTGKARLES; SLTGKARLESS; LTGKARLESSV; TGKARLESSVK; GKARLESSVKD; KARLESSVKDI; ARLESSVKDIT; RLESSVKDITN; LESSVKDITNE; ESSVKDITNEI; SSVKDITNEIE; SVKDITNEIEK; VKDITNEIEKA; KDITNEIEKAI; DITNEIEKAIK; ITNEIEKAIKE; TNEIEKAIKEA; NEIEKAIKEAE; EIEKAIKEAED; IEKAIKEAEDA; EKAIKEAEDAG; KAIKEAEDAGV; AIKEAEDAGVK; IKEAEDAGVKT; KEAEDAGVKTD; EAEDAGVKTDA; AEDAGVKTDAF; EDAGVKTDAFT; DAGVKTDAFTE; AGVKTDAFTET; GVKTDAFTETQ; VKTDAFTETQT; KTDAFTETQTG; TDAFTETQTGG; DAFTETQTGGK; AFTETQTGGKV; FTETQTGGKVG; TETQTGGKVGG; ETQTGGKVGGS; TQTGGKVGGSQ; QTGGKVGGSQI; TGGKVGGSQIR; GGKVGGSQIRA; GKVGGSQIRAA; KVGGSQIRAAK; VGGSQIRAAKI; GGSQIRAAKIR; GSQIRAAKIRV; SQIRAAKIRVA; QIRAAKIRVAD; IRAAKIRVADL; RAAKIRVADLT; AAKIRVADLTI; AKIRVADLTIK; KIRVADLTIKF; IRVADLTIKFL; RVADLTIKFLE; VADLTIKFLEA; ADLTIKFLEAT; DLTIKFLEATE; LTIKFLEATEE; TIKFLEATEEE; IKFLEATEEET; KFLEATEEETI; FLEATEEETIT; LEATEEETITF; EATEEETITFK; ATEEETITFKE; TEEETITFKEN; EEETITFKENG; EETITFKENGA; ETITFKENGAG; TITFKENGAGE; ITFKENGAGEE; TFKENGAGEED; FKENGAGEEDF; KENGAGEEDFS; ENGAGEEDFSG; NGAGEEDFSGI; GAGEEDFSGIY; AGEEDFSGIYD; GEEDFSGIYDL; EEDFSGIYDLI; EDFSGIYDLIL; DFSGIYDLILN; FSGIYDLILNA; SGIYDLILNAA; GIYDLILNAAK; IYDLILNAAKA; YDLILNAAKAV; DLILNAAKAVE; LILNAAKAVEK; ILNAAKAVEKI; LNAAKAVEKIG; NAAKAVEKIGM; AAKAVEKIGMQ; AKAVEKIGMQG; KAVEKIGMQGM; AVEKIGMQGMK; VEKIGMQGMKQ; EKIGMQGMKQA; KIGMQGMKQAV; IGMQGMKQAVE; GMQGMKQAVEE; MQGMKQAVEEA; QGMKQAVEEAA; GMKQAVEEAAK; MKQAVEEAAKE; KQAVEEAAKEK; QAVEEAAKEKP; AVEEAAKEKPK; VEEAAKEKPKT; EEAAKEKPKTT; EAAKEKPKTTA; AAKEKPKTTAD; AKEKPKTTADG; KEKPKTTADGI; EKPKTTADGII; KPKTTADGIIA; PKTTADGIIAI; KTTADGIIAIV; TTADGIIAIVK; TADGIIAIVKV; ADGIIAIVKVM; DGIIAIVKVMK; GIIAIVKVMKA; IIAIVKVMKAK; IAIVKVMKAKV; AIVKVMKAKVE; IVKVMKAKVEN; VKVMKAKVENI; KVMKAKVENIK; VMKAKVENIKE; MKAKVENIKEK; KAKVENIKEKQ; AKVENIKEKQT; KVENIKEKQTK; VENIKEKQTKN; ENIKEKQTKNQ; NIKEKQTKNQK; |
| AAC70057.1<br>Decorin binding protein A; DbpA [Borrelia garinii] | SEQ ID NO 6127-6836/<br>8 mers:<br>MIKYNKIL; IKYNKILL; KYNKILLK; YNKILLKL; NKILLKLS; KILLKLSL; ILLKLSLI; LLKLSLIV; LKLSLIVS; KLSLIVSL; LSLIVSLL; SLIVSLLV; LIVSLLVA; IVSLLVAC; VSLLVACG; SLLVACGL; LLVACGLT; LVACGLTG; VACGLTGE; ACGLTGET; CGLTGETK; GLTGETKI; LTGETKIR; TGETKIRL; GETKIRLE; ETKIRLES; TKIRLESS; KIRLESSA; IRLESSAQ; RLESSAQE; LESSAQEI; ESSAQEIK; SSAQEIKD; SAQEIKDE; AQEIKDEI; QEIKDEIN; EIKDEINK; IKDEINKI; KDEINKIK; DEINKIKA; EINKIKAN; INKIKANA; NKIKANAK; KIKANAKK; IKANAKKE; KANAKKEG; ANAKKEGV; NAKKEGVK; AKKEGVKF; KKEGVKFE; KEGVKFEA; EGVKFEAF; GVKFEAFT; VKFEAFTN; |

```
KFEAFTNT;  FEAFTNTQ;  EAFTNTQT;  AFTNTQTG;  FTNTQTGS;  TNTQTGSK;
NTQTGSKI;  TQTGSKIS;  QTGSKISE;  TGSKISEK;  GSKISEKP;  SKISEKPE;
KISEKPEF;  ISEKPEFI;  SEKPEFIL;  EKPEFILK;  KPEFILKA;  PEFILKAK;
EFILKAKI;  FILKAKIK;  ILKAKIKA;  LKAKIKAI;  KAKIKAIQ;  AKIKAIQV;
KIKAIQVA;  IKAIQVAE;  KAIQVAER;  AIQVAERF;  IQVAERFV;  QVAERFVK;
VAERFVKA;  AERFVKAI;  ERFVKAIK;  RFVKAIKE;  FVKAIKEE;  VKAIKEEA;
KAIKEEAE;  AIKEEAEK;  IKEEAEKL;  KEEAEKLK;  EEAEKLKK;  EAEKLKKS;
AEKLKKSG;  EKLKKSGS;  KLKKSGSS;  LKKSGSSG;  KKSGSSGA;  KSGSSGAF;
SGSSGAFS;  GSSGAFSA;  SSGAFSAM;  SGAFSAMY;  GAFSAMYD;  AFSAMYDL;
FSAMYDLM;  SAMYDLMI;  AMYDLMID;  MYDLMIDV;  YDLMIDVS;  DLMIDVSK;
LMIDVSKP;  MIDVSKPL;  IDVSKPLE;  DVSKPLEE;  VSKPLEEI;  SKPLEEIG;
KPLEEIGI;  PLEEIGIQ;  LEEIGIQK;  EEIGIQKM;  EIGIQKMT;  IGIQKMTG;
GIQKMTGT;  IQKMTGTV;  QKMTGTVT;  KMTGTVTK;  MTGTVTKE;  TGTVTKEA;
GTVTKEAE;  TVTKEAEK;  VTKEAEKT;  TKEAEKTP;  KEAEKTPP;  EAEKTPPT;
AEKTPPTT;  EKTPPTTA;  KTPPTTAD;  TPPTTADG;  PPTTADGI;  PTTADGII;
TTADGIIA;  TADGIIAI;  ADGIIAIA;  DGIIAIAQ;  GIIAIAQA;  IIAIAQAM;
IAIAQAME;  AIAQAMEE;  IAQAMEEK;  AQAMEEKL;  QAMEEKLN;  AMEEKLNN;
MEEKLNNV;  EEKLNNVN;  EKLNNVNK;  KLNNVNKK;  LNNVNKKQ;  NNVNKKQH;
NVNKKQHD;  VNKKQHDA;  NKKQHDAL;  KKQHDALK;  KQHDALKN;  QHDALKNL;
HDALKNLE;  DALKNLEE;  ALKNLEEK;  LKNLEEKA;  KNLEEKAN;  NLEEKANT;
LEEKANTA;  EEKANTAA;  EKANTAAT;  KANTAATT;  ANTAATTT;

9 mers:
MIKYNKILL;  IKYNKILLK;  KYNKILLKL;  YNKILLKLS;  NKILLKLSL;
KILLKLSLI;  ILLKLSLIV;  LLKLSLIVS;  LKLSLIVSL;  KLSLIVSLL;
LSLIVSLLV;  SLIVSLLVA;  LIVSLLVAC;  IVSLLVACG;  VSLLVACGL;
SLLVACGLT;  LLVACGLTG;  LVACGLTGE;  VACGLTGET;  ACGLTGETK;
CGLTGETKI;  GLTGETKIR;  LTGETKIRL;  TGETKIRLE;  GETKIRLES;
ETKIRLESS;  TKIRLESSA;  KIRLESSAQ;  IRLESSAQE;  RLESSAQEI;
LESSAQEIK;  ESSAQEIKD;  SSAQEIKDE;  SAQEIKDEI;  AQEIKDEIN;
QEIKDEINK;  EIKDEINKI;  IKDEINKIK;  KDEINKIKA;  DEINKIKAN;
EINKIKANA;  INKIKANAK;  NKIKANAKK;  KIKANAKKE;  IKANAKKEG;
KANAKKEGV;  ANAKKEGVK;  NAKKEGVKF;  AKKEGVKFE;  KKEGVKFEA;
KEGVKFEAF;  EGVKFEAFT;  GVKFEAFTN;  VKFEAFTNT;  KFEAFTNTQ;
FEAFTNTQT;  EAFTNTQTG;  AFTNTQTGS;  FTNTQTGSK;  TNTQTGSKI;
NTQTGSKIS;  TQTGSKISE;  QTGSKISEK;  TGSKISEKP;  GSKISEKPE;
SKISEKPEF;  KISEKPEFI;  ISEKPEFIL;  SEKPEFILK;  EKPEFILKA;
KPEFILKAK;  PEFILKAKI;  EFILKAKIK;  FILKAKIKA;  ILKAKIKAI;
LKAKIKAIQ;  KAKIKAIQV;  AKIKAIQVA;  KIKAIQVAE;  IKAIQVAER;
KAIQVAERF;  AIQVAERFV;  IQVAERFVK;  QVAERFVKA;  VAERFVKAI;
AERFVKAIK;  ERFVKAIKE;  RFVKAIKEE;  FVKAIKEEA;  VKAIKEEAE;
KAIKEEAEK;  AIKEEAEKL;  IKEEAEKLK;  KEEAEKLKK;  EEAEKLKKS;
EAEKLKKSG;  AEKLKKSGS;  EKLKKSGSS;  KLKKSGSSG;  LKKSGSSGA;
KKSGSSGAF;  KSGSSGAFS;  SGSSGAFSA;  GSSGAFSAM;  SSGAFSAMY;
SGAFSAMYD;  GAFSAMYDL;  AFSAMYDLM;  FSAMYDLMI;  SAMYDLMID;
AMYDLMIDV;  MYDLMIDVS;  YDLMIDVSK;  DLMIDVSKP;  LMIDVSKPL;
MIDVSKPLE;  IDVSKPLEE;  DVSKPLEEI;  VSKPLEEIG;  SKPLEEIGI;
KPLEEIGIQ;  PLEEIGIQK;  LEEIGIQKM;  EEIGIQKMT;  EIGIQKMTG;
IGIQKMTGT;  GIQKMTGTV;  IQKMTGTVT;  QKMTGTVTK;  KMTGTVTKE;
MTGTVTKEA;  TGTVTKEAE;  GTVTKEAEK;  TVTKEAEKT;  VTKEAEKTP;
TKEAEKTPP;  KEAEKTPPT;  EAEKTPPTT;  AEKTPPTTA;  EKTPPTTAD;
KTPPTTADG;  TPPTTADGI;  PPTTADGII;  PTTADGIIA;  TTADGIIAI;
TADGIIAIA;  ADGIIAIAQ;  DGIIAIAQA;  GIIAIAQAM;  IIAIAQAME;
IAIAQAMEE;  AIAQAMEEK;  IAQAMEEKL;  AQAMEEKLN;  QAMEEKLNN;
AMEEKLNNV;  MEEKLNNVN;  EEKLNNVNK;  EKLNNVNKK;  KLNNVNKKQ;
LNNVNKKQH;  NNVNKKQHD;  NVNKKQHDA;  VNKKQHDAL;  NKKQHDALK;
KKQHDALKN;  KQHDALKNL;  QHDALKNLE;  HDALKNLEE;  DALKNLEEK;
```

| | ALKNLEEKA; LKNLEEKAN; KNLEEKANT; NLEEKANTA; LEEKANTAA; EEKANTAAT; EKANTAATT; KANTAATTT;<br><br>10 mers:<br>MIKYNKILLK; IKYNKILLKL; KYNKILLKLS; YNKILLKLSL; NKILLKLSLI;<br>KILLKLSLIV; ILLKLSLIVS; LLKLSLIVSL; LKLSLIVSLL; KLSLIVSLLV;<br>LSLIVSLLVA; SLIVSLLVAC; LIVSLLVACG; IVSLLVACGL; VSLLVACGLT;<br>SLLVACGLTG; LLVACGLTGE; LVACGLTGET; VACGLTGETK; ACGLTGETKI;<br>CGLTGETKIR; GLTGETKIRL; LTGETKIRLE; TGETKIRLES; GETKIRLESS;<br>ETKIRLESSA; TKIRLESSAQ; KIRLESSAQE; IRLESSAQEI; RLESSAQEIK;<br>LESSAQEIKD; ESSAQEIKDE; SSAQEIKDEI; SAQEIKDEIN; AQEIKDEINK;<br>QEIKDEINKI; EIKDEINKIK; IKDEINKIKA; KDEINKIKAN; DEINKIKANA;<br>EINKIKANAK; INKIKANAKK; NKIKANAKKE; KIKANAKKEG; IKANAKKEGV;<br>KANAKKEGVK; ANAKKEGVKF; NAKKEGVKFE; AKKEGVKFEA; KKEGVKFEAF;<br>KEGVKFEAFT; EGVKFEAFTN; GVKFEAFTNT; VKFEAFTNTQ; KFEAFTNTQT;<br>FEAFTNTQTG; EAFTNTQTGS; AFTNTQTGSK; FTNTQTGSKI; TNTQTGSKIS;<br>NTQTGSKISE; TQTGSKISEK; QTGSKISEKP; TGSKISEKPE; GSKISEKPEF;<br>SKISEKPEFI; KISEKPEFIL; ISEKPEFILK; SEKPEFILKA; EKPEFILKAK;<br>KPEFILKAKI; PEFILKAKIK; EFILKAKIKA; FILKAKIKAI; ILKAKIKAIQ;<br>LKAKIKAIQV; KAKIKAIQVA; AKIKAIQVAE; KIKAIQVAER; IKAIQVAERF;<br>KAIQVAERFV; AIQVAERFVK; IQVAERFVKA; QVAERFVKAI; VAERFVKAIK;<br>AERFVKAIKE; ERFVKAIKEE; RFVKAIKEEA; FVKAIKEEAE; VKAIKEEAEK;<br>KAIKEEAEKL; AIKEEAEKLK; IKEEAEKLKK; KEEAEKLKKS; EEAEKLKKSG;<br>EAEKLKKSGS; AEKLKKSGSS; EKLKKSGSSG; KLKKSGSSGA; LKKSGSSGAF;<br>KKSGSSGAFS; KSGSSGAFSA; SGSSGAFSAM; GSSGAFSAMY; SSGAFSAMYD;<br>SGAFSAMYDL; GAFSAMYDLM; AFSAMYDLMI; FSAMYDLMID; SAMYDLMIDV;<br>AMYDLMIDVS; MYDLMIDVSK; YDLMIDVSKP; DLMIDVSKPL; LMIDVSKPLE;<br>MIDVSKPLEE; IDVSKPLEEI; DVSKPLEEIG; VSKPLEEIGI; SKPLEEIGIQ;<br>KPLEEIGIQK; PLEEIGIQKM; LEEIGIQKMT; EEIGIQKMTG; EIGIQKMTGT;<br>IGIQKMTGTV; GIQKMTGTVT; IQKMTGTVTK; QKMTGTVTKE; KMTGTVTKEA;<br>MTGTVTKEAE; TGTVTKEAEK; GTVTKEAEKT; TVTKEAEKTP; VTKEAEKTPP;<br>TKEAEKTPPT; KEAEKTPPTT; EAEKTPPTTA; AEKTPPTTAD; EKTPPTTADG;<br>KTPPTTADGI; TPPTTADGII; PPTTADGIIA; PTTADGIIAI; TTADGIIAIA;<br>TADGIIAIAQ; ADGIIAIAQA; DGIIAIAQAM; GIIAIAQAME; IIAIAQAMEE;<br>IAIAQAMEEK; AIAQAMEEKL; IAQAMEEKLN; AQAMEEKLNN; QAMEEKLNNV;<br>AMEEKLNNVN; MEEKLNNVNK; EEKLNNVNKK; EKLNNVNKKQ; KLNNVNKKQH;<br>LNNVNKKQHD; NNVNKKQHDA; NVNKKQHDAL; VNKKQHDALK; NKKQHDALKN;<br>KKQHDALKNL; KQHDALKNLE; QHDALKNLEE; HDALKNLEEK; DALKNLEEKA;<br>ALKNLEEKAN; LKNLEEKANT; KNLEEKANTA; NLEEKANTAA; LEEKANTAAT;<br>EEKANTAATT; EKANTAATTT;<br><br>11 mers:<br>MIKYNKILLKL; IKYNKILLKLS; KYNKILLKLSL; YNKILLKLSLI;<br>NKILLKLSLIV; KILLKLSLIVS; ILLKLSLIVSL; LLKLSLIVSLL;<br>LKLSLIVSLLV; KLSLIVSLLVA; LSLIVSLLVAC; SLIVSLLVACG;<br>LIVSLLVACGL; IVSLLVACGLT; VSLLVACGLTG; SLLVACGLTGE;<br>LLVACGLTGET; LVACGLTGETK; VACGLTGETKI; ACGLTGETKIR;<br>CGLTGETKIRL; GLTGETKIRLE; LTGETKIRLES; TGETKIRLESS;<br>GETKIRLESSA; ETKIRLESSAQ; TKIRLESSAQE; KIRLESSAQEI;<br>IRLESSAQEIK; RLESSAQEIKD; LESSAQEIKDE; ESSAQEIKDEI;<br>SSAQEIKDEIN; SAQEIKDEINK; AQEIKDEINKI; QEIKDEINKIK;<br>EIKDEINKIKA; IKDEINKIKAN; KDEINKIKANA; DEINKIKANAK;<br>EINKIKANAKK; INKIKANAKKE; NKIKANAKKEG; KIKANAKKEGV;<br>IKANAKKEGVK; KANAKKEGVKF; ANAKKEGVKFE; NAKKEGVKFEA;<br>AKKEGVKFEAF; KKEGVKFEAFT; KEGVKFEAFTN; EGVKFEAFTNT;<br>GVKFEAFTNTQ; VKFEAFTNTQT; KFEAFTNTQTG; FEAFTNTQTGS;<br>EAFTNTQTGSK; AFTNTQTGSKI; FTNTQTGSKIS; TNTQTGSKISE; |

| | |
|---|---|
| | NTQTGSKISEK; TQTGSKISEKP; QTGSKISEKPE; TGSKISEKPEF; GSKISEKPEFI; SKISEKPEFIL; KISEKPEFILK; ISEKPEFILKA; SEKPEFILKAK; EKPEFILKAKI; KPEFILKAKIK; PEFILKAKIKA; EFILKAKIKAI; FILKAKIKAIQ; ILKAKIKAIQV; LKAKIKAIQVA; KAKIKAIQVAE; AKIKAIQVAER; KIKAIQVAERF; IKAIQVAERFV; KAIQVAERFVK; AIQVAERFVKA; IQVAERFVKAI; QVAERFVKAIK; VAERFVKAIKE; AERFVKAIKEE; ERFVKAIKEEA; RFVKAIKEEAE; FVKAIKEEAEK; VKAIKEEAEKL; KAIKEEAEKLK; AIKEEAEKLKK; IKEEAEKLKKS; KEEAEKLKKSG; EEAEKLKKSGS; EAEKLKKSGSS; AEKLKKSGSSG; EKLKKSGSSGA; KLKKSGSSGAF; LKKSGSSGAFS; KKSGSSGAFSA; KSGSSGAFSAM; SGSSGAFSAMY; GSSGAFSAMYD; SSGAFSAMYDL; SGAFSAMYDLM; GAFSAMYDLMI; AFSAMYDLMID; FSAMYDLMIDV; SAMYDLMIDVS; AMYDLMIDVSK; MYDLMIDVSKP; YDLMIDVSKPL; DLMIDVSKPLE; LMIDVSKPLEE; MIDVSKPLEEI; IDVSKPLEEIG; DVSKPLEEIGI; VSKPLEEIGIQ; SKPLEEIGIQK; KPLEEIGIQKM; PLEEIGIQKMT; LEEIGIQKMTG; EEIGIQKMTGT; EIGIQKMTGTV; IGIQKMTGTVT; GIQKMTGTVTK; IQKMTGTVTKE; QKMTGTVTKEA; KMTGTVTKEAE; MTGTVTKEAEK; TGTVTKEAEKT; GTVTKEAEKTP; TVTKEAEKTPP; VTKEAEKTPPT; TKEAEKTPPTT; KEAEKTPPTTA; EAEKTPPTTAD; AEKTPPTTADG; EKTPPTTADGI; KTPPTTADGII; TPPTTADGIIA; PPTTADGIIAI; PTTADGIIAIA; TTADGIIAIAQ; TADGIIAIAQA; ADGIIAIAQAM; DGIIAIAQAME; GIIAIAQAMEE; IIAIAQAMEEK; IAIAQAMEEKL; AIAQAMEEKLN; IAQAMEEKLNN; AQAMEEKLNNV; QAMEEKLNNVN; AMEEKLNNVNK; MEEKLNNVNKK; EEKLNNVNKKQ; EKLNNVNKKQH; KLNNVNKKQHD; LNNVNKKQHDA; NNVNKKQHDAL; NVNKKQHDALK; VNKKQHDALKN; NKKQHDALKNL; KKQHDALKNLE; KQHDALKNLEE; QHDALKNLEEK; HDALKNLEEKA; DALKNLEEKAN; ALKNLEEKANT; LKNLEEKANTA; KNLEEKANTAA; NLEEKANTAAT; LEEKANTAATT; EEKANTAATTT; |
| AAC70021.1 Decorin binding protein B; DbpB [Borrelia burgdorferi] | SEQ ID NO 6837-7522/ 8 mers: SIVIALFF; IVIALFFK; VIALFFKL; IALFFKLL; ALFFKLLV; LFFKLLVA; FFKLLVAC; FKLLVACS; KLLVACSI; LLVACSIG; LVACSIGL; VACSIGLV; ACSIGLVE; CSIGLVER; SIGLVERT; IGLVERTN; GLVERTNA; LVERTNAA; VERTNAAL; ERTNAALE; RTNAALES; TNAALESS; NAALESSS; AALESSSK; ALESSSKD; LESSSKDL; ESSSKDLK; SSSKDLKN; SSKDLKNK; SKDLKNKI; KDLKNKIL; DLKNKILK; LKNKILKI; KNKILKIK; NKILKIKK; KILKIKKE; ILKIKKEA; LKIKKEAT; KIKKEATG; IKKEATGK; KKEATGKG; KEATGKGV; EATGKGVL; ATGKGVLF; TGKGVLFE; GKGVLFEA; KGVLFEAF; GVLFEAFT; VLFEAFTG; LFEAFTGL; FEAFTGLK; EAFTGLKT; AFTGLKTG; FTGLKTGS; TGLKTGSK; GLKTGSKV; LKTGSKVT; KTGSKVTS; TGSKVTSG; GSKVTSGG; SKVTSGGL; KVTSGGLA; VTSGGLAL; TSGGLALR; SGGLALRE; GGLALREA; GLALREAK; LALREAKV; ALREAKVQ; LREAKVQA; REAKVQAI; EAKVQAIV; AKVQAIVE; KVQAIVET; VQAIVETG; QAIVETGK; AIVETGKF; IVETGKFL; VETGKFLK; ETGKFLKI; TGKFLKII; GKFLKIIE; KFLKIIEE; FLKIIEEE; LKIIEEEA; KIIEEEAL; IIEEEALK; IEEEALKL; EEEALKLK; EEALKLKE; EALKLKET; ALKLKETG; LKLKETGN; KLKETGNS; LKETGNSG; KETGNSGQ; ETGNSGQF; TGNSGQFL; GNSGQFLA; NSGQFLAM; SGQFLAMF; GQFLAMFD; QFLAMFDL; FLAMFDLM; LAMFDLML; AMFDLMLE; MFDLMLEV; FDLMLEVV; DLMLEVVE; LMLEVVES; MLEVVESL; LEVVESLE; EVVESLED; VVESLEDV; VESLEDVG; ESLEDVGI; SLEDVGII; LEDVGIIG; EDVGIIGL; DVGIIGLK; VGIIGLKA; GIIGLKAR; IIGLKARV; IGLKARVL; GLKARVLE; LKARVLEE; KARVLEES; ARVLEESK; RVLEESKN; VLEESKNN; LEESKNNP; EESKNNPI; ESKNNPIN; SKNNPINT; KNNPINTA; NNPINTAE; NPINTAER; PINTAERL; INTAERLL; NTAERLLA; TAERLLAA; AERLLAAK; ERLLAAKA; RLLAAKAQ; LLAAKAQI; LAAKAQIE; AAKAQIEN; AKAQIENQ; KAQIENQL; AQIENQLK; |

QIENQLKV; IENQLKVV; ENQLKVVK; NQLKVVKE; QLKVVKEK; LKVVKEKQ;
KVVKEKQN; VVKEKQNI; VKEKQNIE; KEKQNIEN; EKQNIENG; KQNIENGG;
QNIENGGE; NIENGGEK; IENGGEKK; ENGGEKKN; NGGEKKNN; GGEKKNNK;
GEKKNNKS; EKKNNKSK; KKNNKSKK; KNNKSKKK; NNKSKKKK;

9 mers:
SIVIALFFK; IVIALFFKL; VIALFFKLL; IALFFKLLV; ALFFKLLVA;
LFFKLLVAC; FFKLLVACS; FKLLVACSI; KLLVACSIG; LLVACSIGL;
LVACSIGLV; VACSIGLVE; ACSIGLVER; CSIGLVERT; SIGLVERTN;
IGLVERTNA; GLVERTNAA; LVERTNAAL; VERTNAALE; ERTNAALES;
RTNAALESS; TNAALESSS; NAALESSSK; AALESSSKD; ALESSSKDL;
LESSSKDLK; ESSSKDLKN; SSSKDLKNK; SSKDLKNKI; SKDLKNKIL;
KDLKNKILK; DLKNKILKI; LKNKILKIK; KNKILKIKK; NKILKIKKE;
KILKIKKEA; ILKIKKEAT; LKIKKEATG; KIKKEATGK; IKKEATGKG;
KKEATGKGV; KEATGKGVL; EATGKGVLF; ATGKGVLFE; TGKGVLFEA;
GKGVLFEAF; KGVLFEAFT; GVLFEAFTG; VLFEAFTGL; LFEAFTGLK;
FEAFTGLKT; EAFTGLKTG; AFTGLKTGS; FTGLKTGSK; TGLKTGSKV;
GLKTGSKVT; LKTGSKVTS; KTGSKVTSG; TGSKVTSGG; GSKVTSGGL;
SKVTSGGLA; KVTSGGLAL; VTSGGLALR; TSGGLALRE; SGGLALREA;
GGLALREAK; GLALREAKV; LALREAKVQ; ALREAKVQA; LREAKVQAI;
REAKVQAIV; EAKVQAIVE; AKVQAIVET; KVQAIVETG; VQAIVETGK;
QAIVETGKF; AIVETGKFL; IVETGKFLK; VETGKFLKI; ETGKFLKII;
TGKFLKIIE; GKFLKIIEE; KFLKIIEEE; FLKIIEEEA; LKIIEEEAL;
KIIEEEALK; IIEEEALKL; IEEEALKLK; EEEALKLKE; EEALKLKET;
EALKLKETG; ALKLKETGN; LKLKETGNS; KLKETGNSG; LKETGNSGQ;
KETGNSGQF; ETGNSGQFL; TGNSGQFLA; GNSGQFLAM; NSGQFLAMF;
SGQFLAMFD; GQFLAMFDL; QFLAMFDLM; FLAMFDLML; LAMFDLMLE;
AMFDLMLEV; MFDLMLEVV; FDLMLEVVE; DLMLEVVES; LMLEVVESL;
MLEVVESLE; LEVVESLED; EVVESLEDV; VVESLEDVG; VESLEDVGI;
ESLEDVGII; SLEDVGIIG; LEDVGIIGL; EDVGIIGLK; DVGIIGLKA;
VGIIGLKAR; GIIGLKARV; IIGLKARVL; IGLKARVLE; GLKARVLEE;
LKARVLEES; KARVLEESK; ARVLEESKN; RVLEESKNN; VLEESKNNP;
LEESKNNPI; EESKNNPIN; ESKNNPINT; SKNNPINTA; KNNPINTAE;
NNPINTAER; NPINTAERL; PINTAERLL; INTAERLLA; NTAERLLAA;
TAERLLAAK; AERLLAAKA; ERLLAAKAQ; RLLAAKAQI; LLAAKAQIE;
LAAKAQIEN; AAKAQIENQ; AKAQIENQL; KAQIENQLK; AQIENQLKV;
QIENQLKVV; IENQLKVVK; ENQLKVVKE; NQLKVVKEK; QLKVVKEKQ;
LKVVKEKQN; KVVKEKQNI; VVKEKQNIE; VKEKQNIEN; KEKQNIENG;
EKQNIENGG; KQNIENGGE; QNIENGGEK; NIENGGEKK; IENGGEKKN;
ENGGEKKNN; NGGEKKNNK; GGEKKNNKS; GEKKNNKSK; EKKNNKSKK;
KKNNKSKKK; KNNKSKKKK;

10 mers:
SIVIALFFKL; IVIALFFKLL; VIALFFKLLV; IALFFKLLVA; ALFFKLLVAC;
LFFKLLVACS; FFKLLVACSI; FKLLVACSIG; KLLVACSIGL; LLVACSIGLV;
LVACSIGLVE; VACSIGLVER; ACSIGLVERT; CSIGLVERTN; SIGLVERTNA;
IGLVERTNAA; GLVERTNAAL; LVERTNAALE; VERTNAALES; ERTNAALESS;
RTNAALESSS; TNAALESSSK; NAALESSSKD; AALESSSKDL; ALESSSKDLK;
LESSSKDLKN; ESSSKDLKNK; SSSKDLKNKI; SSKDLKNKIL; SKDLKNKILK;
KDLKNKILKI; DLKNKILKIK; LKNKILKIKK; KNKILKIKKE; NKILKIKKEA;
KILKIKKEAT; ILKIKKEATG; LKIKKEATGK; KIKKEATGKG; IKKEATGKGV;
KKEATGKGVL; KEATGKGVLF; EATGKGVLFE; ATGKGVLFEA; TGKGVLFEAF;
GKGVLFEAFT; KGVLFEAFTG; GVLFEAFTGL; VLFEAFTGLK; LFEAFTGLKT;
FEAFTGLKTG; EAFTGLKTGS; AFTGLKTGSK; FTGLKTGSKV; TGLKTGSKVT;
GLKTGSKVTS; LKTGSKVTSG; KTGSKVTSGG; TGSKVTSGGL; GSKVTSGGLA;
SKVTSGGLAL; KVTSGGLALR; VTSGGLALRE; TSGGLALREA; SGGLALREAK;
GGLALREAKV; GLALREAKVQ; LALREAKVQA; ALREAKVQAI; LREAKVQAIV;

620

REAKVQAIVE; EAKVQAIVET; AKVQAIVETG; KVQAIVETGK; VQAIVETGKF;
QAIVETGKFL; AIVETGKFLK; IVETGKFLKI; VETGKFLKII; ETGKFLKIIE;
TGKFLKIIEE; GKFLKIIEEE; KFLKIIEEEA; FLKIIEEEAL; LKIIEEEALK;
KIIEEEALKL; IIEEEALKLE; IEEEALKLKE; EEEALKLKET; EEALKLKETG;
EALKLKETGN; ALKLKETGNS; LKLKETGNSG; KLKETGNSGQ; LKETGNSGQF;
KETGNSGQFL; ETGNSGQFLA; TGNSGQFLAM; GNSGQFLAMF; NSGQFLAMFD;
SGQFLAMFDL; GQFLAMFDLM; QFLAMFDLML; FLAMFDLMLE; LAMFDLMLEV;
AMFDLMLEVV; MFDLMLEVVE; FDLMLEVVES; DLMLEVVESL; LMLEVVESLE;
MLEVVESLED; LEVVESLEDV; EVVESLEDVG; VVESLEDVGI; VESLEDVGII;
ESLEDVGIIG; SLEDVGIIGL; LEDVGIIGLK; EDVGIIGLKA; DVGIIGLKAR;
VGIIGLKARV; GIIGLKARVL; IIGLKARVLE; IGLKARVLEE; GLKARVLEES;
LKARVLEESK; KARVLEESKN; ARVLEESKNN; RVLEESKNNP; VLEESKNNPI;
LEESKNNPIN; EESKNNPINT; ESKNNPINTA; SKNNPINTAE; KNNPINTAER;
NNPINTAERL; NPINTAERLL; PINTAERLLA; INTAERLLAA; NTAERLLAAK;
TAERLLAAKA; AERLLAAKAQ; ERLLAAKAQI; RLLAAKAQIE; LLAAKAQIEN;
LAAKAQIENQ; AAKAQIENQL; AKAQIENQLK; KAQIENQLKV; AQIENQLKVV;
QIENQLKVVK; IENQLKVVKE; ENQLKVVKEK; NQLKVVKEKQ; QLKVVKEKQN;
LKVVKEKQNI; KVVKEKQNIE; VVKEKQNIEN; VKEKQNIENG; KEKQNIENGG;
EKQNIENGGE; KQNIENGGEK; QNIENGGEKK; NIENGGEKKN; IENGGEKKNN;
ENGGEKKNNK; NGGEKKNNKS; GGEKKNNKSK; GEKKNNKSKK; EKKNNKSKKK;
KKNNKSKKKK;

11 mers:
SIVIALFFKLL; IVIALFFKLLV; VIALFFKLLVA; IALFFKLLVAC;
ALFFKLLVACS; LFFKLLVACSI; FFKLLVACSIG; FKLLVACSIGL;
KLLVACSIGLV; LLVACSIGLVE; LVACSIGLVER; VACSIGLVERT;
ACSIGLVERTN; CSIGLVERTNA; SIGLVERTNAA; IGLVERTNAAL;
GLVERTNAALE; LVERTNAALES; VERTNAALESS; ERTNAALESSS;
RTNAALESSSK; TNAALESSSKD; NAALESSSKDL; AALESSSKDLK;
ALESSSKDLKN; LESSSKDLKNK; ESSSKDLKNKI; SSSKDLKNKIL;
SSKDLKNKILK; SKDLKNKILKI; KDLKNKILKIK; DLKNKILKIKK;
LKNKILKIKKE; KNKILKIKKEA; NKILKIKKEAT; KILKIKKEATG;
ILKIKKEATGK; LKIKKEATGKG; KIKKEATGKGV; IKKEATGKGVL;
KKEATGKGVLF; KEATGKGVLFE; EATGKGVLFEA; ATGKGVLFEAF;
TGKGVLFEAFT; GKGVLFEAFTG; KGVLFEAFTGL; GVLFEAFTGLK;
VLFEAFTGLKT; LFEAFTGLKTG; FEAFTGLKTGS; EAFTGLKTGSK;
AFTGLKTGSKV; FTGLKTGSKVT; TGLKTGSKVTS; GLKTGSKVTSG;
LKTGSKVTSGG; KTGSKVTSGGL; TGSKVTSGGLA; GSKVTSGGLAL;
SKVTSGGLALR; KVTSGGLALRE; VTSGGLALREA; TSGGLALREAK;
SGGLALREAKV; GGLALREAKVQ; GLALREAKVQA; LALREAKVQAI;
ALREAKVQAIV; LREAKVQAIVE; REAKVQAIVET; EAKVQAIVETG;
AKVQAIVETGK; KVQAIVETGKF; VQAIVETGKFL; QAIVETGKFLK;
AIVETGKFLKI; IVETGKFLKII; VETGKFLKIIE; ETGKFLKIIEE;
TGKFLKIIEEE; GKFLKIIEEEA; KFLKIIEEEAL; FLKIIEEEALK;
LKIIEEEALKL; KIIEEEALKLK; IIEEEALKLKE; IEEEALKLKET;
EEEALKLKETG; EEALKLKETGN; EALKLKETGNS; ALKLKETGNSG;
LKLKETGNSGQ; KLKETGNSGQF; LKETGNSGQFL; KETGNSGQFLA;
ETGNSGQFLAM; TGNSGQFLAMF; GNSGQFLAMFD; NSGQFLAMFDL;
SGQFLAMFDLM; GQFLAMFDLML; QFLAMFDLMLE; FLAMFDLMLEV;
LAMFDLMLEVV; AMFDLMLEVVE; MFDLMLEVVES; FDLMLEVVESL;
DLMLEVVESLE; LMLEVVESLED; MLEVVESLEDV; LEVVESLEDVG;
EVVESLEDVGI; VVESLEDVGII; VESLEDVGIIG; ESLEDVGIIGL;
SLEDVGIIGLK; LEDVGIIGLKA; EDVGIIGLKAR; DVGIIGLKARV;
VGIIGLKARVL; GIIGLKARVLE; IIGLKARVLEE; IGLKARVLEES;
GLKARVLEESK; LKARVLEESKN; KARVLEESKNN; ARVLEESKNNP;
RVLEESKNNPI; VLEESKNNPIN; LEESKNNPINT; EESKNNPINTA;
ESKNNPINTAE; SKNNPINTAER; KNNPINTAERL; NNPINTAERLL;

| | |
|---|---|
| | NPINTAERLLA; PINTAERLLAA; INTAERLLAAK; NTAERLLAAKA; TAERLLAAKAQ; AERLLAAKAQI; ERLLAAKAQIE; RLLAAKAQIEN; LLAAKAQIENQ; LAAKAQIENQL; AAKAQIENQLK; AKAQIENQLKV; KAQIENQLKVV; AQIENQLKVVK; QIENQLKVVKE; IENQLKVVKEK; ENQLKVVKEKQ; NQLKVVKEKQN; QLKVVKEKQNI; LKVVKEKQNIE; KVVKEKQNIEN; VVKEKQNIENG; VKEKQNIENGG; KEKQNIENGGE; EKQNIENGGEK; KQNIENGGEKK; QNIENGGEKKN; NIENGGEKKNN; IENGGEKKNNK; ENGGEKKNNKS; NGGEKKNNKSK; GGEKKNNKSKK; GEKKNNKSKKK; EKKNNKSKKKK; |
| NP_212694.1<br>Heat shock protein 90 [Borrelia burgdorferi B31] | SEQ ID NO 7523-10087/<br>8 mers:<br>MILIFYFK; ILIFYFKQ; LIFYFKQI; IFYFKQIA; FYFKQIAL; YFKQIALF; FKQIALFI; KQIALFII; QIALFIIF; IALFIIFR; ALFIIFRL; LFIIFRLC; FIIFRLCY; IIFRLCYI; IFRLCYII; FRLCYIIK; RLCYIIKK; LCYIIKKV; CYIIKKVK; YIIKKVKI; IIKKVKIK; IKKVKIKL; KKVKIKLK; KVKIKLKR; VKIKLKRK; KIKLKRKS; IKLKRKSC; KLKRKSCM; LKRKSCMK; KRKSCMKK; RKSCMKKQ; KSCMKKQF; SCMKKQFD; CMKKQFDT; MKKQFDTE; KKQFDTEV; KQFDTEVN; QFDTEVND; FDTEVNDL; DTEVNDLL; TEVNDLLY; EVNDLLYL; VNDLLYLI; NDLLYLII; DLLYLIIH; LLYLIIHS; LYLIIHSL; YLIIHSLY; LIIHSLYS; IIHSLYSH; IHSLYSHK; HSLYSHKE; SLYSHKEI; LYSHKEIF; YSHKEIFL; SHKEIFLR; HKEIFLRE; KEIFLREL; EIFLRELI; IFLRELIS; FLRELISN; LRELISNA; RELISNAS; ELISNASD; LISNASDA; ISNASDAI; SNASDAID; NASDAIDK; ASDAIDKL; SDAIDKLK; DAIDKLKF; AIDKLKFL; IDKLKFLS; DKLKFLSL; KLKFLSLT; LKFLSLTN; KFLSLTNE; FLSLTNEK; LSLTNEKF; SLTNEKFK; LTNEKFKN; TNEKFKNI; NEKFKNIA; EKFKNIAL; KFKNIALE; FKNIALEP; KNIALEPK; NIALEPKI; IALEPKIE; ALEPKIEI; LEPKIEIS; EPKIEISF; PKIEISFD; KIEISFDD; IEISFDDK; EISFDDKS; ISFDDKSI; SFDDKSIL; FDDKSILI; DDKSILIK; DKSILIKD; KSILIKDN; SILIKDNG; ILIKDNGI; LIKDNGIG; IKDNGIGM; KDNGIGMD; DNGIGMDE; NGIGMDEQ; GIGMDEQD; IGMDEQDL; GMDEQDLT; MDEQDLTN; DEQDLTNH; EQDLTNHL; QDLTNHLG; DLTNHLGV; LTNHLGVI; TNHLGVIA; NHLGVIAK; HLGVIAKS; LGVIAKSG; GVIAKSGT; VIAKSGTK; IAKSGTKE; AKSGTKEF; KSGTKEFI; SGTKEFIN; GTKEFINN; TKEFINNL; KEFINNLK; EFINNLKQ; FINNLKQD; INNLKQDE; NNLKQDEK; NLKQDEKK; LKQDEKKS; KQDEKKSA; QDEKKSAS; DEKKSASL; EKKSASLI; KKSASLIG; KSASLIGQ; SASLIGQF; ASLIGQFG; SLIGQFGV; LIGQFGVG; IGQFGVGF; GQFGVGFY; QFGVGFYS; FGVGFYSA; GVGFYSAF; VGFYSAFI; GFYSAFIV; FYSAFIVS; YSAFIVSE; SAFIVSEK; AFIVSEKV; FIVSEKVE; IVSEKVEV; VSEKVEVT; SEKVEVTS; EKVEVTSK; KVEVTSKK; VEVTSKKA; EVTSKKAL; VTSKKALE; TSKKALES; SKKALESD; KKALESDA; KALESDAY; ALESDAYI; LESDAYIW; ESDAYIWS; SDAYIWSS; DAYIWSSD; AYIWSSDG; YIWSSDGK; IWSSDGKT; WSSDGKTG; SSDGKTGY; SDGKTGYE; DGKTGYEI; GKTGYEIE; KTGYEIEK; TGYEIEKA; GYEIEKAK; YEIEKAKK; EIEKAKKE; IEKAKKEE; EKAKKEES; KAKKEESG; AKKEESGT; KKEESGTE; KEESGTEI; EESGTEIK; ESGTEIKL; SGTEIKLY; GTEIKLYL; TEIKLYLN; EIKLYLNK; IKLYLNKE; KLYLNKEG; LYLNKEGL; YLNKEGLE; LNKEGLEY; NKEGLEYA; KEGLEYAN; EGLEYANK; GLEYANKW; LEYANKWK; EYANKWKI; YANKWKIQ; ANKWKIQE; NKWKIQEI; KWKIQEII; WKIQEIIK; KIQEIIKK; IQEIIKKY; QEIIKKYS; EIIKKYSN; IIKKYSNH; IKKYSNHI; KKYSNHIN; KYSNHINY; YSNHINYP; SNHINYPI; NHINYPIY; HINYPIYI; INYPIYIK; NYPIYIKY; YPIYIKYS; PIYIKYSE; IYIKYSEP; YIKYSEPI; IKYSEPIM; KYSEPIMK; YSEPIMKD; SEPIMKDG; EPIMKDGK; PIMKDGKQ; IMKDGKQE; MKDGKQEG; KDGKQEGI; DGKQEGIE; GKQEGIEE; KQEGIEEK; QEGIEEKE; EGIEEKEE; GIEEKEEK; IEEKEEKL; EEKEEKLN; EKEEKLNE; KEEKLNET; EEKLNETT; EKLNETTA; KLNETTAL; LNETTALW; NETTALWT; ETTALWTK; TTALWTKN; TALWTKNK; ALWTKNKS; LWTKNKSE; WTKNKSEI; TKNKSEIK; KNKSEIKA; NKSEIKAE; KSEIKAEE; SEIKAEEY; |

EIKAEEYN; IKAEEYNE; KAEEYNEF; AEEYNEFY; EEYNEFYK; EYNEFYKN;
YNEFYKNT; NEFYKNTT; EFYKNTTF; FYKNTTFD; YKNTTFDY; KNTTFDYE;
NTTFDYEN; TTFDYENP; TFDYENPL; FDYENPLM; DYENPLMH; YENPLMHI;
ENPLMHIH; NPLMHIHT; PLMHIHTK; LMHIHTKA; MHIHTKAE; HIHTKAEG;
IHTKAEGN; HTKAEGNL; TKAEGNLE; KAEGNLEY; AEGNLEYT; EGNLEYTN;
GNLEYTNL; NLEYTNLF; LEYTNLFY; EYTNLFYV; YTNLFYVP; TNLFYVPS;
NLFYVPSK; LFYVPSKA; FYVPSKAP; YVPSKAPY; VPSKAPYD; PSKAPYDL;
SKAPYDLY; KAPYDLYY; APYDLYYP; PYDLYYPN; YDLYYPNT; DLYYPNTK;
LYYPNTKP; YYPNTKPG; YPNTKPGV; PNTKPGVK; NTKPGVKL; TKPGVKLF;
KPGVKLFI; PGVKLFIN; GVKLFINR; VKLFINRI; KLFINRIF; LFINRIFI;
FINRIFIT; INRIFITD; NRIFITDS; RIFITDSE; IFITDSEG; FITDSEGS;
ITDSEGSL; TDSEGSLL; DSEGSLLP; SEGSLLPN; EGSLLPNY; GSLLPNYL;
SLLPNYLR; LLPNYLRF; LPNYLRFI; PNYLRFIK; NYLRFIKG; YLRFIKGI;
LRFIKGII; RFIKGIID; FIKGIIDC; IKGIIDCQ; KGIIDCQD; GIIDCQDL;
IIDCQDLP; IDCQDLPL; DCQDLPLN; CQDLPLNV; QDLPLNVS; DLPLNVSR;
LPLNVSRE; PLNVSREI; LNVSREIL; NVSREILQ; VSREILQQ; SREILQQN;
REILQQNK; EILQQNKI; ILQQNKIL; LQQNKILS; QQNKILSK; QNKILSKI;
NKILSKIK; KILSKIKS; ILSKIKSS; LSKIKSSS; SKIKSSSV; KIKSSSVK;
IKSSSVKK; KSSSVKKI; SSSVKKIL; SSVKKILS; SVKKILSE; VKKILSEL;
KKILSELE; KILSELEK; ILSELEKL; LSELEKLS; SELEKLSK; ELEKLSKK;
LEKLSKKN; EKLSKKNP; KLSKKNPE; LSKKNPEK; SKKNPEKF; KKNPEKFS;
KNPEKFSE; NPEKFSEF; PEKFSEFS; EKFSEFSK; KFSEFSKE; FSEFSKEF;
SEFSKEFG; EFSKEFGR; FSKEFGRC; SKEFGRCI; KEFGRCIK; EFGRCIKE;
FGRCIKEG; GRCIKEGV; RCIKEGVY; CIKEGVYS; IKEGVYSD; KEGVYSDF;
EGVYSDFE; GVYSDFEN; VYSDFENR; YSDFENRE; SDFENREK; DFENREKL;
FENREKLI; ENREKLIS; NREKLISL; REKLISLI; EKLISLIR; KLISLIRF;
LISLIRFK; ISLIRFKS; SLIRFKSS; LIRFKSSS; IRFKSSSV; RFKSSSVD;
FKSSSVDG; KSSSVDGF; SSSVDGFV; SSVDGFVS; SVDGFVSF; VDGFVSFK;
DGFVSFKE; GFVSFKEY; FVSFKEYK; VSFKEYKE; SFKEYKER; FKEYKERM;
KEYKERMN; EYKERMNE; YKERMNES; KERMNESQ; ERMNESQK; RMNESQKS;
MNESQKSI; NESQKSIY; ESQKSIYY; SQKSIYYI; QKSIYYIT; KSIYYITG;
SIYYITGG; IYYITGGK; YYITGGKE; YITGGKEN; ITGGKENI; TGGKENIL;
GGKENILK; GKENILKE; KENILKEN; ENILKENP; NILKENPI; ILKENPIV;
LKENPIVA; KENPIVAA; ENPIVAAY; NPIVAAYK; PIVAAYKE; IVAAYKEK;
VAAYKEKG; AAYKEKGF; AYKEKGFE; YKEKGFEI; KEKGFEIL; EKGFEILI;
KGFEILIM; GFEILIMD; FEILIMDD; EILIMDDE; ILIMDDEL; LIMDDELD;
IMDDELDE; MDDELDEA; DDELDEAI; DELDEAIL; ELDEAILN; LDEAILNL;
DEAILNLI; EAILNLIP; AILNLIPE; ILNLIPEY; LNLIPEYE; NLIPEYEG;
LIPEYEGL; IPEYEGLK; PEYEGLKL; EYEGLKLK; YEGLKLKA; EGLKLKAI;
GLKLKAIN; LKLKAINK; KLKAINKN; LKAINKNE; KAINKNET; AINKNETS;
INKNETSN; NKNETSNE; KNETSNEL; NETSNELK; ETSNELKD; TSNELKDE;
SNELKDEN; NELKDENF; ELKDENFK; LKDENFKK; KDENFKKI; DENFKKIE;
ENFKKIEE; NFKKIEEE; FKKIEEEF; KKIEEEFK; KIEEEFKD; IEEEFKDT;
EEEFKDTL; EEFKDTLT; EFKDTLTK; FKDTLTKV; KDTLTKVK; DTLTKVKE;
TLTKVKEI; LTKVKEIL; TKVKEILK; KVKEILKD; VKEILKDH; KEILKDHI;
EILKDHIK; ILKDHIKE; LKDHIKEV; KDHIKEVN; DHIKEVNL; HIKEVNLS;
IKEVNLSA; KEVNLSAT; EVNLSATL; VNLSATLI; NLSATLIK; LSATLIKE;
SATLIKEP; ATLIKEPS; TLIKEPSA; LIKEPSAI; IKEPSAII; KEPSAIII;
EPSAIIID; PSAIIIDS; SAIIIDSN; AIIIDSND; IIIDSNDP; IIDSNDPT;
IDSNDPTY; DSNDPTYQ; SNDPTYQM; NDPTYQMQ; DPTYQMQK; PTYQMQKI;
TYQMQKIM; YQMQKIML; QMQKIMLS; MQKIMLSM; QKIMLSMG; KIMLSMGQ;
IMLSMGQE; MLSMGQEV; LSMGQEVK; SMGQEVKE; MGQEVKEI; GQEVKEIK;
QEVKEIKP; EVKEIKPI; VKEIKPIL; KEIKPILE; EIKPILEL; IKPILELN;
KPILELNP; PILELNPN; ILELNPNN; LELNPNNK; ELNPNNKI; LNPNNKIV;
NPNNKIVQ; PNNKIVQN; NNKIVQNL; NKIVQNLK; KIVQNLKN; IVQNLKNL;
VQNLKNLE; QNLKNLEP; NLKNLEPE; LKNLEPEK; KNLEPEKL; NLEPEKLE;
LEPEKLEK; EPEKLEKI; PEKLEKIS; EKLEKISI; KLEKISIL; LEKISILL;

EKISILLF; KISILLFE; ISILLFEE; SILLFEEA; ILLFEEAM; LLFEEAML;
LFEEAMLT; FEEAMLTS; EEAMLTSG; EAMLTSGM; AMLTSGMP; MLTSGMPS;
LTSGMPSK; TSGMPSKN; SGMPSKNP; GMPSKNPG; MPSKNPGK; PSKNPGKF;
SKNPGKFI; KNPGKFIN; NPGKFINI; PGKFINII; GKFINIIN; KFINIINE;
FINIINEF; INIINEFI; NIINEFIE; IINEFIEK; INEFIEKD; NEFIEKDF;
EFIEKDFL;

9 mers:
MILIFYFKQ; ILIFYFKQI; LIFYFKQIA; IFYFKQIAL; FYFKQIALF;
YFKQIALFI; FKQIALFII; KQIALFIIF; QIALFIIFR; IALFIIFRL;
ALFIIFRLC; LFIIFRLCY; FIIFRLCYI; IIFRLCYII; IFRLCYIIK;
FRLCYIIKK; RLCYIIKKV; LCYIIKKVK; CYIIKKVKI; YIIKKVKIK;
IIKKVKIKL; IKKVKIKLK; KKVKIKLKR; KVKIKLKRK; VKIKLKRKS;
KIKLKRKSC; IKLKRKSCM; KLKRKSCMK; LKRKSCMKK; KRKSCMKKQ;
RKSCMKKQF; KSCMKKQFD; SCMKKQFDT; CMKKQFDTE; MKKQFDTEV;
KKQFDTEVN; KQFDTEVND; QFDTEVNDL; FDTEVNDLL; DTEVNDLLY;
TEVNDLLYL; EVNDLLYLI; VNDLLYLII; NDLLYLIIH; DLLYLIIHS;
LLYLIIHSL; LYLIIHSLY; YLIIHSLYS; LIIHSLYSH; IIHSLYSHK;
IHSLYSHKE; HSLYSHKEI; SLYSHKEIF; LYSHKEIFL; YSHKEIFLR;
SHKEIFLRE; HKEIFLREL; KEIFLRELI; EIFLRELIS; IFLRELISN;
FLRELISNA; LRELISNAS; RELISNASD; ELISNASDA; LISNASDAI;
ISNASDAID; SNASDAIDK; NASDAIDKL; ASDAIDKLK; SDAIDKLKF;
DAIDKLKFL; AIDKLKFLS; IDKLKFLSL; DKLKFLSLT; KLKFLSLTN;
LKFLSLTNE; KFLSLTNEK; FLSLTNEKF; LSLTNEKFK; SLTNEKFKN;
LTNEKFKNI; TNEKFKNIA; NEKFKNIAL; EKFKNIALE; KFKNIALEP;
FKNIALEPK; KNIALEPKI; NIALEPKIE; IALEPKIEI; ALEPKIEIS;
LEPKIEISF; EPKIEISFD; PKIEISFDD; KIEISFDDK; IEISFDDKS;
EISFDDKSI; ISFDDKSIL; SFDDKSILI; FDDKSILIK; DDKSILIKD;
DKSILIKDN; KSILIKDNG; SILIKDNGI; ILIKDNGIG; LIKDNGIGM;
IKDNGIGMD; KDNGIGMDE; DNGIGMDEQ; NGIGMDEQD; GIGMDEQDL;
IGMDEQDLT; GMDEQDLTN; MDEQDLTNH; DEQDLTNHL; EQDLTNHLG;
QDLTNHLGV; DLTNHLGVI; LTNHLGVIA; TNHLGVIAK; NHLGVIAKS;
HLGVIAKSG; LGVIAKSGT; GVIAKSGTK; VIAKSGTKE; IAKSGTKEF;
AKSGTKEFI; KSGTKEFIN; SGTKEFINN; GTKEFINNL; TKEFINNLK;
KEFINNLKQ; EFINNLKQD; FINNLKQDE; INNLKQDEK; NNLKQDEKK;
NLKQDEKKS; LKQDEKKSA; KQDEKKSAS; QDEKKSASL; DEKKSASLI;
EKKSASLIG; KKSASLIGQ; KSASLIGQF; SASLIGQFG; ASLIGQFGV;
SLIGQFGVG; LIGQFGVGF; IGQFGVGFY; GQFGVGFYS; QFGVGFYSA;
FGVGFYSAF; GVGFYSAFI; VGFYSAFIV; GFYSAFIVS; FYSAFIVSE;
YSAFIVSEK; SAFIVSEKV; AFIVSEKVE; FIVSEKVEV; IVSEKVEVT;
VSEKVEVTS; SEKVEVTSK; EKVEVTSKK; KVEVTSKKA; VEVTSKKAL;
EVTSKKALE; VTSKKALES; TSKKALESD; SKKALESDA; KKALESDAY;
KALESDAYI; ALESDAYIW; LESDAYIWS; ESDAYIWSS; SDAYIWSSD;
DAYIWSSDG; AYIWSSDGK; YIWSSDGKT; IWSSDGKTG; WSSDGKTGY;
SSDGKTGYE; SDGKTGYEI; DGKTGYEIE; GKTGYEIEK; KTGYEIEKA;
TGYEIEKAK; GYEIEKAKK; YEIEKAKKE; EIEKAKKEE; IEKAKKEES;
EKAKKEESG; KAKKEESGT; AKKEESGTE; KKEESGTEI; KEESGTEIK;
EESGTEIKL; ESGTEIKLY; SGTEIKLYL; GTEIKLYLN; TEIKLYLNK;
EIKLYLNKE; IKLYLNKEG; KLYLNKEGL; LYLNKEGLE; YLNKEGLEY;
LNKEGLEYA; NKEGLEYAN; KEGLEYANK; EGLEYANKW; GLEYANKWK;
LEYANKWKI; EYANKWKIQ; YANKWKIQE; ANKWKIQEI; NKWKIQEII;
KWKIQEIIK; WKIQEIIKK; KIQEIIKKY; IQEIIKKYS; QEIIKKYSN;
EIIKKYSNH; IIKKYSNHI; IKKYSNHIN; KKYSNHINY; KYSNHINYP;
YSNHINYPI; SNHINYPIY; NHINYPIYI; HINYPIYIK; INYPIYIKY;
NYPIYIKYS; YPIYIKYSE; PIYIKYSEP; IYIKYSEPI; YIKYSEPIM;
IKYSEPIMK; KYSEPIMKD; YSEPIMKDG; SEPIMKDGK; EPIMKDGKQ;
PIMKDGKQE; IMKDGKQEG; MKDGKQEGI; KDGKQEGIE; DGKQEGIEE;

624

GKQEGIEEK; KQEGIEEKE; QEGIEEKEE; EGIEEKEEK; GIEEKEEKL;
IEEKEEKLN; EEKEEKLNE; EKEEKLNET; KEEKLNETT; EEKLNETTA;
EKLNETTAL; KLNETTALW; LNETTALWT; NETTALWTK; ETTALWTKN;
TTALWTKNK; TALWTKNKS; ALWTKNKSE; LWTKNKSEI; WTKNKSEIK;
TKNKSEIKA; KNKSEIKAE; NKSEIKAEE; KSEIKAEEY; SEIKAEEYN;
EIKAEEYNE; IKAEEYNEF; KAEEYNEFY; AEEYNEFYK; EEYNEFYKN;
EYNEFYKNT; YNEFYKNTT; NEFYKNTTF; EFYKNTTFD; FYKNTTFDY;
YKNTTFDYE; KNTTFDYEN; NTTFDYENP; TTFDYENPL; TFDYENPLM;
FDYENPLMH; DYENPLMHI; YENPLMHIH; ENPLMHIHT; NPLMHIHTK;
PLMHIHTKA; LMHIHTKAE; MHIHTKAEG; HIHTKAEGN; IHTKAEGNL;
HTKAEGNLE; TKAEGNLEY; KAEGNLEYT; AEGNLEYTN; EGNLEYTNL;
GNLEYTNLF; NLEYTNLFY; LEYTNLFYV; EYTNLFYVP; YTNLFYVPS;
TNLFYVPSK; NLFYVPSKA; LFYVPSKAP; FYVPSKAPY; YVPSKAPYD;
VPSKAPYDL; PSKAPYDLY; SKAPYDLYY; KAPYDLYYP; APYDLYYPN;
PYDLYYPNT; YDLYYPNTK; DLYYPNTKP; LYYPNTKPG; YYPNTKPGV;
YPNTKPGVK; PNTKPGVKL; NTKPGVKLF; TKPGVKLFI; KPGVKLFIN;
PGVKLFINR; GVKLFINRI; VKLFINRIF; KLFINRIFI; LFINRIFIT;
FINRIFITD; INRIFITDS; NRIFITDSE; RIFITDSEG; IFITDSEGS;
FITDSEGSL; ITDSEGSLL; TDSEGSLLP; DSEGSLLPN; SEGSLLPNY;
EGSLLPNYL; GSLLPNYLR; SLLPNYLRF; LLPNYLRFI; LPNYLRFIK;
PNYLRFIKG; NYLRFIKGI; YLRFIKGII; LRFIKGIID; RFIKGIIDC;
FIKGIIDCQ; IKGIIDCQD; KGIIDCQDL; GIIDCQDLP; IIDCQDLPL;
IDCQDLPLN; DCQDLPLNV; CQDLPLNVS; QDLPLNVSR; DLPLNVSRE;
LPLNVSREI; PLNVSREIL; LNVSREILQ; NVSREILQQ; VSREILQQN;
SREILQQNK; REILQQNKI; EILQQNKIL; ILQQNKILS; LQQNKILSK;
QQNKILSKI; QNKILSKIK; NKILSKIKS; KILSKIKSS; ILSKIKSSS;
LSKIKSSSV; SKIKSSSVK; KIKSSSVKK; IKSSSVKKI; KSSSVKKIL;
SSSVKKILS; SSVKKILSE; SVKKILSEL; VKKILSELE; KKILSELEK;
KILSELEKL; ILSELEKLS; LSELEKLSK; SELEKLSKK; ELEKLSKKN;
LEKLSKKNP; EKLSKKNPE; KLSKKNPEK; LSKKNPEKF; SKKNPEKFS;
KKNPEKFSE; KNPEKFSEF; NPEKFSEFS; PEKFSEFSK; EKFSEFSKE;
KFSEFSKEF; FSEFSKEFG; SEFSKEFGR; EFSKEFGRC; FSKEFGRCI;
SKEFGRCIK; KEFGRCIKE; EFGRCIKEG; FGRCIKEGV; GRCIKEGVY;
RCIKEGVYS; CIKEGVYSD; IKEGVYSDF; KEGVYSDFE; EGVYSDFEN;
GVYSDFENR; VYSDFENRE; YSDFENREK; SDFENREKL; DFENREKLI;
FENREKLIS; ENREKLISL; NREKLISLI; REKLISLIR; EKLISLIRF;
KLISLIRFK; LISLIRFKS; ISLIRFKSS; SLIRFKSSS; LIRFKSSSV;
IRFKSSSVD; RFKSSSVDG; FKSSSVDGF; KSSSVDGFV; SSSVDGFVS;
SSVDGFVSF; SVDGFVSFK; VDGFVSFKE; DGFVSFKEY; GFVSFKEYK;
FVSFKEYKE; VSFKEYKER; SFKEYKERM; FKEYKERMN; KEYKERMNE;
EYKERMNES; YKERMNESQ; KERMNESQK; ERMNESQKS; RMNESQKSI;
MNESQKSIY; NESQKSIYY; ESQKSIYYI; SQKSIYYIT; QKSIYYITG;
KSIYYITGG; SIYYITGGK; IYYITGGKE; YYITGGKEN; YITGGKENI;
ITGGKENIL; TGGKENILK; GGKENILKE; GKENILKEN; KENILKENP;
ENILKENPI; NILKENPIV; ILKENPIVA; LKENPIVAA; KENPIVAAY;
ENPIVAAYK; NPIVAAYKE; PIVAAYKEK; IVAAYKEKG; VAAYKEKGF;
AAYKEKGFE; AYKEKGFEI; YKEKGFEIL; KEKGFEILI; EKGFEILIM;
KGFEILIMD; GFEILIMDD; FEILIMDDE; EILIMDDEL; ILIMDDELD;
LIMDDELDE; IMDDELDEA; MDDELDEAI; DDELDEAIL; DELDEAILN;
ELDEAILNL; LDEAILNLI; DEAILNLIP; EAILNLIPE; AILNLIPEY;
ILNLIPEYE; LNLIPEYEG; NLIPEYEGL; LIPEYEGLK; IPEYEGLKL;
PEYEGLKLK; EYEGLKLKA; YEGLKLKAI; EGLKLKAIN; GLKLKAINK;
LKLKAINKN; KLKAINKNE; LKAINKNET; KAINKNETS; AINKNETSN;
INKNETSNE; NKNETSNEL; KNETSNELK; NETSNELKD; ETSNELKDE;
TSNELKDEN; SNELKDENF; NELKDENFK; ELKDENFKK; LKDENFKKI;
KDENFKKIE; DENFKKIEE; ENFKKIEEE; NFKKIEEEF; FKKIEEEFK;
KKIEEEFKD; KIEEEFKDT; IEEEFKDTL; EEEFKDTLT; EEFKDTLTK;

EFKDTLTKV; FKDTLTKVK; KDTLTKVKE; DTLTKVKEI; TLTKVKEIL;
LTKVKEILK; TKVKEILKD; KVKEILKDH; VKEILKDHI; KEILKDHIK;
EILKDHIKE; ILKDHIKEV; LKDHIKEVN; KDHIKEVNL; DHIKEVNLS;
HIKEVNLSA; IKEVNLSAT; KEVNLSATL; EVNLSATLI; VNLSATLIK;
NLSATLIKE; LSATLIKEP; SATLIKEPS; ATLIKEPSA; TLIKEPSAI;
LIKEPSAII; IKEPSAIII; KEPSAIIID; EPSAIIIDS; PSAIIIDSN;
SAIIIDSND; AIIIDSNDP; IIIDSNDPT; IIDSNDPTY; IDSNDPTYQ;
DSNDPTYQM; SNDPTYQMQ; NDPTYQMQK; DPTYQMQKI; PTYQMQKIM;
TYQMQKIML; YQMQKIMLS; QMQKIMLSM; MQKIMLSMG; QKIMLSMGQ;
KIMLSMGQE; IMLSMGQEV; MLSMGQEVK; LSMGQEVKE; SMGQEVKEI;
MGQEVKEIK; GQEVKEIKP; QEVKEIKPI; EVKEIKPIL; VKEIKPILE;
KEIKPILEL; EIKPILELN; IKPILELNP; KPILELNPN; PILELNPNN;
ILELNPNNK; LELNPNNKI; ELNPNNKIV; LNPNNKIVQ; NPNNKIVQN;
PNNKIVQNL; NNKIVQNLK; NKIVQNLKN; KIVQNLKNL; IVQNLKNLE;
VQNLKNLEP; QNLKNLEPE; NLKNLEPEK; LKNLEPEKL; KNLEPEKLE;
NLEPEKLEK; LEPEKLEKI; EPEKLEKIS; PEKLEKISI; EKLEKISIL;
KLEKISILL; LEKISILLF; EKISILLFE; KISILLFEE; ISILLFEEA;
SILLFEEAM; ILLFEEAML; LLFEEAMLT; LFEEAMLTS; FEEAMLTSG;
EEAMLTSGM; EAMLTSGMP; AMLTSGMPS; MLTSGMPSK; LTSGMPSKN;
TSGMPSKNP; SGMPSKNPG; GMPSKNPGK; MPSKNPGKF; PSKNPGKFI;
SKNPGKFIN; KNPGKFINI; NPGKFINII; PGKFINIIN; GKFINIINE;
KFINIINEF; FINIINEFI; INIINEFIE; NIINEFIEK; IINEFIEKD;
INEFIEKDF;

10 mers:
MILIFYFKQI; ILIFYFKQIA; LIFYFKQIAL; IFYFKQIALF; FYFKQIALFI;
YFKQIALFII; FKQIALFIIF; KQIALFIIFR; QIALFIIFRL; IALFIIFRLC;
ALFIIFRLCY; LFIIFRLCYI; FIIFRLCYII; IIFRLCYIIK; IFRLCYIIKK;
FRLCYIIKKV; RLCYIIKKVK; LCYIIKKVKI; CYIIKKVKIK; YIIKKVKIKL;
IIKKVKIKLK; IKKVKIKLKR; KKVKIKLKRK; KVKIKLKRKS; VKIKLKRKSC;
KIKLKRKSCM; IKLKRKSCMK; KLKRKSCMKK; LKRKSCMKKQ; KRKSCMKKQF;
RKSCMKKQFD; KSCMKKQFDT; SCMKKQFDTE; CMKKQFDTEV; MKKQFDTEVN;
KKQFDTEVND; KQFDTEVNDL; QFDTEVNDLL; FDTEVNDLLY; DTEVNDLLYL;
TEVNDLLYLI; EVNDLLYLII; VNDLLYLIIH; NDLLYLIIHS; DLLYLIIHSL;
LLYLIIHSLY; LYLIIHSLYS; YLIIHSLYSH; LIIHSLYSHK; IIHSLYSHKE;
IHSLYSHKEI; HSLYSHKEIF; SLYSHKEIFL; LYSHKEIFLR; YSHKEIFLRE;
SHKEIFLREL; HKEIFLRELI; KEIFLRELIS; EIFLRELISN; IFLRELISNA;
FLRELISNAS; LRELISNASD; RELISNASDA; ELISNASDAI; LISNASDAID;
ISNASDAIDK; SNASDAIDKL; NASDAIDKLK; ASDAIDKLKF; SDAIDKLKFL;
DAIDKLKFLS; AIDKLKFLSL; IDKLKFLSLT; DKLKFLSLTN; KLKFLSLTNE;
LKFLSLTNEK; KFLSLTNEKF; FLSLTNEKFK; LSLTNEKFKN; SLTNEKFKNI;
LTNEKFKNIA; TNEKFKNIAL; NEKFKNIALE; EKFKNIALEP; KFKNIALEPK;
FKNIALEPKI; KNIALEPKIE; NIALEPKIEI; IALEPKIEIS; ALEPKIEISF;
LEPKIEISFD; EPKIEISFDD; PKIEISFDDK; KIEISFDDKS; IEISFDDKSI;
EISFDDKSIL; ISFDDKSILI; SFDDKSILIK; FDDKSILIKD; DDKSILIKDN;
DKSILIKDNG; KSILIKDNGI; SILIKDNGIG; ILIKDNGIGM; LIKDNGIGMD;
IKDNGIGMDE; KDNGIGMDEQ; DNGIGMDEQD; NGIGMDEQDL; GIGMDEQDLT;
IGMDEQDLTN; GMDEQDLTNH; MDEQDLTNHL; DEQDLTNHLG; EQDLTNHLGV;
QDLTNHLGVI; DLTNHLGVIA; LTNHLGVIAK; TNHLGVIAKS; NHLGVIAKSG;
HLGVIAKSGT; LGVIAKSGTK; GVIAKSGTKE; VIAKSGTKEF; IAKSGTKEFI;
AKSGTKEFIN; KSGTKEFINN; SGTKEFINNL; GTKEFINNLK; TKEFINNLKQ;
KEFINNLKQD; EFINNLKQDE; FINNLKQDEK; INNLKQDEKK; NNLKQDEKKS;
NLKQDEKKSA; LKQDEKKSAS; KQDEKKSASL; QDEKKSASLI; DEKKSASLIG;
EKKSASLIGQ; KKSASLIGQF; KSASLIGQFG; SASLIGQFGV; ASLIGQFGVG;
SLIGQFGVGF; LIGQFGVGFY; IGQFGVGFYS; GQFGVGFYSA; QFGVGFYSAF;
FGVGFYSAFI; GVGFYSAFIV; VGFYSAFIVS; GFYSAFIVSE; FYSAFIVSEK;
YSAFIVSEKV; SAFIVSEKVE; AFIVSEKVEV; FIVSEKVEVT; IVSEKVEVTS;

EP 2 254 592 B1

| | |
|---|---|
| | VSEKVEVTSK; SEKVEVTSKK; EKVEVTSKKA; KVEVTSKKAL; VEVTSKKALE;<br>EVTSKKALES; VTSKKALESD; TSKKALESDA; SKKALESDAY; KKALESDAYI;<br>KALESDAYIW; ALESDAYIWS; LESDAYIWSS; ESDAYIWSSD; SDAYIWSSDG;<br>DAYIWSSDGK; AYIWSSDGKT; YIWSSDGKTG; IWSSDGKTGY; WSSDGKTGYE;<br>SSDGKTGYEI; SDGKTGYEIE; DGKTGYEIEK; GKTGYEIEKA; KTGYEIEKAK;<br>TGYEIEKAKK; GYEIEKAKKE; YEIEKAKKEE; EIEKAKKEES; IEKAKKEESG;<br>EKAKKEESGT; KAKKEESGTE; AKKEESGTEI; KKEESGTEIK; KEESGTEIKL;<br>EESGTEIKLY; ESGTEIKLYL; SGTEIKLYLN; GTEIKLYLNK; TEIKLYLNKE;<br>EIKLYLNKEG; IKLYLNKEGL; KLYLNKEGLE; LYLNKEGLEY; YLNKEGLEYA;<br>LNKEGLEYAN; NKEGLEYANK; KEGLEYANKW; EGLEYANKWK; GLEYANKWKI;<br>LEYANKWKIQ; EYANKWKIQE; YANKWKIQEI; ANKWKIQEII; NKWKIQEIIK;<br>KWKIQEIIKK; WKIQEIIKKY; KIQEIIKKYS; IQEIIKKYSN; QEIIKKYSNH;<br>EIIKKYSNHI; IIKKYSNHIN; IKKYSNHINY; KKYSNHINYP; KYSNHINYPI;<br>YSNHINYPIY; SNHINYPIYI; NHINYPIYIK; HINYPIYIKY; INYPIYIKYS;<br>NYPIYIKYSE; YPIYIKYSEP; PIYIKYSEPI; IYIKYSEPIM; YIKYSEPIMK;<br>IKYSEPIMKD; KYSEPIMKDG; YSEPIMKDGK; SEPIMKDGKQ; EPIMKDGKQE;<br>PIMKDGKQEG; IMKDGKQEGI; MKDGKQEGIE; KDGKQEGIEE; DGKQEGIEEK;<br>GKQEGIEEKE; KQEGIEEKEE; QEGIEEKEEK; EGIEEKEEKL; GIEEKEEKLN;<br>IEEKEEKLNE; EEKEEKLNET; EKEEKLNETT; KEEKLNETTA; EEKLNETTAL;<br>EKLNETTALW; KLNETTALWT; LNETTALWTK; NETTALWTKN; ETTALWTKNK;<br>TTALWTKNKS; TALWTKNKSE; ALWTKNKSEI; LWTKNKSEIK; WTKNKSEIKA;<br>TKNKSEIKAE; KNKSEIKAEE; NKSEIKAEEY; KSEIKAEEYN; SEIKAEEYNE;<br>EIKAEEYNEF; IKAEEYNEFY; KAEEYNEFYK; AEEYNEFYKN; EEYNEFYKNT;<br>EYNEFYKNTT; YNEFYKNTTF; NEFYKNTTFD; EFYKNTTFDY; FYKNTTFDYE;<br>YKNTTFDYEN; KNTTFDYENP; NTTFDYENPL; TTFDYENPLM; TFDYENPLMH;<br>FDYENPLMHI; DYENPLMHIH; YENPLMHIHT; ENPLMHIHTK; NPLMHIHTKA;<br>PLMHIHTKAE; LMHIHTKAEG; MHIHTKAEGN; HIHTKAEGNL; IHTKAEGNLE;<br>HTKAEGNLEY; TKAEGNLEYT; KAEGNLEYTN; AEGNLEYTNL; EGNLEYTNLF;<br>GNLEYTNLFY; NLEYTNLFYV; LEYTNLFYVP; EYTNLFYVPS; YTNLFYVPSK;<br>TNLFYVPSKA; NLFYVPSKAP; LFYVPSKAPY; FYVPSKAPYD; YVPSKAPYDL;<br>VPSKAPYDLY; PSKAPYDLYY; SKAPYDLYYP; KAPYDLYYPN; APYDLYYPNT;<br>PYDLYYPNTK; YDLYYPNTKP; DLYYPNTKPG; LYYPNTKPGV; YYPNTKPGVK;<br>YPNTKPGVKL; PNTKPGVKLF; NTKPGVKLFI; TKPGVKLFIN; KPGVKLFINR;<br>PGVKLFINRI; GVKLFINRIF; VKLFINRIFI; KLFINRIFIT; LFINRIFITD;<br>FINRIFITDS; INRIFITDSE; NRIFITDSEG; RIFITDSEGS; IFITDSEGSL;<br>FITDSEGSLL; ITDSEGSLLP; TDSEGSLLPN; DSEGSLLPNY; SEGSLLPNYL;<br>EGSLLPNYLR; GSLLPNYLRF; SLLPNYLRFI; LLPNYLRFIK; LPNYLRFIKG;<br>PNYLRFIKGI; NYLRFIKGII; YLRFIKGIID; LRFIKGIIDC; RFIKGIIDCQ;<br>FIKGIIDCQD; IKGIIDCQDL; KGIIDCQDLP; GIIDCQDLPL; IIDCQDLPLN;<br>IDCQDLPLNV; DCQDLPLNVS; CQDLPLNVSR; QDLPLNVSRE; DLPLNVSREI;<br>LPLNVSREIL; PLNVSREILQ; LNVSREILQQ; NVSREILQQN; VSREILQQNK;<br>SREILQQNKI; REILQQNKIL; EILQQNKILS; ILQQNKILSK; LQQNKILSKI;<br>QQNKILSKIK; QNKILSKIKS; NKILSKIKSS; KILSKIKSSS; ILSKIKSSSV;<br>LSKIKSSSVK; SKIKSSSVKK; KIKSSSVKKI; IKSSSVKKIL; KSSSVKKILS;<br>SSSVKKILSE; SSVKKILSEL; SVKKILSELE; VKKILSELEK; KKILSELEKL;<br>KILSELEKLS; ILSELEKLSK; LSELEKLSKK; SELEKLSKKN; ELEKLSKKNP;<br>LEKLSKKNPE; EKLSKKNPEK; KLSKKNPEKF; LSKKNPEKFS; SKKNPEKFSE;<br>KKNPEKFSEF; KNPEKFSEFS; NPEKFSEFSK; PEKFSEFSKE; EKFSEFSKEF;<br>KFSEFSKEFG; FSEFSKEFGR; SEFSKEFGRC; EFSKEFGRCI; FSKEFGRCIK;<br>SKEFGRCIKE; KEFGRCIKEG; EFGRCIKEGV; FGRCIKEGVY; GRCIKEGVYS;<br>RCIKEGVYSD; CIKEGVYSDF; IKEGVYSDFE; KEGVYSDFEN; EGVYSDFENR;<br>GVYSDFENRE; VYSDFENREK; YSDFENREKL; SDFENREKLI; DFENREKLIS;<br>FENREKLISL; ENREKLISLI; NREKLISLIR; REKLISLIRF; EKLISLIRFK;<br>KLISLIRFKS; LISLIRFKSS; ISLIRFKSSS; SLIRFKSSSV; LIRFKSSSVD;<br>IRFKSSSVDG; RFKSSSVDGF; FKSSSVDGFV; KSSSVDGFVS; SSSVDGFVSF;<br>SSVDGFVSFK; SVDGFVSFKE; VDGFVSFKEY; DGFVSFKEYK; GFVSFKEYKE;<br>FVSFKEYKER; VSFKEYKERM; SFKEYKERMN; FKEYKERMNE; KEYKERMNES; |

EYKERMNESQ; YKERMNESQK; KERMNESQKS; ERMNESQKSI; RMNESQKSIY;
MNESQKSIYY; NESQKSIYYI; ESQKSIYYIT; SQKSIYYITG; QKSIYYITGG;
KSIYYITGGK; SIYYITGGKE; IYYITGGKEN; YYITGGKENI; YITGGKENIL;
ITGGKENILK; TGGKENILKE; GGKENILKEN; GKENILKENP; KENILKENPI;
ENILKENPIV; NILKENPIVA; ILKENPIVAA; LKENPIVAAY; KENPIVAAYK;
ENPIVAAYKE; NPIVAAYKEK; PIVAAYKEKG; IVAAYKEKGF; VAAYKEKGFE;
AAYKEKGFEI; AYKEKGFEIL; YKEKGFEILI; KEKGFEILIM; EKGFEILIMD;
KGFEILIMDD; GFEILIMDDE; FEILIMDDEL; EILIMDDELD; ILIMDDELDE;
LIMDDELDEA; IMDDELDEAI; MDDELDEAIL; DDELDEAILN; DELDEAILNL;
ELDEAILNLI; LDEAILNLIP; DEAILNLIPE; EAILNLIPEY; AILNLIPEYE;
ILNLIPEYEG; LNLIPEYEGL; NLIPEYEGLK; LIPEYEGLKL; IPEYEGLKLK;
PEYEGLKLKA; EYEGLKLKAI; YEGLKLKAIN; EGLKLKAINK; GLKLKAINKN;
LKLKAINKNE; KLKAINKNET; LKAINKNETS; KAINKNETSN; AINKNETSNE;
INKNETSNEL; NKNETSNELK; KNETSNELKD; NETSNELKDE; ETSNELKDEN;
TSNELKDENF; SNELKDENFK; NELKDENFKK; ELKDENFKKI; LKDENFKKIE;
KDENFKKIEE; DENFKKIEEE; ENFKKIEEEF; NFKKIEEEFK; FKKIEEEFKD;
KKIEEEFKDT; KIEEEFKDTL; IEEEFKDTLT; EEEFKDTLTK; EEFKDTLTKV;
EFKDTLTKVK; FKDTLTKVKE; KDTLTKVKEI; DTLTKVKEIL; TLTKVKEILK;
LTKVKEILKD; TKVKEILKDH; KVKEILKDHI; VKEILKDHIK; KEILKDHIKE;
EILKDHIKEV; ILKDHIKEVN; LKDHIKEVNL; KDHIKEVNLS; DHIKEVNLSA;
HIKEVNLSAT; IKEVNLSATL; KEVNLSATLI; EVNLSATLIK; VNLSATLIKE;
NLSATLIKEP; LSATLIKEPS; SATLIKEPSA; ATLIKEPSAI; TLIKEPSAII;
LIKEPSAIII; IKEPSAIIID; KEPSAIIIDS; EPSAIIIDSN; PSAIIIDSND;
SAIIIDSNDP; AIIIDSNDPT; IIIDSNDPTY; IIDSNDPTYQ; IDSNDPTYQM;
DSNDPTYQMQ; SNDPTYQMQK; NDPTYQMQKI; DPTYQMQKIM; PTYQMQKIML;
TYQMQKIMLS; YQMQKIMLSM; QMQKIMLSMG; MQKIMLSMGQ; QKIMLSMGQE;
KIMLSMGQEV; IMLSMGQEVK; MLSMGQEVKE; LSMGQEVKEI; SMGQEVKEIK;
MGQEVKEIKP; GQEVKEIKPI; QEVKEIKPIL; EVKEIKPILE; VKEIKPILEL;
KEIKPILELN; EIKPILELNP; IKPILELNPN; KPILELNPNN; PILELNPNNK;
ILELNPNNKI; LELNPNNKIV; ELNPNNKIVQ; LNPNNKIVQN; NPNNKIVQNL;
PNNKIVQNLK; NNKIVQNLKN; NKIVQNLKNL; KIVQNLKNLE; IVQNLKNLEP;
VQNLKNLEPE; QNLKNLEPEK; NLKNLEPEKL; LKNLEPEKLE; KNLEPEKLEK;
NLEPEKLEKI; LEPEKLEKIS; EPEKLEKISI; PEKLEKISIL; EKLEKISILL;
KLEKISILLF; LEKISILLFE; EKISILLFEE; KISILLFEEA; ISILLFEEAM;
SILLFEEAML; ILLFEEAMLT; LLFEEAMLTS; LFEEAMLTSG; FEEAMLTSGM;
EEAMLTSGMP; EAMLTSGMPS; AMLTSGMPSK; MLTSGMPSKN; LTSGMPSKNP;
TSGMPSKNPG; SGMPSKNPGK; GMPSKNPGKF; MPSKNPGKFI; PSKNPGKFIN;
SKNPGKFINI; KNPGKFINII; NPGKFINIIN; PGKFINIINE; GKFINIINEF;
KFINIINEFI; FINIINEFIE; INIINEFIEK; NIINEFIEKD; IINEFIEKDF;
INEFIEKDFL

11 mers:
MILIFYFKQIA; ILIFYFKQIAL; LIFYFKQIALF; IFYFKQIALFI;
FYFKQIALFII; YFKQIALFIIF; FKQIALFIIFR; KQIALFIIFRL;
QIALFIIFRLC; IALFIIFRLCY; ALFIIFRLCYI; LFIIFRLCYII;
FIIFRLCYIIK; IIFRLCYIIKK; IFRLCYIIKKV; FRLCYIIKKVK;
RLCYIIKKVKI; LCYIIKKVKIK; CYIIKKVKIKL; YIIKKVKIKLK;
IIKKVKIKLKR; IKKVKIKLKRR; KKVKIKLKRKS; KVKIKLKRKSC;
VKIKLKRKSCM; KIKLKRKSCMK; IKLKRKSCMKK; KLKRKSCMKKQ;
LKRKSCMKKQF; KRKSCMKKQFD; RKSCMKKQFDT; KSCMKKQFDTE;
SCMKKQFDTEV; CMKKQFDTEVN; MKKQFDTEVND; KKQFDTEVNDL;
KQFDTEVNDLL; QFDTEVNDLLY; FDTEVNDLLYL; DTEVNDLLYLI;
TEVNDLLYLII; EVNDLLYLIIH; VNDLLYLIIHS; NDLLYLIIHSL;
DLLYLIIHSLY; LLYLIIHSLYS; LYLIIHSLYSH; YLIIHSLYSHK;
LIIHSLYSHKE; IIHSLYSHKEI; IHSLYSHKEIF; HSLYSHKEIFL;
SLYSHKEIFLR; LYSHKEIFLRE; YSHKEIFLREL; SHKEIFLRELI;
HKEIFLRELIS; KEIFLRELISN; EIFLRELISNA; IFLRELISNAS;

| | |
|---|---|
| FLRELISNASD; LRELISNASDA; RELISNASDAI; ELISNASDAID; LISNASDAIDK; ISNASDAIDKL; SNASDAIDKLK; NASDAIDKLKF; ASDAIDKLKFL; SDAIDKLKFLS; DAIDKLKFLSL; AIDKLKFLSLT; IDKLKFLSLTN; DKLKFLSLTNE; KLKFLSLTNEK; LKFLSLTNEKF; KFLSLTNEKFK; FLSLTNEKFKN; LSLTNEKFKNI; SLTNEKFKNIA; LTNEKFKNIAL; TNEKFKNIALE; NEKFKNIALEP; EKFKNIALEPK; KFKNIALEPKI; FKNIALEPKIE; KNIALEPKIEI; NIALEPKIEIS; IALEPKIEISF; ALEPKIEISFD; LEPKIEISFDD; EPKIEISFDDK; PKIEISFDDKS; KIEISFDDKSI; IEISFDDKSIL; EISFDDKSILI; ISFDDKSILIK; SFDDKSILIKD; FDDKSILIKDN; DDKSILIKDNG; DKSILIKDNGI; KSILIKDNGIG; SILIKDNGIGM; ILIKDNGIGMD; LIKDNGIGMDE; IKDNGIGMDEQ; KDNGIGMDEQD; DNGIGMDEQDL; NGIGMDEQDLT; GIGMDEQDLTN; IGMDEQDLTNH; GMDEQDLTNHL; MDEQDLTNHLG; DEQDLTNHLGV; EQDLTNHLGVI; QDLTNHLGVIA; DLTNHLGVIAK; LTNHLGVIAKS; TNHLGVIAKSG; NHLGVIAKSGT; HLGVIAKSGTK; LGVIAKSGTKE; GVIAKSGTKEF; VIAKSGTKEFI; IAKSGTKEFIN; AKSGTKEFINN; KSGTKEFINNL; SGTKEFINNLK; GTKEFINNLKQ; TKEFINNLKQD; KEFINNLKQDE; EFINNLKQDEK; FINNLKQDEKK; INNLKQDEKKS; NNLKQDEKKSA; NLKQDEKKSAS; LKQDEKKSASL; KQDEKKSASLI; QDEKKSASLIG; DEKKSASLIGQ; EKKSASLIGQF; KKSASLIGQFG; KSASLIGQFGV; SASLIGQFGVG; ASLIGQFGVGF; SLIGQFGVGFY; LIGQFGVGFYS; IGQFGVGFYSA; GQFGVGFYSAF; QFGVGFYSAFI; FGVGFYSAFIV; GVGFYSAFIVS; VGFYSAFIVSE; GFYSAFIVSEK; FYSAFIVSEKV; YSAFIVSEKVE; SAFIVSEKVEV; AFIVSEKVEVT; FIVSEKVEVTS; IVSEKVEVTSK; VSEKVEVTSKK; SEKVEVTSKKA; EKVEVTSKKAL; KVEVTSKKALE; VEVTSKKALES; EVTSKKALESD; VTSKKALESDA; TSKKALESDAY; SKKALESDAYI; KKALESDAYIW; KALESDAYIWS; ALESDAYIWSS; LESDAYIWSSD; ESDAYIWSSDG; SDAYIWSSDGK; DAYIWSSDGKT; AYIWSSDGKTG; YIWSSDGKTGY; IWSSDGKTGYE; WSSDGKTGYEI; SSDGKTGYEIE; SDGKTGYEIEK; DGKTGYEIEKA; GKTGYEIEKAK; KTGYEIEKAKK; TGYEIEKAKKE; GYEIEKAKKEE; YEIEKAKKEES; EIEKAKKEESG; IEKAKKEESGT; EKAKKEESGTE; KAKKEESGTEI; AKKEESGTEIK; KKEESGTEIKL; KEESGTEIKLY; EESGTEIKLYL; ESGTEIKLYLN; SGTEIKLYLNK; GTEIKLYLNKE; TEIKLYLNKEG; EIKLYLNKEGL; IKLYLNKEGLE; KLYLNKEGLEY; LYLNKEGLEYA; YLNKEGLEYAN; LNKEGLEYANK; NKEGLEYANKW; KEGLEYANKWK; EGLEYANKWKI; GLEYANKWKIQ; LEYANKWKIQE; EYANKWKIQEI; YANKWKIQEII; ANKWKIQEIIK; NKWKIQEIIKK; KWKIQEIIKKY; WKIQEIIKKYS; KIQEIIKKYSN; IQEIIKKYSNH; QEIIKKYSNHI; EIIKKYSNHIN; IIKKYSNHINY; IKKYSNHINYP; KKYSNHINYPI; KYSNHINYPIY; YSNHINYPIYI; SNHINYPIYIK; NHINYPIYIKY; HINYPIYIKYS; INYPIYIKYSE; NYPIYIKYSEP; YPIYIKYSEPI; PIYIKYSEPIM; IYIKYSEPIMK; YIKYSEPIMKD; IKYSEPIMKDG; KYSEPIMKDGK; YSEPIMKDGKQ; SEPIMKDGKQE; EPIMKDGKQEG; PIMKDGKQEGI; IMKDGKQEGIE; MKDGKQEGIEE; KDGKQEGIEEK; DGKQEGIEEKE; GKQEGIEEKEE; KQEGIEEKEEK; QEGIEEKEEKL; EGIEEKEEKLN; GIEEKEEKLNE; IEEKEEKLNET; EEKEEKLNETT; EKEEKLNETTA; KEEKLNETTAL; EEKLNETTALW; EKLNETTALWT; KLNETTALWTK; LNETTALWTKN; NETTALWTKNK; ETTALWTKNKS; TTALWTKNKSE; TALWTKNKSEI; ALWTKNKSEIK; LWTKNKSEIKA; WTKNKSEIKAE; TKNKSEIKAEE; KNKSEIKAEEY; NKSEIKAEEYN; KSEIKAEEYNE; SEIKAEEYNEF; EIKAEEYNEFY; IKAEEYNEFYK; KAEEYNEFYKN; AEEYNEFYKNT; EEYNEFYKNTT; EYNEFYKNTTF; YNEFYKNTTFD; NEFYKNTTFDY; EFYKNTTFDYE; FYKNTTFDYEN; YKNTTFDYENP; KNTTFDYENPL; NTTFDYENPLM; TTFDYENPLMH; TFDYENPLMHI; FDYENPLMHIH; DYENPLMHIHT; YENPLMHIHTK; | |

```
ENPLMHIHTKA;   NPLMHIHTKAE;   PLMHIHTKAEG;   LMHIHTKAEGN;
MHIHTKAEGNL;   HIHTKAEGNLE;   IHTKAEGNLEY;   HTKAEGNLEYT;
TKAEGNLEYTN;   KAEGNLEYTNL;   AEGNLEYTNLF;   EGNLEYTNLFY;
GNLEYTNLFYV;   NLEYTNLFYVP;   LEYTNLFYVPS;   EYTNLFYVPSK;
YTNLFYVPSKA;   TNLFYVPSKAP;   NLFYVPSKAPY;   LFYVPSKAPYD;
FYVPSKAPYDL;   YVPSKAPYDLY;   VPSKAPYDLYY;   PSKAPYDLYYP;
SKAPYDLYYPN;   KAPYDLYYPNT;   APYDLYYPNTK;   PYDLYYPNTKP;
YDLYYPNTKPG;   DLYYPNTKPGV;   LYYPNTKPGVK;   YYPNTKPGVKL;
YPNTKPGVKLF;   PNTKPGVKLFI;   NTKPGVKLFIN;   TKPGVKLFINR;
KPGVKLFINRI;   PGVKLFINRIF;   GVKLFINRIFI;   VKLFINRIFIT;
KLFINRIFITD;   LFINRIFITDS;   FINRIFITDSE;   INRIFITDSEG;
NRIFITDSEGS;   RIFITDSEGSL;   IFITDSEGSLL;   FITDSEGSLLP;
ITDSEGSLLPN;   TDSEGSLLPNY;   DSEGSLLPNYL;   SEGSLLPNYLR;
EGSLLPNYLRF;   GSLLPNYLRFI;   SLLPNYLRFIK;   LLPNYLRFIKG;
LPNYLRFIKGI;   PNYLRFIKGII;   NYLRFIKGIID;   YLRFIKGIIDC;
LRFIKGIIDCQ;   RFIKGIIDCQD;   FIKGIIDCQDL;   IKGIIDCQDLP;
KGIIDCQDLPL;   GIIDCQDLPLN;   IIDCQDLPLNV;   IDCQDLPLNVS;
DCQDLPLNVSR;   CQDLPLNVSRE;   QDLPLNVSREI;   DLPLNVSREIL;
LPLNVSREILQ;   PLNVSREILQQ;   LNVSREILQQN;   NVSREILQQNK;
VSREILQQNKI;   SREILQQNKIL;   REILQQNKILS;   EILQQNKILSK;
ILQQNKILSKI;   LQQNKILSKIK;   QQNKILSKIKS;   QNKILSKIKSS;
NKILSKIKSSS;   KILSKIKSSSV;   ILSKIKSSSVK;   LSKIKSSSVKK;
SKIKSSSVKKI;   KIKSSSVKKIL;   IKSSSVKKILS;   KSSSVKKILSE;
SSSVKKILSEL;   SSVKKILSELE;   SVKKILSELEK;   VKKILSELEKL;
KKILSELEKLS;   KILSELEKLSK;   ILSELEKLSKK;   LSELEKLSKKN;
SELEKLSKKNP;   ELEKLSKKNPE;   LEKLSKKNPEK;   EKLSKKNPEKF;
KLSKKNPEKFS;   LSKKNPEKFSE;   SKKNPEKFSEF;   KKNPEKFSEFS;
KNPEKFSEFSK;   NPEKFSEFSKE;   PEKFSEFSKEF;   EKFSEFSKEFG;
KFSEFSKEFGR;   FSEFSKEFGRC;   SEFSKEFGRCI;   EFSKEFGRCIK;
FSKEFGRCIKE;   SKEFGRCIKEG;   KEFGRCIKEGV;   EFGRCIKEGVY;
FGRCIKEGVYS;   GRCIKEGVYSD;   RCIKEGVYSDF;   CIKEGVYSDFE;
IKEGVYSDFEN;   KEGVYSDFENR;   EGVYSDFENRE;   GVYSDFENREK;
VYSDFENREKL;   YSDFENREKLI;   SDFENREKLIS;   DFENREKLISL;
FENREKLISLI;   ENREKLISLIR;   NREKLISLIRF;   REKLISLIRFK;
EKLISLIRFKS;   KLISLIRFKSS;   LISLIRFKSSS;   ISLIRFKSSSV;
SLIRFKSSSVD;   LIRFKSSSVDG;   IRFKSSSVDGF;   RFKSSSVDGFV;
FKSSSVDGFVS;   KSSSVDGFVSF;   SSSVDGFVSFK;   SSVDGFVSFKE;
SVDGFVSFKEY;   VDGFVSFKEYK;   DGFVSFKEYKE;   GFVSFKEYKER;
FVSFKEYKERM;   VSFKEYKERMN;   SFKEYKERMNE;   FKEYKERMNES;
KEYKERMNESQ;   EYKERMNESQK;   YKERMNESQKS;   KERMNESQKSI;
ERMNESQKSIY;   RMNESQKSIYY;   MNESQKSIYYI;   NESQKSIYYIT;
ESQKSIYYITG;   SQKSIYYITGG;   QKSIYYITGGK;   KSIYYITGGKE;
SIYYITGGKEN;   IYYITGGKENI;   YYITGGKENIL;   YITGGKENILK;
ITGGKENILKE;   TGGKENILKEN;   GGKENILKENP;   GKENILKENPI;
KENILKENPIV;   ENILKENPIVA;   NILKENPIVAA;   ILKENPIVAAY;
LKENPIVAAYK;   KENPIVAAYKE;   ENPIVAAYKEK;   NPIVAAYKEKG;
PIVAAYKEKGF;   IVAAYKEKGFE;   VAAYKEKGFEI;   AAYKEKGFEIL;
AYKEKGFEILI;   YKEKGFEILIM;   KEKGFEILIMD;   EKGFEILIMDD;
KGFEILIMDDE;   GFEILIMDDEL;   FEILIMDDELD;   EILIMDDELDE;
ILIMDDELDEA;   LIMDDELDEAI;   IMDDELDEAIL;   MDDELDEAILN;
DDELDEAILNL;   DELDEAILNLI;   ELDEAILNLIP;   LDEAILNLIPE;
DEAILNLIPEY;   EAILNLIPEYE;   AILNLIPEYEG;   ILNLIPEYEGL;
LNLIPEYEGLK;   NLIPEYEGLKL;   LIPEYEGLKLK;   IPEYEGLKLKA;
PEYEGLKLKAI;   EYEGLKLKAIN;   YEGLKLKAINK;   EGLKLKAINKN;
GLKLKAINKNE;   LKLKAINKNET;   KLKAINKNETS;   LKAINKNETSN;
KAINKNETSNE;   AINKNETSNEL;   INKNETSNELK;   NKNETSNELKD;
KNETSNELKDE;   NETSNELKDEN;   ETSNELKDENF;   TSNELKDENFK;
```

| | |
|---|---|
| | SNELKDENFKK; NELKDENFKKI; ELKDENFKKIE; LKDENFKKIEE;<br>KDENFKKIEEE; DENFKKIEEEF; ENFKKIEEEFK; NFKKIEEEFKD;<br>FKKIEEEFKDT; KKIEEEFKDTL; KIEEEFKDTLT; IEEEFKDTLTK;<br>EEEFKDTLTKV; EEFKDTLTKVK; EFKDTLTKVKE; FKDTLTKVKEI;<br>KDTLTKVKEIL; DTLTKVKEILK; TLTKVKEILKD; LTKVKEILKDH;<br>TKVKEILKDHI; KVKEILKDHIK; VKEILKDHIKE; KEILKDHIKEV;<br>EILKDHIKEVN; ILKDHIKEVNL; LKDHIKEVNLS; KDHIKEVNLSA;<br>DHIKEVNLSAT; HIKEVNLSATL; IKEVNLSATLI; KEVNLSATLIK;<br>EVNLSATLIKE; VNLSATLIKEP; NLSATLIKEPS; LSATLIKEPSA;<br>SATLIKEPSAI; ATLIKEPSAII; TLIKEPSAIII; LIKEPSAIIID;<br>IKEPSAIIIDS; KEPSAIIIDSN; EPSAIIIDSND; PSAIIIDSNDP;<br>SAIIIDSNDPT; AIIIDSNDPTY; IIIDSNDPTYQ; IIDSNDPTYQM;<br>IDSNDPTYQMQ; DSNDPTYQMQK; SNDPTYQMQKI; NDPTYQMQKIM;<br>DPTYQMQKIML; PTYQMQKIMLS; TYQMQKIMLSM; YQMQKIMLSMG;<br>QMQKIMLSMGQ; MQKIMLSMGQE; QKIMLSMGQEV; KIMLSMGQEVK;<br>IMLSMGQEVKE; MLSMGQEVKEI; LSMGQEVKEIK; SMGQEVKEIKP;<br>MGQEVKEIKPI; GQEVKEIKPIL; QEVKEIKPILE; EVKEIKPILEL;<br>VKEIKPILELN; KEIKPILELNP; EIKPILELNPN; IKPILELNPNN;<br>KPILELNPNNK; PILELNPNNKI; ILELNPNNKIV; LELNPNNKIVQ;<br>ELNPNNKIVQN; LNPNNKIVQNL; NPNNKIVQNLK; PNNKIVQNLKN;<br>NNKIVQNLKNL; NKIVQNLKNLE; KIVQNLKNLEP; IVQNLKNLEPE;<br>VQNLKNLEPEK; QNLKNLEPEKL; NLKNLEPEKLE; LKNLEPEKLEK;<br>KNLEPEKLEKI; NLEPEKLEKIS; LEPEKLEKISI; EPEKLEKISIL;<br>PEKLEKISILL; EKLEKISILLF; KLEKISILLFE; LEKISILLFEE;<br>EKISILLFEEA; KISILLFEEAM; ISILLFEEAML; SILLFEEAMLT;<br>ILLFEEAMLTS; LLFEEAMLTSG; LFEEAMLTSGM; FEEAMLTSGMP;<br>EEAMLTSGMPS; EAMLTSGMPSK; AMLTSGMPSKN; MLTSGMPSKNP;<br>LTSGMPSKNPG; TSGMPSKNPGK; SGMPSKNPGKF; GMPSKNPGKFI;<br>MPSKNPGKFIN; PSKNPGKFINI; SKNPGKFINII; KNPGKFINIIN;<br>NPGKFINIINE; PGKFINIINEF; GKFINIINEFI; KFINIINEFIE;<br>FINIINEFIEK; INIINEFIEKD; NIINEFIEKDF; IINEFIEKDFL; |
| CAA44492.1\| Outer surface protein A [Borrelia burgdorferi] | SEQ ID NO 10088-11149/<br>8 mers:<br>MKKYLLGI; KKYLLGIG; KYLLGIGL; YLLGIGLI; LLGIGLIL; LGIGLILA;<br>GIGLILAL; IGLILALI; GLILALIA; LILALIAC; ILALIACK; LALIACKQ;<br>ALIACKQN; LIACKQNV; IACKQNVS; ACKQNVST; CKQNVSTL; KQNVSTLD;<br>QNVSTLDE; NVSTLDEK; VSTLDEKN; STLDEKNS; TLDEKNSV; LDEKNSVS;<br>DEKNSVSV; EKNSVSVD; KNSVSVDL; NSVSVDLP; SVSVDLPG; VSVDLPGG;<br>SVDLPGGM; VDLPGGMT; DLPGGMTE; LPGGMTEL; PGGMTELV; GGMTELVS;<br>GMTELVSK; MTELVSKE; TELVSKEK; ELVSKEKD; LVSKEKDK; VSKEKDKD;<br>SKEKDKDG; KEKDKDGK; EKDKDGKY; KDKDGKYS; DKDGKYSL; KDGKYSLE;<br>DGKYSLEA; GKYSLEAT; KYSLEATV; YSLEATVD; SLEATVDK; LEATVDKL;<br>EATVDKLE; ATVDKLEL; TVDKLELK; VDKLELKG; DKLELKGT; KLELKGTS;<br>LELKGTSD; ELKGTSDK; LKGTSDKN; KGTSDKNN; GTSDKNNG; TSDKNNGS;<br>SDKNNGSG; DKNNGSGT; KNNGSGTL; NNGSGTLE; NGSGTLEG; GSGTLEGE;<br>SGTLEGEK; GTLEGEKT; TLEGEKTD; LEGEKTDK; EGEKTDKS; GEKTDKSK;<br>EKTDKSKV; KTDKSKVK; TDKSKVKL; DKSKVKLT; KSKVKLTI; SKVKLTIA;<br>KVKLTIAD; VKLTIADD; KLTIADDL; LTIADDLS; TIADDLSQ; IADDLSQT;<br>ADDLSQTK; DDLSQTKF; DLSQTKFE; LSQTKFEI; SQTKFEIF; QTKFEIFK;<br>TKFEIFKE; KFEIFKED; FEIFKEDA; EIFKEDAK; IFKEDAKT; FKEDAKTL;<br>KEDAKTLV; EDAKTLVS; DAKTLVSK; AKTLVSKK; KTLVSKKV; TLVSKKVT;<br>LVSKKVTL; VSKKVTLK; SKKVTLKD; KKVTLKDK; KVTLKDKS; VTLKDKSS;<br>TLKDKSST; LKDKSSTE; KDKSSTEE; DKSSTEEK; KSSTEEKF; SSTEEKFN;<br>STEEKFNE; TEEKFNEK; EEKFNEKG; EKFNEKGE; KFNEKGET; FNEKGETS;<br>NEKGETSE; EKGETSEK; KGETSEKT; GETSEKTI; ETSEKTIV; TSEKTIVR;<br>SEKTIVRA; EKTIVRAN; KTIVRANG; TIVRANGT; IVRANGTR; VRANGTRL; |

RANGTRLE; ANGTRLEY; NGTRLEYT; GTRLEYTD; TRLEYTDI; RLEYTDIK;
LEYTDIKS; EYTDIKSD; YTDIKSDG; TDIKSDGS; DIKSDGSK; IKSDGSGK;
KSDGSGKA; SDGSGKAK; DGSGKAKE; GSGKAKEV; SGKAKEVL; GKAKEVLK;
KAKEVLKD; AKEVLKDF; KEVLKDFT; EVLKDFTL; VLKDFTLE; LKDFTLEG;
KDFTLEGT; DFTLEGTL; FTLEGTLA; TLEGTLAA; LEGTLAAD; EGTLAADG;
GTLAADGK; TLAADGKT; LAADGKTT; AADGKTTL; ADGKTTLK; DGKTTLKV;
GKTTLKVT; KTTLKVTE; TTLKVTEG; TLKVTEGT; LKVTEGTV; KVTEGTVV;
VTEGTVVL; TEGTVVLS; EGTVVLSK; GTVVLSKN; TVVLSKNI; VVLSKNIL;
VLSKNILK; LSKNILKS; SKNILKSG; KNILKSGE; NILKSGEI; ILKSGEIT;
LKSGEITV; KSGEITVA; SGEITVAL; GEITVALD; EITVALDD; ITVALDDS;
TVALDDSD; VALDDSDT; ALDDSDTT; LDDSDTTQ; DDSDTTQA; DSDTTQAT;
SDTTQATK; DTTQATKK; TTQATKKT; TQATKKTG; QATKKTGK; ATKKTGKW;
TKKTGKWD; KKTGKWDS; KTGKWDSK; TGKWDSKT; GKWDSKTS; KWDSKTST;
WDSKTSTL; DSKTSTLT; SKTSTLTI; KTSTLTIS; TSTLTISV; STLTISVN;
TLTISVNS; LTISVNSQ; TISVNSQK; ISVNSQKT; SVNSQKTK; VNSQKTKN;
NSQKTKNL; SQKTKNLV; QKTKNLVF; KTKNLVFT; TKNLVFTK; KNLVFTKE;
NLVFTKED; LVFTKEDT; VFTKEDTI; FTKEDTIT; TKEDTITV; KEDTITVQ;
EDTITVQK; DTITVQKY; TITVQKYD; ITVQKYDS; TVQKYDSA; VQKYDSAG;
QKYDSAGT; KYDSAGTN; YDSAGTNL; DSAGTNLE; SAGTNLEG; AGTNLEGK;
GTNLEGKA; TNLEGKAV; NLEGKAVE; LEGKAVEI; EGKAVEIT; GKAVEITT;
KAVEITTL; AVEITTLK; VEITTLKE; EITTLKEL; ITTLKELK; TTLKELKN;
TLKELKNA; LKELKNAL; KELKNALK;

9 mers:
MKKYLLGIG; KKYLLGIGL; KYLLGIGLI; YLLGIGLIL; LLGIGLILA;
LGIGLILAL; GIGLILALI; IGLILALIA; GLILALIAC; LILALIACK;
ILALIACKQ; LALIACKQN; ALIACKQNV; LIACKQNVS; IACKQNVST;
ACKQNVSTL; CKQNVSTLD; KQNVSTLDE; QNVSTLDEK; NVSTLDEKN;
VSTLDEKNS; STLDEKNSV; TLDEKNSVS; LDEKNSVSV; DEKNSVSVD;
EKNSVSVDL; KNSVSVDLP; NSVSVDLPG; SVSVDLPGG; VSVDLPGGM;
SVDLPGGMT; VDLPGGMTE; DLPGGMTEL; LPGGMTELV; PGGMTELVS;
GGMTELVSK; GMTELVSKE; MTELVSKEK; TELVSKEKD; ELVSKEKDK;
LVSKEKDKD; VSKEKDKDG; SKEKDKDGK; KEKDKDGKY; EKDKDGKYS;
KDKDGKYSL; DKDGKYSLE; KDGKYSLEA; DGKYSLEAT; GKYSLEATV;
KYSLEATVD; YSLEATVDK; SLEATVDKL; LEATVDKLE; EATVDKLEL;
ATVDKLELK; TVDKLELKG; VDKLELKGT; DKLELKGTS; KLELKGTSD;
LELKGTSDK; ELKGTSDKN; LKGTSDKNN; KGTSDKNNG; GTSDKNNGS;
TSDKNNGSG; SDKNNGSGT; DKNNGSGTL; KNNGSGTLE; NNGSGTLEG;
NGSGTLEGE; GSGTLEGEK; SGTLEGEKT; GTLEGEKTD; TLEGEKTDK;
LEGEKTDKS; EGEKTDKSK; GEKTDKSKV; EKTDKSKVK; KTDKSKVKL;
TDKSKVKLT; DKSKVKLTI; KSKVKLTIA; SKVKLTIAD; KVKLTIADD;
VKLTIADDL; KLTIADDLS; LTIADDLSQ; TIADDLSQT; IADDLSQTK;
ADDLSQTKF; DDLSQTKFE; DLSQTKFEI; LSQTKFEIF; SQTKFEIFK;
QTKFEIFKE; TKFEIFKED; KFEIFKEDA; FEIFKEDAK; EIFKEDAKT;
IFKEDAKTL; FKEDAKTLV; KEDAKTLVS; EDAKTLVSK; DAKTLVSKK;
AKTLVSKKV; KTLVSKKVT; TLVSKKVTL; LVSKKVTLK; VSKKVTLKD;
SKKVTLKDK; KKVTLKDKS; KVTLKDKSS; VTLKDKSST; TLKDKSSTE;
LKDKSSTEE; KDKSSTEEK; DKSSTEEKF; KSSTEEKFN; SSTEEKFNE;
STEEKFNEK; TEEKFNEKG; EEKFNEKGE; EKFNEKGET; KFNEKGETS;
FNEKGETSE; NEKGETSEK; EKGETSEKT; KGETSEKTI; GETSEKTIV;
ETSEKTIVR; TSEKTIVRA; SEKTIVRAN; EKTIVRANG; KTIVRANGT;
TIVRANGTR; IVRANGTRL; VRANGTRLE; RANGTRLEY; ANGTRLEYT;
NGTRLEYTD; GTRLEYTDI; TRLEYTDIK; RLEYTDIKS; LEYTDIKSD;
EYTDIKSDG; YTDIKSDGS; TDIKSDGSG; DIKSDGSGK; IKSDGSGKA;
KSDGSGKAK; SDGSGKAKE; DGSGKAKEV; GSGKAKEVL; SGKAKEVLK;
GKAKEVLKD; KAKEVLKDF; AKEVLKDFT; KEVLKDFTL; EVLKDFTLE;
VLKDFTLEG; LKDFTLEGT; KDFTLEGTL; DFTLEGTLA; FTLEGTLAA;

TLEGTLAAD; LEGTLAADG; EGTLAADGK; GTLAADGKT; TLAADGKTT;
LAADGKTTL; AADGKTTLK; ADGKTTLKV; DGKTTLKVT; GKTTLKVTE;
KTTLKVTEG; TTLKVTEGT; TLKVTEGTV; LKVTEGTVV; KVTEGTVVL;
VTEGTVVLS; TEGTVVLSK; EGTVVLSKN; GTVVLSKNI; TVVLSKNIL;
VVLSKNILK; VLSKNILKS; LSKNILKSG; SKNILKSGE; KNILKSGEI;
NILKSGEIT; ILKSGEITV; LKSGEITVA; KSGEITVAL; SGEITVALD;
GEITVALDD; EITVALDDS; ITVALDDSD; TVALDDSDT; VALDDSDTT;
ALDDSDTTQ; LDDSDTTQA; DDSDTTQAT; DSDTTQATK; SDTTQATKK;
DTTQATKKT; TTQATKKTG; TQATKKTGK; QATKKTGKW; ATKKTGKWD;
TKKTGKWDS; KKTGKWDSK; KTGKWDSKT; TGKWDSKTS; GKWDSKTST;
KWDSKTSTL; WDSKTSTLT; DSKTSTLTI; SKTSTLTIS; KTSTLTISV;
TSTLTISVN; STLTISVNS; TLTISVNSQ; LTISVNSQK; TISVNSQKT;
ISVNSQKTK; SVNSQKTKN; VNSQKTKNL; NSQKTKNLV; SQKTKNLVF;
QKTKNLVFT; KTKNLVFTK; TKNLVFTKE; KNLVFTKED; NLVFTKEDT;
LVFTKEDTI; VFTKEDTIT; FTKEDTITV; TKEDTITVQ; KEDTITVQK;
EDTITVQKY; DTITVQKYD; TITVQKYDS; ITVQKYDSA; TVQKYDSAG;
VQKYDSAGT; QKYDSAGTN; KYDSAGTNL; YDSAGTNLE; DSAGTNLEG;
SAGTNLEGK; AGTNLEGKA; GTNLEGKAV; TNLEGKAVE; NLEGKAVEI;
LEGKAVEIT; EGKAVEITT; GKAVEITTL; KAVEITTLK; AVEITTLKE;
VEITTLKEL; EITTLKELK; ITTLKELKN; TTLKELKNA; TLKELKNAL;
LKELKNALK

10 mers:
MKKYLLGIGL; KKYLLGIGLI; KYLLGIGLIL; YLLGIGLILA; LLGIGLILAL;
LGIGLILALI; GIGLILALIA; IGLILALIAC; GLILALIACK; LILALIACKQ;
ILALIACKQN; LALIACKQNV; ALIACKQNVS; LIACKQNVST; IACKQNVSTL;
ACKQNVSTLD; CKQNVSTLDE; KQNVSTLDEK; QNVSTLDEKN; NVSTLDEKNS;
VSTLDEKNSV; STLDEKNSVS; TLDEKNSVSV; LDEKNSVSVD; DEKNSVSVDL;
EKNSVSVDLP; KNSVSVDLPG; NSVSVDLPGG; SVSVDLPGGM; VSVDLPGGMT;
SVDLPGGMTE; VDLPGGMTEL; DLPGGMTELV; LPGGMTELVS; PGGMTELVSK;
GGMTELVSKE; GMTELVSKEK; MTELVSKEKD; TELVSKEKDK; ELVSKEKDKD;
LVSKEKDKDG; VSKEKDKDGK; SKEKDKDGKY; KEKDKDGKYS; EKDKDGKYSL;
KDKDGKYSLE; DKDGKYSLEA; KDGKYSLEAT; DGKYSLEATV; GKYSLEATVD;
KYSLEATVDK; YSLEATVDKL; SLEATVDKLE; LEATVDKLEL; EATVDKLELK;
ATVDKLELKG; TVDKLELKGT; VDKLELKGTS; DKLELKGTSD; KLELKGTSDK;
LELKGTSDKN; ELKGTSDKNN; LKGTSDKNNG; KGTSDKNNGS; GTSDKNNGSG;
TSDKNNGSGT; SDKNNGSGTL; DKNNGSGTLE; KNNGSGTLEG; NNGSGTLEGE;
NGSGTLEGEK; GSGTLEGEKT; SGTLEGEKTD; GTLEGEKTDK; TLEGEKTDKS;
LEGEKTDKSK; EGEKTDKSKV; GEKTDKSKVK; EKTDKSKVKL; KTDKSKVKLT;
TDKSKVKLTI; DKSKVKLTIA; KSKVKLTIAD; SKVKLTIADD; KVKLTIADDL;
VKLTIADDLS; KLTIADDLSQ; LTIADDLSQT; TIADDLSQTK; IADDLSQTKF;
ADDLSQTKFE; DDLSQTKFEI; DLSQTKFEIF; LSQTKFEIFK; SQTKFEIFKE;
QTKFEIFKED; TKFEIFKEDA; KFEIFKEDAK; FEIFKEDAKT; EIFKEDAKTL;
IFKEDAKTLV; FKEDAKTLVS; KEDAKTLVSK; EDAKTLVSKK; DAKTLVSKKV;
AKTLVSKKVT; KTLVSKKVTL; TLVSKKVTLK; LVSKKVTLKD; VSKKVTLKDK;
SKKVTLKDKS; KKVTLKDKSS; KVTLKDKSST; VTLKDKSSTE; TLKDKSSTEE;
LKDKSSTEEK; KDKSSTEEKF; DKSSTEEKFN; KSSTEEKFNE; SSTEEKFNEK;
STEEKFNEKG; TEEKFNEKGE; EEKFNEKGET; EKFNEKGETS; KFNEKGETSE;
FNEKGETSEK; NEKGETSEKT; EKGETSEKTI; KGETSEKTIV; GETSEKTIVR;
ETSEKTIVRA; TSEKTIVRAN; SEKTIVRANG; EKTIVRANGT; KTIVRANGTR;
TIVRANGTRL; IVRANGTRLE; VRANGTRLEY; RANGTRLEYT; ANGTRLEYTD;
NGTRLEYTDI; GTRLEYTDIK; TRLEYTDIKS; RLEYTDIKSD; LEYTDIKSDG;
EYTDIKSDGS; YTDIKSDGSG; TDIKSDGSGK; DIKSDGSGKA; IKSDGSGKAK;
KSDGSGKAKE; SDGSGKAKEV; DGSGKAKEVL; GSGKAKEVLK; SGKAKEVLKD;
GKAKEVLKDF; KAKEVLKDFT; AKEVLKDFTL; KEVLKDFTLE; EVLKDFTLEG;
VLKDFTLEGT; LKDFTLEGTL; KDFTLEGTLA; DFTLEGTLAA; FTLEGTLAAD;
TLEGTLAADG; LEGTLAADGK; EGTLAADGKT; GTLAADGKTT; TLAADGKTTL;

LAADGKTTLK; AADGKTTLKV; ADGKTTLKVT; DGKTTLKVTE; GKTTLKVTEG;
KTTLKVTEGT; TTLKVTEGTV; TLKVTEGTVV; LKVTEGTVVL; KVTEGTVVLS;
VTEGTVVLSK; TEGTVVLSKN; EGTVVLSKNI; GTVVLSKNIL; TVVLSKNILK;
VVLSKNILKS; VLSKNILKSG; LSKNILKSGE; SKNILKSGEI; KNILKSGEIT;
NILKSGEITV; ILKSGEITVA; LKSGEITVAL; KSGEITVALD; SGEITVALDD;
GEITVALDDS; EITVALDDSD; ITVALDDSDT; TVALDDSDTT; VALDDSDTTQ;
ALDDSDTTQA; LDDSDTTQAT; DDSDTTQATK; DSDTTQATKK; SDTTQATKKT;
DTTQATKKTG; TTQATKKTGK; TQATKKTGKW; QATKKTGKWD; ATKKTGKWDS;
TKKTGKWDSK; KKTGKWDSKT; KTGKWDSKTS; TGKWDSKTST; GKWDSKTSTL;
KWDSKTSTLT; WDSKTSTLTI; DSKTSTLTIS; SKTSTLTISV; KTSTLTISVN;
TSTLTISVNS; STLTISVNSQ; TLTISVNSQK; LTISVNSQKT; TISVNSQKTK;
ISVNSQKTKN; SVNSQKTKNL; VNSQKTKNLV; NSQKTKNLVF; SQKTKNLVFT;
QKTKNLVFTK; KTKNLVFTKE; TKNLVFTKED; KNLVFTKEDT; NLVFTKEDTI;
LVFTKEDTIT; VFTKEDTITV; FTKEDTITVQ; TKEDTITVQK; KEDTITVQKY;
EDTITVQKYD; DTITVQKYDS; TITVQKYDSA; ITVQKYDSAG; TVQKYDSAGT;
VQKYDSAGTN; QKYDSAGTNL; KYDSAGTNLE; YDSAGTNLEG; DSAGTNLEGK;
SAGTNLEGKA; AGTNLEGKAV; GTNLEGKAVE; TNLEGKAVEI; NLEGKAVEIT;
LEGKAVEITT; EGKAVEITTL; GKAVEITTLK; KAVEITTLKE; AVEITTLKEL;
VEITTLKELK; EITTLKELKN; ITTLKELKNA; TTLKELKNAL; TLKELKNALK;

11 mers:
MKKYLLGIGLI; KKYLLGIGLIL; KYLLGIGLILA; YLLGIGLILAL;
LLGIGLILALI; LGIGLILALIA; GIGLILALIAC; IGLILALIACK;
GLILALIACKQ; LILALIACKQN; ILALIACKQNV; LALIACKQNVS;
ALIACKQNVST; LIACKQNVSTL; IACKQNVSTLD; ACKQNVSTLDE;
CKQNVSTLDEK; KQNVSTLDEKN; QNVSTLDEKNS; NVSTLDEKNSV;
VSTLDEKNSVS; STLDEKNSVSV; TLDEKNSVSVD; LDEKNSVSVDL;
DEKNSVSVDLP; EKNSVSVDLPG; KNSVSVDLPGG; NSVSVDLPGGM;
SVSVDLPGGMT; VSVDLPGGMTE; SVDLPGGMTEL; VDLPGGMTELV;
DLPGGMTELVS; LPGGMTELVSK; PGGMTELVSKE; GGMTELVSKEK;
GMTELVSKEKD; MTELVSKEKDK; TELVSKEKDKD; ELVSKEKDKDG;
LVSKEKDKDGK; VSKEKDKDGKY; SKEKDKDGKYS; KEKDKDGKYSL;
EKDKDGKYSLE; KDKDGKYSLEA; DKDGKYSLEAT; KDGKYSLEATV;
DGKYSLEATVD; GKYSLEATVDK; KYSLEATVDKL; YSLEATVDKLE;
SLEATVDKLEL; LEATVDKLELK; EATVDKLELKG; ATVDKLELKGT;
TVDKLELKGTS; VDKLELKGTSD; DKLELKGTSDK; KLELKGTSDKN;
LELKGTSDKNN; ELKGTSDKNNG; LKGTSDKNNGS; KGTSDKNNGSG;
GTSDKNNGSGT; TSDKNNGSGTL; SDKNNGSGTLE; DKNNGSGTLEG;
KNNGSGTLEGE; NNGSGTLEGEK; NGSGTLEGEKT; GSGTLEGEKTD;
SGTLEGEKTDK; GTLEGEKTDKS; TLEGEKTDKSK; LEGEKTDKSKV;
EGEKTDKSKVK; GEKTDKSKVKL; EKTDKSKVKLT; KTDKSKVKLTI;
TDKSKVKLTIA; DKSKVKLTIAD; KSKVKLTIADD; SKVKLTIADDL;
KVKLTIADDLS; VKLTIADDLSQ; KLTIADDLSQT; LTIADDLSQTK;
TIADDLSQTKF; IADDLSQTKFE; ADDLSQTKFEI; DDLSQTKFEIF;
DLSQTKFEIFK; LSQTKFEIFKE; SQTKFEIFKED; QTKFEIFKEDA;
TKFEIFKEDAK; KFEIFKEDAKT; FEIFKEDAKTL; EIFKEDAKTLV;
IFKEDAKTLVS; FKEDAKTLVSK; KEDAKTLVSKK; EDAKTLVSKKV;
DAKTLVSKKVT; AKTLVSKKVTL; KTLVSKKVTLK; TLVSKKVTLKD;
LVSKKVTLKDK; VSKKVTLKDKS; SKKVTLKDKSS; KKVTLKDKSST;
KVTLKDKSSTE; VTLKDKSSTEE; TLKDKSSTEEK; LKDKSSTEEKF;
KDKSSTEEKFN; DKSSTEEKFNE; KSSTEEKFNEK; SSTEEKFNEKG;
STEEKFNEKGE; TEEKFNEKGET; EEKFNEKGETS; EKFNEKGETSE;
KFNEKGETSEK; FNEKGETSEKT; NEKGETSEKTI; EKGETSEKTIV;
KGETSEKTIVR; GETSEKTIVRA; ETSEKTIVRAN; TSEKTIVRANG;
SEKTIVRANGT; EKTIVRANGTR; KTIVRANGTRL; TIVRANGTRLE;
IVRANGTRLEY; VRANGTRLEYT; RANGTRLEYTD; ANGTRLEYTDI;
NGTRLEYTDIK; GTRLEYTDIKS; TRLEYTDIKSD; RLEYTDIKSDG;

| | |
|---|---|
| | LEYTDIKSDGS; EYTDIKSDGSG; YTDIKSDGSGK; TDIKSDGSGKA; DIKSDGSGKAK; IKSDGSGKAKE; KSDGSGKAKEV; SDGSGKAKEVL; DGSGKAKEVLK; GSGKAKEVLKD; SGKAKEVLKDF; GKAKEVLKDFT; KAKEVLKDFTL; AKEVLKDFTLE; KEVLKDFTLEG; EVLKDFTLEGT; VLKDFTLEGTL; LKDFTLEGTLA; KDFTLEGTLAA; DFTLEGTLAAD; FTLEGTLAADG; TLEGTLAADGK; LEGTLAADGKT; EGTLAADGKTT; GTLAADGKTTL; TLAADGKTTLK; LAADGKTTLKV; AADGKTTLKVT; ADGKTTLKVTE; DGKTTLKVTEG; GKTTLKVTEGT; KTTLKVTEGTV; TTLKVTEGTVV; TLKVTEGTVVL; LKVTEGTVVLS; KVTEGTVVLSK; VTEGTVVLSKN; TEGTVVLSKNI; EGTVVLSKNIL; GTVVLSKNILK; TVVLSKNILKS; VVLSKNILKSG; VLSKNILKSGE; LSKNILKSGEI; SKNILKSGEIT; KNILKSGEITV; NILKSGEITVA; ILKSGEITVAL; LKSGEITVALD; KSGEITVALDD; SGEITVALDDS; GEITVALDDSD; EITVALDDSDT; ITVALDDSDTT; TVALDDSDTTQ; VALDDSDTTQA; ALDDSDTTQAT; LDDSDTTQATK; DDSDTTQATKK; DSDTTQATKKT; SDTTQATKKTG; DTTQATKKTGK; TTQATKKTGKW; TQATKKTGKWD; QATKKTGKWDS; ATKKTGKWDSK; TKKTGKWDSKT; KKTGKWDSKTS; KTGKWDSKTST; TGKWDSKTSTL; GKWDSKTSTLT; KWDSKTSTLTI; WDSKTSTLTIS; DSKTSTLTISV; SKTSTLTISVN; KTSTLTISVNS; TSTLTISVNSQ; STLTISVNSQK; TLTISVNSQKT; LTISVNSQKTK; TISVNSQKTKN; ISVNSQKTKNL; SVNSQKTKNLV; VNSQKTKNLVF; NSQKTKNLVFT; SQKTKNLVFTK; QKTKNLVFTKE; KTKNLVFTKED; TKNLVFTKEDT; KNLVFTKEDTI; NLVFTKEDTIT; LVFTKEDTITV; VFTKEDTITVQ; FTKEDTITVQK; TKEDTITVQKY; KEDTITVQKYD; EDTITVQKYDS; DTITVQKYDSA; TITVQKYDSAG; ITVQKYDSAGT; TVQKYDSAGTN; VQKYDSAGTNL; QKYDSAGTNLE; KYDSAGTNLEG; YDSAGTNLEGK; DSAGTNLEGKA; SAGTNLEGKAV; AGTNLEGKAVE; GTNLEGKAVEI; TNLEGKAVEIT; NLEGKAVEITT; LEGKAVEITTL; EGKAVEITTLK; GKAVEITTLKE; KAVEITTLKEL; AVEITTLKELK; VEITTLKELKN; EITTLKELKNA; ITTLKELKNAL; TTLKELKNALK; |
| \|BAA22351.1\| Outer surface protein B [Borrelia garinii] | SEQ ID NO 11150-12307/<br>8 mers:<br>MKKYLLGF; KKYLLGFA; KYLLGFAL; YLLGFALV; LLGFALVL; LGFALVLA; GFALVLAL; FALVLALI; ALVLALIA; LVLALIAC; VLALIACG; LALIACGQ; ALIACGQK; LIACGQKG; IACGQKGA; ACGQKGAE; CGQKGAEP; GQKGAEPK; QKGAEPKH; KGAEPKHN; GAEPKHND; AEPKHNDQ; EPKHNDQE; PKHNDQEV; KHNDQEVE; HNDQEVED; NDQEVEDS; DQEVEDSK; QEVEDSKK; EVEDSKKD; VEDSKKDQ; EDSKKDQK; DSKKDQKD; SKKDQKDA; KKDQKDAS; KDQKDASK; DQKDASKK; QKDASKKD; KDASKKDL; DASKKDLP; ASKKDLPL; SKKDLPLV; KKDLPLVT; KDLPLVTE; DLPLVTED; LPLVTEDT; PLVTEDTV; LVTEDTVK; VTEDTVKL; TEDTVKLF; EDTVKLFN; DTVKLFND; TVKLFNDT; VKLFNDTK; KLFNDTKI; LFNDTKIF; FNDTKIFI; NDTKIFIS; DTKIFISK; TKIFISKE; KIFISKEK; IFISKEKN; FISKEKNK; ISKEKNKD; SKEKNKDG; KEKNKDGK; EKNKDGKY; KNKDGKYE; NKDGKYEL; KDGKYELR; DGKYELRA; GKYELRAT; KYELRATV; YELRATVD; ELRATVDT; LRATVDTV; RATVDTVE; ATVDTVEL; TVDTVELK; VDTVELKG; DTVELKGV; TVELKGVA; VELKGVAD; ELKGVADK; LKGVADKN; KGVADKND; GVADKNDG; VADKNDGS; ADKNDGSG; DKNDGSGG; KNDGSGGK; NDGSGGKL; DGSGGKLE; GSGGKLEG; SGGKLEGV; GGKLEGVK; GKLEGVKS; KLEGVKSD; LEGVKSDQ; EGVKSDQS; GVKSDQSK; VKSDQSKV; KSDQSKVT; SDQSKVTM; DQSKVTMS; QSKVTMSI; SKVTMSIT; KVTMSITD; VTMSITDD; TMSITDDL; MSITDDLN; SITDDLNT; ITDDLNTI; TDDLNTIT; DDLNTITV; DLNTITVE; LNTITVET; NTITVETY; TITVETYD; ITVETYDS; TVETYDSS; VETYDSSN; ETYDSSNT; TYDSSNTK; YDSSNTKV; DSSNTKVA; SSNTKVAS; SNTKVASK; NTKVASKV; TKVASKVF; KVASKVFK; VASKVFKK; ASKVFKKQ; SKVFKKQG; KVFKKQGS; VFKKQGSL; FKKQGSLT; KKQGSLTE; KQGSLTEE; QGSLTEET; GSLTEETE; SLTEETEE; LTEETEET; TEETEETY; |

EETEETYK; ETEETYKT; TEETYKTG; EETYKTGK; ETYKTGKL; TYKTGKLS;
YKTGKLST; KTGKLSTK; TGKLSTKK; GKLSTKKI; KLSTKKIT; LSTKKITR;
STKKITRT; TKKITRTN; KKITRTNG; KITRTNGT; ITRTNGTT; TRTNGTTL;
RTNGTTLE; TNGTTLEY; NGTTLEYS; GTTLEYSD; TTLEYSDM; TLEYSDMT;
LEYSDMTN; EYSDMTND; YSDMTNDE; SDMTNDEN; DMTNDENA; MTNDENAT;
TNDENATK; NDENATKA; DENATKAV; ENATKAVE; NATKAVET; ATKAVETL;
TKAVETLK; KAVETLKN; AVETLKNG; VETLKNGI; ETLKNGIM; TLKNGIML;
LKNGIMLE; KNGIMLEG; NGIMLEGN; GIMLEGNL; IMLEGNLV; MLEGNLVG;
LEGNLVGG; EGNLVGGK; GNLVGGKT; NLVGGKTS; LVGGKTSV; VGGKTSVE;
GGKTSVEI; GKTSVEIK; KTSVEIKE; TSVEIKEG; SVEIKEGT; VEIKEGTV;
EIKEGTVT; IKEGTVTL; KEGTVTLK; EGTVTLKK; GTVTLKKE; TVTLKKEI;
VTLKKEIE; TLKKEIEK; LKKEIEKA; KKEIEKAG; KEIEKAGT; EIEKAGTV;
IEKAGTVK; EKAGTVKL; KAGTVKLF; AGTVKLFL; GTVKLFLD; TVKLFLDD;
VKLFLDDT; KLFLDDTS; LFLDDTSS; FLDDTSSG; LDDTSSGS; DDTSSGST;
DTSSGSTK; TSSGSTKK; SSGSTKKT; SGSTKKTA; GSTKKTAV; STKKTAVW;
TKKTAVWS; KKTAVWSD; KTAVWSDT; TAVWSDTS; AVWSDTSN; VWSDTSNT;
WSDTSNTL; SDTSNTLT; DTSNTLTV; TSNTLTVS; SNTLTVSA; NTLTVSAD;
TLTVSADS; LTVSADSK; TVSADSKK; VSADSKKI; SADSKKIK; ADSKKIKD;
DSKKIKDF; SKKIKDFV; KKIKDFVF; KIKDFVFL; IKDFVFLT; KDFVFLTD;
DFVFLTDG; FVFLTDGT; VFLTDGTI; FLTDGTIT; LTDGTITV; TDGTITVQ;
DGTITVQN; GTITVQNY; TITVQNYD; ITVQNYDT; TVQNYDTA; VQNYDTAG;
QNYDTAGT; NYDTAGTK; YDTAGTKL; DTAGTKLA; TAGTKLAG; AGTKLAGT;
GTKLAGTA; TKLAGTAT; KLAGTATE; LAGTATEI; AGTATEIK; GTATEIKD;
TATEIKDL; ATEIKDLE; TEIKDLEA; EIKDLEAL; IKDLEALK; KDLEALKA;
DLEALKAA; LEALKAAL; EALKAALK

9 mers:
MKKYLLGFA; KKYLLGFAL; KYLLGFALV; YLLGFALVL; LLGFALVLA;
LGFALVLAL; GFALVLALI; FALVLALIA; ALVLALIAC; LVLALIACG;
VLALIACGQ; LALIACGQK; ALIACGQKG; LIACGQKGA; IACGQKGAE;
ACGQKGAEP; CGQKGAEPK; GQKGAEPKH; QKGAEPKHN; KGAEPKHND;
GAEPKHNDQ; AEPKHNDQE; EPKHNDQEV; PKHNDQEVE; KHNDQEVED;
HNDQEVEDS; NDQEVEDSK; DQEVEDSKK; QEVEDSKKD; EVEDSKKDQ;
VEDSKKDQK; EDSKKDQKD; DSKKDQKDA; SKKDQKDAS; KKDQKDASK;
KDQKDASKK; DQKDASKKD; QKDASKKDL; KDASKKDLP; DASKKDLPL;
ASKKDLPLV; SKKDLPLVT; KKDLPLVTE; KDLPLVTED; DLPLVTEDT;
LPLVTEDTV; PLVTEDTVK; LVTEDTVKL; VTEDTVKLF; TEDTVKLFN;
EDTVKLFND; DTVKLFNDT; TVKLFNDTK; VKLFNDTKI; KLFNDTKIF;
LFNDTKIFI; FNDTKIFIS; NDTKIFISK; DTKIFISKE; TKIFISKEK;
KIFISKEKN; IFISKEKNK; FISKEKNKD; ISKEKNKDG; SKEKNKDGK;
KEKNKDGKY; EKNKDGKYE; KNKDGKYEL; NKDGKYELR; KDGKYELRA;
DGKYELRAT; GKYELRATV; KYELRATVD; YELRATVDT; ELRATVDTV;
LRATVDTVE; RATVDTVEL; ATVDTVELK; TVDTVELKG; VDTVELKGV;
DTVELKGVA; TVELKGVAD; VELKGVADK; ELKGVADKN; LKGVADKND;
KGVADKNDG; GVADKNDGS; VADKNDGSG; ADKNDGSGG; DKNDGSGGK;
KNDGSGGKL; NDGSGGKLE; DGSGGKLEG; GSGGKLEGV; SGGKLEGVK;
GGKLEGVKS; GKLEGVKSD; KLEGVKSDQ; LEGVKSDQS; EGVKSDQSK;
GVKSDQSKV; VKSDQSKVT; KSDQSKVTM; SDQSKVTMS; DQSKVTMSI;
QSKVTMSIT; SKVTMSITD; KVTMSITDD; VTMSITDDL; TMSITDDLN;
MSITDDLNT; SITDDLNTI; ITDDLNTIT; TDDLNTITV; DDLNTITVE;
DLNTITVET; LNTITVETY; NTITVETYD; TITVETYDS; ITVETYDSS;
TVETYDSSN; VETYDSSNT; ETYDSSNTK; TYDSSNTKV; YDSSNTKVA;
DSSNTKVAS; SSNTKVASK; SNTKVASKV; NTKVASKVF; TKVASKVFK;
KVASKVFKK; VASKVFKKQ; ASKVFKKQG; SKVFKKQGS; KVFKKQGSL;
VFKKQGSLT; FKKQGSLTE; KKQGSLTEE; KQGSLTEET; QGSLTEETE;
GSLTEETEE; SLTEETEET; LTEETEETY; TEETEETYK; EETEETYKT;
ETEETYKTG; TEETYKTGK; EETYKTGKL; ETYKTGKLS; TYKTGKLST;

YKTGKLSTK; KTGKLSTKK; TGKLSTKKI; GKLSTKKIT; KLSTKKITR;
LSTKKITRT; STKKITRTN; TKKITRTNG; KKITRTNGT; KITRTNGTT;
ITRTNGTTL; TRTNGTTLE; RTNGTTLEY; TNGTTLEYS; NGTTLEYSD;
GTTLEYSDM; TTLEYSDMT; TLEYSDMTN; LEYSDMTND; EYSDMTNDE;
YSDMTNDEN; SDMTNDENA; DMTNDENAT; MTNDENATK; TNDENATKA;
NDENATKAV; DENATKAVE; ENATKAVET; NATKAVETL; ATKAVETLK;
TKAVETLKN; KAVETLKNG; AVETLKNGI; VETLKNGIM; ETLKNGIML;
TLKNGIMLE; LKNGIMLEG; KNGIMLEGN; NGIMLEGNL; GIMLEGNLV;
IMLEGNLVG; MLEGNLVGG; LEGNLVGGK; EGNLVGGKT; GNLVGGKTS;
NLVGGKTSV; LVGGKTSVE; VGGKTSVEI; GGKTSVEIK; GKTSVEIKE;
KTSVEIKEG; TSVEIKEGT; SVEIKEGTV; VEIKEGTVT; EIKEGTVTL;
IKEGTVTLK; KEGTVTLKK; EGTVTLKKE; GTVTLKKEI; TVTLKKEIE;
VTLKKEIEK; TLKKEIEKA; LKKEIEKAG; KKEIEKAGT; KEIEKAGTV;
EIEKAGTVK; IEKAGTVKL; EKAGTVKLF; KAGTVKLFL; AGTVKLFLD;
GTVKLFLDD; TVKLFLDDT; VKLFLDDTS; KLFLDDTSS; LFLDDTSSG;
FLDDTSSGS; LDDTSSGST; DDTSSGSTK; DTSSGSTKK; TSSGSTKKT;
SSGSTKKTA; SGSTKKTAV; GSTKKTAVW; STKKTAVWS; TKKTAVWSD;
KKTAVWSDT; KTAVWSDTS; TAVWSDTSN; AVWSDTSNT; VWSDTSNTL;
WSDTSNTLT; SDTSNTLTV; DTSNTLTVS; TSNTLTVSA; SNTLTVSAD;
NTLTVSADS; TLTVSADSK; LTVSADSKK; TVSADSKKI; VSADSKKIK;
SADSKKIKD; ADSKKIKDF; DSKKIKDFV; SKKIKDFVF; KKIKDFVFL;
KIKDFVFLT; IKDFVFLTD; KDFVFLTDG; DFVFLTDGT; FVFLTDGTI;
VFLTDGTIT; FLTDGTITV; LTDGTITVQ; TDGTITVQN; DGTITVQNY;
GTITVQNYD; TITVQNYDT; ITVQNYDTA; TVQNYDTAG; VQNYDTAGT;
QNYDTAGTK; NYDTAGTKL; YDTAGTKLA; DTAGTKLAG; TAGTKLAGT;
AGTKLAGTA; GTKLAGTAT; TKLAGTATE; KLAGTATEI; LAGTATEIK;
AGTATEIKD; GTATEIKDL; TATEIKDLE; ATEIKDLEA; TEIKDLEAL;
EIKDLEALK; IKDLEALKA; KDLEALKAA; DLEALKAAL; LEALKAALK;

10 mers:
MKKYLLGFAL; KKYLLGFALV; KYLLGFALVL; YLLGFALVLA; LLGFALVLAL;
LGFALVLALI; GFALVLALIA; FALVLALIAC; ALVLALIACG; LVLALIACGQ;
VLALIACGQK; LALIACGQKG; ALIACGQKGA; LIACGQKGAE; IACGQKGAEP;
ACGQKGAEPK; CGQKGAEPKH; GQKGAEPKHN; QKGAEPKHND; KGAEPKHNDQ;
GAEPKHNDQE; AEPKHNDQEV; EPKHNDQEVE; PKHNDQEVED; KHNDQEVEDS;
HNDQEVEDSK; NDQEVEDSKK; DQEVEDSKKD; QEVEDSKKDQ; EVEDSKKDQK;
VEDSKKDQKD; EDSKKDQKDA; DSKKDQKDAS; SKKDQKDASK; KKDQKDASKK;
KDQKDASKKD; DQKDASKKDL; QKDASKKDLP; KDASKKDLPL; DASKKDLPLV;
ASKKDLPLVT; SKKDLPLVTE; KKDLPLVTED; KDLPLVTEDT; DLPLVTEDTV;
LPLVTEDTVK; PLVTEDTVKL; LVTEDTVKLF; VTEDTVKLFN; TEDTVKLFND;
EDTVKLFNDT; DTVKLFNDTK; TVKLFNDTKI; VKLFNDTKIF; KLFNDTKIFI;
LFNDTKIFIS; FNDTKIFISK; NDTKIFISKE; DTKIFISKEK; TKIFISKEKN;
KIFISKEKNK; IFISKEKNKD; FISKEKNKDG; ISKEKNKDGK; SKEKNKDGKY;
KEKNKDGKYE; EKNKDGKYEL; KNKDGKYELR; NKDGKYELRA; KDGKYELRAT;
DGKYELRATV; GKYELRATVD; KYELRATVDT; YELRATVDTV; ELRATVDTVE;
LRATVDTVEL; RATVDTVELK; ATVDTVELKG; TVDTVELKGV; VDTVELKGVA;
DTVELKGVAD; TVELKGVADK; VELKGVADKN; ELKGVADKND; LKGVADKNDG;
KGVADKNDGS; GVADKNDGSG; VADKNDGSGG; ADKNDGSGGK; DKNDGSGGKL;
KNDGSGGKLE; NDGSGGKLEG; DGSGGKLEGV; GSGGKLEGVK; SGGKLEGVKS;
GGKLEGVKSD; GKLEGVKSDQ; KLEGVKSDQS; LEGVKSDQSK; EGVKSDQSKV;
GVKSDQSKVT; VKSDQSKVTM; KSDQSKVTMS; SDQSKVTMSI; DQSKVTMSIT;
QSKVTMSITD; SKVTMSITDD; KVTMSITDDL; VTMSITDDLN; TMSITDDLNT;
MSITDDLNTI; SITDDLNTIT; ITDDLNTITV; TDDLNTITVE; DDLNTITVET;
DLNTITVETY; LNTITVETYD; NTITVETYDS; TITVETYDSS; ITVETYDSSN;
TVETYDSSNT; VETYDSSNTK; ETYDSSNTKV; TYDSSNTKVA; YDSSNTKVAS;
DSSNTKVASK; SSNTKVASKV; SNTKVASKVF; NTKVASKVFK; TKVASKVFKK;
KVASKVFKKQ; VASKVFKKQG; ASKVFKKQGS; SKVFKKQGSL; KVFKKQGSLT;

VFKKQGSLTE; FKKQGSLTEE; KKQGSLTEET; KQGSLTEETE; QGSLTEETEE;
GSLTEETEET; SLTEETEETY; LTEETEETYK; TEETEETYKT; EETEETYKTG;
ETEETYKTGK; TEETYKTGKL; EETYKTGKLS; ETYKTGKLST; TYKTGKLSTK;
YKTGKLSTKK; KTGKLSTKKI; TGKLSTKKIT; GKLSTKKITR; KLSTKKITRT;
LSTKKITRTN; STKKITRTNG; TKKITRTNGT; KKITRTNGTT; KITRTNGTTL;
ITRTNGTTLE; TRTNGTTLEY; RTNGTTLEYS; TNGTTLEYSD; NGTTLEYSDM;
GTTLEYSDMT; TTLEYSDMTN; TLEYSDMTND; LEYSDMTNDE; EYSDMTNDEN;
YSDMTNDENA; SDMTNDENAT; DMTNDENATK; MTNDENATKA; TNDENATKAV;
NDENATKAVE; DENATKAVET; ENATKAVETL; NATKAVETLK; ATKAVETLKN;
TKAVETLKNG; KAVETLKNGI; AVETLKNGIM; VETLKNGIML; ETLKNGIMLE;
TLKNGIMLEG; LKNGIMLEGN; KNGIMLEGNL; NGIMLEGNLV; GIMLEGNLVG;
IMLEGNLVGG; MLEGNLVGGK; LEGNLVGGKT; EGNLVGGKTS; GNLVGGKTSV;
NLVGGKTSVE; LVGGKTSVEI; VGGKTSVEIK; GGKTSVEIKE; GKTSVEIKEG;
KTSVEIKEGT; TSVEIKEGTV; SVEIKEGTVT; VEIKEGTVTL; EIKEGTVTLK;
IKEGTVTLKK; KEGTVTLKKE; EGTVTLKKEI; GTVTLKKEIE; TVTLKKEIEK;
VTLKKEIEKA; TLKKEIEKAG; LKKEIEKAGT; KKEIEKAGTV; KEIEKAGTVK;
EIEKAGTVKL; IEKAGTVKLF; EKAGTVKLFL; KAGTVKLFLD; AGTVKLFLDD;
GTVKLFLDDT; TVKLFLDDTS; VKLFLDDTSS; KLFLDDTSSG; LFLDDTSSGS;
FLDDTSSGST; LDDTSSGSTK; DDTSSGSTKK; DTSSGSTKKT; TSSGSTKKTA;
SSGSTKKTAV; SGSTKKTAVW; GSTKKTAVWS; STKKTAVWSD; TKKTAVWSDT;
KKTAVWSDTS; KTAVWSDTSN; TAVWSDTSNT; AVWSDTSNTL; VWSDTSNTLT;
WSDTSNTLTV; SDTSNTLTVS; DTSNTLTVSA; TSNTLTVSAD; SNTLTVSADS;
NTLTVSADSK; TLTVSADSKK; LTVSADSKKI; TVSADSKKIK; VSADSKKIKD;
SADSKKIKDF; ADSKKIKDFV; DSKKIKDFVF; SKKIKDFVFL; KKIKDFVFLT;
KIKDFVFLTD; IKDFVFLTDG; KDFVFLTDGT; DFVFLTDGTI; FVFLTDGTIT;
VFLTDGTITV; FLTDGTITVQ; LTDGTITVQN; TDGTITVQNY; DGTITVQNYD;
GTITVQNYDT; TITVQNYDTA; ITVQNYDTAG; TVQNYDTAGT; VQNYDTAGTK;
QNYDTAGTKL; NYDTAGTKLA; YDTAGTKLAG; DTAGTKLAGT; TAGTKLAGTA;
AGTKLAGTAT; GTKLAGTATE; TKLAGTATEI; KLAGTATEIK; LAGTATEIKD;
AGTATEIKDL; GTATEIKDLE; TATEIKDLEA; ATEIKDLEAL; TEIKDLEALK;
EIKDLEALKA; IKDLEALKAA; KDLEALKAAL; DLEALKAALK;

11 mers:
MKKYLLGFALV; KKYLLGFALVL; KYLLGFALVLA; YLLGFALVLAL;
LLGFALVLALI; LGFALVLALIA; GFALVLALIAC; FALVLALIACG;
ALVLALIACGQ; LVLALIACGQK; VLALIACGQKG; LALIACGQKGA;
ALIACGQKGAE; LIACGQKGAEP; IACGQKGAEPK; ACGQKGAEPKH;
CGQKGAEPKHN; GQKGAEPKHND; QKGAEPKHNDQ; KGAEPKHNDQE;
GAEPKHNDQEV; AEPKHNDQEVE; EPKHNDQEVED; PKHNDQEVEDS;
KHNDQEVEDSK; HNDQEVEDSKK; NDQEVEDSKKD; DQEVEDSKKDQ;
QEVEDSKKDQK; EVEDSKKDQKD; VEDSKKDQKDA; EDSKKDQKDAS;
DSKKDQKDASK; SKKDQKDASKK; KKDQKDASKKD; KDQKDASKKDL;
DQKDASKKDLP; QKDASKKDLPL; KDASKKDLPLV; DASKKDLPLVT;
ASKKDLPLVTE; SKKDLPLVTED; KKDLPLVTEDT; KDLPLVTEDTV;
DLPLVTEDTVK; LPLVTEDTVKL; PLVTEDTVKLF; LVTEDTVKLFN;
VTEDTVKLFND; TEDTVKLFNDT; EDTVKLFNDTK; DTVKLFNDTKI;
TVKLFNDTKIF; VKLFNDTKIFI; KLFNDTKIFIS; LFNDTKIFISK;
FNDTKIFISKE; NDTKIFISKEK; DTKIFISKEKN; TKIFISKEKNK;
KIFISKEKNKD; IFISKEKNKDG; FISKEKNKDGK; ISKEKNKDGKY;
SKEKNKDGKYE; KEKNKDGKYEL; EKNKDGKYELR; KNKDGKYELRA;
NKDGKYELRAT; KDGKYELRATV; DGKYELRATVD; GKYELRATVDT;
KYELRATVDTV; YELRATVDTVE; ELRATVDTVEL; LRATVDTVELK;
RATVDTVELKG; ATVDTVELKGV; TVDTVELKGVA; VDTVELKGVAD;
DTVELKGVADK; TVELKGVADKN; VELKGVADKND; ELKGVADKNDG;
LKGVADKNDGS; KGVADKNDGSG; GVADKNDGSGG; VADKNDGSGGK;
ADKNDGSGGKL; DKNDGSGGKLE; KNDGSGGKLEG; NDGSGGKLEGV;
DGSGGKLEGVK; GSGGKLEGVKS; SGGKLEGVKSD; GGKLEGVKSDQ;

| | |
|---|---|
| | GKLEGVKSDQS;  KLEGVKSDQSK;  LEGVKSDQSKV;  EGVKSDQSKVT;<br>GVKSDQSKVTM;  VKSDQSKVTMS;  KSDQSKVTMSI;  SDQSKVTMSIT;<br>DQSKVTMSITD;  QSKVTMSITDD;  SKVTMSITDDL;  KVTMSITDDLN;<br>VTMSITDDLNT;  TMSITDDLNTI;  MSITDDLNTIT;  SITDDLNTITV;<br>ITDDLNTITVE;  TDDLNTITVET;  DDLNTITVETY;  DLNTITVETYD;<br>LNTITVETYDS;  NTITVETYDSS;  TITVETYDSSN;  ITVETYDSSNT;<br>TVETYDSSNTK;  VETYDSSNTKV;  ETYDSSNTKVA;  TYDSSNTKVAS;<br>YDSSNTKVASK;  DSSNTKVASKV;  SSNTKVASKVF;  SNTKVASKVFK;<br>NTKVASKVFKK;  TKVASKVFKKQ;  KVASKVFKKQG;  VASKVFKKQGS;<br>ASKVFKKQGSL;  SKVFKKQGSLT;  KVFKKQGSLTE;  VFKKQGSLTEE;<br>FKKQGSLTEET;  KKQGSLTEETE;  KQGSLTEETEE;  QGSLTEETEET;<br>GSLTEETEETY;  SLTEETEETYK;  LTEETEETYKT;  TEETEETYKTG;<br>EETEETYKTGK;  ETEETYKTGKL;  TEETYKTGKLS;  EETYKTGKLST;<br>ETYKTGKLSTK;  TYKTGKLSTKK;  YKTGKLSTKKI;  KTGKLSTKKIT;<br>TGKLSTKKITR;  GKLSTKKITRT;  KLSTKKITRTN;  LSTKKITRTNG;<br>STKKITRTNGT;  TKKITRTNGTT;  KKITRTNGTTL;  KITRTNGTTLE;<br>ITRTNGTTLEY;  TRTNGTTLEYS;  RTNGTTLEYSD;  TNGTTLEYSDM;<br>NGTTLEYSDMT;  GTTLEYSDMTN;  TTLEYSDMTND;  TLEYSDMTNDE;<br>LEYSDMTNDEN;  EYSDMTNDENA;  YSDMTNDENAT;  SDMTNDENATK;<br>DMTNDENATKA;  MTNDENATKAV;  TNDENATKAVE;  NDENATKAVET;<br>DENATKAVETL;  ENATKAVETLK;  NATKAVETLKN;  ATKAVETLKNG;<br>TKAVETLKNGI;  KAVETLKNGIM;  AVETLKNGIML;  VETLKNGIMLE;<br>ETLKNGIMLEG;  TLKNGIMLEGN;  LKNGIMLEGNL;  KNGIMLEGNLV;<br>NGIMLEGNLVG;  GIMLEGNLVGG;  IMLEGNLVGGK;  MLEGNLVGGKT;<br>LEGNLVGGKTS;  EGNLVGGKTSV;  GNLVGGKTSVE;  NLVGGKTSVEI;<br>LVGGKTSVEIK;  VGGKTSVEIKE;  GGKTSVEIKEG;  GKTSVEIKEGT;<br>KTSVEIKEGTV;  TSVEIKEGTVT;  SVEIKEGTVTL;  VEIKEGTVTLK;<br>EIKEGTVTLKK;  IKEGTVTLKKE;  KEGTVTLKKEI;  EGTVTLKKEIE;<br>GTVTLKKEIEK;  TVTLKKEIEKA;  VTLKKEIEKAG;  TLKKEIEKAGT;<br>LKKEIEKAGTV;  KKEIEKAGTVK;  KEIEKAGTVKL;  EIEKAGTVKLF;<br>IEKAGTVKLFL;  EKAGTVKLFLD;  KAGTVKLFLDD;  AGTVKLFLDDT;<br>GTVKLFLDDTS;  TVKLFLDDTSS;  VKLFLDDTSSG;  KLFLDDTSSGS;<br>LFLDDTSSGST;  FLDDTSSGSTK;  LDDTSSGSTKK;  DDTSSGSTKKT;<br>DTSSGSTKKTA;  TSSGSTKKTAV;  SSGSTKKTAVW;  SGSTKKTAVWS;<br>GSTKKTAVWSD;  STKKTAVWSDT;  TKKTAVWSDTS;  KKTAVWSDTSN;<br>KTAVWSDTSNT;  TAVWSDTSNTL;  AVWSDTSNTLT;  VWSDTSNTLTV;<br>WSDTSNTLTVS;  SDTSNTLTVSA;  DTSNTLTVSAD;  TSNTLTVSADS;<br>SNTLTVSADSK;  NTLTVSADSKK;  TLTVSADSKKI;  LTVSADSKKIK;<br>TVSADSKKIKD;  VSADSKKIKDF;  SADSKKIKDFV;  ADSKKIKDFVF;<br>DSKKIKDFVFL;  SKKIKDFVFLT;  KKIKDFVFLTD;  KIKDFVFLTDG;<br>IKDFVFLTDGT;  KDFVFLTDGTI;  DFVFLTDGTIT;  FVFLTDGTITV;<br>VFLTDGTITVQ;  FLTDGTITVQN;  LTDGTITVQNY;  TDGTITVQNYD;<br>DGTITVQNYDT;  GTITVQNYDTA;  TITVQNYDTAG;  ITVQNYDTAGT;<br>TVQNYDTAGTK;  VQNYDTAGTKL;  QNYDTAGTKLA;  NYDTAGTKLAG;<br>YDTAGTKLAGT;  DTAGTKLAGTA;  TAGTKLAGTAT;  AGTKLAGTATE;<br>GTKLAGTATEI;  TKLAGTATEIK;  KLAGTATEIKD;  LAGTATEIKDL;<br>AGTATEIKDLE;  GTATEIKDLEA;  TATEIKDLEAL;  ATEIKDLEALK;<br>TEIKDLEALKA;  EIKDLEALKAA;  IKDLEALKAAL;  KDLEALKAALK; |
| AAM22469.1\| Outer surface protein C [Borrelia afzelii] | SEQ ID NO 12308-13117/<br>8 mers:<br>MKKNTLSA;  KKNTLSAI;  KNTLSAIL;  NTLSAILM;  TLSAILMT;  LSAILMTL;<br>SAILMTLF;  AILMTLFL;  ILMTLFLF;  LMTLFLFI;  MTLFLFIS;  TLFLFISC;<br>LFLFISCN;  FLFISCNN;  LFISCNNS;  FISCNNSG;  ISCNNSGK;  SCNNSGKG;<br>CNNSGKGG;  NNSGKGGD;  NSGKGGDS;  SGKGGDSA;  GKGGDSAS;  KGGDSAST;<br>GGDSASTN;  GDSASTNP;  DSASTNPA;  SASTNPAD;  ASTNPADE;  STNPADES;<br>TNPADESA;  NPADESAK;  PADESAKG;  ADESAKGP;  DESAKGPN;  ESAKGPNL;<br>SAKGPNLT;  AKGPNLTE;  KGPNLTEI;  GPNLTEIS;  PNLTEISK;  NLTEISKK; |

LTEISKKI; TEISKKIT; EISKKITD; ISKKITDS; SKKITDSN; KKITDSNA;
KITDSNAF; ITDSNAFV; TDSNAFVL; DSNAFVLA; SNAFVLAV; NAFVLAVK;
AFVLAVKE; FVLAVKEV; VLAVKEVE; LAVKEVET; AVKEVETL; VKEVETLV;
KEVETLVS; EVETLVSS; VETLVSSI; ETLVSSID; TLVSSIDE; LVSSIDEL;
VSSIDELA; SSIDELAN; SIDELANK; IDELANKA; DELANKAI; ELANKAIG;
LANKAIGK; ANKAIGKK; NKAIGKKI; KAIGKKIQ; AIGKKIQQ; IGKKIQQN;
GKKIQQNG; KKIQQNGL; KIQQNGLG; IQQNGLGA; QQNGLGAE; QNGLGAEA;
NGLGAEAN; GLGAEANR; LGAEANRN; GAEANRNE; AEANRNES; EANRNESL;
ANRNESLL; NRNESLLA; RNESLLAG; NESLLAGV; ESLLAGVH; SLLAGVHE;
LLAGVHEI; LAGVHEIS; AGVHEIST; GVHEISTL; VHEISTLI; HEISTLIT;
EISTLITE; ISTLITEK; STLITEKL; TLITEKLS; LITEKLSK; ITEKLSKL;
TEKLSKLK; EKLSKLKN; KLSKLKNS; LSKLKNSG; SKLKNSGE; KLKNSGEL;
LKNSGELK; KNSGELKA; NSGELKAK; SGELKAKI; GELKAKIE; ELKAKIED;
LKAKIEDA; KAKIEDAK; AKIEDAKK; KIEDAKKC; IEDAKKCS; EDAKKCSE;
DAKKCSEE; AKKCSEEF; KKCSEEFT; KCSEEFTN; CSEEFTNK; SEEFTNKL;
EEFTNKLR; EFTNKLRV; FTNKLRVS; TNKLRVSH; NKLRVSHA; KLRVSHAD;
LRVSHADL; RVSHADLG; VSHADLGK; SHADLGKQ; HADLGKQG; ADLGKQGV;
DLGKQGVN; LGKQGVND; GKQGVNDD; KQGVNDDD; QGVNDDDA; GVNDDDAK;
VNDDDAKK; NDDDAKKA; DDDAKKAI; DDAKKAIL; DAKKAILK; AKKAILKT;
KKAILKTN; KAILKTNA; AILKTNAD; ILKTNADK; LKTNADKT; KTNADKTK;
TNADKTKG; NADKTKGA; ADKTKGAE; DKTKGAEE; KTKGAEEL; TKGAEELG;
KGAEELGK; GAEELGKL; AEELGKLF; EELGKLFK; ELGKLFKS; LGKLFKSV;
GKLFKSVE; KLFKSVEG; LFKSVEGL; FKSVEGLV; KSVEGLVK; SVEGLVKA;
VEGLVKAA; EGLVKAAQ; GLVKAAQE; LVKAAQEA; VKAAQEAL; KAAQEALT;
AAQEALTN; AQEALTNS; QEALTNSV; EALTNSVK; ALTNSVKE; LTNSVKEL;
TNSVKELT; NSVKELTS; SVKELTSP; VKELTSPV; KELTSPVV; ELTSPVVA;
LTSPVVAE; TSPVVAES; SPVVAESP; PVVAESPK; VVAESPKK; VAESPKKP;

9 mers:
MKKNTLSAI; KKNTLSAIL; KNTLSAILM; NTLSAILMT; TLSAILMTL;
LSAILMTLF; SAILMTLFL; AILMTLFLF; ILMTLFLFI; LMTLFLFIS;
MTLFLFISC; TLFLFISCN; LFLFISCNN; FLFISCNNS; LFISCNNSG;
FISCNNSGK; ISCNNSGKG; SCNNSGKGG; CNNSGKGGD; NNSGKGGDS;
NSGKGGDSA; SGKGGDSAS; GKGGDSAST; KGGDSASTN; GGDSASTNP;
GDSASTNPA; DSASTNPAD; SASTNPADE; ASTNPADES; STNPADESA;
TNPADESAK; NPADESAKG; PADESAKGP; ADESAKGPN; DESAKGPNL;
ESAKGPNLT; SAKGPNLTE; AKGPNLTEI; KGPNLTEIS; GPNLTEISK;
PNLTEISKK; NLTEISKKI; LTEISKKIT; TEISKKITD; EISKKITDS;
ISKKITDSN; SKKITDSNA; KKITDSNAF; KITDSNAFV; ITDSNAFVL;
TDSNAFVLA; DSNAFVLAV; SNAFVLAVK; NAFVLAVKE; AFVLAVKEV;
FVLAVKEVE; VLAVKEVET; LAVKEVETL; AVKEVETLV; VKEVETLVS;
KEVETLVSS; EVETLVSSI; VETLVSSID; ETLVSSIDE; TLVSSIDEL;
LVSSIDELA; VSSIDELAN; SSIDELANK; SIDELANKA; IDELANKAI;
DELANKAIG; ELANKAIGK; LANKAIGKK; ANKAIGKKI; NKAIGKKIQ;
KAIGKKIQQ; AIGKKIQQN; IGKKIQQNG; GKKIQQNGL; KKIQQNGLG;
KIQQNGLGA; IQQNGLGAE; QQNGLGAEA; QNGLGAEAN; NGLGAEANR;
GLGAEANRN; LGAEANRNE; GAEANRNES; AEANRNESL; EANRNESLL;
ANRNESLLA; NRNESLLAG; RNESLLAGV; NESLLAGVH; ESLLAGVHE;
SLLAGVHEI; LLAGVHEIS; LAGVHEIST; AGVHEISTL; GVHEISTLI;
VHEISTLIT; HEISTLITE; EISTLITEK; ISTLITEKL; STLITEKLS;
TLITEKLSK; LITEKLSKL; ITEKLSKLK; TEKLSKLKN; EKLSKLKNS;
KLSKLKNSG; LSKLKNSGE; SKLKNSGEL; KLKNSGELK; LKNSGELKA;
KNSGELKAK; NSGELKAKI; SGELKAKIE; GELKAKIED; ELKAKIEDA;
LKAKIEDAK; KAKIEDAKK; AKIEDAKKC; KIEDAKKCS; IEDAKKCSE;
EDAKKCSEE; DAKKCSEEF; AKKCSEEFT; KKCSEEFTN; KCSEEFTNK;
CSEEFTNKL; SEEFTNKLR; EEFTNKLRV; EFTNKLRVS; FTNKLRVSH;
TNKLRVSHA; NKLRVSHAD; KLRVSHADL; LRVSHADLG; RVSHADLGK;

VSHADLGKQ; SHADLGKQG; HADLGKQGV; ADLGKQGVN; DLGKQGVND;
LGKQGVNDD; GKQGVNDDD; KQGVNDDDA; QGVNDDDAK; GVNDDDAKK;
VNDDDAKKA; NDDDAKKAI; DDDAKKAIL; DDAKKAILK; DAKKAILKT;
AKKAILKTN; KKAILKTNA; KAILKTNAD; AILKTNADK; ILKTNADKT;
LKTNADKTK; KTNADKTKG; TNADKTKGA; NADKTKGAE; ADKTKGAEE;
DKTKGAEEL; KTKGAEELG; TKGAEELGK; KGAEELGKL; GAEELGKLF;
AEELGKLFK; EELGKLFKS; ELGKLFKSV; LGKLFKSVE; GKLFKSVEG;
KLFKSVEGL; LFKSVEGLV; FKSVEGLVK; KSVEGLVKA; SVEGLVKAA;
VEGLVKAAQ; EGLVKAAQE; GLVKAAQEA; LVKAAQEAL; VKAAQEALT;
KAAQEALTN; AAQEALTNS; AQEALTNSV; QEALTNSVK; EALTNSVKE;
ALTNSVKEL; LTNSVKELT; TNSVKELTS; NSVKELTSP; SVKELTSPV;
VKELTSPVV; KELTSPVVA; ELTSPVVAE; LTSPVVAES; TSPVVAESP;
SPVVAESPK; PVVAESPKK; VVAESPKKP;

10 mers:
MKKNTLSAIL; KKNTLSAILM; KNTLSAILMT; NTLSAILMTL; TLSAILMTLF;
LSAILMTLFL; SAILMTLFLF; AILMTLFLFI; ILMTLFLFIS; LMTLFLFISC;
MTLFLFISCN; TLFLFISCNN; LFLFISCNNS; FLFISCNNSG; LFISCNNSGK;
FISCNNSGKG; ISCNNSGKGG; SCNNSGKGGD; CNNSGKGGDS; NNSGKGGDSA;
NSGKGGDSAS; SGKGGDSAST; GKGGDSASTN; KGGDSASTNP; GGDSASTNPA;
GDSASTNPAD; DSASTNPADE; SASTNPADES; ASTNPADESA; STNPADESAK;
TNPADESAKG; NPADESAKGP; PADESAKGPN; ADESAKGPNL; DESAKGPNLT;
ESAKGPNLTE; SAKGPNLTEI; AKGPNLTEIS; KGPNLTEISK; GPNLTEISKK;
PNLTEISKKI; NLTEISKKIT; LTEISKKITD; TEISKKITDS; EISKKITDSN;
ISKKITDSNA; SKKITDSNAF; KKITDSNAFV; KITDSNAFVL; ITDSNAFVLA;
TDSNAFVLAV; DSNAFVLAVK; SNAFVLAVKE; NAFVLAVKEV; AFVLAVKEVE;
FVLAVKEVET; VLAVKEVETL; LAVKEVETLV; AVKEVETLVS; VKEVETLVSS;
KEVETLVSSI; EVETLVSSID; VETLVSSIDE; ETLVSSIDEL; TLVSSIDELA;
LVSSIDELAN; VSSIDELANK; SSIDELANKA; SIDELANKAI; IDELANKAIG;
DELANKAIGK; ELANKAIGKK; LANKAIGKKI; ANKAIGKKIQ; NKAIGKKIQQ;
KAIGKKIQQN; AIGKKIQQNG; IGKKIQQNGL; GKKIQQNGLG; KKIQQNGLGA;
KIQQNGLGAE; IQQNGLGAEA; QQNGLGAEAN; QNGLGAEANR; NGLGAEANRN;
GLGAEANRNE; LGAEANRNES; GAEANRNESL; AEANRNESLL; EANRNESLLA;
ANRNESLLAG; NRNESLLAGV; RNESLLAGVH; NESLLAGVHE; ESLLAGVHEI;
SLLAGVHEIS; LLAGVHEIST; LAGVHEISTL; AGVHEISTLI; GVHEISTLIT;
VHEISTLITE; HEISTLITEK; EISTLITEKL; ISTLITEKLS; STLITEKLSK;
TLITEKLSKL; LITEKLSKLK; ITEKLSKLKN; TEKLSKLKNS; EKLSKLKNSG;
KLSKLKNSGE; LSKLKNSGEL; SKLKNSGELK; KLKNSGELKA; LKNSGELKAK;
KNSGELKAKI; NSGELKAKIE; SGELKAKIED; GELKAKIEDA; ELKAKIEDAK;
LKAKIEDAKK; KAKIEDAKKC; AKIEDAKKCS; KIEDAKKCSE; IEDAKKCSEE;
EDAKKCSEEF; DAKKCSEEFT; AKKCSEEFTN; KKCSEEFTNK; KCSEEFTNKL;
CSEEFTNKLR; SEEFTNKLRV; EEFTNKLRVS; EFTNKLRVSH; FTNKLRVSHA;
TNKLRVSHAD; NKLRVSHADL; KLRVSHADLG; LRVSHADLGK; RVSHADLGKQ;
VSHADLGKQG; SHADLGKQGV; HADLGKQGVN; ADLGKQGVND; DLGKQGVNDD;
LGKQGVNDDD; GKQGVNDDDA; KQGVNDDDAK; QGVNDDDAKK; GVNDDDAKKA;
VNDDDAKKAI; NDDDAKKAIL; DDDAKKAILK; DDAKKAILKT; DAKKAILKTN;
AKKAILKTNA; KKAILKTNAD; KAILKTNADK; AILKTNADKT; ILKTNADKTK;
LKTNADKTKG; KTNADKTKGA; TNADKTKGAE; NADKTKGAEE; ADKTKGAEEL;
DKTKGAEELG; KTKGAEELGK; TKGAEELGKL; KGAEELGKLF; GAEELGKLFK;
AEELGKLFKS; EELGKLFKSV; ELGKLFKSVE; LGKLFKSVEG; GKLFKSVEGL;
KLFKSVEGLV; LFKSVEGLVK; FKSVEGLVKA; KSVEGLVKAA; SVEGLVKAAQ;
VEGLVKAAQE; EGLVKAAQEA; GLVKAAQEAL; LVKAAQEALT; VKAAQEALTN;
KAAQEALTNS; AAQEALTNSV; AQEALTNSVK; QEALTNSVKE; EALTNSVKEL;
ALTNSVKELT; LTNSVKELTS; TNSVKELTSP; NSVKELTSPV; SVKELTSPVV;
VKELTSPVVA; KELTSPVVAE; ELTSPVVAES; LTSPVVAESP; TSPVVAESPK;
SPVVAESPKK; PVVAESPKKP;

| | |
|---|---|
| | 11 mers:<br>MKKNTLSAILM; KKNTLSAILMT; KNTLSAILMTL; NTLSAILMTLF;<br>TLSAILMTLFL; LSAILMTLFLF; SAILMTLFLFI; AILMTLFLFIS;<br>ILMTLFLFISC; LMTLFLFISCN; MTLFLFISCNN; TLFLFISCNNS;<br>LFLFISCNNSG; FLFISCNNSGK; LFISCNNSGKG; FISCNNSGKGG;<br>ISCNNSGKGGD; SCNNSGKGGDS; CNNSGKGGDSA; NNSGKGGDSAS;<br>NSGKGGDSAST; SGKGGDSASTN; GKGGDSASTNP; KGGDSASTNPA;<br>GGDSASTNPAD; GDSASTNPADE; DSASTNPADES; SASTNPADESA;<br>ASTNPADESAK; STNPADESAKG; TNPADESAKGP; NPADESAKGPN;<br>PADESAKGPNL; ADESAKGPNLT; DESAKGPNLTE; ESAKGPNLTEI;<br>SAKGPNLTEIS; AKGPNLTEISK; KGPNLTEISKK; GPNLTEISKKI;<br>PNLTEISKKIT; NLTEISKKITD; LTEISKKITDS; TEISKKITDSN;<br>EISKKITDSNA; ISKKITDSNAF; SKKITDSNAFV; KKITDSNAFVL;<br>KITDSNAFVLA; ITDSNAFVLAV; TDSNAFVLAVK; DSNAFVLAVKE;<br>SNAFVLAVKEV; NAFVLAVKEVE; AFVLAVKEVET; FVLAVKEVETL;<br>VLAVKEVETLV; LAVKEVETLVS; AVKEVETLVSS; VKEVETLVSSI;<br>KEVETLVSSID; EVETLVSSIDE; VETLVSSIDEL; ETLVSSIDELA;<br>TLVSSIDELAN; LVSSIDELANK; VSSIDELANKA; SSIDELANKAI;<br>SIDELANKAIG; IDELANKAIGK; DELANKAIGKK; ELANKAIGKKI;<br>LANKAIGKKIQ; ANKAIGKKIQQ; NKAIGKKIQQN; KAIGKKIQQNG;<br>AIGKKIQQNGL; IGKKIQQNGLG; GKKIQQNGLGA; KKIQQNGLGAE;<br>KIQQNGLGAEA; IQQNGLGAEAN; QQNGLGAEANR; QNGLGAEANRN;<br>NGLGAEANRNE; GLGAEANRNES; LGAEANRNESL; GAEANRNESLL;<br>AEANRNESLLA; EANRNESLLAG; ANRNESLLAGV; NRNESLLAGVH;<br>RNESLLAGVHE; NESLLAGVHEI; ESLLAGVHEIS; SLLAGVHEIST;<br>LLAGVHEISTL; LAGVHEISTLI; AGVHEISTLIT; GVHEISTLITE;<br>VHEISTLITEK; HEISTLITEKL; EISTLITEKLS; ISTLITEKLSK;<br>STLITEKLSKL; TLITEKLSKLK; LITEKLSKLKN; ITEKLSKLKNS;<br>TEKLSKLKNSG; EKLSKLKNSGE; KLSKLKNSGEL; LSKLKNSGELK;<br>SKLKNSGELKA; KLKNSGELKAK; LKNSGELKAKI; KNSGELKAKIE;<br>NSGELKAKIED; SGELKAKIEDA; GELKAKIEDAK; ELKAKIEDAKK;<br>LKAKIEDAKKC; KAKIEDAKKCS; AKIEDAKKCSE; KIEDAKKCSEE;<br>IEDAKKCSEEF; EDAKKCSEEFT; DAKKCSEEFTN; AKKCSEEFTNK;<br>KKCSEEFTNKL; KCSEEFTNKLR; CSEEFTNKLRV; SEEFTNKLRVS;<br>EEFTNKLRVSH; EFTNKLRVSHA; FTNKLRVSHAD; TNKLRVSHADL;<br>NKLRVSHADLG; KLRVSHADLGK; LRVSHADLGKQ; RVSHADLGKQG;<br>VSHADLGKQGV; SHADLGKQGVN; HADLGKQGVND; ADLGKQGVNDD;<br>DLGKQGVNDDD; LGKQGVNDDDA; GKQGVNDDDAK; KQGVNDDDAKK;<br>QGVNDDDAKKA; GVNDDDAKKAI; VNDDDAKKAIL; NDDDAKKAILK;<br>DDDAKKAILKT; DDAKKAILKTN; DAKKAILKTNA; AKKAILKTNAD;<br>KKAILKTNADK; KAILKTNADKT; AILKTNADKTK; ILKTNADKTKG;<br>LKTNADKTKGA; KTNADKTKGAE; TNADKTKGAEE; NADKTKGAEEL;<br>ADKTKGAEELG; DKTKGAEELGK; KTKGAEELGKL; TKGAEELGKLF;<br>KGAEELGKLFK; GAEELGKLFKS; AEELGKLFKSV; EELGKLFKSVE;<br>ELGKLFKSVEG; LGKLFKSVEGL; GKLFKSVEGLV; KLFKSVEGLVK;<br>LFKSVEGLVKA; FKSVEGLVKAA; KSVEGLVKAAQ; SVEGLVKAAQE;<br>VEGLVKAAQEA; EGLVKAAQEAL; GLVKAAQEALT; LVKAAQEALTN;<br>VKAAQEALTNS; KAAQEALTNSV; AAQEALTNSVK; AQEALTNSVKE;<br>QEALTNSVKEL; EALTNSVKELT; ALTNSVKELTS; LTNSVKELTSP;<br>TNSVKELTSPV; NSVKELTSPVV; SVKELTSPVVA; VKELTSPVVAE;<br>KELTSPVVAES; ELTSPVVAESP; LTSPVVAESPK; TSPVVAESPKK;<br>SPVVAESPKKP; |
| AAC62927.1\| OspE-<br>related lipoprotein<br>[Borrelia garinii] | SEQ ID NO 13118-13799<br>8 mers:<br>MNKKMKMF; NKKMKMFI; KKMKMFII; KMKMFIIC; MKMFIICA; KMFIICAV;<br>MFIICAVF; FIICAVFV; IICAVFVL; ICAVFVLI; CAVFVLIS; AVFVLISS; |

```
VFVLISSC;  FVLISSCG;  VLISSCGN;  LISSCGNF;  ISSCGNFR;  SSCGNFRS;
SCGNFRSS;  CGNFRSSL;  GNFRSSLS;  NFRSSLSD;  FRSSLSDQ;  RSSLSDQG;
SSLSDQGS;  SLSDQGSL;  LSDQGSLS;  SDQGSLSD;  DQGSLSDQ;  QGSLSDQG;
GSLSDQGS;  SLSDQGSL;  LSDQGSLS;  SDQGSLSD;  DQGSLSDQ;  QGSLSDQG;
GSLSDQGS;  SLSDQGSL;  LSDQGSLS;  SDQGSLSD;  DQGSLSDQ;  QGSLSDQG;
GSLSDQGG;  SLSDQGGL;  LSDQGGLS;  SDQGGLSG;  DQGGLSGQ;  QGGLSGQA;
GGLSGQAS;  GLSGQASS;  LSGQASSD;  SGQASSDT;  GQASSDTI;  QASSDTIK;
ASSDTIKF;  SSDTIKFS;  SDTIKFSE;  DTIKFSEF;  TIKFSEFT;  IKFSEFTV;
KFSEFTVN;  FSEFTVNI;  SEFTVNIK;  EFTVNIKN;  FTVNIKNK;  TVNIKNKK;
VNIKNKKD;  NIKNKKDN;  IKNKKDNN;  KNKKDNNG;  NKKDNNGD;  KKDNNGDW;
KDNNGDWS;  DNNGDWSN;  NNGDWSNL;  NGDWSNLG;  GDWSNLGT;  DWSNLGTL;
WSNLGTLV;  SNLGTLVI;  NLGTLVIR;  LGTLVIRK;  GTLVIRKE;  TLVIRKEQ;
LVIRKEQD;  VIRKEQDG;  IRKEQDGV;  RKEQDGVE;  KEQDGVET;  EQDGVETG;
QDGVETGL;  DGVETGLN;  GVETGLNV;  VETGLNVI;  ETGLNVIG;  TGLNVIGT;
GLNVIGTI;  LNVIGTIN;  NVIGTING;  VIGTINGQ;  IGTINGQL;  GTINGQLR;
TINGQLRG;  INGQLRGH;  NGQLRGHS;  GQLRGHSA;  QLRGHSAT;  LRGHSATF;
RGHSATFF;  GHSATFFC;  HSATFFCI;  SATFFCIE;  ATFFCIEE;  TFFCIEEA;
FFCIEEAE;  FCIEEAEV;  CIEEAEVN;  IEEAEVNN;  EEAEVNNF;  EAEVNNFV;
AEVNNFVK;  EVNNFVKA;  VNNFVKAM;  NNFVKAMT;  NFVKAMTN;  FVKAMTNV;
VKAMTNVG;  KAMTNVGS;  AMTNVGSF;  MTNVGSFK;  TNVGSFKT;  NVGSFKTS;
VGSFKTSL;  GSFKTSLY;  SFKTSLYY;  FKTSLYYG;  KTSLYYGY;  TSLYYGYK;
SLYYGYKE;  LYYGYKEE;  YYGYKEEQ;  YGYKEEQS;  GYKEEQSS;  YKEEQSST;
KEEQSSTN;  EEQSSTNG;  EQSSTNGI;  QSSTNGIK;  SSTNGIKG;  STNGIKGK;
TNGIKGKE;  NGIKGKEI;  GIKGKEIT;  IKGKEITT;  KGKEITTK;  GKEITTKI;
KEITTKIE;  EITTKIET;  ITTKIETI;  TTKIETIN;  TKIETINN;  KIETINNS;
IETINNSE;  ETINNSEH;  TINNSEHI;  INNSEHIT;  NNSEHITF;  NSEHITFS;
SEHITFSG;  EHITFSGD;  HITFSGDK;  ITFSGDKI;

9 mers:
MNKKMKMFI;  NKKMKMFII;  KKMKMFIIC;  KMKMFIICA;  MKMFIICAV;
KMFIICAVF;  MFIICAVFV;  FIICAVFVL;  IICAVFVLI;  ICAVFVLIS;
CAVFVLISS;  AVFVLISSC;  VFVLISSCG;  FVLISSCGN;  VLISSCGNF;
LISSCGNFR;  ISSCGNFRS;  SSCGNFRSS;  SCGNFRSSL;  CGNFRSSLS;
GNFRSSLSD;  NFRSSLSDQ;  FRSSLSDQG;  RSSLSDQGS;  SSLSDQGSL;
SLSDQGSLS;  LSDQGSLSD;  SDQGSLSDQ;  DQGSLSDQG;  QGSLSDQGS;
GSLSDQGSL;  SLSDQGSLS;  LSDQGSLSD;  SDQGSLSDQ;  DQGSLSDQG;
QGSLSDQGS;  GSLSDQGSL;  SLSDQGSLS;  LSDQGSLSD;  SDQGSLSDQ;
DQGSLSDQG;  QGSLSDQGG;  GSLSDQGGL;  SLSDQGGLS;  LSDQGGLSG;
SDQGGLSGQ;  DQGGLSGQA;  QGGLSGQAS;  GGLSGQASS;  GLSGQASSD;
LSGQASSDT;  SGQASSDTI;  GQASSDTIK;  QASSDTIKF;  ASSDTIKFS;
SSDTIKFSE;  SDTIKFSEF;  DTIKFSEFT;  TIKFSEFTV;  IKFSEFTVN;
KFSEFTVNI;  FSEFTVNIK;  SEFTVNIKN;  EFTVNIKNK;  FTVNIKNKK;
TVNIKNKKD;  VNIKNKKDN;  NIKNKKDNN;  IKNKKDNNG;  KNKKDNNGD;
NKKDNNGDW;  KKDNNGDWS;  KDNNGDWSN;  DNNGDWSNL;  NNGDWSNLG;
NGDWSNLGT;  GDWSNLGTL;  DWSNLGTLV;  WSNLGTLVI;  SNLGTLVIR;
NLGTLVIRK;  LGTLVIRKE;  GTLVIRKEQ;  TLVIRKEQD;  LVIRKEQDG;
VIRKEQDGV;  IRKEQDGVE;  RKEQDGVET;  KEQDGVETG;  EQDGVETGL;
QDGVETGLN;  DGVETGLNV;  GVETGLNVI;  VETGLNVIG;  ETGLNVIGT;
TGLNVIGTI;  GLNVIGTIN;  LNVIGTING;  NVIGTINGQ;  VIGTINGQL;
IGTINGQLR;  GTINGQLRG;  TINGQLRGH;  INGQLRGHS;  NGQLRGHSA;
GQLRGHSAT;  QLRGHSATF;  LRGHSATFF;  RGHSATFFC;  GHSATFFCI;
HSATFFCIE;  SATFFCIEE;  ATFFCIEEA;  TFFCIEEAE;  FFCIEEAEV;
FCIEEAEVN;  CIEEAEVNN;  IEEAEVNNF;  EEAEVNNFV;  EAEVNNFVK;
AEVNNFVKA;  EVNNFVKAM;  VNNFVKAMT;  NNFVKAMTN;  NFVKAMTNV;
FVKAMTNVG;  VKAMTNVGS;  KAMTNVGSF;  AMTNVGSFK;  MTNVGSFKT;
TNVGSFKTS;  NVGSFKTSL;  VGSFKTSLY;  GSFKTSLYY;  SFKTSLYYG;
FKTSLYYGY;  KTSLYYGYK;  TSLYYGYKE;  SLYYGYKEE;  LYYGYKEEQ;
```

643

YYGYKEEQS; YGYKEEQSS; GYKEEQSST; YKEEQSSTN; KEEQSSTNG;
EEQSSTNGI; EQSSTNGIK; QSSTNGIKG; SSTNGIKGK; STNGIKGKE;
TNGIKGKEI; NGIKGKEIT; GIKGKEITT; IKGKEITTK; KGKEITTKI;
GKEITTKIE; KEITTKIET; EITTKIETI; ITTKIETIN; TTKIETINN;
TKIETINNS; KIETINNSE; IETINNSEH; ETINNSEHI; TINNSEHIT;
INNSEHITF; NNSEHITFS; NSEHITFSG; SEHITFSGD; EHITFSGDK;
HITFSGDKI;

10 mers:
MNKKMKMFII; NKKMKMFIIC; KKMKMFIICA; KMKMFIICAV; MKMFIICAVF;
KMFIICAVFV; MFIICAVFVL; FIICAVFVLI; IICAVFVLIS; ICAVFVLISS;
CAVFVLISSC; AVFVLISSCG; VFVLISSCGN; FVLISSCGNF; VLISSCGNFR;
LISSCGNFRS; ISSCGNFRSS; SSCGNFRSSL; SCGNFRSSLS; CGNFRSSLSD;
GNFRSSLSDQ; NFRSSLSDQG; FRSSLSDQGS; RSSLSDQGSL; SSLSDQGSLS;
SLSDQGSLSD; LSDQGSLSDQ; SDQGSLSDQG; DQGSLSDQGS; QGSLSDQGSL;
GSLSDQGSLS; SLSDQGSLSD; LSDQGSLSDQ; SDQGSLSDQG; DQGSLSDQGS;
QGSLSDQGSL; GSLSDQGSLS; SLSDQGSLSD; LSDQGSLSDQ; SDQGSLSDQG;
DQGSLSDQGG; QGSLSDQGGL; GSLSDQGGLS; SLSDQGGLSG; LSDQGGLSGQ;
SDQGGLSGQA; DQGGLSGQAS; QGGLSGQASS; GGLSGQASSD; GLSGQASSDT;
LSGQASSDTI; SGQASSDTIK; GQASSDTIKF; QASSDTIKFS; ASSDTIKFSE;
SSDTIKFSEF; SDTIKFSEFT; DTIKFSEFTV; TIKFSEFTVN; IKFSEFTVNI;
KFSEFTVNIK; FSEFTVNIKN; SEFTVNIKNK; EFTVNIKNKK; FTVNIKNKKD;
TVNIKNKKDN; VNIKNKKDNN; NIKNKKDNNG; IKNKKDNNGD; KNKKDNNGDW;
NKKDNNGDWS; KKDNNGDWSN; KDNNGDWSNL; DNNGDWSNLG; NNGDWSNLGT;
NGDWSNLGTL; GDWSNLGTLV; DWSNLGTLVI; WSNLGTLVIR; SNLGTLVIRK;
NLGTLVIRKE; LGTLVIRKEQ; GTLVIRKEQD; TLVIRKEQDG; LVIRKEQDGV;
VIRKEQDGVE; IRKEQDGVET; RKEQDGVETG; KEQDGVETGL; EQDGVETGLN;
QDGVETGLNV; DGVETGLNVI; GVETGLNVIG; VETGLNVIGT; ETGLNVIGTI;
TGLNVIGTIN; GLNVIGTING; LNVIGTINGQ; NVIGTINGQL; VIGTINGQLR;
IGTINGQLRG; GTINGQLRGH; TINGQLRGHS; INGQLRGHSA; NGQLRGHSAT;
GQLRGHSATF; QLRGHSATFF; LRGHSATFFC; RGHSATFFCI; GHSATFFCIE;
HSATFFCIEE; SATFFCIEEA; ATFFCIEEAE; TFFCIEEAEV; FFCIEEAEVN;
FCIEEAEVNN; CIEEAEVNNF; IEEAEVNNFV; EEAEVNNFVK; EAEVNNFVKA;
AEVNNFVKAM; EVNNFVKAMT; VNNFVKAMTN; NNFVKAMTNV; NFVKAMTNVG;
FVKAMTNVGS; VKAMTNVGSF; KAMTNVGSFK; AMTNVGSFKT; MTNVGSFKTS;
TNVGSFKTSL; NVGSFKTSLY; VGSFKTSLYY; GSFKTSLYYG; SFKTSLYYGY;
FKTSLYYGYK; KTSLYYGYKE; TSLYYGYKEE; SLYYGYKEEQ; LYYGYKEEQS;
YYGYKEEQSS; YGYKEEQSST; GYKEEQSSTN; YKEEQSSTNG; KEEQSSTNGI;
EEQSSTNGIK; EQSSTNGIKG; QSSTNGIKGK; SSTNGIKGKE; STNGIKGKEI;
TNGIKGKEIT; NGIKGKEITT; GIKGKEITTK; IKGKEITTKI; KGKEITTKIE;
GKEITTKIET; KEITTKIETI; EITTKIETIN; ITTKIETINN; TTKIETINNS;
TKIETINNSE; KIETINNSEH; IETINNSEHI; ETINNSEHIT; TINNSEHITF;
INNSEHITFS; NNSEHITFSG; NSEHITFSGD; SEHITFSGDK; EHITFSGDKI;

11 mers:
MNKKMKMFIIC; NKKMKMFIICA; KKMKMFIICAV; KMKMFIICAVF;
MKMFIICAVFV; KMFIICAVFVL; MFIICAVFVLI; FIICAVFVLIS;
IICAVFVLISS; ICAVFVLISSC; CAVFVLISSCG; AVFVLISSCGN;
VFVLISSCGNF; FVLISSCGNFR; VLISSCGNFRS; LISSCGNFRSS;
ISSCGNFRSSL; SSCGNFRSSLS; SCGNFRSSLSD; CGNFRSSLSDQ;
GNFRSSLSDQG; NFRSSLSDQGS; FRSSLSDQGSL; RSSLSDQGSLS;
SSLSDQGSLSD; SLSDQGSLSDQ; LSDQGSLSDQG; SDQGSLSDQGS;
DQGSLSDQGSL; QGSLSDQGSLS; GSLSDQGSLSD; SLSDQGSLSDQ;
LSDQGSLSDQG; SDQGSLSDQGS; DQGSLSDQGSL; QGSLSDQGSLS;
GSLSDQGSLSD; SLSDQGSLSDQ; LSDQGSLSDQG; SDQGSLSDQGG;
DQGSLSDQGGL; QGSLSDQGGLS; GSLSDQGGLSG; SLSDQGGLSGQ;
LSDQGGLSGQA; SDQGGLSGQAS; DQGGLSGQASS; QGGLSGQASSD;

| | |
|---|---|
| | GGLSGQASSDT; GLSGQASSDTI; LSGQASSDTIK; SGQASSDTIKF;<br>GQASSDTIKFS; QASSDTIKFSE; ASSDTIKFSEF; SSDTIKFSEFT;<br>SDTIKFSEFTV; DTIKFSEFTVN; TIKFSEFTVNI; IKFSEFTVNIK;<br>KFSEFTVNIKN; FSEFTVNIKNK; SEFTVNIKNKK; EFTVNIKNKKD;<br>FTVNIKNKKDN; TVNIKNKKDNN; VNIKNKKDNNG; NIKNKKDNNGD;<br>IKNKKDNNGDW; KNKKDNNGDWS; NKKDNNGDWSN; KKDNNGDWSNL;<br>KDNNGDWSNLG; DNNGDWSNLGT; NNGDWSNLGTL; NGDWSNLGTLV;<br>GDWSNLGTLVI; DWSNLGTLVIR; WSNLGTLVIRK; SNLGTLVIRKE;<br>NLGTLVIRKEQ; LGTLVIRKEQD; GTLVIRKEQDG; TLVIRKEQDGV;<br>LVIRKEQDGVE; VIRKEQDGVET; IRKEQDGVETG; RKEQDGVETGL;<br>KEQDGVETGLN; EQDGVETGLNV; QDGVETGLNVI; DGVETGLNVIG;<br>GVETGLNVIGT; VETGLNVIGTI; ETGLNVIGTIN; TGLNVIGTING;<br>GLNVIGTINGQ; LNVIGTINGQL; NVIGTINGQLR; VIGTINGQLRG;<br>IGTINGQLRGH; GTINGQLRGHS; TINGQLRGHSA; INGQLRGHSAT;<br>NGQLRGHSATF; GQLRGHSATFF; QLRGHSATFFC; LRGHSATFFCI;<br>RGHSATFFCIE; GHSATFFCIEE; HSATFFCIEEA; SATFFCIEEAE;<br>ATFFCIEEAEV; TFFCIEEAEVN; FFCIEEAEVNN; FCIEEAEVNNF;<br>CIEEAEVNNFV; IEEAEVNNFVK; EEAEVNNFVKA; EAEVNNFVKAM;<br>AEVNNFVKAMT; EVNNFVKAMTN; VNNFVKAMTNV; NNFVKAMTNVG;<br>NFVKAMTNVGS; FVKAMTNVGSF; VKAMTNVGSFK; KAMTNVGSFKT;<br>AMTNVGSFKTS; MTNVGSFKTSL; TNVGSFKTSLY; NVGSFKTSLYY;<br>VGSFKTSLYYG; GSFKTSLYYGY; SFKTSLYYGYK; FKTSLYYGYKE;<br>KTSLYYGYKEE; TSLYYGYKEEQ; SLYYGYKEEQS; LYYGYKEEQSS;<br>YYGYKEEQSST; YGYKEEQSSTN; GYKEEQSSTNG; YKEEQSSTNGI;<br>KEEQSSTNGIK; EEQSSTNGIKG; EQSSTNGIKGK; QSSTNGIKGKE;<br>SSTNGIKGKEI; STNGIKGKEIT; TNGIKGKEITT; NGIKGKEITTK;<br>GIKGKEITTKI; IKGKEITTKIE; KGKEITTKIET; GKEITTKIETI;<br>KEITTKIETIN; EITTKIETINN; ITTKIETINNS; TTKIETINNSE;<br>TKIETINNSEH; KIETINNSEHI; IETINNSEHIT; ETINNSEHITF;<br>TINNSEHITFS; INNSEHITFSG; NNSEHITFSGD; NSEHITFSGDK;<br>SEHITFSGDKI |
| CAA57806.1\| Outer surface protein G [Borrelia burgdorferi] | SEQ ID NO 13800-14549/<br>8 mers:<br>MNKKMKNL; NKKMKNLI; KKMKNLII; KMKNLIIC; MKNLIICA; KNLIICAV;<br>NLIICAVF; LIICAVFV; IICAVFVL; ICAVFVLI; CAVFVLII; AVFVLIIS;<br>VFVLIISC; FVLIISCK; VLIISCKI; LIISCKID; IISCKIDA; ISCKIDAS;<br>SCKIDASS; CKIDASSE; KIDASSED; IDASSEDL; DASSEDLK; ASSEDLKQ;<br>SSEDLKQN; SEDLKQNV; EDLKQNVK; DLKQNVKE; LKQNVKEK; KQNVKEKV;<br>QNVKEKVE; NVKEKVEG; VKEKVEGF; KEKVEGFL; EKVEGFLD; KVEGFLDK;<br>VEGFLDKE; EGFLDKEL; GFLDKELM; FLDKELMQ; LDKELMQG; DKELMQGD;<br>KELMQGDD; ELMQGDDP; LMQGDDPN; MQGDDPNN; QGDDPNNS; GDDPNNSL;<br>DDPNNSLF; DPNNSLFN; PNNSLFNP; NNSLFNPP; NSLFNPPP; SLFNPPPV;<br>LFNPPPVL; FNPPPVLP; NPPPVLPA; PPPVLPAS; PPVLPASS; PVLPASSH;<br>VLPASSHD; LPASSHDN; PASSHDNT; ASSHDNTP; SSHDNTPV; SHDNTPVL;<br>HDNTPVLK; DNTPVLKA; NTPVLKAV; TPVLKAVQ; PVLKAVQA; VLKAVQAK;<br>LKAVQAKD; KAVQAKDG; AVQAKDGG; VQAKDGGQ; QAKDGGQQ; AKDGGQQE;<br>KDGGQQEG; DGGQQEGK; GGQQEGKE; GQQEGKEE; QQEGKEEK; QEGKEEKE;<br>EGKEEKEK; GKEEKEKE; KEEKEKEI; EEKEKEIQ; EKEKEIQE; KEKEIQEL;<br>EKEIQELK; KEIQELKD; EIQELKDK; IQELKDKI; QELKDKID; ELKDKIDK;<br>LKDKIDKR; KDKIDKRK; DKIDKRKK; KIDKRKKE; IDKRKKEL; DKRKKELE;<br>KRKKELEE; RKKELEEA; KKELEEAR; KELEEARK; ELEEARKK; LEEARKKF;<br>EEARKKFQ; EARKKFQE; ARKKFQEF; RKKFQEFK; KKFQEFKE; KFQEFKEQ;<br>FQEFKEQV; QEFKEQVE; EFKEQVES; FKEQVESA; KEQVESAT; EQVESATG;<br>QVESATGE; VESATGES; ESATGEST; SATGESTE; ATGESTEK; TGESTEKV;<br>GESTEKVK; ESTEKVKK; STEKVKKQ; TEKVKKQG; EKVKKQGN; KVKKQGNI;<br>VKKQGNIG; KKQGNIGQ; KQGNIGQK; QGNIGQKA; GNIGQKAL; NIGQKALK; |

IGQKALKY; GQKALKYA; QKALKYAK; KALKYAKE; ALKYAKEL; LKYAKELG;
KYAKELGV; YAKELGVN; AKELGVNG; KELGVNGS; ELGVNGSY; LGVNGSYS;
GVNGSYSV; VNGSYSVN; NGSYSVND; GSYSVNDG; SYSVNDGT; YSVNDGTN;
SVNDGTNT; VNDGTNTN; NDGTNTND; DGTNTNDF; GTNTNDFV; TNTNDFVK;
NTNDFVKK; TNDFVKKV; NDFVKKVI; DFVKKVID; FVKKVIDD; VKKVIDDA;
KKVIDDAL; KVIDDALK; VIDDALKN; IDDALKNI; DDALKNIE; DALKNIEE;
ALKNIEEE; LKNIEEEL; KNIEEELE; NIEEELEK; IEEELEKL; EEELEKLA;
EELEKLAE; ELEKLAEP; LEKLAEPQ; EKLAEPQN; KLAEPQNI; LAEPQNIE;
AEPQNIED; EPQNIEDK; PQNIEDKK;

9 mers:
MNKKMKNLI; NKKMKNLII; KKMKNLIIC; KMKNLIICA; MKNLIICAV;
KNLIICAVF; NLIICAVFV; LIICAVFVL; IICAVFVLI; ICAVFVLII;
CAVFVLIIS; AVFVLIISC; VFVLIISCK; FVLIISCKI; VLIISCKID;
LIISCKIDA; IISCKIDAS; ISCKIDASS; SCKIDASSE; CKIDASSED;
KIDASSEDL; IDASSEDLK; DASSEDLKQ; ASSEDLKQN; SSEDLKQNV;
SEDLKQNVK; EDLKQNVKE; DLKQNVKEK; LKQNVKEKV; KQNVKEKVE;
QNVKEKVEG; NVKEKVEGF; VKEKVEGFL; KEKVEGFLD; EKVEGFLDK;
KVEGFLDKE; VEGFLDKEL; EGFLDKELM; GFLDKELMQ; FLDKELMQG;
LDKELMQGD; DKELMQGDD; KELMQGDDP; ELMQGDDPN; LMQGDDPNN;
MQGDDPNNS; QGDDPNNSL; GDDPNNSLF; DDPNNSLFN; DPNNSLFNP;
PNNSLFNPP; NNSLFNPPP; NSLFNPPPV; SLFNPPPVL; LFNPPPVLP;
FNPPPVLPA; NPPPVLPAS; PPPVLPASS; PPVLPASSH; PVLPASSHD;
VLPASSHDN; LPASSHDNT; PASSHDNTP; ASSHDNTPV; SSHDNTPVL;
SHDNTPVLK; HDNTPVLKA; DNTPVLKAV; NTPVLKAVQ; TPVLKAVQA;
PVLKAVQAK; VLKAVQAKD; LKAVQAKDG; KAVQAKDGG; AVQAKDGGQ;
VQAKDGGQQ; QAKDGGQQE; AKDGGQQEG; KDGGQQEGK; DGGQQEGKE;
GGQQEGKEE; GQQEGKEEK; QQEGKEEKE; QEGKEEKEK; EGKEEKEKE;
GKEEKEKEI; KEEKEKEIQ; EEKEKEIQE; EKEKEIQEL; KEKEIQELK;
EKEIQELKD; KEIQELKDK; EIQELKDKI; IQELKDKID; QELKDKIDK;
ELKDKIDKR; LKDKIDKRK; KDKIDKRKK; DKIDKRKKE; KIDKRKKEL;
IDKRKKELE; DKRKKELEE; KRKKELEEA; RKKELEEAR; KKELEEARK;
KELEEARKK; ELEEARKKF; LEEARKKFQ; EEARKKFQE; EARKKFQEF;
ARKKFQEFK; RKKFQEFKE; KKFQEFKEQ; KFQEFKEQV; FQEFKEQVE;
QEFKEQVES; EFKEQVESA; FKEQVESAT; KEQVESATG; EQVESATGE;
QVESATGES; VESATGEST; ESATGESTE; SATGESTEK; ATGESTEKV;
TGESTEKVK; GESTEKVKK; ESTEKVKKQ; STEKVKKQG; TEKVKKQGN;
EKVKKQGNI; KVKKQGNIG; VKKQGNIGQ; KKQGNIGQK; KQGNIGQKA;
QGNIGQKAL; GNIGQKALK; NIGQKALKY; IGQKALKYA; GQKALKYAK;
QKALKYAKE; KALKYAKEL; ALKYAKELG; LKYAKELGV; KYAKELGVN;
YAKELGVNG; AKELGVNGS; KELGVNGSY; ELGVNGSYS; LGVNGSYSV;
GVNGSYSVN; VNGSYSVND; NGSYSVNDG; GSYSVNDGT; SYSVNDGTN;
YSVNDGTNT; SVNDGTNTN; VNDGTNTND; NDGTNTNDF; DGTNTNDFV;
GTNTNDFVK; TNTNDFVKK; NTNDFVKKV; TNDFVKKVI; NDFVKKVID;
DFVKKVIDD; FVKKVIDDA; VKKVIDDAL; KKVIDDALK; KVIDDALKN;
VIDDALKNI; IDDALKNIE; DDALKNIEE; DALKNIEEE; ALKNIEEEL;
LKNIEEELE; KNIEEELEK; NIEEELEKL; IEEELEKLA; EEELEKLAE;
EELEKLAEP; ELEKLAEPQ; LEKLAEPQN; EKLAEPQNI; KLAEPQNIE;
LAEPQNIED; AEPQNIEDK; EPQNIEDKK;

10 mers:
MNKKMKNLII; NKKMKNLIIC; KKMKNLIICA; KMKNLIICAV; MKNLIICAVF;
KNLIICAVFV; NLIICAVFVL; LIICAVFVLI; IICAVFVLII; ICAVFVLIIS;
CAVFVLIISC; AVFVLIISCK; VFVLIISCKI; FVLIISCKID; VLIISCKIDA;
LIISCKIDAS; IISCKIDASS; ISCKIDASSE; SCKIDASSED; CKIDASSEDL;
KIDASSEDLK; IDASSEDLKQ; DASSEDLKQN; ASSEDLKQNV; SSEDLKQNVK;
SEDLKQNVKE; EDLKQNVKEK; DLKQNVKEKV; LKQNVKEKVE; KQNVKEKVEG;

QNVKEKVEGF; NVKEKVEGFL; VKEKVEGFLD; KEKVEGFLDK; EKVEGFLDKE;
KVEGFLDKEL; VEGFLDKELM; EGFLDKELMQ; GFLDKELMQG; FLDKELMQGD;
LDKELMQGDD; DKELMQGDDP; KELMQGDDPN; ELMQGDDPNN; LMQGDDPNNS;
MQGDDPNNSL; QGDDPNNSLF; GDDPNNSLFN; DDPNNSLFNP; DPNNSLFNPP;
PNNSLFNPPP; NNSLFNPPPV; NSLFNPPPVL; SLFNPPPVLP; LFNPPPVLPA;
FNPPPVLPAS; NPPPVLPASS; PPPVLPASSH; PPVLPASSHD; PVLPASSHDN;
VLPASSHDNT; LPASSHDNTP; PASSHDNTPV; ASSHDNTPVL; SSHDNTPVLK;
SHDNTPVLKA; HDNTPVLKAV; DNTPVLKAVQ; NTPVLKAVQA; TPVLKAVQAK;
PVLKAVQAKD; VLKAVQAKDG; LKAVQAKDGG; KAVQAKDGGQ; AVQAKDGGQQ;
VQAKDGGQQE; QAKDGGQQEG; AKDGGQQEGK; KDGGQQEGKE; DGGQQEGKEE;
GGQQEGKEEK; GQQEGKEEKE; QQEGKEEKEK; QEGKEEKEKE; EGKEEKEKEI;
GKEEKEKEIQ; KEEKEKEIQE; EEKEKEIQEL; EKEKEIQELK; KEKEIQELKD;
EKEIQELKDK; KEIQELKDKI; EIQELKDKID; IQELKDKIDK; QELKDKIDKR;
ELKDKIDKRK; LKDKIDKRKK; KDKIDKRKKE; DKIDKRKKEL; KIDKRKKELE;
IDKRKKELEE; DKRKKELEEA; KRKKELEEAR; RKKELEEARK; KKELEEARKK;
KELEEARKKF; ELEEARKKFQ; LEEARKKFQE; EEARKKFQEF; EARKKFQEFK;
ARKKFQEFKE; RKKFQEFKEQ; KKFQEFKEQV; KFQEFKEQVE; FQEFKEQVES;
QEFKEQVESA; EFKEQVESAT; FKEQVESATG; KEQVESATGE; EQVESATGES;
QVESATGEST; VESATGESTE; ESATGESTEK; SATGESTEKV; ATGESTEKVK;
TGESTEKVKK; GESTEKVKKQ; ESTEKVKKQG; STEKVKKQGN; TEKVKKQGNI;
EKVKKQGNIG; KVKKQGNIGQ; VKKQGNIGQK; KKQGNIGQKA; KQGNIGQKAL;
QGNIGQKALK; GNIGQKALKY; NIGQKALKYA; IGQKALKYAK; GQKALKYAKE;
QKALKYAKEL; KALKYAKELG; ALKYAKELGV; LKYAKELGVN; KYAKELGVNG;
YAKELGVNGS; AKELGVNGSY; KELGVNGSYS; ELGVNGSYSV; LGVNGSYSVN;
GVNGSYSVND; VNGSYSVNDG; NGSYSVNDGT; GSYSVNDGTN; SYSVNDGTNT;
YSVNDGTNTN; SVNDGTNTND; VNDGTNTNDF; NDGTNTNDFV; DGTNTNDFVK;
GTNTNDFVKK; TNTNDFVKKV; NTNDFVKKVI; TNDFVKKVID; NDFVKKVIDD;
DFVKKVIDDA; FVKKVIDDAL; VKKVIDDALK; KKVIDDALKN; KVIDDALKNI;
VIDDALKNIE; IDDALKNIEE; DDALKNIEEE; DALKNIEEEL; ALKNIEEELE;
LKNIEEELEK; KNIEEELEKL; NIEEELEKLA; IEEELEKLAE; EEELEKLAEP;
EELEKLAEPQ; ELEKLAEPQN; LEKLAEPQNI; EKLAEPQNIE; KLAEPQNIED;
LAEPQNIEDK; AEPQNIEDKK

11 mers:
MNKKMKNLIIC; NKKMKNLIICA; KKMKNLIICAV; KMKNLIICAVF;
MKNLIICAVFV; KNLIICAVFVL; NLIICAVFVLI; LIICAVFVLII;
IICAVFVLIIS; ICAVFVLIISC; CAVFVLIISCK; AVFVLIISCKI;
VFVLIISCKID; FVLIISCKIDA; VLIISCKIDAS; LIISCKIDASS;
IISCKIDASSE; ISCKIDASSED; SCKIDASSEDL; CKIDASSEDLK;
KIDASSEDLKQ; IDASSEDLKQN; DASSEDLKQNV; ASSEDLKQNVK;
SSEDLKQNVKE; SEDLKQNVKEK; EDLKQNVKEKV; DLKQNVKEKVE;
LKQNVKEKVEG; KQNVKEKVEGF; QNVKEKVEGFL; NVKEKVEGFLD;
VKEKVEGFLDK; KEKVEGFLDKE; EKVEGFLDKEL; KVEGFLDKELM;
VEGFLDKELMQ; EGFLDKELMQG; GFLDKELMQGD; FLDKELMQGDD;
LDKELMQGDDP; DKELMQGDDPN; KELMQGDDPNN; ELMQGDDPNNS;
LMQGDDPNNSL; MQGDDPNNSLF; QGDDPNNSLFN; GDDPNNSLFNP;
DDPNNSLFNPP; DPNNSLFNPPP; PNNSLFNPPPV; NNSLFNPPPVL;
NSLFNPPPVLP; SLFNPPPVLPA; LFNPPPVLPAS; FNPPPVLPASS;
NPPPVLPASSH; PPPVLPASSHD; PPVLPASSHDN; PVLPASSHDNT;
VLPASSHDNTP; LPASSHDNTPV; PASSHDNTPVL; ASSHDNTPVLK;
SSHDNTPVLKA; SHDNTPVLKAV; HDNTPVLKAVQ; DNTPVLKAVQA;
NTPVLKAVQAK; TPVLKAVQAKD; PVLKAVQAKDG; VLKAVQAKDGG;
LKAVQAKDGGQ; KAVQAKDGGQQ; AVQAKDGGQQE; VQAKDGGQQEG;
QAKDGGQQEGK; AKDGGQQEGKE; KDGGQQEGKEE; DGGQQEGKEEK;
GGQQEGKEEKE; GQQEGKEEKEK; QQEGKEEKEKE; QEGKEEKEKEI;
EGKEEKEKEIQ; GKEEKEKEIQE; KEEKEKEIQEL; EEKEKEIQELK;
EKEKEIQELKD; KEKEIQELKDK; EKEIQELKDKI; KEIQELKDKID;

647

| | |
|---|---|
| | EIQELKDKIDK; IQELKDKIDKR; QELKDKIDKRK; ELKDKIDKRKK; LKDKIDKRKKE; KDKIDKRKKEL; DKIDKRKKELE; KIDKRKKELEE; IDKRKKELEEA; DKRKKELEEAR; KRKKELEEARK; RKKELEEARKK; KKELEEARKKF; KELEEARKKFQ; ELEEARKKFQE; LEEARKKFQEF; EEARKKFQEFK; EARKKFQEFKE; ARKKFQEFKEQ; RKKFQEFKEQV; KKFQEFKEQVE; KFQEFKEQVES; FQEFKEQVESA; QEFKEQVESAT; EFKEQVESATG; FKEQVESATGE; KEQVESATGES; EQVESATGEST; QVESATGESTE; VESATGESTEK; ESATGESTEKV; SATGESTEKVK; ATGESTEKVKK; TGESTEKVKKQ; GESTEKVKKQG; ESTEKVKKQGN; STEKVKKQGNI; TEKVKKQGNIG; EKVKKQGNIGQ; KVKKQGNIGQK; VKKQGNIGQKA; KKQGNIGQKAL; KQGNIGQKALK; QGNIGQKALKY; GNIGQKALKYA; NIGQKALKYAK; IGQKALKYAKE; GQKALKYAKEL; QKALKYAKELG; KALKYAKELGV; ALKYAKELGVN; LKYAKELGVNG; KYAKELGVNGS; YAKELGVNGSY; AKELGVNGSYS; KELGVNGSYSV; ELGVNGSYSVN; LGVNGSYSVND; GVNGSYSVNDG; VNGSYSVNDGT; NGSYSVNDGTN; GSYSVNDGTNT; SYSVNDGTNTN; YSVNDGTNTND; SVNDGTNTNDF; VNDGTNTNDFV; NDGTNTNDFVK; DGTNTNDFVKK; GTNTNDFVKKV; TNTNDFVKKVI; NTNDFVKKVID; TNDFVKKVIDD; NDFVKKVIDDA; DFVKKVIDDAL; FVKKVIDDALK; VKKVIDDALKN; KKVIDDALKNI; KVIDDALKNIE; VIDDALKNIEE; IDDALKNIEEE; DDALKNIEEEL; DALKNIEEELE; ALKNIEEELEK; LKNIEEELEKL; KNIEEELEKLA; NIEEELEKLAE; IEEELEKLAEP; EEELEKLAEPQ; EEELEKLAEPQN; ELEKLAEPQNI; LEKLAEPQNIE; EKLAEPQNIED; KLAEPQNIEDK; LAEPQNIEDKK; |
| AAC44770.1\| FlaA protein (Borrelia burgdorferi) | SEQ ID NO 14550-15879/ 8 mers: MKRKAKSI; KRKAKSIL; RKAKSILF; KAKSILFF; AKSILFFL; KSILFFLL; SILFFLLS; ILFFLLST; LFFLLSTV; FFLLSTVL; FLLSTVLF; LLSTVLFA; LSTVLFAQ; STVLFAQE; TVLFAQET; VLFAQETD; LFAQETDG; FAQETDGL; AQETDGLA; QETDGLAE; ETDGLAEG; TDGLAEGS; DGLAEGSK; GLAEGSKR; LAEGSKRA; AEGSKRAE; EGSKRAEP; GSKRAEPG; SKRAEPGE; KRAEPGEL; RAEPGELV; AEPGELVL; EPGELVLD; PGELVLDF; GELVLDFA; ELVLDFAE; LVLDFAEL; VLDFAELA; LDFAELAR; DFAELARD; FAELARDP; AELARDPS; ELARDPSS; LARDPSST; ARDPSSTR; RDPSSTRL; DPSSTRLD; PSSTRLDL; SSTRLDLT; STRLDLTN; TRLDLTNY; RLDLTNYV; LDLTNYVD; DLTNYVDY; LTNYVDYV; TNYVDYVY; NYVDYVYS; YVDYVYSG; VDYVYSGA; DYVYSGAS; YVYSGASG; VYSGASGI; YSGASGIV; SGASGIVK; GASGIVKP; ASGIVKPE; SGIVKPED; GIVKPEDM; IVKPEDMV; VKPEDMVV; KPEDMVVD; PEDMVVDL; EDMVVDLG; DMVVDLGI; MVVDLGIN; VVDLGINN; VDLGINNW; DLGINNWS; LGINNWSV; GINNWSVL; INNWSVLL; NNWSVLLT; NWSVLLTP; WSVLLTPS; SVLLTPSA; VLLTPSAR; LLTPSARL; LTPSARLQ; TPSARLQA; PSARLQAY; SARLQAYV; ARLQAYVK; RLQAYVKN; LQAYVKNS; QAYVKNSV; AYVKNSVV; YVKNSVVA; VKNSVVAP; KNSVVAPA; NSVVAPAV; SVVAPAVV; VVAPAVVK; VAPAVVKS; APAVVKSE; PAVVKSES; AVVKSESK; VVKSESKR; VKSESKRY; KSESKRYA; SESKRYAG; ESKRYAGD; SKRYAGDT; KRYAGDTI; RYAGDTIL; YAGDTILG; AGDTILGV; GDTILGVR; DTILGVRV; TILGVRVL; ILGVRVLF; LGVRVLFP; GVRVLFPS; VRVLFPSY; RVLFPSYS; VLFPSYSQ; LFPSYSQS; FPSYSQSS; PSYSQSSA; SYSQSSAM; YSQSSAMI; SQSSAMIM; QSSAMIMP; SSAMIMPP; SAMIMPPF; AMIMPPFK; MIMPPFKI; IMPPFKIP; MPPFKIPF; PPFKIPFY; PFKIPFYS; FKIPFYSG; KIPFYSGE; IPFYSGES; PFYSGESG; FYSGESGN; YSGESGNQ; SGESGNQF; GESGNQFL; ESGNQFLG; SGNQFLGK; GNQFLGKG; NQFLGKGL; QFLGKGLI; FLGKGLID; LGKGLIDN; GKGLIDNI; KGLIDNIK; GLIDNIKT; LIDNIKTM; IDNIKTMK; DNIKTMKE; NIKTMKEI; IKTMKEIK; KTMKEIKV; TMKEIKVS; MKEIKVSV; KEIKVSVY; EIKVSVYS; IKVSVYSL; KVSVYSLG; VSVYSLGY; SVYSLGYE; VYSLGYEI; YSLGYEID; SLGYEIDL; LGYEIDLE; GYEIDLEV; YEIDLEVL; EIDLEVLF; IDLEVLFE; |

DLEVLFED; LEVLFEDM; EVLFEDMN; VLFEDMNG; LFEDMNGM; FEDMNGME;
EDMNGMEY; DMNGMEYA; MNGMEYAY; NGMEYAYS; GMEYAYSM; MEYAYSMG;
EYAYSMGT; YAYSMGTL; AYSMGTLK; YSMGTLKF; SMGTLKFK; MGTLKFKG;
GTLKFKGW; TLKFKGWA; LKFKGWAD; KFKGWADL; FKGWADLI; KGWADLIW;
GWADLIWS; WADLIWSN; ADLIWSNP; DLIWSNPN; LIWSNPNY; IWSNPNYI;
WSNPNYIP; SNPNYIPN; NPNYIPNI; PNYIPNIS; NYIPNISS; YIPNISSR;
IPNISSRI; PNISSRII; NISSRIIK; ISSRIIKD; SSRIIKDD; SRIIKDDV;
RIIKDDVP; IIKDDVPN; IKDDVPNY; KDDVPNYP; DDVPNYPL; DVPNYPLA;
VPNYPLAS; PNYPLASS; NYPLASSK; YPLASSKM; PLASSKMR; LASSKMRF;
ASSKMRFK; SSKMRFKA; SKMRFKAF; KMRFKAFR; MRFKAFRV; RFKAFRVS;
FKAFRVSK; KAFRVSKS; AFRVSKSH; FRVSKSHS; RVSKSHSS; VSKSHSSK;
SKSHSSKV; KSHSSKVK; SHSSKVKN; HSSKVKNF; SSKVKNFI; SKVKNFIF;
KVKNFIFY; VKNFIFYV; KNFIFYVK; NFIFYVKD; FIFYVKDL; IFYVKDLR;
FYVKDLRV; YVKDLRVL; VKDLRVLY; KDLRVLYD; DLRVLYDK; LRVLYDKL;
RVLYDKLS; VLYDKLSV; LYDKLSVS; YDKLSVSI; DKLSVSID; KLSVSIDS;
LSVSIDSD; SVSIDSDI; VSIDSDID; SIDSDIDS; IDSDIDSE; DSDIDSES;
SDIDSESV; DIDSESVF; IDSESVFK; DSESVFKV; SESVFKVY; ESVFKVYE;
SVFKVYET; VFKVYETS; FKVYETSG; KVYETSGT; VYETSGTE; YETSGTES;
ETSGTESL; TSGTESLR; SGTESLRK; GTESLRKL; TESLRKLK; ESLRKLKA;
SLRKLKAH; LRKLKAHE; RKLKAHET; KLKAHETF; LKAHETFK; KAHETFKR;
AHETFKRV; HETFKRVL; ETFKRVLK; TFKRVLKL; FKRVLKLR; KRVLKLRE;
RVLKLREK; VLKLREKI; LKLREKIS; KLREKISI; LREKISIA; REKISIAE;
EKISIAEG; KISIAEGS; ISIAEGSF; SIAEGSFQ; IAEGSFQN; AEGSFQNF;
EGSFQNFV; GSFQNFVE; SFQNFVEK; FQNFVEKI; QNFVEKIE; NFVEKIES;
FVEKIESE; VEKIESEK; EKIESEKP; KIESEKPE; IESEKPEE; ESEKPEES;
SEKPEESS; EKPEESSP; KPEESSPK; PEESSPKN

9 mers:
MKRKAKSIL; KRKAKSILF; RKAKSILFF; KAKSILFFL; AKSILFFLL;
KSILFFLLS; SILFFLLST; ILFFLLSTV; LFFLLSTVL; FFLLSTVLF;
FLLSTVLFA; LLSTVLFAQ; LSTVLFAQE; STVLFAQET; TVLFAQETD;
VLFAQETDG; LFAQETDGL; FAQETDGLA; AQETDGLAE; QETDGLAEG;
ETDGLAEGS; TDGLAEGSK; DGLAEGSKR; GLAEGSKRA; LAEGSKRAE;
AEGSKRAEP; EGSKRAEPG; GSKRAEPGE; SKRAEPGEL; KRAEPGELV;
RAEPGELVL; AEPGELVLD; EPGELVLDF; PGELVLDFA; GELVLDFAE;
ELVLDFAEL; LVLDFAELA; VLDFAELAR; LDFAELARD; DFAELARDP;
FAELARDPS; AELARDPSS; ELARDPSST; LARDPSSTR; ARDPSSTRL;
RDPSSTRLD; DPSSTRLDL; PSSTRLDLT; SSTRLDLTN; STRLDLTNY;
TRLDLTNYV; RLDLTNYVD; LDLTNYVDY; DLTNYVDYV; LTNYVDYVY;
TNYVDYVYS; NYVDYVYSG; YVDYVYSGA; VDYVYSGAS; DYVYSGASG;
YVYSGASGI; VYSGASGIV; YSGASGIVK; SGASGIVKP; GASGIVKPE;
ASGIVKPED; SGIVKPEDM; GIVKPEDMV; IVKPEDMVV; VKPEDMVVD;
KPEDMVVDL; PEDMVVDLG; EDMVVDLGI; DMVVDLGIN; MVVDLGINN;
VVDLGINNW; VDLGINNWS; DLGINNWSV; LGINNWSVL; GINNWSVLL;
INNWSVLLT; NNWSVLLTP; NWSVLLTPS; WSVLLTPSA; SVLLTPSAR;
VLLTPSARL; LLTPSARLQ; LTPSARLQA; TPSARLQAY; PSARLQAYV;
SARLQAYVK; ARLQAYVKN; RLQAYVKNS; LQAYVKNSV; QAYVKNSVV;
AYVKNSVVA; YVKNSVVAP; VKNSVVAPA; KNSVVAPAV; NSVVAPAVV;
SVVAPAVVK; VVAPAVVKS; VAPAVVKSE; APAVVKSES; PAVVKSESK;
AVVKSESKR; VVKSESKRY; VKSESKRYA; KSESKRYAG; SESKRYAGD;
ESKRYAGDT; SKRYAGDTI; KRYAGDTIL; RYAGDTILG; YAGDTILGV;
AGDTILGVR; GDTILGVRV; DTILGVRVL; TILGVRVLF; ILGVRVLFP;
LGVRVLFPS; GVRVLFPSY; VRVLFPSYS; RVLFPSYSQ; VLFPSYSQS;
LFPSYSQSS; FPSYSQSSA; PSYSQSSAM; SYSQSSAMI; YSQSSAMIM;
SQSSAMIMP; QSSAMIMPP; SSAMIMPPF; SAMIMPPFK; AMIMPPFKI;
MIMPPFKIP; IMPPFKIPF; MPPFKIPFY; PPFKIPFYS; PFKIPFYSG;
FKIPFYSGE; KIPFYSGES; IPFYSGESG; PFYSGESGN; FYSGESGNQ;

YSGESGNQF; SGESGNQFL; GESGNQFLG; ESGNQFLGK; SGNQFLGKG;
GNQFLGKGL; NQFLGKGLI; QFLGKGLID; FLGKGLIDN; LGKGLIDNI;
GKGLIDNIK; KGLIDNIKT; GLIDNIKTM; LIDNIKTMK; IDNIKTMKE;
DNIKTMKEI; NIKTMKEIK; IKTMKEIKV; KTMKEIKVS; TMKEIKVSV;
MKEIKVSVY; KEIKVSVYS; EIKVSVYSL; IKVSVYSLG; KVSVYSLGY;
VSVYSLGYE; SVYSLGYEI; VYSLGYEID; YSLGYEIDL; SLGYEIDLE;
LGYEIDLEV; GYEIDLEVL; YEIDLEVLF; EIDLEVLFE; IDLEVLFED;
DLEVLFEDM; LEVLFEDMN; EVLFEDMNG; VLFEDMNGM; LFEDMNGME;
FEDMNGMEY; EDMNGMEYA; DMNGMEYAY; MNGMEYAYS; NGMEYAYSM;
GMEYAYSMG; MEYAYSMGT; EYAYSMGTL; YAYSMGTLK; AYSMGTLKF;
YSMGTLKFK; SMGTLKFKG; MGTLKFKGW; GTLKFKGWA; TLKFKGWAD;
LKFKGWADL; KFKGWADLI; FKGWADLIW; KGWADLIWS; GWADLIWSN;
WADLIWSNP; ADLIWSNPN; DLIWSNPNY; LIWSNPNYI; IWSNPNYIP;
WSNPNYIPN; SNPNYIPNI; NPNYIPNIS; PNYIPNISS; NYIPNISSR;
YIPNISSRI; IPNISSRII; PNISSRIIK; NISSRIIKD; ISSRIIKDD;
SSRIIKDDV; SRIIKDDVP; RIIKDDVPN; IIKDDVPNY; IKDDVPNYP;
KDDVPNYPL; DDVPNYPLA; DVPNYPLAS; VPNYPLASS; PNYPLASSK;
NYPLASSKM; YPLASSKMR; PLASSKMRF; LASSKMRFK; ASSKMRFKA;
SSKMRFKAF; SKMRFKAFR; KMRFKAFRV; MRFKAFRVS; RFKAFRVSK;
FKAFRVSKS; KAFRVSKSH; AFRVSKSHS; FRVSKSHSS; RVSKSHSSK;
VSKSHSSKV; SKSHSSKVK; KSHSSKVKN; SHSSKVKNF; HSSKVKNFI;
SSKVKNFIF; SKVKNFIFY; KVKNFIFYV; VKNFIFYVK; KNFIFYVKD;
NFIFYVKDL; FIFYVKDLR; IFYVKDLRV; FYVKDLRVL; YVKDLRVLY;
VKDLRVLYD; KDLRVLYDK; DLRVLYDKL; LRVLYDKLS; RVLYDKLSV;
VLYDKLSVS; LYDKLSVSI; YDKLSVSID; DKLSVSIDS; KLSVSIDSD;
LSVSIDSDI; SVSIDSDID; VSIDSDIDS; SIDSDIDSE; IDSDIDSES;
DSDIDSESV; SDIDSESVF; DIDSESVFK; IDSESVFKV; DSESVFKVY;
SESVFKVYE; ESVFKVYET; SVFKVYETS; VFKVYETSG; FKVYETSGT;
KVYETSGTE; VYETSGTES; YETSGTESL; ETSGTESLR; TSGTESLRK;
SGTESLRKL; GTESLRKLK; TESLRKLKA; ESLRKLKAH; SLRKLKAHE;
LRKLKAHET; RKLKAHETF; KLKAHETFK; LKAHETFKR; KAHETFKRV;
AHETFKRVL; HETFKRVLK; ETFKRVLKL; TFKRVLKLR; FKRVLKLRE;
KRVLKLREK; RVLKLREKI; VLKLREKIS; LKLREKISI; KLREKISIA;
LREKISIAE; REKISIAEG; EKISIAEGS; KISIAEGSF; ISIAEGSFQ;
SIAEGSFQN; IAEGSFQNF; AEGSFQNFV; EGSFQNFVE; GSFQNFVEK;
SFQNFVEKI; FQNFVEKIE; QNFVEKIES; NFVEKIESE; FVEKIESEK;
VEKIESEKP; EKIESEKPE; KIESEKPEE; IESEKPEES; ESEKPEESS;
SEKPEESSP; EKPEESSPK; KPEESSPKN;

10 mers:
MKRKAKSILF; KRKAKSILFF; RKAKSILFFL; KAKSILFFLL; AKSILFFLLS;
KSILFFLLST; SILFFLLSTV; ILFFLLSTVL; LFFLLSTVLF; FFLLSTVLFA;
FLLSTVLFAQ; LLSTVLFAQE; LSTVLFAQET; STVLFAQETD; TVLFAQETDG;
VLFAQETDGL; LFAQETDGLA; FAQETDGLAE; AQETDGLAEG; QETDGLAEGS;
ETDGLAEGSK; TDGLAEGSKR; DGLAEGSKRA; GLAEGSKRAE; LAEGSKRAEP;
AEGSKRAEPG; EGSKRAEPGE; GSKRAEPGEL; SKRAEPGELV; KRAEPGELVL;
RAEPGELVLD; AEPGELVLDF; EPGELVLDFA; PGELVLDFAE; GELVLDFAEL;
ELVLDFAELA; LVLDFAELAR; VLDFAELARD; LDFAELARDP; DFAELARDPS;
FAELARDPSS; AELARDPSST; ELARDPSSTR; LARDPSSTRL; ARDPSSTRLD;
RDPSSTRLDL; DPSSTRLDLT; PSSTRLDLTN; SSTRLDLTNY; STRLDLTNYV;
TRLDLTNYVD; RLDLTNYVDY; LDLTNYVDYV; DLTNYVDYVY; LTNYVDYVYS;
TNYVDYVYSG; NYVDYVYSGA; YVDYVYSGAS; VDYVYSGASG; DYVYSGASGI;
YVYSGASGIV; VYSGASGIVK; YSGASGIVKP; SGASGIVKPE; GASGIVKPED;
ASGIVKPEDM; SGIVKPEDMV; GIVKPEDMVV; IVKPEDMVVD; VKPEDMVVDL;
KPEDMVVDLG; PEDMVVDLGI; EDMVVDLGIN; DMVVDLGINN; MVVDLGINNW;
VVDLGINNWS; VDLGINNWSV; DLGINNWSVL; LGINNWSVLL; GINNWSVLLT;
INNWSVLLTP; NNWSVLLTPS; NWSVLLTPSA; WSVLLTPSAR; SVLLTPSARL;

VLLTPSARLQ; LLTPSARLQA; LTPSARLQAY; TPSARLQAYV; PSARLQAYVK;
SARLQAYVKN; ARLQAYVKNS; RLQAYVKNSV; LQAYVKNSVV; QAYVKNSVVA;
AYVKNSVVAP; YVKNSVVAPA; VKNSVVAPAV; KNSVVAPAVV; NSVVAPAVVK;
SVVAPAVVKS; VVAPAVVKSE; VAPAVVKSES; APAVVKSESK; PAVVKSESKR;
AVVKSESKRY; VVKSESKRYA; VKSESKRYAG; KSESKRYAGD; SESKRYAGDT;
ESKRYAGDTI; SKRYAGDTIL; KRYAGDTILG; RYAGDTILGV; YAGDTILGVR;
AGDTILGVRV; GDTILGVRVL; DTILGVRVLF; TILGVRVLFP; ILGVRVLFPS;
LGVRVLFPSY; GVRVLFPSYS; VRVLFPSYSQ; RVLFPSYSQS; VLFPSYSQSS;
LFPSYSQSSA; FPSYSQSSAM; PSYSQSSAMI; SYSQSSAMIM; YSQSSAMIMP;
SQSSAMIMPP; QSSAMIMPPF; SSAMIMPPFK; SAMIMPPFKI; AMIMPPFKIP;
MIMPPFKIPF; IMPPFKIPFY; MPPFKIPFYS; PPFKIPFYSG; PFKIPFYSGE;
FKIPFYSGES; KIPFYSGESG; IPFYSGESGN; PFYSGESGNQ; FYSGESGNQF;
YSGESGNQFL; SGESGNQFLG; GESGNQFLGK; ESGNQFLGKG; SGNQFLGKGL;
GNQFLGKGLI; NQFLGKGLID; QFLGKGLIDN; FLGKGLIDNI; LGKGLIDNIK;
GKGLIDNIKT; KGLIDNIKTM; GLIDNIKTMK; LIDNIKTMKE; IDNIKTMKEI;
DNIKTMKEIK; NIKTMKEIKV; IKTMKEIKVS; KTMKEIKVSV; TMKEIKVSVY;
MKEIKVSVYS; KEIKVSVYSL; EIKVSVYSLG; IKVSVYSLGY; KVSVYSLGYE;
VSVYSLGYEI; SVYSLGYEID; VYSLGYEIDL; YSLGYEIDLE; SLGYEIDLEV;
LGYEIDLEVL; GYEIDLEVLF; YEIDLEVLFE; EIDLEVLFED; IDLEVLFEDM;
DLEVLFEDMN; LEVLFEDMNG; EVLFEDMNGM; VLFEDMNGME; LFEDMNGMEY;
FEDMNGMEYA; EDMNGMEYAY; DMNGMEYAYS; MNGMEYAYSM; NGMEYAYSMG;
GMEYAYSMGT; MEYAYSMGTL; EYAYSMGTLK; YAYSMGTLKF; AYSMGTLKFK;
YSMGTLKFKG; SMGTLKFKGW; MGTLKFKGWA; GTLKFKGWAD; TLKFKGWADL;
LKFKGWADLI; KFKGWADLIW; FKGWADLIWS; KGWADLIWSN; GWADLIWSNP;
WADLIWSNPN; ADLIWSNPNY; DLIWSNPNYI; LIWSNPNYIP; IWSNPNYIPN;
WSNPNYIPNI; SNPNYIPNIS; NPNYIPNISS; PNYIPNISSR; NYIPNISSRI;
YIPNISSRII; IPNISSRIIK; PNISSRIIKD; NISSRIIKDD; ISSRIIKDDV;
SSRIIKDDVP; SRIIKDDVPN; RIIKDDVPNY; IIKDDVPNYP; IKDDVPNYPL;
KDDVPNYPLA; DDVPNYPLAS; DVPNYPLASS; VPNYPLASSK; PNYPLASSKM;
NYPLASSKMR; YPLASSKMRF; PLASSKMRFK; LASSKMRFKA; ASSKMRFKAF;
SSKMRFKAFR; SKMRFKAFRV; KMRFKAFRVS; MRFKAFRVSK; RFKAFRVSKS;
FKAFRVSKSH; KAFRVSKSHS; AFRVSKSHSS; FRVSKSHSSK; RVSKSHSSKV;
VSKSHSSKVK; SKSHSSKVKN; KSHSSKVKNF; SHSSKVKNFI; HSSKVKNFIF;
SSKVKNFIFY; SKVKNFIFYV; KVKNFIFYVK; VKNFIFYVKD; KNFIFYVKDL;
NFIFYVKDLR; FIFYVKDLRV; IFYVKDLRVL; FYVKDLRVLY; YVKDLRVLYD;
VKDLRVLYDK; KDLRVLYDKL; DLRVLYDKLS; LRVLYDKLSV; RVLYDKLSVS;
VLYDKLSVSI; LYDKLSVSID; YDKLSVSIDS; DKLSVSIDSD; KLSVSIDSDI;
LSVSIDSDID; SVSIDSDIDS; VSIDSDIDSE; SIDSDIDSES; IDSDIDSESV;
DSDIDSESVF; SDIDSESVFK; DIDSESVFKV; IDSESVFKVY; DSESVFKVYE;
SESVFKVYET; ESVFKVYETS; SVFKVYETSG; VFKVYETSGT; FKVYETSGTE;
KVYETSGTES; VYETSGTESL; YETSGTESLR; ETSGTESLRK; TSGTESLRKL;
SGTESLRKLK; GTESLRKLKA; TESLRKLKAH; ESLRKLKAHE; SLRKLKAHET;
LRKLKAHETF; RKLKAHETFK; KLKAHETFKR; LKAHETFKRV; KAHETFKRVL;
AHETFKRVLK; HETFKRVLKL; ETFKRVLKLR; TFKRVLKLRE; FKRVLKLREK;
KRVLKLREKI; RVLKLREKIS; VLKLREKISI; LKLREKISIA; KLREKISIAE;
LREKISIAEG; REKISIAEGS; EKISIAEGSF; KISIAEGSFQ; ISIAEGSFQN;
SIAEGSFQNF; IAEGSFQNFV; AEGSFQNFVE; EGSFQNFVEK; GSFQNFVEKI;
SFQNFVEKIE; FQNFVEKIES; QNFVEKIESE; NFVEKIESEK; FVEKIESEKP;
VEKIESEKPE; EKIESEKPEE; KIESEKPEES; IESEKPEESS; ESEKPEESSP;
SEKPEESSPK; EKPEESSPKN;

11 mers:
MKRKAKSILFF; KRKAKSILFFL; RKAKSILFFLL; KAKSILFFLLS;
AKSILFFLLST; KSILFFLLSTV; SILFFLLSTVL; ILFFLLSTVLF;
LFFLLSTVLFA; FFLLSTVLFAQ; FLLSTVLFAQE; LLSTVLFAQET;
LSTVLFAQETD; STVLFAQETDG; TVLFAQETDGL; VLFAQETDGLA;
LFAQETDGLAE; FAQETDGLAEG; AQETDGLAEGS; QETDGLAEGSK;

| | | | |
|---|---|---|---|
| ETDGLAEGSKR; | TDGLAEGSKRA; | DGLAEGSKRAE; | GLAEGSKRAEP; |
| LAEGSKRAEPG; | AEGSKRAEPGE; | EGSKRAEPGEL; | GSKRAEPGELV; |
| SKRAEPGELVL; | KRAEPGELVLD; | RAEPGELVLDF; | AEPGELVLDFA; |
| EPGELVLDFAE; | PGELVLDFAEL; | GELVLDFAELA; | ELVLDFAELAR; |
| LVLDFAELARD; | VLDFAELARDP; | LDFAELARDPS; | DFAELARDPSS; |
| FAELARDPSST; | AELARDPSSTR; | ELARDPSSTRL; | LARDPSSTRLD; |
| ARDPSSTRLDL; | RDPSSTRLDLT; | DPSSTRLDLTN; | PSSTRLDLTNY; |
| SSTRLDLTNYV; | STRLDLTNYVD; | TRLDLTNYVDY; | RLDLTNYVDYV; |
| LDLTNYVDYVY; | DLTNYVDYVYS; | LTNYVDYVYSG; | TNYVDYVYSGA; |
| NYVDYVYSGAS; | YVDYVYSGASG; | VDYVYSGASGI; | DYVYSGASGIV; |
| YVYSGASGIVK; | VYSGASGIVKP; | YSGASGIVKPE; | SGASGIVKPED; |
| GASGIVKPEDM; | ASGIVKPEDMV; | SGIVKPEDMVV; | GIVKPEDMVVD; |
| IVKPEDMVVDL; | VKPEDMVVDLG; | KPEDMVVDLGI; | PEDMVVDLGIN; |
| EDMVVDLGINN; | DMVVDLGINNW; | MVVDLGINNWS; | VVDLGINNWSV; |
| VDLGINNWSVL; | DLGINNWSVLL; | LGINNWSVLLT; | GINNWSVLLTP; |
| INNWSVLLTPS; | NNWSVLLTPSA; | NWSVLLTPSAR; | WSVLLTPSARL; |
| SVLLTPSARLQ; | VLLTPSARLQA; | LLTPSARLQAY; | LTPSARLQAYV; |
| TPSARLQAYVK; | PSARLQAYVKN; | SARLQAYVKNS; | ARLQAYVKNSV; |
| RLQAYVKNSVV; | LQAYVKNSVVA; | QAYVKNSVVAP; | AYVKNSVVAPA; |
| YVKNSVVAPAV; | VKNSVVAPAVV; | KNSVVAPAVVK; | NSVVAPAVVKS; |
| SVVAPAVVKSE; | VVAPAVVKSES; | VAPAVVKSESK; | APAVVKSESKR; |
| PAVVKSESKRY; | AVVKSESKRYA; | VVKSESKRYAG; | VKSESKRYAGD; |
| KSESKRYAGDT; | SESKRYAGDTI; | ESKRYAGDTIL; | SKRYAGDTILG; |
| KRYAGDTILGV; | RYAGDTILGVR; | YAGDTILGVRV; | AGDTILGVRVL; |
| GDTILGVRVLF; | DTILGVRVLFP; | TILGVRVLFPS; | ILGVRVLFPSY; |
| LGVRVLFPSYS; | GVRVLFPSYSQ; | VRVLFPSYSQS; | RVLFPSYSQSS; |
| VLFPSYSQSSA; | LFPSYSQSSAM; | FPSYSQSSAMI; | PSYSQSSAMIM; |
| SYSQSSAMIMP; | YSQSSAMIMPP; | SQSSAMIMPPF; | QSSAMIMPPFK; |
| SSAMIMPPFKI; | SAMIMPPFKIP; | AMIMPPFKIPF; | MIMPPFKIPFY; |
| IMPPFKIPFYS; | MPPFKIPFYSG; | PPFKIPFYSGE; | PFKIPFYSGES; |
| FKIPFYSGESG; | KIPFYSGESGN; | IPFYSGESGNQ; | PFYSGESGNQF; |
| FYSGESGNQFL; | YSGESGNQFLG; | SGESGNQFLGK; | GESGNQFLGKG; |
| ESGNQFLGKGL; | SGNQFLGKGLI; | GNQFLGKGLID; | NQFLGKGLIDN; |
| QFLGKGLIDNI; | FLGKGLIDNIK; | LGKGLIDNIKT; | GKGLIDNIKTM; |
| KGLIDNIKTMK; | GLIDNIKTMKE; | LIDNIKTMKEI; | IDNIKTMKEIK; |
| DNIKTMKEIKV; | NIKTMKEIKVS; | IKTMKEIKVSV; | KTMKEIKVSVY; |
| TMKEIKVSVYS; | MKEIKVSVYSL; | KEIKVSVYSLG; | EIKVSVYSLGY; |
| IKVSVYSLGYE; | KVSVYSLGYEI; | VSVYSLGYEID; | SVYSLGYEIDL; |
| VYSLGYEIDLE; | YSLGYEIDLEV; | SLGYEIDLEVL; | LGYEIDLEVLF; |
| GYEIDLEVLFE; | YEIDLEVLFED; | EIDLEVLFEDM; | IDLEVLFEDMN; |
| DLEVLFEDMNG; | LEVLFEDMNGM; | EVLFEDMNGME; | VLFEDMNGMEY; |
| LFEDMNGMEYA; | FEDMNGMEYAY; | EDMNGMEYAYS; | DMNGMEYAYSM; |
| MNGMEYAYSMG; | NGMEYAYSMGT; | GMEYAYSMGTL; | MEYAYSMGTLK; |
| EYAYSMGTLKF; | YAYSMGTLKFK; | AYSMGTLKFKG; | YSMGTLKFKGW; |
| SMGTLKFKGWA; | MGTLKFKGWAD; | GTLKFKGWADL; | TLKFKGWADLI; |
| LKFKGWADLIW; | KFKGWADLIWS; | FKGWADLIWSN; | KGWADLIWSNP; |
| GWADLIWSNPN; | WADLIWSNPNY; | ADLIWSNPNYI; | DLIWSNPNYIP; |
| LIWSNPNYIPN; | IWSNPNYIPNI; | WSNPNYIPNIS; | SNPNYIPNISS; |
| NPNYIPNISSR; | PNYIPNISSRI; | NYIPNISSRII; | YIPNISSRIIK; |
| IPNISSRIIKD; | PNISSRIIKDD; | NISSRIIKDDV; | ISSRIIKDDVP; |
| SSRIIKDDVPN; | SRIIKDDVPNY; | RIIKDDVPNYP; | IIKDDVPNYPL; |
| IKDDVPNYPLA; | KDDVPNYPLAS; | DDVPNYPLASS; | DVPNYPLASSK; |
| VPNYPLASSKM; | PNYPLASSKMR; | NYPLASSKMRF; | YPLASSKMRFK; |
| PLASSKMRFKA; | LASSKMRFKAF; | ASSKMRFKAFR; | SSKMRFKAFRV; |
| SKMRFKAFRVS; | KMRFKAFRVSK; | MRFKAFRVSKS; | RFKAFRVSKSH; |
| FKAFRVSKSHS; | KAFRVSKSHSS; | AFRVSKSHSSK; | FRVSKSHSSKV; |
| RVSKSHSSKVK; | VSKSHSSKVKN; | SKSHSSKVKNF; | KSHSSKVKNFI; |

| | |
|---|---|
| | SHSSKVKNFIF; HSSKVKNFIFY; SSKVKNFIFYV; SKVKNFIFYVK;<br>KVKNFIFYVKD; VKNFIFYVKDL; KNFIFYVKDLR; NFIFYVKDLRV;<br>FIFYVKDLRVL; IFYVKDLRVLY; FYVKDLRVLYD; YVKDLRVLYDK;<br>VKDLRVLYDKL; KDLRVLYDKLS; DLRVLYDKLSV; LRVLYDKLSVS;<br>RVLYDKLSVSI; VLYDKLSVSID; LYDKLSVSIDS; YDKLSVSIDSD;<br>DKLSVSIDSDI; KLSVSIDSDID; LSVSIDSDIDS; SVSIDSDIDSE;<br>VSIDSDIDSES; SIDSDIDSESV; IDSDIDSESVF; DSDIDSESVFK;<br>SDIDSESVFKV; DIDSESVFKVY; IDSESVFKVYE; DSESVFKVYET;<br>SESVFKVYETS; ESVFKVYETSG; SVFKVYETSGT; VFKVYETSGTE;<br>FKVYETSGTES; KVYETSGTESL; VYETSGTESLR; YETSGTESLRK;<br>ETSGTESLRKL; TSGTESLRKLK; SGTESLRKLKA; GTESLRKLKAH;<br>TESLRKLKAHE; ESLRKLKAHET; SLRKLKAHETF; LRKLKAHETFK;<br>RKLKAHETFKR; KLKAHETFKRV; LKAHETFKRVL; KAHETFKRVLK;<br>AHETFKRVLKL; HETFKRVLKLR; ETFKRVLKLRE; TFKRVLKLREK;<br>FKRVLKLREKI; KRVLKLREKIS; RVLKLREKISI; VLKLREKISIA;<br>LKLREKISIAE; KLREKISIAEG; LREKISIAEGS; REKISIAEGSF;<br>EKISIAEGSFQ; KISIAEGSFQN; ISIAEGSFQNF; SIAEGSFQNFV;<br>IAEGSFQNFVE; AEGSFQNFVEK; EGSFQNFVEKI; GSFQNFVEKIE;<br>SFQNFVEKIES; FQNFVEKIESE; QNFVEKIESEK; NFVEKIESEKP;<br>FVEKIESEKPE; VEKIESEKPEE; EKIESEKPEES; KIESEKPEESS;<br>IESEKPEESSP; ESEKPEESSPK; SEKPEESSPKN; |
| AAU07005.1\| flagellar filament 41 kDa core protein [Borrelia garinii PBi] | SEQ ID NO 15880-17189/<br>8 mers:<br>MIINHNTS; IINHNTSA; INHNTSAI; NHNTSAIN; HNTSAINA; NTSAINAS;<br>TSAINASR; SAINASRN; AINASRNN; INASRNNS; NASRNNSI; ASRNNSIN;<br>SRNNSINA; RNNSINAA; NNSINAAN; NSINAANL; SINAANLS; INAANLSK;<br>NAANLSKT; AANLSKTQ; ANLSKTQE; NLSKTQEK; LSKTQEKL; SKTQEKLS;<br>KTQEKLSS; TQEKLSSG; QEKLSSGY; EKLSSGYR; KLSSGYRI; LSSGYRIN;<br>SSGYRINR; SGYRINRA; GYRINRAS; YRINRASD; RINRASDD; INRASDDA;<br>NRASDDAA; RASDDAAG; ASDDAAGM; SDDAAGMG; DDAAGMGV; DAAGMGVS;<br>AAGMGVSG; AGMGVSGK; GMGVSGKI; MGVSGKIN; GVSGKINA; VSGKINAQ;<br>SGKINAQI; GKINAQIR; KINAQIRG; INAQIRGL; NAQIRGLS; AQIRGLSQ;<br>QIRGLSQA; IRGLSQAS; RGLSQASR; GLSQASRN; LSQASRNT; SQASRNTS;<br>QASRNTSK; ASRNTSKA; SRNTSKAI; RNTSKAIN; NTSKAINF; TSKAINFI;<br>SKAINFIQ; KAINFIQT; AINFIQTT; INFIQTTE; NFIQTTEG; FIQTTEGN;<br>IQTTEGNL; QTTEGNLN; TTEGNLNE; TEGNLNEV; EGNLNEVE; GNLNEVEK;<br>NLNEVEKV; LNEVEKVL; NEVEKVLV; EVEKVLVR; VEKVLVRM; EKVLVRMK;<br>KVLVRMKE; VLVRMKEL; LVRMKELA; VRMKELAV; RMKELAVQ; MKELAVQS;<br>KELAVQSG; ELAVQSGN; LAVQSGNG; AVQSGNGT; VQSGNGTY; QSGNGTYS;<br>SGNGTYSD; GNGTYSDA; NGTYSDAD; GTYSDADR; TYSDADRG; YSDADRGS;<br>SDADRGSI; DADRGSIQ; ADRGSIQI; DRGSIQIE; RGSIQIEI; GSIQIEIE;<br>SIQIEIEQ; IQIEIEQL; QIEIEQLT; IEIEQLTD; EIEQLTDE; IEQLTDEI;<br>EQLTDEIN; QLTDEINR; LTDEINRI; TDEINRIA; DEINRIAD; EINRIADQ;<br>INRIADQA; NRIADQAQ; RIADQAQY; IADQAQYN; ADQAQYNQ; DQAQYNQM;<br>QAQYNQMH; AQYNQMHM; QYNQMHML; YNQMHMLS; NQMHMLSN; QMHMLSNK;<br>MHMLSNKS; HMLSNKSA; MLSNKSAS; LSNKSASQ; SNKSASQN; NKSASQNV;<br>KSASQNVR; SASQNVRT; ASQNVRTA; SQNVRTAE; QNVRTAEE; NVRTAEEL;<br>VRTAEELG; RTAEELGM; TAEELGMQ; AEELGMQP; EELGMQPA; ELGMQPAK;<br>LGMQPAKI; GMQPAKIN; MQPAKINT; QPAKINTP; PAKINTPA; AKINTPAS;<br>KINTPASL; INTPASLS; NTPASLSG; TPASLSGS; PASLSGSQ; ASLSGSQA;<br>SLSGSQAS; LSGSQASW; SGSQASWT; GSQASWTL; SQASWTLR; QASWTLRV;<br>ASWTLRVH; SWTLRVHV; WTLRVHVG; TLRVHVGA; LRVHVGAN; RVHVGANQ;<br>VHVGANQD; HVGANQDE; VGANQDEA; GANQDEAI; ANQDEAIA; NQDEAIAV;<br>QDEAIAVN; DEAIAVNI; EAIAVNIY; AIAVNIYA; IAVNIYAA; AVNIYAAN;<br>VNIYAANV; NIYAANVA; IYAANVAN; YAANVANL; AANVANLF; ANVANLFS;<br>NVANLFSG; VANLFSGE; ANLFSGEG; NLFSGEGS; LFSGEGSQ; FSGEGSQA; |

SGEGSQAA; GEGSQAAQ; EGSQAAQT; GSQAAQTA; SQAAQTAP; QAAQTAPV;
AAQTAPVQ; AQTAPVQE; QTAPVQEG; TAPVQEGA; APVQEGAQ; PVQEGAQQ;
VQEGAQQE; QEGAQQEG; EGAQQEGA; GAQQEGAQ; AQQEGAQQ; QQEGAQQP;
QEGAQQPA; EGAQQPAP; GAQQPAPA; AQQPAPAT; QQPAPATA; QPAPATAP;
PAPATAPS; APATAPSQ; PATAPSQG; ATAPSQGG; TAPSQGGV; APSQGGVN;
PSQGGVNS; SQGGVNSP; QGGVNSPV; GGVNSPVN; GVNSPVNV; VNSPVNVT;
NSPVNVTT; SPVNVTTT; PVNVTTTV; VNVTTTVD; NVTTTVDA; VTTTVDAN;
TTTVDANT; TTVDANTS; TVDANTSL; VDANTSLA; DANTSLAK; ANTSLAKI;
NTSLAKIE; TSLAKIEN; SLAKIENA; LAKIENAI; AKIENAIR; KIENAIRM;
IENAIRMI; ENAIRMIS; NAIRMISD; AIRMISDQ; IRMISDQR; RMISDQRA;
MISDQRAN; ISDQRANL; SDQRANLG; DQRANLGA; QRANLGAF; RANLGAFQ;
ANLGAFQN; NLGAFQNR; LGAFQNRL; GAFQNRLE; AFQNRLES; FQNRLESI;
QNRLESIK; NRLESIKD; RLESIKDS; LESIKDST; ESIKDSTE; SIKDSTEY;
IKDSTEYA; KDSTEYAI; DSTEYAIE; STEYAIEN; TEYAIENL; EYAIENLK;
YAIENLKA; AIENLKAS; IENLKASY; ENLKASYA; NLKASYAQ; LKASYAQI;
KASYAQIK; ASYAQIKD; SYAQIKDA; YAQIKDAT; AQIKDATM; QIKDATMT;
IKDATMTD; KDATMTDE; DATMTDEV; ATMTDEVV; TMTDEVVA; MTDEVVAS;
TDEVVAST; DEVVASTT; EVVASTTN; VVASTTNS; VASTTNSI; ASTTNSIL;
STTNSILT; TTNSILTQ; TNSILTQS; NSILTQSA; SILTQSAM; ILTQSAMA;
LTQSAMAM; TQSAMAMI; QSAMAMIA; SAMAMIAQ; AMAMIAQA; MAMIAQAN;
AMIAQANQ; MIAQANQV; IAQANQVP; AQANQVPQ; QANQVPQY; ANQVPQYV;
NQVPQYVL; QVPQYVLS; VPQYVLSL; PQYVLSLL; QYVLSLLR;

9 mers:
MIINHNTSA; IINHNTSAI; INHNTSAIN; NHNTSAINA; HNTSAINAS;
NTSAINASR; TSAINASRN; SAINASRNN; AINASRNNS; INASRNNSI;
NASRNNSIN; ASRNNSINA; SRNNSINAA; RNNSINAAN; NNSINAANL;
NSINAANLS; SINAANLSK; INAANLSKT; NAANLSKTQ; AANLSKTQE;
ANLSKTQEK; NLSKTQEKL; LSKTQEKLS; SKTQEKLSS; KTQEKLSSG;
TQEKLSSGY; QEKLSSGYR; EKLSSGYRI; KLSSGYRIN; LSSGYRINR;
SSGYRINRA; SGYRINRAS; GYRINRASD; YRINRASDD; RINRASDDA;
INRASDDAA; NRASDDAAG; RASDDAAGM; ASDDAAGMG; SDDAAGMGV;
DDAAGMGVS; DAAGMGVSG; AAGMGVSGK; AGMGVSGKI; GMGVSGKIN;
MGVSGKINA; GVSGKINAQ; VSGKINAQI; SGKINAQIR; GKINAQIRG;
KINAQIRGL; INAQIRGLS; NAQIRGLSQ; AQIRGLSQA; QIRGLSQAS;
IRGLSQASR; RGLSQASRN; GLSQASRNT; LSQASRNTS; SQASRNTSK;
QASRNTSKA; ASRNTSKAI; SRNTSKAIN; RNTSKAINF; NTSKAINFI;
TSKAINFIQ; SKAINFIQT; KAINFIQTT; AINFIQTTE; INFIQTTEG;
NFIQTTEGN; FIQTTEGNL; IQTTEGNLN; QTTEGNLNE; TTEGNLNEV;
TEGNLNEVE; EGNLNEVEK; GNLNEVEKV; NLNEVEKVL; LNEVEKVLV;
NEVEKVLVR; EVEKVLVRM; VEKVLVRMK; EKVLVRMKE; KVLVRMKEL;
VLVRMKELA; LVRMKELAV; VRMKELAVQ; RMKELAVQS; MKELAVQSG;
KELAVQSGN; ELAVQSGNG; LAVQSGNGT; AVQSGNGTY; VQSGNGTYS;
QSGNGTYSD; SGNGTYSDA; GNGTYSDAD; NGTYSDADR; GTYSDADRG;
TYSDADRGS; YSDADRGSI; SDADRGSIQ; DADRGSIQI; ADRGSIQIE;
DRGSIQIEI; RGSIQIEIE; GSIQIEIEQ; SIQIEIEQL; IQIEIEQLT;
QIEIEQLTD; IEIEQLTDE; EIEQLTDEI; IEQLTDEIN; EQLTDEINR;
QLTDEINRI; LTDEINRIA; TDEINRIAD; DEINRIADQ; EINRIADQA;
INRIADQAQ; NRIADQAQY; RIADQAQYN; IADQAQYNQ; ADQAQYNQM;
DQAQYNQMH; QAQYNQMHM; AQYNQMHML; QYNQMHMLS; YNQMHMLSN;
NQMHMLSNK; QMHMLSNKS; MHMLSNKSA; HMLSNKSAS; MLSNKSASQ;
LSNKSASQN; SNKSASQNV; NKSASQNVR; KSASQNVRT; SASQNVRTA;
ASQNVRTAE; SQNVRTAEE; QNVRTAEEL; NVRTAEELG; VRTAEELGM;
RTAEELGMQ; TAEELGMQP; AEELGMQPA; EELGMQPAK; ELGMQPAKI;
LGMQPAKIN; GMQPAKINT; MQPAKINTP; QPAKINTPA; PAKINTPAS;
AKINTPASL; KINTPASLS; INTPASLSG; NTPASLSGS; TPASLSGSQ;
PASLSGSQA; ASLSGSQAS; SLSGSQASW; LSGSQASWT; SGSQASWTL;

GSQASWTLR; SQASWTLRV; QASWTLRVH; ASWTLRVHV; SWTLRVHVG;
WTLRVHVGA; TLRVHVGAN; LRVHVGANQ; RVHVGANQD; VHVGANQDE;
HVGANQDEA; VGANQDEAI; GANQDEAIA; ANQDEAIAV; NQDEAIAVN;
QDEAIAVNI; DEAIAVNIY; EAIAVNIYA; AIAVNIYAA; IAVNIYAAN;
AVNIYAANV; VNIYAANVA; NIYAANVAN; IYAANVANL; YAANVANLF;
AANVANLFS; ANVANLFSG; NVANLFSGE; VANLFSGEG; ANLFSGEGS;
NLFSGEGSQ; LFSGEGSQA; FSGEGSQAA; SGEGSQAAQ; GEGSQAAQT;
EGSQAAQTA; GSQAAQTAP; SQAAQTAPV; QAAQTAPVQ; AAQTAPVQE;
AQTAPVQEG; QTAPVQEGA; TAPVQEGAQ; APVQEGAQQ; PVQEGAQQE;
VQEGAQQEG; QEGAQQEGA; EGAQQEGAQ; GAQQEGAQQ; AQQEGAQQP;
QQEGAQQPA; QEGAQQPAP; EGAQQPAPA; GAQQPAPAT; AQQPAPATA;
QQPAPATAP; QPAPATAPS; PAPATAPSQ; APATAPSQG; PATAPSQGG;
ATAPSQGGV; TAPSQGGVN; APSQGGVNS; PSQGGVNSP; SQGGVNSPV;
QGGVNSPVN; GGVNSPVNV; GVNSPVNVT; VNSPVNVTT; NSPVNVTTT;
SPVNVTTTV; PVNVTTTVD; VNVTTTVDA; NVTTTVDAN; VTTTVDANT;
TTTVDANTS; TTVDANTSL; TVDANTSLA; VDANTSLAK; DANTSLAKI;
ANTSLAKIE; NTSLAKIEN; TSLAKIENA; SLAKIENAI; LAKIENAIR;
AKIENAIRM; KIENAIRMI; IENAIRMIS; ENAIRMISD; NAIRMISDQ;
AIRMISDQR; IRMISDQRA; RMISDQRAN; MISDQRANL; ISDQRANLG;
SDQRANLGA; DQRANLGAF; QRANLGAFQ; RANLGAFQN; ANLGAFQNR;
NLGAFQNRL; LGAFQNRLE; GAFQNRLES; AFQNRLESI; FQNRLESIK;
QNRLESIKD; NRLESIKDS; RLESIKDST; LESIKDSTE; ESIKDSTEY;
SIKDSTEYA; IKDSTEYAI; KDSTEYAIE; DSTEYAIEN; STEYAIENL;
TEYAIENLK; EYAIENLKA; YAIENLKAS; AIENLKASY; IENLKASYA;
ENLKASYAQ; NLKASYAQI; LKASYAQIK; KASYAQIKD; ASYAQIKDA;
SYAQIKDAT; YAQIKDATM; AQIKDATMT; QIKDATMTD; IKDATMTDE;
KDATMTDEV; DATMTDEVV; ATMTDEVVA; TMTDEVVAS; MTDEVVAST;
TDEVVASTT; DEVVASTTN; EVVASTTNS; VVASTTNSI; VASTTNSIL;
ASTTNSILT; STTNSILTQ; TTNSILTQS; TNSILTQSA; NSILTQSAM;
SILTQSAMA; ILTQSAMAM; LTQSAMAMI; TQSAMAMIA; QSAMAMIAQ;
SAMAMIAQA; AMAMIAQAN; MAMIAQANQ; AMIAQANQV; MIAQANQVP;
IAQANQVPQ; AQANQVPQY; QANQVPQYV; ANQVPQYVL; NQVPQYVLS;
QVPQYVLSL; VPQYVLSLL; PQYVLSLLR;

10 mers:
MIINHNTSAI; IINHNTSAIN; INHNTSAINA; NHNTSAINAS; HNTSAINASR;
NTSAINASRN; TSAINASRNN; SAINASRNNS; AINASRNNSI; INASRNNSIN;
NASRNNSINA; ASRNNSINAA; SRNNSINAAN; RNNSINAANL; NNSINAANLS;
NSINAANLSK; SINAANLSKT; INAANLSKTQ; NAANLSKTQE; AANLSKTQEK;
ANLSKTQEKL; NLSKTQEKLS; LSKTQEKLSS; SKTQEKLSSG; KTQEKLSSGY;
TQEKLSSGYR; QEKLSSGYRI; EKLSSGYRIN; KLSSGYRINR; LSSGYRINRA;
SSGYRINRAS; SGYRINRASD; GYRINRASDD; YRINRASDDA; RINRASDDAA;
INRASDDAAG; NRASDDAAGM; RASDDAAGMG; ASDDAAGMGV; SDDAAGMGVS;
DDAAGMGVSG; DAAGMGVSGK; AAGMGVSGKI; AGMGVSGKIN; GMGVSGKINA;
MGVSGKINAQ; GVSGKINAQI; VSGKINAQIR; SGKINAQIRG; GKINAQIRGL;
KINAQIRGLS; INAQIRGLSQ; NAQIRGLSQA; AQIRGLSQAS; QIRGLSQASR;
IRGLSQASRN; RGLSQASRNT; GLSQASRNTS; LSQASRNTSK; SQASRNTSKA;
QASRNTSKAI; ASRNTSKAIN; SRNTSKAINF; RNTSKAINFI; NTSKAINFIQ;
TSKAINFIQT; SKAINFIQTT; KAINFIQTTE; AINFIQTTEG; INFIQTTEGN;
NFIQTTEGNL; FIQTTEGNLN; IQTTEGNLNE; QTTEGNLNEV; TTEGNLNEVE;
TEGNLNEVEK; EGNLNEVEKV; GNLNEVEKVL; NLNEVEKVLV; LNEVEKVLVR;
NEVEKVLVRM; EVEKVLVRMK; VEKVLVRMKE; EKVLVRMKEL; KVLVRMKELA;
VLVRMKELAV; LVRMKELAVQ; VRMKELAVQS; RMKELAVQSG; MKELAVQSGN;
KELAVQSGNG; ELAVQSGNGT; LAVQSGNGTY; AVQSGNGTYS; VQSGNGTYSD;
QSGNGTYSDA; SGNGTYSDAD; GNGTYSDADR; NGTYSDADRG; GTYSDADRGS;
TYSDADRGSI; YSDADRGSIQ; SDADRGSIQI; DADRGSIQIE; ADRGSIQIEI;
DRGSIQIEIE; RGSIQIEIEQ; GSIQIEIEQL; SIQIEIEQLT; IQIEIEQLTD;

QIEIEQLTDE; IEIEQLTDEI; EIEQLTDEIN; IEQLTDEINR; EQLTDEINRI;
QLTDEINRIA; LTDEINRIAD; TDEINRIADQ; DEINRIADQA; EINRIADQAQ;
INRIADQAQY; NRIADQAQYN; RIADQAQYNQ; IADQAQYNQM; ADQAQYNQMH;
DQAQYNQMHM; QAQYNQMHML; AQYNQMHMLS; QYNQMHMLSN; YNQMHMLSNK;
NQMHMLSNKS; QMHMLSNKSA; MHMLSNKSAS; HMLSNKSASQ; MLSNKSASQN;
LSNKSASQNV; SNKSASQNVR; NKSASQNVRT; KSASQNVRTA; SASQNVRTAE;
ASQNVRTAEE; SQNVRTAEEL; QNVRTAEELG; NVRTAEELGM; VRTAEELGMQ;
RTAEELGMQP; TAEELGMQPA; AEELGMQPAK; EELGMQPAKI; ELGMQPAKIN;
LGMQPAKINT; GMQPAKINTP; MQPAKINTPA; QPAKINTPAS; PAKINTPASL;
AKINTPASLS; KINTPASLSG; INTPASLSGS; NTPASLSGSQ; TPASLSGSQA;
PASLSGSQAS; ASLSGSQASW; SLSGSQASWT; LSGSQASWTL; SGSQASWTLR;
GSQASWTLRV; SQASWTLRVH; QASWTLRVHV; ASWTLRVHVG; SWTLRVHVGA;
WTLRVHVGAN; TLRVHVGANQ; LRVHVGANQD; RVHVGANQDE; VHVGANQDEA;
HVGANQDEAI; VGANQDEAIA; GANQDEAIAV; ANQDEAIAVN; NQDEAIAVNI;
QDEAIAVNIY; DEAIAVNIYA; EAIAVNIYAA; AIAVNIYAAN; IAVNIYAANV;
AVNIYAANVA; VNIYAANVAN; NIYAANVANL; IYAANVANLF; YAANVANLFS;
AANVANLFSG; ANVANLFSGE; NVANLFSGEG; VANLFSGEGS; ANLFSGEGSQ;
NLFSGEGSQA; LFSGEGSQAA; FSGEGSQAAQ; SGEGSQAAQT; GEGSQAAQTA;
EGSQAAQTAP; GSQAAQTAPV; SQAAQTAPVQ; QAAQTAPVQE; AAQTAPVQEG;
AQTAPVQEGA; QTAPVQEGAQ; TAPVQEGAQQ; APVQEGAQQE; PVQEGAQQEG;
VQEGAQQEGA; QEGAQQEGAQ; EGAQQEGAQQ; GAQQEGAQQP; AQQEGAQQPA;
QQEGAQQPAP; QEGAQQPAPA; EGAQQPAPAT; GAQQPAPATA; AQQPAPATAP;
QQPAPATAPS; QPAPATAPSQ; PAPATAPSQG; APATAPSQGG; PATAPSQGGV;
ATAPSQGGVN; TAPSQGGVNS; APSQGGVNSP; PSQGGVNSPV; SQGGVNSPVN;
QGGVNSPVNV; GGVNSPVNVT; GVNSPVNVTT; VNSPVNVTTT; NSPVNVTTTV;
SPVNVTTTVD; PVNVTTTVDA; VNVTTTVDAN; NVTTTVDANT; VTTTVDANTS;
TTTVDANTSL; TTVDANTSLA; TVDANTSLAK; VDANTSLAKI; DANTSLAKIE;
ANTSLAKIEN; NTSLAKIENA; TSLAKIENAI; SLAKIENAIR; LAKIENAIRM;
AKIENAIRMI; KIENAIRMIS; IENAIRMISD; ENAIRMISDQ; NAIRMISDQR;
AIRMISDQRA; IRMISDQRAN; RMISDQRANL; MISDQRANLG; ISDQRANLGA;
SDQRANLGAF; DQRANLGAFQ; QRANLGAFQN; RANLGAFQNR; ANLGAFQNRL;
NLGAFQNRLE; LGAFQNRLES; GAFQNRLESI; AFQNRLESIK; FQNRLESIKD;
QNRLESIKDS; NRLESIKDST; RLESIKDSTE; LESIKDSTEY; ESIKDSTEYA;
SIKDSTEYAI; IKDSTEYAIE; KDSTEYAIEN; DSTEYAIENL; STEYAIENLK;
TEYAIENLKA; EYAIENLKAS; YAIENLKASY; AIENLKASYA; IENLKASYAQ;
ENLKASYAQI; NLKASYAQIK; LKASYAQIKD; KASYAQIKDA; ASYAQIKDAT;
SYAQIKDATM; YAQIKDATMT; AQIKDATMTD; QIKDATMTDE; IKDATMTDEV;
KDATMTDEVV; DATMTDEVVA; ATMTDEVVAS; TMTDEVVAST; MTDEVVASTT;
TDEVVASTTN; DEVVASTTNS; EVVASTTNSI; VVASTTNSIL; VASTTNSILT;
ASTTNSILTQ; STTNSILTQS; TTNSILTQSA; TNSILTQSAM; NSILTQSAMA;
SILTQSAMAM; ILTQSAMAMI; LTQSAMAMIA; TQSAMAMIAQ; QSAMAMIAQA;
SAMAMIAQAN; AMAMIAQANQ; MAMIAQANQV; AMIAQANQVP; MIAQANQVPQ;
IAQANQVPQY; AQANQVPQYV; QANQVPQYVL; ANQVPQYVLS; NQVPQYVLSL;
QVPQYVLSLL; VPQYVLSLLR;

11 mers:
MIINHNTSAIN; IINHNTSAINA; INHNTSAINAS; NHNTSAINASR;
HNTSAINASRN; NTSAINASRNN; TSAINASRNNS; SAINASRNNSI;
AINASRNNSIN; INASRNNSINA; NASRNNSINAA; ASRNNSINAAN;
SRNNSINAANL; RNNSINAANLS; NNSINAANLSK; NSINAANLSKT;
SINAANLSKTQ; INAANLSKTQE; NAANLSKTQEK; AANLSKTQEKL;
ANLSKTQEKLS; NLSKTQEKLSS; LSKTQEKLSSG; SKTQEKLSSGY;
KTQEKLSSGYR; TQEKLSSGYRI; QEKLSSGYRIN; EKLSSGYRINR;
KLSSGYRINRA; LSSGYRINRAS; SSGYRINRASD; SGYRINRASDD;
GYRINRASDDA; YRINRASDDAA; RINRASDDAAG; INRASDDAAGM;
NRASDDAAGMG; RASDDAAGMGV; ASDDAAGMGVS; SDDAAGMGVSG;
DDAAGMGVSGK; DAAGMGVSGKI; AAGMGVSGKIN; AGMGVSGKINA;

656

GMGVSGKINAQ; MGVSGKINAQI; GVSGKINAQIR; VSGKINAQIRG;
SGKINAQIRGL; GKINAQIRGLS; KINAQIRGLSQ; INAQIRGLSQA;
NAQIRGLSQAS; AQIRGLSQASR; QIRGLSQASRN; IRGLSQASRNT;
RGLSQASRNTS; GLSQASRNTSK; LSQASRNTSKA; SQASRNTSKAI;
QASRNTSKAIN; ASRNTSKAINF; SRNTSKAINFI; RNTSKAINFIQ;
NTSKAINFIQT; TSKAINFIQTT; SKAINFIQTTE; KAINFIQTTEG;
AINFIQTTEGN; INFIQTTEGNL; NFIQTTEGNLN; FIQTTEGNLNE;
IQTTEGNLNEV; QTTEGNLNEVE; TTEGNLNEVEK; TEGNLNEVEKV;
EGNLNEVEKVL; GNLNEVEKVLV; NLNEVEKVLVR; LNEVEKVLVRM;
NEVEKVLVRMK; EVEKVLVRMKE; VEKVLVRMKEL; EKVLVRMKELA;
KVLVRMKELAV; VLVRMKELAVQ; LVRMKELAVQS; VRMKELAVQSG;
RMKELAVQSGN; MKELAVQSGNG; KELAVQSGNGT; ELAVQSGNGTY;
LAVQSGNGTYS; AVQSGNGTYSD; VQSGNGTYSDA; QSGNGTYSDAD;
SGNGTYSDADR; GNGTYSDADRG; NGTYSDADRGS; GTYSDADRGSI;
TYSDADRGSIQ; YSDADRGSIQI; SDADRGSIQIE; DADRGSIQIEI;
ADRGSIQIEIE; DRGSIQIEIEQ; RGSIQIEIEQL; GSIQIEIEQLT;
SIQIEIEQLTD; IQIEIEQLTDE; QIEIEQLTDEI; IEIEQLTDEIN;
EIEQLTDEINR; IEQLTDEINRI; EQLTDEINRIA; QLTDEINRIAD;
LTDEINRIADQ; TDEINRIADQA; DEINRIADQAQ; EINRIADQAQY;
INRIADQAQYN; NRIADQAQYNQ; RIADQAQYNQM; IADQAQYNQMH;
ADQAQYNQMHM; DQAQYNQMHML; QAQYNQMHMLS; AQYNQMHMLSN;
QYNQMHMLSNK; YNQMHMLSNKS; NQMHMLSNKSA; QMHMLSNKSAS;
MHMLSNKSASQ; HMLSNKSASQN; MLSNKSASQNV; LSNKSASQNVR;
SNKSASQNVRT; NKSASQNVRTA; KSASQNVRTAE; SASQNVRTAEE;
ASQNVRTAEEL; SQNVRTAEELG; QNVRTAEELGM; NVRTAEELGMQ;
VRTAEELGMQP; RTAEELGMQPA; TAEELGMQPAK; AEELGMQPAKI;
EELGMQPAKIN; ELGMQPAKINT; LGMQPAKINTP; GMQPAKINTPA;
MQPAKINTPAS; QPAKINTPASL; PAKINTPASLS; AKINTPASLSG;
KINTPASLSGS; INTPASLSGSQ; NTPASLSGSQA; TPASLSGSQAS;
PASLSGSQASW; ASLSGSQASWT; SLSGSQASWTL; LSGSQASWTLR;
SGSQASWTLRV; GSQASWTLRVH; SQASWTLRVHV; QASWTLRVHVG;
ASWTLRVHVGA; SWTLRVHVGAN; WTLRVHVGANQ; TLRVHVGANQD;
LRVHVGANQDE; RVHVGANQDEA; VHVGANQDEAI; HVGANQDEAIA;
VGANQDEAIAV; GANQDEAIAVN; ANQDEAIAVNI; NQDEAIAVNIY;
QDEAIAVNIYA; DEAIAVNIYAA; EAIAVNIYAAN; AIAVNIYAANV;
IAVNIYAANVA; AVNIYAANVAN; VNIYAANVANL; NIYAANVANLF;
IYAANVANLFS; YAANVANLFSG; AANVANLFSGE; ANVANLFSGEG;
NVANLFSGEGS; VANLFSGEGSQ; ANLFSGEGSQA; NLFSGEGSQAA;
LFSGEGSQAAQ; FSGEGSQAAQT; SGEGSQAAQTA; GEGSQAAQTAP;
EGSQAAQTAPV; GSQAAQTAPVQ; SQAAQTAPVQE; QAAQTAPVQEG;
AAQTAPVQEGA; AQTAPVQEGAQ; QTAPVQEGAQQ; TAPVQEGAQQE;
APVQEGAQQEG; PVQEGAQQEGA; VQEGAQQEGAQ; QEGAQQEGAQQ;
EGAQQEGAQQP; GAQQEGAQQPA; AQQEGAQQPAP; QQEGAQQPAPA;
QEGAQQPAPAT; EGAQQPAPATA; GAQQPAPATAP; AQQPAPATAPS;
QQPAPATAPSQ; QPAPATAPSQG; PAPATAPSQGG; APATAPSQGGV;
PATAPSQGGVN; ATAPSQGGVNS; TAPSQGGVNSP; APSQGGVNSPV;
PSQGGVNSPVN; SQGGVNSPVNV; QGGVNSPVNVT; GGVNSPVNVTT;
GVNSPVNVTTT; VNSPVNVTTTV; NSPVNVTTTVD; SPVNVTTTVDA;
PVNVTTTVDAN; VNVTTTVDANT; NVTTTVDANTS; VTTTVDANTSL;
TTTVDANTSLA; TTVDANTSLAK; TVDANTSLAKI; VDANTSLAKIE;
DANTSLAKIEN; ANTSLAKIENA; NTSLAKIENAI; TSLAKIENAIR;
SLAKIENAIRM; LAKIENAIRMI; AKIENAIRMIS; KIENAIRMISD;
IENAIRMISDQ; ENAIRMISDQR; NAIRMISDQRA; AIRMISDQRAN;
IRMISDQRANL; RMISDQRANLG; MISDQRANLGA; ISDQRANLGAF;
SDQRANLGAFQ; DQRANLGAFQN; QRANLGAFQNR; RANLGAFQNRL;
ANLGAFQNRLE; NLGAFQNRLES; LGAFQNRLESI; GAFQNRLESIK;
AFQNRLESIKD; FQNRLESIKDS; QNRLESIKDST; NRLESIKDSTE;

| | |
|---|---|
| | RLESIKDSTEY; LESIKDSTEYA; ESIKDSTEYAI; SIKDSTEYAIE; IKDSTEYAIEN; KDSTEYAIENL; DSTEYAIENLK; STEYAIENLKA; TEYAIENLKAS; EYAIENLKASY; YAIENLKASYA; AIENLKASYAQ; IENLKASYAQI; ENLKASYAQIK; NLKASYAQIKD; LKASYAQIKDA; KASYAQIKDAT; ASYAQIKDATM; SYAQIKDATMT; YAQIKDATMTD; AQIKDATMTDE; QIKDATMTDEV; IKDATMTDEVV; KDATMTDEVVA; DATMTDEVVAS; ATMTDEVVAST; TMTDEVVASTT; MTDEVVASTTN; TDEVVASTTNS; DEVVASTTNSI; EVVASTTNSIL; VVASTTNSILT; VASTTNSILTQ; ASTTNSILTQS; STTNSILTQSA; TTNSILTQSAM; TNSILTQSAMA; NSILTQSAMAM; SILTQSAMAMI; ILTQSAMAMIA; LTQSAMAMIAQ; TQSAMAMIAQA; QSAMAMIAQAN; SAMAMIAQANQ; AMAMIAQANQV; MAMIAQANQVP; AMIAQANQVPQ; MIAQANQVPQY; IAQANQVPQYV; AQANQVPQYVL; QANQVPQYVLS; ANQVPQYVLSL; NQVPQYVLSLL; QVPQYVLSLLR; |
| 1L8W\|A Chain A, Crystal Structure Of Lyme Disease Variable Surface Antigen Vlse Of Borrelia Burgdorferi | SEQ ID NO 17190-18547/<br>8 mers:<br>MRGSHHHH; RGSHHHHH; GSHHHHHH; SHHHHHHG; HHHHHHGS; HHHHHGSS; HHHHGSSQ; HHHGSSQV; HHGSSQVA; HGSSQVAD; GSSQVADK; SSQVADKD; SQVADKDD; QVADKDDP; VADKDDPT; ADKDDPTN; DKDDPTNK; KDDPTNKF; DDPTNKFY; DPTNKFYQ; PTNKFYQS; TNKFYQSV; NKFYQSVI; KFYQSVIQ; FYQSVIQL; YQSVIQLG; QSVIQLGN; SVIQLGNG; VIQLGNGF; IQLGNGFL; QLGNGFLD; LGNGFLDV; GNGFLDVF; NGFLDVFT; GFLDVFTS; FLDVFTSF; LDVFTSFG; DVFTSFGG; VFTSFGGL; FTSFGGLV; TSFGGLVA; SFGGLVAE; FGGLVAEA; GGLVAEAF; GLVAEAFG; LVAEAFGF; VAEAFGFK; AEAFGFKS; EAFGFKSD; AFGFKSDP; FGFKSDPK; GFKSDPKK; FKSDPKKS; KSDPKKSD; SDPKKSDV; DPKKSDVK; PKKSDVKT; KKSDVKTY; KSDVKTYF; SDVKTYFT; DVKTYFTT; VKTYFTTV; KTYFTTVA; TYFTTVAA; YFTTVAAK; FTTVAAKL; TTVAAKLE; TVAAKLEK; VAAKLEKT; AAKLEKTK; AKLEKTKT; KLEKTKTD; LEKTKTDL; EKTKTDLN; KTKTDLNS; TKTDLNSL; KTDLNSLP; TDLNSLPK; DLNSLPKE; LNSLPKEK; NSLPKEKS; SLPKEKSD; LPKEKSDI; PKEKSDIS; KEKSDISS; EKSDISST; KSDISSTT; SDISSTTG; DISSTTGK; ISSTTGKP; SSTTGKPD; STTGKPDS; TTGKPDST; TGKPDSTG; GKPDSTGS; KPDSTGSV; PDSTGSVG; DSTGSVGT; STGSVGTA; TGSVGTAV; GSVGTAVE; SVGTAVEG; VGTAVEGA; GTAVEGAI; TAVEGAIK; AVEGAIKE; VEGAIKEV; EGAIKEVS; GAIKEVSE; AIKEVSEL; IKEVSELL; KEVSELLD; EVSELLDK; VSELLDKL; SELLDKLV; ELLDKLVK; LLDKLVKA; LDKLVKAV; DKLVKAVK; KLVKAVKT; LVKAVKTA; VKAVKTAE; KAVKTAEG; AVKTAEGA; VKTAEGAS; KTAEGASS; TAEGASSG; AEGASSGT; EGASSGTA; GASSGTAA; ASSGTAAI; SSGTAAIG; SGTAAIGE; GTAAIGEV; TAAIGEVV; AAIGEVVA; AIGEVVAD; IGEVVADA; GEVVADAD; EVVADADA; VVADADAA; VADADAAK; ADADAAKV; DADAAKVA; ADAAKVAD; DAAKVADK; AAKVADKA; AKVADKAS; KVADKASV; VADKASVK; ADKASVKG; DKASVKGI; KASVKGIA; ASVKGIAK; SVKGIAKG; VKGIAKGI; KGIAKGIK; GIAKGIKE; IAKGIKEI; AKGIKEIV; KGIKEIVE; GIKEIVEA; IKEIVEAA; KEIVEAAG; EIVEAAGG; IVEAAGGS; VEAAGGSE; EAAGGSEK; AAGGSEKL; AGGSEKLK; GGSEKLKA; GSEKLKAV; SEKLKAVA; EKLKAVAA; KLKAVAAA; LKAVAAAK; KAVAAAKG; AVAAAKGE; VAAAKGEN; AAAKGENN; AAKGENNK; AKGENNKG; KGENNKGA; GENNKGAG; ENNKGAGK; NNKGAGKL; NKGAGKLF; KGAGKLFG; GAGKLFGK; AGKLFGKA; GKLFGKAG; KLFGKAGA; LFGKAGAA; FGKAGAAA; GKAGAAAH; KAGAAAHG; AGAAAHGD; GAAAHGDS; AAAHGDSE; AAHGDSEA; AHGDSEAA; HGDSEAAS; GDSEAASK; DSEAASKA; SEAASKAA; EAASKAAG; AASKAAGA; ASKAAGAV; SKAAGAVS; KAAGAVSA; AAGAVSAV; AGAVSAVS; GAVSAVSG; AVSAVSGE; VSAVSGEQ; SAVSGEQI; AVSGEQIL; VSGEQILS; SGEQILSA; GEQILSAI; EQILSAIV; QILSAIVT; ILSAIVTA; LSAIVTAA; SAIVTAAD; AIVTAADA; IVTAADAA; VTAADAAE; TAADAAEQ; AADAAEQD; ADAAEQDG; DAAEQDGK; AAEQDGKK; AEQDGKKP; EQDGKKPE; QDGKKPEE; DGKKPEEA; GKKPEEAK; KKPEEAKN; KPEEAKNP; |

PEEAKNPI; EEAKNPIA; EAKNPIAA; AKNPIAAA; KNPIAAAI; NPIAAAIG;
PIAAAIGD; IAAAIGDK; AAAIGDKD; AAIGDKDG; AIGDKDGG; IGDKDGGA;
GDKDGGAE; DKDGGAEF; KDGGAEFG; DGGAEFGQ; GGAEFGQD; GAEFGQDE;
AEFGQDEX; EFGQDEXK; FGQDEXKK; GQDEXKKD; QDEXKKDD; DEXKKDDQ;
EXKKDDQI; XKKDDQIA; KKDDQIAA; KDDQIAAA; DDQIAAAI; DQIAAAIA;
QIAAAIAL; IAAAIALR; AAAIALRG; AAIALRGX; AIALRGXA; IALRGXAK;
ALRGXAKD; LRGXAKDG; RGXAKDGK; GXAKDGKF; XAKDGKFA; AKDGKFAV;
KDGKFAVK; DGKFAVKD; GKFAVKDG; KFAVKDGE; FAVKDGEK; AVKDGEKE;
VKDGEKEK; KDGEKEKA; DGEKEKAE; GEKEKAEG; EKEKAEGA; KEKAEGAI;
EKAEGAIK; KAEGAIKG; AEGAIKGA; EGAIKGAA; GAIKGAAE; AIKGAAES;
IKGAAESA; KGAAESAV; GAAESAVR; AAESAVRK; AESAVRKV; ESAVRKVL;
SAVRKVLG; AVRKVLGA; VRKVLGAI; RKVLGAIT; KVLGAITG; VLGAITGL;
LGAITGLI; GAITGLIG; AITGLIGD; ITGLIGDA; TGLIGDAV; GLIGDAVS;
LIGDAVSS; IGDAVSSG; GDAVSSGL; DAVSSGLR; AVSSGLRK; VSSGLRKV;
SSGLRKVG; SGLRKVGD; GLRKVGDS; LRKVGDSV; RKVGDSVK; KVGDSVKA;
VGDSVKAA; GDSVKAAS; DSVKAASK; SVKAASKE; VKAASKET; KAASKETP;
AASKETPP; ASKETPPA; SKETPPAL; KETPPALN; ETPPALNK;

9 mers:
MRGSHHHHH; RGSHHHHHH; GSHHHHHHG; SHHHHHHGS; HHHHHHGSS;
HHHHHGSSQ; HHHHGSSQV; HHHGSSQVA; HHGSSQVAD; HGSSQVADK;
GSSQVADKD; SSQVADKDD; SQVADKDDP; QVADKDDPT; VADKDDPTN;
ADKDDPTNK; DKDDPTNKF; KDDPTNKFY; DDPTNKFYQ; DPTNKFYQS;
PTNKFYQSV; TNKFYQSVI; NKFYQSVIQ; KFYQSVIQL; FYQSVIQLG;
YQSVIQLGN; QSVIQLGNG; SVIQLGNGF; VIQLGNGFL; IQLGNGFLD;
QLGNGFLDV; LGNGFLDVF; GNGFLDVFT; NGFLDVFTS; GFLDVFTSF;
FLDVFTSFG; LDVFTSFGG; DVFTSFGGL; VFTSFGGLV; FTSFGGLVA;
TSFGGLVAE; SFGGLVAEA; FGGLVAEAF; GGLVAEAFG; GLVAEAFGF;
LVAEAFGFK; VAEAFGFKS; AEAFGFKSD; EAFGFKSDP; AFGFKSDPK;
FGFKSDPKK; GFKSDPKKS; FKSDPKKSD; KSDPKKSDV; SDPKKSDVK;
DPKKSDVKT; PKKSDVKTY; KKSDVKTYF; KSDVKTYFT; SDVKTYFTT;
DVKTYFTTV; VKTYFTTVA; KTYFTTVAA; TYFTTVAAK; YFTTVAAKL;
FTTVAAKLE; TTVAAKLEK; TVAAKLEKT; VAAKLEKTK; AAKLEKTKT;
AKLEKTKTD; KLEKTKTDL; LEKTKTDLN; EKTKTDLNS; KTKTDLNSL;
TKTDLNSLP; KTDLNSLPK; TDLNSLPKE; DLNSLPKEK; LNSLPKEKS;
NSLPKEKSD; SLPKEKSDI; LPKEKSDIS; PKEKSDISS; KEKSDISST;
EKSDISSTT; KSDISSTTG; SDISSTTGK; DISSTTGKP; ISSTTGKPD;
SSTTGKPDS; STTGKPDST; TTGKPDSTG; TGKPDSTGS; GKPDSTGSV;
KPDSTGSVG; PDSTGSVGT; DSTGSVGTA; STGSVGTAV; TGSVGTAVE;
GSVGTAVEG; SVGTAVEGA; VGTAVEGAI; GTAVEGAIK; TAVEGAIKE;
AVEGAIKEV; VEGAIKEVS; EGAIKEVSE; GAIKEVSEL; AIKEVSELL;
IKEVSELLD; KEVSELLDK; EVSELLDKL; VSELLDKLV; SELLDKLVK;
ELLDKLVKA; LLDKLVKAV; LDKLVKAVK; DKLVKAVKT; KLVKAVKTA;
LVKAVKTAE; VKAVKTAEG; KAVKTAEGA; AVKTAEGAS; VKTAEGASS;
KTAEGASSG; TAEGASSGT; AEGASSGTA; EGASSGTAA; GASSGTAAI;
ASSGTAAIG; SSGTAAIGE; SGTAAIGEV; GTAAIGEVV; TAAIGEVVA;
AAIGEVVAD; AIGEVVADA; IGEVVADAD; GEVVADADA; EVVADADAA;
VVADADAAK; VADADAAKV; ADADAAKVA; DADAAKVAD; ADAAKVADK;
DAAKVADKA; AAKVADKAS; AKVADKASV; KVADKASVK; VADKASVKG;
ADKASVKGI; DKASVKGIA; KASVKGIAK; ASVKGIAKG; SVKGIAKGI;
VKGIAKGIK; KGIAKGIKE; GIAKGIKEI; IAKGIKEIV; AKGIKEIVE;
KGIKEIVEA; GIKEIVEAA; IKEIVEAAG; KEIVEAAGG; EIVEAAGGS;
IVEAAGGSE; VEAAGGSEK; EAAGGSEKL; AAGGSEKLK; AGGSEKLKA;
GGSEKLKAV; GSEKLKAVA; SEKLKAVAA; EKLKAVAAA; KLKAVAAAK;
LKAVAAAKG; KAVAAAKGE; AVAAAKGEN; VAAAKGENN; AAAKGENNK;
AAKGENNKG; AKGENNKGA; KGENNKGAG; GENNKGAGK; ENNKGAGKL;
NNKGAGKLF; NKGAGKLFG; KGAGKLFGK; GAGKLFGKA; AGKLFGKAG;

GKLFGKAGA; KLFGKAGAA; LFGKAGAAA; FGKAGAAAH; GKAGAAAHG;
KAGAAAHGD; AGAAAHGDS; GAAAHGDSE; AAAHGDSEA; AAHGDSEAA;
AHGDSEAAS; HGDSEAASK; GDSEAASKA; DSEAASKAA; SEAASKAAG;
EAASKAAGA; AASKAAGAV; ASKAAGAVS; SKAAGAVSA; KAAGAVSAV;
AAGAVSAVS; AGAVSAVSG; GAVSAVSGE; AVSAVSGEQ; VSAVSGEQI;
SAVSGEQIL; AVSGEQILS; VSGEQILSA; SGEQILSAI; GEQILSAIV;
EQILSAIVT; QILSAIVTA; ILSAIVTAA; LSAIVTAAD; SAIVTAADA;
AIVTAADAA; IVTAADAAE; VTAADAAEQ; TAADAAEQD; AADAAEQDG;
ADAAEQDGK; DAAEQDGKK; AAEQDGKKP; AEQDGKKPE; EQDGKKPEE;
QDGKKPEEA; DGKKPEEAK; GKKPEEAKN; KKPEEAKNP; KPEEAKNPI;
PEEAKNPIA; EEAKNPIAA; EAKNPIAAA; AKNPIAAAI; KNPIAAAIG;
NPIAAAIGD; PIAAAIGDK; IAAAIGDKD; AAAIGDKDG; AAIGDKDGG;
AIGDKDGGA; IGDKDGGAE; GDKDGGAEF; DKDGGAEFG; KDGGAEFGQ;
DGGAEFGQD; GGAEFGQDE; GAEFGQDEX; AEFGQDEXK; EFGQDEXKK;
FGQDEXKKD; GQDEXKKDD; QDEXKKDDQ; DEXKKDDQI; EXKKDDQIA;
XKKDDQIAA; KKDDQIAAA; KDDQIAAAI; DDQIAAAIA; DQIAAAIAL;
QIAAAIALR; IAAAIALRG; AAAIALRGX; AAIALRGXA; AIALRGXAK;
IALRGXAKD; ALRGXAKDG; LRGXAKDGK; RGXAKDGKF; GXAKDGKFA;
XAKDGKFAV; AKDGKFAVK; KDGKFAVKD; DGKFAVKDG; GKFAVKDGE;
KFAVKDGEK; FAVKDGEKE; AVKDGEKEK; VKDGEKEKA; KDGEKEKAE;
DGEKEKAEG; GEKEKAEGA; EKEKAEGAI; KEKAEGAIK; EKAEGAIKG;
KAEGAIKGA; AEGAIKGAA; EGAIKGAAE; GAIKGAAES; AIKGAAESA;
IKGAAESAV; KGAAESAVR; GAAESAVRK; AAESAVRKV; AESAVRKVL;
ESAVRKVLG; SAVRKVLGA; AVRKVLGAI; VRKVLGAIT; RKVLGAITG;
KVLGAITGL; VLGAITGLI; LGAITGLIG; GAITGLIGD; AITGLIGDA;
ITGLIGDAV; TGLIGDAVS; GLIGDAVSS; LIGDAVSSG; IGDAVSSGL;
GDAVSSGLR; DAVSSGLRK; AVSSGLRKV; VSSGLRKVG; SSGLRKVGD;
SGLRKVGDS; GLRKVGDSV; LRKVGDSVK; RKVGDSVKA; KVGDSVKAA;
VGDSVKAAS; GDSVKAASK; DSVKAASKE; SVKAASKET; VKAASKETP;
KAASKETPP; AASKETPPA; ASKETPPAL; SKETPPALN; KETPPALNK

10 mers:
MRGSHHHHHH; RGSHHHHHHG; GSHHHHHHGS; SHHHHHHGSS; HHHHHHGSSQ;
HHHHHGSSQV; HHHHGSSQVA; HHHGSSQVAD; HHGSSQVADK; HGSSQVADKD;
GSSQVADKDD; SSQVADKDDP; SQVADKDDPT; QVADKDDPTN; VADKDDPTNK;
ADKDDPTNKF; DKDDPTNKFY; KDDPTNKFYQ; DDPTNKFYQS; DPTNKFYQSV;
PTNKFYQSVI; TNKFYQSVIQ; NKFYQSVIQL; KFYQSVIQLG; FYQSVIQLGN;
YQSVIQLGNG; QSVIQLGNGF; SVIQLGNGFL; VIQLGNGFLD; IQLGNGFLDV;
QLGNGFLDVF; LGNGFLDVFT; GNGFLDVFTS; NGFLDVFTSF; GFLDVFTSFG;
FLDVFTSFGG; LDVFTSFGGL; DVFTSFGGLV; VFTSFGGLVA; FTSFGGLVAE;
TSFGGLVAEA; SFGGLVAEAF; FGGLVAEAFG; GGLVAEAFGF; GLVAEAFGFK;
LVAEAFGFKS; VAEAFGFKSD; AEAFGFKSDP; EAFGFKSDPK; AFGFKSDPKK;
FGFKSDPKKS; GFKSDPKKSD; FKSDPKKSDV; KSDPKKSDVK; SDPKKSDVKT;
DPKKSDVKTY; PKKSDVKTYF; KKSDVKTYFT; KSDVKTYFTT; SDVKTYFTTV;
DVKTYFTTVA; VKTYFTTVAA; KTYFTTVAAK; TYFTTVAAKL; YFTTVAAKLE;
FTTVAAKLEK; TTVAAKLEKT; TVAAKLEKTK; VAAKLEKTKT; AAKLEKTKTD;
AKLEKTKTDL; KLEKTKTDLN; LEKTKTDLNS; EKTKTDLNSL; KTKTDLNSLP;
TKTDLNSLPK; KTDLNSLPKE; TDLNSLPKEK; DLNSLPKEKS; LNSLPKEKSD;
NSLPKEKSDI; SLPKEKSDIS; LPKEKSDISS; PKEKSDISST; KEKSDISSTT;
EKSDISSTTG; KSDISSTTGK; SDISSTTGKP; DISSTTGKPD; ISSTTGKPDS;
SSTTGKPDST; STTGKPDSTG; TTGKPDSTGS; TGKPDSTGSV; GKPDSTGSVG;
KPDSTGSVGT; PDSTGSVGTA; DSTGSVGTAV; STGSVGTAVE; TGSVGTAVEG;
GSVGTAVEGA; SVGTAVEGAI; VGTAVEGAIK; GTAVEGAIKE; TAVEGAIKEV;
AVEGAIKEVS; VEGAIKEVSE; EGAIKEVSEL; GAIKEVSELL; AIKEVSELLD;
IKEVSELLDK; KEVSELLDKL; EVSELLDKLV; VSELLDKLVK; SELLDKLVKA;
ELLDKLVKAV; LLDKLVKAVK; LDKLVKAVKT; DKLVKAVKTA; KLVKAVKTAE;
LVKAVKTAEG; VKAVKTAEGA; KAVKTAEGAS; AVKTAEGASS; VKTAEGASSG;

KTAEGASSGT; TAEGASSGTA; AEGASSGTAA; EGASSGTAAI; GASSGTAAIG;
ASSGTAAIGE; SSGTAAIGEV; SGTAAIGEVV; GTAAIGEVVA; TAAIGEVVAD;
AAIGEVVADA; AIGEVVADAD; IGEVVADADA; GEVVADADAA; EVVADADAAK;
VVADADAAKV; VADADAAKVA; ADADAAKVAD; DADAAKVADK; ADAAKVADKA;
DAAKVADKAS; AAKVADKASV; AKVADKASVK; KVADKASVKG; VADKASVKGI;
ADKASVKGIA; DKASVKGIAK; KASVKGIAKG; ASVKGIAKGI; SVKGIAKGIK;
VKGIAKGIKE; KGIAKGIKEI; GIAKGIKEIV; IAKGIKEIVE; AKGIKEIVEA;
KGIKEIVEAA; GIKEIVEAAG; IKEIVEAAGG; KEIVEAAGGS; EIVEAAGGSE;
IVEAAGGSEK; VEAAGGSEKL; EAAGGSEKLK; AAGGSEKLKA; AGGSEKLKAV;
GGSEKLKAVA; GSEKLKAVAA; SEKLKAVAAA; EKLKAVAAAK; KLKAVAAAKG;
LKAVAAAKGE; KAVAAAKGEN; AVAAAKGENN; VAAAKGENNK; AAAKGENNKG;
AAKGENNKGA; AKGENNKGAG; KGENNKGAGK; GENNKGAGKL; ENNKGAGKLF;
NNKGAGKLFG; NKGAGKLFGK; KGAGKLFGKA; GAGKLFGKAG; AGKLFGKAGA;
GKLFGKAGAA; KLFGKAGAAA; LFGKAGAAAH; FGKAGAAAHG; GKAGAAAHGD;
KAGAAAHGDS; AGAAAHGDSE; GAAAHGDSEA; AAAHGDSEAA; AAHGDSEAAS;
AHGDSEAASK; HGDSEAASKA; GDSEAASKAA; DSEAASKAAG; SEAASKAAGA;
EAASKAAGAV; AASKAAGAVS; ASKAAGAVSA; SKAAGAVSAV; KAAGAVSAVS;
AAGAVSAVSG; AGAVSAVSGE; GAVSAVSGEQ; AVSAVSGEQI; VSAVSGEQIL;
SAVSGEQILS; AVSGEQILSA; VSGEQILSAI; SGEQILSAIV; GEQILSAIVT;
EQILSAIVTA; QILSAIVTAA; ILSAIVTAAD; LSAIVTAADA; SAIVTAADAA;
AIVTAADAAE; IVTAADAAEQ; VTAADAAEQD; TAADAAEQDG; AADAAEQDGK;
ADAAEQDGKK; DAAEQDGKKP; AAEQDGKKPE; AEQDGKKPEE; EQDGKKPEEA;
QDGKKPEEAK; DGKKPEEAKN; GKKPEEAKNP; KKPEEAKNPI; KPEEAKNPIA;
PEEAKNPIAA; EEAKNPIAAA; EAKNPIAAAI; AKNPIAAAIG; KNPIAAAIGD;
NPIAAAIGDK; PIAAAIGDKD; IAAAIGDKDG; AAAIGDKDGG; AAIGDKDGGA;
AIGDKDGGAE; IGDKDGGAEF; GDKDGGAEFG; DKDGGAEFGQ; KDGGAEFGQD;
DGGAEFGQDE; GGAEFGQDEX; GAEFGQDEXK; AEFGQDEXKK; EFGQDEXKKD;
FGQDEXKKDD; GQDEXKKDDQ; QDEXKKDDQI; DEXKKDDQIA; EXKKDDQIAA;
XKKDDQIAAA; KKDDQIAAAI; KDDQIAAAIA; DDQIAAAIAL; DQIAAAIALR;
QIAAAIALRG; IAAAIALRGX; AAAIALRGXA; AAIALRGXAK; AIALRGXAKD;
IALRGXAKDG; ALRGXAKDGK; LRGXAKDGKF; RGXAKDGKFA; GXAKDGKFAV;
XAKDGKFAVK; AKDGKFAVKD; KDGKFAVKDG; DGKFAVKDGE; GKFAVKDGEK;
KFAVKDGEKE; FAVKDGEKEK; AVKDGEKEKA; VKDGEKEKAE; KDGEKEKAEG;
DGEKEKAEGA; GEKEKAEGAI; EKEKAEGAIK; KEKAEGAIKG; EKAEGAIKGA;
KAEGAIKGAA; AEGAIKGAAE; EGAIKGAAES; GAIKGAAESA; AIKGAAESAV;
IKGAAESAVR; KGAAESAVRK; GAAESAVRKV; AAESAVRKVL; AESAVRKVLG;
ESAVRKVLGA; SAVRKVLGAI; AVRKVLGAIT; VRKVLGAITG; RKVLGAITGL;
KVLGAITGLI; VLGAITGLIG; LGAITGLIGD; GAITGLIGDA; AITGLIGDAV;
ITGLIGDAVS; TGLIGDAVSS; GLIGDAVSSG; LIGDAVSSGL; IGDAVSSGLR;
GDAVSSGLRK; DAVSSGLRKV; AVSSGLRKVG; VSSGLRKVGD; SSGLRKVGDS;
SGLRKVGDSV; GLRKVGDSVK; LRKVGDSVKA; RKVGDSVKAA; KVGDSVKAAS;
VGDSVKAASK; GDSVKAASKE; DSVKAASKET; SVKAASKETP; VKAASKETPP;
KAASKETPPA; AASKETPPAL; ASKETPPALN; SKETPPALNK

11 mers:
MRGSHHHHHG; RGSHHHHHGS; GSHHHHHGSS; SHHHHHGSSQ;
HHHHHGSSQV; HHHHGSSQVA; HHHGSSQVAD; HHGSSQVADK;
HHGSSQVADKD; HGSSQVADKDD; GSSQVADKDDP; SSQVADKDDPT;
SQVADKDDPTN; QVADKDDPTNK; VADKDDPTNKF; ADKDDPTNKFY;
DKDDPTNKFYQ; KDDPTNKFYQS; DDPTNKFYQSV; DPTNKFYQSVI;
PTNKFYQSVIQ; TNKFYQSVIQL; NKFYQSVIQLG; KFYQSVIQLGN;
FYQSVIQLGNG; YQSVIQLGNGF; QSVIQLGNGFL; SVIQLGNGFLD;
VIQLGNGFLDV; IQLGNGFLDVF; QLGNGFLDVFT; LGNGFLDVFTS;
GNGFLDVFTSF; NGFLDVFTSFG; GFLDVFTSFGG; FLDVFTSFGGL;
LDVFTSFGGLV; DVFTSFGGLVA; VFTSFGGLVAE; FTSFGGLVAEA;
TSFGGLVAEAF; SFGGLVAEAFG; FGGLVAEAFGF; GGLVAEAFGFK;
GLVAEAFGFKS; LVAEAFGFKSD; VAEAFGFKSDP; AEAFGFKSDPK;

661

EAFGFKSDPKK; AFGFKSDPKKS; FGFKSDPKKSD; GFKSDPKKSDV;
FKSDPKKSDVK; KSDPKKSDVKT; SDPKKSDVKTY; DPKKSDVKTYF;
PKKSDVKTYFT; KKSDVKTYFTT; KSDVKTYFTTV; SDVKTYFTTVA;
DVKTYFTTVAA; VKTYFTTVAAK; KTYFTTVAAKL; TYFTTVAAKLE;
YFTTVAAKLEK; FTTVAAKLEKT; TTVAAKLEKTK; TVAAKLEKTKT;
VAAKLEKTKTD; AAKLEKTKTDL; AKLEKTKTDLN; KLEKTKTDLNS;
LEKTKTDLNSL; EKTKTDLNSLP; KTKTDLNSLPK; TKTDLNSLPKE;
KTDLNSLPKEK; TDLNSLPKEKS; DLNSLPKEKSD; LNSLPKEKSDI;
NSLPKEKSDIS; SLPKEKSDISS; LPKEKSDISST; PKEKSDISSTT;
KEKSDISSTTG; EKSDISSTTGK; KSDISSTTGKP; SDISSTTGKPD;
DISSTTGKPDS; ISSTTGKPDST; SSTTGKPDSTG; STTGKPDSTGS;
TTGKPDSTGSV; TGKPDSTGSVG; GKPDSTGSVGT; KPDSTGSVGTA;
PDSTGSVGTAV; DSTGSVGTAVE; STGSVGTAVEG; TGSVGTAVEGA;
GSVGTAVEGAI; SVGTAVEGAIK; VGTAVEGAIKE; GTAVEGAIKEV;
TAVEGAIKEVS; AVEGAIKEVSE; VEGAIKEVSEL; EGAIKEVSELL;
GAIKEVSELLD; AIKEVSELLDK; IKEVSELLDKL; KEVSELLDKLV;
EVSELLDKLVK; VSELLDKLVKA; SELLDKLVKAV; ELLDKLVKAVK;
LLDKLVKAVKT; LDKLVKAVKTA; DKLVKAVKTAE; KLVKAVKTAEG;
LVKAVKTAEGA; VKAVKTAEGAS; KAVKTAEGASS; AVKTAEGASSG;
VKTAEGASSGT; KTAEGASSGTA; TAEGASSGTAA; AEGASSGTAAI;
EGASSGTAAIG; GASSGTAAIGE; ASSGTAAIGEV; SSGTAAIGEVV;
SGTAAIGEVVA; GTAAIGEVVAD; TAAIGEVVADA; AAIGEVVADAD;
AIGEVVADADA; IGEVVADADAA; GEVVADADAAK; EVVADADAAKV;
VVADADAAKVA; VADADAAKVAD; ADADAAKVADK; DADAAKVADKA;
ADAAKVADKAS; DAAKVADKASV; AAKVADKASVK; AKVADKASVKG;
KVADKASVKGI; VADKASVKGIA; ADKASVKGIAK; DKASVKGIAKG;
KASVKGIAKGI; ASVKGIAKGIK; SVKGIAKGIKE; VKGIAKGIKEI;
KGIAKGIKEIV; GIAKGIKEIVE; IAKGIKEIVEA; AKGIKEIVEAA;
KGIKEIVEAAG; GIKEIVEAAGG; IKEIVEAAGGS; KEIVEAAGGSE;
EIVEAAGGSEK; IVEAAGGSEKL; VEAAGGSEKLK; EAAGGSEKLKA;
AAGGSEKLKAV; AGGSEKLKAVA; GGSEKLKAVAA; GSEKLKAVAAA;
SEKLKAVAAAK; EKLKAVAAAKG; KLKAVAAAKGE; LKAVAAAKGEN;
KAVAAAKGENN; AVAAAKGENNK; VAAAKGENNKG; AAAKGENNKGA;
AAKGENNKGAG; AKGENNKGAGK; KGENNKGAGKL; GENNKGAGKLF;
ENNKGAGKLFG; NNKGAGKLFGK; NKGAGKLFGKA; KGAGKLFGKAG;
GAGKLFGKAGA; AGKLFGKAGAA; GKLFGKAGAAA; KLFGKAGAAAH;
LFGKAGAAAHG; FGKAGAAAHGD; GKAGAAAHGDS; KAGAAAHGDSE;
AGAAAHGDSEA; GAAAHGDSEAA; AAAHGDSEAAS; AAHGDSEAASK;
AHGDSEAASKA; HGDSEAASKAA; GDSEAASKAAG; DSEAASKAAGA;
SEAASKAAGAV; EAASKAAGAVS; AASKAAGAVSA; ASKAAGAVSAV;
SKAAGAVSAVS; KAAGAVSAVSG; AAGAVSAVSGE; AGAVSAVSGEQ;
GAVSAVSGEQI; AVSAVSGEQIL; VSAVSGEQILS; SAVSGEQILSA;
AVSGEQILSAI; VSGEQILSAIV; SGEQILSAIVT; GEQILSAIVTA;
EQILSAIVTAA; QILSAIVTAAD; ILSAIVTAADA; LSAIVTAADAA;
SAIVTAADAAE; AIVTAADAAEQ; IVTAADAAEQD; VTAADAAEQDG;
TAADAAEQDGK; AADAAEQDGKK; ADAAEQDGKKP; DAAEQDGKKPE;
AAEQDGKKPEE; AEQDGKKPEEA; EQDGKKPEEAK; QDGKKPEEAKN;
DGKKPEEAKNP; GKKPEEAKNPI; KKPEEAKNPIA; KPEEAKNPIAA;
PEEAKNPIAAA; EEAKNPIAAAI; EAKNPIAAAIG; AKNPIAAAIGD;
KNPIAAAIGDK; NPIAAAIGDKD; PIAAAIGDKDG; IAAAIGDKDGG;
AAAIGDKDGGA; AAIGDKDGGAE; AIGDKDGGAEF; IGDKDGGAEFG;
GDKDGGAEFGQ; DKDGGAEFGQD; KDGGAEFGQDE; DGGAEFGQDEX;
GGAEFGQDEXK; GAEFGQDEXKK; AEFGQDEXKKD; EFGQDEXKKDD;
FGQDEXKKDDQ; GQDEXKKDDQI; QDEXKKDDQIA; DEXKKDDQIAA;
EXKKDDQIAAA; XKKDDQIAAAI; KKDDQIAAAIA; KDDQIAAAIAL;
DDQIAAAIALR; DQIAAAIALRG; QIAAAIALRGX; IAAAIALRGXA;
AAAIALRGXAK; AAIALRGXAKD; AIALRGXAKDG; IALRGXAKDGK;

| | |
|---|---|
| | ALRGXAKDGKF; LRGXAKDGKFA; RGXAKDGKFAV; GXAKDGKFAVK; XAKDGKFAVKD; AKDGKFAVKDG; KDGKFAVKDGE; DGKFAVKDGEK; GKFAVKDGEKE; KFAVKDGEKEK; FAVKDGEKEKA; AVKDGEKEKAE; VKDGEKEKAEG; KDGEKEKAEGA; DGEKEKAEGAI; GEKEKAEGAIK; EKEKAEGAIKG; KEKAEGAIKGA; EKAEGAIKGAA; KAEGAIKGAAE; AEGAIKGAAES; EGAIKGAAESA; GAIKGAAESAV; AIKGAAESAVR; IKGAAESAVRK; KGAAESAVRKV; GAAESAVRKVL; AAESAVRKVLG; AESAVRKVLGA; ESAVRKVLGAI; SAVRKVLGAIT; AVRKVLGAITG; VRKVLGAITGL; RKVLGAITGLI; KVLGAITGLIG; VLGAITGLIGD; LGAITGLIGDA; GAITGLIGDAV; AITGLIGDAVS; ITGLIGDAVSS; TGLIGDAVSSG; GLIGDAVSSGL; LIGDAVSSGLR; IGDAVSSGLRK; GDAVSSGLRKV; DAVSSGLRKVG; AVSSGLRKVGD; VSSGLRKVGDS; SSGLRKVGDSV; SGLRKVGDSVK; GLRKVGDSVKA; LRKVGDSVKAA; RKVGDSVKAAS; KVGDSVKAASK; VGDSVKAASKE; GDSVKAASKET; DSVKAASKETP; SVKAASKETPP; VKAASKETPPA; KAASKETPPAL; AASKETPPALN; ASKETPPALNK; |
| CAA57807.1 Associated protein A (BapA)[Borrelia burgdorferi] | SEQ ID NO 18548-19189/ 8 mers: MKKISLLI; KKISLLIF; KISLLIFL; ISLLIFLF; SLLIFLFL; LLIFLFLF; LIFLFLFV; IFLFLFVV; FLFLFVVS; LFLFVVSL; FLFVVSLS; LFVVSLSA; FVVSLSAN; VVSLSANI; VSLSANIE; SLSANIEE; LSANIEEN; SANIEENY; ANIEENYT; NIEENYTE; IEENYTET; EENYTETK; ENYTETKR; NYTETKRA; YTETKRAF; TETKRAFS; ETKRAFSK; TKRAFSKE; KRAFSKED; RAFSKEDF; AFSKEDFN; FSKEDFNL; SKEDFNLI; KEDFNLIN; EDFNLINK; DFNLINKR; FNLINKRL; NLINKRLD; LINKRLDN; INKRLDNY; NKRLDNYD; KRLDNYDF; RLDNYDFK; LDNYDFKN; DNYDFKNE; NYDFKNEY; YDFKNEYE; DFKNEYEK; FKNEYEKS; KNEYEKSH; NEYEKSHV; EYEKSHVF; YEKSHVFS; EKSHVFSD; KSHVFSDA; SHVFSDAP; HVFSDAPR; VFSDAPRI; FSDAPRIR; SDAPRIRG; DAPRIRGD; APRIRGDL; PRIRGDLR; RIRGDLRK; IRGDLRKI; RGDLRKIG; GDLRKIGI; DLRKIGIK; LRKIGIKE; RKIGIKEK; KIGIKEKS; IGIKEKSV; GIKEKSVF; IKEKSVFL; KEKSVFLD; EKSVFLDA; KSVFLDAL; SVFLDALE; VFLDALEA; FLDALEAI; LDALEAIE; DALEAIEY; ALEAIEYL; LEAIEYLI; EAIEYLIK; AIEYLIKI; IEYLIKIK; EYLIKIKI; YLIKIKIS; LIKIKIST; IKIKISTD; KIKISTDS; IKISTDSI; KISTDSIF; ISTDSIFL; STDSIFLS; TDSIFLSE; DSIFLSED; SIFLSEDM; IFLSEDMI; FLSEDMIR; LSEDMIRL; SEDMIRLI; EDMIRLIG; DMIRLIGS; MIRLIGSY; IRLIGSYP; RLIGSYPD; LIGSYPDS; IGSYPDSI; GSYPDSIF; SYPDSIFN; YPDSIFNY; PDSIFNYL; DSIFNYLI; SIFNYLIQ; IFNYLIQL; FNYLIQLN; NYLIQLNS; YLIQLNSD; LIQLNSDK; IQLNSDKI; QLNSDKID; LNSDKIDY; NSDKIDYA; SDKIDYAE; DKIDYAEK; KIDYAEKY; IDYAEKYG; DYAEKYGD; YAEKYGDN; AEKYGDNA; EKYGDNAR; KYGDNARN; YGDNARNN; GDNARNNF; DNARNNFK; NARNNFKK; ARNNFKKD; RNNFKKDY; NNFKKDYS; NFKKDYSE; FKKDYSED; KKDYSEDK; KDYSEDKA; DYSEDKAN; YSEDKANT; SEDKANTV; EDKANTVK; DKANTVKQ; KANTVKQI; ANTVKQIL; NTVKQILK; TVKQILKQ; VKQILKQI; KQILKQIL; QILKQILA; ILKQILAD; LKQILADL; KQILADLP; QILADLPK; ILADLPKD; 9 mers: MKKISLLIF; KKISLLIFL; KISLLIFLF; ISLLIFLFL; SLLIFLFLF; LLIFLFLFV; LIFLFLFVV; IFLFLFVVS; FLFLFVVSL; LFLFVVSLS; FLFVVSLSA; LFVVSLSAN; FVVSLSANI; VVSLSANIE; VSLSANIEE; SLSANIEEN; LSANIEENY; SANIEENYT; ANIEENYTE; NIEENYTET; IEENYTETK; EENYTETKR; ENYTETKRA; NYTETKRAF; YTETKRAFS; TETKRAFSK; ETKRAFSKE; TKRAFSKED; KRAFSKEDF; RAFSKEDFN; AFSKEDFNL; FSKEDFNLI; SKEDFNLIN; KEDFNLINK; EDFNLINKR; DFNLINKRL; FNLINKRLD; NLINKRLDN; LINKRLDNY; INKRLDNYD; NKRLDNYDF; KRLDNYDFK; RLDNYDFKN; LDNYDFKNE; DNYDFKNEY; |

663

NYDFKNEYE; YDFKNEYEK; DFKNEYEKS; FKNEYEKSH; KNEYEKSHV;
NEYEKSHVF; EYEKSHVFS; YEKSHVFSD; EKSHVFSDA; KSHVFSDAP;
SHVFSDAPR; HVFSDAPRI; VFSDAPRIR; FSDAPRIRG; SDAPRIRGD;
DAPRIRGDL; APRIRGDLR; PRIRGDLRK; RIRGDLRKI; IRGDLRKIG;
RGDLRKIGI; GDLRKIGIK; DLRKIGIKE; LRKIGIKEK; RKIGIKEKS;
KIGIKEKSV; IGIKEKSVF; GIKEKSVFL; IKEKSVFLD; KEKSVFLDA;
EKSVFLDAL; KSVFLDALE; SVFLDALEA; VFLDALEAI; FLDALEAIE;
LDALEAIEY; DALEAIEYL; ALEAIEYLI; LEAIEYLIK; EAIEYLIKI;
AIEYLIKIK; IEYLIKIKI; EYLIKIKIS; YLIKIKIST; LIKIKISTD;
IKIKISTDS; KIKISTDSI; IKISTDSIF; KISTDSIFL; ISTDSIFLS;
STDSIFLSE; TDSIFLSED; DSIFLSEDM; SIFLSEDMI; IFLSEDMIR;
FLSEDMIRL; LSEDMIRLI; SEDMIRLIG; EDMIRLIGS; DMIRLIGSY;
MIRLIGSYP; IRLIGSYPD; RLIGSYPDS; LIGSYPDSI; IGSYPDSIF;
GSYPDSIFN; SYPDSIFNY; YPDSIFNYL; PDSIFNYLI; DSIFNYLIQ;
SIFNYLIQL; IFNYLIQLN; FNYLIQLNS; NYLIQLNSD; YLIQLNSDK;
LIQLNSDKI; IQLNSDKID; QLNSDKIDY; LNSDKIDYA; NSDKIDYAE;
SDKIDYAEK; DKIDYAEKY; KIDYAEKYG; IDYAEKYGD; DYAEKYGDN;
YAEKYGDNA; AEKYGDNAR; EKYGDNARN; KYGDNARNN; YGDNARNNF;
GDNARNNFK; DNARNNFKK; NARNNFKKD; ARNNFKKDY; RNNFKKDYS;
NNFKKDYSE; NFKKDYSED; FKKDYSEDK; KKDYSEDKA; KDYSEDKAN;
DYSEDKANT; YSEDKANTV; SEDKANTVK; EDKANTVKQ; DKANTVKQI;
KANTVKQIL; ANTVKQILK; NTVKQILKQ; TVKQILKQI; VKQILKQIL;
KQILKQILA; QILKQILAD; ILKQILADL; LKQILADLP; KQILADLPK;
QILADLPKD;

10 mers:
MKKISLLIFL; KKISLLIFLF; KISLLIFLFL; ISLLIFLFLV; SLLIFLFLFV;
LLIFLFLFVV; LIFLFLFVVS; IFLFLFVVSL; FLFLFVVSLS; LFLFVVSLSA;
FLFVVSLSAN; LFVVSLSANI; FVVSLSANIE; VVSLSANIEE; VSLSANIEEN;
SLSANIEENY; LSANIEENYT; SANIEENYTE; ANIEENYTET; NIEENYTETK;
IEENYTETKR; EENYTETKRA; ENYTETKRAF; NYTETKRAFS; YTETKRAFSK;
TETKRAFSKE; ETKRAFSKED; TKRAFSKEDF; KRAFSKEDFN; RAFSKEDFNL;
AFSKEDFNLI; FSKEDFNLIN; SKEDFNLINK; KEDFNLINKR; EDFNLINKRL;
DFNLINKRLD; FNLINKRLDN; NLINKRLDNY; LINKRLDNYD; INKRLDNYDF;
NKRLDNYDFK; KRLDNYDFKN; RLDNYDFKNE; LDNYDFKNEY; DNYDFKNEYE;
NYDFKNEYEK; YDFKNEYEKS; DFKNEYEKSH; FKNEYEKSHV; KNEYEKSHVF;
NEYEKSHVFS; EYEKSHVFSD; YEKSHVFSDA; EKSHVFSDAP; KSHVFSDAPR;
SHVFSDAPRI; HVFSDAPRIR; VFSDAPRIRG; FSDAPRIRGD; SDAPRIRGDL;
DAPRIRGDLR; APRIRGDLRK; PRIRGDLRKI; RIRGDLRKIG; IRGDLRKIGI;
RGDLRKIGIK; GDLRKIGIKE; DLRKIGIKEK; LRKIGIKEKS; RKIGIKEKSV;
KIGIKEKSVF; IGIKEKSVFL; GIKEKSVFLD; IKEKSVFLDA; KEKSVFLDAL;
EKSVFLDALE; KSVFLDALEA; SVFLDALEAI; VFLDALEAIE; FLDALEAIEY;
LDALEAIEYL; DALEAIEYLI; ALEAIEYLIK; LEAIEYLIKI; EAIEYLIKIK;
AIEYLIKIKI; IEYLIKIKIS; EYLIKIKIST; YLIKIKISTD; LIKIKISTDS;
IKIKISTDSI; KIKISTDSIF; IKISTDSIFL; KISTDSIFLS; ISTDSIFLSE;
STDSIFLSED; TDSIFLSEDM; DSIFLSEDMI; SIFLSEDMIR; IFLSEDMIRL;
FLSEDMIRLI; LSEDMIRLIG; SEDMIRLIGS; EDMIRLIGSY; DMIRLIGSYP;
MIRLIGSYPD; IRLIGSYPDS; RLIGSYPDSI; LIGSYPDSIF; IGSYPDSIFN;
GSYPDSIFNY; SYPDSIFNYL; YPDSIFNYLI; PDSIFNYLIQ; DSIFNYLIQL;
SIFNYLIQLN; IFNYLIQLNS; FNYLIQLNSD; NYLIQLNSDK; YLIQLNSDKI;
LIQLNSDKID; IQLNSDKIDY; QLNSDKIDYA; LNSDKIDYAE; NSDKIDYAEK;
SDKIDYAEKY; DKIDYAEKYG; KIDYAEKYGD; IDYAEKYGDN; DYAEKYGDNA;
YAEKYGDNAR; AEKYGDNARN; EKYGDNARNN; KYGDNARNNF; YGDNARNNFK;
GDNARNNFKK; DNARNNFKKD; NARNNFKKDY; ARNNFKKDYS; RNNFKKDYSE;
NNFKKDYSED; NFKKDYSEDK; FKKDYSEDKA; KKDYSEDKAN; KDYSEDKANT;
DYSEDKANTV; YSEDKANTVK; SEDKANTVKQ; EDKANTVKQI; DKANTVKQIL;
KANTVKQILK; ANTVKQILKQ; NTVKQILKQI; TVKQILKQIL; VKQILKQILA;

| | |
|---|---|
| | KQILKQILAD; QILKQILADL; ILKQILADLP; LKQILADLPK; KQILADLPKD;<br><br>11 mers:<br>MKKISLLIFLF; KKISLLIFLFL; KISLLIFLFLF; ISLLIFLFLFV;<br>SLLIFLFLFVV; LLIFLFLFVVS; LIFLFLFVVSL; IFLFLFVVSLS;<br>FLFLFVVSLSA; LFLFVVSLSAN; FLFVVSLSANI; LFVVSLSANIE;<br>FVVSLSANIEE; VVSLSANIEEN; VSLSANIEENY; SLSANIEENYT;<br>LSANIEENYTE; SANIEENYTET; ANIEENYTETK; NIEENYTETKR;<br>IEENYTETKRA; EENYTETKRAF; ENYTETKRAFS; NYTETKRAFSK;<br>YTETKRAFSKE; TETKRAFSKED; ETKRAFSKEDF; TKRAFSKEDFN;<br>KRAFSKEDFNL; RAFSKEDFNLI; AFSKEDFNLIN; FSKEDFNLINK;<br>SKEDFNLINKR; KEDFNLINKRL; EDFNLINKRLD; DFNLINKRLDN;<br>FNLINKRLDNY; NLINKRLDNYD; LINKRLDNYDF; INKRLDNYDFK;<br>NKRLDNYDFKN; KRLDNYDFKNE; RLDNYDFKNEY; LDNYDFKNEYE;<br>DNYDFKNEYEK; NYDFKNEYEKS; YDFKNEYEKSH; DFKNEYEKSHV;<br>FKNEYEKSHVF; KNEYEKSHVFS; NEYEKSHVFSD; EYEKSHVFSDA;<br>YEKSHVFSDAP; EKSHVFSDAPR; KSHVFSDAPRI; SHVFSDAPRIR;<br>HVFSDAPRIRG; VFSDAPRIRGD; FSDAPRIRGDL; SDAPRIRGDLR;<br>DAPRIRGDLRK; APRIRGDLRKI; PRIRGDLRKIG; RIRGDLRKIGI;<br>IRGDLRKIGIK; RGDLRKIGIKE; GDLRKIGIKEK; DLRKIGIKEKS;<br>LRKIGIKEKSV; RKIGIKEKSVF; KIGIKEKSVFL; IGIKEKSVFLD;<br>GIKEKSVFLDA; IKEKSVFLDAL; KEKSVFLDALE; EKSVFLDALEA;<br>KSVFLDALEAI; SVFLDALEAIE; VFLDALEAIEY; FLDALEAIEYL;<br>LDALEAIEYLI; DALEAIEYLIK; ALEAIEYLIKI; LEAIEYLIKIK;<br>EAIEYLIKIKI; AIEYLIKIKIS; IEYLIKIKIST; EYLIKIKISTD;<br>YLIKIKISTDS; LIKIKISTDSI; IKIKISTDSIF; KIKISTDSIFL;<br>IKISTDSIFLS; KISTDSIFLSE; ISTDSIFLSED; STDSIFLSEDM;<br>TDSIFLSEDMI; DSIFLSEDMIR; SIFLSEDMIRL; IFLSEDMIRLI;<br>FLSEDMIRLIG; LSEDMIRLIGS; SEDMIRLIGSY; EDMIRLIGSYP;<br>DMIRLIGSYPD; MIRLIGSYPDS; IRLIGSYPDSI; RLIGSYPDSIF;<br>LIGSYPDSIFN; IGSYPDSIFNY; GSYPDSIFNYL; SYPDSIFNYLI;<br>YPDSIFNYLIQ; PDSIFNYLIQL; DSIFNYLIQLN; SIFNYLIQLNS;<br>IFNYLIQLNSD; FNYLIQLNSDK; NYLIQLNSDKI; YLIQLNSDKID;<br>LIQLNSDKIDY; IQLNSDKIDYA; QLNSDKIDYAE; LNSDKIDYAEK;<br>NSDKIDYAEKY; SDKIDYAEKYG; DKIDYAEKYGD; KIDYAEKYGDN;<br>IDYAEKYGDNA; DYAEKYGDNAR; YAEKYGDNARN; AEKYGDNARNN;<br>EKYGDNARNNF; KYGDNARNNFK; YGDNARNNFKK; GDNARNNFKKD;<br>DNARNNFKKDY; NARNNFKKDYS; ARNNFKKDYSE; RNNFKKDYSED;<br>NNFKKDYSEDK; NFKKDYSEDKA; FKKDYSEDKAN; KKDYSEDKANT;<br>KDYSEDKANTV; DYSEDKANTVK; YSEDKANTVKQ; SEDKANTVKQI;<br>EDKANTVKQIL; DKANTVKQILK; KANTVKQILKQ; ANTVKQILKQI;<br>NTVKQILKQIL; TVKQILKQILA; VKQILKQILAD; KQILKQILADL;<br>QILKQILADLP; ILKQILADLPK; LKQILADLPKD; |
| Seq19 | SEQ ID NO 19190-20455/<br>8 mers:<br>MCAFLLLN; CAFLLLNL; AFLLLNLV; FLLLNLVN; LLLNLVNC;<br>LLNLVNCK; LNLVNCKF; NLVNCKFD; LVNCKFDS; VNCKFDSL;<br>NCKFDSLN; CKFDSLNL; KFDSLNLS; FDSLNLST; DSLNLSTK;<br>SLNLSTKS; LNLSTKSV; NLSTKSVD; LSTKSVDD; STKSVDDK;<br>TKSVDDKN; KSVDDKNN; SVDDKNNS; VDDKNNSI; DDKNNSIA;<br>DKNNSIAK; KNNSIAKL; NNSIAKLL; NSIAKLLQ; SIAKLLQH;<br>IAKLLQHL; AKLLQHLS; KLLQHLSK; LLQHLSKS; LQHLSKSE;<br>QHLSKSED; HLSKSEDQ; LSKSEDQA; SKSEDQAN; KSEDQANK;<br>SEDQANKT; EDQANKTS; DQANKTST; QANKTSTS; ANKTSTSE; |

NKTSTSED; KTSTSEDQ; TSTSEDQK; STSEDQKE; TSEDQKEL;
SEDQKELE; EDQKELEI; DQKELEIT; QKELEITE; KELEITEN;
ELEITENK; LEITENKE; EITENKEQ; ITENKEQE; TENKEQEH;
ENKEQEHE; NKEQEHEK; KEQEHEKL; EQEHEKLS; QEHEKLSQ;
EHEKLSQV; HEKLSQVA; EKLSQVAQ; KLSQVAQH; LSQVAQHA;
SQVAQHAP; QVAQHAPN; VAQHAPNS; AQHAPNSK; QHAPNSKI;
HAPNSKIE; APNSKIEK; PNSKIEKV; NSKIEKVK; SKIEKVKS;
KIEKVKSD; IEKVKSDG; EKVKSDGK; KVKSDGKP; VKSDGKPV;
KSDGKPVP; SDGKPVPG; DGKPVPGD; GKPVPGDK; KPVPGDKI;
PVPGDKIL; VPGDKILS; PGDKILSS; GDKILSSN; DKILSSNK;
KILSSNKD; ILSSNKDI; LSSNKDIY; SSNKDIYN; SNKDIYNS;
NKDIYNSY; KDIYNSYI; DIYNSYIP; IYNSYIPE; YNSYIPEV;
NSYIPEVK; SYIPEVKE; YIPEVKEE; IPEVKEEI; PEVKEEIV;
EVKEEIVY; VKEEIVYE; KEEIVYEI; EEIVYEIL; EIVYEILE;
IVYEILEE; VYEILEEV; YEILEEVI; EILEEVII; ILEEVIIP;
LEEVIIPE; EEVIIPET; EVIIPETK; VIIPETKI; IIPETKIP;
IPETKIPE; PETKIPEI; ETKIPEIT; TKIPEITE; KIPEITEE;
IPEITEEV; PEITEEVI; EITEEVIM; ITEEVIMP; TEEVIMPI;
EEVIMPIP; EVIMPIPQ; VIMPIPQT; IMPIPQTI; MPIPQTID;
PIPQTIDF; IPQTIDFY; PQTIDFYI; QTIDFYIE; TIDFYIEP;
IDFYIEPR; DFYIEPRP; FYIEPRPI; YIEPRPIS; IEPRPISS;
EPRPISSF; PRPISSFL; RPISSFLT; PISSFLTQ; ISSFLTQG;
SSFLTQGT; SFLTQGTS; FLTQGTSP; LTQGTSPS; TQGTSPSI;
QGTSPSIT; GTSPSITS; TSPSITST; SPSITSTI; PSITSTIK;
SITSTIKS; ITSTIKSY; TSTIKSYK; STIKSYKE; TIKSYKEL;
IKSYKELA; KSYKELAK; SYKELAKE; YKELAKEK; KELAKEKI;
ELAKEKIN; LAKEKINN; AKEKINNG; KEKINNGL; EKINNGLN;
KINNGLNI; INNGLNIV; NNGLNIVQ; NGLNIVQK; GLNIVQKI;
LNIVQKIT; NIVQKITQ; IVQKITQN; VQKITQNI; QKITQNID;
KITQNIDN; ITQNIDNI; TQNIDNIT; QNIDNITE; NIDNITEN;
IDNITENL; DNITENLN; NITENLNS; ITENLNSK; TENLNSKE;
ENLNSKET; NLNSKETP; LNSKETPK; NSKETPKE; SKETPKEI;
KETPKEIS; ETPKEISG; TPKEISGK; PKEISGKE; KEISGKEV;
EISGKEVE; ISGKEVEE; SGKEVEEK; GKEVEEKI; KEVEEKIT;
EVEEKITH; VEEKITHP; EEKITHPI; EKITHPIF; KITHPIFD;
ITHPIFDH; THPIFDHI; HPIFDHIT; PIFDHITG; IFDHITGS;
FDHITGSG; DHITGSGN; HITGSGNN; ITGSGNNP; TGSGNNPG;
GSGNNPGQ; SGNNPGQD; GNNPGQDS; NNPGQDSI; NPGQDSIS;
PGQDSISN; GQDSISNT; QDSISNTW; DSISNTWG; SISNTWGE;
ISNTWGEG; SNTWGEGL; NTWGEGLE; TWGEGLEI; WGEGLEIG;
GEGLEIGG; EGLEIGGD; GLEIGGDS; LEIGGDSN; EIGGDSNF;
IGGDSNFF; GGDSNFFT; GDSNFFTN; DSNFFTNL; SNFFTNLE;
NFFTNLEE; FFTNLEEV; FTNLEEVR; TNLEEVRS; NLEEVRSS;
LEEVRSSI; EEVRSSIR; EVRSSIRT; VRSSIRTK; RSSIRTKI;
SSIRTKIK; SIRTKIKV; IRTKIKVS; RTKIKVSD; TKIKVSDG;
KIKVSDGT; IKVSDGTE; KVSDGTEQ; VSDGTEQT; SDGTEQTK;
DGTEQTKD; GTEQTKDK; TEQTKDKV; EQTKDKVE; QTKDKVEI;
TKDKVEID; KDKVEIDE; DKVEIDEI; KVEIDEII; VEIDEIIE;
EIDEIIED; IDEIIEDL; DEIIEDLQ; EIIEDLQK; IIEDLQKL;
IEDLQKLK; EDLQKLKE; DLQKLKEF; LQKLKEFL; QKLKEFLE;
KLKEFLEK; LKEFLEKL; KEFLEKLK; EFLEKLKK; FLEKLKKY;

666

LEKLKKYL; EKLKKYLK; KLKKYLKD; LKKYLKDT; KKYLKDTN;
KYLKDTNN; YLKDTNNL; LKDTNNLS; KDTNNLSA; DTNNLSAI;
TNNLSAIE; NNLSAIEE; NLSAIEES; LSAIEESV; SAIEESVK;
AIEESVKG; IEESVKGL; EESVKGLS;

9 mers:
MCAFLLLNL; CAFLLLNLV; AFLLLNLVN; FLLLNLVNC; LLLNLVNCK;
LLNLVNCKF; LNLVNCKFD; NLVNCKFDS; LVNCKFDSL; VNCKFDSLN;
NCKFDSLNL; CKFDSLNLS; KFDSLNLST; FDSLNLSTK; DSLNLSTKS;
SLNLSTKSV; LNLSTKSVD; NLSTKSVDD; LSTKSVDDK; STKSVDDKN;
TKSVDDKNN; KSVDDKNNS; SVDDKNNSI; VDDKNNSIA; DDKNNSIAK;
DKNNSIAKL; KNNSIAKLL; NNSIAKLLQ; NSIAKLLQH; SIAKLLQHL;
IAKLLQHLS; AKLLQHLSK; KLLQHLSKS; LLQHLSKSE; LQHLSKSED;
QHLSKSEDQ; HLSKSEDQA; LSKSEDQAN; SKSEDQANK; KSEDQANKT;
SEDQANKTS; EDQANKTST; DQANKTSTS; QANKTSTSE; ANKTSTSED;
NKTSTSEDQ; KTSTSEDQK; TSTSEDQKE; STSEDQKEL; TSEDQKELE;
SEDQKELEI; EDQKELEIT; DQKELEITE; QKELEITEN; KELEITENK;
ELEITENKE; LEITENKEQ; EITENKEQE; ITENKEQEH; TENKEQEHE;
ENKEQEHEK; NKEQEHEKL; KEQEHEKLS; EQEHEKLSQ; QEHEKLSQV;
EHEKLSQVA; HEKLSQVAQ; EKLSQVAQH; KLSQVAQHA; LSQVAQHAP;
SQVAQHAPN; QVAQHAPNS; VAQHAPNSK; AQHAPNSKI; QHAPNSKIE;
HAPNSKIEK; APNSKIEKV; PNSKIEKVK; NSKIEKVKS; SKIEKVKSD;
KIEKVKSDG; IEKVKSDGK; EKVKSDGKP; KVKSDGKPV; VKSDGKPVP;
KSDGKPVPG; SDGKPVPGD; DGKPVPGDK; GKPVPGDKI; KPVPGDKIL;
PVPGDKILS; VPGDKILSS; PGDKILSSN; GDKILSSNK; DKILSSNKD;
KILSSNKDI; ILSSNKDIY; LSSNKDIYN; SSNKDIYNS; SNKDIYNSY;
NKDIYNSYI; KDIYNSYIP; DIYNSYIPE; IYNSYIPEV; YNSYIPEVK;
NSYIPEVKE; SYIPEVKEE; YIPEVKEEI; IPEVKEEIV; PEVKEEIVY;
EVKEEIVYE; VKEEIVYEI; KEEIVYEIL; EEIVYEILE; EIVYEILEE;
IVYEILEEV; VYEILEEVI; YEILEEVII; EILEEVIIP; ILEEVIIPE;
LEEVIIPET; EEVIIPETK; EVIIPETKI; VIIPETKIP; IIPETKIPE;
IPETKIPEI; PETKIPEIT; ETKIPEITE; TKIPEITEE; KIPEITEEV;
IPEITEEVI; PEITEEVIM; EITEEVIMP; ITEEVIMPI; TEEVIMPIP;
EEVIMPIPQ; EVIMPIPQT; VIMPIPQTI; IMPIPQTID; MPIPQTIDF;
PIPQTIDFY; IPQTIDFYI; PQTIDFYIE; QTIDFYIEP; TIDFYIEPR;
IDFYIEPRP; DFYIEPRPI; FYIEPRPIS; YIEPRPISS; IEPRPISSF;
EPRPISSFL; PRPISSFLT; RPISSFLTQ; PISSFLTQG; ISSFLTQGT;
SSFLTQGTS; SFLTQGTSP; FLTQGTSPS; LTQGTSPSI; TQGTSPSIT;
QGTSPSITS; GTSPSITST; TSPSITSTI; SPSITSTIK; PSITSTIKS;
SITSTIKSY; ITSTIKSYK; TSTIKSYKE; STIKSYKEL; TIKSYKELA;
IKSYKELAK; KSYKELAKE; SYKELAKEK; YKELAKEKI; KELAKEKIN;
ELAKEKINN; LAKEKINNG; AKEKINNGL; KEKINNGLN; EKINNGLNI;
KINNGLNIV; INNGLNIVQ; NNGLNIVQK; NGLNIVQKI; GLNIVQKIT;
LNIVQKITQ; NIVQKITQN; IVQKITQNI; VQKITQNID; QKITQNIDN;
KITQNIDNI; ITQNIDNIT; TQNIDNITE; QNIDNITEN; NIDNITENL;
IDNITENLN; DNITENLNS; NITENLNSK; ITENLNSKE; TENLNSKET;
ENLNSKETP; NLNSKETPK; LNSKETPKE; NSKETPKEI; SKETPKEIS;
KETPKEISG; ETPKEISGK; TPKEISGKE; PKEISGKEV; KEISGKEVE;
EISGKEVEE; ISGKEVEEK; SGKEVEEKI; GKEVEEKIT; KEVEEKITH;
EVEEKITHP; VEEKITHPI; EEKITHPIF; EKITHPIFD; KITHPIFDH;
ITHPIFDHI; THPIFDHIT; HPIFDHITG; PIFDHITGS; IFDHITGSG;

FDHITGSGN; DHITGSGNN; HITGSGNNP; ITGSGNNPG; TGSGNNPGQ;
GSGNNPGQD; SGNNPGQDS; GNNPGQDSI; NNPGQDSIS; NPGQDSISN;
PGQDSISNT; GQDSISNTW; QDSISNTWG; DSISNTWGE; SISNTWGEG;
ISNTWGEGL; SNTWGEGLE; NTWGEGLEI; TWGEGLEIG; WGEGLEIGG;
GEGLEIGGD; EGLEIGGDS; GLEIGGDSN; LEIGGDSNF; EIGGDSNFF;
IGGDSNFFT; GGDSNFFTN; GDSNFFTNL; DSNFFTNLE; SNFFTNLEE;
NFFTNLEEV; FFTNLEEVR; FTNLEEVRS; TNLEEVRSS; NLEEVRSSI;
LEEVRSSIR; EEVRSSIRT; EVRSSIRTK; VRSSIRTKI; RSSIRTKIK;
SSIRTKIKV; SIRTKIKVS; IRTKIKVSD; RTKIKVSDG; TKIKVSDGT;
KIKVSDGTE; IKVSDGTEQ; KVSDGTEQT; VSDGTEQTK; SDGTEQTKD;
DGTEQTKDK; GTEQTKDKV; TEQTKDKVE; EQTKDKVEI; QTKDKVEID;
TKDKVEIDE; KDKVEIDEI; DKVEIDEII; KVEIDEIIE; VEIDEIIED;
EIDEIIEDL; IDEIIEDLQ; DEIIEDLQK; EIIEDLQKL; IIEDLQKLK;
IEDLQKLKE; EDLQKLKEF; DLQKLKEFL; LQKLKEFLE; QKLKEFLEK;
KLKEFLEKL; LKEFLEKLK; KEFLEKLKK; EFLEKLKKY; FLEKLKKYL;
LEKLKKYLK; EKLKKYLKD; KLKKYLKDT; LKKYLKDTN; KKYLKDTNN;
KYLKDTNNL; YLKDTNNLS; LKDTNNLSA; KDTNNLSAI; DTNNLSAIE;
TNNLSAIEE; NNLSAIEES; NLSAIEESV; LSAIEESVK; SAIEESVKG;
AIEESVKGL; IEESVKGLS;

10 mers:
MCAFLLLNLV; CAFLLLNLVN; AFLLLNLVNC; FLLLNLVNCK;
LLLNLVNCKF; LLNLVNCKFD; LNLVNCKFDS; NLVNCKFDSL;
LVNCKFDSLN; VNCKFDSLNL; NCKFDSLNLS; CKFDSLNLST;
KFDSLNLSTK; FDSLNLSTKS; DSLNLSTKSV; SLNLSTKSVD;
LNLSTKSVDD; NLSTKSVDDK; LSTKSVDDKN; STKSVDDKNN;
TKSVDDKNNS; KSVDDKNNSI; SVDDKNNSIA; VDDKNNSIAK;
DDKNNSIAKL; DKNNSIAKLL; KNNSIAKLLQ; NNSIAKLLQH;
NSIAKLLQHL; SIAKLLQHLS; IAKLLQHLSK; AKLLQHLSKS;
KLLQHLSKSE; LLQHLSKSED; LQHLSKSEDQ; QHLSKSEDQA;
HLSKSEDQAN; LSKSEDQANK; SKSEDQANKT; KSEDQANKTS;
SEDQANKTST; EDQANKTSTS; DQANKTSTSE; QANKTSTSED;
ANKTSTSEDQ; NKTSTSEDQK; KTSTSEDQKE; TSTSEDQKEL;
STSEDQKELE; TSEDQKELEI; SEDQKELEIT; EDQKELEITE;
DQKELEITEN; QKELEITENK; KELEITENKE; ELEITENKEQ;
LEITENKEQE; EITENKEQEH; ITENKEQEHE; TENKEQEHEK;
ENKEQEHEKL; NKEQEHEKLS; KEQEHEKLSQ; EQEHEKLSQV;
QEHEKLSQVA; EHEKLSQVAQ; HEKLSQVAQH; EKLSQVAQHA;
KLSQVAQHAP; LSQVAQHAPN; SQVAQHAPNS; QVAQHAPNSK;
VAQHAPNSKI; AQHAPNSKIE; QHAPNSKIEK; HAPNSKIEKV;
APNSKIEKVK; PNSKIEKVKS; NSKIEKVKSD; SKIEKVKSDG;
KIEKVKSDGK; IEKVKSDGKP; EKVKSDGKPV; KVKSDGKPVP;
VKSDGKPVPG; KSDGKPVPGD; SDGKPVPGDK; DGKPVPGDKI;
GKPVPGDKIL; KPVPGDKILS; PVPGDKILSS; VPGDKILSSN;
PGDKILSSNK; GDKILSSNKD; DKILSSNKDI; KILSSNKDIY;
ILSSNKDIYN; LSSNKDIYNS; SSNKDIYNSY; SNKDIYNSYI;
NKDIYNSYIP; KDIYNSYIPE; DIYNSYIPEV; IYNSYIPEVK;
YNSYIPEVKE; NSYIPEVKEE; SYIPEVKEEI; YIPEVKEEIV;
IPEVKEEIVY; PEVKEEIVYE; EVKEEIVYEI; VKEEIVYEIL;
KEEIVYEILE; EEIVYEILEE; EIVYEILEEV; IVYEILEEVI;
VYEILEEVII; YEILEEVIIP; EILEEVIIPE; ILEEVIIPET;

| | LEEVIIPETK; EEVIIPETKI; EVIIPETKIP; VIIPETKIPE;<br>IIPETKIPEI; IPETKIPEIT; PETKIPEITE; ETKIPEITEE;<br>TKIPEITEEV; KIPEITEEVI; IPEITEEVIM; PEITEEVIMP;<br>EITEEVIMPI; ITEEVIMPIP; TEEVIMPIPQ; EEVIMPIPQT;<br>EVIMPIPQTI; VIMPIPQTID; IMPIPQTIDF; MPIPQTIDFY;<br>PIPQTIDFYI; IPQTIDFYIE; PQTIDFYIEP; QTIDFYIEPR;<br>TIDFYIEPRP; IDFYIEPRPI; DFYIEPRPIS; FYIEPRPISS;<br>YIEPRPISSF; IEPRPISSFL; EPRPISSFLT; PRPISSFLTQ;<br>RPISSFLTQG; PISSFLTQGT; ISSFLTQGTS; SSFLTQGTSP;<br>SFLTQGTSPS; FLTQGTSPSI; LTQGTSPSIT; TQGTSPSITS;<br>QGTSPSITST; GTSPSITSTI; TSPSITSTIK; SPSITSTIKS;<br>PSITSTIKSY; SITSTIKSYK; ITSTIKSYKE; TSTIKSYKEL;<br>STIKSYKELA; TIKSYKELAK; IKSYKELAKE; KSYKELAKEK;<br>SYKELAKEKI; YKELAKEKIN; KELAKEKINN; ELAKEKINNG;<br>LAKEKINNGL; AKEKINNGLN; KEKINNGLNI; EKINNGLNIV;<br>KINNGLNIVQ; INNGLNIVQK; NNGLNIVQKI; NGLNIVQKIT;<br>GLNIVQKITQ; LNIVQKITQN; NIVQKITQNI; IVQKITQNID;<br>VQKITQNIDN; QKITQNIDNI; KITQNIDNIT; ITQNIDNITE;<br>TQNIDNITEN; QNIDNITENL; NIDNITENLN; IDNITENLNS;<br>DNITENLNSK; NITENLNSKE; ITENLNSKET; TENLNSKETP;<br>ENLNSKETPK; NLNSKETPKE; LNSKETPKEI; NSKETPKEIS;<br>SKETPKEISG; KETPKEISGK; ETPKEISGKE; TPKEISGKEV;<br>PKEISGKEVE; KEISGKEVEE; EISGKEVEEK; ISGKEVEEKI;<br>SGKEVEEKIT; GKEVEEKITH; KEVEEKITHP; EVEEKITHPI;<br>VEEKITHPIF; EEKITHPIFD; EKITHPIFDH; KITHPIFDHI;<br>ITHPIFDHIT; THPIFDHITG; HPIFDHITGS; PIFDHITGSG;<br>IFDHITGSGN; FDHITGSGNN; DHITGSGNNP; HITGSGNNPG;<br>ITGSGNNPGQ; TGSGNNPGQD; GSGNNPGQDS; SGNNPGQDSI;<br>GNNPGQDSIS; NNPGQDSISN; NPGQDSISNT; PGQDSISNTW;<br>GQDSISNTWG; QDSISNTWGE; DSISNTWGEG; SISNTWGEGL;<br>ISNTWGEGLE; SNTWGEGLEI; NTWGEGLEIG; TWGEGLEIGG;<br>WGEGLEIGGD; GEGLEIGGDS; EGLEIGGDSN; GLEIGGDSNF;<br>LEIGGDSNFF; EIGGDSNFFT; IGGDSNFFTN; GGDSNFFTNL;<br>GDSNFFTNLE; DSNFFTNLEE; SNFFTNLEEV; NFFTNLEEVR;<br>FFTNLEEVRS; FTNLEEVRSS; TNLEEVRSSI; NLEEVRSSIR;<br>LEEVRSSIRT; EEVRSSIRTK; EVRSSIRTKI; VRSSIRTKIK;<br>RSSIRTKIKV; SSIRTKIKVS; SIRTKIKVSD; IRTKIKVSDG;<br>RTKIKVSDGT; TKIKVSDGTE; KIKVSDGTEQ; IKVSDGTEQT;<br>KVSDGTEQTK; VSDGTEQTKD; SDGTEQTKDK; DGTEQTKDKV;<br>GTEQTKDKVE; TEQTKDKVEI; EQTKDKVEID; QTKDKVEIDE;<br>TKDKVEIDEI; KDKVEIDEII; DKVEIDEIIE; KVEIDEIIED;<br>VEIDEIIEDL; EIDEIIEDLQ; IDEIIEDLQK; DEIIEDLQKL;<br>EIIEDLQKLK; IIEDLQKLKE; IEDLQKLKEF; EDLQKLKEFL;<br>DLQKLKEFLE; LQKLKEFLEK; QKLKEFLEKL; KLKEFLEKLK;<br>LKEFLEKLKK; KEFLEKLKKY; EFLEKLKKYL; FLEKLKKYLK;<br>LEKLKKYLKD; EKLKKYLKDT; KLKKYLKDTN; LKKYLKDTNN;<br>KKYLKDTNNL; KYLKDTNNLS; YLKDTNNLSA; LKDTNNLSAI;<br>KDTNNLSAIE; DTNNLSAIEE; TNNLSAIEES; NNLSAIEESV;<br>NLSAIEESVK; LSAIEESVKG; SAIEESVKGL; AIEESVKGLS;<br><br>11 mers: |

MCAFLLLNLVN; CAFLLLNLVNC; AFLLLNLVNCK; FLLLNLVNCKF;
LLLNLVNCKFD; LLNLVNCKFDS; LNLVNCKFDSL; NLVNCKFDSLN;
LVNCKFDSLNL; VNCKFDSLNLS; NCKFDSLNLST; CKFDSLNLSTK;
KFDSLNLSTKS; FDSLNLSTKSV; DSLNLSTKSVD; SLNLSTKSVDD;
LNLSTKSVDDK; NLSTKSVDDKN; LSTKSVDDKNN; STKSVDDKNNS;
TKSVDDKNNSI; KSVDDKNNSIA; SVDDKNNSIAK; VDDKNNSIAKL;
DDKNNSIAKLL; DKNNSIAKLLQ; KNNSIAKLLQH; NNSIAKLLQHL;
NSIAKLLQHLS; SIAKLLQHLSK; IAKLLQHLSKS; AKLLQHLSKSE;
KLLQHLSKSED; LLQHLSKSEDQ; LQHLSKSEDQA; QHLSKSEDQAN;
HLSKSEDQANK; LSKSEDQANKT; SKSEDQANKTS; KSEDQANKTST;
SEDQANKTSTS; EDQANKTSTSE; DQANKTSTSED; QANKTSTSEDQ;
ANKTSTSEDQK; NKTSTSEDQKE; KTSTSEDQKEL; TSTSEDQKELE;
STSEDQKELEI; TSEDQKELEIT; SEDQKELEITE; EDQKELEITEN;
DQKELEITENK; QKELEITENKE; KELEITENKEQ; ELEITENKEQE;
LEITENKEQEH; EITENKEQEHE; ITENKEQEHEK; TENKEQEHEKL;
ENKEQEHEKLS; NKEQEHEKLSQ; KEQEHEKLSQV; EQEHEKLSQVA;
QEHEKLSQVAQ; EHEKLSQVAQH; HEKLSQVAQHA; EKLSQVAQHAP;
KLSQVAQHAPN; LSQVAQHAPNS; SQVAQHAPNSK; QVAQHAPNSKI;
VAQHAPNSKIE; AQHAPNSKIEK; QHAPNSKIEKV; HAPNSKIEKVK;
APNSKIEKVKS; PNSKIEKVKSD; NSKIEKVKSDG; SKIEKVKSDGK;
KIEKVKSDGKP; IEKVKSDGKPV; EKVKSDGKPVP; KVKSDGKPVPG;
VKSDGKPVPGD; KSDGKPVPGDK; SDGKPVPGDKI; DGKPVPGDKIL;
GKPVPGDKILS; KPVPGDKILSS; PVPGDKILSSN; VPGDKILSSNK;
PGDKILSSNKD; GDKILSSNKDI; DKILSSNKDIY; KILSSNKDIYN;
ILSSNKDIYNS; LSSNKDIYNSY; SSNKDIYNSYI; SNKDIYNSYIP;
NKDIYNSYIPE; KDIYNSYIPEV; DIYNSYIPEVK; IYNSYIPEVKE;
YNSYIPEVKEE; NSYIPEVKEEI; SYIPEVKEEIV; YIPEVKEEIVY;
IPEVKEEIVYE; PEVKEEIVYEI; EVKEEIVYEIL; VKEEIVYEILE;
KEEIVYEILEE; EEIVYEILEEV; EIVYEILEEVI; IVYEILEEVII;
VYEILEEVIIP; YEILEEVIIPE; EILEEVIIPET; ILEEVIIPETK;
LEEVIIPETKI; EEVIIPETKIP; EVIIPETKIPE; VIIPETKIPEI;
IIPETKIPEIT; IPETKIPEITE; PETKIPEITEE; ETKIPEITEEV;
TKIPEITEEVI; KIPEITEEVIM; IPEITEEVIMP; PEITEEVIMPI;
EITEEVIMPIP; ITEEVIMPIPQ; TEEVIMPIPQT; EEVIMPIPQTI;
EVIMPIPQTID; VIMPIPQTIDF; IMPIPQTIDFY; MPIPQTIDFYI;
PIPQTIDFYIE; IPQTIDFYIEP; PQTIDFYIEPR; QTIDFYIEPRP;
TIDFYIEPRPI; IDFYIEPRPIS; DFYIEPRPISS; FYIEPRPISSF;
YIEPRPISSFL; IEPRPISSFLT; EPRPISSFLTQ; PRPISSFLTQG;
RPISSFLTQGT; PISSFLTQGTS; ISSFLTQGTSP; SSFLTQGTSPS;
SFLTQGTSPSI; FLTQGTSPSIT; LTQGTSPSITS; TQGTSPSITST;
QGTSPSITSTI; GTSPSITSTIK; TSPSITSTIKS; SPSITSTIKSY;
PSITSTIKSYK; SITSTIKSYKE; ITSTIKSYKEL; TSTIKSYKELA;
STIKSYKELAK; TIKSYKELAKE; IKSYKELAKEK; KSYKELAKEKI;
SYKELAKEKIN; YKELAKEKINN; KELAKEKINNG; ELAKEKINNGL;
LAKEKINNGLN; AKEKINNGLNI; KEKINNGLNIV; EKINNGLNIVQ;
KINNGLNIVQK; INNGLNIVQKI; NNGLNIVQKIT; NGLNIVQKITQ;
GLNIVQKITQN; LNIVQKITQNI; NIVQKITQNID; IVQKITQNIDN;
VQKITQNIDNI; QKITQNIDNIT; KITQNIDNITE; ITQNIDNITEN;
TQNIDNITENL; QNIDNITENLN; NIDNITENLNS; IDNITENLNSK;
DNITENLNSKE; NITENLNSKET; ITENLNSKETP; TENLNSKETPK;
ENLNSKETPKE; NLNSKETPKEI; LNSKETPKEIS; NSKETPKEISG;

| | |
|---|---|
| | SKETPKEISGK; KETPKEISGKE; ETPKEISGKEV; TPKEISGKEVE;<br>PKEISGKEVEE; KEISGKEVEEK; EISGKEVEEKI; ISGKEVEEKIT;<br>SGKEVEEKITH; GKEVEEKITHP; KEVEEKITHPI; EVEEKITHPIF;<br>VEEKITHPIFD; EEKITHPIFDH; EKITHPIFDHI; KITHPIFDHIT;<br>ITHPIFDHITG; THPIFDHITGS; HPIFDHITGSG; PIFDHITGSGN;<br>IFDHITGSGNN; FDHITGSGNNP; DHITGSGNNPG; HITGSGNNPGQ;<br>ITGSGNNPGQD; TGSGNNPGQDS; GSGNNPGQDSI; SGNNPGQDSIS;<br>GNNPGQDSISN; NNPGQDSISNT; NPGQDSISNTW; PGQDSISNTWG;<br>GQDSISNTWGE; QDSISNTWGEG; DSISNTWGEGL; SISNTWGEGLE;<br>ISNTWGEGLEI; SNTWGEGLEIG; NTWGEGLEIGG; TWGEGLEIGGD;<br>WGEGLEIGGDS; GEGLEIGGDSN; EGLEIGGDSNF; GLEIGGDSNFF;<br>LEIGGDSNFFT; EIGGDSNFFTN; IGGDSNFFTNL; GGDSNFFTNLE;<br>GDSNFFTNLEE; DSNFFTNLEEV; SNFFTNLEEVR; NFFTNLEEVRS;<br>FFTNLEEVRSS; FTNLEEVRSSI; TNLEEVRSSIR; NLEEVRSSIRT;<br>LEEVRSSIRTK; EEVRSSIRTKI; EVRSSIRTKIK; VRSSIRTKIKV;<br>RSSIRTKIKVS; SSIRTKIKVSD; SIRTKIKVSDG; IRTKIKVSDGT;<br>RTKIKVSDGTE; TKIKVSDGTEQ; KIKVSDGTEQT; IKVSDGTEQTK;<br>KVSDGTEQTKD; VSDGTEQTKDK; SDGTEQTKDKV; DGTEQTKDKVE;<br>GTEQTKDKVEI; TEQTKDKVEID; EQTKDKVEIDE; QTKDKVEIDEI;<br>TKDKVEIDEII; KDKVEIDEIIE; DKVEIDEIIED; KVEIDEIIEDL;<br>VEIDEIIEDLQ; EIDEIIEDLQK; IDEIIEDLQKL; DEIIEDLQKLK;<br>EIIEDLQKLKE; IIEDLQKLKEF; IEDLQKLKEFL; EDLQKLKEFLE;<br>DLQKLKEFLEK; LQKLKEFLEKL; QKLKEFLEKLK; KLKEFLEKLKK;<br>LKEFLEKLKKY; KEFLEKLKKYL; EFLEKLKKYLK; FLEKLKKYLKD;<br>LEKLKKYLKDT; EKLKKYLKDTN; KLKKYLKDTNN; LKKYLKDTNNL;<br>KKYLKDTNNLS; KYLKDTNNLSA; YLKDTNNLSAI; LKDTNNLSAIE;<br>KDTNNLSAIEE; DTNNLSAIEES; TNNLSAIEESV; NNLSAIEESVK;<br>NLSAIEESVKG; LSAIEESVKGL; SAIEESVKGLS; |
| Seq 20 | SEQ ID NO 20456-22321/<br>8 mers:<br>MIKMSLNY; IKMSLNYT; KMSLNYTE; MSLNYTEI; SLNYTEIN;<br>LNYTEINT; NYTEINTL; YTEINTLI; TEINTLIK; EINTLIKE;<br>INTLIKEI; NTLIKEIP; TLIKEIPF; LIKEIPFT; IKEIPFTN;<br>KEIPFTNS; EIPFTNSL; IPFTNSLI; PFTNSLIT; FTNSLITK;<br>TNSLITKI; NSLITKII; SLITKIIQ; LITKIIQP; ITKIIQPD;<br>TKIIQPDY; KIIQPDYK; IIQPDYKS; IQPDYKSL; QPDYKSLV;<br>PDYKSLVL; DYKSLVLE; YKSLVLEI; KSLVLEIY; SLVLEIYN;<br>LVLEIYNK; VLEIYNKI; LEIYNKID; EIYNKIDN; IYNKIDNK;<br>YNKIDNKK; NKIDNKKF; KIDNKKFK; IDNKKFKI; DNKKFKIL;<br>NKKFKILI; KKFKILIC; KFKILICL; FKILICLN; KILICLNP;<br>ILICLNPN; LICLNPNT; ICLNPNTT; CLNPNTTR; LNPNTTRF;<br>NPNTTRFH; PNTTRFHI; NTTRFHIT; TTRFHITK; TRFHITKK;<br>RFHITKKN; FHITKKNF; HITKKNFK; ITKKNFKK; TKKNFKKN;<br>KKNFKKNA; KNFKKNAL; NFKKNALK; FKKNALKL; KKNALKLR;<br>KNALKLRF; NALKLRFS; ALKLRFSD; LKLRFSDF; KLRFSDFL;<br>LRFSDFLK; RFSDFLKS; FSDFLKSK; SDFLKSKI; DFLKSKIQ;<br>FLKSKIQN; LKSKIQNG; KSKIQNGK; SKIQNGKI; KIQNGKII;<br>IQNGKIIK; QNGKIIKA; NGKIIKAF; GKIIKAFQ; KIIKAFQM;<br>IIKAFQMK; IKAFQMKN; KAFQMKNE; AFQMKNER; FQMKNERI; |

QMKNERII; MKNERIIS; KNERIISL; NERIISLE; ERIISLEI;
RIISLEIL; IISLEILQ; ISLEILQK; SLEILQKD; LEILQKDM;
EILQKDMI; ILQKDMII; LQKDMIIL; QKDMIILF; KDMIILFI;
DMIILFIK; MIILFIKL; IILFIKLW; ILFIKLWP; LFIKLWPS;
FIKLWPSS; IKLWPSSP; KLWPSSPN; LWPSSPNI; WPSSPNII;
PSSPNIIA; SSPNIIAT; SPNIIATN; PNIIATNS; NIIATNSN;
IIATNSNF; IATNSNFK; ATNSNFKI; TNSNFKIL; NSNFKILD;
SNFKILDA; NFKILDAY; FKILDAYY; KILDAYYR; ILDAYYRR;
LDAYYRRP; DAYYRRPK; AYYRRPKI; YRRPKIK; YRRPKIKE;
RRPKIKET; RPKIKETT; PKIKETTG; KIKETTGE; IKETTGEI;
KETTGEIF; ETTGEIFL; TTGEIFLK; TGEIFLKA; GEIFLKAK;
EIFLKAKE; IFLKAKEI; FLKAKEIH; LKAKEIHE; KAKEIHES;
AKEIHESN; KEIHESNK; EIHESNKM; IHESNKMS; HESNKMSD;
ESNKMSDK; SNKMSDKK; NKMSDKKI; KMSDKKIM; MSDKKIME;
SDKKIMEL; DKKIMELK; KKIMELKE; KIMELKEE; IMELKEEY;
MELKEEYN; ELKEEYNN; LKEEYNNT; KEEYNNTS; EEYNNTSY;
EYNNTSYT; YNNTSYTS; NNTSYTSY; NTSYTSYS; TSYTSYSE;
SYTSYSEF; YTSYSEFL; TSYSEFLE; SYSEFLEN; YSEFLENY;
SEFLENYY; EFLENYYE; FLENYYES; LENYYESL; ENYYESLN;
NYYESLND; YYESLNDQ; YESLNDQI; ESLNDQIK; SLNDQIKK;
LNDQIKKT; NDQIKKTN; DQIKKTNI; QIKKTNIK; IKKTNIKE;
KKTNIKEL; KTNIKELL; TNIKELLI; NIKELLIE; IKELLIEK;
KELLIEKY; ELLIEKYK; LLIEKYKK; LIEKYKKE; IEKYKKEL;
EKYKKELI; KYKKELIV; YKKELIVL; KKELIVLE; KELIVLEK;
ELIVLEKR; LIVLEKRI; IVLEKRID; VLEKRIDS; LEKRIDSL;
EKRIDSLK; KRIDSLKQ; RIDSLKQQ; IDSLKQQI; DSLKQQIK;
SLKQQIKL; LKQQIKLL; KQQIKLLE; QQIKLLEN; QIKLLENI;
IKLLENIE; KLLENIEN; LLENIENE; LENIENEK; ENIENEKE;
NIENEKEK; IENEKEKG; ENEKEKGE; NEKEKGEL; EKEKGELI;
KEKGELIL; EKGELILL; KGELILLN; GELILLNI; ELILLNIN;
LILLNINK; ILLNINKI; LLNINKIQ; LNINKIQK; NINKIQKG;
INKIQKGI; NKIQKGIK; KIQKGIKE; IQKGIKEI; QKGIKEIN;
KGIKEINL; GIKEINLL; IKEINLLN; KEINLLNY; EINLLNYK;
INLLNYKE; NLLNYKEE; LLNYKEEK; LNYKEEKI; NYKEEKIK;
YKEEKIKI; KEEKIKIS; EEKIKISL; EKIKISLN; KIKISLNQ;
IKISLNQS; KISLNQSL; ISLNQSLS; SLNQSLSP; LNQSLSPK;
NQSLSPKE; QSLSPKEN; SLSPKENA; LSPKENAL; SPKENALQ;
PKENALQY; KENALQYF; ENALQYFK; NALQYFKA; ALQYFKAY;
LQYFKAYK; QYFKAYKK; YFKAYKKG; FKAYKKGK; KAYKKGKN;
AYKKGKNS; YKKGKNSF; KKGKNSFK; KGKNSFKT; GKNSFKTI;
KNSFKTIQ; NSFKTIQN; SFKTIQNQ; FKTIQNQL; KTIQNQLK;
TIQNQLKD; IQNQLKDN; QNQLKDNL; NQLKDNLD; QLKDNLDK;
LKDNLDKF; KDNLDKFN; DNLDKFNL; NLDKFNLI; LDKFNLIQ;
DKFNLIQS; KFNLIQSK; FNLIQSKI; NLIQSKIT; LIQSKITM;
IQSKITML; QSKITMLK; SKITMLKV; KITMLKVE; ITMLKVEN;
TMLKVENL; MLKVENLI; LKVENLIP; KVENLIPE; VENLIPEE;
ENLIPEEE; NLIPEEEY; LIPEEEYN; IPEEEYNQ; PEEEYNQE;
EEEYNQEK; EEYNQEKT; EYNQEKTA; YNQEKTAI; NQEKTAIK;
QEKTAIKE; EKTAIKEK; KTAIKEKE; TAIKEKEK; AIKEKEKT;
IKEKEKTP; KEKEKTPK; EKEKTPKI; KEKTPKIG; EKTPKIGL;
KTPKIGLH; TPKIGLHF; PKIGLHFT; KIGLHFTY; IGLHFTYC;

GLHFTYCG; LHFTYCGF; HFTYCGFE; FTYCGFEI; TYCGFEIL;
YCGFEILI; CGFEILIG; GFEILIGR; FEILIGRN; EILIGRNA;
ILIGRNAK; LIGRNAKE; IGRNAKEN; GRNAKEND; RNAKENDK;
NAKENDKL; AKENDKLL; KENDKLLR; ENDKLLRH; NDKLLRHC;
DKLLRHCV; KLLRHCVK; LLRHCVKG; LRHCVKGN; RHCVKGND;
HCVKGNDY; CVKGNDYW; VKGNDYWL; KGNDYWLH; GNDYWLHT;
NDYWLHTR; DYWLHTRD; YWLHTRDY; WLHTRDYP; LHTRDYPG;
HTRDYPGA; TRDYPGAY; RDYPGAYV; DYPGAYVF; YPGAYVFI;
PGAYVFIK; GAYVFIKN; AYVFIKNQ; YVFIKNQK; VFIKNQKN;
FIKNQKNK; IKNQKNKT; KNQKNKTP; NQKNKTPS; QKNKTPSL;
KNKTPSLD; NKTPSLDV; KTPSLDVL; TPSLDVLL; PSLDVLLG;
SLDVLLGA; LDVLLGAG; DVLLGAGN; VLLGAGNL; LLGAGNLC;
LGAGNLCV; GAGNLCVF; AGNLCVFY; GNLCVFYT; NLCVFYTK;
LCVFYTKL; CVFYTKLA; VFYTKLAK; FYTKLAKK; YTKLAKKS;
TKLAKKSG; KLAKKSGK; LAKKSGKA; AKKSGKAD; KKSGKADL;
KSGKADLY; SGKADLYY; GKADLYYT; KADLYYTQ; ADLYYTQV;
DLYYTQVK; LYYTQVKN; YYTQVKNL; YTQVKNLR; TQVKNLRR;
QVKNLRRV; VKNLRRVK; KNLRRVKN; NLRRVKNK; LRRVKNKK;
RRVKNKKL; RVKNKKLG; VKNKKLGL; KNKKLGLV; NKKLGLVI;
KKLGLVIP; KLGLVIPK; LGLVIPKA; GLVIPKAE; LVIPKAEK;
VIPKAEKN; IPKAEKNL; PKAEKNLH; KAEKNLHI; AEKNLHIK;
EKNLHIKL; KNLHIKLD; NLHIKLDE; LHIKLDEN; HIKLDENL;
IKLDENLI; KLDENLIK; LDENLIKK; DENLIKKI; ENLIKKIK;
NLIKKIKN; LIKKIKNQ; IKKIKNQT;

9 mers:
MIKMSLNYT; IKMSLNYTE; KMSLNYTEI; MSLNYTEIN; SLNYTEINT;
LNYTEINTL; NYTEINTLI; YTEINTLIK; TEINTLIKE; EINTLIKEI;
INTLIKEIP; NTLIKEIPF; TLIKEIPFT; LIKEIPFTN; IKEIPFTNS;
KEIPFTNSL; EIPFTNSLI; IPFTNSLIT; PFTNSLITK; FTNSLITKI;
TNSLITKII; NSLITKIIQ; SLITKIIQP; LITKIIQPD; ITKIIQPDY;
TKIIQPDYK; KIIQPDYKS; IIQPDYKSL; IQPDYKSLV; QPDYKSLVL;
PDYKSLVLE; DYKSLVLEI; YKSLVLEIY; KSLVLEIYN; SLVLEIYNK;
LVLEIYNKI; VLEIYNKID; LEIYNKIDN; EIYNKIDNK; IYNKIDNKK;
YNKIDNKKF; NKIDNKKFK; KIDNKKFKI; IDNKKFKIL; DNKKFKILI;
NKKFKILIC; KKFKILICL; KFKILICLN; FKILICLNP; KILICLNPN;
ILICLNPNT; LICLNPNTT; ICLNPNTTR; CLNPNTTRF; LNPNTTRFH;
NPNTTRFHI; PNTTRFHIT; NTTRFHITK; TTRFHITKK; TRFHITKKN;
RFHITKKNF; FHITKKNFK; HITKKNFKK; ITKKNFKKN; TKKNFKKNA;
KKNFKKNAL; KNFKKNALK; NFKKNALKL; FKKNALKLR; KKNALKLRF;
KNALKLRFS; NALKLRFSD; ALKLRFSDF; LKLRFSDFL; KLRFSDFLK;
LRFSDFLKS; RFSDFLKSK; FSDFLKSKI; SDFLKSKIQ; DFLKSKIQN;
FLKSKIQNG; LKSKIQNGK; KSKIQNGKI; SKIQNGKII; KIQNGKIIK;
IQNGKIIKA; QNGKIIKAF; NGKIIKAFQ; GKIIKAFQM; KIIKAFQMK;
IIKAFQMKN; IKAFQMKNE; KAFQMKNER; AFQMKNERI; FQMKNERII;
QMKNERIIS; MKNERIISL; KNERIISLE; NERIISLEI; ERIISLEIL;
RIISLEILQ; IISLEILQK; ISLEILQKD; SLEILQKDM; LEILQKDMI;
EILQKDMII; ILQKDMIIL; LQKDMIILF; QKDMIILFI; KDMIILFIK;
DMIILFIKL; MIILFIKLW; IILFIKLWP; ILFIKLWPS; LFIKLWPSS;
FIKLWPSSP; IKLWPSSPN; KLWPSSPNI; LWPSSPNII; WPSSPNIIA;
PSSPNIIAT; SSPNIIATN; SPNIIATNS; PNIIATNSN; NIIATNSNF;

| | IIATNSNFK; | IATNSNFKI; | ATNSNFKIL; | TNSNFKILD; | NSNFKILDA; |
|---|---|---|---|---|---|
| | SNFKILDAY; | NFKILDAYY; | FKILDAYYR; | KILDAYYRR; | ILDAYYRRP; |
| | LDAYYRRPK; | DAYYRRPKI; | AYYRRPKIK; | YRRPKIKE; | YRRPKIKET; |
| | RRPKIKETT; | RPKIKETTG; | PKIKETTGE; | KIKETTGEI; | IKETTGEIF; |
| | KETTGEIFL; | ETTGEIFLK; | TTGEIFLKA; | TGEIFLKAK; | GEIFLKAKE; |
| | EIFLKAKEI; | IFLKAKEIH; | FLKAKEIHE; | LKAKEIHES; | KAKEIHESN; |
| | AKEIHESNK; | KEIHESNKM; | EIHESNKMS; | IHESNKMSD; | HESNKMSDK; |
| | ESNKMSDKK; | SNKMSDKKI; | NKMSDKKIM; | KMSDKKIME; | MSDKKIMEL; |
| | SDKKIMELK; | DKKIMELKE; | KKIMELKEE; | KIMELKEEY; | IMELKEEYN; |
| | MELKEEYNN; | ELKEEYNNT; | LKEEYNNTS; | KEEYNNTSY; | EEYNNTSYT; |
| | EYNNTSYTS; | YNNTSYTSY; | NNTSYTSYS; | NTSYTSYSE; | TSYTSYSEF; |
| | SYTSYSEFL; | YTSYSEFLE; | TSYSEFLEN; | SYSEFLENY; | YSEFLENYY; |
| | SEFLENYYE; | EFLENYYES; | FLENYYESL; | LENYYESLN; | ENYYESLND; |
| | NYYESLNDQ; | YYESLNDQI; | YESLNDQIK; | ESLNDQIKK; | SLNDQIKKT; |
| | LNDQIKKTN; | NDQIKKTNI; | DQIKKTNIK; | QIKKTNIKE; | IKKTNIKEL; |
| | KKTNIKELL; | KTNIKELLI; | TNIKELLIE; | NIKELLIEK; | IKELLIEKY; |
| | KELLIEKYK; | ELLIEKYKK; | LLIEKYKKE; | LIEKYKKEL; | IEKYKKELI; |
| | EKYKKELIV; | KYKKELIVL; | YKKELIVLE; | KKELIVLEK; | KELIVLEKR; |
| | ELIVLEKRI; | LIVLEKRID; | IVLEKRIDS; | VLEKRIDSL; | LEKRIDSLK; |
| | EKRIDSLKQ; | KRIDSLKQQ; | RIDSLKQQI; | IDSLKQQIK; | DSLKQQIKL; |
| | SLKQQIKLL; | LKQQIKLLE; | KQQIKLLEN; | QQIKLLENI; | QIKLLENIE; |
| | IKLLENIEN; | KLLENIENE; | LLENIENEK; | LENIENEKE; | ENIENEKEK; |
| | NIENEKEKG; | IENEKEKGE; | ENEKEKGEL; | NEKEKGELI; | EKEKGELIL; |
| | KEKGELILL; | EKGELILLN; | KGELILLNI; | GELILLNIN; | ELILLNINK; |
| | LILLNINKI; | ILLNINKIQ; | LLNINKIQK; | LNINKIQKG; | NINKIQKGI; |
| | INKIQKGIK; | NKIQKGIKE; | KIQKGIKEI; | IQKGIKEIN; | QKGIKEINL; |
| | KGIKEINLL; | GIKEINLLN; | IKEINLLNY; | KEINLLNYK; | EINLLNYKE; |
| | INLLNYKEE; | NLLNYKEEK; | LLNYKEEKI; | LNYKEEKIK; | NYKEEKIKI; |
| | YKEEKIKIS; | KEEKIKISL; | EEKIKISLN; | EKIKISLNQ; | KIKISLNQS; |
| | IKISLNQSL; | KISLNQSLS; | ISLNQSLSP; | SLNQSLSPK; | LNQSLSPKE; |
| | NQSLSPKEN; | QSLSPKENA; | SLSPKENAL; | LSPKENALQ; | SPKENALQY; |
| | PKENALQYF; | KENALQYFK; | ENALQYFKA; | NALQYFKAY; | ALQYFKAYK; |
| | LQYFKAYKK; | QYFKAYKKG; | YFKAYKKGK; | FKAYKKGKN; | KAYKKGKNS; |
| | AYKKGKNSF; | YKKGKNSFK; | KKGKNSFKT; | KGKNSFKTI; | GKNSFKTIQ; |
| | KNSFKTIQN; | NSFKTIQNQ; | SFKTIQNQL; | FKTIQNQLK; | KTIQNQLKD; |
| | TIQNQLKDN; | IQNQLKDNL; | QNQLKDNLD; | NQLKDNLDK; | QLKDNLDKF; |
| | LKDNLDKFN; | KDNLDKFNL; | DNLDKFNLI; | NLDKFNLIQ; | LDKFNLIQS; |
| | DKFNLIQSK; | KFNLIQSKI; | FNLIQSKIT; | NLIQSKITM; | LIQSKITML; |
| | IQSKITMLK; | QSKITMLKV; | SKITMLKVE; | KITMLKVEN; | ITMLKVENL; |
| | TMLKVENLI; | MLKVENLIP; | LKVENLIPE; | KVENLIPEE; | VENLIPEEE; |
| | ENLIPEEEY; | NLIPEEEYN; | LIPEEEYNQ; | IPEEEYNQE; | PEEEYNQEK; |
| | EEEYNQEKT; | EEYNQEKTA; | EYNQEKTAI; | YNQEKTAIK; | NQEKTAIKE; |
| | QEKTAIKEK; | EKTAIKEKE; | KTAIKEKEK; | TAIKEKEKT; | AIKEKEKTP; |
| | IKEKEKTPK; | KEKEKTPKI; | EKEKTPKIG; | KEKTPKIGL; | EKTPKIGLH; |
| | KTPKIGLHF; | TPKIGLHFT; | PKIGLHFTY; | KIGLHFTYC; | IGLHFTYCG; |
| | GLHFTYCGF; | LHFTYCGFE; | HFTYCGFEI; | FTYCGFEIL; | TYCGFEILI; |
| | YCGFEILIG; | CGFEILIGR; | GFEILIGRN; | FEILIGRNA; | EILIGRNAK; |
| | ILIGRNAKE; | LIGRNAKEN; | IGRNAKEND; | GRNAKENDK; | RNAKENDKL; |
| | NAKENDKLL; | AKENDKLLR; | KENDKLLRH; | ENDKLLRHC; | NDKLLRHCV; |
| | DKLLRHCVK; | KLLRHCVKG; | LLRHCVKGN; | LRHCVKGND; | RHCVKGNDY; |
| | HCVKGNDYW; | CVKGNDYWL; | VKGNDYWLH; | KGNDYWLHT; | GNDYWLHTR; |

NDYWLHTRD; DYWLHTRDY; YWLHTRDYP; WLHTRDYPG; LHTRDYPGA;
HTRDYPGAY; TRDYPGAYV; RDYPGAYVF; DYPGAYVFI; YPGAYVFIK;
PGAYVFIKN; GAYVFIKNQ; AYVFIKNQK; YVFIKNQKN; VFIKNQKNK;
FIKNQKNKT; IKNQKNKTP; KNQKNKTPS; NQKNKTPSL; QKNKTPSLD;
KNKTPSLDV; NKTPSLDVL; KTPSLDVLL; TPSLDVLLG; PSLDVLLGA;
SLDVLLGAG; LDVLLGAGN; DVLLGAGNL; VLLGAGNLC; LLGAGNLCV;
LGAGNLCVF; GAGNLCVFY; AGNLCVFYT; GNLCVFYTK; NLCVFYTKL;
LCVFYTKLA; CVFYTKLAK; VFYTKLAKK; FYTKLAKKS; YTKLAKKSG;
TKLAKKSGK; KLAKKSGKA; LAKKSGKAD; AKKSGKADL; KKSGKADLY;
KSGKADLYY; SGKADLYYT; GKADLYYTQ; KADLYYTQV; ADLYYTQVK;
DLYYTQVKN; LYYTQVKNL; YYTQVKNLR; YTQVKNLRR; TQVKNLRRV;
QVKNLRRVK; VKNLRRVKN; KNLRRVKNK; NLRRVKNKK; LRRVKNKKL;
RRVKNKKLG; RVKNKKLGL; VKNKKLGLV; KNKKLGLVI; NKKLGLVIP;
KKLGLVIPK; KLGLVIPKA; LGLVIPKAE; GLVIPKAEK; LVIPKAEKN;
VIPKAEKNL; IPKAEKNLH; PKAEKNLHI; KAEKNLHIK; AEKNLHIKL;
EKNLHIKLD; KNLHIKLDE; NLHIKLDEN; LHIKLDENL; HIKLDENLI;
IKLDENLIK; KLDENLIKK; LDENLIKKI; DENLIKKIK; ENLIKKIKN;
NLIKKIKNQ; LIKKIKNQT;

10 mers:
MIKMSLNYTE; IKMSLNYTEI; KMSLNYTEIN; MSLNYTEINT;
SLNYTEINTL; LNYTEINTLI; NYTEINTLIK; YTEINTLIKE;
TEINTLIKEI; EINTLIKEIP; INTLIKEIPF; NTLIKEIPFT;
TLIKEIPFTN; LIKEIPFTNS; IKEIPFTNSL; KEIPFTNSLI;
EIPFTNSLIT; IPFTNSLITK; PFTNSLITKI; FTNSLITKII;
TNSLITKIIQ; NSLITKIIQP; SLITKIIQPD; LITKIIQPDY;
ITKIIQPDYK; TKIIQPDYKS; KIIQPDYKSL; IIQPDYKSLV;
IQPDYKSLVL; QPDYKSLVLE; PDYKSLVLEI; DYKSLVLEIY;
YKSLVLEIYN; KSLVLEIYNK; SLVLEIYNKI; LVLEIYNKID;
VLEIYNKIDN; LEIYNKIDNK; EIYNKIDNKK; IYNKIDNKKF;
YNKIDNKKFK; NKIDNKKFKI; KIDNKKFKIL; IDNKKFKILI;
DNKKFKILIC; NKKFKILICL; KKFKILICLN; KFKILICLNP;
FKILICLNPN; KILICLNPNT; ILICLNPNTT; LICLNPNTTR;
ICLNPNTTRF; CLNPNTTRFH; LNPNTTRFHI; NPNTTRFHIT;
PNTTRFHITK; NTTRFHITKK; TTRFHITKKN; TRFHITKKNF;
RFHITKKNFK; FHITKKNFKK; HITKKNFKKN; ITKKNFKKNA;
TKKNFKKNAL; KKNFKKNALK; KNFKKNALKL; NFKKNALKLR;
FKKNALKLRF; KKNALKLRFS; KNALKLRFSD; NALKLRFSDF;
ALKLRFSDFL; LKLRFSDFLK; KLRFSDFLKS; LRFSDFLKSK;
RFSDFLKSKI; FSDFLKSKIQ; SDFLKSKIQN; DFLKSKIQNG;
FLKSKIQNGK; LKSKIQNGKI; KSKIQNGKII; SKIQNGKIIK;
KIQNGKIIKA; IQNGKIIKAF; QNGKIIKAFQ; NGKIIKAFQM;
GKIIKAFQMK; KIIKAFQMKN; IIKAFQMKNE; IKAFQMKNER;
KAFQMKNERI; AFQMKNERII; FQMKNERIIS; QMKNERIISL;
MKNERIISLE; KNERIISLEI; NERIISLEIL; ERIISLEILQ;
RIISLEILQK; IISLEILQKD; ISLEILQKDM; SLEILQKDMI;
LEILQKDMII; EILQKDMIIL; ILQKDMIILF; LQKDMIILFI;
QKDMIILFIK; KDMIILFIKL; DMIILFIKLW; MIILFIKLWP;
IILFIKLWPS; ILFIKLWPSS; LFIKLWPSSP; FIKLWPSSPN;
IKLWPSSPNI; KLWPSSPNII; LWPSSPNIIA; WPSSPNIIAT;
PSSPNIIATN; SSPNIIATNS; SPNIIATNSN; PNIIATNSNF;

| | | | |
|---|---|---|---|
| NIIATNSNFK; | IIATNSNFKI; | IATNSNFKIL; | ATNSNFKILD; |
| TNSNFKILDA; | NSNFKILDAY; | SNFKILDAYY; | NFKILDAYYR; |
| FKILDAYYRR; | KILDAYYRRP; | ILDAYYRRPK; | LDAYYRRPKI; |
| DAYYRRPKIK; | AYYRRPKIKE; | YYRRPKIKET; | YRRPKIKETT; |
| RRPKIKETTG; | RPKIKETTGE; | PKIKETTGEI; | KIKETTGEIF; |
| IKETTGEIFL; | KETTGEIFLK; | ETTGEIFLKA; | TTGEIFLKAK; |
| TGEIFLKAKE; | GEIFLKAKEI; | EIFLKAKEIH; | IFLKAKEIHE; |
| FLKAKEIHES; | LKAKEIHESN; | KAKEIHESNK; | AKEIHESNKM; |
| KEIHESNKMS; | EIHESNKMSD; | IHESNKMSDK; | HESNKMSDKK; |
| ESNKMSDKKI; | SNKMSDKKIM; | NKMSDKKIME; | KMSDKKIMEL; |
| MSDKKIMELK; | SDKKIMELKE; | DKKIMELKEE; | KKIMELKEEY; |
| KIMELKEEYN; | IMELKEEYNN; | MELKEEYNNT; | ELKEEYNNTS; |
| LKEEYNNTSY; | KEEYNNTSYT; | EEYNNTSYTS; | EYNNTSYTSY; |
| YNNTSYTSYS; | NNTSYTSYSE; | NTSYTSYSEF; | TSYTSYSEFL; |
| SYTSYSEFLE; | YTSYSEFLEN; | TSYSEFLENY; | SYSEFLENYY; |
| YSEFLENYYE; | SEFLENYYES; | EFLENYYESL; | FLENYYESLN; |
| LENYYESLND; | ENYYESLNDQ; | NYYESLNDQI; | YYESLNDQIK; |
| YESLNDQIKK; | ESLNDQIKKT; | SLNDQIKKTN; | LNDQIKKTNI; |
| NDQIKKTNIK; | DQIKKTNIKE; | QIKKTNIKEL; | IKKTNIKELL; |
| KKTNIKELLI; | KTNIKELLIE; | TNIKELLIEK; | NIKELLIEKY; |
| IKELLIEKYK; | KELLIEKYKK; | ELLIEKYKKE; | LLIEKYKKEL; |
| LIEKYKKELI; | IEKYKKELIV; | EKYKKELIVL; | KYKKELIVLE; |
| YKKELIVLEK; | KKELIVLEKR; | KELIVLEKRI; | ELIVLEKRID; |
| LIVLEKRIDS; | IVLEKRIDSL; | VLEKRIDSLK; | LEKRIDSLKQ; |
| EKRIDSLKQQ; | KRIDSLKQQI; | RIDSLKQQIK; | IDSLKQQIKL; |
| DSLKQQIKLL; | SLKQQIKLLE; | LKQQIKLLEN; | KQQIKLLENI; |
| QQIKLLENIE; | QIKLLENIEN; | IKLLENIENE; | KLLENIENEK; |
| LLENIENEKE; | LENIENEKEK; | ENIENEKEKG; | NIENEKEKGE; |
| IENEKEKGEL; | ENEKEKGELI; | NEKEKGELIL; | EKEKGELILL; |
| KEKGELILLN; | EKGELILLNI; | KGELILLNIN; | GELILLNINK; |
| ELILLNINKI; | LILLNINKIQ; | ILLNINKIQK; | LLNINKIQKG; |
| LNINKIQKGI; | NINKIQKGIK; | INKIQKGIKE; | NKIQKGIKEI; |
| KIQKGIKEIN; | IQKGIKEINL; | QKGIKEINLL; | KGIKEINLLN; |
| GIKEINLLNY; | IKEINLLNYK; | KEINLLNYKE; | EINLLNYKEE; |
| INLLNYKEEK; | NLLNYKEEKI; | LLNYKEEKIK; | LNYKEEKIKI; |
| NYKEEKIKIS; | YKEEKIKISL; | KEEKIKISLN; | EEKIKISLNQ; |
| EKIKISLNQS; | KIKISLNQSL; | IKISLNQSLS; | KISLNQSLSP; |
| ISLNQSLSPK; | SLNQSLSPKE; | LNQSLSPKEN; | NQSLSPKENA; |
| QSLSPKENAL; | SLSPKENALQ; | LSPKENALQY; | SPKENALQYF; |
| PKENALQYFK; | KENALQYFKA; | ENALQYFKAY; | NALQYFKAYK; |
| ALQYFKAYKK; | LQYFKAYKKG; | QYFKAYKKGK; | YFKAYKKGKN; |
| FKAYKKGKNS; | KAYKKGKNSF; | AYKKGKNSFK; | YKKGKNSFKT; |
| KKGKNSFKTI; | KGKNSFKTIQ; | GKNSFKTIQN; | KNSFKTIQNQ; |
| NSFKTIQNQL; | SFKTIQNQLK; | FKTIQNQLKD; | KTIQNQLKDN; |
| TIQNQLKDNL; | IQNQLKDNLD; | QNQLKDNLDK; | NQLKDNLDKF; |
| QLKDNLDKFN; | LKDNLDKFNL; | KDNLDKFNLI; | DNLDKFNLIQ; |
| NLDKFNLIQS; | LDKFNLIQSK; | DKFNLIQSKI; | KFNLIQSKIT; |
| FNLIQSKITM; | NLIQSKITML; | LIQSKITMLK; | IQSKITMLKV; |
| QSKITMLKVE; | SKITMLKVEN; | KITMLKVENL; | ITMLKVENLI; |
| TMLKVENLIP; | MLKVENLIPE; | LKVENLIPEE; | KVENLIPEEE; |
| VENLIPEEEY; | ENLIPEEEYN; | NLIPEEEYNQ; | LIPEEEYNQE; |

```
IPEEEYNQEK; PEEEYNQEKT; EEEYNQEKTA; EEYNQEKTAI;
EYNQEKTAIK; YNQEKTAIKE; NQEKTAIKEK; QEKTAIKEKE;
EKTAIKEKEK; KTAIKEKEKT; TAIKEKEKTP; AIKEKEKTPK;
IKEKEKTPKI; KEKEKTPKIG; EKEKTPKIGL; KEKTPKIGLH;
EKTPKIGLHF; KTPKIGLHFT; TPKIGLHFTY; PKIGLHFTYC;
KIGLHFTYCG; IGLHFTYCGF; GLHFTYCGFE; LHFTYCGFEI;
HFTYCGFEIL; FTYCGFEILI; TYCGFEILIG; YCGFEILIGR;
CGFEILIGRN; GFEILIGRNA; FEILIGRNAK; EILIGRNAKE;
ILIGRNAKEN; LIGRNAKEND; IGRNAKENDK; GRNAKENDKL;
RNAKENDKLL; NAKENDKLLR; AKENDKLLRH; KENDKLLRHC;
ENDKLLRHCV; NDKLLRHCVK; DKLLRHCVKG; KLLRHCVKGN;
LLRHCVKGND; LRHCVKGNDY; RHCVKGNDYW; HCVKGNDYWL;
CVKGNDYWLH; VKGNDYWLHT; KGNDYWLHTR; GNDYWLHTRD;
NDYWLHTRDY; DYWLHTRDYP; YWLHTRDYPG; WLHTRDYPGA;
LHTRDYPGAY; HTRDYPGAYV; TRDYPGAYVF; RDYPGAYVFI;
DYPGAYVFIK; YPGAYVFIKN; PGAYVFIKNQ; GAYVFIKNQK;
AYVFIKNQKN; YVFIKNQKNK; VFIKNQKNKT; FIKNQKNKTP;
IKNQKNKTPS; KNQKNKTPSL; NQKNKTPSLD; QKNKTPSLDV;
KNKTPSLDVL; NKTPSLDVLL; KTPSLDVLLG; TPSLDVLLGA;
PSLDVLLGAG; SLDVLLGAGN; LDVLLGAGNL; DVLLGAGNLC;
VLLGAGNLCV; LLGAGNLCVF; LGAGNLCVFY; GAGNLCVFYT;
AGNLCVFYTK; GNLCVFYTKL; NLCVFYTKLA; LCVFYTKLAK;
CVFYTKLAKK; VFYTKLAKKS; FYTKLAKKSG; YTKLAKKSGK;
TKLAKKSGKA; KLAKKSGKAD; LAKKSGKADL; AKKSGKADLY;
KKSGKADLYY; KSGKADLYYT; SGKADLYYTQ; GKADLYYTQV;
KADLYYTQVK; ADLYYTQVKN; DLYYTQVKNL; LYYTQVKNLR;
YYTQVKNLRR; YTQVKNLRRV; TQVKNLRRVK; QVKNLRRVKN;
VKNLRRVKNK; KNLRRVKNKK; NLRRVKNKKL; LRRVKNKKLG;
RRVKNKKLGL; RVKNKKLGLV; VKNKKLGLVI; KNKKLGLVIP;
NKKLGLVIPK; KKLGLVIPKA; KLGLVIPKAE; LGLVIPKAEK;
GLVIPKAEKN; LVIPKAEKNL; VIPKAEKNLH; IPKAEKNLHI;
PKAEKNLHIK; KAEKNLHIKL; AEKNLHIKLD; EKNLHIKLDE;
KNLHIKLDEN; NLHIKLDENL; LHIKLDENLI; HIKLDENLIK;
IKLDENLIKK; KLDENLIKKI; LDENLIKKIK; DENLIKKIKN;
ENLIKKIKNQ; NLIKKIKNQT;

11 mers:
MIKMSLNYTEI; IKMSLNYTEIN; KMSLNYTEINT; MSLNYTEINTL;
SLNYTEINTLI; LNYTEINTLIK; NYTEINTLIKE; YTEINTLIKEI;
TEINTLIKEIP; EINTLIKEIPF; INTLIKEIPFT; NTLIKEIPFTN;
TLIKEIPFTNS; LIKEIPFTNSL; IKEIPFTNSLI; KEIPFTNSLIT;
EIPFTNSLITK; IPFTNSLITKI; PFTNSLITKII; FTNSLITKIIQ;
TNSLITKIIQP; NSLITKIIQPD; SLITKIIQPDY; LITKIIQPDYK;
ITKIIQPDYKS; TKIIQPDYKSL; KIIQPDYKSLV; IIQPDYKSLVL;
IQPDYKSLVLE; QPDYKSLVLEI; PDYKSLVLEIY; DYKSLVLEIYN;
YKSLVLEIYNK; KSLVLEIYNKI; SLVLEIYNKID; LVLEIYNKIDN;
VLEIYNKIDNK; LEIYNKIDNKK; EIYNKIDNKKF; IYNKIDNKKFK;
YNKIDNKKFKI; NKIDNKKFKIL; KIDNKKFKILI; IDNKKFKILIC;
DNKKFKILICL; NKKFKILICLN; KKFKILICLNP; KFKILICLNPN;
FKILICLNPNT; KILICLNPNTT; ILICLNPNTTR; LICLNPNTTRF;
ICLNPNTTRFH; CLNPNTTRFHI; LNPNTTRFHIT; NPNTTRFHITK;
```

| | | | |
|---|---|---|---|
| PNTTRFHITKK; | NTTRFHITKKN; | TTRFHITKKNF; | TRFHITKKNFK; |
| RFHITKKNFKK; | FHITKKNFKKN; | HITKKNFKKNA; | ITKKNFKKNAL; |
| TKKNFKKNALK; | KKNFKKNALKL; | KNFKKNALKLR; | NFKKNALKLRF; |
| FKKNALKLRFS; | KKNALKLRFSD; | KNALKLRFSDF; | NALKLRFSDFL; |
| ALKLRFSDFLK; | LKLRFSDFLKS; | KLRFSDFLKSK; | LRFSDFLKSKI; |
| RFSDFLKSKIQ; | FSDFLKSKIQN; | SDFLKSKIQNG; | DFLKSKIQNGK; |
| FLKSKIQNGKI; | LKSKIQNGKII; | KSKIQNGKIIK; | SKIQNGKIIKA; |
| KIQNGKIIKAF; | IQNGKIIKAFQ; | QNGKIIKAFQM; | NGKIIKAFQMK; |
| GKIIKAFQMKN; | KIIKAFQMKNE; | IIKAFQMKNER; | IKAFQMKNERI; |
| KAFQMKNERII; | AFQMKNERIIS; | FQMKNERIISL; | QMKNERIISLE; |
| MKNERIISLEI; | KNERIISLEIL; | NERIISLEILQ; | ERIISLEILQK; |
| RIISLEILQKD; | IISLEILQKDM; | ISLEILQKDMI; | SLEILQKDMII; |
| LEILQKDMIIL; | EILQKDMIILF; | ILQKDMIILFI; | LQKDMIILFIK; |
| QKDMIILFIKL; | KDMIILFIKLW; | DMIILFIKLWP; | MIILFIKLWPS; |
| IILFIKLWPSS; | ILFIKLWPSSP; | LFIKLWPSSPN; | FIKLWPSSPNI; |
| IKLWPSSPNII; | KLWPSSPNIIA; | LWPSSPNIIAT; | WPSSPNIIATN; |
| PSSPNIIATNS; | SSPNIIATNSN; | SPNIIATNSNF; | PNIIATNSNFK; |
| NIIATNSNFKI; | IIATNSNFKIL; | IATNSNFKILD; | ATNSNFKILDA; |
| TNSNFKILDAY; | NSNFKILDAYY; | SNFKILDAYYR; | NFKILDAYYRR; |
| FKILDAYYRRP; | KILDAYYRRPK; | ILDAYYRRPKI; | LDAYYRRPKIK; |
| DAYYRRPKIKE; | AYYRRPKIKET; | YYRRPKIKETT; | YRRPKIKETTG; |
| RRPKIKETTGE; | RPKIKETTGEI; | PKIKETTGEIF; | KIKETTGEIFL; |
| IKETTGEIFLK; | KETTGEIFLKA; | ETTGEIFLKAK; | TTGEIFLKAKE; |
| TGEIFLKAKEI; | GEIFLKAKEIH; | EIFLKAKEIHE; | IFLKAKEIHES; |
| FLKAKEIHESN; | LKAKEIHESNK; | KAKEIHESNKM; | AKEIHESNKMS; |
| KEIHESNKMSD; | EIHESNKMSDK; | IHESNKMSDKK; | HESNKMSDKKI; |
| ESNKMSDKKIM; | SNKMSDKKIME; | NKMSDKKIMEL; | KMSDKKIMELK; |
| MSDKKIMELKE; | SDKKIMELKEE; | DKKIMELKEEY; | KKIMELKEEYN; |
| KIMELKEEYNN; | IMELKEEYNNT; | MELKEEYNNTS; | ELKEEYNNTSY; |
| LKEEYNNTSYT; | KEEYNNTSYTS; | EEYNNTSYTSY; | EYNNTSYTSYS; |
| YNNTSYTSYSE; | NNTSYTSYSEF; | NTSYTSYSEFL; | TSYTSYSEFLE; |
| SYTSYSEFLEN; | YTSYSEFLENY; | TSYSEFLENYY; | SYSEFLENYYE; |
| YSEFLENYYES; | SEFLENYYESL; | EFLENYYESLN; | FLENYYESLND; |
| LENYYESLNDQ; | ENYYESLNDQI; | NYYESLNDQIK; | YYESLNDQIKK; |
| YESLNDQIKKT; | ESLNDQIKKTN; | SLNDQIKKTNI; | LNDQIKKTNIK; |
| NDQIKKTNIKE; | DQIKKTNIKEL; | QIKKTNIKELL; | IKKTNIKELLI; |
| KKTNIKELLIE; | KTNIKELLIEK; | TNIKELLIEKY; | NIKELLIEKYK; |
| IKELLIEKYKK; | KELLIEKYKKE; | ELLIEKYKKEL; | LLIEKYKKELI; |
| LIEKYKKELIV; | IEKYKKELIVL; | EKYKKELIVLE; | KYKKELIVLEK; |
| YKKELIVLEKR; | KKELIVLEKRI; | KELIVLEKRID; | ELIVLEKRIDS; |
| LIVLEKRIDSL; | IVLEKRIDSLK; | VLEKRIDSLKQ; | LEKRIDSLKQQ; |
| EKRIDSLKQQI; | KRIDSLKQQIK; | RIDSLKQQIKL; | IDSLKQQIKLL; |
| DSLKQQIKLLE; | SLKQQIKLLEN; | LKQQIKLLENI; | KQQIKLLENIE; |
| QQIKLLENIEN; | QIKLLENIENE; | IKLLENIENEK; | KLLENIENEKE; |
| LLENIENEKEK; | LENIENEKEKG; | ENIENEKEKGE; | NIENEKEKGEL; |
| IENEKEKGELI; | ENEKEKGELIL; | NEKEKGELILL; | EKEKGELILLN; |
| KEKGELILLNI; | EKGELILLNIN; | KGELILLNINK; | GELILLNINKI; |
| ELILLNINKIQ; | LILLNINKIQK; | ILLNINKIQKG; | LLNINKIQKGI; |
| LNINKIQKGIK; | NINKIQKGIKE; | INKIQKGIKEI; | NKIQKGIKEIN; |
| KIQKGIKEINL; | IQKGIKEINLL; | QKGIKEINLLN; | KGIKEINLLNY; |
| GIKEINLLNYK; | IKEINLLNYKE; | KEINLLNYKEE; | EINLLNYKEEK; |

| | | | |
|---|---|---|---|
| INLLNYKEEKI; | NLLNYKEEKIK; | LLNYKEEKIKI; | LNYKEEKIKIS; |
| NYKEEKIKISL; | YKEEKIKISLN; | KEEKIKISLNQ; | EEKIKISLNQS; |
| EKIKISLNQSL; | KIKISLNQSLS; | IKISLNQSLSP; | KISLNQSLSPK; |
| ISLNQSLSPKE; | SLNQSLSPKEN; | LNQSLSPKENA; | NQSLSPKENAL; |
| QSLSPKENALQ; | SLSPKENALQY; | LSPKENALQYF; | SPKENALQYFK; |
| PKENALQYFKA; | KENALQYFKAY; | ENALQYFKAYK; | NALQYFKAYKK; |
| ALQYFKAYKKG; | LQYFKAYKKGK; | QYFKAYKKGKN; | YFKAYKKGKNS; |
| FKAYKKGKNSF; | KAYKKGKNSFK; | AYKKGKNSFKT; | YKKGKNSFKTI; |
| KKGKNSFKTIQ; | KGKNSFKTIQN; | GKNSFKTIQNQ; | KNSFKTIQNQL; |
| NSFKTIQNQLK; | SFKTIQNQLKD; | FKTIQNQLKDN; | KTIQNQLKDNL; |
| TIQNQLKDNLD; | IQNQLKDNLDK; | QNQLKDNLDKF; | NQLKDNLDKFN; |
| QLKDNLDKFNL; | LKDNLDKFNLI; | KDNLDKFNLIQ; | DNLDKFNLIQS; |
| NLDKFNLIQSK; | LDKFNLIQSKI; | DKFNLIQSKIT; | KFNLIQSKITM; |
| FNLIQSKITML; | NLIQSKITMLK; | LIQSKITMLKV; | IQSKITMLKVE; |
| QSKITMLKVEN; | SKITMLKVENL; | KITMLKVENLI; | ITMLKVENLIP; |
| TMLKVENLIPE; | MLKVENLIPEE; | LKVENLIPEEE; | KVENLIPEEEY; |
| VENLIPEEEYN; | ENLIPEEEYNQ; | NLIPEEEYNQE; | LIPEEEYNQEK; |
| IPEEEYNQEKT; | PEEEYNQEKTA; | EEEYNQEKTAI; | EEYNQEKTAIK; |
| EYNQEKTAIKE; | YNQEKTAIKEK; | NQEKTAIKEKE; | QEKTAIKEKEK; |
| EKTAIKEKEKT; | KTAIKEKEKTP; | TAIKEKEKTPK; | AIKEKEKTPKI; |
| IKEKEKTPKIG; | KEKEKTPKIGL; | EKEKTPKIGLH; | KEKTPKIGLHF; |
| EKTPKIGLHFT; | KTPKIGLHFTY; | TPKIGLHFTYC; | PKIGLHFTYCG; |
| KIGLHFTYCGF; | IGLHFTYCGFE; | GLHFTYCGFEI; | LHFTYCGFEIL; |
| HFTYCGFEILI; | FTYCGFEILIG; | TYCGFEILIGR; | YCGFEILIGRN; |
| CGFEILIGRNA; | GFEILIGRNAK; | FEILIGRNAKE; | EILIGRNAKEN; |
| ILIGRNAKEND; | LIGRNAKENDK; | IGRNAKENDKL; | GRNAKENDKLL; |
| RNAKENDKLLR; | NAKENDKLLRH; | AKENDKLLRHC; | KENDKLLRHCV; |
| ENDKLLRHCVK; | NDKLLRHCVKG; | DKLLRHCVKGN; | KLLRHCVKGND; |
| LLRHCVKGNDY; | LRHCVKGNDYW; | RHCVKGNDYWL; | HCVKGNDYWLH; |
| CVKGNDYWLHT; | VKGNDYWLHTR; | KGNDYWLHTRD; | GNDYWLHTRDY; |
| NDYWLHTRDYP; | DYWLHTRDYPG; | YWLHTRDYPGA; | WLHTRDYPGAY; |
| LHTRDYPGAYV; | HTRDYPGAYVF; | TRDYPGAYVFI; | RDYPGAYVFIK; |
| DYPGAYVFIKN; | YPGAYVFIKNQ; | PGAYVFIKNQK; | GAYVFIKNQKN; |
| AYVFIKNQKNK; | YVFIKNQKNKT; | VFIKNQKNKTP; | FIKNQKNKTPS; |
| IKNQKNKTPSL; | KNQKNKTPSLD; | NQKNKTPSLDV; | QKNKTPSLDVL; |
| KNKTPSLDVLL; | NKTPSLDVLLG; | KTPSLDVLLGA; | TPSLDVLLGAG; |
| PSLDVLLGAGN; | SLDVLLGAGNL; | LDVLLGAGNLC; | DVLLGAGNLCV; |
| VLLGAGNLCVF; | LLGAGNLCVFY; | LGAGNLCVFYT; | GAGNLCVFYTK; |
| AGNLCVFYTKL; | GNLCVFYTKLA; | NLCVFYTKLAK; | LCVFYTKLAKK; |
| CVFYTKLAKKS; | VFYTKLAKKSG; | FYTKLAKKSGK; | YTKLAKKSGKA; |
| TKLAKKSGKAD; | KLAKKSGKADL; | LAKKSGKADLY; | AKKSGKADLYY; |
| KKSGKADLYYT; | KSGKADLYYTQ; | SGKADLYYTQV; | GKADLYYTQVK; |
| KADLYYTQVKN; | ADLYYTQVKNL; | DLYYTQVKNLR; | LYYTQVKNLRR; |
| YYTQVKNLRRV; | YTQVKNLRRVK; | TQVKNLRRVKN; | QVKNLRRVKNK; |
| VKNLRRVKNKK; | KNLRRVKNKKL; | NLRRVKNKKLG; | LRRVKNKKLGL; |
| RRVKNKKLGLV; | RVKNKKLGLVI; | VKNKKLGLVIP; | KNKKLGLVIPK; |
| NKKLGLVIPKA; | KKLGLVIPKAE; | KLGLVIPKAEK; | LGLVIPKAEKN; |
| GLVIPKAEKNL; | LVIPKAEKNLH; | VIPKAEKNLHI; | IPKAEKNLHIK; |
| PKAEKNLHIKL; | KAEKNLHIKLD; | AEKNLHIKLDE; | EKNLHIKLDEN; |
| KNLHIKLDENL; | NLHIKLDENLI; | LHIKLDENLIK; | HIKLDENLIKK; |
| IKLDENLIKKI; | KLDENLIKKIK; | LDENLIKKIKN; | DENLIKKIKNQ; |

| | |
|---|---|
| | ENLIKKIKNQT; |
| Seq 21 | SEQ ID NO 22322-23355/<br>8 mers:<br>MKLQRSLS; KLQRSLSL; LQRSLSLI; QRSLSLII; RSLSLIIF;<br>SLSLIIFS; LSLIIFSL; SLIIFSLT; LIIFSLTV; IIFSLTVL;<br>IFSLTVLC; FSLTVLCC; SLTVLCCD; LTVLCCDN; TVLCCDNK;<br>VLCCDNKE; LCCDNKER; CCDNKERK; CDNKERKE; DNKERKEG;<br>NKERKEGV; KERKEGVS; ERKEGVST; RKEGVSTK; KEGVSTKI;<br>EGVSTKIS; GVSTKISL; VSTKISLG; STKISLGA; TKISLGAE;<br>KISLGAEP; ISLGAEPR; SLGAEPRS; LGAEPRSL; GAEPRSLD;<br>AEPRSLDP; EPRSLDPQ; PRSLDPQL; RSLDPQLA; SLDPQLAE;<br>LDPQLAED; DPQLAEDN; PQLAEDNV; QLAEDNVA; LAEDNVAS;<br>AEDNVASK; EDNVASKM; DNVASKMI; NVASKMID; VASKMIDT;<br>ASKMIDTL; SKMIDTLY; KMIDTLYR; MIDTLYRG; IDTLYRGI;<br>DTLYRGIV; TLYRGIVT; LYRGIVTG; YRGIVTGD; RGIVTGDP;<br>GIVTGDPN; IVTGDPNT; VTGDPNTG; TGDPNTGG; GDPNTGGH;<br>DPNTGGHK; PNTGGHKP; NTGGHKPG; TGGHKPGL; GGHKPGLA;<br>GHKPGLAK; HKPGLAKG; KPGLAKGW; PGLAKGWE; GLAKGWET;<br>LAKGWETS; AKGWETSS; KGWETSSD; GWETSSDG; WETSSDGT;<br>ETSSDGTA; TSSDGTAY; SSDGTAYP; SDGTAYPF; DGTAYPFY;<br>GTAYPFYL; TAYPFYLR; AYPFYLRD; YPFYLRDN; PFYLRDNL;<br>FYLRDNLT; YLRDNLTW; LRDNLTWS; RDNLTWSD; DNLTWSDG;<br>NLTWSDGV; LTWSDGVA; TWSDGVAI; WSDGVAIT; SDGVAITA;<br>DGVAITAE; GVAITAEG; VAITAEGI; AITAEGIR; ITAEGIRK;<br>TAEGIRKS; AEGIRKSY; EGIRKSYL; GIRKSYLR; IRKSYLRI;<br>RKSYLRIL; KSYLRILN; SYLRILNK; YLRILNKE; LRILNKET;<br>RILNKETG; ILNKETGS; LNKETGST; NKETGSTY; KETGSTYV;<br>ETGSTYVD; TGSTYVDM; GSTYVDMV; STYVDMVK; TYVDMVKS;<br>YVDMVKSI; VDMVKSII; DMVKSIIK; MVKSIIKN; VKSIIKNG;<br>KSIIKNGQ; SIIKNGQE; IIKNGQEY; IKNGQEYF; KNGQEYFD;<br>NGQEYFDG; GQEYFDGQ; QEYFDGQV; EYFDGQVT; YFDGQVTD;<br>FDGQVTDS; DGQVTDSE; GQVTDSEL; QVTDSELG; VTDSELGI;<br>TDSELGIR; DSELGIRA; SELGIRAI; ELGIRAID; LGIRAIDS;<br>GIRAIDSK; IRAIDSKT; RAIDSKTL; AIDSKTLE; IDSKTLEI;<br>DSKTLEIT; SKTLEITL; KTLEITLA; TLEITLAS; LEITLASP;<br>EITLASPK; ITLASPKP; TLASPKPY; LASPKPYF; ASPKPYFI;<br>SPKPYFID; PKPYFIDL; KPYFIDLL; PYFIDLLV; YFIDLLVH;<br>FIDLLVHQ; IDLLVHQS; DLLVHQSF; LLVHQSFI; LVHQSFIP;<br>VHQSFIPV; HQSFIPVP; QSFIPVPV; SFIPVPVH; FIPVPVHV;<br>IPVPVHVT; PVPVHVTD; VPVHVTDK; PVHVTDKY; VHVTDKYG;<br>HVTDKYGQ; VTDKYGQN; TDKYGQNW; DKYGQNWT; KYGQNWTS;<br>YGQNWTSP; GQNWTSPE; QNWTSPEN; NWTSPENM; WTSPENMV;<br>TSPENMVT; SPENMVTS; PENMVTSG; ENMVTSGP; NMVTSGPF;<br>MVTSGPFK; VTSGPFKL; TSGPFKLK; SGPFKLKE; GPFKLKER;<br>PFKLKERI; FKLKERIP; KLKERIPS; LKERIPSE; KERIPSEK;<br>ERIPSEKY; RIPSEKYV; IPSEKYVF; PSEKYVFE; SEKYVFEK;<br>EKYVFEKD; KYVFEKDN; YVFEKDNK; VFEKDNKY; FEKDNKYY;<br>EKDNKYYD; KDNKYYDS; DNKYYDSN; NKYYDSNE; KYYDSNEV;<br>YYDSNEVE; YDSNEVEL; DSNEVELE; SNEVELEE; NEVELEEI;<br>EVELEEIT; VELEEITF; ELEEITFY; LEEITFYT; EEITFYTT; |

EITFYTTN; ITFYTTND; TFYTTNDS; FYTTNDSS; YTTNDSST;
TTNDSSTA; TNDSSTAY; NDSSTAYK; DSSTAYKM; SSTAYKMY;
STAYKMYV; TAYKMYVN; AYKMYVNE; YKMYVNEE; KMYVNEEL;
MYVNEELD; YVNEELDA; VNEELDAI; NEELDAIL; EELDAILV;
ELDAILVP; LDAILVPY; DAILVPYP; AILVPYPQ; ILVPYPQI;

9 mers:
MKLQRSLSL; KLQRSLSLI; LQRSLSLII; QRSLSLIIF; RSLSLIIFS;
SLSLIIFSL; LSLIIFSLT; SLIIFSLTV; LIIFSLTVL; IIFSLTVLC;
IFSLTVLCC; FSLTVLCCD; SLTVLCCDN; LTVLCCDNK; TVLCCDNKE;
VLCCDNKER; LCCDNKERK; CCDNKERKE; CDNKERKEG; DNKERKEGV;
NKERKEGVS; KERKEGVST; ERKEGVSTK; RKEGVSTKI; KEGVSTKIS;
EGVSTKISL; GVSTKISLG; VSTKISLGA; STKISLGAE; TKISLGAEP;
KISLGAEPR; ISLGAEPRS; SLGAEPRSL; LGAEPRSLD; GAEPRSLDP;
AEPRSLDPQ; EPRSLDPQL; PRSLDPQLA; RSLDPQLAE; SLDPQLAED;
LDPQLAEDN; DPQLAEDNV; PQLAEDNVA; QLAEDNVAS; LAEDNVASK;
AEDNVASKM; EDNVASKMI; DNVASKMID; NVASKMIDT; VASKMIDTL;
ASKMIDTLY; SKMIDTLYR; KMIDTLYRG; MIDTLYRGI; IDTLYRGIV;
DTLYRGIVT; TLYRGIVTG; LYRGIVTGD; YRGIVTGDP; RGIVTGDPN;
GIVTGDPNT; IVTGDPNTG; VTGDPNTGG; TGDPNTGGH; GDPNTGGHK;
DPNTGGHKP; PNTGGHKPG; NTGGHKPGL; TGGHKPGLA; GGHKPGLAK;
GHKPGLAKG; HKPGLAKGW; KPGLAKGWE; PGLAKGWET; GLAKGWETS;
LAKGWETSS; AKGWETSSD; KGWETSSDG; GWETSSDGT; WETSSDGTA;
ETSSDGTAY; TSSDGTAYP; SSDGTAYPF; SDGTAYPFY; DGTAYPFYL;
GTAYPFYLR; TAYPFYLRD; AYPFYLRDN; YPFYLRDNL; PFYLRDNLT;
FYLRDNLTW; YLRDNLTWS; LRDNLTWSD; RDNLTWSDG; DNLTWSDGV;
NLTWSDGVA; LTWSDGVAI; TWSDGVAIT; WSDGVAITA; SDGVAITAE;
DGVAITAEG; GVAITAEGI; VAITAEGIR; AITAEGIRK; ITAEGIRKS;
TAEGIRKSY; AEGIRKSYL; EGIRKSYLR; GIRKSYLRI; IRKSYLRIL;
RKSYLRILN; KSYLRILNK; SYLRILNKE; YLRILNKET; LRILNKETG;
RILNKETGS; ILNKETGST; LNKETGSTY; NKETGSTYV; KETGSTYVD;
ETGSTYVDM; TGSTYVDMV; GSTYVDMVK; STYVDMVKS; TYVDMVKSI;
YVDMVKSII; VDMVKSIIK; DMVKSIIKN; MVKSIIKNG; VKSIIKNGQ;
KSIIKNGQE; SIIKNGQEY; IIKNGQEYF; IKNGQEYFD; KNGQEYFDG;
NGQEYFDGQ; GQEYFDGQV; QEYFDGQVT; EYFDGQVTD; YFDGQVTDS;
FDGQVTDSE; DGQVTDSEL; GQVTDSELG; QVTDSELGI; VTDSELGIR;
TDSELGIRA; DSELGIRAI; SELGIRAID; ELGIRAIDS; LGIRAIDSK;
GIRAIDSKT; IRAIDSKTL; RAIDSKTLE; AIDSKTLEI; IDSKTLEIT;
DSKTLEITL; SKTLEITLA; KTLEITLAS; TLEITLASP; LEITLASPK;
EITLASPKP; ITLASPKPY; TLASPKPYF; LASPKPYFI; ASPKPYFID;
SPKPYFIDL; PKPYFIDLL; KPYFIDLLV; PYFIDLLVH; YFIDLLVHQ;
FIDLLVHQS; IDLLVHQSF; DLLVHQSFI; LLVHQSFIP; LVHQSFIPV;
VHQSFIPVP; HQSFIPVPV; QSFIPVPVH; SFIPVPVHV; FIPVPVHVT;
IPVPVHVTD; PVPVHVTDK; VPVHVTDKY; PVHVTDKYG; VHVTDKYGQ;
HVTDKYGQN; VTDKYGQNW; TDKYGQNWT; DKYGQNWTS; KYGQNWTSP;
YGQNWTSPE; GQNWTSPEN; QNWTSPENM; NWTSPENMV; WTSPENMVT;
TSPENMVTS; SPENMVTSG; PENMVTSGP; ENMVTSGPF; NMVTSGPFK;
MVTSGPFKL; VTSGPFKLK; TSGPFKLKE; SGPFKLKER; GPFKLKERI;
PFKLKERIP; FKLKERIPS; KLKERIPSE; LKERIPSEK; KERIPSEKY;
ERIPSEKYV; RIPSEKYVF; IPSEKYVFE; PSEKYVFEK; SEKYVFEKD;
EKYVFEKDN; KYVFEKDNK; YVFEKDNKY; VFEKDNKYY; FEKDNKYYD;

EKDNKYYDS; KDNKYYDSN; DNKYYDSNE; NKYYDSNEV; KYYDSNEVE;
YYDSNEVEL; YDSNEVELE; DSNEVELEE; SNEVELEEI; NEVELEEIT;
EVELEEITF; VELEEITFY; ELEEITFYT; LEEITFYTT; EEITFYTTN;
EITFYTTND; ITFYTTNDS; TFYTTNDSS; FYTTNDSST; YTTNDSSTA;
TTNDSSTAY; TNDSSTAYK; NDSSTAYKM; DSSTAYKMY; SSTAYKMYV;
STAYKMYVN; TAYKMYVNE; AYKMYVNEE; YKMYVNEEL; KMYVNEELD;
MYVNEELDA; YVNEELDAI; VNEELDAIL; NEELDAILV; EELDAILVP;
ELDAILVPY; LDAILVPYP; DAILVPYPQ; AILVPYPQI;

10 mers:
MKLQRSLSLI; KLQRSLSLII; LQRSLSLIIF; QRSLSLIIFS;
RSLSLIIFSL; SLSLIIFSLT; LSLIIFSLTV; SLIIFSLTVL;
LIIFSLTVLC; IIFSLTVLCC; IFSLTVLCCD; FSLTVLCCDN;
SLTVLCCDNK; LTVLCCDNKE; TVLCCDNKER; VLCCDNKERK;
LCCDNKERKE; CCDNKERKEG; CDNKERKEGV; DNKERKEGVS;
NKERKEGVST; KERKEGVSTK; ERKEGVSTKI; RKEGVSTKIS;
KEGVSTKISL; EGVSTKISLG; GVSTKISLGA; VSTKISLGAE;
STKISLGAEP; TKISLGAEPR; KISLGAEPRS; ISLGAEPRSL;
SLGAEPRSLD; LGAEPRSLDP; GAEPRSLDPQ; AEPRSLDPQL;
EPRSLDPQLA; PRSLDPQLAE; RSLDPQLAED; SLDPQLAEDN;
LDPQLAEDNV; DPQLAEDNVA; PQLAEDNVAS; QLAEDNVASK;
LAEDNVASKM; AEDNVASKMI; EDNVASKMID; DNVASKMIDT;
NVASKMIDTL; VASKMIDTLY; ASKMIDTLYR; SKMIDTLYRG;
KMIDTLYRGI; MIDTLYRGIV; IDTLYRGIVT; DTLYRGIVTG;
TLYRGIVTGD; LYRGIVTGDP; YRGIVTGDPN; RGIVTGDPNT;
GIVTGDPNTG; IVTGDPNTGG; VTGDPNTGGH; TGDPNTGGHK;
GDPNTGGHKP; DPNTGGHKPG; PNTGGHKPGL; NTGGHKPGLA;
TGGHKPGLAK; GGHKPGLAKG; GHKPGLAKGW; HKPGLAKGWE;
KPGLAKGWET; PGLAKGWETS; GLAKGWETSS; LAKGWETSSD;
AKGWETSSDG; KGWETSSDGT; GWETSSDGTA; WETSSDGTAY;
ETSSDGTAYP; TSSDGTAYPF; SSDGTAYPFY; SDGTAYPFYL;
DGTAYPFYLR; GTAYPFYLRD; TAYPFYLRDN; AYPFYLRDNL;
YPFYLRDNLT; PFYLRDNLTW; FYLRDNLTWS; YLRDNLTWSD;
LRDNLTWSDG; RDNLTWSDGV; DNLTWSDGVA; NLTWSDGVAI;
LTWSDGVAIT; TWSDGVAITA; WSDGVAITAE; SDGVAITAEG;
DGVAITAEGI; GVAITAEGIR; VAITAEGIRK; AITAEGIRKS;
ITAEGIRKSY; TAEGIRKSYL; AEGIRKSYLR; EGIRKSYLRI;
GIRKSYLRIL; IRKSYLRILN; RKSYLRILNK; KSYLRILNKE;
SYLRILNKET; YLRILNKETG; LRILNKETGS; RILNKETGST;
ILNKETGSTY; LNKETGSTYV; NKETGSTYVD; KETGSTYVDM;
ETGSTYVDMV; TGSTYVDMVK; GSTYVDMVKS; STYVDMVKSI;
TYVDMVKSII; YVDMVKSIIK; VDMVKSIIKN; DMVKSIIKNG;
MVKSIIKNGQ; VKSIIKNGQE; KSIIKNGQEY; SIIKNGQEYF;
IIKNGQEYFD; IKNGQEYFDG; KNGQEYFDGQ; NGQEYFDGQV;
GQEYFDGQVT; QEYFDGQVTD; EYFDGQVTDS; YFDGQVTDSE;
FDGQVTDSEL; DGQVTDSELG; GQVTDSELGI; QVTDSELGIR;
VTDSELGIRA; TDSELGIRAI; DSELGIRAID; SELGIRAIDS;
ELGIRAIDSK; LGIRAIDSKT; GIRAIDSKTL; IRAIDSKTLE;
RAIDSKTLEI; AIDSKTLEIT; IDSKTLEITL; DSKTLEITLA;
SKTLEITLAS; KTLEITLASP; TLEITLASPK; LEITLASPKP;
EITLASPKPY; ITLASPKPYF; TLASPKPYFI; LASPKPYFID;

ASPKPYFIDL; SPKPYFIDLL; PKPYFIDLLV; KPYFIDLLVH;
PYFIDLLVHQ; YFIDLLVHQS; FIDLLVHQSF; IDLLVHQSFI;
DLLVHQSFIP; LLVHQSFIPV; LVHQSFIPVP; VHQSFIPVPV;
HQSFIPVPVH; QSFIPVPVHV; SFIPVPVHVT; FIPVPVHVTD;
IPVPVHVTDK; PVPVHVTDKY; VPVHVTDKYG; PVHVTDKYGQ;
VHVTDKYGQN; HVTDKYGQNW; VTDKYGQNWT; TDKYGQNWTS;
DKYGQNWTSP; KYGQNWTSPE; YGQNWTSPEN; GQNWTSPENM;
QNWTSPENMV; NWTSPENMVT; WTSPENMVTS; TSPENMVTSG;
SPENMVTSGP; PENMVTSGPF; ENMVTSGPFK; NMVTSGPFKL;
MVTSGPFKLK; VTSGPFKLKE; TSGPFKLKER; SGPFKLKERI;
GPFKLKERIP; PFKLKERIPS; FKLKERIPSE; KLKERIPSEK;
LKERIPSEKY; KERIPSEKYV; ERIPSEKYVF; RIPSEKYVFE;
IPSEKYVFEK; PSEKYVFEKD; SEKYVFEKDN; EKYVFEKDNK;
KYVFEKDNKY; YVFEKDNKYY; VFEKDNKYYD; FEKDNKYYDS;
EKDNKYYDSN; KDNKYYDSNE; DNKYYDSNEV; NKYYDSNEVE;
KYYDSNEVEL; YYDSNEVELE; YDSNEVELEE; DSNEVELEEI;
SNEVELEEIT; NEVELEEITF; EVELEEITFY; VELEEITFYT;
ELEEITFYTT; LEEITFYTTN; EEITFYTTND; EITFYTTNDS;
ITFYTTNDSS; TFYTTNDSST; FYTTNDSSTA; YTTNDSSTAY;
TTNDSSTAYK; TNDSSTAYKM; NDSSTAYKMY; DSSTAYKMYV;
SSTAYKMYVN; STAYKMYVNE; TAYKMYVNEE; AYKMYVNEEL;
YKMYVNEELD; KMYVNEELDA; MYVNEELDAI; YVNEELDAIL;
VNEELDAILV; NEELDAILVP; EELDAILVPY; ELDAILVPYP;
LDAILVPYPQ; DAILVPYPQI;

11 mers:
MKLQRSLSLII; KLQRSLSLIIF; LQRSLSLIIFS; QRSLSLIIFSL;
RSLSLIIFSLT; SLSLIIFSLTV; LSLIIFSLTVL; SLIIFSLTVLC;
LIIFSLTVLCC; IIFSLTVLCCD; IFSLTVLCCDN; FSLTVLCCDNK;
SLTVLCCDNKE; LTVLCCDNKER; TVLCCDNKERK; VLCCDNKERKE;
LCCDNKERKEG; CCDNKERKEGV; CDNKERKEGVS; DNKERKEGVST;
NKERKEGVSTK; KERKEGVSTKI; ERKEGVSTKIS; RKEGVSTKISL;
KEGVSTKISLG; EGVSTKISLGA; GVSTKISLGAE; VSTKISLGAEP;
STKISLGAEPR; TKISLGAEPRS; KISLGAEPRSL; ISLGAEPRSLD;
SLGAEPRSLDP; LGAEPRSLDPQ; GAEPRSLDPQL; AEPRSLDPQLA;
EPRSLDPQLAE; PRSLDPQLAED; RSLDPQLAEDN; SLDPQLAEDNV;
LDPQLAEDNVA; DPQLAEDNVAS; PQLAEDNVASK; QLAEDNVASKM;
LAEDNVASKMI; AEDNVASKMID; EDNVASKMIDT; DNVASKMIDTL;
NVASKMIDTLY; VASKMIDTLYR; ASKMIDTLYRG; SKMIDTLYRGI;
KMIDTLYRGIV; MIDTLYRGIVT; IDTLYRGIVTG; DTLYRGIVTGD;
TLYRGIVTGDP; LYRGIVTGDPN; YRGIVTGDPNT; RGIVTGDPNTG;
GIVTGDPNTGG; IVTGDPNTGGH; VTGDPNTGGHK; TGDPNTGGHKP;
GDPNTGGHKPG; DPNTGGHKPGL; PNTGGHKPGLA; NTGGHKPGLAK;
TGGHKPGLAKG; GGHKPGLAKGW; GHKPGLAKGWE; HKPGLAKGWET;
KPGLAKGWETS; PGLAKGWETSS; GLAKGWETSSD; LAKGWETSSDG;
AKGWETSSDGT; KGWETSSDGTA; GWETSSDGTAY; WETSSDGTAYP;
ETSSDGTAYPF; TSSDGTAYPFY; SSDGTAYPFYL; SDGTAYPFYLR;
DGTAYPFYLRD; GTAYPFYLRDN; TAYPFYLRDNL; AYPFYLRDNLT;
YPFYLRDNLTW; PFYLRDNLTWS; FYLRDNLTWSD; YLRDNLTWSDG;
LRDNLTWSDGV; RDNLTWSDGVA; DNLTWSDGVAI; NLTWSDGVAIT;
LTWSDGVAITA; TWSDGVAITAE; WSDGVAITAEG; SDGVAITAEGI;

| | |
|---|---|
| | DGVAITAEGIR; GVAITAEGIRK; VAITAEGIRKS; AITAEGIRKSY;<br>ITAEGIRKSYL; TAEGIRKSYLR; AEGIRKSYLRI; EGIRKSYLRIL;<br>GIRKSYLRILN; IRKSYLRILNK; RKSYLRILNKE; KSYLRILNKET;<br>SYLRILNKETG; YLRILNKETGS; LRILNKETGST; RILNKETGSTY;<br>ILNKETGSTYV; LNKETGSTYVD; NKETGSTYVDM; KETGSTYVDMV;<br>ETGSTYVDMVK; TGSTYVDMVKS; GSTYVDMVKSI; STYVDMVKSII;<br>TYVDMVKSIIK; YVDMVKSIIKN; VDMVKSIIKNG; DMVKSIIKNGQ;<br>MVKSIIKNGQE; VKSIIKNGQEY; KSIIKNGQEYF; SIIKNGQEYFD;<br>IIKNGQEYFDG; IKNGQEYFDGQ; KNGQEYFDGQV; NGQEYFDGQVT;<br>GQEYFDGQVTD; QEYFDGQVTDS; EYFDGQVTDSE; YFDGQVTDSEL;<br>FDGQVTDSELG; DGQVTDSELGI; GQVTDSELGIR; QVTDSELGIRA;<br>VTDSELGIRAI; TDSELGIRAID; DSELGIRAIDS; SELGIRAIDSK;<br>ELGIRAIDSKT; LGIRAIDSKTL; GIRAIDSKTLE; IRAIDSKTLEI;<br>RAIDSKTLEIT; AIDSKTLEITL; IDSKTLEITLA; DSKTLEITLAS;<br>SKTLEITLASP; KTLEITLASPK; TLEITLASPKP; LEITLASPKPY;<br>EITLASPKPYF; ITLASPKPYFI; TLASPKPYFID; LASPKPYFIDL;<br>ASPKPYFIDLL; SPKPYFIDLLV; PKPYFIDLLVH; KPYFIDLLVHQ;<br>PYFIDLLVHQS; YFIDLLVHQSF; FIDLLVHQSFI; IDLLVHQSFIP;<br>DLLVHQSFIPV; LLVHQSFIPVP; LVHQSFIPVPV; VHQSFIPVPVH;<br>HQSFIPVPVHV; QSFIPVPVHVT; SFIPVPVHVTD; FIPVPVHVTDK;<br>IPVPVHVTDKY; PVPVHVTDKYG; VPVHVTDKYGQ; PVHVTDKYGQN;<br>VHVTDKYGQNW; HVTDKYGQNWT; VTDKYGQNWTS; TDKYGQNWTSP;<br>DKYGQNWTSPE; KYGQNWTSPEN; YGQNWTSPENM; GQNWTSPENMV;<br>QNWTSPENMVT; NWTSPENMVTS; WTSPENMVTSG; TSPENMVTSGP;<br>SPENMVTSGPF; PENMVTSGPFK; ENMVTSGPFKL; NMVTSGPFKLK;<br>MVTSGPFKLKE; VTSGPFKLKER; TSGPFKLKERI; SGPFKLKERIP;<br>GPFKLKERIPS; PFKLKERIPSE; FKLKERIPSEK; KLKERIPSEKY;<br>LKERIPSEKYV; KERIPSEKYVF; ERIPSEKYVFE; RIPSEKYVFEK;<br>IPSEKYVFEKD; PSEKYVFEKDN; SEKYVFEKDNK; EKYVFEKDNKY;<br>KYVFEKDNKYY; YVFEKDNKYYD; VFEKDNKYYDS; FEKDNKYYDSN;<br>EKDNKYYDSNE; KDNKYYDSNEV; DNKYYDSNEVE; NKYYDSNEVEL;<br>KYYDSNEVELE; YYDSNEVELEE; YDSNEVELEEI; DSNEVELEEIT;<br>SNEVELEEITF; NEVELEEITFY; EVELEEITFYT; VELEEITFYTT;<br>ELEEITFYTTN; LEEITFYTTND; EEITFYTTNDS; EITFYTTNDSS;<br>ITFYTTNDSST; TFYTTNDSSTA; FYTTNDSSTAY; YTTNDSSTAYK;<br>TTNDSSTAYKM; TNDSSTAYKMY; NDSSTAYKMYV; DSSTAYKMYVN;<br>SSTAYKMYVNE; STAYKMYVNEE; TAYKMYVNEEL; AYKMYVNEELD;<br>YKMYVNEELDA; KMYVNEELDAI; MYVNEELDAIL; YVNEELDAILV;<br>VNEELDAILVP; NEELDAILVPY; EELDAILVPYP; ELDAILVPYPQ;<br>LDAILVPYPQI; |
| Seq 22 | SEQ ID NO 23356-24473/<br>8 mers:<br>MNLINKLF; NLINKLFI; LINKLFIL; INKLFILT; NKLFILTI;<br>KLFILTIL; LFILTILF; FILTILFS; ILTILFSS; LTILFSSV;<br>TILFSSVI; ILFSSVIS; LFSSVISC; FSSVISCK; SSVISCKL;<br>SVISCKLY; VISCKLYK; ISCKLYKK; SCKLYKKI; CKLYKKIT;<br>KLYKKITY; LYKKITYN; YKKITYNA; KKITYNAD; KITYNADQ;<br>ITYNADQV; TYNADQVI; YNADQVID; NADQVIDK; ADQVIDKL;<br>DQVIDKLK; QVIDKLKS; VIDKLKSN; IDKLKSNN; DKLKSNNG;<br>KLKSNNGS; LKSNNGSF; KSNNGSFN; SNNGSFNT; NNGSFNTL; |

NGSFNTLK; GSFNTLKS; SFNTLKSN; FNTLKSND; NTLKSNDD;
TLKSNDDS; LKSNDDSK; KSNDDSKR; SNDDSKRS; NDDSKRSG;
DDSKRSGR; DSKRSGRK; SKRSGRKP; KRSGRKPR; RSGRKPRS;
SGRKPRSV; GRKPRSVD; RKPRSVDN; KPRSVDNT; PRSVDNTY;
RSVDNTYM; SVDNTYMD; VDNTYMDQ; DNTYMDQD; NTYMDQDT;
TYMDQDTG; YMDQDTGK; MDQDTGKK; DQDTGKKP; QDTGKKPL;
DTGKKPLM; TGKKPLMA; GKKPLMAD; KKPLMADM; KPLMADMQ;
PLMADMQP; LMADMQPD; MADMQPDM; ADMQPDMQ; DMQPDMQN;
MQPDMQND; QPDMQNDN; PDMQNDNS; DMQNDNSS; MQNDNSSS;
QNDNSSSN; NDNSSSNH; DNSSSNHT; NSSSNHTL; SSSNHTLQ;
SSNHTLQV; SNHTLQVN; NHTLQVNI; HTLQVNIQ; TLQVNIQD;
LQVNIQDN; QVNIQDNE; VNIQDNEA; NIQDNEAS; IQDNEASE;
QDNEASEA; DNEASEAR; NEASEARN; EASEARNI; ASEARNIM;
SEARNIMT; EARNIMTE; ARNIMTEI; RNIMTEIE; NIMTEIES;
IMTEIESS; MTEIESSK; TEIESSKE; EIESSKEE; IESSKEEY;
ESSKEEYN; SSKEEYNR; SKEEYNRI; KEEYNRIN; EEYNRINE;
EYNRINED; YNRINEDL; NRINEDLA; RINEDLAK; INEDLAKV;
NEDLAKVK; EDLAKVKA; DLAKVKAS; LAKVKASL; AKVKASLD;
KVKASLDK; VKASLDKI; KASLDKIK; ASLDKIKS; SLDKIKSL;
LDKIKSLL; DKIKSLLS; KIKSLLST; IKSLLSTA; KSLLSTAK;
SLLSTAKS; LLSTAKSY; LSTAKSYL; STAKSYLE; TAKSYLEQ;
AKSYLEQT; KSYLEQTR; SYLEQTRR; YLEQTRRG; LEQTRRGV;
EQTRRGVG; QTRRGVGS; TRRGVGSS; RRGVGSSK; RGVGSSKA;
GVGSSKAN; VGSSKANL; GSSKANLA; SSKANLAL; SKANLALL;
KANLALLP; ANLALLPS; NLALLPSL; LALLPSLE; ALLPSLEE;
LLPSLEEA; LPSLEEAI; PSLEEAIA; SLEEAIAK; LEEAIAKV;
EEAIAKVK; EAIAKVKS; AIAKVKSN; IAKVKSNH; AKVKSNHA;
KVKSNHAS; VKSNHASA; KSNHASAD; SNHASADT; NHASADTH;
HASADTHC; ASADTHCN; SADTHCND; ADTHCNDA; DTHCNDAI;
THCNDAIA; HCNDAIAA; CNDAIAAL; NDAIAALK; DAIAALKR;
AIAALKRA; IAALKRAK; AALKRAKN; ALKRAKND; LKRAKNDF;
KRAKNDFE; RAKNDFEY; AKNDFEYA; KNDFEYAQ; NDFEYAQR;
DFEYAQRK; FEYAQRKA; EYAQRKAD; YAQRKADR; AQRKADRA;
QRKADRAL; RKADRALE; KADRALEE; ADRALEEA; DRALEEAL;
RALEEALS; ALEEALSN; LEEALSNS; EEALSNSN; EALSNSNA;
ALSNSNAS; LSNSNASR; SNSNASRH; NSNASRHE; SNASRHES;
NASRHESY; ASRHESYY; SRHESYYY; RHESYYYA; HESYYYAG;
ESYYYAGY; SYYYAGYH; YYYAGYHQ; YYAGYHQF; YAGYHQFM;
AGYHQFMA; GYHQFMAD; YHQFMADA; HQFMADAK; QFMADAKA;
FMADAKAS; MADAKASM; ADAKASMS; DAKASMSS; AKASMSST;
KASMSSTK; ASMSSTKS; SMSSTKSL; MSSTKSLL; SSTKSLLE;
STKSLLEV; TKSLLEVA; KSLLEVAK; SLLEVAKN; LLEVAKNK;
LEVAKNKQ; EVAKNKQK; VAKNKQKE; AKNKQKEL; KNKQKELN;
NKQKELNE; KQKELNEN; QKELNENM; KELNENMT; ELNENMTK;
LNENMTKT; NENMTKTN; ENMTKTNK; NMTKTNKD; MTKTNKDF;
TKTNKDFQ; KTNKDFQE; TNKDFQEL; NKDFQELN; KDFQELND;
DFQELNDI; FQELNDIY; QELNDIYK; ELNDIYKK; LNDIYKKL;
NDIYKKLQ; DIYKKLQD; IYKKLQDM; YKKLQDMD; KKLQDMDS;
KLQDMDSR;

9 mers:

MNLINKLFI; NLINKLFIL; LINKLFILT; INKLFILTI; NKLFILTIL;
KLFILTILF; LFILTILFS; FILTILFSS; ILTILFSSV; LTILFSSVI;
TILFSSVIS; ILFSSVISC; LFSSVISCK; FSSVISCKL; SSVISCKLY;
SVISCKLYK; VISCKLYKK; ISCKLYKKI; SCKLYKKIT; CKLYKKITY;
KLYKKITYN; LYKKITYNA; YKKITYNAD; KKITYNADQ; KITYNADQV;
ITYNADQVI; TYNADQVID; YNADQVIDK; NADQVIDKL; ADQVIDKLK;
DQVIDKLKS; QVIDKLKSN; VIDKLKSNN; IDKLKSNNG; DKLKSNNGS;
KLKSNNGSF; LKSNNGSFN; KSNNGSFNT; SNNGSFNTL; NNGSFNTLK;
NGSFNTLKS; GSFNTLKSN; SFNTLKSND; FNTLKSNDD; NTLKSNDDS;
TLKSNDDSK; LKSNDDSKR; KSNDDSKRS; SNDDSKRSG; NDDSKRSGR;
DDSKRSGRK; DSKRSGRKP; SKRSGRKPR; KRSGRKPRS; RSGRKPRSV;
SGRKPRSVD; GRKPRSVDN; RKPRSVDNT; KPRSVDNTY; PRSVDNTYM;
RSVDNTYMD; SVDNTYMDQ; VDNTYMDQD; DNTYMDQDT; NTYMDQDTG;
TYMDQDTGK; YMDQDTGKK; MDQDTGKKP; DQDTGKKPL; QDTGKKPLM;
DTGKKPLMA; TGKKPLMAD; GKKPLMADM; KKPLMADMQ; KPLMADMQP;
PLMADMQPD; LMADMQPDM; MADMQPDMQ; ADMQPDMQN; DMQPDMQND;
MQPDMQNDN; QPDMQNDNS; PDMQNDNSS; DMQNDNSSS; MQNDNSSSN;
QNDNSSSNH; NDNSSSNHT; DNSSSNHTL; NSSSNHTLQ; SSSNHTLQV;
SSNHTLQVN; SNHTLQVNI; NHTLQVNIQ; HTLQVNIQD; TLQVNIQDN;
LQVNIQDNE; QVNIQDNEA; VNIQDNEAS; NIQDNEASE; IQDNEASEA;
QDNEASEAR; DNEASEARN; NEASEARNI; EASEARNIM; ASEARNIMT;
SEARNIMTE; EARNIMTEI; ARNIMTEIE; RNIMTEIES; NIMTEIESS;
IMTEIESSK; MTEIESSKE; TEIESSKEE; EIESSKEEY; IESSKEEYN;
ESSKEEYNR; SSKEEYNRI; SKEEYNRIN; KEEYNRINE; EEYNRINED;
EYNRINEDL; YNRINEDLA; NRINEDLAK; RINEDLAKV; INEDLAKVK;
NEDLAKVKA; EDLAKVKAS; DLAKVKASL; LAKVKASLD; AKVKASLDK;
KVKASLDKI; VKASLDKIK; KASLDKIKS; ASLDKIKSL; SLDKIKSLL;
LDKIKSLLS; DKIKSLLST; KIKSLLSTA; IKSLLSTAK; KSLLSTAKS;
SLLSTAKSY; LLSTAKSYL; LSTAKSYLE; STAKSYLEQ; TAKSYLEQT;
AKSYLEQTR; KSYLEQTRR; SYLEQTRRG; YLEQTRRGV; LEQTRRGVG;
EQTRRGVGS; QTRRGVGSS; TRRGVGSSK; RRGVGSSKA; RGVGSSKAN;
GVGSSKANL; VGSSKANLA; GSSKANLAL; SSKANLALL; SKANLALLP;
KANLALLPS; ANLALLPSL; NLALLPSLE; LALLPSLEE; ALLPSLEEA;
LLPSLEEAI; LPSLEEAIA; PSLEEAIAK; SLEEAIAKV; LEEAIAKVK;
EEAIAKVKS; EAIAKVKSN; AIAKVKSNH; IAKVKSNHA; AKVKSNHAS;
KVKSNHASA; VKSNHASAD; KSNHASADT; SNHASADTH; NHASADTHC;
HASADTHCN; ASADTHCND; SADTHCNDA; ADTHCNDAI; DTHCNDAIA;
THCNDAIAA; HCNDAIAAL; CNDAIAALK; NDAIAALKR; DAIAALKRA;
AIAALKRAK; IAALKRAKN; AALKRAKND; ALKRAKNDF; LKRAKNDFE;
KRAKNDFEY; RAKNDFEYA; AKNDFEYAQ; KNDFEYAQR; NDFEYAQRK;
DFEYAQRKA; FEYAQRKAD; EYAQRKADR; YAQRKADRA; AQRKADRAL;
QRKADRALE; RKADRALEE; KADRALEEA; ADRALEEAL; DRALEEALS;
RALEEALSN; ALEEALSNS; LEEALSNSN; EEALSNSNA; EALSNSNAS;
ALSNSNASR; LSNSNASRH; SNSNASRHE; NSNASRHES; SNASRHESY;
NASRHESYY; ASRHESYYY; SRHESYYYA; RHESYYYAG; HESYYYAGY;
ESYYYAGYH; SYYYAGYHQ; YYYAGYHQF; YYAGYHQFM; YAGYHQFMA;
AGYHQFMAD; GYHQFMADA; YHQFMADAK; HQFMADAKA; QFMADAKAS;
FMADAKASM; MADAKASMS; ADAKASMSS; DAKASMSST; AKASMSSTK;
KASMSSTKS; ASMSSTKSL; SMSSTKSLL; MSSTKSLLE; SSTKSLLEV;
STKSLLEVA; TKSLLEVAK; KSLLEVAKN; SLLEVAKNK; LLEVAKNKQ;
LEVAKNKQK; EVAKNKQKE; VAKNKQKEL; AKNKQKELN; KNKQKELNE;

NKQKELNEN; KQKELNENM; QKELNENMT; KELNENMTK; ELNENMTKT;
LNENMTKTN; NENMTKTNK; ENMTKTNKD; NMTKTNKDF; MTKTNKDFQ;
TKTNKDFQE; KTNKDFQEL; TNKDFQELN; NKDFQELND; KDFQELNDI;
DFQELNDIY; FQELNDIYK; QELNDIYKK; ELNDIYKKL; LNDIYKKLQ;
NDIYKKLQD; DIYKKLQDM; IYKKLQDMD; YKKLQDMDS; KKLQDMDSR;

10 mers:
MNLINKLFIL; NLINKLFILT; LINKLFILTI; INKLFILTIL;
NKLFILTILF; KLFILTILFS; LFILTILFSS; FILTILFSSV;
ILTILFSSVI; LTILFSSVIS; TILFSSVISC; ILFSSVISCK;
LFSSVISCKL; FSSVISCKLY; SSVISCKLYK; SVISCKLYKK;
VISCKLYKKI; ISCKLYKKIT; SCKLYKKITY; CKLYKKITYN;
KLYKKITYNA; LYKKITYNAD; YKKITYNADQ; KKITYNADQV;
KITYNADQVI; ITYNADQVID; TYNADQVIDK; YNADQVIDKL;
NADQVIDKLK; ADQVIDKLKS; DQVIDKLKSN; QVIDKLKSNN;
VIDKLKSNNG; IDKLKSNNGS; DKLKSNNGSF; KLKSNNGSFN;
LKSNNGSFNT; KSNNGSFNTL; SNNGSFNTLK; NNGSFNTLKS;
NGSFNTLKSN; GSFNTLKSND; SFNTLKSNDD; FNTLKSNDDS;
NTLKSNDDSK; TLKSNDDSKR; LKSNDDSKRS; KSNDDSKRSG;
SNDDSKRSGR; NDDSKRSGRK; DDSKRSGRKP; DSKRSGRKPR;
SKRSGRKPRS; KRSGRKPRSV; RSGRKPRSVD; SGRKPRSVDN;
GRKPRSVDNT; RKPRSVDNTY; KPRSVDNTYM; PRSVDNTYMD;
RSVDNTYMDQ; SVDNTYMDQD; VDNTYMDQDT; DNTYMDQDTG;
NTYMDQDTGK; TYMDQDTGKK; YMDQDTGKKP; MDQDTGKKPL;
DQDTGKKPLM; QDTGKKPLMA; DTGKKPLMAD; TGKKPLMADM;
GKKPLMADMQ; KKPLMADMQP; KPLMADMQPD; PLMADMQPDM;
LMADMQPDMQ; MADMQPDMQN; ADMQPDMQND; DMQPDMQNDN;
MQPDMQNDNS; QPDMQNDNSS; PDMQNDNSSS; DMQNDNSSSN;
MQNDNSSSNH; QNDNSSSNHT; NDNSSSNHTL; DNSSSNHTLQ;
NSSSNHTLQV; SSSNHTLQVN; SSNHTLQVNI; SNHTLQVNIQ;
NHTLQVNIQD; HTLQVNIQDN; TLQVNIQDNE; LQVNIQDNEA;
QVNIQDNEAS; VNIQDNEASE; NIQDNEASEA; IQDNEASEAR;
QDNEASEARN; DNEASEARNI; NEASEARNIM; EASEARNIMT;
ASEARNIMTE; SEARNIMTEI; EARNIMTEIE; ARNIMTEIES;
RNIMTEIESS; NIMTEIESSK; IMTEIESSKE; MTEIESSKEE;
TEIESSKEEY; EIESSKEEYN; IESSKEEYNR; ESSKEEYNRI;
SSKEEYNRIN; SKEEYNRINE; KEEYNRINED; EEYNRINEDL;
EYNRINEDLA; YNRINEDLAK; NRINEDLAKV; RINEDLAKVK;
INEDLAKVKA; NEDLAKVKAS; EDLAKVKASL; DLAKVKASLD;
LAKVKASLDK; AKVKASLDKI; KVKASLDKIK; VKASLDKIKS;
KASLDKIKSL; ASLDKIKSLL; SLDKIKSLLS; LDKIKSLLST;
DKIKSLLSTA; KIKSLLSTAK; IKSLLSTAKS; KSLLSTAKSY;
SLLSTAKSYL; LLSTAKSYLE; LSTAKSYLEQ; STAKSYLEQT;
TAKSYLEQTR; AKSYLEQTRR; KSYLEQTRRG; SYLEQTRRGV;
YLEQTRRGVG; LEQTRRGVGS; EQTRRGVGSS; QTRRGVGSSK;
TRRGVGSSKA; RRGVGSSKAN; RGVGSSKANL; GVGSSKANLA;
VGSSKANLAL; GSSKANLALL; SSKANLALLP; SKANLALLPS;
KANLALLPSL; ANLALLPSLE; NLALLPSLEE; LALLPSLEEA;
ALLPSLEEAI; LLPSLEEAIA; LPSLEEAIAK; PSLEEAIAKV;
SLEEAIAKVK; LEEAIAKVKS; EEAIAKVKSN; EAIAKVKSNH;
AIAKVKSNHA; IAKVKSNHAS; AKVKSNHASA; KVKSNHASAD;

```
VKSNHASADT;  KSNHASADTH;  SNHASADTHC;  NHASADTHCN;
HASADTHCND;  ASADTHCNDA;  SADTHCNDAI;  ADTHCNDAIA;
DTHCNDAIAA;  THCNDAIAAL;  HCNDAIAALK;  CNDAIAALKR;
NDAIAALKRA;  DAIAALKRAK;  AIAALKRAKN;  IAALKRAKND;
AALKRAKNDF;  ALKRAKNDFE;  LKRAKNDFEY;  KRAKNDFEYA;
RAKNDFEYAQ;  AKNDFEYAQR;  KNDFEYAQRK;  NDFEYAQRKA;
DFEYAQRKAD;  FEYAQRKADR;  EYAQRKADRA;  YAQRKADRAL;
AQRKADRALE;  QRKADRALEE;  RKADRALEEA;  KADRALEEAL;
ADRALEEALS;  DRALEEALSN;  RALEEALSNS;  ALEEALSNSN;
LEEALSNSNA;  EEALSNSNAS;  EALSNSNASR;  ALSNSNASRH;
LSNSNASRHE;  SNSNASRHES;  NSNASRHESY;  SNASRHESYY;
NASRHESYYY;  ASRHESYYYA;  SRHESYYYAG;  RHESYYYAGY;
HESYYYAGYH;  ESYYYAGYHQ;  SYYYAGYHQF;  YYYAGYHQFM;
YYAGYHQFMA;  YAGYHQFMAD;  AGYHQFMADA;  GYHQFMADAK;
YHQFMADAKA;  HQFMADAKAS;  QFMADAKASM;  FMADAKASMS;
MADAKASMSS;  ADAKASMSST;  DAKASMSSTK;  AKASMSSTKS;
KASMSSTKSL;  ASMSSTKSLL;  SMSSTKSLLE;  MSSTKSLLEV;
SSTKSLLEVA;  STKSLLEVAK;  TKSLLEVAKN;  KSLLEVAKNK;
SLLEVAKNKQ;  LLEVAKNKQK;  LEVAKNKQKE;  EVAKNKQKEL;
VAKNKQKELN;  AKNKQKELNE;  KNKQKELNEN;  NKQKELNENM;
KQKELNENMT;  QKELNENMTK;  KELNENMTKT;  ELNENMTKTN;
LNENMTKTNK;  NENMTKTNKD;  ENMTKTNKDF;  NMTKTNKDFQ;
MTKTNKDFQE;  TKTNKDFQEL;  KTNKDFQELN;  TNKDFQELND;
NKDFQELNDI;  KDFQELNDIY;  DFQELNDIYK;  FQELNDIYKK;
QELNDIYKKL;  ELNDIYKKLQ;  LNDIYKKLQD;  NDIYKKLQDM;
DIYKKLQDMD;  IYKKLQDMDS;  YKKLQDMDSR;

11 mers:
MNLINKLFILT;  NLINKLFILTI;  LINKLFILTIL;  INKLFILTILF;
NKLFILTILFS;  KLFILTILFSS;  LFILTILFSSV;  FILTILFSSVI;
ILTILFSSVIS;  LTILFSSVISC;  TILFSSVISCK;  ILFSSVISCKL;
LFSSVISCKLY;  FSSVISCKLYK;  SSVISCKLYKK;  SVISCKLYKKI;
VISCKLYKKIT;  ISCKLYKKITY;  SCKLYKKITYN;  CKLYKKITYNA;
KLYKKITYNAD;  LYKKITYNADQ;  YKKITYNADQV;  KKITYNADQVI;
KITYNADQVID;  ITYNADQVIDK;  TYNADQVIDKL;  YNADQVIDKLK;
NADQVIDKLKS;  ADQVIDKLKSN;  DQVIDKLKSNN;  QVIDKLKSNNG;
VIDKLKSNNGS;  IDKLKSNNGSF;  DKLKSNNGSFN;  KLKSNNGSFNT;
LKSNNGSFNTL;  KSNNGSFNTLK;  SNNGSFNTLKS;  NNGSFNTLKSN;
NGSFNTLKSND;  GSFNTLKSNDD;  SFNTLKSNDDS;  FNTLKSNDDSK;
NTLKSNDDSKR;  TLKSNDDSKRS;  LKSNDDSKRSG;  KSNDDSKRSGR;
SNDDSKRSGRK;  NDDSKRSGRKP;  DDSKRSGRKPR;  DSKRSGRKPRS;
SKRSGRKPRSV;  KRSGRKPRSVD;  RSGRKPRSVDN;  SGRKPRSVDNT;
GRKPRSVDNTY;  RKPRSVDNTYM;  KPRSVDNTYMD;  PRSVDNTYMDQ;
RSVDNTYMDQD;  SVDNTYMDQDT;  VDNTYMDQDTG;  DNTYMDQDTGK;
NTYMDQDTGKK;  TYMDQDTGKKP;  YMDQDTGKKPL;  MDQDTGKKPLM;
DQDTGKKPLMA;  QDTGKKPLMAD;  DTGKKPLMADM;  TGKKPLMADMQ;
GKKPLMADMQP;  KKPLMADMQPD;  KPLMADMQPDM;  PLMADMQPDMQ;
LMADMQPDMQN;  MADMQPDMQND;  ADMQPDMQNDN;  DMQPDMQNDNS;
MQPDMQNDNSS;  QPDMQNDNSSS;  PDMQNDNSSSN;  DMQNDNSSSNH;
MQNDNSSSNHT;  QNDNSSSNHTL;  NDNSSSNHTLQ;  DNSSSNHTLQV;
NSSSNHTLQVN;  SSSNHTLQVNI;  SSNHTLQVNIQ;  SNHTLQVNIQD;
```

| | |
|---|---|
| | NHTLQVNIQDN; HTLQVNIQDNE; TLQVNIQDNEA; LQVNIQDNEAS; QVNIQDNEASE; VNIQDNEASEA; NIQDNEASEAR; IQDNEASEARN; QDNEASEARNI; DNEASEARNIM; NEASEARNIMT; EASEARNIMTE; ASEARNIMTEI; SEARNIMTEIE; EARNIMTEIES; ARNIMTEIESS; RNIMTEIESSK; NIMTEIESSKE; IMTEIESSKEE; MTEIESSKEEY; TEIESSKEEYN; EIESSKEEYNR; IESSKEEYNRI; ESSKEEYNRIN; SSKEEYNRINE; SKEEYNRINED; KEEYNRINEDL; EEYNRINEDLA; EYNRINEDLAK; YNRINEDLAKV; NRINEDLAKVK; RINEDLAKVKA; INEDLAKVKAS; NEDLAKVKASL; EDLAKVKASLD; DLAKVKASLDK; LAKVKASLDKI; AKVKASLDKIK; KVKASLDKIKS; VKASLDKIKSL; KASLDKIKSLL; ASLDKIKSLLS; SLDKIKSLLST; LDKIKSLLSTA; DKIKSLLSTAK; KIKSLLSTAKS; IKSLLSTAKSY; KSLLSTAKSYL; SLLSTAKSYLE; LLSTAKSYLEQ; LSTAKSYLEQT; STAKSYLEQTR; TAKSYLEQTRR; AKSYLEQTRRG; KSYLEQTRRGV; SYLEQTRRGVG; YLEQTRRGVGS; LEQTRRGVGSS; EQTRRGVGSSK; QTRRGVGSSKA; TRRGVGSSKAN; RRGVGSSKANL; RGVGSSKANLA; GVGSSKANLAL; VGSSKANLALL; GSSKANLALLP; SSKANLALLPS; SKANLALLPSL; KANLALLPSLE; ANLALLPSLEE; NLALLPSLEEA; LALLPSLEEAI; ALLPSLEEAIA; LLPSLEEAIAK; LPSLEEAIAKV; PSLEEAIAKVK; SLEEAIAKVKS; LEEAIAKVKSN; EEAIAKVKSNH; EAIAKVKSNHA; AIAKVKSNHAS; IAKVKSNHASA; AKVKSNHASAD; KVKSNHASADT; VKSNHASADTH; KSNHASADTHC; SNHASADTHCN; NHASADTHCND; HASADTHCNDA; ASADTHCNDAI; SADTHCNDAIA; ADTHCNDAIAA; DTHCNDAIAAL; THCNDAIAALK; HCNDAIAALKR; CNDAIAALKRA; NDAIAALKRAK; DAIAALKRAKN; AIAALKRAKND; IAALKRAKNDF; AALKRAKNDFE; ALKRAKNDFEY; LKRAKNDFEYA; KRAKNDFEYAQ; RAKNDFEYAQR; AKNDFEYAQRK; KNDFEYAQRKA; NDFEYAQRKAD; DFEYAQRKADR; FEYAQRKADRA; EYAQRKADRAL; YAQRKADRALE; AQRKADRALEE; QRKADRALEEA; RKADRALEEAL; KADRALEEALS; ADRALEEALSN; DRALEEALSNS; RALEEALSNSN; ALEEALSNSNA; LEEALSNSNAS; EEALSNSNASR; EALSNSNASRH; ALSNSNASRHE; LSNSNASRHES; SNSNASRHESY; NSNASRHESYY; SNASRHESYYY; NASRHESYYYA; ASRHESYYYAG; SRHESYYYAGY; RHESYYYAGYH; HESYYYAGYHQ; ESYYYAGYHQF; SYYYAGYHQFM; YYYAGYHQFMA; YYAGYHQFMAD; YAGYHQFMADA; AGYHQFMADAK; GYHQFMADAKA; YHQFMADAKAS; HQFMADAKASM; QFMADAKASMS; FMADAKASMSS; MADAKASMSST; ADAKASMSSTK; DAKASMSSTKS; AKASMSSTKSL; KASMSSTKSLL; ASMSSTKSLLE; SMSSTKSLLEV; MSSTKSLLEVA; SSTKSLLEVAK; STKSLLEVAKN; TKSLLEVAKNK; KSLLEVAKNKQ; SLLEVAKNKQK; LLEVAKNKQKE; LEVAKNKQKEL; EVAKNKQKELN; VAKNKQKELNE; AKNKQKELNEN; KNKQKELNENM; NKQKELNENMT; KQKELNENMTK; QKELNENMTKT; KELNENMTKTN; ELNENMTKTNK; LNENMTKTNKD; NENMTKTNKDF; ENMTKTNKDFQ; NMTKTNKDFQE; MTKTNKDFQEL; TKTNKDFQELN; KTNKDFQELND; TNKDFQELNDI; NKDFQELNDIY; KDFQELNDIYK; DFQELNDIYKK; FQELNDIYKKL; QELNDIYKKLQ; ELNDIYKKLQD; LNDIYKKLQDM; NDIYKKLQDMD; DIYKKLQDMDS; IYKKLQDMDSR; |
| Seq 23 | SEQ ID NO 24474-25783/ <br> 8 mers: <br> MIINHNTS; IINHNTSA; INHNTSAI; NHNTSAIN; HNTSAINA; |

| | NTSAINAS; TSAINASR; SAINASRN; AINASRNN; INASRNNG; |
|---|---|
| | NASRNNGI; ASRNNGIN; SRNNGINA; RNNGINAA; NNGINAAN; |
| | NGINAANL; GINAANLS; INAANLSK; NAANLSKT; AANLSKTQ; |
| | ANLSKTQE; NLSKTQEK; LSKTQEKL; SKTQEKLS; KTQEKLSS; |
| | TQEKLSSG; QEKLSSGY; EKLSSGYR; KLSSGYRI; LSSGYRIN; |
| | SSGYRINR; SGYRINRA; GYRINRAS; YRINRASD; RINRASDD; |
| | INRASDDA; NRASDDAA; RASDDAAG; ASDDAAGM; SDDAAGMG; |
| | DDAAGMGV; DAAGMGVS; AAGMGVSG; AGMGVSGK; GMGVSGKI; |
| | MGVSGKIN; GVSGKINA; VSGKINAQ; SGKINAQI; GKINAQIR; |
| | KINAQIRG; INAQIRGL; NAQIRGLS; AQIRGLSQ; QIRGLSQA; |
| | IRGLSQAS; RGLSQASR; GLSQASRN; LSQASRNT; SQASRNTS; |
| | QASRNTSK; ASRNTSKA; SRNTSKAI; RNTSKAIN; NTSKAINF; |
| | TSKAINFI; SKAINFIQ; KAINFIQT; AINFIQTT; INFIQTTE; |
| | NFIQTTEG; FIQTTEGN; IQTTEGNL; QTTEGNLN; TTEGNLNE; |
| | TEGNLNEV; EGNLNEVE; GNLNEVEK; NLNEVEKV; LNEVEKVL; |
| | NEVEKVLV; EVEKVLVR; VEKVLVRM; EKVLVRMK; KVLVRMKE; |
| | VLVRMKEL; LVRMKELA; VRMKELAV; RMKELAVQ; MKELAVQS; |
| | KELAVQSG; ELAVQSGN; LAVQSGNG; AVQSGNGT; VQSGNGTY; |
| | QSGNGTYS; SGNGTYSD; GNGTYSDA; NGTYSDAD; GTYSDADR; |
| | TYSDADRG; YSDADRGS; SDADRGSI; DADRGSIQ; ADRGSIQI; |
| | DRGSIQIE; RGSIQIEI; GSIQIEIE; SIQIEIEQ; IQIEIEQL; |
| | QIEIEQLT; IEIEQLTD; EIEQLTDE; IEQLTDEI; EQLTDEIN; |
| | QLTDEINR; LTDEINRI; TDEINRIA; DEINRIAD; EINRIADQ; |
| | INRIADQA; NRIADQAQ; RIADQAQY; IADQAQYN; ADQAQYNQ; |
| | DQAQYNQM; QAQYNQMH; AQYNQMHM; QYNQMHML; YNQMHMLS; |
| | NQMHMLSN; QMHMLSNK; MHMLSNKS; HMLSNKSA; MLSNKSAS; |
| | LSNKSASQ; SNKSASQN; NKSASQNV; KSASQNVR; SASQNVRT; |
| | ASQNVRTA; SQNVRTAE; QNVRTAEE; NVRTAEEL; VRTAEELG; |
| | RTAEELGM; TAEELGMQ; AEELGMQP; EELGMQPA; ELGMQPAK; |
| | LGMQPAKI; GMQPAKIN; MQPAKINT; QPAKINTP; PAKINTPA; |
| | AKINTPAS; KINTPASL; INTPASLS; NTPASLSG; TPASLSGS; |
| | PASLSGSQ; ASLSGSQA; SLSGSQAS; LSGSQASW; SGSQASWT; |
| | GSQASWTL; SQASWTLR; QASWTLRV; ASWTLRVH; SWTLRVHV; |
| | WTLRVHVG; TLRVHVGA; LRVHVGAN; RVHVGANQ; VHVGANQD; |
| | HVGANQDE; VGANQDEA; GANQDEAI; ANQDEAIA; NQDEAIAV; |
| | QDEAIAVN; DEAIAVNI; EAIAVNIY; AIAVNIYA; IAVNIYAA; |
| | AVNIYAAN; VNIYAANV; NIYAANVA; IYAANVAN; YAANVANL; |
| | AANVANLF; ANVANLFS; NVANLFSG; VANLFSGE; ANLFSGEG; |
| | NLFSGEGA; LFSGEGAQ; FSGEGAQT; SGEGAQTA; GEGAQTAQ; |
| | EGAQTAQA; GAQTAQAA; AQTAQAAP; QTAQAAPV; TAQAAPVQ; |
| | AQAAPVQE; QAAPVQEG; AAPVQEGV; APVQEGVQ; PVQEGVQQ; |
| | VQEGVQQE; QEGVQQEG; EGVQQEGA; GVQQEGAQ; VQQEGAQQ; |
| | QQEGAQQP; QEGAQQPA; EGAQQPAP; GAQQPAPA; AQQPAPAT; |
| | QQPAPATA; QPAPATAP; PAPATAPS; APATAPSQ; PATAPSQG; |
| | ATAPSQGG; TAPSQGGV; APSQGGVN; PSQGGVNS; SQGGVNSP; |
| | QGGVNSPV; GGVNSPVN; GVNSPVNV; VNSPVNVT; NSPVNVTT; |
| | SPVNVTTT; PVNVTTTV; VNVTTTVD; NVTTTVDA; VTTTVDAN; |
| | TTTVDANT; TTVDANTS; TVDANTSL; VDANTSLA; DANTSLAK; |
| | ANTSLAKI; NTSLAKIE; TSLAKIEN; SLAKIENA; LAKIENAI; |
| | AKIENAIR; KIENAIRM; IENAIRMI; ENAIRMIS; NAIRMISD; |
| | AIRMISDQ; IRMISDQR; RMISDQRA; MISDQRAN; ISDQRANL; |

SDQRANLG; DQRANLGA; QRANLGAF; RANLGAFQ; ANLGAFQN;
NLGAFQNR; LGAFQNRL; GAFQNRLE; AFQNRLES; FQNRLESI;
QNRLESIK; NRLESIKN; RLESIKNS; LESIKNST; ESIKNSTE;
SIKNSTEY; IKNSTEYA; KNSTEYAI; NSTEYAIE; STEYAIEN;
TEYAIENL; EYAIENLK; YAIENLKA; AIENLKAS; IENLKASY;
ENLKASYA; NLKASYAQ; LKASYAQI; KASYAQIK; ASYAQIKD;
SYAQIKDA; YAQIKDAT; AQIKDATM; QIKDATMT; IKDATMTD;
KDATMTDE; DATMTDEV; ATMTDEVV; TMTDEVVA; MTDEVVAA;
TDEVVAAT; DEVVAATT; EVVAATTN; VVAATTNS; VAATTNSI;
AATTNSIL; ATTNSILT; TTNSILTQ; TNSILTQS; NSILTQSA;
SILTQSAM; ILTQSAMA; LTQSAMAM; TQSAMAMI; QSAMAMIA;
SAMAMIAQ; AMAMIAQA; MAMIAQAN; AMIAQANQ; MIAQANQV;
IAQANQVP; AQANQVPQ; QANQVPQY; ANQVPQYV; NQVPQYVL;
QVPQYVLS; VPQYVLSL; PQYVLSLL; QYVLSLLR;

9 mers:
MIINHNTSA; IINHNTSAI; INHNTSAIN; NHNTSAINA; HNTSAINAS;
NTSAINASR; TSAINASRN; SAINASRNN; AINASRNNG; INASRNNGI;
NASRNNGIN; ASRNNGINA; SRNNGINAA; RNNGINAAN; NNGINAANL;
NGINAANLS; GINAANLSK; INAANLSKT; NAANLSKTQ; AANLSKTQE;
ANLSKTQEK; NLSKTQEKL; LSKTQEKLS; SKTQEKLSS; KTQEKLSSG;
TQEKLSSGY; QEKLSSGYR; EKLSSGYRI; KLSSGYRIN; LSSGYRINR;
SSGYRINRA; SGYRINRAS; GYRINRASD; YRINRASDD; RINRASDDA;
INRASDDAA; NRASDDAAG; RASDDAAGM; ASDDAAGMG; SDDAAGMGV;
DDAAGMGVS; DAAGMGVSG; AAGMGVSGK; AGMGVSGKI; GMGVSGKIN;
MGVSGKINA; GVSGKINAQ; VSGKINAQI; SGKINAQIR; GKINAQIRG;
KINAQIRGL; INAQIRGLS; NAQIRGLSQ; AQIRGLSQA; QIRGLSQAS;
IRGLSQASR; RGLSQASRN; GLSQASRNT; LSQASRNTS; SQASRNTSK;
QASRNTSKA; ASRNTSKAI; SRNTSKAIN; RNTSKAINF; NTSKAINFI;
TSKAINFIQ; SKAINFIQT; KAINFIQTT; AINFIQTTE; INFIQTTEG;
NFIQTTEGN; FIQTTEGNL; IQTTEGNLN; QTTEGNLNE; TTEGNLNEV;
TEGNLNEVE; EGNLNEVEK; GNLNEVEKV; NLNEVEKVL; LNEVEKVLV;
NEVEKVLVR; EVEKVLVRM; VEKVLVRMK; EKVLVRMKE; KVLVRMKEL;
VLVRMKELA; LVRMKELAV; VRMKELAVQ; RMKELAVQS; MKELAVQSG;
KELAVQSGN; ELAVQSGNG; LAVQSGNGT; AVQSGNGTY; VQSGNGTYS;
QSGNGTYSD; SGNGTYSDA; GNGTYSDAD; NGTYSDADR; GTYSDADRG;
TYSDADRGS; YSDADRGSI; SDADRGSIQ; DADRGSIQI; ADRGSIQIE;
DRGSIQIEI; RGSIQIEIE; GSIQIEIEQ; SIQIEIEQL; IQIEIEQLT;
QIEIEQLTD; IEIEQLTDE; EIEQLTDEI; IEQLTDEIN; EQLTDEINR;
QLTDEINRI; LTDEINRIA; TDEINRIAD; DEINRIADQ; EINRIADQA;
INRIADQAQ; NRIADQAQY; RIADQAQYN; IADQAQYNQ; ADQAQYNQM;
DQAQYNQMH; QAQYNQMHM; AQYNQMHML; QYNQMHMLS; YNQMHMLSN;
NQMHMLSNK; QMHMLSNKS; MHMLSNKSA; HMLSNKSAS; MLSNKSASQ;
LSNKSASQN; SNKSASQNV; NKSASQNVR; KSASQNVRT; SASQNVRTA;
ASQNVRTAE; SQNVRTAEE; QNVRTAEEL; NVRTAEELG; VRTAEELGM;
RTAEELGMQ; TAEELGMQP; AEELGMQPA; EELGMQPAK; ELGMQPAKI;
LGMQPAKIN; GMQPAKINT; MQPAKINTP; QPAKINTPA; PAKINTPAS;
AKINTPASL; KINTPASLS; INTPASLSG; NTPASLSGS; TPASLSGSQ;
PASLSGSQA; ASLSGSQAS; SLSGSQASW; LSGSQASWT; SGSQASWTL;
GSQASWTLR; SQASWTLRV; QASWTLRVH; ASWTLRVHV; SWTLRVHVG;
WTLRVHVGA; TLRVHVGAN; LRVHVGANQ; RVHVGANQD; VHVGANQDE;

HVGANQDEA; VGANQDEAI; GANQDEAIA; ANQDEAIAV; NQDEAIAVN;
QDEAIAVNI; DEAIAVNIY; EAIAVNIYA; AIAVNIYAA; IAVNIYAAN;
AVNIYAANV; VNIYAANVA; NIYAANVAN; IYAANVANL; YAANVANLF;
AANVANLFS; ANVANLFSG; NVANLFSGE; VANLFSGEG; ANLFSGEGA;
NLFSGEGAQ; LFSGEGAQT; FSGEGAQTA; SGEGAQTAQ; GEGAQTAQA;
EGAQTAQAA; GAQTAQAAP; AQTAQAAPV; QTAQAAPVQ; TAQAAPVQE;
AQAAPVQEG; QAAPVQEGV; AAPVQEGVQ; APVQEGVQQ; PVQEGVQQE;
VQEGVQQEG; QEGVQQEGA; EGVQQEGAQ; GVQQEGAQQ; VQQEGAQQP;
QQEGAQQPA; QEGAQQPAP; EGAQQPAPA; GAQQPAPAT; AQQPAPATA;
QQPAPATAP; QPAPATAPS; PAPATAPSQ; APATAPSQG; PATAPSQGG;
ATAPSQGGV; TAPSQGGVN; APSQGGVNS; PSQGGVNSP; SQGGVNSPV;
QGGVNSPVN; GGVNSPVNV; GVNSPVNVT; VNSPVNVTT; NSPVNVTTT;
SPVNVTTTV; PVNVTTTVD; VNVTTTVDA; NVTTTVDAN; VTTTVDANT;
TTTVDANTS; TTVDANTSL; TVDANTSLA; VDANTSLAK; DANTSLAKI;
ANTSLAKIE; NTSLAKIEN; TSLAKIENA; SLAKIENAI; LAKIENAIR;
AKIENAIRM; KIENAIRMI; IENAIRMIS; ENAIRMISD; NAIRMISDQ;
AIRMISDQR; IRMISDQRA; RMISDQRAN; MISDQRANL; ISDQRANLG;
SDQRANLGA; DQRANLGAF; QRANLGAFQ; RANLGAFQN; ANLGAFQNR;
NLGAFQNRL; LGAFQNRLE; GAFQNRLES; AFQNRLESI; FQNRLESIK;
QNRLESIKN; NRLESIKNS; RLESIKNST; LESIKNSTE; ESIKNSTEY;
SIKNSTEYA; IKNSTEYAI; KNSTEYAIE; NSTEYAIEN; STEYAIENL;
TEYAIENLK; EYAIENLKA; YAIENLKAS; AIENLKASY; IENLKASYA;
ENLKASYAQ; NLKASYAQI; LKASYAQIK; KASYAQIKD; ASYAQIKDA;
SYAQIKDAT; YAQIKDATM; AQIKDATMT; QIKDATMTD; IKDATMTDE;
KDATMTDEV; DATMTDEVV; ATMTDEVVA; TMTDEVVAA; MTDEVVAAT;
TDEVVAATT; DEVVAATTN; EVVAATTNS; VVAATTNSI; VAATTNSIL;
AATTNSILT; ATTNSILTQ; TTNSILTQS; TNSILTQSA; NSILTQSAM;
SILTQSAMA; ILTQSAMAM; LTQSAMAMI; TQSAMAMIA; QSAMAMIAQ;
SAMAMIAQA; AMAMIAQAN; MAMIAQANQ; AMIAQANQV; MIAQANQVP;
IAQANQVPQ; AQANQVPQY; QANQVPQYV; ANQVPQYVL; NQVPQYVLS;
QVPQYVLSL; VPQYVLSLL; PQYVLSLLR;

10 mers:
MIINHNTSAI; IINHNTSAIN; INHNTSAINA; NHNTSAINAS;
HNTSAINASR; NTSAINASRN; TSAINASRNN; SAINASRNNG;
AINASRNNGI; INASRNNGIN; NASRNNGINA; ASRNNGINAA;
SRNNGINAAN; RNNGINAANL; NNGINAANLS; NGINAANLSK;
GINAANLSKT; INAANLSKTQ; NAANLSKTQE; AANLSKTQEK;
ANLSKTQEKL; NLSKTQEKLS; LSKTQEKLSS; SKTQEKLSSG;
KTQEKLSSGY; TQEKLSSGYR; QEKLSSGYRI; EKLSSGYRIN;
KLSSGYRINR; LSSGYRINRA; SSGYRINRAS; SGYRINRASD;
GYRINRASDD; YRINRASDDA; RINRASDDAA; INRASDDAAG;
NRASDDAAGM; RASDDAAGMG; ASDDAAGMGV; SDDAAGMGVS;
DDAAGMGVSG; DAAGMGVSGK; AAGMGVSGKI; AGMGVSGKIN;
GMGVSGKINA; MGVSGKINAQ; GVSGKINAQI; VSGKINAQIR;
SGKINAQIRG; GKINAQIRGL; KINAQIRGLS; INAQIRGLSQ;
NAQIRGLSQA; AQIRGLSQAS; QIRGLSQASR; IRGLSQASRN;
RGLSQASRNT; GLSQASRNTS; LSQASRNTSK; SQASRNTSKA;
QASRNTSKAI; ASRNTSKAIN; SRNTSKAINF; RNTSKAINFI;
NTSKAINFIQ; TSKAINFIQT; SKAINFIQTT; KAINFIQTTE;
AINFIQTTEG; INFIQTTEGN; NFIQTTEGNL; FIQTTEGNLN;

IQTTEGNLNE; QTTEGNLNEV; TTEGNLNEVE; TEGNLNEVEK;
EGNLNEVEKV; GNLNEVEKVL; NLNEVEKVLV; LNEVEKVLVR;
NEVEKVLVRM; EVEKVLVRMK; VEKVLVRMKE; EKVLVRMKEL;
KVLVRMKELA; VLVRMKELAV; LVRMKELAVQ; VRMKELAVQS;
RMKELAVQSG; MKELAVQSGN; KELAVQSGNG; ELAVQSGNGT;
LAVQSGNGTY; AVQSGNGTYS; VQSGNGTYSD; QSGNGTYSDA;
SGNGTYSDAD; GNGTYSDADR; NGTYSDADRG; GTYSDADRGS;
TYSDADRGSI; YSDADRGSIQ; SDADRGSIQI; DADRGSIQIE;
ADRGSIQIEI; DRGSIQIEIE; RGSIQIEIEQ; GSIQIEIEQL;
SIQIEIEQLT; IQIEIEQLTD; QIEIEQLTDE; IEIEQLTDEI;
EIEQLTDEIN; IEQLTDEINR; EQLTDEINRI; QLTDEINRIA;
LTDEINRIAD; TDEINRIADQ; DEINRIADQA; EINRIADQAQ;
INRIADQAQY; NRIADQAQYN; RIADQAQYNQ; IADQAQYNQM;
ADQAQYNQMH; DQAQYNQMHM; QAQYNQMHML; AQYNQMHMLS;
QYNQMHMLSN; YNQMHMLSNK; NQMHMLSNKS; QMHMLSNKSA;
MHMLSNKSAS; HMLSNKSASQ; MLSNKSASQN; LSNKSASQNV;
SNKSASQNVR; NKSASQNVRT; KSASQNVRTA; SASQNVRTAE;
ASQNVRTAEE; SQNVRTAEEL; QNVRTAEELG; NVRTAEELGM;
VRTAEELGMQ; RTAEELGMQP; TAEELGMQPA; AEELGMQPAK;
EELGMQPAKI; ELGMQPAKIN; LGMQPAKINT; GMQPAKINTP;
MQPAKINTPA; QPAKINTPAS; PAKINTPASL; AKINTPASLS;
KINTPASLSG; INTPASLSGS; NTPASLSGSQ; TPASLSGSQA;
PASLSGSQAS; ASLSGSQASW; SLSGSQASWT; LSGSQASWTL;
SGSQASWTLR; GSQASWTLRV; SQASWTLRVH; QASWTLRVHV;
ASWTLRVHVG; SWTLRVHVGA; WTLRVHVGAN; TLRVHVGANQ;
LRVHVGANQD; RVHVGANQDE; VHVGANQDEA; HVGANQDEAI;
VGANQDEAIA; GANQDEAIAV; ANQDEAIAVN; NQDEAIAVNI;
QDEAIAVNIY; DEAIAVNIYA; EAIAVNIYAA; AIAVNIYAAN;
IAVNIYAANV; AVNIYAANVA; VNIYAANVAN; NIYAANVANL;
IYAANVANLF; YAANVANLFS; AANVANLFSG; ANVANLFSGE;
NVANLFSGEG; VANLFSGEGA; ANLFSGEGAQ; NLFSGEGAQT;
LFSGEGAQTA; FSGEGAQTAQ; SGEGAQTAQA; GEGAQTAQAA;
EGAQTAQAAP; GAQTAQAAPV; AQTAQAAPVQ; QTAQAAPVQE;
TAQAAPVQEG; AQAAPVQEGV; QAAPVQEGVQ; AAPVQEGVQQ;
APVQEGVQQE; PVQEGVQQEG; VQEGVQQEGA; QEGVQQEGAQ;
EGVQQEGAQQ; GVQQEGAQQP; VQQEGAQQPA; QQEGAQQPAP;
QEGAQQPAPA; EGAQQPAPAT; GAQQPAPATA; AQQPAPATAP;
QQPAPATAPS; QPAPATAPSQ; PAPATAPSQG; APATAPSQGG;
PATAPSQGGV; ATAPSQGGVN; TAPSQGGVNS; APSQGGVNSP;
PSQGGVNSPV; SQGGVNSPVN; QGGVNSPVNV; GGVNSPVNVT;
GVNSPVNVTT; VNSPVNVTTT; NSPVNVTTTV; SPVNVTTTVD;
PVNVTTTVDA; VNVTTTVDAN; NVTTTVDANT; VTTTVDANTS;
TTTVDANTSL; TTVDANTSLA; TVDANTSLAK; VDANTSLAKI;
DANTSLAKIE; ANTSLAKIEN; NTSLAKIENA; TSLAKIENAI;
SLAKIENAIR; LAKIENAIRM; AKIENAIRMI; KIENAIRMIS;
IENAIRMISD; ENAIRMISDQ; NAIRMISDQR; AIRMISDQRA;
IRMISDQRAN; RMISDQRANL; MISDQRANLG; ISDQRANLGA;
SDQRANLGAF; DQRANLGAFQ; QRANLGAFQN; RANLGAFQNR;
ANLGAFQNRL; NLGAFQNRLE; LGAFQNRLES; GAFQNRLESI;
AFQNRLESIK; FQNRLESIKN; QNRLESIKNS; NRLESIKNST;
RLESIKNSTE; LESIKNSTEY; ESIKNSTEYA; SIKNSTEYAI;

IKNSTEYAIE; KNSTEYAIEN; NSTEYAIENL; STEYAIENLK;
TEYAIENLKA; EYAIENLKAS; YAIENLKASY; AIENLKASYA;
IENLKASYAQ; ENLKASYAQI; NLKASYAQIK; LKASYAQIKD;
KASYAQIKDA; ASYAQIKDAT; SYAQIKDATM; YAQIKDATMT;
AQIKDATMTD; QIKDATMTDE; IKDATMTDEV; KDATMTDEVV;
DATMTDEVVA; ATMTDEVVAA; TMTDEVVAAT; MTDEVVAATT;
TDEVVAATTN; DEVVAATTNS; EVVAATTNSI; VVAATTNSIL;
VAATTNSILT; AATTNSILTQ; ATTNSILTQS; TTNSILTQSA;
TNSILTQSAM; NSILTQSAMA; SILTQSAMAM; ILTQSAMAMI;
LTQSAMAMIA; TQSAMAMIAQ; QSAMAMIAQA; SAMAMIAQAN;
AMAMIAQANQ; MAMIAQANQV; AMIAQANQVP; MIAQANQVPQ;
IAQANQVPQY; AQANQVPQYV; QANQVPQYVL; ANQVPQYVLS;
NQVPQYVLSL; QVPQYVLSLL; VPQYVLSLLR;

11 mers:
MIINHNTSAIN; IINHNTSAINA; INHNTSAINAS; NHNTSAINASR;
HNTSAINASRN; NTSAINASRNN; TSAINASRNNG; SAINASRNNGI;
AINASRNNGIN; INASRNNGINA; NASRNNGINAA; ASRNNGINAAN;
SRNNGINAANL; RNNGINAANLS; NNGINAANLSK; NGINAANLSKT;
GINAANLSKTQ; INAANLSKTQE; NAANLSKTQEK; AANLSKTQEKL;
ANLSKTQEKLS; NLSKTQEKLSS; LSKTQEKLSSG; SKTQEKLSSGY;
KTQEKLSSGYR; TQEKLSSGYRI; QEKLSSGYRIN; EKLSSGYRINR;
KLSSGYRINRA; LSSGYRINRAS; SSGYRINRASD; SGYRINRASDD;
GYRINRASDDA; YRINRASDDAA; RINRASDDAAG; INRASDDAAGM;
NRASDDAAGMG; RASDDAAGMGV; ASDDAAGMGVS; SDDAAGMGVSG;
DDAAGMGVSGK; DAAGMGVSGKI; AAGMGVSGKIN; AGMGVSGKINA;
GMGVSGKINAQ; MGVSGKINAQI; GVSGKINAQIR; VSGKINAQIRG;
SGKINAQIRGL; GKINAQIRGLS; KINAQIRGLSQ; INAQIRGLSQA;
NAQIRGLSQAS; AQIRGLSQASR; QIRGLSQASRN; IRGLSQASRNT;
RGLSQASRNTS; GLSQASRNTSK; LSQASRNTSKA; SQASRNTSKAI;
QASRNTSKAIN; ASRNTSKAINF; SRNTSKAINFI; RNTSKAINFIQ;
NTSKAINFIQT; TSKAINFIQTT; SKAINFIQTTE; KAINFIQTTEG;
AINFIQTTEGN; INFIQTTEGNL; NFIQTTEGNLN; FIQTTEGNLNE;
IQTTEGNLNEV; QTTEGNLNEVE; TTEGNLNEVEK; TEGNLNEVEKV;
EGNLNEVEKVL; GNLNEVEKVLV; NLNEVEKVLVR; LNEVEKVLVRM;
NEVEKVLVRMK; EVEKVLVRMKE; VEKVLVRMKEL; EKVLVRMKELA;
KVLVRMKELAV; VLVRMKELAVQ; LVRMKELAVQS; VRMKELAVQSG;
RMKELAVQSGN; MKELAVQSGNG; KELAVQSGNGT; ELAVQSGNGTY;
LAVQSGNGTYS; AVQSGNGTYSD; VQSGNGTYSDA; QSGNGTYSDAD;
SGNGTYSDADR; GNGTYSDADRG; NGTYSDADRGS; GTYSDADRGSI;
TYSDADRGSIQ; YSDADRGSIQI; SDADRGSIQIE; DADRGSIQIEI;
ADRGSIQIEIE; DRGSIQIEIEQ; RGSIQIEIEQL; GSIQIEIEQLT;
SIQIEIEQLTD; IQIEIEQLTDE; QIEIEQLTDEI; IEIEQLTDEIN;
EIEQLTDEINR; IEQLTDEINRI; EQLTDEINRIA; QLTDEINRIAD;
LTDEINRIADQ; TDEINRIADQA; DEINRIADQAQ; EINRIADQAQY;
INRIADQAQYN; NRIADQAQYNQ; RIADQAQYNQM; IADQAQYNQMH;
ADQAQYNQMHM; DQAQYNQMHML; QAQYNQMHMLS; AQYNQMHMLSN;
QYNQMHMLSNK; YNQMHMLSNKS; NQMHMLSNKSA; QMHMLSNKSAS;
MHMLSNKSASQ; HMLSNKSASQN; MLSNKSASQNV; LSNKSASQNVR;
SNKSASQNVRT; NKSASQNVRTA; KSASQNVRTAE; SASQNVRTAEE;
ASQNVRTAEEL; SQNVRTAEELG; QNVRTAEELGM; NVRTAEELGMQ;

| | |
|---|---|
| | VRTAEELGMQP; RTAEELGMQPA; TAEELGMQPAK; AEELGMQPAKI; EELGMQPAKIN; ELGMQPAKINT; LGMQPAKINTP; GMQPAKINTPA; MQPAKINTPAS; QPAKINTPASL; PAKINTPASLS; AKINTPASLSG; KINTPASLSGS; INTPASLSGSQ; NTPASLSGSQA; TPASLSGSQAS; PASLSGSQASW; ASLSGSQASWT; SLSGSQASWTL; LSGSQASWTLR; SGSQASWTLRV; GSQASWTLRVH; SQASWTLRVHV; QASWTLRVHVG; ASWTLRVHVGA; SWTLRVHVGAN; WTLRVHVGANQ; TLRVHVGANQD; LRVHVGANQDE; RVHVGANQDEA; VHVGANQDEAI; HVGANQDEAIA; VGANQDEAIAV; GANQDEAIAVN; ANQDEAIAVNI; NQDEAIAVNIY; QDEAIAVNIYA; DEAIAVNIYAA; EAIAVNIYAAN; AIAVNIYAANV; IAVNIYAANVA; AVNIYAANVAN; VNIYAANVANL; NIYAANVANLF; IYAANVANLFS; YAANVANLFSG; AANVANLFSGE; ANVANLFSGEG; NVANLFSGEGA; VANLFSGEGAQ; ANLFSGEGAQT; NLFSGEGAQTA; LFSGEGAQTAQ; FSGEGAQTAQA; SGEGAQTAQAA; GEGAQTAQAAP; EGAQTAQAAPV; GAQTAQAAPVQ; AQTAQAAPVQE; QTAQAAPVQEG; TAQAAPVQEGV; AQAAPVQEGVQ; QAAPVQEGVQQ; AAPVQEGVQQE; APVQEGVQQEG; PVQEGVQQEGA; VQEGVQQEGAQ; QEGVQQEGAQQ; EGVQQEGAQQP; GVQQEGAQQPA; VQQEGAQQPAP; QQEGAQQPAPA; QEGAQQPAPAT; EGAQQPAPATA; GAQQPAPATAP; AQQPAPATAPS; QQPAPATAPSQ; QPAPATAPSQG; PAPATAPSQGG; APATAPSQGGV; PATAPSQGGVN; ATAPSQGGVNS; TAPSQGGVNSP; APSQGGVNSPV; PSQGGVNSPVN; SQGGVNSPVNV; QGGVNSPVNVT; GGVNSPVNVTT; GVNSPVNVTTT; VNSPVNVTTTV; NSPVNVTTTVD; SPVNVTTTVDA; PVNVTTTVDAN; VNVTTTVDANT; NVTTTVDANTS; VTTTVDANTSL; TTTVDANTSLA; TTVDANTSLAK; TVDANTSLAKI; VDANTSLAKIE; DANTSLAKIEN; ANTSLAKIENA; NTSLAKIENAI; TSLAKIENAIR; SLAKIENAIRM; LAKIENAIRMI; AKIENAIRMIS; KIENAIRMISD; IENAIRMISDQ; ENAIRMISDQR; NAIRMISDQRA; AIRMISDQRAN; IRMISDQRANL; RMISDQRANLG; MISDQRANLGA; ISDQRANLGAF; SDQRANLGAFQ; DQRANLGAFQN; QRANLGAFQNR; RANLGAFQNRL; ANLGAFQNRLE; NLGAFQNRLES; LGAFQNRLESI; GAFQNRLESIK; AFQNRLESIKN; FQNRLESIKNS; QNRLESIKNST; NRLESIKNSTE; RLESIKNSTEY; LESIKNSTEYA; ESIKNSTEYAI; SIKNSTEYAIE; IKNSTEYAIEN; KNSTEYAIENL; NSTEYAIENLK; STEYAIENLKA; TEYAIENLKAS; EYAIENLKASY; YAIENLKASYA; AIENLKASYAQ; IENLKASYAQI; ENLKASYAQIK; NLKASYAQIKD; LKASYAQIKDA; KASYAQIKDAT; ASYAQIKDATM; SYAQIKDATMT; YAQIKDATMTD; AQIKDATMTDE; QIKDATMTDEV; IKDATMTDEVV; KDATMTDEVVA; DATMTDEVVAA; ATMTDEVVAAT; TMTDEVVAATT; MTDEVVAATTN; TDEVVAATTNS; DEVVAATTNSI; EVVAATTNSIL; VVAATTNSILT; VAATTNSILTQ; AATTNSILTQS; ATTNSILTQSA; TTNSILTQSAM; TNSILTQSAMA; NSILTQSAMAM; SILTQSAMAMI; ILTQSAMAMIA; LTQSAMAMIAQ; TQSAMAMIAQA; QSAMAMIAQAN; SAMAMIAQANQ; AMAMIAQANQV; MAMIAQANQVP; AMIAQANQVPQ; MIAQANQVPQY; IAQANQVPQYV; AQANQVPQYVL; QANQVPQYVLS; ANQVPQYVLSL; NQVPQYVLSLL; QVPQYVLSLLR; |
| Seq 24 | SEQ ID NO 25784-27861/ 8 mers: MKLQRSLF; KLQRSLFL; LQRSLFLI; QRSLFLII; RSLFLIIF; SLFLIIFF; LFLIIFFL; FLIIFFLT; LIIFFLTF; IIFFLTFL; |

EP 2 254 592 B1

| | IFFLTFLC; FFLTFLCC; FLTFLCCN; LTFLCCNN; TFLCCNNK; FLCCNNKE; LCCNNKER; CCNNKERK; CNNKERKE; NNKERKEG; NKERKEGV; KERKEGVS; ERKEGVSF; RKEGVSFK; KEGVSFKI; EGVSFKIS; GVSFKISL; VSFKISLG; SFKISLGA; FKISLGAE; KISLGAEP; ISLGAEPS; SLGAEPSS; LGAEPSSL; GAEPSSLD; AEPSSLDP; EPSSLDPQ; PSSLDPQL; SSLDPQLA; SLDPQLAE; LDPQLAED; DPQLAEDN; PQLAEDNV; QLAEDNVA; LAEDNVAS; AEDNVASK; EDNVASKM; DNVASKMI; NVASKMID; VASKMIDT; ASKMIDTM; SKMIDTMF; KMIDTMFR; MIDTMFRG; IDTMFRGI; DTMFRGIV; TMFRGIVT; MFRGIVTG; FRGIVTGD; RGIVTGDP; GIVTGDPN; IVTGDPNT; VTGDPNTG; TGDPNTGG; GDPNTGGN; DPNTGGNK; PNTGGNKP; NTGGNKPG; TGGNKPGL; GGNKPGLA; GNKPGLAK; NKPGLAKG; KPGLAKGW; PGLAKGWD; GLAKGWDI; LAKGWDIS; AKGWDISS; KGWDISSD; GWDISSDG; WDISSDGT; DISSDGTV; ISSDGTVY; SSDGTVYT; SDGTVYTF; DGTVYTFN; GTVYTFNL; TVYTFNLR; VYTFNLRE; YTFNLREK; TFNLREKI; FNLREKIT; NLREKITW; LREKITWS; REKITWSD; EKITWSDG; KITWSDGV; ITWSDGVA; TWSDGVAI; WSDGVAIT; SDGVAITA; DGVAITAE; GVAITAEG; VAITAEGI; AITAEGIR; ITAEGIRK; TAEGIRKS; AEGIRKSY; EGIRKSYL; GIRKSYLR; IRKSYLRI; RKSYLRIL; KSYLRILN; SYLRILNK; YLRILNKE; LRILNKET; RILNKETG; ILNKETGS; LNKETGSK; NKETGSKY; KETGSKYV; ETGSKYVE; TGSKYVEM; GSKYVEMV; SKYVEMVK; KYVEMVKS; YVEMVKSV; VEMVKSVI; EMVKSVIK; MVKSVIKN; VKSVIKNG; KSVIKNGQ; SVIKNGQK; VIKNGQKY; IKNGQKYF; KNGQKYFD; NGQKYFDG; GQKYFDGQ; QKYFDGQV; KYFDGQVT; YFDGQVTD; FDGQVTDS; DGQVTDSE; GQVTDSEL; QVTDSELG; VTDSELGI; TDSELGIR; DSELGIRA; SELGIRAI; ELGIRAID; LGIRAIDE; GIRAIDEK; IRAIDEKT; RAIDEKTL; AIDEKTLE; IDEKTLEI; DEKTLEIT; EKTLEITL; KTLEITLE; TLEITLES; LEITLESP; EITLESPK; ITLESPKP; TLESPKPY; LESPKPYF; ESPKPYFI; SPKPYFID; PKPYFIDM; KPYFIDML; PYFIDMLV; YFIDMLVH; FIDMLVHQ; IDMLVHQS; DMLVHQSF; MLVHQSFI; LVHQSFIP; VHQSFIPV; HQSFIPVP; QSFIPVPV; SFIPVPVH; FIPVPVHV; IPVPVHVT; PVPHVTE; VPVHVTEK; PVHVTEKY; VHVTEKYG; HVTEKYGQ; VTEKYGQN; TEKYGQNW; EKYGQNWT; KYGQNWTS; YGQNWTSP; GQNWTSPE; QNWTSPEN; NWTSPENM; WTSPENMV; TSPENMVT; SPENMVTS; PENMVTSG; ENMVTSGP; NMVTSGPF; MVTSGPFK; VTSGPFKL; TSGPFKLK; SGPFKLKE; GPFKLKER; PFKLKERI; FKLKERIP; KLKERIPN; LKERIPNE; KERIPNEK; ERIPNEKY; RIPNEKYV; IPNEKYVF; PNEKYVFE; NEKYVFEK; EKYVFEKN; KYVFEKNN; YVFEKNNK; VFEKNNKY; FEKNNKYY; EKNNKYYD; KNNKYYDS; NNKYYDSN; NKYYDSNE; KYYDSNEV; YYDSNEVE; YDSNEVEL; DSNEVELE; SNEVELEE; NEVELEEI; EVELEEIT; VELEEITF; ELEEITFY; LEEITFYT; EEITFYTT; EITFYTTN; ITFYTTND; TFYTTNDS; FYTTNDSS; YTTNDSST; TTNDSSTA; TNDSSTAY; NDSSTAYK; DSSTAYKM; SSTAYKMY; STAYKMYE; TAYKMYEN; AYKMYENE; YKMYENEE; KMYENEEL; MYENEELD; YENEELDA; ENEELDAI; NEELDAIF; EELDAIFG; ELDAIFGS; LDAIFGSI; DAIFGSIP; AIFGSIPP; IFGSIPPD; FGSIPPDL; GSIPPDLI; SIPPDLIK; IPPDLIKN; PPDLIKNL; |

| |
|---|
| PDLIKNLK; DLIKNLKL; LIKNLKLR; IKNLKLRS; KNLKLRSD; NLKLRSDY; LKLRSDYY; KLRSDYYS; LRSDYYSS; RSDYYSSA; SDYYSSAV; DYYSSAVN; YYSSAVNA; YSSAVNAI; SSAVNAIY; SAVNAIYF; AVNAIYFY; VNAIYFYA; NAIYFYAF; AIYFYAFN; IYFYAFNT; YFYAFNTH; FYAFNTHI; YAFNTHIK; AFNTHIKP; FNTHIKPL; NTHIKPLD; THIKPLDN; HIKPLDNV; IKPLDNVK; KPLDNVKI; PLDNVKIR; LDNVKIRK; DNVKIRKA; NVKIRKAL; VKIRKALT; KIRKALTL; IRKALTLA; RKALTLAI; KALTLAID; ALTLAIDR; LTLAIDRE; TLAIDRET; LAIDRETL; AIDRETLT; IDRETLTY; DRETLTYK; RETLTYKV; ETLTYKVL; TLTYKVLD; LTYKVLDN; TYKVLDNG; YKVLDNGT; KVLDNGTT; VLDNGTTP; LDNGTTPT; DNGTTPTR; NGTTPTRR; GTTPTRRA; TTPTRRAT; TPTRRATP; PTRRATPN; TRRATPNF; RRATPNFS; RATPNFSS; ATPNFSSY; TPNFSSYS; PNFSSYSY; NFSSYSYA; FSSYSYAK; SSYSYAKS; SYSYAKSL; YSYAKSLE; SYAKSLEL; YAKSLELF; AKSLELFN; KSLELFNP; SLELFNPE; LELFNPEI; ELFNPEIA; LFNPEIAK; FNPEIAKT; NPEIAKTL; PEIAKTLL; EIAKTLLA; IAKTLLAE; AKTLLAEA; KTLLAEAG; TLLAEAGY; LLAEAGYP; LAEAGYPN; AEAGYPNG; EAGYPNGN; AGYPNGNG; GYPNGNGF; YPNGNGFP; PNGNGFPI; NGNGFPIL; GNGFPILK; NGFPILKL; GFPILKLK; FPILKLKY; PILKLKYN; ILKLKYNT; LKLKYNTN; KLKYNTNE; LKYNTNEA; KYNTNEAN; YNTNEANK; NTNEANKK; TNEANKKI; NEANKKIC; EANKKICE; ANKKICEF; NKKICEFI; KKICEFIQ; KICEFIQN; ICEFIQNQ; CEFIQNQW; EFIQNQWK; FIQNQWKK; IQNQWKKN; QNQWKKNL; NQWKKNLN; QWKKNLNI; WKKNLNID; KKNLNIDV; KNLNIDVE; NLNIDVEL; LNIDVELE; NIDVELEN; IDVELENE; DVELENEE; VELENEEW; ELENEEWT; LENEEWTT; ENEEWTTY; NEEWTTYL; EEWTTYLN; EWTTYLNT; WTTYLNTK; TTYLNTKA; TYLNTKAN; YLNTKANG; LNTKANGN; NTKANGNY; TKANGNYE; KANGNYEI; ANGNYEIA; NGNYEIAR; GNYEIARA; NYEIARAG; YEIARAGW; EIARAGWI; IARAGWIG; ARAGWIGD; RAGWIGDY; AGWIGDYA; GWIGDYAD; WIGDYADP; IGDYADPL; GDYADPLT; DYADPLTF; YADPLTFL; ADPLTFLS; DPLTFLSI; PLTFLSIF; LTFLSIFT; TFLSIFTQ; FLSIFTQG; LSIFTQGY; SIFTQGYT; IFTQGYTQ; FTQGYTQF; TQGYTQFS; QGYTQFSS; GYTQFSSH; YTQFSSHN; TQFSSHNY; QFSSHNYS; FSSHNYSN; SSHNYSNP; SHNYSNPE; HNYSNPEY; NYSNPEYN; YSNPEYNE; SNPEYNEL; NPEYNELI; PEYNELIK; EYNELIKK; YNELIKKS; NELIKKSD; ELIKKSDL; LIKKSDLE; IKKSDLEL; KKSDLELD; KSDLELDP; SDLELDPI; DLELDPIK; LELDPIKR; ELDPIKRQ; LDPIKRQD; DPIKRQDI; PIKRQDIL; IKRQDILR; KRQDILRQ; RQDILRQA; QDILRQAE; DILRQAEE; ILRQAEEI; LRQAEEII; RQAEEIII; QAEEIIIE; AEEIIIEK; EEIIIEKD; EIIIEKDF; IIIEKDFP; IIEKDFPI; IEKDFPIA; EKDFPIAP; KDFPIAPI; DFPIAPIY; FPIAPIYI; PIAPIYIY; IAPIYIYG; APIYIYGN; PIYIYGNS; IYIYGNSY; YIYGNSYL; IYGNSYLF; YGNSYLFR; GNSYLFRN; NSYLFRND; SYLFRNDK; YLFRNDKW; LFRNDKWT; FRNDKWTG; RNDKWTGW; NDKWTGWN; DKWTGWNT; KWTGWNTN; WTGWNTNI; TGWNTNIL; GWNTNILE; WNTNILER; NTNILERF; TNILERFD; NILERFDL; ILERFDLS; LERFDLSQ; ERFDLSQL; RFDLSQLK; FDLSQLKL; DLSQLKLK; LSQLKLKN; |

SQLKLKNK;

9 mers:

| | | | | |
|---|---|---|---|---|
| MKLQRSLFL; | KLQRSLFLI; | LQRSLFLII; | QRSLFLIIF; | RSLFLIIFF; |
| SLFLIIFFL; | LFLIIFFLT; | FLIIFFLTF; | LIIFFLTFL; | IIFFLTFLC; |
| IFFLTFLCC; | FFLTFLCCN; | FLTFLCCNN; | LTFLCCNNK; | TFLCCNNKE; |
| FLCCNNKER; | LCCNNKERK; | CCNNKERKE; | CNNKERKEG; | NNKERKEGV; |
| NKERKEGVS; | KERKEGVSF; | ERKEGVSFK; | RKEGVSFKI; | KEGVSFKIS; |
| EGVSFKISL; | GVSFKISLG; | VSFKISLGA; | SFKISLGAE; | FKISLGAEP; |
| KISLGAEPS; | ISLGAEPSS; | SLGAEPSSL; | LGAEPSSLD; | GAEPSSLDP; |
| AEPSSLDPQ; | EPSSLDPQL; | PSSLDPQLA; | SSLDPQLAE; | SLDPQLAED; |
| LDPQLAEDN; | DPQLAEDNV; | PQLAEDNVA; | QLAEDNVAS; | LAEDNVASK; |
| AEDNVASKM; | EDNVASKMI; | DNVASKMID; | NVASKMIDT; | VASKMIDTM; |
| ASKMIDTMF; | SKMIDTMFR; | KMIDTMFRG; | MIDTMFRGI; | IDTMFRGIV; |
| DTMFRGIVT; | TMFRGIVTG; | MFRGIVTGD; | FRGIVTGDP; | RGIVTGDPN; |
| GIVTGDPNT; | IVTGDPNTG; | VTGDPNTGG; | TGDPNTGGN; | GDPNTGGNK; |
| DPNTGGNKP; | PNTGGNKPG; | NTGGNKPGL; | TGGNKPGLA; | GGNKPGLAK; |
| GNKPGLAKG; | NKPGLAKGW; | KPGLAKGWD; | PGLAKGWDI; | GLAKGWDIS; |
| LAKGWDISS; | AKGWDISSD; | KGWDISSDG; | GWDISSDGT; | WDISSDGTV; |
| DISSDGTVY; | ISSDGTVYT; | SSDGTVYTF; | SDGTVYTFN; | DGTVYTFNL; |
| GTVYTFNLR; | TVYTFNLRE; | VYTFNLREK; | YTFNLREKI; | TFNLREKIT; |
| FNLREKITW; | NLREKITWS; | LREKITWSD; | REKITWSDG; | EKITWSDGV; |
| KITWSDGVA; | ITWSDGVAI; | TWSDGVAIT; | WSDGVAITA; | SDGVAITAE; |
| DGVAITAEG; | GVAITAEGI; | VAITAEGIR; | AITAEGIRK; | ITAEGIRKS; |
| TAEGIRKSY; | AEGIRKSYL; | EGIRKSYLR; | GIRKSYLRI; | IRKSYLRIL; |
| RKSYLRILN; | KSYLRILNK; | SYLRILNKE; | YLRILNKET; | LRILNKETG; |
| RILNKETGS; | ILNKETGSK; | LNKETGSKY; | NKETGSKYV; | KETGSKYVE; |
| ETGSKYVEM; | TGSKYVEMV; | GSKYVEMVK; | SKYVEMVKS; | KYVEMVKSV; |
| YVEMVKSVI; | VEMVKSVIK; | EMVKSVIKN; | MVKSVIKNG; | VKSVIKNGQ; |
| KSVIKNGQK; | SVIKNGQKY; | VIKNGQKYF; | IKNGQKYFD; | KNGQKYFDG; |
| NGQKYFDGQ; | GQKYFDGQV; | QKYFDGQVT; | KYFDGQVTD; | YFDGQVTDS; |
| FDGQVTDSE; | DGQVTDSEL; | GQVTDSELG; | QVTDSELGI; | VTDSELGIR; |
| TDSELGIRA; | DSELGIRAI; | SELGIRAID; | ELGIRAIDE; | LGIRAIDEK; |
| GIRAIDEKT; | IRAIDEKTL; | RAIDEKTLE; | AIDEKTLEI; | IDEKTLEIT; |
| DEKTLEITL; | EKTLEITLE; | KTLEITLES; | TLEITLESP; | LEITLESPK; |
| EITLESPKP; | ITLESPKPY; | TLESPKPYF; | LESPKPYFI; | ESPKPYFID; |
| SPKPYFIDM; | PKPYFIDML; | KPYFIDMLV; | PYFIDMLVH; | YFIDMLVHQ; |
| FIDMLVHQS; | IDMLVHQSF; | DMLVHQSFI; | MLVHQSFIP; | LVHQSFIPV; |
| VHQSFIPVP; | HQSFIPVPV; | QSFIPVPVH; | SFIPVPVHV; | FIPVPVHVT; |
| IPVPVHVTE; | PVPVHVTEK; | VPVHVTEKY; | PVHVTEKYG; | VHVTEKYGQ; |
| HVTEKYGQN; | VTEKYGQNW; | TEKYGQNWT; | EKYGQNWTS; | KYGQNWTSP; |
| YGQNWTSPE; | GQNWTSPEN; | QNWTSPENM; | NWTSPENMV; | WTSPENMVT; |
| TSPENMVTS; | SPENMVTSG; | PENMVTSGP; | ENMVTSGPF; | NMVTSGPFK; |
| MVTSGPFKL; | VTSGPFKLK; | TSGPFKLKE; | SGPFKLKER; | GPFKLKERI; |
| PFKLKERIP; | FKLKERIPN; | KLKERIPNE; | LKERIPNEK; | KERIPNEKY; |
| ERIPNEKYV; | RIPNEKYVF; | IPNEKYVFE; | PNEKYVFEK; | NEKYVFEKN; |
| EKYVFEKNN; | KYVFEKNNK; | YVFEKNNKY; | VFEKNNKYY; | FEKNNKYYD; |
| EKNNKYYDS; | KNNKYYDSN; | NNKYYDSNE; | NKYYDSNEV; | KYYDSNEVE; |
| YYDSNEVEL; | YDSNEVELE; | DSNEVELEE; | SNEVELEEI; | NEVELEEIT; |
| EVELEEITF; | VELEEITFY; | ELEEITFYT; | LEEITFYTT; | EEITFYTTN; |
| EITFYTTND; | ITFYTTNDS; | TFYTTNDSS; | FYTTNDSST; | YTTNDSSTA; |

| | | | | |
|---|---|---|---|---|
| TTNDSSTAY; | TNDSSTAYK; | NDSSTAYKM; | DSSTAYKMY; | SSTAYKMYE; |
| STAYKMYEN; | TAYKMYENE; | AYKMYENEE; | YKMYENEEL; | KMYENEELD; |
| MYENEELDA; | YENEELDAI; | ENEELDAIF; | NEELDAIFG; | EELDAIFGS; |
| ELDAIFGSI; | LDAIFGSIP; | DAIFGSIPP; | AIFGSIPPD; | IFGSIPPDL; |
| FGSIPPDLI; | GSIPPDLIK; | SIPPDLIKN; | IPPDLIKNL; | PPDLIKNLK; |
| PDLIKNLKL; | DLIKNLKLR; | LIKNLKLRS; | IKNLKLRSD; | KNLKLRSDY; |
| NLKLRSDYY; | LKLRSDYYS; | KLRSDYYSS; | LRSDYYSSA; | RSDYYSSAV; |
| SDYYSSAVN; | DYYSSAVNA; | YYSSAVNAI; | YSSAVNAIY; | SSAVNAIYF; |
| SAVNAIYFY; | AVNAIYFYA; | VNAIYFYAF; | NAIYFYAFN; | AIYFYAFNT; |
| IYFYAFNTH; | YFYAFNTHI; | FYAFNTHIK; | YAFNTHIKP; | AFNTHIKPL; |
| FNTHIKPLD; | NTHIKPLDN; | THIKPLDNV; | HIKPLDNVK; | IKPLDNVKI; |
| KPLDNVKIR; | PLDNVKIRK; | LDNVKIRKA; | DNVKIRKAL; | NVKIRKALT; |
| VKIRKALTL; | KIRKALTLA; | IRKALTLAI; | RKALTLAID; | KALTLAIDR; |
| ALTLAIDRE; | LTLAIDRET; | TLAIDRETL; | LAIDRETLT; | AIDRETLTY; |
| IDRETLTYK; | DRETLTYKV; | RETLTYKVL; | ETLTYKVLD; | TLTYKVLDN; |
| LTYKVLDNG; | TYKVLDNGT; | YKVLDNGTT; | KVLDNGTTP; | VLDNGTTPT; |
| LDNGTTPTR; | DNGTTPTRR; | NGTTPTRRA; | GTTPTRRAT; | TTPTRRATP; |
| TPTRRATPN; | PTRRATPNF; | TRRATPNFS; | RRATPNFSS; | RATPNFSSY; |
| ATPNFSSYS; | TPNFSSYSY; | PNFSSYSYA; | NFSSYSYAK; | FSSYSYAKS; |
| SSYSYAKSL; | SYSYAKSLE; | YSYAKSLEL; | SYAKSLELF; | YAKSLELFN; |
| AKSLELFNP; | KSLELFNPE; | SLELFNPEI; | LELFNPEIA; | ELFNPEIAK; |
| LFNPEIAKT; | FNPEIAKTL; | NPEIAKTLL; | PEIAKTLLA; | EIAKTLLAE; |
| IAKTLLAEA; | AKTLLAEAG; | KTLLAEAGY; | TLLAEAGYP; | LLAEAGYPN; |
| LAEAGYPNG; | AEAGYPNGN; | EAGYPNGNG; | AGYPNGNGF; | GYPNGNGFP; |
| YPNGNGFPI; | PNGNGFPIL; | NGNGFPILK; | GNGFPILKL; | NGFPILKLK; |
| GFPILKLKY; | FPILKLKYN; | PILKLKYNT; | ILKLKYNTN; | LKLKYNTNE; |
| KLKYNTNEA; | LKYNTNEAN; | KYNTNEANK; | YNTNEANKK; | NTNEANKKI; |
| TNEANKKIC; | NEANKKICE; | EANKKICEF; | ANKKICEFI; | NKKICEFIQ; |
| KKICEFIQN; | KICEFIQNQ; | ICEFIQNQW; | CEFIQNQWK; | EFIQNQWKK; |
| FIQNQWKKN; | IQNQWKKNL; | QNQWKKNLN; | NQWKKNLNI; | QWKKNLNID; |
| WKKNLNIDV; | KKNLNIDVE; | KNLNIDVEL; | NLNIDVELE; | LNIDVELEN; |
| NIDVELENE; | IDVELENEE; | DVELENEEW; | VELENEEWT; | ELENEEWTT; |
| LENEEWTTY; | ENEEWTTYL; | NEEWTTYLN; | EEWTTYLNT; | EWTTYLNTK; |
| WTTYLNTKA; | TTYLNTKAN; | TYLNTKANG; | YLNTKANGN; | LNTKANGNY; |
| NTKANGNYE; | TKANGNYEI; | KANGNYEIA; | ANGNYEIAR; | NGNYEIARA; |
| GNYEIARAG; | NYEIARAGW; | YEIARAGWI; | EIARAGWIG; | IARAGWIGD; |
| ARAGWIGDY; | RAGWIGDYA; | AGWIGDYAD; | GWIGDYADP; | WIGDYADPL; |
| IGDYADPLT; | GDYADPLTF; | DYADPLTFL; | YADPLTFLS; | ADPLTFLSI; |
| DPLTFLSIF; | PLTFLSIFT; | LTFLSIFTQ; | TFLSIFTQG; | FLSIFTQGY; |
| LSIFTQGYT; | SIFTQGYTQ; | IFTQGYTQF; | FTQGYTQFS; | TQGYTQFSS; |
| QGYTQFSSH; | GYTQFSSHN; | YTQFSSHNY; | TQFSSHNYS; | QFSSHNYSN; |
| FSSHNYSNP; | SSHNYSNPE; | SHNYSNPEY; | HNYSNPEYN; | NYSNPEYNE; |
| YSNPEYNEL; | SNPEYNELI; | NPEYNELIK; | PEYNELIKK; | EYNELIKKS; |
| YNELIKKSD; | NELIKKSDL; | ELIKKSDLE; | LIKKSDLEL; | IKKSDLELD; |
| KKSDLELDP; | KSDLELDPI; | SDLELDPIK; | DLELDPIKR; | LELDPIKRQ; |
| ELDPIKRQD; | LDPIKRQDI; | DPIKRQDIL; | PIKRQDILR; | IKRQDILRQ; |
| KRQDILRQA; | RQDILRQAE; | QDILRQAEE; | DILRQAEEI; | ILRQAEEII; |
| LRQAEEIII; | RQAEEIIIE; | QAEEIIIEK; | AEEIIIEKD; | EEIIIEKDF; |
| EIIIEKDFP; | IIIEKDFPI; | IIEKDFPIA; | IEKDFPIAP; | EKDFPIAPI; |
| KDFPIAPIY; | DFPIAPIYI; | FPIAPIYIY; | PIAPIYIYG; | IAPIYIYGN; |
| APIYIYGNS; | PIYIYGNSY; | IYIYGNSYL; | YIYGNSYLF; | IYGNSYLFR; |

EP 2 254 592 B1

YGNSYLFRN; GNSYLFRND; NSYLFRNDK; SYLFRNDKW; YLFRNDKWT;
LFRNDKWTG; FRNDKWTGW; RNDKWTGWN; NDKWTGWNT; DKWTGWNTN;
KWTGWNTNI; WTGWNTNIL; TGWNTNILE; GWNTNILER; WNTNILERF;
NTNILERFD; TNILERFDL; NILERFDLS; ILERFDLSQ; LERFDLSQL;
ERFDLSQLK; RFDLSQLKL; FDLSQLKLK; DLSQLKLKN; LSQLKLKNK;

10 mers:
MKLQRSLFLI; KLQRSLFLII; LQRSLFLIIF; QRSLFLIIFF;
RSLFLIIFFL; SLFLIIFFLT; LFLIIFFLTF; FLIIFFLTFL;
LIIFFLTFLC; IIFFLTFLCC; IFFLTFLCCN; FFLTFLCCNN;
FLTFLCCNNK; LTFLCCNNKE; TFLCCNNKER; FLCCNNKERK;
LCCNNKERKE; CCNNKERKEG; CNNKERKEGV; NNKERKEGVS;
NKERKEGVSF; KERKEGVSFK; ERKEGVSFKI; RKEGVSFKIS;
KEGVSFKISL; EGVSFKISLG; GVSFKISLGA; VSFKISLGAE;
SFKISLGAEP; FKISLGAEPS; KISLGAEPSS; ISLGAEPSSL;
SLGAEPSSLD; LGAEPSSLDP; GAEPSSLDPQ; AEPSSLDPQL;
EPSSLDPQLA; PSSLDPQLAE; SSLDPQLAED; SLDPQLAEDN;
LDPQLAEDNV; DPQLAEDNVA; PQLAEDNVAS; QLAEDNVASK;
LAEDNVASKM; AEDNVASKMI; EDNVASKMID; DNVASKMIDT;
NVASKMIDTM; VASKMIDTMF; ASKMIDTMFR; SKMIDTMFRG;
KMIDTMFRGI; MIDTMFRGIV; IDTMFRGIVT; DTMFRGIVTG;
TMFRGIVTGD; MFRGIVTGDP; FRGIVTGDPN; RGIVTGDPNT;
GIVTGDPNTG; IVTGDPNTGG; VTGDPNTGGN; TGDPNTGGNK;
GDPNTGGNKP; DPNTGGNKPG; PNTGGNKPGL; NTGGNKPGLA;
TGGNKPGLAK; GGNKPGLAKG; GNKPGLAKGW; NKPGLAKGWD;
KPGLAKGWDI; PGLAKGWDIS; GLAKGWDISS; LAKGWDISSD;
AKGWDISSDG; KGWDISSDGT; GWDISSDGTV; WDISSDGTVY;
DISSDGTVYT; ISSDGTVYTF; SSDGTVYTFN; SDGTVYTFNL;
DGTVYTFNLR; GTVYTFNLRE; TVYTFNLREK; VYTFNLREKI;
YTFNLREKIT; TFNLREKITW; FNLREKITWS; NLREKITWSD;
LREKITWSDG; REKITWSDGV; EKITWSDGVA; KITWSDGVAI;
ITWSDGVAIT; TWSDGVAITA; WSDGVAITAE; SDGVAITAEG;
DGVAITAEGI; GVAITAEGIR; VAITAEGIRK; AITAEGIRKS;
ITAEGIRKSY; TAEGIRKSYL; AEGIRKSYLR; EGIRKSYLRI;
GIRKSYLRIL; IRKSYLRILN; RKSYLRILNK; KSYLRILNKE;
SYLRILNKET; YLRILNKETG; LRILNKETGS; RILNKETGSK;
ILNKETGSKY; LNKETGSKYV; NKETGSKYVE; KETGSKYVEM;
ETGSKYVEMV; TGSKYVEMVK; GSKYVEMVKS; SKYVEMVKSV;
KYVEMVKSVI; YVEMVKSVIK; VEMVKSVIKN; EMVKSVIKNG;
MVKSVIKNGQ; VKSVIKNGQK; KSVIKNGQKY; SVIKNGQKYF;
VIKNGQKYFD; IKNGQKYFDG; KNGQKYFDGQ; NGQKYFDGQV;
GQKYFDGQVT; QKYFDGQVTD; KYFDGQVTDS; YFDGQVTDSE;
FDGQVTDSEL; DGQVTDSELG; GQVTDSELGI; QVTDSELGIR;
VTDSELGIRA; TDSELGIRAI; DSELGIRAID; SELGIRAIDE;
ELGIRAIDEK; LGIRAIDEKT; GIRAIDEKTL; IRAIDEKTLE;
RAIDEKTLEI; AIDEKTLEIT; IDEKTLEITL; DEKTLEITLE;
EKTLEITLES; KTLEITLESP; TLEITLESPK; LEITLESPKP;
EITLESPKPY; ITLESPKPYF; TLESPKPYFI; LESPKPYFID;
ESPKPYFIDM; SPKPYFIDML; PKPYFIDMLV; KPYFIDMLVH;
PYFIDMLVHQ; YFIDMLVHQS; FIDMLVHQSF; IDMLVHQSFI;
DMLVHQSFIP; MLVHQSFIPV; LVHQSFIPVP; VHQSFIPVPV;

700

HQSFIPVPVH; QSFIPVPVHV; SFIPVPVHVT; FIPVPVHVTE;
IPVPVHVTEK; PVPVHVTEKY; VPVHVTEKYG; PVHVTEKYGQ;
VHVTEKYGQN; HVTEKYGQNW; VTEKYGQNWT; TEKYGQNWTS;
EKYGQNWTSP; KYGQNWTSPE; YGQNWTSPEN; GQNWTSPENM;
QNWTSPENMV; NWTSPENMVT; WTSPENMVTS; TSPENMVTSG;
SPENMVTSGP; PENMVTSGPF; ENMVTSGPFK; NMVTSGPFKL;
MVTSGPFKLK; VTSGPFKLKE; TSGPFKLKER; SGPFKLKERI;
GPFKLKERIP; PFKLKERIPN; FKLKERIPNE; KLKERIPNEK;
LKERIPNEKY; KERIPNEKYV; ERIPNEKYVF; RIPNEKYVFE;
IPNEKYVFEK; PNEKYVFEKN; NEKYVFEKNN; EKYVFEKNNK;
KYVFEKNNKY; YVFEKNNKYY; VFEKNNKYYD; FEKNNKYYDS;
EKNNKYYDSN; KNNKYYDSNE; NNKYYDSNEV; NKYYDSNEVE;
KYYDSNEVEL; YYDSNEVELE; YDSNEVELEE; DSNEVELEEI;
SNEVELEEIT; NEVELEEITF; EVELEEITFY; VELEEITFYT;
ELEEITFYTT; LEEITFYTTN; EEITFYTTND; EITFYTTNDS;
ITFYTTNDSS; TFYTTNDSST; FYTTNDSSTA; YTTNDSSTAY;
TTNDSSTAYK; TNDSSTAYKM; NDSSTAYKMY; DSSTAYKMYE;
SSTAYKMYEN; STAYKMYENE; TAYKMYENEE; AYKMYENEEL;
YKMYENEELD; KMYENEELDA; MYENEELDAI; YENEELDAIF;
ENEELDAIFG; NEELDAIFGS; EELDAIFGSI; ELDAIFGSIP;
LDAIFGSIPP; DAIFGSIPPD; AIFGSIPPDL; IFGSIPPDLI;
FGSIPPDLIK; GSIPPDLIKN; SIPPDLIKNL; IPPDLIKNLK;
PPDLIKNLKL; PDLIKNLKLR; DLIKNLKLRS; LIKNLKLRSD;
IKNLKLRSDY; KNLKLRSDYY; NLKLRSDYYS; LKLRSDYYSS;
KLRSDYYSSA; LRSDYYSSAV; RSDYYSSAVN; SDYYSSAVNA;
DYYSSAVNAI; YYSSAVNAIY; YSSAVNAIYF; SSAVNAIYFY;
SAVNAIYFYA; AVNAIYFYAF; VNAIYFYAFN; NAIYFYAFNT;
AIYFYAFNTH; IYFYAFNTHI; YFYAFNTHIK; FYAFNTHIKP;
YAFNTHIKPL; AFNTHIKPLD; FNTHIKPLDN; NTHIKPLDNV;
THIKPLDNVK; HIKPLDNVKI; IKPLDNVKIR; KPLDNVKIRK;
PLDNVKIRKA; LDNVKIRKAL; DNVKIRKALT; NVKIRKALTL;
VKIRKALTLA; KIRKALTLAI; IRKALTLAID; RKALTLAIDR;
KALTLAIDRE; ALTLAIDRET; LTLAIDRETL; TLAIDRETLT;
LAIDRETLTY; AIDRETLTYK; IDRETLTYKV; DRETLTYKVL;
RETLTYKVLD; ETLTYKVLDN; TLTYKVLDNG; LTYKVLDNGT;
TYKVLDNGTT; YKVLDNGTTP; KVLDNGTTPT; VLDNGTTPTR;
LDNGTTPTRR; DNGTTPTRRA; NGTTPTRRAT; GTTPTRRATP;
TTPTRRATPN; TPTRRATPNF; PTRRATPNFS; TRRATPNFSS;
RRATPNFSSY; RATPNFSSYS; ATPNFSSYSY; TPNFSSYSYA;
PNFSSYSYAK; NFSSYSYAKS; FSSYSYAKSL; SSYSYAKSLE;
SYSYAKSLEL; YSYAKSLELF; SYAKSLELFN; YAKSLELFNP;
AKSLELFNPE; KSLELFNPEI; SLELFNPEIA; LELFNPEIAK;
ELFNPEIAKT; LFNPEIAKTL; FNPEIAKTLL; NPEIAKTLLA;
PEIAKTLLAE; EIAKTLLAEA; IAKTLLAEAG; AKTLLAEAGY;
KTLLAEAGYP; TLLAEAGYPN; LLAEAGYPNG; LAEAGYPNGN;
AEAGYPNGNG; EAGYPNGNGF; AGYPNGNGFP; GYPNGNGFPI;
YPNGNGFPIL; PNGNGFPILK; NGNGFPILKL; GNGFPILKLK;
NGFPILKLKY; GFPILKLKYN; FPILKLKYNT; PILKLKYNTN;
ILKLKYNTNE; LKLKYNTNEA; KLKYNTNEAN; LKYNTNEANK;
KYNTNEANKK; YNTNEANKKI; NTNEANKKIC; TNEANKKICE;
NEANKKICEF; EANKKICEFI; ANKKICEFIQ; NKKICEFIQN;

701

KKICEFIQNQ; KICEFIQNQW; ICEFIQNQWK; CEFIQNQWKK;
EFIQNQWKKN; FIQNQWKKNL; IQNQWKKNLN; QNQWKKNLNI;
NQWKKNLNID; QWKKNLNIDV; WKKNLNIDVE; KKNLNIDVEL;
KNLNIDVELE; NLNIDVELEN; LNIDVELENE; NIDVELENEE;
IDVELENEEW; DVELENEEWT; VELENEEWTT; ELENEEWTTY;
LENEEWTTYL; ENEEWTTYLN; NEEWTTYLNT; EEWTTYLNTK;
EWTTYLNTKA; WTTYLNTKAN; TTYLNTKANG; TYLNTKANGN;
YLNTKANGNY; LNTKANGNYE; NTKANGNYEI; TKANGNYEIA;
KANGNYEIAR; ANGNYEIARA; NGNYEIARAG; GNYEIARAGW;
NYEIARAGWI; YEIARAGWIG; EIARAGWIGD; IARAGWIGDY;
ARAGWIGDYA; RAGWIGDYAD; AGWIGDYADP; GWIGDYADPL;
WIGDYADPLT; IGDYADPLTF; GDYADPLTFL; DYADPLTFLS;
YADPLTFLSI; ADPLTFLSIF; DPLTFLSIFT; PLTFLSIFTQ;
LTFLSIFTQG; TFLSIFTQGY; FLSIFTQGYT; LSIFTQGYTQ;
SIFTQGYTQF; IFTQGYTQFS; FTQGYTQFSS; TQGYTQFSSH;
QGYTQFSSHN; GYTQFSSHNY; YTQFSSHNYS; TQFSSHNYSN;
QFSSHNYSNP; FSSHNYSNPE; SSHNYSNPEY; SHNYSNPEYN;
HNYSNPEYNE; NYSNPEYNEL; YSNPEYNELI; SNPEYNELIK;
NPEYNELIKK; PEYNELIKKS; EYNELIKKSD; YNELIKKSDL;
NELIKKSDLE; ELIKKSDLEL; LIKKSDLELD; IKKSDLELDP;
KKSDLELDPI; KSDLELDPIK; SDLELDPIKR; DLELDPIKRQ;
LELDPIKRQD; ELDPIKRQDI; LDPIKRQDIL; DPIKRQDILR;
PIKRQDILRQ; IKRQDILRQA; KRQDILRQAE; RQDILRQAEE;
QDILRQAEEI; DILRQAEEII; ILRQAEEIII; LRQAEEIIIE;
RQAEEIIIEK; QAEEIIIEKD; AEEIIIEKDF; EEIIIEKDFP;
EIIIEKDFPI; IIIEKDFPIA; IIEKDFPIAP; IEKDFPIAPI;
EKDFPIAPIY; KDFPIAPIYI; DFPIAPIYIY; FPIAPIYIYG;
PIAPIYIYGN; IAPIYIYGNS; APIYIYGNSY; PIYIYGNSYL;
IYIYGNSYLF; YIYGNSYLFR; IYGNSYLFRN; YGNSYLFRND;
GNSYLFRNDK; NSYLFRNDKW; SYLFRNDKWT; YLFRNDKWTG;
LFRNDKWTGW; FRNDKWTGWN; RNDKWTGWNT; NDKWTGWNTN;
DKWTGWNTNI; KWTGWNTNIL; WTGWNTNILE; TGWNTNILER;
GWNTNILERF; WNTNILERFD; NTNILERFDL; TNILERFDLS;
NILERFDLSQ; ILERFDLSQL; LERFDLSQLK; ERFDLSQLKL;
RFDLSQLKLK; FDLSQLKLKN; DLSQLKLKNK;

11 mers:
MKLQRSLFLII; KLQRSLFLIIF; LQRSLFLIIFF; QRSLFLIIFFL;
RSLFLIIFFLT; SLFLIIFFLTF; LFLIIFFLTFL; FLIIFFLTFLC;
LIIFFLTFLCC; IIFFLTFLCCN; IFFLTFLCCNN; FFLTFLCCNNK;
FLTFLCCNNKE; LTFLCCNNKER; TFLCCNNKERK; FLCCNNKERKE;
LCCNNKERKEG; CCNNKERKEGV; CNNKERKEGVS; NNKERKEGVSF;
NKERKEGVSFK; KERKEGVSFKI; ERKEGVSFKIS; RKEGVSFKISL;
KEGVSFKISLG; EGVSFKISLGA; GVSFKISLGAE; VSFKISLGAEP;
SFKISLGAEPS; FKISLGAEPSS; KISLGAEPSSL; ISLGAEPSSLD;
SLGAEPSSLDP; LGAEPSSLDPQ; GAEPSSLDPQL; AEPSSLDPQLA;
EPSSLDPQLAE; PSSLDPQLAED; SSLDPQLAEDN; SLDPQLAEDNV;
LDPQLAEDNVA; DPQLAEDNVAS; PQLAEDNVASK; QLAEDNVASKM;
LAEDNVASKMI; AEDNVASKMID; EDNVASKMIDT; DNVASKMIDTM;
NVASKMIDTMF; VASKMIDTMFR; ASKMIDTMFRG; SKMIDTMFRGI;
KMIDTMFRGIV; MIDTMFRGIVT; IDTMFRGIVTG; DTMFRGIVTGD;

| | | | |
|---|---|---|---|
| TMFRGIVTGDP; | MFRGIVTGDPN; | FRGIVTGDPNT; | RGIVTGDPNTG; |
| GIVTGDPNTGG; | IVTGDPNTGGN; | VTGDPNTGGNK; | TGDPNTGGNKP; |
| GDPNTGGNKPG; | DPNTGGNKPGL; | PNTGGNKPGLA; | NTGGNKPGLAK; |
| TGGNKPGLAKG; | GGNKPGLAKGW; | GNKPGLAKGWD; | NKPGLAKGWDI; |
| KPGLAKGWDIS; | PGLAKGWDISS; | GLAKGWDISSD; | LAKGWDISSDG; |
| AKGWDISSDGT; | KGWDISSDGTV; | GWDISSDGTVY; | WDISSDGTVYT; |
| DISSDGTVYTF; | ISSDGTVYTFN; | SSDGTVYTFNL; | SDGTVYTFNLR; |
| DGTVYTFNLRE; | GTVYTFNLREK; | TVYTFNLREKI; | VYTFNLREKIT; |
| YTFNLREKITW; | TFNLREKITWS; | FNLREKITWSD; | NLREKITWSDG; |
| LREKITWSDGV; | REKITWSDGVA; | EKITWSDGVAI; | KITWSDGVAIT; |
| ITWSDGVAITA; | TWSDGVAITAE; | WSDGVAITAEG; | SDGVAITAEGI; |
| DGVAITAEGIR; | GVAITAEGIRK; | VAITAEGIRKS; | AITAEGIRKSY; |
| ITAEGIRKSYL; | TAEGIRKSYLR; | AEGIRKSYLRI; | EGIRKSYLRIL; |
| GIRKSYLRILN; | IRKSYLRILNK; | RKSYLRILNKE; | KSYLRILNKET; |
| SYLRILNKETG; | YLRILNKETGS; | LRILNKETGSK; | RILNKETGSKY; |
| ILNKETGSKYV; | LNKETGSKYVE; | NKETGSKYVEM; | KETGSKYVEMV; |
| ETGSKYVEMVK; | TGSKYVEMVKS; | GSKYVEMVKSV; | SKYVEMVKSVI; |
| KYVEMVKSVIK; | YVEMVKSVIKN; | VEMVKSVIKNG; | EMVKSVIKNGQ; |
| MVKSVIKNGQK; | VKSVIKNGQKY; | KSVIKNGQKYF; | SVIKNGQKYFD; |
| VIKNGQKYFDG; | IKNGQKYFDGQ; | KNGQKYFDGQV; | NGQKYFDGQVT; |
| GQKYFDGQVTD; | QKYFDGQVTDS; | KYFDGQVTDSE; | YFDGQVTDSEL; |
| FDGQVTDSELG; | DGQVTDSELGI; | GQVTDSELGIR; | QVTDSELGIRA; |
| VTDSELGIRAI; | TDSELGIRAID; | DSELGIRAIDE; | SELGIRAIDEK; |
| ELGIRAIDEKT; | LGIRAIDEKTL; | GIRAIDEKTLE; | IRAIDEKTLEI; |
| RAIDEKTLEIT; | AIDEKTLEITL; | IDEKTLEITLE; | DEKTLEITLES; |
| EKTLEITLESP; | KTLEITLESPK; | TLEITLESPKP; | LEITLESPKPY; |
| EITLESPKPYF; | ITLESPKPYFI; | TLESPKPYFID; | LESPKPYFIDM; |
| ESPKPYFIDML; | SPKPYFIDMLV; | PKPYFIDMLVH; | KPYFIDMLVHQ; |
| PYFIDMLVHQS; | YFIDMLVHQSF; | FIDMLVHQSFI; | IDMLVHQSFIP; |
| DMLVHQSFIPV; | MLVHQSFIPVP; | LVHQSFIPVPV; | VHQSFIPVPVH; |
| HQSFIPVPVHV; | QSFIPVPVHVT; | SFIPVPVHVTE; | FIPVPVHVTEK; |
| IPVPVHVTEKY; | PVPVHVTEKYG; | VPVHVTEKYGQ; | PVHVTEKYGQN; |
| VHVTEKYGQNW; | HVTEKYGQNWT; | VTEKYGQNWTS; | TEKYGQNWTSP; |
| EKYGQNWTSPE; | KYGQNWTSPEN; | YGQNWTSPENM; | GQNWTSPENMV; |
| QNWTSPENMVT; | NWTSPENMVTS; | WTSPENMVTSG; | TSPENMVTSGP; |
| SPENMVTSGPF; | PENMVTSGPFK; | ENMVTSGPFKL; | NMVTSGPFKLK; |
| MVTSGPFKLKE; | VTSGPFKLKER; | TSGPFKLKERI; | SGPFKLKERIP; |
| GPFKLKERIPN; | PFKLKERIPNE; | FKLKERIPNEK; | KLKERIPNEKY; |
| LKERIPNEKYV; | KERIPNEKYVF; | ERIPNEKYVFE; | RIPNEKYVFEK; |
| IPNEKYVFEKN; | PNEKYVFEKNN; | NEKYVFEKNNK; | EKYVFEKNNKY; |
| KYVFEKNNKYY; | YVFEKNNKYYD; | VFEKNNKYYDS; | FEKNNKYYDSN; |
| EKNNKYYDSNE; | KNNKYYDSNEV; | NNKYYDSNEVE; | NKYYDSNEVEL; |
| KYYDSNEVELE; | YYDSNEVELEE; | YDSNEVELEEI; | DSNEVELEEIT; |
| SNEVELEEITF; | NEVELEEITFY; | EVELEEITFYT; | VELEEITFYTT; |
| ELEEITFYTTN; | LEEITFYTTND; | EEITFYTTNDS; | EITFYTTNDSS; |
| ITFYTTNDSST; | TFYTTNDSSTA; | FYTTNDSSTAY; | YTTNDSSTAYK; |
| TTNDSSTAYKM; | TNDSSTAYKMY; | NDSSTAYKMYE; | DSSTAYKMYEN; |
| SSTAYKMYENE; | STAYKMYENEE; | TAYKMYENEEL; | AYKMYENEELD; |
| YKMYENEELDA; | KMYENEELDAI; | MYENEELDAIF; | YENEELDAIFG; |
| ENEELDAIFGS; | NEELDAIFGSI; | EELDAIFGSIP; | ELDAIFGSIPP; |
| LDAIFGSIPPD; | DAIFGSIPPDL; | AIFGSIPPDLI; | IFGSIPPDLIK; |

FGSIPPDLIKN; GSIPPDLIKNL; SIPPDLIKNLK; IPPDLIKNLKL;
PPDLIKNLKLR; PDLIKNLKLRS; DLIKNLKLRSD; LIKNLKLRSDY;
IKNLKLRSDYY; KNLKLRSDYYS; NLKLRSDYYSS; LKLRSDYYSSA;
KLRSDYYSSAV; LRSDYYSSAVN; RSDYYSSAVNA; SDYYSSAVNAI;
DYYSSAVNAIY; YSSAVNAIYF; YSSAVNAIYFY; SSAVNAIYFYA;
SAVNAIYFYAF; AVNAIYFYAFN; VNAIYFYAFNT; NAIYFYAFNTH;
AIYFYAFNTHI; IYFYAFNTHIK; YFYAFNTHIKP; FYAFNTHIKPL;
YAFNTHIKPLD; AFNTHIKPLDN; FNTHIKPLDNV; NTHIKPLDNVK;
THIKPLDNVKI; HIKPLDNVKIR; IKPLDNVKIRK; KPLDNVKIRKA;
PLDNVKIRKAL; LDNVKIRKALT; DNVKIRKALTL; NVKIRKALTLA;
VKIRKALTLAI; KIRKALTLAID; IRKALTLAIDR; RKALTLAIDRE;
KALTLAIDRET; ALTLAIDRETL; LTLAIDRETLT; TLAIDRETLTY;
LAIDRETLTYK; AIDRETLTYKV; IDRETLTYKVL; DRETLTYKVLD;
RETLTYKVLDN; ETLTYKVLDNG; TLTYKVLDNGT; LTYKVLDNGTT;
TYKVLDNGTTP; YKVLDNGTTPT; KVLDNGTTPTR; VLDNGTTPTRR;
LDNGTTPTRRA; DNGTTPTRRAT; NGTTPTRRATP; GTTPTRRATPN;
TTPTRRATPNF; TPTRRATPNFS; PTRRATPNFSS; TRRATPNFSSY;
RRATPNFSSYS; RATPNFSSYSY; ATPNFSSYSYA; TPNFSSYSYAK;
PNFSSYSYAKS; NFSSYSYAKSL; FSSYSYAKSLE; SSYSYAKSLEL;
SYSYAKSLELF; YSYAKSLELFN; SYAKSLELFNP; YAKSLELFNPE;
AKSLELFNPEI; KSLELFNPEIA; SLELFNPEIAK; LELFNPEIAKT;
ELFNPEIAKTL; LFNPEIAKTLL; FNPEIAKTLLA; NPEIAKTLLAE;
PEIAKTLLAEA; EIAKTLLAEAG; IAKTLLAEAGY; AKTLLAEAGYP;
KTLLAEAGYPN; TLLAEAGYPNG; LLAEAGYPNGN; LAEAGYPNGNG;
AEAGYPNGNGF; EAGYPNGNGFP; AGYPNGNGFPI; GYPNGNGFPIL;
YPNGNGFPILK; PNGNGFPILKL; NGNGFPILKLK; GNGFPILKLKY;
NGFPILKLKYN; GFPILKLKYNT; FPILKLKYNTN; PILKLKYNTNE;
ILKLKYNTNEA; LKLKYNTNEAN; KLKYNTNEANK; LKYNTNEANKK;
KYNTNEANKKI; YNTNEANKKIC; NTNEANKKICE; TNEANKKICEF;
NEANKKICEFI; EANKKICEFIQ; ANKKICEFIQN; NKKICEFIQNQ;
KKICEFIQNQW; KICEFIQNQWK; ICEFIQNQWKK; CEFIQNQWKKN;
EFIQNQWKKNL; FIQNQWKKNLN; IQNQWKKNLNI; QNQWKKNLNID;
NQWKKNLNIDV; QWKKNLNIDVE; WKKNLNIDVEL; KKNLNIDVELE;
KNLNIDVELEN; NLNIDVELENE; LNIDVELENEE; NIDVELENEEW;
IDVELENEEWT; DVELENEEWTT; VELENEEWTTY; ELENEEWTTYL;
LENEEWTTYLN; ENEEWTTYLNT; NEEWTTYLNTK; EEWTTYLNTKA;
EWTTYLNTKAN; WTTYLNTKANG; TTYLNTKANGN; TYLNTKANGNY;
YLNTKANGNYE; LNTKANGNYEI; NTKANGNYEIA; TKANGNYEIAR;
KANGNYEIARA; ANGNYEIARAG; NGNYEIARAGW; GNYEIARAGWI;
NYEIARAGWIG; YEIARAGWIGD; EIARAGWIGDY; IARAGWIGDYA;
ARAGWIGDYAD; RAGWIGDYADP; AGWIGDYADPL; GWIGDYADPLT;
WIGDYADPLTF; IGDYADPLTFL; GDYADPLTFLS; DYADPLTFLSI;
YADPLTFLSIF; ADPLTFLSIFT; DPLTFLSIFTQ; PLTFLSIFTQG;
LTFLSIFTQGY; TFLSIFTQGYT; FLSIFTQGYTQ; LSIFTQGYTQF;
SIFTQGYTQFS; IFTQGYTQFSS; FTQGYTQFSSH; TQGYTQFSSHN;
QGYTQFSSHNY; GYTQFSSHNYS; YTQFSSHNYSN; TQFSSHNYSNP;
QFSSHNYSNPE; FSSHNYSNPEY; SSHNYSNPEYN; SHNYSNPEYNE;
HNYSNPEYNEL; NYSNPEYNELI; YSNPEYNELIK; SNPEYNELIKK;
NPEYNELIKKS; PEYNELIKKSD; EYNELIKKSDL; YNELIKKSDLE;
NELIKKSDLEL; ELIKKSDLELD; LIKKSDLELDP; IKKSDLELDPI;
KKSDLELDPIK; KSDLELDPIKR; SDLELDPIKRQ; DLELDPIKRQD;

| | |
|---|---|
| | LELDPIKRQDI;  ELDPIKRQDIL;  LDPIKRQDILR;  DPIKRQDILRQ; <br> PIKRQDILRQA;  IKRQDILRQAE;  KRQDILRQAEE;  RQDILRQAEEI; <br> QDILRQAEEII;  DILRQAEEIII;  ILRQAEEIIIE;  LRQAEEIIIEK; <br> RQAEEIIIEKD;  QAEEIIIEKDF;  AEEIIIEKDFP;  EEIIIEKDFPI; <br> EIIIEKDFPIA;  IIIEKDFPIAP;  IIEKDFPIAPI;  IEKDFPIAPIY; <br> EKDFPIAPIYI;  KDFPIAPIYIY;  DFPIAPIYIYG;  FPIAPIYIYGN; <br> PIAPIYIYGNS;  IAPIYIYGNSY;  APIYIYGNSYL;  PIYIYGNSYLF; <br> IYIYGNSYLFR;  YIYGNSYLFRN;  IYGNSYLFRND;  YGNSYLFRNDK; <br> GNSYLFRNDKW;  NSYLFRNDKWT;  SYLFRNDKWTG;  YLFRNDKWTGW; <br> LFRNDKWTGWN;  FRNDKWTGWNT;  RNDKWTGWNTN;  NDKWTGWNTNI; <br> DKWTGWNTNIL;  KWTGWNTNILE;  WTGWNTNILER;  TGWNTNILERF; <br> GWNTNILERFD;  WNTNILERFDL;  NTNILERFDLS;  TNILERFDLSQ; <br> NILERFDLSQL;  ILERFDLSQLK;  LERFDLSQLKL;  ERFDLSQLKLK; <br> RFDLSQLKLKN;  FDLSQLKLKNK; |
| Seq 25 | SEQ ID NO 27862-28855/ <br> 8 mers: <br> MTKIFSNL;  TKIFSNLI;  KIFSNLII;  IFSNLIIN;  FSNLIING; <br> SNLIINGL;  NLIINGLL;  LIINGLLF;  IINGLLFG;  INGLLFGF; <br> NGLLFGFV;  GLLFGFVS;  LLFGFVSL;  LFGFVSLN;  FGFVSLNV; <br> GFVSLNVF;  FVSLNVFA;  VSLNVFAD;  SLNVFADS;  LNVFADSN; <br> NVFADSNN;  VFADSNNA;  FADSNNAN;  ADSNNANI;  DSNNANIL; <br> SNNANILK;  NNANILKP;  NANILKPQ;  ANILKPQS;  NILKPQSN; <br> ILKPQSNV;  LKPQSNVL;  KPQSNVLE;  PQSNVLEH;  QSNVLEHS; <br> SNVLEHSD;  NVLEHSDQ;  VLEHSDQK;  LEHSDQKD;  EHSDQKDN; <br> HSDQKDNK;  SDQKDNKK;  DQKDNKKL;  QKDNKKLD;  KDNKKLDQ; <br> DNKKLDQK;  NKKLDQKD;  KKLDQKDQ;  KLDQKDQV;  LDQKDQVN; <br> DQKDQVNQ;  QKDQVNQA;  KDQVNQAL;  DQVNQALD;  QVNQALDT; <br> VNQALDTI;  NQALDTIN;  QALDTINK;  ALDTINKV;  LDTINKVT; <br> DTINKVTE;  TINKVTED;  INKVTEDV;  NKVTEDVS;  KVTEDVSS; <br> VTEDVSSK;  TEDVSSKL;  EDVSSKLE;  DVSSKLEG;  VSSKLEGV; <br> SSKLEGVR;  SKLEGVRE;  KLEGVRES;  LEGVRESS;  EGVRESSL; <br> GVRESSLE;  VRESSLEL;  RESSLELV;  ESSLELVE;  SSLELVES; <br> SLELVESN;  LELVESND;  ELVESNDA;  LVESNDAG;  VESNDAGV; <br> ESNDAGVV;  SNDAGVVK;  NDAGVVKK;  DAGVVKKF;  AGVVKKFV; <br> GVVKKFVG;  VVKKFVGS;  VKKFVGSM;  KKFVGSMS;  KFVGSMSL; <br> FVGSMSLM;  VGSMSLMS;  GSMSLMSD;  SMSLMSDV;  MSLMSDVA; <br> SLMSDVAK;  LMSDVAKG;  MSDVAKGT;  SDVAKGTV;  DVAKGTVV; <br> VAKGTVVA;  AKGTVVAS;  KGTVVASQ;  GTVVASQE;  TVVASQEA; <br> VVASQEAT;  VASQEATI;  ASQEATIV;  SQEATIVA;  QEATIVAK; <br> EATIVAKC;  ATIVAKCS;  TIVAKCSG;  IVAKCSGM;  VAKCSGMV; <br> AKCSGMVA;  KCSGMVAE;  CSGMVAEG;  SGMVAEGA;  GMVAEGAN; <br> MVAEGANK;  VAEGANKV;  AEGANKVV;  EGANKVVE;  GANKVVEM; <br> ANKVVEMS;  NKVVEMSK;  KVVEMSKK;  VVEMSKKA;  VEMSKKAV; <br> EMSKKAVQ;  MSKKAVQE;  SKKAVQET;  KKAVQETQ;  KAVQETQK; <br> AVQETQKA;  VQETQKAV;  QETQKAVS;  ETQKAVSV;  TQKAVSVA; <br> QKAVSVAG;  KAVSVAGE;  AVSVAGEA;  VSVAGEAT;  SVAGEATF; <br> VAGEATFL;  AGEATFLI;  GEATFLIE;  EATFLIEK;  ATFLIEKQ; <br> TFLIEKQI;  FLIEKQIM;  LIEKQIML;  IEKQIMLN;  EKQIMLNK; <br> KQIMLNKS;  QIMLNKSP;  IMLNKSPN;  MLNKSPNN;  LNKSPNNK; <br> NKSPNNKE;  KSPNNKEL;  SPNNKELE;  PNNKELEL;  NNKELELT; |

```
NKELELTK;  KELELTKE;  ELELTKEE;  LELTKEEF;  ELTKEEFA;
LTKEEFAK;  TKEEFAKV;  KEEFAKVD;  EEFAKVDE;  EFAKVDEV;
FAKVDEVK;  AKVDEVKE;  KVDEVKET;  VDEVKETL;  DEVKETLM;
EVKETLMA;  VKETLMAS;  KETLMASE;  ETLMASER;  TLMASERA;
LMASERAL;  MASERALD;  ASERALDE;  SERALDET;  ERALDETV;
RALDETVQ;  ALDETVQE;  LDETVQEA;  DETVQEAQ;  ETVQEAQK;
TVQEAQKV;  VQEAQKVL;  QEAQKVLN;  EAQKVLNM;  AQKVLNMV;
QKVLNMVN;  KVLNMVNG;  VLNMVNGL;  LNMVNGLN;  NMVNGLNP;
MVNGLNPS;  VNGLNPSN;  NGLNPSNK;  GLNPSNKD;  LNPSNKDQ;
NPSNKDQV;  PSNKDQVL;  SNKDQVLA;  NKDQVLAK;  KDQVLAKK;
DQVLAKKD;  QVLAKKDV;  VLAKKDVR;  LAKKDVRK;  AKKDVRKA;
KKDVRKAI;  KDVRKAIS;  DVRKAISN;  VRKAISNV;  RKAISNVV;
KAISNVVK;  AISNVVKV;  ISNVVKVA;  SNVVKVAQ;  NVVKVAQG;
VVKVAQGA;  VKVAQGAR;  KVAQGARD;  VAQGARDL;  AQGARDLT;
QGARDLTK;  GARDLTKV;  ARDLTKVM;  RDLTKVMA;  DLTKVMAI;
LTKVMAIS;  TKVMAISL;  KVMAISLY;  VMAISLYM;  MAISLYMR;

9 mers:
MTKIFSNLI;  TKIFSNLII;  KIFSNLIIN;  IFSNLIING;  FSNLIINGL;
SNLIINGLL;  NLIINGLLF;  LIINGLLFG;  IINGLLFGF;  INGLLFGFV;
NGLLFGFVS;  GLLFGFVSL;  LLFGFVSLN;  LFGFVSLNV;  FGFVSLNVF;
GFVSLNVFA;  FVSLNVFAD;  VSLNVFADS;  SLNVFADSN;  LNVFADSNN;
NVFADSNNA;  VFADSNNAN;  FADSNNANI;  ADSNNANIL;  DSNNANILK;
SNNANILKP;  NNANILKPQ;  NANILKPQS;  ANILKPQSN;  NILKPQSNV;
ILKPQSNVL;  LKPQSNVLE;  KPQSNVLEH;  PQSNVLEHS;  QSNVLEHSD;
SNVLEHSDQ;  NVLEHSDQK;  VLEHSDQKD;  LEHSDQKDN;  EHSDQKDNK;
HSDQKDNKK;  SDQKDNKKL;  DQKDNKKLD;  QKDNKKLDQ;  KDNKKLDQK;
DNKKLDQKD;  NKKLDQKDQ;  KKLDQKDQV;  KLDQKDQVN;  LDQKDQVNQ;
DQKDQVNQA;  QKDQVNQAL;  KDQVNQALD;  DQVNQALDT;  QVNQALDTI;
VNQALDTIN;  NQALDTINK;  QALDTINKV;  ALDTINKVT;  LDTINKVTE;
DTINKVTED;  TINKVTEDV;  INKVTEDVS;  NKVTEDVSS;  KVTEDVSSK;
VTEDVSSKL;  TEDVSSKLE;  EDVSSKLEG;  DVSSKLEGV;  VSSKLEGVR;
SSKLEGVRE;  SKLEGVRES;  KLEGVRESS;  LEGVRESSL;  EGVRESSLE;
GVRESSLEL;  VRESSLELV;  RESSLELVE;  ESSLELVES;  SSLELVESN;
SLELVESND;  LELVESNDA;  ELVESNDAG;  LVESNDAGV;  VESNDAGVV;
ESNDAGVVK;  SNDAGVVKK;  NDAGVVKKF;  DAGVVKKFV;  AGVVKKFVG;
GVVKKFVGS;  VVKKFVGSM;  VKKFVGSMS;  KKFVGSMSL;  KFVGSMSLM;
FVGSMSLMS;  VGSMSLMSD;  GSMSLMSDV;  SMSLMSDVA;  MSLMSDVAK;
SLMSDVAKG;  LMSDVAKGT;  MSDVAKGTV;  SDVAKGTVV;  DVAKGTVVA;
VAKGTVVAS;  AKGTVVASQ;  KGTVVASQE;  GTVVASQEA;  TVVASQEAT;
VVASQEATI;  VASQEATIV;  ASQEATIVA;  SQEATIVAK;  QEATIVAKC;
EATIVAKCS;  ATIVAKCSG;  TIVAKCSGM;  IVAKCSGMV;  VAKCSGMVA;
AKCSGMVAE;  KCSGMVAEG;  CSGMVAEGA;  SGMVAEGAN;  GMVAEGANK;
MVAEGANKV;  VAEGANKVV;  AEGANKVVE;  EGANKVVEM;  GANKVVEMS;
ANKVVEMSK;  NKVVEMSKK;  KVVEMSKKA;  VVEMSKKAV;  VEMSKKAVQ;
EMSKKAVQE;  MSKKAVQET;  SKKAVQETQ;  KKAVQETQK;  KAVQETQKA;
AVQETQKAV;  VQETQKAVS;  QETQKAVSV;  ETQKAVSVA;  TQKAVSVAG;
QKAVSVAGE;  KAVSVAGEA;  AVSVAGEAT;  VSVAGEATF;  SVAGEATFL;
VAGEATFLI;  AGEATFLIE;  GEATFLIEK;  EATFLIEKQ;  ATFLIEKQI;
TFLIEKQIM;  FLIEKQIML;  LIEKQIMLN;  IEKQIMLNK;  EKQIMLNKS;
KQIMLNKSP;  QIMLNKSPN;  IMLNKSPNN;  MLNKSPNNK;  LNKSPNNKE;
```

NKSPNNKEL; KSPNNKELE; SPNNKELEL; PNNKELELT; NNKELELTK;
NKELELTKE; KELELTKEE; ELELTKEEF; LELTKEEFA; ELTKEEFAK;
LTKEEFAKV; TKEEFAKVD; KEEFAKVDE; EEFAKVDEV; EFAKVDEVK;
FAKVDEVKE; AKVDEVKET; KVDEVKETL; VDEVKETLM; DEVKETLMA;
EVKETLMAS; VKETLMASE; KETLMASER; ETLMASERA; TLMASERAL;
LMASERALD; MASERALDE; ASERALDET; SERALDETV; ERALDETVQ;
RALDETVQE; ALDETVQEA; LDETVQEAQ; DETVQEAQK; ETVQEAQKV;
TVQEAQKVL; VQEAQKVLN; QEAQKVLNM; EAQKVLNMV; AQKVLNMVN;
QKVLNMVNG; KVLNMVNGL; VLNMVNGLN; LNMVNGLNP; NMVNGLNPS;
MVNGLNPSN; VNGLNPSNK; NGLNPSNKD; GLNPSNKDQ; LNPSNKDQV;
NPSNKDQVL; PSNKDQVLA; SNKDQVLAK; NKDQVLAKK; KDQVLAKKD;
DQVLAKKDV; QVLAKKDVR; VLAKKDVRK; LAKKDVRKA; AKKDVRKAI;
KKDVRKAIS; KDVRKAISN; DVRKAISNV; VRKAISNVV; RKAISNVVK;
KAISNVVKV; AISNVVKVA; ISNVVKVAQ; SNVVKVAQG; NVVKVAQGA;
VVKVAQGAR; VKVAQGARD; KVAQGARDL; VAQGARDLT; AQGARDLTK;
QGARDLTKV; GARDLTKVM; ARDLTKVMA; RDLTKVMAI; DLTKVMAIS;
LTKVMAISL; TKVMAISLY; KVMAISLYM; VMAISLYMR;

10 mers:
MTKIFSNLII; TKIFSNLIIN; KIFSNLIING; IFSNLIINGL;
FSNLIINGLL; SNLIINGLLF; NLIINGLLFG; LIINGLLFGF;
IINGLLFGFV; INGLLFGFVS; NGLLFGFVSL; GLLFGFVSLN;
LLFGFVSLNV; LFGFVSLNVF; FGFVSLNVFA; GFVSLNVFAD;
FVSLNVFADS; VSLNVFADSN; SLNVFADSNN; LNVFADSNNA;
NVFADSNNAN; VFADSNNANI; FADSNNANIL; ADSNNANILK;
DSNNANILKP; SNNANILKPQ; NNANILKPQS; NANILKPQSN;
ANILKPQSNV; NILKPQSNVL; ILKPQSNVLE; LKPQSNVLEH;
KPQSNVLEHS; PQSNVLEHSD; QSNVLEHSDQ; SNVLEHSDQK;
NVLEHSDQKD; VLEHSDQKDN; LEHSDQKDNK; EHSDQKDNKK;
HSDQKDNKKL; SDQKDNKKLD; DQKDNKKLDQ; QKDNKKLDQK;
KDNKKLDQKD; DNKKLDQKDQ; NKKLDQKDQV; KKLDQKDQVN;
KLDQKDQVNQ; LDQKDQVNQA; DQKDQVNQAL; QKDQVNQALD;
KDQVNQALDT; DQVNQALDTI; QVNQALDTIN; VNQALDTINK;
NQALDTINKV; QALDTINKVT; ALDTINKVTE; LDTINKVTED;
DTINKVTEDV; TINKVTEDVS; INKVTEDVSS; NKVTEDVSSK;
KVTEDVSSKL; VTEDVSSKLE; TEDVSSKLEG; EDVSSKLEGV;
DVSSKLEGVR; VSSKLEGVRE; SSKLEGVRES; SKLEGVRESS;
KLEGVRESSL; LEGVRESSLE; EGVRESSLEL; GVRESSLELV;
VRESSLELVE; RESSLELVES; ESSLELVESN; SSLELVESND;
SLELVESNDA; LELVESNDAG; ELVESNDAGV; LVESNDAGVV;
VESNDAGVVK; ESNDAGVVKK; SNDAGVVKKF; NDAGVVKKFV;
DAGVVKKFVG; AGVVKKFVGS; GVVKKFVGSM; VVKKFVGSMS;
VKKFVGSMSL; KKFVGSMSLM; KFVGSMSLMS; FVGSMSLMSD;
VGSMSLMSDV; GSMSLMSDVA; SMSLMSDVAK; MSLMSDVAKG;
SLMSDVAKGT; LMSDVAKGTV; MSDVAKGTVV; SDVAKGTVVA;
DVAKGTVVAS; VAKGTVVASQ; AKGTVVASQE; KGTVVASQEA;
GTVVASQEAT; TVVASQEATI; VVASQEATIV; VASQEATIVA;
ASQEATIVAK; SQEATIVAKC; QEATIVAKCS; EATIVAKCSG;
ATIVAKCSGM; TIVAKCSGMV; IVAKCSGMVA; VAKCSGMVAE;
AKCSGMVAEG; KCSGMVAEGA; CSGMVAEGAN; SGMVAEGANK;
GMVAEGANKV; MVAEGANKVV; VAEGANKVVE; AEGANKVVEM;

EGANKVVEMS; GANKVVEMSK; ANKVVEMSKK; NKVVEMSKKA;
KVVEMSKKAV; VVEMSKKAVQ; VEMSKKAVQE; EMSKKAVQET;
MSKKAVQETQ; SKKAVQETQK; KKAVQETQKA; KAVQETQKAV;
AVQETQKAVS; VQETQKAVSV; QETQKAVSVA; ETQKAVSVAG;
TQKAVSVAGE; QKAVSVAGEA; KAVSVAGEAT; AVSVAGEATF;
VSVAGEATFL; SVAGEATFLI; VAGEATFLIE; AGEATFLIEK;
GEATFLIEKQ; EATFLIEKQI; ATFLIEKQIM; TFLIEKQIML;
FLIEKQIMLN; LIEKQIMLNK; IEKQIMLNKS; EKQIMLNKSP;
KQIMLNKSPN; QIMLNKSPNN; IMLNKSPNNK; MLNKSPNNKE;
LNKSPNNKEL; NKSPNNKELE; KSPNNKELEL; SPNNKELELT;
PNNKELELTK; NNKELELTKE; NKELELTKEE; KELELTKEEF;
ELELTKEEFA; LELTKEEFAK; ELTKEEFAKV; LTKEEFAKVD;
TKEEFAKVDE; KEEFAKVDEV; EEFAKVDEVK; EFAKVDEVKE;
FAKVDEVKET; AKVDEVKETL; KVDEVKETLM; VDEVKETLMA;
DEVKETLMAS; EVKETLMASE; VKETLMASER; KETLMASERA;
ETLMASERAL; TLMASERALD; LMASERALDE; MASERALDET;
ASERALDETV; SERALDETVQ; ERALDETVQE; RALDETVQEA;
ALDETVQEAQ; LDETVQEAQK; DETVQEAQKV; ETVQEAQKVL;
TVQEAQKVLN; VQEAQKVLNM; QEAQKVLNMV; EAQKVLNMVN;
AQKVLNMVNG; QKVLNMVNGL; KVLNMVNGLN; VLNMVNGLNP;
LNMVNGLNPS; NMVNGLNPSN; MVNGLNPSNK; VNGLNPSNKD;
NGLNPSNKDQ; GLNPSNKDQV; LNPSNKDQVL; NPSNKDQVLA;
PSNKDQVLAK; SNKDQVLAKK; NKDQVLAKKD; KDQVLAKKDV;
DQVLAKKDVR; QVLAKKDVRK; VLAKKDVRKA; LAKKDVRKAI;
AKKDVRKAIS; KKDVRKAISN; KDVRKAISNV; DVRKAISNVV;
VRKAISNVVK; RKAISNVVKV; KAISNVVKVA; AISNVVKVAQ;
ISNVVKVAQG; SNVVKVAQGA; NVVKVAQGAR; VVKVAQGARD;
VKVAQGARDL; KVAQGARDLT; VAQGARDLTK; AQGARDLTKV;
QGARDLTKVM; GARDLTKVMA; ARDLTKVMAI; RDLTKVMAIS;
DLTKVMAISL; LTKVMAISLY; TKVMAISLYM; KVMAISLYMR;

11 mers:
MTKIFSNLIIN; TKIFSNLIING; KIFSNLIINGL; IFSNLIINGLL;
FSNLIINGLLF; SNLIINGLLFG; NLIINGLLFGF; LIINGLLFGFV;
IINGLLFGFVS; INGLLFGFVSL; NGLLFGFVSLN; GLLFGFVSLNV;
LLFGFVSLNVF; LFGFVSLNVFA; FGFVSLNVFAD; GFVSLNVFADS;
FVSLNVFADSN; VSLNVFADSNN; SLNVFADSNNA; LNVFADSNNAN;
NVFADSNNANI; VFADSNNANIL; FADSNNANILK; ADSNNANILKP;
DSNNANILKPQ; SNNANILKPQS; NNANILKPQSN; NANILKPQSNV;
ANILKPQSNVL; NILKPQSNVLE; ILKPQSNVLEH; LKPQSNVLEHS;
KPQSNVLEHSD; PQSNVLEHSDQ; QSNVLEHSDQK; SNVLEHSDQKD;
NVLEHSDQKDN; VLEHSDQKDNK; LEHSDQKDNKK; EHSDQKDNKKL;
HSDQKDNKKLD; SDQKDNKKLDQ; DQKDNKKLDQK; QKDNKKLDQKD;
KDNKKLDQKDQ; DNKKLDQKDQV; NKKLDQKDQVN; KKLDQKDQVNQ;
KLDQKDQVNQA; LDQKDQVNQAL; DQKDQVNQALD; QKDQVNQALDT;
KDQVNQALDTI; DQVNQALDTIN; QVNQALDTINK; VNQALDTINKV;
NQALDTINKVT; QALDTINKVTE; ALDTINKVTED; LDTINKVTEDV;
DTINKVTEDVS; TINKVTEDVSS; INKVTEDVSSK; NKVTEDVSSKL;
KVTEDVSSKLE; VTEDVSSKLEG; TEDVSSKLEGV; EDVSSKLEGVR;
DVSSKLEGVRE; VSSKLEGVRES; SSKLEGVRESS; SKLEGVRESSL;
KLEGVRESSLE; LEGVRESSLEL; EGVRESSLELV; GVRESSLELVE;

| | |
|---|---|
| | VRESSLELVES; RESSLELVESN; ESSLELVESND; SSLELVESNDA; SLELVESNDAG; LELVESNDAGV; ELVESNDAGVV; LVESNDAGVVK; VESNDAGVVKK; ESNDAGVVKKF; SNDAGVVKKFV; NDAGVVKKFVG; DAGVVKKFVGS; AGVVKKFVGSM; GVVKKFVGSMS; VVKKFVGSMSL; VKKFVGSMSLM; KKFVGSMSLMS; KFVGSMSLMSD; FVGSMSLMSDV; VGSMSLMSDVA; GSMSLMSDVAK; SMSLMSDVAKG; MSLMSDVAKGT; SLMSDVAKGTV; LMSDVAKGTVV; MSDVAKGTVVA; SDVAKGTVVAS; DVAKGTVVASQ; VAKGTVVASQE; AKGTVVASQEA; KGTVVASQEAT; GTVVASQEATI; TVVASQEATIV; VVASQEATIVA; VASQEATIVAK; ASQEATIVAKC; SQEATIVAKCS; QEATIVAKCSG; EATIVAKCSGM; ATIVAKCSGMV; TIVAKCSGMVA; IVAKCSGMVAE; VAKCSGMVAEG; AKCSGMVAEGA; KCSGMVAEGAN; CSGMVAEGANK; SGMVAEGANKV; GMVAEGANKVV; MVAEGANKVVE; VAEGANKVVEM; AEGANKVVEMS; EGANKVVEMSK; GANKVVEMSKK; ANKVVEMSKKA; NKVVEMSKKAV; KVVEMSKKAVQ; VVEMSKKAVQE; VEMSKKAVQET; EMSKKAVQETQ; MSKKAVQETQK; SKKAVQETQKA; KKAVQETQKAV; KAVQETQKAVS; AVQETQKAVSV; VQETQKAVSVA; QETQKAVSVAG; ETQKAVSVAGE; TQKAVSVAGEA; QKAVSVAGEAT; KAVSVAGEATF; AVSVAGEATFL; VSVAGEATFLI; SVAGEATFLIE; VAGEATFLIEK; AGEATFLIEKQ; GEATFLIEKQI; EATFLIEKQIM; ATFLIEKQIML; TFLIEKQIMLN; FLIEKQIMLNK; LIEKQIMLNKS; IEKQIMLNKSP; EKQIMLNKSPN; KQIMLNKSPNN; QIMLNKSPNNK; IMLNKSPNNKE; MLNKSPNNKEL; LNKSPNNKELE; NKSPNNKELEL; KSPNNKELELT; SPNNKELELTK; PNNKELELTKE; NNKELELTKEE; NKELELTKEEF; KELELTKEEFA; ELELTKEEFAK; LELTKEEFAKV; ELTKEEFAKVD; LTKEEFAKVDE; TKEEFAKVDEV; KEEFAKVDEVK; EEFAKVDEVKE; EFAKVDEVKET; FAKVDEVKETL; AKVDEVKETLM; KVDEVKETLMA; VDEVKETLMAS; DEVKETLMASE; EVKETLMASER; VKETLMASERA; KETLMASERAL; ETLMASERALD; TLMASERALDE; LMASERALDET; MASERALDETV; ASERALDETVQ; SERALDETVQE; ERALDETVQEA; RALDETVQEAQ; ALDETVQEAQK; LDETVQEAQKV; DETVQEAQKVL; ETVQEAQKVLN; TVQEAQKVLNM; VQEAQKVLNMV; QEAQKVLNMVN; EAQKVLNMVNG; AQKVLNMVNGL; QKVLNMVNGLN; KVLNMVNGLNP; VLNMVNGLNPS; LNMVNGLNPSN; NMVNGLNPSNK; MVNGLNPSNKD; VNGLNPSNKDQ; NGLNPSNKDQV; GLNPSNKDQVL; LNPSNKDQVLA; NPSNKDQVLAK; PSNKDQVLAKK; SNKDQVLAKKD; NKDQVLAKKDV; KDQVLAKKDVR; DQVLAKKDVRK; QVLAKKDVRKA; VLAKKDVRKAI; LAKKDVRKAIS; AKKDVRKAISN; KKDVRKAISNV; KDVRKAISNVV; DVRKAISNVVK; VRKAISNVVKV; RKAISNVVKVA; KAISNVVKVAQ; AISNVVKVAQG; ISNVVKVAQGA; SNVVKVAQGAR; NVVKVAQGARD; VVKVAQGARDL; VKVAQGARDLT; KVAQGARDLTK; VAQGARDLTKV; AQGARDLTKVM; QGARDLTKVMA; GARDLTKVMAI; ARDLTKVMAIS; RDLTKVMAISL; DLTKVMAISLY; LTKVMAISLYM; TKVMAISLYMR; |
| Seq 26 | SEQ ID NO 28856-31653/<br>8 mers:<br>MKKMLLIF; KKMLLIFS; KMLLIFSF; MLLIFSFF; LLIFSFFL; LIFSFFLI; IFSFFLIF; FSFFLIFL; SFFLIFLN; FFLIFLNG; FLIFLNGF; LIFLNGFP; IFLNGFPL; FLNGFPLN; LNGFPLNA; NGFPLNAR; GFPLNARK; FPLNARKV; PLNARKVD; LNARKVDK; NARKVDKE; ARKVDKEK; RKVDKEKL; KVDKEKLK; VDKEKLKD; |

| | | | | |
|---|---|---|---|---|
| DKEKLKDF; | KEKLKDFV; | EKLKDFVN; | KLKDFVNM; | LKDFVNMD; |
| KDFVNMDL; | DFVNMDLE; | FVNMDLEF; | VNMDLEFV; | NMDLEFVN; |
| MDLEFVNY; | DLEFVNYK; | LEFVNYKG; | EFVNYKGP; | FVNYKGPY; |
| VNYKGPYD; | NYKGPYDS; | YKGPYDST; | KGPYDSTN; | GPYDSTNT; |
| PYDSTNTY; | YDSTNTYE; | DSTNTYEQ; | STNTYEQI; | TNTYEQIV; |
| NTYEQIVG; | TYEQIVGI; | YEQIVGIG; | EQIVGIGE; | QIVGIGEF; |
| IVGIGEFL; | VGIGEFLA; | GIGEFLAR; | IGEFLARP; | GEFLARPL; |
| EFLARPLT; | FLARPLTN; | LARPLTNS; | ARPLTNSN; | RPLTNSNS; |
| PLTNSNSN; | LTNSNSNS; | TNSNSNSS; | NSNSNSSY; | SNSNSSYY; |
| NSNSSYYG; | SNSSYYGK; | NSSYYGKY; | SSYYGKYF; | SYYGKYFI; |
| YYGKYFIN; | YGKYFINR; | GKYFINRF; | KYFINRFI; | YFINRFID; |
| FINRFIDD; | INRFIDDQ; | NRFIDDQD; | RFIDDQDK; | FIDDQDKK; |
| IDDQDKKA; | DDQDKKAS; | DQDKKASV; | QDKKASVD; | DKKASVDV; |
| KKASVDVF; | KASVDVFS; | ASVDVFSI; | SVDVFSIS; | VDVFSISS; |
| DVFSISSK; | VFSISSKS; | FSISSKSE; | SISSKSEL; | ISSKSELD; |
| SSKSELDS; | SKSELDSI; | KSELDSIL; | SELDSILN; | ELDSILNL; |
| LDSILNLR; | DSILNLRR; | SILNLRRI; | ILNLRRIL; | LNLRRILT; |
| NLRRILTG; | LRRILTGY; | RRILTGYI; | RILTGYII; | ILTGYIIK; |
| LTGYIIKS; | TGYIIKSF; | GYIIKSFD; | YIIKSFDY; | IIKSFDYD; |
| IKSFDYDR; | KSFDYDRS; | SFDYDRSS; | FDYDRSSA; | DYDRSSAE; |
| YDRSSAEL; | DRSSAELI; | RSSAELIA; | SSAELIAK; | SAELIAKV; |
| AELIAKVI; | ELIAKVIT; | LIAKVITI; | IAKVITIY; | AKVITIYN; |
| KVITIYNA; | VITIYNAV; | ITIYNAVY; | TIYNAVYR; | IYNAVYRG; |
| YNAVYRGD; | NAVYRGDL; | AVYRGDLD; | VYRGDLDY; | YRGDLDYY; |
| RGDLDYYK; | GDLDYYKG; | DLDYYKGF; | LDYYKGFY; | DYYKGFYI; |
| YYKGFYIE; | YKGFYIEP; | KGFYIEPA; | GFYIEPAL; | FYIEPALK; |
| YIEPALKS; | IEPALKSL; | EPALKSLT; | PALKSLTK; | ALKSLTKE; |
| LKSLTKEN; | KSLTKENA; | SLTKENAG; | LTKENAGL; | TKENAGLS; |
| KENAGLSR; | ENAGLSRV; | NAGLSRVY; | AGLSRVYS; | GLSRVYSQ; |
| LSRVYSQW; | SRVYSQWA; | RVYSQWAG; | VYSQWAGK; | YSQWAGKT; |
| SQWAGKTQ; | QWAGKTQI; | WAGKTQIF; | AGKTQIFI; | GKTQIFIP; |
| KTQIFIPL; | TQIFIPLK; | QIFIPLKK; | IFIPLKKD; | FIPLKKDI; |
| IPLKKDIL; | PLKKDILS; | LKKDILSG; | KKDILSGN; | KDILSGNI; |
| DILSGNIE; | ILSGNIES; | LSGNIESD; | SGNIESDI; | GNIESDID; |
| NIESDIDI; | IESDIDID; | ESDIDIDS; | SDIDIDSL; | DIDIDSLV; |
| IDIDSLVT; | DIDSLVTD; | IDSLVTDK; | DSLVTDKV; | SLVTDKVI; |
| LVTDKVIA; | VTDKVIAA; | TDKVIAAL; | DKVIAALL; | KVIAALLS; |
| VIAALLSE; | IAALLSEN; | AALLSENE; | ALLSENEA; | LLSENEAG; |
| LSENEAGV; | SENEAGVN; | ENEAGVNF; | NEAGVNFA; | EAGVNFAR; |
| AGVNFARD; | GVNFARDI; | VNFARDIT; | NFARDITD; | FARDITDI; |
| ARDITDIQ; | RDITDIQG; | DITDIQGE; | ITDIQGET; | TDIQGETH; |
| DIQGETHK; | IQGETHKA; | QGETHKAD; | GETHKADQ; | ETHKADQD; |
| THKADQDK; | HKADQDKI; | KADQDKID; | ADQDKIDT; | DQDKIDTE; |
| QDKIDTEL; | DKIDTELD; | KIDTELDN; | IDTELDNI; | DTELDNIH; |
| TELDNIHE; | ELDNIHES; | LDNIHESD; | DNIHESDS; | NIHESDSN; |
| IHESDSNI; | HESDSNIT; | ESDSNITE; | SDSNITET; | DSNITETI; |
| SNITETIE; | NITETIEN; | ITETIENL; | TETIENLR; | ETIENLRD; |
| TIENLRDQ; | IENLRDQL; | ENLRDQLE; | NLRDQLEK; | LRDQLEKA; |
| RDQLEKAT; | DQLEKATD; | QLEKATDE; | LEKATDEE; | EKATDEEH; |
| KATDEEHK; | ATDEEHKK; | TDEEHKKE; | DEEHKKEI; | EEHKKEIE; |
| EHKKEIES; | HKKEIESQ; | KKEIESQV; | KEIESQVD; | EIESQVDA; |

| | | | | |
|---|---|---|---|---|
| IESQVDAK; | ESQVDAKK; | SQVDAKKK; | QVDAKKKE; | VDAKKKEK; |
| DAKKKEKE; | AKKKEKEE; | KKKEKEEL; | KKEKEELD; | KEKEELDK; |
| EKEELDKK; | KEELDKKA; | EELDKKAI; | ELDKKAIN; | LDKKAINL; |
| DKKAINLD; | KKAINLDK; | KAINLDKA; | AINLDKAQ; | INLDKAQQ; |
| NLDKAQQK; | LDKAQQKL; | DKAQQKLD; | KAQQKLDS; | AQQKLDSA; |
| QQKLDSAE; | QKLDSAED; | KLDSAEDN; | LDSAEDNL; | DSAEDNLD; |
| SAEDNLDV; | AEDNLDVQ; | EDNLDVQR; | DNLDVQRD; | NLDVQRDT; |
| LDVQRDTV; | DVQRDTVR; | VQRDTVRE; | QRDTVREK; | RDTVREKI; |
| DTVREKIQ; | TVREKIQE; | VREKIQED; | REKIQEDI; | EKIQEDIN; |
| KIQEDINE; | IQEDINEI; | QEDINEIN; | EDINEINK; | DINEINKE; |
| INEINKEK; | NEINKEKN; | EINKEKNL; | INKEKNLP; | NKEKNLPK; |
| KEKNLPKP; | EKNLPKPG; | KNLPKPGD; | NLPKPGDV; | LPKPGDVS; |
| PKPGDVSS; | KPGDVSSP; | PGDVSSPK; | GDVSSPKV; | DVSSPKVD; |
| VSSPKVDK; | SSPKVDKQ; | SPKVDKQL; | PKVDKQLQ; | KVDKQLQI; |
| VDKQLQIK; | DKQLQIKE; | KQLQIKES; | QLQIKESL; | LQIKESLE; |
| QIKESLED; | IKESLEDL; | KESLEDLQ; | ESLEDLQE; | SLEDLQEQ; |
| LEDLQEQL; | EDLQEQLK; | DLQEQLKE; | LQEQLKEA; | QEQLKEAG; |
| EQLKEAGD; | QLKEAGDE; | LKEAGDEN; | KEAGDENQ; | EAGDENQK; |
| AGDENQKR; | GDENQKRE; | DENQKREI; | ENQKREIE; | NQKREIEK; |
| QKREIEKQ; | KREIEKQI; | REIEKQIE; | EIEKQIEI; | IEKQIEIK; |
| EKQIEIKK; | KQIEIKKR; | QIEIKKRD; | IEIKKRDE; | EIKKRDEE; |
| IKKRDEEL; | KKRDEELL; | KRDEELLK; | RDEELLKS; | DEELLKSK; |
| EELLKSKD; | ELLKSKDG; | LLKSKDGK; | LKSKDGKV; | KSKDGKVS; |
| SKDGKVSK; | KDGKVSKD; | DGKVSKDY; | GKVSKDYE; | KVSKDYEA; |
| VSKDYEAL; | SKDYEALD; | KDYEALDL; | DYEALDLD; | YEALDLDR; |
| EALDLDRE; | ALDLDREL; | LDLDRELS; | DLDRELSK; | LDRELSKA; |
| DRELSKAS; | RELSKASS; | ELSKASSK; | LSKASSKE; | SKASSKEK; |
| KASSKEKS; | ASSKEKSK; | SSKEKSKV; | SKEKSKVK; | KEKSKVKE; |
| EKSKVKEE; | KSKVKEEE; | SKVKEEEI; | KVKEEEIT; | VKEEEITK; |
| KEEEITKG; | EEEITKGK; | EEITKGKS; | EITKGKSR; | ITKGKSRA; |
| TKGKSRAS; | KGKSRASL; | GKSRASLG; | KSRASLGD; | SRASLGDL; |
| RASLGDLN; | ASLGDLNN; | SLGDLNND; | LGDLNNDK; | GDLNNDKN; |
| DLNNDKNL; | LNNDKNLM; | NNDKNLML; | NDKNLMLP; | DKNLMLPE; |
| KNLMLPED; | NLMLPEDQ; | LMLPEDQK; | MLPEDQKL; | LPEDQKLP; |
| PEDQKLPE; | EDQKLPED; | DQKLPEDK; | QKLPEDKK; | KLPEDKKL; |
| LPEDKKLD; | PEDKKLDS; | EDKKLDSK; | DKKLDSKL; | KKLDSKLD; |
| KLDSKLDG; | LDSKLDGK; | DSKLDGKK; | SKLDGKKE; | KLDGKKEF; |
| LDGKKEFK; | DGKKEFKP; | GKKEFKPV; | KKEFKPVS; | KEFKPVSE; |
| EFKPVSEV; | FKPVSEVE; | KPVSEVEK; | PVSEVEKL; | VSEVEKLD; |
| SEVEKLDK; | EVEKLDKI; | VEKLDKIS; | EKLDKISK; | KLDKISKS; |
| LDKISKSN; | DKISKSNN; | KISKSNNN; | ISKSNNNE; | SKSNNNEV; |
| KSNNNEVG; | SNNNEVGK; | NNNEVGKL; | NNEVGKLS; | NEVGKLSP; |
| EVGKLSPL; | VGKLSPLD; | GKLSPLDK; | KLSPLDKP; | LSPLDKPS; |
| SPLDKPSY; | PLDKPSYD; | LDKPSYDD; | DKPSYDDI; | KPSYDDID; |
| PSYDDIDS; | SYDDIDSK; | YDDIDSKE; | DDIDSKEE; | DIDSKEEV; |
| IDSKEEVD; | DSKEEVDN; | SKEEVDNK; | KEEVDNKA; | EEVDNKAI; |
| EVDNKAIN; | VDNKAINL; | DNKAINLQ; | NKAINLQK; | KAINLQKI; |
| AINLQKID; | INLQKIDP; | NLQKIDPK; | LQKIDPKV; | QKIDPKVK; |
| KIDPKVKD; | IDPKVKDQ; | DPKVKDQT; | PKVKDQTT; | KVKDQTTS; |
| VKDQTTSL; | KDQTTSLN; | DQTTSLNE; | QTTSLNED; | TTSLNEDL; |
| TSLNEDLD; | SLNEDLDK; | LNEDLDKD; | NEDLDKDL; | EDLDKDLT; |

DLDKDLTT; LDKDLTTM; DKDLTTMS; KDLTTMSI; DLTTMSID;
LTTMSIDS; TTMSIDSS; TMSIDSSS; MSIDSSSP; SIDSSSPV;
IDSSSPVF; DSSSPVFL; SSSPVFLE; SSPVFLEV; SPVFLEVI;
PVFLEVID; VFLEVIDP; FLEVIDPI; LEVIDPIT; EVIDPITN;
VIDPITNL; IDPITNLG; DPITNLGT; PITNLGTL; ITNLGTLQ;
TNLGTLQL; NLGTLQLI; LGTLQLID; GTLQLIDL; TLQLIDLN;
LQLIDLNT; QLIDLNTG; LIDLNTGV; IDLNTGVR; DLNTGVRL;
LNTGVRLK; NTGVRLKE; TGVRLKES; GVRLKEST; VRLKESTQ;
RLKESTQQ; LKESTQQG; KESTQQGI; ESTQQGIQ; STQQGIQR;
TQQGIQRY; QQGIQRYG; QGIQRYGI; GIQRYGIY; IQRYGIYE;
QRYGIYER; RYGIYERE; YGIYEREK; GIYEREKD; IYEREKDL;
YEREKDLV; EREKDLVV; REKDLVVI; EKDLVVIK; KDLVVIKM;
DLVVIKMD; LVVIKMDS; VVIKMDSG; VIKMDSGK; IKMDSGKA;
KMDSGKAK; MDSGKAKL; DSGKAKLQ; SGKAKLQI; GKAKLQIL;
KAKLQILN; AKLQILNK; KLQILNKL; LQILNKLE; QILNKLEN;
ILNKLENL; LNKLENLK; NKLENLKV; KLENLKVV; LENLKVVS;
ENLKVVSE; NLKVVSES; LKVVSESN; KVVSESNF; VVSESNFE;
VSESNFEI; SESNFEIN; ESNFEINK; SNFEINKN; NFEINKNS;
FEINKNSS; EINKNSSL; INKNSSLY; NKNSSLYV; KNSSLYVD;
NSSLYVDS; SSLYVDSK; SLYVDSKM; LYVDSKMI; YVDSKMIL;
VDSKMILA; DSKMILAA; SKMILAAV; KMILAAVR; MILAAVRD;
ILAAVRDK; LAAVRDKD; AAVRDKDD; AVRDKDDS; VRDKDDSN;
RDKDDSNA; DKDDSNAW; KDDSNAWR; DDSNAWRL; DSNAWRLA;
SNAWRLAK; NAWRLAKF; AWRLAKFS; WRLAKFSP; RLAKFSPK;
LAKFSPKN; AKFSPKNL; KFSPKNLD; FSPKNLDE; SPKNLDEF;
PKNLDEFI; KNLDEFIL; NLDEFILS; LDEFILSE; DEFILSEN;
EFILSENK; FILSENKI; ILSENKIL; LSENKILP; SENKILPF;
ENKILPFT; NKILPFTS; KILPFTSF; ILPFTSFS; LPFTSFSV;
PFTSFSVR; FTSFSVRK; TSFSVRKN; SFSVRKNF; FSVRKNFI;
SVRKNFIY; VRKNFIYL; RKNFIYLQ; KNFIYLQD; NFIYLQDE;
FIYLQDEL; IYLQDELK; YLQDELKN; LQDELKNL; QDELKNLV;
DELKNLVI; ELKNLVIL; LKNLVILD; KNLVILDV; NLVILDVN;
LVILDVNT; VILDVNTL; ILDVNTLK; LDVNTLKK; DVNTLKKV;
VNTLKKVK;

9 mers:
MKKMLLIFS; KKMLLIFSF; KMLLIFSFF; MLLIFSFFL; LLIFSFFLI;
LIFSFFLIF; IFSFFLIFL; FSFFLIFLN; SFFLIFLNG; FFLIFLNGF;
FLIFLNGFP; LIFLNGFPL; IFLNGFPLN; FLNGFPLNA; LNGFPLNAR;
NGFPLNARK; GFPLNARKV; FPLNARKVD; PLNARKVDK; LNARKVDKE;
NARKVDKEK; ARKVDKEKL; RKVDKEKLK; KVDKEKLKD; VDKEKLKDF;
DKEKLKDFV; KEKLKDFVN; EKLKDFVNM; KLKDFVNMD; LKDFVNMDL;
KDFVNMDLE; DFVNMDLEF; FVNMDLEFV; VNMDLEFVN; NMDLEFVNY;
MDLEFVNYK; DLEFVNYKG; LEFVNYKGP; EFVNYKGPY; FVNYKGPYD;
VNYKGPYDS; NYKGPYDST; YKGPYDSTN; KGPYDSTNT; GPYDSTNTY;
PYDSTNTYE; YDSTNTYEQ; DSTNTYEQI; STNTYEQIV; TNTYEQIVG;
NTYEQIVGI; TYEQIVGIG; YEQIVGIGE; EQIVGIGEF; QIVGIGEFL;
IVGIGEFLA; VGIGEFLAR; GIGEFLARP; IGEFLARPL; GEFLARPLT;
EFLARPLTN; FLARPLTNS; LARPLTNSN; ARPLTNSNS; RPLTNSNSN;
PLTNSNSNS; LTNSNSNSS; TNSNSNSSY; NSNSNSSYY; SNSNSSYYG;
NSNSSYYGK; SNSSYYGKY; NSSYYGKYF; SSYYGKYFI; SYYGKYFIN;

| | | | | |
|---|---|---|---|---|
| YYGKYFINR; | YGKYFINRF; | GKYFINRFI; | KYFINRFID; | YFINRFIDD; |
| FINRFIDDQ; | INRFIDDQD; | NRFIDDQDK; | RFIDDQDKK; | FIDDQDKKA; |
| IDDQDKKAS; | DDQDKKASV; | DQDKKASVD; | QDKKASVDV; | DKKASVDVF; |
| KKASVDVFS; | KASVDVFSI; | ASVDVFSIS; | SVDVFSISS; | VDVFSISSK; |
| DVFSISSKS; | VFSISSKSE; | FSISSKSEL; | SISSKSELD; | ISSKSELDS; |
| SSKSELDSI; | SKSELDSIL; | KSELDSILN; | SELDSILNL; | ELDSILNLR; |
| LDSILNLRR; | DSILNLRRI; | SILNLRRIL; | ILNLRRILT; | LNLRRILTG; |
| NLRRILTGY; | LRRILTGYI; | RRILTGYII; | RILTGYIIK; | ILTGYIIKS; |
| LTGYIIKSF; | TGYIIKSFD; | GYIIKSFDY; | YIIKSFDYD; | IIKSFDYDR; |
| IKSFDYDRS; | KSFDYDRSS; | SFDYDRSSA; | FDYDRSSAE; | DYDRSSAEL; |
| YDRSSAELI; | DRSSAELIA; | RSSAELIAK; | SSAELIAKV; | SAELIAKVI; |
| AELIAKVIT; | ELIAKVITI; | LIAKVITIY; | IAKVITIYN; | AKVITIYNA; |
| KVITIYNAV; | VITIYNAVY; | ITIYNAVYR; | TIYNAVYRG; | IYNAVYRGD; |
| YNAVYRGDL; | NAVYRGDLD; | AVYRGDLDY; | VYRGDLDYY; | YRGDLDYYK; |
| RGDLDYYKG; | GDLDYYKGF; | DLDYYKGFY; | LDYYKGFYI; | DYYKGFYIE; |
| YYKGFYIEP; | YKGFYIEPA; | KGFYIEPAL; | GFYIEPALK; | FYIEPALKS; |
| YIEPALKSL; | IEPALKSLT; | EPALKSLTK; | PALKSLTKE; | ALKSLTKEN; |
| LKSLTKENA; | KSLTKENAG; | SLTKENAGL; | LTKENAGLS; | TKENAGLSR; |
| KENAGLSRV; | ENAGLSRVY; | NAGLSRVYS; | AGLSRVYSQ; | GLSRVYSQW; |
| LSRVYSQWA; | SRVYSQWAG; | RVYSQWAGK; | VYSQWAGKT; | YSQWAGKTQ; |
| SQWAGKTQI; | QWAGKTQIF; | WAGKTQIFI; | AGKTQIFIP; | GKTQIFIPL; |
| KTQIFIPLK; | TQIFIPLKK; | QIFIPLKKD; | IFIPLKKDI; | FIPLKKDIL; |
| IPLKKDILS; | PLKKDILSG; | LKKDILSGN; | KKDILSGNI; | KDILSGNIE; |
| DILSGNIES; | ILSGNIESD; | LSGNIESDI; | SGNIESDID; | GNIESDIDI; |
| NIESDIDID; | IESDIDIDS; | ESDIDIDSL; | SDIDIDSLV; | DIDIDSLVT; |
| IDIDSLVTD; | DIDSLVTDK; | IDSLVTDKV; | DSLVTDKVI; | SLVTDKVIA; |
| LVTDKVIAA; | VTDKVIAAL; | TDKVIAALL; | DKVIAALLS; | KVIAALLSE; |
| VIAALLSEN; | IAALLSENE; | AALLSENEA; | ALLSENEAG; | LLSENEAGV; |
| LSENEAGVN; | SENEAGVNF; | ENEAGVNFA; | NEAGVNFAR; | EAGVNFARD; |
| AGVNFARDI; | GVNFARDIT; | VNFARDITD; | NFARDITDI; | FARDITDIQ; |
| ARDITDIQG; | RDITDIQGE; | DITDIQGET; | ITDIQGETH; | TDIQGETHK; |
| DIQGETHKA; | IQGETHKAD; | QGETHKADQ; | GETHKADQD; | ETHKADQDK; |
| THKADQDKI; | HKADQDKID; | KADQDKIDT; | ADQDKIDTE; | DQDKIDTEL; |
| QDKIDTELD; | DKIDTELDN; | KIDTELDNI; | IDTELDNIH; | DTELDNIHE; |
| TELDNIHES; | ELDNIHESD; | LDNIHESDS; | DNIHESDSN; | NIHESDSNI; |
| IHESDSNIT; | HESDSNITE; | ESDSNITET; | SDSNITETI; | DSNITETIE; |
| SNITETIEN; | NITETIENL; | ITETIENLR; | TETIENLRD; | ETIENLRDQ; |
| TIENLRDQL; | IENLRDQLE; | ENLRDQLEK; | NLRDQLEKA; | LRDQLEKAT; |
| RDQLEKATD; | DQLEKATDE; | QLEKATDEE; | LEKATDEEH; | EKATDEEHK; |
| KATDEEHKK; | ATDEEHKKE; | TDEEHKKEI; | DEEHKKEIE; | EEHKKEIES; |
| EHKKEIESQ; | HKKEIESQV; | KKEIESQVD; | KEIESQVDA; | EIESQVDAK; |
| IESQVDAKK; | ESQVDAKKK; | SQVDAKKKE; | QVDAKKKEK; | VDAKKKEKE; |
| DAKKKEKEE; | AKKKEKEEL; | KKKEKEELD; | KKEKEELDK; | KEKEELDKK; |
| EKEELDKKA; | KEELDKKAI; | EELDKKAIN; | ELDKKAINL; | LDKKAINLD; |
| DKKAINLDK; | KKAINLDKA; | KAINLDKAQ; | AINLDKAQQ; | INLDKAQQK; |
| NLDKAQQKL; | LDKAQQKLD; | DKAQQKLDS; | KAQQKLDSA; | AQQKLDSAE; |
| QQKLDSAED; | QKLDSAEDN; | KLDSAEDNL; | LDSAEDNLD; | DSAEDNLDV; |
| SAEDNLDVQ; | AEDNLDVQR; | EDNLDVQRD; | DNLDVQRDT; | NLDVQRDTV; |
| LDVQRDTVR; | DVQRDTVRE; | VQRDTVREK; | QRDTVREKI; | RDTVREKIQ; |
| DTVREKIQE; | TVREKIQED; | VREKIQEDI; | REKIQEDIN; | EKIQEDINE; |
| KIQEDINEI; | IQEDINEIN; | QEDINEINK; | EDINEINKE; | DINEINKEK; |

INEINKEKN; NEINKEKNL; EINKEKNLP; INKEKNLPK; NKEKNLPKP;
KEKNLPKPG; EKNLPKPGD; KNLPKPGDV; NLPKPGDVS; LPKPGDVSS;
PKPGDVSSP; KPGDVSSPK; PGDVSSPKV; GDVSSPKVD; DVSSPKVDK;
VSSPKVDKQ; SSPKVDKQL; SPKVDKQLQ; PKVDKQLQI; KVDKQLQIK;
VDKQLQIKE; DKQLQIKES; KQLQIKESL; QLQIKESLE; LQIKESLED;
QIKESLEDL; IKESLEDLQ; KESLEDLQE; ESLEDLQEQ; SLEDLQEQL;
LEDLQEQLK; EDLQEQLKE; DLQEQLKEA; LQEQLKEAG; QEQLKEAGD;
EQLKEAGDE; QLKEAGDEN; LKEAGDENQ; KEAGDENQK; EAGDENQKR;
AGDENQKRE; GDENQKREI; DENQKREIE; ENQKREIEK; NQKREIEKQ;
QKREIEKQI; KREIEKQIE; REIEKQIEI; EIEKQIEIK; IEKQIEIKK;
EKQIEIKKR; KQIEIKKRD; QIEIKKRDE; IEIKKRDEE; EIKKRDEEL;
IKKRDEELL; KKRDEELLK; KRDEELLKS; RDEELLKSK; DEELLKSKD;
EELLKSKDG; ELLKSKDGK; LLKSKDGKV; LKSKDGKVS; KSKDGKVSK;
SKDGKVSKD; KDGKVSKDY; DGKVSKDYE; GKVSKDYEA; KVSKDYEAL;
VSKDYEALD; SKDYEALDL; KDYEALDLD; DYEALDLDR; YEALDLDRE;
EALDLDREL; ALDLDRELS; LDLDRELSK; DLDRELSKA; LDRELSKAS;
DRELSKASS; RELSKASSK; ELSKASSKE; LSKASSKEK; SKASSKEKS;
KASSKEKSK; ASSKEKSKV; SSKEKSKVK; SKEKSKVKE; KEKSKVKEE;
EKSKVKEEE; KSKVKEEEI; SKVKEEEIT; KVKEEEITK; VKEEEITKG;
KEEEITKGK; EEEITKGKS; EEITKGKSR; EITKGKSRA; ITKGKSRAS;
TKGKSRASL; KGKSRASLG; GKSRASLGD; KSRASLGDL; SRASLGDLN;
RASLGDLNN; ASLGDLNND; SLGDLNNDK; LGDLNNDKN; GDLNNDKNL;
DLNNDKNLM; LNNDKNLML; NNDKNLMLP; NDKNLMLPE; DKNLMLPED;
KNLMLPEDQ; NLMLPEDQK; LMLPEDQKL; MLPEDQKLP; LPEDQKLPE;
PEDQKLPED; EDQKLPEDK; DQKLPEDKK; QKLPEDKKL; KLPEDKKLD;
LPEDKKLDS; PEDKKLDSK; EDKKLDSKL; DKKLDSKLD; KKLDSKLDG;
KLDSKLDGK; LDSKLDGKK; DSKLDGKKE; SKLDGKKEF; KLDGKKEFK;
LDGKKEFKP; DGKKEFKPV; GKKEFKPVS; KKEFKPVSE; KEFKPVSEV;
EFKPVSEVE; FKPVSEVEK; KPVSEVEKL; PVSEVEKLD; VSEVEKLDK;
SEVEKLDKI; EVEKLDKIS; VEKLDKISK; EKLDKISKS; KLDKISKSN;
LDKISKSNN; DKISKSNNN; KISKSNNNE; ISKSNNNEV; SKSNNNEVG;
KSNNNEVGK; SNNNEVGKL; NNNEVGKLS; NNEVGKLSP; NEVGKLSPL;
EVGKLSPLD; VGKLSPLDK; GKLSPLDKP; KLSPLDKPS; LSPLDKPSY;
SPLDKPSYD; PLDKPSYDD; LDKPSYDDI; DKPSYDDID; KPSYDDIDS;
PSYDDIDSK; SYDDIDSKE; YDDIDSKEE; DDIDSKEEV; DIDSKEEVD;
IDSKEEVDN; DSKEEVDNK; SKEEVDNKA; KEEVDNKAI; EEVDNKAIN;
EVDNKAINL; VDNKAINLQ; DNKAINLQK; NKAINLQKI; KAINLQKID;
AINLQKIDP; INLQKIDPK; NLQKIDPKV; LQKIDPKVK; QKIDPKVKD;
KIDPKVKDQ; IDPKVKDQT; DPKVKDQTT; PKVKDQTTS; KVKDQTTSL;
VKDQTTSLN; KDQTTSLNE; DQTTSLNED; QTTSLNEDL; TTSLNEDLD;
TSLNEDLDK; SLNEDLDKD; LNEDLDKDL; NEDLDKDLT; EDLDKDLTT;
DLDKDLTTM; LDKDLTTMS; DKDLTTMSI; KDLTTMSID; DLTTMSIDS;
LTTMSIDSS; TTMSIDSSS; TMSIDSSSP; MSIDSSSPV; SIDSSSPVF;
IDSSSPVFL; DSSSPVFLE; SSSPVFLEV; SSPVFLEVI; SPVFLEVID;
PVFLEVIDP; VFLEVIDPI; FLEVIDPIT; LEVIDPITN; EVIDPITNL;
VIDPITNLG; IDPITNLGT; DPITNLGTL; PITNLGTLQ; ITNLGTLQL;
TNLGTLQLI; NLGTLQLID; LGTLQLIDL; GTLQLIDLN; TLQLIDLNT;
LQLIDLNTG; QLIDLNTGV; LIDLNTGVR; IDLNTGVRL; DLNTGVRLK;
LNTGVRLKE; NTGVRLKES; TGVRLKEST; GVRLKESTQ; VRLKESTQQ;
RLKESTQQG; LKESTQQGI; KESTQQGIQ; ESTQQGIQR; STQQGIQRY;
TQQGIQRYG; QQGIQRYGI; QGIQRYGIY; GIQRYGIYE; IQRYGIYER;

QRYGIYERE; RYGIYEREK; YGIYEREKD; GIYEREKDL; IYEREKDLV;
YEREKDLVV; EREKDLVVI; REKDLVVIK; EKDLVVIKM; KDLVVIKMD;
DLVVIKMDS; LVVIKMDSG; VVIKMDSGK; VIKMDSGKA; IKMDSGKAK;
KMDSGKAKL; MDSGKAKLQ; DSGKAKLQI; SGKAKLQIL; GKAKLQILN;
KAKLQILNK; AKLQILNKL; KLQILNKLE; LQILNKLEN; QILNKLENL;
ILNKLENLK; LNKLENLKV; NKLENLKVV; KLENLKVVS; LENLKVVSE;
ENLKVVSES; NLKVVSESN; LKVVSESNF; KVVSESNFE; VVSESNFEI;
VSESNFEIN; SESNFEINK; ESNFEINKN; SNFEINKNS; NFEINKNSS;
FEINKNSSL; EINKNSSLY; INKNSSLYV; NKNSSLYVD; KNSSLYVDS;
NSSLYVDSK; SSLYVDSKM; SLYVDSKMI; LYVDSKMIL; YVDSKMILA;
VDSKMILAA; DSKMILAAV; SKMILAAVR; KMILAAVRD; MILAAVRDK;
ILAAVRDKD; LAAVRDKDD; AAVRDKDDS; AVRDKDDSN; VRDKDDSNA;
RDKDDSNAW; DKDDSNAWR; KDDSNAWRL; DDSNAWRLA; DSNAWRLAK;
SNAWRLAKF; NAWRLAKFS; AWRLAKFSP; WRLAKFSPK; RLAKFSPKN;
LAKFSPKNL; AKFSPKNLD; KFSPKNLDE; FSPKNLDEF; SPKNLDEFI;
PKNLDEFIL; KNLDEFILS; NLDEFILSE; LDEFILSEN; DEFILSENK;
EFILSENKI; FILSENKIL; ILSENKILP; LSENKILPF; SENKILPFT;
ENKILPFTS; NKILPFTSF; KILPFTSFS; ILPFTSFSV; LPFTSFSVR;
PFTSFSVRK; FTSFSVRKN; TSFSVRKNF; SFSVRKNFI; FSVRKNFIY;
SVRKNFIYL; VRKNFIYLQ; RKNFIYLQD; KNFIYLQDE; NFIYLQDEL;
FIYLQDELK; IYLQDELKN; YLQDELKNL; LQDELKNLV; QDELKNLVI;
DELKNLVIL; ELKNLVILD; LKNLVILDV; KNLVILDVN; NLVILDVNT;
LVILDVNTL; VILDVNTLK; ILDVNTLKK; LDVNTLKKV; DVNTLKKVK;

10 mers:
MKKMLLIFSF; KKMLLIFSFF; KMLLIFSFFL; MLLIFSFFLI;
LLIFSFFLIF; LIFSFFLIFL; IFSFFLIFLN; FSFFLIFLNG;
SFFLIFLNGF; FFLIFLNGFP; FLIFLNGFPL; LIFLNGFPLN;
IFLNGFPLNA; FLNGFPLNAR; LNGFPLNARK; NGFPLNARKV;
GFPLNARKVD; FPLNARKVDK; PLNARKVDKE; LNARKVDKEK;
NARKVDKEKL; ARKVDKEKLK; RKVDKEKLKD; KVDKEKLKDF;
VDKEKLKDFV; DKEKLKDFVN; KEKLKDFVNM; EKLKDFVNMD;
KLKDFVNMDL; LKDFVNMDLE; KDFVNMDLEF; DFVNMDLEFV;
FVNMDLEFVN; VNMDLEFVNY; NMDLEFVNYK; MDLEFVNYKG;
DLEFVNYKGP; LEFVNYKGPY; EFVNYKGPYD; FVNYKGPYDS;
VNYKGPYDST; NYKGPYDSTN; YKGPYDSTNT; KGPYDSTNTY;
GPYDSTNTYE; PYDSTNTYEQ; YDSTNTYEQI; DSTNTYEQIV;
STNTYEQIVG; TNTYEQIVGI; NTYEQIVGIG; TYEQIVGIGE;
YEQIVGIGEF; EQIVGIGEFL; QIVGIGEFLA; IVGIGEFLAR;
VGIGEFLARP; GIGEFLARPL; IGEFLARPLT; GEFLARPLTN;
EFLARPLTNS; FLARPLTNSN; LARPLTNSNS; ARPLTNSNSN;
RPLTNSNSNS; PLTNSNSNSS; LTNSNSNSSY; TNSNSNSSYY;
NSNSNSSYYG; SNSNSSYYGK; NSNSSYYGKY; SNSSYYGKYF;
NSSYYGKYFI; SSYYGKYFIN; SYYGKYFINR; YYGKYFINRF;
YGKYFINRFI; GKYFINRFID; KYFINRFIDD; YFINRFIDDQ;
FINRFIDDQD; INRFIDDQDK; NRFIDDQDKK; RFIDDQDKKA;
FIDDQDKKAS; IDDQDKKASV; DDQDKKASVD; DQDKKASVDV;
QDKKASVDVF; DKKASVDVFS; KKASVDVFSI; KASVDVFSIS;
ASVDVFSISS; SVDVFSISSK; VDVFSISSKS; DVFSISSKSE;
VFSISSKSEL; FSISSKSELD; SISSKSELDS; ISSKSELDSI;
SSKSELDSIL; SKSELDSILN; KSELDSILNL; SELDSILNLR;

EP 2 254 592 B1

ELDSILNLRR; LDSILNLRRI; DSILNLRRIL; SILNLRRILT;
ILNLRRILTG; LNLRRILTGY; NLRRILTGYI; LRRILTGYII;
RRILTGYIIK; RILTGYIIKS; ILTGYIIKSF; LTGYIIKSFD;
TGYIIKSFDY; GYIIKSFDYD; YIIKSFDYDR; IIKSFDYDRS;
IKSFDYDRSS; KSFDYDRSSA; SFDYDRSSAE; FDYDRSSAEL;
DYDRSSAELI; YDRSSAELIA; DRSSAELIAK; RSSAELIAKV;
SSAELIAKVI; SAELIAKVIT; AELIAKVITI; ELIAKVITIY;
LIAKVITIYN; IAKVITIYNA; AKVITIYNAV; KVITIYNAVY;
VITIYNAVYR; ITIYNAVYRG; TIYNAVYRGD; IYNAVYRGDL;
YNAVYRGDLD; NAVYRGDLDY; AVYRGDLDYY; VYRGDLDYYK;
YRGDLDYYKG; RGDLDYYKGF; GDLDYYKGFY; DLDYYKGFYI;
LDYYKGFYIE; DYYKGFYIEP; YYKGFYIEPA; YKGFYIEPAL;
KGFYIEPALK; GFYIEPALKS; FYIEPALKSL; YIEPALKSLT;
IEPALKSLTK; EPALKSLTKE; PALKSLTKEN; ALKSLTKENA;
LKSLTKENAG; KSLTKENAGL; SLTKENAGLS; LTKENAGLSR;
TKENAGLSRV; KENAGLSRVY; ENAGLSRVYS; NAGLSRVYSQ;
AGLSRVYSQW; GLSRVYSQWA; LSRVYSQWAG; SRVYSQWAGK;
RVYSQWAGKT; VYSQWAGKTQ; YSQWAGKTQI; SQWAGKTQIF;
QWAGKTQIFI; WAGKTQIFIP; AGKTQIFIPL; GKTQIFIPLK;
KTQIFIPLKK; TQIFIPLKKD; QIFIPLKKDI; IFIPLKKDIL;
FIPLKKDILS; IPLKKDILSG; PLKKDILSGN; LKKDILSGNI;
KKDILSGNIE; KDILSGNIES; DILSGNIESD; ILSGNIESDI;
LSGNIESDID; SGNIESDIDI; GNIESDIDID; NIESDIDIDS;
IESDIDIDSL; ESDIDIDSLV; SDIDIDSLVT; DIDIDSLVTD;
IDIDSLVTDK; DIDSLVTDKV; IDSLVTDKVI; DSLVTDKVIA;
SLVTDKVIAA; LVTDKVIAAL; VTDKVIAALL; TDKVIAALLS;
DKVIAALLSE; KVIAALLSEN; VIAALLSENE; IAALLSENEA;
AALLSENEAG; ALLSENEAGV; LLSENEAGVN; LSENEAGVNF;
SENEAGVNFA; ENEAGVNFAR; NEAGVNFARD; EAGVNFARDI;
AGVNFARDIT; GVNFARDITD; VNFARDITDI; NFARDITDIQ;
FARDITDIQG; ARDITDIQGE; RDITDIQGET; DITDIQGETH;
ITDIQGETHK; TDIQGETHKA; DIQGETHKAD; IQGETHKADQ;
QGETHKADQD; GETHKADQDK; ETHKADQDKI; THKADQDKID;
HKADQDKIDT; KADQDKIDTE; ADQDKIDTEL; DQDKIDTELD;
QDKIDTELDN; DKIDTELDNI; KIDTELDNIH; IDTELDNIHE;
DTELDNIHES; TELDNIHESD; ELDNIHESDS; LDNIHESDSN;
DNIHESDSNI; NIHESDSNIT; IHESDSNITE; HESDSNITET;
ESDSNITETI; SDSNITETIE; DSNITETIEN; SNITETIENL;
NITETIENLR; ITETIENLRD; TETIENLRDQ; ETIENLRDQL;
TIENLRDQLE; IENLRDQLEK; ENLRDQLEKA; NLRDQLEKAT;
LRDQLEKATD; RDQLEKATDE; DQLEKATDEE; QLEKATDEEH;
LEKATDEEHK; EKATDEEHKK; KATDEEHKKE; ATDEEHKKEI;
TDEEHKKEIE; DEEHKKEIES; EEHKKEIESQ; EHKKEIESQV;
HKKEIESQVD; KKEIESQVDA; KEIESQVDAK; EIESQVDAKK;
IESQVDAKKK; ESQVDAKKKE; SQVDAKKKEK; QVDAKKKEKE;
VDAKKKEKEE; DAKKKEKEEL; AKKKEKEELD; KKKEKEELDK;
KKEKEELDKK; KEKEELDKKA; EKEELDKKAI; KEELDKKAIN;
EELDKKAINL; ELDKKAINLD; LDKKAINLDK; DKKAINLDKA;
KKAINLDKAQ; KAINLDKAQQ; AINLDKAQQK; INLDKAQQKL;
NLDKAQQKLD; LDKAQQKLDS; DKAQQKLDSA; KAQQKLDSAE;
AQQKLDSAED; QQKLDSAEDN; QKLDSAEDNL; KLDSAEDNLD;

| | | | |
|---|---|---|---|
| LDSAEDNLDV; | DSAEDNLDVQ; | SAEDNLDVQR; | AEDNLDVQRD; |
| EDNLDVQRDT; | DNLDVQRDTV; | NLDVQRDTVR; | LDVQRDTVRE; |
| DVQRDTVREK; | VQRDTVREKI; | QRDTVREKIQ; | RDTVREKIQE; |
| DTVREKIQED; | TVREKIQEDI; | VREKIQEDIN; | REKIQEDINE; |
| EKIQEDINEI; | KIQEDINEIN; | IQEDINEINK; | QEDINEINKE; |
| EDINEINKEK; | DINEINKEKN; | INEINKEKNL; | NEINKEKNLP; |
| EINKEKNLPK; | INKEKNLPKP; | NKEKNLPKPG; | KEKNLPKPGD; |
| EKNLPKPGDV; | KNLPKPGDVS; | NLPKPGDVSS; | LPKPGDVSSP; |
| PKPGDVSSPK; | KPGDVSSPKV; | PGDVSSPKVD; | GDVSSPKVDK; |
| DVSSPKVDKQ; | VSSPKVDKQL; | SSPKVDKQLQ; | SPKVDKQLQI; |
| PKVDKQLQIK; | KVDKQLQIKE; | VDKQLQIKES; | DKQLQIKESL; |
| KQLQIKESLE; | QLQIKESLED; | LQIKESLEDL; | QIKESLEDLQ; |
| IKESLEDLQE; | KESLEDLQEQ; | ESLEDLQEQL; | SLEDLQEQLK; |
| LEDLQEQLKE; | EDLQEQLKEA; | DLQEQLKEAG; | LQEQLKEAGD; |
| QEQLKEAGDE; | EQLKEAGDEN; | QLKEAGDENQ; | LKEAGDENQK; |
| KEAGDENQKR; | EAGDENQKRE; | AGDENQKREI; | GDENQKREIE; |
| DENQKREIEK; | ENQKREIEKQ; | NQKREIEKQI; | QKREIEKQIE; |
| KREIEKQIEI; | REIEKQIEIK; | EIEKQIEIKK; | IEKQIEIKKR; |
| EKQIEIKKRD; | KQIEIKKRDE; | QIEIKKRDEE; | IEIKKRDEEL; |
| EIKKRDEELL; | IKKRDEELLK; | KKRDEELLKS; | KRDEELLKSK; |
| RDEELLKSKD; | DEELLKSKDG; | EELLKSKDGK; | ELLKSKDGKV; |
| LLKSKDGKVS; | LKSKDGKVSK; | KSKDGKVSKD; | SKDGKVSKDY; |
| KDGKVSKDYE; | DGKVSKDYEA; | GKVSKDYEAL; | KVSKDYEALD; |
| VSKDYEALDL; | SKDYEALDLD; | KDYEALDLDR; | DYEALDLDRE; |
| YEALDLDREL; | EALDLDRELS; | ALDLDRELSK; | LDLDRELSKA; |
| DLDRELSKAS; | LDRELSKASS; | DRELSKASSK; | RELSKASSKE; |
| ELSKASSKEK; | LSKASSKEKS; | SKASSKEKSK; | KASSKEKSKV; |
| ASSKEKSKVK; | SSKEKSKVKE; | SKEKSKVKEE; | KEKSKVKEEE; |
| EKSKVKEEEI; | KSKVKEEEIT; | SKVKEEEITK; | KVKEEEITKG; |
| VKEEEITKGK; | KEEEITKGKS; | EEEITKGKSR; | EEITKGKSRA; |
| EITKGKSRAS; | ITKGKSRASL; | TKGKSRASLG; | KGKSRASLGD; |
| GKSRASLGDL; | KSRASLGDLN; | SRASLGDLNN; | RASLGDLNND; |
| ASLGDLNNDK; | SLGDLNNDKN; | LGDLNNDKNL; | GDLNNDKNLM; |
| DLNNDKNLML; | LNNDKNLMLP; | NNDKNLMLPE; | NDKNLMLPED; |
| DKNLMLPEDQ; | KNLMLPEDQK; | NLMLPEDQKL; | LMLPEDQKLP; |
| MLPEDQKLPE; | LPEDQKLPED; | PEDQKLPEDK; | EDQKLPEDKK; |
| DQKLPEDKKL; | QKLPEDKKLD; | KLPEDKKLDS; | LPEDKKLDSK; |
| PEDKKLDSKL; | EDKKLDSKLD; | DKKLDSKLDG; | KKLDSKLDGK; |
| KLDSKLDGKK; | LDSKLDGKKE; | DSKLDGKKEF; | SKLDGKKEFK; |
| KLDGKKEFKP; | LDGKKEFKPV; | DGKKEFKPVS; | GKKEFKPVSE; |
| KKEFKPVSEV; | KEFKPVSEVE; | EFKPVSEVEK; | FKPVSEVEKL; |
| KPVSEVEKLD; | PVSEVEKLDK; | VSEVEKLDKI; | SEVEKLDKIS; |
| EVEKLDKISK; | VEKLDKISKS; | EKLDKISKSN; | KLDKISKSNN; |
| LDKISKSNNN; | DKISKSNNNE; | KISKSNNNEV; | ISKSNNNEVG; |
| SKSNNNEVGK; | KSNNNEVGKL; | SNNNEVGKLS; | NNNEVGKLSP; |
| NNEVGKLSPL; | NEVGKLSPLD; | EVGKLSPLDK; | VGKLSPLDKP; |
| GKLSPLDKPS; | KLSPLDKPSY; | LSPLDKPSYD; | SPLDKPSYDD; |
| PLDKPSYDDI; | LDKPSYDDID; | DKPSYDDIDS; | KPSYDDIDSK; |
| PSYDDIDSKE; | SYDDIDSKEE; | YDDIDSKEEV; | DDIDSKEEVD; |
| DIDSKEEVDN; | IDSKEEVDNK; | DSKEEVDNKA; | SKEEVDNKAI; |
| KEEVDNKAIN; | EEVDNKAINL; | EVDNKAINLQ; | VDNKAINLQK; |

DNKAINLQKI; NKAINLQKID; KAINLQKIDP; AINLQKIDPK;
INLQKIDPKV; NLQKIDPKVK; LQKIDPKVKD; QKIDPKVKDQ;
KIDPKVKDQT; IDPKVKDQTT; DPKVKDQTTS; PKVKDQTTSL;
KVKDQTTSLN; VKDQTTSLNE; KDQTTSLNED; DQTTSLNEDL;
QTTSLNEDLD; TTSLNEDLDK; TSLNEDLDKD; SLNEDLDKDL;
LNEDLDKDLT; NEDLDKDLTT; EDLDKDLTTM; DLDKDLTTMS;
LDKDLTTMSI; DKDLTTMSID; KDLTTMSIDS; DLTTMSIDSS;
LTTMSIDSSS; TTMSIDSSSP; TMSIDSSSPV; MSIDSSSPVF;
SIDSSSPVFL; IDSSSPVFLE; DSSSPVFLEV; SSSPVFLEVI;
SSPVFLEVID; SPVFLEVIDP; PVFLEVIDPI; VFLEVIDPIT;
FLEVIDPITN; LEVIDPITNL; EVIDPITNLG; VIDPITNLGT;
IDPITNLGTL; DPITNLGTLQ; PITNLGTLQL; ITNLGTLQLI;
TNLGTLQLID; NLGTLQLIDL; LGTLQLIDLN; GTLQLIDLNT;
TLQLIDLNTG; LQLIDLNTGV; QLIDLNTGVR; LIDLNTGVRL;
IDLNTGVRLK; DLNTGVRLKE; LNTGVRLKES; NTGVRLKEST;
TGVRLKESTQ; GVRLKESTQQ; VRLKESTQQG; RLKESTQQGI;
LKESTQQGIQ; KESTQQGIQR; ESTQQGIQRY; STQQGIQRYG;
TQQGIQRYGI; QQGIQRYGIY; QGIQRYGIYE; GIQRYGIYER;
IQRYGIYERE; QRYGIYEREK; RYGIYEREKD; YGIYEREKDL;
GIYEREKDLV; IYEREKDLVV; YEREKDLVVI; EREKDLVVIK;
REKDLVVIKM; EKDLVVIKMD; KDLVVIKMDS; DLVVIKMDSG;
LVVIKMDSGK; VVIKMDSGKA; VIKMDSGKAK; IKMDSGKAKL;
KMDSGKAKLQ; MDSGKAKLQI; DSGKAKLQIL; SGKAKLQILN;
GKAKLQILNK; KAKLQILNKL; AKLQILNKLE; KLQILNKLEN;
LQILNKLENL; QILNKLENLK; ILNKLENLKV; LNKLENLKVV;
NKLENLKVVS; KLENLKVVSE; LENLKVVSES; ENLKVVSESN;
NLKVVSESNF; LKVVSESNFE; KVVSESNFEI; VVSESNFEIN;
VSESNFEINK; SESNFEINKN; ESNFEINKNS; SNFEINKNSS;
NFEINKNSSL; FEINKNSSLY; EINKNSSLYV; INKNSSLYVD;
NKNSSLYVDS; KNSSLYVDSK; NSSLYVDSKM; SSLYVDSKMI;
SLYVDSKMIL; LYVDSKMILA; YVDSKMILAA; VDSKMILAAV;
DSKMILAAVR; SKMILAAVRD; KMILAAVRDK; MILAAVRDKD;
ILAAVRDKDD; LAAVRDKDDS; AAVRDKDDSN; AVRDKDDSNA;
VRDKDDSNAW; RDKDDSNAWR; DKDDSNAWRL; KDDSNAWRLA;
DDSNAWRLAK; DSNAWRLAKF; SNAWRLAKFS; NAWRLAKFSP;
AWRLAKFSPK; WRLAKFSPKN; RLAKFSPKNL; LAKFSPKNLD;
AKFSPKNLDE; KFSPKNLDEF; FSPKNLDEFI; SPKNLDEFIL;
PKNLDEFILS; KNLDEFILSE; NLDEFILSEN; LDEFILSENK;
DEFILSENKI; EFILSENKIL; FILSENKILP; ILSENKILPF;
LSENKILPFT; SENKILPFTS; ENKILPFTSF; NKILPFTSFS;
KILPFTSFSV; ILPFTSFSVR; LPFTSFSVRK; PFTSFSVRKN;
FTSFSVRKNF; TSFSVRKNFI; SFSVRKNFIY; FSVRKNFIYL;
SVRKNFIYLQ; VRKNFIYLQD; RKNFIYLQDE; KNFIYLQDEL;
NFIYLQDELK; FIYLQDELKN; IYLQDELKNL; YLQDELKNLV;
LQDELKNLVI; QDELKNLVIL; DELKNLVILD; ELKNLVILDV;
LKNLVILDVN; KNLVILDVNT; NLVILDVNTL; LVILDVNTLK;
VILDVNTLKK; ILDVNTLKKV; LDVNTLKKVK;

11 mers:
MKKMLLIFSFF; KKMLLIFSFFL; KMLLIFSFFLI; MLLIFSFFLIF;
LLIFSFFLIFL; LIFSFFLIFLN; IFSFFLIFLNG; FSFFLIFLNGF;

| | | | |
|---|---|---|---|
| SFFLIFLNGFP; | FFLIFLNGFPL; | FLIFLNGFPLN; | LIFLNGFPLNA; |
| IFLNGFPLNAR; | FLNGFPLNARK; | LNGFPLNARKV; | NGFPLNARKVD; |
| GFPLNARKVDK; | FPLNARKVDKE; | PLNARKVDKEK; | LNARKVDKEKL; |
| NARKVDKEKLK; | ARKVDKEKLKD; | RKVDKEKLKDF; | KVDKEKLKDFV; |
| VDKEKLKDFVN; | DKEKLKDFVNM; | KEKLKDFVNMD; | EKLKDFVNMDL; |
| KLKDFVNMDLE; | LKDFVNMDLEF; | KDFVNMDLEFV; | DFVNMDLEFVN; |
| FVNMDLEFVNY; | VNMDLEFVNYK; | NMDLEFVNYKG; | MDLEFVNYKGP; |
| DLEFVNYKGPY; | LEFVNYKGPYD; | EFVNYKGPYDS; | FVNYKGPYDST; |
| VNYKGPYDSTN; | NYKGPYDSTNT; | YKGPYDSTNTY; | KGPYDSTNTYE; |
| GPYDSTNTYEQ; | PYDSTNTYEQI; | YDSTNTYEQIV; | DSTNTYEQIVG; |
| STNTYEQIVGI; | TNTYEQIVGIG; | NTYEQIVGIGE; | TYEQIVGIGEF; |
| YEQIVGIGEFL; | EQIVGIGEFLA; | QIVGIGEFLAR; | IVGIGEFLARP; |
| VGIGEFLARPL; | GIGEFLARPLT; | IGEFLARPLTN; | GEFLARPLTNS; |
| EFLARPLTNSN; | FLARPLTNSNS; | LARPLTNSNSN; | ARPLTNSNSNS; |
| RPLTNSNSNSS; | PLTNSNSNSSY; | LTNSNSNSSYY; | TNSNSNSSYYG; |
| NSNSNSSYYGK; | SNSNSSYYGKY; | NSNSSYYGKYF; | SNSSYYGKYFI; |
| NSSYYGKYFIN; | SSYYGKYFINR; | SYYGKYFINRF; | YYGKYFINRFI; |
| YGKYFINRFID; | GKYFINRFIDD; | KYFINRFIDDQ; | YFINRFIDDQD; |
| FINRFIDDQDK; | INRFIDDQDKK; | NRFIDDQDKKA; | RFIDDQDKKAS; |
| FIDDQDKKASV; | IDDQDKKASVD; | DDQDKKASVDV; | DQDKKASVDVF; |
| QDKKASVDVFS; | DKKASVDVFSI; | KKASVDVFSIS; | KASVDVFSISS; |
| ASVDVFSISSK; | SVDVFSISSKS; | VDVFSISSKSE; | DVFSISSKSEL; |
| VFSISSKSELD; | FSISSKSELDS; | SISSKSELDSI; | ISSKSELDSIL; |
| SSKSELDSILN; | SKSELDSILNL; | KSELDSILNLR; | SELDSILNLRR; |
| ELDSILNLRRI; | LDSILNLRRIL; | DSILNLRRILT; | SILNLRRILTG; |
| ILNLRRILTGY; | LNLRRILTGYI; | NLRRILTGYII; | LRRILTGYIIK; |
| RRILTGYIIKS; | RILTGYIIKSF; | ILTGYIIKSFD; | LTGYIIKSFDY; |
| TGYIIKSFDYD; | GYIIKSFDYDR; | YIIKSFDYDRS; | IIKSFDYDRSS; |
| IKSFDYDRSSA; | KSFDYDRSSAE; | SFDYDRSSAEL; | FDYDRSSAELI; |
| DYDRSSAELIA; | YDRSSAELIAK; | DRSSAELIAKV; | RSSAELIAKVI; |
| SSAELIAKVIT; | SAELIAKVITI; | AELIAKVITIY; | ELIAKVITIYN; |
| LIAKVITIYNA; | IAKVITIYNAV; | AKVITIYNAVY; | KVITIYNAVYR; |
| VITIYNAVYRG; | ITIYNAVYRGD; | TIYNAVYRGDL; | IYNAVYRGDLD; |
| YNAVYRGDLDY; | NAVYRGDLDYY; | AVYRGDLDYYK; | VYRGDLDYYKG; |
| YRGDLDYYKGF; | RGDLDYYKGFY; | GDLDYYKGFYI; | DLDYYKGFYIE; |
| LDYYKGFYIEP; | DYYKGFYIEPA; | YYKGFYIEPAL; | YKGFYIEPALK; |
| KGFYIEPALKS; | GFYIEPALKSL; | FYIEPALKSLT; | YIEPALKSLTK; |
| IEPALKSLTKE; | EPALKSLTKEN; | PALKSLTKENA; | ALKSLTKENAG; |
| LKSLTKENAGL; | KSLTKENAGLS; | SLTKENAGLSR; | LTKENAGLSRV; |
| TKENAGLSRVY; | KENAGLSRVYS; | ENAGLSRVYSQ; | NAGLSRVYSQW; |
| AGLSRVYSQWA; | GLSRVYSQWAG; | LSRVYSQWAGK; | SRVYSQWAGKT; |
| RVYSQWAGKTQ; | VYSQWAGKTQI; | YSQWAGKTQIF; | SQWAGKTQIFI; |
| QWAGKTQIFIP; | WAGKTQIFIPL; | AGKTQIFIPLK; | GKTQIFIPLKK; |
| KTQIFIPLKKD; | TQIFIPLKKDI; | QIFIPLKKDIL; | IFIPLKKDILS; |
| FIPLKKDILSG; | IPLKKDILSGN; | PLKKDILSGNI; | LKKDILSGNIE; |
| KKDILSGNIES; | KDILSGNIESD; | DILSGNIESDI; | ILSGNIESDID; |
| LSGNIESDIDI; | SGNIESDIDID; | GNIESDIDIDS; | NIESDIDIDSL; |
| IESDIDIDSLV; | ESDIDIDSLVT; | SDIDIDSLVTD; | DIDIDSLVTDK; |
| IDIDSLVTDKV; | DIDSLVTDKVI; | IDSLVTDKVIA; | DSLVTDKVIAA; |
| SLVTDKVIAAL; | LVTDKVIAALL; | VTDKVIAALLS; | TDKVIAALLSE; |
| DKVIAALLSEN; | KVIAALLSENE; | VIAALLSENEA; | IAALLSENEAG; |

| | AALLSENEAGV; ALLSENEAGVN; LLSENEAGVNF; LSENEAGVNFA; SENEAGVNFAR; ENEAGVNFARD; NEAGVNFARDI; EAGVNFARDIT; AGVNFARDITD; GVNFARDITDI; VNFARDITDIQ; NFARDITDIQG; FARDITDIQGE; ARDITDIQGET; RDITDIQGETH; DITDIQGETHK; ITDIQGETHKA; TDIQGETHKAD; DIQGETHKADQ; IQGETHKADQD; QGETHKADQDK; GETHKADQDKI; ETHKADQDKID; THKADQDKIDT; HKADQDKIDTE; KADQDKIDTEL; ADQDKIDTELD; DQDKIDTELDN; QDKIDTELDNI; DKIDTELDNIH; KIDTELDNIHE; IDTELDNIHES; DTELDNIHESD; TELDNIHESDS; ELDNIHESDSN; LDNIHESDSNI; DNIHESDSNIT; NIHESDSNITE; IHESDSNITET; HESDSNITETI; ESDSNITETIE; SDSNITETIEN; DSNITETIENL; SNITETIENLR; NITETIENLRD; ITETIENLRDQ; TETIENLRDQL; ETIENLRDQLE; TIENLRDQLEK; IENLRDQLEKA; ENLRDQLEKAT; NLRDQLEKATD; LRDQLEKATDE; RDQLEKATDEE; DQLEKATDEEH; QLEKATDEEHK; LEKATDEEHKK; EKATDEEHKKE; KATDEEHKKEI; ATDEEHKKEIE; TDEEHKKEIES; DEEHKKEIESQ; EEHKKEIESQV; EHKKEIESQVD; HKKEIESQVDA; KKEIESQVDAK; KEIESQVDAKK; EIESQVDAKKK; IESQVDAKKKE; ESQVDAKKKEK; SQVDAKKKEKE; QVDAKKKEKEE; VDAKKKEKEEL; DAKKKEKEELD; AKKKEKEELDK; KKKEKEELDKK; KKEKEELDKKA; KEKEELDKKAI; EKEELDKKAIN; KEELDKKAINL; EELDKKAINLD; ELDKKAINLDK; LDKKAINLDKA; DKKAINLDKAQ; KKAINLDKAQQ; KAINLDKAQQK; AINLDKAQQKL; INLDKAQQKLD; NLDKAQQKLDS; LDKAQQKLDSA; DKAQQKLDSAE; KAQQKLDSAED; AQQKLDSAEDN; QQKLDSAEDNL; QKLDSAEDNLD; KLDSAEDNLDV; LDSAEDNLDVQ; DSAEDNLDVQR; SAEDNLDVQRD; AEDNLDVQRDT; EDNLDVQRDTV; DNLDVQRDTVR; NLDVQRDTVRE; LDVQRDTVREK; DVQRDTVREKI; VQRDTVREKIQ; QRDTVREKIQE; RDTVREKIQED; DTVREKIQEDI; TVREKIQEDIN; VREKIQEDINE; REKIQEDINEI; EKIQEDINEIN; KIQEDINEINK; IQEDINEINKE; QEDINEINKEK; EDINEINKEKN; DINEINKEKNL; INEINKEKNLP; NEINKEKNLPK; EINKEKNLPKP; INKEKNLPKPG; NKEKNLPKPGD; KEKNLPKPGDV; EKNLPKPGDVS; KNLPKPGDVSS; NLPKPGDVSSP; LPKPGDVSSPK; PKPGDVSSPKV; KPGDVSSPKVD; PGDVSSPKVDK; GDVSSPKVDKQ; DVSSPKVDKQL; VSSPKVDKQLQ; SSPKVDKQLQI; SPKVDKQLQIK; PKVDKQLQIKE; KVDKQLQIKES; VDKQLQIKESL; DKQLQIKESLE; KQLQIKESLED; QLQIKESLEDL; LQIKESLEDLQ; QIKESLEDLQE; IKESLEDLQEQ; KESLEDLQEQL; ESLEDLQEQLK; SLEDLQEQLKE; LEDLQEQLKEA; EDLQEQLKEAG; DLQEQLKEAGD; LQEQLKEAGDE; QEQLKEAGDEN; EQLKEAGDENQ; QLKEAGDENQK; LKEAGDENQKR; KEAGDENQKRE; EAGDENQKREI; AGDENQKREIE; GDENQKREIEK; DENQKREIEKQ; ENQKREIEKQI; NQKREIEKQIE; QKREIEKQIEI; KREIEKQIEIK; REIEKQIEIKK; EIEKQIEIKKR; IEKQIEIKKRD; EKQIEIKKRDE; KQIEIKKRDEE; QIEIKKRDEEL; IEIKKRDEELL; EIKKRDEELLK; IKKRDEELLKS; KKRDEELLKSK; KRDEELLKSKD; RDEELLKSKDG; DEELLKSKDGK; EELLKSKDGKV; ELLKSKDGKVS; LLKSKDGKVSK; LKSKDGKVSKD; KSKDGKVSKDY; SKDGKVSKDYE; KDGKVSKDYEA; DGKVSKDYEAL; GKVSKDYEALD; KVSKDYEALDL; VSKDYEALDLD; SKDYEALDLDR; KDYEALDLDRE; DYEALDLDREL; YEALDLDRELS; EALDLDRELSK; ALDLDRELSKA; LDLDRELSKAS; DLDRELSKASS; LDRELSKASSK; DRELSKASSKE; RELSKASSKEK; ELSKASSKEKS; LSKASSKEKSK; SKASSKEKSKV; KASSKEKSKVK; |
|---|---|

| | | | |
|---|---|---|---|
| ASSKEKSKVKE; | SSKEKSKVKEE; | SKEKSKVKEEE; | KEKSKVKEEEI; |
| EKSKVKEEEIT; | KSKVKEEEITK; | SKVKEEEITKG; | KVKEEEITKGK; |
| VKEEEITKGKS; | KEEEITKGKSR; | EEEITKGKSRA; | EEITKGKSRAS; |
| EITKGKSRASL; | ITKGKSRASLG; | TKGKSRASLGD; | KGKSRASLGDL; |
| GKSRASLGDLN; | KSRASLGDLNN; | SRASLGDLNND; | RASLGDLNNDK; |
| ASLGDLNNDKN; | SLGDLNNDKNL; | LGDLNNDKNLM; | GDLNNDKNLML; |
| DLNNDKNLMLP; | LNNDKNLMLPE; | NNDKNLMLPED; | NDKNLMLPEDQ; |
| DKNLMLPEDQK; | KNLMLPEDQKL; | NLMLPEDQKLP; | LMLPEDQKLPE; |
| MLPEDQKLPED; | LPEDQKLPEDK; | PEDQKLPEDKK; | EDQKLPEDKKL; |
| DQKLPEDKKLD; | QKLPEDKKLDS; | KLPEDKKLDSK; | LPEDKKLDSKL; |
| PEDKKLDSKLD; | EDKKLDSKLDG; | DKKLDSKLDGK; | KKLDSKLDGKK; |
| KLDSKLDGKKE; | LDSKLDGKKEF; | DSKLDGKKEFK; | SKLDGKKEFKP; |
| KLDGKKEFKPV; | LDGKKEFKPVS; | DGKKEFKPVSE; | GKKEFKPVSEV; |
| KKEFKPVSEVE; | KEFKPVSEVEK; | EFKPVSEVEKL; | FKPVSEVEKLD; |
| KPVSEVEKLDK; | PVSEVEKLDKI; | VSEVEKLDKIS; | SEVEKLDKISK; |
| EVEKLDKISKS; | VEKLDKISKSN; | EKLDKISKSNN; | KLDKISKSNNN; |
| LDKISKSNNNE; | DKISKSNNNEV; | KISKSNNNEVG; | ISKSNNNEVGK; |
| SKSNNNEVGKL; | KSNNNEVGKLS; | SNNNEVGKLSP; | NNNEVGKLSPL; |
| NNEVGKLSPLD; | NEVGKLSPLDK; | EVGKLSPLDKP; | VGKLSPLDKPS; |
| GKLSPLDKPSY; | KLSPLDKPSYD; | LSPLDKPSYDD; | SPLDKPSYDDI; |
| PLDKPSYDDID; | LDKPSYDDIDS; | DKPSYDDIDSK; | KPSYDDIDSKE; |
| PSYDDIDSKEE; | SYDDIDSKEEV; | YDDIDSKEEVD; | DDIDSKEEVDN; |
| DIDSKEEVDNK; | IDSKEEVDNKA; | DSKEEVDNKAI; | SKEEVDNKAIN; |
| KEEVDNKAINL; | EEVDNKAINLQ; | EVDNKAINLQK; | VDNKAINLQKI; |
| DNKAINLQKID; | NKAINLQKIDP; | KAINLQKIDPK; | AINLQKIDPKV; |
| INLQKIDPKVK; | NLQKIDPKVKD; | LQKIDPKVKDQ; | QKIDPKVKDQT; |
| KIDPKVKDQTT; | IDPKVKDQTTS; | DPKVKDQTTSL; | PKVKDQTTSLN; |
| KVKDQTTSLNE; | VKDQTTSLNED; | KDQTTSLNEDL; | DQTTSLNEDLD; |
| QTTSLNEDLDK; | TTSLNEDLDKD; | TSLNEDLDKDL; | SLNEDLDKDLT; |
| LNEDLDKDLTT; | NEDLDKDLTTM; | EDLDKDLTTMS; | DLDKDLTTMSI; |
| LDKDLTTMSID; | DKDLTTMSIDS; | KDLTTMSIDSS; | DLTTMSIDSSS; |
| LTTMSIDSSSP; | TTMSIDSSSPV; | TMSIDSSSPVF; | MSIDSSSPVFL; |
| SIDSSSPVFLE; | IDSSSPVFLEV; | DSSSPVFLEVI; | SSSPVFLEVID; |
| SSPVFLEVIDP; | SPVFLEVIDPI; | PVFLEVIDPIT; | VFLEVIDPITN; |
| FLEVIDPITNL; | LEVIDPITNLG; | EVIDPITNLGT; | VIDPITNLGTL; |
| IDPITNLGTLQ; | DPITNLGTLQL; | PITNLGTLQLI; | ITNLGTLQLID; |
| TNLGTLQLIDL; | NLGTLQLIDLN; | LGTLQLIDLNT; | GTLQLIDLNTG; |
| TLQLIDLNTGV; | LQLIDLNTGVR; | QLIDLNTGVRL; | LIDLNTGVRLK; |
| IDLNTGVRLKE; | DLNTGVRLKES; | LNTGVRLKEST; | NTGVRLKESTQ; |
| TGVRLKESTQQ; | GVRLKESTQQG; | VRLKESTQQGI; | RLKESTQQGIQ; |
| LKESTQQGIQR; | KESTQQGIQRY; | ESTQQGIQRYG; | STQQGIQRYGI; |
| TQQGIQRYGIY; | QQGIQRYGIYE; | QGIQRYGIYER; | GIQRYGIYERE; |
| IQRYGIYEREK; | QRYGIYEREKD; | RYGIYEREKDL; | YGIYEREKDLV; |
| GIYEREKDLVV; | IYEREKDLVVI; | YEREKDLVVIK; | EREKDLVVIKM; |
| REKDLVVIKMD; | EKDLVVIKMDS; | KDLVVIKMDSG; | DLVVIKMDSGK; |
| LVVIKMDSGKA; | VVIKMDSGKAK; | VIKMDSGKAKL; | IKMDSGKAKLQ; |
| KMDSGKAKLQI; | MDSGKAKLQIL; | DSGKAKLQILN; | SGKAKLQILNK; |
| GKAKLQILNKL; | KAKLQILNKLE; | AKLQILNKLEN; | KLQILNKLENL; |
| LQILNKLENLK; | QILNKLENLKV; | ILNKLENLKVV; | LNKLENLKVVS; |
| NKLENLKVVSE; | KLENLKVVSES; | LENLKVVSESN; | ENLKVVSESNF; |
| NLKVVSESNFE; | LKVVSESNFEI; | KVVSESNFEIN; | VVSESNFEINK; |

| | |
|---|---|
| | VSESNFEINKN; SESNFEINKNS; ESNFEINKNSS; SNFEINKNSSL; NFEINKNSSLY; FEINKNSSLYV; EINKNSSLYVD; INKNSSLYVDS; NKNSSLYVDSK; KNSSLYVDSKM; NSSLYVDSKMI; SSLYVDSKMIL; SLYVDSKMILA; LYVDSKMILAA; YVDSKMILAAV; VDSKMILAAVR; DSKMILAAVRD; SKMILAAVRDK; KMILAAVRDKD; MILAAVRDKDD; ILAAVRDKDDS; LAAVRDKDDSN; AAVRDKDDSNA; AVRDKDDSNAW; VRDKDDSNAWR; RDKDDSNAWRL; DKDDSNAWRLA; KDDSNAWRLAK; DDSNAWRLAKF; DSNAWRLAKFS; SNAWRLAKFSP; NAWRLAKFSPK; AWRLAKFSPKN; WRLAKFSPKNL; RLAKFSPKNLD; LAKFSPKNLDE; AKFSPKNLDEF; KFSPKNLDEFI; FSPKNLDEFIL; SPKNLDEFILS; PKNLDEFILSE; KNLDEFILSEN; NLDEFILSENK; LDEFILSENKI; DEFILSENKIL; EFILSENKILP; FILSENKILPF; ILSENKILPFT; LSENKILPFTS; SENKILPFTSF; ENKILPFTSFS; NKILPFTSFSV; KILPFTSFSVR; ILPFTSFSVRK; LPFTSFSVRKN; PFTSFSVRKNF; FTSFSVRKNFI; TSFSVRKNFIY; SFSVRKNFIYL; FSVRKNFIYLQ; SVRKNFIYLQD; VRKNFIYLQDE; RKNFIYLQDEL; KNFIYLQDELK; NFIYLQDELKN; FIYLQDELKNL; IYLQDELKNLV; YLQDELKNLVI; LQDELKNLVIL; QDELKNLVILD; DELKNLVILDV; ELKNLVILDVN; LKNLVILDVNT; KNLVILDVNTL; NLVILDVNTLK; LVILDVNTLKK; VILDVNTLKKV; ILDVNTLKKVK; |
| Seq 27 | SEQ ID NO 31564–33623/ 8 mers: MKYILSID; KYILSIDQ; YILSIDQG; ILSIDQGT; LSIDQGTT; SIDQGTTS; IDQGTTSS; DQGTTSSR; QGTTSSRA; GTTSSRAM; TTSSRAMV; TSSRAMVF; SSRAMVFD; SRAMVFDK; RAMVFDKN; AMVFDKNA; MVFDKNAN; VFDKNANI; FDKNANIK; DKNANIKG; KNANIKGF; NANIKGFA; ANIKGFAQ; NIKGFAQK; IKGFAQKE; KGFAQKEF; GFAQKEFT; FAQKEFTQ; AQKEFTQI; QKEFTQIY; KEFTQIYP; EFTQIYPQ; FTQIYPQP; TQIYPQPS; QIYPQPSW; IYPQPSWV; YPQPSWVE; PQPSWVEH; QPSWVEHD; PSWVEHDP; SWVEHDPT; WVEHDPTE; VEHDPTEI; EHDPTEIW; HDPTEIWG; DPTEIWGS; PTEIWGSQ; TEIWGSQL; EIWGSQLG; IWGSQLGV; WGSQLGVI; GSQLGVIT; SQLGVITE; QLGVITEA; LGVITEAM; GVITEAMA; VITEAMAN; ITEAMANA; TEAMANAR; EAMANARI; AMANARIL; MANARILP; ANARILPN; NARILPNE; ARILPNEI; RILPNEID; ILPNEIDA; LPNEIDAI; PNEIDAIG; NEIDAIGI; EIDAIGIT; IDAIGITN; DAIGITNQ; AIGITNQR; IGITNQRE; GITNQRET; ITNQRETT; TNQRETTV; NQRETTVI; QRETTVIW; RETTVIWE; ETTVIWEK; TTVIWEKN; TVIWEKNT; VIWEKNTG; IWEKNTGK; WEKNTGKP; EKNTGKPI; KNTGKPIY; NTGKPIYN; TGKPIYNA; GKPIYNAI; KPIYNAIV; PIYNAIVW; IYNAIVWQ; YNAIVWQD; NAIVWQDR; AIVWQDRR; IVWQDRRT; VWQDRRTA; WQDRRTAK; QDRRTAKI; DRRTAKIC; RRTAKICD; RTAKICDQ; TAKICDQL; AKICDQLK; KICDQLKK; ICDQLKKE; CDQLKKEG; DQLKKEGK; QLKKEGKD; LKKEGKDK; KKEGKDKI; KEGKDKII; EGKDKIIL; GKDKIILE; KDKIILEK; DKIILEKT; KIILEKTG; IILEKTGL; ILEKTGLV; LEKTGLVL; EKTGLVLD; KTGLVLDS; TGLVLDSY; GLVLDSYF; LVLDSYFS; VLDSYFSG; LDSYFSGT; DSYFSGTK; SYFSGTKI; YFSGTKIM; FSGTKIMW; SGTKIMWI; GTKIMWIL; TKIMWILD; KIMWILDN; IMWILDNV; MWILDNVE; |

WILDNVEG; ILDNVEGA; LDNVEGAR; DNVEGARQ; NVEGARQR;
VEGARQRA; EGARQRAE; GARQRAEN; ARQRAENG; RQRAENGE;
QRAENGEL; RAENGELC; AENGELCF; ENGELCFG; NGELCFGT;
GELCFGTI; ELCFGTID; LCFGTIDT; CFGTIDTW; FGTIDTWI;
GTIDTWIL; TIDTWILW; IDTWILWN; DTWILWNL; TWILWNLT;
WILWNLTQ; ILWNLTQK; LWNLTQKK; WNLTQKKE; NLTQKKEH;
LTQKKEHA; TQKKEHAT; QKKEHATD; KKEHATDY; KEHATDYS;
EHATDYSN; HATDYSNA; ATDYSNAS; TDYSNASR; DYSNASRT;
YSNASRTL; SNASRTLL; NASRTLLL; ASRTLLLN; SRTLLLNI;
RTLLLNIK; TLLLNIKT; LLLNIKTL; LLNIKTLE; LNIKTLEW;
NIKTLEWD; IKTLEWDD; KTLEWDDE; TLEWDDEL; LEWDDELL;
EWDDELLS; WDDELLSI; DDELLSIL; DELLSILN; ELLSILNV;
LLSILNVP; LSILNVPR; SILNVPRA; ILNVPRAI; LNVPRAIL;
NVPRAILP; VPRAILPE; PRAILPEL; RAILPELK; AILPELKE;
ILPELKES; LPELKESS; PELKESST; ELKESSTI; LKESSTIY;
KESSTIYG; ESSTIYGK; SSTIYGKT; STIYGKTD; TIYGKTDK;
IYGKTDKA; YGKTDKAL; GKTDKALF; KTDKALFG; TDKALFGA;
DKALFGAE; KALFGAEI; ALFGAEIP; LFGAEIPI; FGAEIPIA;
GAEIPIAG; AEIPIAGI; EIPIAGIA; IPIAGIAG; PIAGIAGD;
IAGIAGDQ; AGIAGDQF; GIAGDQFA; IAGDQFAA; AGDQFAAT;
GDQFAATF; DQFAATFG; QFAATFGQ; FAATFGQA; AATFGQAC;
ATFGQACL; TFGQACLK; FGQACLKK; GQACLKKG; QACLKKGM;
ACLKKGMA; CLKKGMAK; LKKGMAKN; KKGMAKNT; KGMAKNTY;
GMAKNTYG; MAKNTYGT; AKNTYGTG; KNTYGTGC; NTYGTGCF;
TYGTGCFL; YGTGCFLT; GTGCFLTV; TGCFLTVN; GCFLTVNI;
CFLTVNIG; FLTVNIGK; LTVNIGKE; TVNIGKEP; VNIGKEPI;
NIGKEPII; IGKEPIIS; GKEPIISH; KEPIISHD; EPIISHDK;
PIISHDKL; IISHDKLL; ISHDKLLT; SHDKLLTS; HDKLLTSI;
DKLLTSIA; KLLTSIAW; LLTSIAWG; LTSIAWGR; TSIAWGRK;
SIAWGRKK; IAWGRKKS; AWGRKKSV; WGRKKSVT; GRKKSVTY;
RKKSVTYV; KKSVTYVL; KSVTYVLE; SVTYVLEG; VTYVLEGS;
TYVLEGSV; YVLEGSVF; VLEGSVFI; LEGSVFIG; EGSVFIGG;
GSVFIGGA; SVFIGGAV; VFIGGAVI; FIGGAVIQ; IGGAVIQW;
GGAVIQWL; GAVIQWLR; AVIQWLRD; VIQWLRDG; IQWLRDGL;
QWLRDGLE; WLRDGLEF; LRDGLEFF; RDGLEFFR; DGLEFFRK;
GLEFFRKS; LEFFRKSS; EFFRKSSD; FFRKSSDA; FRKSSDAE;
RKSSDAEA; KSSDAEAL; SSDAEALA; SDAEALAS; DAEALASS;
AEALASSV; EALASSVS; ALASSVSD; LASSVSDN; ASSVSDNG;
SSVSDNGG; SVSDNGGV; VSDNGGVY; SDNGGVYF; DNGGVYFV;
NGGVYFVP; GGVYFVPA; GVYFVPAF; VYFVPAFV; YFVPAFVG;
FVPAFVGL; VPAFVGLG; PAFVGLGA; AFVGLGAP; FVGLGAPH;
VGLGAPHW; GLGAPHWD; LGAPHWDS; GAPHWDSY; APHWDSYA;
PHWDSYAR; HWDSYARG; WDSYARGT; DSYARGTI; SYARGTII;
YARGTIIG; ARGTIIGI; RGTIIGIT; GTIIGITR; TIIGITRG;
IIGITRGS; IGITRGST; GITRGSTK; ITRGSTKA; TRGSTKAH;
RGSTKAHI; GSTKAHIT; STKAHITR; TKAHITRA; KAHITRAA;
AHITRAAL; HITRAALE; ITRAALES; TRAALESI; RAALESIA;
AALESIAF; ALESIAFQ; LESIAFQS; ESIAFQSF; SIAFQSFD;
IAFQSFDI; AFQSFDIL; FQSFDILN; QSFDILNT; SFDILNTM;
FDILNTMK; DILNTMKK; ILNTMKKS; LNTMKKSI; NTMKKSIP;
TMKKSIPN; MKKSIPNF; KKSIPNFE; KSIPNFEI; SIPNFEIQ;

IPNFEIQE; PNFEIQEL; NFEIQELR; FEIQELRV; EIQELRVD;
IQELRVDG; QELRVDGG; ELRVDGGA; LRVDGGAS; RVDGGASQ;
VDGGASQN; DGGASQNN; GGASQNNL; GASQNNLL; ASQNNLLM;
SQNNLLMQ; QNNLLMQF; NNLLMQFQ; NLLMQFQA; LLMQFQAD;
LMQFQADL; MQFQADLL; QFQADLLE; FQADLLEC; QADLLECK;
ADLLECKV; DLLECKVV; LLECKVVR; LECKVVRP; ECKVVRPK;
CKVVRPKI; KVVRPKIT; VVRPKITE; VRPKITET; RPKITETT;
PKITETTA; KITETTAL; ITETTALG; TETTALGA; ETTALGAA;
TTALGAAY; TALGAAYL; ALGAAYLA; LGAAYLAG; GAAYLAGL;
AAYLAGLA; AYLAGLAT; YLAGLATG; LAGLATGY; AGLATGYW;
GLATGYWQ; LATGYWQS; ATGYWQSA; TGYWQSAE; GYWQSAEE;
YWQSAEEI; WQSAEEIV; QSAEEIVS; SAEEIVSL; AEEIVSLW;
EEIVSLWQ; EIVSLWQV; IVSLWQVD; VSLWQVDK; SLWQVDKI;
LWQVDKIF; WQVDKIFE; QVDKIFEP; VDKIFEPS; DKIFEPSM;
KIFEPSMP; IFEPSMPK; FEPSMPKN; EPSMPKNQ; PSMPKNQK;
SMPKNQKE; MPKNQKEK; PKNQKEKL; KNQKEKLL; NQKEKLLE;
QKEKLLEN; KEKLLENW; EKLLENWN; KLLENWNK; LLENWNKA;
LENWNKAV; ENWNKAVG; NWNKAVGK; WNKAVGKA; NKAVGKAK;
KAVGKAKS; AVGKAKSW; VGKAKSWI; GKAKSWIQ; KAKSWIQN;
AKSWIQNS; KSWIQNSH; SWIQNSHS; WIQNSHSS;

9 mers:
MKYILSIDQ; KYILSIDQG; YILSIDQGT; ILSIDQGTT; LSIDQGTTS;
SIDQGTTSS; IDQGTTSSR; DQGTTSSRA; QGTTSSRAM; GTTSSRAMV;
TTSSRAMVF; TSSRAMVFD; SSRAMVFDK; SRAMVFDKN; RAMVFDKNA;
AMVFDKNAN; MVFDKNANI; VFDKNANIK; FDKNANIKG; DKNANIKGF;
KNANIKGFA; NANIKGFAQ; ANIKGFAQK; NIKGFAQKE; IKGFAQKEF;
KGFAQKEFT; GFAQKEFTQ; FAQKEFTQI; AQKEFTQIY; QKEFTQIYP;
KEFTQIYPQ; EFTQIYPQP; FTQIYPQPS; TQIYPQPSW; QIYPQPSWV;
IYPQPSWVE; YPQPSWVEH; PQPSWVEHD; QPSWVEHDP; PSWVEHDPT;
SWVEHDPTE; WVEHDPTEI; VEHDPTEIW; EHDPTEIWG; HDPTEIWGS;
DPTEIWGSQ; PTEIWGSQL; TEIWGSQLG; EIWGSQLGV; IWGSQLGVI;
WGSQLGVIT; GSQLGVITE; SQLGVITEA; QLGVITEAM; LGVITEAMA;
GVITEAMAN; VITEAMANA; ITEAMANAR; TEAMANARI; EAMANARIL;
AMANARILP; MANARILPN; ANARILPNE; NARILPNEI; ARILPNEID;
RILPNEIDA; ILPNEIDAI; LPNEIDAIG; PNEIDAIGI; NEIDAIGIT;
EIDAIGITN; IDAIGITNQ; DAIGITNQR; AIGITNQRE; IGITNQRET;
GITNQRETT; ITNQRETTV; TNQRETTVI; NQRETTVIW; QRETTVIWE;
RETTVIWEK; ETTVIWEKN; TTVIWEKNT; TVIWEKNTG; VIWEKNTGK;
IWEKNTGKP; WEKNTGKPI; EKNTGKPIY; KNTGKPIYN; NTGKPIYNA;
TGKPIYNAI; GKPIYNAIV; KPIYNAIVW; PIYNAIVWQ; IYNAIVWQD;
YNAIVWQDR; NAIVWQDRR; AIVWQDRRT; IVWQDRRTA; VWQDRRTAK;
WQDRRTAKI; QDRRTAKIC; DRRTAKICD; RRTAKICDQ; RTAKICDQL;
TAKICDQLK; AKICDQLKK; KICDQLKKE; ICDQLKKEG; CDQLKKEGK;
DQLKKEGKD; QLKKEGKDK; LKKEGKDKI; KKEGKDKII; KEGKDKIIL;
EGKDKIILE; GKDKIILEK; KDKIILEKT; DKIILEKTG; KIILEKTGL;
IILEKTGLV; ILEKTGLVL; LEKTGLVLD; EKTGLVLDS; KTGLVLDSY;
TGLVLDSYF; GLVLDSYFS; LVLDSYFSG; VLDSYFSGT; LDSYFSGTK;
DSYFSGTKI; SYFSGTKIM; YFSGTKIMW; FSGTKIMWI; SGTKIMWIL;
GTKIMWILD; TKIMWILDN; KIMWILDNV; IMWILDNVE; MWILDNVEG;
WILDNVEGA; ILDNVEGAR; LDNVEGARQ; DNVEGARQR; NVEGARQRA;

| | VEGARQRAE; EGARQRAEN; GARQRAENG; ARQRAENGE; RQRAENGEL; |
|---|---|
| | QRAENGELC; RAENGELCF; AENGELCFG; ENGELCFGT; NGELCFGTI; |
| | GELCFGTID; ELCFGTIDT; LCFGTIDTW; CFGTIDTWI; FGTIDTWIL; |
| | GTIDTWILW; TIDTWILWN; IDTWILWNL; DTWILWNLT; TWILWNLTQ; |
| | WILWNLTQK; ILWNLTQKK; LWNLTQKKE; WNLTQKKEH; NLTQKKEHA; |
| | LTQKKEHAT; TQKKEHATD; QKKEHATDY; KKEHATDYS; KEHATDYSN; |
| | EHATDYSNA; HATDYSNAS; ATDYSNASR; TDYSNASRT; DYSNASRTL; |
| | YSNASRTLL; SNASRTLLL; NASRTLLLN; ASRTLLLNI; SRTLLLNIK; |
| | RTLLLNIKT; TLLLNIKTL; LLLNIKTLE; LLNIKTLEW; LNIKTLEWD; |
| | NIKTLEWDD; IKTLEWDDE; KTLEWDDEL; TLEWDDELL; LEWDDELLS; |
| | EWDDELLSI; WDDELLSIL; DDELLSILN; DELLSILNV; ELLSILNVP; |
| | LLSILNVPR; LSILNVPRA; SILNVPRAI; ILNVPRAIL; LNVPRAILP; |
| | NVPRAILPE; VPRAILPEL; PRAILPELK; RAILPELKE; AILPELKES; |
| | ILPELKESS; LPELKESST; PELKESSTI; ELKESSTIY; LKESSTIYG; |
| | KESSTIYGK; ESSTIYGKT; SSTIYGKTD; STIYGKTDK; TIYGKTDKA; |
| | IYGKTDKAL; YGKTDKALF; GKTDKALFG; KTDKALFGA; TDKALFGAE; |
| | DKALFGAEI; KALFGAEIP; ALFGAEIPI; LFGAEIPIA; FGAEIPIAG; |
| | GAEIPIAGI; AEIPIAGIA; EIPIAGIAG; IPIAGIAGD; PIAGIAGDQ; |
| | IAGIAGDQF; AGIAGDQFA; GIAGDQFAA; IAGDQFAAT; AGDQFAATF; |
| | GDQFAATFG; DQFAATFGQ; QFAATFGQA; FAATFGQAC; AATFGQACL; |
| | ATFGQACLK; TFGQACLKK; FGQACLKKG; GQACLKKGM; QACLKKGMA; |
| | ACLKKGMAK; CLKKGMAKN; LKKGMAKNT; KKGMAKNTY; KGMAKNTYG; |
| | GMAKNTYGT; MAKNTYGTG; AKNTYGTGC; KNTYGTGCF; NTYGTGCFL; |
| | TYGTGCFLT; YGTGCFLTV; GTGCFLTVN; TGCFLTVNI; GCFLTVNIG; |
| | CFLTVNIGK; FLTVNIGKE; LTVNIGKEP; TVNIGKEPI; VNIGKEPII; |
| | NIGKEPIIS; IGKEPIISH; GKEPIISHD; KEPIISHDK; EPIISHDKL; |
| | PIISHDKLL; IISHDKLLT; ISHDKLLTS; SHDKLLTSI; HDKLLTSIA; |
| | DKLLTSIAW; KLLTSIAWG; LLTSIAWGR; LTSIAWGRK; TSIAWGRKK; |
| | SIAWGRKKS; IAWGRKKSV; AWGRKKSVT; WGRKKSVTY; GRKKSVTYV; |
| | RKKSVTYVL; KKSVTYVLE; KSVTYVLEG; SVTYVLEGS; VTYVLEGSV; |
| | TYVLEGSVF; YVLEGSVFI; VLEGSVFIG; LEGSVFIGG; EGSVFIGGA; |
| | GSVFIGGAV; SVFIGGAVI; VFIGGAVIQ; FIGGAVIQW; IGGAVIQWL; |
| | GGAVIQWLR; GAVIQWLRD; AVIQWLRDG; VIQWLRDGL; IQWLRDGLE; |
| | QWLRDGLEF; WLRDGLEFF; LRDGLEFFR; RDGLEFFRK; DGLEFFRKS; |
| | GLEFFRKSS; LEFFRKSSD; EFFRKSSDA; FFRKSSDAE; FRKSSDAEA; |
| | RKSSDAEAL; KSSDAEALA; SSDAEALAS; SDAEALASS; DAEALASSV; |
| | AEALASSVS; EALASSVSD; ALASSVSDN; LASSVSDNG; ASSVSDNGG; |
| | SSVSDNGGV; SVSDNGGVY; VSDNGGVYF; SDNGGVYFV; DNGGVYFVP; |
| | NGGVYFVPA; GGVYFVPAF; GVYFVPAFV; VYFVPAFVG; YFVPAFVGL; |
| | FVPAFVGLG; VPAFVGLGA; PAFVGLGAP; AFVGLGAPH; FVGLGAPHW; |
| | VGLGAPHWD; GLGAPHWDS; LGAPHWDSY; GAPHWDSYA; APHWDSYAR; |
| | PHWDSYARG; HWDSYARGT; WDSYARGTI; DSYARGTII; SYARGTIIG; |
| | YARGTIIGI; ARGTIIGIT; RGTIIGITR; GTIIGITRG; TIIGITRGS; |
| | IIGITRGST; IGITRGSTK; GITRGSTKA; ITRGSTKAH; TRGSTKAHI; |
| | RGSTKAHIT; GSTKAHITR; STKAHITRA; TKAHITRAA; KAHITRAAL; |
| | AHITRAALE; HITRAALES; ITRAALESI; TRAALESIA; RAALESIAF; |
| | AALESIAFQ; ALESIAFQS; LESIAFQSF; ESIAFQSFD; SIAFQSFDI; |
| | IAFQSFDIL; AFQSFDILN; FQSFDILNT; QSFDILNTM; SFDILNTMK; |
| | FDILNTMKK; DILNTMKKS; ILNTMKKSI; LNTMKKSIP; NTMKKSIPN; |
| | TMKKSIPNF; MKKSIPNFE; KKSIPNFEI; KSIPNFEIQ; SIPNFEIQE; |
| | IPNFEIQEL; PNFEIQELR; NFEIQELRV; FEIQELRVD; EIQELRVDG; |

IQELRVDGG; QELRVDGGA; ELRVDGGAS; LRVDGGASQ; RVDGGASQN;
VDGGASQNN; DGGASQNNL; GGASQNNLL; GASQNNLLM; ASQNNLLMQ;
SQNNLLMQF; QNNLLMQFQ; NNLLMQFQA; NLLMQFQAD; LLMQFQADL;
LMQFQADLL; MQFQADLLE; QFQADLLEC; FQADLLECK; QADLLECKV;
ADLLECKVV; DLLECKVVR; LLECKVVRP; LECKVVRPK; ECKVVRPKI;
CKVVRPKIT; KVVRPKITE; VVRPKITET; VRPKITETT; RPKITETTA;
PKITETTAL; KITETTALG; ITETTALGA; TETTALGAA; ETTALGAAY;
TTALGAAYL; TALGAAYLA; ALGAAYLAG; LGAAYLAGL; GAAYLAGLA;
AAYLAGLAT; AYLAGLATG; YLAGLATGY; LAGLATGYW; AGLATGYWQ;
GLATGYWQS; LATGYWQSA; ATGYWQSAE; TGYWQSAEE; GYWQSAEEI;
YWQSAEEIV; WQSAEEIVS; QSAEEIVSL; SAEEIVSLW; AEEIVSLWQ;
EEIVSLWQV; EIVSLWQVD; IVSLWQVDK; VSLWQVDKI; SLWQVDKIF;
LWQVDKIFE; WQVDKIFEP; QVDKIFEPS; VDKIFEPSM; DKIFEPSMP;
KIFEPSMPK; IFEPSMPKN; FEPSMPKNQ; EPSMPKNQK; PSMPKNQKE;
SMPKNQKEK; MPKNQKEKL; PKNQKEKLL; KNQKEKLLE; NQKEKLLEN;
QKEKLLENW; KEKLLENWN; EKLLENWNK; KLLENWNKA; LLENWNKAV;
LENWNKAVG; ENWNKAVGK; NWNKAVGKA; WNKAVGKAK; NKAVGKAKS;
KAVGKAKSW; AVGKAKSWI; VGKAKSWIQ; GKAKSWIQN; KAKSWIQNS;
AKSWIQNSH; KSWIQNSHS; SWIQNSHSS;

10 mers:
MKYILSIDQG; KYILSIDQGT; YILSIDQGTT; ILSIDQGTTS;
LSIDQGTTSS; SIDQGTTSSR; IDQGTTSSRA; DQGTTSSRAM;
QGTTSSRAMV; GTTSSRAMVF; TTSSRAMVFD; TSSRAMVFDK;
SSRAMVFDKN; SRAMVFDKNA; RAMVFDKNAN; AMVFDKNANI;
MVFDKNANIK; VFDKNANIKG; FDKNANIKGF; DKNANIKGFA;
KNANIKGFAQ; NANIKGFAQK; ANIKGFAQKE; NIKGFAQKEF;
IKGFAQKEFT; KGFAQKEFTQ; GFAQKEFTQI; FAQKEFTQIY;
AQKEFTQIYP; QKEFTQIYPQ; KEFTQIYPQP; EFTQIYPQPS;
FTQIYPQPSW; TQIYPQPSWV; QIYPQPSWVE; IYPQPSWVEH;
YPQPSWVEHD; PQPSWVEHDP; QPSWVEHDPT; PSWVEHDPTE;
SWVEHDPTEI; WVEHDPTEIW; VEHDPTEIWG; EHDPTEIWGS;
HDPTEIWGSQ; DPTEIWGSQL; PTEIWGSQLG; TEIWGSQLGV;
EIWGSQLGVI; IWGSQLGVIT; WGSQLGVITE; GSQLGVITEA;
SQLGVITEAM; QLGVITEAMA; LGVITEAMAN; GVITEAMANA;
VITEAMANAR; ITEAMANARI; TEAMANARIL; EAMANARILP;
AMANARILPN; MANARILPNE; ANARILPNEI; NARILPNEID;
ARILPNEIDA; RILPNEIDAI; ILPNEIDAIG; LPNEIDAIGI;
PNEIDAIGIT; NEIDAIGITN; EIDAIGITNQ; IDAIGITNQR;
DAIGITNQRE; AIGITNQRET; IGITNQRETT; GITNQRETTV;
ITNQRETTVI; TNQRETTVIW; NQRETTVIWE; QRETTVIWEK;
RETTVIWEKN; ETTVIWEKNT; TTVIWEKNTG; TVIWEKNTGK;
VIWEKNTGKP; IWEKNTGKPI; WEKNTGKPIY; EKNTGKPIYN;
KNTGKPIYNA; NTGKPIYNAI; TGKPIYNAIV; GKPIYNAIVW;
KPIYNAIVWQ; PIYNAIVWQD; IYNAIVWQDR; YNAIVWQDRR;
NAIVWQDRRT; AIVWQDRRTA; IVWQDRRTAK; VWQDRRTAKI;
WQDRRTAKIC; QDRRTAKICD; DRRTAKICDQ; RRTAKICDQL;
RTAKICDQLK; TAKICDQLKK; AKICDQLKKE; KICDQLKKEG;
ICDQLKKEGK; CDQLKKEGKD; DQLKKEGKDK; QLKKEGKDKI;
LKKEGKDKII; KKEGKDKIIL; KEGKDKIILE; EGKDKIILEK;
GKDKIILEKT; KDKIILEKTG; DKIILEKTGL; KIILEKTGLV;

IILEKTGLVL; ILEKTGLVLD; LEKTGLVLDS; EKTGLVLDSY;
KTGLVLDSYF; TGLVLDSYFS; GLVLDSYFSG; LVLDSYFSGT;
VLDSYFSGTK; LDSYFSGTKI; DSYFSGTKIM; SYFSGTKIMW;
YFSGTKIMWI; FSGTKIMWIL; SGTKIMWILD; GTKIMWILDN;
TKIMWILDNV; KIMWILDNVE; IMWILDNVEG; MWILDNVEGA;
WILDNVEGAR; ILDNVEGARQ; LDNVEGARQR; DNVEGARQRA;
NVEGARQRAE; VEGARQRAEN; EGARQRAENG; GARQRAENGE;
ARQRAENGEL; RQRAENGELC; QRAENGELCF; RAENGELCFG;
AENGELCFGT; ENGELCFGTI; NGELCFGTID; GELCFGTIDT;
ELCFGTIDTW; LCFGTIDTWI; CFGTIDTWIL; FGTIDTWILW;
GTIDTWILWN; TIDTWILWNL; IDTWILWNLT; DTWILWNLTQ;
TWILWNLTQK; WILWNLTQKK; ILWNLTQKKE; LWNLTQKKEH;
WNLTQKKEHA; NLTQKKEHAT; LTQKKEHATD; TQKKEHATDY;
QKKEHATDYS; KKEHATDYSN; KEHATDYSNA; EHATDYSNAS;
HATDYSNASR; ATDYSNASRT; TDYSNASRTL; DYSNASRTLL;
YSNASRTLLL; SNASRTLLLN; NASRTLLLNI; ASRTLLLNIK;
SRTLLLNIKT; RTLLLNIKTL; TLLLNIKTLE; LLLNIKTLEW;
LLNIKTLEWD; LNIKTLEWDD; NIKTLEWDDE; IKTLEWDDEL;
KTLEWDDELL; TLEWDDELLS; LEWDDELLSI; EWDDELLSIL;
WDDELLSILN; DDELLSILNV; DELLSILNVP; ELLSILNVPR;
LLSILNVPRA; LSILNVPRAI; SILNVPRAIL; ILNVPRAILP;
LNVPRAILPE; NVPRAILPEL; VPRAILPELK; PRAILPELKE;
RAILPELKES; AILPELKESS; ILPELKESST; LPELKESSTI;
PELKESSTIY; ELKESSTIYG; LKESSTIYGK; KESSTIYGKT;
ESSTIYGKTD; SSTIYGKTDK; STIYGKTDKA; TIYGKTDKAL;
IYGKTDKALF; YGKTDKALFG; GKTDKALFGA; KTDKALFGAE;
TDKALFGAEI; DKALFGAEIP; KALFGAEIPI; ALFGAEIPIA;
LFGAEIPIAG; FGAEIPIAGI; GAEIPIAGIA; AEIPIAGIAG;
EIPIAGIAGD; IPIAGIAGDQ; PIAGIAGDQF; IAGIAGDQFA;
AGIAGDQFAA; GIAGDQFAAT; IAGDQFAATF; AGDQFAATFG;
GDQFAATFGQ; DQFAATFGQA; QFAATFGQAC; FAATFGQACL;
AATFGQACLK; ATFGQACLKK; TFGQACLKKG; FGQACLKKGM;
GQACLKKGMA; QACLKKGMAK; ACLKKGMAKN; CLKKGMAKNT;
LKKGMAKNTY; KKGMAKNTYG; KGMAKNTYGT; GMAKNTYGTG;
MAKNTYGTGC; AKNTYGTGCF; KNTYGTGCFL; NTYGTGCFLT;
TYGTGCFLTV; YGTGCFLTVN; GTGCFLTVNI; TGCFLTVNIG;
GCFLTVNIGK; CFLTVNIGKE; FLTVNIGKEP; LTVNIGKEPI;
TVNIGKEPII; VNIGKEPIIS; NIGKEPIISH; IGKEPIISHD;
GKEPIISHDK; KEPIISHDKL; EPIISHDKLL; PIISHDKLLT;
IISHDKLLTS; ISHDKLLTSI; SHDKLLTSIA; HDKLLTSIAW;
DKLLTSIAWG; KLLTSIAWGR; LLTSIAWGRK; LTSIAWGRKK;
TSIAWGRKKS; SIAWGRKKSV; IAWGRKKSVT; AWGRKKSVTY;
WGRKKSVTYV; GRKKSVTYVL; RKKSVTYVLE; KKSVTYVLEG;
KSVTYVLEGS; SVTYVLEGSV; VTYVLEGSVF; TYVLEGSVFI;
YVLEGSVFIG; VLEGSVFIGG; LEGSVFIGGA; EGSVFIGGAV;
GSVFIGGAVI; SVFIGGAVIQ; VFIGGAVIQW; FIGGAVIQWL;
IGGAVIQWLR; GGAVIQWLRD; GAVIQWLRDG; AVIQWLRDGL;
VIQWLRDGLE; IQWLRDGLEF; QWLRDGLEFF; WLRDGLEFFR;
LRDGLEFFRK; RDGLEFFRKS; DGLEFFRKSS; GLEFFRKSSD;
LEFFRKSSDA; EFFRKSSDAE; FFRKSSDAEA; FRKSSDAEAL;
RKSSDAEALA; KSSDAEALAS; SSDAEALASS; SDAEALASSV;

DAEALASSVS; AEALASSVSD; EALASSVSDN; ALASSVSDNG;
LASSVSDNGG; ASSVSDNGGV; SSVSDNGGVY; SVSDNGGVYF;
VSDNGGVYFV; SDNGGVYFVP; DNGGVYFVPA; NGGVYFVPAF;
GGVYFVPAFV; GVYFVPAFVG; VYFVPAFVGL; YFVPAFVGLG;
FVPAFVGLGA; VPAFVGLGAP; PAFVGLGAPH; AFVGLGAPHW;
FVGLGAPHWD; VGLGAPHWDS; GLGAPHWDSY; LGAPHWDSYA;
GAPHWDSYAR; APHWDSYARG; PHWDSYARGT; HWDSYARGTI;
WDSYARGTII; DSYARGTIIG; SYARGTIIGI; YARGTIIGIT;
ARGTIIGITR; RGTIIGITRG; GTIIGITRGS; TIIGITRGST;
IIGITRGSTK; IGITRGSTKA; GITRGSTKAH; ITRGSTKAHI;
TRGSTKAHIT; RGSTKAHITR; GSTKAHITRA; STKAHITRAA;
TKAHITRAAL; KAHITRAALE; AHITRAALES; HITRAALESI;
ITRAALESIA; TRAALESIAF; RAALESIAFQ; AALESIAFQS;
ALESIAFQSF; LESIAFQSFD; ESIAFQSFDI; SIAFQSFDIL;
IAFQSFDILN; AFQSFDILNT; FQSFDILNTM; QSFDILNTMK;
SFDILNTMKK; FDILNTMKKS; DILNTMKKSI; ILNTMKKSIP;
LNTMKKSIPN; NTMKKSIPNF; TMKKSIPNFE; MKKSIPNFEI;
KKSIPNFEIQ; KSIPNFEIQE; SIPNFEIQEL; IPNFEIQELR;
PNFEIQELRV; NFEIQELRVD; FEIQELRVDG; EIQELRVDGG;
IQELRVDGGA; QELRVDGGAS; ELRVDGGASQ; LRVDGGASQN;
RVDGGASQNN; VDGGASQNNL; DGGASQNNLL; GGASQNNLLM;
GASQNNLLMQ; ASQNNLLMQF; SQNNLLMQFQ; QNNLLMQFQA;
NNLLMQFQAD; NLLMQFQADL; LLMQFQADLL; LMQFQADLLE;
MQFQADLLEC; QFQADLLECK; FQADLLECKV; QADLLECKVV;
ADLLECKVVR; DLLECKVVRP; LLECKVVRPK; LECKVVRPKI;
ECKVVRPKIT; CKVVRPKITE; KVVRPKITET; VVRPKITETT;
VRPKITETTA; RPKITETTAL; PKITETTALG; KITETTALGA;
ITETTALGAA; TETTALGAAY; ETTALGAAYL; TTALGAAYLA;
TALGAAYLAG; ALGAAYLAGL; LGAAYLAGLA; GAAYLAGLAT;
AAYLAGLATG; AYLAGLATGY; YLAGLATGYW; LAGLATGYWQ;
AGLATGYWQS; GLATGYWQSA; LATGYWQSAE; ATGYWQSAEE;
TGYWQSAEEI; GYWQSAEEIV; YWQSAEEIVS; WQSAEEIVSL;
QSAEEIVSLW; SAEEIVSLWQ; AEEIVSLWQV; EEIVSLWQVD;
EIVSLWQVDK; IVSLWQVDKI; VSLWQVDKIF; SLWQVDKIFE;
LWQVDKIFEP; WQVDKIFEPS; QVDKIFEPSM; VDKIFEPSMP;
DKIFEPSMPK; KIFEPSMPKN; IFEPSMPKNQ; FEPSMPKNQK;
EPSMPKNQKE; PSMPKNQKEK; SMPKNQKEKL; MPKNQKEKLL;
PKNQKEKLLE; KNQKEKLLEN; NQKEKLLENW; QKEKLLENWN;
KEKLLENWNK; EKLLENWNKA; KLLENWNKAV; LLENWNKAVG;
LENWNKAVGK; ENWNKAVGKA; NWNKAVGKAK; WNKAVGKAKS;
NKAVGKAKSW; KAVGKAKSWI; AVGKAKSWIQ; VGKAKSWIQN;
GKAKSWIQNS; KAKSWIQNSH; AKSWIQNSHS; KSWIQNSHSS;

11 mers:
MKYILSIDQGT; KYILSIDQGTT; YILSIDQGTTS; ILSIDQGTTSS;
LSIDQGTTSSR; SIDQGTTSSRA; IDQGTTSSRAM; DQGTTSSRAMV;
QGTTSSRAMVF; GTTSSRAMVFD; TTSSRAMVFDK; TSSRAMVFDKN;
SSRAMVFDKNA; SRAMVFDKNAN; RAMVFDKNANI; AMVFDKNANIK;
MVFDKNANIKG; VFDKNANIKGF; FDKNANIKGFA; DKNANIKGFAQ;
KNANIKGFAQK; NANIKGFAQKE; ANIKGFAQKEF; NIKGFAQKEFT;
IKGFAQKEFTQ; KGFAQKEFTQI; GFAQKEFTQIY; FAQKEFTQIYP;

AQKEFTQIYPQ; QKEFTQIYPQP; KEFTQIYPQPS; EFTQIYPQPSW;
FTQIYPQPSWV; TQIYPQPSWVE; QIYPQPSWVEH; IYPQPSWVEHD;
YPQPSWVEHDP; PQPSWVEHDPT; QPSWVEHDPTE; PSWVEHDPTEI;
SWVEHDPTEIW; WVEHDPTEIWG; VEHDPTEIWGS; EHDPTEIWGSQ;
HDPTEIWGSQL; DPTEIWGSQLG; PTEIWGSQLGV; TEIWGSQLGVI;
EIWGSQLGVIT; IWGSQLGVITE; WGSQLGVITEA; GSQLGVITEAM;
SQLGVITEAMA; QLGVITEAMAN; LGVITEAMANA; GVITEAMANAR;
VITEAMANARI; ITEAMANARIL; TEAMANARILP; EAMANARILPN;
AMANARILPNE; MANARILPNEI; ANARILPNEID; NARILPNEIDA;
ARILPNEIDAI; RILPNEIDAIG; ILPNEIDAIGI; LPNEIDAIGIT;
PNEIDAIGITN; NEIDAIGITNQ; EIDAIGITNQR; IDAIGITNQRE;
DAIGITNQRET; AIGITNQRETT; IGITNQRETTV; GITNQRETTVI;
ITNQRETTVIW; TNQRETTVIWE; NQRETTVIWEK; QRETTVIWEKN;
RETTVIWEKNT; ETTVIWEKNTG; TTVIWEKNTGK; TVIWEKNTGKP;
VIWEKNTGKPI; IWEKNTGKPIY; WEKNTGKPIYN; EKNTGKPIYNA;
KNTGKPIYNAI; NTGKPIYNAIV; TGKPIYNAIVW; GKPIYNAIVWQ;
KPIYNAIVWQD; PIYNAIVWQDR; IYNAIVWQDRR; YNAIVWQDRRT;
NAIVWQDRRTA; AIVWQDRRTAK; IVWQDRRTAKI; VWQDRRTAKIC;
WQDRRTAKICD; QDRRTAKICDQ; DRRTAKICDQL; RRTAKICDQLK;
RTAKICDQLKK; TAKICDQLKKE; AKICDQLKKEG; KICDQLKKEGK;
ICDQLKKEGKD; CDQLKKEGKDK; DQLKKEGKDKI; QLKKEGKDKII;
LKKEGKDKIIL; KKEGKDKIILE; KEGKDKIILEK; EGKDKIILEKT;
GKDKIILEKTG; KDKIILEKTGL; DKIILEKTGLV; KIILEKTGLVL;
IILEKTGLVLD; ILEKTGLVLDS; LEKTGLVLDSY; EKTGLVLDSYF;
KTGLVLDSYFS; TGLVLDSYFSG; GLVLDSYFSGT; LVLDSYFSGTK;
VLDSYFSGTKI; LDSYFSGTKIM; DSYFSGTKIMW; SYFSGTKIMWI;
YFSGTKIMWIL; FSGTKIMWILD; SGTKIMWILDN; GTKIMWILDNV;
TKIMWILDNVE; KIMWILDNVEG; IMWILDNVEGA; MWILDNVEGAR;
WILDNVEGARQ; ILDNVEGARQR; LDNVEGARQRA; DNVEGARQRAE;
NVEGARQRAEN; VEGARQRAENG; EGARQRAENGE; GARQRAENGEL;
ARQRAENGELC; RQRAENGELCF; QRAENGELCFG; RAENGELCFGT;
AENGELCFGTI; ENGELCFGTID; NGELCFGTIDT; GELCFGTIDTW;
ELCFGTIDTWI; LCFGTIDTWIL; CFGTIDTWILW; FGTIDTWILWN;
GTIDTWILWNL; TIDTWILWNLT; IDTWILWNLTQ; DTWILWNLTQK;
TWILWNLTQKK; WILWNLTQKKE; ILWNLTQKKEH; LWNLTQKKEHA;
WNLTQKKEHAT; NLTQKKEHATD; LTQKKEHATDY; TQKKEHATDYS;
QKKEHATDYSN; KKEHATDYSNA; KEHATDYSNAS; EHATDYSNASR;
HATDYSNASRT; ATDYSNASRTL; TDYSNASRTLL; DYSNASRTLLL;
YSNASRTLLLN; SNASRTLLLNI; NASRTLLLNIK; ASRTLLLNIKT;
SRTLLLNIKTL; RTLLLNIKTLE; TLLLNIKTLEW; LLLNIKTLEWD;
LLNIKTLEWDD; LNIKTLEWDDE; NIKTLEWDDEL; IKTLEWDDELL;
KTLEWDDELLS; TLEWDDELLSI; LEWDDELLSIL; EWDDELLSILN;
WDDELLSILNV; DDELLSILNVP; DELLSILNVPR; ELLSILNVPRA;
LLSILNVPRAI; LSILNVPRAIL; SILNVPRAILP; ILNVPRAILPE;
LNVPRAILPEL; NVPRAILPELK; VPRAILPELKE; PRAILPELKES;
RAILPELKESS; AILPELKESST; ILPELKESSTI; LPELKESSTIY;
PELKESSTIYG; ELKESSTIYGK; LKESSTIYGKT; KESSTIYGKTD;
ESSTIYGKTDK; SSTIYGKTDKA; STIYGKTDKAL; TIYGKTDKALF;
IYGKTDKALFG; YGKTDKALFGA; GKTDKALFGAE; KTDKALFGAEI;
TDKALFGAEIP; DKALFGAEIPI; KALFGAEIPIA; ALFGAEIPIAG;
LFGAEIPIAGI; FGAEIPIAGIA; GAEIPIAGIAG; AEIPIAGIAGD;

| | | | |
|---|---|---|---|
| EIPIAGIAGDQ; | IPIAGIAGDQF; | PIAGIAGDQFA; | IAGIAGDQFAA; |
| AGIAGDQFAAT; | GIAGDQFAATF; | IAGDQFAATFG; | AGDQFAATFGQ; |
| GDQFAATFGQA; | DQFAATFGQAC; | QFAATFGQACL; | FAATFGQACLK; |
| AATFGQACLKK; | ATFGQACLKKG; | TFGQACLKKGM; | FGQACLKKGMA; |
| GQACLKKGMAK; | QACLKKGMAKN; | ACLKKGMAKNT; | CLKKGMAKNTY; |
| LKKGMAKNTYG; | KKGMAKNTYGT; | KGMAKNTYGTG; | GMAKNTYGTGC; |
| MAKNTYGTGCF; | AKNTYGTGCFL; | KNTYGTGCFLT; | NTYGTGCFLTV; |
| TYGTGCFLTVN; | YGTGCFLTVNI; | GTGCFLTVNIG; | TGCFLTVNIGK; |
| GCFLTVNIGKE; | CFLTVNIGKEP; | FLTVNIGKEPI; | LTVNIGKEPII; |
| TVNIGKEPIIS; | VNIGKEPIISH; | NIGKEPIISHD; | IGKEPIISHDK; |
| GKEPIISHDKL; | KEPIISHDKLL; | EPIISHDKLLT; | PIISHDKLLTS; |
| IISHDKLLTSI; | ISHDKLLTSIA; | SHDKLLTSIAW; | HDKLLTSIAWG; |
| DKLLTSIAWGR; | KLLTSIAWGRK; | LLTSIAWGRKK; | LTSIAWGRKKS; |
| TSIAWGRKKSV; | SIAWGRKKSVT; | IAWGRKKSVTY; | AWGRKKSVTYV; |
| WGRKKSVTYVL; | GRKKSVTYVLE; | RKKSVTYVLEG; | KKSVTYVLEGS; |
| KSVTYVLEGSV; | SVTYVLEGSVF; | VTYVLEGSVFI; | TYVLEGSVFIG; |
| YVLEGSVFIGG; | VLEGSVFIGGA; | LEGSVFIGGAV; | EGSVFIGGAVI; |
| GSVFIGGAVIQ; | SVFIGGAVIQW; | VFIGGAVIQWL; | FIGGAVIQWLR; |
| IGGAVIQWLRD; | GGAVIQWLRDG; | GAVIQWLRDGL; | AVIQWLRDGLE; |
| VIQWLRDGLEF; | IQWLRDGLEFF; | QWLRDGLEFFR; | WLRDGLEFFRK; |
| LRDGLEFFRKS; | RDGLEFFRKSS; | DGLEFFRKSSD; | GLEFFRKSSDA; |
| LEFFRKSSDAE; | EFFRKSSDAEA; | FFRKSSDAEAL; | FRKSSDAEALA; |
| RKSSDAEALAS; | KSSDAEALASS; | SSDAEALASSV; | SDAEALASSVS; |
| DAEALASSVSD; | AEALASSVSDN; | EALASSVSDNG; | ALASSVSDNGG; |
| LASSVSDNGGV; | ASSVSDNGGVY; | SSVSDNGGVYF; | SVSDNGGVYFV; |
| VSDNGGVYFVP; | SDNGGVYFVPA; | DNGGVYFVPAF; | NGGVYFVPAFV; |
| GGVYFVPAFVG; | GVYFVPAFVGL; | VYFVPAFVGLG; | YFVPAFVGLGA; |
| FVPAFVGLGAP; | VPAFVGLGAPH; | PAFVGLGAPHW; | AFVGLGAPHWD; |
| FVGLGAPHWDS; | VGLGAPHWDSY; | GLGAPHWDSYA; | LGAPHWDSYAR; |
| GAPHWDSYARG; | APHWDSYARGT; | PHWDSYARGTI; | HWDSYARGTII; |
| WDSYARGTIIG; | DSYARGTIIGI; | SYARGTIIGIT; | YARGTIIGITR; |
| ARGTIIGITRG; | RGTIIGITRGS; | GTIIGITRGST; | TIIGITRGSTK; |
| IIGITRGSTKA; | IGITRGSTKAH; | GITRGSTKAHI; | ITRGSTKAHIT; |
| TRGSTKAHITR; | RGSTKAHITRA; | GSTKAHITRAA; | STKAHITRAAL; |
| TKAHITRAALE; | KAHITRAALES; | AHITRAALESI; | HITRAALESIA; |
| ITRAALESIAF; | TRAALESIAFQ; | RAALESIAFQS; | AALESIAFQSF; |
| ALESIAFQSFD; | LESIAFQSFDI; | ESIAFQSFDIL; | SIAFQSFDILN; |
| IAFQSFDILNT; | AFQSFDILNTM; | FQSFDILNTMK; | QSFDILNTMKK; |
| SFDILNTMKKS; | FDILNTMKKSI; | DILNTMKKSIP; | ILNTMKKSIPN; |
| LNTMKKSIPNF; | NTMKKSIPNFE; | TMKKSIPNFEI; | MKKSIPNFEIQ; |
| KKSIPNFEIQE; | KSIPNFEIQEL; | SIPNFEIQELR; | IPNFEIQELRV; |
| PNFEIQELRVD; | NFEIQELRVDG; | FEIQELRVDGG; | EIQELRVDGGA; |
| IQELRVDGGAS; | QELRVDGGASQ; | ELRVDGGASQN; | LRVDGGASQNN; |
| RVDGGASQNNL; | VDGGASQNNLL; | DGGASQNNLLM; | GGASQNNLLMQ; |
| GASQNNLLMQF; | ASQNNLLMQFQ; | SQNNLLMQFQA; | QNNLLMQFQAD; |
| NNLLMQFQADL; | NLLMQFQADLL; | LLMQFQADLLE; | LMQFQADLLEC; |
| MQFQADLLECK; | QFQADLLECKV; | FQADLLECKVV; | QADLLECKVVR; |
| ADLLECKVVRP; | DLLECKVVRPK; | LLECKVVRPKI; | LECKVVRPKIT; |
| ECKVVRPKITE; | CKVVRPKITET; | KVVRPKITETT; | VVRPKITETTA; |
| VRPKITETTAL; | RPKITETTALG; | PKITETTALGA; | KITETTALGAA; |
| ITETTALGAAY; | TETTALGAAYL; | ETTALGAAYLA; | TTALGAAYLAG; |

| | |
|---|---|
| | TALGAAYLAGL; ALGAAYLAGLA; LGAAYLAGLAT; GAAYLAGLATG; AAYLAGLATGY; AYLAGLATGYW; YLAGLATGYWQ; LAGLATGYWQS; AGLATGYWQSA; GLATGYWQSAE; LATGYWQSAEE; ATGYWQSAEEI; TGYWQSAEEIV; GYWQSAEEIVS; YWQSAEEIVSL; WQSAEEIVSLW; QSAEEIVSLWQ; SAEEIVSLWQV; AEEIVSLWQVD; EEIVSLWQVDK; EIVSLWQVDKI; IVSLWQVDKIF; VSLWQVDKIFE; SLWQVDKIFEP; LWQVDKIFEPS; WQVDKIFEPSM; QVDKIFEPSMP; VDKIFEPSMPK; DKIFEPSMPKN; KIFEPSMPKNQ; IFEPSMPKNQK; FEPSMPKNQKE; EPSMPKNQKEK; PSMPKNQKEKL; SMPKNQKEKLL; MPKNQKEKLLE; PKNQKEKLLEN; KNQKEKLLENW; NQKEKLLENWN; QKEKLLENWNK; KEKLLENWNKA; EKLLENWNKAV; KLLENWNKAVG; LLENWNKAVGK; LENWNKAVGKA; ENWNKAVGKAK; NWNKAVGKAKS; WNKAVGKAKSW; NKAVGKAKSWI; KAVGKAKSWIQ; AVGKAKSWIQN; VGKAKSWIQNS; GKAKSWIQNSH; KAKSWIQNSHS; AKSWIQNSHSS; |
| Seq 28 | SEQ ID NO 33624-35913/<br>8 mers:<br>MKTIKKDS; KTIKKDSE; TIKKDSEF; IKKDSEFY; KKDSEFYD; KDSEFYDS; DSEFYDSL; SEFYDSLA; EFYDSLAT; FYDSLATL; YDSLATLK; DSLATLKK; SLATLKKH; LATLKKHI; ATLKKHIN; TLKKHINK; LKKHINKY; KKHINKYI; KHINKYIE; HINKYIEE; INKYIEEK; NKYIEEKV; KYIEEKVL; YIEEKVLK; IEEKVLKY; EEKVLKYS; EKVLKYSI; KVLKYSIS; VLKYSISS; LKYSISSQ; KYSISSQL; YSISSQLY; SISSQLYK; ISSQLYKL; SSQLYKLE; SQLYKLEK; QLYKLEKP; LYKLEKPE; YKLEKPEI; KLEKPEII; LEKPEIIE; EKPEIIEL; KPEIIELI; PEIIELIK; EIIELIKI; IIELIKIS; IELIKISN; ELIKISND; LIKISNDY; IKISNDYE; KISNDYEK; ISNDYEKE; SNDYEKEK; NDYEKEKN; DYEKEKNA; YEKEKNAF; EKEKNAFN; KEKNAFNT; EKNAFNTS; KNAFNTSL; NAFNTSLE; AFNTSLEE; FNTSLEEC; NTSLEECY; TSLEECYY; SLEECYYK; LEECYYKN; EECYYKNT; ECYYKNTQ; CYYKNTQN; YYKNTQNE; YKNTQNEA; KNTQNEAI; NTQNEAIK; TQNEAIKK; QNEAIKKW; NEAIKKWI; EAIKKWIL; AIKKWILE; IKKWILEI; KKWILEIV; KWILEIVR; WILEIVRN; ILEIVRNK; LEIVRNKN; EIVRNKNF; IVRNKNFI; VRNKNFIQ; RNKNFIQI; NKNFIQIS; KNFIQISK; NFIQISKE; FIQISKEA; IQISKEAN; QISKEANL; ISKEANLN; SKEANLNT; KEANLNTK; EANLNTKK; ANLNTKKL; NLNTKKLG; LNTKKLGT; NTKKLGTT; TKKLGTTY; KKLGTTYK; KLGTTYKL; LGTTYKLK; GTTYKLKS; TTYKLKSS; TYKLKSSN; YKLKSSNF; KLKSSNFL; LKSSNFLK; KSSNFLKL; SSNFLKLI; SNFLKLIE; NFLKLIEI; FLKLIEIQ; LKLIEIQN; KLIEIQNS; LIEIQNSP; IEIQNSPY; EIQNSPYY; IQNSPYYS; QNSPYYSQ; NSPYYSQE; SPYYSQEK; PYYSQEKK; YYSQEKKD; YSQEKKDI; SQEKKDIY; QEKKDIYK; EKKDIYKQ; KKDIYKQI; KDIYKQII; DIYKQIIL; IYKQIILN; YKQIILNF; KQIILNFS; QIILNFSS; IILNFSSN; ILNFSSNI; LNFSSNIN; NFSSNINI; FSSNINID; SSNINIDS; SNINIDSL; NINIDSLE; INIDSLEQ; NIDSLEQT; IDSLEQTI; DSLEQTID; SLEQTIDI; LEQTIDIL; EQTIDILV; QTIDILVA; TIDILVAV; IDILVAVR; DILVAVRN; ILVAVRNR; LVAVRNRN; VAVRNRNK; AVRNRNKI; VRNRNKIK; RNRNKIKI; NRNKIKIL; RNKIKILN; NKIKILNI; KIKILNIL; IKILNILN; |

KILNILNK; ILNILNKN; LNILNKNL; NILNKNLK; ILNKNLKY;
LNKNLKYR; NKNLKYRP; KNLKYRPE; NLKYRPEN; LKYRPENQ;
KYRPENQK; YRPENQKV; RPENQKVF; PENQKVFK; ENQKVFKS;
NQKVFKSS; QKVFKSSL; KVFKSSLT; VFKSSLTN; FKSSLTNK;
KSSLTNKE; SSLTNKES; SLTNKESR; LTNKESRL; TNKESRLI;
NKESRLIK; KESRLIKL; ESRLIKLK; SRLIKLKR; RLIKLKRM;
LIKLKRML; IKLKRMLI; KLKRMLIL; LKRMLILT; KRMLILTY;
RMLILTYW; MLILTYWP; LILTYWPV; ILTYWPVG; LTYWPVGC;
TYWPVGCL; YWPVGCLS; WPVGCLSK; PVGCLSKN; VGCLSKNI;
GCLSKNIF; CLSKNIFI; LSKNIFIK; SKNIFIKI; KNIFIKIL;
NIFIKILI; IFIKILIK; FIKILIKX; IKILIKXY; KILIKXYK;
ILIKXYKY; LIKXYKYL; IKXYKYLE; KXYKYLEE; XYKYLEEE;
YKYLEEEI; KYLEEEIL; YLEEEILA; LEEEILAL; EEEILALK;
EEILALKY; EILALKYN; ILALKYNE; LALKYNEI; ALKYNEIL;
LKYNEILN; KYNEILNY; YNEILNYL; NEILNYLR; EILNYLRA;
ILNYLRAL; LNYLRALK; NYLRALKT; YLRALKTL; LRALKTLS;
RALKTLSL; ALKTLSLN; LKTLSLNE; KTLSLNEI; TLSLNEIF;
LSLNEIFY; SLNEIFYK; LNEIFYKG; NEIFYKGS; EIFYKGSS;
IFYKGSSK; FYKGSSKN; YKGSSKNI; KGSSKNIS; GSSKNISF;
SSKNISFN; SKNISFNY; KNISFNYF; NISFNYFF; ISFNYFFS;
SFNYFFSD; FNYFFSDF; NYFFSDFT; YFFSDFTQ; FFSDFTQY;
FSDFTQYA; SDFTQYAP; DFTQYAPK; FTQYAPKD; TQYAPKDF;
QYAPKDFQ; YAPKDFQD; APKDFQDT; PKDFQDTL; KDFQDTLK;
DFQDTLKC; FQDTLKCY; QDTLKCYL; DTLKCYLY; TLKCYLYV;
LKCYLYVI; KCYLYVIE; CYLYVIEK; YLYVIEKT; LYVIEKTI;
YVIEKTIT; VIEKTITK; IEKTITKK; EKTITKKY; KTITKKYL;
TITKKYLT; ITKKYLTW; TKKYLTWF; KKYLTWFF; KYLTWFFK;
YLTWFFKD; LTWFFKDK; TWFFKDKI; WFFKDKIS; FFKDKISN;
FKDKISNN; KDKISNNW; DKISNNWE; KISNNWES; ISNNWESF;
SNNWESFI; NNWESFID; NWESFIDA; WESFIDAL; ESFIDALE;
SFIDALEY; FIDALEYI; IDALEYIE; DALEYIEK; ALEYIEKH;
LEYIEKHK; EYIEKHKL; YIEKHKLI; IEKHKLIN; EKHKLINI;
KHKLINIK; HKLINIKE; KLINIKEK; LINIKEKI; INIKEKIK;
NIKEKIKE; IKEKIKEI; KEKIKEII; EKIKEIIT; KIKEIITA;
IKEIITAK; KEIITAKF; EIITAKFQ; IITAKFQT; ITAKFQTE;
TAKFQTED; AKFQTEDF; KFQTEDFF; FQTEDFFI; QTEDFFIS;
TEDFFISF; EDFFISFD; DFFISFDK; FFISFDKT; FISFDKTN;
ISFDKTNF; SFDKTNFN; FDKTNFNP; DKTNFNPY; KTNFNPYE;
TNFNPYES; NFNPYESS; FNPYESSI; NPYESSIF; PYESSIFK;
YESSIFKE; ESSIFKER; SSIFKERY; SIFKERYI; IFKERYIK;
FKERYIKS; KERYIKSA; ERYIKSAF; RYIKSAFL; YIKSAFLE;
IKSAFLEI; KSAFLEII; SAFLEIIM; AFLEIIMH; FLEIIMHY;
LEIIMHYV; EIIMHYVK; IIMHYVKK; IMHYVKKN; MHYVKKNS;
HYVKKNSQ; YVKKNSQN; VKKNSQNI; KKNSQNIE; KNSQNIET;
NSQNIETY; SQNIETYG; QNIETYGW; NIETYGWI; IETYGWIA;
ETYGWIAF; TYGWIAFY; YGWIAFYI; GWIAFYIY; WIAFYIYA;
IAFYIYAD; AFYIYADN; FYIYADNK; YIYADNKD; IYADNKDK;
YADNKDKK; ADNKDKKI; DNKDKKIF; NKDKKIFC; KDKKIFCQ;
DKKIFCQR; KKIFCQRM; KIFCQRMK; IFCQRMKE; FCQRMKEF;
CQRMKEFF; QRMKEFFK; RMKEFFKS; MKEFFKSK; KEFFKSKT;
EFFKSKTF; FFKSKTFE; FKSKTFEI; KSKTFEIQ; SKTFEIQN;

KTFEIQNQ; TFEIQNQI; FEIQNQIF; EIQNQIFV; IQNQIFVY;
QNQIFVYF; NQIFVYFL; QIFVYFLS; IFVYFLSF; FVYFLSFY;
VYFLSFYP; YFLSFYPN; FLSFYPNI; LSFYPNIE; SFYPNIEY;
FYPNIEYK; YPNIEYKH; PNIEYKHF; NIEYKHFK; IEYKHFKF;
EYKHFKFI; YKHFKFIS; KHFKFISD; HFKFISDI; FKFISDIL;
KFISDILL; FISDILLY; ISDILLYL; SDILLYLD; DILLYLDE;
ILLYLDEN; LLYLDENK; LYLDENKI; YLDENKIT; LDENKITI;
DENKITIP; ENKITIPK; NKITIPKK; KITIPKKI; ITIPKKII;
TIPKKIIK; IPKKIIKI; PKKIIKIQ; KKIIKIQK; KIIKIQKI;
IIKIQKIN; IKIQKINP; KIQKINPN; IQKINPNQ; QKINPNQL;
KINPNQLD; INPNQLDY; NPNQLDYQ; PNQLDYQK; NQLDYQKS;
QLDYQKSY; LDYQKSYL; DYQKSYLL; YQKSYLLI; QKSYLLIK;
KSYLLIKS; SYLLIKSL; YLLIKSLK; LLIKSLKT; LIKSLKTR;
IKSLKTRS; KSLKTRSR; SLKTRSRI; LKTRSRIL; KTRSRILK;
TRSRILKI; RSRILKII; SRILKIII; RILKIIIK; ILKIIIKN;
LKIIIKNI; KIIIKNIR; IIIKNIRF; IIKNIRFN; IKNIRFNL;
KNIRFNLI; NIRFNLIE; IRFNLIEK; RFNLIEKL; FNLIEKLE;
NLIEKLED; LIEKLEDE; IEKLEDEN; EKLEDENE; KLEDENEK;
LEDENEKK; EDENEKKL; DENEKKLL; ENEKKLLP; NEKKLLPA;
EKKLLPAM; KKLLPAMC; KLLPAMCY; LLPAMCYL; LPAMCYLI;
PAMCYLIY; AMCYLIYC; MCYLIYCE; CYLIYCEN; YLIYCENL;
LIYCENLV; IYCENLVE; YCENLVEL; CENLVELK; ENLVELKN;
NLVELKNN; LVELKNNR; VELKNNRK; ELKNNRKI; LKNNRKIK;
KNNRKIKS; NNRKIKSN; NRKIKSNE; RKIKSNEK; KIKSNEKN;
IKSNEKNS; KSNEKNSL; SNEKNSLL; NEKNSLLE; EKNSLLEF;
KNSLLEFI; NSLLEFIS; SLLEFISF; LLEFISFF; LEFISFFK;
EFISFFKT; FISFFKTK; ISFFKTKL; SFFKTKLK; FFKTKLKQ;
FKTKLKQK; KTKLKQKI; TKLKQKIN; KLKQKINF; LKQKINFK;
KQKINFKK; QKINFKKE; KINFKKEL; INFKKELM; NFKKELMK;
FKKELMKS; KKELMKSK; KELMKSKG; ELMKSKGI;

9 mers:
MKTIKKDSE; KTIKKDSEF; TIKKDSEFY; IKKDSEFYD; KKDSEFYDS;
KDSEFYDSL; DSEFYDSLA; SEFYDSLAT; EFYDSLATL; FYDSLATLK;
YDSLATLKK; DSLATLKKH; SLATLKKHI; LATLKKHIN; ATLKKHINK;
TLKKHINKY; LKKHINKYI; KKHINKYIE; KHINKYIEE; HINKYIEEK;
INKYIEEKV; NKYIEEKVL; KYIEEKVLK; YIEEKVLKY; IEEKVLKYS;
EEKVLKYSI; EKVLKYSIS; KVLKYSISS; VLKYSISSQ; LKYSISSQL;
KYSISSQLY; YSISSQLYK; SISSQLYKL; ISSQLYKLE; SSQLYKLEK;
SQLYKLEKP; QLYKLEKPE; LYKLEKPEI; YKLEKPEII; KLEKPEIIE;
LEKPEIIEL; EKPEIIELI; KPEIIELIK; PEIIELIKI; EIIELIKIS;
IIELIKISN; IELIKISND; ELIKISNDY; LIKISNDYE; IKISNDYEK;
KISNDYEKE; ISNDYEKEK; SNDYEKEKN; NDYEKEKNA; DYEKEKNAF;
YEKEKNAFN; EKEKNAFNT; KEKNAFNTS; EKNAFNTSL; KNAFNTSLE;
NAFNTSLEE; AFNTSLEEC; FNTSLEECY; NTSLEECYY; TSLEECYYK;
SLEECYYKN; LEECYYKNT; EECYYKNTQ; ECYYKNTQN; CYYKNTQNE;
YYKNTQNEA; YKNTQNEAI; KNTQNEAIK; NTQNEAIKK; TQNEAIKKW;
QNEAIKKWI; NEAIKKWIL; EAIKKWILE; AIKKWILEI; IKKWILEIV;
KKWILEIVR; KWILEIVRN; WILEIVRNK; ILEIVRNKN; LEIVRNKNF;
EIVRNKNFI; IVRNKNFIQ; VRNKNFIQI; RNKNFIQIS; NKNFIQISK;
KNFIQISKE; NFIQISKEA; FIQISKEAN; IQISKEANL; QISKEANLN;

ISKEANLNT; SKEANLNTK; KEANLNTKK; EANLNTKKL; ANLNTKKLG;
NLNTKKLGT; LNTKKLGTT; NTKKLGTTY; TKKLGTTYK; KKLGTTYKL;
KLGTTYKLK; LGTTYKLKS; GTTYKLKSS; TTYKLKSSN; TYKLKSSNF;
YKLKSSNFL; KLKSSNFLK; LKSSNFLKL; KSSNFLKLI; SSNFLKLIE;
SNFLKLIEI; NFLKLIEIQ; FLKLIEIQN; LKLIEIQNS; KLIEIQNSP;
LIEIQNSPY; IEIQNSPYY; EIQNSPYYS; IQNSPYYSQ; QNSPYYSQE;
NSPYYSQEK; SPYYSQEKK; PYYSQEKKD; YYSQEKKDI; YSQEKKDIY;
SQEKKDIYK; QEKKDIYKQ; EKKDIYKQI; KKDIYKQII; KDIYKQIIL;
DIYKQIILN; IYKQIILNF; YKQIILNFS; KQIILNFSS; QIILNFSSN;
IILNFSSNI; ILNFSSNIN; LNFSSNINI; NFSSNINID; FSSNINIDS;
SSNINIDSL; SNINIDSLE; NINIDSLEQ; INIDSLEQT; NIDSLEQTI;
IDSLEQTID; DSLEQTIDI; SLEQTIDIL; LEQTIDILV; EQTIDILVA;
QTIDILVAV; TIDILVAVR; IDILVAVRN; DILVAVRNR; ILVAVRNRN;
LVAVRNRNK; VAVRNRNKI; AVRNRNKIK; VRNRNKIKI; RNRNKIKIL;
NRNKIKILN; RNKIKILNI; NKIKILNIL; KIKILNILN; IKILNILNK;
KILNILNKN; ILNILNKNL; LNILNKNLK; NILNKNLKY; ILNKNLKYR;
LNKNLKYRP; NKNLKYRPE; KNLKYRPEN; NLKYRPENQ; LKYRPENQK;
KYRPENQKV; YRPENQKVF; RPENQKVFK; PENQKVFKS; ENQKVFKSS;
NQKVFKSSL; QKVFKSSLT; KVFKSSLTN; VFKSSLTNK; FKSSLTNKE;
KSSLTNKES; SSLTNKESR; SLTNKESRL; LTNKESRLI; TNKESRLIK;
NKESRLIKL; KESRLIKLK; ESRLIKLKR; SRLIKLKRM; RLIKLKRML;
LIKLKRMLI; IKLKRMLIL; KLKRMLILT; LKRMLILTY; KRMLILTYW;
RMLILTYWP; MLILTYWPV; LILTYWPVG; ILTYWPVGC; LTYWPVGCL;
TYWPVGCLS; YWPVGCLSK; WPVGCLSKN; PVGCLSKNI; VGCLSKNIF;
GCLSKNIFI; CLSKNIFIK; LSKNIFIKI; SKNIFIKIL; KNIFIKILI;
NIFIKILIK; IFIKILIKX; FIKILIKXY; IKILIKXYK; KILIKXYKY;
ILIKXYKYL; LIKXYKYLE; IKXYKYLEE; KXYKYLEEE; XYKYLEEEI;
YKYLEEEIL; KYLEEEILA; YLEEEILAL; LEEEILALK; EEEILALKY;
EEILALKYN; EILALKYNE; ILALKYNEI; LALKYNEIL; ALKYNEILN;
LKYNEILNY; KYNEILNYL; YNEILNYLR; NEILNYLRA; EILNYLRAL;
ILNYLRALK; LNYLRALKT; NYLRALKTL; YLRALKTLS; LRALKTLSL;
RALKTLSLN; ALKTLSLNE; LKTLSLNEI; KTLSLNEIF; TLSLNEIFY;
LSLNEIFYK; SLNEIFYKG; LNEIFYKGS; NEIFYKGSS; EIFYKGSSK;
IFYKGSSKN; FYKGSSKNI; YKGSSKNIS; KGSSKNISF; GSSKNISFN;
SSKNISFNY; SKNISFNYF; KNISFNYFF; NISFNYFFS; ISFNYFFSD;
SFNYFFSDF; FNYFFSDFT; NYFFSDFTQ; YFFSDFTQY; FFSDFTQYA;
FSDFTQYAP; SDFTQYAPK; DFTQYAPKD; FTQYAPKDF; TQYAPKDFQ;
QYAPKDFQD; YAPKDFQDT; APKDFQDTL; PKDFQDTLK; KDFQDTLKC;
DFQDTLKCY; FQDTLKCYL; QDTLKCYLY; DTLKCYLYV; TLKCYLYVI;
LKCYLYVIE; KCYLYVIEK; CYLYVIEKT; YLYVIEKTI; LYVIEKTIT;
YVIEKTITK; VIEKTITKK; IEKTITKKY; EKTITKKYL; KTITKKYLT;
TITKKYLTW; ITKKYLTWF; TKKYLTWFF; KKYLTWFFK; KYLTWFFKD;
YLTWFFKDK; LTWFFKDKI; TWFFKDKIS; WFFKDKISN; FFKDKISNN;
FKDKISNNW; KDKISNNWE; DKISNNWES; KISNNWESF; ISNNWESFI;
SNNWESFID; NNWESFIDA; NWESFIDAL; WESFIDALE; ESFIDALEY;
SFIDALEYI; FIDALEYIE; IDALEYIEK; DALEYIEKH; ALEYIEKHK;
LEYIEKHKL; EYIEKHKLI; YIEKHKLIN; IEKHKLINI; EKHKLINIK;
KHKLINIKE; HKLINIKEK; KLINIKEKI; LINIKEKIK; INIKEKIKE;
NIKEKIKEI; IKEKIKEII; KEKIKEIIT; EKIKEIITA; KIKEIITAK;
IKEIITAKF; KEIITAKFQ; EIITAKFQT; IITAKFQTE; ITAKFQTED;
TAKFQTEDF; AKFQTEDFF; KFQTEDFFI; FQTEDFFIS; QTEDFFISF;

EP 2 254 592 B1

| | |
|---|---|
| | TEDFFISFD; EDFFISFDK; DFFISFDKT; FFISFDKTN; FISFDKTNF; |
| | ISFDKTNFN; SFDKTNFNP; FDKTNFNPY; DKTNFNPYE; KTNFNPYES; |
| | TNFNPYESS; NFNPYESSI; FNPYESSIF; NPYESSIFK; PYESSIFKE; |
| | YESSIFKER; ESSIFKERY; SSIFKERYI; SIFKERYIK; IFKERYIKS; |
| | FKERYIKSA; KERYIKSAF; ERYIKSAFL; RYIKSAFLE; YIKSAFLEI; |
| | IKSAFLEII; KSAFLEIIM; SAFLEIIMH; AFLEIIMHY; FLEIIMHYV; |
| | LEIIMHYVK; EIIMHYVKK; IIMHYVKKN; IMHYVKKNS; MHYVKKNSQ; |
| | HYVKKNSQN; YVKKNSQNI; VKKNSQNIE; KKNSQNIET; KNSQNIETY; |
| | NSQNIETYG; SQNIETYGW; QNIETYGWI; NIETYGWIA; IETYGWIAF; |
| | ETYGWIAFY; TYGWIAFYI; YGWIAFYIY; GWIAFYIYA; WIAFYIYAD; |
| | IAFYIYADN; AFYIYADNK; FYIYADNKD; YIYADNKDK; IYADNKDKK; |
| | YADNKDKKI; ADNKDKKIF; DNKDKKIFC; NKDKKIFCQ; KDKKIFCQR; |
| | DKKIFCQRM; KKIFCQRMK; KIFCQRMKE; IFCQRMKEF; FCQRMKEFF; |
| | CQRMKEFFK; QRMKEFFKS; RMKEFFKSK; MKEFFKSKT; KEFFKSKTF; |
| | EFFKSKTFE; FFKSKTFEI; FKSKTFEIQ; KSKTFEIQN; SKTFEIQNQ; |
| | KTFEIQNQI; TFEIQNQIF; FEIQNQIFV; EIQNQIFVY; IQNQIFVYF; |
| | QNQIFVYFL; NQIFVYFLS; QIFVYFLSF; IFVYFLSFY; FVYFLSFYP; |
| | VYFLSFYPN; YFLSFYPNI; FLSFYPNIE; LSFYPNIEY; SFYPNIEYK; |
| | FYPNIEYKH; YPNIEYKHF; PNIEYKHFK; NIEYKHFKF; IEYKHFKFI; |
| | EYKHFKFIS; YKHFKFISD; KHFKFISDI; HFKFISDIL; FKFISDILL; |
| | KFISDILLY; FISDILLYL; ISDILLYLD; SDILLYLDE; DILLYLDEN; |
| | ILLYLDENK; LLYLDENKI; LYLDENKIT; YLDENKITI; LDENKITIP; |
| | DENKITIPK; ENKITIPKK; NKITIPKKI; KITIPKKII; ITIPKKIIK; |
| | TIPKKIIKI; IPKKIIKIQ; PKKIIKIQK; KKIIKIQKI; KIIKIQKIN; |
| | IIKIQKINP; IKIQKINPN; KIQKINPNQ; IQKINPNQL; QKINPNQLD; |
| | KINPNQLDY; INPNQLDYQ; NPNQLDYQK; PNQLDYQKS; NQLDYQKSY; |
| | QLDYQKSYL; LDYQKSYLL; DYQKSYLLI; YQKSYLLIK; QKSYLLIKS; |
| | KSYLLIKSL; SYLLIKSLK; YLLIKSLKT; LLIKSLKTR; LIKSLKTRS; |
| | IKSLKTRSR; KSLKTRSRI; SLKTRSRIL; LKTRSRILK; KTRSRILKI; |
| | TRSRILKII; RSRILKIII; SRILKIIIK; RILKIIIKN; ILKIIIKNI; |
| | LKIIIKNIR; KIIIKNIRF; IIIKNIRFN; IIKNIRFNL; IKNIRFNLI; |
| | KNIRFNLIE; NIRFNLIEK; IRFNLIEKL; RFNLIEKLE; FNLIEKLED; |
| | NLIEKLEDE; LIEKLEDEN; IEKLEDENE; EKLEDENEK; KLEDENEKK; |
| | LEDENEKKL; EDENEKKLL; DENEKKLLP; ENEKKLLPA; NEKKLLPAM; |
| | EKKLLPAMC; KKLLPAMCY; KLLPAMCYL; LLPAMCYLI; LPAMCYLIY; |
| | PAMCYLIYC; AMCYLIYCE; MCYLIYCEN; CYLIYCENL; YLIYCENLV; |
| | LIYCENLVE; IYCENLVEL; YCENLVELK; CENLVELKN; ENLVELKNN; |
| | NLVELKNNR; LVELKNNRK; VELKNNRKI; ELKNNRKIK; LKNNRKIKS; |
| | KNNRKIKSN; NNRKIKSNE; NRKIKSNEK; RKIKSNEKN; KIKSNEKNS; |
| | IKSNEKNSL; KSNEKNSLL; SNEKNSLLE; NEKNSLLEF; EKNSLLEFI; |
| | KNSLLEFIS; NSLLEFISF; SLLEFISFF; LLEFISFFK; LEFISFFKT; |
| | EFISFFKTK; FISFFKTKL; ISFFKTKLK; SFFKTKLKQ; FFKTKLKQK; |
| | FKTKLKQKI; KTKLKQKIN; TKLKQKINF; KLKQKINFK; LKQKINFKK; |
| | KQKINFKKE; QKINFKKEL; KINFKKELM; INFKKELMK; NFKKELMKS; |
| | FKKELMKSK; KKELMKSKG; KELMKSKGI; |
| | |
| | 10 mers: |
| | MKTIKKDSEF; KTIKKDSEFY; TIKKDSEFYD; IKKDSEFYDS; |
| | KKDSEFYDSL; KDSEFYDSLA; DSEFYDSLAT; SEFYDSLATL; |
| | EFYDSLATLK; FYDSLATLKK; YDSLATLKKH; DSLATLKKHI; |
| | SLATLKKHIN; LATLKKHINK; ATLKKHINKY; TLKKHINKYI; |

LKKHINKYIE; KKHINKYIEE; KHINKYIEEK; HINKYIEEKV;
INKYIEEKVL; NKYIEEKVLK; KYIEEKVLKY; YIEEKVLKYS;
IEEKVLKYSI; EEKVLKYSIS; EKVLKYSISS; KVLKYSISSQ;
VLKYSISSQL; LKYSISSQLY; KYSISSQLYK; YSISSQLYKL;
SISSQLYKLE; ISSQLYKLEK; SSQLYKLEKP; SQLYKLEKPE;
QLYKLEKPEI; LYKLEKPEII; YKLEKPEIIE; KLEKPEIIEL;
LEKPEIIELI; EKPEIIELIK; KPEIIELIKI; PEIIELIKIS;
EIIELIKISN; IIELIKISND; IELIKISNDY; ELIKISNDYE;
LIKISNDYEK; IKISNDYEKE; KISNDYEKEK; ISNDYEKEKN;
SNDYEKEKNA; NDYEKEKNAF; DYEKEKNAFN; YEKEKNAFNT;
EKEKNAFNTS; KEKNAFNTSL; EKNAFNTSLE; KNAFNTSLEE;
NAFNTSLEEC; AFNTSLEECY; FNTSLEECYY; NTSLEECYYK;
TSLEECYYKN; SLEECYYKNT; LEECYYKNTQ; EECYYKNTQN;
ECYYKNTQNE; CYYKNTQNEA; YYKNTQNEAI; YKNTQNEAIK;
KNTQNEAIKK; NTQNEAIKKW; TQNEAIKKWI; QNEAIKKWIL;
NEAIKKWILE; EAIKKWILEI; AIKKWILEIV; IKKWILEIVR;
KKWILEIVRN; KWILEIVRNK; WILEIVRNKN; ILEIVRNKNF;
LEIVRNKNFI; EIVRNKNFIQ; IVRNKNFIQI; VRNKNFIQIS;
RNKNFIQISK; NKNFIQISKE; KNFIQISKEA; NFIQISKEAN;
FIQISKEANL; IQISKEANLN; QISKEANLNT; ISKEANLNTK;
SKEANLNTKK; KEANLNTKKL; EANLNTKKLG; ANLNTKKLGT;
NLNTKKLGTT; LNTKKLGTTY; NTKKLGTTYK; TKKLGTTYKL;
KKLGTTYKLK; KLGTTYKLKS; LGTTYKLKSS; GTTYKLKSSN;
TTYKLKSSNF; TYKLKSSNFL; YKLKSSNFLK; KLKSSNFLKL;
LKSSNFLKLI; KSSNFLKLIE; SSNFLKLIEI; SNFLKLIEIQ;
NFLKLIEIQN; FLKLIEIQNS; LKLIEIQNSP; KLIEIQNSPY;
LIEIQNSPYY; IEIQNSPYYS; EIQNSPYYSQ; IQNSPYYSQE;
QNSPYYSQEK; NSPYYSQEKK; SPYYSQEKKD; PYYSQEKKDI;
YYSQEKKDIY; YSQEKKDIYK; SQEKKDIYKQ; QEKKDIYKQI;
EKKDIYKQII; KKDIYKQIIL; KDIYKQIILN; DIYKQIILNF;
IYKQIILNFS; YKQIILNFSS; KQIILNFSSN; QIILNFSSNI;
IILNFSSNIN; ILNFSSNINI; LNFSSNINID; NFSSNINIDS;
FSSNINIDSL; SSNINIDSLE; SNINIDSLEQ; NINIDSLEQT;
INIDSLEQTI; NIDSLEQTID; IDSLEQTIDI; DSLEQTIDIL;
SLEQTIDILV; LEQTIDILVA; EQTIDILVAV; QTIDILVAVR;
TIDILVAVRN; IDILVAVRNR; DILVAVRNRN; ILVAVRNRNK;
LVAVRNRNKI; VAVRNRNKIK; AVRNRNKIKI; VRNRNKIKIL;
RNRNKIKILN; NRNKIKILNI; RNKIKILNIL; NKIKILNILN;
KIKILNILNK; IKILNILNKN; KILNILNKNL; ILNILNKNLK;
LNILNKNLKY; NILNKNLKYR; ILNKNLKYRP; LNKNLKYRPE;
NKNLKYRPEN; KNLKYRPENQ; NLKYRPENQK; LKYRPENQKV;
KYRPENQKVF; YRPENQKVFK; RPENQKVFKS; PENQKVFKSS;
ENQKVFKSSL; NQKVFKSSLT; QKVFKSSLTN; KVFKSSLTNK;
VFKSSLTNKE; FKSSLTNKES; KSSLTNKESR; SSLTNKESRL;
SLTNKESRLI; LTNKESRLIK; TNKESRLIKL; NKESRLIKLK;
KESRLIKLKR; ESRLIKLKRM; SRLIKLKRML; RLIKLKRMLI;
LIKLKRMLIL; IKLKRMLILT; KLKRMLILTY; LKRMLILTYW;
KRMLILTYWP; RMLILTYWPV; MLILTYWPVG; LILTYWPVGC;
ILTYWPVGCL; LTYWPVGCLS; TYWPVGCLSK; YWPVGCLSKN;
WPVGCLSKNI; PVGCLSKNIF; VGCLSKNIFI; GCLSKNIFIK;
CLSKNIFIKI; LSKNIFIKIL; SKNIFIKILI; KNIFIKILIK;

NIFIKILIKX; IFIKILIKXY; FIKILIKXYK; IKILIKXYKY;
KILIKXYKYL; ILIKXYKYLE; LIKXYKYLEE; IKXYKYLEEE;
KXYKYLEEEI; XYKYLEEEIL; YKYLEEEILA; KYLEEEILAL;
YLEEEILALK; LEEEILALKY; EEEILALKYN; EEILALKYNE;
EILALKYNEI; ILALKYNEIL; LALKYNEILN; ALKYNEILNY;
LKYNEILNYL; KYNEILNYLR; YNEILNYLRA; NEILNYLRAL;
EILNYLRALK; ILNYLRALKT; LNYLRALKTL; NYLRALKTLS;
YLRALKTLSL; LRALKTLSLN; RALKTLSLNE; ALKTLSLNEI;
LKTLSLNEIF; KTLSLNEIFY; TLSLNEIFYK; LSLNEIFYKG;
SLNEIFYKGS; LNEIFYKGSS; NEIFYKGSSK; EIFYKGSSKN;
IFYKGSSKNI; FYKGSSKNIS; YKGSSKNISF; KGSSKNISFN;
GSSKNISFNY; SSKNISFNYF; SKNISFNYFF; KNISFNYFFS;
NISFNYFFSD; ISFNYFFSDF; SFNYFFSDFT; FNYFFSDFTQ;
NYFFSDFTQY; YFFSDFTQYA; FFSDFTQYAP; FSDFTQYAPK;
SDFTQYAPKD; DFTQYAPKDF; FTQYAPKDFQ; TQYAPKDFQD;
QYAPKDFQDT; YAPKDFQDTL; APKDFQDTLK; PKDFQDTLKC;
KDFQDTLKCY; DFQDTLKCYL; FQDTLKCYLY; QDTLKCYLYV;
DTLKCYLYVI; TLKCYLYVIE; LKCYLYVIEK; KCYLYVIEKT;
CYLYVIEKTI; YLYVIEKTIT; LYVIEKTITK; YVIEKTITKK;
VIEKTITKKY; IEKTITKKYL; EKTITKKYLT; KTITKKYLTW;
TITKKYLTWF; ITKKYLTWFF; TKKYLTWFFK; KKYLTWFFKD;
KYLTWFFKDK; YLTWFFKDKI; LTWFFKDKIS; TWFFKDKISN;
WFFKDKISNN; FFKDKISNNW; FKDKISNNWE; KDKISNNWES;
DKISNNWESF; KISNNWESFI; ISNNWESFID; SNNWESFIDA;
NNWESFIDAL; NWESFIDALE; WESFIDALEY; ESFIDALEYI;
SFIDALEYIE; FIDALEYIEK; IDALEYIEKH; DALEYIEKHK;
ALEYIEKHKL; LEYIEKHKLI; EYIEKHKLIN; YIEKHKLINI;
IEKHKLINIK; EKHKLINIKE; KHKLINIKEK; HKLINIKEKI;
KLINIKEKIK; LINIKEKIKE; INIKEKIKEI; NIKEKIKEII;
IKEKIKEIIT; KEKIKEIITA; EKIKEIITAK; KIKEIITAKF;
IKEIITAKFQ; KEIITAKFQT; EIITAKFQTE; IITAKFQTED;
ITAKFQTEDF; TAKFQTEDFF; AKFQTEDFFI; KFQTEDFFIS;
FQTEDFFISF; QTEDFFISFD; TEDFFISFDK; EDFFISFDKT;
DFFISFDKTN; FFISFDKTNF; FISFDKTNFN; ISFDKTNFNP;
SFDKTNFNPY; FDKTNFNPYE; DKTNFNPYES; KTNFNPYESS;
TNFNPYESSI; NFNPYESSIF; FNPYESSIFK; NPYESSIFKE;
PYESSIFKER; YESSIFKERY; ESSIFKERYI; SSIFKERYIK;
SIFKERYIKS; IFKERYIKSA; FKERYIKSAF; KERYIKSAFL;
ERYIKSAFLE; RYIKSAFLEI; YIKSAFLEII; IKSAFLEIIM;
KSAFLEIIMH; SAFLEIIMHY; AFLEIIMHYV; FLEIIMHYVK;
LEIIMHYVKK; EIIMHYVKKN; IIMHYVKKNS; IMHYVKKNSQ;
MHYVKKNSQN; HYVKKNSQNI; YVKKNSQNIE; VKKNSQNIET;
KKNSQNIETY; KNSQNIETYG; NSQNIETYGW; SQNIETYGWI;
QNIETYGWIA; NIETYGWIAF; IETYGWIAFY; ETYGWIAFYI;
TYGWIAFYIY; YGWIAFYIYA; GWIAFYIYAD; WIAFYIYADN;
IAFYIYADNK; AFYIYADNKD; FYIYADNKDK; YIYADNKDKK;
IYADNKDKKI; YADNKDKKIF; ADNKDKKIFC; DNKDKKIFCQ;
NKDKKIFCQR; KDKKIFCQRM; DKKIFCQRMK; KKIFCQRMKE;
KIFCQRMKEF; IFCQRMKEFF; FCQRMKEFFK; CQRMKEFFKS;
QRMKEFFKSK; RMKEFFKSKT; MKEFFKSKTF; KEFFKSKTFE;
EFFKSKTFEI; FFKSKTFEIQ; FKSKTFEIQN; KSKTFEIQNQ;

SKTFEIQNQI; KTFEIQNQIF; TFEIQNQIFV; FEIQNQIFVY;
EIQNQIFVYF; IQNQIFVYFL; QNQIFVYFLS; NQIFVYFLSF;
QIFVYFLSFY; IFVYFLSFYP; FVYFLSFYPN; VYFLSFYPNI;
YFLSFYPNIE; FLSFYPNIEY; LSFYPNIEYK; SFYPNIEYKH;
FYPNIEYKHF; YPNIEYKHFK; PNIEYKHFKF; NIEYKHFKFI;
IEYKHFKFIS; EYKHFKFISD; YKHFKFISDI; KHFKFISDIL;
HFKFISDILL; FKFISDILLY; KFISDILLYL; FISDILLYLD;
ISDILLYLDE; SDILLYLDEN; DILLYLDENK; ILLYLDENKI;
LLYLDENKIT; LYLDENKITI; YLDENKITIP; LDENKITIPK;
DENKITIPKK; ENKITIPKKI; NKITIPKKII; KITIPKKIIK;
ITIPKKIIKI; TIPKKIIKIQ; IPKKIIKIQK; PKKIIKIQKI;
KKIIKIQKIN; KIIKIQKINP; IIKIQKINPN; IKIQKINPNQ;
KIQKINPNQL; IQKINPNQLD; QKINPNQLDY; KINPNQLDYQ;
INPNQLDYQK; NPNQLDYQKS; PNQLDYQKSY; NQLDYQKSYL;
QLDYQKSYLL; LDYQKSYLLI; DYQKSYLLIK; YQKSYLLIKS;
QKSYLLIKSL; KSYLLIKSLK; SYLLIKSLKT; YLLIKSLKTR;
LLIKSLKTRS; LIKSLKTRSR; IKSLKTRSRI; KSLKTRSRIL;
SLKTRSRILK; LKTRSRILKI; KTRSRILKII; TRSRILKIII;
RSRILKIIIK; SRILKIIIKN; RILKIIIKNI; ILKIIIKNIR;
LKIIIKNIRF; KIIIKNIRFN; IIIKNIRFNL; IIKNIRFNLI;
IKNIRFNLIE; KNIRFNLIEK; NIRFNLIEKL; IRFNLIEKLE;
RFNLIEKLED; FNLIEKLEDE; NLIEKLEDEN; LIEKLEDENE;
IEKLEDENEK; EKLEDENEKK; KLEDENEKKL; LEDENEKKLL;
EDENEKKLLP; DENEKKLLPA; ENEKKLLPAM; NEKKLLPAMC;
EKKLLPAMCY; KKLLPAMCYL; KLLPAMCYLI; LLPAMCYLIY;
LPAMCYLIYC; PAMCYLIYCE; AMCYLIYCEN; MCYLIYCENL;
CYLIYCENLV; YLIYCENLVE; LIYCENLVEL; IYCENLVELK;
YCENLVELKN; CENLVELKNN; ENLVELKNNR; NLVELKNNRK;
LVELKNNRKI; VELKNNRKIK; ELKNNRKIKS; LKNNRKIKSN;
KNNRKIKSNE; NNRKIKSNEK; NRKIKSNEKN; RKIKSNEKNS;
KIKSNEKNSL; IKSNEKNSLL; KSNEKNSLLE; SNEKNSLLEF;
NEKNSLLEFI; EKNSLLEFIS; KNSLLEFISF; NSLLEFISFF;
SLLEFISFFK; LLEFISFFKT; LEFISFFKTK; EFISFFKTKL;
FISFFKTKLK; ISFFKTKLKQ; SFFKTKLKQK; FFKTKLKQKI;
FKTKLKQKIN; KTKLKQKINF; TKLKQKINFK; KLKQKINFKK;
LKQKINFKKE; KQKINFKKEL; QKINFKKELM; KINFKKELMK;
INFKKELMKS; NFKKELMKSK; FKKELMKSKG; KKELMKSKGI;

11 mers:
MKTIKKDSEFY; KTIKKDSEFYD; TIKKDSEFYDS; IKKDSEFYDSL;
KKDSEFYDSLA; KDSEFYDSLAT; DSEFYDSLATL; SEFYDSLATLK;
EFYDSLATLKK; FYDSLATLKKH; YDSLATLKKHI; DSLATLKKHIN;
SLATLKKHINK; LATLKKHINKY; ATLKKHINKYI; TLKKHINKYIE;
LKKHINKYIEE; KKHINKYIEEK; KHINKYIEEKV; HINKYIEEKVL;
INKYIEEKVLK; NKYIEEKVLKY; KYIEEKVLKYS; YIEEKVLKYSI;
IEEKVLKYSIS; EEKVLKYSISS; EKVLKYSISSQ; KVLKYSISSQL;
VLKYSISSQLY; LKYSISSQLYK; KYSISSQLYKL; YSISSQLYKLE;
SISSQLYKLEK; ISSQLYKLEKP; SSQLYKLEKPE; SQLYKLEKPEI;
QLYKLEKPEII; LYKLEKPEIIE; YKLEKPEIIEL; KLEKPEIIELI;
LEKPEIIELIK; EKPEIIELIKI; KPEIIELIKIS; PEIIELIKISN;
EIIELIKISND; IIELIKISNDY; IELIKISNDYE; ELIKISNDYEK;

LIKISNDYEKE; IKISNDYEKEK; KISNDYEKEKN; ISNDYEKEKNA;
SNDYEKEKNAF; NDYEKEKNAFN; DYEKEKNAFNT; YEKEKNAFNTS;
EKEKNAFNTSL; KEKNAFNTSLE; EKNAFNTSLEE; KNAFNTSLEEC;
NAFNTSLEECY; AFNTSLEECYY; FNTSLEECYYK; NTSLEECYYKN;
TSLEECYYKNT; SLEECYYKNTQ; LEECYYKNTQN; EECYYKNTQNE;
ECYYKNTQNEA; CYYKNTQNEAI; YYKNTQNEAIK; YKNTQNEAIKK;
KNTQNEAIKKW; NTQNEAIKKWI; TQNEAIKKWIL; QNEAIKKWILE;
NEAIKKWILEI; EAIKKWILEIV; AIKKWILEIVR; IKKWILEIVRN;
KKWILEIVRNK; KWILEIVRNKN; WILEIVRNKNF; ILEIVRNKNFI;
LEIVRNKNFIQ; EIVRNKNFIQI; IVRNKNFIQIS; VRNKNFIQISK;
RNKNFIQISKE; NKNFIQISKEA; KNFIQISKEAN; NFIQISKEANL;
FIQISKEANLN; IQISKEANLNT; QISKEANLNTK; ISKEANLNTKK;
SKEANLNTKKL; KEANLNTKKLG; EANLNTKKLGT; ANLNTKKLGTT;
NLNTKKLGTTY; LNTKKLGTTYK; NTKKLGTTYKL; TKKLGTTYKLK;
KKLGTTYKLKS; KLGTTYKLKSS; LGTTYKLKSSN; GTTYKLKSSNF;
TTYKLKSSNFL; TYKLKSSNFLK; YKLKSSNFLKL; KLKSSNFLKLI;
LKSSNFLKLIE; KSSNFLKLIEI; SSNFLKLIEIQ; SNFLKLIEIQN;
NFLKLIEIQNS; FLKLIEIQNSP; LKLIEIQNSPY; KLIEIQNSPYY;
LIEIQNSPYYS; IEIQNSPYYSQ; EIQNSPYYSQE; IQNSPYYSQEK;
QNSPYYSQEKK; NSPYYSQEKKD; SPYYSQEKKDI; PYYSQEKKDIY;
YYSQEKKDIYK; YSQEKKDIYKQ; SQEKKDIYKQI; QEKKDIYKQII;
EKKDIYKQIIL; KKDIYKQIILN; KDIYKQIILNF; DIYKQIILNFS;
IYKQIILNFSS; YKQIILNFSSN; KQIILNFSSNI; QIILNFSSNIN;
IILNFSSNINI; ILNFSSNINID; LNFSSNINIDS; NFSSNINIDSL;
FSSNINIDSLE; SSNINIDSLEQ; SNINIDSLEQT; NINIDSLEQTI;
INIDSLEQTID; NIDSLEQTIDI; IDSLEQTIDIL; DSLEQTIDILV;
SLEQTIDILVA; LEQTIDILVAV; EQTIDILVAVR; QTIDILVAVRN;
TIDILVAVRNR; IDILVAVRNRN; DILVAVRNRNK; ILVAVRNRNKI;
LVAVRNRNKIK; VAVRNRNKIKI; AVRNRNKIKIL; VRNRNKIKILN;
RNRNKIKILNI; NRNKIKILNIL; RNKIKILNILN; NKIKILNILNK;
KIKILNILNKN; IKILNILNKNL; KILNILNKNLK; ILNILNKNLKY;
LNILNKNLKYR; NILNKNLKYRP; ILNKNLKYRPE; LNKNLKYRPEN;
NKNLKYRPENQ; KNLKYRPENQK; NLKYRPENQKV; LKYRPENQKVF;
KYRPENQKVFK; YRPENQKVFKS; RPENQKVFKSS; PENQKVFKSSL;
ENQKVFKSSLT; NQKVFKSSLTN; QKVFKSSLTNK; KVFKSSLTNKE;
VFKSSLTNKES; FKSSLTNKESR; KSSLTNKESRL; SSLTNKESRLI;
SLTNKESRLIK; LTNKESRLIKL; TNKESRLIKLK; NKESRLIKLKR;
KESRLIKLKRM; ESRLIKLKRML; SRLIKLKRMLI; RLIKLKRMLIL;
LIKLKRMLILT; IKLKRMLILTY; KLKRMLILTYW; LKRMLILTYWP;
KRMLILTYWPV; RMLILTYWPVG; MLILTYWPVGC; LILTYWPVGCL;
ILTYWPVGCLS; LTYWPVGCLSK; TYWPVGCLSKN; YWPVGCLSKNI;
WPVGCLSKNIF; PVGCLSKNIFI; VGCLSKNIFIK; GCLSKNIFIKI;
CLSKNIFIKIL; LSKNIFIKILI; SKNIFIKILIK; KNIFIKILIKX;
NIFIKILIKXY; IFIKILIKXYK; FIKILIKXYKY; IKILIKXYKYL;
KILIKXYKYLE; ILIKXYKYLEE; LIKXYKYLEEE; IKXYKYLEEEI;
KXYKYLEEEIL; XYKYLEEEILA; YKYLEEEILAL; KYLEEEILALK;
YLEEEILALKY; LEEEILALKYN; EEEILALKYNE; EEILALKYNEI;
EILALKYNEIL; ILALKYNEILN; LALKYNEILNY; ALKYNEILNYL;
LKYNEILNYLR; KYNEILNYLRA; YNEILNYLRAL; NEILNYLRALK;
EILNYLRALKT; ILNYLRALKTL; LNYLRALKTLS; NYLRALKTLSL;
YLRALKTLSLN; LRALKTLSLNE; RALKTLSLNEI; ALKTLSLNEIF;

| | | | |
|---|---|---|---|
| LKTLSLNEIFY; | KTLSLNEIFYK; | TLSLNEIFYKG; | LSLNEIFYKGS; |
| SLNEIFYKGSS; | LNEIFYKGSSK; | NEIFYKGSSKN; | EIFYKGSSKNI; |
| IFYKGSSKNIS; | FYKGSSKNISF; | YKGSSKNISFN; | KGSSKNISFNY; |
| GSSKNISFNYF; | SSKNISFNYFF; | SKNISFNYFFS; | KNISFNYFFSD; |
| NISFNYFFSDF; | ISFNYFFSDFT; | SFNYFFSDFTQ; | FNYFFSDFTQY; |
| NYFFSDFTQYA; | YFFSDFTQYAP; | FFSDFTQYAPK; | FSDFTQYAPKD; |
| SDFTQYAPKDF; | DFTQYAPKDFQ; | FTQYAPKDFQD; | TQYAPKDFQDT; |
| QYAPKDFQDTL; | YAPKDFQDTLK; | APKDFQDTLKC; | PKDFQDTLKCY; |
| KDFQDTLKCYL; | DFQDTLKCYLY; | FQDTLKCYLYV; | QDTLKCYLYVI; |
| DTLKCYLYVIE; | TLKCYLYVIEK; | LKCYLYVIEKT; | KCYLYVIEKTI; |
| CYLYVIEKTIT; | YLYVIEKTITK; | LYVIEKTITKK; | YVIEKTITKKY; |
| VIEKTITKKYL; | IEKTITKKYLT; | EKTITKKYLTW; | KTITKKYLTWF; |
| TITKKYLTWFF; | ITKKYLTWFFK; | TKKYLTWFFKD; | KKYLTWFFKDK; |
| KYLTWFFKDKI; | YLTWFFKDKIS; | LTWFFKDKISN; | TWFFKDKISNN; |
| WFFKDKISNNW; | FFKDKISNNWE; | FKDKISNNWES; | KDKISNNWESF; |
| DKISNNWESFI; | KISNNWESFID; | ISNNWESFIDA; | SNNWESFIDAL; |
| NNWESFIDALE; | NWESFIDALEY; | WESFIDALEYI; | ESFIDALEYIE; |
| SFIDALEYIEK; | FIDALEYIEKH; | IDALEYIEKHK; | DALEYIEKHKL; |
| ALEYIEKHKLI; | LEYIEKHKLIN; | EYIEKHKLINI; | YIEKHKLINIK; |
| IEKHKLINIKE; | EKHKLINIKEK; | KHKLINIKEKI; | HKLINIKEKIK; |
| KLINIKEKIKE; | LINIKEKIKEI; | INIKEKIKEII; | NIKEKIKEIIT; |
| IKEKIKEIITA; | KEKIKEIITAK; | EKIKEIITAKF; | KIKEIITAKFQ; |
| IKEIITAKFQT; | KEIITAKFQTE; | EIITAKFQTED; | IITAKFQTEDF; |
| ITAKFQTEDFF; | TAKFQTEDFFI; | AKFQTEDFFIS; | KFQTEDFFISF; |
| FQTEDFFISFD; | QTEDFFISFDK; | TEDFFISFDKT; | EDFFISFDKTN; |
| DFFISFDKTNF; | FFISFDKTNFN; | FISFDKTNFNP; | ISFDKTNFNPY; |
| SFDKTNFNPYE; | FDKTNFNPYES; | DKTNFNPYESS; | KTNFNPYESSI; |
| TNFNPYESSIF; | NFNPYESSIFK; | FNPYESSIFKE; | NPYESSIFKER; |
| PYESSIFKERY; | YESSIFKERYI; | ESSIFKERYIK; | SSIFKERYIKS; |
| SIFKERYIKSA; | IFKERYIKSAF; | FKERYIKSAFL; | KERYIKSAFLE; |
| ERYIKSAFLEI; | RYIKSAFLEII; | YIKSAFLEIIM; | IKSAFLEIIMH; |
| KSAFLEIIMHY; | SAFLEIIMHYV; | AFLEIIMHYVK; | FLEIIMHYVKK; |
| LEIIMHYVKKN; | EIIMHYVKKNS; | IIMHYVKKNSQ; | IMHYVKKNSQN; |
| MHYVKKNSQNI; | HYVKKNSQNIE; | YVKKNSQNIET; | VKKNSQNIETY; |
| KKNSQNIETYG; | KNSQNIETYGW; | NSQNIETYGWI; | SQNIETYGWIA; |
| QNIETYGWIAF; | NIETYGWIAFY; | IETYGWIAFYI; | ETYGWIAFYIY; |
| TYGWIAFYIYA; | YGWIAFYIYAD; | GWIAFYIYADN; | WIAFYIYADNK; |
| IAFYIYADNKD; | AFYIYADNKDK; | FYIYADNKDKK; | YIYADNKDKKI; |
| IYADNKDKKIF; | YADNKDKKIFC; | ADNKDKKIFCQ; | DNKDKKIFCQR; |
| NKDKKIFCQRM; | KDKKIFCQRMK; | DKKIFCQRMKE; | KKIFCQRMKEF; |
| KIFCQRMKEFF; | IFCQRMKEFFK; | FCQRMKEFFKS; | CQRMKEFFKSK; |
| QRMKEFFKSKT; | RMKEFFKSKTF; | MKEFFKSKTFE; | KEFFKSKTFEI; |
| EFFKSKTFEIQ; | FFKSKTFEIQN; | FKSKTFEIQNQ; | KSKTFEIQNQI; |
| SKTFEIQNQIF; | KTFEIQNQIFV; | TFEIQNQIFVY; | FEIQNQIFVYF; |
| EIQNQIFVYFL; | IQNQIFVYFLS; | QNQIFVYFLSF; | NQIFVYFLSFY; |
| QIFVYFLSFYP; | IFVYFLSFYPN; | FVYFLSFYPNI; | VYFLSFYPNIE; |
| YFLSFYPNIEY; | FLSFYPNIEYK; | LSFYPNIEYKH; | SFYPNIEYKHF; |
| FYPNIEYKHFK; | YPNIEYKHFKF; | PNIEYKHFKFI; | NIEYKHFKFIS; |
| IEYKHFKFISD; | EYKHFKFISDI; | YKHFKFISDIL; | KHFKFISDILL; |
| HFKFISDILLY; | FKFISDILLYL; | KFISDILLYLD; | FISDILLYLDE; |
| ISDILLYLDEN; | SDILLYLDENK; | DILLYLDENKI; | ILLYLDENKIT; |

| | |
|---|---|
| | LLYLDENKITI; LYLDENKITIP; YLDENKITIPK; LDENKITIPKK; DENKITIPKKI; ENKITIPKKII; NKITIPKKIIK; KITIPKKIIKI; ITIPKKIIKIQ; TIPKKIIKIQK; IPKKIIKIQKI; PKKIIKIQKIN; KKIIKIQKINP; KIIKIQKINPN; IIKIQKINPNQ; IKIQKINPNQL; KIQKINPNQLD; IQKINPNQLDY; QKINPNQLDYQ; KINPNQLDYQK; INPNQLDYQKS; NPNQLDYQKSY; PNQLDYQKSYL; NQLDYQKSYLL; QLDYQKSYLLI; LDYQKSYLLIK; DYQKSYLLIKS; YQKSYLLIKSL; QKSYLLIKSLK; KSYLLIKSLKT; SYLLIKSLKTR; YLLIKSLKTRS; LLIKSLKTRSR; LIKSLKTRSRI; IKSLKTRSRIL; KSLKTRSRILK; SLKTRSRILKI; LKTRSRILKII; KTRSRILKIII; TRSRILKIIIK; RSRILKIIIKN; SRILKIIIKNI; RILKIIIKNIR; ILKIIIKNIRF; LKIIIKNIRFN; KIIIKNIRFNL; IIIKNIRFNLI; IIKNIRFNLIE; IKNIRFNLIEK; KNIRFNLIEKL; NIRFNLIEKLE; IRFNLIEKLED; RFNLIEKLEDE; FNLIEKLEDEN; NLIEKLEDENE; LIEKLEDENEK; IEKLEDENEKK; EKLEDENEKKL; KLEDENEKKLL; LEDENEKKLLP; EDENEKKLLPA; DENEKKLLPAM; ENEKKLLPAMC; NEKKLLPAMCY; EKKLLPAMCYL; KKLLPAMCYLI; KLLPAMCYLIY; LLPAMCYLIYC; LPAMCYLIYCE; PAMCYLIYCEN; AMCYLIYCENL; MCYLIYCENLV; CYLIYCENLVE; YLIYCENLVEL; LIYCENLVELK; IYCENLVELKN; YCENLVELKNN; CENLVELKNNR; ENLVELKNNRK; NLVELKNNRKI; LVELKNNRKIK; VELKNNRKIKS; ELKNNRKIKSN; LKNNRKIKSNE; KNNRKIKSNEK; NNRKIKSNEKN; NRKIKSNEKNS; RKIKSNEKNSL; KIKSNEKNSLL; IKSNEKNSLLE; KSNEKNSLLEF; SNEKNSLLEFI; NEKNSLLEFIS; EKNSLLEFISF; KNSLLEFISFF; NSLLEFISFFK; SLLEFISFFKT; LLEFISFFKTK; LEFISFFKTKL; EFISFFKTKLK; FISFFKTKLKQ; ISFFKTKLKQK; SFFKTKLKQKI; FFKTKLKQKIN; FKTKLKQKINF; KTKLKQKINFK; TKLKQKINFKK; KLKQKINFKKE; LKQKINFKKEL; KQKINFKKELM; QKINFKKELMK; KINFKKELMKS; INFKKELMKSK; NFKKELMKSKG; FKKELMKSKGI; |
| Seq 29 | SEQ ID NO 35914-36939/<br>8 mers:<br>MITQQDYT; ITQQDYTE; TQQDYTEI; QQDYTEIK; QDYTEIKK; DYTEIKKK; YTEIKKKL; TEIKKKLE; EIKKKLEN; IKKKLENT; KKKLENTK; KKLENTKT; KLENTKTR; LENTKTRI; ENTKTRIQ; NTKTRIQF; TKTRIQFR; KTRIQFRN; TRIQFRNG; RIQFRNGN; IQFRNGNK; QFRNGNKI; FRNGNKID; RNGNKIDS; NGNKIDSL; GNKIDSLG; NKIDSLGY; KIDSLGYQ; IDSLGYQG; DSLGYQGS; SLGYQGSL; LGYQGSLG; GYQGSLGE; YQGSLGEP; QGSLGEPI; GSLGEPIL; SLGEPILL; LGEPILLK; GEPILLKD; EPILLKDK; PILLKDKL; ILLKDKLT; LLKDKLTF; LKDKLTFS; KDKLTFSI; DKLTFSIC; KLTFSICD; LTFSICDG; TFSICDGK; FSICDGKN; SICDGKNI; ICDGKNID; CDGKNIDN; DGKNIDNR; GKNIDNRK; KNIDNRKE; NIDNRKEY; IDNRKEYE; DNRKEYEI; NRKEYEIT; RKEYEITK; KEYEITKN; EYEITKNA; YEITKNAP; EITKNAPS; ITKNAPSL; TKNAPSLD; KNAPSLDS; NAPSLDSL; APSLDSLI; PSLDSLIE; SLDSLIEY; LDSLIEYL; DSLIEYLE; SLIEYLEH; LIEYLEHM; IEYLEHMI; EYLEHMIL; YLEHMILF; LEHMILFF; EHMILFFL; HMILFFLE; MILFFLEK; ILFFLEKT; LFFLEKTP; FFLEKTPV; FLEKTPVG; LEKTPVGT; EKTPVGTL; KTPVGTLT; |

TPVGTLTA; PVGTLTAK; VGTLTAKF; GTLTAKFA; TLTAKFAM;
LTAKFAME; TAKFAMEG; AKFAMEGS; KFAMEGSI; FAMEGSIL;
AMEGSILT; MEGSILTR; EGSILTRS; GSILTRSK; SILTRSKL;
ILTRSKLI; LTRSKLIQ; TRSKLIQA; RSKLIQAF; SKLIQAFT;
KLIQAFTN; LIQAFTNT; IQAFTNTN; QAFTNTNK; AFTNTNKF;
FTNTNKFC; TNTNKFCI; NTNKFCIP; TNKFCIPD; NKFCIPDG;
KFCIPDGR; FCIPDGRS; CIPDGRSL; IPDGRSLP; PDGRSLPS;
DGRSLPSN; GRSLPSNC; RSLPSNCY; SLPSNCYA; LPSNCYAY;
PSNCYAYK; SNCYAYKV; NCYAYKVL; CYAYKVLG; YAYKVLGI;
AYKVLGIS; YKVLGISS; KVLGISSA; VLGISSAP; LGISSAPK;
GISSAPKL; ISSAPKLS; SSAPKLSG; SAPKLSGR; APKLSGRF;
PKLSGRFL; KLSGRFLR; LSGRFLRH; SGRFLRHY; GRFLRHYD;
RFLRHYDS; FLRHYDSG; LRHYDSGG; RHYDSGGS; HYDSGGST;
YDSGGSTD; DSGGSTDS; SGGSTDSD; GGSTDSDG; GSTDSDGT;
STDSDGTR; TDSDGTRS; DSDGTRSL; SDGTRSLG; DGTRSLGH;
GTRSLGHT; TRSLGHTQ; RSLGHTQE; SLGHTQED; LGHTQEDS;
GHTQEDSY; HTQEDSYK; TQEDSYKN; QEDSYKNH; EDSYKNHD;
DSYKNHDH; SYKNHDHD; YKNHDHDL; KNHDHDLD; NHDHDLDF;
HDHDLDFS; DHDLDFSR; HDLDFSRI; DLDFSRIN; LDFSRINF;
DFSRINFK; FSRINFKG; SRINFKGK; RINFKGKL; INFKGKLV;
NFKGKLVK; FKGKLVKR; KGKLVKRM; GKLVKRMT; KLVKRMTM;
LVKRMTMI; VKRMTMIY; KRMTMIYR; RMTMIYRS; MTMIYRSA;
TMIYRSAD; MIYRSADT; IYRSADTW; YRSADTWY; RSADTWYT;
SADTWYTW; ADTWYTWY; DTWYTWYW; TWYTWYWA; WYTWYWAE;
YTWYWAEE; TWYWAEEI; WYWAEEIM; YWAEEIMS; WAEEIMSP;
AEEIMSPF; EEIMSPFI; EIMSPFIT; IMSPFITG; MSPFITGY;
SPFITGYS; PFITGYSY; FITGYSYE; ITGYSYEE; TGYSYEEI;
GYSYEEID; YSYEEIDP; SYEEIDPE; YEEIDPEY; EEIDPEYL;
EIDPEYLL; IDPEYLLP; DPEYLLPP; PEYLLPPF; EYLLPPFT;
YLLPPFTS; LLPPFTSQ; LPPFTSQS; PPFTSQSG; PFTSQSGS;
FTSQSGSK; TSQSGSKE; SQSGSKET; QSGSKETK; SGSKETKP;
GSKETKPK; SKETKPKN; KETKPKNT; ETKPKNTC; TKPKNTCY;
KPKNTCYI; PKNTCYIS; KNTCYISF; NTCYISFY; TCYISFYR;
CYISFYRY; YISFYRYD; ISFYRYDL;

9 mers:
MITQQDYTE; ITQQDYTEI; TQQDYTEIK; QQDYTEIKK; QDYTEIKKK;
DYTEIKKKL; YTEIKKKLE; TEIKKKLEN; EIKKKLENT; IKKKLENTK;
KKKLENTKT; KKLENTKTR; KLENTKTRI; LENTKTRIQ; ENTKTRIQF;
NTKTRIQFR; TKTRIQFRN; KTRIQFRNG; TRIQFRNGN; RIQFRNGNK;
IQFRNGNKI; QFRNGNKID; FRNGNKIDS; RNGNKIDSL; NGNKIDSLG;
GNKIDSLGY; NKIDSLGYQ; KIDSLGYQG; IDSLGYQGS; DSLGYQGSL;
SLGYQGSLG; LGYQGSLGE; GYQGSLGEP; YQGSLGEPI; QGSLGEPIL;
GSLGEPILL; SLGEPILLK; LGEPILLKD; GEPILLKDK; EPILLKDKL;
PILLKDKLT; ILLKDKLTF; LLKDKLTFS; LKDKLTFSI; KDKLTFSIC;
DKLTFSICD; KLTFSICDG; LTFSICDGK; TFSICDGKN; FSICDGKNI;
SICDGKNID; ICDGKNIDN; CDGKNIDNR; DGKNIDNRK; GKNIDNRKE;
KNIDNRKEY; NIDNRKEYE; IDNRKEYEI; DNRKEYEIT; NRKEYEITK;
RKEYEITKN; KEYEITKNA; EYEITKNAP; YEITKNAPS; EITKNAPSL;
ITKNAPSLD; TKNAPSLDS; KNAPSLDSL; NAPSLDSLI; APSLDSLIE;
PSLDSLIEY; SLDSLIEYL; LDSLIEYLE; DSLIEYLEH; SLIEYLEHM;

LIEYLEHMI; IEYLEHMIL; EYLEHMILF; YLEHMILFF; LEHMILFFL;
EHMILFFLE; HMILFFLEK; MILFFLEKT; ILFFLEKTP; LFFLEKTPV;
FFLEKTPVG; FLEKTPVGT; LEKTPVGTL; EKTPVGTLT; KTPVGTLTA;
TPVGTLTAK; PVGTLTAKF; VGTLTAKFA; GTLTAKFAM; TLTAKFAME;
LTAKFAMEG; TAKFAMEGS; AKFAMEGSI; KFAMEGSIL; FAMEGSILT;
AMEGSILTR; MEGSILTRS; EGSILTRSK; GSILTRSKL; SILTRSKLI;
ILTRSKLIQ; LTRSKLIQA; TRSKLIQAF; RSKLIQAFT; SKLIQAFTN;
KLIQAFTNT; LIQAFTNTN; IQAFTNTNK; QAFTNTNKF; AFTNTNKFC;
FTNTNKFCI; TNTNKFCIP; NTNKFCIPD; TNKFCIPDG; NKFCIPDGR;
KFCIPDGRS; FCIPDGRSL; CIPDGRSLP; IPDGRSLPS; PDGRSLPSN;
DGRSLPSNC; GRSLPSNCY; RSLPSNCYA; SLPSNCYAY; LPSNCYAYK;
PSNCYAYKV; SNCYAYKVL; NCYAYKVLG; CYAYKVLGI; YAYKVLGIS;
AYKVLGISS; YKVLGISSA; KVLGISSAP; VLGISSAPK; LGISSAPKL;
GISSAPKLS; ISSAPKLSG; SSAPKLSGR; SAPKLSGRF; APKLSGRFL;
PKLSGRFLR; KLSGRFLRH; LSGRFLRHY; SGRFLRHYD; GRFLRHYDS;
RFLRHYDSG; FLRHYDSGG; LRHYDSGGS; RHYDSGGST; HYDSGGSTD;
YDSGGSTDS; DSGGSTDSD; SGGSTDSDG; GGSTDSDGT; GSTDSDGTR;
STDSDGTRS; TDSDGTRSL; DSDGTRSLG; SDGTRSLGH; DGTRSLGHT;
GTRSLGHTQ; TRSLGHTQE; RSLGHTQED; SLGHTQEDS; LGHTQEDSY;
GHTQEDSYK; HTQEDSYKN; TQEDSYKNH; QEDSYKNHD; EDSYKNHDH;
DSYKNHDHD; SYKNHDHDL; YKNHDHDLD; KNHDHDLDF; NHDHDLDFS;
HDHDLDFSR; DHDLDFSRI; HDLDFSRIN; DLDFSRINF; LDFSRINFK;
DFSRINFKG; FSRINFKGK; SRINFKGKL; RINFKGKLV; INFKGKLVK;
NFKGKLVKR; FKGKLVKRM; KGKLVKRMT; GKLVKRMTM; KLVKRMTMI;
LVKRMTMIY; VKRMTMIYR; KRMTMIYRS; RMTMIYRSA; MTMIYRSAD;
TMIYRSADT; MIYRSADTW; IYRSADTWY; YRSADTWYT; RSADTWYTW;
SADTWYTWY; ADTWYTWYW; DTWYTWYWA; TWYTWYWAE; WYTWYWAEE;
YTWYWAEEI; TWYWAEEIM; WYWAEEIMS; YWAEEIMSP; WAEEIMSPF;
AEEIMSPFI; EEIMSPFIT; EIMSPFITG; IMSPFITGY; MSPFITGYS;
SPFITGYSY; PFITGYSYE; FITGYSYEE; ITGYSYEEI; TGYSYEEID;
GYSYEEIDP; YSYEEIDPE; SYEEIDPEY; YEEIDPEYL; EEIDPEYLL;
EIDPEYLLP; IDPEYLLPP; DPEYLLPPF; PEYLLPPFT; EYLLPPFTS;
YLLPPFTSQ; LLPPFTSQS; LPPFTSQSG; PPFTSQSGS; PFTSQSGSK;
FTSQSGSKE; TSQSGSKET; SQSGSKETK; QSGSKETKP; SGSKETKPK;
GSKETKPKN; SKETKPKNT; KETKPKNTC; ETKPKNTCY; TKPKNTCYI;
KPKNTCYIS; PKNTCYISF; KNTCYISFY; NTCYISFYR; TCYISFYRY;
CYISFYRYD; YISFYRYDL;

10 mers:
MITQQDYTEI; ITQQDYTEIK; TQQDYTEIKK; QQDYTEIKKK;
QDYTEIKKKL; DYTEIKKKLE; YTEIKKKLEN; TEIKKKLENT;
EIKKKLENTK; IKKKLENTKT; KKKLENTKTR; KKLENTKTRI;
KLENTKTRIQ; LENTKTRIQF; ENTKTRIQFR; NTKTRIQFRN;
TKTRIQFRNG; KTRIQFRNGN; TRIQFRNGNK; RIQFRNGNKI;
IQFRNGNKID; QFRNGNKIDS; FRNGNKIDSL; RNGNKIDSLG;
NGNKIDSLGY; GNKIDSLGYQ; NKIDSLGYQG; KIDSLGYQGS;
IDSLGYQGSL; DSLGYQGSLG; SLGYQGSLGE; LGYQGSLGEP;
GYQGSLGEPI; YQGSLGEPIL; QGSLGEPILL; GSLGEPILLK;
SLGEPILLKD; LGEPILLKDK; GEPILLKDKL; EPILLKDKLT;
PILLKDKLTF; ILLKDKLTFS; LLKDKLTFSI; LKDKLTFSIC;
KDKLTFSICD; DKLTFSICDG; KLTFSICDGK; LTFSICDGKN;

| | |
|---|---|
| TFSICDGKNI; FSICDGKNID; SICDGKNIDN; ICDGKNIDNR; CDGKNIDNRK; DGKNIDNRKE; GKNIDNRKEY; KNIDNRKEYE; NIDNRKEYEI; IDNRKEYEIT; DNRKEYEITK; NRKEYEITKN; RKEYEITKNA; KEYEITKNAP; EYEITKNAPS; YEITKNAPSL; EITKNAPSLD; ITKNAPSLDS; TKNAPSLDSL; KNAPSLDSLI; NAPSLDSLIE; APSLDSLIEY; PSLDSLIEYL; SLDSLIEYLE; LDSLIEYLEH; DSLIEYLEHM; SLIEYLEHMI; LIEYLEHMIL; IEYLEHMILF; EYLEHMILFF; YLEHMILFFL; LEHMILFFLE; EHMILFFLEK; HMILFFLEKT; MILFFLEKTP; ILFFLEKTPV; LFFLEKTPVG; FFLEKTPVGT; FLEKTPVGTL; LEKTPVGTLT; EKTPVGTLTA; KTPVGTLTAK; TPVGTLTAKF; PVGTLTAKFA; VGTLTAKFAM; GTLTAKFAME; TLTAKFAMEG; LTAKFAMEGS; TAKFAMEGSI; AKFAMEGSIL; KFAMEGSILT; FAMEGSILTR; AMEGSILTRS; MEGSILTRSK; EGSILTRSKL; GSILTRSKLI; SILTRSKLIQ; ILTRSKLIQA; LTRSKLIQAF; TRSKLIQAFT; RSKLIQAFTN; SKLIQAFTNT; KLIQAFTNTN; LIQAFTNTNK; IQAFTNTNKF; QAFTNTNKFC; AFTNTNKFCI; FTNTNKFCIP; TNTNKFCIPD; NTNKFCIPDG; TNKFCIPDGR; NKFCIPDGRS; KFCIPDGRSL; FCIPDGRSLP; CIPDGRSLPS; IPDGRSLPSN; PDGRSLPSNC; DGRSLPSNCY; GRSLPSNCYA; RSLPSNCYAY; SLPSNCYAYK; LPSNCYAYKV; PSNCYAYKVL; SNCYAYKVLG; NCYAYKVLGI; CYAYKVLGIS; YAYKVLGISS; AYKVLGISSA; YKVLGISSAP; KVLGISSAPK; VLGISSAPKL; LGISSAPKLS; GISSAPKLSG; ISSAPKLSGR; SSAPKLSGRF; SAPKLSGRFL; APKLSGRFLR; PKLSGRFLRH; KLSGRFLRHY; LSGRFLRHYD; SGRFLRHYDS; GRFLRHYDSG; RFLRHYDSGG; FLRHYDSGGS; LRHYDSGGST; RHYDSGGSTD; HYDSGGSTDS; YDSGGSTDSD; DSGGSTDSDG; SGGSTDSDGT; GGSTDSDGTR; GSTDSDGTRS; STDSDGTRSL; TDSDGTRSLG; DSDGTRSLGH; SDGTRSLGHT; DGTRSLGHTQ; GTRSLGHTQE; TRSLGHTQED; RSLGHTQEDS; SLGHTQEDSY; LGHTQEDSYK; GHTQEDSYKN; HTQEDSYKNH; TQEDSYKNHD; QEDSYKNHDH; EDSYKNHDHD; DSYKNHDHDL; SYKNHDHDLD; YKNHDHDLDF; KNHDHDLDFS; NHDHDLDFSR; HDHDLDFSRI; DHDLDFSRIN; HDLDFSRINF; DLDFSRINFK; LDFSRINFKG; DFSRINFKGK; FSRINFKGKL; SRINFKGKLV; RINFKGKLVK; INFKGKLVKR; NFKGKLVKRM; FKGKLVKRMT; KGKLVKRMTM; GKLVKRMTMI; KLVKRMTMIY; LVKRMTMIYR; VKRMTMIYRS; KRMTMIYRSA; RMTMIYRSAD; MTMIYRSADT; TMIYRSADTW; MIYRSADTWY; IYRSADTWYT; YRSADTWYTW; RSADTWYTWY; SADTWYTWYW; ADTWYTWYWA; DTWYTWYWAE; TWYTWYWAEE; WYTWYWAEEI; YTWYWAEEIM; TWYWAEEIMS; WYWAEEIMSP; YWAEEIMSPF; WAEEIMSPFI; AEEIMSPFIT; EEIMSPFITG; EIMSPFITGY; IMSPFITGYS; MSPFITGYSY; SPFITGYSYE; PFITGYSYEE; FITGYSYEEI; ITGYSYEEID; TGYSYEEIDP; GYSYEEIDPE; YSYEEIDPEY; SYEEIDPEYL; YEEIDPEYLL; EEIDPEYLLP; EIDPEYLLPP; IDPEYLLPPF; DPEYLLPPFT; PEYLLPPFTS; EYLLPPFTSQ; YLLPPFTSQS; LLPPFTSQSG; LPPFTSQSGS; PPFTSQSGSK; PFTSQSGSKE; FTSQSGSKET; TSQSGSKETK; SQSGSKETKP; QSGSKETKPK; SGSKETKPKN; GSKETKPKNT; SKETKPKNTC; KETKPKNTCY; ETKPKNTCYI; TKPKNTCYIS; KPKNTCYISF; PKNTCYISFY; |

KNTCYISFYR; NTCYISFYRY; TCYISFYRYD; CYISFYRYDL;

11 mers:
MITQQDYTEIK; ITQQDYTEIKK; TQQDYTEIKKK; QQDYTEIKKKL;
QDYTEIKKKLE; DYTEIKKKLEN; YTEIKKKLENT; TEIKKKLENTK;
EIKKKLENTKT; IKKKLENTKTR; KKKLENTKTRI; KKLENTKTRIQ;
KLENTKTRIQF; LENTKTRIQFR; ENTKTRIQFRN; NTKTRIQFRNG;
TKTRIQFRNGN; KTRIQFRNGNK; TRIQFRNGNKI; RIQFRNGNKID;
IQFRNGNKIDS; QFRNGNKIDSL; FRNGNKIDSLG; RNGNKIDSLGY;
NGNKIDSLGYQ; GNKIDSLGYQG; NKIDSLGYQGS; KIDSLGYQGSL;
IDSLGYQGSLG; DSLGYQGSLGE; SLGYQGSLGEP; LGYQGSLGEPI;
GYQGSLGEPIL; YQGSLGEPILL; QGSLGEPILLK; GSLGEPILLKD;
SLGEPILLKDK; LGEPILLKDKL; GEPILLKDKLT; EPILLKDKLTF;
PILLKDKLTFS; ILLKDKLTFSI; LLKDKLTFSIC; LKDKLTFSICD;
KDKLTFSICDG; DKLTFSICDGK; KLTFSICDGKN; LTFSICDGKNI;
TFSICDGKNID; FSICDGKNIDN; SICDGKNIDNR; ICDGKNIDNRK;
CDGKNIDNRKE; DGKNIDNRKEY; GKNIDNRKEYE; KNIDNRKEYEI;
NIDNRKEYEIT; IDNRKEYEITK; DNRKEYEITKN; NRKEYEITKNA;
RKEYEITKNAP; KEYEITKNAPS; EYEITKNAPSL; YEITKNAPSLD;
EITKNAPSLDS; ITKNAPSLDSL; TKNAPSLDSLI; KNAPSLDSLIE;
NAPSLDSLIEY; APSLDSLIEYL; PSLDSLIEYLE; SLDSLIEYLEH;
LDSLIEYLEHM; DSLIEYLEHMI; SLIEYLEHMIL; LIEYLEHMILF;
IEYLEHMILFF; EYLEHMILFFL; YLEHMILFFLE; LEHMILFFLEK;
EHMILFFLEKT; HMILFFLEKTP; MILFFLEKTPV; ILFFLEKTPVG;
LFFLEKTPVGT; FFLEKTPVGTL; FLEKTPVGTLT; LEKTPVGTLTA;
EKTPVGTLTAK; KTPVGTLTAKF; TPVGTLTAKFA; PVGTLTAKFAM;
VGTLTAKFAME; GTLTAKFAMEG; TLTAKFAMEGS; LTAKFAMEGSI;
TAKFAMEGSIL; AKFAMEGSILT; KFAMEGSILTR; FAMEGSILTRS;
AMEGSILTRSK; MEGSILTRSKL; EGSILTRSKLI; GSILTRSKLIQ;
SILTRSKLIQA; ILTRSKLIQAF; LTRSKLIQAFT; TRSKLIQAFTN;
RSKLIQAFTNT; SKLIQAFTNTN; KLIQAFTNTNK; LIQAFTNTNKF;
IQAFTNTNKFC; QAFTNTNKFCI; AFTNTNKFCIP; FTNTNKFCIPD;
TNTNKFCIPDG; NTNKFCIPDGR; TNKFCIPDGRS; NKFCIPDGRSL;
KFCIPDGRSLP; FCIPDGRSLPS; CIPDGRSLPSN; IPDGRSLPSNC;
PDGRSLPSNCY; DGRSLPSNCYA; GRSLPSNCYAY; RSLPSNCYAYK;
SLPSNCYAYKV; LPSNCYAYKVL; PSNCYAYKVLG; SNCYAYKVLGI;
NCYAYKVLGIS; CYAYKVLGISS; YAYKVLGISSA; AYKVLGISSAP;
YKVLGISSAPK; KVLGISSAPKL; VLGISSAPKLS; LGISSAPKLSG;
GISSAPKLSGR; ISSAPKLSGRF; SSAPKLSGRFL; SAPKLSGRFLR;
APKLSGRFLRH; PKLSGRFLRHY; KLSGRFLRHYD; LSGRFLRHYDS;
SGRFLRHYDSG; GRFLRHYDSGG; RFLRHYDSGGS; FLRHYDSGGST;
LRHYDSGGSTD; RHYDSGGSTDS; HYDSGGSTDSD; YDSGGSTDSDG;
DSGGSTDSDGT; SGGSTDSDGTR; GGSTDSDGTRS; GSTDSDGTRSL;
STDSDGTRSLG; TDSDGTRSLGH; DSDGTRSLGHT; SDGTRSLGHTQ;
DGTRSLGHTQE; GTRSLGHTQED; TRSLGHTQEDS; RSLGHTQEDSY;
SLGHTQEDSYK; LGHTQEDSYKN; GHTQEDSYKNH; HTQEDSYKNHD;
TQEDSYKNHDH; QEDSYKNHDHD; EDSYKNHDHDL; DSYKNHDHDLD;
SYKNHDHDLDF; YKNHDHDLDFS; KNHDHDLDFSR; NHDHDLDFSRI;
HDHDLDFSRIN; DHDLDFSRINF; HDLDFSRINFK; DLDFSRINFKG;
LDFSRINFKGK; DFSRINFKGKL; FSRINFKGKLV; SRINFKGKLVK;
RINFKGKLVKR; INFKGKLVKRM; NFKGKLVKRMT; FKGKLVKRMTM;

| | |
|---|---|
| | KGKLVKRMTMI; GKLVKRMTMIY; KLVKRMTMIYR; LVKRMTMIYRS;<br>VKRMTMIYRSA; KRMTMIYRSAD; RMTMIYRSADT; MTMIYRSADTW;<br>TMIYRSADTWY; MIYRSADTWYT; IYRSADTWYTW; YRSADTWYTWY;<br>RSADTWYTWYW; SADTWYTWYWA; ADTWYTWYWAE; DTWYTWYWAEE;<br>TWYTWYWAEEI; WYTWYWAEEIM; YTWYWAEEIMS; TWYWAEEIMSP;<br>WYWAEEIMSPF; YWAEEIMSPFI; WAEEIMSPFIT; AEEIMSPFITG;<br>EEIMSPFITGY; EIMSPFITGYS; IMSPFITGYSY; MSPFITGYSYE;<br>SPFITGYSYEE; PFITGYSYEEI; FITGYSYEEID; ITGYSYEEIDP;<br>TGYSYEEIDPE; GYSYEEIDPEY; YSYEEIDPEYL; SYEEIDPEYLL;<br>YEEIDPEYLLP; EEIDPEYLLPP; EIDPEYLLPPF; IDPEYLLPPFT;<br>DPEYLLPPFTS; PEYLLPPFTSQ; EYLLPPFTSQS; YLLPPFTSQSG;<br>LLPPFTSQSGS; LPPFTSQSGSK; PPFTSQSGSKE; PFTSQSGSKET;<br>FTSQSGSKETK; TSQSGSKETKP; SQSGSKETKPK; QSGSKETKPKN;<br>SGSKETKPKNT; GSKETKPKNTC; SKETKPKNTCY; KETKPKNTCYI;<br>ETKPKNTCYIS; TKPKNTCYISF; KPKNTCYISFY; PKNTCYISFYR;<br>KNTCYISFYRY; NTCYISFYRYD; TCYISFYRYDL; |
| Seq 30 | SEQ ID NO 36940-38333/<br>8 mers:<br>MDLLDLLE; DLLDLLEK; LLDLLEKE; LDLLEKEK; DLLEKEKQ;<br>LLEKEKQI; LEKEKQIN; EKEKQINK; KEKQINKN; EKQINKNK;<br>KQINKNKG; QINKNKGV; INKNKGVF; NKNKGVFM; KNKGVFMT;<br>NKGVFMTK; KGVFMTKP; GVFMTKPK; VFMTKPKI; FMTKPKIF;<br>MTKPKIFS; TKPKIFSI; KPKIFSIN; PKIFSINK; KIFSINKE;<br>IFSINKEK; FSINKEKI; SINKEKIK; INKEKIKI; NKEKIKIL;<br>KEKIKILI; EKIKILII; KIKILIIV; IKILIIVV; KILIIVVL;<br>ILIIVVLT; LIIVVLTS; IIVVLTST; IVVLTSTF; VVLTSTFL;<br>VLTSTFLL; LTSTFLLG; TSTFLLGI; STFLLGII; TFLLGIIF;<br>FLLGIIFS; LLGIIFSN; LGIIFSNE; GIIFSNEN; IIFSNENK;<br>IFSNENKV; FSNENKVA; SNENKVAR; NENKVARI; ENKVARIL;<br>NKVARILE; KVARILEE; VARILEEK; ARILEEKF; RILEEKFF;<br>ILEEKFFD; LEEKFFDF; EEKFFDFD; EKFFDFDF; KFFDFDFN;<br>FFDFDFNL; FDFDFNLI; DFDFNLIS; FDFNLISK; DFNLISKI;<br>FNLISKIE; NLISKIET; LISKIETE; ISKIETEL; SKIETELE;<br>KIETELEG; IETELEGT; ETELEGTL; TELEGTLT; ELEGTLTK;<br>LEGTLTKL; EGTLTKLG; GTLTKLGK; TLTKLGKD; LTKLGKDW;<br>TKLGKDWI; KLGKDWIL; LGKDWILT; GKDWILTY; KDWILTYN;<br>DWILTYNK; WILTYNKQ; ILTYNKQN; LTYNKQNI; TYNKQNIP;<br>YNKQNIPV; NKQNIPVD; KQNIPVDN; QNIPVDNK; NIPVDNKK;<br>IPVDNKKV; PVDNKKVN; VDNKKVNS; DNKKVNSL; NKKVNSLI;<br>KKVNSLIK; KVNSLIKA; VNSLIKAL; NSLIKALD; SLIKALDE;<br>LIKALDEL; IKALDELQ; KALDELQK; ALDELQKN; LDELQKNK;<br>DELQKNKL; ELQKNKLV; LQKNKLVS; QKNKLVSR; KNKLVSRD;<br>NKLVSRDQ; KLVSRDQK; LVSRDQKK; VSRDQKKH; SRDQKKHK;<br>RDQKKHKE; DQKKHKEL; QKKHKELG; KKHKELGI; KHKELGIG;<br>HKELGIGE; KELGIGEN; ELGIGENP; LGIGENPS; GIGENPSF;<br>IGENPSFK; GENPSFKL; ENPSFKLF; NPSFKLFD; PSFKLFDN;<br>SFKLFDNN; FKLFDNNN; KLFDNNNK; LFDNNNKL; FDNNNKLL;<br>DNNNKLLT; NNNKLLTE; NNKLLTEI; NKLLTEIF; KLLTEIFV;<br>LLTEIFVG; LTEIFVGK; TEIFVGKS; EIFVGKSG; IFVGKSGE;<br>FVGKSGEG; VGKSGEGD; GKSGEGDS; KSGEGDSR; SGEGDSRL; |

GEGDSRLA; EGDSRLAY; GDSRLAYI; DSRLAYIK; SRLAYIKG;
RLAYIKGS; LAYIKGSD; AYIKGSDE; YIKGSDEN; IKGSDENV;
KGSDENVY; GSDENVYL; SDENVYLT; DENVYLTK; ENVYLTKN;
NVYLTKNI; VYLTKNIF; YLTKNIFL; LTKNIFLS; TKNIFLSY;
KNIFLSYK; NIFLSYKG; IFLSYKGN; FLSYKGNS; LSYKGNSY;
SYKGNSYN; YKGNSYNT; KGNSYNTF; GNSYNTFS; NSYNTFSD;
SYNTFSDT; YNTFSDTT; NTFSDTTL; TFSDTTLF; FSDTTLFQ;
SDTTLFQE; DTTLFQEK; TTLFQEKN; TLFQEKNT; LFQEKNTK;
FQEKNTKL; QEKNTKLE; EKNTKLEN; KNTKLENL; NTKLENLS;
TKLENLSF; KLENLSFK; LENLSFKI; ENLSFKII; NLSFKIIR;
LSFKIIRK; SFKIIRKL; FKIIRKLN; KIIRKLNK; IIRKLNKE;
IRKLNKEN; RKLNKENE; KLNKENEN; LNKENENN; NKENENNI;
KENENNIN; ENENNINN; NENNINNN; ENNINNNY; NNINNNYE;
NINNNYEI; INNNYEII; NNNYEIIS; NNYEIISK; NYEIISKD;
YEIISKDG; EIISKDGL; IISKDGLY; ISKDGLYF; SKDGLYFL;
KDGLYFLN; DGLYFLNN; GLYFLNNQ; LYFLNNQK; YFLNNQKM;
FLNNQKMT; LNNQKMTK; NNQKMTKE; NQKMTKER; QKMTKERP;
KMTKERPL; MTKERPLN; TKERPLNI; KERPLNII; ERPLNIIA;
RPLNIIAE; PLNIIAEF; LNIIAEFK; NIIAEFKA; IIAEFKAD;
IAEFKADG; AEFKADGL; EFKADGLE; FKADGLEI; KADGLEID;
ADGLEIDK; DGLEIDKS; GLEIDKSK; LEIDKSKI; EIDKSKID;
IDKSKIDD; DKSKIDDY; KSKIDDYN; SKIDDYNL; KIDDYNLQ;
IDDYNLQY; DDYNLQYK; DYNLQYKI; YNLQYKIE; NLQYKIEV;
LQYKIEVK; QYKIEVKW; YKIEVKWS; KIEVKWSN; IEVKWSNK;
EVKWSNKS; VKWSNKSV; KWSNKSVN; WSNKSVNN; SNKSVNNI;
NKSVNNIE; KSVNNIEV; SVNNIEVY; VNNIEVYF; NNIEVYFN;
NIEVYFNK; IEVYFNKN; EVYFNKNE; VYFNKNEE; YFNKNEEN;
FNKNEEND; NKNEENDK; KNEENDKD; NEENDKDI; EENDKDIL;
ENDKDILI; NDKDILIK; DKDILIKK; KDILIKKD; DILIKKDK;
ILIKKDKD; LIKKDKDE; IKKDKDEY; KKDKDEYY; KDKDEYYY;
DKDEYYYT; KDEYYYTT; DEYYYTTS; EYYYTTSK; YYYTTSKW;
YYTTSKWT; YTTSKWTF; TTSKWTFF; TSKWTFFD; SKWTFFDV;
KWTFFDVF; WTFFDVFD; TFFDVFDL; FFDVFDLE; FDVFDLEK;
DVFDLEKK; VFDLEKKL; FDLEKKLT; DLEKKLTE; LEKKLTEK;
EKKLTEKD; KKLTEKDD; KLTEKDDI; LTEKDDIS; TEKDDISS;
EKDDISSN; KDDISSND; DDISSNDN; DISSNDNQ; ISSNDNQE;
SSNDNQED; SNDNQEDH; NDNQEDHH; DNQEDHHE; NQEDHHEH;
QEDHHEHH; EDHHEHHN; DHHEHHNN; HHEHHNNA; HEHHNNAD;

9 mers:
MDLLDLLEK; DLLDLLEKE; LLDLLEKEK; LDLLEKEKQ; DLLEKEKQI;
LLEKEKQIN; LEKEKQINK; EKEKQINKN; KEKQINKNK; EKQINKNKG;
KQINKNKGV; QINKNKGVF; INKNKGVFM; NKNKGVFMT; KNKGVFMTK;
NKGVFMTKP; KGVFMTKPK; GVFMTKPKI; VFMTKPKIF; FMTKPKIFS;
MTKPKIFSI; TKPKIFSIN; KPKIFSINK; PKIFSINKE; KIFSINKEK;
IFSINKEKI; FSINKEKIK; SINKEKIKI; INKEKIKIL; NKEKIKILI;
KEKIKILII; EKIKILIIV; KIKILIIVV; IKILIIVVL; KILIIVVLT;
ILIIVVLTS; LIIVVLTST; IIVVLTSTF; IVVLTSTFL; VVLTSTFLL;
VLTSTFLLG; LTSTFLLGI; TSTFLLGII; STFLLGIIF; TFLLGIIFS;
FLLGIIFSN; LLGIIFSNE; LGIIFSNEN; GIIFSNENK; IIFSNENKV;
IFSNENKVA; FSNENKVAR; SNENKVARI; NENKVARIL; ENKVARILE;

747

NKVARILEE; KVARILEEK; VARILEEKF; ARILEEKFF; RILEEKFFD;
ILEEKFFDF; LEEKFFDFD; EEKFFDFDF; EKFFDFDFN; KFFDFDFNL;
FFDFDFNLI; FDFDFNLIS; DFDFNLISK; FDFNLISKI; DFNLISKIE;
FNLISKIET; NLISKIETE; LISKIETEL; ISKIETELE; SKIETELEG;
KIETELEGT; IETELEGTL; ETELEGTLT; TELEGTLTK; ELEGTLTKL;
LEGTLTKLG; EGTLTKLGK; GTLTKLGKD; TLTKLGKDW; LTKLGKDWI;
TKLGKDWIL; KLGKDWILT; LGKDWILTY; GKDWILTYN; KDWILTYNK;
DWILTYNKQ; WILTYNKQN; ILTYNKQNI; LTYNKQNIP; TYNKQNIPV;
YNKQNIPVD; NKQNIPVDN; KQNIPVDNK; QNIPVDNKK; NIPVDNKKV;
IPVDNKKVN; PVDNKKVNS; VDNKKVNSL; DNKKVNSLI; NKKVNSLIK;
KKVNSLIKA; KVNSLIKAL; VNSLIKALD; NSLIKALDE; SLIKALDEL;
LIKALDELQ; IKALDELQK; KALDELQKN; ALDELQKNK; LDELQKNKL;
DELQKNKLV; ELQKNKLVS; LQKNKLVSR; QKNKLVSRD; KNKLVSRDQ;
NKLVSRDQK; KLVSRDQKK; LVSRDQKKH; VSRDQKKHK; SRDQKKHKE;
RDQKKHKEL; DQKKHKELG; QKKHKELGI; KKHKELGIG; KHKELGIGE;
HKELGIGEN; KELGIGENP; ELGIGENPS; LGIGENPSF; GIGENPSFK;
IGENPSFKL; GENPSFKLF; ENPSFKLFD; NPSFKLFDN; PSFKLFDNN;
SFKLFDNNN; FKLFDNNNK; KLFDNNNKL; LFDNNNKLL; FDNNNKLLT;
DNNNKLLTE; NNNKLLTEI; NNKLLTEIF; NKLLTEIFV; KLLTEIFVG;
LLTEIFVGK; LTEIFVGKS; TEIFVGKSG; EIFVGKSGE; IFVGKSGEG;
FVGKSGEGD; VGKSGEGDS; GKSGEGDSR; KSGEGDSRL; SGEGDSRLA;
GEGDSRLAY; EGDSRLAYI; GDSRLAYIK; DSRLAYIKG; SRLAYIKGS;
RLAYIKGSD; LAYIKGSDE; AYIKGSDEN; YIKGSDENV; IKGSDENVY;
KGSDENVYL; GSDENVYLT; SDENVYLTK; DENVYLTKN; ENVYLTKNI;
NVYLTKNIF; VYLTKNIFL; YLTKNIFLS; LTKNIFLSY; TKNIFLSYK;
KNIFLSYKG; NIFLSYKGN; IFLSYKGNS; FLSYKGNSY; LSYKGNSYN;
SYKGNSYNT; YKGNSYNTF; KGNSYNTFS; GNSYNTFSD; NSYNTFSDT;
SYNTFSDTT; YNTFSDTTL; NTFSDTTLF; TFSDTTLFQ; FSDTTLFQE;
SDTTLFQEK; DTTLFQEKN; TTLFQEKNT; TLFQEKNTK; LFQEKNTKL;
FQEKNTKLE; QEKNTKLEN; EKNTKLENL; KNTKLENLS; NTKLENLSF;
TKLENLSFK; KLENLSFKI; LENLSFKII; ENLSFKIIR; NLSFKIIRK;
LSFKIIRKL; SFKIIRKLN; FKIIRKLNK; KIIRKLNKE; IIRKLNKEN;
IRKLNKENE; RKLNKENEN; KLNKENENN; LNKENENNI; NKENENNIN;
KENENNINN; ENENNINNN; NENNINNNY; ENNINNNYE; NNINNNYEI;
NINNNYEII; INNNYEIIS; NNNYEIISK; NNYEIISKD; NYEIISKDG;
YEIISKDGL; EIISKDGLY; IISKDGLYF; ISKDGLYFL; SKDGLYFLN;
KDGLYFLNN; DGLYFLNNQ; GLYFLNNQK; LYFLNNQKM; YFLNNQKMT;
FLNNQKMTK; LNNQKMTKE; NNQKMTKER; NQKMTKERP; QKMTKERPL;
KMTKERPLN; MTKERPLNI; TKERPLNII; KERPLNIIA; ERPLNIIAE;
RPLNIIAEF; PLNIIAEFK; LNIIAEFKA; NIIAEFKAD; IIAEFKADG;
IAEFKADGL; AEFKADGLE; EFKADGLEI; FKADGLEID; KADGLEIDK;
ADGLEIDKS; DGLEIDKSK; GLEIDKSKI; LEIDKSKID; EIDKSKIDD;
IDKSKIDDY; DKSKIDDYN; KSKIDDYNL; SKIDDYNLQ; KIDDYNLQY;
IDDYNLQYK; DDYNLQYKI; DYNLQYKIE; YNLQYKIEV; NLQYKIEVK;
LQYKIEVKW; QYKIEVKWS; YKIEVKWSN; KIEVKWSNK; IEVKWSNKS;
EVKWSNKSV; VKWSNKSVN; KWSNKSVNN; WSNKSVNNI; SNKSVNNIE;
NKSVNNIEV; KSVNNIEVY; SVNNIEVYF; VNNIEVYFN; NNIEVYFNK;
NIEVYFNKN; IEVYFNKNE; EVYFNKNEE; VYFNKNEEN; YFNKNEEND;
FNKNEENDK; NKNEENDKD; KNEENDKDI; NEENDKDIL; EENDKDILI;
ENDKDILIK; NDKDILIKK; DKDILIKKD; KDILIKKDK; DILIKKDKD;
ILIKKDKDE; LIKKDKDEY; IKKDKDEYY; KKDKDEYYY; KDKDEYYYT;

DKDEYYYTT; KDEYYYTTS; DEYYYTTSK; EYYYTTSKW; YYYTTSKWT;
YYTTSKWTF; YTTSKWTFF; TTSKWTFFD; TSKWTFFDV; SKWTFFDVF;
KWTFFDVFD; WTFFDVFDL; TFFDVFDLE; FFDVFDLEK; FDVFDLEKK;
DVFDLEKKL; VFDLEKKLT; FDLEKKLTE; DLEKKLTEK; LEKKLTEKD;
EKKLTEKDD; KKLTEKDDI; KLTEKDDIS; LTEKDDISS; TEKDDISSN;
EKDDISSND; KDDISSNDN; DDISSNDNQ; DISSNDNQE; ISSNDNQED;
SSNDNQEDH; SNDNQEDHH; NDNQEDHHE; DNQEDHHEH; NQEDHHEHH;
QEDHHEHHN; EDHHEHHNN; DHHEHHNNA; HHEHHNNAD;


10 mers:
MDLLDLLEKE; DLLDLLEKEK; LLDLLEKEKQ; LDLLEKEKQI;
DLLEKEKQIN; LLEKEKQINK; LEKEKQINKN; EKEKQINKNK;
KEKQINKNKG; EKQINKNKGV; KQINKNKGVF; QINKNKGVFM;
INKNKGVFMT; NKNKGVFMTK; KNKGVFMTKP; NKGVFMTKPK;
KGVFMTKPKI; GVFMTKPKIF; VFMTKPKIFS; FMTKPKIFSI;
MTKPKIFSIN; TKPKIFSINK; KPKIFSINKE; PKIFSINKEK;
KIFSINKEKI; IFSINKEKIK; FSINKEKIKI; SINKEKIKIL;
INKEKIKILI; NKEKIKILII; KEKIKILIIV; EKIKILIIVV;
KIKILIIVVL; IKILIIVVLT; KILIIVVLTS; ILIIVVLTST;
LIIVVLTSTF; IIVVLTSTFL; IVVLTSTFLL; VVLTSTFLLG;
VLTSTFLLGI; LTSTFLLGII; TSTFLLGIIF; STFLLGIIFS;
TFLLGIIFSN; FLLGIIFSNE; LLGIIFSNEN; LGIIFSNENK;
GIIFSNENKV; IIFSNENKVA; IFSNENKVAR; FSNENKVARI;
SNENKVARIL; NENKVARILE; ENKVARILEE; NKVARILEEK;
KVARILEEKF; VARILEEKFF; ARILEEKFFD; RILEEKFFDF;
ILEEKFFDFD; LEEKFFDFDF; EEKFFDFDFN; EKFFDFDFNL;
KFFDFDFNLI; FFDFDFNLIS; FDFDFNLISK; DFDFNLISKI;
FDFNLISKIE; DFNLISKIET; FNLISKIETE; NLISKIETEL;
LISKIETELE; ISKIETELEG; SKIETELEGT; KIETELEGTL;
IETELEGTLT; ETELEGTLTK; TELEGTLTKL; ELEGTLTKLG;
LEGTLTKLGK; EGTLTKLGKD; GTLTKLGKDW; TLTKLGKDWI;
LTKLGKDWIL; TKLGKDWILT; KLGKDWILTY; LGKDWILTYN;
GKDWILTYNK; KDWILTYNKQ; DWILTYNKQN; WILTYNKQNI;
ILTYNKQNIP; LTYNKQNIPV; TYNKQNIPVD; YNKQNIPVDN;
NKQNIPVDNK; KQNIPVDNKK; QNIPVDNKKV; NIPVDNKKVN;
IPVDNKKVNS; PVDNKKVNSL; VDNKKVNSLI; DNKKVNSLIK;
NKKVNSLIKA; KKVNSLIKAL; KVNSLIKALD; VNSLIKALDE;
NSLIKALDEL; SLIKALDELQ; LIKALDELQK; IKALDELQKN;
KALDELQKNK; ALDELQKNKL; LDELQKNKLV; DELQKNKLVS;
ELQKNKLVSR; LQKNKLVSRD; QKNKLVSRDQ; KNKLVSRDQK;
NKLVSRDQKK; KLVSRDQKKH; LVSRDQKKHK; VSRDQKKHKE;
SRDQKKHKEL; RDQKKHKELG; DQKKHKELGI; QKKHKELGIG;
KKHKELGIGE; KHKELGIGEN; HKELGIGENP; KELGIGENPS;
ELGIGENPSF; LGIGENPSFK; GIGENPSFKL; IGENPSFKLF;
GENPSFKLFD; ENPSFKLFDN; NPSFKLFDNN; PSFKLFDNNN;
SFKLFDNNNK; FKLFDNNNKL; KLFDNNNKLL; LFDNNNKLLT;
FDNNNKLLTE; DNNNKLLTEI; NNNKLLTEIF; NNKLLTEIFV;
NKLLTEIFVG; KLLTEIFVGK; LLTEIFVGKS; LTEIFVGKSG;
TEIFVGKSGE; EIFVGKSGEG; IFVGKSGEGD; FVGKSGEGDS;
VGKSGEGDSR; GKSGEGDSRL; KSGEGDSRLA; SGEGDSRLAY;

GEGDSRLAYI; EGDSRLAYIK; GDSRLAYIKG; DSRLAYIKGS;
SRLAYIKGSD; RLAYIKGSDE; LAYIKGSDEN; AYIKGSDENV;
YIKGSDENVY; IKGSDENVYL; KGSDENVYLT; GSDENVYLTK;
SDENVYLTKN; DENVYLTKNI; ENVYLTKNIF; NVYLTKNIFL;
VYLTKNIFLS; YLTKNIFLSY; LTKNIFLSYK; TKNIFLSYKG;
KNIFLSYKGN; NIFLSYKGNS; IFLSYKGNSY; FLSYKGNSYN;
LSYKGNSYNT; SYKGNSYNTF; YKGNSYNTFS; KGNSYNTFSD;
GNSYNTFSDT; NSYNTFSDTT; SYNTFSDTTL; YNTFSDTTLF;
NTFSDTTLFQ; TFSDTTLFQE; FSDTTLFQEK; SDTTLFQEKN;
DTTLFQEKNT; TTLFQEKNTK; TLFQEKNTKL; LFQEKNTKLE;
FQEKNTKLEN; QEKNTKLENL; EKNTKLENLS; KNTKLENLSF;
NTKLENLSFK; TKLENLSFKI; KLENLSFKII; LENLSFKIIR;
ENLSFKIIRK; NLSFKIIRKL; LSFKIIRKLN; SFKIIRKLNK;
FKIIRKLNKE; KIIRKLNKEN; IIRKLNKENE; IRKLNKENEN;
RKLNKENENN; KLNKENENNI; LNKENENNIN; NKENENNINN;
KENENNINNN; ENENNINNNY; NENNINNNYE; ENNINNNYEI;
NNINNNYEII; NINNNYEIIS; INNNYEIISK; NNNYEIISKD;
NNYEIISKDG; NYEIISKDGL; YEIISKDGLY; EIISKDGLYF;
IISKDGLYFL; ISKDGLYFLN; SKDGLYFLNN; KDGLYFLNNQ;
DGLYFLNNQK; GLYFLNNQKM; LYFLNNQKMT; YFLNNQKMTK;
FLNNQKMTKE; LNNQKMTKER; NNQKMTKERP; NQKMTKERPL;
QKMTKERPLN; KMTKERPLNI; MTKERPLNII; TKERPLNIIA;
KERPLNIIAE; ERPLNIIAEF; RPLNIIAEFK; PLNIIAEFKA;
LNIIAEFKAD; NIIAEFKADG; IIAEFKADGL; IAEFKADGLE;
AEFKADGLEI; EFKADGLEID; FKADGLEIDK; KADGLEIDKS;
ADGLEIDKSK; DGLEIDKSKI; GLEIDKSKID; LEIDKSKIDD;
EIDKSKIDDY; IDKSKIDDYN; DKSKIDDYNL; KSKIDDYNLQ;
SKIDDYNLQY; KIDDYNLQYK; IDDYNLQYKI; DDYNLQYKIE;
DYNLQYKIEV; YNLQYKIEVK; NLQYKIEVKW; LQYKIEVKWS;
QYKIEVKWSN; YKIEVKWSNK; KIEVKWSNKS; IEVKWSNKSV;
EVKWSNKSVN; VKWSNKSVNN; KWSNKSVNNI; WSNKSVNNIE;
SNKSVNNIEV; NKSVNNIEVY; KSVNNIEVYF; SVNNIEVYFN;
VNNIEVYFNK; NNIEVYFNKN; NIEVYFNKNE; IEVYFNKNEE;
EVYFNKNEEN; VYFNKNEEND; YFNKNEENDK; FNKNEENDKD;
NKNEENDKDI; KNEENDKDIL; NEENDKDILI; EENDKDILIK;
ENDKDILIKK; NDKDILIKKD; DKDILIKKDK; KDILIKKDKD;
DILIKKDKDE; ILIKKDKDEY; LIKKDKDEYY; IKKDKDEYYY;
KKDKDEYYYT; KDKDEYYYTT; DKDEYYYTTS; KDEYYYTTSK;
DEYYYTTSKW; EYYYTTSKWT; YYYTTSKWTF; YYTTSKWTFF;
YTTSKWTFFD; TTSKWTFFDV; TSKWTFFDVF; SKWTFFDVFD;
KWTFFDVFDL; WTFFDVFDLE; TFFDVFDLEK; FFDVFDLEKK;
FDVFDLEKKL; DVFDLEKKLT; VFDLEKKLTE; FDLEKKLTEK;
DLEKKLTEKD; LEKKLTEKDD; EKKLTEKDDI; KKLTEKDDIS;
KLTEKDDISS; LTEKDDISSN; TEKDDISSND; EKDDISSNDN;
KDDISSNDNQ; DDISSNDNQE; DISSNDNQED; ISSNDNQEDH;
SSNDNQEDHH; SNDNQEDHHE; NDNQEDHHEH; DNQEDHHEHH;
NQEDHHEHHN; QEDHHEHHNN; EDHHEHHNNA; DHHEHHNNAD;

11 mers:
MDLLDLLEKEK; DLLDLLEKEKQ; LLDLLEKEKQI; LDLLEKEKQIN;
DLLEKEKQINK; LLEKEKQINKN; LEKEKQINKNK; EKEKQINKNKG;

KEKQINKNKGV; EKQINKNKGVF; KQINKNKGVFM; QINKNKGVFMT;
INKNKGVFMTK; NKNKGVFMTKP; KNKGVFMTKPK; NKGVFMTKPKI;
KGVFMTKPKIF; GVFMTKPKIFS; VFMTKPKIFSI; FMTKPKIFSIN;
MTKPKIFSINK; TKPKIFSINKE; KPKIFSINKEK; PKIFSINKEKI;
KIFSINKEKIK; IFSINKEKIKI; FSINKEKIKIL; SINKEKIKILI;
INKEKIKILII; NKEKIKILIIV; KEKIKILIIVV; EKIKILIIVVL;
KIKILIIVVLT; IKILIIVVLTS; KILIIVVLTST; ILIIVVLTSTF;
LIIVVLTSTFL; IIVVLTSTFLL; IVVLTSTFLLG; VVLTSTFLLGI;
VLTSTFLLGII; LTSTFLLGIIF; TSTFLLGIIFS; STFLLGIIFSN;
TFLLGIIFSNE; FLLGIIFSNEN; LLGIIFSNENK; LGIIFSNENKV;
GIIFSNENKVA; IIFSNENKVAR; IFSNENKVARI; FSNENKVARIL;
SNENKVARILE; NENKVARILEE; ENKVARILEEK; NKVARILEEKF;
KVARILEEKFF; VARILEEKFFD; ARILEEKFFDF; RILEEKFFDFD;
ILEEKFFDFDF; LEEKFFDFDFN; EEKFFDFDFNL; EKFFDFDFNLI;
KFFDFDFNLIS; FFDFDFNLISK; FDFDFNLISKI; DFDFNLISKIE;
FDFNLISKIET; DFNLISKIETE; FNLISKIETEL; NLISKIETELE;
LISKIETELEG; ISKIETELEGT; SKIETELEGTL; KIETELEGTLT;
IETELEGTLTK; ETELEGTLTKL; TELEGTLTKLG; ELEGTLTKLGK;
LEGTLTKLGKD; EGTLTKLGKDW; GTLTKLGKDWI; TLTKLGKDWIL;
LTKLGKDWILT; TKLGKDWILTY; KLGKDWILTYN; LGKDWILTYNK;
GKDWILTYNKQ; KDWILTYNKQN; DWILTYNKQNI; WILTYNKQNIP;
ILTYNKQNIPV; LTYNKQNIPVD; TYNKQNIPVDN; YNKQNIPVDNK;
NKQNIPVDNKK; KQNIPVDNKKV; QNIPVDNKKVN; NIPVDNKKVNS;
IPVDNKKVNSL; PVDNKKVNSLI; VDNKKVNSLIK; DNKKVNSLIKA;
NKKVNSLIKAL; KKVNSLIKALD; KVNSLIKALDE; VNSLIKALDEL;
NSLIKALDELQ; SLIKALDELQK; LIKALDELQKN; IKALDELQKNK;
KALDELQKNKL; ALDELQKNKLV; LDELQKNKLVS; DELQKNKLVSR;
ELQKNKLVSRD; LQKNKLVSRDQ; QKNKLVSRDQK; KNKLVSRDQKK;
NKLVSRDQKKH; KLVSRDQKKHK; LVSRDQKKHKE; VSRDQKKHKEL;
SRDQKKHKELG; RDQKKHKELGI; DQKKHKELGIG; QKKHKELGIGE;
KKHKELGIGEN; KHKELGIGENP; HKELGIGENPS; KELGIGENPSF;
ELGIGENPSFK; LGIGENPSFKL; GIGENPSFKLF; IGENPSFKLFD;
GENPSFKLFDN; ENPSFKLFDNN; NPSFKLFDNNN; PSFKLFDNNNK;
SFKLFDNNNKL; FKLFDNNNKLL; KLFDNNNKLLT; LFDNNNKLLTE;
FDNNNKLLTEI; DNNNKLLTEIF; NNNKLLTEIFV; NNKLLTEIFVG;
NKLLTEIFVGK; KLLTEIFVGKS; LLTEIFVGKSG; LTEIFVGKSGE;
TEIFVGKSGEG; EIFVGKSGEGD; IFVGKSGEGDS; FVGKSGEGDSR;
VGKSGEGDSRL; GKSGEGDSRLA; KSGEGDSRLAY; SGEGDSRLAYI;
GEGDSRLAYIK; EGDSRLAYIKG; GDSRLAYIKGS; DSRLAYIKGSD;
SRLAYIKGSDE; RLAYIKGSDEN; LAYIKGSDENV; AYIKGSDENVY;
YIKGSDENVYL; IKGSDENVYLT; KGSDENVYLTK; GSDENVYLTKN;
SDENVYLTKNI; DENVYLTKNIF; ENVYLTKNIFL; NVYLTKNIFLS;
VYLTKNIFLSY; YLTKNIFLSYK; LTKNIFLSYKG; TKNIFLSYKGN;
KNIFLSYKGNS; NIFLSYKGNSY; IFLSYKGNSYN; FLSYKGNSYNT;
LSYKGNSYNTF; SYKGNSYNTFS; YKGNSYNTFSD; KGNSYNTFSDT;
GNSYNTFSDTT; NSYNTFSDTTL; SYNTFSDTTLF; YNTFSDTTLFQ;
NTFSDTTLFQE; TFSDTTLFQEK; FSDTTLFQEKN; SDTTLFQEKNT;
DTTLFQEKNTK; TTLFQEKNTKL; TLFQEKNTKLE; LFQEKNTKLEN;
FQEKNTKLENL; QEKNTKLENLS; EKNTKLENLSF; KNTKLENLSFK;
NTKLENLSFKI; TKLENLSFKII; KLENLSFKIIR; LENLSFKIIRK;
ENLSFKIIRKL; NLSFKIIRKLN; LSFKIIRKLNK; SFKIIRKLNKE;

| | |
|---|---|
| | FKIIRKLNKEN; KIIRKLNKENE; IIRKLNKENEN; IRKLNKENENN;<br>RKLNKENENNI; KLNKENENNIN; LNKENENNINN; NKENENNINNN;<br>KENENNINNNY; ENENNINNNYE; NENNINNNYEI; ENNINNNYEII;<br>NNINNNYEIIS; NINNNYEIISK; INNNYEIISKD; NNNYEIISKDG;<br>NNYEIISKDGL; NYEIISKDGLY; YEIISKDGLYF; EIISKDGLYFL;<br>IISKDGLYFLN; ISKDGLYFLNN; SKDGLYFLNNQ; KDGLYFLNNQK;<br>DGLYFLNNQKM; GLYFLNNQKMT; LYFLNNQKMTK; YFLNNQKMTKE;<br>FLNNQKMTKER; LNNQKMTKERP; NNQKMTKERPL; NQKMTKERPLN;<br>QKMTKERPLNI; KMTKERPLNII; MTKERPLNIIA; TKERPLNIIAE;<br>KERPLNIIAEF; ERPLNIIAEFK; RPLNIIAEFKA; PLNIIAEFKAD;<br>LNIIAEFKADG; NIIAEFKADGL; IIAEFKADGLE; IAEFKADGLEI;<br>AEFKADGLEID; EFKADGLEIDK; FKADGLEIDKS; KADGLEIDKSK;<br>ADGLEIDKSKI; DGLEIDKSKID; GLEIDKSKIDD; LEIDKSKIDDY;<br>EIDKSKIDDYN; IDKSKIDDYNL; DKSKIDDYNLQ; KSKIDDYNLQY;<br>SKIDDYNLQYK; KIDDYNLQYKI; IDDYNLQYKIE; DDYNLQYKIEV;<br>DYNLQYKIEVK; YNLQYKIEVKW; NLQYKIEVKWS; LQYKIEVKWSN;<br>QYKIEVKWSNK; YKIEVKWSNKS; KIEVKWSNKSV; IEVKWSNKSVN;<br>EVKWSNKSVNN; VKWSNKSVNNI; KWSNKSVNNIE; WSNKSVNNIEV;<br>SNKSVNNIEVY; NKSVNNIEVYF; KSVNNIEVYFN; SVNNIEVYFNK;<br>VNNIEVYFNKN; NNIEVYFNKNE; NIEVYFNKNEE; IEVYFNKNEEN;<br>EVYFNKNEEND; VYFNKNEENDK; YFNKNEENDKD; FNKNEENDKDI;<br>NKNEENDKDIL; KNEENDKDILI; NEENDKDILIK; EENDKDILIKK;<br>ENDKDILIKKD; NDKDILIKKDK; DKDILIKKDKD; KDILIKKDKDE;<br>DILIKKDKDEY; ILIKKDKDEYY; LIKKDKDEYYY; IKKDKDEYYYT;<br>KKDKDEYYYTT; KDKDEYYYTTS; DKDEYYYTTSK; KDEYYYTTSKW;<br>DEYYYTTSKWT; EYYYTTSKWTF; YYYTTSKWTFF; YYTTSKWTFFD;<br>YTTSKWTFFDV; TTSKWTFFDVF; TSKWTFFDVFD; SKWTFFDVFDL;<br>KWTFFDVFDLE; WTFFDVFDLEK; TFFDVFDLEKK; FFDVFDLEKKL;<br>FDVFDLEKKLT; DVFDLEKKLTE; VFDLEKKLTEK; FDLEKKLTEKD;<br>DLEKKLTEKDD; LEKKLTEKDDI; EKKLTEKDDIS; KKLTEKDDISS;<br>KLTEKDDISSN; LTEKDDISSND; TEKDDISSNDN; EKDDISSNDNQ;<br>KDDISSNDNQE; DDISSNDNQED; DISSNDNQEDH; ISSNDNQEDHH;<br>SSNDNQEDHHE; SNDNQEDHHEH; NDNQEDHHEHH; DNQEDHHEHHN;<br>NQEDHHEHHNN; QEDHHEHHNNA; EDHHEHHNNAD; |
| Seq 31 | SEQ ID NO 38334-38919/<br>8 mers:<br>MKKLIIIF; KKLIIIFT; KLIIIFTL; LIIIFTLF; IIIFTLFL;<br>IIFTLFLS; IFTLFLSQ; FTLFLSQA; TLFLSQAC; LFLSQACN;<br>FLSQACNL; LSQACNLS; SQACNLST; QACNLSTM; ACNLSTMH;<br>CNLSTMHK; NLSTMHKI; LSTMHKID; STMHKIDT; TMHKIDTK;<br>MHKIDTKE; HKIDTKED; KIDTKEDM; IDTKEDMK; DTKEDMKI;<br>TKEDMKIL; KEDMKILY; EDMKILYS; DMKILYSE; MKILYSEI;<br>KILYSEIA; ILYSEIAE; LYSEIAEL; YSEIAELR; SEIAELRK;<br>EIAELRKK; IAELRKKL; AELRKKLN; ELRKKLNL; LRKKLNLN;<br>RKKLNLNH; KKLNLNHL; KLNLNHLE; LNLNHLEI; NLNHLEID;<br>LNHLEIDD; NHLEIDDT; HLEIDDTL; LEIDDTLE; EIDDTLEK;<br>IDDTLEKV; DDTLEKVA; DTLEKVAK; TLEKVAKE; LEKVAKEY;<br>EKVAKEYA; KVAKEYAI; VAKEYAIK; AKEYAIKL; KEYAIKLG;<br>EYAIKLGE; YAIKLGEN; AIKLGENR; IKLGENRT; KLGENRTI;<br>LGENRTIT; GENRTITH; ENRTITHT; NRTITHTL; RTITHTLF; |

TITHTLFG; ITHTLFGT; THTLFGTT; HTLFGTTP; TLFGTTPM;
LFGTTPMQ; FGTTPMQR; GTTPMQRI; TTPMQRIH; TPMQRIHK;
PMQRIHKY; MQRIHKYD; QRIHKYDQ; RIHKYDQS; IHKYDQSF;
HKYDQSFN; KYDQSFNL; YDQSFNLT; DQSFNLTR; QSFNLTRE;
SFNLTREI; FNLTREIL; NLTREILA; LTREILAS; TREILASG;
REILASGI; EILASGIE; ILASGIEL; LASGIELN; ASGIELNR;
SGIELNRV; GIELNRVV; IELNRVVN; ELNRVVNA; LNRVVNAW;
NRVVNAWL; RVVNAWLN; VVNAWLNS; VNAWLNSP; NAWLNSPS;
AWLNSPSH; WLNSPSHK; LNSPSHKE; NSPSHKEA; SPSHKEAL;
PSHKEALI; SHKEALIN; HKEALINT; KEALINTD; EALINTDT;
ALINTDTD; LINTDTDK; INTDTDKI; NTDTDKIG; TDTDKIGG;
DTDKIGGY; TDKIGGYR; DKIGGYRL; KIGGYRLK; IGGYRLKT;
GGYRLKTT; GYRLKTTD; YRLKTTDN; RLKTTDNI; LKTTDNID;
KTTDNIDI; TTDNIDIF; TDNIDIFV; DNIDIFVV; NIDIFVVL;
IDIFVVLF; DIFVVLFG; IFVVLFGK; FVVLFGKR; VVLFGKRK;
VLFGKRKY; LFGKRKYK; FGKRKYKN;

9 mers:
MKKLIIIFT; KKLIIIFTL; KLIIIFTLF; LIIIFTLFL; IIIFTLFLS;
IIFTLFLSQ; IFTLFLSQA; FTLFLSQAC; TLFLSQACN; LFLSQACNL;
FLSQACNLS; LSQACNLST; SQACNLSTM; QACNLSTMH; ACNLSTMHK;
CNLSTMHKI; NLSTMHKID; LSTMHKIDT; STMHKIDTK; TMHKIDTKE;
MHKIDTKED; HKIDTKEDM; KIDTKEDMK; IDTKEDMKI; DTKEDMKIL;
TKEDMKILY; KEDMKILYS; EDMKILYSE; DMKILYSEI; MKILYSEIA;
KILYSEIAE; ILYSEIAEL; LYSEIAELR; YSEIAELRK; SEIAELRKK;
EIAELRKKL; IAELRKKLN; AELRKKLNL; ELRKKLNLN; LRKKLNLNH;
RKKLNLNHL; KKLNLNHLE; KLNLNHLEI; LNLNHLEID; NLNHLEIDD;
LNHLEIDDT; NHLEIDDTL; HLEIDDTLE; LEIDDTLEK; EIDDTLEKV;
IDDTLEKVA; DDTLEKVAK; DTLEKVAKE; TLEKVAKEY; LEKVAKEYA;
EKVAKEYAI; KVAKEYAIK; VAKEYAIKL; AKEYAIKLG; KEYAIKLGE;
EYAIKLGEN; YAIKLGENR; AIKLGENRT; IKLGENRTI; KLGENRTIT;
LGENRTITH; GENRTITHT; ENRTITHTL; NRTITHTLF; RTITHTLFG;
TITHTLFGT; ITHTLFGTT; THTLFGTTP; HTLFGTTPM; TLFGTTPMQ;
LFGTTPMQR; FGTTPMQRI; GTTPMQRIH; TTPMQRIHK; TPMQRIHKY;
PMQRIHKYD; MQRIHKYDQ; QRIHKYDQS; RIHKYDQSF; IHKYDQSFN;
HKYDQSFNL; KYDQSFNLT; YDQSFNLTR; DQSFNLTRE; QSFNLTREI;
SFNLTREIL; FNLTREILA; NLTREILAS; LTREILASG; TREILASGI;
REILASGIE; EILASGIEL; ILASGIELN; LASGIELNR; ASGIELNRV;
SGIELNRVV; GIELNRVVN; IELNRVVNA; ELNRVVNAW; LNRVVNAWL;
NRVVNAWLN; RVVNAWLNS; VVNAWLNSP; VNAWLNSPS; NAWLNSPSH;
AWLNSPSHK; WLNSPSHKE; LNSPSHKEA; NSPSHKEAL; SPSHKEALI;
PSHKEALIN; SHKEALINT; HKEALINTD; KEALINTDT; EALINTDTD;
ALINTDTDK; LINTDTDKI; INTDTDKIG; NTDTDKIGG; TDTDKIGGY;
DTDKIGGYR; TDKIGGYRL; DKIGGYRLK; KIGGYRLKT; IGGYRLKTT;
GGYRLKTTD; GYRLKTTDN; YRLKTTDNI; RLKTTDNID; LKTTDNIDI;
KTTDNIDIF; TTDNIDIFV; TDNIDIFVV; DNIDIFVVL; NIDIFVVLF;
IDIFVVLFG; DIFVVLFGK; IFVVLFGKR; FVVLFGKRK; VVLFGKRKY;
VLFGKRKYK; LFGKRKYKN;

10 mers:
MKKLIIIFTL; KKLIIIFTLF; KLIIIFTLFL; LIIIFTLFLS;

IIIFTLFLSQ; IIFTLFLSQA; IFTLFLSQAC; FTLFLSQACN;
TLFLSQACNL; LFLSQACNLS; FLSQACNLST; LSQACNLSTM;
SQACNLSTMH; QACNLSTMHK; ACNLSTMHKI; CNLSTMHKID;
NLSTMHKIDT; LSTMHKIDTK; STMHKIDTKE; TMHKIDTKED;
MHKIDTKEDM; HKIDTKEDMK; KIDTKEDMKI; IDTKEDMKIL;
DTKEDMKILY; TKEDMKILYS; KEDMKILYSE; EDMKILYSEI;
DMKILYSEIA; MKILYSEIAE; KILYSEIAEL; ILYSEIAELR;
LYSEIAELRK; YSEIAELRKK; SEIAELRKKL; EIAELRKKLN;
IAELRKKLNL; AELRKKLNLN; ELRKKLNLNH; LRKKLNLNHL;
RKKLNLNHLE; KKLNLNHLEI; KLNLNHLEID; LNLNHLEIDD;
NLNHLEIDDT; LNHLEIDDTL; NHLEIDDTLE; HLEIDDTLEK;
LEIDDTLEKV; EIDDTLEKVA; IDDTLEKVAK; DDTLEKVAKE;
DTLEKVAKEY; TLEKVAKEYA; LEKVAKEYAI; EKVAKEYAIK;
KVAKEYAIKL; VAKEYAIKLG; AKEYAIKLGE; KEYAIKLGEN;
EYAIKLGENR; YAIKLGENRT; AIKLGENRTI; IKLGENRTIT;
KLGENRTITH; LGENRTITHT; GENRTITHTL; ENRTITHTLF;
NRTITHTLFG; RTITHTLFGT; TITHTLFGTT; ITHTLFGTTP;
THTLFGTTPM; HTLFGTTPMQ; TLFGTTPMQR; LFGTTPMQRI;
FGTTPMQRIH; GTTPMQRIHK; TTPMQRIHKY; TPMQRIHKYD;
PMQRIHKYDQ; MQRIHKYDQS; QRIHKYDQSF; RIHKYDQSFN;
IHKYDQSFNL; HKYDQSFNLT; KYDQSFNLTR; YDQSFNLTRE;
DQSFNLTREI; QSFNLTREIL; SFNLTREILA; FNLTREILAS;
NLTREILASG; LTREILASGI; TREILASGIE; REILASGIEL;
EILASGIELN; ILASGIELNR; LASGIELNRV; ASGIELNRVV;
SGIELNRVVN; GIELNRVVNA; IELNRVVNAW; ELNRVVNAWL;
LNRVVNAWLN; NRVVNAWLNS; RVVNAWLNSP; VVNAWLNSPS;
VNAWLNSPSH; NAWLNSPSHK; AWLNSPSHKE; WLNSPSHKEA;
LNSPSHKEAL; NSPSHKEALI; SPSHKEALIN; PSHKEALINT;
SHKEALINTD; HKEALINTDT; KEALINTDTD; EALINTDTDK;
ALINTDTDKI; LINTDTDKIG; INTDTDKIGG; NTDTDKIGGY;
TDTDKIGGYR; DTDKIGGYRL; TDKIGGYRLK; DKIGGYRLKT;
KIGGYRLKTT; IGGYRLKTTD; GGYRLKTTDN; GYRLKTTDNI;
YRLKTTDNID; RLKTTDNIDI; LKTTDNIDIF; KTTDNIDIFV;
TTDNIDIFVV; TDNIDIFVVL; DNIDIFVVLF; NIDIFVVLFG;
IDIFVVLFGK; DIFVVLFGKR; IFVVLFGKRK; FVVLFGKRKY;
VVLFGKRKYK; VLFGKRKYKN;

11 mers:
MKKLIIIFTLF; KKLIIIFTLFL; KLIIIFTLFLS; LIIIFTLFLSQ;
IIIFTLFLSQA; IIFTLFLSQAC; IFTLFLSQACN; FTLFLSQACNL;
TLFLSQACNLS; LFLSQACNLST; FLSQACNLSTM; LSQACNLSTMH;
SQACNLSTMHK; QACNLSTMHKI; ACNLSTMHKID; CNLSTMHKIDT;
NLSTMHKIDTK; LSTMHKIDTKE; STMHKIDTKED; TMHKIDTKEDM;
MHKIDTKEDMK; HKIDTKEDMKI; KIDTKEDMKIL; IDTKEDMKILY;
DTKEDMKILYS; TKEDMKILYSE; KEDMKILYSEI; EDMKILYSEIA;
DMKILYSEIAE; MKILYSEIAEL; KILYSEIAELR; ILYSEIAELRK;
LYSEIAELRKK; YSEIAELRKKL; SEIAELRKKLN; EIAELRKKLNL;
IAELRKKLNLN; AELRKKLNLNH; ELRKKLNLNHL; LRKKLNLNHLE;
RKKLNLNHLEI; KKLNLNHLEID; KLNLNHLEIDD; LNLNHLEIDDT;
NLNHLEIDDTL; LNHLEIDDTLE; NHLEIDDTLEK; HLEIDDTLEKV;
LEIDDTLEKVA; EIDDTLEKVAK; IDDTLEKVAKE; DDTLEKVAKEY;

| | |
|---|---|
| | DTLEKVAKEYA; TLEKVAKEYAI; LEKVAKEYAIK; EKVAKEYAIKL; KVAKEYAIKLG; VAKEYAIKLGE; AKEYAIKLGEN; KEYAIKLGENR; EYAIKLGENRT; YAIKLGENRTI; AIKLGENRTIT; IKLGENRTITH; KLGENRTITHT; LGENRTITHTL; GENRTITHTLF; ENRTITHTLFG; NRTITHTLFGT; RTITHTLFGTT; TITHTLFGTTP; ITHTLFGTTPM; THTLFGTTPMQ; HTLFGTTPMQR; TLFGTTPMQRI; LFGTTPMQRIH; FGTTPMQRIHK; GTTPMQRIHKY; TTPMQRIHKYD; TPMQRIHKYDQ; PMQRIHKYDQS; MQRIHKYDQSF; QRIHKYDQSFN; RIHKYDQSFNL; IHKYDQSFNLT; HKYDQSFNLTR; KYDQSFNLTRE; YDQSFNLTREI; DQSFNLTREIL; QSFNLTREILA; SFNLTREILAS; FNLTREILASG; NLTREILASGI; LTREILASGIE; TREILASGIEL; REILASGIELN; EILASGIELNR; ILASGIELNRV; LASGIELNRVV; ASGIELNRVVN; SGIELNRVVNA; GIELNRVVNAW; IELNRVVNAWL; ELNRVVNAWLN; LNRVVNAWLNS; NRVVNAWLNSP; RVVNAWLNSPS; VVNAWLNSPSH; VNAWLNSPSHK; NAWLNSPSHKE; AWLNSPSHKEA; WLNSPSHKEAL; LNSPSHKEALI; NSPSHKEALIN; SPSHKEALINT; PSHKEALINTD; SHKEALINTDT; HKEALINTDTD; KEALINTDTDK; EALINTDTDKI; ALINTDTDKIG; LINTDTDKIGG; INTDTDKIGGY; NTDTDKIGGYR; TDTDKIGGYRL; DTDKIGGYRLK; TDKIGGYRLKT; DKIGGYRLKTT; KIGGYRLKTTD; IGGYRLKTTDN; GGYRLKTTDNI; GYRLKTTDNID; YRLKTTDNIDI; RLKTTDNIDIF; LKTTDNIDIFV; KTTDNIDIFVV; TTDNIDIFVVL; TDNIDIFVVLF; DNIDIFVVLFG; NIDIFVVLFGK; IDIFVVLFGKR; DIFVVLFGKRK; IFVVLFGKRKY; FVVLFGKRKYK; VVLFGKRKYKN; |
| Seq 32 | SEQ ID NO 38920-39737/<br>8 mers:<br>MMQRISIL; MQRISILL; QRISILLM; RISILLML; ISILLMLL; SILLMLLA; ILLMLLAV; LLMLLAVF; LMLLAVFS; MLLAVFSC; LLAVFSCK; LAVFSCKQ; AVFSCKQF; VFSCKQFG; FSCKQFGD; SCKQFGDV; CKQFGDVK; KQFGDVKS; QFGDVKSL; FGDVKSLT; GDVKSLTE; DVKSLTEI; VKSLTEID; KSLTEIDS; SLTEIDSG; LTEIDSGN; TEIDSGNG; EIDSGNGI; IDSGNGIP; DSGNGIPL; SGNGIPLV; GNGIPLVV; NGIPLVVS; GIPLVVSD; IPLVVSDV; PLVVSDVV; LVVSDVVK; VVSDVVKD; VSDVVKDL; SDVVKDLI; DVVKDLIP; VVKDLIPK; VKDLIPKE; KDLIPKEI; DLIPKEIS; LIPKEISL; IPKEISLT; PKEISLTP; KEISLTPE; EISLTPEE; ISLTPEEA; SLTPEEAE; LTPEEAEK; TPEEAEKL; PEEAEKLE; EEAEKLES; EAEKLESL; AEKLESLK; EKLESLKV; KLESLKVF; LESLKVFL; ESLKVFLK; SLKVFLKD; LKVFLKDA; KVFLKDAM; VFLKDAMS; FLKDAMSV; LKDAMSVN; KDAMSVNG; DAMSVNGR; AMSVNGRE; MSVNGREE; SVNGREEA; VNGREEAL; NGREEALK; GREEALKA; REEALKAE; EEALKAEY; EALKAEYE; ALKAEYEK; LKAEYEKS; KAEYEKSY; AEYEKSYK; EYEKSYKE; YEKSYKEF; EKSYKEFF; KSYKEFFD; SYKEFFDW; YKEFFDWL; KEFFDWLS; EFFDWLSK; FFDWLSKD; FDWLSKDV; DWLSKDVN; WLSKDVNR; LSKDVNRQ; SKDVNRQK; KDVNRQKE; DVNRQKEF; VNRQKEFI; NRQKEFIS; RQKEFISS; QKEFISSF; KEFISSFD; EFISSFDN; FISSFDNI; ISSFDNIS; SSFDNISS; SFDNISSI; FDNISSIV; DNISSIVS; NISSIVSK; ISSIVSKA; SSIVSKAV; SIVSKAVD; IVSKAVDA; VSKAVDAS; SKAVDASK; KAVDASKK; AVDASKKR; VDASKKRR; DASKKRRP; ASKKRRPT; SKKRRPTE; KKRRPTEQ; |

KRRPTEQQ; RRPTEQQS; RPTEQQSL; PTEQQSLG; TEQQSLGF;
EQQSLGFK; QQSLGFKE; QSLGFKEY; SLGFKEYV; LGFKEYVC;
GFKEYVCY; FKEYVCYK; KEYVCYKI; EYVCYKIK; YVCYKIKN;
VCYKIKNS; CYKIKNSK; YKIKNSKG; KIKNSKGE; IKNSKGEA;
KNSKGEAL; NSKGEALS; SKGEALSL; KGEALSLF; GEALSLFF;
EALSLFFQ; ALSLFFQK; LSLFFQKV; SLFFQKVV; LFFQKVVD;
FFQKVVDA; FQKVVDAF; QKVVDAFG; KVVDAFGA; VVDAFGAD;
VDAFGADP; DAFGADPY; AFGADPYK; FGADPYKK; GADPYKKD;
ADPYKKDN; DPYKKDND; PYKKDNDE; YKKDNDES; KKDNDESV;
KDNDESVQ; DNDESVQK; NDESVQKP; DESVQKPV; ESVQKPVK;
SVQKPVKC; VQKPVKCN; QKPVKCNE; KPVKCNEE; PVKCNEEI;
VKCNEEIF; KCNEEIFK; CNEEIFKV; NEEIFKVI; EEIFKVIK;
EIFKVIKK; IFKVIKKV; FKVIKKVL; KVIKKVLT; VIKKVLTE;
IKKVLTES; KKVLTESE; KVLTESES; VLTESESN; LTESESNN;
TESESNNE; ESESNNEL; SESNNELK; ESNNELKN; SNNELKNL;
NNELKNLK; NELKNLKN; ELKNLKNY; LKNLKNYG; KNLKNYGN;
NLKNYGNV;

9 mers:
MMQRISILL; MQRISILLM; QRISILLML; RISILLMLL; ISILLMLLA;
SILLMLLAV; ILLMLLAVF; LLMLLAVFS; LMLLAVFSC; MLLAVFSCK;
LLAVFSCKQ; LAVFSCKQF; AVFSCKQFG; VFSCKQFGD; FSCKQFGDV;
SCKQFGDVK; CKQFGDVKS; KQFGDVKSL; QFGDVKSLT; FGDVKSLTE;
GDVKSLTEI; DVKSLTEID; VKSLTEIDS; KSLTEIDSG; SLTEIDSGN;
LTEIDSGNG; TEIDSGNGI; EIDSGNGIP; IDSGNGIPL; DSGNGIPLV;
SGNGIPLVV; GNGIPLVVS; NGIPLVVSD; GIPLVVSDV; IPLVVSDVV;
PLVVSDVVK; LVVSDVVKD; VVSDVVKDL; VSDVVKDLI; SDVVKDLIP;
DVVKDLIPK; VVKDLIPKE; VKDLIPKEI; KDLIPKEIS; DLIPKEISL;
LIPKEISLT; IPKEISLTP; PKEISLTPE; KEISLTPEE; EISLTPEEA;
ISLTPEEAE; SLTPEEAEK; LTPEEAEKL; TPEEAEKLE; PEEAEKLES;
EEAEKLESL; EAEKLESLK; AEKLESLKV; EKLESLKVF; KLESLKVFL;
LESLKVFLK; ESLKVFLKD; SLKVFLKDA; LKVFLKDAM; KVFLKDAMS;
VFLKDAMSV; FLKDAMSVN; LKDAMSVNG; KDAMSVNGR; DAMSVNGRE;
AMSVNGREE; MSVNGREEA; SVNGREEAL; VNGREEALK; NGREEALKA;
GREEALKAE; REEALKAEY; EEALKAEYE; EALKAEYEK; ALKAEYEKS;
LKAEYEKSY; KAEYEKSYK; AEYEKSYKE; EYEKSYKEF; YEKSYKEFF;
EKSYKEFFD; KSYKEFFDW; SYKEFFDWL; YKEFFDWLS; KEFFDWLSK;
EFFDWLSKD; FFDWLSKDV; FDWLSKDVN; DWLSKDVNR; WLSKDVNRQ;
LSKDVNRQK; SKDVNRQKE; KDVNRQKEF; DVNRQKEFI; VNRQKEFIS;
NRQKEFISS; RQKEFISSF; QKEFISSFD; KEFISSFDN; EFISSFDNI;
FISSFDNIS; ISSFDNISS; SSFDNISSI; SFDNISSIV; FDNISSIVS;
DNISSIVSK; NISSIVSKA; ISSIVSKAV; SSIVSKAVD; SIVSKAVDA;
IVSKAVDAS; VSKAVDASK; SKAVDASKK; KAVDASKKR; AVDASKKRR;
VDASKKRRP; DASKKRRPT; ASKKRRPTE; SKKRRPTEQ; KKRRPTEQQ;
KRRPTEQQS; RRPTEQQSL; RPTEQQSLG; PTEQQSLGF; TEQQSLGFK;
EQQSLGFKE; QQSLGFKEY; QSLGFKEYV; SLGFKEYVC; LGFKEYVCY;
GFKEYVCYK; FKEYVCYKI; KEYVCYKIK; EYVCYKIKN; YVCYKIKNS;
VCYKIKNSK; CYKIKNSKG; YKIKNSKGE; KIKNSKGEA; IKNSKGEAL;
KNSKGEALS; NSKGEALSL; SKGEALSLF; KGEALSLFF; GEALSLFFQ;
EALSLFFQK; ALSLFFQKV; LSLFFQKVV; SLFFQKVVD; LFFQKVVDA;
FFQKVVDAF; FQKVVDAFG; QKVVDAFGA; KVVDAFGAD; VVDAFGADP;

VDAFGADPY; DAFGADPYK; AFGADPYKK; FGADPYKKD; GADPYKKDN;
ADPYKKDND; DPYKKDNDE; PYKKDNDES; YKKDNDESV; KKDNDESVQ;
KDNDESVQK; DNDESVQKP; NDESVQKPV; DESVQKPVK; ESVQKPVKC;
SVQKPVKCN; VQKPVKCNE; QKPVKCNEE; KPVKCNEEI; PVKCNEEIF;
VKCNEEIFK; KCNEEIFKV; CNEEIFKVI; NEEIFKVIK; EEIFKVIKK;
EIFKVIKKV; IFKVIKKVL; FKVIKKVLT; KVIKKVLTE; VIKKVLTES;
IKKVLTESE; KKVLTESES; KVLTESESN; VLTESESNN; LTESESNNE;
TESESNNEL; ESESNNELK; SESNNELKN; ESNNELKNL; SNNELKNLK;
NNELKNLKN; NELKNLKNY; ELKNLKNYG; LKNLKNYGN; KNLKNYGNV;

10 mers:
MMQRISILLM; MQRISILLML; QRISILLMLL; RISILLMLLA;
ISILLMLLAV; SILLMLLAVF; ILLMLLAVFS; LLMLLAVFSC;
LMLLAVFSCK; MLLAVFSCKQ; LLAVFSCKQF; LAVFSCKQFG;
AVFSCKQFGD; VFSCKQFGDV; FSCKQFGDVK; SCKQFGDVKS;
CKQFGDVKSL; KQFGDVKSLT; QFGDVKSLTE; FGDVKSLTEI;
GDVKSLTEID; DVKSLTEIDS; VKSLTEIDSG; KSLTEIDSGN;
SLTEIDSGNG; LTEIDSGNGI; TEIDSGNGIP; EIDSGNGIPL;
IDSGNGIPLV; DSGNGIPLVV; SGNGIPLVVS; GNGIPLVVSD;
NGIPLVVSDV; GIPLVVSDVV; IPLVVSDVVK; PLVVSDVVKD;
LVVSDVVKDL; VVSDVVKDLI; VSDVVKDLIP; SDVVKDLIPK;
DVVKDLIPKE; VVKDLIPKEI; VKDLIPKEIS; KDLIPKEISL;
DLIPKEISLT; LIPKEISLTP; IPKEISLTPE; PKEISLTPEE;
KEISLTPEEA; EISLTPEEAE; ISLTPEEAEK; SLTPEEAEKL;
LTPEEAEKLE; TPEEAEKLES; PEEAEKLESL; EEAEKLESLK;
EAEKLESLKV; AEKLESLKVF; EKLESLKVFL; KLESLKVFLK;
LESLKVFLKD; ESLKVFLKDA; SLKVFLKDAM; LKVFLKDAMS;
KVFLKDAMSV; VFLKDAMSVN; FLKDAMSVNG; LKDAMSVNGR;
KDAMSVNGRE; DAMSVNGREE; AMSVNGREEA; MSVNGREEAL;
SVNGREEALK; VNGREEALKA; NGREEALKAE; GREEALKAEY;
REEALKAEYE; EEALKAEYEK; EALKAEYEKS; ALKAEYEKSY;
LKAEYEKSYK; KAEYEKSYKE; AEYEKSYKEF; EYEKSYKEFF;
YEKSYKEFFD; EKSYKEFFDW; KSYKEFFDWL; SYKEFFDWLS;
YKEFFDWLSK; KEFFDWLSKD; EFFDWLSKDV; FFDWLSKDVN;
FDWLSKDVNR; DWLSKDVNRQ; WLSKDVNRQK; LSKDVNRQKE;
SKDVNRQKEF; KDVNRQKEFI; DVNRQKEFIS; VNRQKEFISS;
NRQKEFISSF; RQKEFISSFD; QKEFISSFDN; KEFISSFDNI;
EFISSFDNIS; FISSFDNISS; ISSFDNISSI; SSFDNISSIV;
SFDNISSIVS; FDNISSIVSK; DNISSIVSKA; NISSIVSKAV;
ISSIVSKAVD; SSIVSKAVDA; SIVSKAVDAS; IVSKAVDASK;
VSKAVDASKK; SKAVDASKKR; KAVDASKKRR; AVDASKKRRP;
VDASKKRRPT; DASKKRRPTE; ASKKRRPTEQ; SKKRRPTEQQ;
KKRRPTEQQS; KRRPTEQQSL; RRPTEQQSLG; RPTEQQSLGF;
PTEQQSLGFK; TEQQSLGFKE; EQQSLGFKEY; QQSLGFKEYV;
QSLGFKEYVC; SLGFKEYVCY; LGFKEYVCYK; GFKEYVCYKI;
FKEYVCYKIK; KEYVCYKIKN; EYVCYKIKNS; YVCYKIKNSK;
VCYKIKNSKG; CYKIKNSKGE; YKIKNSKGEA; KIKNSKGEAL;
IKNSKGEALS; KNSKGEALSL; NSKGEALSLF; SKGEALSLFF;
KGEALSLFFQ; GEALSLFFQK; EALSLFFQKV; ALSLFFQKVV;
LSLFFQKVVD; SLFFQKVVDA; LFFQKVVDAF; FFQKVVDAFG;
FQKVVDAFGA; QKVVDAFGAD; KVVDAFGADP; VVDAFGADPY;

```
VDAFGADPYK;  DAFGADPYKK;  AFGADPYKKD;  FGADPYKKDN;
GADPYKKDND;  ADPYKKDNDE;  DPYKKDNDES;  PYKKDNDESV;
YKKDNDESVQ;  KKDNDESVQK;  KDNDESVQKP;  DNDESVQKPV;
NDESVQKPVK;  DESVQKPVKC;  ESVQKPVKCN;  SVQKPVKCNE;
VQKPVKCNEE;  QKPVKCNEEI;  KPVKCNEEIF;  PVKCNEEIFK;
VKCNEEIFKV;  KCNEEIFKVI;  CNEEIFKVIK;  NEEIFKVIKK;
EEIFKVIKKV;  EIFKVIKKVL;  IFKVIKKVLT;  FKVIKKVLTE;
KVIKKVLTES;  VIKKVLTESE;  IKKVLTESES;  KKVLTESESN;
KVLTESESNN;  VLTESESNNE;  LTESESNNEL;  TESESNNELK;
ESESNNELKN;  SESNNELKNL;  ESNNELKNLK;  SNNELKNLKN;
NNELKNLKNY;  NELKNLKNYG;  ELKNLKNYGN;  LKNLKNYGNV;

11 mers:
MMQRISILLML;  MQRISILLMLL;  QRISILLMLLA;  RISILLMLLAV;
ISILLMLLAVF;  SILLMLLAVFS;  ILLMLLAVFSC;  LLMLLAVFSCK;
LMLLAVFSCKQ;  MLLAVFSCKQF;  LLAVFSCKQFG;  LAVFSCKQFGD;
AVFSCKQFGDV;  VFSCKQFGDVK;  FSCKQFGDVKS;  SCKQFGDVKSL;
CKQFGDVKSLT;  KQFGDVKSLTE;  QFGDVKSLTEI;  FGDVKSLTEID;
GDVKSLTEIDS;  DVKSLTEIDSG;  VKSLTEIDSGN;  KSLTEIDSGNG;
SLTEIDSGNGI;  LTEIDSGNGIP;  TEIDSGNGIPL;  EIDSGNGIPLV;
IDSGNGIPLVV;  DSGNGIPLVVS;  SGNGIPLVVSD;  GNGIPLVVSDV;
NGIPLVVSDVV;  GIPLVVSDVVK;  IPLVVSDVVKD;  PLVVSDVVKDL;
LVVSDVVKDLI;  VVSDVVKDLIP;  VSDVVKDLIPK;  SDVVKDLIPKE;
DVVKDLIPKEI;  VVKDLIPKEIS;  VKDLIPKEISL;  KDLIPKEISLT;
DLIPKEISLTP;  LIPKEISLTPE;  IPKEISLTPEE;  PKEISLTPEEA;
KEISLTPEEAE;  EISLTPEEAEK;  ISLTPEEAEKL;  SLTPEEAEKLE;
LTPEEAEKLES;  TPEEAEKLESL;  PEEAEKLESLK;  EEAEKLESLKV;
EAEKLESLKVF;  AEKLESLKVFL;  EKLESLKVFLK;  KLESLKVFLKD;
LESLKVFLKDA;  ESLKVFLKDAM;  SLKVFLKDAMS;  LKVFLKDAMSV;
KVFLKDAMSVN;  VFLKDAMSVNG;  FLKDAMSVNGR;  LKDAMSVNGRE;
KDAMSVNGREE;  DAMSVNGREEA;  AMSVNGREEAL;  MSVNGREEALK;
SVNGREEALKA;  VNGREEALKAE;  NGREEALKAEY;  GREEALKAEYE;
REEALKAEYEK;  EEALKAEYEKS;  EALKAEYEKSY;  ALKAEYEKSYK;
LKAEYEKSYKE;  KAEYEKSYKEF;  AEYEKSYKEFF;  EYEKSYKEFFD;
YEKSYKEFFDW;  EKSYKEFFDWL;  KSYKEFFDWLS;  SYKEFFDWLSK;
YKEFFDWLSKD;  KEFFDWLSKDV;  EFFDWLSKDVN;  FFDWLSKDVNR;
FDWLSKDVNRQ;  DWLSKDVNRQK;  WLSKDVNRQKE;  LSKDVNRQKEF;
SKDVNRQKEFI;  KDVNRQKEFIS;  DVNRQKEFISS;  VNRQKEFISSF;
NRQKEFISSFD;  RQKEFISSFDN;  QKEFISSFDNI;  KEFISSFDNIS;
EFISSFDNISS;  FISSFDNISSI;  ISSFDNISSIV;  SSFDNISSIVS;
SFDNISSIVSK;  FDNISSIVSKA;  DNISSIVSKAV;  NISSIVSKAVD;
ISSIVSKAVDA;  SSIVSKAVDAS;  SIVSKAVDASK;  IVSKAVDASKK;
VSKAVDASKKR;  SKAVDASKKRR;  KAVDASKKRRP;  AVDASKKRRPT;
VDASKKRRPTE;  DASKKRRPTEQ;  ASKKRRPTEQQ;  SKKRRPTEQQS;
KKRRPTEQQSL;  KRRPTEQQSLG;  RRPTEQQSLGF;  RPTEQQSLGFK;
PTEQQSLGFKE;  TEQQSLGFKEY;  EQQSLGFKEYV;  QQSLGFKEYVC;
QSLGFKEYVCY;  SLGFKEYVCYK;  LGFKEYVCYKI;  GFKEYVCYKIK;
FKEYVCYKIKN;  KEYVCYKIKNS;  EYVCYKIKNSK;  YVCYKIKNSKG;
VCYKIKNSKGE;  CYKIKNSKGEA;  YKIKNSKGEAL;  KIKNSKGEALS;
IKNSKGEALSL;  KNSKGEALSLF;  NSKGEALSLFF;  SKGEALSLFFQ;
KGEALSLFFQK;  GEALSLFFQKV;  EALSLFFQKVV;  ALSLFFQKVVD;
```

| | |
|---|---|
| | LSLFFQKVVDA; SLFFQKVVDAF; LFFQKVVDAFG; FFQKVVDAFGA; FQKVVDAFGAD; QKVVDAFGADP; KVVDAFGADPY; VVDAFGADPYK; VDAFGADPYKK; DAFGADPYKKD; AFGADPYKKDN; FGADPYKKDND; GADPYKKDNDE; ADPYKKDNDES; DPYKKDNDESV; PYKKDNDESVQ; YKKDNDESVQK; KKDNDESVQKP; KDNDESVQKPV; DNDESVQKPVK; NDESVQKPVKC; DESVQKPVKCN; ESVQKPVKCNE; SVQKPVKCNEE; VQKPVKCNEEI; QKPVKCNEEIF; KPVKCNEEIFK; PVKCNEEIFKV; VKCNEEIFKVI; KCNEEIFKVIK; CNEEIFKVIKK; NEEIFKVIKKV; EEIFKVIKKVL; EIFKVIKKVLT; IFKVIKKVLTE; FKVIKKVLTES; KVIKKVLTESE; VIKKVLTESES; IKKVLTESESN; KKVLTESESNN; KVLTESESNNE; VLTESESNNEL; LTESESNNELK; TESESNNELKN; ESESNNELKNL; SESNNELKNLK; ESNNELKNLKN; SNNELKNLKNY; NNELKNLKNYG; NELKNLKNYGN; ELKNLKNYGNV; |
| Seq 33 | SEQ ID NO 39738-40661/<br>8 mers:<br>MKRISILS; KRISILSI; RISILSIL; ISILSILL; SILSILLL; ILSILLLL; LSILLLLL; SILLLLLL; ILLLLLLF; LLLLLLFS; LLLLLFSC; LLLLFSCK; LLLFSCKQ; LLFSCKQY; LFSCKQYG; FSCKQYGD; SCKQYGDV; CKQYGDVK; KQYGDVKS; QYGDVKSL; YGDVKSLT; GDVKSLTE; DVKSLTEV; VKSLTEVA; KSLTEVAT; SLTEVATD; LTEVATDL; TEVATDLE; EVATDLED; VATDLEDD; ATDLEDDN; TDLEDDNS; DLEDDNSF; LEDDNSFA; EDDNSFAS; DDNSFASG; DNSFASGS; NSFASGSV; SFASGSVE; FASGSVES; ASGSVESK; SGSVESKD; GSVESKDQ; SVESKDQI; VESKDQII; ESKDQIIE; SKDQIIEK; KDQIIEKG; DQIIEKGP; QIIEKGPV; IIEKGPVL; IEKGPVLT; EKGPVLTS; KGPVLTSE; GPVLTSEE; PVLTSEEF; VLTSEEFE; LTSEEFER; TSEEFERL; SEEFERLE; EEFERLEA; EFERLEAL; FERLEALK; ERLEALKT; RLEALKTF; LEALKTFL; EALKTFLK; ALKTFLKD; LKTFLKDA; KTFLKDAM; TFLKDAMG; FLKDAMGV; LKDAMGVN; KDAMGVNG; DAMGVNGR; AMGVNGRE; MGVNGREG; GVNGREGD; VNGREGDT; NGREGDTK; GREGDTKA; REGDTKAE; EGDTKAEY; GDTKAEYE; DTKAEYEK; TKAEYEKS; KAEYEKSY; AEYEKSYK; EYEKSYKE; YEKSYKEF; EKSYKEFF; KSYKEFFD; SYKEFFDW; YKEFFDWL; KEFFDWLS; EFFDWLSK; FFDWLSKD; FDWLSKDV; DWLSKDVN; WLSKDVNR; LSKDVNRQ; SKDVNRQK; KDVNRQKE; DVNRQKEF; VNRQKEFV; NRQKEFVS; RQKEFVSF; QKEFVSFF; KEFVSFFN; EFVSFFNN; FVSFFNNI; VSFFNNIC; SFFNNICG; FFNNICGI; FNNICGII; NNICGIIT; NICGIITK; ICGIITKA; CGIITKAV; GIITKAVD; IITKAVDA; ITKAVDAS; TKAVDASK; KAVDASKK; AVDASKKR; VDASKKRY; DASKKRYN; ASKKRYNS; SKKRYNSN; KKRYNSNP; KRYNSNPK; RYNSNPKS; YNSNPKSL; NSNPKSLG; SNPKSLGF; NPKSLGFN; PKSLGFNE; KSLGFNEY; SLGFNEYV; LGFNEYVC; GFNEYVCY; FNEYVCYD; NEYVCYDI; EYVCYDIK; YVCYDIKT; VCYDIKTR; CYDIKTRT; YDIKTRTG; DIKTRTGD; IKTRTGDD; KTRTGDDL; TRTGDDLS; RTGDDLSL; TGDDLSLF; GDDLSLFF; DDLSLFFQ; DLSLFFQK; LSLFFQKV; SLFFQKVA; LFFQKVAD; FFQKVADA; FQKVADAF; QKVADAFG; KVADAFGT; VADAFGTQ; ADAFGTQE; DAFGTQEY; AFGTQEYK; FGTQEYKN; GTQEYKNK; TQEYKNKD; QEYKNKDE; EYKNKDED; YKNKDEDD; KNKDEDDE; |

NKDEDDEN; KDEDDENN; DEDDENNQ; EDDENNQK; DDENNQKP;
DENNQKPE; ENNQKPEK; NNQKPEKC; NQKPEKCN; QKPEKCNE;
KPEKCNEE; PEKCNEEI; EKCNEEIF; KCNEEIFK; CNEEIFKV;
NEEIFKVI; EEIFKVIK; EIFKVIKR; IFKVIKRV; FKVIKRVF;
KVIKRVFT; VIKRVFTE; IKRVFTES; KRVFTESE; RVFTESEN;
VFTESENN; FTESENNN; TESENNNE; ESENNNEL; SENNNELA;
ENNNELAN; NNNELANL; NNELANLK; NELANLKN; ELANLKNL;
LANLKNLN; ANLKNLNS; NLKNLNSY; LKNLNSYN; KNLNSYNL;
NLNSYNLN; LNSYNLNS; NSYNLNSN; SYNLNSNN; YNLNSNNK;

9 mers:
MKRISILSI; KRISILSIL; RISILSILL; ISILSILLL; SILSILLLL;
ILSILLLLL; LSILLLLLL; SILLLLLLF; ILLLLLLFS; LLLLLLFSC;
LLLLLFSCK; LLLLFSCKQ; LLLFSCKQY; LLFSCKQYG; LFSCKQYGD;
FSCKQYGDV; SCKQYGDVK; CKQYGDVKS; KQYGDVKSL; QYGDVKSLT;
YGDVKSLTE; GDVKSLTEV; DVKSLTEVA; VKSLTEVAT; KSLTEVATD;
SLTEVATDL; LTEVATDLE; TEVATDLED; EVATDLEDD; VATDLEDDN;
ATDLEDDNS; TDLEDDNSF; DLEDDNSFA; LEDDNSFAS; EDDNSFASG;
DDNSFASGS; DNSFASGSV; NSFASGSVE; SFASGSVES; FASGSVESK;
ASGSVESKD; SGSVESKDQ; GSVESKDQI; SVESKDQII; VESKDQIIE;
ESKDQIIEK; SKDQIIEKG; KDQIIEKGP; DQIIEKGPV; QIIEKGPVL;
IIEKGPVLT; IEKGPVLTS; EKGPVLTSE; KGPVLTSEE; GPVLTSEEF;
PVLTSEEFE; VLTSEEFER; LTSEEFERL; TSEEFERLE; SEEFERLEA;
EEFERLEAL; EFERLEALK; FERLEALKT; ERLEALKTF; RLEALKTFL;
LEALKTFLK; EALKTFLKD; ALKTFLKDA; LKTFLKDAM; KTFLKDAMG;
TFLKDAMGV; FLKDAMGVN; LKDAMGVNG; KDAMGVNGR; DAMGVNGRE;
AMGVNGREG; MGVNGREGD; GVNGREGDT; VNGREGDTK; NGREGDTKA;
GREGDTKAE; REGDTKAEY; EGDTKAEYE; GDTKAEYEK; DTKAEYEKS;
TKAEYEKSY; KAEYEKSYK; AEYEKSYKE; EYEKSYKEF; YEKSYKEFF;
EKSYKEFFD; KSYKEFFDW; SYKEFFDWL; YKEFFDWLS; KEFFDWLSK;
EFFDWLSKD; FFDWLSKDV; FDWLSKDVN; DWLSKDVNR; WLSKDVNRQ;
LSKDVNRQK; SKDVNRQKE; KDVNRQKEF; DVNRQKEFV; VNRQKEFVS;
NRQKEFVSF; RQKEFVSFF; QKEFVSFFN; KEFVSFFNN; EFVSFFNNI;
FVSFFNNIC; VSFFNNICG; SFFNNICGI; FFNNICGII; FNNICGIIT;
NNICGIITK; NICGIITKA; ICGIITKAV; CGIITKAVD; GIITKAVDA;
IITKAVDAS; ITKAVDASK; TKAVDASKK; KAVDASKKR; AVDASKKRY;
VDASKKRYN; DASKKRYNS; ASKKRYNSN; SKKRYNSNP; KKRYNSNPK;
KRYNSNPKS; RYNSNPKSL; YNSNPKSLG; NSNPKSLGF; SNPKSLGFN;
NPKSLGFNE; PKSLGFNEY; KSLGFNEYV; SLGFNEYVC; LGFNEYVCY;
GFNEYVCYD; FNEYVCYDI; NEYVCYDIK; EYVCYDIKT; YVCYDIKTR;
VCYDIKTRT; CYDIKTRTG; YDIKTRTGD; DIKTRTGDD; IKTRTGDDL;
KTRTGDDLS; TRTGDDLSL; RTGDDLSLF; TGDDLSLFF; GDDLSLFFQ;
DDLSLFFQK; DLSLFFQKV; LSLFFQKVA; SLFFQKVAD; LFFQKVADA;
FFQKVADAF; FQKVADAFG; QKVADAFGT; KVADAFGTQ; VADAFGTQE;
ADAFGTQEY; DAFGTQEYK; AFGTQEYKN; FGTQEYKNK; GTQEYKNKD;
TQEYKNKDE; QEYKNKDED; EYKNKDEDD; YKNKDEDDE; KNKDEDDEN;
NKDEDDENN; KDEDDENNQ; DEDDENNQK; EDDENNQKP; DDENNQKPE;
DENNQKPEK; ENNQKPEKC; NNQKPEKCN; NQKPEKCNE; QKPEKCNEE;
KPEKCNEEI; PEKCNEEIF; EKCNEEIFK; KCNEEIFKV; CNEEIFKVI;
NEEIFKVIK; EEIFKVIKR; EIFKVIKRV; IFKVIKRVF; FKVIKRVFT;
KVIKRVFTE; VIKRVFTES; IKRVFTESE; KRVFTESEN; RVFTESENN;

VFTESENNN; FTESENNNE; TESENNNEL; ESENNNELA; SENNNELAN;
ENNNELANL; NNNELANLK; NNELANLKN; NELANLKNL; ELANLKNLN;
LANLKNLNS; ANLKNLNSY; NLKNLNSYN; LKNLNSYNL; KNLNSYNLN;
NLNSYNLNS; LNSYNLNSN; NSYNLNSNN; SYNLNSNNK;

10 mers:
MKRISILSIL; KRISILSILL; RISILSILLL; ISILSILLLL;
SILSILLLLL; ILSILLLLLL; LSILLLLLLF; SILLLLLLFS;
ILLLLLLFSC; LLLLLLFSCK; LLLLLFSCKQ; LLLLFSCKQY;
LLLFSCKQYG; LLFSCKQYGD; LFSCKQYGDV; FSCKQYGDVK;
SCKQYGDVKS; CKQYGDVKSL; KQYGDVKSLT; QYGDVKSLTE;
YGDVKSLTEV; GDVKSLTEVA; DVKSLTEVAT; VKSLTEVATD;
KSLTEVATDL; SLTEVATDLE; LTEVATDLED; TEVATDLEDD;
EVATDLEDDN; VATDLEDDNS; ATDLEDDNSF; TDLEDDNSFA;
DLEDDNSFAS; LEDDNSFASG; EDDNSFASGS; DDNSFASGSV;
DNSFASGSVE; NSFASGSVES; SFASGSVESK; FASGSVESKD;
ASGSVESKDQ; SGSVESKDQI; GSVESKDQII; SVESKDQIIE;
VESKDQIIEK; ESKDQIIEKG; SKDQIIEKGP; KDQIIEKGPV;
DQIIEKGPVL; QIIEKGPVLT; IIEKGPVLTS; IEKGPVLTSE;
EKGPVLTSEE; KGPVLTSEEF; GPVLTSEEFE; PVLTSEEFER;
VLTSEEFERL; LTSEEFERLE; TSEEFERLEA; SEEFERLEAL;
EEFERLEALK; EFERLEALKT; FERLEALKTF; ERLEALKTFL;
RLEALKTFLK; LEALKTFLKD; EALKTFLKDA; ALKTFLKDAM;
LKTFLKDAMG; KTFLKDAMGV; TFLKDAMGVN; FLKDAMGVNG;
LKDAMGVNGR; KDAMGVNGRE; DAMGVNGREG; AMGVNGREGD;
MGVNGREGDT; GVNGREGDTK; VNGREGDTKA; NGREGDTKAE;
GREGDTKAEY; REGDTKAEYE; EGDTKAEYEK; GDTKAEYEKS;
DTKAEYEKSY; TKAEYEKSYK; KAEYEKSYKE; AEYEKSYKEF;
EYEKSYKEFF; YEKSYKEFFD; EKSYKEFFDW; KSYKEFFDWL;
SYKEFFDWLS; YKEFFDWLSK; KEFFDWLSKD; EFFDWLSKDV;
FFDWLSKDVN; FDWLSKDVNR; DWLSKDVNRQ; WLSKDVNRQK;
LSKDVNRQKE; SKDVNRQKEF; KDVNRQKEFV; DVNRQKEFVS;
VNRQKEFVSF; NRQKEFVSFF; RQKEFVSFFN; QKEFVSFFNN;
KEFVSFFNNI; EFVSFFNNIC; FVSFFNNICG; VSFFNNICGI;
SFFNNICGII; FFNNICGIIT; FNNICGIITK; NNICGIITKA;
NICGIITKAV; ICGIITKAVD; CGIITKAVDA; GIITKAVDAS;
IITKAVDASK; ITKAVDASKK; TKAVDASKKR; KAVDASKKRY;
AVDASKKRYN; VDASKKRYNS; DASKKRYNSN; ASKKRYNSNP;
SKKRYNSNPK; KKRYNSNPKS; KRYNSNPKSL; RYNSNPKSLG;
YNSNPKSLGF; NSNPKSLGFN; SNPKSLGFNE; NPKSLGFNEY;
PKSLGFNEYV; KSLGFNEYVC; SLGFNEYVCY; LGFNEYVCYD;
GFNEYVCYDI; FNEYVCYDIK; NEYVCYDIKT; EYVCYDIKTR;
YVCYDIKTRT; VCYDIKTRTG; CYDIKTRTGD; YDIKTRTGDD;
DIKTRTGDDL; IKTRTGDDLS; KTRTGDDLSL; TRTGDDLSLF;
RTGDDLSLFF; TGDDLSLFFQ; GDDLSLFFQK; DDLSLFFQKV;
DLSLFFQKVA; LSLFFQKVAD; SLFFQKVADA; LFFQKVADAF;
FFQKVADAFG; FQKVADAFGT; QKVADAFGTQ; KVADAFGTQE;
VADAFGTQEY; ADAFGTQEYK; DAFGTQEYKN; AFGTQEYKNK;
FGTQEYKNKD; GTQEYKNKDE; TQEYKNKDED; QEYKNKDEDD;
EYKNKDEDDE; YKNKDEDDEN; KNKDEDDENN; NKDEDDENNQ;
KDEDDENNQK; DEDDENNQKP; EDDENNQKPE; DDENNQKPEK;

DENNQKPEKC; ENNQKPEKCN; NNQKPEKCNE; NQKPEKCNEE;
QKPEKCNEEI; KPEKCNEEIF; PEKCNEEIFK; EKCNEEIFKV;
KCNEEIFKVI; CNEEIFKVIK; NEEIFKVIKR; EEIFKVIKRV;
EIFKVIKRVF; IFKVIKRVFT; FKVIKRVFTE; KVIKRVFTES;
VIKRVFTESE; IKRVFTESEN; KRVFTESENN; RVFTESENNN;
VFTESENNNE; FTESENNNEL; TESENNNELA; ESENNNELAN;
SENNNELANL; ENNNELANLK; NNNELANLKN; NNELANLKNL;
NELANLKNLN; ELANLKNLNS; LANLKNLNSY; ANLKNLNSYN;
NLKNLNSYNL; LKNLNSYNLN; KNLNSYNLNS; NLNSYNLNSN;
LNSYNLNSNN; NSYNLNSNNK;

11 mers:
MKRISILSILL; KRISILSILLL; RISILSILLLL; ISILSILLLLL;
SILSILLLLLL; ILSILLLLLLF; LSILLLLLLFS; SILLLLLLFSC;
ILLLLLLFSCK; LLLLLLFSCKQ; LLLLLFSCKQY; LLLLFSCKQYG;
LLLFSCKQYGD; LLFSCKQYGDV; LFSCKQYGDVK; FSCKQYGDVKS;
SCKQYGDVKSL; CKQYGDVKSLT; KQYGDVKSLTE; QYGDVKSLTEV;
YGDVKSLTEVA; GDVKSLTEVAT; DVKSLTEVATD; VKSLTEVATDL;
KSLTEVATDLE; SLTEVATDLED; LTEVATDLEDD; TEVATDLEDDN;
EVATDLEDDNS; VATDLEDDNSF; ATDLEDDNSFA; TDLEDDNSFAS;
DLEDDNSFASG; LEDDNSFASGS; EDDNSFASGSV; DDNSFASGSVE;
DNSFASGSVES; NSFASGSVESK; SFASGSVESKD; FASGSVESKDQ;
ASGSVESKDQI; SGSVESKDQII; GSVESKDQIIE; SVESKDQIIEK;
VESKDQIIEKG; ESKDQIIEKGP; SKDQIIEKGPV; KDQIIEKGPVL;
DQIIEKGPVLT; QIIEKGPVLTS; IIEKGPVLTSE; IEKGPVLTSEE;
EKGPVLTSEEF; KGPVLTSEEFE; GPVLTSEEFER; PVLTSEEFERL;
VLTSEEFERLE; LTSEEFERLEA; TSEEFERLEAL; SEEFERLEALK;
EEFERLEALKT; EFERLEALKTF; FERLEALKTFL; ERLEALKTFLK;
RLEALKTFLKD; LEALKTFLKDA; EALKTFLKDAM; ALKTFLKDAMG;
LKTFLKDAMGV; KTFLKDAMGVN; TFLKDAMGVNG; FLKDAMGVNGR;
LKDAMGVNGRE; KDAMGVNGREG; DAMGVNGREGD; AMGVNGREGDT;
MGVNGREGDTK; GVNGREGDTKA; VNGREGDTKAE; NGREGDTKAEY;
GREGDTKAEYE; REGDTKAEYEK; EGDTKAEYEKS; GDTKAEYEKSY;
DTKAEYEKSYK; TKAEYEKSYKE; KAEYEKSYKEF; AEYEKSYKEFF;
EYEKSYKEFFD; YEKSYKEFFDW; EKSYKEFFDWL; KSYKEFFDWLS;
SYKEFFDWLSK; YKEFFDWLSKD; KEFFDWLSKDV; EFFDWLSKDVN;
FFDWLSKDVNR; FDWLSKDVNRQ; DWLSKDVNRQK; WLSKDVNRQKE;
LSKDVNRQKEF; SKDVNRQKEFV; KDVNRQKEFVS; DVNRQKEFVSF;
VNRQKEFVSFF; NRQKEFVSFFN; RQKEFVSFFNN; QKEFVSFFNNI;
KEFVSFFNNIC; EFVSFFNNICG; FVSFFNNICGI; VSFFNNICGII;
SFFNNICGIIT; FFNNICGIITK; FNNICGIITKA; NNICGIITKAV;
NICGIITKAVD; ICGIITKAVDA; CGIITKAVDAS; GIITKAVDASK;
IITKAVDASKK; ITKAVDASKKR; TKAVDASKKRY; KAVDASKKRYN;
AVDASKKRYNS; VDASKKRYNSN; DASKKRYNSNP; ASKKRYNSNPK;
SKKRYNSNPKS; KKRYNSNPKSL; KRYNSNPKSLG; RYNSNPKSLGF;
YNSNPKSLGFN; NSNPKSLGFNE; SNPKSLGFNEY; NPKSLGFNEYV;
PKSLGFNEYVC; KSLGFNEYVCY; SLGFNEYVCYD; LGFNEYVCYDI;
GFNEYVCYDIK; FNEYVCYDIKT; NEYVCYDIKTR; EYVCYDIKTRT;
YVCYDIKTRTG; VCYDIKTRTGD; CYDIKTRTGDD; YDIKTRTGDDL;
DIKTRTGDDLS; IKTRTGDDLSL; KTRTGDDLSLF; TRTGDDLSLFF;
RTGDDLSLFFQ; TGDDLSLFFQK; GDDLSLFFQKV; DDLSLFFQKVA;

| | |
|---|---|
| | DLSLFFQKVAD; LSLFFQKVADA; SLFFQKVADAF; LFFQKVADAFG; FFQKVADAFGT; FQKVADAFGTQ; QKVADAFGTQE; KVADAFGTQEY; VADAFGTQEYK; ADAFGTQEYKN; DAFGTQEYKNK; AFGTQEYKNKD; FGTQEYKNKDE; GTQEYKNKDED; TQEYKNKDEDD; QEYKNKDEDDE; EYKNKDEDDEN; YKNKDEDDENN; KNKDEDDENNQ; NKDEDDENNQK; KDEDDENNQKP; DEDDENNQKPE; EDDENNQKPEK; DDENNQKPEKC; DENNQKPEKCN; ENNQKPEKCNE; NNQKPEKCNEE; NQKPEKCNEEI; QKPEKCNEEIF; KPEKCNEEIFK; PEKCNEEIFKV; EKCNEEIFKVI; KCNEEIFKVIK; CNEEIFKVIKR; NEEIFKVIKRV; EEIFKVIKRVF; EIFKVIKRVFT; IFKVIKRVFTE; FKVIKRVFTES; KVIKRVFTESE; VIKRVFTESEN; IKRVFTESENN; KRVFTESENNN; RVFTESENNNE; VFTESENNNEL; FTESENNNELA; TESENNNELAN; ESENNNELANL; SENNNELANLK; ENNNELANLKN; NNNELANLKNL; NNELANLKNLN; NELANLKNLNS; ELANLKNLNSY; LANLKNLNSYN; ANLKNLNSYNL; NLKNLNSYNLN; LKNLNSYNLNS; KNLNSYNLNSN; NLNSYNLNSNN; LNSYNLNSNNK; |
| Seq 34 | SEQ ID NO 40612–42220/<br>8 mers:<br>MKIKPLIQ; KIKPLIQL; IKPLIQLK; KPLIQLKL; PLIQLKLL; LIQLKLLG; IQLKLLGL; QLKLLGLF; LKLLGLFL; KLLGLFLF; LLGLFLFS; LGLFLFSC; GLFLFSCT; LFLFSCTI; FLFSCTID; LFSCTIDA; FSCTIDAN; SCTIDANL; CTIDANLN; TIDANLNE; IDANLNED; DANLNEDY; ANLNEDYK; NLNEDYKN; LNEDYKNK; NEDYKNKV; EDYKNKVK; DYKNKVKG; YKNKVKGI; KNKVKGIL; NKVKGILN; KVKGILNK; VKGILNKA; KGILNKAA; GILNKAAD; ILNKAADD; LNKAADDQ; NKAADDQE; KAADDQET; AADDQETT; ADDQETTS; DDQETTSA; DQETTSAD; QETTSADT; ETTSADTN; TTSADTNS; TSADTNSN; SADTNSNA; ADTNSNAA; DTNSNAAK; TNSNAAKN; NSNAAKNI; SNAAKNIP; NAAKNIPI; AAKNIPIA; AKNIPIAD; KNIPIADN; NIPIADND; IPIADNDK; PIADNDKV; IADNDKVA; ADNDKVAA; DNDKVAAE; NDKVAAEL; DKVAAELK; KVAAELKK; VAAELKKQ; AAELKKQS; AELKKQSQ; ELKKQSQA; LKKQSQAA; KKQSQAAK; KQSQAAKT; QSQAAKTV; SQAAKTVA; QAAKTVAA; AAKTVAAA; AKTVAAAP; KTVAAAPN; TVAAAPNK; VAAAPNKG; AAAPNKGS; AAPNKGSQ; APNKGSQN; PNKGSQNQ; NKGSQNQP; KGSQNQPQ; GSQNQPQT; SQNQPQTT; QNQPQTTP; NQPQTTPN; QPQTTPNK; PQTTPNKG; QTTPNKGS; TTPNKGSQ; TPNKGSQN; PNKGSQNQ; NKGSQNQQ; KGSQNQQA; GSQNQQAA; SQNQQAAP; QNQQAAPS; NQQAAPSP; QQAAPSPQ; QAAPSPQL; AAPSPQLQ; APSPQLQS; PSPQLQSL; SPQLQSLS; PQLQSLSF; QLQSLSFS; LQSLSFSA; QSLSFSAD; SLSFSADL; LSFSADLS; SFSADLSN; FSADLSNL; SADLSNLP; ADLSNLPK; DLSNLPKT; LSNLPKTT; SNLPKTTA; NLPKTTAA; LPKTTAAR; PKTTAARA; KTTAARAA; TTAARAAS; TAARAASL; AARAASLT; ARAASLTK; RAASLTKQ; AASLTKQR; ASLTKQRI; SLTKQRIP; LTKQRIPI; TKQRIPIQ; KQRIPIQA; QRIPIQAV; RIPIQAVT; IPIQAVTT; PIQAVTTV; IQAVTTVP; QAVTTVPG; AVTTVPGN; VTTVPGNT; TTVPGNTR; TVPGNTRT; VPGNTRTF; PGNTRTFN; GNTRTFNS; NTRTFNSR; TRTFNSRN; RTFNSRNS; TFNSRNSG; FNSRNSGL; NSRNSGLP; SRNSGLPT; RNSGLPTF; NSGLPTFA; SGLPTFAL; |

EP 2 254 592 B1

| | |
|---|---|
| | GLPTFALN; LPTFALNY; PTFALNYS; TFALNYSF; FALNYSFS; ALNYSFSQ; LNYSFSQP; NYSFSQPT; YSFSQPTR; SFSQPTRQ; FSQPTRQQ; SQPTRQQT; QPTRQQTN; PTRQQTNS; TRQQTNSS; RQQTNSSS; QQTNSSSA; QTNSSSAV; TNSSSAVQ; NSSSAVQT; SSSAVQTT; SSAVQTTT; SAVQTTTS; AVQTTTSS; VQTTTSSG; QTTTSSGS; TTTSSGSK; TTSSGSKL; TSSGSKLQ; SSGSKLQT; SGSKLQTL; GSKLQTLK; SKLQTLKN; KLQTLKNE; LQTLKNEL; QTLKNELI; TLKNELIR; LKNELIRA; KNELIRAI; NELIRAIS; ELIRAISE; LIRAISEE; IRAISEEK; RAISEEKN; AISEEKNK; ISEEKNKT; SEEKNKTQ; EEKNKTQN; EKNKTQNN; KNKTQNNF; NKTQNNFG; KTQNNFGF; TQNNFGFR; QNNFGFRE; NNFGFRET; NFGFRETY; FGFRETYD; GFRETYDQ; FRETYDQF; RETYDQFK; ETYDQFKM; TYDQFKMK; YDQFKMKD; DQFKMKDS; QFKMKDSA; FKMKDSAF; KMKDSAFE; MKDSAFEL; KDSAFELL; DSAFELLD; SAFELLDV; AFELLDVI; FELLDVIS; ELLDVISS; LLDVISSA; LDVISSAK; DVISSAKV; VISSAKVY; ISSAKVYD; SSAKVYDR; SAKVYDRS; AKVYDRSY; KVYDRSYA; VYDRSYAP; YDRSYAPQ; DRSYAPQL; RSYAPQLN; SYAPQLNS; YAPQLNSN; APQLNSNT; PQLNSNTP; QLNSNTPE; LNSNTPEA; NSNTPEAE; SNTPEAEN; NTPEAENE; TPEAENER; PEAENERN; EAENERNK; AENERNKF; ENERNKFY; NERNKFYA; ERNKFYAL; RNKFYALM; NKFYALMD; KFYALMDF; FYALMDFD; YALMDFDQ; ALMDFDQY; LMDFDQYK; MDFDQYKI; DFDQYKIE; FDQYKIEQ; DQYKIEQF; QYKIEQFG; YKIEQFGS; KIEQFGSI; IEQFGSIM; EQFGSIME; QFGSIMEA; FGSIMEAL; GSIMEALY; SIMEALYN; IMEALYNE; MEALYNEN; EALYNENQ; ALYNENQN; LYNENQNH; YNENQNHS; NENQNHSL; ENQNHSLI; NQNHSLIR; QNHSLIRE; NHSLIREL; HSLIRELM; SLIRELMI; LIRELMIS; IRELMISG; RELMISGL; ELMISGLG; LMISGLGT; MISGLGTQ; ISGLGTQI; SGLGTQIS; GLGTQISF; LGTQISFE; GTQISFEL; TQISFELA; QISFELAL; ISFELALE; SFELALEE; FELALEEI; ELALEEIN; LALEEINK; ALEEINKK; LEEINKKI; EEINKKIE; EINKKIEI; INKKIEIF; NKKIEIFN; KKIEIFNQ; KIEIFNQD; IEIFNQDY; EIFNQDYL; IFNQDYLN; FNQDYLNA; NQDYLNAK; QDYLNAKI; DYLNAKIN; YLNAKINS; LNAKINSF; NAKINSFD; AKINSFDF; KINSFDFT; INSFDFTM; NSFDFTMK; SFDFTMKL; FDFTMKLK; DFTMKLKE; FTMKLKEL; TMKLKELK; MKLKELKS; KLKELKSK; LKELKSKL; KELKSKLN; ELKSKLNQ; LKSKLNQI; KSKLNQIL; SKLNQILD; KLNQILDK; LNQILDKR; NQILDKRK; QILDKRKE; ILDKRKEW; LDKRKEWS; DKRKEWSR; KRKEWSRQ; RKEWSRQA; KEWSRQAD; EWSRQADG; WSRQADGL; SRQADGLI; RQADGLIA; QADGLIAN; ADGLIANA; DGLIANAS; GLIANASS; LIANASSN; IANASSNS; ANASSNSS; NASSNSSL; ASSNSSLS; SSNSSLSD; SNSSLSDS; NSSLSDSK; SSLSDSKS; SLSDSKSL; LSDSKSLA; SDSKSLAE; DSKSLAEY; SKSLAEYI; KSLAEYIK; SLAEYIKK; LAEYIKKR; AEYIKKRY; EYIKKRYL; YIKKRYLD; IKKRYLDN; KKRYLDNM; KRYLDNMQ; RYLDNMQN; YLDNMQNA; LDNMQNAR; DNMQNARQ; NMQNARQS; MQNARQSV; QNARQSVL; NARQSVLE; ARQSVLEA; RQSVLEAY; QSVLEAYI; SVLEAYIS; VLEAYISI; LEAYISIM;

9 mers: |

764

MKIKPLIQL; KIKPLIQLK; IKPLIQLKL; KPLIQLKLL; PLIQLKLLG;
LIQLKLLGL; IQLKLLGLF; QLKLLGLFL; LKLLGLFLF; KLLGLFLFS;
LLGLFLFSC; LGLFLFSCT; GLFLFSCTI; LFLFSCTID; FLFSCTIDA;
LFSCTIDAN; FSCTIDANL; SCTIDANLN; CTIDANLNE; TIDANLNED;
IDANLNEDY; DANLNEDYK; ANLNEDYKN; NLNEDYKNK; LNEDYKNKV;
NEDYKNKVK; EDYKNKVKG; DYKNKVKGI; YKNKVKGIL; KNKVKGILN;
NKVKGILNK; KVKGILNKA; VKGILNKAA; KGILNKAAD; GILNKAADD;
ILNKAADDQ; LNKAADDQE; NKAADDQET; KAADDQETT; AADDQETTS;
ADDQETTSA; DDQETTSAD; DQETTSADT; QETTSADTN; ETTSADTNS;
TTSADTNSN; TSADTNSNA; SADTNSNAA; ADTNSNAAK; DTNSNAAKN;
TNSNAAKNI; NSNAAKNIP; SNAAKNIPI; NAAKNIPIA; AAKNIPIAD;
AKNIPIADN; KNIPIADND; NIPIADNDK; IPIADNDKV; PIADNDKVA;
IADNDKVAA; ADNDKVAAE; DNDKVAAEL; NDKVAAELK; DKVAAELKK;
KVAAELKKQ; VAAELKKQS; AAELKKQSQ; AELKKQSQA; ELKKQSQAA;
LKKQSQAAK; KKQSQAAKT; KQSQAAKTV; QSQAAKTVA; SQAAKTVAA;
QAAKTVAAA; AAKTVAAAP; AKTVAAAPN; KTVAAAPNK; TVAAAPNKG;
VAAAPNKGS; AAAPNKGSQ; AAPNKGSQN; APNKGSQNQ; PNKGSQNQP;
NKGSQNQPQ; KGSQNQPQT; GSQNQPQTT; SQNQPQTTP; QNQPQTTPN;
NQPQTTPNK; QPQTTPNKG; PQTTPNKGS; QTTPNKGSQ; TTPNKGSQN;
TPNKGSQNQ; PNKGSQNQQ; NKGSQNQQA; KGSQNQQAA; GSQNQQAAP;
SQNQQAAPS; QNQQAAPSP; NQQAAPSPQ; QQAAPSPQL; QAAPSPQLQ;
AAPSPQLQS; APSPQLQSL; PSPQLQSLS; SPQLQSLSF; PQLQSLSFS;
QLQSLSFSA; LQSLSFSAD; QSLSFSADL; SLSFSADLS; LSFSADLSN;
SFSADLSNL; FSADLSNLP; SADLSNLPK; ADLSNLPKT; DLSNLPKTT;
LSNLPKTTA; SNLPKTTAA; NLPKTTAAR; LPKTTAARA; PKTTAARAA;
KTTAARAAS; TTAARAASL; TAARAASLT; AARAASLTK; ARAASLTKQ;
RAASLTKQR; AASLTKQRI; ASLTKQRIP; SLTKQRIPI; LTKQRIPIQ;
TKQRIPIQA; KQRIPIQAV; QRIPIQAVT; RIPIQAVTT; IPIQAVTTV;
PIQAVTTVP; IQAVTTVPG; QAVTTVPGN; AVTTVPGNT; VTTVPGNTR;
TTVPGNTRT; TVPGNTRTF; VPGNTRTFN; PGNTRTFNS; GNTRTFNSR;
NTRTFNSRN; TRTFNSRNS; RTFNSRNSG; TFNSRNSGL; FNSRNSGLP;
NSRNSGLPT; SRNSGLPTF; RNSGLPTFA; NSGLPTFAL; SGLPTFALN;
GLPTFALNY; LPTFALNYS; PTFALNYSF; TFALNYSFS; FALNYSFSQ;
ALNYSFSQP; LNYSFSQPT; NYSFSQPTR; YSFSQPTRQ; SFSQPTRQQ;
FSQPTRQQT; SQPTRQQTN; QPTRQQTNS; PTRQQTNSS; TRQQTNSSS;
RQQTNSSSA; QQTNSSSAV; QTNSSSAVQ; TNSSSAVQT; NSSSAVQTT;
SSSAVQTTT; SSAVQTTTS; SAVQTTTSS; AVQTTTSSG; VQTTTSSGS;
QTTTSSGSK; TTTSSGSKL; TTSSGSKLQ; TSSGSKLQT; SSGSKLQTL;
SGSKLQTLK; GSKLQTLKN; SKLQTLKNE; KLQTLKNEL; LQTLKNELI;
QTLKNELIR; TLKNELIRA; LKNELIRAI; KNELIRAIS; NELIRAISE;
ELIRAISEE; LIRAISEEK; IRAISEEKN; RAISEEKNK; AISEEKNKT;
ISEEKNKTQ; SEEKNKTQN; EEKNKTQNN; EKNKTQNNF; KNKTQNNFG;
NKTQNNFGF; KTQNNFGFR; TQNNFGFRE; QNNFGFRET; NNFGFRETY;
NFGFRETYD; FGFRETYDQ; GFRETYDQF; FRETYDQFK; RETYDQFKM;
ETYDQFKMK; TYDQFKMKD; YDQFKMKDS; DQFKMKDSA; QFKMKDSAF;
FKMKDSAFE; KMKDSAFEL; MKDSAFELL; KDSAFELLD; DSAFELLDV;
SAFELLDVI; AFELLDVIS; FELLDVISS; ELLDVISSA; LLDVISSAK;
LDVISSAKV; DVISSAKVY; VISSAKVYD; ISSAKVYDR; SSAKVYDRS;
SAKVYDRSY; AKVYDRSYA; KVYDRSYAP; VYDRSYAPQ; YDRSYAPQL;
DRSYAPQLN; RSYAPQLNS; SYAPQLNSN; YAPQLNSNT; APQLNSNTP;
PQLNSNTPE; QLNSNTPEA; LNSNTPEAE; NSNTPEAEN; SNTPEAENE;

NTPEAENER; TPEAENERN; PEAENERNK; EAENERNKF; AENERNKFY;
ENERNKFYA; NERNKFYAL; ERNKFYALM; RNKFYALMD; NKFYALMDF;
KFYALMDFD; FYALMDFDQ; YALMDFDQY; ALMDFDQYK; LMDFDQYKI;
MDFDQYKIE; DFDQYKIEQ; FDQYKIEQF; DQYKIEQFG; QYKIEQFGS;
YKIEQFGSI; KIEQFGSIM; IEQFGSIME; EQFGSIMEA; QFGSIMEAL;
FGSIMEALY; GSIMEALYN; SIMEALYNE; IMEALYNEN; MEALYNENQ;
EALYNENQN; ALYNENQNH; LYNENQNHS; YNENQNHSL; NENQNHSLI;
ENQNHSLIR; NQNHSLIRE; QNHSLIREL; NHSLIRELM; HSLIRELMI;
SLIRELMIS; LIRELMISG; IRELMISGL; RELMISGLG; ELMISGLGT;
LMISGLGTQ; MISGLGTQI; ISGLGTQIS; SGLGTQISF; GLGTQISFE;
LGTQISFEL; GTQISFELA; TQISFELAL; QISFELALE; ISFELALEE;
SFELALEEI; FELALEEIN; ELALEEINK; LALEEINKK; ALEEINKKI;
LEEINKKIE; EEINKKIEI; EINKKIEIF; INKKIEIFN; NKKIEIFNQ;
KKIEIFNQD; KIEIFNQDY; IEIFNQDYL; EIFNQDYLN; IFNQDYLNA;
FNQDYLNAK; NQDYLNAKI; QDYLNAKIN; DYLNAKINS; YLNAKINSF;
LNAKINSFD; NAKINSFDF; AKINSFDFT; KINSFDFTM; INSFDFTMK;
NSFDFTMKL; SFDFTMKLK; FDFTMKLKE; DFTMKLKEL; FTMKLKELK;
TMKLKELKS; MKLKELKSK; KLKELKSKL; LKELKSKLN; KELKSKLNQ;
ELKSKLNQI; LKSKLNQIL; KSKLNQILD; SKLNQILDK; KLNQILDKR;
LNQILDKRK; NQILDKRKE; QILDKRKEW; ILDKRKEWS; LDKRKEWSR;
DKRKEWSRQ; KRKEWSRQA; RKEWSRQAD; KEWSRQADG; EWSRQADGL;
WSRQADGLI; SRQADGLIA; RQADGLIAN; QADGLIANA; ADGLIANAS;
DGLIANASS; GLIANASSN; LIANASSNS; IANASSNSS; ANASSNSSL;
NASSNSSLS; ASSNSSLSD; SSNSSLSDS; SNSSLSDSK; NSSLSDSKS;
SSLSDSKSL; SLSDSKSLA; LSDSKSLAE; SDSKSLAEY; DSKSLAEYI;
SKSLAEYIK; KSLAEYIKK; SLAEYIKKR; LAEYIKKRY; AEYIKKRYL;
EYIKKRYLD; YIKKRYLDN; IKKRYLDNM; KKRYLDNMQ; KRYLDNMQN;
RYLDNMQNA; YLDNMQNAR; LDNMQNARQ; DNMQNARQS; NMQNARQSV;
MQNARQSVL; QNARQSVLE; NARQSVLEA; ARQSVLEAY; RQSVLEAYI;
QSVLEAYIS; SVLEAYISI; VLEAYISIM;

10 mers:
MKIKPLIQLK; KIKPLIQLKL; IKPLIQLKLL; KPLIQLKLLG;
PLIQLKLLGL; LIQLKLLGLF; IQLKLLGLFL; QLKLLGLFLF;
LKLLGLFLFS; KLLGLFLFSC; LLGLFLFSCT; LGLFLFSCTI;
GLFLFSCTID; LFLFSCTIDA; FLFSCTIDAN; LFSCTIDANL;
FSCTIDANLN; SCTIDANLNE; CTIDANLNED; TIDANLNEDY;
IDANLNEDYK; DANLNEDYKN; ANLNEDYKNK; NLNEDYKNKV;
LNEDYKNKVK; NEDYKNKVKG; EDYKNKVKGI; DYKNKVKGIL;
YKNKVKGILN; KNKVKGILNK; NKVKGILNKA; KVKGILNKAA;
VKGILNKAAD; KGILNKAADD; GILNKAADDQ; ILNKAADDQE;
LNKAADDQET; NKAADDQETT; KAADDQETTS; AADDQETTSA;
ADDQETTSAD; DDQETTSADT; DQETTSADTN; QETTSADTNS;
ETTSADTNSN; TTSADTNSNA; TSADTNSNAA; SADTNSNAAK;
ADTNSNAAKN; DTNSNAAKNI; TNSNAAKNIP; NSNAAKNIPI;
SNAAKNIPIA; NAAKNIPIAD; AAKNIPIADN; AKNIPIADND;
KNIPIADNDK; NIPIADNDKV; IPIADNDKVA; PIADNDKVAA;
IADNDKVAAE; ADNDKVAAEL; DNDKVAAELK; NDKVAAELKK;
DKVAAELKKQ; KVAAELKKQS; VAAELKKQSQ; AAELKKQSQA;
AELKKQSQAA; ELKKQSQAAK; LKKQSQAAKT; KKQSQAAKTV;
KQSQAAKTVA; QSQAAKTVAA; SQAAKTVAAA; QAAKTVAAAP;

AAKTVAAAPN; AKTVAAAPNK; KTVAAAPNKG; TVAAAPNKGS;
VAAAPNKGSQ; AAAPNKGSQN; AAPNKGSQNQ; APNKGSQNQP;
PNKGSQNQPQ; NKGSQNQPQT; KGSQNQPQTT; GSQNQPQTTP;
SQNQPQTTPN; QNQPQTTPNK; NQPQTTPNKG; QPQTTPNKGS;
PQTTPNKGSQ; QTTPNKGSQN; TTPNKGSQNQ; TPNKGSQNQQ;
PNKGSQNQQA; NKGSQNQQAA; KGSQNQQAAP; GSQNQQAAPS;
SQNQQAAPSP; QNQQAAPSPQ; NQQAAPSPQL; QQAAPSPQLQ;
QAAPSPQLQS; AAPSPQLQSL; APSPQLQSLS; PSPQLQSLSF;
SPQLQSLSFS; PQLQSLSFSA; QLQSLSFSAD; LQSLSFSADL;
QSLSFSADLS; SLSFSADLSN; LSFSADLSNL; SFSADLSNLP;
FSADLSNLPK; SADLSNLPKT; ADLSNLPKTT; DLSNLPKTTA;
LSNLPKTTAA; SNLPKTTAAR; NLPKTTAARA; LPKTTAARAA;
PKTTAARAAS; KTTAARAASL; TTAARAASLT; TAARAASLTK;
AARAASLTKQ; ARAASLTKQR; RAASLTKQRI; AASLTKQRIP;
ASLTKQRIPI; SLTKQRIPIQ; LTKQRIPIQA; TKQRIPIQAV;
KQRIPIQAVT; QRIPIQAVTT; RIPIQAVTTV; IPIQAVTTVP;
PIQAVTTVPG; IQAVTTVPGN; QAVTTVPGNT; AVTTVPGNTR;
VTTVPGNTRT; TTVPGNTRTF; TVPGNTRTFN; VPGNTRTFNS;
PGNTRTFNSR; GNTRTFNSRN; NTRTFNSRNS; TRTFNSRNSG;
RTFNSRNSGL; TFNSRNSGLP; FNSRNSGLPT; NSRNSGLPTF;
SRNSGLPTFA; RNSGLPTFAL; NSGLPTFALN; SGLPTFALNY;
GLPTFALNYS; LPTFALNYSF; PTFALNYSFS; TFALNYSFSQ;
FALNYSFSQP; ALNYSFSQPT; LNYSFSQPTR; NYSFSQPTRQ;
YSFSQPTRQQ; SFSQPTRQQT; FSQPTRQQTN; SQPTRQQTNS;
QPTRQQTNSS; PTRQQTNSSS; TRQQTNSSSA; RQQTNSSSAV;
QQTNSSSAVQ; QTNSSSAVQT; TNSSSAVQTT; NSSSAVQTTT;
SSSAVQTTTS; SSAVQTTTSS; SAVQTTTSSG; AVQTTTSSGS;
VQTTTSSGSK; QTTTSSGSKL; TTTSSGSKLQ; TTSSGSKLQT;
TSSGSKLQTL; SSGSKLQTLK; SGSKLQTLKN; GSKLQTLKNE;
SKLQTLKNEL; KLQTLKNELI; LQTLKNELIR; QTLKNELIRA;
TLKNELIRAI; LKNELIRAIS; KNELIRAISE; NELIRAISEE;
ELIRAISEEK; LIRAISEEKN; IRAISEEKNK; RAISEEKNKT;
AISEEKNKTQ; ISEEKNKTQN; SEEKNKTQNN; EEKNKTQNNF;
EKNKTQNNFG; KNKTQNNFGF; NKTQNNFGFR; KTQNNFGFRE;
TQNNFGFRET; QNNFGFRETY; NNFGFRETYD; NFGFRETYDQ;
FGFRETYDQF; GFRETYDQFK; FRETYDQFKM; RETYDQFKMK;
ETYDQFKMKD; TYDQFKMKDS; YDQFKMKDSA; DQFKMKDSAF;
QFKMKDSAFE; FKMKDSAFEL; KMKDSAFELL; MKDSAFELLD;
KDSAFELLDV; DSAFELLDVI; SAFELLDVIS; AFELLDVISS;
FELLDVISSA; ELLDVISSAK; LLDVISSAKV; LDVISSAKVY;
DVISSAKVYD; VISSAKVYDR; ISSAKVYDRS; SSAKVYDRSY;
SAKVYDRSYA; AKVYDRSYAP; KVYDRSYAPQ; VYDRSYAPQL;
YDRSYAPQLN; DRSYAPQLNS; RSYAPQLNSN; SYAPQLNSNT;
YAPQLNSNTP; APQLNSNTPE; PQLNSNTPEA; QLNSNTPEAE;
LNSNTPEAEN; NSNTPEAENE; SNTPEAENER; NTPEAENERN;
TPEAENERNK; PEAENERNKF; EAENERNKFY; AENERNKFYA;
ENERNKFYAL; NERNKFYALM; ERNKFYALMD; RNKFYALMDF;
NKFYALMDFD; KFYALMDFDQ; FYALMDFDQY; YALMDFDQYK;
ALMDFDQYKI; LMDFDQYKIE; MDFDQYKIEQ; DFDQYKIEQF;
FDQYKIEQFG; DQYKIEQFGS; QYKIEQFGSI; YKIEQFGSIM;
KIEQFGSIME; IEQFGSIMEA; EQFGSIMEAL; QFGSIMEALY;

FGSIMEALYN; GSIMEALYNE; SIMEALYNEN; IMEALYNENQ;
MEALYNENQN; EALYNENQNH; ALYNENQNHS; LYNENQNHSL;
YNENQNHSLI; NENQNHSLIR; ENQNHSLIRE; NQNHSLIREL;
QNHSLIRELM; NHSLIRELMI; HSLIRELMIS; SLIRELMISG;
LIRELMISGL; IRELMISGLG; RELMISGLGT; ELMISGLGTQ;
LMISGLGTQI; MISGLGTQIS; ISGLGTQISF; SGLGTQISFE;
GLGTQISFEL; LGTQISFELA; GTQISFELAL; TQISFELALE;
QISFELALEE; ISFELALEEI; SFELALEEIN; FELALEEINK;
ELALEEINKK; LALEEINKKI; ALEEINKKIE; LEEINKKIEI;
EEINKKIEIF; EINKKIEIFN; INKKIEIFNQ; NKKIEIFNQD;
KKIEIFNQDY; KIEIFNQDYL; IEIFNQDYLN; EIFNQDYLNA;
IFNQDYLNAK; FNQDYLNAKI; NQDYLNAKIN; QDYLNAKINS;
DYLNAKINSF; YLNAKINSFD; LNAKINSFDF; NAKINSFDFT;
AKINSFDFTM; KINSFDFTMK; INSFDFTMKL; NSFDFTMKLK;
SFDFTMKLKE; FDFTMKLKEL; DFTMKLKELK; FTMKLKELKS;
TMKLKELKSK; MKLKELKSKL; KLKELKSKLN; LKELKSKLNQ;
KELKSKLNQI; ELKSKLNQIL; LKSKLNQILD; KSKLNQILDK;
SKLNQILDKR; KLNQILDKRK; LNQILDKRKE; NQILDKRKEW;
QILDKRKEWS; ILDKRKEWSR; LDKRKEWSRQ; DKRKEWSRQA;
KRKEWSRQAD; RKEWSRQADG; KEWSRQADGL; EWSRQADGLI;
WSRQADGLIA; SRQADGLIAN; RQADGLIANA; QADGLIANAS;
ADGLIANASS; DGLIANASSN; GLIANASSNS; LIANASSNSS;
IANASSNSSL; ANASSNSSLS; NASSNSSLSD; ASSNSSLSDS;
SSNSSLSDSK; SNSSLSDSKS; NSSLSDSKSL; SSLSDSKSLA;
SLSDSKSLAE; LSDSKSLAEY; SDSKSLAEYI; DSKSLAEYIK;
SKSLAEYIKK; KSLAEYIKKR; SLAEYIKKRY; LAEYIKKRYL;
AEYIKKRYLD; EYIKKRYLDN; YIKKRYLDNM; IKKRYLDNMQ;
KKRYLDNMQN; KRYLDNMQNA; RYLDNMQNAR; YLDNMQNARQ;
LDNMQNARQS; DNMQNARQSV; NMQNARQSVL; MQNARQSVLE;
QNARQSVLEA; NARQSVLEAY; ARQSVLEAYI; RQSVLEAYIS;
QSVLEAYISI;

11 mers:
MKIKPLIQLKL; KIKPLIQLKLL; IKPLIQLKLLG; KPLIQLKLLGL;
PLIQLKLLGLF; LIQLKLLGLFL; IQLKLLGLFLF; QLKLLGLFLFS;
LKLLGLFLFSC; KLLGLFLFSCT; LLGLFLFSCTI; LGLFLFSCTID;
GLFLFSCTIDA; LFLFSCTIDAN; FLFSCTIDANL; LFSCTIDANLN;
FSCTIDANLNE; SCTIDANLNED; CTIDANLNEDY; TIDANLNEDYK;
IDANLNEDYKN; DANLNEDYKNK; ANLNEDYKNKV; NLNEDYKNKVK;
LNEDYKNKVKG; NEDYKNKVKGI; EDYKNKVKGIL; DYKNKVKGILN;
YKNKVKGILNK; KNKVKGILNKA; NKVKGILNKAA; KVKGILNKAAD;
VKGILNKAADD; KGILNKAADDQ; GILNKAADDQE; ILNKAADDQET;
LNKAADDQETT; NKAADDQETTS; KAADDQETTSA; AADDQETTSAD;
ADDQETTSADT; DDQETTSADTN; DQETTSADTNS; QETTSADTNSN;
ETTSADTNSNA; TTSADTNSNAA; TSADTNSNAAK; SADTNSNAAKN;
ADTNSNAAKNI; DTNSNAAKNIP; TNSNAAKNIPI; NSNAAKNIPIA;
SNAAKNIPIAD; NAAKNIPIADN; AAKNIPIADND; AKNIPIADNDK;
KNIPIADNDKV; NIPIADNDKVA; IPIADNDKVAA; PIADNDKVAAE;
IADNDKVAAEL; ADNDKVAAELK; DNDKVAAELKK; NDKVAAELKKQ;
DKVAAELKKQS; KVAAELKKQSQ; VAAELKKQSQA; AAELKKQSQAA;
AELKKQSQAAK; ELKKQSQAAKT; LKKQSQAAKTV; KKQSQAAKTVA;

| | KQSQAAKTVAA; | QSQAAKTVAAA; | SQAAKTVAAAP; | QAAKTVAAAPN; |
|---|---|---|---|---|
| | AAKTVAAAPNK; | AKTVAAAPNKG; | KTVAAAPNKGS; | TVAAAPNKGSQ; |
| | VAAAPNKGSQN; | AAAPNKGSQNQ; | AAPNKGSQNQP; | APNKGSQNQPQ; |
| | PNKGSQNQPQT; | NKGSQNQPQTT; | KGSQNQPQTTP; | GSQNQPQTTPN; |
| | SQNQPQTTPNK; | QNQPQTTPNKG; | NQPQTTPNKGS; | QPQTTPNKGSQ; |
| | PQTTPNKGSQN; | QTTPNKGSQNQ; | TTPNKGSQNQQ; | TPNKGSQNQQA; |
| | PNKGSQNQQAA; | NKGSQNQQAAP; | KGSQNQQAAPS; | GSQNQQAAPSP; |
| | SQNQQAAPSPQ; | QNQQAAPSPQL; | NQQAAPSPQLQ; | QQAAPSPQLQS; |
| | QAAPSPQLQSL; | AAPSPQLQSLS; | APSPQLQSLSF; | PSPQLQSLSFS; |
| | SPQLQSLSFSA; | PQLQSLSFSAD; | QLQSLSFSADL; | LQSLSFSADLS; |
| | QSLSFSADLSN; | SLSFSADLSNL; | LSFSADLSNLP; | SFSADLSNLPK; |
| | FSADLSNLPKT; | SADLSNLPKTT; | ADLSNLPKTTA; | DLSNLPKTTAA; |
| | LSNLPKTTAAR; | SNLPKTTAARA; | NLPKTTAARAA; | LPKTTAARAAS; |
| | PKTTAARAASL; | KTTAARAASLT; | TTAARAASLTK; | TAARAASLTKQ; |
| | AARAASLTKQR; | ARAASLTKQRI; | RAASLTKQRIP; | AASLTKQRIPI; |
| | ASLTKQRIPIQ; | SLTKQRIPIQA; | LTKQRIPIQAV; | TKQRIPIQAVT; |
| | KQRIPIQAVTT; | QRIPIQAVTTV; | RIPIQAVTTVP; | IPIQAVTTVPG; |
| | PIQAVTTVPGN; | IQAVTTVPGNT; | QAVTTVPGNTR; | AVTTVPGNTRT; |
| | VTTVPGNTRTF; | TTVPGNTRTFN; | TVPGNTRTFNS; | VPGNTRTFNSR; |
| | PGNTRTFNSRN; | GNTRTFNSRNS; | NTRTFNSRNSG; | TRTFNSRNSGL; |
| | RTFNSRNSGLP; | TFNSRNSGLPT; | FNSRNSGLPTF; | NSRNSGLPTFA; |
| | SRNSGLPTFAL; | RNSGLPTFALN; | NSGLPTFALNY; | SGLPTFALNYS; |
| | GLPTFALNYSF; | LPTFALNYSFS; | PTFALNYSFSQ; | TFALNYSFSQP; |
| | FALNYSFSQPT; | ALNYSFSQPTR; | LNYSFSQPTRQ; | NYSFSQPTRQQ; |
| | YSFSQPTRQQT; | SFSQPTRQQTN; | FSQPTRQQTNS; | SQPTRQQTNSS; |
| | QPTRQQTNSSS; | PTRQQTNSSSA; | TRQQTNSSSAV; | RQQTNSSSAVQ; |
| | QQTNSSSAVQT; | QTNSSSAVQTT; | TNSSSAVQTTT; | NSSSAVQTTTS; |
| | SSSAVQTTTSS; | SSAVQTTTSSG; | SAVQTTTSSGS; | AVQTTTSSGSK; |
| | VQTTTSSGSKL; | QTTTSSGSKLQ; | TTTSSGSKLQT; | TTSSGSKLQTL; |
| | TSSGSKLQTLK; | SSGSKLQTLKN; | SGSKLQTLKNE; | GSKLQTLKNEL; |
| | SKLQTLKNELI; | KLQTLKNELIR; | LQTLKNELIRA; | QTLKNELIRAI; |
| | TLKNELIRAIS; | LKNELIRAISE; | KNELIRAISEE; | NELIRAISEEK; |
| | ELIRAISEEKN; | LIRAISEEKNK; | IRAISEEKNKT; | RAISEEKNKTQ; |
| | AISEEKNKTQN; | ISEEKNKTQNN; | SEEKNKTQNNF; | EEKNKTQNNFG; |
| | EKNKTQNNFGF; | KNKTQNNFGFR; | NKTQNNFGFRE; | KTQNNFGFRET; |
| | TQNNFGFRETY; | QNNFGFRETYD; | NNFGFRETYDQ; | NFGFRETYDQF; |
| | FGFRETYDQFK; | GFRETYDQFKM; | FRETYDQFKMK; | RETYDQFKMKD; |
| | ETYDQFKMKDS; | TYDQFKMKDSA; | YDQFKMKDSAF; | DQFKMKDSAFE; |
| | QFKMKDSAFEL; | FKMKDSAFELL; | KMKDSAFELLD; | MKDSAFELLDV; |
| | KDSAFELLDVI; | DSAFELLDVIS; | SAFELLDVISS; | AFELLDVISSA; |
| | FELLDVISSAK; | ELLDVISSAKV; | LLDVISSAKVY; | LDVISSAKVYD; |
| | DVISSAKVYDR; | VISSAKVYDRS; | ISSAKVYDRSY; | SSAKVYDRSYA; |
| | SAKVYDRSYAP; | AKVYDRSYAPQ; | KVYDRSYAPQL; | VYDRSYAPQLN; |
| | YDRSYAPQLNS; | DRSYAPQLNSN; | RSYAPQLNSNT; | SYAPQLNSNTP; |
| | YAPQLNSNTPE; | APQLNSNTPEA; | PQLNSNTPEAE; | QLNSNTPEAEN; |
| | LNSNTPEAENE; | NSNTPEAENER; | SNTPEAENERN; | NTPEAENERNK; |
| | TPEAENERNKF; | PEAENERNKFY; | EAENERNKFYA; | AENERNKFYAL; |
| | ENERNKFYALM; | NERNKFYALMD; | ERNKFYALMDF; | RNKFYALMDFD; |
| | NKFYALMDFDQ; | KFYALMDFDQY; | FYALMDFDQYK; | YALMDFDQYKI; |
| | ALMDFDQYKIE; | LMDFDQYKIEQ; | MDFDQYKIEQF; | DFDQYKIEQFG; |
| | FDQYKIEQFGS; | DQYKIEQFGSI; | QYKIEQFGSIM; | YKIEQFGSIME; |

| | |
|---|---|
| | KIEQFGSIMEA; IEQFGSIMEAL; EQFGSIMEALY; QFGSIMEALYN; FGSIMEALYNE; GSIMEALYNEN; SIMEALYNENQ; IMEALYNENQN; MEALYNENQNH; EALYNENQNHS; ALYNENQNHSL; LYNENQNHSLI; YNENQNHSLIR; NENQNHSLIRE; ENQNHSLIREL; NQNHSLIRELM; QNHSLIRELMI; NHSLIRELMIS; HSLIRELMISG; SLIRELMISGL; LIRELMISGLG; IRELMISGLGT; RELMISGLGTQ; ELMISGLGTQI; LMISGLGTQIS; MISGLGTQISF; ISGLGTQISFE; SGLGTQISFEL; GLGTQISFELA; LGTQISFELAL; GTQISFELALE; TQISFELALEE; QISFELALEEI; ISFELALEEIN; SFELALEEINK; FELALEEINKK; ELALEEINKKI; LALEEINKKIE; ALEEINKKIEI; LEEINKKIEIF; EEINKKIEIFN; EINKKIEIFNQ; INKKIEIFNQD; NKKIEIFNQDY; KKIEIFNQDYL; KIEIFNQDYLN; IEIFNQDYLNA; EIFNQDYLNAK; IFNQDYLNAKI; FNQDYLNAKIN; NQDYLNAKINS; QDYLNAKINSF; DYLNAKINSFD; YLNAKINSFDF; LNAKINSFDFT; NAKINSFDFTM; AKINSFDFTMK; KINSFDFTMKL; INSFDFTMKLK; NSFDFTMKLKE; SFDFTMKLKEL; FDFTMKLKELK; DFTMKLKELKS; FTMKLKELKSK; TMKLKELKSKL; MKLKELKSKLN; KLKELKSKLNQ; LKELKSKLNQI; KELKSKLNQIL; ELKSKLNQILD; LKSKLNQILDK; KSKLNQILDKR; SKLNQILDKRK; KLNQILDKRKE; LNQILDKRKEW; NQILDKRKEWS; QILDKRKEWSR; ILDKRKEWSRQ; LDKRKEWSRQA; DKRKEWSRQAD; KRKEWSRQADG; RKEWSRQADGL; KEWSRQADGLI; EWSRQADGLIA; WSRQADGLIAN; SRQADGLIANA; RQADGLIANAS; QADGLIANASS; ADGLIANASSN; DGLIANASSNS; GLIANASSNSS; LIANASSNSSL; IANASSNSSLS; ANASSNSSLSD; NASSNSSLSDS; ASSNSSLSDSK; SSNSSLSDSKS; SNSSLSDSKSL; NSSLSDSKSLA; SSLSDSKSLAE; SLSDSKSLAEY; LSDSKSLAEYI; SDSKSLAEYIK; DSKSLAEYIKK; SKSLAEYIKKR; KSLAEYIKKRY; SLAEYIKKRYL; LAEYIKKRYLD; AEYIKKRYLDN; EYIKKRYLDNM; YIKKRYLDNMQ; IKKRYLDNMQN; KKRYLDNMQNA; KRYLDNMQNAR; RYLDNMQNARQ; YLDNMQNARQS; LDNMQNARQSV; DNMQNARQSVL; NMQNARQSVLE; MQNARQSVLEA; QNARQSVLEAY; NARQSVLEAYI; ARQSVLEAYIS; RQSVLEAYISI; QSVLEAYISIM; |
| Seq 35 | SEQ ID NO 42221-43880/ <br> 8 mers: <br> MSSCTIDA; SSCTIDAN; SCTIDANL; CTIDANLN; TIDANLNK; IDANLNKD; DANLNKDY; ANLNKDYK; NLNKDYKN; LNKDYKNK; NKDYKNKV; KDYKNKVE; DYKNKVEE; YKNKVEEL; KNKVEELL; NKVEELLN; KVEELLNS; VEELLNSS; EELLNSST; ELLNSSTD; LLNSSTDD; LNSSTDDQ; NSSTDDQA; SSTDDQAK; STDDQAKI; TDDQAKIS; DDQAKISI; DQAKISIN; QAKISINT; AKISINTG; KISINTGS; ISINTGSN; SINTGSNA; INTGSNAT; NTGSNATK; TGSNATKN; GSNATKNK; SNATKNKT; NATKNKTN; ATKNKTNI; TKNKTNIK; KNKTNIKV; NKTNIKVA; KTNIKVAG; TNIKVAGL; NIKVAGLQ; IKVAGLQK; KVAGLQKN; VAGLQKNT; AGLQKNTQ; GLQKNTQS; LQKNTQSK; QKNTQSKK; KNTQSKKN; NTQSKKNN; TQSKKNNN; QSKKNNNL; SKKNNNLQ; KKNNNLQG; KNNNLQGL; NNNLQGLN; NNLQGLNP; NLQGLNPA; LQGLNPAN; QGLNPANQ; GLNPANQV; LNPANQVN; NPANQVNP; PANQVNPG; ANQVNPGN; NQVNPGNP; QVNPGNPM; VNPGNPMQ; NPGNPMQI; PGNPMQIA; GNPMQIAN; NPMQIANQ; PMQIANQA; MQIANQAN; QIANQANQ; |

IANQANQA; ANQANQAN; NQANQANQ; QANQANQA; ANQANQAN;
NQANQANQ; QANQANQA; ANQANQAN; NQANQANQ; QANQANQA;
ANQANQAN; NQANQANQ; QANQANQA; ANQANQAS; NQANQASQ;
QANQASQA; ANQASQAS; NQASQASQ; QASQASQA; ASQASQAS;
SQASQASQ; QASQASQV; ASQASQVA; SQASQVAS; QASQVASS;
ASQVASSA; SQVASSAS; QVASSASQ; VASSASQA; ASSASQAS;
SSASQASP; SASQASPV; ASQASPVA; SQASPVAS; QASPVASP;
ASPVASPA; SPVASPAT; PVASPATN; VASPATNV; ASPATNVQ;
SPATNVQA; PATNVQAT; ATNVQATP; TNVQATPP; NVQATPPK;
VQATPPKQ; QATPPKQI; ATPPKQIA; TPPKQIAS; PPKQIASA;
PKQIASAQ; KQIASAQA; QIASAQAI; IASAQAIQ; ASAQAIQT;
SAQAIQTV; AQAIQTVP; QAIQTVPN; AIQTVPNN; IQTVPNNT;
QTVPNNTS; TVPNNTST; VPNNTSTP; PNNTSTPN; NNTSTPNQ;
NTSTPNQS; TSTPNQSI; STPNQSII; TPNQSIIK; PNQSIIKP;
NQSIIKPQ; QSIIKPQQ; SIIKPQQY; IIKPQQYT; IKPQQYTF;
KPQQYTFS; PQQYTFSS; QQYTFSSS; QYTFSSSF; YTFSSSFS;
TFSSSFSQ; FSSSFSQP; SSSFSQPT; SSFSQPTS; SFSQPTSQ;
FSQPTSQT; SQPTSQTN; QPTSQTNF; PTSQTNFN; TSQTNFNN;
SQTNFNNS; QTNFNNSQ; TNFNNSQS; NFNNSQSN; FNNSQSNN;
NNSQSNNV; NSQSNNVL; SQSNNVLT; QSNNVLTN; SNNVLTNY;
NNVLTNYR; NVLTNYRH; VLTNYRHQ; LTNYRHQT; TNYRHQTQ;
NYRHQTQP; YRHQTQPS; RHQTQPSF; HQTQPSFV; QTQPSFVV;
TQPSFVVP; QPSFVVPV; PSFVVPVY; SFVVPVYS; FVVPVYSG;
VVPVYSGN; VPVYSGNS; PVYSGNSP; VYSGNSPL; YSGNSPLQ;
SGNSPLQK; GNSPLQKL; NSPLQKLK; SPLQKLKN; PLQKLKNN;
LQKLKNNL; QKLKNNLL; KLKNNLLR; LKNNLLRR; KNNLLRRI;
NNLLRRIA; NLLRRIAE; LLRRIAEE; LRRIAEEK; RRIAEEKN;
RIAEEKNK; IAEEKNKT; AEEKNKTH; EEKNKTHN; EKNKTHNH;
KNKTHNHG; NKTHNHGF; KTHNHGFR; THNHGFRE; HNHGFRET;
NHGFRETY; HGFRETYD; GFRETYDQ; FRETYDQF; RETYDQFK;
ETYDQFKM; TYDQFKMK; YDQFKMKD; DQFKMKDS; QFKMKDSA;
FKMKDSAF; KMKDSAFT; MKDSAFTL; KDSAFTLL; DSAFTLLD;
SAFTLLDV; AFTLLDVI; FTLLDVIS; TLLDVISN; LLDVISNI;
LDVISNIS; DVISNISV; VISNISVF; ISNISVFD; SNISVFDR;
NISVFDRG; ISVFDRGS; SVFDRGSA; VFDRGSAP; FDRGSAPQ;
DRGSAPQL; RGSAPQLS; GSAPQLSS; SAPQLSSN; APQLSSNT;
PQLSSNTP; QLSSNTPE; LSSNTPEA; SSNTPEAE; SNTPEAES;
NTPEAESE; TPEAESER; PEAESERN; EAESERNR; AESERNRL;
ESERNRLY; SERNRLYA; ERNRLYAM; RNRLYAMM; NRLYAMMD;
RLYAMMDF; LYAMMDFD; YAMMDFDQ; AMMDFDQA; MMDFDQAK;
MDFDQAKT; DFDQAKTT; FDQAKTTE; DQAKTTEF; QAKTTEFG;
AKTTEFGS; KTTEFGSI; TTEFGSIM; TEFGSIMN; EFGSIMNI;
FGSIMNIL; GSIMNILY; SIMNILYQ; IMNILYQE; MNILYQEN;
NILYQENQ; ILYQENQN; LYQENQNH; YQENQNHS; QENQNHSL;
ENQNHSLI; NQNHSLIR; QNHSLIRS; NHSLIRSL; HSLIRSLI;
SLIRSLII; LIRSLIIS; IRSLIISG; RSLIISGL; SLIISGLG;
LIISGLGI; IISGLGIQ; ISGLGIQI; SGLGIQIS; GLGIQISL;
LGIQISLE; GIQISLES; IQISLEST; QISLESTL; ISLESTLE;
SLESTLEE; LESTLEEI; ESTLEEIE; STLEEIEK; TLEEIEKK;
LEEIEKKI; EEIEKKIE; EIEKKIES; IEKKIESF; EKKIESFN;
KKIESFNT; KIESFNTQ; IESFNTQY; ESFNTQYL; SFNTQYLN;

FNTQYLNT; NTQYLNTI; TQYLNTII; QYLNTIIN; YLNTIINS;
LNTIINSY; NTIINSYT; TIINSYTF; IINSYTFK; INSYTFKD;
NSYTFKDK; SYTFKDKL; YTFKDKLK; TFKDKLKE; FKDKLKEL;
KDKLKELE; DKLKELES; KLKELESK; LKELESKL; KELESKLN;
ELESKLNS; LESKLNSI; ESKLNSIL; SKLNSILA; KLNSILAE;
LNSILAEK; NSILAEKK; SILAEKKE; ILAEKKEW; LAEKKEWL;
AEKKEWLN; EKKEWLNY; KKEWLNYA; KEWLNYAD; EWLNYADA;
WLNYADAI; LNYADAII; NYADAIIT; YADAIITN; ADAIITNT;
DAIITNTS; AIITNTSS; IITNTSSN; ITNTSSNS; TNTSSNSK;
NTSSNSKR; TSSNSKRN; SSNSKRND; SNSKRNDP; NSKRNDPQ;
SKRNDPQS; KRNDPQSL; RNDPQSLG; NDPQSLGQ; DPQSLGQY;
PQSLGQYI; QSLGQYIK; SLGQYIKN; LGQYIKNK; GQYIKNKY;
QYIKNKYL; YIKNKYLD; IKNKYLDK; KNKYLDKM; NKYLDKMQ;
KYLDKMQD; YLDKMQDA; LDKMQDAR; DKMQDARQ; KMQDARQS;
MQDARQSA; QDARQSAL; DARQSALD; ARQSALDL; RQSALDLY;
QSALDLYL; SALDLYLN; ALDLYLNI; LDLYLNIT; DLYLNITE;
LYLNITEI;

9 mers:
MSSCTIDAN; SSCTIDANL; SCTIDANLN; CTIDANLNK; TIDANLNKD;
IDANLNKDY; DANLNKDYK; ANLNKDYKN; NLNKDYKNK; LNKDYKNKV;
NKDYKNKVE; KDYKNKVEE; DYKNKVEEL; YKNKVEELL; KNKVEELLN;
NKVEELLNS; KVEELLNSS; VEELLNSST; EELLNSSTD; ELLNSSTDD;
LLNSSTDDQ; LNSSTDDQA; NSSTDDQAK; SSTDDQAKI; STDDQAKIS;
TDDQAKISI; DDQAKISIN; DQAKISINT; QAKISINTG; AKISINTGS;
KISINTGSN; ISINTGSNA; SINTGSNAT; INTGSNATK; NTGSNATKN;
TGSNATKNK; GSNATKNKT; SNATKNKTN; NATKNKTNI; ATKNKTNIK;
TKNKTNIKV; KNKTNIKVA; NKTNIKVAG; KTNIKVAGL; TNIKVAGLQ;
NIKVAGLQK; IKVAGLQKN; KVAGLQKNT; VAGLQKNTQ; AGLQKNTQS;
GLQKNTQSK; LQKNTQSKK; QKNTQSKKN; KNTQSKKNN; NTQSKKNNN;
TQSKKNNNL; QSKKNNNLQ; SKKNNNLQG; KKNNNLQGL; KNNNLQGLN;
NNNLQGLNP; NNLQGLNPA; NLQGLNPAN; LQGLNPANQ; QGLNPANQV;
GLNPANQVN; LNPANQVNP; NPANQVNPG; PANQVNPGN; ANQVNPGNP;
NQVNPGNPM; QVNPGNPMQ; VNPGNPMQI; NPGNPMQIA; PGNPMQIAN;
GNPMQIANQ; NPMQIANQA; PMQIANQAN; MQIANQANQ; QIANQANQA;
IANQANQAN; ANQANQANQ; NQANQANQA; QANQANQAN; ANQANQANQ;
NQANQANQA; QANQANQAN; ANQANQANQ; NQANQANQA; QANQANQAN;
ANQANQANQ; NQANQANQA; QANQANQAS; ANQANQASQ; NQANQASQA;
QANQASQAS; ANQASQASQ; NQASQASQA; QASQASQAS; ASQASQASQ;
SQASQASQV; QASQASQVA; ASQASQVAS; SQASQVASS; QASQVASSA;
ASQVASSAS; SQVASSASQ; QVASSASQA; VASSASQAS; ASSASQASP;
SSASQASPV; SASQASPVA; ASQASPVAS; SQASPVASP; QASPVASPA;
ASPVASPAT; SPVASPATN; PVASPATNV; VASPATNVQ; ASPATNVQA;
SPATNVQAT; PATNVQATP; ATNVQATPP; TNVQATPPK; NVQATPPKQ;
VQATPPKQI; QATPPKQIA; ATPPKQIAS; TPPKQIASA; PPKQIASAQ;
PKQIASAQA; KQIASAQAI; QIASAQAIQ; IASAQAIQT; ASAQAIQTV;
SAQAIQTVP; AQAIQTVPN; QAIQTVPNN; AIQTVPNNT; IQTVPNNTS;
QTVPNNTST; TVPNNTSTP; VPNNTSTPN; PNNTSTPNQ; NNTSTPNQS;
NTSTPNQSI; TSTPNQSII; STPNQSIIK; TPNQSIIKP; PNQSIIKPQ;
NQSIIKPQQ; QSIIKPQQY; SIIKPQQYT; IIKPQQYTF; IKPQQYTFS;
KPQQYTFSS; PQQYTFSSS; QQYTFSSSF; QYTFSSSFS; YTFSSSFSQ;

TFSSSFSQP; FSSSFSQPT; SSSFSQPTS; SSFSQPTSQ; SFSQPTSQT;
FSQPTSQTN; SQPTSQTNF; QPTSQTNFN; PTSQTNFNN; TSQTNFNNS;
SQTNFNNSQ; QTNFNNSQS; TNFNNSQSN; NFNNSQSNN; FNNSQSNNV;
NNSQSNNVL; NSQSNNVLT; SQSNNVLTN; QSNNVLTNY; SNNVLTNYR;
NNVLTNYRH; NVLTNYRHQ; VLTNYRHQT; LTNYRHQTQ; TNYRHQTQP;
NYRHQTQPS; YRHQTQPSF; RHQTQPSFV; HQTQPSFVV; QTQPSFVVP;
TQPSFVVPV; QPSFVVPVY; PSFVVPVYS; SFVVPVYSG; FVVPVYSGN;
VVPVYSGNS; VPVYSGNSP; PVYSGNSPL; VYSGNSPLQ; YSGNSPLQK;
SGNSPLQKL; GNSPLQKLK; NSPLQKLKN; SPLQKLKNN; PLQKLKNNL;
LQKLKNNLL; QKLKNNLLR; KLKNNLLRR; LKNNLLRRI; KNNLLRRIA;
NNLLRRIAE; NLLRRIAEE; LLRRIAEEK; LRRIAEEKN; RRIAEEKNK;
RIAEEKNKT; IAEEKNKTH; AEEKNKTHN; EEKNKTHNH; EKNKTHNHG;
KNKTHNHGF; NKTHNHGFR; KTHNHGFRE; THNHGFRET; HNHGFRETY;
NHGFRETYD; HGFRETYDQ; GFRETYDQF; FRETYDQFK; RETYDQFKM;
ETYDQFKMK; TYDQFKMKD; YDQFKMKDS; DQFKMKDSA; QFKMKDSAF;
FKMKDSAFT; KMKDSAFTL; MKDSAFTLL; KDSAFTLLD; DSAFTLLDV;
SAFTLLDVI; AFTLLDVIS; FTLLDVISN; TLLDVISNI; LLDVISNIS;
LDVISNISV; DVISNISVF; VISNISVFD; ISNISVFDR; SNISVFDRG;
NISVFDRGS; ISVFDRGSA; SVFDRGSAP; VFDRGSAPQ; FDRGSAPQL;
DRGSAPQLS; RGSAPQLSS; GSAPQLSSN; SAPQLSSNT; APQLSSNTP;
PQLSSNTPE; QLSSNTPEA; LSSNTPEAE; SSNTPEAES; SNTPEAESE;
NTPEAESER; TPEAESERN; PEAESERNR; EAESERNRL; AESERNRLY;
ESERNRLYA; SERNRLYAM; ERNRLYAMM; RNRLYAMMD; NRLYAMMDF;
RLYAMMDFD; LYAMMDFDQ; YAMMDFDQA; AMMDFDQAK; MMDFDQAKT;
MDFDQAKTT; DFDQAKTTE; FDQAKTTEF; DQAKTTEFG; QAKTTEFGS;
AKTTEFGSI; KTTEFGSIM; TTEFGSIMN; TEFGSIMNI; EFGSIMNIL;
FGSIMNILY; GSIMNILYQ; SIMNILYQE; IMNILYQEN; MNILYQENQ;
NILYQENQN; ILYQENQNH; LYQENQNHS; YQENQNHSL; QENQNHSLI;
ENQNHSLIR; NQNHSLIRS; QNHSLIRSL; NHSLIRSLI; HSLIRSLII;
SLIRSLIIS; LIRSLIISG; IRSLIISGL; RSLIISGLG; SLIISGLGI;
LIISGLGIQ; IISGLGIQI; ISGLGIQIS; SGLGIQISL; GLGIQISLE;
LGIQISLES; GIQISLEST; IQISLESTL; QISLESTLE; ISLESTLEE;
SLESTLEEI; LESTLEEIE; ESTLEEIEK; STLEEIEKK; TLEEIEKKI;
LEEIEKKIE; EEIEKKIES; EIEKKIESF; IEKKIESFN; EKKIESFNT;
KKIESFNTQ; KIESFNTQY; IESFNTQYL; ESFNTQYLN; SFNTQYLNT;
FNTQYLNTI; NTQYLNTII; TQYLNTIIN; QYLNTIINS; YLNTIINSY;
LNTIINSYT; NTIINSYTF; TIINSYTFK; IINSYTFKD; INSYTFKDK;
NSYTFKDKL; SYTFKDKLK; YTFKDKLKE; TFKDKLKEL; FKDKLKELE;
KDKLKELES; DKLKELESK; KLKELESKL; LKELESKLN; KELESKLNS;
ELESKLNSI; LESKLNSIL; ESKLNSILA; SKLNSILAE; KLNSILAEK;
LNSILAEKK; NSILAEKKE; SILAEKKEW; ILAEKKEWL; LAEKKEWLN;
AEKKEWLNY; EKKEWLNYA; KKEWLNYAD; KEWLNYADA; EWLNYADAI;
WLNYADAII; LNYADAIIT; NYADAIITN; YADAIITNT; ADAIITNTS;
DAIITNTSS; AIITNTSSN; IITNTSSNS; ITNTSSNSK; TNTSSNSKR;
NTSSNSKRN; TSSNSKRND; SSNSKRNDP; SNSKRNDPQ; NSKRNDPQS;
SKRNDPQSL; KRNDPQSLG; RNDPQSLGQ; NDPQSLGQY; DPQSLGQYI;
PQSLGQYIK; QSLGQYIKN; SLGQYIKNK; LGQYIKNKY; GQYIKNKYL;
QYIKNKYLD; YIKNKYLDK; IKNKYLDKM; KNKYLDKMQ; NKYLDKMQD;
KYLDKMQDA; YLDKMQDAR; LDKMQDARQ; DKMQDARQS; KMQDARQSA;
MQDARQSAL; QDARQSALD; DARQSALDL; ARQSALDLY; RQSALDLYL;
QSALDLYLN; SALDLYLNI; ALDLYLNIT; LDLYLNITE; DLYLNITEI;

10 mers:
MSSCTIDANL; SSCTIDANLN; SCTIDANLNK; CTIDANLNKD;
TIDANLNKDY; IDANLNKDYK; DANLNKDYKN; ANLNKDYKNK;
NLNKDYKNKV; LNKDYKNKVE; NKDYKNKVEE; KDYKNKVEEL;
DYKNKVEELL; YKNKVEELLN; KNKVEELLNS; NKVEELLNSS;
KVEELLNSST; VEELLNSSTD; EELLNSSTDD; ELLNSSTDDQ;
LLNSSTDDQA; LNSSTDDQAK; NSSTDDQAKI; SSTDDQAKIS;
STDDQAKISI; TDDQAKISIN; DDQAKISINT; DQAKISINTG;
QAKISINTGS; AKISINTGSN; KISINTGSNA; ISINTGSNAT;
SINTGSNATK; INTGSNATKN; NTGSNATKNK; TGSNATKNKT;
GSNATKNKTN; SNATKNKTNI; NATKNKTNIK; ATKNKTNIKV;
TKNKTNIKVA; KNKTNIKVAG; NKTNIKVAGL; KTNIKVAGLQ;
TNIKVAGLQK; NIKVAGLQKN; IKVAGLQKNT; KVAGLQKNTQ;
VAGLQKNTQS; AGLQKNTQSK; GLQKNTQSKK; LQKNTQSKKN;
QKNTQSKKNN; KNTQSKKNNN; NTQSKKNNNL; TQSKKNNNLQ;
QSKKNNNLQG; SKKNNNLQGL; KKNNNLQGLN; KNNNLQGLNP;
NNNLQGLNPA; NNLQGLNPAN; NLQGLNPANQ; LQGLNPANQV;
QGLNPANQVN; GLNPANQVNP; LNPANQVNPG; NPANQVNPGN;
PANQVNPGNP; ANQVNPGNPM; NQVNPGNPMQ; QVNPGNPMQI;
VNPGNPMQIA; NPGNPMQIAN; PGNPMQIANQ; GNPMQIANQA;
NPMQIANQAN; PMQIANQANQ; MQIANQANQA; QIANQANQAN;
IANQANQANQ; ANQANQANQA; NQANQANQAN; QANQANQANQ;
ANQANQANQA; NQANQANQAN; QANQANQANQ; ANQANQANQA;
NQANQANQAN; QANQANQANQ; ANQANQANQA; NQANQANQAS;
QANQANQASQ; ANQANQASQA; NQANQASQAS; QANQASQASQ;
ANQASQASQA; NQASQASQAS; QASQASQASQ; ASQASQASQV;
SQASQASQVA; QASQASQVAS; ASQASQVASS; SQASQVASSA;
QASQVASSAS; ASQVASSASQ; SQVASSASQA; QVASSASQAS;
VASSASQASP; ASSASQASPV; SSASQASPVA; SASQASPVAS;
ASQASPVASP; SQASPVASPA; QASPVASPAT; ASPVASPATN;
SPVASPATNV; PVASPATNVQ; VASPATNVQA; ASPATNVQAT;
SPATNVQATP; PATNVQATPP; ATNVQATPPK; TNVQATPPKQ;
NVQATPPKQI; VQATPPKQIA; QATPPKQIAS; ATPPKQIASA;
TPPKQIASAQ; PPKQIASAQA; PKQIASAQAI; KQIASAQAIQ;
QIASAQAIQT; IASAQAIQTV; ASAQAIQTVP; SAQAIQTVPN;
AQAIQTVPNN; QAIQTVPNNT; AIQTVPNNTS; IQTVPNNTST;
QTVPNNTSTP; TVPNNTSTPN; VPNNTSTPNQ; PNNTSTPNQS;
NNTSTPNQSI; NTSTPNQSII; TSTPNQSIIK; STPNQSIIKP;
TPNQSIIKPQ; PNQSIIKPQQ; NQSIIKPQQY; QSIIKPQQYT;
SIIKPQQYTF; IIKPQQYTFS; IKPQQYTFSS; KPQQYTFSSS;
PQQYTFSSSF; QQYTFSSSFS; QYTFSSSFSQ; YTFSSSFSQP;
TFSSSFSQPT; FSSSFSQPTS; SSSFSQPTSQ; SSFSQPTSQT;
SFSQPTSQTN; FSQPTSQTNF; SQPTSQTNFN; QPTSQTNFNN;
PTSQTNFNNS; TSQTNFNNSQ; SQTNFNNSQS; QTNFNNSQSN;
TNFNNSQSNN; NFNNSQSNNV; FNNSQSNNVL; NNSQSNNVLT;
NSQSNNVLTN; SQSNNVLTNY; QSNNVLTNYR; SNNVLTNYRH;
NNVLTNYRHQ; NVLTNYRHQT; VLTNYRHQTQ; LTNYRHQTQP;
TNYRHQTQPS; NYRHQTQPSF; YRHQTQPSFV; RHQTQPSFVV;
HQTQPSFVVP; QTQPSFVVPV; TQPSFVVPVY; QPSFVVPVYS;
PSFVVPVYSG; SFVVPVYSGN; FVVPVYSGNS; VVPVYSGNSP;

| | | | |
|---|---|---|---|
| VPVYSGNSPL; | PVYSGNSPLQ; | VYSGNSPLQK; | YSGNSPLQKL; |
| SGNSPLQKLK; | GNSPLQKLKN; | NSPLQKLKNN; | SPLQKLKNNL; |
| PLQKLKNNLL; | LQKLKNNLLR; | QKLKNNLLRR; | KLKNNLLRRI; |
| LKNNLLRRIA; | KNNLLRRIAE; | NNLLRRIAEE; | NLLRRIAEEK; |
| LLRRIAEEKN; | LRRIAEEKNK; | RRIAEEKNKT; | RIAEEKNKTH; |
| IAEEKNKTHN; | AEEKNKTHNH; | EEKNKTHNHG; | EKNKTHNHGF; |
| KNKTHNHGFR; | NKTHNHGFRE; | KTHNHGFRET; | THNHGFRETY; |
| HNHGFRETYD; | NHGFRETYDQ; | HGFRETYDQF; | GFRETYDQFK; |
| FRETYDQFKM; | RETYDQFKMK; | ETYDQFKMKD; | TYDQFKMKDS; |
| YDQFKMKDSA; | DQFKMKDSAF; | QFKMKDSAFT; | FKMKDSAFTL; |
| KMKDSAFTLL; | MKDSAFTLLD; | KDSAFTLLDV; | DSAFTLLDVI; |
| SAFTLLDVIS; | AFTLLDVISN; | FTLLDVISNI; | TLLDVISNIS; |
| LLDVISNISV; | LDVISNISVF; | DVISNISVFD; | VISNISVFDR; |
| ISNISVFDRG; | SNISVFDRGS; | NISVFDRGSA; | ISVFDRGSAP; |
| SVFDRGSAPQ; | VFDRGSAPQL; | FDRGSAPQLS; | DRGSAPQLSS; |
| RGSAPQLSSN; | GSAPQLSSNT; | SAPQLSSNTP; | APQLSSNTPE; |
| PQLSSNTPEA; | QLSSNTPEAE; | LSSNTPEAES; | SSNTPEAESE; |
| SNTPEAESER; | NTPEAESERN; | TPEAESERNR; | PEAESERNRL; |
| EAESERNRLY; | AESERNRLYA; | ESERNRLYAM; | SERNRLYAMM; |
| ERNRLYAMMD; | RNRLYAMMDF; | NRLYAMMDFD; | RLYAMMDFDQ; |
| LYAMMDFDQA; | YAMMDFDQAK; | AMMDFDQAKT; | MMDFDQAKTT; |
| MDFDQAKTTE; | DFDQAKTTEF; | FDQAKTTEFG; | DQAKTTEFGS; |
| QAKTTEFGSI; | AKTTEFGSIM; | KTTEFGSIMN; | TTEFGSIMNI; |
| TEFGSIMNIL; | EFGSIMNILY; | FGSIMNILYQ; | GSIMNILYQE; |
| SIMNILYQEN; | IMNILYQENQ; | MNILYQENQN; | NILYQENQNH; |
| ILYQENQNHS; | LYQENQNHSL; | YQENQNHSLI; | QENQNHSLIR; |
| ENQNHSLIRS; | NQNHSLIRSL; | QNHSLIRSLI; | NHSLIRSLII; |
| HSLIRSLIIS; | SLIRSLIISG; | LIRSLIISGL; | IRSLIISGLG; |
| RSLIISGLGI; | SLIISGLGIQ; | LIISGLGIQI; | IISGLGIQIS; |
| ISGLGIQISL; | SGLGIQISLE; | GLGIQISLES; | LGIQISLEST; |
| GIQISLESTL; | IQISLESTLE; | QISLESTLEE; | ISLESTLEEI; |
| SLESTLEEIE; | LESTLEEIEK; | ESTLEEIEKK; | STLEEIEKKI; |
| TLEEIEKKIE; | LEEIEKKIES; | EEIEKKIESF; | EIEKKIESFN; |
| IEKKIESFNT; | EKKIESFNTQ; | KKIESFNTQY; | KIESFNTQYL; |
| IESFNTQYLN; | ESFNTQYLNT; | SFNTQYLNTI; | FNTQYLNTII; |
| NTQYLNTIIN; | TQYLNTIINS; | QYLNTIINSY; | YLNTIINSYT; |
| LNTIINSYTF; | NTIINSYTFK; | TIINSYTFKD; | IINSYTFKDK; |
| INSYTFKDKL; | NSYTFKDKLK; | SYTFKDKLKE; | YTFKDKLKEL; |
| TFKDKLKELE; | FKDKLKELES; | KDKLKELESK; | DKLKELESKL; |
| KLKELESKLN; | LKELESKLNS; | KELESKLNSI; | ELESKLNSIL; |
| LESKLNSILA; | ESKLNSILAE; | SKLNSILAEK; | KLNSILAEKK; |
| LNSILAEKKE; | NSILAEKKEW; | SILAEKKEWL; | ILAEKKEWLN; |
| LAEKKEWLNY; | AEKKEWLNYA; | EKKEWLNYAD; | KKEWLNYADA; |
| KEWLNYADAI; | EWLNYADAII; | WLNYADAIIT; | LNYADAIITN; |
| NYADAIITNT; | YADAIITNTS; | ADAIITNTSS; | DAIITNTSSN; |
| AIITNTSSNS; | IITNTSSNSK; | ITNTSSNSKR; | TNTSSNSKRN; |
| NTSSNSKRND; | TSSNSKRNDP; | SSNSKRNDPQ; | SNSKRNDPQS; |
| NSKRNDPQSL; | SKRNDPQSLG; | KRNDPQSLGQ; | RNDPQSLGQY; |
| NDPQSLGQYI; | DPQSLGQYIK; | PQSLGQYIKN; | QSLGQYIKNK; |
| SLGQYIKNKY; | LGQYIKNKYL; | GQYIKNKYLD; | QYIKNKYLDK; |
| YIKNKYLDKM; | IKNKYLDKMQ; | KNKYLDKMQD; | NKYLDKMQDA; |

KYLDKMQDAR; YLDKMQDARQ; LDKMQDARQS; DKMQDARQSA;
KMQDARQSAL; MQDARQSALD; QDARQSALDL; DARQSALDLY;
ARQSALDLYL; RQSALDLYLN; QSALDLYLNI; SALDLYLNIT;
ALDLYLNITE; LDLYLNITEI; DLYLNITEIR;

11 mers:
MSSCTIDANLN; SSCTIDANLNK; SCTIDANLNKD; CTIDANLNKDY;
TIDANLNKDYK; IDANLNKDYKN; DANLNKDYKNK; ANLNKDYKNKV;
NLNKDYKNKVE; LNKDYKNKVEE; NKDYKNKVEEL; KDYKNKVEELL;
DYKNKVEELLN; YKNKVEELLNS; KNKVEELLNSS; NKVEELLNSST;
KVEELLNSSTD; VEELLNSSTDD; EELLNSSTDDQ; ELLNSSTDDQA;
LLNSSTDDQAK; LNSSTDDQAKI; NSSTDDQAKIS; SSTDDQAKISI;
STDDQAKISIN; TDDQAKISINT; DDQAKISINTG; DQAKISINTGS;
QAKISINTGSN; AKISINTGSNA; KISINTGSNAT; ISINTGSNATK;
SINTGSNATKN; INTGSNATKNK; NTGSNATKNKT; TGSNATKNKTN;
GSNATKNKTNI; SNATKNKTNIK; NATKNKTNIKV; ATKNKTNIKVA;
TKNKTNIKVAG; KNKTNIKVAGL; NKTNIKVAGLQ; KTNIKVAGLQK;
TNIKVAGLQKN; NIKVAGLQKNT; IKVAGLQKNTQ; KVAGLQKNTQS;
VAGLQKNTQSK; AGLQKNTQSKK; GLQKNTQSKKN; LQKNTQSKKNN;
QKNTQSKKNNN; KNTQSKKNNNL; NTQSKKNNNLQ; TQSKKNNNLQG;
QSKKNNNLQGL; SKKNNNLQGLN; KKNNNLQGLNP; KNNNLQGLNPA;
NNNLQGLNPAN; NNLQGLNPANQ; NLQGLNPANQV; LQGLNPANQVN;
QGLNPANQVNP; GLNPANQVNPG; LNPANQVNPGN; NPANQVNPGNP;
PANQVNPGNPM; ANQVNPGNPMQ; NQVNPGNPMQI; QVNPGNPMQIA;
VNPGNPMQIAN; NPGNPMQIANQ; PGNPMQIANQA; GNPMQIANQAN;
NPMQIANQANQ; PMQIANQANQA; MQIANQANQAN; QIANQANQANQ;
IANQANQANQA; ANQANQANQAN; NQANQANQANQ; QANQANQANQA;
ANQANQANQAN; NQANQANQANQ; QANQANQANQA; ANQANQANQAN;
NQANQANQANQ; QANQANQANQA; ANQANQANQAS; NQANQANQASQ;
QANQANQASQA; ANQANQASQAS; NQANQASQASQ; QANQASQASQA;
ANQASQASQAS; NQASQASQASQ; QASQASQASQV; ASQASQASQVA;
SQASQASQVAS; QASQASQVASS; ASQASQVASSA; SQASQVASSAS;
QASQVASSASQ; ASQVASSASQA; SQVASSASQAS; QVASSASQASP;
VASSASQASPV; ASSASQASPVA; SSASQASPVAS; SASQASPVASP;
ASQASPVASPA; SQASPVASPAT; QASPVASPATN; ASPVASPATNV;
SPVASPATNVQ; PVASPATNVQA; VASPATNVQAT; ASPATNVQATP;
SPATNVQATPP; PATNVQATPPK; ATNVQATPPKQ; TNVQATPPKQI;
NVQATPPKQIA; VQATPPKQIAS; QATPPKQIASA; ATPPKQIASAQ;
TPPKQIASAQA; PPKQIASAQAI; PKQIASAQAIQ; KQIASAQAIQT;
QIASAQAIQTV; IASAQAIQTVP; ASAQAIQTVPN; SAQAIQTVPNN;
AQAIQTVPNNT; QAIQTVPNNTS; AIQTVPNNTST; IQTVPNNTSTP;
QTVPNNTSTPN; TVPNNTSTPNQ; VPNNTSTPNQS; PNNTSTPNQSI;
NNTSTPNQSII; NTSTPNQSIIK; TSTPNQSIIKP; STPNQSIIKPQ;
TPNQSIIKPQQ; PNQSIIKPQQY; NQSIIKPQQYT; QSIIKPQQYTF;
SIIKPQQYTFS; IIKPQQYTFSS; IKPQQYTFSSS; KPQQYTFSSSF;
PQQYTFSSSFS; QQYTFSSSFSQ; QYTFSSSFSQP; YTFSSSFSQPT;
TFSSSFSQPTS; FSSSFSQPTSQ; SSSFSQPTSQT; SSFSQPTSQTN;
SFSQPTSQTNF; FSQPTSQTNFN; SQPTSQTNFNN; QPTSQTNFNNS;
PTSQTNFNNSQ; TSQTNFNNSQS; SQTNFNNSQSN; QTNFNNSQSNN;
TNFNNSQSNNV; NFNNSQSNNVL; FNNSQSNNVLT; NNSQSNNVLTN;
NSQSNNVLTNY; SQSNNVLTNYR; QSNNVLTNYRH; SNNVLTNYRHQ;

NNVLTNYRHQT; NVLTNYRHQTQ; VLTNYRHQTQP; LTNYRHQTQPS;
TNYRHQTQPSF; NYRHQTQPSFV; YRHQTQPSFVV; RHQTQPSFVVP;
HQTQPSFVVPV; QTQPSFVVPVY; TQPSFVVPVYS; QPSFVVPVYSG;
PSFVVPVYSGN; SFVVPVYSGNS; FVVPVYSGNSP; VVPVYSGNSPL;
VPVYSGNSPLQ; PVYSGNSPLQK; VYSGNSPLQKL; YSGNSPLQKLK;
SGNSPLQKLKN; GNSPLQKLKNN; NSPLQKLKNNL; SPLQKLKNNLL;
PLQKLKNNLLR; LQKLKNNLLRR; QKLKNNLLRRI; KLKNNLLRRIA;
LKNNLLRRIAE; KNNLLRRIAEE; NNLLRRIAEEK; NLLRRIAEEKN;
LLRRIAEEKNK; LRRIAEEKNKT; RRIAEEKNKTH; RIAEEKNKTHN;
IAEEKNKTHNH; AEEKNKTHNHG; EEKNKTHNHGF; EKNKTHNHGFR;
KNKTHNHGFRE; NKTHNHGFRET; KTHNHGFRETY; THNHGFRETYD;
HNHGFRETYDQ; NHGFRETYDQF; HGFRETYDQFK; GFRETYDQFKM;
FRETYDQFKMK; RETYDQFKMKD; ETYDQFKMKDS; TYDQFKMKDSA;
YDQFKMKDSAF; DQFKMKDSAFT; QFKMKDSAFTL; FKMKDSAFTLL;
KMKDSAFTLLD; MKDSAFTLLDV; KDSAFTLLDVI; DSAFTLLDVIS;
SAFTLLDVISN; AFTLLDVISNI; FTLLDVISNIS; TLLDVISNISV;
LLDVISNISVF; LDVISNISVFD; DVISNISVFDR; VISNISVFDRG;
ISNISVFDRGS; SNISVFDRGSA; NISVFDRGSAP; ISVFDRGSAPQ;
SVFDRGSAPQL; VFDRGSAPQLS; FDRGSAPQLSS; DRGSAPQLSSN;
RGSAPQLSSNT; GSAPQLSSNTP; SAPQLSSNTPE; APQLSSNTPEA;
PQLSSNTPEAE; QLSSNTPEAES; LSSNTPEAESE; SSNTPEAESER;
SNTPEAESERN; NTPEAESERNR; TPEAESERNRL; PEAESERNRLY;
EAESERNRLYA; AESERNRLYAM; ESERNRLYAMM; SERNRLYAMMD;
ERNRLYAMMDF; RNRLYAMMDFD; NRLYAMMDFDQ; RLYAMMDFDQA;
LYAMMDFDQAK; YAMMDFDQAKT; AMMDFDQAKTT; MMDFDQAKTTE;
MDFDQAKTTEF; DFDQAKTTEFG; FDQAKTTEFGS; DQAKTTEFGSI;
QAKTTEFGSIM; AKTTEFGSIMN; KTTEFGSIMNI; TTEFGSIMNIL;
TEFGSIMNILY; EFGSIMNILYQ; FGSIMNILYQE; GSIMNILYQEN;
SIMNILYQENQ; IMNILYQENQN; MNILYQENQNH; NILYQENQNHS;
ILYQENQNHSL; LYQENQNHSLI; YQENQNHSLIR; QENQNHSLIRS;
ENQNHSLIRSL; NQNHSLIRSLI; QNHSLIRSLII; NHSLIRSLIIS;
HSLIRSLIISG; SLIRSLIISGL; LIRSLIISGLG; IRSLIISGLGI;
RSLIISGLGIQ; SLIISGLGIQI; LIISGLGIQIS; IISGLGIQISL;
ISGLGIQISLE; SGLGIQISLES; GLGIQISLEST; LGIQISLESTL;
GIQISLESTLE; IQISLESTLEE; QISLESTLEEI; ISLESTLEEIE;
SLESTLEEIEK; LESTLEEIEKK; ESTLEEIEKKI; STLEEIEKKIE;
TLEEIEKKIES; LEEIEKKIESF; EEIEKKIESFN; EIEKKIESFNT;
IEKKIESFNTQ; EKKIESFNTQY; KKIESFNTQYL; KIESFNTQYLN;
IESFNTQYLNT; ESFNTQYLNTI; SFNTQYLNTII; FNTQYLNTIIN;
NTQYLNTIINS; TQYLNTIINSY; QYLNTIINSYT; YLNTIINSYTF;
LNTIINSYTFK; NTIINSYTFKD; TIINSYTFKDK; IINSYTFKDKL;
INSYTFKDKLK; NSYTFKDKLKE; SYTFKDKLKEL; YTFKDKLKELE;
TFKDKLKELES; FKDKLKELESK; KDKLKELESKL; DKLKELESKLN;
KLKELESKLNS; LKELESKLNSI; KELESKLNSIL; ELESKLNSILA;
LESKLNSILAE; ESKLNSILAEK; SKLNSILAEKK; KLNSILAEKKE;
LNSILAEKKEW; NSILAEKKEWL; SILAEKKEWLN; ILAEKKEWLNY;
LAEKKEWLNYA; AEKKEWLNYAD; EKKEWLNYADA; KKEWLNYADAI;
KEWLNYADAII; EWLNYADAIIT; WLNYADAIITN; LNYADAIITNT;
NYADAIITNTS; YADAIITNTSS; ADAIITNTSSN; DAIITNTSSNS;
AIITNTSSNSK; IITNTSSNSKR; ITNTSSNSKRN; TNTSSNSKRND;
NTSSNSKRNDP; TSSNSKRNDPQ; SSNSKRNDPQS; SNSKRNDPQSL;

| | |
|---|---|
| | NSKRNDPQSLG; SKRNDPQSLGQ; KRNDPQSLGQY; RNDPQSLGQYI; NDPQSLGQYIK; DPQSLGQYIKN; PQSLGQYIKNK; QSLGQYIKNKY; SLGQYIKNKYL; LGQYIKNKYLD; GQYIKNKYLDK; QYIKNKYLDKM; YIKNKYLDKMQ; IKNKYLDKMQD; KNKYLDKMQDA; NKYLDKMQDAR; KYLDKMQDARQ; YLDKMQDARQS; LDKMQDARQSA; DKMQDARQSAL; KMQDARQSALD; MQDARQSALDL; QDARQSALDLY; DARQSALDLYL; ARQSALDLYLN; RQSALDLYLNI; QSALDLYLNIT; SALDLYLNITE; ALDLYLNITEI; LDLYLNITEIR; |
| Seq 36 | SEQ ID NO 43881–44898/ <br> 8 mers: <br> MKKAKLNI; KKAKLNII; KAKLNIIK; AKLNIIKI; KLNIIKIN; LNIIKINI; NIIKINII; IIKINIIT; IKINIITM; KINIITMI; INIITMIL; NIITMILT; IITMILTL; ITMILTLI; TMILTLIC; MILTLICI; ILTLICIS; LTLICISC; TLICISCA; LICISCAP; ICISCAPF; CISCAPFN; ISCAPFNK; SCAPFNKI; CAPFNKIN; APFNKINP; PFNKINPK; FNKINPKA; NKINPKAN; KINPKANE; INPKANEN; NPKANENT; PKANENTK; KANENTKL; ANENTKLK; NENTKLKK; ENTKLKKN; NTKLKKNT; TKLKKNTR; KLKKNTRL; LKKNTRLK; KKNTRLKK; KNTRLKKP; NTRLKKPA; TRLKKPAN; RLKKPANP; LKKPANPG; KKPANPGE; KPANPGEN; PANPGENI; ANPGENIQ; NPGENIQN; PGENIQNF; GENIQNFK; ENIQNFKD; NIQNFKDK; IQNFKDKS; QNFKDKSG; NFKDKSGD; FKDKSGDL; KDKSGDLG; DKSGDLGA; KSGDLGAS; SGDLGASD; GDLGASDE; DLGASDEK; LGASDEKF; GASDEKFM; ASDEKFMG; SDEKFMGT; DEKFMGTT; EKFMGTTA; KFMGTTAS; FMGTTASE; MGTTASEL; GTTASELK; TTASELKA; TASELKAI; ASELKAIG; SELKAIGK; ELKAIGKE; LKAIGKEL; KAIGKELE; AIGKELED; IGKELEDR; GKELEDRK; KELEDRKN; ELEDRKNQ; LEDRKNQY; EDRKNQYD; DRKNQYDI; RKNQYDIQ; KNQYDIQI; NQYDIQIA; QYDIQIAK; YDIQIAKI; DIQIAKIT; IQIAKITN; QIAKITNE; IAKITNEE; AKITNEES; KITNEESN; ITNEESNL; TNEESNLL; NEESNLLD; EESNLLDT; ESNLLDTY; SNLLDTYI; NLLDTYIR; LLDTYIRA; LDTYIRAY; DTYIRAYE; TYIRAYEL; YIRAYELA; IRAYELAN; RAYELANE; AYELANEN; YELANENE; ELANENEK; LANENEKM; ANENEKML; NENEKMLL; ENEKMLLK; NEKMLLKR; EKMLLKRF; KMLLKRFL; MLLKRFLL; LLKRFLLS; LKRFLLSS; KRFLLSSL; RFLLSSLD; FLLSSLDY; LLSSLDYK; LSSLDYKK; SSLDYKKE; SLDYKKEN; LDYKKENI; DYKKENIE; YKKENIET; KKENIETL; KENIETLK; ENIETLKE; NIETLKEI; IETLKEIL; ETLKEILE; TLKEILEK; LKEILEKL; KEILEKLI; EILEKLIN; ILEKLINN; LEKLINNY; EKLINNYE; KLINNYEN; LINNYEND; INNYENDP; NNYENDPK; NYENDPKI; YENDPKIA; ENDPKIAA; NDPKIAAN; DPKIAANF; PKIAANFL; KIAANFLY; IAANFLYR; AANFLYRI; ANFLYRIA; NFLYRIAL; FLYRIALD; LYRIALDI; YRIALDIQ; RIALDIQL; IALDIQLK; ALDIQLKL; LDIQLKLE; DIQLKLEK; IQLKLEKH; QLKLEKHL; LKLEKHLK; KLEKHLKS; LEKHLKSI; EKHLKSIN; KHLKSINE; HLKSINEK; LKSINEKL; KSINEKLD; SINEKLDT; INEKLDTL; NEKLDTLS; EKLDTLSK; KLDTLSKE; LDTLSKEN; DTLSKENS; TLSKENSK; LSKENSKE; SKENSKED; KENSKEDL; ENSKEDLE; NSKEDLEA; SKEDLEAL; KEDLEALL; |

EDLEALLE; DLEALLEQ; LEALLEQV; EALLEQVK; ALLEQVKS;
LLEQVKSA; LEQVKSAL; EQVKSALQ; QVKSALQL; VKSALQLQ;
KSALQLQE; SALQLQEK; ALQLQEKF; LQLQEKFK; QLQEKFKK;
LQEKFKKT; QEKFKKTL; EKFKKTLN; KFKKTLNK; FKKTLNKT;
KKTLNKTL; KTLNKTLE; TLNKTLED; LNKTLEDY; NKTLEDYR;
KTLEDYRK; TLEDYRKN; LEDYRKNT; EDYRKNTN; DYRKNTNN;
YRKNTNNI; RKNTNNIQ; KNTNNIQE; NTNNIQEN; TNNIQENK;
NNIQENKV; NIQENKVL; IQENKVLA; QENKVLAE; ENKVLAEH;
NKVLAEHF; KVLAEHFN; VLAEHFNK; LAEHFNKY; AEHFNKYY;
EHFNKYYK; HFNKYYKD; FNKYYKDS; NKYYKDSD; KYYKDSDS;
YYKDSDSL; YKDSDSLQ; KDSDSLQS; DSDSLQSA; SDSLQSAF;
DSLQSAFY;

9 mers:
MKKAKLNII; KKAKLNIIK; KAKLNIIKI; AKLNIIKIN; KLNIIKINI;
LNIIKINII; NIIKINIIT; IIKINIITM; IKINIITMI; KINIITMIL;
INIITMILT; NIITMILTL; IITMILTLI; ITMILTLIC; TMILTLICI;
MILTLICIS; ILTLICISC; LTLICISCA; TLICISCAP; LICISCAPF;
ICISCAPFN; CISCAPFNK; ISCAPFNKI; SCAPFNKIN; CAPFNKINP;
APFNKINPK; PFNKINPKA; FNKINPKAN; NKINPKANE; KINPKANEN;
INPKANENT; NPKANENTK; PKANENTKL; KANENTKLK; ANENTKLKK;
NENTKLKKN; ENTKLKKNT; NTKLKKNTR; TKLKKNTRL; KLKKNTRLK;
LKKNTRLKK; KKNTRLKKP; KNTRLKKPA; NTRLKKPAN; TRLKKPANP;
RLKKPANPG; LKKPANPGE; KKPANPGEN; KPANPGENI; PANPGENIQ;
ANPGENIQN; NPGENIQNF; PGENIQNFK; GENIQNFKD; ENIQNFKDK;
NIQNFKDKS; IQNFKDKSG; QNFKDKSGD; NFKDKSGDL; FKDKSGDLG;
KDKSGDLGA; DKSGDLGAS; KSGDLGASD; SGDLGASDE; GDLGASDEK;
DLGASDEKF; LGASDEKFM; GASDEKFMG; ASDEKFMGT; SDEKFMGTT;
DEKFMGTTA; EKFMGTTAS; KFMGTTASE; FMGTTASEL; MGTTASELK;
GTTASELKA; TTASELKAI; TASELKAIG; ASELKAIGK; SELKAIGKE;
ELKAIGKEL; LKAIGKELE; KAIGKELED; AIGKELEDR; IGKELEDRK;
GKELEDRKN; KELEDRKNQ; ELEDRKNQY; LEDRKNQYD; EDRKNQYDI;
DRKNQYDIQ; RKNQYDIQI; KNQYDIQIA; NQYDIQIAK; QYDIQIAKI;
YDIQIAKIT; DIQIAKITN; IQIAKITNE; QIAKITNEE; IAKITNEES;
AKITNEESN; KITNEESNL; ITNEESNLL; TNEESNLLD; NEESNLLDT;
EESNLLDTY; ESNLLDTYI; SNLLDTYIR; NLLDTYIRA; LLDTYIRAY;
LDTYIRAYE; DTYIRAYEL; TYIRAYELA; YIRAYELAN; IRAYELANE;
RAYELANEN; AYELANENE; YELANENEK; ELANENEKM; LANENEKML;
ANENEKMLL; NENEKMLLK; ENEKMLLKR; NEKMLLKRF; EKMLLKRFL;
KMLLKRFLL; MLLKRFLLS; LLKRFLLSS; LKRFLLSSL; KRFLLSSLD;
RFLLSSLDY; FLLSSLDYK; LLSSLDYKK; LSSLDYKKE; SSLDYKKEN;
SLDYKKENI; LDYKKENIE; DYKKENIET; YKKENIETL; KKENIETLK;
KENIETLKE; ENIETLKEI; NIETLKEIL; IETLKEILE; ETLKEILEK;
TLKEILEKL; LKEILEKLI; KEILEKLIN; EILEKLINN; ILEKLINNY;
LEKLINNYE; EKLINNYEN; KLINNYEND; LINNYENDP; INNYENDPK;
NNYENDPKI; NYENDPKIA; YENDPKIAA; ENDPKIAAN; NDPKIAANF;
DPKIAANFL; PKIAANFLY; KIAANFLYR; IAANFLYRI; AANFLYRIA;
ANFLYRIAL; NFLYRIALD; FLYRIALDI; LYRIALDIQ; YRIALDIQL;
RIALDIQLK; IALDIQLKL; ALDIQLKLE; LDIQLKLEK; DIQLKLEKH;
IQLKLEKHL; QLKLEKHLK; LKLEKHLKS; KLEKHLKSI; LEKHLKSIN;
EKHLKSINE; KHLKSINEK; HLKSINEKL; LKSINEKLD; KSINEKLDT;

SINEKLDTL; INEKLDTLS; NEKLDTLSK; EKLDTLSKE; KLDTLSKEN;
LDTLSKENS; DTLSKENSK; TLSKENSKE; LSKENSKED; SKENSKEDL;
KENSKEDLE; ENSKEDLEA; NSKEDLEAL; SKEDLEALL; KEDLEALLE;
EDLEALLEQ; DLEALLEQV; LEALLEQVK; EALLEQVKS; ALLEQVKSA;
LLEQVKSAL; LEQVKSALQ; EQVKSALQL; QVKSALQLQ; VKSALQLQE;
KSALQLQEK; SALQLQEKF; ALQLQEKFK; LQLQEKFKK; QLQEKFKKT;
LQEKFKKTL; QEKFKKTLN; EKFKKTLNK; KFKKTLNKT; FKKTLNKTL;
KKTLNKTLE; KTLNKTLED; TLNKTLEDY; LNKTLEDYR; NKTLEDYRK;
KTLEDYRKN; TLEDYRKNT; LEDYRKNTN; EDYRKNTNN; DYRKNTNNI;
YRKNTNNIQ; RKNTNNIQE; KNTNNIQEN; NTNNIQENK; TNNIQENKV;
NNIQENKVL; NIQENKVLA; IQENKVLAE; QENKVLAEH; ENKVLAEHF;
NKVLAEHFN; KVLAEHFNK; VLAEHFNKY; LAEHFNKYY; AEHFNKYYK;
EHFNKYYKD; HFNKYYKDS; FNKYYKDSD; NKYYKDSDS; KYYKDSDSL;
YYKDSDSLQ; YKDSDSLQS; KDSDSLQSA; DSDSLQSAF; SDSLQSAFY;

10 mers:
MKKAKLNIIK; KKAKLNIIKI; KAKLNIIKIN; AKLNIIKINI;
KLNIIKINII; LNIIKINIIT; NIIKINIITM; IIKINIITMI;
IKINIITMIL; KINIITMILT; INIITMILTL; NIITMILTLI;
IITMILTLIC; ITMILTLICI; TMILTLICIS; MILTLICISC;
ILTLICISCA; LTLICISCAP; TLICISCAPF; LICISCAPFN;
ICISCAPFNK; CISCAPFNKI; ISCAPFNKIN; SCAPFNKINP;
CAPFNKINPK; APFNKINPKA; PFNKINPKAN; FNKINPKANE;
NKINPKANEN; KINPKANENT; INPKANENTK; NPKANENTKL;
PKANENTKLK; KANENTKLKK; ANENTKLKKN; NENTKLKKNT;
ENTKLKKNTR; NTKLKKNTRL; TKLKKNTRLK; KLKKNTRLKK;
LKKNTRLKKP; KKNTRLKKPA; KNTRLKKPAN; NTRLKKPANP;
TRLKKPANPG; RLKKPANPGE; LKKPANPGEN; KKPANPGENI;
KPANPGENIQ; PANPGENIQN; ANPGENIQNF; NPGENIQNFK;
PGENIQNFKD; GENIQNFKDK; ENIQNFKDKS; NIQNFKDKSG;
IQNFKDKSGD; QNFKDKSGDL; NFKDKSGDLG; FKDKSGDLGA;
KDKSGDLGAS; DKSGDLGASD; KSGDLGASDE; SGDLGASDEK;
GDLGASDEKF; DLGASDEKFM; LGASDEKFMG; GASDEKFMGT;
ASDEKFMGTT; SDEKFMGTTA; DEKFMGTTAS; EKFMGTTASE;
KFMGTTASEL; FMGTTASELK; MGTTASELKA; GTTASELKAI;
TTASELKAIG; TASELKAIGK; ASELKAIGKE; SELKAIGKEL;
ELKAIGKELE; LKAIGKELED; KAIGKELEDR; AIGKELEDRK;
IGKELEDRKN; GKELEDRKNQ; KELEDRKNQY; ELEDRKNQYD;
LEDRKNQYDI; EDRKNQYDIQ; DRKNQYDIQI; RKNQYDIQIA;
KNQYDIQIAK; NQYDIQIAKI; QYDIQIAKIT; YDIQIAKITN;
DIQIAKITNE; IQIAKITNEE; QIAKITNEES; IAKITNEESN;
AKITNEESNL; KITNEESNLL; ITNEESNLLD; TNEESNLLDT;
NEESNLLDTY; EESNLLDTYI; ESNLLDTYIR; SNLLDTYIRA;
NLLDTYIRAY; LLDTYIRAYE; LDTYIRAYEL; DTYIRAYELA;
TYIRAYELAN; YIRAYELANE; IRAYELANEN; RAYELANENE;
AYELANENEK; YELANENEKM; ELANENEKML; LANENEKMLL;
ANENEKMLLK; NENEKMLLKR; ENEKMLLKRF; NEKMLLKRFL;
EKMLLKRFLL; KMLLKRFLLS; MLLKRFLLSS; LLKRFLLSSL;
LKRFLLSSLD; KRFLLSSLDY; RFLLSSLDYK; FLLSSLDYKK;
LLSSLDYKKE; LSSLDYKKEN; SSLDYKKENI; SLDYKKENIE;
LDYKKENIET; DYKKENIETL; YKKENIETLK; KKENIETLKE;

KENIETLKEI; ENIETLKEIL; NIETLKEILE; IETLKEILEK;
ETLKEILEKL; TLKEILEKLI; LKEILEKLIN; KEILEKLINN;
EILEKLINNY; ILEKLINNYE; LEKLINNYEN; EKLINNYEND;
KLINNYENDP; LINNYENDPK; INNYENDPKI; NNYENDPKIA;
NYENDPKIAA; YENDPKIAAN; ENDPKIAANF; NDPKIAANFL;
DPKIAANFLY; PKIAANFLYR; KIAANFLYRI; IAANFLYRIA;
AANFLYRIAL; ANFLYRIALD; NFLYRIALDI; FLYRIALDIQ;
LYRIALDIQL; YRIALDIQLK; RIALDIQLKL; IALDIQLKLE;
ALDIQLKLEK; LDIQLKLEKH; DIQLKLEKHL; IQLKLEKHLK;
QLKLEKHLKS; LKLEKHLKSI; KLEKHLKSIN; LEKHLKSINE;
EKHLKSINEK; KHLKSINEKL; HLKSINEKLD; LKSINEKLDT;
KSINEKLDTL; SINEKLDTLS; INEKLDTLSK; NEKLDTLSKE;
EKLDTLSKEN; KLDTLSKENS; LDTLSKENSK; DTLSKENSKE;
TLSKENSKED; LSKENSKEDL; SKENSKEDLE; KENSKEDLEA;
ENSKEDLEAL; NSKEDLEALL; SKEDLEALLE; KEDLEALLEQ;
EDLEALLEQV; DLEALLEQVK; LEALLEQVKS; EALLEQVKSA;
ALLEQVKSAL; LLEQVKSALQ; LEQVKSALQL; EQVKSALQLQ;
QVKSALQLQE; VKSALQLQEK; KSALQLQEKF; SALQLQEKFK;
ALQLQEKFKK; LQLQEKFKKT; QLQEKFKKTL; LQEKFKKTLN;
QEKFKKTLNK; EKFKKTLNKT; KFKKTLNKTL; FKKTLNKTLE;
KKTLNKTLED; KTLNKTLEDY; TLNKTLEDYR; LNKTLEDYRK;
NKTLEDYRKN; KTLEDYRKNT; TLEDYRKNTN; LEDYRKNTNN;
EDYRKNTNNI; DYRKNTNNIQ; YRKNTNNIQE; RKNTNNIQEN;
KNTNNIQENK; NTNNIQENKV; TNNIQENKVL; NNIQENKVLA;
NIQENKVLAE; IQENKVLAEH; QENKVLAEHF; ENKVLAEHFN;
NKVLAEHFNK; KVLAEHFNKY; VLAEHFNKYY; LAEHFNKYYK;
AEHFNKYYKD; EHFNKYYKDS; HFNKYYKDSD; FNKYYKDSDS;
NKYYKDSDSL; KYYKDSDSLQ; YYKDSDSLQS; YKDSDSLQSA;
KDSDSLQSAF; DSDSLQSAFY;

11 mers:
MKKAKLNIIKI; KKAKLNIIKIN; KAKLNIIKINI; AKLNIIKINII;
KLNIIKINIIT; LNIIKINIITM; NIIKINIITMI; IIKINIITMIL;
IKINIITMILT; KINIITMILTL; INIITMILTLI; NIITMILTLIC;
IITMILTLICI; ITMILTLICIS; TMILTLICISC; MILTLICISCA;
ILTLICISCAP; LTLICISCAPF; TLICISCAPFN; LICISCAPFNK;
ICISCAPFNKI; CISCAPFNKIN; ISCAPFNKINP; SCAPFNKINPK;
CAPFNKINPKA; APFNKINPKAN; PFNKINPKANE; FNKINPKANEN;
NKINPKANENT; KINPKANENTK; INPKANENTKL; NPKANENTKLK;
PKANENTKLKK; KANENTKLKKN; ANENTKLKKNT; NENTKLKKNTR;
ENTKLKKNTRL; NTKLKKNTRLK; TKLKKNTRLKK; KLKKNTRLKKP;
LKKNTRLKKPA; KKNTRLKKPAN; KNTRLKKPANP; NTRLKKPANPG;
TRLKKPANPGE; RLKKPANPGEN; LKKPANPGENI; KKPANPGENIQ;
KPANPGENIQN; PANPGENIQNF; ANPGENIQNFK; NPGENIQNFKD;
PGENIQNFKDK; GENIQNFKDKS; ENIQNFKDKSG; NIQNFKDKSGD;
IQNFKDKSGDL; QNFKDKSGDLG; NFKDKSGDLGA; FKDKSGDLGAS;
KDKSGDLGASD; DKSGDLGASDE; KSGDLGASDEK; SGDLGASDEKF;
GDLGASDEKFM; DLGASDEKFMG; LGASDEKFMGT; GASDEKFMGTT;
ASDEKFMGTTA; SDEKFMGTTAS; DEKFMGTTASE; EKFMGTTASEL;
KFMGTTASELK; FMGTTASELKA; MGTTASELKAI; GTTASELKAIG;
TTASELKAIGK; TASELKAIGKE; ASELKAIGKEL; SELKAIGKELE;

| | |
|---|---|
| | ELKAIGKELED; LKAIGKELEDR; KAIGKELEDRK; AIGKELEDRKN; IGKELEDRKNQ; GKELEDRKNQY; KELEDRKNQYD; ELEDRKNQYDI; LEDRKNQYDIQ; EDRKNQYDIQI; DRKNQYDIQIA; RKNQYDIQIAK; KNQYDIQIAKI; NQYDIQIAKIT; QYDIQIAKITN; YDIQIAKITNE; DIQIAKITNEE; IQIAKITNEES; QIAKITNEESN; IAKITNEESNL; AKITNEESNLL; KITNEESNLLD; ITNEESNLLDT; TNEESNLLDTY; NEESNLLDTYI; EESNLLDTYIR; ESNLLDTYIRA; SNLLDTYIRAY; NLLDTYIRAYE; LLDTYIRAYEL; LDTYIRAYELA; DTYIRAYELAN; TYIRAYELANE; YIRAYELANEN; IRAYELANENE; RAYELANENEK; AYELANENEKM; YELANENEKML; ELANENEKMLL; LANENEKMLLK; ANENEKMLLKR; NENEKMLLKRF; ENEKMLLKRFL; NEKMLLKRFLL; EKMLLKRFLLS; KMLLKRFLLSS; MLLKRFLLSSL; LLKRFLLSSLD; LKRFLLSSLDY; KRFLLSSLDYK; RFLLSSLDYKK; FLLSSLDYKKE; LLSSLDYKKEN; LSSLDYKKENI; SSLDYKKENIE; SLDYKKENIET; LDYKKENIETL; DYKKENIETLK; YKKENIETLKE; KKENIETLKEI; KENIETLKEIL; ENIETLKEILE; NIETLKEILEK; IETLKEILEKL; ETLKEILEKLI; TLKEILEKLIN; LKEILEKLINN; KEILEKLINNY; EILEKLINNYE; ILEKLINNYEN; LEKLINNYEND; EKLINNYENDP; KLINNYENDPK; LINNYENDPKI; INNYENDPKIA; NNYENDPKIAA; NYENDPKIAAN; YENDPKIAANF; ENDPKIAANFL; NDPKIAANFLY; DPKIAANFLYR; PKIAANFLYRI; KIAANFLYRIA; IAANFLYRIAL; AANFLYRIALD; ANFLYRIALDI; NFLYRIALDIQ; FLYRIALDIQL; LYRIALDIQLK; YRIALDIQLKL; RIALDIQLKLE; IALDIQLKLEK; ALDIQLKLEKH; LDIQLKLEKHL; DIQLKLEKHLK; IQLKLEKHLKS; QLKLEKHLKSI; LKLEKHLKSIN; KLEKHLKSINE; LEKHLKSINEK; EKHLKSINEKL; KHLKSINEKLD; HLKSINEKLDT; LKSINEKLDTL; KSINEKLDTLS; SINEKLDTLSK; INEKLDTLSKE; NEKLDTLSKEN; EKLDTLSKENS; KLDTLSKENSK; LDTLSKENSKE; DTLSKENSKED; TLSKENSKEDL; LSKENSKEDLE; SKENSKEDLEA; KENSKEDLEAL; ENSKEDLEALL; NSKEDLEALLE; SKEDLEALLEQ; KEDLEALLEQV; EDLEALLEQVK; DLEALLEQVKS; LEALLEQVKSA; EALLEQVKSAL; ALLEQVKSALQ; LLEQVKSALQL; LEQVKSALQLQ; EQVKSALQLQE; QVKSALQLQEK; VKSALQLQEKF; KSALQLQEKFK; SALQLQEKFKK; ALQLQEKFKKT; LQLQEKFKKTL; QLQEKFKKTLN; LQEKFKKTLNK; QEKFKKTLNKT; EKFKKTLNKTL; KFKKTLNKTLE; FKKTLNKTLED; KKTLNKTLEDY; KTLNKTLEDYR; TLNKTLEDYRK; LNKTLEDYRKN; NKTLEDYRKNT; KTLEDYRKNTN; TLEDYRKNTNN; LEDYRKNTNNI; EDYRKNTNNIQ; DYRKNTNNIQE; YRKNTNNIQEN; RKNTNNIQENK; KNTNNIQENKV; NTNNIQENKVL; TNNIQENKVLA; NNIQENKVLAE; NIQENKVLAEH; IQENKVLAEHF; QENKVLAEHFN; ENKVLAEHFNK; NKVLAEHFNKY; KVLAEHFNKYY; VLAEHFNKYYK; LAEHFNKYYKD; AEHFNKYYKDS; EHFNKYYKDSD; HFNKYYKDSDS; FNKYYKDSDSL; NKYYKDSDSLQ; KYYKDSDSLQS; YYKDSDSLQSA; YKDSDSLQSAF; KDSDSLQSAFY; |
| Seq 37 | SEQ ID NO 44899-45904/ 8 mers: MKKTKLNI; KKTKLNII; KTKLNIIK; TKLNIIKL; KLNIIKLN; LNIIKLNI; NIIKLNIL; IIKLNILT; IKLNILTT; KLNILTTT; LNILTTTL; NILTTTLT; ILTTTLTL; LTTTLTLI; TTTLTLIC; TTLTLICI; TLTLICIS; LTLICISC; TLICISCA; LICISCAV; |

ICISCAVN; CISCAVNK; ISCAVNKI; SCAVNKID; CAVNKIDP;
AVNKIDPE; VNKIDPEP; NKIDPEPK; KIDPEPKS; IDPEPKSK;
DPEPKSKT; PEPKSKTN; EPKSKTNK; PKSKTNKK; KSKTNKKE;
SKTNKKEN; KTNKKENI; TNKKENIK; NKKENIKN; KKENIKNF;
KENIKNFV; ENIKNFVN; NIKNFVNK; IKNFVNKF; KNFVNKFQ;
NFVNKFQD; FVNKFQDL; VNKFQDLE; NKFQDLEP; KFQDLEPS;
FQDLEPSK; QDLEPSKK; DLEPSKKQ; LEPSKKQN; EPSKKQNK;
PSKKQNKD; SKKQNKDL; KKQNKDLE; KQNKDLEP; QNKDLEPL;
NKDLEPLR; KDLEPLRE; DLEPLREK; LEPLREKY; EPLREKYP;
PLREKYPE; LREKYPEA; REKYPEAT; EKYPEATA; KYPEATAS;
YPEATASK; PEATASKL; EATASKLE; ATASKLET; TASKLETT;
ASKLETTL; SKLETTLK; KLETTLKI; LETTLKIL; ETTLKILE;
TTLKILEA; TLKILEAQ; LKILEAQK; KILEAQKE; ILEAQKEK;
LEAQKEKE; EAQKEKEN; AQKEKENI; QKEKENIE; KEKENIEI;
EKENIEIA; KENIEIAK; ENIEIAKI; NIEIAKID; IEIAKIDN;
EIAKIDNT; IAKIDNTQ; AKIDNTQI; KIDNTQID; IDNTQIDF;
DNTQIDFL; NTQIDFLK; TQIDFLKT; QIDFLKTF; IDFLKTFK;
DFLKTFKT; FLKTFKTD; LKTFKTDP; KTFKTDPH; TFKTDPHD;
FKTDPHDS; KTDPHDSL; TDPHDSLP; DPHDSLPE; PHDSLPED;
HDSLPEDE; DSLPEDEK; SLPEDEKM; LPEDEKMQ; PEDEKMQM;
EDEKMQMK; DEKMQMKK; EKMQMKKI; KMQMKKII; MQMKKIIY;
QMKKIIYS; MKKIIYSS; KKIIYSSL; KIIYSSLN; IIYSSLNY;
IYSSLNYE; YSSLNYET; SSLNYETE; SLNYETEK; LNYETEKI;
NYETEKIK; YETEKIKI; ETEKIKIL; TEKIKILQ; EKIKILQE;
KIKILQEI; IKILQEIL; KILQEILE; ILQEILEK; LQEILEKL;
QEILEKLD; EILEKLDK; ILEKLDKN; LEKLDKNL; EKLDKNLQ;
KLDKNLQH; LDKNLQHK; DKNLQHKK; KNLQHKKI; NLQHKKIA;
LQHKKIAK; QHKKIAKN; HKKIAKNF; KKIAKNFI; KIAKNFIY;
IAKNFIYD; AKNFIYDI; KNFIYDIP; NFIYDIPI; FIYDIPII;
IYDIPIII; YDIPIIIQ; DIPIIIQS; IPIIIQSR; PIIIQSRL;
IIIQSRLD; IIQSRLDI; IQSRLDII; QSRLDIIS; SRLDIISK;
RLDIISKV; LDIISKVI; DIISKVIK; IISKVIKN; ISKVIKNP;
SKVIKNPI; KVIKNPIK; VIKNPIKD; IKNPIKDE; KNPIKDEL;
NPIKDELQ; PIKDELQI; IKDELQIL; KDELQILN; DELQILNQ;
ELQILNQK; LQILNQKE; QILNQKEI; ILNQKEIE; LNQKEIEE;
NQKEIEEL; QKEIEELL; KEIEELLM; EIEELLMR; IEELLMRI;
EELLMRIE; ELLMRIES; LLMRIESE; LMRIESEL; MRIESELK;
RIESELKI; IESELKIK; ESELKIKE; SELKIKEN; ELKIKENF;
LKIKENFK; KIKENFKK; IKENFKKA; KENFKKAL; ENFKKALN;
NFKKALNK; FKKALNKT; KKALNKTI; KALNKTID; ALNKTIDA;
LNKTIDAY; NKTIDAYN; KTIDAYNQ; TIDAYNQD; IDAYNQDS;
DAYNQDSE; AYNQDSEN; YNQDSENI; NQDSENIK; QDSENIKT;
DSENIKTS; SENIKTSA; ENIKTSAE; NIKTSAEQ; IKTSAEQL;
KTSAEQLE; TSAEQLEK; SAEQLEKH; AEQLEKHI; EQLEKHIN;
QLEKHINE; LEKHINEN; EKHINENY; KHINENYK; HINENYKE;
INENYKEF; NENYKEFN; ENYKEFNS; NYKEFNSL; YKEFNSLK;
KEFNSLKP; EFNSLKPI; FNSLKPIY;

9 mers:
MKKTKLNII; KKTKLNIIK; KTKLNIIKL; TKLNIIKLN; KLNIIKLNI;
LNIIKLNIL; NIIKLNILT; IIKLNILTT; IKLNILTTT; KLNILTTTL;

LNILTTTLT; NILTTTLTL; ILTTTLTLI; LTTTLTLIC; TTTLTLICI;
TTLTLICIS; TLTLICISC; LTLICISCA; TLICISCAV; LICISCAVN;
ICISCAVNK; CISCAVNKI; ISCAVNKID; SCAVNKIDP; CAVNKIDPE;
AVNKIDPEP; VNKIDPEPK; NKIDPEPKS; KIDPEPKSK; IDPEPKSKT;
DPEPKSKTN; PEPKSKTNK; EPKSKTNKK; PKSKTNKKE; KSKTNKKEN;
SKTNKKENI; KTNKKENIK; TNKKENIKN; NKKENIKNF; KKENIKNFV;
KENIKNFVN; ENIKNFVNK; NIKNFVNKF; IKNFVNKFQ; KNFVNKFQD;
NFVNKFQDL; FVNKFQDLE; VNKFQDLEP; NKFQDLEPS; KFQDLEPSK;
FQDLEPSKK; QDLEPSKKQ; DLEPSKKQN; LEPSKKQNK; EPSKKQNKD;
PSKKQNKDL; SKKQNKDLE; KKQNKDLEP; KQNKDLEPL; QNKDLEPLR;
NKDLEPLRE; KDLEPLREK; DLEPLREKY; LEPLREKYP; EPLREKYPE;
PLREKYPEA; LREKYPEAT; REKYPEATA; EKYPEATAS; KYPEATASK;
YPEATASKL; PEATASKLE; EATASKLET; ATASKLETT; TASKLETTL;
ASKLETTLK; SKLETTLKI; KLETTLKIL; LETTLKILE; ETTLKILEA;
TTLKILEAQ; TLKILEAQK; LKILEAQKE; KILEAQKEK; ILEAQKEKE;
LEAQKEKEN; EAQKEKENI; AQKEKENIE; QKEKENIEI; KEKENIEIA;
EKENIEIAK; KENIEIAKI; ENIEIAKID; NIEIAKIDN; IEIAKIDNT;
EIAKIDNTQ; IAKIDNTQI; AKIDNTQID; KIDNTQIDF; IDNTQIDFL;
DNTQIDFLK; NTQIDFLKT; TQIDFLKTF; QIDFLKTFK; IDFLKTFKT;
DFLKTFKTD; FLKTFKTDP; LKTFKTDPH; KTFKTDPHD; TFKTDPHDS;
FKTDPHDSL; KTDPHDSLP; TDPHDSLPE; DPHDSLPED; PHDSLPEDE;
HDSLPEDEK; DSLPEDEKM; SLPEDEKMQ; LPEDEKMQM; PEDEKMQMK;
EDEKMQMKK; DEKMQMKKI; EKMQMKKII; KMQMKKIIY; MQMKKIIYS;
QMKKIIYSS; MKKIIYSSL; KKIIYSSLN; KIIYSSLNY; IIYSSLNYE;
IYSSLNYET; YSSLNYETE; SSLNYETEK; SLNYETEKI; LNYETEKIK;
NYETEKIKI; YETEKIKIL; ETEKIKILQ; TEKIKILQE; EKIKILQEI;
KIKILQEIL; IKILQEILE; KILQEILEK; ILQEILEKL; LQEILEKLD;
QEILEKLDK; EILEKLDKN; ILEKLDKNL; LEKLDKNLQ; EKLDKNLQH;
KLDKNLQHK; LDKNLQHKK; DKNLQHKKI; KNLQHKKIA; NLQHKKIAK;
LQHKKIAKN; QHKKIAKNF; HKKIAKNFI; KKIAKNFIY; KIAKNFIYD;
IAKNFIYDI; AKNFIYDIP; KNFIYDIPI; NFIYDIPII; FIYDIPIII;
IYDIPIIIQ; YDIPIIIQS; DIPIIIQSR; IPIIIQSRL; PIIIQSRLD;
IIIQSRLDI; IIQSRLDII; IQSRLDIIS; QSRLDIISK; SRLDIISKV;
RLDIISKVI; LDIISKVIK; DIISKVIKN; IISKVIKNP; ISKVIKNPI;
SKVIKNPIK; KVIKNPIKD; VIKNPIKDE; IKNPIKDEL; KNPIKDELQ;
NPIKDELQI; PIKDELQIL; IKDELQILN; KDELQILNQ; DELQILNQK;
ELQILNQKE; LQILNQKEI; QILNQKEIE; ILNQKEIEE; LNQKEIEEL;
NQKEIEELL; QKEIEELLM; KEIEELLMR; EIEELLMRI; IEELLMRIE;
EELLMRIES; ELLMRIESE; LLMRIESEL; LMRIESELK; MRIESELKI;
RIESELKIK; IESELKIKE; ESELKIKEN; SELKIKENF; ELKIKENFK;
LKIKENFKK; KIKENFKKA; IKENFKKAL; KENFKKALN; ENFKKALNK;
NFKKALNKT; FKKALNKTI; KKALNKTID; KALNKTIDA; ALNKTIDAY;
LNKTIDAYN; NKTIDAYNQ; KTIDAYNQD; TIDAYNQDS; IDAYNQDSE;
DAYNQDSEN; AYNQDSENI; YNQDSENIK; NQDSENIKT; QDSENIKTS;
DSENIKTSA; SENIKTSAE; ENIKTSAEQ; NIKTSAEQL; IKTSAEQLE;
KTSAEQLEK; TSAEQLEKH; SAEQLEKHI; AEQLEKHIN; EQLEKHINE;
QLEKHINEN; LEKHINENY; EKHINENYK; KHINENYKE; HINENYKEF;
INENYKEFN; NENYKEFNS; ENYKEFNSL; NYKEFNSLK; YKEFNSLKP;
KEFNSLKPI; EFNSLKPIY;

10 mers:

784

MKKTKLNIIK; KKTKLNIIKL; KTKLNIIKLN; TKLNIIKLNI;
KLNIIKLNIL; LNIIKLNILT; NIIKLNILTT; IIKLNILTTT;
IKLNILTTTL; KLNILTTTLT; LNILTTTLTL; NILTTTLTLI;
ILTTTLTLIC; LTTTLTLICI; TTTLTLICIS; TTLTLICISC;
TLTLICISCA; LTLICISCAV; TLICISCAVN; LICISCAVNK;
ICISCAVNKI; CISCAVNKID; ISCAVNKIDP; SCAVNKIDPE;
CAVNKIDPEP; AVNKIDPEPK; VNKIDPEPKS; NKIDPEPKSK;
KIDPEPKSKT; IDPEPKSKTN; DPEPKSKTNK; PEPKSKTNKK;
EPKSKTNKKE; PKSKTNKKEN; KSKTNKKENI; SKTNKKENIK;
KTNKKENIKN; TNKKENIKNF; NKKENIKNFV; KKENIKNFVN;
KENIKNFVNK; ENIKNFVNKF; NIKNFVNKFQ; IKNFVNKFQD;
KNFVNKFQDL; NFVNKFQDLE; FVNKFQDLEP; VNKFQDLEPS;
NKFQDLEPSK; KFQDLEPSKK; FQDLEPSKKQ; QDLEPSKKQN;
DLEPSKKQNK; LEPSKKQNKD; EPSKKQNKDL; PSKKQNKDLE;
SKKQNKDLEP; KKQNKDLEPL; KQNKDLEPLR; QNKDLEPLRE;
NKDLEPLREK; KDLEPLREKY; DLEPLREKYP; LEPLREKYPE;
EPLREKYPEA; PLREKYPEAT; LREKYPEATA; REKYPEATAS;
EKYPEATASK; KYPEATASKL; YPEATASKLE; PEATASKLET;
EATASKLETT; ATASKLETTL; TASKLETTLK; ASKLETTLKI;
SKLETTLKIL; KLETTLKILE; LETTLKILEA; ETTLKILEAQ;
TTLKILEAQK; TLKILEAQKE; LKILEAQKEK; KILEAQKEKE;
ILEAQKEKEN; LEAQKEKENI; EAQKEKENIE; AQKEKENIEI;
QKEKENIEIA; KEKENIEIAK; EKENIEIAKI; KENIEIAKID;
ENIEIAKIDN; NIEIAKIDNT; IEIAKIDNTQ; EIAKIDNTQI;
IAKIDNTQID; AKIDNTQIDF; KIDNTQIDFL; IDNTQIDFLK;
DNTQIDFLKT; NTQIDFLKTF; TQIDFLKTFK; QIDFLKTFKT;
IDFLKTFKTD; DFLKTFKTDP; FLKTFKTDPH; LKTFKTDPHD;
KTFKTDPHDS; TFKTDPHDSL; FKTDPHDSLP; KTDPHDSLPE;
TDPHDSLPED; DPHDSLPEDE; PHDSLPEDEK; HDSLPEDEKM;
DSLPEDEKMQ; SLPEDEKMQM; LPEDEKMQMK; PEDEKMQMKK;
EDEKMQMKKI; DEKMQMKKII; EKMQMKKIIY; KMQMKKIIYS;
MQMKKIIYSS; QMKKIIYSSL; MKKIIYSSLN; KKIIYSSLNY;
KIIYSSLNYE; IIYSSLNYET; IYSSLNYETE; YSSLNYETEK;
SSLNYETEKI; SLNYETEKIK; LNYETEKIKI; NYETEKIKIL;
YETEKIKILQ; ETEKIKILQE; TEKIKILQEI; EKIKILQEIL;
KIKILQEILE; IKILQEILEK; KILQEILEKL; ILQEILEKLD;
LQEILEKLDK; QEILEKLDKN; EILEKLDKNL; ILEKLDKNLQ;
LEKLDKNLQH; EKLDKNLQHK; KLDKNLQHKK; LDKNLQHKKI;
DKNLQHKKIA; KNLQHKKIAK; NLQHKKIAKN; LQHKKIAKNF;
QHKKIAKNFI; HKKIAKNFIY; KKIAKNFIYD; KIAKNFIYDI;
IAKNFIYDIP; AKNFIYDIPI; KNFIYDIPII; NFIYDIPIII;
FIYDIPIIIQ; IYDIPIIIQS; YDIPIIIQSR; DIPIIIQSRL;
IPIIIQSRLD; PIIIQSRLDI; IIIQSRLDII; IIQSRLDIIS;
IQSRLDIISK; QSRLDIISKV; SRLDIISKVI; RLDIISKVIK;
LDIISKVIKN; DIISKVIKNP; IISKVIKNPI; ISKVIKNPIK;
SKVIKNPIKD; KVIKNPIKDE; VIKNPIKDEL; IKNPIKDELQ;
KNPIKDELQI; NPIKDELQIL; PIKDELQILN; IKDELQILNQ;
KDELQILNQK; DELQILNQKE; ELQILNQKEI; LQILNQKEIE;
QILNQKEIEE; ILNQKEIEEL; LNQKEIEELL; NQKEIEELLM;
QKEIEELLMR; KEIEELLMRI; EIEELLMRIE; IEELLMRIES;
EELLMRIESE; ELLMRIESEL; LLMRIESELK; LMRIESELKI;

MRIESELKIK; RIESELKIKE; IESELKIKEN; ESELKIKENF;
SELKIKENFK; ELKIKENFKK; LKIKENFKKA; KIKENFKKAL;
IKENFKKALN; KENFKKALNK; ENFKKALNKT; NFKKALNKTI;
FKKALNKTID; KKALNKTIDA; KALNKTIDAY; ALNKTIDAYN;
LNKTIDAYNQ; NKTIDAYNQD; KTIDAYNQDS; TIDAYNQDSE;
IDAYNQDSEN; DAYNQDSENI; AYNQDSENIK; YNQDSENIKT;
NQDSENIKTS; QDSENIKTSA; DSENIKTSAE; SENIKTSAEQ;
ENIKTSAEQL; NIKTSAEQLE; IKTSAEQLEK; KTSAEQLEKH;
TSAEQLEKHI; SAEQLEKHIN; AEQLEKHINE; EQLEKHINEN;
QLEKHINENY; LEKHINENYK; EKHINENYKE; KHINENYKEF;
HINENYKEFN; INENYKEFNS; NENYKEFNSL; ENYKEFNSLK;
NYKEFNSLKP; YKEFNSLKPI; KEFNSLKPIY;

11 mers:
MKKTKLNIIKL; KKTKLNIIKLN; KTKLNIIKLNI; TKLNIIKLNIL;
KLNIIKLNILT; LNIIKLNILTT; NIIKLNILTTT; IIKLNILTTTL;
IKLNILTTTLT; KLNILTTTLTL; LNILTTTLTLI; NILTTTLTLIC;
ILTTTLTLICI; LTTTLTLICIS; TTTLTLICISC; TTLTLICISCA;
TLTLICISCAV; LTLICISCAVN; TLICISCAVNK; LICISCAVNKI;
ICISCAVNKID; CISCAVNKIDP; ISCAVNKIDPE; SCAVNKIDPEP;
CAVNKIDPEPK; AVNKIDPEPKS; VNKIDPEPKSK; NKIDPEPKSKT;
KIDPEPKSKTN; IDPEPKSKTNK; DPEPKSKTNKK; PEPKSKTNKKE;
EPKSKTNKKEN; PKSKTNKKENI; KSKTNKKENIK; SKTNKKENIKN;
KTNKKENIKNF; TNKKENIKNFV; NKKENIKNFVN; KKENIKNFVNK;
KENIKNFVNKF; ENIKNFVNKFQ; NIKNFVNKFQD; IKNFVNKFQDL;
KNFVNKFQDLE; NFVNKFQDLEP; FVNKFQDLEPS; VNKFQDLEPSK;
NKFQDLEPSKK; KFQDLEPSKKQ; FQDLEPSKKQN; QDLEPSKKQNK;
DLEPSKKQNKD; LEPSKKQNKDL; EPSKKQNKDLE; PSKKQNKDLEP;
SKKQNKDLEPL; KKQNKDLEPLR; KQNKDLEPLRE; QNKDLEPLREK;
NKDLEPLREKY; KDLEPLREKYP; DLEPLREKYPE; LEPLREKYPEA;
EPLREKYPEAT; PLREKYPEATA; LREKYPEATAS; REKYPEATASK;
EKYPEATASKL; KYPEATASKLE; YPEATASKLET; PEATASKLETT;
EATASKLETTL; ATASKLETTLK; TASKLETTLKI; ASKLETTLKIL;
SKLETTLKILE; KLETTLKILEA; LETTLKILEAQ; ETTLKILEAQK;
TTLKILEAQKE; TLKILEAQKEK; LKILEAQKEKE; KILEAQKEKEN;
ILEAQKEKENI; LEAQKEKENIE; EAQKEKENIEI; AQKEKENIEIA;
QKEKENIEIAK; KEKENIEIAKI; EKENIEIAKID; KENIEIAKIDN;
ENIEIAKIDNT; NIEIAKIDNTQ; IEIAKIDNTQI; EIAKIDNTQID;
IAKIDNTQIDF; AKIDNTQIDFL; KIDNTQIDFLK; IDNTQIDFLKT;
DNTQIDFLKTF; NTQIDFLKTFK; TQIDFLKTFKT; QIDFLKTFKTD;
IDFLKTFKTDP; DFLKTFKTDPH; FLKTFKTDPHD; LKTFKTDPHDS;
KTFKTDPHDSL; TFKTDPHDSLP; FKTDPHDSLPE; KTDPHDSLPED;
TDPHDSLPEDE; DPHDSLPEDEK; PHDSLPEDEKM; HDSLPEDEKMQ;
DSLPEDEKMQM; SLPEDEKMQMK; LPEDEKMQMKK; PEDEKMQMKKI;
EDEKMQMKKII; DEKMQMKKIIY; EKMQMKKIIYS; KMQMKKIIYSS;
MQMKKIIYSSL; QMKKIIYSSLN; MKKIIYSSLNY; KKIIYSSLNYE;
KIIYSSLNYET; IIYSSLNYETE; IYSSLNYETEK; YSSLNYETEKI;
SSLNYETEKIK; SLNYETEKIKI; LNYETEKIKIL; NYETEKIKILQ;
YETEKIKILQE; ETEKIKILQEI; TEKIKILQEIL; EKIKILQEILE;
KIKILQEILEK; IKILQEILEKL; KILQEILEKLD; ILQEILEKLDK;
LQEILEKLDKN; QEILEKLDKNL; EILEKLDKNLQ; ILEKLDKNLQH;

| | |
|---|---|
| | LEKLDKNLQHK; EKLDKNLQHKK; KLDKNLQHKKI; LDKNLQHKKIA; DKNLQHKKIAK; KNLQHKKIAKN; NLQHKKIAKNF; LQHKKIAKNFI; QHKKIAKNFIY; HKKIAKNFIYD; KKIAKNFIYDI; KIAKNFIYDIP; IAKNFIYDIPI; AKNFIYDIPII; KNFIYDIPIII; NFIYDIPIIIQ; FIYDIPIIIQS; IYDIPIIIQSR; YDIPIIIQSRL; DIPIIIQSRLD; IPIIIQSRLDI; PIIIQSRLDII; IIIQSRLDIIS; IIQSRLDIISK; IQSRLDIISKV; QSRLDIISKVI; SRLDIISKVIK; RLDIISKVIKN; LDIISKVIKNP; DIISKVIKNPI; IISKVIKNPIK; ISKVIKNPIKD; SKVIKNPIKDE; KVIKNPIKDEL; VIKNPIKDELQ; IKNPIKDELQI; KNPIKDELQIL; NPIKDELQILN; PIKDELQILNQ; IKDELQILNQK; KDELQILNQKE; DELQILNQKEI; ELQILNQKEIE; LQILNQKEIEE; QILNQKEIEEL; ILNQKEIEELL; LNQKEIEELLM; NQKEIEELLMR; QKEIEELLMRI; KEIEELLMRIE; EIEELLMRIES; IEELLMRIESE; EELLMRIESEL; ELLMRIESELK; LLMRIESELKI; LMRIESELKIK; MRIESELKIKE; RIESELKIKEN; IESELKIKENF; ESELKIKENFK; SELKIKENFKK; ELKIKENFKKA; LKIKENFKKAL; KIKENFKKALN; IKENFKKALNK; KENFKKALNKT; ENFKKALNKTI; NFKKALNKTID; FKKALNKTIDA; KKALNKTIDAY; KALNKTIDAYN; ALNKTIDAYNQ; LNKTIDAYNQD; NKTIDAYNQDS; KTIDAYNQDSE; TIDAYNQDSEN; IDAYNQDSENI; DAYNQDSENIK; AYNQDSENIKT; YNQDSENIKTS; NQDSENIKTSA; QDSENIKTSAE; DSENIKTSAEQ; SENIKTSAEQL; ENIKTSAEQLE; NIKTSAEQLEK; IKTSAEQLEKH; KTSAEQLEKHI; TSAEQLEKHIN; SAEQLEKHINE; AEQLEKHINEN; EQLEKHINENY; QLEKHINENYK; LEKHINENYKE; EKHINENYKEF; KHINENYKEFN; HINENYKEFNS; INENYKEFNSL; NENYKEFNSLK; ENYKEFNSLKP; NYKEFNSLKPI; YKEFNSLKPIY; |
| Seq 38 | SEQ ID NO 45905-46606/ 8 mers: MRILVGVC; RILVGVCI; ILVGVCII; LVGVCIIA; VGVCIIAL; GVCIIALA; VCIIALAL; CIIALALL; IIALALLG; IALALLGC; ALALLGCY; LALLGCYL; ALLGCYLP; LLGCYLPD; LGCYLPDN; GCYLPDNQ; CYLPDNQE; YLPDNQEQ; LPDNQEQA; PDNQEQAV; DNQEQAVQ; NQEQAVQT; QEQAVQTF; EQAVQTFF; QAVQTFFE; AVQTFFEN; VQTFFENS; QTFFENSE; TFFENSES; FFENSESS; FENSESSD; ENSESSDM; NSESSDMG; SESSDMGS; ESSDMGSD; SSDMGSDE; SDMGSDEI; DMGSDEIV; MGSDEIVT; GSDEIVTE; SDEIVTEG; DEIVTEGI; EIVTEGIF; IVTEGIFS; VTEGIFSS; TEGIFSSL; EGIFSSLK; GIFSSLKL; IFSSLKLY; FSSLKLYA; SSLKLYAS; SLKLYASE; LKLYASEH; KLYASEHR; LYASEHRL; YASEHRLL; ASEHRLLV; SEHRLLVE; EHRLLVEI; HRLLVEIK; RLLVEIKK; LLVEIKKT; LVEIKKTL; VEIKKTLI; EIKKTLIS; IKKTLISL; KKTLISLK; KTLISLKD; TLISLKDP; LISLKDPN; ISLKDPNY; SLKDPNYR; LKDPNYRG; KDPNYRGV; DPNYRGVV; PNYRGVVL; NYRGVVLP; YRGVVLPV; RGVVLPVS; GVVLPVSD; VVLPVSDY; VLPVSDYN; LPVSDYNE; PVSDYNEE; VSDYNEEY; SDYNEEYF; DYNEEYFN; YNEEYFNK; NEEYFNKF; EEYFNKFF; EYFNKFFL; YFNKFFLD; FNKFFLDL; NKFFLDLG; KFFLDLGS; FFLDLGSE; FLDLGSEQ; LDLGSEQS; DLGSEQSK; LGSEQSKD; GSEQSKDL; SEQSKDLI; EQSKDLIK; QSKDLIKL; SKDLIKLF; KDLIKLFI; DLIKLFIM; LIKLFIMV; IKLFIMVK; KLFIMVKN; |

LFIMVKNE; FIMVKNEQ; IMVKNEQN; MVKNEQNN; VKNEQNNN;
KNEQNNNK; NEQNNNKF; EQNNNKFM; QNNNKFMR; NNNKFMRI;
NNKFMRIV; NKFMRIVR; KFMRIVRW; FMRIVRWL; MRIVRWLY;
RIVRWLYS; IVRWLYSC; VRWLYSCI; RWLYSCIE; WLYSCIEE;
LYSCIEEL; YSCIEELY; SCIEELYS; CIEELYSP; IEELYSPD;
EELYSPDI; ELYSPDIK; LYSPDIKY; YSPDIKYS; SPDIKYSG;
PDIKYSGE; DIKYSGEE; IKYSGEEG; KYSGEEGS; YSGEEGSP;
SGEEGSPE; GEEGSPEY; EEGSPEYY; EGSPEYYR; GSPEYYRN;
SPEYYRNM; PEYYRNMP; EYYRNMPR; YYRNMPRP; YRNMPRPT;
RNMPRPTA; NMPRPTAY; MPRPTAYQ; PRPTAYQQ; RPTAYQQY;
PTAYQQYL; TAYQQYLK; AYQQYLKV; YQQYLKVK; QQYLKVKR;
QYLKVKRY; YLKVKRYD; LKVKRYDY; KVKRYDYN; VKRYDYNR;
KRYDYNRP; RYDYNRPV; YDYNRPVP; DYNRPVPI; YNRPVPIL;
NRPVPILP; RPVPILPT;

9 mers:
MRILVGVCI; RILVGVCII; ILVGVCIIA; LVGVCIIAL; VGVCIIALA;
GVCIIALAL; VCIIALALL; CIIALALLG; IIALALLGC; IALALLGCY;
ALALLGCYL; LALLGCYLP; ALLGCYLPD; LLGCYLPDN; LGCYLPDNQ;
GCYLPDNQE; CYLPDNQEQ; YLPDNQEQA; LPDNQEQAV; PDNQEQAVQ;
DNQEQAVQT; NQEQAVQTF; QEQAVQTFF; EQAVQTFFE; QAVQTFFEN;
AVQTFFENS; VQTFFENSE; QTFFENSES; TFFENSESS; FFENSESSD;
FENSESSDM; ENSESSDMG; NSESSDMGS; SESSDMGSD; ESSDMGSDE;
SSDMGSDEI; SDMGSDEIV; DMGSDEIVT; MGSDEIVTE; GSDEIVTEG;
SDEIVTEGI; DEIVTEGIF; EIVTEGIFS; IVTEGIFSS; VTEGIFSSL;
TEGIFSSLK; EGIFSSLKL; GIFSSLKLY; IFSSLKLYA; FSSLKLYAS;
SSLKLYASE; SLKLYASEH; LKLYASEHR; KLYASEHRL; LYASEHRLL;
YASEHRLLV; ASEHRLLVE; SEHRLLVEI; EHRLLVEIK; HRLLVEIKK;
RLLVEIKKT; LLVEIKKTL; LVEIKKTLI; VEIKKTLIS; EIKKTLISL;
IKKTLISLK; KKTLISLKD; KTLISLKDP; TLISLKDPN; LISLKDPNY;
ISLKDPNYR; SLKDPNYRG; LKDPNYRGV; KDPNYRGVV; DPNYRGVVL;
PNYRGVVLP; NYRGVVLPV; YRGVVLPVS; RGVVLPVSD; GVVLPVSDY;
VVLPVSDYN; VLPVSDYNE; LPVSDYNEE; PVSDYNEEY; VSDYNEEYF;
SDYNEEYFN; DYNEEYFNK; YNEEYFNKF; NEEYFNKFF; EEYFNKFFL;
EYFNKFFLD; YFNKFFLDL; FNKFFLDLG; NKFFLDLGS; KFFLDLGSE;
FFLDLGSEQ; FLDLGSEQS; LDLGSEQSK; DLGSEQSKD; LGSEQSKDL;
GSEQSKDLI; SEQSKDLIK; EQSKDLIKL; QSKDLIKLF; SKDLIKLFI;
KDLIKLFIM; DLIKLFIMV; LIKLFIMVK; IKLFIMVKN; KLFIMVKNE;
LFIMVKNEQ; FIMVKNEQN; IMVKNEQNN; MVKNEQNNN; VKNEQNNNK;
KNEQNNNKF; NEQNNNKFM; EQNNNKFMR; QNNNKFMRI; NNNKFMRIV;
NNKFMRIVR; NKFMRIVRW; KFMRIVRWL; FMRIVRWLY; MRIVRWLYS;
RIVRWLYSC; IVRWLYSCI; VRWLYSCIE; RWLYSCIEE; WLYSCIEEL;
LYSCIEELY; YSCIEELYS; SCIEELYSP; CIEELYSPD; IEELYSPDI;
EELYSPDIK; ELYSPDIKY; LYSPDIKYS; YSPDIKYSG; SPDIKYSGE;
PDIKYSGEE; DIKYSGEEG; IKYSGEEGS; KYSGEEGSP; YSGEEGSPE;
SGEEGSPEY; GEEGSPEYY; EEGSPEYYR; EGSPEYYRN; GSPEYYRNM;
SPEYYRNMP; PEYYRNMPR; EYYRNMPRP; YYRNMPRPT; YRNMPRPTA;
RNMPRPTAY; NMPRPTAYQ; MPRPTAYQQ; PRPTAYQQY; RPTAYQQYL;
PTAYQQYLK; TAYQQYLKV; AYQQYLKVK; YQQYLKVKR; QQYLKVKRY;
QYLKVKRYD; YLKVKRYDY; LKVKRYDYN; KVKRYDYNR; VKRYDYNRP;
KRYDYNRPV; RYDYNRPVP; YDYNRPVPI; DYNRPVPIL; YNRPVPILP;

NRPVPILPT;

10 mers:
MRILVGVCII; RILVGVCIIA; ILVGVCIIAL; LVGVCIIALA;
VGVCIIALAL; GVCIIALALL; VCIIALALLG; CIIALALLGC;
IIALALLGCY; IALALLGCYL; ALALLGCYLP; LALLGCYLPD;
ALLGCYLPDN; LLGCYLPDNQ; LGCYLPDNQE; GCYLPDNQEQ;
CYLPDNQEQA; YLPDNQEQAV; LPDNQEQAVQ; PDNQEQAVQT;
DNQEQAVQTF; NQEQAVQTFF; QEQAVQTFFE; EQAVQTFFEN;
QAVQTFFENS; AVQTFFENSE; VQTFFENSES; QTFFENSESS;
TFFENSESSD; FFENSESSDM; FENSESSDMG; ENSESSDMGS;
NSESSDMGSD; SESSDMGSDE; ESSDMGSDEI; SSDMGSDEIV;
SDMGSDEIVT; DMGSDEIVTE; MGSDEIVTEG; GSDEIVTEGI;
SDEIVTEGIF; DEIVTEGIFS; EIVTEGIFSS; IVTEGIFSSL;
VTEGIFSSLK; TEGIFSSLKL; EGIFSSLKLY; GIFSSLKLYA;
IFSSLKLYAS; FSSLKLYASE; SSLKLYASEH; SLKLYASEHR;
LKLYASEHRL; KLYASEHRLL; LYASEHRLLV; YASEHRLLVE;
ASEHRLLVEI; SEHRLLVEIK; EHRLLVEIKK; HRLLVEIKKT;
RLLVEIKKTL; LLVEIKKTLI; LVEIKKTLIS; VEIKKTLISL;
EIKKTLISLK; IKKTLISLKD; KKTLISLKDP; KTLISLKDPN;
TLISLKDPNY; LISLKDPNYR; ISLKDPNYRG; SLKDPNYRGV;
LKDPNYRGVV; KDPNYRGVVL; DPNYRGVVLP; PNYRGVVLPV;
NYRGVVLPVS; YRGVVLPVSD; RGVVLPVSDY; GVVLPVSDYN;
VVLPVSDYNE; VLPVSDYNEE; LPVSDYNEEY; PVSDYNEEYF;
VSDYNEEYFN; SDYNEEYFNK; DYNEEYFNKF; YNEEYFNKFF;
NEEYFNKFFL; EEYFNKFFLD; EYFNKFFLDL; YFNKFFLDLG;
FNKFFLDLGS; NKFFLDLGSE; KFFLDLGSEQ; FFLDLGSEQS;
FLDLGSEQSK; LDLGSEQSKD; DLGSEQSKDL; LGSEQSKDLI;
GSEQSKDLIK; SEQSKDLIKL; EQSKDLIKLF; QSKDLIKLFI;
SKDLIKLFIM; KDLIKLFIMV; DLIKLFIMVK; LIKLFIMVKN;
IKLFIMVKNE; KLFIMVKNEQ; LFIMVKNEQN; FIMVKNEQNN;
IMVKNEQNNN; MVKNEQNNNK; VKNEQNNNKF; KNEQNNNKFM;
NEQNNNKFMR; EQNNNKFMRI; QNNNKFMRIV; NNNKFMRIVR;
NNKFMRIVRW; NKFMRIVRWL; KFMRIVRWLY; FMRIVRWLYS;
MRIVRWLYSC; RIVRWLYSCI; IVRWLYSCIE; VRWLYSCIEE;
RWLYSCIEEL; WLYSCIEELY; LYSCIEELYS; YSCIEELYSP;
SCIEELYSPD; CIEELYSPDI; IEELYSPDIK; EELYSPDIKY;
ELYSPDIKYS; LYSPDIKYSG; YSPDIKYSGE; SPDIKYSGEE;
PDIKYSGEEG; DIKYSGEEGS; IKYSGEEGSP; KYSGEEGSPE;
YSGEEGSPEY; SGEEGSPEYY; GEEGSPEYYR; EEGSPEYYRN;
EGSPEYYRNM; GSPEYYRNMP; SPEYYRNMPR; PEYYRNMPRP;
EYYRNMPRPT; YYRNMPRPTA; YRNMPRPTAY; RNMPRPTAYQ;
NMPRPTAYQQ; MPRPTAYQQY; PRPTAYQQYL; RPTAYQQYLK;
PTAYQQYLKV; TAYQQYLKVK; AYQQYLKVKR; YQQYLKVKRY;
QQYLKVKRYD; QYLKVKRYDY; YLKVKRYDYN; LKVKRYDYNR;
KVKRYDYNRP; VKRYDYNRPV; KRYDYNRPVP; RYDYNRPVPI;
YDYNRPVPIL; DYNRPVPILP; YNRPVPILPT;

11 mers:
MRILVGVCIIA; RILVGVCIIAL; ILVGVCIIALA; LVGVCIIALAL;
VGVCIIALALL; GVCIIALALLG; VCIIALALLGC; CIIALALLGCY;

| | |
|---|---|
| | IIALALLGCYL; IALALLGCYLP; ALALLGCYLPD; LALLGCYLPDN; ALLGCYLPDNQ; LLGCYLPDNQE; LGCYLPDNQEQ; GCYLPDNQEQA; CYLPDNQEQAV; YLPDNQEQAVQ; LPDNQEQAVQT; PDNQEQAVQTF; DNQEQAVQTFF; NQEQAVQTFFE; QEQAVQTFFEN; EQAVQTFFENS; QAVQTFFENSE; AVQTFFENSES; VQTFFENSESS; QTFFENSESSD; TFFENSESSDM; FFENSESSDMG; FENSESSDMGS; ENSESSDMGSD; NSESSDMGSDE; SESSDMGSDEI; ESSDMGSDEIV; SSDMGSDEIVT; SDMGSDEIVTE; DMGSDEIVTEG; MGSDEIVTEGI; GSDEIVTEGIF; SDEIVTEGIFS; DEIVTEGIFSS; EIVTEGIFSSL; IVTEGIFSSLK; VTEGIFSSLKL; TEGIFSSLKLY; EGIFSSLKLYA; GIFSSLKLYAS; IFSSLKLYASE; FSSLKLYASEH; SSLKLYASEHR; SLKLYASEHRL; LKLYASEHRLL; KLYASEHRLLV; LYASEHRLLVE; YASEHRLLVEI; ASEHRLLVEIK; SEHRLLVEIKK; EHRLLVEIKKT; HRLLVEIKKTL; RLLVEIKKTLI; LLVEIKKTLIS; LVEIKKTLISL; VEIKKTLISLK; EIKKTLISLKD; IKKTLISLKDP; KKTLISLKDPN; KTLISLKDPNY; TLISLKDPNYR; LISLKDPNYRG; ISLKDPNYRGV; SLKDPNYRGVV; LKDPNYRGVVL; KDPNYRGVVLP; DPNYRGVVLPV; PNYRGVVLPVS; NYRGVVLPVSD; YRGVVLPVSDY; RGVVLPVSDYN; GVVLPVSDYNE; VVLPVSDYNEE; VLPVSDYNEEY; LPVSDYNEEYF; PVSDYNEEYFN; VSDYNEEYFNK; SDYNEEYFNKF; DYNEEYFNKFF; YNEEYFNKFFL; NEEYFNKFFLD; EEYFNKFFLDL; EYFNKFFLDLG; YFNKFFLDLGS; FNKFFLDLGSE; NKFFLDLGSEQ; KFFLDLGSEQS; FFLDLGSEQSK; FLDLGSEQSKD; LDLGSEQSKDL; DLGSEQSKDLI; LGSEQSKDLIK; GSEQSKDLIKL; SEQSKDLIKLF; EQSKDLIKLFI; QSKDLIKLFIM; SKDLIKLFIMV; KDLIKLFIMVK; DLIKLFIMVKN; LIKLFIMVKNE; IKLFIMVKNEQ; KLFIMVKNEQN; LFIMVKNEQNN; FIMVKNEQNNN; IMVKNEQNNNK; MVKNEQNNNKF; VKNEQNNNKFM; KNEQNNNKFMR; NEQNNNKFMRI; EQNNNKFMRIV; QNNNKFMRIVR; NNNKFMRIVRW; NNKFMRIVRWL; NKFMRIVRWLY; KFMRIVRWLYS; FMRIVRWLYSC; MRIVRWLYSCI; RIVRWLYSCIE; IVRWLYSCIEE; VRWLYSCIEEL; RWLYSCIEELY; WLYSCIEELYS; LYSCIEELYSP; YSCIEELYSPD; SCIEELYSPDI; CIEELYSPDIK; IEELYSPDIKY; EELYSPDIKYS; ELYSPDIKYSG; LYSPDIKYSGE; YSPDIKYSGEE; SPDIKYSGEEG; PDIKYSGEEGS; DIKYSGEEGSP; IKYSGEEGSPE; KYSGEEGSPEY; YSGEEGSPEYY; SGEEGSPEYYR; GEEGSPEYYRN; EEGSPEYYRNM; EGSPEYYRNMP; GSPEYYRNMPR; SPEYYRNMPRP; PEYYRNMPRPT; EYYRNMPRPTA; YYRNMPRPTAY; YRNMPRPTAYQ; RNMPRPTAYQQ; NMPRPTAYQQY; MPRPTAYQQYL; PRPTAYQQYLK; RPTAYQQYLKV; PTAYQQYLKVK; TAYQQYLKVKR; AYQQYLKVKRY; YQQYLKVKRYD; QQYLKVKRYDY; QYLKVKRYDYN; YLKVKRYDYNR; LKVKRYDYNRP; KVKRYDYNRPV; VKRYDYNRPVP; KRYDYNRPVPI; RYDYNRPVPIL; YDYNRPVPILP; DYNRPVPILPT; |
| Seq 39 | SEQ ID NO 46607-47212/ 8 mers: MRNKNIFK; RNKNIFKL; NKNIFKLF; KNIFKLFF; NIFKLFFA; IFKLFFAS; FKLFFASM; KLFFASML; LFFASMLF; FFASMLFV; FASMLFVM; ASMLFVMA; SMLFVMAC; MLFVMACK; LFVMACKA; FVMACKAY; VMACKAYV; MACKAYVE; ACKAYVEE; CKAYVEEK; KAYVEEKK; AYVEEKKE; YVEEKKEI; VEEKKEID; EEKKEIDS; EKKEIDSL; KKEIDSLM; KEIDSLME; EIDSLMED; IDSLMEDV; |

```
DSLMEDVL;  SLMEDVLA;  LMEDVLAL;  MEDVLALV;  EDVLALVN;
DVLALVND;  VLALVNDS;  LALVNDSS;  ALVNDSSG;  LVNDSSGG;
VNDSSGGK;  NDSSGGKF;  DSSGGKFK;  SSGGKFKD;  SGGKFKDY;
GGKFKDYK;  GKFKDYKD;  KFKDYKDK;  FKDYKDKI;  KDYKDKIN;
DYKDKINE;  YKDKINEL;  KDKINELK;  DKINELKE;  KINELKEN;
INELKENL;  NELKENLK;  ELKENLKD;  LKENLKDI;  KENLKDIG;
ENLKDIGN;  NLKDIGNA;  LKDIGNAE;  KDIGNAEL;  DIGNAELK;
IGNAELKE;  GNAELKEK;  NAELKEKL;  AELKEKLL;  ELKEKLLN;
LKEKLLNL;  KEKLLNLQ;  EKLLNLQN;  KLLNLQNS;  LLNLQNSF;
LNLQNSFQ;  NLQNSFQD;  LQNSFQDK;  QNSFQDKL;  NSFQDKLA;
SFQDKLAA;  FQDKLAAK;  QDKLAAKL;  DKLAAKLA;  KLAAKLAA;
LAAKLAAL;  AAKLAALK;  AKLAALKA;  KLAALKAA;  LAALKAAK;
AALKAAKN;  ALKAAKNT;  LKAAKNTI;  KAAKNTIE;  AAKNTIEN;
AKNTIENI;  KNTIENIT;  NTIENITD;  TIENITDK;  IENITDKD;
ENITDKDQ;  NITDKDQD;  ITDKDQDI;  TDKDQDIS;  DKDQDISK;
KDQDISKR;  DQDISKRK;  QDISKRKI;  DISKRKIW;  ISKRKIWS;
SKRKIWSE;  KRKIWSEA;  RKIWSEAK;  KIWSEAKL;  IWSEAKLV;
WSEAKLVG;  SEAKLVGV;  EAKLVGVT;  AKLVGVTV;  KLVGVTVP;
LVGVTVPL;  VGVTVPLL;  GVTVPLLG;  VTVPLLGS;  TVPLLGSN;
VPLLGSNT;  PLLGSNTS;  LLGSNTSG;  LGSNTSGN;  GSNTSGNG;
SNTSGNGD;  NTSGNGDK;  TSGNGDKM;  SGNGDKMS;  GNGDKMSK;
NGDKMSKN;  GDKMSKNA;  DKMSKNAV;  KMSKNAVE;  MSKNAVEQ;
SKNAVEQI;  KNAVEQID;  NAVEQIDK;  AVEQIDKV;  VEQIDKVI;
EQIDKVIK;  QIDKVIKF;  IDKVIKFL;  DKVIKFLE;  KVIKFLEE;
VIKFLEEG;  IKFLEEGT;  KFLEEGTN;

9 mers:
MRNKNIFKL;  RNKNIFKLF;  NKNIFKLFF;  KNIFKLFFA;  NIFKLFFAS;
IFKLFFASM;  FKLFFASML;  KLFFASMLF;  LFFASMLFV;  FFASMLFVM;
FASMLFVMA;  ASMLFVMAC;  SMLFVMACK;  MLFVMACKA;  LFVMACKAY;
FVMACKAYV;  VMACKAYVE;  MACKAYVEE;  ACKAYVEEK;  CKAYVEEKK;
KAYVEEKKE;  AYVEEKKEI;  YVEEKKEID;  VEEKKEIDS;  EEKKEIDSL;
EKKEIDSLM;  KKEIDSLME;  KEIDSLMED;  EIDSLMEDV;  IDSLMEDVL;
DSLMEDVLA;  SLMEDVLAL;  LMEDVLALV;  MEDVLALVN;  EDVLALVND;
DVLALVNDS;  VLALVNDSS;  LALVNDSSG;  ALVNDSSGG;  LVNDSSGGK;
VNDSSGGKF;  NDSSGGKFK;  DSSGGKFKD;  SSGGKFKDY;  SGGKFKDYK;
GGKFKDYKD;  GKFKDYKDK;  KFKDYKDKI;  FKDYKDKIN;  KDYKDKINE;
DYKDKINEL;  YKDKINELK;  KDKINELKE;  DKINELKEN;  KINELKENL;
INELKENLK;  NELKENLKD;  ELKENLKDI;  LKENLKDIG;  KENLKDIGN;
ENLKDIGNA;  NLKDIGNAE;  LKDIGNAEL;  KDIGNAELK;  DIGNAELKE;
IGNAELKEK;  GNAELKEKL;  NAELKEKLL;  AELKEKLLN;  ELKEKLLNL;
LKEKLLNLQ;  KEKLLNLQN;  EKLLNLQNS;  KLLNLQNSF;  LLNLQNSFQ;
LNLQNSFQD;  NLQNSFQDK;  LQNSFQDKL;  QNSFQDKLA;  NSFQDKLAA;
SFQDKLAAK;  FQDKLAAKL;  QDKLAAKLA;  DKLAAKLAA;  KLAAKLAAL;
LAAKLAALK;  AAKLAALKA;  AKLAALKAA;  KLAALKAAK;  LAALKAAKN;
AALKAAKNT;  ALKAAKNTI;  LKAAKNTIE;  KAAKNTIEN;  AAKNTIENI;
AKNTIENIT;  KNTIENITD;  NTIENITDK;  TIENITDKD;  IENITDKDQ;
ENITDKDQD;  NITDKDQDI;  ITDKDQDIS;  TDKDQDISK;  DKDQDISKR;
KDQDISKRK;  DQDISKRKI;  QDISKRKIW;  DISKRKIWS;  ISKRKIWSE;
SKRKIWSEA;  KRKIWSEAK;  RKIWSEAKL;  KIWSEAKLV;  IWSEAKLVG;
WSEAKLVGV;  SEAKLVGVT;  EAKLVGVTV;  AKLVGVTVP;  KLVGVTVPL;
```

LVGVTVPLL; VGVTVPLLG; GVTVPLLGS; VTVPLLGSN; TVPLLGSNT;
VPLLGSNTS; PLLGSNTSG; LLGSNTSGN; LGSNTSGNG; GSNTSGNGD;
SNTSGNGDK; NTSGNGDKM; TSGNGDKMS; SGNGDKMSK; GNGDKMSKN;
NGDKMSKNA; GDKMSKNAV; DKMSKNAVE; KMSKNAVEQ; MSKNAVEQI;
SKNAVEQID; KNAVEQIDK; NAVEQIDKV; AVEQIDKVI; VEQIDKVIK;
EQIDKVIKF; QIDKVIKFL; IDKVIKFLE; DKVIKFLEE; KVIKFLEEG;
VIKFLEEGT; IKFLEEGTN;

10 mers:
MRNKNIFKLF; RNKNIFKLFF; NKNIFKLFFA; KNIFKLFFAS;
NIFKLFFASM; IFKLFFASML; FKLFFASMLF; KLFFASMLFV;
LFFASMLFVM; FFASMLFVMA; FASMLFVMAC; ASMLFVMACK;
SMLFVMACKA; MLFVMACKAY; LFVMACKAYV; FVMACKAYVE;
VMACKAYVEE; MACKAYVEEK; ACKAYVEEKK; CKAYVEEKKE;
KAYVEEKKEI; AYVEEKKEID; YVEEKKEIDS; VEEKKEIDSL;
EEKKEIDSLM; EKKEIDSLME; KKEIDSLMED; KEIDSLMEDV;
EIDSLMEDVL; IDSLMEDVLA; DSLMEDVLAL; SLMEDVLALV;
LMEDVLALVN; MEDVLALVND; EDVLALVNDS; DVLALVNDSS;
VLALVNDSSG; LALVNDSSGG; ALVNDSSGGK; LVNDSSGGKF;
VNDSSGGKFK; NDSSGGKFKD; DSSGGKFKDY; SSGGKFKDYK;
SGGKFKDYKD; GGKFKDYKDK; GKFKDYKDKI; KFKDYKDKIN;
FKDYKDKINE; KDYKDKINEL; DYKDKINELK; YKDKINELKE;
KDKINELKEN; DKINELKENL; KINELKENLK; INELKENLKD;
NELKENLKDI; ELKENLKDIG; LKENLKDIGN; KENLKDIGNA;
ENLKDIGNAE; NLKDIGNAEL; LKDIGNAELK; KDIGNAELKE;
DIGNAELKEK; IGNAELKEKL; GNAELKEKLL; NAELKEKLLN;
AELKEKLLNL; ELKEKLLNLQ; LKEKLLNLQN; KEKLLNLQNS;
EKLLNLQNSF; KLLNLQNSFQ; LLNLQNSFQD; LNLQNSFQDK;
NLQNSFQDKL; LQNSFQDKLA; QNSFQDKLAA; NSFQDKLAAK;
SFQDKLAAKL; FQDKLAAKLA; QDKLAAKLAA; DKLAAKLAAL;
KLAAKLAALK; LAAKLAALKA; AAKLAALKAA; AKLAALKAAK;
KLAALKAAKN; LAALKAAKNT; AALKAAKNTI; ALKAAKNTIE;
LKAAKNTIEN; KAAKNTIENI; AAKNTIENIT; AKNTIENITD;
KNTIENITDK; NTIENITDKD; TIENITDKDQ; IENITDKDQD;
ENITDKDQDI; NITDKDQDIS; ITDKDQDISK; TDKDQDISKR;
DKDQDISKRK; KDQDISKRKI; DQDISKRKIW; QDISKRKIWS;
DISKRKIWSE; ISKRKIWSEA; SKRKIWSEAK; KRKIWSEAKL;
RKIWSEAKLV; KIWSEAKLVG; IWSEAKLVGV; WSEAKLVGVT;
SEAKLVGVTV; EAKLVGVTVP; AKLVGVTVPL; KLVGVTVPLL;
LVGVTVPLLG; VGVTVPLLGS; GVTVPLLGSN; VTVPLLGSNT;
TVPLLGSNTS; VPLLGSNTSG; PLLGSNTSGN; LLGSNTSGNG;
LGSNTSGNGD; GSNTSGNGDK; SNTSGNGDKM; NTSGNGDKMS;
TSGNGDKMSK; SGNGDKMSKN; GNGDKMSKNA; NGDKMSKNAV;
GDKMSKNAVE; DKMSKNAVEQ; KMSKNAVEQI; MSKNAVEQID;
SKNAVEQIDK; KNAVEQIDKV; NAVEQIDKVI; AVEQIDKVIK;
VEQIDKVIKF; EQIDKVIKFL; QIDKVIKFLE; IDKVIKFLEE;
DKVIKFLEEG; KVIKFLEEGT; VIKFLEEGTN;

11 mers:
MRNKNIFKLFF; RNKNIFKLFFA; NKNIFKLFFAS; KNIFKLFFASM;
NIFKLFFASML; IFKLFFASMLF; FKLFFASMLFV; KLFFASMLFVM;

| | |
|---|---|
| | LFFASMLFVMA; FFASMLFVMAC; FASMLFVMACK; ASMLFVMACKA; SMLFVMACKAY; MLFVMACKAYV; LFVMACKAYVE; FVMACKAYVEE; VMACKAYVEEK; MACKAYVEEKK; ACKAYVEEKKE; CKAYVEEKKEI; KAYVEEKKEID; AYVEEKKEIDS; YVEEKKEIDSL; VEEKKEIDSLM; EEKKEIDSLME; EKKEIDSLMED; KKEIDSLMEDV; KEIDSLMEDVL; EIDSLMEDVLA; IDSLMEDVLAL; DSLMEDVLALV; SLMEDVLALVN; LMEDVLALVND; MEDVLALVNDS; EDVLALVNDSS; DVLALVNDSSG; VLALVNDSSGG; LALVNDSSGGK; ALVNDSSGGKF; LVNDSSGGKFK; VNDSSGGKFKD; NDSSGGKFKDY; DSSGGKFKDYK; SSGGKFKDYKD; SGGKFKDYKDK; GGKFKDYKDKI; GKFKDYKDKIN; KFKDYKDKINE; FKDYKDKINEL; KDYKDKINELK; DYKDKINELKE; YKDKINELKEN; KDKINELKENL; DKINELKENLK; KINELKENLKD; INELKENLKDI; NELKENLKDIG; ELKENLKDIGN; LKENLKDIGNA; KENLKDIGNAE; ENLKDIGNAEL; NLKDIGNAELK; LKDIGNAELKE; KDIGNAELKEK; DIGNAELKEKL; IGNAELKEKLL; GNAELKEKLLN; NAELKEKLLNL; AELKEKLLNLQ; ELKEKLLNLQN; LKEKLLNLQNS; KEKLLNLQNSF; EKLLNLQNSFQ; KLLNLQNSFQD; LLNLQNSFQDK; LNLQNSFQDKL; NLQNSFQDKLA; LQNSFQDKLAA; QNSFQDKLAAK; NSFQDKLAAKL; SFQDKLAAKLA; FQDKLAAKLAA; QDKLAAKLAAL; DKLAAKLAALK; KLAAKLAALKA; LAAKLAALKAA; AAKLAALKAAK; AKLAALKAAKN; KLAALKAAKNT; LAALKAAKNTI; AALKAAKNTIE; ALKAAKNTIEN; LKAAKNTIENI; KAAKNTIENIT; AAKNTIENITD; AKNTIENITDK; KNTIENITDKD; NTIENITDKDQ; TIENITDKDQD; IENITDKDQDI; ENITDKDQDIS; NITDKDQDISK; ITDKDQDISKR; TDKDQDISKRK; DKDQDISKRKI; KDQDISKRKIW; DQDISKRKIWS; QDISKRKIWSE; DISKRKIWSEA; ISKRKIWSEAK; SKRKIWSEAKL; KRKIWSEAKLV; RKIWSEAKLVG; KIWSEAKLVGV; IWSEAKLVGVT; WSEAKLVGVTV; SEAKLVGVTVP; EAKLVGVTVPL; AKLVGVTVPLL; KLVGVTVPLLG; LVGVTVPLLGS; VGVTVPLLGSN; GVTVPLLGSNT; VTVPLLGSNTS; TVPLLGSNTSG; VPLLGSNTSGN; PLLGSNTSGNG; LLGSNTSGNGD; LGSNTSGNGDK; GSNTSGNGDKM; SNTSGNGDKMS; NTSGNGDKMSK; TSGNGDKMSKN; SGNGDKMSKNA; GNGDKMSKNAV; NGDKMSKNAVE; GDKMSKNAVEQ; DKMSKNAVEQI; KMSKNAVEQID; MSKNAVEQIDK; SKNAVEQIDKV; KNAVEQIDKVI; NAVEQIDKVIK; AVEQIDKVIKF; VEQIDKVIKFL; EQIDKVIKFLE; QIDKVIKFLEE; IDKVIKFLEEG; DKVIKFLEEGT; KVIKFLEEGTN; |
| Seq 40 | SEQ ID NO 47213-48406<br>8 mers:<br>MKDNILKN; KDNILKNN; DNILKNNK; NILKNNKL; ILKNNKLI; LKNNKLIA; KNNKLIAI; NNKLIAIF; NKLIAIFL; KLIAIFLL; LIAIFLLH; IAIFLLHV; AIFLLHVL; IFLLHVLT; FLLHVLTV; LLHVLTVL; LHVLTVLI; HVLTVLIL; VLTVLILI; LTVLILIS; TVLILISC; VLILISCS; LILISCSL; ILISCSLE; LISCSLEV; ISCSLEVK; SCSLEVKD; CSLEVKDS; SLEVKDSN; LEVKDSNE; EVKDSNES; VKDSNESK; KDSNESKK; DSNESKKH; SNESKKHK; NESKKHKK; ESKKHKKE; SKKHKKEK; KKHKKEKR; KHKKEKRK; HKKEKRKG; KKEKRKGK; KEKRKGKV; EKRKGKVE; KRKGKVEN; RKGKVENL; KGKVENLL; GKVENLLV; KVENLLVA; VENLLVAI; ENLLVAIN; NLLVAINN; LLVAINNL; LVAINNLK; VAINNLKN; AINNLKNP; INNLKNPT; NNLKNPTK; NLKNPTKP; LKNPTKPA; |

KNPTKPAA; NPTKPAAG; PTKPAAGK; TKPAAGKN; KPAAGKNK;
PAAGKNKA; AAGKNKAN; AGKNKANS; GKNKANSK; KNKANSKA;
NKANSKAS; KANSKASK; ANSKASKQ; NSKASKQK; SKASKQKN;
KASKQKNN; ASKQKNNP; SKQKNNPN; KQKNNPNA; QKNNPNAN;
KNNPNANA; NNPNANAN; NPNANANN; PNANANNA; NANANNAP;
ANANNAPK; NANNAPKK; ANNAPKKI; NNAPKKIL; NAPKKILD;
APKKILDP; PKKILDPE; KKILDPEV; KILDPEVA; ILDPEVAK;
LDPEVAKL; DPEVAKLI; PEVAKLIQ; EVAKLIQK; VAKLIQKI;
AKLIQKIL; KLIQKILD; LIQKILDR; IQKILDRS; QKILDRSE;
KILDRSEN; ILDRSENI; LDRSENII; DRSENIIQ; RSENIIQI;
SENIIQIS; ENIIQISE; NIIQISEM; IIQISEMD; IQISEMDS;
QISEMDSS; ISEMDSSR; SEMDSSRG; EMDSSRGE; MDSSRGEP;
DSSRGEPN; SSRGEPND; SRGEPNDQ; RGEPNDQF; GEPNDQFG;
EPNDQFGM; PNDQFGMR; NDQFGMRA; DQFGMRAE; QFGMRAEI;
FGMRAEIF; GMRAEIFS; MRAEIFSK; RAEIFSKI; AEIFSKIF;
EIFSKIFF; IFSKIFFN; FSKIFFNA; SKIFFNAN; KIFFNANS;
IFFNANST; FFNANSTV; FNANSTVH; NANSTVHF; ANSTVHFD;
NSTVHFDS; STVHFDSH; TVHFDSHE; VHFDSHEY; HFDSHEYT;
FDSHEYTE; DSHEYTEE; SHEYTEER; HEYTEERR; EYTEERRM;
YTEERRML; TEERRMLY; EERRMLYT; ERRMLYTS; RRMLYTSL;
RMLYTSLN; MLYTSLNF; LYTSLNFN; YTSLNFNE; TSLNFNEG;
SLNFNEGK; LNFNEGKI; NFNEGKIF; FNEGKIFN; NEGKIFNL;
EGKIFNLG; GKIFNLGQ; KIFNLGQI; IFNLGQIL; FNLGQILS;
NLGQILSK; LGQILSKL; GQILSKLS; QILSKLSQ; ILSKLSQD;
LSKLSQDS; SKLSQDSN; KLSQDSNY; LSQDSNYR; SQDSNYRG;
QDSNYRGL; DSNYRGLV; SNYRGLVK; NYRGLVKE; YRGLVKET;
RGLVKETL; GLVKETLI; LVKETLIN; VKETLINR; KETLINRG;
ETLINRGF; TLINRGFS; LINRGFSI; INRGFSIQ; NRGFSIQL;
RGFSIQLA; GFSIQLAM; FSIQLAME; SIQLAMEE; IQLAMEEI;
QLAMEEIS; LAMEEISA; AMEEISAK; MEEISAKI; EEISAKIL;
EISAKILN; ISAKILNV; SAKILNVK; AKILNVKD; KILNVKDK;
ILNVKDKL; LNVKDKLQ; NVKDKLQQ; VKDKLQQL; KDKLQQLN;
DKLQQLNK; KLQQLNKP; LQQLNKPN; QQLNKPNL; QLNKPNLE;
LNKPNLET; NKPNLETL; KPNLETLY; PNLETLYN; NLETLYND;
LETLYNDF; ETLYNDFE; TLYNDFEK; LYNDFEKL; YNDFEKLT;
NDFEKLTS; DFEKLTSL; FEKLTSLK; EKLTSLKE; KLTSLKEK;
LTSLKEKW; TSLKEKWL; SLKEKWLK; LKEKWLKD; KEKWLKDT;
EKWLKDTD; KWLKDTDD; WLKDTDDL; LKDTDDLI; KDTDDLID;
DTDDLIDE; TDDLIDEY; DDLIDEYN; DLIDEYNT; LIDEYNTN;
IDEYNTNP; DEYNTNPD; EYNTNPDL; YNTNPDLQ; NTNPDLQT;
TNPDLQTD; NPDLQTDV; PDLQTDVS; DLQTDVSK; LQTDVSKL;
QTDVSKLN; TDVSKLND; DVSKLNDT; VSKLNDTL; SKLNDTLR;
KLNDTLRS; LNDTLRSK; NDTLRSKN; DTLRSKNS; TLRSKNSR;
LRSKNSRA; RSKNSRAQ; SKNSRAQF; KNSRAQFA; NSRAQFAN;
SRAQFANI; RAQFANIH; AQFANIHD; QFANIHDI; FANIHDII;
ANIHDIIL; NIHDIILD; IHDIILDL; HDIILDLV; DIILDLVN;
IILDLVNT; ILDLVNTT; LDLVNTTT; DLVNTTTN; LVNTTTNI;
VNTTTNIL; NTTTNILA; TTTNILAP; TTNILAPI; TNILAPIQ;

9 mers:
MKDNILKNN; KDNILKNNK; DNILKNNKL; NILKNNKLI; ILKNNKLIA;

LKNNKLIAI; KNNKLIAIF; NNKLIAIFL; NKLIAIFLL; KLIAIFLLH;
LIAIFLLHV; IAIFLLHVL; AIFLLHVLT; IFLLHVLTV; FLLHVLTVL;
LLHVLTVLI; LHVLTVLIL; HVLTVLILI; VLTVLILIS; LTVLILISC;
TVLILISCS; VLILISCSL; LILISCSLE; ILISCSLEV; LISCSLEVK;
ISCSLEVKD; SCSLEVKDS; CSLEVKDSN; SLEVKDSNE; LEVKDSNES;
EVKDSNESK; VKDSNESKK; KDSNESKKH; DSNESKKHK; SNESKKHKK;
NESKKHKKE; ESKKHKKEK; SKKHKKEKR; KKHKKEKRK; KHKKEKRKG;
HKKEKRKGK; KKEKRKGKV; KEKRKGKVE; EKRKGKVEN; KRKGKVENL;
RKGKVENLL; KGKVENLLV; GKVENLLVA; KVENLLVAI; VENLLVAIN;
ENLLVAINN; NLLVAINNL; LLVAINNLK; LVAINNLKN; VAINNLKNP;
AINNLKNPT; INNLKNPTK; NNLKNPTKP; NLKNPTKPA; LKNPTKPAA;
KNPTKPAAG; NPTKPAAGK; PTKPAAGKN; TKPAAGKNK; KPAAGKNKA;
PAAGKNKAN; AAGKNKANS; AGKNKANSK; GKNKANSKA; KNKANSKAS;
NKANSKASK; KANSKASKQ; ANSKASKQK; NSKASKQKN; SKASKQKNN;
KASKQKNNP; ASKQKNNPN; SKQKNNPNA; KQKNNPNAN; QKNNPNANA;
KNNPNANAN; NNPNANANN; NPNANANNA; PNANANNAP; NANANNAPK;
ANANNAPKK; NANNAPKKI; ANNAPKKIL; NNAPKKILD; NAPKKILDP;
APKKILDPE; PKKILDPEV; KKILDPEVA; KILDPEVAK; ILDPEVAKL;
LDPEVAKLI; DPEVAKLIQ; PEVAKLIQK; EVAKLIQKI; VAKLIQKIL;
AKLIQKILD; KLIQKILDR; LIQKILDRS; IQKILDRSE; QKILDRSEN;
KILDRSENI; ILDRSENII; LDRSENIIQ; DRSENIIQI; RSENIIQIS;
SENIIQISE; ENIIQISEM; NIIQISEMD; IIQISEMDS; IQISEMDSS;
QISEMDSSR; ISEMDSSRG; SEMDSSRGE; EMDSSRGEP; MDSSRGEPN;
DSSRGEPND; SSRGEPNDQ; SRGEPNDQF; RGEPNDQFG; GEPNDQFGM;
EPNDQFGMR; PNDQFGMRA; NDQFGMRAE; DQFGMRAEI; QFGMRAEIF;
FGMRAEIFS; GMRAEIFSK; MRAEIFSKI; RAEIFSKIF; AEIFSKIFF;
EIFSKIFFN; IFSKIFFNA; FSKIFFNAN; SKIFFNANS; KIFFNANST;
IFFNANSTV; FFNANSTVH; FNANSTVHF; NANSTVHFD; ANSTVHFDS;
NSTVHFDSH; STVHFDSHE; TVHFDSHEY; VHFDSHEYT; HFDSHEYTE;
FDSHEYTEE; DSHEYTEER; SHEYTEERR; HEYTEERRM; EYTEERRML;
YTEERRMLY; TEERRMLYT; EERRMLYTS; ERRMLYTSL; RRMLYTSLN;
RMLYTSLNF; MLYTSLNFN; LYTSLNFNE; YTSLNFNEG; TSLNFNEGK;
SLNFNEGKI; LNFNEGKIF; NFNEGKIFN; FNEGKIFNL; NEGKIFNLG;
EGKIFNLGQ; GKIFNLGQI; KIFNLGQIL; IFNLGQILS; FNLGQILSK;
NLGQILSKL; LGQILSKLS; GQILSKLSQ; QILSKLSQD; ILSKLSQDS;
LSKLSQDSN; SKLSQDSNY; KLSQDSNYR; LSQDSNYRG; SQDSNYRGL;
QDSNYRGLV; DSNYRGLVK; SNYRGLVKE; NYRGLVKET; YRGLVKETL;
RGLVKETLI; GLVKETLIN; LVKETLINR; VKETLINRG; KETLINRGF;
ETLINRGFS; TLINRGFSI; LINRGFSIQ; INRGFSIQL; NRGFSIQLA;
RGFSIQLAM; GFSIQLAME; FSIQLAMEE; SIQLAMEEI; IQLAMEEIS;
QLAMEEISA; LAMEEISAK; AMEEISAKI; MEEISAKIL; EEISAKILN;
EISAKILNV; ISAKILNVK; SAKILNVKD; AKILNVKDK; KILNVKDKL;
ILNVKDKLQ; LNVKDKLQQ; NVKDKLQQL; VKDKLQQLN; KDKLQQLNK;
DKLQQLNKP; KLQQLNKPN; LQQLNKPNL; QQLNKPNLE; QLNKPNLET;
LNKPNLETL; NKPNLETLY; KPNLETLYN; PNLETLYND; NLETLYNDF;
LETLYNDFE; ETLYNDFEK; TLYNDFEKL; LYNDFEKLT; YNDFEKLTS;
NDFEKLTSL; DFEKLTSLK; FEKLTSLKE; EKLTSLKEK; KLTSLKEKW;
LTSLKEKWL; TSLKEKWLK; SLKEKWLKD; LKEKWLKDT; KEKWLKDTD;
EKWLKDTDD; KWLKDTDDL; WLKDTDDLI; LKDTDDLID; KDTDDLIDE;
DTDDLIDEY; TDDLIDEYN; DDLIDEYNT; DLIDEYNTN; LIDEYNTNP;
IDEYNTNPD; DEYNTNPDL; EYNTNPDLQ; YNTNPDLQT; NTNPDLQTD;

TNPDLQTDV; NPDLQTDVS; PDLQTDVSK; DLQTDVSKL; LQTDVSKLN;
QTDVSKLND; TDVSKLNDT; DVSKLNDTL; VSKLNDTLR; SKLNDTLRS;
KLNDTLRSK; LNDTLRSKN; NDTLRSKNS; DTLRSKNSR; TLRSKNSRA;
LRSKNSRAQ; RSKNSRAQF; SKNSRAQFA; KNSRAQFAN; NSRAQFANI;
SRAQFANIH; RAQFANIHD; AQFANIHDI; QFANIHDII; FANIHDIIL;
ANIHDIILD; NIHDIILDL; IHDIILDLV; HDIILDLVN; DIILDLVNT;
IILDLVNTT; ILDLVNTTT; LDLVNTTTN; DLVNTTTNI; LVNTTTNIL;
VNTTTNILA; NTTTNILAP; TTTNILAPI; TTNILAPIQ;

10 mers:
MKDNILKNNK; KDNILKNNKL; DNILKNNKLI; NILKNNKLIA;
ILKNNKLIAI; LKNNKLIAIF; KNNKLIAIFL; NNKLIAIFLL;
NKLIAIFLLH; KLIAIFLLHV; LIAIFLLHVL; IAIFLLHVLT;
AIFLLHVLTV; IFLLHVLTVL; FLLHVLTVLI; LLHVLTVLIL;
LHVLTVLILI; HVLTVLILIS; VLTVLILISC; LTVLILISCS;
TVLILISCSL; VLILISCSLE; LILISCSLEV; ILISCSLEVK;
LISCSLEVKD; ISCSLEVKDS; SCSLEVKDSN; CSLEVKDSNE;
SLEVKDSNES; LEVKDSNESK; EVKDSNESKK; VKDSNESKKH;
KDSNESKKHK; DSNESKKHKK; SNESKKHKKE; NESKKHKKEK;
ESKKHKKEKR; SKKHKKEKRK; KKHKKEKRKG; KHKKEKRKGK;
HKKEKRKGKV; KKEKRKGKVE; KEKRKGKVEN; EKRKGKVENL;
KRKGKVENLL; RKGKVENLLV; KGKVENLLVA; GKVENLLVAI;
KVENLLVAIN; VENLLVAINN; ENLLVAINNL; NLLVAINNLK;
LLVAINNLKN; LVAINNLKNP; VAINNLKNPT; AINNLKNPTK;
INNLKNPTKP; NNLKNPTKPA; NLKNPTKPAA; LKNPTKPAAG;
KNPTKPAAGK; NPTKPAAGKN; PTKPAAGKNK; TKPAAGKNKA;
KPAAGKNKAN; PAAGKNKANS; AAGKNKANSK; AGKNKANSKA;
GKNKANSKAS; KNKANSKASK; NKANSKASKQ; KANSKASKQK;
ANSKASKQKN; NSKASKQKNN; SKASKQKNNP; KASKQKNNPN;
ASKQKNNPNA; SKQKNNPNAN; KQKNNPNANA; QKNNPNANAN;
KNNPNANANN; NNPNANANNA; NPNANANNAP; PNANANNAPK;
NANANNAPKK; ANANNAPKKI; NANNAPKKIL; ANNAPKKILD;
NNAPKKILDP; NAPKKILDPE; APKKILDPEV; PKKILDPEVA;
KKILDPEVAK; KILDPEVAKL; ILDPEVAKLI; LDPEVAKLIQ;
DPEVAKLIQK; PEVAKLIQKI; EVAKLIQKIL; VAKLIQKILD;
AKLIQKILDR; KLIQKILDRS; LIQKILDRSE; IQKILDRSEN;
QKILDRSENI; KILDRSENII; ILDRSENIIQ; LDRSENIIQI;
DRSENIIQIS; RSENIIQISE; SENIIQISEM; ENIIQISEMD;
NIIQISEMDS; IIQISEMDSS; IQISEMDSSR; QISEMDSSRG;
ISEMDSSRGE; SEMDSSRGEP; EMDSSRGEPN; MDSSRGEPND;
DSSRGEPNDQ; SSRGEPNDQF; SRGEPNDQFG; RGEPNDQFGM;
GEPNDQFGMR; EPNDQFGMRA; PNDQFGMRAE; NDQFGMRAEI;
DQFGMRAEIF; QFGMRAEIFS; FGMRAEIFSK; GMRAEIFSKI;
MRAEIFSKIF; RAEIFSKIFF; AEIFSKIFFN; EIFSKIFFNA;
IFSKIFFNAN; FSKIFFNANS; SKIFFNANST; KIFFNANSTV;
IFFNANSTVH; FFNANSTVHF; FNANSTVHFD; NANSTVHFDS;
ANSTVHFDSH; NSTVHFDSHE; STVHFDSHEY; TVHFDSHEYT;
VHFDSHEYTE; HFDSHEYTEE; FDSHEYTEER; DSHEYTEERR;
SHEYTEERRM; HEYTEERRML; EYTEERRMLY; YTEERRMLYT;
TEERRMLYTS; EERRMLYTSL; ERRMLYTSLN; RRMLYTSLNF;
RMLYTSLNFN; MLYTSLNFNE; LYTSLNFNEG; YTSLNFNEGK;

TSLNFNEGKI; SLNFNEGKIF; LNFNEGKIFN; NFNEGKIFNL;
FNEGKIFNLG; NEGKIFNLGQ; EGKIFNLGQI; GKIFNLGQIL;
KIFNLGQILS; IFNLGQILSK; FNLGQILSKL; NLGQILSKLS;
LGQILSKLSQ; GQILSKLSQD; QILSKLSQDS; ILSKLSQDSN;
LSKLSQDSNY; SKLSQDSNYR; KLSQDSNYRG; LSQDSNYRGL;
SQDSNYRGLV; QDSNYRGLVK; DSNYRGLVKE; SNYRGLVKET;
NYRGLVKETL; YRGLVKETLI; RGLVKETLIN; GLVKETLINR;
LVKETLINRG; VKETLINRGF; KETLINRGFS; ETLINRGFSI;
TLINRGFSIQ; LINRGFSIQL; INRGFSIQLA; NRGFSIQLAM;
RGFSIQLAME; GFSIQLAMEE; FSIQLAMEEI; SIQLAMEEIS;
IQLAMEEISA; QLAMEEISAK; LAMEEISAKI; AMEEISAKIL;
MEEISAKILN; EEISAKILNV; EISAKILNVK; ISAKILNVKD;
SAKILNVKDK; AKILNVKDKL; KILNVKDKLQ; ILNVKDKLQQ;
LNVKDKLQQL; NVKDKLQQLN; VKDKLQQLNK; KDKLQQLNKP;
DKLQQLNKPN; KLQQLNKPNL; LQQLNKPNLE; QQLNKPNLET;
QLNKPNLETL; LNKPNLETLY; NKPNLETLYN; KPNLETLYND;
PNLETLYNDF; NLETLYNDFE; LETLYNDFEK; ETLYNDFEKL;
TLYNDFEKLT; LYNDFEKLTS; YNDFEKLTSL; NDFEKLTSLK;
DFEKLTSLKE; FEKLTSLKEK; EKLTSLKEKW; KLTSLKEKWL;
LTSLKEKWLK; TSLKEKWLKD; SLKEKWLKDT; LKEKWLKDTD;
KEKWLKDTDD; EKWLKDTDDL; KWLKDTDDLI; WLKDTDDLID;
LKDTDDLIDE; KDTDDLIDEY; DTDDLIDEYN; TDDLIDEYNT;
DDLIDEYNTN; DLIDEYNTNP; LIDEYNTNPD; IDEYNTNPDL;
DEYNTNPDLQ; EYNTNPDLQT; YNTNPDLQTD; NTNPDLQTDV;
TNPDLQTDVS; NPDLQTDVSK; PDLQTDVSKL; DLQTDVSKLN;
LQTDVSKLND; QTDVSKLNDT; TDVSKLNDTL; DVSKLNDTLR;
VSKLNDTLRS; SKLNDTLRSK; KLNDTLRSKN; LNDTLRSKNS;
NDTLRSKNSR; DTLRSKNSRA; TLRSKNSRAQ; LRSKNSRAQF;
RSKNSRAQFA; SKNSRAQFAN; KNSRAQFANI; NSRAQFANIH;
SRAQFANIHD; RAQFANIHDI; AQFANIHDII; QFANIHDIIL;
FANIHDIILD; ANIHDIILDL; NIHDIILDLV; IHDIILDLVN;
HDIILDLVNT; DIILDLVNTT; IILDLVNTTT; ILDLVNTTTN;
LDLVNTTTNI; DLVNTTTNIL; LVNTTTNILA; VNTTTNILAP;
NTTTNILAPI; TTTNILAPIQ;

11 mers:
MKDNILKNNKL; KDNILKNNKLI; DNILKNNKLIA; NILKNNKLIAI;
ILKNNKLIAIF; LKNNKLIAIFL; KNNKLIAIFLL; NNKLIAIFLLH;
NKLIAIFLLHV; KLIAIFLLHVL; LIAIFLLHVLT; IAIFLLHVLTV;
AIFLLHVLTVL; IFLLHVLTVLI; FLLHVLTVLIL; LLHVLTVLILI;
LHVLTVLILIS; HVLTVLILISC; VLTVLILISCS; LTVLILISCSL;
TVLILISCSLE; VLILISCSLEV; LILISCSLEVK; ILISCSLEVKD;
LISCSLEVKDS; ISCSLEVKDSN; SCSLEVKDSNE; CSLEVKDSNES;
SLEVKDSNESK; LEVKDSNESKK; EVKDSNESKKH; VKDSNESKKHK;
KDSNESKKHKK; DSNESKKHKKE; SNESKKHKKEK; NESKKHKKEKR;
ESKKHKKEKRK; SKKHKKEKRKG; KKHKKEKRKGK; KHKKEKRKGKV;
HKKEKRKGKVE; KKEKRKGKVEN; KEKRKGKVENL; EKRKGKVENLL;
KRKGKVENLLV; RKGKVENLLVA; KGKVENLLVAI; GKVENLLVAIN;
KVENLLVAINN; VENLLVAINNL; ENLLVAINNLK; NLLVAINNLKN;
LLVAINNLKNP; LVAINNLKNPT; VAINNLKNPTK; AINNLKNPTKP;
INNLKNPTKPA; NNLKNPTKPAA; NLKNPTKPAAG; LKNPTKPAAGK;

| | | | |
|---|---|---|---|
| KNPTKPAAGKN; | NPTKPAAGKNK; | PTKPAAGKNKA; | TKPAAGKNKAN; |
| KPAAGKNKANS; | PAAGKNKANSK; | AAGKNKANSKA; | AGKNKANSKAS; |
| GKNKANSKASK; | KNKANSKASKQ; | NKANSKASKQK; | KANSKASKQKN; |
| ANSKASKQKNN; | NSKASKQKNNP; | SKASKQKNNPN; | KASKQKNNPNA; |
| ASKQKNNPNAN; | SKQKNNPNANA; | KQKNNPNANAN; | QKNNPNANANN; |
| KNNPNANANNA; | NNPNANANNAP; | NPNANANNAPK; | PNANANNAPKK; |
| NANANNAPKKI; | ANANNAPKKIL; | NANNAPKKILD; | ANNAPKKILDP; |
| NNAPKKILDPE; | NAPKKILDPEV; | APKKILDPEVA; | PKKILDPEVAK; |
| KKILDPEVAKL; | KILDPEVAKLI; | ILDPEVAKLIQ; | LDPEVAKLIQK; |
| DPEVAKLIQKI; | PEVAKLIQKIL; | EVAKLIQKILD; | VAKLIQKILDR; |
| AKLIQKILDRS; | KLIQKILDRSE; | LIQKILDRSEN; | IQKILDRSENI; |
| QKILDRSENII; | KILDRSENIIQ; | ILDRSENIIQI; | LDRSENIIQIS; |
| DRSENIIQISE; | RSENIIQISEM; | SENIIQISEMD; | ENIIQISEMDS; |
| NIIQISEMDSS; | IIQISEMDSSR; | IQISEMDSSRG; | QISEMDSSRGE; |
| ISEMDSSRGEP; | SEMDSSRGEPN; | EMDSSRGEPND; | MDSSRGEPNDQ; |
| DSSRGEPNDQF; | SSRGEPNDQFG; | SRGEPNDQFGM; | RGEPNDQFGMR; |
| GEPNDQFGMRA; | EPNDQFGMRAE; | PNDQFGMRAEI; | NDQFGMRAEIF; |
| DQFGMRAEIFS; | QFGMRAEIFSK; | FGMRAEIFSKI; | GMRAEIFSKIF; |
| MRAEIFSKIFF; | RAEIFSKIFFN; | AEIFSKIFFNA; | EIFSKIFFNAN; |
| IFSKIFFNANS; | FSKIFFNANST; | SKIFFNANSTV; | KIFFNANSTVH; |
| IFFNANSTVHF; | FFNANSTVHFD; | FNANSTVHFDS; | NANSTVHFDSH; |
| ANSTVHFDSHE; | NSTVHFDSHEY; | STVHFDSHEYT; | TVHFDSHEYTE; |
| VHFDSHEYTEE; | HFDSHEYTEER; | FDSHEYTEERR; | DSHEYTEERRM; |
| SHEYTEERRML; | HEYTEERRMLY; | EYTEERRMLYT; | YTEERRMLYTS; |
| TEERRMLYTSL; | EERRMLYTSLN; | ERRMLYTSLNF; | RRMLYTSLNFN; |
| RMLYTSLNFNE; | MLYTSLNFNEG; | LYTSLNFNEGK; | YTSLNFNEGKI; |
| TSLNFNEGKIF; | SLNFNEGKIFN; | LNFNEGKIFNL; | NFNEGKIFNLG; |
| FNEGKIFNLGQ; | NEGKIFNLGQI; | EGKIFNLGQIL; | GKIFNLGQILS; |
| KIFNLGQILSK; | IFNLGQILSKL; | FNLGQILSKLS; | NLGQILSKLSQ; |
| LGQILSKLSQD; | GQILSKLSQDS; | QILSKLSQDSN; | ILSKLSQDSNY; |
| LSKLSQDSNYR; | SKLSQDSNYRG; | KLSQDSNYRGL; | LSQDSNYRGLV; |
| SQDSNYRGLVK; | QDSNYRGLVKE; | DSNYRGLVKET; | SNYRGLVKETL; |
| NYRGLVKETLI; | YRGLVKETLIN; | RGLVKETLINR; | GLVKETLINRG; |
| LVKETLINRGF; | VKETLINRGFS; | KETLINRGFSI; | ETLINRGFSIQ; |
| TLINRGFSIQL; | LINRGFSIQLA; | INRGFSIQLAM; | NRGFSIQLAME; |
| RGFSIQLAMEE; | GFSIQLAMEEI; | FSIQLAMEEIS; | SIQLAMEEISA; |
| IQLAMEEISAK; | QLAMEEISAKI; | LAMEEISAKIL; | AMEEISAKILN; |
| MEEISAKILNV; | EEISAKILNVK; | EISAKILNVKD; | ISAKILNVKDK; |
| SAKILNVKDKL; | AKILNVKDKLQ; | KILNVKDKLQQ; | ILNVKDKLQQL; |
| LNVKDKLQQLN; | NVKDKLQQLNK; | VKDKLQQLNKP; | KDKLQQLNKPN; |
| DKLQQLNKPNL; | KLQQLNKPNLE; | LQQLNKPNLET; | QQLNKPNLETL; |
| QLNKPNLETLY; | LNKPNLETLYN; | NKPNLETLYND; | KPNLETLYNDF; |
| PNLETLYNDFE; | NLETLYNDFEK; | LETLYNDFEKL; | ETLYNDFEKLT; |
| TLYNDFEKLTS; | LYNDFEKLTSL; | YNDFEKLTSLK; | NDFEKLTSLKE; |
| DFEKLTSLKEK; | FEKLTSLKEKW; | EKLTSLKEKWL; | KLTSLKEKWLK; |
| LTSLKEKWLKD; | TSLKEKWLKDT; | SLKEKWLKDTD; | LKEKWLKDTDD; |
| KEKWLKDTDDL; | EKWLKDTDDLI; | KWLKDTDDLID; | WLKDTDDLIDE; |
| LKDTDDLIDEY; | KDTDDLIDEYN; | DTDDLIDEYNT; | TDDLIDEYNTN; |
| DDLIDEYNTNP; | DLIDEYNTNPD; | LIDEYNTNPDL; | IDEYNTNPDLQ; |
| DEYNTNPDLQT; | EYNTNPDLQTD; | YNTNPDLQTDV; | NTNPDLQTDVS; |
| TNPDLQTDVSK; | NPDLQTDVSKL; | PDLQTDVSKLN; | DLQTDVSKLND; |

| | |
|---|---|
| | LQTDVSKLNDT; QTDVSKLNDTL; TDVSKLNDTLR; DVSKLNDTLRS; VSKLNDTLRSK; SKLNDTLRSKN; KLNDTLRSKNS; LNDTLRSKNSR; NDTLRSKNSRA; DTLRSKNSRAQ; TLRSKNSRAQF; LRSKNSRAQFA; RSKNSRAQFAN; SKNSRAQFANI; KNSRAQFANIH; NSRAQFANIHD; SRAQFANIHDI; RAQFANIHDII; AQFANIHDIIL; QFANIHDIILD; FANIHDIILDL; ANIHDIILDLV; NIHDIILDLVN; IHDIILDLVNT; HDIILDLVNTT; DIILDLVNTTT; IILDLVNTTTN; ILDLVNTTTNI; LDLVNTTTNIL; DLVNTTTNILA; LVNTTTNILAP; VNTTTNILAPI; NTTTNILAPIQ; |
| Seq 41 | SEQ ID NO 48407-49644/ <br> 8 mers: <br> MKNNKLIA; KNNKLIAI; NNKLIAIF; NKLIAIFL; KLIAIFLL; LIAIFLLH; IAIFLLHI; AIFLLHIL; IFLLHILT; FLLHILTG; LLHILTGL; LHILTGLI; HILTGLIL; ILTGLILL; LTGLILLS; TGLILLSC; GLILLSCS; LILLSCSL; ILLSCSLE; LLSCSLEV; LSCSLEVN; SCSLEVNQ; CSLEVNQD; SLEVNQDD; LEVNQDDN; EVNQDDNQ; VNQDDNQE; NQDDNQEK; QDDNQEKQ; DDNQEKQK; DNQEKQKK; NQEKQKKA; QEKQKKAK; EKQKKAKT; KQKKAKTK; QKKAKTKT; KKAKTKTS; KAKTKTSK; AKTKTSKS; KTKTSKSE; TKTSKSEN; KTSKSENN; TSKSENNS; SKSENNSS; KSENNSSK; SENNSSKM; ENNSSKMK; NNSSKMKK; NSSKMKKL; SSKMKKLS; SKMKKLSK; KMKKLSKN; MKKLSKNA; KKLSKNAK; KLSKNAKN; LSKNAKNK; SKNAKNKK; KNAKNKKP; NAKNKKPT; AKNKKPTV; KNKKPTVD; NKKPTVDN; KKPTVDNL; KPTVDNLL; PTVDNLLV; TVDNLLVA; VDNLLVAI; DNLLVAIN; NLLVAINT; LLVAINTL; LVAINTLK; VAINTLKN; AINTLKNP; INTLKNPP; NTLKNPPK; TLKNPPKT; LKNPPKTA; KNPPKTAG; NPPKTAGK; PPKTAGKN; PKTAGKNK; KTAGKNKS; TAGKNKSN; AGKNKSNS; GKNKSNSA; KNKSNSAA; NKSNSAAA; KSNSAAAL; SNSAAALK; NSAAALKQ; SAAALKQP; AAALKQPN; AALKQPNN; ALKQPNNA; LKQPNNAN; KQPNNANA; QPNNANAL; PNNANALK; NNANALKQ; NANALKQI; ANALKQID; NALKQIDP; ALKQIDPE; LKQIDPEA; KQIDPEAK; QIDPEAKE; IDPEAKEL; DPEAKELI; PEAKELIQ; EAKELIQK; AKELIQKI; KELIQKIL; ELIQKILE; LIQKILER; IQKILERS; QKILERSE; KILERSED; ILERSEDI; LERSEDIV; ERSEDIVQ; RSEDIVQI; SEDIVQIS; EDIVQISE; DIVQISEI; IVQISEID; VQISEIDA; QISEIDAN; ISEIDANK; SEIDANKG; EIDANKGE; IDANKGEP; DANKGEPD; ANKGEPDD; NKGEPDDQ; KGEPDDQF; GEPDDQFE; EPDDQFEM; PDDQFEMK; DDQFEMKA; DQFEMKAE; QFEMKAEI; FEMKAEIF; EMKAEIFS; MKAEIFSK; KAEIFSKI; AEIFSKIF; EIFSKIFF; IFSKIFFN; FSKIFFNA; SKIFFNAG; KIFFNAGS; IFFNAGST; FFNAGSTV; FNAGSTVT; NAGSTVTF; AGSTVTFD; GSTVTFDD; STVTFDDN; TVTFDDNE; VTFDDNEY; TFDDNEYV; FDDNEYVN; DDNEYVNE; DNEYVNER; NEYVNERR; EYVNERRI; YVNERRIL; VNERRILY; NERRILYT; ERRILYTS; RRILYTSL; RILYTSLN; ILYTSLNF; LYTSLNFN; YTSLNFNE; TSLNFNEN; SLNFNENK; LNFNENKI; NFNENKIL; FNENKILN; NENKILNL; ENKILNLG; NKILNLGK; KILNLGKI; ILNLGKIL; LNLGKILS; NLGKILSK; LGKILSKL; GKILSKLS; KILSKLSQ; ILSKLSQD; LSKLSQDS; SKLSQDSN; KLSQDSNY; LSQDSNYR; |

SQDSNYRS; QDSNYRSL; DSNYRSLV; SNYRSLVK; NYRSLVKE;
YRSLVKEI; RSLVKEIL; SLVKEILI; LVKEILIN; VKEILINR;
KEILINRG; EILINRGF; ILINRGFS; LINRGFSI; INRGFSIQ;
NRGFSIQL; RGFSIQLA; GFSIQLAI; FSIQLAIE; SIQLAIEE;
IQLAIEEI; QLAIEEIS; LAIEEISL; AIEEISLR; IEEISLRT;
EEISLRTL; EISLRTLN; ISLRTLNV; SLRTLNVK; LRTLNVKD;
RTLNVKDK; TLNVKDKI; LNVKDKIQ; NVKDKIQH; VKDKIQHL;
KDKIQHLN; DKIQHLNK; KIQHLNKP; IQHLNKPN; QHLNKPNL;
HLNKPNLK; LNKPNLKT; NKPNLKTL; KPNLKTLY; PNLKTLYH;
NLKTLYHD; LKTLYHDF; KTLYHDFN; TLYHDFNK; LYHDFNKL;
YHDFNKLI; HDFNKLIP; DFNKLIPL; FNKLIPLK; NKLIPLKE;
KLIPLKEK; LIPLKEKW; IPLKEKWL; PLKEKWLK; LKEKWLKD;
KEKWLKDV; EKWLKDVD; KWLKDVDD; WLKDVDDI; LKDVDDII;
KDVDDIIK; DVDDIIKD; VDDIIKDY; DDIIKDYN; DIIKDYNA;
IIKDYNAN; IKDYNANP; KDYNANPE; DYNANPEL; YNANPELR;
NANPELRT; ANPELRTD; NPELRTDI; PELRTDIS; ELRTDISK;
LRTDISKL; RTDISKLN; TDISKLND; DISKLNDY; ISKLNDYI;
SKLNDYII; KLNDYIIS; LNDYIISK; NDYIISKN; DYIISKNS;
YIISKNSK; IISKNSKA; ISKNSKAQ; SKNSKAQF; KNSKAQFT;
NSKAQFTD; SKAQFTDI; KAQFTDIH; AQFTDIHN; QFTDIHNI;
FTDIHNII; TDIHNIIL; DIHNIILN; IHNIILNL; HNIILNLI;
NIILNLIN; IILNLINT; ILNLINTT; LNLINTTT; NLINTTTN;
LINTTTNI; INTTTNIL; NTTTNILA; TTTNILAP; TTNILAPI;
TNILAPIQ;

9 mers:
MKNNKLIAI; KNNKLIAIF; NNKLIAIFL; NKLIAIFLL; KLIAIFLLH;
LIAIFLLHI; IAIFLLHIL; AIFLLHILT; IFLLHILTG; FLLHILTGL;
LLHILTGLI; LHILTGLIL; HILTGLILL; ILTGLILLS; LTGLILLSC;
TGLILLSCS; GLILLSCSL; LILLSCSLE; ILLSCSLEV; LLSCSLEVN;
LSCSLEVNQ; SCSLEVNQD; CSLEVNQDD; SLEVNQDDN; LEVNQDDNQ;
EVNQDDNQE; VNQDDNQEK; NQDDNQEKQ; QDDNQEKQK; DDNQEKQKK;
DNQEKQKKA; NQEKQKKAK; QEKQKKAKT; EKQKKAKTK; KQKKAKTKT;
QKKAKTKTS; KKAKTKTSK; KAKTKTSKS; AKTKTSKSE; KTKTSKSEN;
TKTSKSENN; KTSKSENNS; TSKSENNSS; SKSENNSSK; KSENNSSKM;
SENNSSKMK; ENNSSKMKK; NNSSKMKKL; NSSKMKKLS; SSKMKKLSK;
SKMKKLSKN; KMKKLSKNA; MKKLSKNAK; KKLSKNAKN; KLSKNAKNK;
LSKNAKNKK; SKNAKNKKP; KNAKNKKPT; NAKNKKPTV; AKNKKPTVD;
KNKKPTVDN; NKKPTVDNL; KKPTVDNLL; KPTVDNLLV; PTVDNLLVA;
TVDNLLVAI; VDNLLVAIN; DNLLVAINT; NLLVAINTL; LLVAINTLK;
LVAINTLKN; VAINTLKNP; AINTLKNPP; INTLKNPPK; NTLKNPPKT;
TLKNPPKTA; LKNPPKTAG; KNPPKTAGK; NPPKTAGKN; PPKTAGKNK;
PKTAGKNKS; KTAGKNKSN; TAGKNKSNS; AGKNKSNSA; GKNKSNSAA;
KNKSNSAAA; NKSNSAAAL; KSNSAAALK; SNSAAALKQ; NSAAALKQP;
SAAALKQPN; AAALKQPNN; AALKQPNNA; ALKQPNNAN; LKQPNNANA;
KQPNNANAL; QPNNANALK; PNNANALKQ; NNANALKQI; NANALKQID;
ANALKQIDP; NALKQIDPE; ALKQIDPEA; LKQIDPEAK; KQIDPEAKE;
QIDPEAKEL; IDPEAKELI; DPEAKELIQ; PEAKELIQK; EAKELIQKI;
AKELIQKIL; KELIQKILE; ELIQKILER; LIQKILERS; IQKILERSE;
QKILERSED; KILERSEDI; ILERSEDIV; LERSEDIVQ; ERSEDIVQI;
RSEDIVQIS; SEDIVQISE; EDIVQISEI; DIVQISEID; IVQISEIDA;

VQISEIDAN; QISEIDANK; ISEIDANKG; SEIDANKGE; EIDANKGEP;
IDANKGEPD; DANKGEPDD; ANKGEPDDQ; NKGEPDDQF; KGEPDDQFE;
GEPDDQFEM; EPDDQFEMK; PDDQFEMKA; DDQFEMKAE; DQFEMKAEI;
QFEMKAEIF; FEMKAEIFS; EMKAEIFSK; MKAEIFSKI; KAEIFSKIF;
AEIFSKIFF; EIFSKIFFN; IFSKIFFNA; FSKIFFNAG; SKIFFNAGS;
KIFFNAGST; IFFNAGSTV; FFNAGSTVT; FNAGSTVTF; NAGSTVTFD;
AGSTVTFDD; GSTVTFDDN; STVTFDDNE; TVTFDDNEY; VTFDDNEYV;
TFDDNEYVN; FDDNEYVNE; DDNEYVNER; DNEYVNERR; NEYVNERRI;
EYVNERRIL; YVNERRILY; VNERRILYT; NERRILYTS; ERRILYTSL;
RRILYTSLN; RILYTSLNF; ILYTSLNFN; LYTSLNFNE; YTSLNFNEN;
TSLNFNENK; SLNFNENKI; LNFNENKIL; NFNENKILN; FNENKILNL;
NENKILNLG; ENKILNLGK; NKILNLGKI; KILNLGKIL; ILNLGKILS;
LNLGKILSK; NLGKILSKL; LGKILSKLS; GKILSKLSQ; KILSKLSQD;
ILSKLSQDS; LSKLSQDSN; SKLSQDSNY; KLSQDSNYR; LSQDSNYRS;
SQDSNYRSL; QDSNYRSLV; DSNYRSLVK; SNYRSLVKE; NYRSLVKEI;
YRSLVKEIL; RSLVKEILI; SLVKEILIN; LVKEILINR; VKEILINRG;
KEILINRGF; EILINRGFS; ILINRGFSI; LINRGFSIQ; INRGFSIQL;
NRGFSIQLA; RGFSIQLAI; GFSIQLAIE; FSIQLAIEE; SIQLAIEEI;
IQLAIEEIS; QLAIEEISL; LAIEEISLR; AIEEISLRT; IEEISLRTL;
EEISLRTLN; EISLRTLNV; ISLRTLNVK; SLRTLNVKD; LRTLNVKDK;
RTLNVKDKI; TLNVKDKIQ; LNVKDKIQH; NVKDKIQHL; VKDKIQHLN;
KDKIQHLNK; DKIQHLNKP; KIQHLNKPN; IQHLNKPNL; QHLNKPNLK;
HLNKPNLKT; LNKPNLKTL; NKPNLKTLY; KPNLKTLYH; PNLKTLYHD;
NLKTLYHDF; LKTLYHDFN; KTLYHDFNK; TLYHDFNKL; LYHDFNKLI;
YHDFNKLIP; HDFNKLIPL; DFNKLIPLK; FNKLIPLKE; NKLIPLKEK;
KLIPLKEKW; LIPLKEKWL; IPLKEKWLK; PLKEKWLKD; LKEKWLKDV;
KEKWLKDVD; EKWLKDVDD; KWLKDVDDI; WLKDVDDII; LKDVDDIIK;
KDVDDIIKD; DVDDIIKDY; VDDIIKDYN; DDIIKDYNA; DIIKDYNAN;
IIKDYNANP; IKDYNANPE; KDYNANPEL; DYNANPELR; YNANPELRT;
NANPELRTD; ANPELRTDI; NPELRTDIS; PELRTDISK; ELRTDISKL;
LRTDISKLN; RTDISKLND; TDISKLNDY; DISKLNDYI; ISKLNDYII;
SKLNDYIIS; KLNDYIISK; LNDYIISKN; NDYIISKNS; DYIISKNSK;
YIISKNSKA; IISKNSKAQ; ISKNSKAQF; SKNSKAQFT; KNSKAQFTD;
NSKAQFTDI; SKAQFTDIH; KAQFTDIHN; AQFTDIHNI; QFTDIHNII;
FTDIHNIIL; TDIHNIILN; DIHNIILNL; IHNIILNLI; HNIILNLIN;
NIILNLINT; IILNLINTT; ILNLINTTT; LNLINTTTN; NLINTTTNI;
LINTTTNIL; INTTTNILA; NTTTNILAP; TTTNILAPI; TTNILAPIQ;

10 mers:
MKNNKLIAIF; KNNKLIAIFL; NNKLIAIFLL; NKLIAIFLLH;
KLIAIFLLHI; LIAIFLLHIL; IAIFLLHILT; AIFLLHILTG;
IFLLHILTGL; FLLHILTGLI; LLHILTGLIL; LHILTGLILL;
HILTGLILLS; ILTGLILLSC; LTGLILLSCS; TGLILLSCSL;
GLILLSCSLE; LILLSCSLEV; ILLSCSLEVN; LLSCSLEVNQ;
LSCSLEVNQD; SCSLEVNQDD; CSLEVNQDDN; SLEVNQDDNQ;
LEVNQDDNQE; EVNQDDNQEK; VNQDDNQEKQ; NQDDNQEKQK;
QDDNQEKQKK; DDNQEKQKKA; DNQEKQKKAK; NQEKQKKAKT;
QEKQKKAKTK; EKQKKAKTKT; KQKKAKTKTS; QKKAKTKTSK;
KKAKTKTSKS; KAKTKTSKSE; AKTKTSKSEN; KTKTSKSENN;
TKTSKSENNS; KTSKSENNSS; TSKSENNSSK; SKSENNSSKM;
KSENNSSKMK; SENNSSKMKK; ENNSSKMKKL; NNSSKMKKLS;

| | |
|---|---|
| | NSSKMKKLSK; SSKMKKLSKN; SKMKKLSKNA; KMKKLSKNAK; MKKLSKNAKN; KKLSKNAKNK; KLSKNAKNKK; LSKNAKNKKP; SKNAKNKKPT; KNAKNKKPTV; NAKNKKPTVD; AKNKKPTVDN; KNKKPTVDNL; NKKPTVDNLL; KKPTVDNLLV; KPTVDNLLVA; PTVDNLLVAI; TVDNLLVAIN; VDNLLVAINT; DNLLVAINTL; NLLVAINTLK; LLVAINTLKN; LVAINTLKNP; VAINTLKNPP; AINTLKNPPK; INTLKNPPKT; NTLKNPPKTA; TLKNPPKTAG; LKNPPKTAGK; KNPPKTAGKN; NPPKTAGKNK; PPKTAGKNKS; PKTAGKNKSN; KTAGKNKSNS; TAGKNKSNSA; AGKNKSNSAA; GKNKSNSAAA; KNKSNSAAAL; NKSNSAAALK; KSNSAAALKQ; SNSAAALKQP; NSAAALKQPN; SAAALKQPNN; AAALKQPNNA; AALKQPNNAN; ALKQPNNANA; LKQPNNANAL; KQPNNANALK; QPNNANALKQ; PNNANALKQI; NNANALKQID; NANALKQIDP; ANALKQIDPE; NALKQIDPEA; ALKQIDPEAK; LKQIDPEAKE; KQIDPEAKEL; QIDPEAKELI; IDPEAKELIQ; DPEAKELIQK; PEAKELIQKI; EAKELIQKIL; AKELIQKILE; KELIQKILER; ELIQKILERS; LIQKILERSE; IQKILERSED; QKILERSEDI; KILERSEDIV; ILERSEDIVQ; LERSEDIVQI; ERSEDIVQIS; RSEDIVQISE; SEDIVQISEI; EDIVQISEID; DIVQISEIDA; IVQISEIDAN; VQISEIDANK; QISEIDANKG; ISEIDANKGE; SEIDANKGEP; EIDANKGEPD; IDANKGEPDD; DANKGEPDDQ; ANKGEPDDQF; NKGEPDDQFE; KGEPDDQFEM; GEPDDQFEMK; EPDDQFEMKA; PDDQFEMKAE; DDQFEMKAEI; DQFEMKAEIF; QFEMKAEIFS; FEMKAEIFSK; EMKAEIFSKI; MKAEIFSKIF; KAEIFSKIFF; AEIFSKIFFN; EIFSKIFFNA; IFSKIFFNAG; FSKIFFNAGS; SKIFFNAGST; KIFFNAGSTV; IFFNAGSTVT; FFNAGSTVTF; FNAGSTVTFD; NAGSTVTFDD; AGSTVTFDDN; GSTVTFDDNE; STVTFDDNEY; TVTFDDNEYV; VTFDDNEYVN; TFDDNEYVNE; FDDNEYVNER; DDNEYVNERR; DNEYVNERRI; NEYVNERRIL; EYVNERRILY; YVNERRILYT; VNERRILYTS; NERRILYTSL; ERRILYTSLN; RRILYTSLNF; RILYTSLNFN; ILYTSLNFNE; LYTSLNFNEN; YTSLNFNENK; TSLNFNENKI; SLNFNENKIL; LNFNENKILN; NFNENKILNL; FNENKILNLG; NENKILNLGK; ENKILNLGKI; NKILNLGKIL; KILNLGKILS; ILNLGKILSK; LNLGKILSKL; NLGKILSKLS; LGKILSKLSQ; GKILSKLSQD; KILSKLSQDS; ILSKLSQDSN; LSKLSQDSNY; SKLSQDSNYR; KLSQDSNYRS; LSQDSNYRSL; SQDSNYRSLV; QDSNYRSLVK; DSNYRSLVKE; SNYRSLVKEI; NYRSLVKEIL; YRSLVKEILI; RSLVKEILIN; SLVKEILINR; LVKEILINRG; VKEILINRGF; KEILINRGFS; EILINRGFSI; ILINRGFSIQ; LINRGFSIQL; INRGFSIQLA; NRGFSIQLAI; RGFSIQLAIE; GFSIQLAIEE; FSIQLAIEEI; SIQLAIEEIS; IQLAIEEISL; QLAIEEISLR; LAIEEISLRT; AIEEISLRTL; IEEISLRTLN; EEISLRTLNV; EISLRTLNVK; ISLRTLNVKD; SLRTLNVKDK; LRTLNVKDKI; RTLNVKDKIQ; TLNVKDKIQH; LNVKDKIQHL; NVKDKIQHLN; VKDKIQHLNK; KDKIQHLNKP; DKIQHLNKPN; KIQHLNKPNL; IQHLNKPNLK; QHLNKPNLKT; HLNKPNLKTL; LNKPNLKTLY; NKPNLKTLYH; KPNLKTLYHD; PNLKTLYHDF; NLKTLYHDFN; LKTLYHDFNK; KTLYHDFNKL; TLYHDFNKLI; LYHDFNKLIP; YHDFNKLIPL; HDFNKLIPLK; DFNKLIPLKE; FNKLIPLKEK; NKLIPLKEKW; KLIPLKEKWL; LIPLKEKWLK; |

IPLKEKWLKD; PLKEKWLKDV; LKEKWLKDVD; KEKWLKDVDD;
EKWLKDVDDI; KWLKDVDDII; WLKDVDDIIK; LKDVDDIIKD;
KDVDDIIKDY; DVDDIIKDYN; VDDIIKDYNA; DDIIKDYNAN;
DIIKDYNANP; IIKDYNANPE; IKDYNANPEL; KDYNANPELR;
DYNANPELRT; YNANPELRTD; NANPELRTDI; ANPELRTDIS;
NPELRTDISK; PELRTDISKL; ELRTDISKLN; LRTDISKLND;
RTDISKLNDY; TDISKLNDYI; DISKLNDYII; ISKLNDYIIS;
SKLNDYIISK; KLNDYIISKN; LNDYIISKNS; NDYIISKNSK;
DYIISKNSKA; YIISKNSKAQ; IISKNSKAQF; ISKNSKAQFT;
SKNSKAQFTD; KNSKAQFTDI; NSKAQFTDIH; SKAQFTDIHN;
KAQFTDIHNI; AQFTDIHNII; QFTDIHNIIL; FTDIHNIILN;
TDIHNIILNL; DIHNIILNLI; IHNIILNLIN; HNIILNLINT;
NIILNLINTT; IILNLINTTT; ILNLINTTTN; LNLINTTTNI;
NLINTTTNIL; LINTTTNILA; INTTTNILAP; NTTTNILAPI;
TTTNILAPIQ;

11 mers:
MKNNKLIAIFL; KNNKLIAIFLL; NNKLIAIFLLH; NKLIAIFLLHI;
KLIAIFLLHIL; LIAIFLLHILT; IAIFLLHILTG; AIFLLHILTGL;
IFLLHILTGLI; FLLHILTGLIL; LLHILTGLILL; LHILTGLILLS;
HILTGLILLSC; ILTGLILLSCS; LTGLILLSCSL; TGLILLSCSLE;
GLILLSCSLEV; LILLSCSLEVN; ILLSCSLEVNQ; LLSCSLEVNQD;
LSCSLEVNQDD; SCSLEVNQDDN; CSLEVNQDDNQ; SLEVNQDDNQE;
LEVNQDDNQEK; EVNQDDNQEKQ; VNQDDNQEKQK; NQDDNQEKQKK;
QDDNQEKQKKA; DDNQEKQKKAK; DNQEKQKKAKT; NQEKQKKAKTK;
QEKQKKAKTKT; EKQKKAKTKTS; KQKKAKTKTSK; QKKAKTKTSKS;
KKAKTKTSKSE; KAKTKTSKSEN; AKTKTSKSENN; KTKTSKSENNS;
TKTSKSENNSS; KTSKSENNSSK; TSKSENNSSKM; SKSENNSSKMK;
KSENNSSKMKK; SENNSSKMKKL; ENNSSKMKKLS; NNSSKMKKLSK;
NSSKMKKLSKN; SSKMKKLSKNA; SKMKKLSKNAK; KMKKLSKNAKN;
MKKLSKNAKNK; KKLSKNAKNKK; KLSKNAKNKKP; LSKNAKNKKPT;
SKNAKNKKPTV; KNAKNKKPTVD; NAKNKKPTVDN; AKNKKPTVDNL;
KNKKPTVDNLL; NKKPTVDNLLV; KKPTVDNLLVA; KPTVDNLLVAI;
PTVDNLLVAIN; TVDNLLVAINT; VDNLLVAINTL; DNLLVAINTLK;
NLLVAINTLKN; LLVAINTLKNP; LVAINTLKNPP; VAINTLKNPPK;
AINTLKNPPKT; INTLKNPPKTA; NTLKNPPKTAG; TLKNPPKTAGK;
LKNPPKTAGKN; KNPPKTAGKNK; NPPKTAGKNKS; PPKTAGKNKSN;
PKTAGKNKSNS; KTAGKNKSNSA; TAGKNKSNSAA; AGKNKSNSAAA;
GKNKSNSAAAL; KNKSNSAAALK; NKSNSAAALKQ; KSNSAAALKQP;
SNSAAALKQPN; NSAAALKQPNN; SAAALKQPNNA; AAALKQPNNAN;
AALKQPNNANA; ALKQPNNANAL; LKQPNNANALK; KQPNNANALKQ;
QPNNANALKQI; PNNANALKQID; NNANALKQIDP; NANALKQIDPE;
ANALKQIDPEA; NALKQIDPEAK; ALKQIDPEAKE; LKQIDPEAKEL;
KQIDPEAKELI; QIDPEAKELIQ; IDPEAKELIQK; DPEAKELIQKI;
PEAKELIQKIL; EAKELIQKILE; AKELIQKILER; KELIQKILERS;
ELIQKILERSE; LIQKILERSED; IQKILERSEDI; QKILERSEDIV;
KILERSEDIVQ; ILERSEDIVQI; LERSEDIVQIS; ERSEDIVQISE;
RSEDIVQISEI; SEDIVQISEID; EDIVQISEIDA; DIVQISEIDAN;
IVQISEIDANK; VQISEIDANKG; QISEIDANKGE; ISEIDANKGEP;
SEIDANKGEPD; EIDANKGEPDD; IDANKGEPDDQ; DANKGEPDDQF;
ANKGEPDDQFE; NKGEPDDQFEM; KGEPDDQFEMK; GEPDDQFEMKA;

| | |
|---|---|
| | EPDDQFEMKAE; PDDQFEMKAEI; DDQFEMKAEIF; DQFEMKAEIFS; QFEMKAEIFSK; FEMKAEIFSKI; EMKAEIFSKIF; MKAEIFSKIFF; KAEIFSKIFFN; AEIFSKIFFNA; EIFSKIFFNAG; IFSKIFFNAGS; FSKIFFNAGST; SKIFFNAGSTV; KIFFNAGSTVT; IFFNAGSTVTF; FFNAGSTVTFD; FNAGSTVTFDD; NAGSTVTFDDN; AGSTVTFDDNE; GSTVTFDDNEY; STVTFDDNEYV; TVTFDDNEYVN; VTFDDNEYVNE; TFDDNEYVNER; FDDNEYVNERR; DDNEYVNERRI; DNEYVNERRIL; NEYVNERRILY; EYVNERRILYT; YVNERRILYTS; VNERRILYTSL; NERRILYTSLN; ERRILYTSLNF; RRILYTSLNFN; RILYTSLNFNE; ILYTSLNFNEN; LYTSLNFNENK; YTSLNFNENKI; TSLNFNENKIL; SLNFNENKILN; LNFNENKILNL; NFNENKILNLG; FNENKILNLGK; NENKILNLGKI; ENKILNLGKIL; NKILNLGKILS; KILNLGKILSK; ILNLGKILSKL; LNLGKILSKLS; NLGKILSKLSQ; LGKILSKLSQD; GKILSKLSQDS; KILSKLSQDSN; ILSKLSQDSNY; LSKLSQDSNYR; SKLSQDSNYRS; KLSQDSNYRSL; LSQDSNYRSLV; SQDSNYRSLVK; QDSNYRSLVKE; DSNYRSLVKEI; SNYRSLVKEIL; NYRSLVKEILI; YRSLVKEILIN; RSLVKEILINR; SLVKEILINRG; LVKEILINRGF; VKEILINRGFS; KEILINRGFSI; EILINRGFSIQ; ILINRGFSIQL; LINRGFSIQLA; INRGFSIQLAI; NRGFSIQLAIE; RGFSIQLAIEE; GFSIQLAIEEI; FSIQLAIEEIS; SIQLAIEEISL; IQLAIEEISLR; QLAIEEISLRT; LAIEEISLRTL; AIEEISLRTLN; IEEISLRTLNV; EEISLRTLNVK; EISLRTLNVKD; ISLRTLNVKDK; SLRTLNVKDKI; LRTLNVKDKIQ; RTLNVKDKIQH; TLNVKDKIQHL; LNVKDKIQHLN; NVKDKIQHLNK; VKDKIQHLNKP; KDKIQHLNKPN; DKIQHLNKPNL; KIQHLNKPNLK; IQHLNKPNLKT; QHLNKPNLKTL; HLNKPNLKTLY; LNKPNLKTLYH; NKPNLKTLYHD; KPNLKTLYHDF; PNLKTLYHDFN; NLKTLYHDFNK; LKTLYHDFNKL; KTLYHDFNKLI; TLYHDFNKLIP; LYHDFNKLIPL; YHDFNKLIPLK; HDFNKLIPLKE; DFNKLIPLKEK; FNKLIPLKEKW; NKLIPLKEKWL; KLIPLKEKWLK; LIPLKEKWLKD; IPLKEKWLKDV; PLKEKWLKDVD; LKEKWLKDVDD; KEKWLKDVDDI; EKWLKDVDDII; KWLKDVDDIIK; WLKDVDDIIKD; LKDVDDIIKDY; KDVDDIIKDYN; DVDDIIKDYNA; VDDIIKDYNAN; DDIIKDYNANP; DIIKDYNANPE; IIKDYNANPEL; IKDYNANPELR; KDYNANPELRT; DYNANPELRTD; YNANPELRTDI; NANPELRTDIS; ANPELRTDISK; NPELRTDISKL; PELRTDISKLN; ELRTDISKLND; LRTDISKLNDY; RTDISKLNDYI; TDISKLNDYII; DISKLNDYIIS; ISKLNDYIISK; SKLNDYIISKN; KLNDYIISKNS; LNDYIISKNSK; NDYIISKNSKA; DYIISKNSKAQ; YIISKNSKAQF; IISKNSKAQFT; ISKNSKAQFTD; SKNSKAQFTDI; KNSKAQFTDIH; NSKAQFTDIHN; SKAQFTDIHNI; KAQFTDIHNII; AQFTDIHNIIL; QFTDIHNIILN; FTDIHNIILNL; TDIHNIILNLI; DIHNIILNLIN; IHNIILNLINT; HNIILNLINTT; NIILNLINTTT; IILNLINTTTN; ILNLINTTTNI; LNLINTTTNIL; NLINTTTNILA; LINTTTNILAP; INTTTNILAPI; NTTTNILAPIQ; |
| Seq 42 | SEQ ID NO 49645-51050/ 8 mers: MKKVKSKY; KKVKSKYL; KVKSKYLA; VKSKYLAL; KSKYLALG; SKYLALGL; KYLALGLL; YLALGLLF; LALGLLFG; ALGLLFGF; LGLLFGFI; GLLFGFIS; LLFGFISC; LFGFISCD; FGFISCDL; GFISCDLF; FISCDLFI; ISCDLFIR; SCDLFIRY; CDLFIRYE; DLFIRYEM; LFIRYEMK; FIRYEMKE; IRYEMKEE; RYEMKEES; |

YEMKEESP; EMKEESPG; MKEESPGL; KEESPGLF; EESPGLFD;
ESPGLFDK; SPGLFDKG; PGLFDKGN; GLFDKGNS; LFDKGNSI;
FDKGNSIL; DKGNSILE; KGNSILET; GNSILETS; NSILETSE;
SILETSEE; ILETSEES; LETSEESI; ETSEESIK; TSEESIKK;
SEESIKKP; EESIKKPM; ESIKKPMN; SIKKPMNK; IKKPMNKK;
KKPMNKKG; KPMNKKGK; PMNKKGKG; MNKKGKGK; NKKGKGKI;
KKGKGKIA; KGKGKIAR; GKGKIARK; KGKIARKK; GKIARKKG;
KIARKKGK; IARKKGKS; ARKKGKSK; RKKGKSKV; KKGKSKVS;
KGKSKVSR; GKSKVSRK; KSKVSRKE; SKVSRKEP; KVSRKEPY;
VSRKEPYI; SRKEPYIH; RKEPYIHS; KEPYIHSL; EPYIHSLK;
PYIHSLKR; YIHSLKRD; IHSLKRDS; HSLKRDSA; SLKRDSAN;
LKRDSANK; KRDSANKS; RDSANKSN; DSANKSNF; SANKSNFL;
ANKSNFLQ; NKSNFLQK; KSNFLQKN; SNFLQKNV; NFLQKNVI;
FLQKNVIL; LQKNVILE; QKNVILEE; KNVILEEE; NVILEEES;
VILEEESL; ILEEESLK; LEEESLKT; EEESLKTE; EESLKTEL;
ESLKTELL; SLKTELLK; LKTELLKE; KTELLKEQ; TELLKEQS;
ELLKEQSE; LLKEQSET; LKEQSETR; KEQSETRK; EQSETRKE;
QSETRKEK; SETRKEKI; ETRKEKIQ; TRKEKIQK; RKEKIQKQ;
KEKIQKQQ; EKIQKQQD; KIQKQQDE; IQKQQDEY; QKQQDEYK;
KQQDEYKG; QQDEYKGM; QDEYKGMT; DEYKGMTQ; EYKGMTQG;
YKGMTQGS; KGMTQGSL; GMTQGSLN; MTQGSLNS; TQGSLNSL;
QGSLNSLS; GSLNSLSG; SLNSLSGE; LNSLSGES; NSLSGESG;
SLSGESGE; LSGESGEL; SGESGELK; GESGELKE; ESGELKET;
SGELKETI; GELKETIE; ELKETIES; LKETIESN; KETIESNE;
ETIESNEI; TIESNEID; IESNEIDI; ESNEIDIT; SNEIDITI;
NEIDITID; EIDITIDS; IDITIDSD; DITIDSDL; ITIDSDLR;
TIDSDLRP; IDSDLRPK; DSDLRPKS; SDLRPKSS; DLRPKSSL;
LRPKSSLQ; RPKSSLQD; PKSSLQDI; KSSLQDIA; SSLQDIAG;
SLQDIAGS; LQDIAGSN; QDIAGSNS; DIAGSNSI; IAGSNSIS;
AGSNSISY; GSNSISYT; SNSISYTD; NSISYTDE; SISYTDEI;
ISYTDEIE; SYTDEIEE; YTDEIEEE; TDEIEEED; DEIEEEDY;
EIEEEDYA; IEEEDYAR; EEEDYARY; EEDYARYY; EDYARYYL;
DYARYYLD; YARYYLDE; ARYYLDED; RYYLDEDD; YYLDEDDE;
YLDEDDED; LDEDDEDD; DEDDEDDE; EDDEDDEY; DDEDDEYY;
DEDDEYYE; EDDEYYED; DDEYYEDD; DEYYEDDY; EYYEDDYE;
YYEDDYEE; YEDDYEEI; EDDYEEIR; DDYEEIRL; DYEEIRLS;
YEEIRLSN; EEIRLSNR; EIRLSNRY; IRLSNRYQ; RLSNRYQS;
LSNRYQSY; SNRYQSYL; NRYQSYLE; RYQSYLEG; YQSYLEGV;
QSYLEGVK; SYLEGVKY; YLEGVKYN; LEGVKYNV; EGVKYNVD;
GVKYNVDS; VKYNVDSA; KYNVDSAI; YNVDSAIN; NVDSAINT;
VDSAINTI; DSAINTIN; SAINTINK; AINTINKI; INTINKIY;
NTINKIYD; TINKIYDT; INKIYDTY; NKIYDTYT; KIYDTYTL;
IYDTYTLF; YDTYTLFS; DTYTLFST; TYTLFSTK; YTLFSTKL;
TLFSTKLT; LFSTKLTQ; FSTKLTQM; STKLTQMY; TKLTQMYS;
KLTQMYST; LTQMYSTR; TQMYSTRL; QMYSTRLD; MYSTRLDN;
YSTRLDNL; STRLDNLA; TRLDNLAK; RLDNLAKA; LDNLAKAK;
DNLAKAKA; NLAKAKAK; LAKAKAKE; AKAKAKEE; KAKAKEEA;
AKAKEEAA; KAKEEAAK; AKEEAAKF; KEEAAKFT; EEAAKFTK;
EAAKFTKE; AAKFTKED; AKFTKEDL; KFTKEDLE; FTKEDLEK;
TKEDLEKN; KEDLEKNF; EDLEKNFK; DLEKNFKT; LEKNFKTL;
EKNFKTLL; KNFKTLLN; NFKTLLNY; FKTLLNYI; KTLLNYIQ;

TLLNYIQV; LLNYIQVS; LNYIQVSV; NYIQVSVK; YIQVSVKT;
IQVSVKTA; QVSVKTAA; VSVKTAAN; SVKTAANF; VKTAANFV;
KTAANFVY; TAANFVYI; AANFVYIN; ANFVYIND; NFVYINDT;
FVYINDTH; VYINDTHA; YINDTHAK; INDTHAKR; NDTHAKRK;
DTHAKRKL; THAKRKLE; HAKRKLEN; AKRKLENI; KRKLENIE;
RKLENIEA; KLENIEAE; LENIEAEI; ENIEAEIK; NIEAEIKT;
IEAEIKTL; EAEIKTLI; AEIKTLIA; EIKTLIAK; IKTLIAKI;
KTLIAKIK; TLIAKIKE; LIAKIKEQ; IAKIKEQS; AKIKEQSN;
KIKEQSNL; IKEQSNLY; KEQSNLYE; EQSNLYEA; QSNLYEAY;
SNLYEAYK; NLYEAYKA; LYEAYKAI; YEAYKAIV; EAYKAIVT;
AYKAIVTS; YKAIVTSI; KAIVTSIL; AIVTSILL; IVTSILLM;
VTSILLMR; TSILLMRD; SILLMRDS; ILLMRDSL; LLMRDSLK;
LMRDSLKE; MRDSLKEV; RDSLKEVQ; DSLKEVQG; SLKEVQGI;
LKEVQGII; KEVQGIID; EVQGIIDK; VQGIIDKN; QGIIDKNG;
GIIDKNGV; IIDKNGVW; IDKNGVWY;

9 mers:
MKKVKSKYL; KKVKSKYLA; KVKSKYLAL; VKSKYLALG; KSKYLALGL;
SKYLALGLL; KYLALGLLF; YLALGLLFG; LALGLLFGF; ALGLLFGFI;
LGLLFGFIS; GLLFGFISC; LLFGFISCD; LFGFISCDL; FGFISCDLF;
GFISCDLFI; FISCDLFIR; ISCDLFIRY; SCDLFIRYE; CDLFIRYEM;
DLFIRYEMK; LFIRYEMKE; FIRYEMKEE; IRYEMKEES; RYEMKEESP;
YEMKEESPG; EMKEESPGL; MKEESPGLF; KEESPGLFD; EESPGLFDK;
ESPGLFDKG; SPGLFDKGN; PGLFDKGNS; GLFDKGNSI; LFDKGNSIL;
FDKGNSILE; DKGNSILET; KGNSILETS; GNSILETSE; NSILETSEE;
SILETSEES; ILETSEESI; LETSEESIK; ETSEESIKK; TSEESIKKP;
SEESIKKPM; EESIKKPMN; ESIKKPMNK; SIKKPMNKK; IKKPMNKKG;
KKPMNKKGK; KPMNKKGKG; PMNKKGKGK; MNKKGKGKI; NKKGKGKIA;
KKGKGKIAR; KGKGKIARK; GKGKIARKK; KGKIARKKG; GKIARKKGK;
KIARKKGKS; IARKKGKSK; ARKKGKSKV; RKKGKSKVS; KKGKSKVSR;
KGKSKVSRK; GKSKVSRKE; KSKVSRKEP; SKVSRKEPY; KVSRKEPYI;
VSRKEPYIH; SRKEPYIHS; RKEPYIHSL; KEPYIHSLK; EPYIHSLKR;
PYIHSLKRD; YIHSLKRDS; IHSLKRDSA; HSLKRDSAN; SLKRDSANK;
LKRDSANKS; KRDSANKSN; RDSANKSNF; DSANKSNFL; SANKSNFLQ;
ANKSNFLQK; NKSNFLQKN; KSNFLQKNV; SNFLQKNVI; NFLQKNVIL;
FLQKNVILE; LQKNVILEE; QKNVILEEE; KNVILEEES; NVILEEESL;
VILEEESLK; ILEEESLKT; LEEESLKTE; EEESLKTEL; EESLKTELL;
ESLKTELLK; SLKTELLKE; LKTELLKEQ; KTELLKEQS; TELLKEQSE;
ELLKEQSET; LLKEQSETR; LKEQSETRK; KEQSETRKE; EQSETRKEK;
QSETRKEKI; SETRKEKIQ; ETRKEKIQK; TRKEKIQKQ; RKEKIQKQQ;
KEKIQKQQD; EKIQKQQDE; KIQKQQDEY; IQKQQDEYK; QKQQDEYKG;
KQQDEYKGM; QQDEYKGMT; QDEYKGMTQ; DEYKGMTQG; EYKGMTQGS;
YKGMTQGSL; KGMTQGSLN; GMTQGSLNS; MTQGSLNSL; TQGSLNSLS;
QGSLNSLSG; GSLNSLSGE; SLNSLSGES; LNSLSGESG; NSLSGESGE;
SLSGESGEL; LSGESGELK; SGESGELKE; GESGELKET; ESGELKETI;
SGELKETIE; GELKETIES; ELKETIESN; LKETIESNE; KETIESNEI;
ETIESNEID; TIESNEIDI; IESNEIDIT; ESNEIDITI; SNEIDITID;
NEIDITIDS; EIDITIDSD; IDITIDSDL; DITIDSDLR; ITIDSDLRP;
TIDSDLRPK; IDSDLRPKS; DSDLRPKSS; SDLRPKSSL; DLRPKSSLQ;
LRPKSSLQD; RPKSSLQDI; PKSSLQDIA; KSSLQDIAG; SSLQDIAGS;
SLQDIAGSN; LQDIAGSNS; QDIAGSNSI; DIAGSNSIS; IAGSNSISY;

AGSNSISYT; GSNSISYTD; SNSISYTDE; NSISYTDEI; SISYTDEIE;
ISYTDEIEE; SYTDEIEEE; YTDEIEEED; TDEIEEEDY; DEIEEEDYA;
EIEEEDYAR; IEEEDYARY; EEEDYARYY; EEDYARYYL; EDYARYYLD;
DYARYYLDE; YARYYLDED; ARYYLDEDD; RYYLDEDDE; YYLDEDDED;
YLDEDDEDD; LDEDDEDDE; DEDDEDDEY; EDDEDDEYY; DDEDDEYYE;
DEDDEYYED; EDDEYYEDD; DDEYYEDDY; DEYYEDDYE; EYYEDDYEE;
YYEDDYEEI; YEDDYEEIR; EDDYEEIRL; DDYEEIRLS; DYEEIRLSN;
YEEIRLSNR; EEIRLSNRY; EIRLSNRYQ; IRLSNRYQS; RLSNRYQSY;
LSNRYQSYL; SNRYQSYLE; NRYQSYLEG; RYQSYLEGV; YQSYLEGVK;
QSYLEGVKY; SYLEGVKYN; YLEGVKYNV; LEGVKYNVD; EGVKYNVDS;
GVKYNVDSA; VKYNVDSAI; KYNVDSAIN; YNVDSAINT; NVDSAINTI;
VDSAINTIN; DSAINTINK; SAINTINKI; AINTINKIY; INTINKIYD;
NTINKIYDT; TINKIYDTY; INKIYDTYT; NKIYDTYTL; KIYDTYTLF;
IYDTYTLFS; YDTYTLFST; DTYTLFSTK; TYTLFSTKL; YTLFSTKLT;
TLFSTKLTQ; LFSTKLTQM; FSTKLTQMY; STKLTQMYS; TKLTQMYST;
KLTQMYSTR; LTQMYSTRL; TQMYSTRLD; QMYSTRLDN; MYSTRLDNL;
YSTRLDNLA; STRLDNLAK; TRLDNLAKA; RLDNLAKAK; LDNLAKAKA;
DNLAKAKAK; NLAKAKAKE; LAKAKAKEE; AKAKAKEEA; KAKAKEEAA;
AKAKEEAAK; KAKEEAAKF; AKEEAAKFT; KEEAAKFTK; EEAAKFTKE;
EAAKFTKED; AAKFTKEDL; AKFTKEDLE; KFTKEDLEK; FTKEDLEKN;
TKEDLEKNF; KEDLEKNFK; EDLEKNFKT; DLEKNFKTL; LEKNFKTLL;
EKNFKTLLN; KNFKTLLNY; NFKTLLNYI; FKTLLNYIQ; KTLLNYIQV;
TLLNYIQVS; LLNYIQVSV; LNYIQVSVK; NYIQVSVKT; YIQVSVKTA;
IQVSVKTAA; QVSVKTAAN; VSVKTAANF; SVKTAANFV; VKTAANFVY;
KTAANFVYI; TAANFVYIN; AANFVYIND; ANFVYINDT; NFVYINDTH;
FVYINDTHA; VYINDTHAK; YINDTHAKR; INDTHAKRK; NDTHAKRKL;
DTHAKRKLE; THAKRKLEN; HAKRKLENI; AKRKLENIE; KRKLENIEA;
RKLENIEAE; KLENIEAEI; LENIEAEIK; ENIEAEIKT; NIEAEIKTL;
IEAEIKTLI; EAEIKTLIA; AEIKTLIAK; EIKTLIAKI; IKTLIAKIK;
KTLIAKIKE; TLIAKIKEQ; LIAKIKEQS; IAKIKEQSN; AKIKEQSNL;
KIKEQSNLY; IKEQSNLYE; KEQSNLYEA; EQSNLYEAY; QSNLYEAYK;
SNLYEAYKA; NLYEAYKAI; LYEAYKAIV; YEAYKAIVT; EAYKAIVTS;
AYKAIVTSI; YKAIVTSIL; KAIVTSILL; AIVTSILLM; IVTSILLMR;
VTSILLMRD; TSILLMRDS; SILLMRDSL; ILLMRDSLK; LLMRDSLKE;
LMRDSLKEV; MRDSLKEVQ; RDSLKEVQG; DSLKEVQGI; SLKEVQGII;
LKEVQGIID; KEVQGIIDK; EVQGIIDKN; VQGIIDKNG; QGIIDKNGV;
GIIDKNGVW; IIDKNGVWY;

10 mers:
MKKVKSKYLA; KKVKSKYLAL; KVKSKYLALG; VKSKYLALGL;
KSKYLALGLL; SKYLALGLLF; KYLALGLLFG; YLALGLLFGF;
LALGLLFGFI; ALGLLFGFIS; LGLLFGFISC; GLLFGFISCD;
LLFGFISCDL; LFGFISCDLF; FGFISCDLFI; GFISCDLFIR;
FISCDLFIRY; ISCDLFIRYE; SCDLFIRYEM; CDLFIRYEMK;
DLFIRYEMKE; LFIRYEMKEE; FIRYEMKEES; IRYEMKEESP;
RYEMKEESPG; YEMKEESPGL; EMKEESPGLF; MKEESPGLFD;
KEESPGLFDK; EESPGLFDKG; ESPGLFDKGN; SPGLFDKGNS;
PGLFDKGNSI; GLFDKGNSIL; LFDKGNSILE; FDKGNSILET;
DKGNSILETS; KGNSILETSE; GNSILETSEE; NSILETSEES;
SILETSEESI; ILETSEESIK; LETSEESIKK; ETSEESIKKP;
TSEESIKKPM; SEESIKKPMN; EESIKKPMNK; ESIKKPMNKK;

SIKKPMNKKG; IKKPMNKKGK; KKPMNKKGKG; KPMNKKGKGK;
PMNKKGKGKI; MNKKGKGKIA; NKKGKGKIAR; KKGKGKIARK;
KGKGKIARKK; GKGKIARKKG; KGKIARKKGK; GKIARKKGKS;
KIARKKGKSK; IARKKGKSKV; ARKKGKSKVS; RKKGKSKVSR;
KKGKSKVSRK; KGKSKVSRKE; GKSKVSRKEP; KSKVSRKEPY;
SKVSRKEPYI; KVSRKEPYIH; VSRKEPYIHS; SRKEPYIHSL;
RKEPYIHSLK; KEPYIHSLKR; EPYIHSLKRD; PYIHSLKRDS;
YIHSLKRDSA; IHSLKRDSAN; HSLKRDSANK; SLKRDSANKS;
LKRDSANKSN; KRDSANKSNF; RDSANKSNFL; DSANKSNFLQ;
SANKSNFLQK; ANKSNFLQKN; NKSNFLQKNV; KSNFLQKNVI;
SNFLQKNVIL; NFLQKNVILE; FLQKNVILEE; LQKNVILEEE;
QKNVILEEES; KNVILEEESL; NVILEEESLK; VILEEESLKT;
ILEEESLKTE; LEEESLKTEL; EEESLKTELL; EESLKTELLK;
ESLKTELLKE; SLKTELLKEQ; LKTELLKEQS; KTELLKEQSE;
TELLKEQSET; ELLKEQSETR; LLKEQSETRK; LKEQSETRKE;
KEQSETRKEK; EQSETRKEKI; QSETRKEKIQ; SETRKEKIQK;
ETRKEKIQKQ; TRKEKIQKQQ; RKEKIQKQQD; KEKIQKQQDE;
EKIQKQQDEY; KIQKQQDEYK; IQKQQDEYKG; QKQQDEYKGM;
KQQDEYKGMT; QQDEYKGMTQ; QDEYKGMTQG; DEYKGMTQGS;
EYKGMTQGSL; YKGMTQGSLN; KGMTQGSLNS; GMTQGSLNSL;
MTQGSLNSLS; TQGSLNSLSG; QGSLNSLSGE; GSLNSLSGES;
SLNSLSGESG; LNSLSGESGE; NSLSGESGEL; SLSGESGELK;
LSGESGELKE; SGESGELKET; GESGELKETI; ESGELKETIE;
SGELKETIES; GELKETIESN; ELKETIESNE; LKETIESNEI;
KETIESNEID; ETIESNEIDI; TIESNEIDIT; IESNEIDITI;
ESNEIDITID; SNEIDITIDS; NEIDITIDSD; EIDITIDSDL;
IDITIDSDLR; DITIDSDLRP; ITIDSDLRPK; TIDSDLRPKS;
IDSDLRPKSS; DSDLRPKSSL; SDLRPKSSLQ; DLRPKSSLQD;
LRPKSSLQDI; RPKSSLQDIA; PKSSLQDIAG; KSSLQDIAGS;
SSLQDIAGSN; SLQDIAGSNS; LQDIAGSNSI; QDIAGSNSIS;
DIAGSNSISY; IAGSNSISYT; AGSNSISYTD; GSNSISYTDE;
SNSISYTDEI; NSISYTDEIE; SISYTDEIEE; ISYTDEIEEE;
SYTDEIEEED; YTDEIEEEDY; TDEIEEEDYA; DEIEEEDYAR;
EIEEEDYARY; IEEEDYARYY; EEEDYARYYL; EEDYARYYLD;
EDYARYYLDE; DYARYYLDED; YARYYLDEDD; ARYYLDEDDE;
RYYLDEDDED; YYLDEDDEDD; YLDEDDEDDE; LDEDDEDDEY;
DEDDEDDEYY; EDDEDDEYYE; DDEDDEYYED; DEDDEYYEDD;
EDDEYYEDDY; DDEYYEDDYE; DEYYEDDYEE; EYYEDDYEEI;
YYEDDYEEIR; YEDDYEEIRL; EDDYEEIRLS; DDYEEIRLSN;
DYEEIRLSNR; YEEIRLSNRY; EEIRLSNRYQ; EIRLSNRYQS;
IRLSNRYQSY; RLSNRYQSYL; LSNRYQSYLE; SNRYQSYLEG;
NRYQSYLEGV; RYQSYLEGVK; YQSYLEGVKY; QSYLEGVKYN;
SYLEGVKYNV; YLEGVKYNVD; LEGVKYNVDS; EGVKYNVDSA;
GVKYNVDSAI; VKYNVDSAIN; KYNVDSAINT; YNVDSAINTI;
NVDSAINTIN; VDSAINTINK; DSAINTINKI; SAINTINKIY;
AINTINKIYD; INTINKIYDT; NTINKIYDTY; TINKIYDTYT;
INKIYDTYTL; NKIYDTYTLF; KIYDTYTLFS; IYDTYTLFST;
YDTYTLFSTK; DTYTLFSTKL; TYTLFSTKLT; YTLFSTKLTQ;
TLFSTKLTQM; LFSTKLTQMY; FSTKLTQMYS; STKLTQMYST;
TKLTQMYSTR; KLTQMYSTRL; LTQMYSTRLD; TQMYSTRLDN;
QMYSTRLDNL; MYSTRLDNLA; YSTRLDNLAK; STRLDNLAKA;

TRLDNLAKAK; RLDNLAKAKA; LDNLAKAKAK; DNLAKAKAKE;
NLAKAKAKEE; LAKAKAKEEA; AKAKAKEEAA; KAKAKEEAAK;
AKAKEEAAKF; KAKEEAAKFT; AKEEAAKFTK; KEEAAKFTKE;
EEAAKFTKED; EAAKFTKEDL; AAKFTKEDLE; AKFTKEDLEK;
KFTKEDLEKN; FTKEDLEKNF; TKEDLEKNFK; KEDLEKNFKT;
EDLEKNFKTL; DLEKNFKTLL; LEKNFKTLLN; EKNFKTLLNY;
KNFKTLLNYI; NFKTLLNYIQ; FKTLLNYIQV; KTLLNYIQVS;
TLLNYIQVSV; LLNYIQVSVK; LNYIQVSVKT; NYIQVSVKTA;
YIQVSVKTAA; IQVSVKTAAN; QVSVKTAANF; VSVKTAANFV;
SVKTAANFVY; VKTAANFVYI; KTAANFVYIN; TAANFVYIND;
AANFVYINDT; ANFVYINDTH; NFVYINDTHA; FVYINDTHAK;
VYINDTHAKR; YINDTHAKRK; INDTHAKRKL; NDTHAKRKLE;
DTHAKRKLEN; THAKRKLENI; HAKRKLENIE; AKRKLENIEA;
KRKLENIEAE; RKLENIEAEI; KLENIEAEIK; LENIEAEIKT;
ENIEAEIKTL; NIEAEIKTLI; IEAEIKTLIA; EAEIKTLIAK;
AEIKTLIAKI; EIKTLIAKIK; IKTLIAKIKE; KTLIAKIKEQ;
TLIAKIKEQS; LIAKIKEQSN; IAKIKEQSNL; AKIKEQSNLY;
KIKEQSNLYE; IKEQSNLYEA; KEQSNLYEAY; EQSNLYEAYK;
QSNLYEAYKA; SNLYEAYKAI; NLYEAYKAIV; LYEAYKAIVT;
YEAYKAIVTS; EAYKAIVTSI; AYKAIVTSIL; YKAIVTSILL;
KAIVTSILLM; AIVTSILLMR; IVTSILLMRD; VTSILLMRDS;
TSILLMRDSL; SILLMRDSLK; ILLMRDSLKE; LLMRDSLKEV;
LMRDSLKEVQ; MRDSLKEVQG; RDSLKEVQGI; DSLKEVQGII;
SLKEVQGIID; LKEVQGIIDK; KEVQGIIDKN; EVQGIIDKNG;
VQGIIDKNGV; QGIIDKNGVW; GIIDKNGVWY;

11 mers:
MKKVKSKYLAL; KKVKSKYLALG; KVKSKYLALGL; VKSKYLALGLL;
KSKYLALGLLF; SKYLALGLLFG; KYLALGLLFGF; YLALGLLFGFI;
LALGLLFGFIS; ALGLLFGFISC; LGLLFGFISCD; GLLFGFISCDL;
LLFGFISCDLF; LFGFISCDLFI; FGFISCDLFIR; GFISCDLFIRY;
FISCDLFIRYE; ISCDLFIRYEM; SCDLFIRYEMK; CDLFIRYEMKE;
DLFIRYEMKEE; LFIRYEMKEES; FIRYEMKEESP; IRYEMKEESPG;
RYEMKEESPGL; YEMKEESPGLF; EMKEESPGLFD; MKEESPGLFDK;
KEESPGLFDKG; EESPGLFDKGN; ESPGLFDKGNS; SPGLFDKGNSI;
PGLFDKGNSIL; GLFDKGNSILE; LFDKGNSILET; FDKGNSILETS;
DKGNSILETSE; KGNSILETSEE; GNSILETSEES; NSILETSEESI;
SILETSEESIK; ILETSEESIKK; LETSEESIKKP; ETSEESIKKPM;
TSEESIKKPMN; SEESIKKPMNK; EESIKKPMNKK; ESIKKPMNKKG;
SIKKPMNKKGK; IKKPMNKKGKG; KKPMNKKGKGK; KPMNKKGKGKI;
PMNKKGKGKIA; MNKKGKGKIAR; NKKGKGKIARK; KKGKGKIARKK;
KGKGKIARKKG; GKGKIARKKGK; KGKIARKKGKS; GKIARKKGKSK;
KIARKKGKSKV; IARKKGKSKVS; ARKKGKSKVSR; RKKGKSKVSRK;
KKGKSKVSRKE; KGKSKVSRKEP; GKSKVSRKEPY; KSKVSRKEPYI;
SKVSRKEPYIH; KVSRKEPYIHS; VSRKEPYIHSL; SRKEPYIHSLK;
RKEPYIHSLKR; KEPYIHSLKRD; EPYIHSLKRDS; PYIHSLKRDSA;
YIHSLKRDSAN; IHSLKRDSANK; HSLKRDSANKS; SLKRDSANKSN;
LKRDSANKSNF; KRDSANKSNFL; RDSANKSNFLQ; DSANKSNFLQK;
SANKSNFLQKN; ANKSNFLQKNV; NKSNFLQKNVI; KSNFLQKNVIL;
SNFLQKNVILE; NFLQKNVILEE; FLQKNVILEEE; LQKNVILEEES;
QKNVILEEESL; KNVILEEESLK; NVILEEESLKT; VILEEESLKTE;

| | |
|---|---|
| ILEEESLKTEL; LEEESLKTELL; EEESLKTELLK; EESLKTELLKE; | |
| ESLKTELLKEQ; SLKTELLKEQS; LKTELLKEQSE; KTELLKEQSET; | |
| TELLKEQSETR; ELLKEQSETRK; LLKEQSETRKE; LKEQSETRKEK; | |
| KEQSETRKEKI; EQSETRKEKIQ; QSETRKEKIQK; SETRKEKIQKQ; | |
| ETRKEKIQKQQ; TRKEKIQKQQD; RKEKIQKQQDE; KEKIQKQQDEY; | |
| EKIQKQQDEYK; KIQKQQDEYKG; IQKQQDEYKGM; QKQQDEYKGMT; | |
| KQQDEYKGMTQ; QQDEYKGMTQG; QDEYKGMTQGS; DEYKGMTQGSL; | |
| EYKGMTQGSLN; YKGMTQGSLNS; KGMTQGSLNSL; GMTQGSLNSLS; | |
| MTQGSLNSLSG; TQGSLNSLSGE; QGSLNSLSGES; GSLNSLSGESG; | |
| SLNSLSGESGE; LNSLSGESGEL; NSLSGESGELK; SLSGESGELKE; | |
| LSGESGELKET; SGESGELKETI; GESGELKETIE; ESGELKETIES; | |
| SGELKETIESN; GELKETIESNE; ELKETIESNEI; LKETIESNEID; | |
| KETIESNEIDI; ETIESNEIDIT; TIESNEIDITI; IESNEIDITID; | |
| ESNEIDITIDS; SNEIDITIDSD; NEIDITIDSDL; EIDITIDSDLR; | |
| IDITIDSDLRP; DITIDSDLRPK; ITIDSDLRPKS; TIDSDLRPKSS; | |
| IDSDLRPKSSL; DSDLRPKSSLQ; SDLRPKSSLQD; DLRPKSSLQDI; | |
| LRPKSSLQDIA; RPKSSLQDIAG; PKSSLQDIAGS; KSSLQDIAGSN; | |
| SSLQDIAGSNS; SLQDIAGSNSI; LQDIAGSNSIS; QDIAGSNSISY; | |
| DIAGSNSISYT; IAGSNSISYTD; AGSNSISYTDE; GSNSISYTDEI; | |
| SNSISYTDEIE; NSISYTDEIEE; SISYTDEIEEE; ISYTDEIEEED; | |
| SYTDEIEEEDY; YTDEIEEEDYA; TDEIEEEDYAR; DEIEEEDYARY; | |
| EIEEEDYARYY; IEEEDYARYYL; EEEDYARYYLD; EEDYARYYLDE; | |
| EDYARYYLDED; DYARYYLDEDD; YARYYLDEDDE; ARYYLDEDDED; | |
| RYYLDEDDEDD; YYLDEDDEDDE; YLDEDDEDDEY; LDEDDEDDEYY; | |
| DEDDEDDEYYE; EDDEDDEYYED; DDEDDEYYEDD; DEDDEYYEDDY; | |
| EDDEYYEDDYE; DDEYYEDDYEE; DEYYEDDYEEI; EYYEDDYEEIR; | |
| YYEDDYEEIRL; YEDDYEEIRLS; EDDYEEIRLSN; DDYEEIRLSNR; | |
| DYEEIRLSNRY; YEEIRLSNRYQ; EEIRLSNRYQS; EIRLSNRYQSY; | |
| IRLSNRYQSYL; RLSNRYQSYLE; LSNRYQSYLEG; SNRYQSYLEGV; | |
| NRYQSYLEGVK; RYQSYLEGVKY; YQSYLEGVKYN; QSYLEGVKYNV; | |
| SYLEGVKYNVD; YLEGVKYNVDS; LEGVKYNVDSA; EGVKYNVDSAI; | |
| GVKYNVDSAIN; VKYNVDSAINT; KYNVDSAINTI; YNVDSAINTIN; | |
| NVDSAINTINK; VDSAINTINKI; DSAINTINKIY; SAINTINKIYD; | |
| AINTINKIYDT; INTINKIYDTY; NTINKIYDTYT; TINKIYDTYTL; | |
| INKIYDTYTLF; NKIYDTYTLFS; KIYDTYTLFST; IYDTYTLFSTK; | |
| YDTYTLFSTKL; DTYTLFSTKLT; TYTLFSTKLTQ; YTLFSTKLTQM; | |
| TLFSTKLTQMY; LFSTKLTQMYS; FSTKLTQMYST; STKLTQMYSTR; | |
| TKLTQMYSTRL; KLTQMYSTRLD; LTQMYSTRLDN; TQMYSTRLDNL; | |
| QMYSTRLDNLA; MYSTRLDNLAK; YSTRLDNLAKA; STRLDNLAKAK; | |
| TRLDNLAKAKA; RLDNLAKAKAK; LDNLAKAKAKE; DNLAKAKAKEE; | |
| NLAKAKAKEEA; LAKAKAKEEAA; AKAKAKEEAAK; KAKAKEEAAKF; | |
| AKAKEEAAKFT; KAKEEAAKFTK; AKEEAAKFTKE; KEEAAKFTKED; | |
| EEAAKFTKEDL; EAAKFTKEDLE; AAKFTKEDLEK; AKFTKEDLEKN; | |
| KFTKEDLEKNF; FTKEDLEKNFK; TKEDLEKNFKT; KEDLEKNFKTL; | |
| EDLEKNFKTLL; DLEKNFKTLLN; LEKNFKTLLNY; EKNFKTLLNYI; | |
| KNFKTLLNYIQ; NFKTLLNYIQV; FKTLLNYIQVS; KTLLNYIQVSV; | |
| TLLNYIQVSVK; LLNYIQVSVKT; LNYIQVSVKTA; NYIQVSVKTAA; | |
| YIQVSVKTAAN; IQVSVKTAANF; QVSVKTAANFV; VSVKTAANFVY; | |
| SVKTAANFVYI; VKTAANFVYIN; KTAANFVYIND; TAANFVYINDT; | |
| AANFVYINDTH; ANFVYINDTHA; NFVYINDTHAK; FVYINDTHAKR; | |
| VYINDTHAKRK; YINDTHAKRKL; INDTHAKRKLE; NDTHAKRKLEN; | |

| | |
|---|---|
| | DTHAKRKLENI; THAKRKLENIE; HAKRKLENIEA; AKRKLENIEAE;<br>KRKLENIEAEI; RKLENIEAEIK; KLENIEAEIKT; LENIEAEIKTL;<br>ENIEAEIKTLI; NIEAEIKTLIA; IEAEIKTLIAK; EAEIKTLIAKI;<br>AEIKTLIAKIK; EIKTLIAKIKE; IKTLIAKIKEQ; KTLIAKIKEQS;<br>TLIAKIKEQSN; LIAKIKEQSNL; IAKIKEQSNLY; AKIKEQSNLYE;<br>KIKEQSNLYEA; IKEQSNLYEAY; KEQSNLYEAYK; EQSNLYEAYKA;<br>QSNLYEAYKAI; SNLYEAYKAIV; NLYEAYKAIVT; LYEAYKAIVTS;<br>YEAYKAIVTSI; EAYKAIVTSIL; AYKAIVTSILL; YKAIVTSILLM;<br>KAIVTSILLMR; AIVTSILLMRD; IVTSILLMRDS; VTSILLMRDSL;<br>TSILLMRDSLK; SILLMRDSLKE; ILLMRDSLKEV; LLMRDSLKEVQ;<br>LMRDSLKEVQG; MRDSLKEVQGI; RDSLKEVQGII; DSLKEVQGIID;<br>SLKEVQGIIDK; LKEVQGIIDKN; KEVQGIIDKNG; EVQGIIDKNGV;<br>VQGIIDKNGVW; QGIIDKNGVWY; |
| Seq 43 | SEQ ID NO 51051-52424/<br>8 mers:<br>MKIKSKCL; KIKSKCLA; IKSKCLAL; KSKCLALG; SKCLALGL;<br>KCLALGLL; CLALGLLF; LALGLLFG; ALGLLFGF; LGLLFGFI;<br>GLLFGFIS; LLFGFISC; LFGFISCD; FGFISCDL; GFISCDLF;<br>FISCDLFI; ISCDLFIR; SCDLFIRD; CDLFIRDE; DLFIRDEI;<br>LFIRDEIK; FIRDEIKE; IRDEIKEK; RDEIKEKS; DEIKEKSL;<br>EIKEKSLG; IKEKSLGL; KEKSLGLC; EKSLGLCD; KSLGLCDE;<br>SLGLCDEE; LGLCDEES; GLCDEESS; LCDEESSI; CDEESSIL;<br>DEESSILE; EESSILET; ESSILETG; SSILETGD; SILETGDK;<br>ILETGDKS; LETGDKSV; ETGDKSVK; TGDKSVKK; GDKSVKKS;<br>DKSVKKSL; KSVKKSLN; SVKKSLNK; VKKSLNKK; KKSLNKKG;<br>KSLNKKGK; SLNKKGKD; LNKKGKDK; NKKGKDKV; KKGKDKVA;<br>KGKDKVAR; GKDKVARK; KDKVARKK; DKVARKKV; KVARKKVE;<br>VARKKVEG; ARKKVEGN; RKKVEGNA; KKVEGNAV; KVEGNAVK;<br>VEGNAVKK; EGNAVKKD; GNAVKKDP; NAVKKDPF; AVKKDPFN;<br>VKKDPFNH; KKDPFNHH; KDPFNHHV; DPFNHHVK; PFNHHVKR;<br>FNHHVKRE; NHHVKRES; HHVKRESV; HVKRESVN; VKRESVNN;<br>KRESVNNS; RESVNNSN; ESVNNSNL; SVNNSNLS; VNNSNLSQ;<br>NNSNLSQK; NSNLSQKN; SNLSQKNV; NLSQKNVI; LSQKNVIS;<br>SQKNVISE; QKNVISEE; KNVISEEE; NVISEEEI; VISEEEIL;<br>ISEEEILK; SEEEILKT; EEEILKTK; EEILKTKL; EILKTKLL;<br>ILKTKLLR; LKTKLLRE; KTKLLRER; TKLLRERP; KLLRERPE;<br>LLRERPET; LRERPETR; RERPETRK; ERPETRKE; RPETRKEE;<br>PETRKEEI; ETRKEEIQ; TRKEEIQK; RKEEIQKQ; KEEIQKQQ;<br>EEIQKQQD; EIQKQQDE; IQKQQDEH; QKQQDEHK; KQQDEHKR;<br>QQDEHKRM; QDEHKRML; DEHKRMLQ; EHKRMLQG; HKRMLQGS;<br>KRMLQGSL; RMLQGSLS; MLQGSLSF; LQGSLSFL; QGSLSFLS;<br>GSLSFLSG; SLSFLSGE; LSFLSGES; SFLSGESG; FLSGESGE;<br>LSGESGEL; SGESGELK; GESGELKD; ESGELKDT; SGELKDTI;<br>GELKDTIE; ELKDTIES; LKDTIESN; KDTIESNE; DTIESNEI;<br>TIESNEID; IESNEIDF; ESNEIDFT; SNEIDFTI; NEIDFTID;<br>EIDFTIDS; IDFTIDSD; DFTIDSDL; FTIDSDLR; TIDSDLRL;<br>IDSDLRLK; DSDLRLKS; SDLRLKSD; DLRLKSDL; LRLKSDLQ;<br>RLKSDLQA; LKSDLQAI; KSDLQAIS; SDLQAISG; DLQAISGS;<br>LQAISGSN; QAISGSNS; AISGSNSI; ISGSNSIS; SGSNSISY;<br>GSNSISYT; SNSISYTD; NSISYTDE; SISYTDEI; ISYTDEIE; |

SYTDEIEE; YTDEIEEE; TDEIEEED; DEIEEEDY; EIEEEDYD;
IEEEDYDQ; EEEDYDQY; EEDYDQYS; EDYDQYSL; DYDQYSLE;
YDQYSLEE; DQYSLEED; QYSLEEDY; YSLEEDYY; SLEEDYYY;
LEEDYYYD; EEDYYYDG; EDYYYDGE; DYYYDGET; YYYDGETR;
YYDGETRL; YDGETRLS; DGETRLSN; GETRLSNR; ETRLSNRY;
TRLSNRYE; RLSNRYES; LSNRYESY; SNRYESYL; NRYESYLE;
RYESYLEG; YESYLEGV; ESYLEGVK; SYLEGVKY; YLEGVKYN;
LEGVKYNV; EGVKYNVS; GVKYNVSS; VKYNVSSA; KYNVSSAI;
YNVSSAIK; NVSSAIKT; VSSAIKTI; SSAIKTIV; SAIKTIVK;
AIKTIVKI; IKTIVKIY; KTIVKIYD; TIVKIYDN; IVKIYDNY;
VKIYDNYT; KIYDNYTL; IYDNYTLL; YDNYTLLS; DNYTLLST;
NYTLLSTK; YTLLSTKQ; TLLSTKQT; LLSTKQTQ; LSTKQTQM;
STKQTQMY; TKQTQMYS; KQTQMYST; QTQMYSTR; TQMYSTRL;
QMYSTRLD; MYSTRLDN; YSTRLDNL; STRLDNLA; TRLDNLAK;
RLDNLAKA; LDNLAKAK; DNLAKAKA; NLAKAKAR; LAKAKARE;
AKAKAREE; KAKAREEA; AKAREEAK; KAREEAKK; AREEAKKF;
REEAKKFT; EEAKKFTK; EAKKFTKE; AKKFTKEE; KKFTKEEL;
KFTKEELE; FTKEELEK; TKEELEKD; KEELEKDL; EELEKDLK;
ELEKDLKT; LEKDLKTL; EKDLKTLL; KDLKTLLN; DLKTLLNY;
LKTLLNYI; KTLLNYIQ; TLLNYIQV; LLNYIQVS; LNYIQVSA;
NYIQVSAR; YIQVSART; IQVSARTA; QVSARTAT; VSARTATN;
SARTATNF; ARTATNFV; RTATNFVY; TATNFVYA; ATNFVYAR;
TNFVYARE; NFVYAREI; FVYAREIY; VYAREIYS; YAREIYSK;
AREIYSKR; REIYSKRK; EIYSKRKL; IYSKRKLD; YSKRKLDA;
SKRKLDAI; KRKLDAIE; RKLDAIET; KLDAIETE; LDAIETEI;
DAIETEIK; AIETEIKN; IETEIKNL; ETEIKNLI; TEIKNLIL;
EIKNLILK; IKNLILKI; KNLILKIK; NLILKIKG; LILKIKGQ;
ILKIKGQS; LKIKGQSD; KIKGQSDL; IKGQSDLY; KGQSDLYE;
GQSDLYEA; QSDLYEAY; SDLYEAYK; DLYEAYKA; LYEAYKAI;
YEAYKAIV; EAYKAIVR; AYKAIVRS; YKAIVRSI; KAIVRSIL;
AIVRSILL; IVRSILLM; VRSILLMK; RSILLMKD; SILLMKDS;
ILLMKDSL; LLMKDSLK; LMKDSLKI; MKDSLKII; KDSLKIIE;
DSLKIIEI; SLKIIEIV; LKIIEIVI; KIIEIVID; IIEIVIDK;
IEIVIDKN; EIVIDKNG; IVIDKNGV; VIDKNGVW; IDKNGVWY;

9 mers:
MKIKSKCLA; KIKSKCLAL; IKSKCLALG; KSKCLALGL; SKCLALGLL;
KCLALGLLF; CLALGLLFG; LALGLLFGF; ALGLLFGFI; LGLLFGFIS;
GLLFGFISC; LLFGFISCD; LFGFISCDL; FGFISCDLF; GFISCDLFI;
FISCDLFIR; ISCDLFIRD; SCDLFIRDE; CDLFIRDEI; DLFIRDEIK;
LFIRDEIKE; FIRDEIKEK; IRDEIKEKS; RDEIKEKSL; DEIKEKSLG;
EIKEKSLGL; IKEKSLGLC; KEKSLGLCD; EKSLGLCDE; KSLGLCDEE;
SLGLCDEES; LGLCDEESS; GLCDEESSI; LCDEESSIL; CDEESSILE;
DEESSILET; EESSILETG; ESSILETGD; SSILETGDK; SILETGDKS;
ILETGDKSV; LETGDKSVK; ETGDKSVKK; TGDKSVKKS; GDKSVKKSL;
DKSVKKSLN; KSVKKSLNK; SVKKSLNKK; VKKSLNKKG; KKSLNKKGK;
KSLNKKGKD; SLNKKGKDK; LNKKGKDKV; NKKGKDKVA; KKGKDKVAR;
KGKDKVARK; GKDKVARKK; KDKVARKKV; DKVARKKVE; KVARKKVEG;
VARKKVEGN; ARKKVEGNA; RKKVEGNAV; KKVEGNAVK; KVEGNAVKK;
VEGNAVKKD; EGNAVKKDP; GNAVKKDPF; NAVKKDPFN; AVKKDPFNH;
VKKDPFNHH; KKDPFNHHV; KDPFNHHVK; DPFNHHVKR; PFNHHVKRE;

FNHHVKRES; NHHVKRESV; HHVKRESVN; HVKRESVNN; VKRESVNNS;
KRESVNNSN; RESVNNSNL; ESVNNSNLS; SVNNSNLSQ; VNNSNLSQK;
NNSNLSQKN; NSNLSQKNV; SNLSQKNVI; NLSQKNVIS; LSQKNVISE;
SQKNVISEE; QKNVISEEE; KNVISEEEI; NVISEEEIL; VISEEEILK;
ISEEEILKT; SEEEILKTK; EEEILKTKL; EEILKTKLL; EILKTKLLR;
ILKTKLLRE; LKTKLLRER; KTKLLRERP; TKLLRERPE; KLLRERPET;
LLRERPETR; LRERPETRK; RERPETRKE; ERPETRKEE; RPETRKEEI;
PETRKEEIQ; ETRKEEIQK; TRKEEIQKQ; RKEEIQKQQ; KEEIQKQQD;
EEIQKQQDE; EIQKQQDEH; IQKQQDEHK; QKQQDEHKR; KQQDEHKRM;
QQDEHKRML; QDEHKRMLQ; DEHKRMLQG; EHKRMLQGS; HKRMLQGSL;
KRMLQGSLS; RMLQGSLSF; MLQGSLSFL; LQGSLSFLS; QGSLSFLSG;
GSLSFLSGE; SLSFLSGES; LSFLSGESG; SFLSGESGE; FLSGESGEL;
LSGESGELK; SGESGELKD; GESGELKDT; ESGELKDTI; SGELKDTIE;
GELKDTIES; ELKDTIESN; LKDTIESNE; KDTIESNEI; DTIESNEID;
TIESNEIDF; IESNEIDFT; ESNEIDFTI; SNEIDFTID; NEIDFTIDS;
EIDFTIDSD; IDFTIDSDL; DFTIDSDLR; FTIDSDLRL; TIDSDLRLK;
IDSDLRLKS; DSDLRLKSD; SDLRLKSDL; DLRLKSDLQ; LRLKSDLQA;
RLKSDLQAI; LKSDLQAIS; KSDLQAISG; SDLQAISGS; DLQAISGSN;
LQAISGSNS; QAISGSNSI; AISGSNSIS; ISGSNSISY; SGSNSISYT;
GSNSISYTD; SNSISYTDE; NSISYTDEI; SISYTDEIE; ISYTDEIEE;
SYTDEIEEE; YTDEIEEED; TDEIEEEDY; DEIEEEDYD; EIEEEDYDQ;
IEEEDYDQY; EEEDYDQYS; EEDYDQYSL; EDYDQYSLE; DYDQYSLEE;
YDQYSLEED; DQYSLEEDY; QYSLEEDYY; YSLEEDYYY; SLEEDYYYD;
LEEDYYYDG; EEDYYYDGE; EDYYYDGET; DYYYDGETR; YYYDGETRL;
YYDGETRLS; YDGETRLSN; DGETRLSNR; GETRLSNRY; ETRLSNRYE;
TRLSNRYES; RLSNRYESY; LSNRYESYL; SNRYESYLE; NRYESYLEG;
RYESYLEGV; YESYLEGVK; ESYLEGVKY; SYLEGVKYN; YLEGVKYNV;
LEGVKYNVS; EGVKYNVSS; GVKYNVSSA; VKYNVSSAI; KYNVSSAIK;
YNVSSAIKT; NVSSAIKTI; VSSAIKTIV; SSAIKTIVK; SAIKTIVKI;
AIKTIVKIY; IKTIVKIYD; KTIVKIYDN; TIVKIYDNY; IVKIYDNYT;
VKIYDNYTL; KIYDNYTLL; IYDNYTLLS; YDNYTLLST; DNYTLLSTK;
NYTLLSTKQ; YTLLSTKQT; TLLSTKQTQ; LLSTKQTQM; LSTKQTQMY;
STKQTQMYS; TKQTQMYST; KQTQMYSTR; QTQMYSTRL; TQMYSTRLD;
QMYSTRLDN; MYSTRLDNL; YSTRLDNLA; STRLDNLAK; TRLDNLAKA;
RLDNLAKAK; LDNLAKAKA; DNLAKAKAR; NLAKAKARE; LAKAKAREE;
AKAKAREEA; KAKAREEAK; AKAREEAKK; KAREEAKKF; AREEAKKFT;
REEAKKFTK; EEAKKFTKE; EAKKFTKEE; AKKFTKEEL; KKFTKEELE;
KFTKEELEK; FTKEELEKD; TKEELEKDL; KEELEKDLK; EELEKDLKT;
ELEKDLKTL; LEKDLKTLL; EKDLKTLLN; KDLKTLLNY; DLKTLLNYI;
LKTLLNYIQ; KTLLNYIQV; TLLNYIQVS; LLNYIQVSA; LNYIQVSAR;
NYIQVSART; YIQVSARTA; IQVSARTAT; QVSARTATN; VSARTATNF;
SARTATNFV; ARTATNFVY; RTATNFVYA; TATNFVYAR; ATNFVYARE;
TNFVYAREI; NFVYAREIY; FVYAREIYS; VYAREIYSK; YAREIYSKR;
AREIYSKRK; REIYSKRKL; EIYSKRKLD; IYSKRKLDA; YSKRKLDAI;
SKRKLDAIE; KRKLDAIET; RKLDAIETE; KLDAIETEI; LDAIETEIK;
DAIETEIKN; AIETEIKNL; IETEIKNLI; ETEIKNLIL; TEIKNLILK;
EIKNLILKI; IKNLILKIK; KNLILKIKG; NLILKIKGQ; LILKIKGQS;
ILKIKGQSD; LKIKGQSDL; KIKGQSDLY; IKGQSDLYE; KGQSDLYEA;
GQSDLYEAY; QSDLYEAYK; SDLYEAYKA; DLYEAYKAI; LYEAYKAIV;
YEAYKAIVR; EAYKAIVRS; AYKAIVRSI; YKAIVRSIL; KAIVRSILL;
AIVRSILLM; IVRSILLMK; VRSILLMKD; RSILLMKDS; SILLMKDSL;

ILLMKDSLK; LLMKDSLKI; LMKDSLKII; MKDSLKIIE; KDSLKIIEI;
DSLKIIEIV; SLKIIEIVI; LKIIEIVID; KIIEIVIDK; IIEIVIDKN;
IEIVIDKNG; EIVIDKNGV; IVIDKNGVW; VIDKNGVWY;

10 mers:
MKIKSKCLAL; KIKSKCLALG; IKSKCLALGL; KSKCLALGLL;
SKCLALGLLF; KCLALGLLFG; CLALGLLFGF; LALGLLFGFI;
ALGLLFGFIS; LGLLFGFISC; GLLFGFISCD; LLFGFISCDL;
LFGFISCDLF; FGFISCDLFI; GFISCDLFIR; FISCDLFIRD;
ISCDLFIRDE; SCDLFIRDEI; CDLFIRDEIK; DLFIRDEIKE;
LFIRDEIKEK; FIRDEIKEKS; IRDEIKEKSL; RDEIKEKSLG;
DEIKEKSLGL; EIKEKSLGLC; IKEKSLGLCD; KEKSLGLCDE;
EKSLGLCDEE; KSLGLCDEES; SLGLCDEESS; LGLCDEESSI;
GLCDEESSIL; LCDEESSILE; CDEESSILET; DEESSILETG;
EESSILETGD; ESSILETGDK; SSILETGDKS; SILETGDKSV;
ILETGDKSVK; LETGDKSVKK; ETGDKSVKKS; TGDKSVKKSL;
GDKSVKKSLN; DKSVKKSLNK; KSVKKSLNKK; SVKKSLNKKG;
VKKSLNKKGK; KKSLNKKGKD; KSLNKKGKDK; SLNKKGKDKV;
LNKKGKDKVA; NKKGKDKVAR; KKGKDKVARK; KGKDKVARKK;
GKDKVARKKV; KDKVARKKVE; DKVARKKVEG; KVARKKVEGN;
VARKKVEGNA; ARKKVEGNAV; RKKVEGNAVK; KKVEGNAVKK;
KVEGNAVKKD; VEGNAVKKDP; EGNAVKKDPF; GNAVKKDPFN;
NAVKKDPFNH; AVKKDPFNHH; VKKDPFNHHV; KKDPFNHHVK;
KDPFNHHVKR; DPFNHHVKRE; PFNHHVKRES; FNHHVKRESV;
NHHVKRESVN; HHVKRESVNN; HVKRESVNNS; VKRESVNNSN;
KRESVNNSNL; RESVNNSNLS; ESVNNSNLSQ; SVNNSNLSQK;
VNNSNLSQKN; NNSNLSQKNV; NSNLSQKNVI; SNLSQKNVIS;
NLSQKNVISE; LSQKNVISEE; SQKNVISEEE; QKNVISEEEI;
KNVISEEEIL; NVISEEEILK; VISEEEILKT; ISEEEILKTK;
SEEEILKTKL; EEEILKTKLL; EEILKTKLLR; EILKTKLLRE;
ILKTKLLRER; LKTKLLRERP; KTKLLRERPE; TKLLRERPET;
KLLRERPETR; LLRERPETRK; LRERPETRKE; RERPETRKEE;
ERPETRKEEI; RPETRKEEIQ; PETRKEEIQK; ETRKEEIQKQ;
TRKEEIQKQQ; RKEEIQKQQD; KEEIQKQQDE; EEIQKQQDEH;
EIQKQQDEHK; IQKQQDEHKR; QKQQDEHKRM; KQQDEHKRML;
QQDEHKRMLQ; QDEHKRMLQG; DEHKRMLQGS; EHKRMLQGSL;
HKRMLQGSLS; KRMLQGSLSF; RMLQGSLSFL; MLQGSLSFLS;
LQGSLSFLSG; QGSLSFLSGE; GSLSFLSGES; SLSFLSGESG;
LSFLSGESGE; SFLSGESGEL; FLSGESGELK; LSGESGELKD;
SGESGELKDT; GESGELKDTI; ESGELKDTIE; SGELKDTIES;
GELKDTIESN; ELKDTIESNE; LKDTIESNEI; KDTIESNEID;
DTIESNEIDF; TIESNEIDFT; IESNEIDFTI; ESNEIDFTID;
SNEIDFTIDS; NEIDFTIDSD; EIDFTIDSDL; IDFTIDSDLR;
DFTIDSDLRL; FTIDSDLRLK; TIDSDLRLKS; IDSDLRLKSD;
DSDLRLKSDL; SDLRLKSDLQ; DLRLKSDLQA; LRLKSDLQAI;
RLKSDLQAIS; LKSDLQAISG; KSDLQAISGS; SDLQAISGSN;
DLQAISGSNS; LQAISGSNSI; QAISGSNSIS; AISGSNSISY;
ISGSNSISYT; SGSNSISYTD; GSNSISYTDE; SNSISYTDEI;
NSISYTDEIE; SISYTDEIEE; ISYTDEIEEE; SYTDEIEEED;
YTDEIEEEDY; TDEIEEEDYD; DEIEEEDYDQ; EIEEEDYDQY;
IEEEDYDQYS; EEEDYDQYSL; EEDYDQYSLE; EDYDQYSLEE;

DYDQYSLEED; YDQYSLEEDY; DQYSLEEDYY; QYSLEEDYYY;
YSLEEDYYYD; SLEEDYYYDG; LEEDYYYDGE; EEDYYYDGET;
EDYYYDGETR; DYYYDGETRL; YYYDGETRLS; YYDGETRLSN;
YDGETRLSNR; DGETRLSNRY; GETRLSNRYE; ETRLSNRYES;
TRLSNRYESY; RLSNRYESYL; LSNRYESYLE; SNRYESYLEG;
NRYESYLEGV; RYESYLEGVK; YESYLEGVKY; ESYLEGVKYN;
SYLEGVKYNV; YLEGVKYNVS; LEGVKYNVSS; EGVKYNVSSA;
GVKYNVSSAI; VKYNVSSAIK; KYNVSSAIKT; YNVSSAIKTI;
NVSSAIKTIV; VSSAIKTIVK; SSAIKTIVKI; SAIKTIVKIY;
AIKTIVKIYD; IKTIVKIYDN; KTIVKIYDNY; TIVKIYDNYT;
IVKIYDNYTL; VKIYDNYTLL; KIYDNYTLLS; IYDNYTLLST;
YDNYTLLSTK; DNYTLLSTKQ; NYTLLSTKQT; YTLLSTKQTQ;
TLLSTKQTQM; LLSTKQTQMY; LSTKQTQMYS; STKQTQMYST;
TKQTQMYSTR; KQTQMYSTRL; QTQMYSTRLD; TQMYSTRLDN;
QMYSTRLDNL; MYSTRLDNLA; YSTRLDNLAK; STRLDNLAKA;
TRLDNLAKAK; RLDNLAKAKA; LDNLAKAKAR; DNLAKAKARE;
NLAKAKAREE; LAKAKAREEA; AKAKAREEAK; KAKAREEAKK;
AKAREEAKKF; KAREEAKKFT; AREEAKKFTK; REEAKKFTKE;
EEAKKFTKEE; EAKKFTKEEL; AKKFTKEELE; KKFTKEELEK;
KFTKEELEKD; FTKEELEKDL; TKEELEKDLK; KEELEKDLKT;
EELEKDLKTL; ELEKDLKTLL; LEKDLKTLLN; EKDLKTLLNY;
KDLKTLLNYI; DLKTLLNYIQ; LKTLLNYIQV; KTLLNYIQVS;
TLLNYIQVSA; LLNYIQVSAR; LNYIQVSART; NYIQVSARTA;
YIQVSARTAT; IQVSARTATN; QVSARTATNF; VSARTATNFV;
SARTATNFVY; ARTATNFVYA; RTATNFVYAR; TATNFVYARE;
ATNFVYAREI; TNFVYAREIY; NFVYAREIYS; FVYAREIYSK;
VYAREIYSKR; YAREIYSKRK; AREIYSKRKL; REIYSKRKLD;
EIYSKRKLDA; IYSKRKLDAI; YSKRKLDAIE; SKRKLDAIET;
KRKLDAIETE; RKLDAIETEI; KLDAIETEIK; LDAIETEIKN;
DAIETEIKNL; AIETEIKNLI; IETEIKNLIL; ETEIKNLILK;
TEIKNLILKI; EIKNLILKIK; IKNLILKIKG; KNLILKIKGQ;
NLILKIKGQS; LILKIKGQSD; ILKIKGQSDL; LKIKGQSDLY;
KIKGQSDLYE; IKGQSDLYEA; KGQSDLYEAY; GQSDLYEAYK;
QSDLYEAYKA; SDLYEAYKAI; DLYEAYKAIV; LYEAYKAIVR;
YEAYKAIVRS; EAYKAIVRSI; AYKAIVRSIL; YKAIVRSILL;
KAIVRSILLM; AIVRSILLMK; IVRSILLMKD; VRSILLMKDS;
RSILLMKDSL; SILLMKDSLK; ILLMKDSLKI; LLMKDSLKII;
LMKDSLKIIE; MKDSLKIIEI; KDSLKIIEIV; DSLKIIEIVI;
SLKIIEIVID; LKIIEIVIDK; KIIEIVIDKN; IIEIVIDKNG;
IEIVIDKNGV; EIVIDKNGVW; IVIDKNGVWY;

11 mers:
MKIKSKCLALG; KIKSKCLALGL; IKSKCLALGLL; KSKCLALGLLF;
SKCLALGLLFG; KCLALGLLFGF; CLALGLLFGFI; LALGLLFGFIS;
ALGLLFGFISC; LGLLFGFISCD; GLLFGFISCDL; LLFGFISCDLF;
LFGFISCDLFI; FGFISCDLFIR; GFISCDLFIRD; FISCDLFIRDE;
ISCDLFIRDEI; SCDLFIRDEIK; CDLFIRDEIKE; DLFIRDEIKEK;
LFIRDEIKEKS; FIRDEIKEKSL; IRDEIKEKSLG; RDEIKEKSLGL;
DEIKEKSLGLC; EIKEKSLGLCD; IKEKSLGLCDE; KEKSLGLCDEE;
EKSLGLCDEES; KSLGLCDEESS; SLGLCDEESSI; LGLCDEESSIL;
GLCDEESSILE; LCDEESSILET; CDEESSILETG; DEESSILETGD;

| | EESSILETGDK; ESSILETGDKS; SSILETGDKSV; SILETGDKSVK; |
|---|---|
| | ILETGDKSVKK; LETGDKSVKKS; ETGDKSVKKSL; TGDKSVKKSLN; |
| | GDKSVKKSLNK; DKSVKKSLNKK; KSVKKSLNKKG; SVKKSLNKKGK; |
| | VKKSLNKKGKD; KKSLNKKGKDK; KSLNKKGKDKV; SLNKKGKDKVA; |
| | LNKKGKDKVAR; NKKGKDKVARK; KKGKDKVARKK; KGKDKVARKKV; |
| | GKDKVARKKVE; KDKVARKKVEG; DKVARKKVEGN; KVARKKVEGNA; |
| | VARKKVEGNAV; ARKKVEGNAVK; RKKVEGNAVKK; KKVEGNAVKKD; |
| | KVEGNAVKKDP; VEGNAVKKDPF; EGNAVKKDPFN; GNAVKKDPFNH; |
| | NAVKKDPFNHH; AVKKDPFNHHV; VKKDPFNHHVK; KKDPFNHHVKR; |
| | KDPFNHHVKRE; DPFNHHVKRES; PFNHHVKRESV; FNHHVKRESVN; |
| | NHHVKRESVNN; HHVKRESVNNS; HVKRESVNNSN; VKRESVNNSNL; |
| | KRESVNNSNLS; RESVNNSNLSQ; ESVNNSNLSQK; SVNNSNLSQKN; |
| | VNNSNLSQKNV; NNSNLSQKNVI; NSNLSQKNVIS; SNLSQKNVISE; |
| | NLSQKNVISEE; LSQKNVISEEE; SQKNVISEEEI; QKNVISEEEIL; |
| | KNVISEEEILK; NVISEEEILKT; VISEEEILKTK; ISEEEILKTKL; |
| | SEEEILKTKLL; EEEILKTKLLR; EEILKTKLLRE; EILKTKLLRER; |
| | ILKTKLLRERP; LKTKLLRERPE; KTKLLRERPET; TKLLRERPETR; |
| | KLLRERPETRK; LLRERPETRKE; LRERPETRKEE; RERPETRKEEI; |
| | ERPETRKEEIQ; RPETRKEEIQK; PETRKEEIQKQ; ETRKEEIQKQQ; |
| | TRKEEIQKQQD; RKEEIQKQQDE; KEEIQKQQDEH; EEIQKQQDEHK; |
| | EIQKQQDEHKR; IQKQQDEHKRM; QKQQDEHKRML; KQQDEHKRMLQ; |
| | QQDEHKRMLQG; QDEHKRMLQGS; DEHKRMLQGSL; EHKRMLQGSLS; |
| | HKRMLQGSLSF; KRMLQGSLSFL; RMLQGSLSFLS; MLQGSLSFLSG; |
| | LQGSLSFLSGE; QGSLSFLSGES; GSLSFLSGESG; SLSFLSGESGE; |
| | LSFLSGESGEL; SFLSGESGELK; FLSGESGELKD; LSGESGELKDT; |
| | SGESGELKDTI; GESGELKDTIE; ESGELKDTIES; SGELKDTIESN; |
| | GELKDTIESNE; ELKDTIESNEI; LKDTIESNEID; KDTIESNEIDF; |
| | DTIESNEIDFT; TIESNEIDFTI; IESNEIDFTID; ESNEIDFTIDS; |
| | SNEIDFTIDSD; NEIDFTIDSDL; EIDFTIDSDLR; IDFTIDSDLRL; |
| | DFTIDSDLRLK; FTIDSDLRLKS; TIDSDLRLKSD; IDSDLRLKSDL; |
| | DSDLRLKSDLQ; SDLRLKSDLQA; DLRLKSDLQAI; LRLKSDLQAIS; |
| | RLKSDLQAISG; LKSDLQAISGS; KSDLQAISGSN; SDLQAISGSNS; |
| | DLQAISGSNSI; LQAISGSNSIS; QAISGSNSISY; AISGSNSISYT; |
| | ISGSNSISYTD; SGSNSISYTDE; GSNSISYTDEI; SNSISYTDEIE; |
| | NSISYTDEIEE; SISYTDEIEEE; ISYTDEIEEED; SYTDEIEEEDY; |
| | YTDEIEEEDYD; TDEIEEEDYDQ; DEIEEEDYDQY; EIEEEDYDQYS; |
| | IEEEDYDQYSL; EEEDYDQYSLE; EEDYDQYSLEE; EDYDQYSLEED; |
| | DYDQYSLEEDY; YDQYSLEEDYY; DQYSLEEDYYY; QYSLEEDYYYD; |
| | YSLEEDYYYDG; SLEEDYYYDGE; LEEDYYYDGET; EEDYYYDGETR; |
| | EDYYYDGETRL; DYYYDGETRLS; YYYDGETRLSN; YYDGETRLSNR; |
| | YDGETRLSNRY; DGETRLSNRYE; GETRLSNRYES; ETRLSNRYESY; |
| | TRLSNRYESYL; RLSNRYESYLE; LSNRYESYLEG; SNRYESYLEGV; |
| | NRYESYLEGVK; RYESYLEGVKY; YESYLEGVKYN; ESYLEGVKYNV; |
| | SYLEGVKYNVS; YLEGVKYNVSS; LEGVKYNVSSA; EGVKYNVSSAI; |
| | GVKYNVSSAIK; VKYNVSSAIKT; KYNVSSAIKTI; YNVSSAIKTIV; |
| | NVSSAIKTIVK; VSSAIKTIVKI; SSAIKTIVKIY; SAIKTIVKIYD; |
| | AIKTIVKIYDN; IKTIVKIYDNY; KTIVKIYDNYT; TIVKIYDNYTL; |
| | IVKIYDNYTLL; VKIYDNYTLLS; KIYDNYTLLST; IYDNYTLLSTK; |
| | YDNYTLLSTKQ; DNYTLLSTKQT; NYTLLSTKQTQ; YTLLSTKQTQM; |
| | TLLSTKQTQMY; LLSTKQTQMYS; LSTKQTQMYST; STKQTQMYSTR; |
| | TKQTQMYSTRL; KQTQMYSTRLD; QTQMYSTRLDN; TQMYSTRLDNL; |

| | |
|---|---|
| | QMYSTRLDNLA; MYSTRLDNLAK; YSTRLDNLAKA; STRLDNLAKAK; TRLDNLAKAKA; RLDNLAKAKAR; LDNLAKAKARE; DNLAKAKAREE; NLAKAKAREEA; LAKAKAREEAK; AKAKAREEAKK; KAKAREEAKKF; AKAREEAKKFT; KAREEAKKFTK; AREEAKKFTKE; REEAKKFTKEE; EEAKKFTKEEL; EAKKFTKEELE; AKKFTKEELEK; KKFTKEELEKD; KFTKEELEKDL; FTKEELEKDLK; TKEELEKDLKT; KEELEKDLKTL; EELEKDLKTLL; ELEKDLKTLLN; LEKDLKTLLNY; EKDLKTLLNYI; KDLKTLLNYIQ; DLKTLLNYIQV; LKTLLNYIQVS; KTLLNYIQVSA; TLLNYIQVSAR; LLNYIQVSART; LNYIQVSARTA; NYIQVSARTAT; YIQVSARTATN; IQVSARTATNF; QVSARTATNFV; VSARTATNFVY; SARTATNFVYA; ARTATNFVYAR; RTATNFVYARE; TATNFVYAREI; ATNFVYAREIY; TNFVYAREIYS; NFVYAREIYSK; FVYAREIYSKR; VYAREIYSKRK; YAREIYSKRKL; AREIYSKRKLD; REIYSKRKLDA; EIYSKRKLDAI; IYSKRKLDAIE; YSKRKLDAIET; SKRKLDAIETE; KRKLDAIETEI; RKLDAIETEIK; KLDAIETEIKN; LDAIETEIKNL; DAIETEIKNLI; AIETEIKNLIL; IETEIKNLILK; ETEIKNLILKI; TEIKNLILKIK; EIKNLILKIKG; IKNLILKIKGQ; KNLILKIKGQS; NLILKIKGQSD; LILKIKGQSDL; ILKIKGQSDLY; LKIKGQSDLYE; KIKGQSDLYEA; IKGQSDLYEAY; KGQSDLYEAYK; GQSDLYEAYKA; QSDLYEAYKAI; SDLYEAYKAIV; DLYEAYKAIVR; LYEAYKAIVRS; YEAYKAIVRSI; EAYKAIVRSIL; AYKAIVRSILL; YKAIVRSILLM; KAIVRSILLMK; AIVRSILLMKD; IVRSILLMKDS; VRSILLMKDSL; RSILLMKDSLK; SILLMKDSLKI; ILLMKDSLKII; LLMKDSLKIIE; LMKDSLKIIEI; MKDSLKIIEIV; KDSLKIIEIVI; DSLKIIEIVID; SLKIIEIVIDK; LKIIEIVIDKN; KIIEIVIDKNG; IIEIVIDKNGV; IEIVIDKNGVW; EIVIDKNGVWY; |
| Seq 44 | SEQ ID NO 52425-54050/ 8 mers: MKKVKSKY; KKVKSKYL; KVKSKYLA; VKSKYLAL; KSKYLALG; SKYLALGL; KYLALGLL; YLALGLLF; LALGLLFG; ALGLLFGF; LGLLFGFI; GLLFGFIS; LLFGFISC; LFGFISCD; FGFISCDL; GFISCDLF; FISCDLFI; ISCDLFIR; SCDLFIRY; CDLFIRYE; DLFIRYEM; LFIRYEMK; FIRYEMKE; IRYEMKEE; RYEMKEES; YEMKEESP; EMKEESPG; MKEESPGL; KEESPGLF; EESPGLFD; ESPGLFDK; SPGLFDKG; PGLFDKGN; GLFDKGNS; LFDKGNSI; FDKGNSIL; DKGNSILE; KGNSILET; GNSILETS; NSILETSE; SILETSEE; ILETSEES; LETSEESI; ETSEESIK; TSEESIKK; SEESIKKP; EESIKKPM; ESIKKPMN; SIKKPMNK; IKKPMNKK; KKPMNKKG; KPMNKKGK; PMNKKGKG; MNKKGKGK; NKKGKGKI; KKGKGKIA; KGKGKIAR; GKGKIARK; KGKIARKN; GKIARKNG; KIARKNGK; IARKNGKS; ARKNGKSK; RKNGKSKV; KNGKSKVS; NGKSKVSG; GKSKVSGK; KSKVSGKE; SKVSGKEP; KVSGKEPF; VSGKEPFI; SGKEPFIH; GKEPFIHS; KEPFIHSF; EPFIHSFK; PFIHSFKR; FIHSFKRD; IHSFKRDA; HSFKRDAA; SFKRDAAN; FKRDAANK; KRDAANKS; RDAANKSN; DAANKSNF; AANKSNFL; ANKSNFLQ; NKSNFLQK; KSNFLQKN; SNFLQKNV; NFLQKNVM; FLQKNVML; LQKNVMLE; QKNVMLEE; KNVMLEEE; NVMLEEES; VMLEEESL; MLEEESLK; LEEESLKT; EEESLKTE; EESLKTEL; ESLKTELL; SLKTELLK; LKTELLKE; KTELLKEQ; TELLKEQS; ELLKEQSE; LLKEQSET; LKEQSETR; KEQSETRK; EQSETRKE; |

QSETRKEK; SETRKEKI; ETRKEKIQ; TRKEKIQK; RKEKIQKQ;
KEKIQKQQ; EKIQKQQD; KIQKQQDE; IQKQQDEY; QKQQDEYK;
KQQDEYKG; QQDEYKGM; QDEYKGMT; DEYKGMTK; EYKGMTKG;
YKGMTKGS; KGMTKGSL; GMTKGSLN; MTKGSLNS; TKGSLNSL;
KGSLNSLS; GSLNSLSG; SLNSLSGE; LNSLSGES; NSLSGESG;
SLSGESGE; LSGESGEL; SGESGELK; GESGELKE; ESGELKET;
SGELKETI; GELKETIE; ELKETIES; LKETIESN; KETIESNE;
ETIESNEI; TIESNEID; IESNEIDI; ESNEIDIT; SNEIDITI;
NEIDITID; EIDITIDS; IDITIDSD; DITIDSDL; ITIDSDLR;
TIDSDLRP; IDSDLRPK; DSDLRPKS; SDLRPKSS; DLRPKSSL;
LRPKSSLQ; RPKSSLQD; PKSSLQDI; KSSLQDIA; SSLQDIAG;
SLQDIAGS; LQDIAGSN; QDIAGSNS; DIAGSNSI; IAGSNSIS;
AGSNSISY; GSNSISYT; SNSISYTD; NSISYTDE; SISYTDEI;
ISYTDEIE; SYTDEIEE; YTDEIEEE; TDEIEEED; DEIEEEDY;
EIEEEDYA; IEEEDYAR; EEEDYARY; EEDYARYY; EDYARYYL;
DYARYYLD; YARYYLDE; ARYYLDED; RYYLDEDD; YYLDEDDE;
YLDEDDED; LDEDDEDD; DEDDEDDE; EDDEDDEY; DDEDDEYY;
DEDDEYYE; EDDEYYED; DDEYYEDD; DEYYEDDY; EYYEDDYE;
YYEDDYEE; YEDDYEEI; EDDYEEIR; DDYEEIRL; DYEEIRLS;
YEEIRLSN; EEIRLSNR; EIRLSNRY; IRLSNRYQ; RLSNRYQS;
LSNRYQSY; SNRYQSYL; NRYQSYLE; RYQSYLEG; YQSYLEGV;
QSYLEGVK; SYLEGVKY; YLEGVKYN; LEGVKYNV; EGVKYNVD;
GVKYNVDS; VKYNVDSA; KYNVDSAI; YNVDSAIN; NVDSAINT;
VDSAINTI; DSAINTIN; SAINTINK; AINTINKI; INTINKIY;
NTINKIYD; TINKIYDT; INKIYDTY; NKIYDTYT; KIYDTYTL;
IYDTYTLF; YDTYTLFS; DTYTLFST; TYTLFSTK; YTLFSTKL;
TLFSTKLT; LFSTKLTQ; FSTKLTQM; STKLTQMY; TKLTQMYS;
KLTQMYST; LTQMYSTR; TQMYSTRL; QMYSTRLD; MYSTRLDN;
YSTRLDNL; STRLDNLA; TRLDNLAK; RLDNLAKA; LDNLAKAK;
DNLAKAKA; NLAKAKAK; LAKAKAKE; AKAKAKEE; KAKAKEEA;
AKAKEEAA; KAKEEAAK; AKEEAAKF; KEEAAKFT; EEAAKFTK;
EAAKFTKE; AAKFTKED; AKFTKEDL; KFTKEDLE; FTKEDLEK;
TKEDLEKN; KEDLEKNF; EDLEKNFK; DLEKNFKT; LEKNFKTL;
EKNFKTLL; KNFKTLLN; NFKTLLNY; FKTLLNYI; KTLLNYIQ;
TLLNYIQV; LLNYIQVS; LNYIQVSV; NYIQVSVK; YIQVSVKT;
IQVSVKTA; QVSVKTAT; VSVKTATN; SVKTATNF; VKTATNFV;
KTATNFVY; TATNFVYI; ATNFVYIN; TNFVYINE; NFVYINEM;
FVYINEMH; VYINEMHA; YINEMHAK; INEMHAKR; NEMHAKRK;
EMHAKRKL; MHAKRKLE; HAKRKLEN; AKRKLENI; KRKLENIE;
RKLENIEA; KLENIEAK; LENIEAKI; ENIEAKIK; NIEAKIKT;
IEAKIKTL; EAKIKTLI; AKIKTLIA; KIKTLIAK; IKTLIAKI;
KTLIAKIK; TLIAKIKE; LIAKIKEK; IAKIKEKS; AKIKEKSN;
KIKEKSNL; IKEKSNLY; KEKSNLYS; EKSNLYSA; KSNLYSAY;
SNLYSAYK; NLYSAYKA; LYSAYKAI; YSAYKAIV; SAYKAIVS;
AYKAIVSS; YKAIVSSI; KAIVSSIL; AIVSSILL; IVSSILLM;
VSSILLMR; SSILLMRD; SILLMRDS; ILLMRDSL; LLMRDSLK;
LMRDSLKE; MRDSLKEV; RDSLKEVQ; DSLKEVQY; SLKEVQYA;
LKEVQYAI; KEVQYAID; EVQYAIDK; VQYAIDKN; QYAIDKNG;
YAIDKNGI; AIDKNGIW; IDKNGIWY; DKNGIWYR; KNGIWYRK;
NGIWYRKL; GIWYRKLD; IWYRKLDA; WYRKLDAI; YRKLDAIE;
RKLDAIET; KLDAIETE; LDAIETEI; DAIETEIK; AIETEIKN;

IETEIKNL; ETEIKNLI; TEIKNLIL; EIKNLILK; IKNLILKI;
KNLILKIK; NLILKIKG; LILKIKGQ; ILKIKGQS; LKIKGQSD;
KIKGQSDL; IKGQSDLY; KGQSDLYE; GQSDLYEA; QSDLYEAY;
SDLYEAYK; DLYEAYKA; LYEAYKAI; YEAYKAIV; EAYKAIVR;
AYKAIVRS; YKAIVRSI; KAIVRSIL; AIVRSILL; IVRSILLM;
VRSILLMK; RSILLMKD; SILLMKDS; ILLMKDSL; LLMKDSLK;
LMKDSLKI; MKDSLKII; KDSLKIIE; DSLKIIEI; SLKIIEIV;
LKIIEIVI; KIIEIVID; IIEIVIDK; IEIVIDKN; EIVIDKNG;
IVIDKNGV; VIDKNGVW; IDKNGVWY;

9 mers:
MKKVKSKYL; KKVKSKYLA; KVKSKYLAL; VKSKYLALG; KSKYLALGL;
SKYLALGLL; KYLALGLLF; YLALGLLFG; LALGLLFGF; ALGLLFGFI;
LGLLFGFIS; GLLFGFISC; LLFGFISCD; LFGFISCDL; FGFISCDLF;
GFISCDLFI; FISCDLFIR; ISCDLFIRY; SCDLFIRYE; CDLFIRYEM;
DLFIRYEMK; LFIRYEMKE; FIRYEMKEE; IRYEMKEES; RYEMKEESP;
YEMKEESPG; EMKEESPGL; MKEESPGLF; KEESPGLFD; EESPGLFDK;
ESPGLFDKG; SPGLFDKGN; PGLFDKGNS; GLFDKGNSI; LFDKGNSIL;
FDKGNSILE; DKGNSILET; KGNSILETS; GNSILETSE; NSILETSEE;
SILETSEES; ILETSEESI; LETSEESIK; ETSEESIKK; TSEESIKKP;
SEESIKKPM; EESIKKPMN; ESIKKPMNK; SIKKPMNKK; IKKPMNKKG;
KKPMNKKGK; KPMNKKGKG; PMNKKGKGK; MNKKGKGKI; NKKGKGKIA;
KKGKGKIAR; KGKGKIARK; GKGKIARKN; KGKIARKNG; GKIARKNGK;
KIARKNGKS; IARKNGKSK; ARKNGKSKV; RKNGKSKVS; KNGKSKVSG;
NGKSKVSGK; GKSKVSGKE; KSKVSGKEP; SKVSGKEPF; KVSGKEPFI;
VSGKEPFIH; SGKEPFIHS; GKEPFIHSF; KEPFIHSFK; EPFIHSFKR;
PFIHSFKRD; FIHSFKRDA; IHSFKRDAA; HSFKRDAAN; SFKRDAANK;
FKRDAANKS; KRDAANKSN; RDAANKSNF; DAANKSNFL; AANKSNFLQ;
ANKSNFLQK; NKSNFLQKN; KSNFLQKNV; SNFLQKNVM; NFLQKNVML;
FLQKNVMLE; LQKNVMLEE; QKNVMLEEE; KNVMLEEES; NVMLEEESL;
VMLEEESLK; MLEEESLKT; LEEESLKTE; EEESLKTEL; EESLKTELL;
ESLKTELLK; SLKTELLKE; LKTELLKEQ; KTELLKEQS; TELLKEQSE;
ELLKEQSET; LLKEQSETR; LKEQSETRK; KEQSETRKE; EQSETRKEK;
QSETRKEKI; SETRKEKIQ; ETRKEKIQK; TRKEKIQKQ; RKEKIQKQQ;
KEKIQKQQD; EKIQKQQDE; KIQKQQDEY; IQKQQDEYK; QKQQDEYKG;
KQQDEYKGM; QQDEYKGMT; QDEYKGMTK; DEYKGMTKG; EYKGMTKGS;
YKGMTKGSL; KGMTKGSLN; GMTKGSLNS; MTKGSLNSL; TKGSLNSLS;
KGSLNSLSG; GSLNSLSGE; SLNSLSGES; LNSLSGESG; NSLSGESGE;
SLSGESGEL; LSGESGELK; SGESGELKE; GESGELKET; ESGELKETI;
SGELKETIE; GELKETIES; ELKETIESN; LKETIESNE; KETIESNEI;
ETIESNEID; TIESNEIDI; IESNEIDIT; ESNEIDITI; SNEIDITID;
NEIDITIDS; EIDITIDSD; IDITIDSDL; DITIDSDLR; ITIDSDLRP;
TIDSDLRPK; IDSDLRPKS; DSDLRPKSS; SDLRPKSSL; DLRPKSSLQ;
LRPKSSLQD; RPKSSLQDI; PKSSLQDIA; KSSLQDIAG; SSLQDIAGS;
SLQDIAGSN; LQDIAGSNS; QDIAGSNSI; DIAGSNSIS; IAGSNSISY;
AGSNSISYT; GSNSISYTD; SNSISYTDE; NSISYTDEI; SISYTDEIE;
ISYTDEIEE; SYTDEIEEE; YTDEIEEED; TDEIEEEDY; DEIEEEDYA;
EIEEEDYAR; IEEEDYARY; EEEDYARYY; EEDYARYYL; EDYARYYLD;
DYARYYLDE; YARYYLDED; ARYYLDEDD; RYYLDEDDE; YYLDEDDED;
YLDEDDEDD; LDEDDEDDE; DEDDEDDEY; EDDEDDEYY; DDEDDEYYE;
DEDDEYYED; EDDEYYEDD; DDEYYEDDY; DEYYEDDYE; EYYEDDYEE;

YYEDDYEEI; YEDDYEEIR; EDDYEEIRL; DDYEEIRLS; DYEEIRLSN;
YEEIRLSNR; EEIRLSNRY; EIRLSNRYQ; IRLSNRYQS; RLSNRYQSY;
LSNRYQSYL; SNRYQSYLE; NRYQSYLEG; RYQSYLEGV; YQSYLEGVK;
QSYLEGVKY; SYLEGVKYN; YLEGVKYNV; LEGVKYNVD; EGVKYNVDS;
GVKYNVDSA; VKYNVDSAI; KYNVDSAIN; YNVDSAINT; NVDSAINTI;
VDSAINTIN; DSAINTINK; SAINTINKI; AINTINKIY; INTINKIYD;
NTINKIYDT; TINKIYDTY; INKIYDTYT; NKIYDTYTL; KIYDTYTLF;
IYDTYTLFS; YDTYTLFST; DTYTLFSTK; TYTLFSTKL; YTLFSTKLT;
TLFSTKLTQ; LFSTKLTQM; FSTKLTQMY; STKLTQMYS; TKLTQMYST;
KLTQMYSTR; LTQMYSTRL; TQMYSTRLD; QMYSTRLDN; MYSTRLDNL;
YSTRLDNLA; STRLDNLAK; TRLDNLAKA; RLDNLAKAK; LDNLAKAKA;
DNLAKAKAK; NLAKAKAKE; LAKAKAKEE; AKAKAKEEA; KAKAKEEAA;
AKAKEEAAK; KAKEEAAKF; AKEEAAKFT; KEEAAKFTK; EEAAKFTKE;
EAAKFTKED; AAKFTKEDL; AKFTKEDLE; KFTKEDLEK; FTKEDLEKN;
TKEDLEKNF; KEDLEKNFK; EDLEKNFKT; DLEKNFKTL; LEKNFKTLL;
EKNFKTLLN; KNFKTLLNY; NFKTLLNYI; FKTLLNYIQ; KTLLNYIQV;
TLLNYIQVS; LLNYIQVSV; LNYIQVSVK; NYIQVSVKT; YIQVSVKTA;
IQVSVKTAT; QVSVKTATN; VSVKTATNF; SVKTATNFV; VKTATNFVY;
KTATNFVYI; TATNFVYIN; ATNFVYINE; TNFVYINEM; NFVYINEMH;
FVYINEMHA; VYINEMHAK; YINEMHAKR; INEMHAKRK; NEMHAKRKL;
EMHAKRKLE; MHAKRKLEN; HAKRKLENI; AKRKLENIE; KRKLENIEA;
RKLENIEAK; KLENIEAKI; LENIEAKIK; ENIEAKIKT; NIEAKIKTL;
IEAKIKTLI; EAKIKTLIA; AKIKTLIAK; KIKTLIAKI; IKTLIAKIK;
KTLIAKIKE; TLIAKIKEK; LIAKIKEKS; IAKIKEKSN; AKIKEKSNL;
KIKEKSNLY; IKEKSNLYS; KEKSNLYSA; EKSNLYSAY; KSNLYSAYK;
SNLYSAYKA; NLYSAYKAI; LYSAYKAIV; YSAYKAIVS; SAYKAIVSS;
AYKAIVSSI; YKAIVSSIL; KAIVSSILL; AIVSSILLM; IVSSILLMR;
VSSILLMRD; SSILLMRDS; SILLMRDSL; ILLMRDSLK; LLMRDSLKE;
LMRDSLKEV; MRDSLKEVQ; RDSLKEVQY; DSLKEVQYA; SLKEVQYAI;
LKEVQYAID; KEVQYAIDK; EVQYAIDKN; VQYAIDKNG; QYAIDKNGI;
YAIDKNGIW; AIDKNGIWY; IDKNGIWYR; DKNGIWYRK; KNGIWYRKL;
NGIWYRKLD; GIWYRKLDA; IWYRKLDAI; WYRKLDAIE; YRKLDAIET;
RKLDAIETE; KLDAIETEI; LDAIETEIK; DAIETEIKN; AIETEIKNL;
IETEIKNLI; ETEIKNLIL; TEIKNLILK; EIKNLILKI; IKNLILKIK;
KNLILKIKG; NLILKIKGQ; LILKIKGQS; ILKIKGQSD; LKIKGQSDL;
KIKGQSDLY; IKGQSDLYE; KGQSDLYEA; GQSDLYEAY; QSDLYEAYK;
SDLYEAYKA; DLYEAYKAI; LYEAYKAIV; YEAYKAIVR; EAYKAIVRS;
AYKAIVRSI; YKAIVRSIL; KAIVRSILL; AIVRSILLM; IVRSILLMK;
VRSILLMKD; RSILLMKDS; SILLMKDSL; ILLMKDSLK; LLMKDSLKI;
LMKDSLKII; MKDSLKIIE; KDSLKIIEI; DSLKIIEIV; SLKIIEIVI;
LKIIEIVID; KIIEIVIDK; IIEIVIDKN; IEIVIDKNG; EIVIDKNGV;
IVIDKNGVW; VIDKNGVWY;

10 mers:
MKKVKSKYLA; KKVKSKYLAL; KVKSKYLALG; VKSKYLALGL;
KSKYLALGLL; SKYLALGLLF; KYLALGLLFG; YLALGLLFGF;
LALGLLFGFI; ALGLLFGFIS; LGLLFGFISC; GLLFGFISCD;
LLFGFISCDL; LFGFISCDLF; FGFISCDLFI; GFISCDLFIR;
FISCDLFIRY; ISCDLFIRYE; SCDLFIRYEM; CDLFIRYEMK;
DLFIRYEMKE; LFIRYEMKEE; FIRYEMKEES; IRYEMKEESP;
RYEMKEESPG; YEMKEESPGL; EMKEESPGLF; MKEESPGLFD;

820

| | |
|---|---|
| | KEESPGLFDK; EESPGLFDKG; ESPGLFDKGN; SPGLFDKGNS;<br>PGLFDKGNSI; GLFDKGNSIL; LFDKGNSILE; FDKGNSILET;<br>DKGNSILETS; KGNSILETSE; GNSILETSEE; NSILETSEES;<br>SILETSEESI; ILETSEESIK; LETSEESIKK; ETSEESIKKP;<br>TSEESIKKPM; SEESIKKPMN; EESIKKPMNK; ESIKKPMNKK;<br>SIKKPMNKKG; IKKPMNKKGK; KKPMNKKGKG; KPMNKKGKGK;<br>PMNKKGKGKI; MNKKGKGKIA; NKKGKGKIAR; KKGKGKIARK;<br>KGKGKIARKN; GKGKIARKNG; KGKIARKNGK; GKIARKNGKS;<br>KIARKNGKSK; IARKNGKSKV; ARKNGKSKVS; RKNGKSKVSG;<br>KNGKSKVSGK; NGKSKVSGKE; GKSKVSGKEP; KSKVSGKEPF;<br>SKVSGKEPFI; KVSGKEPFIH; VSGKEPFIHS; SGKEPFIHSF;<br>GKEPFIHSFK; KEPFIHSFKR; EPFIHSFKRD; PFIHSFKRDA;<br>FIHSFKRDAA; IHSFKRDAAN; HSFKRDAANK; SFKRDAANKS;<br>FKRDAANKSN; KRDAANKSNF; RDAANKSNFL; DAANKSNFLQ;<br>AANKSNFLQK; ANKSNFLQKN; NKSNFLQKNV; KSNFLQKNVM;<br>SNFLQKNVML; NFLQKNVMLE; FLQKNVMLEE; LQKNVMLEEE;<br>QKNVMLEEES; KNVMLEEESL; NVMLEEESLK; VMLEEESLKT;<br>MLEEESLKTE; LEEESLKTEL; EEESLKTELL; EESLKTELLK;<br>ESLKTELLKE; SLKTELLKEQ; LKTELLKEQS; KTELLKEQSE;<br>TELLKEQSET; ELLKEQSETR; LLKEQSETRK; LKEQSETRKE;<br>KEQSETRKEK; EQSETRKEKI; QSETRKEKIQ; SETRKEKIQK;<br>ETRKEKIQKQ; TRKEKIQKQQ; RKEKIQKQQD; KEKIQKQQDE;<br>EKIQKQQDEY; KIQKQQDEYK; IQKQQDEYKG; QKQQDEYKGM;<br>KQQDEYKGMT; QQDEYKGMTK; QDEYKGMTKG; DEYKGMTKGS;<br>EYKGMTKGSL; YKGMTKGSLN; KGMTKGSLNS; GMTKGSLNSL;<br>MTKGSLNSLS; TKGSLNSLSG; KGSLNSLSGE; GSLNSLSGES;<br>SLNSLSGESG; LNSLSGESGE; NSLSGESGEL; SLSGESGELK;<br>LSGESGELKE; SGESGELKET; GESGELKETI; ESGELKETIE;<br>SGELKETIES; GELKETIESN; ELKETIESNE; LKETIESNEI;<br>KETIESNEID; ETIESNEIDI; TIESNEIDIT; IESNEIDITI;<br>ESNEIDITID; SNEIDITIDS; NEIDITIDSD; EIDITIDSDL;<br>IDITIDSDLR; DITIDSDLRP; ITIDSDLRPK; TIDSDLRPKS;<br>IDSDLRPKSS; DSDLRPKSSL; SDLRPKSSLQ; DLRPKSSLQD;<br>LRPKSSLQDI; RPKSSLQDIA; PKSSLQDIAG; KSSLQDIAGS;<br>SSLQDIAGSN; SLQDIAGSNS; LQDIAGSNSI; QDIAGSNSIS;<br>DIAGSNSISY; IAGSNSISYT; AGSNSISYTD; GSNSISYTDE;<br>SNSISYTDEI; NSISYTDEIE; SISYTDEIEE; ISYTDEIEEE;<br>SYTDEIEEED; YTDEIEEEDY; TDEIEEEDYA; DEIEEEDYAR;<br>EIEEEDYARY; IEEEDYARYY; EEEDYARYYL; EEDYARYYLD;<br>EDYARYYLDE; DYARYYLDED; YARYYLDEDD; ARYYLDEDDE;<br>RYYLDEDDED; YYLDEDDEDD; YLDEDDEDDE; LDEDDEDDEY;<br>DEDDEDDEYY; EDDEDDEYYE; DDEDDEYYED; DEDDEYYEDD;<br>EDDEYYEDDY; DDEYYEDDYE; DEYYEDDYEE; EYYEDDYEEI;<br>YYEDDYEEIR; YEDDYEEIRL; EDDYEEIRLS; DDYEEIRLSN;<br>DYEEIRLSNR; YEEIRLSNRY; EEIRLSNRYQ; EIRLSNRYQS;<br>IRLSNRYQSY; RLSNRYQSYL; LSNRYQSYLE; SNRYQSYLEG;<br>NRYQSYLEGV; RYQSYLEGVK; YQSYLEGVKY; QSYLEGVKYN;<br>SYLEGVKYNV; YLEGVKYNVD; LEGVKYNVDS; EGVKYNVDSA;<br>GVKYNVDSAI; VKYNVDSAIN; KYNVDSAINT; YNVDSAINTI;<br>NVDSAINTIN; VDSAINTINK; DSAINTINKI; SAINTINKIY;<br>AINTINKIYD; INTINKIYDT; NTINKIYDTY; TINKIYDTYT; |

INKIYDTYTL; NKIYDTYTLF; KIYDTYTLFS; IYDTYTLFST;
YDTYTLFSTK; DTYTLFSTKL; TYTLFSTKLT; YTLFSTKLTQ;
TLFSTKLTQM; LFSTKLTQMY; FSTKLTQMYS; STKLTQMYST;
TKLTQMYSTR; KLTQMYSTRL; LTQMYSTRLD; TQMYSTRLDN;
QMYSTRLDNL; MYSTRLDNLA; YSTRLDNLAK; STRLDNLAKA;
TRLDNLAKAK; RLDNLAKAKA; LDNLAKAKAK; DNLAKAKAKE;
NLAKAKAKEE; LAKAKAKEEA; AKAKAKEEAA; KAKAKEEAAK;
AKAKEEAAKF; KAKEEAAKFT; AKEEAAKFTK; KEEAAKFTKE;
EEAAKFTKED; EAAKFTKEDL; AAKFTKEDLE; AKFTKEDLEK;
KFTKEDLEKN; FTKEDLEKNF; TKEDLEKNFK; KEDLEKNFKT;
EDLEKNFKTL; DLEKNFKTLL; LEKNFKTLLN; EKNFKTLLNY;
KNFKTLLNYI; NFKTLLNYIQ; FKTLLNYIQV; KTLLNYIQVS;
TLLNYIQVSV; LLNYIQVSVK; LNYIQVSVKT; NYIQVSVKTA;
YIQVSVKTAT; IQVSVKTATN; QVSVKTATNF; VSVKTATNFV;
SVKTATNFVY; VKTATNFVYI; KTATNFVYIN; TATNFVYINE;
ATNFVYINEM; TNFVYINEMH; NFVYINEMHA; FVYINEMHAK;
VYINEMHAKR; YINEMHAKRK; INEMHAKRKL; NEMHAKRKLE;
EMHAKRKLEN; MHAKRKLENI; HAKRKLENIE; AKRKLENIEA;
KRKLENIEAK; RKLENIEAKI; KLENIEAKIK; LENIEAKIKT;
ENIEAKIKTL; NIEAKIKTLI; IEAKIKTLIA; EAKIKTLIAK;
AKIKTLIAKI; KIKTLIAKIK; IKTLIAKIKE; KTLIAKIKEK;
TLIAKIKEKS; LIAKIKEKSN; IAKIKEKSNL; AKIKEKSNLY;
KIKEKSNLYS; IKEKSNLYSA; KEKSNLYSAY; EKSNLYSAYK;
KSNLYSAYKA; SNLYSAYKAI; NLYSAYKAIV; LYSAYKAIVS;
YSAYKAIVSS; SAYKAIVSSI; AYKAIVSSIL; YKAIVSSILL;
KAIVSSILLM; AIVSSILLMR; IVSSILLMRD; VSSILLMRDS;
SSILLMRDSL; SILLMRDSLK; ILLMRDSLKE; LLMRDSLKEV;
LMRDSLKEVQ; MRDSLKEVQY; RDSLKEVQYA; DSLKEVQYAI;
SLKEVQYAID; LKEVQYAIDK; KEVQYAIDKN; EVQYAIDKNG;
VQYAIDKNGI; QYAIDKNGIW; YAIDKNGIWY; AIDKNGIWYR;
IDKNGIWYRK; DKNGIWYRKL; KNGIWYRKLD; NGIWYRKLDA;
GIWYRKLDAI; IWYRKLDAIE; WYRKLDAIET; YRKLDAIETE;
RKLDAIETEI; KLDAIETEIK; LDAIETEIKN; DAIETEIKNL;
AIETEIKNLI; IETEIKNLIL; ETEIKNLILK; TEIKNLILKI;
EIKNLILKIK; IKNLILKIKG; KNLILKIKGQ; NLILKIKGQS;
LILKIKGQSD; ILKIKGQSDL; LKIKGQSDLY; KIKGQSDLYE;
IKGQSDLYEA; KGQSDLYEAY; GQSDLYEAYK; QSDLYEAYKA;
SDLYEAYKAI; DLYEAYKAIV; LYEAYKAIVR; YEAYKAIVRS;
EAYKAIVRSI; AYKAIVRSIL; YKAIVRSILL; KAIVRSILLM;
AIVRSILLMK; IVRSILLMKD; VRSILLMKDS; RSILLMKDSL;
SILLMKDSLK; ILLMKDSLKI; LLMKDSLKII; LMKDSLKIIE;
MKDSLKIIEI; KDSLKIIEIV; DSLKIIEIVI; SLKIIEIVID;
LKIIEIVIDK; KIIEIVIDKN; IIEIVIDKNG; IEIVIDKNGV;
EIVIDKNGVW; IVIDKNGVWY;

11 mers:
MKKVKSKYLAL; KKVKSKYLALG; KVKSKYLALGL; VKSKYLALGLL;
KSKYLALGLLF; SKYLALGLLFG; KYLALGLLFGF; YLALGLLFGFI;
LALGLLFGFIS; ALGLLFGFISC; LGLLFGFISCD; GLLFGFISCDL;
LLFGFISCDLF; LFGFISCDLFI; FGFISCDLFIR; GFISCDLFIRY;
FISCDLFIRYE; ISCDLFIRYEM; SCDLFIRYEMK; CDLFIRYEMKE;

| | | | |
|---|---|---|---|
| DLFIRYEMKEE; | LFIRYEMKEES; | FIRYEMKEESP; | IRYEMKEESPG; |
| RYEMKEESPGL; | YEMKEESPGLF; | EMKEESPGLFD; | MKEESPGLFDK; |
| KEESPGLFDKG; | EESPGLFDKGN; | ESPGLFDKGNS; | SPGLFDKGNSI; |
| PGLFDKGNSIL; | GLFDKGNSILE; | LFDKGNSILET; | FDKGNSILETS; |
| DKGNSILETSE; | KGNSILETSEE; | GNSILETSEES; | NSILETSEESI; |
| SILETSEESIK; | ILETSEESIKK; | LETSEESIKKP; | ETSEESIKKPM; |
| TSEESIKKPMN; | SEESIKKPMNK; | EESIKKPMNKK; | ESIKKPMNKKG; |
| SIKKPMNKKGK; | IKKPMNKKGKG; | KKPMNKKGKGK; | KPMNKKGKGKI; |
| PMNKKGKGKIA; | MNKKGKGKIAR; | NKKGKGKIARK; | KKGKGKIARKN; |
| KGKGKIARKNG; | GKGKIARKNGK; | KGKIARKNGKS; | GKIARKNGKSK; |
| KIARKNGKSKV; | IARKNGKSKVS; | ARKNGKSKVSG; | RKNGKSKVSGK; |
| KNGKSKVSGKE; | NGKSKVSGKEP; | GKSKVSGKEPF; | KSKVSGKEPFI; |
| SKVSGKEPFIH; | KVSGKEPFIHS; | VSGKEPFIHSF; | SGKEPFIHSFK; |
| GKEPFIHSFKR; | KEPFIHSFKRD; | EPFIHSFKRDA; | PFIHSFKRDAA; |
| FIHSFKRDAAN; | IHSFKRDAANK; | HSFKRDAANKS; | SFKRDAANKSN; |
| FKRDAANKSNF; | KRDAANKSNFL; | RDAANKSNFLQ; | DAANKSNFLQK; |
| AANKSNFLQKN; | ANKSNFLQKNV; | NKSNFLQKNVM; | KSNFLQKNVML; |
| SNFLQKNVMLE; | NFLQKNVMLEE; | FLQKNVMLEEE; | LQKNVMLEEES; |
| QKNVMLEEESL; | KNVMLEEESLK; | NVMLEEESLKT; | VMLEEESLKTE; |
| MLEEESLKTEL; | LEEESLKTELL; | EEESLKTELLK; | EESLKTELLKE; |
| ESLKTELLKEQ; | SLKTELLKEQS; | LKTELLKEQSE; | KTELLKEQSET; |
| TELLKEQSETR; | ELLKEQSETRK; | LLKEQSETRKE; | LKEQSETRKEK; |
| KEQSETRKEKI; | EQSETRKEKIQ; | QSETRKEKIQK; | SETRKEKIQKQ; |
| ETRKEKIQKQQ; | TRKEKIQKQQD; | RKEKIQKQQDE; | KEKIQKQQDEY; |
| EKIQKQQDEYK; | KIQKQQDEYKG; | IQKQQDEYKGM; | QKQQDEYKGMT; |
| KQQDEYKGMTK; | QQDEYKGMTKG; | QDEYKGMTKGS; | DEYKGMTKGSL; |
| EYKGMTKGSLN; | YKGMTKGSLNS; | KGMTKGSLNSL; | GMTKGSLNSLS; |
| MTKGSLNSLSG; | TKGSLNSLSGE; | KGSLNSLSGES; | GSLNSLSGESG; |
| SLNSLSGESGE; | LNSLSGESGEL; | NSLSGESGELK; | SLSGESGELKE; |
| LSGESGELKET; | SGESGELKETI; | GESGELKETIE; | ESGELKETIES; |
| SGELKETIESN; | GELKETIESNE; | ELKETIESNEI; | LKETIESNEID; |
| KETIESNEIDI; | ETIESNEIDIT; | TIESNEIDITI; | IESNEIDITID; |
| ESNEIDITIDS; | SNEIDITIDSD; | NEIDITIDSDL; | EIDITIDSDLR; |
| IDITIDSDLRP; | DITIDSDLRPK; | ITIDSDLRPKS; | TIDSDLRPKSS; |
| IDSDLRPKSSL; | DSDLRPKSSLQ; | SDLRPKSSLQD; | DLRPKSSLQDI; |
| LRPKSSLQDIA; | RPKSSLQDIAG; | PKSSLQDIAGS; | KSSLQDIAGSN; |
| SSLQDIAGSNS; | SLQDIAGSNSI; | LQDIAGSNSIS; | QDIAGSNSISY; |
| DIAGSNSISYT; | IAGSNSISYTD; | AGSNSISYTDE; | GSNSISYTDEI; |
| SNSISYTDEIE; | NSISYTDEIEE; | SISYTDEIEEE; | ISYTDEIEEED; |
| SYTDEIEEEDY; | YTDEIEEEDYA; | TDEIEEEDYAR; | DEIEEEDYARY; |
| EIEEEDYARYY; | IEEEDYARYYL; | EEEDYARYYLD; | EEDYARYYLDE; |
| EDYARYYLDED; | DYARYYLDEDD; | YARYYLDEDDE; | ARYYLDEDDED; |
| RYYLDEDDEDD; | YYLDEDDEDDE; | YLDEDDEDDEY; | LDEDDEDDEYY; |
| DEDDEDDEYYE; | EDDEDDEYYED; | DDEDDEYYEDD; | DEDDEYYEDDY; |
| EDDEYYEDDYE; | DDEYYEDDYEE; | DEYYEDDYEEI; | EYYEDDYEEIR; |
| YYEDDYEEIRL; | YEDDYEEIRLS; | EDDYEEIRLSN; | DDYEEIRLSNR; |
| DYEEIRLSNRY; | YEEIRLSNRYQ; | EEIRLSNRYQS; | EIRLSNRYQSY; |
| IRLSNRYQSYL; | RLSNRYQSYLE; | LSNRYQSYLEG; | SNRYQSYLEGV; |
| NRYQSYLEGVK; | RYQSYLEGVKY; | YQSYLEGVKYN; | QSYLEGVKYNV; |
| SYLEGVKYNVD; | YLEGVKYNVDS; | LEGVKYNVDSA; | EGVKYNVDSAI; |
| GVKYNVDSAIN; | VKYNVDSAINT; | KYNVDSAINTI; | YNVDSAINTIN; |

|  | NVDSAINTINK; VDSAINTINKI; DSAINTINKIY; SAINTINKIYD; AINTINKIYDT; INTINKIYDTY; NTINKIYDTYT; TINKIYDTYTL; INKIYDTYTLF; NKIYDTYTLFS; KIYDTYTLFST; IYDTYTLFSTK; YDTYTLFSTKL; DTYTLFSTKLT; TYTLFSTKLTQ; YTLFSTKLTQM; TLFSTKLTQMY; LFSTKLTQMYS; FSTKLTQMYST; STKLTQMYSTR; TKLTQMYSTRL; KLTQMYSTRLD; LTQMYSTRLDN; TQMYSTRLDNL; QMYSTRLDNLA; MYSTRLDNLAK; YSTRLDNLAKA; STRLDNLAKAK; TRLDNLAKAKA; RLDNLAKAKAK; LDNLAKAKAKE; DNLAKAKAKEE; NLAKAKAKEEA; LAKAKAKEEAA; AKAKAKEEAAK; KAKAKEEAAKF; AKAKEEAAKFT; KAKEEAAKFTK; AKEEAAKFTKE; KEEAAKFTKED; EEAAKFTKEDL; EAAKFTKEDLE; AAKFTKEDLEK; AKFTKEDLEKN; KFTKEDLEKNF; FTKEDLEKNFK; TKEDLEKNFKT; KEDLEKNFKTL; EDLEKNFKTLL; DLEKNFKTLLN; LEKNFKTLLNY; EKNFKTLLNYI; KNFKTLLNYIQ; NFKTLLNYIQV; FKTLLNYIQVS; KTLLNYIQVSV; TLLNYIQVSVK; LLNYIQVSVKT; LNYIQVSVKTA; NYIQVSVKTAT; YIQVSVKTATN; IQVSVKTATNF; QVSVKTATNFV; VSVKTATNFVY; SVKTATNFVYI; VKTATNFVYIN; KTATNFVYINE; TATNFVYINEM; ATNFVYINEMH; TNFVYINEMHA; NFVYINEMHAK; FVYINEMHAKR; VYINEMHAKRK; YINEMHAKRKL; INEMHAKRKLE; NEMHAKRKLEN; EMHAKRKLENI; MHAKRKLENIE; HAKRKLENIEA; AKRKLENIEAK; KRKLENIEAKI; RKLENIEAKIK; KLENIEAKIKT; LENIEAKIKTL; ENIEAKIKTLI; NIEAKIKTLIA; IEAKIKTLIAK; EAKIKTLIAKI; AKIKTLIAKIK; KIKTLIAKIKE; IKTLIAKIKEK; KTLIAKIKEKS; TLIAKIKEKSN; LIAKIKEKSNL; IAKIKEKSNLY; AKIKEKSNLYS; KIKEKSNLYSA; IKEKSNLYSAY; KEKSNLYSAYK; EKSNLYSAYKA; KSNLYSAYKAI; SNLYSAYKAIV; NLYSAYKAIVS; LYSAYKAIVSS; YSAYKAIVSSI; SAYKAIVSSIL; AYKAIVSSILL; YKAIVSSILLM; KAIVSSILLMR; AIVSSILLMRD; IVSSILLMRDS; VSSILLMRDSL; SSILLMRDSLK; SILLMRDSLKE; ILLMRDSLKEV; LLMRDSLKEVQ; LMRDSLKEVQY; MRDSLKEVQYA; RDSLKEVQYAI; DSLKEVQYAID; SLKEVQYAIDK; LKEVQYAIDKN; KEVQYAIDKNG; EVQYAIDKNGI; VQYAIDKNGIW; QYAIDKNGIWY; YAIDKNGIWYR; AIDKNGIWYRK; IDKNGIWYRKL; DKNGIWYRKLD; KNGIWYRKLDA; NGIWYRKLDAI; GIWYRKLDAIE; IWYRKLDAIET; WYRKLDAIETE; YRKLDAIETEI; RKLDAIETEIK; KLDAIETEIKN; LDAIETEIKNL; DAIETEIKNLI; AIETEIKNLIL; IETEIKNLILK; ETEIKNLILKI; TEIKNLILKIK; EIKNLILKIKG; IKNLILKIKGQ; KNLILKIKGQS; NLILKIKGQSD; LILKIKGQSDL; ILKIKGQSDLY; LKIKGQSDLYE; KIKGQSDLYEA; IKGQSDLYEAY; KGQSDLYEAYK; GQSDLYEAYKA; QSDLYEAYKAI; SDLYEAYKAIV; DLYEAYKAIVR; LYEAYKAIVRS; YEAYKAIVRSI; EAYKAIVRSIL; AYKAIVRSILL; YKAIVRSILLM; KAIVRSILLMK; AIVRSILLMKD; IVRSILLMKDS; VRSILLMKDSL; RSILLMKDSLK; SILLMKDSLKI; ILLMKDSLKII; LLMKDSLKIIE; LMKDSLKIIEI; MKDSLKIIEIV; KDSLKIIEIVI; DSLKIIEIVID; SLKIIEIVIDK; LKIIEIVIDKN; KIIEIVIDKNG; IIEIVIDKNGV; IEIVIDKNGVW; EIVIDKNGVWY; |

## Fig. 32.

| Antigen designation | HLA-chain; Class 1 | Amino acid sequence |
|---|---|---|
| NP_212517.1 ; Basic membrane protein A (bmpA) [Borrelia burgdorferi B31; Seq1 | HLA-A0101 | YSDEIDIIH; IVDAFRYGY; GSDLIWLIGY; YSDEIDIIHH; YSDEIDIIHHA; FTSNHLKTNTF |
| | HLA-A0201 | ILLESIVFL; SLGSEIPKV; LLLILLESI; NLVGMTFRA; YLAPDNVIT; RMYSDEIDI; GVDEDQAYL; KIINKEIIV; FLGGIEGEI; LLILLESIV; ILLLILLES; FLTGYIAAK; LLESIVFLS; GSGHYIIGV; YIAAKLSKT; LIGYRFSDV; FLTGYIAAKL; ILLLILLESI; RMYSDEIDII; FLGGIEGEIV; WLIGYRFSDV; LLLILLESIV; ILLESIVFLS; GLGGIGAIEV; YLAPDNVITS; LILLESIVFL; FLSCSGKGSL; YIGSFADLEA; GLKEGVVGFV; ALNIFTSNHL; LINYGLKEGV; YIIGVDEDQA; ALQNPDMKYA; MISFELEKEI; GVDEDQAYLA; YLAPDNVITST; KLINYGLKEGV; ILLLILLESIV; KMISFELEKEI; KILLLILLESI; LLILLESIVFL; SLGSEIPKVSL; WLIGYRFSDVA; YLSDLEGLKDA; ILLESIVFLSC; VLKESSSNSYL; IIDPIYSNDPI; NLSSKIINKEI; ALQNPDMKYAI; MTFRAQEGAFL; KSFNESALNGV; ELGSGHYIIGV; KLSKTGKIGFL; KISTQYIGSFA; SVATRMYSDEI; FLTGYIAAKLS; GLGGIGAIEVA; YSDEIDIIHHA; YSNDPIPANLV; IVDAFRYGYEA |
| | HLA-A0301 | IVFLSCSGK; KMISFELEK; NLSSKIINK; NIFTSNHLK; FLTGYIAAK; ALNGVKKVK; KTNTFEGGK; VVGFVRNPK; GSLGSEIPK; GIGAIEVAK; YLSDLEGLK; ALQNPDMKY; SIVFLSCSGK; TGYIAAKLSK; GVVGFVRNPK; AFLTGYIAAK; LIIDGTFDDK; SALNGVKKVK; LNIFTSNHLK; IAAKLSKTGK; GVKKVKEEFK; KGSLGSEIPK; SYLSDLEGLK; IGYRFSDVAK; FRYGYEAGAK; RYGYEAGAKY; ALNIFTSNHLK; LTGYIAAKLSK; HLKTNTFEGGK; RMYSDEIDIIH; YIAAKLSKTGK; KVAALQNPDMK; GAFLTGYIAAK; NSYLSDLEGLK; LIGYRFSDVAK; SFNESALNGVK; SLIIDGTFDDK |
| | HLA-A1101 | KMISFELEK; IVFLSCSGK; NIFTSNHLK; KTNTFEGGK; VVGFVRNPK; GSLGSEIPK; GIGAIEVAK; AALQNPDMK; NLSSKIINK; IVDAFRYGY; YLSDLEGLK; GYIAAKLSK; PSNKESYEK; ALNGVKKVK; IIDGTFDDK; AAKLSKTGK; FLTGYIAAK; ESALNGVKK; KESYEKFLK; ALQNPDMKY; KEIIVPSNK; KEIDNLSSK; EAGAKYANK; SIVFLSCSGK; GVVGFVRNPK; LIIDGTFDDK; SALNGVKKVK; GVKKVKEEFK; VAALQNPDMK; SYLSDLEGLK; GSFADLEAGR; IAAKLSKTGK; TGYIAAKLSK; AFLTGYIAAK; IGYRFSDVAK; ITSTTKDVGR; KGSLGSEIPK; LNIFTSNHLK; PANLVGMTFR; GSDLIWLIGY; AALQNPDMKY; ALNIFTSNHLK; KVAALQNPDMK; GAFLTGYIAAK; NSYLSDLEGLK; LTGYIAAKLSK; SLIIDGTFDDK; YIAAKLSKTGK; IVPSNKESYEK; SFNESALNGVK; ESIVFLSCSGK; LIGYRFSDVAK; GSDLIWLIGYR; RAQEGAFLTGY; NTFEGGKLINY; HLKTNTFEGGK; SNKESYEKFLK |
| | HLA-A2402 | KYANKDIKI; SYEKFLKEF; QYIGSFADL; MYSDEIDII; AYLAPDNVI; SYLSDLEGL; GFVRNPKMI; KYAIIDPIY; SYEKFLKEFI; SYLSDLEGLK; IYSNDPIPANL; NYGLKEGVVGF; GFVRNPKMISF; AFLTGYIAAKL; VFLSCSGKGSL |
| | HLA-A2902 | KYAIIDPIY; ALQNPDMKY; YGYEAGAKY; KDIKISTQY; IVPSNKESY; IVDAFRYGY; GEIVDAFRY; LIWLIGYRF; IGVDEDQAY; RYGYEAGAKY; IIGVDEDQAY; VAKELGSGHY; TFEGGKLINY; EIVDAFRYGY; LLILLESIVF; FVRNPKMISF; YIIGVDEDQAY; EIIVPSNKESY; EVAKELGSGHY; VAALQNPDMKY; RAQEGAFLTGY; LVLKESSSNSY; NTFEGGKLINY |
| | HLA-A6801 | NIFTSNHLK; DLIWLIGYR; IVFLSCSGK; EAGRSVATR; ESALNGVKK; FLTGYIAAK; EGEIVDAFR; TSTTKDVGR; KTNTFEGGK; YLSDLEGLK; SFADLEAGR; DVAKVAALQ; VVGFVRNPK; EAGAKYANK; KMISFELEK; |

| | | |
|---|---|---|
| | | NLSSKIINK; EFKIELVLK; DNVITSTTK; ESSSNSYLS; SIVFLSCSGK; ITSTTKDVGR; EEFKIELVLK; GSFADLEAGR; LNIFTSNHLK; GVVGFVRNPK; EIVDAFRYGY; LIIDGTFDDK; IAAKLSKTGK; GVKKVKEEFK; VAALQNPDMK; PANLVGMTFR; MTFRAQEGAF; TGYIAAKLSK; IGYRFSDVAK; MKYAIIDPIY; LEAGRSVATR; YEAGAKYANK; IEGEIVDAFR; ESIVFLSCSGK; NSYLSDLEGLK; IPANLVGMTFR; YIAAKLSKTGK; ESALNGVKKVK; LTGYIAAKLSK; IVPSNKESYEK; NPKMISFELEK; DLEAGRSVATR; GIEGEIVDAFR; GAFLTGYIAAK; VITSTTKDVGR; HLKTNTFEGGK; ALNIFTSNHLK; KVAALQNPDMK; MTFRAQEGAFL; NTFEGGKLINY; EVAKELGSGHY; IGSFADLEAGR; EIIVPSNKESY; LIGYRFSDVAK; SLIIDGTFDDK; EGVVGFVRNPK; YIIGVDEDQAY; SFNESALNGVK; GSDLIWLIGYR |
| | HLA-B0702 | APDNVITST; IPANLVGMT; NPKMISFEL; DPIYSNDPI; TTKDVGRAL; NPDMKYAII; IPANLVGMTF; FVRNPKMISF; APDNVITSTT; VPSNKESYEKF; RALNIFTSNHL; HAAGLGGIGAI |
| | HLA-B0801 | NPKMISFEL; FVRNPKMISF; |
| | HLA-B1501 | GLKEGVVGF; YGYEAGAKY; ALQNPDMKY; ISTQYIGSF; IGVDEDQAY; LILLESIVF; TFRAQEGAF; LSKTGKIGF; TTKDVGRAL; VSLIIDGTF; SNHLKTNTF; VLKESSSNSY; FVRNPKMISF; LLILLESIVF; AQEGAFLTGY; VAKELGSGHY; MTFRAQEGAF; KLSKTGKIGF; TQYIGSFADL; KVSLIIDGTF; IIVPSNKESY; KISTQYIGSF; TSNHLKTNTF; YGLKEGVVGF; IIGVDEDQAY; KVAALQNPDM; LQNPDMKYAI; MKYAIIDPIY; FLSCSGKGSL; YIIGVDEDQAY; LVLKESSSNSY; GMTFRAQEGAF; RAQEGAFLTGY; FTSNHLKTNTF; LLLILLESIVF; NTFEGGKLINY; VLKESSSNSYL; DMKYAIIDPIY; GGIEGEIVDAF; RMYSDEIDIIH; EIIVPSNKESY; VAALQNPDMKY; EVAKELGSGHY |
| | HLA-B2705 | FRYGYEAGA; YRFSDVAKV; VRNPKMISF; GRSVATRMY; FRAQEGAFL; FRYGYEAGAK; YRFSDVAKVA; YRFSDVAKVAA; GRALNIFTSNH |
| | HLA-B3501 | IGVDEDQAY; YGYEAGAKY; LIWLIGYRF; NPKMISFEL; LILLESIVF; DPIYSNDPI; PANLVGMTF; VAALQNPDM; DVGRALNIF; IPANLVGMT; TFRAQEGAF; LKESSSNSY; DPIPANLVG; YSDEIDIIH; VSLIIDGTF; IEGEIVDAF; IPANLVGMTF; DPIPANLVGM; MTFRAQEGAF; MKYAIIDPIY; EIVDAFRYGY; IIGVDEDQAY; AALQNPDMKY; EAGRSVATRM; LLILLESIVF; FSDVAKVAAL; TSNHLKTNTF; IIVPSNKESY; KVSLIIDGTF; DLIWLIGYRF; VAKELGSGHY; FVRNPKMISF; YIIGVDEDQAY; EAGRSVATRMY; FTSNHLKTNTF; VAALQNPDMKY; LVLKESSSNSY; VPSNKESYEKF; DPIYSNDPIPA; RAQEGAFLTGY; FRYGYEAGAKY; DMKYAIIDPIY; DPIPANLVGMT; LLLILLESIVF; EVAKELGSGHY; HAAGLGGIGAI; EIIVPSNKESY |
| | HLA-B4403 | EEFKIELVL; GEIVDAFRY; SEIPKVSLI; DEIDIIHHA; SEIPKVSLII; EEFKIELVLK; DEIDIIHHAA; KEIDNLSSKI; GEIVDAFRYGY; EEFKIELVLKE; KEIIVPSNKES; KEIDNLSSKII; KESYEKFLKEF; SEIPKVSLIID |
| | HLA-B5101 | IPKVSLIID; IPANLVGMT; DPIYSNDPI; IPANLVGMTF; IPKVSLIIDG; DPIPANLVGM; IPKVSLIIDGT |
| | HLA-B5701 | |
| NP_212516.1 ; Basic membrane protein B (bmpB) [Borrelia burgdorferi B31; Seq2 | HLA-A0101 | FSCAISGVY; SSENPKISY; IVDAFRYGY; GSDLIWLVGY; ETAKNLGDGY; VSSENPKISY; NKDIEIISEY; ETAKNLGDGYY |
| | HLA-A0201 | MLTDASLLV; NLIAVVFRV; YMLTDASLL; KMYSKGIDV; RIVIFIFGI; YLAPKNFIT; NIGDALYLI; GLRDGVIGL; NLGDGYYVI; KISMLVDGV; FIFGILLTS; QIPENLIAV; KIINKEIIV; VIFIFGILL; FITSVIKNI; FLAGYIAAK; LIWLVGYML; VLDDKSFNS; KGIDVIHFA; GIDVIHFAA; SANEALLRL; ISGVYSSYV; NGSDLIWLV; YANKDIEII; YMLTDASLLV; KMYSKGIDVI; AISGVYSSYV; ALYLITGEYI; KISYGIIDPI; WLVGYMLTDA; LLVSSENPKI; YLAPKNFITS; VQIPENLIAV; GLAGIGVIET; FAAGLAGIGV; QIPENLIAVV; |

| | | |
|---|---|---|
| | | MLTDASLLVS; FIFGILLTSC; GVYSSYVSDL; EVFSCAISGV; IVIFIFGILL; LAPKNFITSV; RIVIFIFGIL; VLDDKSFNSS; FIGGMKGNIV; FLAGYIAAKK; YLAPKNFITSV; VLDDKSFNSSA; GLPNANEFEYI; NLGDGYYVIGA; SLLVSSENPKI; GLAGIGVIETA; MLTDASLLVSS; RIVIFIFGILL; LLTSCFSRNGI; YMLTDASLLVS; GIIDPIYGDDV; CAISGVYSSYV; VQIPENLIAVV; GIDVIHFAAGL; FLAGYIAAKKS; QMGLRDGVIGL; VVFRVEQGAFL; MLVDGVLDDKS; VIGADQDQSYL; TIASKMYSKGI |
| | HLA-A0301 | FLAGYIAAK; TIASKMYSK; KIGFIGGMK; VLERKIINK; ILLTSCFSR; EIFIKQILK; ALYLITGEY; LAGYIAAKK; AAKKSFSGK; LLVSSENPK; AISGVYSSY; SSANEALLR; KMYSKGIDV; KNFITSVIK; GIGVIETAK; RTIASKMYSK; FLAGYIAAKK; KVLERKIINK; SLLVSSENPK; AFLAGYIAAK; MLVDGVLDDK; IAAKKSFSGK; YEIFIKQILK; GKIGFIGGMK; GILLTSCFSR; SANEALLRLK; LYLITGEYIK; FRYGYESGAK; CAISGVYSSY; SRNGIESSSK; VDIGRTIASK; RYGYESGAKY; RNGIESSSKK; YESGAKYANK; AGIGVIETAK; PKNFITSVIK; FEYIKVLERK; VFSCAISGVY; ALYLITGEYIK; YIAAKKSFSGK; KMYSKGIDVIH; SSANEALLRLK; FSRNGIESSSK; SSYVSDLDNLK; GAFLAGYIAAK; SANEALLRLKK; AFLAGYIAAKK; ASLLVSSENPK; EVFSCAISGVY; KISYGIIDPIY; RVEQGAFLAGY; SMLVDGVLDDK; LVSSENPKISY |
| | HLA-A1101 | TIASKMYSK; SSANEALLR; YVSDLDNLK; KIGFIGGMK; AAKKSFSGK; EIFIKQILK; NANEFEYIK; IVDAFRYGY; VLERKIINK; AISGVYSSY; ALYLITGEY; ILLTSCFSR; LLVSSENPK; FLAGYIAAK; LVDGVLDDK; GIGVIETAK; YLITGEYIK; LAGYIAAKK; KNFITSVIK; ESGAKYANK; SSENPKISY; RTIASKMYS; RTIASKMYSK; KVLERKIINK; SANEALLRLK; SLLVSSENPK; IAAKKSFSGK; GILLTSCFSR; AFLAGYIAAK; MLVDGVLDDK; NSSANEALLR; FLAGYIAAKK; SYVSDLDNLK; YVSDLDNLKR; ISYGIIDPIY; NQEEYEIFIK; NSFSDVDIGR; GIESSSKKIK; AGIGVIETAK; SSYVSDLDNLK; SSANEALLRLK; SANEALLRLKK; ASLLVSSENPK; ALYLITGEYIK; GAFLAGYIAAK; YIAAKKSFSGK; SMLVDGVLDDK; SVIKNIGDALY; AFLAGYIAAKK; LAGIGVIETAK; RVEQGAFLAGY; ADQDQSYLAPK; EVFSCAISGVY; LVSSENPKISY; KISYGIIDPIY; LPNANEFEYIK |
| | HLA-A2402 | VYSSYVSDL; LYLITGEYI; KYANKDIEI; EYIKNNNVW; SYLAPKNFI; GYMLTDASL; MYSKGIDVI; IFIKQILKL; SYVSDLDNL; GYIAAKKSF; EYEIFIKQI; EYIKVLERK; EYSNSFSDV; EYIKVLERKI; KYANKDIEII; GYMLTDASLL; IFGILLTSCF; NFITSVIKNI; DFPENIEEVF; EYEIFIKQIL; SYVSDLDNLK; LYLITGEYIK; EYIKNNNVWE; MYSKGIDVIHF; EYIKVLERKII; EYSNSFSDVDI; GYMLTDASLLV; SFSDVDIGRTI; EYIKNNNVWEG |
| | HLA-A2902 | SYGIIDPIY; IVPCNQEEY; FSCAISGVY; GNIVDAFRY; ALYLITGEY; YGYESGAKY; LPNANEFEY; IVDAFRYGY; SSENPKISY; VFRVEQGAF; RYGYESGAKY; GLPNANEFEY; VFSCAISGVY; CAISGVYSSY; IIVPCNQEEY; VVFRVEQGAF; VIGADQDQSY; DALYLITGEY; VIKNIGDALY; KGNIVDAFRY; TAKNLGDGYY; ISYGIIDPIY; VSSENPKISY; EVFSCAISGVY; YVIGADQDQSY; EIIVPCNQEEY; SVIKNIGDALY; LVSSENPKISY; IGLPNANEFEY; ETAKNLGDGYY; FIFGILLTSCF; SCAISGVYSSY; RVEQGAFLAGY; KISYGIIDPIY; GNIVDAFRYGY |
| | HLA-A6801 | TIASKMYSK; EIFIKQILK; YVSDLDNLK; NANEFEYIK; SSANEALLR; FLAGYIAAK; EYIKVLERK; ENLIAVVFR; LAGYIAAKK; ILLTSCFSR; YLITGEYIK; SFSDVDIGR; FSCAISGVY; ESGAKYANK; LLVSSENPK; KGNIVDAFR; DIGRTIASK; FEYIKVLER; NGIESSSKK; IVPCNQEEY; LVDGVLDDK; NSFSDVDIGR; NSSANEALLR; YVSDLDNLKR; FLAGYIAAKK; RTIASKMYSK; EFEYIKVLER; IAAKKSFSGK; ETAKNLGDGY; MKGNIVDAFR; MLVDGVLDDK; SANEALLRLK; |

| | | |
|---|---|---|
| | | ISYGIIDPIY; CAISGVYSSY; GILLTSCFSR; YEIFIKQILK; SYVSDLDNLK; NIVDAFRYGY; DALYLITGEY; NQEEYEIFIK; EVFSCAISGVY; YIAAKKSFSGK; SSYVSDLDNLK; ETAKNLGDGYY; FGILLTSCFSR; FNSSANEALLR; SSANEALLRLK; EFEYIKVLERK; GAFLAGYIAAK; SANEALLRLKK; SNSFSDVDIGR; GMKGNIVDAFR; NEFEYIKVLER; IPENLIAVVFR; SYVSDLDNLKR; SVIKNIGDALY; EYEIFIKQILK; EIIVPCNQEEY; LPNANEFEYIK; ALYLITGEYIK; FSRNGIESSSK; YVIGADQDQSY; DVDIGRTIASK; EGGKVVQMGLR; NLIAVVFRVEQ; LAGIGVIETAK; FIFGILLTSCF |
| | HLA-B0702 | APKNFITSV; IPENLIAVV; NPKISYGII; FPENIEEVF; VIKNIGDAL; APKNFITSVI; IPENLIAVVF; SVIKNIGDAL; LPNANEFEYI; AVVFRVEQGAF |
| | HLA-B0801 | YIKVLERKI; NLKRNGSDL; YIKVLERKII; |
| | HLA-B1501 | FSCAISGVY; IISEYSNSF; ALYLITGEY; AISGVYSSY; YGYESGAKY; EQGAFLAGY; FGILLTSCF; IGADQDQSY; VFRVEQGAF; KSFSGKIGF; YMLTDASLL; GLRDGVIGL; TAKNLGDGY; VQIPENLIA; KDIEIISEY; SSENPKISY; IGLPNANEF; ISMLVDGVL; GMKGNIVDAF; VVFRVEQGAF; YSKGIDVIHF; ISYGIIDPIY; VSSENPKISY; VQIPENLIAV; VIKNIGDALY; VQMGLRDGVI; TAKNLGDGYY; IIVPCNQEEY; VFSCAISGVY; VIGADQDQSY; IGRTIASKMY; CAISGVYSSY; EIISEYSNSF; VIGLPNANEF; YVIGADQDQSY; FIFGILLTSCF; GVIGLPNANEF; SVIKNIGDALY; LVSSENPKISY; VQIPENLIAVV; AVVFRVEQGAF; EVFSCAISGVY; RVEQGAFLAGY; KISYGIIDPIY; KSFNSSANEAL; ANKDIEIISEY; KMYSKGIDVIH; SCAISGVYSSY; YLAPKNFITSV; IEIISEYSNSF; QIPENLIAVVF; GGMKGNIVDAF; EIIVPCNQEEY |
| | HLA-B2705 | FRYGYESGA; FRVEQGAFL; GRTIASKMY; SRNGIESSS; KRNGSDLIW; FRYGYESGAK; SRNGIESSSK; KRNGSDLIWL; MRIVIFIFGI; SRNGIESSSKK; GRTIASKMYSK; KRNGSDLIWLV; MRIVIFIFGIL |
| | HLA-B3501 | FPENIEEVF; LPNANEFEY; TAKNLGDGY; FSCAISGVY; IISEYSNSF; IGADQDQSY; DPIYGDDVQ; MKGNIVDAF; YGYESGAKY; IPENLIAVV; IKNIGDALY; LIWLVGYML; FNSSANEAL; IGLPNANEF; IVPCNQEEY; FGILLTSCF; FRVEQGAFL; SANEALLRL; YMLTDASLL; IPENLIAVVF; DALYLITGEY; TAKNLGDGYY; CAISGVYSSY; FPENIEEVFS; ISYGIIDPIY; IIVPCNQEEY; VFSCAISGVY; NIVDAFRYGY; EIISEYSNSF; DPIYGDDVQI; VVFRVEQGAF; LPNANEFEYI; DFPENIEEVF; VIKNIGDALY; SFNSSANEAL; VPCNQEEYEI; YSKGIDVIHF; ETAKNLGDGY; FAAGLAGIGV; YVIGADQDQSY; VPCNQEEYEIF; EVFSCAISGVY; NVWEGGKVVQM; NANEFEYIKVL; LVSSENPKISY; FIFGILLTSCF; FPENIEEVFSC; LAGYIAAKKSF; FAAGLAGIGVI; RVEQGAFLAGY; SVIKNIGDALY; KISYGIIDPIY; LVDGVLDDKSF; IGLPNANEFEY; QIPENLIAVVF; FRYGYESGAKY; MKGNIVDAFRY; GVIGLPNANEF; AVVFRVEQGAF; NGSDLIWLVGY; EIIVPCNQEEY; TSVIKNIGDAL; LVGYMLTDASL; ETAKNLGDGYY |
| | HLA-B4403 | NEFEYIKVL; KEIIVPCNQ; EEYEIFIKQ; PENLIAVVF; EEVFSCAIS; EEYEIFIKQI; SENPKISYGI; NEFEYIKVLE; GEYIKNNNVW; KEIIVPCNQE; EEVFSCAISG; EEYEIFIKQIL; SENPKISYGII; IEIISEYSNSF; NEFEYIKVLER; YEIFIKQILKL; EEVFSCAISGV; KEIIVPCNQEE; NEALLRLKKDF |
| | HLA-B5101 | IPENLIAVV; NPKISYGII; LPNANEFEYI; DPIYGDDVQI; LAPKNFITSV; APKNFITSVI; IPENLIAVVF; VPCNQEEYEI; LAPKNFITSVI; FPENIEEVFSC |
| | HLA-B5701 | ISYGIIDPIY; TGEYIKNNNVW |
| NP_212518.1 ; Basic | HLA-A0101 | FSDLSVKLSY; TVDEAMTEDAY |
| | HLA-A0201 | AMTEDAYEV; RLVDEVIDL; FIFITLSLL; KIISGEIIV; GLKEGVIEI; |

| | | |
|---|---|---|
| membrane protein C (bmpC) [Borrelia burgdorferi B31; Seq3 | | GIFYANPKL; GLNQDQSYI; ITLSLLVFA; RLLTVDEAM; FLAGYIAAK; SIIKDIGKV; SVHDGVVKL; IIVPDSEYA; KSDKVVVGV; KLSYERPDI; YIAPQNVIT; KVGVIFPIA; YANPKLRLV; FMYKEDKVGV; FLAGYIAAKM; FIFITLSLLV; FLTGPMSEHV; VLNNRLVDEV; ALFMYKEDKV; VIYSISSEYI; FITLSLLVFA; WLIGYRFSDL; GLKEGVIEIV; RLVSKKAPSL; KLRDNFGIKL; FGFKAGIFYA; GLNQDQSYIA; YAFDLFKSKL; RLLTVDEAMT; IIDRGLKEGV; ISSEYINNRV; YIAPQNVITSI; FMYKEDKVGVI; KVIYSISSEYI; VIFPIAGITGL; SISSEYINNRV; IIIDRGLKEGV; LIGYRFSDLSV; VLNNRLVDEVI; YINNRVFKGGI; FLAGYIAAKMS; GIFYANPKLRL; KMSRKEKIGFL; SLAIKFRNEEA; SFYDKGYNQSV; KLRDNFGIKLI; LITKSLRPYPI; RFIFITLSLLV; KVGVIFPIAGI; GVIEIVKDPDV; GLGVYDAAKEL; IVPDSEYAFDL; SLFDKEKGKAM |
| | HLA-A0301 | KAMALFMYK; FLAGYIAAK; YIAAKMSRK; SLLVFACFK; KLRDNFGIK; GVLAHGSFY; NLFWLIGYR; VIYSISSEY; ALFMYKEDK; LAHGSFYDK; GIIIDRGLK; SLFDKEKGK; VFACFKSNK; IFYANPKLR; MSEHVKDFK; SEYAFDLFK; YAFDLFKSK; FACFKSNKK; KAMALFMYK; FLAGYIAAK; YIAAKMSRK; SLLVFACFK; KLRDNFGIK; GVLAHGSFY; NLFWLIGYR; VIYSISSEY; ALFMYKEDK; LAHGSFYDK; GIIIDRGLK; SLFDKEKGK; VFACFKSNK; IFYANPKLR; MSEHVKDFK; SEYAFDLFK; YAFDLFKSK; FACFKSNKK; LVFACFKSNKK; KSNKKSIKSDK; TLSLLVFACFK; GVLAHGSFYDK; KSLRPYPIEGK; LLVFACFKSNK; RVFKGGIIIDR; FLTGPMSEHVK; KGKAMALFMYK; KLSYERPDIYY; YIAAKMSRKEK; GLKEGVIEIVK; VSKKAPSLFDK; AMALFMYKEDK; AAFLAGYIAAK; IGYRFSDLSVK; YANPKLRLVSK; VITSIIKDIGK; IQVPKNSLAIK; AMTEDAYEVQK; VVGVLAHGSFY; VVKLRDNFGIK; RFSDLSVKLSY; ITGLGVYDAAK; SSEYINNRVFK; AGYIAAKMSRK; SLRPYPIEGKR; PLNLFWLIGYR; RDNFGIKLITK |
| | HLA-A1101 | KAMALFMYK; SLLVFACFK; SEYAFDLFK; YIAAKMSRK; GIIIDRGLK; MSEHVKDFK; TSIIKDIGK; YAFDLFKSK; GVLAHGSFY; ALFMYKEDK; LAHGSFYDK; VIYSISSEY; SLFDKEKGK; AGIFYANPK; NLFWLIGYR; AAKMSRKEK; FLAGYIAAK; ISSEYINNR; FACFKSNKK; VFACFKSNK; QSVHDGVVK; KLRDNFGIK; LSVKLSYER; GLGVYDAAK; EYINNRVFK; LVFACFKSNK; LSLLVFACFK; HVKDFKFGFK; VLAHGSFYDK; KAGIFYANPK; MTEDAYEVQK; ITSIIKDIGK; KVIYSISSEY; SISSEYINNR; MALFMYKEDK; LTGPMSEHVK; AFLAGYIAAK; SVHDGVVKLR; GYIAAKMSRK; GIFYANPKLR; GKAMALFMYK; IAAKMSRKEK; SEYINNRVFK; KAPSLFDKEK; QVPKNSLAIK; SLRPYPIEGK; DSEYAFDLFK; LSYERPDIYY; KAMALFMYKE; PMSEHVKDFK; VFACFKSNKK; PQNVITSIIK; KLITKSLRPY; NQSVHDGVVK; SKKAPSLFDK; TLSLLVFACFK; GVLAHGSFYDK; LVFACFKSNKK; AAFLAGYIAAK; SSEYINNRVFK; KSLRPYPIEGK; AMALFMYKEDK; AMTEDAYEVQK; KSNKKSIKSDK; ITGLGVYDAAK; KGKAMALFMYK; YIAAKMSRKEK; VSKKAPSLFDK; SEYAFDLFK; VVKLRDNFGIK; LVDEVIDLENK; VITSIIKDIGK; YANPKLRLVSK; LLVFACFKSNK; RVFKGGIIIDR; VVGVLAHGSFY; AGYIAAKMSRK; YSISSEYINNR; FLTGPMSEHVK; GPMSEHVKDFK; APQNVITSIIK; IGYRFSDLSVK; IVKDPDVLNNR; IQVPKNSLAIK; GLKEGVIEIVK; AFDYGDIQVPK; KLSYERPDIYY; SIIKDIGKVIY |
| | HLA-A2402 | IYSISSEYI; IYYGIIDAF; IFITLSLLV; SYIAPQNVI; RFIFITLSL; LFWLIGYRF; FYANPKLRL; EYINNRVFK; KFGFKAGIF; IVPDSEYAF; VSKKAPSLF; LSLLVFACF; NPLNLFWLI; IFITLSLLVF; RFIFITLSLL; MYKEDKVGVI; MFKRFIFITL; FYANPKLRLV; EVQKNPLNLF; EYAFDLFKSK; PYPIEGKRLL; YYGIIDAFDY; KNPLNLFWLI; EYAFDLFKSKL; RFIFITLSLLV; SYERPDIYYGI; MYKEDKVGVIF; ITLSLLVFACF; AYEVQKNPLNL; IYYGIIDAFDY; GYRFSDLSVKL; |

| | | |
|---|---|---|
| | | QVPKNSLAIKF |
| | HLA-A2902 | SYERPDIYY; YGIIDAFDY; VIYSISSEY; EIIVPDSEY; FGFKAGIFY; LFWLIGYRF; IVPDSEYAF; HVKDFKFGF; IFITLSLLV; YYGIIDAFDY; IFITLSLLVF; KVIYSISSEY; KLITKSLRPY; KFGFKAGIFY; VIGLNQDQSY; IIKDIGKVIY; PLNLFWLIGY; LSYERPDIYY; DAAKELGPKY; IIVPDSEYAF; IAGITGLGVY; YVIGLNQDQSY; RFSDLSVKLSY; IYYGIIDAFDY; VVGVLAHGSFY; KLSYERPDIYY; SIIKDIGKVIY; EIIVPDSEYAF; YDAAKELGPKY; PIAGITGLGVY; FIFITLSLLVF; FKFGFKAGIFY; TVDEAMTEDAY; QVPKNSLAIKF; PMSEHVKDFKF |
| | HLA-A6801 | NLFWLIGYR; EYINNRVFK; MSEHVKDFK; LSVKLSYER; ISSEYINNR; YIAAKMSRK; FLAGYIAAK; SLLVFACFK; YAFDLFKSK; FACFKSNKK; KAMALFMYK; TSIIKDIGK; IFYANPKLR; SEYAFDLFK; LAHGSFYDK; VIYSISSEY; VFACFKSNK; GYIAAKMSR; DAAKELGPK; GIIIDRGLK; EIIVPDSEY; RPYPIEGKR; DYGDIQVPK; ALFMYKEDK; MTEDAYEVQ; QSVHDGVVK; YRFSDLSVK; EAAFLAGYI; MTEDAYEVQK; HVKDFKFGFK; DSEYAFDLFK; LVFACFKSNK; SVHDGVVKLR; SISSEYINNR; GIFYANPKLR; LSLLVFACFK; ITSIIKDIGK; DLSVKLSYER; EYAFDLFKSK; MALFMYKEDK; IAAKMSRKEK; LTGPMSEHVK; GIKLITKSLR; DNFGIKLITK; KVIYSISSEY; SEYINNRVFK; QVPKNSLAIK; LNLFWLIGYR; VFACFKSNKK; LSYERPDIYY; VLAHGSFYDK; FDYGDIQVPK; GYIAAKMSRK; YSISSEYINNR; TLSLLVFACFK; SSEYINNRVFK; LAGYIAAKMSR; YIAAKMSRKEK; LLVFACFKSNK; LVFACFKSNKK; IVKDPDVLNNR; QSVHDGVVKLR; FLTGPMSEHVK; FGIKLITKSLR; RVFKGGIIIDR; AAFLAGYIAAK; YANPKLRLVSK; ITGLGVYDAAK; YVIGLNQDQSY; VITSIIKDIGK; FKAGIFYANPK; LVDEVIDLENK; IGYRFSDLSVK; EHVKDFKFGFK; AMALFMYKEDK; AMTEDAYEVQK; IYYGIIDAFDY; SLRPYPIEGKR; PLNLFWLIGYR; GVLAHGSFYDK; VVKLRDNFGIK; AGIFYANPKLR; VPKNSLAIKFR |
| | HLA-B0702 | APQNVITSI; YPIEGKRLL; FPIAGITGL; RPDIYYGII; GPKYYVIGL; LVSKKAPSL; QVPKNSLAI; RPYPIEGKRL; APQNVITSII; VPKNSLAIKF; YPIEGKRLLT; GPMSEHVKDF; KLRDNFGIKL; RLVSKKAPSL; RPYPIEGKRLL; YPIEGKRLLTV; FPIAGITGLGV; FKRFIFITLSL; NPKLRLVSKKA |
| | HLA-B0801 | FDKEKGKAM; KEKGKAMAL; FKRFIFITL; MFKRFIFITL; FGIKLITKSL; FDKEKGKAMAL; AIKFRNEEAAF; FMYKEDKVGVI; NPKLRLVSKKA; FKRFIFITLSL |
| | HLA-B1501 | VQKNPLNLF; GVLAHGSFY; VIYSISSEY; AAKELGPKY; KFRNEEAAF; KVVVGVLAH; IVPDSEYAF; EIIVPDSEY; HVKDFKFGF; LITKSLRPY; FITLSLLVF; FGFKAGIFY; LSYERPDIY; PMSEHVKDF; YGIIDAFDY; AGITGLGVY; RLLTVDEAM; LSLLVFACF; IGLNQDQSY; KVIYSISSEY; KLITKSLRPY; LSYERPDIYY; KMSRKEKIGF; IIKDIGKVIY; IQVPKNSLAI; AAKELGPKYY; IIVPDSEYAF; IFITLSLLVF; VQKNPLNLFW; VVGVLAHGSF; TLSLLVFACF; IAGITGLGVY; FLAGYIAAKM; KLSYERPDIY; KGKAMALFMY; NLFWLIGYRF; LVSKKAPSLF; WLIGYRFSDL; SSEYINNRVF; YVIGLNQDQSY; ISSEYINNRVF; VVVGVLAHGSF; FIFITLSLLVF; KLSYERPDIYY; AIKFRNEEAAF; RFSDLSVKLSY; SLFDKEKGKAM; VVGVLAHGSFY; LAHGSFYDKGY; EIIVPDSEYAF; FMYKEDKVGVI; RLVSKKAPSLF; VQKNPLNLFWL; SIIKDIGKVIY; PMSEHVKDFKF |
| | HLA-B2705 | YRFSDLSVK; KRFIFITLS; FRNEEAAFL; FKFGFKAGI; KRFIFITLSL; YRFSDLSVKL; KRLLTVDEAM; KRFIFITLSLL; LRLVSKKAPSL; FKFGFKAGIFY; KRLLTVDEAMT |
| | HLA-B3501 | FPIAGITGL; IVPDSEYAF; YPIEGKRLL; LFWLIGYRF; LAGYIAAKM; DPDVLNNRL; IGLNQDQSY; FITLSLLVF; DEAMTEDAY; DSEYAFDLF; EIIVPDSEY; IYYGIIDAF; FGFKAGIFY; NPLNLFWLI; RLLTVDEAM; |

| | | |
|---|---|---|
| | | VIYSISSEY; LSLLVFACF; LSYERPDIY; YGIIDAFDY; KFRNEEAAF; AAKELGPKY; YIAPQNVIT; IIVPDSEYAF; IAGITGLGVY; VPKNSLAIKF; DIYYGIIDAF; FLAGYIAAKM; DAAKELGPKY; IFITLSLLVF; DAYEVQKNPL; YPIEGKRLLT; YAFDLFKSKL; KVIYSISSEY; NLFWLIGYRF; FSDLSVKLSY; TLSLLVFACF; FPIAGITGLG; VDEAMTEDAY; VPDSEYAFDL; IKFRNEEAAF; EKIGFLTGPM; EAAFLAGYIA; LSYERPDIYY; EAMTEDAYEV; NEEAAFLAGY; NPLNLFWLIGY; TVDEAMTEDAY; YVIGLNQDQSY; VPDSEYAFDLF; LAHGSFYDKGY; EIIVPDSEYAF; FIFITLSLLVF; YPIEGKRLLTV; FPIAGITGLGV; EAAFLAGYIAA; DAAKELGPKYY; ISSEYINNRVF; FKFGFKAGIFY; VVVGVLAHGSF; YEVQKNPLNLF; VVGVLAHGSFY; RFSDLSVKLSY; ITLSLLVFACF |
| | HLA-B4403 | SEHVKDFKF; KEDKVGVIF; SEYINNRVF; EEAAFLAGY; SEYAFDLFK; GEIIVPDSEY; YEVQKNPLNL; EEAAFLAGYI; NEEAAFLAGY; SEYAFDLFKS; KEKGKAMALF; SEHVKDFKFGF; GEIIVPDSEYA; KEKGKAMALFM; KEDKVGVIFPI; YEVQKNPLNLF; KEKIGFLTGPM; EEAAFLAGYIA |
| | HLA-B5101 | NPLNLFWLI; FPIAGITGL; YPIEGKRLL; APQNVITSI; APQNVITSII; VPKNSLAIKF; IAPQNVITSI; FPIAGITGLG; YPIEGKRLLT; YPIEGKRLLTV; FPIAGITGLGV; IAPQNVITSII |
| | HLA-B5701 | EVQKNPLNLFW |
| NP_212519.1 B; Basic membrane protein D (bmpD) [Borrelia burgdorferi B31; Seq4 | HLA-A0101 | ASENVSVNY; VLESFMYGY; NSNIKVVSQY; RSEEVAFLAGY |
| | HLA-A0201 | FMYGYEAGA; YLAPNNVIV; SIMNGIIKV; LIWGIGFRL; KVYYFLIFL; FLIFLFIVA; KVDSLMYSL; YFLIFLFIV; MLKKVYYFL; FAAAGLSGI; FMLKKVYYF; FLAGYFASK; FLGLKEDGL; YYFLIFLFI; EIYFLYFFI; NLLNISFRS; GLKEDGLGL; NISFRSEEV; FLFIVACSS; KVSYFVLQM; SLTKKYLET; FMLKKVYYFL; FLAGYFASKA; RLSDILFQRA; SVNYAIIEGV; VLDGGKTMFL; MLKKVYYFLI; NLIWGIGFRL; ILFQRASENV; AIIEGVYDEI; GLSGIGVIEA; LMYSLTKKYL; YYFLIFLFIV; YVGTFGDFGL; KLKADLNINI; YEIYFLYFFI; YLAPNNVIVS; FLIFLFIVAC; NMYRDGVDII; KIGQSIMNGI; FLFIVACSSS; FQRASENVSV; GIIKVPYDKV; GLKEDGLGLV; GLGLVLNENL; KKVYYFLIFL; FMLKKVYYFLI; YLAPNNVIVSA; KVYYFLIFLFI; FAAAGLSGIGV; SLTKKYLETGV; SLMYSLTKKYL; LLNISFRSEEV; FLGLKEDGLGL; VLESFMYGYEA; KVPYDKVSYFV; GLSGIGVIEAA; FLIFLFIVACS; GVLDGGKTMFL; FIKSKEDIFML; IIFAAAGLSGI; FLAGYFASKAS; TMFLGLKEDGL; STASNMYRDGV; ELGPDHYIIGV; VYYFLIFLFIV; RLSDILFQRAS; KLKADLNINII; KTGKIGFVGGV; VSVNYAIIEGV; YAIIEGVYDEI |
| | HLA-A0301 | FLAGYFASK; FLYFFIKSK; SLMYSLTKK; RSTASNMYR; RLSDILFQR; IYFLYFFIK; LMYSLTKKY; KGFNESSSK; SSKAIRKLK; KIGFVGGVR; GIIKVPYDK; GYFASKASK; ESSSKAIRK; NVIVSAVKK; KVYYFLIFL; ASKASKTGK; NLIWGIGFR; GLVLNENLK; QSIMNGIIK; FMYGYEAGAK; GVYDEIQIPK; AFLAGYFASK; EIYFLYFFIK; AGYFASKASK; IMNGIIKVPY; SLMYSLTKKY; KSKEDIFMLK; YFLYFFIKSK; KSNYSEIYNK; KVLESFMYGY; SSSKAIRKLK; GQSIMNGIIK; LIVDGAFDDK; DSLMYSLTKK; KVYYFLIFLF; VDSLMYSLTK; ADLNINIIEK; YSEIYNKSLK; GTFGDFGLGR; FASKASKTGK; GAKYANSNIK; DIFMLKKVYY; SSDDGKSEAK; GGKTMFLGLK; RASENVSVNY; AVKKVDSLMY; VYYFLIFLFI; KVDSLMYSLTK; KIGFVGGVRGK; VAFLAGYFASK; KSKEDIFMLKK; GLGLVLNENLK; IYFLYFFIKSK; FMYGYEAGAKY; IVACSSSDDGK; KVYYFLIFLFI; SIMNGIIKVPY; LAGYFASKASK; ESSSKAIRKLK; NSNLIWGIGFR; SVNYAIIEGV; GLGRSTASNMY; YFASKASKTGK |
| | HLA-A1101 | SLMYSLTKK; RSTASNMYR; GIIKVPYDK; SSKAIRKLK; QSIMNGIIK; |

|  |  |  |
|---|---|---|
|  |  | IYFLYFFIK; NVIVSAVKK; RLSDILFQR; SNYSEIYNK; ASKASKTGK; DSLMYSLTK; FLAGYFASK; FLYFFIKSK; ESSSKAIRK; GYFASKASK; IVDGAFDDK; SKEDIFMLK; KVYYFLIFL; GIGVIEAAK; GLVLNENLK; KGFNESSSK; ASENVSVNY; NLIWGIGFR; VLESFMYGY; VYDEIQIPK; KIGFVGGVR; KTMFLGLKE; DLNINIIEK; KEDIFMLKK; AKYANSNIK; SEIYNKSLK; GKTMFLGLK; GVYDEIQIPK; KSNYSEIYNK; EIYFLYFFIK; KSKEDIFMLK; KVLESFMYGY; SSSKAIRKLK; GTFGDFGLGR; AFLAGYFASK; FMYGYEAGAK; SSDDGKSEAK; VACSSSDDGK; GQSIMNGIIK; DSLMYSLTKK; KVYYFLIFLF; LIVDGAFDDK; SLMYSLTKKY; AGYFASKASK; YSEIYNKSLK; YFLYFFIKSK; AVKKVDSLMY; GAKYANSNIK; RASENVSVNY; SGIGVIEAAK; SKEDIFMLKK; GGKTMFLGLK; IMNGIIKVPY; FASKASKTGK; KVDSLMYSLTK; VAFLAGYFASK; KSKEDIFMLKK; SIMNGIIKVPY; SSSDDGKSEAK; IVACSSSDDGK; SLIVDGAFDDK; KADLNINIIEK; KIGFVGGVRGK; SFMYGYEAGAK; IYFLYFFIKSK; GLGLVLNENLK; SVNYAIIEGVY; KVYYFLIFLFI; LAGYFASKASK; NSNLIWGIGFR; ESSSKAIRKLK; LSGIGVIEAAK; APNNVIVSAVK; YEIYFLYFFIK; RSEEVAFLAGY; LVLNENLKSNY |
| | HLA-A2402 | MDNYYEIYF; DNYYEIYFL; VYYFLIFLF; YYFLIFLFI; YYEIYFLYF; NYYEIYFLY; QYVGTFGDF; IYFLYFFIK; PYDKVSYFV; SYLAPNNVI; SYLGDIANL; KYANSNIKV; FMLKKVYYF; MYRDGVDII; YFLIFLFIV; EIYFLYFFI; YEIYFLYFF; VYYFLIFLFI; YYEIYFLYFF; NYYEIYFLYF; PYDKVSYFVL; IFMLKKVYYF; YYFLIFLFIV; MYRDGVDIIF; KVYYFLIFLF; NYSEIYNKSL; KYANSNIKVV; FFIKSKEDIF; IFAAAGLSGI; SYLAPNNVIV; MLKKVYYFLI; YEIYFLYFFI; KVVSQYVGTF; NYYEIYFLYFF; YYEIYFLYFFI; VYYFLIFLFIV; IFMLKKVYYFL; FMLKKVYYFLI; QYVGTFGDFGL; LYFFIKSKEDI; VYDEIQIPKNL; YYFLIFLFIVA; NYSEIYNKSLK; IWGIGFRLSDI; YFFIKSKEDIF; KKVYYFLIFLF; IYFLYFFIKSK |
| | HLA-A2902 | NYYEIYFLY; VYYFLIFLF; LMYSLTKKY; NYAIIEGVY; IFMLKKVYY; KVPYDKVSY; VLESFMYGY; YYEIYFLYF; YGYEAGAKY; DIFMLKKVY; EVAFLAGYF; YYFLIFLFI; FMLKKVYYF; EIYFLYFFI; MNGIIKVPY; SFRSEEVAF; YEIYFLYFF; ASENVSVNY; YFLIFLFIV; MYGYEAGAKY; IMNGIIKVPY; DIFMLKKVYY; NYYEIYFLYF; DNYYEIYFLY; KVYYFLIFLF; SLMYSLTKKY; YYEIYFLYFF; KVLESFMYGY; YYFLIFLFIV; IIGVDQDQSY; AVKKVDSLMY; VLNENLKSNY; VNYAIIEGVY; VYYFLIFLFI; IFMLKKVYYF; RGKVLESFMY; RASENVSVNY; YIIGVDQDQSY; FMYGYEAGAKY; SIMNGIIKVPY; NYYEIYFLYFF; SVNYAIIEGVY; LVLNENLKSNY; MDNYYEIYFLY; IIKVPYDKVSY; VYYFLIFLFIV; YFFIKSKEDIF; QRASENVSVNY; NMYRDGVDIIF; YYEIYFLYFFI; DIFMLKKVYYF; SAVKKVDSLMY; VRGKVLESFMY; EDIFMLKKVYY |
| | HLA-A6801 | NLIWGIGFR; NVIVSAVKK; RSTASNMYR; RLSDILFQR; FLAGYFASK; ESSSKAIRK; NYYEIYFLY; FLYFFIKSK; IYFLYFFIK; SSKAIRKLK; DSLMYSLTK; ETGVLDGGK; SLMYSLTKK; DLNINIIEK; EVAFLAGYF; NNVIVSAVK; EIYFLYFFI; SNYSEIYNK; QSIMNGIIK; SEIYNKSLK; GIIKVPYDK; MYGYEAGAK; GYFASKASK; NESSSKAIR; GLVLNENLK; TFGDFGLGR; IVDGAFDDK; SKEDIFMLK; EIYFLYFFIK; GTFGDFGLGR; FMYGYEAGAK; YSEIYNKSLK; FASKASKTGK; SSSKAIRKLK; DSLMYSLTKK; DNYYEIYFLY; GVYDEIQIPK; LIVDGAFDDK; NNVIVSAVKK; DIFMLKKVYY; KSNYSEIYNK; VACSSSDDGK; EVAFLAGYFA; YFLYFFIKSK; NSNIKVVSQY; SGIGVIEAAK; KSKEDIFMLK; GAKYANSNIK; FNESSSKAIR; FRLSDILFQR; SNLIWGIGFR; KVYYFLIFLF; NSNLIWGIGFR; ESSSKAIRKLK; IVACSSSDDGK; NYSEIYNKSLK; VAFLAGYFASK; YEIYFLYFFIK; LAGYFASKASK; IPKNLLNISFR; IYFLYFFIKSK; YFASKASKTGK; |

| | | |
|---|---|---|
| | | MDNYYEIYFLY; EGVYDEIQIPK; SVNYAIIEGVY; NYYEIYFLYFF; SFMYGYEAGAK; FMYGYEAGAKY; EAAKELGPDHY; SLIVDGAFDDK; EVAFLAGYFAS; LGRSTASNMYR; YIIGVDQDQSY; SSSDDGKSEAK; EIYFLYFFIKS; NSYLGDIANLE; MNGIIKVPYDK; SIMNGIIKVPY; LSGIGVIEAAK |
| | HLA-B0702 | APNNVIVSA; VPYDKVSYF; SAVKKVDSL; AIRKLKADL; KASTGNSYL; KVSYFVLQM; APNNVIVSAV; IPKNLLNISF; VPYDKVSYFV; FVGGVRGKVL; GVRGKVLESF; VPYDKVSYFVL; IVSAVKKVDSL |
| | HLA-B0801 | YDKVSYFVL; MLKKVYYFL; FMLKKVYYF; FMLKKVYYFL; MLKKVYYFLI; IPKNLLNISF; FIKSKEDIFM; MLKKVYYFLIF; YDKVSYFVLQM; FIKSKEDIFML; FMLKKVYYFLI |
| | HLA-B1501 | LMYSLTKKY; YGYEAGAKY; FMLKKVYYF; VVSQYVGTF; FQRASENVS; VSLIVDGAF; IGVDQDQSY; GLKEDGLGL; GVLDGGKTM; ASKTGKIGF; SLKIGQSIM; KVPYDKVSY; SFRSEEVAF; GVIEAAKEL; IMNGIIKVPY; SQYVGTFGDF; FQRASENVSV; ISFRSEEVAF; IEKASTGNSY; RASENVSVNY; GVRGKVLESF; AVKKVDSLMY; SLMYSLTKKY; VLNENLKSNY; TVSLIVDGAF; KVLESFMYGY; KVYYFLIFLF; GVLDGGKTMF; VNYAIIEGVY; KVVSQYVGTF; LGRSTASNMY; RGKVLESFMY; AAKELGPDHY; IQIPKNLLNI; NLKSNYSEIY; KASKTGKIGF; IIGVDQDQSY; LMYSLTKKYL; IGFRLSDILF; FMYGYEAGAKY; YIIGVDQDQSY; SIMNGIIKVPY; NMYRDGVDIIF; SVNYAIIEGVY; IIKVPYDKVSY; MLKKVYYFLIF; GLGRSTASNMY; GLKEDGLGLVL; KTVSLIVDGAF; RSEEVAFLAGY; IIEKASTGNSY; LVLNENLKSNY; FQRASENVSVN; VSQYVGTFGDF; YFFIKSKEDIF |
| | HLA-B2705 | YRDGVDIIF; FRSEEVAFL; GRSTASNMY; QRASENVSV; FRLSDILFQR; GRSTASNMYR; KKVDSLMYSL; QRASENVSVNY |
| | HLA-B3501 | YGYEAGAKY; IGVDQDQSY; VPYDKVSYF; VVSQYVGTF; EVAFLAGYF; LKSNYSEIY; VSLIVDGAF; EKASTGNSY; YRDGVDIIF; MDNYYEIYF; YYEIYFLYF; SFRSEEVAF; NYAIIEGVY; YEIYFLYFF; MNGIIKVPY; EEVAFLAGY; LIWGIGFRL; IPKNLLNISF; TVSLIVDGAF; RASENVSVNY; IMNGIIKVPY; KVVSQYVGTF; ISFRSEEVAF; MYRDGVDIIF; MYGYEAGAKY; YVGTFGDFGL; FFIKSKEDIF; KVLESFMYGY; IKVPYDKVSY; YANSNIKVVS; TGVLDGGKTM; IIGVDQDQSY; YYEIYFLYFF; SAVKKVDSLM; APNNVIVSAV; YIIGVDQDQSY; SVNYAIIEGVY; FMYGYEAGAKY; EAAKELGPDHY; SAVKKVDSLMY; SIMNGIIKVPY; VPYDKVSYFVL; NISFRSEEVAF; MDNYYEIYFLY; NMYRDGVDIIF; YFFIKSKEDIF; LVLNENLKSNY; IIEKASTGNSY; KTVSLIVDGAF; IVDGAFDDKGF; YAIIEGVYDEI; FFIKSKEDIFM; TGVLDGGKTMF; DSLMYSLTKKY |
| | HLA-B4403 | EEVAFLAGY; YEIYFLYFF; DEIQIPKNL; KEDGLGLVL; KELGPDHYI; EEVAFLAGYF; SEIYNKSLKI; YEIYFLYFFI; SEEVAFLAGY; SENVSVNYAI; DEIQIPKNLL; SEAKTVSLIV; SEEVAFLAGYF; EEVAFLAGYFA; DEIQIPKNLLN; SENVSVNYAII; KEDIFMLKKVY |
| | HLA-B5101 | VPYDKVSYF; FAAAGLSGI; IPKNLLNIS; VPYDKVSYFV; IPKNLLNISF; VPYDKVSYFVL; LAPNNVIVSAV; FAAAGLSGIGV |
| | HLA-B5701 | ISFRSEEVAF |
| AAC70056.1; Decorin binding protein A; DbpA [Borrelia afzelii; Seq5 | HLA-A0101 | ATEEETITF; FTETQTGGKV; |
| | HLA-A0201 | GIYDLILNA; TLLASLLAA; IILTLTLLA; LTLTLLASL; TTADGIIAI; SLLAACSLT; KIILTLTLL; TADGIIAIV; GMQGMKQAV; VMKAKVENI; LLASLLAAC; GMKQAVEEA; FLEATEEET; GIYDLILNAA; TTADGIIAIV; ILTLTLLASL; FLEATEEETI; RVADLTIKFL; KIILTLTLLA; KVMKAKVENI; LTLTLLASLL; KTTADGIIAI; AIVKVMKAKV; LTLLASLLAA; LLASLLAACS; IILTLTLLASL; LLASLLAACSL; LLAACSLTGKA; ILNAAKAVEKI; KTTADGIIAIV; LTLTLLASLLA; TADGIIAIVKV; ILTLTLLASLL; TLTLLASLLAA; GIIAIVKVMKA |

| | | |
|---|---|---|
| | HLA-A0301 | IIAIVKVMK; LAACSLTGK; KARLESSVK; AIVKVMKAK; FTETQTGGK; LLAACSLTGK; ILNAAKAVEK; GIIAIVKVMK; KIRVADLTIK; ATEEETITFK; IAIVKVMKAK; TADGIIAIVK; IYDLILNAAK; VMKAKVENIK; ITNEIEKAIK; AFTETQTGGK; GIYDLILNAAK; SLLAACSLTGK; KVGGSQIRAAK; IIAIVKVMKAK; KVMKAKVENIK; TTADGIIAIVK; LILNAAKAVEK; AVEKIGMQGMK; KVENIKEKQTK; SVKDITNEIEK |
| | HLA-A1101 | IIAIVKVMK; AIVKVMKAK; LAACSLTGK; AVEEAAKEK; KQAVEEAAK; FTETQTGGK; KARLESSVK; ATEEETITFK; GIIAIVKVMK; ILNAAKAVEK; TADGIIAIVK; LLAACSLTGK; ITNEIEKAIK; IAIVKVMKAK; VMKAKVENIK; AACSLTGKAR; KIRVADLTIK; KAKVENIKEK; AFTETQTGGK; TTADGIIAIVK; GIYDLILNAAK; SLLAACSLTGK; SVKDITNEIEK; KVMKAKVENIK; KVGGSQIRAAK; AVEKIGMQGMK; IIAIVKVMKAK; LILNAAKAVEK; AVEEAAKEKPK; KVENIKEKQTK; AIKEAEDAGVK; KQAVEEAAKEK; GMKQAVEEAAK; EATEEETITFK; RVADLTIKFLE; DAFTETQTGGK |
| | HLA-A2402 | KYNKIILTL; DFSGIYDLI; KYNKIILTLT; IYDLILNAAK; KYNKIILTLTL; KFLEATEEETI |
| | HLA-A2902 | RVADLTIKF; GAGEEDFSGIY |
| | HLA-A6801 | IIAIVKVMK; FTETQTGGK; LAACSLTGK; EKIGMQGMK; MKAKVENIK; LNAAKAVEK; AIVKVMKAK; ENIKEKQTK; ITNEIEKAIK; TADGIIAIVK; GIIAIVKVMK; QAVEEAAKEK; ILNAAKAVEK; IAIVKVMKAK; LLAACSLTGK; ATEEETITFK; AACSLTGKAR; TTADGIIAIVK; EATEEETITFK; DAFTETQTGGK; SVKDITNEIEK; IIAIVKVMKAK; LAACSLTGKAR; DITNEIEKAIK; GIYDLILNAAK; NIKEKQTKNQK; DGIIAIVKVMK; LILNAAKAVEK; KVMKAKVENIK; MKAKVENIKEK; SLLAACSLTGK; AVEKIGMQGMK; KVENIKEKQTK; KVGGSQIRAAK |
| | HLA-B0702 | RVADLTIKF; KPKTTADGI; MIKYNKIIL; RAAKIRVADL; KPKTTADGII; RVADLTIKFL; LASLLAACSL; KPKTTADGIIA; MIKYNKIILTL |
| | HLA-B0801 | MIKYNKIIL; YNKIILTLTL; RAAKIRVADL; MIKYNKIILTL |
| | HLA-B1501 | RVADLTIKF; GIIAIVKVM; ATEEETITF; RVADLTIKFL; KIRVADLTIKF; SQIRAAKIRVA |
| | HLA-B2705 | KQAVEEAAK; IRVADLTIKF; IRAAKIRVADL |
| | HLA-B3501 | DAGVKTDAF; RVADLTIKF; ATEEETITF; FSGIYDLIL; EATEEETITF; LASLLAACSL; EDAGVKTDAF; NAAKAVEKIGM; LEATEEETITF; GAGEEDFSGIY; KAVEKIGMQGM |
| | HLA-B4403 | GEEDFSGIY; EEDFSGIYD; NEIEKAIKEA; EEDFSGIYDL; KENGAGEEDF; EEDFSGIYDLI; AEDAGVKTDAF |
| | HLA-B5101 | KPKTTADGI; KPKTTADGII; LASLLAACSL; |
| | HLA-B5701 | ATEEETITF; ; |
| AAC70057.1; Decorin binding protein A; DbpA [Borrelia garinii]; Seq6 | HLA-A0101 | SSGAFSAMY; FSAMYDLMI; GSSGAFSAMY; |
| | HLA-A0201 | AMYDLMIDV; ILLKLSLIV; LMIDVSKPL; KLSLIVSLL; KISEKPEFI; SLIVSLLVA; AMEEKLNNV; TTADGIIAI; FILKAKIKA; KILLKLSLI; LSLIVSLLV; FSAMYDLMI; GAFSAMYDL; KLSLIVSLLV; SAMYDLMIDV; KILLKLSLIV; FILKAKIKAI; KMTGTVTKEA; KISEKPEFIL; LLKLSLIVSL; GLTGETKIRL; TTADGIIAIA; AMYDLMIDVS; AIAQAMEEKL; ILLKLSLIVSL; KLSLIVSLLVA; FSAMYDLMIDV; MIDVSKPLEEI; LIVSLLVACGL; LKLSLIVSLLV; GAFSAMYDLMI; LLVACGLTGET; ILKAKIKAIQV; LLKLSLIVSLL |
| | HLA-A0301 | FTNTQTGSK; QTGSKISEK; IKYNKILLK; IQVAERFVK; MIKYNKILLK; IQKMTGTVTK; AIQVAERFVK; AFTNTQTGSK; VACGLTGETK; TQTGSKISEK; GSSGAFSAMY; IAIAQAMEEK; ISEKPEFILK; KPLEEIGIQK; KANAKKEGVK; RLESSAQEIK; MYDLMIDVSK; KIKAIQVAER; AMYDLMIDVSK; KISEKPEFILK; GIQKMTGTVTK; QVAERFVKAIK; MTGTVTKEAEK; NVNKKQHDALK; LVACGLTGETK; KAIQVAERFVK; IIAIAQAMEEK; EINKIKANAKK; EAFTNTQTGSK; |

|  |  |  |
|---|---|---|
|  |  | AMEEKLNNVNK; AIKEEAEKLKK |
|  | HLA-A1101 | AIAQAMEEK; FTNTQTGSK; IQVAERFVK; QTGSKISEK; SSGAFSAMY; GTVTKEAEK; KAIKEEAEK; IKYNKILLK; SEKPEFILK; MIKYNKILLK; IAIAQAMEEK; AIQVAERFVK; ISEKPEFILK; TQTGSKISEK; VACGLTGETK; KANAKKEGVK; IQKMTGTVTK; VAERFVKAIK; AIKEEAEKLK; GSSGAFSAMY; RLESSAQEIK; AFTNTQTGSK; AQEIKDEINK; KIKAIQVAER; EINKIKANAK; MYDLMIDVSK; AMYDLMIDVSK; KISEKPEFILK; KAIQVAERFVK; SAQEIKDEINK; GIQKMTGTVTK; MTGTVTKEAEK; QVAERFVKAIK; NVNKKQHDALK; AMEEKLNNVNK; NTQTGSKISEK; IIAIAQAMEEK; LVACGLTGETK; KAIKEEAEKLK; AIKEEAEKLKK; EAFTNTQTGSK; FVKAIKEEAEK; EINKIKANAKK |
|  | HLA-A2402 | KYNKILLKL; AFSAMYDLMI; KYNKILLKLSL |
|  | HLA-A2902 | SSGAFSAMY; ; SGSSGAFSAMY |
|  | HLA-A6801 | FTNTQTGSK; QTGSKISEK; AIAQAMEEK; DALKNLEEK; GTVTKEAEK; IKAIQVAER; IQVAERFVK; EFILKAKIK; KAIKEEAEK; SSGAFSAMY; IAIAQAMEEK; EINKIKANAK; MIKYNKILLK; KIKAIQVAER; EIKDEINKIK; TQTGSKISEK; MYDLMIDVSK; VACGLTGETK; ISEKPEFILK; TGTVTKEAEK; VAERFVKAIK; EAFTNTQTGSK; MTGTVTKEAEK; EINKIKANAKK; FVKAIKEEAEK; IIAIAQAMEEK; NVNKKQHDALK; QVAERFVKAIK; NTQTGSKISEK; KAIQVAERFVK; LVACGLTGETK; SAQEIKDEINK; AMYDLMIDVSK; TTADGIIAIAQ |
|  | HLA-B0702 | KPEFILKAKI; KPLEEIGIQKM; PPTTADGIIAI |
|  | HLA-B0801 | ILKAKIKAI; LLKLSLIVSL; FILKAKIKAI; YNKILLKLSL; KLKKSGSSGAF; LLKLSLIVSLL |
|  | HLA-B1501 | LMIDVSKPL; SSGAFSAMY; GIIAIAQAM; KQHDALKNL; IQKMTGTVT; KKSGSSGAF; ILKAKIKAI; KAIQVAERF; GSSGAFSAMY; LLKLSLIVSL; LKKSGSSGAF; GSKISEKPEF; KLKKSGSSGAF; IQVAERFVKAI; KIKAIQVAERF; SGSSGAFSAMY |
|  | HLA-B2705 | IRLESSAQEIK |
|  | HLA-B3501 | KAIQVAERF; GSSGAFSAM; SSGAFSAMY; KANTAATTT; DGIIAIAQAM; SGSSGAFSAM; GSSGAFSAMY; GAFSAMYDLM; KPLEEIGIQKM; SGSSGAFSAMY; TPPTTADGIIA |
|  | HLA-B4403 | EEIGIQKMT; EEIGIQKMTG; SEKPEFILKA; QEIKDEINKI; EEIGIQKMTGT |
|  | HLA-B5101 | TPPTTADGI; PPTTADGII; TPPTTADGII; KPEFILKAKI; PPTTADGIIA; PPTTADGIIAI; TPPTTADGIIA |
|  | HLA-B5701 |  |
| AAC70021.1; Decorin binding protein B; DbpB [Borrelia burgdorferi]; Seq7 | HLA-A0101 |  |
|  | HLA-A0201 | AMFDLMLEV; VLFEAFTGL; LMLEVVESL; FLAMFDLML; LLVACSIGL; IVIALFFKL; ALFFKLLVA; LVACSIGLV; GLVERTNAA; RLLAAKAQI; FLKIIEEEA; GLALREAKV; AQIENQLKV; GIIGLKARV; IALFFKLLV; GQFLAMFDL; AMFDLMLEVV; LAMFDLMLEV; KLLVACSIGL; SLEDVGIIGL; LLVACSIGLV; GLVERTNAAL; SIVIALFFKL; KIIEEEALKL; VIALFFKLLV; FLKIIEEEAL; GVLFEAFTGL; IVIALFFKLL; DLMLEVVESL; ALREAKVQAI; ATGKGVLFEA; FLAMFDLMLEV; KLLVACSIGLV; IVIALFFKLLV; VLFEAFTGLKT; LAMFDLMLEVV; MLEVVESLEDV; ALREAKVQAIV; GQFLAMFDLML; SIVIALFFKLL; AIVETGKFLKI |
|  | HLA-A0301 | FTGLKTGSK; KIKKEATGK; SIVIALFFK; IVETGKFLK; KIIEEEALK; VTSGGLALR; LFEAFTGLK; VLFEAFTGLK; KVQAIVETGK; AFTGLKTGSK; AIVETGKFLK; KVTSGGLALR; NTAERLLAAK; ALESSSKDLK; KQNIENGGEK; TNAALESSSK; QIENQLKVVK; DLKNKILKIK; IIEEEALKLK; RTNAALESSSK; GVLFEAFTGLK; GLKARVLEESK; ILKIKKEATGK; SLEDVGIIGLK; KIIEEEALKLK; FLKIIEEEALK; TSGGLALREAK; KQNIENGGEKK; SSKDLKNKILK; |

| | | |
|---|---|---|
| | | LVACSIGLVER; EAFTGLKTGSK; AALESSSKDLK |
| | HLA-A1101 | SIVIALFFK; IVETGKFLK; KIIEEEALK; SSSKDLKNK; TAERLLAAK; VTSGGLALR; KAQIENQLK; FTGLKTGSK; NAALESSSK; VQAIVETGK; LFEAFTGLK; KIKKEATGK; IVIALFFKL; NQLKVVKEK; KARVLEESK; VLFEAFTGLK; KVQAIVETGK; AIVETGKFLK; NTAERLLAAK; KVTSGGLALR; KQNIENGGEK; ALESSSKDLK; VACSIGLVER; IIEEEALKLK; TNAALESSSK; AFTGLKTGSK; ESSSKDLKNK; QIENQLKVVK; RTNAALESSSK; GVLFEAFTGLK; AALESSSKDLK; SSKDLKNKILK; TSGGLALREAK; SLEDVGIIGLK; QAIVETGKFLK; KIIEEEALKLK; AQIENQLKVVK; AAKAQIENQLK; GLKARVLEESK; LVACSIGLVER; KQNIENGGEKK; EAFTGLKTGSK; IVIALFFKLLV |
| | HLA-A2402 | QFLAMFDLML; FFKLLVACSI; LFFKLLVACSI; KFLKIIEEEAL |
| | HLA-A2902 | IVIALFFKL; |
| | HLA-A6801 | SIVIALFFK; NAALESSSK; VTSGGLALR; IVETGKFLK; EDVGIIGLK; NNPINTAER; FTGLKTGSK; LFEAFTGLK; TAERLLAAK; KIIEEEALK; NIENGGEKK; NTAERLLAAK; VACSIGLVER; VLFEAFTGLK; KVTSGGLALR; ESSSKDLKNK; TNAALESSSK; DVGIIGLKAR; KVQAIVETGK; AIVETGKFLK; KNNPINTAER; LVACSIGLVER; EAFTGLKTGSK; QAIVETGKFLK; RTNAALESSSK; GVLFEAFTGLK; FLKIIEEEALK; TSGGLALREAK; SSKDLKNKILK; EDVGIIGLKAR; SLEDVGIIGLK; SKNNPINTAER; AALESSSKDLK; SKVTSGGLALR |
| | HLA-B0702 | KVTSGGLAL; NPINTAERL; LVERTNAAL; NPINTAERLL; ALREAKVQAI; SKVTSGGLAL; |
| | HLA-B0801 | ILKIKKEAT; LVERTNAAL; FLKIIEEEAL; FFKLLVACSI; |
| | HLA-B1501 | LMLEVVESL; KVTSGGLAL; QAIVETGKF; AMFDLMLEV; FLAMFDLML; GQFLAMFDL; LLVACSIGL; VLFEAFTGL; AQIENQLKV; VQAIVETGKF; GLVERTNAAL; AQIENQLKVV; GQFLAMFDLM; KLKETGNSGQF; KVQAIVETGKF; GQFLAMFDLML; VQAIVETGKFL; GSKVTSGGLAL |
| | HLA-B2705 | GQFLAMFDL; ; GQFLAMFDLML |
| | HLA-B3501 | NPINTAERL; QFLAMFDLM; LVERTNAAL; EATGKGVLF; QAIVETGKF; FLAMFDLML; NSGQFLAMF; NPINTAERLL; TGNSGQFLAM; ETGNSGQFLAM; LAAKAQIENQL; TGNSGQFLAMF |
| | HLA-B4403 | KETGNSGQF; KEATGKGVLF; EESKNNPINT; AERLLAAKAQI |
| | HLA-B5101 | LAMFDLMLEV; LAMFDLMLEVV |
| | HLA-B5701 | |
| NP_212694.1; Heat shock protein 90 [Borrelia burgdorferi B31]; Seq8 | HLA-A0101 | DTEVNDLLY; NLEYTNLFY; KSEIKAEEY; YLNKEGLEY; KAEEYNEFY; WSSDGKTGY; ITDSEGSLL; IIDSNDPTY; HTKAEGNLEY; YSNHINYPIY; DSEGSLLPNY; ASDAIDKLKF; SVDGFVSFKEY; QFDTEVNDLLY |
| | HLA-A0201 | LLYLIIHSL; FINIINEFI; IMLSMGQEV; FLRELISNA; FIIFRLCYI; IMDDELDEA; FIVSEKVEV; LLPNYLRFI; KILSELEKL; KLFINRIFI; KLEKISILL; QMQKIMLSM; ELDEAILNL; YLIIHSLYS; LLFEEAMLT; IALFIIFRL; KALESDAYI; NLIPEYEGL; TTFDYENPL; ILLFEEAML; RLCYIIKKV; NILKENPIV; LEYTNLFYV; ILKDHIKEV; IIFRLCYII; ILIFYFKQI; IIDCQDLPL; YSNHINYPI; YQMQKIMLS; TLIKEPSAI; FITDSEGSL; KLYLNKEGL; RMNESQKSI; SAFIVSEKV; ASLIGQFGV; NLFYVPSKA; SMGQEVKEI; GVGFYSAFI; EVNDLLYLI; YLNKEGLEYA; SLLPNYLRFI; IMDDELDEAI; SLYSHKEIFL; YQMQKIMLSM; NLEYTNLFYV; FIIFRLCYII; SLIRFKSSSV; LIMDDELDEA; KIMLSMGQEV; GIIDCQDLPL; ILSKIKSSSV; QIALFIIFRL; FITDSEGSLL; KLNETTALWT; ALWTKNKSEI; ELDEAILNLI; KLFINRIFIT; LLFEEAMLTS; LIFYFKQIAL; KISILLFEEA; YSAFIVSEKV; GQFGVGFYSA; YIIKKVKIKL; MILIFYFKQI; AIDKLKFLSL; GVGFYSAFIV; TLIKEPSAII; DLLYLIIHSL; ILIKDNGIGM; FYFKQIALFI; TTFDYENPLM; YANKWKIQEI; SILLFEEAML; YVPSKAPYDL; KLISLIRFKS; FRLCYIIKKV; ILIFYFKQIA; LIPEYEGLKL; SASLIGQFGV; SLTNEKFKNI; ILLFEEAMLT; |

| | | |
|---|---|---|
| | | KQFDTEVNDL; KQIALFIIFRL; IMDDELDEAIL; ALFIIFRLCYI; KILSKIKSSSV; LIMDDELDEAI; ILIFYFKQIAL; GMDEQDLTNHL; KQFDTEVNDLL; IIDCQDLPLNV; ILNLIPEYEGL; ILIMDDELDEA; MLSMGQEVKEI; IINEFIEKDFL; NLIPEYEGLKL; LLYLIIHSLYS; YSNHINYPIYI; TLIKEPSAIII; GNLEYTNLFYV; ILQQNKILSKI; KSASLIGQFGV; FYSAFIVSEKV; SAFIVSEKVEV; MILIFYFKQIA; FYFKQIALFII; ILKDHIKEVNL; RLCYIIKKVKI; GMPSKNPGKFI |
| | HLA-A0301 | KLISLIRFK; IIHSLYSHK; MLTSGMPSK; KIKSSSVKK; SIYYITGGK; HINYPIYIK; KLSKKNPEK; GLKLKAINK; KLKRKSCMK; GVIAKSGTK; SVDGFVSFK; TTALWTKNK; YSAFIVSEK; LQQNKILSK; KVKIKLKRK; MLSMGQEVK; KFLSLTNEK; GMPSKNPGK; QIALFIIFR; ILELNPNNK; LTKVKEILK; IFRLCYIIK; YLNKEGLEY; GVYSDFENR; KIQEIIKKY; NIINEFIEK; LSLTNEKFK; YSHKEIFLR; GLEYANKWK; GSLLPNYLR; KSIYYITGGK; RLCYIIKKVK; KLKRKSCMKK; AMLTSGMPSK; LIIHSLYSHK; ILQQNKILSK; SSVDGFVSFK; ILSELEKLSK; LLYLIIHSLY; IIFRLCYIIK; ITGGKENILK; LTNHLGVIAK; IMLSMGQEVK; TLTKVKEILK; FYSAFIVSEK; YTNLFYVPSK; ALFIIFRLCY; HINYPIYIKY; LLPNYLRFIK; LSKIKSSSVK; YIKYSEPIMK; IIKKVKIKLK; KENPIVAAYK; KFKNIALEPK; FLSLTNEKFK; GTKEFINNLK; IFRLCYIIKK; GTEIKLYLNK; KQIALFIIFR; LYLNKEGLEY; LIGQFGVGFY; EVNLSATLIK; NLIPEYEGLK; IKLKRKSCMK; LFYVPSKAPY; FINNLKQDEK; KTGYEIEKAK; VYSDFENREK; LGVIAKSGTK; CYIIKKVKIK; GNLEYTNLFY; SFDDKSILIK; RMNESQKSIY; KNLEPEKLEK; DAYIWSSDGK; SKIKSSSVKK; SGMPSKNPGK; INIINEFIEK; ISNASDAIDK; YYPNTKPGVK; SMGQEVKEIK; NHINYPIYIK; IIFRLCYIIKK; ILSKIKSSSVK; KLKFLSLTNEK; RMNESQKSIYY; KLNETTALWTK; SLLPNYLRFIK; SLYSHKEIFLR; KILSELEKLSK; HLGVIAKSGTK; YLIIHSLYSHK; ILSELEKLSKK; KIMLSMGQEVK; GFYSAFIVSEK; FIIFRLCYIIK; KLYLNKEGLEY; ISFDDKSILIK; GVYSDFENREK; FINIINEFIEK; KIKLKRKSCMK; LSKIKSSSVKK; IVSEKVEVTSK; SSSVDGFVSFK; KFLSLTNEKFK; SLIGQFGVGFY; ALWTKNKSEIK; SVKKILSELEK; KTGYEIEKAKK; TSGMPSKNPGK; VSEKVEVTSKK; IIKKVKIKLKR; YIIKKVKIKLK; EILQQNKILSK; NVSREILQQNK; KVKEILKDHIK; LISNASDAIDK; FINNLKQDEKK; IIKKYSNHINY; NLFYVPSKAPY; YITGGKENILK; VQNLKNLEPEK; ILKENPIVAAY; IALFIIFRLCY; LYYPNTKPGVK; TSNELKDENFK; DSNDPTYQMQK; LSMGQEVKEIK |
| | HLA-A1101 | SVDGFVSFK; SIYYITGGK; TTALWTKNK; GVIAKSGTK; KLISLIRFK; HINYPIYIK; MLTSGMPSK; YSAFIVSEK; NIINEFIEK; IIHSLYSHK; GVYSDFENR; GSLLPNYLR; KIKSSSVKK; QIALFIIFR; LQQNKILSK; KLSKKNPEK; ASDAIDKLK; AILNLIPEY; LTKVKEILK; KFLSLTNEK; TNLFYVPSK; MLSMGQEVK; LSLTNEKFK; GLKLKAINK; KIEISFDDK; AYIWSSDGK; KVKIKLKRK; LSELEKLSK; TEIKLYLNK; GMPSKNPGK; LIPEYEGLK; YSHKEIFLR; ILELNPNNK; KLKRKSCMK; LPNYLRFIK; GFVSFKEYK; VNLSATLIK; YIIKKVKIK; TNHLGVIAK; NLKNLEPEK; SSVDGFVSFK; AMLTSGMPSK; YTNLFYVPSK; KSIYYITGGK; KQIALFIIFR; IIFRLCYIIK; LTNHLGVIAK; LIIHSLYSHK; GTEIKLYLNK; GTKEFINNLK; ISNASDAIDK; KAEEYNEFYK; ITGGKENILK; ILQQNKILSK; TLTKVKEILK; EVNLSATLIK; IMLSMGQEVK; ETTALWTKNK; ALFIIFRLCY; ILSELEKLSK; KTGYEIEKAK; VSEKVEVTSK; KLKRKSCMKK; RLCYIIKKVK; SFDDKSILIK; YIKYSEPIMK; IIKKVKIKLK; LLPNYLRFIK; VSREILQQNK; SMGQEVKEIK; PILELNPNNK; FINNLKQDEK; LSKIKSSSVK; SGMPSKNPGK; LSELEKLSKK; NLIPEYEGLK; NASDAIDKLK; DAYIWSSDGK; LLYLIIHSLY; QQNKILSKIK; FLSLTNEKFK; FYSAFIVSEK; HINYPIYIKY; KFKNIALEPK; KENPIVAAYK; IIIDSNDPTY; |

|  |  | INIINEFIEK; SSSVDGFVSFK; IIFRLCYIIKK; SLLPNYLRFIK; TSNELKDENFK; KIMLSMGQEVK; GVYSDFENREK; SVKKILSELEK; KILSELEKLSK; ISFDDKSILIK; KLNETTALWTK; TSGMPSKNPGK; SLYSHKEIFLR; IVSEKVEVTSK; YLIIHSLYSHK; FIIFRLCYIIK; GFYSAFIVSEK; KLKFLSLTNEK; FINIINEFIEK; VQNLKNLEPEK; DSNDPTYQMQK; NVSREILQQNK; EAMLTSGMPSK; KTGYEIEKAKK; LSMGQEVKEIK; APYDLYYPNTK; SLIGQFGVGFY; LISNASDAIDK; IALFIIFRLCY; TTFDYENPLMH; ILSKIKSSSVK; VSEKVEVTSKK; YITGGKENILK; KVKEILKDHIK; ILSELEKLSKK; KFLSLTNEKFK; YIIKKVKIKLK; KIKLKRKSCMK; DTLTKVKEILK; SVDGFVSFKEY; LSKIKSSSVKK; RMNESQKSIYY; SGTEIKLYLNK; IYIKYSEPIMK; EILQQNKILSK; LQQNKILSKIK; ALWTKNKSEIK; LIPEYEGLKLK; KLYLNKEGLEY; KYSEPIMKDGK; SGTKEFINNLK; FINNLKQDEKK; KPILELNPNNK; HLGVIAKSGTK; AIIIDSNDPTY; SNHINYPIYIK |
| HLA-A2402 | IYIKYSEPI; FYFKQIALF; KFINIINEF; LYLIIHSLY; AYKEKGFEI; YFKQIALFI; TYQMQKIML; LYSHKEIFL; NYLRFIKGI; CYIIKKVKI; DYENPLMHI; KQIALFIIF; SLLPNYLRF; FYKNTTFDY; YYPNTKPGV; SFDDKSILI; LWTKNKSEI; AYIWSSDGK; KFSEFSKEF; NFKKIEEEF; KYSNHINYPI; FYFKQIALFI; IFYFKQIALF; KFLSLTNEKF; KFINIINEFI; IYIKYSEPIM; YFKQIALFII; NYLRFIKGII; YYITGGKENI; QFGVGFYSAF; RFKSSSVDGF; YYPNTKPGVK; EYEGLKLKAI; AYKEKGFEIL; IFITDSEGSL; LFIIFRLCYI; FYSAFIVSEK; LYSHKEIFLR; FYFKQIALFII; EYANKWKIQEI; EYNEFYKNTTF; YFKQIALFIIF; YYPNTKPGVKL; YYITGGKENIL; AYKEKGFEILI; IYIKYSEPIMK; IFITDSEGSLL; IFYFKQIALFI; IYYITGGKENI; FYVPSKAPYDL; TYQMQKIMLSM; VYSDFENREKL; KYSNHINYPIY; LFIIFRLCYII; CYIIKKVKIKL; EYTNLFYVPSK; KFKNIALEPKI; QFGVGFYSAFI; FYSAFIVSEKV; TFDYENPLMHI |
| HLA-A2902 | FYKNTTFDY; YLNKEGLEY; LFIIFRLCY; FYVPSKAPY; INYPIYIKY; NLEYTNLFY; LYLIIHSLY; SKAPYDLYY; TKAEGNLEY; DTEVNDLLY; FYFKQIALF; KFINIINEF; AILNLIPEY; YSNHINYPI; IIDSNDPTY; KIQEIIKKY; EFYKNTTFDY; HINYPIYIKY; YSNHINYPIY; LYLNKEGLEY; LFYVPSKAPY; IIIDSNDPTY; IWSSDGKTGY; LLYLIIHSLY; ALFIIFRLCY; EAILNLIPEY; HTKAEGNLEY; FDTEVNDLLY; LIGQFGVGFY; KFLSLTNEKF; IFYFKQIALF; RMNESQKSIY; PSKAPYDLYY; YVPSKAPYDLY; YIWSSDGKTGY; IALFIIFRLCY; KLYLNKEGLEY; NLFYVPSKAPY; RMNESQKSIYY; QFDTEVNDLLY; YFKQIALFIIF; DLLYLIIHSLY; EYNEFYKNTTF; KYSNHINYPIY; SLIGQFGVGFY; EGNLEYTNLFY; EFGRCIKEGVY; AIIIDSNDPTY; ILKENPIVAAY; NEFYKNTTFDY; NHINYPIYIKY; EIKAEEYNEFY; IIKKYSNHINY; KWKIQEIIKKY |
| HLA-A6801 | YSAFIVSEK; QIALFIIFR; YSHKEIFLR; TTALWTKNK; SIYYITGGK; SVDGFVSFK; HINYPIYIK; MLTSGMPSK; GVYSDFENR; NIINEFIEK; ENPIVAAYK; MLSMGQEVK; IIHSLYSHK; ELKDENFKK; LPNYLRFIK; EKVEVTSKK; NLKNLEPEK; VSFKEYKER; TKEFINNLK; GFVSFKEYK; GSLLPNYLR; LTKVKEILK; WTKNKSEIK; SEFSKEFGR; LIPEYEGLK; TNLFYVPSK; EEFKDTLTK; NNKIVQNLK; YIIKKVKIK; EGIEEKEEK; GVIAKSGTK; LSLTNEKFK; AYIWSSDGK; KLISLIRFK; YSDFENREK; NPLMHIHTK; DTEVNDLLY; TGYEIEKAK; MGQEVKEIK; SNASDAIDK; ETTALWTKNK; YTNLFYVPSK; FSEFSKEFGR; EVNLSATLIK; SSVDGFVSFK; FVSFKEYKER; DAYIWSSDGK; FYSAFIVSEK; NASDAIDKLK; GTKEFINNLK; LIIHSLYSHK; EGVYSDFENR; TLTKVKEILK; EAILNLIPEY; EGSLLPNYLR; KSIYYITGGK; FLSLTNEKFK; FINNLKQDEK; NLIPEYEGLK; YIKYSEPIMK; LYSHKEIFLR; EFYKNTTFDY; IIFRLCYIIK; NFKKIEEEFK; FSKEFGRCIK; LTNHLGVIAK; NPEKFSEFSK; TTFDYENPLM; DGFVSFKEYK; |

| | | |
|---|---|---|
| | | KAEEYNEFYK; LLYLIIHSLY; NPIVAAYKEK; YSEPIMKDGK; ISNASDAIDK; YSNHINYPIY; ITGGKENILK; HINYPIYIKY; ENPLMHIHTK; TGYEIEKAKK; KQIALFIIFR; INIINEFIEK; LLPNYLRFIK; IIKKVKIKLK; LSKIKSSSVK; NHINYPIYIK; HTKAEGNLEY; EFKDTLTKVK; IIIDSNDPTY; LKFLSLTNEK; SLYSHKEIFLR; EAMLTSGMPSK; TSNELKDENFK; DSNDPTYQMQK; FINIINEFIEK; EYTNLFYVPSK; FIIFRLCYIIK; SSSVDGFVSFK; YITGGKENILK; IIFRLCYIIKK; YLIIHSLYSHK; SVKKILSELEK; ENFKKIEEEFK; NVSREILQQNK; ISFDDKSILIK; GVYSDFENREK; EFINNLKQDEK; LSMGQEVKEIK; FINNLKQDEKK; IIKKVKIKLKR; DTLTKVKEILK; YIIKKVKIKLK; EYKERMNESQK; TTFDYENPLMH; ILSKIKSSSVK; IVSEKVEVTSK; NPNNKIVQNLK; EIKAEEYNEFY; EILQQNKILSK; EFSKEFGRCIK; LISNASDAIDK; GFYSAFIVSEK; IYIKYSEPIMK; EKFKNIALEPK; NLFYVPSKAPY; DLTNHLGVIAK; FKQIALFIIFR; ENPIVAAYKEK; TSGMPSKNPGK; VSEKVEVTSKK; SLIGQFGVGFY; LSKIKSSSVKK; YVPSKAPYDLY; SNASDAIDKLK; LYYPNTKPGVK; SLLPNYLRFIK; SDAYIWSSDGK; GFVSFKEYKER; KFSEFSKEFGR; TKPGVKLFINR; NTTFDYENPLM; IALFIIFRLCY; EEFKDTLTKVK; KIMLSMGQEVK; SGTKEFINNLK; EVNDLLYLIIH; LIPEYEGLKLK; EAILNLIPEYE; EPKIEISFDDK; ILSELEKLSKK; DLLYLIIHSLY; IKAEEYNEFYK; HLGVIAKSGTK; KTGYEIEKAKK |
| | HLA-B0702 | MPSKNPGKF; IPEYEGLKL; VPSKAPYDL; LPLNVSREI; YPIYIKYSE; LIRFKSSSV; SVKKILSEL; YPNTKPGVKL; MPSKNPGKFI; LPLNVSREIL; KVEVTSKKAL; NPNNKIVQNL; YPIYIKYSEPI; YPNTKPGVKLF; LPNYLRFIKGI; KPGVKLFINRI; IPEYEGLKLA; DPTYQMKIML; SATLIKEPSAI; AAYKEKGFEIL; EPEKLEKISIL |
| | HLA-B0801 | IDKLKFLSL; NEKFKNIAL; IIKKVKIKL; QMKIMLSM; IFYFKQIAL; FIIFRLCYI; YLRFIKGII; KIKLKRKSCM; YIIKKVKIKL; HIKEVNLSATL; YFKQIALFIIF; ILIFYFKQIAL |
| | HLA-B1501 | KQIALFIIF; YLNKEGLEY; SSVDGFVSF; WSSDGKTGY; RMNESQKSI; FGVGFYSAF; IAKSGTKEF; QMKIMLSM; KFINIINEF; FLSLTNEKF; KFSEFSKEF; LLYLIIHSL; KENPIVAAY; KIQEIIKKY; LFIIFRLCY; AILNLIPEY; KSASLIGQF; SLLPNYLRF; TKAEGNLEY; YQMQKIMLS; KLNETTALW; LIGQFGVGF; NTKPGVKLF; IKAEEYNEF; INYPIYIKY; IGQFGVGFY; FYVPSKAPY; YQMQKIMLSM; RMNESQKSIY; HTKAEGNLEY; YSNHINYPIY; LLYLIIHSLY; SLIGQFGVGF; LFYVPSKAPY; IIIDSNDPTY; KQFDTEVNDL; ALFIIFRLCY; SSSVDGFVSF; VIAKSGTKEF; GVKLFINRIF; YLIIHSLYSH; IVAAYKEKGF; HINYPIYIKY; ILIKDNGIGM; IINEFIEKDF; GQFGVGFYSA; PSKAPYDLYY; RFKSSSVDGF; ALEPKIEISF; QFGVGFYSAF; LIGQFGVGFY; KLEKISILLF; EIKAEEYNEF; GQFGVGFYSAF; RMNESQKSIYY; ILKENPIVAAY; GVIAKSGTKEF; SLIGQFGVGFY; KLYLNKEGLEY; KQIALFIIFRL; NLFYVPSKAPY; KQFDTEVNDLL; YFKQIALFIIF; KSSSVDGFVSF; AIIIDSNDPTY; YIWSSDGKTGY; IIKKYSNHINY; TSKKALESDAY; IALEPKIEISF; LIFYFKQIALF; YVPSKAPYDLY; ASLIGQFGVGF; HIKEVNLSATL; ILIFYFKQIAL; IALFIIFRLCY; RIFITDSEGSL; KNKSEIKAEEY |
| | HLA-B2705 | FRLCYIIKK; KQIALFIIF; LQQNKILSK; GRCIKEGVY; KKVKIKLKR; SREILQQNK; YQMQKIMLSM; KRKSCMKKQF; FRLCYIIKKV; KQIALFIIFR; FRLCYIIKKVK; GQFGVGFYSAF; IRFKSSSVDGF; KQIALFIIFRL; ERMNESQKSIY; NREKLISLIRF |
| | HLA-B3501 | MPSKNPGKF; FYVPSKAPY; FGVGFYSAF; WSSDGKTGY; IIDSNDPTY; SSVDGFVSF; KAEEYNEFY; TKAEGNLEY; IPEYEGLKL; TTFDYENPL; YLNKEGLEY; IAKSGTKEF; DTEVNDLLY; NASDAIDKL; YIKYSEPIM; EILIMDDEL; KENPIVAAY; SILLFEEAM; KFINIINEF; LPLNVSREI; KKALESDAY; TFDYENPLM; LISNASDAI; YPIYIKYSE; EKLNETTAL; |

| | | |
|---|---|---|
| | | FYFKQIALF; IIDCQDLPL; AILNLIPEY; SKAPYDLYY; IKAEEYNEF; MNESQKSIY; NLEYTNLFY; NPLMHIHTK; EAILNLIPEY; DPTYQMQKIM; YSNHINYPIY; IIIDSNDPTY; VPSKAPYDLY; LPLNVSREIL; YPNTKPGVKL; LFYVPSKAPY; TTFDYENPLM; ISILLFEEAM; LKENPIVAAY; QFGVGFYSAF; SSSVDGFVSF; FDTEVNDLLY; NPNNKIVQNL; YNEFYKNTTF; FKQIALFIIF; EGNLEYTNLF; EFYKNTTFDY; NKSEIKAEEY; HTKAEGNLEY; MDDELDEAIL; IKAEEYNEFY; YQMQKIMLSM; YPNTKPGVKLF; VPSKAPYDLYY; YIWSSDGKTGY; YPIYIKYSEPI; NASDAIDKLKF; IALFIIFRLCY; YFKQIALFIIF; DPTYQMQKIML; IALEPKIEISF; NLFYVPSKAPY; ILKENPIVAAY; LFEEAMLTSGM; AIIIDSNDPTY; TSKKALESDAY; FVSFKEYKERM; YVPSKAPYDLY; QFDTEVNDLLY; EPEKLEKISIL; NPLMHIHTKAE; LIFYFKQIALF; SVDGFVSFKEY; ERMNESQKSIY; EVNDLLYLIIH; DEAILNLIPEY; EYNEFYKNTTF; YKEKGFEILIM |
| | HLA-B4403 | KEIKPILEL; KENPIVAAY; NEFYKNTTF; EEAMLTSGM; KEFINNLKQ; NESQKSIYY; EEFKDTLTK; SEGSLLPNY; REILQQNKI; SEIKAEEYN; TEIKLYLNK; NEFIEKDFL; KEVNLSATL; SEFSKEFGR; TEVNDLLYL; LEKISILLF; KEKGFEILI; QEVKEIKPI; KEILKDHIK; KEIFLRELI; REILQQNKIL; REKLISLIRF; DELDEAILNL; KEIFLRELIS; KEVNLSATLI; SEGSLLPNYL; AEGNLEYTNL; EEFKDTLTKV; EEKLNETTAL; FEILIMDDEL; KEKGFEILIM; KEGLEYANKW; EESGTEIKLY; QEVKEIKPIL; SEIKAEEYNE; KEPSAIIIDS; KEFINNLKQD; TEVNDLLYLI; SEIKAEEYNEF; AEGNLEYTNLF; SEFSKEFGRCI; REILQQNKILS; NEFYKNTTFDY; KEILKDHIKEV; EEKLNETTALW; EEAMLTSGMPS; EESGTEIKLYL; EEFKDTLTKVK; TEVNDLLYLII; DEAILNLIPEY; RELISNASDAI; KEVNLSATLIK; DELDEAILNLI; TEIKLYLNKEG; KEESGTEIKLY; KENILKENPIV; KEFGRCIKEGV |
| | HLA-B5101 | LPLNVSREI; IALEPKIEI; NPGKFINII; MPSKNPGKFI; LPLNVSREIL; EPEKLEKISI; YPNTKPGVKL; YPIYIKYSEPI; LPNYLRFIKGI; KPGVKLFINRI |
| | HLA-B5701 | EGLEYANKW; KLNETTALW; KALESDAYIW; HTKAEGNLEY; |
| CAA44492.1\| Outer surface protein A [Borrelia burgdorferi]; Seq9 | HLA-A0101 | |
| | HLA-A0201 | YLLGIGLIL; FTLEGTLAA; KTSTLTISV; FTKEDTITV; ALIACKQNV; LLGIGLILA; SLEATVDKL; ILKSGEITV; KVTEGTVVL; STLDEKNSV; TLVSKKVTL; GIGLILALI; YLLGIGLILA; TLDEKNSVSV; ALDDSDTTQA; LLGIGLILAL; NILKSGEITV; TLAADGKTTL; VLKDFTLEGT; YSLEATVDKL; YLLGIGLILAL; LVFTKEDTITV; LLGIGLILALI; ILALIACKQNV; STLDEKNSVSV; ALDDSDTTQAT; VLKDFTLEGTL; SLEATVDKLEL; ILKSGEITVAL; ALIACKQNVST; TTLKVTEGTVV; LAADGKTTLKV; LIACKQNVSTL |
| | HLA-A0301 | VVLSKNILK; KTKNLVFTK; LVSKKVTLK; LILALIACK; LTISVNSQK; KAVEITTLK; AADGKTTLK; ISVNSQKTK; RANGTRLEY; SQTKFEIFK; KSDGSGKAK; TQATKKTGK; MTELVSKEK; TLVSKKVTLK; TLTISVNSQK; TVVLSKNILK; GLILALIACK; TLKELKNALK; LSQTKFEIFK; LAADGKTTLK; VTEGTVVLSK; KLELKGTSDK; TTQATKKTGK; GMTELVSKEK; KYSLEATVDK; KQNVSTLDEK; TIADDLSQTK; TISVNSQKTK; KEDAKTLVSK; GSGKAKEVLK; KTIVRANGTR; GTLEGEKTDK; GKAVEITTLK; GTRLEYTDIK; VSKKVTLKDK; IKSDGSGKAK; TLAADGKTTLK; STLTISVNSQK; KTLVSKKVTLK; KVTEGTVVLSK; TTLKELKNALK; LTISVNSQKTK; GTVVLSKNILK; FTKEDTITVQK; IVRANGTRLEY; TLKDKSSTEEK; LVSKKVTLKDK; KFNEKGETSEK; LTIADDLSQTK; SQKTKNLVFTK; KSSTEEKFNEK; ATKKTGKWDSK |
| | HLA-A1101 | VVLSKNILK, KTKNLVFTK, SQTKFEIFK, LTISVNSQK, ATVDKLELK, LILALIACK, STEEKFNEK, KAVEITTLK, LVSKKVTLK, MTELVSKEK, |

EP 2 254 592 B1

| | | AADGKTTLK, SAGTNLEGK, YSLEATVDK, GSGTLEGEK, TQATKKTGK, RANGTRLEY, IADDLSQTK, ISVNSQKTK, KSDGSGKAK, GGMTELVSK, TEGTVVLSK, EITTLKELK, TIVRANGTR; TVVLSKNILK; TTQATKKTGK; TIADDLSQTK; VTEGTVVLSK; LSQTKFEIFK; SSTEEKFNEK; GLILALIACK; KQNVSTLDEK; TLTISVNSQK; TLVSKKVTLK; TISVNSQKTK; LAADGKTTLK; GMTELVSKEK; TLKELKNALK; KTIVRANGTR; GSGKAKEVLK; GTLEGEKTDK; VSKKVTLKDK; GTRLEYTDIK; KLELKGTSDK; DSAGTNLEGK; STLTISVNSQK; TTLKELKNALK; KVTEGTVVLSK; KTLVSKKVTLK; GTVVLSKNILK; LTIADDLSQTK; TLAADGKTTLK; AVEITTLKELK; LTISVNSQKTK; SQKTKNLVFTK; KSSTEEKFNEK; ATKKTGKWDSK; TLKDKSSTEEK; FTKEDTITVQK; TLEGTLAADGK; LVSKKVTLKDK; MTELVSKEKDK; DLSQTKFEIFK; YTDIKSDGSGK; LVSKEKDKDGK; KFNEKGETSEK; IGLILALIACK |
| | HLA-A2402 | KYLLGIGLI; KWDSKTSTL; KYDSAGTNL; KYLLGIGLIL; KWDSKTSTLTI; KYSLEATVDKL |
| | HLA-A2902 | IVRANGTRLEY; TIADDLSQTKF |
| | HLA-A6801 | LTISVNSQK; ETSEKTIVR; MTELVSKEK; EITTLKELK; TIVRANGTR; KAVEITTLK; DAKTLVSKK; KTKNLVFTK; STEEKFNEK; ATVDKLELK; LVSKKVTLK; SQTKFEIFK; YSLEATVDK; EGTLAADGK; ISVNSQKTK; LILALIACK; DIKSDGSGK; VVLSKNILK; EDAKTLVSK; DSDTTQATK; SAGTNLEGK; TVVLSKNILK; TLTISVNSQK; EATVDKLELK; TIADDLSQTK; DSAGTNLEGK; TTQATKKTGK; LAADGKTTLK; KTIVRANGTR; TISVNSQKTK; TLVSKKVTLK; SSTEEKFNEK; TLKELKNALK; LSQTKFEIFK; VTEGTVVLSK; EDAKTLVSKK; DSDTTQATKK; GLILALIACK; |
| | HLA-B0702 | IVRANGTRL; KVTEGTVVL; LAADGKTTL; LPGGMTELV; TVVLSKNIL; ; KAVEITTLKEL |
| | HLA-B0801 | TLKELKNAL; ; ILKSGEITVAL; YLLGIGLILAL; TLKVTEGTVVL |
| | HLA-B1501 | SQKTKNLVF; RANGTRLEY; YLLGIGLIL; KVTEGTVVL; TLKELKNAL; KAKEVLKDF; IVRANGTRL; LGIGLILAL; TLAADGKTTL; IVRANGTRLEY; YLLGIGLILAL; VQKYDSAGTNL; ILKSGEITVAL; VSKEKDKDGKY; VLKDFTLEGTL; TLKVTEGTVVL; VNSQKTKNLVF |
| | HLA-B2705 | KKYLLGIGL; VRANGTRLEY; GKWDSKTSTL; KKYLLGIGLIL |
| | HLA-B3501 | LAADGKTTL; RANGTRLEY; FTLEGTLAA; TVVLSKNIL; VALDDSDTT; YLLGIGLIL; LPGGMTELV; IADDLSQTKF; LPGGMTELVS; LKVTEGTVVL; YSLEATVDKL; YLLGIGLILAL; NSVSVDLPGGM; IVRANGTRLEY; TIADDLSQTKF; KAVEITTLKEL |
| | HLA-B4403 | KEVLKDFTL; GEITVALDD; KEKDKDGKY; KEDAKTLVS; GEITVALDDS; KEDTITVQKY; GEITVALDDSD; KEVLKDFTLEG |
| | HLA-B5101 | LPGGMTELV; |
| | HLA-B5701 | TTQATKKTGKW; |
| \|BAA22351.1 \| Outer surface protein B [Borrelia garinii]; Seq10 | HLA-A0101 | LTEETEETY; RTNGTTLEY; WSDTSNTLT; ITDDLNTIT; WSDTSNTLTV; ITDDLNTITV; LTDGTITVQNY; ITRTNGTTLEY; WSDTSNTLTVS; YSDMTNDENAT |
| | HLA-A0201 | FLTDGTITV; YLLGFALVL; KLAGTATEI; LLGFALVLA; SLTEETEET; KIKDFVFLT; FLDDTSSGS; SITDDLNTI; GIMLEGNLV; NLVGGKTSV; LVTEDTVKL; VTMSITDDL; YLLGFALVLA; KLFNDTKIFI; LLGFALVLAL; FLDDTSSGST; ITDDLNTITV; FLTDGTITVQ; AVWSDTSNTL; ALIACGQKGA; KLSTKKITRT; KKYLLGFALV; YLLGFALVLAL; FVFLTDGTITV; SITDDLNTITV; LLGFALVLALI; KLFNDTKIFIS; NLVGGKTSVEI; TMSITDDLNTI; MTNDENATKAV; ATVDTVELKGV |
| | HLA-A0301 | KVASKVFKK; KTGKLSTKK; RTNGTTLEY; SSNTKVASK; ATKAVETLK; KLSTKKITR; MTNDENATK; VTLKKEIEK; ETYDSSNTK; TLTVSADSK; KEGTVTLKK; VLALIACGQK; KLAGTATEIK; NTKVASKVFK; |

841

| | | |
|---|---|---|
| | | NTLTVSADSK; TYKTGKLSTK; MLEGNLVGGK; KIFISKEKNK; VQNYDTAGTK; TVTLKKEIEK; TLTVSADSKK; EIKEGTVTLK; DLEALKAALK; DSSNTKVASK; RATVDTVELK; KEIEKAGTVK; YKTGKLSTKK; GSGGKLEGVK; TVELKGVADK; DTKIFISKEK; IKEGTVTLKK; FNDTKIFISK; IMLEGNLVGGK; LVLALIACGQK; ETYKTGKLSTK; KLEGVKSDQSK; FLDDTSSGSTK; LFNDTKIFISK; LVGGKTSVEIK; ITRTNGTTLEY; NTLTVSADSKK; SLTEETEETYK; TVQNYDTAGTK; EIKEGTVTLKK |
| | HLA-A1101 | KVASKVFKK; ATVDTVELK; SSNTKVASK; ATKAVETLK; MTNDENATK; ETYDSSNTK; KTGKLSTKK; VTLKKEIEK; TVKLFNDTK; RTNGTTLEY; TLTVSADSK; LTVSADSKK; VSADSKKIK; LALIACGQK; DTSSGSTKK; TKVASKVFK; EIKDLEALK; KLSTKKITR; TVTLKKEIEK; NTLTVSADSK; TVSADSKKIK; KIFISKEKNK; RATVDTVELK; VQNYDTAGTK; VLALIACGQK; KLAGTATEIK; TVELKGVADK; TLTVSADSKK; NTKVASKVFK; LTEETEETYK; MLEGNLVGGK; GSGGKLEGVK; NATKAVETLK; DSSNTKVASK; DTVKLFNDTK; TKVASKVFKK; EIKEGTVTLK; TVQNYDTAGTK; LVLALIACGQK; ATEIKDLEALK; TVETYDSSNTK; GTVTLKKEIEK; NTLTVSADSKK; IMLEGNLVGGK; SLTEETEETYK; ETYKTGKLSTK; IACGQKGAEPK; NTKVASKVFKK; LFNDTKIFISK; LVGGKTSVEIK; LTVSADSKKIK; SNTKVASKVFK; KLEGVKSDQSK; VADKNDGSGGK; EIKEGTVTLKK |
| | HLA-A2402 | VWSDTSNTL; KYLLGFALV; GFALVLALI; NYDTAGTKL; KYLLGFALVL; DFVFLTDGTI; VWSDTSNTLV; KYELRATVDTV; KYLLGFALVLA |
| | HLA-A2902 | RTNGTTLEY; DLNTITVETY; SLTEETEETY; ITRTNGTTLEY |
| | HLA-A6801 | ETYDSSNTK; MTNDENATK; DTSSGSTKK; LTVSADSKK; TVKLFNDTK; EIKDLEALK; ATVDTVELK; ATKAVETLK; TLTVSADSK; KVASKVFKK; TKVASKVFK; TKIFISKEK; SSNTKVASK; RTNGTTLEY; EIEKAGTVK; LALIACGQK; QNYDTAGTK; LAGTATEIK; NDTKIFISK; TATEIKDLE; VTLKKEIEK; DTVKLFNDTK; DTKIFISKEK; NATKAVETLK; NTLTVSADSK; NTKVASKVFK; ETEETYKTGK; EIKEGTVTLK; TVTLKKEIEK; TLTVSADSKK; DSSNTKVASK; EVEDSKKDQK; TVSADSKKIK; LTEETEETYK; VLALIACGQK; RATVDTVELK; MLEGNLVGGK; DLEALKAALK; DMTNDENATK; TKVASKVFKK; TVELKGVADK; ETYDSSNTKV; DLNTITVETY; TEIKDLEALK; KIFISKEKNK; KLAGTATEIK; TYKTGKLSTK; ETYKTGKLSTK; NTLTVSADSKK; DTVELKGVADK; TVETYDSSNTK; NTKVASKVFKK; TVQNYDTAGTK; EIKEGTVTLKK; LTVSADSKKIK; ENATKAVETLK; FISKEKNKDGK; LVLALIACGQK; SNTKVASKVFK; SLTEETEETYK; GTVTLKKEIEK; IACGQKGAEPK; DLPLVTEDTVK; LVGGKTSVEIK; ATEIKDLEALK; LFNDTKIFISK; EETEETYKTGK; EKNKDGKYELR; TKIFISKEKNK; TYKTGKLSTKK; NDTKIFISKEK; DSKKDQKDASK; SNTLTVSADSK; EDTVKLFNDTK |
| | HLA-B0702 | ITRTNGTTL; RATVDTVEL; KVFKKQGSL; AVWSDTSNTL; KITRTNGTTL; LPLVTEDTVKL; TAVWSDTSNTL; AVETLKNGIML |
| | HLA-B0801 | MKKYLLGFAL; YLLGFALVLAL |
| | HLA-B1501 | RTNGTTLEY; KLFNDTKIF; YLLGFALVL; NTKVASKVF; LGFALVLAL; LTEETEETY; SLTEETEETY; LVTEDTVKLF; IEKAGTVKLF; ITRTNGTTLEY; YLLGFALVLAL; ISKEKNKDGKY; SSNTKVASKVF; TVKLFNDTKIF; VQNYDTAGTKL; VSADSKKIKDF |
| | HLA-B2705 | KKYLLGFAL; TRTNGTTLEY; LRATVDTVEL; KKYLLGFALVL; LRATVDTVELK |
| | HLA-B3501 | LTEETEETY; FALVLALIA; LPLVTEDTV; RTNGTTLEY; TAVWSDTSN; YLLGFALVL; DASKKDLPL; RATVDTVEL; DLNTITVETY; AVWSDTSNTL; FALVLALIAC; LVTEDTVKLF; TAVWSDTSNT; TRTNGTTLEY; FVFLTDGTIT; SLTEETEETY; LPLVTEDTVK; |

| | | |
|---|---|---|
| | | TAVWSDTSNTL; LPLVTEDTVKL; TATEIKDLEAL; YLLGFALVLAL; KAVETLKNGIM; ITRTNGTTLEY; TVKLFNDTKIF; DDLNTITVETY; FVFLTDGTITV |
| | HLA-B4403 | KEIEKAGTV; KEKNKDGKY; EETYKTGKL; AEPKHNDQE; EETEETYKT; AEPKHNDQEV; EETYKTGKLS; VEIKEGTVTL; KEIEKAGTVKL; KEGTVTLKKEI |
| | HLA-B5101 | LPLVTEDTV; FALVLALIA; LPLVTEDTVK; LPLVTEDTVKL |
| | HLA-B5701 | LTEETEETY; GSTKKTAVW; RTNGTTLEY; ; SSGSTKKTAVW; GSLTEETEETY,; ITRTNGTTLEY |
| AAM22469.1\| Outer surface protein C [Borrelia afzelii]; Seq11 | HLA-A0101 | LSAILMTLF; ITDSNAFVL; ; ITDSNAFVLAV |
| | HLA-A0201 | ILMTLFLFI; SLLAGVHEI; KITDSNAFV; KLFKSVEGL; TLSAILMTL; TLVSSIDEL; ALTNSVKEL; GVHEISTLI; SAILMTLFL; FLFISCNNS; SVKELTSPV; LITEKLSKL; LMTLFLFIS; LVSSIDELA; AQEALTNSV; KLFKSVEGLV; AILMTLFLFI; KITDSNAFVL; NTLSAILMTL; TLVSSIDELA; ILMTLFLFIS; VLAVKEVETL; TLITEKLSKL; LLAGVHEIST; GLVKAAQEAL; AAQEALTNSV; FVLAVKEVET; TLSAILMTLFL; VLAVKEVETLV; LLAGVHEISTL; FVLAVKEVETL; KITDSNAFVLA; SLLAGVHEIST; ITDSNAFVLAV; ILMTLFLFISC; KAAQEALTNSV; SAILMTLFLFI |
| | HLA-A0301 | KLKNSGELK; TLITEKLSK; ELANKAIGK; LANKAIGKK; RVSHADLGK; FISCNNSGK; ITEKLSKLK; AILKTNADK; EISTLITEK; ILMTLFLFI; STLITEKLSK; LITEKLSKLK; KTKGAEELGK; KAILKTNADK; ELANKAIGKK; LFKSVEGLVK; AQEALTNSVK; LFISCNNSGK; DSNAFVLAVK; TSPVVAESPK; GAEELGKLFK; ILKTNADKTK; SKLKNSGELK; VSSIDELANK; HEISTLITEK; ; KLFKSVEGLVK; FLFISCNNSGK; KLRVSHADLGK; TLITEKLSKLK; KLKNSGELKAK; ISTLITEKLSK; LTSPVVAESPK; LVSSIDELANK; LSKLKNSGELK; TLSAILMTLFL; KGAEELGKLFK; AILKTNADKTK |
| | HLA-A1101 | SSIDELANK; TLITEKLSK; RVSHADLGK; AILKTNADK; GVNDDDAKK; ITEKLSKLK; LANKAIGKK; KLKNSGELK; FISCNNSGK; EISTLITEK; PVVAESPKK; SNAFVLAVK; AILMTLFLF; ELANKAIGK; GPNLTEISK; STLITEKLSK; STNPADESAK; VSSIDELANK; TSPVVAESPK; KAILKTNADK; DSNAFVLAVK; AQEALTNSVK; KTKGAEELGK; GAEELGKLFK; LITEKLSKLK; AILMTLFLFI; LFISCNNSGK; LTSPVVAESPK; KLFKSVEGLVK; ASTNPADESAK; LVSSIDELANK; AAQEALTNSVK; TLITEKLSKLK; ISTLITEKLSK; TSPVVAESPKK; AILKTNADKTK; FLFISCNNSGK; LSKLKNSGELK; KGAEELGKLFK; KLKNSGELKAK; KLRVSHADLGK |
| | HLA-A2402 | AILMTLFLF; ILMTLFLFI; SAILMTLFLF; LSAILMTLFLF; NTLSAILMTLF |
| | HLA-A2902 | SAILMTLFLF; |
| | HLA-A6801 | EISTLITEK; FISCNNSGK; ELANKAIGK; SSIDELANK; SNAFVLAVK; LANKAIGKK; ITEKLSKLK; TLITEKLSK; RVSHADLGK; GVNDDDAKK; SPVVAESPK; DSNAFVLAVK; STNPADESAK; CSEEFTNKLR; TSPVVAESPK; ELANKAIGKK; LFISCNNSGK; STLITEKLSK; ELKAKIEDAK; LITEKLSKLK; HEISTLITEK; KTKGAEELGK; SPVVAESPKK; SAILMTLFLF; VSSIDELANK; LTSPVVAESPK; FLFISCNNSGK; LVSSIDELANK; TSPVVAESPKK; ISTLITEKLSK; TLITEKLSKLK; ELKAKIEDAKK; LSKLKNSGELK; QQNGLGAEANR; ASTNPADESAK; TDSNAFVLAVK; MTLFLFISCNN |
| | HLA-B0702 | SVKELTSPV; LVKAAQEAL; SAILMTLFL; AAQEALTNSV; KAAQEALTNSV; GPNLTEISKKI |
| | HLA-B0801 | KLRVSHADL; LVKAAQEAL; ; |
| | HLA-B1501 | LVKAAQEAL; QQNGLGAEA; AQEALTNSV; KKITDSNAF; IQQNGLGAE; SVKELTSPV; KLRVSHADL; LSAILMTLF; TLSAILMTLF; IQQNGLGAEA; SAILMTLFLF; ISKKITDSNAF; LLAGVHEISTL; LSAILMTLFLF |

| | HLA-B2705 | LRVSHADLGK; |
|---|---|---|
| | HLA-B3501 | KKITDSNAF; SAILMTLFL; LAVKEVETL; LSAILMTLF; DAKKCSEEF; NPADESAKG; SAILMTLFLF; EALTNSVKEL; LAGVHEISTL; NPADESAKGPN; NTLSAILMTLF; FVLAVKEVETL; ISKKITDSNAF |
| | HLA-B4403 | EEFTNKLRV; KELTSPVVA; AEANRNESL; EEFTNKLRVS; AEANRNESLL; AEANRNESLLA; EEFTNKLRVSH; KELTSPVVAES |
| | HLA-B5101 | GPNLTEISKKI; LAGVHEISTLI |
| | HLA-B5701 | ISKKITDSNAF |
| AAC62927.1\| OspE-related lipoprotein [Borrelia garinii]; Seq12 | HLA-A0101 | GSFKTSLYY; SSDTIKFSEF; |
| | HLA-A0201 | FIICAVFVL; KMKMFIICA; MKMFIICAV; ATFFCIEEA; IICAVFVLI; MFIICAVFV; KMFIICAVFV; FIICAVFVLI; KMKMFIICAV; KMFIICAVFVL; ATFFCIEEAEV; MKMFIICAVFV; KKMKMFIICAV; GLSGQASSDTI; FIICAVFVLIS; TLVIRKEQDGV |
| | HLA-A0301 | AMTNVGSFK; KTSLYYGYK; GSFKTSLYY; NLGTLVIRK; LISSCGNFR; FTVNIKNKK; GQASSDTIK; KAMTNVGSFK; GIKGKEITTK; VGSFKTSLYY; SNLGTLVIRK; VLISSCGNFR; NVGSFKTSLY; KFSEFTVNIK; QLRGHSATFF; FKTSLYYGYK; QSSTNGIKGK; WSNLGTLVIRK; GSFKTSLYYGY; SFKTSLYYGYK; FVLISSCGNFR |
| | HLA-A1101 | KTSLYYGYK; AMTNVGSFK; GSFKTSLYY; SSTNGIKGK; NLGTLVIRK; GQASSDTIK; FTVNIKNKK; LISSCGNFR; FSEFTVNIK; KAMTNVGSFK; VLISSCGNFR; SNLGTLVIRK; QSSTNGIKGK; KFSEFTVNIK; GIKGKEITTK; SEFTVNIKNK; SGQASSDTIK; NVGSFKTSLY; WSNLGTLVIRK; GSFKTSLYYGY; SFKTSLYYGYK; NSEHITFSGDK; FVLISSCGNFR; NVGSFKTSLYY; NVIGTINGQLR; LSGQASSDTIK; SEFTVNIKNKK; FSEFTVNIKNK |
| | HLA-A2402 | KAMTNVGSF; DWSNLGTLV; KFSEFTVNI; MFIICAVFVL; DWSNLGTLVI; MFIICAVFVLI; VFVLISSCGNF; ETINNSEHITF |
| | HLA-A2902 | GSFKTSLYY; FKTSLYYGY; KMFIICAVF; SFKTSLYYGY; NVGSFKTSLY; VGSFKTSLYY; NVGSFKTSLYY; GSFKTSLYYGY |
| | HLA-A6801 | FTVNIKNKK; LISSCGNFR; EAEVNNFVK; KTSLYYGYK; FSEFTVNIK; AMTNVGSFK; EFTVNIKNK; DTIKFSEFT; SSTNGIKGK; EQSSTNGIK; EHITFSGDK; HSATFFCIE; NLGTLVIRK; MTNVGSFKT; NVIGTINGQ; ETINNSEHI; VLISSCGNFR; EEAEVNNFVK; KAMTNVGSFK; EFTVNIKNKK; WSNLGTLVIR; NVGSFKTSLY; VIGTINGQLR; FKTSLYYGYK; HSATFFCIEE; QSSTNGIKGK; ETINNSEHIT; DTIKFSEFTV; NVIGTINGQLR; FVLISSCGNFR; NSEHITFSGDK; FSEFTVNIKNK; WSNLGTLVIRK; ETINNSEHITF; SFKTSLYYGYK; DWSNLGTLVIR; NVGSFKTSLYY; EQSSTNGIKGK; SEFTVNIKNKK; TNVGSFKTSLY |
| | HLA-B0702 | KAMTNVGSF; FVKAMTNVGSF |
| | HLA-B0801 | QLRGHSATF; FVKAMTNVGSF; NGQLRGHSATF |
| | HLA-B1501 | KMFIICAVF; QLRGHSATF; GSFKTSLYY; KAMTNVGSF; VLISSCGNF; GQLRGHSAT; GQLRGHSATF; QLRGHSATFF; GQASSDTIKF; FVLISSCGNF; TINNSEHITF; KMKMFIICAV; VGSFKTSLYY; FVKAMTNVGSF; KMKMFIICAVF; GQLRGHSATFF; KMFIICAVFVL; GSFKTSLYYGY; ASSDTIKFSEF; ETINNSEHITF; FCIEEAEVNNF |
| | HLA-B2705 | GQLRGHSATF; FRSSLSDQGSL |
| | HLA-B3501 | EVNNFVKAM; QASSDTIKF; FIICAVFVL; KAMTNVGSF; QLRGHSATF; MKMFIICAVF; TINNSEHITF; MFIICAVFVL; FVLISSCGNF; NVGSFKTSLY; EAEVNNFVKAM; FVKAMTNVGSF; ETINNSEHITF; FCIEEAEVNNF; NVGSFKTSLYY; MTNVGSFKTSL |
| | HLA-B4403 | SEFTVNIKN; EEAEVNNFV; KEITTKIET; EEQSSTNGI; AEVNNFVKA; AEVNNFVKAM; SEFTVNIKNK; KEITTKIETI; AEVNNFVKAMT; SEFTVNIKNKK; EEAEVNNFVKA |
| | HLA-B5101 | |

| | HLA-B5701 | |
|---|---|---|
| CAA57806.1\| Outer surface protein G [Borrelia burgdorferi]; Seq13 | HLA-A0101 | |
| | HLA-A0201 | LIICAVFVL; NLIICAVFV; SLFNPPPVL; KMKNLIICA; FVLIISCKI; ALKNIEEEL; IICAVFVLI; VIDDALKNI; ATGESTEKV; KMKNLIICAV; LIICAVFVLI; NLIICAVFVL; ALKYAKELGV; KVIDDALKNI; IICAVFVLII; SLFNPPPVLPA; LIICAVFVLII; LMQGDDPNNSL; NLIICAVFVLI; KALKYAKELGV; FVLIISCKIDA; KKMKNLIICAV; AVFVLIISCKI |
| | HLA-A0301 | GTNTNDFVK; VFVLIISCK; AVFVLIISCK; SSHDNTPVLK; GTNTNDFVKK; KIDASSEDLK; ESATGESTEK; KVKKQGNIGQK; KLAEPQNIEDK; KQGNIGQKALK; ASSHDNTPVLK; CAVFVLIISCK; FVKKVIDDALK; ATGESTEKVKK; ALKNIEEELEK |
| | HLA-A1101 | GTNTNDFVK; SATGESTEK; GQKALKYAK; VFVLIISCK; PVLKAVQAK; GQQEGKEEK; AVFVLIISCK; SSHDNTPVLK; GTNTNDFVKK; KIDASSEDLK; SSEDLKQNVK; ATGESTEKVK; ESATGESTEK; ASSHDNTPVLK; ATGESTEKVKK; CAVFVLIISCK; ASSEDLKQNVK; KVKKQGNIGQK; KQGNIGQKALK; NIGQKALKYAK; SATGESTEKVK; ALKNIEEELEK; KLAEPQNIEDK; FVKKVIDDALK; NTPVLKAVQAK |
| | HLA-A2402 | |
| | HLA-A2902 | NIGQKALKY; YAKELGVNGSY |
| | HLA-A6801 | GTNTNDFVK; SATGESTEK; ELKDKIDKR; VFVLIISCK; EPQNIEDKK; TNTNDFVKK; DLKQNVKEK; IDASSEDLK; ESATGESTEK; EARKKFQEFK; AVFVLIISCK; SSHDNTPVLK; GTNTNDFVKK; TPVLKAVQAK; DGTNTNDFVK; ELKDKIDKRK; LAEPQNIEDK; SSEDLKQNVK; CAVFVLIISCK; FVKKVIDDALK; NTPVLKAVQAK; EEARKKFQEFK; EIQELKDKIDK; SATGESTEKVK; NIGQKALKYAK; LAEPQNIEDKK; CKIDASSEDLK; QAKDGGQQEGK; DGTNTNDFVKK; ELKDKIDKRKK; ASSHDNTPVLK |
| | HLA-B0702 | TPVLKAVQA; LPASSHDNT; ; LPASSHDNTPV |
| | HLA-B0801 | EARKKFQEF; FVKKVIDDAL; |
| | HLA-B1501 | SLFNPPPVL; KELGVNGSY; NVKEKVEGF; KQGNIGQKAL; MQGDDPNNSL; KMKNLIICAV; YAKELGVNGSY; KMKNLIICAVF; KQNVKEKVEGF; MQGDDPNNSLF; SVNDGTNTNDF; LMQGDDPNNSL; GQKALKYAKEL |
| | HLA-B2705 | GQKALKYAK; ; KRKKELEEARK; KQGNIGQKALK |
| | HLA-B3501 | NPPPVLPAS; EARKKFQEF; LIICAVFVL; MKNLIICAVF; FVKKVIDDAL; TPVLKAVQAK; LPASSHDNTP; YAKELGVNGSY; SVNDGTNTNDF; LPASSHDNTPV; PASSHDNTPVL; NPPPVLPASSH; MQGDDPNNSLF |
| | HLA-B4403 | QEFKEQVES; KELGVNGSY; EEARKKFQEF; EEKEKEIQEL; KELEEARKKF; KEIQELKDKI; EEELELKLAEPQ; QEFKEQVESAT; EELELKLAEPQN |
| | HLA-B5101 | PPPVLPASS; TPVLKAVQA; LPASSHDNT; LPASSHDNTP; NPPPVLPASS; CAVFVLIISC; LPASSHDNTPV |
| | HLA-B5701 | |
| AAC44770.1\| FlaA protein (Borrelia burgdorferi) ; Seq14 | HLA-A0101 | LTNYVDYVY; KVSVYSLGY; FEDMNGMEY; DSESVFKVY; RLDLTNYVDY; SSKVKNFIFY; LTPSARLQAY; SSTRLDLTNY; HSSKVKNFIFY; WADLIWSNPNY; PSSTRLDLTNY; DIDSESVFKVY; KTMKEIKVSVY |
| | HLA-A0201 | FLLSTVLFA; ILFFLLSTV; KVKNFIFYV; YAGDTILGV; VLFEDMNGM; LIWSNPNYI; AMIMPPFKI; YVYSGASGI; GINNWSVLL; VLLTPSARL; KMRFKAFRV; YVDYVYSGA; LVLDFAELA; YIPNISSRI; TMKEIKVSV; GLIDNIKTM; DLTNYVDYV; RVLYDKLSV; SVYSLGYEI; KAKSILFFL; LLSTVLFAQ; KLREKISIA; LQAYVKNSV; SILFFLLST; LGYEIDLEV; VLYDKLSVS; GLAEGSKRA; VLYDKLSVSI; FIFYVKDLRV; FLGKGLIDNI; SLGYEIDLEV; SILFFLLSTV; FLLSTVLFAQ; RLQAYVKNSV; KTMKEIKVSV; VLFAQETDGL; ILFFLLSTVL; YVYSGASGIV; |

|  |  |  |
|---|---|---|
|  |  | KLSVSIDSDI; FFLLSTVLFA; SKVKNFIFYV; VLFPSYSQSS; LQAYVKNSVV; KAKSILFFLL; YIPNISSRII; YVKNSVVAPA; LLTPSARLQA; RLDLTNYVDYV; SIAEGSFQNFV; SIDSDIDSESV; VLFPSYSQSSA; YSLGYEIDLEV; RLQAYVKNSVV; KVYETSGTESL; YAGDTILGVRV; SLGYEIDLEVL; VVDLGINNWSV; SILFFLLSTVL; KVSVYSLGYEI; FIFYVKDLRVL; LLSTVLFAQET; KSILFFLLSTV; VLLTPSARLQA; SVYSLGYEIDL; YVKNSVVAPAV; LTPSARLQAYV; VLFAQETDGLA; IMPPFKIPFYS; IIKDDVPNYPL; RVLYDKLSVSI; KLKAHETFKRV |
|  | HLA-A0301 | KLKAHETFK; RVSKSHSSK; YAYSMGTLK; GSFQNFVEK; LASSKMRFK; KVSVYSLGY; SVVAPAVVK; SAMIMPPFK; RFKAFRVSK; TSGTESLRK; YSMGTLKFK; YSGASGIVK; LIDNIKTMK; GTESLRKLK; GVRVLFPSY; KAFRVSKSH; KVKNFIFYVK; AYSMGTLKFK; SSAMIMPPFK; VYSGASGIVK; GLIDNIKTMK; PLASSKMRFK; EYAYSMGTLK; RKLKAHETFK; VPNYPLASSK; ETSGTESLRK; VSKSHSSKVK; IMPPFKIPFY; SDIDSESVFK; SSKVKNFIFY; NSVVAPAVVK; RLDLTNYVDY; KLKAHETFKR; MRFKAFRVSK; APAVVKSESK; AHETFKRVLK; GESGNQFLGK; SSKMRFKAFR; LVLDFAELAR; FRVSKSHSSK; MIMPPFKIPF; IPNISSRIIK; KISIAEGSFQ; KMRFKAFRVSK; YVYSGASGIVK; RVSKSHSSKVK; MEYAYSMGTLK; MIMPPFKIPFY; KAHETFKRVLK; QSSAMIMPPFK; YAYSMGTLKFK; HSSKVKNFIFY; ILGVRVLFPSY; KTMKEIKVSVY; VLFEDMNGMEY; FLGKGLIDNIK; TSGTESLRKLK; YIPNISSRIIK; ASSKMRFKAFR; LLTPSARLQAY |
|  | HLA-A1101 | SAMIMPPFK; SVVAPAVVK; GSFQNFVEK; RVSKSHSSK; YAYSMGTLK; YSMGTLKFK; TSGTESLRK; SVLLTPSAR; KLKAHETFK; GTESLRKLK; LASSKMRFK; KVSVYSLGY; YSGASGIVK; SARLQAYVK; RFKAFRVSK; ESGNQFLGK; VKNFIFYVK; LIDNIKTMK; LTNYVDYVY; DIDSESVFK; AVVKSESKR; RVLFPSYSQ; FVEKIESEK; KSILFFLLS; FIFYVKDLR; GVRVLFPSY; YVKDLRVLY; SVYSLGYEI; STRLDLTNY; SSAMIMPPFK; KVKNFIFYVK; SSKMRFKAFR; ETSGTESLRK; GLIDNIKTMK; AYSMGTLKFK; SSKVKNFIFY; LVLDFAELAR; VSKSHSSKVK; NSVVAPAVVK; PSARLQAYVK; PLASSKMRFK; SSTRLDLTNY; ETFKRVLKLR; SDIDSESVFK; RIIKDDVPNY; KLKAHETFKR; VPNYPLASSK; VYSGASGIVK; ETDGLAEGSK; QSSAMIMPPFK; YVYSGASGIVK; YAYSMGTLKFK; MIMPPFKIPFY; KAHETFKRVLK; ASSKMRFKAFR; RVSKSHSSKVK; TSGTESLRKLK; KMRFKAFRVSK; MEYAYSMGTLK; YVKDLRVLYDK; TPSARLQAYVK; KTMKEIKVSVY; VAPAVVKSESK; YIPNISSRIIK; HSSKVKNFIFY; VLFEDMNGMEY; DVPNYPLASSK; KNSVVAPAVVK; SKVKNFIFYVK; SGESGNQFLGK; AEGSFQNFVEK |
|  | HLA-A2402 | SYSQSSAMI; EYAYSMGTL; FFLLSTVLF; LYDKLSVSI; AYSMGTLKF; FYVKDLRVL; SFQNFVEKI; NYPLASSKM; VYSGASGIV; KFKGWADLI; NYIPNISSRI; FYSGESGNQF; LFFLLSTVLF; GYEIDLEVLF; EYAYSMGTLK; DYVYSGASGI; VYETSGTESL; VYSLGYEIDL; RYAGDTILGV; SYSQSSAMIM; FYVKDLRVLY; EYAYSMGTLKF; NYPLASSKMRF; NYIPNISSRII; IWSNPNYIPNI; FYSGESGNQFL; DYVYSGASGIV |
|  | HLA-A2902 | YVKDLRVLY; DMNGMEYAY; LTNYVDYVY; KVSVYSLGY; IMPPFKIPF; YEIDLEVLF; GVRVLFPSY; AYSMGTLKF; DLIWSNPNY; FFLLSTVLF; FYVKDLRVLY; IMPPFKIPFY; YAYSMGTLKF; LFEDMNGMEY; DLTNYVDYVY; LFFLLSTVLF; TMKEIKVSVY; SSTRLDLTNY; RIIKDDVPNY; MIMPPFKIPF; SSKVKNFIFY; RLDLTNYVDY; EDMNGMEYAY; IFYVKDLRVLY; VLFEDMNGMEY; MIMPPFKIPFY; AMIMPPFKIPF; EYAYSMGTLKF; HSSKVKNFIFY; ILGVRVLFPSY; ILFFLLSTVLF; FEDMNGMEYAY; DIDSESVFKVY |

| | | |
|---|---|---|
| | HLA-A6801 | ETSGTESLR; FIFYVKDLR; YAYSMGTLK; FVEKIESEK; NYIPNISSR; YSMGTLKFK; SAMIMPPFK; LTNYVDYVY; GSFQNFVEK; SVVAPAVVK; SVLLTPSAR; AVVKSESKR; LASSKMRFK; SKMRFKAFR; RVSKSHSSK; NIKTMKEIK; DIDSESVFK; LARDPSSTR; YVKDLRVLY; TFKRVLKLR; ESGNQFLGK; DLIWSNPNY; LIDNIKTMK; SARLQAYVK; MPPFKIPFY; GTESLRKLK; HETFKRVLK; YPLASSKMR; LKAHETFKR; DMNGMEYAY; VKNFIFYVK; RFKAFRVSK; ETFKRVLKLR; ETSGTESLRK; SSAMIMPPFK; NFIFYVKDLR; EYAYSMGTLK; WSVLLTPSAR; LVLDFAELAR; SSKMRFKAFR; ETDGLAEGSK; ELARDPSSTR; EGSFQNFVEK; NSVVAPAVVK; KVKNFIFYVK; YAGDTILGVR; YETSGTESLR; NFVEKIESEK; MRFKAFRVSK; NYPLASSKMR; DLTNYVDYVY; DTILGVRVLF; SDIDSESVFK; GLIDNIKTMK; KLKAHETFKR; DNIKTMKEIK; YAYSMGTLKFK; YVVYSGASGIVK; QSSAMIMPPFK; ELVLDFAELAR; ASSKMRFKAFR; ETDGLAEGSKR; NPNYIPNISSR; DVPNYPLASSK; YVKDLRVLYDK; MEYAYSMGTLK; YIPNISSRIIK; MIMPPFKIPFY; HSSKVKNFIFY; TPSARLQAYVK; KNFIFYVKDLR; ESEKPEESSPK; DSDIDSESVFK; HETFKRVLKLR; QNFVEKIESEK; ETFKRVLKLRE; RYAGDTILGVR; TSGTESLRKLK; APAVVKSESKR; RVSKSHSSKVK; YPLASSKMRFK; NWSVLLTPSAR; VAPAVVKSESK; KAHETFKRVLK; VYETSGTESLR; SKVKNFIFYVK |
| | HLA-B0702 | FPSYSQSSA; IPNISSRII; TPSARLQAY; MKRKAKSIL; VPNYPLASS; DPSSTRLDL; APAVVKSES; RVLYDKLSV; IPFYSGESG; KPEDMVVDL; RAEPGELVL; LARDPSSTRL; YPLASSKMRF; RVSKSHSSKV; TPSARLQAYV; KAHETFKRVL; SKRYAGDTIL; APAVVKSESK; YAYSMGTLKF; FPSYSQSSAMI; VPNYPLASSKM; KVYETSGTESL; RVLYDKLSVSI; RVLKLREKISI; LASSKMRFKAF; YVKNSVVAPAV |
| | HLA-B0801 | SSKMRFKAF; EIKVSVYSL; QAYVKNSVV; FPSYSQSSAM; VLKLREKISI; LRKLKAHETF; SLRKLKAHETF; LASSKMRFKAF; FKRVLKLREKI; VLKLREKISIA |
| | HLA-B1501 | YVKDLRVLY; YSGESGNQF; IMPPFKIPF; KVSVYSLGY; GVRVLFPSY; SSAMIMPPF; SSKMRFKAF; LTNYVDYVY; IIKDDVPNY; DMNGMEYAY; VVKSESKRY; GLIDNIKTM; SSKVKNFIF; VLFEDMNGM; LQAYVKNSV; KISIAEGSF; YSQSSAMIM; STRLDLTNY; DLIWSNPNY; YEIDLEVLF; FFLLSTVLF; TMKEIKVSVY; QSSAMIMPPF; MIMPPFKIPF; SIAEGSFQNF; IMPPFKIPFY; YAYSMGTLKF; RIIKDDVPNY; LQAYVKNSVV; SSKVKNFIFY; LFFLLSTVLF; ILFFLLSTVL; ASSKMRFKAF; LTPSARLQAY; LGVRVLFPSY; YVKNSVVAPA; AVVKSESKRY; TMKEIKVSVY; QSSAMIMPPF; MIMPPFKIPF; SIAEGSFQNF; IMPPFKIPFY; YAYSMGTLKF; RIIKDDVPNY; LQAYVKNSVV; SSKVKNFIFY; LFFLLSTVLF; ILFFLLSTVL; ASSKMRFKAF; LTPSARLQAY; LGVRVLFPSY; YVKNSVVAPA; AVVKSESKRY |
| | HLA-B2705 | KRKAKSILF; KRVLKLREK; KRYAGDTIL; RKAKSILFF; RKLKAHETF; ARLQAYVKN; MRFKAFRVSK; FRVSKSHSSK; KRKAKSILFF; KRVLKLREKI; KRAEPGELVL; RKLKAHETFK; KRYAGDTILGV; KRKAKSILFFL; TRLDLTNYVDY; FRVSKSHSSKV; SRIIKDDVPNY; ARLQAYVKNSV; RKLKAHETFKR |
| | HLA-B3501 | TPSARLQAY; MPPFKIPFY; DMNGMEYAY; LTNYVDYVY; EPGELVLDF; FFLLSTVLF; IAEGSFQNF; FPSYSQSSA; NPNYIPNIS; YVKDLRVLY; YSGESGNQF; YSQSSAMIM; SSAMIMPPF; FEDMNGMEY; KVSVYSLGY; DLIWSNPNY; ELVLDFAEL; YEIDLEVLF; LFFLLSTVL; NGMEYAYSM; KPEDMVVDL; FAELARDPS; DSESVFKVY; RAEPGELVL; FKGWADLIW; PSYSQSSAM; IPNISSRII; FPSYSQSSAM; YPLASSKMRF; YAYSMGTLKF; LFEDMNGMEY; EDMNGMEYAY; MIMPPFKIPF; EVLFEDMNGM; QSSAMIMPPF; LFFLLSTVLF; DLTNYVDYVY; MVVDLGINNW; LTPSARLQAY; NPNYIPNISS; |

| | | |
|---|---|---|
| | | FYSGESGNQF; LGVRVLFPSY; FYVKDLRVLY; FAQETDGLAE; DSDIDSESVF; SIAEGSFQNF; FPSYSQSSAMI; WADLIWSNPNY; VPNYPLASSKM; FEDMNGMEYAY; LASSKMRFKAF; LFPSYSQSSAM; DMNGMEYAYSM; MIMPPFKIPFY; VLFEDMNGMEY; LLTPSARLQAY; LDLTNYVDYVY; EPGELVLDFAE; DIDSESVFKVY; RAEPGELVLDF; SQSSAMIMPPF; WSVLLTPSARL; IDSDIDSESVF; LGYEIDLEVLF; TVLFAQETDGL |
| | HLA-B4403 | YEIDLEVLF; KEIKVSVYS; AELARDPSS; AEGSFQNFV; GELVLDFAE; KEIKVSVYSL; AEPGELVLDF; GELVLDFAEL; YEIDLEVLFE; REKISIAEGSF; KEIKVSVYSLG; GELVLDFAELA; YEIDLEVLFED |
| | HLA-B5101 | IPNISSRII; FPSYSQSSA; IPFYSGESG; MPPFKIPFY; EPGELVLDF; MPPFKIPFYS; FPSYSQSSAM; YPLASSKMRF; EPGELVLDFA; TPSARLQAYV; FPSYSQSSAMI; MPPFKIPFYSG; KPEDMVVDLGI; VPNYPLASSKM |
| | HLA-B5701 | YSMGTLKFKGW; KTMKEIKVSVY |
| BAD18055.1; FlaB protein [Borrelia garinii]; Seq15 | HLA-A0101 | NQDEAIAVNIY; ELAVQSGNGTY |
| | HLA-A0201 | SQASWTLRV; QLTDEINRI; AQAAQTAPV; AIAVNIYAA; SLAKIENAI; AVNIYAANV; AQYNQMHML; TTVDANTSL; SQGGVNSPV; QTAPVQEGV; NIYAANVANL; NLNEVEKVLV; VLVRMKELAV; QLTDEINRIA; NLFSGEGAQA; IAVNIYAANV; SLSGSQASWTL; MLSNKSASQNV; AIAVNIYAANV; SQASWTLRVHV; GMQPAKINTPA; KVLVRMKELAV; SLAKIENAIRM; NLFSGEGAQAA |
| | HLA-A0301 | NQMHMLSNK; GSQASWTLR; TVDANTSLAK; YNQMHMLSNK; LSGSQASWTLR; TTVDANTSLAK; LSNKSASQNVR |
| | HLA-A1101 | NQMHMLSNK; GSQASWTLR; AVQSGNGTY; TVDANTSLAK; YNQMHMLSNK; EVEKVLVRMK; TTVDANTSLAK; TTEGNLNEVEK; TAEELGMQPAK; TSLAKIENAIR; QYNQMHMLSNK; LSGSQASWTLR; LSNKSASQNVR |
| | HLA-A2402 | IYAANVANL; YAANVANLF; IYAANVANLF; TYSDADRGSI; IYAANVANLFS |
| | HLA-A2902 | AVQSGNGTY; YAANVANLF; ; ELAVQSGNGTY; EINRIADQAQY |
| | HLA-A6801 | LAKIENAIR; EGNLNEVEK; YAANVANLF; NGTYSDADR; GSQASWTLR; NQMHMLSNK; TSKAINFIQ; NKSASQNVR; EVEKVLVRMK; SLAKIENAIR; TVDANTSLAK; NTSKAINFIQ; SGSQASWTLR; TTVDANTSLA; TTVDANTSLAK; TTEGNLNEVEK; EIEQLTDEINR; LSNKSASQNVR; TSLAKIENAIR; EINRIADQAQY; NLNEVEKVLVR; LSGSQASWTLR; TAEELGMQPAK; QYNQMHMLSNK; NIYAANVANLF; QTAPVQEGVQQ |
| | HLA-B0702 | SPVNVTTTV; QPAKINTPA; QPAPATAPS; LVRMKELAV; TPASLSGSQ; APSQGGVNS; APATAPSQG; APVQEGVQQ; TPASLSGSQA; SPVNVTTTVD; APSQGGVNSP; QPAKINTPAS; APSQGGVNSPV; QPAKINTPASL; APATAPSQGGV; SPVNVTTTVDA; TPASLSGSQAS |
| | HLA-B0801 | KVLVRMKEL; VEKVLVRMKEL |
| | HLA-B1501 | YAANVANLF; AVQSGNGTY; AQAAQTAPV; AQYNQMHML; SQGGVNSPV; SLSGSQASW; SQASWTLRV; IQIEIEQLT; LAVQSGNGTY; SQASWTLRVH; SQNVRTAEEL; VQQEGAQQPA; INRIADQAQY; AQAAQTAPVQ; MQPAKINTPA; ELAVQSGNGTY; NIYAANVANLF; NQDEAIAVNIY; SLSGSQASWTL |
| | HLA-B2705 | NRIADQAQY; |
| | HLA-B3501 | YAANVANLF; QPAPATAPS; SPVNVTTTV; DEAIAVNIY; TTVDANTSL; LAVQSGNGT; QPAKINTPA; AVQSGNGTY; QASWTLRVH; IAVNIYAAN; LAVQSGNGTY; IADQAQYNQM; QPAKINTPAS; LAKIENAIRM; QPAPATAPSQ; YAANVANLFS; TAEELGMQPA; EAIAVNIYAA; QAQYNQMHML; TPASLSGSQA; SPVNVTTTVD; TPASLSGSQAS; ELAVQSGNGTY; NQDEAIAVNIY; QPAKINTPASL; IAVNIYAANVA; |

| | | |
|---|---|---|
| | | NIYAANVANLF; SPVNVTTTVDA; EINRIADQAQY; IADQAQYNQMH; APSQGGVNSPV; LAVQSGNGTYS |
| | HLA-B4403 | EELGMQPAKI; NEVEKVLVRM; DEINRIADQA; DEINRIADQAQ |
| | HLA-B5101 | SPVNVTTTV; APATAPSQGGV; APSQGGVNSPV |
| | HLA-B5701 | ASLSGSQASW; |
| AAU07005.1\| flagellar filament 41 kDa core protein [Borrelia garinii PBi]; Seq16 | HLA-A0101 | KTQEKLSSGY; MTDEVVASTT; ASDDAAGMGV; NQDEAIAVNIY; ISDQRANLGAF; RLESIKDSTEY; ELAVQSGNGTY; MIAQANQVPQY |
| | HLA-A0201 | SQASWTLRV; QLTDEINRI; SQAAQTAPV; AMIAQANQV; MIINHNTSA; QVPQYVLSL; AIAVNIYAA; SLAKIENAI; AVNIYAANV; AQYNQMHML; MTDEVVAST; SIKDSTEYA; TTVDANTSL; NLGAFQNRL; SQGGVNSPV; SAMAMIAQA; VVASTTNSI; MISDQRANL; ILTQSAMAM; SILTQSAMA; LTQSAMAMI; TMTDEVVAST; AQANQVPQYV; MIINHNTSAI; ILTQSAMAMI; NIYAANVANL; RMISDQRANL; NLNEVEKVLV; VLVRMKELAV; MAMIAQANQV; QLTDEINRIA; QVPQYVLSLL; NQVPQYVLSL; IAVNIYAANV; SLSGSQASWTL; MLSNKSASQNV; AMAMIAQANQV; AIAVNIYAANV; SQASWTLRVHV; KLSSGYRINRA; SILTQSAMAMI; GMQPAKINTPA; KVLVRMKELAV; MISDQRANLGA; NLFSGEGSQAA; YAIENLKASYA; SLAKIENAIRM; TMTDEVVASTT; ILTQSAMAMIA |
| | HLA-A0301 | SINAANLSK; SQASRNTSK; NQMHMLSNK; GSQASWTLR; FQNRLESIK; LSSGYRINR; NSINAANLSK; TVDANTSLAK; LSQASRNTSK; KLSSGYRINR; NLKASYAQIK; AANLSKTQEK; STEYAIENLK; KTQEKLSSGY; YNQMHMLSNK; AFQNRLESIK; QIRGLSQASR; VPQYVLSLLR; GLSQASRNTSK; MIAQANQVPQY; LSGSQASWTLR; GAFQNRLESIK; KTQEKLSSGYR; TTVDANTSLAK; KINAQIRGLSQ; RLESIKDSTEY; LSNKSASQNVR |
| | HLA-A1101 | SINAANLSK; SQASRNTSK; NQMHMLSNK; GSQASWTLR; AAGMGVSGK; LSSGYRINR; NTSAINASR; TEYAIENLK; AVQSGNGTY; PQYVLSLLR; FQNRLESIK; AQANQVPQY; TVDANTSLAK; STEYAIENLK; LSQASRNTSK; NSINAANLSK; AANLSKTQEK; RANLGAFQNR; KTQEKLSSGY; KLSSGYRINR; AFQNRLESIK; YNQMHMLSNK; IAQANQVPQY; TQEKLSSGYR; NLKASYAQIK; EVEKVLVRMK; TTVDANTSLAK; TTEGNLNEVEK; KTQEKLSSGYR; GAFQNRLESIK; TAEELGMQPAK; GLSQASRNTSK; TSLAKIENAIR; QYNQMHMLSNK; NAANLSKTQEK; QVPQYVLSLLR; AQIRGLSQASR; DSTEYAIENLK; LSGSQASWTLR; GVSGKINAQIR; MIAQANQVPQY; LSNKSASQNVR; SAMAMIAQANQ |
| | HLA-A2402 | IYAANVANL; YAANVANLF; AFQNRLESI; IYAANVANLF; TYSDADRGSI; SYAQIKDATM; IYAANVANLFS |
| | HLA-A2902 | ESIKDSTEY; AVQSGNGTY; YAANVANLF; AIENLKASY; YAIENLKASY; IAQANQVPQY; EYAIENLKASY; ELAVQSGNGTY; EINRIADQAQY; MIAQANQVPQY; RLESIKDSTEY |
| | HLA-A6801 | NTSAINASR; LSSGYRINR; TEYAIENLK; SINAANLSK; ESIKDSTEY; AIRMISDQR; LAKIENAIR; EGNLNEVEK; YAANVANLF; NGTYSDADR; GSQASWTLR; NQMHMLSNK; TSKAINFIQ; MAMIAQANQ; NKSASQNVR; TTNSILTQS; SQASRNTSK; NAIRMISDQR; STEYAIENLK; NSINAANLSK; DAAGMGVSGK; HNTSAINASR; EVEKVLVRMK; SLAKIENAIR; TVDANTSLAK; RANLGAFQNR; QIRGLSQASR; NTSKAINFIQ; LSQASRNTSK; NLKASYAQIK; SGSQASWTLR; TQEKLSSGYR; VPQYVLSLLR; AANLSKTQEK; YAIENLKASY; VSGKINAQIR; TTVDANTSLA; KLSSGYRINR; MIAQANQVPQ; NTSAINASRN; DSTEYAIENLK; TTVDANTSLAK; TTEGNLNEVEK; EIEQLTDEINR; QVPQYVLSLLR; NAANLSKTQEK; ENAIRMISDQR; LSNKSASQNVR; MIAQANQVPQY; TSLAKIENAIR; EINRIADQAQY; NLNEVEKVLVR; LSGSQASWTLR; GAFQNRLESIK; |

| | | |
|---|---|---|
| | | TAEELGMQPAK; QYNQMHMLSNK; GVSGKINAQIR; KTQEKLSSGYR; NHNTSAINASR; NIYAANVANLF; EKLSSGYRINR; NNSINAANLSK; QTAPVQEGAQQ; ENLKASYAQIK |
| | HLA-B0702 | SPVNVTTTV; VPQYVLSLL; QPAKINTPA; VVASTTNSI; QPAPATAPS; LVRMKELAV; ASRNTSKAI; IINHNTSAI; TPASLSGSQ; APSQGGVNS; APVQEGAQQ; APATAPSQG; VASTTNSIL; ILTQSAMAM; TPASLSGSQA; VVASTTNSIL; SPVNVTTTVD; APSQGGVNSP; QPAKINTPAS; QASRNTSKAI; MIINHNTSAI; APSQGGVNSPV; QPAKINTPASL; APATAPSQGGV; SPVNVTTTVDA; TPASLSGSQAS; SAINASRNNSI; AANLSKTQEKL; RANLGAFQNRL |
| | HLA-B0801 | YAQIKDATM; KVLVRMKEL; ; VEKVLVRMKEL |
| | HLA-B1501 | AQANVPQY; DQRANLGAF; YAANVANLF; SQAAQTAPV; AVQSGNGTY; TQEKLSSGY; ILTQSAMAM; AQYNQMHML; SQGGVNSPV; ESIKDSTEY; SLSGSQASW; SQASWTLRV; IQIEIEQLT; AMIAQANQV; YAIENLKASY; LAVQSGNGTY; SQASWTLRVH; KTQEKLSSGY; SQNVRTAEEL; RMISDQRANL; IAQANVPQY; AQQEGAQQPA; AQIRGLSQAS; NQVPQYVLSL; SQAAQTAPVQ; INRIADQAQY; MQPAKINTPA; SQASRNTSKAI; AQANVPQYVL; MIAQANQVPQY; ASRNTSKAINF; ELAVQSGNGTY; NIYAANVANLF; NQDEAIAVNIY; RLESIKDSTEY; SLSGSQASWTL |
| | HLA-B2705 | NRIADQAQY; IRGLSQASR; QRANLGAFQ; SRNTSKAINF; QRANLGAFQNR; SRNNSINAANL; IRMISDQRANL |
| | HLA-B3501 | YAANVANLF; YAQIKDATM; QPAPATAPS; RASDDAAGM; ESIKDSTEY; SPVNVTTTV; ILTQSAMAM; DEAIAVNIY; TTVDANTSL; LAVQSGNGT; QPAKINTPA; NSILTQSAM; YAIENLKAS; AVQSGNGTY; QASWTLRVH; VASTTNSIL; IAVNIYAAN; FSGEGSQAA; VPQYVLSLL; LAVQSGNGTY; YAIENLKASY; IADQAYNQM; SILTQSAMAM; IAQANVPQY; QPAKINTPAS; LAKIENAIRM; QANQVPQYVL; QPAPATAPSQ; YAANVANLFS; TAEELGMQPA; MIINHNTSAI; EAIAVNIYAA; NRASDDAAGM; QAAYNQMHML; TPASLSGSQA; SPVNVTTTVD; SYAQIKDATM; MAMIAQANQV; MIAQANQVPQY; TPASLSGSQAS; ELAVQSGNGTY; NQDEAIAVNIY; QPAKINTPASL; IAVNIYAANVA; NSILTQSAMAM; NIYAANVANLF; TTNSILTQSAM; EVVASTTNSIL; SPVNVTTTVDA; EINRIADQAQY; IADQAYNQMH; NASRNNSINAA; ISDQRANLGAF; APSQGGVNSPV; LAVQSGNGTYS; EYAIENLKASY |
| | HLA-B4403 | EELGMQPAKI; NEVEKVLVRM; DEINRIADQA; DEINRIADQAQ |
| | HLA-B5101 | SPVNVTTTV; VPQYVLSLL; APATAPSQGGV; APSQGGVNSPV |
| | HLA-B5701 | ASLSGSQASW; |
| 1L8W\|A Chain A, Crystal Structure Of Lyme Disease Variable Surface Antigen VlsE1 Of Borrelia Burgdorferi; Seq17 | HLA-A0101 | KSDVKTYFTTV |
| | HLA-A0201 | ILSAIVTAA; KVLGAITGL; LLDKLVKAV; QILSAIVTA; VLGAITGLI; ELLDKLVKA; QLGNGFLDV; KAAGAVSAV; KLVKAVKTA; KTYFTTVAA; FLDVFTSFG; AIGEVVADA; GTAAIGEVV; QILSAIVTAA; KVLGAITGLI; VVADADAAKV; LIGDAVSSGL; KLFGKAGAAA; ELLDKLVKAV; GIAKGIKEIV; AITGLIGDAV; IQLGNGFLDV; AVSGEQILSA; FLDVFTSFGG; FLDVFTSFGGL; GLIGDAVSSGL; ILSAIVTAADA; KSDVKTYFTTV; FTSFGGLVAEA; KTYFTTVAAKL |
| | HLA-A0301 | KLKAVAAAK; KVADKASVK; LVAEAFGFK; AIALRGXAK; KTDLNSLPK; GAAESAVRK; QIAAAIALR; KASVKGIAK; TTVAAKLEK; KGAGKLFGK; KETPPALNK; GTAVEGAIK; TYFTTVAAK; KTYFTTVAAK; GLVAEAFGFK; AAIALRGXAK; FTTVAAKLEK; ALRGXAKDGK; IVEAAGGSEK; KSDISSTTGK; LLDKLVKAVK; SVKGIAKGIK; GLRKVGDSVK; XAKDGKFAVK; TKTDLNSLPK; EAFGFKSDPK; GDAVSSGLRK; KSDPKKSDVK; TVAAKLEKTK; KGAAESAVRK; KVGDSVKAASK; KTKTDLNSLPK; KLFGKAGAAAH; ASKETPPALNK; AVAAAKGENNK; KTDLNSLPKEK; AIKEVSELLDK; ELLDKLVKAVK; |

| | | |
|---|---|---|
| | | AAHGDSEAASK; GXAKDGKFAVK |
| | HLA-A1101 | TTVAAKLEK; KTDLNSLPK; LVAEAFGFK; KVADKASVK; AIALRGXAK; GAAESAVRK; VVADADAAK; KASVKGIAK; KLKAVAAAK; GTAVEGAIK; TYFTTVAAK; AAAKGENNK; QIAAAIALR; AAGGSEKLK; AVKDGEKEK; KGAGKLFGK; AFGFKSDPK; DAVSSGLRK; VAAKLEKTK; PIAAAIGDK; AVSAVSGEQ; SDISSTTGK; KTYFTTVAA; KETPPALNK; KTYFTTVAAK; AAIALRGXAK; GLVAEAFGFK; TVAAKLEKTK; KSDISSTTGK; IVEAAGGSEK; SVKGIAKGIK; VAAAKGENNK; FTTVAAKLEK; EAFGFKSDPK; VSELLDKLVK; AADAAEQDGK; XAKDGKFAVK; KSDPKKSDVK; EVVADADAAK; VADKDDPTNK; KGAAESAVRK; TKTDLNSLPK; AFGFKSDPKK; GAEFGQDEXK; AVAAAKGENNK; KTKTDLNSLPK; ASKETPPALNK; TTVAAKLEKTK; KVGDSVKAASK; KTDLNSLPKEK; SVGTAVEGAIK; QVADKDDPTNK; AAAIALRGXAK; TAADAAEQDGK; AIKEVSELLDK; AAHGDSEAASK; ASVKGIAKGIK; EVSELLDKLVK; EAFGFKSDPKK; IALRGXAKDGK; AADAAEQDGKK; AAKVADKASVK; GXAKDGKFAVK; GAEFGQDEXKK; GGLVAEAFGFK; ELLDKLVKAVK; LIGDAVSSGLR |
| | HLA-A2402 | GFLDVFTSF; TYFTTVAAK; YFTTVAAKL; KFYQSVIQL; FYQSVIQLG; TYFTTVAAKL; SFGGLVAEAF; IQLGNGFLDVF |
| | HLA-A2902 | GFLDVFTSF; SVIQLGNGF; ; |
| | HLA-A6801 | TTVAAKLEK; QIAAAIALR; LVAEAFGFK; TYFTTVAAK; VVADADAAK; DAAEQDGKK; DAVSSGLRK; GAAESAVRK; KVADKASVK; FGFKSDPKK; DLNSLPKEK; SDISSTTGK; KLKAVAAAK; GTAVEGAIK; AAAKGENNK; FTTVAAKLEK; EVVADADAAK; EAFGFKSDPK; EAAGGSEKLK; KTYFTTVAAK; DQIAAAIALR; TVAAKLEKTK; SVKGIAKGIK; IVEAAGGSEK; FAVKDGEKEK; NPIAAAIGDK; GLVAEAFGFK; XAKDGKFAVK; VAAAKGENNK; AAIALRGXAK; DADAAKVADK; EAFGFKSDPKK; TTVAAKLEKTK; EIVEAAGGSEK; TAADAAEQDGK; EVSELLDKLVK; YFTTVAAKLEK; LIGDAVSSGLR; AVAAAKGENNK; QVADKDDPTNK; EKSDISSTTGK; ELLDKLVKAVK; AIKGAAESAVR; DDQIAAAIALR; SVGTAVEGAIK; AAHGDSEAASK |
| | HLA-B0702 | AVRKVLGAI; KPEEAKNPI; AASKAAGAV; GASSGTAAI; KAAGAVSAV; KPDSTGSVG; AASKETPPAL; AAESAVRKVL; AVRKVLGAIT; SAVRKVLGAI; KPDSTGSVGT; KPEEAKNPIA; KPEEAKNPIAA; KAASKETPPAL; KPDSTGSVGTA; GAAESAVRKVL; LPKEKSDISST; AVSGEQILSAI; AVSAVSGEQIL; AASKAAGAVSA |
| | HLA-B0801 | ; ; |
| | HLA-B1501 | SVIQLGNGF; DQIAAAIAL; GLVAEAFGF; FGKAGAAAH; QLGNGFLDVF; QSVIQLGNGF; YQSVIQLGNGF; IQLGNGFLDVF; TSFGGLVAEAF; KLFGKAGAAAH; ALRGXAKDGKF; GLIGDAVSSGL; AIGDKDGGAEF |
| | HLA-B2705 | RKVLGAITGL; |
| | HLA-B3501 | SVIQLGNGF; SAVSGEQIL; FGGLVAEAF; EAAGGSEKL; KPEEAKNPI; EVVADADAA; LGNGFLDVF; TAAIGEVVA; DPKKSDVKTY; SFGGLVAEAF; AASKETPPAL; QSVIQLGNGF; QLGNGFLDVF; SAIVTAADAA; TSFGGLVAEAF; DPKKSDVKTYF; TAEGASSGTAA; VADKDDPTNKF; KPEEAKNPIAA; YQSVIQLGNGF; KAASKETPPAL; TAVEGAIKEVS |
| | HLA-B4403 | EEAKNPIAA; GEVVADADA; AEFGQDEXK; AEGAIKGAA; KEIVEAAGGS; EEAKNPIAAA; GEVVADADAA; EEAKNPIAAAI; GENNKGAGKLF; AEGASSGTAAI; KEVSELLDKLV; AEGAIKGAAES; SELLDKLVKAV; AEFGQDEXKKD |
| | HLA-B5101 | KPEEAKNPI; LPKEKSDISS; LPKEKSDISST |
| | HLA-B5701 | |
| CAA57807.1; Associated | HLA-A0101 | LSANIEENY; YSEDKANTV; FLDALEAIEY; NSDKIDYAEKY; RLDNYDFKNEY; STDSIFLSEDM |

| protein A (BapA)[Borrelia burgdorferi]; Seq18 | HLA-A0201 | FLSEDMIRL; FLFLFVVSL; LLIFLFLFV; FLFVVSLSA; SIFNYLIQL; LIFLFLFVV; YLIKIKIST; KISTDSIFL; ALEAIEYLI; FVVSLSANI; ILKQILADL; LIGSYPDSI; KKISLLIFL; HVFSDAPRI; ISLLIFLFL; FLSEDMIRLI; SLLIFLFLFV; LLIFLFLFVV; RLIGSYPDSI; YLIQLNSDKI; KISLLIFLFL; FLFLFVVSLS; QLNSDKIDYA; QILKQILADL; SVFLDALEAI; FLDALEAIEYL; SLLIFLFLFVV; FLFVVSLSANI; FLFLFVVSLSA; LIFLFLFVVSL; ALEAIEYLIKI; ISLLIFLFLFV; SIFLSEDMIRL; SLSANIEENYT; KKISLLIFLFL |
|  | HLA-A0301 | YLIQLNSDK; KANTVKQILK; ALEAIEYLIK; YTETKRAFSK; NYLIQLNSDK; SLLIFLFLFV; APRIRGDLRK; NLINKRLDNY; ILKQILADLPK; FSKEDFNLINK; FNYLIQLNSDK |
|  | HLA-A1101 | KQILADLPK; AIEYLIKIK; YLIQLNSDK; TETKRAFSK; GDNARNNFK; KEDFNLINK; KANTVKQILK; YTETKRAFSK; GSYPDSIFNY; SIFLSEDMIR; ALEAIEYLIK; KSHVFSDAPR; HVFSDAPRIR; NSDKIDYAEK; YSEDKANTVK; EAIEYLIKIK; SLSANIEENY; NIEENYTETK; SKEDFNLINK; ILKQILADLPK; FSKEDFNLINK; KYGDNARNNFK |
|  | HLA-A2402 | SYPDSIFNY; KISLLIFLF; NYTETKRAF; VFLDALEAI; SLLIFLFLF; SYPDSIFNYL; KYGDNARNNF; ISLLIFLFLF; IFLFLFVVSL; AFSKEDFNLI; KKISLLIFLF; LFVVSLSANI; IFLSEDMIRL; SYPDSIFNYLI; NYLIQLNSDKI; IFLSEDMIRLI; KISLLIFLFLF; KYGDNARNNFK |
|  | HLA-A2902 | SYPDSIFNY; DMIRLIGSY; KISLLIFLF; SLLIFLFLF; QLNSDKIDY; FLDALEAIEY; GSYPDSIFNY; SLSANIEENY; NLINKRLDNY; VFLDALEAIEY; KISLLIFLFLF; RLDNYDFKNEY; IGSYPDSIFNY; VSLSANIEENY |
|  | HLA-A6801 | YLIQLNSDK; EDFNLINKR; DNARNNFKK; YDFKNEYEK; EENYTETKR; LSANIEENY; SHVFSDAPR; TETKRAFSK; IFLSEDMIR; DNYDFKNEY; HVFSDAPRIR; SIFLSEDMIR; EAIEYLIKIK; YTETKRAFSK; DAPRIRGDLR; YAEKYGDNAR; NIEENYTETK; NSDKIDYAEK; KSHVFSDAPR; YSEDKANTVK; KANTVKQILK; NYDFKNEYEK; GSYPDSIFNY; DSIFLSEDMIR; DNYDFKNEYEK; NIEENYTETKR; NYTETKRAFSK; FNYLIQLNSDK; EKSHVFSDAPR; DYAEKYGDNAR; FSKEDFNLINK; DAPRIRGDLRK; DALEAIEYLIK; DYSEDKANTVK; NNFKKDYSEDK; ILKQILADLPK |
|  | HLA-B0702 | YPDSIFNYL; APRIRGDLR; APRIRGDLRK; YPDSIFNYLI; APRIRGDLRKI; RIRGDLRKIGI |
|  | HLA-B0801 |  |
|  | HLA-B1501 | DMIRLIGSY; FLFLFVVSL; LSANIEENY; IGSYPDSIF; KISLLIFLF; NEYEKSHVF; QLNSDKIDY; IQLNSDKIDY; GSYPDSIFNY; SLSANIEENY; KIKISTDSIF; FLDALEAIEY; RLIGSYPDSI; KQILKQILADL; FKNEYEKSHVF; IGSYPDSIFNY |
|  | HLA-B2705 | KRLDNYDFK; KQILADLPK; KRAFSKEDF; ARNNFKKDY; RKIGIKEKSVF; KRAFSKEDFNL; KQILKQILADL |
|  | HLA-B3501 | YPDSIFNYL; DALEAIEYL; LDALEAIEY; LSANIEENY; NEYEKSHVF; YPDSIFNYLI; FLDALEAIEY; LDNYDFKNEY; YAEKYGDNAR; VFLDALEAIEY; FKNEYEKSHVF; YPDSIFNYLIQ; RLDNYDFKNEY |
|  | HLA-B4403 | NEYEKSHVF; SEDMIRLIGS; EENYTETKRAF; SEDMIRLIGSY; SEDKANTVKQI |
|  | HLA-B5101 | EAIEYLIKI; YPDSIFNYL; YPDSIFNYLI; EAIEYLIKIKI |
|  | HLA-B5701 | GSYPDSIFNY; VSLSANIEENY |
| AAL25643.1, P37-47 [Borrelia burgdorferi]; Seq19 | HLA-A0101 | SSNKDIYNSY; LSSNKDIYNSY |
|  | HLA-A0201 | IVYEILEEV; KIPEITEEV; NLSAIEESV; KLKEFLEKL; VIMPIPQTI; YIPEVKEEI; SLNLSTKSV; KINNGLNIV; IYNSYIPEV; FLTQGTSPS; NIDNITENL; FLLLNLVNC; SIAKLLQHL; ITEEVIMPI; KLSQVAQHA; NTWGEGLEI; CAFLLLNLV; FLEKLKKYL; FLTQGTSPSI; YLKDTNNLSA; |

| | | |
|---|---|---|
| | | ILEEVIIPET; YIPEVKEEIV; NLVNCKFDSL; IIPETKIPEI; DIYNSYIPEV; IVYEILEEVI; KIPEITEEVI; EIVYEILEEV; MCAFLLLNLV; YLKDTNNLSAI; VIIPETKIPEI; IVYEILEEVII; YIEPRPISSFL; FLTQGTSPSIT; KIPEITEEVIM; TIDFYIEPRPI |
| | HLA-A0301 | ITSTIKSYK; LLLNLVNCK; RSSIRTKIK; NLNSKETPK; KITHPIFDH; EVRSSIRTK; KTSTSEDQK; IIEDLQKLK; SITSTIKSYK; IAKLLQHLSK; TIKSYKELAK; FLLLNLVNCK; KLKEFLEKLK; LQKLKEFLEK; QVAQHAPNSK; KVSDGTEQTK; TSPSITSTIK; IYNSYIPEVK; KSYKELAKEK; FLEKLKKYLK; NLSAIEESVK; KFDSLNLSTK; NLSTKSVDDK; KIEKVKSDGK; INNGLNIVQK; QHAPNSKIEK; KINNGLNIVQK; SIAKLLQHLSK; KLKEFLEKLKK; HLSKSEDQANK; GTSPSITSTIK; AQHAPNSKIEK; STIKSYKELAK; ILEEVIIPETK; PSITSTIKSYK; KSDGKPVPGDK; DIYNSYIPEVK |
| | HLA-A1101 | ITSTIKSYK; KTSTSEDQK; VAQHAPNSK; LLLNLVNCK; RSSIRTKIK; TIDFYIEPR; IIEDLQKLK; NLNSKETPK; NITENLNSK; LSAIEESVK; HAPNSKIEK; ISGKEVEEK; VSDGTEQTK; LSTKSVDDK; SPSITSTIK; SITSTIKSY; AKLLQHLSK; SITSTIKSYK; KSYKELAKEK; QVAQHAPNSK; KVSDGTEQTK; TSPSITSTIK; TIKSYKELAK; QTIDFYIEPR; IAKLLQHLSK; FLLLNLVNCK; SSNKDIYNSY; LQKLKEFLEK; KLKEFLEKLK; KIEKVKSDGK; KFDSLNLSTK; NLSAIEESVK; IYNSYIPEVK; EIIEDLQKLK; LSKSEDQANK; FLEKLKKYLK; SIAKLLQHLSK; STIKSYKELAK; KINNGLNIVQK; SVDDKNNSIAK; GTSPSITSTIK; AQHAPNSKIEK; SQVAQHAPNSK; PSITSTIKSYK; AFLLLNLVNCK; KSDGKPVPGDK; ITENKEQEHEK; KLKEFLEKLKK; DIYNSYIPEVK; HLSKSEDQANK; ILEEVIIPETK; DLQKLKEFLEK |
| | HLA-A2402 | KYLKDTNNL; VYEILEEVI; IYNSYIPEV; VIMPIPQTI; TSPSITSTI; SYIPEVKEEI; VYEILEEVII; SYKELAKEKI; IYNSYIPEVK; KYLKDTNNLS; FYIEPRPISSF; SFLTQGTSPSI; SYIPEVKEEIV |
| | HLA-A2902 | LLNLVNCKF; EFLEKLKKY; SSNKDIYNSY; IMPIPQTIDF; YIPEVKEEIVY; IMPIPQTIDFY; FYIEPRPISSF; FLLLNLVNCKF |
| | HLA-A6801 | ITSTIKSYK; ETPKEISGK; TIDFYIEPR; NITENLNSK; EVRSSIRTK; HAPNSKIEK; FFTNLEEVR; KTSTSEDQK; LSAIEESVK; LSTKSVDDK; DSNFFTNLE; YNSYIPEVK; EEVIIPETK; NLNSKETPK; ENKEQEHEK; IIEDLQKLK; QTIDFYIEPR; SITSTIKSYK; EIIEDLQKLK; EISGKEVEEK; QVAQHAPNSK; TSPSITSTIK; NFFTNLEEVR; NLSAIEESVK; NLEEVRSSIR; TIKSYKELAK; MPIPQTIDFY; IYNSYIPEVK; IAKLLQHLSK; FLEKLKKYLK; KVSDGTEQTK; FLLLNLVNCK; NLSTKSVDDK; KSYKELAKEK; LSKSEDQANK; INNGLNIVQK; DTNNLSAIEE; DSNFFTNLEE; SSNKDIYNSY; DIYNSYIPEVK; STIKSYKELAK; SNFFTNLEEVR; SIAKLLQHLSK; EIDEIIEDLQK; ITENKEQEHEK; EVRSSIRTKIK; HLSKSEDQANK; GTSPSITSTIK; HAPNSKIEKVK; PSITSTIKSYK; CKFDSLNLSTK; DLQKLKEFLEK; KINNGLNIVQK; ILEEVIIPETK; EFLEKLKKYLK |
| | HLA-B0702 | EPRPISSFL; KPVPGDKIL; RPISSFLTQ; MPIPQTIDF; APNSKIEKV; RPISSFLTQG; TPKEISGKEV; SPSITSTIKS; IPEITEEVIM; EVRSSIRTKI; EPRPISSFLT; RPISSFLTQGT; SPSITSTIKSY; KPVPGDKILSS; MPIPQTIDFYI; HPIFDHITGSG |
| | HLA-B0801 | FLEKLKKYL; ; YLKDTNNLSAI |
| | HLA-B1501 | SNKDIYNSY; SITSTIKSY; LLNLVNCKF; AQHAPNSKI; LEIGGDSNF; SSNKDIYNSY; YIEPRPISSF; IMPIPQTIDF; PSITSTIKSY; LLLNLVNCKF; YLKDTNNLSA; LEIGGDSNFF; LSSNKDIYNSY; YLKDTNNLSAI; IMPIPQTIDFY; FYIEPRPISSF; VIMPIPQTIDF; YIPEVKEEIVY; TQNIDNITENL; FLLLNLVNCKF |
| | HLA-B2705 | VRSSIRTKIK; |
| | HLA-B3501 | MPIPQTIDF; KPVPGDKIL; EPRPISSFL; EIGGDSNFF; ILSSNKDIY; |

| | | |
|---|---|---|
| | | IPEITEEVI; MPIPQTIDFY; IPEVKEEIVY; IPEITEEVIM; LEIGGDSNFF; SPSITSTIKSY; NPGQDSISNTW; MPIPQTIDFYI; YIPEVKEEIVY; LSSNKDIYNSY; EVEEKITHPIF |
| | HLA-B4403 | EEVIMPIPQ; EEIVYEILE; EEKITHPIF; EEVRSSIRT; YEILEEVII; DEIIEDLQK; SEDQKELEI; PEITEEVIM; PEVKEEIVY; SEDQANKTS; LEIGGDSNF; EEVIMPIPQT; EEIVYEILEE; KEFLEKLKKY; EEVIIPETKI; EEKITHPIFD; VEIDEIIEDL; KEKINNGLNI; DEIIEDLQKL; EEVIMPIPQTI; KEFLEKLKKYL; EEIVYEILEEV; KEVEEKITHPI; EEVRSSIRTKI; PEITEEVIMPI; VEIDEIIEDLQ; YEILEEVIIPE |
| | HLA-B5101 | IPQTIDFYI; MPIPQTIDF; IPETKIPEI; IPEITEEVI; CAFLLLNLV; IPEVKEEIV; KPVPGDKIL; EPRPISSFL; MPIPQTIDFY; TPKEISGKEV; MPIPQTIDFYI |
| | HLA-B5701 | PGQDSISNTW; |
| NP_212481.1 \| fibronectin/fib rinogen- binding protein, putative [Borrelia burgdorferi B31]; Seq20 | HLA-A0101 | YSEFLENYY; HTRDYPGAY; KSGKADLYY; YTEINTLIK; MSDKKIMEL; LSPKENALQY; NSNFKILDAY; MSDKKIMELK; TSYSEFLENY; YTSYSEFLENY; NSNFKILDAYY; KTPKIGLHFTY; TSYSEFLENYY; SLSPKENALQY; WLHTRDYPGAY |
| | HLA-A0201 | FLENYYESL; KLWPSSPNI; LLGAGNLCV; KMSLNYTEI; TLIKEIPFT; FTYCGFEIL; ILQKDMIIL; FTNSLITKI; KLGLVIPKA; KADLYYTQV; ILFIKLWPS; LVLEIYNKI; VLEKRIDSL; LIQSKITML; TMLKVENLI; ILICLNPNT; IQPDYKSLV; LLNYKEEKI; SLSPKENAL; NLCVFYTKL; KTPSLDVLL; KMSDKKIMEL; KLWPSSPNII; VLLGAGNLCV; KLDENLIKKI; FTYCGFEILI; SLVLEIYNKI; NLIQSKITML; NLLNYKEEKI; SLNYTEINTL; IIATNSNFKI; IQSKITMLKV; WLHTRDYPGA; LQKDMIILFI; LLIEKYKKEL; IVLEKRIDSL; IIQPDYKSLV; FTNSLITKII; YTQVKNLRRV; QMKNERIISL; KILICLNPNT; ILFIKLWPSS; KLWPSSPNIIA; ILQKDMIILFI; FQMKNERIISL; ILDAYYRRPKI; LIQSKITMLKV; SLNYTEINTLI; LLIEKYKKELI; KIIQPDYKSLV; SLNDQIKKTNI; GLHFTYCGFEI; CLNPNTTRFHI; NIIATNSNFKI; LLNYKEEKIKI; FLKSKIQNGKI; KIDNKKFKILI; MSLNYTEINTL; LIKEIPFTNSL; FIKLWPSSPNI; SLDVLLGAGNL; QLKDNLDKFNL; NTSYTSYSEFL |
| | HLA-A0301 | KLRFSDFLK; IQSKITMLK; SLNQSLSPK; TTRFHITKK; LQYFKAYKK; IIATNSNFK; HITKKNFKK; KIIKAFQMK; VFYTKLAKK; CVFYTKLAK; LLNINKIQK; IISLEILQK; KLDENLIKK; KNFKKNALK; ALQYFKAYK; KIQNGKIIK; SLVLEIYNK; NLRRVKNKK; GLVIPKAEK; ELILLNINK; KENALQYFK; EIYNKIDNK; KSGKADLYY; NTTRFHITK; QVKNLRRVK; ETTGEIFLK; KTAIKEKEK; KILDAYYRR; AYYRRPKIK; LIQSKITMLK; ILLNINKIQK; ALQYFKAYKK; ISLNQSLSPK; CVFYTKLAKK; RIISLEILQK; ILDAYYRRPK; AYKKGKNSFK; NIIATNSNFK; LCVFYTKLAK; NALQYFKAYK; KSLVLEIYNK; KKNFKKNALK; GAYVFIKNQK; RFHITKKNFK; YTKLAKKSGK; IPFTNSLITK; KLLENIENEK; ITKIIQPDYK; NTTRFHITKK; RIDSLKQQIK; FLKSKIQNGK; KADLYYTQVK; AIKEKEKTPK; MSDKKIMELK; LLNYKEEKIK; AGNLCVFYTK; VLEKRIDSLK; YVFIKNQKNK; SFKTIQNQLK; GELILLNINK; LKLRFSDFLK; TTGEIFLKAK; GKIIKAFQMK; HIKLDENLIK; QYFKAYKKGK; NINKIQKGIK; EIYNKIDNKK; KETTGEIFLK; LGLVIPKAEK; KELLIEKYKK; GLHFTYCGFE; NYTEINTLIK; KNLRRVKNKK; KAYKKGKNSFK; KLRFSDFLKSK; KISLNQSLSPK; KLGLVIPKAEK; NLIQSKITMLK; ALKLRFSDFLK; KMSDKKIMELK; IVLEKRIDSLK; KTNIKELLIEK; NSFKTIQNQLK; KILDAYYRRPK; LQYFKAYKKGK; LILLNINKIQK; GIKEINLLNYK; RFHITKKNFKK; LLGAGNLCVFY; KSKIQNGKIIK; KTPKIGLHFTY; TSYSEFLENYY; HIKLDENLIKK; RDYPGAYVFIK; ILICLNPNTTR; LITKIIQPDYK; NLDKFNLIQSK; KLDENLIKKIK; SLSPKENALQY; QVKNLRRVKNK; SLITKIIQPDY; EIHESNKMSDK; NLCVFYTKLAK; GAGNLCVFYTK; LQKDMIILFIK; IQNQLKDNLDK |

| HLA-A1101 | SLNQSLSPK; IQSKITMLK; IIATNSNFK; ALQYFKAYK; CVFYTKLAK; SLVLEIYNK; KIIKAFQMK; LQYFKAYKK; NTTRFHITK; IISLEILQK; TTRFHITKK; ETTGEIFLK; KENALQYFK; KIQNGKIIK; KLRFSDFLK; KILDAYYRR; HITKKNFKK; YTEINTLIK; KLDENLIKK; ELILLNINK; KTAIKEKEK; EIYNKIDNK; KAEKNLHIK; NIKELLIEK; LLNINKIQK; GNLCVFYTK; YTQVKNLRR; VFYTKLAKK; KDMIILFIK; KAFQMKNER; RFSDFLKSK; KEINLLNYK; GLVIPKAEK; ESLNDQIKK; KKLGLVIPK; KIMELKEEY; ESNKMSDKK; KNFKKNALK; AYYRRPKIK; NLLNYKEEK; QVKNLRRVK; AYVFIKNQK; YPGAYVFIK; EILIGRNAK; NQLKDNLDK; GAGNLCVFY; KSGKADLYY; TSYSEFLEN; KSLVLEIYNK; RIISLEILQK; ISLNQSLSPK; LIQSKITMLK; NIIATNSNFK; TTGEIFLKAK; ALQYFKAYKK; CVFYTKLAKK; GAYVFIKNQK; TSYSEFLENY; MSDKKIMELK; NTTRFHITKK; YVFIKNQKNK; ITKIIQPDYK; AGNLCVFYTK; NALQYFKAYK; KLLENIENEK; KADLYYTQVK; ILLNINKIQK; KAKEIHESNK; RIDSLKQQIK; ILDAYYRRPK; AIKEKEKTPK; VLEKRIDSLK; EIYNKIDNKK; IPFTNSLITK; TQVKNLRRVK; TNIKELLIEK; YTKLAKKSGK; NINKIQKGIK; HIKLDENLIK; KETTGEIFLK; SFKTIQNQLK; DAYYRRPKIK; LICLNPNTTR; GIKEINLLNY; RFHITKKNFK; KTNIKELLIEK; KISLNQSLSPK; KAYKKGKNSFK; KILDAYYRRPK; KMSDKKIMELK; IVLEKRIDSLK; ALKLRFSDFLK; TSYSEFLENYY; GAGNLCVFYTK; NLIQSKITMLK; NSFKTIQNQLK; GIKEINLLNYK; TAIKEKEKTPK; LILLNINKIQK; KLGLVIPKAEK; NALQYFKAYKK; LITKIIQPDYK; YTQVKNLRRVK; LQYFKAYKKGK; NLCVFYTKLAK; LQKDMIILFIK; IQNQLKDNLDK; KSKIQNGKIIK; KLRFSDFLKSK; SPKENALQYFK; EINLLNYKEEK; KTPKIGLHFTY; ETTGEIFLKAK; QVKNLRRVKNK; HIKLDENLIKK; YTSYSEFLENY; NIKELLIEKYK; EIPFTNSLITK; RDYPGAYVFIK; NLDKFNLIQSK; VLEIYNKIDNK; ILICLNPNTTR; IIKAFQMKNER; KLDENLIKKIK; KGELILLNINK; RFHITKKNFKK; LIPEEEYNQEK; SNFKILDAYYR; SLITKIIQPDY; TYCGFEILIGR; TSYTSYSEFLE; SLSPKENALQY |
|---|---|
| HLA-A2402 | DYPGAYVFI; DYKSLVLEI; LWPSSPNII; SYTSYSEFL; YYESLNDQI; NYTEINTLI; TYCGFEILI; KYKKELIVL; LYYTQVKNL; TMLKVENLI; HFTYCGFEI; AYKKGKNSF; KTPKIGLHF; IYNKIDNKK; EYNQEKTAI; TSYTSYSEF; EYNNTSYTS; KFNLIQSKI; IYNKIDNKKF; DYPGAYVFIK; NYYESLNDQI; EFLENYYESL; EYNNTSYTSY; ITMLKVENLI; IIATNSNFKI; SYTSYSEFLE; NTSYTSYSEF; HFTYCGFEILI; NFKKNALKLRF; YYTQVKNLRRV; KYKKELIVLEK |
| HLA-A2902 | YSEFLENYY; YNNTSYTSY; NFKILDAYY; SYSEFLENY; PKIGLHFTY; GAGNLCVFY; KIMELKEEY; KSGKADLYY; KEEYNNTSY; EYNNTSYTSY; SYSEFLENYY; SNFKILDAYY; TSYSEFLENY; DYKSLVLEIY; LSPKENALQY; GIKEINLLNY; IYNKIDNKKF; ENALQYFKAY; NIKELLIEKY; KTPKIGLHFTY; TSYSEFLENYY; SLSPKENALQY; YTSYSEFLENY; ELKEEYNNTSY; SLITKIIQPDY; LLGAGNLCVFY; KVENLIPEEEY; WLHTRDYPGAY; EEYNNTSYTSY; NSNFKILDAYY; TNIKELLIEKY |
| HLA-A6801 | ETTGEIFLK; NTTRFHITK; YYTQVKNLR; CVFYTKLAK; IIATNSNFK; EIYNKIDNK; ESNKMSDKK; TTRFHITKK; ELILLNINK; HITKKNFKK; KAFQMKNER; YTQVKNLRR; FKILDAYYR; YTEINTLIK; YTSYSEFLE; SLVLEIYNK; IQSKITMLK; EILIGRNAK; ESLNDQIKK; NIKELLIEK; NLLNYKEEK; CGFEILIGR; LQYFKAYKK; IISLEILQK; YPGAYVFIK; LLENIENEK; ALQYFKAYK; NTSYTSYSE; KILDAYYRR; AYVFIKNQK; QVKNLRRVK; IYNKIDNKK; YSEFLENYY; SLNQSLSPK; LLNINKIQK; ELLIEKYKK; ENIENEKEK; KIIKAFQMK; KLRFSDFLK; TSYTSYSEF; DKFNLIQSK; NLRRVKNKK; KTAIKEKEK; ITKIIQPDY; NIIATNSNFK; NTTRFHITKK; CVFYTKLAKK; EIYNKIDNKK; YVFIKNQKNK; NALQYFKAYK; YTKLAKKSGK; NFKILDAYYR; ITKIIQPDYK; |

855

| | | |
|---|---|---|
| | | YYTQVKNLRR; DAYYRRPKIK; NAKENDKLLR; TTGEIFLKAK; LICLNPNTTR; LYYTQVKNLR; MSDKKIMELK; LIQSKITMLK; TSYSEFLENY; NINKIQKGIK; GAYVFIKNQK; NYTEINTLIK; FKILDAYYRR; RIISLEILQK; DYPGAYVFIK; IPFTNSLITK; EYNQEKTAIK; NFKKNALKLR; NTSYTSYSEF; HIKLDENLIK; IKAFQMKNER; FLKSKIQNGK; TSYTSYSEFL; TNIKELLIEK; SFKTIQNQLK; ILLNINKIQK; EYNNTSYTSY; QYFKAYKKGK; NSNFKILDAY; EKTAIKEKEK; ISLNQSLSPK; YYESLNDQIK; ALQYFKAYKK; KAKEIHESNK; DLYYTQVKNLR; ETTGEIFLKAK; EINLLNYKEEK; NSFKTIQNQLK; TYCGFEILIGR; IIKAFQMKNER; NLIQSKITMLK; SNFKILDAYYR; EIPFTNSLITK; ENALQYFKAYK; YTSYSEFLENY; TSYSEFLENYY; ILICLNPNTTR; EIHESNKMSDK; LITKIIQPDYK; NALQYFKAYKK; NFKILDAYYRR; TAIKEKEKTPK; NIKELLIEKYK; NLCVFYTKLAK; TSYTSYSEFLE; IVLEKRIDSLK; NPNTTRFHITK; HIKLDENLIKK; NYYESLNDQIK; YTQVKNLRRVK; LYYTQVKNLRR; NSNFKILDAYY; IYNKIDNKKFK; LILLNINKIQK; KAYKKGKNSFK; SPKENALQYFK; FYTKLAKKSGK; TRFHITKKNFK; NTSYTSYSEFL; GIKEINLLNYK; QVKNLRRVKNK; KTNIKELLIEK; EEYNQEKTAIK; LIPEEEYNQEK; YYESLNDQIKK; KISLNQSLSPK; EEYNNTSYTSY; LQYFKAYKKGK; ERIISLEILQK; LNYTEINTLIK; GAGNLCVFYTK; LLENIENEKEK; NLDKFNLIQSK; NGKIIKAFQMK |
| | HLA-B0702 | QPDYKSLVL; RVKNKKLGL; NPNTTRFHI; WPSSPNIIA; RPKIKETTG; IPFTNSLIT; WPSSPNIIAT; SPKENALQYF; IPKAEKNLHI; KAYKKGKNSF; IVLEKRIDSL; LAKKSGKADL; RPKIKETTGE; NPNTTRFHIT; RPKIKETTGEI; SPNIIATNSNF; RVKNKKLGLVI; IPFTNSLITKI; QPDYKSLVLEI; TPKIGLHFTYC |
| | HLA-B0801 | ALKLRFSDF; LRRVKNKKL; NLRRVKNKKL; QIKKTNIKEL; QMKNERIISL; ITKKNFKKNAL; FLKSKIQNGKI; RPKIKETTGEI; QIKKTNIKELL |
| | HLA-B1501 | LQKDMIILF; YNNTSYTSY; HTRDYPGAY; NQKNKTPSL; TSYTSYSEF; KIMELKEEY; ALKLRFSDF; NIIATNSNF; ITKIIQPDY; YSEFLENYY; GLHFTYCGF; RDYPGAYVF; KSGKADLYY; IQNGKIIKAF; KIKETTGEIF; LLGAGNLCVF; QMKNERIISL; KAYKKGKNSF; GIKEINLLNY; ILQKDMIILF; KMSDKKIMEL; IQKGIKEINL; IQPDYKSLVL; LQKDMIILFI; TSYSEFLENY; NTSYTSYSEF; WLHTRDYPGAY; LLRHCVKGNDY; FQMKNERIISL; ELKEEYNNTSY; LLGAGNLCVFY; VLLGAGNLCVF; HTRDYPGAYVF; KIQNGKIIKAF; SLSPKENALQY; YTSYSEFLENY; IQKGIKEINLL; SLITKIIQPDY; TSYSEFLENYY; LAKKSGKADLY; NQSLSPKENAL; KENALQYFKAY; KVENLIPEEEY; KTPKIGLHFTY; LIKEIPFTNSL; NSNFKILDAYY |
| | HLA-B2705 | KKLGLVIPK; KKNALKLRF; GRNAKENDK; LQYFKAYKK; RRVKNKKLGL; LRFSDFLKSK; TRFHITKKNF; KRIDSLKQQI; KRIDSLKQQIK; RRVKNKKLGLV; TRFHITKKNFK; LRFSDFLKSKI; LRRVKNKKLGL; FQMKNERIISL |
| | HLA-B3501 | NALQYFKAY; SPKENALQY; YSEFLENYY; QPDYKSLVL; IPFTNSLIT; WPSSPNIIA; HTRDYPGAY; FTYCGFEIL; YNNTSYTSY; NFKILDAYY; YKSLVLEIY; TSYTSYSEF; NLIQSKITM; TPKIGLHFTY; WPSSPNIIAT; SPKENALQYF; NSNFKILDAY; NPNTTRFHIT; LKEEYNNTSY; ENALQYFKAY; EYNNTSYTSY; NTSYTSYSEF; TPSLDVLLGA; TSYSEFLENY; FNLIQSKITM; SPNIIATNSNF; WLHTRDYPGAY; MSLNYTEINTL; TSYSEFLENYY; EINTLIKEIPF; YTSYSEFLENY; WPSSPNIIATN; EILQKDMIILF; LAKKSGKADLY; NSNFKILDAYY; TNSNFKILDAY; KVENLIPEEEY; HTRDYPGAYVF; TPSLDVLLGAG; EEYNNTSYTSY; VLLGAGNLCVF; LICLNPNTTRF; ELKEEYNNTSY; IPFTNSLITKI |
| | HLA-B4403 | KEIPFTNSL; SEFLENYYE; AEKNLHIKL; KEIHESNKM; KETTGEIFL; |

| | | |
|---|---|---|
| | | EEYNNTSYT; KEEYNNTSY; FEILIGRNA; SEFLENYYES; KEIPFTNSLI; KEIHESNKMS; GEIFLKAKEI; EEYNQEKTAI; TEINTLIKEI; LEILQKDMII; EEYNNTSYTS; AEKNLHIKLD; EEKIKISLNQ; SEFLENYYESL; KEIPFTNSLIT; KENALQYFKAY; EEYNNTSYTSY; KEKTPKIGLHF; KEKEKTPKIGL |
| | HLA-B5101 | IPFTNSLIT; WPSSPNIIA; NPNTTRFHI; IPFTNSLITK; IPKAEKNLHI; WPSSPNIIAT; IPFTNSLITKI; QPDYKSLVLEI |
| | HLA-B5701 | TSYSEFLENY; LSPKENALQY; TSYSEFLENYY; KTPKIGLHFTY |
| AAC44656.1\| P30 Borrelia burgdorferi; Seq21 | HLA-A0101 | TTNDSSTAY; ETSSDGTAY; DSSTAYKMY; ASKMIDTLY; ELDAILVPY; SSDGTAYPFY; YTTNDSSTAY; VASKMIDTLY; EVELEEITFY; TSSDGTAYPFY; NVASKMIDTLY; VTDKYGQNWTS |
| | HLA-A0201 | SLSLIIFSL; SLIIFSLTV; KLQRSLSLI; AIDSKTLEI; YVNEELDAI; LVHQSFIPV; HQSFIPVPV; MVTSGPFKL; YKMYVNEEL; ELEEITFYT; LTWSDGVAI; LIIFSLTVL; YLRDNLTWS; KMIDTLYRG; SSTAYKMYV; MIDTLYRGI; KMIDTLYRGI; LLVHQSFIPV; TLASPKPYFI; SLIIFSLTVL; KLQRSLSLII; RSLSLIIFSL; NMVTSGPFKL; YVNEELDAIL; SLSLIIFSLT; KMYVNEELDA; LSLIIFSLTV; MIDTLYRGIV; RAIDSKTLEI; SLSLIIFSLTV; YVNEELDAILV; ILNKETGSTYV; KMIDTLYRGIV; SLDPQLAEDNV; KMYVNEELDAI; FIDLLVHQSFI; LVHQSFIPVPV; AIDSKTLEITL; ITLASPKPYFI; LTWSDGVAITA; QLAEDNVASKM; DLLVHQSFIPV; SSDGTAYPFYL |
| | HLA-A0301 | KSYLRILNK; NMVTSGPFK; VTSGPFKLK; GTAYPFYLR; AITAEGIRK; TTNDSSTAY; GSTYVDMVK; GGHKPGLAK; RSLSLIIFS; TTNDSSTAYK; MVTSGPFKLK; KLKERIPSEK; TLEITLASPK; YVDMVKSIIK; RKSYLRILNK; VAITAEGIRK; SLTVLCCDNK; ILNKETGSTY; ENMVTSGPFK; ELGIRAIDSK; QLAEDNVASK; TGGHKPGLAK; SSDGTAYPFY; VLCCDNKERK; TGSTYVDMVK; KTLEITLASPK; NMVTSGPFKLK; GVAITAEGIRK; RIPSEKYVFEK; YTTNDSSTAYK; RILNKETGSTY; KLKERIPSEKY; VTGDPNTGGHK; NTGGHKPGLAK; NVASKMIDTLY; TSSDGTAYPFY; FSLTVLCCDNK; FIPVPVHVTDK |
| | HLA-A1101 | KSYLRILNK; GTAYPFYLR; VTSGPFKLK; AITAEGIRK; GSTYVDMVK; TTNDSSTAY; NMVTSGPFK; LTVLCCDNK; ITLASPKPY; PSEKYVFEK; KISLGAEPR; LAEDNVASK; TNDSSTAYK; ASKMIDTLY; VTDSELGIR; SIIKNGQEY; TTNDSSTAYK; MVTSGPFKLK; TLEITLASPK; VAITAEGIRK; YVDMVKSIIK; SLTVLCCDNK; KLKERIPSEK; ASKMIDTLYR; SSDGTAYPFY; QLAEDNVASK; VLCCDNKERK; TVLCCDNKER; TGSTYVDMVK; RKSYLRILNK; IPSEKYVFEK; TSGPFKLKER; QVTDSELGIR; GVAITAEGIR; KTLEITLASPK; GVAITAEGIRK; TVLCCDNKERK; YTTNDSSTAYK; RIPSEKYVFEK; VTGDPNTGGHK; TSSDGTAYPFY; NTGGHKPGLAK; FSLTVLCCDNK; NMVTSGPFKLK; VASKMIDTLYR; STKISLGAEPR; VTSGPFKLKER; ETGSTYVDMVK; TAEGIRKSYLR; TYVDMVKSIIK; NVASKMIDTLY |
| | HLA-A2402 | FYLRDNLTW; TYVDMVKSI; YYDSNEVEL; SFIPVPVHV; SYLRILNKE; TYVDMVKSII; MYVNEELDAI; AYPFYLRDNL; AYKMYVNEEL; KYYDSNEVEL; YFIDLLVHQSF; MYVNEELDAIL; ITLASPKPYFI; FYTTNDSSTAY |
| | HLA-A2902 | YVFEKDNKY; ETSSDGTAY; SIIKNGQEY; TTNDSSTAY; VFEKDNKYY; ELDAILVPY; FYLRDNLTW; ASKMIDTLY; YFIDLLVHQ; YVFEKDNKYY; YTTNDSSTAY; EVELEEITFY; KYVFEKDNKY; VASKMIDTLY; ILNKETGSTY; KSIIKNGQEY; PVPVHVTDKY; FYTTNDSSTAY; YFIDLLVHQSF; NVASKMIDTLY; KYVFEKDNKYY; GWETSSDGTAY; RILNKETGSTY |
| | HLA-A6801 | GTAYPFYLR; LTVLCCDNK; NMVTSGPFK; GSTYVDMVK; TTNDSSTAY; EGIRKSYLR; VAITAEGIR; ETSSDGTAY; VTSGPFKLK; LAEDNVASK; YVFEKDNKY; TNDSSTAYK; SKMIDTLYR; KISLGAEPR; KSYLRILNK; |

| | | |
|---|---|---|
| | | VLCCDNKER; PSEKYVFEK; TTNDSSTAYK; MVTSGPFKLK; TVLCCDNKER; DGTAYPFYLR; QVTDSELGIR; YTTNDSSTAY; ASKMIDTLYR; TSGPFKLKER; ENMVTSGPFK; GVAITAEGIR; YVFEKDNKYY; YVDMVKSIIK; QLAEDNVASK; TGSTYVDMVK; SLTVLCCDNK; IPSEKYVFEK; EVELEEITFY; TLEITLASPK; ITAEGIRKSY; TKISLGAEPR; VAITAEGIRK; ELGIRAIDSK; TAYKMYVNEE; EKYVFEKDNK; STAYKMYVNE; MVKSIIKNGQ; VASKMIDTLY; IPVPVHVTDK; EITLASPKPY; YTTNDSSTAYK; TAEGIRKSYLR; LTVLCCDNKER; ETGSTYVDMVK; STKISLGAEPR; TVLCCDNKERK; VASKMIDTLYR; TSSDGTAYPFY; NVASKMIDTLY; VTSGPFKLKER; DGVAITAEGIR; FSLTVLCCDNK; NMVTSGPFKLK; KTLEITLASPK; TYVDMVKSIIK; NTGGHKPGLAK; GVAITAEGIRK; RIPSEKYVFEK; STAYKMYVNEE; ETSSDGTAYPF; FIPVPVHVTDK; MVTSGPFKLKE; TTNDSSTAYKM |
| | HLA-B0702 | YPFYLRDNL; SPKPYFIDL; KPYFIDLLV; EPRSLDPQL; SPENMVTSG; SPKPYFIDLL; RAIDSKTLEI; GIRKSYLRIL; KPGLAKGWET; SPENMVTSGPF; YPFYLRDNLTW; SPKPYFIDLLV; KISLGAEPRSL |
| | HLA-B0801 | |
| | HLA-B1501 | YVFEKDNKY; TTNDSSTAY; ITLASPKPY; LNKETGSTY; SIIKNGQEY; IIKNGQEYF; ASKMIDTLY; LQRSLSLII; ETSSDGTAY; TLASPKPYF; ENMVTSGPF; SLSLIIFSL; YVFEKDNKYY; LQRSLSLIIF; ITAEGIRKSY; YTTNDSSTAY; ILNKETGSTY; TSSDGTAYPF; KSIIKNGQEY; HQSFIPVPVH; SIIKNGQEYF; GQNWTSPENM; ITLASPKPYF; YVNEELDAIL; SLIIFSLTVL; KMIDTLYRGI; WETSSDGTAY; KLKERIPSEKY; YFIDLLVHQSF; KLQRSLSLIIF; RILNKETGSTY; TSSDGTAYPFY; AITAEGIRKSY; KSIIKNGQEYF; LEITLASPKPY; QLAEDNVASKM; ETSSDGTAYPF |
| | HLA-B2705 | QRSLSLIIF; RKSYLRILNK; QRSLSLIIFSL; RKEGVSTKISL; IRKSYLRILNK |
| | HLA-B3501 | VPVHVTDKY; TTNDSSTAY; ETSSDGTAY; YVFEKDNKY; EVELEEITF; YPFYLRDNL; YVNEELDAI; ELDAILVPY; TAEGIRKSY; LTWSDGVAI; EPRSLDPQL; DSSTAYKMY; VASKMIDTL; SSDGTAYPF; SIIKNGQEY; ENMVTSGPF; YTTNDSSTAY; YVNEELDAIL; VASKMIDTLY; TSSDGTAYPF; YVFEKDNKYY; YPFYLRDNLT; WETSSDGTAY; LAEDNVASKM; EVELEEITFY; EELDAILVPY; KPYFIDLLVH; EITLASPKPY; DPQLAEDNVA; NEVELEEITF; FIDLLVHQSF; ILNKETGSTY; SPKPYFIDLL; LTWSDGVAIT; SPENMVTSGPF; IPVPVHVTDKY; YPFYLRDNLTW; FYTTNDSSTAY; ETSSDGTAYPF; TSSDGTAYPFY; DPNTGGHKPGL; YFIDLLVHQSF; NVASKMIDTLY; DPQLAEDNVAS; TTNDSSTAYKM; MYVNEELDAIL; NEVELEEITFY; NEELDAILVPY; TAYKMYVNEEL; YFDGQVTDSEL; LEITLASPKPY |
| | HLA-B4403 | EELDAILVP; EEITFYTTN; AEDNVASKM; AEGIRKSYL; VELEEITFY; EELDAILVPY; NEVELEEITF; EEITFYTTND; AEPRSLDPQL; AEDNVASKMI; PENMVTSGPF; WETSSDGTAY; EEITFYTTNDS; NEVELEEITFY; LEITLASPKPY; EELDAILVPYP; AEGIRKSYLRI; NEELDAILVPY; AEPRSLDPQLA |
| | HLA-B5101 | KPYFIDLLV; IPVPVHVTD; GPFKLKERI; LASPKPYFI; ; IPVPVHVTDKY; SPKPYFIDLLV |
| | HLA-B5701 | VTDKYGQNW; |
| NP_045619.1 \| immunogenic protein P37, putative [Borrelia | HLA-A0101 | FSSVISCKLY; NSNASRHESY; MSSTKSLLEV; MTEIESSKEEY; NSNASRHESYY; ISCKLYKKITY |
| | HLA-A0201 | FMADAKASM; ALLPSLEEA; SLEEAIAKV; NLINKLFIL; FILTILFSS; ILTILFSSV; RINEDLAKV; LLSTAKSYL; SLDKIKSLL; LMADMQPDM; LLPSLEEAI; SMSSTKSLL; ILFSSVISC; YAGYHQFMA; KITYNADQV; FILTILFSSV; KLYKKITYNA; ALLPSLEEAI; SLLSTAKSYL; KLFILTILFS; |

| burgdorferi B31]; Seq22 | | LINKLFILTI; NLINKLFILT; FMADAKASMS; ILTILFSSVI; MSSTKSLLEV; KANLALLPSL; LLPSLEEAIA; FILTILFSSVI; SMSSTKSLLEV; NLINKLFILTI; ILFSSVISCKL; KLFILTILFSS; ALLPSLEEAIA; YMDQDTGKKPL; FMADAKASMSS; LINKLFILTIL; NLALLPSLEEA; SLDKIKSLLST; ALEEALSNSNA; YYYAGYHQFMA |
|---|---|---|
| | HLA-A0301 | SVISCKLYK; SLLEVAKNK; VISCKLYKK; IMTEIESSK; KSYLEQTRR; KLYKKITYN; AIAALKRAK; LFSSVISCK; ALSNSNASR; KLFILTILF; SLLSTAKSY; ILFSSVISCK; KIKSLLSTAK; QTRRGVGSSK; SSVISCKLYK; NIMTEIESSK; SVISCKLYKK; KSLLEVAKNK; LAKVKASLDK; STKSLLEVAK; HCNDAIAALK; KSLLSTAKSY; SNNGSFNTLK; LLEVAKNKQK; GYHQFMADAK; TYMDQDTGKK; KLFILTILFS; SLEEAIAKVK; NTYMDQDTGK; DAKASMSSTK; RINEDLAKVK; SMSSTKSLLE; KSNNGSFNTLK; TILFSSVISCK; ITYNADQVIDK; SLLEVAKNKQK; FSSVISCKLYK; NTYMDQDTGKK; ELNENMTKTNK; KQKELNENMTK; LLPSLEEAIAK; SSTKSLLEVAK; KLFILTILFSS; DLAKVKASLDK; AGYHQFMADAK |
| | HLA-A1101 | SVISCKLYK; VISCKLYKK; AIAALKRAK; SLLEVAKNK; KSYLEQTRR; IMTEIESSK; LFSSVISCK; ALSNSNASR; TYMDQDTGK; PSLEEAIAK; TLKSNDDSK; ASRHESYYY; STAKSYLEQ; FQELNDIYK; YMDQDTGKK; SVISCKLYKK; ILFSSVISCK; SSVISCKLYK; STKSLLEVAK; KSLLEVAKNK; KIKSLLSTAK; NTYMDQDTGK; NIMTEIESSK; RINEDLAKVK; KVKASLDKIK; SNNGSFNTLK; NTLKSNDDSK; TYMDQDTGKK; TYNADQVIDK; QTRRGVGSSK; SLEEAIAKVK; KSLLSTAKSY; LAKVKASLDK; GYHQFMADAK; TILFSSVISCK; KSNNGSFNTLK; SSTKSLLEVAK; ITYNADQVIDK; NTYMDQDTGKK; SSVISCKLYKK; FSSVISCKLYK; SLLEVAKNKQK; STAKSYLEQTR; AGYHQFMADAK; KQKELNENMTK; KSNDDSKRSGR; NSNASRHESYY; ELNENMTKTNK; LLPSLEEAIAK; RNIMTEIESSK; TAKSYLEQTRR; ASMSSTKSLLE; AKNDFEYAQRK; THCNDAIAALK; PSLEEAIAKVK |
| | HLA-A2402 | YYYAGYHQF; YYAGYHQFM; EYNRINEDL; SYYYAGYHQF; YYYAGYHQFM; YYAGYHQFMA; LFSSVISCKL; SYYYAGYHQFM; EYAQRKADRAL; TYNADQVIDKL; YYYAGYHQFMA |
| | HLA-A2902 | YYYAGYHQF; DFQELNDIY; ASRHESYYY; KRAKNDFEY; KLFILTILF; SLLSTAKSY; SYYYAGYHQF; NASRHESYYY; FSSVISCKLY; SNASRHESYYY; ALKRAKNDFEY; LFSSVISCKLY; ISCKLYKKITY |
| | HLA-A6801 | ESSKEEYNR; SVISCKLYK; ESYYYAGYH; IMTEIESSK; EYAQRKADR; LFSSVISCK; KSYLEQTRR; MTKTNKDFQ; TLKSNDDSK; NNGSFNTLK; NDAIAALKR; TYMDQDTGK; NASRHESYY; CNDAIAALK; ALSNSNASR; NDFEYAQRK; EIESSKEEY; VISCKLYKK; NSSSNHTLQ; NTYMDQDTGK; EALSNSNASR; SVISCKLYKK; TAKSYLEQTR; DAKASMSSTK; NIMTEIESSK; ILFSSVISCK; NTLKSNDDSK; STKSLLEVAK; ESYYYAGYHQ; TLKSNDDSKR; SSVISCKLYK; DAIAALKRAK; NADQVIDKLK; TYMDQDTGKK; NASRHESYYY; NSNASRHESY; SNNGSFNTLK; HCNDAIAALK; QTRRGVGSSK; FSSVISCKLY; TYNADQVIDK; DFQELNDIYK; MTKTNKDFQE; CNDAIAALKR; DSKRSGRKPR; FEYAQRKADR; NTYMDQDTGKK; STAKSYLEQTR; EIESSKEEYNR; NTLKSNDDSKR; TAKSYLEQTRR; FSSVISCKLYK; TILFSSVISCK; ITYNADQVIDK; NIQDNEASEAR; ELNENMTKTNK; EEALSNSNASR; NSNASRHESYY; SSVISCKLYKK; MTEIESSKEEY; ESYYYAGYHQF; EYNRINEDLAK; SSTKSLLEVAK; DLAKVKASLDK; RAKNDFEYAQR; KSNNGSFNTLK; THCNDAIAALK; YNADQVIDKLK; HCNDAIAALKR; KSNDDSKRSGR; DFEYAQRKADR; DFQELNDIYKK; DNTYMDQDTGK; IYKKLQDMDSR; EQTRRGVGSSK |
| | HLA-B0702 | AQRKADRAL; KPRSVDNTY; ASMSSTKSL; KPRSVDNTYM; KASMSSTKSL; YAQRKADRAL; KASLDKIKSL; KANLALLPSL; KASMSSTKSLL; KPRSVDNTYMD; LPSLEEAIAKV; GVGSSKANLAL; |

| | | KPLMADMQPDM; KASLDKIKSLL; RKADRALEEAL; SKRSGRKPRSV |
|---|---|---|
| | HLA-B0801 | YAQRKADRAL; |
| | HLA-B1501 | KLKSNNGSF; FMADAKASM; KQKELNENM; AQRKADRAL; KLFILTILF; SLLSTAKSY; ASRHESYYY; YYYAGYHQF; LMADMQPDM; ALKRAKNDF; SSVISCKLY; SNASRHESY; NMTKTNKDF; MQNDNSSSNH; KSLLSTAKSY; NSNASRHESY; FSSVISCKLY; KQKELNENMT; HQFMADAKASM; ALKRAKNDFEY; NSNASRHESYY; MQNDNSSSNHT; SNSNASRHESY; INKLFILTILF; ISCKLYKKITY; IKSLLSTAKSY |
| | HLA-B2705 | TRRGVGSSK; KRAKNDFEY; NRINEDLAK; KRSGRKPRS; KRSGRKPRSV; SRHESYYYAGY; RRGVGSSKANL; NRINEDLAKVK; HQFMADAKASM; KRAKNDFEYAQ; KQKELNENMTK; GRKPRSVDNTY |
| | HLA-B3501 | EASEARNIM; KPRSVDNTY; FMADAKASM; NASRHESYY; LPSLEEAIA; DFQELNDIY; HCNDAIAAL; EALSNSNAS; LMADMQPDM; YYYAGYHQF; EIESSKEEY; YYAGYHQFM; HESYYYAGY; NASRHESYYY; KPRSVDNTYM; YAQRKADRAL; LPSLEEAIAK; FSSVISCKLY; QFMADAKASM; QPDMQNDNSS; EASEARNIMT; THCNDAIAAL; TEIESSKEEY; KPLMADMQPDM; MTEIESSKEEY; DTHCNDAIAAL; ESYYYAGYHQF; HQFMADAKASM; IAALKRAKNDF; YMDQDTGKKPL; LPSLEEAIAKV; NSNASRHESYY; EALSNSNASRH; NKDFQELNDIY |
| | HLA-B4403 | HESYYYAGY; EEALSNSNA; TEIESSKEEY; EEALSNSNAS; EEYNRINEDL; EEYNRINEDLA |
| | HLA-B5101 | LPSLEEAIA; ; LPSLEEAIAKV; LALLPSLEEAI |
| | HLA-B5701 | ISCKLYKKITY |
| NP_212281.1 \| flagellin [Borrelia burgdorferi B31]; Seq23 | HLA-A0101 | ESIKNSTEY; KTQEKLSSGY; MTDEVVAATT; ASDDAAGMGV; NQDEAIAVNIY; RLESIKNSTEY; ISDQRANLGAF; ELAVQSGNGTY; MIAQANQVPQY |
| | HLA-A0201 | SQASWTLRV; TMTDEVVAA; QLTDEINRI; AMIAQANQV; MIINHNTSA; QVPQYVLSL; AIAVNIYAA; SLAKIENAI; AVNIYAANV; AQYNQMHML; TTVDANTSL; NLGAFQNRL; SQGGVNSPV; MTDEVVAAT; SAMAMIAQA; AQTAQAAPV; VVAATTNSI; MISDQRANL; ILTQSAMAM; SILTQSAMA; LTQSAMAMI; AQANQVPQYV; MIINHNTSAI; ILTQSAMAMI; NIYAANVANL; TMTDEVVAAT; RMISDQRANL; NLNEVEKVLV; VLVRMKELAV; MAMIAQANQV; AQAAPVQEGV; QLTDEINRIA; ATMTDEVVAA; QVPQYVLSLL; NQVPQYVLSL; IAVNIYAANV; SLSGSQASWTL; MLSNKSASQNV; AMAMIAQANQV; AIAVNIYAANV; SQASWTLRVHV; KLSSGYRINRA; SILTQSAMAMI; GMQPAKINTPA; KVLVRMKELAV; TMTDEVVAATT; MISDQRANLGA; YAIENLKASYA; SLAKIENAIRM; NLFSGEGAQTA; ILTQSAMAMIA |
| | HLA-A0301 | GINAANLSK; SQASRNTSK; NQMHMLSNK; GSQASWTLR; FQNRLESIK; LSSGYRINR; TVDANTSLAK; LSQASRNTSK; KLSSGYRINR; NLKASYAQIK; NGINAANLSK; AANLSKTQEK; STEYAIENLK; KTQEKLSSGY; YNQMHMLSNK; AFQNRLESIK; QIRGLSQASR; VPQYVLSLLR; GLSQASRNTSK; MIAQANQVPQY; LSGSQASWTLR; GAFQNRLESIK; KTQEKLSSGYR; RLESIKNSTEY; TTVDANTSLAK; KINAQIRGLSQ; LSNKSASQNVR |
| | HLA-A1101 | GINAANLSK; SQASRNTSK; NQMHMLSNK; GSQASWTLR; AAGMGVSGK; LSSGYRINR; NTSAINASR; TEYAIENLK; AVQSGNGTY; PQYVLSLLR; FQNRLESIK; AQANQVPQY; TVDANTSLAK; STEYAIENLK; LSQASRNTSK; AANLSKTQEK; RANLGAFQNR; KTQEKLSSGY; KLSSGYRINR; AFQNRLESIK; YNQMHMLSNK; IAQANQVPQY; TQEKLSSGYR; NLKASYAQIK; EVEKVLVRMK; TTVDANTSLAK; TTEGNLNEVEK; KTQEKLSSGYR; GAFQNRLESIK; TAEELGMQPAK; NSTEYAIENLK; GLSQASRNTSK; TSLAKIENAIR; QYNQMHMLSNK; NAANLSKTQEK; QVPQYVLSLLR; AQIRGLSQASR; |

| | | |
|---|---|---|
| | | LSGSQASWTLR; GVSGKINAQIR; MIAQANQVPQY; LSNKSASQNVR; SAMAMIAQANQ; |
| | HLA-A2402 | IYAANVANL; YAANVANLF; AFQNRLESI; IYAANVANLF; TYSDADRGSI; SYAQIKDATM; IYAANVANLFS |
| | HLA-A2902 | ESIKNSTEY; AVQSGNGTY; YAANVANLF; AIENLKASY; YAIENLKASY; IAQANQVPQY; EYAIENLKASY; ELAVQSGNGTY; RLESIKNSTEY; EINRIADQAQY; MIAQANQVPQY |
| | HLA-A6801 | NTSAINASR; LSSGYRINR; ESIKNSTEY; TEYAIENLK; AIRMISDQR; LAKIENAIR; EGNLNEVEK; YAANVANLF; NGTYSDADR; GSQASWTLR; NQMHMLSNK; QTAQAAPVQ; TSKAINFIQ; MAMIAQANQ; NKSASQNVR; GINAANLSK; TTNSILTQS; SQASRNTSK; NAIRMISDQR; STEYAIENLK; DAAGMGVSGK; HNTSAINASR; EVEKVLVRMK; SLAKIENAIR; TVDANTSLAK; RANLGAFQNR; QIRGLSQASR; NTSKAINFIQ; LSQASRNTSK; NLKASYAQIK; SGSQASWTLR; TQEKLSSGYR; VPQYVLSLLR; NGINAANLSK; AANLSKTQEK; YAIENLKASY; VSGKINAQIR; TTVDANTSLA; KLSSGYRINR; MIAQANQVPQ; QTAQAAPVQE; NTSAINASRN; NSTEYAIENLK; TTVDANTSLAK; TTEGNLNEVEK; EIEQLTDEINR; QVPQYVLSLLR; NAANLSKTQEK; ENAIRMISDQR; LSNKSASQNVR; MIAQANQVPQY; TSLAKIENAIR; EINRIADQAQY; NLNEVEKVLVR; LSGSQASWTLR; GAFQNRLESIK; TAEELGMQPAK; QYNQMHMLSNK; GVSGKINAQIR; KTQEKLSSGYR; NHNTSAINASR; NIYAANVANLF; EKLSSGYRINR; ENLKASYAQIK |
| | HLA-B0702 | SPVNVTTTV; VPQYVLSLL; QPAKINTPA; VVAATTNSI; QPAPATAPS; LVRMKELAV; ASRNTSKAI; IINHNTSAI; TPASLSGSQ; APSQGGVNS; VAATTNSIL; APATAPSQG; APVQEGVQQ; ILTQSAMAM; TPASLSGSQA; SPVNVTTTVD; VVAATTNSIL; APSQGGVNSP; QPAKINTPAS; QASRNTSKAI; MIINHNTSAI; APSQGGVNSPV; QPAKINTPASL; APATAPSQGGV; SPVNVTTTVDA; TPASLSGSQAS; AANLSKTQEKL; RANLGAFQNRL |
| | HLA-B0801 | YAQIKDATM; KVLVRMKEL; ; VEKVLVRMKEL |
| | HLA-B1501 | AQANQVPQY; DQRANLGAF; YAANVANLF; AVQSGNGTY; TQEKLSSGY; ILTQSAMAM; AQYNQMHML; VVAATTNSI; AQTAQAAPV; SQGGVNSPV; ESIKNSTEY; SLSGSQASW; SQASWTLRV; IQIEIEQLT; AMIAQANQV; YAIENLKASY; LAVQSGNGTY; SQASWTLRVH; KTQEKLSSGY; SQNVRTAEEL; RMISDQRANL; VQQEGAQQPA; IAQANQVPQY; VVAATTNSIL; AQAAPVQEGV; AQIRGLSQAS; NQVPQYVLSL; INRIADQAQY; MQPAKINTPA; SQASRNTSKAI; AQANQVPQYVL; MIAQANQVPQY; ASRNTSKAINF; ELAVQSGNGTY; RLESIKNSTEY; NIYAANVANLF; NQDEAIAVNIY; SLSGSQASWTL |
| | HLA-B2705 | NRIADQAQY; IRGLSQASR; QRANLGAFQ; SRNTSKAINF; QRANLGAFQNR; IRMISDQRANL; SRNNGINAANL |
| | HLA-B3501 | YAANVANLF; YAQIKDATM; QPAPATAPS; RASDDAAGM; SPVNVTTTV; ESIKNSTEY; ILTQSAMAM; DEAIAVNIY; TTVDANTSL; LAVQSGNGT; QPAKINTPA; NSILTQSAM; YAIENLKAS; AVQSGNGTY; QASWTLRVH; VAATTNSIL; IAVNIYAAN; MTDEVVAAT; VPQYVLSLL; LAVQSGNGTY; YAIENLKASY; IADQAYNQM; SILTQSAMAM; IAQANQVPQY; QPAKINTPAS; LAKIENAIRM; QANQVPQYVL; QPAPATAPSQ; YAANVANLFS; TAEELGMQPA; MIINHNTSAI; EAIAVNIYAA; NRASDDAAGM; QAQYNQMHML; TPASLSGSQA; SPVNVTTTVD; SYAQIKDATM; MAMIAQANQV; MIAQANQVPQY; TPASLSGSQAS; ELAVQSGNGTY; NQDEAIAVNIY; QPAKINTPASL; IAVNIYAANVA; NSILTQSAMAM; NIYAANVANLF; EVVAATTNSIL; TTNSILTQSAM; SPVNVTTTVDA; EINRIADQAQY; NASRNNGINAA; IADQAQYNQMH; ISDQRANLGAF; APSQGGVNSPV; LAVQSGNGTYS; EYAIENLKASY |
| | HLA-B4403 | EELGMQPAKI; NEVEKVLVRM; DEINRIADQA; EEYNRINEDLA |

| | HLA-B5101 | LPSLEEAIA; APATAPSQGGV; APSQGGVNSPV |
|---|---|---|
| | HLA-B5701 | ASLSGSQASW; |
| NP_212463.1 \| oligopeptide ABC transporter, periplasmic oligopeptide-binding protein (oppA-2) ; [Borrelia burgdorferi B31]; Seq24 | HLA-A0101 | YSSAVNAIY; TTNDSSTAY; YTQFSSHNY; AIDRETLTY; DSSTAYKMY; FLSIFTQGY; RSDYYSSAV; SSDGTVYTF; YTTNDSSTAY; SSAVNAIYFY; SSHNYSNPEY; YLNTKANGNY; ATPNFSSYSY; ELENEEWTTY; YSSAVNAIYF; EVELEEITFY; YSSAVNAIYFY; FSSHNYSNPEY; TLAIDRETLTY; LTFLSIFTQGY; YADPLTFLSIF |
| | HLA-A0201 | SLFLIIFFL; KLQRSLFLI; IIIEKDFPI; AVNAIYFYA; AIDEKTLEI; LIIFFLTFL; LVHQSFIPV; VLDNGTTPT; SLELFNPEI; YSYAKSLEL; SLGAEPSSL; HQSFIPVPV; WIGDYADPL; MVTSGPFKL; ELEEITFYT; KMIDTMFRG; AIYFYAFNT; KTLEITLES; ITWSDGVAI; FLIIFFLTF; MIDTMFRGI; RSDYYSSAV; YKMYENEEL; YFYAFNTHI; FLIIFFLTFL; MLVHQSFIPV; KMIDTMFRGI; KLQRSLFLII; SLFLIIFFLT; YADPLTFLSI; KMYENEELDA; AIFGSIPPDL; FLSIFTQGYT; NMVTSGPFKL; KLRSDYYSSA; RSLFLIIFFL; SAVNAIYFYA; IIIEKDFPIA; KSLELFNPEI; YAFNTHIKPL; MIDTMFRGIV; TLESPKPYFI; ILERFDLSQL; KVLDNGTTPT; SIPPDLIKNL; RAIDEKTLEI; FLIIFFLTFL; MLVHQSFIPV; KMIDTMFRGI; KLQRSLFLII; SLFLIIFFLT; YADPLTFLSI; KMYENEELDA; AIFGSIPPDL; FLSIFTQGYT; NMVTSGPFKL; KLRSDYYSSA; RSLFLIIFFL; SAVNAIYFYA; IIIEKDFPIA; KSLELFNPEI; YAFNTHIKPL; MIDTMFRGIV; TLESPKPYFI; ILERFDLSQL; KVLDNGTTPT; SIPPDLIKNL; RAIDEKTLEI |
| | HLA-A0301 | KSYLRILNK; ILNKETGSK; NMVTSGPFK; VTSGPFKLK; ELFNPEIAK; LTFLCCNNK; KSVIKNGQK; GSKYVEMVK; AITAEGIRK; TTNDSSTAY; GSIPPDLIK; RATPNFSSY; NSYLFRNDK; GGNKPGLAK; RILNKETGSK; YIYGNSYLFR; TVYTFNLREK; TTNDSSTAYK; MVTSGPFKLK; AIDRETLTYK; FLTFLCCNNK; ATPNFSSYSY; RKSYLRILNK; YFYAFNTHIK; VAITAEGIRK; KLKERIPNEK; YVEMVKSVIK; KPLDNVKIRK; RQAEEIIIEK; TLEITLESPK; SSAVNAIYFY; GNGFPILKLK; TGGNKPGLAK; ENMVTSGPFK; ILNKETGSKY; QLAEDNVASK; RFDLSQLKLK; AIYFYAFNTH; IPVPVHVTEK; YLNTKANGNY; LELFNPEIAK; ASKMIDTMFR; FLCCNNKERK; VLDNGTTPTR; ELGIRAIDEK; KERKEGVSFK; DLSQLKLKNK; RRATPNFSSY; LERFDLSQLK; LKYNTNEANK; KTLEITLESPK; KLKYNTNEANK; MVKSVIKNGQK; KICEFIQNQWK; ILERFDLSQLK; NMVTSGPFKLK; GVAITAEGIRK; RILNKETGSKY; YTTNDSSTAYK; RIPNEKYVFEK; VTGDPNTGGNK; KVLDNGTTPTR; KLKERIPNEKY; TLAIDRETLTY; SLELFNPEIAK; IYFYAFNTHIK; FIPVPVHVTEK; LTFLSIFTQGY; LAIDRETLTYK; GTVYTFNLREK; IFGSIPPDLIK; VLDNGTTPTRR; RATPNFSSYSY; SIPPDLIKNLK; NTGGNKPGLAK; YSSAVNAIYFY |
| | HLA-A1101 | KSYLRILNK; VTSGPFKLK; LTFLCCNNK; AITAEGIRK; NSYLFRNDK; TTNDSSTAY; GSIPPDLIK; GTVYTFNLR; NMVTSGPFK; GSKYVEMVK; SAVNAIYFY; KSVIKNGQK; YTQFSSHNY; ILNKETGSK; LSQLKLKNK; QAEEIIIEK; ELFNPEIAK; AVNAIYFYA; NFSSYSYAK; KALTLAIDR; TVYTFNLRE; NGNGFPILK; LAEDNVASK; TNDSSTAYK; SVIKNGQKY; KYNTNEANK; SKMIDTMFR; AIYFYAFNT; ITLESPKPY; SLFLIIFFL; KTLLAEAGY; LTFLSIFTQ; VTDSELGIR; PVPVHVTEK; RATPNFSSY; AIDRETLTY; GGNKPGLAK; TTNDSSTAYK; TVYTFNLREK; MVTSGPFKLK; SSAVNAIYFY; AIDRETLTYK; YIYGNSYLFR; RILNKETGSK; RQAEEIIIEK; VAITAEGIRK; ATPNFSSYSY; ASKMIDTMFR; TLEITLESPK; KSDLELDPIK; YFYAFNTHIK; YVEMVKSVIK; KLKERIPNEK; AIYFYAFNTH; SSHNYSNPEY; QLAEDNVASK; FLTFLCCNNK; IPNEKYVFEK; KANGNYEIAR; RKSYLRILNK; TGSKYVEMVK; AVNAIYFYAF; KPLDNVKIRK; TSGPFKLKER; YVFEKNNKYY; QVTDSELGIR; KYNTNEANKK; GVAITAEGIR; RFDLSQLKLK; FLCCNNKERK; KTLEITLESPK; KICEFIQNQWK; GVAITAEGIRK; YTTNDSSTAYK; GTVYTFNLREK; |

| | | |
|---|---|---|
| | | VTGDPNTGGNK; MVKSVIKNGQK; SLELFNPEIAK; LAIDRETLTYK; TPNFSSYSYAK; SIPPDLIKNLK; RIPNEKYVFEK; KVLDNGTTPTR; VASKMIDTMFR; YSSAVNAIYFY; NTGGNKPGLAK; NMVTSGPFKLK; ILERFDLSQLK; LTFLSIFTQGY; YSNPEYNELIK; VTSGPFKLKER; IYFYAFNTHIK; ETGSKYVEMVK; KLKYNTNEANK; NTHIKPLDNVK; LTFLCCNNKER; TAEGIRKSYLR; AVNAIYFYAFN; RATPNFSSYSY; FIPVPVHVTEK; YPNGNGFPILK; TFLCCNNKERK; KSDLELDPIKR; WTGWNTNILER; IYIYGNSYLFR |
| | HLA-A2402 | SYAKSLELF; YYSSAVNAI; IYIYGNSYL; YYDSNEVEL; FYAFNTHIK; DFPIAPIYI; SYLFRNDKW; DYADPLTFL; IYGNSYLFR; IFTQGYTQF; KLQRSLFLI; YIIGNSYLF; SFIPVPVHV; KYVEMVKSV; IFGSIPPDL; RSLFLIIFF; YFYAFNTHI; KWTGWNTNI; SYLRILNKE; LFLIIFFLT; IYFYAFNTH; IYIYGNSYLF; IYFYAFNTHI; LFLIIFFLTF; DYYSSAVNAI; VYTFNLREKI; KYVEMVKSVI; SYSYAKSLEL; IFGSIPPDLI; GYPNGNGFPI; GWNTNILERF; NYSNPEYNEL; MYENEELDAI; KYYDSNEVEL; NYEIARAGWI; YYSSAVNAIY; KWTGWNTNIL; AYKMYENEEL; KYNTNEANKK; AVNAIYFYAF; YYSSAVNAIYF; SYSYAKSLELF; YFIDMLVHQSF; DYADPLTFLSI; FYAFNTHIKPL; NYSNPEYNELI; KYNTNEANKKI; MYENEELDAIF; IYFYAFNTHIK; LFLIIFFLTFL; IYIYGNSYLFR; GYPNGNGFPIL; ITLESPKPYFI; TTPTRRATPNF; FYTTNDSSTAY |
| | HLA-A2902 | YVFEKNNKY; SVIKNGQKY; GFPILKLKY; DISSDGTVY; YTQFSSHNY; YSSAVNAIY; TTNDSSTAY; FLSIFTQGY; YIYGNSYLF; FLIIFFLTF; YFYAFNTHI; VFEKNNKYY; FPIAPIYIY; PIYIYGNSY; IFTQGYTQF; SAVNAIYFY; AIDRETLTY; YVFEKNNKYY; YYSSAVNAIY; YTTNDSSTAY; TFLSIFTQGY; YLNTKANGNY; IYIYGNSYLF; ELENEEWTTY; EVELEEITFY; GYTQFSSHNY; KYVFEKNNKY; LAIDRETLTY; SSAVNAIYFY; ILNKETGSKY; SIFTQGYTQF; PVPVHVTEKY; DFPIAPIYIY; ATPNFSSYSY; AVNAIYFYAF; NGFPILKLKY; LFLIIFFLTF; YFYAFNTHIK; YSSAVNAIYF; SVIKNGQKYF; RRATPNFSSY; WDISSDGTVY; EITLESPKPY; FYTTNDSSTAY; YFIDMLVHQSF; DYYSSAVNAIY; YSSAVNAIYFY; TYLNTKANGNY; TLAIDRETLTY; KYVFEKNNKYY; YYSSAVNAIYF; SLFLIIFFLTF; FSSHNYSNPEY; RILNKETGSKY; MYENEELDAIF; EIARAGWIGDY; KDFPIAPIYIY; GWDISSDGTVY; RATPNFSSYSY; LTFLSIFTQGY; YADPLTFLSIF; NVASKMIDTMF |
| | HLA-A6801 | GTVYTFNLR; NSYLFRNDK; FYAFNTHIK; LTFLCCNNK; SAVNAIYFY; ELFNPEIAK; NFSSYSYAK; EFIQNQWKK; DLIKNLKLR; YTQFSSHNY; FLCCNNKER; NMVTSGPFK; QAEEIIIEK; TTNDSSTAY; EGIRKSYLR; SKMIDTMFR; YSSAVNAIY; YIYGNSYLF; VAITAEGIR; LTFLSIFTQ; HIKPLDNVK; YVFEKNNKY; TPNFSSYSY; ERFDLSQLK; VTSGPFKLK; NGNGFPILK; GSKYVEMVK; NGFPILKLK; EWTTYLNTK; LAEDNVASK; NAIYFYAFN; TNDSSTAYK; KSYLRILNK; IYGNSYLFR; TVYTFNLRE; SLFLIIFFL; DLELDPIKR; VYTFNLREK; LDNGTTPTR; FPIAPIYIY; KALTLAIDR; SSTAYKMYE; ILNKETGSK; STAYKMYEN; YIYGNSYLFR; TTNDSSTAYK; TVYTFNLREK; MVTSGPFKLK; YFYAFNTHIK; ASKMIDTMFR; QVTDSELGIR; FLTFLCCNNK; DGTVYTFNLR; SSAVNAIYFY; YVEMVKSVIK; YTTNDSSTAY; YVFEKNNKYY; TSGPFKLKER; ENMVTSGPFK; GVAITAEGIR; DPIKRQDILR; FLCCNNKERK; KANGNYEIAR; QLAEDNVASK; TFLCCNNKER; TGSKYVEMVK; IPVPVHVTEK; ELGIRAIDEK; EVELEEITFY; IPNEKYVFEK; TGWNTNILER; SSHNYSNPEY; SSYSYAKSLE; ITAEGIRKSY; TLEITLESPK; EKYVFEKNNK; VAITAEGIRK; ATPNFSSYSY; DLSQLKLKNK; CEFIQNQWKK; STAYKMYENE; NAIYFYAFNT; TAYKMYENEE; YTTNDSSTAYK; LTFLCCNNKER; TAEGIRKSYLR; MVKSVIKNGQK; VASKMIDTMFR; IYIYGNSYLFR; |

| | | |
|---|---|---|
| | | YSSAVNAIYFY; WTGWNTNILER; ETGSKYVEMVK; TPNFSSYSYAK; LAIDRETLTYK; GTVYTFNLREK; IYFYAFNTHIK; LTFLSIFTQGY; NTHIKPLDNVK; VTSGPFKLKER; HIKPLDNVKIR; DGVAITAEGIR; NMVTSGPFKLK; YSNPEYNELIK; YPNGNGFPILK; FSSHNYSNPEY; TKANGNYEIAR; NTGGNKPGLAK; DYYSSAVNAIY; FIPVPVHVTEK; KTLEITLESPK; SIPPDLIKNLK; KVLDNGTTPTR; NAIYFYAFNTH; GVAITAEGIRK; RIPNEKYVFEK; SLELFNPEIAK; STAYKMYENEE; NGNGFPILKLK; ERFDLSQLKLK; KICEFIQNQWK; TFLCCNNKERK; ILERFDLSQLK; YGNSYLFRNDK; EIARAGWIGDY; FFLTFLCCNNK; MVTSGPFKLKE; FTQGYTQFSSH; TTNDSSTAYKM |
| HLA-B0702 | YPNGNGFPI; NPEIAKTLL; SPKPYFIDM; TPTRRATPN; TPNFSSYSY; SPENMVTSG; KPYFIDMLV; DPIKRQDIL; YPNGNGFPIL; KIRKALTLAI; TPTRRATPNF; SPKPYFIDML; APIYIYGNSY; TPNFSSYSYA; KPGLAKGWDI; GIRKSYLRIL; SPENMVTSGPF; APIYIYGNSYL; IPPDLIKNLKL; KPLDNVKIRKA; KISLGAEPSSL; TPTRRATPNFS |
| HLA-B0801 | FLIIFFLTF; IRKALTLAI; NVKIRKALTL; KIRKALTLAI; |
| HLA-B1501 | YVFEKNNKY; YSSAVNAIY; TTNDSSTAY; YTQFSSHNY; FLSIFTQGY; FLIIFFLTF; ITLESPKPY; YIYGNSYLF; SVIKNGQKY; VIKNGQKYF; RATPNFSSY; LNKETGSKY; YSYAKSLEL; IQNQWKKNL; SSAVNAIYF; AGYPNGNGF; NLKLRSDYY; ASKMIDTMF; PIYIYGNSY; ENMVTSGPF; KERKEGVSF; RSLFLIIFF; LENEEWTTY; RQAEEIIIE; KTLLAEAGY; YSNPEYNEL; YVFEKNNKYY; ITAEGIRKSY; YTTNDSSTAY; YSSAVNAIYF; IARAGWIGDY; YLNTKANGNY; LQRSLFLIIF; YSYAKSLELF; ISSDGTVYTF; AVNAIYFYAF; SSHNYSNPEY; LAIDRETLTY; ILNKETGSKY; SVIKNGQKYF; HQSFIPVPVH; SSAVNAIYFY; GQNWTSPENM; YAFNTHIKPL; ATPNFSSYSY; SIFTQGYTQF; KSVIKNGQKY; ITLESPKPYF; YENEELDAIF; YYSSAVNAIY; WDISSDGTVY; GQKYFDGQVT; KMIDTMFRGI; YSSAVNAIYFY; LQRSLFLIIFF; LTFLSIFTQGY; KLKERIPNEKY; YFIDMLVHQSF; FSSHNYSNPEY; SLFLIIFFLTF; TLAIDRETLTY; RATPNFSSYSY; LSIFTQGYTQF; KLQRSLFLIIF; AITAEGIRKSY; IAKTLLAEAGY; KSVIKNGQKYF; RILNKETGSKY; LIKNLKLRSDY; LEITLESPKPY; YLFRNDKWTGW; WIGDYADPLTF; QGYTQFSSHNY; IAPIYIYGNSY; QLAEDNVASKM; SAVNAIYFYAF; IQNQWKKNLNI; KMYENEELDAI; IEKDFPIAPIY |
| HLA-B2705 | ARAGWIGDY; RRATPNFSSY; RQAEEIIIEK; ERFDLSQLKL; QRSLFLIIFF; RKSYLRILNK; KRQDILRQAE; LRILNKETGSK; RRATPNFSSYS; RKEGVSFKISL; QRSLFLIIFFL; LRQAEEIIIEK; ERFDLSQLKLK; IRKSYLRILNK |
| HLA-B3501 | TPNFSSYSY; FPIAPIYIY; TTNDSSTAY; DPLTFLSIF; SAVNAIYFY; YPNGNGFPI; VPVHVTEKY; YSSAVNAIY; DISSDGTVY; YVFEKNNKY; EPSSLDPQL; VASKMIDTM; EVELEEITF; FLIIFFLTF; YIYGNSYLF; FLSIFTQGY; WTGWNTNIL; YTQFSSHNY; SPKPYFIDM; NPEIAKTLL; RATPNFSSY; WIGDYADPL; LENEEWTTY; ENEELDAIF; TAEGIRKSY; YSNPEYNEL; ITLESPKPY; DSSTAYKMY; VNAIYFYAF; ITWSDGVAI; EANKKICEF; SHNYSNPEY; YYSSAVNAI; ENMVTSGPF; IFTQGYTQF; YPNGNGFPIL; YTTNDSSTAY; LAIDRETLTY; APIYIYGNSY; VASKMIDTMF; YYSSAVNAIY; YENEELDAIF; WDISSDGTVY; LAEDNVASKM; YVFEKNNKYY; YAFNTHIKPL; EVELEEITFY; TPTRRATPNF; ELENEEWTTY; ISSDGTVYTF; AVNAIYFYAF; TFLSIFTQGY; YSSAVNAIYF; LFLIIFFLTF; SSHNYSNPEY; KPYFIDMLVH; NVASKMIDTM; EAGYPNGNGF; DPQLAEDNVA; SPKPYFIDML; NEVELEEITF; EPSSLDPQLA; TPNFSSYSYA; EITLESPKPY; FIDMLVHQSF; SSAVNAIYFY; YLNTKANGNY; YADPLTFLSI; DPLTFLSIFT; IGDYADPLTF; SIFTQGYTQF; YSYAKSLELF; DPNTGGNKPG; DFPIAPIYIY; SPENMVTSGPF; |

| | | |
|---|---|---|
| | | SAVNAIYFYAF; IPVPVHVTEKY; YADPLTFLSIF; FYTTNDSSTAY; IAPIYIYGNSY; YSSAVNAIYFY; YFIDMLVHQSF; IAKTLLAEAGY; DPNTGGNKPGL; FSSHNYSNPEY; WIGDYADPLTF; RATPNFSSYSY; NAIYFYAFNTH; TPNFSSYSYAK; NVASKMIDTMF; MYENEELDAIF; YYSSAVNAIYF; DISSDGTVYTF; TLAIDRETLTY; DPQLAEDNVAS; LTFLSIFTQGY; EPSSLDPQLAE; TTNDSSTAYKM; NEVELEEITFY; QAEEIIIEKDF; YPNGNGFPILK; TAYKMYENEEL; TGWNTNILERF; YFDGQVTDSEL; DKWTGWNTNIL; EIARAGWIGDY; DYYSSAVNAIY; LEITLESPKPY; NPEIAKTLLAE; AGWIGDYADPL; APIYIYGNSYL; DAIFGSIPPDL; FYAFNTHIKPL; FPILKLKYNTN |
| | HLA-B4403 | EELDAIFGS; EEIIIEKDF; EEITFYTTN; AEDNVASKM; YEIARAGWI; AEGIRKSYL; KERKEGVSF; AEAGYPNGN; VELEEITFY; NEVELEEITF; EELDAIFGSI; EEITFYTTND; AEPSSLDPQL; YENEELDAIF; AEDNVASKMI; AEEIIIEKDF; NEANKKICEF; PENMVTSGPF; NEELDAIFGS; EEIIIEKDFPI; AEAGYPNGNGF; EEITFYTTNDS; NEVELEEITFY; LEITLESPKPY; IEKDFPIAPIY; AEGIRKSYLRI; NELIKKSDLEL; EELDAIFGSIP |
| | HLA-B5101 | KPYFIDMLV; FPIAPIYIY; YPNGNGFPI; IPVPVHVTE; IPPDLIKNL; GPFKLKERI; DPLTFLSIF; FPIAPIYIYG; FPILKLKYNT; DPLTFLSIFT; IPVPVHVTEK; YPNGNGFPIL; IPPDLIKNLKL; IPVPVHVTEKY; FPILKLKYNTN; SPKPYFIDMLV; FPIAPIYIYGN |
| | HLA-B5701 | RATPNFSSY; DVELENEEW; KICEFIQNQW; NSYLFRNDKW; YTFNLREKITW; LTFLSIFTQGY |
| AAC44381.1\| outer membrane porin protein Oms28 precursor; Seq25 | HLA-A0101 | MSDVAKGTV; LTKVMAISLY; MSDVAKGTVV; FSNLIINGLLF |
| | HLA-A0201 | FLIEKQIML; KVMAISLYM; ALDETVQEA; GLLFGFVSL; KVLNMVNGL; VAGEATFLI; KAISNVVKV; TLMASERAL; QALDTINKV; FADSNNANI; MVAEGANKV; SVAGEATFL; TINKVTEDV; KVDEVKETL; LLFGFVSLNV; IINGLLFGFV; SVAGEATFLI; LMSDVAKGTV; GMVAEGANKV; SLMSDVAKGT; RALDETVQEA; KVTEDVSSKL; GLNPSNKDQV; VVASQEATIV; GLLFGFVSLNV; SLMSDVAKGTV; KIFSNLIINGL; LMSDVAKGTVV; LIINGLLFGFV; FVGSMSLMSDV; KLDQKDQVNQA; MLNKSPNNKEL; GMVAEGANKVV; KVDEVKETLMA; LMASERALDET; FLIEKQIMLNK; AVQETQKAVSV; MASERALDETV |
| | HLA-A0301 | MLNKSPNNK; VLAKKDVRK; KVTEDVSSK; GMVAEGANK; KVMAISLYM; MSLMSDVAK; VMAISLYMR; NQALDTINK; IMLNKSPNNK; KVMAISLYMR; MVNGLNPSNK; SMSLMSDVAK; LIEKQIMLNK; ASQEATIVAK; QVLAKKDVRK; GANKVVEMSK; ADSNNANILK; SGMVAEGANK; VAQGARDLTK; LTKVMAISLY; ESNDAGVVKK; FLIEKQIMLNK; KVAQGARDLTK; GSMSLMSDVAK; QIMLNKSPNNK; GANKVVEMSKK; MSKKAVQETQK; QVNQALDTINK; NMVNGLNPSNK; CSGMVAEGANK; ALDETVQEAQK; PSNKDQVLAKK |
| | HLA-A1101 | MSLMSDVAK; KVTEDVSSK; SQEATIVAK; MLNKSPNNK; AQGARDLTK; NVLEHSDQK; GMVAEGANK; VMAISLYMR; DSNNANILK; NQALDTINK; KVMAISLYM; VLAKKDVRK; ESNDAGVVK; GEATFLIEK; MVNGLNPSNK; KVMAISLYMR; ASQEATIVAK; SMSLMSDVAK; GANKVVEMSK; IMLNKSPNNK; QVLAKKDVRK; VAQGARDLTK; LIEKQIMLNK; ESNDAGVVKK; AGEATFLIEK; PSNKDQVLAK; SGMVAEGANK; VNQALDTINK; LTKVMAISLY; GSMSLMSDVAK; QVNQALDTINK; QIMLNKSPNNK; KVAQGARDLTK; VAGEATFLIEK; GANKVVEMSKK; QSNVLEHSDQK; VASQEATIVAK; FLIEKQIMLNK; CSGMVAEGANK; NMVNGLNPSNK; MSKKAVQETQK; ALDETVQEAQK; LVESNDAGVVK; FADSNNANILK; SPNNKELELTK; PSNKDQVLAKK; VLEHSDQKDNK |
| | HLA-A2402 | LFGFVSLNVF; VFADSNNANI; TFLIEKQIML; IFSNLIINGLL; VFADSNNANIL |

| | HLA-A2902 | IINGLLFGF; LIINGLLFGF; NLIINGLLFGF |
|---|---|---|
| | HLA-A6801 | DSNNANILK; MSLMSDVAK; NVLEHSDQK; EFAKVDEVK; VMAISLYMR; ELTKEEFAK; ESNDAGVVK; MLNKSPNNK; VVKVAQGAR; KVTEDVSSK; QVLAKKDVR; EATFLIEKQ; VSSKLEGVR; NQALDTINK; MVNGLNPSNK; NVVKVAQGAR; KVMAISLYMR; DVSSKLEGVR; ESNDAGVVKK; SMSLMSDVAK; GANKVVEMSK; LTKVMAISLY; EEFAKVDEVK; LIEKQIMLNK; QVLAKKDVRK; VAQGARDLTK; EVKETLMASER; MSKKAVQETQK; QVNQALDTINK; TKVMAISLYMR; QIMLNKSPNNK; QSNVLEHSDQK; FADSNNANILK; FLIEKQIMLNK; EGANKVVEMSK; DVRKAISNVVK; ELELTKEEFAK; NMVNGLNPSNK; CSGMVAEGANK; DLTKVMAISLY; GSMSLMSDVAK; LVESNDAGVVK; EDVSSKLEGVR; GANKVVEMSKK; VASQEATIVAK; VAGEATFLIEK; KVAQGARDLTK; MVAEGANKVVE |
| | HLA-B0702 | GVRESSLEL; SPNNKELEL; GARDLTKVM; NPSNKDQVL; KVAQGARDL; VVKKFVGSM; KVMAISLYM; AVQETQKAV; KVVEMSKKAV; KVTEDVSSKL; KAVQETQKAV; GARDLTKVMAI; AVSVAGEATFL; VVKKFVGSMSL; AVQETQKAVSV |
| | HLA-B0801 | ILKPQSNVL; FLIEKQIML; LAKKDVRKAI; FAKVDEVKETL; VLAKKDVRKAI; VVKKFVGSMSL |
| | HLA-B1501 | VSVAGEATF; KVMAISLYM; FGFVSLNVF; TLMASERAL; ILKPQSNVL; GVRESSLEL; NLIINGLLF; FLIEKQIML; IINGLLFGF; VVKKFVGSM; AVSVAGEATF; LTKVMAISLY; LIINGLLFGF; DQKDQVNQAL; MVAEGANKVV; LLFGFVSLNVF; AQGARDLTKVM; FSNLIINGLLF; AQKVLNMVNGL; LMSDVAKGTVV; VVKKFVGSMSL; NLIINGLLFGF; KAVSVAGEATF; ILKPQSNVLEH; GMVAEGANKVV; TQKAVSVAGEA |
| | HLA-B2705 | RKAISNVVK; KKFVGSMSL; VRKAISNVVK; |
| | HLA-B3501 | NPSNKDQVL; FGFVSLNVF; VSVAGEATF; SPNNKELEL; TKVMAISLY; TFLIEKQIM; FSNLIINGL; AVSVAGEATF; LFGFVSLNVF; MASERALDET; VASQEATIVA; NPSNKDQVLA; LIINGLLFGF; MVAEGANKVV; ETLMASERAL; LTKVMAISLY; KAVSVAGEATF; VAEGANKVVEM; EATFLIEKQIM; FSNLIINGLLF; LLFGFVSLNVF; FAKVDEVKETL; NPSNKDQVLAK; ESNDAGVVKKF; VFADSNNANIL; MASERALDETV |
| | HLA-B4403 | DEVKETLMA; EEFAKVDEV; QEAQKVLNM; KELELTKEEF; AEGANKVVEM; DEVKETLMAS; KETLMASERAL; GEATFLIEKQI; AEGANKVVEMS |
| | HLA-B5101 | |
| | HLA-B5701 | |
| CAA49829.1\| p93 [Borrelia burgdorferi]; Seq26 | HLA-A0101 | DLDYYKGFY; NSNSNSSYY; NMDLEFVNY; STQQGIQRY; FSVRKNFIY; LIAKVITIY; ITDIQGETH; LTNSNSNSSY; NSNSSYYGKY; ESTQQGIQRY; VTDKVIAALL; ESDIDIDSLV; LTNSNSNSSYY; LTGYIIKSFDY; VTDKVIAALLS; TSFSVRKNFIY |
| | HLA-A0201 | MLLIFSFFL; LLIFSFFLI; FLNGFPLNA; QLIDLNTGV; FVNMDLEFV; ILPFTSFSV; YLQDELKNL; LLSENEAGV; VVSESNFEI; KIQEDINEI; YVDSKMILA; NLQKIDPKV; KVITIYNAV; LMLPEDQKL; SLTKENAGL; SLEDLQEQL; LIFLNGFPL; KMDSGKAKL; KLDSAEDNL; FILSENKIL; NTYEQIVGI; KASVDVFSI; SSAELIAKV; KIDTELDNI; KILPFTSFS; ILTGYIIKS; MSIDSSSPV; LVTDKVIAA; VTDKVIAAL; ELIAKVITI; QILNKLENL; FLEVIDPIT; NITETIENL; QIVGIGEFL; SSSPVFLEV; KQLQIKESL; SLYVDSKMI; LQDELKNLV; NLDKAQQKL; STNTYEQIV; KILPFTSFSV; KMLLIFSFFL; MLLIFSFFLI; FLIFLNGFPL; YLQDELKNLV; ALLSENEAGV; LIFSFFLIFL; TMSIDSSSPV; YVDSKMILAA; ILNKLENLKV; SIDSSSPVFL; ILDVNTLKKV; LQLIDLNTGV; KVVSESNFEI; KLKDFVNMDL; SLYVDSKMIL; SLVTDKVIAA; LVTDKVIAAL; ILSGNIESDI; KISKSNNNEV; RLAKFSPKNL; SLNEDLDKDL; GIYEREKDLV; GLSRVYSQWA; VTDKVIAALL; |

| | | |
|---|---|---|
| | | NLVILDVNTL; FSVRKNFIYL; RLKESTQQGI; NLGTLQLIDL; GIGEFLARPL; QIVGIGEFLA; KMLLIFSFFLI; YVDSKMILAAV; LLIFSFFLIFL; YLQDELKNLVI; FLEVIDPITNL; FIDDQDKKASV; QLIDLNTGVRL; KLDSAEDNLDV; TTMSIDSSSPV; TLQLIDLNTGV; VILDVNTLKKV; SLVTDKVIAAL; ILSENKILPFT; KMDSGKAKLQI; KLDGKKEFKPV; QILNKLENLKV; SQWAGKTQIFI; KKMLLIFSFFL; GIYEREKDLVV; SLYVDSKMILA; FIYLQDELKNL; KLQILNKLENL; LIAKVITIYNA; LVTDKVIAALL; FFLIFLNGFPL; MSIDSSSPVFL; ILNKLENLKVV; VIDPITNLGTL; SLGDLNNDKNL; LIFLNGFPLNA; SISSKSELDSI; VIAALLSENEA; FEINKNSSLYV; FTSFSVRKNFI; KVDKEKLKDFV |
| | HLA-A0301 | RVYSQWAGK; RILTGYIIK; ILNKLENLK; KTQIFIPLK; ILDVNTLKK; VILDVNTLK; TQIFIPLKK; KAKLQILNK; RSSAELIAK; KLDGKKEFK; MILAAVRDK; KVDKQLQIK; GFYIEPALK; LIAKVITIY; RELSKASSK; FIYLQDELK; KSNNNEVGK; AVYRGDLDY; KLDSKLDGK; KSKDGKVSK; PLNARKVDK; NSNSSYYGK; EINKNSSLY; KVKEEEITK; DLNTGVRLK; ITIYNAVYR; NLRRILTGY; LSKASSKEK; NSNSNSSYY; KMLLIFSFF; VVIKMDSGK; KASSKEKSK; DSNAWRLAK; KTQIFIPLKK; VILDVNTLKK; KMILAAVRDK; SVDVFSISSK; LVILDVNTLK; AWRLAKFSPK; QILNKLENLK; AINLQKIDPK; LPFTSFSVRK; ILPFTSFSVR; SYYGKYFINR; ALDLDRELSK; LVVIKMDSGK; ITDIQGETHK; KVITIYNAVY; KGFYIEPALK; VITIYNAVYR; RRILTGYIIK; AVYRGDLDYY; IDLNTGVRLK; SKLDGKKEFK; AINLDKAQQK; ELSKASSKEK; IEPALKSLTK; LNGFPLNARK; LTNSNSNSSY; NMDLEFVNYK; EINKEKNLPK; SLEDLQEQLK; SNSNSSYYGK; VIKMDSGKAK; KLDSKLDGKK; LLIFSFFLIF; IVGIGEFLAR; FPLNARKVDK; SRVYSQWAGK; ASSKEKSKVK; FLNGFPLNAR; ASLGDLNNDK; SQVDAKKKEK; VYRGDLDYYK; KKLDSKLDGK; EVGKLSPLDK; VDNKAINLQK; KLSPLDKPSY; KNSSLYVDSK; FLNGFPLNARK; ILPFTSFSVRK; KILPFTSFSVR; ILNLRRILTGY; AVYRGDLDYYK; SSYYGKYFINR; LLKSDGKVSK; ASVDVFSISSK; VVSESNFEINK; LVILDVNTLKK; TSFSVRKNFIY; LSRVYSQWAGK; LTNSNSNSSYY; KVITIYNAVYR; LIDLNTGVRLK; ILDVNTLKKVK; KIQEDINEINK; NSNSNSSYYGK; KAINLDKAQQK; NLVILDVNTLK; NAWRLAKFSPK; KVKEEEITKGK; KEFKPVSEVEK; RVYSQWAGKTQ; LSKASSKEKSK; KASSKEKSKVK; KLPEDKKLDSK; VVIKMDSGKAK; KMLLIFSFFLI; EVDNKAINLQK; ELDKKAINLDK; KAINLQKIDPK |
| | HLA-A1101 | KTQIFIPLK; RVYSQWAGK; TQIFIPLKK; RSSAELIAK; VILDVNTLK; NSNSSYYGK; VVIKMDSGK; RILTGYIIK; KSNNNEVGK; DSNAWRLAK; ITIYNAVYR; KAKLQILNK; TSLNEDLDK; ILNKLENLK; NSSLYVDSK; MILAAVRDK; KSKDGKVSK; KVKEEEITK; FIYLQDELK; KVDKQLQIK; ILDVNTLKK; KATDEEHKK; SLGDLNNDK; AVYRGDLDY; STQQGIQRY; GFYIEPALK; NLMLPEDQK; PSYDDIDSK; KASSKEKSK; KLDGKKEFK; LSKASSKEK; SESNFEINK; KLDSKLDGK; SSKEKSKVK; KPGDVSSPK; QVDAKKKEK; VSEVEKLDK; DVSSPKVDK; NSNSNSSYY; ITETIENLR; KVIAALLSE; MDLEFVNYK; KTQIFIPLKK; SVDVFSISSK; VILDVNTLKK; AINLQKIDPK; LVILDVNTLK; TTSLNEDLDK; AINLDKAQQK; ASLGDLNNDK; VSESNFEINK; AVYRGDLDYY; QILNKLENLK; KMILAAVRDK; SLEDLQEQLK; ALDLDRELSK; GIQRYGIYER; ASSKEKSKVK; NMDLEFVNYK; LVVIKMDSGK; SNSNSSYYGK; SYYGKYFINR; KVITIYNAVY; KGFYIEPALK; ITDIQGETHK; EINKEKNLPK; LPFTSFSVRK; VITIYNAVYR; IVGIGEFLAR; KLDSKLDGKK; VIKMDSGKAK; IQEDINEINK; LTNSNSNSSY; EVGKLSPLDK; SQVDAKKKEK; YIIKSFDYDR; DVQRDTVREK; KNSSLYVDSK; PVSEVEKLDK; LTKENAGLSR; ILPFTSFSVR; FLNGFPLNAR; AVYRGDLDYYK; ASVDVFSISSK; SSYYGKYFINR; |

|  |  | VVSESNFEINK; LVILDVNTLKK; NSNSNSSYYGK; KIQEDINEINK; KAINLQKIDPK; TIENLRDQLEK; KVITIYNAVYR; TSFSVRKNFIY; VVIKMDSGKAK; ILPFTSFSVRK; KAINLDKAQQK; KILPFTSFSVR; FLNGFPLNARK; QTTSLNEDLDK; LQILNKLENLK; NAWRLAKFSPK; RASLGDLNNDK; VNMDLEFVNYK; LTNSNSNSSYY; EVDNKAINLQK; ISKSNNNEVGK; KASSKEKSKVK; LIDLNTGVRLK; KVKEEEITKGK; KSKVKEEEITK; KSELDSILNLR; NLVILDVNTLK; NLDEFILSENK; YIEPALKSLTK; FVNMDLEFVNY; EALDLDRELSK; LSRVYSQWAGK; LSKASSKEKSK; ESLEDLQEQLK; FINRFIDDQDK; KNFIYLQDELK; SGKAKLQILNK; AGKTQIFIPLK; SLTKENAGLSR; KEFKPVSEVEK; QIVGIGEFLAR; ILDVNTLKKVK; LIFSFFLIFLN; KLPEDKKLDSK |
| HLA-A2402 | QWAGKTQIF; KMLLIFSFF; DYDRSSAEL; VFLEVIDPI; DFVNMDLEF; FFLIFLNGF; LYVDSKMIL; EFILSENKI; IFSFFLIFL; SFSVRKNFI; LLIFSFFLI; IYEREKDLV; GYIIKSFDY; KKMLLIFSF; YYGKYFINRF; DYDRSSAELI; QWAGKTQIFI; IYLQDELKNL; SFFLIFLNGF; IYNAVYRGDL; KFSPKNLDEF; KMLLIFSFFL; FYIEPALKSL; IYEREKDLVV; KKMLLIFSFF; MLLIFSFFLI; SYYGKYFINRF; YYGKYFINRFI; IYEREKDLVVI; KMLLIFSFFLI; YYKGFYIEPAL; TYEQIVGIGEF; IYLQDELKNLV; PYDSTNTYEQI; VYSQWAGKTQI; KFSPKNLDEFI; FFLIFLNGFPL; SFSVRKNFIYL |
| HLA-A2902 | GYIIKSFDY; FSVRKNFIY; AVYRGDLDY; LIAKVITIY; EFVNYKGPY; VITIYNAVY; EINKNSSLY; NMDLEFVNY; LIFSFFLIF; DFVNMDLEF; NSNSNSSYY; ENAGLSRVY; VYRGDLDYY; NLRRILTGY; KVITIYNAVY; SFSVRKNFIY; VNMDLEFVNY; ELIAKVITIY; FLIFLNGFPL; AVYRGDLDYY; KLSPLDKPSY; TGYIIKSFDY; FEINKNSSLY; SFFLIFLNGF; LLIFSFFLIF; FVNMDLEFVNY; TSFSVRKNFIY; DLEFVNYKGPY; YNAVYRGDLDY; MLLIFSFFLIF; FILSENKILPF; LTNSNSNSSYY; ILNLRRILTGY; LTGYIIKSFDY; FSFFLIFLNGF; SYYGKYFINRF; NFEINKNSSLY |
| HLA-A6801 | ITIYNAVYR; ITETIENLR; FIYLQDELK; NSNSSYYGK; ESTQQGIQR; NSSLYVDSK; ELDSILNLR; ETHKADQDK; IIKSFDYDR; DINEINKEK; NEAGVNFAR; DSNAWRLAK; NSNSNSSYY; VVIKMDSGK; LPFTSFSVR; EINKNSSLY; EIESQVDAK; RVYSQWAGK; EAGDENQKR; KTQIFIPLK; DVSSPKVDK; TQIFIPLKK; DVNTLKKVK; EIEKQIEIK; MILAAVRDK; FSFFLIFLN; VILDVNTLK; NLMLPEDQK; ILNKLENLK; MDLEFVNYK; EPALKSLTK; DNKAINLQK; ESQVDAKKK; YYGKYFINR; DSKEEVDNK; NGFPLNARK; NARKVDKEK; LIDLNTGVR; DLNTGVRLK; LSKASSKEK; TTMSIDSSS; DVFSISSKS; DYEALDLDR; FSVRKNFIY; ELLKSKDGK; IQRYGIYER; LIAKVITIY; EKATDEEHK; DIDSLVTDK; SKMILAAVR; TSLNEDLDK; PSYDDIDSK; NITETIENLR; YIIKSFDYDR; LVILDVNTLK; VITIYNAVYR; DSKMILAAVR; TTSLNEDLDK; SYYGKYFINR; QLIDLNTGVR; EIESQVDAKK; NFIYLQDELK; LTKENAGLSR; FLNGFPLNAR; LVVIKMDSGK; SAEDNLDVQR; ELDSILNLRR; ENEAGVNFAR; ELSKASSKEK; ILPFTSFSVR; EIEKQIEIKK; LPFTSFSVRK; EFKPVSEVEK; LTNSNSNSSY; EVEKLDKISK; SVDVFSISSK; DVQRDTVREK; EINKEKNLPK; EVGKLSPLDK; KTQIFIPLKK; IVGIGEFLAR; NSNSSYYGKY; GIQRYGIYER; SNSNSSYYGK; NMDLEFVNYK; ESTQQGIQRY; QILNKLENLK; EDINEINKEK; ELIAKVITIY; ITDIQGETHK; VILDVNTLKK; TNSNSNSSYY; MSIDSSSPVF; DVFSISSKSE; VSESNFEINK; EKATDEEHKK; SLEDLQEQLK; TGYIIKSFDY; NLQKIDPKVK; KGFYIEPALK; NLDVQRDTVR; SSYYGKYFINR; NSNSNSSYYGK; KVITIYNAVYR; DSAEDNLDVQR; DITDIQGETHK; QIVGIGEFLAR; TSFSVRKNFIY; NLVILDVNTLK; QTTSLNEDLDK; LVILDVNTLKK; LTNSNSNSSYY; DLVVIKMDSGK; EVDNKAINLQK; FLNGFPLNARK; AVYRGDLDYYK; ASVDVFSISSK; VVSESNFEINK; ESLEDLQEQLK; TIENLRDQLEK; |

| | | |
|---|---|---|
| | | EIEKQIEIKKR; EIKKRDEELLK; SNITETIENLR; EALDLDRELSK; FINRFIDDQDK; KILPFTSFSVR; ILPFTSFSVRK; EIESQVDAKKK; NAVYRGDLDYY; QGIQRYGIYER; ISKSNNNEVGK; DSKLDGKKEFK; ESQVDAKKKEK; VVIKMDSGKAK; NAWRLAKFSPK; FVNMDLEFVNY; SLTKENAGLSR; NARKVDKEKLK; NLDEFILSENK; KSELDSILNLR; GYIIKSFDYDR; VNMDLEFVNYK; ETIENLRDQLE; KAINLDKAQQK; DVFSISSKSEL; FSFFLIFLNGF; SENEAGVNFAR; YIEPALKSLTK; MSIDSSSPVFL; FSVRKNFIYLQ; LQILNKLENLK; QLEKATDEEHK; LSRVYSQWAGK |
| | HLA-B0702 | SVRKNFIYL; KVKDQTTSL; KPVSEVEKL; RPLTNSNSN; KVSKDYEAL; GPYDSTNTY; DPITNLGTL; KPGDVSSPKV; SPKVDKQLQI; LVTDKVIAAL; SPKNLDEFIL; RPLTNSNSNS; ITKGKSRASL; RPLTNSNSNSS; SPVFLEVIDPI; LPEDKKLDSKL; DPKVKDQTTSL; AVRDKDDSNAW; DPITNLGTLQL; SPLDKPSYDDI |
| | HLA-B0801 | EIKKRDEEL; EIKKRDEELL; ITKGKSRASL; FLIFLNGFPL; IAKVITIYNAV; DPKVKDQTTSL; YYKGFYIEPAL; NLRRILTGYII |
| | HLA-B1501 | EQIVGIGEF; AVYRGDLDY; KMLLIFSFF; FSVRKNFIY; STQQGIQRY; LSENKILPF; TNSNSNSSY; LIAKVITIY; QGIQRYGIY; YGKYFINRF; KVKDQTTSL; NLRRILTGY; VITIYNAVY; LTGYIIKSF; LIFSFFLIF; LVILDVNTL; MSIDSSSPV; KQLQIKESL; LIFLNGFPL; NSNSNSSYY; ENAGLSRVY; SQWAGKTQIF; MSIDSSSPVF; KVITIYNAVY; ILSENKILPF; LTNSNSNSSY; KLSPLDKPSY; ILTGYIIKSF; QQGIQRYGIY; LLIFSFFLIF; ELIAKVITIY; AVYRGDLDYY; FLIFLNGFPL; LQIKESLEDL; KENAGLSRVY; YEQIVGIGEF; EQIVGIGEFL; NLKVVSESNF; LQILNKLENL; LEFVNYKGPY; TNSNSNSSYY; FTSFSVRKNF; FEINKNSSLY; KLKDFVNMDL; TMSIDSSSPV; RLKESTQQGI; ITKGKSRASL; LVTDKVIAAL; VNMDLEFVNY; TQQGIQRYGIY; TMSIDSSSPVF; YSQWAGKTQIF; LLSENEAGVNF; ILNLRRILTGY; KSKDGKVSKDY; LTNSNSNSSYY; FSFFLIFLNGF; RILTGYIIKSF; FILSENKILPF; MLLIFSFFLIF; YNAVYRGDLDY; FVNMDLEFVNY; TSFSVRKNFIY; PLTNSNSNSSY; QQKLDSAEDNL; AVRDKDDSNAW; QLIDLNTGVRL; YLQDELKNLVI |
| | HLA-B2705 | WRLAKFSPK; RRILTGYII; YRGDLDYYK; KKMLLIFSF; QRYGIYERE; KQLQIKESL; RRILTGYIIK; QRYGIYEREK; SRVYSQWAGK; KRDEELLKSK; SQWAGKTQIF; KKMLLIFSFF; WRLAKFSPKN; RRILTGYIIKS; YRGDLDYYKGF; WRLAKFSPKNL; LRRILTGYIIK |
| | HLA-B3501 | GPYDSTNTY; DFVNMDLEF; EFVNYKGPY; DPITNLGTL; EALDLDREL; FFLIFLNGF; LIFSFFLIF; LVILDVNTL; VITIYNAVY; FSVRKNFIY; NSNSNSSYY; LPEDQKLPE; LIAKVITIY; LPKPGDVSS; SIDSSSPVF; LSENKILPF; LPFTSFSVR; EINKNSSLY; ENAGLSRVY; LIFLNGFPL; MSIDSSSPVF; NAVYRGDLDY; KVITIYNAVY; LTNSNSNSSY; ESTQQGIQRY; LLIFSFFLIF; SPKNLDEFIL; FLIFLNGFPL; LVTDKVIAAL; ILSENKILPF; AVYRGDLDYY; SQWAGKTQIF; LSENEAGVNF; ELIAKVITIY; NLVILDVNTL; EDLDKDLTTM; NAVYRGDLDYY; FVNMDLEFVNY; YSQWAGKTQIF; YKGPYDSTNTY; TMSIDSSSPVF; SPVFLEVIDPI; FILSENKILPF; MLLIFSFFLIF; DPITNLGTLQL; LTNSNSNSSYY; FSFFLIFLNGF; WAGKTQIFIPL; FFLIFLNGFPL; TKENAGLSRVY; DPKVKDQTTSL; DLEFVNYKGPY; LPEDKKLDSKL; LLSENEAGVNF; MSIDSSSPVFL; NFEINKNSSLY |
| | HLA-B4403 | SENEAGVNF; SELDSILNL; REIEKQIEI; GEFLARPLT; AEDNLDVQR; EEHKKEIES; KEIESQVDA; AELIAKVIT; KESLEDLQE; EEVDNKAIN; SENKILPFT; NEINKEKNL; TELDNIHES; EEVDNKAINL; EELDKKAINL; SENKILPFTS; GEFLARPLTN; AELIAKVITI; SENEAGVNFA; REKDLVVIKM; SEVEKLDKIS; FEINKNSSLY; KEKLKDFVNM; LEFVNYKGPY; EEITKGKSRA; YEQIVGIGEF; SELDSILNLR; AELIAKVITIY; EEITKGKSRAS; EEVDNKAINLQ; SENKILPFTSF; |

| | | |
|---|---|---|
| | | SENEAGVNFAR; KESLEDLQEQL; GEFLARPLTNS; EEHKKEIESQV; SELDSILNLRR; FEINKNSSLYV; DEFILSENKIL; SESNFEINKNS; EELLKSKDGKV; AEDNLDVQRDT; KESTQQGIQRY; EELDKKAINLD |
| | HLA-B5101 | LPFTSFSVR; LPKPGDVSS; DPITNLGTL; LPFTSFSVRK; LPKPGDVSSP; SPVFLEVIDPI; LPFTSFSVRKN; LPKPGDVSSPK; SPLDKPSYDDI |
| | HLA-B5701 | STQQGIQRY; ; NAGLSRVYSQW |
| NP_212375.1 \| glycerol kinase (glpK) [Borrelia burgdorferi B31]; Seq27 | HLA-A0101 | ETTALGAAY; SVSDNGGVY; YLAGLATGY; SSDAEALAS; VSDNGGVYF; SSVSDNGGVY; FAQKEFTQIY; VSDNGGVYFV; ITETTALGAAY; ASSVSDNGGVY; LTQKKEHATDY; SSDAEALASSV |
| | HLA-A0201 | KIMWILDNV; YVLEGSVFI; ALFGAEIPI; LLMQFQADL; VLDSYFSGT; KTLEWDDEL; MVFDKNANI; ILPNEIDAI; WILDNVEGA; GVYFVPAFV; KTDKALFGA; IILEKTGLV; SQLGVITEA; KLLENWNKA; QIYPQPSWV; YILSIDQGT; GMAKNTYGT; EIWGSQLGV; VITEAMANA; LLENWNKAV; LMQFQADLL; GIAGDQFAA; TLEWDDELL; FQSFDILNT; KLLTSIAWG; SIAFQSFDI; QLGVITEAM; FSGTKIMWI; TLLLNIKTL; TIDTWILWNL; ALGAAYLAGL; LLMQFQADLL; ALFGAEIPIA; FIGGAVIQWL; FQADLLECKV; KLLENWNKAV; KTLEWDDELL; LLSILNVPRA; GLATGYWQSA; LVLDSYFSGT; FVPAFVGLGA; KITETTALGA; FQSFDILNTM; FAATFGQACL; IILEKTGLVL; AMVFDKNANI; NLLMQFQADL; RILPNEIDAI; SIPNFEIQEL; SVTYVLEGSV; KIILEKTGLV; VSDNGGVYFV; FSGTKIMWIL; WQSAEEIVSL; SIAFQSFDIL; TQIYPQPSWV; SQLGVITEAM; NVPRAILPEL; VLDSYFSGTKI; FLTVNIGKEPI; IMWILDNVEGA; FTQIYPQPSWV; FQADLLECKVV; ILPNEIDAIGI; SVSDNGGVYFV; NTYGTGCFLTV; TLEWDDELLSI; GTIDTWILWNL; ILPELKESSTI; VITEAMANARI; VIWEKNTGKPI; KTDKALFGAEI; GVYFVPAFVGL; TMKKSIPNFEI; YLAGLATGYWQ; LLSILNVPRAI; GLGAPHWDSYA; ELLSILNVPRA; TALGAAYLAGL; NLLMQFQADLL; LLECKVVRPKI; VLEGSVFIGGA; IISHDKLLTSI; WQVDKIFEPSM; KSVTYVLEGSV; SAEEIVSLWQV; ALGAAYLAGLA; KITETTALGAA; SQLGVITEAMA; GLVLDSYFSGT |
| | HLA-A0301 | KIFEPSMPK; ILWNLTQKK; ATFGQACLK; TSIAWGRKK; IVSLWQVDK; VIWEKNTGK; SSRAMVFDK; LTSIAWGRK; WILWNLTQK; YLAGLATGY; CFLTVNIGK; STIYGKTDK; ATFGQACLKK; LLTSIAWGRK; IIGITRGSTK; VLDSYFSGTK; AATFGQACLK; KLLTSIAWGR; QACLKKGMAK; LTSIAWGRKK; QSFDILNTMK; LLECKVVRPK; WILWNLTQKK; TVIWEKNTGK; MVFDKNANIK; ASRTLLLNIK; SSTIYGKTDK; TSSRAMVFDK; AYLAGLATGY; SFDILNTMKK; NANIKGFAQK; IVWQDRRTAK; GCFLTVNIGK; TWILWNLTQK; RTAKICDQLK; GLGAPHWDSY; DKIFEPSMPK; LLLNIKTLEW; SIDQGTTSSR; ELLSILNVPR; WLRDGLEFFR; EIVSLWQVDK; KLLTSIAWGRK; RTAKICDQLKK; LLTSIAWGRKK; TIIGITRGSTK; QSFDILNTMKK; TTSSRAMVFDK; LVLDSYFSGTK; FAATFGQACLK; GQACLKKGMAK; LLENWNKAVGK; FQSFDILNTMK; KNANIKGFAQK; AIVWQDRRTAK; MQFQADLLECK; AATFGQACLKK; QIYPQPSWVEH; AMVFDKNANIK; WLRDGLEFFRK; AAYLAGLATGY |
| | HLA-A1101 | KIFEPSMPK; ATFGQACLK; SSRAMVFDK; TSIAWGRKK; LTSIAWGRK; STIYGKTDK; IVSLWQVDK; WILWNLTQK; ATDYSNASR; ILWNLTQKK; VIWEKNTGK; TAKICDQLK; ITEAMANAR; FQADLLECK; SVSDNGGVY; KTGLVLDSY; KESSTIYGK; SFDILNTMK; CFLTVNIGK; SMPKNQKEK; ANIKGFAQK; TFGQACLKK; LLSILNVPR; GSTKAHITR; VWQDRRTAK; ATFGQACLKK; TSSRAMVFDK; MVFDKNANIK; TVIWEKNTGK; QSFDILNTMK; RTAKICDQLK; AATFGQACLK; LTSIAWGRKK; VLDSYFSGTK; ASRTLLLNIK; QACLKKGMAK; SSTIYGKTDK; NANIKGFAQK; LLTSIAWGRK; IVWQDRRTAK; WILWNLTQKK; IIGITRGSTK; TWILWNLTQK; TAKICDQLKK; SFDILNTMKK; |

| | | |
|---|---|---|
| | | SIDQGTTSSR; KLLTSIAWGR; QFQADLLECK; VITEAMANAR; EIVSLWQVDK; LLECKVVRPK; PSMPKNQKEK; GCFLTVNIGK; TTSSRAMVFDK; QSFDILNTMKK; LVLDSYFSGTK; TIIGITRGSTK; RTAKICDQLKK; KLLTSIAWGRK; MQFQADLLECK; TTVIWEKNTGK; AATFGQACLKK; AIVWQDRRTAK; KICDQLKKEGK; AMVFDKNANIK; GQACLKKGMAK; GVITEAMANAR; TGCFLTVNIGK; NASRTLLLNIK; FAATFGQACLK; NVPRAILPELK; FQSFDILNTMK; AAYLAGLATGY; TWILWNLTQKK; DTWILWNLTQK; SIPNFEIQELR; LLTSIAWGRKK; LSIDQGTTSSR; LLENWNKAVGK; WLRDGLEFFRK; KNANIKGFAQK; ASQNNLLMQFQ |
| | HLA-A2402 | QWLRDGLEF; TYVLEGSVF; DYSNASRTL; IWGSQLGVI; IYGKTDKAL; CFGTIDTWI; IYPQPSWVE; YWQSAEEIV; EWDDELLSI; YFVPAFVGL; TYGTGCFLT; GYWQSAEEI; IYGKTDKALF; QWLRDGLEFF; TYVLEGSVFI; SYARGTIIGI; DYSNASRTLL; TYGTGCFLTV; VWQDRRTAKI; VYFVPAFVGL; SWVEHDPTEI; YFSGTKIMWI; IWEKNTGKPI; SYFSGTKIMW; CFGTIDTWIL; GFAQKEFTQI; IYNAIVWQDR; DYSNASRTLLL; SYFSGTKIMWI; YWQSAEEIVSL; YFSGTKIMWIL; AYLAGLATGYW; VFIGGAVIQWL; VFDKNANIKGF |
| | HLA-A2902 | YLAGLATGY; SVSDNGGVY; ETTALGAAY; ELKESSTIY; QWLRDGLEF; FAQKEFTQIY; AYLAGLATGY; SVSDNGGVYF; SSVSDNGGVY; VTYVLEGSVF; AWGRKKSVTY; GFAQKEFTQIY; ITETTALGAAY; AAYLAGLATGY; IWEKNTGKPIY |
| | HLA-A6801 | DAIGITNQR; ATFGQACLK; LLTSIAWGR; ITEAMANAR; STIYGKTDK; NAIVWQDRR; TAKICDQLK; ETTALGAAY; TSIAWGRKK; LTSIAWGRK; IVSLWQVDK; LLSILNVPR; ATDYSNASR; SSRAMVFDK; WILWNLTQK; EPSMPKNQK; GSTKAHITR; NTYGTGCFL; ESIAFQSFD; DLLECKVVR; TTSSRAMVF; FDILNTMKK; GGAVIQWLR; KIFEPSMPK; YNAIVWQDR; TTALGAAYL; CFLTVNIGK; PNFEIQELR; FQADLLECK; APHWDSYAR; DNVEGARQR; YLAGLATGY; ILDNVEGAR; ENWNKAVGK; MVFDKNANIK; HATDYSNASR; TVIWEKNTGK; QSFDILNTMK; TSSRAMVFDK; ATFGQACLKK; EIVSLWQVDK; RTAKICDQLK; ELLSILNVPR; IPNFEIQELR; VITEAMANAR; AATFGQACLK; NANIKGFAQK; LLTSIAWGRK; WLRDGLEFFR; LTSIAWGRKK; TAKICDQLKK; YNAIVWQDRR; WILDNVEGAR; IYNAIVWQDR; TWILWNLTQK; SIDQGTTSSR; NTYGTGCFLT; SSTIYGKTDK; IVWQDRRTAK; WILWNLTQKK; GAPHWDSYAR; TTALGAAYLA; IDAIGITNQR; IGGAVIQWLR; QACLKKGMAK; ETTALGAAYL; EGKDKIILEK; TTSSRAMVFD; KLLTSIAWGR; DKIFEPSMPK; TTSSRAMVFDK; QSFDILNTMKK; FAATFGQACLK; TTVIWEKNTGK; SIPNFEIQELR; EIDAIGITNQR; TIIGITRGSTK; FIGGAVIQWLR; ESSTIYGKTDK; LSIDQGTTSSR; GVITEAMANAR; DTWILWNLTQK; RTAKICDQLKK; MWILDNVEGAR; IYNAIVWQDRR; MQFQADLLECK; YARGTIIGITR; NVPRAILPELK; LVLDSYFSGTK; ELKESSTIYGK; EHATDYSNASR; NASRTLLLNIK; TGCFLTVNIGK; LLTSIAWGRKK; FQSFDILNTMK; EPSMPKNQKEK; QADLLECKVVR; AATFGQACLKK; TWILWNLTQKK; ETTALGAAYLA; EEIVSLWQVDK; NTMKKSIPNFE; AIVWQDRRTAK; PIYNAIVWQDR; LGAPHWDSYAR; TIYGKTDKALF; ILDNVEGARQR; QWLRDGLEFFR |
| | HLA-B0702 | KAHITRAAL; KPIYNAIVW; VPRAILPEL; MPKNQKEKL; RAALESIAF; RPKITETTA; VPAFVGLGA; AAYLAGLAT; IPNFEIQEL; EPIISHDKL; LPELKESST; IAWGRKKSV; RPKITETTAL; MPKNQKEKLL; LPELKESSTI; DPTEIWGSQL; VPAFVGLGAP; TKAHITRAAL; SVSDNGGVYF; EPIISHDKLL; IPIAGIAGDQF; GARQRAENGEL; VPAFVGLGAPH; APHWDSYARGT; FFRKSSDAEAL; WGRKKSVTYVL; IVWQDRRTAKI; RPKITETTALG; STKAHITRAAL |
| | HLA-B0801 | KAHITRAAL; MPKNQKEKL; RKKSVTYVL; RPKITETTAL; |

| | | |
|---|---|---|
| | | FRKSSDAEAL; MPKNQKEKLL; WGRKKSVTYV; WGRKKSVTYVL; FFRKSSDAEAL |
| | HLA-B1501 | AQKEFTQIY; YLAGLATGY; RQRAENGEL; SVSDNGGVY; RAALESIAF; TMKKSIPNF; SQNNLLMQF; WLRDGLEFF; ELKESSTIY; TQIYPQPSW; ETTALGAAY; NQRETTVIW; WGRKKSVTY; ITRGSTKAH; TTSSRAMVF; SLWQVDKIF; QSFDILNTM; SVFIGGAVI; QSAEEIVSL; LGAPHWDSY; WQSAEEIVS; YSNASRTLL; GGVYFVPAF; IQWLRDGLEF; WQSAEEIVSL; TQKKEHATDY; SQLGVITEAM; SVSDNGGVYF; FQSFDILNTM; VTYVLEGSVF; SSVSDNGGVY; GLGAPHWDSY; YLAGLATGYW; FAQKEFTQIY; WEKNTGKPIY; YSNASRTLLL; IAGDQFAATF; RQRAENGELC; ALESIAFQSF; NIKGFAQKEF; ASQNNLLMQF; RQRAENGELCF; ITRAALESIAF; WQVDKIFEPSM; IQWLRDGLEFF; ASSVSDNGGVY; MAKNTYGTGCF; WQSAEEIVSLW; ITETTALGAAY; IVSLWQVDKIF; AAYLAGLATGY; GIAGDQFAATF; IAWGRKKSVTY; AALESIAFQSF; FQADLLECKVV; SVTYVLEGSVF; VIQWLRDGLEF; SSVSDNGGVYF; LTQKKEHATDY; AQKEFTQIYPQ; CLKKGMAKNTY; STKAHITRAAL; GFAQKEFTQIY |
| | HLA-B2705 | RKKSVTYVL; GRKKSVTYVL; QRAENGELCF; RRTAKICDQL; TRAALESIAF; QRETTVIWEK; LRDGLEFFRK; ARGTIIGITR; RRTAKICDQLK; GQACLKKGMAK; RQRAENGELCF |
| | HLA-B3501 | RAALESIAF; IPNFEIQEL; ETTALGAAY; SVSDNGGVY; YPQPSWVEH; LPNEIDAIG; IAFQSFDIL; YLAGLATGY; FAATFGQAC; YFVPAFVGL; IAGDQFAAT; QSFDILNTM; IAGIAGDQF; KPIYNAIVW; HATDYSNAS; MPKNQKEKL; SLWQVDKIF; RAENGELCF; ELKESSTIY; EKNTGKPIY; TYVLEGSVF; APHWDSYAR; QLGVITEAM; LESIAFQSF; YVLEGSVFI; GASQNNLLM; LGAPHWDSY; EAMANARIL; VPRAILPEL; AAYLAGLAT; TTSSRAMVF; TLEWDDELL; EPIISHDKL; VSDNGGVYF; FAQKEFTQIY; IAGDQFAATF; FAATFGQACL; QPSWVEHDPT; LPNEIDAIGI; FQSFDILNTM; SVSDNGGVYF; TETTALGAAY; RPKITETTAL; SSVSDNGGVY; MPKNQKEKLL; EPIISHDKLL; VSLWQVDKIF; TRAALESIAF; NGGVYFVPAF; IQWLRDGLEF; DPTEIWGSQL; SQLGVITEAM; DSYFSGTKIM; QVDKIFEPSM; LPELKESSTI; LPELKESSTIY; IPIAGIAGDQF; IAWGRKKSVTY; VPAFVGLGAPH; LPNEIDAIGIT; AAYLAGLATGY; MAKNTYGTGCF; ITETTALGAAY; AALESIAFQSF; TALGAAYLAGL; IVSLWQVDKIF; QPSWVEHDPTE; ASSVSDNGGVY; WQVDKIFEPSM; ITRAALESIAF; SVTYVLEGSVF; MANARILPNEI; SSVSDNGGVYF; YFSGTKIMWIL; APHWDSYARGT; FFRKSSDAEAL; GASQNNLLMQF |
| | HLA-B4403 | AEIPIAGIA; EEIVSLWQV; NEIDAIGIT; KEFTQIYPQ; AEALASSVS; AEEIVSLWQ; QELRVDGGA; TEIWGSQLG; DELLSILNV; AEIPIAGIAG; TEIWGSQLGV; AEEIVSLWQV; NEIDAIGITN; TETTALGAAY; EEIVSLWQVD; AENGELCFGT; KEFTQIYPQP; QELRVDGGAS; KEFTQIYPQPS; AEIPIAGIAGD; NEIDAIGITNQ; TEIWGSQLGVI; KEPIISHDKLL; AENGELCFGTI; TETTALGAAYL; KEHATDYSNAS; GELCFGTIDTW |
| | HLA-B5101 | VPAFVGLGA; VPRAILPEL; LPNEIDAIG; LPELKESSTI; EPIISHDKLL; LPNEIDAIGIT; IPIAGIAGDQF; IPNFEIQELRV |
| | HLA-B5701 | GTIDTWILW; SAEEIVSLW; KTGLVLDSY; LCFGTIDTW; FTQIYPQPSW; QSAEEIVSLW; TNQRETTVIW; FAQKEFTQIY; ITNQRETTVIW; TGKPIYNAIVW,; DSYFSGTKIMW; KNQKEKLLENW |
| NP_212342.1 | hypothetical protein BB0208 [Borrelia | HLA-A0101 | |
| | | YIEEKVLKY; NTSLEECYY; YSQEKKDIY; ESSIFKERY; LIEIQNSPY; NTKKLGTTY; ESFIDALEY; LSFYPNIEY; SSKNISFNY; ETYGWIAFY; QTEDFFISF; TLSLNEIFY; FQDTLKCYLY; GSSKNISFNY; FLSFYPNIEY; LIEIQNSPYY; KTLSLNEIFY; KTIKKDSEFY; ATLKKHINKY; YLEEEILALKY; NIETYGWIAFY; KSAFLEIIMHY; NLNTKKLGTTY; |

| burgdorferi | | ETYGWIAFYIY; ISFDKTNFNPY |
|---|---|---|
| B31]; Seq28 | HLA-A0201 | FISDILLYL; MLILTYWPV; YLEEEILAL; FLEIIMHYV; KLLPAMCYL; YLIYCENLV; YLDENKITI; LLPAMCYLI; QTIDILVAV; YLYVIEKTI; KLINIKEKI; SISSQLYKL; YIKSAFLEI; YLLIKSLKT; FFSDFTQYA; TLKCYLYVI; FISFFKTKL; FQDTLKCYL; ILIKXYKYL; SLLEFISFF; IILNFSSNI; KTFEIQNQI; AIKKWILEI; YFLSFYPNI; SLEQTIDIL; QLDYQKSYL; ILALKYNEI; SFIDALEYI; LLYLDENKI; LALKYNEIL; FQTEDFFIS; RMLILTYWPV; KLLPAMCYLI; YLTWFFKDKI; LIYCENLVEL; YLRALKTLSL; SLEQTIDILV; AFLEIIMHYV; CLSKNIFIKI; IQNQIFVYFL; ILNFSSNINI; KXYKYLEEEI; ILTYWPVGCL; QLDYQKSYLL; HINKYIEEKV; ETYGWIAFYI; KFISDILLYL; KLKSSNFLKL; RILKIIIKNI; KLEKPEIIEL; ILALKYNEIL; YFFSDFTQYA; YIKSAFLEII; QLYKLEKPEI; ILLYLDENKI; AIKKWILEIV; KILNILNKNL; KILIKXYKYL; NQLDYQKSYL; KISNNWESFI; YGWIAFYIYA; IIIKNIRFNL; YSISSQLYKL; YLYVIEKTIT; SLLEFISFFK; LLEFISFFKT; FIQISKEANL; VLKYSISSQL; ALKTLSLNEI; RLIKLKRMLI; FSSNINIDSL; YLIYCENLVEL; FQDTLKCYLYV; KTFEIQNQIFV; FVYFLSFYPNI; SLLEFISFFKT; SAFLEIIMHYV; AMCYLIYCENL; IILNFSSNINI; KVLKYSISSQL; NQLDYQKSYLL; YIEEKVLKYSI; QLDYQKSYLLI; LLYLDENKITI; KIIIKNIRFNL; ALKYNEILNYL; KLEKPEIIELI; KXYKYLEEEIL; RLIKLKRMLIL; KLKSSNFLKLI; LILTYWPVGCL; FKFISDILLYL; KRMLILTYWPV; QLYKLEKPEII; ILNYLRALKTL; KQIILNFSSNI; LLPAMCYLIYC; YIYADNKDKKI; CLSKNIFIKIL; YKLEKPEIIEL; KLEDENEKKLL; YQKSYLLIKSL; SQLYKLEKPEI; IIIKNIRFNLI |
| | HLA-A0301 | |
| | | KLKSSNFLK; ILNYLRALK; ISFFKTKLK; LLEFISFFK; KLKQKINFK; KLGTTYKLK; CLSKNIFIK; LSLNEIFYK; YSISSQLYK; RMKEFFKSK; NIFIKILIK; ATLKKHINK; KIKEIITAK; EIFYKGSSK; HINKYIEEK; SIFKERYIK; KINPNQLDY; KKYLTWFFK; ITIPKKIIK; VIEKTITKK; ETYGWIAFY; LVAVRNRNK; KCYLYVIEK; ILNKNLKYR; YVIEKTITK; VFKSSLTNK; TLSLNEIFY; KFISDILLY; TLKKHINKY; SFYPNIEYK; ⟨illegible⟩ |

| | | |
|---|---|---|
| | | LKYSISSQLYK; EFYDSLATLKK; LLIKSLKTRSR; IFCQRMKEFFK; NFNPYESSIFK; ISFDKTNFNPY; NLNTKKLGTTY; YLDENKITIPK; QISKEANLNTK; ETYGWIAFYIY; SEFYDSLATLK; AFLEIIMHYVK; HFKFISDILLY; KYLEEEILALK; WIAFYIYADNK |
| | HLA-A1101 | SSQLYKLEK; ATLKKHINK; YSISSQLYK; SIFKERYIK; TSLEECYYK; LSLNEIFYK; LLEFISFFK; ISFFKTKLK; KLKSSNFLK; ILNYLRALK; YVIEKTITK; KKYLTWFFK; ITIPKKIIK; NTQNEAIKK; NIFIKILIK; SSKNISFNY; SFYPNIEYK; HINKYIEEK; AFYIYADNK; RMKEFFKSK; LVAVRNRNK; ETYGWIAFY; SYLLIKSLK; EIIMHYVKK; ISNDYEKEK; KLKQKINFK; KIKEIITAK; CLSKNIFIK; CQRMKEFFK; SQEKKDIYK; NPYESSIFK; EIFYKGSSK; KCYLYVIEK; YQKSYLLIK; VIEKTITKK; ILLYLDENK; LVELKNNRK; YIYADNKDK; KLGTTYKLK; NIRFNLIEK; TLSLNEIFY; YLTWFFKDK; LSFYPNIEY; SPYYSQEKK; KILIKXYKY; WILEIVRNK; NSPYYSQEK; AVRNRNKIK; KYIEEKVLK; SSLTNKESR; SDFTQYAPK; VFKSSLTNK; KFISDILLY; KINPNQLDY; TIDILVAVR; ALEYIEKHK; SAFLEIIMH;  SLLEFISFFK; KVFKSSLTNK; KSYLLIKSLK; TLSLNEIFYK; SSIFKERYIK; LSFYPNIEYK; KINFKKELMK; IAFYIYADNK; ISSQLYKLEK; KIKILNILNK; YVIEKTITKK; QIFVYFLSFY; LTNKESRLIK; KTLSLNEIFY; NTSLEECYYK; SLKTRSRILK; SAFLEIIMHY; ILNILNKNLK; KYSISSQLYK; FISFFKTKLK; YIYADNKDKK; YSQEKKDIYK; ILVAVRNRNK; KLKQKINFKK; KISNDYEKEK; FSDFTQYAPK; KITIPKKIIK; NTKKLGTTYK; EILNYLRALK; FIDALEYIEK; QTIDILVAVR; ISKEANLNTK; LATLKKHINK; SFFKTKLKQK; KTIKKDSEFY; GSSKNISFNY; KNIRFNLIEK; RSRILKIIIK; KYLTWFFKDK; LIKISNDYEK; TKKYLTWFFK; ATLKKHINKY; KLINIKEKIK; KNIFIKILIK; IYCENLVELK; FLEIIMHYVK; EFYDSLATLK; TYWPVGCLSK; YLEEEILALK; FIKILIKXYK; TEDFFISFDK; KSSLTNKESR; VAVRNRNKIK; KLIEIQNSPY; KYNEILNYLR; DILLYLDENK; NSPYYSQEKK; FVYFLSFYPN; KWILEIVRNK; LEFISFFKTK;  KTLSLNEIFYK; LTYWPVGCLSK; SISSQLYKLEK; KSLKTRSRILK; NSLLEFISFFK; ITKKYLTWFFK; KILNILNKNLK; QTEDFFISFDK; KINPNQLDYQK; LIYCENLVELK; ISFFKTKLQK; SLATLKKHINK; TLKCYLYVIEK; IQNSPYYSQEK; KTKLKQKINFK; SLTNKESRLIK; WIAFYIYADNK; TIPKKIIKIQK; SFIDALEYIEK; AFLEIIMHYVK; KSAFLEIIMHY; IFCQRMKEFFK; ISFDKTNFNPY; YLYVIEKTITK; FLSFYPNIEYK; SEFYDSLATLK; QISKEANLNTK; TYKLKSSNFLK; ISKEANLNTKK; LLEFISFFKTK; LVAVRNRNKIK; ETYGWIAFYIY; IFIKILIKXYK; KYLEEEILALK; YLDENKITIPK; NQIFVYFLSFY; KLIEIQNSPYY; NFNPYESSIFK; ESSIFKERYIK; LKYSISSQLYK; EFYDSLATLKK; AFYIYADNKDK; ELIKISNDYEK; FLEIIMHYVKK; AIKKWILEIVR; FFSDFTQYAPK; LVELKNNRKIK; KKYLTWFFKDK; RILKIIIKNIR; YIEKHKLINIK; KLLPAMCYLIY; VGCLSKNIFIK; CQRMKEFFKSK; SKNIFIKILIK; TIDILVAVRNR; YAPKDFQDTLK |
| | HLA-A2402 | DYQKSYLLI; TYGWIAFYI; IYKQIILNF; TYKLKSSNF; KFQTEDFFI; EYIEKHKLI; YFLSFYPNI; NYLRALKTL; XYKYLEEEI; KYNEILNYL; IYCENLVEL; KYSISSQLY; RYIKSAFLE; SFNYFFSDF; DYEKEKNAF; FFKSKTFEI; SYLLIKSLK; FYKGSSKNI; TFEIQNQIF; KYLTWFFKD; SFIDALEYI; LYKLEKPEI; IQNQIFVYF; CYLIYCENL; FYDSLATLK; TKKYLTWFF; IFCQRMKEF;  RYIKSAFLEI; FYPNIEYKHF; VYFLSFYPNI; CYLYVIEKTI; YYKNTQNEAI; TYKLKSSNFL; LYLDENKITI; HYVKKNSQNI; IYADNKDKKI; KYLEEEILAL; TYGWIAFYIY; KYRPENQKVF; NQIFVYFLSF; KFISDILLYL; LYKLEKPEII; XYKYLEEEIL; KYLTWFFKDK; CYLIYCENLV; DYQKSYLLIK; IFCQRMKEFF; ITKKYLTWFF; FFISFDKTNF; IYCENLVELK; EFFKSKTFEI; KYSISSQLYK; NFNPYESSIF; KYIEEKVLKY; KYNEILNYLR; RYIKSAFLEII; KYLTWFFKDKI; KYSISSQLYKL; KFQTEDFFISF; |

| | | |
|---|---|---|
| | | EYIEKHKLINI; NYLRALKTLSL; IYADNKDKKIF; CYYKNTQNEAI; FYKGSSKNISF; QYAPKDFQDTL; YWPVGCLSKNI; EYKHFKFISDI; IFKERYIKSAF; SFYPNIEYKHF; FYPNIEYKHFK; TYKLKSSNFLK; TYGWIAFYIYA; KYLEEEILALK; KTITKKYLTWF; TITKKYLTWFF; IIIKNIRFNLI; SSKNISFNYFF; YYKNTQNEAIK; FYIYADNKDKK; ITAKFQTEDFF |
| | HLA-A2902 | YFFSDFTQY; KFISDILLY; EIQNQIFVY; LSFYPNIEY; YIEEKVLKY; NILNKNLKY; SSKNISFNY; ESFIDALEY; AFLEIIMHY; IFVYFLSFY; KINPNQLDY; TLSLNEIFY; ELIKISNDY; KILIKXYKY; KYSISSQLY; ETYGWIAFY; YGWIAFYIY; NTKKLGTTY; NTSLEECYY; KNISFNYFF; FNTSLEECY; FVYFLSFYP; QIFVYFLSF; IEIQNSPYY; FYPNIEYKH; LIEIQNSPY; FIKILIKXY; FISFDKTNF; NYFFSDFTQY; KYIEEKVLKY; KLIEIQNSPY; TYGWIAFYIY; IFIKILIKXY; FEIQNQIFVY; FLSFYPNIEY; QIFVYFLSFY; SAFLEIIMHY; FFISFDKTNF; SFDKTNFNPY; YYSQEKKDIY; FYPNIEYKHF; KTLSLNEIFY; FNTSLEECYY; LLPAMCYLIY; FQDTLKCYLY; QKINPNQLDY; KLKRMLILTY; FKFISDILLY; KTIKKDSEFY; LNILNKNLKY; IKILIKXYKY; VIEKTITKKY; GSSKNISFNY; ALKYNEILNY; AFNTSLEECY; EIQNQIFVYF; LIEIQNSPYY; YFLSFYPNIEY; YLEEEILALKY; YVIEKTITKKY; ILNILNKNLKY; KLIEIQNSPYY; TFEIQNQIFVY; FNYFFSDFTQY; HFKFISDILLY; KSAFLEIIMHY; NLNTKKLGTTY; ISFDKTNFNPY; NQIFVYFLSFY; FIKILIKXYKY; KLLPAMCYLIY; NAFNTSLEECY; NWESFIDALEY; PYYSQEKKDIY; DFQDTLKCYLY; ETYGWIAFYIY; AFNTSLEECYY; KGSSKNISFNY; KFQTEDFFISF; PYESSIFKERY; SFYPNIEYKHF; NIFIKILIKXY; NIETYGWIAFY; IIELIKISNDY |
| | HLA-A6801 | ETYGWIAFY; YSISSQLYK; LLEFISFFK; HINKYIEEK; EIIMHYVKK; EIFYKGSSK; EDFFISFDK; YVIEKTITK; ISFFKTKLK; NSPYYSQEK; ESRLIKLKR; NPYESSIFK; SIFKERYIK; ESFIDALEY; ILNYLRALK; EFISFFKTK; NLVELKNNR; NTQNEAIKK; TSLEECYYK; LSLNEIFYK; SFYPNIEYK; NTSLEECYY; YIYADNKDK; YNEILNYLR; NIRFNLIEK; NPNQLDYQK; ESSIFKERY; SPYYSQEKK; LVAVRNRNK; NIFIKILIK; CLSKNIFIK; ITIPKKIIK; TIDILVAVR; YLTWFFKDK; SSQLYKLEK; SSLTNKESR; SYLLIKSLK; LSFYPNIEY; SSKNISFNY; IFVYFLSFY; DILVAVRNR; EIQNQIFVY; ILNKNLKYR; FVYFLSFYP; ISNDYEKEK; NKNFIQISK; YESSIFKER; LNILNKNLK; ELIKISNDY; NYFFSDFTQ; TLSLNEIFY; FYDSLATLK; YFFSDFTQY; KLKSSNFLK; LVELKNNRK; LLIKSLKTR; TIKKDSEFY; IYADNKDKK; IKILIKXYK; NISFNYFFS; ILLYLDENK; ELKNNRKIK; IKISNDYEK; WILEIVRNK; ENKITIPKK; DENKITIPK; AFYIYADNK; KIKEIITAK; LKIIIKNIR; CQRMKEFFK; LEIIMHYVK; QTIDILVAVR; NTSLEECYYK; EFYDSLATLK; IAFYIYADNK; LSFYPNIEYK; YVIEKTITKK; FISFFKTKLK; SLLEFISFFK; EILNYLRALK; TLSLNEIFYK; YIYADNKDKK; NTKKLGTTYK; ETYGWIAFYI; NSPYYSQEKK; SSIFKERYIK; LIKISNDYEK; QIFVYFLSFY; SAFLEIIMHY; FIKILIKXYK; NYFFSDFTQY; FLEIIMHYVK; NILNKNLKYR; KSYLLIKSLK; DALEYIEKHK; KVFKSSLTNK; YPNIEYKHFK; ISSQLYKLEK; LTNKESRLIK; DILLYLDENK; KYNEILNYLR; ILVAVRNRNK; NLVELKNNRK; TKKYLTWFFK; FYIYADNKDK; ILNILNKNLK; LIKSLKTRSR; LATLKKHINK; FSDFTQYAPK; ILKIIIKNIR; YSQEKKDIYK; FIDALEYIEK; IYCENLVELK; NLKYRPENQK; KSSLTNKESR; YLEEEILALK; FNPYESSIFK; TTYKLKSSNF; EKIKEIITAK; ISKEANLNTK; FLSFYPNIEY; QNSPYYSQEK; TYGWIAFYIY; TYWPVGCLSK; TEDFFISFDK; SFFKTKLKQK; FVYFLSFYPN; NEIFYKGSSK; IDILVAVRNR; FNYFFSDFTQ; LTYWPVGCLS; IPKKIIKIQK; YKLKSSNFLK; KTIKKDSEFY; SLKTRSRILK; DENKITIPKK; LYVIEKTITK; ETYGWIAFYIY; ELIKISNDYEK; WIAFYIYADNK; ESSIFKERYIK; LTYWPVGCLSK; |

| | | |
|---|---|---|
| | | QTEDFFISFDK; FLSFYPNIEYK; EFYDSLATLKK; NSLLEFISFFK; NPYESSIFKER; ITKKYLTWFFK; EFISFFKTKLK; LIYCENLVELK; FYPNIEYKHFK; NFNPYESSIFK; YLYVIEKTITK; ISFFKTKLKQK; SISSQLYKLEK; KTLSLNEIFYK; FYIYADNKDKK; TLKCYLYVIEK; NQIFVYFLSFY; IFIKILIKXYK; FNTSLEECYYK; NAFNTSLEECY; TYKLKSSNFLK; IFCQRMKEFFK; EQTIDILVAVR; QISKEANLNTK; YAPKDFQDTLK; LKYNEILNYLR; FFSDFTQYAPK; SFIDALEYIEK; TIPKKIIKIQK; FNYFFSDFTQY; LKYSISSQLYK; TTYKLKSSNFL; TIDILVAVRNR; LLEFISFFKTK; LVAVRNRNKIK; FLEIIMHYVKK; LNILNKNLKYR; SEFYDSLATLK; DNKDKKIFCQR; LLIKSLKTRSR; NEILNYLRALK; YVIEKTITKKY; RILKIIIKNIR; ISFDKTNFNPY; SLATLKKHINK; YYSQEKKDIYK; FKSSLTNKESR; ITAKFQTEDFF; ISKEANLNTKK; LIEKLEDENEK; KTKLKQKINFK; ESFIDALEYIE; KINPNQLDYQK; YYKNTQNEAIK; KSAFLEIIMHY; YIEKHKLINIK; NIETYGWIAFY; INKYIEEKVLK; SYLLIKSLKTR; AIKKWILEIVR; DILVAVRNRNK; SSKNISFNYFF; HFKFISDILLY; KILNILNKNLK; QKSYLLIKSLK; NKIKILNILNK; LYVIEKTITKK; SLTNKESRLIK; ISFNYFFSDFT; QNSPYYSQEKK; FSSNINIDSLE; LSFYPNIEYKH; CENLVELKNNR |
| | HLA-B0702 | APKDFQDTL; KERYIKSAF; YPNIEYKHF; VAVRNRNKI; AVRNRNKIKI; YLRALKTLSL; IVRNKNFIQI; WPVGCLSKNI; LPAMCYLIYC; AVRNRNKIKIL; WPVGCLSKNIF; SPYYSQEKKDI; YPNIEYKHFKF; RPENQKVFKSS; APKDFQDTLKC; IPKKIIKIQKI; KVLKYSISSQL |
| | HLA-B0801 | SLKTRSRIL; FFKSKTFEI; LIKLKRMLI; RLIKLKRML; YLYVIEKTI; ILIKXYKYL; YIKSAFLEI; YLRALKTLSL; LIKLKRMLIL; FFKTKLKQKI; ILALKYNEIL; YIKSAFLEII; YYKNTQNEAI; FKERYIKSAF; IFKERYIKSAF; RMKEFFKSKTF; RLIKLKRMLIL; YIKSAFLEIIM; IPKKIIKIQKI; YQKSYLLIKSL |
| | HLA-B1501 | LSFYPNIEY; IQNQIFVYF; NQLDYQKSY; QIFVYFLSF; SSKNISFNY; SLLEFISFF; FIKILIKXY; YLEEEILAL; ETYGWIAFY; KISNNWESF; TLKKHINKY; KTIKKDSEF; KINPNQLDY; ELIKISNDY; NTKKLGTTY; ESFIDALEY; LIEIQNSPY; IQISKEANL; YSQEKKDIY; YIEEKVLKY; IQKINPNQL; NQKVFKSSL; YFFSDFTQY; IEIQNSPYY; AFLEIIMHY; KFISDILLY; QTEDFFISF; TQNEAIKKW; KIIIKNIRF; KQIILNFSS; IETYGWIAF; TLSLNEIFY; KNSQNIETY; FDKTNFNPY; SQNIETYGW; KLIEIQNSPY; FQTEDFFISF; NQIFVYFLSF; KTFEIQNQIF; KLKRMLILTY; FLSFYPNIEY; YLRALKTLSL; ALKYNEILNY; KIKEIITAKF; SSKNISFNYF; SAFLEIIMHY; ISFNYFFSDF; QIFVYFLSFY; KQKINFKKEL; LLPAMCYLIY; KTIKKDSEFY; IQNQIFVYFL; FEIQNQIFVY; KIFCQRMKEF; IFIKILIKXY; ITAKFQTEDF; GSSKNISFNY; FQDTLKCYLY; WESFIDALEY; VLKYSISSQL; RMKEFFKSKTF; ISFDKTNFNPY; YVIEKTITKKY; KLIEIQNSPYY; IQKINPNQLDY; VLKYSISSQLY; YQKSYLLIKSL; QNIETYGWIAF; KSNEKNSLLEF; NQIFVYFLSFY; YLEEEILALKY; ILNILNKNLKY; IFKERYIKSAF; KSAFLEIIMHY; ALKTLSLNEIF; KLLPAMCYLIY; ILKIIIKNIRF; YFLSFYPNIEY; WILEIVRNKNF; KQIILNFSSNI; YLIYCENLVEL; SSKNISFNYFF; ETYGWIAFYIY; FYKGSSKNISF; FIKILIKXYKY; NLNTKKLGTTY; KQKINFKKELM; TNFNPYESSIF; KFQTEDFFISF; NAFNTSLEECY; TQNEAIKKWIL; GSSKNISFNYF; HFKFISDILLY |
| | HLA-B2705 | SRILKIIIK; LRALKTLSL; KRMLILTYW; IRFNLIEKL; KKLGTTYKL; ERYIKSAFL; YRPENQKVF; SRLIKLKRM; KKWILEIVR; KKYLTWFFK; QRMKEFFKSK; KRMLILTYWP; SRLIKLKRML; YRPENQKVFK; KKLGTTYKLK; NQIFVYFLSF; NRNKIKILNI; KRMLILTYWPV; VRNKNFIQISK; KKWILEIVRNK; SRLIKLKRMLI; SRILKIIIKNI |
| | HLA-B3501 | LPAMCYLIY; YGWIAFYIY; YPNIEYKHF; YFFSDFTQY; LIEIQNSPY; FDKTNFNPY; EIQNQIFVY; ETYGWIAFY; ESFIDALEY; TFEIQNQIF; |

| | | |
|---|---|---|
| | | YLEEEILAL; TAKFQTEDF; LSFYPNIEY; IFVYFLSFY; QTEDFFISF; QIFVYFLSF; EFYDSLATL; NSLLEFISF; IQNQIFVYF; FISFDKTNF; YSQEKKDIY; LALKYNEIL; TLSLNEIFY; IEIQNSPYY; APKDFQDTL; IETYGWIAF; NTKKLGTTY; YIEEKVLKY; FLSFYPNIEY; NIETYGWIAF; WESFIDALEY; YADNKDKKIF; FEIQNQIFVY; NFNPYESSIF; TAKFQTEDFF; SAFLEIIMHY; LIEIQNSPYY; FQTEDFFISF; TYGWIAFYIY; FFISFDKTNF; SFDKTNFNPY; FKFISDILLY; YAPKDFQDTL; EIQNQIFVYF; NYFFSDFTQY; KLIEIQNSPY; NPNQLDYQKS; QIFVYFLSFY; WPVGCLSKNI; YYSQEKKDIY; NNWESFIDAL; FKERYIKSAF; LPAMCYLIYC; FSSNINIDSL; YESSIFKERY; WPVGCLSKNIF; NPNQLDYQKSY; YPNIEYKHFKF; NAFNTSLEECY; ETYGWIAFYIY; ISFDKTNFNPY; TFEIQNQIFVY; YFLSFYPNIEY; YVIEKTITKKY; NIETYGWIAFY; LALKYNEILNY; YLEEEILALKY; YIKSAFLEIIM; LATLKKHINKY; FEIQNQIFVYF; FNYFFSDFTQY; NLNTKKLGTTY; LKLIEIQNSPY; YLIYCENLVEL; HFKFISDILLY |
| | HLA-B4403 | EEEILALKY; SEFYDSLAT; IEIQNSPYY; NEILNYLRA; EEILALKYN; FEIQNQIFV; KEFFKSKTF; NEKNSLLEF; LEIVRNKNF; IETYGWIAF; NEIFYKGSS; KEIITAKFQ; FEIQNQIFVY; SEFYDSLATL; NEILNYLRAL; KEIITAKFQT; WESFIDALEY; EEILALKYNE; IEIQNSPYYS; EEILALKYNEI; FEIQNQIFVYF; KEFFKSKTFEI; NEKKLLPAMCY; SEFYDSLATLK; EEKVLKYSISS; DENEKKLLPAM; NEILNYLRALK |
| | HLA-B5101 | LPAMCYLIY; WPVGCLSKNI; LPAMCYLIYC; KPEIIELIKI; IPKKIIKIQKI; WPVGCLSKNIF; SPYYSQEKKDI |
| | HLA-B5701 | KTLSLNEIF; TITKKYLTW; NTKKLGTTY; KTITKKYLTW; NSQNIETYGW; KTFEIQNQI; NTQNEAIKKW; KTLSLNEIFY; ITAKFQTEDFF; KNSQNIETYGW; ISFDKTNFNPY |
| NP_045482.1 \| hypothetical protein BBG22 [Borrelia burgdorferi B31]; Seq29 | HLA-A0101 | SADTWYTWY; YLEHMILFF; ETKPKNTCY; NTCYISFYRY; RSADTWYTWY; MSPFITGYSY; YSYEEIDPEY; |
| | HLA-A0201 | SLDSLIEYL; SLIEYLEHM; YTWYWAEEI; KLVKRMTMI; FAMEGSILT; KLIQAFTNT; YLLPPFTSQ; YISFYRYDL; FTNTNKFCI; MILFFLEKT; RMTMIYRSA; YQGSLGEPI; FLEKTPVGT; YLEHMILFFL; SLIEYLEHMI; FITGYSYEEI; LLKDKLTFSI; ILFFLEKTPV; YLLPPFTSQS; YTWYWAEEIM; FLEKTPVGTL; VLGISSAPKL; MITQQDYTEI; HMILFFLEKT; IMSPFITGYS; ILTRSKLIQA; SLIEYLEHMIL; MILFFLEKTPV; ILLKDKLTFSI; SLPSNCYAYKV; YSYEEIDPEYL; KVLGISSAPKL; MIYRSADTWYT; YQGSLGEPILL; YAYKVLGISSA; KIDSLGYQGSL; YWAEEIMSPFI; FAMEGSILTRS; SILTRSKLIQA |
| | HLA-A0301 | HMILFFLEK; SLGEPILLK; RIQFRNGNK; KLSGRFLRH; VLGISSAPK; IQAFTNTNK; LVKRMTMIY; IMSPFITGY; LTFSICDGK; LDFSRINFK; INFKGKLVK; AMEGSILTR; KVLGISSAPK; RINFKGKLVK; LIQAFTNTNK; KLTFSICDGK; SLPSNCYAYK; GSLGEPILLK; KLSGRFLRHY; KLVKRMTMIY; KTPVGTLTAK; RSADTWYTWY; LVKRMTMIYR; EHMILFFLEK; MIYRSADTWY; DLDFSRINFK; NTCYISFYRY; MSPFITGYSY; RSLPSNCYAY; EIMSPFITGY; TQQDYTEIKK; ISSAPKLSGR; TSQSGSKETK; QSGSKETKPK; KNTCYISFYR; KLIQAFTNTNK; RSLPSNCYAYK; KLVKRMTMIYR; KTRIQFRNGNK; RINFKGKLVKR; AMEGSILTRSK; SLGHTQEDSYK; IMSPFITGYSY; SLGEPILLKDK; TMIYRSADTWY; ITQQDYTEIKK; FTSQSGSKETK; RSLGHTQEDSY; MITQQDYTEIK |
| | HLA-A1101 | HMILFFLEK; SLGEPILLK; RIQFRNGNK; LTFSICDGK; NTCYISFYR; TQQDYTEIK; IQAFTNTNK; VLGISSAPK; SQSGSKETK; SSAPKLSGR; AMEGSILTR; NTKTRIQFR; KLSGRFLRH; QQDYTEIKK; LVKRMTMIY; LDFSRINFK; SADTWYTWY; TPVGTLTAK; IMSPFITGY; MITQQDYTEI; ITQQDYTEIK; KVLGISSAPK; GSLGEPILLK; LIQAFTNTNK; |

| | | |
|---|---|---|
| | | TQQDYTEIKK; TSQSGSKETK; RINFKGKLVK; KTPVGTLTAK; SLPSNCYAYK; NTCYISFYRY; KLTFSICDGK; RSADTWYTWY; LVKRMTMIYR; ISSAPKLSGR; MIYRSADTWY; RSLPSNCYAY; QSGSKETKPK; FAMEGSILTR; MSPFITGYSY; EIMSPFITGY; KLSGRFLRHY; DLDFSRINFK; KLVKRMTMIY; MITQQDYTEIK; RSLPSNCYAYK; KLIQAFTNTNK; ITQQDYTEIKK; AMEGSILTRSK; SQSGSKETKPK; KTRIQFRNGNK; TQQDYTEIKKK; SLGHTQEDSYK; FTSQSGSKETK; KLVKRMTMIYR; SICDGKNIDNR; SLGEPILLKDK; SAPKLSGRFLR; RINFKGKLVKR; NTNKFCIPDGR; LEHMILFFLEK; IMSPFITGYSY; GISSAPKLSGR; TMIYRSADTWY |
| | HLA-A2402 | EYLEHMILF; CYAYKVLGI; SYKNHDHDL; EYLLPPFTS; EYLEHMILFF; YWAEEIMSPF; WYTWYWAEEI; CYISFYRYDL; IQAFTNTNKF; GYQGSLGEPI; AFTNTNKFCI; SYEEIDPEYL; EYLEHMILFFL; YWAEEIMSPFI; WYWAEEIMSPF; TWYTWYWAEEI; IYRSADTWYTW; SYKNHDHDLDF; SYEEIDPEYLL; EYEITKNAPSL; PFITGYSYEEI; WYTWYWAEEIM |
| | HLA-A2902 | SLPSNCYAY; IMSPFITGY; IYRSADTWY; SYEEIDPEY; SADTWYTWY; YLEHMILFF; SPFITGYSY; ETKPKNTCY; TCYISFYRY; LVKRMTMIY; EIMSPFITGY; YSYEEIDPEY; MSPFITGYSY; YWAEEIMSPF; MIYRSADTWY; RSADTWYTWY; NTCYISFYRY; RSLPSNCYAY; EYLEHMILFF; KLVKRMTMIY; SLGHTQEDSY; TMIYRSADTWY; IMSPFITGYSY; GYSYEEIDPEY; YRSADTWYTWY; NAPSLDSLIEY; WYWAEEIMSPF |
| | HLA-A6801 | NTCYISFYR; LTFSICDGK; NTKTRIQFR; HMILFFLEK; SSAPKLSGR; LDFSRINFK; LPSNCYAYK; TPVGTLTAK; NKFCIPDGR; GSTDSDGTR; HDHDLDFSR; IQAFTNTNK; MSPFITGYS; ETKPKNTCY; TQQDYTEIK; NFKGKLVKR; FITGYSYEE; MTMIYRSAD; RIQFRNGNK; LVKRMTMIY; NTCYISFYRY; FAMEGSILTR; LVKRMTMIYR; ENTKTRIQFR; MIYRSADTWY; MSPFITGYSY; KNTCYISFYR; ISSAPKLSGR; LIQAFTNTNK; ITQQDYTEIK; YSYEEIDPEY; TSQSGSKETK; DLDFSRINFK; EHMILFFLEK; EIKKKLENTK; EIMSPFITGY; SLPSNCYAYK; TNKFCIPDGR; DFSRINFKGK; KLTFSICDGK; INFKGKLVKR; KVLGISSAPK; TRIQFRNGNK; TQQDYTEIKK; NTNKFCIPDGR; MITQQDYTEIK; FTSQSGSKETK; SAPKLSGRFLR; SICDGKNIDNR; ITQQDYTEIKK; MSPFITGYSE; KLVKRMTMIYR; MTMIYRSADTW; GISSAPKLSGR; TMIYRSADTWY; RSLPSNCYAYK; RINFKGKLVKR; MIYRSADTWYT; NTCYISFYRYD; EKTPVGTLTAK; NAPSLDSLIEY; KLIQAFTNTNK |
| | HLA-B0702 | APKLSGRFL; IPDGRSLPS; TPVGTLTAK; TPVGTLTAKF; KPKNTCYISF; LPSNCYAYKV; LPSNCYAYKVL; APSLDSLIEYL; TPVGTLTAKFA; APKLSGRFLRH; IPDGRSLPSNC; EPILLKDKLTF |
| | HLA-B0801 | KGKLVKRMTM; LTRSKLIQAF; ILTRSKLIQAF |
| | HLA-B1501 | IMSPFITGY; LVKRMTMIY; WAEEIMSPF; YLEHMILFF; IQFRNGNKI; YQGSLGEPI; KNIDNRKEY; QAFTNTNKF; SLPSNCYAY; ETKPKNTCY; ILLKDKLTF; IQAFTNTNKF; YSYEEIDPEY; RSLPSNCYAY; RSADTWYTWY; KLSGRFLRHY; LTRSKLIQAF; KLVKRMTMIY; YQGSLGEPIL; MSPFITGYSY; MIYRSADTWY; YWAEEIMSPF; TMIYRSADTW; EIMSPFITGY; SLGHTQEDSY; SSAPKLSGRF; IMSPFITGYSY; TMIYRSADTWY; ILTRSKLIQAF; LIQAFTNTNKF; RSLGHTQEDSY; ISSAPKLSGRF; YQGSLGEPILL |
| | HLA-B2705 | KRMTMIYRS; SRINFKGKL; NRKEYEITK; TRSKLIQAF; GRFLRHYDS; KRMTMIYRSA; FRNGNKIDSL; TRIQFRNGNK; SRINFKGKLVK; YRSADTWYTWY; KRMTMIYRSAD; GRSLPSNCYAY; YKVLGISSAPK |
| | HLA-B3501 | DPEYLLPPF; WAEEIMSPF; SPFITGYSY; QAFTNTNKF; FAMEGSILT; LVKRMTMIY; SADTWYTWY; TPVGTLTAK; SYEEIDPEY; YLEHMILFF; |

EP 2 254 592 B1

| | | |
|---|---|---|
| | | IMSPFITGY; YTWYWAEEI; TWYWAEEIM; EPILLKDKL; LGHTQEDSY; TCYISFYRY; IPDGRSLPS; TRSKLIQAF; TPVGTLTAKF; APSLDSLIEY; YTWYWAEEIM; YSYEEIDPEY; MIYRSADTWY; MSPFITGYSY; YWAEEIMSPF; KPKNTCYISF; EIMSPFITGY; RSLPSNCYAY; NTCYISFYRY; WAEEIMSPFI; DPEYLLPPFT; LPSNCYAYKV; EPILLKDKLTF; LPSNCYAYKVL; NAPSLDSLIEY; WYWAEEIMSPF; WYTWYWAEEIM; IMSPFITGYSY; EIDPEYLLPPF; KPKNTCYISFY; TMIYRSADTWY; APSLDSLIEYL; WAEEIMSPFIT; LIEYLEHMILF; TPVGTLTAKFA; FAMEGSILTRS; YRSADTWYTWY; RSLGHTQEDSY; DPEYLLPPFTS; LIQAFTNTNKF; MTMIYRSADTW; PVGTLTAKFAM |
| | HLA-B4403 | EEIDPEYLL; EEIMSPFIT; AEEIMSPFI; YEITKNAPS; EEIDPEYLLP; EEIMSPFITG; AEEIMSPFIT; IEYLEHMILF; YEITKNAPSL; KETKPKNTCY; EEIMSPFITGY; EEIDPEYLLPP; IEYLEHMILFF; KEYEITKNAPS |
| | HLA-B5101 | LPPFTSQSG; LPSNCYAYKV; LPPFTSQSGS; LPSNCYAYKVL; LPPFTSQSGSK; EPILLKDKLTF |
| | HLA-B5701 | RSADTWYTW; ETKPKNTCY; IMSPFITGY; MIYRSADTW; YSYEEIDPEY; MTMIYRSADTW; RSADTWYTWYW |
| NP_212885.1 \| hypothetical protein BB0751 [Borrelia burgdorferi B31]; Seq30 | HLA-A0101 | KIDDYNLQY; LTKNIFLSY; FLSYKGNSY; YTTSKWTFF; EIDKSKIDDY; YLTKNIFLSY; FSDTTLFQEK; LTSTFLLGIIF; TTSKWTFFDVF; KSGEGDSRLAY |
| | HLA-A0201 | KLFDNNNKL; VVLTSTFLL; SLIKALDEL; FLLGIIFSN; KLENLSFKI; YLTKNIFLS; LTSTFLLGI; KFFDFDFNL; KILIIVVLT; WTFFDVFDL; IIFSNENKV; LISKIETEL; MTKPKIFSI; KIKILIIVV; KLLTEIFVG; FMTKPKIFSI; VLTSTFLLGI; KLFDNNNKLL; NLISKIETEL; LTYNKQNIPV; IISKDGLYFL; TTSKWTFFDV; KMTKERPLNI; TLFQEKNTKL; ILIIVVLTST; FLLGIIFSNE; KIFSINKEKI; KLNKENENNI; IVVLTSTFLL; NLSFKIIRKL; ALDELQKNKL; IIVVLTSTFL; KFFDFDFNLI; IIAEFKADGL; KLENLSFKII; GIIFSNENKV; KIDDYNLQYKI; VVLTSTFLLGI; ILTYNKQNIPV; KLFDNNNKLLT; ALDELQKNKLV; IIVVLTSTFLL; YTTSKWTFFDV; FLSYKGNSYNT; LIIVVLTSTFL; VLTSTFLLGII; YIKGSDENVYL; KILIIVVLTST; KMTKERPLNII; FLLGIIFSNEN; TLTKLGKDWIL; KLLTEIFVGKS |
| | HLA-A0301 | GLYFLNNQK; FLNNQKMTK; KIFSINKEK; NLSFKIIRK; KIEVKWSNK; KLVSRDQKK; LLTEIFVGK; GIGENPSFK; KVARILEEK; LTKNIFLSY; TLFQEKNTK; KNKGVFMTK; GIIFSNENK; PLNIIAEFK; KIDDYNLQY; KQNIPVDNK; ALDELQKNK; KLLTEIFVGK; LTKNIFLSYK; RPLNIIAEFK; KIDDYNLQYK; YFLNNQKMTK; TTLFQEKNTK; YLTKNIFLSY; NTKLENLSFK; SFKIIRKLNK; GSDENVYLTK; INNNYEIISK; TFFDVFDLEK; KDEYYYTTSK; IFLSYKGNSY; LIKALDELQK; NKNKGVFMTK; LEGTLTKLGK; FDFDFNLISK; KLGKDWILTY; FSDTTLFQEK; LLEKEKQINK; KALDELQKNK; LGIGENPSFK; ENLSFKIIRK; LVSRDQKKHK; LSFKIIRKLNK; MTKPKIFSINK; KIFSINKEKIK; YLTKNIFLSYK; SVNNIEVYFNK; KLVSRDQKKHK; LLGIIFSNENK; SLIKALDELQK; NINNNYEIISK; KLENLSFKIIR; FLNNQKMTKER; LYFLNNQKMTK; IIFSNENKVAR; WTFFDVFDLEK; TFFDVFDLEKK; ELGIGENPSFK |
| | HLA-A1101 | GLYFLNNQK; GIIFSNENK; KVARILEEK; KIFSINKEK; GIGENPSFK; KQNIPVDNK; KIEVKWSNK; NLSFKIIRK; TLFQEKNTK; LLTEIFVGK; TKNIFLSYK; FLNNQKMTK; KADGLEIDK; LTKNIFLSY; KLVSRDQKK; KIDDYNLQY; PLNIIAEFK; KNKGVFMTK; ALDELQKNK; TKLENLSFK; NNIEVYFNK; KPKIFSINK; TELEGTLTK; NLQYKIEVK; KDWILTYNK; KGVFMTKPK; LTKNIFLSYK; TTLFQEKNTK; KIDDYNLQYK; TFFDVFDLEK; KLLTEIFVGK; GSDENVYLTK; NTKLENLSFK; KALDELQKNK; KQNIPVDNKK; FSDTTLFQEK; LIKALDELQK; |

| | | |
|---|---|---|
| | | VNNIEVYFNK; RPLNIIAEFK; LVSRDQKKHK; ETELEGTLTK; SFKIIRKLNK; STFLLGIIFS; NTFSDTTLFQ; SFKLFDNNNK; INNNYEIISK; SVNNIEVYFNK; MTKPKIFSINK; WTFFDVFDLEK; LSFKIIRKLNK; SLIKALDELQK; KIFSINKEKIK; TFSDTTLFQEK; YLTKNIFLSYK; TFFDVFDLEKK; NINNNYEIISK; PSFKLFDNNNK; LLGIIFSNENK; IIFSNENKVAR; KADGLEIDKSK; KLVSRDQKKHK; STFLLGIIFSN; LYFLNNQKMTK; KGSDENVYLTK; KLENLSFKIIR; SKIDDYNLQYK; KNTKLENLSFK; DTTLFQEKNTK |
| | HLA-A2402 | YYTTSKWTF; VYLTKNIFL; VFMTKPKIF; EYYYTTSKW; VVLTSTFLL; FFDFDFNLI; YYYTTSKWT; LYFLNNQKM; YTTSKWTFF; TYNKQNIPV; YYYTTSKWTF; SYKGNSYNTF; YYTTSKWTFF; KWSNKSVNNI; SYNTFSDTTL; KFFDFDFNLI; VYLTKNIFLS; KWTFFDVFDL; IVVLTSTFLL; TSKWTFFDVF; EYYYTTSKWTF; YYYTTSKWTFF; SYNTFSDTTLF; VFMTKPKIFSI; DWILTYNKQNI; VYLTKNIFLSY; ILIIVVLTSTF; IFSNENKVARI; TTSKWTFFDVF |
| | HLA-A2902 | KIDDYNLQY; SVNNIEVYF; EIISKDGLY; FLSYKGNSY; YYTTSKWTF; KFFDFDFNL; LTKNIFLSY; IISKDGLYF; STFLLGIIF; YLTKNIFLSY; YYYTTSKWTF; IFLSYKGNSY; YYTTSKWTFF; KLGKDWILTY; SKIDDYNLQY; EIISKDGLYF; YIKGSDENVY; ILIKKDKDEY; VYLTKNIFLSY; YYYTTSKWTFF; AYIKGSDENVY; ILIKKDKDEYY; SYNTFSDTTLF; EYYYTTSKWTF; NYEIISKDGLY; LIKKDKDEYYY; KSKIDDYNLQY; NIFLSYKGNSY; ILIIVVLTSTF; ILEEKFFDFDF |
| | HLA-A6801 | FSNENKVAR; NNIEVYFNK; NTFSDTTLF; TLFQEKNTK; GIIFSNENK; KVARILEEK; KIFSINKEK; EVYFNKNEE; ENLSFKIIR; GLYFLNNQK; DEYYYTTSK; LLTEIFVGK; TKNIFLSYK; FLNNQKMTK; NLSFKIIRK; TTSKWTFFD; PLNIIAEFK; FSINKEKIK; NNNYEIISK; NLQYKIEVK; EIISKDGLY; LTKNIFLSY; YTTSKWTFF; EGTLTKLGK; SVNNIEVYF; QNIPVDNKK; NNQKMTKER; FFDVFDLEK; NTKLENLSFK; TFFDVFDLEK; LTKNIFLSYK; TTLFQEKNTK; NTFSDTTLFQ; ETELEGTLTK; ELQKNKLVSR; WTFFDVFDLE; LIKALDELQK; FDFDFNLISK; ENLSFKIIRK; EVYFNKNEEN; LGIGENPSFK; IFSNENKVAR; LVSRDQKKHK; FSDTTLFQEK; LGIIFSNENK; SFKLFDNNNK; RPLNIIAEFK; YLTKNIFLSY; YTTSKWTFFD; NKVARILEEK; NKNKGVFMTK; YKIEVKWSNK; WTFFDVFDLEK; SVNNIEVYFNK; DTTLFQEKNTK; IIFSNENKVAR; MTKPKIFSINK; YLTKNIFLSYK; TFFDVFDLEKK; NTFSDTTLFQE; LSFKIIRKLNK; FLNNQKMTKER; NINNNYEIISK; LYFLNNQKMTK; TFSDTTLFQEK; FVGKSGEGDSR; SLIKALDELQK; ELGIGENPSFK; ELEGTLTKLGK; ENKVARILEEK; KIFSINKEKIK; TTSKWTFFDVF; LLGIIFSNENK; PSFKLFDNNNK; ERPLNIIAEFK; NVYLTKNIFLS; NIFLSYKGNSY; EFKADGLEIDK; DYNLQYKIEVK; STFLLGIIFSN |
| | HLA-B0702 | RPLNIIAEF; KVNSLIKAL; IVVLTSTFL; IVVLTSTFLL; IPVDNKKVNSL; RPLNIIAEFKA |
| | HLA-B0801 | RDQKKHKEL; KIKILIIVVL; NKKVNSLIKAL; YIKGSDENVYL |
| | HLA-B1501 | FLSYKGNSY; LTKNIFLSY; LGKDWILTY; KSVNNIEVY; STFLLGIIF; IIVVLTSTF; LGIGENPSF; IISKDGLYF; NTKLENLSF; SVNNIEVYF; KQINKNKGV; KLFDNNNKL; KVNSLIKAL; KQINKNKGVF; YIKGSDENVY; YLTKNIFLSY; LIIVVLTSTF; KLGKDWILTY; KSVNNIEVYF; ILIKKDKDEY; TSKWTFFDVF; KLFDNNNKLL; KVARILEEKF; IFLSYKGNSY; GVFMTKPKIF; NQKMTKERPL; KQINKNKGVF; YIKGSDENVY; YLTKNIFLSY; LIIVVLTSTF; KLGKDWILTY; KSVNNIEVYF; ILIKKDKDEY; TSKWTFFDVF; KLFDNNNKLL; KVARILEEKF; IFLSYKGNSY; GVFMTKPKIF; NQKMTKERPL |
| | HLA-B2705 | KQINKNKGVF; ARILEEKFFDF; KQINKNKGVFM |

| | HLA-B3501 | RPLNIIAEF; IIVVLTSTF; SKWTFFDVF; FLSYKGNSY; NVYLTKNIF; NTFSDTTLF; SVNNIEVYF; KSVNNIEVY; YKGNSYNTF; LTKNIFLSY; LGKDWILTY; STFLLGIIF; YYTTSKWTF; IKGSDENVY; IVVLTSTFL; NENNINNNY; YIKGSDENVY; TSKWTFFDVF; LIIVVLTSTF; NKSVNNIEVY; YYYTTSKWTF; YLTKNIFLSY; IFLSYKGNSY; YNTFSDTTLF; SGEGDSRLAY; TSTFLLGIIF; IVVLTSTFLL; IPVDNKKVNSL; TTSKWTFFDVF; LTSTFLLGIIF; LSYKGNSYNTF; NIFLSYKGNSY; KSGEGDSRLAY; TKLGKDWILTY; ILIIVVLTSTF; FSNENKVARIL; NSYNTFSDTTL |
|---|---|---|
| | HLA-B4403 | EENDKDILI; EEKFFDFDF; AEFKADGLE; GEGDSRLAY; GENPSFKLF; NENNINNNY; NENKVARIL; KENENNINN; KERPLNIIA; YEIISKDGL; AEFKADGLEI; YEIISKDGLY; EENDKDILIK; GEGDSRLAYI; YEIISKDGLYF; EEKFFDFDFNL; KENENNINNNY; AEFKADGLEID; KERPLNIIAEF; KELGIGENPSF; NENNINNNYEI; EENDKDILIKK |
| | HLA-B5101 | IPVDNKKVNSL |
| | HLA-B5701 | LTKNIFLSY; KSVNNIEVY; KSKIDDYNLQY; KSGEGDSRLAY; LSYKGNSYNTF |
| AAC67038.1\| predicted coding region BB0689 [Borrelia burgdorferi B31]; Seq31 | HLA-A0101 | TTDNIDIFV; TLEKVAKEY; NTDTDKIGGY; TTDNIDIFVV; TTPMQRIHKY; DTKEDMKILY; GTTPMQRIHKY; TTDNIDIFVVL |
| | HLA-A0201 | ILYSEIAEL; LIIIFTLFL; TTDNIDIFV; KLNLNHLEI; KKLIIIFTL; FLSQACNLS; TLFGTTPMQ; KLIIIFTLFL; FLSQACNLST; KILYSEIAEL; KTTDNIDIFV; TTDNIDIFVV; ALINTDTDKI; TLFLSQACNL; IIFTLFLSQA; KVAKEYAIKL; ILASGIELNRV; FLSQACNLSTM; TLFGTTPMQRI; KTTDNIDIFVV; KLIIIFTLFLS; FTLFLSQACNL; RIHKYDQSFNL; KKLIIIFTLFL; WLNSPSHKEAL; NLTREILASGI; NLNHLEIDDTL; HLEIDDTLEKV; IIIFTLFLSQA |
| | HLA-A0301 | VLFGKRKYK; KVAKEYAIK; STMHKIDTK; ACNLSTMHK; TTPMQRIHK; IIFTLFLSQ; DIFVVLFGK; VVLFGKRKYK; QACNLSTMHK; TLFGTTPMQR; GTTPMQRIHK; IDIFVVLFGK; ILYSEIAELR; LSTMHKIDTK; IFVVLFGKRK; IIIFTLFLSQ; ILASGIELNR; LYSEIAELRK; NAWLNSPSHK; ILYSEIAELRK; HTLFGTTPMQR; SQACNLSTMHK; KILYSEIAELR; VVNAWLNSPSH; FVVLFGKRKYK |
| | HLA-A1101 | STMHKIDTK; TTPMQRIHK; DIFVVLFGK; KVAKEYAIK; VLFGKRKYK; ACNLSTMHK; ALINTDTDK; YSEIAELRK; FVVLFGKRK; KIDTKEDMK; AWLNSPSHK; IIFTLFLSQ; LASGIELNR; QACNLSTMHK; TLFGTTPMQR; GTTPMQRIHK; VVLFGKRKYK; LSTMHKIDTK; ILYSEIAELR; NAWLNSPSHK; TTPMQRIHKY; IDIFVVLFGK; ILASGIELNR; HLEIDDTLEK; IIIFTLFLSQ; LYSEIAELRK; DIFVVLFGKR; YSEIAELRKK; SQACNLSTMHK; ILYSEIAELRK; HTLFGTTPMQR; NIDIFVVLFGK; KILYSEIAELR; FVVLFGKRKYK; DIFVVLFGKRK; VVNAWLNSPSH; NLSTMHKIDTK; EILASGIELNR; VNAWLNSPSHK |
| | HLA-A2402 | KLIIIFTLF; KYDQSFNLT; LFGTTPMQRI; KKLIIIFTLF; GYRLKTTDNI; |
| | HLA-A2902 | VVLFGKRKY; KLIIIFTLF; FVVLFGKRKY; TTPMQRIHKY; IFVVLFGKRKY; GTTPMQRIHKY |
| | HLA-A6801 | DIFVVLFGK; LASGIELNR; TTPMQRIHK; LYSEIAELR; YAIKLGENR; DTDKIGGYR; STMHKIDTK; FVVLFGKRK; YSEIAELRK; TLFGTTPMQ; IFVVLFGKR; YDQSFNLTR; VLFGKRKYK; KVAKEYAIK; TLFGTTPMQR; DIFVVLFGKR; ILYSEIAELR; QACNLSTMHK; ILASGIELNR; NAWLNSPSHK; EYAIKLGENR; EALINTDTDK; GTTPMQRIHK; TTPMQRIHKY; LSTMHKIDTK; IDIFVVLFGK; LYSEIAELRK; DTKEDMKILY; HTLFGTTPMQ; HLEIDDTLEK; TDTDKIGGYR; YSEIAELRKK; VVLFGKRKYK; HTLFGTTPMQR; EILASGIELNR; FVVLFGKRKYK; DIFVVLFGKRK; NTDTDKIGGYR; HKYDQSFNLTR; NIDIFVVLFGK; ILYSEIAELRK; KILYSEIAELR; DTDKIGGYRLK; NLSTMHKIDTK; SQACNLSTMHK; IDIFVVLFGKR; EIDDTLEKVAK; |

| | | |
|---|---|---|
| | | LYSEIAELRKK; VNAWLNSPSHK |
| | HLA-B0702 | SPSHKEALI; TPMQRIHKY;  ; |
| | HLA-B0801 | RIHKYDQSF;  ; ELRKKLNLNHL |
| | HLA-B1501 | RIHKYDQSF; SQACNLSTM; KLIIIFTLF; ILYSEIAEL;  FVVLFGKRKY; SQACNLSTMH; LSQACNLSTM;  MQRIHKYDQSF; RLKTTDNIDIF; VVNAWLNSPSH; FLSQACNLSTM; WLNSPSHKEAL |
| | HLA-B2705 | YRLKTTDNI;  QRIHKYDQSF;  YRLKTTDNIDI |
| | HLA-B3501 | TPMQRIHKY; HTLFGTTPM; NAWLNSPSH; QACNLSTMH; FVVLFGKRKY; THTLFGTTPM;  FLSQACNLSTM; ITHTLFGTTPM; TTDNIDIFVVL; WLNSPSHKEAL |
| | HLA-B4403 | AELRKKLNL; REILASGIE; KEDMKILYS;  REILASGIEL; SEIAELRKKL; GENRTITHTLF; AELRKKLNLNH; REILASGIELN |
| | HLA-B5101 | |
| | HLA-B5701 | ELNRVVNAW; KTTDNIDIF; |
| NP_045709.1 \| lipoprotein BBA36[Borrelia burgdorferi B31];  Seq32 | HLA-A0101 | VVDAFGADPY; |
| | HLA-A0201 | MMQRISILL; SILLMLLAV; ALSLFFQKV; KLESLKVFL; RISILLMLL; LMLLAVFSC; LLMLLAVFS; KCNEEIFKV; KQFGDVKSL;  MMQRISILLM; KVFLKDAMSV; ALSLFFQKVV; SLTPEEAEKL; SSFDNISSIV; LLMLLAVFSC; SLFFQKVVDA; RISILLMLLA; NISSIVSKAV; MLLAVFSCKQ; ISILLMLLAV;  MMQRISILLML; FISSFDNISSI; SLTEIDSGNGI; RISILLMLLAV; VLTESESNNEL |
| | HLA-A0301 | MLLAVFSCK; KEFFDWLSK; EALSLFFQK; VSKAVDASK; VCYKIKNSK; LMLLAVFSCK; KLESLKVFLK; YVCYKIKNSK; SLGFKEYVCY; SVNGREEALK; ISLTPEEAEK; IVSKAVDASK; WLSKDVNRQK; PTEQQSLGFK; IPLVVSDVVK; MLLAVFSCKQ; VSKAVDASKK; LKAEYEKSYK;  LLMLLAVFSCK; SLGFKEYVCYK; IVSKAVDASKK; ALKAEYEKSYK; SIVSKAVDASK; MSVNGREEALK; VVDAFGADPYK; KGEALSLFFQK; GIPLVVSDVVK |
| | HLA-A1101 | MLLAVFSCK; EALSLFFQK; VSKAVDASK; KEFFDWLSK; KAEYEKSYK; DVVKDLIPK; AFGADPYKK; TEQQSLGFK; SLTPEEAEK; DAFGADPYK; LSKDVNRQK; SNNELKNLK; LESLKVFLK; AVDASKKRR; EALKAEYEK; GFKEYVCYK;  SVNGREEALK; LMLLAVFSCK; KLESLKVFLK; IVSKAVDASK; YVCYKIKNSK; ISLTPEEAEK; VSKAVDASKK; GEALSLFFQK; PTEQQSLGFK; FSCKQFGDVK; ESNNELKNLK; DAFGADPYKK; VVDAFGADPY; PVKCNEEIFK; LGFKEYVCYK; LLMLLAVFSCK; SIVSKAVDASK; VVDAFGADPYK; SLGFKEYVCYK; MSVNGREEALK; IVSKAVDASKK; VSDVVKDLIPK; LTESESNNELK; KGEALSLFFQK; ALKAEYEKSYK; SYKEFFDWLSK; KVVDAFGADPY; SFDNISSIVSK; GIPLVVSDVVK; EISLTPEEAEK; VFSCKQFGDVK; RPTEQQSLGFK |
| | HLA-A2402 | FFQKVVDAF; EYEKSYKEF; SYKEFFDWL; EFISSFDNI; MMQRISILL; EYEKSYKEFF; LFFQKVVDAF;  ISILLMLLAVF |
| | HLA-A2902 | LGFKEYVCY;  SLGFKEYVCY; VVDAFGADPY; EQQSLGFKEY; KVVDAFGADPY |
| | HLA-A6801 | DAFGADPYK; EALSLFFQK; ESESNNELK; EAEKLESLK; EALKAEYEK; MLLAVFSCK; DNISSIVSK; DVVKDLIPK; VSKAVDASK; EEIFKVIKK; LSKDVNRQK; GFKEYVCYK; KAVDASKKR; SLTPEEAEK; KAEYEKSYK; DAFGADPYKK; YVCYKIKNSK; ESNNELKNLK; SVNGREEALK; IVSKAVDASK; FSCKQFGDVK; EEALKAEYEK; LGFKEYVCYK; VSKAVDASKK; LMLLAVFSCK; EEAEKLESLK; KAVDASKKRR; TESESNNELK; WLSKDVNRQK; ISLTPEEAEK;  MSVNGREEALK; EISLTPEEAEK; LTESESNNELK; EYVCYIKNSK; IVSKAVDASKK; FLKDAMSVNGR; SIVSKAVDASK; LLMLLAVFSCK; SLGFKEYVCYK; VSKAVDASKKR; VVDAFGADPYK; SYKEFFDWLSK; FFDWLSKDVNR |
| | HLA-B0702 | KPVKCNEEI; IPLVVSDVV; SVNGREEAL;  RPTEQQSLGF; |

| | | |
|---|---|---|
| | | KPVKCNEEIF; KIKNSKGEAL; IPKEISLTPE; TPEEAEKLESL; KKRRPTEQQSL |
| | HLA-B0801 | IFKVIKKVL; FFQKVVDAF; MMQRISILL; KIKNSKGEAL; YKIKNSKGEAL; MMQRISILLML |
| | HLA-B1501 | RQKEFISSF; QQSLGFKEY; KQFGDVKSL; MQRISILLM; ILLMLLAVF; MMQRISILL; FFQKVVDAF; LGFKEYVCY; ALKAEYEKSY; MQRISILLML; EQQSLGFKEY; MMQRISILLM; LLAVFSCKQF; VVDAFGADPY; LFFQKVVDAF; NSKGEALSLF; KVVDAFGADPY; ISILLMLLAVF; LSKDVNRQKEF; MQRISILLMLL; MLLAVFSCKQF; SLFFQKVVDAF; AMSVNGREEAL; MMQRISILLML; QSLGFKEYVCY; NSKGEALSLFF; KQFGDVKSLTE; VNRQKEFISSF |
| | HLA-B2705 | QRISILLML; RQKEFISSF; RRPTEQQSL; KQFGDVKSL; QRISILLMLL; NRQKEFISSF; GREEALKAEY; KRRPTEQQSL; RRPTEQQSLGF; QRISILLMLLA |
| | HLA-B3501 | FFQKVVDAF; VDAFGADPY; LAVFSCKQF; LKVFLKDAM; ILLMLLAVF; IPLVVSDVV; QQSLGFKEY; RPTEQQSLGF; KPVKCNEEIF; VVDAFGADPY; LFFQKVVDAF; MSVNGREEAL; SILLMLLAVF; MMQRISILLM; TPEEAEKLES; KVVDAFGADPY; EALKAEYEKSY; TPEEAEKLESL; EAEKLESLKVF; ISILLMLLAVF; ESLKVFLKDAM; SLFFQKVVDAF |
| | HLA-B4403 | TEIDSGNGI; AEKLESLKV; KEISLTPEE; AEKLESLKVF; AEYEKSYKEF; KEFISSFDNI; KEISLTPEEA; EEIFKVIKKV; TEIDSGNGIPL; KEFISSFDNIS; AEYEKSYKEFF; AEKLESLKVFL; EEIFKVIKKVL; KEFFDWLSKDV |
| | HLA-B5101 | IPLVVSDVV; IPKEISLTP; IPLVVSDVVK; DPYKKDNDESV; IPLVVSDVVKD |
| | HLA-B5701 | KSYKEFFDW; |
| AAT93789.1\| lipoprotein BBA36 (homolog 67%)[Borrelia garinii PBi]; Seq33 | HLA-A0101 | VADAFGTQEY; FTESENNNEL; ATDLEDDNSF; |
| | HLA-A0201 | SILSILLLL; KSLGFNEYV; ILSILLLLL; SLTEVATDL; RISILSILL; ILLLLLLFS; LLLLLLLFSC; RLEALKTFL; KQYGDVKSL; KCNEEIFKV; LTSEEFERL; SILSILLLLL; ILSILLLLLL; VLTSEEFERL; KTFLKDAMGV; RISILSILLL; SLFFQKVADA; SILSILLLLLL; FVSFFNNICGI; LLFSCKQYGDV; RISILSILLLL |
| | HLA-A0301 | LLLLLLFSCK; KEFFDWLSK; ITKAVDASK; LEALKTFLK; RLEALKTFLK; LLLLLLLFSCK; LLLLFSCKQY; NSYNLNSNNK; IITKAVDASK; ADAFGTQEYK; SLGFNEYVCY; ITKAVDASKK; GVNGREGDTK; SFASGSVESK; WLSKDVNRQK; TKAEYEKSYK; ILLLLLLFSCK; NSFASGSVESK; IITKAVDASKK; GIITKAVDASK; KVADAFGTQEY; FFNNICGIITK; ASKKRYNSNPK; RISILSILLLL |
| | HLA-A1101 | LLLLLFSCK; ITKAVDASK; KEFFDWLSK; SYNLNSNNK; KAEYEKSYK; FASGSVESK; DAFGTQEYK; ESKDQIIEK; LSKDVNRQK; LEALKTFLK; VLTSEEFER; NSYNLNSNNK; RLEALKTFLK; LLLLLLFSCK; ITKAVDASKK; GVNGREGDTK; IITKAVDASK; SFASGSVESK; FSCKQYGDVK; SILLLLLLFS; ILLLLLLFSCK; NSFASGSVESK; SVESKDQIIEK; GIITKAVDASK; ASKKRYNSNPK; VADAFGTQEYK; TGDDLSLFFQK; KVADAFGTQEY; IITKAVDASKK; SYKEFFDWLSK; KSLGFNEYVCY; SENNNELANLK; RTGDDLSLFFQ; DTKAEYEKSYK; GFNEYVCYDIK |
| | HLA-A2402 | EYEKSYKEF; RYNSNPKSL; FFQKVADAF; SYKEFFDWL; EFVSFFNNI; IFKVIKRVF; SYNLNSNNK; SILLLLLLF; EYEKSYKEFF; LSILLLLLLF; LFFQKVADAF; RYNSNPKSLGF; EFERLEALKTF; ILSILLLLLLF |
| | HLA-A2902 | LGFNEYVCY; SLGFNEYVCY; VADAFGTQEY; LLLLFSCKQY; KVADAFGTQEY; ELANLKNLSY; KSLGFNEYVCY; ILSILLLLLLF |
| | HLA-A6801 | DAFGTQEYK; YVCYDIKTR; ITKAVDASK; FASGSVESK; ESKDQIIEK; VLTSEEFER; EEIFKVIKR; NNNELANLK; EFERLEALK; LSKDVNRQK; |

| | | |
|---|---|---|
| | | SYNLNSNNK; EKCNEEIFK; NNICGIITK; KAVDASKKR; LEALKTFLK; DDLSLFFQK; TKAVDASKK; KAEYEKSYK; NSYNLNSNNK; ITKAVDASKK; EYVCYDIKTR; IITKAVDASK; ENNNELANLK; EEFERLEALK; FSCKQYGDVK; SFASGSVESK; TKAEYEKSYK; PVLTSEEFER; WLSKDVNRQK; TKAVDASKKR; RLEALKTFLK; FNEYVCYDIK; NSFASGSVESK; DTKAEYEKSYK; ITKAVDASKKR; DAFGTQEYKNK; IITKAVDASKK; FLKDAMGVNGR; FFNNICGIITK; SVESKDQIIEK; ELANLKNLNSY; GPVLTSEEFER; GIITKAVDASK; VADAFGTQEYK; LNSYNLNSNNK; SYKEFFDWLSK; MGVNGREGDTK; FFDWLSKDVNR; NEYVCYDIKTR |
| | HLA-B0702 | GPVLTSEEF; KPEKCNEEI; KPEKCNEEIF; KTRTGDDLSL; KKRYNSNPKSL |
| | HLA-B0801 | IFKVIKRVF; FFQKVADAF; ; |
| | HLA-B1501 | RQKEFVSFF; KQYGDVKSL; LLLFSCKQY; LGFNEYVCY; QIIEKGPVL; FFQKVADAF; IFKVIKRVF; NSNPKSLGF; LANLKNLNSY; LLLLFSCKQY; LFFQKVADAF; LSILLLLLF; YNSNPKSLGF; RTGDDLSLFF; SLGFNEYVCY; VADAFGTQEY; KVADAFGTQEY; LSKDVNRQKEF; KSLGFNEYVCY; SLFFQKVADAF; ELANLKNLNSY; ILSILLLLLF; LLLLLFSCKQY; KTRTGDDLSLF; KQYGDVKSLTE; VATDLEDDNSF |
| | HLA-B2705 | KRISILSIL; NRQKEFVSF; KRYNSNPKS; KQYGDVKSL; RQKEFVSFF; ERLEALKTF; TRTGDDLSL; KRYNSNPKSL; KRISILSILL; GREGDTKAEY; NRQKEFVSFF; KRISILSILLL; ERLEALKTFLK; TRTGDDLSLFF |
| | HLA-B3501 | GPVLTSEEF; FFQKVADAF; TDLEDDNSF; ADAFGTQEY; NPKSLGFNEY; VADAFGTQEY; LANLKNLNSY; LFFQKVADAF; KPEKCNEEIF; KAVDASKKRY; FTESENNNEL; KVADAFGTQEY; EALKTFLKDAM; VATDLEDDNSF; ELANLKNLNSY; SLFFQKVADAF; DVNRQKEFVSF; EFERLEALKTF; NGREGDTKAEY |
| | HLA-B4403 | EEFERLEAL; SENNNELAN; REGDTKAEY; TEVATDLED; AEYEKSYKEF; SENNNELANL; KEFVSFFNNI; EEFERLEALK; EEIFKVIKRV; EEIFKVIKRVF; EEFERLEALKT; AEYEKSYKEFF; KEFFDWLSKDV; SENNNELANLK |
| | HLA-B5101 | |
| | HLA-B5701 | KSYKEFFDW; KSLGFNEYVCY |
| NP_045739.1 \| BBA66 antigen, P35, putative [Borrelia burgdorferi B31]; Seq34 | HLA-A0101 | KIEIFNQDY; LSDSKSLAEY; TIDANLNEDY; SSAKVYDRSY; CTIDANLNEDY; LLDVISSAKVY; NSGLPTFALNY; ISSAKVYDRSY; SLSDSKSLAEY; LSDSKSLAEYI |
| | HLA-A0201 | FLFSCTIDA; KMKDSAFEL; LMDFDQYKI; QLQSLSFSA; KLLGLFLFS; SVLEAYISI; QLNSNTPEA; ELLDVISSA; KINSFDFTM; GLFLFSCTI; VLEAYISIM; SLSDSKSLA; NMQNARQSV; FALNYSFSQ; KLQTLKNEL; NSFDFTMKL; TQISFELAL; ALMDFDQYK; SLTKQRIPI; MISGLGTQI; LIQLKLLGL; ALMDFDQYKI; LLDVISSAKV; GLGTQISFEL; KMKDSAFELL; LMISGLGTQI; KLLGLFLFSC; KLQTLKNELI; FKMKDSAFEL; RQADGLIANA; SVLEAYISIM; NLNEDYKNKV; IQLKLLGLFL; ALNYSFSQPT; LLGLFLFSCT; RIPIQAVTTV; LIRELMISGL; FLFSCTIDANL; ALYNENQNHSL; KVYDRSYAPQL; SLIRELMISGL; KINSFDFTMKL; SLAEYIKKRYL; KLLGLFLFSCT; ELLDVISSAKV; SLSFSADLSNL; LLGLFLFSCTI; QLQSLSFSADL; GLFLFSCTIDA; FALNYSFSQPT; LIANASSNSSL; ALEEINKKIEI; GLGTQISFELA; YALMDFDQYKI; FKMKDSAFELL; MQNARQSVLEA; QISFELALEEI; RQSVLEAYISI; KAADDQETTSA; SIMEALYNENQ |
| | HLA-A0301 | KIKPLIQLK; KSLAEYIKK; KTVAAAPNK; LLDVISSAK; QTTTSSGSK; KTQNNFGFR; FTMKLKELK; KLLGLFLFS; ALMDFDQYK; RSYAPQLNS; KLNQILDKR; AARAASLTK; LIRAISEEK; GLPTFALNY; ETYDQFKMK; RAASLTKQR; SLAEYIKKR; ALYNENQNH; ELLDVISSAK; |

|  |  | |
|---|---|---|
|  |  | KINSFDFTMK; TAARAASLTK; FSADLSNLPK; NSFDFTMKLK; VQTTTSSGSK; SSGSKLQTLK; TMKLKELKSK; KSKLNQILDK; ELIRAISEEK; SSNSSLSDSK; KLNQILDKRK; KVYDRSYAPQ; SKSLAEYIKK; SLAEYIKKRY; SADTNSNAAK; KNKVKGILNK; ELKKQSQAAK; MKIKPLIQLK; AVTTVPGNTR; TTAARAASLTK; TSSGSKLQTLK; ALNYSFSQPTR; AVQTTTSSGSK; SQNQPQTTPNK; FTMKLKELKSK; ASSNSSLSDSK; TSADTNSNAAK; LIRAISEEKNK; AAKTVAAAPNK; SLSDSKSLAEY; SFSADLSNLPK; EIFNQDYLNAK; KLQTLKNELIR; KFYALMDFDQY; KSLAEYIKKRY; LSNLPKTTAAR |
|  | HLA-A1101 | KTVAAAPNK; ALMDFDQYK; KSLAEYIKK; KTQNNFGFR; SADLSNLPK; QTTTSSGSK; FTMKLKELK; KIKPLIQLK; AARAASLTK; ETYDQFKMK; ISSAKVYDR; RAISEEKNK; SFDFTMKLK; SGSKLQTLK; LLDVISSAK; LIRAISEEK; LALEEINKK; SNSSLSDSK; NQPQTTPNK; RAASLTKQR; VTTVPGNTR; QTLKNELIR; SIMEALYNE; KLNQILDKR; INSFDFTMK; KVYDRSYAP; GLPTFALNY; SSNSSLSDSK; KINSFDFTMK; NSFDFTMKLK; TAARAASLTK; SSGSKLQTLK; FSADLSNLPK; YALMDFDQYK; SADTNSNAAK; KSKLNQILDK; VQTTTSSGSK; KLNQILDKRK; AVTTVPGNTR; KVYDRSYAPQ; TMKLKELKSK; ELLDVISSAK; KSLAEYIKKR; VISSAKVYDR; IFNQDYLNAK; ELIRAISEEK; MKIKPLIQLK; KTQNNFGFRE; TTAARAASLTK; AVQTTTSSGSK; TSSGSKLQTLK; ASSNSSLSDSK; TSADTNSNAAK; SQNQPQTTPNK; SFSADLSNLPK; TIDANLNEDYK; AAKTVAAAPNK; FTMKLKELKSK; ALNYSFSQPTR; EIFNQDYLNAK; SLSDSKSLAEY; TQNNFGFRETY; KFYALMDFDQY; AKINSFDFTMK; LSNLPKTTAAR; SFELALEEINK; KSLAEYIKKRY; LIRAISEEKNK; KLQTLKNELIR; SIMEALYNENQ; NSGLPTFALNY; INSFDFTMKLK |
|  | HLA-A2402 | IQLKLLGLF; DYKNKVKGI; DYLNAKINSF; VYDRSYAPQL; QYKIEQFGSI; EWSRQADGLI; LYNENQNHSL; FYALMDFDQY; DYKNKVKGIL; RYLDNMQNAR; LYNENQNHSLI; IQLKLLGLFLF; EYIKKRYLDNM; NFGFRETYDQF; IFNQDYLNAKI; FYALMDFDQYK; SYAPQLNSNTP |
|  | HLA-A2902 | GLPTFALNY; PTFALNYSF; YALMDFDQY; DVISSAKVY; KIEIFNQDY; IDANLNEDY; FYALMDFDQY; QFGSIMEALY; KFYALMDFDQY; CTIDANLNEDY; NSGLPTFALNY; TQNNFGFRETY; YLNAKINSFDF |
|  | HLA-A6801 | ETYDQFKMK; FTMKLKELK; NTPEAENER; NYSFSQPTR; DANLNEDYK; QTTTSSGSK; KTVAAAPNK; KTQNNFGFR; RAASLTKQR; ISSAKVYDR; SLAEYIKKR; QTLKNELIR; LIRAISEEK; ELALEEINK; NLPKTTAAR; VTTVPGNTR; YLDNMQNAR; NIPIADNDK; ALMDFDQYK; DVISSAKVY; NNFGFRETY; KLNQILDKR; INSFDFTMK; LLDVISSAK; NSFDFTMKL; ENQNHSLIR; TTSSGSKLQ; QTNSSSAVQ; EIFNQDYLN; LALEEINKK; NSFDFTMKLK; ELIRAISEEK; TAARAASLTK; FSADLSNLPK; ELLDVISSAK; YALMDFDQYK; DSKSLAEYIK; SSNSSLSDSK; LNYSFSQPTR; ELALEEINKK; NKTQNNFGFR; VISSAKVYDR; KINSFDFTMK; AVTTVPGNTR; TTAARAASLT; ELKKQSQAAK; DFTMKLKELK; MKIKPLIQLK; SNTPEAENER; TTTSSGSKLQ; FYALMDFDQY; SSGSKLQTLK; NENQNHSLIR; SADTNSNAAK; ETYDQFKMKD; TTAARAASLTK; DVISSAKVYDR; EIFNQDYLNAK; TSADTNSNAAK; TSSGSKLQTLK; NSNTPEAENER; FYALMDFDQYK; FTMKLKELKSK; LSNLPKTTAAR; QAVTTVPGNTR; NTPEAENERNK; TIDANLNEDYK; EQFGSIMEALY; DSKSLAEYIKK; FGFRETYDQFK; ALNYSFSQPTR; CTIDANLNEDY; DANLNEDYKNK; FDFTMKLKELK; QILDKRKEWSR; INSFDFTMKLK; AAKTVAAAPNK; NSFDFTMKLKE; KSKLNQILDKR; SFSADLSNLPK; AVQTTTSSGSK; LIRAISEEKNK; ASSNSSLSDSK; AARAASLTKQR; TTVPGNTRTFN |
|  | HLA-B0702 | SPQLQSLSF; APSPQLQSL; IPIQAVTTV; KPLIQLKLL; APQLNSNTP; KPEKCNEEIF; KTRTGDDLSL; APSPQLQSLSF; APQLNSNTPEA; |

|  |  |  |
|---|---|---|
|  |  | SPQLQSLSFSA; KPLIQLKLLGL; IPIADNDKVAA; IPIQAVTTVPG; AASLTKQRIPI; LPKTTAARAAS; LIANASSNSSL; TPEAENERNKF; KVYDRSYAPQL; APNKGSQNQPQ |
|  | HLA-B0801 | QFKMKDSAF; ELKKQSQAA; NERNKFYAL; QLKLLGLFL; KLKELKSKL; YIKKRYLDNM; ELKSKLNQIL; YDQFKMKDSAF |
|  | HLA-B1501 | YLNAKINSF; MQNARQSVL; QQAAPSPQL; KQRIPIQAV; KMKDSAFEL; TQISFELAL; IQLKLLGLF; SAKVYDRSY; SQAAKTVAA; QQTNSSSAV; QFKMKDSAF; RQQTNSSSA; RQADGLIAN; TVPGNTRTF; PTFALNYSF; KQSQAAKTV; RQSVLEAYI; SGLGTQISF; DVISSAKVY; TTAARAASL; RQQTNSSSAV; EQFGSIMEAL; QLKLLGLFLF; ISGLGTQISF; SQAAKTVAAA; LQSLSFSADL; TTVPGNTRTF; SSAKVYDRSY; NSRNSGLPTF; KQRIPIQAVT; SLAEYIKKRY; KMKDSAFELL; RQADGLIANA; DQFKMKDSAF; NARQSVLEAY; IANASSNSSL; NQQAAPSPQL; NMQNARQSVL; IQLKLLGLFL; NQNHSLIREL; QNNFGFRETY; RTFNSRNSGL; LMISGLGTQI; YLNAKINSFDF; TQNNFGFRETY; EQFGSIMEALY; MISGLGTQISF; ISSAKVYDRSY; SLSDSKSLAEY; IQLKLLGLFLF; RQADGLIANAS; VTTVPGNTRTF; RQSVLEAYISI; MQNARQSVLEA; ALYNENQNHSL; KQSQAAKTVAA; LLDVISSAKVY; KQRIPIQAVTT; RQQTNSSSAVQ; QNARQSVLEAY; VQTTTSSGSKL; GLPTFALNYSF; KSLAEYIKKRY; FNSRNSGLPTF; KVYDRSYAPQL |
|  | HLA-B2705 | SRNSGLPTF; ERNKFYALM; KRKEWSRQA; KRYLDNMQN; ARQSVLEAY; KRYLDNMQNA; ARAASLTKQR; RQQTNSSSAV; KRYLDNMQNAR; QRIPIQAVTTV; ERNKFYALMDF; RQSVLEAYISI; SRNSGLPTFAL; FRETYDQFKMK |
|  | HLA-B3501 | YALMDFDQY; SPQLQSLSF; DVISSAKVY; FGSIMEALY; IPIQAVTTV; EAENERNKF; APSPQLQSL; SAKVYDRSY; TVPGNTRTF; LAEYIKKRY; LPTFALNYS; SADTNSNAA; YLNAKINSF; KINSFDFTM; FALNYSFSQ; NAKINSFDF; LPTFALNYSF; NARQSVLEAY; TTVPGNTRTF; FYALMDFDQY; EAENERNKFY; SVLEAYISIM; YKIEQFGSIM; LPKTTAARAA; IANASSNSSL; LSDSKSLAEY; IPIADNDKVA; QFGSIMEALY; LDVISSAKVY; NSRNSGLPTF; IPIQAVTTVP; APSPQLQSLSF; TPEAENERNKF; FALNYSFSQPT; NAKINSFDFTM; IPIADNDKVAA; IPIQAVTTVPG; MISGLGTQISF; LPKTTAARAAS; IADNDKVAAEL; EQFGSIMEALY; LLDVISSAKVY; QSVLEAYISIM; TQNNFGFRETY; QAAPSPQLQSL; SLSDSKSLAEY; QNARQSVLEAY; FNSRNSGLPTF |
|  | HLA-B4403 | AENERNKFY; EEINKKIEI; NENQNHSLI; EEINKKIEIF; AENERNKFYA; EEKNKTQNNF; RELMISGLGT; AELKKQSQAA; EEIFKVIKRVF; EEFERLEALKT; AEYEKSYKEFF; KEFFDWLSKDV; SENNNELANLK |
|  | HLA-B5101 | IPIQAVTTV; LPKTTAARA; LPTFALNYS; IPIADNDKV; APSPQLQSL; KPLIQLKLL; LPTFALNYSF; LPKTTAARAA; IPIQAVTTVP; LALEEINKKI; IPIQAVTTVPG; LPTFALNYSFS; KPLIQLKLLGL |
|  | HLA-B5701 | KTHNHGFRETY; QTQPSFVVPVY |
| AAT93824.1\| BBA66 antigen, P35, putative [Borrelia garinii PBi]; Seq35 | HLA-A0101 | QSNNVLTNY; YLNTIINSY; KIESFNTQY; LAEKKEWLNY; TIDANLNKDY; EAESERNRLY; NSQSNNVLTNY; CTIDANLNKDY; QTQPSFVVPVY; KTHNHGFRETY |
|  | HLA-A0201 | TLLDVISNI; KMKDSAFTL; QLSSNTPEA; TQPSFVVPV; ILAEKKEWL; KMQDARQSA; SLIISGLGI; SLESTLEEI; RQSALDLYL; WLNYADAII; SALDLYLNI; MMDFDQAKT; ALDLYLNIT; YADAIITNT; YAMMDFDQA; YTFSSSFSQ; ASAQAIQTV; YLNTIINSYT; LLDVISNISV; AMMDFDQAKT; KMKDSAFTLL; KMQDARQSAL; FTLLDVISNI; KIESFNTQYL; FKMKDSAFTL; QTQPSFVVPV; LIISGLGIQI; YTFKDKLKEL; LLNSSTDDQA; TLLDVISNIS; LIRSLIISGL; ISLESTLEEI; TLLDVISNISV; ALDLYLNITEI; SLIRSLIISGL; ILYQENQNHSL; |

| | | |
|---|---|---|
| | | RLYAMMDFDQA; QIASAQAIQTV; SLGQYIKNKYL; AMMDFDQAKTT; IISGLGIQISL; SLIISGLGIQI; SVFDRGSAPQL; YTFSSSFSQPT; IINSYTFKDKL; FKMKDSAFTLL; NLQGLNPANQV; YLNTIINSYTF; KTTEFGSIMNI; HQTQPSFVVPV; RQSALDLYLNI |
| | HLA-A0301 | KLNSILAEK; TIINSYTFK; ITNTSSNSK; GLQKNTQSK; KLKNNLLRR; SLGQYIKNK; YSGNSPLQK; LLRRIAEEK; STPNQSIIK; QSNNVLTNY; CTIDANLNK; ATKNKTNIK; KIESFNTQY; LQKNTQSKK; YIKNKYLDK; ETYDQFKMK; NLKDYKNK; YLNTIINSY; ISNISVFDR; NIKVAGLQK; NTIINSYTFK; IITNTSSNSK; IINSYTFKDK; SINTGSNATK; ATNVQATPPK; KLNSILAEKK; VYSGNSPLQK; NLLRRIAEEK; GLQKNTQSKK; SKLNSILAEK; TSTPNQSIIK; NSYTFKDKLK; QSLGQYIKNK; TNIKVAGLQK; QYIKNKYLDK; QYLNTIINSY; SQSNNVLTNY; NTGSNATKNK; SLGQYIKNKY; AGLQKNTQSK; VISNISVFDR; KTNIKVAGLQK; PVYSGNSPLQK; ISINTGSNATK; AIITNTSSNSK; LLNSSTDDQAK; GQYIKNKYLDK; ILAEKKEWLNY; LLRRIAEEKNK; TIINSYTFKDK; KTHNHGFRETY; NTSTPNQSIIK; YSGNSPLQKLK; IITNTSSNSKR; TQYLNTIINSY; HGFRETYDQFK; SSCTIDANLNK |
| | HLA-A1101 | TIINSYTFK; ITNTSSNSK; CTIDANLNK; AMMDFDQAK; KLNSILAEK; STLEEIEKK; STPNQSIIK; YSGNSPLQK; ATKNKTNIK; ETYDQFKMK; ISNISVFDR; SLGQYIKNK; GLQKNTQSK; YTFSSSFSQ; QSNNVLTNY; NIKVAGLQK; SIMNILYQE; KIESFNTQY; KLKNNLLRR; YIKNKYLDK; TNVQATPPK; NSSTDDQAK; ESTLEEIEK; ATNVQATPPK; SINTGSNATK; NTIINSYTFK; IITNTSSNSK; KLNSILAEKK; TSTPNQSIIK; IINSYTFKDK; YAMMDFDQAK; NSYTFKDKLK; ITNTSSNSKR; QSLGQYIKNK; QSNNVLTNYR; GLQKNTQSKK; VISNISVFDR; NTGSNATKNK; VYSGNSPLQK; SQSNNVLTNY; SVFDRGSAPQ; NLLRRIAEEK; SGNSPLQKLK; TNIKVAGLQK; SKLNSILAEK; TQPSFVVPVY; MSSCTIDANLN; SSCTIDANLNK; KTNIKVAGLQK; AIITNTSSNSK; ISINTGSNATK; TIINSYTFKDK; TIDANLNKDYK; PVYSGNSPLQK; NTSTPNQSIIK; SLESTLEEIEK; GQYIKNKYLDK; SQSNNVLTNYR; SSNTPEAESER; YSGNSPLQKLK; TQYLNTIINSY; QTQPSFVVPVY; LLNSSTDDQAK; KTHNHGFRETY; PATNVQATPPK; VAGLQKNTQSK; ESKLNSILAEK; IITNTSSNSKR; SIMNILYQENQ; HGFRETYDQFK |
| | HLA-A2402 | EWLNYADAI; NTIINSYTF; DYKNKVEEL; VYSGNSPLQ; EFGSIMNIL; NYRHQTQPSF; EWLNYADAII; LYQENQNHSL; SFNTQYLNTI; DYKNKVEELL; VYSGNSPLQK; QYLNTIINSY; QYIKNKYLDK; PQQYTFSSSF; LYQENQNHSLI; VYSGNSPLQKL; SFNTQYLNTII; QYIKNKYLDKM; YLNTIINSYTF; NYRHQTQPSFV; SYTFKDKLKEL; LYAMMDFDQAK; SFSQPTSQTNF |
| | HLA-A2902 | YLNTIINSY; KIESFNTQY; QSNNVLTNY; FGSIMNILY; EFGSIMNILY; QYLNTIINSY; SIIKPQQYTF; SQSNNVLTNY; KKIESFNTQY; SLGQYIKNKY; TQPSFVVPVY; YLNTIINSYTF; CTIDANLNKDY; ILAEKKEWLNY; NSQSNNVLTNY; SFSQPTSQTNF; TQYLNTIINSY; KTHNHGFRETY; QTQPSFVVPVY |
| | HLA-A6801 | ETYDQFKMK; TIINSYTFK; NTPEAESER; CTIDANLNK; YTFSSSFSQ; ITNTSSNSK; ISNISVFDR; DANLNKDYK; ESTLEEIEK; TNTSSNSKR; STLEEIEKK; SNNVLTNYR; LYLNITEIR; NKTHNHGFR; STPNQSIIK; NSSTDDQAK; TNVQATPPK; KLNSILAEK; NTIINSYTF; ESFNTQYLN; NIKVAGLQK; LLRRIAEEK; YLDKMQDAR; YSGNSPLQK; DVISNISVF; ENQNHSLIR; QSNNVLTNY; INTGSNATK; SYTFKDKLK; YLNTIINSY; ATKNKTNIK; NTIINSYTFK; ITNTSSNSKR; QSNNVLTNYR; DLYLNITEIR; NSYTFKDKLK; YAMMDFDQAK; ESTLEEIEKK; VISNISVFDR; NTGSNATKNK; TSTPNQSIIK; NATKNKTNIK; IITNTSSNSK; SINTGSNATK; IINSYTFKDK; TNIKVAGLQK; |

|  |  |  |
|---|---|---|
|  |  | ATNVQATPPK; NLLRRIAEEK; SNTPEAESER; YTFSSSFSQP; QYIKNKYLDK; KLNSILAEKK; TPEAESERNR; QSLGQYIKNK; ETYDQFKMKD; EFGSIMNILY; DVISNISVFDR; IITNTSSNSKR; ESKLNSILAEK; NTPEAESERNR; SSNTPEAESER; NTSTPNQSIIK; TIINSYTFKDK; SSCTIDANLNK; ISINTGSNATK; LNTIINSYTFK; TIDANLNKDYK; EKNKTHNHGFR; HGFRETYDQFK; LYAMMDFDQAK; SQSNNVLTNYR; DANLNKDYKNK; AIITNTSSNSK; PVYSGNSPLQK; NKYLDKMQDAR; YTFKDKLKELE; LLNSSTDDQAK; QTQPSFVVPVY; CTIDANLNKDY; TEFGSIMNILY; YSGNSPLQKLK; KTNIKVAGLQK; YTFSSSFSQPT; SLESTLEEIEK; PATNVQATPPK; NSQSNNVLTNY |
| | HLA-B0702 | SPATNVQAT; VPVYSGNSP; KPQQYTFSS; VPNNTSTPN; SERNRLYAM; SPVASPATN; NPMQIANQA; APQLSSNTP; SKRNDPQSL; VPVYSGNSPL; SPVASPATNV; KPQQYTFSSS; APQLSSNTPE; SPLQKLKNNL; KMQDARQSAL; KPQQYTFSSSF; SPLQKLKNNLL; APQLSSNTPEA; SPVASPATNVQ; TPEAESERNRL; PPKQIASAQAI; SPATNVQATPP; VPVYSGNSPLQ; VASSASQASPV; VPNNTSTPNQS |
| | HLA-B0801 | QFKMKDSAF; IIKPQQYTF; YIKNKYLDKM; YDQFKMKDSAF |
| | HLA-B1501 | QQYTFSSSF; YLNTIINSY; KQIASAQAI; NQVNPGNPM; RQSALDLYL; KMKDSAFTL; YQENQNHSL; IQISLESTL; LQKLKNNLL; SQPTSQTNF; MQDARQSAL; SQVASSASQ; QFKMKDSAF; QSNNVLTNY; IIKPQQYTF; GQYIKNKYL; HQTQPSFVV; KNKTHNHGF; QSIIKPQQY; HNHGFRETY; TQSKKNNNL; KMQDARQSA; MQIANQANQ; SQSNNVLTNY; KMQDARQSAL; PQQYTFSSSF; TQPSFVVPVY; FSQPTSQTNF; KMKDSAFTLL; DQFKMKDSAF; KQIASAQAIQ; SIIKPQQYTF; NQNHSLIRSL; SQASPVASPA; NQSIIKPQQY; MQIANQANQA; IQTVPNNTST; SQVASSASQA; TQYLNTIINSY; YLNTIINSYTF; QTQPSFVVPVY; KTHNHGFRETY; ILAEKKEWLNY; RQSALDLYLNI; ILYQENQNHSL; SQVASSASQAS; KQIASAQAIQT; MQIANQANQAN; SQASPVASPAT; HQTQPSFVVPV; QSIIKPQQYTF; SVFDRGSAPQL; LLDVISNISVF; QQYTFSSSFSQ; TNYRHQTQPSF; SLIRSLIISGL; AMMDFDQAKTT |
| | HLA-B2705 | RRIAEEKNK; YRHQTQPSF; NRLYAMMDF; QQYTFSSSF; RQSALDLYL; ERNRLYAMM; IRSLIISGL; KRNDPQSLGQ; ARQSALDLYL; RRIAEEKNKTH; KRNDPQSLGQY; GQYIKNKYLDK; ERNRLYAMMDF; RQSALDLYLNI; YRHQTQPSFVV; FRETYDQFKMK |
| | HLA-B3501 | QPSFVVPVY; YLNTIINSY; NPANQVNPG; SPATNVQAT; QANQASQAS; QASQASQAS; DVISNISVF; QANQANQAS; NTIINSYTF; FGSIMNILY; NPMQIANQA; QASQASQVA; NQVNPGNPM; VPNNTSTPN; SASQASPVA; VASSASQAS; IANQANQAN; WLNYADAII; QANQANQAN; QANQANQAN; QANQANQAN; YAMMDFDQA; YQENQNHSL; VASPATNVQ; FSSSFSQPT; VPVYSGNSPL; DARQSALDLY; NPMQIANQAN; EAESERNRLY; QASPVASPAT; EFGSIMNILY; DFDQAKTTEF; YAMMDFDQAK; QASQASQVAS; TPNQSIIKPQ; SASQASPVAS; YADAIITNTS; FSQPTSQTNF; MSSCTIDANL; QASQVASSAS; ESERNRLYAM; THNHGFRETY; LDVISNISVF; FNNSQSNNVL; NPANQVNPGN; KPQQYTFSSSF; YLNTIINSYTF; PANQVNPGNPM; TPEAESERNRL; VASPATNVQAT; SPVASPATNVQ; QTQPSFVVPVY; MDFDQAKTTEF; TQYLNTIINSY; QAKTTEFGSIM; LLDVISNISVF; YAMMDFDQAKT; VPVYSGNSPLQ; IANQANQANQA; NFNNSQSNNVL; VPNNTSTPNQS; QANQANQANQA; QANQANQANQA; QANQANQANQA; SPLQKLKNNLL |
| | HLA-B4403 | AEKKEWLNY; EEIEKKIES; AESERNRLY; TEFGSIMNI; KELESKLNS; QENQNHSLI; EEINKKIEIF; AENERNKFYA; EEKNKTQNNF; RELMISGLGT; AELKKQSQAA; TEFGSIMNILY; EEKNKTHNHGF; AESERNRLYAM; EEIEKKIESFN; KELESKLNSIL; AEKKEWLNYAD; EELLNSSTDDQ |

| | HLA-B5101 | VPVYSGNSP; DPQSLGQYI; TPPKQIASA; SPVASPATNV; VPVYSGNSPL; PPKQIASAQAI |
|---|---|---|
| | HLA-B5701 | FSQPTSQTNF; NSILAEKKEW; |
| NP_045742.1 \| hypothetical protein BBA69 [Borrelia burgdorferi B31]; Seq36 | HLA-A0101 | LLDTYIRAY; LAEHFNKYY; DSDSLQSAF; ILEKLINNY; DSDSLQSAFY; KTLNKTLEDY; |
| | HLA-A0201 | FLYRIALDI; NLLDTYIRA; FMGTTASEL; KMLLKRFLL; NIITMILTL; ALLEQVKSA; TMILTLICI; TLKEILEKL; KLNIIKINI; IITMILTLI; IALDIQLKL; KINIITMIL; FLLSSLDYK; SINEKLDTL; MLLKRFLLS; IAANFLYRI; LTLICISCA; SLDYKKENI; KIAANFLYRI; ALLEQVKSAL; NIITMILTLI; MLLKRFLLSS; ILTLICISCA; ITMILTLICI; KLNIIKINII; NQYDIQIAKI; LLKRFLLSSL; RIALDIQLKL; FLYRIALDIQ; NIIKINIITM; FLYRIALDIQL; MLLKRFLLSSL; LLDTYIRAYEL; MILTLICISCA; FMGTTASELKA; KINIITMILTL; NIIKINIITMI; IITMILTLICI; KLNIIKINIIT; KMLLKRFLLSS |
| | HLA-A0301 | KLKKNTRLK; KVLAEHFNK; RIALDIQLK; KIAANFLYR; FLLSSLDYK; KSALQLQEK; CISCAPFNK; KANENTKLK; MLLKRFLLS; VLAEHFNKY; LLSSLDYKK; NQYDIQIAK; QLKLEKHLK; ALQLQEKFK; RFLLSSLDY; NTNNIQENK; ETLKEILEK; RFLLSSLDYK; KLKKNTRLKK; ALDIQLKLEK; FMGTTASELK; ALQLQEKFKK; LINNYENDPK; IQLKLEKHLK; KTLNKTLEDY; FLLSSLDYKK; VLAEHFNKYY; KANENTKLKK; QEKFKKTLNK; SALQLQEKFK; TASELKAIGK; LAEHFNKYYK; KVLAEHFNKY; CAPFNKINPK; ICISCAPFNK; NLLDTYIRAY; ANENEKMLLK; KRFLLSSLDY; TKLKKNTRLK; KMLLKRFLLS; KNQYDIQIAK; VLAEHFNKYYK; KSALQLQEKFK; KINPKANENTK; KLDTLSKENSK; SINEKLDTLSK; KFMGTTASELK; RAYELANENEK; KVLAEHFNKYY; TTASELKAIGK; TLNKTLEDYRK; KLINNYENDPK; RFLLSSLDYKK; LICISCAPFNK; LANENEKMLLK; IALDIQLKLEK; QVKSALQLQEK; KTLNKTLEDYR; NTKLKKNTRLK |
| | HLA-A1101 | KVLAEHFNK; KIAANFLYR; CISCAPFNK; RIALDIQLK; KSALQLQEK; NTNNIQENK; NQYDIQIAK; ASELKAIGK; KANENTKLK; FLLSSLDYK; LLSSLDYKK; ETLKEILEK; ALQLQEKFK; APFNKINPK; LQLQEKFKK; KLKKNTRLK; AEHFNKYYK; QLKLEKHLK; DTLSKENSK; TASELKAIGK; SALQLQEKFK; KANENTKLKK; KVLAEHFNKY; ALQLQEKFKK; LINNYENDPK; RFLLSSLDYK; LAEHFNKYYK; FLLSSLDYKK; IQLKLEKHLK; ALDIQLKLEK; AIGKELEDRK; KLKKNTRLKK; ICISCAPFNK; FMGTTASELK; CAPFNKINPK; KTLNKTLEDY; TLNKTLEDYR; SINEKLDTLSK; TTASELKAIGK; LICISCAPFNK; RAYELANENEK; SALQLQEKFKK; VLAEHFNKYYK; IALDIQLKLEK; KSALQLQEKFK; TLNKTLEDYRK; KLINNYENDPK; KTLNKTLEDYR; KSGDLGASDEK; KINPKANENTK; KFMGTTASELK; LANENEKMLLK; KAIGKELEDRK; KVLAEHFNKYY; RFLLSSLDYKK; QVKSALQLQEK; KLDTLSKENSK; NTKLKKNTRLK; NIETLKEILEK; LQEKFKKTLNK; DIQLKLEKHLK; KRFLLSSLDYK |
| | HLA-A2402 | DYRKNTNNI; TYIRAYELA; KYYKDSDSL; QYDIQIAKI; NFLYRIALDI; DYKKENIETL; KFMGTTASEL; LYRIALDIQL; ITMILTLICI; |
| | HLA-A2902 | VLAEHFNKY; RFLLSSLDY; PKIAANFLY; LAEHFNKYY; ILEKLINNY; VLAEHFNKYY; KVLAEHFNKY; NLLDTYIRAY; TLICISCAPF; EILEKLINNY; KVLAEHFNKYY |
| | HLA-A6801 | NTNNIQENK; ETLKEILEK; NTKLKKNTR; CISCAPFNK; KIAANFLYR; MGTTASELK; NQYDIQIAK; DTLSKENSK; LLSSLDYKK; FLLSSLDYK; RIALDIQLK; KVLAEHFNK; DTYIRAYEL; EKFKKTLNK; ENEKMLLKR; NPKANENTK; ENIQNFKDK; LNKTLEDYR; QLKLEKHLK; KSALQLQEK; KANENTKLK; ESNLLDTYIR; TLNKTLEDYR; TASELKAIGK; LAEHFNKYYK; LINNYENDPK; CAPFNKINPK; FMGTTASELK; FLLSSLDYKK; SALQLQEKFK; ENTKLKKNTR; NPGENIQNFK; NKVLAEHFNK; KAIGKELEDR; ICISCAPFNK; DTYIRAYELA; |

| | | |
|---|---|---|
| | | DLEALLEQVK; TTASELKAIGK; NTKLKKNTRLK; LICISCAPFNK; RAYELANENEK; DPKIAANFLYR; QVKSALQLQEK; KTLNKTLEDYR; TLNKTLEDYRK; LANENEKMLLK; NIETLKEILEK; VLAEHFNKYYK; IALDIQLKLEK; SALQLQEKFKK; DIQLKLEKHLK; EESNLLDTYIR; SINEKLDTLSK; ENKVLAEHFNK; DYKKENIETLK; NPKANENTKLK; KSALQLQEKFK; KFMGTTASELK |
| | HLA-B0702 | KPANPGENI; AANFLYRIAL; NPKANENTKL; IAANFLYRIAL |
| | HLA-B0801 | FKKTLNKTL; ELKAIGKEL; HLKSINEKL; LLKRFLLSSL; QLQEKFKKTL; KFKKTLNKTL; MLLKRFLLSSL; FLYRIALDIQL; IIKINIITMIL |
| | HLA-B1501 | VLAEHFNKY; LICISCAPF; RFLLSSLDY; ILEKLINNY; FMGTTASEL; LLDTYIRAY; TLNKTLEDY; TLICISCAPF; VLAEHFNKYY; KVLAEHFNKY; NLLDTYIRAY; IQIAKITNEE; LLKRFLLSSL; IQENKVLAEH; KTLNKTLEDY; KVLAEHFNKYY; IQENKVLAEHF; LTLICISCAPF; IQNFKDKSGDL; YENDPKIAANF; IQIAKITNEES; FLYRIALDIQL |
| | HLA-B2705 | YRIALDIQL; NQYDIQIAK; KRFLLSSLD; KRFLLSSLDY; YRIALDIQLK; YRIALDIQLKL; KRFLLSSLDYK; RKNQYDIQIAK |
| | HLA-B3501 | NPGENIQNF; LLDTYIRAY; LAEHFNKYY; EESNLLDTY; DPKIAANFL; VLAEHFNKY; LICISCAPF; LANENEKML; SALQLQEKF; DSDSLQSAF; NIITMILTL; SDSLQSAFY; DPKIAANFLY; NLLDTYIRAY; TLICISCAPF; LANENEKMLL; DSDSLQSAFY; AANFLYRIAL; NPKANENTKL; NIIKINIITM; VLAEHFNKYY; IAANFLYRIAL; EALLEQVKSAL; YKDSDSLQSAF; LTLICISCAPF; NKVLAEHFNKY; TNEESNLLDTY; PANPGENIQNF |
| | HLA-B4403 | KEILEKLIN; EESNLLDTY; SELKAIGKEL; EESNLLDTYI; KELEDRKNQY; KEILEKLINN; QENKVLAEHF; KENSKEDLEA; KEDLEALLEQ; KEILEKLINNY; YENDPKIAANF; KENIETLKEIL; NENEKMLLKRF; AEHFNKYYKDS |
| | HLA-B5101 | KPANPGENI; IAANFLYRI; |
| | HLA-B5701 | |
| AAT93826.1\| conserved hypothetical protein BBA69[Borrel ia garinii PBi]; Seq37; | HLA-A0101 | QLEKHINENY; |
| | HLA-A0201 | FIYDIPIII; ILQEILEKL; TLICISCAV; LLMRIESEL; KLNILTTTL; ILTTTLTLI; SLNYETEKI; NILTTTLTL; KLNIIKLNI; MQMKKIIYS; KQNKDLEPL; LTLICISCA; KILQEILEKL; ILNQKEIEEL; LTLICISCAV; KIAKNFIYDI; KIDNTQIDFL; NILTTTLTLI; FIYDIPIIIQ; KLNILTTTLT; IIYSSLNYET; TLTLICISCA; KLNIIKLNIL; QMKKIIYSSL; MQMKKIIYSS; LTTTLTLICI; LLMRIESELKI; MQMKKIIYSSL; KLNILTTTLTL; ILTTTLTLICI; TLTLICISCAV; FIYDIPIIIQS; ILNQKEIEELL; SLNYETEKIKI; KLETTLKILEA; KIIYSSLNYET; KLNIIKLNILT; QILNQKEIEEL; KLDKNLQHKKI |
| | HLA-A0301 | KIIYSSLNY; KLDKNLQHK; KILQEILEK; KTSAEQLEK; QIDFLKTFK; KTNKKENIK; ASKLETTLK; KMQMKKIIY; TLKILEAQK; KILEAQKEK; SSLNYETEK; NLQHKKIAK; LMRIESELK; QSRLDIISK; KIDPEPKSK; LLMRIESELK; TQIDFLKTFK; RLDIISKVIK; LICISCAVNK; KENFKKALNK; KNLQHKKIAK; TTLKILEAQK; TASKLETTLK; SLNYETEKIK; IQSRLDIISK; KLDKNLQHKK; KKIIYSSLNY; YSSLNYETEK; KENIKNFVNK; ISKVIKNPIK; KFQDLEPSKK; ELKIKENFKK; IKILQEILEK; KALNKTIDAY; IKTSAEQLEK; TLICISCAVNK; ATASKLETTLK; IISKVIKNPIK; IIQSRLDIISK; KIDNTQIDFLK; KIKILQEILEK; ILQEILEKLDK; QLEKHINENYK; ELLMRIESELK; NTQIDFLKTFK; TLKILEAQKEK; NIKTSAEQLEK; LMRIESELKIK |
| | HLA-A1101 | KTSAEQLEK; SSLNYETEK; KILQEILEK; KIIYSSLNY; ASKLETTLK; QIDFLKTFK; KTNKKENIK; KILEAQKEK; KFQDLEPSK; TLKILEAQK; KLDKNLQHK; QSRLDIISK; NLQHKKIAK; KIDPEPKSK; RIESELKIK; KMQMKKIIY; ICISCAVNK; TQIDFLKTFK; TTLKILEAQK; AVNKIDPEPK; TASKLETTLK; LICISCAVNK; LLMRIESELK; IQSRLDIISK; YSSLNYETEK; |

| | | |
|---|---|---|
| | | SLNYETEKIK; KLDKNLQHKK; KFQDLEPSKK; ISKVIKNPIK; KQNKDLEPLR; RLDIISKVIK; KENIKNFVNK; KENFKKALNK; AYNQDSENIK; ATASKLETTLK; TLICISCAVNK; IIQSRLDIISK; NTQIDFLKTFK; KIDNTQIDFLK; SSLNYETEKIK; IISKVIKNPIK; KIKILQEILEK; ILQEILEKLDK; ETTLKILEAQK; NIKTSAEQLEK; CAVNKIDPEPK; IYSSLNYETEK; ELLMRIESELK; SLPEDEKMQMK; TLKILEAQKEK; KSKTNKKENIK; QLEKHINENYK |
| | HLA-A2402 | NFIYDIPII; AYNQDSENI; NYETEKIKI; IYSSLNYET; KYPEATASK; KYPEATASKL; NFIYDIPIII; IYSSLNYETE; NYKEFNSLKPI; IYSSLNYETEK |
| | HLA-A2902 | KIIYSSLNY; EFNSLKPIY; KKIAKNFIY; KMQMKKIIY; DLEPLREKY; ; YKEFNSLKPIY |
| | HLA-A6801 | DNTQIDFLK; DIPIIIQSR; NYKEFNSLK; QIDFLKTFK; ENIKNFVNK; SSLNYETEK; TLKILEAQK; ELKIKENFK; ENFKKALNK; KTSAEQLEK; ETEKIKILQ; QNKDLEPLR; EPKSKTNKK; EKHINENYK; EIAKIDNTQ; EKENIEIAK; TTLKILEAQK; TASKLETTLK; YSSLNYETEK; ENYKEFNSLK; TQIDFLKTFK; LLMRIESELK; LICISCAVNK; YDIPIIIQSR; EKYPEATASK; ELKIKENFKK; AVNKIDPEPK; ISKVIKNPIK; ETTLKILEAQ; SLNYETEKIK; IDNTQIDFLK; ETTLKILEAQK; TLICISCAVNK; NTQIDFLKTFK; DAYNQDSENIK; ATASKLETTLK; ESELKIKENFK; NIKTSAEQLEK; IYSSLNYETEK; ELLMRIESELK; IISKVIKNPIK; CAVNKIDPEPK; NENYKEFNSLK; TLKILEAQKEK; NQKEIEELLMR; DPHDSLPEDEK; IIQSRLDIISK; QLEKHINENYK; SSLNYETEKIK |
| | HLA-B0702 | YPEATASKL; IPIIIQSRL; LPEDEKMQM; KIKENFKKAL; EPSKKQNKDL; NPIKDELQIL; IPIIIQSRLDI; YPEATASKLET |
| | HLA-B0801 | MKKIIYSSL; FKKALNKTI; KIKILQEIL; QMKKIIYSSL; KIKENFKKAL; IIKLNILTTTL |
| | HLA-B1501 | TQIDFLKTF; KIIYSSLNY; ALNKTIDAY; KMQMKKIIY; HINENYKEF; KQNKDLEPL; LQILNQKEI; KLNILTTTL; KKIAKNFIY; LQHKKIAKNF; QMKKIIYSSL; KALNKTIDAY; AQKEKENIEI; NQKEIEELLM; QLEKHINENY; MQMKKIIYSSL; EQLEKHINENY; KLNILTTTLTL; IAKIDNTQIDF |
| | HLA-B2705 | SRLDIISKV; MRIESELKI; MRIESELKIK; SRLDIISKVI; SRLDIISKVIK; MQMKKIIYSSL |
| | HLA-B3501 | LPEDEKMQM; YPEATASKL; EFNSLKPIY; TASKLETTL; IPIIIQSRL; KALNKTIDAY; NPIKDELQIL; EKMQMKKIIY; YPEATASKLET; EPLREKYPEAT; YKEFNSLKPIY; EATASKLETTL |
| | HLA-B4403 | EELLMRIES; SELKIKENF; KEIEELLMR; KEFNSLKPI; KEFNSLKPIY; KEIEELLMRI; QEILEKLDKNL; EELLMRIESEL; SENIKTSAEQL; KENIKNFVNKF |
| | HLA-B5101 | IPIIIQSRL; NPIKDELQI; ; IPIIIQSRLDI |
| | HLA-B5701 | |
| NP_045573.1 \| hypothetical protein BBI42 [Borrelia burgdorferi B31]; Seq38 | HLA-A0101 | PVSDYNEEY; FMRIVRWLY; VSDYNEEYF; YSGEEGSPEY; SSDMGSDEIV; YSGEEGSPEYY; GSDEIVTEGIF |
| | HLA-A0201 | WLYSCIEEL; KLYASEHRL; YLPDNQEQA; ILVGVCIIA; ALALLGCYL; YASEHRLLV; LVGVCIIAL; LLVEIKKTL; DLIKLFIMV; TAYQQYLKV; YLPDNQEQAV; ILVGVCIIAL; KLYASEHRLL; LLVEIKKTLI; GIFSSLKLYA; IVTEGIFSSL; RIVRWLYSCI; SLKDPNYRGV; RLLVEIKKTL; LVGVCIIALA; RILVGVCIIA; KLYASEHRLLV; ILVGVCIIALA; RILVGVCIIAL; WLYSCIEELYS; YASEHRLLVEI; RLLVEIKKTLI; IIALALLGCYL |
| | HLA-A0301 | LIKLFIMVK; GIFSSLKLY; PTAYQQYLK; ELYSPDIKY; VTEGIFSSLK; KFMRIVRWLY; RPTAYQQYLK; DLIKLFIMVK; TAYQQYLKVK; SLKLYASEHR; FLDLGSEQSK; IIALALLGCY; EIKKTLISLK; |

| | | |
|---|---|---|
| | | WLYSCIEELY; MVKNEQNNNK; SDYNEEYFNK; IVTEGIFSSLK; KLYASEHRLLV; IMVKNEQNNNK; CIIALALLGCY; TAYQQYLKVKR |
| | HLA-A1101 | LIKLFIMVK; PTAYQQYLK; GIFSSLKLY; IALALLGCY; KVKRYDYNR; ISLKDPNYR; TEGIFSSLK; AYQQYLKVK; VTEGIFSSLK; TAYQQYLKVK; MVKNEQNNNK; DLIKLFIMVK; SDYNEEYFNK; RPTAYQQYLK; GSEQSKDLIK; IIALALLGCY; EIKKTLISLK; TLISLKDPNY; LISLKDPNYR; KFMRIVRWLY; IVTEGIFSSLK; ASEHRLLVEIK; VSDYNEEYFNK; TAYQQYLKVKR; TLISLKDPNYR; SSLKLYASEHR; CIIALALLGCY; KTLISLKDPNY; IMVKNEQNNNK; PTAYQQYLKVK; GSPEYYRNMPR; CIEELYSPDIK; KDLIKLFIMVK |
| | HLA-A2402 | LYASEHRLL; KFMRIVRWL; DYNRPVPIL; DYNEEYFNK; NYRGVVLPV; DYNEEYFNKF; RYDYNRPVPI; EYFNKFFLDL; LYASEHRLLV; RWLYSCIEEL; KFMRIVRWLY; DYNEEYFNKFF; RYDYNRPVPIL; RWLYSCIEELY; CYLPDNQEQAV; YYRNMPRPTAY |
| | HLA-A2902 | ELYSPDIKY; FMRIVRWLY; LYSCIEELY; PVSDYNEEY; YLKVKRYDY; IALALLGCY; KFMRIVRWLY; QYLKVKRYDY; TLISLKDPNY; YSGEEGSPEY; WLYSCIEELY; YRNMPRPTAY; IIALALLGCY; YQQYLKVKRY; DYNEEYFNKF; YYRNMPRPTAY; CIIALALLGCY; NMPRPTAYQQY; VLPVSDYNEEY; KYSGEEGSPEY; YSGEEGSPEYY; RWLYSCIEELY; DYNEEYFNKFF |
| | HLA-A6801 | EQNNNKFMR; PTAYQQYLK; KVKRYDYNR; NNKFMRIVR; LIKLFIMVK; DYNEEYFNK; ISLKDPNYR; YQQYLKVKR; EEGSPEYYR; LYSCIEELY; ELYSPDIKY; QTFFENSES; TEGIFSSLK; FMRIVRWLY; MVKNEQNNNK; EIKKTLISLK; VTEGIFSSLK; TAYQQYLKVK; SLKLYASEHR; DLIKLFIMVK; LISLKDPNYR; NNNKFMRIVR; SPEYYRNMPR; RPTAYQQYLK; WLYSCIEELY; SDYNEEYFNK; NEQNNNKFMR; EGIFSSLKLY; TAYQQYLKVKR; IVTEGIFSSLK; TLISLKDPNYR; SSLKLYASEHR; YLKVKRYDYNR; IMVKNEQNNNK; PTAYQQYLKVK; CIEELYSPDIK; QNNNKFMRIVR; CIIALALLGCY; GSPEYYRNMPR; NKFMRIVRWLY |
| | HLA-B0702 | DPNYRGVVL; RPTAYQQYL; YASEHRLLV; MPRPTAYQQ; SPEYYRNMP; IVTEGIFSSL; SPEYYRNMPR; MPRPTAYQQY; MPRPTAYQQYL; RPTAYQQYLKV; DPNYRGVVLPV; LPVSDYNEEYF; RILVGVCIIAL; YASEHRLLVEI |
| | HLA-B0801 | YLKVKRYDY; EIKKTLISL; ; MPRPTAYQQYL; FMRIVRWLYSC |
| | HLA-B1501 | QQYLKVKRY; FMRIVRWLY; YLKVKRYDY; RNMPRPTAY; QSKDLIKLF; NQEQAVQTF; GVVLPVSDY; IALALLGCY; ELYSPDIKY; KLYASEHRL; SGEEGSPEY; YQQYLKVKRY; YSGEEGSPEY; WLYSCIEELY; IIALALLGCY; ILVGVCIIAL; EQSKDLIKLF; NQEQAVQTFF; IVTEGIFSSL; TLISLKDPNY; KLYASEHRLL; RGVVLPVSDY; QQYLKVKRYDY; YSGEEGSPEYY; MVKNEQNNNKF; NMPRPTAYQQY; YYRNMPRPTAY; VLPVSDYNEEY; CIIALALLGCY |
| | HLA-B2705 | HRLLVEIKK; KRYDYNRPV; MRILVGVCI; YQQYLKVKR; QQYLKVKRY; YRNMPRPTAY; KRYDYNRPVP; KRYDYNRPVPI; HRLLVEIKKTL; YRGVVLPVSDY; VRWLYSCIEEL; QQYLKVKRYDY; MRIVRWLYSCI |
| | HLA-B3501 | DPNYRGVVL; LPDNQEQAV; NQEQAVQTF; IALALLGCY; LPVSDYNEE; PVSDYNEEY; SGEEGSPEY; YNEEYFNKF; LVGVCIIAL; LPVSDYNEEY; MPRPTAYQQY; YSGEEGSPEY; LPDNQEQAVQ; WLYSCIEELY; YRNMPRPTAY; FFENSESSDM; IIALALLGCY; LPVSDYNEEYF; MPRPTAYQQYL; LPDNQEQAVQT; YSGEEGSPEYY; MVKNEQNNNKF; YYRNMPRPTAY; YASEHRLLVEI; LVGVCIIALAL; DPNYRGVVLPV; TFFENSESSDM |
| | HLA-B4403 | NEEYFNKFF; EEYFNKFFL; VEIKKTLIS; DEIVTEGIF; SEHRLLVEI; GEEGSPEYY; EELYSPDIKY; DEIVTEGIFS; VEIKKTLISL; SEQSKDLIKLF; EELYSPDIKYS; EEYFNKFFLDL; EEGSPEYYRNM; |

| | | |
|---|---|---|
| | | TEGIFSSLKLY |
| | HLA-B5101 | LPDNQEQAV; LPVSDYNEEY; LPVSDYNEEYF; MPRPTAYQQYL |
| | HLA-B5701 | |
| NP_051318.1 \| revA protein[Borrelia burgdorferi B31]; Seq39 | HLA-A0101 | |
| | HLA-A0201 | SLMEDVLAL; FVMACKAYV; KLVGVTVPL; LMEDVLALV; KLAAKLAAL; KIWSEAKLV; LFFASMLFV; MLFVMACKA; FQDKLAAKL; LVGVTVPLL; FASMLFVMA; FFASMLFVM; SLMEDVLALV; KLFFASMLFV; KLVGVTVPLL; KMSKNAVEQI; SMLFVMACKA; NIFKLFFASM; NLKDIGNAEL; NLQNSFQDKL; FFASMLFVMA; MLFVMACKAYV; KIWSEAKLVGV; KLFFASMLFVM; KLAAKLAALKA; FKLFFASMLFV; NIFKLFFASML; FQDKLAAKLAA |
| | HLA-A0301 | SMLFVMACK; KLAALKAAK; LAAKLAALK; LVNDSSGGK; KLFFASMLF; NLQNSFQDK; KLAAKLAALK; ASMLFVMACK; ALVNDSSGGK; SSGGKFKDYK; KLFFASMLFV; KLLNLQNSFQ; AKLAALKAAK; TSGNGDKMSK; MLFVMACKAY; KINELKENLK; GSNTSGNGDK; NSFQDKLAAK; LLNLQNSFQDK; LVNDSSGGKFK; VMACKAYVEEK; FASMLFVMACK; KLFFASMLFVM; ISKRKIWSEAK; KLAAKLAALKA; SMLFVMACKAY; MSKNAVEQIDK; NTSGNGDKMSK |
| | HLA-A1101 | SMLFVMACK; LVNDSSGGK; KLAALKAAK; LAAKLAALK; NTIENITDK; SFQDKLAAK; SGNGDKMSK; NLQNSFQDK; ASMLFVMACK; KLAAKLAALK; KINELKENLK; MACKAYVEEK; SSGGKFKDYK; NSFQDKLAAK; TSGNGDKMSK; GSNTSGNGDK; ALVNDSSGGK; ITDKDQDISK; AVEQIDKVIK; LVNDSSGGKFK; FASMLFVMACK; VMACKAYVEEK; NTSGNGDKMSK; MACKAYVEEKK; AAKLAALKAAK; MSKNAVEQIDK; LLNLQNSFQDK; ISKRKIWSEAK; LALVNDSSGGK; SMLFVMACKAY; NITDKDQDISK; DSSGGKFKDYK |
| | HLA-A2402 | FFASMLFVM; DYKDKINEL; KLFFASMLF; IFKLFFASML; IFKLFFASMLF |
| | HLA-A2902 | FFASMLFVM; KLLNLQNSF; MLFVMACKAY; SMLFVMACKAY |
| | HLA-A6801 | NTIENITDK; LAAKLAALK; LVNDSSGGK; SMLFVMACK; CKAYVEEKK; KLAALKAAK; NLQNSFQDK; MACKAYVEEK; NSFQDKLAAK; ASMLFVMACK; MLFVMACKAY; DYKDKINELK; TSGNGDKMSK; SSGGKFKDYK; KLAAKLAALK; KINELKENLK; DIGNAELKEK; ITDKDQDISK; MACKAYVEEKK; NTSGNGDKMSK; FASMLFVMACK; DSSGGKFKDYK; LVNDSSGGKFK; MSKNAVEQIDK; NLKDIGNAELK; NAVEQIDKVIK; ITDKDQDISKR; VMACKAYVEEK; NITDKDQDISK; LALVNDSSGGK; LLNLQNSFQDK; ISKRKIWSEAK |
| | HLA-B0702 | KLAAKLAAL; VPLLGSNTSG; |
| | HLA-B0801 | IFKLFFASM; KLAAKLAAL; IFKLFFASML; EAKLVGVTVPL; QDKLAAKLAAL |
| | HLA-B1501 | KLLNLQNSF; KLFFASMLF; SLMEDVLAL; EQIDKVIKF; KLVGVTVPL; KLAAKLAAL; LFVMACKAY; MLFVMACKAY; LVNDSSGGKF; NLKDIGNAEL; KLVGVTVPLL; EQIDKVIKFL; SMLFVMACKAY; ALVNDSSGGKF; KLFFASMLFVM; IFKLFFASMLF; KEKLLNLQNSF |
| | HLA-B2705 | KRKIWSEAK; RKIWSEAKL; MRNKNIFKL; KRKIWSEAKL; MRNKNIFKLF; MRNKNIFKLFF |
| | HLA-B3501 | FFASMLFVM; LFVMACKAY; VPLLGSNTS; FASMLFVMA; MLFVMACKAY; FASMLFVMAC; LVNDSSGGKF; LFFASMLFVM; FKLFFASMLF; NAVEQIDKVI; NIFKLFFASM; DSLMEDVLAL; EAKLVGVTVPL; SMLFVMACKAY |
| | HLA-B4403 | AELKEKLLN; KEKLLNLQN; KENLKDIGN; KEIDSLMED; EEKKEIDSL; AELKEKLLNL; EEKKEIDSLM; KENLKDIGNA; SEAKLVGVTV; KEIDSLMEDVL; KEKLLNLQNSF; AELKEKLLNLQ |
| | HLA-B5101 | VPLLGSNTS; LAALKAAKNTI |
| | HLA-B5701 | |
| NP_045737.1 | HLA-A0101 | YTEERRMLY; DTDDLIDEY; STVHFDSHEY; NSTVHFDSHEY; |

| | BBA64 antigen, P35 [Borrelia burgdorferi B31]; Seq40 | | QTDVSKLNDTL; LKDTDDLIDEY; QLNKPNLETLY |
|---|---|---|---|
| | | HLA-A0201 | ILISCSLEV; FLLHVLTVL; ILDPEVAKL; TLINRGFSI; TLYNDFEKL; LIAIFLLHV; KILDRSENI; VLILISCSL; LLHVLTVLI; HVLTVLILI; AMEEISAKI; NLLVAINNL; NIHDIILDL; NLGQILSKL; KIFNLGQIL; IILDLVNTT; QLAMEEISA; TTTNILAPI; AQFANIHDI; KLIAIFLLHV; FLLHVLTVLI; LILISCSLEV; NIHDIILDLV; AIFLLHVLTV; KIFFNANSTV; KILDPEVAKL; ILDPEVAKLI; LLHVLTVLIL; LAMEEISAKI; KILDRSENII; GMRAEIFSKI; KLQQLNKPNL; LIAIFLLHVL; QLNKPNLETL; IILDLVNTTT; TVLILISCSL; EIFSKIFFNA; KLTSLKEKWL; FSIQLAMEEI; FLLHVLTVLIL; KLIAIFLLHVL; VLILISCSLEV; TLINRGFSIQL; LLHVLTVLILI; ILDLVNTTTNI; LIDEYNTNPDL; ILDRSENIIQI; QLAMEEISAKI; KLSQDSNYRGL; IAIFLLHVLTV; KILDPEVAKLI; AQFANIHDIIL; AIFLLHVLTVL; LTVLILISCSL; ILNVKDKLQQL; NILKNNKLIAI; FANIHDIILDL |
| | | HLA-A0301 | KLIAIFLLH; GMRAEIFSK; KLNDTLRSK; LLVAINNLK; ISAKILNVK; KILDPEVAK; KLIQKILDR; LISCSLEVK; KLSQDSNYR; TSLKEKWLK; TVHFDSHEY; KLIAIFLLH; GMRAEIFSK; KLNDTLRSK; LLVAINNLK; ISAKILNVK; KILDPEVAK; KLIQKILDR; LISCSLEVK; KLSQDSNYR; TSLKEKWLK; TVHFDSHEY; KIFNLGQILSK; KLTSLKEKWLK; QLNKPNLETLY; KIFFNANSTVH; LILISCSLEVK; ISAKILNVKDK; ILSKLSQDSNY; IQLAMEEISAK; NVKDKLQQLNK |
| | | HLA-A1101 | TSLNFNEGK; TSLKEKWLK; KILDPEVAK; GMRAEIFSK; NANANNAPK; ISAKILNVK; KLNDTLRSK; LAMEEISAK; ETLYNDFEK; LLVAINNLK; LISCSLEVK; KLIAIFLLH; TVHFDSHEY; DSNYRGLVK; YTEERRMLY; KLSQDSNYR; LVKETLINR; KLIQKILDR; EVKDSNESK; AINNLKNPTK; YTSLNFNEGK; ILISCSLEVK; LTSLKEKWLK; KANSKASKQK; NANANNAPKK; STVHFDSHEY; NLLVAINNLK; QLAMEEISAK; EISAKILNVK; IFNLGQILSK; AAGKNKANSK; SAKILNVKDK; FGMRAEIFSK; IQISEMDSSR; EVKDSNESKK; KIFNLGQILSK; VAINNLKNPTK; KLTSLKEKWLK; IQLAMEEISAK; LILISCSLEVK; SQDSNYRGLVK; NVKDKLQQLNK; ISAKILNVKDK; VSKLNDTLRSK; SLEVKDSNESK; IIQISEMDSSR; QFGMRAEIFSK; NLETLYNDFEK; KIFFNANSTVH |
| | | HLA-A2402 | RMLYTSLNF; QFANIHDII; IFLLHVLTV; EYNTNPDLQ; KWLKDTDDL; QFGMRAEIF; KWLKDTDDLI; IFLLHVLTVL; FFNANSTVHF; QFANIHDIIL; NYRGLVKETL; IFLLHVLTVLI; IFFNANSTVHF; NYRGLVKETLI; LYNDFEKLTSL |
| | | HLA-A2902 | TVHFDSHEY; NKPNLETLY; YTEERRMLY; RMLYTSLNF; STVHFDSHEY; FFNANSTVHF; EYTEERRMLY; QLNKPNLETLY; IFFNANSTVHF; ILSKLSQDSNY |
| | | HLA-A6801 | ETLYNDFEK; DSHEYTEER; EVKDSNESK; QISEMDSSR; LLVAINNLK; TSLNFNEGK; DTLRSKNSR; NANANNAPK; TVHFDSHEY; ISAKILNVK; EPNDQFGMR; LAMEEISAK; TSLKEKWLK; YTEERRMLY; VSKLNDTLR; DFEKLTSLK; LISCSLEVK; LVKETLINR; EIFSKIFFN; DSNESKKHK; DSNYRGLVK; ESKKHKKEK; TTNILAPIQ; KLSQDSNYR; NPTKPAAGK; STVHFDSHE; FSIQLAMEE; DTDDLIDEY; YTSLNFNEGK; DVSKLNDTLR; DSHEYTEERR; EVKDSNESKK; NANANNAPKK; EISAKILNVK; STVHFDSHEY; LTSLKEKWLK; ESKKHKKEKR; NLLVAINNLK; DSNESKKHKK; TTTNILAPIQ; ILISCSLEVK; QLAMEEISAK; IQISEMDSSR; FGMRAEIFSK; NDFEKLTSLK; LETLYNDFEK; FDSHEYTEER; EYTEERRMLY; MLYTSLNFNE; IFNLGQILSK; AINNLKNPTK; SAKILNVKDK; LSKLSQDSNYR; IIQISEMDSSR; LYTSLNFNEGK; ENLLVAINNLK; NLETLYNDFEK; ISAKILNVKDK; NVKDKLQQLNK; NPNANANNAPK; HFDSHEYTEER; VAINNLKNPTK; NSTVHFDSHEY; EEISAKILNVK; TDVSKLNDTLR; KIFNLGQILSK; LILISCSLEVK; NTTTNILAPIQ; VAKLIQKILDR; |

| | | |
|---|---|---|
| | | KLTSLKEKWLK; ESKKHKKEKRK; ETLINRGFSIQ |
| | HLA-B0702 | KPAAGKNKA; LVNTTTNIL; KPAAGKNKAN; APKKILDPEV; KPNLETLYNDF; APKKILDPEVA; NPDLQTDVSKL; KPAAGKNKANS |
| | HLA-B0801 | VAKLIQKIL; ILKNNKLIAI; LLHVLTVLIL; |
| | HLA-B1501 | RMLYTSLNF; TVHFDSHEY; RSKNSRAQF; AQFANIHDI; YTEERRMLY; FNANSTVHF; LNFNEGKIF; VLILISCSL; KLIAIFLLH; KIFNLGQIL; TLINRGFSI; STVHFDSHEY; FFNANSTVHF; DQFGMRAEIF; LSKLSQDSNY; SLNFNEGKIF; AQFANIHDII; SSRGEPNDQF; GMRAEIFSKIF; AQFANIHDIIL; LVKETLINRGF; QLNKPNLETLY; ILKNNKLIAIF; ILSKLSQDSNY; KLIAIFLLHVL; IFFNANSTVHF; TLRSKNSRAQF; IQKILDRSENI; TLINRGFSIQL; QQLNKPNLETL; INRGFSIQLAM; TSLNFNEGKIF |
| | HLA-B2705 | KRKGKVENL; RRMLYTSLN; YRGLVKETL; ERRMLYTSL; MRAEIFSKI; RMLYTSLNF; RRMLYTSLNF; MRAEIFSKIF; LRSKNSRAQF; KRKGKVENLL; MRAEIFSKIFF; KRKGKVENLLV; ERRMLYTSLNF; RRMLYTSLNFN |
| | HLA-B3501 | TVHFDSHEY; FANIHDIIL; IAIFLLHVL; NPDLQTDVS; LVNTTTNIL; DTDDLIDEY; FFNANSTVH; RAEIFSKIF; EPNDQFGMR; FNANSTVHF; NPNANANNA; FFNANSTVHF; STVHFDSHEY; EPNDQFGMRA; MRAEIFSKIF; NPNANANNAP; RAEIFSKIFF; DLVNTTTNIL; KPNLETLYNDF; FANIHDIILDL; NSTVHFDSHEY; EPNDQFGMRAE; NPDLQTDVSKL; LAMEEISAKIL; IFFNANSTVHF; LKDTDDLIDEY; NPNANANNAPK; NANSTVHFDSH; MRAEIFSKIFF; TSLNFNEGKIF |
| | HLA-B4403 | AEIFSKIFF; EEISAKILN; KETLINRGF; SEMDSSRGE; GEPNDQFGM; EEISAKILNV; SENIIQISEM; AEIFSKIFFN; AEIFSKIFFNA; SEMDSSRGEPN; EEISAKILNVK; KETLINRGFSI; HEYTEERRMLY; SENIIQISEMD |
| | HLA-B5101 | IAIFLLHVL; LAMEEISAKI; LHVLTVLILI; IAIFLLHVLTV |
| | HLA-B5701 | KLTSLKEKW; STVHFDSHEY; NSTVHFDSHEY |
| AAT93822.1\| BBA64 antigen, P35 [Borrelia garinii PBi]; Seq41 | HLA-A0101 | FTDIHNIIL; YVNERRILY; DVDDIIKDY; RTDISKLNDY; STVTFDDNEY; FTDIHNIILN; FTDIHNIILNL; HLNKPNLKTLY; GSTVTFDDNEY; RTDISKLNDYI |
| | HLA-A0201 | FLLHILTGL; ILLSCSLEV; VTFDDNEYV; ILINRGFSI; NLINTTTNI; HILTGLILL; NLLVAINTL; TLYHDFNKL; KLNDYIISK; AQFTDIHNI; LIAIFLLHI; GLILLSCSL; QLAIEEISL; FTDIHNIIL; YIISKNSKA; NLGKILSKL; KILERSEDI; SIQLAIEEI; TTTNILAPI; LLHILTGLI; TVDNLLVAI; ALKQIDPEA; ILTGLILLS; ILNLINTTT; KLIAIFLLHI; FLLHILTGLI; LILLSCSLEV; TLYHDFNKLI; KIFFNAGSTV; KILERSEDIV; NLINTTTNIL; LIAIFLLHIL; YVNERRILYT; KLIPLKEKWL; IQLAIEEISL; KTLYHDFNKL; TVTFDDNEYV; FSIQLAIEEI; LLHILTGLIL; SQDSNYRSLV; LINTTTNILA; EIFSKIFFNA; AQFTDIHNII; FLLHILTGLIL; KLIAIFLLHIL; GLILLSCSLEV; ILINRGFSIQL; ILNLINTTTNI; FTDIHNIILNL; STVTFDDNEYV; NLINTTTNILA; AIFLLHILTGL; LLHILTGLILL; KLSQDSNYRSL; AQFTDIHNIIL; IIKDYNANPEL; KLNDYIISKNS; ILNLGKILSKL; KTLYHDFNKLI; SIQLAIEEISL; ILERSEDIVQI |
| | HLA-A0301 | KLNDYIISK; KSNSAAALK; KLIAIFLLH; LLVAINTLK; KTLYHDFNK; KLSKNAKNK; ISLRTLNVK; YVNERRILY; SSKMKKLSK; EMKAEIFSK; KLSQDSNYR; TSLNFNENK; DSNYRSLVK; LNLGKILSK; ILNLGKILSK; NLLVAINTLK; AINTLKNPPK; LIPLKEKWLK; KQPNNANALK; KMKKLSKNAK; QDSNYRSLVK; KLSKNAKNKK; NSSKMKKLSK; SLRTLNVKDK; KSENNSSKMK; SENNSSKMKK; EISLRTLNVK; SKLNDYIISK; IQHLNKPNLK; YTSLNFNENK; NKSNSAAALK; LKNPPKTAGK; TSKSENNSSK; QKKAKTKTSK; HDFNKLIPLK; SLVKEILINR; ALKQIDPEAK; FEMKAEIFSK; KILNLGKILSK; |

| | | |
|---|---|---|
| | | HLNKPNLKTLY; KLIPLKEKWLK; KIQHLNKPNLK; KTSKSENNSSK; TLKNPPKTAGK; KSENNSSKMKK; KQKKAKTKTSK; NLKTLYHDFNK; ILSKLSQDSNY; ISKLNDYIISK; RSLVKEILINR; ISLRTLNVKDK; IVQISEIDANK; NVKDKIQHLNK |
| | HLA-A1101 | KTLYHDFNK; KSNSAAALK; KLNDYIISK; TSLNFNENK; SSKMKKLSK; ISLRTLNVK; LLVAINTLK; KLIAIFLLH; QISEIDANK; DSNYRSLVK; YVNERRILY; EMKAEIFSK; KLSKNAKNK; LVKEILINR; KLSQDSNYR; LAIEEISLR; AINTLKNPPK; YTSLNFNENK; ILNLGKILSK; TSKSENNSSK; VQISEIDANK; NSSKMKKLSK; KSENNSSKMK; IQHLNKPNLK; NLLVAINTLK; KQPNNANALK; EISLRTLNVK; KLSKNAKNKK; KMKKLSKNAK; SLVKEILINR; ALKQIDPEAK; STVTFDDNEY; SENNSSKMKK; SKLNDYIISK; EVNQDDNQEK; LIPLKEKWLK; SLRTLNVKDK; HDFNKLIPLK; KTSKSENNSSK; KIQHLNKPNLK; KILNLGKILSK; VAINTLKNPPK; IVQISEIDANK; KSENNSSKMKK; KLIPLKEKWLK; SQDSNYRSLVK; ISKLNDYIISK; RSLVKEILINR; NVKDKIQHLNK; ISLRTLNVKDK; TLKNPPKTAGK; NLKTLYHDFNK; IQLAIEEISLR; KQKKAKTKTSK; NALKQIDPEAK; LYTSLNFNENK |
| | HLA-A2402 | LYHDFNKLI; KWLKDVDDI; NYRSLVKEI; QFTDIHNII; RILYTSLNF; EYVNERRIL; KWLKDVDDII; FFNAGSTVTF; IFLLHILTGL; NYRSLVKEIL; IFLLHILTGLI; IFFNAGSTVTF; NYRSLVKEILI; LYHDFNKLIPL |
| | HLA-A2902 | YVNERRILY; TVTFDDNEY; EYVNERRILY; FFNAGSTVTF; STVTFDDNEY; HLNKPNLKTLY; ILSKLSQDSNY; YIISKNSKAQF; IFFNAGSTVTF |
| | HLA-A6801 | LAIEEISLR; ELIQKILER; TSLNFNENK; LLVAINTLK; QISEIDANK; EMKAEIFSK; LVKEILINR; DYNANPELR; TVTFDDNEY; DSNYRSLVK; KSNSAAALK; DFNKLIPLK; ENNSSKMKK; KTLYHDFNK; QPNNANALK; EIFSKIFFN; DNEYVNERR; DYIISKNSK; YVNERRILY; DDNEYVNER; LSKNAKNKK; TTNILAPIQ; KLSQDSNYR; KLNDYIISK; ENKILNLGK; YTSLNFNENK; EVNQDDNQEK; EISLRTLNVK; QLAIEEISLR; TSKSENNSSK; STVTFDDNEY; SLVKEILINR; NLLVAINTLK; NSSKMKKLSK; TTTNILAPIQ; DDNEYVNERR; HDFNKLIPLK; NKSNSAAALK; FEMKAEIFSK; LIPLKEKWLK; ILNLGKILSK; NDYIISKNSK; LKTLYHDFNK; LSKLSQDSNYR; LYTSLNFNENK; IVQISEIDANK; KTSKSENNSSK; NVKDKIQHLNK; NLKTLYHDFNK; TFDDNEYVNER; ISKLNDYIISK; DNLLVAINTLK; IQLAIEEISLR; EEISLRTLNVK; VAINTLKNPPK; NALKQIDPEAK; DFNKLIPLKEK; NTTTNILAPIQ; TLKNPPKTAGK |
| | HLA-B0702 | KPTVDNLLV; KPNLKTLYH; KPTVDNLLVA; KPTVDNLLVAI; NPELRTDISKL; KPNLKTLYHDF; QPNNANALKQI |
| | HLA-B0801 | |
| | HLA-B1501 | YVNERRILY; FNAGSTVTF; KQPNNANAL; RILYTSLNF; ISKNSKAQF; ILINRGFSI; TVTFDDNEY; NLKTLYHDF; AQFTDIHNI; KLIAIFLLH; GLILLSCSL; IQHLNKPNL; FFNAGSTVTF; KQIDPEAKEL; IQLAIEEISL; LSKLSQDSNY; DQFEMKAEIF; AQFTDIHNII; STVTFDDNEY; IISKNSKAQF; EMKAEIFSKIF; IFFNAGSTVTF; LVKEILINRGF; AQFTDIHNIIL; ALKQPNNANAL; ILSKLSQDSNY; HLNKPNLKTLY; YIISKNSKAQF; ILINRGFSIQL; KLIAIFLLHIL; KLSQDSNYRSL; KQIDPEAKELI |
| | HLA-B2705 | RRILYTSLN; KKAKTKTSK; ERRILYTSL; YRSLVKEIL; RRILYTSLNF; KQKKAKTKTSK; ERRILYTSLNF; RRILYTSLNFN |
| | HLA-B3501 | TVTFDDNEY; YVNERRILY; FNAGSTVTF; IAIFLLHIL; FFNAGSTVT; LINTTTNIL; NKSNSAAAL; NLLVAINTL; EPDDQFEMK; KAEIFSKIF; FFNAGSTVTF; STVTFDDNEY; MKAEIFSKIF; MKNNKLIAIF; EPDDQFEMKA; DQFEMKAEIF; IFFNAGSTVTF; KPNLKTLYHDF; |

| | | |
|---|---|---|
| | | DANKGEPDDQF; LAIEEISLRTL; EPDDQFEMKAE; MKAEIFSKIFF; NPELRTDISKL; YIISKNSKAQF |
| | HLA-B4403 | KEILINRGF; AEIFSKIFF; SEIDANKGE; FEMKAEIFS; GEPDDQFEM; EEISLRTLN; EEISLRTLNV; KEILINRGFS; AEIFSKIFFN; SEIDANKGEP; SEDIVQISEI; KEILINRGFSI; AEIFSKIFFNA; SEIDANKGEPD; NEYVNERRILY |
| | HLA-B5101 | IAIFLLHIL; ; IPLKEKWLKDV; KPTVDNLLVAI |
| | HLA-B5701 | KLIPLKEKW; STVTFDDNEY; GSTVTFDDNEY |
| AAL84596.1\| BBK32 [Borrelia burgdorferi]; Seq42 | HLA-A0101 | FSTKLTQMY; ISCDLFIRY; IIDKNGVWY; YTDEIEEEDY; NTINKIYDTY; FISCDLFIRY; YLDEDDEDDEY; DSAINTINKIY |
| | HLA-A0201 | LLNYIQVSV; GLFDKGNSI; YLEGVKYNV; KTAANFVYI; KLENIEAEI; NLYEAYKAI; AIVTSILLM; KTLLNYIQV; LMRDSLKEV; YLALGLLFG; TLLNYIQVS; ILETSEESI; ALGLLFGFI; SLSGESGEL; MTQGSLNSL; TLLNYIQVSV; LLMRDSLKEV; LLFGFISCDL; GLFDKGNSIL; RLSNRYQSYL; NLYEAYKAIV; KLTQMYSTRL; QMYSTRLDNL; KIYDTYTLFS; GMTQGSLNSL; SILETSEESI; YLALGLLFGF; TLFSTKLTQM; LALGLLFGFI; YLALGLLFGFI; KIYDTYTLFST; SLQDIAGSNSI; KTLLNYIQVSV; ILLMRDSLKEV; GLLFGFISCDL; YTDEIEEEDYA; ILEEESLKTEL; TINKIYDTYTL; QMYSTRLDNLA; LLNYIQVSVKT; TQMYSTRLDNL; NLYEAYKAIVT; YINDTHAKRKL |
| | HLA-A0301 | ILLMRDSLK; RLSNRYQSY; SIKKPMNKK; SLKRDSANK; RLDNLAKAK; DTYTLFSTK; QSNLYEAYK; IVTSILLMR; KLTQMYSTR; KIKEQSNLY; VILEEESLK; ESIKKPMNK; STRLDNLAK; ISCDLFIRY; ESLKTELLK; KGKSKVSRK; IARKKGKSK; KNFKTLLNY; QSYLEGVKY; LNYIQVSVK; LLNYIQVSVK; SLSGESGELK; SILLMRDSLK; KIARKKGKSK; KPMNKKGKGK; AIVTSILLMR; RYQSYLEGVK; GIIDKNGVWY; FVYINDTHAK; SANKSNFLQK; ITIDSDLRPK; RKEPYIHSLK; LLKEQSETRK; CDLFIRYEMK; YDTYTLFSTK; EQSNLYEAYK; YINDTHAKRK; KLENIEAEIK; HSLKRDSANK; SVKTAANFVY; KIQKQQDEYK; NVILEEESLK; KIYDTYTLFS; YSTRLDNLAK; FISCDLFIRY; ILETSEESIK; YTLFSTKLTQ; TLLNYIQVSVK; TLFSTKLTQMY; RLDNLAKAKAK; TSILLMRDSLK; SLKEVQGIIDK; ILETSEESIKK; ELLKEQSETRK; SIKKPMNKKGK; SILETSEESIK; FVYINDTHAKR; KAKEEAAKFTK; STRLDNLAKAK; DSANKSNFLQK; NVDSAINTINK |
| | HLA-A1101 | DTYTLFSTK; QSNLYEAYK; STRLDNLAK; ILLMRDSLK; SIKKPMNKK; ISCDLFIRY; VILEEESLK; IVTSILLMR; TIDSDLRPK; ESIKKPMNK; ESLKTELLK; SLKRDSANK; ETSEESIKK; DSAINTINK; LSGESGELK; VYINDTHAK; ANKSNFLQK; RLDNLAKAK; YQSYLEGVK; AEIKTLIAK; FISCDLFIR; YINDTHAKR; KLTQMYSTR; SANKSNFLQ; SANKSNFLQK; SILLMRDSLK; ITIDSDLRPK; SLSGESGELK; FVYINDTHAK; KIQKQQDEYK; AIVTSILLMR; NVILEEESLK; HSLKRDSANK; YINDTHAKRK; YSTRLDNLAK; LLNYIQVSVK; KIARKKGKSK; SVKTAANFVY; ESIKKPMNKK; KAKAKEEAAK; EQSNLYEAYK; RYQSYLEGVK; KIYDTYTLFS; KLENIEAEIK; FISCDLFIRY; TSILLMRDSLK; SILETSEESIK; TLLNYIQVSVK; NVDSAINTINK; KAIVTSILLMR; STRLDNLAKAK; STKLTQMYSTR; NSLSGESGELK; KAKEEAAKFTK; AAKFTKEDLEK; TLFSTKLTQMY; SIKKPMNKKGK; DSANKSNFLQK; SLKEVQGIIDK; MYSTRLDNLAK; FTKEDLEKNFK; KEQSNLYEAYK; ILETSEESIKK; QSETRKEKIQK; IYDTYTLFSTK; RLDNLAKAKAK; FVYINDTHAKR; VYINDTHAKRK; VSVKTAANFVY |
| | HLA-A2402 | KYLALGLLF; AYKAIVTSI; YYEDDYEEI; TYTLFSTKL; MYSTRLDNL; VYINDTHAK; GFISCDLFI; NFKTLLNYI; RYQSYLEGV; KIYDTYTLF; LFGFISCDLF; EYYEDDYEEI; AYKAIVTSIL; EYKGMTQGSL; IYDTYTLFST; SYLEGVKYNV; RYQSYLEGVK; VYINDTHAKR; |

| | | |
|---|---|---|
| | | KYLALGLLFGF; KYNVDSAINTI; AYKAIVTSILL; YYEDDYEEIRL; IYDTYTLFSTK; LFGFISCDLFI; VYINDTHAKRK; RYQSYLEGVKY; MYSTRLDNLAK |
| | HLA-A2902 | QSYLEGVKY; FSTKLTQMY; IAGSNSISY; KYLALGLLF; ISCDLFIRY; IIDKNGVWY; EQSNLYEAY; AINTINKIY; VKTAANFVY; KIYDTYTLF; TINKIYDTY; RLSNRYQSY; KNFKTLLNY; YQSYLEGVKY; FISCDLFIRY; SVKTAANFVY; GIIDKNGVWY; LFSTKLTQMY; YLALGLLFGF; SAINTINKIY; EIEEEDYARY; YTDEIEEEDY; DIAGSNSISY; NTINKIYDTY; TLFSTKLTQMY; GFISCDLFIRY; RYQSYLEGVKY; VSVKTAANFVY; YLDEDDEDDEY; EIEEEDYARYY; KYLALGLLFGF; SYTDEIEEEDY; DYEEIRLSNRY |
| | HLA-A6801 | DTYTLFSTK; IVTSILLMR; YINDTHAKR; ETSEESIKK; DITIDSDLR; QSNLYEAYK; FISCDLFIR; DSAINTINK; EPYIHSLKR; ESIKKPMNK; EIEEEDYAR; DLFIRYEMK; ETRKEKIQK; ESLKTELLK; KLTQMYSTR; FSTKLTQMY; TINKIYDTY; STRLDNLAK; ILLMRDSLK; LLKEQSETR; VYINDTHAK; LNYIQVSVK; SIKKPMNKK; SANKSNFLQ; EQSETRKEK; TAANFVYIN; EESPGLFDK; VILEEESLK; YQSYLEGVK; FVYINDTHAK; NVILEEESLK; ESIKKPMNKK; NTINKIYDTY; SANKSNFLQK; ITIDSDLRPK; EQSNLYEAYK; YSTRLDNLAK; LLNYIQVSVK; EIKTLIAKIK; AIVTSILLMR; SILLMRDSLK; YINDTHAKRK; ELLKEQSETR; DYEEIRLSNR; VYINDTHAKR; EAEIKTLIAK; HSLKRDSANK; DIAGSNSISY; IDITIDSDLR; GFISCDLFIR; YYEDDYEEIR; SVKTAANFVY; SLSGESGELK; YDTYTLFSTK; DSANKSNFLQ; FISCDLFIRY; SAINTINKIY; EESLKTELLK; DEIEEEDYAR; CDLFIRYEMK; FVYINDTHAKR; STKLTQMYSTR; EYYEDDYEEIR; TSILLMRDSLK; FTKEDLEKNFK; DSANKSNFLQK; NFVYINDTHAK; EIDITIDSDLR; FGFISCDLFIR; NVDSAINTINK; KAIVTSILLMR; NSLSGESGELK; TLLNYIQVSVK; MYSTRLDNLAK; TLFSTKLTQMY; DITIDSDLRPK; STRLDNLAKAK; ELLKEQSETRK; SILETSEESIK; DSAINTINKIY; DDYEEIRLSNR; EKIQKQQDEYK; MNKKGKGKIAR; EAAKFTKEDLE; QSETRKEKIQK; DTYTLFSTKLT |
| | HLA-B0702 | KVKSKYLAL; RPKSSLQDI; KPMNKKGKG; IARKKGKSKV; RPKSSLQDIA; KPMNKKGKGI; SPGLFDKGNSI; RPKSSLQDIAG; KVKSKYLALGL |
| | HLA-B0801 | KVKSKYLAL; MKKVKSKYL; HAKRKLENI; YIQVSVKTAA; MKKVKSKYLAL |
| | HLA-B1501 | RLSNRYQSY; EQSNLYEAY; KIKEQSNLY; KQQDEYKGM; KIYDTYTLF; QSYLEGVKY; FSTKLTQMY; KAKEEAAKF; IAGSNSISY; AINTINKIY; KVKSKYLAL; VSVKTAANF; LMRDSLKEV; GLFDKGNSI; EMKEESPGL; LQKNVILEE; ISCDLFIRY; KNFKTLLNY; YQSYLEGVKY; YLALGLLFGF; SVKTAANFVY; KSKVSRKEPY; EMKEESPGLF; QVSVKTAANF; SAINTINKIY; GMTQGSLNSL; GIIDKNGVWY; FTKEDLEKNF; GLFDKGNSIL; RLSNRYQSYL; DIAGSNSISY; LLFGFISCDL; IQVSVKTAANF; LLFGFISCDLF; TLFSTKLTQMY; KSKYLALGLLF; IQKQQDEYKGM; VSVKTAANFVY; EIRLSNRYQSY; QDIAGSNSISY; IAKIKEQSNLY; KAKAKEEAAKF; TQMYSTRLDNL; YLDEDDEDDEY |
| | HLA-B2705 | IRLSNRYQSY; TRLDNLAKAK; KRDSANKSNF; RKKGKSKVSR; NRYQSYLEGV; NRYQSYLEGVK; IRLSNRYQSYL; RKKGKSKVSRK; ARKKGKSKVSR; SRKEPYIHSLK; KRKLENIEAEI |
| | HLA-B3501 | IAGSNSISY; EQSNLYEAY; LALGLLFGF; FSTKLTQMY; TINKIYDTY; DEDDEDDEY; ISCDLFIRY; EDDEDDEYY; NFVYINDTH; SAINTINKIY; FISCDLFIRY; EIEEEDYARY; SVKTAANFVY; DIAGSNSISY; LFSTKLTQMY; YQSYLEGVKY; YLALGLLFGF; YTDEIEEEDY; LDEDDEDDEY; NTINKIYDTY; KAIVTSILLM; QVSVKTAANF; DEDDEDDEYY; YLDEDDEDDEY; EIEEEDYARYY; ETSEESIKKPM; |

EP 2 254 592 B1

| | | |
|---|---|---|
| | | IAKIKEQSNLY; EAYKAIVTSIL; VSVKTAANFVY; IKEQSNLYEAY; TLFSTKLTQMY; DSAINTINKIY; YTLFSTKLTQM; ISCDLFIRYEM; LDEDDEDDEYY; YKAIVTSILLM; QDIAGSNSISY |
| | HLA-B4403 | EEEDYARYY; EEIRLSNRY; NEIDITIDS; GELKETIES; EEDYARYYL; DEDDEDDEY; EESLKTELL; AEIKTLIAK; DEIEEEDYA; SEESIKKPM; EEIRLSNRYQ; EEEEDYARYYL; AEIKTLIAKI; DEDDEDDEYY; YEMKEESPGL; NEIDITIDSD; EEEESLKTELL; EEIRLSNRYQS; NEIDITIDSDL; YEMKEESPGLF; DEIEEEDYARY; EEEDYARYYLD; AEIKTLIAKIK; KEKIQKQQDEY; DEDDEYYEDDY |
| | HLA-B5101 | LALGLLFGFI; SPGLFDKGNSI |
| | HLA-B5701 | ISCDLFIRY; ; VSVKTAANFVY |
| AAL84590.1\| BBK32 [Borrelia afzelii]; Seq43 | HLA-A0101 | LSTKQTQMY; YSLEEDYYY; VIDKNGVWY; ISGSNSISY; YTDEIEEEDY; LLSTKQTQMY; SSAIKTIVKIY; ATNFVYAREIY; VSARTATNFVY |
| | HLA-A0201 | LLMKDSLKI; KIYDNYTLL; MLQGSLSFL; KLDAIETEI; FTIDSDLRL; LLNYIQVSA; YLEGVKYNV; FLSGESGEL; KTLLNYIQV; GLCDEESSI; RTATNFVYA; TLLNYIQVS; RLKSDLQAI; LLSTKQTQM; AIVRSILLM; ALGLLFGFI; RMLQGSLSFL; TLLNYIQVSA; LLFGFISCDL; LLMKDSLKII; RLSNRYESYL; QMYSTRLDNL; ILLMKDSLKI; SILETGDKSV; KIYDNYTLLS; MLQGSLSFLS; GLCDEESSIL; LALGLLFGFI; KIYDNYTLLST; LMKDSLKIIEI; GLLFGFISCDL; QVSARTATNFV; ILLMKDSLKII; RMLQGSLSFLS; QMYSTRLDNLA; LLNYIQVSART; TQMYSTRLDNL; TIVKIYDNYTL; CLALGLLFGFI; SLGLCDEESSI; KTLLNYIQVSA |
| | HLA-A0301 | KSVKKSLNK; IVRSILLMK; ILLMKDSLK; SSAIKTIVK; KIIEIVIDK; RLDNLAKAK; KVEGNAVKK; RLSNRYESY; SVKKSLNKK; SLNKKGKDK; ISGSNSISY; TIDSDLRLK; STRLDNLAK; KYNVSSAIK; VISEEEILK; KIKGQSDLY; AIVRSILLMK; FVYAREIYSK; SILLMKDSLK; FLSGESGELK; VSSAIKTIVK; KSLNKKGKDK; VKYNVSSAIK; AISGSNSISY; SVNNSNLSQK; GQSDLYEAYK; LLRERPETRK; IVIDKNGVWY; KLDAIETEIK; LLNYIQVSAR; KSVKKSLNKK; LLSTKQTQMY; YDNYTLLSTK; RTATNFVYAR; FTIDSDLRLK; ILETGDKSVK; NVISEEEILK; KTIVKIYDNY; EIKNLILKIK; YSTRLDNLAK; ILKTKLLRER; KKFTKEELEK; RYESYLEGVK; ETEIKNLILK; KLLRERPETRK; RSILLMKDSLK; FVYAREIYSKR; KAIVRSILLMK; TLLNYIQVSAR; GVKYNVSSAIK; SLKIIEIVIDK; ILETGDKSVKK; VSARTATNFVY; TLLSTKQTQMY; NVSSAIKTIVK; SILETGDKSVK; RMLQGSLSFLS; KAREEAKKFT; STRLDNLAKAK; SVKKSLNKKGK |
| | HLA-A1101 | SSAIKTIVK; KSVKKSLNK; SSILETGDK; KIIEIVIDK; SVKKSLNKK; IVRSILLMK; STRLDNLAK; VISEEEILK; TATNFVYAR; TIDSDLRLK; KVEGNAVKK; ILLMKDSLK; QSDLYEAYK; KAKAREEAK; LSGESGELK; TEIKNLILK; VYAREIYSK; ETGDKSVKK; KYNVSSAIK; SVNNSNLSQ; RLDNLAKAK; FISCDLFIR; SLNKKGKDK; ISGSNSISY; VNNSNLSQK; SVNNSNLSQK; FVYAREIYSK; AIVRSILLMK; SILLMKDSLK; VSSAIKTIVK; RTATNFVYAR; FTIDSDLRLK; KSVKKSLNKK; GQSDLYEAYK; NVISEEEILK; YSTRLDNLAK; AISGSNSISY; KTIVKIYDNY; ETEIKNLILK; KSLNKKGKDK; KLDAIETEIK; KAKAREEAKK; ISEEEILKTK; IVIDKNGVWY; FLSGESGELK; KAIVRSILLMK; RSILLMKDSLK; SILETGDKSVK; NVSSAIKTIVK; KLLRERPETRK; GVKYNVSSAIK; STRLDNLAKAK; SVKKSLNKKGK; FVYAREIYSKR; ATNFVYAREIY; STKQTQMYSTR; VISEEEILKTK; SFLSGESGELK; SLKIIEIVIDK; MYSTRLDNLAK; VSARTATNFVY; KGQSDLYEAYK; TLLNYIQVSAR; ESVNNSNLSQK; KAREEAKKFTK; FTKEELEKDLK; IYDNYTLLSTK; QAISGSNSISY; SSAIKTIVKIY; ILETGDKSVKK; LAKAKAREEAK |
| | HLA-A2402 | AYKAIVRSI; MYSTRLDNL; VYAREIYSK; GFISCDLFI; KYNVSSAIK; |

899

|  |  | YYYDGETRL; IYSKRKLDAI; LFGFISCDLF; AYKAIVRSIL; VYAREIYSKR; DYYYDGETRL; SYLEGVKYNV; KYNVSSAIKTI; AYKAIVRSILL; LFGFISCDLFI; IYDNYTLLSTK; VYAREIYSKRK; MYSTRLDNLAK |
|  | HLA-A2902 | YSLEEDYYY; ESYLEGVKY; QYSLEEDYY; VIDKNGVWY; ISGSNSISY; YYYDGETRL; DQYSLEEDY; RMLQGSLSF; TIVKIYDNY; IVIDKNGVWY; QYSLEEDYYY; YESYLEGVKY; SARTATNFVY; LLSTKQTQMY; YTDEIEEEDY; DQYSLEEDYY; EIEEEDYDQY; KTIVKIYDNY; SAIKTIVKIY; VSARTATNFVY; DQYSLEEDYYY; QAISGSNSISY; RYESYLEGVKY; SYTDEIEEEDY; TLLSTKQTQMY; EIVIDKNGVWY; DYDQYSLEEDY |
|  | HLA-A6801 | TATNFVYAR; FISCDLFIR; DPFNHHVKR; YAREIYSKR; EILKTKLLR; DYYYDGETR; ETRKEEIQK; ETGDKSVKK; DFTIDSDLR; DLFIRDEIK; QSDLYEAYK; SVKKSLNKK; SSILETGDK; IVRSILLMK; LNYIQVSAR; SSAIKTIVK; TIVKIYDNY; DNYTLLSTK; ESYLEGVKY; STRLDNLAK; KIIEIVIDK; YEAYKAIVR; VISEEEILK; KVEGNAVKK; TIDSDLRLK; TEIKNLILK; ILLMKDSLK; NFVYAREIY; VYAREIYSK; FVYAREIYSK; RTATNFVYAR; FTIDSDLRLK; NVISEEEILK; SVNNSNLSQK; LLNYIQVSAR; ETEIKNLILK; ESSILETGDK; EDYYYDGETR; YSTRLDNLAK; EIQKQQDEHK; FLSGESGELK; EIKNLILKIK; IDFTIDSDLR; SILLMKDSLK; EEILKTKLLR; YAREIYSKRK; VYAREIYSKR; VSSAIKTIVK; GFISCDLFIR; LYEAYKAIVR; AIVRSILLMK; TATNFVYARE; ESVNNSNLSQ; TKQTQMYSTR; FVYAREIYSKR; ESVNNSNLSQK; STKQTQMYSTR; EIDFTIDSDLR; DLYEAYKAIVR; NVSSAIKTIVK; EIQKQQDEHKR; FTKEELEKDLK; TLLNYIQVSAR; FGFISCDLFIR; EILKTKLLRER; NFVYAREIYSK; DLFIRDEIKEK; MYSTRLDNLAK; RSILLMKDSLK; GVKYNVSSAIK; ATNFVYAREIY; ETRLSNRYESY; DFTIDSDLRLK; STRLDNLAKAK; SVKKSLNKKGK; KAIVRSILLMK; LAKAKAREEAK; QAISGSNSISY; SLKIIEIVIDK; EESSILETGDK; EIVIDKNGVWY; DQYSLEEDYYY; YYDGETRLSNR; SILETGDKSVK; DTIESNEIDFT |
|  | HLA-B0702 | RPETRKEEI; SARTATNFV; KAIVRSILL; KIKSKCLAL; GVKYNVSSAI; YAREIYSKRKL; VARKKVEGNAV; SARTATNFVYA |
|  | HLA-B0801 | YSKRKLDAI; KIKSKCLAL; EIKEKSLGL; RLKSDLQAI; EAKKFTKEEL; ILKIKGQSDL; YIQVSARTAT; YAREIYSKRKL; VARKKVEGNAV |
|  | HLA-B1501 | RMLQGSLSF; GQSDLYEAY; RLSNRYESY; YSLEEDYYY; VSARTATNF; ISGSNSISY; KQQDEHKRM; AIKTIVKIY; FLSGESGEL; KIKGQSDLY; KAREEAKKF; IVIDKNGVW; LSTKQTQMY; AISGSNSISY; QVSARTATNF; IVIDKNGVWY; LQAISGSNSI; SARTATNFVY; LLSTKQTQMY; RMLQGSLSFL; KQQDEHKRML; SAIKTIVKIY; KTIVKIYDNY; KQTQMYSTRL; LLFGFISCDL; IQVSARTATNF; QAISGSNSISY; VSARTATNFVY; LLFGFISCDLF; ILKIKGQSDLY; KSKCLALGLLF; SSAIKTIVKIY; IQKQQDEHKRM; ETRLSNRYESY; KAKAREEAKKF; TLLSTKQTQMY; TQMYSTRLDNL; DQYSLEEDYYY; LMKDSLKIIEI; ATNFVYAREIY; SQKNVISEEEI |
|  | HLA-B2705 | RMLQGSLSF; ARTATNFVY; KRMLQGSLS; KQTQMYSTR; KRMLQGSLSF; TRLSNRYESY; TRLDNLAKAK; AREEAKKFTK; KRESVNNSNL; NRYESYLEGV; KKVEGNAVKK; KRMLQGSLSFL; NRYESYLEGVK; TRLSNRYESYL; ARTATNFVYAR; ARKKVEGNAVK |
|  | HLA-B3501 | YSLEEDYYY; NFVYAREIY; LALGLLFGF; LSTKQTQMY; FTIDSDLRL; ESYLEGVKY; RMLQGSLSF; ISGSNSISY; SARTATNFVY; EIEEEDYDQY; YTDEIEEEDY; SAIKTIVKIY; YESYLEGVKY; LLSTKQTQMY; IVIDKNGVWY; QAISGSNSISY; TLLSTKQTQMY; EIVIDKNGVWY; IKGQSDLYEAY; VSARTATNFVY; YTLLSTKQTQM; YDQYSLEEDYY; EAYKAIVRSIL |
|  | HLA-B4403 | EEILKTKLL; NEIDFTIDS; EEEDYDQYS; EELEKDLKT; GELKDTIES; |

| | | |
|---|---|---|
| | | GETRLSNRY; REIYSKRKL; KEKSLGLCD; EEILKTKLLR; EEEDYDQYSL; TEIKNLILKI; DEIKEKSLGL; EEIQKQQDEH; EEDYYYDGET; EELEKDLKTL; EEEILKTKLL; NEIDFTIDSD; NEIDFTIDSDL; DEIEEEDYDQY; EELEKDLKTLL; IEIVIDKNGVW; EEILKTKLLRE; REIYSKRKLDA; EEEDYDQYSLE; SEEEILKTKLL |
| | HLA-B5101 | LALGLLFGFI; |
| | HLA-B5701 | VSARTATNF; ; VSARTATNFVY |
| AAL84595.1\| BBK32 [Borrelia garinii]; Seq44 | HLA-A0101 | FSTKLTQMY; AIDKNGIWY; ISCDLFIRY; YTDEIEEEDY; NTINKIYDTY; FISCDLFIRY; YLDEDDEDDEY; DSAINTINKIY; VSVKTATNFVY |
| | HLA-A0201 | LLNYIQVSV; GLFDKGNSI; YLEGVKYNV; KTATNFVYI; SLKEVQYAI; AIVSSILLM; KTLLNYIQV; LMRDSLKEV; NLYSAYKAI; KLENIEAKI; YLALGLLFG; KVSGKEPFI; TLLNYIQVS; ILETSEESI; ALGLLFGFI; SLSGESGEL; KAIVSSILL; MLEEESLKT; TLLNYIQVSV; LLMRDSLKEV; LLFGFISCDL; GLFDKGNSIL; RLSNRYQSYL; NLYSAYKAIV; KLTQMYSTRL; QMYSTRLDNL; KIYDTYTLFS; GMTKGSLNSL; SILETSEESI; VMLEEESLKT; YLALGLLFGF; TLFSTKLTQM; SAYKAIVSSI; LALGLLFGFI; YLALGLLFGFI; KIYDTYTLFST; SLQDIAGSNSI; KTLLNYIQVSV; ILLMRDSLKEV; MLEEESLKTEL; GLLFGFISCDL; YTDEIEEEDYA; TINKIYDTYTL; QMYSTRLDNLA; LLNYIQVSVKT; TQMYSTRLDNL; QVSVKTATNFV; YINEMHAKRKL |
| | HLA-A0301 | KSNLYSAYK; ILLMRDSLK; RLSNRYQSY; SIKKPMNKK; TLIAKIKEK; RLDNLAKAK; DTYTLFSTK; VMLEEESLK; IVSSILLMR; KLTQMYSTR; KIKEKSNLY; ESIKKPMNK; STRLDNLAK; KEPFIHSFK; ISCDLFIRY; ESLKTELLK; KNFKTLLNY; QSYLEGVKY; LNYIQVSVK; AIDKNGIWY; LLNYIQVSVK; SLSGESGELK; SILLMRDSLK; KTLIAKIKEK; FVYINEMHAK; KIARKNGKSK; KPMNKKGKGK; KIKTLIAKIK; QQDEYKGMTK; AIVSSILLMR; AANKSNFLQK; RYQSYLEGVK; NVMLEEESLK; ITIDSDLRPK; GKEPFIHSFK; LLKEQSETRK; CDLFIRYEMK; YDTYTLFSTK; EKSNLYSAYK; KLENIEAKIK; SVKTATNFVY; KIQKQQDEYK; EAKIKTLIAK; KIYDTYTLFS; YINEMHAKRK; YSTRLDNLAK; FISCDLFIRY; ILETSEESIK; HSFKRDAANK; YTLFSTKLTQ; TLLNYIQVSVK; TLFSTKLTQMY; RLDNLAKAKAK; SLKEVQYAIDK; SSILLMRDSLK; ILETSEESIKK; FVYINEMHAKR; ELLKEQSETRK; SIKKPMNKKGK; SILETSEESIK; KQQDEYKGMTK; KAKEEAAKFTK; STRLDNLAKAK; NVDSAINTINK |
| | HLA-A1101 | KSNLYSAYK; DTYTLFSTK; IVSSILLMR; STRLDNLAK; ILLMRDSLK; SIKKPMNKK; TLIAKIKEK; ISCDLFIRY; TIDSDLRPK; ESIKKPMNK; VMLEEESLK; ESLKTELLK; ETSEESIKK; VYINEMHAK; DSAINTINK; LSGESGELK; ANKSNFLQK; RLDNLAKAK; YQSYLEGVK; FISCDLFIR; YINEMHAKR; AKIKTLIAK; KEPFIHSFK; KLTQMYSTR; SFKRDAANK; ATNFVYINE; AANKSNFLQK; SILLMRDSLK; ITIDSDLRPK; FVYINEMHAK; KTLIAKIKEK; HSFKRDAANK; SLSGESGELK; NVMLEEESLK; AIVSSILLMR; KIQKQQDEYK; YINEMHAKRK; KIARKNGKSK; YSTRLDNLAK; LLNYIQVSVK; SVKTATNFVY; ESIKKPMNKK; KIKTLIAKIK; KAKAKEEAAK; RYQSYLEGVK; QQDEYKGMTK; KIYDTYTLFS; FISCDLFIRY; SSILLMRDSLK; SILETSEESIK; TLLNYIQVSVK; KAIVSSILLMR; NVDSAINTINK; KQQDEYKGMTK; SLKEVQYAIDK; STRLDNLAKAK; STKLTQMYSTR; NSLSGESGELK; KAKEEAAKFTK; AAKFTKEDLEK; TLFSTKLTQMY; SIKKPMNKKGK; MYSTRLDNLAK; SGKEPFIHSFK; FTKEDLEKNFK; FVYINEMHAKR; DAANKSNFLQK; ILETSEESIKK; QSETRKEKIQK; ATNFVYINEMH; VYINEMHAKRK; IYDTYTLFSTK; RLDNLAKAKAK; VSVKTATNFVY |
| | HLA-A2402 | KYLALGLLF; AYKAIVSSI; YYEDDYEEI; TYTLFSTKL; MYSTRLDNL; GFISCDLFI; VYINEMHAK; NFKTLLNYI; KTATNFVYI; RYQSYLEGV; |

| | | |
|---|---|---|
| | | QYAIDKNGI; KIYDTYTLF; LFGFISCDLF; AYKAIVSSIL; EYYEDDYEEI; IYDTYTLFST; SYLEGVKYNV; RYQSYLEGVK; KYLALGLLFGF; KYNVDSAINTI; AYKAIVSSILL; YYEDDYEEIRL; IYDTYTLFSTK; LFGFISCDLFI; RYQSYLEGVKY; VYINEMHAKRK; MYSTRLDNLAK |
| | HLA-A2902 | QSYLEGVKY; FSTKLTQMY; IAGSNSISY; VKTATNFVY; KYLALGLLF; ISCDLFIRY; AINTINKIY; KIYDTYTLF; TINKIYDTY; RLSNRYQSY; KNFKTLLNY; VYINEMHAK; EKSNLYSAY; YAIDKNGIWY; YQSYLEGVKY; SVKTATNFVY; FISCDLFIRY; LFSTKLTQMY; YLALGLLFGF; SAINTINKIY; EIEEEDYARY; YTDEIEEEDY; DIAGSNSISY; NTINKIYDTY; TLFSTKLTQMY; GFISCDLFIRY; VSVKTATNFVY; QYAIDKNGIWY; RYQSYLEGVKY; YLDEDDEDDEY; EIEEEDYARYY; KYLALGLLFGF; SYTDEIEEEDY; DYEEIRLSNRY; LMRDSLKEVQY |
| | HLA-A6801 | DTYTLFSTK; EPFIHSFKR; YINEMHAKR; IVSSILLMR; ETSEESIKK; DITIDSDLR; FISCDLFIR; DSAINTINK; ESIKKPMNK; EIEEEDYAR; DLFIRYEMK; ETRKEKIQK; ESLKTELLK; KSNLYSAYK; TLIAKIKEK; KLTQMYSTR; FSTKLTQMY; TINKIYDTY; STRLDNLAK; ILLMRDSLK; LLKEQSETR; LNYIQVSVK; VYINEMHAK; SIKKPMNKK; EQSETRKEK; EESPGLFDK; YQSYLEGVK; FVYINEMHAK; HSFKRDAANK; NVMLEEESLK; ESIKKPMNKK; NTINKIYDTY; ITIDSDLRPK; AIVSSILLMR; YINEMHAKRK; YSTRLDNLAK; LLNYIQVSVK; SILLMRDSLK; EKSNLYSAYK; ELLKEQSETR; DYEEIRLSNR; VYINEMHAKR; EAKIKTLIAK; DIAGSNSISY; AANKSNFLQK; IDITIDSDLR; GFISCDLFIR; YYEDDYEEIR; SVKTATNFVY; SLSGESGELK; DAANKSNFLQ; YDTYTLFSTK; FISCDLFIRY; SAINTINKIY; EESLKTELLK; TATNFVYINE; DEIEEEDYAR; CDLFIRYEMK; FVYINEMHAKR; STKLTQMYSTR; EYYEDDYEEIR; FTKEDLEKNFK; DAANKSNFLQK; NFVYINEMHAK; SSILLMRDSLK; EIDITIDSDLR; FGFISCDLFIR; KAIVSSILLMR; NVDSAINTINK; NSLSGESGELK; TLLNYIQVSVK; MYSTRLDNLAK; TLFSTKLTQMY; DITIDSDLRPK; STRLDNLAKAK; ELLKEQSETRK; SILETSEESIK; DSAINTINKIY; SLKEVQYAIDK; DDYEEIRLSNR; EKIQKQQDEYK; MNKKGKGKIAR; EAAKFTKEDLE; QSETRKEKIQK; SGKEPFIHSFK; DTYTLFSTKLT |
| | HLA-B0702 | KVKSKYLAL; RPKSSLQDI; KPMNKKGKG; IARKNGKSKV; RPKSSLQDIA; SAYKAIVSSI; KPMNKKGKGKI; SPGLFDKGNSI; SAYKAIVSSIL; RPKSSLQDIAG; KVKSKYLALGL |
| | HLA-B0801 | KVKSKYLAL; MKKVKSKYL; HAKRKLENI; ; MKKVKSKYLAL |
| | HLA-B1501 | RLSNRYQSY; KIKEKSNLY; KQQDEYKGM; KIYDTYTLF; QSYLEGVKY; FSTKLTQMY; KAKEEAAKF; IAGSNSISY; AINTINKIY; KVKSKYLAL; LMRDSLKEV; MTKGSLNSL; VSVKTATNF; GLFDKGNSI; EMKEESPGL; ISCDLFIRY; KNFKTLLNY; YQSYLEGVKY; YLALGLLFGF; SVKTATNFVY; KSKVSGKEPF; YAIDKNGIWY; EMKEESPGLF; SAINTINKIY; SGKEPFIHSF; QVSVKTATNF; FTKEDLEKNF; GMTKGSLNSL; GLFDKGNSIL; VQYAIDKNGI; RLSNRYQSYL; DIAGSNSISY; KEKSNLYSAY; LLFGFISCDL; LMRDSLKEVQY; IQVSVKTATNF; LLFGFISCDLF; TLFSTKLTQMY; KSKYLALGLLF; IQKQQDEYKGM; VSVKTATNFVY; VQYAIDKNGIW; EIRLSNRYQSY; QDIAGSNSISY; IAKIKEKSNLY; KAKAKEEAAKF; TQMYSTRLDNL; YLDEDDEDDEY; VSGKEPFIHSF |
| | HLA-B2705 | RKLENIEAK; IRLSNRYQSY; KRKLENIEAK; KRDAANKSNF; TRLDNLAKAK; NRYQSYLEGV; NRYQSYLEGVK; RKNGKSKVSGK; IRLSNRYQSYL; KRKLENIEAKI; KQQDEYKGMTK |
| | HLA-B3501 | IAGSNSISY; LALGLLFGF; EKSNLYSAY; FSTKLTQMY; TINKIYDTY; DEDDEDDEY; ISCDLFIRY; EDDEDDEYY; YAIDKNGIW; DAANKSNFL; |

EP 2 254 592 B1

| | | TNFVYINEM; YAIDKNGIWY; SAINTINKIY; FISCDLFIRY; EIEEEDYARY; SVKTATNFVY; DIAGSNSISY; LFSTKLTQMY; YQSYLEGVKY; YLALGLLFGF; YTDEIEEEDY; LDEDDEDDEY; NTINKIYDTY; KAIVSSILLM; DEDDEDDEYY; QVSVKTATNF; TATNFVYINEM; YLDEDDEDDEY; EIEEEDYARYY; ETSEESIKKPM; TLFSTKLTQMY; DSAINTINKIY; VSVKTATNFVY; YTLFSTKLTQM; ISCDLFIRYEM; LDEDDEDDEYY; IAKIKEKSNLY; QDIAGSNSISY; NKSNFLQKNVM; YKAIVSSILLM |
|---|---|---|
| | HLA-B4403 | EEEDYARYY; EEIRLSNRY; NEIDITIDS; GELKETIES; EEDYARYYL; DEDDEDDEY; EESLKTELL; KEVQYAIDK; DEIEEEDYA; SEESIKKPM; EEIRLSNRYQ; EEEDYARYYL; DEDDEDDEYY; YEMKEESPGL; NEIDITIDSD; EEESLKTELL; EEIRLSNRYQS; NEIDITIDSDL; YEMKEESPGLF; DEIEEEDYARY; EEEDYARYYLD; KEKIQKQQDEY; KEVQYAIDKNG; DEDDEYYEDDY |
| | HLA-B5101 | LALGLLFGFI; SPGLFDKGNSI |
| | HLA-B5701 | VSVKTATNF; ISCDLFIRY; VSGKEPFIHSF; VSVKTATNFVY |

# Fig. 33.

| Antigen designation | Class 2 Antigen peptide sequences |
|---|---|
| NP_212517.1 Basic membrane protein A (bmpA) [Borrelia burgdorferi B31 | 13 mers:<br>MNKILLLILLESI; NKILLLILLESIV; KILLLILLESIVF; ILLLILLESIVFL;<br>LLLILLESIVFLS; LLILLESIVFLSC; LILLESIVFLSCS; ILLESIVFLSCSG;<br>LLESIVFLSCSGK; LESIVFLSCSGKG; ESIVFLSCSGKGS; SIVFLSCSGKGSL;<br>IVFLSCSGKGSLG; VFLSCSGKGSLGS; FLSCSGKGSLGSE; LSCSGKGSLGSEI;<br>SCSGKGSLGSEIP; CSGKGSLGSEIPK; SGKGSLGSEIPKV; GKGSLGSEIPKVS;<br>KGSLGSEIPKVSL; GSLGSEIPKVSLI; SLGSEIPKVSLII; LGSEIPKVSLIID;<br>GSEIPKVSLIIDG; SEIPKVSLIIDGT; EIPKVSLIIDGTF; IPKVSLIIDGTFD;<br>PKVSLIIDGTFDD; KVSLIIDGTFDDK; VSLIIDGTFDDKS; SLIIDGTFDDKSF;<br>LIIDGTFDDKSFN; IIDGTFDDKSFNE; IDGTFDDKSFNES; DGTFDDKSFNESA;<br>GTFDDKSFNESAL; TFDDKSFNESALN; FDDKSFNESALNG; DDKSFNESALNGV;<br>DKSFNESALNGVK; KSFNESALNGVKK; SFNESALNGVKKV; FNESALNGVKKVK;<br>NESALNGVKKVKE; ESALNGVKKVKEE; SALNGVKKVKEEF; ALNGVKKVKEEFK;<br>LNGVKKVKEEFKI; NGVKKVKEEFKIE; GVKKVKEEFKIEL; VKKVKEEFKIELV;<br>KKVKEEFKIELVL; KVKEEFKIELVLK; VKEEFKIELVLKE; KEEFKIELVLKES;<br>EEFKIELVLKESS; EFKIELVLKESSS; FKIELVLKESSSN; KIELVLKESSSNS;<br>IELVLKESSSNSY; ELVLKESSSNSYL; LVLKESSSNSYLS; VLKESSSNSYLSD;<br>LKESSSNSYLSDL; KESSSNSYLSDLE; ESSSNSYLSDLEG; SSSNSYLSDLEGL;<br>SSNSYLSDLEGLK; SNSYLSDLEGLKD; NSYLSDLEGLKDA; SYLSDLEGLKDAG;<br>YLSDLEGLKDAGS; LSDLEGLKDAGSD; SDLEGLKDAGSDL; DLEGLKDAGSDLI;<br>LEGLKDAGSDLIW; EGLKDAGSDLIWL; GLKDAGSDLIWLI; LKDAGSDLIWLIG;<br>KDAGSDLIWLIGY; DAGSDLIWLIGYR; AGSDLIWLIGYRF; GSDLIWLIGYRFS;<br>SDLIWLIGYRFSD; DLIWLIGYRFSDV; LIWLIGYRFSDVA; IWLIGYRFSDVAK;<br>WLIGYRFSDVAKV; LIGYRFSDVAKVA; IGYRFSDVAKVAA; GYRFSDVAKVAAL;<br>YRFSDVAKVAALQ; RFSDVAKVAALQN; FSDVAKVAALQNP; SDVAKVAALQNPD;<br>DVAKVAALQNPDM; VAKVAALQNPDMK; AKVAALQNPDMKY; KVAALQNPDMKYA;<br>VAALQNPDMKYAI; AALQNPDMKYAII; ALQNPDMKYAIID; LQNPDMKYAIIDP;<br>QNPDMKYAIIDPI; NPDMKYAIIDPIY; PDMKYAIIDPIYS; DMKYAIIDPIYSN;<br>MKYAIIDPIYSND; KYAIIDPIYSNDP; YAIIDPIYSNDPI; AIIDPIYSNDPIP;<br>IIDPIYSNDPIPA; IDPIYSNDPIPAN; DPIYSNDPIPANL; PIYSNDPIPANLV;<br>IYSNDPIPANLVG; YSNDPIPANLVGM; SNDPIPANLVGMT; NDPIPANLVGMTF;<br>DPIPANLVGMTFR; PIPANLVGMTFRA; IPANLVGMTFRAQ; PANLVGMTFRAQE;<br>ANLVGMTFRAQEG; NLVGMTFRAQEGA; LVGMTFRAQEGAF; VGMTFRAQEGAFL;<br>GMTFRAQEGAFLT; MTFRAQEGAFLTG; TFRAQEGAFLTGY; FRAQEGAFLTGYI;<br>RAQEGAFLTGYIA; AQEGAFLTGYIAA; QEGAFLTGYIAAK; EGAFLTGYIAAKL;<br>GAFLTGYIAAKLS; AFLTGYIAAKLSK; FLTGYIAAKLSKT; LTGYIAAKLSKTG;<br>TGYIAAKLSKTGK; GYIAAKLSKTGKI; YIAAKLSKTGKIG; IAAKLSKTGKIGF;<br>AAKLSKTGKIGFL; AKLSKTGKIGFLG; KLSKTGKIGFLGG; LSKTGKIGFLGGI;<br>SKTGKIGFLGGIE; KTGKIGFLGGIEG; TGKIGFLGGIEGE; GKIGFLGGIEGEI;<br>KIGFLGGIEGEIV; IGFLGGIEGEIVD; GFLGGIEGEIVDA; FLGGIEGEIVDAF;<br>LGGIEGEIVDAFR; GGIEGEIVDAFRY; GIEGEIVDAFRYG; IEGEIVDAFRYGY;<br>EGEIVDAFRYGYE; GEIVDAFRYGYEA; EIVDAFRYGYEAG; IVDAFRYGYEAGA;<br>VDAFRYGYEAGAK; DAFRYGYEAGAKY; AFRYGYEAGAKYA; FRYGYEAGAKYAN;<br>RYGYEAGAKYANK; YGYEAGAKYANKD; GYEAGAKYANKDI; YEAGAKYANKDIK;<br>EAGAKYANKDIKI; AGAKYANKDIKIS; GAKYANKDIKIST; AKYANKDIKISTQ;<br>KYANKDIKISTQY; YANKDIKISTQYI; ANKDIKISTQYIG; NKDIKISTQYIGS;<br>KDIKISTQYIGSF; DIKISTQYIGSFA; IKISTQYIGSFAD; KISTQYIGSFADL;<br>ISTQYIGSFADLE; STQYIGSFADLEA; TQYIGSFADLEAG; QYIGSFADLEAGR;<br>YIGSFADLEAGRS; IGSFADLEAGRSV; GSFADLEAGRSVA; SFADLEAGRSVAT;<br>FADLEAGRSVATR; ADLEAGRSVATRM; DLEAGRSVATRMY; LEAGRSVATRMYS;<br>EAGRSVATRMYSD; AGRSVATRMYSDE; GRSVATRMYSDEI; RSVATRMYSDEID;<br>SVATRMYSDEIDI; VATRMYSDEIDII; ATRMYSDEIDIIH; TRMYSDEIDIIHH; |

RMYSDEIDIIHHA; MYSDEIDIIHHAA; YSDEIDIIHHAAG; SDEIDIIHHAAGL;
DEIDIIHHAAGLG; EIDIIHHAAGLGG; IDIIHHAAGLGGI; DIIHHAAGLGGIG;
IIHHAAGLGGIGA; IHHAAGLGGIGAI; HHAAGLGGIGAIE; HAAGLGGIGAIEV;
AAGLGGIGAIEVA; AGLGGIGAIEVAK; GLGGIGAIEVAKE; LGGIGAIEVAKEL;
GGIGAIEVAKELG; GIGAIEVAKELGS; IGAIEVAKELGSG; GAIEVAKELGSGH;
AIEVAKELGSGHY; IEVAKELGSGHYI; EVAKELGSGHYII; VAKELGSGHYIIG;
AKELGSGHYIIGV; KELGSGHYIIGVD; ELGSGHYIIGVDE; LGSGHYIIGVDED;
GSGHYIIGVDEDQ; SGHYIIGVDEDQA; GHYIIGVDEDQAY; HYIIGVDEDQAYL;
YIIGVDEDQAYLA; IIGVDEDQAYLAP; IGVDEDQAYLAPD; GVDEDQAYLAPDN;
VDEDQAYLAPDNV; DEDQAYLAPDNVI; EDQAYLAPDNVIT; DQAYLAPDNVITS;
QAYLAPDNVITST; AYLAPDNVITSTT; YLAPDNVITSTTK; LAPDNVITSTTKD;
APDNVITSTTKDV; PDNVITSTTKDVG; DNVITSTTKDVGR; NVITSTTKDVGRA;
VITSTTKDVGRAL; ITSTTKDVGRALN; TSTTKDVGRALNI; STTKDVGRALNIF;
TTKDVGRALNIFT; TKDVGRALNIFTS; KDVGRALNIFTSN; DVGRALNIFTSNH;
VGRALNIFTSNHL; GRALNIFTSNHLK; RALNIFTSNHLKT; ALNIFTSNHLKTN;
LNIFTSNHLKTNT; NIFTSNHLKTNTF; IFTSNHLKTNTFE; FTSNHLKTNTFEG;
TSNHLKTNTFEGG; SNHLKTNTFEGGK; NHLKTNTFEGGKL; HLKTNTFEGGKLI;
LKTNTFEGGKLIN; KTNTFEGGKLINY; TNTFEGGKLINYG; NTFEGGKLINYGL;
TFEGGKLINYGLK; FEGGKLINYGLKE; EGGKLINYGLKEG; GGKLINYGLKEGV;
GKLINYGLKEGVV; KLINYGLKEGVVG; LINYGLKEGVVGF; INYGLKEGVVGFV;
NYGLKEGVVGFVR; YGLKEGVVGFVRN; GLKEGVVGFVRNP; LKEGVVGFVRNPK;
KEGVVGFVRNPKM; EGVVGFVRNPKMI; GVVGFVRNPKMIS; VVGFVRNPKMISF;
VGFVRNPKMISFE; GFVRNPKMISFEL; FVRNPKMISFELE; VRNPKMISFELEK;
RNPKMISFELEKE; NPKMISFELEKEI; PKMISFELEKEID; KMISFELEKEIDN;
MISFELEKEIDNL; ISFELEKEIDNLS; SFELEKEIDNLSS; FELEKEIDNLSSK;
ELEKEIDNLSSKI; LEKEIDNLSSKII; EKEIDNLSSKIIN; KEIDNLSSKIINK;
EIDNLSSKIINKE; IDNLSSKIINKEI; DNLSSKIINKEII; NLSSKIINKEIIV;
LSSKIINKEIIVP; SSKIINKEIIVPS; SKIINKEIIVPSN; KIINKEIIVPSNK;
IINKEIIVPSNKE; INKEIIVPSNKES; NKEIIVPSNKESY; KEIIVPSNKESYE;
EIIVPSNKESYEK; IIVPSNKESYEKF; IVPSNKESYEKFL; VPSNKESYEKFLK;
PSNKESYEKFLKE; SNKESYEKFLKEF; NKESYEKFLKEFI;

14 mers:
MNKILLLILLESIV; NKILLLILLESIVF; KILLLILLESIVFL;
ILLLILLESIVFLS; LLLILLESIVFLSC; LLILLESIVFLSCS;
LILLESIVFLSCSG; ILLESIVFLSCSGK; LLESIVFLSCSGKG;
LESIVFLSCSGKGS; ESIVFLSCSGKGSL; SIVFLSCSGKGSLG;
IVFLSCSGKGSLGS; VFLSCSGKGSLGSE; FLSCSGKGSLGSEI;
LSCSGKGSLGSEIP; SCSGKGSLGSEIPK; CSGKGSLGSEIPKV;
SGKGSLGSEIPKVS; GKGSLGSEIPKVSL; KGSLGSEIPKVSLI;
GSLGSEIPKVSLII; SLGSEIPKVSLIID; LGSEIPKVSLIIDG;
GSEIPKVSLIIDGT; SEIPKVSLIIDGTF; EIPKVSLIIDGTFD;
IPKVSLIIDGTFDD; PKVSLIIDGTFDDK; KVSLIIDGTFDDKS;
VSLIIDGTFDDKSF; SLIIDGTFDDKSFN; LIIDGTFDDKSFNE;
IIDGTFDDKSFNES; IDGTFDDKSFNESA; DGTFDDKSFNESAL;
GTFDDKSFNESALN; TFDDKSFNESALNG; FDDKSFNESALNGV;
DDKSFNESALNGVK; DKSFNESALNGVKK; KSFNESALNGVKKV;
SFNESALNGVKKVK; FNESALNGVKKVKE; NESALNGVKKVKEE;
ESALNGVKKVKEEF; SALNGVKKVKEEFK; ALNGVKKVKEEFKI;
LNGVKKVKEEFKIE; NGVKKVKEEFKIEL; GVKKVKEEFKIELV;
VKKVKEEFKIELVL; KKVKEEFKIELVLK; KVKEEFKIELVLKE;
VKEEFKIELVLKES; KEEFKIELVLKESS; EEFKIELVLKESSS;
EFKIELVLKESSSN; FKIELVLKESSSNS; KIELVLKESSSNSY;
IELVLKESSSNSYL; ELVLKESSSNSYLS; LVLKESSSNSYLSD;
VLKESSSNSYLSDL; LKESSSNSYLSDLE; KESSSNSYLSDLEG;
ESSSNSYLSDLEGL; SSSNSYLSDLEGLK; SSNSYLSDLEGLKD;
SNSYLSDLEGLKDA; NSYLSDLEGLKDAG; SYLSDLEGLKDAGS;

| | | |
|---|---|---|
| YLSDLEGLKDAGSD; | LSDLEGLKDAGSDL; | SDLEGLKDAGSDLI; |
| DLEGLKDAGSDLIW; | LEGLKDAGSDLIWL; | EGLKDAGSDLIWLI; |
| GLKDAGSDLIWLIG; | LKDAGSDLIWLIGY; | KDAGSDLIWLIGYR; |
| DAGSDLIWLIGYRF; | AGSDLIWLIGYRFS; | GSDLIWLIGYRFSD; |
| SDLIWLIGYRFSDV; | DLIWLIGYRFSDVA; | LIWLIGYRFSDVAK; |
| IWLIGYRFSDVAKV; | WLIGYRFSDVAKVA; | LIGYRFSDVAKVAA; |
| IGYRFSDVAKVAAL; | GYRFSDVAKVAALQ; | YRFSDVAKVAALQN; |
| RFSDVAKVAALQNP; | FSDVAKVAALQNPD; | SDVAKVAALQNPDM; |
| DVAKVAALQNPDMK; | VAKVAALQNPDMKY; | AKVAALQNPDMKYA; |
| KVAALQNPDMKYAI; | VAALQNPDMKYAII; | AALQNPDMKYAIID; |
| ALQNPDMKYAIIDP; | LQNPDMKYAIIDPI; | QNPDMKYAIIDPIY; |
| NPDMKYAIIDPIYS; | PDMKYAIIDPIYSN; | DMKYAIIDPIYSND; |
| MKYAIIDPIYSNDP; | KYAIIDPIYSNDPI; | YAIIDPIYSNDPIP; |
| AIIDPIYSNDPIPA; | IIDPIYSNDPIPAN; | IDPIYSNDPIPANL; |
| DPIYSNDPIPANLV; | PIYSNDPIPANLVG; | IYSNDPIPANLVGM; |
| YSNDPIPANLVGMT; | SNDPIPANLVGMTF; | NDPIPANLVGMTFR; |
| DPIPANLVGMTFRA; | PIPANLVGMTFRAQ; | IPANLVGMTFRAQE; |
| PANLVGMTFRAQEG; | ANLVGMTFRAQEGA; | NLVGMTFRAQEGAF; |
| LVGMTFRAQEGAFL; | VGMTFRAQEGAFLT; | GMTFRAQEGAFLTG; |
| MTFRAQEGAFLTGY; | TFRAQEGAFLTGYI; | FRAQEGAFLTGYIA; |
| RAQEGAFLTGYIAA; | AQEGAFLTGYIAAK; | QEGAFLTGYIAAKL; |
| EGAFLTGYIAAKLS; | GAFLTGYIAAKLSK; | AFLTGYIAAKLSKT; |
| FLTGYIAAKLSKTG; | LTGYIAAKLSKTGK; | TGYIAAKLSKTGKI; |
| GYIAAKLSKTGKIG; | YIAAKLSKTGKIGF; | IAAKLSKTGKIGFL; |
| AAKLSKTGKIGFLG; | AKLSKTGKIGFLGG; | KLSKTGKIGFLGGI; |
| LSKTGKIGFLGGIE; | SKTGKIGFLGGIEG; | KTGKIGFLGGIEGE; |
| TGKIGFLGGIEGEI; | GKIGFLGGIEGEIV; | KIGFLGGIEGEIVD; |
| IGFLGGIEGEIVDA; | GFLGGIEGEIVDAF; | FLGGIEGEIVDAFR; |
| LGGIEGEIVDAFRY; | GGIEGEIVDAFRYG; | GIEGEIVDAFRYGY; |
| IEGEIVDAFRYGYE; | EGEIVDAFRYGYEA; | GEIVDAFRYGYEAG; |
| EIVDAFRYGYEAGA; | IVDAFRYGYEAGAK; | VDAFRYGYEAGAKY; |
| DAFRYGYEAGAKYA; | AFRYGYEAGAKYAN; | FRYGYEAGAKYANK; |
| RYGYEAGAKYANKD; | YGYEAGAKYANKDI; | GYEAGAKYANKDIK; |
| YEAGAKYANKDIKI; | EAGAKYANKDIKIS; | AGAKYANKDIKIST; |
| GAKYANKDIKISTQ; | AKYANKDIKISTQY; | KYANKDIKISTQYI; |
| YANKDIKISTQYIG; | ANKDIKISTQYIGS; | NKDIKISTQYIGSF; |
| KDIKISTQYIGSFA; | DIKISTQYIGSFAD; | IKISTQYIGSFADL; |
| KISTQYIGSFADLE; | ISTQYIGSFADLEA; | STQYIGSFADLEAG; |
| TQYIGSFADLEAGR; | QYIGSFADLEAGRS; | YIGSFADLEAGRSV; |
| IGSFADLEAGRSVA; | GSFADLEAGRSVAT; | SFADLEAGRSVATR; |
| FADLEAGRSVATRM; | ADLEAGRSVATRMY; | DLEAGRSVATRMYS; |
| LEAGRSVATRMYSD; | EAGRSVATRMYSDE; | AGRSVATRMYSDEI; |
| GRSVATRMYSDEID; | RSVATRMYSDEIDI; | SVATRMYSDEIDII; |
| VATRMYSDEIDIIH; | ATRMYSDEIDIIHH; | TRMYSDEIDIIHHA; |
| RMYSDEIDIIHHAA; | MYSDEIDIIHHAAG; | YSDEIDIIHHAAGL; |
| SDEIDIIHHAAGLG; | DEIDIIHHAAGLGG; | EIDIIHHAAGLGGI; |
| IDIIHHAAGLGGIG; | DIIHHAAGLGGIGA; | IIHHAAGLGGIGAI; |
| IHHAAGLGGIGAIE; | HHAAGLGGIGAIEV; | HAAGLGGIGAIEVA; |
| AAGLGGIGAIEVAK; | AGLGGIGAIEVAKE; | GLGGIGAIEVAKEL; |
| LGGIGAIEVAKELG; | GGIGAIEVAKELGS; | GIGAIEVAKELGSG; |
| IGAIEVAKELGSGH; | GAIEVAKELGSGHY; | AIEVAKELGSGHYI; |
| IEVAKELGSGHYII; | EVAKELGSGHYIIG; | VAKELGSGHYIIGV; |
| AKELGSGHYIIGVD; | KELGSGHYIIGVDE; | ELGSGHYIIGVDED; |
| LGSGHYIIGVDEDQ; | GSGHYIIGVDEDQA; | SGHYIIGVDEDQAY; |
| GHYIIGVDEDQAYL; | HYIIGVDEDQAYLA; | YIIGVDEDQAYLAP; |
| IIGVDEDQAYLAPD; | IGVDEDQAYLAPDN; | GVDEDQAYLAPDNV; |
| VDEDQAYLAPDNVI; | DEDQAYLAPDNVIT; | EDQAYLAPDNVITS; |

DQAYLAPDNVITST; QAYLAPDNVITSTT; AYLAPDNVITSTTK;
YLAPDNVITSTTKD; LAPDNVITSTTKDV; APDNVITSTTKDVG;
PDNVITSTTKDVGR; DNVITSTTKDVGRA; NVITSTTKDVGRAL;
VITSTTKDVGRALN; ITSTTKDVGRALNI; TSTTKDVGRALNIF;
STTKDVGRALNIFT; TTKDVGRALNIFTS; TKDVGRALNIFTSN;
KDVGRALNIFTSNH; DVGRALNIFTSNHL; VGRALNIFTSNHLK;
GRALNIFTSNHLKT; RALNIFTSNHLKTN; ALNIFTSNHLKTNT;
LNIFTSNHLKTNTF; NIFTSNHLKTNTFE; IFTSNHLKTNTFEG;
FTSNHLKTNTFEGG; TSNHLKTNTFEGGK; SNHLKTNTFEGGKL;
NHLKTNTFEGGKLI; HLKTNTFEGGKLIN; LKTNTFEGGKLINY;
KTNTFEGGKLINYG; TNTFEGGKLINYGL; NTFEGGKLINYGLK;
TFEGGKLINYGLKE; FEGGKLINYGLKEG; EGGKLINYGLKEGV;
GGKLINYGLKEGVV; GKLINYGLKEGVVG; KLINYGLKEGVVGF;
LINYGLKEGVVGFV; INYGLKEGVVGFVR; NYGLKEGVVGFVRN;
YGLKEGVVGFVRNP; GLKEGVVGFVRNPK; LKEGVVGFVRNPKM;
KEGVVGFVRNPKMI; EGVVGFVRNPKMIS; GVVGFVRNPKMISF;
VVGFVRNPKMISFE; VGFVRNPKMISFEL; GFVRNPKMISFELE;
FVRNPKMISFELEK; VRNPKMISFELEKE; RNPKMISFELEKEI;
NPKMISFELEKEID; PKMISFELEKEIDN; KMISFELEKEIDNL;
MISFELEKEIDNLS; ISFELEKEIDNLSS; SFELEKEIDNLSSK;
FELEKEIDNLSSKI; ELEKEIDNLSSKII; LEKEIDNLSSKIIN;
EKEIDNLSSKIINK; KEIDNLSSKIINKE; EIDNLSSKIINKEI;
IDNLSSKIINKEII; DNLSSKIINKEIIV; NLSSKIINKEIIVP;
LSSKIINKEIIVPS; SSKIINKEIIVPSN; SKIINKEIIVPSNK;
KIINKEIIVPSNKE; IINKEIIVPSNKES; INKEIIVPSNKESY;
NKEIIVPSNKESYE; KEIIVPSNKESYEK; EIIVPSNKESYEKF;
IIVPSNKESYEKFL; IVPSNKESYEKFLK; VPSNKESYEKFLKE;
PSNKESYEKFLKEF; SNKESYEKFLKEFI;

15 mers:
MNKILLLILLESIVF; NKILLLILLESIVFL; KILLLILLESIVFLS;
ILLLILLESIVFLSC; LLLILLESIVFLSCS; LLILLESIVFLSCSG;
LILLESIVFLSCSGK; ILLESIVFLSCSGKG; LLESIVFLSCSGKGS;
LESIVFLSCSGKGSL; ESIVFLSCSGKGSLG; SIVFLSCSGKGSLGS;
IVFLSCSGKGSLGSE; VFLSCSGKGSLGSEI; FLSCSGKGSLGSEIP;
LSCSGKGSLGSEIPK; SCSGKGSLGSEIPKV; CSGKGSLGSEIPKVS;
SGKGSLGSEIPKVSL; GKGSLGSEIPKVSLI; KGSLGSEIPKVSLII;
GSLGSEIPKVSLIID; SLGSEIPKVSLIIDG; LGSEIPKVSLIIDGT;
GSEIPKVSLIIDGTF; SEIPKVSLIIDGTFD; EIPKVSLIIDGTFDD;
IPKVSLIIDGTFDDK; PKVSLIIDGTFDDKS; KVSLIIDGTFDDKSF;
VSLIIDGTFDDKSFN; SLIIDGTFDDKSFNE; LIIDGTFDDKSFNES;
IIDGTFDDKSFNESA; IDGTFDDKSFNESAL; DGTFDDKSFNESALN;
GTFDDKSFNESALNG; TFDDKSFNESALNGV; FDDKSFNESALNGVK;
DDKSFNESALNGVKK; DKSFNESALNGVKKV; KSFNESALNGVKKVK;
SFNESALNGVKKVKE; FNESALNGVKKVKEE; NESALNGVKKVKEEF;
ESALNGVKKVKEEFK; SALNGVKKVKEEFKI; ALNGVKKVKEEFKIE;
LNGVKKVKEEFKIEL; NGVKKVKEEFKIELV; GVKKVKEEFKIELVL;
VKKVKEEFKIELVLK; KKVKEEFKIELVLKE; KVKEEFKIELVLKES;
VKEEFKIELVLKESS; KEEFKIELVLKESSS; EEFKIELVLKESSSN;
EFKIELVLKESSSNS; FKIELVLKESSSNSY; KIELVLKESSSNSYL;
IELVLKESSSNSYLS; ELVLKESSSNSYLSD; LVLKESSSNSYLSDL;
VLKESSSNSYLSDLE; LKESSSNSYLSDLEG; KESSSNSYLSDLEGL;
ESSSNSYLSDLEGLK; SSSNSYLSDLEGLKD; SSNSYLSDLEGLKDA;
SNSYLSDLEGLKDAG; NSYLSDLEGLKDAGS; SYLSDLEGLKDAGSD;
YLSDLEGLKDAGSDL; LSDLEGLKDAGSDLI; SDLEGLKDAGSDLIW;
DLEGLKDAGSDLIWL; LEGLKDAGSDLIWLI; EGLKDAGSDLIWLIG;
GLKDAGSDLIWLIGY; LKDAGSDLIWLIGYR; KDAGSDLIWLIGYRF;

DAGSDLIWLIGYRFS; AGSDLIWLIGYRFSD; GSDLIWLIGYRFSDV;
SDLIWLIGYRFSDVA; DLIWLIGYRFSDVAK; LIWLIGYRFSDVAKV;
IWLIGYRFSDVAKVA; WLIGYRFSDVAKVAA; LIGYRFSDVAKVAAL;
IGYRFSDVAKVAALQ; GYRFSDVAKVAALQN; YRFSDVAKVAALQNP;
RFSDVAKVAALQNPD; FSDVAKVAALQNPDM; SDVAKVAALQNPDMK;
DVAKVAALQNPDMKY; VAKVAALQNPDMKYA; AKVAALQNPDMKYAI;
KVAALQNPDMKYAII; VAALQNPDMKYAIID; AALQNPDMKYAIIDP;
ALQNPDMKYAIIDPI; LQNPDMKYAIIDPIY; QNPDMKYAIIDPIYS;
NPDMKYAIIDPIYSN; PDMKYAIIDPIYSND; DMKYAIIDPIYSNDP;
MKYAIIDPIYSNDPI; KYAIIDPIYSNDPIP; YAIIDPIYSNDPIPA;
AIIDPIYSNDPIPAN; IIDPIYSNDPIPANL; IDPIYSNDPIPANLV;
DPIYSNDPIPANLVG; PIYSNDPIPANLVGM; IYSNDPIPANLVGMT;
YSNDPIPANLVGMTF; SNDPIPANLVGMTFR; NDPIPANLVGMTFRA;
DPIPANLVGMTFRAQ; PIPANLVGMTFRAQE; IPANLVGMTFRAQEG;
PANLVGMTFRAQEGA; ANLVGMTFRAQEGAF; NLVGMTFRAQEGAFL;
LVGMTFRAQEGAFLT; VGMTFRAQEGAFLTG; GMTFRAQEGAFLTGY;
MTFRAQEGAFLTGYI; TFRAQEGAFLTGYIA; FRAQEGAFLTGYIAA;
RAQEGAFLTGYIAAK; AQEGAFLTGYIAAKL; QEGAFLTGYIAAKLS;
EGAFLTGYIAAKLSK; GAFLTGYIAAKLSKT; AFLTGYIAAKLSKTG;
FLTGYIAAKLSKTGK; LTGYIAAKLSKTGKI; TGYIAAKLSKTGKIG;
GYIAAKLSKTGKIGF; YIAAKLSKTGKIGFL; IAAKLSKTGKIGFLG;
AAKLSKTGKIGFLGG; AKLSKTGKIGFLGGI; KLSKTGKIGFLGGIE;
LSKTGKIGFLGGIEG; SKTGKIGFLGGIEGE; KTGKIGFLGGIEGEI;
TGKIGFLGGIEGEIV; GKIGFLGGIEGEIVD; KIGFLGGIEGEIVDA;
IGFLGGIEGEIVDAF; GFLGGIEGEIVDAFR; FLGGIEGEIVDAFRY;
LGGIEGEIVDAFRYG; GGIEGEIVDAFRYGY; GIEGEIVDAFRYGYE;
IEGEIVDAFRYGYEA; EGEIVDAFRYGYEAG; GEIVDAFRYGYEAGA;
EIVDAFRYGYEAGAK; IVDAFRYGYEAGAKY; VDAFRYGYEAGAKYA;
DAFRYGYEAGAKYAN; AFRYGYEAGAKYANK; FRYGYEAGAKYANKD;
RYGYEAGAKYANKDI; YGYEAGAKYANKDIK; GYEAGAKYANKDIKI;
YEAGAKYANKDIKIS; EAGAKYANKDIKIST; AGAKYANKDIKISTQ;
GAKYANKDIKISTQY; AKYANKDIKISTQYI; KYANKDIKISTQYIG;
YANKDIKISTQYIGS; ANKDIKISTQYIGSF; NKDIKISTQYIGSFA;
KDIKISTQYIGSFAD; DIKISTQYIGSFADL; IKISTQYIGSFADLE;
KISTQYIGSFADLEA; ISTQYIGSFADLEAG; STQYIGSFADLEAGR;
TQYIGSFADLEAGRS; QYIGSFADLEAGRSV; YIGSFADLEAGRSVA;
IGSFADLEAGRSVAT; GSFADLEAGRSVATR; SFADLEAGRSVATRM;
FADLEAGRSVATRMY; ADLEAGRSVATRMYS; DLEAGRSVATRMYSD;
LEAGRSVATRMYSDE; EAGRSVATRMYSDEI; AGRSVATRMYSDEID;
GRSVATRMYSDEIDI; RSVATRMYSDEIDII; SVATRMYSDEIDIIH;
VATRMYSDEIDIIHH; ATRMYSDEIDIIHHA; TRMYSDEIDIIHHAA;
RMYSDEIDIIHHAAG; MYSDEIDIIHHAAGL; YSDEIDIIHHAAGLG;
SDEIDIIHHAAGLGG; DEIDIIHHAAGLGGI; EIDIIHHAAGLGGIG;
IDIIHHAAGLGGIGA; DIIHHAAGLGGIGAI; IIHHAAGLGGIGAIE;
IHHAAGLGGIGAIEV; HHAAGLGGIGAIEVA; HAAGLGGIGAIEVAK;
AAGLGGIGAIEVAKE; AGLGGIGAIEVAKEL; GLGGIGAIEVAKELG;
LGGIGAIEVAKELGS; GGIGAIEVAKELGSG; GIGAIEVAKELGSGH;
IGAIEVAKELGSGHY; GAIEVAKELGSGHYI; AIEVAKELGSGHYII;
IEVAKELGSGHYIIG; EVAKELGSGHYIIGV; VAKELGSGHYIIGVD;
AKELGSGHYIIGVDE; KELGSGHYIIGVDED; ELGSGHYIIGVDEDQ;
LGSGHYIIGVDEDQA; GSGHYIIGVDEDQAY; SGHYIIGVDEDQAYL;
GHYIIGVDEDQAYLA; HYIIGVDEDQAYLAP; YIIGVDEDQAYLAPD;
IIGVDEDQAYLAPDN; IGVDEDQAYLAPDNV; GVDEDQAYLAPDNVI;
VDEDQAYLAPDNVIT; DEDQAYLAPDNVITS; EDQAYLAPDNVITST;
DQAYLAPDNVITSTT; QAYLAPDNVITSTTK; AYLAPDNVITSTTKD;
YLAPDNVITSTTKDV; LAPDNVITSTTKDVG; APDNVITSTTKDVGR;
PDNVITSTTKDVGRA; DNVITSTTKDVGRAL; NVITSTTKDVGRALN;

VITSTTKDVGRALNI; ITSTTKDVGRALNIF; TSTTKDVGRALNIFT;
STTKDVGRALNIFTS; TTKDVGRALNIFTSN; TKDVGRALNIFTSNH;
KDVGRALNIFTSNHL; DVGRALNIFTSNHLK; VGRALNIFTSNHLKT;
GRALNIFTSNHLKTN; RALNIFTSNHLKTNT; ALNIFTSNHLKTNTF;
LNIFTSNHLKTNTFE; NIFTSNHLKTNTFEG; IFTSNHLKTNTFEGG;
FTSNHLKTNTFEGGK; TSNHLKTNTFEGGKL; SNHLKTNTFEGGKLI;
NHLKTNTFEGGKLIN; HLKTNTFEGGKLINY; LKTNTFEGGKLINYG;
KTNTFEGGKLINYGL; TNTFEGGKLINYGLK; NTFEGGKLINYGLKE;
TFEGGKLINYGLKEG; FEGGKLINYGLKEGV; EGGKLINYGLKEGVV;
GGKLINYGLKEGVVG; GKLINYGLKEGVVGF; KLINYGLKEGVVGFV;
LINYGLKEGVVGFVR; INYGLKEGVVGFVRN; NYGLKEGVVGFVRNP;
YGLKEGVVGFVRNPK; GLKEGVVGFVRNPKM; LKEGVVGFVRNPKMI;
KEGVVGFVRNPKMIS; EGVVGFVRNPKMISF; GVVGFVRNPKMISFE;
VVGFVRNPKMISFEL; VGFVRNPKMISFELE; GFVRNPKMISFELEK;
FVRNPKMISFELEKE; VRNPKMISFELEKEI; RNPKMISFELEKEID;
NPKMISFELEKEIDN; PKMISFELEKEIDNL; KMISFELEKEIDNLS;
MISFELEKEIDNLSS; ISFELEKEIDNLSSK; SFELEKEIDNLSSKI;
FELEKEIDNLSSKII; ELEKEIDNLSSKIIN; LEKEIDNLSSKIINK;
EKEIDNLSSKIINKE; KEIDNLSSKIINKEI; EIDNLSSKIINKEII;
IDNLSSKIINKEIIV; DNLSSKIINKEIIVP; NLSSKIINKEIIVPS;
LSSKIINKEIIVPSN; SSKIINKEIIVPSNK; SKIINKEIIVPSNKE;
KIINKEIIVPSNKES; IINKEIIVPSNKESY; INKEIIVPSNKESYE;
NKEIIVPSNKESYEK; KEIIVPSNKESYEKF; EIIVPSNKESYEKFL;
IIVPSNKESYEKFLK; IVPSNKESYEKFLKE; VPSNKESYEKFLKEF;
PSNKESYEKFLKEFI;

16 mers:
MNKILLLILLESIVFL; NKILLLILLESIVFLS; KILLLILLESIVFLSC;
ILLLILLESIVFLSCS; LLLILLESIVFLSCSG; LLILLESIVFLSCSGK;
LILLESIVFLSCSGKG; ILLESIVFLSCSGKGS; LLESIVFLSCSGKGSL;
LESIVFLSCSGKGSLG; ESIVFLSCSGKGSLGS; SIVFLSCSGKGSLGSE;
IVFLSCSGKGSLGSEI; VFLSCSGKGSLGSEIP; FLSCSGKGSLGSEIPK;
LSCSGKGSLGSEIPKV; SCSGKGSLGSEIPKVS; CSGKGSLGSEIPKVSL;
SGKGSLGSEIPKVSLI; GKGSLGSEIPKVSLII; KGSLGSEIPKVSLIID;
GSLGSEIPKVSLIIDG; SLGSEIPKVSLIIDGT; LGSEIPKVSLIIDGTF;
GSEIPKVSLIIDGTFD; SEIPKVSLIIDGTFDD; EIPKVSLIIDGTFDDK;
IPKVSLIIDGTFDDKS; PKVSLIIDGTFDDKSF; KVSLIIDGTFDDKSFN;
VSLIIDGTFDDKSFNE; SLIIDGTFDDKSFNES; LIIDGTFDDKSFNESA;
IIDGTFDDKSFNESAL; IDGTFDDKSFNESALN; DGTFDDKSFNESALNG;
GTFDDKSFNESALNGV; TFDDKSFNESALNGVK; FDDKSFNESALNGVKK;
DDKSFNESALNGVKKV; DKSFNESALNGVKKVK; KSFNESALNGVKKVKE;
SFNESALNGVKKVKEE; FNESALNGVKKVKEEF; NESALNGVKKVKEEFK;
ESALNGVKKVKEEFKI; SALNGVKKVKEEFKIE; ALNGVKKVKEEFKIEL;
LNGVKKVKEEFKIELV; NGVKKVKEEFKIELVL; GVKKVKEEFKIELVLK;
VKKVKEEFKIELVLKE; KKVKEEFKIELVLKES; KVKEEFKIELVLKESS;
VKEEFKIELVLKESSS; KEEFKIELVLKESSSN; EEFKIELVLKESSSNS;
EFKIELVLKESSSNSY; FKIELVLKESSSNSYL; KIELVLKESSSNSYLS;
IELVLKESSSNSYLSD; ELVLKESSSNSYLSDL; LVLKESSSNSYLSDLE;
VLKESSSNSYLSDLEG; LKESSSNSYLSDLEGL; KESSSNSYLSDLEGLK;
ESSSNSYLSDLEGLKD; SSSNSYLSDLEGLKDA; SSNSYLSDLEGLKDAG;
SNSYLSDLEGLKDAGS; NSYLSDLEGLKDAGSD; SYLSDLEGLKDAGSDL;
YLSDLEGLKDAGSDLI; LSDLEGLKDAGSDLIW; SDLEGLKDAGSDLIWL;
DLEGLKDAGSDLIWLI; LEGLKDAGSDLIWLIG; EGLKDAGSDLIWLIGY;
GLKDAGSDLIWLIGYR; LKDAGSDLIWLIGYRF; KDAGSDLIWLIGYRFS;
DAGSDLIWLIGYRFSD; AGSDLIWLIGYRFSDV; GSDLIWLIGYRFSDVA;
SDLIWLIGYRFSDVAK; DLIWLIGYRFSDVAKV; LIWLIGYRFSDVAKVA;
IWLIGYRFSDVAKVAA; WLIGYRFSDVAKVAAL; LIGYRFSDVAKVAALQ;

| | | |
|---|---|---|
| IGYRFSDVAKVAALQN; | GYRFSDVAKVAALQNP; | YRFSDVAKVAALQNPD; |
| RFSDVAKVAALQNPDM; | FSDVAKVAALQNPDMK; | SDVAKVAALQNPDMKY; |
| DVAKVAALQNPDMKYA; | VAKVAALQNPDMKYAI; | AKVAALQNPDMKYAII; |
| KVAALQNPDMKYAIID; | VAALQNPDMKYAIIDP; | AALQNPDMKYAIIDPI; |
| ALQNPDMKYAIIDPIY; | LQNPDMKYAIIDPIYS; | QNPDMKYAIIDPIYSN; |
| NPDMKYAIIDPIYSND; | PDMKYAIIDPIYSNDP; | DMKYAIIDPIYSNDPI; |
| MKYAIIDPIYSNDPIP; | KYAIIDPIYSNDPIPA; | YAIIDPIYSNDPIPAN; |
| AIIDPIYSNDPIPANL; | IIDPIYSNDPIPANLV; | IDPIYSNDPIPANLVG; |
| DPIYSNDPIPANLVGM; | PIYSNDPIPANLVGMT; | IYSNDPIPANLVGMTF; |
| YSNDPIPANLVGMTFR; | SNDPIPANLVGMTFRA; | NDPIPANLVGMTFRAQ; |
| DPIPANLVGMTFRAQE; | PIPANLVGMTFRAQEG; | IPANLVGMTFRAQEGA; |
| PANLVGMTFRAQEGAF; | ANLVGMTFRAQEGAFL; | NLVGMTFRAQEGAFLT; |
| LVGMTFRAQEGAFLTG; | VGMTFRAQEGAFLTGY; | GMTFRAQEGAFLTGYI; |
| MTFRAQEGAFLTGYIA; | TFRAQEGAFLTGYIAA; | FRAQEGAFLTGYIAAK; |
| RAQEGAFLTGYIAAKL; | AQEGAFLTGYIAAKLS; | QEGAFLTGYIAAKLSK; |
| EGAFLTGYIAAKLSKT; | GAFLTGYIAAKLSKTG; | AFLTGYIAAKLSKTGK; |
| FLTGYIAAKLSKTGKI; | LTGYIAAKLSKTGKIG; | TGYIAAKLSKTGKIGF; |
| GYIAAKLSKTGKIGFL; | YIAAKLSKTGKIGFLG; | IAAKLSKTGKIGFLGG; |
| AAKLSKTGKIGFLGGI; | AKLSKTGKIGFLGGIE; | KLSKTGKIGFLGGIEG; |
| LSKTGKIGFLGGIEGE; | SKTGKIGFLGGIEGEI; | KTGKIGFLGGIEGEIV; |
| TGKIGFLGGIEGEIVD; | GKIGFLGGIEGEIVDA; | KIGFLGGIEGEIVDAF; |
| IGFLGGIEGEIVDAFR; | GFLGGIEGEIVDAFRY; | FLGGIEGEIVDAFRYG; |
| LGGIEGEIVDAFRYGY; | GGIEGEIVDAFRYGYE; | GIEGEIVDAFRYGYEA; |
| IEGEIVDAFRYGYEAG; | EGEIVDAFRYGYEAGA; | GEIVDAFRYGYEAGAK; |
| EIVDAFRYGYEAGAKY; | IVDAFRYGYEAGAKYA; | VDAFRYGYEAGAKYAN; |
| DAFRYGYEAGAKYANK; | AFRYGYEAGAKYANKD; | FRYGYEAGAKYANKDI; |
| RYGYEAGAKYANKDIK; | YGYEAGAKYANKDIKI; | GYEAGAKYANKDIKIS; |
| YEAGAKYANKDIKIST; | EAGAKYANKDIKISTQ; | AGAKYANKDIKISTQY; |
| GAKYANKDIKISTQYI; | AKYANKDIKISTQYIG; | KYANKDIKISTQYIGS; |
| YANKDIKISTQYIGSF; | ANKDIKISTQYIGSFA; | NKDIKISTQYIGSFAD; |
| KDIKISTQYIGSFADL; | DIKISTQYIGSFADLE; | IKISTQYIGSFADLEA; |
| KISTQYIGSFADLEAG; | ISTQYIGSFADLEAGR; | STQYIGSFADLEAGRS; |
| TQYIGSFADLEAGRSV; | QYIGSFADLEAGRSVA; | YIGSFADLEAGRSVAT; |
| IGSFADLEAGRSVATR; | GSFADLEAGRSVATRM; | SFADLEAGRSVATRMY; |
| FADLEAGRSVATRMYS; | ADLEAGRSVATRMYSD; | DLEAGRSVATRMYSDE; |
| LEAGRSVATRMYSDEI; | EAGRSVATRMYSDEID; | AGRSVATRMYSDEIDI; |
| GRSVATRMYSDEIDII; | RSVATRMYSDEIDIIH; | SVATRMYSDEIDIIHH; |
| VATRMYSDEIDIIHHA; | ATRMYSDEIDIIHHAA; | TRMYSDEIDIIHHAAG; |
| RMYSDEIDIIHHAAGL; | MYSDEIDIIHHAAGLG; | YSDEIDIIHHAAGLGG; |
| SDEIDIIHHAAGLGGI; | DEIDIIHHAAGLGGIG; | EIDIIHHAAGLGGIGA; |
| IDIIHHAAGLGGIGAI; | DIIHHAAGLGGIGAIE; | IIHHAAGLGGIGAIEV; |
| IHHAAGLGGIGAIEVA; | HHAAGLGGIGAIEVAK; | HAAGLGGIGAIEVAKE; |
| AAGLGGIGAIEVAKEL; | AGLGGIGAIEVAKELG; | GLGGIGAIEVAKELGS; |
| LGGIGAIEVAKELGSG; | GGIGAIEVAKELGSGH; | GIGAIEVAKELGSGHY; |
| IGAIEVAKELGSGHYI; | GAIEVAKELGSGHYII; | AIEVAKELGSGHYIIG; |
| IEVAKELGSGHYIIGV; | EVAKELGSGHYIIGVD; | VAKELGSGHYIIGVDE; |
| AKELGSGHYIIGVDED; | KELGSGHYIIGVDEDQ; | ELGSGHYIIGVDEDQA; |
| LGSGHYIIGVDEDQAY; | GSGHYIIGVDEDQAYL; | SGHYIIGVDEDQAYLA; |
| GHYIIGVDEDQAYLAP; | HYIIGVDEDQAYLAPD; | YIIGVDEDQAYLAPDN; |
| IIGVDEDQAYLAPDNV; | IGVDEDQAYLAPDNVI; | GVDEDQAYLAPDNVIT; |
| VDEDQAYLAPDNVITS; | DEDQAYLAPDNVITST; | EDQAYLAPDNVITSTT; |
| DQAYLAPDNVITSTTK; | QAYLAPDNVITSTTKD; | AYLAPDNVITSTTKDV; |
| YLAPDNVITSTTKDVG; | LAPDNVITSTTKDVGR; | APDNVITSTTKDVGRA; |
| PDNVITSTTKDVGRAL; | DNVITSTTKDVGRALN; | NVITSTTKDVGRALNI; |
| VITSTTKDVGRALNIF; | ITSTTKDVGRALNIFT; | TSTTKDVGRALNIFTS; |
| STTKDVGRALNIFTSN; | TTKDVGRALNIFTSNH; | TKDVGRALNIFTSNHL; |
| KDVGRALNIFTSNHLK; | DVGRALNIFTSNHLKT; | VGRALNIFTSNHLKTN; |

| | |
|---|---|
| | GRALNIFTSNHLKTNT; RALNIFTSNHLKTNTF; ALNIFTSNHLKTNTFE;<br>LNIFTSNHLKTNTFEG; NIFTSNHLKTNTFEGG; IFTSNHLKTNTFEGGK;<br>FTSNHLKTNTFEGGKL; TSNHLKTNTFEGGKLI; SNHLKTNTFEGGKLIN;<br>NHLKTNTFEGGKLINY; HLKTNTFEGGKLINYG; LKTNTFEGGKLINYGL;<br>KTNTFEGGKLINYGLK; TNTFEGGKLINYGLKE; NTFEGGKLINYGLKEG;<br>TFEGGKLINYGLKEGV; FEGGKLINYGLKEGVV; EGGKLINYGLKEGVVG;<br>GGKLINYGLKEGVVGF; GKLINYGLKEGVVGFV; KLINYGLKEGVVGFVR;<br>LINYGLKEGVVGFVRN; INYGLKEGVVGFVRNP; NYGLKEGVVGFVRNPK;<br>YGLKEGVVGFVRNPKM; GLKEGVVGFVRNPKMI; LKEGVVGFVRNPKMIS;<br>KEGVVGFVRNPKMISF; EGVVGFVRNPKMISFE; GVVGFVRNPKMISFEL;<br>VVGFVRNPKMISFELE; VGFVRNPKMISFELEK; GFVRNPKMISFELEKE;<br>FVRNPKMISFELEKEI; VRNPKMISFELEKEID; RNPKMISFELEKEIDN;<br>NPKMISFELEKEIDNL; PKMISFELEKEIDNLS; KMISFELEKEIDNLSS;<br>MISFELEKEIDNLSSK; ISFELEKEIDNLSSKI; SFELEKEIDNLSSKII;<br>FELEKEIDNLSSKIIN; ELEKEIDNLSSKIINK; LEKEIDNLSSKIINKE;<br>EKEIDNLSSKIINKEI; KEIDNLSSKIINKEII; EIDNLSSKIINKEIIV;<br>IDNLSSKIINKEIIVP; DNLSSKIINKEIIVPS; NLSSKIINKEIIVPSN;<br>LSSKIINKEIIVPSNK; SSKIINKEIIVPSNKE; SKIINKEIIVPSNKES;<br>KIINKEIIVPSNKESY; IINKEIIVPSNKESYE; INKEIIVPSNKESYEK;<br>NKEIIVPSNKESYEKF; KEIIVPSNKESYEKFL; EIIVPSNKESYEKFLK;<br>IIVPSNKESYEKFLKE; IVPSNKESYEKFLKEF; VPSNKESYEKFLKEFI; |
| NP_212516.1<br>Basic membrane<br>protein B (bmpB)<br>[Borrelia<br>burgdorferi B31 | 13 mers:<br>MRIVIFIFGILLT; RIVIFIFGILLTS; IVIFIFGILLTSC; VIFIFGILLTSCF;<br>IFIFGILLTSCFS; FIFGILLTSCFSR; IFGILLTSCFSRN; FGILLTSCFSRNG;<br>GILLTSCFSRNGI; ILLTSCFSRNGIE; LLTSCFSRNGIES; LTSCFSRNGIESS;<br>TSCFSRNGIESSS; SCFSRNGIESSSK; CFSRNGIESSSKK; FSRNGIESSSKKI;<br>SRNGIESSSKKIK; RNGIESSSKKIKI; NGIESSSKKIKIS; GIESSSKKIKISM;<br>IESSSKKIKISML; ESSSKKIKISMLV; SSSKKIKISMLVD; SSKKIKISMLVDG;<br>SKKIKISMLVDGV; KKIKISMLVDGVL; KIKISMLVDGVLD; IKISMLVDGVLDD;<br>KISMLVDGVLDDK; ISMLVDGVLDDKS; SMLVDGVLDDKSF; MLVDGVLDDKSFN;<br>LVDGVLDDKSFNS; VDGVLDDKSFNSS; DGVLDDKSFNSSA; GVLDDKSFNSSAN;<br>VLDDKSFNSSANE; LDDKSFNSSANEA; DDKSFNSSANEAL; DKSFNSSANEALL;<br>KSFNSSANEALLR; SFNSSANEALLRL; FNSSANEALLRLK; NSSANEALLRLKK;<br>SSANEALLRLKKD; SANEALLRLKKDF; ANEALLRLKKDFP; NEALLRLKKDFPE;<br>EALLRLKKDFPEN; ALLRLKKDFPENI; LLRLKKDFPENIE; LRLKKDFPENIEE;<br>RLKKDFPENIEEV; LKKDFPENIEEVF; KKDFPENIEEVFS; KDFPENIEEVFSC;<br>DFPENIEEVFSCA; FPENIEEVFSCAI; PENIEEVFSCAIS; ENIEEVFSCAISG;<br>NIEEVFSCAISGV; IEEVFSCAISGVY; EEVFSCAISGVYS; EVFSCAISGVYSS;<br>VFSCAISGVYSSY; FSCAISGVYSSYV; SCAISGVYSSYVS; CAISGVYSSYVSD;<br>AISGVYSSYVSDL; ISGVYSSYVSDLD; SGVYSSYVSDLDN; GVYSSYVSDLDNL;<br>VYSSYVSDLDNLK; YSSYVSDLDNLKR; SSYVSDLDNLKRN; SYVSDLDNLKRNG;<br>YVSDLDNLKRNGS; VSDLDNLKRNGSD; SDLDNLKRNGSDL; DLDNLKRNGSDLI;<br>LDNLKRNGSDLIW; DNLKRNGSDLIWL; NLKRNGSDLIWLV; LKRNGSDLIWLVG;<br>KRNGSDLIWLVGY; RNGSDLIWLVGYM; NGSDLIWLVGYML; GSDLIWLVGYMLT;<br>SDLIWLVGYMLTD; DLIWLVGYMLTDA; LIWLVGYMLTDAS; IWLVGYMLTDASL;<br>WLVGYMLTDASLL; LVGYMLTDASLLV; VGYMLTDASLLVS; GYMLTDASLLVSS;<br>YMLTDASLLVSSE; MLTDASLLVSSEN; LTDASLLVSSENP; TDASLLVSSENPK;<br>DASLLVSSENPKI; ASLLVSSENPKIS; SLLVSSENPKISY; LLVSSENPKISYG;<br>LVSSENPKISYGI; VSSENPKISYGII; SSENPKISYGIID; SENPKISYGIIDP;<br>ENPKISYGIIDPI; NPKISYGIIDPIY; PKISYGIIDPIYG; KISYGIIDPIYGD;<br>ISYGIIDPIYGDD; SYGIIDPIYGDDV; YGIIDPIYGDDVQ; GIIDPIYGDDVQI;<br>IIDPIYGDDVQIP; IDPIYGDDVQIPE; DPIYGDDVQIPEN; PIYGDDVQIPENL;<br>IYGDDVQIPENLI; YGDDVQIPENLIA; GDDVQIPENLIAV; DDVQIPENLIAVV;<br>DVQIPENLIAVVF; VQIPENLIAVVFR; QIPENLIAVVFRV; IPENLIAVVFRVE;<br>PENLIAVVFRVEQ; ENLIAVVFRVEQG; NLIAVVFRVEQGA; LIAVVFRVEQGAF; |

IAVVFRVEQGAFL; AVVFRVEQGAFLA; VVFRVEQGAFLAG; VFRVEQGAFLAGY;
FRVEQGAFLAGYI; RVEQGAFLAGYIA; VEQGAFLAGYIAA; EQGAFLAGYIAAK;
QGAFLAGYIAAKK; GAFLAGYIAAKKS; AFLAGYIAAKKSF; FLAGYIAAKKSFS;
LAGYIAAKKSFSG; AGYIAAKKSFSGK; GYIAAKKSFSGKI; YIAAKKSFSGKIG;
IAAKKSFSGKIGF; AAKKSFSGKIGFI; AKKSFSGKIGFIG; KKSFSGKIGFIGG;
KSFSGKIGFIGGM; SFSGKIGFIGGMK; FSGKIGFIGGMKG; SGKIGFIGGMKGN;
GKIGFIGGMKGNI; KIGFIGGMKGNIV; IGFIGGMKGNIVD; GFIGGMKGNIVDA;
FIGGMKGNIVDAF; IGGMKGNIVDAFR; GGMKGNIVDAFRY; GMKGNIVDAFRYG;
MKGNIVDAFRYGY; KGNIVDAFRYGYE; GNIVDAFRYGYES; NIVDAFRYGYESG;
IVDAFRYGYESGA; VDAFRYGYESGAK; DAFRYGYESGAKY; AFRYGYESGAKYA;
FRYGYESGAKYAN; RYGYESGAKYANK; YGYESGAKYANKD; GYESGAKYANKDI;
YESGAKYANKDIE; ESGAKYANKDIEI; SGAKYANKDIEII; GAKYANKDIEIIS;
AKYANKDIEIISE; KYANKDIEIISEY; YANKDIEIISEYS; ANKDIEIISEYSN;
NKDIEIISEYSNS; KDIEIISEYSNSF; DIEIISEYSNSFS; IEIISEYSNSFSD;
EIISEYSNSFSDV; IISEYSNSFSDVD; ISEYSNSFSDVDI; SEYSNSFSDVDIG;
EYSNSFSDVDIGR; YSNSFSDVDIGRT; SNSFSDVDIGRTI; NSFSDVDIGRTIA;
SFSDVDIGRTIAS; FSDVDIGRTIASK; SDVDIGRTIASKM; DVDIGRTIASKMY;
VDIGRTIASKMYS; DIGRTIASKMYSK; IGRTIASKMYSKG; GRTIASKMYSKGI;
RTIASKMYSKGID; TIASKMYSKGIDV; IASKMYSKGIDVI; ASKMYSKGIDVIH;
SKMYSKGIDVIHF; KMYSKGIDVIHFA; MYSKGIDVIHFAA; YSKGIDVIHFAAG;
SKGIDVIHFAAGL; KGIDVIHFAAGLA; GIDVIHFAAGLAG; IDVIHFAAGLAGI;
DVIHFAAGLAGIG; VIHFAAGLAGIGV; IHFAAGLAGIGVI; HFAAGLAGIGVIE;
FAAGLAGIGVIET; AAGLAGIGVIETA; AGLAGIGVIETAK; GLAGIGVIETAKN;
LAGIGVIETAKNL; AGIGVIETAKNLG; GIGVIETAKNLGD; IGVIETAKNLGDG;
GVIETAKNLGDGY; VIETAKNLGDGYY; IETAKNLGDGYYV; ETAKNLGDGYYVI;
TAKNLGDGYYVIG; AKNLGDGYYVIGA; KNLGDGYYVIGAD; NLGDGYYVIGADQ;
LGDGYYVIGADQD; GDGYYVIGADQDQ; DGYYVIGADQDQS; GYYVIGADQDQSY;
YYVIGADQDQSYL; YVIGADQDQSYLA; VIGADQDQSYLAP; IGADQDQSYLAPK;
GADQDQSYLAPKN; ADQDQSYLAPKNF; DQDQSYLAPKNFI; QDQSYLAPKNFIT;
DQSYLAPKNFITS; QSYLAPKNFITSV; SYLAPKNFITSVI; YLAPKNFITSVIK;
LAPKNFITSVIKN; APKNFITSVIKNI; PKNFITSVIKNIG; KNFITSVIKNIGD;
NFITSVIKNIGDA; FITSVIKNIGDAL; ITSVIKNIGDALY; TSVIKNIGDALYL;
SVIKNIGDALYLI; VIKNIGDALYLIT; IKNIGDALYLITG; KNIGDALYLITGE;
NIGDALYLITGEY; IGDALYLITGEYI; GDALYLITGEYIK; DALYLITGEYIKN;
ALYLITGEYIKNN; LYLITGEYIKNNN; YLITGEYIKNNNV; LITGEYIKNNNVW;
ITGEYIKNNNVWE; TGEYIKNNNVWEG; GEYIKNNNVWEGG; EYIKNNNVWEGGK;
YIKNNNVWEGGKV; IKNNNVWEGGKVV; KNNNVWEGGKVVQ; NNNVWEGGKVVQM;
NNVWEGGKVVQMG; NVWEGGKVVQMGL; VWEGGKVVQMGLR; WEGGKVVQMGLRD;
EGGKVVQMGLRDG; GGKVVQMGLRDGV; GKVVQMGLRDGVI; KVVQMGLRDGVIG;
VVQMGLRDGVIGL; VQMGLRDGVIGLP; QMGLRDGVIGLPN; MGLRDGVIGLPNA;
GLRDGVIGLPNAN; LRDGVIGLPNANE; RDGVIGLPNANEF; DGVIGLPNANEFE;
GVIGLPNANEFEY; VIGLPNANEFEYI; IGLPNANEFEYIK; GLPNANEFEYIKV;
LPNANEFEYIKVL; PNANEFEYIKVLE; NANEFEYIKVLER; ANEFEYIKVLERK;
NEFEYIKVLERKI; EFEYIKVLERKII; FEYIKVLERKIIN; EYIKVLERKIINK;
YIKVLERKIINKE; IKVLERKIINKEI; KVLERKIINKEII; VLERKIINKEIIV;
LERKIINKEIIVP; ERKIINKEIIVPC; RKIINKEIIVPCN; KIINKEIIVPCNQ;
IINKEIIVPCNQE; INKEIIVPCNQEE; NKEIIVPCNQEEY; KEIIVPCNQEEYE;
EIIVPCNQEEYEI; IIVPCNQEEYEIF; IVPCNQEEYEIFI; VPCNQEEYEIFIK;
PCNQEEYEIFIKQ; CNQEEYEIFIKQI; NQEEYEIFIKQIL; QEEYEIFIKQILK;
EEYEIFIKQILKL;

14 mers:
MRIVIFIFGILLTS; RIVIFIFGILLTSC; IVIFIFGILLTSCF;
VIFIFGILLTSCFS; IFIFGILLTSCFSR; FIFGILLTSCFSRN;
IFGILLTSCFSRNG; FGILLTSCFSRNGI; GILLTSCFSRNGIE;
ILLTSCFSRNGIES; LLTSCFSRNGIESS; LTSCFSRNGIESSS;
TSCFSRNGIESSSK; SCFSRNGIESSSKK; CFSRNGIESSSKKI;

FSRNGIESSSKKIK; SRNGIESSSKKIKI; RNGIESSSKKIKIS;
NGIESSSKKIKISM; GIESSSKKIKISML; IESSSKKIKISMLV;
ESSSKKIKISMLVD; SSSKKIKISMLVDG; SSKKIKISMLVDGV;
SKKIKISMLVDGVL; KKIKISMLVDGVLD; KIKISMLVDGVLDD;
IKISMLVDGVLDDK; KISMLVDGVLDDKS; ISMLVDGVLDDKSF;
SMLVDGVLDDKSFN; MLVDGVLDDKSFNS; LVDGVLDDKSFNSS;
VDGVLDDKSFNSSA; DGVLDDKSFNSSAN; GVLDDKSFNSSANE;
VLDDKSFNSSANEA; LDDKSFNSSANEAL; DDKSFNSSANEALL;
DKSFNSSANEALLR; KSFNSSANEALLRL; SFNSSANEALLRLK;
FNSSANEALLRLKK; NSSANEALLRLKKD; SSANEALLRLKKDF;
SANEALLRLKKDFP; ANEALLRLKKDFPE; NEALLRLKKDFPEN;
EALLRLKKDFPENI; ALLRLKKDFPENIE; LLRLKKDFPENIEE;
LRLKKDFPENIEEV; RLKKDFPENIEEVF; LKKDFPENIEEVFS;
KKDFPENIEEVFSC; KDFPENIEEVFSCA; DFPENIEEVFSCAI;
FPENIEEVFSCAIS; PENIEEVFSCAISG; ENIEEVFSCAISGV;
NIEEVFSCAISGVY; IEEVFSCAISGVYS; EEVFSCAISGVYSS;
EVFSCAISGVYSSY; VFSCAISGVYSSYV; FSCAISGVYSSYVS;
SCAISGVYSSYVSD; CAISGVYSSYVSDL; AISGVYSSYVSDLD;
ISGVYSSYVSDLDN; SGVYSSYVSDLDNL; GVYSSYVSDLDNLK;
VYSSYVSDLDNLKR; YSSYVSDLDNLKRN; SSYVSDLDNLKRNG;
SYVSDLDNLKRNGS; YVSDLDNLKRNGSD; VSDLDNLKRNGSDL;
SDLDNLKRNGSDLI; DLDNLKRNGSDLIW; LDNLKRNGSDLIWL;
DNLKRNGSDLIWLV; NLKRNGSDLIWLVG; LKRNGSDLIWLVGY;
KRNGSDLIWLVGYM; RNGSDLIWLVGYML; NGSDLIWLVGYMLT;
GSDLIWLVGYMLTD; SDLIWLVGYMLTDA; DLIWLVGYMLTDAS;
LIWLVGYMLTDASL; IWLVGYMLTDASLL; WLVGYMLTDASLLV;
LVGYMLTDASLLVS; VGYMLTDASLLVSS; GYMLTDASLLVSSE;
YMLTDASLLVSSEN; MLTDASLLVSSENP; LTDASLLVSSENPK;
TDASLLVSSENPKI; DASLLVSSENPKIS; ASLLVSSENPKISY;
SLLVSSENPKISYG; LLVSSENPKISYGI; LVSSENPKISYGII;
VSSENPKISYGIID; SSENPKISYGIIDP; SENPKISYGIIDPI;
ENPKISYGIIDPIY; NPKISYGIIDPIYG; PKISYGIIDPIYGD;
KISYGIIDPIYGDD; ISYGIIDPIYGDDV; SYGIIDPIYGDDVQ;
YGIIDPIYGDDVQI; GIIDPIYGDDVQIP; IIDPIYGDDVQIPE;
IDPIYGDDVQIPEN; DPIYGDDVQIPENL; PIYGDDVQIPENLI;
IYGDDVQIPENLIA; YGDDVQIPENLIAV; GDDVQIPENLIAVV;
DDVQIPENLIAVVF; DVQIPENLIAVVFR; VQIPENLIAVVFRV;
QIPENLIAVVFRVE; IPENLIAVVFRVEQ; PENLIAVVFRVEQG;
ENLIAVVFRVEQGA; NLIAVVFRVEQGAF; LIAVVFRVEQGAFL;
IAVVFRVEQGAFLA; AVVFRVEQGAFLAG; VVFRVEQGAFLAGY;
VFRVEQGAFLAGYI; FRVEQGAFLAGYIA; RVEQGAFLAGYIAA;
VEQGAFLAGYIAAK; EQGAFLAGYIAAKK; QGAFLAGYIAAKKS;
GAFLAGYIAAKKSF; AFLAGYIAAKKSFS; FLAGYIAAKKSFSG;
LAGYIAAKKSFSGK; AGYIAAKKSFSGKI; GYIAAKKSFSGKIG;
YIAAKKSFSGKIGF; IAAKKSFSGKIGFI; AAKKSFSGKIGFIG;
AKKSFSGKIGFIGG; KKSFSGKIGFIGGM; KSFSGKIGFIGGMK;
SFSGKIGFIGGMKG; FSGKIGFIGGMKGN; SGKIGFIGGMKGNI;
GKIGFIGGMKGNIV; KIGFIGGMKGNIVD; IGFIGGMKGNIVDA;
GFIGGMKGNIVDAF; FIGGMKGNIVDAFR; IGGMKGNIVDAFRY;
GGMKGNIVDAFRYG; GMKGNIVDAFRYGY; MKGNIVDAFRYGYE;
KGNIVDAFRYGYES; GNIVDAFRYGYESG; NIVDAFRYGYESGA;
IVDAFRYGYESGAK; VDAFRYGYESGAKY; DAFRYGYESGAKYA;
AFRYGYESGAKYAN; FRYGYESGAKYANK; RYGYESGAKYANKD;
YGYESGAKYANKDI; GYESGAKYANKDIE; YESGAKYANKDIEI;
ESGAKYANKDIEII; SGAKYANKDIEIIS; GAKYANKDIEIISE;
AKYANKDIEIISEY; KYANKDIEIISEYS; YANKDIEIISEYSN;
ANKDIEIISEYSNS; NKDIEIISEYSNSF; KDIEIISEYSNSFS;

EP 2 254 592 B1

| | |
|---|---|
| | DIEIISEYSNSFSD; IEIISEYSNSFSDV; EIISEYSNSFSDVD; IISEYSNSFSDVDI; ISEYSNSFSDVDIG; SEYSNSFSDVDIGR; EYSNSFSDVDIGRT; YSNSFSDVDIGRTI; SNSFSDVDIGRTIA; NSFSDVDIGRTIAS; SFSDVDIGRTIASK; FSDVDIGRTIASKM; SDVDIGRTIASKMY; DVDIGRTIASKMYS; VDIGRTIASKMYSK; DIGRTIASKMYSKG; IGRTIASKMYSKGI; GRTIASKMYSKGID; RTIASKMYSKGIDV; TIASKMYSKGIDVI; IASKMYSKGIDVIH; ASKMYSKGIDVIHF; SKMYSKGIDVIHFA; KMYSKGIDVIHFAA; MYSKGIDVIHFAAG; YSKGIDVIHFAAGL; SKGIDVIHFAAGLA; KGIDVIHFAAGLAG; GIDVIHFAAGLAGI; IDVIHFAAGLAGIG; DVIHFAAGLAGIGV; VIHFAAGLAGIGVI; IHFAAGLAGIGVIE; HFAAGLAGIGVIET; FAAGLAGIGVIETA; AAGLAGIGVIETAK; AGLAGIGVIETAKN; GLAGIGVIETAKNL; LAGIGVIETAKNLG; AGIGVIETAKNLGD; GIGVIETAKNLGDG; IGVIETAKNLGDGY; GVIETAKNLGDGYY; VIETAKNLGDGYYV; IETAKNLGDGYYVI; ETAKNLGDGYYVIG; TAKNLGDGYYVIGA; AKNLGDGYYVIGAD; KNLGDGYYVIGADQ; NLGDGYYVIGADQD; LGDGYYVIGADQDQ; GDGYYVIGADQDQS; DGYYVIGADQDQSY; GYYVIGADQDQSYL; YYVIGADQDQSYLA; YVIGADQDQSYLAP; VIGADQDQSYLAPK; IGADQDQSYLAPKN; GADQDQSYLAPKNF; ADQDQSYLAPKNFI; DQDQSYLAPKNFIT; QDQSYLAPKNFITS; DQSYLAPKNFITSV; QSYLAPKNFITSVI; SYLAPKNFITSVIK; YLAPKNFITSVIKN; LAPKNFITSVIKNI; APKNFITSVIKNIG; PKNFITSVIKNIGD; KNFITSVIKNIGDA; NFITSVIKNIGDAL; FITSVIKNIGDALY; ITSVIKNIGDALYL; TSVIKNIGDALYLI; SVIKNIGDALYLIT; VIKNIGDALYLITG; IKNIGDALYLITGE; KNIGDALYLITGEY; NIGDALYLITGEYI; IGDALYLITGEYIK; GDALYLITGEYIKN; DALYLITGEYIKNN; ALYLITGEYIKNNN; LYLITGEYIKNNNV; YLITGEYIKNNNVW; LITGEYIKNNNVWE; ITGEYIKNNNVWEG; TGEYIKNNNVWEGG; GEYIKNNNVWEGGK; EYIKNNNVWEGGKV; YIKNNNVWEGGKVV; IKNNNVWEGGKVVQ; KNNNVWEGGKVVQM; NNNVWEGGKVVQMG; NNVWEGGKVVQMGL; NVWEGGKVVQMGLR; VWEGGKVVQMGLRD; WEGGKVVQMGLRDG; EGGKVVQMGLRDGV; GGKVVQMGLRDGVI; GKVVQMGLRDGVIG; KVVQMGLRDGVIGL; VVQMGLRDGVIGLP; VQMGLRDGVIGLPN; QMGLRDGVIGLPNA; MGLRDGVIGLPNAN; GLRDGVIGLPNANE; LRDGVIGLPNANEF; RDGVIGLPNANEFE; DGVIGLPNANEFEY; GVIGLPNANEFEYI; VIGLPNANEFEYIK; IGLPNANEFEYIKV; GLPNANEFEYIKVL; LPNANEFEYIKVLE; PNANEFEYIKVLER; NANEFEYIKVLERK; ANEFEYIKVLERKI; NEFEYIKVLERKII; EFEYIKVLERKIIN; FEYIKVLERKIINK; EYIKVLERKIINKE; YIKVLERKIINKEI; IKVLERKIINKEII; KVLERKIINKEIIV; VLERKIINKEIIVP; LERKIINKEIIVPC; ERKIINKEIIVPCN; RKIINKEIIVPCNQ; KIINKEIIVPCNQE; IINKEIIVPCNQEE; INKEIIVPCNQEEY; NKEIIVPCNQEEYE; KEIIVPCNQEEYEI; EIIVPCNQEEYEIF; IIVPCNQEEYEIFI; IVPCNQEEYEIFIK; VPCNQEEYEIFIKQ; PCNQEEYEIFIKQI; CNQEEYEIFIKQIL; NQEEYEIFIKQILK; QEEYEIFIKQILKL;<br><br>15 mers:<br>MRIVIFIFGILLTSC; RIVIFIFGILLTSCF; IVIFIFGILLTSCFS; VIFIFGILLTSCFSR; IFIFGILLTSCFSRN; FIFGILLTSCFSRNG; IFGILLTSCFSRNGI; FGILLTSCFSRNGIE; GILLTSCFSRNGIES; ILLTSCFSRNGIESS; LLTSCFSRNGIESSS; LTSCFSRNGIESSSK; TSCFSRNGIESSSKK; SCFSRNGIESSSKKI; CFSRNGIESSSKKIK; FSRNGIESSSKKIKI; SRNGIESSSKKIKIS; RNGIESSSKKIKISM; NGIESSSKKIKISML; GIESSSKKIKISMLV; IESSSKKIKISMLVD; |

914

| | |
|---|---|
| ESSSKKIKISMLVDG; SSSKKIKISMLVDGV; SSKKIKISMLVDGVL; | |
| SKKIKISMLVDGVLD; KKIKISMLVDGVLDD; KIKISMLVDGVLDDK; | |
| IKISMLVDGVLDDKS; KISMLVDGVLDDKSF; ISMLVDGVLDDKSFN; | |
| SMLVDGVLDDKSFNS; MLVDGVLDDKSFNSS; LVDGVLDDKSFNSSA; | |
| VDGVLDDKSFNSSAN; DGVLDDKSFNSSANE; GVLDDKSFNSSANEA; | |
| VLDDKSFNSSANEAL; LDDKSFNSSANEALL; DDKSFNSSANEALLR; | |
| DKSFNSSANEALLRL; KSFNSSANEALLRLK; SFNSSANEALLRLKK; | |
| FNSSANEALLRLKKD; NSSANEALLRLKKDF; SSANEALLRLKKDFP; | |
| SANEALLRLKKDFPE; ANEALLRLKKDFPEN; NEALLRLKKDFPENI; | |
| EALLRLKKDFPENIE; ALLRLKKDFPENIEE; LLRLKKDFPENIEEV; | |
| LRLKKDFPENIEEVF; RLKKDFPENIEEVFS; LKKDFPENIEEVFSC; | |
| KKDFPENIEEVFSCA; KDFPENIEEVFSCAI; DFPENIEEVFSCAIS; | |
| FPENIEEVFSCAISG; PENIEEVFSCAISGV; ENIEEVFSCAISGVY; | |
| NIEEVFSCAISGVYS; IEEVFSCAISGVYSS; EEVFSCAISGVYSSY; | |
| EVFSCAISGVYSSYV; VFSCAISGVYSSYVS; FSCAISGVYSSYVSD; | |
| SCAISGVYSSYVSDL; CAISGVYSSYVSDLD; AISGVYSSYVSDLDN; | |
| ISGVYSSYVSDLDNL; SGVYSSYVSDLDNLK; GVYSSYVSDLDNLKR; | |
| VYSSYVSDLDNLKRN; YSSYVSDLDNLKRNG; SSYVSDLDNLKRNGS; | |
| SYVSDLDNLKRNGSD; YVSDLDNLKRNGSDL; VSDLDNLKRNGSDLI; | |
| SDLDNLKRNGSDLIW; DLDNLKRNGSDLIWL; LDNLKRNGSDLIWLV; | |
| DNLKRNGSDLIWLVG; NLKRNGSDLIWLVGY; LKRNGSDLIWLVGYM; | |
| KRNGSDLIWLVGYML; RNGSDLIWLVGYMLT; NGSDLIWLVGYMLTD; | |
| GSDLIWLVGYMLTDA; SDLIWLVGYMLTDAS; DLIWLVGYMLTDASL; | |
| LIWLVGYMLTDASLL; IWLVGYMLTDASLLV; WLVGYMLTDASLLVS; | |
| LVGYMLTDASLLVSS; VGYMLTDASLLVSSE; GYMLTDASLLVSSEN; | |
| YMLTDASLLVSSENP; MLTDASLLVSSENPK; LTDASLLVSSENPKI; | |
| TDASLLVSSENPKIS; DASLLVSSENPKISY; ASLLVSSENPKISYG; | |
| SLLVSSENPKISYGI; LLVSSENPKISYGII; LVSSENPKISYGIID; | |
| VSSENPKISYGIIDP; SSENPKISYGIIDPI; SENPKISYGIIDPIY; | |
| ENPKISYGIIDPIYG; NPKISYGIIDPIYGD; PKISYGIIDPIYGDD; | |
| KISYGIIDPIYGDDV; ISYGIIDPIYGDDVQ; SYGIIDPIYGDDVQI; | |
| YGIIDPIYGDDVQIP; GIIDPIYGDDVQIPE; IIDPIYGDDVQIPEN; | |
| IDPIYGDDVQIPENL; DPIYGDDVQIPENLI; PIYGDDVQIPENLIA; | |
| IYGDDVQIPENLIAV; YGDDVQIPENLIAVV; GDDVQIPENLIAVVF; | |
| DDVQIPENLIAVVFR; DVQIPENLIAVVFRV; VQIPENLIAVVFRVE; | |
| QIPENLIAVVFRVEQ; IPENLIAVVFRVEQG; PENLIAVVFRVEQGA; | |
| ENLIAVVFRVEQGAF; NLIAVVFRVEQGAFL; LIAVVFRVEQGAFLA; | |
| IAVVFRVEQGAFLAG; AVVFRVEQGAFLAGY; VVFRVEQGAFLAGYI; | |
| VFRVEQGAFLAGYIA; FRVEQGAFLAGYIAA; RVEQGAFLAGYIAAK; | |
| VEQGAFLAGYIAAKK; EQGAFLAGYIAAKKS; QGAFLAGYIAAKKSF; | |
| GAFLAGYIAAKKSFS; AFLAGYIAAKKSFSG; FLAGYIAAKKSFSGK; | |
| LAGYIAAKKSFSGKI; AGYIAAKKSFSGKIG; GYIAAKKSFSGKIGF; | |
| YIAAKKSFSGKIGFI; IAAKKSFSGKIGFIG; AAKKSFSGKIGFIGG; | |
| AKKSFSGKIGFIGGM; KKSFSGKIGFIGGMK; KSFSGKIGFIGGMKG; | |
| SFSGKIGFIGGMKGN; FSGKIGFIGGMKGNI; SGKIGFIGGMKGNIV; | |
| GKIGFIGGMKGNIVD; KIGFIGGMKGNIVDA; IGFIGGMKGNIVDAF; | |
| GFIGGMKGNIVDAFR; FIGGMKGNIVDAFRY; IGGMKGNIVDAFRYG; | |
| GGMKGNIVDAFRYGY; GMKGNIVDAFRYGYE; MKGNIVDAFRYGYES; | |
| KGNIVDAFRYGYESG; GNIVDAFRYGYESGA; NIVDAFRYGYESGAK; | |
| IVDAFRYGYESGAKY; VDAFRYGYESGAKYA; DAFRYGYESGAKYAN; | |
| AFRYGYESGAKYANK; FRYGYESGAKYANKD; RYGYESGAKYANKDI; | |
| YGYESGAKYANKDIE; GYESGAKYANKDIEI; YESGAKYANKDIEII; | |
| ESGAKYANKDIEIIS; SGAKYANKDIEIISE; GAKYANKDIEIISEY; | |
| AKYANKDIEIISEYS; KYANKDIEIISEYSN; YANKDIEIISEYSNS; | |
| ANKDIEIISEYSNSF; NKDIEIISEYSNSFS; KDIEIISEYSNSFSD; | |
| DIEIISEYSNSFSDV; IEIISEYSNSFSDVD; EIISEYSNSFSDVDI; | |
| IISEYSNSFSDVDIG; ISEYSNSFSDVDIGR; SEYSNSFSDVDIGRT; | |

EYSNSFSDVDIGRTI; YSNSFSDVDIGRTIA; SNSFSDVDIGRTIAS;
NSFSDVDIGRTIASK; SFSDVDIGRTIASKM; FSDVDIGRTIASKMY;
SDVDIGRTIASKMYS; DVDIGRTIASKMYSK; VDIGRTIASKMYSKG;
DIGRTIASKMYSKGI; IGRTIASKMYSKGID; GRTIASKMYSKGIDV;
RTIASKMYSKGIDVI; TIASKMYSKGIDVIH; IASKMYSKGIDVIHF;
ASKMYSKGIDVIHFA; SKMYSKGIDVIHFAA; KMYSKGIDVIHFAAG;
MYSKGIDVIHFAAGL; YSKGIDVIHFAAGLA; SKGIDVIHFAAGLAG;
KGIDVIHFAAGLAGI; GIDVIHFAAGLAGIG; IDVIHFAAGLAGIGV;
DVIHFAAGLAGIGVI; VIHFAAGLAGIGVIE; IHFAAGLAGIGVIET;
HFAAGLAGIGVIETA; FAAGLAGIGVIETAK; AAGLAGIGVIETAKN;
AGLAGIGVIETAKNL; GLAGIGVIETAKNLG; LAGIGVIETAKNLGD;
AGIGVIETAKNLGDG; GIGVIETAKNLGDGY; IGVIETAKNLGDGYY;
GVIETAKNLGDGYYV; VIETAKNLGDGYYVI; IETAKNLGDGYYVIG;
ETAKNLGDGYYVIGA; TAKNLGDGYYVIGAD; AKNLGDGYYVIGADQ;
KNLGDGYYVIGADQD; NLGDGYYVIGADQDQ; LGDGYYVIGADQDQS;
GDGYYVIGADQDQSY; DGYYVIGADQDQSYL; GYYVIGADQDQSYLA;
YYVIGADQDQSYLAP; YVIGADQDQSYLAPK; VIGADQDQSYLAPKN;
IGADQDQSYLAPKNF; GADQDQSYLAPKNFI; ADQDQSYLAPKNFIT;
DQDQSYLAPKNFITS; QDQSYLAPKNFITSV; DQSYLAPKNFITSVI;
QSYLAPKNFITSVIK; SYLAPKNFITSVIKN; YLAPKNFITSVIKNI;
LAPKNFITSVIKNIG; APKNFITSVIKNIGD; PKNFITSVIKNIGDA;
KNFITSVIKNIGDAL; NFITSVIKNIGDALY; FITSVIKNIGDALYL;
ITSVIKNIGDALYLI; TSVIKNIGDALYLIT; SVIKNIGDALYLITG;
VIKNIGDALYLITGE; IKNIGDALYLITGEY; KNIGDALYLITGEYI;
NIGDALYLITGEYIK; IGDALYLITGEYIKN; GDALYLITGEYIKNN;
DALYLITGEYIKNNN; ALYLITGEYIKNNNV; LYLITGEYIKNNNVW;
YLITGEYIKNNNVWE; LITGEYIKNNNVWEG; ITGEYIKNNNVWEGG;
TGEYIKNNNVWEGGK; GEYIKNNNVWEGGKV; EYIKNNNVWEGGKVV;
YIKNNNVWEGGKVVQ; IKNNNVWEGGKVVQM; KNNNVWEGGKVVQMG;
NNNVWEGGKVVQMGL; NNVWEGGKVVQMGLR; NVWEGGKVVQMGLRD;
VWEGGKVVQMGLRDG; WEGGKVVQMGLRDGV; EGGKVVQMGLRDGVI;
GGKVVQMGLRDGVIG; GKVVQMGLRDGVIGL; KVVQMGLRDGVIGLP;
VVQMGLRDGVIGLPN; VQMGLRDGVIGLPNA; QMGLRDGVIGLPNAN;
MGLRDGVIGLPNANE; GLRDGVIGLPNANEF; LRDGVIGLPNANEFE;
RDGVIGLPNANEFEY; DGVIGLPNANEFEYI; GVIGLPNANEFEYIK;
VIGLPNANEFEYIKV; IGLPNANEFEYIKVL; GLPNANEFEYIKVLE;
LPNANEFEYIKVLER; PNANEFEYIKVLERK; NANEFEYIKVLERKI;
ANEFEYIKVLERKII; NEFEYIKVLERKIIN; EFEYIKVLERKIINK;
FEYIKVLERKIINKE; EYIKVLERKIINKEI; YIKVLERKIINKEII;
IKVLERKIINKEIIV; KVLERKIINKEIIVP; VLERKIINKEIIVPC;
LERKIINKEIIVPCN; ERKIINKEIIVPCNQ; RKIINKEIIVPCNQE;
KIINKEIIVPCNQEE; IINKEIIVPCNQEEY; INKEIIVPCNQEEYE;
NKEIIVPCNQEEYEI; KEIIVPCNQEEYEIF; EIIVPCNQEEYEIFI;
IIVPCNQEEYEIFIK; IVPCNQEEYEIFIKQ; VPCNQEEYEIFIKQI;
PCNQEEYEIFIKQIL; CNQEEYEIFIKQILK; NQEEYEIFIKQILKL;

16 mers:
MRIVIFIFGILLTSCF; RIVIFIFGILLTSCFS; IVIFIFGILLTSCFSR;
VIFIFGILLTSCFSRN; IFIFGILLTSCFSRNG; FIFGILLTSCFSRNGI;
IFGILLTSCFSRNGIE; FGILLTSCFSRNGIES; GILLTSCFSRNGIESS;
ILLTSCFSRNGIESSS; LLTSCFSRNGIESSSK; LTSCFSRNGIESSSKK;
TSCFSRNGIESSSKKI; SCFSRNGIESSSKKIK; CFSRNGIESSSKKIKI;
FSRNGIESSSKKIKIS; SRNGIESSSKKIKISM; RNGIESSSKKIKISML;
NGIESSSKKIKISMLV; GIESSSKKIKISMLVD; IESSSKKIKISMLVDG;
ESSSKKIKISMLVDGV; SSSKKIKISMLVDGVL; SSKKIKISMLVDGVLD;
SKKIKISMLVDGVLDD; KKIKISMLVDGVLDDK; KIKISMLVDGVLDDKS;
IKISMLVDGVLDDKSF; KISMLVDGVLDDKSFN; ISMLVDGVLDDKSFNS;

| | | |
|---|---|---|
| SMLVDGVLDDKSFNSS; | MLVDGVLDDKSFNSSA; | LVDGVLDDKSFNSSAN; |
| VDGVLDDKSFNSSANE; | DGVLDDKSFNSSANEA; | GVLDDKSFNSSANEAL; |
| VLDDKSFNSSANEALL; | LDDKSFNSSANEALLR; | DDKSFNSSANEALLRL; |
| DKSFNSSANEALLRLK; | KSFNSSANEALLRLKK; | SFNSSANEALLRLKKD; |
| FNSSANEALLRLKKDF; | NSSANEALLRLKKDFP; | SSANEALLRLKKDFPE; |
| SANEALLRLKKDFPEN; | ANEALLRLKKDFPENI; | NEALLRLKKDFPENIE; |
| EALLRLKKDFPENIEE; | ALLRLKKDFPENIEEV; | LLRLKKDFPENIEEVF; |
| LRLKKDFPENIEEVFS; | RLKKDFPENIEEVFSC; | LKKDFPENIEEVFSCA; |
| KKDFPENIEEVFSCAI; | KDFPENIEEVFSCAIS; | DFPENIEEVFSCAISG; |
| FPENIEEVFSCAISGV; | PENIEEVFSCAISGVY; | ENIEEVFSCAISGVYS; |
| NIEEVFSCAISGVYSS; | IEEVFSCAISGVYSSY; | EEVFSCAISGVYSSYV; |
| EVFSCAISGVYSSYVS; | VFSCAISGVYSSYVSD; | FSCAISGVYSSYVSDL; |
| SCAISGVYSSYVSDLD; | CAISGVYSSYVSDLDN; | AISGVYSSYVSDLDNL; |
| ISGVYSSYVSDLDNLK; | SGVYSSYVSDLDNLKR; | GVYSSYVSDLDNLKRN; |
| VYSSYVSDLDNLKRNG; | YSSYVSDLDNLKRNGS; | SSYVSDLDNLKRNGSD; |
| SYVSDLDNLKRNGSDL; | YVSDLDNLKRNGSDLI; | VSDLDNLKRNGSDLIW; |
| SDLDNLKRNGSDLIWL; | DLDNLKRNGSDLIWLV; | LDNLKRNGSDLIWLVG; |
| DNLKRNGSDLIWLVGY; | NLKRNGSDLIWLVGYM; | LKRNGSDLIWLVGYML; |
| KRNGSDLIWLVGYMLT; | RNGSDLIWLVGYMLTD; | NGSDLIWLVGYMLTDA; |
| GSDLIWLVGYMLTDAS; | SDLIWLVGYMLTDASL; | DLIWLVGYMLTDASLL; |
| LIWLVGYMLTDASLLV; | IWLVGYMLTDASLLVS; | WLVGYMLTDASLLVSS; |
| LVGYMLTDASLLVSSE; | VGYMLTDASLLVSSEN; | GYMLTDASLLVSSENP; |
| YMLTDASLLVSSENPK; | MLTDASLLVSSENPKI; | LTDASLLVSSENPKIS; |
| TDASLLVSSENPKISY; | DASLLVSSENPKISYG; | ASLLVSSENPKISYGI; |
| SLLVSSENPKISYGII; | LLVSSENPKISYGIID; | LVSSENPKISYGIIDP; |
| VSSENPKISYGIIDPI; | SSENPKISYGIIDPIY; | SENPKISYGIIDPIYG; |
| ENPKISYGIIDPIYGD; | NPKISYGIIDPIYGDD; | PKISYGIIDPIYGDDV; |
| KISYGIIDPIYGDDVQ; | ISYGIIDPIYGDDVQI; | SYGIIDPIYGDDVQIP; |
| YGIIDPIYGDDVQIPE; | GIIDPIYGDDVQIPEN; | IIDPIYGDDVQIPENL; |
| IDPIYGDDVQIPENLI; | DPIYGDDVQIPENLIA; | PIYGDDVQIPENLIAV; |
| IYGDDVQIPENLIAVV; | YGDDVQIPENLIAVVF; | GDDVQIPENLIAVVFR; |
| DDVQIPENLIAVVFRV; | DVQIPENLIAVVFRVE; | VQIPENLIAVVFRVEQ; |
| QIPENLIAVVFRVEQG; | IPENLIAVVFRVEQGA; | PENLIAVVFRVEQGAF; |
| ENLIAVVFRVEQGAFL; | NLIAVVFRVEQGAFLA; | LIAVVFRVEQGAFLAG; |
| IAVVFRVEQGAFLAGY; | AVVFRVEQGAFLAGYI; | VVFRVEQGAFLAGYIA; |
| VFRVEQGAFLAGYIAA; | FRVEQGAFLAGYIAAK; | RVEQGAFLAGYIAAKK; |
| VEQGAFLAGYIAAKKS; | EQGAFLAGYIAAKKSF; | QGAFLAGYIAAKKSFS; |
| GAFLAGYIAAKKSFSG; | AFLAGYIAAKKSFSGK; | FLAGYIAAKKSFSGKI; |
| LAGYIAAKKSFSGKIG; | AGYIAAKKSFSGKIGF; | GYIAAKKSFSGKIGFI; |
| YIAAKKSFSGKIGFIG; | IAAKKSFSGKIGFIGG; | AAKKSFSGKIGFIGGM; |
| AKKSFSGKIGFIGGMK; | KKSFSGKIGFIGGMKG; | KSFSGKIGFIGGMKGN; |
| SFSGKIGFIGGMKGNI; | FSGKIGFIGGMKGNIV; | SGKIGFIGGMKGNIVD; |
| GKIGFIGGMKGNIVDA; | KIGFIGGMKGNIVDAF; | IGFIGGMKGNIVDAFR; |
| GFIGGMKGNIVDAFRY; | FIGGMKGNIVDAFRYG; | IGGMKGNIVDAFRYGY; |
| GGMKGNIVDAFRYGYE; | GMKGNIVDAFRYGYES; | MKGNIVDAFRYGYESG; |
| KGNIVDAFRYGYESGA; | GNIVDAFRYGYESGAK; | NIVDAFRYGYESGAKY; |
| IVDAFRYGYESGAKYA; | VDAFRYGYESGAKYAN; | DAFRYGYESGAKYANK; |
| AFRYGYESGAKYANKD; | FRYGYESGAKYANKDI; | RYGYESGAKYANKDIE; |
| YGYESGAKYANKDIEI; | GYESGAKYANKDIEII; | YESGAKYANKDIEIIS; |
| ESGAKYANKDIEIISE; | SGAKYANKDIEIISEY; | GAKYANKDIEIISEYS; |
| AKYANKDIEIISEYSN; | KYANKDIEIISEYSNS; | YANKDIEIISEYSNSF; |
| ANKDIEIISEYSNSFS; | NKDIEIISEYSNSFSD; | KDIEIISEYSNSFSDV; |
| DIEIISEYSNSFSDVD; | IEIISEYSNSFSDVDI; | EIISEYSNSFSDVDIG; |
| IISEYSNSFSDVDIGR; | ISEYSNSFSDVDIGRT; | SEYSNSFSDVDIGRTI; |
| EYSNSFSDVDIGRTIA; | YSNSFSDVDIGRTIAS; | SNSFSDVDIGRTIASK; |
| NSFSDVDIGRTIASKM; | SFSDVDIGRTIASKMY; | FSDVDIGRTIASKMYS; |
| SDVDIGRTIASKMYSK; | DVDIGRTIASKMYSKG; | VDIGRTIASKMYSKGI; |

| | |
|---|---|
| | DIGRTIASKMYSKGID;   IGRTIASKMYSKGIDV;   GRTIASKMYSKGIDVI;<br>RTIASKMYSKGIDVIH;   TIASKMYSKGIDVIHF;   IASKMYSKGIDVIHFA;<br>ASKMYSKGIDVIHFAA;   SKMYSKGIDVIHFAAG;   KMYSKGIDVIHFAAGL;<br>MYSKGIDVIHFAAGLA;   YSKGIDVIHFAAGLAG;   SKGIDVIHFAAGLAGI;<br>KGIDVIHFAAGLAGIG;   GIDVIHFAAGLAGIGV;   IDVIHFAAGLAGIGVI;<br>DVIHFAAGLAGIGVIE;   VIHFAAGLAGIGVIET;   IHFAAGLAGIGVIETA;<br>HFAAGLAGIGVIETAK;   FAAGLAGIGVIETAKN;   AAGLAGIGVIETAKNL;<br>AGLAGIGVIETAKNLG;   GLAGIGVIETAKNLGD;   LAGIGVIETAKNLGDG;<br>AGIGVIETAKNLGDGY;   GIGVIETAKNLGDGYY;   IGVIETAKNLGDGYYV;<br>GVIETAKNLGDGYYVI;   VIETAKNLGDGYYVIG;   IETAKNLGDGYYVIGA;<br>ETAKNLGDGYYVIGAD;   TAKNLGDGYYVIGADQ;   AKNLGDGYYVIGADQD;<br>KNLGDGYYVIGADQDQ;   NLGDGYYVIGADQDQS;   LGDGYYVIGADQDQSY;<br>GDGYYVIGADQDQSYL;   DGYYVIGADQDQSYLA;   GYYVIGADQDQSYLAP;<br>YYVIGADQDQSYLAPK;   YVIGADQDQSYLAPKN;   VIGADQDQSYLAPKNF;<br>IGADQDQSYLAPKNFI;   GADQDQSYLAPKNFIT;   ADQDQSYLAPKNFITS;<br>DQDQSYLAPKNFITSV;   QDQSYLAPKNFITSVI;   DQSYLAPKNFITSVIK;<br>QSYLAPKNFITSVIKN;   SYLAPKNFITSVIKNI;   YLAPKNFITSVIKNIG;<br>LAPKNFITSVIKNIGD;   APKNFITSVIKNIGDA;   PKNFITSVIKNIGDAL;<br>KNFITSVIKNIGDALY;   NFITSVIKNIGDALYL;   FITSVIKNIGDALYLI;<br>ITSVIKNIGDALYLIT;   TSVIKNIGDALYLITG;   SVIKNIGDALYLITGE;<br>VIKNIGDALYLITGEY;   IKNIGDALYLITGEYI;   KNIGDALYLITGEYIK;<br>NIGDALYLITGEYIKN;   IGDALYLITGEYIKNN;   GDALYLITGEYIKNNN;<br>DALYLITGEYIKNNNV;   ALYLITGEYIKNNNVW;   LYLITGEYIKNNNVWE;<br>YLITGEYIKNNNVWEG;   LITGEYIKNNNVWEGG;   ITGEYIKNNNVWEGGK;<br>TGEYIKNNNVWEGGKV;   GEYIKNNNVWEGGKVV;   EYIKNNNVWEGGKVVQ;<br>YIKNNNVWEGGKVVQM;   IKNNNVWEGGKVVQMG;   KNNNVWEGGKVVQMGL;<br>NNNVWEGGKVVQMGLR;   NNVWEGGKVVQMGLRD;   NVWEGGKVVQMGLRDG;<br>VWEGGKVVQMGLRDGV;   WEGGKVVQMGLRDGVI;   EGGKVVQMGLRDGVIG;<br>GGKVVQMGLRDGVIGL;   GKVVQMGLRDGVIGLP;   KVVQMGLRDGVIGLPN;<br>VVQMGLRDGVIGLPNA;   VQMGLRDGVIGLPNAN;   QMGLRDGVIGLPNANE;<br>MGLRDGVIGLPNANEF;   GLRDGVIGLPNANEFE;   LRDGVIGLPNANEFEY;<br>RDGVIGLPNANEFEYI;   DGVIGLPNANEFEYIK;   GVIGLPNANEFEYIKV;<br>VIGLPNANEFEYIKVL;   IGLPNANEFEYIKVLE;   GLPNANEFEYIKVLER;<br>LPNANEFEYIKVLERK;   PNANEFEYIKVLERKI;   NANEFEYIKVLERKII;<br>ANEFEYIKVLERKIIN;   NEFEYIKVLERKIINK;   EFEYIKVLERKIINKE;<br>FEYIKVLERKIINKEI;   EYIKVLERKIINKEII;   YIKVLERKIINKEIIV;<br>IKVLERKIINKEIIVP;   KVLERKIINKEIIVPC;   VLERKIINKEIIVPCN;<br>LERKIINKEIIVPCNQ;   ERKIINKEIIVPCNQE;   RKIINKEIIVPCNQEE;<br>KIINKEIIVPCNQEEY;   IINKEIIVPCNQEEYE;   INKEIIVPCNQEEYEI;<br>NKEIIVPCNQEEYEIF;   KEIIVPCNQEEYEIFI;   EIIVPCNQEEYEIFIK;<br>IIVPCNQEEYEIFIKQ;   IVPCNQEEYEIFIKQI;   VPCNQEEYEIFIKQIL;<br>PCNQEEYEIFIKQILK;   CNQEEYEIFIKQILKL; |
| NP_212518.1<br>Basic membrane<br>protein C (bmpC)<br>[Borrelia<br>burgdorferi B31 | 13 mers:<br>MFKRFIFITLSLL;   FKRFIFITLSLLV;   KRFIFITLSLLVF;   RFIFITLSLLVFA;<br>FIFITLSLLVFAC;   IFITLSLLVFACF;   FITLSLLVFACFK;   ITLSLLVFACFKS;<br>TLSLLVFACFKSN;   LSLLVFACFKSNK;   SLLVFACFKSNKK;   LLVFACFKSNKKS;<br>LVFACFKSNKKSI;   VFACFKSNKKSIK;   FACFKSNKKSIKS;   ACFKSNKKSIKSD;<br>CFKSNKKSIKSDK;   FKSNKKSIKSDKV;   KSNKKSIKSDKVV;   SNKKSIKSDKVVV;<br>NKKSIKSDKVVVG;   KKSIKSDKVVVGV;   KSIKSDKVVVGVL;   SIKSDKVVVGVLA;<br>IKSDKVVVGVLAH;   KSDKVVVGVLAHG;   SDKVVVGVLAHGS;   DKVVVGVLAHGSF;<br>KVVVGVLAHGSFY;   VVVGVLAHGSFYD;   VVGVLAHGSFYDK;   VGVLAHGSFYDKG;<br>GVLAHGSFYDKGY;   VLAHGSFYDKGYN;   LAHGSFYDKGYNQ;   AHGSFYDKGYNQS;<br>HGSFYDKGYNQSV;   GSFYDKGYNQSVH;   SFYDKGYNQSVHD;   FYDKGYNQSVHDG;<br>YDKGYNQSVHDGV;   DKGYNQSVHDGVV;   KGYNQSVHDGVVK;   GYNQSVHDGVVKL;<br>YNQSVHDGVVKLR;   NQSVHDGVVKLRD;   QSVHDGVVKLRDN;   SVHDGVVKLRDNF; |

VHDGVVKLRDNFG; HDGVVKLRDNFGI; DGVVKLRDNFGIK; GVVKLRDNFGIKL;
VVKLRDNFGIKLI; VKLRDNFGIKLIT; KLRDNFGIKLITK; LRDNFGIKLITKS;
RDNFGIKLITKSL; DNFGIKLITKSLR; NFGIKLITKSLRP; FGIKLITKSLRPY;
GIKLITKSLRPYP; IKLITKSLRPYPI; KLITKSLRPYPIE; LITKSLRPYPIEG;
ITKSLRPYPIEGK; TKSLRPYPIEGKR; KSLRPYPIEGKRL; SLRPYPIEGKRLL;
LRPYPIEGKRLLT; RPYPIEGKRLLTV; PYPIEGKRLLTVD; YPIEGKRLLTVDE;
PIEGKRLLTVDEA; IEGKRLLTVDEAM; EGKRLLTVDEAMT; GKRLLTVDEAMTE;
KRLLTVDEAMTED; RLLTVDEAMTEDA; LLTVDEAMTEDAY; LTVDEAMTEDAYE;
TVDEAMTEDAYEV; VDEAMTEDAYEVQ; DEAMTEDAYEVQK; EAMTEDAYEVQKN;
AMTEDAYEVQKNP; MTEDAYEVQKNPL; TEDAYEVQKNPLN; EDAYEVQKNPLNL;
DAYEVQKNPLNLF; AYEVQKNPLNLFW; YEVQKNPLNLFWL; EVQKNPLNLFWLI;
VQKNPLNLFWLIG; QKNPLNLFWLIGY; KNPLNLFWLIGYR; NPLNLFWLIGYRF;
PLNLFWLIGYRFS; LNLFWLIGYRFSD; NLFWLIGYRFSDL; LFWLIGYRFSDLS;
FWLIGYRFSDLSV; WLIGYRFSDLSVK; LIGYRFSDLSVKL; IGYRFSDLSVKLS;
GYRFSDLSVKLSY; YRFSDLSVKLSYE; RFSDLSVKLSYER; FSDLSVKLSYERP;
SDLSVKLSYERPD; DLSVKLSYERPDI; LSVKLSYERPDIY; SVKLSYERPDIYY;
VKLSYERPDIYYG; KLSYERPDIYYGI; LSYERPDIYYGII; SYERPDIYYGIID;
YERPDIYYGIIDA; ERPDIYYGIIDAF; RPDIYYGIIDAFD; PDIYYGIIDAFDY;
DIYYGIIDAFDYG; IYYGIIDAFDYGD; YYGIIDAFDYGDI; YGIIDAFDYGDIQ;
GIIDAFDYGDIQV; IIDAFDYGDIQVP; IDAFDYGDIQVPK; DAFDYGDIQVPKN;
AFDYGDIQVPKNS; FDYGDIQVPKNSL; DYGDIQVPKNSLA; YGDIQVPKNSLAI;
GDIQVPKNSLAIK; DIQVPKNSLAIKF; IQVPKNSLAIKFR; QVPKNSLAIKFRN;
VPKNSLAIKFRNE; PKNSLAIKFRNEE; KNSLAIKFRNEEA; NSLAIKFRNEEAA;
SLAIKFRNEEAAF; LAIKFRNEEAAFL; AIKFRNEEAAFLA; IKFRNEEAAFLAG;
KFRNEEAAFLAGY; FRNEEAAFLAGYI; RNEEAAFLAGYIA; NEEAAFLAGYIAA;
EEAAFLAGYIAAK; EAAFLAGYIAAKM; AAFLAGYIAAKMS; AFLAGYIAAKMSR;
FLAGYIAAKMSRK; LAGYIAAKMSRKE; AGYIAAKMSRKEK; GYIAAKMSRKEKI;
YIAAKMSRKEKIG; IAAKMSRKEKIGF; AAKMSRKEKIGFL; AKMSRKEKIGFLT;
KMSRKEKIGFLTG; MSRKEKIGFLTGP; SRKEKIGFLTGPM; RKEKIGFLTGPMS;
KEKIGFLTGPMSE; EKIGFLTGPMSEH; KIGFLTGPMSEHV; IGFLTGPMSEHVK;
GFLTGPMSEHVKD; FLTGPMSEHVKDF; LTGPMSEHVKDFK; TGPMSEHVKDFKF;
GPMSEHVKDFKFG; PMSEHVKDFKFGF; MSEHVKDFKFGFK; SEHVKDFKFGFKA;
EHVKDFKFGFKAG; HVKDFKFGFKAGI; VKDFKFGFKAGIF; KDFKFGFKAGIFY;
DFKFGFKAGIFYA; FKFGFKAGIFYAN; KFGFKAGIFYANP; FGFKAGIFYANPK;
GFKAGIFYANPKL; FKAGIFYANPKLR; KAGIFYANPKLRL; AGIFYANPKLRLV;
GIFYANPKLRLVS; IFYANPKLRLVSK; FYANPKLRLVSKK; YANPKLRLVSKKA;
ANPKLRLVSKKAP; NPKLRLVSKKAPS; PKLRLVSKKAPSL; KLRLVSKKAPSLF;
LRLVSKKAPSLFD; RLVSKKAPSLFDK; LVSKKAPSLFDKE; VSKKAPSLFDKEK;
SKKAPSLFDKEKG; KKAPSLFDKEKGK; KAPSLFDKEKGKA; APSLFDKEKGKAM;
PSLFDKEKGKAMA; SLFDKEKGKAMAL; LFDKEKGKAMALF; FDKEKGKAMALFM;
DKEKGKAMALFMY; KEKGKAMALFMYK; EKGKAMALFMYKE; KGKAMALFMYKED;
GKAMALFMYKEDK; KAMALFMYKEDKV; AMALFMYKEDKVG; MALFMYKEDKVGV;
ALFMYKEDKVGVI; LFMYKEDKVGVIF; FMYKEDKVGVIFP; MYKEDKVGVIFPI;
YKEDKVGVIFPIA; KEDKVGVIFPIAG; EDKVGVIFPIAGI; DKVGVIFPIAGIT;
KVGVIFPIAGITG; VGVIFPIAGITGL; GVIFPIAGITGLG; VIFPIAGITGLGV;
IFPIAGITGLGVY; FPIAGITGLGVYD; PIAGITGLGVYDA; IAGITGLGVYDAA;
AGITGLGVYDAAK; GITGLGVYDAAKE; ITGLGVYDAAKEL; TGLGVYDAAKELG;
GLGVYDAAKELGP; LGVYDAAKELGPK; GVYDAAKELGPKY; VYDAAKELGPKYY;
YDAAKELGPKYYV; DAAKELGPKYYVI; AAKELGPKYYVIG; AKELGPKYYVIGL;
KELGPKYYVIGLN; ELGPKYYVIGLNQ; LGPKYYVIGLNQD; GPKYYVIGLNQDQ;
PKYYVIGLNQDQS; KYYVIGLNQDQSY; YYVIGLNQDQSYI; YVIGLNQDQSYIA;
VIGLNQDQSYIAP; IGLNQDQSYIAPQ; GLNQDQSYIAPQN; LNQDQSYIAPQNV;
NQDQSYIAPQNVI; QDQSYIAPQNVIT; DQSYIAPQNVITS; QSYIAPQNVITSI;
SYIAPQNVITSII; YIAPQNVITSIIK; IAPQNVITSIIKD; APQNVITSIIKDI;
PQNVITSIIKDIG; QNVITSIIKDIGK; NVITSIIKDIGKV; VITSIIKDIGKVI;
ITSIIKDIGKVIY; TSIIKDIGKVIYS; SIIKDIGKVIYSI; IIKDIGKVIYSIS;
IKDIGKVIYSISS; KDIGKVIYSISSE; DIGKVIYSISSEY; IGKVIYSISSEYI;

GKVIYSISSEYIN; KVIYSISSEYINN; VIYSISSEYINNR; IYSISSEYINNRV;
YSISSEYINNRVF; SISSEYINNRVFK; ISSEYINNRVFKG; SSEYINNRVFKGG;
SEYINNRVFKGGI; EYINNRVFKGGII; YINNRVFKGGIII; INNRVFKGGIIID;
NNRVFKGGIIIDR; NRVFKGGIIIDRG; RVFKGGIIIDRGL; VFKGGIIIDRGLK;
FKGGIIIDRGLKE; KGGIIIDRGLKEG; GGIIIDRGLKEGV; GIIIDRGLKEGVI;
IIIDRGLKEGVIE; IIDRGLKEGVIEI; IDRGLKEGVIEIV; DRGLKEGVIEIVK;
RGLKEGVIEIVKD; GLKEGVIEIVKDP; LKEGVIEIVKDPD; KEGVIEIVKDPDV;
EGVIEIVKDPDVL; GVIEIVKDPDVLN; VIEIVKDPDVLNN; IEIVKDPDVLNNR;
EIVKDPDVLNNRL; IVKDPDVLNNRLV; VKDPDVLNNRLVD; KDPDVLNNRLVDE;
DPDVLNNRLVDEV; PDVLNNRLVDEVI; DVLNNRLVDEVID; VLNNRLVDEVIDL;
LNNRLVDEVIDLE; NNRLVDEVIDLEN; NRLVDEVIDLENK; RLVDEVIDLENKI;
LVDEVIDLENKII; VDEVIDLENKIIS; DEVIDLENKIISG; EVIDLENKIISGE;
VIDLENKIISGEI; IDLENKIISGEII; DLENKIISGEIIV; LENKIISGEIIVP;
ENKIISGEIIVPD; NKIISGEIIVPDS; KIISGEIIVPDSE; IISGEIIVPDSEY;
ISGEIIVPDSEYA; SGEIIVPDSEYAF; GEIIVPDSEYAFD; EIIVPDSEYAFDL;
IIVPDSEYAFDLF; IVPDSEYAFDLFK; VPDSEYAFDLFKS; PDSEYAFDLFKSK;
DSEYAFDLFKSKL;

14 mers:
MFKRFIFITLSLLV; FKRFIFITLSLLVF; KRFIFITLSLLVFA;
RFIFITLSLLVFAC; FIFITLSLLVFACF; IFITLSLLVFACFK;
FITLSLLVFACFKS; ITLSLLVFACFKSN; TLSLLVFACFKSNK;
LSLLVFACFKSNKK; SLLVFACFKSNKKS; LLVFACFKSNKKSI;
LVFACFKSNKKSIK; VFACFKSNKKSIKS; FACFKSNKKSIKSD;
ACFKSNKKSIKSDK; CFKSNKKSIKSDKV; FKSNKKSIKSDKVV;
KSNKKSIKSDKVVV; SNKKSIKSDKVVVG; NKKSIKSDKVVVGV;
KKSIKSDKVVVGVL; KSIKSDKVVVGVLA; SIKSDKVVVGVLAH;
IKSDKVVVGVLAHG; KSDKVVVGVLAHGS; SDKVVVGVLAHGSF;
DKVVVGVLAHGSFY; KVVVGVLAHGSFYD; VVVGVLAHGSFYDK;
VVGVLAHGSFYDKG; VGVLAHGSFYDKGY; GVLAHGSFYDKGYN;
VLAHGSFYDKGYNQ; LAHGSFYDKGYNQS; AHGSFYDKGYNQSV;
HGSFYDKGYNQSVH; GSFYDKGYNQSVHD; SFYDKGYNQSVHDG;
FYDKGYNQSVHDGV; YDKGYNQSVHDGVV; DKGYNQSVHDGVVK;
KGYNQSVHDGVVKL; GYNQSVHDGVVKLR; YNQSVHDGVVKLRD;
NQSVHDGVVKLRDN; QSVHDGVVKLRDNF; SVHDGVVKLRDNFG;
VHDGVVKLRDNFGI; HDGVVKLRDNFGIK; DGVVKLRDNFGIKL;
GVVKLRDNFGIKLI; VVKLRDNFGIKLIT; VKLRDNFGIKLITK;
KLRDNFGIKLITKS; LRDNFGIKLITKSL; RDNFGIKLITKSLR;
DNFGIKLITKSLRP; NFGIKLITKSLRPY; FGIKLITKSLRPYP;
GIKLITKSLRPYPI; IKLITKSLRPYPIE; KLITKSLRPYPIEG;
LITKSLRPYPIEGK; ITKSLRPYPIEGKR; TKSLRPYPIEGKRL;
KSLRPYPIEGKRLL; SLRPYPIEGKRLLT; LRPYPIEGKRLLTV;
RPYPIEGKRLLTVD; PYPIEGKRLLTVDE; YPIEGKRLLTVDEA;
PIEGKRLLTVDEAM; IEGKRLLTVDEAMT; EGKRLLTVDEAMTE;
GKRLLTVDEAMTED; KRLLTVDEAMTEDA; RLLTVDEAMTEDAY;
LLTVDEAMTEDAYE; LTVDEAMTEDAYEV; TVDEAMTEDAYEVQ;
VDEAMTEDAYEVQK; DEAMTEDAYEVQKN; EAMTEDAYEVQKNP;
AMTEDAYEVQKNPL; MTEDAYEVQKNPLN; TEDAYEVQKNPLNL;
EDAYEVQKNPLNLF; DAYEVQKNPLNLFW; AYEVQKNPLNLFWL;
YEVQKNPLNLFWLI; EVQKNPLNLFWLIG; VQKNPLNLFWLIGY;
QKNPLNLFWLIGYR; KNPLNLFWLIGYRF; NPLNLFWLIGYRFS;
PLNLFWLIGYRFSD; LNLFWLIGYRFSDL; NLFWLIGYRFSDLS;
LFWLIGYRFSDLSV; FWLIGYRFSDLSVK; WLIGYRFSDLSVKL;
LIGYRFSDLSVKLS; IGYRFSDLSVKLSY; GYRFSDLSVKLSYE;
YRFSDLSVKLSYER; RFSDLSVKLSYERP; FSDLSVKLSYERPD;
SDLSVKLSYERPDI; DLSVKLSYERPDIY; LSVKLSYERPDIYY;
SVKLSYERPDIYYG; VKLSYERPDIYYGI; KLSYERPDIYYGII;

LSYERPDIYYGIID; SYERPDIYYGIIDA; YERPDIYYGIIDAF;
ERPDIYYGIIDAFD; RPDIYYGIIDAFDY; PDIYYGIIDAFDYG;
DIYYGIIDAFDYGD; IYYGIIDAFDYGDI; YYGIIDAFDYGDIQ;
YGIIDAFDYGDIQV; GIIDAFDYGDIQVP; IIDAFDYGDIQVPK;
IDAFDYGDIQVPKN; DAFDYGDIQVPKNS; AFDYGDIQVPKNSL;
FDYGDIQVPKNSLA; DYGDIQVPKNSLAI; YGDIQVPKNSLAIK;
GDIQVPKNSLAIKF; DIQVPKNSLAIKFR; IQVPKNSLAIKFRN;
QVPKNSLAIKFRNE; VPKNSLAIKFRNEE; PKNSLAIKFRNEEA;
KNSLAIKFRNEEAA; NSLAIKFRNEEAAF; SLAIKFRNEEAAFL;
LAIKFRNEEAAFLA; AIKFRNEEAAFLAG; IKFRNEEAAFLAGY;
KFRNEEAAFLAGYI; FRNEEAAFLAGYIA; RNEEAAFLAGYIAA;
NEEAAFLAGYIAAK; EEAAFLAGYIAAKM; EAAFLAGYIAAKMS;
AAFLAGYIAAKMSR; AFLAGYIAAKMSRK; FLAGYIAAKMSRKE;
LAGYIAAKMSRKEK; AGYIAAKMSRKEKI; GYIAAKMSRKEKIG;
YIAAKMSRKEKIGF; IAAKMSRKEKIGFL; AAKMSRKEKIGFLT;
AKMSRKEKIGFLTG; KMSRKEKIGFLTGP; MSRKEKIGFLTGPM;
SRKEKIGFLTGPMS; RKEKIGFLTGPMSE; KEKIGFLTGPMSEH;
EKIGFLTGPMSEHV; KIGFLTGPMSEHVK; IGFLTGPMSEHVKD;
GFLTGPMSEHVKDF; FLTGPMSEHVKDFK; LTGPMSEHVKDFKF;
TGPMSEHVKDFKFG; GPMSEHVKDFKFGF; PMSEHVKDFKFGFK;
MSEHVKDFKFGFKA; SEHVKDFKFGFKAG; EHVKDFKFGFKAGI;
HVKDFKFGFKAGIF; VKDFKFGFKAGIFY; KDFKFGFKAGIFYA;
DFKFGFKAGIFYAN; FKFGFKAGIFYANP; KFGFKAGIFYANPK;
FGFKAGIFYANPKL; GFKAGIFYANPKLR; FKAGIFYANPKLRL;
KAGIFYANPKLRLV; AGIFYANPKLRLVS; GIFYANPKLRLVSK;
IFYANPKLRLVSKK; FYANPKLRLVSKKA; YANPKLRLVSKKAP;
ANPKLRLVSKKAPS; NPKLRLVSKKAPSL; PKLRLVSKKAPSLF;
KLRLVSKKAPSLFD; LRLVSKKAPSLFDK; RLVSKKAPSLFDKE;
LVSKKAPSLFDKEK; VSKKAPSLFDKEKG; SKKAPSLFDKEKGK;
KKAPSLFDKEKGKA; KAPSLFDKEKGKAM; APSLFDKEKGKAMA;
PSLFDKEKGKAMAL; SLFDKEKGKAMALF; LFDKEKGKAMALFM;
FDKEKGKAMALFMY; DKEKGKAMALFMYK; KEKGKAMALFMYKE;
EKGKAMALFMYKED; KGKAMALFMYKEDK; GKAMALFMYKEDKV;
KAMALFMYKEDKVG; AMALFMYKEDKVGV; MALFMYKEDKVGVI;
ALFMYKEDKVGVIF; LFMYKEDKVGVIFP; FMYKEDKVGVIFPI;
MYKEDKVGVIFPIA; YKEDKVGVIFPIAG; KEDKVGVIFPIAGI;
EDKVGVIFPIAGIT; DKVGVIFPIAGITG; KVGVIFPIAGITGL;
VGVIFPIAGITGLG; GVIFPIAGITGLGV; VIFPIAGITGLGVY;
IFPIAGITGLGVYD; FPIAGITGLGVYDA; PIAGITGLGVYDAA;
IAGITGLGVYDAAK; AGITGLGVYDAAKE; GITGLGVYDAAKEL;
ITGLGVYDAAKELG; TGLGVYDAAKELGP; GLGVYDAAKELGPK;
LGVYDAAKELGPKY; GVYDAAKELGPKYY; VYDAAKELGPKYYV;
YDAAKELGPKYYVI; DAAKELGPKYYVIG; AAKELGPKYYVIGL;
AKELGPKYYVIGLN; KELGPKYYVIGLNQ; ELGPKYYVIGLNQD;
LGPKYYVIGLNQDQ; GPKYYVIGLNQDQS; PKYYVIGLNQDQSY;
KYYVIGLNQDQSYI; YYVIGLNQDQSYIA; YVIGLNQDQSYIAP;
VIGLNQDQSYIAPQ; IGLNQDQSYIAPQN; GLNQDQSYIAPQNV;
LNQDQSYIAPQNVI; NQDQSYIAPQNVIT; QDQSYIAPQNVITS;
DQSYIAPQNVITSI; QSYIAPQNVITSII; SYIAPQNVITSIIK;
YIAPQNVITSIIKD; IAPQNVITSIIKDI; APQNVITSIIKDIG;
PQNVITSIIKDIGK; QNVITSIIKDIGKV; NVITSIIKDIGKVI;
VITSIIKDIGKVIY; ITSIIKDIGKVIYS; TSIIKDIGKVIYSI;
SIIKDIGKVIYSIS; IIKDIGKVIYSISS; IKDIGKVIYSISSE;
KDIGKVIYSISSEY; DIGKVIYSISSEYI; IGKVIYSISSEYIN;
GKVIYSISSEYINN; KVIYSISSEYINNR; VIYSISSEYINNRV;
IYSISSEYINNRVF; YSISSEYINNRVFK; SISSEYINNRVFKG;
ISSEYINNRVFKGG; SSEYINNRVFKGGI; SEYINNRVFKGGII;

EYINNRVFKGGIII; YINNRVFKGGIIID; INNRVFKGGIIIDR;
NNRVFKGGIIIDRG; NRVFKGGIIIDRGL; RVFKGGIIIDRGLK;
VFKGGIIIDRGLKE; FKGGIIIDRGLKEG; KGGIIIDRGLKEGV;
GGIIIDRGLKEGVI; GIIIDRGLKEGVIE; IIIDRGLKEGVIEI;
IIDRGLKEGVIEIV; IDRGLKEGVIEIVK; DRGLKEGVIEIVKD;
RGLKEGVIEIVKDP; GLKEGVIEIVKDPD; LKEGVIEIVKDPDV;
KEGVIEIVKDPDVL; EGVIEIVKDPDVLN; GVIEIVKDPDVLNN;
VIEIVKDPDVLNNR; IEIVKDPDVLNNRL; EIVKDPDVLNNRLV;
IVKDPDVLNNRLVD; VKDPDVLNNRLVDE; KDPDVLNNRLVDEV;
DPDVLNNRLVDEVI; PDVLNNRLVDEVID; DVLNNRLVDEVIDL;
VLNNRLVDEVIDLE; LNNRLVDEVIDLEN; NNRLVDEVIDLENK;
NRLVDEVIDLENKI; RLVDEVIDLENKII; LVDEVIDLENKIIS;
VDEVIDLENKIISG; DEVIDLENKIISGE; EVIDLENKIISGEI;
VIDLENKIISGEII; IDLENKIISGEIIV; DLENKIISGEIIVP;
LENKIISGEIIVPD; ENKIISGEIIVPDS; NKIISGEIIVPDSE;
KIISGEIIVPDSEY; IISGEIIVPDSEYA; ISGEIIVPDSEYAF;
SGEIIVPDSEYAFD; GEIIVPDSEYAFDL; EIIVPDSEYAFDLF;
IIVPDSEYAFDLFK; IVPDSEYAFDLFKS; VPDSEYAFDLFKSK;
PDSEYAFDLFKSKL;

15 mers:
MFKRFIFITLSLLVF; FKRFIFITLSLLVFA; KRFIFITLSLLVFAC;
RFIFITLSLLVFACF; FIFITLSLLVFACFK; IFITLSLLVFACFKS;
FITLSLLVFACFKSN; ITLSLLVFACFKSNK; TLSLLVFACFKSNKK;
LSLLVFACFKSNKKS; SLLVFACFKSNKKSI; LLVFACFKSNKKSIK;
LVFACFKSNKKSIKS; VFACFKSNKKSIKSD; FACFKSNKKSIKSDK;
ACFKSNKKSIKSDKV; CFKSNKKSIKSDKVV; FKSNKKSIKSDKVVV;
KSNKKSIKSDKVVVG; SNKKSIKSDKVVVGV; NKKSIKSDKVVVGVL;
KKSIKSDKVVVGVLA; KSIKSDKVVVGVLAH; SIKSDKVVVGVLAHG;
IKSDKVVVGVLAHGS; KSDKVVVGVLAHGSF; SDKVVVGVLAHGSFY;
DKVVVGVLAHGSFYD; KVVVGVLAHGSFYDK; VVVGVLAHGSFYDKG;
VVGVLAHGSFYDKGY; VGVLAHGSFYDKGYN; GVLAHGSFYDKGYNQ;
VLAHGSFYDKGYNQS; LAHGSFYDKGYNQSV; AHGSFYDKGYNQSVH;
HGSFYDKGYNQSVHD; GSFYDKGYNQSVHDG; SFYDKGYNQSVHDGV;
FYDKGYNQSVHDGVV; YDKGYNQSVHDGVVK; DKGYNQSVHDGVVKL;
KGYNQSVHDGVVKLR; GYNQSVHDGVVKLRD; YNQSVHDGVVKLRDN;
NQSVHDGVVKLRDNF; QSVHDGVVKLRDNFG; SVHDGVVKLRDNFGI;
VHDGVVKLRDNFGIK; HDGVVKLRDNFGIKL; DGVVKLRDNFGIKLI;
GVVKLRDNFGIKLIT; VVKLRDNFGIKLITK; VKLRDNFGIKLITKS;
KLRDNFGIKLITKSL; LRDNFGIKLITKSLR; RDNFGIKLITKSLRP;
DNFGIKLITKSLRPY; NFGIKLITKSLRPYP; FGIKLITKSLRPYPI;
GIKLITKSLRPYPIE; IKLITKSLRPYPIEG; KLITKSLRPYPIEGK;
LITKSLRPYPIEGKR; ITKSLRPYPIEGKRL; TKSLRPYPIEGKRLL;
KSLRPYPIEGKRLLT; SLRPYPIEGKRLLTV; LRPYPIEGKRLLTVD;
RPYPIEGKRLLTVDE; PYPIEGKRLLTVDEA; YPIEGKRLLTVDEAM;
PIEGKRLLTVDEAMT; IEGKRLLTVDEAMTE; EGKRLLTVDEAMTED;
GKRLLTVDEAMTEDA; KRLLTVDEAMTEDAY; RLLTVDEAMTEDAYE;
LLTVDEAMTEDAYEV; LTVDEAMTEDAYEVQ; TVDEAMTEDAYEVQK;
VDEAMTEDAYEVQKN; DEAMTEDAYEVQKNP; EAMTEDAYEVQKNPL;
AMTEDAYEVQKNPLN; MTEDAYEVQKNPLNL; TEDAYEVQKNPLNLF;
EDAYEVQKNPLNLFW; DAYEVQKNPLNLFWL; AYEVQKNPLNLFWLI;
YEVQKNPLNLFWLIG; EVQKNPLNLFWLIGY; VQKNPLNLFWLIGYR;
QKNPLNLFWLIGYRF; KNPLNLFWLIGYRFS; NPLNLFWLIGYRFSD;
PLNLFWLIGYRFSDL; LNLFWLIGYRFSDLS; NLFWLIGYRFSDLSV;
LFWLIGYRFSDLSVK; FWLIGYRFSDLSVKL; WLIGYRFSDLSVKLS;
LIGYRFSDLSVKLSY; IGYRFSDLSVKLSYE; GYRFSDLSVKLSYER;
YRFSDLSVKLSYERP; RFSDLSVKLSYERPD; FSDLSVKLSYERPDI;

SDLSVKLSYERPDIY; DLSVKLSYERPDIYY; LSVKLSYERPDIYYG;
SVKLSYERPDIYYGI; VKLSYERPDIYYGII; KLSYERPDIYYGIID;
LSYERPDIYYGIIDA; SYERPDIYYGIIDAF; YERPDIYYGIIDAFD;
ERPDIYYGIIDAFDY; RPDIYYGIIDAFDYG; PDIYYGIIDAFDYGD;
DIYYGIIDAFDYGDI; IYYGIIDAFDYGDIQ; YYGIIDAFDYGDIQV;
YGIIDAFDYGDIQVP; GIIDAFDYGDIQVPK; IIDAFDYGDIQVPKN;
IDAFDYGDIQVPKNS; DAFDYGDIQVPKNSL; AFDYGDIQVPKNSLA;
FDYGDIQVPKNSLAI; DYGDIQVPKNSLAIK; YGDIQVPKNSLAIKF;
GDIQVPKNSLAIKFR; DIQVPKNSLAIKFRN; IQVPKNSLAIKFRNE;
QVPKNSLAIKFRNEE; VPKNSLAIKFRNEEA; PKNSLAIKFRNEEAA;
KNSLAIKFRNEEAAF; NSLAIKFRNEEAAFL; SLAIKFRNEEAAFLA;
LAIKFRNEEAAFLAG; AIKFRNEEAAFLAGY; IKFRNEEAAFLAGYI;
KFRNEEAAFLAGYIA; FRNEEAAFLAGYIAA; RNEEAAFLAGYIAAK;
NEEAAFLAGYIAAKM; EEAAFLAGYIAAKMS; EAAFLAGYIAAKMSR;
AAFLAGYIAAKMSRK; AFLAGYIAAKMSRKE; FLAGYIAAKMSRKEK;
LAGYIAAKMSRKEKI; AGYIAAKMSRKEKIG; GYIAAKMSRKEKIGF;
YIAAKMSRKEKIGFL; IAAKMSRKEKIGFLT; AAKMSRKEKIGFLTG;
AKMSRKEKIGFLTGP; KMSRKEKIGFLTGPM; MSRKEKIGFLTGPMS;
SRKEKIGFLTGPMSE; RKEKIGFLTGPMSEH; KEKIGFLTGPMSEHV;
EKIGFLTGPMSEHVK; KIGFLTGPMSEHVKD; IGFLTGPMSEHVKDF;
GFLTGPMSEHVKDFK; FLTGPMSEHVKDFKF; LTGPMSEHVKDFKFG;
TGPMSEHVKDFKFGF; GPMSEHVKDFKFGFK; PMSEHVKDFKFGFKA;
MSEHVKDFKFGFKAG; SEHVKDFKFGFKAGI; EHVKDFKFGFKAGIF;
HVKDFKFGFKAGIFY; VKDFKFGFKAGIFYA; KDFKFGFKAGIFYAN;
DFKFGFKAGIFYANP; FKFGFKAGIFYANPK; KFGFKAGIFYANPKL;
FGFKAGIFYANPKLR; GFKAGIFYANPKLRL; FKAGIFYANPKLRLV;
KAGIFYANPKLRLVS; AGIFYANPKLRLVSK; GIFYANPKLRLVSKK;
IFYANPKLRLVSKKA; FYANPKLRLVSKKAP; YANPKLRLVSKKAPS;
ANPKLRLVSKKAPSL; NPKLRLVSKKAPSLF; PKLRLVSKKAPSLFD;
KLRLVSKKAPSLFDK; LRLVSKKAPSLFDKE; RLVSKKAPSLFDKEK;
LVSKKAPSLFDKEKG; VSKKAPSLFDKEKGK; SKKAPSLFDKEKGKA;
KKAPSLFDKEKGKAM; KAPSLFDKEKGKAMA; APSLFDKEKGKAMAL;
PSLFDKEKGKAMALF; SLFDKEKGKAMALFM; LFDKEKGKAMALFMY;
FDKEKGKAMALFMYK; DKEKGKAMALFMYKE; KEKGKAMALFMYKED;
EKGKAMALFMYKEDK; KGKAMALFMYKEDKV; GKAMALFMYKEDKVG;
KAMALFMYKEDKVGV; AMALFMYKEDKVGVI; MALFMYKEDKVGVIF;
ALFMYKEDKVGVIFP; LFMYKEDKVGVIFPI; FMYKEDKVGVIFPIA;
MYKEDKVGVIFPIAG; YKEDKVGVIFPIAGI; KEDKVGVIFPIAGIT;
EDKVGVIFPIAGITG; DKVGVIFPIAGITGL; KVGVIFPIAGITGLG;
VGVIFPIAGITGLGV; GVIFPIAGITGLGVY; VIFPIAGITGLGVYD;
IFPIAGITGLGVYDA; FPIAGITGLGVYDAA; PIAGITGLGVYDAAK;
IAGITGLGVYDAAKE; AGITGLGVYDAAKEL; GITGLGVYDAAKELG;
ITGLGVYDAAKELGP; TGLGVYDAAKELGPK; GLGVYDAAKELGPKY;
LGVYDAAKELGPKYY; GVYDAAKELGPKYYV; VYDAAKELGPKYYVI;
YDAAKELGPKYYVIG; DAAKELGPKYYVIGL; AAKELGPKYYVIGLN;
AKELGPKYYVIGLNQ; KELGPKYYVIGLNQD; ELGPKYYVIGLNQDQ;
LGPKYYVIGLNQDQS; GPKYYVIGLNQDQSY; PKYYVIGLNQDQSYI;
KYYVIGLNQDQSYIA; YYVIGLNQDQSYIAP; YVIGLNQDQSYIAPQ;
VIGLNQDQSYIAPQN; IGLNQDQSYIAPQNV; GLNQDQSYIAPQNVI;
LNQDQSYIAPQNVIT; NQDQSYIAPQNVITS; QDQSYIAPQNVITSI;
DQSYIAPQNVITSII; QSYIAPQNVITSIIK; SYIAPQNVITSIIKD;
YIAPQNVITSIIKDI; IAPQNVITSIIKDIG; APQNVITSIIKDIGK;
PQNVITSIIKDIGKV; QNVITSIIKDIGKVI; NVITSIIKDIGKVIY;
VITSIIKDIGKVIYS; ITSIIKDIGKVIYSI; TSIIKDIGKVIYSIS;
SIIKDIGKVIYSISS; IIKDIGKVIYSISSE; IKDIGKVIYSISSEY;
KDIGKVIYSISSEYI; DIGKVIYSISSEYIN; IGKVIYSISSEYINN;
GKVIYSISSEYINNR; KVIYSISSEYINNRV; VIYSISSEYINNRVF;

IYSISSEYINNRVFK; YSISSEYINNRVFKG; SISSEYINNRVFKGG;
ISSEYINNRVFKGGI; SSEYINNRVFKGGII; SEYINNRVFKGGIII;
EYINNRVFKGGIIID; YINNRVFKGGIIIDR; INNRVFKGGIIIDRG;
NNRVFKGGIIIDRGL; NRVFKGGIIIDRGLK; RVFKGGIIIDRGLKE;
VFKGGIIIDRGLKEG; FKGGIIIDRGLKEGV; KGGIIIDRGLKEGVI;
GGIIIDRGLKEGVIE; GIIIDRGLKEGVIEI; IIIDRGLKEGVIEIV;
IIDRGLKEGVIEIVK; IDRGLKEGVIEIVKD; DRGLKEGVIEIVKDP;
RGLKEGVIEIVKDPD; GLKEGVIEIVKDPDV; LKEGVIEIVKDPDVL;
KEGVIEIVKDPDVLN; EGVIEIVKDPDVLNN; GVIEIVKDPDVLNNR;
VIEIVKDPDVLNNRL; IEIVKDPDVLNNRLV; EIVKDPDVLNNRLVD;
IVKDPDVLNNRLVDE; VKDPDVLNNRLVDEV; KDPDVLNNRLVDEVI;
DPDVLNNRLVDEVID; PDVLNNRLVDEVIDL; DVLNNRLVDEVIDLE;
VLNNRLVDEVIDLEN; LNNRLVDEVIDLENK; NNRLVDEVIDLENKI;
NRLVDEVIDLENKII; RLVDEVIDLENKIIS; LVDEVIDLENKIISG;
VDEVIDLENKIISGE; DEVIDLENKIISGEI; EVIDLENKIISGEII;
VIDLENKIISGEIIV; IDLENKIISGEIIVP; DLENKIISGEIIVPD;
LENKIISGEIIVPDS; ENKIISGEIIVPDSE; NKIISGEIIVPDSEY;
KIISGEIIVPDSEYA; IISGEIIVPDSEYAF; ISGEIIVPDSEYAFD;
SGEIIVPDSEYAFDL; GEIIVPDSEYAFDLF; EIIVPDSEYAFDLFK;
IIVPDSEYAFDLFKS; IVPDSEYAFDLFKSK; VPDSEYAFDLFKSKL;

16 mers:
MFKRFIFITLSLLVFA; FKRFIFITLSLLVFAC; KRFIFITLSLLVFACF;
RFIFITLSLLVFACFK; FIFITLSLLVFACFKS; IFITLSLLVFACFKSN;
FITLSLLVFACFKSNK; ITLSLLVFACFKSNKK; TLSLLVFACFKSNKKS;
LSLLVFACFKSNKKSI; SLLVFACFKSNKKSIK; LLVFACFKSNKKSIKS;
LVFACFKSNKKSIKSD; VFACFKSNKKSIKSDK; FACFKSNKKSIKSDKV;
ACFKSNKKSIKSDKVV; CFKSNKKSIKSDKVVV; FKSNKKSIKSDKVVVG;
KSNKKSIKSDKVVVGV; SNKKSIKSDKVVVGVL; NKKSIKSDKVVVGVLA;
KKSIKSDKVVVGVLAH; KSIKSDKVVVGVLAHG; SIKSDKVVVGVLAHGS;
IKSDKVVVGVLAHGSF; KSDKVVVGVLAHGSFY; SDKVVVGVLAHGSFYD;
DKVVVGVLAHGSFYDK; KVVVGVLAHGSFYDKG; VVVGVLAHGSFYDKGY;
VVGVLAHGSFYDKGYN; VGVLAHGSFYDKGYNQ; GVLAHGSFYDKGYNQS;
VLAHGSFYDKGYNQSV; LAHGSFYDKGYNQSVH; AHGSFYDKGYNQSVHD;
HGSFYDKGYNQSVHDG; GSFYDKGYNQSVHDGV; SFYDKGYNQSVHDGVV;
FYDKGYNQSVHDGVVK; YDKGYNQSVHDGVVKL; DKGYNQSVHDGVVKLR;
KGYNQSVHDGVVKLRD; GYNQSVHDGVVKLRDN; YNQSVHDGVVKLRDNF;
NQSVHDGVVKLRDNFG; QSVHDGVVKLRDNFGI; SVHDGVVKLRDNFGIK;
VHDGVVKLRDNFGIKL; HDGVVKLRDNFGIKLI; DGVVKLRDNFGIKLIT;
GVVKLRDNFGIKLITK; VVKLRDNFGIKLITKS; VKLRDNFGIKLITKSL;
KLRDNFGIKLITKSLR; LRDNFGIKLITKSLRP; RDNFGIKLITKSLRPY;
DNFGIKLITKSLRPYP; NFGIKLITKSLRPYPI; FGIKLITKSLRPYPIE;
GIKLITKSLRPYPIEG; IKLITKSLRPYPIEGK; KLITKSLRPYPIEGKR;
LITKSLRPYPIEGKRL; ITKSLRPYPIEGKRLL; TKSLRPYPIEGKRLLT;
KSLRPYPIEGKRLLTV; SLRPYPIEGKRLLTVD; LRPYPIEGKRLLTVDE;
RPYPIEGKRLLTVDEA; PYPIEGKRLLTVDEAM; YPIEGKRLLTVDEAMT;
PIEGKRLLTVDEAMTE; IEGKRLLTVDEAMTED; EGKRLLTVDEAMTEDA;
GKRLLTVDEAMTEDAY; KRLLTVDEAMTEDAYE; RLLTVDEAMTEDAYEV;
LLTVDEAMTEDAYEVQ; LTVDEAMTEDAYEVQK; TVDEAMTEDAYEVQKN;
VDEAMTEDAYEVQKNP; DEAMTEDAYEVQKNPL; EAMTEDAYEVQKNPLN;
AMTEDAYEVQKNPLNL; MTEDAYEVQKNPLNLF; TEDAYEVQKNPLNLFW;
EDAYEVQKNPLNLFWL; DAYEVQKNPLNLFWLI; AYEVQKNPLNLFWLIG;
YEVQKNPLNLFWLIGY; EVQKNPLNLFWLIGYR; VQKNPLNLFWLIGYRF;
QKNPLNLFWLIGYRFS; KNPLNLFWLIGYRFSD; NPLNLFWLIGYRFSDL;
PLNLFWLIGYRFSDLS; LNLFWLIGYRFSDLSV; NLFWLIGYRFSDLSVK;
LFWLIGYRFSDLSVKL; FWLIGYRFSDLSVKLS; WLIGYRFSDLSVKLSY;
LIGYRFSDLSVKLSYE; IGYRFSDLSVKLSYER; GYRFSDLSVKLSYERP;

| | | |
|---|---|---|
| YRFSDLSVKLSYERPD; | RFSDLSVKLSYERPDI; | FSDLSVKLSYERPDIY; |
| SDLSVKLSYERPDIYY; | DLSVKLSYERPDIYYG; | LSVKLSYERPDIYYGI; |
| SVKLSYERPDIYYGII; | VKLSYERPDIYYGIID; | KLSYERPDIYYGIIDA; |
| LSYERPDIYYGIIDAF; | SYERPDIYYGIIDAFD; | YERPDIYYGIIDAFDY; |
| ERPDIYYGIIDAFDYG; | RPDIYYGIIDAFDYGD; | PDIYYGIIDAFDYGDI; |
| DIYYGIIDAFDYGDIQ; | IYYGIIDAFDYGDIQV; | YYGIIDAFDYGDIQVP; |
| YGIIDAFDYGDIQVPK; | GIIDAFDYGDIQVPKN; | IIDAFDYGDIQVPKNS; |
| IDAFDYGDIQVPKNSL; | DAFDYGDIQVPKNSLA; | AFDYGDIQVPKNSLAI; |
| FDYGDIQVPKNSLAIK; | DYGDIQVPKNSLAIKF; | YGDIQVPKNSLAIKFR; |
| GDIQVPKNSLAIKFRN; | DIQVPKNSLAIKFRNE; | IQVPKNSLAIKFRNEE; |
| QVPKNSLAIKFRNEEA; | VPKNSLAIKFRNEEAA; | PKNSLAIKFRNEEAAF; |
| KNSLAIKFRNEEAAFL; | NSLAIKFRNEEAAFLA; | SLAIKFRNEEAAFLAG; |
| LAIKFRNEEAAFLAGY; | AIKFRNEEAAFLAGYI; | IKFRNEEAAFLAGYIA; |
| KFRNEEAAFLAGYIAA; | FRNEEAAFLAGYIAAK; | RNEEAAFLAGYIAAKM; |
| NEEAAFLAGYIAAKMS; | EEAAFLAGYIAAKMSR; | EAAFLAGYIAAKMSRK; |
| AAFLAGYIAAKMSRKE; | AFLAGYIAAKMSRKEK; | FLAGYIAAKMSRKEKI; |
| LAGYIAAKMSRKEKIG; | AGYIAAKMSRKEKIGF; | GYIAAKMSRKEKIGFL; |
| YIAAKMSRKEKIGFLT; | IAAKMSRKEKIGFLTG; | AAKMSRKEKIGFLTGP; |
| AKMSRKEKIGFLTGPM; | KMSRKEKIGFLTGPMS; | MSRKEKIGFLTGPMSE; |
| SRKEKIGFLTGPMSEH; | RKEKIGFLTGPMSEHV; | KEKIGFLTGPMSEHVK; |
| EKIGFLTGPMSEHVKD; | KIGFLTGPMSEHVKDF; | IGFLTGPMSEHVKDFK; |
| GFLTGPMSEHVKDFKF; | FLTGPMSEHVKDFKFG; | LTGPMSEHVKDFKFGF; |
| TGPMSEHVKDFKFGFK; | GPMSEHVKDFKFGFKA; | PMSEHVKDFKFGFKAG; |
| MSEHVKDFKFGFKAGI; | SEHVKDFKFGFKAGIF; | EHVKDFKFGFKAGIFY; |
| HVKDFKFGFKAGIFYA; | VKDFKFGFKAGIFYAN; | KDFKFGFKAGIFYANP; |
| DFKFGFKAGIFYANPK; | FKFGFKAGIFYANPKL; | KFGFKAGIFYANPKLR; |
| FGFKAGIFYANPKLRL; | GFKAGIFYANPKLRLV; | FKAGIFYANPKLRLVS; |
| KAGIFYANPKLRLVSK; | AGIFYANPKLRLVSKK; | GIFYANPKLRLVSKKA; |
| IFYANPKLRLVSKKAP; | FYANPKLRLVSKKAPS; | YANPKLRLVSKKAPSL; |
| ANPKLRLVSKKAPSLF; | NPKLRLVSKKAPSLFD; | PKLRLVSKKAPSLFDK; |
| KLRLVSKKAPSLFDKE; | LRLVSKKAPSLFDKEK; | RLVSKKAPSLFDKEKG; |
| LVSKKAPSLFDKEKGK; | VSKKAPSLFDKEKGKA; | SKKAPSLFDKEKGKAM; |
| KKAPSLFDKEKGKAMA; | KAPSLFDKEKGKAMAL; | APSLFDKEKGKAMALF; |
| PSLFDKEKGKAMALFM; | SLFDKEKGKAMALFMY; | LFDKEKGKAMALFMYK; |
| FDKEKGKAMALFMYKE; | DKEKGKAMALFMYKED; | KEKGKAMALFMYKEDK; |
| EKGKAMALFMYKEDKV; | KGKAMALFMYKEDKVG; | GKAMALFMYKEDKVGV; |
| KAMALFMYKEDKVGVI; | AMALFMYKEDKVGVIF; | MALFMYKEDKVGVIFP; |
| ALFMYKEDKVGVIFPI; | LFMYKEDKVGVIFPIA; | FMYKEDKVGVIFPIAG; |
| MYKEDKVGVIFPIAGI; | YKEDKVGVIFPIAGIT; | KEDKVGVIFPIAGITG; |
| EDKVGVIFPIAGITGL; | DKVGVIFPIAGITGLG; | KVGVIFPIAGITGLGV; |
| VGVIFPIAGITGLGVY; | GVIFPIAGITGLGVYD; | VIFPIAGITGLGVYDA; |
| IFPIAGITGLGVYDAA; | FPIAGITGLGVYDAAK; | PIAGITGLGVYDAAKE; |
| IAGITGLGVYDAAKEL; | AGITGLGVYDAAKELG; | GITGLGVYDAAKELGP; |
| ITGLGVYDAAKELGPK; | TGLGVYDAAKELGPKY; | GLGVYDAAKELGPKYY; |
| LGVYDAAKELGPKYYV; | GVYDAAKELGPKYYVI; | VYDAAKELGPKYYVIG; |
| YDAAKELGPKYYVIGL; | DAAKELGPKYYVIGLN; | AAKELGPKYYVIGLNQ; |
| AKELGPKYYVIGLNQD; | KELGPKYYVIGLNQDQ; | ELGPKYYVIGLNQDQS; |
| LGPKYYVIGLNQDQSY; | GPKYYVIGLNQDQSYI; | PKYYVIGLNQDQSYIA; |
| KYYVIGLNQDQSYIAP; | YYVIGLNQDQSYIAPQ; | YVIGLNQDQSYIAPQN; |
| VIGLNQDQSYIAPQNV; | IGLNQDQSYIAPQNVI; | GLNQDQSYIAPQNVIT; |
| LNQDQSYIAPQNVITS; | NQDQSYIAPQNVITSI; | QDQSYIAPQNVITSII; |
| DQSYIAPQNVITSIIK; | QSYIAPQNVITSIIKD; | SYIAPQNVITSIIKDI; |
| YIAPQNVITSIIKDIG; | IAPQNVITSIIKDIGK; | APQNVITSIIKDIGKV; |
| PQNVITSIIKDIGKVI; | QNVITSIIKDIGKVIY; | NVITSIIKDIGKVIYS; |
| VITSIIKDIGKVIYSI; | ITSIIKDIGKVIYSIS; | TSIIKDIGKVIYSISS; |
| SIIKDIGKVIYSISSE; | IIKDIGKVIYSISSEY; | IKDIGKVIYSISSEYI; |
| KDIGKVIYSISSEYIN; | DIGKVIYSISSEYINN; | IGKVIYSISSEYINNR; |

| | |
|---|---|
| | GKVIYSISSEYINNRV; KVIYSISSEYINNRVF; VIYSISSEYINNRVFK;<br>IYSISSEYINNRVFKG; YSISSEYINNRVFKGG; SISSEYINNRVFKGGI;<br>ISSEYINNRVFKGGII; SSEYINNRVFKGGIII; SEYINNRVFKGGIIID;<br>EYINNRVFKGGIIIDR; YINNRVFKGGIIIDRG; INNRVFKGGIIIDRGL;<br>NNRVFKGGIIIDRGLK; NRVFKGGIIIDRGLKE; RVFKGGIIIDRGLKEG;<br>VFKGGIIIDRGLKEGV; FKGGIIIDRGLKEGVI; KGGIIIDRGLKEGVIE;<br>GGIIIDRGLKEGVIEI; GIIIDRGLKEGVIEIV; IIIDRGLKEGVIEIVK;<br>IIDRGLKEGVIEIVKD; IDRGLKEGVIEIVKDP; DRGLKEGVIEIVKDPD;<br>RGLKEGVIEIVKDPDV; GLKEGVIEIVKDPDVL; LKEGVIEIVKDPDVLN;<br>KEGVIEIVKDPDVLNN; EGVIEIVKDPDVLNNR; GVIEIVKDPDVLNNRL;<br>VIEIVKDPDVLNNRLV; IEIVKDPDVLNNRLVD; EIVKDPDVLNNRLVDE;<br>IVKDPDVLNNRLVDEV; VKDPDVLNNRLVDEVI; KDPDVLNNRLVDEVID;<br>DPDVLNNRLVDEVIDL; PDVLNNRLVDEVIDLE; DVLNNRLVDEVIDLEN;<br>VLNNRLVDEVIDLENK; LNNRLVDEVIDLENKI; NNRLVDEVIDLENKII;<br>NRLVDEVIDLENKIIS; RLVDEVIDLENKIISG; LVDEVIDLENKIISGE;<br>VDEVIDLENKIISGEI; DEVIDLENKIISGEII; EVIDLENKIISGEIIV;<br>VIDLENKIISGEIIVP; IDLENKIISGEIIVPD; DLENKIISGEIIVPDS;<br>LENKIISGEIIVPDSE; ENKIISGEIIVPDSEY; NKIISGEIIVPDSEYA;<br>KIISGEIIVPDSEYAF; IISGEIIVPDSEYAFD; ISGEIIVPDSEYAFDL;<br>SGEIIVPDSEYAFDLF; GEIIVPDSEYAFDLFK; EIIVPDSEYAFDLFKS;<br>IIVPDSEYAFDLFKSK; IVPDSEYAFDLFKSKL; |
| NP_212519.1B<br>Basic membrane<br>protein D (bmpD)<br>[Borrelia<br>burgdorferi B31 | 13 mers:<br>MDNYYEIYFLYFF; DNYYEIYFLYFFI; NYYEIYFLYFFIK; YYEIYFLYFFIKS;<br>YEIYFLYFFIKSK; EIYFLYFFIKSKE; IYFLYFFIKSKED; YFLYFFIKSKEDI;<br>FLYFFIKSKEDIF; LYFFIKSKEDIFM; YFFIKSKEDIFML; FFIKSKEDIFMLK;<br>FIKSKEDIFMLKK; IKSKEDIFMLKKV; KSKEDIFMLKKVY; SKEDIFMLKKVYY;<br>KEDIFMLKKVYYF; EDIFMLKKVYYFL; DIFMLKKVYYFLI; IFMLKKVYYFLIF;<br>FMLKKVYYFLIFL; MLKKVYYFLIFLF; LKKVYYFLIFLFI; KKVYYFLIFLFIV;<br>KVYYFLIFLFIVA; VYYFLIFLFIVAC; YYFLIFLFIVACS; YFLIFLFIVACSS;<br>FLIFLFIVACSSS; LIFLFIVACSSSD; IFLFIVACSSSDD; FLFIVACSSSDDG;<br>LFIVACSSSDDGK; FIVACSSSDDGKS; IVACSSSDDGKSE; VACSSSDDGKSEA;<br>ACSSSDDGKSEAK; CSSSDDGKSEAKT; SSSDDGKSEAKTV; SSDDGKSEAKTVS;<br>SDDGKSEAKTVSL; DDGKSEAKTVSLI; DGKSEAKTVSLIV; GKSEAKTVSLIVD;<br>KSEAKTVSLIVDG; SEAKTVSLIVDGA; EAKTVSLIVDGAF; AKTVSLIVDGAFD;<br>KTVSLIVDGAFDD; TVSLIVDGAFDDK; VSLIVDGAFDDKG; SLIVDGAFDDKGF;<br>LIVDGAFDDKGFN; IVDGAFDDKGFNE; VDGAFDDKGFNES; DGAFDDKGFNESS;<br>GAFDDKGFNESSS; AFDDKGFNESSSK; FDDKGFNESSSKA; DDKGFNESSSKAI;<br>DKGFNESSSKAIR; KGFNESSSKAIRK; GFNESSSKAIRKL; FNESSSKAIRKLK;<br>NESSSKAIRKLKA; ESSSKAIRKLKAD; SSSKAIRKLKADL; SSKAIRKLKADLN;<br>SKAIRKLKADLNI; KAIRKLKADLNIN; AIRKLKADLNINI; IRKLKADLNINII;<br>RKLKADLNINIIE; KLKADLNINIIEK; LKADLNINIIEKA; KADLNINIIEKAS;<br>ADLNINIIEKAST; DLNINIIEKASTG; LNINIIEKASTGN; NINIIEKASTGNS;<br>INIIEKASTGNSY; NIIEKASTGNSYL; IIEKASTGNSYLG; IEKASTGNSYLGD;<br>EKASTGNSYLGDI; KASTGNSYLGDIA; ASTGNSYLGDIAN; STGNSYLGDIANL;<br>TGNSYLGDIANLE; GNSYLGDIANLED; NSYLGDIANLEDG; SYLGDIANLEDGN;<br>YLGDIANLEDGNS; LGDIANLEDGNSN; GDIANLEDGNSNL; DIANLEDGNSNLI;<br>IANLEDGNSNLIW; ANLEDGNSNLIWG; NLEDGNSNLIWGI; LEDGNSNLIWGIG;<br>EDGNSNLIWGIGF; DGNSNLIWGIGFR; GNSNLIWGIGFRL; NSNLIWGIGFRLS;<br>SNLIWGIGFRLSD; NLIWGIGFRLSDI; LIWGIGFRLSDIL; IWGIGFRLSDILF;<br>WGIGFRLSDILFQ; GIGFRLSDILFQR; IGFRLSDILFQRA; GFRLSDILFQRAS;<br>FRLSDILFQRASE; RLSDILFQRASEN; LSDILFQRASENV; SDILFQRASENVS;<br>DILFQRASENVSV; ILFQRASENVSVN; LFQRASENVSVNY; FQRASENVSVNYA;<br>QRASENVSVNYAI; RASENVSVNYAII; ASENVSVNYAIIE; SENVSVNYAIIEG;<br>ENVSVNYAIIEGV; NVSVNYAIIEGVY; VSVNYAIIEGVYD; SVNYAIIEGVYDE;<br>VNYAIIEGVYDEI; NYAIIEGVYDEIQ; YAIIEGVYDEIQI; AIIEGVYDEIQIP; |

EP 2 254 592 B1

IIEGVYDEIQIPK; IEGVYDEIQIPKN; EGVYDEIQIPKNL; GVYDEIQIPKNLL;
VYDEIQIPKNLLN; YDEIQIPKNLLNI; DEIQIPKNLLNIS; EIQIPKNLLNISF;
IQIPKNLLNISFR; QIPKNLLNISFRS; IPKNLLNISFRSE; PKNLLNISFRSEE;
KNLLNISFRSEEV; NLLNISFRSEEVA; LLNISFRSEEVAF; LNISFRSEEVAFL;
NISFRSEEVAFLA; ISFRSEEVAFLAG; SFRSEEVAFLAGY; FRSEEVAFLAGYF;
RSEEVAFLAGYFA; SEEVAFLAGYFAS; EEVAFLAGYFASK; EVAFLAGYFASKA;
VAFLAGYFASKAS; AFLAGYFASKASK; FLAGYFASKASKT; LAGYFASKASKTG;
AGYFASKASKTGK; GYFASKASKTGKI; YFASKASKTGKIG; FASKASKTGKIGF;
ASKASKTGKIGFV; SKASKTGKIGFVG; KASKTGKIGFVGG; ASKTGKIGFVGGV;
SKTGKIGFVGGVR; KTGKIGFVGGVRG; TGKIGFVGGVRGK; GKIGFVGGVRGKV;
KIGFVGGVRGKVL; IGFVGGVRGKVLE; GFVGGVRGKVLES; FVGGVRGKVLESF;
VGGVRGKVLESFM; GGVRGKVLESFMY; GVRGKVLESFMYG; VRGKVLESFMYGY;
RGKVLESFMYGYE; GKVLESFMYGYEA; KVLESFMYGYEAG; VLESFMYGYEAGA;
LESFMYGYEAGAK; ESFMYGYEAGAKY; SFMYGYEAGAKYA; FMYGYEAGAKYAN;
MYGYEAGAKYANS; YGYEAGAKYANSN; GYEAGAKYANSNI; YEAGAKYANSNIK;
EAGAKYANSNIKV; AGAKYANSNIKVV; GAKYANSNIKVVS; AKYANSNIKVVSQ;
KYANSNIKVVSQY; YANSNIKVVSQYV; ANSNIKVVSQYVG; NSNIKVVSQYVGT;
SNIKVVSQYVGTF; NIKVVSQYVGTFG; IKVVSQYVGTFGD; KVVSQYVGTFGDF;
VVSQYVGTFGDFG; VSQYVGTFGDFGL; SQYVGTFGDFGLG; QYVGTFGDFGLGR;
YVGTFGDFGLGRS; VGTFGDFGLGRST; GTFGDFGLGRSTA; TFGDFGLGRSTAS;
FGDFGLGRSTASN; GDFGLGRSTASNM; DFGLGRSTASNMY; FGLGRSTASNMYR;
GLGRSTASNMYRD; LGRSTASNMYRDG; GRSTASNMYRDGV; RSTASNMYRDGVD;
STASNMYRDGVDI; TASNMYRDGVDII; ASNMYRDGVDIIF; SNMYRDGVDIIFA;
NMYRDGVDIIFAA; MYRDGVDIIFAAA; YRDGVDIIFAAAG; RDGVDIIFAAAGL;
DGVDIIFAAAGLS; GVDIIFAAAGLSG; VDIIFAAAGLSGI; DIIFAAAGLSGIG;
IIFAAAGLSGIGV; IFAAAGLSGIGVI; FAAAGLSGIGVIE; AAAGLSGIGVIEA;
AAGLSGIGVIEAA; AGLSGIGVIEAAK; GLSGIGVIEAAKE; LSGIGVIEAAKEL;
SGIGVIEAAKELG; GIGVIEAAKELGP; IGVIEAAKELGPD; GVIEAAKELGPDH;
VIEAAKELGPDHY; IEAAKELGPDHYI; EAAKELGPDHYII; AAKELGPDHYIIG;
AKELGPDHYIIGV; KELGPDHYIIGVD; ELGPDHYIIGVDQ; LGPDHYIIGVDQD;
GPDHYIIGVDQDQ; PDHYIIGVDQDQS; DHYIIGVDQDQSY; HYIIGVDQDQSYL;
YIIGVDQDQSYLA; IIGVDQDQSYLAP; IGVDQDQSYLAPN; GVDQDQSYLAPNN;
VDQDQSYLAPNNV; DQDQSYLAPNNVI; QDQSYLAPNNVIV; DQSYLAPNNVIVS;
QSYLAPNNVIVSA; SYLAPNNVIVSAV; YLAPNNVIVSAVK; LAPNNVIVSAVKK;
APNNVIVSAVKKV; PNNVIVSAVKKVD; NNVIVSAVKKVDS; NVIVSAVKKVDSL;
VIVSAVKKVDSLM; IVSAVKKVDSLMY; VSAVKKVDSLMYS; SAVKKVDSLMYSL;
AVKKVDSLMYSLT; VKKVDSLMYSLTK; KKVDSLMYSLTKK; KVDSLMYSLTKKY;
VDSLMYSLTKKYL; DSLMYSLTKKYLE; SLMYSLTKKYLET; LMYSLTKKYLETG;
MYSLTKKYLETGV; YSLTKKYLETGVL; SLTKKYLETGVLD; LTKKYLETGVLDG;
TKKYLETGVLDGG; KKYLETGVLDGGK; KYLETGVLDGGKT; YLETGVLDGGKTM;
LETGVLDGGKTMF; ETGVLDGGKTMFL; TGVLDGGKTMFLG; GVLDGGKTMFLGL;
VLDGGKTMFLGLK; LDGGKTMFLGLKE; DGGKTMFLGLKED; GGKTMFLGLKEDG;
GKTMFLGLKEDGL; KTMFLGLKEDGLG; TMFLGLKEDGLGL; MFLGLKEDGLGLV;
FLGLKEDGLGLVL; LGLKEDGLGLVLN; GLKEDGLGLVLNE; LKEDGLGLVLNEN;
KEDGLGLVLNENL; EDGLGLVLNENLK; DGLGLVLNENLKS; GLGLVLNENLKSN;
LGLVLNENLKSNY; GLVLNENLKSNYS; LVLNENLKSNYSE; VLNENLKSNYSEI;
LNENLKSNYSEIY; NENLKSNYSEIYN; ENLKSNYSEIYNK; NLKSNYSEIYNKS;
LKSNYSEIYNKSL; KSNYSEIYNKSLK; SNYSEIYNKSLKI; NYSEIYNKSLKIG;
YSEIYNKSLKIGQ; SEIYNKSLKIGQS; EIYNKSLKIGQSI; IYNKSLKIGQSIM;
YNKSLKIGQSIMN; NKSLKIGQSIMNG; KSLKIGQSIMNGI; SLKIGQSIMNGII;
LKIGQSIMNGIIK; KIGQSIMNGIIKV; IGQSIMNGIIKVP; GQSIMNGIIKVPY;
QSIMNGIIKVPYD; SIMNGIIKVPYDK; IMNGIIKVPYDKV; MNGIIKVPYDKVS;
NGIIKVPYDKVSY; GIIKVPYDKVSYF; IIKVPYDKVSYFV; IKVPYDKVSYFVL;
KVPYDKVSYFVLQ; VPYDKVSYFVLQM; PYDKVSYFVLQME; YDKVSYFVLQMEN;

14 mers:
MDNYYEIYFLYFFI; DNYYEIYFLYFFIK; NYYEIYFLYFFIKS;

927

YYEIYFLYFFIKSK; YEIYFLYFFIKSKE; EIYFLYFFIKSKED;
IYFLYFFIKSKEDI; YFLYFFIKSKEDIF; FLYFFIKSKEDIFM;
LYFFIKSKEDIFML; YFFIKSKEDIFMLK; FFIKSKEDIFMLKK;
FIKSKEDIFMLKKV; IKSKEDIFMLKKVY; KSKEDIFMLKKVYY;
SKEDIFMLKKVYYF; KEDIFMLKKVYYFL; EDIFMLKKVYYFLI;
DIFMLKKVYYFLIF; IFMLKKVYYFLIFL; FMLKKVYYFLIFLF;
MLKKVYYFLIFLFI; LKKVYYFLIFLFIV; KKVYYFLIFLFIVA;
KVYYFLIFLFIVAC; VYYFLIFLFIVACS; YYFLIFLFIVACSS;
YFLIFLFIVACSSS; FLIFLFIVACSSSD; LIFLFIVACSSSDD;
IFLFIVACSSSDDG; FLFIVACSSSDDGK; LFIVACSSSDDGKS;
FIVACSSSDDGKSE; IVACSSSDDGKSEA; VACSSSDDGKSEAK;
ACSSSDDGKSEAKT; CSSSDDGKSEAKTV; SSSDDGKSEAKTVS;
SSDDGKSEAKTVSL; SDDGKSEAKTVSLI; DDGKSEAKTVSLIV;
DGKSEAKTVSLIVD; GKSEAKTVSLIVDG; KSEAKTVSLIVDGA;
SEAKTVSLIVDGAF; EAKTVSLIVDGAFD; AKTVSLIVDGAFDD;
KTVSLIVDGAFDDK; TVSLIVDGAFDDKG; VSLIVDGAFDDKGF;
SLIVDGAFDDKGFN; LIVDGAFDDKGFNE; IVDGAFDDKGFNES;
VDGAFDDKGFNESS; DGAFDDKGFNESSS; GAFDDKGFNESSSK;
AFDDKGFNESSSKA; FDDKGFNESSSKAI; DDKGFNESSSKAIR;
DKGFNESSSKAIRK; KGFNESSSKAIRKL; GFNESSSKAIRKLK;
FNESSSKAIRKLKA; NESSSKAIRKLKAD; ESSSKAIRKLKADL;
SSSKAIRKLKADLN; SSKAIRKLKADLNI; SKAIRKLKADLNIN;
KAIRKLKADLNINI; AIRKLKADLNINII; IRKLKADLNINIIE;
RKLKADLNINIIEK; KLKADLNINIIEKA; LKADLNINIIEKAS;
KADLNINIIEKAST; ADLNINIIEKASTG; DLNINIIEKASTGN;
LNINIIEKASTGNS; NINIIEKASTGNSY; INIIEKASTGNSYL;
NIIEKASTGNSYLG; IIEKASTGNSYLGD; IEKASTGNSYLGDI;
EKASTGNSYLGDIA; KASTGNSYLGDIAN; ASTGNSYLGDIANL;
STGNSYLGDIANLE; TGNSYLGDIANLED; GNSYLGDIANLEDG;
NSYLGDIANLEDGN; SYLGDIANLEDGNS; YLGDIANLEDGNSN;
LGDIANLEDGNSNL; GDIANLEDGNSNLI; DIANLEDGNSNLIW;
IANLEDGNSNLIWG; ANLEDGNSNLIWGI; NLEDGNSNLIWGIG;
LEDGNSNLIWGIGF; EDGNSNLIWGIGFR; DGNSNLIWGIGFRL;
GNSNLIWGIGFRLS; NSNLIWGIGFRLSD; SNLIWGIGFRLSDI;
NLIWGIGFRLSDIL; LIWGIGFRLSDILF; IWGIGFRLSDILFQ;
WGIGFRLSDILFQR; GIGFRLSDILFQRA; IGFRLSDILFQRAS;
GFRLSDILFQRASE; FRLSDILFQRASEN; RLSDILFQRASENV;
LSDILFQRASENVS; SDILFQRASENVSV; DILFQRASENVSVN;
ILFQRASENVSVNY; LFQRASENVSVNYA; FQRASENVSVNYAI;
QRASENVSVNYAII; RASENVSVNYAIIE; ASENVSVNYAIIEG;
SENVSVNYAIIEGV; ENVSVNYAIIEGVY; NVSVNYAIIEGVYD;
VSVNYAIIEGVYDE; SVNYAIIEGVYDEI; VNYAIIEGVYDEIQ;
NYAIIEGVYDEIQI; YAIIEGVYDEIQIP; AIIEGVYDEIQIPK;
IIEGVYDEIQIPKN; IEGVYDEIQIPKNL; EGVYDEIQIPKNLL;
GVYDEIQIPKNLLN; VYDEIQIPKNLLNI; YDEIQIPKNLLNIS;
DEIQIPKNLLNISF; EIQIPKNLLNISFR; IQIPKNLLNISFRS;
QIPKNLLNISFRSE; IPKNLLNISFRSEE; PKNLLNISFRSEEV;
KNLLNISFRSEEVA; NLLNISFRSEEVAF; LLNISFRSEEVAFL;
LNISFRSEEVAFLA; NISFRSEEVAFLAG; ISFRSEEVAFLAGY;
SFRSEEVAFLAGYF; FRSEEVAFLAGYFA; RSEEVAFLAGYFAS;
SEEVAFLAGYFASK; EEVAFLAGYFASKA; EVAFLAGYFASKAS;
VAFLAGYFASKASK; AFLAGYFASKASKT; FLAGYFASKASKTG;
LAGYFASKASKTGK; AGYFASKASKTGKI; GYFASKASKTGKIG;
YFASKASKTGKIGF; FASKASKTGKIGFV; ASKASKTGKIGFVG;
SKASKTGKIGFVGG; KASKTGKIGFVGGV; ASKTGKIGFVGGVR;
SKTGKIGFVGGVRG; KTGKIGFVGGVRGK; TGKIGFVGGVRGKV;
GKIGFVGGVRGKVL; KIGFVGGVRGKVLE; IGFVGGVRGKVLES;

| |
|---|
| GFVGGVRGKVLESF; FVGGVRGKVLESFM; VGGVRGKVLESFMY;<br>GGVRGKVLESFMYG; GVRGKVLESFMYGY; VRGKVLESFMYGYE;<br>RGKVLESFMYGYEA; GKVLESFMYGYEAG; KVLESFMYGYEAGA;<br>VLESFMYGYEAGAK; LESFMYGYEAGAKY; ESFMYGYEAGAKYA;<br>SFMYGYEAGAKYAN; FMYGYEAGAKYANS; MYGYEAGAKYANSN;<br>YGYEAGAKYANSNI; GYEAGAKYANSNIK; YEAGAKYANSNIKV;<br>EAGAKYANSNIKVV; AGAKYANSNIKVVS; GAKYANSNIKVVSQ;<br>AKYANSNIKVVSQY; KYANSNIKVVSQYV; YANSNIKVVSQYVG;<br>ANSNIKVVSQYVGT; NSNIKVVSQYVGTF; SNIKVVSQYVGTFG;<br>NIKVVSQYVGTFGD; IKVVSQYVGTFGDF; KVVSQYVGTFGDFG;<br>VVSQYVGTFGDFGL; VSQYVGTFGDFGLG; SQYVGTFGDFGLGR;<br>QYVGTFGDFGLGRS; YVGTFGDFGLGRST; VGTFGDFGLGRSTA;<br>GTFGDFGLGRSTAS; TFGDFGLGRSTASN; FGDFGLGRSTASNM;<br>GDFGLGRSTASNMY; DFGLGRSTASNMYR; FGLGRSTASNMYRD;<br>GLGRSTASNMYRDG; LGRSTASNMYRDGV; GRSTASNMYRDGVD;<br>RSTASNMYRDGVDI; STASNMYRDGVDII; TASNMYRDGVDIIF;<br>ASNMYRDGVDIIFA; SNMYRDGVDIIFAA; NMYRDGVDIIFAAA;<br>MYRDGVDIIFAAAG; YRDGVDIIFAAAGL; RDGVDIIFAAAGLS;<br>DGVDIIFAAAGLSG; GVDIIFAAAGLSGI; VDIIFAAAGLSGIG;<br>DIIFAAAGLSGIGV; IIFAAAGLSGIGVI; IFAAAGLSGIGVIE;<br>FAAAGLSGIGVIEA; AAAGLSGIGVIEAA; AAGLSGIGVIEAAK;<br>AGLSGIGVIEAAKE; GLSGIGVIEAAKEL; LSGIGVIEAAKELG;<br>SGIGVIEAAKELGP; GIGVIEAAKELGPD; IGVIEAAKELGPDH;<br>GVIEAAKELGPDHY; VIEAAKELGPDHYI; IEAAKELGPDHYII;<br>EAAKELGPDHYIIG; AAKELGPDHYIIGV; AKELGPDHYIIGVD;<br>KELGPDHYIIGVDQ; ELGPDHYIIGVDQD; LGPDHYIIGVDQDQ;<br>GPDHYIIGVDQDQS; PDHYIIGVDQDQSY; DHYIIGVDQDQSYL;<br>HYIIGVDQDQSYLA; YIIGVDQDQSYLAP; IIGVDQDQSYLAPN;<br>IGVDQDQSYLAPNN; GVDQDQSYLAPNNV; VDQDQSYLAPNNVI;<br>DQDQSYLAPNNVIV; QDQSYLAPNNVIVS; DQSYLAPNNVIVSA;<br>QSYLAPNNVIVSAV; SYLAPNNVIVSAVK; YLAPNNVIVSAVKK;<br>LAPNNVIVSAVKKV; APNNVIVSAVKKVD; PNNVIVSAVKKVDS;<br>NNVIVSAVKKVDSL; NVIVSAVKKVDSLM; VIVSAVKKVDSLMY;<br>IVSAVKKVDSLMYS; VSAVKKVDSLMYSL; SAVKKVDSLMYSLT;<br>AVKKVDSLMYSLTK; VKKVDSLMYSLTKK; KKVDSLMYSLTKKY;<br>KVDSLMYSLTKKYL; VDSLMYSLTKKYLE; DSLMYSLTKKYLET;<br>SLMYSLTKKYLETG; LMYSLTKKYLETGV; MYSLTKKYLETGVL;<br>YSLTKKYLETGVLD; SLTKKYLETGVLDG; LTKKYLETGVLDGG;<br>TKKYLETGVLDGGK; KKYLETGVLDGGKT; KYLETGVLDGGKTM;<br>YLETGVLDGGKTMF; LETGVLDGGKTMFL; ETGVLDGGKTMFLG;<br>TGVLDGGKTMFLGL; GVLDGGKTMFLGLK; VLDGGKTMFLGLKE;<br>LDGGKTMFLGLKED; DGGKTMFLGLKEDG; GGKTMFLGLKEDGL;<br>GKTMFLGLKEDGLG; KTMFLGLKEDGLGL; TMFLGLKEDGLGLV;<br>MFLGLKEDGLGLVL; FLGLKEDGLGLVLN; LGLKEDGLGLVLNE;<br>GLKEDGLGLVLNEN; LKEDGLGLVLNENL; KEDGLGLVLNENLK;<br>EDGLGLVLNENLKS; DGLGLVLNENLKSN; GLGLVLNENLKSNY;<br>LGLVLNENLKSNYS; GLVLNENLKSNYSE; LVLNENLKSNYSEI;<br>VLNENLKSNYSEIY; LNENLKSNYSEIYN; NENLKSNYSEIYNK;<br>ENLKSNYSEIYNKS; NLKSNYSEIYNKSL; LKSNYSEIYNKSLK;<br>KSNYSEIYNKSLKI; SNYSEIYNKSLKIG; NYSEIYNKSLKIGQ;<br>YSEIYNKSLKIGQS; SEIYNKSLKIGQSI; EIYNKSLKIGQSIM;<br>IYNKSLKIGQSIMN; YNKSLKIGQSIMNG; NKSLKIGQSIMNGI;<br>KSLKIGQSIMNGII; SLKIGQSIMNGIIK; LKIGQSIMNGIIKV;<br>KIGQSIMNGIIKVP; IGQSIMNGIIKVPY; GQSIMNGIIKVPYD;<br>QSIMNGIIKVPYDK; SIMNGIIKVPYDKV; IMNGIIKVPYDKVS;<br>MNGIIKVPYDKVSY; NGIIKVPYDKVSYF; GIIKVPYDKVSYFV;<br>IIKVPYDKVSYFVL; IKVPYDKVSYFVLQ; KVPYDKVSYFVLQM; |

VPYDKVSYFVLQME; PYDKVSYFVLQMEN;

15 mers:
MDNYYEIYFLYFFIK; DNYYEIYFLYFFIKS; NYYEIYFLYFFIKSK;
YYEIYFLYFFIKSKE; YEIYFLYFFIKSKED; EIYFLYFFIKSKEDI;
IYFLYFFIKSKEDIF; YFLYFFIKSKEDIFM; FLYFFIKSKEDIFML;
LYFFIKSKEDIFMLK; YFFIKSKEDIFMLKK; FFIKSKEDIFMLKKV;
FIKSKEDIFMLKKVY; IKSKEDIFMLKKVYY; KSKEDIFMLKKVYYF;
SKEDIFMLKKVYYFL; KEDIFMLKKVYYFLI; EDIFMLKKVYYFLIF;
DIFMLKKVYYFLIFL; IFMLKKVYYFLIFLF; FMLKKVYYFLIFLFI;
MLKKVYYFLIFLFIV; LKKVYYFLIFLFIVA; KKVYYFLIFLFIVAC;
KVYYFLIFLFIVACS; VYYFLIFLFIVACSS; YYFLIFLFIVACSSS;
YFLIFLFIVACSSSD; FLIFLFIVACSSSDD; LIFLFIVACSSSDDG;
IFLFIVACSSSDDGK; FLFIVACSSSDDGKS; LFIVACSSSDDGKSE;
FIVACSSSDDGKSEA; IVACSSSDDGKSEAK; VACSSSDDGKSEAKT;
ACSSSDDGKSEAKTV; CSSSDDGKSEAKTVS; SSSDDGKSEAKTVSL;
SSDDGKSEAKTVSLI; SDDGKSEAKTVSLIV; DDGKSEAKTVSLIVD;
DGKSEAKTVSLIVDG; GKSEAKTVSLIVDGA; KSEAKTVSLIVDGAF;
SEAKTVSLIVDGAFD; EAKTVSLIVDGAFDD; AKTVSLIVDGAFDDK;
KTVSLIVDGAFDDKG; TVSLIVDGAFDDKGF; VSLIVDGAFDDKGFN;
SLIVDGAFDDKGFNE; LIVDGAFDDKGFNES; IVDGAFDDKGFNESS;
VDGAFDDKGFNESSS; DGAFDDKGFNESSSK; GAFDDKGFNESSSKA;
AFDDKGFNESSSKAI; FDDKGFNESSSKAIR; DDKGFNESSSKAIRK;
DKGFNESSSKAIRKL; KGFNESSSKAIRKLK; GFNESSSKAIRKLKA;
FNESSSKAIRKLKAD; NESSSKAIRKLKADL; ESSSKAIRKLKADLN;
SSSKAIRKLKADLNI; SSKAIRKLKADLNIN; SKAIRKLKADLNINI;
KAIRKLKADLNINII; AIRKLKADLNINIIE; IRKLKADLNINIIEK;
RKLKADLNINIIEKA; KLKADLNINIIEKAS; LKADLNINIIEKAST;
KADLNINIIEKASTG; ADLNINIIEKASTGN; DLNINIIEKASTGNS;
LNINIIEKASTGNSY; NINIIEKASTGNSYL; INIIEKASTGNSYLG;
NIIEKASTGNSYLGD; IIEKASTGNSYLGDI; IEKASTGNSYLGDIA;
EKASTGNSYLGDIAN; KASTGNSYLGDIANL; ASTGNSYLGDIANLE;
STGNSYLGDIANLED; TGNSYLGDIANLEDG; GNSYLGDIANLEDGN;
NSYLGDIANLEDGNS; SYLGDIANLEDGNSN; YLGDIANLEDGNSNL;
LGDIANLEDGNSNLI; GDIANLEDGNSNLIW; DIANLEDGNSNLIWG;
IANLEDGNSNLIWGI; ANLEDGNSNLIWGIG; NLEDGNSNLIWGIGF;
LEDGNSNLIWGIGFR; EDGNSNLIWGIGFRL; DGNSNLIWGIGFRLS;
GNSNLIWGIGFRLSD; NSNLIWGIGFRLSDI; SNLIWGIGFRLSDIL;
NLIWGIGFRLSDILF; LIWGIGFRLSDILFQ; IWGIGFRLSDILFQR;
WGIGFRLSDILFQRA; GIGFRLSDILFQRAS; IGFRLSDILFQRASE;
GFRLSDILFQRASEN; FRLSDILFQRASENV; RLSDILFQRASENVS;
LSDILFQRASENVSV; SDILFQRASENVSVN; DILFQRASENVSVNY;
ILFQRASENVSVNYA; LFQRASENVSVNYAI; FQRASENVSVNYAII;
QRASENVSVNYAIIE; RASENVSVNYAIIEG; ASENVSVNYAIIEGV;
SENVSVNYAIIEGVY; ENVSVNYAIIEGVYD; NVSVNYAIIEGVYDE;
VSVNYAIIEGVYDEI; SVNYAIIEGVYDEIQ; VNYAIIEGVYDEIQI;
NYAIIEGVYDEIQIP; YAIIEGVYDEIQIPK; AIIEGVYDEIQIPKN;
IIEGVYDEIQIPKNL; IEGVYDEIQIPKNLL; EGVYDEIQIPKNLLN;
GVYDEIQIPKNLLNI; VYDEIQIPKNLLNIS; YDEIQIPKNLLNISF;
DEIQIPKNLLNISFR; EIQIPKNLLNISFRS; IQIPKNLLNISFRSE;
QIPKNLLNISFRSEE; IPKNLLNISFRSEEV; PKNLLNISFRSEEVA;
KNLLNISFRSEEVAF; NLLNISFRSEEVAFL; LLNISFRSEEVAFLA;
LNISFRSEEVAFLAG; NISFRSEEVAFLAGY; ISFRSEEVAFLAGYF;
SFRSEEVAFLAGYFA; FRSEEVAFLAGYFAS; RSEEVAFLAGYFASK;
SEEVAFLAGYFASKA; EEVAFLAGYFASKAS; EVAFLAGYFASKASK;
VAFLAGYFASKASKT; AFLAGYFASKASKTG; FLAGYFASKASKTGK;
LAGYFASKASKTGKI; AGYFASKASKTGKIG; GYFASKASKTGKIGF;

YFASKASKTGKIGFV; FASKASKTGKIGFVG; ASKASKTGKIGFVGG;
SKASKTGKIGFVGGV; KASKTGKIGFVGGVR; ASKTGKIGFVGGVRG;
SKTGKIGFVGGVRGK; KTGKIGFVGGVRGKV; TGKIGFVGGVRGKVL;
GKIGFVGGVRGKVLE; KIGFVGGVRGKVLES; IGFVGGVRGKVLESF;
GFVGGVRGKVLESFM; FVGGVRGKVLESFMY; VGGVRGKVLESFMYG;
GGVRGKVLESFMYGY; GVRGKVLESFMYGYE; VRGKVLESFMYGYEA;
RGKVLESFMYGYEAG; GKVLESFMYGYEAGA; KVLESFMYGYEAGAK;
VLESFMYGYEAGAKY; LESFMYGYEAGAKYA; ESFMYGYEAGAKYAN;
SFMYGYEAGAKYANS; FMYGYEAGAKYANSN; MYGYEAGAKYANSNI;
YGYEAGAKYANSNIK; GYEAGAKYANSNIKV; YEAGAKYANSNIKVV;
EAGAKYANSNIKVVS; AGAKYANSNIKVVSQ; GAKYANSNIKVVSQY;
AKYANSNIKVVSQYV; KYANSNIKVVSQYVG; YANSNIKVVSQYVGT;
ANSNIKVVSQYVGTF; NSNIKVVSQYVGTFG; SNIKVVSQYVGTFGD;
NIKVVSQYVGTFGDF; IKVVSQYVGTFGDFG; KVVSQYVGTFGDFGL;
VVSQYVGTFGDFGLG; VSQYVGTFGDFGLGR; SQYVGTFGDFGLGRS;
QYVGTFGDFGLGRST; YVGTFGDFGLGRSTA; VGTFGDFGLGRSTAS;
GTFGDFGLGRSTASN; TFGDFGLGRSTASNM; FGDFGLGRSTASNMY;
GDFGLGRSTASNMYR; DFGLGRSTASNMYRD; FGLGRSTASNMYRDG;
GLGRSTASNMYRDGV; LGRSTASNMYRDGVD; GRSTASNMYRDGVDI;
RSTASNMYRDGVDII; STASNMYRDGVDIIF; TASNMYRDGVDIIFA;
ASNMYRDGVDIIFAA; SNMYRDGVDIIFAAA; NMYRDGVDIIFAAAG;
MYRDGVDIIFAAAGL; YRDGVDIIFAAAGLS; RDGVDIIFAAAGLSG;
DGVDIIFAAAGLSGI; GVDIIFAAAGLSGIG; VDIIFAAAGLSGIGV;
DIIFAAAGLSGIGVI; IIFAAAGLSGIGVIE; IFAAAGLSGIGVIEA;
FAAAGLSGIGVIEAA; AAAGLSGIGVIEAAK; AAGLSGIGVIEAAKE;
AGLSGIGVIEAAKEL; GLSGIGVIEAAKELG; LSGIGVIEAAKELGP;
SGIGVIEAAKELGPD; GIGVIEAAKELGPDH; IGVIEAAKELGPDHY;
GVIEAAKELGPDHYI; VIEAAKELGPDHYII; IEAAKELGPDHYIIG;
EAAKELGPDHYIIGV; AAKELGPDHYIIGVD; AKELGPDHYIIGVDQ;
KELGPDHYIIGVDQD; ELGPDHYIIGVDQDQ; LGPDHYIIGVDQDQS;
GPDHYIIGVDQDQSY; PDHYIIGVDQDQSYL; DHYIIGVDQDQSYLA;
HYIIGVDQDQSYLAP; YIIGVDQDQSYLAPN; IIGVDQDQSYLAPNN;
IGVDQDQSYLAPNNV; GVDQDQSYLAPNNVI; VDQDQSYLAPNNVIV;
DQDQSYLAPNNVIVS; QDQSYLAPNNVIVSA; DQSYLAPNNVIVSAV;
QSYLAPNNVIVSAVK; SYLAPNNVIVSAVKK; YLAPNNVIVSAVKKV;
LAPNNVIVSAVKKVD; APNNVIVSAVKKVDS; PNNVIVSAVKKVDSL;
NNVIVSAVKKVDSLM; NVIVSAVKKVDSLMY; VIVSAVKKVDSLMYS;
IVSAVKKVDSLMYSL; VSAVKKVDSLMYSLT; SAVKKVDSLMYSLTK;
AVKKVDSLMYSLTKK; VKKVDSLMYSLTKKY; KKVDSLMYSLTKKYL;
KVDSLMYSLTKKYLE; VDSLMYSLTKKYLET; DSLMYSLTKKYLETG;
SLMYSLTKKYLETGV; LMYSLTKKYLETGVL; MYSLTKKYLETGVLD;
YSLTKKYLETGVLDG; SLTKKYLETGVLDGG; LTKKYLETGVLDGGK;
TKKYLETGVLDGGKT; KKYLETGVLDGGKTM; KYLETGVLDGGKTMF;
YLETGVLDGGKTMFL; LETGVLDGGKTMFLG; ETGVLDGGKTMFLGL;
TGVLDGGKTMFLGLK; GVLDGGKTMFLGLKE; VLDGGKTMFLGLKED;
LDGGKTMFLGLKEDG; DGGKTMFLGLKEDGL; GGKTMFLGLKEDGLG;
GKTMFLGLKEDGLGL; KTMFLGLKEDGLGLV; TMFLGLKEDGLGLVL;
MFLGLKEDGLGLVLN; FLGLKEDGLGLVLNE; LGLKEDGLGLVLNEN;
GLKEDGLGLVLNENL; LKEDGLGLVLNENLK; KEDGLGLVLNENLKS;
EDGLGLVLNENLKSN; DGLGLVLNENLKSNY; GLGLVLNENLKSNYS;
LGLVLNENLKSNYSE; GLVLNENLKSNYSEI; LVLNENLKSNYSEIY;
VLNENLKSNYSEIYN; LNENLKSNYSEIYNK; NENLKSNYSEIYNKS;
ENLKSNYSEIYNKSL; NLKSNYSEIYNKSLK; LKSNYSEIYNKSLKI;
KSNYSEIYNKSLKIG; SNYSEIYNKSLKIGQ; NYSEIYNKSLKIGQS;
YSEIYNKSLKIGQSI; SEIYNKSLKIGQSIM; EIYNKSLKIGQSIMN;
IYNKSLKIGQSIMNG; YNKSLKIGQSIMNGI; NKSLKIGQSIMNGII;
KSLKIGQSIMNGIIK; SLKIGQSIMNGIIKV; LKIGQSIMNGIIKVP;

KIGQSIMNGIIKVPY; IGQSIMNGIIKVPYD; GQSIMNGIIKVPYDK;
QSIMNGIIKVPYDKV; SIMNGIIKVPYDKVS; IMNGIIKVPYDKVSY;
MNGIIKVPYDKVSYF; NGIIKVPYDKVSYFV; GIIKVPYDKVSYFVL;
IIKVPYDKVSYFVLQ; IKVPYDKVSYFVLQM; KVPYDKVSYFVLQME;
VPYDKVSYFVLQMEN;

16 mers:
MDNYYEIYFLYFFIKS; DNYYEIYFLYFFIKSK; NYYEIYFLYFFIKSKE;
YYEIYFLYFFIKSKED; YEIYFLYFFIKSKEDI; EIYFLYFFIKSKEDIF;
IYFLYFFIKSKEDIFM; YFLYFFIKSKEDIFML; FLYFFIKSKEDIFMLK;
LYFFIKSKEDIFMLKK; YFFIKSKEDIFMLKKV; FFIKSKEDIFMLKKVY;
FIKSKEDIFMLKKVYY; IKSKEDIFMLKKVYYF; KSKEDIFMLKKVYYFL;
SKEDIFMLKKVYYFLI; KEDIFMLKKVYYFLIF; EDIFMLKKVYYFLIFL;
DIFMLKKVYYFLIFLF; IFMLKKVYYFLIFLFI; FMLKKVYYFLIFLFIV;
MLKKVYYFLIFLFIVA; LKKVYYFLIFLFIVAC; KKVYYFLIFLFIVACS;
KVYYFLIFLFIVACSS; VYYFLIFLFIVACSSS; YYFLIFLFIVACSSSD;
YFLIFLFIVACSSSDD; FLIFLFIVACSSSDDG; LIFLFIVACSSSDDGK;
IFLFIVACSSSDDGKS; FLFIVACSSSDDGKSE; LFIVACSSSDDGKSEA;
FIVACSSSDDGKSEAK; IVACSSSDDGKSEAKT; VACSSSDDGKSEAKTV;
ACSSSDDGKSEAKTVS; CSSSDDGKSEAKTVSL; SSSDDGKSEAKTVSLI;
SSDDGKSEAKTVSLIV; SDDGKSEAKTVSLIVD; DDGKSEAKTVSLIVDG;
DGKSEAKTVSLIVDGA; GKSEAKTVSLIVDGAF; KSEAKTVSLIVDGAFD;
SEAKTVSLIVDGAFDD; EAKTVSLIVDGAFDDK; AKTVSLIVDGAFDDKG;
KTVSLIVDGAFDDKGF; TVSLIVDGAFDDKGFN; VSLIVDGAFDDKGFNE;
SLIVDGAFDDKGFNES; LIVDGAFDDKGFNESS; IVDGAFDDKGFNESSS;
VDGAFDDKGFNESSSK; DGAFDDKGFNESSSKA; GAFDDKGFNESSSKAI;
AFDDKGFNESSSKAIR; FDDKGFNESSSKAIRK; DDKGFNESSSKAIRKL;
DKGFNESSSKAIRKLK; KGFNESSSKAIRKLKA; GFNESSSKAIRKLKAD;
FNESSSKAIRKLKADL; NESSSKAIRKLKADLN; ESSSKAIRKLKADLNI;
SSSKAIRKLKADLNIN; SSKAIRKLKADLNINI; SKAIRKLKADLNINII;
KAIRKLKADLNINIIE; AIRKLKADLNINIIEK; IRKLKADLNINIIEKA;
RKLKADLNINIIEKAS; KLKADLNINIIEKAST; LKADLNINIIEKASTG;
KADLNINIIEKASTGN; ADLNINIIEKASTGNS; DLNINIIEKASTGNSY;
LNINIIEKASTGNSYL; NINIIEKASTGNSYLG; INIIEKASTGNSYLGD;
NIIEKASTGNSYLGDI; IIEKASTGNSYLGDIA; IEKASTGNSYLGDIAN;
EKASTGNSYLGDIANL; KASTGNSYLGDIANLE; ASTGNSYLGDIANLED;
STGNSYLGDIANLEDG; TGNSYLGDIANLEDGN; GNSYLGDIANLEDGNS;
NSYLGDIANLEDGNSN; SYLGDIANLEDGNSNL; YLGDIANLEDGNSNLI;
LGDIANLEDGNSNLIW; GDIANLEDGNSNLIWG; DIANLEDGNSNLIWGI;
IANLEDGNSNLIWGIG; ANLEDGNSNLIWGIGF; NLEDGNSNLIWGIGFR;
LEDGNSNLIWGIGFRL; EDGNSNLIWGIGFRLS; DGNSNLIWGIGFRLSD;
GNSNLIWGIGFRLSDI; NSNLIWGIGFRLSDIL; SNLIWGIGFRLSDILF;
NLIWGIGFRLSDILFQ; LIWGIGFRLSDILFQR; IWGIGFRLSDILFQRA;
WGIGFRLSDILFQRAS; GIGFRLSDILFQRASE; IGFRLSDILFQRASEN;
GFRLSDILFQRASENV; FRLSDILFQRASENVS; RLSDILFQRASENVSV;
LSDILFQRASENVSVN; SDILFQRASENVSVNY; DILFQRASENVSVNYA;
ILFQRASENVSVNYAI; LFQRASENVSVNYAII; FQRASENVSVNYAIIE;
QRASENVSVNYAIIEG; RASENVSVNYAIIEGV; ASENVSVNYAIIEGVY;
SENVSVNYAIIEGVYD; ENVSVNYAIIEGVYDE; NVSVNYAIIEGVYDEI;
VSVNYAIIEGVYDEIQ; SVNYAIIEGVYDEIQI; VNYAIIEGVYDEIQIP;
NYAIIEGVYDEIQIPK; YAIIEGVYDEIQIPKN; AIIEGVYDEIQIPKNL;
IIEGVYDEIQIPKNLL; IEGVYDEIQIPKNLLN; EGVYDEIQIPKNLLNI;
GVYDEIQIPKNLLNIS; VYDEIQIPKNLLNISF; YDEIQIPKNLLNISFR;
DEIQIPKNLLNISFRS; EIQIPKNLLNISFRSE; IQIPKNLLNISFRSEE;
QIPKNLLNISFRSEEV; IPKNLLNISFRSEEVA; PKNLLNISFRSEEVAF;
KNLLNISFRSEEVAFL; NLLNISFRSEEVAFLA; LLNISFRSEEVAFLAG;
LNISFRSEEVAFLAGY; NISFRSEEVAFLAGYF; ISFRSEEVAFLAGYFA;

| | |
|---|---|
| SFRSEEVAFLAGYFAS; FRSEEVAFLAGYFASK; RSEEVAFLAGYFASKA; | |
| SEEVAFLAGYFASKAS; EEVAFLAGYFASKASK; EVAFLAGYFASKASKT; | |
| VAFLAGYFASKASKTG; AFLAGYFASKASKTGK; FLAGYFASKASKTGKI; | |
| LAGYFASKASKTGKIG; AGYFASKASKTGKIGF; GYFASKASKTGKIGFV; | |
| YFASKASKTGKIGFVG; FASKASKTGKIGFVGG; ASKASKTGKIGFVGGV; | |
| SKASKTGKIGFVGGVR; KASKTGKIGFVGGVRG; ASKTGKIGFVGGVRGK; | |
| SKTGKIGFVGGVRGKV; KTGKIGFVGGVRGKVL; TGKIGFVGGVRGKVLE; | |
| GKIGFVGGVRGKVLES; KIGFVGGVRGKVLESF; IGFVGGVRGKVLESFM; | |
| GFVGGVRGKVLESFMY; FVGGVRGKVLESFMYG; VGGVRGKVLESFMYGY; | |
| GGVRGKVLESFMYGYE; GVRGKVLESFMYGYEA; VRGKVLESFMYGYEAG; | |
| RGKVLESFMYGYEAGA; GKVLESFMYGYEAGAK; KVLESFMYGYEAGAKY; | |
| VLESFMYGYEAGAKYA; LESFMYGYEAGAKYAN; ESFMYGYEAGAKYANS; | |
| SFMYGYEAGAKYANSN; FMYGYEAGAKYANSNI; MYGYEAGAKYANSNIK; | |
| YGYEAGAKYANSNIKV; GYEAGAKYANSNIKVV; YEAGAKYANSNIKVVS; | |
| EAGAKYANSNIKVVSQ; AGAKYANSNIKVVSQY; GAKYANSNIKVVSQYV; | |
| AKYANSNIKVVSQYVG; KYANSNIKVVSQYVGT; YANSNIKVVSQYVGTF; | |
| ANSNIKVVSQYVGTFG; NSNIKVVSQYVGTFGD; SNIKVVSQYVGTFGDF; | |
| NIKVVSQYVGTFGDFG; IKVVSQYVGTFGDFGL; KVVSQYVGTFGDFGLG; | |
| VVSQYVGTFGDFGLGR; VSQYVGTFGDFGLGRS; SQYVGTFGDFGLGRST; | |
| QYVGTFGDFGLGRSTA; YVGTFGDFGLGRSTAS; VGTFGDFGLGRSTASN; | |
| GTFGDFGLGRSTASNM; TFGDFGLGRSTASNMY; FGDFGLGRSTASNMYR; | |
| GDFGLGRSTASNMYRD; DFGLGRSTASNMYRDG; FGLGRSTASNMYRDGV; | |
| GLGRSTASNMYRDGVD; LGRSTASNMYRDGVDI; GRSTASNMYRDGVDII; | |
| RSTASNMYRDGVDIIF; STASNMYRDGVDIIFA; TASNMYRDGVDIIFAA; | |
| ASNMYRDGVDIIFAAA; SNMYRDGVDIIFAAAG; NMYRDGVDIIFAAAGL; | |
| MYRDGVDIIFAAAGLS; YRDGVDIIFAAAGLSG; RDGVDIIFAAAGLSGI; | |
| DGVDIIFAAAGLSGIG; GVDIIFAAAGLSGIGV; VDIIFAAAGLSGIGVI; | |
| DIIFAAAGLSGIGVIE; IIFAAAGLSGIGVIEA; IFAAAGLSGIGVIEAA; | |
| FAAAGLSGIGVIEAAK; AAAGLSGIGVIEAAKE; AAGLSGIGVIEAAKEL; | |
| AGLSGIGVIEAAKELG; GLSGIGVIEAAKELGP; LSGIGVIEAAKELGPD; | |
| SGIGVIEAAKELGPDH; GIGVIEAAKELGPDHY; IGVIEAAKELGPDHYI; | |
| GVIEAAKELGPDHYII; VIEAAKELGPDHYIIG; IEAAKELGPDHYIIGV; | |
| EAAKELGPDHYIIGVD; AAKELGPDHYIIGVDQ; AKELGPDHYIIGVDQD; | |
| KELGPDHYIIGVDQDQ; ELGPDHYIIGVDQDQS; LGPDHYIIGVDQDQSY; | |
| GPDHYIIGVDQDQSYL; PDHYIIGVDQDQSYLA; DHYIIGVDQDQSYLAP; | |
| HYIIGVDQDQSYLAPN; YIIGVDQDQSYLAPNN; IIGVDQDQSYLAPNNV; | |
| IGVDQDQSYLAPNNVI; GVDQDQSYLAPNNVIV; VDQDQSYLAPNNVIVS; | |
| DQDQSYLAPNNVIVSA; QDQSYLAPNNVIVSAV; DQSYLAPNNVIVSAVK; | |
| QSYLAPNNVIVSAVKK; SYLAPNNVIVSAVKKV; YLAPNNVIVSAVKKVD; | |
| LAPNNVIVSAVKKVDS; APNNVIVSAVKKVDSL; PNNVIVSAVKKVDSLM; | |
| NNVIVSAVKKVDSLMY; NVIVSAVKKVDSLMYS; VIVSAVKKVDSLMYSL; | |
| IVSAVKKVDSLMYSLT; VSAVKKVDSLMYSLTK; SAVKKVDSLMYSLTKK; | |
| AVKKVDSLMYSLTKKY; VKKVDSLMYSLTKKYL; KKVDSLMYSLTKKYLE; | |
| KVDSLMYSLTKKYLET; VDSLMYSLTKKYLETG; DSLMYSLTKKYLETGV; | |
| SLMYSLTKKYLETGVL; LMYSLTKKYLETGVLD; MYSLTKKYLETGVLDG; | |
| YSLTKKYLETGVLDGG; SLTKKYLETGVLDGGK; LTKKYLETGVLDGGKT; | |
| TKKYLETGVLDGGKTM; KKYLETGVLDGGKTMF; KYLETGVLDGGKTMFL; | |
| YLETGVLDGGKTMFLG; LETGVLDGGKTMFLGL; ETGVLDGGKTMFLGLK; | |
| TGVLDGGKTMFLGLKE; GVLDGGKTMFLGLKED; VLDGGKTMFLGLKEDG; | |
| LDGGKTMFLGLKEDGL; DGGKTMFLGLKEDGLG; GGKTMFLGLKEDGLGL; | |
| GKTMFLGLKEDGLGLV; KTMFLGLKEDGLGLVL; TMFLGLKEDGLGLVLN; | |
| MFLGLKEDGLGLVLNE; FLGLKEDGLGLVLNEN; LGLKEDGLGLVLNENL; | |
| GLKEDGLGLVLNENLK; LKEDGLGLVLNENLKS; KEDGLGLVLNENLKSN; | |
| EDGLGLVLNENLKSNY; DGLGLVLNENLKSNYS; GLGLVLNENLKSNYSE; | |
| LGLVLNENLKSNYSEI; GLVLNENLKSNYSEIY; LVLNENLKSNYSEIYN; | |
| VLNENLKSNYSEIYNK; LNENLKSNYSEIYNKS; NENLKSNYSEIYNKSL; | |
| ENLKSNYSEIYNKSLK; NLKSNYSEIYNKSLKI; LKSNYSEIYNKSLKIG; | |

| | |
|---|---|
| | KSNYSEIYNKSLKIGQ; SNYSEIYNKSLKIGQS; NYSEIYNKSLKIGQSI; YSEIYNKSLKIGQSIM; SEIYNKSLKIGQSIMN; EIYNKSLKIGQSIMNG; IYNKSLKIGQSIMNGI; YNKSLKIGQSIMNGII; NKSLKIGQSIMNGIIK; KSLKIGQSIMNGIIKV; SLKIGQSIMNGIIKVP; LKIGQSIMNGIIKVPY; KIGQSIMNGIIKVPYD; IGQSIMNGIIKVPYDK; GQSIMNGIIKVPYDKV; QSIMNGIIKVPYDKVS; SIMNGIIKVPYDKVSY; IMNGIIKVPYDKVSYF; MNGIIKVPYDKVSYFV; NGIIKVPYDKVSYFVL; GIIKVPYDKVSYFVLQ; IIKVPYDKVSYFVLQM; IKVPYDKVSYFVLQME; KVPYDKVSYFVLQMEN; |
| AAC70056.1 Decorin binding protein A; DbpA [Borrelia afzelii | 13 mers: MIKYNKIILTLTL; IKYNKIILTLTLL; KYNKIILTLTLLA; YNKIILTLTLLAS; NKIILTLTLLASL; KIILTLTLLASLL; IILTLTLLASLLA; ILTLTLLASLLAA; LTLTLLASLLAAC; TLTLLASLLAACS; LTLLASLLAACSL; TLLASLLAACSLT; LLASLLAACSLTG; LASLLAACSLTGK; ASLLAACSLTGKA; SLLAACSLTGKAR; LLAACSLTGKARL; LAACSLTGKARLE; AACSLTGKARLES; ACSLTGKARLESS; CSLTGKARLESSV; SLTGKARLESSVK; LTGKARLESSVKD; TGKARLESSVKDI; GKARLESSVKDIT; KARLESSVKDITN; ARLESSVKDITNE; RLESSVKDITNEI; LESSVKDITNEIE; ESSVKDITNEIEK; SSVKDITNEIEKA; SVKDITNEIEKAI; VKDITNEIEKAIK; KDITNEIEKAIKE; DITNEIEKAIKEA; ITNEIEKAIKEAE; TNEIEKAIKEAED; NEIEKAIKEAEDA; EIEKAIKEAEDAG; IEKAIKEAEDAGV; EKAIKEAEDAGVK; KAIKEAEDAGVKT; AIKEAEDAGVKTD; IKEAEDAGVKTDA; KEAEDAGVKTDAF; EAEDAGVKTDAFT; AEDAGVKTDAFTE; EDAGVKTDAFTET; DAGVKTDAFTETQ; AGVKTDAFTETQT; GVKTDAFTETQTG; VKTDAFTETQTGG; KTDAFTETQTGGK; TDAFTETQTGGKV; DAFTETQTGGKVG; AFTETQTGGKVGG; FTETQTGGKVGGS; TETQTGGKVGGSQ; ETQTGGKVGGSQI; TQTGGKVGGSQIR; QTGGKVGGSQIRA; TGGKVGGSQIRAA; GGKVGGSQIRAAK; GKVGGSQIRAAKI; KVGGSQIRAAKIR; VGGSQIRAAKIRV; GGSQIRAAKIRVA; GSQIRAAKIRVAD; SQIRAAKIRVADL; QIRAAKIRVADLT; IRAAKIRVADLTI; RAAKIRVADLTIK; AAKIRVADLTIKF; AKIRVADLTIKFL; KIRVADLTIKFLE; IRVADLTIKFLEA; RVADLTIKFLEAT; VADLTIKFLEATE; ADLTIKFLEATEE; DLTIKFLEATEEE; LTIKFLEATEEET; TIKFLEATEEETI; IKFLEATEEETIT; KFLEATEEETITF; FLEATEEETITFK; LEATEEETITFKE; EATEEETITFKEN; ATEEETITFKENG; TEEETITFKENGA; EEETITFKENGAG; EETITFKENGAGE; ETITFKENGAGEE; TITFKENGAGEED; ITFKENGAGEEDF; TFKENGAGEEDFS; FKENGAGEEDFSG; KENGAGEEDFSGI; ENGAGEEDFSGIY; NGAGEEDFSGIYD; GAGEEDFSGIYDL; AGEEDFSGIYDLI; GEEDFSGIYDLIL; EEDFSGIYDLILN; EDFSGIYDLILNA; DFSGIYDLILNAA; FSGIYDLILNAAK; SGIYDLILNAAKA; GIYDLILNAAKAV; IYDLILNAAKAVE; YDLILNAAKAVEK; DLILNAAKAVEKI; LILNAAKAVEKIG; ILNAAKAVEKIGM; LNAAKAVEKIGMQ; NAAKAVEKIGMQG; AAKAVEKIGMQGM; AKAVEKIGMQGMK; KAVEKIGMQGMKQ; AVEKIGMQGMKQA; VEKIGMQGMKQAV; EKIGMQGMKQAVE; KIGMQGMKQAVEE; IGMQGMKQAVEEA; GMQGMKQAVEEAA; MQGMKQAVEEAAK; QGMKQAVEEAAKE; GMKQAVEEAAKEK; MKQAVEEAAKEKP; KQAVEEAAKEKPK; QAVEEAAKEKPKT; AVEEAAKEKPKTT; VEEAAKEKPKTTA; EEAAKEKPKTTAD; EAAKEKPKTTADG; AAKEKPKTTADGI; AKEKPKTTADGII; KEKPKTTADGIIA; EKPKTTADGIIAI; KPKTTADGIIAIV; PKTTADGIIAIVK; KTTADGIIAIVKV; TTADGIIAIVKVM; TADGIIAIVKVMK; ADGIIAIVKVMKA; DGIIAIVKVMKAK; GIIAIVKVMKAKV; IIAIVKVMKAKVE; IAIVKVMKAKVEN; AIVKVMKAKVENI; IVKVMKAKVENIK; VKVMKAKVENIKE; KVMKAKVENIKEK; VMKAKVENIKEKQ; MKAKVENIKEKQT; KAKVENIKEKQTK; AKVENIKEKQTKN; KVENIKEKQTKNQ; VENIKEKQTKNQK;<br><br>14 mers: MIKYNKIILTLTLL; IKYNKIILTLTLLA; KYNKIILTLTLLAS; YNKIILTLTLLASL; NKIILTLTLLASLL; KIILTLTLLASLLA; IILTLTLLASLLAA; ILTLTLLASLLAAC; LTLTLLASLLAACS; TLTLLASLLAACSL; LTLLASLLAACSLT; TLLASLLAACSLTG; |

LLASLLAACSLTGK; LASLLAACSLTGKA; ASLLAACSLTGKAR;
SLLAACSLTGKARL; LLAACSLTGKARLE; LAACSLTGKARLES;
AACSLTGKARLESS; ACSLTGKARLESSV; CSLTGKARLESSVK;
SLTGKARLESSVKD; LTGKARLESSVKDI; TGKARLESSVKDIT;
GKARLESSVKDITN; KARLESSVKDITNE; ARLESSVKDITNEI;
RLESSVKDITNEIE; LESSVKDITNEIEK; ESSVKDITNEIEKA;
SSVKDITNEIEKAI; SVKDITNEIEKAIK; VKDITNEIEKAIKE;
KDITNEIEKAIKEA; DITNEIEKAIKEAE; ITNEIEKAIKEAED;
TNEIEKAIKEAEDA; NEIEKAIKEAEDAG; EIEKAIKEAEDAGV;
IEKAIKEAEDAGVK; EKAIKEAEDAGVKT; KAIKEAEDAGVKTD;
AIKEAEDAGVKTDA; IKEAEDAGVKTDAF; KEAEDAGVKTDAFT;
EAEDAGVKTDAFTE; AEDAGVKTDAFTET; EDAGVKTDAFTETQ;
DAGVKTDAFTETQT; AGVKTDAFTETQTG; GVKTDAFTETQTGG;
VKTDAFTETQTGGK; KTDAFTETQTGGKV; TDAFTETQTGGKVG;
DAFTETQTGGKVGG; AFTETQTGGKVGGS; FTETQTGGKVGGSQ;
TETQTGGKVGGSQI; ETQTGGKVGGSQIR; TQTGGKVGGSQIRA;
QTGGKVGGSQIRAA; TGGKVGGSQIRAAK; GGKVGGSQIRAAKI;
GKVGGSQIRAAKIR; KVGGSQIRAAKIRV; VGGSQIRAAKIRVA;
GGSQIRAAKIRVAD; GSQIRAAKIRVADL; SQIRAAKIRVADLT;
QIRAAKIRVADLTI; IRAAKIRVADLTIK; RAAKIRVADLTIKF;
AAKIRVADLTIKFL; AKIRVADLTIKFLE; KIRVADLTIKFLEA;
IRVADLTIKFLEAT; RVADLTIKFLEATE; VADLTIKFLEATEE;
ADLTIKFLEATEEE; DLTIKFLEATEEET; LTIKFLEATEEETI;
TIKFLEATEEETIT; IKFLEATEEETITF; KFLEATEEETITFK;
FLEATEEETITFKE; LEATEEETITFKEN; EATEEETITFKENG;
ATEEETITFKENGA; TEEETITFKENGAG; EEETITFKENGAGE;
EETITFKENGAGEE; ETITFKENGAGEED; TITFKENGAGEEDF;
ITFKENGAGEEDFS; TFKENGAGEEDFSG; FKENGAGEEDFSGI;
KENGAGEEDFSGIY; ENGAGEEDFSGIYD; NGAGEEDFSGIYDL;
GAGEEDFSGIYDLI; AGEEDFSGIYDLIL; GEEDFSGIYDLILN;
EEDFSGIYDLILNA; EDFSGIYDLILNAA; DFSGIYDLILNAAK;
FSGIYDLILNAAKA; SGIYDLILNAAKAV; GIYDLILNAAKAVE;
IYDLILNAAKAVEK; YDLILNAAKAVEKI; DLILNAAKAVEKIG;
LILNAAKAVEKIGM; ILNAAKAVEKIGMQ; LNAAKAVEKIGMQG;
NAAKAVEKIGMQGM; AAKAVEKIGMQGMK; AKAVEKIGMQGMKQ;
KAVEKIGMQGMKQA; AVEKIGMQGMKQAV; VEKIGMQGMKQAVE;
EKIGMQGMKQAVEE; KIGMQGMKQAVEEA; IGMQGMKQAVEEAA;
GMQGMKQAVEEAAK; MQGMKQAVEEAAKE; QGMKQAVEEAAKEK;
GMKQAVEEAAKEKP; MKQAVEEAAKEKPK; KQAVEEAAKEKPKT;
QAVEEAAKEKPKTT; AVEEAAKEKPKTTA; VEEAAKEKPKTTAD;
EEAAKEKPKTTADG; EAAKEKPKTTADGI; AAKEKPKTTADGII;
AKEKPKTTADGIIA; KEKPKTTADGIIAI; EKPKTTADGIIAIV;
KPKTTADGIIAIVK; PKTTADGIIAIVKV; KTTADGIIAIVKVM;
TTADGIIAIVKVMK; TADGIIAIVKVMKA; ADGIIAIVKVMKAK;
DGIIAIVKVMKAKV; GIIAIVKVMKAKVE; IIAIVKVMKAKVEN;
IAIVKVMKAKVENI; AIVKVMKAKVENIK; IVKVMKAKVENIKE;
VKVMKAKVENIKEK; KVMKAKVENIKEKQ; VMKAKVENIKEKQT;
MKAKVENIKEKQTK; KAKVENIKEKQTKN; AKVENIKEKQTKNQ;
KVENIKEKQTKNQK;

15 mers:
MIKYNKIILTLTLLA; IKYNKIILTLTLLAS; KYNKIILTLTLLASL;
YNKIILTLTLLASLL; NKIILTLTLLASLLA; KIILTLTLLASLLAA;
IILTLTLLASLLAAC; ILTLTLLASLLAACS; LTLTLLASLLAACSL;
TLTLLASLLAACSLT; LTLLASLLAACSLTG; TLLASLLAACSLTGK;
LLASLLAACSLTGKA; LASLLAACSLTGKAR; ASLLAACSLTGKARL;
SLLAACSLTGKARLE; LLAACSLTGKARLES; LAACSLTGKARLESS;

935

AACSLTGKARLESSV; ACSLTGKARLESSVK; CSLTGKARLESSVKD;
SLTGKARLESSVKDI; LTGKARLESSVKDIT; TGKARLESSVKDITN;
GKARLESSVKDITNE; KARLESSVKDITNEI; ARLESSVKDITNEIE;
RLESSVKDITNEIEK; LESSVKDITNEIEKA; ESSVKDITNEIEKAI;
SSVKDITNEIEKAIK; SVKDITNEIEKAIKE; VKDITNEIEKAIKEA;
KDITNEIEKAIKEAE; DITNEIEKAIKEAED; ITNEIEKAIKEAEDA;
TNEIEKAIKEAEDAG; NEIEKAIKEAEDAGV; EIEKAIKEAEDAGVK;
IEKAIKEAEDAGVKT; EKAIKEAEDAGVKTD; KAIKEAEDAGVKTDA;
AIKEAEDAGVKTDAF; IKEAEDAGVKTDAFT; KEAEDAGVKTDAFTE;
EAEDAGVKTDAFTET; AEDAGVKTDAFTETQ; EDAGVKTDAFTETQT;
DAGVKTDAFTETQTG; AGVKTDAFTETQTGG; GVKTDAFTETQTGGK;
VKTDAFTETQTGGKV; KTDAFTETQTGGKVG; TDAFTETQTGGKVGG;
DAFTETQTGGKVGGS; AFTETQTGGKVGGSQ; FTETQTGGKVGGSQI;
TETQTGGKVGGSQIR; ETQTGGKVGGSQIRA; TQTGGKVGGSQIRAA;
QTGGKVGGSQIRAAK; TGGKVGGSQIRAAKI; GGKVGGSQIRAAKIR;
GKVGGSQIRAAKIRV; KVGGSQIRAAKIRVA; VGGSQIRAAKIRVAD;
GGSQIRAAKIRVADL; GSQIRAAKIRVADLT; SQIRAAKIRVADLTI;
QIRAAKIRVADLTIK; IRAAKIRVADLTIKF; RAAKIRVADLTIKFL;
AAKIRVADLTIKFLE; AKIRVADLTIKFLEA; KIRVADLTIKFLEAT;
IRVADLTIKFLEATE; RVADLTIKFLEATEE; VADLTIKFLEATEEE;
ADLTIKFLEATEEET; DLTIKFLEATEEETI; LTIKFLEATEEETIT;
TIKFLEATEEETITF; IKFLEATEEETITFK; KFLEATEEETITFKE;
FLEATEEETITFKEN; LEATEEETITFKENG; EATEEETITFKENGA;
ATEEETITFKENGAG; TEEETITFKENGAGE; EEETITFKENGAGEE;
EEITITFKENGAGEED; ETITFKENGAGEEDF; TITFKENGAGEEDFS;
ITFKENGAGEEDFSG; TFKENGAGEEDFSGI; FKENGAGEEDFSGIY;
KENGAGEEDFSGIYD; ENGAGEEDFSGIYDL; NGAGEEDFSGIYDLI;
GAGEEDFSGIYDLIL; AGEEDFSGIYDLILN; GEEDFSGIYDLILNA;
EEDFSGIYDLILNAA; EDFSGIYDLILNAAK; DFSGIYDLILNAAKA;
FSGIYDLILNAAKAV; SGIYDLILNAAKAVE; GIYDLILNAAKAVEK;
IYDLILNAAKAVEKI; YDLILNAAKAVEKIG; DLILNAAKAVEKIGM;
LILNAAKAVEKIGMQ; ILNAAKAVEKIGMQG; LNAAKAVEKIGMQGM;
NAAKAVEKIGMQGMK; AAKAVEKIGMQGMKQ; AKAVEKIGMQGMKQA;
KAVEKIGMQGMKQAV; AVEKIGMQGMKQAVE; VEKIGMQGMKQAVEE;
EKIGMQGMKQAVEEA; KIGMQGMKQAVEEAA; IGMQGMKQAVEEAAK;
GMQGMKQAVEEAAKE; MQGMKQAVEEAAKEK; QGMKQAVEEAAKEKP;
GMKQAVEEAAKEKPK; MKQAVEEAAKEKPKT; KQAVEEAAKEKPKTT;
QAVEEAAKEKPKTTA; AVEEAAKEKPKTTAD; VEEAAKEKPKTTADG;
EEAAKEKPKTTADGI; EAAKEKPKTTADGII; AAKEKPKTTADGIIA;
AKEKPKTTADGIIAI; KEKPKTTADGIIAIV; EKPKTTADGIIAIVK;
KPKTTADGIIAIVKV; PKTTADGIIAIVKVM; KTTADGIIAIVKVMK;
TTADGIIAIVKVMKA; TADGIIAIVKVMKAK; ADGIIAIVKVMKAKV;
DGIIAIVKVMKAKVE; GIIAIVKVMKAKVEN; IIAIVKVMKAKVENI;
IAIVKVMKAKVENIK; AIVKVMKAKVENIKE; IVKVMKAKVENIKEK;
VKVMKAKVENIKEKQ; KVMKAKVENIKEKQT; VMKAKVENIKEKQTK;
MKAKVENIKEKQTKN; KAKVENIKEKQTKNQ; AKVENIKEKQTKNQK;

16 mers:
MIKYNKIILTLTLLAS; IKYNKIILTLTLLASL; KYNKIILTLTLLASLL;
YNKIILTLTLLASLLA; NKIILTLTLLASLLAA; KIILTLTLLASLLAAC;
IILTLTLLASLLAACS; ILTLTLLASLLAACSL; LTLTLLASLLAACSLT;
TLTLLASLLAACSLTG; LTLLASLLAACSLTGK; TLLASLLAACSLTGKA;
LLASLLAACSLTGKAR; LASLLAACSLTGKARL; ASLLAACSLTGKARLE;
SLLAACSLTGKARLES; LLAACSLTGKARLESS; LAACSLTGKARLESSV;
AACSLTGKARLESSVK; ACSLTGKARLESSVKD; CSLTGKARLESSVKDI;
SLTGKARLESSVKDIT; LTGKARLESSVKDITN; TGKARLESSVKDITNE;
GKARLESSVKDITNEI; KARLESSVKDITNEIE; ARLESSVKDITNEIEK;

| | |
|---|---|
| | RLESSVKDITNEIEKA; LESSVKDITNEIEKAI; ESSVKDITNEIEKAIK; SSVKDITNEIEKAIKE; SVKDITNEIEKAIKEA; VKDITNEIEKAIKEAE; KDITNEIEKAIKEAED; DITNEIEKAIKEAEDA; ITNEIEKAIKEAEDAG; TNEIEKAIKEAEDAGV; NEIEKAIKEAEDAGVK; EIEKAIKEAEDAGVKT; IEKAIKEAEDAGVKTD; EKAIKEAEDAGVKTDA; KAIKEAEDAGVKTDAF; AIKEAEDAGVKTDAFT; IKEAEDAGVKTDAFTE; KEAEDAGVKTDAFTET; EAEDAGVKTDAFTETQ; AEDAGVKTDAFTETQT; EDAGVKTDAFTETQTG; DAGVKTDAFTETQTGG; AGVKTDAFTETQTGGK; GVKTDAFTETQTGGKV; VKTDAFTETQTGGKVG; KTDAFTETQTGGKVGG; TDAFTETQTGGKVGGS; DAFTETQTGGKVGGSQ; AFTETQTGGKVGGSQI; FTETQTGGKVGGSQIR; TETQTGGKVGGSQIRA; ETQTGGKVGGSQIRAA; TQTGGKVGGSQIRAAK; QTGGKVGGSQIRAAKI; TGGKVGGSQIRAAKIR; GGKVGGSQIRAAKIRV; GKVGGSQIRAAKIRVA; KVGGSQIRAAKIRVAD; VGGSQIRAAKIRVADL; GGSQIRAAKIRVADLT; GSQIRAAKIRVADLTI; SQIRAAKIRVADLTIK; QIRAAKIRVADLTIKF; IRAAKIRVADLTIKFL; RAAKIRVADLTIKFLE; AAKIRVADLTIKFLEA; AKIRVADLTIKFLEAT; KIRVADLTIKFLEATE; IRVADLTIKFLEATEE; RVADLTIKFLEATEEE; VADLTIKFLEATEEET; ADLTIKFLEATEEETI; DLTIKFLEATEEETIT; LTIKFLEATEEETITF; TIKFLEATEEETITFK; IKFLEATEEETITFKE; KFLEATEEETITFKEN; FLEATEEETITFKENG; LEATEEETITFKENGA; EATEEETITFKENGAG; ATEEETITFKENGAGE; TEEETITFKENGAGEE; EEETITFKENGAGEED; EETITFKENGAGEEDF; ETITFKENGAGEEDFS; TITFKENGAGEEDFSG; ITFKENGAGEEDFSGI; TFKENGAGEEDFSGIY; FKENGAGEEDFSGIYD; KENGAGEEDFSGIYDL; ENGAGEEDFSGIYDLI; NGAGEEDFSGIYDLIL; GAGEEDFSGIYDLILN; AGEEDFSGIYDLILNA; GEEDFSGIYDLILNAA; EEDFSGIYDLILNAAK; EDFSGIYDLILNAAKA; DFSGIYDLILNAAKAV; FSGIYDLILNAAKAVE; SGIYDLILNAAKAVEK; GIYDLILNAAKAVEKI; IYDLILNAAKAVEKIG; YDLILNAAKAVEKIGM; DLILNAAKAVEKIGMQ; LILNAAKAVEKIGMQG; ILNAAKAVEKIGMQGM; LNAAKAVEKIGMQGMK; NAAKAVEKIGMQGMKQ; AAKAVEKIGMQGMKQA; AKAVEKIGMQGMKQAV; KAVEKIGMQGMKQAVE; AVEKIGMQGMKQAVEE; VEKIGMQGMKQAVEEA; EKIGMQGMKQAVEEAA; KIGMQGMKQAVEEAAK; IGMQGMKQAVEEAAKE; GMQGMKQAVEEAAKEK; MQGMKQAVEEAAKEKP; QGMKQAVEEAAKEKPK; GMKQAVEEAAKEKPKT; MKQAVEEAAKEKPKTT; KQAVEEAAKEKPKTTA; QAVEEAAKEKPKTTAD; AVEEAAKEKPKTTADG; VEEAAKEKPKTTADGI; EEAAKEKPKTTADGII; EAAKEKPKTTADGIIA; AAKEKPKTTADGIIAI; AKEKPKTTADGIIAIV; KEKPKTTADGIIAIVK; EKPKTTADGIIAIVKV; KPKTTADGIIAIVKVM; PKTTADGIIAIVKVMK; KTTADGIIAIVKVMKA; TTADGIIAIVKVMKAK; TADGIIAIVKVMKAKV; ADGIIAIVKVMKAKVE; DGIIAIVKVMKAKVEN; GIIAIVKVMKAKVENI; IIAIVKVMKAKVENIK; IAIVKVMKAKVENIKE; AIVKVMKAKVENIKEK; IVKVMKAKVENIKEKQ; VKVMKAKVENIKEKQT; KVMKAKVENIKEKQTK; VMKAKVENIKEKQTKN; MKAKVENIKEKQTKNQ; KAKVENIKEKQTKNQK; |
| AAC70057.1 Decorin binding protein A; DbpA [Borrelia garinii] | 13 mers: MIKYNKILLKLSL; IKYNKILLKLSLI; KYNKILLKLSLIV; YNKILLKLSLIVS; NKILLKLSLIVSL; KILLKLSLIVSLL; ILLKLSLIVSLLV; LLKLSLIVSLLVA; LKLSLIVSLLVAC; KLSLIVSLLVACG; LSLIVSLLVACGL; SLIVSLLVACGLT; LIVSLLVACGLTG; IVSLLVACGLTGE; VSLLVACGLTGET; SLLVACGLTGETK; LLVACGLTGETKI; LVACGLTGETKIR; VACGLTGETKIRL; ACGLTGETKIRLE; CGLTGETKIRLES; GLTGETKIRLESS; LTGETKIRLESSA; TGETKIRLESSAQ; GETKIRLESSAQE; ETKIRLESSAQEI; TKIRLESSAQEIK; KIRLESSAQEIKD; IRLESSAQEIKDE; RLESSAQEIKDEI; LESSAQEIKDEIN; ESSAQEIKDEINK; SSAQEIKDEINKI; SAQEIKDEINKIK; AQEIKDEINKIKA; QEIKDEINKIKAN; EIKDEINKIKANA; IKDEINKIKANAK; KDEINKIKANAKK; DEINKIKANAKKE; EINKIKANAKKEG; INKIKANAKKEGV; NKIKANAKKEGVK; KIKANAKKEGVKF; |

IKANAKKEGVKFE; KANAKKEGVKFEA; ANAKKEGVKFEAF; NAKKEGVKFEAFT;
AKKEGVKFEAFTN; KKEGVKFEAFTNT; KEGVKFEAFTNTQ; EGVKFEAFTNTQT;
GVKFEAFTNTQTG; VKFEAFTNTQTGS; KFEAFTNTQTGSK; FEAFTNTQTGSKI;
EAFTNTQTGSKIS; AFTNTQTGSKISE; FTNTQTGSKISEK; TNTQTGSKISEKP;
NTQTGSKISEKPE; TQTGSKISEKPEF; QTGSKISEKPEFI; TGSKISEKPEFIL;
GSKISEKPEFILK; SKISEKPEFILKA; KISEKPEFILKAK; ISEKPEFILKAKI;
SEKPEFILKAKIK; EKPEFILKAKIKA; KPEFILKAKIKAI; PEFILKAKIKAIQ;
EFILKAKIKAIQV; FILKAKIKAIQVA; ILKAKIKAIQVAE; LKAKIKAIQVAER;
KAKIKAIQVAERF; AKIKAIQVAERFV; KIKAIQVAERFVK; IKAIQVAERFVKA;
KAIQVAERFVKAI; AIQVAERFVKAIK; IQVAERFVKAIKE; QVAERFVKAIKEE;
VAERFVKAIKEEA; AERFVKAIKEEAE; ERFVKAIKEEAEK; RFVKAIKEEAEKL;
FVKAIKEEAEKLK; VKAIKEEAEKLKK; KAIKEEAEKLKKS; AIKEEAEKLKKSG;
IKEEAEKLKKSGS; KEEAEKLKKSGSS; EEAEKLKKSGSSG; EAEKLKKSGSSGA;
AEKLKKSGSSGAF; EKLKKSGSSGAFS; KLKKSGSSGAFSA; LKKSGSSGAFSAM;
KKSGSSGAFSAMY; KSGSSGAFSAMYD; SGSSGAFSAMYDL; GSSGAFSAMYDLM;
SSGAFSAMYDLMI; SGAFSAMYDLMID; GAFSAMYDLMIDV; AFSAMYDLMIDVS;
FSAMYDLMIDVSK; SAMYDLMIDVSKP; AMYDLMIDVSKPL; MYDLMIDVSKPLE;
YDLMIDVSKPLEE; DLMIDVSKPLEEI; LMIDVSKPLEEIG; MIDVSKPLEEIGI;
IDVSKPLEEIGIQ; DVSKPLEEIGIQK; VSKPLEEIGIQKM; SKPLEEIGIQKMT;
KPLEEIGIQKMTG; PLEEIGIQKMTGT; LEEIGIQKMTGTV; EEIGIQKMTGTVT;
EIGIQKMTGTVTK; IGIQKMTGTVTKE; GIQKMTGTVTKEA; IQKMTGTVTKEAE;
QKMTGTVTKEAEK; KMTGTVTKEAEKT; MTGTVTKEAEKTP; TGTVTKEAEKTPP;
GTVTKEAEKTPPT; TVTKEAEKTPPTT; VTKEAEKTPPTTA; TKEAEKTPPTTAD;
KEAEKTPPTTADG; EAEKTPPTTADGI; AEKTPPTTADGII; EKTPPTTADGIIA;
KTPPTTADGIIAI; TPPTTADGIIAIA; PPTTADGIIAIAQ; PTTADGIIAIAQA;
TTADGIIAIAQAM; TADGIIAIAQAME; ADGIIAIAQAMEE; DGIIAIAQAMEEK;
GIIAIAQAMEEKL; IIAIAQAMEEKLN; IAIAQAMEEKLNN; AIAQAMEEKLNNV;
IAQAMEEKLNNVN; AQAMEEKLNNVNK; QAMEEKLNNVNKK; AMEEKLNNVNKKQ;
MEEKLNNVNKKQH; EEKLNNVNKKQHD; EKLNNVNKKQHDA; KLNNVNKKQHDAL;
LNNVNKKQHDALK; NNVNKKQHDALKN; NVNKKQHDALKNL; VNKKQHDALKNLE;
NKKQHDALKNLEE; KKQHDALKNLEEK; KQHDALKNLEEKA; QHDALKNLEEKAN;
HDALKNLEEKANT; DALKNLEEKANTA; ALKNLEEKANTAA; LKNLEEKANTAAT;
KNLEEKANTAATT; NLEEKANTAATTT;

14 mers:
MIKYNKILLKLSLI; IKYNKILLKLSLIV; KYNKILLKLSLIVS;
YNKILLKLSLIVSL; NKILLKLSLIVSLL; KILLKLSLIVSLLV;
ILLKLSLIVSLLVA; LLKLSLIVSLLVAC; LKLSLIVSLLVACG;
KLSLIVSLLVACGL; LSLIVSLLVACGLT; SLIVSLLVACGLTG;
LIVSLLVACGLTGE; IVSLLVACGLTGET; VSLLVACGLTGETK;
SLLVACGLTGETKI; LLVACGLTGETKIR; LVACGLTGETKIRL;
VACGLTGETKIRLE; ACGLTGETKIRLES; CGLTGETKIRLESS;
GLTGETKIRLESSA; LTGETKIRLESSAQ; TGETKIRLESSAQE;
GETKIRLESSAQEI; ETKIRLESSAQEIK; TKIRLESSAQEIKD;
KIRLESSAQEIKDE; IRLESSAQEIKDEI; RLESSAQEIKDEIN;
LESSAQEIKDEINK; ESSAQEIKDEINKI; SSAQEIKDEINKIK;
SAQEIKDEINKIKA; AQEIKDEINKIKAN; QEIKDEINKIKANA;
EIKDEINKIKANAK; IKDEINKIKANAKK; KDEINKIKANAKKE;
DEINKIKANAKKEG; EINKIKANAKKEGV; INKIKANAKKEGVK;
NKIKANAKKEGVKF; KIKANAKKEGVKFE; IKANAKKEGVKFEA;
KANAKKEGVKFEAF; ANAKKEGVKFEAFT; NAKKEGVKFEAFTN;
AKKEGVKFEAFTNT; KKEGVKFEAFTNTQ; KEGVKFEAFTNTQT;
EGVKFEAFTNTQTG; GVKFEAFTNTQTGS; VKFEAFTNTQTGSK;
KFEAFTNTQTGSKI; FEAFTNTQTGSKIS; EAFTNTQTGSKISE;
AFTNTQTGSKISEK; FTNTQTGSKISEKP; TNTQTGSKISEKPE;
NTQTGSKISEKPEF; TQTGSKISEKPEFI; QTGSKISEKPEFIL;
TGSKISEKPEFILK; GSKISEKPEFILKA; SKISEKPEFILKAK;

KISEKPEFILKAKI; ISEKPEFILKAKIK; SEKPEFILKAKIKA;
EKPEFILKAKIKAI; KPEFILKAKIKAIQ; PEFILKAKIKAIQV;
EFILKAKIKAIQVA; FILKAKIKAIQVAE; ILKAKIKAIQVAER;
LKAKIKAIQVAERF; KAKIKAIQVAERFV; AKIKAIQVAERFVK;
KIKAIQVAERFVKA; IKAIQVAERFVKAI; KAIQVAERFVKAIK;
AIQVAERFVKAIKE; IQVAERFVKAIKEE; QVAERFVKAIKEEA;
VAERFVKAIKEEAE; AERFVKAIKEEAEK; ERFVKAIKEEAEKL;
RFVKAIKEEAEKLK; FVKAIKEEAEKLKK; VKAIKEEAEKLKKS;
KAIKEEAEKLKKSG; AIKEEAEKLKKSGS; IKEEAEKLKKSGSS;
KEEAEKLKKSGSSG; EEAEKLKKSGSSGA; EAEKLKKSGSSGAF;
AEKLKKSGSSGAFS; EKLKKSGSSGAFSA; KLKKSGSSGAFSAM;
LKKSGSSGAFSAMY; KKSGSSGAFSAMYD; KSGSSGAFSAMYDL;
SGSSGAFSAMYDLM; GSSGAFSAMYDLMI; SSGAFSAMYDLMID;
SGAFSAMYDLMIDV; GAFSAMYDLMIDVS; AFSAMYDLMIDVSK;
FSAMYDLMIDVSKP; SAMYDLMIDVSKPL; AMYDLMIDVSKPLE;
MYDLMIDVSKPLEE; YDLMIDVSKPLEEI; DLMIDVSKPLEEIG;
LMIDVSKPLEEIGI; MIDVSKPLEEIGIQ; IDVSKPLEEIGIQK;
DVSKPLEEIGIQKM; VSKPLEEIGIQKMT; SKPLEEIGIQKMTG;
KPLEEIGIQKMTGT; PLEEIGIQKMTGTV; LEEIGIQKMTGTVT;
EEIGIQKMTGTVTK; EIGIQKMTGTVTKE; IGIQKMTGTVTKEA;
GIQKMTGTVTKEAE; IQKMTGTVTKEAEK; QKMTGTVTKEAEKT;
KMTGTVTKEAEKTP; MTGTVTKEAEKTPP; TGTVTKEAEKTPPT;
GTVTKEAEKTPPTT; TVTKEAEKTPPTTA; VTKEAEKTPPTTAD;
TKEAEKTPPTTADG; KEAEKTPPTTADGI; EAEKTPPTTADGII;
AEKTPPTTADGIIA; EKTPPTTADGIIAI; KTPPTTADGIIAIA;
TPPTTADGIIAIAQ; PPTTADGIIAIAQA; PTTADGIIAIAQAM;
TTADGIIAIAQAME; TADGIIAIAQAMEE; ADGIIAIAQAMEEK;
DGIIAIAQAMEEKL; GIIAIAQAMEEKLN; IIAIAQAMEEKLNN;
IAIAQAMEEKLNNV; AIAQAMEEKLNNVN; IAQAMEEKLNNVNK;
AQAMEEKLNNVNKK; QAMEEKLNNVNKKQ; AMEEKLNNVNKKQH;
MEEKLNNVNKKQHD; EEKLNNVNKKQHDA; EKLNNVNKKQHDAL;
KLNNVNKKQHDALK; LNNVNKKQHDALKN; NNVNKKQHDALKNL;
NVNKKQHDALKNLE; VNKKQHDALKNLEE; NKKQHDALKNLEEK;
KKQHDALKNLEEKA; KQHDALKNLEEKAN; QHDALKNLEEKANT;
HDALKNLEEKANTA; DALKNLEEKANTAA; ALKNLEEKANTAAT;
LKNLEEKANTAATT; KNLEEKANTAATTT;

15 mers:
MIKYNKILLKLSLIV; IKYNKILLKLSLIVS; KYNKILLKLSLIVSL;
YNKILLKLSLIVSLL; NKILLKLSLIVSLLV; KILLKLSLIVSLLVA;
ILLKLSLIVSLLVAC; LLKLSLIVSLLVACG; LKLSLIVSLLVACGL;
KLSLIVSLLVACGLT; LSLIVSLLVACGLTG; SLIVSLLVACGLTGE;
LIVSLLVACGLTGET; IVSLLVACGLTGETK; VSLLVACGLTGETKI;
SLLVACGLTGETKIR; LLVACGLTGETKIRL; LVACGLTGETKIRLE;
VACGLTGETKIRLES; ACGLTGETKIRLESS; CGLTGETKIRLESSA;
GLTGETKIRLESSAQ; LTGETKIRLESSAQE; TGETKIRLESSAQEI;
GETKIRLESSAQEIK; ETKIRLESSAQEIKD; TKIRLESSAQEIKDE;
KIRLESSAQEIKDEI; IRLESSAQEIKDEIN; RLESSAQEIKDEINK;
LESSAQEIKDEINKI; ESSAQEIKDEINKIK; SSAQEIKDEINKIKA;
SAQEIKDEINKIKAN; AQEIKDEINKIKANA; QEIKDEINKIKANAK;
EIKDEINKIKANAKK; IKDEINKIKANAKKE; KDEINKIKANAKKEG;
DEINKIKANAKKEGV; EINKIKANAKKEGVK; INKIKANAKKEGVKF;
NKIKANAKKEGVKFE; KIKANAKKEGVKFEA; IKANAKKEGVKFEAF;
KANAKKEGVKFEAFT; ANAKKEGVKFEAFTN; NAKKEGVKFEAFTNT;
AKKEGVKFEAFTNTQ; KKEGVKFEAFTNTQT; KEGVKFEAFTNTQTG;
EGVKFEAFTNTQTGS; GVKFEAFTNTQTGSK; VKFEAFTNTQTGSKI;
KFEAFTNTQTGSKIS; FEAFTNTQTGSKISE; EAFTNTQTGSKISEK;

AFTNTQTGSKISEKP; FTNTQTGSKISEKPE; TNTQTGSKISEKPEF;
NTQTGSKISEKPEFI; TQTGSKISEKPEFIL; QTGSKISEKPEFILK;
TGSKISEKPEFILKA; GSKISEKPEFILKAK; SKISEKPEFILKAKI;
KISEKPEFILKAKIK; ISEKPEFILKAKIKA; SEKPEFILKAKIKAI;
EKPEFILKAKIKAIQ; KPEFILKAKIKAIQV; PEFILKAKIKAIQVA;
EFILKAKIKAIQVAE; FILKAKIKAIQVAER; ILKAKIKAIQVAERF;
LKAKIKAIQVAERFV; KAKIKAIQVAERFVK; AKIKAIQVAERFVKA;
KIKAIQVAERFVKAI; IKAIQVAERFVKAIK; KAIQVAERFVKAIKE;
AIQVAERFVKAIKEE; IQVAERFVKAIKEEA; QVAERFVKAIKEEAE;
VAERFVKAIKEEAEK; AERFVKAIKEEAEKL; ERFVKAIKEEAEKLK;
RFVKAIKEEAEKLKK; FVKAIKEEAEKLKKS; VKAIKEEAEKLKKSG;
KAIKEEAEKLKKSGS; AIKEEAEKLKKSGSS; IKEEAEKLKKSGSSG;
KEEAEKLKKSGSSGA; EEAEKLKKSGSSGAF; EAEKLKKSGSSGAFS;
AEKLKKSGSSGAFSA; EKLKKSGSSGAFSAM; KLKKSGSSGAFSAMY;
LKKSGSSGAFSAMYD; KKSGSSGAFSAMYDL; KSGSSGAFSAMYDLM;
SGSSGAFSAMYDLMI; GSSGAFSAMYDLMID; SSGAFSAMYDLMIDV;
SGAFSAMYDLMIDVS; GAFSAMYDLMIDVSK; AFSAMYDLMIDVSKP;
FSAMYDLMIDVSKPL; SAMYDLMIDVSKPLE; AMYDLMIDVSKPLEE;
MYDLMIDVSKPLEEI; YDLMIDVSKPLEEIG; DLMIDVSKPLEEIGI;
LMIDVSKPLEEIGIQ; MIDVSKPLEEIGIQK; IDVSKPLEEIGIQKM;
DVSKPLEEIGIQKMT; VSKPLEEIGIQKMTG; SKPLEEIGIQKMTGT;
KPLEEIGIQKMTGTV; PLEEIGIQKMTGTVT; LEEIGIQKMTGTVTK;
EEIGIQKMTGTVTKE; EIGIQKMTGTVTKEA; IGIQKMTGTVTKEAE;
GIQKMTGTVTKEAEK; IQKMTGTVTKEAEKT; QKMTGTVTKEAEKTP;
KMTGTVTKEAEKTPP; MTGTVTKEAEKTPPT; TGTVTKEAEKTPPTT;
GTVTKEAEKTPPTTA; TVTKEAEKTPPTTAD; VTKEAEKTPPTTADG;
TKEAEKTPPTTADGI; KEAEKTPPTTADGII; EAEKTPPTTADGIIA;
AEKTPPTTADGIIAI; EKTPPTTADGIIAIA; KTPPTTADGIIAIAQ;
TPPTTADGIIAIAQA; PPTTADGIIAIAQAM; PTTADGIIAIAQAME;
TTADGIIAIAQAMEE; TADGIIAIAQAMEEK; ADGIIAIAQAMEEKL;
DGIIAIAQAMEEKLN; GIIAIAQAMEEKLNN; IIAIAQAMEEKLNNV;
IAIAQAMEEKLNNVN; AIAQAMEEKLNNVNK; IAQAMEEKLNNVNKK;
AQAMEEKLNNVNKKQ; QAMEEKLNNVNKKQH; AMEEKLNNVNKKQHD;
MEEKLNNVNKKQHDA; EEKLNNVNKKQHDAL; EKLNNVNKKQHDALK;
KLNNVNKKQHDALKN; LNNVNKKQHDALKNL; NNVNKKQHDALKNLE;
NVNKKQHDALKNLEE; VNKKQHDALKNLEEK; NKKQHDALKNLEEKA;
KKQHDALKNLEEKAN; KQHDALKNLEEKANT; QHDALKNLEEKANTA;
HDALKNLEEKANTAA; DALKNLEEKANTAAT; ALKNLEEKANTAATT;
LKNLEEKANTAATTT;

16 mers:
MIKYNKILLKLSLIVS; IKYNKILLKLSLIVSL; KYNKILLKLSLIVSLL;
YNKILLKLSLIVSLLV; NKILLKLSLIVSLLVA; KILLKLSLIVSLLVAC;
ILLKLSLIVSLLVACG; LLKLSLIVSLLVACGL; LKLSLIVSLLVACGLT;
KLSLIVSLLVACGLTG; LSLIVSLLVACGLTGE; SLIVSLLVACGLTGET;
LIVSLLVACGLTGETK; IVSLLVACGLTGETKI; VSLLVACGLTGETKIR;
SLLVACGLTGETKIRL; LLVACGLTGETKIRLE; LVACGLTGETKIRLES;
VACGLTGETKIRLESS; ACGLTGETKIRLESSA; CGLTGETKIRLESSAQ;
GLTGETKIRLESSAQE; LTGETKIRLESSAQEI; TGETKIRLESSAQEIK;
GETKIRLESSAQEIKD; ETKIRLESSAQEIKDE; TKIRLESSAQEIKDEI;
KIRLESSAQEIKDEIN; IRLESSAQEIKDEINK; RLESSAQEIKDEINKI;
LESSAQEIKDEINKIK; ESSAQEIKDEINKIKA; SSAQEIKDEINKIKAN;
SAQEIKDEINKIKANA; AQEIKDEINKIKANAK; QEIKDEINKIKANAKK;
EIKDEINKIKANAKKE; IKDEINKIKANAKKEG; KDEINKIKANAKKEGV;
DEINKIKANAKKEGVK; EINKIKANAKKEGVKF; INKIKANAKKEGVKFE;
NKIKANAKKEGVKFEA; KIKANAKKEGVKFEAF; IKANAKKEGVKFEAFT;
KANAKKEGVKFEAFTN; ANAKKEGVKFEAFTNT; NAKKEGVKFEAFTNTQ;

| | |
|---|---|
| | AKKEGVKFEAFTNTQT; KKEGVKFEAFTNTQTG; KEGVKFEAFTNTQTGS;<br>EGVKFEAFTNTQTGSK; GVKFEAFTNTQTGSKI; VKFEAFTNTQTGSKIS;<br>KFEAFTNTQTGSKISE; FEAFTNTQTGSKISEK; EAFTNTQTGSKISEKP;<br>AFTNTQTGSKISEKPE; FTNTQTGSKISEKPEF; TNTQTGSKISEKPEFI;<br>NTQTGSKISEKPEFIL; TQTGSKISEKPEFILK; QTGSKISEKPEFILKA;<br>TGSKISEKPEFILKAK; GSKISEKPEFILKAKI; SKISEKPEFILKAKIK;<br>KISEKPEFILKAKIKA; ISEKPEFILKAKIKAI; SEKPEFILKAKIKAIQ;<br>EKPEFILKAKIKAIQV; KPEFILKAKIKAIQVA; PEFILKAKIKAIQVAE;<br>EFILKAKIKAIQVAER; FILKAKIKAIQVAERF; ILKAKIKAIQVAERFV;<br>LKAKIKAIQVAERFVK; KAKIKAIQVAERFVKA; AKIKAIQVAERFVKAI;<br>KIKAIQVAERFVKAIK; IKAIQVAERFVKAIKE; KAIQVAERFVKAIKEE;<br>AIQVAERFVKAIKEEA; IQVAERFVKAIKEEAE; QVAERFVKAIKEEAEK;<br>VAERFVKAIKEEAEKL; AERFVKAIKEEAEKLK; ERFVKAIKEEAEKLKK;<br>RFVKAIKEEAEKLKKS; FVKAIKEEAEKLKKSG; VKAIKEEAEKLKKSGS;<br>KAIKEEAEKLKKSGSS; AIKEEAEKLKKSGSSG; IKEEAEKLKKSGSSGA;<br>KEEAEKLKKSGSSGAF; EEAEKLKKSGSSGAFS; EAEKLKKSGSSGAFSA;<br>AEKLKKSGSSGAFSAM; EKLKKSGSSGAFSAMY; KLKKSGSSGAFSAMYD;<br>LKKSGSSGAFSAMYDL; KKSGSSGAFSAMYDLM; KSGSSGAFSAMYDLMI;<br>SGSSGAFSAMYDLMID; GSSGAFSAMYDLMIDV; SSGAFSAMYDLMIDVS;<br>SGAFSAMYDLMIDVSK; GAFSAMYDLMIDVSKP; AFSAMYDLMIDVSKPL;<br>FSAMYDLMIDVSKPLE; SAMYDLMIDVSKPLEE; AMYDLMIDVSKPLEEI;<br>MYDLMIDVSKPLEEIG; YDLMIDVSKPLEEIGI; DLMIDVSKPLEEIGIQ;<br>LMIDVSKPLEEIGIQK; MIDVSKPLEEIGIQKM; IDVSKPLEEIGIQKMT;<br>DVSKPLEEIGIQKMTG; VSKPLEEIGIQKMTGT; SKPLEEIGIQKMTGTV;<br>KPLEEIGIQKMTGTVT; PLEEIGIQKMTGTVTK; LEEIGIQKMTGTVTKE;<br>EEIGIQKMTGTVTKEA; EIGIQKMTGTVTKEAE; IGIQKMTGTVTKEAEK;<br>GIQKMTGTVTKEAEKT; IQKMTGTVTKEAEKTP; QKMTGTVTKEAEKTPP;<br>KMTGTVTKEAEKTPPT; MTGTVTKEAEKTPPTT; TGTVTKEAEKTPPTTA;<br>GTVTKEAEKTPPTTAD; TVTKEAEKTPPTTADG; VTKEAEKTPPTTADGI;<br>TKEAEKTPPTTADGII; KEAEKTPPTTADGIIA; EAEKTPPTTADGIIAI;<br>AEKTPPTTADGIIAIA; EKTPPTTADGIIAIAQ; KTPPTTADGIIAIAQA;<br>TPPTTADGIIAIAQAM; PPTTADGIIAIAQAME; PTTADGIIAIAQAMEE;<br>TTADGIIAIAQAMEEK; TADGIIAIAQAMEEKL; ADGIIAIAQAMEEKLN;<br>DGIIAIAQAMEEKLNN; GIIAIAQAMEEKLNNV; IIAIAQAMEEKLNNVN;<br>IAIAQAMEEKLNNVNK; AIAQAMEEKLNNVNKK; IAQAMEEKLNNVNKKQ;<br>AQAMEEKLNNVNKKQH; QAMEEKLNNVNKKQHD; AMEEKLNNVNKKQHDA;<br>MEEKLNNVNKKQHDAL; EEKLNNVNKKQHDALK; EKLNNVNKKQHDALKN;<br>KLNNVNKKQHDALKNL; LNNVNKKQHDALKNLE; NNVNKKQHDALKNLEE;<br>NVNKKQHDALKNLEEK; VNKKQHDALKNLEEKA; NKKQHDALKNLEEKAN;<br>KKQHDALKNLEEKANT; KQHDALKNLEEKANTA; QHDALKNLEEKANTAA;<br>HDALKNLEEKANTAAT; DALKNLEEKANTAATT; ALKNLEEKANTAATTT; |
| AAC70021.1<br>Decorin binding<br>protein B; DbpB<br>[Borrelia<br>burgdorferi] | 13 mers:<br>SIVIALFFKLLVA; IVIALFFKLLVAC; VIALFFKLLVACS; IALFFKLLVACSI;<br>ALFFKLLVACSIG; LFFKLLVACSIGL; FFKLLVACSIGLV; FKLLVACSIGLVE;<br>KLLVACSIGLVER; LLVACSIGLVERT; LVACSIGLVERTN; VACSIGLVERTNA;<br>ACSIGLVERTNAA; CSIGLVERTNAAL; SIGLVERTNAALE; IGLVERTNAALES;<br>GLVERTNAALESS; LVERTNAALESSS; VERTNAALESSSK; ERTNAALESSSKD;<br>RTNAALESSSKDL; TNAALESSSKDLK; NAALESSSKDLKN; AALESSSKDLKNK;<br>ALESSSKDLKNKI; LESSSKDLKNKIL; ESSSKDLKNKILK; SSSKDLKNKILKI;<br>SSKDLKNKILKIK; SKDLKNKILKIKK; KDLKNKILKIKKE; DLKNKILKIKKEA;<br>LKNKILKIKKEAT; KNKILKIKKEATG; NKILKIKKEATGK; KILKIKKEATGKG;<br>ILKIKKEATGKGV; LKIKKEATGKGVL; KIKKEATGKGVLF; IKKEATGKGVLFE;<br>KKEATGKGVLFEA; KEATGKGVLFEAF; EATGKGVLFEAFT; ATGKGVLFEAFTG;<br>TGKGVLFEAFTGL; GKGVLFEAFTGLK; KGVLFEAFTGLKT; GVLFEAFTGLKTG;<br>VLFEAFTGLKTGS; LFEAFTGLKTGSK; FEAFTGLKTGSKV; EAFTGLKTGSKVT; |

AFTGLKTGSKVTS; FTGLKTGSKVTSG; TGLKTGSKVTSGG; GLKTGSKVTSGGL;
LKTGSKVTSGGLA; KTGSKVTSGGLAL; TGSKVTSGGLALR; GSKVTSGGLALRE;
SKVTSGGLALREA; KVTSGGLALREAK; VTSGGLALREAKV; TSGGLALREAKVQ;
SGGLALREAKVQA; GGLALREAKVQAI; GLALREAKVQAIV; LALREAKVQAIVE;
ALREAKVQAIVET; LREAKVQAIVETG; REAKVQAIVETGK; EAKVQAIVETGKF;
AKVQAIVETGKFL; KVQAIVETGKFLK; VQAIVETGKFLKI; QAIVETGKFLKII;
AIVETGKFLKIIE; IVETGKFLKIIEE; VETGKFLKIIEEE; ETGKFLKIIEEEA;
TGKFLKIIEEEAL; GKFLKIIEEEALK; KFLKIIEEEALKL; FLKIIEEEALKLK;
LKIIEEEALKLKE; KIIEEEALKLKET; IIEEEALKLKETG; IEEEALKLKETGN;
EEEALKLKETGNS; EEALKLKETGNSG; EALKLKETGNSGQ; ALKLKETGNSGQF;
LKLKETGNSGQFL; KLKETGNSGQFLA; LKETGNSGQFLAM; KETGNSGQFLAMF;
ETGNSGQFLAMFD; TGNSGQFLAMFDL; GNSGQFLAMFDLM; NSGQFLAMFDLML;
SGQFLAMFDLMLE; GQFLAMFDLMLEV; QFLAMFDLMLEVV; FLAMFDLMLEVVE;
LAMFDLMLEVVES; AMFDLMLEVVESL; MFDLMLEVVESLE; FDLMLEVVESLED;
DLMLEVVESLEDV; LMLEVVESLEDVG; MLEVVESLEDVGI; LEVVESLEDVGII;
EVVESLEDVGIIG; VVESLEDVGIIGL; VESLEDVGIIGLK; ESLEDVGIIGLKA;
SLEDVGIIGLKAR; LEDVGIIGLKARV; EDVGIIGLKARVL; DVGIIGLKARVLE;
VGIIGLKARVLEE; GIIGLKARVLEES; IIGLKARVLEESK; IGLKARVLEESKN;
GLKARVLEESKNN; LKARVLEESKNNP; KARVLEESKNNPI; ARVLEESKNNPIN;
RVLEESKNNPINT; VLEESKNNPINTA; LEESKNNPINTAE; EESKNNPINTAER;
ESKNNPINTAERL; SKNNPINTAERLL; KNNPINTAERLLA; NNPINTAERLLAA;
NPINTAERLLAAK; PINTAERLLAAKA; INTAERLLAAKAQ; NTAERLLAAKAQI;
TAERLLAAKAQIE; AERLLAAKAQIEN; ERLLAAKAQIENQ; RLLAAKAQIENQL;
LLAAKAQIENQLK; LAAKAQIENQLKV; AAKAQIENQLKVV; AKAQIENQLKVVK;
KAQIENQLKVVKE; AQIENQLKVVKEK; QIENQLKVVKEKQ; IENQLKVVKEKQN;
ENQLKVVKEKQNI; NQLKVVKEKQNIE; QLKVVKEKQNIEN; LKVVKEKQNIENG;
KVVKEKQNIENGG; VVKEKQNIENGGE; VKEKQNIENGGEK; KEKQNIENGGEKK;
EKQNIENGGEKKN; KQNIENGGEKKNN; QNIENGGEKKNNK; NIENGGEKKNNKS;
IENGGEKKNNKSK; ENGGEKKNNKSKK; NGGEKKNNKSKKK; GGEKKNNKSKKKK;

14 mers:
SIVIALFFKLLVAC; IVIALFFKLLVACS; VIALFFKLLVACSI;
IALFFKLLVACSIG; ALFFKLLVACSIGL; LFFKLLVACSIGLV;
FFKLLVACSIGLVE; FKLLVACSIGLVER; KLLVACSIGLVERT;
LLVACSIGLVERTN; LVACSIGLVERTNA; VACSIGLVERTNAA;
ACSIGLVERTNAAL; CSIGLVERTNAALE; SIGLVERTNAALES;
IGLVERTNAALESS; GLVERTNAALESSS; LVERTNAALESSSK;
VERTNAALESSSKD; ERTNAALESSSKDL; RTNAALESSSKDLK;
TNAALESSSKDLKN; NAALESSSKDLKNK; AALESSSKDLKNKI;
ALESSSKDLKNKIL; LESSSKDLKNKILK; ESSSKDLKNKILKI;
SSSKDLKNKILKIK; SSKDLKNKILKIKK; SKDLKNKILKIKKE;
KDLKNKILKIKKEA; DLKNKILKIKKEAT; LKNKILKIKKEATG;
KNKILKIKKEATGK; NKILKIKKEATGKG; KILKIKKEATGKGV;
ILKIKKEATGKGVL; LKIKKEATGKGVLF; KIKKEATGKGVLFE;
IKKEATGKGVLFEA; KKEATGKGVLFEAF; KEATGKGVLFEAFT;
EATGKGVLFEAFTG; ATGKGVLFEAFTGL; TGKGVLFEAFTGLK;
GKGVLFEAFTGLKT; KGVLFEAFTGLKTG; GVLFEAFTGLKTGS;
VLFEAFTGLKTGSK; LFEAFTGLKTGSKV; FEAFTGLKTGSKVT;
EAFTGLKTGSKVTS; AFTGLKTGSKVTSG; FTGLKTGSKVTSGG;
TGLKTGSKVTSGGL; GLKTGSKVTSGGLA; LKTGSKVTSGGLAL;
KTGSKVTSGGLALR; TGSKVTSGGLALRE; GSKVTSGGLALREA;
SKVTSGGLALREAK; KVTSGGLALREAKV; VTSGGLALREAKVQ;
TSGGLALREAKVQA; SGGLALREAKVQAI; GGLALREAKVQAIV;
GLALREAKVQAIVE; LALREAKVQAIVET; ALREAKVQAIVETG;
LREAKVQAIVETGK; REAKVQAIVETGKF; EAKVQAIVETGKFL;
AKVQAIVETGKFLK; KVQAIVETGKFLKI; VQAIVETGKFLKII;
QAIVETGKFLKIIE; AIVETGKFLKIIEE; IVETGKFLKIIEEE;

VETGKFLKIIEEEA; ETGKFLKIIEEEAL; TGKFLKIIEEEALK;
GKFLKIIEEEALKL; KFLKIIEEEALKLK; FLKIIEEEALKLKE;
LKIIEEEALKLKET; KIIEEEALKLKETG; IIEEEALKLKETGN;
IEEEALKLKETGNS; EEEALKLKETGNSG; EEALKLKETGNSGQ;
EALKLKETGNSGQF; ALKLKETGNSGQFL; LKLKETGNSGQFLA;
KLKETGNSGQFLAM; LKETGNSGQFLAMF; KETGNSGQFLAMFD;
ETGNSGQFLAMFDL; TGNSGQFLAMFDLM; GNSGQFLAMFDLML;
NSGQFLAMFDLMLE; SGQFLAMFDLMLEV; GQFLAMFDLMLEVV;
QFLAMFDLMLEVVE; FLAMFDLMLEVVES; LAMFDLMLEVVESL;
AMFDLMLEVVESLE; MFDLMLEVVESLED; FDLMLEVVESLEDV;
DLMLEVVESLEDVG; LMLEVVESLEDVGI; MLEVVESLEDVGII;
LEVVESLEDVGIIG; EVVESLEDVGIIGL; VVESLEDVGIIGLK;
VESLEDVGIIGLKA; ESLEDVGIIGLKAR; SLEDVGIIGLKARV;
LEDVGIIGLKARVL; EDVGIIGLKARVLE; DVGIIGLKARVLEE;
VGIIGLKARVLEES; GIIGLKARVLEESK; IIGLKARVLEESKN;
IGLKARVLEESKNN; GLKARVLEESKNNP; LKARVLEESKNNPI;
KARVLEESKNNPIN; ARVLEESKNNPINT; RVLEESKNNPINTA;
VLEESKNNPINTAE; LEESKNNPINTAER; EESKNNPINTAERL;
ESKNNPINTAERLL; SKNNPINTAERLLA; KNNPINTAERLLAA;
NNPINTAERLLAAK; NPINTAERLLAAKA; PINTAERLLAAKAQ;
INTAERLLAAKAQI; NTAERLLAAKAQIE; TAERLLAAKAQIEN;
AERLLAAKAQIENQ; ERLLAAKAQIENQL; RLLAAKAQIENQLK;
LLAAKAQIENQLKV; LAAKAQIENQLKVV; AAKAQIENQLKVVK;
AKAQIENQLKVVKE; KAQIENQLKVVKEK; AQIENQLKVVKEKQ;
QIENQLKVVKEKQN; IENQLKVVKEKQNI; ENQLKVVKEKQNIE;
NQLKVVKEKQNIEN; QLKVVKEKQNIENG; LKVVKEKQNIENGG;
KVVKEKQNIENGGE; VVKEKQNIENGGEK; VKEKQNIENGGEKK;
KEKQNIENGGEKKN; EKQNIENGGEKKNN; KQNIENGGEKKNNK;
QNIENGGEKKNNKS; NIENGGEKKNNKSK; IENGGEKKNNKSKK;
ENGGEKKNNKSKKK; NGGEKKNNKSKKKK;

15 mers:
SIVIALFFKLLVACS; IVIALFFKLLVACSI; VIALFFKLLVACSIG;
IALFFKLLVACSIGL; ALFFKLLVACSIGLV; LFFKLLVACSIGLVE;
FFKLLVACSIGLVER; FKLLVACSIGLVERT; KLLVACSIGLVERTN;
LLVACSIGLVERTNA; LVACSIGLVERTNAA; VACSIGLVERTNAAL;
ACSIGLVERTNAALE; CSIGLVERTNAALES; SIGLVERTNAALESS;
IGLVERTNAALESSS; GLVERTNAALESSSK; LVERTNAALESSSKD;
VERTNAALESSSKDL; ERTNAALESSSKDLK; RTNAALESSSKDLKN;
TNAALESSSKDLKNK; NAALESSSKDLKNKI; AALESSSKDLKNKIL;
ALESSSKDLKNKILK; LESSSKDLKNKILKI; ESSSKDLKNKILKIK;
SSSKDLKNKILKIKK; SSKDLKNKILKIKKE; SKDLKNKILKIKKEA;
KDLKNKILKIKKEAT; DLKNKILKIKKEATG; LKNKILKIKKEATGK;
KNKILKIKKEATGKG; NKILKIKKEATGKGV; KILKIKKEATGKGVL;
ILKIKKEATGKGVLF; LKIKKEATGKGVLFE; KIKKEATGKGVLFEA;
IKKEATGKGVLFEAF; KKEATGKGVLFEAFT; KEATGKGVLFEAFTG;
EATGKGVLFEAFTGL; ATGKGVLFEAFTGLK; TGKGVLFEAFTGLKT;
GKGVLFEAFTGLKTG; KGVLFEAFTGLKTGS; GVLFEAFTGLKTGSK;
VLFEAFTGLKTGSKV; LFEAFTGLKTGSKVT; FEAFTGLKTGSKVTS;
EAFTGLKTGSKVTSG; AFTGLKTGSKVTSGG; FTGLKTGSKVTSGGL;
TGLKTGSKVTSGGLA; GLKTGSKVTSGGLAL; LKTGSKVTSGGLALR;
KTGSKVTSGGLALRE; TGSKVTSGGLALREA; GSKVTSGGLALREAK;
SKVTSGGLALREAKV; KVTSGGLALREAKVQ; VTSGGLALREAKVQA;
TSGGLALREAKVQAI; SGGLALREAKVQAIV; GGLALREAKVQAIVE;
GLALREAKVQAIVET; LALREAKVQAIVETG; ALREAKVQAIVETGK;
LREAKVQAIVETGKF; REAKVQAIVETGKFL; EAKVQAIVETGKFLK;
AKVQAIVETGKFLKI; KVQAIVETGKFLKII; VQAIVETGKFLKIIE;

QAIVETGKFLKIIEE; AIVETGKFLKIIEEE; IVETGKFLKIIEEEA;
VETGKFLKIIEEEAL; ETGKFLKIIEEEALK; TGKFLKIIEEEALKL;
GKFLKIIEEEALKLK; KFLKIIEEEALKLKE; FLKIIEEEALKLKET;
LKIIEEEALKLKETG; KIIEEEALKLKETGN; IIEEEALKLKETGNS;
IEEEALKLKETGNSG; EEEALKLKETGNSGQ; EEALKLKETGNSGQF;
EALKLKETGNSGQFL; ALKLKETGNSGQFLA; LKLKETGNSGQFLAM;
KLKETGNSGQFLAMF; LKETGNSGQFLAMFD; KETGNSGQFLAMFDL;
ETGNSGQFLAMFDLM; TGNSGQFLAMFDLML; GNSGQFLAMFDLMLE;
NSGQFLAMFDLMLEV; SGQFLAMFDLMLEVV; GQFLAMFDLMLEVVE;
QFLAMFDLMLEVVES; FLAMFDLMLEVVESL; LAMFDLMLEVVESLE;
AMFDLMLEVVESLED; MFDLMLEVVESLEDV; FDLMLEVVESLEDVG;
DLMLEVVESLEDVGI; LMLEVVESLEDVGII; MLEVVESLEDVGIIG;
LEVVESLEDVGIIGL; EVVESLEDVGIIGLK; VVESLEDVGIIGLKA;
VESLEDVGIIGLKAR; ESLEDVGIIGLKARV; SLEDVGIIGLKARVL;
LEDVGIIGLKARVLE; EDVGIIGLKARVLEE; DVGIIGLKARVLEES;
VGIIGLKARVLEESK; GIIGLKARVLEESKN; IIGLKARVLEESKNN;
IGLKARVLEESKNNP; GLKARVLEESKNNPI; LKARVLEESKNNPIN;
KARVLEESKNNPINT; ARVLEESKNNPINTA; RVLEESKNNPINTAE;
VLEESKNNPINTAER; LEESKNNPINTAERL; EESKNNPINTAERLL;
ESKNNPINTAERLLA; SKNNPINTAERLLAA; KNNPINTAERLLAAK;
NNPINTAERLLAAKA; NPINTAERLLAAKAQ; PINTAERLLAAKAQI;
INTAERLLAAKAQIE; NTAERLLAAKAQIEN; TAERLLAAKAQIENQ;
AERLLAAKAQIENQL; ERLLAAKAQIENQLK; RLLAAKAQIENQLKV;
LLAAKAQIENQLKVV; LAAKAQIENQLKVVK; AAKAQIENQLKVVKE;
AKAQIENQLKVVKEK; KAQIENQLKVVKEKQ; AQIENQLKVVKEKQN;
QIENQLKVVKEKQNI; IENQLKVVKEKQNIE; ENQLKVVKEKQNIEN;
NQLKVVKEKQNIENG; QLKVVKEKQNIENGG; LKVVKEKQNIENGGE;
KVVKEKQNIENGGEK; VVKEKQNIENGGEKK; VKEKQNIENGGEKKN;
KEKQNIENGGEKKNN; EKQNIENGGEKKNNK; KQNIENGGEKKNNKS;
QNIENGGEKKNNKSK; NIENGGEKKNNKSKK; IENGGEKKNNKSKKK;
ENGGEKKNNKSKKKK;

16 mers:
SIVIALFFKLLVACSI; IVIALFFKLLVACSIG; VIALFFKLLVACSIGL;
IALFFKLLVACSIGLV; ALFFKLLVACSIGLVE; LFFKLLVACSIGLVER;
FFKLLVACSIGLVERT; FKLLVACSIGLVERTN; KLLVACSIGLVERTNA;
LLVACSIGLVERTNAA; LVACSIGLVERTNAAL; VACSIGLVERTNAALE;
ACSIGLVERTNAALES; CSIGLVERTNAALESS; SIGLVERTNAALESSS;
IGLVERTNAALESSSK; GLVERTNAALESSSKD; LVERTNAALESSSKDL;
VERTNAALESSSKDLK; ERTNAALESSSKDLKN; RTNAALESSSKDLKNK;
TNAALESSSKDLKNKI; NAALESSSKDLKNKIL; AALESSSKDLKNKILK;
ALESSSKDLKNKILKI; LESSSKDLKNKILKIK; ESSSKDLKNKILKIKK;
SSSKDLKNKILKIKKE; SSKDLKNKILKIKKEA; SKDLKNKILKIKKEAT;
KDLKNKILKIKKEATG; DLKNKILKIKKEATGK; LKNKILKIKKEATGKG;
KNKILKIKKEATGKGV; NKILKIKKEATGKGVL; KILKIKKEATGKGVLF;
ILKIKKEATGKGVLFE; LKIKKEATGKGVLFEA; KIKKEATGKGVLFEAF;
IKKEATGKGVLFEAFT; KKEATGKGVLFEAFTG; KEATGKGVLFEAFTGL;
EATGKGVLFEAFTGLK; ATGKGVLFEAFTGLKT; TGKGVLFEAFTGLKTG;
GKGVLFEAFTGLKTGS; KGVLFEAFTGLKTGSK; GVLFEAFTGLKTGSKV;
VLFEAFTGLKTGSKVT; LFEAFTGLKTGSKVTS; FEAFTGLKTGSKVTSG;
EAFTGLKTGSKVTSGG; AFTGLKTGSKVTSGGL; FTGLKTGSKVTSGGLA;
TGLKTGSKVTSGGLAL; GLKTGSKVTSGGLALR; LKTGSKVTSGGLALRE;
KTGSKVTSGGLALREA; TGSKVTSGGLALREAK; GSKVTSGGLALREAKV;
SKVTSGGLALREAKVQ; KVTSGGLALREAKVQA; VTSGGLALREAKVQAI;
TSGGLALREAKVQAIV; SGGLALREAKVQAIVE; GGLALREAKVQAIVET;
GLALREAKVQAIVETG; LALREAKVQAIVETGK; ALREAKVQAIVETGKF;
LREAKVQAIVETGKFL; REAKVQAIVETGKFLK; EAKVQAIVETGKFLKI;

| | |
|---|---|
| | AKVQAIVETGKFLKII; KVQAIVETGKFLKIIE; VQAIVETGKFLKIIEE;<br>QAIVETGKFLKIIEEE; AIVETGKFLKIIEEEA; IVETGKFLKIIEEEAL;<br>VETGKFLKIIEEEALK; ETGKFLKIIEEEALKL; TGKFLKIIEEEALKLK;<br>GKFLKIIEEEALKLKE; KFLKIIEEEALKLKET; FLKIIEEEALKLKETG;<br>LKIIEEEALKLKETGN; KIIEEEALKLKETGNS; IIEEEALKLKETGNSG;<br>IEEEALKLKETGNSGQ; EEEALKLKETGNSGQF; EEALKLKETGNSGQFL;<br>EALKLKETGNSGQFLA; ALKLKETGNSGQFLAM; LKLKETGNSGQFLAMF;<br>KLKETGNSGQFLAMFD; LKETGNSGQFLAMFDL; KETGNSGQFLAMFDLM;<br>ETGNSGQFLAMFDLML; TGNSGQFLAMFDLMLE; GNSGQFLAMFDLMLEV;<br>NSGQFLAMFDLMLEVV; SGQFLAMFDLMLEVVE; GQFLAMFDLMLEVVES;<br>QFLAMFDLMLEVVESL; FLAMFDLMLEVVESLE; LAMFDLMLEVVESLED;<br>AMFDLMLEVVESLEDV; MFDLMLEVVESLEDVG; FDLMLEVVESLEDVGI;<br>DLMLEVVESLEDVGII; LMLEVVESLEDVGIIG; MLEVVESLEDVGIIGL;<br>LEVVESLEDVGIIGLK; EVVESLEDVGIIGLKA; VVESLEDVGIIGLKAR;<br>VESLEDVGIIGLKARV; ESLEDVGIIGLKARVL; SLEDVGIIGLKARVLE;<br>LEDVGIIGLKARVLEE; EDVGIIGLKARVLEES; DVGIIGLKARVLEESK;<br>VGIIGLKARVLEESKN; GIIGLKARVLEESKNN; IIGLKARVLEESKNNP;<br>IGLKARVLEESKNNPI; GLKARVLEESKNNPIN; LKARVLEESKNNPINT;<br>KARVLEESKNNPINTA; ARVLEESKNNPINTAE; RVLEESKNNPINTAER;<br>VLEESKNNPINTAERL; LEESKNNPINTAERLL; EESKNNPINTAERLLA;<br>ESKNNPINTAERLLAA; SKNNPINTAERLLAAK; KNNPINTAERLLAAKA;<br>NNPINTAERLLAAKAQ; NPINTAERLLAAKAQI; PINTAERLLAAKAQIE;<br>INTAERLLAAKAQIEN; NTAERLLAAKAQIENQ; TAERLLAAKAQIENQL;<br>AERLLAAKAQIENQLK; ERLLAAKAQIENQLKV; RLLAAKAQIENQLKVV;<br>LLAAKAQIENQLKVVK; LAAKAQIENQLKVVKE; AAKAQIENQLKVVKEK;<br>AKAQIENQLKVVKEKQ; KAQIENQLKVVKEKQN; AQIENQLKVVKEKQNI;<br>QIENQLKVVKEKQNIE; IENQLKVVKEKQNIEN; ENQLKVVKEKQNIENG;<br>NQLKVVKEKQNIENGG; QLKVVKEKQNIENGGE; LKVVKEKQNIENGGEK;<br>KVVKEKQNIENGGEKK; VVKEKQNIENGGEKKN; VKEKQNIENGGEKKNN;<br>KEKQNIENGGEKKNNK; EKQNIENGGEKKNNKS; KQNIENGGEKKNNKSK;<br>QNIENGGEKKNNKSKK; NIENGGEKKNNKSKKK; IENGGEKKNNKSKKKK; |
| NP_212694.1<br>Heat shock protein<br>90 [Borrelia<br>burgdorferi B31] | 13 mers:<br>MILIFYFKQIALF; ILIFYFKQIALFI; LIFYFKQIALFII; IFYFKQIALFIIF;<br>FYFKQIALFIIFR; YFKQIALFIIFRL; FKQIALFIIFRLC; KQIALFIIFRLCY;<br>QIALFIIFRLCYI; IALFIIFRLCYII; ALFIIFRLCYIIK; LFIIFRLCYIIKK;<br>FIIFRLCYIIKKV; IIFRLCYIIKKVK; IFRLCYIIKKVKI; FRLCYIIKKVKIK;<br>RLCYIIKKVKIKL; LCYIIKKVKIKLK; CYIIKKVKIKLKR; YIIKKVKIKLKRK;<br>IIKKVKIKLKRKS; IKKVKIKLKRKSC; KKVKIKLKRKSCM; KVKIKLKRKSCMK;<br>VKIKLKRKSCMKK; KIKLKRKSCMKKQ; IKLKRKSCMKKQF; KLKRKSCMKKQFD;<br>LKRKSCMKKQFDT; KRKSCMKKQFDTE; RKSCMKKQFDTEV; KSCMKKQFDTEVN;<br>SCMKKQFDTEVND; CMKKQFDTEVNDL; MKKQFDTEVNDLL; KKQFDTEVNDLLY;<br>KQFDTEVNDLLYL; QFDTEVNDLLYLI; FDTEVNDLLYLII; DTEVNDLLYLIIH;<br>TEVNDLLYLIIHS; EVNDLLYLIIHSL; VNDLLYLIIHSLY; NDLLYLIIHSLYS;<br>DLLYLIIHSLYSH; LLYLIIHSLYSHK; LYLIIHSLYSHKE; YLIIHSLYSHKEI;<br>LIIHSLYSHKEIF; IIHSLYSHKEIFL; IHSLYSHKEIFLR; HSLYSHKEIFLRE;<br>SLYSHKEIFLREL; LYSHKEIFLRELI; YSHKEIFLRELIS; SHKEIFLRELISN;<br>HKEIFLRELISNA; KEIFLRELISNAS; EIFLRELISNASD; IFLRELISNASDA;<br>FLRELISNASDAI; LRELISNASDAID; RELISNASDAIDK; ELISNASDAIDKL;<br>LISNASDAIDKLK; ISNASDAIDKLKF; SNASDAIDKLKFL; NASDAIDKLKFLS;<br>ASDAIDKLKFLSL; SDAIDKLKFLSLT; DAIDKLKFLSLTN; AIDKLKFLSLTNE;<br>IDKLKFLSLTNEK; DKLKFLSLTNEKF; KLKFLSLTNEKFK; LKFLSLTNEKFKN;<br>KFLSLTNEKFKNI; FLSLTNEKFKNIA; LSLTNEKFKNIAL; SLTNEKFKNIALE;<br>LTNEKFKNIALEP; TNEKFKNIALEPK; NEKFKNIALEPKI; EKFKNIALEPKIE;<br>KFKNIALEPKIEI; FKNIALEPKIEIS; KNIALEPKIEISF; NIALEPKIEISFD;<br>IALEPKIEISFDD; ALEPKIEISFDDK; LEPKIEISFDDKS; EPKIEISFDDKSI; |

| |
|---|
| PKIEISFDDKSIL; KIEISFDDKSILI; IEISFDDKSILIK; EISFDDKSILIKD;<br>ISFDDKSILIKDN; SFDDKSILIKDNG; FDDKSILIKDNGI; DDKSILIKDNGIG;<br>DKSILIKDNGIGM; KSILIKDNGIGMD; SILIKDNGIGMDE; ILIKDNGIGMDEQ;<br>LIKDNGIGMDEQD; IKDNGIGMDEQDL; KDNGIGMDEQDLT; DNGIGMDEQDLTN;<br>NGIGMDEQDLTNH; GIGMDEQDLTNHL; IGMDEQDLTNHLG; GMDEQDLTNHLGV;<br>MDEQDLTNHLGVI; DEQDLTNHLGVIA; EQDLTNHLGVIAK; QDLTNHLGVIAKS;<br>DLTNHLGVIAKSG; LTNHLGVIAKSGT; TNHLGVIAKSGTK; NHLGVIAKSGTKE;<br>HLGVIAKSGTKEF; LGVIAKSGTKEFI; GVIAKSGTKEFIN; VIAKSGTKEFINN;<br>IAKSGTKEFINNL; AKSGTKEFINNLK; KSGTKEFINNLKQ; SGTKEFINNLKQD;<br>GTKEFINNLKQDE; TKEFINNLKQDEK; KEFINNLKQDEKK; EFINNLKQDEKKS;<br>FINNLKQDEKKSA; INNLKQDEKKSAS; NNLKQDEKKSASL; NLKQDEKKSASLI;<br>LKQDEKKSASLIG; KQDEKKSASLIGQ; QDEKKSASLIGQF; DEKKSASLIGQFG;<br>EKKSASLIGQFGV; KKSASLIGQFGVG; KSASLIGQFGVGF; SASLIGQFGVGFY;<br>ASLIGQFGVGFYS; SLIGQFGVGFYSA; LIGQFGVGFYSAF; IGQFGVGFYSAFI;<br>GQFGVGFYSAFIV; QFGVGFYSAFIVS; FGVGFYSAFIVSE; GVGFYSAFIVSEK;<br>VGFYSAFIVSEKV; GFYSAFIVSEKVE; FYSAFIVSEKVEV; YSAFIVSEKVEVT;<br>SAFIVSEKVEVTS; AFIVSEKVEVTSK; FIVSEKVEVTSKK; IVSEKVEVTSKKA;<br>VSEKVEVTSKKAL; SEKVEVTSKKALE; EKVEVTSKKALES; KVEVTSKKALESD;<br>VEVTSKKALESDA; EVTSKKALESDAY; VTSKKALESDAYI; TSKKALESDAYIW;<br>SKKALESDAYIWS; KKALESDAYIWSS; KALESDAYIWSSD; ALESDAYIWSSDG;<br>LESDAYIWSSDGK; ESDAYIWSSDGKT; SDAYIWSSDGKTG; DAYIWSSDGKTGY;<br>AYIWSSDGKTGYE; YIWSSDGKTGYEI; IWSSDGKTGYEIE; WSSDGKTGYEIEK;<br>SSDGKTGYEIEKA; SDGKTGYEIEKAK; DGKTGYEIEKAKK; GKTGYEIEKAKKE;<br>KTGYEIEKAKKEE; TGYEIEKAKKEES; GYEIEKAKKEESG; YEIEKAKKEESGT;<br>EIEKAKKEESGTE; IEKAKKEESGTEI; EKAKKEESGTEIK; KAKKEESGTEIKL;<br>AKKEESGTEIKLY; KKEESGTEIKLYL; KEESGTEIKLYLN; EESGTEIKLYLNK;<br>ESGTEIKLYLNKE; SGTEIKLYLNKEG; GTEIKLYLNKEGL; TEIKLYLNKEGLE;<br>EIKLYLNKEGLEY; IKLYLNKEGLEYA; KLYLNKEGLEYAN; LYLNKEGLEYANK;<br>YLNKEGLEYANKW; LNKEGLEYANKWK; NKEGLEYANKWKI; KEGLEYANKWKIQ;<br>EGLEYANKWKIQE; GLEYANKWKIQEI; LEYANKWKIQEII; EYANKWKIQEIIK;<br>YANKWKIQEIIKK; ANKWKIQEIIKKY; NKWKIQEIIKKYS; KWKIQEIIKKYSN;<br>WKIQEIIKKYSNH; KIQEIIKKYSNHI; IQEIIKKYSNHIN; QEIIKKYSNHINY;<br>EIIKKYSNHINYP; IIKKYSNHINYPI; IKKYSNHINYPIY; KKYSNHINYPIYI;<br>KYSNHINYPIYIK; YSNHINYPIYIKY; SNHINYPIYIKYS; NHINYPIYIKYSE;<br>HINYPIYIKYSEP; INYPIYIKYSEPI; NYPIYIKYSEPIM; YPIYIKYSEPIMK;<br>PIYIKYSEPIMKD; IYIKYSEPIMKDG; YIKYSEPIMKDGK; IKYSEPIMKDGKQ;<br>KYSEPIMKDGKQE; YSEPIMKDGKQEG; SEPIMKDGKQEGI; EPIMKDGKQEGIE;<br>PIMKDGKQEGIEE; IMKDGKQEGIEEK; MKDGKQEGIEEKE; KDGKQEGIEEKEE;<br>DGKQEGIEEKEEK; GKQEGIEEKEEKL; KQEGIEEKEEKLN; QEGIEEKEEKLNE;<br>EGIEEKEEKLNET; GIEEKEEKLNETT; IEEKEEKLNETTA; EEKEEKLNETTAL;<br>EKEEKLNETTALW; KEEKLNETTALWT; EEKLNETTALWTK; EKLNETTALWTKN;<br>KLNETTALWTKNK; LNETTALWTKNKS; NETTALWTKNKSE; ETTALWTKNKSEI;<br>TTALWTKNKSEIK; TALWTKNKSEIKA; ALWTKNKSEIKAE; LWTKNKSEIKAEE;<br>WTKNKSEIKAEEY; TKNKSEIKAEEYN; KNKSEIKAEEYNE; NKSEIKAEEYNEF;<br>KSEIKAEEYNEFY; SEIKAEEYNEFYK; EIKAEEYNEFYKN; IKAEEYNEFYKNT;<br>KAEEYNEFYKNTT; AEEYNEFYKNTTF; EEYNEFYKNTTFD; EYNEFYKNTTFDY;<br>YNEFYKNTTFDYE; NEFYKNTTFDYEN; EFYKNTTFDYENP; FYKNTTFDYENPL;<br>YKNTTFDYENPLM; KNTTFDYENPLMH; NTTFDYENPLMHI; TTFDYENPLMHIH;<br>TFDYENPLMHIHT; FDYENPLMHIHTK; DYENPLMHIHTKA; YENPLMHIHTKAE;<br>ENPLMHIHTKAEG; NPLMHIHTKAEGN; PLMHIHTKAEGNL; LMHIHTKAEGNLE;<br>MHIHTKAEGNLEY; HIHTKAEGNLEYT; IHTKAEGNLEYTN; HTKAEGNLEYTNL;<br>TKAEGNLEYTNLF; KAEGNLEYTNLFY; AEGNLEYTNLFYV; EGNLEYTNLFYVP;<br>GNLEYTNLFYVPS; NLEYTNLFYVPSK; LEYTNLFYVPSKA; EYTNLFYVPSKAP;<br>YTNLFYVPSKAPY; TNLFYVPSKAPYD; NLFYVPSKAPYDL; LFYVPSKAPYDLY;<br>FYVPSKAPYDLYY; YVPSKAPYDLYYP; VPSKAPYDLYYPN; PSKAPYDLYYPNT;<br>SKAPYDLYYPNTK; KAPYDLYYPNTKP; APYDLYYPNTKPG; PYDLYYPNTKPGV;<br>YDLYYPNTKPGVK; DLYYPNTKPGVKL; LYYPNTKPGVKLF; YYPNTKPGVKLFI; |

YPNTKPGVKLFIN; PNTKPGVKLFINR; NTKPGVKLFINRI; TKPGVKLFINRIF;
KPGVKLFINRIFI; PGVKLFINRIFIT; GVKLFINRIFITD; VKLFINRIFITDS;
KLFINRIFITDSE; LFINRIFITDSEG; FINRIFITDSEGS; INRIFITDSEGSL;
NRIFITDSEGSLL; RIFITDSEGSLLP; IFITDSEGSLLPN; FITDSEGSLLPNY;
ITDSEGSLLPNYL; TDSEGSLLPNYLR; DSEGSLLPNYLRF; SEGSLLPNYLRFI;
EGSLLPNYLRFIK; GSLLPNYLRFIKG; SLLPNYLRFIKGI; LLPNYLRFIKGII;
LPNYLRFIKGIID; PNYLRFIKGIIDC; NYLRFIKGIIDCQ; YLRFIKGIIDCQD;
LRFIKGIIDCQDL; RFIKGIIDCQDLP; FIKGIIDCQDLPL; IKGIIDCQDLPLN;
KGIIDCQDLPLNV; GIIDCQDLPLNVS; IIDCQDLPLNVSR; IDCQDLPLNVSRE;
DCQDLPLNVSREI; CQDLPLNVSREIL; QDLPLNVSREILQ; DLPLNVSREILQQ;
LPLNVSREILQQN; PLNVSREILQQNK; LNVSREILQQNKI; NVSREILQQNKIL;
VSREILQQNKILS; SREILQQNKILSK; REILQQNKILSKI; EILQQNKILSKIK;
ILQQNKILSKIKS; LQQNKILSKIKSS; QQNKILSKIKSSS; QNKILSKIKSSSV;
NKILSKIKSSSVK; KILSKIKSSSVKK; ILSKIKSSSVKKI; LSKIKSSSVKKIL;
SKIKSSSVKKILS; KIKSSSVKKILSE; IKSSSVKKILSEL; KSSSVKKILSELE;
SSSVKKILSELEK; SSVKKILSELEKL; SVKKILSELEKLS; VKKILSELEKLSK;
KKILSELEKLSKK; KILSELEKLSKKN; ILSELEKLSKKNP; LSELEKLSKKNPE;
SELEKLSKKNPEK; ELEKLSKKNPEKF; LEKLSKKNPEKFS; EKLSKKNPEKFSE;
KLSKKNPEKFSEF; LSKKNPEKFSEFS; SKKNPEKFSEFSK; KKNPEKFSEFSKE;
KNPEKFSEFSKEF; NPEKFSEFSKEFG; PEKFSEFSKEFGR; EKFSEFSKEFGRC;
KFSEFSKEFGRCI; FSEFSKEFGRCIK; SEFSKEFGRCIKE; EFSKEFGRCIKEG;
FSKEFGRCIKEGV; SKEFGRCIKEGVY; KEFGRCIKEGVYS; EFGRCIKEGVYSD;
FGRCIKEGVYSDF; GRCIKEGVYSDFE; RCIKEGVYSDFEN; CIKEGVYSDFENR;
IKEGVYSDFENRE; KEGVYSDFENREK; EGVYSDFENREKL; GVYSDFENREKLI;
VYSDFENREKLIS; YSDFENREKLISL; SDFENREKLISLI; DFENREKLISLIR;
FENREKLISLIRF; ENREKLISLIRFK; NREKLISLIRFKS; REKLISLIRFKSS;
EKLISLIRFKSSS; KLISLIRFKSSSV; LISLIRFKSSSVD; ISLIRFKSSSVDG;
SLIRFKSSSVDGF; LIRFKSSSVDGFV; IRFKSSSVDGFVS; RFKSSSVDGFVSF;
FKSSSVDGFVSFK; KSSSVDGFVSFKE; SSSVDGFVSFKEY; SSVDGFVSFKEYK;
SVDGFVSFKEYKE; VDGFVSFKEYKER; DGFVSFKEYKERM; GFVSFKEYKERMN;
FVSFKEYKERMNE; VSFKEYKERMNES; SFKEYKERMNESQ; FKEYKERMNESQK;
KEYKERMNESQKS; EYKERMNESQKSI; YKERMNESQKSIY; KERMNESQKSIYY;
ERMNESQKSIYYI; RMNESQKSIYYIT; MNESQKSIYYITG; NESQKSIYYITGG;
ESQKSIYYITGGK; SQKSIYYITGGKE; QKSIYYITGGKEN; KSIYYITGGKENI;
SIYYITGGKENIL; IYYITGGKENILK; YYITGGKENILKE; YITGGKENILKEN;
ITGGKENILKENP; TGGKENILKENPI; GGKENILKENPIV; GKENILKENPIVA;
KENILKENPIVAA; ENILKENPIVAAY; NILKENPIVAAYK; ILKENPIVAAYKE;
LKENPIVAAYKEK; KENPIVAAYKEKG; ENPIVAAYKEKGF; NPIVAAYKEKGFE;
PIVAAYKEKGFEI; IVAAYKEKGFEIL; VAAYKEKGFEILI; AAYKEKGFEILIM;
AYKEKGFEILIMD; YKEKGFEILIMDD; KEKGFEILIMDDE; EKGFEILIMDDEL;
KGFEILIMDDELD; GFEILIMDDELDE; FEILIMDDELDEA; EILIMDDELDEAI;
ILIMDDELDEAIL; LIMDDELDEAILN; IMDDELDEAILNL; MDDELDEAILNLI;
DDELDEAILNLIP; DELDEAILNLIPE; ELDEAILNLIPEY; LDEAILNLIPEYE;
DEAILNLIPEYEG; EAILNLIPEYEGL; AILNLIPEYEGLK; ILNLIPEYEGLKL;
LNLIPEYEGLKLK; NLIPEYEGLKLKA; LIPEYEGLKLKAI; IPEYEGLKLKAIN;
PEYEGLKLKAINK; EYEGLKLKAINKN; YEGLKLKAINKNE; EGLKLKAINKNET;
GLKLKAINKNETS; LKLKAINKNETSN; KLKAINKNETSNE; LKAINKNETSNEL;
KAINKNETSNELK; AINKNETSNELKD; INKNETSNELKDE; NKNETSNELKDEN;
KNETSNELKDENF; NETSNELKDENFK; ETSNELKDENFKK; TSNELKDENFKKI;
SNELKDENFKKIE; NELKDENFKKIEE; ELKDENFKKIEEE; LKDENFKKIEEEF;
KDENFKKIEEEFK; DENFKKIEEEFKD; ENFKKIEEEFKDT; NFKKIEEEFKDTL;
FKKIEEEFKDTLT; KKIEEEFKDTLTK; KIEEEFKDTLTKV; IEEEFKDTLTKVK;
EEEFKDTLTKVKE; EEFKDTLTKVKEI; EFKDTLTKVKEIL; FKDTLTKVKEILK;
KDTLTKVKEILKD; DTLTKVKEILKDH; TLTKVKEILKDHI; LTKVKEILKDHIK;
TKVKEILKDHIKE; KVKEILKDHIKEV; VKEILKDHIKEVN; KEILKDHIKEVNL;
EILKDHIKEVNLS; ILKDHIKEVNLSA; LKDHIKEVNLSAT; KDHIKEVNLSATL;
DHIKEVNLSATLI; HIKEVNLSATLIK; IKEVNLSATLIKE; KEVNLSATLIKEP;

EP 2 254 592 B1

EVNLSATLIKEPS; VNLSATLIKEPSA; NLSATLIKEPSAI; LSATLIKEPSAII;
SATLIKEPSAIII; ATLIKEPSAIIID; TLIKEPSAIIIDS; LIKEPSAIIIDSN;
IKEPSAIIIDSND; KEPSAIIIDSNDP; EPSAIIIDSNDPT; PSAIIIDSNDPTY;
SAIIIDSNDPTYQ; AIIIDSNDPTYQM; IIIDSNDPTYQMQ; IIDSNDPTYQMQK;
IDSNDPTYQMQKI; DSNDPTYQMQKIM; SNDPTYQMQKIML; NDPTYQMQKIMLS;
DPTYQMQKIMLSM; PTYQMQKIMLSMG; TYQMQKIMLSMGQ; YQMQKIMLSMGQE;
QMQKIMLSMGQEV; MQKIMLSMGQEVK; QKIMLSMGQEVKE; KIMLSMGQEVKEI;
IMLSMGQEVKEIK; MLSMGQEVKEIKP; LSMGQEVKEIKPI; SMGQEVKEIKPIL;
MGQEVKEIKPILE; GQEVKEIKPILEL; QEVKEIKPILELN; EVKEIKPILELNP;
VKEIKPILELNPN; KEIKPILELNPNN; EIKPILELNPNNK; IKPILELNPNNKI;
KPILELNPNNKIV; PILELNPNNKIVQ; ILELNPNNKIVQN; LELNPNNKIVQNL;
ELNPNNKIVQNLK; LNPNNKIVQNLKN; NPNNKIVQNLKNL; PNNKIVQNLKNLE;
NNKIVQNLKNLEP; NKIVQNLKNLEPE; KIVQNLKNLEPEK; IVQNLKNLEPEKL;
VQNLKNLEPEKLE; QNLKNLEPEKLEK; NLKNLEPEKLEKI; LKNLEPEKLEKIS;
KNLEPEKLEKISI; NLEPEKLEKISIL; LEPEKLEKISILL; EPEKLEKISILLF;
PEKLEKISILLFE; EKLEKISILLFEE; KLEKISILLFEEA; LEKISILLFEEAM;
EKISILLFEEAML; KISILLFEEAMLT; ISILLFEEAMLTS; SILLFEEAMLTSG;
ILLFEEAMLTSGM; LLFEEAMLTSGMP; LFEEAMLTSGMPS; FEEAMLTSGMPSK;
EEAMLTSGMPSKN; EAMLTSGMPSKNP; AMLTSGMPSKNPG; MLTSGMPSKNPGK;
LTSGMPSKNPGKF; TSGMPSKNPGKFI; SGMPSKNPGKFIN; GMPSKNPGKFINI;
MPSKNPGKFINII; PSKNPGKFINIIN; SKNPGKFINIINE; KNPGKFINIINEF;
NPGKFINIINEFI; PGKFINIINEFIE; GKFINIINEFIEK; KFINIINEFIEKD;
FINIINEFIEKDF; INIINEFIEKDFL;

14 mers:
MILIFYFKQIALFI; ILIFYFKQIALFII; LIFYFKQIALFIIF;
IFYFKQIALFIIFR; FYFKQIALFIIFRL; YFKQIALFIIFRLC;
FKQIALFIIFRLCY; KQIALFIIFRLCYI; QIALFIIFRLCYII;
IALFIIFRLCYIIK; ALFIIFRLCYIIKK; LFIIFRLCYIIKKV;
FIIFRLCYIIKKVK; IIFRLCYIIKKVKI; IFRLCYIIKKVKIK;
FRLCYIIKKVKIKL; RLCYIIKKVKIKLK; LCYIIKKVKIKLKR;
CYIIKKVKIKLKRK; YIIKKVKIKLKRKS; IIKKVKIKLKRKSC;
IKKVKIKLKRKSCM; KKVKIKLKRKSCMK; KVKIKLKRKSCMKK;
VKIKLKRKSCMKKQ; KIKLKRKSCMKKQF; IKLKRKSCMKKQFD;
KLKRKSCMKKQFDT; LKRKSCMKKQFDTE; KRKSCMKKQFDTEV;
RKSCMKKQFDTEVN; KSCMKKQFDTEVND; SCMKKQFDTEVNDL;
CMKKQFDTEVNDLL; MKKQFDTEVNDLLY; KKQFDTEVNDLLYL;
KQFDTEVNDLLYLI; QFDTEVNDLLYLII; FDTEVNDLLYLIIH;
DTEVNDLLYLIIHS; TEVNDLLYLIIHSL; EVNDLLYLIIHSLY;
VNDLLYLIIHSLYS; NDLLYLIIHSLYSH; DLLYLIIHSLYSHK;
LLYLIIHSLYSHKE; LYLIIHSLYSHKEI; YLIIHSLYSHKEIF;
LIIHSLYSHKEIFL; IIHSLYSHKEIFLR; IHSLYSHKEIFLRE;
HSLYSHKEIFLREL; SLYSHKEIFLRELI; LYSHKEIFLRELIS;
YSHKEIFLRELISN; SHKEIFLRELISNA; HKEIFLRELISNAS;
KEIFLRELISNASD; EIFLRELISNASDA; IFLRELISNASDAI;
FLRELISNASDAID; LRELISNASDAIDK; RELISNASDAIDKL;
ELISNASDAIDKLK; LISNASDAIDKLKF; ISNASDAIDKLKFL;
SNASDAIDKLKFLS; NASDAIDKLKFLSL; ASDAIDKLKFLSLT;
SDAIDKLKFLSLTN; DAIDKLKFLSLTNE; AIDKLKFLSLTNEK;
IDKLKFLSLTNEKF; DKLKFLSLTNEKFK; KLKFLSLTNEKFKN;
LKFLSLTNEKFKNI; KFLSLTNEKFKNIA; FLSLTNEKFKNIAL;
LSLTNEKFKNIALE; SLTNEKFKNIALEP; LTNEKFKNIALEPK;
TNEKFKNIALEPKI; NEKFKNIALEPKIE; EKFKNIALEPKIEI;
KFKNIALEPKIEIS; FKNIALEPKIEISF; KNIALEPKIEISFD;
NIALEPKIEISFDD; IALEPKIEISFDDK; ALEPKIEISFDDKS;
LEPKIEISFDDKSI; EPKIEISFDDKSIL; PKIEISFDDKSILI;
KIEISFDDKSILIK; IEISFDDKSILIKD; EISFDDKSILIKDN;

ISFDDKSILIKDNG; SFDDKSILIKDNGI; FDDKSILIKDNGIG;
DDKSILIKDNGIGM; DKSILIKDNGIGMD; KSILIKDNGIGMDE;
SILIKDNGIGMDEQ; ILIKDNGIGMDEQD; LIKDNGIGMDEQDL;
IKDNGIGMDEQDLT; KDNGIGMDEQDLTN; DNGIGMDEQDLTNH;
NGIGMDEQDLTNHL; GIGMDEQDLTNHLG; IGMDEQDLTNHLGV;
GMDEQDLTNHLGVI; MDEQDLTNHLGVIA; DEQDLTNHLGVIAK;
EQDLTNHLGVIAKS; QDLTNHLGVIAKSG; DLTNHLGVIAKSGT;
LTNHLGVIAKSGTK; TNHLGVIAKSGTKE; NHLGVIAKSGTKEF;
HLGVIAKSGTKEFI; LGVIAKSGTKEFIN; GVIAKSGTKEFINN;
VIAKSGTKEFINNL; IAKSGTKEFINNLK; AKSGTKEFINNLKQ;
KSGTKEFINNLKQD; SGTKEFINNLKQDE; GTKEFINNLKQDEK;
TKEFINNLKQDEKK; KEFINNLKQDEKKS; EFINNLKQDEKKSA;
FINNLKQDEKKSAS; INNLKQDEKKSASL; NNLKQDEKKSASLI;
NLKQDEKKSASLIG; LKQDEKKSASLIGQ; KQDEKKSASLIGQF;
QDEKKSASLIGQFG; DEKKSASLIGQFGV; EKKSASLIGQFGVG;
KKSASLIGQFGVGF; KSASLIGQFGVGFY; SASLIGQFGVGFYS;
ASLIGQFGVGFYSA; SLIGQFGVGFYSAF; LIGQFGVGFYSAFI;
IGQFGVGFYSAFIV; GQFGVGFYSAFIVS; QFGVGFYSAFIVSE;
FGVGFYSAFIVSEK; GVGFYSAFIVSEKV; VGFYSAFIVSEKVE;
GFYSAFIVSEKVEV; FYSAFIVSEKVEVT; YSAFIVSEKVEVTS;
SAFIVSEKVEVTSK; AFIVSEKVEVTSKK; FIVSEKVEVTSKKA;
IVSEKVEVTSKKAL; VSEKVEVTSKKALE; SEKVEVTSKKALES;
EKVEVTSKKALESD; KVEVTSKKALESDA; VEVTSKKALESDAY;
EVTSKKALESDAYI; VTSKKALESDAYIW; TSKKALESDAYIWS;
SKKALESDAYIWSS; KKALESDAYIWSSD; KALESDAYIWSSDG;
ALESDAYIWSSDGK; LESDAYIWSSDGKT; ESDAYIWSSDGKTG;
SDAYIWSSDGKTGY; DAYIWSSDGKTGYE; AYIWSSDGKTGYEI;
YIWSSDGKTGYEIE; IWSSDGKTGYEIEK; WSSDGKTGYEIEKA;
SSDGKTGYEIEKAK; SDGKTGYEIEKAKK; DGKTGYEIEKAKKE;
GKTGYEIEKAKKEE; KTGYEIEKAKKEES; TGYEIEKAKKEESG;
GYEIEKAKKEESGT; YEIEKAKKEESGTE; EIEKAKKEESGTEI;
IEKAKKEESGTEIK; EKAKKEESGTEIKL; KAKKEESGTEIKLY;
AKKEESGTEIKLYL; KKEESGTEIKLYLN; KEESGTEIKLYLNK;
EESGTEIKLYLNKE; ESGTEIKLYLNKEG; SGTEIKLYLNKEGL;
GTEIKLYLNKEGLE; TEIKLYLNKEGLEY; EIKLYLNKEGLEYA;
IKLYLNKEGLEYAN; KLYLNKEGLEYANK; LYLNKEGLEYANKW;
YLNKEGLEYANKWK; LNKEGLEYANKWKI; NKEGLEYANKWKIQ;
KEGLEYANKWKIQE; EGLEYANKWKIQEI; GLEYANKWKIQEII;
LEYANKWKIQEIIK; EYANKWKIQEIIKK; YANKWKIQEIIKKY;
ANKWKIQEIIKKYS; NKWKIQEIIKKYSN; KWKIQEIIKKYSNH;
WKIQEIIKKYSNHI; KIQEIIKKYSNHIN; IQEIIKKYSNHINY;
QEIIKKYSNHINYP; EIIKKYSNHINYPI; IIKKYSNHINYPIY;
IKKYSNHINYPIYI; KKYSNHINYPIYIK; KYSNHINYPIYIKY;
YSNHINYPIYIKYS; SNHINYPIYIKYSE; NHINYPIYIKYSEP;
HINYPIYIKYSEPI; INYPIYIKYSEPIM; NYPIYIKYSEPIMK;
YPIYIKYSEPIMKD; PIYIKYSEPIMKDG; IYIKYSEPIMKDGK;
YIKYSEPIMKDGKQ; IKYSEPIMKDGKQE; KYSEPIMKDGKQEG;
YSEPIMKDGKQEGI; SEPIMKDGKQEGIE; EPIMKDGKQEGIEE;
PIMKDGKQEGIEEK; IMKDGKQEGIEEKE; MKDGKQEGIEEKEE;
KDGKQEGIEEKEEK; DGKQEGIEEKEEKL; GKQEGIEEKEEKLN;
KQEGIEEKEEKLNE; QEGIEEKEEKLNET; EGIEEKEEKLNETT;
GIEEKEEKLNETTA; IEEKEEKLNETTAL; EEKEEKLNETTALW;
EKEEKLNETTALWT; KEEKLNETTALWTK; EEKLNETTALWTKN;
EKLNETTALWTKNK; KLNETTALWTKNKS; LNETTALWTKNKSE;
NETTALWTKNKSEI; ETTALWTKNKSEIK; TTALWTKNKSEIKA;
TALWTKNKSEIKAE; ALWTKNKSEIKAEE; LWTKNKSEIKAEEY;
WTKNKSEIKAEEYN; TKNKSEIKAEEYNE; KNKSEIKAEEYNEF;

NKSEIKAEEYNEFY; KSEIKAEEYNEFYK; SEIKAEEYNEFYKN;
EIKAEEYNEFYKNT; IKAEEYNEFYKNTT; KAEEYNEFYKNTTF;
AEEYNEFYKNTTFD; EEYNEFYKNTTFDY; EYNEFYKNTTFDYE;
YNEFYKNTTFDYEN; NEFYKNTTFDYENP; EFYKNTTFDYENPL;
FYKNTTFDYENPLM; YKNTTFDYENPLMH; KNTTFDYENPLMHI;
NTTFDYENPLMHIH; TTFDYENPLMHIHT; TFDYENPLMHIHTK;
FDYENPLMHIHTKA; DYENPLMHIHTKAE; YENPLMHIHTKAEG;
ENPLMHIHTKAEGN; NPLMHIHTKAEGNL; PLMHIHTKAEGNLE;
LMHIHTKAEGNLEY; MHIHTKAEGNLEYT; HIHTKAEGNLEYTN;
IHTKAEGNLEYTNL; HTKAEGNLEYTNLF; TKAEGNLEYTNLFY;
KAEGNLEYTNLFYV; AEGNLEYTNLFYVP; EGNLEYTNLFYVPS;
GNLEYTNLFYVPSK; NLEYTNLFYVPSKA; LEYTNLFYVPSKAP;
EYTNLFYVPSKAPY; YTNLFYVPSKAPYD; TNLFYVPSKAPYDL;
NLFYVPSKAPYDLY; LFYVPSKAPYDLYY; FYVPSKAPYDLYYP;
YVPSKAPYDLYYPN; VPSKAPYDLYYPNT; PSKAPYDLYYPNTK;
SKAPYDLYYPNTKP; KAPYDLYYPNTKPG; APYDLYYPNTKPGV;
PYDLYYPNTKPGVK; YDLYYPNTKPGVKL; DLYYPNTKPGVKLF;
LYYPNTKPGVKLFI; YYPNTKPGVKLFIN; YPNTKPGVKLFINR;
PNTKPGVKLFINRI; NTKPGVKLFINRIF; TKPGVKLFINRIFI;
KPGVKLFINRIFIT; PGVKLFINRIFITD; GVKLFINRIFITDS;
VKLFINRIFITDSE; KLFINRIFITDSEG; LFINRIFITDSEGS;
FINRIFITDSEGSL; INRIFITDSEGSLL; NRIFITDSEGSLLP;
RIFITDSEGSLLPN; IFITDSEGSLLPNY; FITDSEGSLLPNYL;
ITDSEGSLLPNYLR; TDSEGSLLPNYLRF; DSEGSLLPNYLRFI;
SEGSLLPNYLRFIK; EGSLLPNYLRFIKG; GSLLPNYLRFIKGI;
SLLPNYLRFIKGII; LLPNYLRFIKGIID; LPNYLRFIKGIIDC;
PNYLRFIKGIIDCQ; NYLRFIKGIIDCQD; YLRFIKGIIDCQDL;
LRFIKGIIDCQDLP; RFIKGIIDCQDLPL; FIKGIIDCQDLPLN;
IKGIIDCQDLPLNV; KGIIDCQDLPLNVS; GIIDCQDLPLNVSR;
IIDCQDLPLNVSRE; IDCQDLPLNVSREI; DCQDLPLNVSREIL;
CQDLPLNVSREILQ; QDLPLNVSREILQQ; DLPLNVSREILQQN;
LPLNVSREILQQNK; PLNVSREILQQNKI; LNVSREILQQNKIL;
NVSREILQQNKILS; VSREILQQNKILSK; SREILQQNKILSKI;
REILQQNKILSKIK; EILQQNKILSKIKS; ILQQNKILSKIKSS;
LQQNKILSKIKSSS; QQNKILSKIKSSSV; QNKILSKIKSSSVK;
NKILSKIKSSSVKK; KILSKIKSSSVKKI; ILSKIKSSSVKKIL;
LSKIKSSSVKKILS; SKIKSSSVKKILSE; KIKSSSVKKILSEL;
IKSSSVKKILSELE; KSSSVKKILSELEK; SSSVKKILSELEKL;
SSVKKILSELEKLS; SVKKILSELEKLSK; VKKILSELEKLSKK;
KKILSELEKLSKKN; KILSELEKLSKKNP; ILSELEKLSKKNPE;
LSELEKLSKKNPEK; SELEKLSKKNPEKF; ELEKLSKKNPEKFS;
LEKLSKKNPEKFSE; EKLSKKNPEKFSEF; KLSKKNPEKFSEFS;
LSKKNPEKFSEFSK; SKKNPEKFSEFSKE; KKNPEKFSEFSKEF;
KNPEKFSEFSKEFG; NPEKFSEFSKEFGR; PEKFSEFSKEFGRC;
EKFSEFSKEFGRCI; KFSEFSKEFGRCIK; FSEFSKEFGRCIKE;
SEFSKEFGRCIKEG; EFSKEFGRCIKEGV; FSKEFGRCIKEGVY;
SKEFGRCIKEGVYS; KEFGRCIKEGVYSD; EFGRCIKEGVYSDF;
FGRCIKEGVYSDFE; GRCIKEGVYSDFEN; RCIKEGVYSDFENR;
CIKEGVYSDFENRE; IKEGVYSDFENREK; KEGVYSDFENREKL;
EGVYSDFENREKLI; GVYSDFENREKLIS; VYSDFENREKLISL;
YSDFENREKLISLI; SDFENREKLISLIR; DFENREKLISLIRF;
FENREKLISLIRFK; ENREKLISLIRFKS; NREKLISLIRFKSS;
REKLISLIRFKSSS; EKLISLIRFKSSSV; KLISLIRFKSSSVD;
LISLIRFKSSSVDG; ISLIRFKSSSVDGF; SLIRFKSSSVDGFV;
LIRFKSSSVDGFVS; IRFKSSSVDGFVSF; RFKSSSVDGFVSFK;
FKSSSVDGFVSFKE; KSSSVDGFVSFKEY; SSSVDGFVSFKEYK;
SSVDGFVSFKEYKE; SVDGFVSFKEYKER; VDGFVSFKEYKERM;

DGFVSFKEYKERMN; GFVSFKEYKERMNE; FVSFKEYKERMNES;
VSFKEYKERMNESQ; SFKEYKERMNESQK; FKEYKERMNESQKS;
KEYKERMNESQKSI; EYKERMNESQKSIY; YKERMNESQKSIYY;
KERMNESQKSIYYI; ERMNESQKSIYYIT; RMNESQKSIYYITG;
MNESQKSIYYITGG; NESQKSIYYITGGK; ESQKSIYYITGGKE;
SQKSIYYITGGKEN; QKSIYYITGGKENI; KSIYYITGGKENIL;
SIYYITGGKENILK; IYYITGGKENILKE; YYITGGKENILKEN;
YITGGKENILKENP; ITGGKENILKENPI; TGGKENILKENPIV;
GGKENILKENPIVA; GKENILKENPIVAA; KENILKENPIVAAY;
ENILKENPIVAAYK; NILKENPIVAAYKE; ILKENPIVAAYKEK;
LKENPIVAAYKEKG; KENPIVAAYKEKGF; ENPIVAAYKEKGFE;
NPIVAAYKEKGFEI; PIVAAYKEKGFEIL; IVAAYKEKGFEILI;
VAAYKEKGFEILIM; AAYKEKGFEILIMD; AYKEKGFEILIMDD;
YKEKGFEILIMDDE; KEKGFEILIMDDEL; EKGFEILIMDDELD;
KGFEILIMDDELDE; GFEILIMDDELDEA; FEILIMDDELDEAI;
EILIMDDELDEAIL; ILIMDDELDEAILN; LIMDDELDEAILNL;
IMDDELDEAILNLI; MDDELDEAILNLIP; DDELDEAILNLIPE;
DELDEAILNLIPEY; ELDEAILNLIPEYE; LDEAILNLIPEYEG;
DEAILNLIPEYEGL; EAILNLIPEYEGLK; AILNLIPEYEGLKL;
ILNLIPEYEGLKLK; LNLIPEYEGLKLKA; NLIPEYEGLKLKAI;
LIPEYEGLKLKAIN; IPEYEGLKLKAINK; PEYEGLKLKAINKN;
EYEGLKLKAINKNE; YEGLKLKAINKNET; EGLKLKAINKNETS;
GLKLKAINKNETSN; LKLKAINKNETSNE; KLKAINKNETSNEL;
LKAINKNETSNELK; KAINKNETSNELKD; AINKNETSNELKDE;
INKNETSNELKDEN; NKNETSNELKDENF; KNETSNELKDENFK;
NETSNELKDENFKK; ETSNELKDENFKKI; TSNELKDENFKKIE;
SNELKDENFKKIEE; NELKDENFKKIEEE; ELKDENFKKIEEEF;
LKDENFKKIEEEFK; KDENFKKIEEEFKD; DENFKKIEEEFKDT;
ENFKKIEEEFKDTL; NFKKIEEEFKDTLT; FKKIEEEFKDTLTK;
KKIEEEFKDTLTKV; KIEEEFKDTLTKVK; IEEEFKDTLTKVKE;
EEEFKDTLTKVKEI; EEFKDTLTKVKEIL; EFKDTLTKVKEILK;
FKDTLTKVKEILKD; KDTLTKVKEILKDH; DTLTKVKEILKDHI;
TLTKVKEILKDHIK; LTKVKEILKDHIKE; TKVKEILKDHIKEV;
KVKEILKDHIKEVN; VKEILKDHIKEVNL; KEILKDHIKEVNLS;
EILKDHIKEVNLSA; ILKDHIKEVNLSAT; LKDHIKEVNLSATL;
KDHIKEVNLSATLI; DHIKEVNLSATLIK; HIKEVNLSATLIKE;
IKEVNLSATLIKEP; KEVNLSATLIKEPS; EVNLSATLIKEPSA;
VNLSATLIKEPSAI; NLSATLIKEPSAII; LSATLIKEPSAIII;
SATLIKEPSAIIID; ATLIKEPSAIIIDS; TLIKEPSAIIIDSN;
LIKEPSAIIIDSND; IKEPSAIIIDSNDP; KEPSAIIIDSNDPT;
EPSAIIIDSNDPTY; PSAIIIDSNDPTYQ; SAIIIDSNDPTYQM;
AIIIDSNDPTYQMQ; IIIDSNDPTYQMQK; IIDSNDPTYQMQKI;
IDSNDPTYQMQKIM; DSNDPTYQMQKIML; SNDPTYQMQKIMLS;
NDPTYQMQKIMLSM; DPTYQMQKIMLSMG; PTYQMQKIMLSMGQ;
TYQMQKIMLSMGQE; YQMQKIMLSMGQEV; QMQKIMLSMGQEVK;
MQKIMLSMGQEVKE; QKIMLSMGQEVKEI; KIMLSMGQEVKEIK;
IMLSMGQEVKEIKP; MLSMGQEVKEIKPI; LSMGQEVKEIKPIL;
SMGQEVKEIKPILE; MGQEVKEIKPILEL; GQEVKEIKPILELN;
QEVKEIKPILELNP; EVKEIKPILELNPN; VKEIKPILELNPNN;
KEIKPILELNPNNK; EIKPILELNPNNKI; IKPILELNPNNKIV;
KPILELNPNNKIVQ; PILELNPNNKIVQN; ILELNPNNKIVQNL;
LELNPNNKIVQNLK; ELNPNNKIVQNLKN; LNPNNKIVQNLKNL;
NPNNKIVQNLKNLE; PNNKIVQNLKNLEP; NNKIVQNLKNLEPE;
NKIVQNLKNLEPEK; KIVQNLKNLEPEKL; IVQNLKNLEPEKLE;
VQNLKNLEPEKLEK; QNLKNLEPEKLEKI; NLKNLEPEKLEKIS;
LKNLEPEKLEKISI; KNLEPEKLEKISIL; NLEPEKLEKISILL;
LEPEKLEKISILLF; EPEKLEKISILLFE; PEKLEKISILLFEE;

EKLEKISILLFEEA; KLEKISILLFEEAM; LEKISILLFEEAML;
EKISILLFEEAMLT; KISILLFEEAMLTS; ISILLFEEAMLTSG;
SILLFEEAMLTSGM; ILLFEEAMLTSGMP; LLFEEAMLTSGMPS;
LFEEAMLTSGMPSK; FEEAMLTSGMPSKN; EEAMLTSGMPSKNP;
EAMLTSGMPSKNPG; AMLTSGMPSKNPGK; MLTSGMPSKNPGKF;
LTSGMPSKNPGKFI; TSGMPSKNPGKFIN; SGMPSKNPGKFINI;
GMPSKNPGKFINII; MPSKNPGKFINIIN; PSKNPGKFINIINE;
SKNPGKFINIINEF; KNPGKFINIINEFI; NPGKFINIINEFIE;
PGKFINIINEFIEK; GKFINIINEFIEKD; KFINIINEFIEKDF;
FINIINEFIEKDFL;

15 mers:
MILIFYFKQIALFII; ILIFYFKQIALFIIF; LIFYFKQIALFIIFR;
IFYFKQIALFIIFRL; FYFKQIALFIIFRLC; YFKQIALFIIFRLCY;
FKQIALFIIFRLCYI; KQIALFIIFRLCYII; QIALFIIFRLCYIIK;
IALFIIFRLCYIIKK; ALFIIFRLCYIIKKV; LFIIFRLCYIIKKVK;
FIIFRLCYIIKKVKI; IIFRLCYIIKKVKIK; IFRLCYIIKKVKIKL;
FRLCYIIKKVKIKLK; RLCYIIKKVKIKLKR; LCYIIKKVKIKLKRK;
CYIIKKVKIKLKRKS; YIIKKVKIKLKRKSC; IIKKVKIKLKRKSCM;
IKKVKIKLKRKSCMK; KKVKIKLKRKSCMKK; KVKIKLKRKSCMKKQ;
VKIKLKRKSCMKKQF; KIKLKRKSCMKKQFD; IKLKRKSCMKKQFDT;
KLKRKSCMKKQFDTE; LKRKSCMKKQFDTEV; KRKSCMKKQFDTEVN;
RKSCMKKQFDTEVND; KSCMKKQFDTEVNDL; SCMKKQFDTEVNDLL;
CMKKQFDTEVNDLLY; MKKQFDTEVNDLLYL; KKQFDTEVNDLLYLI;
KQFDTEVNDLLYLII; QFDTEVNDLLYLIIH; FDTEVNDLLYLIIHS;
DTEVNDLLYLIIHSL; TEVNDLLYLIIHSLY; EVNDLLYLIIHSLYS;
VNDLLYLIIHSLYSH; NDLLYLIIHSLYSHK; DLLYLIIHSLYSHKE;
LLYLIIHSLYSHKEI; LYLIIHSLYSHKEIF; YLIIHSLYSHKEIFL;
LIIHSLYSHKEIFLR; IIHSLYSHKEIFLRE; IHSLYSHKEIFLREL;
HSLYSHKEIFLRELI; SLYSHKEIFLRELIS; LYSHKEIFLRELISN;
YSHKEIFLRELISNA; SHKEIFLRELISNAS; HKEIFLRELISNASD;
KEIFLRELISNASDA; EIFLRELISNASDAI; IFLRELISNASDAID;
FLRELISNASDAIDK; LRELISNASDAIDKL; RELISNASDAIDKLK;
ELISNASDAIDKLKF; LISNASDAIDKLKFL; ISNASDAIDKLKFLS;
SNASDAIDKLKFLSL; NASDAIDKLKFLSLT; ASDAIDKLKFLSLTN;
SDAIDKLKFLSLTNE; DAIDKLKFLSLTNEK; AIDKLKFLSLTNEKF;
IDKLKFLSLTNEKFK; DKLKFLSLTNEKFKN; KLKFLSLTNEKFKNI;
LKFLSLTNEKFKNIA; KFLSLTNEKFKNIAL; FLSLTNEKFKNIALE;
LSLTNEKFKNIALEP; SLTNEKFKNIALEPK; LTNEKFKNIALEPKI;
TNEKFKNIALEPKIE; NEKFKNIALEPKIEI; EKFKNIALEPKIEIS;
KFKNIALEPKIEISF; FKNIALEPKIEISFD; KNIALEPKIEISFDD;
NIALEPKIEISFDDK; IALEPKIEISFDDKS; ALEPKIEISFDDKSI;
LEPKIEISFDDKSIL; EPKIEISFDDKSILI; PKIEISFDDKSILIK;
KIEISFDDKSILIKD; IEISFDDKSILIKDN; EISFDDKSILIKDNG;
ISFDDKSILIKDNGI; SFDDKSILIKDNGIG; FDDKSILIKDNGIGM;
DDKSILIKDNGIGMD; DKSILIKDNGIGMDE; KSILIKDNGIGMDEQ;
SILIKDNGIGMDEQD; ILIKDNGIGMDEQDL; LIKDNGIGMDEQDLT;
IKDNGIGMDEQDLTN; KDNGIGMDEQDLTNH; DNGIGMDEQDLTNHL;
NGIGMDEQDLTNHLG; GIGMDEQDLTNHLGV; IGMDEQDLTNHLGVI;
GMDEQDLTNHLGVIA; MDEQDLTNHLGVIAK; DEQDLTNHLGVIAKS;
EQDLTNHLGVIAKSG; QDLTNHLGVIAKSGT; DLTNHLGVIAKSGTK;
LTNHLGVIAKSGTKE; TNHLGVIAKSGTKEF; NHLGVIAKSGTKEFI;
HLGVIAKSGTKEFIN; LGVIAKSGTKEFINN; GVIAKSGTKEFINNL;
VIAKSGTKEFINNLK; IAKSGTKEFINNLKQ; AKSGTKEFINNLKQD;
KSGTKEFINNLKQDE; SGTKEFINNLKQDEK; GTKEFINNLKQDEKK;
TKEFINNLKQDEKKS; KEFINNLKQDEKKSA; EFINNLKQDEKKSAS;
FINNLKQDEKKSASL; INNLKQDEKKSASLI; NNLKQDEKKSASLIG;

NLKQDEKKSASLIGQ; LKQDEKKSASLIGQF; KQDEKKSASLIGQFG;
QDEKKSASLIGQFGV; DEKKSASLIGQFGVG; EKKSASLIGQFGVGF;
KKSASLIGQFGVGFY; KSASLIGQFGVGFYS; SASLIGQFGVGFYSA;
ASLIGQFGVGFYSAF; SLIGQFGVGFYSAFI; LIGQFGVGFYSAFIV;
IGQFGVGFYSAFIVS; GQFGVGFYSAFIVSE; QFGVGFYSAFIVSEK;
FGVGFYSAFIVSEKV; GVGFYSAFIVSEKVE; VGFYSAFIVSEKVEV;
GFYSAFIVSEKVEVT; FYSAFIVSEKVEVTS; YSAFIVSEKVEVTSK;
SAFIVSEKVEVTSKK; AFIVSEKVEVTSKKA; FIVSEKVEVTSKKAL;
IVSEKVEVTSKKALE; VSEKVEVTSKKALES; SEKVEVTSKKALESD;
EKVEVTSKKALESDA; KVEVTSKKALESDAY; VEVTSKKALESDAYI;
EVTSKKALESDAYIW; VTSKKALESDAYIWS; TSKKALESDAYIWSS;
SKKALESDAYIWSSD; KKALESDAYIWSSDG; KALESDAYIWSSDGK;
ALESDAYIWSSDGKT; LESDAYIWSSDGKTG; ESDAYIWSSDGKTGY;
SDAYIWSSDGKTGYE; DAYIWSSDGKTGYEI; AYIWSSDGKTGYEIE;
YIWSSDGKTGYEIEK; IWSSDGKTGYEIEKA; WSSDGKTGYEIEKAK;
SSDGKTGYEIEKAKK; SDGKTGYEIEKAKKE; DGKTGYEIEKAKKEE;
GKTGYEIEKAKKEES; KTGYEIEKAKKEESG; TGYEIEKAKKEESGT;
GYEIEKAKKEESGTE; YEIEKAKKEESGTEI; EIEKAKKEESGTEIK;
IEKAKKEESGTEIKL; EKAKKEESGTEIKLY; KAKKEESGTEIKLYL;
AKKEESGTEIKLYLN; KKEESGTEIKLYLNK; KEESGTEIKLYLNKE;
EESGTEIKLYLNKEG; ESGTEIKLYLNKEGL; SGTEIKLYLNKEGLE;
GTEIKLYLNKEGLEY; TEIKLYLNKEGLEYA; EIKLYLNKEGLEYAN;
IKLYLNKEGLEYANK; KLYLNKEGLEYANKW; LYLNKEGLEYANKWK;
YLNKEGLEYANKWKI; LNKEGLEYANKWKIQ; NKEGLEYANKWKIQE;
KEGLEYANKWKIQEI; EGLEYANKWKIQEII; GLEYANKWKIQEIIK;
LEYANKWKIQEIIKK; EYANKWKIQEIIKKY; YANKWKIQEIIKKYS;
ANKWKIQEIIKKYSN; NKWKIQEIIKKYSNH; KWKIQEIIKKYSNHI;
WKIQEIIKKYSNHIN; KIQEIIKKYSNHINY; IQEIIKKYSNHINYP;
QEIIKKYSNHINYPI; EIIKKYSNHINYPIY; IIKKYSNHINYPIYI;
IKKYSNHINYPIYIK; KKYSNHINYPIYIKY; KYSNHINYPIYIKYS;
YSNHINYPIYIKYSE; SNHINYPIYIKYSEP; NHINYPIYIKYSEPI;
HINYPIYIKYSEPIM; INYPIYIKYSEPIMK; NYPIYIKYSEPIMKD;
YPIYIKYSEPIMKDG; PIYIKYSEPIMKDGK; IYIKYSEPIMKDGKQ;
YIKYSEPIMKDGKQE; IKYSEPIMKDGKQEG; KYSEPIMKDGKQEGI;
YSEPIMKDGKQEGIE; SEPIMKDGKQEGIEE; EPIMKDGKQEGIEEK;
PIMKDGKQEGIEEKE; IMKDGKQEGIEEKEE; MKDGKQEGIEEKEEK;
KDGKQEGIEEKEEKL; DGKQEGIEEKEEKLN; GKQEGIEEKEEKLNE;
KQEGIEEKEEKLNET; QEGIEEKEEKLNETT; EGIEEKEEKLNETTA;
GIEEKEEKLNETTAL; IEEKEEKLNETTALW; EEKEEKLNETTALWT;
EKEEKLNETTALWTK; KEEKLNETTALWTKN; EEKLNETTALWTKNK;
EKLNETTALWTKNKS; KLNETTALWTKNKSE; LNETTALWTKNKSEI;
NETTALWTKNKSEIK; ETTALWTKNKSEIKA; TTALWTKNKSEIKAE;
TALWTKNKSEIKAEE; ALWTKNKSEIKAEEY; LWTKNKSEIKAEEYN;
WTKNKSEIKAEEYNE; TKNKSEIKAEEYNEF; KNKSEIKAEEYNEFY;
NKSEIKAEEYNEFYK; KSEIKAEEYNEFYKN; SEIKAEEYNEFYKNT;
EIKAEEYNEFYKNTT; IKAEEYNEFYKNTTF; KAEEYNEFYKNTTFD;
AEEYNEFYKNTTFDY; EEYNEFYKNTTFDYE; EYNEFYKNTTFDYEN;
YNEFYKNTTFDYENP; NEFYKNTTFDYENPL; EFYKNTTFDYENPLM;
FYKNTTFDYENPLMH; YKNTTFDYENPLMHI; KNTTFDYENPLMHIH;
NTTFDYENPLMHIHT; TTFDYENPLMHIHTK; TFDYENPLMHIHTKA;
FDYENPLMHIHTKAE; DYENPLMHIHTKAEG; YENPLMHIHTKAEGN;
ENPLMHIHTKAEGNL; NPLMHIHTKAEGNLE; PLMHIHTKAEGNLEY;
LMHIHTKAEGNLEYT; MHIHTKAEGNLEYTN; HIHTKAEGNLEYTNL;
IHTKAEGNLEYTNLF; HTKAEGNLEYTNLFY; TKAEGNLEYTNLFYV;
KAEGNLEYTNLFYVP; AEGNLEYTNLFYVPS; EGNLEYTNLFYVPSK;
GNLEYTNLFYVPSKA; NLEYTNLFYVPSKAP; LEYTNLFYVPSKAPY;
EYTNLFYVPSKAPYD; YTNLFYVPSKAPYDL; TNLFYVPSKAPYDLY;

NLFYVPSKAPYDLYY; LFYVPSKAPYDLYYP; FYVPSKAPYDLYYPN;
YVPSKAPYDLYYPNT; VPSKAPYDLYYPNTK; PSKAPYDLYYPNTKP;
SKAPYDLYYPNTKPG; KAPYDLYYPNTKPGV; APYDLYYPNTKPGVK;
PYDLYYPNTKPGVKL; YDLYYPNTKPGVKLF; DLYYPNTKPGVKLFI;
LYYPNTKPGVKLFIN; YYPNTKPGVKLFINR; YPNTKPGVKLFINRI;
PNTKPGVKLFINRIF; NTKPGVKLFINRIFI; TKPGVKLFINRIFIT;
KPGVKLFINRIFITD; PGVKLFINRIFITDS; GVKLFINRIFITDSE;
VKLFINRIFITDSEG; KLFINRIFITDSEGS; LFINRIFITDSEGSL;
FINRIFITDSEGSLL; INRIFITDSEGSLLP; NRIFITDSEGSLLPN;
RIFITDSEGSLLPNY; IFITDSEGSLLPNYL; FITDSEGSLLPNYLR;
ITDSEGSLLPNYLRF; TDSEGSLLPNYLRFI; DSEGSLLPNYLRFIK;
SEGSLLPNYLRFIKG; EGSLLPNYLRFIKGI; GSLLPNYLRFIKGII;
SLLPNYLRFIKGIID; LLPNYLRFIKGIIDC; LPNYLRFIKGIIDCQ;
PNYLRFIKGIIDCQD; NYLRFIKGIIDCQDL; YLRFIKGIIDCQDLP;
LRFIKGIIDCQDLPL; RFIKGIIDCQDLPLN; FIKGIIDCQDLPLNV;
IKGIIDCQDLPLNVS; KGIIDCQDLPLNVSR; GIIDCQDLPLNVSRE;
IIDCQDLPLNVSREI; IDCQDLPLNVSREIL; DCQDLPLNVSREILQ;
CQDLPLNVSREILQQ; QDLPLNVSREILQQN; DLPLNVSREILQQNK;
LPLNVSREILQQNKI; PLNVSREILQQNKIL; LNVSREILQQNKILS;
NVSREILQQNKILSK; VSREILQQNKILSKI; SREILQQNKILSKIK;
REILQQNKILSKIKS; EILQQNKILSKIKSS; ILQQNKILSKIKSSS;
LQQNKILSKIKSSSV; QQNKILSKIKSSSVK; QNKILSKIKSSSVKK;
NKILSKIKSSSVKKI; KILSKIKSSSVKKIL; ILSKIKSSSVKKILS;
LSKIKSSSVKKILSE; SKIKSSSVKKILSEL; KIKSSSVKKILSELE;
IKSSSVKKILSELEK; KSSSVKKILSELEKL; SSSVKKILSELEKLS;
SSVKKILSELEKLSK; SVKKILSELEKLSKK; VKKILSELEKLSKKN;
KKILSELEKLSKKNP; KILSELEKLSKKNPE; ILSELEKLSKKNPEK;
LSELEKLSKKNPEKF; SELEKLSKKNPEKFS; ELEKLSKKNPEKFSE;
LEKLSKKNPEKFSEF; EKLSKKNPEKFSEFS; KLSKKNPEKFSEFSK;
LSKKNPEKFSEFSKE; SKKNPEKFSEFSKEF; KKNPEKFSEFSKEFG;
KNPEKFSEFSKEFGR; NPEKFSEFSKEFGRC; PEKFSEFSKEFGRCI;
EKFSEFSKEFGRCIK; KFSEFSKEFGRCIKE; FSEFSKEFGRCIKEG;
SEFSKEFGRCIKEGV; EFSKEFGRCIKEGVY; FSKEFGRCIKEGVYS;
SKEFGRCIKEGVYSD; KEFGRCIKEGVYSDF; EFGRCIKEGVYSDFE;
FGRCIKEGVYSDFEN; GRCIKEGVYSDFENR; RCIKEGVYSDFENRE;
CIKEGVYSDFENREK; IKEGVYSDFENREKL; KEGVYSDFENREKLI;
EGVYSDFENREKLIS; GVYSDFENREKLISL; VYSDFENREKLISLI;
YSDFENREKLISLIR; SDFENREKLISLIRF; DFENREKLISLIRFK;
FENREKLISLIRFKS; ENREKLISLIRFKSS; NREKLISLIRFKSSS;
REKLISLIRFKSSSV; EKLISLIRFKSSSVD; KLISLIRFKSSSVDG;
LISLIRFKSSSVDGF; ISLIRFKSSSVDGFV; SLIRFKSSSVDGFVS;
LIRFKSSSVDGFVSF; IRFKSSSVDGFVSFK; RFKSSSVDGFVSFKE;
FKSSSVDGFVSFKEY; KSSSVDGFVSFKEYK; SSSVDGFVSFKEYKE;
SSVDGFVSFKEYKER; SVDGFVSFKEYKERM; VDGFVSFKEYKERMN;
DGFVSFKEYKERMNE; GFVSFKEYKERMNES; FVSFKEYKERMNESQ;
VSFKEYKERMNESQK; SFKEYKERMNESQKS; FKEYKERMNESQKSI;
KEYKERMNESQKSIY; EYKERMNESQKSIYY; YKERMNESQKSIYYI;
KERMNESQKSIYYIT; ERMNESQKSIYYITG; RMNESQKSIYYITGG;
MNESQKSIYYITGGK; NESQKSIYYITGGKE; ESQKSIYYITGGKEN;
SQKSIYYITGGKENI; QKSIYYITGGKENIL; KSIYYITGGKENILK;
SIYYITGGKENILKE; IYYITGGKENILKEN; YYITGGKENILKENP;
YITGGKENILKENPI; ITGGKENILKENPIV; TGGKENILKENPIVA;
GGKENILKENPIVAA; GKENILKENPIVAAY; KENILKENPIVAAYK;
ENILKENPIVAAYKE; NILKENPIVAAYKEK; ILKENPIVAAYKEKG;
LKENPIVAAYKEKGF; KENPIVAAYKEKGFE; ENPIVAAYKEKGFEI;
NPIVAAYKEKGFEIL; PIVAAYKEKGFEILI; IVAAYKEKGFEILIM;
VAAYKEKGFEILIMD; AAYKEKGFEILIMDD; AYKEKGFEILIMDDE;

```
YKEKGFEILIMDDEL; KEKGFEILIMDDELD; EKGFEILIMDDELDE;
KGFEILIMDDELDEA; GFEILIMDDELDEAI; FEILIMDDELDEAIL;
EILIMDDELDEAILN; ILIMDDELDEAILNL; LIMDDELDEAILNLI;
IMDDELDEAILNLIP; MDDELDEAILNLIPE; DDELDEAILNLIPEY;
DELDEAILNLIPEYE; ELDEAILNLIPEYEG; LDEAILNLIPEYEGL;
DEAILNLIPEYEGLK; EAILNLIPEYEGLKL; AILNLIPEYEGLKLK;
ILNLIPEYEGLKLKA; LNLIPEYEGLKLKAI; NLIPEYEGLKLKAIN;
LIPEYEGLKLKAINK; IPEYEGLKLKAINKN; PEYEGLKLKAINKNE;
EYEGLKLKAINKNET; YEGLKLKAINKNETS; EGLKLKAINKNETSN;
GLKLKAINKNETSNE; LKLKAINKNETSNEL; KLKAINKNETSNELK;
LKAINKNETSNELKD; KAINKNETSNELKDE; AINKNETSNELKDEN;
INKNETSNELKDENF; NKNETSNELKDENFK; KNETSNELKDENFKK;
NETSNELKDENFKKI; ETSNELKDENFKKIE; TSNELKDENFKKIEE;
SNELKDENFKKIEEE; NELKDENFKKIEEEF; ELKDENFKKIEEEFK;
LKDENFKKIEEEFKD; KDENFKKIEEEFKDT; DENFKKIEEEFKDTL;
ENFKKIEEEFKDTLT; NFKKIEEEFKDTLTK; FKKIEEEFKDTLTKV;
KKIEEEFKDTLTKVK; KIEEEFKDTLTKVKE; IEEEFKDTLTKVKEI;
EEEFKDTLTKVKEIL; EEFKDTLTKVKEILK; EFKDTLTKVKEILKD;
FKDTLTKVKEILKDH; KDTLTKVKEILKDHI; DTLTKVKEILKDHIK;
TLTKVKEILKDHIKE; LTKVKEILKDHIKEV; TKVKEILKDHIKEVN;
KVKEILKDHIKEVNL; VKEILKDHIKEVNLS; KEILKDHIKEVNLSA;
EILKDHIKEVNLSAT; ILKDHIKEVNLSATL; LKDHIKEVNLSATLI;
KDHIKEVNLSATLIK; DHIKEVNLSATLIKE; HIKEVNLSATLIKEP;
IKEVNLSATLIKEPS; KEVNLSATLIKEPSA; EVNLSATLIKEPSAI;
VNLSATLIKEPSAII; NLSATLIKEPSAIII; LSATLIKEPSAIIID;
SATLIKEPSAIIIDS; ATLIKEPSAIIIDSN; TLIKEPSAIIIDSND;
LIKEPSAIIIDSNDP; IKEPSAIIIDSNDPT; KEPSAIIIDSNDPTY;
EPSAIIIDSNDPTYQ; PSAIIIDSNDPTYQM; SAIIIDSNDPTYQMQ;
AIIIDSNDPTYQMQK; IIIDSNDPTYQMQKI; IIDSNDPTYQMQKIM;
IDSNDPTYQMQKIML; DSNDPTYQMQKIMLS; SNDPTYQMQKIMLSM;
NDPTYQMQKIMLSMG; DPTYQMQKIMLSMGQ; PTYQMQKIMLSMGQE;
TYQMQKIMLSMGQEV; YQMQKIMLSMGQEVK; QMQKIMLSMGQEVKE;
MQKIMLSMGQEVKEI; QKIMLSMGQEVKEIK; KIMLSMGQEVKEIKP;
IMLSMGQEVKEIKPI; MLSMGQEVKEIKPIL; LSMGQEVKEIKPILE;
SMGQEVKEIKPILEL; MGQEVKEIKPILELN; GQEVKEIKPILELNP;
QEVKEIKPILELNPN; EVKEIKPILELNPNN; VKEIKPILELNPNNK;
KEIKPILELNPNNKI; EIKPILELNPNNKIV; IKPILELNPNNKIVQ;
KPILELNPNNKIVQN; PILELNPNNKIVQNL; ILELNPNNKIVQNLK;
LELNPNNKIVQNLKN; ELNPNNKIVQNLKNL; LNPNNKIVQNLKNLE;
NPNNKIVQNLKNLEP; PNNKIVQNLKNLEPE; NNKIVQNLKNLEPEK;
NKIVQNLKNLEPEKL; KIVQNLKNLEPEKLE; IVQNLKNLEPEKLEK;
VQNLKNLEPEKLEKI; QNLKNLEPEKLEKIS; NLKNLEPEKLEKISI;
LKNLEPEKLEKISIL; KNLEPEKLEKISILL; NLEPEKLEKISILLF;
LEPEKLEKISILLFE; EPEKLEKISILLFEE; PEKLEKISILLFEEA;
EKLEKISILLFEEAM; KLEKISILLFEEAML; LEKISILLFEEAMLT;
EKISILLFEEAMLTS; KISILLFEEAMLTSG; ISILLFEEAMLTSGM;
SILLFEEAMLTSGMP; ILLFEEAMLTSGMPS; LLFEEAMLTSGMPSK;
LFEEAMLTSGMPSKN; FEEAMLTSGMPSKNP; EEAMLTSGMPSKNPG;
EAMLTSGMPSKNPGK; AMLTSGMPSKNPGKF; MLTSGMPSKNPGKFI;
LTSGMPSKNPGKFIN; TSGMPSKNPGKFINI; SGMPSKNPGKFINII;
GMPSKNPGKFINIIN; MPSKNPGKFINIINE; PSKNPGKFINIINEF;
SKNPGKFINIINEFI; KNPGKFINIINEFIE; NPGKFINIINEFIEK;
PGKFINIINEFIEKD; GKFINIINEFIEKDF; KFINIINEFIEKDFL;

16 mers:
MILIFYFKQIALFIIF; ILIFYFKQIALFIIFR; LIFYFKQIALFIIFRL;
IFYFKQIALFIIFRLC; FYFKQIALFIIFRLCY; YFKQIALFIIFRLCYI;
```

| | |
|---|---|
| FKQIALFIIFRLCYII; | KQIALFIIFRLCYIIK; | QIALFIIFRLCYIIKK; |
| IALFIIFRLCYIIKKV; | ALFIIFRLCYIIKKVK; | LFIIFRLCYIIKKVKI; |
| FIIFRLCYIIKKVKIK; | IIFRLCYIIKKVKIKL; | IFRLCYIIKKVKIKLK; |
| FRLCYIIKKVKIKLKR; | RLCYIIKKVKIKLKRK; | LCYIIKKVKIKLKRKS; |
| CYIIKKVKIKLKRKSC; | YIIKKVKIKLKRKSCM; | IIKKVKIKLKRKSCMK; |
| IKKVKIKLKRKSCMKK; | KKVKIKLKRKSCMKKQ; | KVKIKLKRKSCMKKQF; |
| VKIKLKRKSCMKKQFD; | KIKLKRKSCMKKQFDT; | IKLKRKSCMKKQFDTE; |
| KLKRKSCMKKQFDTEV; | LKRKSCMKKQFDTEVN; | KRKSCMKKQFDTEVND; |
| RKSCMKKQFDTEVNDL; | KSCMKKQFDTEVNDLL; | SCMKKQFDTEVNDLLY; |
| CMKKQFDTEVNDLLYL; | MKKQFDTEVNDLLYLI; | KKQFDTEVNDLLYLII; |
| KQFDTEVNDLLYLIIH; | QFDTEVNDLLYLIIHS; | FDTEVNDLLYLIIHSL; |
| DTEVNDLLYLIIHSLY; | TEVNDLLYLIIHSLYS; | EVNDLLYLIIHSLYSH; |
| VNDLLYLIIHSLYSHK; | NDLLYLIIHSLYSHKE; | DLLYLIIHSLYSHKEI; |
| LLYLIIHSLYSHKEIF; | LYLIIHSLYSHKEIFL; | YLIIHSLYSHKEIFLR; |
| LIIHSLYSHKEIFLRE; | IIHSLYSHKEIFLREL; | IHSLYSHKEIFLRELI; |
| HSLYSHKEIFLRELIS; | SLYSHKEIFLRELISN; | LYSHKEIFLRELISNA; |
| YSHKEIFLRELISNAS; | SHKEIFLRELISNASD; | HKEIFLRELISNASDA; |
| KEIFLRELISNASDAI; | EIFLRELISNASDAID; | IFLRELISNASDAIDK; |
| FLRELISNASDAIDKL; | LRELISNASDAIDKLK; | RELISNASDAIDKLKF; |
| ELISNASDAIDKLKFL; | LISNASDAIDKLKFLS; | ISNASDAIDKLKFLSL; |
| SNASDAIDKLKFLSLT; | NASDAIDKLKFLSLTN; | ASDAIDKLKFLSLTNE; |
| SDAIDKLKFLSLTNEK; | DAIDKLKFLSLTNEKF; | AIDKLKFLSLTNEKFK; |
| IDKLKFLSLTNEKFKN; | DKLKFLSLTNEKFKNI; | KLKFLSLTNEKFKNIA; |
| LKFLSLTNEKFKNIAL; | KFLSLTNEKFKNIALE; | FLSLTNEKFKNIALEP; |
| LSLTNEKFKNIALEPK; | SLTNEKFKNIALEPKI; | LTNEKFKNIALEPKIE; |
| TNEKFKNIALEPKIEI; | NEKFKNIALEPKIEIS; | EKFKNIALEPKIEISF; |
| KFKNIALEPKIEISFD; | FKNIALEPKIEISFDD; | KNIALEPKIEISFDDK; |
| NIALEPKIEISFDDKS; | IALEPKIEISFDDKSI; | ALEPKIEISFDDKSIL; |
| LEPKIEISFDDKSILI; | EPKIEISFDDKSILIK; | PKIEISFDDKSILIKD; |
| KIEISFDDKSILIKDN; | IEISFDDKSILIKDNG; | EISFDDKSILIKDNGI; |
| ISFDDKSILIKDNGIG; | SFDDKSILIKDNGIGM; | FDDKSILIKDNGIGMD; |
| DDKSILIKDNGIGMDE; | DKSILIKDNGIGMDEQ; | KSILIKDNGIGMDEQD; |
| SILIKDNGIGMDEQDL; | ILIKDNGIGMDEQDLT; | LIKDNGIGMDEQDLTN; |
| IKDNGIGMDEQDLTNH; | KDNGIGMDEQDLTNHL; | DNGIGMDEQDLTNHLG; |
| NGIGMDEQDLTNHLGV; | GIGMDEQDLTNHLGVI; | IGMDEQDLTNHLGVIA; |
| GMDEQDLTNHLGVIAK; | MDEQDLTNHLGVIAKS; | DEQDLTNHLGVIAKSG; |
| EQDLTNHLGVIAKSGT; | QDLTNHLGVIAKSGTK; | DLTNHLGVIAKSGTKE; |
| LTNHLGVIAKSGTKEF; | TNHLGVIAKSGTKEFI; | NHLGVIAKSGTKEFIN; |
| HLGVIAKSGTKEFINN; | LGVIAKSGTKEFINNL; | GVIAKSGTKEFINNLK; |
| VIAKSGTKEFINNLKQ; | IAKSGTKEFINNLKQD; | AKSGTKEFINNLKQDE; |
| KSGTKEFINNLKQDEK; | SGTKEFINNLKQDEKK; | GTKEFINNLKQDEKKS; |
| TKEFINNLKQDEKKSA; | KEFINNLKQDEKKSAS; | EFINNLKQDEKKSASL; |
| FINNLKQDEKKSASLI; | INNLKQDEKKSASLIG; | NNLKQDEKKSASLIGQ; |
| NLKQDEKKSASLIGQF; | LKQDEKKSASLIGQFG; | KQDEKKSASLIGQFGV; |
| QDEKKSASLIGQFGVG; | DEKKSASLIGQFGVGF; | EKKSASLIGQFGVGFY; |
| KKSASLIGQFGVGFYS; | KSASLIGQFGVGFYSA; | SASLIGQFGVGFYSAF; |
| ASLIGQFGVGFYSAFI; | SLIGQFGVGFYSAFIV; | LIGQFGVGFYSAFIVS; |
| IGQFGVGFYSAFIVSE; | GQFGVGFYSAFIVSEK; | QFGVGFYSAFIVSEKV; |
| FGVGFYSAFIVSEKVE; | GVGFYSAFIVSEKVEV; | VGFYSAFIVSEKVEVT; |
| GFYSAFIVSEKVEVTS; | FYSAFIVSEKVEVTSK; | YSAFIVSEKVEVTSKK; |
| SAFIVSEKVEVTSKKA; | AFIVSEKVEVTSKKAL; | FIVSEKVEVTSKKALE; |
| IVSEKVEVTSKKALES; | VSEKVEVTSKKALESD; | SEKVEVTSKKALESDA; |
| EKVEVTSKKALESDAY; | KVEVTSKKALESDAYI; | VEVTSKKALESDAYIW; |
| EVTSKKALESDAYIWS; | VTSKKALESDAYIWSS; | TSKKALESDAYIWSSD; |
| SKKALESDAYIWSSDG; | KKALESDAYIWSSDGK; | KALESDAYIWSSDGKT; |
| ALESDAYIWSSDGKTG; | LESDAYIWSSDGKTGY; | ESDAYIWSSDGKTGYE; |
| SDAYIWSSDGKTGYEI; | DAYIWSSDGKTGYEIE; | AYIWSSDGKTGYEIEK; |

YIWSSDGKTGYEIEKA; IWSSDGKTGYEIEKAK; WSSDGKTGYEIEKAKK;
SSDGKTGYEIEKAKKE; SDGKTGYEIEKAKKEE; DGKTGYEIEKAKKEES;
GKTGYEIEKAKKEESG; KTGYEIEKAKKEESGT; TGYEIEKAKKEESGTE;
GYEIEKAKKEESGTEI; YEIEKAKKEESGTEIK; EIEKAKKEESGTEIKL;
IEKAKKEESGTEIKLY; EKAKKEESGTEIKLYL; KAKKEESGTEIKLYLN;
AKKEESGTEIKLYLNK; KKEESGTEIKLYLNKE; KEESGTEIKLYLNKEG;
EESGTEIKLYLNKEGL; ESGTEIKLYLNKEGLE; SGTEIKLYLNKEGLEY;
GTEIKLYLNKEGLEYA; TEIKLYLNKEGLEYAN; EIKLYLNKEGLEYANK;
IKLYLNKEGLEYANKW; KLYLNKEGLEYANKWK; LYLNKEGLEYANKWKI;
YLNKEGLEYANKWKIQ; LNKEGLEYANKWKIQE; NKEGLEYANKWKIQEI;
KEGLEYANKWKIQEII; EGLEYANKWKIQEIIK; GLEYANKWKIQEIIKK;
LEYANKWKIQEIIKKY; EYANKWKIQEIIKKYS; YANKWKIQEIIKKYSN;
ANKWKIQEIIKKYSNH; NKWKIQEIIKKYSNHI; KWKIQEIIKKYSNHIN;
WKIQEIIKKYSNHINY; KIQEIIKKYSNHINYP; IQEIIKKYSNHINYPI;
QEIIKKYSNHINYPIY; EIIKKYSNHINYPIYI; IIKKYSNHINYPIYIK;
IKKYSNHINYPIYIKY; KKYSNHINYPIYIKYS; KYSNHINYPIYIKYSE;
YSNHINYPIYIKYSEP; SNHINYPIYIKYSEPI; NHINYPIYIKYSEPIM;
HINYPIYIKYSEPIMK; INYPIYIKYSEPIMKD; NYPIYIKYSEPIMKDG;
YPIYIKYSEPIMKDGK; PIYIKYSEPIMKDGKQ; IYIKYSEPIMKDGKQE;
YIKYSEPIMKDGKQEG; IKYSEPIMKDGKQEGI; KYSEPIMKDGKQEGIE;
YSEPIMKDGKQEGIEE; SEPIMKDGKQEGIEEK; EPIMKDGKQEGIEEKE;
PIMKDGKQEGIEEKEE; IMKDGKQEGIEEKEEK; MKDGKQEGIEEKEEKL;
KDGKQEGIEEKEEKLN; DGKQEGIEEKEEKLNE; GKQEGIEEKEEKLNET;
KQEGIEEKEEKLNETT; QEGIEEKEEKLNETTA; EGIEEKEEKLNETTAL;
GIEEKEEKLNETTALW; IEEKEEKLNETTALWT; EEKEEKLNETTALWTK;
EKEEKLNETTALWTKN; KEEKLNETTALWTKNK; EEKLNETTALWTKNKS;
EKLNETTALWTKNKSE; KLNETTALWTKNKSEI; LNETTALWTKNKSEIK;
NETTALWTKNKSEIKA; ETTALWTKNKSEIKAE; TTALWTKNKSEIKAEE;
TALWTKNKSEIKAEEY; ALWTKNKSEIKAEEYN; LWTKNKSEIKAEEYNE;
WTKNKSEIKAEEYNEF; TKNKSEIKAEEYNEFY; KNKSEIKAEEYNEFYK;
NKSEIKAEEYNEFYKN; KSEIKAEEYNEFYKNT; SEIKAEEYNEFYKNTT;
EIKAEEYNEFYKNTTF; IKAEEYNEFYKNTTFD; KAEEYNEFYKNTTFDY;
AEEYNEFYKNTTFDYE; EEYNEFYKNTTFDYEN; EYNEFYKNTTFDYENP;
YNEFYKNTTFDYENPL; NEFYKNTTFDYENPLM; EFYKNTTFDYENPLMH;
FYKNTTFDYENPLMHI; YKNTTFDYENPLMHIH; KNTTFDYENPLMHIHT;
NTTFDYENPLMHIHTK; TTFDYENPLMHIHTKA; TFDYENPLMHIHTKAE;
FDYENPLMHIHTKAEG; DYENPLMHIHTKAEGN; YENPLMHIHTKAEGNL;
ENPLMHIHTKAEGNLE; NPLMHIHTKAEGNLEY; PLMHIHTKAEGNLEYT;
LMHIHTKAEGNLEYTN; MHIHTKAEGNLEYTNL; HIHTKAEGNLEYTNLF;
IHTKAEGNLEYTNLFY; HTKAEGNLEYTNLFYV; TKAEGNLEYTNLFYVP;
KAEGNLEYTNLFYVPS; AEGNLEYTNLFYVPSK; EGNLEYTNLFYVPSKA;
GNLEYTNLFYVPSKAP; NLEYTNLFYVPSKAPY; LEYTNLFYVPSKAPYD;
EYTNLFYVPSKAPYDL; YTNLFYVPSKAPYDLY; TNLFYVPSKAPYDLYY;
NLFYVPSKAPYDLYYP; LFYVPSKAPYDLYYPN; FYVPSKAPYDLYYPNT;
YVPSKAPYDLYYPNTK; VPSKAPYDLYYPNTKP; PSKAPYDLYYPNTKPG;
SKAPYDLYYPNTKPGV; KAPYDLYYPNTKPGVK; APYDLYYPNTKPGVKL;
PYDLYYPNTKPGVKLF; YDLYYPNTKPGVKLFI; DLYYPNTKPGVKLFIN;
LYYPNTKPGVKLFINR; YYPNTKPGVKLFINRI; YPNTKPGVKLFINRIF;
PNTKPGVKLFINRIFI; NTKPGVKLFINRIFIT; TKPGVKLFINRIFITD;
KPGVKLFINRIFITDS; PGVKLFINRIFITDSE; GVKLFINRIFITDSEG;
VKLFINRIFITDSEGS; KLFINRIFITDSEGSL; LFINRIFITDSEGSLL;
FINRIFITDSEGSLLP; INRIFITDSEGSLLPN; NRIFITDSEGSLLPNY;
RIFITDSEGSLLPNYL; IFITDSEGSLLPNYLR; FITDSEGSLLPNYLRF;
ITDSEGSLLPNYLRFI; TDSEGSLLPNYLRFIK; DSEGSLLPNYLRFIKG;
SEGSLLPNYLRFIKGI; EGSLLPNYLRFIKGII; GSLLPNYLRFIKGIID;
SLLPNYLRFIKGIIDC; LLPNYLRFIKGIIDCQ; LPNYLRFIKGIIDCQD;
PNYLRFIKGIIDCQDL; NYLRFIKGIIDCQDLP; YLRFIKGIIDCQDLPL;

| | | |
|---|---|---|
| LRFIKGIIDCQDLPLN; | RFIKGIIDCQDLPLNV; | FIKGIIDCQDLPLNVS; |
| IKGIIDCQDLPLNVSR; | KGIIDCQDLPLNVSRE; | GIIDCQDLPLNVSREI; |
| IIDCQDLPLNVSREIL; | IDCQDLPLNVSREILQ; | DCQDLPLNVSREILQQ; |
| CQDLPLNVSREILQQN; | QDLPLNVSREILQQNK; | DLPLNVSREILQQNKI; |
| LPLNVSREILQQNKIL; | PLNVSREILQQNKILS; | LNVSREILQQNKILSK; |
| NVSREILQQNKILSKI; | VSREILQQNKILSKIK; | SREILQQNKILSKIKS; |
| REILQQNKILSKIKSS; | EILQQNKILSKIKSSS; | ILQQNKILSKIKSSSV; |
| LQQNKILSKIKSSSVK; | QQNKILSKIKSSSVKK; | QNKILSKIKSSSVKKI; |
| NKILSKIKSSSVKKIL; | KILSKIKSSSVKKILS; | ILSKIKSSSVKKILSE; |
| LSKIKSSSVKKILSEL; | SKIKSSSVKKILSELE; | KIKSSSVKKILSELEK; |
| IKSSSVKKILSELEKL; | KSSSVKKILSELEKLS; | SSSVKKILSELEKLSK; |
| SSVKKILSELEKLSKK; | SVKKILSELEKLSKKN; | VKKILSELEKLSKKNP; |
| KKILSELEKLSKKNPE; | KILSELEKLSKKNPEK; | ILSELEKLSKKNPEKF; |
| LSELEKLSKKNPEKFS; | SELEKLSKKNPEKFSE; | ELEKLSKKNPEKFSEF; |
| LEKLSKKNPEKFSEFS; | EKLSKKNPEKFSEFSK; | KLSKKNPEKFSEFSKE; |
| LSKKNPEKFSEFSKEF; | SKKNPEKFSEFSKEFG; | KKNPEKFSEFSKEFGR; |
| KNPEKFSEFSKEFGRC; | NPEKFSEFSKEFGRCI; | PEKFSEFSKEFGRCIK; |
| EKFSEFSKEFGRCIKE; | KFSEFSKEFGRCIKEG; | FSEFSKEFGRCIKEGV; |
| SEFSKEFGRCIKEGVY; | EFSKEFGRCIKEGVYS; | FSKEFGRCIKEGVYSD; |
| SKEFGRCIKEGVYSDF; | KEFGRCIKEGVYSDFE; | EFGRCIKEGVYSDFEN; |
| FGRCIKEGVYSDFENR; | GRCIKEGVYSDFENRE; | RCIKEGVYSDFENREK; |
| CIKEGVYSDFENREKL; | IKEGVYSDFENREKLI; | KEGVYSDFENREKLIS; |
| EGVYSDFENREKLISL; | GVYSDFENREKLISLI; | VYSDFENREKLISLIR; |
| YSDFENREKLISLIRF; | SDFENREKLISLIRFK; | DFENREKLISLIRFKS; |
| FENREKLISLIRFKSS; | ENREKLISLIRFKSSS; | NREKLISLIRFKSSSV; |
| REKLISLIRFKSSSVD; | EKLISLIRFKSSSVDG; | KLISLIRFKSSSVDGF; |
| LISLIRFKSSSVDGFV; | ISLIRFKSSSVDGFVS; | SLIRFKSSSVDGFVSF; |
| LIRFKSSSVDGFVSFK; | IRFKSSSVDGFVSFKE; | RFKSSSVDGFVSFKEY; |
| FKSSSVDGFVSFKEYK; | KSSSVDGFVSFKEYKE; | SSSVDGFVSFKEYKER; |
| SSVDGFVSFKEYKERM; | SVDGFVSFKEYKERMN; | VDGFVSFKEYKERMNE; |
| DGFVSFKEYKERMNES; | GFVSFKEYKERMNESQ; | FVSFKEYKERMNESQK; |
| VSFKEYKERMNESQKS; | SFKEYKERMNESQKSI; | FKEYKERMNESQKSIY; |
| KEYKERMNESQKSIYY; | EYKERMNESQKSIYYI; | YKERMNESQKSIYYIT; |
| KERMNESQKSIYYITG; | ERMNESQKSIYYITGG; | RMNESQKSIYYITGGK; |
| MNESQKSIYYITGGKE; | NESQKSIYYITGGKEN; | ESQKSIYYITGGKENI; |
| SQKSIYYITGGKENIL; | QKSIYYITGGKENILK; | KSIYYITGGKENILKE; |
| SIYYITGGKENILKEN; | IYYITGGKENILKENP; | YYITGGKENILKENPI; |
| YITGGKENILKENPIV; | ITGGKENILKENPIVA; | TGGKENILKENPIVAA; |
| GGKENILKENPIVAAY; | GKENILKENPIVAAYK; | KENILKENPIVAAYKE; |
| ENILKENPIVAAYKEK; | NILKENPIVAAYKEKG; | ILKENPIVAAYKEKGF; |
| LKENPIVAAYKEKGFE; | KENPIVAAYKEKGFEI; | ENPIVAAYKEKGFEIL; |
| NPIVAAYKEKGFEILI; | PIVAAYKEKGFEILIM; | IVAAYKEKGFEILIMD; |
| VAAYKEKGFEILIMDD; | AAYKEKGFEILIMDDE; | AYKEKGFEILIMDDEL; |
| YKEKGFEILIMDDELD; | KEKGFEILIMDDELDE; | EKGFEILIMDDELDEA; |
| KGFEILIMDDELDEAI; | GFEILIMDDELDEAIL; | FEILIMDDELDEAILN; |
| EILIMDDELDEAILNL; | ILIMDDELDEAILNLI; | LIMDDELDEAILNLIP; |
| IMDDELDEAILNLIPE; | MDDELDEAILNLIPEY; | DDELDEAILNLIPEYE; |
| DELDEAILNLIPEYEG; | ELDEAILNLIPEYEGL; | LDEAILNLIPEYEGLK; |
| DEAILNLIPEYEGLKL; | EAILNLIPEYEGLKLK; | AILNLIPEYEGLKLKA; |
| ILNLIPEYEGLKLKAI; | LNLIPEYEGLKLKAIN; | NLIPEYEGLKLKAINK; |
| LIPEYEGLKLKAINKN; | IPEYEGLKLKAINKNE; | PEYEGLKLKAINKNET; |
| EYEGLKLKAINKNETS; | YEGLKLKAINKNETSN; | EGLKLKAINKNETSNE; |
| GLKLKAINKNETSNEL; | LKLKAINKNETSNELK; | KLKAINKNETSNELKD; |
| LKAINKNETSNELKDE; | KAINKNETSNELKDEN; | AINKNETSNELKDENF; |
| INKNETSNELKDENFK; | NKNETSNELKDENFKK; | KNETSNELKDENFKKI; |
| NETSNELKDENFKKIE; | ETSNELKDENFKKIEE; | TSNELKDENFKKIEEE; |
| SNELKDENFKKIEEEF; | NELKDENFKKIEEEFK; | ELKDENFKKIEEEFKD; |

| | |
|---|---|
| | LKDENFKKIEEEFKDT; KDENFKKIEEEFKDTL; DENFKKIEEEFKDTLT;<br>ENFKKIEEEFKDTLTK; NFKKIEEEFKDTLTKV; FKKIEEEFKDTLTKVK;<br>KKIEEEFKDTLTKVKE; KIEEEFKDTLTKVKEI; IEEEFKDTLTKVKEIL;<br>EEEFKDTLTKVKEILK; EEFKDTLTKVKEILKD; EFKDTLTKVKEILKDH;<br>FKDTLTKVKEILKDHI; KDTLTKVKEILKDHIK; DTLTKVKEILKDHIKE;<br>TLTKVKEILKDHIKEV; LTKVKEILKDHIKEVN; TKVKEILKDHIKEVNL;<br>KVKEILKDHIKEVNLS; VKEILKDHIKEVNLSA; KEILKDHIKEVNLSAT;<br>EILKDHIKEVNLSATL; ILKDHIKEVNLSATLI; LKDHIKEVNLSATLIK;<br>KDHIKEVNLSATLIKE; DHIKEVNLSATLIKEP; HIKEVNLSATLIKEPS;<br>IKEVNLSATLIKEPSA; KEVNLSATLIKEPSAI; EVNLSATLIKEPSAII;<br>VNLSATLIKEPSAIII; NLSATLIKEPSAIIID; LSATLIKEPSAIIIDS;<br>SATLIKEPSAIIIDSN; ATLIKEPSAIIIDSND; TLIKEPSAIIIDSNDP;<br>LIKEPSAIIIDSNDPT; IKEPSAIIIDSNDPTY; KEPSAIIIDSNDPTYQ;<br>EPSAIIIDSNDPTYQM; PSAIIIDSNDPTYQMQ; SAIIIDSNDPTYQMQK;<br>AIIIDSNDPTYQMQKI; IIIDSNDPTYQMQKIM; IIDSNDPTYQMQKIML;<br>IDSNDPTYQMQKIMLS; DSNDPTYQMQKIMLSM; SNDPTYQMQKIMLSMG;<br>NDPTYQMQKIMLSMGQ; DPTYQMQKIMLSMGQE; PTYQMQKIMLSMGQEV;<br>TYQMQKIMLSMGQEVK; YQMQKIMLSMGQEVKE; QMQKIMLSMGQEVKEI;<br>MQKIMLSMGQEVKEIK; QKIMLSMGQEVKEIKP; KIMLSMGQEVKEIKPI;<br>IMLSMGQEVKEIKPIL; MLSMGQEVKEIKPILE; LSMGQEVKEIKPILEL;<br>SMGQEVKEIKPILELN; MGQEVKEIKPILELNP; GQEVKEIKPILELNPN;<br>QEVKEIKPILELNPNN; EVKEIKPILELNPNNK; VKEIKPILELNPNNKI;<br>KEIKPILELNPNNKIV; EIKPILELNPNNKIVQ; IKPILELNPNNKIVQN;<br>KPILELNPNNKIVQNL; PILELNPNNKIVQNLK; ILELNPNNKIVQNLKN;<br>LELNPNNKIVQNLKNL; ELNPNNKIVQNLKNLE; LNPNNKIVQNLKNLEP;<br>NPNNKIVQNLKNLEPE; PNNKIVQNLKNLEPEK; NNKIVQNLKNLEPEKL;<br>NKIVQNLKNLEPEKLE; KIVQNLKNLEPEKLEK; IVQNLKNLEPEKLEKI;<br>VQNLKNLEPEKLEKIS; QNLKNLEPEKLEKISI; NLKNLEPEKLEKISIL;<br>LKNLEPEKLEKISILL; KNLEPEKLEKISILLF; NLEPEKLEKISILLFE;<br>LEPEKLEKISILLFEE; EPEKLEKISILLFEEA; PEKLEKISILLFEEAM;<br>EKLEKISILLFEEAML; KLEKISILLFEEAMLT; LEKISILLFEEAMLTS;<br>EKISILLFEEAMLTSG; KISILLFEEAMLTSGM; ISILLFEEAMLTSGMP;<br>SILLFEEAMLTSGMPS; ILLFEEAMLTSGMPSK; LLFEEAMLTSGMPSKN;<br>LFEEAMLTSGMPSKNP; FEEAMLTSGMPSKNPG; EEAMLTSGMPSKNPGK;<br>EAMLTSGMPSKNPGKF; AMLTSGMPSKNPGKFI; MLTSGMPSKNPGKFIN;<br>LTSGMPSKNPGKFINI; TSGMPSKNPGKFINII; SGMPSKNPGKFINIIN;<br>GMPSKNPGKFINIINE; MPSKNPGKFINIINEF; PSKNPGKFINIINEFI;<br>SKNPGKFINIINEFIE; KNPGKFINIINEFIEK; NPGKFINIINEFIEKD;<br>PGKFINIINEFIEKDF; GKFINIINEFIEKDFL; |
| CAA44492.1\| Outer<br>surface protein A<br>[Borrelia<br>burgdorferi] | 13 mers:<br>MKKYLLGIGLILA; KKYLLGIGLILAL; KYLLGIGLILALI; YLLGIGLILALIA;<br>LLGIGLILALIAC; LGIGLILALIACK; GIGLILALIACKQ; IGLILALIACKQN;<br>GLILALIACKQNV; LILALIACKQNVS; ILALIACKQNVST; LALIACKQNVSTL;<br>ALIACKQNVSTLD; LIACKQNVSTLDE; IACKQNVSTLDEK; ACKQNVSTLDEKN;<br>CKQNVSTLDEKNS; KQNVSTLDEKNSV; QNVSTLDEKNSVS; NVSTLDEKNSVSV;<br>VSTLDEKNSVSVD; STLDEKNSVSVDL; TLDEKNSVSVDLP; LDEKNSVSVDLPG;<br>DEKNSVSVDLPGG; EKNSVSVDLPGGM; KNSVSVDLPGGMT; NSVSVDLPGGMTE;<br>SVSVDLPGGMTEL; VSVDLPGGMTELV; SVDLPGGMTELVS; VDLPGGMTELVSK;<br>DLPGGMTELVSKE; LPGGMTELVSKEK; PGGMTELVSKEKD; GGMTELVSKEKDK;<br>GMTELVSKEKDKD; MTELVSKEKDKDG; TELVSKEKDKDGK; ELVSKEKDKDGKY;<br>LVSKEKDKDGKYS; VSKEKDKDGKYSL; SKEKDKDGKYSLE; KEKDKDGKYSLEA;<br>EKDKDGKYSLEAT; KDKDGKYSLEATV; DKDGKYSLEATVD; KDGKYSLEATVDK;<br>DGKYSLEATVDKL; GKYSLEATVDKLE; KYSLEATVDKLEL; YSLEATVDKLELK;<br>SLEATVDKLELKG; LEATVDKLELKGT; EATVDKLELKGTS; ATVDKLELKGTSD;<br>TVDKLELKGTSDK; VDKLELKGTSDKN; DKLELKGTSDKNN; KLELKGTSDKNNG; |

LELKGTSDKNNGS; ELKGTSDKNNGSG; LKGTSDKNNGSGT; KGTSDKNNGSGTL;
GTSDKNNGSGTLE; TSDKNNGSGTLEG; SDKNNGSGTLEGE; DKNNGSGTLEGEK;
KNNGSGTLEGEKT; NNGSGTLEGEKTD; NGSGTLEGEKTDK; GSGTLEGEKTDKS;
SGTLEGEKTDKSK; GTLEGEKTDKSKV; TLEGEKTDKSKVK; LEGEKTDKSKVKL;
EGEKTDKSKVKLT; GEKTDKSKVKLTI; EKTDKSKVKLTIA; KTDKSKVKLTIAD;
TDKSKVKLTIADD; DKSKVKLTIADDL; KSKVKLTIADDLS; SKVKLTIADDLSQ;
KVKLTIADDLSQT; VKLTIADDLSQTK; KLTIADDLSQTKF; LTIADDLSQTKFE;
TIADDLSQTKFEI; IADDLSQTKFEIF; ADDLSQTKFEIFK; DDLSQTKFEIFKE;
DLSQTKFEIFKED; LSQTKFEIFKEDA; SQTKFEIFKEDAK; QTKFEIFKEDAKT;
TKFEIFKEDAKTL; KFEIFKEDAKTLV; FEIFKEDAKTLVS; EIFKEDAKTLVSK;
IFKEDAKTLVSKK; FKEDAKTLVSKKV; KEDAKTLVSKKVT; EDAKTLVSKKVTL;
DAKTLVSKKVTLK; AKTLVSKKVTLKD; KTLVSKKVTLKDK; TLVSKKVTLKDKS;
LVSKKVTLKDKSS; VSKKVTLKDKSST; SKKVTLKDKSSTE; KKVTLKDKSSTEE;
KVTLKDKSSTEEK; VTLKDKSSTEEKF; TLKDKSSTEEKFN; LKDKSSTEEKFNE;
KDKSSTEEKFNEK; DKSSTEEKFNEKG; KSSTEEKFNEKGE; SSTEEKFNEKGET;
STEEKFNEKGETS; TEEKFNEKGETSE; EEKFNEKGETSEK; EKFNEKGETSEKT;
KFNEKGETSEKTI; FNEKGETSEKTIV; NEKGETSEKTIVR; EKGETSEKTIVRA;
KGETSEKTIVRAN; GETSEKTIVRANG; ETSEKTIVRANGT; TSEKTIVRANGTR;
SEKTIVRANGTRL; EKTIVRANGTRLE; KTIVRANGTRLEY; TIVRANGTRLEYT;
IVRANGTRLEYTD; VRANGTRLEYTDI; RANGTRLEYTDIK; ANGTRLEYTDIKS;
NGTRLEYTDIKSD; GTRLEYTDIKSDG; TRLEYTDIKSDGS; RLEYTDIKSDGSG;
LEYTDIKSDGSGK; EYTDIKSDGSGKA; YTDIKSDGSGKAK; TDIKSDGSGKAKE;
DIKSDGSGKAKEV; IKSDGSGKAKEVL; KSDGSGKAKEVLK; SDGSGKAKEVLKD;
DGSGKAKEVLKDF; GSGKAKEVLKDFT; SGKAKEVLKDFTL; GKAKEVLKDFTLE;
KAKEVLKDFTLEG; AKEVLKDFTLEGT; KEVLKDFTLEGTL; EVLKDFTLEGTLA;
VLKDFTLEGTLAA; LKDFTLEGTLAAD; KDFTLEGTLAADG; DFTLEGTLAADGK;
FTLEGTLAADGKT; TLEGTLAADGKTT; LEGTLAADGKTTL; EGTLAADGKTTLK;
GTLAADGKTTLKV; TLAADGKTTLKVT; LAADGKTTLKVTE; AADGKTTLKVTEG;
ADGKTTLKVTEGT; DGKTTLKVTEGTV; GKTTLKVTEGTVV; KTTLKVTEGTVVL;
TTLKVTEGTVVLS; TLKVTEGTVVLSK; LKVTEGTVVLSKN; KVTEGTVVLSKNI;
VTEGTVVLSKNIL; TEGTVVLSKNILK; EGTVVLSKNILKS; GTVVLSKNILKSG;
TVVLSKNILKSGE; VVLSKNILKSGEI; VLSKNILKSGEIT; LSKNILKSGEITV;
SKNILKSGEITVA; KNILKSGEITVAL; NILKSGEITVALD; ILKSGEITVALDD;
LKSGEITVALDDS; KSGEITVALDDSD; SGEITVALDDSDT; GEITVALDDSDTT;
EITVALDDSDTTQ; ITVALDDSDTTQA; TVALDDSDTTQAT; VALDDSDTTQATK;
ALDDSDTTQATKK; LDDSDTTQATKKT; DDSDTTQATKKTG; DSDTTQATKKTGK;
SDTTQATKKTGKW; DTTQATKKTGKWD; TTQATKKTGKWDS; TQATKKTGKWDSK;
QATKKTGKWDSKT; ATKKTGKWDSKTS; TKKTGKWDSKTST; KKTGKWDSKTSTL;
KTGKWDSKTSTLT; TGKWDSKTSTLTI; GKWDSKTSTLTIS; KWDSKTSTLTISV;
WDSKTSTLTISVN; DSKTSTLTISVNS; SKTSTLTISVNSQ; KTSTLTISVNSQK;
TSTLTISVNSQKT; STLTISVNSQKTK; TLTISVNSQKTKN; LTISVNSQKTKNL;
TISVNSQKTKNLV; ISVNSQKTKNLVF; SVNSQKTKNLVFT; VNSQKTKNLVFTK;
NSQKTKNLVFTKE; SQKTKNLVFTKED; QKTKNLVFTKEDT; KTKNLVFTKEDTI;
TKNLVFTKEDTIT; KNLVFTKEDTITV; NLVFTKEDTITVQ; LVFTKEDTITVQK;
VFTKEDTITVQKY; FTKEDTITVQKYD; TKEDTITVQKYDS; KEDTITVQKYDSA;
EDTITVQKYDSAG; DTITVQKYDSAGT; TITVQKYDSAGTN; ITVQKYDSAGTNL;
TVQKYDSAGTNLE; VQKYDSAGTNLEG; QKYDSAGTNLEGK; KYDSAGTNLEGKA;
YDSAGTNLEGKAV; DSAGTNLEGKAVE; SAGTNLEGKAVEI; AGTNLEGKAVEIT;
GTNLEGKAVEITT; TNLEGKAVEITTL; NLEGKAVEITTLK; LEGKAVEITTLKE;
EGKAVEITTLKEL; GKAVEITTLKELK; KAVEITTLKELKN; AVEITTLKELKNA;
VEITTLKELKNAL; EITTLKELKNALK;

14 mers:
MKKYLLGIGLILAL; KKYLLGIGLILALI; KYLLGIGLILALIA;
YLLGIGLILALIAC; LLGIGLILALIACK; LGIGLILALIACKQ;
GIGLILALIACKQN; IGLILALIACKQNV; GLILALIACKQNVS;
LILALIACKQNVST; ILALIACKQNVSTL; LALIACKQNVSTLD;

| | |
|---|---|
| ALIACKQNVSTLDE; | LIACKQNVSTLDEK; | IACKQNVSTLDEKN; |
| ACKQNVSTLDEKNS; | CKQNVSTLDEKNSV; | KQNVSTLDEKNSVS; |
| QNVSTLDEKNSVSV; | NVSTLDEKNSVSVD; | VSTLDEKNSVSVDL; |
| STLDEKNSVSVDLP; | TLDEKNSVSVDLPG; | LDEKNSVSVDLPGG; |
| DEKNSVSVDLPGGM; | EKNSVSVDLPGGMT; | KNSVSVDLPGGMTE; |
| NSVSVDLPGGMTEL; | SVSVDLPGGMTELV; | VSVDLPGGMTELVS; |
| SVDLPGGMTELVSK; | VDLPGGMTELVSKE; | DLPGGMTELVSKEK; |
| LPGGMTELVSKEKD; | PGGMTELVSKEKDK; | GGMTELVSKEKDKD; |
| GMTELVSKEKDKDG; | MTELVSKEKDKDGK; | TELVSKEKDKDGKY; |
| ELVSKEKDKDGKYS; | LVSKEKDKDGKYSL; | VSKEKDKDGKYSLE; |
| SKEKDKDGKYSLEA; | KEKDKDGKYSLEAT; | EKDKDGKYSLEATV; |
| KDKDGKYSLEATVD; | DKDGKYSLEATVDK; | KDGKYSLEATVDKL; |
| DGKYSLEATVDKLE; | GKYSLEATVDKLEL; | KYSLEATVDKLELK; |
| YSLEATVDKLELKG; | SLEATVDKLELKGT; | LEATVDKLELKGTS; |
| EATVDKLELKGTSD; | ATVDKLELKGTSDK; | TVDKLELKGTSDKN; |
| VDKLELKGTSDKNN; | DKLELKGTSDKNNG; | KLELKGTSDKNNGS; |
| LELKGTSDKNNGSG; | ELKGTSDKNNGSGT; | LKGTSDKNNGSGTL; |
| KGTSDKNNGSGTLE; | GTSDKNNGSGTLEG; | TSDKNNGSGTLEGE; |
| SDKNNGSGTLEGEK; | DKNNGSGTLEGEKT; | KNNGSGTLEGEKTD; |
| NNGSGTLEGEKTDK; | NGSGTLEGEKTDKS; | GSGTLEGEKTDKSK; |
| SGTLEGEKTDKSKV; | GTLEGEKTDKSKVK; | TLEGEKTDKSKVKL; |
| LEGEKTDKSKVKLT; | EGEKTDKSKVKLTI; | GEKTDKSKVKLTIA; |
| EKTDKSKVKLTIAD; | KTDKSKVKLTIADD; | TDKSKVKLTIADDL; |
| DKSKVKLTIADDLS; | KSKVKLTIADDLSQ; | SKVKLTIADDLSQT; |
| KVKLTIADDLSQTK; | VKLTIADDLSQTKF; | KLTIADDLSQTKFE; |
| LTIADDLSQTKFEI; | TIADDLSQTKFEIF; | IADDLSQTKFEIFK; |
| ADDLSQTKFEIFKE; | DDLSQTKFEIFKED; | DLSQTKFEIFKEDA; |
| LSQTKFEIFKEDAK; | SQTKFEIFKEDAKT; | QTKFEIFKEDAKTL; |
| TKFEIFKEDAKTLV; | KFEIFKEDAKTLVS; | FEIFKEDAKTLVSK; |
| EIFKEDAKTLVSKK; | IFKEDAKTLVSKKV; | FKEDAKTLVSKKVT; |
| KEDAKTLVSKKVTL; | EDAKTLVSKKVTLK; | DAKTLVSKKVTLKD; |
| AKTLVSKKVTLKDK; | KTLVSKKVTLKDKS; | TLVSKKVTLKDKSS; |
| LVSKKVTLKDKSST; | VSKKVTLKDKSSTE; | SKKVTLKDKSSTEE; |
| KKVTLKDKSSTEEK; | KVTLKDKSSTEEKF; | VTLKDKSSTEEKFN; |
| TLKDKSSTEEKFNE; | LKDKSSTEEKFNEK; | KDKSSTEEKFNEKG; |
| DKSSTEEKFNEKGE; | KSSTEEKFNEKGET; | SSTEEKFNEKGETS; |
| STEEKFNEKGETSE; | TEEKFNEKGETSEK; | EEKFNEKGETSEKT; |
| EKFNEKGETSEKTI; | KFNEKGETSEKTIV; | FNEKGETSEKTIVR; |
| NEKGETSEKTIVRA; | EKGETSEKTIVRAN; | KGETSEKTIVRANG; |
| GETSEKTIVRANGT; | ETSEKTIVRANGTR; | TSEKTIVRANGTRL; |
| SEKTIVRANGTRLE; | EKTIVRANGTRLEY; | KTIVRANGTRLEYT; |
| TIVRANGTRLEYTD; | IVRANGTRLEYTDI; | VRANGTRLEYTDIK; |
| RANGTRLEYTDIKS; | ANGTRLEYTDIKSD; | NGTRLEYTDIKSDG; |
| GTRLEYTDIKSDGS; | TRLEYTDIKSDGSG; | RLEYTDIKSDGSGK; |
| LEYTDIKSDGSGKA; | EYTDIKSDGSGKAK; | YTDIKSDGSGKAKE; |
| TDIKSDGSGKAKEV; | DIKSDGSGKAKEVL; | IKSDGSGKAKEVLK; |
| KSDGSGKAKEVLKD; | SDGSGKAKEVLKDF; | DGSGKAKEVLKDFT; |
| GSGKAKEVLKDFTL; | SGKAKEVLKDFTLE; | GKAKEVLKDFTLEG; |
| KAKEVLKDFTLEGT; | AKEVLKDFTLEGTL; | KEVLKDFTLEGTLA; |
| EVLKDFTLEGTLAA; | VLKDFTLEGTLAAD; | LKDFTLEGTLAADG; |
| KDFTLEGTLAADGK; | DFTLEGTLAADGKT; | FTLEGTLAADGKTT; |
| TLEGTLAADGKTTL; | LEGTLAADGKTTLK; | EGTLAADGKTTLKV; |
| GTLAADGKTTLKVT; | TLAADGKTTLKVTE; | LAADGKTTLKVTEG; |
| AADGKTTLKVTEGT; | ADGKTTLKVTEGTV; | DGKTTLKVTEGTVV; |
| GKTTLKVTEGTVVL; | KTTLKVTEGTVVLS; | TTLKVTEGTVVLSK; |
| TLKVTEGTVVLSKN; | LKVTEGTVVLSKNI; | KVTEGTVVLSKNIL; |
| VTEGTVVLSKNILK; | TEGTVVLSKNILKS; | EGTVVLSKNILKSG; |

GTVVLSKNILKSGE; TVVLSKNILKSGEI; VVLSKNILKSGEIT;
VLSKNILKSGEITV; LSKNILKSGEITVA; SKNILKSGEITVAL;
KNILKSGEITVALD; NILKSGEITVALDD; ILKSGEITVALDDS;
LKSGEITVALDDSD; KSGEITVALDDSDT; SGEITVALDDSDTT;
GEITVALDDSDTTQ; EITVALDDSDTTQA; ITVALDDSDTTQAT;
TVALDDSDTTQATK; VALDDSDTTQATKK; ALDDSDTTQATKKT;
LDDSDTTQATKKTG; DDSDTTQATKKTGK; DSDTTQATKKTGKW;
SDTTQATKKTGKWD; DTTQATKKTGKWDS; TTQATKKTGKWDSK;
TQATKKTGKWDSKT; QATKKTGKWDSKTS; ATKKTGKWDSKTST;
TKKTGKWDSKTSTL; KKTGKWDSKTSTLT; KTGKWDSKTSTLTI;
TGKWDSKTSTLTIS; GKWDSKTSTLTISV; KWDSKTSTLTISVN;
WDSKTSTLTISVNS; DSKTSTLTISVNSQ; SKTSTLTISVNSQK;
KTSTLTISVNSQKT; TSTLTISVNSQKTK; STLTISVNSQKTKN;
TLTISVNSQKTKNL; LTISVNSQKTKNLV; TISVNSQKTKNLVF;
ISVNSQKTKNLVFT; SVNSQKTKNLVFTK; VNSQKTKNLVFTKE;
NSQKTKNLVFTKED; SQKTKNLVFTKEDT; QKTKNLVFTKEDTI;
KTKNLVFTKEDTIT; TKNLVFTKEDTITV; KNLVFTKEDTITVQ;
NLVFTKEDTITVQK; LVFTKEDTITVQKY; VFTKEDTITVQKYD;
FTKEDTITVQKYDS; TKEDTITVQKYDSA; KEDTITVQKYDSAG;
EDTITVQKYDSAGT; DTITVQKYDSAGTN; TITVQKYDSAGTNL;
ITVQKYDSAGTNLE; TVQKYDSAGTNLEG; VQKYDSAGTNLEGK;
QKYDSAGTNLEGKA; KYDSAGTNLEGKAV; YDSAGTNLEGKAVE;
DSAGTNLEGKAVEI; SAGTNLEGKAVEIT; AGTNLEGKAVEITT;
GTNLEGKAVEITTL; TNLEGKAVEITTLK; NLEGKAVEITTLKE;
LEGKAVEITTLKEL; EGKAVEITTLKELK; GKAVEITTLKELKN;
KAVEITTLKELKNA; AVEITTLKELKNAL; VEITTLKELKNALK;

15 mers:
MKKYLLGIGLILALI; KKYLLGIGLILALIA; KYLLGIGLILALIAC;
YLLGIGLILALIACK; LLGIGLILALIACKQ; LGIGLILALIACKQN;
GIGLILALIACKQNV; IGLILALIACKQNVS; GLILALIACKQNVST;
LILALIACKQNVSTL; ILALIACKQNVSTLD; LALIACKQNVSTLDE;
ALIACKQNVSTLDEK; LIACKQNVSTLDEKN; IACKQNVSTLDEKNS;
ACKQNVSTLDEKNSV; CKQNVSTLDEKNSVS; KQNVSTLDEKNSVSV;
QNVSTLDEKNSVSVD; NVSTLDEKNSVSVDL; VSTLDEKNSVSVDLP;
STLDEKNSVSVDLPG; TLDEKNSVSVDLPGG; LDEKNSVSVDLPGGM;
DEKNSVSVDLPGGMT; EKNSVSVDLPGGMTE; KNSVSVDLPGGMTEL;
NSVSVDLPGGMTELV; SVSVDLPGGMTELVS; VSVDLPGGMTELVSK;
SVDLPGGMTELVSKE; VDLPGGMTELVSKEK; DLPGGMTELVSKEKD;
LPGGMTELVSKEKDK; PGGMTELVSKEKDKD; GGMTELVSKEKDKDG;
GMTELVSKEKDKDGK; MTELVSKEKDKDGKY; TELVSKEKDKDGKYS;
ELVSKEKDKDGKYSL; LVSKEKDKDGKYSLE; VSKEKDKDGKYSLEA;
SKEKDKDGKYSLEAT; KEKDKDGKYSLEATV; EKDKDGKYSLEATVD;
KDKDGKYSLEATVDK; DKDGKYSLEATVDKL; KDGKYSLEATVDKLE;
DGKYSLEATVDKLEL; GKYSLEATVDKLELK; KYSLEATVDKLELKG;
YSLEATVDKLELKGT; SLEATVDKLELKGTS; LEATVDKLELKGTSD;
EATVDKLELKGTSDK; ATVDKLELKGTSDKN; TVDKLELKGTSDKNN;
VDKLELKGTSDKNNG; DKLELKGTSDKNNGS; KLELKGTSDKNNGSG;
LELKGTSDKNNGSGT; ELKGTSDKNNGSGTL; LKGTSDKNNGSGTLE;
KGTSDKNNGSGTLEG; GTSDKNNGSGTLEGE; TSDKNNGSGTLEGEK;
SDKNNGSGTLEGEKT; DKNNGSGTLEGEKTD; KNNGSGTLEGEKTDK;
NNGSGTLEGEKTDKS; NGSGTLEGEKTDKSK; GSGTLEGEKTDKSKV;
SGTLEGEKTDKSKVK; GTLEGEKTDKSKVKL; TLEGEKTDKSKVKLT;
LEGEKTDKSKVKLTI; EGEKTDKSKVKLTIA; GEKTDKSKVKLTIAD;
EKTDKSKVKLTIADD; KTDKSKVKLTIADDL; TDKSKVKLTIADDLS;
DKSKVKLTIADDLSQ; KSKVKLTIADDLSQT; SKVKLTIADDLSQTK;
KVKLTIADDLSQTKF; VKLTIADDLSQTKFE; KLTIADDLSQTKFEI;

LTIADDLSQTKFEIF; TIADDLSQTKFEIFK; IADDLSQTKFEIFKE;
ADDLSQTKFEIFKED; DDLSQTKFEIFKEDA; DLSQTKFEIFKEDAK;
LSQTKFEIFKEDAKT; SQTKFEIFKEDAKTL; QTKFEIFKEDAKTLV;
TKFEIFKEDAKTLVS; KFEIFKEDAKTLVSK; FEIFKEDAKTLVSKK;
EIFKEDAKTLVSKKV; IFKEDAKTLVSKKVT; FKEDAKTLVSKKVTL;
KEDAKTLVSKKVTLK; EDAKTLVSKKVTLKD; DAKTLVSKKVTLKDK;
AKTLVSKKVTLKDKS; KTLVSKKVTLKDKSS; TLVSKKVTLKDKSST;
LVSKKVTLKDKSSTE; VSKKVTLKDKSSTEE; SKKVTLKDKSSTEEK;
KKVTLKDKSSTEEKF; KVTLKDKSSTEEKFN; VTLKDKSSTEEKFNE;
TLKDKSSTEEKFNEK; LKDKSSTEEKFNEKG; KDKSSTEEKFNEKGE;
DKSSTEEKFNEKGET; KSSTEEKFNEKGETS; SSTEEKFNEKGETSE;
STEEKFNEKGETSEK; TEEKFNEKGETSEKT; EEKFNEKGETSEKTI;
EKFNEKGETSEKTIV; KFNEKGETSEKTIVR; FNEKGETSEKTIVRA;
NEKGETSEKTIVRAN; EKGETSEKTIVRANG; KGETSEKTIVRANGT;
GETSEKTIVRANGTR; ETSEKTIVRANGTRL; TSEKTIVRANGTRLE;
SEKTIVRANGTRLEY; EKTIVRANGTRLEYT; KTIVRANGTRLEYTD;
TIVRANGTRLEYTDI; IVRANGTRLEYTDIK; VRANGTRLEYTDIKS;
RANGTRLEYTDIKSD; ANGTRLEYTDIKSDG; NGTRLEYTDIKSDGS;
GTRLEYTDIKSDGSG; TRLEYTDIKSDGSGK; RLEYTDIKSDGSGKA;
LEYTDIKSDGSGKAK; EYTDIKSDGSGKAKE; YTDIKSDGSGKAKEV;
TDIKSDGSGKAKEVL; DIKSDGSGKAKEVLK; IKSDGSGKAKEVLKD;
KSDGSGKAKEVLKDF; SDGSGKAKEVLKDFT; DGSGKAKEVLKDFTL;
GSGKAKEVLKDFTLE; SGKAKEVLKDFTLEG; GKAKEVLKDFTLEGT;
KAKEVLKDFTLEGTL; AKEVLKDFTLEGTLA; KEVLKDFTLEGTLAA;
EVLKDFTLEGTLAAD; VLKDFTLEGTLAADG; LKDFTLEGTLAADGK;
KDFTLEGTLAADGKT; DFTLEGTLAADGKTT; FTLEGTLAADGKTTL;
TLEGTLAADGKTTLK; LEGTLAADGKTTLKV; EGTLAADGKTTLKVT;
GTLAADGKTTLKVTE; TLAADGKTTLKVTEG; LAADGKTTLKVTEGT;
AADGKTTLKVTEGTV; ADGKTTLKVTEGTVV; DGKTTLKVTEGTVVL;
GKTTLKVTEGTVVLS; KTTLKVTEGTVVLSK; TTLKVTEGTVVLSKN;
TLKVTEGTVVLSKNI; LKVTEGTVVLSKNIL; KVTEGTVVLSKNILK;
VTEGTVVLSKNILKS; TEGTVVLSKNILKSG; EGTVVLSKNILKSGE;
GTVVLSKNILKSGEI; TVVLSKNILKSGEIT; VVLSKNILKSGEITV;
VLSKNILKSGEITVA; LSKNILKSGEITVAL; SKNILKSGEITVALD;
KNILKSGEITVALDD; NILKSGEITVALDDS; ILKSGEITVALDDSD;
LKSGEITVALDDSDT; KSGEITVALDDSDTT; SGEITVALDDSDTTQ;
GEITVALDDSDTTQA; EITVALDDSDTTQAT; ITVALDDSDTTQATK;
TVALDDSDTTQATKK; VALDDSDTTQATKKT; ALDDSDTTQATKKTG;
LDDSDTTQATKKTGK; DDSDTTQATKKTGKW; DSDTTQATKKTGKWD;
SDTTQATKKTGKWDS; DTTQATKKTGKWDSK; TTQATKKTGKWDSKT;
TQATKKTGKWDSKTS; QATKKTGKWDSKTST; ATKKTGKWDSKTSTL;
TKKTGKWDSKTSTLT; KKTGKWDSKTSTLTI; KTGKWDSKTSTLTIS;
TGKWDSKTSTLTISV; GKWDSKTSTLTISVN; KWDSKTSTLTISVNS;
WDSKTSTLTISVNSQ; DSKTSTLTISVNSQK; SKTSTLTISVNSQKT;
KTSTLTISVNSQKTK; TSTLTISVNSQKTKN; STLTISVNSQKTKNL;
TLTISVNSQKTKNLV; LTISVNSQKTKNLVF; TISVNSQKTKNLVFT;
ISVNSQKTKNLVFTK; SVNSQKTKNLVFTKE; VNSQKTKNLVFTKED;
NSQKTKNLVFTKEDT; SQKTKNLVFTKEDTI; QKTKNLVFTKEDTIT;
KTKNLVFTKEDTITV; TKNLVFTKEDTITVQ; KNLVFTKEDTITVQK;
NLVFTKEDTITVQKY; LVFTKEDTITVQKYD; VFTKEDTITVQKYDS;
FTKEDTITVQKYDSA; TKEDTITVQKYDSAG; KEDTITVQKYDSAGT;
EDTITVQKYDSAGTN; DTITVQKYDSAGTNL; TITVQKYDSAGTNLE;
ITVQKYDSAGTNLEG; TVQKYDSAGTNLEGK; VQKYDSAGTNLEGKA;
QKYDSAGTNLEGKAV; KYDSAGTNLEGKAVE; YDSAGTNLEGKAVEI;
DSAGTNLEGKAVEIT; SAGTNLEGKAVEITT; AGTNLEGKAVEITTL;
GTNLEGKAVEITTLK; TNLEGKAVEITTLKE; NLEGKAVEITTLKEL;
LEGKAVEITTLKELK; EGKAVEITTLKELKN; GKAVEITTLKELKNA;

KAVEITTLKELKNAL; AVEITTLKELKNALK;

16 mers:
MKKYLLGIGLILALIA; KKYLLGIGLILALIAC; KYLLGIGLILALIACK;
YLLGIGLILALIACKQ; LLGIGLILALIACKQN; LGIGLILALIACKQNV;
GIGLILALIACKQNVS; IGLILALIACKQNVST; GLILALIACKQNVSTL;
LILALIACKQNVSTLD; ILALIACKQNVSTLDE; LALIACKQNVSTLDEK;
ALIACKQNVSTLDEKN; LIACKQNVSTLDEKNS; IACKQNVSTLDEKNSV;
ACKQNVSTLDEKNSVS; CKQNVSTLDEKNSVSV; KQNVSTLDEKNSVSVD;
QNVSTLDEKNSVSVDL; NVSTLDEKNSVSVDLP; VSTLDEKNSVSVDLPG;
STLDEKNSVSVDLPGG; TLDEKNSVSVDLPGGM; LDEKNSVSVDLPGGMT;
DEKNSVSVDLPGGMTE; EKNSVSVDLPGGMTEL; KNSVSVDLPGGMTELV;
NSVSVDLPGGMTELVS; SVSVDLPGGMTELVSK; VSVDLPGGMTELVSKE;
SVDLPGGMTELVSKEK; VDLPGGMTELVSKEKD; DLPGGMTELVSKEKDK;
LPGGMTELVSKEKDKD; PGGMTELVSKEKDKDG; GGMTELVSKEKDKDGK;
GMTELVSKEKDKDGKY; MTELVSKEKDKDGKYS; TELVSKEKDKDGKYSL;
ELVSKEKDKDGKYSLE; LVSKEKDKDGKYSLEA; VSKEKDKDGKYSLEAT;
SKEKDKDGKYSLEATV; KEKDKDGKYSLEATVD; EKDKDGKYSLEATVDK;
KDKDGKYSLEATVDKL; DKDGKYSLEATVDKLE; KDGKYSLEATVDKLEL;
DGKYSLEATVDKLELK; GKYSLEATVDKLELKG; KYSLEATVDKLELKGT;
YSLEATVDKLELKGTS; SLEATVDKLELKGTSD; LEATVDKLELKGTSDK;
EATVDKLELKGTSDKN; ATVDKLELKGTSDKNN; TVDKLELKGTSDKNNG;
VDKLELKGTSDKNNGS; DKLELKGTSDKNNGSG; KLELKGTSDKNNGSGT;
LELKGTSDKNNGSGTL; ELKGTSDKNNGSGTLE; LKGTSDKNNGSGTLEG;
KGTSDKNNGSGTLEGE; GTSDKNNGSGTLEGEK; TSDKNNGSGTLEGEKT;
SDKNNGSGTLEGEKTD; DKNNGSGTLEGEKTDK; KNNGSGTLEGEKTDKS;
NNGSGTLEGEKTDKSK; NGSGTLEGEKTDKSKV; GSGTLEGEKTDKSKVK;
SGTLEGEKTDKSKVKL; GTLEGEKTDKSKVKLT; TLEGEKTDKSKVKLTI;
LEGEKTDKSKVKLTIA; EGEKTDKSKVKLTIAD; GEKTDKSKVKLTIADD;
EKTDKSKVKLTIADDL; KTDKSKVKLTIADDLS; TDKSKVKLTIADDLSQ;
DKSKVKLTIADDLSQT; KSKVKLTIADDLSQTK; SKVKLTIADDLSQTKF;
KVKLTIADDLSQTKFE; VKLTIADDLSQTKFEI; KLTIADDLSQTKFEIF;
LTIADDLSQTKFEIFK; TIADDLSQTKFEIFKE; IADDLSQTKFEIFKED;
ADDLSQTKFEIFKEDA; DDLSQTKFEIFKEDAK; DLSQTKFEIFKEDAKT;
LSQTKFEIFKEDAKTL; SQTKFEIFKEDAKTLV; QTKFEIFKEDAKTLVS;
TKFEIFKEDAKTLVSK; KFEIFKEDAKTLVSKK; FEIFKEDAKTLVSKKV;
EIFKEDAKTLVSKKVT; IFKEDAKTLVSKKVTL; FKEDAKTLVSKKVTLK;
KEDAKTLVSKKVTLKD; EDAKTLVSKKVTLKDK; DAKTLVSKKVTLKDKS;
AKTLVSKKVTLKDKSS; KTLVSKKVTLKDKSST; TLVSKKVTLKDKSSTE;
LVSKKVTLKDKSSTEE; VSKKVTLKDKSSTEEK; SKKVTLKDKSSTEEKF;
KKVTLKDKSSTEEKFN; KVTLKDKSSTEEKFNE; VTLKDKSSTEEKFNEK;
TLKDKSSTEEKFNEKG; LKDKSSTEEKFNEKGE; KDKSSTEEKFNEKGET;
DKSSTEEKFNEKGETS; KSSTEEKFNEKGETSE; SSTEEKFNEKGETSEK;
STEEKFNEKGETSEKT; TEEKFNEKGETSEKTI; EEKFNEKGETSEKTIV;
EKFNEKGETSEKTIVR; KFNEKGETSEKTIVRA; FNEKGETSEKTIVRAN;
NEKGETSEKTIVRANG; EKGETSEKTIVRANGT; KGETSEKTIVRANGTR;
GETSEKTIVRANGTRL; ETSEKTIVRANGTRLE; TSEKTIVRANGTRLEY;
SEKTIVRANGTRLEYT; EKTIVRANGTRLEYTD; KTIVRANGTRLEYTDI;
TIVRANGTRLEYTDIK; IVRANGTRLEYTDIKS; VRANGTRLEYTDIKSD;
RANGTRLEYTDIKSDG; ANGTRLEYTDIKSDGS; NGTRLEYTDIKSDGSG;
GTRLEYTDIKSDGSGK; TRLEYTDIKSDGSGKA; RLEYTDIKSDGSGKAK;
LEYTDIKSDGSGKAKE; EYTDIKSDGSGKAKEV; YTDIKSDGSGKAKEVL;
TDIKSDGSGKAKEVLK; DIKSDGSGKAKEVLKD; IKSDGSGKAKEVLKDF;
KSDGSGKAKEVLKDFT; SDGSGKAKEVLKDFTL; DGSGKAKEVLKDFTLE;
GSGKAKEVLKDFTLEG; SGKAKEVLKDFTLEGT; GKAKEVLKDFTLEGTL;
KAKEVLKDFTLEGTLA; AKEVLKDFTLEGTLAA; KEVLKDFTLEGTLAAD;
EVLKDFTLEGTLAADG; VLKDFTLEGTLAADGK; LKDFTLEGTLAADGKT;

| | |
|---|---|
| | KDFTLEGTLAADGKTT; DFTLEGTLAADGKTTL; FTLEGTLAADGKTTLK; TLEGTLAADGKTTLKV; LEGTLAADGKTTLKVT; EGTLAADGKTTLKVTE; GTLAADGKTTLKVTEG; TLAADGKTTLKVTEGT; LAADGKTTLKVTEGTV; AADGKTTLKVTEGTVV; ADGKTTLKVTEGTVVL; DGKTTLKVTEGTVVLS; GKTTLKVTEGTVVLSK; KTTLKVTEGTVVLSKN; TTLKVTEGTVVLSKNI; TLKVTEGTVVLSKNIL; LKVTEGTVVLSKNILK; KVTEGTVVLSKNILKS; VTEGTVVLSKNILKSG; TEGTVVLSKNILKSGE; EGTVVLSKNILKSGEI; GTVVLSKNILKSGEIT; TVVLSKNILKSGEITV; VVLSKNILKSGEITVA; VLSKNILKSGEITVAL; LSKNILKSGEITVALD; SKNILKSGEITVALDD; KNILKSGEITVALDDS; NILKSGEITVALDDSD; ILKSGEITVALDDSDT; LKSGEITVALDDSDTT; KSGEITVALDDSDTTQ; SGEITVALDDSDTTQA; GEITVALDDSDTTQAT; EITVALDDSDTTQATK; ITVALDDSDTTQATKK; TVALDDSDTTQATKKT; VALDDSDTTQATKKTG; ALDDSDTTQATKKTGK; LDDSDTTQATKKTGKW; DDSDTTQATKKTGKWD; DSDTTQATKKTGKWDS; SDTTQATKKTGKWDSK; DTTQATKKTGKWDSKT; TTQATKKTGKWDSKTS; TQATKKTGKWDSKTST; QATKKTGKWDSKTSTL; ATKKTGKWDSKTSTLT; TKKTGKWDSKTSTLTI; KKTGKWDSKTSTLTIS; KTGKWDSKTSTLTISV; TGKWDSKTSTLTISVN; GKWDSKTSTLTISVNS; KWDSKTSTLTISVNSQ; WDSKTSTLTISVNSQK; DSKTSTLTISVNSQKT; SKTSTLTISVNSQKTK; KTSTLTISVNSQKTKN; TSTLTISVNSQKTKNL; STLTISVNSQKTKNLV; TLTISVNSQKTKNLVF; LTISVNSQKTKNLVFT; TISVNSQKTKNLVFTK; ISVNSQKTKNLVFTKE; SVNSQKTKNLVFTKED; VNSQKTKNLVFTKEDT; NSQKTKNLVFTKEDTI; SQKTKNLVFTKEDTIT; QKTKNLVFTKEDTITV; KTKNLVFTKEDTITVQ; TKNLVFTKEDTITVQK; KNLVFTKEDTITVQKY; NLVFTKEDTITVQKYD; LVFTKEDTITVQKYDS; VFTKEDTITVQKYDSA; FTKEDTITVQKYDSAG; TKEDTITVQKYDSAGT; KEDTITVQKYDSAGTN; EDTITVQKYDSAGTNL; DTITVQKYDSAGTNLE; TITVQKYDSAGTNLEG; ITVQKYDSAGTNLEGK; TVQKYDSAGTNLEGKA; VQKYDSAGTNLEGKAV; QKYDSAGTNLEGKAVE; KYDSAGTNLEGKAVEI; YDSAGTNLEGKAVEIT; DSAGTNLEGKAVEITT; SAGTNLEGKAVEITTL; AGTNLEGKAVEITTLK; GTNLEGKAVEITTLKE; TNLEGKAVEITTLKEL; NLEGKAVEITTLKELK; LEGKAVEITTLKELKN; EGKAVEITTLKELKNA; GKAVEITTLKELKNAL; KAVEITTLKELKNALK; AVEITTLKELKNALK□; |
| \|BAA22351.1\| Outer surface protein B [Borrelia garinii] | 13 mers: MKKYLLGFALVLA; KKYLLGFALVLAL; KYLLGFALVLALI; YLLGFALVLALIA; LLGFALVLALIAC; LGFALVLALIACG; GFALVLALIACGQ; FALVLALIACGQK; ALVLALIACGQKG; LVLALIACGQKGA; VLALIACGQKGAE; LALIACGQKGAEP; ALIACGQKGAEPK; LIACGQKGAEPKH; IACGQKGAEPKHN; ACGQKGAEPKHND; CGQKGAEPKHNDQ; GQKGAEPKHNDQE; QKGAEPKHNDQEV; KGAEPKHNDQEVE; GAEPKHNDQEVED; AEPKHNDQEVEDS; EPKHNDQEVEDSK; PKHNDQEVEDSKK; KHNDQEVEDSKKD; HNDQEVEDSKKDQ; NDQEVEDSKKDQK; DQEVEDSKKDQKD; QEVEDSKKDQKDA; EVEDSKKDQKDAS; VEDSKKDQKDASK; EDSKKDQKDASKK; DSKKDQKDASKKD; SKKDQKDASKKDL; KKDQKDASKKDLP; KDQKDASKKDLPL; DQKDASKKDLPLV; QKDASKKDLPLVT; KDASKKDLPLVTE; DASKKDLPLVTED; ASKKDLPLVTEDT; SKKDLPLVTEDTV; KKDLPLVTEDTVK; KDLPLVTEDTVKL; DLPLVTEDTVKLF; LPLVTEDTVKLFN; PLVTEDTVKLFND; LVTEDTVKLFNDT; VTEDTVKLFNDTK; TEDTVKLFNDTKI; EDTVKLFNDTKIF; DTVKLFNDTKIFI; TVKLFNDTKIFIS; VKLFNDTKIFISK; KLFNDTKIFISKE; LFNDTKIFISKEK; FNDTKIFISKEKN; NDTKIFISKEKNK; DTKIFISKEKNKD; TKIFISKEKNKDG; KIFISKEKNKDGK; IFISKEKNKDGKY; FISKEKNKDGKYE; ISKEKNKDGKYEL; SKEKNKDGKYELR; KEKNKDGKYELRA; EKNKDGKYELRAT; KNKDGKYELRATV; NKDGKYELRATVD; KDGKYELRATVDT; DGKYELRATVDTV; GKYELRATVDTVE; KYELRATVDTVEL; YELRATVDTVELK; ELRATVDTVELKG; LRATVDTVELKGV; RATVDTVELKGVA; ATVDTVELKGVAD; TVDTVELKGVADK; VDTVELKGVADKN; DTVELKGVADKND; TVELKGVADKNDG; VELKGVADKNDGS; ELKGVADKNDGSG; LKGVADKNDGSGG; KGVADKNDGSGGK; GVADKNDGSGGKL; VADKNDGSGGKLE; |

ADKNDGSGGKLEG; DKNDGSGGKLEGV; KNDGSGGKLEGVK; NDGSGGKLEGVKS;
DGSGGKLEGVKSD; GSGGKLEGVKSDQ; SGGKLEGVKSDQS; GGKLEGVKSDQSK;
GKLEGVKSDQSKV; KLEGVKSDQSKVT; LEGVKSDQSKVTM; EGVKSDQSKVTMS;
GVKSDQSKVTMSI; VKSDQSKVTMSIT; KSDQSKVTMSITD; SDQSKVTMSITDD;
DQSKVTMSITDDL; QSKVTMSITDDLN; SKVTMSITDDLNT; KVTMSITDDLNTI;
VTMSITDDLNTIT; TMSITDDLNTITV; MSITDDLNTITVE; SITDDLNTITVET;
ITDDLNTITVETY; TDDLNTITVETYD; DDLNTITVETYDS; DLNTITVETYDSS;
LNTITVETYDSSN; NTITVETYDSSNT; TITVETYDSSNTK; ITVETYDSSNTKV;
TVETYDSSNTKVA; VETYDSSNTKVAS; ETYDSSNTKVASK; TYDSSNTKVASKV;
YDSSNTKVASKVF; DSSNTKVASKVFK; SSNTKVASKVFKK; SNTKVASKVFKKQ;
NTKVASKVFKKQG; TKVASKVFKKQGS; KVASKVFKKQGSL; VASKVFKKQGSLT;
ASKVFKKQGSLTE; SKVFKKQGSLTEE; KVFKKQGSLTEET; VFKKQGSLTEETE;
FKKQGSLTEETEE; KKQGSLTEETEET; KQGSLTEETEETY; QGSLTEETEETYK;
GSLTEETEETYKT; SLTEETEETYKTG; LTEETEETYKTGK; TEETEETYKTGKL;
EETEETYKTGKLS; ETEETYKTGKLST; TEETYKTGKLSTK; EETYKTGKLSTKK;
ETYKTGKLSTKKI; TYKTGKLSTKKIT; YKTGKLSTKKITR; KTGKLSTKKITRT;
TGKLSTKKITRTN; GKLSTKKITRTNG; KLSTKKITRTNGT; LSTKKITRTNGTT;
STKKITRTNGTTL; TKKITRTNGTTLE; KKITRTNGTTLEY; KITRTNGTTLEYS;
ITRTNGTTLEYSD; TRTNGTTLEYSDM; RTNGTTLEYSDMT; TNGTTLEYSDMTN;
NGTTLEYSDMTND; GTTLEYSDMTNDE; TTLEYSDMTNDEN; TLEYSDMTNDENA;
LEYSDMTNDENAT; EYSDMTNDENATK; YSDMTNDENATKA; SDMTNDENATKAV;
DMTNDENATKAVE; MTNDENATKAVET; TNDENATKAVETL; NDENATKAVETLK;
DENATKAVETLKN; ENATKAVETLKNG; NATKAVETLKNGI; ATKAVETLKNGIM;
TKAVETLKNGIML; KAVETLKNGIMLE; AVETLKNGIMLEG; VETLKNGIMLEGN;
ETLKNGIMLEGNL; TLKNGIMLEGNLV; LKNGIMLEGNLVG; KNGIMLEGNLVGG;
NGIMLEGNLVGGK; GIMLEGNLVGGKT; IMLEGNLVGGKTS; MLEGNLVGGKTSV;
LEGNLVGGKTSVE; EGNLVGGKTSVEI; GNLVGGKTSVEIK; NLVGGKTSVEIKE;
LVGGKTSVEIKEG; VGGKTSVEIKEGT; GGKTSVEIKEGTV; GKTSVEIKEGTVT;
KTSVEIKEGTVTL; TSVEIKEGTVTLK; SVEIKEGTVTLKK; VEIKEGTVTLKKE;
EIKEGTVTLKKEI; IKEGTVTLKKEIE; KEGTVTLKKEIEK; EGTVTLKKEIEKA;
GTVTLKKEIEKAG; TVTLKKEIEKAGT; VTLKKEIEKAGTV; TLKKEIEKAGTVK;
LKKEIEKAGTVKL; KKEIEKAGTVKLF; KEIEKAGTVKLFL; EIEKAGTVKLFLD;
IEKAGTVKLFLDD; EKAGTVKLFLDDT; KAGTVKLFLDDTS; AGTVKLFLDDTSS;
GTVKLFLDDTSSG; TVKLFLDDTSSGS; VKLFLDDTSSGST; KLFLDDTSSGSTK;
LFLDDTSSGSTKK; FLDDTSSGSTKKT; LDDTSSGSTKKTA; DDTSSGSTKKTAV;
DTSSGSTKKTAVW; TSSGSTKKTAVWS; SSGSTKKTAVWSD; SGSTKKTAVWSDT;
GSTKKTAVWSDTS; STKKTAVWSDTSN; TKKTAVWSDTSNT; KKTAVWSDTSNTL;
KTAVWSDTSNTLT; TAVWSDTSNTLTV; AVWSDTSNTLTVS; VWSDTSNTLTVSA;
WSDTSNTLTVSAD; SDTSNTLTVSADS; DTSNTLTVSADSK; TSNTLTVSADSKK;
SNTLTVSADSKKI; NTLTVSADSKKIK; TLTVSADSKKIKD; LTVSADSKKIKDF;
TVSADSKKIKDFV; VSADSKKIKDFVF; SADSKKIKDFVFL; ADSKKIKDFVFLT;
DSKKIKDFVFLTD; SKKIKDFVFLTDG; KKIKDFVFLTDGT; KIKDFVFLTDGTI;
IKDFVFLTDGTIT; KDFVFLTDGTITV; DFVFLTDGTITVQ; FVFLTDGTITVQN;
VFLTDGTITVQNY; FLTDGTITVQNYD; LTDGTITVQNYDT; TDGTITVQNYDTA;
DGTITVQNYDTAG; GTITVQNYDTAGT; TITVQNYDTAGTK; ITVQNYDTAGTKL;
TVQNYDTAGTKLA; VQNYDTAGTKLAG; QNYDTAGTKLAGT; NYDTAGTKLAGTA;
YDTAGTKLAGTAT; DTAGTKLAGTATE; TAGTKLAGTATEI; AGTKLAGTATEIK;
GTKLAGTATEIKD; TKLAGTATEIKDL; KLAGTATEIKDLE; LAGTATEIKDLEA;
AGTATEIKDLEAL; GTATEIKDLEALK; TATEIKDLEALKA; ATEIKDLEALKAA;
TEIKDLEALKAAL; EIKDLEALKAALK;

14 mers:
MKKYLLGFALVLAL; KKYLLGFALVLALI; KYLLGFALVLALIA;
YLLGFALVLALIAC; LLGFALVLALIACG; LGFALVLALIACGQ;
GFALVLALIACGQK; FALVLALIACGQKG; ALVLALIACGQKGA;
LVLALIACGQKGAE; VLALIACGQKGAEP; LALIACGQKGAEPK;
ALIACGQKGAEPKH; LIACGQKGAEPKHN; IACGQKGAEPKHND;

| | |
|---|---|
| ACGQKGAEPKHNDQ; CGQKGAEPKHNDQE; GQKGAEPKHNDQEV; QKGAEPKHNDQEVE; KGAEPKHNDQEVED; GAEPKHNDQEVEDS; AEPKHNDQEVEDSK; EPKHNDQEVEDSKK; PKHNDQEVEDSKKD; KHNDQEVEDSKKDQ; HNDQEVEDSKKDQK; NDQEVEDSKKDQKD; DQEVEDSKKDQKDA; QEVEDSKKDQKDAS; EVEDSKKDQKDASK; VEDSKKDQKDASKK; EDSKKDQKDASKKD; DSKKDQKDASKKDL; SKKDQKDASKKDLP; KKDQKDASKKDLPL; KDQKDASKKDLPLV; DQKDASKKDLPLVT; QKDASKKDLPLVTE; KDASKKDLPLVTED; DASKKDLPLVTEDT; ASKKDLPLVTEDTV; SKKDLPLVTEDTVK; KKDLPLVTEDTVKL; KDLPLVTEDTVKLF; DLPLVTEDTVKLFN; LPLVTEDTVKLFND; PLVTEDTVKLFNDT; LVTEDTVKLFNDTK; VTEDTVKLFNDTKI; TEDTVKLFNDTKIF; EDTVKLFNDTKIFI; DTVKLFNDTKIFIS; TVKLFNDTKIFISK; VKLFNDTKIFISKE; KLFNDTKIFISKEK; LFNDTKIFISKEKN; FNDTKIFISKEKNK; NDTKIFISKEKNKD; DTKIFISKEKNKDG; TKIFISKEKNKDGK; KIFISKEKNKDGKY; IFISKEKNKDGKYE; FISKEKNKDGKYEL; ISKEKNKDGKYELR; SKEKNKDGKYELRA; KEKNKDGKYELRAT; EKNKDGKYELRATV; KNKDGKYELRATVD; NKDGKYELRATVDT; KDGKYELRATVDTV; DGKYELRATVDTVE; GKYELRATVDTVEL; KYELRATVDTVELK; YELRATVDTVELKG; ELRATVDTVELKGV; LRATVDTVELKGVA; RATVDTVELKGVAD; ATVDTVELKGVADK; TVDTVELKGVADKN; VDTVELKGVADKND; DTVELKGVADKNDG; TVELKGVADKNDGS; VELKGVADKNDGSG; ELKGVADKNDGSGG; LKGVADKNDGSGGK; KGVADKNDGSGGKL; GVADKNDGSGGKLE; VADKNDGSGGKLEG; ADKNDGSGGKLEGV; DKNDGSGGKLEGVK; KNDGSGGKLEGVKS; NDGSGGKLEGVKSD; DGSGGKLEGVKSDQ; GSGGKLEGVKSDQS; SGGKLEGVKSDQSK; GGKLEGVKSDQSKV; GKLEGVKSDQSKVT; KLEGVKSDQSKVTM; LEGVKSDQSKVTMS; EGVKSDQSKVTMSI; GVKSDQSKVTMSIT; VKSDQSKVTMSITD; KSDQSKVTMSITDD; SDQSKVTMSITDDL; DQSKVTMSITDDLN; QSKVTMSITDDLNT; SKVTMSITDDLNTI; KVTMSITDDLNTIT; VTMSITDDLNTITV; TMSITDDLNTITVE; MSITDDLNTITVET; SITDDLNTITVETY; ITDDLNTITVETYD; TDDLNTITVETYDS; DDLNTITVETYDSS; DLNTITVETYDSSN; LNTITVETYDSSNT; NTITVETYDSSNTK; TITVETYDSSNTKV; ITVETYDSSNTKVA; TVETYDSSNTKVAS; VETYDSSNTKVASK; ETYDSSNTKVASKV; TYDSSNTKVASKVF; YDSSNTKVASKVFK; DSSNTKVASKVFKK; SSNTKVASKVFKKQ; SNTKVASKVFKKQG; NTKVASKVFKKQGS; TKVASKVFKKQGSL; KVASKVFKKQGSLT; VASKVFKKQGSLTE; ASKVFKKQGSLTEE; SKVFKKQGSLTEET; KVFKKQGSLTEETE; VFKKQGSLTEETEE; FKKQGSLTEETEET; KKQGSLTEETEETY; KQGSLTEETEETYK; QGSLTEETEETYKT; GSLTEETEETYKTG; SLTEETEETYKTGK; LTEETEETYKTGKL; TEETEETYKTGKLS; EETEETYKTGKLST; ETEETYKTGKLSTK; TEETYKTGKLSTKK; EETYKTGKLSTKKI; ETYKTGKLSTKKIT; TYKTGKLSTKKITR; YKTGKLSTKKITRT; KTGKLSTKKITRTN; TGKLSTKKITRTNG; GKLSTKKITRTNGT; KLSTKKITRTNGTT; LSTKKITRTNGTTL; STKKITRTNGTTLE; TKKITRTNGTTLEY; KKITRTNGTTLEYS; KITRTNGTTLEYSD; ITRTNGTTLEYSDM; TRTNGTTLEYSDMT; RTNGTTLEYSDMTN; TNGTTLEYSDMTND; NGTTLEYSDMTNDE; GTTLEYSDMTNDEN; TTLEYSDMTNDENA; TLEYSDMTNDENAT; LEYSDMTNDENATK; EYSDMTNDENATKA; YSDMTNDENATKAV; SDMTNDENATKAVE; DMTNDENATKAVET; MTNDENATKAVETL; TNDENATKAVETLK; NDENATKAVETLKN; DENATKAVETLKNG; ENATKAVETLKNGI; NATKAVETLKNGIM; ATKAVETLKNGIML; TKAVETLKNGIMLE; KAVETLKNGIMLEG; AVETLKNGIMLEGN; VETLKNGIMLEGNL; ETLKNGIMLEGNLV; TLKNGIMLEGNLVG; |

LKNGIMLEGNLVGG; KNGIMLEGNLVGGK; NGIMLEGNLVGGKT;
GIMLEGNLVGGKTS; IMLEGNLVGGKTSV; MLEGNLVGGKTSVE;
LEGNLVGGKTSVEI; EGNLVGGKTSVEIK; GNLVGGKTSVEIKE;
NLVGGKTSVEIKEG; LVGGKTSVEIKEGT; VGGKTSVEIKEGTV;
GGKTSVEIKEGTVT; GKTSVEIKEGTVTL; KTSVEIKEGTVTLK;
TSVEIKEGTVTLKK; SVEIKEGTVTLKKE; VEIKEGTVTLKKEI;
EIKEGTVTLKKEIE; IKEGTVTLKKEIEK; KEGTVTLKKEIEKA;
EGTVTLKKEIEKAG; GTVTLKKEIEKAGT; TVTLKKEIEKAGTV;
VTLKKEIEKAGTVK; TLKKEIEKAGTVKL; LKKEIEKAGTVKLF;
KKEIEKAGTVKLFL; KEIEKAGTVKLFLD; EIEKAGTVKLFLDD;
IEKAGTVKLFLDDT; EKAGTVKLFLDDTS; KAGTVKLFLDDTSS;
AGTVKLFLDDTSSG; GTVKLFLDDTSSGS; TVKLFLDDTSSGST;
VKLFLDDTSSGSTK; KLFLDDTSSGSTKK; LFLDDTSSGSTKKT;
FLDDTSSGSTKKTA; LDDTSSGSTKKTAV; DDTSSGSTKKTAVW;
DTSSGSTKKTAVWS; TSSGSTKKTAVWSD; SSGSTKKTAVWSDT;
SGSTKKTAVWSDTS; GSTKKTAVWSDTSN; STKKTAVWSDTSNT;
TKKTAVWSDTSNTL; KKTAVWSDTSNTLT; KTAVWSDTSNTLTV;
TAVWSDTSNTLTVS; AVWSDTSNTLTVSA; VWSDTSNTLTVSAD;
WSDTSNTLTVSADS; SDTSNTLTVSADSK; DTSNTLTVSADSKK;
TSNTLTVSADSKKI; SNTLTVSADSKKIK; NTLTVSADSKKIKD;
TLTVSADSKKIKDF; LTVSADSKKIKDFV; TVSADSKKIKDFVF;
VSADSKKIKDFVFL; SADSKKIKDFVFLT; ADSKKIKDFVFLTD;
DSKKIKDFVFLTDG; SKKIKDFVFLTDGT; KKIKDFVFLTDGTI;
KIKDFVFLTDGTIT; IKDFVFLTDGTITV; KDFVFLTDGTITVQ;
DFVFLTDGTITVQN; FVFLTDGTITVQNY; VFLTDGTITVQNYD;
FLTDGTITVQNYDT; LTDGTITVQNYDTA; TDGTITVQNYDTAG;
DGTITVQNYDTAGT; GTITVQNYDTAGTK; TITVQNYDTAGTKL;
ITVQNYDTAGTKLA; TVQNYDTAGTKLAG; VQNYDTAGTKLAGT;
QNYDTAGTKLAGTA; NYDTAGTKLAGTAT; YDTAGTKLAGTATE;
DTAGTKLAGTATEI; TAGTKLAGTATEIK; AGTKLAGTATEIKD;
GTKLAGTATEIKDL; TKLAGTATEIKDLE; KLAGTATEIKDLEA;
LAGTATEIKDLEAL; AGTATEIKDLEALK; GTATEIKDLEALKA;
TATEIKDLEALKAA; ATEIKDLEALKAAL; TEIKDLEALKAALK;

15 mers:
MKKYLLGFALVLALI; KKYLLGFALVLALIA; KYLLGFALVLALIAC;
YLLGFALVLALIACG; LLGFALVLALIACGQ; LGFALVLALIACGQK;
GFALVLALIACGQKG; FALVLALIACGQKGA; ALVLALIACGQKGAE;
LVLALIACGQKGAEP; VLALIACGQKGAEPK; LALIACGQKGAEPKH;
ALIACGQKGAEPKHN; LIACGQKGAEPKHND; IACGQKGAEPKHNDQ;
ACGQKGAEPKHNDQE; CGQKGAEPKHNDQEV; GQKGAEPKHNDQEVE;
QKGAEPKHNDQEVED; KGAEPKHNDQEVEDS; GAEPKHNDQEVEDSK;
AEPKHNDQEVEDSKK; EPKHNDQEVEDSKKD; PKHNDQEVEDSKKDQ;
KHNDQEVEDSKKDQK; HNDQEVEDSKKDQKD; NDQEVEDSKKDQKDA;
DQEVEDSKKDQKDAS; QEVEDSKKDQKDASK; EVEDSKKDQKDASKK;
VEDSKKDQKDASKKD; EDSKKDQKDASKKDL; DSKKDQKDASKKDLP;
SKKDQKDASKKDLPL; KKDQKDASKKDLPLV; KDQKDASKKDLPLVT;
DQKDASKKDLPLVTE; QKDASKKDLPLVTED; KDASKKDLPLVTEDT;
DASKKDLPLVTEDTV; ASKKDLPLVTEDTVK; SKKDLPLVTEDTVKL;
KKDLPLVTEDTVKLF; KDLPLVTEDTVKLFN; DLPLVTEDTVKLFND;
LPLVTEDTVKLFNDT; PLVTEDTVKLFNDTK; LVTEDTVKLFNDTKI;
VTEDTVKLFNDTKIF; TEDTVKLFNDTKIFI; EDTVKLFNDTKIFIS;
DTVKLFNDTKIFISK; TVKLFNDTKIFISKE; VKLFNDTKIFISKEK;
KLFNDTKIFISKEKN; LFNDTKIFISKEKNK; FNDTKIFISKEKNKD;
NDTKIFISKEKNKDG; DTKIFISKEKNKDGK; TKIFISKEKNKDGKY;
KIFISKEKNKDGKYE; IFISKEKNKDGKYEL; FISKEKNKDGKYELR;
ISKEKNKDGKYELRA; SKEKNKDGKYELRAT; KEKNKDGKYELRATV;

EKNKDGKYELRATVD; KNKDGKYELRATVDT; NKDGKYELRATVDTV;
KDGKYELRATVDTVE; DGKYELRATVDTVEL; GKYELRATVDTVELK;
KYELRATVDTVELKG; YELRATVDTVELKGV; ELRATVDTVELKGVA;
LRATVDTVELKGVAD; RATVDTVELKGVADK; ATVDTVELKGVADKN;
TVDTVELKGVADKND; VDTVELKGVADKNDG; DTVELKGVADKNDGS;
TVELKGVADKNDGSG; VELKGVADKNDGSGG; ELKGVADKNDGSGGK;
LKGVADKNDGSGGKL; KGVADKNDGSGGKLE; GVADKNDGSGGKLEG;
VADKNDGSGGKLEGV; ADKNDGSGGKLEGVK; DKNDGSGGKLEGVKS;
KNDGSGGKLEGVKSD; NDGSGGKLEGVKSDQ; DGSGGKLEGVKSDQS;
GSGGKLEGVKSDQSK; SGGKLEGVKSDQSKV; GGKLEGVKSDQSKVT;
GKLEGVKSDQSKVTM; KLEGVKSDQSKVTMS; LEGVKSDQSKVTMSI;
EGVKSDQSKVTMSIT; GVKSDQSKVTMSITD; VKSDQSKVTMSITDD;
KSDQSKVTMSITDDL; SDQSKVTMSITDDLN; DQSKVTMSITDDLNT;
QSKVTMSITDDLNTI; SKVTMSITDDLNTIT; KVTMSITDDLNTITV;
VTMSITDDLNTITVE; TMSITDDLNTITVET; MSITDDLNTITVETY;
SITDDLNTITVETYD; ITDDLNTITVETYDS; TDDLNTITVETYDSS;
DDLNTITVETYDSSN; DLNTITVETYDSSNT; LNTITVETYDSSNTK;
NTITVETYDSSNTKV; TITVETYDSSNTKVA; ITVETYDSSNTKVAS;
TVETYDSSNTKVASK; VETYDSSNTKVASKV; ETYDSSNTKVASKVF;
TYDSSNTKVASKVFK; YDSSNTKVASKVFKK; DSSNTKVASKVFKKQ;
SSNTKVASKVFKKQG; SNTKVASKVFKKQGS; NTKVASKVFKKQGSL;
TKVASKVFKKQGSLT; KVASKVFKKQGSLTE; VASKVFKKQGSLTEE;
ASKVFKKQGSLTEET; SKVFKKQGSLTEETE; KVFKKQGSLTEETEE;
VFKKQGSLTEETEET; FKKQGSLTEETEETY; KKQGSLTEETEETYK;
KQGSLTEETEETYKT; QGSLTEETEETYKTG; GSLTEETEETYKTGK;
SLTEETEETYKTGKL; LTEETEETYKTGKLS; TEETEETYKTGKLST;
EETEETYKTGKLSTK; ETEETYKTGKLSTKK; TEETYKTGKLSTKKI;
EETYKTGKLSTKKIT; ETYKTGKLSTKKITR; TYKTGKLSTKKITRT;
YKTGKLSTKKITRTN; KTGKLSTKKITRTNG; TGKLSTKKITRTNGT;
GKLSTKKITRTNGTT; KLSTKKITRTNGTTL; LSTKKITRTNGTTLE;
STKKITRTNGTTLEY; TKKITRTNGTTLEYS; KKITRTNGTTLEYSD;
KITRTNGTTLEYSDM; ITRTNGTTLEYSDMT; TRTNGTTLEYSDMTN;
RTNGTTLEYSDMTND; TNGTTLEYSDMTNDE; NGTTLEYSDMTNDEN;
GTTLEYSDMTNDENA; TTLEYSDMTNDENAT; TLEYSDMTNDENATK;
LEYSDMTNDENATKA; EYSDMTNDENATKAV; YSDMTNDENATKAVE;
SDMTNDENATKAVET; DMTNDENATKAVETL; MTNDENATKAVETLK;
TNDENATKAVETLKN; NDENATKAVETLKNG; DENATKAVETLKNGI;
ENATKAVETLKNGIM; NATKAVETLKNGIML; ATKAVETLKNGIMLE;
TKAVETLKNGIMLEG; KAVETLKNGIMLEGN; AVETLKNGIMLEGNL;
VETLKNGIMLEGNLV; ETLKNGIMLEGNLVG; TLKNGIMLEGNLVGG;
LKNGIMLEGNLVGGK; KNGIMLEGNLVGGKT; NGIMLEGNLVGGKTS;
GIMLEGNLVGGKTSV; IMLEGNLVGGKTSVE; MLEGNLVGGKTSVEI;
LEGNLVGGKTSVEIK; EGNLVGGKTSVEIKE; GNLVGGKTSVEIKEG;
NLVGGKTSVEIKEGT; LVGGKTSVEIKEGTV; VGGKTSVEIKEGTVT;
GGKTSVEIKEGTVTL; GKTSVEIKEGTVTLK; KTSVEIKEGTVTLKK;
TSVEIKEGTVTLKKE; SVEIKEGTVTLKKEI; VEIKEGTVTLKKEIE;
EIKEGTVTLKKEIEK; IKEGTVTLKKEIEKA; KEGTVTLKKEIEKAG;
EGTVTLKKEIEKAGT; GTVTLKKEIEKAGTV; TVTLKKEIEKAGTVK;
VTLKKEIEKAGTVKL; TLKKEIEKAGTVKLF; LKKEIEKAGTVKLFL;
KKEIEKAGTVKLFLD; KEIEKAGTVKLFLDD; EIEKAGTVKLFLDDT;
IEKAGTVKLFLDDTS; EKAGTVKLFLDDTSS; KAGTVKLFLDDTSSG;
AGTVKLFLDDTSSGS; GTVKLFLDDTSSGST; TVKLFLDDTSSGSTK;
VKLFLDDTSSGSTKK; KLFLDDTSSGSTKKT; LFLDDTSSGSTKKTA;
FLDDTSSGSTKKTAV; LDDTSSGSTKKTAVW; DDTSSGSTKKTAVWS;
DTSSGSTKKTAVWSD; TSSGSTKKTAVWSDT; SSGSTKKTAVWSDTS;
SGSTKKTAVWSDTSN; GSTKKTAVWSDTSNT; STKKTAVWSDTSNTL;
TKKTAVWSDTSNTLT; KKTAVWSDTSNTLTV; KTAVWSDTSNTLTVS;

TAVWSDTSNTLTVSA; AVWSDTSNTLTVSAD; VWSDTSNTLTVSADS;
WSDTSNTLTVSADSK; SDTSNTLTVSADSKK; DTSNTLTVSADSKKI;
TSNTLTVSADSKKIK; SNTLTVSADSKKIKD; NTLTVSADSKKIKDF;
TLTVSADSKKIKDFV; LTVSADSKKIKDFVF; TVSADSKKIKDFVFL;
VSADSKKIKDFVFLT; SADSKKIKDFVFLTD; ADSKKIKDFVFLTDG;
DSKKIKDFVFLTDGT; SKKIKDFVFLTDGTI; KKIKDFVFLTDGTIT;
KIKDFVFLTDGTITV; IKDFVFLTDGTITVQ; KDFVFLTDGTITVQN;
DFVFLTDGTITVQNY; FVFLTDGTITVQNYD; VFLTDGTITVQNYDT;
FLTDGTITVQNYDTA; LTDGTITVQNYDTAG; TDGTITVQNYDTAGT;
DGTITVQNYDTAGTK; GTITVQNYDTAGTKL; TITVQNYDTAGTKLA;
ITVQNYDTAGTKLAG; TVQNYDTAGTKLAGT; VQNYDTAGTKLAGTA;
QNYDTAGTKLAGTAT; NYDTAGTKLAGTATE; YDTAGTKLAGTATEI;
DTAGTKLAGTATEIK; TAGTKLAGTATEIKD; AGTKLAGTATEIKDL;
GTKLAGTATEIKDLE; TKLAGTATEIKDLEA; KLAGTATEIKDLEAL;
LAGTATEIKDLEALK; AGTATEIKDLEALKA; GTATEIKDLEALKAA;
TATEIKDLEALKAAL; ATEIKDLEALKAALK;

16 mers:
MKKYLLGFALVLALIA; KKYLLGFALVLALIAC; KYLLGFALVLALIACG;
YLLGFALVLALIACGQ; LLGFALVLALIACGQK; LGFALVLALIACGQKG;
GFALVLALIACGQKGA; FALVLALIACGQKGAE; ALVLALIACGQKGAEP;
LVLALIACGQKGAEPK; VLALIACGQKGAEPKH; LALIACGQKGAEPKHN;
ALIACGQKGAEPKHND; LIACGQKGAEPKHNDQ; IACGQKGAEPKHNDQE;
ACGQKGAEPKHNDQEV; CGQKGAEPKHNDQEVE; GQKGAEPKHNDQEVED;
QKGAEPKHNDQEVEDS; KGAEPKHNDQEVEDSK; GAEPKHNDQEVEDSKK;
AEPKHNDQEVEDSKKD; EPKHNDQEVEDSKKDQ; PKHNDQEVEDSKKDQK;
KHNDQEVEDSKKDQKD; HNDQEVEDSKKDQKDA; NDQEVEDSKKDQKDAS;
DQEVEDSKKDQKDASK; QEVEDSKKDQKDASKK; EVEDSKKDQKDASKKD;
VEDSKKDQKDASKKDL; EDSKKDQKDASKKDLP; DSKKDQKDASKKDLPL;
SKKDQKDASKKDLPLV; KKDQKDASKKDLPLVT; KDQKDASKKDLPLVTE;
DQKDASKKDLPLVTED; QKDASKKDLPLVTEDT; KDASKKDLPLVTEDTV;
DASKKDLPLVTEDTVK; ASKKDLPLVTEDTVKL; SKKDLPLVTEDTVKLF;
KKDLPLVTEDTVKLFN; KDLPLVTEDTVKLFND; DLPLVTEDTVKLFNDT;
LPLVTEDTVKLFNDTK; PLVTEDTVKLFNDTKI; LVTEDTVKLFNDTKIF;
VTEDTVKLFNDTKIFI; TEDTVKLFNDTKIFIS; EDTVKLFNDTKIFISK;
DTVKLFNDTKIFISKE; TVKLFNDTKIFISKEK; VKLFNDTKIFISKEKN;
KLFNDTKIFISKEKNK; LFNDTKIFISKEKNKD; FNDTKIFISKEKNKDG;
NDTKIFISKEKNKDGK; DTKIFISKEKNKDGKY; TKIFISKEKNKDGKYE;
KIFISKEKNKDGKYEL; IFISKEKNKDGKYELR; FISKEKNKDGKYELRA;
ISKEKNKDGKYELRAT; SKEKNKDGKYELRATV; KEKNKDGKYELRATVD;
EKNKDGKYELRATVDT; KNKDGKYELRATVDTV; NKDGKYELRATVDTVE;
KDGKYELRATVDTVEL; DGKYELRATVDTVELK; GKYELRATVDTVELKG;
KYELRATVDTVELKGV; YELRATVDTVELKGVA; ELRATVDTVELKGVAD;
LRATVDTVELKGVADK; RATVDTVELKGVADKN; ATVDTVELKGVADKND;
TVDTVELKGVADKNDG; VDTVELKGVADKNDGS; DTVELKGVADKNDGSG;
TVELKGVADKNDGSGG; VELKGVADKNDGSGGK; ELKGVADKNDGSGGKL;
LKGVADKNDGSGGKLE; KGVADKNDGSGGKLEG; GVADKNDGSGGKLEGV;
VADKNDGSGGKLEGVK; ADKNDGSGGKLEGVKS; DKNDGSGGKLEGVKSD;
KNDGSGGKLEGVKSDQ; NDGSGGKLEGVKSDQS; DGSGGKLEGVKSDQSK;
GSGGKLEGVKSDQSKV; SGGKLEGVKSDQSKVT; GGKLEGVKSDQSKVTM;
GKLEGVKSDQSKVTMS; KLEGVKSDQSKVTMSI; LEGVKSDQSKVTMSIT;
EGVKSDQSKVTMSITD; GVKSDQSKVTMSITDD; VKSDQSKVTMSITDDL;
KSDQSKVTMSITDDLN; SDQSKVTMSITDDLNT; DQSKVTMSITDDLNTI;
QSKVTMSITDDLNTIT; SKVTMSITDDLNTITV; KVTMSITDDLNTITVE;
VTMSITDDLNTITVET; TMSITDDLNTITVETY; MSITDDLNTITVETYD;
SITDDLNTITVETYDS; ITDDLNTITVETYDSS; TDDLNTITVETYDSSN;
DDLNTITVETYDSSNT; DLNTITVETYDSSNTK; LNTITVETYDSSNTKV;

| | | |
|---|---|---|
| NTITVETYDSSNTKVA; | TITVETYDSSNTKVAS; | ITVETYDSSNTKVASK; |
| TVETYDSSNTKVASKV; | VETYDSSNTKVASKVF; | ETYDSSNTKVASKVFK; |
| TYDSSNTKVASKVFKK; | YDSSNTKVASKVFKKQ; | DSSNTKVASKVFKKQG; |
| SSNTKVASKVFKKQGS; | SNTKVASKVFKKQGSL; | NTKVASKVFKKQGSLT; |
| TKVASKVFKKQGSLTE; | KVASKVFKKQGSLTEE; | VASKVFKKQGSLTEET; |
| ASKVFKKQGSLTEETE; | SKVFKKQGSLTEETEE; | KVFKKQGSLTEETEET; |
| VFKKQGSLTEETEETY; | FKKQGSLTEETEETYK; | KKQGSLTEETEETYKT; |
| KQGSLTEETEETYKTG; | QGSLTEETEETYKTGK; | GSLTEETEETYKTGKL; |
| SLTEETEETYKTGKLS; | LTEETEETYKTGKLST; | TEETEETYKTGKLSTK; |
| EETEETYKTGKLSTKK; | ETEETYKTGKLSTKKI; | TEETYKTGKLSTKKIT; |
| EETYKTGKLSTKKITR; | ETYKTGKLSTKKITRT; | TYKTGKLSTKKITRTN; |
| YKTGKLSTKKITRTNG; | KTGKLSTKKITRTNGT; | TGKLSTKKITRTNGTT; |
| GKLSTKKITRTNGTTL; | KLSTKKITRTNGTTLE; | LSTKKITRTNGTTLEY; |
| STKKITRTNGTTLEYS; | TKKITRTNGTTLEYSD; | KKITRTNGTTLEYSDM; |
| KITRTNGTTLEYSDMT; | ITRTNGTTLEYSDMTN; | TRTNGTTLEYSDMTND; |
| RTNGTTLEYSDMTNDE; | TNGTTLEYSDMTNDEN; | NGTTLEYSDMTNDENA; |
| GTTLEYSDMTNDENAT; | TTLEYSDMTNDENATK; | TLEYSDMTNDENATKA; |
| LEYSDMTNDENATKAV; | EYSDMTNDENATKAVE; | YSDMTNDENATKAVET; |
| SDMTNDENATKAVETL; | DMTNDENATKAVETLK; | MTNDENATKAVETLKN; |
| TNDENATKAVETLKNG; | NDENATKAVETLKNGI; | DENATKAVETLKNGIM; |
| ENATKAVETLKNGIML; | NATKAVETLKNGIMLE; | ATKAVETLKNGIMLEG; |
| TKAVETLKNGIMLEGN; | KAVETLKNGIMLEGNL; | AVETLKNGIMLEGNLV; |
| VETLKNGIMLEGNLVG; | ETLKNGIMLEGNLVGG; | TLKNGIMLEGNLVGGK; |
| LKNGIMLEGNLVGGKT; | KNGIMLEGNLVGGKTS; | NGIMLEGNLVGGKTSV; |
| GIMLEGNLVGGKTSVE; | IMLEGNLVGGKTSVEI; | MLEGNLVGGKTSVEIK; |
| LEGNLVGGKTSVEIKE; | EGNLVGGKTSVEIKEG; | GNLVGGKTSVEIKEGT; |
| NLVGGKTSVEIKEGTV; | LVGGKTSVEIKEGTVT; | VGGKTSVEIKEGTVTL; |
| GGKTSVEIKEGTVTLK; | GKTSVEIKEGTVTLKK; | KTSVEIKEGTVTLKKE; |
| TSVEIKEGTVTLKKEI; | SVEIKEGTVTLKKEIE; | VEIKEGTVTLKKEIEK; |
| EIKEGTVTLKKEIEKA; | IKEGTVTLKKEIEKAG; | KEGTVTLKKEIEKAGT; |
| EGTVTLKKEIEKAGTV; | GTVTLKKEIEKAGTVK; | TVTLKKEIEKAGTVKL; |
| VTLKKEIEKAGTVKLF; | TLKKEIEKAGTVKLFL; | LKKEIEKAGTVKLFLD; |
| KKEIEKAGTVKLFLDD; | KEIEKAGTVKLFLDDT; | EIEKAGTVKLFLDDTS; |
| IEKAGTVKLFLDDTSS; | EKAGTVKLFLDDTSSG; | KAGTVKLFLDDTSSGS; |
| AGTVKLFLDDTSSGST; | GTVKLFLDDTSSGSTK; | TVKLFLDDTSSGSTKK; |
| VKLFLDDTSSGSTKKT; | KLFLDDTSSGSTKKTA; | LFLDDTSSGSTKKTAV; |
| FLDDTSSGSTKKTAVW; | LDDTSSGSTKKTAVWS; | DDTSSGSTKKTAVWSD; |
| DTSSGSTKKTAVWSDT; | TSSGSTKKTAVWSDTS; | SSGSTKKTAVWSDTSN; |
| SGSTKKTAVWSDTSNT; | GSTKKTAVWSDTSNTL; | STKKTAVWSDTSNTLT; |
| TKKTAVWSDTSNTLTV; | KKTAVWSDTSNTLTVS; | KTAVWSDTSNTLTVSA; |
| TAVWSDTSNTLTVSAD; | AVWSDTSNTLTVSADS; | VWSDTSNTLTVSADSK; |
| WSDTSNTLTVSADSKK; | SDTSNTLTVSADSKKI; | DTSNTLTVSADSKKIK; |
| TSNTLTVSADSKKIKD; | SNTLTVSADSKKIKDF; | NTLTVSADSKKIKDFV; |
| TLTVSADSKKIKDFVF; | LTVSADSKKIKDFVFL; | TVSADSKKIKDFVFLT; |
| VSADSKKIKDFVFLTD; | SADSKKIKDFVFLTDG; | ADSKKIKDFVFLTDGT; |
| DSKKIKDFVFLTDGTI; | SKKIKDFVFLTDGTIT; | KKIKDFVFLTDGTITV; |
| KIKDFVFLTDGTITVQ; | IKDFVFLTDGTITVQN; | KDFVFLTDGTITVQNY; |
| DFVFLTDGTITVQNYD; | FVFLTDGTITVQNYDT; | VFLTDGTITVQNYDTA; |
| FLTDGTITVQNYDTAG; | LTDGTITVQNYDTAGT; | TDGTITVQNYDTAGTK; |
| DGTITVQNYDTAGTKL; | GTITVQNYDTAGTKLA; | TITVQNYDTAGTKLAG; |
| ITVQNYDTAGTKLAGT; | TVQNYDTAGTKLAGTA; | VQNYDTAGTKLAGTAT; |
| QNYDTAGTKLAGTATE; | NYDTAGTKLAGTATEI; | YDTAGTKLAGTATEIK; |
| DTAGTKLAGTATEIKD; | TAGTKLAGTATEIKDL; | AGTKLAGTATEIKDLE; |
| GTKLAGTATEIKDLEA; | TKLAGTATEIKDLEAL; | KLAGTATEIKDLEALK; |
| LAGTATEIKDLEALKA; | AGTATEIKDLEALKAA; | GTATEIKDLEALKAAL; |
| TATEIKDLEALKAALK; | | |

| AAM22469.1\| Outer surface protein C [Borrelia afzelii] | 13 mers: MKKNTLSAILMTL; KKNTLSAILMTLF; KNTLSAILMTLFL; NTLSAILMTLFLF; TLSAILMTLFLFI; LSAILMTLFLFIS; SAILMTLFLFISC; AILMTLFLFISCN; ILMTLFLFISCNN; LMTLFLFISCNNS; MTLFLFISCNNSG; TLFLFISCNNSGK; LFLFISCNNSGKG; FLFISCNNSGKGG; LFISCNNSGKGGD; FISCNNSGKGGDS; ISCNNSGKGGDSA; SCNNSGKGGDSAS; CNNSGKGGDSAST; NNSGKGGDSASTN; NSGKGGDSASTNP; SGKGGDSASTNPA; GKGGDSASTNPAD; KGGDSASTNPADE; GGDSASTNPADES; GDSASTNPADESA; DSASTNPADESAK; SASTNPADESAKG; ASTNPADESAKGP; STNPADESAKGPN; TNPADESAKGPNL; NPADESAKGPNLT; PADESAKGPNLTE; ADESAKGPNLTEI; DESAKGPNLTEIS; ESAKGPNLTEISK; SAKGPNLTEISKK; AKGPNLTEISKKI; KGPNLTEISKKIT; GPNLTEISKKITD; PNLTEISKKITDS; NLTEISKKITDSN; LTEISKKITDSNA; TEISKKITDSNAF; EISKKITDSNAFV; ISKKITDSNAFVL; SKKITDSNAFVLA; KKITDSNAFVLAV; KITDSNAFVLAVK; ITDSNAFVLAVKE; TDSNAFVLAVKEV; DSNAFVLAVKEVE; SNAFVLAVKEVET; NAFVLAVKEVETL; AFVLAVKEVETLV; FVLAVKEVETLVS; VLAVKEVETLVSS; LAVKEVETLVSSI; AVKEVETLVSSID; VKEVETLVSSIDE; KEVETLVSSIDEL; EVETLVSSIDELA; VETLVSSIDELAN; ETLVSSIDELANK; TLVSSIDELANKA; LVSSIDELANKAI; VSSIDELANKAIG; SSIDELANKAIGK; SIDELANKAIGKK; IDELANKAIGKKI; DELANKAIGKKIQ; ELANKAIGKKIQQ; LANKAIGKKIQQN; ANKAIGKKIQQNG; NKAIGKKIQQNGL; KAIGKKIQQNGLG; AIGKKIQQNGLGA; IGKKIQQNGLGAE; GKKIQQNGLGAEA; KKIQQNGLGAEAN; KIQQNGLGAEANR; IQQNGLGAEANRN; QQNGLGAEANRNE; QNGLGAEANRNES; NGLGAEANRNESL; GLGAEANRNESLL; LGAEANRNESLLA; GAEANRNESLLAG; AEANRNESLLAGV; EANRNESLLAGVH; ANRNESLLAGVHE; NRNESLLAGVHEI; RNESLLAGVHEIS; NESLLAGVHEIST; ESLLAGVHEISTL; SLLAGVHEISTLI; LLAGVHEISTLIT; LAGVHEISTLITE; AGVHEISTLITEK; GVHEISTLITEKL; VHEISTLITEKLS; HEISTLITEKLSK; EISTLITEKLSKL; ISTLITEKLSKLK; STLITEKLSKLKN; TLITEKLSKLKNS; LITEKLSKLKNSG; ITEKLSKLKNSGE; TEKLSKLKNSGEL; EKLSKLKNSGELK; KLSKLKNSGELKA; LSKLKNSGELKAK; SKLKNSGELKAKI; KLKNSGELKAKIE; LKNSGELKAKIED; KNSGELKAKIEDA; NSGELKAKIEDAK; SGELKAKIEDAKK; GELKAKIEDAKKC; ELKAKIEDAKKCS; LKAKIEDAKKCSE; KAKIEDAKKCSEE; AKIEDAKKCSEEF; KIEDAKKCSEEFT; IEDAKKCSEEFTN; EDAKKCSEEFTNK; DAKKCSEEFTNKL; AKKCSEEFTNKLR; KKCSEEFTNKLRV; KCSEEFTNKLRVS; CSEEFTNKLRVSH; SEEFTNKLRVSHA; EEFTNKLRVSHAD; EFTNKLRVSHADL; FTNKLRVSHADLG; TNKLRVSHADLGK; NKLRVSHADLGKQ; KLRVSHADLGKQG; LRVSHADLGKQGV; RVSHADLGKQGVN; VSHADLGKQGVND; SHADLGKQGVNDD; HADLGKQGVNDDD; ADLGKQGVNDDDA; DLGKQGVNDDDAK; LGKQGVNDDDAKK; GKQGVNDDDAKKA; KQGVNDDDAKKAI; QGVNDDDAKKAIL; GVNDDDAKKAILK; VNDDDAKKAILKT; NDDDAKKAILKTN; DDDAKKAILKTNA; DDAKKAILKTNAD; DAKKAILKTNADK; AKKAILKTNADKT; KKAILKTNADKTK; KAILKTNADKTKG; AILKTNADKTKGA; ILKTNADKTKGAE; LKTNADKTKGAEE; KTNADKTKGAEEL; TNADKTKGAEELG; NADKTKGAEELGK; ADKTKGAEELGKL; DKTKGAEELGKLF; KTKGAEELGKLFK; TKGAEELGKLFKS; KGAEELGKLFKSV; GAEELGKLFKSVE; AEELGKLFKSVEG; EELGKLFKSVEGL; ELGKLFKSVEGLV; LGKLFKSVEGLVK; GKLFKSVEGLVKA; KLFKSVEGLVKAA; LFKSVEGLVKAAQ; FKSVEGLVKAAQE; KSVEGLVKAAQEA; SVEGLVKAAQEAL; VEGLVKAAQEALT; EGLVKAAQEALTN; GLVKAAQEALTNS; LVKAAQEALTNSV; VKAAQEALTNSVK; KAAQEALTNSVKE; AAQEALTNSVKEL; AQEALTNSVKELT; QEALTNSVKELTS; EALTNSVKELTSP; ALTNSVKELTSPV; LTNSVKELTSPVV; TNSVKELTSPVVA; NSVKELTSPVVAE; SVKELTSPVVAES; VKELTSPVVAESP; KELTSPVVAESPK; ELTSPVVAESPKK; LTSPVVAESPKKP;<br><br>14 mers: MKKNTLSAILMTLF; KKNTLSAILMTLFL; KNTLSAILMTLFLF; NTLSAILMTLFLFI; TLSAILMTLFLFIS; LSAILMTLFLFISC; SAILMTLFLFISCN; AILMTLFLFISCNN; ILMTLFLFISCNNS; |

LMTLFLFISCNNSG; MTLFLFISCNNSGK; TLFLFISCNNSGKG;
LFLFISCNNSGKGG; FLFISCNNSGKGGD; LFISCNNSGKGGDS;
FISCNNSGKGGDSA; ISCNNSGKGGDSAS; SCNNSGKGGDSAST;
CNNSGKGGDSASTN; NNSGKGGDSASTNP; NSGKGGDSASTNPA;
SGKGGDSASTNPAD; GKGGDSASTNPADE; KGGDSASTNPADES;
GGDSASTNPADESA; GDSASTNPADESAK; DSASTNPADESAKG;
SASTNPADESAKGP; ASTNPADESAKGPN; STNPADESAKGPNL;
TNPADESAKGPNLT; NPADESAKGPNLTE; PADESAKGPNLTEI;
ADESAKGPNLTEIS; DESAKGPNLTEISK; ESAKGPNLTEISKK;
SAKGPNLTEISKKI; AKGPNLTEISKKIT; KGPNLTEISKKITD;
GPNLTEISKKITDS; PNLTEISKKITDSN; NLTEISKKITDSNA;
LTEISKKITDSNAF; TEISKKITDSNAFV; EISKKITDSNAFVL;
ISKKITDSNAFVLA; SKKITDSNAFVLAV; KKITDSNAFVLAVK;
KITDSNAFVLAVKE; ITDSNAFVLAVKEV; TDSNAFVLAVKEVE;
DSNAFVLAVKEVET; SNAFVLAVKEVETL; NAFVLAVKEVETLV;
AFVLAVKEVETLVS; FVLAVKEVETLVSS; VLAVKEVETLVSSI;
LAVKEVETLVSSID; AVKEVETLVSSIDE; VKEVETLVSSIDEL;
KEVETLVSSIDELA; EVETLVSSIDELAN; VETLVSSIDELANK;
ETLVSSIDELANKA; TLVSSIDELANKAI; LVSSIDELANKAIG;
VSSIDELANKAIGK; SSIDELANKAIGKK; SIDELANKAIGKKI;
IDELANKAIGKKIQ; DELANKAIGKKIQQ; ELANKAIGKKIQQN;
LANKAIGKKIQQNG; ANKAIGKKIQQNGL; NKAIGKKIQQNGLG;
KAIGKKIQQNGLGA; AIGKKIQQNGLGAE; IGKKIQQNGLGAEA;
GKKIQQNGLGAEAN; KKIQQNGLGAEANR; KIQQNGLGAEANRN;
IQQNGLGAEANRNE; QQNGLGAEANRNES; QNGLGAEANRNESL;
NGLGAEANRNESLL; GLGAEANRNESLLA; LGAEANRNESLLAG;
GAEANRNESLLAGV; AEANRNESLLAGVH; EANRNESLLAGVHE;
ANRNESLLAGVHEI; NRNESLLAGVHEIS; RNESLLAGVHEIST;
NESLLAGVHEISTL; ESLLAGVHEISTLI; SLLAGVHEISTLIT;
LLAGVHEISTLITE; LAGVHEISTLITEK; AGVHEISTLITEKL;
GVHEISTLITEKLS; VHEISTLITEKLSK; HEISTLITEKLSKL;
EISTLITEKLSKLK; ISTLITEKLSKLKN; STLITEKLSKLKNS;
TLITEKLSKLKNSG; LITEKLSKLKNSGE; ITEKLSKLKNSGEL;
TEKLSKLKNSGELK; EKLSKLKNSGELKA; KLSKLKNSGELKAK;
LSKLKNSGELKAKI; SKLKNSGELKAKIE; KLKNSGELKAKIED;
LKNSGELKAKIEDA; KNSGELKAKIEDAK; NSGELKAKIEDAKK;
SGELKAKIEDAKKC; GELKAKIEDAKKCS; ELKAKIEDAKKCSE;
LKAKIEDAKKCSEE; KAKIEDAKKCSEEF; AKIEDAKKCSEEFT;
KIEDAKKCSEEFTN; IEDAKKCSEEFTNK; EDAKKCSEEFTNKL;
DAKKCSEEFTNKLR; AKKCSEEFTNKLRV; KKCSEEFTNKLRVS;
KCSEEFTNKLRVSH; CSEEFTNKLRVSHA; SEEFTNKLRVSHAD;
EEFTNKLRVSHADL; EFTNKLRVSHADLG; FTNKLRVSHADLGK;
TNKLRVSHADLGKQ; NKLRVSHADLGKQG; KLRVSHADLGKQGV;
LRVSHADLGKQGVN; RVSHADLGKQGVND; VSHADLGKQGVNDD;
SHADLGKQGVNDDD; HADLGKQGVNDDDA; ADLGKQGVNDDDAK;
DLGKQGVNDDDAKK; LGKQGVNDDDAKKA; GKQGVNDDDAKKAI;
KQGVNDDDAKKAIL; QGVNDDDAKKAILK; GVNDDDAKKAILKT;
VNDDDAKKAILKTN; NDDDAKKAILKTNA; DDDAKKAILKTNAD;
DDAKKAILKTNADK; DAKKAILKTNADKT; AKKAILKTNADKTK;
KKAILKTNADKTKG; KAILKTNADKTKGA; AILKTNADKTKGAE;
ILKTNADKTKGAEE; LKTNADKTKGAEEL; KTNADKTKGAEELG;
TNADKTKGAEELGK; NADKTKGAEELGKL; ADKTKGAEELGKLF;
DKTKGAEELGKLFK; KTKGAEELGKLFKS; TKGAEELGKLFKSV;
KGAEELGKLFKSVE; GAEELGKLFKSVEG; AEELGKLFKSVEGL;
EELGKLFKSVEGLV; ELGKLFKSVEGLVK; LGKLFKSVEGLVKA;
GKLFKSVEGLVKAA; KLFKSVEGLVKAAQ; LFKSVEGLVKAAQE;
FKSVEGLVKAAQEA; KSVEGLVKAAQEAL; SVEGLVKAAQEALT;

VEGLVKAAQEALTN; EGLVKAAQEALTNS; GLVKAAQEALTNSV;
LVKAAQEALTNSVK; VKAAQEALTNSVKE; KAAQEALTNSVKEL;
AAQEALTNSVKELT; AQEALTNSVKELTS; QEALTNSVKELTSP;
EALTNSVKELTSPV; ALTNSVKELTSPVV; LTNSVKELTSPVVA;
TNSVKELTSPVVAE; NSVKELTSPVVAES; SVKELTSPVVAESP;
VKELTSPVVAESPK; KELTSPVVAESPKK; ELTSPVVAESPKKP;

15 mers:
MKKNTLSAILMTLFL; KKNTLSAILMTLFLF; KNTLSAILMTLFLFI;
NTLSAILMTLFLFIS; TLSAILMTLFLFISC; LSAILMTLFLFISCN;
SAILMTLFLFISCNN; AILMTLFLFISCNNS; ILMTLFLFISCNNSG;
LMTLFLFISCNNSGK; MTLFLFISCNNSGKG; TLFLFISCNNSGKGG;
LFLFISCNNSGKGGD; FLFISCNNSGKGGDS; LFISCNNSGKGGDSA;
FISCNNSGKGGDSAS; ISCNNSGKGGDSAST; SCNNSGKGGDSASTN;
CNNSGKGGDSASTNP; NNSGKGGDSASTNPA; NSGKGGDSASTNPAD;
SGKGGDSASTNPADE; GKGGDSASTNPADES; KGGDSASTNPADESA;
GGDSASTNPADESAK; GDSASTNPADESAKG; DSASTNPADESAKGP;
SASTNPADESAKGPN; ASTNPADESAKGPNL; STNPADESAKGPNLT;
TNPADESAKGPNLTE; NPADESAKGPNLTEI; PADESAKGPNLTEIS;
ADESAKGPNLTEISK; DESAKGPNLTEISKK; ESAKGPNLTEISKKI;
SAKGPNLTEISKKIT; AKGPNLTEISKKITD; KGPNLTEISKKITDS;
GPNLTEISKKITDSN; PNLTEISKKITDSNA; NLTEISKKITDSNAF;
LTEISKKITDSNAFV; TEISKKITDSNAFVL; EISKKITDSNAFVLA;
ISKKITDSNAFVLAV; SKKITDSNAFVLAVK; KKITDSNAFVLAVKE;
KITDSNAFVLAVKEV; ITDSNAFVLAVKEVE; TDSNAFVLAVKEVET;
DSNAFVLAVKEVETL; SNAFVLAVKEVETLV; NAFVLAVKEVETLVS;
AFVLAVKEVETLVSS; FVLAVKEVETLVSSI; VLAVKEVETLVSSID;
LAVKEVETLVSSIDE; AVKEVETLVSSIDEL; VKEVETLVSSIDELA;
KEVETLVSSIDELAN; EVETLVSSIDELANK; VETLVSSIDELANKA;
ETLVSSIDELANKAI; TLVSSIDELANKAIG; LVSSIDELANKAIGK;
VSSIDELANKAIGKK; SSIDELANKAIGKKI; SIDELANKAIGKKIQ;
IDELANKAIGKKIQQ; DELANKAIGKKIQQN; ELANKAIGKKIQQNG;
LANKAIGKKIQQNGL; ANKAIGKKIQQNGLG; NKAIGKKIQQNGLGA;
KAIGKKIQQNGLGAE; AIGKKIQQNGLGAEA; IGKKIQQNGLGAEAN;
GKKIQQNGLGAEANR; KKIQQNGLGAEANRN; KIQQNGLGAEANRNE;
IQQNGLGAEANRNES; QQNGLGAEANRNESL; QNGLGAEANRNESLL;
NGLGAEANRNESLLA; GLGAEANRNESLLAG; LGAEANRNESLLAGV;
GAEANRNESLLAGVH; AEANRNESLLAGVHE; EANRNESLLAGVHEI;
ANRNESLLAGVHEIS; NRNESLLAGVHEIST; RNESLLAGVHEISTL;
NESLLAGVHEISTLI; ESLLAGVHEISTLIT; SLLAGVHEISTLITE;
LLAGVHEISTLITEK; LAGVHEISTLITEKL; AGVHEISTLITEKLS;
GVHEISTLITEKLSK; VHEISTLITEKLSKL; HEISTLITEKLSKLK;
EISTLITEKLSKLKN; ISTLITEKLSKLKNS; STLITEKLSKLKNSG;
TLITEKLSKLKNSGE; LITEKLSKLKNSGEL; ITEKLSKLKNSGELK;
TEKLSKLKNSGELKA; EKLSKLKNSGELKAK; KLSKLKNSGELKAKI;
LSKLKNSGELKAKIE; SKLKNSGELKAKIED; KLKNSGELKAKIEDA;
LKNSGELKAKIEDAK; KNSGELKAKIEDAKK; NSGELKAKIEDAKKC;
SGELKAKIEDAKKCS; GELKAKIEDAKKCSE; ELKAKIEDAKKCSEE;
LKAKIEDAKKCSEEF; KAKIEDAKKCSEEFT; AKIEDAKKCSEEFTN;
KIEDAKKCSEEFTNK; IEDAKKCSEEFTNKL; EDAKKCSEEFTNKLR;
DAKKCSEEFTNKLRV; AKKCSEEFTNKLRVS; KKCSEEFTNKLRVSH;
KCSEEFTNKLRVSHA; CSEEFTNKLRVSHAD; SEEFTNKLRVSHADL;
EEFTNKLRVSHADLG; EFTNKLRVSHADLGK; FTNKLRVSHADLGKQ;
TNKLRVSHADLGKQG; NKLRVSHADLGKQGV; KLRVSHADLGKQGVN;
LRVSHADLGKQGVND; RVSHADLGKQGVNDD; VSHADLGKQGVNDDD;
SHADLGKQGVNDDDA; HADLGKQGVNDDDAK; ADLGKQGVNDDDAKK;
DLGKQGVNDDDAKKA; LGKQGVNDDDAKKAI; GKQGVNDDDAKKAIL;

KQGVNDDDAKKAILK; QGVNDDDAKKAILKT; GVNDDDAKKAILKTN;
VNDDDAKKAILKTNA; NDDDAKKAILKTNAD; DDDAKKAILKTNADK;
DDAKKAILKTNADKT; DAKKAILKTNADKTK; AKKAILKTNADKTKG;
KKAILKTNADKTKGA; KAILKTNADKTKGAE; AILKTNADKTKGAEE;
ILKTNADKTKGAEEL; LKTNADKTKGAEELG; KTNADKTKGAEELGK;
TNADKTKGAEELGKL; NADKTKGAEELGKLF; ADKTKGAEELGKLFK;
DKTKGAEELGKLFKS; KTKGAEELGKLFKSV; TKGAEELGKLFKSVE;
KGAEELGKLFKSVEG; GAEELGKLFKSVEGL; AEELGKLFKSVEGLV;
EELGKLFKSVEGLVK; ELGKLFKSVEGLVKA; LGKLFKSVEGLVKAA;
GKLFKSVEGLVKAAQ; KLFKSVEGLVKAAQE; LFKSVEGLVKAAQEA;
FKSVEGLVKAAQEAL; KSVEGLVKAAQEALT; SVEGLVKAAQEALTN;
VEGLVKAAQEALTNS; EGLVKAAQEALTNSV; GLVKAAQEALTNSVK;
LVKAAQEALTNSVKE; VKAAQEALTNSVKEL; KAAQEALTNSVKELT;
AAQEALTNSVKELTS; AQEALTNSVKELTSP; QEALTNSVKELTSPV;
EALTNSVKELTSPVV; ALTNSVKELTSPVVA; LTNSVKELTSPVVAE;
TNSVKELTSPVVAES; NSVKELTSPVVAESP; SVKELTSPVVAESPK;
VKELTSPVVAESPKK; KELTSPVVAESPKKP;

16 mers:
MKKNTLSAILMTLFLF; KKNTLSAILMTLFLFI; KNTLSAILMTLFLFIS;
NTLSAILMTLFLFISC; TLSAILMTLFLFISCN; LSAILMTLFLFISCNN;
SAILMTLFLFISCNNS; AILMTLFLFISCNNSG; ILMTLFLFISCNNSGK;
LMTLFLFISCNNSGKG; MTLFLFISCNNSGKGG; TLFLFISCNNSGKGGD;
LFLFISCNNSGKGGDS; FLFISCNNSGKGGDSA; LFISCNNSGKGGDSAS;
FISCNNSGKGGDSAST; ISCNNSGKGGDSASTN; SCNNSGKGGDSASTNP;
CNNSGKGGDSASTNPA; NNSGKGGDSASTNPAD; NSGKGGDSASTNPADE;
SGKGGDSASTNPADES; GKGGDSASTNPADESA; KGGDSASTNPADESAK;
GGDSASTNPADESAKG; GDSASTNPADESAKGP; DSASTNPADESAKGPN;
SASTNPADESAKGPNL; ASTNPADESAKGPNLT; STNPADESAKGPNLTE;
TNPADESAKGPNLTEI; NPADESAKGPNLTEIS; PADESAKGPNLTEISK;
ADESAKGPNLTEISKK; DESAKGPNLTEISKKI; ESAKGPNLTEISKKIT;
SAKGPNLTEISKKITD; AKGPNLTEISKKITDS; KGPNLTEISKKITDSN;
GPNLTEISKKITDSNA; PNLTEISKKITDSNAF; NLTEISKKITDSNAFV;
LTEISKKITDSNAFVL; TEISKKITDSNAFVLA; EISKKITDSNAFVLAV;
ISKKITDSNAFVLAVK; SKKITDSNAFVLAVKE; KKITDSNAFVLAVKEV;
KITDSNAFVLAVKEVE; ITDSNAFVLAVKEVET; TDSNAFVLAVKEVETL;
DSNAFVLAVKEVETLV; SNAFVLAVKEVETLVS; NAFVLAVKEVETLVSS;
AFVLAVKEVETLVSSI; FVLAVKEVETLVSSID; VLAVKEVETLVSSIDE;
LAVKEVETLVSSIDEL; AVKEVETLVSSIDELA; VKEVETLVSSIDELAN;
KEVETLVSSIDELANK; EVETLVSSIDELANKA; VETLVSSIDELANKAI;
ETLVSSIDELANKAIG; TLVSSIDELANKAIGK; LVSSIDELANKAIGKK;
VSSIDELANKAIGKKI; SSIDELANKAIGKKIQ; SIDELANKAIGKKIQQ;
IDELANKAIGKKIQQN; DELANKAIGKKIQQNG; ELANKAIGKKIQQNGL;
LANKAIGKKIQQNGLG; ANKAIGKKIQQNGLGA; NKAIGKKIQQNGLGAE;
KAIGKKIQQNGLGAEA; AIGKKIQQNGLGAEAN; IGKKIQQNGLGAEANR;
GKKIQQNGLGAEANRN; KKIQQNGLGAEANRNE; KIQQNGLGAEANRNES;
IQQNGLGAEANRNESL; QQNGLGAEANRNESLL; QNGLGAEANRNESLLA;
NGLGAEANRNESLLAG; GLGAEANRNESLLAGV; LGAEANRNESLLAGVH;
GAEANRNESLLAGVHE; AEANRNESLLAGVHEI; EANRNESLLAGVHEIS;
ANRNESLLAGVHEIST; NRNESLLAGVHEISTL; RNESLLAGVHEISTLI;
NESLLAGVHEISTLIT; ESLLAGVHEISTLITE; SLLAGVHEISTLITEK;
LLAGVHEISTLITEKL; LAGVHEISTLITEKLS; AGVHEISTLITEKLSK;
GVHEISTLITEKLSKL; VHEISTLITEKLSKLK; HEISTLITEKLSKLKN;
EISTLITEKLSKLKNS; ISTLITEKLSKLKNSG; STLITEKLSKLKNSGE;
TLITEKLSKLKNSGEL; LITEKLSKLKNSGELK; ITEKLSKLKNSGELKA;
TEKLSKLKNSGELKAK; EKLSKLKNSGELKAKI; KLSKLKNSGELKAKIE;
LSKLKNSGELKAKIED; SKLKNSGELKAKIEDA; KLKNSGELKAKIEDAK;

| | |
|---|---|
| | LKNSGELKAKIEDAKK; KNSGELKAKIEDAKKC; NSGELKAKIEDAKKCS; SGELKAKIEDAKKCSE; GELKAKIEDAKKCSEE; ELKAKIEDAKKCSEEF; LKAKIEDAKKCSEEFT; KAKIEDAKKCSEEFTN; AKIEDAKKCSEEFTNK; KIEDAKKCSEEFTNKL; IEDAKKCSEEFTNKLR; EDAKKCSEEFTNKLRV; DAKKCSEEFTNKLRVS; AKKCSEEFTNKLRVSH; KKCSEEFTNKLRVSHA; KCSEEFTNKLRVSHAD; CSEEFTNKLRVSHADL; SEEFTNKLRVSHADLG; EEFTNKLRVSHADLGK; EFTNKLRVSHADLGKQ; FTNKLRVSHADLGKQG; TNKLRVSHADLGKQGV; NKLRVSHADLGKQGVN; KLRVSHADLGKQGVND; LRVSHADLGKQGVNDD; RVSHADLGKQGVNDDD; VSHADLGKQGVNDDDA; SHADLGKQGVNDDDAK; HADLGKQGVNDDDAKK; ADLGKQGVNDDDAKKA; DLGKQGVNDDDAKKAI; LGKQGVNDDDAKKAIL; GKQGVNDDDAKKAILK; KQGVNDDDAKKAILKT; QGVNDDDAKKAILKTN; GVNDDDAKKAILKTNA; VNDDDAKKAILKTNAD; NDDDAKKAILKTNADK; DDDAKKAILKTNADKT; DDAKKAILKTNADKTK; DAKKAILKTNADKTKG; AKKAILKTNADKTKGA; KKAILKTNADKTKGAE; KAILKTNADKTKGAEE; AILKTNADKTKGAEEL; ILKTNADKTKGAEELG; LKTNADKTKGAEELGK; KTNADKTKGAEELGKL; TNADKTKGAEELGKLF; NADKTKGAEELGKLFK; ADKTKGAEELGKLFKS; DKTKGAEELGKLFKSV; KTKGAEELGKLFKSVE; TKGAEELGKLFKSVEG; KGAEELGKLFKSVEGL; GAEELGKLFKSVEGLV; AEELGKLFKSVEGLVK; EELGKLFKSVEGLVKA; ELGKLFKSVEGLVKAA; LGKLFKSVEGLVKAAQ; GKLFKSVEGLVKAAQE; KLFKSVEGLVKAAQEA; LFKSVEGLVKAAQEAL; FKSVEGLVKAAQEALT; KSVEGLVKAAQEALTN; SVEGLVKAAQEALTNS; VEGLVKAAQEALTNSV; EGLVKAAQEALTNSVK; GLVKAAQEALTNSVKE; LVKAAQEALTNSVKEL; VKAAQEALTNSVKELT; KAAQEALTNSVKELTS; AAQEALTNSVKELTSP; AQEALTNSVKELTSPV; QEALTNSVKELTSPVV; EALTNSVKELTSPVVA; ALTNSVKELTSPVVAE; LTNSVKELTSPVVAES; TNSVKELTSPVVAESP; NSVKELTSPVVAESPK; SVKELTSPVVAESPKK; VKELTSPVVAESPKKP; |
| AAC62927.1\| OspE-related lipoprotein [Borrelia garinii] | 13 mers: MNKKMKMFIICAV; NKKMKMFIICAVF; KKMKMFIICAVFV; KMKMFIICAVFVL; MKMFIICAVFVLI; KMFIICAVFVLIS; MFIICAVFVLISS; FIICAVFVLISSC; IICAVFVLISSCG; ICAVFVLISSCGN; CAVFVLISSCGNF; AVFVLISSCGNFR; VFVLISSCGNFRS; FVLISSCGNFRSS; VLISSCGNFRSSL; LISSCGNFRSSLS; ISSCGNFRSSLSD; SSCGNFRSSLSDQ; SCGNFRSSLSDQG; CGNFRSSLSDQGS; GNFRSSLSDQGSL; NFRSSLSDQGSLS; FRSSLSDQGSLSD; RSSLSDQGSLSDQ; SSLSDQGSLSDQG; SLSDQGSLSDQGS; LSDQGSLSDQGSL; SDQGSLSDQGSLS; DQGSLSDQGSLSD; QGSLSDQGSLSDQ; GSLSDQGSLSDQG; SLSDQGSLSDQGS; LSDQGSLSDQGSL; SDQGSLSDQGSLS; DQGSLSDQGSLSD; QGSLSDQGSLSDQ; GSLSDQGSLSDQG; SLSDQGSLSDQGG; LSDQGSLSDQGGL; SDQGSLSDQGGLS; DQGSLSDQGGLSG; QGSLSDQGGLSGQ; GSLSDQGGLSGQA; SLSDQGGLSGQAS; LSDQGGLSGQASS; SDQGGLSGQASSD; DQGGLSGQASSDT; QGGLSGQASSDTI; GGLSGQASSDTIK; GLSGQASSDTIKF; LSGQASSDTIKFS; SGQASSDTIKFSE; GQASSDTIKFSEF; QASSDTIKFSEFT; ASSDTIKFSEFTV; SSDTIKFSEFTVN; SDTIKFSEFTVNI; DTIKFSEFTVNIK; TIKFSEFTVNIKN; IKFSEFTVNIKNK; KFSEFTVNIKNKK; FSEFTVNIKNKKD; SEFTVNIKNKKDN; EFTVNIKNKKDNN; FTVNIKNKKDNNG; TVNIKNKKDNNGD; VNIKNKKDNNGDW; NIKNKKDNNGDWS; IKNKKDNNGDWSN; KNKKDNNGDWSNL; NKKDNNGDWSNLG; KKDNNGDWSNLGT; KDNNGDWSNLGTL; DNNGDWSNLGTLV; NNGDWSNLGTLVI; NGDWSNLGTLVIR; GDWSNLGTLVIRK; DWSNLGTLVIRKE; WSNLGTLVIRKEQ; SNLGTLVIRKEQD; NLGTLVIRKEQDG; LGTLVIRKEQDGV; GTLVIRKEQDGVE; TLVIRKEQDGVET; LVIRKEQDGVETG; VIRKEQDGVETGL; IRKEQDGVETGLN; RKEQDGVETGLNV; KEQDGVETGLNVI; EQDGVETGLNVIG; QDGVETGLNVIGT; DGVETGLNVIGTI; GVETGLNVIGTIN; VETGLNVIGTING; ETGLNVIGTINGQ; TGLNVIGTINGQL; GLNVIGTINGQLR; LNVIGTINGQLRG; NVIGTINGQLRGH; VIGTINGQLRGHS; IGTINGQLRGHSA; GTINGQLRGHSAT; TINGQLRGHSATF; INGQLRGHSATFF; |

NGQLRGHSATFFC; GQLRGHSATFFCI; QLRGHSATFFCIE; LRGHSATFFCIEE;
RGHSATFFCIEEA; GHSATFFCIEEAE; HSATFFCIEEAEV; SATFFCIEEAEVN;
ATFFCIEEAEVNN; TFFCIEEAEVNNF; FFCIEEAEVNNFV; FCIEEAEVNNFVK;
CIEEAEVNNFVKA; IEEAEVNNFVKAM; EEAEVNNFVKAMT; EAEVNNFVKAMTN;
AEVNNFVKAMTNV; EVNNFVKAMTNVG; VNNFVKAMTNVGS; NNFVKAMTNVGSF;
NFVKAMTNVGSFK; FVKAMTNVGSFKT; VKAMTNVGSFKTS; KAMTNVGSFKTSL;
AMTNVGSFKTSLY; MTNVGSFKTSLYY; TNVGSFKTSLYYG; NVGSFKTSLYYGY;
VGSFKTSLYYGYK; GSFKTSLYYGYKE; SFKTSLYYGYKEE; FKTSLYYGYKEEQ;
KTSLYYGYKEEQS; TSLYYGYKEEQSS; SLYYGYKEEQSST; LYYGYKEEQSSTN;
YYGYKEEQSSTNG; YGYKEEQSSTNGI; GYKEEQSSTNGIK; YKEEQSSTNGIKG;
KEEQSSTNGIKGK; EEQSSTNGIKGKE; EQSSTNGIKGKEI; QSSTNGIKGKEIT;
SSTNGIKGKEITT; STNGIKGKEITTK; TNGIKGKEITTKI; NGIKGKEITTKIE;
GIKGKEITTKIET; IKGKEITTKIETI; KGKEITTKIETIN; GKEITTKIETINN;
KEITTKIETINNS; EITTKIETINNSE; ITTKIETINNSEH; TTKIETINNSEHI;
TKIETINNSEHIT; KIETINNSEHITF; IETINNSEHITFS; ETINNSEHITFSG;
TINNSEHITFSGD; INNSEHITFSGDK; NNSEHITFSGDKI;

14 mers:
MNKKMKMFIICAVF; NKKMKMFIICAVFV; KKMKMFIICAVFVL;
KMKMFIICAVFVLI; MKMFIICAVFVLIS; KMFIICAVFVLISS;
MFIICAVFVLISSC; FIICAVFVLISSCG; IICAVFVLISSCGN;
ICAVFVLISSCGNF; CAVFVLISSCGNFR; AVFVLISSCGNFRS;
VFVLISSCGNFRSS; FVLISSCGNFRSSL; VLISSCGNFRSSLS;
LISSCGNFRSSLSD; ISSCGNFRSSLSDQ; SSCGNFRSSLSDQG;
SCGNFRSSLSDQGS; CGNFRSSLSDQGSL; GNFRSSLSDQGSLS;
NFRSSLSDQGSLSD; FRSSLSDQGSLSDQ; RSSLSDQGSLSDQG;
SSLSDQGSLSDQGS; SLSDQGSLSDQGSL; LSDQGSLSDQGSLS;
SDQGSLSDQGSLSD; DQGSLSDQGSLSDQ; QGSLSDQGSLSDQG;
GSLSDQGSLSDQGS; SLSDQGSLSDQGSL; LSDQGSLSDQGSLS;
SDQGSLSDQGSLSD; DQGSLSDQGSLSDQ; QGSLSDQGSLSDQG;
GSLSDQGSLSDQGG; SLSDQGSLSDQGGL; LSDQGSLSDQGGLS;
SDQGSLSDQGGLSG; DQGSLSDQGGLSGQ; QGSLSDQGGLSGQA;
GSLSDQGGLSGQAS; SLSDQGGLSGQASS; LSDQGGLSGQASSD;
SDQGGLSGQASSDT; DQGGLSGQASSDTI; QGGLSGQASSDTIK;
GGLSGQASSDTIKF; GLSGQASSDTIKFS; LSGQASSDTIKFSE;
SGQASSDTIKFSEF; GQASSDTIKFSEFT; QASSDTIKFSEFTV;
ASSDTIKFSEFTVN; SSDTIKFSEFTVNI; SDTIKFSEFTVNIK;
DTIKFSEFTVNIKN; TIKFSEFTVNIKNK; IKFSEFTVNIKNKK;
KFSEFTVNIKNKKD; FSEFTVNIKNKKDN; SEFTVNIKNKKDNN;
EFTVNIKNKKDNNG; FTVNIKNKKDNNGD; TVNIKNKKDNNGDW;
VNIKNKKDNNGDWS; NIKNKKDNNGDWSN; IKNKKDNNGDWSNL;
KNKKDNNGDWSNLG; NKKDNNGDWSNLGT; KKDNNGDWSNLGTL;
KDNNGDWSNLGTLV; DNNGDWSNLGTLVI; NNGDWSNLGTLVIR;
NGDWSNLGTLVIRK; GDWSNLGTLVIRKE; DWSNLGTLVIRKEQ;
WSNLGTLVIRKEQD; SNLGTLVIRKEQDG; NLGTLVIRKEQDGV;
LGTLVIRKEQDGVE; GTLVIRKEQDGVET; TLVIRKEQDGVETG;
LVIRKEQDGVETGL; VIRKEQDGVETGLN; IRKEQDGVETGLNV;
RKEQDGVETGLNVI; KEQDGVETGLNVIG; EQDGVETGLNVIGT;
QDGVETGLNVIGTI; DGVETGLNVIGTIN; GVETGLNVIGTING;
VETGLNVIGTINGQ; ETGLNVIGTINGQL; TGLNVIGTINGQLR;
GLNVIGTINGQLRG; LNVIGTINGQLRGH; NVIGTINGQLRGHS;
VIGTINGQLRGHSA; IGTINGQLRGHSAT; GTINGQLRGHSATF;
TINGQLRGHSATFF; INGQLRGHSATFFC; NGQLRGHSATFFCI;
GQLRGHSATFFCIE; QLRGHSATFFCIEE; LRGHSATFFCIEEA;
RGHSATFFCIEEAE; GHSATFFCIEEAEV; HSATFFCIEEAEVN;
SATFFCIEEAEVNN; ATFFCIEEAEVNNF; TFFCIEEAEVNNFV;
FFCIEEAEVNNFVK; FCIEEAEVNNFVKA; CIEEAEVNNFVKAM;

IEEAEVNNFVKAMT; EEAEVNNFVKAMTN; EAEVNNFVKAMTNV;
AEVNNFVKAMTNVG; EVNNFVKAMTNVGS; VNNFVKAMTNVGSF;
NNFVKAMTNVGSFK; NFVKAMTNVGSFKT; FVKAMTNVGSFKTS;
VKAMTNVGSFKTSL; KAMTNVGSFKTSLY; AMTNVGSFKTSLYY;
MTNVGSFKTSLYYG; TNVGSFKTSLYYGY; NVGSFKTSLYYGYK;
VGSFKTSLYYGYKE; GSFKTSLYYGYKEE; SFKTSLYYGYKEEQ;
FKTSLYYGYKEEQS; KTSLYYGYKEEQSS; TSLYYGYKEEQSST;
SLYYGYKEEQSSTN; LYYGYKEEQSSTNG; YYGYKEEQSSTNGI;
YGYKEEQSSTNGIK; GYKEEQSSTNGIKG; YKEEQSSTNGIKGK;
KEEQSSTNGIKGKE; EEQSSTNGIKGKEI; EQSSTNGIKGKEIT;
QSSTNGIKGKEITT; SSTNGIKGKEITTK; STNGIKGKEITTKI;
TNGIKGKEITTKIE; NGIKGKEITTKIET; GIKGKEITTKIETI;
IKGKEITTKIETIN; KGKEITTKIETINN; GKEITTKIETINNS;
KEITTKIETINNSE; EITTKIETINNSEH; ITTKIETINNSEHI;
TTKIETINNSEHIT; TKIETINNSEHITF; KIETINNSEHITFS;
IETINNSEHITFSG; ETINNSEHITFSGD; TINNSEHITFSGDK;
INNSEHITFSGDKI;

15 mers:
MNKKMKMFIICAVFV; NKKMKMFIICAVFVL; KKMKMFIICAVFVLI;
KMKMFIICAVFVLIS; MKMFIICAVFVLISS; KMFIICAVFVLISSC;
MFIICAVFVLISSCG; FIICAVFVLISSCGN; IICAVFVLISSCGNF;
ICAVFVLISSCGNFR; CAVFVLISSCGNFRS; AVFVLISSCGNFRSS;
VFVLISSCGNFRSSL; FVLISSCGNFRSSLS; VLISSCGNFRSSLSD;
LISSCGNFRSSLSDQ; ISSCGNFRSSLSDQG; SSCGNFRSSLSDQGS;
SCGNFRSSLSDQGSL; CGNFRSSLSDQGSLS; GNFRSSLSDQGSLSD;
NFRSSLSDQGSLSDQ; FRSSLSDQGSLSDQG; RSSLSDQGSLSDQGS;
SSLSDQGSLSDQGSL; SLSDQGSLSDQGSLS; LSDQGSLSDQGSLSD;
SDQGSLSDQGSLSDQ; DQGSLSDQGSLSDQG; QGSLSDQGSLSDQGS;
GSLSDQGSLSDQGSL; SLSDQGSLSDQGSLS; LSDQGSLSDQGSLSD;
SDQGSLSDQGSLSDQ; DQGSLSDQGSLSDQG; QGSLSDQGSLSDQGG;
GSLSDQGSLSDQGGL; SLSDQGSLSDQGGLS; LSDQGSLSDQGGLSG;
SDQGSLSDQGGLSGQ; DQGSLSDQGGLSGQA; QGSLSDQGGLSGQAS;
GSLSDQGGLSGQASS; SLSDQGGLSGQASSD; LSDQGGLSGQASSDT;
SDQGGLSGQASSDTI; DQGGLSGQASSDTIK; QGGLSGQASSDTIKF;
GGLSGQASSDTIKFS; GLSGQASSDTIKFSE; LSGQASSDTIKFSEF;
SGQASSDTIKFSEFT; GQASSDTIKFSEFTV; QASSDTIKFSEFTVN;
ASSDTIKFSEFTVNI; SSDTIKFSEFTVNIK; SDTIKFSEFTVNIKN;
DTIKFSEFTVNIKNK; TIKFSEFTVNIKNKK; IKFSEFTVNIKNKKD;
KFSEFTVNIKNKKDN; FSEFTVNIKNKKDNN; SEFTVNIKNKKDNNG;
EFTVNIKNKKDNNGD; FTVNIKNKKDNNGDW; TVNIKNKKDNNGDWS;
VNIKNKKDNNGDWSN; NIKNKKDNNGDWSNL; IKNKKDNNGDWSNLG;
KNKKDNNGDWSNLGT; NKKDNNGDWSNLGTL; KKDNNGDWSNLGTLV;
KDNNGDWSNLGTLVI; DNNGDWSNLGTLVIR; NNGDWSNLGTLVIRK;
NGDWSNLGTLVIRKE; GDWSNLGTLVIRKEQ; DWSNLGTLVIRKEQD;
WSNLGTLVIRKEQDG; SNLGTLVIRKEQDGV; NLGTLVIRKEQDGVE;
LGTLVIRKEQDGVET; GTLVIRKEQDGVETG; TLVIRKEQDGVETGL;
LVIRKEQDGVETGLN; VIRKEQDGVETGLNV; IRKEQDGVETGLNVI;
RKEQDGVETGLNVIG; KEQDGVETGLNVIGT; EQDGVETGLNVIGTI;
QDGVETGLNVIGTIN; DGVETGLNVIGTING; GVETGLNVIGTINGQ;
VETGLNVIGTINGQL; ETGLNVIGTINGQLR; TGLNVIGTINGQLRG;
GLNVIGTINGQLRGH; LNVIGTINGQLRGHS; NVIGTINGQLRGHSA;
VIGTINGQLRGHSAT; IGTINGQLRGHSATF; GTINGQLRGHSATFF;
TINGQLRGHSATFFC; INGQLRGHSATFFCI; NGQLRGHSATFFCIE;
GQLRGHSATFFCIEE; QLRGHSATFFCIEEA; LRGHSATFFCIEEAE;
RGHSATFFCIEEAEV; GHSATFFCIEEAEVN; HSATFFCIEEAEVNN;
SATFFCIEEAEVNNF; ATFFCIEEAEVNNFV; TFFCIEEAEVNNFVK;

FFCIEEAEVNNFVKA; FCIEEAEVNNFVKAM; CIEEAEVNNFVKAMT;
IEEAEVNNFVKAMTN; EEAEVNNFVKAMTNV; EAEVNNFVKAMTNVG;
AEVNNFVKAMTNVGS; EVNNFVKAMTNVGSF; VNNFVKAMTNVGSFK;
NNFVKAMTNVGSFKT; NFVKAMTNVGSFKTS; FVKAMTNVGSFKTSL;
VKAMTNVGSFKTSLY; KAMTNVGSFKTSLYY; AMTNVGSFKTSLYYG;
MTNVGSFKTSLYYGY; TNVGSFKTSLYYGYK; NVGSFKTSLYYGYKE;
VGSFKTSLYYGYKEE; GSFKTSLYYGYKEEQ; SFKTSLYYGYKEEQS;
FKTSLYYGYKEEQSS; KTSLYYGYKEEQSST; TSLYYGYKEEQSSTN;
SLYYGYKEEQSSTNG; LYYGYKEEQSSTNGI; YYGYKEEQSSTNGIK;
YGYKEEQSSTNGIKG; GYKEEQSSTNGIKGK; YKEEQSSTNGIKGKE;
KEEQSSTNGIKGKEI; EEQSSTNGIKGKEIT; EQSSTNGIKGKEITT;
QSSTNGIKGKEITTK; SSTNGIKGKEITTKI; STNGIKGKEITTKIE;
TNGIKGKEITTKIET; NGIKGKEITTKIETI; GIKGKEITTKIETIN;
IKGKEITTKIETINN; KGKEITTKIETINNS; GKEITTKIETINNSE;
KEITTKIETINNSEH; EITTKIETINNSEHI; ITTKIETINNSEHIT;
TTKIETINNSEHITF; TKIETINNSEHITFS; KIETINNSEHITFSG;
IETINNSEHITFSGD; ETINNSEHITFSGDK; TINNSEHITFSGDKI;

16 mers:
MNKKMKMFIICAVFVL; NKKMKMFIICAVFVLI; KKMKMFIICAVFVLIS;
KMKMFIICAVFVLISS; MKMFIICAVFVLISSC; KMFIICAVFVLISSCG;
MFIICAVFVLISSCGN; FIICAVFVLISSCGNF; IICAVFVLISSCGNFR;
ICAVFVLISSCGNFRS; CAVFVLISSCGNFRSS; AVFVLISSCGNFRSSL;
VFVLISSCGNFRSSLS; FVLISSCGNFRSSLSD; VLISSCGNFRSSLSDQ;
LISSCGNFRSSLSDQG; ISSCGNFRSSLSDQGS; SSCGNFRSSLSDQGSL;
SCGNFRSSLSDQGSLS; CGNFRSSLSDQGSLSD; GNFRSSLSDQGSLSDQ;
NFRSSLSDQGSLSDQG; FRSSLSDQGSLSDQGS; RSSLSDQGSLSDQGSL;
SSLSDQGSLSDQGSLS; SLSDQGSLSDQGSLSD; LSDQGSLSDQGSLSDQ;
SDQGSLSDQGSLSDQG; DQGSLSDQGSLSDQGS; QGSLSDQGSLSDQGSL;
GSLSDQGSLSDQGSLS; SLSDQGSLSDQGSLSD; LSDQGSLSDQGSLSDQ;
SDQGSLSDQGSLSDQG; DQGSLSDQGSLSDQGG; QGSLSDQGSLSDQGGL;
GSLSDQGSLSDQGGLS; SLSDQGSLSDQGGLSG; LSDQGSLSDQGGLSGQ;
SDQGSLSDQGGLSGQA; DQGSLSDQGGLSGQAS; QGSLSDQGGLSGQASS;
GSLSDQGGLSGQASSD; SLSDQGGLSGQASSDT; LSDQGGLSGQASSDTI;
SDQGGLSGQASSDTIK; DQGGLSGQASSDTIKF; QGGLSGQASSDTIKFS;
GGLSGQASSDTIKFSE; GLSGQASSDTIKFSEF; LSGQASSDTIKFSEFT;
SGQASSDTIKFSEFTV; GQASSDTIKFSEFTVN; QASSDTIKFSEFTVNI;
ASSDTIKFSEFTVNIK; SSDTIKFSEFTVNIKN; SDTIKFSEFTVNIKNK;
DTIKFSEFTVNIKNKK; TIKFSEFTVNIKNKKD; IKFSEFTVNIKNKKDN;
KFSEFTVNIKNKKDNN; FSEFTVNIKNKKDNNG; SEFTVNIKNKKDNNGD;
EFTVNIKNKKDNNGDW; FTVNIKNKKDNNGDWS; TVNIKNKKDNNGDWSN;
VNIKNKKDNNGDWSNL; NIKNKKDNNGDWSNLG; IKNKKDNNGDWSNLGT;
KNKKDNNGDWSNLGTL; NKKDNNGDWSNLGTLV; KKDNNGDWSNLGTLVI;
KDNNGDWSNLGTLVIR; DNNGDWSNLGTLVIRK; NNGDWSNLGTLVIRKE;
NGDWSNLGTLVIRKEQ; GDWSNLGTLVIRKEQD; DWSNLGTLVIRKEQDG;
WSNLGTLVIRKEQDGV; SNLGTLVIRKEQDGVE; NLGTLVIRKEQDGVET;
LGTLVIRKEQDGVETG; GTLVIRKEQDGVETGL; TLVIRKEQDGVETGLN;
LVIRKEQDGVETGLNV; VIRKEQDGVETGLNVI; IRKEQDGVETGLNVIG;
RKEQDGVETGLNVIGT; KEQDGVETGLNVIGTI; EQDGVETGLNVIGTIN;
QDGVETGLNVIGTING; DGVETGLNVIGTINGQ; GVETGLNVIGTINGQL;
VETGLNVIGTINGQLR; ETGLNVIGTINGQLRG; TGLNVIGTINGQLRGH;
GLNVIGTINGQLRGHS; LNVIGTINGQLRGHSA; NVIGTINGQLRGHSAT;
VIGTINGQLRGHSATF; IGTINGQLRGHSATFF; GTINGQLRGHSATFFC;
TINGQLRGHSATFFCI; INGQLRGHSATFFCIE; NGQLRGHSATFFCIEE;
GQLRGHSATFFCIEEA; QLRGHSATFFCIEEAE; LRGHSATFFCIEEAEV;
RGHSATFFCIEEAEVN; GHSATFFCIEEAEVNN; HSATFFCIEEAEVNNF;
SATFFCIEEAEVNNFV; ATFFCIEEAEVNNFVK; TFFCIEEAEVNNFVKA;

| | |
|---|---|
| | FFCIEEAEVNNFVKAM; FCIEEAEVNNFVKAMT; CIEEAEVNNFVKAMTN;<br>IEEAEVNNFVKAMTNV; EEAEVNNFVKAMTNVG; EAEVNNFVKAMTNVGS;<br>AEVNNFVKAMTNVGSF; EVNNFVKAMTNVGSFK; VNNFVKAMTNVGSFKT;<br>NNFVKAMTNVGSFKTS; NFVKAMTNVGSFKTSL; FVKAMTNVGSFKTSLY;<br>VKAMTNVGSFKTSLYY; KAMTNVGSFKTSLYYG; AMTNVGSFKTSLYYGY;<br>MTNVGSFKTSLYYGYK; TNVGSFKTSLYYGYKE; NVGSFKTSLYYGYKEE;<br>VGSFKTSLYYGYKEEQ; GSFKTSLYYGYKEEQS; SFKTSLYYGYKEEQSS;<br>FKTSLYYGYKEEQSST; KTSLYYGYKEEQSSTN; TSLYYGYKEEQSSTNG;<br>SLYYGYKEEQSSTNGI; LYYGYKEEQSSTNGIK; YYGYKEEQSSTNGIKG;<br>YGYKEEQSSTNGIKGK; GYKEEQSSTNGIKGKE; YKEEQSSTNGIKGKEI;<br>KEEQSSTNGIKGKEIT; EEQSSTNGIKGKEITT; EQSSTNGIKGKEITTK;<br>QSSTNGIKGKEITTKI; SSTNGIKGKEITTKIE; STNGIKGKEITTKIET;<br>TNGIKGKEITTKIETI; NGIKGKEITTKIETIN; GIKGKEITTKIETINN;<br>IKGKEITTKIETINNS; KGKEITTKIETINNSE; GKEITTKIETINNSEH;<br>KEITTKIETINNSEHI; EITTKIETINNSEHIT; ITTKIETINNSEHITF;<br>TTKIETINNSEHITFS; TKIETINNSEHITFSG; KIETINNSEHITFSGD;<br>IETINNSEHITFSGDK; ETINNSEHITFSGDKI; |
| CAA57806.1\| Outer surface protein G [Borrelia burgdorferi] | 13 mers:<br>MNKKMKNLIICAV; NKKMKNLIICAVF; KKMKNLIICAVFV; KMKNLIICAVFVL;<br>MKNLIICAVFVLI; KNLIICAVFVLII; NLIICAVFVLIIS; LIICAVFVLIISC;<br>IICAVFVLIISCK; ICAVFVLIISCKI; CAVFVLIISCKID; AVFVLIISCKIDA;<br>VFVLIISCKIDAS; FVLIISCKIDASS; VLIISCKIDASSE; LIISCKIDASSED;<br>IISCKIDASSEDL; ISCKIDASSEDLK; SCKIDASSEDLKQ; CKIDASSEDLKQN;<br>KIDASSEDLKQNV; IDASSEDLKQNVK; DASSEDLKQNVKE; ASSEDLKQNVKEK;<br>SSEDLKQNVKEKV; SEDLKQNVKEKVE; EDLKQNVKEKVEG; DLKQNVKEKVEGF;<br>LKQNVKEKVEGFL; KQNVKEKVEGFLD; QNVKEKVEGFLDK; NVKEKVEGFLDKE;<br>VKEKVEGFLDKEL; KEKVEGFLDKELM; EKVEGFLDKELMQ; KVEGFLDKELMQG;<br>VEGFLDKELMQGD; EGFLDKELMQGDD; GFLDKELMQGDDP; FLDKELMQGDDPN;<br>LDKELMQGDDPNN; DKELMQGDDPNNS; KELMQGDDPNNSL; ELMQGDDPNNSLF;<br>LMQGDDPNNSLFN; MQGDDPNNSLFNP; QGDDPNNSLFNPP; GDDPNNSLFNPPP;<br>DDPNNSLFNPPPV; DPNNSLFNPPPVL; PNNSLFNPPPVLP; NNSLFNPPPVLPA;<br>NSLFNPPPVLPAS; SLFNPPPVLPASS; LFNPPPVLPASSH; FNPPPVLPASSHD;<br>NPPPVLPASSHDN; PPPVLPASSHDNT; PPVLPASSHDNTP; PVLPASSHDNTPV;<br>VLPASSHDNTPVL; LPASSHDNTPVLK; PASSHDNTPVLKA; ASSHDNTPVLKAV;<br>SSHDNTPVLKAVQ; SHDNTPVLKAVQA; HDNTPVLKAVQAK; DNTPVLKAVQAKD;<br>NTPVLKAVQAKDG; TPVLKAVQAKDGG; PVLKAVQAKDGGQ; VLKAVQAKDGGQQ;<br>LKAVQAKDGGQQE; KAVQAKDGGQQEG; AVQAKDGGQQEGK; VQAKDGGQQEGKE;<br>QAKDGGQQEGKEE; AKDGGQQEGKEEK; KDGGQQEGKEEKE; DGGQQEGKEEKEK;<br>GGQQEGKEEKEKE; GQQEGKEEKEKEI; QQEGKEEKEKEIQ; QEGKEEKEKEIQE;<br>EGKEEKEKEIQEL; GKEEKEKEIQELK; KEEKEKEIQELKD; EEKEKEIQELKDK;<br>EKEKEIQELKDKI; KEKEIQELKDKID; EKEIQELKDKIDK; KEIQELKDKIDKR;<br>EIQELKDKIDKRK; IQELKDKIDKRKK; QELKDKIDKRKKE; ELKDKIDKRKKEL;<br>LKDKIDKRKKELE; KDKIDKRKKELEE; DKIDKRKKELEEA; KIDKRKKELEEAR;<br>IDKRKKELEEARK; DKRKKELEEARKK; KRKKELEEARKKF; RKKELEEARKKFQ;<br>KKELEEARKKFQE; KELEEARKKFQEF; ELEEARKKFQEFK; LEEARKKFQEFKE;<br>EEARKKFQEFKEQ; EARKKFQEFKEQV; ARKKFQEFKEQVE; RKKFQEFKEQVES;<br>KKFQEFKEQVESA; KFQEFKEQVESAT; FQEFKEQVESATG; QEFKEQVESATGE;<br>EFKEQVESATGES; FKEQVESATGEST; KEQVESATGESTE; EQVESATGESTEK;<br>QVESATGESTEKV; VESATGESTEKVK; ESATGESTEKVKK; SATGESTEKVKKQ;<br>ATGESTEKVKKQG; TGESTEKVKKQGN; GESTEKVKKQGNI; ESTEKVKKQGNIG;<br>STEKVKKQGNIGQ; TEKVKKQGNIGQK; EKVKKQGNIGQKA; KVKKQGNIGQKAL;<br>VKKQGNIGQKALK; KKQGNIGQKALKY; KQGNIGQKALKYA; QGNIGQKALKYAK;<br>GNIGQKALKYAKE; NIGQKALKYAKEL; IGQKALKYAKELG; GQKALKYAKELGV;<br>QKALKYAKELGVN; KALKYAKELGVNG; ALKYAKELGVNGS; LKYAKELGVNGSY;<br>KYAKELGVNGSYS; YAKELGVNGSYSV; AKELGVNGSYSVN; KELGVNGSYSVND; |

ELGVNGSYSVNDG; LGVNGSYSVNDGT; GVNGSYSVNDGTN; VNGSYSVNDGTNT;
NGSYSVNDGTNTN; GSYSVNDGTNTND; SYSVNDGTNTNDF; YSVNDGTNTNDFV;
SVNDGTNTNDFVK; VNDGTNTNDFVKK; NDGTNTNDFVKKV; DGTNTNDFVKKVI;
GTNTNDFVKKVID; TNTNDFVKKVIDD; NTNDFVKKVIDDA; TNDFVKKVIDDAL;
NDFVKKVIDDALK; DFVKKVIDDALKN; FVKKVIDDALKNI; VKKVIDDALKNIE;
KKVIDDALKNIEE; KVIDDALKNIEEE; VIDDALKNIEEEL; IDDALKNIEEELE;
DDALKNIEEELEK; DALKNIEEELEKL; ALKNIEEELEKLA; LKNIEEELEKLAE;
KNIEEELEKLAEP; NIEEELEKLAEPQ; IEEELEKLAEPQN; EEELEKLAEPQNI;
EELEKLAEPQNIE; ELEKLAEPQNIED; LEKLAEPQNIEDK; EKLAEPQNIEDKK;

14 mers:
MNKKMKNLIICAVF; NKKMKNLIICAVFV; KKMKNLIICAVFVL;
KMKNLIICAVFVLI; MKNLIICAVFVLII; KNLIICAVFVLIIS;
NLIICAVFVLIISC; LIICAVFVLIISCK; IICAVFVLIISCKI;
ICAVFVLIISCKID; CAVFVLIISCKIDA; AVFVLIISCKIDAS;
VFVLIISCKIDASS; FVLIISCKIDASSE; VLIISCKIDASSED;
LIISCKIDASSEDL; IISCKIDASSEDLK; ISCKIDASSEDLKQ;
SCKIDASSEDLKQN; CKIDASSEDLKQNV; KIDASSEDLKQNVK;
IDASSEDLKQNVKE; DASSEDLKQNVKEK; ASSEDLKQNVKEKV;
SSEDLKQNVKEKVE; SEDLKQNVKEKVEG; EDLKQNVKEKVEGF;
DLKQNVKEKVEGFL; LKQNVKEKVEGFLD; KQNVKEKVEGFLDK;
QNVKEKVEGFLDKE; NVKEKVEGFLDKEL; VKEKVEGFLDKELM;
KEKVEGFLDKELMQ; EKVEGFLDKELMQG; KVEGFLDKELMQGD;
VEGFLDKELMQGDD; EGFLDKELMQGDDP; GFLDKELMQGDDPN;
FLDKELMQGDDPNN; LDKELMQGDDPNNS; DKELMQGDDPNNSL;
KELMQGDDPNNSLF; ELMQGDDPNNSLFN; LMQGDDPNNSLFNP;
MQGDDPNNSLFNPP; QGDDPNNSLFNPPP; GDDPNNSLFNPPPV;
DDPNNSLFNPPPVL; DPNNSLFNPPPVLP; PNNSLFNPPPVLPA;
NNSLFNPPPVLPAS; NSLFNPPPVLPASS; SLFNPPPVLPASSH;
LFNPPPVLPASSHD; FNPPPVLPASSHDN; NPPPVLPASSHDNT;
PPPVLPASSHDNTP; PPVLPASSHDNTPV; PVLPASSHDNTPVL;
VLPASSHDNTPVLK; LPASSHDNTPVLKA; PASSHDNTPVLKAV;
ASSHDNTPVLKAVQ; SSHDNTPVLKAVQA; SHDNTPVLKAVQAK;
HDNTPVLKAVQAKD; DNTPVLKAVQAKDG; NTPVLKAVQAKDGG;
TPVLKAVQAKDGGQ; PVLKAVQAKDGGQQ; VLKAVQAKDGGQQE;
LKAVQAKDGGQQEG; KAVQAKDGGQQEGK; AVQAKDGGQQEGKE;
VQAKDGGQQEGKEE; QAKDGGQQEGKEEK; AKDGGQQEGKEEKE;
KDGGQQEGKEEKEK; DGGQQEGKEEKEKE; GGQQEGKEEKEKEI;
GQQEGKEEKEKEIQ; QQEGKEEKEKEIQE; QEGKEEKEKEIQEL;
EGKEEKEKEIQELK; GKEEKEKEIQELKD; KEEKEKEIQELKDK;
EEKEKEIQELKDKI; EKEKEIQELKDKID; KEKEIQELKDKIDK;
EKEIQELKDKIDKR; KEIQELKDKIDKRK; EIQELKDKIDKRKK;
IQELKDKIDKRKKE; QELKDKIDKRKKEL; ELKDKIDKRKKELE;
LKDKIDKRKKELEE; KDKIDKRKKELEEA; DKIDKRKKELEEAR;
KIDKRKKELEEARK; IDKRKKELEEARKK; DKRKKELEEARKKF;
KRKKELEEARKKFQ; RKKELEEARKKFQE; KKELEEARKKFQEF;
KELEEARKKFQEFK; ELEEARKKFQEFKE; LEEARKKFQEFKEQ;
EEARKKFQEFKEQV; EARKKFQEFKEQVE; ARKKFQEFKEQVES;
RKKFQEFKEQVESA; KKFQEFKEQVESAT; KFQEFKEQVESATG;
FQEFKEQVESATGE; QEFKEQVESATGES; EFKEQVESATGEST;
FKEQVESATGESTE; KEQVESATGESTEK; EQVESATGESTEKV;
QVESATGESTEKVK; VESATGESTEKVKK; ESATGESTEKVKKQ;
SATGESTEKVKKQG; ATGESTEKVKKQGN; TGESTEKVKKQGNI;
GESTEKVKKQGNIG; ESTEKVKKQGNIGQ; STEKVKKQGNIGQK;
TEKVKKQGNIGQKA; EKVKKQGNIGQKAL; KVKKQGNIGQKALK;
VKKQGNIGQKALKY; KKQGNIGQKALKYA; KQGNIGQKALKYAK;
QGNIGQKALKYAKE; GNIGQKALKYAKEL; NIGQKALKYAKELG;

IGQKALKYAKELGV; GQKALKYAKELGVN; QKALKYAKELGVNG;
KALKYAKELGVNGS; ALKYAKELGVNGSY; LKYAKELGVNGSYS;
KYAKELGVNGSYSV; YAKELGVNGSYSVN; AKELGVNGSYSVND;
KELGVNGSYSVNDG; ELGVNGSYSVNDGT; LGVNGSYSVNDGTN;
GVNGSYSVNDGTNT; VNGSYSVNDGTNTN; NGSYSVNDGTNTND;
GSYSVNDGTNTNDF; SYSVNDGTNTNDFV; YSVNDGTNTNDFVK;
SVNDGTNTNDFVKK; VNDGTNTNDFVKKV; NDGTNTNDFVKKVI;
DGTNTNDFVKKVID; GTNTNDFVKKVIDD; TNTNDFVKKVIDDA;
NTNDFVKKVIDDAL; TNDFVKKVIDDALK; NDFVKKVIDDALKN;
DFVKKVIDDALKNI; FVKKVIDDALKNIE; VKKVIDDALKNIEE;
KKVIDDALKNIEEE; KVIDDALKNIEEEL; VIDDALKNIEEELE;
IDDALKNIEEELEK; DDALKNIEEELEKL; DALKNIEEELEKLA;
ALKNIEEELEKLAE; LKNIEEELEKLAEP; KNIEEELEKLAEPQ;
NIEEELEKLAEPQN; IEEELEKLAEPQNI; EEELEKLAEPQNIE;
EEELEKLAEPQNIED; ELEKLAEPQNIEDK; LEKLAEPQNIEDKK;

15 mers:
MNKKMKNLIICAVFV; NKKMKNLIICAVFVL; KKMKNLIICAVFVLI;
KMKNLIICAVFVLII; MKNLIICAVFVLIIS; KNLIICAVFVLIISC;
NLIICAVFVLIISCK; LIICAVFVLIISCKI; IICAVFVLIISCKID;
ICAVFVLIISCKIDA; CAVFVLIISCKIDAS; AVFVLIISCKIDASS;
VFVLIISCKIDASSE; FVLIISCKIDASSED; VLIISCKIDASSEDL;
LIISCKIDASSEDLK; IISCKIDASSEDLKQ; ISCKIDASSEDLKQN;
SCKIDASSEDLKQNV; CKIDASSEDLKQNVK; KIDASSEDLKQNVKE;
IDASSEDLKQNVKEK; DASSEDLKQNVKEKV; ASSEDLKQNVKEKVE;
SSEDLKQNVKEKVEG; SEDLKQNVKEKVEGF; EDLKQNVKEKVEGFL;
DLKQNVKEKVEGFLD; LKQNVKEKVEGFLDK; KQNVKEKVEGFLDKE;
QNVKEKVEGFLDKEL; NVKEKVEGFLDKELM; VKEKVEGFLDKELMQ;
KEKVEGFLDKELMQG; EKVEGFLDKELMQGD; KVEGFLDKELMQGDD;
VEGFLDKELMQGDDP; EGFLDKELMQGDDPN; GFLDKELMQGDDPNN;
FLDKELMQGDDPNNS; LDKELMQGDDPNNSL; DKELMQGDDPNNSLF;
KELMQGDDPNNSLFN; ELMQGDDPNNSLFNP; LMQGDDPNNSLFNPP;
MQGDDPNNSLFNPPP; QGDDPNNSLFNPPPV; GDDPNNSLFNPPPVL;
DDPNNSLFNPPPVLP; DPNNSLFNPPPVLPA; PNNSLFNPPPVLPAS;
NNSLFNPPPVLPASS; NSLFNPPPVLPASSH; SLFNPPPVLPASSHD;
LFNPPPVLPASSHDN; FNPPPVLPASSHDNT; NPPPVLPASSHDNTP;
PPPVLPASSHDNTPV; PPVLPASSHDNTPVL; PVLPASSHDNTPVLK;
VLPASSHDNTPVLKA; LPASSHDNTPVLKAV; PASSHDNTPVLKAVQ;
ASSHDNTPVLKAVQA; SSHDNTPVLKAVQAK; SHDNTPVLKAVQAKD;
HDNTPVLKAVQAKDG; DNTPVLKAVQAKDGG; NTPVLKAVQAKDGGQ;
TPVLKAVQAKDGGQQ; PVLKAVQAKDGGQQE; VLKAVQAKDGGQQEG;
LKAVQAKDGGQQEGK; KAVQAKDGGQQEGKE; AVQAKDGGQQEGKEE;
VQAKDGGQQEGKEEK; QAKDGGQQEGKEEKE; AKDGGQQEGKEEKEK;
KDGGQQEGKEEKEKE; DGGQQEGKEEKEKEI; GGQQEGKEEKEKEIQ;
GQQEGKEEKEKEIQE; QQEGKEEKEKEIQEL; QEGKEEKEKEIQELK;
EGKEEKEKEIQELKD; GKEEKEKEIQELKDK; KEEKEKEIQELKDKI;
EEKEKEIQELKDKID; EKEKEIQELKDKIDK; KEKEIQELKDKIDKR;
EKEIQELKDKIDKRK; KEIQELKDKIDKRKK; EIQELKDKIDKRKKE;
IQELKDKIDKRKKEL; QELKDKIDKRKKELE; ELKDKIDKRKKELEE;
LKDKIDKRKKELEEA; KDKIDKRKKELEEAR; DKIDKRKKELEEARK;
KIDKRKKELEEARKK; IDKRKKELEEARKKF; DKRKKELEEARKKFQ;
KRKKELEEARKKFQE; RKKELEEARKKFQEF; KKELEEARKKFQEFK;
KELEEARKKFQEFKE; ELEEARKKFQEFKEQ; LEEARKKFQEFKEQV;
EEARKKFQEFKEQVE; EARKKFQEFKEQVES; ARKKFQEFKEQVESA;
RKKFQEFKEQVESAT; KKFQEFKEQVESATG; KFQEFKEQVESATGE;
FQEFKEQVESATGES; QEFKEQVESATGEST; EFKEQVESATGESTE;
FKEQVESATGESTEK; KEQVESATGESTEKV; EQVESATGESTEKVK;

QVESATGESTEKVKK; VESATGESTEKVKKQ; ESATGESTEKVKKQG;
SATGESTEKVKKQGN; ATGESTEKVKKQGNI; TGESTEKVKKQGNIG;
GESTEKVKKQGNIGQ; ESTEKVKKQGNIGQK; STEKVKKQGNIGQKA;
TEKVKKQGNIGQKAL; EKVKKQGNIGQKALK; KVKKQGNIGQKALKY;
VKKQGNIGQKALKYA; KKQGNIGQKALKYAK; KQGNIGQKALKYAKE;
QGNIGQKALKYAKEL; GNIGQKALKYAKELG; NIGQKALKYAKELGV;
IGQKALKYAKELGVN; GQKALKYAKELGVNG; QKALKYAKELGVNGS;
KALKYAKELGVNGSY; ALKYAKELGVNGSYS; LKYAKELGVNGSYSV;
KYAKELGVNGSYSVN; YAKELGVNGSYSVND; AKELGVNGSYSVNDG;
KELGVNGSYSVNDGT; ELGVNGSYSVNDGTN; LGVNGSYSVNDGTNT;
GVNGSYSVNDGTNTN; VNGSYSVNDGTNTND; NGSYSVNDGTNTNDF;
GSYSVNDGTNTNDFV; SYSVNDGTNTNDFVK; YSVNDGTNTNDFVKK;
SVNDGTNTNDFVKKV; VNDGTNTNDFVKKVI; NDGTNTNDFVKKVID;
DGTNTNDFVKKVIDD; GTNTNDFVKKVIDDA; TNTNDFVKKVIDDAL;
NTNDFVKKVIDDALK; TNDFVKKVIDDALKN; NDFVKKVIDDALKNI;
DFVKKVIDDALKNIE; FVKKVIDDALKNIEE; VKKVIDDALKNIEEE;
KKVIDDALKNIEEEL; KVIDDALKNIEEELE; VIDDALKNIEEELEK;
IDDALKNIEEELEKL; DDALKNIEEELEKLA; DALKNIEEELEKLAE;
ALKNIEEELEKLAEP; LKNIEEELEKLAEPQ; KNIEEELEKLAEPQN;
NIEEELEKLAEPQNI; IEEELEKLAEPQNIE; EEELEKLAEPQNIED;
EELEKLAEPQNIEDK; ELEKLAEPQNIEDKK;

16 mers:
MNKKMKNLIICAVFVL; NKKMKNLIICAVFVLI; KKMKNLIICAVFVLII;
KMKNLIICAVFVLIIS; MKNLIICAVFVLIISC; KNLIICAVFVLIISCK;
NLIICAVFVLIISCKI; LIICAVFVLIISCKID; IICAVFVLIISCKIDA;
ICAVFVLIISCKIDAS; CAVFVLIISCKIDASS; AVFVLIISCKIDASSE;
VFVLIISCKIDASSED; FVLIISCKIDASSEDL; VLIISCKIDASSEDLK;
LIISCKIDASSEDLKQ; IISCKIDASSEDLKQN; ISCKIDASSEDLKQNV;
SCKIDASSEDLKQNVK; CKIDASSEDLKQNVKE; KIDASSEDLKQNVKEK;
IDASSEDLKQNVKEKV; DASSEDLKQNVKEKVE; ASSEDLKQNVKEKVEG;
SSEDLKQNVKEKVEGF; SEDLKQNVKEKVEGFL; EDLKQNVKEKVEGFLD;
DLKQNVKEKVEGFLDK; LKQNVKEKVEGFLDKE; KQNVKEKVEGFLDKEL;
QNVKEKVEGFLDKELM; NVKEKVEGFLDKELMQ; VKEKVEGFLDKELMQG;
KEKVEGFLDKELMQGD; EKVEGFLDKELMQGDD; KVEGFLDKELMQGDDP;
VEGFLDKELMQGDDPN; EGFLDKELMQGDDPNN; GFLDKELMQGDDPNNS;
FLDKELMQGDDPNNSL; LDKELMQGDDPNNSLF; DKELMQGDDPNNSLFN;
KELMQGDDPNNSLFNP; ELMQGDDPNNSLFNPP; LMQGDDPNNSLFNPPP;
MQGDDPNNSLFNPPPV; QGDDPNNSLFNPPPVL; GDDPNNSLFNPPPVLP;
DDPNNSLFNPPPVLPA; DPNNSLFNPPPVLPAS; PNNSLFNPPPVLPASS;
NNSLFNPPPVLPASSH; NSLFNPPPVLPASSHD; SLFNPPPVLPASSHDN;
LFNPPPVLPASSHDNT; FNPPPVLPASSHDNTP; NPPPVLPASSHDNTPV;
PPPVLPASSHDNTPVL; PPVLPASSHDNTPVLK; PVLPASSHDNTPVLKA;
VLPASSHDNTPVLKAV; LPASSHDNTPVLKAVQ; PASSHDNTPVLKAVQA;
ASSHDNTPVLKAVQAK; SSHDNTPVLKAVQAKD; SHDNTPVLKAVQAKDG;
HDNTPVLKAVQAKDGG; DNTPVLKAVQAKDGGQ; NTPVLKAVQAKDGGQQ;
TPVLKAVQAKDGGQQE; PVLKAVQAKDGGQQEG; VLKAVQAKDGGQQEGK;
LKAVQAKDGGQQEGKE; KAVQAKDGGQQEGKEE; AVQAKDGGQQEGKEEK;
VQAKDGGQQEGKEEKE; QAKDGGQQEGKEEKEK; AKDGGQQEGKEEKEKE;
KDGGQQEGKEEKEKEI; DGGQQEGKEEKEKEIQ; GGQQEGKEEKEKEIQE;
GQQEGKEEKEKEIQEL; QQEGKEEKEKEIQELK; QEGKEEKEKEIQELKD;
EGKEEKEKEIQELKDK; GKEEKEKEIQELKDKI; KEEKEKEIQELKDKID;
EEKEKEIQELKDKIDK; EKEKEIQELKDKIDKR; KEKEIQELKDKIDKRK;
EKEIQELKDKIDKRKK; KEIQELKDKIDKRKKE; EIQELKDKIDKRKKEL;
IQELKDKIDKRKKELE; QELKDKIDKRKKELEE; ELKDKIDKRKKELEEA;
LKDKIDKRKKELEEAR; KDKIDKRKKELEEARK; DKIDKRKKELEEARKK;
KIDKRKKELEEARKKF; IDKRKKELEEARKKFQ; DKRKKELEEARKKFQE;

| | |
|---|---|
| | KRKKELEEARKKFQEF; RKKELEEARKKFQEFK; KKELEEARKKFQEFKE;<br>KELEEARKKFQEFKEQ; ELEEARKKFQEFKEQV; LEEARKKFQEFKEQVE;<br>EEARKKFQEFKEQVES; EARKKFQEFKEQVESA; ARKKFQEFKEQVESAT;<br>RKKFQEFKEQVESATG; KKFQEFKEQVESATGE; KFQEFKEQVESATGES;<br>FQEFKEQVESATGEST; QEFKEQVESATGESTE; EFKEQVESATGESTEK;<br>FKEQVESATGESTEKV; KEQVESATGESTEKVK; EQVESATGESTEKVKK;<br>QVESATGESTEKVKKQ; VESATGESTEKVKKQG; ESATGESTEKVKKQGN;<br>SATGESTEKVKKQGNI; ATGESTEKVKKQGNIG; TGESTEKVKKQGNIGQ;<br>GESTEKVKKQGNIGQK; ESTEKVKKQGNIGQKA; STEKVKKQGNIGQKAL;<br>TEKVKKQGNIGQKALK; EKVKKQGNIGQKALKY; KVKKQGNIGQKALKYA;<br>VKKQGNIGQKALKYAK; KKQGNIGQKALKYAKE; KQGNIGQKALKYAKEL;<br>QGNIGQKALKYAKELG; GNIGQKALKYAKELGV; NIGQKALKYAKELGVN;<br>IGQKALKYAKELGVNG; GQKALKYAKELGVNGS; QKALKYAKELGVNGSY;<br>KALKYAKELGVNGSYS; ALKYAKELGVNGSYSV; LKYAKELGVNGSYSVN;<br>KYAKELGVNGSYSVND; YAKELGVNGSYSVNDG; AKELGVNGSYSVNDGT;<br>KELGVNGSYSVNDGTN; ELGVNGSYSVNDGTNT; LGVNGSYSVNDGTNTN;<br>GVNGSYSVNDGTNTND; VNGSYSVNDGTNTNDF; NGSYSVNDGTNTNDFV;<br>GSYSVNDGTNTNDFVK; SYSVNDGTNTNDFVKK; YSVNDGTNTNDFVKKV;<br>SVNDGTNTNDFVKKVI; VNDGTNTNDFVKKVID; NDGTNTNDFVKKVIDD;<br>DGTNTNDFVKKVIDDA; GTNTNDFVKKVIDDAL; TNTNDFVKKVIDDALK;<br>NTNDFVKKVIDDALKN; TNDFVKKVIDDALKNI; NDFVKKVIDDALKNIE;<br>DFVKKVIDDALKNIEE; FVKKVIDDALKNIEEE; VKKVIDDALKNIEEEL;<br>KKVIDDALKNIEEELE; KVIDDALKNIEEELEK; VIDDALKNIEEELEKL;<br>IDDALKNIEEELEKLA; DDALKNIEEELEKLAE; DALKNIEEELEKLAEP;<br>ALKNIEEELEKLAEPQ; LKNIEEELEKLAEPQN; KNIEEELEKLAEPQNI;<br>NIEEELEKLAEPQNIE; IEEELEKLAEPQNIED; EEELEKLAEPQNIEDK;<br>EELEKLAEPQNIEDKK; |
| AAC44770.1\| FlaA<br>protein (Borrelia<br>burgdorferi) | 13 mers:<br>MKRKAKSILFFLL; KRKAKSILFFLLS; RKAKSILFFLLST; KAKSILFFLLSTV;<br>AKSILFFLLSTVL; KSILFFLLSTVLF; SILFFLLSTVLFA; ILFFLLSTVLFAQ;<br>LFFLLSTVLFAQE; FFLLSTVLFAQET; FLLSTVLFAQETD; LLSTVLFAQETDG;<br>LSTVLFAQETDGL; STVLFAQETDGLA; TVLFAQETDGLAE; VLFAQETDGLAEG;<br>LFAQETDGLAEGS; FAQETDGLAEGSK; AQETDGLAEGSKR; QETDGLAEGSKRA;<br>ETDGLAEGSKRAE; TDGLAEGSKRAEP; DGLAEGSKRAEPG; GLAEGSKRAEPGE;<br>LAEGSKRAEPGEL; AEGSKRAEPGELV; EGSKRAEPGELVL; GSKRAEPGELVLD;<br>SKRAEPGELVLDF; KRAEPGELVLDFA; RAEPGELVLDFAE; AEPGELVLDFAEL;<br>EPGELVLDFAELA; PGELVLDFAELAR; GELVLDFAELARD; ELVLDFAELARDP;<br>LVLDFAELARDPS; VLDFAELARDPSS; LDFAELARDPSST; DFAELARDPSSTR;<br>FAELARDPSSTRL; AELARDPSSTRLD; ELARDPSSTRLDL; LARDPSSTRLDLT;<br>ARDPSSTRLDLTN; RDPSSTRLDLTNY; DPSSTRLDLTNYV; PSSTRLDLTNYVD;<br>SSTRLDLTNYVDY; STRLDLTNYVDYV; TRLDLTNYVDYVY; RLDLTNYVDYVYS;<br>LDLTNYVDYVYSG; DLTNYVDYVYSGA; LTNYVDYVYSGAS; TNYVDYVYSGASG;<br>NYVDYVYSGASGI; YVDYVYSGASGIV; VDYVYSGASGIVK; DYVYSGASGIVKP;<br>YVYSGASGIVKPE; VYSGASGIVKPED; YSGASGIVKPEDM; SGASGIVKPEDMV;<br>GASGIVKPEDMVV; ASGIVKPEDMVVD; SGIVKPEDMVVDL; GIVKPEDMVVDLG;<br>IVKPEDMVVDLGI; VKPEDMVVDLGIN; KPEDMVVDLGINN; PEDMVVDLGINNW;<br>EDMVVDLGINNWS; DMVVDLGINNWSV; MVVDLGINNWSVL; VVDLGINNWSVLL;<br>VDLGINNWSVLLT; DLGINNWSVLLTP; LGINNWSVLLTPS; GINNWSVLLTPSA;<br>INNWSVLLTPSAR; NNWSVLLTPSARL; NWSVLLTPSARLQ; WSVLLTPSARLQA;<br>SVLLTPSARLQAY; VLLTPSARLQAYV; LLTPSARLQAYVK; LTPSARLQAYVKN;<br>TPSARLQAYVKNS; PSARLQAYVKNSV; SARLQAYVKNSVV; ARLQAYVKNSVVA;<br>RLQAYVKNSVVAP; LQAYVKNSVVAPA; QAYVKNSVVAPAV; AYVKNSVVAPAVV;<br>YVKNSVVAPAVVK; VKNSVVAPAVVKS; KNSVVAPAVVKSE; NSVVAPAVVKSES;<br>SVVAPAVVKSESK; VVAPAVVKSESKR; VAPAVVKSESKRY; APAVVKSESKRYA;<br>PAVVKSESKRYAG; AVVKSESKRYAGD; VVKSESKRYAGDT; VKSESKRYAGDTI; |

KSESKRYAGDTIL; SESKRYAGDTILG; ESKRYAGDTILGV; SKRYAGDTILGVR;
KRYAGDTILGVRV; RYAGDTILGVRVL; YAGDTILGVRVLF; AGDTILGVRVLFP;
GDTILGVRVLFPS; DTILGVRVLFPSY; TILGVRVLFPSYS; ILGVRVLFPSYSQ;
LGVRVLFPSYSQS; GVRVLFPSYSQSS; VRVLFPSYSQSSA; RVLFPSYSQSSAM;
VLFPSYSQSSAMI; LFPSYSQSSAMIM; FPSYSQSSAMIMP; PSYSQSSAMIMPP;
SYSQSSAMIMPPF; YSQSSAMIMPPFK; SQSSAMIMPPFKI; QSSAMIMPPFKIP;
SSAMIMPPFKIPF; SAMIMPPFKIPFY; AMIMPPFKIPFYS; MIMPPFKIPFYSG;
IMPPFKIPFYSGE; MPPFKIPFYSGES; PPFKIPFYSGESG; PFKIPFYSGESGN;
FKIPFYSGESGNQ; KIPFYSGESGNQF; IPFYSGESGNQFL; PFYSGESGNQFLG;
FYSGESGNQFLGK; YSGESGNQFLGKG; SGESGNQFLGKGL; GESGNQFLGKGLI;
ESGNQFLGKGLID; SGNQFLGKGLIDN; GNQFLGKGLIDNI; NQFLGKGLIDNIK;
QFLGKGLIDNIKT; FLGKGLIDNIKTM; LGKGLIDNIKTMK; GKGLIDNIKTMKE;
KGLIDNIKTMKEI; GLIDNIKTMKEIK; LIDNIKTMKEIKV; IDNIKTMKEIKVS;
DNIKTMKEIKVSV; NIKTMKEIKVSVY; IKTMKEIKVSVYS; KTMKEIKVSVYSL;
TMKEIKVSVYSLG; MKEIKVSVYSLGY; KEIKVSVYSLGYE; EIKVSVYSLGYEI;
IKVSVYSLGYEID; KVSVYSLGYEIDL; VSVYSLGYEIDLE; SVYSLGYEIDLEV;
VYSLGYEIDLEVL; YSLGYEIDLEVLF; SLGYEIDLEVLFE; LGYEIDLEVLFED;
GYEIDLEVLFEDM; YEIDLEVLFEDMN; EIDLEVLFEDMNG; IDLEVLFEDMNGM;
DLEVLFEDMNGME; LEVLFEDMNGMEY; EVLFEDMNGMEYA; VLFEDMNGMEYAY;
LFEDMNGMEYAYS; FEDMNGMEYAYSM; EDMNGMEYAYSMG; DMNGMEYAYSMGT;
MNGMEYAYSMGTL; NGMEYAYSMGTLK; GMEYAYSMGTLKF; MEYAYSMGTLKFK;
EYAYSMGTLKFKG; YAYSMGTLKFKGW; AYSMGTLKFKGWA; YSMGTLKFKGWAD;
SMGTLKFKGWADL; MGTLKFKGWADLI; GTLKFKGWADLIW; TLKFKGWADLIWS;
LKFKGWADLIWSN; KFKGWADLIWSNP; FKGWADLIWSNPN; KGWADLIWSNPNY;
GWADLIWSNPNYI; WADLIWSNPNYIP; ADLIWSNPNYIPN; DLIWSNPNYIPNI;
LIWSNPNYIPNIS; IWSNPNYIPNISS; WSNPNYIPNISSR; SNPNYIPNISSRI;
NPNYIPNISSRII; PNYIPNISSRIIK; NYIPNISSRIIKD; YIPNISSRIIKDD;
IPNISSRIIKDDV; PNISSRIIKDDVP; NISSRIIKDDVPN; ISSRIIKDDVPNY;
SSRIIKDDVPNYP; SRIIKDDVPNYPL; RIIKDDVPNYPLA; IIKDDVPNYPLAS;
IKDDVPNYPLASS; KDDVPNYPLASSK; DDVPNYPLASSKM; DVPNYPLASSKMR;
VPNYPLASSKMRF; PNYPLASSKMRFK; NYPLASSKMRFKA; YPLASSKMRFKAF;
PLASSKMRFKAFR; LASSKMRFKAFRV; ASSKMRFKAFRVS; SSKMRFKAFRVSK;
SKMRFKAFRVSKS; KMRFKAFRVSKSH; MRFKAFRVSKSHS; RFKAFRVSKSHSS;
FKAFRVSKSHSSK; KAFRVSKSHSSKV; AFRVSKSHSSKVK; FRVSKSHSSKVKN;
RVSKSHSSKVKNF; VSKSHSSKVKNFI; SKSHSSKVKNFIF; KSHSSKVKNFIFY;
SHSSKVKNFIFYV; HSSKVKNFIFYVK; SSKVKNFIFYVKD; SKVKNFIFYVKDL;
KVKNFIFYVKDLR; VKNFIFYVKDLRV; KNFIFYVKDLRVL; NFIFYVKDLRVLY;
FIFYVKDLRVLYD; IFYVKDLRVLYDK; FYVKDLRVLYDKL; YVKDLRVLYDKLS;
VKDLRVLYDKLSV; KDLRVLYDKLSVS; DLRVLYDKLSVSI; LRVLYDKLSVSID;
RVLYDKLSVSIDS; VLYDKLSVSIDSD; LYDKLSVSIDSDI; YDKLSVSIDSDID;
DKLSVSIDSDIDS; KLSVSIDSDIDSE; LSVSIDSDIDSES; SVSIDSDIDSESV;
VSIDSDIDSESVF; SIDSDIDSESVFK; IDSDIDSESVFKV; DSDIDSESVFKVY;
SDIDSESVFKVYE; DIDSESVFKVYET; IDSESVFKVYETS; DSESVFKVYETSG;
SESVFKVYETSGT; ESVFKVYETSGTE; SVFKVYETSGTES; VFKVYETSGTESL;
FKVYETSGTESLR; KVYETSGTESLRK; VYETSGTESLRKL; YETSGTESLRKLK;
ETSGTESLRKLKA; TSGTESLRKLKAH; SGTESLRKLKAHE; GTESLRKLKAHET;
TESLRKLKAHETF; ESLRKLKAHETFK; SLRKLKAHETFKR; LRKLKAHETFKRV;
RKLKAHETFKRVL; KLKAHETFKRVLK; LKAHETFKRVLKL; KAHETFKRVLKLR;
AHETFKRVLKLRE; HETFKRVLKLREK; ETFKRVLKLREKI; TFKRVLKLREKIS;
FKRVLKLREKISI; KRVLKLREKISIA; RVLKLREKISIAE; VLKLREKISIAEG;
LKLREKISIAEGS; KLREKISIAEGSF; LREKISIAEGSFQ; REKISIAEGSFQN;
EKISIAEGSFQNF; KISIAEGSFQNFV; ISIAEGSFQNFVE; SIAEGSFQNFVEK;
IAEGSFQNFVEKI; AEGSFQNFVEKIE; EGSFQNFVEKIES; GSFQNFVEKIESE;
SFQNFVEKIESEK; FQNFVEKIESEKP; QNFVEKIESEKPE; NFVEKIESEKPEE;
FVEKIESEKPEES; VEKIESEKPEESS; EKIESEKPEESSP; KIESEKPEESSPK;
IESEKPEESSPKN;

EP 2 254 592 B1

| | 14 mers:<br>MKRKAKSILFFLLS; KRKAKSILFFLLST; RKAKSILFFLLSTV;<br>KAKSILFFLLSTVL; AKSILFFLLSTVLF; KSILFFLLSTVLFA;<br>SILFFLLSTVLFAQ; ILFFLLSTVLFAQE; LFFLLSTVLFAQET;<br>FFLLSTVLFAQETD; FLLSTVLFAQETDG; LLSTVLFAQETDGL;<br>LSTVLFAQETDGLA; STVLFAQETDGLAE; TVLFAQETDGLAEG;<br>VLFAQETDGLAEGS; LFAQETDGLAEGSK; FAQETDGLAEGSKR;<br>AQETDGLAEGSKRA; QETDGLAEGSKRAE; ETDGLAEGSKRAEP;<br>TDGLAEGSKRAEPG; DGLAEGSKRAEPGE; GLAEGSKRAEPGEL;<br>LAEGSKRAEPGELV; AEGSKRAEPGELVL; EGSKRAEPGELVLD;<br>GSKRAEPGELVLDF; SKRAEPGELVLDFA; KRAEPGELVLDFAE;<br>RAEPGELVLDFAEL; AEPGELVLDFAELA; EPGELVLDFAELAR;<br>PGELVLDFAELARD; GELVLDFAELARDP; ELVLDFAELARDPS;<br>LVLDFAELARDPSS; VLDFAELARDPSST; LDFAELARDPSSTR;<br>DFAELARDPSSTRL; FAELARDPSSTRLD; AELARDPSSTRLDL;<br>ELARDPSSTRLDLT; LARDPSSTRLDLTN; ARDPSSTRLDLTNY;<br>RDPSSTRLDLTNYV; DPSSTRLDLTNYVD; PSSTRLDLTNYVDY;<br>SSTRLDLTNYVDYV; STRLDLTNYVDYVY; TRLDLTNYVDYVYS;<br>RLDLTNYVDYVYSG; LDLTNYVDYVYSGA; DLTNYVDYVYSGAS;<br>LTNYVDYVYSGASG; TNYVDYVYSGASGI; NYVDYVYSGASGIV;<br>YVDYVYSGASGIVK; VDYVYSGASGIVKP; DYVYSGASGIVKPE;<br>YVYSGASGIVKPED; VYSGASGIVKPEDM; YSGASGIVKPEDMV;<br>SGASGIVKPEDMVV; GASGIVKPEDMVVD; ASGIVKPEDMVVDL;<br>SGIVKPEDMVVDLG; GIVKPEDMVVDLGI; IVKPEDMVVDLGIN;<br>VKPEDMVVDLGINN; KPEDMVVDLGINNW; PEDMVVDLGINNWS;<br>EDMVVDLGINNWSV; DMVVDLGINNWSVL; MVVDLGINNWSVLL;<br>VVDLGINNWSVLLT; VDLGINNWSVLLTP; DLGINNWSVLLTPS;<br>LGINNWSVLLTPSA; GINNWSVLLTPSAR; INNWSVLLTPSARL;<br>NNWSVLLTPSARLQ; NWSVLLTPSARLQA; WSVLLTPSARLQAY;<br>SVLLTPSARLQAYV; VLLTPSARLQAYVK; LLTPSARLQAYVKN;<br>LTPSARLQAYVKNS; TPSARLQAYVKNSV; PSARLQAYVKNSVV;<br>SARLQAYVKNSVVA; ARLQAYVKNSVVAP; RLQAYVKNSVVAPA;<br>LQAYVKNSVVAPAV; QAYVKNSVVAPAVV; AYVKNSVVAPAVVK;<br>YVKNSVVAPAVVKS; VKNSVVAPAVVKSE; KNSVVAPAVVKSES;<br>NSVVAPAVVKSESK; SVVAPAVVKSESKR; VVAPAVVKSESKRY;<br>VAPAVVKSESKRYA; APAVVKSESKRYAG; PAVVKSESKRYAGD;<br>AVVKSESKRYAGDT; VVKSESKRYAGDTI; VKSESKRYAGDTIL;<br>KSESKRYAGDTILG; SESKRYAGDTILGV; ESKRYAGDTILGVR;<br>SKRYAGDTILGVRV; KRYAGDTILGVRVL; RYAGDTILGVRVLF;<br>YAGDTILGVRVLFP; AGDTILGVRVLFPS; GDTILGVRVLFPSY;<br>DTILGVRVLFPSYS; TILGVRVLFPSYSQ; ILGVRVLFPSYSQS;<br>LGVRVLFPSYSQSS; GVRVLFPSYSQSSA; VRVLFPSYSQSSAM;<br>RVLFPSYSQSSAMI; VLFPSYSQSSAMIM; LFPSYSQSSAMIMP;<br>FPSYSQSSAMIMPP; PSYSQSSAMIMPPF; SYSQSSAMIMPPFK;<br>YSQSSAMIMPPFKI; SQSSAMIMPPFKIP; QSSAMIMPPFKIPF;<br>SSAMIMPPFKIPFY; SAMIMPPFKIPFYS; AMIMPPFKIPFYSG;<br>MIMPPFKIPFYSGE; IMPPFKIPFYSGES; MPPFKIPFYSGESG;<br>PPFKIPFYSGESGN; PFKIPFYSGESGNQ; FKIPFYSGESGNQF;<br>KIPFYSGESGNQFL; IPFYSGESGNQFLG; PFYSGESGNQFLGK;<br>FYSGESGNQFLGKG; YSGESGNQFLGKGL; SGESGNQFLGKGLI;<br>GESGNQFLGKGLID; ESGNQFLGKGLIDN; SGNQFLGKGLIDNI;<br>GNQFLGKGLIDNIK; NQFLGKGLIDNIKT; QFLGKGLIDNIKTM;<br>FLGKGLIDNIKTMK; LGKGLIDNIKTMKE; GKGLIDNIKTMKEI;<br>KGLIDNIKTMKEIK; GLIDNIKTMKEIKV; LIDNIKTMKEIKVS;<br>IDNIKTMKEIKVSV; DNIKTMKEIKVSVY; NIKTMKEIKVSVYS;<br>IKTMKEIKVSVYSL; KTMKEIKVSVYSLG; TMKEIKVSVYSLGY;<br>MKEIKVSVYSLGYE; KEIKVSVYSLGYEI; EIKVSVYSLGYEID; |
|---|---|

IKVSVYSLGYEIDL; KVSVYSLGYEIDLE; VSVYSLGYEIDLEV;
SVYSLGYEIDLEVL; VYSLGYEIDLEVLF; YSLGYEIDLEVLFE;
SLGYEIDLEVLFED; LGYEIDLEVLFEDM; GYEIDLEVLFEDMN;
YEIDLEVLFEDMNG; EIDLEVLFEDMNGM; IDLEVLFEDMNGME;
DLEVLFEDMNGMEY; LEVLFEDMNGMEYA; EVLFEDMNGMEYAY;
VLFEDMNGMEYAYS; LFEDMNGMEYAYSM; FEDMNGMEYAYSMG;
EDMNGMEYAYSMGT; DMNGMEYAYSMGTL; MNGMEYAYSMGTLK;
NGMEYAYSMGTLKF; GMEYAYSMGTLKFK; MEYAYSMGTLKFKG;
EYAYSMGTLKFKGW; YAYSMGTLKFKGWA; AYSMGTLKFKGWAD;
YSMGTLKFKGWADL; SMGTLKFKGWADLI; MGTLKFKGWADLIW;
GTLKFKGWADLIWS; TLKFKGWADLIWSN; LKFKGWADLIWSNP;
KFKGWADLIWSNPN; FKGWADLIWSNPNY; KGWADLIWSNPNYI;
GWADLIWSNPNYIP; WADLIWSNPNYIPN; ADLIWSNPNYIPNI;
DLIWSNPNYIPNIS; LIWSNPNYIPNISS; IWSNPNYIPNISSR;
WSNPNYIPNISSRI; SNPNYIPNISSRII; NPNYIPNISSRIIK;
PNYIPNISSRIIKD; NYIPNISSRIIKDD; YIPNISSRIIKDDV;
IPNISSRIIKDDVP; PNISSRIIKDDVPN; NISSRIIKDDVPNY;
ISSRIIKDDVPNYP; SSRIIKDDVPNYPL; SRIIKDDVPNYPLA;
RIIKDDVPNYPLAS; IIKDDVPNYPLASS; IKDDVPNYPLASSK;
KDDVPNYPLASSKM; DDVPNYPLASSKMR; DVPNYPLASSKMRF;
VPNYPLASSKMRFK; PNYPLASSKMRFKA; NYPLASSKMRFKAF;
YPLASSKMRFKAFR; PLASSKMRFKAFRV; LASSKMRFKAFRVS;
ASSKMRFKAFRVSK; SSKMRFKAFRVSKS; SKMRFKAFRVSKSH;
KMRFKAFRVSKSHS; MRFKAFRVSKSHSS; RFKAFRVSKSHSSK;
FKAFRVSKSHSSKV; KAFRVSKSHSSKVK; AFRVSKSHSSKVKN;
FRVSKSHSSKVKNF; RVSKSHSSKVKNFI; VSKSHSSKVKNFIF;
SKSHSSKVKNFIFY; KSHSSKVKNFIFYV; SHSSKVKNFIFYVK;
HSSKVKNFIFYVKD; SSKVKNFIFYVKDL; SKVKNFIFYVKDLR;
KVKNFIFYVKDLRV; VKNFIFYVKDLRVL; KNFIFYVKDLRVLY;
NFIFYVKDLRVLYD; FIFYVKDLRVLYDK; IFYVKDLRVLYDKL;
FYVKDLRVLYDKLS; YVKDLRVLYDKLSV; VKDLRVLYDKLSVS;
KDLRVLYDKLSVSI; DLRVLYDKLSVSID; LRVLYDKLSVSIDS;
RVLYDKLSVSIDSD; VLYDKLSVSIDSDI; LYDKLSVSIDSDID;
YDKLSVSIDSDIDS; DKLSVSIDSDIDSE; KLSVSIDSDIDSES;
LSVSIDSDIDSESV; SVSIDSDIDSESVF; VSIDSDIDSESVFK;
SIDSDIDSESVFKV; IDSDIDSESVFKVY; DSDIDSESVFKVYE;
SDIDSESVFKVYET; DIDSESVFKVYETS; IDSESVFKVYETSG;
DSESVFKVYETSGT; SESVFKVYETSGTE; ESVFKVYETSGTES;
SVFKVYETSGTESL; VFKVYETSGTESLR; FKVYETSGTESLRK;
KVYETSGTESLRKL; VYETSGTESLRKLK; YETSGTESLRKLKA;
ETSGTESLRKLKAH; TSGTESLRKLKAHE; SGTESLRKLKAHET;
GTESLRKLKAHETF; TESLRKLKAHETFK; ESLRKLKAHETFKR;
SLRKLKAHETFKRV; LRKLKAHETFKRVL; RKLKAHETFKRVLK;
KLKAHETFKRVLKL; LKAHETFKRVLKLR; KAHETFKRVLKLRE;
AHETFKRVLKLREK; HETFKRVLKLREKI; ETFKRVLKLREKIS;
TFKRVLKLREKISI; FKRVLKLREKISIA; KRVLKLREKISIAE;
RVLKLREKISIAEG; VLKLREKISIAEGS; LKLREKISIAEGSF;
KLREKISIAEGSFQ; LREKISIAEGSFQN; REKISIAEGSFQNF;
EKISIAEGSFQNFV; KISIAEGSFQNFVE; ISIAEGSFQNFVEK;
SIAEGSFQNFVEKI; IAEGSFQNFVEKIE; AEGSFQNFVEKIES;
EGSFQNFVEKIESE; GSFQNFVEKIESEK; SFQNFVEKIESEKP;
FQNFVEKIESEKPE; QNFVEKIESEKPEE; NFVEKIESEKPEES;
FVEKIESEKPEESS; VEKIESEKPEESSP; EKIESEKPEESSPK;
KIESEKPEESSPKN;

15 mers:
MKRKAKSILFFLLST; KRKAKSILFFLLSTV; RKAKSILFFLLSTVL;

KAKSILFFLLSTVLF; AKSILFFLLSTVLFA; KSILFFLLSTVLFAQ;
SILFFLLSTVLFAQE; ILFFLLSTVLFAQET; LFFLLSTVLFAQETD;
FFLLSTVLFAQETDG; FLLSTVLFAQETDGL; LLSTVLFAQETDGLA;
LSTVLFAQETDGLAE; STVLFAQETDGLAEG; TVLFAQETDGLAEGS;
VLFAQETDGLAEGSK; LFAQETDGLAEGSKR; FAQETDGLAEGSKRA;
AQETDGLAEGSKRAE; QETDGLAEGSKRAEP; ETDGLAEGSKRAEPG;
TDGLAEGSKRAEPGE; DGLAEGSKRAEPGEL; GLAEGSKRAEPGELV;
LAEGSKRAEPGELVL; AEGSKRAEPGELVLD; EGSKRAEPGELVLDF;
GSKRAEPGELVLDFA; SKRAEPGELVLDFAE; KRAEPGELVLDFAEL;
RAEPGELVLDFAELA; AEPGELVLDFAELAR; EPGELVLDFAELARD;
PGELVLDFAELARDP; GELVLDFAELARDPS; ELVLDFAELARDPSS;
LVLDFAELARDPSST; VLDFAELARDPSSTR; LDFAELARDPSSTRL;
DFAELARDPSSTRLD; FAELARDPSSTRLDL; AELARDPSSTRLDLT;
ELARDPSSTRLDLTN; LARDPSSTRLDLTNY; ARDPSSTRLDLTNYV;
RDPSSTRLDLTNYVD; DPSSTRLDLTNYVDY; PSSTRLDLTNYVDYV;
SSTRLDLTNYVDYVY; STRLDLTNYVDYVYS; TRLDLTNYVDYVYSG;
RLDLTNYVDYVYSGA; LDLTNYVDYVYSGAS; DLTNYVDYVYSGASG;
LTNYVDYVYSGASGI; TNYVDYVYSGASGIV; NYVDYVYSGASGIVK;
YVDYVYSGASGIVKP; VDYVYSGASGIVKPE; DYVYSGASGIVKPED;
YVYSGASGIVKPEDM; VYSGASGIVKPEDMV; YSGASGIVKPEDMVV;
SGASGIVKPEDMVVD; GASGIVKPEDMVVDL; ASGIVKPEDMVVDLG;
SGIVKPEDMVVDLGI; GIVKPEDMVVDLGIN; IVKPEDMVVDLGINN;
VKPEDMVVDLGINNW; KPEDMVVDLGINNWS; PEDMVVDLGINNWSV;
EDMVVDLGINNWSVL; DMVVDLGINNWSVLL; MVVDLGINNWSVLLT;
VVDLGINNWSVLLTP; VDLGINNWSVLLTPS; DLGINNWSVLLTPSA;
LGINNWSVLLTPSAR; GINNWSVLLTPSARL; INNWSVLLTPSARLQ;
NNWSVLLTPSARLQA; NWSVLLTPSARLQAY; WSVLLTPSARLQAYV;
SVLLTPSARLQAYVK; VLLTPSARLQAYVKN; LLTPSARLQAYVKNS;
LTPSARLQAYVKNSV; TPSARLQAYVKNSVV; PSARLQAYVKNSVVA;
SARLQAYVKNSVVAP; ARLQAYVKNSVVAPA; RLQAYVKNSVVAPAV;
LQAYVKNSVVAPAVV; QAYVKNSVVAPAVVK; AYVKNSVVAPAVVKS;
YVKNSVVAPAVVKSE; VKNSVVAPAVVKSES; KNSVVAPAVVKSESK;
NSVVAPAVVKSESKR; SVVAPAVVKSESKRY; VVAPAVVKSESKRYA;
VAPAVVKSESKRYAG; APAVVKSESKRYAGD; PAVVKSESKRYAGDT;
AVVKSESKRYAGDTI; VVKSESKRYAGDTIL; VKSESKRYAGDTILG;
KSESKRYAGDTILGV; SESKRYAGDTILGVR; ESKRYAGDTILGVRV;
SKRYAGDTILGVRVL; KRYAGDTILGVRVLF; RYAGDTILGVRVLFP;
YAGDTILGVRVLFPS; AGDTILGVRVLFPSY; GDTILGVRVLFPSYS;
DTILGVRVLFPSYSQ; TILGVRVLFPSYSQS; ILGVRVLFPSYSQSS;
LGVRVLFPSYSQSSA; GVRVLFPSYSQSSAM; VRVLFPSYSQSSAMI;
RVLFPSYSQSSAMIM; VLFPSYSQSSAMIMP; LFPSYSQSSAMIMPP;
FPSYSQSSAMIMPPF; PSYSQSSAMIMPPFK; SYSQSSAMIMPPFKI;
YSQSSAMIMPPFKIP; SQSSAMIMPPFKIPF; QSSAMIMPPFKIPFY;
SSAMIMPPFKIPFYS; SAMIMPPFKIPFYSG; AMIMPPFKIPFYSGE;
MIMPPFKIPFYSGES; IMPPFKIPFYSGESG; MPPFKIPFYSGESGN;
PPFKIPFYSGESGNQ; PFKIPFYSGESGNQF; FKIPFYSGESGNQFL;
KIPFYSGESGNQFLG; IPFYSGESGNQFLGK; PFYSGESGNQFLGKG;
FYSGESGNQFLGKGL; YSGESGNQFLGKGLI; SGESGNQFLGKGLID;
GESGNQFLGKGLIDN; ESGNQFLGKGLIDNI; SGNQFLGKGLIDNIK;
GNQFLGKGLIDNIKT; NQFLGKGLIDNIKTM; QFLGKGLIDNIKTMK;
FLGKGLIDNIKTMKE; LGKGLIDNIKTMKEI; GKGLIDNIKTMKEIK;
KGLIDNIKTMKEIKV; GLIDNIKTMKEIKVS; LIDNIKTMKEIKVSV;
IDNIKTMKEIKVSVY; DNIKTMKEIKVSVYS; NIKTMKEIKVSVYSL;
IKTMKEIKVSVYSLG; KTMKEIKVSVYSLGY; TMKEIKVSVYSLGYE;
MKEIKVSVYSLGYEI; KEIKVSVYSLGYEID; EIKVSVYSLGYEIDL;
IKVSVYSLGYEIDLE; KVSVYSLGYEIDLEV; VSVYSLGYEIDLEVL;
SVYSLGYEIDLEVLF; VYSLGYEIDLEVLFE; YSLGYEIDLEVLFED;

```
SLGYEIDLEVLFEDM;  LGYEIDLEVLFEDMN;  GYEIDLEVLFEDMNG;
YEIDLEVLFEDMNGM;  EIDLEVLFEDMNGME;  IDLEVLFEDMNGMEY;
DLEVLFEDMNGMEYA;  LEVLFEDMNGMEYAY;  EVLFEDMNGMEYAYS;
VLFEDMNGMEYAYSM;  LFEDMNGMEYAYSMG;  FEDMNGMEYAYSMGT;
EDMNGMEYAYSMGTL;  DMNGMEYAYSMGTLK;  MNGMEYAYSMGTLKF;
NGMEYAYSMGTLKFK;  GMEYAYSMGTLKFKG;  MEYAYSMGTLKFKGW;
EYAYSMGTLKFKGWA;  YAYSMGTLKFKGWAD;  AYSMGTLKFKGWADL;
YSMGTLKFKGWADLI;  SMGTLKFKGWADLIW;  MGTLKFKGWADLIWS;
GTLKFKGWADLIWSN;  TLKFKGWADLIWSNP;  LKFKGWADLIWSNPN;
KFKGWADLIWSNPNY;  FKGWADLIWSNPNYI;  KGWADLIWSNPNYIP;
GWADLIWSNPNYIPN;  WADLIWSNPNYIPNI;  ADLIWSNPNYIPNIS;
DLIWSNPNYIPNISS;  LIWSNPNYIPNISSR;  IWSNPNYIPNISSRI;
WSNPNYIPNISSRII;  SNPNYIPNISSRIIK;  NPNYIPNISSRIIKD;
PNYIPNISSRIIKDD;  NYIPNISSRIIKDDV;  YIPNISSRIIKDDVP;
IPNISSRIIKDDVPN;  PNISSRIIKDDVPNY;  NISSRIIKDDVPNYP;
ISSRIIKDDVPNYPL;  SSRIIKDDVPNYPLA;  SRIIKDDVPNYPLAS;
RIIKDDVPNYPLASS;  IIKDDVPNYPLASSK;  IKDDVPNYPLASSKM;
KDDVPNYPLASSKMR;  DDVPNYPLASSKMRF;  DVPNYPLASSKMRFK;
VPNYPLASSKMRFKA;  PNYPLASSKMRFKAF;  NYPLASSKMRFKAFR;
YPLASSKMRFKAFRV;  PLASSKMRFKAFRVS;  LASSKMRFKAFRVSK;
ASSKMRFKAFRVSKS;  SSKMRFKAFRVSKSH;  SKMRFKAFRVSKSHS;
KMRFKAFRVSKSHSS;  MRFKAFRVSKSHSSK;  RFKAFRVSKSHSSKV;
FKAFRVSKSHSSKVK;  KAFRVSKSHSSKVKN;  AFRVSKSHSSKVKNF;
FRVSKSHSSKVKNFI;  RVSKSHSSKVKNFIF;  VSKSHSSKVKNFIFY;
SKSHSSKVKNFIFYV;  KSHSSKVKNFIFYVK;  SHSSKVKNFIFYVKD;
HSSKVKNFIFYVKDL;  SSKVKNFIFYVKDLR;  SKVKNFIFYVKDLRV;
KVKNFIFYVKDLRVL;  VKNFIFYVKDLRVLY;  KNFIFYVKDLRVLYD;
NFIFYVKDLRVLYDK;  FIFYVKDLRVLYDKL;  IFYVKDLRVLYDKLS;
FYVKDLRVLYDKLSV;  YVKDLRVLYDKLSVS;  VKDLRVLYDKLSVSI;
KDLRVLYDKLSVSID;  DLRVLYDKLSVSIDS;  LRVLYDKLSVSIDSD;
RVLYDKLSVSIDSDI;  VLYDKLSVSIDSDID;  LYDKLSVSIDSDIDS;
YDKLSVSIDSDIDSE;  DKLSVSIDSDIDSES;  KLSVSIDSDIDSESV;
LSVSIDSDIDSESVF;  SVSIDSDIDSESVFK;  VSIDSDIDSESVFKV;
SIDSDIDSESVFKVY;  IDSDIDSESVFKVYE;  DSDIDSESVFKVYET;
SDIDSESVFKVYETS;  DIDSESVFKVYETSG;  IDSESVFKVYETSGT;
DSESVFKVYETSGTE;  SESVFKVYETSGTES;  ESVFKVYETSGTESL;
SVFKVYETSGTESLR;  VFKVYETSGTESLRK;  FKVYETSGTESLRKL;
KVYETSGTESLRKLK;  VYETSGTESLRKLKA;  YETSGTESLRKLKAH;
ETSGTESLRKLKAHE;  TSGTESLRKLKAHET;  SGTESLRKLKAHETF;
GTESLRKLKAHETFK;  TESLRKLKAHETFKR;  ESLRKLKAHETFKRV;
SLRKLKAHETFKRVL;  LRKLKAHETFKRVLK;  RKLKAHETFKRVLKL;
KLKAHETFKRVLKLR;  LKAHETFKRVLKLRE;  KAHETFKRVLKLREK;
AHETFKRVLKLREKI;  HETFKRVLKLREKIS;  ETFKRVLKLREKISI;
TFKRVLKLREKISIA;  FKRVLKLREKISIAE;  KRVLKLREKISIAEG;
RVLKLREKISIAEGS;  VLKLREKISIAEGSF;  LKLREKISIAEGSFQ;
KLREKISIAEGSFQN;  LREKISIAEGSFQNF;  REKISIAEGSFQNFV;
EKISIAEGSFQNFVE;  KISIAEGSFQNFVEK;  ISIAEGSFQNFVEKI;
SIAEGSFQNFVEKIE;  IAEGSFQNFVEKIES;  AEGSFQNFVEKIESE;
EGSFQNFVEKIESEK;  GSFQNFVEKIESEKP;  SFQNFVEKIESEKPE;
FQNFVEKIESEKPEE;  QNFVEKIESEKPEES;  NFVEKIESEKPEESS;
FVEKIESEKPEESSP;  VEKIESEKPEESSPK;  EKIESEKPEESSPKN;

16 mers:
MKRKAKSILFFLLSTV;  KRKAKSILFFLLSTVL;  RKAKSILFFLLSTVLF;
KAKSILFFLLSTVLFA;  AKSILFFLLSTVLFAQ;  KSILFFLLSTVLFAQE;
SILFFLLSTVLFAQET;  ILFFLLSTVLFAQETD;  LFFLLSTVLFAQETDG;
FFLLSTVLFAQETDGL;  FLLSTVLFAQETDGLA;  LLSTVLFAQETDGLAE;
```

| | | |
|---|---|---|
| LSTVLFAQETDGLAEG; | STVLFAQETDGLAEGS; | TVLFAQETDGLAEGSK; |
| VLFAQETDGLAEGSKR; | LFAQETDGLAEGSKRA; | FAQETDGLAEGSKRAE; |
| AQETDGLAEGSKRAEP; | QETDGLAEGSKRAEPG; | ETDGLAEGSKRAEPGE; |
| TDGLAEGSKRAEPGEL; | DGLAEGSKRAEPGELV; | GLAEGSKRAEPGELVL; |
| LAEGSKRAEPGELVLD; | AEGSKRAEPGELVLDF; | EGSKRAEPGELVLDFA; |
| GSKRAEPGELVLDFAE; | SKRAEPGELVLDFAEL; | KRAEPGELVLDFAELA; |
| RAEPGELVLDFAELAR; | AEPGELVLDFAELARD; | EPGELVLDFAELARDP; |
| PGELVLDFAELARDPS; | GELVLDFAELARDPSS; | ELVLDFAELARDPSST; |
| LVLDFAELARDPSSTR; | VLDFAELARDPSSTRL; | LDFAELARDPSSTRLD; |
| DFAELARDPSSTRLDL; | FAELARDPSSTRLDLT; | AELARDPSSTRLDLTN; |
| ELARDPSSTRLDLTNY; | LARDPSSTRLDLTNYV; | ARDPSSTRLDLTNYVD; |
| RDPSSTRLDLTNYVDY; | DPSSTRLDLTNYVDYV; | PSSTRLDLTNYVDYVY; |
| SSTRLDLTNYVDYVYS; | STRLDLTNYVDYVYSG; | TRLDLTNYVDYVYSGA; |
| RLDLTNYVDYVYSGAS; | LDLTNYVDYVYSGASG; | DLTNYVDYVYSGASGI; |
| LTNYVDYVYSGASGIV; | TNYVDYVYSGASGIVK; | NYVDYVYSGASGIVKP; |
| YVDYVYSGASGIVKPE; | VDYVYSGASGIVKPED; | DYVYSGASGIVKPEDM; |
| YVVSGASGIVKPEDMV; | VYSGASGIVKPEDMVV; | YSGASGIVKPEDMVVD; |
| SGASGIVKPEDMVVDL; | GASGIVKPEDMVVDLG; | ASGIVKPEDMVVDLGI; |
| SGIVKPEDMVVDLGIN; | GIVKPEDMVVDLGINN; | IVKPEDMVVDLGINNW; |
| VKPEDMVVDLGINNWS; | KPEDMVVDLGINNWSV; | PEDMVVDLGINNWSVL; |
| EDMVVDLGINNWSVLL; | DMVVDLGINNWSVLLT; | MVVDLGINNWSVLLTP; |
| VVDLGINNWSVLLTPS; | VDLGINNWSVLLTPSA; | DLGINNWSVLLTPSAR; |
| LGINNWSVLLTPSARL; | GINNWSVLLTPSARLQ; | INNWSVLLTPSARLQA; |
| NNWSVLLTPSARLQAY; | NWSVLLTPSARLQAYV; | WSVLLTPSARLQAYVK; |
| SVLLTPSARLQAYVKN; | VLLTPSARLQAYVKNS; | LLTPSARLQAYVKNSV; |
| LTPSARLQAYVKNSVV; | TPSARLQAYVKNSVVA; | PSARLQAYVKNSVVAP; |
| SARLQAYVKNSVVAPA; | ARLQAYVKNSVVAPAV; | RLQAYVKNSVVAPAVV; |
| LQAYVKNSVVAPAVVK; | QAYVKNSVVAPAVVKS; | AYVKNSVVAPAVVKSE; |
| YVKNSVVAPAVVKSES; | VKNSVVAPAVVKSESK; | KNSVVAPAVVKSESKR; |
| NSVVAPAVVKSESKRY; | SVVAPAVVKSESKRYA; | VVAPAVVKSESKRYAG; |
| VAPAVVKSESKRYAGD; | APAVVKSESKRYAGDT; | PAVVKSESKRYAGDTI; |
| AVVKSESKRYAGDTIL; | VVKSESKRYAGDTILG; | VKSESKRYAGDTILGV; |
| KSESKRYAGDTILGVR; | SESKRYAGDTILGVRV; | ESKRYAGDTILGVRVL; |
| SKRYAGDTILGVRVLF; | KRYAGDTILGVRVLFP; | RYAGDTILGVRVLFPS; |
| YAGDTILGVRVLFPSY; | AGDTILGVRVLFPSYS; | GDTILGVRVLFPSYSQ; |
| DTILGVRVLFPSYSQS; | TILGVRVLFPSYSQSS; | ILGVRVLFPSYSQSSA; |
| LGVRVLFPSYSQSSAM; | GVRVLFPSYSQSSAMI; | VRVLFPSYSQSSAMIM; |
| RVLFPSYSQSSAMIMP; | VLFPSYSQSSAMIMPP; | LFPSYSQSSAMIMPPF; |
| FPSYSQSSAMIMPPFK; | PSYSQSSAMIMPPFKI; | SYSQSSAMIMPPFKIP; |
| YSQSSAMIMPPFKIPF; | SQSSAMIMPPFKIPFY; | QSSAMIMPPFKIPFYS; |
| SSAMIMPPFKIPFYSG; | SAMIMPPFKIPFYSGE; | AMIMPPFKIPFYSGES; |
| MIMPPFKIPFYSGESG; | IMPPFKIPFYSGESGN; | MPPFKIPFYSGESGNQ; |
| PPFKIPFYSGESGNQF; | PFKIPFYSGESGNQFL; | FKIPFYSGESGNQFLG; |
| KIPFYSGESGNQFLGK; | IPFYSGESGNQFLGKG; | PFYSGESGNQFLGKGL; |
| FYSGESGNQFLGKGLI; | YSGESGNQFLGKGLID; | SGESGNQFLGKGLIDN; |
| GESGNQFLGKGLIDNI; | ESGNQFLGKGLIDNIK; | SGNQFLGKGLIDNIKT; |
| GNQFLGKGLIDNIKTM; | NQFLGKGLIDNIKTMK; | QFLGKGLIDNIKTMKE; |
| FLGKGLIDNIKTMKEI; | LGKGLIDNIKTMKEIK; | GKGLIDNIKTMKEIKV; |
| KGLIDNIKTMKEIKVS; | GLIDNIKTMKEIKVSV; | LIDNIKTMKEIKVSVY; |
| IDNIKTMKEIKVSVYS; | DNIKTMKEIKVSVYSL; | NIKTMKEIKVSVYSLG; |
| IKTMKEIKVSVYSLGY; | KTMKEIKVSVYSLGYE; | TMKEIKVSVYSLGYEI; |
| MKEIKVSVYSLGYEID; | KEIKVSVYSLGYEIDL; | EIKVSVYSLGYEIDLE; |
| IKVSVYSLGYEIDLEV; | KVSVYSLGYEIDLEVL; | VSVYSLGYEIDLEVLF; |
| SVYSLGYEIDLEVLFE; | VYSLGYEIDLEVLFED; | YSLGYEIDLEVLFEDM; |
| SLGYEIDLEVLFEDMN; | LGYEIDLEVLFEDMNG; | GYEIDLEVLFEDMNGM; |
| YEIDLEVLFEDMNGME; | EIDLEVLFEDMNGMEY; | IDLEVLFEDMNGMEYA; |
| DLEVLFEDMNGMEYAY; | LEVLFEDMNGMEYAYS; | EVLFEDMNGMEYAYSM; |

| | |
|---|---|
| | VLFEDMNGMEYAYSMG; LFEDMNGMEYAYSMGT; FEDMNGMEYAYSMGTL; EDMNGMEYAYSMGTLK; DMNGMEYAYSMGTLKF; MNGMEYAYSMGTLKFK; NGMEYAYSMGTLKFKG; GMEYAYSMGTLKFKGW; MEYAYSMGTLKFKGWA; EYAYSMGTLKFKGWAD; YAYSMGTLKFKGWADL; AYSMGTLKFKGWADLI; YSMGTLKFKGWADLIW; SMGTLKFKGWADLIWS; MGTLKFKGWADLIWSN; GTLKFKGWADLIWSNP; TLKFKGWADLIWSNPN; LKFKGWADLIWSNPNY; KFKGWADLIWSNPNYI; FKGWADLIWSNPNYIP; KGWADLIWSNPNYIPN; GWADLIWSNPNYIPNI; WADLIWSNPNYIPNIS; ADLIWSNPNYIPNISS; DLIWSNPNYIPNISSR; LIWSNPNYIPNISSRI; IWSNPNYIPNISSRII; WSNPNYIPNISSRIIK; SNPNYIPNISSRIIKD; NPNYIPNISSRIIKDD; PNYIPNISSRIIKDDV; NYIPNISSRIIKDDVP; YIPNISSRIIKDDVPN; IPNISSRIIKDDVPNY; PNISSRIIKDDVPNYP; NISSRIIKDDVPNYPL; ISSRIIKDDVPNYPLA; SSRIIKDDVPNYPLAS; SRIIKDDVPNYPLASS; RIIKDDVPNYPLASSK; IIKDDVPNYPLASSKM; IKDDVPNYPLASSKMR; KDDVPNYPLASSKMRF; DDVPNYPLASSKMRFK; DVPNYPLASSKMRFKA; VPNYPLASSKMRFKAF; PNYPLASSKMRFKAFR; NYPLASSKMRFKAFRV; YPLASSKMRFKAFRVS; PLASSKMRFKAFRVSK; LASSKMRFKAFRVSKS; ASSKMRFKAFRVSKSH; SSKMRFKAFRVSKSHS; SKMRFKAFRVSKSHSS; KMRFKAFRVSKSHSSK; MRFKAFRVSKSHSSKV; RFKAFRVSKSHSSKVK; FKAFRVSKSHSSKVKN; KAFRVSKSHSSKVKNF; AFRVSKSHSSKVKNFI; FRVSKSHSSKVKNFIF; RVSKSHSSKVKNFIFY; VSKSHSSKVKNFIFYV; SKSHSSKVKNFIFYVK; KSHSSKVKNFIFYVKD; SHSSKVKNFIFYVKDL; HSSKVKNFIFYVKDLR; SSKVKNFIFYVKDLRV; SKVKNFIFYVKDLRVL; KVKNFIFYVKDLRVLY; VKNFIFYVKDLRVLYD; KNFIFYVKDLRVLYDK; NFIFYVKDLRVLYDKL; FIFYVKDLRVLYDKLS; IFYVKDLRVLYDKLSV; FYVKDLRVLYDKLSVS; YVKDLRVLYDKLSVSI; VKDLRVLYDKLSVSID; KDLRVLYDKLSVSIDS; DLRVLYDKLSVSIDSD; LRVLYDKLSVSIDSDI; RVLYDKLSVSIDSDID; VLYDKLSVSIDSDIDS; LYDKLSVSIDSDIDSE; YDKLSVSIDSDIDSES; DKLSVSIDSDIDSESV; KLSVSIDSDIDSESVF; LSVSIDSDIDSESVFK; SVSIDSDIDSESVFKV; VSIDSDIDSESVFKVY; SIDSDIDSESVFKVYE; IDSDIDSESVFKVYET; DSDIDSESVFKVYETS; SDIDSESVFKVYETSG; DIDSESVFKVYETSGT; IDSESVFKVYETSGTE; DSESVFKVYETSGTES; SESVFKVYETSGTESL; ESVFKVYETSGTESLR; SVFKVYETSGTESLRK; VFKVYETSGTESLRKL; FKVYETSGTESLRKLK; KVYETSGTESLRKLKA; VYETSGTESLRKLKAH; YETSGTESLRKLKAHE; ETSGTESLRKLKAHET; TSGTESLRKLKAHETF; SGTESLRKLKAHETFK; GTESLRKLKAHETFKR; TESLRKLKAHETFKRV; ESLRKLKAHETFKRVL; SLRKLKAHETFKRVLK; LRKLKAHETFKRVLKL; RKLKAHETFKRVLKLR; KLKAHETFKRVLKLRE; LKAHETFKRVLKLREK; KAHETFKRVLKLREKI; AHETFKRVLKLREKIS; HETFKRVLKLREKISI; ETFKRVLKLREKISIA; TFKRVLKLREKISIAE; FKRVLKLREKISIAEG; KRVLKLREKISIAEGS; RVLKLREKISIAEGSF; VLKLREKISIAEGSFQ; LKLREKISIAEGSFQN; KLREKISIAEGSFQNF; LREKISIAEGSFQNFV; REKISIAEGSFQNFVE; EKISIAEGSFQNFVEK; KISIAEGSFQNFVEKI; ISIAEGSFQNFVEKIE; SIAEGSFQNFVEKIES; IAEGSFQNFVEKIESE; AEGSFQNFVEKIESEK; EGSFQNFVEKIESEKP; GSFQNFVEKIESEKPE; SFQNFVEKIESEKPEE; FQNFVEKIESEKPEES; QNFVEKIESEKPEESS; NFVEKIESEKPEESSP; FVEKIESEKPEESSPK; VEKIESEKPEESSPKN; |
| AAU07005.1\| flagellar filament 41 kDa core protein [Borrelia garinii PBi] | 13 mers: MIINHNTSAINAS; IINHNTSAINASR; INHNTSAINASRN; NHNTSAINASRNN; HNTSAINASRNNS; NTSAINASRNNSI; TSAINASRNNSIN; SAINASRNNSINA; AINASRNNSINAA; INASRNNSINAAN; NASRNNSINAANL; ASRNNSINAANLS; SRNNSINAANLSK; RNNSINAANLSKT; NNSINAANLSKTQ; NSINAANLSKTQE; SINAANLSKTQEK; INAANLSKTQEKL; NAANLSKTQEKLS; AANLSKTQEKLSS; ANLSKTQEKLSSG; NLSKTQEKLSSGY; LSKTQEKLSSGYR; SKTQEKLSSGYRI; |

| | |
|---|---|
| KTQEKLSSGYRIN; TQEKLSSGYRINR; QEKLSSGYRINRA; EKLSSGYRINRAS; KLSSGYRINRASD; LSSGYRINRASDD; SSGYRINRASDDA; SGYRINRASDDAA; GYRINRASDDAAG; YRINRASDDAAGM; RINRASDDAAGMG; INRASDDAAGMGV; NRASDDAAGMGVS; RASDDAAGMGVSG; ASDDAAGMGVSGK; SDDAAGMGVSGKI; DDAAGMGVSGKIN; DAAGMGVSGKINA; AAGMGVSGKINAQ; AGMGVSGKINAQI; GMGVSGKINAQIR; MGVSGKINAQIRG; GVSGKINAQIRGL; VSGKINAQIRGLS; SGKINAQIRGLSQ; GKINAQIRGLSQA; KINAQIRGLSQAS; INAQIRGLSQASR; NAQIRGLSQASRN; AQIRGLSQASRNT; QIRGLSQASRNTS; IRGLSQASRNTSK; RGLSQASRNTSKA; GLSQASRNTSKAI; LSQASRNTSKAIN; SQASRNTSKAINF; QASRNTSKAINFI; ASRNTSKAINFIQ; SRNTSKAINFIQT; RNTSKAINFIQTT; NTSKAINFIQTTE; TSKAINFIQTTEG; SKAINFIQTTEGN; KAINFIQTTEGNL; AINFIQTTEGNLN; INFIQTTEGNLNE; NFIQTTEGNLNEV; FIQTTEGNLNEVE; IQTTEGNLNEVEK; QTTEGNLNEVEKV; TTEGNLNEVEKVL; TEGNLNEVEKVLV; EGNLNEVEKVLVR; GNLNEVEKVLVRM; NLNEVEKVLVRMK; LNEVEKVLVRMKE; NEVEKVLVRMKEL; EVEKVLVRMKELA; VEKVLVRMKELAV; EKVLVRMKELAVQ; KVLVRMKELAVQS; VLVRMKELAVQSG; LVRMKELAVQSGN; VRMKELAVQSGNG; RMKELAVQSGNGT; MKELAVQSGNGTY; KELAVQSGNGTYS; ELAVQSGNGTYSD; LAVQSGNGTYSDA; AVQSGNGTYSDAD; VQSGNGTYSDADR; QSGNGTYSDADRG; SGNGTYSDADRGS; GNGTYSDADRGSI; NGTYSDADRGSIQ; GTYSDADRGSIQI; TYSDADRGSIQIE; YSDADRGSIQIEI; SDADRGSIQIEIE; DADRGSIQIEIEQ; ADRGSIQIEIEQL; DRGSIQIEIEQLT; RGSIQIEIEQLTD; GSIQIEIEQLTDE; SIQIEIEQLTDEI; IQIEIEQLTDEIN; QIEIEQLTDEINR; IEIEQLTDEINRI; EIEQLTDEINRIA; IEQLTDEINRIAD; EQLTDEINRIADQ; QLTDEINRIADQA; LTDEINRIADQAQ; TDEINRIADQAQY; DEINRIADQAQYN; EINRIADQAQYNQ; INRIADQAQYNQM; NRIADQAQYNQMH; RIADQAQYNQMHM; IADQAQYNQMHML; ADQAQYNQMHMLS; DQAQYNQMHMLSN; QAQYNQMHMLSNK; AQYNQMHMLSNKS; QYNQMHMLSNKSA; YNQMHMLSNKSAS; NQMHMLSNKSASQ; QMHMLSNKSASQN; MHMLSNKSASQNV; HMLSNKSASQNVR; MLSNKSASQNVRT; LSNKSASQNVRTA; SNKSASQNVRTAE; NKSASQNVRTAEE; KSASQNVRTAEEL; SASQNVRTAEELG; ASQNVRTAEELGM; SQNVRTAEELGMQ; QNVRTAEELGMQP; NVRTAEELGMQPA; VRTAEELGMQPAK; RTAEELGMQPAKI; TAEELGMQPAKIN; AEELGMQPAKINT; EELGMQPAKINTP; ELGMQPAKINTPA; LGMQPAKINTPAS; GMQPAKINTPASL; MQPAKINTPASLS; QPAKINTPASLSG; PAKINTPASLSGS; AKINTPASLSGSQ; KINTPASLSGSQA; INTPASLSGSQAS; NTPASLSGSQASW; TPASLSGSQASWT; PASLSGSQASWTL; ASLSGSQASWTLR; SLSGSQASWTLRV; LSGSQASWTLRVH; SGSQASWTLRVHV; GSQASWTLRVHVG; SQASWTLRVHVGA; QASWTLRVHVGAN; ASWTLRVHVGANQ; SWTLRVHVGANQD; WTLRVHVGANQDE; TLRVHVGANQDEA; LRVHVGANQDEAI; RVHVGANQDEAIA; VHVGANQDEAIAV; HVGANQDEAIAVN; VGANQDEAIAVNI; GANQDEAIAVNIY; ANQDEAIAVNIYA; NQDEAIAVNIYAA; QDEAIAVNIYAAN; DEAIAVNIYAANV; EAIAVNIYAANVA; AIAVNIYAANVAN; IAVNIYAANVANL; AVNIYAANVANLF; VNIYAANVANLFS; NIYAANVANLFSG; IYAANVANLFSGE; YAANVANLFSGEG; AANVANLFSGEGS; ANVANLFSGEGSQ; NVANLFSGEGSQA; VANLFSGEGSQAA; ANLFSGEGSQAAQ; NLFSGEGSQAAQT; LFSGEGSQAAQTA; FSGEGSQAAQTAP; SGEGSQAAQTAPV; GEGSQAAQTAPVQ; EGSQAAQTAPVQE; GSQAAQTAPVQEG; SQAAQTAPVQEGA; QAAQTAPVQEGAQ; AAQTAPVQEGAQQ; AQTAPVQEGAQQE; QTAPVQEGAQQEG; TAPVQEGAQQEGA; APVQEGAQQEGAQ; PVQEGAQQEGAQQ; VQEGAQQEGAQQP; QEGAQQEGAQQPA; EGAQQEGAQQPAP; GAQQEGAQQPAPA; AQQEGAQQPAPAT; QQEGAQQPAPATA; QEGAQQPAPATAP; EGAQQPAPATAPS; GAQQPAPATAPSQ; AQQPAPATAPSQG; QQPAPATAPSQGG; QPAPATAPSQGGV; PAPATAPSQGGVN; APATAPSQGGVNS; PATAPSQGGVNSP; ATAPSQGGVNSPV; TAPSQGGVNSPVN; APSQGGVNSPVNV; PSQGGVNSPVNVT; SQGGVNSPVNVTT; QGGVNSPVNVTTT; GGVNSPVNVTTTV; GVNSPVNVTTTVD; VNSPVNVTTTVDA; NSPVNVTTTVDAN; SPVNVTTTVDANT; PVNVTTTVDANTS; VNVTTTVDANTSL; NVTTTVDANTSLA; VTTTVDANTSLAK; TTTVDANTSLAKI; TTVDANTSLAKIE; TVDANTSLAKIEN; VDANTSLAKIENA; DANTSLAKIENAI; ANTSLAKIENAIR; NTSLAKIENAIRM; TSLAKIENAIRMI; SLAKIENAIRMIS; LAKIENAIRMISD; AKIENAIRMISDQ; KIENAIRMISDQR; |

IENAIRMISDQRA; ENAIRMISDQRAN; NAIRMISDQRANL; AIRMISDQRANLG;
IRMISDQRANLGA; RMISDQRANLGAF; MISDQRANLGAFQ; ISDQRANLGAFQN;
SDQRANLGAFQNR; DQRANLGAFQNRL; QRANLGAFQNRLE; RANLGAFQNRLES;
ANLGAFQNRLESI; NLGAFQNRLESIK; LGAFQNRLESIKD; GAFQNRLESIKDS;
AFQNRLESIKDST; FQNRLESIKDSTE; QNRLESIKDSTEY; NRLESIKDSTEYA;
RLESIKDSTEYAI; LESIKDSTEYAIE; ESIKDSTEYAIEN; SIKDSTEYAIENL;
IKDSTEYAIENLK; KDSTEYAIENLKA; DSTEYAIENLKAS; STEYAIENLKASY;
TEYAIENLKASYA; EYAIENLKASYAQ; YAIENLKASYAQI; AIENLKASYAQIK;
IENLKASYAQIKD; ENLKASYAQIKDA; NLKASYAQIKDAT; LKASYAQIKDATM;
KASYAQIKDATMT; ASYAQIKDATMTD; SYAQIKDATMTDE; YAQIKDATMTDEV;
AQIKDATMTDEVV; QIKDATMTDEVVA; IKDATMTDEVVAS; KDATMTDEVVAST;
DATMTDEVVASTT; ATMTDEVVASTTN; TMTDEVVASTTNS; MTDEVVASTTNSI;
TDEVVASTTNSIL; DEVVASTTNSILT; EVVASTTNSILTQ; VVASTTNSILTQS;
VASTTNSILTQSA; ASTTNSILTQSAM; STTNSILTQSAMA; TTNSILTQSAMAM;
TNSILTQSAMAMI; NSILTQSAMAMIA; SILTQSAMAMIAQ; ILTQSAMAMIAQA;
LTQSAMAMIAQAN; TQSAMAMIAQANQ; QSAMAMIAQANQV; SAMAMIAQANQVP;
AMAMIAQANQVPQ; MAMIAQANQVPQY; AMIAQANQVPQYV; MIAQANQVPQYVL;
IAQANQVPQYVLS; AQANQVPQYVLSL; QANQVPQYVLSLL; ANQVPQYVLSLLR;

14 mers:
MIINHNTSAINASR; IINHNTSAINASRN; INHNTSAINASRNN;
NHNTSAINASRNNS; HNTSAINASRNNSI; NTSAINASRNNSIN;
TSAINASRNNSINA; SAINASRNNSINAA; AINASRNNSINAAN;
INASRNNSINAANL; NASRNNSINAANLS; ASRNNSINAANLSK;
SRNNSINAANLSKT; RNNSINAANLSKTQ; NNSINAANLSKTQE;
NSINAANLSKTQEK; SINAANLSKTQEKL; INAANLSKTQEKLS;
NAANLSKTQEKLSS; AANLSKTQEKLSSG; ANLSKTQEKLSSGY;
NLSKTQEKLSSGYR; LSKTQEKLSSGYRI; SKTQEKLSSGYRIN;
KTQEKLSSGYRINR; TQEKLSSGYRINRA; QEKLSSGYRINRAS;
EKLSSGYRINRASD; KLSSGYRINRASDD; LSSGYRINRASDDA;
SSGYRINRASDDAA; SGYRINRASDDAAG; GYRINRASDDAAGM;
YRINRASDDAAGMG; RINRASDDAAGMGV; INRASDDAAGMGVS;
NRASDDAAGMGVSG; RASDDAAGMGVSGK; ASDDAAGMGVSGKI;
SDDAAGMGVSGKIN; DDAAGMGVSGKINA; DAAGMGVSGKINAQ;
AAGMGVSGKINAQI; AGMGVSGKINAQIR; GMGVSGKINAQIRG;
MGVSGKINAQIRGL; GVSGKINAQIRGLS; VSGKINAQIRGLSQ;
SGKINAQIRGLSQA; GKINAQIRGLSQAS; KINAQIRGLSQASR;
INAQIRGLSQASRN; NAQIRGLSQASRNT; AQIRGLSQASRNTS;
QIRGLSQASRNTSK; IRGLSQASRNTSKA; RGLSQASRNTSKAI;
GLSQASRNTSKAIN; LSQASRNTSKAINF; SQASRNTSKAINFI;
QASRNTSKAINFIQ; ASRNTSKAINFIQT; SRNTSKAINFIQTT;
RNTSKAINFIQTTE; NTSKAINFIQTTEG; TSKAINFIQTTEGN;
SKAINFIQTTEGNL; KAINFIQTTEGNLN; AINFIQTTEGNLNE;
INFIQTTEGNLNEV; NFIQTTEGNLNEVE; FIQTTEGNLNEVEK;
IQTTEGNLNEVEKV; QTTEGNLNEVEKVL; TTEGNLNEVEKVLV;
TEGNLNEVEKVLVR; EGNLNEVEKVLVRM; GNLNEVEKVLVRMK;
NLNEVEKVLVRMKE; LNEVEKVLVRMKEL; NEVEKVLVRMKELA;
EVEKVLVRMKELAV; VEKVLVRMKELAVQ; EKVLVRMKELAVQS;
KVLVRMKELAVQSG; VLVRMKELAVQSGN; LVRMKELAVQSGNG;
VRMKELAVQSGNGT; RMKELAVQSGNGTY; MKELAVQSGNGTYS;
KELAVQSGNGTYSD; ELAVQSGNGTYSDA; LAVQSGNGTYSDAD;
AVQSGNGTYSDADR; VQSGNGTYSDADRG; QSGNGTYSDADRGS;
SGNGTYSDADRGSI; GNGTYSDADRGSIQ; NGTYSDADRGSIQI;
GTYSDADRGSIQIE; TYSDADRGSIQIEI; YSDADRGSIQIEIE;
SDADRGSIQIEIEQ; DADRGSIQIEIEQL; ADRGSIQIEIEQLT;
DRGSIQIEIEQLTD; RGSIQIEIEQLTDE; GSIQIEIEQLTDEI;
SIQIEIEQLTDEIN; IQIEIEQLTDEINR; QIEIEQLTDEINRI;

| | |
|---|---|
| IEIEQLTDEINRIA; EIEQLTDEINRIAD; IEQLTDEINRIADQ;<br>EQLTDEINRIADQA; QLTDEINRIADQAQ; LTDEINRIADQAQY;<br>TDEINRIADQAQYN; DEINRIADQAQYNQ; EINRIADQAQYNQM;<br>INRIADQAQYNQMH; NRIADQAQYNQMHM; RIADQAQYNQMHML;<br>IADQAQYNQMHMLS; ADQAQYNQMHMLSN; DQAQYNQMHMLSNK;<br>QAQYNQMHMLSNKS; AQYNQMHMLSNKSA; QYNQMHMLSNKSAS;<br>YNQMHMLSNKSASQ; NQMHMLSNKSASQN; QMHMLSNKSASQNV;<br>MHMLSNKSASQNVR; HMLSNKSASQNVRT; MLSNKSASQNVRTA;<br>LSNKSASQNVRTAE; SNKSASQNVRTAEE; NKSASQNVRTAEEL;<br>KSASQNVRTAEELG; SASQNVRTAEELGM; ASQNVRTAEELGMQ;<br>SQNVRTAEELGMQP; QNVRTAEELGMQPA; NVRTAEELGMQPAK;<br>VRTAEELGMQPAKI; RTAEELGMQPAKIN; TAEELGMQPAKINT;<br>AEELGMQPAKINTP; EELGMQPAKINTPA; ELGMQPAKINTPAS;<br>LGMQPAKINTPASL; GMQPAKINTPASLS; MQPAKINTPASLSG;<br>QPAKINTPASLSGS; PAKINTPASLSGSQ; AKINTPASLSGSQA;<br>KINTPASLSGSQAS; INTPASLSGSQASW; NTPASLSGSQASWT;<br>TPASLSGSQASWTL; PASLSGSQASWTLR; ASLSGSQASWTLRV;<br>SLSGSQASWTLRVH; LSGSQASWTLRVHV; SGSQASWTLRVHVG;<br>GSQASWTLRVHVGA; SQASWTLRVHVGAN; QASWTLRVHVGANQ;<br>ASWTLRVHVGANQD; SWTLRVHVGANQDE; WTLRVHVGANQDEA;<br>TLRVHVGANQDEAI; LRVHVGANQDEAIA; RVHVGANQDEAIAV;<br>VHVGANQDEAIAVN; HVGANQDEAIAVNI; VGANQDEAIAVNIY;<br>GANQDEAIAVNIYA; ANQDEAIAVNIYAA; NQDEAIAVNIYAAN;<br>QDEAIAVNIYAANV; DEAIAVNIYAANVA; EAIAVNIYAANVAN;<br>AIAVNIYAANVANL; IAVNIYAANVANLF; AVNIYAANVANLFS;<br>VNIYAANVANLFSG; NIYAANVANLFSGE; IYAANVANLFSGEG;<br>YAANVANLFSGEGS; AANVANLFSGEGSQ; ANVANLFSGEGSQA;<br>NVANLFSGEGSQAA; VANLFSGEGSQAAQ; ANLFSGEGSQAAQT;<br>NLFSGEGSQAAQTA; LFSGEGSQAAQTAP; FSGEGSQAAQTAPV;<br>SGEGSQAAQTAPVQ; GEGSQAAQTAPVQE; EGSQAAQTAPVQEG;<br>GSQAAQTAPVQEGA; SQAAQTAPVQEGAQ; QAAQTAPVQEGAQQ;<br>AAQTAPVQEGAQQE; AQTAPVQEGAQQEG; QTAPVQEGAQQEGA;<br>TAPVQEGAQQEGAQ; APVQEGAQQEGAQQ; PVQEGAQQEGAQQP;<br>VQEGAQQEGAQQPA; QEGAQQEGAQQPAP; EGAQQEGAQQPAPA;<br>GAQQEGAQQPAPAT; AQQEGAQQPAPATA; QQEGAQQPAPATAP;<br>QEGAQQPAPATAPS; EGAQQPAPATAPSQ; GAQQPAPATAPSQG;<br>AQQPAPATAPSQGG; QQPAPATAPSQGGV; QPAPATAPSQGGVN;<br>PAPATAPSQGGVNS; APATAPSQGGVNSP; PATAPSQGGVNSPV;<br>ATAPSQGGVNSPVN; TAPSQGGVNSPVNV; APSQGGVNSPVNVT;<br>PSQGGVNSPVNVTT; SQGGVNSPVNVTTT; QGGVNSPVNVTTTV;<br>GGVNSPVNVTTTVD; GVNSPVNVTTTVDA; VNSPVNVTTTVDAN;<br>NSPVNVTTTVDANT; SPVNVTTTVDANTS; PVNVTTTVDANTSL;<br>VNVTTTVDANTSLA; NVTTTVDANTSLAK; VTTTVDANTSLAKI;<br>TTTVDANTSLAKIE; TTVDANTSLAKIEN; TVDANTSLAKIENA;<br>VDANTSLAKIENAI; DANTSLAKIENAIR; ANTSLAKIENAIRM;<br>NTSLAKIENAIRMI; TSLAKIENAIRMIS; SLAKIENAIRMISD;<br>LAKIENAIRMISDQ; AKIENAIRMISDQR; KIENAIRMISDQRA;<br>IENAIRMISDQRAN; ENAIRMISDQRANL; NAIRMISDQRANLG;<br>AIRMISDQRANLGA; IRMISDQRANLGAF; RMISDQRANLGAFQ;<br>MISDQRANLGAFQN; ISDQRANLGAFQNR; SDQRANLGAFQNRL;<br>DQRANLGAFQNRLE; QRANLGAFQNRLES; RANLGAFQNRLESI;<br>ANLGAFQNRLESIK; NLGAFQNRLESIKD; LGAFQNRLESIKDS;<br>GAFQNRLESIKDST; AFQNRLESIKDSTE; FQNRLESIKDSTEY;<br>QNRLESIKDSTEYA; NRLESIKDSTEYAI; RLESIKDSTEYAIE;<br>LESIKDSTEYAIEN; ESIKDSTEYAIENL; SIKDSTEYAIENLK;<br>IKDSTEYAIENLKA; KDSTEYAIENLKAS; DSTEYAIENLKASY;<br>STEYAIENLKASYA; TEYAIENLKASYAQ; EYAIENLKASYAQI; |

YAIENLKASYAQIK; AIENLKASYAQIKD; IENLKASYAQIKDA;
ENLKASYAQIKDAT; NLKASYAQIKDATM; LKASYAQIKDATMT;
KASYAQIKDATMTD; ASYAQIKDATMTDE; SYAQIKDATMTDEV;
YAQIKDATMTDEVV; AQIKDATMTDEVVA; QIKDATMTDEVVAS;
IKDATMTDEVVAST; KDATMTDEVVASTT; DATMTDEVVASTTN;
ATMTDEVVASTTNS; TMTDEVVASTTNSI; MTDEVVASTTNSIL;
TDEVVASTTNSILT; DEVVASTTNSILTQ; EVVASTTNSILTQS;
VVASTTNSILTQSA; VASTTNSILTQSAM; ASTTNSILTQSAMA;
STTNSILTQSAMAM; TTNSILTQSAMAMI; TNSILTQSAMAMIA;
NSILTQSAMAMIAQ; SILTQSAMAMIAQA; ILTQSAMAMIAQAN;
LTQSAMAMIAQANQ; TQSAMAMIAQANQV; QSAMAMIAQANQVP;
SAMAMIAQANQVPQ; AMAMIAQANQVPQY; MAMIAQANQVPQYV;
AMIAQANQVPQYVL; MIAQANQVPQYVLS; IAQANQVPQYVLSL;
AQANQVPQYVLSLL; QANQVPQYVLSLLR;

15 mers:
MIINHNTSAINASRN; IINHNTSAINASRNN; INHNTSAINASRNNS;
NHNTSAINASRNNSI; HNTSAINASRNNSIN; NTSAINASRNNSINA;
TSAINASRNNSINAA; SAINASRNNSINAAN; AINASRNNSINAANL;
INASRNNSINAANLS; NASRNNSINAANLSK; ASRNNSINAANLSKT;
SRNNSINAANLSKTQ; RNNSINAANLSKTQE; NNSINAANLSKTQEK;
NSINAANLSKTQEKL; SINAANLSKTQEKLS; INAANLSKTQEKLSS;
NAANLSKTQEKLSSG; AANLSKTQEKLSSGY; ANLSKTQEKLSSGYR;
NLSKTQEKLSSGYRI; LSKTQEKLSSGYRIN; SKTQEKLSSGYRINR;
KTQEKLSSGYRINRA; TQEKLSSGYRINRAS; QEKLSSGYRINRASD;
EKLSSGYRINRASDD; KLSSGYRINRASDDA; LSSGYRINRASDDAA;
SSGYRINRASDDAAG; SGYRINRASDDAAGM; GYRINRASDDAAGMG;
YRINRASDDAAGMGV; RINRASDDAAGMGVS; INRASDDAAGMGVSG;
NRASDDAAGMGVSGK; RASDDAAGMGVSGKI; ASDDAAGMGVSGKIN;
SDDAAGMGVSGKINA; DDAAGMGVSGKINAQ; DAAGMGVSGKINAQI;
AAGMGVSGKINAQIR; AGMGVSGKINAQIRG; GMGVSGKINAQIRGL;
MGVSGKINAQIRGLS; GVSGKINAQIRGLSQ; VSGKINAQIRGLSQA;
SGKINAQIRGLSQAS; GKINAQIRGLSQASR; KINAQIRGLSQASRN;
INAQIRGLSQASRNT; NAQIRGLSQASRNTS; AQIRGLSQASRNTSK;
QIRGLSQASRNTSKA; IRGLSQASRNTSKAI; RGLSQASRNTSKAIN;
GLSQASRNTSKAINF; LSQASRNTSKAINFI; SQASRNTSKAINFIQ;
QASRNTSKAINFIQT; ASRNTSKAINFIQTT; SRNTSKAINFIQTTE;
RNTSKAINFIQTTEG; NTSKAINFIQTTEGN; TSKAINFIQTTEGNL;
SKAINFIQTTEGNLN; KAINFIQTTEGNLNE; AINFIQTTEGNLNEV;
INFIQTTEGNLNEVE; NFIQTTEGNLNEVEK; FIQTTEGNLNEVEKV;
IQTTEGNLNEVEKVL; QTTEGNLNEVEKVLV; TTEGNLNEVEKVLVR;
TEGNLNEVEKVLVRM; EGNLNEVEKVLVRMK; GNLNEVEKVLVRMKE;
NLNEVEKVLVRMKEL; LNEVEKVLVRMKELA; NEVEKVLVRMKELAV;
EVEKVLVRMKELAVQ; VEKVLVRMKELAVQS; EKVLVRMKELAVQSG;
KVLVRMKELAVQSGN; VLVRMKELAVQSGNG; LVRMKELAVQSGNGT;
VRMKELAVQSGNGTY; RMKELAVQSGNGTYS; MKELAVQSGNGTYSD;
KELAVQSGNGTYSDA; ELAVQSGNGTYSDAD; LAVQSGNGTYSDADR;
AVQSGNGTYSDADRG; VQSGNGTYSDADRGS; QSGNGTYSDADRGSI;
SGNGTYSDADRGSIQ; GNGTYSDADRGSIQI; NGTYSDADRGSIQIE;
GTYSDADRGSIQIEI; TYSDADRGSIQIEIE; YSDADRGSIQIEIEQ;
SDADRGSIQIEIEQL; DADRGSIQIEIEQLT; ADRGSIQIEIEQLTD;
DRGSIQIEIEQLTDE; RGSIQIEIEQLTDEI; GSIQIEIEQLTDEIN;
SIQIEIEQLTDEINR; IQIEIEQLTDEINRI; QIEIEQLTDEINRIA;
IEIEQLTDEINRIAD; EIEQLTDEINRIADQ; IEQLTDEINRIADQA;
EQLTDEINRIADQAQ; QLTDEINRIADQAQY; LTDEINRIADQAQYN;
TDEINRIADQAQYNQ; DEINRIADQAQYNQM; EINRIADQAQYNQMH;
INRIADQAQYNQMHM; NRIADQAQYNQMHML; RIADQAQYNQMHMLS;

```
IADQAQYNQMHMLSN;   ADQAQYNQMHMLSNK;   DQAQYNQMHMLSNKS;
QAQYNQMHMLSNKSA;   AQYNQMHMLSNKSAS;   QYNQMHMLSNKSASQ;
YNQMHMLSNKSASQN;   NQMHMLSNKSASQNV;   QMHMLSNKSASQNVR;
MHMLSNKSASQNVRT;   HMLSNKSASQNVRTA;   MLSNKSASQNVRTAE;
LSNKSASQNVRTAEE;   SNKSASQNVRTAEEL;   NKSASQNVRTAEELG;
KSASQNVRTAEELGM;   SASQNVRTAEELGMQ;   ASQNVRTAEELGMQP;
SQNVRTAEELGMQPA;   QNVRTAEELGMQPAK;   NVRTAEELGMQPAKI;
VRTAEELGMQPAKIN;   RTAEELGMQPAKINT;   TAEELGMQPAKINTP;
AEELGMQPAKINTPA;   EELGMQPAKINTPAS;   ELGMQPAKINTPASL;
LGMQPAKINTPASLS;   GMQPAKINTPASLSG;   MQPAKINTPASLSGS;
QPAKINTPASLSGSQ;   PAKINTPASLSGSQA;   AKINTPASLSGSQAS;
KINTPASLSGSQASW;   INTPASLSGSQASWT;   NTPASLSGSQASWTL;
TPASLSGSQASWTLR;   PASLSGSQASWTLRV;   ASLSGSQASWTLRVH;
SLSGSQASWTLRVHV;   LSGSQASWTLRVHVG;   SGSQASWTLRVHVGA;
GSQASWTLRVHVGAN;   SQASWTLRVHVGANQ;   QASWTLRVHVGANQD;
ASWTLRVHVGANQDE;   SWTLRVHVGANQDEA;   WTLRVHVGANQDEAI;
TLRVHVGANQDEAIA;   LRVHVGANQDEAIAV;   RVHVGANQDEAIAVN;
VHVGANQDEAIAVNI;   HVGANQDEAIAVNIY;   VGANQDEAIAVNIYA;
GANQDEAIAVNIYAA;   ANQDEAIAVNIYAAN;   NQDEAIAVNIYAANV;
QDEAIAVNIYAANVA;   DEAIAVNIYAANVAN;   EAIAVNIYAANVANL;
AIAVNIYAANVANLF;   IAVNIYAANVANLFS;   AVNIYAANVANLFSG;
VNIYAANVANLFSGE;   NIYAANVANLFSGEG;   IYAANVANLFSGEGS;
YAANVANLFSGEGSQ;   AANVANLFSGEGSQA;   ANVANLFSGEGSQAA;
NVANLFSGEGSQAAQ;   VANLFSGEGSQAAQT;   ANLFSGEGSQAAQTA;
NLFSGEGSQAAQTAP;   LFSGEGSQAAQTAPV;   FSGEGSQAAQTAPVQ;
SGEGSQAAQTAPVQE;   GEGSQAAQTAPVQEG;   EGSQAAQTAPVQEGA;
GSQAAQTAPVQEGAQ;   SQAAQTAPVQEGAQQ;   QAAQTAPVQEGAQQE;
AAQTAPVQEGAQQEG;   AQTAPVQEGAQQEGA;   QTAPVQEGAQQEGAQ;
TAPVQEGAQQEGAQQ;   APVQEGAQQEGAQQP;   PVQEGAQQEGAQQPA;
VQEGAQQEGAQQPAP;   QEGAQQEGAQQPAPA;   EGAQQEGAQQPAPAT;
GAQQEGAQQPAPATA;   AQQEGAQQPAPATAP;   QQEGAQQPAPATAPS;
QEGAQQPAPATAPSQ;   EGAQQPAPATAPSQG;   GAQQPAPATAPSQGG;
AQQPAPATAPSQGGV;   QQPAPATAPSQGGVN;   QPAPATAPSQGGVNS;
PAPATAPSQGGVNSP;   APATAPSQGGVNSPV;   PATAPSQGGVNSPVN;
ATAPSQGGVNSPVNV;   TAPSQGGVNSPVNVT;   APSQGGVNSPVNVTT;
PSQGGVNSPVNVTTT;   SQGGVNSPVNVTTTV;   QGGVNSPVNVTTTVD;
GGVNSPVNVTTTVDA;   GVNSPVNVTTTVDAN;   VNSPVNVTTTVDANT;
NSPVNVTTTVDANTS;   SPVNVTTTVDANTSL;   PVNVTTTVDANTSLA;
VNVTTTVDANTSLAK;   NVTTTVDANTSLAKI;   VTTTVDANTSLAKIE;
TTTVDANTSLAKIEN;   TTVDANTSLAKIENA;   TVDANTSLAKIENAI;
VDANTSLAKIENAIR;   DANTSLAKIENAIRM;   ANTSLAKIENAIRMI;
NTSLAKIENAIRMIS;   TSLAKIENAIRMISD;   SLAKIENAIRMISDQ;
LAKIENAIRMISDQR;   AKIENAIRMISDQRA;   KIENAIRMISDQRAN;
IENAIRMISDQRANL;   ENAIRMISDQRANLG;   NAIRMISDQRANLGA;
AIRMISDQRANLGAF;   IRMISDQRANLGAFQ;   RMISDQRANLGAFQN;
MISDQRANLGAFQNR;   ISDQRANLGAFQNRL;   SDQRANLGAFQNRLE;
DQRANLGAFQNRLES;   QRANLGAFQNRLESI;   RANLGAFQNRLESIK;
ANLGAFQNRLESIKD;   NLGAFQNRLESIKDS;   LGAFQNRLESIKDST;
GAFQNRLESIKDSTE;   AFQNRLESIKDSTEY;   FQNRLESIKDSTEYA;
QNRLESIKDSTEYAI;   NRLESIKDSTEYAIE;   RLESIKDSTEYAIEN;
LESIKDSTEYAIENL;   ESIKDSTEYAIENLK;   SIKDSTEYAIENLKA;
IKDSTEYAIENLKAS;   KDSTEYAIENLKASY;   DSTEYAIENLKASYA;
STEYAIENLKASYAQ;   TEYAIENLKASYAQI;   EYAIENLKASYAQIK;
YAIENLKASYAQIKD;   AIENLKASYAQIKDA;   IENLKASYAQIKDAT;
ENLKASYAQIKDATM;   NLKASYAQIKDATMT;   LKASYAQIKDATMTD;
KASYAQIKDATMTDE;   ASYAQIKDATMTDEV;   SYAQIKDATMTDEVV;
YAQIKDATMTDEVVA;   AQIKDATMTDEVVAS;   QIKDATMTDEVVAST;
```

```
IKDATMTDEVVASTT;  KDATMTDEVVASTTN;  DATMTDEVVASTTNS;
ATMTDEVVASTTNSI;  TMTDEVVASTTNSIL;  MTDEVVASTTNSILT;
TDEVVASTTNSILTQ;  DEVVASTTNSILTQS;  EVVASTTNSILTQSA;
VVASTTNSILTQSAM;  VASTTNSILTQSAMA;  ASTTNSILTQSAMAM;
STTNSILTQSAMAMI;  TTNSILTQSAMAMIA;  TNSILTQSAMAMIAQ;
NSILTQSAMAMIAQA;  SILTQSAMAMIAQAN;  ILTQSAMAMIAQANQ;
LTQSAMAMIAQANQV;  TQSAMAMIAQANQVP;  QSAMAMIAQANQVPQ;
SAMAMIAQANQVPQY;  AMAMIAQANQVPQYV;  MAMIAQANQVPQYVL;
AMIAQANQVPQYVLS;  MIAQANQVPQYVLSL;  IAQANQVPQYVLSLL;
AQANQVPQYVLSLLR;

16 mers:
MIINHNTSAINASRNN;  IINHNTSAINASRNNS;  INHNTSAINASRNNSI;
NHNTSAINASRNNSIN;  HNTSAINASRNNSINA;  NTSAINASRNNSINAA;
TSAINASRNNSINAAN;  SAINASRNNSINAANL;  AINASRNNSINAANLS;
INASRNNSINAANLSK;  NASRNNSINAANLSKT;  ASRNNSINAANLSKTQ;
SRNNSINAANLSKTQE;  RNNSINAANLSKTQEK;  NNSINAANLSKTQEKL;
NSINAANLSKTQEKLS;  SINAANLSKTQEKLSS;  INAANLSKTQEKLSSG;
NAANLSKTQEKLSSGY;  AANLSKTQEKLSSGYR;  ANLSKTQEKLSSGYRI;
NLSKTQEKLSSGYRIN;  LSKTQEKLSSGYRINR;  SKTQEKLSSGYRINRA;
KTQEKLSSGYRINRAS;  TQEKLSSGYRINRASD;  QEKLSSGYRINRASDD;
EKLSSGYRINRASDDA;  KLSSGYRINRASDDAA;  LSSGYRINRASDDAAG;
SSGYRINRASDDAAGM;  SGYRINRASDDAAGMG;  GYRINRASDDAAGMGV;
YRINRASDDAAGMGVS;  RINRASDDAAGMGVSG;  INRASDDAAGMGVSGK;
NRASDDAAGMGVSGKI;  RASDDAAGMGVSGKIN;  ASDDAAGMGVSGKINA;
SDDAAGMGVSGKINAQ;  DDAAGMGVSGKINAQI;  DAAGMGVSGKINAQIR;
AAGMGVSGKINAQIRG;  AGMGVSGKINAQIRGL;  GMGVSGKINAQIRGLS;
MGVSGKINAQIRGLSQ;  GVSGKINAQIRGLSQA;  VSGKINAQIRGLSQAS;
SGKINAQIRGLSQASR;  GKINAQIRGLSQASRN;  KINAQIRGLSQASRNT;
INAQIRGLSQASRNTS;  NAQIRGLSQASRNTSK;  AQIRGLSQASRNTSKA;
QIRGLSQASRNTSKAI;  IRGLSQASRNTSKAIN;  RGLSQASRNTSKAINF;
GLSQASRNTSKAINFI;  LSQASRNTSKAINFIQ;  SQASRNTSKAINFIQT;
QASRNTSKAINFIQTT;  ASRNTSKAINFIQTTE;  SRNTSKAINFIQTTEG;
RNTSKAINFIQTTEGN;  NTSKAINFIQTTEGNL;  TSKAINFIQTTEGNLN;
SKAINFIQTTEGNLNE;  KAINFIQTTEGNLNEV;  AINFIQTTEGNLNEVE;
INFIQTTEGNLNEVEK;  NFIQTTEGNLNEVEKV;  FIQTTEGNLNEVEKVL;
IQTTEGNLNEVEKVLV;  QTTEGNLNEVEKVLVR;  TTEGNLNEVEKVLVRM;
TEGNLNEVEKVLVRMK;  EGNLNEVEKVLVRMKE;  GNLNEVEKVLVRMKEL;
NLNEVEKVLVRMKELA;  LNEVEKVLVRMKELAV;  NEVEKVLVRMKELAVQ;
EVEKVLVRMKELAVQS;  VEKVLVRMKELAVQSG;  EKVLVRMKELAVQSGN;
KVLVRMKELAVQSGNG;  VLVRMKELAVQSGNGT;  LVRMKELAVQSGNGTY;
VRMKELAVQSGNGTYS;  RMKELAVQSGNGTYSD;  MKELAVQSGNGTYSDA;
KELAVQSGNGTYSDAD;  ELAVQSGNGTYSDADR;  LAVQSGNGTYSDADRG;
AVQSGNGTYSDADRGS;  VQSGNGTYSDADRGSI;  QSGNGTYSDADRGSIQ;
SGNGTYSDADRGSIQI;  GNGTYSDADRGSIQIE;  NGTYSDADRGSIQIEI;
GTYSDADRGSIQIEIE;  TYSDADRGSIQIEIEQ;  YSDADRGSIQIEIEQL;
SDADRGSIQIEIEQLT;  DADRGSIQIEIEQLTD;  ADRGSIQIEIEQLTDE;
DRGSIQIEIEQLTDEI;  RGSIQIEIEQLTDEIN;  GSIQIEIEQLTDEINR;
SIQIEIEQLTDEINRI;  IQIEIEQLTDEINRIA;  QIEIEQLTDEINRIAD;
IEIEQLTDEINRIADQ;  EIEQLTDEINRIADQA;  IEQLTDEINRIADQAQ;
EQLTDEINRIADQAQY;  QLTDEINRIADQAQYN;  LTDEINRIADQAQYNQ;
TDEINRIADQAQYNQM;  DEINRIADQAQYNQMH;  EINRIADQAQYNQMHM;
INRIADQAQYNQMHML;  NRIADQAQYNQMHMLS;  RIADQAQYNQMHMLSN;
IADQAQYNQMHMLSNK;  ADQAQYNQMHMLSNKS;  DQAQYNQMHMLSNKSA;
QAQYNQMHMLSNKSAS;  AQYNQMHMLSNKSASQ;  QYNQMHMLSNKSASQN;
YNQMHMLSNKSASQNV;  NQMHMLSNKSASQNVR;  QMHMLSNKSASQNVRT;
MHMLSNKSASQNVRTA;  HMLSNKSASQNVRTAE;  MLSNKSASQNVRTAEE;
```

| | | |
|---|---|---|
| LSNKSASQNVRTAEEL; | SNKSASQNVRTAEELG; | NKSASQNVRTAEELGM; |
| KSASQNVRTAEELGMQ; | SASQNVRTAEELGMQP; | ASQNVRTAEELGMQPA; |
| SQNVRTAEELGMQPAK; | QNVRTAEELGMQPAKI; | NVRTAEELGMQPAKIN; |
| VRTAEELGMQPAKINT; | RTAEELGMQPAKINTP; | TAEELGMQPAKINTPA; |
| AEELGMQPAKINTPAS; | EELGMQPAKINTPASL; | ELGMQPAKINTPASLS; |
| LGMQPAKINTPASLSG; | GMQPAKINTPASLSGS; | MQPAKINTPASLSGSQ; |
| QPAKINTPASLSGSQA; | PAKINTPASLSGSQAS; | AKINTPASLSGSQASW; |
| KINTPASLSGSQASWT; | INTPASLSGSQASWTL; | NTPASLSGSQASWTLR; |
| TPASLSGSQASWTLRV; | PASLSGSQASWTLRVH; | ASLSGSQASWTLRVHV; |
| SLSGSQASWTLRVHVG; | LSGSQASWTLRVHVGA; | SGSQASWTLRVHVGAN; |
| GSQASWTLRVHVGANQ; | SQASWTLRVHVGANQD; | QASWTLRVHVGANQDE; |
| ASWTLRVHVGANQDEA; | SWTLRVHVGANQDEAI; | WTLRVHVGANQDEAIA; |
| TLRVHVGANQDEAIAV; | LRVHVGANQDEAIAVN; | RVHVGANQDEAIAVNI; |
| VHVGANQDEAIAVNIY; | HVGANQDEAIAVNIYA; | VGANQDEAIAVNIYAA; |
| GANQDEAIAVNIYAAN; | ANQDEAIAVNIYAANV; | NQDEAIAVNIYAANVA; |
| QDEAIAVNIYAANVAN; | DEAIAVNIYAANVANL; | EAIAVNIYAANVANLF; |
| AIAVNIYAANVANLFS; | IAVNIYAANVANLFSG; | AVNIYAANVANLFSGE; |
| VNIYAANVANLFSGEG; | NIYAANVANLFSGEGS; | IYAANVANLFSGEGSQ; |
| YAANVANLFSGEGSQA; | AANVANLFSGEGSQAA; | ANVANLFSGEGSQAAQ; |
| NVANLFSGEGSQAAQT; | VANLFSGEGSQAAQTA; | ANLFSGEGSQAAQTAP; |
| NLFSGEGSQAAQTAPV; | LFSGEGSQAAQTAPVQ; | FSGEGSQAAQTAPVQE; |
| SGEGSQAAQTAPVQEG; | GEGSQAAQTAPVQEGA; | EGSQAAQTAPVQEGAQ; |
| GSQAAQTAPVQEGAQQ; | SQAAQTAPVQEGAQQE; | QAAQTAPVQEGAQQEG; |
| AAQTAPVQEGAQQEGA; | AQTAPVQEGAQQEGAQ; | QTAPVQEGAQQEGAQQ; |
| TAPVQEGAQQEGAQQP; | APVQEGAQQEGAQQPA; | PVQEGAQQEGAQQPAP; |
| VQEGAQQEGAQQPAPA; | QEGAQQEGAQQPAPAT; | EGAQQEGAQQPAPATA; |
| GAQQEGAQQPAPATAP; | AQQEGAQQPAPATAPS; | QQEGAQQPAPATAPSQ; |
| QEGAQQPAPATAPSQG; | EGAQQPAPATAPSQGG; | GAQQPAPATAPSQGGV; |
| AQQPAPATAPSQGGVN; | QQPAPATAPSQGGVNS; | QPAPATAPSQGGVNSP; |
| PAPATAPSQGGVNSPV; | APATAPSQGGVNSPVN; | PATAPSQGGVNSPVNV; |
| ATAPSQGGVNSPVNVT; | TAPSQGGVNSPVNVTT; | APSQGGVNSPVNVTTT; |
| PSQGGVNSPVNVTTTV; | SQGGVNSPVNVTTTVD; | QGGVNSPVNVTTTVDA; |
| GGVNSPVNVTTTVDAN; | GVNSPVNVTTTVDANT; | VNSPVNVTTTVDANTS; |
| NSPVNVTTTVDANTSL; | SPVNVTTTVDANTSLA; | PVNVTTTVDANTSLAK; |
| VNVTTTVDANTSLAKI; | NVTTTVDANTSLAKIE; | VTTTVDANTSLAKIEN; |
| TTTVDANTSLAKIENA; | TTVDANTSLAKIENAI; | TVDANTSLAKIENAIR; |
| VDANTSLAKIENAIRM; | DANTSLAKIENAIRMI; | ANTSLAKIENAIRMIS; |
| NTSLAKIENAIRMISD; | TSLAKIENAIRMISDQ; | SLAKIENAIRMISDQR; |
| LAKIENAIRMISDQRA; | AKIENAIRMISDQRAN; | KIENAIRMISDQRANL; |
| IENAIRMISDQRANLG; | ENAIRMISDQRANLGA; | NAIRMISDQRANLGAF; |
| AIRMISDQRANLGAFQ; | IRMISDQRANLGAFQN; | RMISDQRANLGAFQNR; |
| MISDQRANLGAFQNRL; | ISDQRANLGAFQNRLE; | SDQRANLGAFQNRLES; |
| DQRANLGAFQNRLESI; | QRANLGAFQNRLESIK; | RANLGAFQNRLESIKD; |
| ANLGAFQNRLESIKDS; | NLGAFQNRLESIKDST; | LGAFQNRLESIKDSTE; |
| GAFQNRLESIKDSTEY; | AFQNRLESIKDSTEYA; | FQNRLESIKDSTEYAI; |
| QNRLESIKDSTEYAIE; | NRLESIKDSTEYAIEN; | RLESIKDSTEYAIENL; |
| LESIKDSTEYAIENLK; | ESIKDSTEYAIENLKA; | SIKDSTEYAIENLKAS; |
| IKDSTEYAIENLKASY; | KDSTEYAIENLKASYA; | DSTEYAIENLKASYAQ; |
| STEYAIENLKASYAQI; | TEYAIENLKASYAQIK; | EYAIENLKASYAQIKD; |
| YAIENLKASYAQIKDA; | AIENLKASYAQIKDAT; | IENLKASYAQIKDATM; |
| ENLKASYAQIKDATMT; | NLKASYAQIKDATMTD; | LKASYAQIKDATMTDE; |
| KASYAQIKDATMTDEV; | ASYAQIKDATMTDEVV; | SYAQIKDATMTDEVVA; |
| YAQIKDATMTDEVVAS; | AQIKDATMTDEVVAST; | QIKDATMTDEVVASTT; |
| IKDATMTDEVVASTTN; | KDATMTDEVVASTTNS; | DATMTDEVVASTTNSI; |
| ATMTDEVVASTTNSIL; | TMTDEVVASTTNSILT; | MTDEVVASTTNSILTQ; |
| TDEVVASTTNSILTQS; | DEVVASTTNSILTQSA; | EVVASTTNSILTQSAM; |
| VVASTTNSILTQSAMA; | VASTTNSILTQSAMAM; | ASTTNSILTQSAMAMI; |

| | |
|---|---|
| | STTNSILTQSAMAMIA; TTNSILTQSAMAMIAQ; TNSILTQSAMAMIAQA;<br>NSILTQSAMAMIAQAN; SILTQSAMAMIAQANQ; ILTQSAMAMIAQANQV;<br>LTQSAMAMIAQANQVP; TQSAMAMIAQANQVPQ; QSAMAMIAQANQVPQY;<br>SAMAMIAQANQVPQYV; AMAMIAQANQVPQYVL; MAMIAQANQVPQYVLS;<br>AMIAQANQVPQYVLSL; MIAQANQVPQYVLSLL; IAQANQVPQYVLSLLR; |
| 1L8W\|A Chain A, Crystal Structure Of Lyme Disease Variable Surface Antigen Vlse Of Borrelia Burgdorferi | 13 mers:<br>MRGSHHHHHHGSS; RGSHHHHHHGSSQ; GSHHHHHHGSSQV; SHHHHHHGSSQVA;<br>HHHHHHGSSQVAD; HHHHHGSSQVADK; HHHHGSSQVADKD; HHHGSSQVADKDD;<br>HHGSSQVADKDDP; HGSSQVADKDDPT; GSSQVADKDDPTN; SSQVADKDDPTNK;<br>SQVADKDDPTNKF; QVADKDDPTNKFY; VADKDDPTNKFYQ; ADKDDPTNKFYQS;<br>DKDDPTNKFYQSV; KDDPTNKFYQSVI; DDPTNKFYQSVIQ; DPTNKFYQSVIQL;<br>PTNKFYQSVIQLG; TNKFYQSVIQLGN; NKFYQSVIQLGNG; KFYQSVIQLGNGF;<br>FYQSVIQLGNGFL; YQSVIQLGNGFLD; QSVIQLGNGFLDV; SVIQLGNGFLDVF;<br>VIQLGNGFLDVFT; IQLGNGFLDVFTS; QLGNGFLDVFTSF; LGNGFLDVFTSFG;<br>GNGFLDVFTSFGG; NGFLDVFTSFGGL; GFLDVFTSFGGLV; FLDVFTSFGGLVA;<br>LDVFTSFGGLVAE; DVFTSFGGLVAEA; VFTSFGGLVAEAF; FTSFGGLVAEAFG;<br>TSFGGLVAEAFGF; SFGGLVAEAFGFK; FGGLVAEAFGFKS; GGLVAEAFGFKSD;<br>GLVAEAFGFKSDP; LVAEAFGFKSDPK; VAEAFGFKSDPKK; AEAFGFKSDPKKS;<br>EAFGFKSDPKKSD; AFGFKSDPKKSDV; FGFKSDPKKSDVK; GFKSDPKKSDVKT;<br>FKSDPKKSDVKTY; KSDPKKSDVKTYF; SDPKKSDVKTYFT; DPKKSDVKTYFTT;<br>PKKSDVKTYFTTV; KKSDVKTYFTTVA; KSDVKTYFTTVAA; SDVKTYFTTVAAK;<br>DVKTYFTTVAAKL; VKTYFTTVAAKLE; KTYFTTVAAKLEK; TYFTTVAAKLEKT;<br>YFTTVAAKLEKTK; FTTVAAKLEKTKT; TTVAAKLEKTKTD; TVAAKLEKTKTDL;<br>VAAKLEKTKTDLN; AAKLEKTKTDLNS; AKLEKTKTDLNSL; KLEKTKTDLNSLP;<br>LEKTKTDLNSLPK; EKTKTDLNSLPKE; KTKTDLNSLPKEK; TKTDLNSLPKEKS;<br>KTDLNSLPKEKSD; TDLNSLPKEKSDI; DLNSLPKEKSDIS; LNSLPKEKSDISS;<br>NSLPKEKSDISST; SLPKEKSDISSTT; LPKEKSDISSTTG; PKEKSDISSTTGK;<br>KEKSDISSTTGKP; EKSDISSTTGKPD; KSDISSTTGKPDS; SDISSTTGKPDST;<br>DISSTTGKPDSTG; ISSTTGKPDSTGS; SSTTGKPDSTGSV; STTGKPDSTGSVG;<br>TTGKPDSTGSVGT; TGKPDSTGSVGTA; GKPDSTGSVGTAV; KPDSTGSVGTAVE;<br>PDSTGSVGTAVEG; DSTGSVGTAVEGA; STGSVGTAVEGAI; TGSVGTAVEGAIK;<br>GSVGTAVEGAIKE; SVGTAVEGAIKEV; VGTAVEGAIKEVS; GTAVEGAIKEVSE;<br>TAVEGAIKEVSEL; AVEGAIKEVSELL; VEGAIKEVSELLD; EGAIKEVSELLDK;<br>GAIKEVSELLDKL; AIKEVSELLDKLV; IKEVSELLDKLVK; KEVSELLDKLVKA;<br>EVSELLDKLVKAV; VSELLDKLVKAVK; SELLDKLVKAVKT; ELLDKLVKAVKTA;<br>LLDKLVKAVKTAE; LDKLVKAVKTAEG; DKLVKAVKTAEGA; KLVKAVKTAEGAS;<br>LVKAVKTAEGASS; VKAVKTAEGASSG; KAVKTAEGASSGT; AVKTAEGASSGTA;<br>VKTAEGASSGTAA; KTAEGASSGTAAI; TAEGASSGTAAIG; AEGASSGTAAIGE;<br>EGASSGTAAIGEV; GASSGTAAIGEVV; ASSGTAAIGEVVA; SSGTAAIGEVVAD;<br>SGTAAIGEVVADA; GTAAIGEVVADAD; TAAIGEVVADADA; AAIGEVVADADAA;<br>AIGEVVADADAAK; IGEVVADADAAKV; GEVVADADAAKVA; EVVADADAAKVAD;<br>VVADADAAKVADK; VADADAAKVADKA; ADADAAKVADKAS; DADAAKVADKASV;<br>ADAAKVADKASVK; DAAKVADKASVKG; AAKVADKASVKGI; AKVADKASVKGIA;<br>KVADKASVKGIAK; VADKASVKGIAKG; ADKASVKGIAKGI; DKASVKGIAKGIK;<br>KASVKGIAKGIKE; ASVKGIAKGIKEI; SVKGIAKGIKEIV; VKGIAKGIKEIVE;<br>KGIAKGIKEIVEA; GIAKGIKEIVEAA; IAKGIKEIVEAAG; AKGIKEIVEAAGG;<br>KGIKEIVEAAGGS; GIKEIVEAAGGSE; IKEIVEAAGGSEK; KEIVEAAGGSEKL;<br>EIVEAAGGSEKLK; IVEAAGGSEKLKA; VEAAGGSEKLKAV; EAAGGSEKLKAVA;<br>AAGGSEKLKAVAA; AGGSEKLKAVAAA; GGSEKLKAVAAAK; GSEKLKAVAAAKG;<br>SEKLKAVAAAKGE; EKLKAVAAAKGEN; KLKAVAAAKGENN; LKAVAAAKGENNK;<br>KAVAAAKGENNKG; AVAAAKGENNKGA; VAAAKGENNKGAG; AAAKGENNKGAGK;<br>AAKGENNKGAGKL; AKGENNKGAGKLF; KGENNKGAGKLFG; GENNKGAGKLFGK;<br>ENNKGAGKLFGKA; NNKGAGKLFGKAG; NKGAGKLFGKAGA; KGAGKLFGKAGAA;<br>GAGKLFGKAGAAA; AGKLFGKAGAAAH; GKLFGKAGAAAHG; KLFGKAGAAAHGD;<br>LFGKAGAAAHGDS; FGKAGAAAHGDSE; GKAGAAAHGDSEA; KAGAAAHGDSEAA; |

AGAAAHGDSEAAS; GAAAHGDSEAASK; AAAHGDSEAASKA; AAHGDSEAASKAA;
AHGDSEAASKAAG; HGDSEAASKAAGA; GDSEAASKAAGAV; DSEAASKAAGAVS;
SEAASKAAGAVSA; EAASKAAGAVSAV; AASKAAGAVSAVS; ASKAAGAVSAVSG;
SKAAGAVSAVSGE; KAAGAVSAVSGEQ; AAGAVSAVSGEQI; AGAVSAVSGEQIL;
GAVSAVSGEQILS; AVSAVSGEQILSA; VSAVSGEQILSAI; SAVSGEQILSAIV;
AVSGEQILSAIVT; VSGEQILSAIVTA; SGEQILSAIVTAA; GEQILSAIVTAAD;
EQILSAIVTAADA; QILSAIVTAADAA; ILSAIVTAADAAE; LSAIVTAADAAEQ;
SAIVTAADAAEQD; AIVTAADAAEQDG; IVTAADAAEQDGK; VTAADAAEQDGKK;
TAADAAEQDGKKP; AADAAEQDGKKPE; ADAAEQDGKKPEE; DAAEQDGKKPEEA;
AAEQDGKKPEEAK; AEQDGKKPEEAKN; EQDGKKPEEAKNP; QDGKKPEEAKNPI;
DGKKPEEAKNPIA; GKKPEEAKNPIAA; KKPEEAKNPIAAA; KPEEAKNPIAAAI;
PEEAKNPIAAAIG; EEAKNPIAAAIGD; EAKNPIAAAIGDK; AKNPIAAAIGDKD;
KNPIAAAIGDKDG; NPIAAAIGDKDGG; PIAAAIGDKDGGA; IAAAIGDKDGGAE;
AAAIGDKDGGAEF; AAIGDKDGGAEFG; AIGDKDGGAEFGQ; IGDKDGGAEFGQD;
GDKDGGAEFGQDE; DKDGGAEFGQDEX; KDGGAEFGQDEXK; DGGAEFGQDEXKK;
GGAEFGQDEXKKD; GAEFGQDEXKKDD; AEFGQDEXKKDDQ; EFGQDEXKKDDQI;
FGQDEXKKDDQIA; GQDEXKKDDQIAA; QDEXKKDDQIAAA; DEXKKDDQIAAAI;
EXKKDDQIAAAIA; XKKDDQIAAAIAL; KKDDQIAAAIALR; KDDQIAAAIALRG;
DDQIAAAIALRGX; DQIAAAIALRGXA; QIAAAIALRGXAK; IAAAIALRGXAKD;
AAAIALRGXAKDG; AAIALRGXAKDGK; AIALRGXAKDGKF; IALRGXAKDGKFA;
ALRGXAKDGKFAV; LRGXAKDGKFAVK; RGXAKDGKFAVKD; GXAKDGKFAVKDG;
XAKDGKFAVKDGE; AKDGKFAVKDGEK; KDGKFAVKDGEKE; DGKFAVKDGEKEK;
GKFAVKDGEKEKA; KFAVKDGEKEKAE; FAVKDGEKEKAEG; AVKDGEKEKAEGA;
VKDGEKEKAEGAI; KDGEKEKAEGAIK; DGEKEKAEGAIKG; GEKEKAEGAIKGA;
EKEKAEGAIKGAA; KEKAEGAIKGAAE; EKAEGAIKGAAES; KAEGAIKGAAESA;
AEGAIKGAAESAV; EGAIKGAAESAVR; GAIKGAAESAVRK; AIKGAAESAVRKV;
IKGAAESAVRKVL; KGAAESAVRKVLG; GAAESAVRKVLGA; AAESAVRKVLGAI;
AESAVRKVLGAIT; ESAVRKVLGAITG; SAVRKVLGAITGL; AVRKVLGAITGLI;
VRKVLGAITGLIG; RKVLGAITGLIGD; KVLGAITGLIGDA; VLGAITGLIGDAV;
LGAITGLIGDAVS; GAITGLIGDAVSS; AITGLIGDAVSSG; ITGLIGDAVSSGL;
TGLIGDAVSSGLR; GLIGDAVSSGLRK; LIGDAVSSGLRKV; IGDAVSSGLRKVG;
GDAVSSGLRKVGD; DAVSSGLRKVGDS; AVSSGLRKVGDSV; VSSGLRKVGDSVK;
SSGLRKVGDSVKA; SGLRKVGDSVKAA; GLRKVGDSVKAAS; LRKVGDSVKAASK;
RKVGDSVKAASKE; KVGDSVKAASKET; VGDSVKAASKETP; GDSVKAASKETPP;
DSVKAASKETPPA; SVKAASKETPPAL; VKAASKETPPALN; KAASKETPPALNK;

14 mers:
MRGSHHHHHHGSSQ; RGSHHHHHHGSSQV; GSHHHHHHGSSQVA;
SHHHHHHGSSQVAD; HHHHHHGSSQVADK; HHHHHGSSQVADKD;
HHHHGSSQVADKDD; HHHGSSQVADKDDP; HHGSSQVADKDDPT;
HGSSQVADKDDPTN; GSSQVADKDDPTNK; SSQVADKDDPTNKF;
SQVADKDDPTNKFY; QVADKDDPTNKFYQ; VADKDDPTNKFYQS;
ADKDDPTNKFYQSV; DKDDPTNKFYQSVI; KDDPTNKFYQSVIQ;
DDPTNKFYQSVIQL; DPTNKFYQSVIQLG; PTNKFYQSVIQLGN;
TNKFYQSVIQLGNG; NKFYQSVIQLGNGF; KFYQSVIQLGNGFL;
FYQSVIQLGNGFLD; YQSVIQLGNGFLDV; QSVIQLGNGFLDVF;
SVIQLGNGFLDVFT; VIQLGNGFLDVFTS; IQLGNGFLDVFTSF;
QLGNGFLDVFTSFG; LGNGFLDVFTSFGG; GNGFLDVFTSFGGL;
NGFLDVFTSFGGLV; GFLDVFTSFGGLVA; FLDVFTSFGGLVAE;
LDVFTSFGGLVAEA; DVFTSFGGLVAEAF; VFTSFGGLVAEAFG;
FTSFGGLVAEAFGF; TSFGGLVAEAFGFK; SFGGLVAEAFGFKS;
FGGLVAEAFGFKSD; GGLVAEAFGFKSDP; GLVAEAFGFKSDPK;
LVAEAFGFKSDPKK; VAEAFGFKSDPKKS; AEAFGFKSDPKKSD;
EAFGFKSDPKKSDV; AFGFKSDPKKSDVK; FGFKSDPKKSDVKT;
GFKSDPKKSDVKTY; FKSDPKKSDVKTYF; KSDPKKSDVKTYFT;
SDPKKSDVKTYFTT; DPKKSDVKTYFTTV; PKKSDVKTYFTTVA;
KKSDVKTYFTTVAA; KSDVKTYFTTVAAK; SDVKTYFTTVAAKL;

DVKTYFTTVAAKLE; VKTYFTTVAAKLEK; KTYFTTVAAKLEKT;
TYFTTVAAKLEKTK; YFTTVAAKLEKTKT; FTTVAAKLEKTKTD;
TTVAAKLEKTKTDL; TVAAKLEKTKTDLN; VAAKLEKTKTDLNS;
AAKLEKTKTDLNSL; AKLEKTKTDLNSLP; KLEKTKTDLNSLPK;
LEKTKTDLNSLPKE; EKTKTDLNSLPKEK; KTKTDLNSLPKEKS;
TKTDLNSLPKEKSD; KTDLNSLPKEKSDI; TDLNSLPKEKSDIS;
DLNSLPKEKSDISS; LNSLPKEKSDISST; NSLPKEKSDISSTT;
SLPKEKSDISSTTG; LPKEKSDISSTTGK; PKEKSDISSTTGKP;
KEKSDISSTTGKPD; EKSDISSTTGKPDS; KSDISSTTGKPDST;
SDISSTTGKPDSTG; DISSTTGKPDSTGS; ISSTTGKPDSTGSV;
SSTTGKPDSTGSVG; STTGKPDSTGSVGT; TTGKPDSTGSVGTA;
TGKPDSTGSVGTAV; GKPDSTGSVGTAVE; KPDSTGSVGTAVEG;
PDSTGSVGTAVEGA; DSTGSVGTAVEGAI; STGSVGTAVEGAIK;
TGSVGTAVEGAIKE; GSVGTAVEGAIKEV; SVGTAVEGAIKEVS;
VGTAVEGAIKEVSE; GTAVEGAIKEVSEL; TAVEGAIKEVSELL;
AVEGAIKEVSELLD; VEGAIKEVSELLDK; EGAIKEVSELLDKL;
GAIKEVSELLDKLV; AIKEVSELLDKLVK; IKEVSELLDKLVKA;
KEVSELLDKLVKAV; EVSELLDKLVKAVK; VSELLDKLVKAVKT;
SELLDKLVKAVKTA; ELLDKLVKAVKTAE; LLDKLVKAVKTAEG;
LDKLVKAVKTAEGA; DKLVKAVKTAEGAS; KLVKAVKTAEGASS;
LVKAVKTAEGASSG; VKAVKTAEGASSGT; KAVKTAEGASSGTA;
AVKTAEGASSGTAA; VKTAEGASSGTAAI; KTAEGASSGTAAIG;
TAEGASSGTAAIGE; AEGASSGTAAIGEV; EGASSGTAAIGEVV;
GASSGTAAIGEVVA; ASSGTAAIGEVVAD; SSGTAAIGEVVADA;
SGTAAIGEVVADAD; GTAAIGEVVADADA; TAAIGEVVADADAA;
AAIGEVVADADAAK; AIGEVVADADAAKV; IGEVVADADAAKVA;
GEVVADADAAKVAD; EVVADADAAKVADK; VVADADAAKVADKA;
VADADAAKVADKAS; ADADAAKVADKASV; DADAAKVADKASVK;
ADAAKVADKASVKG; DAAKVADKASVKGI; AAKVADKASVKGIA;
AKVADKASVKGIAK; KVADKASVKGIAKG; VADKASVKGIAKGI;
ADKASVKGIAKGIK; DKASVKGIAKGIKE; KASVKGIAKGIKEI;
ASVKGIAKGIKEIV; SVKGIAKGIKEIVE; VKGIAKGIKEIVEA;
KGIAKGIKEIVEAA; GIAKGIKEIVEAAG; IAKGIKEIVEAAGG;
AKGIKEIVEAAGGS; KGIKEIVEAAGGSE; GIKEIVEAAGGSEK;
IKEIVEAAGGSEKL; KEIVEAAGGSEKLK; EIVEAAGGSEKLKA;
IVEAAGGSEKLKAV; VEAAGGSEKLKAVA; EAAGGSEKLKAVAA;
AAGGSEKLKAVAAA; AGGSEKLKAVAAAK; GGSEKLKAVAAAKG;
GSEKLKAVAAAKGE; SEKLKAVAAAKGEN; EKLKAVAAAKGENN;
KLKAVAAAKGENNK; LKAVAAAKGENNKG; KAVAAAKGENNKGA;
AVAAAKGENNKGAG; VAAAKGENNKGAGK; AAAKGENNKGAGKL;
AAKGENNKGAGKLF; AKGENNKGAGKLFG; KGENNKGAGKLFGK;
GENNKGAGKLFGKA; ENNKGAGKLFGKAG; NNKGAGKLFGKAGA;
NKGAGKLFGKAGAA; KGAGKLFGKAGAAA; GAGKLFGKAGAAAH;
AGKLFGKAGAAAHG; GKLFGKAGAAAHGD; KLFGKAGAAAHGDS;
LFGKAGAAAHGDSE; FGKAGAAAHGDSEA; GKAGAAAHGDSEAA;
KAGAAAHGDSEAAS; AGAAAHGDSEAASK; GAAAHGDSEAASKA;
AAAHGDSEAASKAA; AAHGDSEAASKAAG; AHGDSEAASKAAGA;
HGDSEAASKAAGAV; GDSEAASKAAGAVS; DSEAASKAAGAVSA;
SEAASKAAGAVSAV; EAASKAAGAVSAVS; AASKAAGAVSAVSG;
ASKAAGAVSAVSGE; SKAAGAVSAVSGEQ; KAAGAVSAVSGEQI;
AAGAVSAVSGEQIL; AGAVSAVSGEQILS; GAVSAVSGEQILSA;
AVSAVSGEQILSAI; VSAVSGEQILSAIV; SAVSGEQILSAIVT;
AVSGEQILSAIVTA; VSGEQILSAIVTAA; SGEQILSAIVTAAD;
GEQILSAIVTAADA; EQILSAIVTAADAA; QILSAIVTAADAAE;
ILSAIVTAADAAEQ; LSAIVTAADAAEQD; SAIVTAADAAEQDG;
AIVTAADAAEQDGK; IVTAADAAEQDGKK; VTAADAAEQDGKKP;
TAADAAEQDGKKPE; AADAAEQDGKKPEE; ADAAEQDGKKPEEA;

DAAEQDGKKPEEAK; AAEQDGKKPEEAKN; AEQDGKKPEEAKNP;
EQDGKKPEEAKNPI; QDGKKPEEAKNPIA; DGKKPEEAKNPIAA;
GKKPEEAKNPIAAA; KKPEEAKNPIAAAI; KPEEAKNPIAAAIG;
PEEAKNPIAAAIGD; EEAKNPIAAAIGDK; EAKNPIAAAIGDKD;
AKNPIAAAIGDKDG; KNPIAAAIGDKDGG; NPIAAAIGDKDGGA;
PIAAAIGDKDGGAE; IAAAIGDKDGGAEF; AAAIGDKDGGAEFG;
AAIGDKDGGAEFGQ; AIGDKDGGAEFGQD; IGDKDGGAEFGQDE;
GDKDGGAEFGQDEX; DKDGGAEFGQDEXK; KDGGAEFGQDEXKK;
DGGAEFGQDEXKKD; GGAEFGQDEXKKDD; GAEFGQDEXKKDDQ;
AEFGQDEXKKDDQI; EFGQDEXKKDDQIA; FGQDEXKKDDQIAA;
GQDEXKKDDQIAAA; QDEXKKDDQIAAAI; DEXKKDDQIAAAIA;
EXKKDDQIAAAIAL; XKKDDQIAAAIALR; KKDDQIAAAIALRG;
KDDQIAAAIALRGX; DDQIAAAIALRGXA; DQIAAAIALRGXAK;
QIAAAIALRGXAKD; IAAAIALRGXAKDG; AAAIALRGXAKDGK;
AAIALRGXAKDGKF; AIALRGXAKDGKFA; IALRGXAKDGKFAV;
ALRGXAKDGKFAVK; LRGXAKDGKFAVKD; RGXAKDGKFAVKDG;
GXAKDGKFAVKDGE; XAKDGKFAVKDGEK; AKDGKFAVKDGEKE;
KDGKFAVKDGEKEK; DGKFAVKDGEKEKA; GKFAVKDGEKEKAE;
KFAVKDGEKEKAEG; FAVKDGEKEKAEGA; AVKDGEKEKAEGAI;
VKDGEKEKAEGAIK; KDGEKEKAEGAIKG; DGEKEKAEGAIKGA;
GEKEKAEGAIKGAA; EKEKAEGAIKGAAE; KEKAEGAIKGAAES;
EKAEGAIKGAAESA; KAEGAIKGAAESAV; AEGAIKGAAESAVR;
EGAIKGAAESAVRK; GAIKGAAESAVRKV; AIKGAAESAVRKVL;
IKGAAESAVRKVLG; KGAAESAVRKVLGA; GAAESAVRKVLGAI;
AAESAVRKVLGAIT; AESAVRKVLGAITG; ESAVRKVLGAITGL;
SAVRKVLGAITGLI; AVRKVLGAITGLIG; VRKVLGAITGLIGD;
RKVLGAITGLIGDA; KVLGAITGLIGDAV; VLGAITGLIGDAVS;
LGAITGLIGDAVSS; GAITGLIGDAVSSG; AITGLIGDAVSSGL;
ITGLIGDAVSSGLR; TGLIGDAVSSGLRK; GLIGDAVSSGLRKV;
LIGDAVSSGLRKVG; IGDAVSSGLRKVGD; GDAVSSGLRKVGDS;
DAVSSGLRKVGDSV; AVSSGLRKVGDSVK; VSSGLRKVGDSVKA;
SSGLRKVGDSVKAA; SGLRKVGDSVKAAS; GLRKVGDSVKAASK;
LRKVGDSVKAASKE; RKVGDSVKAASKET; KVGDSVKAASKETP;
VGDSVKAASKETPP; GDSVKAASKETPPA; DSVKAASKETPPAL;
SVKAASKETPPALN; VKAASKETPPALNK;

15 mers:
MRGSHHHHHHGSSQV; RGSHHHHHHGSSQVA; GSHHHHHHGSSQVAD;
SHHHHHHGSSQVADK; HHHHHHGSSQVADKD; HHHHHGSSQVADKDD;
HHHHGSSQVADKDDP; HHHGSSQVADKDDPT; HHGSSQVADKDDPTN;
HGSSQVADKDDPTNK; GSSQVADKDDPTNKF; SSQVADKDDPTNKFY;
SQVADKDDPTNKFYQ; QVADKDDPTNKFYQS; VADKDDPTNKFYQSV;
ADKDDPTNKFYQSVI; DKDDPTNKFYQSVIQ; KDDPTNKFYQSVIQL;
DDPTNKFYQSVIQLG; DPTNKFYQSVIQLGN; PTNKFYQSVIQLGNG;
TNKFYQSVIQLGNGF; NKFYQSVIQLGNGFL; KFYQSVIQLGNGFLD;
FYQSVIQLGNGFLDV; YQSVIQLGNGFLDVF; QSVIQLGNGFLDVFT;
SVIQLGNGFLDVFTS; VIQLGNGFLDVFTSF; IQLGNGFLDVFTSFG;
QLGNGFLDVFTSFGG; LGNGFLDVFTSFGGL; GNGFLDVFTSFGGLV;
NGFLDVFTSFGGLVA; GFLDVFTSFGGLVAE; FLDVFTSFGGLVAEA;
LDVFTSFGGLVAEAF; DVFTSFGGLVAEAFG; VFTSFGGLVAEAFGF;
FTSFGGLVAEAFGFK; TSFGGLVAEAFGFKS; SFGGLVAEAFGFKSD;
FGGLVAEAFGFKSDP; GGLVAEAFGFKSDPK; GLVAEAFGFKSDPKK;
LVAEAFGFKSDPKKS; VAEAFGFKSDPKKSD; AEAFGFKSDPKKSDV;
EAFGFKSDPKKSDVK; AFGFKSDPKKSDVKT; FGFKSDPKKSDVKTY;
GFKSDPKKSDVKTYF; FKSDPKKSDVKTYFT; KSDPKKSDVKTYFTT;
SDPKKSDVKTYFTTV; DPKKSDVKTYFTTVA; PKKSDVKTYFTTVAA;
KKSDVKTYFTTVAAK; KSDVKTYFTTVAAKL; SDVKTYFTTVAAKLE;

| | |
|---|---|
| DVKTYFTTVAAKLEK; VKTYFTTVAAKLEKT; KTYFTTVAAKLEKTK; TYFTTVAAKLEKTKT; YFTTVAAKLEKTKTD; FTTVAAKLEKTKTDL; TTVAAKLEKTKTDLN; TVAAKLEKTKTDLNS; VAAKLEKTKTDLNSL; AAKLEKTKTDLNSLP; AKLEKTKTDLNSLPK; KLEKTKTDLNSLPKE; LEKTKTDLNSLPKEK; EKTKTDLNSLPKEKS; KTKTDLNSLPKEKSD; TKTDLNSLPKEKSDI; KTDLNSLPKEKSDIS; TDLNSLPKEKSDISS; DLNSLPKEKSDISST; LNSLPKEKSDISSTT; NSLPKEKSDISSTTG; SLPKEKSDISSTTGK; LPKEKSDISSTTGKP; PKEKSDISSTTGKPD; KEKSDISSTTGKPDS; EKSDISSTTGKPDST; KSDISSTTGKPDSTG; SDISSTTGKPDSTGS; DISSTTGKPDSTGSV; ISSTTGKPDSTGSVG; SSTTGKPDSTGSVGT; STTGKPDSTGSVGTA; TTGKPDSTGSVGTAV; TGKPDSTGSVGTAVE; GKPDSTGSVGTAVEG; KPDSTGSVGTAVEGA; PDSTGSVGTAVEGAI; DSTGSVGTAVEGAIK; STGSVGTAVEGAIKE; TGSVGTAVEGAIKEV; GSVGTAVEGAIKEVS; SVGTAVEGAIKEVSE; VGTAVEGAIKEVSEL; GTAVEGAIKEVSELL; TAVEGAIKEVSELLD; AVEGAIKEVSELLDK; VEGAIKEVSELLDKL; EGAIKEVSELLDKLV; GAIKEVSELLDKLVK; AIKEVSELLDKLVKA; IKEVSELLDKLVKAV; KEVSELLDKLVKAVK; EVSELLDKLVKAVKT; VSELLDKLVKAVKTA; SELLDKLVKAVKTAE; ELLDKLVKAVKTAEG; LLDKLVKAVKTAEGA; LDKLVKAVKTAEGAS; DKLVKAVKTAEGASS; KLVKAVKTAEGASSG; LVKAVKTAEGASSGT; VKAVKTAEGASSGTA; KAVKTAEGASSGTAA; AVKTAEGASSGTAAI; VKTAEGASSGTAAIG; KTAEGASSGTAAIGE; TAEGASSGTAAIGEV; AEGASSGTAAIGEVV; EGASSGTAAIGEVVA; GASSGTAAIGEVVAD; ASSGTAAIGEVVADA; SSGTAAIGEVVADAD; SGTAAIGEVVADADA; GTAAIGEVVADADAA; TAAIGEVVADADAAK; AAIGEVVADADAAKV; AIGEVVADADAAKVA; IGEVVADADAAKVAD; GEVVADADAAKVADK; EVVADADAAKVADKA; VVADADAAKVADKAS; VADADAAKVADKASV; ADADAAKVADKASVK; DADAAKVADKASVKG; ADAAKVADKASVKGI; DAAKVADKASVKGIA; AAKVADKASVKGIAK; AKVADKASVKGIAKG; KVADKASVKGIAKGI; VADKASVKGIAKGIK; ADKASVKGIAKGIKE; DKASVKGIAKGIKEI; KASVKGIAKGIKEIV; ASVKGIAKGIKEIVE; SVKGIAKGIKEIVEA; VKGIAKGIKEIVEAA; KGIAKGIKEIVEAAG; GIAKGIKEIVEAAGG; IAKGIKEIVEAAGGS; AKGIKEIVEAAGGSE; KGIKEIVEAAGGSEK; GIKEIVEAAGGSEKL; IKEIVEAAGGSEKLK; KEIVEAAGGSEKLKA; EIVEAAGGSEKLKAV; IVEAAGGSEKLKAVA; VEAAGGSEKLKAVAA; EAAGGSEKLKAVAAA; AAGGSEKLKAVAAAK; AGGSEKLKAVAAAKG; GGSEKLKAVAAAKGE; GSEKLKAVAAAKGEN; SEKLKAVAAAKGENN; EKLKAVAAAKGENNK; KLKAVAAAKGENNKG; LKAVAAAKGENNKGA; KAVAAAKGENNKGAG; AVAAAKGENNKGAGK; VAAAKGENNKGAGKL; AAAKGENNKGAGKLF; AAKGENNKGAGKLFG; AKGENNKGAGKLFGK; KGENNKGAGKLFGKA; GENNKGAGKLFGKAG; ENNKGAGKLFGKAGA; NNKGAGKLFGKAGAA; NKGAGKLFGKAGAAA; KGAGKLFGKAGAAAH; GAGKLFGKAGAAAHG; AGKLFGKAGAAAHGD; GKLFGKAGAAAHGDS; KLFGKAGAAAHGDSE; LFGKAGAAAHGDSEA; FGKAGAAAHGDSEAA; GKAGAAAHGDSEAAS; KAGAAAHGDSEAASK; AGAAAHGDSEAASKA; GAAAHGDSEAASKAA; AAAHGDSEAASKAAG; AAHGDSEAASKAAGA; AHGDSEAASKAAGAV; HGDSEAASKAAGAVS; GDSEAASKAAGAVSA; DSEAASKAAGAVSAV; SEAASKAAGAVSAVS; EAASKAAGAVSAVSG; AASKAAGAVSAVSGE; ASKAAGAVSAVSGEQ; SKAAGAVSAVSGEQI; KAAGAVSAVSGEQIL; AAGAVSAVSGEQILS; AGAVSAVSGEQILSA; GAVSAVSGEQILSAI; AVSAVSGEQILSAIV; VSAVSGEQILSAIVT; SAVSGEQILSAIVTA; AVSGEQILSAIVTAA; VSGEQILSAIVTAAD; SGEQILSAIVTAADA; GEQILSAIVTAADAA; EQILSAIVTAADAAE; QILSAIVTAADAAEQ; ILSAIVTAADAAEQD; LSAIVTAADAAEQDG; SAIVTAADAAEQDGK; AIVTAADAAEQDGKK; IVTAADAAEQDGKKP; VTAADAAEQDGKKPE; TAADAAEQDGKKPEE; AADAAEQDGKKPEEA; ADAAEQDGKKPEEAK; | |

DAAEQDGKKPEEAKN; AAEQDGKKPEEAKNP; AEQDGKKPEEAKNPI;
EQDGKKPEEAKNPIA; QDGKKPEEAKNPIAA; DGKKPEEAKNPIAAA;
GKKPEEAKNPIAAAI; KKPEEAKNPIAAAIG; KPEEAKNPIAAAIGD;
PEEAKNPIAAAIGDK; EEAKNPIAAAIGDKD; EAKNPIAAAIGDKDG;
AKNPIAAAIGDKDGG; KNPIAAAIGDKDGGA; NPIAAAIGDKDGGAE;
PIAAAIGDKDGGAEF; IAAAIGDKDGGAEFG; AAAIGDKDGGAEFGQ;
AAIGDKDGGAEFGQD; AIGDKDGGAEFGQDE; IGDKDGGAEFGQDEX;
GDKDGGAEFGQDEXK; DKDGGAEFGQDEXKK; KDGGAEFGQDEXKKD;
DGGAEFGQDEXKKDD; GGAEFGQDEXKKDDQ; GAEFGQDEXKKDDQI;
AEFGQDEXKKDDQIA; EFGQDEXKKDDQIAA; FGQDEXKKDDQIAAA;
GQDEXKKDDQIAAAI; QDEXKKDDQIAAAIA; DEXKKDDQIAAAIAL;
EXKKDDQIAAAIALR; XKKDDQIAAAIALRG; KKDDQIAAAIALRGX;
KDDQIAAAIALRGXA; DDQIAAAIALRGXAK; DQIAAAIALRGXAKD;
QIAAAIALRGXAKDG; IAAAIALRGXAKDGK; AAAIALRGXAKDGKF;
AAIALRGXAKDGKFA; AIALRGXAKDGKFAV; IALRGXAKDGKFAVK;
ALRGXAKDGKFAVKD; LRGXAKDGKFAVKDG; RGXAKDGKFAVKDGE;
GXAKDGKFAVKDGEK; XAKDGKFAVKDGEKE; AKDGKFAVKDGEKEK;
KDGKFAVKDGEKEKA; DGKFAVKDGEKEKAE; GKFAVKDGEKEKAEG;
KFAVKDGEKEKAEGA; FAVKDGEKEKAEGAI; AVKDGEKEKAEGAIK;
VKDGEKEKAEGAIKG; KDGEKEKAEGAIKGA; DGEKEKAEGAIKGAA;
GEKEKAEGAIKGAAE; EKEKAEGAIKGAAES; KEKAEGAIKGAAESA;
EKAEGAIKGAAESAV; KAEGAIKGAAESAVR; AEGAIKGAAESAVRK;
EGAIKGAAESAVRKV; GAIKGAAESAVRKVL; AIKGAAESAVRKVLG;
IKGAAESAVRKVLGA; KGAAESAVRKVLGAI; GAAESAVRKVLGAIT;
AAESAVRKVLGAITG; AESAVRKVLGAITGL; ESAVRKVLGAITGLI;
SAVRKVLGAITGLIG; AVRKVLGAITGLIGD; VRKVLGAITGLIGDA;
RKVLGAITGLIGDAV; KVLGAITGLIGDAVS; VLGAITGLIGDAVSS;
LGAITGLIGDAVSSG; GAITGLIGDAVSSGL; AITGLIGDAVSSGLR;
ITGLIGDAVSSGLRK; TGLIGDAVSSGLRKV; GLIGDAVSSGLRKVG;
LIGDAVSSGLRKVGD; IGDAVSSGLRKVGDS; GDAVSSGLRKVGDSV;
DAVSSGLRKVGDSVK; AVSSGLRKVGDSVKA; VSSGLRKVGDSVKAA;
SSGLRKVGDSVKAAS; SGLRKVGDSVKAASK; GLRKVGDSVKAASKE;
LRKVGDSVKAASKET; RKVGDSVKAASKETP; KVGDSVKAASKETPP;
VGDSVKAASKETPPA; GDSVKAASKETPPAL; DSVKAASKETPPALN;
SVKAASKETPPALNK;

16 mers:
MRGSHHHHHHGSSQVA; RGSHHHHHHGSSQVAD; GSHHHHHHGSSQVADK;
SHHHHHHGSSQVADKD; HHHHHHGSSQVADKDD; HHHHHGSSQVADKDDP;
HHHHGSSQVADKDDPT; HHHGSSQVADKDDPTN; HHGSSQVADKDDPTNK;
HGSSQVADKDDPTNKF; GSSQVADKDDPTNKFY; SSQVADKDDPTNKFYQ;
SQVADKDDPTNKFYQS; QVADKDDPTNKFYQSV; VADKDDPTNKFYQSVI;
ADKDDPTNKFYQSVIQ; DKDDPTNKFYQSVIQL; KDDPTNKFYQSVIQLG;
DDPTNKFYQSVIQLGN; DPTNKFYQSVIQLGNG; PTNKFYQSVIQLGNGF;
TNKFYQSVIQLGNGFL; NKFYQSVIQLGNGFLD; KFYQSVIQLGNGFLDV;
FYQSVIQLGNGFLDVF; YQSVIQLGNGFLDVFT; QSVIQLGNGFLDVFTS;
SVIQLGNGFLDVFTSF; VIQLGNGFLDVFTSFG; IQLGNGFLDVFTSFGG;
QLGNGFLDVFTSFGGL; LGNGFLDVFTSFGGLV; GNGFLDVFTSFGGLVA;
NGFLDVFTSFGGLVAE; GFLDVFTSFGGLVAEA; FLDVFTSFGGLVAEAF;
LDVFTSFGGLVAEAFG; DVFTSFGGLVAEAFGF; VFTSFGGLVAEAFGFK;
FTSFGGLVAEAFGFKS; TSFGGLVAEAFGFKSD; SFGGLVAEAFGFKSDP;
FGGLVAEAFGFKSDPK; GGLVAEAFGFKSDPKK; GLVAEAFGFKSDPKKS;
LVAEAFGFKSDPKKSD; VAEAFGFKSDPKKSDV; AEAFGFKSDPKKSDVK;
EAFGFKSDPKKSDVKT; AFGFKSDPKKSDVKTY; FGFKSDPKKSDVKTYF;
GFKSDPKKSDVKTYFT; FKSDPKKSDVKTYFTT; KSDPKKSDVKTYFTTV;
SDPKKSDVKTYFTTVA; DPKKSDVKTYFTTVAA; PKKSDVKTYFTTVAAK;
KKSDVKTYFTTVAAKL; KSDVKTYFTTVAAKLE; SDVKTYFTTVAAKLEK;

| | |
|---|---|
| DVKTYFTTVAAKLEKT; VKTYFTTVAAKLEKTK; KTYFTTVAAKLEKTKT; TYFTTVAAKLEKTKTD; YFTTVAAKLEKTKTDL; FTTVAAKLEKTKTDLN; TTVAAKLEKTKTDLNS; TVAAKLEKTKTDLNSL; VAAKLEKTKTDLNSLP; AAKLEKTKTDLNSLPK; AKLEKTKTDLNSLPKE; KLEKTKTDLNSLPKEK; LEKTKTDLNSLPKEKS; EKTKTDLNSLPKEKSD; KTKTDLNSLPKEKSDI; TKTDLNSLPKEKSDIS; KTDLNSLPKEKSDISS; TDLNSLPKEKSDISST; DLNSLPKEKSDISSTT; LNSLPKEKSDISSTTG; NSLPKEKSDISSTTGK; SLPKEKSDISSTTGKP; LPKEKSDISSTTGKPD; PKEKSDISSTTGKPDS; KEKSDISSTTGKPDST; EKSDISSTTGKPDSTG; KSDISSTTGKPDSTGS; SDISSTTGKPDSTGSV; DISSTTGKPDSTGSVG; ISSTTGKPDSTGSVGT; SSTTGKPDSTGSVGTA; STTGKPDSTGSVGTAV; TTGKPDSTGSVGTAVE; TGKPDSTGSVGTAVEG; GKPDSTGSVGTAVEGA; KPDSTGSVGTAVEGAI; PDSTGSVGTAVEGAIK; DSTGSVGTAVEGAIKE; STGSVGTAVEGAIKEV; TGSVGTAVEGAIKEVS; GSVGTAVEGAIKEVSE; SVGTAVEGAIKEVSEL; VGTAVEGAIKEVSELL; GTAVEGAIKEVSELLD; TAVEGAIKEVSELLDK; AVEGAIKEVSELLDKL; VEGAIKEVSELLDKLV; EGAIKEVSELLDKLVK; GAIKEVSELLDKLVKA; AIKEVSELLDKLVKAV; IKEVSELLDKLVKAVK; KEVSELLDKLVKAVKT; EVSELLDKLVKAVKTA; VSELLDKLVKAVKTAE; SELLDKLVKAVKTAEG; ELLDKLVKAVKTAEGA; LLDKLVKAVKTAEGAS; LDKLVKAVKTAEGASS; DKLVKAVKTAEGASSG; KLVKAVKTAEGASSGT; LVKAVKTAEGASSGTA; VKAVKTAEGASSGTAA; KAVKTAEGASSGTAAI; AVKTAEGASSGTAAIG; VKTAEGASSGTAAIGE; KTAEGASSGTAAIGEV; TAEGASSGTAAIGEVV; AEGASSGTAAIGEVVA; EGASSGTAAIGEVVAD; GASSGTAAIGEVVADA; ASSGTAAIGEVVADAD; SSGTAAIGEVVADADA; SGTAAIGEVVADADAA; GTAAIGEVVADADAAK; TAAIGEVVADADAAKV; AAIGEVVADADAAKVA; AIGEVVADADAAKVAD; IGEVVADADAAKVADK; GEVVADADAAKVADKA; EVVADADAAKVADKAS; VVADADAAKVADKASV; VADADAAKVADKASVK; ADADAAKVADKASVKG; DADAAKVADKASVKGI; ADAAKVADKASVKGIA; DAAKVADKASVKGIAK; AAKVADKASVKGIAKG; AKVADKASVKGIAKGI; KVADKASVKGIAKGIK; VADKASVKGIAKGIKE; ADKASVKGIAKGIKEI; DKASVKGIAKGIKEIV; KASVKGIAKGIKEIVE; ASVKGIAKGIKEIVEA; SVKGIAKGIKEIVEAA; VKGIAKGIKEIVEAAG; KGIAKGIKEIVEAAGG; GIAKGIKEIVEAAGGS; IAKGIKEIVEAAGGSE; AKGIKEIVEAAGGSEK; KGIKEIVEAAGGSEKL; GIKEIVEAAGGSEKLK; IKEIVEAAGGSEKLKA; KEIVEAAGGSEKLKAV; EIVEAAGGSEKLKAVA; IVEAAGGSEKLKAVAA; VEAAGGSEKLKAVAAA; EAAGGSEKLKAVAAAK; AAGGSEKLKAVAAAKG; AGGSEKLKAVAAAKGE; GGSEKLKAVAAAKGEN; GSEKLKAVAAAKGENN; SEKLKAVAAAKGENNK; EKLKAVAAAKGENNKG; KLKAVAAAKGENNKGA; LKAVAAAKGENNKGAG; KAVAAAKGENNKGAGK; AVAAAKGENNKGAGKL; VAAAKGENNKGAGKLF; AAAKGENNKGAGKLFG; AAKGENNKGAGKLFGK; AKGENNKGAGKLFGKA; KGENNKGAGKLFGKAG; GENNKGAGKLFGKAGA; ENNKGAGKLFGKAGAA; NNKGAGKLFGKAGAAA; NKGAGKLFGKAGAAAH; KGAGKLFGKAGAAAHG; GAGKLFGKAGAAAHGD; AGKLFGKAGAAAHGDS; GKLFGKAGAAAHGDSE; KLFGKAGAAAHGDSEA; LFGKAGAAAHGDSEAA; FGKAGAAAHGDSEAAS; GKAGAAAHGDSEAASK; KAGAAAHGDSEAASKA; AGAAAHGDSEAASKAA; GAAAHGDSEAASKAAG; AAAHGDSEAASKAAGA; AAHGDSEAASKAAGAV; AHGDSEAASKAAGAVS; HGDSEAASKAAGAVSA; GDSEAASKAAGAVSAV; DSEAASKAAGAVSAVS; SEAASKAAGAVSAVSG; EAASKAAGAVSAVSGE; AASKAAGAVSAVSGEQ; ASKAAGAVSAVSGEQI; SKAAGAVSAVSGEQIL; KAAGAVSAVSGEQILS; AAGAVSAVSGEQILSA; AGAVSAVSGEQILSAI; GAVSAVSGEQILSAIV; AVSAVSGEQILSAIVT; VSAVSGEQILSAIVTA; SAVSGEQILSAIVTAA; AVSGEQILSAIVTAAD; VSGEQILSAIVTAADA; SGEQILSAIVTAADAA; GEQILSAIVTAADAAE; EQILSAIVTAADAAEQ; QILSAIVTAADAAEQD; ILSAIVTAADAAEQDG; LSAIVTAADAAEQDGK; SAIVTAADAAEQDGKK; AIVTAADAAEQDGKKP; IVTAADAAEQDGKKPE; VTAADAAEQDGKKPEE; TAADAAEQDGKKPEEA; AADAAEQDGKKPEEAK; ADAAEQDGKKPEEAKN; |

| | |
|---|---|
| | DAAEQDGKKPEEAKNP; AAEQDGKKPEEAKNPI; AEQDGKKPEEAKNPIA;<br>EQDGKKPEEAKNPIAA; QDGKKPEEAKNPIAAA; DGKKPEEAKNPIAAAI;<br>GKKPEEAKNPIAAAIG; KKPEEAKNPIAAAIGD; KPEEAKNPIAAAIGDK;<br>PEEAKNPIAAAIGDKD; EEAKNPIAAAIGDKDG; EAKNPIAAAIGDKDGG;<br>AKNPIAAAIGDKDGGA; KNPIAAAIGDKDGGAE; NPIAAAIGDKDGGAEF;<br>PIAAAIGDKDGGAEFG; IAAAIGDKDGGAEFGQ; AAAIGDKDGGAEFGQD;<br>AAIGDKDGGAEFGQDE; AIGDKDGGAEFGQDEX; IGDKDGGAEFGQDEXK;<br>GDKDGGAEFGQDEXKK; DKDGGAEFGQDEXKKD; KDGGAEFGQDEXKKDD;<br>DGGAEFGQDEXKKDDQ; GGAEFGQDEXKKDDQI; GAEFGQDEXKKDDQIA;<br>AEFGQDEXKKDDQIAA; EFGQDEXKKDDQIAAA; FGQDEXKKDDQIAAAI;<br>GQDEXKKDDQIAAAIA; QDEXKKDDQIAAAIAL; DEXKKDDQIAAAIALR;<br>EXKKDDQIAAAIALRG; XKKDDQIAAAIALRGX; KKDDQIAAAIALRGXA;<br>KDDQIAAAIALRGXAK; DDQIAAAIALRGXAKD; DQIAAAIALRGXAKDG;<br>QIAAAIALRGXAKDGK; IAAAIALRGXAKDGKF; AAAIALRGXAKDGKFA;<br>AAIALRGXAKDGKFAV; AIALRGXAKDGKFAVK; IALRGXAKDGKFAVKD;<br>ALRGXAKDGKFAVKDG; LRGXAKDGKFAVKDGE; RGXAKDGKFAVKDGEK;<br>GXAKDGKFAVKDGEKE; XAKDGKFAVKDGEKEK; AKDGKFAVKDGEKEKA;<br>KDGKFAVKDGEKEKAE; DGKFAVKDGEKEKAEG; GKFAVKDGEKEKAEGA;<br>KFAVKDGEKEKAEGAI; FAVKDGEKEKAEGAIK; AVKDGEKEKAEGAIKG;<br>VKDGEKEKAEGAIKGA; KDGEKEKAEGAIKGAA; DGEKEKAEGAIKGAAE;<br>GEKEKAEGAIKGAAES; EKEKAEGAIKGAAESA; KEKAEGAIKGAAESAV;<br>EKAEGAIKGAAESAVR; KAEGAIKGAAESAVRK; AEGAIKGAAESAVRKV;<br>EGAIKGAAESAVRKVL; GAIKGAAESAVRKVLG; AIKGAAESAVRKVLGA;<br>IKGAAESAVRKVLGAI; KGAAESAVRKVLGAIT; GAAESAVRKVLGAITG;<br>AAESAVRKVLGAITGL; AESAVRKVLGAITGLI; ESAVRKVLGAITGLIG;<br>SAVRKVLGAITGLIGD; AVRKVLGAITGLIGDA; VRKVLGAITGLIGDAV;<br>RKVLGAITGLIGDAVS; KVLGAITGLIGDAVSS; VLGAITGLIGDAVSSG;<br>LGAITGLIGDAVSSGL; GAITGLIGDAVSSGLR; AITGLIGDAVSSGLRK;<br>ITGLIGDAVSSGLRKV; TGLIGDAVSSGLRKVG; GLIGDAVSSGLRKVGD;<br>LIGDAVSSGLRKVGDS; IGDAVSSGLRKVGDSV; GDAVSSGLRKVGDSVK;<br>DAVSSGLRKVGDSVKA; AVSSGLRKVGDSVKAA; VSSGLRKVGDSVKAAS;<br>SSGLRKVGDSVKAASK; SGLRKVGDSVKAASKE; GLRKVGDSVKAASKET;<br>LRKVGDSVKAASKETP; RKVGDSVKAASKETPP; KVGDSVKAASKETPPA;<br>VGDSVKAASKETPPAL; GDSVKAASKETPPALN; DSVKAASKETPPALNK; |
| CAA57807.1<br>Associated protein<br>A (BapA)[Borrelia<br>burgdorferi] | 13 mers:<br>MKKISLLIFLFLF; KKISLLIFLFLFV; KISLLIFLFLFVV; ISLLIFLFLFVVS;<br>SLLIFLFLFVVSL; LLIFLFLFVVSLS; LIFLFLFVVSLSA; IFLFLFVVSLSAN;<br>FLFLFVVSLSANI; LFLFVVSLSANIE; FLFVVSLSANIEE; LFVVSLSANIEEN;<br>FVVSLSANIEENY; VVSLSANIEENYT; VSLSANIEENYTE; SLSANIEENYTET;<br>LSANIEENYTETK; SANIEENYTETKR; ANIEENYTETKRA; NIEENYTETKRAF;<br>IEENYTETKRAFS; EENYTETKRAFSK; ENYTETKRAFSKE; NYTETKRAFSKED;<br>YTETKRAFSKEDF; TETKRAFSKEDFN; ETKRAFSKEDFNL; TKRAFSKEDFNLI;<br>KRAFSKEDFNLIN; RAFSKEDFNLINK; AFSKEDFNLINKR; FSKEDFNLINKRL;<br>SKEDFNLINKRLD; KEDFNLINKRLDN; EDFNLINKRLDNY; DFNLINKRLDNYD;<br>FNLINKRLDNYDF; NLINKRLDNYDFK; LINKRLDNYDFKN; INKRLDNYDFKNE;<br>NKRLDNYDFKNEY; KRLDNYDFKNEYE; RLDNYDFKNEYEK; LDNYDFKNEYEKS;<br>DNYDFKNEYEKSH; NYDFKNEYEKSHV; YDFKNEYEKSHVF; DFKNEYEKSHVFS;<br>FKNEYEKSHVFSD; KNEYEKSHVFSDA; NEYEKSHVFSDAP; EYEKSHVFSDAPR;<br>YEKSHVFSDAPRI; EKSHVFSDAPRIR; KSHVFSDAPRIRG; SHVFSDAPRIRGD;<br>HVFSDAPRIRGDL; VFSDAPRIRGDLR; FSDAPRIRGDLRK; SDAPRIRGDLRKI;<br>DAPRIRGDLRKIG; APRIRGDLRKIGI; PRIRGDLRKIGIK; RIRGDLRKIGIKE;<br>IRGDLRKIGIKEK; RGDLRKIGIKEKS; GDLRKIGIKEKSV; DLRKIGIKEKSVF;<br>LRKIGIKEKSVFL; RKIGIKEKSVFLD; KIGIKEKSVFLDA; IGIKEKSVFLDAL;<br>GIKEKSVFLDALE; IKEKSVFLDALEA; KEKSVFLDALEAI; EKSVFLDALEAIE;<br>KSVFLDALEAIEY; SVFLDALEAIEYL; VFLDALEAIEYLI; FLDALEAIEYLIK; |

LDALEAIEYLIKI; DALEAIEYLIKIK; ALEAIEYLIKIKI; LEAIEYLIKIKIS;
EAIEYLIKIKIST; AIEYLIKIKISTD; IEYLIKIKISTDS; EYLIKIKISTDSI;
YLIKIKISTDSIF; LIKIKISTDSIFL; IKIKISTDSIFLS; KIKISTDSIFLSE;
IKISTDSIFLSED; KISTDSIFLSEDM; ISTDSIFLSEDMI; STDSIFLSEDMIR;
TDSIFLSEDMIRL; DSIFLSEDMIRLI; SIFLSEDMIRLIG; IFLSEDMIRLIGS;
FLSEDMIRLIGSY; LSEDMIRLIGSYP; SEDMIRLIGSYPD; EDMIRLIGSYPDS;
DMIRLIGSYPDSI; MIRLIGSYPDSIF; IRLIGSYPDSIFN; RLIGSYPDSIFNY;
LIGSYPDSIFNYL; IGSYPDSIFNYLI; GSYPDSIFNYLIQ; SYPDSIFNYLIQL;
YPDSIFNYLIQLN; PDSIFNYLIQLNS; DSIFNYLIQLNSD; SIFNYLIQLNSDK;
IFNYLIQLNSDKI; FNYLIQLNSDKID; NYLIQLNSDKIDY; YLIQLNSDKIDYA;
LIQLNSDKIDYAE; IQLNSDKIDYAEK; QLNSDKIDYAEKY; LNSDKIDYAEKYG;
NSDKIDYAEKYGD; SDKIDYAEKYGDN; DKIDYAEKYGDNA; KIDYAEKYGDNAR;
IDYAEKYGDNARN; DYAEKYGDNARNN; YAEKYGDNARNNF; AEKYGDNARNNFK;
EKYGDNARNNFKK; KYGDNARNNFKKD; YGDNARNNFKKDY; GDNARNNFKKDYS;
DNARNNFKKDYSE; NARNNFKKDYSED; ARNNFKKDYSEDK; RNNFKKDYSEDKA;
NNFKKDYSEDKAN; NFKKDYSEDKANT; FKKDYSEDKANTV; KKDYSEDKANTVK;
KDYSEDKANTVKQ; DYSEDKANTVKQI; YSEDKANTVKQIL; SEDKANTVKQILK;
EDKANTVKQILKQ; DKANTVKQILKQI; KANTVKQILKQIL; ANTVKQILKQILA;
NTVKQILKQILAD; TVKQILKQILADL; VKQILKQILADLP; KQILKQILADLPK;
QILKQILADLPKD;

14 mers:
MKKISLLIFLFLFV; KKISLLIFLFLFVV; KISLLIFLFLFVVS;
ISLLIFLFLFVVSL; SLLIFLFLFVVSLS; LLIFLFLFVVSLSA;
LIFLFLFVVSLSAN; IFLFLFVVSLSANI; FLFLFVVSLSANIE;
LFLFVVSLSANIEE; FLFVVSLSANIEEN; LFVVSLSANIEENY;
FVVSLSANIEENYT; VVSLSANIEENYTE; VSLSANIEENYTET;
SLSANIEENYTETK; LSANIEENYTETKR; SANIEENYTETKRA;
ANIEENYTETKRAF; NIEENYTETKRAFS; IEENYTETKRAFSK;
EENYTETKRAFSKE; ENYTETKRAFSKED; NYTETKRAFSKEDF;
YTETKRAFSKEDFN; TETKRAFSKEDFNL; ETKRAFSKEDFNLI;
TKRAFSKEDFNLIN; KRAFSKEDFNLINK; RAFSKEDFNLINKR;
AFSKEDFNLINKRL; FSKEDFNLINKRLD; SKEDFNLINKRLDN;
KEDFNLINKRLDNY; EDFNLINKRLDNYD; DFNLINKRLDNYDF;
FNLINKRLDNYDFK; NLINKRLDNYDFKN; LINKRLDNYDFKNE;
INKRLDNYDFKNEY; NKRLDNYDFKNEYE; KRLDNYDFKNEYEK;
RLDNYDFKNEYEKS; LDNYDFKNEYEKSH; DNYDFKNEYEKSHV;
NYDFKNEYEKSHVF; YDFKNEYEKSHVFS; DFKNEYEKSHVFSD;
FKNEYEKSHVFSDA; KNEYEKSHVFSDAP; NEYEKSHVFSDAPR;
EYEKSHVFSDAPRI; YEKSHVFSDAPRIR; EKSHVFSDAPRIRG;
KSHVFSDAPRIRGD; SHVFSDAPRIRGDL; HVFSDAPRIRGDLR;
VFSDAPRIRGDLRK; FSDAPRIRGDLRKI; SDAPRIRGDLRKIG;
DAPRIRGDLRKIGI; APRIRGDLRKIGIK; PRIRGDLRKIGIKE;
RIRGDLRKIGIKEK; IRGDLRKIGIKEKS; RGDLRKIGIKEKSV;
GDLRKIGIKEKSVF; DLRKIGIKEKSVFL; LRKIGIKEKSVFLD;
RKIGIKEKSVFLDA; KIGIKEKSVFLDAL; IGIKEKSVFLDALE;
GIKEKSVFLDALEA; IKEKSVFLDALEAI; KEKSVFLDALEAIE;
EKSVFLDALEAIEY; KSVFLDALEAIEYL; SVFLDALEAIEYLI;
VFLDALEAIEYLIK; FLDALEAIEYLIKI; LDALEAIEYLIKIK;
DALEAIEYLIKIKI; ALEAIEYLIKIKIS; LEAIEYLIKIKIST;
EAIEYLIKIKISTD; AIEYLIKIKISTDS; IEYLIKIKISTDSI;
EYLIKIKISTDSIF; YLIKIKISTDSIFL; LIKIKISTDSIFLS;
IKIKISTDSIFLSE; KIKISTDSIFLSED; IKISTDSIFLSEDM;
KISTDSIFLSEDMI; ISTDSIFLSEDMIR; STDSIFLSEDMIRL;
TDSIFLSEDMIRLI; DSIFLSEDMIRLIG; SIFLSEDMIRLIGS;
IFLSEDMIRLIGSY; FLSEDMIRLIGSYP; LSEDMIRLIGSYPD;
SEDMIRLIGSYPDS; EDMIRLIGSYPDSI; DMIRLIGSYPDSIF;

MIRLIGSYPDSIFN; IRLIGSYPDSIFNY; RLIGSYPDSIFNYL;
LIGSYPDSIFNYLI; IGSYPDSIFNYLIQ; GSYPDSIFNYLIQL;
SYPDSIFNYLIQLN; YPDSIFNYLIQLNS; PDSIFNYLIQLNSD;
DSIFNYLIQLNSDK; SIFNYLIQLNSDKI; IFNYLIQLNSDKID;
FNYLIQLNSDKIDY; NYLIQLNSDKIDYA; YLIQLNSDKIDYAE;
LIQLNSDKIDYAEK; IQLNSDKIDYAEKY; QLNSDKIDYAEKYG;
LNSDKIDYAEKYGD; NSDKIDYAEKYGDN; SDKIDYAEKYGDNA;
DKIDYAEKYGDNAR; KIDYAEKYGDNARN; IDYAEKYGDNARNN;
DYAEKYGDNARNNF; YAEKYGDNARNNFK; AEKYGDNARNNFKK;
EKYGDNARNNFKKD; KYGDNARNNFKKDY; YGDNARNNFKKDYS;
GDNARNNFKKDYSE; DNARNNFKKDYSED; NARNNFKKDYSEDK;
ARNNFKKDYSEDKA; RNNFKKDYSEDKAN; NNFKKDYSEDKANT;
NFKKDYSEDKANTV; FKKDYSEDKANTVK; KKDYSEDKANTVKQ;
KDYSEDKANTVKQI; DYSEDKANTVKQIL; YSEDKANTVKQILK;
SEDKANTVKQILKQ; EDKANTVKQILKQI; DKANTVKQILKQIL;
KANTVKQILKQILA; ANTVKQILKQILAD; NTVKQILKQILADL;
TVKQILKQILADLP; VKQILKQILADLPK; KQILKQILADLPKD;

15 mers:
MKKISLLIFLFLFVV; KKISLLIFLFLFVVS; KISLLIFLFLFVVSL;
ISLLIFLFLFVVSLS; SLLIFLFLFVVSLSA; LLIFLFLFVVSLSAN;
LIFLFLFVVSLSANI; IFLFLFVVSLSANIE; FLFLFVVSLSANIEE;
LFLFVVSLSANIEEN; FLFVVSLSANIEENY; LFVVSLSANIEENYT;
FVVSLSANIEENYTE; VVSLSANIEENYTET; VSLSANIEENYTETK;
SLSANIEENYTETKR; LSANIEENYTETKRA; SANIEENYTETKRAF;
ANIEENYTETKRAFS; NIEENYTETKRAFSK; IEENYTETKRAFSKE;
EENYTETKRAFSKED; ENYTETKRAFSKEDF; NYTETKRAFSKEDFN;
YTETKRAFSKEDFNL; TETKRAFSKEDFNLI; ETKRAFSKEDFNLIN;
TKRAFSKEDFNLINK; KRAFSKEDFNLINKR; RAFSKEDFNLINKRL;
AFSKEDFNLINKRLD; FSKEDFNLINKRLDN; SKEDFNLINKRLDNY;
KEDFNLINKRLDNYD; EDFNLINKRLDNYDF; DFNLINKRLDNYDFK;
FNLINKRLDNYDFKN; NLINKRLDNYDFKNE; LINKRLDNYDFKNEY;
INKRLDNYDFKNEYE; NKRLDNYDFKNEYEK; KRLDNYDFKNEYEKS;
RLDNYDFKNEYEKSH; LDNYDFKNEYEKSHV; DNYDFKNEYEKSHVF;
NYDFKNEYEKSHVFS; YDFKNEYEKSHVFSD; DFKNEYEKSHVFSDA;
FKNEYEKSHVFSDAP; KNEYEKSHVFSDAPR; NEYEKSHVFSDAPRI;
EYEKSHVFSDAPRIR; YEKSHVFSDAPRIRG; EKSHVFSDAPRIRGD;
KSHVFSDAPRIRGDL; SHVFSDAPRIRGDLR; HVFSDAPRIRGDLRK;
VFSDAPRIRGDLRKI; FSDAPRIRGDLRKIG; SDAPRIRGDLRKIGI;
DAPRIRGDLRKIGIK; APRIRGDLRKIGIKE; PRIRGDLRKIGIKEK;
RIRGDLRKIGIKEKS; IRGDLRKIGIKEKSV; RGDLRKIGIKEKSVF;
GDLRKIGIKEKSVFL; DLRKIGIKEKSVFLD; LRKIGIKEKSVFLDA;
RKIGIKEKSVFLDAL; KIGIKEKSVFLDALE; IGIKEKSVFLDALEA;
GIKEKSVFLDALEAI; IKEKSVFLDALEAIE; KEKSVFLDALEAIEY;
EKSVFLDALEAIEYL; KSVFLDALEAIEYLI; SVFLDALEAIEYLIK;
VFLDALEAIEYLIKI; FLDALEAIEYLIKIK; LDALEAIEYLIKIKI;
DALEAIEYLIKIKIS; ALEAIEYLIKIKIST; LEAIEYLIKIKISTD;
EAIEYLIKIKISTDS; AIEYLIKIKISTDSI; IEYLIKIKISTDSIF;
EYLIKIKISTDSIFL; YLIKIKISTDSIFLS; LIKIKISTDSIFLSE;
IKIKISTDSIFLSED; KIKISTDSIFLSEDM; IKISTDSIFLSEDMI;
KISTDSIFLSEDMIR; ISTDSIFLSEDMIRL; STDSIFLSEDMIRLI;
TDSIFLSEDMIRLIG; DSIFLSEDMIRLIGS; SIFLSEDMIRLIGSY;
IFLSEDMIRLIGSYP; FLSEDMIRLIGSYPD; LSEDMIRLIGSYPDS;
SEDMIRLIGSYPDSI; EDMIRLIGSYPDSIF; DMIRLIGSYPDSIFN;
MIRLIGSYPDSIFNY; IRLIGSYPDSIFNYL; RLIGSYPDSIFNYLI;
LIGSYPDSIFNYLIQ; IGSYPDSIFNYLIQL; GSYPDSIFNYLIQLN;
SYPDSIFNYLIQLNS; YPDSIFNYLIQLNSD; PDSIFNYLIQLNSDK;

DSIFNYLIQLNSDKI; SIFNYLIQLNSDKID; IFNYLIQLNSDKIDY;
FNYLIQLNSDKIDYA; NYLIQLNSDKIDYAE; YLIQLNSDKIDYAEK;
LIQLNSDKIDYAEKY; IQLNSDKIDYAEKYG; QLNSDKIDYAEKYGD;
LNSDKIDYAEKYGDN; NSDKIDYAEKYGDNA; SDKIDYAEKYGDNAR;
DKIDYAEKYGDNARN; KIDYAEKYGDNARNN; IDYAEKYGDNARNNF;
DYAEKYGDNARNNFK; YAEKYGDNARNNFKK; AEKYGDNARNNFKKD;
EKYGDNARNNFKKDY; KYGDNARNNFKKDYS; YGDNARNNFKKDYSE;
GDNARNNFKKDYSED; DNARNNFKKDYSEDK; NARNNFKKDYSEDKA;
ARNNFKKDYSEDKAN; RNNFKKDYSEDKANT; NNFKKDYSEDKANTV;
NFKKDYSEDKANTVK; FKKDYSEDKANTVKQ; KKDYSEDKANTVKQI;
KDYSEDKANTVKQIL; DYSEDKANTVKQILK; YSEDKANTVKQILKQ;
SEDKANTVKQILKQI; EDKANTVKQILKQIL; DKANTVKQILKQILA;
KANTVKQILKQILAD; ANTVKQILKQILADL; NTVKQILKQILADLP;
TVKQILKQILADLPK; VKQILKQILADLPKD;

16 mers:
MKKISLLIFLFLFVVS; KKISLLIFLFLFVVSL; KISLLIFLFLFVVSLS;
ISLLIFLFLFVVSLSA; SLLIFLFLFVVSLSAN; LLIFLFLFVVSLSANI;
LIFLFLFVVSLSANIE; IFLFLFVVSLSANIEE; FLFLFVVSLSANIEEN;
LFLFVVSLSANIEENY; FLFVVSLSANIEENYT; LFVVSLSANIEENYTE;
FVVSLSANIEENYTET; VVSLSANIEENYTETK; VSLSANIEENYTETKR;
SLSANIEENYTETKRA; LSANIEENYTETKRAF; SANIEENYTETKRAFS;
ANIEENYTETKRAFSK; NIEENYTETKRAFSKE; IEENYTETKRAFSKED;
EENYTETKRAFSKEDF; ENYTETKRAFSKEDFN; NYTETKRAFSKEDFNL;
YTETKRAFSKEDFNLI; TETKRAFSKEDFNLIN; ETKRAFSKEDFNLINK;
TKRAFSKEDFNLINKR; KRAFSKEDFNLINKRL; RAFSKEDFNLINKRLD;
AFSKEDFNLINKRLDN; FSKEDFNLINKRLDNY; SKEDFNLINKRLDNYD;
KEDFNLINKRLDNYDF; EDFNLINKRLDNYDFK; DFNLINKRLDNYDFKN;
FNLINKRLDNYDFKNE; NLINKRLDNYDFKNEY; LINKRLDNYDFKNEYE;
INKRLDNYDFKNEYEK; NKRLDNYDFKNEYEKS; KRLDNYDFKNEYEKSH;
RLDNYDFKNEYEKSHV; LDNYDFKNEYEKSHVF; DNYDFKNEYEKSHVFS;
NYDFKNEYEKSHVFSD; YDFKNEYEKSHVFSDA; DFKNEYEKSHVFSDAP;
FKNEYEKSHVFSDAPR; KNEYEKSHVFSDAPRI; NEYEKSHVFSDAPRIR;
EYEKSHVFSDAPRIRG; YEKSHVFSDAPRIRGD; EKSHVFSDAPRIRGDL;
KSHVFSDAPRIRGDLR; SHVFSDAPRIRGDLRK; HVFSDAPRIRGDLRKI;
VFSDAPRIRGDLRKIG; FSDAPRIRGDLRKIGI; SDAPRIRGDLRKIGIK;
DAPRIRGDLRKIGIKE; APRIRGDLRKIGIKEK; PRIRGDLRKIGIKEKS;
RIRGDLRKIGIKEKSV; IRGDLRKIGIKEKSVF; RGDLRKIGIKEKSVFL;
GDLRKIGIKEKSVFLD; DLRKIGIKEKSVFLDA; LRKIGIKEKSVFLDAL;
RKIGIKEKSVFLDALE; KIGIKEKSVFLDALEA; IGIKEKSVFLDALEAI;
GIKEKSVFLDALEAIE; IKEKSVFLDALEAIEY; KEKSVFLDALEAIEYL;
EKSVFLDALEAIEYLI; KSVFLDALEAIEYLIK; SVFLDALEAIEYLIKI;
VFLDALEAIEYLIKIK; FLDALEAIEYLIKIKI; LDALEAIEYLIKIKIS;
DALEAIEYLIKIKIST; ALEAIEYLIKIKISTD; LEAIEYLIKIKISTDS;
EAIEYLIKIKISTDSI; AIEYLIKIKISTDSIF; IEYLIKIKISTDSIFL;
EYLIKIKISTDSIFLS; YLIKIKISTDSIFLSE; LIKIKISTDSIFLSED;
IKIKISTDSIFLSEDM; KIKISTDSIFLSEDMI; IKISTDSIFLSEDMIR;
KISTDSIFLSEDMIRL; ISTDSIFLSEDMIRLI; STDSIFLSEDMIRLIG;
TDSIFLSEDMIRLIGS; DSIFLSEDMIRLIGSY; SIFLSEDMIRLIGSYP;
IFLSEDMIRLIGSYPD; FLSEDMIRLIGSYPDS; LSEDMIRLIGSYPDSI;
SEDMIRLIGSYPDSIF; EDMIRLIGSYPDSIFN; DMIRLIGSYPDSIFNY;
MIRLIGSYPDSIFNYL; IRLIGSYPDSIFNYLI; RLIGSYPDSIFNYLIQ;
LIGSYPDSIFNYLIQL; IGSYPDSIFNYLIQLN; GSYPDSIFNYLIQLNS;
SYPDSIFNYLIQLNSD; YPDSIFNYLIQLNSDK; PDSIFNYLIQLNSDKI;
DSIFNYLIQLNSDKID; SIFNYLIQLNSDKIDY; IFNYLIQLNSDKIDYA;
FNYLIQLNSDKIDYAE; NYLIQLNSDKIDYAEK; YLIQLNSDKIDYAEKY;
LIQLNSDKIDYAEKYG; IQLNSDKIDYAEKYGD; QLNSDKIDYAEKYGDN;

| | |
|---|---|
| | LNSDKIDYAEKYGDNA; NSDKIDYAEKYGDNAR; SDKIDYAEKYGDNARN; DKIDYAEKYGDNARNN; KIDYAEKYGDNARNNF; IDYAEKYGDNARNNFK; DYAEKYGDNARNNFKK; YAEKYGDNARNNFKKD; AEKYGDNARNNFKKDY; EKYGDNARNNFKKDYS; KYGDNARNNFKKDYSE; YGDNARNNFKKDYSED; GDNARNNFKKDYSEDK; DNARNNFKKDYSEDKA; NARNNFKKDYSEDKAN; ARNNFKKDYSEDKANT; RNNFKKDYSEDKANTV; NNFKKDYSEDKANTVK; NFKKDYSEDKANTVKQ; FKKDYSEDKANTVKQI; KKDYSEDKANTVKQIL; KDYSEDKANTVKQILK; DYSEDKANTVKQILKQ; YSEDKANTVKQILKQI; SEDKANTVKQILKQIL; EDKANTVKQILKQILA; DKANTVKQILKQILAD; KANTVKQILKQILADL; ANTVKQILKQILADLP; NTVKQILKQILADLPK; TVKQILKQILADLPKD; |
| Seq19 | 13 mers:<br>MCAFLLLNLVNCK; CAFLLLNLVNCKF; AFLLLNLVNCKFD; FLLLNLVNCKFDS; LLLNLVNCKFDSL; LLNLVNCKFDSLN; LNLVNCKFDSLNL; NLVNCKFDSLNLS; LVNCKFDSLNLST; VNCKFDSLNLSTK; NCKFDSLNLSTKS; CKFDSLNLSTKSV; KFDSLNLSTKSVD; FDSLNLSTKSVDD; DSLNLSTKSVDDK; SLNLSTKSVDDKN; LNLSTKSVDDKNN; NLSTKSVDDKNNS; LSTKSVDDKNNSI; STKSVDDKNNSIA; TKSVDDKNNSIAK; KSVDDKNNSIAKL; SVDDKNNSIAKLL; VDDKNNSIAKLLQ; DDKNNSIAKLLQH; DKNNSIAKLLQHL; KNNSIAKLLQHLS; NNSIAKLLQHLSK; NSIAKLLQHLSKS; SIAKLLQHLSKSE; IAKLLQHLSKSED; AKLLQHLSKSEDQ; KLLQHLSKSEDQA; LLQHLSKSEDQAN; LQHLSKSEDQANK; QHLSKSEDQANKT; HLSKSEDQANKTS; LSKSEDQANKTST; SKSEDQANKTSTS; KSEDQANKTSTSE; SEDQANKTSTSED; EDQANKTSTSEDQ; DQANKTSTSEDQK; QANKTSTSEDQKE; ANKTSTSEDQKEL; NKTSTSEDQKELE; KTSTSEDQKELEI; TSTSEDQKELEIT; STSEDQKELEITE; TSEDQKELEITEN; SEDQKELEITENK; EDQKELEITENKE; DQKELEITENKEQ; QKELEITENKEQE; KELEITENKEQEH; ELEITENKEQEHE; LEITENKEQEHEK; EITENKEQEHEKL; ITENKEQEHEKLS; TENKEQEHEKLSQ; ENKEQEHEKLSQV; NKEQEHEKLSQVA; KEQEHEKLSQVAQ; EQEHEKLSQVAQH; QEHEKLSQVAQHA; EHEKLSQVAQHAP; HEKLSQVAQHAPN; EKLSQVAQHAPNS; KLSQVAQHAPNSK; LSQVAQHAPNSKI; SQVAQHAPNSKIE; QVAQHAPNSKIEK; VAQHAPNSKIEKV; AQHAPNSKIEKVK; QHAPNSKIEKVKS; HAPNSKIEKVKSD; APNSKIEKVKSDG; PNSKIEKVKSDGK; NSKIEKVKSDGKP; SKIEKVKSDGKPV; KIEKVKSDGKPVP; IEKVKSDGKPVPG; EKVKSDGKPVPGD; KVKSDGKPVPGDK; VKSDGKPVPGDKI; KSDGKPVPGDKIL; SDGKPVPGDKILS; DGKPVPGDKILSS; GKPVPGDKILSSN; KPVPGDKILSSNK; PVPGDKILSSNKD; VPGDKILSSNKDI; PGDKILSSNKDIY; GDKILSSNKDIYN; DKILSSNKDIYNS; KILSSNKDIYNSY; ILSSNKDIYNSYI; LSSNKDIYNSYIP; SSNKDIYNSYIPE; SNKDIYNSYIPEV; NKDIYNSYIPEVK; KDIYNSYIPEVKE; DIYNSYIPEVKEE; IYNSYIPEVKEEI; YNSYIPEVKEEIV; NSYIPEVKEEIVY; SYIPEVKEEIVYE; YIPEVKEEIVYEI; IPEVKEEIVYEIL; PEVKEEIVYEILE; EVKEEIVYEILEE; VKEEIVYEILEEV; KEEIVYEILEEVI; EEIVYEILEEVII; |

EIVYEILEEVIIP; IVYEILEEVIIPE; VYEILEEVIIPET;
YEILEEVIIPETK; EILEEVIIPETKI; ILEEVIIPETKIP;
LEEVIIPETKIPE; EEVIIPETKIPEI; EVIIPETKIPEIT;
VIIPETKIPEITE; IIPETKIPEITEE; IPETKIPEITEEV;
PETKIPEITEEVI; ETKIPEITEEVIM; TKIPEITEEVIMP;
KIPEITEEVIMPI; IPEITEEVIMPIP; PEITEEVIMPIPQ;
EITEEVIMPIPQT; ITEEVIMPIPQTI; TEEVIMPIPQTID;
EEVIMPIPQTIDF; EVIMPIPQTIDFY; VIMPIPQTIDFYI;
IMPIPQTIDFYIE; MPIPQTIDFYIEP; PIPQTIDFYIEPR;
IPQTIDFYIEPRP; PQTIDFYIEPRPI; QTIDFYIEPRPIS;
TIDFYIEPRPISS; IDFYIEPRPISSF; DFYIEPRPISSFL;
FYIEPRPISSFLT; YIEPRPISSFLTQ; IEPRPISSFLTQG;
EPRPISSFLTQGT; PRPISSFLTQGTS; RPISSFLTQGTSP;
PISSFLTQGTSPS; ISSFLTQGTSPSI; SSFLTQGTSPSIT;
SFLTQGTSPSITS; FLTQGTSPSITST; LTQGTSPSITSTI;
TQGTSPSITSTIK; QGTSPSITSTIKS; GTSPSITSTIKSY;
TSPSITSTIKSYK; SPSITSTIKSYKE; PSITSTIKSYKEL;
SITSTIKSYKELA; ITSTIKSYKELAK; TSTIKSYKELAKE;
STIKSYKELAKEK; TIKSYKELAKEKI; IKSYKELAKEKIN;
KSYKELAKEKINN; SYKELAKEKINNG; YKELAKEKINNGL;
KELAKEKINNGLN; ELAKEKINNGLNI; LAKEKINNGLNIV;
AKEKINNGLNIVQ; KEKINNGLNIVQK; EKINNGLNIVQKI;
KINNGLNIVQKIT; INNGLNIVQKITQ; NNGLNIVQKITQN;
NGLNIVQKITQNI; GLNIVQKITQNID; LNIVQKITQNIDN;
NIVQKITQNIDNI; IVQKITQNIDNIT; VQKITQNIDNITE;
QKITQNIDNITEN; KITQNIDNITENL; ITQNIDNITENLN;
TQNIDNITENLNS; QNIDNITENLNSK; NIDNITENLNSKE;
IDNITENLNSKET; DNITENLNSKETP; NITENLNSKETPK;
ITENLNSKETPKE; TENLNSKETPKEI; ENLNSKETPKEIS;
NLNSKETPKEISG; LNSKETPKEISGK; NSKETPKEISGKE;
SKETPKEISGKEV; KETPKEISGKEVE; ETPKEISGKEVEE;
TPKEISGKEVEEK; PKEISGKEVEEKI; KEISGKEVEEKIT;
EISGKEVEEKITH; ISGKEVEEKITHP; SGKEVEEKITHPI;
GKEVEEKITHPIF; KEVEEKITHPIFD; EVEEKITHPIFDH;
VEEKITHPIFDHI; EEKITHPIFDHIT; EKITHPIFDHITG;
KITHPIFDHITGS; ITHPIFDHITGSG; THPIFDHITGSGN;
HPIFDHITGSGNN; PIFDHITGSGNNP; IFDHITGSGNNPG;
FDHITGSGNNPGQ; DHITGSGNNPGQD; HITGSGNNPGQDS;
ITGSGNNPGQDSI; TGSGNNPGQDSIS; GSGNNPGQDSISN;
SGNNPGQDSISNT; GNNPGQDSISNTW; NNPGQDSISNTWG;
NPGQDSISNTWGE; PGQDSISNTWGEG; GQDSISNTWGEGL;
QDSISNTWGEGLE; DSISNTWGEGLEI; SISNTWGEGLEIG;
ISNTWGEGLEIGG; SNTWGEGLEIGGD; NTWGEGLEIGGDS;
TWGEGLEIGGDSN; WGEGLEIGGDSNF; GEGLEIGGDSNFF;
EGLEIGGDSNFFT; GLEIGGDSNFFTN; LEIGGDSNFFTNL;
EIGGDSNFFTNLE; IGGDSNFFTNLEE; GGDSNFFTNLEEV;
GDSNFFTNLEEVR; DSNFFTNLEEVRS; SNFFTNLEEVRSS;
NFFTNLEEVRSSI; FFTNLEEVRSSIR; FTNLEEVRSSIRT;
TNLEEVRSSIRTK; NLEEVRSSIRTKI; LEEVRSSIRTKIK;
EEVRSSIRTKIKV; EVRSSIRTKIKVS; VRSSIRTKIKVSD;
RSSIRTKIKVSDG; SSIRTKIKVSDGT; SIRTKIKVSDGTE;

IRTKIKVSDGTEQ; RTKIKVSDGTEQT; TKIKVSDGTEQTK;
KIKVSDGTEQTKD; IKVSDGTEQTKDK; KVSDGTEQTKDKV;
VSDGTEQTKDKVE; SDGTEQTKDKVEI; DGTEQTKDKVEID;
GTEQTKDKVEIDE; TEQTKDKVEIDEI; EQTKDKVEIDEII;
QTKDKVEIDEIIE; TKDKVEIDEIIED; KDKVEIDEIIEDL;
DKVEIDEIIEDLQ; KVEIDEIIEDLQK; VEIDEIIEDLQKL;
EIDEIIEDLQKLK; IDEIIEDLQKLKE; DEIIEDLQKLKEF;
EIIEDLQKLKEFL; IIEDLQKLKEFLE; IEDLQKLKEFLEK;
EDLQKLKEFLEKL; DLQKLKEFLEKLK; LQKLKEFLEKLKK;
QKLKEFLEKLKKY; KLKEFLEKLKKYL; LKEFLEKLKKYLK;
KEFLEKLKKYLKD; EFLEKLKKYLKDT; FLEKLKKYLKDTN;
LEKLKKYLKDTNN; EKLKKYLKDTNNL; KLKKYLKDTNNLS;
LKKYLKDTNNLSA; KKYLKDTNNLSAI; KYLKDTNNLSAIE;
YLKDTNNLSAIEE; LKDTNNLSAIEES; KDTNNLSAIEESV;
DTNNLSAIEESVK; TNNLSAIEESVKG; NNLSAIEESVKGL;
NLSAIEESVKGLS;

14 mers:
MCAFLLLNLVNCKF; CAFLLLNLVNCKFD; AFLLLNLVNCKFDS;
FLLLNLVNCKFDSL; LLLNLVNCKFDSLN; LLNLVNCKFDSLNL;
LNLVNCKFDSLNLS; NLVNCKFDSLNLST; LVNCKFDSLNLSTK;
VNCKFDSLNLSTKS; NCKFDSLNLSTKSV; CKFDSLNLSTKSVD;
KFDSLNLSTKSVDD; FDSLNLSTKSVDDK; DSLNLSTKSVDDKN;
SLNLSTKSVDDKNN; LNLSTKSVDDKNNS; NLSTKSVDDKNNSI;
LSTKSVDDKNNSIA; STKSVDDKNNSIAK; TKSVDDKNNSIAKL;
KSVDDKNNSIAKLL; SVDDKNNSIAKLLQ; VDDKNNSIAKLLQH;
DDKNNSIAKLLQHL; DKNNSIAKLLQHLS; KNNSIAKLLQHLSK;
NNSIAKLLQHLSKS; NSIAKLLQHLSKSE; SIAKLLQHLSKSED;
IAKLLQHLSKSEDQ; AKLLQHLSKSEDQA; KLLQHLSKSEDQAN;
LLQHLSKSEDQANK; LQHLSKSEDQANKT; QHLSKSEDQANKTS;
HLSKSEDQANKTST; LSKSEDQANKTSTS; SKSEDQANKTSTSE;
KSEDQANKTSTSED; SEDQANKTSTSEDQ; EDQANKTSTSEDQK;
DQANKTSTSEDQKE; QANKTSTSEDQKEL; ANKTSTSEDQKELE;
NKTSTSEDQKELEI; KTSTSEDQKELEIT; TSTSEDQKELEITE;
STSEDQKELEITEN; TSEDQKELEITENK; SEDQKELEITENKE;
EDQKELEITENKEQ; DQKELEITENKEQE; QKELEITENKEQEH;
KELEITENKEQEHE; ELEITENKEQEHEK; LEITENKEQEHEKL;
EITENKEQEHEKLS; ITENKEQEHEKLSQ; TENKEQEHEKLSQV;
ENKEQEHEKLSQVA; NKEQEHEKLSQVAQ; KEQEHEKLSQVAQH;
EQEHEKLSQVAQHA; QEHEKLSQVAQHAP; EHEKLSQVAQHAPN;
HEKLSQVAQHAPNS; EKLSQVAQHAPNSK; KLSQVAQHAPNSKI;
LSQVAQHAPNSKIE; SQVAQHAPNSKIEK; QVAQHAPNSKIEKV;
VAQHAPNSKIEKVK; AQHAPNSKIEKVKS; QHAPNSKIEKVKSD;
HAPNSKIEKVKSDG; APNSKIEKVKSDGK; PNSKIEKVKSDGKP;
NSKIEKVKSDGKPV; SKIEKVKSDGKPVP; KIEKVKSDGKPVPG;
IEKVKSDGKPVPGD; EKVKSDGKPVPGDK; KVKSDGKPVPGDKI;
VKSDGKPVPGDKIL; KSDGKPVPGDKILS; SDGKPVPGDKILSS;
DGKPVPGDKILSSN; GKPVPGDKILSSNK; KPVPGDKILSSNKD;
PVPGDKILSSNKDI; VPGDKILSSNKDIY; PGDKILSSNKDIYN;
GDKILSSNKDIYNS; DKILSSNKDIYNSY; KILSSNKDIYNSYI;
ILSSNKDIYNSYIP; LSSNKDIYNSYIPE; SSNKDIYNSYIPEV;

| | |
|---|---|
| SNKDIYNSYIPEVK; NKDIYNSYIPEVKE; KDIYNSYIPEVKEE; | |
| DIYNSYIPEVKEEI; IYNSYIPEVKEEIV; YNSYIPEVKEEIVY; | |
| NSYIPEVKEEIVYE; SYIPEVKEEIVYEI; YIPEVKEEIVYEIL; | |
| IPEVKEEIVYEILE; PEVKEEIVYEILEE; EVKEEIVYEILEEV; | |
| VKEEIVYEILEEVI; KEEIVYEILEEVII; EEIVYEILEEVIIP; | |
| EIVYEILEEVIIPE; IVYEILEEVIIPET; VYEILEEVIIPETK; | |
| YEILEEVIIPETKI; EILEEVIIPETKIP; ILEEVIIPETKIPE; | |
| LEEVIIPETKIPEI; EEVIIPETKIPEIT; EVIIPETKIPEITE; | |
| VIIPETKIPEITEE; IIPETKIPEITEEV; IPETKIPEITEEVI; | |
| PETKIPEITEEVIM; ETKIPEITEEVIMP; TKIPEITEEVIMPI; | |
| KIPEITEEVIMPIP; IPEITEEVIMPIPQ; PEITEEVIMPIPQT; | |
| EITEEVIMPIPQTI; ITEEVIMPIPQTID; TEEVIMPIPQTIDF; | |
| EEVIMPIPQTIDFY; EVIMPIPQTIDFYI; VIMPIPQTIDFYIE; | |
| IMPIPQTIDFYIEP; MPIPQTIDFYIEPR; PIPQTIDFYIEPRP; | |
| IPQTIDFYIEPRPI; PQTIDFYIEPRPIS; QTIDFYIEPRPISS; | |
| TIDFYIEPRPISSF; IDFYIEPRPISSFL; DFYIEPRPISSFLT; | |
| FYIEPRPISSFLTQ; YIEPRPISSFLTQG; IEPRPISSFLTQGT; | |
| EPRPISSFLTQGTS; PRPISSFLTQGTSP; RPISSFLTQGTSPS; | |
| PISSFLTQGTSPSI; ISSFLTQGTSPSIT; SSFLTQGTSPSITS; | |
| SFLTQGTSPSITST; FLTQGTSPSITSTI; LTQGTSPSITSTIK; | |
| TQGTSPSITSTIKS; QGTSPSITSTIKSY; GTSPSITSTIKSYK; | |
| TSPSITSTIKSYKE; SPSITSTIKSYKEL; PSITSTIKSYKELA; | |
| SITSTIKSYKELAK; ITSTIKSYKELAKE; TSTIKSYKELAKEK; | |
| STIKSYKELAKEKI; TIKSYKELAKEKIN; IKSYKELAKEKINN; | |
| KSYKELAKEKINNG; SYKELAKEKINNGL; YKELAKEKINNGLN; | |
| KELAKEKINNGLNI; ELAKEKINNGLNIV; LAKEKINNGLNIVQ; | |
| AKEKINNGLNIVQK; KEKINNGLNIVQKI; EKINNGLNIVQKIT; | |
| KINNGLNIVQKITQ; INNGLNIVQKITQN; NNGLNIVQKITQNI; | |
| NGLNIVQKITQNID; GLNIVQKITQNIDN; LNIVQKITQNIDNI; | |
| NIVQKITQNIDNIT; IVQKITQNIDNITE; VQKITQNIDNITEN; | |
| QKITQNIDNITENL; KITQNIDNITENLN; ITQNIDNITENLNS; | |
| TQNIDNITENLNSK; QNIDNITENLNSKE; NIDNITENLNSKET; | |
| IDNITENLNSKETP; DNITENLNSKETPK; NITENLNSKETPKE; | |
| ITENLNSKETPKEI; TENLNSKETPKEIS; ENLNSKETPKEISG; | |
| NLNSKETPKEISGK; LNSKETPKEISGKE; NSKETPKEISGKEV; | |
| SKETPKEISGKEVE; KETPKEISGKEVEE; ETPKEISGKEVEEK; | |
| TPKEISGKEVEEKI; PKEISGKEVEEKIT; KEISGKEVEEKITH; | |
| EISGKEVEEKITHP; ISGKEVEEKITHPI; SGKEVEEKITHPIF; | |
| GKEVEEKITHPIFD; KEVEEKITHPIFDH; EVEEKITHPIFDHI; | |
| VEEKITHPIFDHIT; EEKITHPIFDHITG; EKITHPIFDHITGS; | |
| KITHPIFDHITGSG; ITHPIFDHITGSGN; THPIFDHITGSGNN; | |
| HPIFDHITGSGNNP; PIFDHITGSGNNPG; IFDHITGSGNNPGQ; | |
| FDHITGSGNNPGQD; DHITGSGNNPGQDS; HITGSGNNPGQDSI; | |
| ITGSGNNPGQDSIS; TGSGNNPGQDSISN; GSGNNPGQDSISNT; | |
| SGNNPGQDSISNTW; GNNPGQDSISNTWG; NNPGQDSISNTWGE; | |
| NPGQDSISNTWGEG; PGQDSISNTWGEGL; GQDSISNTWGEGLE; | |
| QDSISNTWGEGLEI; DSISNTWGEGLEIG; SISNTWGEGLEIGG; | |
| ISNTWGEGLEIGGD; SNTWGEGLEIGGDS; NTWGEGLEIGGDSN; | |
| TWGEGLEIGGDSNF; WGEGLEIGGDSNFF; GEGLEIGGDSNFFT; | |
| EGLEIGGDSNFFTN; GLEIGGDSNFFTNL; LEIGGDSNFFTNLE; | |
| EIGGDSNFFTNLEE; IGGDSNFFTNLEEV; GGDSNFFTNLEEVR; | |

GDSNFFTNLEEVRS;  DSNFFTNLEEVRSS;  SNFFTNLEEVRSSI;
NFFTNLEEVRSSIR;  FFTNLEEVRSSIRT;  FTNLEEVRSSIRTK;
TNLEEVRSSIRTKI;  NLEEVRSSIRTKIK;  LEEVRSSIRTKIKV;
EEVRSSIRTKIKVS;  EVRSSIRTKIKVSD;  VRSSIRTKIKVSDG;
RSSIRTKIKVSDGT;  SSIRTKIKVSDGTE;  SIRTKIKVSDGTEQ;
IRTKIKVSDGTEQT;  RTKIKVSDGTEQTK;  TKIKVSDGTEQTKD;
KIKVSDGTEQTKDK;  IKVSDGTEQTKDKV;  KVSDGTEQTKDKVE;
VSDGTEQTKDKVEI;  SDGTEQTKDKVEID;  DGTEQTKDKVEIDE;
GTEQTKDKVEIDEI;  TEQTKDKVEIDEII;  EQTKDKVEIDEIIE;
QTKDKVEIDEIIED;  TKDKVEIDEIIEDL;  KDKVEIDEIIEDLQ;
DKVEIDEIIEDLQK;  KVEIDEIIEDLQKL;  VEIDEIIEDLQKLK;
EIDEIIEDLQKLKE;  IDEIIEDLQKLKEF;  DEIIEDLQKLKEFL;
EIIEDLQKLKEFLE;  IIEDLQKLKEFLEK;  IEDLQKLKEFLEKL;
EDLQKLKEFLEKLK;  DLQKLKEFLEKLKK;  LQKLKEFLEKLKKY;
QKLKEFLEKLKKYL;  KLKEFLEKLKKYLK;  LKEFLEKLKKYLKD;
KEFLEKLKKYLKDT;  EFLEKLKKYLKDTN;  FLEKLKKYLKDTNN;
LEKLKKYLKDTNNL;  EKLKKYLKDTNNLS;  KLKKYLKDTNNLSA;
LKKYLKDTNNLSAI;  KKYLKDTNNLSAIE;  KYLKDTNNLSAIEE;
YLKDTNNLSAIEES;  LKDTNNLSAIEESV;  KDTNNLSAIEESVK;
DTNNLSAIEESVKG;  TNNLSAIEESVKGL;  NNLSAIEESVKGLS;

15 mers:
MCAFLLLNLVNCKFD;  CAFLLLNLVNCKFDS;  AFLLLNLVNCKFDSL;
FLLLNLVNCKFDSLN;  LLLNLVNCKFDSLNL;  LLNLVNCKFDSLNLS;
LNLVNCKFDSLNLST;  NLVNCKFDSLNLSTK;  LVNCKFDSLNLSTKS;
VNCKFDSLNLSTKSV;  NCKFDSLNLSTKSVD;  CKFDSLNLSTKSVDD;
KFDSLNLSTKSVDDK;  FDSLNLSTKSVDDKN;  DSLNLSTKSVDDKNN;
SLNLSTKSVDDKNNS;  LNLSTKSVDDKNNSI;  NLSTKSVDDKNNSIA;
LSTKSVDDKNNSIAK;  STKSVDDKNNSIAKL;  TKSVDDKNNSIAKLL;
KSVDDKNNSIAKLLQ;  SVDDKNNSIAKLLQH;  VDDKNNSIAKLLQHL;
DDKNNSIAKLLQHLS;  DKNNSIAKLLQHLSK;  KNNSIAKLLQHLSKS;
NNSIAKLLQHLSKSE;  NSIAKLLQHLSKSED;  SIAKLLQHLSKSEDQ;
IAKLLQHLSKSEDQA;  AKLLQHLSKSEDQAN;  KLLQHLSKSEDQANK;
LLQHLSKSEDQANKT;  LQHLSKSEDQANKTS;  QHLSKSEDQANKTST;
HLSKSEDQANKTSTS;  LSKSEDQANKTSTSE;  SKSEDQANKTSTSED;
KSEDQANKTSTSEDQ;  SEDQANKTSTSEDQK;  EDQANKTSTSEDQKE;
DQANKTSTSEDQKEL;  QANKTSTSEDQKELE;  ANKTSTSEDQKELEI;
NKTSTSEDQKELEIT;  KTSTSEDQKELEITE;  TSTSEDQKELEITEN;
STSEDQKELEITENK;  TSEDQKELEITENKE;  SEDQKELEITENKEQ;
EDQKELEITENKEQE;  DQKELEITENKEQEH;  QKELEITENKEQEHE;
KELEITENKEQEHEK;  ELEITENKEQEHEKL;  LEITENKEQEHEKLS;
EITENKEQEHEKLSQ;  ITENKEQEHEKLSQV;  TENKEQEHEKLSQVA;
ENKEQEHEKLSQVAQ;  NKEQEHEKLSQVAQH;  KEQEHEKLSQVAQHA;
EQEHEKLSQVAQHAP;  QEHEKLSQVAQHAPN;  EHEKLSQVAQHAPNS;
HEKLSQVAQHAPNSK;  EKLSQVAQHAPNSKI;  KLSQVAQHAPNSKIE;
LSQVAQHAPNSKIEK;  SQVAQHAPNSKIEKV;  QVAQHAPNSKIEKVK;
VAQHAPNSKIEKVKS;  AQHAPNSKIEKVKSD;  QHAPNSKIEKVKSDG;
HAPNSKIEKVKSDGK;  APNSKIEKVKSDGKP;  PNSKIEKVKSDGKPV;
NSKIEKVKSDGKPVP;  SKIEKVKSDGKPVPG;  KIEKVKSDGKPVPGD;
IEKVKSDGKPVPGDK;  EKVKSDGKPVPGDKI;  KVKSDGKPVPGDKIL;
VKSDGKPVPGDKILS;  KSDGKPVPGDKILSS;  SDGKPVPGDKILSSN;

DGKPVPGDKILSSNK; GKPVPGDKILSSNKD; KPVPGDKILSSNKDI;
PVPGDKILSSNKDIY; VPGDKILSSNKDIYN; PGDKILSSNKDIYNS;
GDKILSSNKDIYNSY; DKILSSNKDIYNSYI; KILSSNKDIYNSYIP;
ILSSNKDIYNSYIPE; LSSNKDIYNSYIPEV; SSNKDIYNSYIPEVK;
SNKDIYNSYIPEVKE; NKDIYNSYIPEVKEE; KDIYNSYIPEVKEEI;
DIYNSYIPEVKEEIV; IYNSYIPEVKEEIVY; YNSYIPEVKEEIVYE;
NSYIPEVKEEIVYEI; SYIPEVKEEIVYEIL; YIPEVKEEIVYEILE;
IPEVKEEIVYEILEE; PEVKEEIVYEILEEV; EVKEEIVYEILEEVI;
VKEEIVYEILEEVII; KEEIVYEILEEVIIP; EEIVYEILEEVIIPE;
EIVYEILEEVIIPET; IVYEILEEVIIPETK; VYEILEEVIIPETKI;
YEILEEVIIPETKIP; EILEEVIIPETKIPE; ILEEVIIPETKIPEI;
LEEVIIPETKIPEIT; EEVIIPETKIPEITE; EVIIPETKIPEITEE;
VIIPETKIPEITEEV; IIPETKIPEITEEVI; IPETKIPEITEEVIM;
PETKIPEITEEVIMP; ETKIPEITEEVIMPI; TKIPEITEEVIMPIP;
KIPEITEEVIMPIPQ; IPEITEEVIMPIPQT; PEITEEVIMPIPQTI;
EITEEVIMPIPQTID; ITEEVIMPIPQTIDF; TEEVIMPIPQTIDFY;
EEVIMPIPQTIDFYI; EVIMPIPQTIDFYIE; VIMPIPQTIDFYIEP;
IMPIPQTIDFYIEPR; MPIPQTIDFYIEPRP; PIPQTIDFYIEPRPI;
IPQTIDFYIEPRPIS; PQTIDFYIEPRPISS; QTIDFYIEPRPISSF;
TIDFYIEPRPISSFL; IDFYIEPRPISSFLT; DFYIEPRPISSFLTQ;
FYIEPRPISSFLTQG; YIEPRPISSFLTQGT; IEPRPISSFLTQGTS;
EPRPISSFLTQGTSP; PRPISSFLTQGTSPS; RPISSFLTQGTSPSI;
PISSFLTQGTSPSIT; ISSFLTQGTSPSITS; SSFLTQGTSPSITST;
SFLTQGTSPSITSTI; FLTQGTSPSITSTIK; LTQGTSPSITSTIKS;
TQGTSPSITSTIKSY; QGTSPSITSTIKSYK; GTSPSITSTIKSYKE;
TSPSITSTIKSYKEL; SPSITSTIKSYKELA; PSITSTIKSYKELAK;
SITSTIKSYKELAKE; ITSTIKSYKELAKEK; TSTIKSYKELAKEKI;
STIKSYKELAKEKIN; TIKSYKELAKEKINN; IKSYKELAKEKINNG;
KSYKELAKEKINNGL; SYKELAKEKINNGLN; YKELAKEKINNGLNI;
KELAKEKINNGLNIV; ELAKEKINNGLNIVQ; LAKEKINNGLNIVQK;
AKEKINNGLNIVQKI; KEKINNGLNIVQKIT; EKINNGLNIVQKITQ;
KINNGLNIVQKITQN; INNGLNIVQKITQNI; NNGLNIVQKITQNID;
NGLNIVQKITQNIDN; GLNIVQKITQNIDNI; LNIVQKITQNIDNIT;
NIVQKITQNIDNITE; IVQKITQNIDNITEN; VQKITQNIDNITENL;
QKITQNIDNITENLN; KITQNIDNITENLNS; ITQNIDNITENLNSK;
TQNIDNITENLNSKE; QNIDNITENLNSKET; NIDNITENLNSKETP;
IDNITENLNSKETPK; DNITENLNSKETPKE; NITENLNSKETPKEI;
ITENLNSKETPKEIS; TENLNSKETPKEISG; ENLNSKETPKEISGK;
NLNSKETPKEISGKE; LNSKETPKEISGKEV; NSKETPKEISGKEVE;
SKETPKEISGKEVEE; KETPKEISGKEVEEK; ETPKEISGKEVEEKI;
TPKEISGKEVEEKIT; PKEISGKEVEEKITH; KEISGKEVEEKITHP;
EISGKEVEEKITHPI; ISGKEVEEKITHPIF; SGKEVEEKITHPIFD;
GKEVEEKITHPIFDH; KEVEEKITHPIFDHI; EVEEKITHPIFDHIT;
VEEKITHPIFDHITG; EEKITHPIFDHITGS; EKITHPIFDHITGSG;
KITHPIFDHITGSGN; ITHPIFDHITGSGNN; THPIFDHITGSGNNP;
HPIFDHITGSGNNPG; PIFDHITGSGNNPGQ; IFDHITGSGNNPGQD;
FDHITGSGNNPGQDS; DHITGSGNNPGQDSI; HITGSGNNPGQDSIS;
ITGSGNNPGQDSISN; TGSGNNPGQDSISNT; GSGNNPGQDSISNTW;
SGNNPGQDSISNTWG; GNNPGQDSISNTWGE; NNPGQDSISNTWGEG;
NPGQDSISNTWGEGL; PGQDSISNTWGEGLE; GQDSISNTWGEGLEI;
QDSISNTWGEGLEIG; DSISNTWGEGLEIGG; SISNTWGEGLEIGGD;

ISNTWGEGLEIGGDS; SNTWGEGLEIGGDSN; NTWGEGLEIGGDSNF;
TWGEGLEIGGDSNFF; WGEGLEIGGDSNFFT; GEGLEIGGDSNFFTN;
EGLEIGGDSNFFTNL; GLEIGGDSNFFTNLE; LEIGGDSNFFTNLEE;
EIGGDSNFFTNLEEV; IGGDSNFFTNLEEVR; GGDSNFFTNLEEVRS;
GDSNFFTNLEEVRSS; DSNFFTNLEEVRSSI; SNFFTNLEEVRSSIR;
NFFTNLEEVRSSIRT; FFTNLEEVRSSIRTK; FTNLEEVRSSIRTKI;
TNLEEVRSSIRTKIK; NLEEVRSSIRTKIKV; LEEVRSSIRTKIKVS;
EEVRSSIRTKIKVSD; EVRSSIRTKIKVSDG; VRSSIRTKIKVSDGT;
RSSIRTKIKVSDGTE; SSIRTKIKVSDGTEQ; SIRTKIKVSDGTEQT;
IRTKIKVSDGTEQTK; RTKIKVSDGTEQTKD; TKIKVSDGTEQTKDK;
KIKVSDGTEQTKDKV; IKVSDGTEQTKDKVE; KVSDGTEQTKDKVEI;
VSDGTEQTKDKVEID; SDGTEQTKDKVEIDE; DGTEQTKDKVEIDEI;
GTEQTKDKVEIDEII; TEQTKDKVEIDEIIE; EQTKDKVEIDEIIED;
QTKDKVEIDEIIEDL; TKDKVEIDEIIEDLQ; KDKVEIDEIIEDLQK;
DKVEIDEIIEDLQKL; KVEIDEIIEDLQKLK; VEIDEIIEDLQKLKE;
EIDEIIEDLQKLKEF; IDEIIEDLQKLKEFL; DEIIEDLQKLKEFLE;
EIIEDLQKLKEFLEK; IIEDLQKLKEFLEKL; IEDLQKLKEFLEKLK;
EDLQKLKEFLEKLKK; DLQKLKEFLEKLKKY; LQKLKEFLEKLKKYL;
QKLKEFLEKLKKYLK; KLKEFLEKLKKYLKD; LKEFLEKLKKYLKDT;
KEFLEKLKKYLKDTN; EFLEKLKKYLKDTNN; FLEKLKKYLKDTNNL;
LEKLKKYLKDTNNLS; EKLKKYLKDTNNLSA; KLKKYLKDTNNLSAI;
LKKYLKDTNNLSAIE; KKYLKDTNNLSAIEE; KYLKDTNNLSAIEES;
YLKDTNNLSAIEESV; LKDTNNLSAIEESVK; KDTNNLSAIEESVKG;
DTNNLSAIEESVKGL; TNNLSAIEESVKGLS;

16 mers:
MCAFLLLNLVNCKFDS; CAFLLLNLVNCKFDSL; AFLLLNLVNCKFDSLN;
FLLLNLVNCKFDSLNL; LLLNLVNCKFDSLNLS; LLNLVNCKFDSLNLST;
LNLVNCKFDSLNLSTK; NLVNCKFDSLNLSTKS; LVNCKFDSLNLSTKSV;
VNCKFDSLNLSTKSVD; NCKFDSLNLSTKSVDD; CKFDSLNLSTKSVDDK;
KFDSLNLSTKSVDDKN; FDSLNLSTKSVDDKNN; DSLNLSTKSVDDKNNS;
SLNLSTKSVDDKNNSI; LNLSTKSVDDKNNSIA; NLSTKSVDDKNNSIAK;
LSTKSVDDKNNSIAKL; STKSVDDKNNSIAKLL; TKSVDDKNNSIAKLLQ;
KSVDDKNNSIAKLLQH; SVDDKNNSIAKLLQHL; VDDKNNSIAKLLQHLS;
DDKNNSIAKLLQHLSK; DKNNSIAKLLQHLSKS; KNNSIAKLLQHLSKSE;
NNSIAKLLQHLSKSED; NSIAKLLQHLSKSEDQ; SIAKLLQHLSKSEDQA;
IAKLLQHLSKSEDQAN; AKLLQHLSKSEDQANK; KLLQHLSKSEDQANKT;
LLQHLSKSEDQANKTS; LQHLSKSEDQANKTST; QHLSKSEDQANKTSTS;
HLSKSEDQANKTSTSE; LSKSEDQANKTSTSED; SKSEDQANKTSTSEDQ;
KSEDQANKTSTSEDQK; SEDQANKTSTSEDQKE; EDQANKTSTSEDQKEL;
DQANKTSTSEDQKELE; QANKTSTSEDQKELEI; ANKTSTSEDQKELEIT;
NKTSTSEDQKELEITE; KTSTSEDQKELEITEN; TSTSEDQKELEITENK;
STSEDQKELEITENKE; TSEDQKELEITENKEQ; SEDQKELEITENKEQE;
EDQKELEITENKEQEH; DQKELEITENKEQEHE; QKELEITENKEQEHEK;
KELEITENKEQEHEKL; ELEITENKEQEHEKLS; LEITENKEQEHEKLSQ;
EITENKEQEHEKLSQV; ITENKEQEHEKLSQVA; TENKEQEHEKLSQVAQ;
ENKEQEHEKLSQVAQH; NKEQEHEKLSQVAQHA; KEQEHEKLSQVAQHAP;
EQEHEKLSQVAQHAPN; QEHEKLSQVAQHAPNS; EHEKLSQVAQHAPNSK;
HEKLSQVAQHAPNSKI; EKLSQVAQHAPNSKIE; KLSQVAQHAPNSKIEK;
LSQVAQHAPNSKIEKV; SQVAQHAPNSKIEKVK; QVAQHAPNSKIEKVKS;
VAQHAPNSKIEKVKSD; AQHAPNSKIEKVKSDG; QHAPNSKIEKVKSDGK;

| | |
|---|---|
| HAPNSKIEKVKSDGKP; APNSKIEKVKSDGKPV; PNSKIEKVKSDGKPVP;<br>NSKIEKVKSDGKPVPG; SKIEKVKSDGKPVPGD; KIEKVKSDGKPVPGDK;<br>IEKVKSDGKPVPGDKI; EKVKSDGKPVPGDKIL; KVKSDGKPVPGDKILS;<br>VKSDGKPVPGDKILSS; KSDGKPVPGDKILSSN; SDGKPVPGDKILSSNK;<br>DGKPVPGDKILSSNKD; GKPVPGDKILSSNKDI; KPVPGDKILSSNKDIY;<br>PVPGDKILSSNKDIYN; VPGDKILSSNKDIYNS; PGDKILSSNKDIYNSY;<br>GDKILSSNKDIYNSYI; DKILSSNKDIYNSYIP; KILSSNKDIYNSYIPE;<br>ILSSNKDIYNSYIPEV; LSSNKDIYNSYIPEVK; SSNKDIYNSYIPEVKE;<br>SNKDIYNSYIPEVKEE; NKDIYNSYIPEVKEEI; KDIYNSYIPEVKEEIV;<br>DIYNSYIPEVKEEIVY; IYNSYIPEVKEEIVYE; YNSYIPEVKEEIVYEI;<br>NSYIPEVKEEIVYEIL; SYIPEVKEEIVYEILE; YIPEVKEEIVYEILEE;<br>IPEVKEEIVYEILEEV; PEVKEEIVYEILEEVI; EVKEEIVYEILEEVII;<br>VKEEIVYEILEEVIIP; KEEIVYEILEEVIIPE; EEIVYEILEEVIIPET;<br>EIVYEILEEVIIPETK; IVYEILEEVIIPETKI; VYEILEEVIIPETKIP;<br>YEILEEVIIPETKIPE; EILEEVIIPETKIPEI; ILEEVIIPETKIPEIT;<br>LEEVIIPETKIPEITE; EEVIIPETKIPEITEE; EVIIPETKIPEITEEV;<br>VIIPETKIPEITEEVI; IIPETKIPEITEEVIM; IPETKIPEITEEVIMP;<br>PETKIPEITEEVIMPI; ETKIPEITEEVIMPIP; TKIPEITEEVIMPIPQ;<br>KIPEITEEVIMPIPQT; IPEITEEVIMPIPQTI; PEITEEVIMPIPQTID;<br>EITEEVIMPIPQTIDF; ITEEVIMPIPQTIDFY; TEEVIMPIPQTIDFYI;<br>EEVIMPIPQTIDFYIE; EVIMPIPQTIDFYIEP; VIMPIPQTIDFYIEPR;<br>IMPIPQTIDFYIEPRP; MPIPQTIDFYIEPRPI; PIPQTIDFYIEPRPIS;<br>IPQTIDFYIEPRPISS; PQTIDFYIEPRPISSF; QTIDFYIEPRPISSFL;<br>TIDFYIEPRPISSFLT; IDFYIEPRPISSFLTQ; DFYIEPRPISSFLTQG;<br>FYIEPRPISSFLTQGT; YIEPRPISSFLTQGTS; IEPRPISSFLTQGTSP;<br>EPRPISSFLTQGTSPS; PRPISSFLTQGTSPSI; RPISSFLTQGTSPSIT;<br>PISSFLTQGTSPSITS; ISSFLTQGTSPSITST; SSFLTQGTSPSITSTI;<br>SFLTQGTSPSITSTIK; FLTQGTSPSITSTIKS; LTQGTSPSITSTIKSY;<br>TQGTSPSITSTIKSYK; QGTSPSITSTIKSYKE; GTSPSITSTIKSYKEL;<br>TSPSITSTIKSYKELA; SPSITSTIKSYKELAK; PSITSTIKSYKELAKE;<br>SITSTIKSYKELAKEK; ITSTIKSYKELAKEKI; TSTIKSYKELAKEKIN;<br>STIKSYKELAKEKINN; TIKSYKELAKEKINNG; IKSYKELAKEKINNGL;<br>KSYKELAKEKINNGLN; SYKELAKEKINNGLNI; YKELAKEKINNGLNIV;<br>KELAKEKINNGLNIVQ; ELAKEKINNGLNIVQK; LAKEKINNGLNIVQKI;<br>AKEKINNGLNIVQKIT; KEKINNGLNIVQKITQ; EKINNGLNIVQKITQN;<br>KINNGLNIVQKITQNI; INNGLNIVQKITQNID; NNGLNIVQKITQNIDN;<br>NGLNIVQKITQNIDNI; GLNIVQKITQNIDNIT; LNIVQKITQNIDNITE;<br>NIVQKITQNIDNITEN; IVQKITQNIDNITENL; VQKITQNIDNITENLN;<br>QKITQNIDNITENLNS; KITQNIDNITENLNSK; ITQNIDNITENLNSKE;<br>TQNIDNITENLNSKET; QNIDNITENLNSKETP; NIDNITENLNSKETPK;<br>IDNITENLNSKETPKE; DNITENLNSKETPKEI; NITENLNSKETPKEIS;<br>ITENLNSKETPKEISG; TENLNSKETPKEISGK; ENLNSKETPKEISGKE;<br>NLNSKETPKEISGKEV; LNSKETPKEISGKEVE; NSKETPKEISGKEVEE;<br>SKETPKEISGKEVEEK; KETPKEISGKEVEEKI; ETPKEISGKEVEEKIT;<br>TPKEISGKEVEEKITH; PKEISGKEVEEKITHP; KEISGKEVEEKITHPI;<br>EISGKEVEEKITHPIF; ISGKEVEEKITHPIFD; SGKEVEEKITHPIFDH;<br>GKEVEEKITHPIFDHI; KEVEEKITHPIFDHIT; EVEEKITHPIFDHITG;<br>VEEKITHPIFDHITGS; EEKITHPIFDHITGSG; EKITHPIFDHITGSGN;<br>KITHPIFDHITGSGNN; ITHPIFDHITGSGNNP; THPIFDHITGSGNNPG;<br>HPIFDHITGSGNNPGQ; PIFDHITGSGNNPGQD; IFDHITGSGNNPGQDS;<br>FDHITGSGNNPGQDSI; DHITGSGNNPGQDSIS; HITGSGNNPGQDSISN; |

| | |
|---|---|
| | ITGSGNNPGQDSISNT; TGSGNNPGQDSISNTW; GSGNNPGQDSISNTWG; SGNNPGQDSISNTWGE; GNNPGQDSISNTWGEG; NNPGQDSISNTWGEGL; NPGQDSISNTWGEGLE; PGQDSISNTWGEGLEI; GQDSISNTWGEGLEIG; QDSISNTWGEGLEIGG; DSISNTWGEGLEIGGD; SISNTWGEGLEIGGDS; ISNTWGEGLEIGGDSN; SNTWGEGLEIGGDSNF; NTWGEGLEIGGDSNFF; TWGEGLEIGGDSNFFT; WGEGLEIGGDSNFFTN; GEGLEIGGDSNFFTNL; EGLEIGGDSNFFTNLE; GLEIGGDSNFFTNLEE; LEIGGDSNFFTNLEEV; EIGGDSNFFTNLEEVR; IGGDSNFFTNLEEVRS; GGDSNFFTNLEEVRSS; GDSNFFTNLEEVRSSI; DSNFFTNLEEVRSSIR; SNFFTNLEEVRSSIRT; NFFTNLEEVRSSIRTK; FFTNLEEVRSSIRTKI; FTNLEEVRSSIRTKIK; TNLEEVRSSIRTKIKV; NLEEVRSSIRTKIKVS; LEEVRSSIRTKIKVSD; EEVRSSIRTKIKVSDG; EVRSSIRTKIKVSDGT; VRSSIRTKIKVSDGTE; RSSIRTKIKVSDGTEQ; SSIRTKIKVSDGTEQT; SIRTKIKVSDGTEQTK; IRTKIKVSDGTEQTKD; RTKIKVSDGTEQTKDK; TKIKVSDGTEQTKDKV; KIKVSDGTEQTKDKVE; IKVSDGTEQTKDKVEI; KVSDGTEQTKDKVEID; VSDGTEQTKDKVEIDE; SDGTEQTKDKVEIDEI; DGTEQTKDKVEIDEII; GTEQTKDKVEIDEIIE; TEQTKDKVEIDEIIED; EQTKDKVEIDEIIEDL; QTKDKVEIDEIIEDLQ; TKDKVEIDEIIEDLQK; KDKVEIDEIIEDLQKL; DKVEIDEIIEDLQKLK; KVEIDEIIEDLQKLKE; VEIDEIIEDLQKLKEF; EIDEIIEDLQKLKEFL; IDEIIEDLQKLKEFLE; DEIIEDLQKLKEFLEK; EIIEDLQKLKEFLEKL; IIEDLQKLKEFLEKLK; IEDLQKLKEFLEKLKK; EDLQKLKEFLEKLKKY; DLQKLKEFLEKLKKYL; LQKLKEFLEKLKKYLK; QKLKEFLEKLKKYLKD; KLKEFLEKLKKYLKDT; LKEFLEKLKKYLKDTN; KEFLEKLKKYLKDTNN; EFLEKLKKYLKDTNNL; FLEKLKKYLKDTNNLS; LEKLKKYLKDTNNLSA; EKLKKYLKDTNNLSAI; KLKKYLKDTNNLSAIE; LKKYLKDTNNLSAIEE; KKYLKDTNNLSAIEES; KYLKDTNNLSAIEESV; YLKDTNNLSAIEESVK; LKDTNNLSAIEESVKG; KDTNNLSAIEESVKGL; DTNNLSAIEESVKGLS; |
| Seq 20 | 13 mers:<br>MIKMSLNYTEINT; IKMSLNYTEINTL; KMSLNYTEINTLI; MSLNYTEINTLIK; SLNYTEINTLIKE; LNYTEINTLIKEI; NYTEINTLIKEIP; YTEINTLIKEIPF; TEINTLIKEIPFT; EINTLIKEIPFTN; INTLIKEIPFTNS; NTLIKEIPFTNSL; TLIKEIPFTNSLI; LIKEIPFTNSLIT; IKEIPFTNSLITK; KEIPFTNSLITKI; EIPFTNSLITKII; IPFTNSLITKIIQ; PFTNSLITKIIQP; FTNSLITKIIQPD; TNSLITKIIQPDY; NSLITKIIQPDYK; SLITKIIQPDYKS; LITKIIQPDYKSL; ITKIIQPDYKSLV; TKIIQPDYKSLVL; KIIQPDYKSLVLE; IIQPDYKSLVLEI; IQPDYKSLVLEIY; QPDYKSLVLEIYN; PDYKSLVLEIYNK; DYKSLVLEIYNKI; YKSLVLEIYNKID; KSLVLEIYNKIDN; SLVLEIYNKIDNK; LVLEIYNKIDNKK; VLEIYNKIDNKKF; LEIYNKIDNKKFK; EIYNKIDNKKFKI; IYNKIDNKKFKIL; YNKIDNKKFKILI; NKIDNKKFKILIC; KIDNKKFKILICL; IDNKKFKILICLN; DNKKFKILICLNP; NKKFKILICLNPN; KKFKILICLNPNT; KFKILICLNPNTT; FKILICLNPNTTR; KILICLNPNTTRF; ILICLNPNTTRFH; LICLNPNTTRFHI; ICLNPNTTRFHIT; CLNPNTTRFHITK; LNPNTTRFHITKK; NPNTTRFHITKKN; PNTTRFHITKKNF; NTTRFHITKKNFK; TTRFHITKKNFKK; TRFHITKKNFKKN; |

EP 2 254 592 B1

RFHITKKNFKKNA; FHITKKNFKKNAL; HITKKNFKKNALK;
ITKKNFKKNALKL; TKKNFKKNALKLR; KKNFKKNALKLRF;
KNFKKNALKLRFS; NFKKNALKLRFSD; FKKNALKLRFSDF;
KKNALKLRFSDFL; KNALKLRFSDFLK; NALKLRFSDFLKS;
ALKLRFSDFLKSK; LKLRFSDFLKSKI; KLRFSDFLKSKIQ;
LRFSDFLKSKIQN; RFSDFLKSKIQNG; FSDFLKSKIQNGK;
SDFLKSKIQNGKI; DFLKSKIQNGKII; FLKSKIQNGKIIK;
LKSKIQNGKIIKA; KSKIQNGKIIKAF; SKIQNGKIIKAFQ;
KIQNGKIIKAFQM; IQNGKIIKAFQMK; QNGKIIKAFQMKN;
NGKIIKAFQMKNE; GKIIKAFQMKNER; KIIKAFQMKNERI;
IIKAFQMKNERII; IKAFQMKNERIIS; KAFQMKNERIISL;
AFQMKNERIISLE; FQMKNERIISLEI; QMKNERIISLEIL;
MKNERIISLEILQ; KNERIISLEILQK; NERIISLEILQKD;
ERIISLEILQKDM; RIISLEILQKDMI; IISLEILQKDMII;
ISLEILQKDMIIL; SLEILQKDMIILF; LEILQKDMIILFI;
EILQKDMIILFIK; ILQKDMIILFIKL; LQKDMIILFIKLW;
QKDMIILFIKLWP; KDMIILFIKLWPS; DMIILFIKLWPSS;
MIILFIKLWPSSP; IILFIKLWPSSPN; ILFIKLWPSSPNI;
LFIKLWPSSPNII; FIKLWPSSPNIIA; IKLWPSSPNIIAT;
KLWPSSPNIIATN; LWPSSPNIIATNS; WPSSPNIIATNSN;
PSSPNIIATNSNF; SSPNIIATNSNFK; SPNIIATNSNFKI;
PNIIATNSNFKIL; NIIATNSNFKILD; IIATNSNFKILDA;
IATNSNFKILDAY; ATNSNFKILDAYY; TNSNFKILDAYYR;
NSNFKILDAYYRR; SNFKILDAYYRRP; NFKILDAYYRRPK;
FKILDAYYRRPKI; KILDAYYRRPKIK; ILDAYYRRPKIKE;
LDAYYRRPKIKET; DAYYRRPKIKETT; AYYRRPKIKETTG;
YRRPKIKETTGE; YRRPKIKETTGEI; RRPKIKETTGEIF;
RPKIKETTGEIFL; PKIKETTGEIFLK; KIKETTGEIFLKA;
IKETTGEIFLKAK; KETTGEIFLKAKE; ETTGEIFLKAKEI;
TTGEIFLKAKEIH; TGEIFLKAKEIHE; GEIFLKAKEIHES;
EIFLKAKEIHESN; IFLKAKEIHESNK; FLKAKEIHESNKM;
LKAKEIHESNKMS; KAKEIHESNKMSD; AKEIHESNKMSDK;
KEIHESNKMSDKK; EIHESNKMSDKKI; IHESNKMSDKKIM;
HESNKMSDKKIME; ESNKMSDKKIMEL; SNKMSDKKIMELK;
NKMSDKKIMELKE; KMSDKKIMELKEE; MSDKKIMELKEEY;
SDKKIMELKEEYN; DKKIMELKEEYNN; KKIMELKEEYNNT;
KIMELKEEYNNTS; IMELKEEYNNTSY; MELKEEYNNTSYT;
ELKEEYNNTSYTS; LKEEYNNTSYTSY; KEEYNNTSYTSYS;
EEYNNTSYTSYSE; EYNNTSYTSYSEF; YNNTSYTSYSEFL;
NNTSYTSYSEFLE; NTSYTSYSEFLEN; TSYTSYSEFLENY;
SYTSYSEFLENYY; YTSYSEFLENYYE; TSYSEFLENYYES;
SYSEFLENYYESL; YSEFLENYYESLN; SEFLENYYESLND;
EFLENYYESLNDQ; FLENYYESLNDQI; LENYYESLNDQIK;
ENYYESLNDQIKK; NYYESLNDQIKKT; YYESLNDQIKKTN;
YESLNDQIKKTNI; ESLNDQIKKTNIK; SLNDQIKKTNIKE;
LNDQIKKTNIKEL; NDQIKKTNIKELL; DQIKKTNIKELLI;
QIKKTNIKELLIE; IKKTNIKELLIEK; KKTNIKELLIEKY;
KTNIKELLIEKYK; TNIKELLIEKYKK; NIKELLIEKYKKE;
IKELLIEKYKKEL; KELLIEKYKKELI; ELLIEKYKKELIV;
LLIEKYKKELIVL; LIEKYKKELIVLE; IEKYKKELIVLEK;
EKYKKELIVLEKR; KYKKELIVLEKRI; YKKELIVLEKRID;

KKELIVLEKRIDS; KELIVLEKRIDSL; ELIVLEKRIDSLK;
LIVLEKRIDSLKQ; IVLEKRIDSLKQQ; VLEKRIDSLKQQI;
LEKRIDSLKQQIK; EKRIDSLKQQIKL; KRIDSLKQQIKLL;
RIDSLKQQIKLLE; IDSLKQQIKLLEN; DSLKQQIKLLENI;
SLKQQIKLLENIE; LKQQIKLLENIEN; KQQIKLLENIENE;
QQIKLLENIENEK; QIKLLENIENEKE; IKLLENIENEKEK;
KLLENIENEKEKG; LLENIENEKEKGE; LENIENEKEKGEL;
ENIENEKEKGELI; NIENEKEKGELIL; IENEKEKGELILL;
ENEKEKGELILLN; NEKEKGELILLNI; EKEKGELILLNIN;
KEKGELILLNINK; EKGELILLNINKI; KGELILLNINKIQ;
GELILLNINKIQK; ELILLNINKIQKG; LILLNINKIQKGI;
ILLNINKIQKGIK; LLNINKIQKGIKE; LNINKIQKGIKEI;
NINKIQKGIKEIN; INKIQKGIKEINL; NKIQKGIKEINLL;
KIQKGIKEINLLN; IQKGIKEINLLNY; QKGIKEINLLNYK;
KGIKEINLLNYKE; GIKEINLLNYKEE; IKEINLLNYKEEK;
KEINLLNYKEEKI; EINLLNYKEEKIK; INLLNYKEEKIKI;
NLLNYKEEKIKIS; LLNYKEEKIKISL; LNYKEEKIKISLN;
NYKEEKIKISLNQ; YKEEKIKISLNQS; KEEKIKISLNQSL;
EEKIKISLNQSLS; EKIKISLNQSLSP; KIKISLNQSLSPK;
IKISLNQSLSPKE; KISLNQSLSPKEN; ISLNQSLSPKENA;
SLNQSLSPKENAL; LNQSLSPKENALQ; NQSLSPKENALQY;
QSLSPKENALQYF; SLSPKENALQYFK; LSPKENALQYFKA;
SPKENALQYFKAY; PKENALQYFKAYK; KENALQYFKAYKK;
ENALQYFKAYKKG; NALQYFKAYKKGK; ALQYFKAYKKGKN;
LQYFKAYKKGKNS; QYFKAYKKGKNSF; YFKAYKKGKNSFK;
FKAYKKGKNSFKT; KAYKKGKNSFKTI; AYKKGKNSFKTIQ;
YKKGKNSFKTIQN; KKGKNSFKTIQNQ; KGKNSFKTIQNQL;
GKNSFKTIQNQLK; KNSFKTIQNQLKD; NSFKTIQNQLKDN;
SFKTIQNQLKDNL; FKTIQNQLKDNLD; KTIQNQLKDNLDK;
TIQNQLKDNLDKF; IQNQLKDNLDKFN; QNQLKDNLDKFNL;
NQLKDNLDKFNLI; QLKDNLDKFNLIQ; LKDNLDKFNLIQS;
KDNLDKFNLIQSK; DNLDKFNLIQSKI; NLDKFNLIQSKIT;
LDKFNLIQSKITM; DKFNLIQSKITML; KFNLIQSKITMLK;
FNLIQSKITMLKV; NLIQSKITMLKVE; LIQSKITMLKVEN;
IQSKITMLKVENL; QSKITMLKVENLI; SKITMLKVENLIP;
KITMLKVENLIPE; ITMLKVENLIPEE; TMLKVENLIPEEE;
MLKVENLIPEEEY; LKVENLIPEEEYN; KVENLIPEEEYNQ;
VENLIPEEEYNQE; ENLIPEEEYNQEK; NLIPEEEYNQEKT;
LIPEEEYNQEKTA; IPEEEYNQEKTAI; PEEEYNQEKTAIK;
EEEYNQEKTAIKE; EEYNQEKTAIKEK; EYNQEKTAIKEKE;
YNQEKTAIKEKEK; NQEKTAIKEKEKT; QEKTAIKEKEKTP;
EKTAIKEKEKTPK; KTAIKEKEKTPKI; TAIKEKEKTPKIG;
AIKEKEKTPKIGL; IKEKEKTPKIGLH; KEKEKTPKIGLHF;
EKEKTPKIGLHFT; KEKTPKIGLHFTY; EKTPKIGLHFTYC;
KTPKIGLHFTYCG; TPKIGLHFTYCGF; PKIGLHFTYCGFE;
KIGLHFTYCGFEI; IGLHFTYCGFEIL; GLHFTYCGFEILI;
LHFTYCGFEILIG; HFTYCGFEILIGR; FTYCGFEILIGRN;
TYCGFEILIGRNA; YCGFEILIGRNAK; CGFEILIGRNAKE;
GFEILIGRNAKEN; FEILIGRNAKEND; EILIGRNAKENDK;
ILIGRNAKENDKL; LIGRNAKENDKLL; IGRNAKENDKLLR;
GRNAKENDKLLRH; RNAKENDKLLRHC; NAKENDKLLRHCV;

AKENDKLLRHCVK; KENDKLLRHCVKG; ENDKLLRHCVKGN;
NDKLLRHCVKGND; DKLLRHCVKGNDY; KLLRHCVKGNDYW;
LLRHCVKGNDYWL; LRHCVKGNDYWLH; RHCVKGNDYWLHT;
HCVKGNDYWLHTR; CVKGNDYWLHTRD; VKGNDYWLHTRDY;
KGNDYWLHTRDYP; GNDYWLHTRDYPG; NDYWLHTRDYPGA;
DYWLHTRDYPGAY; YWLHTRDYPGAYV; WLHTRDYPGAYVF;
LHTRDYPGAYVFI; HTRDYPGAYVFIK; TRDYPGAYVFIKN;
RDYPGAYVFIKNQ; DYPGAYVFIKNQK; YPGAYVFIKNQKN;
PGAYVFIKNQKNK; GAYVFIKNQKNKT; AYVFIKNQKNKTP;
YVFIKNQKNKTPS; VFIKNQKNKTPSL; FIKNQKNKTPSLD;
IKNQKNKTPSLDV; KNQKNKTPSLDVL; NQKNKTPSLDVLL;
QKNKTPSLDVLLG; KNKTPSLDVLLGA; NKTPSLDVLLGAG;
KTPSLDVLLGAGN; TPSLDVLLGAGNL; PSLDVLLGAGNLC;
SLDVLLGAGNLCV; LDVLLGAGNLCVF; DVLLGAGNLCVFY;
VLLGAGNLCVFYT; LLGAGNLCVFYTK; LGAGNLCVFYTKL;
GAGNLCVFYTKLA; AGNLCVFYTKLAK; GNLCVFYTKLAKK;
NLCVFYTKLAKKS; LCVFYTKLAKKSG; CVFYTKLAKKSGK;
VFYTKLAKKSGKA; FYTKLAKKSGKAD; YTKLAKKSGKADL;
TKLAKKSGKADLY; KLAKKSGKADLYY; LAKKSGKADLYYT;
AKKSGKADLYYTQ; KKSGKADLYYTQV; KSGKADLYYTQVK;
SGKADLYYTQVKN; GKADLYYTQVKNL; KADLYYTQVKNLR;
ADLYYTQVKNLRR; DLYYTQVKNLRRV; LYYTQVKNLRRVK;
YYTQVKNLRRVKN; YTQVKNLRRVKNK; TQVKNLRRVKNKK;
QVKNLRRVKNKKL; VKNLRRVKNKKLG; KNLRRVKNKKLGL;
NLRRVKNKKLGLV; LRRVKNKKLGLVI; RRVKNKKLGLVIP;
RVKNKKLGLVIPK; VKNKKLGLVIPKA; KNKKLGLVIPKAE;
NKKLGLVIPKAEK; KKLGLVIPKAEKN; KLGLVIPKAEKNL;
LGLVIPKAEKNLH; GLVIPKAEKNLHI; LVIPKAEKNLHIK;
VIPKAEKNLHIKL; IPKAEKNLHIKLD; PKAEKNLHIKLDE;
KAEKNLHIKLDEN; AEKNLHIKLDENL; EKNLHIKLDENLI;
KNLHIKLDENLIK; NLHIKLDENLIKK; LHIKLDENLIKKI;
HIKLDENLIKKIK; IKLDENLIKKIKN; KLDENLIKKIKNQ;
LDENLIKKIKNQT;

14 mers:
MIKMSLNYTEINTL; IKMSLNYTEINTLI; KMSLNYTEINTLIK;
MSLNYTEINTLIKE; SLNYTEINTLIKEI; LNYTEINTLIKEIP;
NYTEINTLIKEIPF; YTEINTLIKEIPFT; TEINTLIKEIPFTN;
EINTLIKEIPFTNS; INTLIKEIPFTNSL; NTLIKEIPFTNSLI;
TLIKEIPFTNSLIT; LIKEIPFTNSLITK; IKEIPFTNSLITKI;
KEIPFTNSLITKII; EIPFTNSLITKIIQ; IPFTNSLITKIIQP;
PFTNSLITKIIQPD; FTNSLITKIIQPDY; TNSLITKIIQPDYK;
NSLITKIIQPDYKS; SLITKIIQPDYKSL; LITKIIQPDYKSLV;
ITKIIQPDYKSLVL; TKIIQPDYKSLVLE; KIIQPDYKSLVLEI;
IIQPDYKSLVLEIY; IQPDYKSLVLEIYN; QPDYKSLVLEIYNK;
PDYKSLVLEIYNKI; DYKSLVLEIYNKID; YKSLVLEIYNKIDN;
KSLVLEIYNKIDNK; SLVLEIYNKIDNKK; LVLEIYNKIDNKKF;
VLEIYNKIDNKKFK; LEIYNKIDNKKFKI; EIYNKIDNKKFKIL;
IYNKIDNKKFKILI; YNKIDNKKFKILIC; NKIDNKKFKILICL;
KIDNKKFKILICLN; IDNKKFKILICLNP; DNKKFKILICLNPN;
NKKFKILICLNPNT; KKFKILICLNPNTT; KFKILICLNPNTTR;

| | | |
|---|---|---|
| FKILICLNPNTTRF; | KILICLNPNTTRFH; | ILICLNPNTTRFHI; |
| LICLNPNTTRFHIT; | ICLNPNTTRFHITK; | CLNPNTTRFHITKK; |
| LNPNTTRFHITKKN; | NPNTTRFHITKKNF; | PNTTRFHITKKNFK; |
| NTTRFHITKKNFKK; | TTRFHITKKNFKKN; | TRFHITKKNFKKNA; |
| RFHITKKNFKKNAL; | FHITKKNFKKNALK; | HITKKNFKKNALKL; |
| ITKKNFKKNALKLR; | TKKNFKKNALKLRF; | KKNFKKNALKLRFS; |
| KNFKKNALKLRFSD; | NFKKNALKLRFSDF; | FKKNALKLRFSDFL; |
| KKNALKLRFSDFLK; | KNALKLRFSDFLKS; | NALKLRFSDFLKSK; |
| ALKLRFSDFLKSKI; | LKLRFSDFLKSKIQ; | KLRFSDFLKSKIQN; |
| LRFSDFLKSKIQNG; | RFSDFLKSKIQNGK; | FSDFLKSKIQNGKI; |
| SDFLKSKIQNGKII; | DFLKSKIQNGKIIK; | FLKSKIQNGKIIKA; |
| LKSKIQNGKIIKAF; | KSKIQNGKIIKAFQ; | SKIQNGKIIKAFQM; |
| KIQNGKIIKAFQMK; | IQNGKIIKAFQMKN; | QNGKIIKAFQMKNE; |
| NGKIIKAFQMKNER; | GKIIKAFQMKNERI; | KIIKAFQMKNERII; |
| IIKAFQMKNERIIS; | IKAFQMKNERIISL; | KAFQMKNERIISLE; |
| AFQMKNERIISLEI; | FQMKNERIISLEIL; | QMKNERIISLEILQ; |
| MKNERIISLEILQK; | KNERIISLEILQKD; | NERIISLEILQKDM; |
| ERIISLEILQKDMI; | RIISLEILQKDMII; | IISLEILQKDMIIL; |
| ISLEILQKDMIILF; | SLEILQKDMIILFI; | LEILQKDMIILFIK; |
| EILQKDMIILFIKL; | ILQKDMIILFIKLW; | LQKDMIILFIKLWP; |
| QKDMIILFIKLWPS; | KDMIILFIKLWPSS; | DMIILFIKLWPSSP; |
| MIILFIKLWPSSPN; | IILFIKLWPSSPNI; | ILFIKLWPSSPNII; |
| LFIKLWPSSPNIIA; | FIKLWPSSPNIIAT; | IKLWPSSPNIIATN; |
| KLWPSSPNIIATNS; | LWPSSPNIIATNSN; | WPSSPNIIATNSNF; |
| PSSPNIIATNSNFK; | SSPNIIATNSNFKI; | SPNIIATNSNFKIL; |
| PNIIATNSNFKILD; | NIIATNSNFKILDA; | IIATNSNFKILDAY; |
| IATNSNFKILDAYY; | ATNSNFKILDAYYR; | TNSNFKILDAYYRR; |
| NSNFKILDAYYRRP; | SNFKILDAYYRRPK; | NFKILDAYYRRPKI; |
| FKILDAYYRRPKIK; | KILDAYYRRPKIKE; | ILDAYYRRPKIKET; |
| LDAYYRRPKIKETT; | DAYYRRPKIKETTG; | AYYRRPKIKETTGE; |
| YYRRPKIKETTGEI; | YRRPKIKETTGEIF; | RRPKIKETTGEIFL; |
| RPKIKETTGEIFLK; | PKIKETTGEIFLKA; | KIKETTGEIFLKAK; |
| IKETTGEIFLKAKE; | KETTGEIFLKAKEI; | ETTGEIFLKAKEIH; |
| TTGEIFLKAKEIHE; | TGEIFLKAKEIHES; | GEIFLKAKEIHESN; |
| EIFLKAKEIHESNK; | IFLKAKEIHESNKM; | FLKAKEIHESNKMS; |
| LKAKEIHESNKMSD; | KAKEIHESNKMSDK; | AKEIHESNKMSDKK; |
| KEIHESNKMSDKKI; | EIHESNKMSDKKIM; | IHESNKMSDKKIME; |
| HESNKMSDKKIMEL; | ESNKMSDKKIMELK; | SNKMSDKKIMELKE; |
| NKMSDKKIMELKEE; | KMSDKKIMELKEEY; | MSDKKIMELKEEYN; |
| SDKKIMELKEEYNN; | DKKIMELKEEYNNT; | KKIMELKEEYNNTS; |
| KIMELKEEYNNTSY; | IMELKEEYNNTSYT; | MELKEEYNNTSYTS; |
| ELKEEYNNTSYTSY; | LKEEYNNTSYTSYS; | KEEYNNTSYTSYSE; |
| EEYNNTSYTSYSEF; | EYNNTSYTSYSEFL; | YNNTSYTSYSEFLE; |
| NNTSYTSYSEFLEN; | NTSYTSYSEFLENY; | TSYTSYSEFLENYY; |
| SYTSYSEFLENYYE; | YTSYSEFLENYYES; | TSYSEFLENYYESL; |
| SYSEFLENYYESLN; | YSEFLENYYESLND; | SEFLENYYESLNDQ; |
| EFLENYYESLNDQI; | FLENYYESLNDQIK; | LENYYESLNDQIKK; |
| ENYYESLNDQIKKT; | NYYESLNDQIKKTN; | YYESLNDQIKKTNI; |
| YESLNDQIKKTNIK; | ESLNDQIKKTNIKE; | SLNDQIKKTNIKEL; |
| LNDQIKKTNIKELL; | NDQIKKTNIKELLI; | DQIKKTNIKELLIE; |
| QIKKTNIKELLIEK; | IKKTNIKELLIEKY; | KKTNIKELLIEKYK; |

| | | |
|---|---|---|
| KTNIKELLIEKYKK; | TNIKELLIEKYKKE; | NIKELLIEKYKKEL; |
| IKELLIEKYKKELI; | KELLIEKYKKELIV; | ELLIEKYKKELIVL; |
| LLIEKYKKELIVLE; | LIEKYKKELIVLEK; | IEKYKKELIVLEKR; |
| EKYKKELIVLEKRI; | KYKKELIVLEKRID; | YKKELIVLEKRIDS; |
| KKELIVLEKRIDSL; | KELIVLEKRIDSLK; | ELIVLEKRIDSLKQ; |
| LIVLEKRIDSLKQQ; | IVLEKRIDSLKQQI; | VLEKRIDSLKQQIK; |
| LEKRIDSLKQQIKL; | EKRIDSLKQQIKLL; | KRIDSLKQQIKLLE; |
| RIDSLKQQIKLLEN; | IDSLKQQIKLLENI; | DSLKQQIKLLENIE; |
| SLKQQIKLLENIEN; | LKQQIKLLENIENE; | KQQIKLLENIENEK; |
| QQIKLLENIENEKE; | QIKLLENIENEKEK; | IKLLENIENEKEKG; |
| KLLENIENEKEKGE; | LLENIENEKEKGEL; | LENIENEKEKGELI; |
| ENIENEKEKGELIL; | NIENEKEKGELILL; | IENEKEKGELILLN; |
| ENEKEKGELILLNI; | NEKEKGELILLNIN; | EKEKGELILLNINK; |
| KEKGELILLNINKI; | EKGELILLNINKIQ; | KGELILLNINKIQK; |
| GELILLNINKIQKG; | ELILLNINKIQKGI; | LILLNINKIQKGIK; |
| ILLNINKIQKGIKE; | LLNINKIQKGIKEI; | LNINKIQKGIKEIN; |
| NINKIQKGIKEINL; | INKIQKGIKEINLL; | NKIQKGIKEINLLN; |
| KIQKGIKEINLLNY; | IQKGIKEINLLNYK; | QKGIKEINLLNYKE; |
| KGIKEINLLNYKEE; | GIKEINLLNYKEEK; | IKEINLLNYKEEKI; |
| KEINLLNYKEEKIK; | EINLLNYKEEKIKI; | INLLNYKEEKIKIS; |
| NLLNYKEEKIKISL; | LLNYKEEKIKISLN; | LNYKEEKIKISLNQ; |
| NYKEEKIKISLNQS; | YKEEKIKISLNQSL; | KEEKIKISLNQSLS; |
| EEKIKISLNQSLSP; | EKIKISLNQSLSPK; | KIKISLNQSLSPKE; |
| IKISLNQSLSPKEN; | KISLNQSLSPKENA; | ISLNQSLSPKENAL; |
| SLNQSLSPKENALQ; | LNQSLSPKENALQY; | NQSLSPKENALQYF; |
| QSLSPKENALQYFK; | SLSPKENALQYFKA; | LSPKENALQYFKAY; |
| SPKENALQYFKAYK; | PKENALQYFKAYKK; | KENALQYFKAYKKG; |
| ENALQYFKAYKKGK; | NALQYFKAYKKGKN; | ALQYFKAYKKGKNS; |
| LQYFKAYKKGKNSF; | QYFKAYKKGKNSFK; | YFKAYKKGKNSFKT; |
| FKAYKKGKNSFKTI; | KAYKKGKNSFKTIQ; | AYKKGKNSFKTIQN; |
| YKKGKNSFKTIQNQ; | KKGKNSFKTIQNQL; | KGKNSFKTIQNQLK; |
| GKNSFKTIQNQLKD; | KNSFKTIQNQLKDN; | NSFKTIQNQLKDNL; |
| SFKTIQNQLKDNLD; | FKTIQNQLKDNLDK; | KTIQNQLKDNLDKF; |
| TIQNQLKDNLDKFN; | IQNQLKDNLDKFNL; | QNQLKDNLDKFNLI; |
| NQLKDNLDKFNLIQ; | QLKDNLDKFNLIQS; | LKDNLDKFNLIQSK; |
| KDNLDKFNLIQSKI; | DNLDKFNLIQSKIT; | NLDKFNLIQSKITM; |
| LDKFNLIQSKITML; | DKFNLIQSKITMLK; | KFNLIQSKITMLKV; |
| FNLIQSKITMLKVE; | NLIQSKITMLKVEN; | LIQSKITMLKVENL; |
| IQSKITMLKVENLI; | QSKITMLKVENLIP; | SKITMLKVENLIPE; |
| KITMLKVENLIPEE; | ITMLKVENLIPEEE; | TMLKVENLIPEEEY; |
| MLKVENLIPEEEYN; | LKVENLIPEEEYNQ; | KVENLIPEEEYNQE; |
| VENLIPEEEYNQEK; | ENLIPEEEYNQEKT; | NLIPEEEYNQEKTA; |
| LIPEEEYNQEKTAI; | IPEEEYNQEKTAIK; | PEEEYNQEKTAIKE; |
| EEEYNQEKTAIKEK; | EEYNQEKTAIKEKE; | EYNQEKTAIKEKEK; |
| YNQEKTAIKEKEKT; | NQEKTAIKEKEKTP; | QEKTAIKEKEKTPK; |
| EKTAIKEKEKTPKI; | KTAIKEKEKTPKIG; | TAIKEKEKTPKIGL; |
| AIKEKEKTPKIGLH; | IKEKEKTPKIGLHF; | KEKEKTPKIGLHFT; |
| EKEKTPKIGLHFTY; | KEKTPKIGLHFTYC; | EKTPKIGLHFTYCG; |
| KTPKIGLHFTYCGF; | TPKIGLHFTYCGFE; | PKIGLHFTYCGFEI; |
| KIGLHFTYCGFEIL; | IGLHFTYCGFEILI; | GLHFTYCGFEILIG; |
| LHFTYCGFEILIGR; | HFTYCGFEILIGRN; | FTYCGFEILIGRNA; |

TYCGFEILIGRNAK; YCGFEILIGRNAKE; CGFEILIGRNAKEN;
GFEILIGRNAKEND; FEILIGRNAKENDK; EILIGRNAKENDKL;
ILIGRNAKENDKLL; LIGRNAKENDKLLR; IGRNAKENDKLLRH;
GRNAKENDKLLRHC; RNAKENDKLLRHCV; NAKENDKLLRHCVK;
AKENDKLLRHCVKG; KENDKLLRHCVKGN; ENDKLLRHCVKGND;
NDKLLRHCVKGNDY; DKLLRHCVKGNDYW; KLLRHCVKGNDYWL;
LLRHCVKGNDYWLH; LRHCVKGNDYWLHT; RHCVKGNDYWLHTR;
HCVKGNDYWLHTRD; CVKGNDYWLHTRDY; VKGNDYWLHTRDYP;
KGNDYWLHTRDYPG; GNDYWLHTRDYPGA; NDYWLHTRDYPGAY;
DYWLHTRDYPGAYV; YWLHTRDYPGAYVF; WLHTRDYPGAYVFI;
LHTRDYPGAYVFIK; HTRDYPGAYVFIKN; TRDYPGAYVFIKNQ;
RDYPGAYVFIKNQK; DYPGAYVFIKNQKN; YPGAYVFIKNQKNK;
PGAYVFIKNQKNKT; GAYVFIKNQKNKTP; AYVFIKNQKNKTPS;
YVFIKNQKNKTPSL; VFIKNQKNKTPSLD; FIKNQKNKTPSLDV;
IKNQKNKTPSLDVL; KNQKNKTPSLDVLL; NQKNKTPSLDVLLG;
QKNKTPSLDVLLGA; KNKTPSLDVLLGAG; NKTPSLDVLLGAGN;
KTPSLDVLLGAGNL; TPSLDVLLGAGNLC; PSLDVLLGAGNLCV;
SLDVLLGAGNLCVF; LDVLLGAGNLCVFY; DVLLGAGNLCVFYT;
VLLGAGNLCVFYTK; LLGAGNLCVFYTKL; LGAGNLCVFYTKLA;
GAGNLCVFYTKLAK; AGNLCVFYTKLAKK; GNLCVFYTKLAKKS;
NLCVFYTKLAKKSG; LCVFYTKLAKKSGK; CVFYTKLAKKSGKA;
VFYTKLAKKSGKAD; FYTKLAKKSGKADL; YTKLAKKSGKADLY;
TKLAKKSGKADLYY; KLAKKSGKADLYYT; LAKKSGKADLYYTQ;
AKKSGKADLYYTQV; KKSGKADLYYTQVK; KSGKADLYYTQVKN;
SGKADLYYTQVKNL; GKADLYYTQVKNLR; KADLYYTQVKNLRR;
ADLYYTQVKNLRRV; DLYYTQVKNLRRVK; LYYTQVKNLRRVKN;
YYTQVKNLRRVKNK; YTQVKNLRRVKNKK; TQVKNLRRVKNKKL;
QVKNLRRVKNKKLG; VKNLRRVKNKKLGL; KNLRRVKNKKLGLV;
NLRRVKNKKLGLVI; LRRVKNKKLGLVIP; RRVKNKKLGLVIPK;
RVKNKKLGLVIPKA; VKNKKLGLVIPKAE; KNKKLGLVIPKAEK;
NKKLGLVIPKAEKN; KKLGLVIPKAEKNL; KLGLVIPKAEKNLH;
LGLVIPKAEKNLHI; GLVIPKAEKNLHIK; LVIPKAEKNLHIKL;
VIPKAEKNLHIKLD; IPKAEKNLHIKLDE; PKAEKNLHIKLDEN;
KAEKNLHIKLDENL; AEKNLHIKLDENLI; EKNLHIKLDENLIK;
KNLHIKLDENLIKK; NLHIKLDENLIKKI; LHIKLDENLIKKIK;
HIKLDENLIKKIKN; IKLDENLIKKIKNQ; KLDENLIKKIKNQT;

15 mers:
MIKMSLNYTEINTLI; IKMSLNYTEINTLIK; KMSLNYTEINTLIKE;
MSLNYTEINTLIKEI; SLNYTEINTLIKEIP; LNYTEINTLIKEIPF;
NYTEINTLIKEIPFT; YTEINTLIKEIPFTN; TEINTLIKEIPFTNS;
EINTLIKEIPFTNSL; INTLIKEIPFTNSLI; NTLIKEIPFTNSLIT;
TLIKEIPFTNSLITK; LIKEIPFTNSLITKI; IKEIPFTNSLITKII;
KEIPFTNSLITKIIQ; EIPFTNSLITKIIQP; IPFTNSLITKIIQPD;
PFTNSLITKIIQPDY; FTNSLITKIIQPDYK; TNSLITKIIQPDYKS;
NSLITKIIQPDYKSL; SLITKIIQPDYKSLV; LITKIIQPDYKSLVL;
ITKIIQPDYKSLVLE; TKIIQPDYKSLVLEI; KIIQPDYKSLVLEIY;
IIQPDYKSLVLEIYN; IQPDYKSLVLEIYNK; QPDYKSLVLEIYNKI;
PDYKSLVLEIYNKID; DYKSLVLEIYNKIDN; YKSLVLEIYNKIDNK;
KSLVLEIYNKIDNKK; SLVLEIYNKIDNKKF; LVLEIYNKIDNKKFK;
VLEIYNKIDNKKFKI; LEIYNKIDNKKFKIL; EIYNKIDNKKFKILI;

IYNKIDNKKFKILIC; YNKIDNKKFKILICL; NKIDNKKFKILICLN;
KIDNKKFKILICLNP; IDNKKFKILICLNPN; DNKKFKILICLNPNT;
NKKFKILICLNPNTT; KKFKILICLNPNTTR; KFKILICLNPNTTRF;
FKILICLNPNTTRFH; KILICLNPNTTRFHI; ILICLNPNTTRFHIT;
LICLNPNTTRFHITK; ICLNPNTTRFHITKK; CLNPNTTRFHITKKN;
LNPNTTRFHITKKNF; NPNTTRFHITKKNFK; PNTTRFHITKKNFKK;
NTTRFHITKKNFKKN; TTRFHITKKNFKKNA; TRFHITKKNFKKNAL;
RFHITKKNFKKNALK; FHITKKNFKKNALKL; HITKKNFKKNALKLR;
ITKKNFKKNALKLRF; TKKNFKKNALKLRFS; KKNFKKNALKLRFSD;
KNFKKNALKLRFSDF; NFKKNALKLRFSDFL; FKKNALKLRFSDFLK;
KKNALKLRFSDFLKS; KNALKLRFSDFLKSK; NALKLRFSDFLKSKI;
ALKLRFSDFLKSKIQ; LKLRFSDFLKSKIQN; KLRFSDFLKSKIQNG;
LRFSDFLKSKIQNGK; RFSDFLKSKIQNGKI; FSDFLKSKIQNGKII;
SDFLKSKIQNGKIIK; DFLKSKIQNGKIIKA; FLKSKIQNGKIIKAF;
LKSKIQNGKIIKAFQ; KSKIQNGKIIKAFQM; SKIQNGKIIKAFQMK;
KIQNGKIIKAFQMKN; IQNGKIIKAFQMKNE; QNGKIIKAFQMKNER;
NGKIIKAFQMKNERI; GKIIKAFQMKNERII; KIIKAFQMKNERIIS;
IIKAFQMKNERIISL; IKAFQMKNERIISLE; KAFQMKNERIISLEI;
AFQMKNERIISLEIL; FQMKNERIISLEILQ; QMKNERIISLEILQK;
MKNERIISLEILQKD; KNERIISLEILQKDM; NERIISLEILQKDMI;
ERIISLEILQKDMII; RIISLEILQKDMIIL; IISLEILQKDMIILF;
ISLEILQKDMIILFI; SLEILQKDMIILFIK; LEILQKDMIILFIKL;
EILQKDMIILFIKLW; ILQKDMIILFIKLWP; LQKDMIILFIKLWPS;
QKDMIILFIKLWPSS; KDMIILFIKLWPSSP; DMIILFIKLWPSSPN;
MIILFIKLWPSSPNI; IILFIKLWPSSPNII; ILFIKLWPSSPNIIA;
LFIKLWPSSPNIIAT; FIKLWPSSPNIIATN; IKLWPSSPNIIATNS;
KLWPSSPNIIATNSN; LWPSSPNIIATNSNF; WPSSPNIIATNSNFK;
PSSPNIIATNSNFKI; SSPNIIATNSNFKIL; SPNIIATNSNFKILD;
PNIIATNSNFKILDA; NIIATNSNFKILDAY; IIATNSNFKILDAYY;
IATNSNFKILDAYYR; ATNSNFKILDAYYRR; TNSNFKILDAYYRRP;
NSNFKILDAYYRRPK; SNFKILDAYYRRPKI; NFKILDAYYRRPKIK;
FKILDAYYRRPKIKE; KILDAYYRRPKIKET; ILDAYYRRPKIKETT;
LDAYYRRPKIKETTG; DAYYRRPKIKETTGE; AYYRRPKIKETTGEI;
YYRRPKIKETTGEIF; YRRPKIKETTGEIFL; RRPKIKETTGEIFLK;
RPKIKETTGEIFLKA; PKIKETTGEIFLKAK; KIKETTGEIFLKAKE;
IKETTGEIFLKAKEI; KETTGEIFLKAKEIH; ETTGEIFLKAKEIHE;
TTGEIFLKAKEIHES; TGEIFLKAKEIHESN; GEIFLKAKEIHESNK;
EIFLKAKEIHESNKM; IFLKAKEIHESNKMS; FLKAKEIHESNKMSD;
LKAKEIHESNKMSDK; KAKEIHESNKMSDKK; AKEIHESNKMSDKKI;
KEIHESNKMSDKKIM; EIHESNKMSDKKIME; IHESNKMSDKKIMEL;
HESNKMSDKKIMELK; ESNKMSDKKIMELKE; SNKMSDKKIMELKEE;
NKMSDKKIMELKEEY; KMSDKKIMELKEEYN; MSDKKIMELKEEYNN;
SDKKIMELKEEYNNT; DKKIMELKEEYNNTS; KKIMELKEEYNNTSY;
KIMELKEEYNNTSYT; IMELKEEYNNTSYTS; MELKEEYNNTSYTSY;
ELKEEYNNTSYTSYS; LKEEYNNTSYTSYSE; KEEYNNTSYTSYSEF;
EEYNNTSYTSYSEFL; EYNNTSYTSYSEFLE; YNNTSYTSYSEFLEN;
NNTSYTSYSEFLENY; NTSYTSYSEFLENYY; TSYTSYSEFLENYYE;
SYTSYSEFLENYYES; YTSYSEFLENYYESL; TSYSEFLENYYESLN;
SYSEFLENYYESLND; YSEFLENYYESLNDQ; SEFLENYYESLNDQI;
EFLENYYESLNDQIK; FLENYYESLNDQIKK; LENYYESLNDQIKKT;
ENYYESLNDQIKKTN; NYYESLNDQIKKTNI; YYESLNDQIKKTNIK;

YESLNDQIKKTNIKE; ESLNDQIKKTNIKEL; SLNDQIKKTNIKELL;
LNDQIKKTNIKELLI; NDQIKKTNIKELLIE; DQIKKTNIKELLIEK;
QIKKTNIKELLIEKY; IKKTNIKELLIEKYK; KKTNIKELLIEKYKK;
KTNIKELLIEKYKKE; TNIKELLIEKYKKEL; NIKELLIEKYKKELI;
IKELLIEKYKKELIV; KELLIEKYKKELIVL; ELLIEKYKKELIVLE;
LLIEKYKKELIVLEK; LIEKYKKELIVLEKR; IEKYKKELIVLEKRI;
EKYKKELIVLEKRID; KYKKELIVLEKRIDS; YKKELIVLEKRIDSL;
KKELIVLEKRIDSLK; KELIVLEKRIDSLKQ; ELIVLEKRIDSLKQQ;
LIVLEKRIDSLKQQI; IVLEKRIDSLKQQIK; VLEKRIDSLKQQIKL;
LEKRIDSLKQQIKLL; EKRIDSLKQQIKLLE; KRIDSLKQQIKLLEN;
RIDSLKQQIKLLENI; IDSLKQQIKLLENIE; DSLKQQIKLLENIEN;
SLKQQIKLLENIENE; LKQQIKLLENIENEK; KQQIKLLENIENEKE;
QQIKLLENIENEKEK; QIKLLENIENEKEKG; IKLLENIENEKEKGE;
KLLENIENEKEKGEL; LLENIENEKEKGELI; LENIENEKEKGELIL;
ENIENEKEKGELILL; NIENEKEKGELILLN; IENEKEKGELILLNI;
ENEKEKGELILLNIN; NEKEKGELILLNINK; EKEKGELILLNINKI;
KEKGELILLNINKIQ; EKGELILLNINKIQK; KGELILLNINKIQKG;
GELILLNINKIQKGI; ELILLNINKIQKGIK; LILLNINKIQKGIKE;
ILLNINKIQKGIKEI; LLNINKIQKGIKEIN; LNINKIQKGIKEINL;
NINKIQKGIKEINLL; INKIQKGIKEINLLN; NKIQKGIKEINLLNY;
KIQKGIKEINLLNYK; IQKGIKEINLLNYKE; QKGIKEINLLNYKEE;
KGIKEINLLNYKEEK; GIKEINLLNYKEEKI; IKEINLLNYKEEKIK;
KEINLLNYKEEKIKI; EINLLNYKEEKIKIS; INLLNYKEEKIKISL;
NLLNYKEEKIKISLN; LLNYKEEKIKISLNQ; LNYKEEKIKISLNQS;
NYKEEKIKISLNQSL; YKEEKIKISLNQSLS; KEEKIKISLNQSLSP;
EEKIKISLNQSLSPK; EKIKISLNQSLSPKE; KIKISLNQSLSPKEN;
IKISLNQSLSPKENA; KISLNQSLSPKENAL; ISLNQSLSPKENALQ;
SLNQSLSPKENALQY; LNQSLSPKENALQYF; NQSLSPKENALQYFK;
QSLSPKENALQYFKA; SLSPKENALQYFKAY; LSPKENALQYFKAYK;
SPKENALQYFKAYKK; PKENALQYFKAYKKG; KENALQYFKAYKKGK;
ENALQYFKAYKKGKN; NALQYFKAYKKGKNS; ALQYFKAYKKGKNSF;
LQYFKAYKKGKNSFK; QYFKAYKKGKNSFKT; YFKAYKKGKNSFKTI;
FKAYKKGKNSFKTIQ; KAYKKGKNSFKTIQN; AYKKGKNSFKTIQNQ;
YKKGKNSFKTIQNQL; KKGKNSFKTIQNQLK; KGKNSFKTIQNQLKD;
GKNSFKTIQNQLKDN; KNSFKTIQNQLKDNL; NSFKTIQNQLKDNLD;
SFKTIQNQLKDNLDK; FKTIQNQLKDNLDKF; KTIQNQLKDNLDKFN;
TIQNQLKDNLDKFNL; IQNQLKDNLDKFNLI; QNQLKDNLDKFNLIQ;
NQLKDNLDKFNLIQS; QLKDNLDKFNLIQSK; LKDNLDKFNLIQSKI;
KDNLDKFNLIQSKIT; DNLDKFNLIQSKITM; NLDKFNLIQSKITML;
LDKFNLIQSKITMLK; DKFNLIQSKITMLKV; KFNLIQSKITMLKVE;
FNLIQSKITMLKVEN; NLIQSKITMLKVENL; LIQSKITMLKVENLI;
IQSKITMLKVENLIP; QSKITMLKVENLIPE; SKITMLKVENLIPEE;
KITMLKVENLIPEEE; ITMLKVENLIPEEEY; TMLKVENLIPEEEYN;
MLKVENLIPEEEYNQ; LKVENLIPEEEYNQE; KVENLIPEEEYNQEK;
VENLIPEEEYNQEKT; ENLIPEEEYNQEKTA; NLIPEEEYNQEKTAI;
LIPEEEYNQEKTAIK; IPEEEYNQEKTAIKE; PEEEYNQEKTAIKEK;
EEEYNQEKTAIKEKE; EEYNQEKTAIKEKEK; EYNQEKTAIKEKEKT;
YNQEKTAIKEKEKTP; NQEKTAIKEKEKTPK; QEKTAIKEKEKTPKI;
EKTAIKEKEKTPKIG; KTAIKEKEKTPKIGL; TAIKEKEKTPKIGLH;
AIKEKEKTPKIGLHF; IKEKEKTPKIGLHFT; KEKEKTPKIGLHFTY;
EKEKTPKIGLHFTYC; KEKTPKIGLHFTYCG; EKTPKIGLHFTYCGF;

KTPKIGLHFTYCGFE; TPKIGLHFTYCGFEI; PKIGLHFTYCGFEIL;
KIGLHFTYCGFEILI; IGLHFTYCGFEILIG; GLHFTYCGFEILIGR;
LHFTYCGFEILIGRN; HFTYCGFEILIGRNA; FTYCGFEILIGRNAK;
TYCGFEILIGRNAKE; YCGFEILIGRNAKEN; CGFEILIGRNAKEND;
GFEILIGRNAKENDK; FEILIGRNAKENDKL; EILIGRNAKENDKLL;
ILIGRNAKENDKLLR; LIGRNAKENDKLLRH; IGRNAKENDKLLRHC;
GRNAKENDKLLRHCV; RNAKENDKLLRHCVK; NAKENDKLLRHCVKG;
AKENDKLLRHCVKGN; KENDKLLRHCVKGND; ENDKLLRHCVKGNDY;
NDKLLRHCVKGNDYW; DKLLRHCVKGNDYWL; KLLRHCVKGNDYWLH;
LLRHCVKGNDYWLHT; LRHCVKGNDYWLHTR; RHCVKGNDYWLHTRD;
HCVKGNDYWLHTRDY; CVKGNDYWLHTRDYP; VKGNDYWLHTRDYPG;
KGNDYWLHTRDYPGA; GNDYWLHTRDYPGAY; NDYWLHTRDYPGAYV;
DYWLHTRDYPGAYVF; YWLHTRDYPGAYVFI; WLHTRDYPGAYVFIK;
LHTRDYPGAYVFIKN; HTRDYPGAYVFIKNQ; TRDYPGAYVFIKNQK;
RDYPGAYVFIKNQKN; DYPGAYVFIKNQKNK; YPGAYVFIKNQKNKT;
PGAYVFIKNQKNKTP; GAYVFIKNQKNKTPS; AYVFIKNQKNKTPSL;
YVFIKNQKNKTPSLD; VFIKNQKNKTPSLDV; FIKNQKNKTPSLDVL;
IKNQKNKTPSLDVLL; KNQKNKTPSLDVLLG; NQKNKTPSLDVLLGA;
QKNKTPSLDVLLGAG; KNKTPSLDVLLGAGN; NKTPSLDVLLGAGNL;
KTPSLDVLLGAGNLC; TPSLDVLLGAGNLCV; PSLDVLLGAGNLCVF;
SLDVLLGAGNLCVFY; LDVLLGAGNLCVFYT; DVLLGAGNLCVFYTK;
VLLGAGNLCVFYTKL; LLGAGNLCVFYTKLA; LGAGNLCVFYTKLAK;
GAGNLCVFYTKLAKK; AGNLCVFYTKLAKKS; GNLCVFYTKLAKKSG;
NLCVFYTKLAKKSGK; LCVFYTKLAKKSGKA; CVFYTKLAKKSGKAD;
VFYTKLAKKSGKADL; FYTKLAKKSGKADLY; YTKLAKKSGKADLYY;
TKLAKKSGKADLYYT; KLAKKSGKADLYYTQ; LAKKSGKADLYYTQV;
AKKSGKADLYYTQVK; KKSGKADLYYTQVKN; KSGKADLYYTQVKNL;
SGKADLYYTQVKNLR; GKADLYYTQVKNLRR; KADLYYTQVKNLRRV;
ADLYYTQVKNLRRVK; DLYYTQVKNLRRVKN; LYYTQVKNLRRVKNK;
YYTQVKNLRRVKNKK; YTQVKNLRRVKNKKL; TQVKNLRRVKNKKLG;
QVKNLRRVKNKKLGL; VKNLRRVKNKKLGLV; KNLRRVKNKKLGLVI;
NLRRVKNKKLGLVIP; LRRVKNKKLGLVIPK; RRVKNKKLGLVIPKA;
RVKNKKLGLVIPKAE; VKNKKLGLVIPKAEK; KNKKLGLVIPKAEKN;
NKKLGLVIPKAEKNL; KKLGLVIPKAEKNLH; KLGLVIPKAEKNLHI;
LGLVIPKAEKNLHIK; GLVIPKAEKNLHIKL; LVIPKAEKNLHIKLD;
VIPKAEKNLHIKLDE; IPKAEKNLHIKLDEN; PKAEKNLHIKLDENL;
KAEKNLHIKLDENLI; AEKNLHIKLDENLIK; EKNLHIKLDENLIKK;
KNLHIKLDENLIKKI; NLHIKLDENLIKKIK; LHIKLDENLIKKIKN;
HIKLDENLIKKIKNQ; IKLDENLIKKIKNQT;

16 mers:
MIKMSLNYTEINTLIK; IKMSLNYTEINTLIKE; KMSLNYTEINTLIKEI;
MSLNYTEINTLIKEIP; SLNYTEINTLIKEIPF; LNYTEINTLIKEIPFT;
NYTEINTLIKEIPFTN; YTEINTLIKEIPFTNS; TEINTLIKEIPFTNSL;
EINTLIKEIPFTNSLI; INTLIKEIPFTNSLIT; NTLIKEIPFTNSLITK;
TLIKEIPFTNSLITKI; LIKEIPFTNSLITKII; IKEIPFTNSLITKIIQ;
KEIPFTNSLITKIIQP; EIPFTNSLITKIIQPD; IPFTNSLITKIIQPDY;
PFTNSLITKIIQPDYK; FTNSLITKIIQPDYKS; TNSLITKIIQPDYKSL;
NSLITKIIQPDYKSLV; SLITKIIQPDYKSLVL; LITKIIQPDYKSLVLE;
ITKIIQPDYKSLVLEI; TKIIQPDYKSLVLEIY; KIIQPDYKSLVLEIYN;
IIQPDYKSLVLEIYNK; IQPDYKSLVLEIYNKI; QPDYKSLVLEIYNKID;

| | |
|---|---|
| PDYKSLVLEIYNKIDN; DYKSLVLEIYNKIDNK; YKSLVLEIYNKIDNKK; <br> KSLVLEIYNKIDNKKF; SLVLEIYNKIDNKKFK; LVLEIYNKIDNKKFKI; <br> VLEIYNKIDNKKFKIL; LEIYNKIDNKKFKILI; EIYNKIDNKKFKILIC; <br> IYNKIDNKKFKILICL; YNKIDNKKFKILICLN; NKIDNKKFKILICLNP; <br> KIDNKKFKILICLNPN; IDNKKFKILICLNPNT; DNKKFKILICLNPNTT; <br> NKKFKILICLNPNTTR; KKFKILICLNPNTTRF; KFKILICLNPNTTRFH; <br> FKILICLNPNTTRFHI; KILICLNPNTTRFHIT; ILICLNPNTTRFHITK; <br> LICLNPNTTRFHITKK; ICLNPNTTRFHITKKN; CLNPNTTRFHITKKNF; <br> LNPNTTRFHITKKNFK; NPNTTRFHITKKNFKK; PNTTRFHITKKNFKKN; <br> NTTRFHITKKNFKKNA; TTRFHITKKNFKKNAL; TRFHITKKNFKKNALK; <br> RFHITKKNFKKNALKL; FHITKKNFKKNALKLR; HITKKNFKKNALKLRF; <br> ITKKNFKKNALKLRFS; TKKNFKKNALKLRFSD; KKNFKKNALKLRFSDF; <br> KNFKKNALKLRFSDFL; NFKKNALKLRFSDFLK; FKKNALKLRFSDFLKS; <br> KKNALKLRFSDFLKSK; KNALKLRFSDFLKSKI; NALKLRFSDFLKSKIQ; <br> ALKLRFSDFLKSKIQN; LKLRFSDFLKSKIQNG; KLRFSDFLKSKIQNGK; <br> LRFSDFLKSKIQNGKI; RFSDFLKSKIQNGKII; FSDFLKSKIQNGKIIK; <br> SDFLKSKIQNGKIIKA; DFLKSKIQNGKIIKAF; FLKSKIQNGKIIKAFQ; <br> LKSKIQNGKIIKAFQM; KSKIQNGKIIKAFQMK; SKIQNGKIIKAFQMKN; <br> KIQNGKIIKAFQMKNE; IQNGKIIKAFQMKNER; QNGKIIKAFQMKNERI; <br> NGKIIKAFQMKNERII; GKIIKAFQMKNERIIS; KIIKAFQMKNERIISL; <br> IIKAFQMKNERIISLE; IKAFQMKNERIISLEI; KAFQMKNERIISLEIL; <br> AFQMKNERIISLEILQ; FQMKNERIISLEILQK; QMKNERIISLEILQKD; <br> MKNERIISLEILQKDM; KNERIISLEILQKDMI; NERIISLEILQKDMII; <br> ERIISLEILQKDMIIL; RIISLEILQKDMIILF; IISLEILQKDMIILFI; <br> ISLEILQKDMIILFIK; SLEILQKDMIILFIKL; LEILQKDMIILFIKLW; <br> EILQKDMIILFIKLWP; ILQKDMIILFIKLWPS; LQKDMIILFIKLWPSS; <br> QKDMIILFIKLWPSSP; KDMIILFIKLWPSSPN; DMIILFIKLWPSSPNI; <br> MIILFIKLWPSSPNII; IILFIKLWPSSPNIIA; ILFIKLWPSSPNIIAT; <br> LFIKLWPSSPNIIATN; FIKLWPSSPNIIATNS; IKLWPSSPNIIATNSN; <br> KLWPSSPNIIATNSNF; LWPSSPNIIATNSNFK; WPSSPNIIATNSNFKI; <br> PSSPNIIATNSNFKIL; SSPNIIATNSNFKILD; SPNIIATNSNFKILDA; <br> PNIIATNSNFKILDAY; NIIATNSNFKILDAYY; IIATNSNFKILDAYYR; <br> IATNSNFKILDAYYRR; ATNSNFKILDAYYRRP; TNSNFKILDAYYRRPK; <br> NSNFKILDAYYRRPKI; SNFKILDAYYRRPKIK; NFKILDAYYRRPKIKE; <br> FKILDAYYRRPKIKET; KILDAYYRRPKIKETT; ILDAYYRRPKIKETTG; <br> LDAYYRRPKIKETTGE; DAYYRRPKIKETTGEI; AYYRRPKIKETTGEIF; <br> YYRRPKIKETTGEIFL; YRRPKIKETTGEIFLK; RRPKIKETTGEIFLKA; <br> RPKIKETTGEIFLKAK; PKIKETTGEIFLKAKE; KIKETTGEIFLKAKEI; <br> IKETTGEIFLKAKEIH; KETTGEIFLKAKEIHE; ETTGEIFLKAKEIHES; <br> TTGEIFLKAKEIHESN; TGEIFLKAKEIHESNK; GEIFLKAKEIHESNKM; <br> EIFLKAKEIHESNKMS; IFLKAKEIHESNKMSD; FLKAKEIHESNKMSDK; <br> LKAKEIHESNKMSDKK; KAKEIHESNKMSDKKI; AKEIHESNKMSDKKIM; <br> KEIHESNKMSDKKIME; EIHESNKMSDKKIMEL; IHESNKMSDKKIMELK; <br> HESNKMSDKKIMELKE; ESNKMSDKKIMELKEE; SNKMSDKKIMELKEEY; <br> NKMSDKKIMELKEEYN; KMSDKKIMELKEEYNN; MSDKKIMELKEEYNNT; <br> SDKKIMELKEEYNNTS; DKKIMELKEEYNNTSY; KKIMELKEEYNNTSYT; <br> KIMELKEEYNNTSYTS; IMELKEEYNNTSYTSY; MELKEEYNNTSYTSYS; <br> ELKEEYNNTSYTSYSE; LKEEYNNTSYTSYSEF; KEEYNNTSYTSYSEFL; <br> EEYNNTSYTSYSEFLE; EYNNTSYTSYSEFLEN; YNNTSYTSYSEFLENY; <br> NNTSYTSYSEFLENYY; NTSYTSYSEFLENYYE; TSYTSYSEFLENYYES; <br> SYTSYSEFLENYYESL; YTSYSEFLENYYESLN; TSYSEFLENYYESLND; |

| | SYSEFLENYYESLNDQ; YSEFLENYYESLNDQI; SEFLENYYESLNDQIK;<br>EFLENYYESLNDQIKK; FLENYYESLNDQIKKT; LENYYESLNDQIKKTN;<br>ENYYESLNDQIKKTNI; NYYESLNDQIKKTNIK; YYESLNDQIKKTNIKE;<br>YESLNDQIKKTNIKEL; ESLNDQIKKTNIKELL; SLNDQIKKTNIKELLI;<br>LNDQIKKTNIKELLIE; NDQIKKTNIKELLIEK; DQIKKTNIKELLIEKY;<br>QIKKTNIKELLIEKYK; IKKTNIKELLIEKYKK; KKTNIKELLIEKYKKE;<br>KTNIKELLIEKYKKEL; TNIKELLIEKYKKELI; NIKELLIEKYKKELIV;<br>IKELLIEKYKKELIVL; KELLIEKYKKELIVLE; ELLIEKYKKELIVLEK;<br>LLIEKYKKELIVLEKR; LIEKYKKELIVLEKRI; IEKYKKELIVLEKRID;<br>EKYKKELIVLEKRIDS; KYKKELIVLEKRIDSL; YKKELIVLEKRIDSLK;<br>KKELIVLEKRIDSLKQ; KELIVLEKRIDSLKQQ; ELIVLEKRIDSLKQQI;<br>LIVLEKRIDSLKQQIK; IVLEKRIDSLKQQIKL; VLEKRIDSLKQQIKLL;<br>LEKRIDSLKQQIKLLE; EKRIDSLKQQIKLLEN; KRIDSLKQQIKLLENI;<br>RIDSLKQQIKLLENIE; IDSLKQQIKLLENIEN; DSLKQQIKLLENIENE;<br>SLKQQIKLLENIENEK; LKQQIKLLENIENEKE; KQQIKLLENIENEKEK;<br>QQIKLLENIENEKEKG; QIKLLENIENEKEKGE; IKLLENIENEKEKGEL;<br>KLLENIENEKEKGELI; LLENIENEKEKGELIL; LENIENEKEKGELILL;<br>ENIENEKEKGELILLN; NIENEKEKGELILLNI; IENEKEKGELILLNIN;<br>ENEKEKGELILLNINK; NEKEKGELILLNINKI; EKEKGELILLNINKIQ;<br>KEKGELILLNINKIQK; EKGELILLNINKIQKG; KGELILLNINKIQKGI;<br>GELILLNINKIQKGIK; ELILLNINKIQKGIKE; LILLNINKIQKGIKEI;<br>ILLNINKIQKGIKEIN; LLNINKIQKGIKEINL; LNINKIQKGIKEINLL;<br>NINKIQKGIKEINLLN; INKIQKGIKEINLLNY; NKIQKGIKEINLLNYK;<br>KIQKGIKEINLLNYKE; IQKGIKEINLLNYKEE; QKGIKEINLLNYKEEK;<br>KGIKEINLLNYKEEKI; GIKEINLLNYKEEKIK; IKEINLLNYKEEKIKI;<br>KEINLLNYKEEKIKIS; EINLLNYKEEKIKISL; INLLNYKEEKIKISLN;<br>NLLNYKEEKIKISLNQ; LLNYKEEKIKISLNQS; LNYKEEKIKISLNQSL;<br>NYKEEKIKISLNQSLS; YKEEKIKISLNQSLSP; KEEKIKISLNQSLSPK;<br>EEKIKISLNQSLSPKE; EKIKISLNQSLSPKEN; KIKISLNQSLSPKENA;<br>IKISLNQSLSPKENAL; KISLNQSLSPKENALQ; ISLNQSLSPKENALQY;<br>SLNQSLSPKENALQYF; LNQSLSPKENALQYFK; NQSLSPKENALQYFKA;<br>QSLSPKENALQYFKAY; SLSPKENALQYFKAYK; LSPKENALQYFKAYKK;<br>SPKENALQYFKAYKKG; PKENALQYFKAYKKGK; KENALQYFKAYKKGKN;<br>ENALQYFKAYKKGKNS; NALQYFKAYKKGKNSF; ALQYFKAYKKGKNSFK;<br>LQYFKAYKKGKNSFKT; QYFKAYKKGKNSFKTI; YFKAYKKGKNSFKTIQ;<br>FKAYKKGKNSFKTIQN; KAYKKGKNSFKTIQNQ; AYKKGKNSFKTIQNQL;<br>YKKGKNSFKTIQNQLK; KKGKNSFKTIQNQLKD; KGKNSFKTIQNQLKDN;<br>GKNSFKTIQNQLKDNL; KNSFKTIQNQLKDNLD; NSFKTIQNQLKDNLDK;<br>SFKTIQNQLKDNLDKF; FKTIQNQLKDNLDKFN; KTIQNQLKDNLDKFNL;<br>TIQNQLKDNLDKFNLI; IQNQLKDNLDKFNLIQ; QNQLKDNLDKFNLIQS;<br>NQLKDNLDKFNLIQSK; QLKDNLDKFNLIQSKI; LKDNLDKFNLIQSKIT;<br>KDNLDKFNLIQSKITM; DNLDKFNLIQSKITML; NLDKFNLIQSKITMLK;<br>LDKFNLIQSKITMLKV; DKFNLIQSKITMLKVE; KFNLIQSKITMLKVEN;<br>FNLIQSKITMLKVENL; NLIQSKITMLKVENLI; LIQSKITMLKVENLIP;<br>IQSKITMLKVENLIPE; QSKITMLKVENLIPEE; SKITMLKVENLIPEE;<br>KITMLKVENLIPEEEY; ITMLKVENLIPEEEYN; TMLKVENLIPEEEYNQ;<br>MLKVENLIPEEEYNQE; LKVENLIPEEEYNQEK; KVENLIPEEEYNQEKT;<br>VENLIPEEEYNQEKTA; ENLIPEEEYNQEKTAI; NLIPEEEYNQEKTAIK;<br>LIPEEEYNQEKTAIKE; IPEEEYNQEKTAIKEK; PEEEYNQEKTAIKEKE;<br>EEEYNQEKTAIKEKEK; EEYNQEKTAIKEKEKT; EYNQEKTAIKEKEKTP;<br>YNQEKTAIKEKEKTPK; NQEKTAIKEKEKTPKI; QEKTAIKEKEKTPKIG; |

| | |
|---|---|
| | EKTAIKEKEKTPKIGL; KTAIKEKEKTPKIGLH; TAIKEKEKTPKIGLHF; AIKEKEKTPKIGLHFT; IKEKEKTPKIGLHFTY; KEKEKTPKIGLHFTYC; EKEKTPKIGLHFTYCG; KEKTPKIGLHFTYCGF; EKTPKIGLHFTYCGFE; KTPKIGLHFTYCGFEI; TPKIGLHFTYCGFEIL; PKIGLHFTYCGFEILI; KIGLHFTYCGFEILIG; IGLHFTYCGFEILIGR; GLHFTYCGFEILIGRN; LHFTYCGFEILIGRNA; HFTYCGFEILIGRNAK; FTYCGFEILIGRNAKE; TYCGFEILIGRNAKEN; YCGFEILIGRNAKEND; CGFEILIGRNAKENDK; GFEILIGRNAKENDKL; FEILIGRNAKENDKLL; EILIGRNAKENDKLLR; ILIGRNAKENDKLLRH; LIGRNAKENDKLLRHC; IGRNAKENDKLLRHCV; GRNAKENDKLLRHCVK; RNAKENDKLLRHCVKG; NAKENDKLLRHCVKGN; AKENDKLLRHCVKGND; KENDKLLRHCVKGNDY; ENDKLLRHCVKGNDYW; NDKLLRHCVKGNDYWL; DKLLRHCVKGNDYWLH; KLLRHCVKGNDYWLHT; LLRHCVKGNDYWLHTR; LRHCVKGNDYWLHTRD; RHCVKGNDYWLHTRDY; HCVKGNDYWLHTRDYP; CVKGNDYWLHTRDYPG; VKGNDYWLHTRDYPGA; KGNDYWLHTRDYPGAY; GNDYWLHTRDYPGAYV; NDYWLHTRDYPGAYVF; DYWLHTRDYPGAYVFI; YWLHTRDYPGAYVFIK; WLHTRDYPGAYVFIKN; LHTRDYPGAYVFIKNQ; HTRDYPGAYVFIKNQK; TRDYPGAYVFIKNQKN; RDYPGAYVFIKNQKNK; DYPGAYVFIKNQKNKT; YPGAYVFIKNQKNKTP; PGAYVFIKNQKNKTPS; GAYVFIKNQKNKTPSL; AYVFIKNQKNKTPSLD; YVFIKNQKNKTPSLDV; VFIKNQKNKTPSLDVL; FIKNQKNKTPSLDVLL; IKNQKNKTPSLDVLLG; KNQKNKTPSLDVLLGA; NQKNKTPSLDVLLGAG; QKNKTPSLDVLLGAGN; KNKTPSLDVLLGAGNL; NKTPSLDVLLGAGNLC; KTPSLDVLLGAGNLCV; TPSLDVLLGAGNLCVF; PSLDVLLGAGNLCVFY; SLDVLLGAGNLCVFYT; LDVLLGAGNLCVFYTK; DVLLGAGNLCVFYTKL; VLLGAGNLCVFYTKLA; LLGAGNLCVFYTKLAK; LGAGNLCVFYTKLAKK; GAGNLCVFYTKLAKKS; AGNLCVFYTKLAKKSG; GNLCVFYTKLAKKSGK; NLCVFYTKLAKKSGKA; LCVFYTKLAKKSGKAD; CVFYTKLAKKSGKADL; VFYTKLAKKSGKADLY; FYTKLAKKSGKADLYY; YTKLAKKSGKADLYYT; TKLAKKSGKADLYYTQ; KLAKKSGKADLYYTQV; LAKKSGKADLYYTQVK; AKKSGKADLYYTQVKN; KKSGKADLYYTQVKNL; KSGKADLYYTQVKNLR; SGKADLYYTQVKNLRR; GKADLYYTQVKNLRRV; KADLYYTQVKNLRRVK; ADLYYTQVKNLRRVKN; DLYYTQVKNLRRVKNK; LYYTQVKNLRRVKNKK; YYTQVKNLRRVKNKKL; YTQVKNLRRVKNKKLG; TQVKNLRRVKNKKLGL; QVKNLRRVKNKKLGLV; VKNLRRVKNKKLGLVI; KNLRRVKNKKLGLVIP; NLRRVKNKKLGLVIPK; LRRVKNKKLGLVIPKA; RRVKNKKLGLVIPKAE; RVKNKKLGLVIPKAEK; VKNKKLGLVIPKAEKN; KNKKLGLVIPKAEKNL; NKKLGLVIPKAEKNLH; KKLGLVIPKAEKNLHI; KLGLVIPKAEKNLHIK; LGLVIPKAEKNLHIKL; GLVIPKAEKNLHIKLD; LVIPKAEKNLHIKLDE; VIPKAEKNLHIKLDEN; IPKAEKNLHIKLDENL; PKAEKNLHIKLDENLI; KAEKNLHIKLDENLIK; AEKNLHIKLDENLIKK; EKNLHIKLDENLIKKI; KNLHIKLDENLIKKIK; NLHIKLDENLIKKIKN; LHIKLDENLIKKIKNQ; HIKLDENLIKKIKNQT; |
| Seq 21 | 13 mers: MKLQRSLSLIIFS; KLQRSLSLIIFSL; LQRSLSLIIFSLT; QRSLSLIIFSLTV; RSLSLIIFSLTVL; SLSLIIFSLTVLC; LSLIIFSLTVLCC; SLIIFSLTVLCCD; LIIFSLTVLCCDN; IIFSLTVLCCDNK; IFSLTVLCCDNKE; FSLTVLCCDNKER; SLTVLCCDNKERK; LTVLCCDNKERKE; TVLCCDNKERKEG; VLCCDNKERKEGV; LCCDNKERKEGVS; CCDNKERKEGVST; |

| | |
|---|---|
| CDNKERKEGVSTK; DNKERKEGVSTKI; NKERKEGVSTKIS; | |
| KERKEGVSTKISL; ERKEGVSTKISLG; RKEGVSTKISLGA; | |
| KEGVSTKISLGAE; EGVSTKISLGAEP; GVSTKISLGAEPR; | |
| VSTKISLGAEPRS; STKISLGAEPRSL; TKISLGAEPRSLD; | |
| KISLGAEPRSLDP; ISLGAEPRSLDPQ; SLGAEPRSLDPQL; | |
| LGAEPRSLDPQLA; GAEPRSLDPQLAE; AEPRSLDPQLAED; | |
| EPRSLDPQLAEDN; PRSLDPQLAEDNV; RSLDPQLAEDNVA; | |
| SLDPQLAEDNVAS; LDPQLAEDNVASK; DPQLAEDNVASKM; | |
| PQLAEDNVASKMI; QLAEDNVASKMID; LAEDNVASKMIDT; | |
| AEDNVASKMIDTL; EDNVASKMIDTLY; DNVASKMIDTLYR; | |
| NVASKMIDTLYRG; VASKMIDTLYRGI; ASKMIDTLYRGIV; | |
| SKMIDTLYRGIVT; KMIDTLYRGIVTG; MIDTLYRGIVTGD; | |
| IDTLYRGIVTGDP; DTLYRGIVTGDPN; TLYRGIVTGDPNT; | |
| LYRGIVTGDPNTG; YRGIVTGDPNTGG; RGIVTGDPNTGGH; | |
| GIVTGDPNTGGHK; IVTGDPNTGGHKP; VTGDPNTGGHKPG; | |
| TGDPNTGGHKPGL; GDPNTGGHKPGLA; DPNTGGHKPGLAK; | |
| PNTGGHKPGLAKG; NTGGHKPGLAKGW; TGGHKPGLAKGWE; | |
| GGHKPGLAKGWET; GHKPGLAKGWETS; HKPGLAKGWETSS; | |
| KPGLAKGWETSSD; PGLAKGWETSSDG; GLAKGWETSSDGT; | |
| LAKGWETSSDGTA; AKGWETSSDGTAY; KGWETSSDGTAYP; | |
| GWETSSDGTAYPF; WETSSDGTAYPFY; ETSSDGTAYPFYL; | |
| TSSDGTAYPFYLR; SSDGTAYPFYLRD; SDGTAYPFYLRDN; | |
| DGTAYPFYLRDNL; GTAYPFYLRDNLT; TAYPFYLRDNLTW; | |
| AYPFYLRDNLTWS; YPFYLRDNLTWSD; PFYLRDNLTWSDG; | |
| FYLRDNLTWSDGV; YLRDNLTWSDGVA; LRDNLTWSDGVAI; | |
| RDNLTWSDGVAIT; DNLTWSDGVAITA; NLTWSDGVAITAE; | |
| LTWSDGVAITAEG; TWSDGVAITAEGI; WSDGVAITAEGIR; | |
| SDGVAITAEGIRK; DGVAITAEGIRKS; GVAITAEGIRKSY; | |
| VAITAEGIRKSYL; AITAEGIRKSYLR; ITAEGIRKSYLRI; | |
| TAEGIRKSYLRIL; AEGIRKSYLRILN; EGIRKSYLRILNK; | |
| GIRKSYLRILNKE; IRKSYLRILNKET; RKSYLRILNKETG; | |
| KSYLRILNKETGS; SYLRILNKETGST; YLRILNKETGSTY; | |
| LRILNKETGSTYV; RILNKETGSTYVD; ILNKETGSTYVDM; | |
| LNKETGSTYVDMV; NKETGSTYVDMVK; KETGSTYVDMVKS; | |
| ETGSTYVDMVKSI; TGSTYVDMVKSII; GSTYVDMVKSIIK; | |
| STYVDMVKSIIKN; TYVDMVKSIIKNG; YVDMVKSIIKNGQ; | |
| VDMVKSIIKNGQE; DMVKSIIKNGQEY; MVKSIIKNGQEYF; | |
| VKSIIKNGQEYFD; KSIIKNGQEYFDG; SIIKNGQEYFDGQ; | |
| IIKNGQEYFDGQV; IKNGQEYFDGQVT; KNGQEYFDGQVTD; | |
| NGQEYFDGQVTDS; GQEYFDGQVTDSE; QEYFDGQVTDSEL; | |
| EYFDGQVTDSELG; YFDGQVTDSELGI; FDGQVTDSELGIR; | |
| DGQVTDSELGIRA; GQVTDSELGIRAI; QVTDSELGIRAID; | |
| VTDSELGIRAIDS; TDSELGIRAIDSK; DSELGIRAIDSKT; | |
| SELGIRAIDSKTL; ELGIRAIDSKTLE; LGIRAIDSKTLEI; | |
| GIRAIDSKTLEIT; IRAIDSKTLEITL; RAIDSKTLEITLA; | |
| AIDSKTLEITLAS; IDSKTLEITLASP; DSKTLEITLASPK; | |
| SKTLEITLASPKP; KTLEITLASPKPY; TLEITLASPKPYF; | |
| LEITLASPKPYFI; EITLASPKPYFID; ITLASPKPYFIDL; | |
| TLASPKPYFIDLL; LASPKPYFIDLLV; ASPKPYFIDLLVH; | |
| SPKPYFIDLLVHQ; PKPYFIDLLVHQS; KPYFIDLLVHQSF; | |
| PYFIDLLVHQSFI; YFIDLLVHQSFIP; FIDLLVHQSFIPV; | |

IDLLVHQSFIPVP; DLLVHQSFIPVPV; LLVHQSFIPVPVH;
LVHQSFIPVPVHV; VHQSFIPVPVHVT; HQSFIPVPVHVTD;
QSFIPVPVHVTDK; SFIPVPVHVTDKY; FIPVPVHVTDKYG;
IPVPVHVTDKYGQ; PVPVHVTDKYGQN; VPVHVTDKYGQNW;
PVHVTDKYGQNWT; VHVTDKYGQNWTS; HVTDKYGQNWTSP;
VTDKYGQNWTSPE; TDKYGQNWTSPEN; DKYGQNWTSPENM;
KYGQNWTSPENMV; YGQNWTSPENMVT; GQNWTSPENMVTS;
QNWTSPENMVTSG; NWTSPENMVTSGP; WTSPENMVTSGPF;
TSPENMVTSGPFK; SPENMVTSGPFKL; PENMVTSGPFKLK;
ENMVTSGPFKLKE; NMVTSGPFKLKER; MVTSGPFKLKERI;
VTSGPFKLKERIP; TSGPFKLKERIPS; SGPFKLKERIPSE;
GPFKLKERIPSEK; PFKLKERIPSEKY; FKLKERIPSEKYV;
KLKERIPSEKYVF; LKERIPSEKYVFE; KERIPSEKYVFEK;
ERIPSEKYVFEKD; RIPSEKYVFEKDN; IPSEKYVFEKDNK;
PSEKYVFEKDNKY; SEKYVFEKDNKYY; EKYVFEKDNKYYD;
KYVFEKDNKYYDS; YVFEKDNKYYDSN; VFEKDNKYYDSNE;
FEKDNKYYDSNEV; EKDNKYYDSNEVE; KDNKYYDSNEVEL;
DNKYYDSNEVELE; NKYYDSNEVELEE; KYYDSNEVELEEI;
YYDSNEVELEEIT; YDSNEVELEEITF; DSNEVELEEITFY;
SNEVELEEITFYT; NEVELEEITFYTT; EVELEEITFYTTN;
VELEEITFYTTND; ELEEITFYTTNDS; LEEITFYTTNDSS;
EEITFYTTNDSST; EITFYTTNDSSTA; ITFYTTNDSSTAY;
TFYTTNDSSTAYK; FYTTNDSSTAYKM; YTTNDSSTAYKMY;
TTNDSSTAYKMYV; TNDSSTAYKMYVN; NDSSTAYKMYVNE;
DSSTAYKMYVNEE; SSTAYKMYVNEEL; STAYKMYVNEELD;
TAYKMYVNEELDA; AYKMYVNEELDAI; YKMYVNEELDAIL;
KMYVNEELDAILV; MYVNEELDAILVP; YVNEELDAILVPY;
VNEELDAILVPYP; NEELDAILVPYPQ; EELDAILVPYPQI;

14 mers:
MKLQRSLSLIIFSL; KLQRSLSLIIFSLT; LQRSLSLIIFSLTV;
QRSLSLIIFSLTVL; RSLSLIIFSLTVLC; SLSLIIFSLTVLCC;
LSLIIFSLTVLCCD; SLIIFSLTVLCCDN; LIIFSLTVLCCDNK;
IIFSLTVLCCDNKE; IFSLTVLCCDNKER; FSLTVLCCDNKERK;
SLTVLCCDNKERKE; LTVLCCDNKERKEG; TVLCCDNKERKEGV;
VLCCDNKERKEGVS; LCCDNKERKEGVST; CCDNKERKEGVSTK;
CDNKERKEGVSTKI; DNKERKEGVSTKIS; NKERKEGVSTKISL;
KERKEGVSTKISLG; ERKEGVSTKISLGA; RKEGVSTKISLGAE;
KEGVSTKISLGAEP; EGVSTKISLGAEPR; GVSTKISLGAEPRS;
VSTKISLGAEPRSL; STKISLGAEPRSLD; TKISLGAEPRSLDP;
KISLGAEPRSLDPQ; ISLGAEPRSLDPQL; SLGAEPRSLDPQLA;
LGAEPRSLDPQLAE; GAEPRSLDPQLAED; AEPRSLDPQLAEDN;
EPRSLDPQLAEDNV; PRSLDPQLAEDNVA; RSLDPQLAEDNVAS;
SLDPQLAEDNVASK; LDPQLAEDNVASKM; DPQLAEDNVASKMI;
PQLAEDNVASKMID; QLAEDNVASKMIDT; LAEDNVASKMIDTL;
AEDNVASKMIDTLY; EDNVASKMIDTLYR; DNVASKMIDTLYRG;
NVASKMIDTLYRGI; VASKMIDTLYRGIV; ASKMIDTLYRGIVT;
SKMIDTLYRGIVTG; KMIDTLYRGIVTGD; MIDTLYRGIVTGDP;
IDTLYRGIVTGDPN; DTLYRGIVTGDPNT; TLYRGIVTGDPNTG;
LYRGIVTGDPNTGG; YRGIVTGDPNTGGH; RGIVTGDPNTGGHK;
GIVTGDPNTGGHKP; IVTGDPNTGGHKPG; VTGDPNTGGHKPGL;

| | | |
|---|---|---|
| TGDPNTGGHKPGLA; | GDPNTGGHKPGLAK; | DPNTGGHKPGLAKG; |
| PNTGGHKPGLAKGW; | NTGGHKPGLAKGWE; | TGGHKPGLAKGWET; |
| GGHKPGLAKGWETS; | GHKPGLAKGWETSS; | HKPGLAKGWETSSD; |
| KPGLAKGWETSSDG; | PGLAKGWETSSDGT; | GLAKGWETSSDGTA; |
| LAKGWETSSDGTAY; | AKGWETSSDGTAYP; | KGWETSSDGTAYPF; |
| GWETSSDGTAYPFY; | WETSSDGTAYPFYL; | ETSSDGTAYPFYLR; |
| TSSDGTAYPFYLRD; | SSDGTAYPFYLRDN; | SDGTAYPFYLRDNL; |
| DGTAYPFYLRDNLT; | GTAYPFYLRDNLTW; | TAYPFYLRDNLTWS; |
| AYPFYLRDNLTWSD; | YPFYLRDNLTWSDG; | PFYLRDNLTWSDGV; |
| FYLRDNLTWSDGVA; | YLRDNLTWSDGVAI; | LRDNLTWSDGVAIT; |
| RDNLTWSDGVAITA; | DNLTWSDGVAITAE; | NLTWSDGVAITAEG; |
| LTWSDGVAITAEGI; | TWSDGVAITAEGIR; | WSDGVAITAEGIRK; |
| SDGVAITAEGIRKS; | DGVAITAEGIRKSY; | GVAITAEGIRKSYL; |
| VAITAEGIRKSYLR; | AITAEGIRKSYLRI; | ITAEGIRKSYLRIL; |
| TAEGIRKSYLRILN; | AEGIRKSYLRILNK; | EGIRKSYLRILNKE; |
| GIRKSYLRILNKET; | IRKSYLRILNKETG; | RKSYLRILNKETGS; |
| KSYLRILNKETGST; | SYLRILNKETGSTY; | YLRILNKETGSTYV; |
| LRILNKETGSTYVD; | RILNKETGSTYVDM; | ILNKETGSTYVDMV; |
| LNKETGSTYVDMVK; | NKETGSTYVDMVKS; | KETGSTYVDMVKSI; |
| ETGSTYVDMVKSII; | TGSTYVDMVKSIIK; | GSTYVDMVKSIIKN; |
| STYVDMVKSIIKNG; | TYVDMVKSIIKNGQ; | YVDMVKSIIKNGQE; |
| VDMVKSIIKNGQEY; | DMVKSIIKNGQEYF; | MVKSIIKNGQEYFD; |
| VKSIIKNGQEYFDG; | KSIIKNGQEYFDGQ; | SIIKNGQEYFDGQV; |
| IIKNGQEYFDGQVT; | IKNGQEYFDGQVTD; | KNGQEYFDGQVTDS; |
| NGQEYFDGQVTDSE; | GQEYFDGQVTDSEL; | QEYFDGQVTDSELG; |
| EYFDGQVTDSELGI; | YFDGQVTDSELGIR; | FDGQVTDSELGIRA; |
| DGQVTDSELGIRAI; | GQVTDSELGIRAID; | QVTDSELGIRAIDS; |
| VTDSELGIRAIDSK; | TDSELGIRAIDSKT; | DSELGIRAIDSKTL; |
| SELGIRAIDSKTLE; | ELGIRAIDSKTLEI; | LGIRAIDSKTLEIT; |
| GIRAIDSKTLEITL; | IRAIDSKTLEITLA; | RAIDSKTLEITLAS; |
| AIDSKTLEITLASP; | IDSKTLEITLASPK; | DSKTLEITLASPKP; |
| SKTLEITLASPKPY; | KTLEITLASPKPYF; | TLEITLASPKPYFI; |
| LEITLASPKPYFID; | EITLASPKPYFIDL; | ITLASPKPYFIDLL; |
| TLASPKPYFIDLLV; | LASPKPYFIDLLVH; | ASPKPYFIDLLVHQ; |
| SPKPYFIDLLVHQS; | PKPYFIDLLVHQSF; | KPYFIDLLVHQSFI; |
| PYFIDLLVHQSFIP; | YFIDLLVHQSFIPV; | FIDLLVHQSFIPVP; |
| IDLLVHQSFIPVPV; | DLLVHQSFIPVPVH; | LLVHQSFIPVPVHV; |
| LVHQSFIPVPVHVT; | VHQSFIPVPVHVTD; | HQSFIPVPVHVTDK; |
| QSFIPVPVHVTDKY; | SFIPVPVHVTDKYG; | FIPVPVHVTDKYGQ; |
| IPVPVHVTDKYGQN; | PVPVHVTDKYGQNW; | VPVHVTDKYGQNWT; |
| PVHVTDKYGQNWTS; | VHVTDKYGQNWTSP; | HVTDKYGQNWTSPE; |
| VTDKYGQNWTSPEN; | TDKYGQNWTSPENM; | DKYGQNWTSPENMV; |
| KYGQNWTSPENMVT; | YGQNWTSPENMVTS; | GQNWTSPENMVTSG; |
| QNWTSPENMVTSGP; | NWTSPENMVTSGPF; | WTSPENMVTSGPFK; |
| TSPENMVTSGPFKL; | SPENMVTSGPFKLK; | PENMVTSGPFKLKE; |
| ENMVTSGPFKLKER; | NMVTSGPFKLKERI; | MVTSGPFKLKERIP; |
| VTSGPFKLKERIPS; | TSGPFKLKERIPSE; | SGPFKLKERIPSEK; |
| GPFKLKERIPSEKY; | PFKLKERIPSEKYV; | FKLKERIPSEKYVF; |
| KLKERIPSEKYVFE; | LKERIPSEKYVFEK; | KERIPSEKYVFEKD; |
| ERIPSEKYVFEKDN; | RIPSEKYVFEKDNK; | IPSEKYVFEKDNKY; |
| PSEKYVFEKDNKYY; | SEKYVFEKDNKYYD; | EKYVFEKDNKYYDS; |

KYVFEKDNKYYDSN; YVFEKDNKYYDSNE; VFEKDNKYYDSNEV;
FEKDNKYYDSNEVE; EKDNKYYDSNEVEL; KDNKYYDSNEVELE;
DNKYYDSNEVELEE; NKYYDSNEVELEEI; KYYDSNEVELEEIT;
YYDSNEVELEEITF; YDSNEVELEEITFY; DSNEVELEEITFYT;
SNEVELEEITFYTT; NEVELEEITFYTTN; EVELEEITFYTTND;
VELEEITFYTTNDS; ELEEITFYTTNDSS; LEEITFYTTNDSST;
EEITFYTTNDSSTA; EITFYTTNDSSTAY; ITFYTTNDSSTAYK;
TFYTTNDSSTAYKM; FYTTNDSSTAYKMY; YTTNDSSTAYKMYV;
TTNDSSTAYKMYVN; TNDSSTAYKMYVNE; NDSSTAYKMYVNEE;
DSSTAYKMYVNEEL; SSTAYKMYVNEELD; STAYKMYVNEELDA;
TAYKMYVNEELDAI; AYKMYVNEELDAIL; YKMYVNEELDAILV;
KMYVNEELDAILVP; MYVNEELDAILVPY; YVNEELDAILVPYP;
VNEELDAILVPYPQ; NEELDAILVPYPQI;

15 mers:
MKLQRSLSLIIFSLT; KLQRSLSLIIFSLTV; LQRSLSLIIFSLTVL;
QRSLSLIIFSLTVLC; RSLSLIIFSLTVLCC; SLSLIIFSLTVLCCD;
LSLIIFSLTVLCCDN; SLIIFSLTVLCCDNK; LIIFSLTVLCCDNKE;
IIFSLTVLCCDNKER; IFSLTVLCCDNKERK; FSLTVLCCDNKERKE;
SLTVLCCDNKERKEG; LTVLCCDNKERKEGV; TVLCCDNKERKEGVS;
VLCCDNKERKEGVST; LCCDNKERKEGVSTK; CCDNKERKEGVSTKI;
CDNKERKEGVSTKIS; DNKERKEGVSTKISL; NKERKEGVSTKISLG;
KERKEGVSTKISLGA; ERKEGVSTKISLGAE; RKEGVSTKISLGAEP;
KEGVSTKISLGAEPR; EGVSTKISLGAEPRS; GVSTKISLGAEPRSL;
VSTKISLGAEPRSLD; STKISLGAEPRSLDP; TKISLGAEPRSLDPQ;
KISLGAEPRSLDPQL; ISLGAEPRSLDPQLA; SLGAEPRSLDPQLAE;
LGAEPRSLDPQLAED; GAEPRSLDPQLAEDN; AEPRSLDPQLAEDNV;
EPRSLDPQLAEDNVA; PRSLDPQLAEDNVAS; RSLDPQLAEDNVASK;
SLDPQLAEDNVASKM; LDPQLAEDNVASKMI; DPQLAEDNVASKMID;
PQLAEDNVASKMIDT; QLAEDNVASKMIDTL; LAEDNVASKMIDTLY;
AEDNVASKMIDTLYR; EDNVASKMIDTLYRG; DNVASKMIDTLYRGI;
NVASKMIDTLYRGIV; VASKMIDTLYRGIVT; ASKMIDTLYRGIVTG;
SKMIDTLYRGIVTGD; KMIDTLYRGIVTGDP; MIDTLYRGIVTGDPN;
IDTLYRGIVTGDPNT; DTLYRGIVTGDPNTG; TLYRGIVTGDPNTGG;
LYRGIVTGDPNTGGH; YRGIVTGDPNTGGHK; RGIVTGDPNTGGHKP;
GIVTGDPNTGGHKPG; IVTGDPNTGGHKPGL; VTGDPNTGGHKPGLA;
TGDPNTGGHKPGLAK; GDPNTGGHKPGLAKG; DPNTGGHKPGLAKGW;
PNTGGHKPGLAKGWE; NTGGHKPGLAKGWET; TGGHKPGLAKGWETS;
GGHKPGLAKGWETSS; GHKPGLAKGWETSSD; HKPGLAKGWETSSDG;
KPGLAKGWETSSDGT; PGLAKGWETSSDGTA; GLAKGWETSSDGTAY;
LAKGWETSSDGTAYP; AKGWETSSDGTAYPF; KGWETSSDGTAYPFY;
GWETSSDGTAYPFYL; WETSSDGTAYPFYLR; ETSSDGTAYPFYLRD;
TSSDGTAYPFYLRDN; SSDGTAYPFYLRDNL; SDGTAYPFYLRDNLT;
DGTAYPFYLRDNLTW; GTAYPFYLRDNLTWS; TAYPFYLRDNLTWSD;
AYPFYLRDNLTWSDG; YPFYLRDNLTWSDGV; PFYLRDNLTWSDGVA;
FYLRDNLTWSDGVAI; YLRDNLTWSDGVAIT; LRDNLTWSDGVAITA;
RDNLTWSDGVAITAE; DNLTWSDGVAITAEG; NLTWSDGVAITAEGI;
LTWSDGVAITAEGIR; TWSDGVAITAEGIRK; WSDGVAITAEGIRKS;
SDGVAITAEGIRKSY; DGVAITAEGIRKSYL; GVAITAEGIRKSYLR;
VAITAEGIRKSYLRI; AITAEGIRKSYLRIL; ITAEGIRKSYLRILN;
TAEGIRKSYLRILNK; AEGIRKSYLRILNKE; EGIRKSYLRILNKET;

GIRKSYLRILNKETG; IRKSYLRILNKETGS; RKSYLRILNKETGST;
KSYLRILNKETGSTY; SYLRILNKETGSTYV; YLRILNKETGSTYVD;
LRILNKETGSTYVDM; RILNKETGSTYVDMV; ILNKETGSTYVDMVK;
LNKETGSTYVDMVKS; NKETGSTYVDMVKSI; KETGSTYVDMVKSII;
ETGSTYVDMVKSIIK; TGSTYVDMVKSIIKN; GSTYVDMVKSIIKNG;
STYVDMVKSIIKNGQ; TYVDMVKSIIKNGQE; YVDMVKSIIKNGQEY;
VDMVKSIIKNGQEYF; DMVKSIIKNGQEYFD; MVKSIIKNGQEYFDG;
VKSIIKNGQEYFDGQ; KSIIKNGQEYFDGQV; SIIKNGQEYFDGQVT;
IIKNGQEYFDGQVTD; IKNGQEYFDGQVTDS; KNGQEYFDGQVTDSE;
NGQEYFDGQVTDSEL; GQEYFDGQVTDSELG; QEYFDGQVTDSELGI;
EYFDGQVTDSELGIR; YFDGQVTDSELGIRA; FDGQVTDSELGIRAI;
DGQVTDSELGIRAID; GQVTDSELGIRAIDS; QVTDSELGIRAIDSK;
VTDSELGIRAIDSKT; TDSELGIRAIDSKTL; DSELGIRAIDSKTLE;
SELGIRAIDSKTLEI; ELGIRAIDSKTLEIT; LGIRAIDSKTLEITL;
GIRAIDSKTLEITLA; IRAIDSKTLEITLAS; RAIDSKTLEITLASP;
AIDSKTLEITLASPK; IDSKTLEITLASPKP; DSKTLEITLASPKPY;
SKTLEITLASPKPYF; KTLEITLASPKPYFI; TLEITLASPKPYFID;
LEITLASPKPYFIDL; EITLASPKPYFIDLL; ITLASPKPYFIDLLV;
TLASPKPYFIDLLVH; LASPKPYFIDLLVHQ; ASPKPYFIDLLVHQS;
SPKPYFIDLLVHQSF; PKPYFIDLLVHQSFI; KPYFIDLLVHQSFIP;
PYFIDLLVHQSFIPV; YFIDLLVHQSFIPVP; FIDLLVHQSFIPVPV;
IDLLVHQSFIPVPVH; DLLVHQSFIPVPVHV; LLVHQSFIPVPVHVT;
LVHQSFIPVPVHVTD; VHQSFIPVPVHVTDK; HQSFIPVPVHVTDKY;
QSFIPVPVHVTDKYG; SFIPVPVHVTDKYGQ; FIPVPVHVTDKYGQN;
IPVPVHVTDKYGQNW; PVPVHVTDKYGQNWT; VPVHVTDKYGQNWTS;
PVHVTDKYGQNWTSP; VHVTDKYGQNWTSPE; HVTDKYGQNWTSPEN;
VTDKYGQNWTSPENM; TDKYGQNWTSPENMV; DKYGQNWTSPENMVT;
KYGQNWTSPENMVTS; YGQNWTSPENMVTSG; GQNWTSPENMVTSGP;
QNWTSPENMVTSGPF; NWTSPENMVTSGPFK; WTSPENMVTSGPFKL;
TSPENMVTSGPFKLK; SPENMVTSGPFKLKE; PENMVTSGPFKLKER;
ENMVTSGPFKLKERI; NMVTSGPFKLKERIP; MVTSGPFKLKERIPS;
VTSGPFKLKERIPSE; TSGPFKLKERIPSEK; SGPFKLKERIPSEKY;
GPFKLKERIPSEKYV; PFKLKERIPSEKYVF; FKLKERIPSEKYVFE;
KLKERIPSEKYVFEK; LKERIPSEKYVFEKD; KERIPSEKYVFEKDN;
ERIPSEKYVFEKDNK; RIPSEKYVFEKDNKY; IPSEKYVFEKDNKYY;
PSEKYVFEKDNKYYD; SEKYVFEKDNKYYDS; EKYVFEKDNKYYDSN;
KYVFEKDNKYYDSNE; YVFEKDNKYYDSNEV; VFEKDNKYYDSNEVE;
FEKDNKYYDSNEVEL; EKDNKYYDSNEVELE; KDNKYYDSNEVELEE;
DNKYYDSNEVELEEI; NKYYDSNEVELEEIT; KYYDSNEVELEEITF;
YYDSNEVELEEITFY; YDSNEVELEEITFYT; DSNEVELEEITFYTT;
SNEVELEEITFYTTN; NEVELEEITFYTTND; EVELEEITFYTTNDS;
VELEEITFYTTNDSS; ELEEITFYTTNDSST; LEEITFYTTNDSSTA;
EEITFYTTNDSSTAY; EITFYTTNDSSTAYK; ITFYTTNDSSTAYKM;
TFYTTNDSSTAYKMY; FYTTNDSSTAYKMYV; YTTNDSSTAYKMYVN;
TTNDSSTAYKMYVNE; TNDSSTAYKMYVNEE; NDSSTAYKMYVNEEL;
DSSTAYKMYVNEELD; SSTAYKMYVNEELDA; STAYKMYVNEELDAI;
TAYKMYVNEELDAIL; AYKMYVNEELDAILV; YKMYVNEELDAILVP;
KMYVNEELDAILVPY; MYVNEELDAILVPYP; YVNEELDAILVPYPQ;
VNEELDAILVPYPQI;

16 mers:

MKLQRSLSLIIFSLTV; KLQRSLSLIIFSLTVL; LQRSLSLIIFSLTVLC;
QRSLSLIIFSLTVLCC; RSLSLIIFSLTVLCCD; SLSLIIFSLTVLCCDN;
LSLIIFSLTVLCCDNK; SLIIFSLTVLCCDNKE; LIIFSLTVLCCDNKER;
IIFSLTVLCCDNKERK; IFSLTVLCCDNKERKE; FSLTVLCCDNKERKEG;
SLTVLCCDNKERKEGV; LTVLCCDNKERKEGVS; TVLCCDNKERKEGVST;
VLCCDNKERKEGVSTK; LCCDNKERKEGVSTKI; CCDNKERKEGVSTKIS;
CDNKERKEGVSTKISL; DNKERKEGVSTKISLG; NKERKEGVSTKISLGA;
KERKEGVSTKISLGAE; ERKEGVSTKISLGAEP; RKEGVSTKISLGAEPR;
KEGVSTKISLGAEPRS; EGVSTKISLGAEPRSL; GVSTKISLGAEPRSLD;
VSTKISLGAEPRSLDP; STKISLGAEPRSLDPQ; TKISLGAEPRSLDPQL;
KISLGAEPRSLDPQLA; ISLGAEPRSLDPQLAE; SLGAEPRSLDPQLAED;
LGAEPRSLDPQLAEDN; GAEPRSLDPQLAEDNV; AEPRSLDPQLAEDNVA;
EPRSLDPQLAEDNVAS; PRSLDPQLAEDNVASK; RSLDPQLAEDNVASKM;
SLDPQLAEDNVASKMI; LDPQLAEDNVASKMID; DPQLAEDNVASKMIDT;
PQLAEDNVASKMIDTL; QLAEDNVASKMIDTLY; LAEDNVASKMIDTLYR;
AEDNVASKMIDTLYRG; EDNVASKMIDTLYRGI; DNVASKMIDTLYRGIV;
NVASKMIDTLYRGIVT; VASKMIDTLYRGIVTG; ASKMIDTLYRGIVTGD;
SKMIDTLYRGIVTGDP; KMIDTLYRGIVTGDPN; MIDTLYRGIVTGDPNT;
IDTLYRGIVTGDPNTG; DTLYRGIVTGDPNTGG; TLYRGIVTGDPNTGGH;
LYRGIVTGDPNTGGHK; YRGIVTGDPNTGGHKP; RGIVTGDPNTGGHKPG;
GIVTGDPNTGGHKPGL; IVTGDPNTGGHKPGLA; VTGDPNTGGHKPGLAK;
TGDPNTGGHKPGLAKG; GDPNTGGHKPGLAKGW; DPNTGGHKPGLAKGWE;
PNTGGHKPGLAKGWET; NTGGHKPGLAKGWETS; TGGHKPGLAKGWETSS;
GGHKPGLAKGWETSSD; GHKPGLAKGWETSSDG; HKPGLAKGWETSSDGT;
KPGLAKGWETSSDGTA; PGLAKGWETSSDGTAY; GLAKGWETSSDGTAYP;
LAKGWETSSDGTAYPF; AKGWETSSDGTAYPFY; KGWETSSDGTAYPFYL;
GWETSSDGTAYPFYLR; WETSSDGTAYPFYLRD; ETSSDGTAYPFYLRDN;
TSSDGTAYPFYLRDNL; SSDGTAYPFYLRDNLT; SDGTAYPFYLRDNLTW;
DGTAYPFYLRDNLTWS; GTAYPFYLRDNLTWSD; TAYPFYLRDNLTWSDG;
AYPFYLRDNLTWSDGV; YPFYLRDNLTWSDGVA; PFYLRDNLTWSDGVAI;
FYLRDNLTWSDGVAIT; YLRDNLTWSDGVAITA; LRDNLTWSDGVAITAE;
RDNLTWSDGVAITAEG; DNLTWSDGVAITAEGI; NLTWSDGVAITAEGIR;
LTWSDGVAITAEGIRK; TWSDGVAITAEGIRKS; WSDGVAITAEGIRKSY;
SDGVAITAEGIRKSYL; DGVAITAEGIRKSYLR; GVAITAEGIRKSYLRI;
VAITAEGIRKSYLRIL; AITAEGIRKSYLRILN; ITAEGIRKSYLRILNK;
TAEGIRKSYLRILNKE; AEGIRKSYLRILNKET; EGIRKSYLRILNKETG;
GIRKSYLRILNKETGS; IRKSYLRILNKETGST; RKSYLRILNKETGSTY;
KSYLRILNKETGSTYV; SYLRILNKETGSTYVD; YLRILNKETGSTYVDM;
LRILNKETGSTYVDMV; RILNKETGSTYVDMVK; ILNKETGSTYVDMVKS;
LNKETGSTYVDMVKSI; NKETGSTYVDMVKSII; KETGSTYVDMVKSIIK;
ETGSTYVDMVKSIIKN; TGSTYVDMVKSIIKNG; GSTYVDMVKSIIKNGQ;
STYVDMVKSIIKNGQE; TYVDMVKSIIKNGQEY; YVDMVKSIIKNGQEYF;
VDMVKSIIKNGQEYFD; DMVKSIIKNGQEYFDG; MVKSIIKNGQEYFDGQ;
VKSIIKNGQEYFDGQV; KSIIKNGQEYFDGQVT; SIIKNGQEYFDGQVTD;
IIKNGQEYFDGQVTDS; IKNGQEYFDGQVTDSE; KNGQEYFDGQVTDSEL;
NGQEYFDGQVTDSELG; GQEYFDGQVTDSELGI; QEYFDGQVTDSELGIR;
EYFDGQVTDSELGIRA; YFDGQVTDSELGIRAI; FDGQVTDSELGIRAID;
DGQVTDSELGIRAIDS; GQVTDSELGIRAIDSK; QVTDSELGIRAIDSKT;
VTDSELGIRAIDSKTL; TDSELGIRAIDSKTLE; DSELGIRAIDSKTLEI;
SELGIRAIDSKTLEIT; ELGIRAIDSKTLEITL; LGIRAIDSKTLEITLA;
GIRAIDSKTLEITLAS; IRAIDSKTLEITLASP; RAIDSKTLEITLASPK;

|  |  |
|---|---|
|  | AIDSKTLEITLASPKP; IDSKTLEITLASPKPY; DSKTLEITLASPKPYF;<br>SKTLEITLASPKPYFI; KTLEITLASPKPYFID; TLEITLASPKPYFIDL;<br>LEITLASPKPYFIDLL; EITLASPKPYFIDLLV; ITLASPKPYFIDLLVH;<br>TLASPKPYFIDLLVHQ; LASPKPYFIDLLVHQS; ASPKPYFIDLLVHQSF;<br>SPKPYFIDLLVHQSFI; PKPYFIDLLVHQSFIP; KPYFIDLLVHQSFIPV;<br>PYFIDLLVHQSFIPVP; YFIDLLVHQSFIPVPV; FIDLLVHQSFIPVPVH;<br>IDLLVHQSFIPVPVHV; DLLVHQSFIPVPVHVT; LLVHQSFIPVPVHVTD;<br>LVHQSFIPVPVHVTDK; VHQSFIPVPVHVTDKY; HQSFIPVPVHVTDKYG;<br>QSFIPVPVHVTDKYGQ; SFIPVPVHVTDKYGQN; FIPVPVHVTDKYGQNW;<br>IPVPVHVTDKYGQNWT; PVPVHVTDKYGQNWTS; VPVHVTDKYGQNWTSP;<br>PVHVTDKYGQNWTSPE; VHVTDKYGQNWTSPEN; HVTDKYGQNWTSPENM;<br>VTDKYGQNWTSPENMV; TDKYGQNWTSPENMVT; DKYGQNWTSPENMVTS;<br>KYGQNWTSPENMVTSG; YGQNWTSPENMVTSGP; GQNWTSPENMVTSGPF;<br>QNWTSPENMVTSGPFK; NWTSPENMVTSGPFKL; WTSPENMVTSGPFKLK;<br>TSPENMVTSGPFKLKE; SPENMVTSGPFKLKER; PENMVTSGPFKLKERI;<br>ENMVTSGPFKLKERIP; NMVTSGPFKLKERIPS; MVTSGPFKLKERIPSE;<br>VTSGPFKLKERIPSEK; TSGPFKLKERIPSEKY; SGPFKLKERIPSEKYV;<br>GPFKLKERIPSEKYVF; PFKLKERIPSEKYVFE; FKLKERIPSEKYVFEK;<br>KLKERIPSEKYVFEKD; LKERIPSEKYVFEKDN; KERIPSEKYVFEKDNK;<br>ERIPSEKYVFEKDNKY; RIPSEKYVFEKDNKYY; IPSEKYVFEKDNKYYD;<br>PSEKYVFEKDNKYYDS; SEKYVFEKDNKYYDSN; EKYVFEKDNKYYDSNE;<br>KYVFEKDNKYYDSNEV; YVFEKDNKYYDSNEVE; VFEKDNKYYDSNEVEL;<br>FEKDNKYYDSNEVELE; EKDNKYYDSNEVELEE; KDNKYYDSNEVELEEI;<br>DNKYYDSNEVELEEIT; NKYYDSNEVELEEITF; KYYDSNEVELEEITFY;<br>YYDSNEVELEEITFYT; YDSNEVELEEITFYTT; DSNEVELEEITFYTTN;<br>SNEVELEEITFYTTND; NEVELEEITFYTTNDS; EVELEEITFYTTNDSS;<br>VELEEITFYTTNDSST; ELEEITFYTTNDSSTA; LEEITFYTTNDSSTAY;<br>EEITFYTTNDSSTAYK; EITFYTTNDSSTAYKM; ITFYTTNDSSTAYKMY;<br>TFYTTNDSSTAYKMYV; FYTTNDSSTAYKMYVN; YTTNDSSTAYKMYVNE;<br>TTNDSSTAYKMYVNEE; TNDSSTAYKMYVNEEL; NDSSTAYKMYVNEELD;<br>DSSTAYKMYVNEELDA; SSTAYKMYVNEELDAI; STAYKMYVNEELDAIL;<br>TAYKMYVNEELDAILV; AYKMYVNEELDAILVP; YKMYVNEELDAILVPY;<br>KMYVNEELDAILVPYP; MYVNEELDAILVPYPQ; YVNEELDAILVPYPQI; |
| Seq 22 | 13 mers:<br>MNLINKLFILTIL; NLINKLFILTILF; LINKLFILTILFS;<br>INKLFILTILFSS; NKLFILTILFSSV; KLFILTILFSSVI;<br>LFILTILFSSVIS; FILTILFSSVISC; ILTILFSSVISCK;<br>LTILFSSVISCKL; TILFSSVISCKLY; ILFSSVISCKLYK;<br>LFSSVISCKLYKK; FSSVISCKLYKKI; SSVISCKLYKKIT;<br>SVISCKLYKKITY; VISCKLYKKITYN; ISCKLYKKITYNA;<br>SCKLYKKITYNAD; CKLYKKITYNADQ; KLYKKITYNADQV;<br>LYKKITYNADQVI; YKKITYNADQVID; KKITYNADQVIDK;<br>KITYNADQVIDKL; ITYNADQVIDKLK; TYNADQVIDKLKS;<br>YNADQVIDKLKSN; NADQVIDKLKSNN; ADQVIDKLKSNNG;<br>DQVIDKLKSNNGS; QVIDKLKSNNGSF; VIDKLKSNNGSFN;<br>IDKLKSNNGSFNT; DKLKSNNGSFNTL; KLKSNNGSFNTLK;<br>LKSNNGSFNTLKS; KSNNGSFNTLKSN; SNNGSFNTLKSND;<br>NNGSFNTLKSNDD; NGSFNTLKSNDDS; GSFNTLKSNDDSK;<br>SFNTLKSNDDSKR; FNTLKSNDDSKRS; NTLKSNDDSKRSG; |

TLKSNDDSKRSGR; LKSNDDSKRSGRK; KSNDDSKRSGRKP;
SNDDSKRSGRKPR; NDDSKRSGRKPRS; DDSKRSGRKPRSV;
DSKRSGRKPRSVD; SKRSGRKPRSVDN; KRSGRKPRSVDNT;
RSGRKPRSVDNTY; SGRKPRSVDNTYM; GRKPRSVDNTYMD;
RKPRSVDNTYMDQ; KPRSVDNTYMDQD; PRSVDNTYMDQDT;
RSVDNTYMDQDTG; SVDNTYMDQDTGK; VDNTYMDQDTGKK;
DNTYMDQDTGKKP; NTYMDQDTGKKPL; TYMDQDTGKKPLM;
YMDQDTGKKPLMA; MDQDTGKKPLMAD; DQDTGKKPLMADM;
QDTGKKPLMADMQ; DTGKKPLMADMQP; TGKKPLMADMQPD;
GKKPLMADMQPDM; KKPLMADMQPDMQ; KPLMADMQPDMQN;
PLMADMQPDMQND; LMADMQPDMQNDN; MADMQPDMQNDNS;
ADMQPDMQNDNSS; DMQPDMQNDNSSS; MQPDMQNDNSSSN;
QPDMQNDNSSSNH; PDMQNDNSSSNHT; DMQNDNSSSNHTL;
MQNDNSSSNHTLQ; QNDNSSSNHTLQV; NDNSSSNHTLQVN;
DNSSSNHTLQVNI; NSSSNHTLQVNIQ; SSSNHTLQVNIQD;
SSNHTLQVNIQDN; SNHTLQVNIQDNE; NHTLQVNIQDNEA;
HTLQVNIQDNEAS; TLQVNIQDNEASE; LQVNIQDNEASEA;
QVNIQDNEASEAR; VNIQDNEASEARN; NIQDNEASEARNI;
IQDNEASEARNIM; QDNEASEARNIMT; DNEASEARNIMTE;
NEASEARNIMTEI; EASEARNIMTEIE; ASEARNIMTEIES;
SEARNIMTEIESS; EARNIMTEIESSK; ARNIMTEIESSKE;
RNIMTEIESSKEE; NIMTEIESSKEEY; IMTEIESSKEEYN;
MTEIESSKEEYNR; TEIESSKEEYNRI; EIESSKEEYNRIN;
IESSKEEYNRINE; ESSKEEYNRINED; SSKEEYNRINEDL;
SKEEYNRINEDLA; KEEYNRINEDLAK; EEYNRINEDLAKV;
EYNRINEDLAKVK; YNRINEDLAKVKA; NRINEDLAKVKAS;
RINEDLAKVKASL; INEDLAKVKASLD; NEDLAKVKASLDK;
EDLAKVKASLDKI; DLAKVKASLDKIK; LAKVKASLDKIKS;
AKVKASLDKIKSL; KVKASLDKIKSLL; VKASLDKIKSLLS;
KASLDKIKSLLST; ASLDKIKSLLSTA; SLDKIKSLLSTAK;
LDKIKSLLSTAKS; DKIKSLLSTAKSY; KIKSLLSTAKSYL;
IKSLLSTAKSYLE; KSLLSTAKSYLEQ; SLLSTAKSYLEQT;
LLSTAKSYLEQTR; LSTAKSYLEQTRR; STAKSYLEQTRRG;
TAKSYLEQTRRGV; AKSYLEQTRRGVG; KSYLEQTRRGVGS;
SYLEQTRRGVGSS; YLEQTRRGVGSSK; LEQTRRGVGSSKA;
EQTRRGVGSSKAN; QTRRGVGSSKANL; TRRGVGSSKANLA;
RRGVGSSKANLAL; RGVGSSKANLALL; GVGSSKANLALLP;
VGSSKANLALLPS; GSSKANLALLPSL; SSKANLALLPSLE;
SKANLALLPSLEE; KANLALLPSLEEA; ANLALLPSLEEAI;
NLALLPSLEEAIA; LALLPSLEEAIAK; ALLPSLEEAIAKV;
LLPSLEEAIAKVK; LPSLEEAIAKVKS; PSLEEAIAKVKSN;
SLEEAIAKVKSNH; LEEAIAKVKSNHA; EEAIAKVKSNHAS;
EAIAKVKSNHASA; AIAKVKSNHASAD; IAKVKSNHASADT;
AKVKSNHASADTH; KVKSNHASADTHC; VKSNHASADTHCN;
KSNHASADTHCND; SNHASADTHCNDA; NHASADTHCNDAI;
HASADTHCNDAIA; ASADTHCNDAIAA; SADTHCNDAIAAL;
ADTHCNDAIAALK; DTHCNDAIAALKR; THCNDAIAALKRA;
HCNDAIAALKRAK; CNDAIAALKRAKN; NDAIAALKRAKND;
DAIAALKRAKNDF; AIAALKRAKNDFE; IAALKRAKNDFEY;
AALKRAKNDFEYA; ALKRAKNDFEYAQ; LKRAKNDFEYAQR;
KRAKNDFEYAQRK; RAKNDFEYAQRKA; AKNDFEYAQRKAD;

KNDFEYAQRKADR; NDFEYAQRKADRA; DFEYAQRKADRAL;
FEYAQRKADRALE; EYAQRKADRALEE; YAQRKADRALEEA;
AQRKADRALEEAL; QRKADRALEEALS; RKADRALEEALSN;
KADRALEEALSNS; ADRALEEALSNSN; DRALEEALSNSNA;
RALEEALSNSNAS; ALEEALSNSNASR; LEEALSNSNASRH;
EEALSNSNASRHE; EALSNSNASRHES; ALSNSNASRHESY;
LSNSNASRHESYY; SNSNASRHESYYY; NSNASRHESYYYA;
SNASRHESYYYAG; NASRHESYYYAGY; ASRHESYYYAGYH;
SRHESYYYAGYHQ; RHESYYYAGYHQF; HESYYYAGYHQFM;
ESYYYAGYHQFMA; SYYYAGYHQFMAD; YYYAGYHQFMADA;
YYAGYHQFMADAK; YAGYHQFMADAKA; AGYHQFMADAKAS;
GYHQFMADAKASM; YHQFMADAKASMS; HQFMADAKASMSS;
QFMADAKASMSST; FMADAKASMSSTK; MADAKASMSSTKS;
ADAKASMSSTKSL; DAKASMSSTKSLL; AKASMSSTKSLLE;
KASMSSTKSLLEV; ASMSSTKSLLEVA; SMSSTKSLLEVAK;
MSSTKSLLEVAKN; SSTKSLLEVAKNK; STKSLLEVAKNKQ;
TKSLLEVAKNKQK; KSLLEVAKNKQKE; SLLEVAKNKQKEL;
LLEVAKNKQKELN; LEVAKNKQKELNE; EVAKNKQKELNEN;
VAKNKQKELNENM; AKNKQKELNENMT; KNKQKELNENMTK;
NKQKELNENMTKT; KQKELNENMTKTN; QKELNENMTKTNK;
KELNENMTKTNKD; ELNENMTKTNKDF; LNENMTKTNKDFQ;
NENMTKTNKDFQE; ENMTKTNKDFQEL; NMTKTNKDFQELN;
MTKTNKDFQELND; TKTNKDFQELNDI; KTNKDFQELNDIY;
TNKDFQELNDIYK; NKDFQELNDIYKK; KDFQELNDIYKKL;
DFQELNDIYKKLQ; FQELNDIYKKLQD; QELNDIYKKLQDM;
ELNDIYKKLQDMD; LNDIYKKLQDMDS; NDIYKKLQDMDSR;

14 mers:
MNLINKLFILTILF; NLINKLFILTILFS; LINKLFILTILFSS;
INKLFILTILFSSV; NKLFILTILFSSVI; KLFILTILFSSVIS;
LFILTILFSSVISC; FILTILFSSVISCK; ILTILFSSVISCKL;
LTILFSSVISCKLY; TILFSSVISCKLYK; ILFSSVISCKLYKK;
LFSSVISCKLYKKI; FSSVISCKLYKKIT; SSVISCKLYKKITY;
SVISCKLYKKITYN; VISCKLYKKITYNA; ISCKLYKKITYNAD;
SCKLYKKITYNADQ; CKLYKKITYNADQV; KLYKKITYNADQVI;
LYKKITYNADQVID; YKKITYNADQVIDK; KKITYNADQVIDKL;
KITYNADQVIDKLK; ITYNADQVIDKLKS; TYNADQVIDKLKSN;
YNADQVIDKLKSNN; NADQVIDKLKSNNG; ADQVIDKLKSNNGS;
DQVIDKLKSNNGSF; QVIDKLKSNNGSFN; VIDKLKSNNGSFNT;
IDKLKSNNGSFNTL; DKLKSNNGSFNTLK; KLKSNNGSFNTLKS;
LKSNNGSFNTLKSN; KSNNGSFNTLKSND; SNNGSFNTLKSNDD;
NNGSFNTLKSNDDS; NGSFNTLKSNDDSK; GSFNTLKSNDDSKR;
SFNTLKSNDDSKRS; FNTLKSNDDSKRSG; NTLKSNDDSKRSGR;
TLKSNDDSKRSGRK; LKSNDDSKRSGRKP; KSNDDSKRSGRKPR;
SNDDSKRSGRKPRS; NDDSKRSGRKPRSV; DDSKRSGRKPRSVD;
DSKRSGRKPRSVDN; SKRSGRKPRSVDNT; KRSGRKPRSVDNTY;
RSGRKPRSVDNTYM; SGRKPRSVDNTYMD; GRKPRSVDNTYMDQ;
RKPRSVDNTYMDQD; KPRSVDNTYMDQDT; PRSVDNTYMDQDTG;
RSVDNTYMDQDTGK; SVDNTYMDQDTGKK; VDNTYMDQDTGKKP;
DNTYMDQDTGKKPL; NTYMDQDTGKKPLM; TYMDQDTGKKPLMA;
YMDQDTGKKPLMAD; MDQDTGKKPLMADM; DQDTGKKPLMADMQ;

QDTGKKPLMADMQP; DTGKKPLMADMQPD; TGKKPLMADMQPDM;
GKKPLMADMQPDMQ; KKPLMADMQPDMQN; KPLMADMQPDMQND;
PLMADMQPDMQNDN; LMADMQPDMQNDNS; MADMQPDMQNDNSS;
ADMQPDMQNDNSSS; DMQPDMQNDNSSSN; MQPDMQNDNSSSNH;
QPDMQNDNSSSNHT; PDMQNDNSSSNHTL; DMQNDNSSSNHTLQ;
MQNDNSSSNHTLQV; QNDNSSSNHTLQVN; NDNSSSNHTLQVNI;
DNSSSNHTLQVNIQ; NSSSNHTLQVNIQD; SSSNHTLQVNIQDN;
SSNHTLQVNIQDNE; SNHTLQVNIQDNEA; NHTLQVNIQDNEAS;
HTLQVNIQDNEASE; TLQVNIQDNEASEA; LQVNIQDNEASEAR;
QVNIQDNEASEARN; VNIQDNEASEARNI; NIQDNEASEARNIM;
IQDNEASEARNIMT; QDNEASEARNIMTE; DNEASEARNIMTEI;
NEASEARNIMTEIE; EASEARNIMTEIES; ASEARNIMTEIESS;
SEARNIMTEIESSK; EARNIMTEIESSKE; ARNIMTEIESSKEE;
RNIMTEIESSKEEY; NIMTEIESSKEEYN; IMTEIESSKEEYNR;
MTEIESSKEEYNRI; TEIESSKEEYNRIN; EIESSKEEYNRINE;
IESSKEEYNRINED; ESSKEEYNRINEDL; SSKEEYNRINEDLA;
SKEEYNRINEDLAK; KEEYNRINEDLAKV; EEYNRINEDLAKVK;
EYNRINEDLAKVKA; YNRINEDLAKVKAS; NRINEDLAKVKASL;
RINEDLAKVKASLD; INEDLAKVKASLDK; NEDLAKVKASLDKI;
EDLAKVKASLDKIK; DLAKVKASLDKIKS; LAKVKASLDKIKSL;
AKVKASLDKIKSLL; KVKASLDKIKSLLS; VKASLDKIKSLLST;
KASLDKIKSLLSTA; ASLDKIKSLLSTAK; SLDKIKSLLSTAKS;
LDKIKSLLSTAKSY; DKIKSLLSTAKSYL; KIKSLLSTAKSYLE;
IKSLLSTAKSYLEQ; KSLLSTAKSYLEQT; SLLSTAKSYLEQTR;
LLSTAKSYLEQTRR; LSTAKSYLEQTRRG; STAKSYLEQTRRGV;
TAKSYLEQTRRGVG; AKSYLEQTRRGVGS; KSYLEQTRRGVGSS;
SYLEQTRRGVGSSK; YLEQTRRGVGSSKA; LEQTRRGVGSSKAN;
EQTRRGVGSSKANL; QTRRGVGSSKANLA; TRRGVGSSKANLAL;
RRGVGSSKANLALL; RGVGSSKANLALLP; GVGSSKANLALLPS;
VGSSKANLALLPSL; GSSKANLALLPSLE; SSKANLALLPSLEE;
SKANLALLPSLEEA; KANLALLPSLEEAI; ANLALLPSLEEAIA;
NLALLPSLEEAIAK; LALLPSLEEAIAKV; ALLPSLEEAIAKVK;
LLPSLEEAIAKVKS; LPSLEEAIAKVKSN; PSLEEAIAKVKSNH;
SLEEAIAKVKSNHA; LEEAIAKVKSNHAS; EEAIAKVKSNHASA;
EAIAKVKSNHASAD; AIAKVKSNHASADT; IAKVKSNHASADTH;
AKVKSNHASADTHC; KVKSNHASADTHCN; VKSNHASADTHCND;
KSNHASADTHCNDA; SNHASADTHCNDAI; NHASADTHCNDAIA;
HASADTHCNDAIAA; ASADTHCNDAIAAL; SADTHCNDAIAALK;
ADTHCNDAIAALKR; DTHCNDAIAALKRA; THCNDAIAALKRAK;
HCNDAIAALKRAKN; CNDAIAALKRAKND; NDAIAALKRAKNDF;
DAIAALKRAKNDFE; AIAALKRAKNDFEY; IAALKRAKNDFEYA;
AALKRAKNDFEYAQ; ALKRAKNDFEYAQR; LKRAKNDFEYAQRK;
KRAKNDFEYAQRKA; RAKNDFEYAQRKAD; AKNDFEYAQRKADR;
KNDFEYAQRKADRA; NDFEYAQRKADRAL; DFEYAQRKADRALE;
FEYAQRKADRALEE; EYAQRKADRALEEA; YAQRKADRALEEAL;
AQRKADRALEEALS; QRKADRALEEALSN; RKADRALEEALSNS;
KADRALEEALSNSN; ADRALEEALSNSNA; DRALEEALSNSNAS;
RALEEALSNSNASR; ALEEALSNSNASRH; LEEALSNSNASRHE;
EEALSNSNASRHES; EALSNSNASRHESY; ALSNSNASRHESYY;
LSNSNASRHESYYY; SNSNASRHESYYYA; NSNASRHESYYYAG;
SNASRHESYYYAGY; NASRHESYYYAGYH; ASRHESYYYAGYHQ;

SRHESYYYAGYHQF; RHESYYYAGYHQFM; HESYYYAGYHQFMA;
ESYYYAGYHQFMAD; SYYYAGYHQFMADA; YYYAGYHQFMADAK;
YYAGYHQFMADAKA; YAGYHQFMADAKAS; AGYHQFMADAKASM;
GYHQFMADAKASMS; YHQFMADAKASMSS; HQFMADAKASMSST;
QFMADAKASMSSTK; FMADAKASMSSTKS; MADAKASMSSTKSL;
ADAKASMSSTKSLL; DAKASMSSTKSLLE; AKASMSSTKSLLEV;
KASMSSTKSLLEVA; ASMSSTKSLLEVAK; SMSSTKSLLEVAKN;
MSSTKSLLEVAKNK; SSTKSLLEVAKNKQ; STKSLLEVAKNKQK;
TKSLLEVAKNKQKE; KSLLEVAKNKQKEL; SLLEVAKNKQKELN;
LLEVAKNKQKELNE; LEVAKNKQKELNEN; EVAKNKQKELNENM;
VAKNKQKELNENMT; AKNKQKELNENMTK; KNKQKELNENMTKT;
NKQKELNENMTKTN; KQKELNENMTKTNK; QKELNENMTKTNKD;
KELNENMTKTNKDF; ELNENMTKTNKDFQ; LNENMTKTNKDFQE;
NENMTKTNKDFQEL; ENMTKTNKDFQELN; NMTKTNKDFQELND;
MTKTNKDFQELNDI; TKTNKDFQELNDIY; KTNKDFQELNDIYK;
TNKDFQELNDIYKK; NKDFQELNDIYKKL; KDFQELNDIYKKLQ;
DFQELNDIYKKLQD; FQELNDIYKKLQDM; QELNDIYKKLQDMD;
ELNDIYKKLQDMDS; LNDIYKKLQDMDSR;

15 mers:
MNLINKLFILTILFS; NLINKLFILTILFSS; LINKLFILTILFSSV;
INKLFILTILFSSVI; NKLFILTILFSSVIS; KLFILTILFSSVISC;
LFILTILFSSVISCK; FILTILFSSVISCKL; ILTILFSSVISCKLY;
LTILFSSVISCKLYK; TILFSSVISCKLYKK; ILFSSVISCKLYKKI;
LFSSVISCKLYKKIT; FSSVISCKLYKKITY; SSVISCKLYKKITYN;
SVISCKLYKKITYNA; VISCKLYKKITYNAD; ISCKLYKKITYNADQ;
SCKLYKKITYNADQV; CKLYKKITYNADQVI; KLYKKITYNADQVID;
LYKKITYNADQVIDK; YKKITYNADQVIDKL; KKITYNADQVIDKLK;
KITYNADQVIDKLKS; ITYNADQVIDKLKSN; TYNADQVIDKLKSNN;
YNADQVIDKLKSNNG; NADQVIDKLKSNNGS; ADQVIDKLKSNNGSF;
DQVIDKLKSNNGSFN; QVIDKLKSNNGSFNT; VIDKLKSNNGSFNTL;
IDKLKSNNGSFNTLK; DKLKSNNGSFNTLKS; KLKSNNGSFNTLKSN;
LKSNNGSFNTLKSND; KSNNGSFNTLKSNDD; SNNGSFNTLKSNDDS;
NNGSFNTLKSNDDSK; NGSFNTLKSNDDSKR; GSFNTLKSNDDSKRS;
SFNTLKSNDDSKRSG; FNTLKSNDDSKRSGR; NTLKSNDDSKRSGRK;
TLKSNDDSKRSGRKP; LKSNDDSKRSGRKPR; KSNDDSKRSGRKPRS;
SNDDSKRSGRKPRSV; NDDSKRSGRKPRSVD; DDSKRSGRKPRSVDN;
DSKRSGRKPRSVDNT; SKRSGRKPRSVDNTY; KRSGRKPRSVDNTYM;
RSGRKPRSVDNTYMD; SGRKPRSVDNTYMDQ; GRKPRSVDNTYMDQD;
RKPRSVDNTYMDQDT; KPRSVDNTYMDQDTG; PRSVDNTYMDQDTGK;
RSVDNTYMDQDTGKK; SVDNTYMDQDTGKKP; VDNTYMDQDTGKKPL;
DNTYMDQDTGKKPLM; NTYMDQDTGKKPLMA; TYMDQDTGKKPLMAD;
YMDQDTGKKPLMADM; MDQDTGKKPLMADMQ; DQDTGKKPLMADMQP;
QDTGKKPLMADMQPD; DTGKKPLMADMQPDM; TGKKPLMADMQPDMQ;
GKKPLMADMQPDMQN; KKPLMADMQPDMQND; KPLMADMQPDMQNDN;
PLMADMQPDMQNDNS; LMADMQPDMQNDNSS; MADMQPDMQNDNSSS;
ADMQPDMQNDNSSSN; DMQPDMQNDNSSSNH; MQPDMQNDNSSSNHT;
QPDMQNDNSSSNHTL; PDMQNDNSSSNHTLQ; DMQNDNSSSNHTLQV;
MQNDNSSSNHTLQVN; QNDNSSSNHTLQVNI; NDNSSSNHTLQVNIQ;
DNSSSNHTLQVNIQD; NSSSNHTLQVNIQDN; SSSNHTLQVNIQDNE;
SSNHTLQVNIQDNEA; SNHTLQVNIQDNEAS; NHTLQVNIQDNEASE;

HTLQVNIQDNEASEA; TLQVNIQDNEASEAR; LQVNIQDNEASEARN;
QVNIQDNEASEARNI; VNIQDNEASEARNIM; NIQDNEASEARNIMT;
IQDNEASEARNIMTE; QDNEASEARNIMTEI; DNEASEARNIMTEIE;
NEASEARNIMTEIES; EASEARNIMTEIESS; ASEARNIMTEIESSK;
SEARNIMTEIESSKE; EARNIMTEIESSKEE; ARNIMTEIESSKEEY;
RNIMTEIESSKEEYN; NIMTEIESSKEEYNR; IMTEIESSKEEYNRI;
MTEIESSKEEYNRIN; TEIESSKEEYNRINE; EIESSKEEYNRINED;
IESSKEEYNRINEDL; ESSKEEYNRINEDLA; SSKEEYNRINEDLAK;
SKEEYNRINEDLAKV; KEEYNRINEDLAKVK; EEYNRINEDLAKVKA;
EYNRINEDLAKVKAS; YNRINEDLAKVKASL; NRINEDLAKVKASLD;
RINEDLAKVKASLDK; INEDLAKVKASLDKI; NEDLAKVKASLDKIK;
EDLAKVKASLDKIKS; DLAKVKASLDKIKSL; LAKVKASLDKIKSLL;
AKVKASLDKIKSLLS; KVKASLDKIKSLLST; VKASLDKIKSLLSTA;
KASLDKIKSLLSTAK; ASLDKIKSLLSTAKS; SLDKIKSLLSTAKSY;
LDKIKSLLSTAKSYL; DKIKSLLSTAKSYLE; KIKSLLSTAKSYLEQ;
IKSLLSTAKSYLEQT; KSLLSTAKSYLEQTR; SLLSTAKSYLEQTRR;
LLSTAKSYLEQTRRG; LSTAKSYLEQTRRGV; STAKSYLEQTRRGVG;
TAKSYLEQTRRGVGS; AKSYLEQTRRGVGSS; KSYLEQTRRGVGSSK;
SYLEQTRRGVGSSKA; YLEQTRRGVGSSKAN; LEQTRRGVGSSKANL;
EQTRRGVGSSKANLA; QTRRGVGSSKANLAL; TRRGVGSSKANLALL;
RRGVGSSKANLALLP; RGVGSSKANLALLPS; GVGSSKANLALLPSL;
VGSSKANLALLPSLE; GSSKANLALLPSLEE; SSKANLALLPSLEEA;
SKANLALLPSLEEAI; KANLALLPSLEEAIA; ANLALLPSLEEAIAK;
NLALLPSLEEAIAKV; LALLPSLEEAIAKVK; ALLPSLEEAIAKVKS;
LLPSLEEAIAKVKSN; LPSLEEAIAKVKSNH; PSLEEAIAKVKSNHA;
SLEEAIAKVKSNHAS; LEEAIAKVKSNHASA; EEAIAKVKSNHASAD;
EAIAKVKSNHASADT; AIAKVKSNHASADTH; IAKVKSNHASADTHC;
AKVKSNHASADTHCN; KVKSNHASADTHCND; VKSNHASADTHCNDA;
KSNHASADTHCNDAI; SNHASADTHCNDAIA; NHASADTHCNDAIAA;
HASADTHCNDAIAAL; ASADTHCNDAIAALK; SADTHCNDAIAALKR;
ADTHCNDAIAALKRA; DTHCNDAIAALKRAK; THCNDAIAALKRAKN;
HCNDAIAALKRAKND; CNDAIAALKRAKNDF; NDAIAALKRAKNDFE;
DAIAALKRAKNDFEY; AIAALKRAKNDFEYA; IAALKRAKNDFEYAQ;
AALKRAKNDFEYAQR; ALKRAKNDFEYAQRK; LKRAKNDFEYAQRKA;
KRAKNDFEYAQRKAD; RAKNDFEYAQRKADR; AKNDFEYAQRKADRA;
KNDFEYAQRKADRAL; NDFEYAQRKADRALE; DFEYAQRKADRALEE;
FEYAQRKADRALEEA; EYAQRKADRALEEAL; YAQRKADRALEEALS;
AQRKADRALEEALSN; QRKADRALEEALSNS; RKADRALEEALSNSN;
KADRALEEALSNSNA; ADRALEEALSNSNAS; DRALEEALSNSNASR;
RALEEALSNSNASRH; ALEEALSNSNASRHE; LEEALSNSNASRHES;
EEALSNSNASRHESY; EALSNSNASRHESYY; ALSNSNASRHESYYY;
LSNSNASRHESYYYA; SNSNASRHESYYYAG; NSNASRHESYYYAGY;
SNASRHESYYYAGYH; NASRHESYYYAGYHQ; ASRHESYYYAGYHQF;
SRHESYYYAGYHQFM; RHESYYYAGYHQFMA; HESYYYAGYHQFMAD;
ESYYYAGYHQFMADA; SYYYAGYHQFMADAK; YYYAGYHQFMADAKA;
YYAGYHQFMADAKAS; YAGYHQFMADAKASM; AGYHQFMADAKASMS;
GYHQFMADAKASMSS; YHQFMADAKASMSST; HQFMADAKASMSSTK;
QFMADAKASMSSTKS; FMADAKASMSSTKSL; MADAKASMSSTKSLL;
ADAKASMSSTKSLLE; DAKASMSSTKSLLEV; AKASMSSTKSLLEVA;
KASMSSTKSLLEVAK; ASMSSTKSLLEVAKN; SMSSTKSLLEVAKNK;
MSSTKSLLEVAKNKQ; SSTKSLLEVAKNKQK; STKSLLEVAKNKQKE;

TKSLLEVAKNKQKEL; KSLLEVAKNKQKELN; SLLEVAKNKQKELNE;
LLEVAKNKQKELNEN; LEVAKNKQKELNENM; EVAKNKQKELNENMT;
VAKNKQKELNENMTK; AKNKQKELNENMTKT; KNKQKELNENMTKTN;
NKQKELNENMTKTNK; KQKELNENMTKTNKD; QKELNENMTKTNKDF;
KELNENMTKTNKDFQ; ELNENMTKTNKDFQE; LNENMTKTNKDFQEL;
NENMTKTNKDFQELN; ENMTKTNKDFQELND; NMTKTNKDFQELNDI;
MTKTNKDFQELNDIY; TKTNKDFQELNDIYK; KTNKDFQELNDIYKK;
TNKDFQELNDIYKKL; NKDFQELNDIYKKLQ; KDFQELNDIYKKLQD;
DFQELNDIYKKLQDM; FQELNDIYKKLQDMD; QELNDIYKKLQDMDS;
ELNDIYKKLQDMDSR;

16 mers:
MNLINKLFILTILFSS; NLINKLFILTILFSSV; LINKLFILTILFSSVI;
INKLFILTILFSSVIS; NKLFILTILFSSVISC; KLFILTILFSSVISCK;
LFILTILFSSVISCKL; FILTILFSSVISCKLY; ILTILFSSVISCKLYK;
LTILFSSVISCKLYKK; TILFSSVISCKLYKKI; ILFSSVISCKLYKKIT;
LFSSVISCKLYKKITY; FSSVISCKLYKKITYN; SSVISCKLYKKITYNA;
SVISCKLYKKITYNAD; VISCKLYKKITYNADQ; ISCKLYKKITYNADQV;
SCKLYKKITYNADQVI; CKLYKKITYNADQVID; KLYKKITYNADQVIDK;
LYKKITYNADQVIDKL; YKKITYNADQVIDKLK; KKITYNADQVIDKLKS;
KITYNADQVIDKLKSN; ITYNADQVIDKLKSNN; TYNADQVIDKLKSNNG;
YNADQVIDKLKSNNGS; NADQVIDKLKSNNGSF; ADQVIDKLKSNNGSFN;
DQVIDKLKSNNGSFNT; QVIDKLKSNNGSFNTL; VIDKLKSNNGSFNTLK;
IDKLKSNNGSFNTLKS; DKLKSNNGSFNTLKSN; KLKSNNGSFNTLKSND;
LKSNNGSFNTLKSNDD; KSNNGSFNTLKSNDDS; SNNGSFNTLKSNDDSK;
NNGSFNTLKSNDDSKR; NGSFNTLKSNDDSKRS; GSFNTLKSNDDSKRSG;
SFNTLKSNDDSKRSGR; FNTLKSNDDSKRSGRK; NTLKSNDDSKRSGRKP;
TLKSNDDSKRSGRKPR; LKSNDDSKRSGRKPRS; KSNDDSKRSGRKPRSV;
SNDDSKRSGRKPRSVD; NDDSKRSGRKPRSVDN; DDSKRSGRKPRSVDNT;
DSKRSGRKPRSVDNTY; SKRSGRKPRSVDNTYM; KRSGRKPRSVDNTYMD;
RSGRKPRSVDNTYMDQ; SGRKPRSVDNTYMDQD; GRKPRSVDNTYMDQDT;
RKPRSVDNTYMDQDTG; KPRSVDNTYMDQDTGK; PRSVDNTYMDQDTGKK;
RSVDNTYMDQDTGKKP; SVDNTYMDQDTGKKPL; VDNTYMDQDTGKKPLM;
DNTYMDQDTGKKPLMA; NTYMDQDTGKKPLMAD; TYMDQDTGKKPLMADM;
YMDQDTGKKPLMADMQ; MDQDTGKKPLMADMQP; DQDTGKKPLMADMQPD;
QDTGKKPLMADMQPDM; DTGKKPLMADMQPDMQ; TGKKPLMADMQPDMQN;
GKKPLMADMQPDMQND; KKPLMADMQPDMQNDN; KPLMADMQPDMQNDNS;
PLMADMQPDMQNDNSS; LMADMQPDMQNDNSSS; MADMQPDMQNDNSSSN;
ADMQPDMQNDNSSSNH; DMQPDMQNDNSSSNHT; MQPDMQNDNSSSNHTL;
QPDMQNDNSSSNHTLQ; PDMQNDNSSSNHTLQV; DMQNDNSSSNHTLQVN;
MQNDNSSSNHTLQVNI; QNDNSSSNHTLQVNIQ; NDNSSSNHTLQVNIQD;
DNSSSNHTLQVNIQDN; NSSSNHTLQVNIQDNE; SSSNHTLQVNIQDNEA;
SSNHTLQVNIQDNEAS; SNHTLQVNIQDNEASE; NHTLQVNIQDNEASEA;
HTLQVNIQDNEASEAR; TLQVNIQDNEASEARN; LQVNIQDNEASEARNI;
QVNIQDNEASEARNIM; VNIQDNEASEARNIMT; NIQDNEASEARNIMTE;
IQDNEASEARNIMTEI; QDNEASEARNIMTEIE; DNEASEARNIMTEIES;
NEASEARNIMTEIESS; EASEARNIMTEIESSK; ASEARNIMTEIESSKE;
SEARNIMTEIESSKEE; EARNIMTEIESSKEEY; ARNIMTEIESSKEEYN;
RNIMTEIESSKEEYNR; NIMTEIESSKEEYNRI; IMTEIESSKEEYNRIN;
MTEIESSKEEYNRINE; TEIESSKEEYNRINED; EIESSKEEYNRINEDL;
IESSKEEYNRINEDLA; ESSKEEYNRINEDLAK; SSKEEYNRINEDLAKV;

| | | |
|---|---|---|
| SKEEYNRINEDLAKVK; | KEEYNRINEDLAKVKA; | EEYNRINEDLAKVKAS; |
| EYNRINEDLAKVKASL; | YNRINEDLAKVKASLD; | NRINEDLAKVKASLDK; |
| RINEDLAKVKASLDKI; | INEDLAKVKASLDKIK; | NEDLAKVKASLDKIKS; |
| EDLAKVKASLDKIKSL; | DLAKVKASLDKIKSLL; | LAKVKASLDKIKSLLS; |
| AKVKASLDKIKSLLST; | KVKASLDKIKSLLSTA; | VKASLDKIKSLLSTAK; |
| KASLDKIKSLLSTAKS; | ASLDKIKSLLSTAKSY; | SLDKIKSLLSTAKSYL; |
| LDKIKSLLSTAKSYLE; | DKIKSLLSTAKSYLEQ; | KIKSLLSTAKSYLEQT; |
| IKSLLSTAKSYLEQTR; | KSLLSTAKSYLEQTRR; | SLLSTAKSYLEQTRRG; |
| LLSTAKSYLEQTRRGV; | LSTAKSYLEQTRRGVG; | STAKSYLEQTRRGVGS; |
| TAKSYLEQTRRGVGSS; | AKSYLEQTRRGVGSSK; | KSYLEQTRRGVGSSKA; |
| SYLEQTRRGVGSSKAN; | YLEQTRRGVGSSKANL; | LEQTRRGVGSSKANLA; |
| EQTRRGVGSSKANLAL; | QTRRGVGSSKANLALL; | TRRGVGSSKANLALLP; |
| RRGVGSSKANLALLPS; | RGVGSSKANLALLPSL; | GVGSSKANLALLPSLE; |
| VGSSKANLALLPSLEE; | GSSKANLALLPSLEEA; | SSKANLALLPSLEEAI; |
| SKANLALLPSLEEAIA; | KANLALLPSLEEAIAK; | ANLALLPSLEEAIAKV; |
| NLALLPSLEEAIAKVK; | LALLPSLEEAIAKVKS; | ALLPSLEEAIAKVKSN; |
| LLPSLEEAIAKVKSNH; | LPSLEEAIAKVKSNHA; | PSLEEAIAKVKSNHAS; |
| SLEEAIAKVKSNHASA; | LEEAIAKVKSNHASAD; | EEAIAKVKSNHASADT; |
| EAIAKVKSNHASADTH; | AIAKVKSNHASADTHC; | IAKVKSNHASADTHCN; |
| AKVKSNHASADTHCND; | KVKSNHASADTHCNDA; | VKSNHASADTHCNDAI; |
| KSNHASADTHCNDAIA; | SNHASADTHCNDAIAA; | NHASADTHCNDAIAAL; |
| HASADTHCNDAIAALK; | ASADTHCNDAIAALKR; | SADTHCNDAIAALKRA; |
| ADTHCNDAIAALKRAK; | DTHCNDAIAALKRAKN; | THCNDAIAALKRAKND; |
| HCNDAIAALKRAKNDF; | CNDAIAALKRAKNDFE; | NDAIAALKRAKNDFEY; |
| DAIAALKRAKNDFEYA; | AIAALKRAKNDFEYAQ; | IAALKRAKNDFEYAQR; |
| AALKRAKNDFEYAQRK; | ALKRAKNDFEYAQRKA; | LKRAKNDFEYAQRKAD; |
| KRAKNDFEYAQRKADR; | RAKNDFEYAQRKADRA; | AKNDFEYAQRKADRAL; |
| KNDFEYAQRKADRALE; | NDFEYAQRKADRALEE; | DFEYAQRKADRALEEA; |
| FEYAQRKADRALEEAL; | EYAQRKADRALEEALS; | YAQRKADRALEEALSN; |
| AQRKADRALEEALSNS; | QRKADRALEEALSNSN; | RKADRALEEALSNSNA; |
| KADRALEEALSNSNAS; | ADRALEEALSNSNASR; | DRALEEALSNSNASRH; |
| RALEEALSNSNASRHE; | ALEEALSNSNASRHES; | LEEALSNSNASRHESY; |
| EEALSNSNASRHESYY; | EALSNSNASRHESYYY; | ALSNSNASRHESYYYA; |
| LSNSNASRHESYYYAG; | SNSNASRHESYYYAGY; | NSNASRHESYYYAGYH; |
| SNASRHESYYYAGYHQ; | NASRHESYYYAGYHQF; | ASRHESYYYAGYHQFM; |
| SRHESYYYAGYHQFMA; | RHESYYYAGYHQFMAD; | HESYYYAGYHQFMADA; |
| ESYYYAGYHQFMADAK; | SYYYAGYHQFMADAKA; | YYYAGYHQFMADAKAS; |
| YYAGYHQFMADAKASM; | YAGYHQFMADAKASMS; | AGYHQFMADAKASMSS; |
| GYHQFMADAKASMSST; | YHQFMADAKASMSSTK; | HQFMADAKASMSSTKS; |
| QFMADAKASMSSTKSL; | FMADAKASMSSTKSLL; | MADAKASMSSTKSLLE; |
| ADAKASMSSTKSLLEV; | DAKASMSSTKSLLEVA; | AKASMSSTKSLLEVAK; |
| KASMSSTKSLLEVAKN; | ASMSSTKSLLEVAKNK; | SMSSTKSLLEVAKNKQ; |
| MSSTKSLLEVAKNKQK; | SSTKSLLEVAKNKQKE; | STKSLLEVAKNKQKEL; |
| TKSLLEVAKNKQKELN; | KSLLEVAKNKQKELNE; | SLLEVAKNKQKELNEN; |
| LLEVAKNKQKELNENM; | LEVAKNKQKELNENMT; | EVAKNKQKELNENMTK; |
| VAKNKQKELNENMTKT; | AKNKQKELNENMTKTN; | KNKQKELNENMTKTNK; |
| NKQKELNENMTKTNKD; | KQKELNENMTKTNKDF; | QKELNENMTKTNKDFQ; |
| KELNENMTKTNKDFQE; | ELNENMTKTNKDFQEL; | LNENMTKTNKDFQELN; |
| NENMTKTNKDFQELND; | ENMTKTNKDFQELNDI; | NMTKTNKDFQELNDIY; |
| MTKTNKDFQELNDIYK; | TKTNKDFQELNDIYKK; | KTNKDFQELNDIYKKL; |
| TNKDFQELNDIYKKLQ; | NKDFQELNDIYKKLQD; | KDFQELNDIYKKLQDM; |

| | |
|---|---|
| | DFQELNDIYKKLQDMD; FQELNDIYKKLQDMDS; QELNDIYKKLQDMDSR; |
| Seq 23 | 13 mers:<br>MIINHNTSAINAS; IINHNTSAINASR; INHNTSAINASRN;<br>NHNTSAINASRNN; HNTSAINASRNNG; NTSAINASRNNGI;<br>TSAINASRNNGIN; SAINASRNNGINA; AINASRNNGINAA;<br>INASRNNGINAAN; NASRNNGINAANL; ASRNNGINAANLS;<br>SRNNGINAANLSK; RNNGINAANLSKT; NNGINAANLSKTQ;<br>NGINAANLSKTQE; GINAANLSKTQEK; INAANLSKTQEKL;<br>NAANLSKTQEKLS; AANLSKTQEKLSS; ANLSKTQEKLSSG;<br>NLSKTQEKLSSGY; LSKTQEKLSSGYR; SKTQEKLSSGYRI;<br>KTQEKLSSGYRIN; TQEKLSSGYRINR; QEKLSSGYRINRA;<br>EKLSSGYRINRAS; KLSSGYRINRASD; LSSGYRINRASDD;<br>SSGYRINRASDDA; SGYRINRASDDAA; GYRINRASDDAAG;<br>YRINRASDDAAGM; RINRASDDAAGMG; INRASDDAAGMGV;<br>NRASDDAAGMGVS; RASDDAAGMGVSG; ASDDAAGMGVSGK;<br>SDDAAGMGVSGKI; DDAAGMGVSGKIN; DAAGMGVSGKINA;<br>AAGMGVSGKINAQ; AGMGVSGKINAQI; GMGVSGKINAQIR;<br>MGVSGKINAQIRG; GVSGKINAQIRGL; VSGKINAQIRGLS;<br>SGKINAQIRGLSQ; GKINAQIRGLSQA; KINAQIRGLSQAS;<br>INAQIRGLSQASR; NAQIRGLSQASRN; AQIRGLSQASRNT;<br>QIRGLSQASRNTS; IRGLSQASRNTSK; RGLSQASRNTSKA;<br>GLSQASRNTSKAI; LSQASRNTSKAIN; SQASRNTSKAINF;<br>QASRNTSKAINFI; ASRNTSKAINFIQ; SRNTSKAINFIQT;<br>RNTSKAINFIQTT; NTSKAINFIQTTE; TSKAINFIQTTEG;<br>SKAINFIQTTEGN; KAINFIQTTEGNL; AINFIQTTEGNLN;<br>INFIQTTEGNLNE; NFIQTTEGNLNEV; FIQTTEGNLNEVE;<br>IQTTEGNLNEVEK; QTTEGNLNEVEKV; TTEGNLNEVEKVL;<br>TEGNLNEVEKVLV; EGNLNEVEKVLVR; GNLNEVEKVLVRM;<br>NLNEVEKVLVRMK; LNEVEKVLVRMKE; NEVEKVLVRMKEL;<br>EVEKVLVRMKELA; VEKVLVRMKELAV; EKVLVRMKELAVQ;<br>KVLVRMKELAVQS; VLVRMKELAVQSG; LVRMKELAVQSGN;<br>VRMKELAVQSGNG; RMKELAVQSGNGT; MKELAVQSGNGTY;<br>KELAVQSGNGTYS; ELAVQSGNGTYSD; LAVQSGNGTYSDA;<br>AVQSGNGTYSDAD; VQSGNGTYSDADR; QSGNGTYSDADRG;<br>SGNGTYSDADRGS; GNGTYSDADRGSI; NGTYSDADRGSIQ;<br>GTYSDADRGSIQI; TYSDADRGSIQIE; YSDADRGSIQIEI;<br>SDADRGSIQIEIE; DADRGSIQIEIEQ; ADRGSIQIEIEQL;<br>DRGSIQIEIEQLT; RGSIQIEIEQLTD; GSIQIEIEQLTDE;<br>SIQIEIEQLTDEI; IQIEIEQLTDEIN; QIEIEQLTDEINR;<br>IEIEQLTDEINRI; EIEQLTDEINRIA; IEQLTDEINRIAD;<br>EQLTDEINRIADQ; QLTDEINRIADQA; LTDEINRIADQAQ;<br>TDEINRIADQAQY; DEINRIADQAQYN; EINRIADQAQYNQ;<br>INRIADQAQYNQM; NRIADQAQYNQMH; RIADQAQYNQMHM;<br>IADQAQYNQMHML; ADQAQYNQMHMLS; DQAQYNQMHMLSN;<br>QAQYNQMHMLSNK; AQYNQMHMLSNKS; QYNQMHMLSNKSA;<br>YNQMHMLSNKSAS; NQMHMLSNKSASQ; QMHMLSNKSASQN;<br>MHMLSNKSASQNV; HMLSNKSASQNVR; MLSNKSASQNVRT;<br>LSNKSASQNVRTA; SNKSASQNVRTAE; NKSASQNVRTAEE;<br>KSASQNVRTAEEL; SASQNVRTAEELG; ASQNVRTAEELGM; |

SQNVRTAEELGMQ; QNVRTAEELGMQP; NVRTAEELGMQPA;
VRTAEELGMQPAK; RTAEELGMQPAKI; TAEELGMQPAKIN;
AEELGMQPAKINT; EELGMQPAKINTP; ELGMQPAKINTPA;
LGMQPAKINTPAS; GMQPAKINTPASL; MQPAKINTPASLS;
QPAKINTPASLSG; PAKINTPASLSGS; AKINTPASLSGSQ;
KINTPASLSGSQA; INTPASLSGSQAS; NTPASLSGSQASW;
TPASLSGSQASWT; PASLSGSQASWTL; ASLSGSQASWTLR;
SLSGSQASWTLRV; LSGSQASWTLRVH; SGSQASWTLRVHV;
GSQASWTLRVHVG; SQASWTLRVHVGA; QASWTLRVHVGAN;
ASWTLRVHVGANQ; SWTLRVHVGANQD; WTLRVHVGANQDE;
TLRVHVGANQDEA; LRVHVGANQDEAI; RVHVGANQDEAIA;
VHVGANQDEAIAV; HVGANQDEAIAVN; VGANQDEAIAVNI;
GANQDEAIAVNIY; ANQDEAIAVNIYA; NQDEAIAVNIYAA;
QDEAIAVNIYAAN; DEAIAVNIYAANV; EAIAVNIYAANVA;
AIAVNIYAANVAN; IAVNIYAANVANL; AVNIYAANVANLF;
VNIYAANVANLFS; NIYAANVANLFSG; IYAANVANLFSGE;
YAANVANLFSGEG; AANVANLFSGEGA; ANVANLFSGEGAQ;
NVANLFSGEGAQT; VANLFSGEGAQTA; ANLFSGEGAQTAQ;
NLFSGEGAQTAQA; LFSGEGAQTAQAA; FSGEGAQTAQAAP;
SGEGAQTAQAAPV; GEGAQTAQAAPVQ; EGAQTAQAAPVQE;
GAQTAQAAPVQEG; AQTAQAAPVQEGV; QTAQAAPVQEGVQ;
TAQAAPVQEGVQQ; AQAAPVQEGVQQE; QAAPVQEGVQQEG;
AAPVQEGVQQEGA; APVQEGVQQEGAQ; PVQEGVQQEGAQQ;
VQEGVQQEGAQQP; QEGVQQEGAQQPA; EGVQQEGAQQPAP;
GVQQEGAQQPAPA; VQQEGAQQPAPAT; QQEGAQQPAPATA;
QEGAQQPAPATAP; EGAQQPAPATAPS; GAQQPAPATAPSQ;
AQQPAPATAPSQG; QQPAPATAPSQGG; QPAPATAPSQGGV;
PAPATAPSQGGVN; APATAPSQGGVNS; PATAPSQGGVNSP;
ATAPSQGGVNSPV; TAPSQGGVNSPVN; APSQGGVNSPVNV;
PSQGGVNSPVNVT; SQGGVNSPVNVTT; QGGVNSPVNVTTT;
GGVNSPVNVTTTV; GVNSPVNVTTTVD; VNSPVNVTTTVDA;
NSPVNVTTTVDAN; SPVNVTTTVDANT; PVNVTTTVDANTS;
VNVTTTVDANTSL; NVTTTVDANTSLA; VTTTVDANTSLAK;
TTTVDANTSLAKI; TTVDANTSLAKIE; TVDANTSLAKIEN;
VDANTSLAKIENA; DANTSLAKIENAI; ANTSLAKIENAIR;
NTSLAKIENAIRM; TSLAKIENAIRMI; SLAKIENAIRMIS;
LAKIENAIRMISD; AKIENAIRMISDQ; KIENAIRMISDQR;
IENAIRMISDQRA; ENAIRMISDQRAN; NAIRMISDQRANL;
AIRMISDQRANLG; IRMISDQRANLGA; RMISDQRANLGAF;
MISDQRANLGAFQ; ISDQRANLGAFQN; SDQRANLGAFQNR;
DQRANLGAFQNRL; QRANLGAFQNRLE; RANLGAFQNRLES;
ANLGAFQNRLESI; NLGAFQNRLESIK; LGAFQNRLESIKN;
GAFQNRLESIKNS; AFQNRLESIKNST; FQNRLESIKNSTE;
QNRLESIKNSTEY; NRLESIKNSTEYA; RLESIKNSTEYAI;
LESIKNSTEYAIE; ESIKNSTEYAIEN; SIKNSTEYAIENL;
IKNSTEYAIENLK; KNSTEYAIENLKA; NSTEYAIENLKAS;
STEYAIENLKASY; TEYAIENLKASYA; EYAIENLKASYAQ;
YAIENLKASYAQI; AIENLKASYAQIK; IENLKASYAQIKD;
ENLKASYAQIKDA; NLKASYAQIKDAT; LKASYAQIKDATM;
KASYAQIKDATMT; ASYAQIKDATMTD; SYAQIKDATMTDE;
YAQIKDATMTDEV; AQIKDATMTDEVV; QIKDATMTDEVVA;

IKDATMTDEVVAA; KDATMTDEVVAAT; DATMTDEVVAATT;
ATMTDEVVAATTN; TMTDEVVAATTNS; MTDEVVAATTNSI;
TDEVVAATTNSIL; DEVVAATTNSILT; EVVAATTNSILTQ;
VVAATTNSILTQS; VAATTNSILTQSA; AATTNSILTQSAM;
ATTNSILTQSAMA; TTNSILTQSAMAM; TNSILTQSAMAMI;
NSILTQSAMAMIA; SILTQSAMAMIAQ; ILTQSAMAMIAQA;
LTQSAMAMIAQAN; TQSAMAMIAQANQ; QSAMAMIAQANQV;
SAMAMIAQANQVP; AMAMIAQANQVPQ; MAMIAQANQVPQY;
AMIAQANQVPQYV; MIAQANQVPQYVL; IAQANQVPQYVLS;
AQANQVPQYVLSL; QANQVPQYVLSLL; ANQVPQYVLSLLR;

14 mers:
MIINHNTSAINASR; IINHNTSAINASRN; INHNTSAINASRNN;
NHNTSAINASRNNG; HNTSAINASRNNGI; NTSAINASRNNGIN;
TSAINASRNNGINA; SAINASRNNGINAA; AINASRNNGINAAN;
INASRNNGINAANL; NASRNNGINAANLS; ASRNNGINAANLSK;
SRNNGINAANLSKT; RNNGINAANLSKTQ; NNGINAANLSKTQE;
NGINAANLSKTQEK; GINAANLSKTQEKL; INAANLSKTQEKLS;
NAANLSKTQEKLSS; AANLSKTQEKLSSG; ANLSKTQEKLSSGY;
NLSKTQEKLSSGYR; LSKTQEKLSSGYRI; SKTQEKLSSGYRIN;
KTQEKLSSGYRINR; TQEKLSSGYRINRA; QEKLSSGYRINRAS;
EKLSSGYRINRASD; KLSSGYRINRASDD; LSSGYRINRASDDA;
SSGYRINRASDDAA; SGYRINRASDDAAG; GYRINRASDDAAGM;
YRINRASDDAAGMG; RINRASDDAAGMGV; INRASDDAAGMGVS;
NRASDDAAGMGVSG; RASDDAAGMGVSGK; ASDDAAGMGVSGKI;
SDDAAGMGVSGKIN; DDAAGMGVSGKINA; DAAGMGVSGKINAQ;
AAGMGVSGKINAQI; AGMGVSGKINAQIR; GMGVSGKINAQIRG;
MGVSGKINAQIRGL; GVSGKINAQIRGLS; VSGKINAQIRGLSQ;
SGKINAQIRGLSQA; GKINAQIRGLSQAS; KINAQIRGLSQASR;
INAQIRGLSQASRN; NAQIRGLSQASRNT; AQIRGLSQASRNTS;
QIRGLSQASRNTSK; IRGLSQASRNTSKA; RGLSQASRNTSKAI;
GLSQASRNTSKAIN; LSQASRNTSKAINF; SQASRNTSKAINFI;
QASRNTSKAINFIQ; ASRNTSKAINFIQT; SRNTSKAINFIQTT;
RNTSKAINFIQTTE; NTSKAINFIQTTEG; TSKAINFIQTTEGN;
SKAINFIQTTEGNL; KAINFIQTTEGNLN; AINFIQTTEGNLNE;
INFIQTTEGNLNEV; NFIQTTEGNLNEVE; FIQTTEGNLNEVEK;
IQTTEGNLNEVEKV; QTTEGNLNEVEKVL; TTEGNLNEVEKVLV;
TEGNLNEVEKVLVR; EGNLNEVEKVLVRM; GNLNEVEKVLVRMK;
NLNEVEKVLVRMKE; LNEVEKVLVRMKEL; NEVEKVLVRMKELA;
EVEKVLVRMKELAV; VEKVLVRMKELAVQ; EKVLVRMKELAVQS;
KVLVRMKELAVQSG; VLVRMKELAVQSGN; LVRMKELAVQSGNG;
VRMKELAVQSGNGT; RMKELAVQSGNGTY; MKELAVQSGNGTYS;
KELAVQSGNGTYSD; ELAVQSGNGTYSDA; LAVQSGNGTYSDAD;
AVQSGNGTYSDADR; VQSGNGTYSDADRG; QSGNGTYSDADRGS;
SGNGTYSDADRGSI; GNGTYSDADRGSIQ; NGTYSDADRGSIQI;
GTYSDADRGSIQIE; TYSDADRGSIQIEI; YSDADRGSIQIEIE;
SDADRGSIQIEIEQ; DADRGSIQIEIEQL; ADRGSIQIEIEQLT;
DRGSIQIEIEQLTD; RGSIQIEIEQLTDE; GSIQIEIEQLTDEI;
SIQIEIEQLTDEIN; IQIEIEQLTDEINR; QIEIEQLTDEINRI;
IEIEQLTDEINRIA; EIEQLTDEINRIAD; IEQLTDEINRIADQ;
EQLTDEINRIADQA; QLTDEINRIADQAQ; LTDEINRIADQAQY;

| | | |
|---|---|---|
| TDEINRIADQAQYN; | DEINRIADQAQYNQ; | EINRIADQAQYNQM; |
| INRIADQAQYNQMH; | NRIADQAQYNQMHM; | RIADQAQYNQMHML; |
| IADQAQYNQMHMLS; | ADQAQYNQMHMLSN; | DQAQYNQMHMLSNK; |
| QAQYNQMHMLSNKS; | AQYNQMHMLSNKSA; | QYNQMHMLSNKSAS; |
| YNQMHMLSNKSASQ; | NQMHMLSNKSASQN; | QMHMLSNKSASQNV; |
| MHMLSNKSASQNVR; | HMLSNKSASQNVRT; | MLSNKSASQNVRTA; |
| LSNKSASQNVRTAE; | SNKSASQNVRTAEE; | NKSASQNVRTAEEL; |
| KSASQNVRTAEELG; | SASQNVRTAEELGM; | ASQNVRTAEELGMQ; |
| SQNVRTAEELGMQP; | QNVRTAEELGMQPA; | NVRTAEELGMQPAK; |
| VRTAEELGMQPAKI; | RTAEELGMQPAKIN; | TAEELGMQPAKINT; |
| AEELGMQPAKINTP; | EELGMQPAKINTPA; | ELGMQPAKINTPAS; |
| LGMQPAKINTPASL; | GMQPAKINTPASLS; | MQPAKINTPASLSG; |
| QPAKINTPASLSGS; | PAKINTPASLSGSQ; | AKINTPASLSGSQA; |
| KINTPASLSGSQAS; | INTPASLSGSQASW; | NTPASLSGSQASWT; |
| TPASLSGSQASWTL; | PASLSGSQASWTLR; | ASLSGSQASWTLRV; |
| SLSGSQASWTLRVH; | LSGSQASWTLRVHV; | SGSQASWTLRVHVG; |
| GSQASWTLRVHVGA; | SQASWTLRVHVGAN; | QASWTLRVHVGANQ; |
| ASWTLRVHVGANQD; | SWTLRVHVGANQDE; | WTLRVHVGANQDEA; |
| TLRVHVGANQDEAI; | LRVHVGANQDEAIA; | RVHVGANQDEAIAV; |
| VHVGANQDEAIAVN; | HVGANQDEAIAVNI; | VGANQDEAIAVNIY; |
| GANQDEAIAVNIYA; | ANQDEAIAVNIYAA; | NQDEAIAVNIYAAN; |
| QDEAIAVNIYAANV; | DEAIAVNIYAANVA; | EAIAVNIYAANVAN; |
| AIAVNIYAANVANL; | IAVNIYAANVANLF; | AVNIYAANVANLFS; |
| VNIYAANVANLFSG; | NIYAANVANLFSGE; | IYAANVANLFSGEG; |
| YAANVANLFSGEGA; | AANVANLFSGEGAQ; | ANVANLFSGEGAQT; |
| NVANLFSGEGAQTA; | VANLFSGEGAQTAQ; | ANLFSGEGAQTAQA; |
| NLFSGEGAQTAQAA; | LFSGEGAQTAQAAP; | FSGEGAQTAQAAPV; |
| SGEGAQTAQAAPVQ; | GEGAQTAQAAPVQE; | EGAQTAQAAPVQEG; |
| GAQTAQAAPVQEGV; | AQTAQAAPVQEGVQ; | QTAQAAPVQEGVQQ; |
| TAQAAPVQEGVQQE; | AQAAPVQEGVQQEG; | QAAPVQEGVQQEGA; |
| AAPVQEGVQQEGAQ; | APVQEGVQQEGAQQ; | PVQEGVQQEGAQQP; |
| VQEGVQQEGAQQPA; | QEGVQQEGAQQPAP; | EGVQQEGAQQPAPA; |
| GVQQEGAQQPAPAT; | VQQEGAQQPAPATA; | QQEGAQQPAPATAP; |
| QEGAQQPAPATAPS; | EGAQQPAPATAPSQ; | GAQQPAPATAPSQG; |
| AQQPAPATAPSQGG; | QQPAPATAPSQGGV; | QPAPATAPSQGGVN; |
| PAPATAPSQGGVNS; | APATAPSQGGVNSP; | PATAPSQGGVNSPV; |
| ATAPSQGGVNSPVN; | TAPSQGGVNSPVNV; | APSQGGVNSPVNVT; |
| PSQGGVNSPVNVTT; | SQGGVNSPVNVTTT; | QGGVNSPVNVTTTV; |
| GGVNSPVNVTTTVD; | GVNSPVNVTTTVDA; | VNSPVNVTTTVDAN; |
| NSPVNVTTTVDANT; | SPVNVTTTVDANTS; | PVNVTTTVDANTSL; |
| VNVTTTVDANTSLA; | NVTTTVDANTSLAK; | VTTTVDANTSLAKI; |
| TTTVDANTSLAKIE; | TTVDANTSLAKIEN; | TVDANTSLAKIENA; |
| VDANTSLAKIENAI; | DANTSLAKIENAIR; | ANTSLAKIENAIRM; |
| NTSLAKIENAIRMI; | TSLAKIENAIRMIS; | SLAKIENAIRMISD; |
| LAKIENAIRMISDQ; | AKIENAIRMISDQR; | KIENAIRMISDQRA; |
| IENAIRMISDQRAN; | ENAIRMISDQRANL; | NAIRMISDQRANLG; |
| AIRMISDQRANLGA; | IRMISDQRANLGAF; | RMISDQRANLGAFQ; |
| MISDQRANLGAFQN; | ISDQRANLGAFQNR; | SDQRANLGAFQNRL; |
| DQRANLGAFQNRLE; | QRANLGAFQNRLES; | RANLGAFQNRLESI; |
| ANLGAFQNRLESIK; | NLGAFQNRLESIKN; | LGAFQNRLESIKNS; |
| GAFQNRLESIKNST; | AFQNRLESIKNSTE; | FQNRLESIKNSTEY; |

QNRLESIKNSTEYA; NRLESIKNSTEYAI; RLESIKNSTEYAIE;
LESIKNSTEYAIEN; ESIKNSTEYAIENL; SIKNSTEYAIENLK;
IKNSTEYAIENLKA; KNSTEYAIENLKAS; NSTEYAIENLKASY;
STEYAIENLKASYA; TEYAIENLKASYAQ; EYAIENLKASYAQI;
YAIENLKASYAQIK; AIENLKASYAQIKD; IENLKASYAQIKDA;
ENLKASYAQIKDAT; NLKASYAQIKDATM; LKASYAQIKDATMT;
KASYAQIKDATMTD; ASYAQIKDATMTDE; SYAQIKDATMTDEV;
YAQIKDATMTDEVV; AQIKDATMTDEVVA; QIKDATMTDEVVAA;
IKDATMTDEVVAAT; KDATMTDEVVAATT; DATMTDEVVAATTN;
ATMTDEVVAATTNS; TMTDEVVAATTNSI; MTDEVVAATTNSIL;
TDEVVAATTNSILT; DEVVAATTNSILTQ; EVVAATTNSILTQS;
VVAATTNSILTQSA; VAATTNSILTQSAM; AATTNSILTQSAMA;
ATTNSILTQSAMAM; TTNSILTQSAMAMI; TNSILTQSAMAMIA;
NSILTQSAMAMIAQ; SILTQSAMAMIAQA; ILTQSAMAMIAQAN;
LTQSAMAMIAQANQ; TQSAMAMIAQANQV; QSAMAMIAQANQVP;
SAMAMIAQANQVPQ; AMAMIAQANQVPQY; MAMIAQANQVPQYV;
AMIAQANQVPQYVL; MIAQANQVPQYVLS; IAQANQVPQYVLSL;
AQANQVPQYVLSLL; QANQVPQYVLSLLR;

15 mers:
MIINHNTSAINASRN; IINHNTSAINASRNN; INHNTSAINASRNNG;
NHNTSAINASRNNGI; HNTSAINASRNNGIN; NTSAINASRNNGINA;
TSAINASRNNGINAA; SAINASRNNGINAAN; AINASRNNGINAANL;
INASRNNGINAANLS; NASRNNGINAANLSK; ASRNNGINAANLSKT;
SRNNGINAANLSKTQ; RNNGINAANLSKTQE; NNGINAANLSKTQEK;
NGINAANLSKTQEKL; GINAANLSKTQEKLS; INAANLSKTQEKLSS;
NAANLSKTQEKLSSG; AANLSKTQEKLSSGY; ANLSKTQEKLSSGYR;
NLSKTQEKLSSGYRI; LSKTQEKLSSGYRIN; SKTQEKLSSGYRINR;
KTQEKLSSGYRINRA; TQEKLSSGYRINRAS; QEKLSSGYRINRASD;
EKLSSGYRINRASDD; KLSSGYRINRASDDA; LSSGYRINRASDDAA;
SSGYRINRASDDAAG; SGYRINRASDDAAGM; GYRINRASDDAAGMG;
YRINRASDDAAGMGV; RINRASDDAAGMGVS; INRASDDAAGMGVSG;
NRASDDAAGMGVSGK; RASDDAAGMGVSGKI; ASDDAAGMGVSGKIN;
SDDAAGMGVSGKINA; DDAAGMGVSGKINAQ; DAAGMGVSGKINAQI;
AAGMGVSGKINAQIR; AGMGVSGKINAQIRG; GMGVSGKINAQIRGL;
MGVSGKINAQIRGLS; GVSGKINAQIRGLSQ; VSGKINAQIRGLSQA;
SGKINAQIRGLSQAS; GKINAQIRGLSQASR; KINAQIRGLSQASRN;
INAQIRGLSQASRNT; NAQIRGLSQASRNTS; AQIRGLSQASRNTSK;
QIRGLSQASRNTSKA; IRGLSQASRNTSKAI; RGLSQASRNTSKAIN;
GLSQASRNTSKAINF; LSQASRNTSKAINFI; SQASRNTSKAINFIQ;
QASRNTSKAINFIQT; ASRNTSKAINFIQTT; SRNTSKAINFIQTTE;
RNTSKAINFIQTTEG; NTSKAINFIQTTEGN; TSKAINFIQTTEGNL;
SKAINFIQTTEGNLN; KAINFIQTTEGNLNE; AINFIQTTEGNLNEV;
INFIQTTEGNLNEVE; NFIQTTEGNLNEVEK; FIQTTEGNLNEVEKV;
IQTTEGNLNEVEKVL; QTTEGNLNEVEKVLV; TTEGNLNEVEKVLVR;
TEGNLNEVEKVLVRM; EGNLNEVEKVLVRMK; GNLNEVEKVLVRMKE;
NLNEVEKVLVRMKEL; LNEVEKVLVRMKELA; NEVEKVLVRMKELAV;
EVEKVLVRMKELAVQ; VEKVLVRMKELAVQS; EKVLVRMKELAVQSG;
KVLVRMKELAVQSGN; VLVRMKELAVQSGNG; LVRMKELAVQSGNGT;
VRMKELAVQSGNGTY; RMKELAVQSGNGTYS; MKELAVQSGNGTYSD;
KELAVQSGNGTYSDA; ELAVQSGNGTYSDAD; LAVQSGNGTYSDADR;

AVQSGNGTYSDADRG; VQSGNGTYSDADRGS; QSGNGTYSDADRGSI;
SGNGTYSDADRGSIQ; GNGTYSDADRGSIQI; NGTYSDADRGSIQIE;
GTYSDADRGSIQIEI; TYSDADRGSIQIEIE; YSDADRGSIQIEIEQ;
SDADRGSIQIEIEQL; DADRGSIQIEIEQLT; ADRGSIQIEIEQLTD;
DRGSIQIEIEQLTDE; RGSIQIEIEQLTDEI; GSIQIEIEQLTDEIN;
SIQIEIEQLTDEINR; IQIEIEQLTDEINRI; QIEIEQLTDEINRIA;
IEIEQLTDEINRIAD; EIEQLTDEINRIADQ; IEQLTDEINRIADQA;
EQLTDEINRIADQAQ; QLTDEINRIADQAQY; LTDEINRIADQAQYN;
TDEINRIADQAQYNQ; DEINRIADQAQYNQM; EINRIADQAQYNQMH;
INRIADQAQYNQMHM; NRIADQAQYNQMHML; RIADQAQYNQMHMLS;
IADQAQYNQMHMLSN; ADQAQYNQMHMLSNK; DQAQYNQMHMLSNKS;
QAQYNQMHMLSNKSA; AQYNQMHMLSNKSAS; QYNQMHMLSNKSASQ;
YNQMHMLSNKSASQN; NQMHMLSNKSASQNV; QMHMLSNKSASQNVR;
MHMLSNKSASQNVRT; HMLSNKSASQNVRTA; MLSNKSASQNVRTAE;
LSNKSASQNVRTAEE; SNKSASQNVRTAEEL; NKSASQNVRTAEELG;
KSASQNVRTAEELGM; SASQNVRTAEELGMQ; ASQNVRTAEELGMQP;
SQNVRTAEELGMQPA; QNVRTAEELGMQPAK; NVRTAEELGMQPAKI;
VRTAEELGMQPAKIN; RTAEELGMQPAKINT; TAEELGMQPAKINTP;
AEELGMQPAKINTPA; EELGMQPAKINTPAS; ELGMQPAKINTPASL;
LGMQPAKINTPASLS; GMQPAKINTPASLSG; MQPAKINTPASLSGS;
QPAKINTPASLSGSQ; PAKINTPASLSGSQA; AKINTPASLSGSQAS;
KINTPASLSGSQASW; INTPASLSGSQASWT; NTPASLSGSQASWTL;
TPASLSGSQASWTLR; PASLSGSQASWTLRV; ASLSGSQASWTLRVH;
SLSGSQASWTLRVHV; LSGSQASWTLRVHVG; SGSQASWTLRVHVGA;
GSQASWTLRVHVGAN; SQASWTLRVHVGANQ; QASWTLRVHVGANQD;
ASWTLRVHVGANQDE; SWTLRVHVGANQDEA; WTLRVHVGANQDEAI;
TLRVHVGANQDEAIA; LRVHVGANQDEAIAV; RVHVGANQDEAIAVN;
VHVGANQDEAIAVNI; HVGANQDEAIAVNIY; VGANQDEAIAVNIYA;
GANQDEAIAVNIYAA; ANQDEAIAVNIYAAN; NQDEAIAVNIYAANV;
QDEAIAVNIYAANVA; DEAIAVNIYAANVAN; EAIAVNIYAANVANL;
AIAVNIYAANVANLF; IAVNIYAANVANLFS; AVNIYAANVANLFSG;
VNIYAANVANLFSGE; NIYAANVANLFSGEG; IYAANVANLFSGEGA;
YAANVANLFSGEGAQ; AANVANLFSGEGAQT; ANVANLFSGEGAQTA;
NVANLFSGEGAQTAQ; VANLFSGEGAQTAQA; ANLFSGEGAQTAQAA;
NLFSGEGAQTAQAAP; LFSGEGAQTAQAAPV; FSGEGAQTAQAAPVQ;
SGEGAQTAQAAPVQE; GEGAQTAQAAPVQEG; EGAQTAQAAPVQEGV;
GAQTAQAAPVQEGVQ; AQTAQAAPVQEGVQQ; QTAQAAPVQEGVQQE;
TAQAAPVQEGVQQEG; AQAAPVQEGVQQEGA; QAAPVQEGVQQEGAQ;
AAPVQEGVQQEGAQQ; APVQEGVQQEGAQQP; PVQEGVQQEGAQQPA;
VQEGVQQEGAQQPAP; QEGVQQEGAQQPAPA; EGVQQEGAQQPAPAT;
GVQQEGAQQPAPATA; VQQEGAQQPAPATAP; QQEGAQQPAPATAPS;
QEGAQQPAPATAPSQ; EGAQQPAPATAPSQG; GAQQPAPATAPSQGG;
AQQPAPATAPSQGGV; QQPAPATAPSQGGVN; QPAPATAPSQGGVNS;
PAPATAPSQGGVNSP; APATAPSQGGVNSPV; PATAPSQGGVNSPVN;
ATAPSQGGVNSPVNV; TAPSQGGVNSPVNVT; APSQGGVNSPVNVTT;
PSQGGVNSPVNVTTT; SQGGVNSPVNVTTTV; QGGVNSPVNVTTTVD;
GGVNSPVNVTTTVDA; GVNSPVNVTTTVDAN; VNSPVNVTTTVDANT;
NSPVNVTTTVDANTS; SPVNVTTTVDANTSL; PVNVTTTVDANTSLA;
VNVTTTVDANTSLAK; NVTTTVDANTSLAKI; VTTTVDANTSLAKIE;
TTTVDANTSLAKIEN; TTVDANTSLAKIENA; TVDANTSLAKIENAI;
VDANTSLAKIENAIR; DANTSLAKIENAIRM; ANTSLAKIENAIRMI;

NTSLAKIENAIRMIS; TSLAKIENAIRMISD; SLAKIENAIRMISDQ;
LAKIENAIRMISDQR; AKIENAIRMISDQRA; KIENAIRMISDQRAN;
IENAIRMISDQRANL; ENAIRMISDQRANLG; NAIRMISDQRANLGA;
AIRMISDQRANLGAF; IRMISDQRANLGAFQ; RMISDQRANLGAFQN;
MISDQRANLGAFQNR; ISDQRANLGAFQNRL; SDQRANLGAFQNRLE;
DQRANLGAFQNRLES; QRANLGAFQNRLESI; RANLGAFQNRLESIK;
ANLGAFQNRLESIKN; NLGAFQNRLESIKNS; LGAFQNRLESIKNST;
GAFQNRLESIKNSTE; AFQNRLESIKNSTEY; FQNRLESIKNSTEYA;
QNRLESIKNSTEYAI; NRLESIKNSTEYAIE; RLESIKNSTEYAIEN;
LESIKNSTEYAIENL; ESIKNSTEYAIENLK; SIKNSTEYAIENLKA;
IKNSTEYAIENLKAS; KNSTEYAIENLKASY; NSTEYAIENLKASYA;
STEYAIENLKASYAQ; TEYAIENLKASYAQI; EYAIENLKASYAQIK;
YAIENLKASYAQIKD; AIENLKASYAQIKDA; IENLKASYAQIKDAT;
ENLKASYAQIKDATM; NLKASYAQIKDATMT; LKASYAQIKDATMTD;
KASYAQIKDATMTDE; ASYAQIKDATMTDEV; SYAQIKDATMTDEVV;
YAQIKDATMTDEVVA; AQIKDATMTDEVVAA; QIKDATMTDEVVAAT;
IKDATMTDEVVAATT; KDATMTDEVVAATTN; DATMTDEVVAATTNS;
ATMTDEVVAATTNSI; TMTDEVVAATTNSIL; MTDEVVAATTNSILT;
TDEVVAATTNSILTQ; DEVVAATTNSILTQS; EVVAATTNSILTQSA;
VVAATTNSILTQSAM; VAATTNSILTQSAMA; AATTNSILTQSAMAM;
ATTNSILTQSAMAMI; TTNSILTQSAMAMIA; TNSILTQSAMAMIAQ;
NSILTQSAMAMIAQA; SILTQSAMAMIAQAN; ILTQSAMAMIAQANQ;
LTQSAMAMIAQANQV; TQSAMAMIAQANQVP; QSAMAMIAQANQVPQ;
SAMAMIAQANQVPQY; AMAMIAQANQVPQYV; MAMIAQANQVPQYVL;
AMIAQANQVPQYVLS; MIAQANQVPQYVLSL; IAQANQVPQYVLSLL;
AQANQVPQYVLSLLR;

16 mers:
MIINHNTSAINASRNN; IINHNTSAINASRNNG; INHNTSAINASRNNGI;
NHNTSAINASRNNGIN; HNTSAINASRNNGINA; NTSAINASRNNGINAA;
TSAINASRNNGINAAN; SAINASRNNGINAANL; AINASRNNGINAANLS;
INASRNNGINAANLSK; NASRNNGINAANLSKT; ASRNNGINAANLSKTQ;
SRNNGINAANLSKTQE; RNNGINAANLSKTQEK; NNGINAANLSKTQEKL;
NGINAANLSKTQEKLS; GINAANLSKTQEKLSS; INAANLSKTQEKLSSG;
NAANLSKTQEKLSSGY; AANLSKTQEKLSSGYR; ANLSKTQEKLSSGYRI;
NLSKTQEKLSSGYRIN; LSKTQEKLSSGYRINR; SKTQEKLSSGYRINRA;
KTQEKLSSGYRINRAS; TQEKLSSGYRINRASD; QEKLSSGYRINRASDD;
EKLSSGYRINRASDDA; KLSSGYRINRASDDAA; LSSGYRINRASDDAAG;
SSGYRINRASDDAAGM; SGYRINRASDDAAGMG; GYRINRASDDAAGMGV;
YRINRASDDAAGMGVS; RINRASDDAAGMGVSG; INRASDDAAGMGVSGK;
NRASDDAAGMGVSGKI; RASDDAAGMGVSGKIN; ASDDAAGMGVSGKINA;
SDDAAGMGVSGKINAQ; DDAAGMGVSGKINAQI; DAAGMGVSGKINAQIR;
AAGMGVSGKINAQIRG; AGMGVSGKINAQIRGL; GMGVSGKINAQIRGLS;
MGVSGKINAQIRGLSQ; GVSGKINAQIRGLSQA; VSGKINAQIRGLSQAS;
SGKINAQIRGLSQASR; GKINAQIRGLSQASRN; KINAQIRGLSQASRNT;
INAQIRGLSQASRNTS; NAQIRGLSQASRNTSK; AQIRGLSQASRNTSKA;
QIRGLSQASRNTSKAI; IRGLSQASRNTSKAIN; RGLSQASRNTSKAINF;
GLSQASRNTSKAINFI; LSQASRNTSKAINFIQ; SQASRNTSKAINFIQT;
QASRNTSKAINFIQTT; ASRNTSKAINFIQTTE; SRNTSKAINFIQTTEG;
RNTSKAINFIQTTEGN; NTSKAINFIQTTEGNL; TSKAINFIQTTEGNLN;
SKAINFIQTTEGNLNE; KAINFIQTTEGNLNEV; AINFIQTTEGNLNEVE;

| | | |
|---|---|---|
| INFIQTTEGNLNEVEK; | NFIQTTEGNLNEVEKV; | FIQTTEGNLNEVEKVL; |
| IQTTEGNLNEVEKVLV; | QTTEGNLNEVEKVLVR; | TTEGNLNEVEKVLVRM; |
| TEGNLNEVEKVLVRMK; | EGNLNEVEKVLVRMKE; | GNLNEVEKVLVRMKEL; |
| NLNEVEKVLVRMKELA; | LNEVEKVLVRMKELAV; | NEVEKVLVRMKELAVQ; |
| EVEKVLVRMKELAVQS; | VEKVLVRMKELAVQSG; | EKVLVRMKELAVQSGN; |
| KVLVRMKELAVQSGNG; | VLVRMKELAVQSGNGT; | LVRMKELAVQSGNGTY; |
| VRMKELAVQSGNGTYS; | RMKELAVQSGNGTYSD; | MKELAVQSGNGTYSDA; |
| KELAVQSGNGTYSDAD; | ELAVQSGNGTYSDADR; | LAVQSGNGTYSDADRG; |
| AVQSGNGTYSDADRGS; | VQSGNGTYSDADRGSI; | QSGNGTYSDADRGSIQ; |
| SGNGTYSDADRGSIQI; | GNGTYSDADRGSIQIE; | NGTYSDADRGSIQIEI; |
| GTYSDADRGSIQIEIE; | TYSDADRGSIQIEIEQ; | YSDADRGSIQIEIEQL; |
| SDADRGSIQIEIEQLT; | DADRGSIQIEIEQLTD; | ADRGSIQIEIEQLTDE; |
| DRGSIQIEIEQLTDEI; | RGSIQIEIEQLTDEIN; | GSIQIEIEQLTDEINR; |
| SIQIEIEQLTDEINRI; | IQIEIEQLTDEINRIA; | QIEIEQLTDEINRIAD; |
| IEIEQLTDEINRIADQ; | EIEQLTDEINRIADQA; | IEQLTDEINRIADQAQ; |
| EQLTDEINRIADQAQY; | QLTDEINRIADQAQYN; | LTDEINRIADQAQYNQ; |
| TDEINRIADQAQYNQM; | DEINRIADQAQYNQMH; | EINRIADQAQYNQMHM; |
| INRIADQAQYNQMHML; | NRIADQAQYNQMHMLS; | RIADQAQYNQMHMLSN; |
| IADQAQYNQMHMLSNK; | ADQAQYNQMHMLSNKS; | DQAQYNQMHMLSNKSA; |
| QAQYNQMHMLSNKSAS; | AQYNQMHMLSNKSASQ; | QYNQMHMLSNKSASQN; |
| YNQMHMLSNKSASQNV; | NQMHMLSNKSASQNVR; | QMHMLSNKSASQNVRT; |
| MHMLSNKSASQNVRTA; | HMLSNKSASQNVRTAE; | MLSNKSASQNVRTAEE; |
| LSNKSASQNVRTAEEL; | SNKSASQNVRTAEELG; | NKSASQNVRTAEELGM; |
| KSASQNVRTAEELGMQ; | SASQNVRTAEELGMQP; | ASQNVRTAEELGMQPA; |
| SQNVRTAEELGMQPAK; | QNVRTAEELGMQPAKI; | NVRTAEELGMQPAKIN; |
| VRTAEELGMQPAKINT; | RTAEELGMQPAKINTP; | TAEELGMQPAKINTPA; |
| AEELGMQPAKINTPAS; | EELGMQPAKINTPASL; | ELGMQPAKINTPASLS; |
| LGMQPAKINTPASLSG; | GMQPAKINTPASLSGS; | MQPAKINTPASLSGSQ; |
| QPAKINTPASLSGSQA; | PAKINTPASLSGSQAS; | AKINTPASLSGSQASW; |
| KINTPASLSGSQASWT; | INTPASLSGSQASWTL; | NTPASLSGSQASWTLR; |
| TPASLSGSQASWTLRV; | PASLSGSQASWTLRVH; | ASLSGSQASWTLRVHV; |
| SLSGSQASWTLRVHVG; | LSGSQASWTLRVHVGA; | SGSQASWTLRVHVGAN; |
| GSQASWTLRVHVGANQ; | SQASWTLRVHVGANQD; | QASWTLRVHVGANQDE; |
| ASWTLRVHVGANQDEA; | SWTLRVHVGANQDEAI; | WTLRVHVGANQDEAIA; |
| TLRVHVGANQDEAIAV; | LRVHVGANQDEAIAVN; | RVHVGANQDEAIAVNI; |
| VHVGANQDEAIAVNIY; | HVGANQDEAIAVNIYA; | VGANQDEAIAVNIYAA; |
| GANQDEAIAVNIYAAN; | ANQDEAIAVNIYAANV; | NQDEAIAVNIYAANVA; |
| QDEAIAVNIYAANVAN; | DEAIAVNIYAANVANL; | EAIAVNIYAANVANLF; |
| AIAVNIYAANVANLFS; | IAVNIYAANVANLFSG; | AVNIYAANVANLFSGE; |
| VNIYAANVANLFSGEG; | NIYAANVANLFSGEGA; | IYAANVANLFSGEGAQ; |
| YAANVANLFSGEGAQT; | AANVANLFSGEGAQTA; | ANVANLFSGEGAQTAQ; |
| NVANLFSGEGAQTAQA; | VANLFSGEGAQTAQAA; | ANLFSGEGAQTAQAAP; |
| NLFSGEGAQTAQAAPV; | LFSGEGAQTAQAAPVQ; | FSGEGAQTAQAAPVQE; |
| SGEGAQTAQAAPVQEG; | GEGAQTAQAAPVQEGV; | EGAQTAQAAPVQEGVQ; |
| GAQTAQAAPVQEGVQQ; | AQTAQAAPVQEGVQQE; | QTAQAAPVQEGVQQEG; |
| TAQAAPVQEGVQQEGA; | AQAAPVQEGVQQEGAQ; | QAAPVQEGVQQEGAQQ; |
| AAPVQEGVQQEGAQQP; | APVQEGVQQEGAQQPA; | PVQEGVQQEGAQQPAP; |
| VQEGVQQEGAQQPAPA; | QEGVQQEGAQQPAPAT; | EGVQQEGAQQPAPATA; |
| GVQQEGAQQPAPATAP; | VQQEGAQQPAPATAPS; | QQEGAQQPAPATAPSQ; |
| QEGAQQPAPATAPSQG; | EGAQQPAPATAPSQGG; | GAQQPAPATAPSQGGV; |
| AQQPAPATAPSQGGVN; | QQPAPATAPSQGGVNS; | QPAPATAPSQGGVNSP; |

| | |
|---|---|
| | PAPATAPSQGGVNSPV; APATAPSQGGVNSPVN; PATAPSQGGVNSPVNV; ATAPSQGGVNSPVNVT; TAPSQGGVNSPVNVTT; APSQGGVNSPVNVTTT; PSQGGVNSPVNVTTTV; SQGGVNSPVNVTTTVD; QGGVNSPVNVTTTVDA; GGVNSPVNVTTTVDAN; GVNSPVNVTTTVDANT; VNSPVNVTTTVDANTS; NSPVNVTTTVDANTSL; SPVNVTTTVDANTSLA; PVNVTTTVDANTSLAK; VNVTTTVDANTSLAKI; NVTTTVDANTSLAKIE; VTTTVDANTSLAKIEN; TTTVDANTSLAKIENA; TTVDANTSLAKIENAI; TVDANTSLAKIENAIR; VDANTSLAKIENAIRM; DANTSLAKIENAIRMI; ANTSLAKIENAIRMIS; NTSLAKIENAIRMISD; TSLAKIENAIRMISDQ; SLAKIENAIRMISDQR; LAKIENAIRMISDQRA; AKIENAIRMISDQRAN; KIENAIRMISDQRANL; IENAIRMISDQRANLG; ENAIRMISDQRANLGA; NAIRMISDQRANLGAF; AIRMISDQRANLGAFQ; IRMISDQRANLGAFQN; RMISDQRANLGAFQNR; MISDQRANLGAFQNRL; ISDQRANLGAFQNRLE; SDQRANLGAFQNRLES; DQRANLGAFQNRLESI; QRANLGAFQNRLESIK; RANLGAFQNRLESIKN; ANLGAFQNRLESIKNS; NLGAFQNRLESIKNST; LGAFQNRLESIKNSTE; GAFQNRLESIKNSTEY; AFQNRLESIKNSTEYA; FQNRLESIKNSTEYAI; QNRLESIKNSTEYAIE; NRLESIKNSTEYAIEN; RLESIKNSTEYAIENL; LESIKNSTEYAIENLK; ESIKNSTEYAIENLKA; SIKNSTEYAIENLKAS; IKNSTEYAIENLKASY; KNSTEYAIENLKASYA; NSTEYAIENLKASYAQ; STEYAIENLKASYAQI; TEYAIENLKASYAQIK; EYAIENLKASYAQIKD; YAIENLKASYAQIKDA; AIENLKASYAQIKDAT; IENLKASYAQIKDATM; ENLKASYAQIKDATMT; NLKASYAQIKDATMTD; LKASYAQIKDATMTDE; KASYAQIKDATMTDEV; ASYAQIKDATMTDEVV; SYAQIKDATMTDEVVA; YAQIKDATMTDEVVAA; AQIKDATMTDEVVAAT; QIKDATMTDEVVAATT; IKDATMTDEVVAATTN; KDATMTDEVVAATTNS; DATMTDEVVAATTNSI; ATMTDEVVAATTNSIL; TMTDEVVAATTNSILT; MTDEVVAATTNSILTQ; TDEVVAATTNSILTQS; DEVVAATTNSILTQSA; EVVAATTNSILTQSAM; VVAATTNSILTQSAMA; VAATTNSILTQSAMAM; AATTNSILTQSAMAMI; ATTNSILTQSAMAMIA; TTNSILTQSAMAMIAQ; TNSILTQSAMAMIAQA; NSILTQSAMAMIAQAN; SILTQSAMAMIAQANQ; ILTQSAMAMIAQANQV; LTQSAMAMIAQANQVP; TQSAMAMIAQANQVPQ; QSAMAMIAQANQVPQY; SAMAMIAQANQVPQYV; AMAMIAQANQVPQYVL; MAMIAQANQVPQYVLS; AMIAQANQVPQYVLSL; MIAQANQVPQYVLSLL; IAQANQVPQYVLSLLR; |

| Seq 24 | | 13 mers: MKLQRSLFLIIFF; KLQRSLFLIIFFL; LQRSLFLIIFFLT; QRSLFLIIFFLTF; RSLFLIIFFLTFL; SLFLIIFFLTFLC; LFLIIFFLTFLCC; FLIIFFLTFLCCN; LIIFFLTFLCCNN; IIFFLTFLCCNNK; IFFLTFLCCNNKE; FFLTFLCCNNKER; FLTFLCCNNKERK; LTFLCCNNKERKE; TFLCCNNKERKEG; FLCCNNKERKEGV; LCCNNKERKEGVS; CCNNKERKEGVSF; CNNKERKEGVSFK; NNKERKEGVSFKI; NKERKEGVSFKIS; KERKEGVSFKISL; ERKEGVSFKISLG; RKEGVSFKISLGA; KEGVSFKISLGAE; EGVSFKISLGAEP; GVSFKISLGAEPS; VSFKISLGAEPSS; SFKISLGAEPSSL; FKISLGAEPSSLD; KISLGAEPSSLDP; ISLGAEPSSLDPQ; SLGAEPSSLDPQL; LGAEPSSLDPQLA; GAEPSSLDPQLAE; AEPSSLDPQLAED; EPSSLDPQLAEDN; PSSLDPQLAEDNV; SSLDPQLAEDNVA; SLDPQLAEDNVAS; LDPQLAEDNVASK; DPQLAEDNVASKM; PQLAEDNVASKMI; QLAEDNVASKMID; LAEDNVASKMIDT; |

| | AEDNVASKMIDTM; EDNVASKMIDTMF; DNVASKMIDTMFR; NVASKMIDTMFRG; VASKMIDTMFRGI; ASKMIDTMFRGIV; SKMIDTMFRGIVT; KMIDTMFRGIVTG; MIDTMFRGIVTGD; IDTMFRGIVTGDP; DTMFRGIVTGDPN; TMFRGIVTGDPNT; MFRGIVTGDPNTG; FRGIVTGDPNTGG; RGIVTGDPNTGGN; GIVTGDPNTGGNK; IVTGDPNTGGNKP; VTGDPNTGGNKPG; TGDPNTGGNKPGL; GDPNTGGNKPGLA; DPNTGGNKPGLAK; PNTGGNKPGLAKG; NTGGNKPGLAKGW; TGGNKPGLAKGWD; GGNKPGLAKGWDI; GNKPGLAKGWDIS; NKPGLAKGWDISS; KPGLAKGWDISSD; PGLAKGWDISSDG; GLAKGWDISSDGT; LAKGWDISSDGTV; AKGWDISSDGTVY; KGWDISSDGTVYT; GWDISSDGTVYTF; WDISSDGTVYTFN; DISSDGTVYTFNL; ISSDGTVYTFNLR; SSDGTVYTFNLRE; SDGTVYTFNLREK; DGTVYTFNLREKI; GTVYTFNLREKIT; TVYTFNLREKITW; VYTFNLREKITWS; YTFNLREKITWSD; TFNLREKITWSDG; FNLREKITWSDGV; NLREKITWSDGVA; LREKITWSDGVAI; REKITWSDGVAIT; EKITWSDGVAITA; KITWSDGVAITAE; ITWSDGVAITAEG; TWSDGVAITAEGI; WSDGVAITAEGIR; SDGVAITAEGIRK; DGVAITAEGIRKS; GVAITAEGIRKSY; VAITAEGIRKSYL; AITAEGIRKSYLR; ITAEGIRKSYLRI; TAEGIRKSYLRIL; AEGIRKSYLRILN; EGIRKSYLRILNK; GIRKSYLRILNKE; IRKSYLRILNKET; RKSYLRILNKETG; KSYLRILNKETGS; SYLRILNKETGSK; YLRILNKETGSKY; LRILNKETGSKYV; RILNKETGSKYVE; ILNKETGSKYVEM; LNKETGSKYVEMV; NKETGSKYVEMVK; KETGSKYVEMVKS; ETGSKYVEMVKSV; TGSKYVEMVKSVI; GSKYVEMVKSVIK; SKYVEMVKSVIKN; KYVEMVKSVIKNG; YVEMVKSVIKNGQ; VEMVKSVIKNGQK; EMVKSVIKNGQKY; MVKSVIKNGQKYF; VKSVIKNGQKYFD; KSVIKNGQKYFDG; SVIKNGQKYFDGQ; VIKNGQKYFDGQV; IKNGQKYFDGQVT; KNGQKYFDGQVTD; NGQKYFDGQVTDS; GQKYFDGQVTDSE; QKYFDGQVTDSEL; KYFDGQVTDSELG; YFDGQVTDSELGI; FDGQVTDSELGIR; DGQVTDSELGIRA; GQVTDSELGIRAI; QVTDSELGIRAID; VTDSELGIRAIDE; TDSELGIRAIDEK; DSELGIRAIDEKT; SELGIRAIDEKTL; ELGIRAIDEKTLE; LGIRAIDEKTLEI; GIRAIDEKTLEIT; IRAIDEKTLEITL; RAIDEKTLEITLE; AIDEKTLEITLES; IDEKTLEITLESP; DEKTLEITLESPK; EKTLEITLESPKP; KTLEITLESPKPY; TLEITLESPKPYF; LEITLESPKPYFI; EITLESPKPYFID; ITLESPKPYFIDM; TLESPKPYFIDML; LESPKPYFIDMLV; ESPKPYFIDMLVH; SPKPYFIDMLVHQ; PKPYFIDMLVHQS; KPYFIDMLVHQSF; PYFIDMLVHQSFI; YFIDMLVHQSFIP; FIDMLVHQSFIPV; IDMLVHQSFIPVP; DMLVHQSFIPVPV; MLVHQSFIPVPVH; LVHQSFIPVPVHV; VHQSFIPVPVHVT; HQSFIPVPVHVTE; QSFIPVPVHVTEK; SFIPVPVHVTEKY; FIPVPVHVTEKYG; IPVPVHVTEKYGQ; PVPVHVTEKYGQN; VPVHVTEKYGQNW; PVHVTEKYGQNWT; VHVTEKYGQNWTS; HVTEKYGQNWTSP; VTEKYGQNWTSPE; TEKYGQNWTSPEN; EKYGQNWTSPENM; KYGQNWTSPENMV; YGQNWTSPENMVT; GQNWTSPENMVTS; QNWTSPENMVTSG; NWTSPENMVTSGP; WTSPENMVTSGPF; TSPENMVTSGPFK; SPENMVTSGPFKL; PENMVTSGPFKLK; |
|---|---|

| | |
|---|---|
| | ENMVTSGPFKLKE; NMVTSGPFKLKER; MVTSGPFKLKERI;<br>VTSGPFKLKERIP; TSGPFKLKERIPN; SGPFKLKERIPNE;<br>GPFKLKERIPNEK; PFKLKERIPNEKY; FKLKERIPNEKYV;<br>KLKERIPNEKYVF; LKERIPNEKYVFE; KERIPNEKYVFEK;<br>ERIPNEKYVFEKN; RIPNEKYVFEKNN; IPNEKYVFEKNNK;<br>PNEKYVFEKNNKY; NEKYVFEKNNKYY; EKYVFEKNNKYYD;<br>KYVFEKNNKYYDS; YVFEKNNKYYDSN; VFEKNNKYYDSNE;<br>FEKNNKYYDSNEV; EKNNKYYDSNEVE; KNNKYYDSNEVEL;<br>NNKYYDSNEVELE; NKYYDSNEVELEE; KYYDSNEVELEEI;<br>YYDSNEVELEEIT; YDSNEVELEEITF; DSNEVELEEITFY;<br>SNEVELEEITFYT; NEVELEEITFYTT; EVELEEITFYTTN;<br>VELEEITFYTTND; ELEEITFYTTNDS; LEEITFYTTNDSS;<br>EEITFYTTNDSST; EITFYTTNDSSTA; ITFYTTNDSSTAY;<br>TFYTTNDSSTAYK; FYTTNDSSTAYKM; YTTNDSSTAYKMY;<br>TTNDSSTAYKMYE; TNDSSTAYKMYEN; NDSSTAYKMYENE;<br>DSSTAYKMYENEE; SSTAYKMYENEEL; STAYKMYENEELD;<br>TAYKMYENEELDA; AYKMYENEELDAI; YKMYENEELDAIF;<br>KMYENEELDAIFG; MYENEELDAIFGS; YENEELDAIFGSI;<br>ENEELDAIFGSIP; NEELDAIFGSIPP; EELDAIFGSIPPD;<br>ELDAIFGSIPPDL; LDAIFGSIPPDLI; DAIFGSIPPDLIK;<br>AIFGSIPPDLIKN; IFGSIPPDLIKNL; FGSIPPDLIKNLK;<br>GSIPPDLIKNLKL; SIPPDLIKNLKLR; IPPDLIKNLKLRS;<br>PPDLIKNLKLRSD; PDLIKNLKLRSDY; DLIKNLKLRSDYY;<br>LIKNLKLRSDYYS; IKNLKLRSDYYSS; KNLKLRSDYYSSA;<br>NLKLRSDYYSSAV; LKLRSDYYSSAVN; KLRSDYYSSAVNA;<br>LRSDYYSSAVNAI; RSDYYSSAVNAIY; SDYYSSAVNAIYF;<br>DYYSSAVNAIYFY; YYSSAVNAIYFYA; YSSAVNAIYFYAF;<br>SSAVNAIYFYAFN; SAVNAIYFYAFNT; AVNAIYFYAFNTH;<br>VNAIYFYAFNTHI; NAIYFYAFNTHIK; AIYFYAFNTHIKP;<br>IYFYAFNTHIKPL; YFYAFNTHIKPLD; FYAFNTHIKPLDN;<br>YAFNTHIKPLDNV; AFNTHIKPLDNVK; FNTHIKPLDNVKI;<br>NTHIKPLDNVKIR; THIKPLDNVKIRK; HIKPLDNVKIRKA;<br>IKPLDNVKIRKAL; KPLDNVKIRKALT; PLDNVKIRKALTL;<br>LDNVKIRKALTLA; DNVKIRKALTLAI; NVKIRKALTLAID;<br>VKIRKALTLAIDR; KIRKALTLAIDRE; IRKALTLAIDRET;<br>RKALTLAIDRETL; KALTLAIDRETLT; ALTLAIDRETLTY;<br>LTLAIDRETLTYK; TLAIDRETLTYKV; LAIDRETLTYKVL;<br>AIDRETLTYKVLD; IDRETLTYKVLDN; DRETLTYKVLDNG;<br>RETLTYKVLDNGT; ETLTYKVLDNGTT; TLTYKVLDNGTTP;<br>LTYKVLDNGTTPT; TYKVLDNGTTPTR; YKVLDNGTTPTRR;<br>KVLDNGTTPTRRA; VLDNGTTPTRRAT; LDNGTTPTRRATP;<br>DNGTTPTRRATPN; NGTTPTRRATPNF; GTTPTRRATPNFS;<br>TTPTRRATPNFSS; TPTRRATPNFSSY; PTRRATPNFSSYS;<br>TRRATPNFSSYSY; RRATPNFSSYSYA; RATPNFSSYSYAK;<br>ATPNFSSYSYAKS; TPNFSSYSYAKSL; PNFSSYSYAKSLE;<br>NFSSYSYAKSLEL; FSSYSYAKSLELF; SSYSYAKSLELFN;<br>SYSYAKSLELFNP; YSYAKSLELFNPE; SYAKSLELFNPEI;<br>YAKSLELFNPEIA; AKSLELFNPEIAK; KSLELFNPEIAKT;<br>SLELFNPEIAKTL; LELFNPEIAKTLL; ELFNPEIAKTLLA;<br>LFNPEIAKTLLAE; FNPEIAKTLLAEA; NPEIAKTLLAEAG;<br>PEIAKTLLAEAGY; EIAKTLLAEAGYP; IAKTLLAEAGYPN; |

AKTLLAEAGYPNG; KTLLAEAGYPNGN; TLLAEAGYPNGNG;
LLAEAGYPNGNGF; LAEAGYPNGNGFP; AEAGYPNGNGFPI;
EAGYPNGNGFPIL; AGYPNGNGFPILK; GYPNGNGFPILKL;
YPNGNGFPILKLK; PNGNGFPILKLKY; NGNGFPILKLKYN;
GNGFPILKLKYNT; NGFPILKLKYNTN; GFPILKLKYNTNE;
FPILKLKYNTNEA; PILKLKYNTNEAN; ILKLKYNTNEANK;
LKLKYNTNEANKK; KLKYNTNEANKKI; LKYNTNEANKKIC;
KYNTNEANKKICE; YNTNEANKKICEF; NTNEANKKICEFI;
TNEANKKICEFIQ; NEANKKICEFIQN; EANKKICEFIQNQ;
ANKKICEFIQNQW; NKKICEFIQNQWK; KKICEFIQNQWKK;
KICEFIQNQWKKN; ICEFIQNQWKKNL; CEFIQNQWKKNLN;
EFIQNQWKKNLNI; FIQNQWKKNLNID; IQNQWKKNLNIDV;
QNQWKKNLNIDVE; NQWKKNLNIDVEL; QWKKNLNIDVELE;
WKKNLNIDVELEN; KKNLNIDVELENE; KNLNIDVELENEE;
NLNIDVELENEEW; LNIDVELENEEWT; NIDVELENEEWTT;
IDVELENEEWTTY; DVELENEEWTTYL; VELENEEWTTYLN;
ELENEEWTTYLNT; LENEEWTTYLNTK; ENEEWTTYLNTKA;
NEEWTTYLNTKAN; EEWTTYLNTKANG; EWTTYLNTKANGN;
WTTYLNTKANGNY; TTYLNTKANGNYE; TYLNTKANGNYEI;
YLNTKANGNYEIA; LNTKANGNYEIAR; NTKANGNYEIARA;
TKANGNYEIARAG; KANGNYEIARAGW; ANGNYEIARAGWI;
NGNYEIARAGWIG; GNYEIARAGWIGD; NYEIARAGWIGDY;
YEIARAGWIGDYA; EIARAGWIGDYAD; IARAGWIGDYADP;
ARAGWIGDYADPL; RAGWIGDYADPLT; AGWIGDYADPLTF;
GWIGDYADPLTFL; WIGDYADPLTFLS; IGDYADPLTFLSI;
GDYADPLTFLSIF; DYADPLTFLSIFT; YADPLTFLSIFTQ;
ADPLTFLSIFTQG; DPLTFLSIFTQGY; PLTFLSIFTQGYT;
LTFLSIFTQGYTQ; TFLSIFTQGYTQF; FLSIFTQGYTQFS;
LSIFTQGYTQFSS; SIFTQGYTQFSSH; IFTQGYTQFSSHN;
FTQGYTQFSSHNY; TQGYTQFSSHNYS; QGYTQFSSHNYSN;
GYTQFSSHNYSNP; YTQFSSHNYSNPE; TQFSSHNYSNPEY;
QFSSHNYSNPEYN; FSSHNYSNPEYNE; SSHNYSNPEYNEL;
SHNYSNPEYNELI; HNYSNPEYNELIK; NYSNPEYNELIKK;
YSNPEYNELIKKS; SNPEYNELIKKSD; NPEYNELIKKSDL;
PEYNELIKKSDLE; EYNELIKKSDLEL; YNELIKKSDLELD;
NELIKKSDLELDP; ELIKKSDLELDPI; LIKKSDLELDPIK;
IKKSDLELDPIKR; KKSDLELDPIKRQ; KSDLELDPIKRQD;
SDLELDPIKRQDI; DLELDPIKRQDIL; LELDPIKRQDILR;
ELDPIKRQDILRQ; LDPIKRQDILRQA; DPIKRQDILRQAE;
PIKRQDILRQAEE; IKRQDILRQAEEI; KRQDILRQAEEII;
RQDILRQAEEIII; QDILRQAEEIIIE; DILRQAEEIIIEK;
ILRQAEEIIIEKD; LRQAEEIIIEKDF; RQAEEIIIEKDFP;
QAEEIIIEKDFPI; AEEIIIEKDFPIA; EEIIIEKDFPIAP;
EIIIEKDFPIAPI; IIIEKDFPIAPIY; IIEKDFPIAPIYI;
IEKDFPIAPIYIY; EKDFPIAPIYIYG; KDFPIAPIYIYGN;
DFPIAPIYIYGNS; FPIAPIYIYGNSY; PIAPIYIYGNSYL;
IAPIYIYGNSYLF; APIYIYGNSYLFR; PIYIYGNSYLFRN;
IYIYGNSYLFRND; YIYGNSYLFRNDK; IYGNSYLFRNDKW;
YGNSYLFRNDKWT; GNSYLFRNDKWTG; NSYLFRNDKWTGW;
SYLFRNDKWTGWN; YLFRNDKWTGWNT; LFRNDKWTGWNTN;
FRNDKWTGWNTNI; RNDKWTGWNTNIL; NDKWTGWNTNILE;

DKWTGWNTNILER; KWTGWNTNILERF; WTGWNTNILERFD;
TGWNTNILERFDL; GWNTNILERFDLS; WNTNILERFDLSQ;
NTNILERFDLSQL; TNILERFDLSQLK; NILERFDLSQLKL;
ILERFDLSQLKLK; LERFDLSQLKLKN; ERFDLSQLKLKNK;

14 mers:
MKLQRSLFLIIFFL; KLQRSLFLIIFFLT; LQRSLFLIIFFLTF;
QRSLFLIIFFLTFL; RSLFLIIFFLTFLC; SLFLIIFFLTFLCC;
LFLIIFFLTFLCCN; FLIIFFLTFLCCNN; LIIFFLTFLCCNNK;
IIFFLTFLCCNNKE; IFFLTFLCCNNKER; FFLTFLCCNNKERK;
FLTFLCCNNKERKE; LTFLCCNNKERKEG; TFLCCNNKERKEGV;
FLCCNNKERKEGVS; LCCNNKERKEGVSF; CCNNKERKEGVSFK;
CNNKERKEGVSFKI; NNKERKEGVSFKIS; NKERKEGVSFKISL;
KERKEGVSFKISLG; ERKEGVSFKISLGA; RKEGVSFKISLGAE;
KEGVSFKISLGAEP; EGVSFKISLGAEPS; GVSFKISLGAEPSS;
VSFKISLGAEPSSL; SFKISLGAEPSSLD; FKISLGAEPSSLDP;
KISLGAEPSSLDPQ; ISLGAEPSSLDPQL; SLGAEPSSLDPQLA;
LGAEPSSLDPQLAE; GAEPSSLDPQLAED; AEPSSLDPQLAEDN;
EPSSLDPQLAEDNV; PSSLDPQLAEDNVA; SSLDPQLAEDNVAS;
SLDPQLAEDNVASK; LDPQLAEDNVASKM; DPQLAEDNVASKMI;
PQLAEDNVASKMID; QLAEDNVASKMIDT; LAEDNVASKMIDTM;
AEDNVASKMIDTMF; EDNVASKMIDTMFR; DNVASKMIDTMFRG;
NVASKMIDTMFRGI; VASKMIDTMFRGIV; ASKMIDTMFRGIVT;
SKMIDTMFRGIVTG; KMIDTMFRGIVTGD; MIDTMFRGIVTGDP;
IDTMFRGIVTGDPN; DTMFRGIVTGDPNT; TMFRGIVTGDPNTG;
MFRGIVTGDPNTGG; FRGIVTGDPNTGGN; RGIVTGDPNTGGNK;
GIVTGDPNTGGNKP; IVTGDPNTGGNKPG; VTGDPNTGGNKPGL;
TGDPNTGGNKPGLA; GDPNTGGNKPGLAK; DPNTGGNKPGLAKG;
PNTGGNKPGLAKGW; NTGGNKPGLAKGWD; TGGNKPGLAKGWDI;
GGNKPGLAKGWDIS; GNKPGLAKGWDISS; NKPGLAKGWDISSD;
KPGLAKGWDISSDG; PGLAKGWDISSDGT; GLAKGWDISSDGTV;
LAKGWDISSDGTVY; AKGWDISSDGTVYT; KGWDISSDGTVYTF;
GWDISSDGTVYTFN; WDISSDGTVYTFNL; DISSDGTVYTFNLR;
ISSDGTVYTFNLRE; SSDGTVYTFNLREK; SDGTVYTFNLREKI;
DGTVYTFNLREKIT; GTVYTFNLREKITW; TVYTFNLREKITWS;
VYTFNLREKITWSD; YTFNLREKITWSDG; TFNLREKITWSDGV;
FNLREKITWSDGVA; NLREKITWSDGVAI; LREKITWSDGVAIT;
REKITWSDGVAITA; EKITWSDGVAITAE; KITWSDGVAITAEG;
ITWSDGVAITAEGI; TWSDGVAITAEGIR; WSDGVAITAEGIRK;
SDGVAITAEGIRKS; DGVAITAEGIRKSY; GVAITAEGIRKSYL;
VAITAEGIRKSYLR; AITAEGIRKSYLRI; ITAEGIRKSYLRIL;
TAEGIRKSYLRILN; AEGIRKSYLRILNK; EGIRKSYLRILNKE;
GIRKSYLRILNKET; IRKSYLRILNKETG; RKSYLRILNKETGS;
KSYLRILNKETGSK; SYLRILNKETGSKY; YLRILNKETGSKYV;
LRILNKETGSKYVE; RILNKETGSKYVEM; ILNKETGSKYVEMV;
LNKETGSKYVEMVK; NKETGSKYVEMVKS; KETGSKYVEMVKSV;
ETGSKYVEMVKSVI; TGSKYVEMVKSVIK; GSKYVEMVKSVIKN;
SKYVEMVKSVIKNG; KYVEMVKSVIKNGQ; YVEMVKSVIKNGQK;
VEMVKSVIKNGQKY; EMVKSVIKNGQKYF; MVKSVIKNGQKYFD;
VKSVIKNGQKYFDG; KSVIKNGQKYFDGQ; SVIKNGQKYFDGQV;
VIKNGQKYFDGQVT; IKNGQKYFDGQVTD; KNGQKYFDGQVTDS;

| | | |
|---|---|---|
| NGQKYFDGQVTDSE; | GQKYFDGQVTDSEL; | QKYFDGQVTDSELG; |
| KYFDGQVTDSELGI; | YFDGQVTDSELGIR; | FDGQVTDSELGIRA; |
| DGQVTDSELGIRAI; | GQVTDSELGIRAID; | QVTDSELGIRAIDE; |
| VTDSELGIRAIDEK; | TDSELGIRAIDEKT; | DSELGIRAIDEKTL; |
| SELGIRAIDEKTLE; | ELGIRAIDEKTLEI; | LGIRAIDEKTLEIT; |
| GIRAIDEKTLEITL; | IRAIDEKTLEITLE; | RAIDEKTLEITLES; |
| AIDEKTLEITLESP; | IDEKTLEITLESPK; | DEKTLEITLESPKP; |
| EKTLEITLESPKPY; | KTLEITLESPKPYF; | TLEITLESPKPYFI; |
| LEITLESPKPYFID; | EITLESPKPYFIDM; | ITLESPKPYFIDML; |
| TLESPKPYFIDMLV; | LESPKPYFIDMLVH; | ESPKPYFIDMLVHQ; |
| SPKPYFIDMLVHQS; | PKPYFIDMLVHQSF; | KPYFIDMLVHQSFI; |
| PYFIDMLVHQSFIP; | YFIDMLVHQSFIPV; | FIDMLVHQSFIPVP; |
| IDMLVHQSFIPVPV; | DMLVHQSFIPVPVH; | MLVHQSFIPVPVHV; |
| LVHQSFIPVPVHVT; | VHQSFIPVPVHVTE; | HQSFIPVPVHVTEK; |
| QSFIPVPVHVTEKY; | SFIPVPVHVTEKYG; | FIPVPVHVTEKYGQ; |
| IPVPVHVTEKYGQN; | PVPVHVTEKYGQNW; | VPVHVTEKYGQNWT; |
| PVHVTEKYGQNWTS; | VHVTEKYGQNWTSP; | HVTEKYGQNWTSPE; |
| VTEKYGQNWTSPEN; | TEKYGQNWTSPENM; | EKYGQNWTSPENMV; |
| KYGQNWTSPENMVT; | YGQNWTSPENMVTS; | GQNWTSPENMVTSG; |
| QNWTSPENMVTSGP; | NWTSPENMVTSGPF; | WTSPENMVTSGPFK; |
| TSPENMVTSGPFKL; | SPENMVTSGPFKLK; | PENMVTSGPFKLKE; |
| ENMVTSGPFKLKER; | NMVTSGPFKLKERI; | MVTSGPFKLKERIP; |
| VTSGPFKLKERIPN; | TSGPFKLKERIPNE; | SGPFKLKERIPNEK; |
| GPFKLKERIPNEKY; | PFKLKERIPNEKYV; | FKLKERIPNEKYVF; |
| KLKERIPNEKYVFE; | LKERIPNEKYVFEK; | KERIPNEKYVFEKN; |
| ERIPNEKYVFEKNN; | RIPNEKYVFEKNNK; | IPNEKYVFEKNNKY; |
| PNEKYVFEKNNKYY; | NEKYVFEKNNKYYD; | EKYVFEKNNKYYDS; |
| KYVFEKNNKYYDSN; | YVFEKNNKYYDSNE; | VFEKNNKYYDSNEV; |
| FEKNNKYYDSNEVE; | EKNNKYYDSNEVEL; | KNNKYYDSNEVELE; |
| NNKYYDSNEVELEE; | NKYYDSNEVELEEI; | KYYDSNEVELEEIT; |
| YYDSNEVELEEITF; | YDSNEVELEEITFY; | DSNEVELEEITFYT; |
| SNEVELEEITFYTT; | NEVELEEITFYTTN; | EVELEEITFYTTND; |
| VELEEITFYTTNDS; | ELEEITFYTTNDSS; | LEEITFYTTNDSST; |
| EEITFYTTNDSSTA; | EITFYTTNDSSTAY; | ITFYTTNDSSTAYK; |
| TFYTTNDSSTAYKM; | FYTTNDSSTAYKMY; | YTTNDSSTAYKMYE; |
| TTNDSSTAYKMYEN; | TNDSSTAYKMYENE; | NDSSTAYKMYENEE; |
| DSSTAYKMYENEEL; | SSTAYKMYENEELD; | STAYKMYENEELDA; |
| TAYKMYENEELDAI; | AYKMYENEELDAIF; | YKMYENEELDAIFG; |
| KMYENEELDAIFGS; | MYENEELDAIFGSI; | YENEELDAIFGSIP; |
| ENEELDAIFGSIPP; | NEELDAIFGSIPPD; | EELDAIFGSIPPDL; |
| ELDAIFGSIPPDLI; | LDAIFGSIPPDLIK; | DAIFGSIPPDLIKN; |
| AIFGSIPPDLIKNL; | IFGSIPPDLIKNLK; | FGSIPPDLIKNLKL; |
| GSIPPDLIKNLKLR; | SIPPDLIKNLKLRS; | IPPDLIKNLKLRSD; |
| PPDLIKNLKLRSDY; | PDLIKNLKLRSDYY; | DLIKNLKLRSDYYS; |
| LIKNLKLRSDYYSS; | IKNLKLRSDYYSSA; | KNLKLRSDYYSSAV; |
| NLKLRSDYYSSAVN; | LKLRSDYYSSAVNA; | KLRSDYYSSAVNAI; |
| LRSDYYSSAVNAIY; | RSDYYSSAVNAIYF; | SDYYSSAVNAIYFY; |
| DYYSSAVNAIYFYA; | YYSSAVNAIYFYAF; | YSSAVNAIYFYAFN; |
| SSAVNAIYFYAFNT; | SAVNAIYFYAFNTH; | AVNAIYFYAFNTHI; |
| VNAIYFYAFNTHIK; | NAIYFYAFNTHIKP; | AIYFYAFNTHIKPL; |
| IYFYAFNTHIKPLD; | YFYAFNTHIKPLDN; | FYAFNTHIKPLDNV; |

| | |
|---|---|
| YAFNTHIKPLDNVK; AFNTHIKPLDNVKI; FNTHIKPLDNVKIR;<br>NTHIKPLDNVKIRK; THIKPLDNVKIRKA; HIKPLDNVKIRKAL;<br>IKPLDNVKIRKALT; KPLDNVKIRKALTL; PLDNVKIRKALTLA;<br>LDNVKIRKALTLAI; DNVKIRKALTLAID; NVKIRKALTLAIDR;<br>VKIRKALTLAIDRE; KIRKALTLAIDRET; IRKALTLAIDRETL;<br>RKALTLAIDRETLT; KALTLAIDRETLTY; ALTLAIDRETLTYK;<br>LTLAIDRETLTYKV; TLAIDRETLTYKVL; LAIDRETLTYKVLD;<br>AIDRETLTYKVLDN; IDRETLTYKVLDNG; DRETLTYKVLDNGT;<br>RETLTYKVLDNGTT; ETLTYKVLDNGTTP; TLTYKVLDNGTTPT;<br>LTYKVLDNGTTPTR; TYKVLDNGTTPTRR; YKVLDNGTTPTRRA;<br>KVLDNGTTPTRRAT; VLDNGTTPTRRATP; LDNGTTPTRRATPN;<br>DNGTTPTRRATPNF; NGTTPTRRATPNFS; GTTPTRRATPNFSS;<br>TTPTRRATPNFSSY; TPTRRATPNFSSYS; PTRRATPNFSSYSY;<br>TRRATPNFSSYSYA; RRATPNFSSYSYAK; RATPNFSSYSYAKS;<br>ATPNFSSYSYAKSL; TPNFSSYSYAKSLE; PNFSSYSYAKSLEL;<br>NFSSYSYAKSLELF; FSSYSYAKSLELFN; SSYSYAKSLELFNP;<br>SYSYAKSLELFNPE; YSYAKSLELFNPEI; SYAKSLELFNPEIA;<br>YAKSLELFNPEIAK; AKSLELFNPEIAKT; KSLELFNPEIAKTL;<br>SLELFNPEIAKTLL; LELFNPEIAKTLLA; ELFNPEIAKTLLAE;<br>LFNPEIAKTLLAEA; FNPEIAKTLLAEAG; NPEIAKTLLAEAGY;<br>PEIAKTLLAEAGYP; EIAKTLLAEAGYPN; IAKTLLAEAGYPNG;<br>AKTLLAEAGYPNGN; KTLLAEAGYPNGNG; TLLAEAGYPNGNGF;<br>LLAEAGYPNGNGFP; LAEAGYPNGNGFPI; AEAGYPNGNGFPIL;<br>EAGYPNGNGFPILK; AGYPNGNGFPILKL; GYPNGNGFPILKLK;<br>YPNGNGFPILKLKY; PNGNGFPILKLKYN; NGNGFPILKLKYNT;<br>GNGFPILKLKYNTN; NGFPILKLKYNTNE; GFPILKLKYNTNEA;<br>FPILKLKYNTNEAN; PILKLKYNTNEANK; ILKLKYNTNEANKK;<br>LKLKYNTNEANKKI; KLKYNTNEANKKIC; LKYNTNEANKKICE;<br>KYNTNEANKKICEF; YNTNEANKKICEFI; NTNEANKKICEFIQ;<br>TNEANKKICEFIQN; NEANKKICEFIQNQ; EANKKICEFIQNQW;<br>ANKKICEFIQNQWK; NKKICEFIQNQWKK; KKICEFIQNQWKKN;<br>KICEFIQNQWKKNL; ICEFIQNQWKKNLN; CEFIQNQWKKNLNI;<br>EFIQNQWKKNLNID; FIQNQWKKNLNIDV; IQNQWKKNLNIDVE;<br>QNQWKKNLNIDVEL; NQWKKNLNIDVELE; QWKKNLNIDVELEN;<br>WKKNLNIDVELENE; KKNLNIDVELENEE; KNLNIDVELENEEW;<br>NLNIDVELENEEWT; LNIDVELENEEWTT; NIDVELENEEWTTY;<br>IDVELENEEWTTYL; DVELENEEWTTYLN; VELENEEWTTYLNT;<br>ELENEEWTTYLNTK; LENEEWTTYLNTKA; ENEEWTTYLNTKAN;<br>NEEWTTYLNTKANG; EEWTTYLNTKANGN; EWTTYLNTKANGNY;<br>WTTYLNTKANGNYE; TTYLNTKANGNYEI; TYLNTKANGNYEIA;<br>YLNTKANGNYEIAR; LNTKANGNYEIARA; NTKANGNYEIARAG;<br>TKANGNYEIARAGW; KANGNYEIARAGWI; ANGNYEIARAGWIG;<br>NGNYEIARAGWIGD; GNYEIARAGWIGDY; NYEIARAGWIGDYA;<br>YEIARAGWIGDYAD; EIARAGWIGDYADP; IARAGWIGDYADPL;<br>ARAGWIGDYADPLT; RAGWIGDYADPLTF; AGWIGDYADPLTFL;<br>GWIGDYADPLTFLS; WIGDYADPLTFLSI; IGDYADPLTFLSIF;<br>GDYADPLTFLSIFT; DYADPLTFLSIFTQ; YADPLTFLSIFTQG;<br>ADPLTFLSIFTQGY; DPLTFLSIFTQGYT; PLTFLSIFTQGYTQ;<br>LTFLSIFTQGYTQF; TFLSIFTQGYTQFS; FLSIFTQGYTQFSS;<br>LSIFTQGYTQFSSH; SIFTQGYTQFSSHN; IFTQGYTQFSSHNY;<br>FTQGYTQFSSHNYS; TQGYTQFSSHNYSN; QGYTQFSSHNYSNP; |

GYTQFSSHNYSNPE; YTQFSSHNYSNPEY; TQFSSHNYSNPEYN;
QFSSHNYSNPEYNE; FSSHNYSNPEYNEL; SSHNYSNPEYNELI;
SHNYSNPEYNELIK; HNYSNPEYNELIKK; NYSNPEYNELIKKS;
YSNPEYNELIKKSD; SNPEYNELIKKSDL; NPEYNELIKKSDLE;
PEYNELIKKSDLEL; EYNELIKKSDLELD; YNELIKKSDLELDP;
NELIKKSDLELDPI; ELIKKSDLELDPIK; LIKKSDLELDPIKR;
IKKSDLELDPIKRQ; KKSDLELDPIKRQD; KSDLELDPIKRQDI;
SDLELDPIKRQDIL; DLELDPIKRQDILR; LELDPIKRQDILRQ;
ELDPIKRQDILRQA; LDPIKRQDILRQAE; DPIKRQDILRQAEE;
PIKRQDILRQAEEI; IKRQDILRQAEEII; KRQDILRQAEEIII;
RQDILRQAEEIIIE; QDILRQAEEIIIEK; DILRQAEEIIIEKD;
ILRQAEEIIIEKDF; LRQAEEIIIEKDFP; RQAEEIIIEKDFPI;
QAEEIIIEKDFPIA; AEEIIIEKDFPIAP; EEIIIEKDFPIAPI;
EIIIEKDFPIAPIY; IIIEKDFPIAPIYI; IIEKDFPIAPIYIY;
IEKDFPIAPIYIYG; EKDFPIAPIYIYGN; KDFPIAPIYIYGNS;
DFPIAPIYIYGNSY; FPIAPIYIYGNSYL; PIAPIYIYGNSYLF;
IAPIYIYGNSYLFR; APIYIYGNSYLFRN; PIYIYGNSYLFRND;
IYIYGNSYLFRNDK; YIYGNSYLFRNDKW; IYGNSYLFRNDKWT;
YGNSYLFRNDKWTG; GNSYLFRNDKWTGW; NSYLFRNDKWTGWN;
SYLFRNDKWTGWNT; YLFRNDKWTGWNTN; LFRNDKWTGWNTNI;
FRNDKWTGWNTNIL; RNDKWTGWNTNILE; NDKWTGWNTNILER;
DKWTGWNTNILERF; KWTGWNTNILERFD; WTGWNTNILERFDL;
TGWNTNILERFDLS; GWNTNILERFDLSQ; WNTNILERFDLSQL;
NTNILERFDLSQLK; TNILERFDLSQLKL; NILERFDLSQLKLK;
ILERFDLSQLKLKN; LERFDLSQLKLKNK;

15 mers:
MKLQRSLFLIIFFLT; KLQRSLFLIIFFLTF; LQRSLFLIIFFLTFL;
QRSLFLIIFFLTFLC; RSLFLIIFFLTFLCC; SLFLIIFFLTFLCCN;
LFLIIFFLTFLCCNN; FLIIFFLTFLCCNNK; LIIFFLTFLCCNNKE;
IIFFLTFLCCNNKER; IFFLTFLCCNNKERK; FFLTFLCCNNKERKE;
FLTFLCCNNKERKEG; LTFLCCNNKERKEGV; TFLCCNNKERKEGVS;
FLCCNNKERKEGVSF; LCCNNKERKEGVSFK; CCNNKERKEGVSFKI;
CNNKERKEGVSFKIS; NNKERKEGVSFKISL; NKERKEGVSFKISLG;
KERKEGVSFKISLGA; ERKEGVSFKISLGAE; RKEGVSFKISLGAEP;
KEGVSFKISLGAEPS; EGVSFKISLGAEPSS; GVSFKISLGAEPSSL;
VSFKISLGAEPSSLD; SFKISLGAEPSSLDP; FKISLGAEPSSLDPQ;
KISLGAEPSSLDPQL; ISLGAEPSSLDPQLA; SLGAEPSSLDPQLAE;
LGAEPSSLDPQLAED; GAEPSSLDPQLAEDN; AEPSSLDPQLAEDNV;
EPSSLDPQLAEDNVA; PSSLDPQLAEDNVAS; SSLDPQLAEDNVASK;
SLDPQLAEDNVASKM; LDPQLAEDNVASKMI; DPQLAEDNVASKMID;
PQLAEDNVASKMIDT; QLAEDNVASKMIDTM; LAEDNVASKMIDTMF;
AEDNVASKMIDTMFR; EDNVASKMIDTMFRG; DNVASKMIDTMFRGI;
NVASKMIDTMFRGIV; VASKMIDTMFRGIVT; ASKMIDTMFRGIVTG;
SKMIDTMFRGIVTGD; KMIDTMFRGIVTGDP; MIDTMFRGIVTGDPN;
IDTMFRGIVTGDPNT; DTMFRGIVTGDPNTG; TMFRGIVTGDPNTGG;
MFRGIVTGDPNTGGN; FRGIVTGDPNTGGNK; RGIVTGDPNTGGNKP;
GIVTGDPNTGGNKPG; IVTGDPNTGGNKPGL; VTGDPNTGGNKPGLA;
TGDPNTGGNKPGLAK; GDPNTGGNKPGLAKG; DPNTGGNKPGLAKGW;
PNTGGNKPGLAKGWD; NTGGNKPGLAKGWDI; TGGNKPGLAKGWDIS;
GGNKPGLAKGWDISS; GNKPGLAKGWDISSD; NKPGLAKGWDISSDG;

KPGLAKGWDISSDGT; PGLAKGWDISSDGTV; GLAKGWDISSDGTVY;
LAKGWDISSDGTVYT; AKGWDISSDGTVYTF; KGWDISSDGTVYTFN;
GWDISSDGTVYTFNL; WDISSDGTVYTFNLR; DISSDGTVYTFNLRE;
ISSDGTVYTFNLREK; SSDGTVYTFNLREKI; SDGTVYTFNLREKIT;
DGTVYTFNLREKITW; GTVYTFNLREKITWS; TVYTFNLREKITWSD;
VYTFNLREKITWSDG; YTFNLREKITWSDGV; TFNLREKITWSDGVA;
FNLREKITWSDGVAI; NLREKITWSDGVAIT; LREKITWSDGVAITA;
REKITWSDGVAITAE; EKITWSDGVAITAEG; KITWSDGVAITAEGI;
ITWSDGVAITAEGIR; TWSDGVAITAEGIRK; WSDGVAITAEGIRKS;
SDGVAITAEGIRKSY; DGVAITAEGIRKSYL; GVAITAEGIRKSYLR;
VAITAEGIRKSYLRI; AITAEGIRKSYLRIL; ITAEGIRKSYLRILN;
TAEGIRKSYLRILNK; AEGIRKSYLRILNKE; EGIRKSYLRILNKET;
GIRKSYLRILNKETG; IRKSYLRILNKETGS; RKSYLRILNKETGSK;
KSYLRILNKETGSKY; SYLRILNKETGSKYV; YLRILNKETGSKYVE;
LRILNKETGSKYVEM; RILNKETGSKYVEMV; ILNKETGSKYVEMVK;
LNKETGSKYVEMVKS; NKETGSKYVEMVKSV; KETGSKYVEMVKSVI;
ETGSKYVEMVKSVIK; TGSKYVEMVKSVIKN; GSKYVEMVKSVIKNG;
SKYVEMVKSVIKNGQ; KYVEMVKSVIKNGQK; YVEMVKSVIKNGQKY;
VEMVKSVIKNGQKYF; EMVKSVIKNGQKYFD; MVKSVIKNGQKYFDG;
VKSVIKNGQKYFDGQ; KSVIKNGQKYFDGQV; SVIKNGQKYFDGQVT;
VIKNGQKYFDGQVTD; IKNGQKYFDGQVTDS; KNGQKYFDGQVTDSE;
NGQKYFDGQVTDSEL; GQKYFDGQVTDSELG; QKYFDGQVTDSELGI;
KYFDGQVTDSELGIR; YFDGQVTDSELGIRA; FDGQVTDSELGIRAI;
DGQVTDSELGIRAID; GQVTDSELGIRAIDE; QVTDSELGIRAIDEK;
VTDSELGIRAIDEKT; TDSELGIRAIDEKTL; DSELGIRAIDEKTLE;
SELGIRAIDEKTLEI; ELGIRAIDEKTLEIT; LGIRAIDEKTLEITL;
GIRAIDEKTLEITLE; IRAIDEKTLEITLES; RAIDEKTLEITLESP;
AIDEKTLEITLESPK; IDEKTLEITLESPKP; DEKTLEITLESPKPY;
EKTLEITLESPKPYF; KTLEITLESPKPYFI; TLEITLESPKPYFID;
LEITLESPKPYFIDM; EITLESPKPYFIDML; ITLESPKPYFIDMLV;
TLESPKPYFIDMLVH; LESPKPYFIDMLVHQ; ESPKPYFIDMLVHQS;
SPKPYFIDMLVHQSF; PKPYFIDMLVHQSFI; KPYFIDMLVHQSFIP;
PYFIDMLVHQSFIPV; YFIDMLVHQSFIPVP; FIDMLVHQSFIPVPV;
IDMLVHQSFIPVPVH; DMLVHQSFIPVPVHV; MLVHQSFIPVPVHVT;
LVHQSFIPVPVHVTE; VHQSFIPVPVHVTEK; HQSFIPVPVHVTEKY;
QSFIPVPVHVTEKYG; SFIPVPVHVTEKYGQ; FIPVPVHVTEKYGQN;
IPVPVHVTEKYGQNW; PVPVHVTEKYGQNWT; VPVHVTEKYGQNWTS;
PVHVTEKYGQNWTSP; VHVTEKYGQNWTSPE; HVTEKYGQNWTSPEN;
VTEKYGQNWTSPENM; TEKYGQNWTSPENMV; EKYGQNWTSPENMVT;
KYGQNWTSPENMVTS; YGQNWTSPENMVTSG; GQNWTSPENMVTSGP;
QNWTSPENMVTSGPF; NWTSPENMVTSGPFK; WTSPENMVTSGPFKL;
TSPENMVTSGPFKLK; SPENMVTSGPFKLKE; PENMVTSGPFKLKER;
ENMVTSGPFKLKERI; NMVTSGPFKLKERIP; MVTSGPFKLKERIPN;
VTSGPFKLKERIPNE; TSGPFKLKERIPNEK; SGPFKLKERIPNEKY;
GPFKLKERIPNEKYV; PFKLKERIPNEKYVF; FKLKERIPNEKYVFE;
KLKERIPNEKYVFEK; LKERIPNEKYVFEKN; KERIPNEKYVFEKNN;
ERIPNEKYVFEKNNK; RIPNEKYVFEKNNKY; IPNEKYVFEKNNKYY;
PNEKYVFEKNNKYYD; NEKYVFEKNNKYYDS; EKYVFEKNNKYYDSN;
KYVFEKNNKYYDSNE; YVFEKNNKYYDSNEV; VFEKNNKYYDSNEVE;
FEKNNKYYDSNEVEL; EKNNKYYDSNEVELE; KNNKYYDSNEVELEE;
NNKYYDSNEVELEEI; NKYYDSNEVELEEIT; KYYDSNEVELEEITF;

YYDSNEVELEEITFY; YDSNEVELEEITFYT; DSNEVELEEITFYTT;
SNEVELEEITFYTTN; NEVELEEITFYTTND; EVELEEITFYTTNDS;
VELEEITFYTTNDSS; ELEEITFYTTNDSST; LEEITFYTTNDSSTA;
EEITFYTTNDSSTAY; EITFYTTNDSSTAYK; ITFYTTNDSSTAYKM;
TFYTTNDSSTAYKMY; FYTTNDSSTAYKMYE; YTTNDSSTAYKMYEN;
TTNDSSTAYKMYENE; TNDSSTAYKMYENEE; NDSSTAYKMYENEEL;
DSSTAYKMYENEELD; SSTAYKMYENEELDA; STAYKMYENEELDAI;
TAYKMYENEELDAIF; AYKMYENEELDAIFG; YKMYENEELDAIFGS;
KMYENEELDAIFGSI; MYENEELDAIFGSIP; YENEELDAIFGSIPP;
ENEELDAIFGSIPPD; NEELDAIFGSIPPDL; EELDAIFGSIPPDLI;
ELDAIFGSIPPDLIK; LDAIFGSIPPDLIKN; DAIFGSIPPDLIKNL;
AIFGSIPPDLIKNLK; IFGSIPPDLIKNLKL; FGSIPPDLIKNLKLR;
GSIPPDLIKNLKLRS; SIPPDLIKNLKLRSD; IPPDLIKNLKLRSDY;
PPDLIKNLKLRSDYY; PDLIKNLKLRSDYYS; DLIKNLKLRSDYYSS;
LIKNLKLRSDYYSSA; IKNLKLRSDYYSSAV; KNLKLRSDYYSSAVN;
NLKLRSDYYSSAVNA; LKLRSDYYSSAVNAI; KLRSDYYSSAVNAIY;
LRSDYYSSAVNAIYF; RSDYYSSAVNAIYFY; SDYYSSAVNAIYFYA;
DYYSSAVNAIYFYAF; YYSSAVNAIYFYAFN; YSSAVNAIYFYAFNT;
SSAVNAIYFYAFNTH; SAVNAIYFYAFNTHI; AVNAIYFYAFNTHIK;
VNAIYFYAFNTHIKP; NAIYFYAFNTHIKPL; AIYFYAFNTHIKPLD;
IYFYAFNTHIKPLDN; YFYAFNTHIKPLDNV; FYAFNTHIKPLDNVK;
YAFNTHIKPLDNVKI; AFNTHIKPLDNVKIR; FNTHIKPLDNVKIRK;
NTHIKPLDNVKIRKA; THIKPLDNVKIRKAL; HIKPLDNVKIRKALT;
IKPLDNVKIRKALTL; KPLDNVKIRKALTLA; PLDNVKIRKALTLAI;
LDNVKIRKALTLAID; DNVKIRKALTLAIDR; NVKIRKALTLAIDRE;
VKIRKALTLAIDRET; KIRKALTLAIDRETL; IRKALTLAIDRETLT;
RKALTLAIDRETLTY; KALTLAIDRETLTYK; ALTLAIDRETLTYKV;
LTLAIDRETLTYKVL; TLAIDRETLTYKVLD; LAIDRETLTYKVLDN;
AIDRETLTYKVLDNG; IDRETLTYKVLDNGT; DRETLTYKVLDNGTT;
RETLTYKVLDNGTTP; ETLTYKVLDNGTTPT; TLTYKVLDNGTTPTR;
LTYKVLDNGTTPTRR; TYKVLDNGTTPTRRA; YKVLDNGTTPTRRAT;
KVLDNGTTPTRRATP; VLDNGTTPTRRATPN; LDNGTTPTRRATPNF;
DNGTTPTRRATPNFS; NGTTPTRRATPNFSS; GTTPTRRATPNFSSY;
TTPTRRATPNFSSYS; TPTRRATPNFSSYSY; PTRRATPNFSSYSYA;
TRRATPNFSSYSYAK; RRATPNFSSYSYAKS; RATPNFSSYSYAKSL;
ATPNFSSYSYAKSLE; TPNFSSYSYAKSLEL; PNFSSYSYAKSLELF;
NFSSYSYAKSLELFN; FSSYSYAKSLELFNP; SSYSYAKSLELFNPE;
SYSYAKSLELFNPEI; YSYAKSLELFNPEIA; SYAKSLELFNPEIAK;
YAKSLELFNPEIAKT; AKSLELFNPEIAKTL; KSLELFNPEIAKTLL;
SLELFNPEIAKTLLA; LELFNPEIAKTLLAE; ELFNPEIAKTLLAEA;
LFNPEIAKTLLAEAG; FNPEIAKTLLAEAGY; NPEIAKTLLAEAGYP;
PEIAKTLLAEAGYPN; EIAKTLLAEAGYPNG; IAKTLLAEAGYPNGN;
AKTLLAEAGYPNGNG; KTLLAEAGYPNGNGF; TLLAEAGYPNGNGFP;
LLAEAGYPNGNGFPI; LAEAGYPNGNGFPIL; AEAGYPNGNGFPILK;
EAGYPNGNGFPILKL; AGYPNGNGFPILKLK; GYPNGNGFPILKLKY;
YPNGNGFPILKLKYN; PNGNGFPILKLKYNT; NGNGFPILKLKYNTN;
GNGFPILKLKYNTNE; NGFPILKLKYNTNEA; GFPILKLKYNTNEAN;
FPILKLKYNTNEANK; PILKLKYNTNEANKK; ILKLKYNTNEANKKI;
LKLKYNTNEANKKIC; KLKYNTNEANKKICE; LKYNTNEANKKICEF;
KYNTNEANKKICEFI; YNTNEANKKICEFIQ; NTNEANKKICEFIQN;
TNEANKKICEFIQNQ; NEANKKICEFIQNQW; EANKKICEFIQNQWK;

ANKKICEFIQNQWKK; NKKICEFIQNQWKKN; KKICEFIQNQWKKNL;
KICEFIQNQWKKNLN; ICEFIQNQWKKNLNI; CEFIQNQWKKNLNID;
EFIQNQWKKNLNIDV; FIQNQWKKNLNIDVE; IQNQWKKNLNIDVEL;
QNQWKKNLNIDVELE; NQWKKNLNIDVELEN; QWKKNLNIDVELENE;
WKKNLNIDVELENEE; KKNLNIDVELENEEW; KNLNIDVELENEEWT;
NLNIDVELENEEWTT; LNIDVELENEEWTTY; NIDVELENEEWTTYL;
IDVELENEEWTTYLN; DVELENEEWTTYLNT; VELENEEWTTYLNTK;
ELENEEWTTYLNTKA; LENEEWTTYLNTKAN; ENEEWTTYLNTKANG;
NEEWTTYLNTKANGN; EEWTTYLNTKANGNY; EWTTYLNTKANGNYE;
WTTYLNTKANGNYEI; TTYLNTKANGNYEIA; TYLNTKANGNYEIAR;
YLNTKANGNYEIARA; LNTKANGNYEIARAG; NTKANGNYEIARAGW;
TKANGNYEIARAGWI; KANGNYEIARAGWIG; ANGNYEIARAGWIGD;
NGNYEIARAGWIGDY; GNYEIARAGWIGDYA; NYEIARAGWIGDYAD;
YEIARAGWIGDYADP; EIARAGWIGDYADPL; IARAGWIGDYADPLT;
ARAGWIGDYADPLTF; RAGWIGDYADPLTFL; AGWIGDYADPLTFLS;
GWIGDYADPLTFLSI; WIGDYADPLTFLSIF; IGDYADPLTFLSIFT;
GDYADPLTFLSIFTQ; DYADPLTFLSIFTQG; YADPLTFLSIFTQGY;
ADPLTFLSIFTQGYT; DPLTFLSIFTQGYTQ; PLTFLSIFTQGYTQF;
LTFLSIFTQGYTQFS; TFLSIFTQGYTQFSS; FLSIFTQGYTQFSSH;
LSIFTQGYTQFSSHN; SIFTQGYTQFSSHNY; IFTQGYTQFSSHNYS;
FTQGYTQFSSHNYSN; TQGYTQFSSHNYSNP; QGYTQFSSHNYSNPE;
GYTQFSSHNYSNPEY; YTQFSSHNYSNPEYN; TQFSSHNYSNPEYNE;
QFSSHNYSNPEYNEL; FSSHNYSNPEYNELI; SSHNYSNPEYNELIK;
SHNYSNPEYNELIKK; HNYSNPEYNELIKKS; NYSNPEYNELIKKSD;
YSNPEYNELIKKSDL; SNPEYNELIKKSDLE; NPEYNELIKKSDLEL;
PEYNELIKKSDLELD; EYNELIKKSDLELDP; YNELIKKSDLELDPI;
NELIKKSDLELDPIK; ELIKKSDLELDPIKR; LIKKSDLELDPIKRQ;
IKKSDLELDPIKRQD; KKSDLELDPIKRQDI; KSDLELDPIKRQDIL;
SDLELDPIKRQDILR; DLELDPIKRQDILRQ; LELDPIKRQDILRQA;
ELDPIKRQDILRQAE; LDPIKRQDILRQAEE; DPIKRQDILRQAEEI;
PIKRQDILRQAEEII; IKRQDILRQAEEIII; KRQDILRQAEEIIIE;
RQDILRQAEEIIIEK; QDILRQAEEIIIEKD; DILRQAEEIIIEKDF;
ILRQAEEIIIEKDFP; LRQAEEIIIEKDFPI; RQAEEIIIEKDFPIA;
QAEEIIIEKDFPIAP; AEEIIIEKDFPIAPI; EEIIIEKDFPIAPIY;
EIIIEKDFPIAPIYI; IIIEKDFPIAPIYIY; IIEKDFPIAPIYIYG;
IEKDFPIAPIYIYGN; EKDFPIAPIYIYGNS; KDFPIAPIYIYGNSY;
DFPIAPIYIYGNSYL; FPIAPIYIYGNSYLF; PIAPIYIYGNSYLFR;
IAPIYIYGNSYLFRN; APIYIYGNSYLFRND; PIYIYGNSYLFRNDK;
IYIYGNSYLFRNDKW; YIYGNSYLFRNDKWT; IYGNSYLFRNDKWTG;
YGNSYLFRNDKWTGW; GNSYLFRNDKWTGWN; NSYLFRNDKWTGWNT;
SYLFRNDKWTGWNTN; YLFRNDKWTGWNTNI; LFRNDKWTGWNTNIL;
FRNDKWTGWNTNILE; RNDKWTGWNTNILER; NDKWTGWNTNILERF;
DKWTGWNTNILERFD; KWTGWNTNILERFDL; WTGWNTNILERFDLS;
TGWNTNILERFDLSQ; GWNTNILERFDLSQL; WNTNILERFDLSQLK;
NTNILERFDLSQLKL; TNILERFDLSQLKLK; NILERFDLSQLKLKN;
ILERFDLSQLKLKNK;

16 mers:
MKLQRSLFLIIFFLTF; KLQRSLFLIIFFLTFL; LQRSLFLIIFFLTFLC;
QRSLFLIIFFLTFLCC; RSLFLIIFFLTFLCCN; SLFLIIFFLTFLCCNN;
LFLIIFFLTFLCCNNK; FLIIFFLTFLCCNNKE; LIIFFLTFLCCNNKER;

IIFFLTFLCCNNKERK; IFFLTFLCCNNKERKE; FFLTFLCCNNKERKEG;
FLTFLCCNNKERKEGV; LTFLCCNNKERKEGVS; TFLCCNNKERKEGVSF;
FLCCNNKERKEGVSFK; LCCNNKERKEGVSFKI; CCNNKERKEGVSFKIS;
CNNKERKEGVSFKISL; NNKERKEGVSFKISLG; NKERKEGVSFKISLGA;
KERKEGVSFKISLGAE; ERKEGVSFKISLGAEP; RKEGVSFKISLGAEPS;
KEGVSFKISLGAEPSS; EGVSFKISLGAEPSSL; GVSFKISLGAEPSSLD;
VSFKISLGAEPSSLDP; SFKISLGAEPSSLDPQ; FKISLGAEPSSLDPQL;
KISLGAEPSSLDPQLA; ISLGAEPSSLDPQLAE; SLGAEPSSLDPQLAED;
LGAEPSSLDPQLAEDN; GAEPSSLDPQLAEDNV; AEPSSLDPQLAEDNVA;
EPSSLDPQLAEDNVAS; PSSLDPQLAEDNVASK; SSLDPQLAEDNVASKM;
SLDPQLAEDNVASKMI; LDPQLAEDNVASKMID; DPQLAEDNVASKMIDT;
PQLAEDNVASKMIDTM; QLAEDNVASKMIDTMF; LAEDNVASKMIDTMFR;
AEDNVASKMIDTMFRG; EDNVASKMIDTMFRGI; DNVASKMIDTMFRGIV;
NVASKMIDTMFRGIVT; VASKMIDTMFRGIVTG; ASKMIDTMFRGIVTGD;
SKMIDTMFRGIVTGDP; KMIDTMFRGIVTGDPN; MIDTMFRGIVTGDPNT;
IDTMFRGIVTGDPNTG; DTMFRGIVTGDPNTGG; TMFRGIVTGDPNTGGN;
MFRGIVTGDPNTGGNK; FRGIVTGDPNTGGNKP; RGIVTGDPNTGGNKPG;
GIVTGDPNTGGNKPGL; IVTGDPNTGGNKPGLA; VTGDPNTGGNKPGLAK;
TGDPNTGGNKPGLAKG; GDPNTGGNKPGLAKGW; DPNTGGNKPGLAKGWD;
PNTGGNKPGLAKGWDI; NTGGNKPGLAKGWDIS; TGGNKPGLAKGWDISS;
GGNKPGLAKGWDISSD; GNKPGLAKGWDISSDG; NKPGLAKGWDISSDGT;
KPGLAKGWDISSDGTV; PGLAKGWDISSDGTVY; GLAKGWDISSDGTVYT;
LAKGWDISSDGTVYTF; AKGWDISSDGTVYTFN; KGWDISSDGTVYTFNL;
GWDISSDGTVYTFNLR; WDISSDGTVYTFNLRE; DISSDGTVYTFNLREK;
ISSDGTVYTFNLREKI; SSDGTVYTFNLREKIT; SDGTVYTFNLREKITW;
DGTVYTFNLREKITWS; GTVYTFNLREKITWSD; TVYTFNLREKITWSDG;
VYTFNLREKITWSDGV; YTFNLREKITWSDGVA; TFNLREKITWSDGVAI;
FNLREKITWSDGVAIT; NLREKITWSDGVAITA; LREKITWSDGVAITAE;
REKITWSDGVAITAEG; EKITWSDGVAITAEGI; KITWSDGVAITAEGIR;
ITWSDGVAITAEGIRK; TWSDGVAITAEGIRKS; WSDGVAITAEGIRKSY;
SDGVAITAEGIRKSYL; DGVAITAEGIRKSYLR; GVAITAEGIRKSYLRI;
VAITAEGIRKSYLRIL; AITAEGIRKSYLRILN; ITAEGIRKSYLRILNK;
TAEGIRKSYLRILNKE; AEGIRKSYLRILNKET; EGIRKSYLRILNKETG;
GIRKSYLRILNKETGS; IRKSYLRILNKETGSK; RKSYLRILNKETGSKY;
KSYLRILNKETGSKYV; SYLRILNKETGSKYVE; YLRILNKETGSKYVEM;
LRILNKETGSKYVEMV; RILNKETGSKYVEMVK; ILNKETGSKYVEMVKS;
LNKETGSKYVEMVKSV; NKETGSKYVEMVKSVI; KETGSKYVEMVKSVIK;
ETGSKYVEMVKSVIKN; TGSKYVEMVKSVIKNG; GSKYVEMVKSVIKNGQ;
SKYVEMVKSVIKNGQK; KYVEMVKSVIKNGQKY; YVEMVKSVIKNGQKYF;
VEMVKSVIKNGQKYFD; EMVKSVIKNGQKYFDG; MVKSVIKNGQKYFDGQ;
VKSVIKNGQKYFDGQV; KSVIKNGQKYFDGQVT; SVIKNGQKYFDGQVTD;
VIKNGQKYFDGQVTDS; IKNGQKYFDGQVTDSE; KNGQKYFDGQVTDSEL;
NGQKYFDGQVTDSELG; GQKYFDGQVTDSELGI; QKYFDGQVTDSELGIR;
KYFDGQVTDSELGIRA; YFDGQVTDSELGIRAI; FDGQVTDSELGIRAID;
DGQVTDSELGIRAIDE; GQVTDSELGIRAIDEK; QVTDSELGIRAIDEKT;
VTDSELGIRAIDEKTL; TDSELGIRAIDEKTLE; DSELGIRAIDEKTLEI;
SELGIRAIDEKTLEIT; ELGIRAIDEKTLEITL; LGIRAIDEKTLEITLE;
GIRAIDEKTLEITLES; IRAIDEKTLEITLESP; RAIDEKTLEITLESPK;
AIDEKTLEITLESPKP; IDEKTLEITLESPKPY; DEKTLEITLESPKPYF;
EKTLEITLESPKPYFI; KTLEITLESPKPYFID; TLEITLESPKPYFIDM;
LEITLESPKPYFIDML; EITLESPKPYFIDMLV; ITLESPKPYFIDMLVH;

TLESPKPYFIDMLVHQ; LESPKPYFIDMLVHQS; ESPKPYFIDMLVHQSF;
SPKPYFIDMLVHQSFI; PKPYFIDMLVHQSFIP; KPYFIDMLVHQSFIPV;
PYFIDMLVHQSFIPVP; YFIDMLVHQSFIPVPV; FIDMLVHQSFIPVPVH;
IDMLVHQSFIPVPVHV; DMLVHQSFIPVPVHVT; MLVHQSFIPVPVHVTE;
LVHQSFIPVPVHVTEK; VHQSFIPVPVHVTEKY; HQSFIPVPVHVTEKYG;
QSFIPVPVHVTEKYGQ; SFIPVPVHVTEKYGQN; FIPVPVHVTEKYGQNW;
IPVPVHVTEKYGQNWT; PVPVHVTEKYGQNWTS; VPVHVTEKYGQNWTSP;
PVHVTEKYGQNWTSPE; VHVTEKYGQNWTSPEN; HVTEKYGQNWTSPENM;
VTEKYGQNWTSPENMV; TEKYGQNWTSPENMVT; EKYGQNWTSPENMVTS;
KYGQNWTSPENMVTSG; YGQNWTSPENMVTSGP; GQNWTSPENMVTSGPF;
QNWTSPENMVTSGPFK; NWTSPENMVTSGPFKL; WTSPENMVTSGPFKLK;
TSPENMVTSGPFKLKE; SPENMVTSGPFKLKER; PENMVTSGPFKLKERI;
ENMVTSGPFKLKERIP; NMVTSGPFKLKERIPN; MVTSGPFKLKERIPNE;
VTSGPFKLKERIPNEK; TSGPFKLKERIPNEKY; SGPFKLKERIPNEKYV;
GPFKLKERIPNEKYVF; PFKLKERIPNEKYVFE; FKLKERIPNEKYVFEK;
KLKERIPNEKYVFEKN; LKERIPNEKYVFEKNN; KERIPNEKYVFEKNNK;
ERIPNEKYVFEKNNKY; RIPNEKYVFEKNNKYY; IPNEKYVFEKNNKYYD;
PNEKYVFEKNNKYYDS; NEKYVFEKNNKYYDSN; EKYVFEKNNKYYDSNE;
KYVFEKNNKYYDSNEV; YVFEKNNKYYDSNEVE; VFEKNNKYYDSNEVEL;
FEKNNKYYDSNEVELE; EKNNKYYDSNEVELEE; KNNKYYDSNEVELEEI;
NNKYYDSNEVELEEIT; NKYYDSNEVELEEITF; KYYDSNEVELEEITFY;
YYDSNEVELEEITFYT; YDSNEVELEEITFYTT; DSNEVELEEITFYTTN;
SNEVELEEITFYTTND; NEVELEEITFYTTNDS; EVELEEITFYTTNDSS;
VELEEITFYTTNDSST; ELEEITFYTTNDSSTA; LEEITFYTTNDSSTAY;
EEITFYTTNDSSTAYK; EITFYTTNDSSTAYKM; ITFYTTNDSSTAYKMY;
TFYTTNDSSTAYKMYE; FYTTNDSSTAYKMYEN; YTTNDSSTAYKMYENE;
TTNDSSTAYKMYENEE; TNDSSTAYKMYENEEL; NDSSTAYKMYENEELD;
DSSTAYKMYENEELDA; SSTAYKMYENEELDAI; STAYKMYENEELDAIF;
TAYKMYENEELDAIFG; AYKMYENEELDAIFGS; YKMYENEELDAIFGSI;
KMYENEELDAIFGSIP; MYENEELDAIFGSIPP; YENEELDAIFGSIPPD;
ENEELDAIFGSIPPDL; NEELDAIFGSIPPDLI; EELDAIFGSIPPDLIK;
ELDAIFGSIPPDLIKN; LDAIFGSIPPDLIKNL; DAIFGSIPPDLIKNLK;
AIFGSIPPDLIKNLKL; IFGSIPPDLIKNLKLR; FGSIPPDLIKNLKLRS;
GSIPPDLIKNLKLRSD; SIPPDLIKNLKLRSDY; IPPDLIKNLKLRSDYY;
PPDLIKNLKLRSDYYS; PDLIKNLKLRSDYYSS; DLIKNLKLRSDYYSSA;
LIKNLKLRSDYYSSAV; IKNLKLRSDYYSSAVN; KNLKLRSDYYSSAVNA;
NLKLRSDYYSSAVNAI; LKLRSDYYSSAVNAIY; KLRSDYYSSAVNAIYF;
LRSDYYSSAVNAIYFY; RSDYYSSAVNAIYFYA; SDYYSSAVNAIYFYAF;
DYYSSAVNAIYFYAFN; YYSSAVNAIYFYAFNT; YSSAVNAIYFYAFNTH;
SSAVNAIYFYAFNTHI; SAVNAIYFYAFNTHIK; AVNAIYFYAFNTHIKP;
VNAIYFYAFNTHIKPL; NAIYFYAFNTHIKPLD; AIYFYAFNTHIKPLDN;
IYFYAFNTHIKPLDNV; YFYAFNTHIKPLDNVK; FYAFNTHIKPLDNVKI;
YAFNTHIKPLDNVKIR; AFNTHIKPLDNVKIRK; FNTHIKPLDNVKIRKA;
NTHIKPLDNVKIRKAL; THIKPLDNVKIRKALT; HIKPLDNVKIRKALTL;
IKPLDNVKIRKALTLA; KPLDNVKIRKALTLAI; PLDNVKIRKALTLAID;
LDNVKIRKALTLAIDR; DNVKIRKALTLAIDRE; NVKIRKALTLAIDRET;
VKIRKALTLAIDRETL; KIRKALTLAIDRETLT; IRKALTLAIDRETLTY;
RKALTLAIDRETLTYK; KALTLAIDRETLTYKV; ALTLAIDRETLTYKVL;
LTLAIDRETLTYKVLD; TLAIDRETLTYKVLDN; LAIDRETLTYKVLDNG;
AIDRETLTYKVLDNGT; IDRETLTYKVLDNGTT; DRETLTYKVLDNGTTP;
RETLTYKVLDNGTTPT; ETLTYKVLDNGTTPTR; TLTYKVLDNGTTPTRR;

LTYKVLDNGTTPTRRA; TYKVLDNGTTPTRRAT; YKVLDNGTTPTRRATP;
KVLDNGTTPTRRATPN; VLDNGTTPTRRATPNF; LDNGTTPTRRATPNFS;
DNGTTPTRRATPNFSS; NGTTPTRRATPNFSSY; GTTPTRRATPNFSSYS;
TTPTRRATPNFSSYSY; TPTRRATPNFSSYSYA; PTRRATPNFSSYSYAK;
TRRATPNFSSYSYAKS; RRATPNFSSYSYAKSL; RATPNFSSYSYAKSLE;
ATPNFSSYSYAKSLEL; TPNFSSYSYAKSLELF; PNFSSYSYAKSLELFN;
NFSSYSYAKSLELFNP; FSSYSYAKSLELFNPE; SSYSYAKSLELFNPEI;
SYSYAKSLELFNPEIA; YSYAKSLELFNPEIAK; SYAKSLELFNPEIAKT;
YAKSLELFNPEIAKTL; AKSLELFNPEIAKTLL; KSLELFNPEIAKTLLA;
SLELFNPEIAKTLLAE; LELFNPEIAKTLLAEA; ELFNPEIAKTLLAEAG;
LFNPEIAKTLLAEAGY; FNPEIAKTLLAEAGYP; NPEIAKTLLAEAGYPN;
PEIAKTLLAEAGYPNG; EIAKTLLAEAGYPNGN; IAKTLLAEAGYPNGNG;
AKTLLAEAGYPNGNGF; KTLLAEAGYPNGNGFP; TLLAEAGYPNGNGFPI;
LLAEAGYPNGNGFPIL; LAEAGYPNGNGFPILK; AEAGYPNGNGFPILKL;
EAGYPNGNGFPILKLK; AGYPNGNGFPILKLKY; GYPNGNGFPILKLKYN;
YPNGNGFPILKLKYNT; PNGNGFPILKLKYNTN; NGNGFPILKLKYNTNE;
GNGFPILKLKYNTNEA; NGFPILKLKYNTNEAN; GFPILKLKYNTNEANK;
FPILKLKYNTNEANKK; PILKLKYNTNEANKKI; ILKLKYNTNEANKKIC;
LKLKYNTNEANKKICE; KLKYNTNEANKKICEF; LKYNTNEANKKICEFI;
KYNTNEANKKICEFIQ; YNTNEANKKICEFIQN; NTNEANKKICEFIQNQ;
TNEANKKICEFIQNQW; NEANKKICEFIQNQWK; EANKKICEFIQNQWKK;
ANKKICEFIQNQWKKN; NKKICEFIQNQWKKNL; KKICEFIQNQWKKNLN;
KICEFIQNQWKKNLNI; ICEFIQNQWKKNLNID; CEFIQNQWKKNLNIDV;
EFIQNQWKKNLNIDVE; FIQNQWKKNLNIDVEL; IQNQWKKNLNIDVELE;
QNQWKKNLNIDVELEN; NQWKKNLNIDVELENE; QWKKNLNIDVELENEE;
WKKNLNIDVELENEEW; KKNLNIDVELENEEWT; KNLNIDVELENEEWTT;
NLNIDVELENEEWTTY; LNIDVELENEEWTTYL; NIDVELENEEWTTYLN;
IDVELENEEWTTYLNT; DVELENEEWTTYLNTK; VELENEEWTTYLNTKA;
ELENEEWTTYLNTKAN; LENEEWTTYLNTKANG; ENEEWTTYLNTKANGN;
NEEWTTYLNTKANGNY; EEWTTYLNTKANGNYE; EWTTYLNTKANGNYEI;
WTTYLNTKANGNYEIA; TTYLNTKANGNYEIAR; TYLNTKANGNYEIARA;
YLNTKANGNYEIARAG; LNTKANGNYEIARAGW; NTKANGNYEIARAGWI;
TKANGNYEIARAGWIG; KANGNYEIARAGWIGD; ANGNYEIARAGWIGDY;
NGNYEIARAGWIGDYA; GNYEIARAGWIGDYAD; NYEIARAGWIGDYADP;
YEIARAGWIGDYADPL; EIARAGWIGDYADPLT; IARAGWIGDYADPLTF;
ARAGWIGDYADPLTFL; RAGWIGDYADPLTFLS; AGWIGDYADPLTFLSI;
GWIGDYADPLTFLSIF; WIGDYADPLTFLSIFT; IGDYADPLTFLSIFTQ;
GDYADPLTFLSIFTQG; DYADPLTFLSIFTQGY; YADPLTFLSIFTQGYT;
ADPLTFLSIFTQGYTQ; DPLTFLSIFTQGYTQF; PLTFLSIFTQGYTQFS;
LTFLSIFTQGYTQFSS; TFLSIFTQGYTQFSSH; FLSIFTQGYTQFSSHN;
LSIFTQGYTQFSSHNY; SIFTQGYTQFSSHNYS; IFTQGYTQFSSHNYSN;
FTQGYTQFSSHNYSNP; TQGYTQFSSHNYSNPE; QGYTQFSSHNYSNPEY;
GYTQFSSHNYSNPEYN; YTQFSSHNYSNPEYNE; TQFSSHNYSNPEYNEL;
QFSSHNYSNPEYNELI; FSSHNYSNPEYNELIK; SSHNYSNPEYNELIKK;
SHNYSNPEYNELIKKS; HNYSNPEYNELIKKSD; NYSNPEYNELIKKSDL;
YSNPEYNELIKKSDLE; SNPEYNELIKKSDLEL; NPEYNELIKKSDLELD;
PEYNELIKKSDLELDP; EYNELIKKSDLELDPI; YNELIKKSDLELDPIK;
NELIKKSDLELDPIKR; ELIKKSDLELDPIKRQ; LIKKSDLELDPIKRQD;
IKKSDLELDPIKRQDI; KKSDLELDPIKRQDIL; KSDLELDPIKRQDILR;
SDLELDPIKRQDILRQ; DLELDPIKRQDILRQA; LELDPIKRQDILRQAE;
ELDPIKRQDILRQAEE; LDPIKRQDILRQAEEI; DPIKRQDILRQAEEII;

| | |
|---|---|
| | PIKRQDILRQAEEIII; IKRQDILRQAEEIIIE; KRQDILRQAEEIIIEK; RQDILRQAEEIIIEKD; QDILRQAEEIIIEKDF; DILRQAEEIIIEKDFP; ILRQAEEIIIEKDFPI; LRQAEEIIIEKDFPIA; RQAEEIIIEKDFPIAP; QAEEIIIEKDFPIAPI; AEEIIIEKDFPIAPIY; EEIIIEKDFPIAPIYI; EIIIEKDFPIAPIYIY; IIIEKDFPIAPIYIYG; IIEKDFPIAPIYIYGN; IEKDFPIAPIYIYGNS; EKDFPIAPIYIYGNSY; KDFPIAPIYIYGNSYL; DFPIAPIYIYGNSYLF; FPIAPIYIYGNSYLFR; PIAPIYIYGNSYLFRN; IAPIYIYGNSYLFRND; APIYIYGNSYLFRNDK; PIYIYGNSYLFRNDKW; IYIYGNSYLFRNDKWT; YIYGNSYLFRNDKWTG; IYGNSYLFRNDKWTGW; YGNSYLFRNDKWTGWN; GNSYLFRNDKWTGWNT; NSYLFRNDKWTGWNTN; SYLFRNDKWTGWNTNI; YLFRNDKWTGWNTNIL; LFRNDKWTGWNTNILE; FRNDKWTGWNTNILER; RNDKWTGWNTNILERF; NDKWTGWNTNILERFD; DKWTGWNTNILERFDL; KWTGWNTNILERFDLS; WTGWNTNILERFDLSQ; TGWNTNILERFDLSQL; GWNTNILERFDLSQLK; WNTNILERFDLSQLKL; NTNILERFDLSQLKLK; TNILERFDLSQLKLKN; NILERFDLSQLKLKNK; |
| Seq 25 | 13 mers: MTKIFSNLIINGL; TKIFSNLIINGLL; KIFSNLIINGLLF; IFSNLIINGLLFG; FSNLIINGLLFGF; SNLIINGLLFGFV; NLIINGLLFGFVS; LIINGLLFGFVSL; IINGLLFGFVSLN; INGLLFGFVSLNV; NGLLFGFVSLNVF; GLLFGFVSLNVFA; LLFGFVSLNVFAD; LFGFVSLNVFADS; FGFVSLNVFADSN; GFVSLNVFADSNN; FVSLNVFADSNNA; VSLNVFADSNNAN; SLNVFADSNNANI; LNVFADSNNANIL; NVFADSNNANILK; VFADSNNANILKP; FADSNNANILKPQ; ADSNNANILKPQS; DSNNANILKPQSN; SNNANILKPQSNV; NNANILKPQSNVL; NANILKPQSNVLE; ANILKPQSNVLEH; NILKPQSNVLEHS; ILKPQSNVLEHSD; LKPQSNVLEHSDQ; KPQSNVLEHSDQK; PQSNVLEHSDQKD; QSNVLEHSDQKDN; SNVLEHSDQKDNK; NVLEHSDQKDNKK; VLEHSDQKDNKKL; LEHSDQKDNKKLD; EHSDQKDNKKLDQ; HSDQKDNKKLDQK; SDQKDNKKLDQKD; DQKDNKKLDQKDQ; QKDNKKLDQKDQV; KDNKKLDQKDQVN; DNKKLDQKDQVNQ; NKKLDQKDQVNQA; KKLDQKDQVNQAL; KLDQKDQVNQALD; LDQKDQVNQALDT; DQKDQVNQALDTI; QKDQVNQALDTIN; KDQVNQALDTINK; DQVNQALDTINKV; QVNQALDTINKVT; VNQALDTINKVTE; NQALDTINKVTED; QALDTINKVTEDV; ALDTINKVTEDVS; LDTINKVTEDVSS; DTINKVTEDVSSK; TINKVTEDVSSKL; INKVTEDVSSKLE; NKVTEDVSSKLEG; KVTEDVSSKLEGV; VTEDVSSKLEGVR; TEDVSSKLEGVRE; EDVSSKLEGVRES; DVSSKLEGVRESS; VSSKLEGVRESSL; SSKLEGVRESSLE; SKLEGVRESSLEL; KLEGVRESSLELV; LEGVRESSLELVE; EGVRESSLELVES; GVRESSLELVESN; VRESSLELVESND; RESSLELVESNDA; ESSLELVESNDAG; SSLELVESNDAGV; SLELVESNDAGVV; LELVESNDAGVVK; ELVESNDAGVVKK; LVESNDAGVVKKF; VESNDAGVVKKFV; ESNDAGVVKKFVG; SNDAGVVKKFVGS; NDAGVVKKFVGSM; DAGVVKKFVGSMS; AGVVKKFVGSMSL; GVVKKFVGSMSLM; VVKKFVGSMSLMS; VKKFVGSMSLMSD; KKFVGSMSLMSDV; KFVGSMSLMSDVA; FVGSMSLMSDVAK; VGSMSLMSDVAKG; GSMSLMSDVAKGT; SMSLMSDVAKGTV; |

MSLMSDVAKGTVV; SLMSDVAKGTVVA; LMSDVAKGTVVAS;
MSDVAKGTVVASQ; SDVAKGTVVASQE; DVAKGTVVASQEA;
VAKGTVVASQEAT; AKGTVVASQEATI; KGTVVASQEATIV;
GTVVASQEATIVA; TVVASQEATIVAK; VVASQEATIVAKC;
VASQEATIVAKCS; ASQEATIVAKCSG; SQEATIVAKCSGM;
QEATIVAKCSGMV; EATIVAKCSGMVA; ATIVAKCSGMVAE;
TIVAKCSGMVAEG; IVAKCSGMVAEGA; VAKCSGMVAEGAN;
AKCSGMVAEGANK; KCSGMVAEGANKV; CSGMVAEGANKVV;
SGMVAEGANKVVE; GMVAEGANKVVEM; MVAEGANKVVEMS;
VAEGANKVVEMSK; AEGANKVVEMSKK; EGANKVVEMSKKA;
GANKVVEMSKKAV; ANKVVEMSKKAVQ; NKVVEMSKKAVQE;
KVVEMSKKAVQET; VVEMSKKAVQETQ; VEMSKKAVQETQK;
EMSKKAVQETQKA; MSKKAVQETQKAV; SKKAVQETQKAVS;
KKAVQETQKAVSV; KAVQETQKAVSVA; AVQETQKAVSVAG;
VQETQKAVSVAGE; QETQKAVSVAGEA; ETQKAVSVAGEAT;
TQKAVSVAGEATF; QKAVSVAGEATFL; KAVSVAGEATFLI;
AVSVAGEATFLIE; VSVAGEATFLIEK; SVAGEATFLIEKQ;
VAGEATFLIEKQI; AGEATFLIEKQIM; GEATFLIEKQIML;
EATFLIEKQIMLN; ATFLIEKQIMLNK; TFLIEKQIMLNKS;
FLIEKQIMLNKSP; LIEKQIMLNKSPN; IEKQIMLNKSPNN;
EKQIMLNKSPNNK; KQIMLNKSPNNKE; QIMLNKSPNNKEL;
IMLNKSPNNKELE; MLNKSPNNKELEL; LNKSPNNKELELT;
NKSPNNKELELTK; KSPNNKELELTKE; SPNNKELELTKEE;
PNNKELELTKEEF; NNKELELTKEEFA; NKELELTKEEFAK;
KELELTKEEFAKV; ELELTKEEFAKVD; LELTKEEFAKVDE;
ELTKEEFAKVDEV; LTKEEFAKVDEVK; TKEEFAKVDEVKE;
KEEFAKVDEVKET; EEFAKVDEVKETL; EFAKVDEVKETLM;
FAKVDEVKETLMA; AKVDEVKETLMAS; KVDEVKETLMASE;
VDEVKETLMASER; DEVKETLMASERA; EVKETLMASERAL;
VKETLMASERALD; KETLMASERALDE; ETLMASERALDET;
TLMASERALDETV; LMASERALDETVQ; MASERALDETVQE;
ASERALDETVQEA; SERALDETVQEAQ; ERALDETVQEAQK;
RALDETVQEAQKV; ALDETVQEAQKVL; LDETVQEAQKVLN;
DETVQEAQKVLNM; ETVQEAQKVLNMV; TVQEAQKVLNMVN;
VQEAQKVLNMVNG; QEAQKVLNMVNGL; EAQKVLNMVNGLN;
AQKVLNMVNGLNP; QKVLNMVNGLNPS; KVLNMVNGLNPSN;
VLNMVNGLNPSNK; LNMVNGLNPSNKD; NMVNGLNPSNKDQ;
MVNGLNPSNKDQV; VNGLNPSNKDQVL; NGLNPSNKDQVLA;
GLNPSNKDQVLAK; LNPSNKDQVLAKK; NPSNKDQVLAKKD;
PSNKDQVLAKKDV; SNKDQVLAKKDVR; NKDQVLAKKDVRK;
KDQVLAKKDVRKA; DQVLAKKDVRKAI; QVLAKKDVRKAIS;
VLAKKDVRKAISN; LAKKDVRKAISNV; AKKDVRKAISNVV;
KKDVRKAISNVVK; KDVRKAISNVVKV; DVRKAISNVVKVA;
VRKAISNVVKVAQ; RKAISNVVKVAQG; KAISNVVKVAQGA;
AISNVVKVAQGAR; ISNVVKVAQGARD; SNVVKVAQGARDL;
NVVKVAQGARDLT; VVKVAQGARDLTK; VKVAQGARDLTKV;
KVAQGARDLTKVM; VAQGARDLTKVMA; AQGARDLTKVMAI;
QGARDLTKVMAIS; GARDLTKVMAISL; ARDLTKVMAISLY;
RDLTKVMAISLYM; DLTKVMAISLYMR;

14 mers:

| | | |
|---|---|---|
| MTKIFSNLIINGLL; | TKIFSNLIINGLLF; | KIFSNLIINGLLFG; |
| IFSNLIINGLLFGF; | FSNLIINGLLFGFV; | SNLIINGLLFGFVS; |
| NLIINGLLFGFVSL; | LIINGLLFGFVSLN; | IINGLLFGFVSLNV; |
| INGLLFGFVSLNVF; | NGLLFGFVSLNVFA; | GLLFGFVSLNVFAD; |
| LLFGFVSLNVFADS; | LFGFVSLNVFADSN; | FGFVSLNVFADSNN; |
| GFVSLNVFADSNNA; | FVSLNVFADSNNAN; | VSLNVFADSNNANI; |
| SLNVFADSNNANIL; | LNVFADSNNANILK; | NVFADSNNANILKP; |
| VFADSNNANILKPQ; | FADSNNANILKPQS; | ADSNNANILKPQSN; |
| DSNNANILKPQSNV; | SNNANILKPQSNVL; | NNANILKPQSNVLE; |
| NANILKPQSNVLEH; | ANILKPQSNVLEHS; | NILKPQSNVLEHSD; |
| ILKPQSNVLEHSDQ; | LKPQSNVLEHSDQK; | KPQSNVLEHSDQKD; |
| PQSNVLEHSDQKDN; | QSNVLEHSDQKDNK; | SNVLEHSDQKDNKK; |
| NVLEHSDQKDNKKL; | VLEHSDQKDNKKLD; | LEHSDQKDNKKLDQ; |
| EHSDQKDNKKLDQK; | HSDQKDNKKLDQKD; | SDQKDNKKLDQKDQ; |
| DQKDNKKLDQKDQV; | QKDNKKLDQKDQVN; | KDNKKLDQKDQVNQ; |
| DNKKLDQKDQVNQA; | NKKLDQKDQVNQAL; | KKLDQKDQVNQALD; |
| KLDQKDQVNQALDT; | LDQKDQVNQALDTI; | DQKDQVNQALDTIN; |
| QKDQVNQALDTINK; | KDQVNQALDTINKV; | DQVNQALDTINKVT; |
| QVNQALDTINKVTE; | VNQALDTINKVTED; | NQALDTINKVTEDV; |
| QALDTINKVTEDVS; | ALDTINKVTEDVSS; | LDTINKVTEDVSSK; |
| DTINKVTEDVSSKL; | TINKVTEDVSSKLE; | INKVTEDVSSKLEG; |
| NKVTEDVSSKLEGV; | KVTEDVSSKLEGVR; | VTEDVSSKLEGVRE; |
| TEDVSSKLEGVRES; | EDVSSKLEGVRESS; | DVSSKLEGVRESSL; |
| VSSKLEGVRESSLE; | SSKLEGVRESSLEL; | SKLEGVRESSLELV; |
| KLEGVRESSLELVE; | LEGVRESSLELVES; | EGVRESSLELVESN; |
| GVRESSLELVESND; | VRESSLELVESNDA; | RESSLELVESNDAG; |
| ESSLELVESNDAGV; | SSLELVESNDAGVV; | SLELVESNDAGVVK; |
| LELVESNDAGVVKK; | ELVESNDAGVVKKF; | LVESNDAGVVKKFV; |
| VESNDAGVVKKFVG; | ESNDAGVVKKFVGS; | SNDAGVVKKFVGSM; |
| NDAGVVKKFVGSMS; | DAGVVKKFVGSMSL; | AGVVKKFVGSMSLM; |
| GVVKKFVGSMSLMS; | VVKKFVGSMSLMSD; | VKKFVGSMSLMSDV; |
| KKFVGSMSLMSDVA; | KFVGSMSLMSDVAK; | FVGSMSLMSDVAKG; |
| VGSMSLMSDVAKGT; | GSMSLMSDVAKGTV; | SMSLMSDVAKGTVV; |
| MSLMSDVAKGTVVA; | SLMSDVAKGTVVAS; | LMSDVAKGTVVASQ; |
| MSDVAKGTVVASQE; | SDVAKGTVVASQEA; | DVAKGTVVASQEAT; |
| VAKGTVVASQEATI; | AKGTVVASQEATIV; | KGTVVASQEATIVA; |
| GTVVASQEATIVAK; | TVVASQEATIVAKC; | VVASQEATIVAKCS; |
| VASQEATIVAKCSG; | ASQEATIVAKCSGM; | SQEATIVAKCSGMV; |
| QEATIVAKCSGMVA; | EATIVAKCSGMVAE; | ATIVAKCSGMVAEG; |
| TIVAKCSGMVAEGA; | IVAKCSGMVAEGAN; | VAKCSGMVAEGANK; |
| AKCSGMVAEGANKV; | KCSGMVAEGANKVV; | CSGMVAEGANKVVE; |
| SGMVAEGANKVVEM; | GMVAEGANKVVEMS; | MVAEGANKVVEMSK; |
| VAEGANKVVEMSKK; | AEGANKVVEMSKKA; | EGANKVVEMSKKAV; |
| GANKVVEMSKKAVQ; | ANKVVEMSKKAVQE; | NKVVEMSKKAVQET; |
| KVVEMSKKAVQETQ; | VVEMSKKAVQETQK; | VEMSKKAVQETQKA; |
| EMSKKAVQETQKAV; | MSKKAVQETQKAVS; | SKKAVQETQKAVSV; |
| KKAVQETQKAVSVA; | KAVQETQKAVSVAG; | AVQETQKAVSVAGE; |
| VQETQKAVSVAGEA; | QETQKAVSVAGEAT; | ETQKAVSVAGEATF; |
| TQKAVSVAGEATFL; | QKAVSVAGEATFLI; | KAVSVAGEATFLIE; |
| AVSVAGEATFLIEK; | VSVAGEATFLIEKQ; | SVAGEATFLIEKQI; |
| VAGEATFLIEKQIM; | AGEATFLIEKQIML; | GEATFLIEKQIMLN; |

EATFLIEKQIMLNK; ATFLIEKQIMLNKS; TFLIEKQIMLNKSP;
FLIEKQIMLNKSPN; LIEKQIMLNKSPNN; IEKQIMLNKSPNNK;
EKQIMLNKSPNNKE; KQIMLNKSPNNKEL; QIMLNKSPNNKELE;
IMLNKSPNNKELEL; MLNKSPNNKELELT; LNKSPNNKELELTK;
NKSPNNKELELTKE; KSPNNKELELTKEE; SPNNKELELTKEEF;
PNNKELELTKEEFA; NNKELELTKEEFAK; NKELELTKEEFAKV;
KELELTKEEFAKVD; ELELTKEEFAKVDE; LELTKEEFAKVDEV;
ELTKEEFAKVDEVK; LTKEEFAKVDEVKE; TKEEFAKVDEVKET;
KEEFAKVDEVKETL; EEFAKVDEVKETLM; EFAKVDEVKETLMA;
FAKVDEVKETLMAS; AKVDEVKETLMASE; KVDEVKETLMASER;
VDEVKETLMASERA; DEVKETLMASERAL; EVKETLMASERALD;
VKETLMASERALDE; KETLMASERALDET; ETLMASERALDETV;
TLMASERALDETVQ; LMASERALDETVQE; MASERALDETVQEA;
ASERALDETVQEAQ; SERALDETVQEAQK; ERALDETVQEAQKV;
RALDETVQEAQKVL; ALDETVQEAQKVLN; LDETVQEAQKVLNM;
DETVQEAQKVLNMV; ETVQEAQKVLNMVN; TVQEAQKVLNMVNG;
VQEAQKVLNMVNGL; QEAQKVLNMVNGLN; EAQKVLNMVNGLNP;
AQKVLNMVNGLNPS; QKVLNMVNGLNPSN; KVLNMVNGLNPSNK;
VLNMVNGLNPSNKD; LNMVNGLNPSNKDQ; NMVNGLNPSNKDQV;
MVNGLNPSNKDQVL; VNGLNPSNKDQVLA; NGLNPSNKDQVLAK;
GLNPSNKDQVLAKK; LNPSNKDQVLAKKD; NPSNKDQVLAKKDV;
PSNKDQVLAKKDVR; SNKDQVLAKKDVRK; NKDQVLAKKDVRKA;
KDQVLAKKDVRKAI; DQVLAKKDVRKAIS; QVLAKKDVRKAISN;
VLAKKDVRKAISNV; LAKKDVRKAISNVV; AKKDVRKAISNVVK;
KKDVRKAISNVVKV; KDVRKAISNVVKVA; DVRKAISNVVKVAQ;
VRKAISNVVKVAQG; RKAISNVVKVAQGA; KAISNVVKVAQGAR;
AISNVVKVAQGARD; ISNVVKVAQGARDL; SNVVKVAQGARDLT;
NVVKVAQGARDLTK; VVKVAQGARDLTKV; VKVAQGARDLTKVM;
KVAQGARDLTKVMA; VAQGARDLTKVMAI; AQGARDLTKVMAIS;
QGARDLTKVMAISL; GARDLTKVMAISLY; ARDLTKVMAISLYM;
RDLTKVMAISLYMR;

15 mers:
MTKIFSNLIINGLLF; TKIFSNLIINGLLFG; KIFSNLIINGLLFGF;
IFSNLIINGLLFGFV; FSNLIINGLLFGFVS; SNLIINGLLFGFVSL;
NLIINGLLFGFVSLN; LIINGLLFGFVSLNV; IINGLLFGFVSLNVF;
INGLLFGFVSLNVFA; NGLLFGFVSLNVFAD; GLLFGFVSLNVFADS;
LLFGFVSLNVFADSN; LFGFVSLNVFADSNN; FGFVSLNVFADSNNA;
GFVSLNVFADSNNAN; FVSLNVFADSNNANI; VSLNVFADSNNANIL;
SLNVFADSNNANILK; LNVFADSNNANILKP; NVFADSNNANILKPQ;
VFADSNNANILKPQS; FADSNNANILKPQSN; ADSNNANILKPQSNV;
DSNNANILKPQSNVL; SNNANILKPQSNVLE; NNANILKPQSNVLEH;
NANILKPQSNVLEHS; ANILKPQSNVLEHSD; NILKPQSNVLEHSDQ;
ILKPQSNVLEHSDQK; LKPQSNVLEHSDQKD; KPQSNVLEHSDQKDN;
PQSNVLEHSDQKDNK; QSNVLEHSDQKDNKK; SNVLEHSDQKDNKKL;
NVLEHSDQKDNKKLD; VLEHSDQKDNKKLDQ; LEHSDQKDNKKLDQK;
EHSDQKDNKKLDQKD; HSDQKDNKKLDQKDQ; SDQKDNKKLDQKDQV;
DQKDNKKLDQKDQVN; QKDNKKLDQKDQVNQ; KDNKKLDQKDQVNQA;
DNKKLDQKDQVNQAL; NKKLDQKDQVNQALD; KKLDQKDQVNQALDT;
KLDQKDQVNQALDTI; LDQKDQVNQALDTIN; DQKDQVNQALDTINK;
QKDQVNQALDTINKV; KDQVNQALDTINKVT; DQVNQALDTINKVTE;

QVNQALDTINKVTED; VNQALDTINKVTEDV; NQALDTINKVTEDVS;
QALDTINKVTEDVSS; ALDTINKVTEDVSSK; LDTINKVTEDVSSKL;
DTINKVTEDVSSKLE; TINKVTEDVSSKLEG; INKVTEDVSSKLEGV;
NKVTEDVSSKLEGVR; KVTEDVSSKLEGVRE; VTEDVSSKLEGVRES;
TEDVSSKLEGVRESS; EDVSSKLEGVRESSL; DVSSKLEGVRESSLE;
VSSKLEGVRESSLEL; SSKLEGVRESSLELV; SKLEGVRESSLELVE;
KLEGVRESSLELVES; LEGVRESSLELVESN; EGVRESSLELVESND;
GVRESSLELVESNDA; VRESSLELVESNDAG; RESSLELVESNDAGV;
ESSLELVESNDAGVV; SSLELVESNDAGVVK; SLELVESNDAGVVKK;
LELVESNDAGVVKKF; ELVESNDAGVVKKFV; LVESNDAGVVKKFVG;
VESNDAGVVKKFVGS; ESNDAGVVKKFVGSM; SNDAGVVKKFVGSMS;
NDAGVVKKFVGSMSL; DAGVVKKFVGSMSLM; AGVVKKFVGSMSLMS;
GVVKKFVGSMSLMSD; VVKKFVGSMSLMSDV; VKKFVGSMSLMSDVA;
KKFVGSMSLMSDVAK; KFVGSMSLMSDVAKG; FVGSMSLMSDVAKGT;
VGSMSLMSDVAKGTV; GSMSLMSDVAKGTVV; SMSLMSDVAKGTVVA;
MSLMSDVAKGTVVAS; SLMSDVAKGTVVASQ; LMSDVAKGTVVASQE;
MSDVAKGTVVASQEA; SDVAKGTVVASQEAT; DVAKGTVVASQEATI;
VAKGTVVASQEATIV; AKGTVVASQEATIVA; KGTVVASQEATIVAK;
GTVVASQEATIVAKC; TVVASQEATIVAKCS; VVASQEATIVAKCSG;
VASQEATIVAKCSGM; ASQEATIVAKCSGMV; SQEATIVAKCSGMVA;
QEATIVAKCSGMVAE; EATIVAKCSGMVAEG; ATIVAKCSGMVAEGA;
TIVAKCSGMVAEGAN; IVAKCSGMVAEGANK; VAKCSGMVAEGANKV;
AKCSGMVAEGANKVV; KCSGMVAEGANKVVE; CSGMVAEGANKVVEM;
SGMVAEGANKVVEMS; GMVAEGANKVVEMSK; MVAEGANKVVEMSKK;
VAEGANKVVEMSKKA; AEGANKVVEMSKKAV; EGANKVVEMSKKAVQ;
GANKVVEMSKKAVQE; ANKVVEMSKKAVQET; NKVVEMSKKAVQETQ;
KVVEMSKKAVQETQK; VVEMSKKAVQETQKA; VEMSKKAVQETQKAV;
EMSKKAVQETQKAVS; MSKKAVQETQKAVSV; SKKAVQETQKAVSVA;
KKAVQETQKAVSVAG; KAVQETQKAVSVAGE; AVQETQKAVSVAGEA;
VQETQKAVSVAGEAT; QETQKAVSVAGEATF; ETQKAVSVAGEATFL;
TQKAVSVAGEATFLI; QKAVSVAGEATFLIE; KAVSVAGEATFLIEK;
AVSVAGEATFLIEKQ; VSVAGEATFLIEKQI; SVAGEATFLIEKQIM;
VAGEATFLIEKQIML; AGEATFLIEKQIMLN; GEATFLIEKQIMLNK;
EATFLIEKQIMLNKS; ATFLIEKQIMLNKSP; TFLIEKQIMLNKSPN;
FLIEKQIMLNKSPNN; LIEKQIMLNKSPNNK; IEKQIMLNKSPNNKE;
EKQIMLNKSPNNKEL; KQIMLNKSPNNKELE; QIMLNKSPNNKELEL;
IMLNKSPNNKELELT; MLNKSPNNKELELTK; LNKSPNNKELELTKE;
NKSPNNKELELTKEE; KSPNNKELELTKEEF; SPNNKELELTKEEFA;
PNNKELELTKEEFAK; NNKELELTKEEFAKV; NKELELTKEEFAKVD;
KELELTKEEFAKVDE; ELELTKEEFAKVDEV; LELTKEEFAKVDEVK;
ELTKEEFAKVDEVKE; LTKEEFAKVDEVKET; TKEEFAKVDEVKETL;
KEEFAKVDEVKETLM; EEFAKVDEVKETLMA; EFAKVDEVKETLMAS;
FAKVDEVKETLMASE; AKVDEVKETLMASER; KVDEVKETLMASERA;
VDEVKETLMASERAL; DEVKETLMASERALD; EVKETLMASERALDE;
VKETLMASERALDET; KETLMASERALDETV; ETLMASERALDETVQ;
TLMASERALDETVQE; LMASERALDETVQEA; MASERALDETVQEAQ;
ASERALDETVQEAQK; SERALDETVQEAQKV; ERALDETVQEAQKVL;
RALDETVQEAQKVLN; ALDETVQEAQKVLNM; LDETVQEAQKVLNMV;
DETVQEAQKVLNMVN; ETVQEAQKVLNMVNG; TVQEAQKVLNMVNGL;
VQEAQKVLNMVNGLN; QEAQKVLNMVNGLNP; EAQKVLNMVNGLNPS;
AQKVLNMVNGLNPSN; QKVLNMVNGLNPSNK; KVLNMVNGLNPSNKD;

VLNMVNGLNPSNKDQ; LNMVNGLNPSNKDQV; NMVNGLNPSNKDQVL;
MVNGLNPSNKDQVLA; VNGLNPSNKDQVLAK; NGLNPSNKDQVLAKK;
GLNPSNKDQVLAKKD; LNPSNKDQVLAKKDV; NPSNKDQVLAKKDVR;
PSNKDQVLAKKDVRK; SNKDQVLAKKDVRKA; NKDQVLAKKDVRKAI;
KDQVLAKKDVRKAIS; DQVLAKKDVRKAISN; QVLAKKDVRKAISNV;
VLAKKDVRKAISNVV; LAKKDVRKAISNVVK; AKKDVRKAISNVVKV;
KKDVRKAISNVVKVA; KDVRKAISNVVKVAQ; DVRKAISNVVKVAQG;
VRKAISNVVKVAQGA; RKAISNVVKVAQGAR; KAISNVVKVAQGARD;
AISNVVKVAQGARDL; ISNVVKVAQGARDLT; SNVVKVAQGARDLTK;
NVVKVAQGARDLTKV; VVKVAQGARDLTKVM; VKVAQGARDLTKVMA;
KVAQGARDLTKVMAI; VAQGARDLTKVMAIS; AQGARDLTKVMAISL;
QGARDLTKVMAISLY; GARDLTKVMAISLYM; ARDLTKVMAISLYMR;

16 mers:
MTKIFSNLIINGLLFG; TKIFSNLIINGLLFGF; KIFSNLIINGLLFGFV;
IFSNLIINGLLFGFVS; FSNLIINGLLFGFVSL; SNLIINGLLFGFVSLN;
NLIINGLLFGFVSLNV; LIINGLLFGFVSLNVF; IINGLLFGFVSLNVFA;
INGLLFGFVSLNVFAD; NGLLFGFVSLNVFADS; GLLFGFVSLNVFADSN;
LLFGFVSLNVFADSNN; LFGFVSLNVFADSNNA; FGFVSLNVFADSNNAN;
GFVSLNVFADSNNANI; FVSLNVFADSNNANIL; VSLNVFADSNNANILK;
SLNVFADSNNANILKP; LNVFADSNNANILKPQ; NVFADSNNANILKPQS;
VFADSNNANILKPQSN; FADSNNANILKPQSNV; ADSNNANILKPQSNVL;
DSNNANILKPQSNVLE; SNNANILKPQSNVLEH; NNANILKPQSNVLEHS;
NANILKPQSNVLEHSD; ANILKPQSNVLEHSDQ; NILKPQSNVLEHSDQK;
ILKPQSNVLEHSDQKD; LKPQSNVLEHSDQKDN; KPQSNVLEHSDQKDNK;
PQSNVLEHSDQKDNKK; QSNVLEHSDQKDNKKL; SNVLEHSDQKDNKKLD;
NVLEHSDQKDNKKLDQ; VLEHSDQKDNKKLDQK; LEHSDQKDNKKLDQKD;
EHSDQKDNKKLDQKDQ; HSDQKDNKKLDQKDQV; SDQKDNKKLDQKDQVN;
DQKDNKKLDQKDQVNQ; QKDNKKLDQKDQVNQA; KDNKKLDQKDQVNQAL;
DNKKLDQKDQVNQALD; NKKLDQKDQVNQALDT; KKLDQKDQVNQALDTI;
KLDQKDQVNQALDTIN; LDQKDQVNQALDTINK; DQKDQVNQALDTINKV;
QKDQVNQALDTINKVT; KDQVNQALDTINKVTE; DQVNQALDTINKVTED;
QVNQALDTINKVTEDV; VNQALDTINKVTEDVS; NQALDTINKVTEDVSS;
QALDTINKVTEDVSSK; ALDTINKVTEDVSSKL; LDTINKVTEDVSSKLE;
DTINKVTEDVSSKLEG; TINKVTEDVSSKLEGV; INKVTEDVSSKLEGVR;
NKVTEDVSSKLEGVRE; KVTEDVSSKLEGVRES; VTEDVSSKLEGVRESS;
TEDVSSKLEGVRESSL; EDVSSKLEGVRESSLE; DVSSKLEGVRESSLEL;
VSSKLEGVRESSLELV; SSKLEGVRESSLELVE; SKLEGVRESSLELVES;
KLEGVRESSLELVESN; LEGVRESSLELVESND; EGVRESSLELVESNDA;
GVRESSLELVESNDAG; VRESSLELVESNDAGV; RESSLELVESNDAGVV;
ESSLELVESNDAGVVK; SSLELVESNDAGVVKK; SLELVESNDAGVVKKF;
LELVESNDAGVVKKFV; ELVESNDAGVVKKFVG; LVESNDAGVVKKFVGS;
VESNDAGVVKKFVGSM; ESNDAGVVKKFVGSMS; SNDAGVVKKFVGSMSL;
NDAGVVKKFVGSMSLM; DAGVVKKFVGSMSLMS; AGVVKKFVGSMSLMSD;
GVVKKFVGSMSLMSDV; VVKKFVGSMSLMSDVA; VKKFVGSMSLMSDVAK;
KKFVGSMSLMSDVAKG; KFVGSMSLMSDVAKGT; FVGSMSLMSDVAKGTV;
VGSMSLMSDVAKGTVV; GSMSLMSDVAKGTVVA; SMSLMSDVAKGTVVAS;
MSLMSDVAKGTVVASQ; SLMSDVAKGTVVASQE; LMSDVAKGTVVASQEA;
MSDVAKGTVVASQEAT; SDVAKGTVVASQEATI; DVAKGTVVASQEATIV;
VAKGTVVASQEATIVA; AKGTVVASQEATIVAK; KGTVVASQEATIVAKC;
GTVVASQEATIVAKCS; TVVASQEATIVAKCSG; VVASQEATIVAKCSGM;

| | |
|---|---|
| | VASQEATIVAKCSGMV; ASQEATIVAKCSGMVA; SQEATIVAKCSGMVAE;<br>QEATIVAKCSGMVAEG; EATIVAKCSGMVAEGA; ATIVAKCSGMVAEGAN;<br>TIVAKCSGMVAEGANK; IVAKCSGMVAEGANKV; VAKCSGMVAEGANKVV;<br>AKCSGMVAEGANKVVE; KCSGMVAEGANKVVEM; CSGMVAEGANKVVEMS;<br>SGMVAEGANKVVEMSK; GMVAEGANKVVEMSKK; MVAEGANKVVEMSKKA;<br>VAEGANKVVEMSKKAV; AEGANKVVEMSKKAVQ; EGANKVVEMSKKAVQE;<br>GANKVVEMSKKAVQET; ANKVVEMSKKAVQETQ; NKVVEMSKKAVQETQK;<br>KVVEMSKKAVQETQKA; VVEMSKKAVQETQKAV; VEMSKKAVQETQKAVS;<br>EMSKKAVQETQKAVSV; MSKKAVQETQKAVSVA; SKKAVQETQKAVSVAG;<br>KKAVQETQKAVSVAGE; KAVQETQKAVSVAGEA; AVQETQKAVSVAGEAT;<br>VQETQKAVSVAGEATF; QETQKAVSVAGEATFL; ETQKAVSVAGEATFLI;<br>TQKAVSVAGEATFLIE; QKAVSVAGEATFLIEK; KAVSVAGEATFLIEKQ;<br>AVSVAGEATFLIEKQI; VSVAGEATFLIEKQIM; SVAGEATFLIEKQIML;<br>VAGEATFLIEKQIMLN; AGEATFLIEKQIMLNK; GEATFLIEKQIMLNKS;<br>EATFLIEKQIMLNKSP; ATFLIEKQIMLNKSPN; TFLIEKQIMLNKSPNN;<br>FLIEKQIMLNKSPNNK; LIEKQIMLNKSPNNKE; IEKQIMLNKSPNNKEL;<br>EKQIMLNKSPNNKELE; KQIMLNKSPNNKELEL; QIMLNKSPNNKELELT;<br>IMLNKSPNNKELELTK; MLNKSPNNKELELTKE; LNKSPNNKELELTKEE;<br>NKSPNNKELELTKEEF; KSPNNKELELTKEEFA; SPNNKELELTKEEFAK;<br>PNNKELELTKEEFAKV; NNKELELTKEEFAKVD; NKELELTKEEFAKVDE;<br>KELELTKEEFAKVDEV; ELELTKEEFAKVDEVK; LELTKEEFAKVDEVKE;<br>ELTKEEFAKVDEVKET; LTKEEFAKVDEVKETL; TKEEFAKVDEVKETLM;<br>KEEFAKVDEVKETLMA; EEFAKVDEVKETLMAS; EFAKVDEVKETLMASE;<br>FAKVDEVKETLMASER; AKVDEVKETLMASERA; KVDEVKETLMASERAL;<br>VDEVKETLMASERALD; DEVKETLMASERALDE; EVKETLMASERALDET;<br>VKETLMASERALDETV; KETLMASERALDETVQ; ETLMASERALDETVQE;<br>TLMASERALDETVQEA; LMASERALDETVQEAQ; MASERALDETVQEAQK;<br>ASERALDETVQEAQKV; SERALDETVQEAQKVL; ERALDETVQEAQKVLN;<br>RALDETVQEAQKVLNM; ALDETVQEAQKVLNMV; LDETVQEAQKVLNMVN;<br>DETVQEAQKVLNMVNG; ETVQEAQKVLNMVNGL; TVQEAQKVLNMVNGLN;<br>VQEAQKVLNMVNGLNP; QEAQKVLNMVNGLNPS; EAQKVLNMVNGLNPSN;<br>AQKVLNMVNGLNPSNK; QKVLNMVNGLNPSNKD; KVLNMVNGLNPSNKDQ;<br>VLNMVNGLNPSNKDQV; LNMVNGLNPSNKDQVL; NMVNGLNPSNKDQVLA;<br>MVNGLNPSNKDQVLAK; VNGLNPSNKDQVLAKK; NGLNPSNKDQVLAKKD;<br>GLNPSNKDQVLAKKDV; LNPSNKDQVLAKKDVR; NPSNKDQVLAKKDVRK;<br>PSNKDQVLAKKDVRKA; SNKDQVLAKKDVRKAI; NKDQVLAKKDVRKAIS;<br>KDQVLAKKDVRKAISN; DQVLAKKDVRKAISNV; QVLAKKDVRKAISNVV;<br>VLAKKDVRKAISNVVK; LAKKDVRKAISNVVKV; AKKDVRKAISNVVKVA;<br>KKDVRKAISNVVKVAQ; KDVRKAISNVVKVAQG; DVRKAISNVVKVAQGA;<br>VRKAISNVVKVAQGAR; RKAISNVVKVAQGARD; KAISNVVKVAQGARDL;<br>AISNVVKVAQGARDLT; ISNVVKVAQGARDLTK; SNVVKVAQGARDLTKV;<br>NVVKVAQGARDLTKVM; VVKVAQGARDLTKVMA; VKVAQGARDLTKVMAI;<br>KVAQGARDLTKVMAIS; VAQGARDLTKVMAISL; AQGARDLTKVMAISLY;<br>QGARDLTKVMAISLYM; GARDLTKVMAISLYMR; |
| Seq 26 | 13 mers:<br>MKKMLLIFSFFLI; KKMLLIFSFFLIF; KMLLIFSFFLIFL;<br>MLLIFSFFLIFLN; LLIFSFFLIFLNG; LIFSFFLIFLNGF;<br>IFSFFLIFLNGFP; FSFFLIFLNGFPL; SFFLIFLNGFPLN;<br>FFLIFLNGFPLNA; FLIFLNGFPLNAR; LIFLNGFPLNARK; |

| |
|---|
| IFLNGFPLNARKV; FLNGFPLNARKVD; LNGFPLNARKVDK; NGFPLNARKVDKE; GFPLNARKVDKEK; FPLNARKVDKEKL; PLNARKVDKEKLK; LNARKVDKEKLKD; NARKVDKEKLKDF; ARKVDKEKLKDFV; RKVDKEKLKDFVN; KVDKEKLKDFVNM; VDKEKLKDFVNMD; DKEKLKDFVNMDL; KEKLKDFVNMDLE; EKLKDFVNMDLEF; KLKDFVNMDLEFV; LKDFVNMDLEFVN; KDFVNMDLEFVNY; DFVNMDLEFVNYK; FVNMDLEFVNYKG; VNMDLEFVNYKGP; NMDLEFVNYKGPY; MDLEFVNYKGPYD; DLEFVNYKGPYDS; LEFVNYKGPYDST; EFVNYKGPYDSTN; FVNYKGPYDSTNT; VNYKGPYDSTNTY; NYKGPYDSTNTYE; YKGPYDSTNTYEQ; KGPYDSTNTYEQI; GPYDSTNTYEQIV; PYDSTNTYEQIVG; YDSTNTYEQIVGI; DSTNTYEQIVGIG; STNTYEQIVGIGE; TNTYEQIVGIGEF; NTYEQIVGIGEFL; TYEQIVGIGEFLA; YEQIVGIGEFLAR; EQIVGIGEFLARP; QIVGIGEFLARPL; IVGIGEFLARPLT; VGIGEFLARPLTN; GIGEFLARPLTNS; IGEFLARPLTNSN; GEFLARPLTNSNS; EFLARPLTNSNSN; FLARPLTNSNSNS; LARPLTNSNSNSS; ARPLTNSNSNSSY; RPLTNSNSNSSYY; PLTNSNSNSSYYG; LTNSNSNSSYYGK; TNSNSNSSYYGKY; NSNSNSSYYGKYF; SNSNSSYYGKYFI; NSNSSYYGKYFIN; SNSSYYGKYFINR; NSSYYGKYFINRF; SSYYGKYFINRFI; SYYGKYFINRFID; YYGKYFINRFIDD; YGKYFINRFIDDQ; GKYFINRFIDDQD; KYFINRFIDDQDK; YFINRFIDDQDKK; FINRFIDDQDKKA; INRFIDDQDKKAS; NRFIDDQDKKASV; RFIDDQDKKASVD; FIDDQDKKASVDV; IDDQDKKASVDVF; DDQDKKASVDVFS; DQDKKASVDVFSI; QDKKASVDVFSIS; DKKASVDVFSISS; KKASVDVFSISSK; KASVDVFSISSKS; ASVDVFSISSKSE; SVDVFSISSKSEL; VDVFSISSKSELD; DVFSISSKSELDS; VFSISSKSELDSI; FSISSKSELDSIL; SISSKSELDSILN; ISSKSELDSILNL; SSKSELDSILNLR; SKSELDSILNLRR; KSELDSILNLRRI; SELDSILNLRRIL; ELDSILNLRRILT; LDSILNLRRILTG; DSILNLRRILTGY; SILNLRRILTGYI; ILNLRRILTGYII; LNLRRILTGYIIK; NLRRILTGYIIKS; LRRILTGYIIKSF; RRILTGYIIKSFD; RILTGYIIKSFDY; ILTGYIIKSFDYD; LTGYIIKSFDYDR; TGYIIKSFDYDRS; GYIIKSFDYDRSS; YIIKSFDYDRSSA; IIKSFDYDRSSAE; IKSFDYDRSSAEL; KSFDYDRSSAELI; SFDYDRSSAELIA; FDYDRSSAELIAK; DYDRSSAELIAKV; YDRSSAELIAKVI; DRSSAELIAKVIT; RSSAELIAKVITI; SSAELIAKVITIY; SAELIAKVITIYN; AELIAKVITIYNA; ELIAKVITIYNAV; LIAKVITIYNAVY; IAKVITIYNAVYR; AKVITIYNAVYRG; KVITIYNAVYRGD; VITIYNAVYRGDL; ITIYNAVYRGDLD; TIYNAVYRGDLDY; IYNAVYRGDLDYY; YNAVYRGDLDYYK; NAVYRGDLDYYKG; AVYRGDLDYYKGF; VYRGDLDYYKGFY; YRGDLDYYKGFYI; RGDLDYYKGFYIE; GDLDYYKGFYIEP; DLDYYKGFYIEPA; LDYYKGFYIEPAL; DYYKGFYIEPALK; YYKGFYIEPALKS; YKGFYIEPALKSL; KGFYIEPALKSLT; GFYIEPALKSLTK; FYIEPALKSLTKE; YIEPALKSLTKEN; IEPALKSLTKENA; EPALKSLTKENAG; PALKSLTKENAGL; ALKSLTKENAGLS; LKSLTKENAGLSR; KSLTKENAGLSRV; SLTKENAGLSRVY; LTKENAGLSRVYS; TKENAGLSRVYSQ; |

| | | |
|---|---|---|
| KENAGLSRVYSQW; | ENAGLSRVYSQWA; | NAGLSRVYSQWAG; |
| AGLSRVYSQWAGK; | GLSRVYSQWAGKT; | LSRVYSQWAGKTQ; |
| SRVYSQWAGKTQI; | RVYSQWAGKTQIF; | VYSQWAGKTQIFI; |
| YSQWAGKTQIFIP; | SQWAGKTQIFIPL; | QWAGKTQIFIPLK; |
| WAGKTQIFIPLKK; | AGKTQIFIPLKKD; | GKTQIFIPLKKDI; |
| KTQIFIPLKKDIL; | TQIFIPLKKDILS; | QIFIPLKKDILSG; |
| IFIPLKKDILSGN; | FIPLKKDILSGNI; | IPLKKDILSGNIE; |
| PLKKDILSGNIES; | LKKDILSGNIESD; | KKDILSGNIESDI; |
| KDILSGNIESDID; | DILSGNIESDIDI; | ILSGNIESDIDID; |
| LSGNIESDIDIDS; | SGNIESDIDIDSL; | GNIESDIDIDSLV; |
| NIESDIDIDSLVT; | IESDIDIDSLVTD; | ESDIDIDSLVTDK; |
| SDIDIDSLVTDKV; | DIDIDSLVTDKVI; | IDIDSLVTDKVIA; |
| DIDSLVTDKVIAA; | IDSLVTDKVIAAL; | DSLVTDKVIAALL; |
| SLVTDKVIAALLS; | LVTDKVIAALLSE; | VTDKVIAALLSEN; |
| TDKVIAALLSENE; | DKVIAALLSENEA; | KVIAALLSENEAG; |
| VIAALLSENEAGV; | IAALLSENEAGVN; | AALLSENEAGVNF; |
| ALLSENEAGVNFA; | LLSENEAGVNFAR; | LSENEAGVNFARD; |
| SENEAGVNFARDI; | ENEAGVNFARDIT; | NEAGVNFARDITD; |
| EAGVNFARDITDI; | AGVNFARDITDIQ; | GVNFARDITDIQG; |
| VNFARDITDIQGE; | NFARDITDIQGET; | FARDITDIQGETH; |
| ARDITDIQGETHK; | RDITDIQGETHKA; | DITDIQGETHKAD; |
| ITDIQGETHKADQ; | TDIQGETHKADQD; | DIQGETHKADQDK; |
| IQGETHKADQDKI; | QGETHKADQDKID; | GETHKADQDKIDT; |
| ETHKADQDKIDTE; | THKADQDKIDTEL; | HKADQDKIDTELD; |
| KADQDKIDTELDN; | ADQDKIDTELDNI; | DQDKIDTELDNIH; |
| QDKIDTELDNIHE; | DKIDTELDNIHES; | KIDTELDNIHESD; |
| IDTELDNIHESDS; | DTELDNIHESDSN; | TELDNIHESDSNI; |
| ELDNIHESDSNIT; | LDNIHESDSNITE; | DNIHESDSNITET; |
| NIHESDSNITETI; | IHESDSNITETIE; | HESDSNITETIEN; |
| ESDSNITETIENL; | SDSNITETIENLR; | DSNITETIENLRD; |
| SNITETIENLRDQ; | NITETIENLRDQL; | ITETIENLRDQLE; |
| TETIENLRDQLEK; | ETIENLRDQLEKA; | TIENLRDQLEKAT; |
| IENLRDQLEKATD; | ENLRDQLEKATDE; | NLRDQLEKATDEE; |
| LRDQLEKATDEEH; | RDQLEKATDEEHK; | DQLEKATDEEHKK; |
| QLEKATDEEHKKE; | LEKATDEEHKKEI; | EKATDEEHKKEIE; |
| KATDEEHKKEIES; | ATDEEHKKEIESQ; | TDEEHKKEIESQV; |
| DEEHKKEIESQVD; | EEHKKEIESQVDA; | EHKKEIESQVDAK; |
| HKKEIESQVDAKK; | KKEIESQVDAKKK; | KEIESQVDAKKKE; |
| EIESQVDAKKKEK; | IESQVDAKKKEKE; | ESQVDAKKKEKEE; |
| SQVDAKKKEKEEL; | QVDAKKKEKEELD; | VDAKKKEKEELDK; |
| DAKKKEKEELDKK; | AKKKEKEELDKKA; | KKKEKEELDKKAI; |
| KKEKEELDKKAIN; | KEKEELDKKAINL; | EKEELDKKAINLD; |
| KEELDKKAINLDK; | EELDKKAINLDKA; | ELDKKAINLDKAQ; |
| LDKKAINLDKAQQ; | DKKAINLDKAQQK; | KKAINLDKAQQKL; |
| KAINLDKAQQKLD; | AINLDKAQQKLDS; | INLDKAQQKLDSA; |
| NLDKAQQKLDSAE; | LDKAQQKLDSAED; | DKAQQKLDSAEDN; |
| KAQQKLDSAEDNL; | AQQKLDSAEDNLD; | QQKLDSAEDNLDV; |
| QKLDSAEDNLDVQ; | KLDSAEDNLDVQR; | LDSAEDNLDVQRD; |
| DSAEDNLDVQRDT; | SAEDNLDVQRDTV; | AEDNLDVQRDTVR; |
| EDNLDVQRDTVRE; | DNLDVQRDTVREK; | NLDVQRDTVREKI; |
| LDVQRDTVREKIQ; | DVQRDTVREKIQE; | VQRDTVREKIQED; |

QRDTVREKIQEDI; RDTVREKIQEDIN; DTVREKIQEDINE;
TVREKIQEDINEI; VREKIQEDINEIN; REKIQEDINEINK;
EKIQEDINEINKE; KIQEDINEINKEK; IQEDINEINKEKN;
QEDINEINKEKNL; EDINEINKEKNLP; DINEINKEKNLPK;
INEINKEKNLPKP; NEINKEKNLPKPG; EINKEKNLPKPGD;
INKEKNLPKPGDV; NKEKNLPKPGDVS; KEKNLPKPGDVSS;
EKNLPKPGDVSSP; KNLPKPGDVSSPK; NLPKPGDVSSPKV;
LPKPGDVSSPKVD; PKPGDVSSPKVDK; KPGDVSSPKVDKQ;
PGDVSSPKVDKQL; GDVSSPKVDKQLQ; DVSSPKVDKQLQI;
VSSPKVDKQLQIK; SSPKVDKQLQIKE; SPKVDKQLQIKES;
PKVDKQLQIKESL; KVDKQLQIKESLE; VDKQLQIKESLED;
DKQLQIKESLEDL; KQLQIKESLEDLQ; QLQIKESLEDLQE;
LQIKESLEDLQEQ; QIKESLEDLQEQL; IKESLEDLQEQLK;
KESLEDLQEQLKE; ESLEDLQEQLKEA; SLEDLQEQLKEAG;
LEDLQEQLKEAGD; EDLQEQLKEAGDE; DLQEQLKEAGDEN;
LQEQLKEAGDENQ; QEQLKEAGDENQK; EQLKEAGDENQKR;
QLKEAGDENQKRE; LKEAGDENQKREI; KEAGDENQKREIE;
EAGDENQKREIEK; AGDENQKREIEKQ; GDENQKREIEKQI;
DENQKREIEKQIE; ENQKREIEKQIEI; NQKREIEKQIEIK;
QKREIEKQIEIKK; KREIEKQIEIKKR; REIEKQIEIKKRD;
EIEKQIEIKKRDE; IEKQIEIKKRDEE; EKQIEIKKRDEEL;
KQIEIKKRDEELL; QIEIKKRDEELLK; IEIKKRDEELLKS;
EIKKRDEELLKSK; IKKRDEELLKSKD; KKRDEELLKSKDG;
KRDEELLKSKDGK; RDEELLKSKDGKV; DEELLKSKDGKVS;
EELLKSKDGKVSK; ELLKSKDGKVSKD; LLKSKDGKVSKDY;
LKSKDGKVSKDYE; KSKDGKVSKDYEA; SKDGKVSKDYEAL;
KDGKVSKDYEALD; DGKVSKDYEALDL; GKVSKDYEALDLD;
KVSKDYEALDLDR; VSKDYEALDLDRE; SKDYEALDLDREL;
KDYEALDLDRELS; DYEALDLDRELSK; YEALDLDRELSKA;
EALDLDRELSKAS; ALDLDRELSKASS; LDLDRELSKASSK;
DLDRELSKASSKE; LDRELSKASSKEK; DRELSKASSKEKS;
RELSKASSKEKSK; ELSKASSKEKSKV; LSKASSKEKSKVK;
SKASSKEKSKVKE; KASSKEKSKVKEE; ASSKEKSKVKEEE;
SSKEKSKVKEEEI; SKEKSKVKEEEIT; KEKSKVKEEEITK;
EKSKVKEEEITKG; KSKVKEEEITKGK; SKVKEEEITKGKS;
KVKEEEITKGKSR; VKEEEITKGKSRA; KEEEITKGKSRAS;
EEEITKGKSRASL; EEITKGKSRASLG; EITKGKSRASLGD;
ITKGKSRASLGDL; TKGKSRASLGDLN; KGKSRASLGDLNN;
GKSRASLGDLNND; KSRASLGDLNNDK; SRASLGDLNNDKN;
RASLGDLNNDKNL; ASLGDLNNDKNLM; SLGDLNNDKNLML;
LGDLNNDKNLMLP; GDLNNDKNLMLPE; DLNNDKNLMLPED;
LNNDKNLMLPEDQ; NNDKNLMLPEDQK; NDKNLMLPEDQKL;
DKNLMLPEDQKLP; KNLMLPEDQKLPE; NLMLPEDQKLPED;
LMLPEDQKLPEDK; MLPEDQKLPEDKK; LPEDQKLPEDKKL;
PEDQKLPEDKKLD; EDQKLPEDKKLDS; DQKLPEDKKLDSK;
QKLPEDKKLDSKL; KLPEDKKLDSKLD; LPEDKKLDSKLDG;
PEDKKLDSKLDGK; EDKKLDSKLDGKK; DKKLDSKLDGKKE;
KKLDSKLDGKKEF; KLDSKLDGKKEFK; LDSKLDGKKEFKP;
DSKLDGKKEFKPV; SKLDGKKEFKPVS; KLDGKKEFKPVSE;
LDGKKEFKPVSEV; DGKKEFKPVSEVE; GKKEFKPVSEVEK;
KKEFKPVSEVEKL; KEFKPVSEVEKLD; EFKPVSEVEKLDK;

| |
|---|
| FKPVSEVEKLDKI; KPVSEVEKLDKIS; PVSEVEKLDKISK; VSEVEKLDKISKS; SEVEKLDKISKSN; EVEKLDKISKSNN; VEKLDKISKSNNN; EKLDKISKSNNNE; KLDKISKSNNNEV; LDKISKSNNNEVG; DKISKSNNNEVGK; KISKSNNNEVGKL; ISKSNNNEVGKLS; SKSNNNEVGKLSP; KSNNNEVGKLSPL; SNNNEVGKLSPLD; NNNEVGKLSPLDK; NNEVGKLSPLDKP; NEVGKLSPLDKPS; EVGKLSPLDKPSY; VGKLSPLDKPSYD; GKLSPLDKPSYDD; KLSPLDKPSYDDI; LSPLDKPSYDDID; SPLDKPSYDDIDS; PLDKPSYDDIDSK; LDKPSYDDIDSKE; DKPSYDDIDSKEE; KPSYDDIDSKEEV; PSYDDIDSKEEVD; SYDDIDSKEEVDN; YDDIDSKEEVDNK; DDIDSKEEVDNKA; DIDSKEEVDNKAI; IDSKEEVDNKAIN; DSKEEVDNKAINL; SKEEVDNKAINLQ; KEEVDNKAINLQK; EEVDNKAINLQKI; EVDNKAINLQKID; VDNKAINLQKIDP; DNKAINLQKIDPK; NKAINLQKIDPKV; KAINLQKIDPKVK; AINLQKIDPKVKD; INLQKIDPKVKDQ; NLQKIDPKVKDQT; LQKIDPKVKDQTT; QKIDPKVKDQTTS; KIDPKVKDQTTSL; IDPKVKDQTTSLN; DPKVKDQTTSLNE; PKVKDQTTSLNED; KVKDQTTSLNEDL; VKDQTTSLNEDLD; KDQTTSLNEDLDK; DQTTSLNEDLDKD; QTTSLNEDLDKDL; TTSLNEDLDKDLT; TSLNEDLDKDLTT; SLNEDLDKDLTTM; LNEDLDKDLTTMS; NEDLDKDLTTMSI; EDLDKDLTTMSID; DLDKDLTTMSIDS; LDKDLTTMSIDSS; DKDLTTMSIDSSS; KDLTTMSIDSSSP; DLTTMSIDSSSPV; LTTMSIDSSSPVF; TTMSIDSSSPVFL; TMSIDSSSPVFLE; MSIDSSSPVFLEV; SIDSSSPVFLEVI; IDSSSPVFLEVID; DSSSPVFLEVIDP; SSSPVFLEVIDPI; SSPVFLEVIDPIT; SPVFLEVIDPITN; PVFLEVIDPITNL; VFLEVIDPITNLG; FLEVIDPITNLGT; LEVIDPITNLGTL; EVIDPITNLGTLQ; VIDPITNLGTLQL; IDPITNLGTLQLI; DPITNLGTLQLID; PITNLGTLQLIDL; ITNLGTLQLIDLN; TNLGTLQLIDLNT; NLGTLQLIDLNTG; LGTLQLIDLNTGV; GTLQLIDLNTGVR; TLQLIDLNTGVRL; LQLIDLNTGVRLK; QLIDLNTGVRLKE; LIDLNTGVRLKES; IDLNTGVRLKEST; DLNTGVRLKESTQ; LNTGVRLKESTQQ; NTGVRLKESTQQG; TGVRLKESTQQGI; GVRLKESTQQGIQ; VRLKESTQQGIQR; RLKESTQQGIQRY; LKESTQQGIQRYG; KESTQQGIQRYGI; ESTQQGIQRYGIY; STQQGIQRYGIYE; TQQGIQRYGIYER; QQGIQRYGIYERE; QGIQRYGIYEREK; GIQRYGIYEREKD; IQRYGIYEREKDL; QRYGIYEREKDLV; RYGIYEREKDLVV; YGIYEREKDLVVI; GIYEREKDLVVIK; IYEREKDLVVIKM; YEREKDLVVIKMD; EREKDLVVIKMDS; REKDLVVIKMDSG; EKDLVVIKMDSGK; KDLVVIKMDSGKA; DLVVIKMDSGKAK; LVVIKMDSGKAKL; VVIKMDSGKAKLQ; VIKMDSGKAKLQI; IKMDSGKAKLQIL; KMDSGKAKLQILN; MDSGKAKLQILNK; DSGKAKLQILNKL; SGKAKLQILNKLE; GKAKLQILNKLEN; KAKLQILNKLENL; AKLQILNKLENLK; KLQILNKLENLKV; LQILNKLENLKVV; QILNKLENLKVVS; ILNKLENLKVVSE; LNKLENLKVVSES; NKLENLKVVSESN; KLENLKVVSESNF; LENLKVVSESNFE; ENLKVVSESNFEI; NLKVVSESNFEIN; LKVVSESNFEINK; KVVSESNFEINKN; VVSESNFEINKNS; VSESNFEINKNSS; SESNFEINKNSSL; ESNFEINKNSSLY; SNFEINKNSSLYV; |

NFEINKNSSLYVD; FEINKNSSLYVDS; EINKNSSLYVDSK;
INKNSSLYVDSKM; NKNSSLYVDSKMI; KNSSLYVDSKMIL;
NSSLYVDSKMILA; SSLYVDSKMILAA; SLYVDSKMILAAV;
LYVDSKMILAAVR; YVDSKMILAAVRD; VDSKMILAAVRDK;
DSKMILAAVRDKD; SKMILAAVRDKDD; KMILAAVRDKDDS;
MILAAVRDKDDSN; ILAAVRDKDDSNA; LAAVRDKDDSNAW;
AAVRDKDDSNAWR; AVRDKDDSNAWRL; VRDKDDSNAWRLA;
RDKDDSNAWRLAK; DKDDSNAWRLAKF; KDDSNAWRLAKFS;
DDSNAWRLAKFSP; DSNAWRLAKFSPK; SNAWRLAKFSPKN;
NAWRLAKFSPKNL; AWRLAKFSPKNLD; WRLAKFSPKNLDE;
RLAKFSPKNLDEF; LAKFSPKNLDEFI; AKFSPKNLDEFIL;
KFSPKNLDEFILS; FSPKNLDEFILSE; SPKNLDEFILSEN;
PKNLDEFILSENK; KNLDEFILSENKI; NLDEFILSENKIL;
LDEFILSENKILP; DEFILSENKILPF; EFILSENKILPFT;
FILSENKILPFTS; ILSENKILPFTSF; LSENKILPFTSFS;
SENKILPFTSFSV; ENKILPFTSFSVR; NKILPFTSFSVRK;
KILPFTSFSVRKN; ILPFTSFSVRKNF; LPFTSFSVRKNFI;
PFTSFSVRKNFIY; FTSFSVRKNFIYL; TSFSVRKNFIYLQ;
SFSVRKNFIYLQD; FSVRKNFIYLQDE; SVRKNFIYLQDEL;
VRKNFIYLQDELK; RKNFIYLQDELKN; KNFIYLQDELKNL;
NFIYLQDELKNLV; FIYLQDELKNLVI; IYLQDELKNLVIL;
YLQDELKNLVILD; LQDELKNLVILDV; QDELKNLVILDVN;
DELKNLVILDVNT; ELKNLVILDVNTL; LKNLVILDVNTLK;
KNLVILDVNTLKK; NLVILDVNTLKKV; LVILDVNTLKKVK;

14 mers:
MKKMLLIFSFFLIF; KKMLLIFSFFLIFL; KMLLIFSFFLIFLN;
MLLIFSFFLIFLNG; LLIFSFFLIFLNGF; LIFSFFLIFLNGFP;
IFSFFLIFLNGFPL; FSFFLIFLNGFPLN; SFFLIFLNGFPLNA;
FFLIFLNGFPLNAR; FLIFLNGFPLNARK; LIFLNGFPLNARKV;
IFLNGFPLNARKVD; FLNGFPLNARKVDK; LNGFPLNARKVDKE;
NGFPLNARKVDKEK; GFPLNARKVDKEKL; FPLNARKVDKEKLK;
PLNARKVDKEKLKD; LNARKVDKEKLKDF; NARKVDKEKLKDFV;
ARKVDKEKLKDFVN; RKVDKEKLKDFVNM; KVDKEKLKDFVNMD;
VDKEKLKDFVNMDL; DKEKLKDFVNMDLE; KEKLKDFVNMDLEF;
EKLKDFVNMDLEFV; KLKDFVNMDLEFVN; LKDFVNMDLEFVNY;
KDFVNMDLEFVNYK; DFVNMDLEFVNYKG; FVNMDLEFVNYKGP;
VNMDLEFVNYKGPY; NMDLEFVNYKGPYD; MDLEFVNYKGPYDS;
DLEFVNYKGPYDST; LEFVNYKGPYDSTN; EFVNYKGPYDSTNT;
FVNYKGPYDSTNTY; VNYKGPYDSTNTYE; NYKGPYDSTNTYEQ;
YKGPYDSTNTYEQI; KGPYDSTNTYEQIV; GPYDSTNTYEQIVG;
PYDSTNTYEQIVGI; YDSTNTYEQIVGIG; DSTNTYEQIVGIGE;
STNTYEQIVGIGEF; TNTYEQIVGIGEFL; NTYEQIVGIGEFLA;
TYEQIVGIGEFLAR; YEQIVGIGEFLARP; EQIVGIGEFLARPL;
QIVGIGEFLARPLT; IVGIGEFLARPLTN; VGIGEFLARPLTNS;
GIGEFLARPLTNSN; IGEFLARPLTNSNS; GEFLARPLTNSNSN;
EFLARPLTNSNSNS; FLARPLTNSNSNSS; LARPLTNSNSNSSY;
ARPLTNSNSNSSYY; RPLTNSNSNSSYYG; PLTNSNSNSSYYGK;
LTNSNSNSSYYGKY; TNSNSNSSYYGKYF; NSNSNSSYYGKYFI;
SNSNSSYYGKYFIN; NSNSSYYGKYFINR; SNSSYYGKYFINRF;
NSSYYGKYFINRFI; SSYYGKYFINRFID; SYYGKYFINRFIDD;

| | |
|---|---|
| YYGKYFINRFIDDQ; YGKYFINRFIDDQD; GKYFINRFIDDQDK; | |
| KYFINRFIDDQDKK; YFINRFIDDQDKKA; FINRFIDDQDKKAS; | |
| INRFIDDQDKKASV; NRFIDDQDKKASVD; RFIDDQDKKASVDV; | |
| FIDDQDKKASVDVF; IDDQDKKASVDVFS; DDQDKKASVDVFSI; | |
| DQDKKASVDVFSIS; QDKKASVDVFSISS; DKKASVDVFSISSK; | |
| KKASVDVFSISSKS; KASVDVFSISSKSE; ASVDVFSISSKSEL; | |
| SVDVFSISSKSELD; VDVFSISSKSELDS; DVFSISSKSELDSI; | |
| VFSISSKSELDSIL; FSISSKSELDSILN; SISSKSELDSILNL; | |
| ISSKSELDSILNLR; SSKSELDSILNLRR; SKSELDSILNLRRI; | |
| KSELDSILNLRRIL; SELDSILNLRRILT; ELDSILNLRRILTG; | |
| LDSILNLRRILTGY; DSILNLRRILTGYI; SILNLRRILTGYII; | |
| ILNLRRILTGYIIK; LNLRRILTGYIIKS; NLRRILTGYIIKSF; | |
| LRRILTGYIIKSFD; RRILTGYIIKSFDY; RILTGYIIKSFDYD; | |
| ILTGYIIKSFDYDR; LTGYIIKSFDYDRS; TGYIIKSFDYDRSS; | |
| GYIIKSFDYDRSSA; YIIKSFDYDRSSAE; IIKSFDYDRSSAEL; | |
| IKSFDYDRSSAELI; KSFDYDRSSAELIA; SFDYDRSSAELIAK; | |
| FDYDRSSAELIAKV; DYDRSSAELIAKVI; YDRSSAELIAKVIT; | |
| DRSSAELIAKVITI; RSSAELIAKVITIY; SSAELIAKVITIYN; | |
| SAELIAKVITIYNA; AELIAKVITIYNAV; ELIAKVITIYNAVY; | |
| LIAKVITIYNAVYR; IAKVITIYNAVYRG; AKVITIYNAVYRGD; | |
| KVITIYNAVYRGDL; VITIYNAVYRGDLD; ITIYNAVYRGDLDY; | |
| TIYNAVYRGDLDYY; IYNAVYRGDLDYYK; YNAVYRGDLDYYKG; | |
| NAVYRGDLDYYKGF; AVYRGDLDYYKGFY; VYRGDLDYYKGFYI; | |
| YRGDLDYYKGFYIE; RGDLDYYKGFYIEP; GDLDYYKGFYIEPA; | |
| DLDYYKGFYIEPAL; LDYYKGFYIEPALK; DYYKGFYIEPALKS; | |
| YYKGFYIEPALKSL; YKGFYIEPALKSLT; KGFYIEPALKSLTK; | |
| GFYIEPALKSLTKE; FYIEPALKSLTKEN; YIEPALKSLTKENA; | |
| IEPALKSLTKENAG; EPALKSLTKENAGL; PALKSLTKENAGLS; | |
| ALKSLTKENAGLSR; LKSLTKENAGLSRV; KSLTKENAGLSRVY; | |
| SLTKENAGLSRVYS; LTKENAGLSRVYSQ; TKENAGLSRVYSQW; | |
| KENAGLSRVYSQWA; ENAGLSRVYSQWAG; NAGLSRVYSQWAGK; | |
| AGLSRVYSQWAGKT; GLSRVYSQWAGKTQ; LSRVYSQWAGKTQI; | |
| SRVYSQWAGKTQIF; RVYSQWAGKTQIFI; VYSQWAGKTQIFIP; | |
| YSQWAGKTQIFIPL; SQWAGKTQIFIPLK; QWAGKTQIFIPLKK; | |
| WAGKTQIFIPLKKD; AGKTQIFIPLKKDI; GKTQIFIPLKKDIL; | |
| KTQIFIPLKKDILS; TQIFIPLKKDILSG; QIFIPLKKDILSGN; | |
| IFIPLKKDILSGNI; FIPLKKDILSGNIE; IPLKKDILSGNIES; | |
| PLKKDILSGNIESD; LKKDILSGNIESDI; KKDILSGNIESDID; | |
| KDILSGNIESDIDI; DILSGNIESDIDID; ILSGNIESDIDIDS; | |
| LSGNIESDIDIDSL; SGNIESDIDIDSLV; GNIESDIDIDSLVT; | |
| NIESDIDIDSLVTD; IESDIDIDSLVTDK; ESDIDIDSLVTDKV; | |
| SDIDIDSLVTDKVI; DIDIDSLVTDKVIA; IDIDSLVTDKVIAA; | |
| DIDSLVTDKVIAAL; IDSLVTDKVIAALL; DSLVTDKVIAALLS; | |
| SLVTDKVIAALLSE; LVTDKVIAALLSEN; VTDKVIAALLSENE; | |
| TDKVIAALLSENEA; DKVIAALLSENEAG; KVIAALLSENEAGV; | |
| VIAALLSENEAGVN; IAALLSENEAGVNF; AALLSENEAGVNFA; | |
| ALLSENEAGVNFAR; LLSENEAGVNFARD; LSENEAGVNFARDI; | |
| SENEAGVNFARDIT; ENEAGVNFARDITD; NEAGVNFARDITDI; | |
| EAGVNFARDITDIQ; AGVNFARDITDIQG; GVNFARDITDIQGE; | |
| VNFARDITDIQGET; NFARDITDIQGETH; FARDITDIQGETHK; | |
| ARDITDIQGETHKA; RDITDIQGETHKAD; DITDIQGETHKADQ; | |

| | | |
|---|---|---|
| ITDIQGETHKADQD; | TDIQGETHKADQDK; | DIQGETHKADQDKI; |
| IQGETHKADQDKID; | QGETHKADQDKIDT; | GETHKADQDKIDTE; |
| ETHKADQDKIDTEL; | THKADQDKIDTELD; | HKADQDKIDTELDN; |
| KADQDKIDTELDNI; | ADQDKIDTELDNIH; | DQDKIDTELDNIHE; |
| QDKIDTELDNIHES; | DKIDTELDNIHESD; | KIDTELDNIHESDS; |
| IDTELDNIHESDSN; | DTELDNIHESDSNI; | TELDNIHESDSNIT; |
| ELDNIHESDSNITE; | LDNIHESDSNITET; | DNIHESDSNITETI; |
| NIHESDSNITETIE; | IHESDSNITETIEN; | HESDSNITETIENL; |
| ESDSNITETIENLR; | SDSNITETIENLRD; | DSNITETIENLRDQ; |
| SNITETIENLRDQL; | NITETIENLRDQLE; | ITETIENLRDQLEK; |
| TETIENLRDQLEKA; | ETIENLRDQLEKAT; | TIENLRDQLEKATD; |
| IENLRDQLEKATDE; | ENLRDQLEKATDEE; | NLRDQLEKATDEEH; |
| LRDQLEKATDEEHK; | RDQLEKATDEEHKK; | DQLEKATDEEHKKE; |
| QLEKATDEEHKKEI; | LEKATDEEHKKEIE; | EKATDEEHKKEIES; |
| KATDEEHKKEIESQ; | ATDEEHKKEIESQV; | TDEEHKKEIESQVD; |
| DEEHKKEIESQVDA; | EEHKKEIESQVDAK; | EHKKEIESQVDAKK; |
| HKKEIESQVDAKKK; | KKEIESQVDAKKKE; | KEIESQVDAKKKEK; |
| EIESQVDAKKKEKE; | IESQVDAKKKEKEE; | ESQVDAKKKEKEEL; |
| SQVDAKKKEKEELD; | QVDAKKKEKEELDK; | VDAKKKEKEELDKK; |
| DAKKKEKEELDKKA; | AKKKEKEELDKKAI; | KKKEKEELDKKAIN; |
| KKEKEELDKKAINL; | KEKEELDKKAINLD; | EKEELDKKAINLDK; |
| KEELDKKAINLDKA; | EELDKKAINLDKAQ; | ELDKKAINLDKAQQ; |
| LDKKAINLDKAQQK; | DKKAINLDKAQQKL; | KKAINLDKAQQKLD; |
| KAINLDKAQQKLDS; | AINLDKAQQKLDSA; | INLDKAQQKLDSAE; |
| NLDKAQQKLDSAED; | LDKAQQKLDSAEDN; | DKAQQKLDSAEDNL; |
| KAQQKLDSAEDNLD; | AQQKLDSAEDNLDV; | QQKLDSAEDNLDVQ; |
| QKLDSAEDNLDVQR; | KLDSAEDNLDVQRD; | LDSAEDNLDVQRDT; |
| DSAEDNLDVQRDTV; | SAEDNLDVQRDTVR; | AEDNLDVQRDTVRE; |
| EDNLDVQRDTVREK; | DNLDVQRDTVREKI; | NLDVQRDTVREKIQ; |
| LDVQRDTVREKIQE; | DVQRDTVREKIQED; | VQRDTVREKIQEDI; |
| QRDTVREKIQEDIN; | RDTVREKIQEDINE; | DTVREKIQEDINEI; |
| TVREKIQEDINEIN; | VREKIQEDINEINK; | REKIQEDINEINKE; |
| EKIQEDINEINKEK; | KIQEDINEINKEKN; | IQEDINEINKEKNL; |
| QEDINEINKEKNLP; | EDINEINKEKNLPK; | DINEINKEKNLPKP; |
| INEINKEKNLPKPG; | NEINKEKNLPKPGD; | EINKEKNLPKPGDV; |
| INKEKNLPKPGDVS; | NKEKNLPKPGDVSS; | KEKNLPKPGDVSSP; |
| EKNLPKPGDVSSPK; | KNLPKPGDVSSPKV; | NLPKPGDVSSPKVD; |
| LPKPGDVSSPKVDK; | PKPGDVSSPKVDKQ; | KPGDVSSPKVDKQL; |
| PGDVSSPKVDKQLQ; | GDVSSPKVDKQLQI; | DVSSPKVDKQLQIK; |
| VSSPKVDKQLQIKE; | SSPKVDKQLQIKES; | SPKVDKQLQIKESL; |
| PKVDKQLQIKESLE; | KVDKQLQIKESLED; | VDKQLQIKESLEDL; |
| DKQLQIKESLEDLQ; | KQLQIKESLEDLQE; | QLQIKESLEDLQEQ; |
| LQIKESLEDLQEQL; | QIKESLEDLQEQLK; | IKESLEDLQEQLKE; |
| KESLEDLQEQLKEA; | ESLEDLQEQLKEAG; | SLEDLQEQLKEAGD; |
| LEDLQEQLKEAGDE; | EDLQEQLKEAGDEN; | DLQEQLKEAGDENQ; |
| LQEQLKEAGDENQK; | QEQLKEAGDENQKR; | EQLKEAGDENQKRE; |
| QLKEAGDENQKREI; | LKEAGDENQKREIE; | KEAGDENQKREIEK; |
| EAGDENQKREIEKQ; | AGDENQKREIEKQI; | GDENQKREIEKQIE; |
| DENQKREIEKQIEI; | ENQKREIEKQIEIK; | NQKREIEKQIEIKK; |
| QKREIEKQIEIKKR; | KREIEKQIEIKKRD; | REIEKQIEIKKRDE; |
| EIEKQIEIKKRDEE; | IEKQIEIKKRDEEL; | EKQIEIKKRDEELL; |

| | | |
|---|---|---|
| KQIEIKKRDEELLK; | QIEIKKRDEELLKS; | IEIKKRDEELLKSK; |
| EIKKRDEELLKSKD; | IKKRDEELLKSKDG; | KKRDEELLKSKDGK; |
| KRDEELLKSKDGKV; | RDEELLKSKDGKVS; | DEELLKSKDGKVSK; |
| EELLKSKDGKVSKD; | ELLKSKDGKVSKDY; | LLKSKDGKVSKDYE; |
| LKSKDGKVSKDYEA; | KSKDGKVSKDYEAL; | SKDGKVSKDYEALD; |
| KDGKVSKDYEALDL; | DGKVSKDYEALDLD; | GKVSKDYEALDLDR; |
| KVSKDYEALDLDRE; | VSKDYEALDLDREL; | SKDYEALDLDRELS; |
| KDYEALDLDRELSK; | DYEALDLDRELSKA; | YEALDLDRELSKAS; |
| EALDLDRELSKASS; | ALDLDRELSKASSK; | LDLDRELSKASSKE; |
| DLDRELSKASSKEK; | LDRELSKASSKEKS; | DRELSKASSKEKSK; |
| RELSKASSKEKSKV; | ELSKASSKEKSKVK; | LSKASSKEKSKVKE; |
| SKASSKEKSKVKEE; | KASSKEKSKVKEEE; | ASSKEKSKVKEEEI; |
| SSKEKSKVKEEEIT; | SKEKSKVKEEEITK; | KEKSKVKEEEITKG; |
| EKSKVKEEEITKGK; | KSKVKEEEITKGKS; | SKVKEEEITKGKSR; |
| KVKEEEITKGKSRA; | VKEEEITKGKSRAS; | KEEEITKGKSRASL; |
| EEEITKGKSRASLG; | EEITKGKSRASLGD; | EITKGKSRASLGDL; |
| ITKGKSRASLGDLN; | TKGKSRASLGDLNN; | KGKSRASLGDLNND; |
| GKSRASLGDLNNDK; | KSRASLGDLNNDKN; | SRASLGDLNNDKNL; |
| RASLGDLNNDKNLM; | ASLGDLNNDKNLML; | SLGDLNNDKNLMLP; |
| LGDLNNDKNLMLPE; | GDLNNDKNLMLPED; | DLNNDKNLMLPEDQ; |
| LNNDKNLMLPEDQK; | NNDKNLMLPEDQKL; | NDKNLMLPEDQKLP; |
| DKNLMLPEDQKLPE; | KNLMLPEDQKLPED; | NLMLPEDQKLPEDK; |
| LMLPEDQKLPEDKK; | MLPEDQKLPEDKKL; | LPEDQKLPEDKKLD; |
| PEDQKLPEDKKLDS; | EDQKLPEDKKLDSK; | DQKLPEDKKLDSKL; |
| QKLPEDKKLDSKLD; | KLPEDKKLDSKLDG; | LPEDKKLDSKLDGK; |
| PEDKKLDSKLDGKK; | EDKKLDSKLDGKKE; | DKKLDSKLDGKKEF; |
| KKLDSKLDGKKEFK; | KLDSKLDGKKEFKP; | LDSKLDGKKEFKPV; |
| DSKLDGKKEFKPVS; | SKLDGKKEFKPVSE; | KLDGKKEFKPVSEV; |
| LDGKKEFKPVSEVE; | DGKKEFKPVSEVEK; | GKKEFKPVSEVEKL; |
| KKEFKPVSEVEKLD; | KEFKPVSEVEKLDK; | EFKPVSEVEKLDKI; |
| FKPVSEVEKLDKIS; | KPVSEVEKLDKISK; | PVSEVEKLDKISKS; |
| VSEVEKLDKISKSN; | SEVEKLDKISKSNN; | EVEKLDKISKSNNN; |
| VEKLDKISKSNNNE; | EKLDKISKSNNNEV; | KLDKISKSNNNEVG; |
| LDKISKSNNNEVGK; | DKISKSNNNEVGKL; | KISKSNNNEVGKLS; |
| ISKSNNNEVGKLSP; | SKSNNNEVGKLSPL; | KSNNNEVGKLSPLD; |
| SNNNEVGKLSPLDK; | NNNEVGKLSPLDKP; | NNEVGKLSPLDKPS; |
| NEVGKLSPLDKPSY; | EVGKLSPLDKPSYD; | VGKLSPLDKPSYDD; |
| GKLSPLDKPSYDDI; | KLSPLDKPSYDDID; | LSPLDKPSYDDIDS; |
| SPLDKPSYDDIDSK; | PLDKPSYDDIDSKE; | LDKPSYDDIDSKEE; |
| DKPSYDDIDSKEEV; | KPSYDDIDSKEEVD; | PSYDDIDSKEEVDN; |
| SYDDIDSKEEVDNK; | YDDIDSKEEVDNKA; | DDIDSKEEVDNKAI; |
| DIDSKEEVDNKAIN; | IDSKEEVDNKAINL; | DSKEEVDNKAINLQ; |
| SKEEVDNKAINLQK; | KEEVDNKAINLQKI; | EEVDNKAINLQKID; |
| EVDNKAINLQKIDP; | VDNKAINLQKIDPK; | DNKAINLQKIDPKV; |
| NKAINLQKIDPKVK; | KAINLQKIDPKVKD; | AINLQKIDPKVKDQ; |
| INLQKIDPKVKDQT; | NLQKIDPKVKDQTT; | LQKIDPKVKDQTTS; |
| QKIDPKVKDQTTSL; | KIDPKVKDQTTSLN; | IDPKVKDQTTSLNE; |
| DPKVKDQTTSLNED; | PKVKDQTTSLNEDL; | KVKDQTTSLNEDLD; |
| VKDQTTSLNEDLDK; | KDQTTSLNEDLDKD; | DQTTSLNEDLDKDL; |
| QTTSLNEDLDKDLT; | TTSLNEDLDKDLTT; | TSLNEDLDKDLTTM; |
| SLNEDLDKDLTTMS; | LNEDLDKDLTTMSI; | NEDLDKDLTTMSID; |

EDLDKDLTTMSIDS; DLDKDLTTMSIDSS; LDKDLTTMSIDSSS;
DKDLTTMSIDSSSP; KDLTTMSIDSSSPV; DLTTMSIDSSSPVF;
LTTMSIDSSSPVFL; TTMSIDSSSPVFLE; TMSIDSSSPVFLEV;
MSIDSSSPVFLEVI; SIDSSSPVFLEVID; IDSSSPVFLEVIDP;
DSSSPVFLEVIDPI; SSSPVFLEVIDPIT; SSPVFLEVIDPITN;
SPVFLEVIDPITNL; PVFLEVIDPITNLG; VFLEVIDPITNLGT;
FLEVIDPITNLGTL; LEVIDPITNLGTLQ; EVIDPITNLGTLQL;
VIDPITNLGTLQLI; IDPITNLGTLQLID; DPITNLGTLQLIDL;
PITNLGTLQLIDLN; ITNLGTLQLIDLNT; TNLGTLQLIDLNTG;
NLGTLQLIDLNTGV; LGTLQLIDLNTGVR; GTLQLIDLNTGVRL;
TLQLIDLNTGVRLK; LQLIDLNTGVRLKE; QLIDLNTGVRLKES;
LIDLNTGVRLKEST; IDLNTGVRLKESTQ; DLNTGVRLKESTQQ;
LNTGVRLKESTQQG; NTGVRLKESTQQGI; TGVRLKESTQQGIQ;
GVRLKESTQQGIQR; VRLKESTQQGIQRY; RLKESTQQGIQRYG;
LKESTQQGIQRYGI; KESTQQGIQRYGIY; ESTQQGIQRYGIYE;
STQQGIQRYGIYER; TQQGIQRYGIYERE; QQGIQRYGIYEREK;
QGIQRYGIYEREKD; GIQRYGIYEREKDL; IQRYGIYEREKDLV;
QRYGIYEREKDLVV; RYGIYEREKDLVVI; YGIYEREKDLVVIK;
GIYEREKDLVVIKM; IYEREKDLVVIKMD; YEREKDLVVIKMDS;
EREKDLVVIKMDSG; REKDLVVIKMDSGK; EKDLVVIKMDSGKA;
KDLVVIKMDSGKAK; DLVVIKMDSGKAKL; LVVIKMDSGKAKLQ;
VVIKMDSGKAKLQI; VIKMDSGKAKLQIL; IKMDSGKAKLQILN;
KMDSGKAKLQILNK; MDSGKAKLQILNKL; DSGKAKLQILNKLE;
SGKAKLQILNKLEN; GKAKLQILNKLENL; KAKLQILNKLENLK;
AKLQILNKLENLKV; KLQILNKLENLKVV; LQILNKLENLKVVS;
QILNKLENLKVVSE; ILNKLENLKVVSES; LNKLENLKVVSESN;
NKLENLKVVSESNF; KLENLKVVSESNFE; LENLKVVSESNFEI;
ENLKVVSESNFEIN; NLKVVSESNFEINK; LKVVSESNFEINKN;
KVVSESNFEINKNS; VVSESNFEINKNSS; VSESNFEINKNSSL;
SESNFEINKNSSLY; ESNFEINKNSSLYV; SNFEINKNSSLYVD;
NFEINKNSSLYVDS; FEINKNSSLYVDSK; EINKNSSLYVDSKM;
INKNSSLYVDSKMI; NKNSSLYVDSKMIL; KNSSLYVDSKMILA;
NSSLYVDSKMILAA; SSLYVDSKMILAAV; SLYVDSKMILAAVR;
LYVDSKMILAAVRD; YVDSKMILAAVRDK; VDSKMILAAVRDKD;
DSKMILAAVRDKDD; SKMILAAVRDKDDS; KMILAAVRDKDDSN;
MILAAVRDKDDSNA; ILAAVRDKDDSNAW; LAAVRDKDDSNAWR;
AAVRDKDDSNAWRL; AVRDKDDSNAWRLA; VRDKDDSNAWRLAK;
RDKDDSNAWRLAKF; DKDDSNAWRLAKFS; KDDSNAWRLAKFSP;
DDSNAWRLAKFSPK; DSNAWRLAKFSPKN; SNAWRLAKFSPKNL;
NAWRLAKFSPKNLD; AWRLAKFSPKNLDE; WRLAKFSPKNLDEF;
RLAKFSPKNLDEFI; LAKFSPKNLDEFIL; AKFSPKNLDEFILS;
KFSPKNLDEFILSE; FSPKNLDEFILSEN; SPKNLDEFILSENK;
PKNLDEFILSENKI; KNLDEFILSENKIL; NLDEFILSENKILP;
LDEFILSENKILPF; DEFILSENKILPFT; EFILSENKILPFTS;
FILSENKILPFTSF; ILSENKILPFTSFS; LSENKILPFTSFSV;
SENKILPFTSFSVR; ENKILPFTSFSVRK; NKILPFTSFSVRKN;
KILPFTSFSVRKNF; ILPFTSFSVRKNFI; LPFTSFSVRKNFIY;
PFTSFSVRKNFIYL; FTSFSVRKNFIYLQ; TSFSVRKNFIYLQD;
SFSVRKNFIYLQDE; FSVRKNFIYLQDEL; SVRKNFIYLQDELK;
VRKNFIYLQDELKN; RKNFIYLQDELKNL; KNFIYLQDELKNLV;
NFIYLQDELKNLVI; FIYLQDELKNLVIL; IYLQDELKNLVILD;

YLQDELKNLVILDV; LQDELKNLVILDVN; QDELKNLVILDVNT;
DELKNLVILDVNTL; ELKNLVILDVNTLK; LKNLVILDVNTLKK;
KNLVILDVNTLKKV; NLVILDVNTLKKVK;

15 mers:
MKKMLLIFSFFLIFL; KKMLLIFSFFLIFLN; KMLLIFSFFLIFLNG;
MLLIFSFFLIFLNGF; LLIFSFFLIFLNGFP; LIFSFFLIFLNGFPL;
IFSFFLIFLNGFPLN; FSFFLIFLNGFPLNA; SFFLIFLNGFPLNAR;
FFLIFLNGFPLNARK; FLIFLNGFPLNARKV; LIFLNGFPLNARKVD;
IFLNGFPLNARKVDK; FLNGFPLNARKVDKE; LNGFPLNARKVDKEK;
NGFPLNARKVDKEKL; GFPLNARKVDKEKLK; FPLNARKVDKEKLKD;
PLNARKVDKEKLKDF; LNARKVDKEKLKDFV; NARKVDKEKLKDFVN;
ARKVDKEKLKDFVNM; RKVDKEKLKDFVNMD; KVDKEKLKDFVNMDL;
VDKEKLKDFVNMDLE; DKEKLKDFVNMDLEF; KEKLKDFVNMDLEFV;
EKLKDFVNMDLEFVN; KLKDFVNMDLEFVNY; LKDFVNMDLEFVNYK;
KDFVNMDLEFVNYKG; DFVNMDLEFVNYKGP; FVNMDLEFVNYKGPY;
VNMDLEFVNYKGPYD; NMDLEFVNYKGPYDS; MDLEFVNYKGPYDST;
DLEFVNYKGPYDSTN; LEFVNYKGPYDSTNT; EFVNYKGPYDSTNTY;
FVNYKGPYDSTNTYE; VNYKGPYDSTNTYEQ; NYKGPYDSTNTYEQI;
YKGPYDSTNTYEQIV; KGPYDSTNTYEQIVG; GPYDSTNTYEQIVGI;
PYDSTNTYEQIVGIG; YDSTNTYEQIVGIGE; DSTNTYEQIVGIGEF;
STNTYEQIVGIGEFL; TNTYEQIVGIGEFLA; NTYEQIVGIGEFLAR;
TYEQIVGIGEFLARP; YEQIVGIGEFLARPL; EQIVGIGEFLARPLT;
QIVGIGEFLARPLTN; IVGIGEFLARPLTNS; VGIGEFLARPLTNSN;
GIGEFLARPLTNSNS; IGEFLARPLTNSNSN; GEFLARPLTNSNSNS;
EFLARPLTNSNSNSS; FLARPLTNSNSNSSY; LARPLTNSNSNSSYY;
ARPLTNSNSNSSYYG; RPLTNSNSNSSYYGK; PLTNSNSNSSYYGKY;
LTNSNSNSSYYGKYF; TNSNSNSSYYGKYFI; NSNSNSSYYGKYFIN;
SNSNSSYYGKYFINR; NSNSSYYGKYFINRF; SNSSYYGKYFINRFI;
NSSYYGKYFINRFID; SSYYGKYFINRFIDD; SYYGKYFINRFIDDQ;
YYGKYFINRFIDDQD; YGKYFINRFIDDQDK; GKYFINRFIDDQDKK;
KYFINRFIDDQDKKA; YFINRFIDDQDKKAS; FINRFIDDQDKKASV;
INRFIDDQDKKASVD; NRFIDDQDKKASVDV; RFIDDQDKKASVDVF;
FIDDQDKKASVDVFS; IDDQDKKASVDVFSI; DDQDKKASVDVFSIS;
DQDKKASVDVFSISS; QDKKASVDVFSISSK; DKKASVDVFSISSKS;
KKASVDVFSISSKSE; KASVDVFSISSKSEL; ASVDVFSISSKSELD;
SVDVFSISSKSELDS; VDVFSISSKSELDSI; DVFSISSKSELDSIL;
VFSISSKSELDSILN; FSISSKSELDSILNL; SISSKSELDSILNLR;
ISSKSELDSILNLRR; SSKSELDSILNLRRI; SKSELDSILNLRRIL;
KSELDSILNLRRILT; SELDSILNLRRILTG; ELDSILNLRRILTGY;
LDSILNLRRILTGYI; DSILNLRRILTGYII; SILNLRRILTGYIIK;
ILNLRRILTGYIIKS; LNLRRILTGYIIKSF; NLRRILTGYIIKSFD;
LRRILTGYIIKSFDY; RRILTGYIIKSFDYD; RILTGYIIKSFDYDR;
ILTGYIIKSFDYDRS; LTGYIIKSFDYDRSS; TGYIIKSFDYDRSSA;
GYIIKSFDYDRSSAE; YIIKSFDYDRSSAEL; IIKSFDYDRSSAELI;
IKSFDYDRSSAELIA; KSFDYDRSSAELIAK; SFDYDRSSAELIAKV;
FDYDRSSAELIAKVI; DYDRSSAELIAKVIT; YDRSSAELIAKVITI;
DRSSAELIAKVITIY; RSSAELIAKVITIYN; SSAELIAKVITIYNA;
SAELIAKVITIYNAV; AELIAKVITIYNAVY; ELIAKVITIYNAVYR;
LIAKVITIYNAVYRG; IAKVITIYNAVYRGD; AKVITIYNAVYRGDL;
KVITIYNAVYRGDLD; VITIYNAVYRGDLDY; ITIYNAVYRGDLDYY;

TIYNAVYRGDLDYYK; IYNAVYRGDLDYYKG; YNAVYRGDLDYYKGF;
NAVYRGDLDYYKGFY; AVYRGDLDYYKGFYI; VYRGDLDYYKGFYIE;
YRGDLDYYKGFYIEP; RGDLDYYKGFYIEPA; GDLDYYKGFYIEPAL;
DLDYYKGFYIEPALK; LDYYKGFYIEPALKS; DYYKGFYIEPALKSL;
YYKGFYIEPALKSLT; YKGFYIEPALKSLTK; KGFYIEPALKSLTKE;
GFYIEPALKSLTKEN; FYIEPALKSLTKENA; YIEPALKSLTKENAG;
IEPALKSLTKENAGL; EPALKSLTKENAGLS; PALKSLTKENAGLSR;
ALKSLTKENAGLSRV; LKSLTKENAGLSRVY; KSLTKENAGLSRVYS;
SLTKENAGLSRVYSQ; LTKENAGLSRVYSQW; TKENAGLSRVYSQWA;
KENAGLSRVYSQWAG; ENAGLSRVYSQWAGK; NAGLSRVYSQWAGKT;
AGLSRVYSQWAGKTQ; GLSRVYSQWAGKTQI; LSRVYSQWAGKTQIF;
SRVYSQWAGKTQIFI; RVYSQWAGKTQIFIP; VYSQWAGKTQIFIPL;
YSQWAGKTQIFIPLK; SQWAGKTQIFIPLKK; QWAGKTQIFIPLKKD;
WAGKTQIFIPLKKDI; AGKTQIFIPLKKDIL; GKTQIFIPLKKDILS;
KTQIFIPLKKDILSG; TQIFIPLKKDILSGN; QIFIPLKKDILSGNI;
IFIPLKKDILSGNIE; FIPLKKDILSGNIES; IPLKKDILSGNIESD;
PLKKDILSGNIESDI; LKKDILSGNIESDID; KKDILSGNIESDIDI;
KDILSGNIESDIDID; DILSGNIESDIDIDS; ILSGNIESDIDIDSL;
LSGNIESDIDIDSLV; SGNIESDIDIDSLVT; GNIESDIDIDSLVTD;
NIESDIDIDSLVTDK; IESDIDIDSLVTDKV; ESDIDIDSLVTDKVI;
SDIDIDSLVTDKVIA; DIDIDSLVTDKVIAA; IDIDSLVTDKVIAAL;
DIDSLVTDKVIAALL; IDSLVTDKVIAALLS; DSLVTDKVIAALLSE;
SLVTDKVIAALLSEN; LVTDKVIAALLSENE; VTDKVIAALLSENEA;
TDKVIAALLSENEAG; DKVIAALLSENEAGV; KVIAALLSENEAGVN;
VIAALLSENEAGVNF; IAALLSENEAGVNFA; AALLSENEAGVNFAR;
ALLSENEAGVNFARD; LLSENEAGVNFARDI; LSENEAGVNFARDIT;
SENEAGVNFARDITD; ENEAGVNFARDITDI; NEAGVNFARDITDIQ;
EAGVNFARDITDIQG; AGVNFARDITDIQGE; GVNFARDITDIQGET;
VNFARDITDIQGETH; NFARDITDIQGETHK; FARDITDIQGETHKA;
ARDITDIQGETHKAD; RDITDIQGETHKADQ; DITDIQGETHKADQD;
ITDIQGETHKADQDK; TDIQGETHKADQDKI; DIQGETHKADQDKID;
IQGETHKADQDKIDT; QGETHKADQDKIDTE; GETHKADQDKIDTEL;
ETHKADQDKIDTELD; THKADQDKIDTELDN; HKADQDKIDTELDNI;
KADQDKIDTELDNIH; ADQDKIDTELDNIHE; DQDKIDTELDNIHES;
QDKIDTELDNIHESD; DKIDTELDNIHESDS; KIDTELDNIHESDSN;
IDTELDNIHESDSNI; DTELDNIHESDSNIT; TELDNIHESDSNITE;
ELDNIHESDSNITET; LDNIHESDSNITETI; DNIHESDSNITETIE;
NIHESDSNITETIEN; IHESDSNITETIENL; HESDSNITETIENLR;
ESDSNITETIENLRD; SDSNITETIENLRDQ; DSNITETIENLRDQL;
SNITETIENLRDQLE; NITETIENLRDQLEK; ITETIENLRDQLEKA;
TETIENLRDQLEKAT; ETIENLRDQLEKATD; TIENLRDQLEKATDE;
IENLRDQLEKATDEE; ENLRDQLEKATDEEH; NLRDQLEKATDEEHK;
LRDQLEKATDEEHKK; RDQLEKATDEEHKKE; DQLEKATDEEHKKEI;
QLEKATDEEHKKEIE; LEKATDEEHKKEIES; EKATDEEHKKEIESQ;
KATDEEHKKEIESQV; ATDEEHKKEIESQVD; TDEEHKKEIESQVDA;
DEEHKKEIESQVDAK; EEHKKEIESQVDAKK; EHKKEIESQVDAKKK;
HKKEIESQVDAKKKE; KKEIESQVDAKKKEK; KEIESQVDAKKKEKE;
EIESQVDAKKKEKEE; IESQVDAKKKEKEEL; ESQVDAKKKEKEELD;
SQVDAKKKEKEELDK; QVDAKKKEKEELDKK; VDAKKKEKEELDKKA;
DAKKKEKEELDKKAI; AKKKEKEELDKKAIN; KKKEKEELDKKAINL;
KKEKEELDKKAINLD; KEKEELDKKAINLDK; EKEELDKKAINLDKA;

KEELDKKAINLDKAQ; EELDKKAINLDKAQQ; ELDKKAINLDKAQQK;
LDKKAINLDKAQQKL; DKKAINLDKAQQKLD; KKAINLDKAQQKLDS;
KAINLDKAQQKLDSA; AINLDKAQQKLDSAE; INLDKAQQKLDSAED;
NLDKAQQKLDSAEDN; LDKAQQKLDSAEDNL; DKAQQKLDSAEDNLD;
KAQQKLDSAEDNLDV; AQQKLDSAEDNLDVQ; QQKLDSAEDNLDVQR;
QKLDSAEDNLDVQRD; KLDSAEDNLDVQRDT; LDSAEDNLDVQRDTV;
DSAEDNLDVQRDTVR; SAEDNLDVQRDTVRE; AEDNLDVQRDTVREK;
EDNLDVQRDTVREKI; DNLDVQRDTVREKIQ; NLDVQRDTVREKIQE;
LDVQRDTVREKIQED; DVQRDTVREKIQEDI; VQRDTVREKIQEDIN;
QRDTVREKIQEDINE; RDTVREKIQEDINEI; DTVREKIQEDINEIN;
TVREKIQEDINEINK; VREKIQEDINEINKE; REKIQEDINEINKEK;
EKIQEDINEINKEKN; KIQEDINEINKEKNL; IQEDINEINKEKNLP;
QEDINEINKEKNLPK; EDINEINKEKNLPKP; DINEINKEKNLPKPG;
INEINKEKNLPKPGD; NEINKEKNLPKPGDV; EINKEKNLPKPGDVS;
INKEKNLPKPGDVSS; NKEKNLPKPGDVSSP; KEKNLPKPGDVSSPK;
EKNLPKPGDVSSPKV; KNLPKPGDVSSPKVD; NLPKPGDVSSPKVDK;
LPKPGDVSSPKVDKQ; PKPGDVSSPKVDKQL; KPGDVSSPKVDKQLQ;
PGDVSSPKVDKQLQI; GDVSSPKVDKQLQIK; DVSSPKVDKQLQIKE;
VSSPKVDKQLQIKES; SSPKVDKQLQIKESL; SPKVDKQLQIKESLE;
PKVDKQLQIKESLED; KVDKQLQIKESLEDL; VDKQLQIKESLEDLQ;
DKQLQIKESLEDLQE; KQLQIKESLEDLQEQ; QLQIKESLEDLQEQL;
LQIKESLEDLQEQLK; QIKESLEDLQEQLKE; IKESLEDLQEQLKEA;
KESLEDLQEQLKEAG; ESLEDLQEQLKEAGD; SLEDLQEQLKEAGDE;
LEDLQEQLKEAGDEN; EDLQEQLKEAGDENQ; DLQEQLKEAGDENQK;
LQEQLKEAGDENQKR; QEQLKEAGDENQKRE; EQLKEAGDENQKREI;
QLKEAGDENQKREIE; LKEAGDENQKREIEK; KEAGDENQKREIEKQ;
EAGDENQKREIEKQI; AGDENQKREIEKQIE; GDENQKREIEKQIEI;
DENQKREIEKQIEIK; ENQKREIEKQIEIKK; NQKREIEKQIEIKKR;
QKREIEKQIEIKKRD; KREIEKQIEIKKRDE; REIEKQIEIKKRDEE;
EIEKQIEIKKRDEEL; IEKQIEIKKRDEELL; EKQIEIKKRDEELLK;
KQIEIKKRDEELLKS; QIEIKKRDEELLKSK; IEIKKRDEELLKSKD;
EIKKRDEELLKSKDG; IKKRDEELLKSKDGK; KKRDEELLKSKDGKV;
KRDEELLKSKDGKVS; RDEELLKSKDGKVSK; DEELLKSKDGKVSKD;
EELLKSKDGKVSKDY; ELLKSKDGKVSKDYE; LLKSKDGKVSKDYEA;
LKSKDGKVSKDYEAL; KSKDGKVSKDYEALD; SKDGKVSKDYEALDL;
KDGKVSKDYEALDLD; DGKVSKDYEALDLDR; GKVSKDYEALDLDRE;
KVSKDYEALDLDREL; VSKDYEALDLDRELS; SKDYEALDLDRELSK;
KDYEALDLDRELSKA; DYEALDLDRELSKAS; YEALDLDRELSKASS;
EALDLDRELSKASSK; ALDLDRELSKASSKE; LDLDRELSKASSKEK;
DLDRELSKASSKEKS; LDRELSKASSKEKSK; DRELSKASSKEKSKV;
RELSKASSKEKSKVK; ELSKASSKEKSKVKE; LSKASSKEKSKVKEE;
SKASSKEKSKVKEEE; KASSKEKSKVKEEEI; ASSKEKSKVKEEEIT;
SSKEKSKVKEEEITK; SKEKSKVKEEEITKG; KEKSKVKEEEITKGK;
EKSKVKEEEITKGKS; KSKVKEEEITKGKSR; SKVKEEEITKGKSRA;
KVKEEEITKGKSRAS; VKEEEITKGKSRASL; KEEEITKGKSRASLG;
EEEITKGKSRASLGD; EEITKGKSRASLGDL; EITKGKSRASLGDLN;
ITKGKSRASLGDLNN; TKGKSRASLGDLNND; KGKSRASLGDLNNDK;
GKSRASLGDLNNDKN; KSRASLGDLNNDKNL; SRASLGDLNNDKNLM;
RASLGDLNNDKNLML; ASLGDLNNDKNLMLP; SLGDLNNDKNLMLPE;
LGDLNNDKNLMLPED; GDLNNDKNLMLPEDQ; DLNNDKNLMLPEDQK;
LNNDKNLMLPEDQKL; NNDKNLMLPEDQKLP; NDKNLMLPEDQKLPE;

DKNLMLPEDQKLPED; KNLMLPEDQKLPEDK; NLMLPEDQKLPEDKK;
LMLPEDQKLPEDKKL; MLPEDQKLPEDKKLD; LPEDQKLPEDKKLDS;
PEDQKLPEDKKLDSK; EDQKLPEDKKLDSKL; DQKLPEDKKLDSKLD;
QKLPEDKKLDSKLDG; KLPEDKKLDSKLDGK; LPEDKKLDSKLDGKK;
PEDKKLDSKLDGKKE; EDKKLDSKLDGKKEF; DKKLDSKLDGKKEFK;
KKLDSKLDGKKEFKP; KLDSKLDGKKEFKPV; LDSKLDGKKEFKPVS;
DSKLDGKKEFKPVSE; SKLDGKKEFKPVSEV; KLDGKKEFKPVSEVE;
LDGKKEFKPVSEVEK; DGKKEFKPVSEVEKL; GKKEFKPVSEVEKLD;
KKEFKPVSEVEKLDK; KEFKPVSEVEKLDKI; EFKPVSEVEKLDKIS;
FKPVSEVEKLDKISK; KPVSEVEKLDKISKS; PVSEVEKLDKISKSN;
VSEVEKLDKISKSNN; SEVEKLDKISKSNNN; EVEKLDKISKSNNNE;
VEKLDKISKSNNNEV; EKLDKISKSNNNEVG; KLDKISKSNNNEVGK;
LDKISKSNNNEVGKL; DKISKSNNNEVGKLS; KISKSNNNEVGKLSP;
ISKSNNNEVGKLSPL; SKSNNNEVGKLSPLD; KSNNNEVGKLSPLDK;
SNNNEVGKLSPLDKP; NNNEVGKLSPLDKPS; NNEVGKLSPLDKPSY;
NEVGKLSPLDKPSYD; EVGKLSPLDKPSYDD; VGKLSPLDKPSYDDI;
GKLSPLDKPSYDDID; KLSPLDKPSYDDIDS; LSPLDKPSYDDIDSK;
SPLDKPSYDDIDSKE; PLDKPSYDDIDSKEE; LDKPSYDDIDSKEEV;
DKPSYDDIDSKEEVD; KPSYDDIDSKEEVDN; PSYDDIDSKEEVDNK;
SYDDIDSKEEVDNKA; YDDIDSKEEVDNKAI; DDIDSKEEVDNKAIN;
DIDSKEEVDNKAINL; IDSKEEVDNKAINLQ; DSKEEVDNKAINLQK;
SKEEVDNKAINLQKI; KEEVDNKAINLQKID; EEVDNKAINLQKIDP;
EVDNKAINLQKIDPK; VDNKAINLQKIDPKV; DNKAINLQKIDPKVK;
NKAINLQKIDPKVKD; KAINLQKIDPKVKDQ; AINLQKIDPKVKDQT;
INLQKIDPKVKDQTT; NLQKIDPKVKDQTTS; LQKIDPKVKDQTTSL;
QKIDPKVKDQTTSLN; KIDPKVKDQTTSLNE; IDPKVKDQTTSLNED;
DPKVKDQTTSLNEDL; PKVKDQTTSLNEDLD; KVKDQTTSLNEDLDK;
VKDQTTSLNEDLDKD; KDQTTSLNEDLDKDL; DQTTSLNEDLDKDLT;
QTTSLNEDLDKDLTT; TTSLNEDLDKDLTTM; TSLNEDLDKDLTTMS;
SLNEDLDKDLTTMSI; LNEDLDKDLTTMSID; NEDLDKDLTTMSIDS;
EDLDKDLTTMSIDSS; DLDKDLTTMSIDSSS; LDKDLTTMSIDSSSP;
DKDLTTMSIDSSSPV; KDLTTMSIDSSSPVF; DLTTMSIDSSSPVFL;
LTTMSIDSSSPVFLE; TTMSIDSSSPVFLEV; TMSIDSSSPVFLEVI;
MSIDSSSPVFLEVID; SIDSSSPVFLEVIDP; IDSSSPVFLEVIDPI;
DSSSPVFLEVIDPIT; SSSPVFLEVIDPITN; SSPVFLEVIDPITNL;
SPVFLEVIDPITNLG; PVFLEVIDPITNLGT; VFLEVIDPITNLGTL;
FLEVIDPITNLGTLQ; LEVIDPITNLGTLQL; EVIDPITNLGTLQLI;
VIDPITNLGTLQLID; IDPITNLGTLQLIDL; DPITNLGTLQLIDLN;
PITNLGTLQLIDLNT; ITNLGTLQLIDLNTG; TNLGTLQLIDLNTGV;
NLGTLQLIDLNTGVR; LGTLQLIDLNTGVRL; GTLQLIDLNTGVRLK;
TLQLIDLNTGVRLKE; LQLIDLNTGVRLKES; QLIDLNTGVRLKEST;
LIDLNTGVRLKESTQ; IDLNTGVRLKESTQQ; DLNTGVRLKESTQQG;
LNTGVRLKESTQQGI; NTGVRLKESTQQGIQ; TGVRLKESTQQGIQR;
GVRLKESTQQGIQRY; VRLKESTQQGIQRYG; RLKESTQQGIQRYGI;
LKESTQQGIQRYGIY; KESTQQGIQRYGIYE; ESTQQGIQRYGIYER;
STQQGIQRYGIYERE; TQQGIQRYGIYEREK; QQGIQRYGIYEREKD;
QGIQRYGIYEREKDL; GIQRYGIYEREKDLV; IQRYGIYEREKDLVV;
QRYGIYEREKDLVVI; RYGIYEREKDLVVIK; YGIYEREKDLVVIKM;
GIYEREKDLVVIKMD; IYEREKDLVVIKMDS; YEREKDLVVIKMDSG;
EREKDLVVIKMDSGK; REKDLVVIKMDSGKA; EKDLVVIKMDSGKAK;
KDLVVIKMDSGKAKL; DLVVIKMDSGKAKLQ; LVVIKMDSGKAKLQI;

VVIKMDSGKAKLQIL; VIKMDSGKAKLQILN; IKMDSGKAKLQILNK;
KMDSGKAKLQILNKL; MDSGKAKLQILNKLE; DSGKAKLQILNKLEN;
SGKAKLQILNKLENL; GKAKLQILNKLENLK; KAKLQILNKLENLKV;
AKLQILNKLENLKVV; KLQILNKLENLKVVS; LQILNKLENLKVVSE;
QILNKLENLKVVSES; ILNKLENLKVVSESN; LNKLENLKVVSESNF;
NKLENLKVVSESNFE; KLENLKVVSESNFEI; LENLKVVSESNFEIN;
ENLKVVSESNFEINK; NLKVVSESNFEINKN; LKVVSESNFEINKNS;
KVVSESNFEINKNSS; VVSESNFEINKNSSL; VSESNFEINKNSSLY;
SESNFEINKNSSLYV; ESNFEINKNSSLYVD; SNFEINKNSSLYVDS;
NFEINKNSSLYVDSK; FEINKNSSLYVDSKM; EINKNSSLYVDSKMI;
INKNSSLYVDSKMIL; NKNSSLYVDSKMILA; KNSSLYVDSKMILAA;
NSSLYVDSKMILAAV; SSLYVDSKMILAAVR; SLYVDSKMILAAVRD;
LYVDSKMILAAVRDK; YVDSKMILAAVRDKD; VDSKMILAAVRDKDD;
DSKMILAAVRDKDDS; SKMILAAVRDKDDSN; KMILAAVRDKDDSNA;
MILAAVRDKDDSNAW; ILAAVRDKDDSNAWR; LAAVRDKDDSNAWRL;
AAVRDKDDSNAWRLA; AVRDKDDSNAWRLAK; VRDKDDSNAWRLAKF;
RDKDDSNAWRLAKFS; DKDDSNAWRLAKFSP; KDDSNAWRLAKFSPK;
DDSNAWRLAKFSPKN; DSNAWRLAKFSPKNL; SNAWRLAKFSPKNLD;
NAWRLAKFSPKNLDE; AWRLAKFSPKNLDEF; WRLAKFSPKNLDEFI;
RLAKFSPKNLDEFIL; LAKFSPKNLDEFILS; AKFSPKNLDEFILSE;
KFSPKNLDEFILSEN; FSPKNLDEFILSENK; SPKNLDEFILSENKI;
PKNLDEFILSENKIL; KNLDEFILSENKILP; NLDEFILSENKILPF;
LDEFILSENKILPFT; DEFILSENKILPFTS; EFILSENKILPFTSF;
FILSENKILPFTSFS; ILSENKILPFTSFSV; LSENKILPFTSFSVR;
SENKILPFTSFSVRK; ENKILPFTSFSVRKN; NKILPFTSFSVRKNF;
KILPFTSFSVRKNFI; ILPFTSFSVRKNFIY; LPFTSFSVRKNFIYL;
PFTSFSVRKNFIYLQ; FTSFSVRKNFIYLQD; TSFSVRKNFIYLQDE;
SFSVRKNFIYLQDEL; FSVRKNFIYLQDELK; SVRKNFIYLQDELKN;
VRKNFIYLQDELKNL; RKNFIYLQDELKNLV; KNFIYLQDELKNLVI;
NFIYLQDELKNLVIL; FIYLQDELKNLVILD; IYLQDELKNLVILDV;
YLQDELKNLVILDVN; LQDELKNLVILDVNT; QDELKNLVILDVNTL;
DELKNLVILDVNTLK; ELKNLVILDVNTLKK; LKNLVILDVNTLKKV;
KNLVILDVNTLKKVK;

16 mers:
MKKMLLIFSFFLIFLN; KKMLLIFSFFLIFLNG; KMLLIFSFFLIFLNGF;
MLLIFSFFLIFLNGFP; LLIFSFFLIFLNGFPL; LIFSFFLIFLNGFPLN;
IFSFFLIFLNGFPLNA; FSFFLIFLNGFPLNAR; SFFLIFLNGFPLNARK;
FFLIFLNGFPLNARKV; FLIFLNGFPLNARKVD; LIFLNGFPLNARKVDK;
IFLNGFPLNARKVDKE; FLNGFPLNARKVDKEK; LNGFPLNARKVDKEKL;
NGFPLNARKVDKEKLK; GFPLNARKVDKEKLKD; FPLNARKVDKEKLKDF;
PLNARKVDKEKLKDFV; LNARKVDKEKLKDFVN; NARKVDKEKLKDFVNM;
ARKVDKEKLKDFVNMD; RKVDKEKLKDFVNMDL; KVDKEKLKDFVNMDLE;
VDKEKLKDFVNMDLEF; DKEKLKDFVNMDLEFV; KEKLKDFVNMDLEFVN;
EKLKDFVNMDLEFVNY; KLKDFVNMDLEFVNYK; LKDFVNMDLEFVNYKG;
KDFVNMDLEFVNYKGP; DFVNMDLEFVNYKGPY; FVNMDLEFVNYKGPYD;
VNMDLEFVNYKGPYDS; NMDLEFVNYKGPYDST; MDLEFVNYKGPYDSTN;
DLEFVNYKGPYDSTNT; LEFVNYKGPYDSTNTY; EFVNYKGPYDSTNTYE;
FVNYKGPYDSTNTYEQ; VNYKGPYDSTNTYEQI; NYKGPYDSTNTYEQIV;
YKGPYDSTNTYEQIVG; KGPYDSTNTYEQIVGI; GPYDSTNTYEQIVGIG;
PYDSTNTYEQIVGIGE; YDSTNTYEQIVGIGEF; DSTNTYEQIVGIGEFL;

| | | |
|---|---|---|
| STNTYEQIVGIGEFLA; | TNTYEQIVGIGEFLAR; | NTYEQIVGIGEFLARP; |
| TYEQIVGIGEFLARPL; | YEQIVGIGEFLARPLT; | EQIVGIGEFLARPLTN; |
| QIVGIGEFLARPLTNS; | IVGIGEFLARPLTNSN; | VGIGEFLARPLTNSNS; |
| GIGEFLARPLTNSNSN; | IGEFLARPLTNSNSNS; | GEFLARPLTNSNSNSS; |
| EFLARPLTNSNSNSSY; | FLARPLTNSNSNSSYY; | LARPLTNSNSNSSYYG; |
| ARPLTNSNSNSSYYGK; | RPLTNSNSNSSYYGKY; | PLTNSNSNSSYYGKYF; |
| LTNSNSNSSYYGKYFI; | TNSNSNSSYYGKYFIN; | NSNSNSSYYGKYFINR; |
| SNSNSSYYGKYFINRF; | NSNSSYYGKYFINRFI; | SNSSYYGKYFINRFID; |
| NSSYYGKYFINRFIDD; | SSYYGKYFINRFIDDQ; | SYYGKYFINRFIDDQD; |
| YYGKYFINRFIDDQDK; | YGKYFINRFIDDQDKK; | GKYFINRFIDDQDKKA; |
| KYFINRFIDDQDKKAS; | YFINRFIDDQDKKASV; | FINRFIDDQDKKASVD; |
| INRFIDDQDKKASVDV; | NRFIDDQDKKASVDVF; | RFIDDQDKKASVDVFS; |
| FIDDQDKKASVDVFSI; | IDDQDKKASVDVFSIS; | DDQDKKASVDVFSISS; |
| DQDKKASVDVFSISSK; | QDKKASVDVFSISSKS; | DKKASVDVFSISSKSE; |
| KKASVDVFSISSKSEL; | KASVDVFSISSKSELD; | ASVDVFSISSKSELDS; |
| SVDVFSISSKSELDSI; | VDVFSISSKSELDSIL; | DVFSISSKSELDSILN; |
| VFSISSKSELDSILNL; | FSISSKSELDSILNLR; | SISSKSELDSILNLRR; |
| ISSKSELDSILNLRRI; | SSKSELDSILNLRRIL; | SKSELDSILNLRRILT; |
| KSELDSILNLRRILTG; | SELDSILNLRRILTGY; | ELDSILNLRRILTGYI; |
| LDSILNLRRILTGYII; | DSILNLRRILTGYIIK; | SILNLRRILTGYIIKS; |
| ILNLRRILTGYIIKSF; | LNLRRILTGYIIKSFD; | NLRRILTGYIIKSFDY; |
| LRRILTGYIIKSFDYD; | RRILTGYIIKSFDYDR; | RILTGYIIKSFDYDRS; |
| ILTGYIIKSFDYDRSS; | LTGYIIKSFDYDRSSA; | TGYIIKSFDYDRSSAE; |
| GYIIKSFDYDRSSAEL; | YIIKSFDYDRSSAELI; | IIKSFDYDRSSAELIA; |
| IKSFDYDRSSAELIAK; | KSFDYDRSSAELIAKV; | SFDYDRSSAELIAKVI; |
| FDYDRSSAELIAKVIT; | DYDRSSAELIAKVITI; | YDRSSAELIAKVITIY; |
| DRSSAELIAKVITIYN; | RSSAELIAKVITIYNA; | SSAELIAKVITIYNAV; |
| SAELIAKVITIYNAVY; | AELIAKVITIYNAVYR; | ELIAKVITIYNAVYRG; |
| LIAKVITIYNAVYRGD; | IAKVITIYNAVYRGDL; | AKVITIYNAVYRGDLD; |
| KVITIYNAVYRGDLDY; | VITIYNAVYRGDLDYY; | ITIYNAVYRGDLDYYK; |
| TIYNAVYRGDLDYYKG; | IYNAVYRGDLDYYKGF; | YNAVYRGDLDYYKGFY; |
| NAVYRGDLDYYKGFYI; | AVYRGDLDYYKGFYIE; | VYRGDLDYYKGFYIEP; |
| YRGDLDYYKGFYIEPA; | RGDLDYYKGFYIEPAL; | GDLDYYKGFYIEPALK; |
| DLDYYKGFYIEPALKS; | LDYYKGFYIEPALKSL; | DYYKGFYIEPALKSLT; |
| YYKGFYIEPALKSLTK; | YKGFYIEPALKSLTKE; | KGFYIEPALKSLTKEN; |
| GFYIEPALKSLTKENA; | FYIEPALKSLTKENAG; | YIEPALKSLTKENAGL; |
| IEPALKSLTKENAGLS; | EPALKSLTKENAGLSR; | PALKSLTKENAGLSRV; |
| ALKSLTKENAGLSRVY; | LKSLTKENAGLSRVYS; | KSLTKENAGLSRVYSQ; |
| SLTKENAGLSRVYSQW; | LTKENAGLSRVYSQWA; | TKENAGLSRVYSQWAG; |
| KENAGLSRVYSQWAGK; | ENAGLSRVYSQWAGKT; | NAGLSRVYSQWAGKTQ; |
| AGLSRVYSQWAGKTQI; | GLSRVYSQWAGKTQIF; | LSRVYSQWAGKTQIFI; |
| SRVYSQWAGKTQIFIP; | RVYSQWAGKTQIFIPL; | VYSQWAGKTQIFIPLK; |
| YSQWAGKTQIFIPLKK; | SQWAGKTQIFIPLKKD; | QWAGKTQIFIPLKKDI; |
| WAGKTQIFIPLKKDIL; | AGKTQIFIPLKKDILS; | GKTQIFIPLKKDILSG; |
| KTQIFIPLKKDILSGN; | TQIFIPLKKDILSGNI; | QIFIPLKKDILSGNIE; |
| IFIPLKKDILSGNIES; | FIPLKKDILSGNIESD; | IPLKKDILSGNIESDI; |
| PLKKDILSGNIESDID; | LKKDILSGNIESDIDI; | KKDILSGNIESDIDID; |
| KDILSGNIESDIDIDS; | DILSGNIESDIDIDSL; | ILSGNIESDIDIDSLV; |
| LSGNIESDIDIDSLVT; | SGNIESDIDIDSLVTD; | GNIESDIDIDSLVTDK; |
| NIESDIDIDSLVTDKV; | IESDIDIDSLVTDKVI; | ESDIDIDSLVTDKVIA; |
| SDIDIDSLVTDKVIAA; | DIDIDSLVTDKVIAAL; | IDIDSLVTDKVIAALL; |

| | |
|---|---|
| | DIDSLVTDKVIAALLS; IDSLVTDKVIAALLSE; DSLVTDKVIAALLSEN;<br>SLVTDKVIAALLSENE; LVTDKVIAALLSENEA; VTDKVIAALLSENEAG;<br>TDKVIAALLSENEAGV; DKVIAALLSENEAGVN; KVIAALLSENEAGVNF;<br>VIAALLSENEAGVNFA; IAALLSENEAGVNFAR; AALLSENEAGVNFARD;<br>ALLSENEAGVNFARDI; LLSENEAGVNFARDIT; LSENEAGVNFARDITD;<br>SENEAGVNFARDITDI; ENEAGVNFARDITDIQ; NEAGVNFARDITDIQG;<br>EAGVNFARDITDIQGE; AGVNFARDITDIQGET; GVNFARDITDIQGETH;<br>VNFARDITDIQGETHK; NFARDITDIQGETHKA; FARDITDIQGETHKAD;<br>ARDITDIQGETHKADQ; RDITDIQGETHKADQD; DITDIQGETHKADQDK;<br>ITDIQGETHKADQDKI; TDIQGETHKADQDKID; DIQGETHKADQDKIDT;<br>IQGETHKADQDKIDTE; QGETHKADQDKIDTEL; GETHKADQDKIDTELD;<br>ETHKADQDKIDTELDN; THKADQDKIDTELDNI; HKADQDKIDTELDNIH;<br>KADQDKIDTELDNIHE; ADQDKIDTELDNIHES; DQDKIDTELDNIHESD;<br>QDKIDTELDNIHESDS; DKIDTELDNIHESDSN; KIDTELDNIHESDSNI;<br>IDTELDNIHESDSNIT; DTELDNIHESDSNITE; TELDNIHESDSNITET;<br>ELDNIHESDSNITETI; LDNIHESDSNITETIE; DNIHESDSNITETIEN;<br>NIHESDSNITETIENL; IHESDSNITETIENLR; HESDSNITETIENLRD;<br>ESDSNITETIENLRDQ; SDSNITETIENLRDQL; DSNITETIENLRDQLE;<br>SNITETIENLRDQLEK; NITETIENLRDQLEKA; ITETIENLRDQLEKAT;<br>TETIENLRDQLEKATD; ETIENLRDQLEKATDE; TIENLRDQLEKATDEE;<br>IENLRDQLEKATDEEH; ENLRDQLEKATDEEHK; NLRDQLEKATDEEHKK;<br>LRDQLEKATDEEHKKE; RDQLEKATDEEHKKEI; DQLEKATDEEHKKEIE;<br>QLEKATDEEHKKEIES; LEKATDEEHKKEIESQ; EKATDEEHKKEIESQV;<br>KATDEEHKKEIESQVD; ATDEEHKKEIESQVDA; TDEEHKKEIESQVDAK;<br>DEEHKKEIESQVDAKK; EEHKKEIESQVDAKKK; EHKKEIESQVDAKKKE;<br>HKKEIESQVDAKKKEK; KKEIESQVDAKKKEKE; KEIESQVDAKKKEKEE;<br>EIESQVDAKKKEKEEL; IESQVDAKKKEKEELD; ESQVDAKKKEKEELDK;<br>SQVDAKKKEKEELDKK; QVDAKKKEKEELDKKA; VDAKKKEKEELDKKAI;<br>DAKKKEKEELDKKAIN; AKKKEKEELDKKAINL; KKKEKEELDKKAINLD;<br>KKEKEELDKKAINLDK; KEKEELDKKAINLDKA; EKEELDKKAINLDKAQ;<br>KEELDKKAINLDKAQQ; EELDKKAINLDKAQQK; ELDKKAINLDKAQQKL;<br>LDKKAINLDKAQQKLD; DKKAINLDKAQQKLDS; KKAINLDKAQQKLDSA;<br>KAINLDKAQQKLDSAE; AINLDKAQQKLDSAED; INLDKAQQKLDSAEDN;<br>NLDKAQQKLDSAEDNL; LDKAQQKLDSAEDNLD; DKAQQKLDSAEDNLDV;<br>KAQQKLDSAEDNLDVQ; AQQKLDSAEDNLDVQR; QQKLDSAEDNLDVQRD;<br>QKLDSAEDNLDVQRDT; KLDSAEDNLDVQRDTV; LDSAEDNLDVQRDTVR;<br>DSAEDNLDVQRDTVRE; SAEDNLDVQRDTVREK; AEDNLDVQRDTVREKI;<br>EDNLDVQRDTVREKIQ; DNLDVQRDTVREKIQE; NLDVQRDTVREKIQED;<br>LDVQRDTVREKIQEDI; DVQRDTVREKIQEDIN; VQRDTVREKIQEDINE;<br>QRDTVREKIQEDINEI; RDTVREKIQEDINEIN; DTVREKIQEDINEINK;<br>TVREKIQEDINEINKE; VREKIQEDINEINKEK; REKIQEDINEINKEKN;<br>EKIQEDINEINKEKNL; KIQEDINEINKEKNLP; IQEDINEINKEKNLPK;<br>QEDINEINKEKNLPKP; EDINEINKEKNLPKPG; DINEINKEKNLPKPGD;<br>INEINKEKNLPKPGDV; NEINKEKNLPKPGDVS; EINKEKNLPKPGDVSS;<br>INKEKNLPKPGDVSSP; NKEKNLPKPGDVSSPK; KEKNLPKPGDVSSPKV;<br>EKNLPKPGDVSSPKVD; KNLPKPGDVSSPKVDK; NLPKPGDVSSPKVDKQ;<br>LPKPGDVSSPKVDKQL; PKPGDVSSPKVDKQLQ; KPGDVSSPKVDKQLQI;<br>PGDVSSPKVDKQLQIK; GDVSSPKVDKQLQIKE; DVSSPKVDKQLQIKES;<br>VSSPKVDKQLQIKESL; SSPKVDKQLQIKESLE; SPKVDKQLQIKESLED;<br>PKVDKQLQIKESLEDL; KVDKQLQIKESLEDLQ; VDKQLQIKESLEDLQE;<br>DKQLQIKESLEDLQEQ; KQLQIKESLEDLQEQL; QLQIKESLEDLQEQLK; |

| | | |
|---|---|---|
| LQIKESLEDLQEQLKE; | QIKESLEDLQEQLKEA; | IKESLEDLQEQLKEAG; |
| KESLEDLQEQLKEAGD; | ESLEDLQEQLKEAGDE; | SLEDLQEQLKEAGDEN; |
| LEDLQEQLKEAGDENQ; | EDLQEQLKEAGDENQK; | DLQEQLKEAGDENQKR; |
| LQEQLKEAGDENQKRE; | QEQLKEAGDENQKREI; | EQLKEAGDENQKREIE; |
| QLKEAGDENQKREIEK; | LKEAGDENQKREIEKQ; | KEAGDENQKREIEKQI; |
| EAGDENQKREIEKQIE; | AGDENQKREIEKQIEI; | GDENQKREIEKQIEIK; |
| DENQKREIEKQIEIKK; | ENQKREIEKQIEIKKR; | NQKREIEKQIEIKKRD; |
| QKREIEKQIEIKKRDE; | KREIEKQIEIKKRDEE; | REIEKQIEIKKRDEEL; |
| EIEKQIEIKKRDEELL; | IEKQIEIKKRDEELLK; | EKQIEIKKRDEELLKS; |
| KQIEIKKRDEELLKSK; | QIEIKKRDEELLKSKD; | IEIKKRDEELLKSKDG; |
| EIKKRDEELLKSKDGK; | IKKRDEELLKSKDGKV; | KKRDEELLKSKDGKVS; |
| KRDEELLKSKDGKVSK; | RDEELLKSKDGKVSKD; | DEELLKSKDGKVSKDY; |
| EELLKSKDGKVSKDYE; | ELLKSKDGKVSKDYEA; | LLKSKDGKVSKDYEAL; |
| LKSKDGKVSKDYEALD; | KSKDGKVSKDYEALDL; | SKDGKVSKDYEALDLD; |
| KDGKVSKDYEALDLDR; | DGKVSKDYEALDLDRE; | GKVSKDYEALDLDREL; |
| KVSKDYEALDLDRELS; | VSKDYEALDLDRELSK; | SKDYEALDLDRELSKA; |
| KDYEALDLDRELSKAS; | DYEALDLDRELSKASS; | YEALDLDRELSKASSK; |
| EALDLDRELSKASSKE; | ALDLDRELSKASSKEK; | LDLDRELSKASSKEKS; |
| DLDRELSKASSKEKSK; | LDRELSKASSKEKSKV; | DRELSKASSKEKSKVK; |
| RELSKASSKEKSKVKE; | ELSKASSKEKSKVKEE; | LSKASSKEKSKVKEEE; |
| SKASSKEKSKVKEEEI; | KASSKEKSKVKEEEIT; | ASSKEKSKVKEEEITK; |
| SSKEKSKVKEEEITKG; | SKEKSKVKEEEITKGK; | KEKSKVKEEEITKGKS; |
| EKSKVKEEEITKGKSR; | KSKVKEEEITKGKSRA; | SKVKEEEITKGKSRAS; |
| KVKEEEITKGKSRASL; | VKEEEITKGKSRASLG; | KEEEITKGKSRASLGD; |
| EEEITKGKSRASLGDL; | EEITKGKSRASLGDLN; | EITKGKSRASLGDLNN; |
| ITKGKSRASLGDLNND; | TKGKSRASLGDLNNDK; | KGKSRASLGDLNNDKN; |
| GKSRASLGDLNNDKNL; | KSRASLGDLNNDKNLM; | SRASLGDLNNDKNLML; |
| RASLGDLNNDKNLMLP; | ASLGDLNNDKNLMLPE; | SLGDLNNDKNLMLPED; |
| LGDLNNDKNLMLPEDQ; | GDLNNDKNLMLPEDQK; | DLNNDKNLMLPEDQKL; |
| LNNDKNLMLPEDQKLP; | NNDKNLMLPEDQKLPE; | NDKNLMLPEDQKLPED; |
| DKNLMLPEDQKLPEDK; | KNLMLPEDQKLPEDKK; | NLMLPEDQKLPEDKKL; |
| LMLPEDQKLPEDKKLD; | MLPEDQKLPEDKKLDS; | LPEDQKLPEDKKLDSK; |
| PEDQKLPEDKKLDSKL; | EDQKLPEDKKLDSKLD; | DQKLPEDKKLDSKLDG; |
| QKLPEDKKLDSKLDGK; | KLPEDKKLDSKLDGKK; | LPEDKKLDSKLDGKKE; |
| PEDKKLDSKLDGKKEF; | EDKKLDSKLDGKKEFK; | DKKLDSKLDGKKEFKP; |
| KKLDSKLDGKKEFKPV; | KLDSKLDGKKEFKPVS; | LDSKLDGKKEFKPVSE; |
| DSKLDGKKEFKPVSEV; | SKLDGKKEFKPVSEVE; | KLDGKKEFKPVSEVEK; |
| LDGKKEFKPVSEVEKL; | DGKKEFKPVSEVEKLD; | GKKEFKPVSEVEKLDK; |
| KKEFKPVSEVEKLDKI; | KEFKPVSEVEKLDKIS; | EFKPVSEVEKLDKISK; |
| FKPVSEVEKLDKISKS; | KPVSEVEKLDKISKSN; | PVSEVEKLDKISKSNN; |
| VSEVEKLDKISKSNNN; | SEVEKLDKISKSNNNE; | EVEKLDKISKSNNNEV; |
| VEKLDKISKSNNNEVG; | EKLDKISKSNNNEVGK; | KLDKISKSNNNEVGKL; |
| LDKISKSNNNEVGKLS; | DKISKSNNNEVGKLSP; | KISKSNNNEVGKLSPL; |
| ISKSNNNEVGKLSPLD; | SKSNNNEVGKLSPLDK; | KSNNNEVGKLSPLDKP; |
| SNNNEVGKLSPLDKPS; | NNNEVGKLSPLDKPSY; | NNEVGKLSPLDKPSYD; |
| NEVGKLSPLDKPSYDD; | EVGKLSPLDKPSYDDI; | VGKLSPLDKPSYDDID; |
| GKLSPLDKPSYDDIDS; | KLSPLDKPSYDDIDSK; | LSPLDKPSYDDIDSKE; |
| SPLDKPSYDDIDSKEE; | PLDKPSYDDIDSKEEV; | LDKPSYDDIDSKEEVD; |
| DKPSYDDIDSKEEVDN; | KPSYDDIDSKEEVDNK; | PSYDDIDSKEEVDNKA; |
| SYDDIDSKEEVDNKAI; | YDDIDSKEEVDNKAIN; | DDIDSKEEVDNKAINL; |
| DIDSKEEVDNKAINLQ; | IDSKEEVDNKAINLQK; | DSKEEVDNKAINLQKI; |

SKEEVDNKAINLQKID; KEEVDNKAINLQKIDP; EEVDNKAINLQKIDPK;
EVDNKAINLQKIDPKV; VDNKAINLQKIDPKVK; DNKAINLQKIDPKVKD;
NKAINLQKIDPKVKDQ; KAINLQKIDPKVKDQT; AINLQKIDPKVKDQTT;
INLQKIDPKVKDQTTS; NLQKIDPKVKDQTTSL; LQKIDPKVKDQTTSLN;
QKIDPKVKDQTTSLNE; KIDPKVKDQTTSLNED; IDPKVKDQTTSLNEDL;
DPKVKDQTTSLNEDLD; PKVKDQTTSLNEDLDK; KVKDQTTSLNEDLDKD;
VKDQTTSLNEDLDKDL; KDQTTSLNEDLDKDLT; DQTTSLNEDLDKDLTT;
QTTSLNEDLDKDLTTM; TTSLNEDLDKDLTTMS; TSLNEDLDKDLTTMSI;
SLNEDLDKDLTTMSID; LNEDLDKDLTTMSIDS; NEDLDKDLTTMSIDSS;
EDLDKDLTTMSIDSSS; DLDKDLTTMSIDSSSP; LDKDLTTMSIDSSSPV;
DKDLTTMSIDSSSPVF; KDLTTMSIDSSSPVFL; DLTTMSIDSSSPVFLE;
LTTMSIDSSSPVFLEV; TTMSIDSSSPVFLEVI; TMSIDSSSPVFLEVID;
MSIDSSSPVFLEVIDP; SIDSSSPVFLEVIDPI; IDSSSPVFLEVIDPIT;
DSSSPVFLEVIDPITN; SSSPVFLEVIDPITNL; SSPVFLEVIDPITNLG;
SPVFLEVIDPITNLGT; PVFLEVIDPITNLGTL; VFLEVIDPITNLGTLQ;
FLEVIDPITNLGTLQL; LEVIDPITNLGTLQLI; EVIDPITNLGTLQLID;
VIDPITNLGTLQLIDL; IDPITNLGTLQLIDLN; DPITNLGTLQLIDLNT;
PITNLGTLQLIDLNTG; ITNLGTLQLIDLNTGV; TNLGTLQLIDLNTGVR;
NLGTLQLIDLNTGVRL; LGTLQLIDLNTGVRLK; GTLQLIDLNTGVRLKE;
TLQLIDLNTGVRLKES; LQLIDLNTGVRLKEST; QLIDLNTGVRLKESTQ;
LIDLNTGVRLKESTQQ; IDLNTGVRLKESTQQG; DLNTGVRLKESTQQGI;
LNTGVRLKESTQQGIQ; NTGVRLKESTQQGIQR; TGVRLKESTQQGIQRY;
GVRLKESTQQGIQRYG; VRLKESTQQGIQRYGI; RLKESTQQGIQRYGIY;
LKESTQQGIQRYGIYE; KESTQQGIQRYGIYER; ESTQQGIQRYGIYERE;
STQQGIQRYGIYEREK; TQQGIQRYGIYEREKD; QQGIQRYGIYEREKDL;
QGIQRYGIYEREKDLV; GIQRYGIYEREKDLVV; IQRYGIYEREKDLVVI;
QRYGIYEREKDLVVIK; RYGIYEREKDLVVIKM; YGIYEREKDLVVIKMD;
GIYEREKDLVVIKMDS; IYEREKDLVVIKMDSG; YEREKDLVVIKMDSGK;
EREKDLVVIKMDSGKA; REKDLVVIKMDSGKAK; EKDLVVIKMDSGKAKL;
KDLVVIKMDSGKAKLQ; DLVVIKMDSGKAKLQI; LVVIKMDSGKAKLQIL;
VVIKMDSGKAKLQILN; VIKMDSGKAKLQILNK; IKMDSGKAKLQILNKL;
KMDSGKAKLQILNKLE; MDSGKAKLQILNKLEN; DSGKAKLQILNKLENL;
SGKAKLQILNKLENLK; GKAKLQILNKLENLKV; KAKLQILNKLENLKVV;
AKLQILNKLENLKVVS; KLQILNKLENLKVVSE; LQILNKLENLKVVSES;
QILNKLENLKVVSESN; ILNKLENLKVVSESNF; LNKLENLKVVSESNFE;
NKLENLKVVSESNFEI; KLENLKVVSESNFEIN; LENLKVVSESNFEINK;
ENLKVVSESNFEINKN; NLKVVSESNFEINKNS; LKVVSESNFEINKNSS;
KVVSESNFEINKNSSL; VVSESNFEINKNSSLY; VSESNFEINKNSSLYV;
SESNFEINKNSSLYVD; ESNFEINKNSSLYVDS; SNFEINKNSSLYVDSK;
NFEINKNSSLYVDSKM; FEINKNSSLYVDSKMI; EINKNSSLYVDSKMIL;
INKNSSLYVDSKMILA; NKNSSLYVDSKMILAA; KNSSLYVDSKMILAAV;
NSSLYVDSKMILAAVR; SSLYVDSKMILAAVRD; SLYVDSKMILAAVRDK;
LYVDSKMILAAVRDKD; YVDSKMILAAVRDKDD; VDSKMILAAVRDKDDS;
DSKMILAAVRDKDDSN; SKMILAAVRDKDDSNA; KMILAAVRDKDDSNAW;
MILAAVRDKDDSNAWR; ILAAVRDKDDSNAWRL; LAAVRDKDDSNAWRLA;
AAVRDKDDSNAWRLAK; AVRDKDDSNAWRLAKF; VRDKDDSNAWRLAKFS;
RDKDDSNAWRLAKFSP; DKDDSNAWRLAKFSPK; KDDSNAWRLAKFSPKN;
DDSNAWRLAKFSPKNL; DSNAWRLAKFSPKNLD; SNAWRLAKFSPKNLDE;
NAWRLAKFSPKNLDEF; AWRLAKFSPKNLDEFI; WRLAKFSPKNLDEFIL;
RLAKFSPKNLDEFILS; LAKFSPKNLDEFILSE; AKFSPKNLDEFILSEN;
KFSPKNLDEFILSENK; FSPKNLDEFILSENKI; SPKNLDEFILSENKIL;

| | |
|---|---|
| | PKNLDEFILSENKILP; KNLDEFILSENKILPF; NLDEFILSENKILPFT; LDEFILSENKILPFTS; DEFILSENKILPFTSF; EFILSENKILPFTSFS; FILSENKILPFTSFSV; ILSENKILPFTSFSVR; LSENKILPFTSFSVRK; SENKILPFTSFSVRKN; ENKILPFTSFSVRKNF; NKILPFTSFSVRKNFI; KILPFTSFSVRKNFIY; ILPFTSFSVRKNFIYL; LPFTSFSVRKNFIYLQ; PFTSFSVRKNFIYLQD; FTSFSVRKNFIYLQDE; TSFSVRKNFIYLQDEL; SFSVRKNFIYLQDELK; FSVRKNFIYLQDELKN; SVRKNFIYLQDELKNL; VRKNFIYLQDELKNLV; RKNFIYLQDELKNLVI; KNFIYLQDELKNLVIL; NFIYLQDELKNLVILD; FIYLQDELKNLVILDV; IYLQDELKNLVILDVN; YLQDELKNLVILDVNT; LQDELKNLVILDVNTL; QDELKNLVILDVNTLK; DELKNLVILDVNTLKK; ELKNLVILDVNTLKKV; LKNLVILDVNTLKKVK; |
| Seq 27 | 13 mers:<br>MKYILSIDQGTTS; KYILSIDQGTTSS; YILSIDQGTTSSR; ILSIDQGTTSSRA; LSIDQGTTSSRAM; SIDQGTTSSRAMV; IDQGTTSSRAMVF; DQGTTSSRAMVFD; QGTTSSRAMVFDK; GTTSSRAMVFDKN; TTSSRAMVFDKNA; TSSRAMVFDKNAN; SSRAMVFDKNANI; SRAMVFDKNANIK; RAMVFDKNANIKG; AMVFDKNANIKGF; MVFDKNANIKGFA; VFDKNANIKGFAQ; FDKNANIKGFAQK; DKNANIKGFAQKE; KNANIKGFAQKEF; NANIKGFAQKEFT; ANIKGFAQKEFTQ; NIKGFAQKEFTQI; IKGFAQKEFTQIY; KGFAQKEFTQIYP; GFAQKEFTQIYPQ; FAQKEFTQIYPQP; AQKEFTQIYPQPS; QKEFTQIYPQPSW; KEFTQIYPQPSWV; EFTQIYPQPSWVE; FTQIYPQPSWVEH; TQIYPQPSWVEHD; QIYPQPSWVEHDP; IYPQPSWVEHDPT; YPQPSWVEHDPTE; PQPSWVEHDPTEI; QPSWVEHDPTEIW; PSWVEHDPTEIWG; SWVEHDPTEIWGS; WVEHDPTEIWGSQ; VEHDPTEIWGSQL; EHDPTEIWGSQLG; HDPTEIWGSQLGV; DPTEIWGSQLGVI; PTEIWGSQLGVIT; TEIWGSQLGVITE; EIWGSQLGVITEA; IWGSQLGVITEAM; WGSQLGVITEAMA; GSQLGVITEAMAN; SQLGVITEAMANA; QLGVITEAMANAR; LGVITEAMANARI; GVITEAMANARIL; VITEAMANARILP; ITEAMANARILPN; TEAMANARILPNE; EAMANARILPNEI; AMANARILPNEID; MANARILPNEIDA; ANARILPNEIDAI; NARILPNEIDAIG; ARILPNEIDAIGI; RILPNEIDAIGIT; ILPNEIDAIGITN; LPNEIDAIGITNQ; PNEIDAIGITNQR; NEIDAIGITNQRE; EIDAIGITNQRET; IDAIGITNQRETT; DAIGITNQRETTV; AIGITNQRETTVI; IGITNQRETTVIW; GITNQRETTVIWE; ITNQRETTVIWEK; TNQRETTVIWEKN; NQRETTVIWEKNT; QRETTVIWEKNTG; RETTVIWEKNTGK; ETTVIWEKNTGKP; TTVIWEKNTGKPI; TVIWEKNTGKPIY; VIWEKNTGKPIYN; IWEKNTGKPIYNA; WEKNTGKPIYNAI; EKNTGKPIYNAIV; KNTGKPIYNAIVW; NTGKPIYNAIVWQ; TGKPIYNAIVWQD; GKPIYNAIVWQDR; KPIYNAIVWQDRR; PIYNAIVWQDRRT; IYNAIVWQDRRTA; YNAIVWQDRRTAK; NAIVWQDRRTAKI; AIVWQDRRTAKIC; IVWQDRRTAKICD; VWQDRRTAKICDQ; WQDRRTAKICDQL; QDRRTAKICDQLK; DRRTAKICDQLKK; RRTAKICDQLKKE; RTAKICDQLKKEG; TAKICDQLKKEGK; AKICDQLKKEGKD; KICDQLKKEGKDK; ICDQLKKEGKDKI; CDQLKKEGKDKII; DQLKKEGKDKIIL; |

QLKKEGKDKIILE; LKKEGKDKIILEK; KKEGKDKIILEKT;
KEGKDKIILEKTG; EGKDKIILEKTGL; GKDKIILEKTGLV;
KDKIILEKTGLVL; DKIILEKTGLVLD; KIILEKTGLVLDS;
IILEKTGLVLDSY; ILEKTGLVLDSYF; LEKTGLVLDSYFS;
EKTGLVLDSYFSG; KTGLVLDSYFSGT; TGLVLDSYFSGTK;
GLVLDSYFSGTKI; LVLDSYFSGTKIM; VLDSYFSGTKIMW;
LDSYFSGTKIMWI; DSYFSGTKIMWIL; SYFSGTKIMWILD;
YFSGTKIMWILDN; FSGTKIMWILDNV; SGTKIMWILDNVE;
GTKIMWILDNVEG; TKIMWILDNVEGA; KIMWILDNVEGAR;
IMWILDNVEGARQ; MWILDNVEGARQR; WILDNVEGARQRA;
ILDNVEGARQRAE; LDNVEGARQRAEN; DNVEGARQRAENG;
NVEGARQRAENGE; VEGARQRAENGEL; EGARQRAENGELC;
GARQRAENGELCF; ARQRAENGELCFG; RQRAENGELCFGT;
QRAENGELCFGTI; RAENGELCFGTID; AENGELCFGTIDT;
ENGELCFGTIDTW; NGELCFGTIDTWI; GELCFGTIDTWIL;
ELCFGTIDTWILW; LCFGTIDTWILWN; CFGTIDTWILWNL;
FGTIDTWILWNLT; GTIDTWILWNLTQ; TIDTWILWNLTQK;
IDTWILWNLTQKK; DTWILWNLTQKKE; TWILWNLTQKKEH;
WILWNLTQKKEHA; ILWNLTQKKEHAT; LWNLTQKKEHATD;
WNLTQKKEHATDY; NLTQKKEHATDYS; LTQKKEHATDYSN;
TQKKEHATDYSNA; QKKEHATDYSNAS; KKEHATDYSNASR;
KEHATDYSNASRT; EHATDYSNASRTL; HATDYSNASRTLL;
ATDYSNASRTLLL; TDYSNASRTLLLN; DYSNASRTLLLNI;
YSNASRTLLLNIK; SNASRTLLLNIKT; NASRTLLLNIKTL;
ASRTLLLNIKTLE; SRTLLLNIKTLEW; RTLLLNIKTLEWD;
TLLLNIKTLEWDD; LLLNIKTLEWDDE; LLNIKTLEWDDEL;
LNIKTLEWDDELL; NIKTLEWDDELLS; IKTLEWDDELLSI;
KTLEWDDELLSIL; TLEWDDELLSILN; LEWDDELLSILNV;
EWDDELLSILNVP; WDDELLSILNVPR; DDELLSILNVPRA;
DELLSILNVPRAI; ELLSILNVPRAIL; LLSILNVPRAILP;
LSILNVPRAILPE; SILNVPRAILPEL; ILNVPRAILPELK;
LNVPRAILPELKE; NVPRAILPELKES; VPRAILPELKESS;
PRAILPELKESST; RAILPELKESSTI; AILPELKESSTIY;
ILPELKESSTIYG; LPELKESSTIYGK; PELKESSTIYGKT;
ELKESSTIYGKTD; LKESSTIYGKTDK; KESSTIYGKTDKA;
ESSTIYGKTDKAL; SSTIYGKTDKALF; STIYGKTDKALFG;
TIYGKTDKALFGA; IYGKTDKALFGAE; YGKTDKALFGAEI;
GKTDKALFGAEIP; KTDKALFGAEIPI; TDKALFGAEIPIA;
DKALFGAEIPIAG; KALFGAEIPIAGI; ALFGAEIPIAGIA;
LFGAEIPIAGIAG; FGAEIPIAGIAGD; GAEIPIAGIAGDQ;
AEIPIAGIAGDQF; EIPIAGIAGDQFA; IPIAGIAGDQFAA;
PIAGIAGDQFAAT; IAGIAGDQFAATF; AGIAGDQFAATFG;
GIAGDQFAATFGQ; IAGDQFAATFGQA; AGDQFAATFGQAC;
GDQFAATFGQACL; DQFAATFGQACLK; QFAATFGQACLKK;
FAATFGQACLKKG; AATFGQACLKKGM; ATFGQACLKKGMA;
TFGQACLKKGMAK; FGQACLKKGMAKN; GQACLKKGMAKNT;
QACLKKGMAKNTY; ACLKKGMAKNTYG; CLKKGMAKNTYGT;
LKKGMAKNTYGTG; KKGMAKNTYGTGC; KGMAKNTYGTGCF;
GMAKNTYGTGCFL; MAKNTYGTGCFLT; AKNTYGTGCFLTV;
KNTYGTGCFLTVN; NTYGTGCFLTVNI; TYGTGCFLTVNIG;
YGTGCFLTVNIGK; GTGCFLTVNIGKE; TGCFLTVNIGKEP;

GCFLTVNIGKEPI; CFLTVNIGKEPII; FLTVNIGKEPIIS;
LTVNIGKEPIISH; TVNIGKEPIISHD; VNIGKEPIISHDK;
NIGKEPIISHDKL; IGKEPIISHDKLL; GKEPIISHDKLLT;
KEPIISHDKLLTS; EPIISHDKLLTSI; PIISHDKLLTSIA;
IISHDKLLTSIAW; ISHDKLLTSIAWG; SHDKLLTSIAWGR;
HDKLLTSIAWGRK; DKLLTSIAWGRKK; KLLTSIAWGRKKS;
LLTSIAWGRKKSV; LTSIAWGRKKSVT; TSIAWGRKKSVTY;
SIAWGRKKSVTYV; IAWGRKKSVTYVL; AWGRKKSVTYVLE;
WGRKKSVTYVLEG; GRKKSVTYVLEGS; RKKSVTYVLEGSV;
KKSVTYVLEGSVF; KSVTYVLEGSVFI; SVTYVLEGSVFIG;
VTYVLEGSVFIGG; TYVLEGSVFIGGA; YVLEGSVFIGGAV;
VLEGSVFIGGAVI; LEGSVFIGGAVIQ; EGSVFIGGAVIQW;
GSVFIGGAVIQWL; SVFIGGAVIQWLR; VFIGGAVIQWLRD;
FIGGAVIQWLRDG; IGGAVIQWLRDGL; GGAVIQWLRDGLE;
GAVIQWLRDGLEF; AVIQWLRDGLEFF; VIQWLRDGLEFFR;
IQWLRDGLEFFRK; QWLRDGLEFFRKS; WLRDGLEFFRKSS;
LRDGLEFFRKSSD; RDGLEFFRKSSDA; DGLEFFRKSSDAE;
GLEFFRKSSDAEA; LEFFRKSSDAEAL; EFFRKSSDAEALA;
FFRKSSDAEALAS; FRKSSDAEALASS; RKSSDAEALASSV;
KSSDAEALASSVS; SSDAEALASSVSD; SDAEALASSVSDN;
DAEALASSVSDNG; AEALASSVSDNGG; EALASSVSDNGGV;
ALASSVSDNGGVY; LASSVSDNGGVYF; ASSVSDNGGVYFV;
SSVSDNGGVYFVP; SVSDNGGVYFVPA; VSDNGGVYFVPAF;
SDNGGVYFVPAFV; DNGGVYFVPAFVG; NGGVYFVPAFVGL;
GGVYFVPAFVGLG; GVYFVPAFVGLGA; VYFVPAFVGLGAP;
YFVPAFVGLGAPH; FVPAFVGLGAPHW; VPAFVGLGAPHWD;
PAFVGLGAPHWDS; AFVGLGAPHWDSY; FVGLGAPHWDSYA;
VGLGAPHWDSYAR; GLGAPHWDSYARG; LGAPHWDSYARGT;
GAPHWDSYARGTI; APHWDSYARGTII; PHWDSYARGTIIG;
HWDSYARGTIIGI; WDSYARGTIIGIT; DSYARGTIIGITR;
SYARGTIIGITRG; YARGTIIGITRGS; ARGTIIGITRGST;
RGTIIGITRGSTK; GTIIGITRGSTKA; TIIGITRGSTKAH;
IIGITRGSTKAHI; IGITRGSTKAHIT; GITRGSTKAHITR;
ITRGSTKAHITRA; TRGSTKAHITRAA; RGSTKAHITRAAL;
GSTKAHITRAALE; STKAHITRAALES; TKAHITRAALESI;
KAHITRAALESIA; AHITRAALESIAF; HITRAALESIAFQ;
ITRAALESIAFQS; TRAALESIAFQSF; RAALESIAFQSFD;
AALESIAFQSFDI; ALESIAFQSFDIL; LESIAFQSFDILN;
ESIAFQSFDILNT; SIAFQSFDILNTM; IAFQSFDILNTMK;
AFQSFDILNTMKK; FQSFDILNTMKKS; QSFDILNTMKKSI;
SFDILNTMKKSIP; FDILNTMKKSIPN; DILNTMKKSIPNF;
ILNTMKKSIPNFE; LNTMKKSIPNFEI; NTMKKSIPNFEIQ;
TMKKSIPNFEIQE; MKKSIPNFEIQEL; KKSIPNFEIQELR;
KSIPNFEIQELRV; SIPNFEIQELRVD; IPNFEIQELRVDG;
PNFEIQELRVDGG; NFEIQELRVDGGA; FEIQELRVDGGAS;
EIQELRVDGGASQ; IQELRVDGGASQN; QELRVDGGASQNN;
ELRVDGGASQNNL; LRVDGGASQNNLL; RVDGGASQNNLLM;
VDGGASQNNLLMQ; DGGASQNNLLMQF; GGASQNNLLMQFQ;
GASQNNLLMQFQA; ASQNNLLMQFQAD; SQNNLLMQFQADL;
QNNLLMQFQADLL; NNLLMQFQADLLE; NLLMQFQADLLEC;
LLMQFQADLLECK; LMQFQADLLECKV; MQFQADLLECKVV;

QFQADLLECKVVR;   FQADLLECKVVRP;   QADLLECKVVRPK;
ADLLECKVVRPKI;   DLLECKVVRPKIT;   LLECKVVRPKITE;
LECKVVRPKITET;   ECKVVRPKITETT;   CKVVRPKITETTA;
KVVRPKITETTAL;   VVRPKITETTALG;   VRPKITETTALGA;
RPKITETTALGAA;   PKITETTALGAAY;   KITETTALGAAYL;
ITETTALGAAYLA;   TETTALGAAYLAG;   ETTALGAAYLAGL;
TTALGAAYLAGLA;   TALGAAYLAGLAT;   ALGAAYLAGLATG;
LGAAYLAGLATGY;   GAAYLAGLATGYW;   AAYLAGLATGYWQ;
AYLAGLATGYWQS;   YLAGLATGYWQSA;   LAGLATGYWQSAE;
AGLATGYWQSAEE;   GLATGYWQSAEEI;   LATGYWQSAEEIV;
ATGYWQSAEEIVS;   TGYWQSAEEIVSL;   GYWQSAEEIVSLW;
YWQSAEEIVSLWQ;   WQSAEEIVSLWQV;   QSAEEIVSLWQVD;
SAEEIVSLWQVDK;   AEEIVSLWQVDKI;   EEIVSLWQVDKIF;
EIVSLWQVDKIFE;   IVSLWQVDKIFEP;   VSLWQVDKIFEPS;
SLWQVDKIFEPSM;   LWQVDKIFEPSMP;   WQVDKIFEPSMPK;
QVDKIFEPSMPKN;   VDKIFEPSMPKNQ;   DKIFEPSMPKNQK;
KIFEPSMPKNQKE;   IFEPSMPKNQKEK;   FEPSMPKNQKEKL;
EPSMPKNQKEKLL;   PSMPKNQKEKLLE;   SMPKNQKEKLLEN;
MPKNQKEKLLENW;   PKNQKEKLLENWN;   KNQKEKLLENWNK;
NQKEKLLENWNKA;   QKEKLLENWNKAV;   KEKLLENWNKAVG;
EKLLENWNKAVGK;   KLLENWNKAVGKA;   LLENWNKAVGKAK;
LENWNKAVGKAKS;   ENWNKAVGKAKSW;   NWNKAVGKAKSWI;
WNKAVGKAKSWIQ;   NKAVGKAKSWIQN;   KAVGKAKSWIQNS;
AVGKAKSWIQNSH;   VGKAKSWIQNSHS;   GKAKSWIQNSHSS;

14 mers:
MKYILSIDQGTTSS;   KYILSIDQGTTSSR;   YILSIDQGTTSSRA;
ILSIDQGTTSSRAM;   LSIDQGTTSSRAMV;   SIDQGTTSSRAMVF;
IDQGTTSSRAMVFD;   DQGTTSSRAMVFDK;   QGTTSSRAMVFDKN;
GTTSSRAMVFDKNA;   TTSSRAMVFDKNAN;   TSSRAMVFDKNANI;
SSRAMVFDKNANIK;   SRAMVFDKNANIKG;   RAMVFDKNANIKGF;
AMVFDKNANIKGFA;   MVFDKNANIKGFAQ;   VFDKNANIKGFAQK;
FDKNANIKGFAQKE;   DKNANIKGFAQKEF;   KNANIKGFAQKEFT;
NANIKGFAQKEFTQ;   ANIKGFAQKEFTQI;   NIKGFAQKEFTQIY;
IKGFAQKEFTQIYP;   KGFAQKEFTQIYPQ;   GFAQKEFTQIYPQP;
FAQKEFTQIYPQPS;   AQKEFTQIYPQPSW;   QKEFTQIYPQPSWV;
KEFTQIYPQPSWVE;   EFTQIYPQPSWVEH;   FTQIYPQPSWVEHD;
TQIYPQPSWVEHDP;   QIYPQPSWVEHDPT;   IYPQPSWVEHDPTE;
YPQPSWVEHDPTEI;   PQPSWVEHDPTEIW;   QPSWVEHDPTEIWG;
PSWVEHDPTEIWGS;   SWVEHDPTEIWGSQ;   WVEHDPTEIWGSQL;
VEHDPTEIWGSQLG;   EHDPTEIWGSQLGV;   HDPTEIWGSQLGVI;
DPTEIWGSQLGVIT;   PTEIWGSQLGVITE;   TEIWGSQLGVITEA;
EIWGSQLGVITEAM;   IWGSQLGVITEAMA;   WGSQLGVITEAMAN;
GSQLGVITEAMANA;   SQLGVITEAMANAR;   QLGVITEAMANARI;
LGVITEAMANARIL;   GVITEAMANARILP;   VITEAMANARILPN;
ITEAMANARILPNE;   TEAMANARILPNEI;   EAMANARILPNEID;
AMANARILPNEIDA;   MANARILPNEIDAI;   ANARILPNEIDAIG;
NARILPNEIDAIGI;   ARILPNEIDAIGIT;   RILPNEIDAIGITN;
ILPNEIDAIGITNQ;   LPNEIDAIGITNQR;   PNEIDAIGITNQRE;
NEIDAIGITNQRET;   EIDAIGITNQRETT;   IDAIGITNQRETTV;
DAIGITNQRETTVI;   AIGITNQRETTVIW;   IGITNQRETTVIWE;

GITNQRETTVIWEK; ITNQRETTVIWEKN; TNQRETTVIWEKNT;
NQRETTVIWEKNTG; QRETTVIWEKNTGK; RETTVIWEKNTGKP;
ETTVIWEKNTGKPI; TTVIWEKNTGKPIY; TVIWEKNTGKPIYN;
VIWEKNTGKPIYNA; IWEKNTGKPIYNAI; WEKNTGKPIYNAIV;
EKNTGKPIYNAIVW; KNTGKPIYNAIVWQ; NTGKPIYNAIVWQD;
TGKPIYNAIVWQDR; GKPIYNAIVWQDRR; KPIYNAIVWQDRRT;
PIYNAIVWQDRRTA; IYNAIVWQDRRTAK; YNAIVWQDRRTAKI;
NAIVWQDRRTAKIC; AIVWQDRRTAKICD; IVWQDRRTAKICDQ;
VWQDRRTAKICDQL; WQDRRTAKICDQLK; QDRRTAKICDQLKK;
DRRTAKICDQLKKE; RRTAKICDQLKKEG; RTAKICDQLKKEGK;
TAKICDQLKKEGKD; AKICDQLKKEGKDK; KICDQLKKEGKDKI;
ICDQLKKEGKDKII; CDQLKKEGKDKIIL; DQLKKEGKDKIILE;
QLKKEGKDKIILEK; LKKEGKDKIILEKT; KKEGKDKIILEKTG;
KEGKDKIILEKTGL; EGKDKIILEKTGLV; GKDKIILEKTGLVL;
KDKIILEKTGLVLD; DKIILEKTGLVLDS; KIILEKTGLVLDSY;
IILEKTGLVLDSYF; ILEKTGLVLDSYFS; LEKTGLVLDSYFSG;
EKTGLVLDSYFSGT; KTGLVLDSYFSGTK; TGLVLDSYFSGTKI;
GLVLDSYFSGTKIM; LVLDSYFSGTKIMW; VLDSYFSGTKIMWI;
LDSYFSGTKIMWIL; DSYFSGTKIMWILD; SYFSGTKIMWILDN;
YFSGTKIMWILDNV; FSGTKIMWILDNVE; SGTKIMWILDNVEG;
GTKIMWILDNVEGA; TKIMWILDNVEGAR; KIMWILDNVEGARQ;
IMWILDNVEGARQR; MWILDNVEGARQRA; WILDNVEGARQRAE;
ILDNVEGARQRAEN; LDNVEGARQRAENG; DNVEGARQRAENGE;
NVEGARQRAENGEL; VEGARQRAENGELC; EGARQRAENGELCF;
GARQRAENGELCFG; ARQRAENGELCFGT; RQRAENGELCFGTI;
QRAENGELCFGTID; RAENGELCFGTIDT; AENGELCFGTIDTW;
ENGELCFGTIDTWI; NGELCFGTIDTWIL; GELCFGTIDTWILW;
ELCFGTIDTWILWN; LCFGTIDTWILWNL; CFGTIDTWILWNLT;
FGTIDTWILWNLTQ; GTIDTWILWNLTQK; TIDTWILWNLTQKK;
IDTWILWNLTQKKE; DTWILWNLTQKKEH; TWILWNLTQKKEHA;
WILWNLTQKKEHAT; ILWNLTQKKEHATD; LWNLTQKKEHATDY;
WNLTQKKEHATDYS; NLTQKKEHATDYSN; LTQKKEHATDYSNA;
TQKKEHATDYSNAS; QKKEHATDYSNASR; KKEHATDYSNASRT;
KEHATDYSNASRTL; EHATDYSNASRTLL; HATDYSNASRTLLL;
ATDYSNASRTLLLN; TDYSNASRTLLLNI; DYSNASRTLLLNIK;
YSNASRTLLLNIKT; SNASRTLLLNIKTL; NASRTLLLNIKTLE;
ASRTLLLNIKTLEW; SRTLLLNIKTLEWD; RTLLLNIKTLEWDD;
TLLLNIKTLEWDDE; LLLNIKTLEWDDEL; LLNIKTLEWDDELL;
LNIKTLEWDDELLS; NIKTLEWDDELLSI; IKTLEWDDELLSIL;
KTLEWDDELLSILN; TLEWDDELLSILNV; LEWDDELLSILNVP;
EWDDELLSILNVPR; WDDELLSILNVPRA; DDELLSILNVPRAI;
DELLSILNVPRAIL; ELLSILNVPRAILP; LLSILNVPRAILPE;
LSILNVPRAILPEL; SILNVPRAILPELK; ILNVPRAILPELKE;
LNVPRAILPELKES; NVPRAILPELKESS; VPRAILPELKESST;
PRAILPELKESSTI; RAILPELKESSTIY; AILPELKESSTIYG;
ILPELKESSTIYGK; LPELKESSTIYGKT; PELKESSTIYGKTD;
ELKESSTIYGKTDK; LKESSTIYGKTDKA; KESSTIYGKTDKAL;
ESSTIYGKTDKALF; SSTIYGKTDKALFG; STIYGKTDKALFGA;
TIYGKTDKALFGAE; IYGKTDKALFGAEI; YGKTDKALFGAEIP;
GKTDKALFGAEIPI; KTDKALFGAEIPIA; TDKALFGAEIPIAG;
DKALFGAEIPIAGI; KALFGAEIPIAGIA; ALFGAEIPIAGIAG;

| | | |
|---|---|---|
| LFGAEIPIAGIAGD; | FGAEIPIAGIAGDQ; | GAEIPIAGIAGDQF; |
| AEIPIAGIAGDQFA; | EIPIAGIAGDQFAA; | IPIAGIAGDQFAAT; |
| PIAGIAGDQFAATF; | IAGIAGDQFAATFG; | AGIAGDQFAATFGQ; |
| GIAGDQFAATFGQA; | IAGDQFAATFGQAC; | AGDQFAATFGQACL; |
| GDQFAATFGQACLK; | DQFAATFGQACLKK; | QFAATFGQACLKKG; |
| FAATFGQACLKKGM; | AATFGQACLKKGMA; | ATFGQACLKKGMAK; |
| TFGQACLKKGMAKN; | FGQACLKKGMAKNT; | GQACLKKGMAKNTY; |
| QACLKKGMAKNTYG; | ACLKKGMAKNTYGT; | CLKKGMAKNTYGTG; |
| LKKGMAKNTYGTC; | KKGMAKNTYGTCF; | KGMAKNTYGTCFL; |
| GMAKNTYGTCFLT; | MAKNTYGTCFLTV; | AKNTYGTCFLTVN; |
| KNTYGTCFLTVNI; | NTYGTCFLTVNIG; | TYGTCFLTVNIGK; |
| YGTGCFLTVNIGKE; | GTGCFLTVNIGKEP; | TGCFLTVNIGKEPI; |
| GCFLTVNIGKEPII; | CFLTVNIGKEPIIS; | FLTVNIGKEPIISH; |
| LTVNIGKEPIISHD; | TVNIGKEPIISHDK; | VNIGKEPIISHDKL; |
| NIGKEPIISHDKLL; | IGKEPIISHDKLLT; | GKEPIISHDKLLTS; |
| KEPIISHDKLLTSI; | EPIISHDKLLTSIA; | PIISHDKLLTSIAW; |
| IISHDKLLTSIAWG; | ISHDKLLTSIAWGR; | SHDKLLTSIAWGRK; |
| HDKLLTSIAWGRKK; | DKLLTSIAWGRKKS; | KLLTSIAWGRKKSV; |
| LLTSIAWGRKKSVT; | LTSIAWGRKKSVTY; | TSIAWGRKKSVTYV; |
| SIAWGRKKSVTYVL; | IAWGRKKSVTYVLE; | AWGRKKSVTYVLEG; |
| WGRKKSVTYVLEGS; | GRKKSVTYVLEGSV; | RKKSVTYVLEGSVF; |
| KKSVTYVLEGSVFI; | KSVTYVLEGSVFIG; | SVTYVLEGSVFIGG; |
| VTYVLEGSVFIGGA; | TYVLEGSVFIGGAV; | YVLEGSVFIGGAVI; |
| VLEGSVFIGGAVIQ; | LEGSVFIGGAVIQW; | EGSVFIGGAVIQWL; |
| GSVFIGGAVIQWLR; | SVFIGGAVIQWLRD; | VFIGGAVIQWLRDG; |
| FIGGAVIQWLRDGL; | IGGAVIQWLRDGLE; | GGAVIQWLRDGLEF; |
| GAVIQWLRDGLEFF; | AVIQWLRDGLEFFR; | VIQWLRDGLEFFRK; |
| IQWLRDGLEFFRKS; | QWLRDGLEFFRKSS; | WLRDGLEFFRKSSD; |
| LRDGLEFFRKSSDA; | RDGLEFFRKSSDAE; | DGLEFFRKSSDAEA; |
| GLEFFRKSSDAEAL; | LEFFRKSSDAEALA; | EFFRKSSDAEALAS; |
| FFRKSSDAEALASS; | FRKSSDAEALASSV; | RKSSDAEALASSVS; |
| KSSDAEALASSVSD; | SSDAEALASSVSDN; | SDAEALASSVSDNG; |
| DAEALASSVSDNGG; | AEALASSVSDNGGV; | EALASSVSDNGGVY; |
| ALASSVSDNGGVYF; | LASSVSDNGGVYFV; | ASSVSDNGGVYFVP; |
| SSVSDNGGVYFVPA; | SVSDNGGVYFVPAF; | VSDNGGVYFVPAFV; |
| SDNGGVYFVPAFVG; | DNGGVYFVPAFVGL; | NGGVYFVPAFVGLG; |
| GGVYFVPAFVGLGA; | GVYFVPAFVGLGAP; | VYFVPAFVGLGAPH; |
| YFVPAFVGLGAPHW; | FVPAFVGLGAPHWD; | VPAFVGLGAPHWDS; |
| PAFVGLGAPHWDSY; | AFVGLGAPHWDSYA; | FVGLGAPHWDSYAR; |
| VGLGAPHWDSYARG; | GLGAPHWDSYARGT; | LGAPHWDSYARGTI; |
| GAPHWDSYARGTII; | APHWDSYARGTIIG; | PHWDSYARGTIIGI; |
| HWDSYARGTIIGIT; | WDSYARGTIIGITR; | DSYARGTIIGITRG; |
| SYARGTIIGITRGS; | YARGTIIGITRGST; | ARGTIIGITRGSTK; |
| RGTIIGITRGSTKA; | GTIIGITRGSTKAH; | TIIGITRGSTKAHI; |
| IIGITRGSTKAHIT; | IGITRGSTKAHITR; | GITRGSTKAHITRA; |
| ITRGSTKAHITRAA; | TRGSTKAHITRAAL; | RGSTKAHITRAALE; |
| GSTKAHITRAALES; | STKAHITRAALESI; | TKAHITRAALESIA; |
| KAHITRAALESIAF; | AHITRAALESIAFQ; | HITRAALESIAFQS; |
| ITRAALESIAFQSF; | TRAALESIAFQSFD; | RAALESIAFQSFDI; |
| AALESIAFQSFDIL; | ALESIAFQSFDILN; | LESIAFQSFDILNT; |
| ESIAFQSFDILNTM; | SIAFQSFDILNTMK; | IAFQSFDILNTMKK; |

AFQSFDILNTMKKS; FQSFDILNTMKKSI; QSFDILNTMKKSIP;
SFDILNTMKKSIPN; FDILNTMKKSIPNF; DILNTMKKSIPNFE;
ILNTMKKSIPNFEI; LNTMKKSIPNFEIQ; NTMKKSIPNFEIQE;
TMKKSIPNFEIQEL; MKKSIPNFEIQELR; KKSIPNFEIQELRV;
KSIPNFEIQELRVD; SIPNFEIQELRVDG; IPNFEIQELRVDGG;
PNFEIQELRVDGGA; NFEIQELRVDGGAS; FEIQELRVDGGASQ;
EIQELRVDGGASQN; IQELRVDGGASQNN; QELRVDGGASQNNL;
ELRVDGGASQNNLL; LRVDGGASQNNLLM; RVDGGASQNNLLMQ;
VDGGASQNNLLMQF; DGGASQNNLLMQFQ; GGASQNNLLMQFQA;
GASQNNLLMQFQAD; ASQNNLLMQFQADL; SQNNLLMQFQADLL;
QNNLLMQFQADLLE; NNLLMQFQADLLEC; NLLMQFQADLLECK;
LLMQFQADLLECKV; LMQFQADLLECKVV; MQFQADLLECKVVR;
QFQADLLECKVVRP; FQADLLECKVVRPK; QADLLECKVVRPKI;
ADLLECKVVRPKIT; DLLECKVVRPKITE; LLECKVVRPKITET;
LECKVVRPKITETT; ECKVVRPKITETTA; CKVVRPKITETTAL;
KVVRPKITETTALG; VVRPKITETTALGA; VRPKITETTALGAA;
RPKITETTALGAAY; PKITETTALGAAYL; KITETTALGAAYLA;
ITETTALGAAYLAG; TETTALGAAYLAGL; ETTALGAAYLAGLA;
TTALGAAYLAGLAT; TALGAAYLAGLATG; ALGAAYLAGLATGY;
LGAAYLAGLATGYW; GAAYLAGLATGYWQ; AAYLAGLATGYWQS;
AYLAGLATGYWQSA; YLAGLATGYWQSAE; LAGLATGYWQSAEE;
AGLATGYWQSAEEI; GLATGYWQSAEEIV; LATGYWQSAEEIVS;
ATGYWQSAEEIVSL; TGYWQSAEEIVSLW; GYWQSAEEIVSLWQ;
YWQSAEEIVSLWQV; WQSAEEIVSLWQVD; QSAEEIVSLWQVDK;
SAEEIVSLWQVDKI; AEEIVSLWQVDKIF; EEIVSLWQVDKIFE;
EIVSLWQVDKIFEP; IVSLWQVDKIFEPS; VSLWQVDKIFEPSM;
SLWQVDKIFEPSMP; LWQVDKIFEPSMPK; WQVDKIFEPSMPKN;
QVDKIFEPSMPKNQ; VDKIFEPSMPKNQK; DKIFEPSMPKNQKE;
KIFEPSMPKNQKEK; IFEPSMPKNQKEKL; FEPSMPKNQKEKLL;
EPSMPKNQKEKLLE; PSMPKNQKEKLLEN; SMPKNQKEKLLENW;
MPKNQKEKLLENWN; PKNQKEKLLENWNK; KNQKEKLLENWNKA;
NQKEKLLENWNKAV; QKEKLLENWNKAVG; KEKLLENWNKAVGK;
EKLLENWNKAVGKA; KLLENWNKAVGKAK; LLENWNKAVGKAKS;
LENWNKAVGKAKSW; ENWNKAVGKAKSWI; NWNKAVGKAKSWIQ;
WNKAVGKAKSWIQN; NKAVGKAKSWIQNS; KAVGKAKSWIQNSH;
AVGKAKSWIQNSHS; VGKAKSWIQNSHSS;

15 mers:
MKYILSIDQGTTSSR; KYILSIDQGTTSSRA; YILSIDQGTTSSRAM;
ILSIDQGTTSSRAMV; LSIDQGTTSSRAMVF; SIDQGTTSSRAMVFD;
IDQGTTSSRAMVFDK; DQGTTSSRAMVFDKN; QGTTSSRAMVFDKNA;
GTTSSRAMVFDKNAN; TTSSRAMVFDKNANI; TSSRAMVFDKNANIK;
SSRAMVFDKNANIKG; SRAMVFDKNANIKGF; RAMVFDKNANIKGFA;
AMVFDKNANIKGFAQ; MVFDKNANIKGFAQK; VFDKNANIKGFAQKE;
FDKNANIKGFAQKEF; DKNANIKGFAQKEFT; KNANIKGFAQKEFTQ;
NANIKGFAQKEFTQI; ANIKGFAQKEFTQIY; NIKGFAQKEFTQIYP;
IKGFAQKEFTQIYPQ; KGFAQKEFTQIYPQP; GFAQKEFTQIYPQPS;
FAQKEFTQIYPQPSW; AQKEFTQIYPQPSWV; QKEFTQIYPQPSWVE;
KEFTQIYPQPSWVEH; EFTQIYPQPSWVEHD; FTQIYPQPSWVEHDP;
TQIYPQPSWVEHDPT; QIYPQPSWVEHDPTE; IYPQPSWVEHDPTEI;
YPQPSWVEHDPTEIW; PQPSWVEHDPTEIWG; QPSWVEHDPTEIWGS;

PSWVEHDPTEIWGSQ; SWVEHDPTEIWGSQL; WVEHDPTEIWGSQLG;
VEHDPTEIWGSQLGV; EHDPTEIWGSQLGVI; HDPTEIWGSQLGVIT;
DPTEIWGSQLGVITE; PTEIWGSQLGVITEA; TEIWGSQLGVITEAM;
EIWGSQLGVITEAMA; IWGSQLGVITEAMAN; WGSQLGVITEAMANA;
GSQLGVITEAMANAR; SQLGVITEAMANARI; QLGVITEAMANARIL;
LGVITEAMANARILP; GVITEAMANARILPN; VITEAMANARILPNE;
ITEAMANARILPNEI; TEAMANARILPNEID; EAMANARILPNEIDA;
AMANARILPNEIDAI; MANARILPNEIDAIG; ANARILPNEIDAIGI;
NARILPNEIDAIGIT; ARILPNEIDAIGITN; RILPNEIDAIGITNQ;
ILPNEIDAIGITNQR; LPNEIDAIGITNQRE; PNEIDAIGITNQRET;
NEIDAIGITNQRETT; EIDAIGITNQRETTV; IDAIGITNQRETTVI;
DAIGITNQRETTVIW; AIGITNQRETTVIWE; IGITNQRETTVIWEK;
GITNQRETTVIWEKN; ITNQRETTVIWEKNT; TNQRETTVIWEKNTG;
NQRETTVIWEKNTGK; QRETTVIWEKNTGKP; RETTVIWEKNTGKPI;
ETTVIWEKNTGKPIY; TTVIWEKNTGKPIYN; TVIWEKNTGKPIYNA;
VIWEKNTGKPIYNAI; IWEKNTGKPIYNAIV; WEKNTGKPIYNAIVW;
EKNTGKPIYNAIVWQ; KNTGKPIYNAIVWQD; NTGKPIYNAIVWQDR;
TGKPIYNAIVWQDRR; GKPIYNAIVWQDRRT; KPIYNAIVWQDRRTA;
PIYNAIVWQDRRTAK; IYNAIVWQDRRTAKI; YNAIVWQDRRTAKIC;
NAIVWQDRRTAKICD; AIVWQDRRTAKICDQ; IVWQDRRTAKICDQL;
VWQDRRTAKICDQLK; WQDRRTAKICDQLKK; QDRRTAKICDQLKKE;
DRRTAKICDQLKKEG; RRTAKICDQLKKEGK; RTAKICDQLKKEGKD;
TAKICDQLKKEGKDK; AKICDQLKKEGKDKI; KICDQLKKEGKDKII;
ICDQLKKEGKDKIIL; CDQLKKEGKDKIILE; DQLKKEGKDKIILEK;
QLKKEGKDKIILEKT; LKKEGKDKIILEKTG; KKEGKDKIILEKTGL;
KEGKDKIILEKTGLV; EGKDKIILEKTGLVL; GKDKIILEKTGLVLD;
KDKIILEKTGLVLDS; DKIILEKTGLVLDSY; KIILEKTGLVLDSYF;
IILEKTGLVLDSYFS; ILEKTGLVLDSYFSG; LEKTGLVLDSYFSGT;
EKTGLVLDSYFSGTK; KTGLVLDSYFSGTKI; TGLVLDSYFSGTKIM;
GLVLDSYFSGTKIMW; LVLDSYFSGTKIMWI; VLDSYFSGTKIMWIL;
LDSYFSGTKIMWILD; DSYFSGTKIMWILDN; SYFSGTKIMWILDNV;
YFSGTKIMWILDNVE; FSGTKIMWILDNVEG; SGTKIMWILDNVEGA;
GTKIMWILDNVEGAR; TKIMWILDNVEGARQ; KIMWILDNVEGARQR;
IMWILDNVEGARQRA; MWILDNVEGARQRAE; WILDNVEGARQRAEN;
ILDNVEGARQRAENG; LDNVEGARQRAENGE; DNVEGARQRAENGEL;
NVEGARQRAENGELC; VEGARQRAENGELCF; EGARQRAENGELCFG;
GARQRAENGELCFGT; ARQRAENGELCFGTI; RQRAENGELCFGTID;
QRAENGELCFGTIDT; RAENGELCFGTIDTW; AENGELCFGTIDTWI;
ENGELCFGTIDTWIL; NGELCFGTIDTWILW; GELCFGTIDTWILWN;
ELCFGTIDTWILWNL; LCFGTIDTWILWNLT; CFGTIDTWILWNLTQ;
FGTIDTWILWNLTQK; GTIDTWILWNLTQKK; TIDTWILWNLTQKKE;
IDTWILWNLTQKKEH; DTWILWNLTQKKEHA; TWILWNLTQKKEHAT;
WILWNLTQKKEHATD; ILWNLTQKKEHATDY; LWNLTQKKEHATDYS;
WNLTQKKEHATDYSN; NLTQKKEHATDYSNA; LTQKKEHATDYSNAS;
TQKKEHATDYSNASR; QKKEHATDYSNASRT; KKEHATDYSNASRTL;
KEHATDYSNASRTLL; EHATDYSNASRTLLL; HATDYSNASRTLLLN;
ATDYSNASRTLLLNI; TDYSNASRTLLLNIK; DYSNASRTLLLNIKT;
YSNASRTLLLNIKTL; SNASRTLLLNIKTLE; NASRTLLLNIKTLEW;
ASRTLLLNIKTLEWD; SRTLLLNIKTLEWDD; RTLLLNIKTLEWDDE;
TLLLNIKTLEWDDEL; LLLNIKTLEWDDELL; LLNIKTLEWDDELLS;
LNIKTLEWDDELLSI; NIKTLEWDDELLSIL; IKTLEWDDELLSILN;

KTLEWDDELLSILNV; TLEWDDELLSILNVP; LEWDDELLSILNVPR;
EWDDELLSILNVPRA; WDDELLSILNVPRAI; DDELLSILNVPRAIL;
DELLSILNVPRAILP; ELLSILNVPRAILPE; LLSILNVPRAILPEL;
LSILNVPRAILPELK; SILNVPRAILPELKE; ILNVPRAILPELKES;
LNVPRAILPELKESS; NVPRAILPELKESST; VPRAILPELKESSTI;
PRAILPELKESSTIY; RAILPELKESSTIYG; AILPELKESSTIYGK;
ILPELKESSTIYGKT; LPELKESSTIYGKTD; PELKESSTIYGKTDK;
ELKESSTIYGKTDKA; LKESSTIYGKTDKAL; KESSTIYGKTDKALF;
ESSTIYGKTDKALFG; SSTIYGKTDKALFGA; STIYGKTDKALFGAE;
TIYGKTDKALFGAEI; IYGKTDKALFGAEIP; YGKTDKALFGAEIPI;
GKTDKALFGAEIPIA; KTDKALFGAEIPIAG; TDKALFGAEIPIAGI;
DKALFGAEIPIAGIA; KALFGAEIPIAGIAG; ALFGAEIPIAGIAGD;
LFGAEIPIAGIAGDQ; FGAEIPIAGIAGDQF; GAEIPIAGIAGDQFA;
AEIPIAGIAGDQFAA; EIPIAGIAGDQFAAT; IPIAGIAGDQFAATF;
PIAGIAGDQFAATFG; IAGIAGDQFAATFGQ; AGIAGDQFAATFGQA;
GIAGDQFAATFGQAC; IAGDQFAATFGQACL; AGDQFAATFGQACLK;
GDQFAATFGQACLKK; DQFAATFGQACLKKG; QFAATFGQACLKKGM;
FAATFGQACLKKGMA; AATFGQACLKKGMAK; ATFGQACLKKGMAKN;
TFGQACLKKGMAKNT; FGQACLKKGMAKNTY; GQACLKKGMAKNTYG;
QACLKKGMAKNTYGT; ACLKKGMAKNTYGTG; CLKKGMAKNTYGTGC;
LKKGMAKNTYGTGCF; KKGMAKNTYGTGCFL; KGMAKNTYGTGCFLT;
GMAKNTYGTGCFLTV; MAKNTYGTGCFLTVN; AKNTYGTGCFLTVNI;
KNTYGTGCFLTVNIG; NTYGTGCFLTVNIGK; TYGTGCFLTVNIGKE;
YGTGCFLTVNIGKEP; GTGCFLTVNIGKEPI; TGCFLTVNIGKEPII;
GCFLTVNIGKEPIIS; CFLTVNIGKEPIISH; FLTVNIGKEPIISHD;
LTVNIGKEPIISHDK; TVNIGKEPIISHDKL; VNIGKEPIISHDKLL;
NIGKEPIISHDKLLT; IGKEPIISHDKLLTS; GKEPIISHDKLLTSI;
KEPIISHDKLLTSIA; EPIISHDKLLTSIAW; PIISHDKLLTSIAWG;
IISHDKLLTSIAWGR; ISHDKLLTSIAWGRK; SHDKLLTSIAWGRKK;
HDKLLTSIAWGRKKS; DKLLTSIAWGRKKSV; KLLTSIAWGRKKSVT;
LLTSIAWGRKKSVTY; LTSIAWGRKKSVTYV; TSIAWGRKKSVTYVL;
SIAWGRKKSVTYVLE; IAWGRKKSVTYVLEG; AWGRKKSVTYVLEGS;
WGRKKSVTYVLEGSV; GRKKSVTYVLEGSVF; RKKSVTYVLEGSVFI;
KKSVTYVLEGSVFIG; KSVTYVLEGSVFIGG; SVTYVLEGSVFIGGA;
VTYVLEGSVFIGGAV; TYVLEGSVFIGGAVI; YVLEGSVFIGGAVIQ;
VLEGSVFIGGAVIQW; LEGSVFIGGAVIQWL; EGSVFIGGAVIQWLR;
GSVFIGGAVIQWLRD; SVFIGGAVIQWLRDG; VFIGGAVIQWLRDGL;
FIGGAVIQWLRDGLE; IGGAVIQWLRDGLEF; GGAVIQWLRDGLEFF;
GAVIQWLRDGLEFFR; AVIQWLRDGLEFFRK; VIQWLRDGLEFFRKS;
IQWLRDGLEFFRKSS; QWLRDGLEFFRKSSD; WLRDGLEFFRKSSDA;
LRDGLEFFRKSSDAE; RDGLEFFRKSSDAEA; DGLEFFRKSSDAEAL;
GLEFFRKSSDAEALA; LEFFRKSSDAEALAS; EFFRKSSDAEALASS;
FFRKSSDAEALASSV; FRKSSDAEALASSVS; RKSSDAEALASSVSD;
KSSDAEALASSVSDN; SSDAEALASSVSDNG; SDAEALASSVSDNGG;
DAEALASSVSDNGGV; AEALASSVSDNGGVY; EALASSVSDNGGVYF;
ALASSVSDNGGVYFV; LASSVSDNGGVYFVP; ASSVSDNGGVYFVPA;
SSVSDNGGVYFVPAF; SVSDNGGVYFVPAFV; VSDNGGVYFVPAFVG;
SDNGGVYFVPAFVGL; DNGGVYFVPAFVGLG; NGGVYFVPAFVGLGA;
GGVYFVPAFVGLGAP; GVYFVPAFVGLGAPH; VYFVPAFVGLGAPHW;
YFVPAFVGLGAPHWD; FVPAFVGLGAPHWDS; VPAFVGLGAPHWDSY;
PAFVGLGAPHWDSYA; AFVGLGAPHWDSYAR; FVGLGAPHWDSYARG;

VGLGAPHWDSYARGT; GLGAPHWDSYARGTI; LGAPHWDSYARGTII;
GAPHWDSYARGTIIG; APHWDSYARGTIIGI; PHWDSYARGTIIGIT;
HWDSYARGTIIGITR; WDSYARGTIIGITRG; DSYARGTIIGITRGS;
SYARGTIIGITRGST; YARGTIIGITRGSTK; ARGTIIGITRGSTKA;
RGTIIGITRGSTKAH; GTIIGITRGSTKAHI; TIIGITRGSTKAHIT;
IIGITRGSTKAHITR; IGITRGSTKAHITRA; GITRGSTKAHITRAA;
ITRGSTKAHITRAAL; TRGSTKAHITRAALE; RGSTKAHITRAALES;
GSTKAHITRAALESI; STKAHITRAALESIA; TKAHITRAALESIAF;
KAHITRAALESIAFQ; AHITRAALESIAFQS; HITRAALESIAFQSF;
ITRAALESIAFQSFD; TRAALESIAFQSFDI; RAALESIAFQSFDIL;
AALESIAFQSFDILN; ALESIAFQSFDILNT; LESIAFQSFDILNTM;
ESIAFQSFDILNTMK; SIAFQSFDILNTMKK; IAFQSFDILNTMKKS;
AFQSFDILNTMKKSI; FQSFDILNTMKKSIP; QSFDILNTMKKSIPN;
SFDILNTMKKSIPNF; FDILNTMKKSIPNFE; DILNTMKKSIPNFEI;
ILNTMKKSIPNFEIQ; LNTMKKSIPNFEIQE; NTMKKSIPNFEIQEL;
TMKKSIPNFEIQELR; MKKSIPNFEIQELRV; KKSIPNFEIQELRVD;
KSIPNFEIQELRVDG; SIPNFEIQELRVDGG; IPNFEIQELRVDGGA;
PNFEIQELRVDGGAS; NFEIQELRVDGGASQ; FEIQELRVDGGASQN;
EIQELRVDGGASQNN; IQELRVDGGASQNNL; QELRVDGGASQNNLL;
ELRVDGGASQNNLLM; LRVDGGASQNNLLMQ; RVDGGASQNNLLMQF;
VDGGASQNNLLMQFQ; DGGASQNNLLMQFQA; GGASQNNLLMQFQAD;
GASQNNLLMQFQADL; ASQNNLLMQFQADLL; SQNNLLMQFQADLLE;
QNNLLMQFQADLLEC; NNLLMQFQADLLECK; NLLMQFQADLLECKV;
LLMQFQADLLECKVV; LMQFQADLLECKVVR; MQFQADLLECKVVRP;
QFQADLLECKVVRPK; FQADLLECKVVRPKI; QADLLECKVVRPKIT;
ADLLECKVVRPKITE; DLLECKVVRPKITET; LLECKVVRPKITETT;
LECKVVRPKITETTA; ECKVVRPKITETTAL; CKVVRPKITETTALG;
KVVRPKITETTALGA; VVRPKITETTALGAA; VRPKITETTALGAAY;
RPKITETTALGAAYL; PKITETTALGAAYLA; KITETTALGAAYLAG;
ITETTALGAAYLAGL; TETTALGAAYLAGLA; ETTALGAAYLAGLAT;
TTALGAAYLAGLATG; TALGAAYLAGLATGY; ALGAAYLAGLATGYW;
LGAAYLAGLATGYWQ; GAAYLAGLATGYWQS; AAYLAGLATGYWQSA;
AYLAGLATGYWQSAE; YLAGLATGYWQSAEE; LAGLATGYWQSAEEI;
AGLATGYWQSAEEIV; GLATGYWQSAEEIVS; LATGYWQSAEEIVSL;
ATGYWQSAEEIVSLW; TGYWQSAEEIVSLWQ; GYWQSAEEIVSLWQV;
YWQSAEEIVSLWQVD; WQSAEEIVSLWQVDK; QSAEEIVSLWQVDKI;
SAEEIVSLWQVDKIF; AEEIVSLWQVDKIFE; EEIVSLWQVDKIFEP;
EIVSLWQVDKIFEPS; IVSLWQVDKIFEPSM; VSLWQVDKIFEPSMP;
SLWQVDKIFEPSMPK; LWQVDKIFEPSMPKN; WQVDKIFEPSMPKNQ;
QVDKIFEPSMPKNQK; VDKIFEPSMPKNQKE; DKIFEPSMPKNQKEK;
KIFEPSMPKNQKEKL; IFEPSMPKNQKEKLL; FEPSMPKNQKEKLLE;
EPSMPKNQKEKLLEN; PSMPKNQKEKLLENW; SMPKNQKEKLLENWN;
MPKNQKEKLLENWNK; PKNQKEKLLENWNKA; KNQKEKLLENWNKAV;
NQKEKLLENWNKAVG; QKEKLLENWNKAVGK; KEKLLENWNKAVGKA;
EKLLENWNKAVGKAK; KLLENWNKAVGKAKS; LLENWNKAVGKAKSW;
LENWNKAVGKAKSWI; ENWNKAVGKAKSWIQ; NWNKAVGKAKSWIQN;
WNKAVGKAKSWIQNS; NKAVGKAKSWIQNSH; KAVGKAKSWIQNSHS;
AVGKAKSWIQNSHSS;

16 mers:
MKYILSIDQGTTSSRA; KYILSIDQGTTSSRAM; YILSIDQGTTSSRAMV;

| | | |
|---|---|---|
| ILSIDQGTTSSRAMVF; | LSIDQGTTSSRAMVFD; | SIDQGTTSSRAMVFDK; |
| IDQGTTSSRAMVFDKN; | DQGTTSSRAMVFDKNA; | QGTTSSRAMVFDKNAN; |
| GTTSSRAMVFDKNANI; | TTSSRAMVFDKNANIK; | TSSRAMVFDKNANIKG; |
| SSRAMVFDKNANIKGF; | SRAMVFDKNANIKGFA; | RAMVFDKNANIKGFAQ; |
| AMVFDKNANIKGFAQK; | MVFDKNANIKGFAQKE; | VFDKNANIKGFAQKEF; |
| FDKNANIKGFAQKEFT; | DKNANIKGFAQKEFTQ; | KNANIKGFAQKEFTQI; |
| NANIKGFAQKEFTQIY; | ANIKGFAQKEFTQIYP; | NIKGFAQKEFTQIYPQ; |
| IKGFAQKEFTQIYPQP; | KGFAQKEFTQIYPQPS; | GFAQKEFTQIYPQPSW; |
| FAQKEFTQIYPQPSWV; | AQKEFTQIYPQPSWVE; | QKEFTQIYPQPSWVEH; |
| KEFTQIYPQPSWVEHD; | EFTQIYPQPSWVEHDP; | FTQIYPQPSWVEHDPT; |
| TQIYPQPSWVEHDPTE; | QIYPQPSWVEHDPTEI; | IYPQPSWVEHDPTEIW; |
| YPQPSWVEHDPTEIWG; | PQPSWVEHDPTEIWGS; | QPSWVEHDPTEIWGSQ; |
| PSWVEHDPTEIWGSQL; | SWVEHDPTEIWGSQLG; | WVEHDPTEIWGSQLGV; |
| VEHDPTEIWGSQLGVI; | EHDPTEIWGSQLGVIT; | HDPTEIWGSQLGVITE; |
| DPTEIWGSQLGVITEA; | PTEIWGSQLGVITEAM; | TEIWGSQLGVITEAMA; |
| EIWGSQLGVITEAMAN; | IWGSQLGVITEAMANA; | WGSQLGVITEAMANAR; |
| GSQLGVITEAMANARI; | SQLGVITEAMANARIL; | QLGVITEAMANARILP; |
| LGVITEAMANARILPN; | GVITEAMANARILPNE; | VITEAMANARILPNEI; |
| ITEAMANARILPNEID; | TEAMANARILPNEIDA; | EAMANARILPNEIDAI; |
| AMANARILPNEIDAIG; | MANARILPNEIDAIGI; | ANARILPNEIDAIGIT; |
| NARILPNEIDAIGITN; | ARILPNEIDAIGITNQ; | RILPNEIDAIGITNQR; |
| ILPNEIDAIGITNQRE; | LPNEIDAIGITNQRET; | PNEIDAIGITNQRETT; |
| NEIDAIGITNQRETTV; | EIDAIGITNQRETTVI; | IDAIGITNQRETTVIW; |
| DAIGITNQRETTVIWE; | AIGITNQRETTVIWEK; | IGITNQRETTVIWEKN; |
| GITNQRETTVIWEKNT; | ITNQRETTVIWEKNTG; | TNQRETTVIWEKNTGK; |
| NQRETTVIWEKNTGKP; | QRETTVIWEKNTGKPI; | RETTVIWEKNTGKPIY; |
| ETTVIWEKNTGKPIYN; | TTVIWEKNTGKPIYNA; | TVIWEKNTGKPIYNAI; |
| VIWEKNTGKPIYNAIV; | IWEKNTGKPIYNAIVW; | WEKNTGKPIYNAIVWQ; |
| EKNTGKPIYNAIVWQD; | KNTGKPIYNAIVWQDR; | NTGKPIYNAIVWQDRR; |
| TGKPIYNAIVWQDRRT; | GKPIYNAIVWQDRRTA; | KPIYNAIVWQDRRTAK; |
| PIYNAIVWQDRRTAKI; | IYNAIVWQDRRTAKIC; | YNAIVWQDRRTAKICD; |
| NAIVWQDRRTAKICDQ; | AIVWQDRRTAKICDQL; | IVWQDRRTAKICDQLK; |
| VWQDRRTAKICDQLKK; | WQDRRTAKICDQLKKE; | QDRRTAKICDQLKKEG; |
| DRRTAKICDQLKKEGK; | RRTAKICDQLKKEGKD; | RTAKICDQLKKEGKDK; |
| TAKICDQLKKEGKDKI; | AKICDQLKKEGKDKII; | KICDQLKKEGKDKIIL; |
| ICDQLKKEGKDKIILE; | CDQLKKEGKDKIILEK; | DQLKKEGKDKIILEKT; |
| QLKKEGKDKIILEKTG; | LKKEGKDKIILEKTGL; | KKEGKDKIILEKTGLV; |
| KEGKDKIILEKTGLVL; | EGKDKIILEKTGLVLD; | GKDKIILEKTGLVLDS; |
| KDKIILEKTGLVLDSY; | DKIILEKTGLVLDSYF; | KIILEKTGLVLDSYFS; |
| IILEKTGLVLDSYFSG; | ILEKTGLVLDSYFSGT; | LEKTGLVLDSYFSGTK; |
| EKTGLVLDSYFSGTKI; | KTGLVLDSYFSGTKIM; | TGLVLDSYFSGTKIMW; |
| GLVLDSYFSGTKIMWI; | LVLDSYFSGTKIMWIL; | VLDSYFSGTKIMWILD; |
| LDSYFSGTKIMWILDN; | DSYFSGTKIMWILDNV; | SYFSGTKIMWILDNVE; |
| YFSGTKIMWILDNVEG; | FSGTKIMWILDNVEGA; | SGTKIMWILDNVEGAR; |
| GTKIMWILDNVEGARQ; | TKIMWILDNVEGARQR; | KIMWILDNVEGARQRA; |
| IMWILDNVEGARQRAE; | MWILDNVEGARQRAEN; | WILDNVEGARQRAENG; |
| ILDNVEGARQRAENGE; | LDNVEGARQRAENGEL; | DNVEGARQRAENGELC; |
| NVEGARQRAENGELCF; | VEGARQRAENGELCFG; | EGARQRAENGELCFGT; |
| GARQRAENGELCFGTI; | ARQRAENGELCFGTID; | RQRAENGELCFGTIDT; |
| QRAENGELCFGTIDTW; | RAENGELCFGTIDTWI; | AENGELCFGTIDTWIL; |
| ENGELCFGTIDTWILW; | NGELCFGTIDTWILWN; | GELCFGTIDTWILWNL; |

ELCFGTIDTWILWNLT; LCFGTIDTWILWNLTQ; CFGTIDTWILWNLTQK;
FGTIDTWILWNLTQKK; GTIDTWILWNLTQKKE; TIDTWILWNLTQKKEH;
IDTWILWNLTQKKEHA; DTWILWNLTQKKEHAT; TWILWNLTQKKEHATD;
WILWNLTQKKEHATDY; ILWNLTQKKEHATDYS; LWNLTQKKEHATDYSN;
WNLTQKKEHATDYSNA; NLTQKKEHATDYSNAS; LTQKKEHATDYSNASR;
TQKKEHATDYSNASRT; QKKEHATDYSNASRTL; KKEHATDYSNASRTLL;
KEHATDYSNASRTLLL; EHATDYSNASRTLLLN; HATDYSNASRTLLLNI;
ATDYSNASRTLLLNIK; TDYSNASRTLLLNIKT; DYSNASRTLLLNIKTL;
YSNASRTLLLNIKTLE; SNASRTLLLNIKTLEW; NASRTLLLNIKTLEWD;
ASRTLLLNIKTLEWDD; SRTLLLNIKTLEWDDE; RTLLLNIKTLEWDDEL;
TLLLNIKTLEWDDELL; LLLNIKTLEWDDELLS; LLNIKTLEWDDELLSI;
LNIKTLEWDDELLSIL; NIKTLEWDDELLSILN; IKTLEWDDELLSILNV;
KTLEWDDELLSILNVP; TLEWDDELLSILNVPR; LEWDDELLSILNVPRA;
EWDDELLSILNVPRAI; WDDELLSILNVPRAIL; DDELLSILNVPRAILP;
DELLSILNVPRAILPE; ELLSILNVPRAILPEL; LLSILNVPRAILPELK;
LSILNVPRAILPELKE; SILNVPRAILPELKES; ILNVPRAILPELKESS;
LNVPRAILPELKESST; NVPRAILPELKESSTI; VPRAILPELKESSTIY;
PRAILPELKESSTIYG; RAILPELKESSTIYGK; AILPELKESSTIYGKT;
ILPELKESSTIYGKTD; LPELKESSTIYGKTDK; PELKESSTIYGKTDKA;
ELKESSTIYGKTDKAL; LKESSTIYGKTDKALF; KESSTIYGKTDKALFG;
ESSTIYGKTDKALFGA; SSTIYGKTDKALFGAE; STIYGKTDKALFGAEI;
TIYGKTDKALFGAEIP; IYGKTDKALFGAEIPI; YGKTDKALFGAEIPIA;
GKTDKALFGAEIPIAG; KTDKALFGAEIPIAGI; TDKALFGAEIPIAGIA;
DKALFGAEIPIAGIAG; KALFGAEIPIAGIAGD; ALFGAEIPIAGIAGDQ;
LFGAEIPIAGIAGDQF; FGAEIPIAGIAGDQFA; GAEIPIAGIAGDQFAA;
AEIPIAGIAGDQFAAT; EIPIAGIAGDQFAATF; IPIAGIAGDQFAATFG;
PIAGIAGDQFAATFGQ; IAGIAGDQFAATFGQA; AGIAGDQFAATFGQAC;
GIAGDQFAATFGQACL; IAGDQFAATFGQACLK; AGDQFAATFGQACLKK;
GDQFAATFGQACLKKG; DQFAATFGQACLKKGM; QFAATFGQACLKKGMA;
FAATFGQACLKKGMAK; AATFGQACLKKGMAKN; ATFGQACLKKGMAKNT;
TFGQACLKKGMAKNTY; FGQACLKKGMAKNTYG; GQACLKKGMAKNTYGT;
QACLKKGMAKNTYGTG; ACLKKGMAKNTYGTGC; CLKKGMAKNTYGTGCF;
LKKGMAKNTYGTGCFL; KKGMAKNTYGTGCFLT; KGMAKNTYGTGCFLTV;
GMAKNTYGTGCFLTVN; MAKNTYGTGCFLTVNI; AKNTYGTGCFLTVNIG;
KNTYGTGCFLTVNIGK; NTYGTGCFLTVNIGKE; TYGTGCFLTVNIGKEP;
YGTGCFLTVNIGKEPI; GTGCFLTVNIGKEPII; TGCFLTVNIGKEPIIS;
GCFLTVNIGKEPIISH; CFLTVNIGKEPIISHD; FLTVNIGKEPIISHDK;
LTVNIGKEPIISHDKL; TVNIGKEPIISHDKLL; VNIGKEPIISHDKLLT;
NIGKEPIISHDKLLTS; IGKEPIISHDKLLTSI; GKEPIISHDKLLTSIA;
KEPIISHDKLLTSIAW; EPIISHDKLLTSIAWG; PIISHDKLLTSIAWGR;
IISHDKLLTSIAWGRK; ISHDKLLTSIAWGRKK; SHDKLLTSIAWGRKKS;
HDKLLTSIAWGRKKSV; DKLLTSIAWGRKKSVT; KLLTSIAWGRKKSVTY;
LLTSIAWGRKKSVTYV; LTSIAWGRKKSVTYVL; TSIAWGRKKSVTYVLE;
SIAWGRKKSVTYVLEG; IAWGRKKSVTYVLEGS; AWGRKKSVTYVLEGSV;
WGRKKSVTYVLEGSVF; GRKKSVTYVLEGSVFI; RKKSVTYVLEGSVFIG;
KKSVTYVLEGSVFIGG; KSVTYVLEGSVFIGGA; SVTYVLEGSVFIGGAV;
VTYVLEGSVFIGGAVI; TYVLEGSVFIGGAVIQ; YVLEGSVFIGGAVIQW;
VLEGSVFIGGAVIQWL; LEGSVFIGGAVIQWLR; EGSVFIGGAVIQWLRD;
GSVFIGGAVIQWLRDG; SVFIGGAVIQWLRDGL; VFIGGAVIQWLRDGLE;
FIGGAVIQWLRDGLEF; IGGAVIQWLRDGLEFF; GGAVIQWLRDGLEFFR;
GAVIQWLRDGLEFFRK; AVIQWLRDGLEFFRKS; VIQWLRDGLEFFRKSS;

| | | |
|---|---|---|
| IQWLRDGLEFFRKSSD; | QWLRDGLEFFRKSSDA; | WLRDGLEFFRKSSDAE; |
| LRDGLEFFRKSSDAEA; | RDGLEFFRKSSDAEAL; | DGLEFFRKSSDAEALA; |
| GLEFFRKSSDAEALAS; | LEFFRKSSDAEALASS; | EFFRKSSDAEALASSV; |
| FFRKSSDAEALASSVS; | FRKSSDAEALASSVSD; | RKSSDAEALASSVSDN; |
| KSSDAEALASSVSDNG; | SSDAEALASSVSDNGG; | SDAEALASSVSDNGGV; |
| DAEALASSVSDNGGVY; | AEALASSVSDNGGVYF; | EALASSVSDNGGVYFV; |
| ALASSVSDNGGVYFVP; | LASSVSDNGGVYFVPA; | ASSVSDNGGVYFVPAF; |
| SSVSDNGGVYFVPAFV; | SVSDNGGVYFVPAFVG; | VSDNGGVYFVPAFVGL; |
| SDNGGVYFVPAFVGLG; | DNGGVYFVPAFVGLGA; | NGGVYFVPAFVGLGAP; |
| GGVYFVPAFVGLGAPH; | GVYFVPAFVGLGAPHW; | VYFVPAFVGLGAPHWD; |
| YFVPAFVGLGAPHWDS; | FVPAFVGLGAPHWDSY; | VPAFVGLGAPHWDSYA; |
| PAFVGLGAPHWDSYAR; | AFVGLGAPHWDSYARG; | FVGLGAPHWDSYARGT; |
| VGLGAPHWDSYARGTI; | GLGAPHWDSYARGTII; | LGAPHWDSYARGTIIG; |
| GAPHWDSYARGTIIGI; | APHWDSYARGTIIGIT; | PHWDSYARGTIIGITR; |
| HWDSYARGTIIGITRG; | WDSYARGTIIGITRGS; | DSYARGTIIGITRGST; |
| SYARGTIIGITRGSTK; | YARGTIIGITRGSTKA; | ARGTIIGITRGSTKAH; |
| RGTIIGITRGSTKAHI; | GTIIGITRGSTKAHIT; | TIIGITRGSTKAHITR; |
| IIGITRGSTKAHITRA; | IGITRGSTKAHITRAA; | GITRGSTKAHITRAAL; |
| ITRGSTKAHITRAALE; | TRGSTKAHITRAALES; | RGSTKAHITRAALESI; |
| GSTKAHITRAALESIA; | STKAHITRAALESIAF; | TKAHITRAALESIAFQ; |
| KAHITRAALESIAFQS; | AHITRAALESIAFQSF; | HITRAALESIAFQSFD; |
| ITRAALESIAFQSFDI; | TRAALESIAFQSFDIL; | RAALESIAFQSFDILN; |
| AALESIAFQSFDILNT; | ALESIAFQSFDILNTM; | LESIAFQSFDILNTMK; |
| ESIAFQSFDILNTMKK; | SIAFQSFDILNTMKKS; | IAFQSFDILNTMKKSI; |
| AFQSFDILNTMKKSIP; | FQSFDILNTMKKSIPN; | QSFDILNTMKKSIPNF; |
| SFDILNTMKKSIPNFE; | FDILNTMKKSIPNFEI; | DILNTMKKSIPNFEIQ; |
| ILNTMKKSIPNFEIQE; | LNTMKKSIPNFEIQEL; | NTMKKSIPNFEIQELR; |
| TMKKSIPNFEIQELRV; | MKKSIPNFEIQELRVD; | KKSIPNFEIQELRVDG; |
| KSIPNFEIQELRVDGG; | SIPNFEIQELRVDGGA; | IPNFEIQELRVDGGAS; |
| PNFEIQELRVDGGASQ; | NFEIQELRVDGGASQN; | FEIQELRVDGGASQNN; |
| EIQELRVDGGASQNNL; | IQELRVDGGASQNNLL; | QELRVDGGASQNNLLM; |
| ELRVDGGASQNNLLMQ; | LRVDGGASQNNLLMQF; | RVDGGASQNNLLMQFQ; |
| VDGGASQNNLLMQFQA; | DGGASQNNLLMQFQAD; | GGASQNNLLMQFQADL; |
| GASQNNLLMQFQADLL; | ASQNNLLMQFQADLLE; | SQNNLLMQFQADLLEC; |
| QNNLLMQFQADLLECK; | NNLLMQFQADLLECKV; | NLLMQFQADLLECKVV; |
| LLMQFQADLLECKVVR; | LMQFQADLLECKVVRP; | MQFQADLLECKVVRPK; |
| QFQADLLECKVVRPKI; | FQADLLECKVVRPKIT; | QADLLECKVVRPKITE; |
| ADLLECKVVRPKITET; | DLLECKVVRPKITETT; | LLECKVVRPKITETTA; |
| LECKVVRPKITETTAL; | ECKVVRPKITETTALG; | CKVVRPKITETTALGA; |
| KVVRPKITETTALGAA; | VVRPKITETTALGAAY; | VRPKITETTALGAAYL; |
| RPKITETTALGAAYLA; | PKITETTALGAAYLAG; | KITETTALGAAYLAGL; |
| ITETTALGAAYLAGLA; | TETTALGAAYLAGLAT; | ETTALGAAYLAGLATG; |
| TTALGAAYLAGLATGY; | TALGAAYLAGLATGYW; | ALGAAYLAGLATGYWQ; |
| LGAAYLAGLATGYWQS; | GAAYLAGLATGYWQSA; | AAYLAGLATGYWQSAE; |
| AYLAGLATGYWQSAEE; | YLAGLATGYWQSAEEI; | LAGLATGYWQSAEEIV; |
| AGLATGYWQSAEEIVS; | GLATGYWQSAEEIVSL; | LATGYWQSAEEIVSLW; |
| ATGYWQSAEEIVSLWQ; | TGYWQSAEEIVSLWQV; | GYWQSAEEIVSLWQVD; |
| YWQSAEEIVSLWQVDK; | WQSAEEIVSLWQVDKI; | QSAEEIVSLWQVDKIF; |
| SAEEIVSLWQVDKIFE; | AEEIVSLWQVDKIFEP; | EEIVSLWQVDKIFEPS; |
| EIVSLWQVDKIFEPSM; | IVSLWQVDKIFEPSMP; | VSLWQVDKIFEPSMPK; |
| SLWQVDKIFEPSMPKN; | LWQVDKIFEPSMPKNQ; | WQVDKIFEPSMPKNQK; |

| | |
|---|---|
| | QVDKIFEPSMPKNQKE; VDKIFEPSMPKNQKEK; DKIFEPSMPKNQKEKL; KIFEPSMPKNQKEKLL; IFEPSMPKNQKEKLLE; FEPSMPKNQKEKLLEN; EPSMPKNQKEKLLENW; PSMPKNQKEKLLENWN; SMPKNQKEKLLENWNK; MPKNQKEKLLENWNKA; PKNQKEKLLENWNKAV; KNQKEKLLENWNKAVG; NQKEKLLENWNKAVGK; QKEKLLENWNKAVGKA; KEKLLENWNKAVGKAK; EKLLENWNKAVGKAKS; KLLENWNKAVGKAKSW; LLENWNKAVGKAKSWI; LENWNKAVGKAKSWIQ; ENWNKAVGKAKSWIQN; NWNKAVGKAKSWIQNS; WNKAVGKAKSWIQNSH; NKAVGKAKSWIQNSHS; KAVGKAKSWIQNSHSS; |
| Seq 28 | 13 mers:<br>MKTIKKDSEFYDS; KTIKKDSEFYDSL; TIKKDSEFYDSLA; IKKDSEFYDSLAT; KKDSEFYDSLATL; KDSEFYDSLATLK; DSEFYDSLATLKK; SEFYDSLATLKKH; EFYDSLATLKKHI; FYDSLATLKKHIN; YDSLATLKKHINK; DSLATLKKHINKY; SLATLKKHINKYI; LATLKKHINKYIE; ATLKKHINKYIEE; TLKKHINKYIEEK; LKKHINKYIEEKV; KKHINKYIEEKVL; KHINKYIEEKVLK; HINKYIEEKVLKY; INKYIEEKVLKYS; NKYIEEKVLKYSI; KYIEEKVLKYSIS; YIEEKVLKYSISS; IEEKVLKYSISSQ; EEKVLKYSISSQL; EKVLKYSISSQLY; KVLKYSISSQLYK; VLKYSISSQLYKL; LKYSISSQLYKLE; KYSISSQLYKLEK; YSISSQLYKLEKP; SISSQLYKLEKPE; ISSQLYKLEKPEI; SSQLYKLEKPEII; SQLYKLEKPEIIE; QLYKLEKPEIIEL; LYKLEKPEIIELI; YKLEKPEIIELIK; KLEKPEIIELIKI; LEKPEIIELIKIS; EKPEIIELIKISN; KPEIIELIKISND; PEIIELIKISNDY; EIIELIKISNDYE; IIELIKISNDYEK; IELIKISNDYEKE; ELIKISNDYEKEK; LIKISNDYEKEKN; IKISNDYEKEKNA; KISNDYEKEKNAF; ISNDYEKEKNAFN; SNDYEKEKNAFNT; NDYEKEKNAFNTS; DYEKEKNAFNTSL; YEKEKNAFNTSLE; EKEKNAFNTSLEE; KEKNAFNTSLEEC; EKNAFNTSLEECY; KNAFNTSLEECYY; NAFNTSLEECYYK; AFNTSLEECYYKN; FNTSLEECYYKNT; NTSLEECYYKNTQ; TSLEECYYKNTQN; SLEECYYKNTQNE; LEECYYKNTQNEA; EECYYKNTQNEAI; ECYYKNTQNEAIK; CYYKNTQNEAIKK; YYKNTQNEAIKKW; YKNTQNEAIKKWI; KNTQNEAIKKWIL; NTQNEAIKKWILE; TQNEAIKKWILEI; QNEAIKKWILEIV; NEAIKKWILEIVR; EAIKKWILEIVRN; AIKKWILEIVRNK; IKKWILEIVRNKN; KKWILEIVRNKNF; KWILEIVRNKNFI; WILEIVRNKNFIQ; ILEIVRNKNFIQI; LEIVRNKNFIQIS; EIVRNKNFIQISK; IVRNKNFIQISKE; VRNKNFIQISKEA; RNKNFIQISKEAN; NKNFIQISKEANL; KNFIQISKEANLN; NFIQISKEANLNT; FIQISKEANLNTK; IQISKEANLNTKK; QISKEANLNTKKL; ISKEANLNTKKLG; SKEANLNTKKLGT; KEANLNTKKLGTT; EANLNTKKLGTTY; ANLNTKKLGTTYK; NLNTKKLGTTYKL; LNTKKLGTTYKLK; NTKKLGTTYKLKS; TKKLGTTYKLKSS; KKLGTTYKLKSSN; KLGTTYKLKSSNF; LGTTYKLKSSNFL; GTTYKLKSSNFLK; TTYKLKSSNFLKL; TYKLKSSNFLKLI; YKLKSSNFLKLIE; KLKSSNFLKLIEI; LKSSNFLKLIEIQ; KSSNFLKLIEIQN; SSNFLKLIEIQNS; SNFLKLIEIQNSP; NFLKLIEIQNSPY; FLKLIEIQNSPYY; LKLIEIQNSPYYS; KLIEIQNSPYYSQ; |

LIEIQNSPYYSQE; IEIQNSPYYSQEK; EIQNSPYYSQEKK;
IQNSPYYSQEKKD; QNSPYYSQEKKDI; NSPYYSQEKKDIY;
SPYYSQEKKDIYK; PYYSQEKKDIYKQ; YYSQEKKDIYKQI;
YSQEKKDIYKQII; SQEKKDIYKQIIL; QEKKDIYKQIILN;
EKKDIYKQIILNF; KKDIYKQIILNFS; KDIYKQIILNFSS;
DIYKQIILNFSSN; IYKQIILNFSSNI; YKQIILNFSSNIN;
KQIILNFSSNINI; QIILNFSSNINID; IILNFSSNINIDS;
ILNFSSNINIDSL; LNFSSNINIDSLE; NFSSNINIDSLEQ;
FSSNINIDSLEQT; SSNINIDSLEQTI; SNINIDSLEQTID;
NINIDSLEQTIDI; INIDSLEQTIDIL; NIDSLEQTIDILV;
IDSLEQTIDILVA; DSLEQTIDILVAV; SLEQTIDILVAVR;
LEQTIDILVAVRN; EQTIDILVAVRNR; QTIDILVAVRNRN;
TIDILVAVRNRNK; IDILVAVRNRNKI; DILVAVRNRNKIK;
ILVAVRNRNKIKI; LVAVRNRNKIKIL; VAVRNRNKIKILN;
AVRNRNKIKILNI; VRNRNKIKILNIL; RNRNKIKILNILN;
NRNKIKILNILNK; RNKIKILNILNKN; NKIKILNILNKNL;
KIKILNILNKNLK; IKILNILNKNLKY; KILNILNKNLKYR;
ILNILNKNLKYRP; LNILNKNLKYRPE; NILNKNLKYRPEN;
ILNKNLKYRPENQ; LNKNLKYRPENQK; NKNLKYRPENQKV;
KNLKYRPENQKVF; NLKYRPENQKVFK; LKYRPENQKVFKS;
KYRPENQKVFKSS; YRPENQKVFKSSL; RPENQKVFKSSLT;
PENQKVFKSSLTN; ENQKVFKSSLTNK; NQKVFKSSLTNKE;
QKVFKSSLTNKES; KVFKSSLTNKESR; VFKSSLTNKESRL;
FKSSLTNKESRLI; KSSLTNKESRLIK; SSLTNKESRLIKL;
SLTNKESRLIKLK; LTNKESRLIKLKR; TNKESRLIKLKRM;
NKESRLIKLKRML; KESRLIKLKRMLI; ESRLIKLKRMLIL;
SRLIKLKRMLILT; RLIKLKRMLILTY; LIKLKRMLILTYW;
IKLKRMLILTYWP; KLKRMLILTYWPV; LKRMLILTYWPVG;
KRMLILTYWPVGC; RMLILTYWPVGCL; MLILTYWPVGCLS;
LILTYWPVGCLSK; ILTYWPVGCLSKN; LTYWPVGCLSKNI;
TYWPVGCLSKNIF; YWPVGCLSKNIFI; WPVGCLSKNIFIK;
PVGCLSKNIFIKI; VGCLSKNIFIKIL; GCLSKNIFIKILI;
CLSKNIFIKILIK; LSKNIFIKILIKX; SKNIFIKILIKXY;
KNIFIKILIKXYK; NIFIKILIKXYKY; IFIKILIKXYKYL;
FIKILIKXYKYLE; IKILIKXYKYLEE; KILIKXYKYLEEE;
ILIKXYKYLEEEI; LIKXYKYLEEEIL; IKXYKYLEEEILA;
KXYKYLEEEILAL; XYKYLEEEILALK; YKYLEEEILALKY;
KYLEEEILALKYN; YLEEEILALKYNE; LEEEILALKYNEI;
EEEILALKYNEIL; EEILALKYNEILN; EILALKYNEILNY;
ILALKYNEILNYL; LALKYNEILNYLR; ALKYNEILNYLRA;
LKYNEILNYLRAL; KYNEILNYLRALK; YNEILNYLRALKT;
NEILNYLRALKTL; EILNYLRALKTLS; ILNYLRALKTLSL;
LNYLRALKTLSLN; NYLRALKTLSLNE; YLRALKTLSLNEI;
LRALKTLSLNEIF; RALKTLSLNEIFY; ALKTLSLNEIFYK;
LKTLSLNEIFYKG; KTLSLNEIFYKGS; TLSLNEIFYKGSS;
LSLNEIFYKGSSK; SLNEIFYKGSSKN; LNEIFYKGSSKNI;
NEIFYKGSSKNIS; EIFYKGSSKNISF; IFYKGSSKNISFN;
FYKGSSKNISFNY; YKGSSKNISFNYF; KGSSKNISFNYFF;
GSSKNISFNYFFS; SSKNISFNYFFSD; SKNISFNYFFSDF;
KNISFNYFFSDFT; NISFNYFFSDFTQ; ISFNYFFSDFTQY;
SFNYFFSDFTQYA; FNYFFSDFTQYAP; NYFFSDFTQYAPK;

YFFSDFTQYAPKD; FFSDFTQYAPKDF; FSDFTQYAPKDFQ;
SDFTQYAPKDFQD; DFTQYAPKDFQDT; FTQYAPKDFQDTL;
TQYAPKDFQDTLK; QYAPKDFQDTLKC; YAPKDFQDTLKCY;
APKDFQDTLKCYL; PKDFQDTLKCYLY; KDFQDTLKCYLYV;
DFQDTLKCYLYVI; FQDTLKCYLYVIE; QDTLKCYLYVIEK;
DTLKCYLYVIEKT; TLKCYLYVIEKTI; LKCYLYVIEKTIT;
KCYLYVIEKTITK; CYLYVIEKTITKK; YLYVIEKTITKKY;
LYVIEKTITKKYL; YVIEKTITKKYLT; VIEKTITKKYLTW;
IEKTITKKYLTWF; EKTITKKYLTWFF; KTITKKYLTWFFK;
TITKKYLTWFFKD; ITKKYLTWFFKDK; TKKYLTWFFKDKI;
KKYLTWFFKDKIS; KYLTWFFKDKISN; YLTWFFKDKISNN;
LTWFFKDKISNNW; TWFFKDKISNNWE; WFFKDKISNNWES;
FFKDKISNNWESF; FKDKISNNWESFI; KDKISNNWESFID;
DKISNNWESFIDA; KISNNWESFIDAL; ISNNWESFIDALE;
SNNWESFIDALEY; NNWESFIDALEYI; NWESFIDALEYIE;
WESFIDALEYIEK; ESFIDALEYIEKH; SFIDALEYIEKHK;
FIDALEYIEKHKL; IDALEYIEKHKLI; DALEYIEKHKLIN;
ALEYIEKHKLINI; LEYIEKHKLINIK; EYIEKHKLINIKE;
YIEKHKLINIKEK; IEKHKLINIKEKI; EKHKLINIKEKIK;
KHKLINIKEKIKE; HKLINIKEKIKEI; KLINIKEKIKEII;
LINIKEKIKEIIT; INIKEKIKEIITA; NIKEKIKEIITAK;
IKEKIKEIITAKF; KEKIKEIITAKFQ; EKIKEIITAKFQT;
KIKEIITAKFQTE; IKEIITAKFQTED; KEIITAKFQTEDF;
EIITAKFQTEDFF; IITAKFQTEDFFI; ITAKFQTEDFFIS;
TAKFQTEDFFISF; AKFQTEDFFISFD; KFQTEDFFISFDK;
FQTEDFFISFDKT; QTEDFFISFDKTN; TEDFFISFDKTNF;
EDFFISFDKTNFN; DFFISFDKTNFNP; FFISFDKTNFNPY;
FISFDKTNFNPYE; ISFDKTNFNPYES; SFDKTNFNPYESS;
FDKTNFNPYESSI; DKTNFNPYESSIF; KTNFNPYESSIFK;
TNFNPYESSIFKE; NFNPYESSIFKER; FNPYESSIFKERY;
NPYESSIFKERYI; PYESSIFKERYIK; YESSIFKERYIKS;
ESSIFKERYIKSA; SSIFKERYIKSAF; SIFKERYIKSAFL;
IFKERYIKSAFLE; FKERYIKSAFLEI; KERYIKSAFLEII;
ERYIKSAFLEIIM; RYIKSAFLEIIMH; YIKSAFLEIIMHY;
IKSAFLEIIMHYV; KSAFLEIIMHYVK; SAFLEIIMHYVKK;
AFLEIIMHYVKKN; FLEIIMHYVKKNS; LEIIMHYVKKNSQ;
EIIMHYVKKNSQN; IIMHYVKKNSQNI; IMHYVKKNSQNIE;
MHYVKKNSQNIET; HYVKKNSQNIETY; YVKKNSQNIETYG;
VKKNSQNIETYGW; KKNSQNIETYGWI; KNSQNIETYGWIA;
NSQNIETYGWIAF; SQNIETYGWIAFY; QNIETYGWIAFYI;
NIETYGWIAFYIY; IETYGWIAFYIYA; ETYGWIAFYIYAD;
TYGWIAFYIYADN; YGWIAFYIYADNK; GWIAFYIYADNKD;
WIAFYIYADNKDK; IAFYIYADNKDKK; AFYIYADNKDKKI;
FYIYADNKDKKIF; YIYADNKDKKIFC; IYADNKDKKIFCQ;
YADNKDKKIFCQR; ADNKDKKIFCQRM; DNKDKKIFCQRMK;
NKDKKIFCQRMKE; KDKKIFCQRMKEF; DKKIFCQRMKEFF;
KKIFCQRMKEFFK; KIFCQRMKEFFKS; IFCQRMKEFFKSK;
FCQRMKEFFKSKT; CQRMKEFFKSKTF; QRMKEFFKSKTFE;
RMKEFFKSKTFEI; MKEFFKSKTFEIQ; KEFFKSKTFEIQN;
EFFKSKTFEIQNQ; FFKSKTFEIQNQI; FKSKTFEIQNQIF;
KSKTFEIQNQIFV; SKTFEIQNQIFVY; KTFEIQNQIFVYF;

TFEIQNQIFVYFL; FEIQNQIFVYFLS; EIQNQIFVYFLSF;
IQNQIFVYFLSFY; QNQIFVYFLSFYP; NQIFVYFLSFYPN;
QIFVYFLSFYPNI; IFVYFLSFYPNIE; FVYFLSFYPNIEY;
VYFLSFYPNIEYK; YFLSFYPNIEYKH; FLSFYPNIEYKHF;
LSFYPNIEYKHFK; SFYPNIEYKHFKF; FYPNIEYKHFKFI;
YPNIEYKHFKFIS; PNIEYKHFKFISD; NIEYKHFKFISDI;
IEYKHFKFISDIL; EYKHFKFISDILL; YKHFKFISDILLY;
KHFKFISDILLYL; HFKFISDILLYLD; FKFISDILLYLDE;
KFISDILLYLDEN; FISDILLYLDENK; ISDILLYLDENKI;
SDILLYLDENKIT; DILLYLDENKITI; ILLYLDENKITIP;
LLYLDENKITIPK; LYLDENKITIPKK; YLDENKITIPKKI;
LDENKITIPKKII; DENKITIPKKIIK; ENKITIPKKIIKI;
NKITIPKKIIKIQ; KITIPKKIIKIQK; ITIPKKIIKIQKI;
TIPKKIIKIQKIN; IPKKIIKIQKINP; PKKIIKIQKINPN;
KKIIKIQKINPNQ; KIIKIQKINPNQL; IIKIQKINPNQLD;
IKIQKINPNQLDY; KIQKINPNQLDYQ; IQKINPNQLDYQK;
QKINPNQLDYQKS; KINPNQLDYQKSY; INPNQLDYQKSYL;
NPNQLDYQKSYLL; PNQLDYQKSYLLI; NQLDYQKSYLLIK;
QLDYQKSYLLIKS; LDYQKSYLLIKSL; DYQKSYLLIKSLK;
YQKSYLLIKSLKT; QKSYLLIKSLKTR; KSYLLIKSLKTRS;
SYLLIKSLKTRSR; YLLIKSLKTRSRI; LLIKSLKTRSRIL;
LIKSLKTRSRILK; IKSLKTRSRILKI; KSLKTRSRILKII;
SLKTRSRILKIII; LKTRSRILKIIIK; KTRSRILKIIIKN;
TRSRILKIIIKNI; RSRILKIIIKNIR; SRILKIIIKNIRF;
RILKIIIKNIRFN; ILKIIIKNIRFNL; LKIIIKNIRFNLI;
KIIIKNIRFNLIE; IIIKNIRFNLIEK; IIKNIRFNLIEKL;
IKNIRFNLIEKLE; KNIRFNLIEKLED; NIRFNLIEKLEDE;
IRFNLIEKLEDEN; RFNLIEKLEDENE; FNLIEKLEDENEK;
NLIEKLEDENEKK; LIEKLEDENEKKL; IEKLEDENEKKLL;
EKLEDENEKKLLP; KLEDENEKKLLPA; LEDENEKKLLPAM;
EDENEKKLLPAMC; DENEKKLLPAMCY; ENEKKLLPAMCYL;
NEKKLLPAMCYLI; EKKLLPAMCYLIY; KKLLPAMCYLIYC;
KLLPAMCYLIYCE; LLPAMCYLIYCEN; LPAMCYLIYCENL;
PAMCYLIYCENLV; AMCYLIYCENLVE; MCYLIYCENLVEL;
CYLIYCENLVELK; YLIYCENLVELKN; LIYCENLVELKNN;
IYCENLVELKNNR; YCENLVELKNNRK; CENLVELKNNRKI;
ENLVELKNNRKIK; NLVELKNNRKIKS; LVELKNNRKIKSN;
VELKNNRKIKSNE; ELKNNRKIKSNEK; LKNNRKIKSNEKN;
KNNRKIKSNEKNS; NNRKIKSNEKNSL; NRKIKSNEKNSLL;
RKIKSNEKNSLLE; KIKSNEKNSLLEF; IKSNEKNSLLEFI;
KSNEKNSLLEFIS; SNEKNSLLEFISF; NEKNSLLEFISFF;
EKNSLLEFISFFK; KNSLLEFISFFKT; NSLLEFISFFKTK;
SLLEFISFFKTKL; LLEFISFFKTKLK; LEFISFFKTKLKQ;
EFISFFKTKLKQK; FISFFKTKLKQKI; ISFFKTKLKQKIN;
SFFKTKLKQKINF; FFKTKLKQKINFK; FKTKLKQKINFKK;
KTKLKQKINFKKE; TKLKQKINFKKEL; KLKQKINFKKELM;
LKQKINFKKELMK; KQKINFKKELMKS; QKINFKKELMKSK;
KINFKKELMKSKG; INFKKELMKSKGI;

14 mers:
MKTIKKDSEFYDSL; KTIKKDSEFYDSLA; TIKKDSEFYDSLAT;

| | | |
|---|---|---|
| IKKDSEFYDSLATL; | KKDSEFYDSLATLK; | KDSEFYDSLATLKK; |
| DSEFYDSLATLKKH; | SEFYDSLATLKKHI; | EFYDSLATLKKHIN; |
| FYDSLATLKKHINK; | YDSLATLKKHINKY; | DSLATLKKHINKYI; |
| SLATLKKHINKYIE; | LATLKKHINKYIEE; | ATLKKHINKYIEEK; |
| TLKKHINKYIEEKV; | LKKHINKYIEEKVL; | KKHINKYIEEKVLK; |
| KHINKYIEEKVLKY; | HINKYIEEKVLKYS; | INKYIEEKVLKYSI; |
| NKYIEEKVLKYSIS; | KYIEEKVLKYSISS; | YIEEKVLKYSISSQ; |
| IEEKVLKYSISSQL; | EEKVLKYSISSQLY; | EKVLKYSISSQLYK; |
| KVLKYSISSQLYKL; | VLKYSISSQLYKLE; | LKYSISSQLYKLEK; |
| KYSISSQLYKLEKP; | YSISSQLYKLEKPE; | SISSQLYKLEKPEI; |
| ISSQLYKLEKPEII; | SSQLYKLEKPEIIE; | SQLYKLEKPEIIEL; |
| QLYKLEKPEIIELI; | LYKLEKPEIIELIK; | YKLEKPEIIELIKI; |
| KLEKPEIIELIKIS; | LEKPEIIELIKISN; | EKPEIIELIKISND; |
| KPEIIELIKISNDY; | PEIIELIKISNDYE; | EIIELIKISNDYEK; |
| IIELIKISNDYEKE; | IELIKISNDYEKEK; | ELIKISNDYEKEKN; |
| LIKISNDYEKEKNA; | IKISNDYEKEKNAF; | KISNDYEKEKNAFN; |
| ISNDYEKEKNAFNT; | SNDYEKEKNAFNTS; | NDYEKEKNAFNTSL; |
| DYEKEKNAFNTSLE; | YEKEKNAFNTSLEE; | EKEKNAFNTSLEEC; |
| KEKNAFNTSLEECY; | EKNAFNTSLEECYY; | KNAFNTSLEECYYK; |
| NAFNTSLEECYYKN; | AFNTSLEECYYKNT; | FNTSLEECYYKNTQ; |
| NTSLEECYYKNTQN; | TSLEECYYKNTQNE; | SLEECYYKNTQNEA; |
| LEECYYKNTQNEAI; | EECYYKNTQNEAIK; | ECYYKNTQNEAIKK; |
| CYYKNTQNEAIKKW; | YYKNTQNEAIKKWI; | YKNTQNEAIKKWIL; |
| KNTQNEAIKKWILE; | NTQNEAIKKWILEI; | TQNEAIKKWILEIV; |
| QNEAIKKWILEIVR; | NEAIKKWILEIVRN; | EAIKKWILEIVRNK; |
| AIKKWILEIVRNKN; | IKKWILEIVRNKNF; | KKWILEIVRNKNFI; |
| KWILEIVRNKNFIQ; | WILEIVRNKNFIQI; | ILEIVRNKNFIQIS; |
| LEIVRNKNFIQISK; | EIVRNKNFIQISKE; | IVRNKNFIQISKEA; |
| VRNKNFIQISKEAN; | RNKNFIQISKEANL; | NKNFIQISKEANLN; |
| KNFIQISKEANLNT; | NFIQISKEANLNTK; | FIQISKEANLNTKK; |
| IQISKEANLNTKKL; | QISKEANLNTKKLG; | ISKEANLNTKKLGT; |
| SKEANLNTKKLGTT; | KEANLNTKKLGTTY; | EANLNTKKLGTTYK; |
| ANLNTKKLGTTYKL; | NLNTKKLGTTYKLK; | LNTKKLGTTYKLKS; |
| NTKKLGTTYKLKSS; | TKKLGTTYKLKSSN; | KKLGTTYKLKSSNF; |
| KLGTTYKLKSSNFL; | LGTTYKLKSSNFLK; | GTTYKLKSSNFLKL; |
| TTYKLKSSNFLKLI; | TYKLKSSNFLKLIE; | YKLKSSNFLKLIEI; |
| KLKSSNFLKLIEIQ; | LKSSNFLKLIEIQN; | KSSNFLKLIEIQNS; |
| SSNFLKLIEIQNSP; | SNFLKLIEIQNSPY; | NFLKLIEIQNSPYY; |
| FLKLIEIQNSPYYS; | LKLIEIQNSPYYSQ; | KLIEIQNSPYYSQE; |
| LIEIQNSPYYSQEK; | IEIQNSPYYSQEKK; | EIQNSPYYSQEKKD; |
| IQNSPYYSQEKKDI; | QNSPYYSQEKKDIY; | NSPYYSQEKKDIYK; |
| SPYYSQEKKDIYKQ; | PYYSQEKKDIYKQI; | YYSQEKKDIYKQII; |
| YSQEKKDIYKQIIL; | SQEKKDIYKQIILN; | QEKKDIYKQIILNF; |
| EKKDIYKQIILNFS; | KKDIYKQIILNFSS; | KDIYKQIILNFSSN; |
| DIYKQIILNFSSNI; | IYKQIILNFSSNIN; | YKQIILNFSSNINI; |
| KQIILNFSSNINID; | QIILNFSSNINIDS; | IILNFSSNINIDSL; |
| ILNFSSNINIDSLE; | LNFSSNINIDSLEQ; | NFSSNINIDSLEQT; |
| FSSNINIDSLEQTI; | SSNINIDSLEQTID; | SNINIDSLEQTIDI; |
| NINIDSLEQTIDIL; | INIDSLEQTIDILV; | NIDSLEQTIDILVA; |
| IDSLEQTIDILVAV; | DSLEQTIDILVAVR; | SLEQTIDILVAVRN; |
| LEQTIDILVAVRNR; | EQTIDILVAVRNRN; | QTIDILVAVRNRNK; |

| | | |
|---|---|---|
| TIDILVAVRNRNKI; | IDILVAVRNRNKIK; | DILVAVRNRNKIKI; |
| ILVAVRNRNKIKIL; | LVAVRNRNKIKILN; | VAVRNRNKIKILNI; |
| AVRNRNKIKILNIL; | VRNRNKIKILNILN; | RNRNKIKILNILNK; |
| NRNKIKILNILNKN; | RNKIKILNILNKNL; | NKIKILNILNKNLK; |
| KIKILNILNKNLKY; | IKILNILNKNLKYR; | KILNILNKNLKYRP; |
| ILNILNKNLKYRPE; | LNILNKNLKYRPEN; | NILNKNLKYRPENQ; |
| ILNKNLKYRPENQK; | LNKNLKYRPENQKV; | NKNLKYRPENQKVF; |
| KNLKYRPENQKVFK; | NLKYRPENQKVFKS; | LKYRPENQKVFKSS; |
| KYRPENQKVFKSSL; | YRPENQKVFKSSLT; | RPENQKVFKSSLTN; |
| PENQKVFKSSLTNK; | ENQKVFKSSLTNKE; | NQKVFKSSLTNKES; |
| QKVFKSSLTNKESR; | KVFKSSLTNKESRL; | VFKSSLTNKESRLI; |
| FKSSLTNKESRLIK; | KSSLTNKESRLIKL; | SSLTNKESRLIKLK; |
| SLTNKESRLIKLKR; | LTNKESRLIKLKRM; | TNKESRLIKLKRML; |
| NKESRLIKLKRMLI; | KESRLIKLKRMLIL; | ESRLIKLKRMLILT; |
| SRLIKLKRMLILTY; | RLIKLKRMLILTYW; | LIKLKRMLILTYWP; |
| IKLKRMLILTYWPV; | KLKRMLILTYWPVG; | LKRMLILTYWPVGC; |
| KRMLILTYWPVGCL; | RMLILTYWPVGCLS; | MLILTYWPVGCLSK; |
| LILTYWPVGCLSKN; | ILTYWPVGCLSKNI; | LTYWPVGCLSKNIF; |
| TYWPVGCLSKNIFI; | YWPVGCLSKNIFIK; | WPVGCLSKNIFIKI; |
| PVGCLSKNIFIKIL; | VGCLSKNIFIKILI; | GCLSKNIFIKILIK; |
| CLSKNIFIKILIKX; | LSKNIFIKILIKXY; | SKNIFIKILIKXYK; |
| KNIFIKILIKXYKY; | NIFIKILIKXYKYL; | IFIKILIKXYKYLE; |
| FIKILIKXYKYLEE; | IKILIKXYKYLEEE; | KILIKXYKYLEEEI; |
| ILIKXYKYLEEEIL; | LIKXYKYLEEEILA; | IKXYKYLEEEILAL; |
| KXYKYLEEEILALK; | XYKYLEEEILALKY; | YKYLEEEILALKYN; |
| KYLEEEILALKYNE; | YLEEEILALKYNEI; | LEEEILALKYNEIL; |
| EEEILALKYNEILN; | EEILALKYNEILNY; | EILALKYNEILNYL; |
| ILALKYNEILNYLR; | LALKYNEILNYLRA; | ALKYNEILNYLRAL; |
| LKYNEILNYLRALK; | KYNEILNYLRALKT; | YNEILNYLRALKTL; |
| NEILNYLRALKTLS; | EILNYLRALKTLSL; | ILNYLRALKTLSLN; |
| LNYLRALKTLSLNE; | NYLRALKTLSLNEI; | YLRALKTLSLNEIF; |
| LRALKTLSLNEIFY; | RALKTLSLNEIFYK; | ALKTLSLNEIFYKG; |
| LKTLSLNEIFYKGS; | KTLSLNEIFYKGSS; | TLSLNEIFYKGSSK; |
| LSLNEIFYKGSSKN; | SLNEIFYKGSSKNI; | LNEIFYKGSSKNIS; |
| NEIFYKGSSKNISF; | EIFYKGSSKNISFN; | IFYKGSSKNISFNY; |
| FYKGSSKNISFNYF; | YKGSSKNISFNYFF; | KGSSKNISFNYFFS; |
| GSSKNISFNYFFSD; | SSKNISFNYFFSDF; | SKNISFNYFFSDFT; |
| KNISFNYFFSDFTQ; | NISFNYFFSDFTQY; | ISFNYFFSDFTQYA; |
| SFNYFFSDFTQYAP; | FNYFFSDFTQYAPK; | NYFFSDFTQYAPKD; |
| YFFSDFTQYAPKDF; | FFSDFTQYAPKDFQ; | FSDFTQYAPKDFQD; |
| SDFTQYAPKDFQDT; | DFTQYAPKDFQDTL; | FTQYAPKDFQDTLK; |
| TQYAPKDFQDTLKC; | QYAPKDFQDTLKCY; | YAPKDFQDTLKCYL; |
| APKDFQDTLKCYLY; | PKDFQDTLKCYLYV; | KDFQDTLKCYLYVI; |
| DFQDTLKCYLYVIE; | FQDTLKCYLYVIEK; | QDTLKCYLYVIEKT; |
| DTLKCYLYVIEKTI; | TLKCYLYVIEKTIT; | LKCYLYVIEKTITK; |
| KCYLYVIEKTITKK; | CYLYVIEKTITKKY; | YLYVIEKTITKKYL; |
| LYVIEKTITKKYLT; | YVIEKTITKKYLTW; | VIEKTITKKYLTWF; |
| IEKTITKKYLTWFF; | EKTITKKYLTWFFK; | KTITKKYLTWFFKD; |
| TITKKYLTWFFKDK; | ITKKYLTWFFKDKI; | TKKYLTWFFKDKIS; |
| KKYLTWFFKDKISN; | KYLTWFFKDKISNN; | YLTWFFKDKISNNW; |
| LTWFFKDKISNNWE; | TWFFKDKISNNWES; | WFFKDKISNNWESF; |

| | | |
|---|---|---|
| FFKDKISNNWESFI; | FKDKISNNWESFID; | KDKISNNWESFIDA; |
| DKISNNWESFIDAL; | KISNNWESFIDALE; | ISNNWESFIDALEY; |
| SNNWESFIDALEYI; | NNWESFIDALEYIE; | NWESFIDALEYIEK; |
| WESFIDALEYIEKH; | ESFIDALEYIEKHK; | SFIDALEYIEKHKL; |
| FIDALEYIEKHKLI; | IDALEYIEKHKLIN; | DALEYIEKHKLINI; |
| ALEYIEKHKLINIK; | LEYIEKHKLINIKE; | EYIEKHKLINIKEK; |
| YIEKHKLINIKEKI; | IEKHKLINIKEKIK; | EKHKLINIKEKIKE; |
| KHKLINIKEKIKEI; | HKLINIKEKIKEII; | KLINIKEKIKEIIT; |
| LINIKEKIKEIITA; | INIKEKIKEIITAK; | NIKEKIKEIITAKF; |
| IKEKIKEIITAKFQ; | KEKIKEIITAKFQT; | EKIKEIITAKFQTE; |
| KIKEIITAKFQTED; | IKEIITAKFQTEDF; | KEIITAKFQTEDFF; |
| EIITAKFQTEDFFI; | IITAKFQTEDFFIS; | ITAKFQTEDFFISF; |
| TAKFQTEDFFISFD; | AKFQTEDFFISFDK; | KFQTEDFFISFDKT; |
| FQTEDFFISFDKTN; | QTEDFFISFDKTNF; | TEDFFISFDKTNFN; |
| EDFFISFDKTNFNP; | DFFISFDKTNFNPY; | FFISFDKTNFNPYE; |
| FISFDKTNFNPYES; | ISFDKTNFNPYESS; | SFDKTNFNPYESSI; |
| FDKTNFNPYESSIF; | DKTNFNPYESSIFK; | KTNFNPYESSIFKE; |
| TNFNPYESSIFKER; | NFNPYESSIFKERY; | FNPYESSIFKERYI; |
| NPYESSIFKERYIK; | PYESSIFKERYIKS; | YESSIFKERYIKSA; |
| ESSIFKERYIKSAF; | SSIFKERYIKSAFL; | SIFKERYIKSAFLE; |
| IFKERYIKSAFLEI; | FKERYIKSAFLEII; | KERYIKSAFLEIIM; |
| ERYIKSAFLEIIMH; | RYIKSAFLEIIMHY; | YIKSAFLEIIMHYV; |
| IKSAFLEIIMHYVK; | KSAFLEIIMHYVKK; | SAFLEIIMHYVKKN; |
| AFLEIIMHYVKKNS; | FLEIIMHYVKKNSQ; | LEIIMHYVKKNSQN; |
| EIIMHYVKKNSQNI; | IIMHYVKKNSQNIE; | IMHYVKKNSQNIET; |
| MHYVKKNSQNIETY; | HYVKKNSQNIETYG; | YVKKNSQNIETYGW; |
| VKKNSQNIETYGWI; | KKNSQNIETYGWIA; | KNSQNIETYGWIAF; |
| NSQNIETYGWIAFY; | SQNIETYGWIAFYI; | QNIETYGWIAFYIY; |
| NIETYGWIAFYIYA; | IETYGWIAFYIYAD; | ETYGWIAFYIYADN; |
| TYGWIAFYIYADNK; | YGWIAFYIYADNKD; | GWIAFYIYADNKDK; |
| WIAFYIYADNKDKK; | IAFYIYADNKDKKI; | AFYIYADNKDKKIF; |
| FYIYADNKDKKIFC; | YIYADNKDKKIFCQ; | IYADNKDKKIFCQR; |
| YADNKDKKIFCQRM; | ADNKDKKIFCQRMK; | DNKDKKIFCQRMKE; |
| NKDKKIFCQRMKEF; | KDKKIFCQRMKEFF; | DKKIFCQRMKEFFK; |
| KKIFCQRMKEFFKS; | KIFCQRMKEFFKSK; | IFCQRMKEFFKSKT; |
| FCQRMKEFFKSKTF; | CQRMKEFFKSKTFE; | QRMKEFFKSKTFEI; |
| RMKEFFKSKTFEIQ; | MKEFFKSKTFEIQN; | KEFFKSKTFEIQNQ; |
| EFFKSKTFEIQNQI; | FFKSKTFEIQNQIF; | FKSKTFEIQNQIFV; |
| KSKTFEIQNQIFVY; | SKTFEIQNQIFVYF; | KTFEIQNQIFVYFL; |
| TFEIQNQIFVYFLS; | FEIQNQIFVYFLSF; | EIQNQIFVYFLSFY; |
| IQNQIFVYFLSFYP; | QNQIFVYFLSFYPN; | NQIFVYFLSFYPNI; |
| QIFVYFLSFYPNIE; | IFVYFLSFYPNIEY; | FVYFLSFYPNIEYK; |
| VYFLSFYPNIEYKH; | YFLSFYPNIEYKHF; | FLSFYPNIEYKHFK; |
| LSFYPNIEYKHFKF; | SFYPNIEYKHFKFI; | FYPNIEYKHFKFIS; |
| YPNIEYKHFKFISD; | PNIEYKHFKFISDI; | NIEYKHFKFISDIL; |
| IEYKHFKFISDILL; | EYKHFKFISDILLY; | YKHFKFISDILLYL; |
| KHFKFISDILLYLD; | HFKFISDILLYLDE; | FKFISDILLYLDEN; |
| KFISDILLYLDENK; | FISDILLYLDENKI; | ISDILLYLDENKIT; |
| SDILLYLDENKITI; | DILLYLDENKITIP; | ILLYLDENKITIPK; |
| LLYLDENKITIPKK; | LYLDENKITIPKKI; | YLDENKITIPKKII; |
| LDENKITIPKKIIK; | DENKITIPKKIIKI; | ENKITIPKKIIKIQ; |

| | NKITIPKKIIKIQK; KITIPKKIIKIQKI; ITIPKKIIKIQKIN;<br>TIPKKIIKIQKINP; IPKKIIKIQKINPN; PKKIIKIQKINPNQ;<br>KKIIKIQKINPNQL; KIIKIQKINPNQLD; IIKIQKINPNQLDY;<br>IKIQKINPNQLDYQ; KIQKINPNQLDYQK; IQKINPNQLDYQKS;<br>QKINPNQLDYQKSY; KINPNQLDYQKSYL; INPNQLDYQKSYLL;<br>NPNQLDYQKSYLLI; PNQLDYQKSYLLIK; NQLDYQKSYLLIKS;<br>QLDYQKSYLLIKSL; LDYQKSYLLIKSLK; DYQKSYLLIKSLKT;<br>YQKSYLLIKSLKTR; QKSYLLIKSLKTRS; KSYLLIKSLKTRSR;<br>SYLLIKSLKTRSRI; YLLIKSLKTRSRIL; LLIKSLKTRSRILK;<br>LIKSLKTRSRILKI; IKSLKTRSRILKII; KSLKTRSRILKIII;<br>SLKTRSRILKIIIK; LKTRSRILKIIIKN; KTRSRILKIIIKNI;<br>TRSRILKIIIKNIR; RSRILKIIIKNIRF; SRILKIIIKNIRFN;<br>RILKIIIKNIRFNL; ILKIIIKNIRFNLI; LKIIIKNIRFNLIE;<br>KIIIKNIRFNLIEK; IIIKNIRFNLIEKL; IIKNIRFNLIEKLE;<br>IKNIRFNLIEKLED; KNIRFNLIEKLEDE; NIRFNLIEKLEDEN;<br>IRFNLIEKLEDENE; RFNLIEKLEDENEK; FNLIEKLEDENEKK;<br>NLIEKLEDENEKKL; LIEKLEDENEKKLL; IEKLEDENEKKLLP;<br>EKLEDENEKKLLPA; KLEDENEKKLLPAM; LEDENEKKLLPAMC;<br>EDENEKKLLPAMCY; DENEKKLLPAMCYL; ENEKKLLPAMCYLI;<br>NEKKLLPAMCYLIY; EKKLLPAMCYLIYC; KKLLPAMCYLIYCE;<br>KLLPAMCYLIYCEN; LLPAMCYLIYCENL; LPAMCYLIYCENLV;<br>PAMCYLIYCENLVE; AMCYLIYCENLVEL; MCYLIYCENLVELK;<br>CYLIYCENLVELKN; YLIYCENLVELKNN; LIYCENLVELKNNR;<br>IYCENLVELKNNRK; YCENLVELKNNRKI; CENLVELKNNRKIK;<br>ENLVELKNNRKIKS; NLVELKNNRKIKSN; LVELKNNRKIKSNE;<br>VELKNNRKIKSNEK; ELKNNRKIKSNEKN; LKNNRKIKSNEKNS;<br>KNNRKIKSNEKNSL; NNRKIKSNEKNSLL; NRKIKSNEKNSLLE;<br>RKIKSNEKNSLLEF; KIKSNEKNSLLEFI; IKSNEKNSLLEFIS;<br>KSNEKNSLLEFISF; SNEKNSLLEFISFF; NEKNSLLEFISFFK;<br>EKNSLLEFISFFKT; KNSLLEFISFFKTK; NSLLEFISFFKTKL;<br>SLLEFISFFKTKLK; LLEFISFFKTKLKQ; LEFISFFKTKLKQK;<br>EFISFFKTKLKQKI; FISFFKTKLKQKIN; ISFFKTKLKQKINF;<br>SFFKTKLKQKINFK; FFKTKLKQKINFKK; FKTKLKQKINFKKE;<br>KTKLKQKINFKKEL; TKLKQKINFKKELM; KLKQKINFKKELMK;<br>LKQKINFKKELMKS; KQKINFKKELMKSK; QKINFKKELMKSKG;<br>KINFKKELMKSKGI;<br><br>15 mers:<br>MKTIKKDSEFYDSLA; KTIKKDSEFYDSLAT; TIKKDSEFYDSLATL;<br>IKKDSEFYDSLATLK; KKDSEFYDSLATLKK; KDSEFYDSLATLKKH;<br>DSEFYDSLATLKKHI; SEFYDSLATLKKHIN; EFYDSLATLKKHINK;<br>FYDSLATLKKHINKY; YDSLATLKKHINKYI; DSLATLKKHINKYIE;<br>SLATLKKHINKYIEE; LATLKKHINKYIEEK; ATLKKHINKYIEEKV;<br>TLKKHINKYIEEKVL; LKKHINKYIEEKVLK; KKHINKYIEEKVLKY;<br>KHINKYIEEKVLKYS; HINKYIEEKVLKYSI; INKYIEEKVLKYSIS;<br>NKYIEEKVLKYSISS; KYIEEKVLKYSISSQ; YIEEKVLKYSISSQL;<br>IEEKVLKYSISSQLY; EEKVLKYSISSQLYK; EKVLKYSISSQLYKL;<br>KVLKYSISSQLYKLE; VLKYSISSQLYKLEK; LKYSISSQLYKLEKP;<br>KYSISSQLYKLEKPE; YSISSQLYKLEKPEI; SISSQLYKLEKPEII;<br>ISSQLYKLEKPEIIE; SSQLYKLEKPEIIEL; SQLYKLEKPEIIELI;<br>QLYKLEKPEIIELIK; LYKLEKPEIIELIKI; YKLEKPEIIELIKIS; |
|---|---|

KLEKPEIIELIKISN; LEKPEIIELIKISND; EKPEIIELIKISNDY;
KPEIIELIKISNDYE; PEIIELIKISNDYEK; EIIELIKISNDYEKE;
IIELIKISNDYEKEK; IELIKISNDYEKEKN; ELIKISNDYEKEKNA;
LIKISNDYEKEKNAF; IKISNDYEKEKNAFN; KISNDYEKEKNAFNT;
ISNDYEKEKNAFNTS; SNDYEKEKNAFNTSL; NDYEKEKNAFNTSLE;
DYEKEKNAFNTSLEE; YEKEKNAFNTSLEEC; EKEKNAFNTSLEECY;
KEKNAFNTSLEECYY; EKNAFNTSLEECYYK; KNAFNTSLEECYYKN;
NAFNTSLEECYYKNT; AFNTSLEECYYKNTQ; FNTSLEECYYKNTQN;
NTSLEECYYKNTQNE; TSLEECYYKNTQNEA; SLEECYYKNTQNEAI;
LEECYYKNTQNEAIK; EECYYKNTQNEAIKK; ECYYKNTQNEAIKKW;
CYYKNTQNEAIKKWI; YYKNTQNEAIKKWIL; YKNTQNEAIKKWILE;
KNTQNEAIKKWILEI; NTQNEAIKKWILEIV; TQNEAIKKWILEIVR;
QNEAIKKWILEIVRN; NEAIKKWILEIVRNK; EAIKKWILEIVRNKN;
AIKKWILEIVRNKNF; IKKWILEIVRNKNFI; KKWILEIVRNKNFIQ;
KWILEIVRNKNFIQI; WILEIVRNKNFIQIS; ILEIVRNKNFIQISK;
LEIVRNKNFIQISKE; EIVRNKNFIQISKEA; IVRNKNFIQISKEAN;
VRNKNFIQISKEANL; RNKNFIQISKEANLN; NKNFIQISKEANLNT;
KNFIQISKEANLNTK; NFIQISKEANLNTKK; FIQISKEANLNTKKL;
IQISKEANLNTKKLG; QISKEANLNTKKLGT; ISKEANLNTKKLGTT;
SKEANLNTKKLGTTY; KEANLNTKKLGTTYK; EANLNTKKLGTTYKL;
ANLNTKKLGTTYKLK; NLNTKKLGTTYKLKS; LNTKKLGTTYKLKSS;
NTKKLGTTYKLKSSN; TKKLGTTYKLKSSNF; KKLGTTYKLKSSNFL;
KLGTTYKLKSSNFLK; LGTTYKLKSSNFLKL; GTTYKLKSSNFLKLI;
TTYKLKSSNFLKLIE; TYKLKSSNFLKLIEI; YKLKSSNFLKLIEIQ;
KLKSSNFLKLIEIQN; LKSSNFLKLIEIQNS; KSSNFLKLIEIQNSP;
SSNFLKLIEIQNSPY; SNFLKLIEIQNSPYY; NFLKLIEIQNSPYYS;
FLKLIEIQNSPYYSQ; LKLIEIQNSPYYSQE; KLIEIQNSPYYSQEK;
LIEIQNSPYYSQEKK; IEIQNSPYYSQEKKD; EIQNSPYYSQEKKDI;
IQNSPYYSQEKKDIY; QNSPYYSQEKKDIYK; NSPYYSQEKKDIYKQ;
SPYYSQEKKDIYKQI; PYYSQEKKDIYKQII; YYSQEKKDIYKQIIL;
YSQEKKDIYKQIILN; SQEKKDIYKQIILNF; QEKKDIYKQIILNFS;
EKKDIYKQIILNFSS; KKDIYKQIILNFSSN; KDIYKQIILNFSSNI;
DIYKQIILNFSSNIN; IYKQIILNFSSNINI; YKQIILNFSSNINID;
KQIILNFSSNINIDS; QIILNFSSNINIDSL; IILNFSSNINIDSLE;
ILNFSSNINIDSLEQ; LNFSSNINIDSLEQT; NFSSNINIDSLEQTI;
FSSNINIDSLEQTID; SSNINIDSLEQTIDI; SNINIDSLEQTIDIL;
NINIDSLEQTIDILV; INIDSLEQTIDILVA; NIDSLEQTIDILVAV;
IDSLEQTIDILVAVR; DSLEQTIDILVAVRN; SLEQTIDILVAVRNR;
LEQTIDILVAVRNRN; EQTIDILVAVRNRNK; QTIDILVAVRNRNKI;
TIDILVAVRNRNKIK; IDILVAVRNRNKIKI; DILVAVRNRNKIKIL;
ILVAVRNRNKIKILN; LVAVRNRNKIKILNI; VAVRNRNKIKILNIL;
AVRNRNKIKILNILN; VRNRNKIKILNILNK; RNRNKIKILNILNKN;
NRNKIKILNILNKNL; RNKIKILNILNKNLK; NKIKILNILNKNLKY;
KIKILNILNKNLKYR; IKILNILNKNLKYRP; KILNILNKNLKYRPE;
ILNILNKNLKYRPEN; LNILNKNLKYRPENQ; NILNKNLKYRPENQK;
ILNKNLKYRPENQKV; LNKNLKYRPENQKVF; NKNLKYRPENQKVFK;
KNLKYRPENQKVFKS; NLKYRPENQKVFKSS; LKYRPENQKVFKSSL;
KYRPENQKVFKSSLT; YRPENQKVFKSSLTN; RPENQKVFKSSLTNK;
PENQKVFKSSLTNKE; ENQKVFKSSLTNKES; NQKVFKSSLTNKESR;
QKVFKSSLTNKESRL; KVFKSSLTNKESRLI; VFKSSLTNKESRLIK;
FKSSLTNKESRLIKL; KSSLTNKESRLIKLK; SSLTNKESRLIKLKR;

SLTNKESRLIKLKRM; LTNKESRLIKLKRML; TNKESRLIKLKRMLI;
NKESRLIKLKRMLIL; KESRLIKLKRMLILT; ESRLIKLKRMLILTY;
SRLIKLKRMLILTYW; RLIKLKRMLILTYWP; LIKLKRMLILTYWPV;
IKLKRMLILTYWPVG; KLKRMLILTYWPVGC; LKRMLILTYWPVGCL;
KRMLILTYWPVGCLS; RMLILTYWPVGCLSK; MLILTYWPVGCLSKN;
LILTYWPVGCLSKNI; ILTYWPVGCLSKNIF; LTYWPVGCLSKNIFI;
TYWPVGCLSKNIFIK; YWPVGCLSKNIFIKI; WPVGCLSKNIFIKIL;
PVGCLSKNIFIKILI; VGCLSKNIFIKILIK; GCLSKNIFIKILIKX;
CLSKNIFIKILIKXY; LSKNIFIKILIKXYK; SKNIFIKILIKXYKY;
KNIFIKILIKXYKYL; NIFIKILIKXYKYLE; IFIKILIKXYKYLEE;
FIKILIKXYKYLEEE; IKILIKXYKYLEEEI; KILIKXYKYLEEEIL;
ILIKXYKYLEEEILA; LIKXYKYLEEEILAL; IKXYKYLEEEILALK;
KXYKYLEEEILALKY; XYKYLEEEILALKYN; YKYLEEEILALKYNE;
KYLEEEILALKYNEI; YLEEEILALKYNEIL; LEEEILALKYNEILN;
EEEILALKYNEILNY; EEILALKYNEILNYL; EILALKYNEILNYLR;
ILALKYNEILNYLRA; LALKYNEILNYLRAL; ALKYNEILNYLRALK;
LKYNEILNYLRALKT; KYNEILNYLRALKTL; YNEILNYLRALKTLS;
NEILNYLRALKTLSL; EILNYLRALKTLSLN; ILNYLRALKTLSLNE;
LNYLRALKTLSLNEI; NYLRALKTLSLNEIF; YLRALKTLSLNEIFY;
LRALKTLSLNEIFYK; RALKTLSLNEIFYKG; ALKTLSLNEIFYKGS;
LKTLSLNEIFYKGSS; KTLSLNEIFYKGSSK; TLSLNEIFYKGSSKN;
LSLNEIFYKGSSKNI; SLNEIFYKGSSKNIS; LNEIFYKGSSKNISF;
NEIFYKGSSKNISFN; EIFYKGSSKNISFNY; IFYKGSSKNISFNYF;
FYKGSSKNISFNYFF; YKGSSKNISFNYFFS; KGSSKNISFNYFFSD;
GSSKNISFNYFFSDF; SSKNISFNYFFSDFT; SKNISFNYFFSDFTQ;
KNISFNYFFSDFTQY; NISFNYFFSDFTQYA; ISFNYFFSDFTQYAP;
SFNYFFSDFTQYAPK; FNYFFSDFTQYAPKD; NYFFSDFTQYAPKDF;
YFFSDFTQYAPKDFQ; FFSDFTQYAPKDFQD; FSDFTQYAPKDFQDT;
SDFTQYAPKDFQDTL; DFTQYAPKDFQDTLK; FTQYAPKDFQDTLKC;
TQYAPKDFQDTLKCY; QYAPKDFQDTLKCYL; YAPKDFQDTLKCYLY;
APKDFQDTLKCYLYV; PKDFQDTLKCYLYVI; KDFQDTLKCYLYVIE;
DFQDTLKCYLYVIEK; FQDTLKCYLYVIEKT; QDTLKCYLYVIEKTI;
DTLKCYLYVIEKTIT; TLKCYLYVIEKTITK; LKCYLYVIEKTITKK;
KCYLYVIEKTITKKY; CYLYVIEKTITKKYL; YLYVIEKTITKKYLT;
LYVIEKTITKKYLTW; YVIEKTITKKYLTWF; VIEKTITKKYLTWFF;
IEKTITKKYLTWFFK; EKTITKKYLTWFFKD; KTITKKYLTWFFKDK;
TITKKYLTWFFKDKI; ITKKYLTWFFKDKIS; TKKYLTWFFKDKISN;
KKYLTWFFKDKISNN; KYLTWFFKDKISNNW; YLTWFFKDKISNNWE;
LTWFFKDKISNNWES; TWFFKDKISNNWESF; WFFKDKISNNWESFI;
FFKDKISNNWESFID; FKDKISNNWESFIDA; KDKISNNWESFIDAL;
DKISNNWESFIDALE; KISNNWESFIDALEY; ISNNWESFIDALEYI;
SNNWESFIDALEYIE; NNWESFIDALEYIEK; NWESFIDALEYIEKH;
WESFIDALEYIEKHK; ESFIDALEYIEKHKL; SFIDALEYIEKHKLI;
FIDALEYIEKHKLIN; IDALEYIEKHKLINI; DALEYIEKHKLINIK;
ALEYIEKHKLINIKE; LEYIEKHKLINIKEK; EYIEKHKLINIKEKI;
YIEKHKLINIKEKIK; IEKHKLINIKEKIKE; EKHKLINIKEKIKEI;
KHKLINIKEKIKEII; HKLINIKEKIKEIIT; KLINIKEKIKEIITA;
LINIKEKIKEIITAK; INIKEKIKEIITAKF; NIKEKIKEIITAKFQ;
IKEKIKEIITAKFQT; KEKIKEIITAKFQTE; EKIKEIITAKFQTED;
KIKEIITAKFQTEDF; IKEIITAKFQTEDFF; KEIITAKFQTEDFFI;
EIITAKFQTEDFFIS; IITAKFQTEDFFISF; ITAKFQTEDFFISFD;

| | |
|---|---|
| TAKFQTEDFFISFDK; AKFQTEDFFISFDKT; KFQTEDFFISFDKTN; | |
| FQTEDFFISFDKTNF; QTEDFFISFDKTNFN; TEDFFISFDKTNFNP; | |
| EDFFISFDKTNFNPY; DFFISFDKTNFNPYE; FFISFDKTNFNPYES; | |
| FISFDKTNFNPYESS; ISFDKTNFNPYESSI; SFDKTNFNPYESSIF; | |
| FDKTNFNPYESSIFK; DKTNFNPYESSIFKE; KTNFNPYESSIFKER; | |
| TNFNPYESSIFKERY; NFNPYESSIFKERYI; FNPYESSIFKERYIK; | |
| NPYESSIFKERYIKS; PYESSIFKERYIKSA; YESSIFKERYIKSAF; | |
| ESSIFKERYIKSAFL; SSIFKERYIKSAFLE; SIFKERYIKSAFLEI; | |
| IFKERYIKSAFLEII; FKERYIKSAFLEIIM; KERYIKSAFLEIIMH; | |
| ERYIKSAFLEIIMHY; RYIKSAFLEIIMHYV; YIKSAFLEIIMHYVK; | |
| IKSAFLEIIMHYVKK; KSAFLEIIMHYVKKN; SAFLEIIMHYVKKNS; | |
| AFLEIIMHYVKKNSQ; FLEIIMHYVKKNSQN; LEIIMHYVKKNSQNI; | |
| EIIMHYVKKNSQNIE; IIMHYVKKNSQNIET; IMHYVKKNSQNIETY; | |
| MHYVKKNSQNIETYG; HYVKKNSQNIETYGW; YVKKNSQNIETYGWI; | |
| VKKNSQNIETYGWIA; KKNSQNIETYGWIAF; KNSQNIETYGWIAFY; | |
| NSQNIETYGWIAFYI; SQNIETYGWIAFYIY; QNIETYGWIAFYIYA; | |
| NIETYGWIAFYIYAD; IETYGWIAFYIYADN; ETYGWIAFYIYADNK; | |
| TYGWIAFYIYADNKD; YGWIAFYIYADNKDK; GWIAFYIYADNKDKK; | |
| WIAFYIYADNKDKKI; IAFYIYADNKDKKIF; AFYIYADNKDKKIFC; | |
| FYIYADNKDKKIFCQ; YIYADNKDKKIFCQR; IYADNKDKKIFCQRM; | |
| YADNKDKKIFCQRMK; ADNKDKKIFCQRMKE; DNKDKKIFCQRMKEF; | |
| NKDKKIFCQRMKEFF; KDKKIFCQRMKEFFK; DKKIFCQRMKEFFKS; | |
| KKIFCQRMKEFFKSK; KIFCQRMKEFFKSKT; IFCQRMKEFFKSKTF; | |
| FCQRMKEFFKSKTFE; CQRMKEFFKSKTFEI; QRMKEFFKSKTFEIQ; | |
| RMKEFFKSKTFEIQN; MKEFFKSKTFEIQNQ; KEFFKSKTFEIQNQI; | |
| EFFKSKTFEIQNQIF; FFKSKTFEIQNQIFV; FKSKTFEIQNQIFVY; | |
| KSKTFEIQNQIFVYF; SKTFEIQNQIFVYFL; KTFEIQNQIFVYFLS; | |
| TFEIQNQIFVYFLSF; FEIQNQIFVYFLSFY; EIQNQIFVYFLSFYP; | |
| IQNQIFVYFLSFYPN; QNQIFVYFLSFYPNI; NQIFVYFLSFYPNIE; | |
| QIFVYFLSFYPNIEY; IFVYFLSFYPNIEYK; FVYFLSFYPNIEYKH; | |
| VYFLSFYPNIEYKHF; YFLSFYPNIEYKHFK; FLSFYPNIEYKHFKF; | |
| LSFYPNIEYKHFKFI; SFYPNIEYKHFKFIS; FYPNIEYKHFKFISD; | |
| YPNIEYKHFKFISDI; PNIEYKHFKFISDIL; NIEYKHFKFISDILL; | |
| IEYKHFKFISDILLY; EYKHFKFISDILLYL; YKHFKFISDILLYLD; | |
| KHFKFISDILLYLDE; HFKFISDILLYLDEN; FKFISDILLYLDENK; | |
| KFISDILLYLDENKI; FISDILLYLDENKIT; ISDILLYLDENKITI; | |
| SDILLYLDENKITIP; DILLYLDENKITIPK; ILLYLDENKITIPKK; | |
| LLYLDENKITIPKKI; LYLDENKITIPKKII; YLDENKITIPKKIIK; | |
| LDENKITIPKKIIKI; DENKITIPKKIIKIQ; ENKITIPKKIIKIQK; | |
| NKITIPKKIIKIQKI; KITIPKKIIKIQKIN; ITIPKKIIKIQKINP; | |
| TIPKKIIKIQKINPN; IPKKIIKIQKINPNQ; PKKIIKIQKINPNQL; | |
| KKIIKIQKINPNQLD; KIIKIQKINPNQLDY; IIKIQKINPNQLDYQ; | |
| IKIQKINPNQLDYQK; KIQKINPNQLDYQKS; IQKINPNQLDYQKSY; | |
| QKINPNQLDYQKSYL; KINPNQLDYQKSYLL; INPNQLDYQKSYLLI; | |
| NPNQLDYQKSYLLIK; PNQLDYQKSYLLIKS; NQLDYQKSYLLIKSL; | |
| QLDYQKSYLLIKSLK; LDYQKSYLLIKSLKT; DYQKSYLLIKSLKTR; | |
| YQKSYLLIKSLKTRS; QKSYLLIKSLKTRSR; KSYLLIKSLKTRSRI; | |
| SYLLIKSLKTRSRIL; YLLIKSLKTRSRILK; LLIKSLKTRSRILKI; | |
| LIKSLKTRSRILKII; IKSLKTRSRILKIII; KSLKTRSRILKIIIK; | |
| SLKTRSRILKIIIKN; LKTRSRILKIIIKNI; KTRSRILKIIIKNIR; | |
| TRSRILKIIIKNIRF; RSRILKIIIKNIRFN; SRILKIIIKNIRFNL; | |

RILKIIIKNIRFNLI; ILKIIIKNIRFNLIE; LKIIIKNIRFNLIEK;
KIIIKNIRFNLIEKL; IIIKNIRFNLIEKLE; IIKNIRFNLIEKLED;
IKNIRFNLIEKLEDE; KNIRFNLIEKLEDEN; NIRFNLIEKLEDENE;
IRFNLIEKLEDENEK; RFNLIEKLEDENEKK; FNLIEKLEDENEKKL;
NLIEKLEDENEKKLL; LIEKLEDENEKKLLP; IEKLEDENEKKLLPA;
EKLEDENEKKLLPAM; KLEDENEKKLLPAMC; LEDENEKKLLPAMCY;
EDENEKKLLPAMCYL; DENEKKLLPAMCYLI; ENEKKLLPAMCYLIY;
NEKKLLPAMCYLIYC; EKKLLPAMCYLIYCE; KKLLPAMCYLIYCEN;
KLLPAMCYLIYCENL; LLPAMCYLIYCENLV; LPAMCYLIYCENLVE;
PAMCYLIYCENLVEL; AMCYLIYCENLVELK; MCYLIYCENLVELKN;
CYLIYCENLVELKNN; YLIYCENLVELKNNR; LIYCENLVELKNNRK;
IYCENLVELKNNRKI; YCENLVELKNNRKIK; CENLVELKNNRKIKS;
ENLVELKNNRKIKSN; NLVELKNNRKIKSNE; LVELKNNRKIKSNEK;
VELKNNRKIKSNEKN; ELKNNRKIKSNEKNS; LKNNRKIKSNEKNSL;
KNNRKIKSNEKNSLL; NNRKIKSNEKNSLLE; NRKIKSNEKNSLLEF;
RKIKSNEKNSLLEFI; KIKSNEKNSLLEFIS; IKSNEKNSLLEFISF;
KSNEKNSLLEFISFF; SNEKNSLLEFISFFK; NEKNSLLEFISFFKT;
EKNSLLEFISFFKTK; KNSLLEFISFFKTKL; NSLLEFISFFKTKLK;
SLLEFISFFKTKLKQ; LLEFISFFKTKLKQK; LEFISFFKTKLKQKI;
EFISFFKTKLKQKIN; FISFFKTKLKQKINF; ISFFKTKLKQKINFK;
SFFKTKLKQKINFKK; FFKTKLKQKINFKKE; FKTKLKQKINFKKEL;
KTKLKQKINFKKELM; TKLKQKINFKKELMK; KLKQKINFKKELMKS;
LKQKINFKKELMKSK; KQKINFKKELMKSKG; QKINFKKELMKSKGI;

16 mers:
MKTIKKDSEFYDSLAT; KTIKKDSEFYDSLATL; TIKKDSEFYDSLATLK;
IKKDSEFYDSLATLKK; KKDSEFYDSLATLKKH; KDSEFYDSLATLKKHI;
DSEFYDSLATLKKHIN; SEFYDSLATLKKHINK; EFYDSLATLKKHINKY;
FYDSLATLKKHINKYI; YDSLATLKKHINKYIE; DSLATLKKHINKYIEE;
SLATLKKHINKYIEEK; LATLKKHINKYIEEKV; ATLKKHINKYIEEKVL;
TLKKHINKYIEEKVLK; LKKHINKYIEEKVLKY; KKHINKYIEEKVLKYS;
KHINKYIEEKVLKYSI; HINKYIEEKVLKYSIS; INKYIEEKVLKYSISS;
NKYIEEKVLKYSISSQ; KYIEEKVLKYSISSQL; YIEEKVLKYSISSQLY;
IEEKVLKYSISSQLYK; EEKVLKYSISSQLYKL; EKVLKYSISSQLYKLE;
KVLKYSISSQLYKLEK; VLKYSISSQLYKLEKP; LKYSISSQLYKLEKPE;
KYSISSQLYKLEKPEI; YSISSQLYKLEKPEII; SISSQLYKLEKPEIIE;
ISSQLYKLEKPEIIEL; SSQLYKLEKPEIIELI; SQLYKLEKPEIIELIK;
QLYKLEKPEIIELIKI; LYKLEKPEIIELIKIS; YKLEKPEIIELIKISN;
KLEKPEIIELIKISND; LEKPEIIELIKISNDY; EKPEIIELIKISNDYE;
KPEIIELIKISNDYEK; PEIIELIKISNDYEKE; EIIELIKISNDYEKEK;
IIELIKISNDYEKEKN; IELIKISNDYEKEKNA; ELIKISNDYEKEKNAF;
LIKISNDYEKEKNAFN; IKISNDYEKEKNAFNT; KISNDYEKEKNAFNTS;
ISNDYEKEKNAFNTSL; SNDYEKEKNAFNTSLE; NDYEKEKNAFNTSLEE;
DYEKEKNAFNTSLEEC; YEKEKNAFNTSLEECY; EKEKNAFNTSLEECYY;
KEKNAFNTSLEECYYK; EKNAFNTSLEECYYKN; KNAFNTSLEECYYKNT;
NAFNTSLEECYYKNTQ; AFNTSLEECYYKNTQN; FNTSLEECYYKNTQNE;
NTSLEECYYKNTQNEA; TSLEECYYKNTQNEAI; SLEECYYKNTQNEAIK;
LEECYYKNTQNEAIKK; EECYYKNTQNEAIKKW; ECYYKNTQNEAIKKWI;
CYYKNTQNEAIKKWIL; YYKNTQNEAIKKWILE; YKNTQNEAIKKWILEI;
KNTQNEAIKKWILEIV; NTQNEAIKKWILEIVR; TQNEAIKKWILEIVRN;
QNEAIKKWILEIVRNK; NEAIKKWILEIVRNKN; EAIKKWILEIVRNKNF;

AIKKWILEIVRNKNFI; IKKWILEIVRNKNFIQ; KKWILEIVRNKNFIQI;
KWILEIVRNKNFIQIS; WILEIVRNKNFIQISK; ILEIVRNKNFIQISKE;
LEIVRNKNFIQISKEA; EIVRNKNFIQISKEAN; IVRNKNFIQISKEANL;
VRNKNFIQISKEANLN; RNKNFIQISKEANLNT; NKNFIQISKEANLNTK;
KNFIQISKEANLNTKK; NFIQISKEANLNTKKL; FIQISKEANLNTKKLG;
IQISKEANLNTKKLGT; QISKEANLNTKKLGTT; ISKEANLNTKKLGTTY;
SKEANLNTKKLGTTYK; KEANLNTKKLGTTYKL; EANLNTKKLGTTYKLK;
ANLNTKKLGTTYKLKS; NLNTKKLGTTYKLKSS; LNTKKLGTTYKLKSSN;
NTKKLGTTYKLKSSNF; TKKLGTTYKLKSSNFL; KKLGTTYKLKSSNFLK;
KLGTTYKLKSSNFLKL; LGTTYKLKSSNFLKLI; GTTYKLKSSNFLKLIE;
TTYKLKSSNFLKLIEI; TYKLKSSNFLKLIEIQ; YKLKSSNFLKLIEIQN;
KLKSSNFLKLIEIQNS; LKSSNFLKLIEIQNSP; KSSNFLKLIEIQNSPY;
SSNFLKLIEIQNSPYY; SNFLKLIEIQNSPYYS; NFLKLIEIQNSPYYSQ;
FLKLIEIQNSPYYSQE; LKLIEIQNSPYYSQEK; KLIEIQNSPYYSQEKK;
LIEIQNSPYYSQEKKD; IEIQNSPYYSQEKKDI; EIQNSPYYSQEKKDIY;
IQNSPYYSQEKKDIYK; QNSPYYSQEKKDIYKQ; NSPYYSQEKKDIYKQI;
SPYYSQEKKDIYKQII; PYYSQEKKDIYKQIIL; YYSQEKKDIYKQIILN;
YSQEKKDIYKQIILNF; SQEKKDIYKQIILNFS; QEKKDIYKQIILNFSS;
EKKDIYKQIILNFSSN; KKDIYKQIILNFSSNI; KDIYKQIILNFSSNIN;
DIYKQIILNFSSNINI; IYKQIILNFSSNINID; YKQIILNFSSNINIDS;
KQIILNFSSNINIDSL; QIILNFSSNINIDSLE; IILNFSSNINIDSLEQ;
ILNFSSNINIDSLEQT; LNFSSNINIDSLEQTI; NFSSNINIDSLEQTID;
FSSNINIDSLEQTIDI; SSNINIDSLEQTIDIL; SNINIDSLEQTIDILV;
NINIDSLEQTIDILVA; INIDSLEQTIDILVAV; NIDSLEQTIDILVAVR;
IDSLEQTIDILVAVRN; DSLEQTIDILVAVRNR; SLEQTIDILVAVRNRN;
LEQTIDILVAVRNRNK; EQTIDILVAVRNRNKI; QTIDILVAVRNRNKIK;
TIDILVAVRNRNKIKI; IDILVAVRNRNKIKIL; DILVAVRNRNKIKILN;
ILVAVRNRNKIKILNI; LVAVRNRNKIKILNIL; VAVRNRNKIKILNILN;
AVRNRNKIKILNILNK; VRNRNKIKILNILNKN; RNRNKIKILNILNKNL;
NRNKIKILNILNKNLK; RNKIKILNILNKNLKY; NKIKILNILNKNLKYR;
KIKILNILNKNLKYRP; IKILNILNKNLKYRPE; KILNILNKNLKYRPEN;
ILNILNKNLKYRPENQ; LNILNKNLKYRPENQK; NILNKNLKYRPENQKV;
ILNKNLKYRPENQKVF; LNKNLKYRPENQKVFK; NKNLKYRPENQKVFKS;
KNLKYRPENQKVFKSS; NLKYRPENQKVFKSSL; LKYRPENQKVFKSSLT;
KYRPENQKVFKSSLTN; YRPENQKVFKSSLTNK; RPENQKVFKSSLTNKE;
PENQKVFKSSLTNKES; ENQKVFKSSLTNKESR; NQKVFKSSLTNKESRL;
QKVFKSSLTNKESRLI; KVFKSSLTNKESRLIK; VFKSSLTNKESRLIKL;
FKSSLTNKESRLIKLK; KSSLTNKESRLIKLKR; SSLTNKESRLIKLKRM;
SLTNKESRLIKLKRML; LTNKESRLIKLKRMLI; TNKESRLIKLKRMLIL;
NKESRLIKLKRMLILT; KESRLIKLKRMLILTY; ESRLIKLKRMLILTYW;
SRLIKLKRMLILTYWP; RLIKLKRMLILTYWPV; LIKLKRMLILTYWPVG;
IKLKRMLILTYWPVGC; KLKRMLILTYWPVGCL; LKRMLILTYWPVGCLS;
KRMLILTYWPVGCLSK; RMLILTYWPVGCLSKN; MLILTYWPVGCLSKNI;
LILTYWPVGCLSKNIF; ILTYWPVGCLSKNIFI; LTYWPVGCLSKNIFIK;
TYWPVGCLSKNIFIKI; YWPVGCLSKNIFIKIL; WPVGCLSKNIFIKILI;
PVGCLSKNIFIKILIK; VGCLSKNIFIKILIKX; GCLSKNIFIKILIKXY;
CLSKNIFIKILIKXYK; LSKNIFIKILIKXYKY; SKNIFIKILIKXYKYL;
KNIFIKILIKXYKYLE; NIFIKILIKXYKYLEE; IFIKILIKXYKYLEEE;
FIKILIKXYKYLEEEI; IKILIKXYKYLEEEIL; KILIKXYKYLEEEILA;
ILIKXYKYLEEEILAL; LIKXYKYLEEEILALK; IKXYKYLEEEILALKY;
KXYKYLEEEILALKYN; XYKYLEEEILALKYNE; YKYLEEEILALKYNEI;

| | | |
|---|---|---|
| KYLEEEILALKYNEIL; | YLEEEILALKYNEILN; | LEEEILALKYNEILNY; |
| EEEILALKYNEILNYL; | EEILALKYNEILNYLR; | EILALKYNEILNYLRA; |
| ILALKYNEILNYLRAL; | LALKYNEILNYLRALK; | ALKYNEILNYLRALKT; |
| LKYNEILNYLRALKTL; | KYNEILNYLRALKTLS; | YNEILNYLRALKTLSL; |
| NEILNYLRALKTLSLN; | EILNYLRALKTLSLNE; | ILNYLRALKTLSLNEI; |
| LNYLRALKTLSLNEIF; | NYLRALKTLSLNEIFY; | YLRALKTLSLNEIFYK; |
| LRALKTLSLNEIFYKG; | RALKTLSLNEIFYKGS; | ALKTLSLNEIFYKGSS; |
| LKTLSLNEIFYKGSSK; | KTLSLNEIFYKGSSKN; | TLSLNEIFYKGSSKNI; |
| LSLNEIFYKGSSKNIS; | SLNEIFYKGSSKNISF; | LNEIFYKGSSKNISFN; |
| NEIFYKGSSKNISFNY; | EIFYKGSSKNISFNYF; | IFYKGSSKNISFNYFF; |
| FYKGSSKNISFNYFFS; | YKGSSKNISFNYFFSD; | KGSSKNISFNYFFSDF; |
| GSSKNISFNYFFSDFT; | SSKNISFNYFFSDFTQ; | SKNISFNYFFSDFTQY; |
| KNISFNYFFSDFTQYA; | NISFNYFFSDFTQYAP; | ISFNYFFSDFTQYAPK; |
| SFNYFFSDFTQYAPKD; | FNYFFSDFTQYAPKDF; | NYFFSDFTQYAPKDFQ; |
| YFFSDFTQYAPKDFQD; | FFSDFTQYAPKDFQDT; | FSDFTQYAPKDFQDTL; |
| SDFTQYAPKDFQDTLK; | DFTQYAPKDFQDTLKC; | FTQYAPKDFQDTLKCY; |
| TQYAPKDFQDTLKCYL; | QYAPKDFQDTLKCYLY; | YAPKDFQDTLKCYLYV; |
| APKDFQDTLKCYLYVI; | PKDFQDTLKCYLYVIE; | KDFQDTLKCYLYVIEK; |
| DFQDTLKCYLYVIEKT; | FQDTLKCYLYVIEKTI; | QDTLKCYLYVIEKTIT; |
| DTLKCYLYVIEKTITK; | TLKCYLYVIEKTITKK; | LKCYLYVIEKTITKKY; |
| KCYLYVIEKTITKKYL; | CYLYVIEKTITKKYLT; | YLYVIEKTITKKYLTW; |
| LYVIEKTITKKYLTWF; | YVIEKTITKKYLTWFF; | VIEKTITKKYLTWFFK; |
| IEKTITKKYLTWFFKD; | EKTITKKYLTWFFKDK; | KTITKKYLTWFFKDKI; |
| TITKKYLTWFFKDKIS; | ITKKYLTWFFKDKISN; | TKKYLTWFFKDKISNN; |
| KKYLTWFFKDKISNNW; | KYLTWFFKDKISNNWE; | YLTWFFKDKISNNWES; |
| LTWFFKDKISNNWESF; | TWFFKDKISNNWESFI; | WFFKDKISNNWESFID; |
| FFKDKISNNWESFIDA; | FKDKISNNWESFIDAL; | KDKISNNWESFIDALE; |
| DKISNNWESFIDALEY; | KISNNWESFIDALEYI; | ISNNWESFIDALEYIE; |
| SNNWESFIDALEYIEK; | NNWESFIDALEYIEKH; | NWESFIDALEYIEKHK; |
| WESFIDALEYIEKHKL; | ESFIDALEYIEKHKLI; | SFIDALEYIEKHKLIN; |
| FIDALEYIEKHKLINI; | IDALEYIEKHKLINIK; | DALEYIEKHKLINIKE; |
| ALEYIEKHKLINIKEK; | LEYIEKHKLINIKEKI; | EYIEKHKLINIKEKIK; |
| YIEKHKLINIKEKIKE; | IEKHKLINIKEKIKEI; | EKHKLINIKEKIKEII; |
| KHKLINIKEKIKEIIT; | HKLINIKEKIKEIITA; | KLINIKEKIKEIITAK; |
| LINIKEKIKEIITAKF; | INIKEKIKEIITAKFQ; | NIKEKIKEIITAKFQT; |
| IKEKIKEIITAKFQTE; | KEKIKEIITAKFQTED; | EKIKEIITAKFQTEDF; |
| KIKEIITAKFQTEDFF; | IKEIITAKFQTEDFFI; | KEIITAKFQTEDFFIS; |
| EIITAKFQTEDFFISF; | IITAKFQTEDFFISFD; | ITAKFQTEDFFISFDK; |
| TAKFQTEDFFISFDKT; | AKFQTEDFFISFDKTN; | KFQTEDFFISFDKTNF; |
| FQTEDFFISFDKTNFN; | QTEDFFISFDKTNFNP; | TEDFFISFDKTNFNPY; |
| EDFFISFDKTNFNPYE; | DFFISFDKTNFNPYES; | FFISFDKTNFNPYESS; |
| FISFDKTNFNPYESSI; | ISFDKTNFNPYESSIF; | SFDKTNFNPYESSIFK; |
| FDKTNFNPYESSIFKE; | DKTNFNPYESSIFKER; | KTNFNPYESSIFKERY; |
| TNFNPYESSIFKERYI; | NFNPYESSIFKERYIK; | FNPYESSIFKERYIKS; |
| NPYESSIFKERYIKSA; | PYESSIFKERYIKSAF; | YESSIFKERYIKSAFL; |
| ESSIFKERYIKSAFLE; | SSIFKERYIKSAFLEI; | SIFKERYIKSAFLEII; |
| IFKERYIKSAFLEIIM; | FKERYIKSAFLEIIMH; | KERYIKSAFLEIIMHY; |
| ERYIKSAFLEIIMHYV; | RYIKSAFLEIIMHYVK; | YIKSAFLEIIMHYVKK; |
| IKSAFLEIIMHYVKKN; | KSAFLEIIMHYVKKNS; | SAFLEIIMHYVKKNSQ; |
| AFLEIIMHYVKKNSQN; | FLEIIMHYVKKNSQNI; | LEIIMHYVKKNSQNIE; |
| EIIMHYVKKNSQNIET; | IIMHYVKKNSQNIETY; | IMHYVKKNSQNIETYG; |

| | | |
|---|---|---|
| MHYVKKNSQNIETYGW; | HYVKKNSQNIETYGWI; | YVKKNSQNIETYGWIA; |
| VKKNSQNIETYGWIAF; | KKNSQNIETYGWIAFY; | KNSQNIETYGWIAFYI; |
| NSQNIETYGWIAFYIY; | SQNIETYGWIAFYIYA; | QNIETYGWIAFYIYAD; |
| NIETYGWIAFYIYADN; | IETYGWIAFYIYADNK; | ETYGWIAFYIYADNKD; |
| TYGWIAFYIYADNKDK; | YGWIAFYIYADNKDKK; | GWIAFYIYADNKDKKI; |
| WIAFYIYADNKDKKIF; | IAFYIYADNKDKKIFC; | AFYIYADNKDKKIFCQ; |
| FYIYADNKDKKIFCQR; | YIYADNKDKKIFCQRM; | IYADNKDKKIFCQRMK; |
| YADNKDKKIFCQRMKE; | ADNKDKKIFCQRMKEF; | DNKDKKIFCQRMKEFF; |
| NKDKKIFCQRMKEFFK; | KDKKIFCQRMKEFFKS; | DKKIFCQRMKEFFKSK; |
| KKIFCQRMKEFFKSKT; | KIFCQRMKEFFKSKTF; | IFCQRMKEFFKSKTFE; |
| FCQRMKEFFKSKTFEI; | CQRMKEFFKSKTFEIQ; | QRMKEFFKSKTFEIQN; |
| RMKEFFKSKTFEIQNQ; | MKEFFKSKTFEIQNQI; | KEFFKSKTFEIQNQIF; |
| EFFKSKTFEIQNQIFV; | FFKSKTFEIQNQIFVY; | FKSKTFEIQNQIFVYF; |
| KSKTFEIQNQIFVYFL; | SKTFEIQNQIFVYFLS; | KTFEIQNQIFVYFLSF; |
| TFEIQNQIFVYFLSFY; | FEIQNQIFVYFLSFYP; | EIQNQIFVYFLSFYPN; |
| IQNQIFVYFLSFYPNI; | QNQIFVYFLSFYPNIE; | NQIFVYFLSFYPNIEY; |
| QIFVYFLSFYPNIEYK; | IFVYFLSFYPNIEYKH; | FVYFLSFYPNIEYKHF; |
| VYFLSFYPNIEYKHFK; | YFLSFYPNIEYKHFKF; | FLSFYPNIEYKHFKFI; |
| LSFYPNIEYKHFKFIS; | SFYPNIEYKHFKFISD; | FYPNIEYKHFKFISDI; |
| YPNIEYKHFKFISDIL; | PNIEYKHFKFISDILL; | NIEYKHFKFISDILLY; |
| IEYKHFKFISDILLYL; | EYKHFKFISDILLYLD; | YKHFKFISDILLYLDE; |
| KHFKFISDILLYLDEN; | HFKFISDILLYLDENK; | FKFISDILLYLDENKI; |
| KFISDILLYLDENKIT; | FISDILLYLDENKITI; | ISDILLYLDENKITIP; |
| SDILLYLDENKITIPK; | DILLYLDENKITIPKK; | ILLYLDENKITIPKKI; |
| LLYLDENKITIPKKII; | LYLDENKITIPKKIIK; | YLDENKITIPKKIIKI; |
| LDENKITIPKKIIKIQ; | DENKITIPKKIIKIQK; | ENKITIPKKIIKIQKI; |
| NKITIPKKIIKIQKIN; | KITIPKKIIKIQKINP; | ITIPKKIIKIQKINPN; |
| TIPKKIIKIQKINPNQ; | IPKKIIKIQKINPNQL; | PKKIIKIQKINPNQLD; |
| KKIIKIQKINPNQLDY; | KIIKIQKINPNQLDYQ; | IIKIQKINPNQLDYQK; |
| IKIQKINPNQLDYQKS; | KIQKINPNQLDYQKSY; | IQKINPNQLDYQKSYL; |
| QKINPNQLDYQKSYLL; | KINPNQLDYQKSYLLI; | INPNQLDYQKSYLLIK; |
| NPNQLDYQKSYLLIKS; | PNQLDYQKSYLLIKSL; | NQLDYQKSYLLIKSLK; |
| QLDYQKSYLLIKSLKT; | LDYQKSYLLIKSLKTR; | DYQKSYLLIKSLKTRS; |
| YQKSYLLIKSLKTRSR; | QKSYLLIKSLKTRSRI; | KSYLLIKSLKTRSRIL; |
| SYLLIKSLKTRSRILK; | YLLIKSLKTRSRILKI; | LLIKSLKTRSRILKII; |
| LIKSLKTRSRILKIII; | IKSLKTRSRILKIIIK; | KSLKTRSRILKIIIKN; |
| SLKTRSRILKIIIKNI; | LKTRSRILKIIIKNIR; | KTRSRILKIIIKNIRF; |
| TRSRILKIIIKNIRFN; | RSRILKIIIKNIRFNL; | SRILKIIIKNIRFNLI; |
| RILKIIIKNIRFNLIE; | ILKIIIKNIRFNLIEK; | LKIIIKNIRFNLIEKL; |
| KIIIKNIRFNLIEKLE; | IIIKNIRFNLIEKLED; | IIKNIRFNLIEKLEDE; |
| IKNIRFNLIEKLEDEN; | KNIRFNLIEKLEDENE; | NIRFNLIEKLEDENEK; |
| IRFNLIEKLEDENEKK; | RFNLIEKLEDENEKKL; | FNLIEKLEDENEKKLL; |
| NLIEKLEDENEKKLLP; | LIEKLEDENEKKLLPA; | IEKLEDENEKKLLPAM; |
| EKLEDENEKKLLPAMC; | KLEDENEKKLLPAMCY; | LEDENEKKLLPAMCYL; |
| EDENEKKLLPAMCYLI; | DENEKKLLPAMCYLIY; | ENEKKLLPAMCYLIYC; |
| NEKKLLPAMCYLIYCE; | EKKLLPAMCYLIYCEN; | KKLLPAMCYLIYCENL; |
| KLLPAMCYLIYCENLV; | LLPAMCYLIYCENLVE; | LPAMCYLIYCENLVEL; |
| PAMCYLIYCENLVELK; | AMCYLIYCENLVELKN; | MCYLIYCENLVELKNN; |
| CYLIYCENLVELKNNR; | YLIYCENLVELKNNRK; | LIYCENLVELKNNRKI; |
| IYCENLVELKNNRKIK; | YCENLVELKNNRKIKS; | CENLVELKNNRKIKSN; |
| ENLVELKNNRKIKSNE; | NLVELKNNRKIKSNEK; | LVELKNNRKIKSNEKN; |

| | |
|---|---|
| | VELKNNRKIKSNEKNS; ELKNNRKIKSNEKNSL; LKNNRKIKSNEKNSLL; KNNRKIKSNEKNSLLE; NNRKIKSNEKNSLLEF; NRKIKSNEKNSLLEFI; RKIKSNEKNSLLEFIS; KIKSNEKNSLLEFISF; IKSNEKNSLLEFISFF; KSNEKNSLLEFISFFK; SNEKNSLLEFISFFKT; NEKNSLLEFISFFKTK; EKNSLLEFISFFKTKL; KNSLLEFISFFKTKLK; NSLLEFISFFKTKLKQ; SLLEFISFFKTKLKQK; LLEFISFFKTKLKQKI; LEFISFFKTKLKQKIN; EFISFFKTKLKQKINF; FISFFKTKLKQKINFK; ISFFKTKLKQKINFKK; SFFKTKLKQKINFKKE; FFKTKLKQKINFKKEL; FKTKLKQKINFKKELM; KTKLKQKINFKKELMK; TKLKQKINFKKELMKS; KLKQKINFKKELMKSK; LKQKINFKKELMKSKG; KQKINFKKELMKSKGI; |
| Seq 29 | 13 mers:<br>MITQQDYTEIKKK; ITQQDYTEIKKKL; TQQDYTEIKKKLE; QQDYTEIKKKLEN; QDYTEIKKKLENT; DYTEIKKKLENTK; YTEIKKKLENTKT; TEIKKKLENTKTR; EIKKKLENTKTRI; IKKKLENTKTRIQ; KKKLENTKTRIQF; KKLENTKTRIQFR; KLENTKTRIQFRN; LENTKTRIQFRNG; ENTKTRIQFRNGN; NTKTRIQFRNGNK; TKTRIQFRNGNKI; KTRIQFRNGNKID; TRIQFRNGNKIDS; RIQFRNGNKIDSL; IQFRNGNKIDSLG; QFRNGNKIDSLGY; FRNGNKIDSLGYQ; RNGNKIDSLGYQG; NGNKIDSLGYQGS; GNKIDSLGYQGSL; NKIDSLGYQGSLG; KIDSLGYQGSLGE; IDSLGYQGSLGEP; DSLGYQGSLGEPI; SLGYQGSLGEPIL; LGYQGSLGEPILL; GYQGSLGEPILLK; YQGSLGEPILLKD; QGSLGEPILLKDK; GSLGEPILLKDKL; SLGEPILLKDKLT; LGEPILLKDKLTF; GEPILLKDKLTFS; EPILLKDKLTFSI; PILLKDKLTFSIC; ILLKDKLTFSICD; LLKDKLTFSICDG; LKDKLTFSICDGK; KDKLTFSICDGKN; DKLTFSICDGKNI; KLTFSICDGKNID; LTFSICDGKNIDN; TFSICDGKNIDNR; FSICDGKNIDNRK; SICDGKNIDNRKE; ICDGKNIDNRKEY; CDGKNIDNRKEYE; DGKNIDNRKEYEI; GKNIDNRKEYEIT; KNIDNRKEYEITK; NIDNRKEYEITKN; IDNRKEYEITKNA; DNRKEYEITKNAP; NRKEYEITKNAPS; RKEYEITKNAPSL; KEYEITKNAPSLD; EYEITKNAPSLDS; YEITKNAPSLDSL; EITKNAPSLDSLI; ITKNAPSLDSLIE; TKNAPSLDSLIEY; KNAPSLDSLIEYL; NAPSLDSLIEYLE; APSLDSLIEYLEH; PSLDSLIEYLEHM; SLDSLIEYLEHMI; LDSLIEYLEHMIL; DSLIEYLEHMILF; SLIEYLEHMILFF; LIEYLEHMILFFL; IEYLEHMILFFLE; EYLEHMILFFLEK; YLEHMILFFLEKT; LEHMILFFLEKTP; EHMILFFLEKTPV; HMILFFLEKTPVG; MILFFLEKTPVGT; ILFFLEKTPVGTL; LFFLEKTPVGTLT; FFLEKTPVGTLTA; FLEKTPVGTLTAK; LEKTPVGTLTAKF; EKTPVGTLTAKFA; KTPVGTLTAKFAM; TPVGTLTAKFAME; PVGTLTAKFAMEG; VGTLTAKFAMEGS; GTLTAKFAMEGSI; TLTAKFAMEGSIL; LTAKFAMEGSILT; TAKFAMEGSILTR; AKFAMEGSILTRS; KFAMEGSILTRSK; FAMEGSILTRSKL; AMEGSILTRSKLI; MEGSILTRSKLIQ; EGSILTRSKLIQA; GSILTRSKLIQAF; SILTRSKLIQAFT; ILTRSKLIQAFTN; LTRSKLIQAFTNT; TRSKLIQAFTNTN; RSKLIQAFTNTNK; SKLIQAFTNTNKF; KLIQAFTNTNKFC; LIQAFTNTNKFCI; IQAFTNTNKFCIP; QAFTNTNKFCIPD; |

AFTNTNKFCIPDG; FTNTNKFCIPDGR; TNTNKFCIPDGRS;
NTNKFCIPDGRSL; TNKFCIPDGRSLP; NKFCIPDGRSLPS;
KFCIPDGRSLPSN; FCIPDGRSLPSNC; CIPDGRSLPSNCY;
IPDGRSLPSNCYA; PDGRSLPSNCYAY; DGRSLPSNCYAYK;
GRSLPSNCYAYKV; RSLPSNCYAYKVL; SLPSNCYAYKVLG;
LPSNCYAYKVLGI; PSNCYAYKVLGIS; SNCYAYKVLGISS;
NCYAYKVLGISSA; CYAYKVLGISSAP; YAYKVLGISSAPK;
AYKVLGISSAPKL; YKVLGISSAPKLS; KVLGISSAPKLSG;
VLGISSAPKLSGR; LGISSAPKLSGRF; GISSAPKLSGRFL;
ISSAPKLSGRFLR; SSAPKLSGRFLRH; SAPKLSGRFLRHY;
APKLSGRFLRHYD; PKLSGRFLRHYDS; KLSGRFLRHYDSG;
LSGRFLRHYDSGG; SGRFLRHYDSGGS; GRFLRHYDSGGST;
RFLRHYDSGGSTD; FLRHYDSGGSTDS; LRHYDSGGSTDSD;
RHYDSGGSTDSDG; HYDSGGSTDSDGT; YDSGGSTDSDGTR;
DSGGSTDSDGTRS; SGGSTDSDGTRSL; GGSTDSDGTRSLG;
GSTDSDGTRSLGH; STDSDGTRSLGHT; TDSDGTRSLGHTQ;
DSDGTRSLGHTQE; SDGTRSLGHTQED; DGTRSLGHTQEDS;
GTRSLGHTQEDSY; TRSLGHTQEDSYK; RSLGHTQEDSYKN;
SLGHTQEDSYKNH; LGHTQEDSYKNHD; GHTQEDSYKNHDH;
HTQEDSYKNHDHD; TQEDSYKNHDHDL; QEDSYKNHDHDLD;
EDSYKNHDHDLDF; DSYKNHDHDLDFS; SYKNHDHDLDFSR;
YKNHDHDLDFSRI; KNHDHDLDFSRIN; NHDHDLDFSRINF;
HDHDLDFSRINFK; DHDLDFSRINFKG; HDLDFSRINFKGK;
DLDFSRINFKGKL; LDFSRINFKGKLV; DFSRINFKGKLVK;
FSRINFKGKLVKR; SRINFKGKLVKRM; RINFKGKLVKRMT;
INFKGKLVKRMTM; NFKGKLVKRMTMI; FKGKLVKRMTMIY;
KGKLVKRMTMIYR; GKLVKRMTMIYRS; KLVKRMTMIYRSA;
LVKRMTMIYRSAD; VKRMTMIYRSADT; KRMTMIYRSADTW;
RMTMIYRSADTWY; MTMIYRSADTWYT; TMIYRSADTWYTW;
MIYRSADTWYTWY; IYRSADTWYTWYW; YRSADTWYTWYWA;
RSADTWYTWYWAE; SADTWYTWYWAEE; ADTWYTWYWAEEI;
DTWYTWYWAEEIM; TWYTWYWAEEIMS; WYTWYWAEEIMSP;
YTWYWAEEIMSPF; TWYWAEEIMSPFI; WYWAEEIMSPFIT;
YWAEEIMSPFITG; WAEEIMSPFITGY; AEEIMSPFITGYS;
EEIMSPFITGYSY; EIMSPFITGYSYE; IMSPFITGYSYEE;
MSPFITGYSYEEI; SPFITGYSYEEID; PFITGYSYEEIDP;
FITGYSYEEIDPE; ITGYSYEEIDPEY; TGYSYEEIDPEYL;
GYSYEEIDPEYLL; YSYEEIDPEYLLP; SYEEIDPEYLLPP;
YEEIDPEYLLPPF; EEIDPEYLLPPFT; EIDPEYLLPPFTS;
IDPEYLLPPFTSQ; DPEYLLPPFTSQS; PEYLLPPFTSQSG;
EYLLPPFTSQSGS; YLLPPFTSQSGSK; LLPPFTSQSGSKE;
LPPFTSQSGSKET; PPFTSQSGSKETK; PFTSQSGSKETKP;
FTSQSGSKETKPK; TSQSGSKETKPKN; SQSGSKETKPKNT;
QSGSKETKPKNTC; SGSKETKPKNTCY; GSKETKPKNTCYI;
SKETKPKNTCYIS; KETKPKNTCYISF; ETKPKNTCYISFY;
TKPKNTCYISFYR; KPKNTCYISFYRY; PKNTCYISFYRYD;
KNTCYISFYRYDL;

14 mers:
MITQQDYTEIKKKL; ITQQDYTEIKKKLE; TQQDYTEIKKKLEN;
QQDYTEIKKKLENT; QDYTEIKKKLENTK; DYTEIKKKLENTKT;

| | | |
|---|---|---|
| YTEIKKKLENTKTR; | TEIKKKLENTKTRI; | EIKKKLENTKTRIQ; |
| IKKKLENTKTRIQF; | KKKLENTKTRIQFR; | KKLENTKTRIQFRN; |
| KLENTKTRIQFRNG; | LENTKTRIQFRNGN; | ENTKTRIQFRNGNK; |
| NTKTRIQFRNGNKI; | TKTRIQFRNGNKID; | KTRIQFRNGNKIDS; |
| TRIQFRNGNKIDSL; | RIQFRNGNKIDSLG; | IQFRNGNKIDSLGY; |
| QFRNGNKIDSLGYQ; | FRNGNKIDSLGYQG; | RNGNKIDSLGYQGS; |
| NGNKIDSLGYQGSL; | GNKIDSLGYQGSLG; | NKIDSLGYQGSLGE; |
| KIDSLGYQGSLGEP; | IDSLGYQGSLGEPI; | DSLGYQGSLGEPIL; |
| SLGYQGSLGEPILL; | LGYQGSLGEPILLK; | GYQGSLGEPILLKD; |
| YQGSLGEPILLKDK; | QGSLGEPILLKDKL; | GSLGEPILLKDKLT; |
| SLGEPILLKDKLTF; | LGEPILLKDKLTFS; | GEPILLKDKLTFSI; |
| EPILLKDKLTFSIC; | PILLKDKLTFSICD; | ILLKDKLTFSICDG; |
| LLKDKLTFSICDGK; | LKDKLTFSICDGKN; | KDKLTFSICDGKNI; |
| DKLTFSICDGKNID; | KLTFSICDGKNIDN; | LTFSICDGKNIDNR; |
| TFSICDGKNIDNRK; | FSICDGKNIDNRKE; | SICDGKNIDNRKEY; |
| ICDGKNIDNRKEYE; | CDGKNIDNRKEYEI; | DGKNIDNRKEYEIT; |
| GKNIDNRKEYEITK; | KNIDNRKEYEITKN; | NIDNRKEYEITKNA; |
| IDNRKEYEITKNAP; | DNRKEYEITKNAPS; | NRKEYEITKNAPSL; |
| RKEYEITKNAPSLD; | KEYEITKNAPSLDS; | EYEITKNAPSLDSL; |
| YEITKNAPSLDSLI; | EITKNAPSLDSLIE; | ITKNAPSLDSLIEY; |
| TKNAPSLDSLIEYL; | KNAPSLDSLIEYLE; | NAPSLDSLIEYLEH; |
| APSLDSLIEYLEHM; | PSLDSLIEYLEHMI; | SLDSLIEYLEHMIL; |
| LDSLIEYLEHMILF; | DSLIEYLEHMILFF; | SLIEYLEHMILFFL; |
| LIEYLEHMILFFLE; | IEYLEHMILFFLEK; | EYLEHMILFFLEKT; |
| YLEHMILFFLEKTP; | LEHMILFFLEKTPV; | EHMILFFLEKTPVG; |
| HMILFFLEKTPVGT; | MILFFLEKTPVGTL; | ILFFLEKTPVGTLT; |
| LFFLEKTPVGTLTA; | FFLEKTPVGTLTAK; | FLEKTPVGTLTAKF; |
| LEKTPVGTLTAKFA; | EKTPVGTLTAKFAM; | KTPVGTLTAKFAME; |
| TPVGTLTAKFAMEG; | PVGTLTAKFAMEGS; | VGTLTAKFAMEGSI; |
| GTLTAKFAMEGSIL; | TLTAKFAMEGSILT; | LTAKFAMEGSILTR; |
| TAKFAMEGSILTRS; | AKFAMEGSILTRSK; | KFAMEGSILTRSKL; |
| FAMEGSILTRSKLI; | AMEGSILTRSKLIQ; | MEGSILTRSKLIQA; |
| EGSILTRSKLIQAF; | GSILTRSKLIQAFT; | SILTRSKLIQAFTN; |
| ILTRSKLIQAFTNT; | LTRSKLIQAFTNTN; | TRSKLIQAFTNTNK; |
| RSKLIQAFTNTNKF; | SKLIQAFTNTNKFC; | KLIQAFTNTNKFCI; |
| LIQAFTNTNKFCIP; | IQAFTNTNKFCIPD; | QAFTNTNKFCIPDG; |
| AFTNTNKFCIPDGR; | FTNTNKFCIPDGRS; | TNTNKFCIPDGRSL; |
| NTNKFCIPDGRSLP; | TNKFCIPDGRSLPS; | NKFCIPDGRSLPSN; |
| KFCIPDGRSLPSNC; | FCIPDGRSLPSNCY; | CIPDGRSLPSNCYA; |
| IPDGRSLPSNCYAY; | PDGRSLPSNCYAYK; | DGRSLPSNCYAYKV; |
| GRSLPSNCYAYKVL; | RSLPSNCYAYKVLG; | SLPSNCYAYKVLGI; |
| LPSNCYAYKVLGIS; | PSNCYAYKVLGISS; | SNCYAYKVLGISSA; |
| NCYAYKVLGISSAP; | CYAYKVLGISSAPK; | YAYKVLGISSAPKL; |
| AYKVLGISSAPKLS; | YKVLGISSAPKLSG; | KVLGISSAPKLSGR; |
| VLGISSAPKLSGRF; | LGISSAPKLSGRFL; | GISSAPKLSGRFLR; |
| ISSAPKLSGRFLRH; | SSAPKLSGRFLRHY; | SAPKLSGRFLRHYD; |
| APKLSGRFLRHYDS; | PKLSGRFLRHYDSG; | KLSGRFLRHYDSGG; |
| LSGRFLRHYDSGGS; | SGRFLRHYDSGGST; | GRFLRHYDSGGSTD; |
| RFLRHYDSGGSTDS; | FLRHYDSGGSTDSD; | LRHYDSGGSTDSDG; |
| RHYDSGGSTDSDGT; | HYDSGGSTDSDGTR; | YDSGGSTDSDGTRS; |
| DSGGSTDSDGTRSL; | SGGSTDSDGTRSLG; | GGSTDSDGTRSLGH; |

GSTDSDGTRSLGHT; STDSDGTRSLGHTQ; TDSDGTRSLGHTQE;
DSDGTRSLGHTQED; SDGTRSLGHTQEDS; DGTRSLGHTQEDSY;
GTRSLGHTQEDSYK; TRSLGHTQEDSYKN; RSLGHTQEDSYKNH;
SLGHTQEDSYKNHD; LGHTQEDSYKNHDH; GHTQEDSYKNHDHD;
HTQEDSYKNHDHDL; TQEDSYKNHDHDLD; QEDSYKNHDHDLDF;
EDSYKNHDHDLDFS; DSYKNHDHDLDFSR; SYKNHDHDLDFSRI;
YKNHDHDLDFSRIN; KNHDHDLDFSRINF; NHDHDLDFSRINFK;
HDHDLDFSRINFKG; DHDLDFSRINFKGK; HDLDFSRINFKGKL;
DLDFSRINFKGKLV; LDFSRINFKGKLVK; DFSRINFKGKLVKR;
FSRINFKGKLVKRM; SRINFKGKLVKRMT; RINFKGKLVKRMTM;
INFKGKLVKRMTMI; NFKGKLVKRMTMIY; FKGKLVKRMTMIYR;
KGKLVKRMTMIYRS; GKLVKRMTMIYRSA; KLVKRMTMIYRSAD;
LVKRMTMIYRSADT; VKRMTMIYRSADTW; KRMTMIYRSADTWY;
RMTMIYRSADTWYT; MTMIYRSADTWYTW; TMIYRSADTWYTWY;
MIYRSADTWYTWYW; IYRSADTWYTWYWA; YRSADTWYTWYWAE;
RSADTWYTWYWAEE; SADTWYTWYWAEEI; ADTWYTWYWAEEIM;
DTWYTWYWAEEIMS; TWYTWYWAEEIMSP; WYTWYWAEEIMSPF;
YTWYWAEEIMSPFI; TWYWAEEIMSPFIT; WYWAEEIMSPFITG;
YWAEEIMSPFITGY; WAEEIMSPFITGYS; AEEIMSPFITGYSY;
EEIMSPFITGYSYE; EIMSPFITGYSYEE; IMSPFITGYSYEEI;
MSPFITGYSYEEID; SPFITGYSYEEIDP; PFITGYSYEEIDPE;
FITGYSYEEIDPEY; ITGYSYEEIDPEYL; TGYSYEEIDPEYLL;
GYSYEEIDPEYLLP; YSYEEIDPEYLLPP; SYEEIDPEYLLPPF;
YEEIDPEYLLPPFT; EEIDPEYLLPPFTS; EIDPEYLLPPFTSQ;
IDPEYLLPPFTSQS; DPEYLLPPFTSQSG; PEYLLPPFTSQSGS;
EYLLPPFTSQSGSK; YLLPPFTSQSGSKE; LLPPFTSQSGSKET;
LPPFTSQSGSKETK; PPFTSQSGSKETKP; PFTSQSGSKETKPK;
FTSQSGSKETKPKN; TSQSGSKETKPKNT; SQSGSKETKPKNTC;
QSGSKETKPKNTCY; SGSKETKPKNTCYI; GSKETKPKNTCYIS;
SKETKPKNTCYISF; KETKPKNTCYISFY; ETKPKNTCYISFYR;
TKPKNTCYISFYRY; KPKNTCYISFYRYD; PKNTCYISFYRYDL;

15 mers:
MITQQDYTEIKKKLE; ITQQDYTEIKKKLEN; TQQDYTEIKKKLENT;
QQDYTEIKKKLENTK; QDYTEIKKKLENTKT; DYTEIKKKLENTKTR;
YTEIKKKLENTKTRI; TEIKKKLENTKTRIQ; EIKKKLENTKTRIQF;
IKKKLENTKTRIQFR; KKKLENTKTRIQFRN; KKLENTKTRIQFRNG;
KLENTKTRIQFRNGN; LENTKTRIQFRNGNK; ENTKTRIQFRNGNKI;
NTKTRIQFRNGNKID; TKTRIQFRNGNKIDS; KTRIQFRNGNKIDSL;
TRIQFRNGNKIDSLG; RIQFRNGNKIDSLGY; IQFRNGNKIDSLGYQ;
QFRNGNKIDSLGYQG; FRNGNKIDSLGYQGS; RNGNKIDSLGYQGSL;
NGNKIDSLGYQGSLG; GNKIDSLGYQGSLGE; NKIDSLGYQGSLGEP;
KIDSLGYQGSLGEPI; IDSLGYQGSLGEPIL; DSLGYQGSLGEPILL;
SLGYQGSLGEPILLK; LGYQGSLGEPILLKD; GYQGSLGEPILLKDK;
YQGSLGEPILLKDKL; QGSLGEPILLKDKLT; GSLGEPILLKDKLTF;
SLGEPILLKDKLTFS; LGEPILLKDKLTFSI; GEPILLKDKLTFSIC;
EPILLKDKLTFSICD; PILLKDKLTFSICDG; ILLKDKLTFSICDGK;
LLKDKLTFSICDGKN; LKDKLTFSICDGKNI; KDKLTFSICDGKNID;
DKLTFSICDGKNIDN; KLTFSICDGKNIDNR; LTFSICDGKNIDNRK;
TFSICDGKNIDNRKE; FSICDGKNIDNRKEY; SICDGKNIDNRKEYE;
ICDGKNIDNRKEYEI; CDGKNIDNRKEYEIT; DGKNIDNRKEYEITK;

| | GKNIDNRKEYEITKN; KNIDNRKEYEITKNA; NIDNRKEYEITKNAP;<br>IDNRKEYEITKNAPS; DNRKEYEITKNAPSL; NRKEYEITKNAPSLD;<br>RKEYEITKNAPSLDS; KEYEITKNAPSLDSL; EYEITKNAPSLDSLI;<br>YEITKNAPSLDSLIE; EITKNAPSLDSLIEY; ITKNAPSLDSLIEYL;<br>TKNAPSLDSLIEYLE; KNAPSLDSLIEYLEH; NAPSLDSLIEYLEHM;<br>APSLDSLIEYLEHMI; PSLDSLIEYLEHMIL; SLDSLIEYLEHMILF;<br>LDSLIEYLEHMILFF; DSLIEYLEHMILFFL; SLIEYLEHMILFFLE;<br>LIEYLEHMILFFLEK; IEYLEHMILFFLEKT; EYLEHMILFFLEKTP;<br>YLEHMILFFLEKTPV; LEHMILFFLEKTPVG; EHMILFFLEKTPVGT;<br>HMILFFLEKTPVGTL; MILFFLEKTPVGTLT; ILFFLEKTPVGTLTA;<br>LFFLEKTPVGTLTAK; FFLEKTPVGTLTAKF; FLEKTPVGTLTAKFA;<br>LEKTPVGTLTAKFAM; EKTPVGTLTAKFAME; KTPVGTLTAKFAMEG;<br>TPVGTLTAKFAMEGS; PVGTLTAKFAMEGSI; VGTLTAKFAMEGSIL;<br>GTLTAKFAMEGSILT; TLTAKFAMEGSILTR; LTAKFAMEGSILTRS;<br>TAKFAMEGSILTRSK; AKFAMEGSILTRSKL; KFAMEGSILTRSKLI;<br>FAMEGSILTRSKLIQ; AMEGSILTRSKLIQA; MEGSILTRSKLIQAF;<br>EGSILTRSKLIQAFT; GSILTRSKLIQAFTN; SILTRSKLIQAFTNT;<br>ILTRSKLIQAFTNTN; LTRSKLIQAFTNTNK; TRSKLIQAFTNTNKF;<br>RSKLIQAFTNTNKFC; SKLIQAFTNTNKFCI; KLIQAFTNTNKFCIP;<br>LIQAFTNTNKFCIPD; IQAFTNTNKFCIPDG; QAFTNTNKFCIPDGR;<br>AFTNTNKFCIPDGRS; FTNTNKFCIPDGRSL; TNTNKFCIPDGRSLP;<br>NTNKFCIPDGRSLPS; TNKFCIPDGRSLPSN; NKFCIPDGRSLPSNC;<br>KFCIPDGRSLPSNCY; FCIPDGRSLPSNCYA; CIPDGRSLPSNCYAY;<br>IPDGRSLPSNCYAYK; PDGRSLPSNCYAYKV; DGRSLPSNCYAYKVL;<br>GRSLPSNCYAYKVLG; RSLPSNCYAYKVLGI; SLPSNCYAYKVLGIS;<br>LPSNCYAYKVLGISS; PSNCYAYKVLGISSA; SNCYAYKVLGISSAP;<br>NCYAYKVLGISSAPK; CYAYKVLGISSAPKL; YAYKVLGISSAPKLS;<br>AYKVLGISSAPKLSG; YKVLGISSAPKLSGR; KVLGISSAPKLSGRF;<br>VLGISSAPKLSGRFL; LGISSAPKLSGRFLR; GISSAPKLSGRFLRH;<br>ISSAPKLSGRFLRHY; SSAPKLSGRFLRHYD; SAPKLSGRFLRHYDS;<br>APKLSGRFLRHYDSG; PKLSGRFLRHYDSGG; KLSGRFLRHYDSGGS;<br>LSGRFLRHYDSGGST; SGRFLRHYDSGGSTD; GRFLRHYDSGGSTDS;<br>RFLRHYDSGGSTDSD; FLRHYDSGGSTDSDG; LRHYDSGGSTDSDGT;<br>RHYDSGGSTDSDGTR; HYDSGGSTDSDGTRS; YDSGGSTDSDGTRSL;<br>DSGGSTDSDGTRSLG; SGGSTDSDGTRSLGH; GGSTDSDGTRSLGHT;<br>GSTDSDGTRSLGHTQ; STDSDGTRSLGHTQE; TDSDGTRSLGHTQED;<br>DSDGTRSLGHTQEDS; SDGTRSLGHTQEDSY; DGTRSLGHTQEDSYK;<br>GTRSLGHTQEDSYKN; TRSLGHTQEDSYKNH; RSLGHTQEDSYKNHD;<br>SLGHTQEDSYKNHDH; LGHTQEDSYKNHDHD; GHTQEDSYKNHDHDL;<br>HTQEDSYKNHDHDLD; TQEDSYKNHDHDLDF; QEDSYKNHDHDLDFS;<br>EDSYKNHDHDLDFSR; DSYKNHDHDLDFSRI; SYKNHDHDLDFSRIN;<br>YKNHDHDLDFSRINF; KNHDHDLDFSRINFK; NHDHDLDFSRINFKG;<br>HDHDLDFSRINFKGK; DHDLDFSRINFKGKL; HDLDFSRINFKGKLV;<br>DLDFSRINFKGKLVK; LDFSRINFKGKLVKR; DFSRINFKGKLVKRM;<br>FSRINFKGKLVKRMT; SRINFKGKLVKRMTM; RINFKGKLVKRMTMI;<br>INFKGKLVKRMTMIY; NFKGKLVKRMTMIYR; FKGKLVKRMTMIYRS;<br>KGKLVKRMTMIYRSA; GKLVKRMTMIYRSAD; KLVKRMTMIYRSADT;<br>LVKRMTMIYRSADTW; VKRMTMIYRSADTWY; KRMTMIYRSADTWYT;<br>RMTMIYRSADTWYTW; MTMIYRSADTWYTWY; TMIYRSADTWYTWYW;<br>MIYRSADTWYTWYWA; IYRSADTWYTWYWAE; YRSADTWYTWYWAEE;<br>RSADTWYTWYWAEEI; SADTWYTWYWAEEIM; ADTWYTWYWAEEIMS; |
|---|---|

DTWYTWYWAEEIMSP; TWYTWYWAEEIMSPF; WYTWYWAEEIMSPFI;
YTWYWAEEIMSPFIT; TWYWAEEIMSPFITG; WYWAEEIMSPFITGY;
YWAEEIMSPFITGYS; WAEEIMSPFITGYSY; AEEIMSPFITGYSYE;
EEIMSPFITGYSYEE; EIMSPFITGYSYEEI; IMSPFITGYSYEEID;
MSPFITGYSYEEIDP; SPFITGYSYEEIDPE; PFITGYSYEEIDPEY;
FITGYSYEEIDPEYL; ITGYSYEEIDPEYLL; TGYSYEEIDPEYLLP;
GYSYEEIDPEYLLPP; YSYEEIDPEYLLPPF; SYEEIDPEYLLPPFT;
YEEIDPEYLLPPFTS; EEIDPEYLLPPFTSQ; EIDPEYLLPPFTSQS;
IDPEYLLPPFTSQSG; DPEYLLPPFTSQSGS; PEYLLPPFTSQSGSK;
EYLLPPFTSQSGSKE; YLLPPFTSQSGSKET; LLPPFTSQSGSKETK;
LPPFTSQSGSKETKP; PPFTSQSGSKETKPK; PFTSQSGSKETKPKN;
FTSQSGSKETKPKNT; TSQSGSKETKPKNTC; SQSGSKETKPKNTCY;
QSGSKETKPKNTCYI; SGSKETKPKNTCYIS; GSKETKPKNTCYISF;
SKETKPKNTCYISFY; KETKPKNTCYISFYR; ETKPKNTCYISFYRY;
TKPKNTCYISFYRYD; KPKNTCYISFYRYDL;

16 mers:
MITQQDYTEIKKKLEN; ITQQDYTEIKKKLENT; TQQDYTEIKKKLENTK;
QQDYTEIKKKLENTKT; QDYTEIKKKLENTKTR; DYTEIKKKLENTKTRI;
YTEIKKKLENTKTRIQ; TEIKKKLENTKTRIQF; EIKKKLENTKTRIQFR;
IKKKLENTKTRIQFRN; KKKLENTKTRIQFRNG; KKLENTKTRIQFRNGN;
KLENTKTRIQFRNGNK; LENTKTRIQFRNGNKI; ENTKTRIQFRNGNKID;
NTKTRIQFRNGNKIDS; TKTRIQFRNGNKIDSL; KTRIQFRNGNKIDSLG;
TRIQFRNGNKIDSLGY; RIQFRNGNKIDSLGYQ; IQFRNGNKIDSLGYQG;
QFRNGNKIDSLGYQGS; FRNGNKIDSLGYQGSL; RNGNKIDSLGYQGSLG;
NGNKIDSLGYQGSLGE; GNKIDSLGYQGSLGEP; NKIDSLGYQGSLGEPI;
KIDSLGYQGSLGEPIL; IDSLGYQGSLGEPILL; DSLGYQGSLGEPILLK;
SLGYQGSLGEPILLKD; LGYQGSLGEPILLKDK; GYQGSLGEPILLKDKL;
YQGSLGEPILLKDKLT; QGSLGEPILLKDKLTF; GSLGEPILLKDKLTFS;
SLGEPILLKDKLTFSI; LGEPILLKDKLTFSIC; GEPILLKDKLTFSICD;
EPILLKDKLTFSICDG; PILLKDKLTFSICDGK; ILLKDKLTFSICDGKN;
LLKDKLTFSICDGKNI; LKDKLTFSICDGKNID; KDKLTFSICDGKNIDN;
DKLTFSICDGKNIDNR; KLTFSICDGKNIDNRK; LTFSICDGKNIDNRKE;
TFSICDGKNIDNRKEY; FSICDGKNIDNRKEYE; SICDGKNIDNRKEYEI;
ICDGKNIDNRKEYEIT; CDGKNIDNRKEYEITK; DGKNIDNRKEYEITKN;
GKNIDNRKEYEITKNA; KNIDNRKEYEITKNAP; NIDNRKEYEITKNAPS;
IDNRKEYEITKNAPSL; DNRKEYEITKNAPSLD; NRKEYEITKNAPSLDS;
RKEYEITKNAPSLDSL; KEYEITKNAPSLDSLI; EYEITKNAPSLDSLIE;
YEITKNAPSLDSLIEY; EITKNAPSLDSLIEYL; ITKNAPSLDSLIEYLE;
TKNAPSLDSLIEYLEH; KNAPSLDSLIEYLEHM; NAPSLDSLIEYLEHMI;
APSLDSLIEYLEHMIL; PSLDSLIEYLEHMILF; SLDSLIEYLEHMILFF;
LDSLIEYLEHMILFFL; DSLIEYLEHMILFFLE; SLIEYLEHMILFFLEK;
LIEYLEHMILFFLEKT; IEYLEHMILFFLEKTP; EYLEHMILFFLEKTPV;
YLEHMILFFLEKTPVG; LEHMILFFLEKTPVGT; EHMILFFLEKTPVGTL;
HMILFFLEKTPVGTLT; MILFFLEKTPVGTLTA; ILFFLEKTPVGTLTAK;
LFFLEKTPVGTLTAKF; FFLEKTPVGTLTAKFA; FLEKTPVGTLTAKFAM;
LEKTPVGTLTAKFAME; EKTPVGTLTAKFAMEG; KTPVGTLTAKFAMEGS;
TPVGTLTAKFAMEGSI; PVGTLTAKFAMEGSIL; VGTLTAKFAMEGSILT;
GTLTAKFAMEGSILTR; TLTAKFAMEGSILTRS; LTAKFAMEGSILTRSK;
TAKFAMEGSILTRSKL; AKFAMEGSILTRSKLI; KFAMEGSILTRSKLIQ;
FAMEGSILTRSKLIQA; AMEGSILTRSKLIQAF; MEGSILTRSKLIQAFT;

EGSILTRSKLIQAFTN; GSILTRSKLIQAFTNT; SILTRSKLIQAFTNTN;
ILTRSKLIQAFTNTNK; LTRSKLIQAFTNTNKF; TRSKLIQAFTNTNKFC;
RSKLIQAFTNTNKFCI; SKLIQAFTNTNKFCIP; KLIQAFTNTNKFCIPD;
LIQAFTNTNKFCIPDG; IQAFTNTNKFCIPDGR; QAFTNTNKFCIPDGRS;
AFTNTNKFCIPDGRSL; FTNTNKFCIPDGRSLP; TNTNKFCIPDGRSLPS;
NTNKFCIPDGRSLPSN; TNKFCIPDGRSLPSNC; NKFCIPDGRSLPSNCY;
KFCIPDGRSLPSNCYA; FCIPDGRSLPSNCYAY; CIPDGRSLPSNCYAYK;
IPDGRSLPSNCYAYKV; PDGRSLPSNCYAYKVL; DGRSLPSNCYAYKVLG;
GRSLPSNCYAYKVLGI; RSLPSNCYAYKVLGIS; SLPSNCYAYKVLGISS;
LPSNCYAYKVLGISSA; PSNCYAYKVLGISSAP; SNCYAYKVLGISSAPK;
NCYAYKVLGISSAPKL; CYAYKVLGISSAPKLS; YAYKVLGISSAPKLSG;
AYKVLGISSAPKLSGR; YKVLGISSAPKLSGRF; KVLGISSAPKLSGRFL;
VLGISSAPKLSGRFLR; LGISSAPKLSGRFLRH; GISSAPKLSGRFLRHY;
ISSAPKLSGRFLRHYD; SSAPKLSGRFLRHYDS; SAPKLSGRFLRHYDSG;
APKLSGRFLRHYDSGG; PKLSGRFLRHYDSGGS; KLSGRFLRHYDSGGST;
LSGRFLRHYDSGGSTD; SGRFLRHYDSGGSTDS; GRFLRHYDSGGSTDSD;
RFLRHYDSGGSTDSDG; FLRHYDSGGSTDSDGT; LRHYDSGGSTDSDGTR;
RHYDSGGSTDSDGTRS; HYDSGGSTDSDGTRSL; YDSGGSTDSDGTRSLG;
DSGGSTDSDGTRSLGH; SGGSTDSDGTRSLGHT; GGSTDSDGTRSLGHTQ;
GSTDSDGTRSLGHTQE; STDSDGTRSLGHTQED; TDSDGTRSLGHTQEDS;
DSDGTRSLGHTQEDSY; SDGTRSLGHTQEDSYK; DGTRSLGHTQEDSYKN;
GTRSLGHTQEDSYKNH; TRSLGHTQEDSYKNHD; RSLGHTQEDSYKNHDH;
SLGHTQEDSYKNHDHD; LGHTQEDSYKNHDHDL; GHTQEDSYKNHDHDLD;
HTQEDSYKNHDHDLDF; TQEDSYKNHDHDLDFS; QEDSYKNHDHDLDFSR;
EDSYKNHDHDLDFSRI; DSYKNHDHDLDFSRIN; SYKNHDHDLDFSRINF;
YKNHDHDLDFSRINFK; KNHDHDLDFSRINFKG; NHDHDLDFSRINFKGK;
HDHDLDFSRINFKGKL; DHDLDFSRINFKGKLV; HDLDFSRINFKGKLVK;
DLDFSRINFKGKLVKR; LDFSRINFKGKLVKRM; DFSRINFKGKLVKRMT;
FSRINFKGKLVKRMTM; SRINFKGKLVKRMTMI; RINFKGKLVKRMTMIY;
INFKGKLVKRMTMIYR; NFKGKLVKRMTMIYRS; FKGKLVKRMTMIYRSA;
KGKLVKRMTMIYRSAD; GKLVKRMTMIYRSADT; KLVKRMTMIYRSADTW;
LVKRMTMIYRSADTWY; VKRMTMIYRSADTWYT; KRMTMIYRSADTWYTW;
RMTMIYRSADTWYTWY; MTMIYRSADTWYTWYW; TMIYRSADTWYTWYWA;
MIYRSADTWYTWYWAE; IYRSADTWYTWYWAEE; YRSADTWYTWYWAEEI;
RSADTWYTWYWAEEIM; SADTWYTWYWAEEIMS; ADTWYTWYWAEEIMSP;
DTWYTWYWAEEIMSPF; TWYTWYWAEEIMSPFI; WYTWYWAEEIMSPFIT;
YTWYWAEEIMSPFITG; TWYWAEEIMSPFITGY; WYWAEEIMSPFITGYS;
YWAEEIMSPFITGYSY; WAEEIMSPFITGYSYE; AEEIMSPFITGYSYEE;
EEIMSPFITGYSYEEI; EIMSPFITGYSYEEID; IMSPFITGYSYEEIDP;
MSPFITGYSYEEIDPE; SPFITGYSYEEIDPEY; PFITGYSYEEIDPEYL;
FITGYSYEEIDPEYLL; ITGYSYEEIDPEYLLP; TGYSYEEIDPEYLLPP;
GYSYEEIDPEYLLPPF; YSYEEIDPEYLLPPFT; SYEEIDPEYLLPPFTS;
YEEIDPEYLLPPFTSQ; EEIDPEYLLPPFTSQS; EIDPEYLLPPFTSQSG;
IDPEYLLPPFTSQSGS; DPEYLLPPFTSQSGSK; PEYLLPPFTSQSGSKE;
EYLLPPFTSQSGSKET; YLLPPFTSQSGSKETK; LLPPFTSQSGSKETKP;
LPPFTSQSGSKETKPK; PPFTSQSGSKETKPKN; PFTSQSGSKETKPKNT;
FTSQSGSKETKPKNTC; TSQSGSKETKPKNTCY; SQSGSKETKPKNTCYI;
QSGSKETKPKNTCYIS; SGSKETKPKNTCYISF; GSKETKPKNTCYISFY;
SKETKPKNTCYISFYR; KETKPKNTCYISFYRY; ETKPKNTCYISFYRYD;
TKPKNTCYISFYRYDL;

| Seq 30 | |
|---|---|
| | 13 mers:<br>MDLLDLLEKEKQI; DLLDLLEKEKQIN; LLDLLEKEKQINK;<br>LDLLEKEKQINKN; DLLEKEKQINKNK; LLEKEKQINKNKG;<br>LEKEKQINKNKGV; EKEKQINKNKGVF; KEKQINKNKGVFM;<br>EKQINKNKGVFMT; KQINKNKGVFMTK; QINKNKGVFMTKP;<br>INKNKGVFMTKPK; NKNKGVFMTKPKI; KNKGVFMTKPKIF;<br>NKGVFMTKPKIFS; KGVFMTKPKIFSI; GVFMTKPKIFSIN;<br>VFMTKPKIFSINK; FMTKPKIFSINKE; MTKPKIFSINKEK;<br>TKPKIFSINKEKI; KPKIFSINKEKIK; PKIFSINKEKIKI;<br>KIFSINKEKIKIL; IFSINKEKIKILI; FSINKEKIKILII;<br>SINKEKIKILIIV; INKEKIKILIIVV; NKEKIKILIIVVL;<br>KEKIKILIIVVLT; EKIKILIIVVLTS; KIKILIIVVLTST;<br>IKILIIVVLTSTF; KILIIVVLTSTFL; ILIIVVLTSTFLL;<br>LIIVVLTSTFLLG; IIVVLTSTFLLGI; IVVLTSTFLLGII;<br>VVLTSTFLLGIIF; VLTSTFLLGIIFS; LTSTFLLGIIFSN;<br>TSTFLLGIIFSNE; STFLLGIIFSNEN; TFLLGIIFSNENK;<br>FLLGIIFSNENKV; LLGIIFSNENKVA; LGIIFSNENKVAR;<br>GIIFSNENKVARI; IIFSNENKVARIL; IFSNENKVARILE;<br>FSNENKVARILEE; SNENKVARILEEK; NENKVARILEEKF;<br>ENKVARILEEKFF; NKVARILEEKFFD; KVARILEEKFFDF;<br>VARILEEKFFDFD; ARILEEKFFDFDF; RILEEKFFDFDFN;<br>ILEEKFFDFDFNL; LEEKFFDFDFNLI; EEKFFDFDFNLIS;<br>EKFFDFDFNLISK; KFFDFDFNLISKI; FFDFDFNLISKIE;<br>FDFDFNLISKIET; DFDFNLISKIETE; FDFNLISKIETEL;<br>DFNLISKIETELE; FNLISKIETELEG; NLISKIETELEGT;<br>LISKIETELEGTL; ISKIETELEGTLT; SKIETELEGTLTK;<br>KIETELEGTLTKL; IETELEGTLTKLG; ETELEGTLTKLGK;<br>TELEGTLTKLGKD; ELEGTLTKLGKDW; LEGTLTKLGKDWI;<br>EGTLTKLGKDWIL; GTLTKLGKDWILT; TLTKLGKDWILTY;<br>LTKLGKDWILTYN; TKLGKDWILTYNK; KLGKDWILTYNKQ;<br>LGKDWILTYNKQN; GKDWILTYNKQNI; KDWILTYNKQNIP;<br>DWILTYNKQNIPV; WILTYNKQNIPVD; ILTYNKQNIPVDN;<br>LTYNKQNIPVDNK; TYNKQNIPVDNKK; YNKQNIPVDNKKV;<br>NKQNIPVDNKKVN; KQNIPVDNKKVNS; QNIPVDNKKVNSL;<br>NIPVDNKKVNSLI; IPVDNKKVNSLIK; PVDNKKVNSLIKA;<br>VDNKKVNSLIKAL; DNKKVNSLIKALD; NKKVNSLIKALDE;<br>KKVNSLIKALDEL; KVNSLIKALDELQ; VNSLIKALDELQK;<br>NSLIKALDELQKN; SLIKALDELQKNK; LIKALDELQKNKL;<br>IKALDELQKNKLV; KALDELQKNKLVS; ALDELQKNKLVSR;<br>LDELQKNKLVSRD; DELQKNKLVSRDQ; ELQKNKLVSRDQK;<br>LQKNKLVSRDQKK; QKNKLVSRDQKKH; KNKLVSRDQKKHK;<br>NKLVSRDQKKHKE; KLVSRDQKKHKEL; LVSRDQKKHKELG;<br>VSRDQKKHKELGI; SRDQKKHKELGIG; RDQKKHKELGIGE;<br>DQKKHKELGIGEN; QKKHKELGIGENP; KKHKELGIGENPS;<br>KHKELGIGENPSF; HKELGIGENPSFK; KELGIGENPSFKL;<br>ELGIGENPSFKLF; LGIGENPSFKLFD; GIGENPSFKLFDN;<br>IGENPSFKLFDNN; GENPSFKLFDNNN; ENPSFKLFDNNNK;<br>NPSFKLFDNNNKL; PSFKLFDNNNKLL; SFKLFDNNNKLLT;<br>FKLFDNNNKLLTE; KLFDNNNKLLTEI; LFDNNNKLLTEIF;<br>FDNNNKLLTEIFV; DNNNKLLTEIFVG; NNNKLLTEIFVGK; |

| | |
|---|---|
| NNKLLTEIFVGKS; NKLLTEIFVGKSG; KLLTEIFVGKSGE; | |
| LLTEIFVGKSGEG; LTEIFVGKSGEGD; TEIFVGKSGEGDS; | |
| EIFVGKSGEGDSR; IFVGKSGEGDSRL; FVGKSGEGDSRLA; | |
| VGKSGEGDSRLAY; GKSGEGDSRLAYI; KSGEGDSRLAYIK; | |
| SGEGDSRLAYIKG; GEGDSRLAYIKGS; EGDSRLAYIKGSD; | |
| GDSRLAYIKGSDE; DSRLAYIKGSDEN; SRLAYIKGSDENV; | |
| RLAYIKGSDENVY; LAYIKGSDENVYL; AYIKGSDENVYLT; | |
| YIKGSDENVYLTK; IKGSDENVYLTKN; KGSDENVYLTKNI; | |
| GSDENVYLTKNIF; SDENVYLTKNIFL; DENVYLTKNIFLS; | |
| ENVYLTKNIFLSY; NVYLTKNIFLSYK; VYLTKNIFLSYKG; | |
| YLTKNIFLSYKGN; LTKNIFLSYKGNS; TKNIFLSYKGNSY; | |
| KNIFLSYKGNSYN; NIFLSYKGNSYNT; IFLSYKGNSYNTF; | |
| FLSYKGNSYNTFS; LSYKGNSYNTFSD; SYKGNSYNTFSDT; | |
| YKGNSYNTFSDTT; KGNSYNTFSDTTL; GNSYNTFSDTTLF; | |
| NSYNTFSDTTLFQ; SYNTFSDTTLFQE; YNTFSDTTLFQEK; | |
| NTFSDTTLFQEKN; TFSDTTLFQEKNT; FSDTTLFQEKNTK; | |
| SDTTLFQEKNTKL; DTTLFQEKNTKLE; TTLFQEKNTKLEN; | |
| TLFQEKNTKLENL; LFQEKNTKLENLS; FQEKNTKLENLSF; | |
| QEKNTKLENLSFK; EKNTKLENLSFKI; KNTKLENLSFKII; | |
| NTKLENLSFKIIR; TKLENLSFKIIRK; KLENLSFKIIRKL; | |
| LENLSFKIIRKLN; ENLSFKIIRKLNK; NLSFKIIRKLNKE; | |
| LSFKIIRKLNKEN; SFKIIRKLNKENE; FKIIRKLNKENEN; | |
| KIIRKLNKENENN; IIRKLNKENENNI; IRKLNKENENNIN; | |
| RKLNKENENNINN; KLNKENENNINNN; LNKENENNINNNY; | |
| NKENENNINNNYE; KENENNINNNYEI; ENENNINNNYEII; | |
| NENNINNNYEIIS; ENNINNNYEIISK; NNINNNYEIISKD; | |
| NINNNYEIISKDG; INNNYEIISKDGL; NNNYEIISKDGLY; | |
| NNYEIISKDGLYF; NYEIISKDGLYFL; YEIISKDGLYFLN; | |
| EIISKDGLYFLNN; IISKDGLYFLNNQ; ISKDGLYFLNNQK; | |
| SKDGLYFLNNQKM; KDGLYFLNNQKMT; DGLYFLNNQKMTK; | |
| GLYFLNNQKMTKE; LYFLNNQKMTKER; YFLNNQKMTKERP; | |
| FLNNQKMTKERPL; LNNQKMTKERPLN; NNQKMTKERPLNI; | |
| NQKMTKERPLNII; QKMTKERPLNIIA; KMTKERPLNIIAE; | |
| MTKERPLNIIAEF; TKERPLNIIAEFK; KERPLNIIAEFKA; | |
| ERPLNIIAEFKAD; RPLNIIAEFKADG; PLNIIAEFKADGL; | |
| LNIIAEFKADGLE; NIIAEFKADGLEI; IIAEFKADGLEID; | |
| IAEFKADGLEIDK; AEFKADGLEIDKS; EFKADGLEIDKSK; | |
| FKADGLEIDKSKI; KADGLEIDKSKID; ADGLEIDKSKIDD; | |
| DGLEIDKSKIDDY; GLEIDKSKIDDYN; LEIDKSKIDDYNL; | |
| EIDKSKIDDYNLQ; IDKSKIDDYNLQY; DKSKIDDYNLQYK; | |
| KSKIDDYNLQYKI; SKIDDYNLQYKIE; KIDDYNLQYKIEV; | |
| IDDYNLQYKIEVK; DDYNLQYKIEVKW; DYNLQYKIEVKWS; | |
| YNLQYKIEVKWSN; NLQYKIEVKWSNK; LQYKIEVKWSNKS; | |
| QYKIEVKWSNKSV; YKIEVKWSNKSVN; KIEVKWSNKSVNN; | |
| IEVKWSNKSVNNI; EVKWSNKSVNNIE; VKWSNKSVNNIEV; | |
| KWSNKSVNNIEVY; WSNKSVNNIEVYF; SNKSVNNIEVYFN; | |
| NKSVNNIEVYFNK; KSVNNIEVYFNKN; SVNNIEVYFNKNE; | |
| VNNIEVYFNKNEE; NNIEVYFNKNEEN; NIEVYFNKNEEND; | |
| IEVYFNKNEENDK; EVYFNKNEENDKD; VYFNKNEENDKDI; | |
| YFNKNEENDKDIL; FNKNEENDKDILI; NKNEENDKDILIK; | |
| KNEENDKDILIKK; NEENDKDILIKKD; EENDKDILIKKDK; | |

EP 2 254 592 B1

ENDKDILIKKDKD; NDKDILIKKDKDE; DKDILIKKDKDEY;
KDILIKKDKDEYY; DILIKKDKDEYYY; ILIKKDKDEYYYT;
LIKKDKDEYYYT; IKKDKDEYYYTTS; KKDKDEYYYTTSK;
KDKDEYYYTTSKW; DKDEYYYTTSKWT; KDEYYYTTSKWTF;
DEYYYTTSKWTFF; EYYYTTSKWTFFD; YYYTTSKWTFFDV;
YYTTSKWTFFDVF; YTTSKWTFFDVFD; TTSKWTFFDVFDL;
TSKWTFFDVFDLE; SKWTFFDVFDLEK; KWTFFDVFDLEKK;
WTFFDVFDLEKKL; TFFDVFDLEKKLT; FFDVFDLEKKLTE;
FDVFDLEKKLTEK; DVFDLEKKLTEKD; VFDLEKKLTEKDD;
FDLEKKLTEKDDI; DLEKKLTEKDDIS; LEKKLTEKDDISS;
EKKLTEKDDISSN; KKLTEKDDISSND; KLTEKDDISSNDN;
LTEKDDISSNDNQ; TEKDDISSNDNQE; EKDDISSNDNQED;
KDDISSNDNQEDH; DDISSNDNQEDHH; DISSNDNQEDHHE;
ISSNDNQEDHHEH; SSNDNQEDHHEHH; SNDNQEDHHEHHN;
NDNQEDHHEHHNN; DNQEDHHEHHNNA; NQEDHHEHHNNAD;

14 mers:
MDLLDLLEKEKQIN; DLLDLLEKEKQINK; LLDLLEKEKQINKN;
LDLLEKEKQINKNK; DLLEKEKQINKNKG; LLEKEKQINKNKGV;
LEKEKQINKNKGVF; EKEKQINKNKGVFM; KEKQINKNKGVFMT;
EKQINKNKGVFMTK; KQINKNKGVFMTKP; QINKNKGVFMTKPK;
INKNKGVFMTKPKI; NKNKGVFMTKPKIF; KNKGVFMTKPKIFS;
NKGVFMTKPKIFSI; KGVFMTKPKIFSIN; GVFMTKPKIFSINK;
VFMTKPKIFSINKE; FMTKPKIFSINKEK; MTKPKIFSINKEKI;
TKPKIFSINKEKIK; KPKIFSINKEKIKI; PKIFSINKEKIKIL;
KIFSINKEKIKILI; IFSINKEKIKILII; FSINKEKIKILIIV;
SINKEKIKILIIVV; INKEKIKILIIVVL; NKEKIKILIIVVLT;
KEKIKILIIVVLTS; EKIKILIIVVLTST; KIKILIIVVLTSTF;
IKILIIVVLTSTFL; KILIIVVLTSTFLL; ILIIVVLTSTFLLG;
LIIVVLTSTFLLGI; IIVVLTSTFLLGII; IVVLTSTFLLGIIF;
VVLTSTFLLGIIFS; VLTSTFLLGIIFSN; LTSTFLLGIIFSNE;
TSTFLLGIIFSNEN; STFLLGIIFSNENK; TFLLGIIFSNENKV;
FLLGIIFSNENKVA; LLGIIFSNENKVAR; LGIIFSNENKVARI;
GIIFSNENKVARIL; IIFSNENKVARILE; IFSNENKVARILEE;
FSNENKVARILEEK; SNENKVARILEEKF; NENKVARILEEKFF;
ENKVARILEEKFFD; NKVARILEEKFFDF; KVARILEEKFFDFD;
VARILEEKFFDFDF; ARILEEKFFDFDFN; RILEEKFFDFDFNL;
ILEEKFFDFDFNLI; LEEKFFDFDFNLIS; EEKFFDFDFNLISK;
EKFFDFDFNLISKI; KFFDFDFNLISKIE; FFDFDFNLISKIET;
FDFDFNLISKIETE; DFDFNLISKIETEL; FDFNLISKIETELE;
DFNLISKIETELEG; FNLISKIETELEGT; NLISKIETELEGTL;
LISKIETELEGTLT; ISKIETELEGTLTK; SKIETELEGTLTKL;
KIETELEGTLTKLG; IETELEGTLTKLGK; ETELEGTLTKLGKD;
TELEGTLTKLGKDW; ELEGTLTKLGKDWI; LEGTLTKLGKDWIL;
EGTLTKLGKDWILT; GTLTKLGKDWILTY; TLTKLGKDWILTYN;
LTKLGKDWILTYNK; TKLGKDWILTYNKQ; KLGKDWILTYNKQN;
LGKDWILTYNKQNI; GKDWILTYNKQNIP; KDWILTYNKQNIPV;
DWILTYNKQNIPVD; WILTYNKQNIPVDN; ILTYNKQNIPVDNK;
LTYNKQNIPVDNKK; TYNKQNIPVDNKKV; YNKQNIPVDNKKVN;
NKQNIPVDNKKVNS; KQNIPVDNKKVNSL; QNIPVDNKKVNSLI;
NIPVDNKKVNSLIK; IPVDNKKVNSLIKA; PVDNKKVNSLIKAL;

| | | |
|---|---|---|
| VDNKKVNSLIKALD; | DNKKVNSLIKALDE; | NKKVNSLIKALDEL; |
| KKVNSLIKALDELQ; | KVNSLIKALDELQK; | VNSLIKALDELQKN; |
| NSLIKALDELQKNK; | SLIKALDELQKNKL; | LIKALDELQKNKLV; |
| IKALDELQKNKLVS; | KALDELQKNKLVSR; | ALDELQKNKLVSRD; |
| LDELQKNKLVSRDQ; | DELQKNKLVSRDQK; | ELQKNKLVSRDQKK; |
| LQKNKLVSRDQKKH; | QKNKLVSRDQKKHK; | KNKLVSRDQKKHKE; |
| NKLVSRDQKKHKEL; | KLVSRDQKKHKELG; | LVSRDQKKHKELGI; |
| VSRDQKKHKELGIG; | SRDQKKHKELGIGE; | RDQKKHKELGIGEN; |
| DQKKHKELGIGENP; | QKKHKELGIGENPS; | KKHKELGIGENPSF; |
| KHKELGIGENPSFK; | HKELGIGENPSFKL; | KELGIGENPSFKLF; |
| ELGIGENPSFKLFD; | LGIGENPSFKLFDN; | GIGENPSFKLFDNN; |
| IGENPSFKLFDNNN; | GENPSFKLFDNNNK; | ENPSFKLFDNNNKL; |
| NPSFKLFDNNNKLL; | PSFKLFDNNNKLLT; | SFKLFDNNNKLLTE; |
| FKLFDNNNKLLTEI; | KLFDNNNKLLTEIF; | LFDNNNKLLTEIFV; |
| FDNNNKLLTEIFVG; | DNNNKLLTEIFVGK; | NNNKLLTEIFVGKS; |
| NNKLLTEIFVGKSG; | NKLLTEIFVGKSGE; | KLLTEIFVGKSGEG; |
| LLTEIFVGKSGEGD; | LTEIFVGKSGEGDS; | TEIFVGKSGEGDSR; |
| EIFVGKSGEGDSRL; | IFVGKSGEGDSRLA; | FVGKSGEGDSRLAY; |
| VGKSGEGDSRLAYI; | GKSGEGDSRLAYIK; | KSGEGDSRLAYIKG; |
| SGEGDSRLAYIKGS; | GEGDSRLAYIKGSD; | EGDSRLAYIKGSDE; |
| GDSRLAYIKGSDEN; | DSRLAYIKGSDENV; | SRLAYIKGSDENVY; |
| RLAYIKGSDENVYL; | LAYIKGSDENVYLT; | AYIKGSDENVYLTK; |
| YIKGSDENVYLTKN; | IKGSDENVYLTKNI; | KGSDENVYLTKNIF; |
| GSDENVYLTKNIFL; | SDENVYLTKNIFLS; | DENVYLTKNIFLSY; |
| ENVYLTKNIFLSYK; | NVYLTKNIFLSYKG; | VYLTKNIFLSYKGN; |
| YLTKNIFLSYKGNS; | LTKNIFLSYKGNSY; | TKNIFLSYKGNSYN; |
| KNIFLSYKGNSYNT; | NIFLSYKGNSYNTF; | IFLSYKGNSYNTFS; |
| FLSYKGNSYNTFSD; | LSYKGNSYNTFSDT; | SYKGNSYNTFSDTT; |
| YKGNSYNTFSDTTL; | KGNSYNTFSDTTLF; | GNSYNTFSDTTLFQ; |
| NSYNTFSDTTLFQE; | SYNTFSDTTLFQEK; | YNTFSDTTLFQEKN; |
| NTFSDTTLFQEKNT; | TFSDTTLFQEKNTK; | FSDTTLFQEKNTKL; |
| SDTTLFQEKNTKLE; | DTTLFQEKNTKLEN; | TTLFQEKNTKLENL; |
| TLFQEKNTKLENLS; | LFQEKNTKLENLSF; | FQEKNTKLENLSFK; |
| QEKNTKLENLSFKI; | EKNTKLENLSFKII; | KNTKLENLSFKIIR; |
| NTKLENLSFKIIRK; | TKLENLSFKIIRKL; | KLENLSFKIIRKLN; |
| LENLSFKIIRKLNK; | ENLSFKIIRKLNKE; | NLSFKIIRKLNKEN; |
| LSFKIIRKLNKENE; | SFKIIRKLNKENEN; | FKIIRKLNKENENN; |
| KIIRKLNKENENNI; | IIRKLNKENENNIN; | IRKLNKENENNINN; |
| RKLNKENENNINNN; | KLNKENENNINNNY; | LNKENENNINNNYE; |
| NKENENNINNNYEI; | KENENNINNNYEII; | ENENNINNNYEIIS; |
| NENNINNNYEIISK; | ENNINNNYEIISKD; | NNINNNYEIISKDG; |
| NINNNYEIISKDGL; | INNNYEIISKDGLY; | NNNYEIISKDGLYF; |
| NNYEIISKDGLYFL; | NYEIISKDGLYFLN; | YEIISKDGLYFLNN; |
| EIISKDGLYFLNNQ; | IISKDGLYFLNNQK; | ISKDGLYFLNNQKM; |
| SKDGLYFLNNQKMT; | KDGLYFLNNQKMTK; | DGLYFLNNQKMTKE; |
| GLYFLNNQKMTKER; | LYFLNNQKMTKERP; | YFLNNQKMTKERPL; |
| FLNNQKMTKERPLN; | LNNQKMTKERPLNI; | NNQKMTKERPLNII; |
| NQKMTKERPLNIIA; | QKMTKERPLNIIAE; | KMTKERPLNIIAEF; |
| MTKERPLNIIAEFK; | TKERPLNIIAEFKA; | KERPLNIIAEFKAD; |
| ERPLNIIAEFKADG; | RPLNIIAEFKADGL; | PLNIIAEFKADGLE; |
| LNIIAEFKADGLEI; | NIIAEFKADGLEID; | IIAEFKADGLEIDK; |

IAEFKADGLEIDKS; AEFKADGLEIDKSK; EFKADGLEIDKSKI;
FKADGLEIDKSKID; KADGLEIDKSKIDD; ADGLEIDKSKIDDY;
DGLEIDKSKIDDYN; GLEIDKSKIDDYNL; LEIDKSKIDDYNLQ;
EIDKSKIDDYNLQY; IDKSKIDDYNLQYK; DKSKIDDYNLQYKI;
KSKIDDYNLQYKIE; SKIDDYNLQYKIEV; KIDDYNLQYKIEVK;
IDDYNLQYKIEVKW; DDYNLQYKIEVKWS; DYNLQYKIEVKWSN;
YNLQYKIEVKWSNK; NLQYKIEVKWSNKS; LQYKIEVKWSNKSV;
QYKIEVKWSNKSVN; YKIEVKWSNKSVNN; KIEVKWSNKSVNNI;
IEVKWSNKSVNNIE; EVKWSNKSVNNIEV; VKWSNKSVNNIEVY;
KWSNKSVNNIEVYF; WSNKSVNNIEVYFN; SNKSVNNIEVYFNK;
NKSVNNIEVYFKN; KSVNNIEVYFNKNE; SVNNIEVYFNKNEE;
VNNIEVYFNKNEEN; NNIEVYFNKNEEND; NIEVYFNKNEENDK;
IEVYFNKNEENDKD; EVYFNKNEENDKDI; VYFNKNEENDKDIL;
YFNKNEENDKDILI; FNKNEENDKDILIK; NKNEENDKDILIKK;
KNEENDKDILIKKD; NEENDKDILIKKDK; EENDKDILIKKDKD;
ENDKDILIKKDKDE; NDKDILIKKDKDEY; DKDILIKKDKDEYY;
KDILIKKDKDEYYY; DILIKKDKDEYYYT; ILIKKDKDEYYYTT;
LIKKDKDEYYYTTS; IKKDKDEYYYTTSK; KKDKDEYYYTTSKW;
KDKDEYYYTTSKWT; DKDEYYYTTSKWTF; KDEYYYTTSKWTFF;
DEYYYTTSKWTFFD; EYYYTTSKWTFFDV; YYYTTSKWTFFDVF;
YYTTSKWTFFDVFD; YTTSKWTFFDVFDL; TTSKWTFFDVFDLE;
TSKWTFFDVFDLEK; SKWTFFDVFDLEKK; KWTFFDVFDLEKKL;
WTFFDVFDLEKKLT; TFFDVFDLEKKLTE; FFDVFDLEKKLTEK;
FDVFDLEKKLTEKD; DVFDLEKKLTEKDD; VFDLEKKLTEKDDI;
FDLEKKLTEKDDIS; DLEKKLTEKDDISS; LEKKLTEKDDISSN;
EKKLTEKDDISSND; KKLTEKDDISSNDN; KLTEKDDISSNDNQ;
LTEKDDISSNDNQE; TEKDDISSNDNQED; EKDDISSNDNQEDH;
KDDISSNDNQEDHH; DDISSNDNQEDHHE; DISSNDNQEDHHEH;
ISSNDNQEDHHEHH; SSNDNQEDHHEHHN; SNDNQEDHHEHHNN;
NDNQEDHHEHHNNA; DNQEDHHEHHNNAD;

15 mers:
MDLLDLLEKEKQINK; DLLDLLEKEKQINKN; LLDLLEKEKQINKNK;
LDLLEKEKQINKNKG; DLLEKEKQINKNKGV; LLEKEKQINKNKGVF;
LEKEKQINKNKGVFM; EKEKQINKNKGVFMT; KEKQINKNKGVFMTK;
EKQINKNKGVFMTKP; KQINKNKGVFMTKPK; QINKNKGVFMTKPKI;
INKNKGVFMTKPKIF; NKNKGVFMTKPKIFS; KNKGVFMTKPKIFSI;
NKGVFMTKPKIFSIN; KGVFMTKPKIFSINK; GVFMTKPKIFSINKE;
VFMTKPKIFSINKEK; FMTKPKIFSINKEKI; MTKPKIFSINKEKIK;
TKPKIFSINKEKIKI; KPKIFSINKEKIKIL; PKIFSINKEKIKILI;
KIFSINKEKIKILII; IFSINKEKIKILIIV; FSINKEKIKILIIVV;
SINKEKIKILIIVVL; INKEKIKILIIVVLT; NKEKIKILIIVVLTS;
KEKIKILIIVVLTST; EKIKILIIVVLTSTF; KIKILIIVVLTSTFL;
IKILIIVVLTSTFLL; KILIIVVLTSTFLLG; ILIIVVLTSTFLLGI;
LIIVVLTSTFLLGII; IIVVLTSTFLLGIIF; IVVLTSTFLLGIIFS;
VVLTSTFLLGIIFSN; VLTSTFLLGIIFSNE; LTSTFLLGIIFSNEN;
TSTFLLGIIFSNENK; STFLLGIIFSNENKV; TFLLGIIFSNENKVA;
FLLGIIFSNENKVAR; LLGIIFSNENKVARI; LGIIFSNENKVARIL;
GIIFSNENKVARILE; IIFSNENKVARILEE; IFSNENKVARILEEK;
FSNENKVARILEEKF; SNENKVARILEEKFF; NENKVARILEEKFFD;
ENKVARILEEKFFDF; NKVARILEEKFFDFD; KVARILEEKFFDFDF;

| | |
|---|---|
| VARILEEKFFDFDFN; ARILEEKFFDFDFNL; RILEEKFFDFDFNLI; ILEEKFFDFDFNLIS; LEEKFFDFDFNLISK; EEKFFDFDFNLISKI; EKFFDFDFNLISKIE; KFFDFDFNLISKIET; FFDFDFNLISKIETE; FDFDFNLISKIETEL; DFDFNLISKIETELE; FDFNLISKIETELEG; DFNLISKIETELEGT; FNLISKIETELEGTL; NLISKIETELEGTLT; LISKIETELEGTLTK; ISKIETELEGTLTKL; SKIETELEGTLTKLG; KIETELEGTLTKLGK; IETELEGTLTKLGKD; ETELEGTLTKLGKDW; TELEGTLTKLGKDWI; ELEGTLTKLGKDWIL; LEGTLTKLGKDWILT; EGTLTKLGKDWILTY; GTLTKLGKDWILTYN; TLTKLGKDWILTYNK; LTKLGKDWILTYNKQ; TKLGKDWILTYNKQN; KLGKDWILTYNKQNI; LGKDWILTYNKQNIP; GKDWILTYNKQNIPV; KDWILTYNKQNIPVD; DWILTYNKQNIPVDN; WILTYNKQNIPVDNK; ILTYNKQNIPVDNKK; LTYNKQNIPVDNKKV; TYNKQNIPVDNKKVN; YNKQNIPVDNKKVNS; NKQNIPVDNKKVNSL; KQNIPVDNKKVNSLI; QNIPVDNKKVNSLIK; NIPVDNKKVNSLIKA; IPVDNKKVNSLIKAL; PVDNKKVNSLIKALD; VDNKKVNSLIKALDE; DNKKVNSLIKALDEL; NKKVNSLIKALDELQ; KKVNSLIKALDELQK; KVNSLIKALDELQKN; VNSLIKALDELQKNK; NSLIKALDELQKNKL; SLIKALDELQKNKLV; LIKALDELQKNKLVS; IKALDELQKNKLVSR; KALDELQKNKLVSRD; ALDELQKNKLVSRDQ; LDELQKNKLVSRDQK; DELQKNKLVSRDQKK; ELQKNKLVSRDQKKH; LQKNKLVSRDQKKHK; QKNKLVSRDQKKHKE; KNKLVSRDQKKHKEL; NKLVSRDQKKHKELG; KLVSRDQKKHKELGI; LVSRDQKKHKELGIG; VSRDQKKHKELGIGE; SRDQKKHKELGIGEN; RDQKKHKELGIGENP; DQKKHKELGIGENPS; QKKHKELGIGENPSF; KKHKELGIGENPSFK; KHKELGIGENPSFKL; HKELGIGENPSFKLF; KELGIGENPSFKLFD; ELGIGENPSFKLFDN; LGIGENPSFKLFDNN; GIGENPSFKLFDNNN; IGENPSFKLFDNNNK; GENPSFKLFDNNNKL; ENPSFKLFDNNNKLL; NPSFKLFDNNNKLLT; PSFKLFDNNNKLLTE; SFKLFDNNNKLLTEI; FKLFDNNNKLLTEIF; KLFDNNNKLLTEIFV; LFDNNNKLLTEIFVG; FDNNNKLLTEIFVGK; DNNNKLLTEIFVGKS; NNNKLLTEIFVGKSG; NNKLLTEIFVGKSGE; NKLLTEIFVGKSGEG; KLLTEIFVGKSGEGD; LLTEIFVGKSGEGDS; LTEIFVGKSGEGDSR; TEIFVGKSGEGDSRL; EIFVGKSGEGDSRLA; IFVGKSGEGDSRLAY; FVGKSGEGDSRLAYI; VGKSGEGDSRLAYIK; GKSGEGDSRLAYIKG; KSGEGDSRLAYIKGS; SGEGDSRLAYIKGSD; GEGDSRLAYIKGSDE; EGDSRLAYIKGSDEN; GDSRLAYIKGSDENV; DSRLAYIKGSDENVY; SRLAYIKGSDENVYL; RLAYIKGSDENVYLT; LAYIKGSDENVYLTK; AYIKGSDENVYLTKN; YIKGSDENVYLTKNI; IKGSDENVYLTKNIF; KGSDENVYLTKNIFL; GSDENVYLTKNIFLS; SDENVYLTKNIFLSY; DENVYLTKNIFLSYK; ENVYLTKNIFLSYKG; NVYLTKNIFLSYKGN; VYLTKNIFLSYKGNS; YLTKNIFLSYKGNSY; LTKNIFLSYKGNSYN; TKNIFLSYKGNSYNT; KNIFLSYKGNSYNTF; NIFLSYKGNSYNTFS; IFLSYKGNSYNTFSD; FLSYKGNSYNTFSDT; LSYKGNSYNTFSDTT; SYKGNSYNTFSDTTL; YKGNSYNTFSDTTLF; KGNSYNTFSDTTLFQ; GNSYNTFSDTTLFQE; NSYNTFSDTTLFQEK; SYNTFSDTTLFQEKN; YNTFSDTTLFQEKNT; NTFSDTTLFQEKNTK; TFSDTTLFQEKNTKL; FSDTTLFQEKNTKLE; SDTTLFQEKNTKLEN; DTTLFQEKNTKLENL; TTLFQEKNTKLENLS; TLFQEKNTKLENLSF; LFQEKNTKLENLSFK; FQEKNTKLENLSFKI; QEKNTKLENLSFKII; EKNTKLENLSFKIIR; KNTKLENLSFKIIRK; NTKLENLSFKIIRKL; TKLENLSFKIIRKLN; KLENLSFKIIRKLNK; LENLSFKIIRKLNKE; ENLSFKIIRKLNKEN; NLSFKIIRKLNKENE; | |

LSFKIIRKLNKENEN; SFKIIRKLNKENENN; FKIIRKLNKENENNI;
KIIRKLNKENENNIN; IIRKLNKENENNINN; IRKLNKENENNINNN;
RKLNKENENNINNNY; KLNKENENNINNNYE; LNKENENNINNNYEI;
NKENENNINNNYEII; KENENNINNNYEIIS; ENENNINNNYEIISK;
NENNINNNYEIISKD; ENNINNNYEIISKDG; NNINNNYEIISKDGL;
NINNNYEIISKDGLY; INNNYEIISKDGLYF; NNNYEIISKDGLYFL;
NNYEIISKDGLYFLN; NYEIISKDGLYFLNN; YEIISKDGLYFLNNQ;
EIISKDGLYFLNNQK; IISKDGLYFLNNQKM; ISKDGLYFLNNQKMT;
SKDGLYFLNNQKMTK; KDGLYFLNNQKMTKE; DGLYFLNNQKMTKER;
GLYFLNNQKMTKERP; LYFLNNQKMTKERPL; YFLNNQKMTKERPLN;
FLNNQKMTKERPLNI; LNNQKMTKERPLNII; NNQKMTKERPLNIIA;
NQKMTKERPLNIIAE; QKMTKERPLNIIAEF; KMTKERPLNIIAEFK;
MTKERPLNIIAEFKA; TKERPLNIIAEFKAD; KERPLNIIAEFKADG;
ERPLNIIAEFKADGL; RPLNIIAEFKADGLE; PLNIIAEFKADGLEI;
LNIIAEFKADGLEID; NIIAEFKADGLEIDK; IIAEFKADGLEIDKS;
IAEFKADGLEIDKSK; AEFKADGLEIDKSKI; EFKADGLEIDKSKID;
FKADGLEIDKSKIDD; KADGLEIDKSKIDDY; ADGLEIDKSKIDDYN;
DGLEIDKSKIDDYNL; GLEIDKSKIDDYNLQ; LEIDKSKIDDYNLQY;
EIDKSKIDDYNLQYK; IDKSKIDDYNLQYKI; DKSKIDDYNLQYKIE;
KSKIDDYNLQYKIEV; SKIDDYNLQYKIEVK; KIDDYNLQYKIEVKW;
IDDYNLQYKIEVKWS; DDYNLQYKIEVKWSN; DYNLQYKIEVKWSNK;
YNLQYKIEVKWSNKS; NLQYKIEVKWSNKSV; LQYKIEVKWSNKSVN;
QYKIEVKWSNKSVNN; YKIEVKWSNKSVNNI; KIEVKWSNKSVNNIE;
IEVKWSNKSVNNIEV; EVKWSNKSVNNIEVY; VKWSNKSVNNIEVYF;
KWSNKSVNNIEVYFN; WSNKSVNNIEVYFNK; SNKSVNNIEVYFNKN;
NKSVNNIEVYFNKNE; KSVNNIEVYFNKNEE; SVNNIEVYFNKNEEN;
VNNIEVYFNKNEEND; NNIEVYFNKNEENDK; NIEVYFNKNEENDKD;
IEVYFNKNEENDKDI; EVYFNKNEENDKDIL; VYFNKNEENDKDILI;
YFNKNEENDKDILIK; FNKNEENDKDILIKK; NKNEENDKDILIKKD;
KNEENDKDILIKKDK; NEENDKDILIKKDKD; EENDKDILIKKDKDE;
ENDKDILIKKDKDEY; NDKDILIKKDKDEYY; DKDILIKKDKDEYYY;
KDILIKKDKDEYYYT; DILIKKDKDEYYYTT; ILIKKDKDEYYYTTS;
LIKKDKDEYYYTTSK; IKKDKDEYYYTTSKW; KKDKDEYYYTTSKWT;
KDKDEYYYTTSKWTF; DKDEYYYTTSKWTFF; KDEYYYTTSKWTFFD;
DEYYYTTSKWTFFDV; EYYYTTSKWTFFDVF; YYYTTSKWTFFDVFD;
YYTTSKWTFFDVFDL; YTTSKWTFFDVFDLE; TTSKWTFFDVFDLEK;
TSKWTFFDVFDLEKK; SKWTFFDVFDLEKKL; KWTFFDVFDLEKKLT;
WTFFDVFDLEKKLTE; TFFDVFDLEKKLTEK; FFDVFDLEKKLTEKD;
FDVFDLEKKLTEKDD; DVFDLEKKLTEKDDI; VFDLEKKLTEKDDIS;
FDLEKKLTEKDDISS; DLEKKLTEKDDISSN; LEKKLTEKDDISSND;
EKKLTEKDDISSNDN; KKLTEKDDISSNDNQ; KLTEKDDISSNDNQE;
LTEKDDISSNDNQED; TEKDDISSNDNQEDH; EKDDISSNDNQEDHH;
KDDISSNDNQEDHHE; DDISSNDNQEDHHEH; DISSNDNQEDHHEHH;
ISSNDNQEDHHEHHN; SSNDNQEDHHEHHNN; SNDNQEDHHEHHNNA;
NDNQEDHHEHHNNAD;

16 mers:
MDLLDLLEKEKQINKN; DLLDLLEKEKQINKNK; LLDLLEKEKQINKNKG;
LDLLEKEKQINKNKGV; DLLEKEKQINKNKGVF; LLEKEKQINKNKGVFM;
LEKEKQINKNKGVFMT; EKEKQINKNKGVFMTK; KEKQINKNKGVFMTKP;
EKQINKNKGVFMTKPK; KQINKNKGVFMTKPKI; QINKNKGVFMTKPKIF;

INKNKGVFMTKPKIFS; NKNKGVFMTKPKIFSI; KNKGVFMTKPKIFSIN;
NKGVFMTKPKIFSINK; KGVFMTKPKIFSINKE; GVFMTKPKIFSINKEK;
VFMTKPKIFSINKEKI; FMTKPKIFSINKEKIK; MTKPKIFSINKEKIKI;
TKPKIFSINKEKIKIL; KPKIFSINKEKIKILI; PKIFSINKEKIKILII;
KIFSINKEKIKILIIV; IFSINKEKIKILIIVV; FSINKEKIKILIIVVL;
SINKEKIKILIIVVLT; INKEKIKILIIVVLTS; NKEKIKILIIVVLTST;
KEKIKILIIVVLTSTF; EKIKILIIVVLTSTFL; KIKILIIVVLTSTFLL;
IKILIIVVLTSTFLLG; KILIIVVLTSTFLLGI; ILIIVVLTSTFLLGII;
LIIVVLTSTFLLGIIF; IIVVLTSTFLLGIIFS; IVVLTSTFLLGIIFSN;
VVLTSTFLLGIIFSNE; VLTSTFLLGIIFSNEN; LTSTFLLGIIFSNENK;
TSTFLLGIIFSNENKV; STFLLGIIFSNENKVA; TFLLGIIFSNENKVAR;
FLLGIIFSNENKVARI; LLGIIFSNENKVARIL; LGIIFSNENKVARILE;
GIIFSNENKVARILEE; IIFSNENKVARILEEK; IFSNENKVARILEEKF;
FSNENKVARILEEKFF; SNENKVARILEEKFFD; NENKVARILEEKFFDF;
ENKVARILEEKFFDFD; NKVARILEEKFFDFDF; KVARILEEKFFDFDFN;
VARILEEKFFDFDFNL; ARILEEKFFDFDFNLI; RILEEKFFDFDFNLIS;
ILEEKFFDFDFNLISK; LEEKFFDFDFNLISKI; EEKFFDFDFNLISKIE;
EKFFDFDFNLISKIET; KFFDFDFNLISKIETE; FFDFDFNLISKIETEL;
FDFDFNLISKIETELE; DFDFNLISKIETELEG; FDFNLISKIETELEGT;
DFNLISKIETELEGTL; FNLISKIETELEGTLT; NLISKIETELEGTLTK;
LISKIETELEGTLTKL; ISKIETELEGTLTKLG; SKIETELEGTLTKLGK;
KIETELEGTLTKLGKD; IETELEGTLTKLGKDW; ETELEGTLTKLGKDWI;
TELEGTLTKLGKDWIL; ELEGTLTKLGKDWILT; LEGTLTKLGKDWILTY;
EGTLTKLGKDWILTYN; GTLTKLGKDWILTYNK; TLTKLGKDWILTYNKQ;
LTKLGKDWILTYNKQN; TKLGKDWILTYNKQNI; KLGKDWILTYNKQNIP;
LGKDWILTYNKQNIPV; GKDWILTYNKQNIPVD; KDWILTYNKQNIPVDN;
DWILTYNKQNIPVDNK; WILTYNKQNIPVDNKK; ILTYNKQNIPVDNKKV;
LTYNKQNIPVDNKKVN; TYNKQNIPVDNKKVNS; YNKQNIPVDNKKVNSL;
NKQNIPVDNKKVNSLI; KQNIPVDNKKVNSLIK; QNIPVDNKKVNSLIKA;
NIPVDNKKVNSLIKAL; IPVDNKKVNSLIKALD; PVDNKKVNSLIKALDE;
VDNKKVNSLIKALDEL; DNKKVNSLIKALDELQ; NKKVNSLIKALDELQK;
KKVNSLIKALDELQKN; KVNSLIKALDELQKNK; VNSLIKALDELQKNKL;
NSLIKALDELQKNKLV; SLIKALDELQKNKLVS; LIKALDELQKNKLVSR;
IKALDELQKNKLVSRD; KALDELQKNKLVSRDQ; ALDELQKNKLVSRDQK;
LDELQKNKLVSRDQKK; DELQKNKLVSRDQKKH; ELQKNKLVSRDQKKHK;
LQKNKLVSRDQKKHKE; QKNKLVSRDQKKHKEL; KNKLVSRDQKKHKELG;
NKLVSRDQKKHKELGI; KLVSRDQKKHKELGIG; LVSRDQKKHKELGIGE;
VSRDQKKHKELGIGEN; SRDQKKHKELGIGENP; RDQKKHKELGIGENPS;
DQKKHKELGIGENPSF; QKKHKELGIGENPSFK; KKHKELGIGENPSFKL;
KHKELGIGENPSFKLF; HKELGIGENPSFKLFD; KELGIGENPSFKLFDN;
ELGIGENPSFKLFDNN; LGIGENPSFKLFDNNN; GIGENPSFKLFDNNNK;
IGENPSFKLFDNNNKL; GENPSFKLFDNNNKLL; ENPSFKLFDNNNKLLT;
NPSFKLFDNNNKLLTE; PSFKLFDNNNKLLTEI; SFKLFDNNNKLLTEIF;
FKLFDNNNKLLTEIFV; KLFDNNNKLLTEIFVG; LFDNNNKLLTEIFVGK;
FDNNNKLLTEIFVGKS; DNNNKLLTEIFVGKSG; NNNKLLTEIFVGKSGE;
NNKLLTEIFVGKSGEG; NKLLTEIFVGKSGEGD; KLLTEIFVGKSGEGDS;
LLTEIFVGKSGEGDSR; LTEIFVGKSGEGDSRL; TEIFVGKSGEGDSRLA;
EIFVGKSGEGDSRLAY; IFVGKSGEGDSRLAYI; FVGKSGEGDSRLAYIK;
VGKSGEGDSRLAYIKG; GKSGEGDSRLAYIKGS; KSGEGDSRLAYIKGSD;
SGEGDSRLAYIKGSDE; GEGDSRLAYIKGSDEN; EGDSRLAYIKGSDENV;
GDSRLAYIKGSDENVY; DSRLAYIKGSDENVYL; SRLAYIKGSDENVYLT;

RLAYIKGSDENVYLTK; LAYIKGSDENVYLTKN; AYIKGSDENVYLTKNI;
YIKGSDENVYLTKNIF; IKGSDENVYLTKNIFL; KGSDENVYLTKNIFLS;
GSDENVYLTKNIFLSY; SDENVYLTKNIFLSYK; DENVYLTKNIFLSYKG;
ENVYLTKNIFLSYKGN; NVYLTKNIFLSYKGNS; VYLTKNIFLSYKGNSY;
YLTKNIFLSYKGNSYN; LTKNIFLSYKGNSYNT; TKNIFLSYKGNSYNTF;
KNIFLSYKGNSYNTFS; NIFLSYKGNSYNTFSD; IFLSYKGNSYNTFSDT;
FLSYKGNSYNTFSDTT; LSYKGNSYNTFSDTTL; SYKGNSYNTFSDTTLF;
YKGNSYNTFSDTTLFQ; KGNSYNTFSDTTLFQE; GNSYNTFSDTTLFQEK;
NSYNTFSDTTLFQEKN; SYNTFSDTTLFQEKNT; YNTFSDTTLFQEKNTK;
NTFSDTTLFQEKNTKL; TFSDTTLFQEKNTKLE; FSDTTLFQEKNTKLEN;
SDTTLFQEKNTKLENL; DTTLFQEKNTKLENLS; TTLFQEKNTKLENLSF;
TLFQEKNTKLENLSFK; LFQEKNTKLENLSFKI; FQEKNTKLENLSFKII;
QEKNTKLENLSFKIIR; EKNTKLENLSFKIIRK; KNTKLENLSFKIIRKL;
NTKLENLSFKIIRKLN; TKLENLSFKIIRKLNK; KLENLSFKIIRKLNKE;
LENLSFKIIRKLNKEN; ENLSFKIIRKLNKENE; NLSFKIIRKLNKENEN;
LSFKIIRKLNKENENN; SFKIIRKLNKENENNI; FKIIRKLNKENENNIN;
KIIRKLNKENENNINN; IIRKLNKENENNINNN; IRKLNKENENNINNNY;
RKLNKENENNINNNYE; KLNKENENNINNNYEI; LNKENENNINNNYEII;
NKENENNINNNYEIIS; KENENNINNNYEIISK; ENENNINNNYEIISKD;
NENNINNNYEIISKDG; ENNINNNYEIISKDGL; NNINNNYEIISKDGLY;
NINNNYEIISKDGLYF; INNNYEIISKDGLYFL; NNNYEIISKDGLYFLN;
NNYEIISKDGLYFLNN; NYEIISKDGLYFLNNQ; YEIISKDGLYFLNNQK;
EIISKDGLYFLNNQKM; IISKDGLYFLNNQKMT; ISKDGLYFLNNQKMTK;
SKDGLYFLNNQKMTKE; KDGLYFLNNQKMTKER; DGLYFLNNQKMTKERP;
GLYFLNNQKMTKERPL; LYFLNNQKMTKERPLN; YFLNNQKMTKERPLNI;
FLNNQKMTKERPLNII; LNNQKMTKERPLNIIA; NNQKMTKERPLNIIAE;
NQKMTKERPLNIIAEF; QKMTKERPLNIIAEFK; KMTKERPLNIIAEFKA;
MTKERPLNIIAEFKAD; TKERPLNIIAEFKADG; KERPLNIIAEFKADGL;
ERPLNIIAEFKADGLE; RPLNIIAEFKADGLEI; PLNIIAEFKADGLEID;
LNIIAEFKADGLEIDK; NIIAEFKADGLEIDKS; IIAEFKADGLEIDKSK;
IAEFKADGLEIDKSKI; AEFKADGLEIDKSKID; EFKADGLEIDKSKIDD;
FKADGLEIDKSKIDDY; KADGLEIDKSKIDDYN; ADGLEIDKSKIDDYNL;
DGLEIDKSKIDDYNLQ; GLEIDKSKIDDYNLQY; LEIDKSKIDDYNLQYK;
EIDKSKIDDYNLQYKI; IDKSKIDDYNLQYKIE; DKSKIDDYNLQYKIEV;
KSKIDDYNLQYKIEVK; SKIDDYNLQYKIEVKW; KIDDYNLQYKIEVKWS;
IDDYNLQYKIEVKWSN; DDYNLQYKIEVKWSNK; DYNLQYKIEVKWSNKS;
YNLQYKIEVKWSNKSV; NLQYKIEVKWSNKSVN; LQYKIEVKWSNKSVNN;
QYKIEVKWSNKSVNNI; YKIEVKWSNKSVNNIE; KIEVKWSNKSVNNIEV;
IEVKWSNKSVNNIEVY; EVKWSNKSVNNIEVYF; VKWSNKSVNNIEVYFN;
KWSNKSVNNIEVYFNK; WSNKSVNNIEVYFNKN; SNKSVNNIEVYFNKNE;
NKSVNNIEVYFNKNEE; KSVNNIEVYFNKNEEN; SVNNIEVYFNKNEEND;
VNNIEVYFNKNEENDK; NNIEVYFNKNEENDKD; NIEVYFNKNEENDKDI;
IEVYFNKNEENDKDIL; EVYFNKNEENDKDILI; VYFNKNEENDKDILIK;
YFNKNEENDKDILIKK; FNKNEENDKDILIKKD; NKNEENDKDILIKKDK;
KNEENDKDILIKKDKD; NEENDKDILIKKDKDE; EENDKDILIKKDKDEY;
ENDKDILIKKDKDEYY; NDKDILIKKDKDEYYY; DKDILIKKDKDEYYYT;
KDILIKKDKDEYYYTT; DILIKKDKDEYYYTTS; ILIKKDKDEYYYTTSK;
LIKKDKDEYYYTTSKW; IKKDKDEYYYTTSKWT; KKDKDEYYYTTSKWTF;
KDKDEYYYTTSKWTFF; DKDEYYYTTSKWTFFD; KDEYYYTTSKWTFFDV;
DEYYYTTSKWTFFDVF; EYYYTTSKWTFFDVFD; YYYTTSKWTFFDVFDL;
YYTTSKWTFFDVFDLE; YTTSKWTFFDVFDLEK; TTSKWTFFDVFDLEKK;

| | |
|---|---|
| | TSKWTFFDVFDLEKKL; SKWTFFDVFDLEKKLT; KWTFFDVFDLEKKLTE; WTFFDVFDLEKKLTEK; TFFDVFDLEKKLTEKD; FFDVFDLEKKLTEKDD; FDVFDLEKKLTEKDDI; DVFDLEKKLTEKDDIS; VFDLEKKLTEKDDISS; FDLEKKLTEKDDISSN; DLEKKLTEKDDISSND; LEKKLTEKDDISSNDN; EKKLTEKDDISSNDNQ; KKLTEKDDISSNDNQE; KLTEKDDISSNDNQED; LTEKDDISSNDNQEDH; TEKDDISSNDNQEDHH; EKDDISSNDNQEDHHE; KDDISSNDNQEDHHEH; DDISSNDNQEDHHEHH; DISSNDNQEDHHEHHN; ISSNDNQEDHHEHHNN; SSNDNQEDHHEHHNNA; SNDNQEDHHEHHNNAD; |
| Seq 31 | 13 mers:<br>MKKLIIIFTLFLS; KKLIIIFTLFLSQ; KLIIIFTLFLSQA; LIIIFTLFLSQAC; IIIFTLFLSQACN; IIFTLFLSQACNL; IFTLFLSQACNLS; FTLFLSQACNLST; TLFLSQACNLSTM; LFLSQACNLSTMH; FLSQACNLSTMHK; LSQACNLSTMHKI; SQACNLSTMHKID; QACNLSTMHKIDT; ACNLSTMHKIDTK; CNLSTMHKIDTKE; NLSTMHKIDTKED; LSTMHKIDTKEDM; STMHKIDTKEDMK; TMHKIDTKEDMKI; MHKIDTKEDMKIL; HKIDTKEDMKILY; KIDTKEDMKILYS; IDTKEDMKILYSE; DTKEDMKILYSEI; TKEDMKILYSEIA; KEDMKILYSEIAE; EDMKILYSEIAEL; DMKILYSEIAELR; MKILYSEIAELRK; KILYSEIAELRKK; ILYSEIAELRKKL; LYSEIAELRKKLN; YSEIAELRKKLNL; SEIAELRKKLNLN; EIAELRKKLNLNH; IAELRKKLNLNHL; AELRKKLNLNHLE; ELRKKLNLNHLEI; LRKKLNLNHLEID; RKKLNLNHLEIDD; KKLNLNHLEIDDT; KLNLNHLEIDDTL; LNLNHLEIDDTLE; NLNHLEIDDTLEK; LNHLEIDDTLEKV; NHLEIDDTLEKVA; HLEIDDTLEKVAK; LEIDDTLEKVAKE; EIDDTLEKVAKEY; IDDTLEKVAKEYA; DDTLEKVAKEYAI; DTLEKVAKEYAIK; TLEKVAKEYAIKL; LEKVAKEYAIKLG; EKVAKEYAIKLGE; KVAKEYAIKLGEN; VAKEYAIKLGENR; AKEYAIKLGENRT; KEYAIKLGENRTI; EYAIKLGENRTIT; YAIKLGENRTITH; AIKLGENRTITHT; IKLGENRTITHTL; KLGENRTITHTLF; LGENRTITHTLFG; GENRTITHTLFGT; ENRTITHTLFGTT; NRTITHTLFGTTP; RTITHTLFGTTPM; TITHTLFGTTPMQ; ITHTLFGTTPMQR; THTLFGTTPMQRI; HTLFGTTPMQRIH; TLFGTTPMQRIHK; LFGTTPMQRIHKY; FGTTPMQRIHKYD; GTTPMQRIHKYDQ; TTPMQRIHKYDQS; TPMQRIHKYDQSF; PMQRIHKYDQSFN; MQRIHKYDQSFNL; QRIHKYDQSFNLT; RIHKYDQSFNLTR; IHKYDQSFNLTRE; HKYDQSFNLTREI; KYDQSFNLTREIL; YDQSFNLTREILA; DQSFNLTREILAS; QSFNLTREILASG; SFNLTREILASGI; FNLTREILASGIE; NLTREILASGIEL; LTREILASGIELN; TREILASGIELNR; REILASGIELNRV; EILASGIELNRVV; ILASGIELNRVVN; LASGIELNRVVNA; ASGIELNRVVNAW; SGIELNRVVNAWL; GIELNRVVNAWLN; IELNRVVNAWLNS; ELNRVVNAWLNSP; LNRVVNAWLNSPS; NRVVNAWLNSPSH; RVVNAWLNSPSHK; VVNAWLNSPSHKE; VNAWLNSPSHKEA; NAWLNSPSHKEAL; AWLNSPSHKEALI; WLNSPSHKEALIN; LNSPSHKEALINT; NSPSHKEALINTD; SPSHKEALINTDT; PSHKEALINTDTD; SHKEALINTDTDK; HKEALINTDTDKI; KEALINTDTDKIG; EALINTDTDKIGG; |

ALINTDTDKIGGY; LINTDTDKIGGYR; INTDTDKIGGYRL;
NTDTDKIGGYRLK; TDTDKIGGYRLKT; DTDKIGGYRLKTT;
TDKIGGYRLKTTD; DKIGGYRLKTTDN; KIGGYRLKTTDNI;
IGGYRLKTTDNID; GGYRLKTTDNIDI; GYRLKTTDNIDIF;
YRLKTTDNIDIFV; RLKTTDNIDIFVV; LKTTDNIDIFVVL;
KTTDNIDIFVVLF; TTDNIDIFVVLFG; TDNIDIFVVLFGK;
DNIDIFVVLFGKR; NIDIFVVLFGKRK; IDIFVVLFGKRKY;
DIFVVLFGKRKYK; IFVVLFGKRKYKN;

14 mers:
MKKLIIIFTLFLSQ; KKLIIIFTLFLSQA; KLIIIFTLFLSQAC;
LIIIFTLFLSQACN; IIIFTLFLSQACNL; IIFTLFLSQACNLS;
IFTLFLSQACNLST; FTLFLSQACNLSTM; TLFLSQACNLSTMH;
LFLSQACNLSTMHK; FLSQACNLSTMHKI; LSQACNLSTMHKID;
SQACNLSTMHKIDT; QACNLSTMHKIDTK; ACNLSTMHKIDTKE;
CNLSTMHKIDTKED; NLSTMHKIDTKEDM; LSTMHKIDTKEDMK;
STMHKIDTKEDMKI; TMHKIDTKEDMKIL; MHKIDTKEDMKILY;
HKIDTKEDMKILYS; KIDTKEDMKILYSE; IDTKEDMKILYSEI;
DTKEDMKILYSEIA; TKEDMKILYSEIAE; KEDMKILYSEIAEL;
EDMKILYSEIAELR; DMKILYSEIAELRK; MKILYSEIAELRKK;
KILYSEIAELRKKL; ILYSEIAELRKKLN; LYSEIAELRKKLNL;
YSEIAELRKKLNLN; SEIAELRKKLNLNH; EIAELRKKLNLNHL;
IAELRKKLNLNHLE; AELRKKLNLNHLEI; ELRKKLNLNHLEID;
LRKKLNLNHLEIDD; RKKLNLNHLEIDDT; KKLNLNHLEIDDTL;
KLNLNHLEIDDTLE; LNLNHLEIDDTLEK; NLNHLEIDDTLEKV;
LNHLEIDDTLEKVA; NHLEIDDTLEKVAK; HLEIDDTLEKVAKE;
LEIDDTLEKVAKEY; EIDDTLEKVAKEYA; IDDTLEKVAKEYAI;
DDTLEKVAKEYAIK; DTLEKVAKEYAIKL; TLEKVAKEYAIKLG;
LEKVAKEYAIKLGE; EKVAKEYAIKLGEN; KVAKEYAIKLGENR;
VAKEYAIKLGENRT; AKEYAIKLGENRTI; KEYAIKLGENRTIT;
EYAIKLGENRTITH; YAIKLGENRTITHT; AIKLGENRTITHTL;
IKLGENRTITHTLF; KLGENRTITHTLFG; LGENRTITHTLFGT;
GENRTITHTLFGTT; ENRTITHTLFGTTP; NRTITHTLFGTTPM;
RTITHTLFGTTPMQ; TITHTLFGTTPMQR; ITHTLFGTTPMQRI;
THTLFGTTPMQRIH; HTLFGTTPMQRIHK; TLFGTTPMQRIHKY;
LFGTTPMQRIHKYD; FGTTPMQRIHKYDQ; GTTPMQRIHKYDQS;
TTPMQRIHKYDQSF; TPMQRIHKYDQSFN; PMQRIHKYDQSFNL;
MQRIHKYDQSFNLT; QRIHKYDQSFNLTR; RIHKYDQSFNLTRE;
IHKYDQSFNLTREI; HKYDQSFNLTREIL; KYDQSFNLTREILA;
YDQSFNLTREILAS; DQSFNLTREILASG; QSFNLTREILASGI;
SFNLTREILASGIE; FNLTREILASGIEL; NLTREILASGIELN;
LTREILASGIELNR; TREILASGIELNRV; REILASGIELNRVV;
EILASGIELNRVVN; ILASGIELNRVVNA; LASGIELNRVVNAW;
ASGIELNRVVNAWL; SGIELNRVVNAWLN; GIELNRVVNAWLNS;
IELNRVVNAWLNSP; ELNRVVNAWLNSPS; LNRVVNAWLNSPSH;
NRVVNAWLNSPSHK; RVVNAWLNSPSHKE; VVNAWLNSPSHKEA;
VNAWLNSPSHKEAL; NAWLNSPSHKEALI; AWLNSPSHKEALIN;
WLNSPSHKEALINT; LNSPSHKEALINTD; NSPSHKEALINTDT;
SPSHKEALINTDTD; PSHKEALINTDTDK; SHKEALINTDTDKI;
HKEALINTDTDKIG; KEALINTDTDKIGG; EALINTDTDKIGGY;
ALINTDTDKIGGYR; LINTDTDKIGGYRL; INTDTDKIGGYRLK;

NTDTDKIGGYRLKT;  TDTDKIGGYRLKTT;  DTDKIGGYRLKTTD;
TDKIGGYRLKTTDN;  DKIGGYRLKTTDNI;  KIGGYRLKTTDNID;
IGGYRLKTTDNIDI;  GGYRLKTTDNIDIF;  GYRLKTTDNIDIFV;
YRLKTTDNIDIFVV;  RLKTTDNIDIFVVL;  LKTTDNIDIFVVLF;
KTTDNIDIFVVLFG;  TTDNIDIFVVLFGK;  TDNIDIFVVLFGKR;
DNIDIFVVLFGKRK;  NIDIFVVLFGKRKY;  IDIFVVLFGKRKYK;
DIFVVLFGKRKYKN;

15 mers:
MKKLIIIFTLFLSQA;  KKLIIIFTLFLSQAC;  KLIIIFTLFLSQACN;
LIIIFTLFLSQACNL;  IIIFTLFLSQACNLS;  IIFTLFLSQACNLST;
IFTLFLSQACNLSTM;  FTLFLSQACNLSTMH;  TLFLSQACNLSTMHK;
LFLSQACNLSTMHKI;  FLSQACNLSTMHKID;  LSQACNLSTMHKIDT;
SQACNLSTMHKIDTK;  QACNLSTMHKIDTKE;  ACNLSTMHKIDTKED;
CNLSTMHKIDTKEDM;  NLSTMHKIDTKEDMK;  LSTMHKIDTKEDMKI;
STMHKIDTKEDMKIL;  TMHKIDTKEDMKILY;  MHKIDTKEDMKILYS;
HKIDTKEDMKILYSE;  KIDTKEDMKILYSEI;  IDTKEDMKILYSEIA;
DTKEDMKILYSEIAE;  TKEDMKILYSEIAEL;  KEDMKILYSEIAELR;
EDMKILYSEIAELRK;  DMKILYSEIAELRKK;  MKILYSEIAELRKKL;
KILYSEIAELRKKLN;  ILYSEIAELRKKLNL;  LYSEIAELRKKLNLN;
YSEIAELRKKLNLNH;  SEIAELRKKLNLNHL;  EIAELRKKLNLNHLE;
IAELRKKLNLNHLEI;  AELRKKLNLNHLEID;  ELRKKLNLNHLEIDD;
LRKKLNLNHLEIDDT;  RKKLNLNHLEIDDTL;  KKLNLNHLEIDDTLE;
KLNLNHLEIDDTLEK;  LNLNHLEIDDTLEKV;  NLNHLEIDDTLEKVA;
LNHLEIDDTLEKVAK;  NHLEIDDTLEKVAKE;  HLEIDDTLEKVAKEY;
LEIDDTLEKVAKEYA;  EIDDTLEKVAKEYAI;  IDDTLEKVAKEYAIK;
DDTLEKVAKEYAIKL;  DTLEKVAKEYAIKLG;  TLEKVAKEYAIKLGE;
LEKVAKEYAIKLGEN;  EKVAKEYAIKLGENR;  KVAKEYAIKLGENRT;
VAKEYAIKLGENRTI;  AKEYAIKLGENRTIT;  KEYAIKLGENRTITH;
EYAIKLGENRTITHT;  YAIKLGENRTITHTL;  AIKLGENRTITHTLF;
IKLGENRTITHTLFG;  KLGENRTITHTLFGT;  LGENRTITHTLFGTT;
GENRTITHTLFGTTP;  ENRTITHTLFGTTPM;  NRTITHTLFGTTPMQ;
RTITHTLFGTTPMQR;  TITHTLFGTTPMQRI;  ITHTLFGTTPMQRIH;
THTLFGTTPMQRIHK;  HTLFGTTPMQRIHKY;  TLFGTTPMQRIHKYD;
LFGTTPMQRIHKYDQ;  FGTTPMQRIHKYDQS;  GTTPMQRIHKYDQSF;
TTPMQRIHKYDQSFN;  TPMQRIHKYDQSFNL;  PMQRIHKYDQSFNLT;
MQRIHKYDQSFNLTR;  QRIHKYDQSFNLTRE;  RIHKYDQSFNLTREI;
IHKYDQSFNLTREIL;  HKYDQSFNLTREILA;  KYDQSFNLTREILAS;
YDQSFNLTREILASG;  DQSFNLTREILASGI;  QSFNLTREILASGIE;
SFNLTREILASGIEL;  FNLTREILASGIELN;  NLTREILASGIELNR;
LTREILASGIELNRV;  TREILASGIELNRVV;  REILASGIELNRVVN;
EILASGIELNRVVNA;  ILASGIELNRVVNAW;  LASGIELNRVVNAWL;
ASGIELNRVVNAWLN;  SGIELNRVVNAWLNS;  GIELNRVVNAWLNSP;
IELNRVVNAWLNSPS;  ELNRVVNAWLNSPSH;  LNRVVNAWLNSPSHK;
NRVVNAWLNSPSHKE;  RVVNAWLNSPSHKEA;  VVNAWLNSPSHKEAL;
VNAWLNSPSHKEALI;  NAWLNSPSHKEALIN;  AWLNSPSHKEALINT;
WLNSPSHKEALINTD;  LNSPSHKEALINTDT;  NSPSHKEALINTDTD;
SPSHKEALINTDTDK;  PSHKEALINTDTDKI;  SHKEALINTDTDKIG;
HKEALINTDTDKIGG;  KEALINTDTDKIGGY;  EALINTDTDKIGGYR;
ALINTDTDKIGGYRL;  LINTDTDKIGGYRLK;  INTDTDKIGGYRLKT;
NTDTDKIGGYRLKTT;  TDTDKIGGYRLKTTD;  DTDKIGGYRLKTTDN;

TDKIGGYRLKTTDNI; DKIGGYRLKTTDNID; KIGGYRLKTTDNIDI;
IGGYRLKTTDNIDIF; GGYRLKTTDNIDIFV; GYRLKTTDNIDIFVV;
YRLKTTDNIDIFVVL; RLKTTDNIDIFVVLF; LKTTDNIDIFVVLFG;
KTTDNIDIFVVLFGK; TTDNIDIFVVLFGKR; TDNIDIFVVLFGKRK;
DNIDIFVVLFGKRKY; NIDIFVVLFGKRKYK; IDIFVVLFGKRKYKN;

16 mers:
MKKLIIIFTLFLSQAC; KKLIIIFTLFLSQACN; KLIIIFTLFLSQACNL;
LIIIFTLFLSQACNLS; IIIFTLFLSQACNLST; IIFTLFLSQACNLSTM;
IFTLFLSQACNLSTMH; FTLFLSQACNLSTMHK; TLFLSQACNLSTMHKI;
LFLSQACNLSTMHKID; FLSQACNLSTMHKIDT; LSQACNLSTMHKIDTK;
SQACNLSTMHKIDTKE; QACNLSTMHKIDTKED; ACNLSTMHKIDTKEDM;
CNLSTMHKIDTKEDMK; NLSTMHKIDTKEDMKI; LSTMHKIDTKEDMKIL;
STMHKIDTKEDMKILY; TMHKIDTKEDMKILYS; MHKIDTKEDMKILYSE;
HKIDTKEDMKILYSEI; KIDTKEDMKILYSEIA; IDTKEDMKILYSEIAE;
DTKEDMKILYSEIAEL; TKEDMKILYSEIAELR; KEDMKILYSEIAELRK;
EDMKILYSEIAELRKK; DMKILYSEIAELRKKL; MKILYSEIAELRKKLN;
KILYSEIAELRKKLNL; ILYSEIAELRKKLNLN; LYSEIAELRKKLNLNH;
YSEIAELRKKLNLNHL; SEIAELRKKLNLNHLE; EIAELRKKLNLNHLEI;
IAELRKKLNLNHLEID; AELRKKLNLNHLEIDD; ELRKKLNLNHLEIDDT;
LRKKLNLNHLEIDDTL; RKKLNLNHLEIDDTLE; KKLNLNHLEIDDTLEK;
KLNLNHLEIDDTLEKV; LNLNHLEIDDTLEKVA; NLNHLEIDDTLEKVAK;
LNHLEIDDTLEKVAKE; NHLEIDDTLEKVAKEY; HLEIDDTLEKVAKEYA;
LEIDDTLEKVAKEYAI; EIDDTLEKVAKEYAIK; IDDTLEKVAKEYAIKL;
DDTLEKVAKEYAIKLG; DTLEKVAKEYAIKLGE; TLEKVAKEYAIKLGEN;
LEKVAKEYAIKLGENR; EKVAKEYAIKLGENRT; KVAKEYAIKLGENRTI;
VAKEYAIKLGENRTIT; AKEYAIKLGENRTITH; KEYAIKLGENRTITHT;
EYAIKLGENRTITHTL; YAIKLGENRTITHTLF; AIKLGENRTITHTLFG;
IKLGENRTITHTLFGT; KLGENRTITHTLFGTT; LGENRTITHTLFGTTP;
GENRTITHTLFGTTPM; ENRTITHTLFGTTPMQ; NRTITHTLFGTTPMQR;
RTITHTLFGTTPMQRI; TITHTLFGTTPMQRIH; ITHTLFGTTPMQRIHK;
THTLFGTTPMQRIHKY; HTLFGTTPMQRIHKYD; TLFGTTPMQRIHKYDQ;
LFGTTPMQRIHKYDQS; FGTTPMQRIHKYDQSF; GTTPMQRIHKYDQSFN;
TTPMQRIHKYDQSFNL; TPMQRIHKYDQSFNLT; PMQRIHKYDQSFNLTR;
MQRIHKYDQSFNLTRE; QRIHKYDQSFNLTREI; RIHKYDQSFNLTREIL;
IHKYDQSFNLTREILA; HKYDQSFNLTREILAS; KYDQSFNLTREILASG;
YDQSFNLTREILASGI; DQSFNLTREILASGIE; QSFNLTREILASGIEL;
SFNLTREILASGIELN; FNLTREILASGIELNR; NLTREILASGIELNRV;
LTREILASGIELNRVV; TREILASGIELNRVVN; REILASGIELNRVVNA;
EILASGIELNRVVNAW; ILASGIELNRVVNAWL; LASGIELNRVVNAWLN;
ASGIELNRVVNAWLNS; SGIELNRVVNAWLNSP; GIELNRVVNAWLNSPS;
IELNRVVNAWLNSPSH; ELNRVVNAWLNSPSHK; LNRVVNAWLNSPSHKE;
NRVVNAWLNSPSHKEA; RVVNAWLNSPSHKEAL; VVNAWLNSPSHKEALI;
VNAWLNSPSHKEALIN; NAWLNSPSHKEALINT; AWLNSPSHKEALINTD;
WLNSPSHKEALINTDT; LNSPSHKEALINTDTD; NSPSHKEALINTDTDK;
SPSHKEALINTDTDKI; PSHKEALINTDTDKIG; SHKEALINTDTDKIGG;
HKEALINTDTDKIGGY; KEALINTDTDKIGGYR; EALINTDTDKIGGYRL;
ALINTDTDKIGGYRLK; LINTDTDKIGGYRLKT; INTDTDKIGGYRLKTT;
NTDTDKIGGYRLKTTD; TDTDKIGGYRLKTTDN; DTDKIGGYRLKTTDNI;
TDKIGGYRLKTTDNID; DKIGGYRLKTTDNIDI; KIGGYRLKTTDNIDIF;
IGGYRLKTTDNIDIFV; GGYRLKTTDNIDIFVV; GYRLKTTDNIDIFVVL;

| | |
|---|---|
| | YRLKTTDNIDIFVVLF; RLKTTDNIDIFVVLFG; LKTTDNIDIFVVLFGK; KTTDNIDIFVVLFGKR; TTDNIDIFVVLFGKRK; TDNIDIFVVLFGKRKY; DNIDIFVVLFGKRKYK; NIDIFVVLFGKRKYKN; |
| Seq 32 | 13 mers:<br>MMQRISILLMLLA; MQRISILLMLLAV; QRISILLMLLAVF;<br>RISILLMLLAVFS; ISILLMLLAVFSC; SILLMLLAVFSCK;<br>ILLMLLAVFSCKQ; LLMLLAVFSCKQF; LMLLAVFSCKQFG;<br>MLLAVFSCKQFGD; LLAVFSCKQFGDV; LAVFSCKQFGDVK;<br>AVFSCKQFGDVKS; VFSCKQFGDVKSL; FSCKQFGDVKSLT;<br>SCKQFGDVKSLTE; CKQFGDVKSLTEI; KQFGDVKSLTEID;<br>QFGDVKSLTEIDS; FGDVKSLTEIDSG; GDVKSLTEIDSGN;<br>DVKSLTEIDSGNG; VKSLTEIDSGNGI; KSLTEIDSGNGIP;<br>SLTEIDSGNGIPL; LTEIDSGNGIPLV; TEIDSGNGIPLVV;<br>EIDSGNGIPLVVS; IDSGNGIPLVVSD; DSGNGIPLVVSDV;<br>SGNGIPLVVSDVV; GNGIPLVVSDVVK; NGIPLVVSDVVKD;<br>GIPLVVSDVVKDL; IPLVVSDVVKDLI; PLVVSDVVKDLIP;<br>LVVSDVVKDLIPK; VVSDVVKDLIPKE; VSDVVKDLIPKEI;<br>SDVVKDLIPKEIS; DVVKDLIPKEISL; VVKDLIPKEISLT;<br>VKDLIPKEISLTP; KDLIPKEISLTPE; DLIPKEISLTPEE;<br>LIPKEISLTPEEA; IPKEISLTPEEAE; PKEISLTPEEAEK;<br>KEISLTPEEAEKL; EISLTPEEAEKLE; ISLTPEEAEKLES;<br>SLTPEEAEKLESL; LTPEEAEKLESLK; TPEEAEKLESLKV;<br>PEEAEKLESLKVF; EEAEKLESLKVFL; EAEKLESLKVFLK;<br>AEKLESLKVFLKD; EKLESLKVFLKDA; KLESLKVFLKDAM;<br>LESLKVFLKDAMS; ESLKVFLKDAMSV; SLKVFLKDAMSVN;<br>LKVFLKDAMSVNG; KVFLKDAMSVNGR; VFLKDAMSVNGRE;<br>FLKDAMSVNGREE; LKDAMSVNGREEA; KDAMSVNGREEAL;<br>DAMSVNGREEALK; AMSVNGREEALKA; MSVNGREEALKAE;<br>SVNGREEALKAEY; VNGREEALKAEYE; NGREEALKAEYEK;<br>GREEALKAEYEKS; REEALKAEYEKSY; EEALKAEYEKSYK;<br>EALKAEYEKSYKE; ALKAEYEKSYKEF; LKAEYEKSYKEFF;<br>KAEYEKSYKEFFD; AEYEKSYKEFFDW; EYEKSYKEFFDWL;<br>YEKSYKEFFDWLS; EKSYKEFFDWLSK; KSYKEFFDWLSKD;<br>SYKEFFDWLSKDV; YKEFFDWLSKDVN; KEFFDWLSKDVNR;<br>EFFDWLSKDVNRQ; FFDWLSKDVNRQK; FDWLSKDVNRQKE;<br>DWLSKDVNRQKEF; WLSKDVNRQKEFI; LSKDVNRQKEFIS;<br>SKDVNRQKEFISS; KDVNRQKEFISSF; DVNRQKEFISSFD;<br>VNRQKEFISSFDN; NRQKEFISSFDNI; RQKEFISSFDNIS;<br>QKEFISSFDNISS; KEFISSFDNISSI; EFISSFDNISSIV;<br>FISSFDNISSIVS; ISSFDNISSIVSK; SSFDNISSIVSKA;<br>SFDNISSIVSKAV; FDNISSIVSKAVD; DNISSIVSKAVDA;<br>NISSIVSKAVDAS; ISSIVSKAVDASK; SSIVSKAVDASKK;<br>SIVSKAVDASKKR; IVSKAVDASKKRR; VSKAVDASKKRRP;<br>SKAVDASKKRRPT; KAVDASKKRRPTE; AVDASKKRRPTEQ;<br>VDASKKRRPTEQQ; DASKKRRPTEQQS; ASKKRRPTEQQSL;<br>SKKRRPTEQQSLG; KKRRPTEQQSLGF; KRRPTEQQSLGFK;<br>RRPTEQQSLGFKE; RPTEQQSLGFKEY; PTEQQSLGFKEYV;<br>TEQQSLGFKEYVC; EQQSLGFKEYVCY; QQSLGFKEYVCYK;<br>QSLGFKEYVCYKI; SLGFKEYVCYKIK; LGFKEYVCYKIKN; |

GFKEYVCYKIKNS; FKEYVCYKIKNSK; KEYVCYKIKNSKG;
EYVCYKIKNSKGE; YVCYKIKNSKGEA; VCYKIKNSKGEAL;
CYKIKNSKGEALS; YKIKNSKGEALSL; KIKNSKGEALSLF;
IKNSKGEALSLFF; KNSKGEALSLFFQ; NSKGEALSLFFQK;
SKGEALSLFFQKV; KGEALSLFFQKVV; GEALSLFFQKVVD;
EALSLFFQKVVDA; ALSLFFQKVVDAF; LSLFFQKVVDAFG;
SLFFQKVVDAFGA; LFFQKVVDAFGAD; FFQKVVDAFGADP;
FQKVVDAFGADPY; QKVVDAFGADPYK; KVVDAFGADPYKK;
VVDAFGADPYKKD; VDAFGADPYKKDN; DAFGADPYKKDND;
AFGADPYKKDNDE; FGADPYKKDNDES; GADPYKKDNDESV;
ADPYKKDNDESVQ; DPYKKDNDESVQK; PYKKDNDESVQKP;
YKKDNDESVQKPV; KKDNDESVQKPVK; KDNDESVQKPVKC;
DNDESVQKPVKCN; NDESVQKPVKCNE; DESVQKPVKCNEE;
ESVQKPVKCNEEI; SVQKPVKCNEEIF; VQKPVKCNEEIFK;
QKPVKCNEEIFKV; KPVKCNEEIFKVI; PVKCNEEIFKVIK;
VKCNEEIFKVIKK; KCNEEIFKVIKKV; CNEEIFKVIKKVL;
NEEIFKVIKKVLT; EEIFKVIKKVLTE; EIFKVIKKVLTES;
IFKVIKKVLTESE; FKVIKKVLTESES; KVIKKVLTESESN;
VIKKVLTESESNN; IKKVLTESESNNE; KKVLTESESNNEL;
KVLTESESNNELK; VLTESESNNELKN; LTESESNNELKNL;
TESESNNELKNLK; ESESNNELKNLKN; SESNNELKNLKNY;
ESNNELKNLKNYG; SNNELKNLKNYGN; NNELKNLKNYGNV;

14 mers:
MMQRISILLMLLAV; MQRISILLMLLAVF; QRISILLMLLAVFS;
RISILLMLLAVFSC; ISILLMLLAVFSCK; SILLMLLAVFSCKQ;
ILLMLLAVFSCKQF; LLMLLAVFSCKQFG; LMLLAVFSCKQFGD;
MLLAVFSCKQFGDV; LLAVFSCKQFGDVK; LAVFSCKQFGDVKS;
AVFSCKQFGDVKSL; VFSCKQFGDVKSLT; FSCKQFGDVKSLTE;
SCKQFGDVKSLTEI; CKQFGDVKSLTEID; KQFGDVKSLTEIDS;
QFGDVKSLTEIDSG; FGDVKSLTEIDSGN; GDVKSLTEIDSGNG;
DVKSLTEIDSGNGI; VKSLTEIDSGNGIP; KSLTEIDSGNGIPL;
SLTEIDSGNGIPLV; LTEIDSGNGIPLVV; TEIDSGNGIPLVVS;
EIDSGNGIPLVVSD; IDSGNGIPLVVSDV; DSGNGIPLVVSDVV;
SGNGIPLVVSDVVK; GNGIPLVVSDVVKD; NGIPLVVSDVVKDL;
GIPLVVSDVVKDLI; IPLVVSDVVKDLIP; PLVVSDVVKDLIPK;
LVVSDVVKDLIPKE; VVSDVVKDLIPKEI; VSDVVKDLIPKEIS;
SDVVKDLIPKEISL; DVVKDLIPKEISLT; VVKDLIPKEISLTP;
VKDLIPKEISLTPE; KDLIPKEISLTPEE; DLIPKEISLTPEEA;
LIPKEISLTPEEAE; IPKEISLTPEEAEK; PKEISLTPEEAEKL;
KEISLTPEEAEKLE; EISLTPEEAEKLES; ISLTPEEAEKLESL;
SLTPEEAEKLESLK; LTPEEAEKLESLKV; TPEEAEKLESLKVF;
PEEAEKLESLKVFL; EEAEKLESLKVFLK; EAEKLESLKVFLKD;
AEKLESLKVFLKDA; EKLESLKVFLKDAM; KLESLKVFLKDAMS;
LESLKVFLKDAMSV; ESLKVFLKDAMSVN; SLKVFLKDAMSVNG;
LKVFLKDAMSVNGR; KVFLKDAMSVNGRE; VFLKDAMSVNGREE;
FLKDAMSVNGREEA; LKDAMSVNGREEAL; KDAMSVNGREEALK;
DAMSVNGREEALKA; AMSVNGREEALKAE; MSVNGREEALKAEY;
SVNGREEALKAEYE; VNGREEALKAEYEK; NGREEALKAEYEKS;
GREEALKAEYEKSY; REEALKAEYEKSYK; EEALKAEYEKSYKE;
EALKAEYEKSYKEF; ALKAEYEKSYKEFF; LKAEYEKSYKEFFD;

KAEYEKSYKEFFDW; AEYEKSYKEFFDWL; EYEKSYKEFFDWLS;
YEKSYKEFFDWLSK; EKSYKEFFDWLSKD; KSYKEFFDWLSKDV;
SYKEFFDWLSKDVN; YKEFFDWLSKDVNR; KEFFDWLSKDVNRQ;
EFFDWLSKDVNRQK; FFDWLSKDVNRQKE; FDWLSKDVNRQKEF;
DWLSKDVNRQKEFI; WLSKDVNRQKEFIS; LSKDVNRQKEFISS;
SKDVNRQKEFISSF; KDVNRQKEFISSFD; DVNRQKEFISSFDN;
VNRQKEFISSFDNI; NRQKEFISSFDNIS; RQKEFISSFDNISS;
QKEFISSFDNISSI; KEFISSFDNISSIV; EFISSFDNISSIVS;
FISSFDNISSIVSK; ISSFDNISSIVSKA; SSFDNISSIVSKAV;
SFDNISSIVSKAVD; FDNISSIVSKAVDA; DNISSIVSKAVDAS;
NISSIVSKAVDASK; ISSIVSKAVDASKK; SSIVSKAVDASKKR;
SIVSKAVDASKKRR; IVSKAVDASKKRRP; VSKAVDASKKRRPT;
SKAVDASKKRRPTE; KAVDASKKRRPTEQ; AVDASKKRRPTEQQ;
VDASKKRRPTEQQS; DASKKRRPTEQQSL; ASKKRRPTEQQSLG;
SKKRRPTEQQSLGF; KKRRPTEQQSLGFK; KRRPTEQQSLGFKE;
RRPTEQQSLGFKEY; RPTEQQSLGFKEYV; PTEQQSLGFKEYVC;
TEQQSLGFKEYVCY; EQQSLGFKEYVCYK; QQSLGFKEYVCYKI;
QSLGFKEYVCYKIK; SLGFKEYVCYKIKN; LGFKEYVCYKIKNS;
GFKEYVCYKIKNSK; FKEYVCYKIKNSKG; KEYVCYKIKNSKGE;
EYVCYKIKNSKGEA; YVCYKIKNSKGEAL; VCYKIKNSKGEALS;
CYKIKNSKGEALSL; YKIKNSKGEALSLF; KIKNSKGEALSLFF;
IKNSKGEALSLFFQ; KNSKGEALSLFFQK; NSKGEALSLFFQKV;
SKGEALSLFFQKVV; KGEALSLFFQKVVD; GEALSLFFQKVVDA;
EALSLFFQKVVDAF; ALSLFFQKVVDAFG; LSLFFQKVVDAFGA;
SLFFQKVVDAFGAD; LFFQKVVDAFGADP; FFQKVVDAFGADPY;
FQKVVDAFGADPYK; QKVVDAFGADPYKK; KVVDAFGADPYKKD;
VVDAFGADPYKKDN; VDAFGADPYKKDND; DAFGADPYKKDNDE;
AFGADPYKKDNDES; FGADPYKKDNDESV; GADPYKKDNDESVQ;
ADPYKKDNDESVQK; DPYKKDNDESVQKP; PYKKDNDESVQKPV;
YKKDNDESVQKPVK; KKDNDESVQKPVKC; KDNDESVQKPVKCN;
DNDESVQKPVKCNE; NDESVQKPVKCNEE; DESVQKPVKCNEEI;
ESVQKPVKCNEEIF; SVQKPVKCNEEIFK; VQKPVKCNEEIFKV;
QKPVKCNEEIFKVI; KPVKCNEEIFKVIK; PVKCNEEIFKVIKK;
VKCNEEIFKVIKKV; KCNEEIFKVIKKVL; CNEEIFKVIKKVLT;
NEEIFKVIKKVLTE; EEIFKVIKKVLTES; EIFKVIKKVLTESE;
IFKVIKKVLTESES; FKVIKKVLTESESN; KVIKKVLTESESNN;
VIKKVLTESESNNE; IKKVLTESESNNEL; KKVLTESESNNELK;
KVLTESESNNELKN; VLTESESNNELKNL; LTESESNNELKNLK;
TESESNNELKNLKN; ESESNNELKNLKNY; SESNNELKNLKNYG;
ESNNELKNLKNYGN; SNNELKNLKNYGNV;

15 mers:
MMQRISILLMLLAVF; MQRISILLMLLAVFS; QRISILLMLLAVFSC;
RISILLMLLAVFSCK; ISILLMLLAVFSCKQ; SILLMLLAVFSCKQF;
ILLMLLAVFSCKQFG; LLMLLAVFSCKQFGD; LMLLAVFSCKQFGDV;
MLLAVFSCKQFGDVK; LLAVFSCKQFGDVKS; LAVFSCKQFGDVKSL;
AVFSCKQFGDVKSLT; VFSCKQFGDVKSLTE; FSCKQFGDVKSLTEI;
SCKQFGDVKSLTEID; CKQFGDVKSLTEIDS; KQFGDVKSLTEIDSG;
QFGDVKSLTEIDSGN; FGDVKSLTEIDSGNG; GDVKSLTEIDSGNGI;
DVKSLTEIDSGNGIP; VKSLTEIDSGNGIPL; KSLTEIDSGNGIPLV;
SLTEIDSGNGIPLVV; LTEIDSGNGIPLVVS; TEIDSGNGIPLVVSD;

| | | |
|---|---|---|
| EIDSGNGIPLVVSDV; | IDSGNGIPLVVSDVV; | DSGNGIPLVVSDVVK; |
| SGNGIPLVVSDVVKD; | GNGIPLVVSDVVKDL; | NGIPLVVSDVVKDLI; |
| GIPLVVSDVVKDLIP; | IPLVVSDVVKDLIPK; | PLVVSDVVKDLIPKE; |
| LVVSDVVKDLIPKEI; | VVSDVVKDLIPKEIS; | VSDVVKDLIPKEISL; |
| SDVVKDLIPKEISLT; | DVVKDLIPKEISLTP; | VVKDLIPKEISLTPE; |
| VKDLIPKEISLTPEE; | KDLIPKEISLTPEEA; | DLIPKEISLTPEEAE; |
| LIPKEISLTPEEAEK; | IPKEISLTPEEAEKL; | PKEISLTPEEAEKLE; |
| KEISLTPEEAEKLES; | EISLTPEEAEKLESL; | ISLTPEEAEKLESLK; |
| SLTPEEAEKLESLKV; | LTPEEAEKLESLKVF; | TPEEAEKLESLKVFL; |
| PEEAEKLESLKVFLK; | EEAEKLESLKVFLKD; | EAEKLESLKVFLKDA; |
| AEKLESLKVFLKDAM; | EKLESLKVFLKDAMS; | KLESLKVFLKDAMSV; |
| LESLKVFLKDAMSVN; | ESLKVFLKDAMSVNG; | SLKVFLKDAMSVNGR; |
| LKVFLKDAMSVNGRE; | KVFLKDAMSVNGREE; | VFLKDAMSVNGREEA; |
| FLKDAMSVNGREEAL; | LKDAMSVNGREEALK; | KDAMSVNGREEALKA; |
| DAMSVNGREEALKAE; | AMSVNGREEALKAEY; | MSVNGREEALKAEYE; |
| SVNGREEALKAEYEK; | VNGREEALKAEYEKS; | NGREEALKAEYEKSY; |
| GREEALKAEYEKSYK; | REEALKAEYEKSYKE; | EEALKAEYEKSYKEF; |
| EALKAEYEKSYKEFF; | ALKAEYEKSYKEFFD; | LKAEYEKSYKEFFDW; |
| KAEYEKSYKEFFDWL; | AEYEKSYKEFFDWLS; | EYEKSYKEFFDWLSK; |
| YEKSYKEFFDWLSKD; | EKSYKEFFDWLSKDV; | KSYKEFFDWLSKDVN; |
| SYKEFFDWLSKDVNR; | YKEFFDWLSKDVNRQ; | KEFFDWLSKDVNRQK; |
| EFFDWLSKDVNRQKE; | FFDWLSKDVNRQKEF; | FDWLSKDVNRQKEFI; |
| DWLSKDVNRQKEFIS; | WLSKDVNRQKEFISS; | LSKDVNRQKEFISSF; |
| SKDVNRQKEFISSFD; | KDVNRQKEFISSFDN; | DVNRQKEFISSFDNI; |
| VNRQKEFISSFDNIS; | NRQKEFISSFDNISS; | RQKEFISSFDNISSI; |
| QKEFISSFDNISSIV; | KEFISSFDNISSIVS; | EFISSFDNISSIVSK; |
| FISSFDNISSIVSKA; | ISSFDNISSIVSKAV; | SSFDNISSIVSKAVD; |
| SFDNISSIVSKAVDA; | FDNISSIVSKAVDAS; | DNISSIVSKAVDASK; |
| NISSIVSKAVDASKK; | ISSIVSKAVDASKKR; | SSIVSKAVDASKKRR; |
| SIVSKAVDASKKRRP; | IVSKAVDASKKRRPT; | VSKAVDASKKRRPTE; |
| SKAVDASKKRRPTEQ; | KAVDASKKRRPTEQQ; | AVDASKKRRPTEQQS; |
| VDASKKRRPTEQQSL; | DASKKRRPTEQQSLG; | ASKKRRPTEQQSLGF; |
| SKKRRPTEQQSLGFK; | KKRRPTEQQSLGFKE; | KRRPTEQQSLGFKEY; |
| RRPTEQQSLGFKEYV; | RPTEQQSLGFKEYVC; | PTEQQSLGFKEYVCY; |
| TEQQSLGFKEYVCYK; | EQQSLGFKEYVCYKI; | QQSLGFKEYVCYKIK; |
| QSLGFKEYVCYKIKN; | SLGFKEYVCYKIKNS; | LGFKEYVCYKIKNSK; |
| GFKEYVCYKIKNSKG; | FKEYVCYKIKNSKGE; | KEYVCYKIKNSKGEA; |
| EYVCYKIKNSKGEAL; | YVCYKIKNSKGEALS; | VCYKIKNSKGEALSL; |
| CYKIKNSKGEALSLF; | YKIKNSKGEALSLFF; | KIKNSKGEALSLFFQ; |
| IKNSKGEALSLFFQK; | KNSKGEALSLFFQKV; | NSKGEALSLFFQKVV; |
| SKGEALSLFFQKVVD; | KGEALSLFFQKVVDA; | GEALSLFFQKVVDAF; |
| EALSLFFQKVVDAFG; | ALSLFFQKVVDAFGA; | LSLFFQKVVDAFGAD; |
| SLFFQKVVDAFGADP; | LFFQKVVDAFGADPY; | FFQKVVDAFGADPYK; |
| FQKVVDAFGADPYKK; | QKVVDAFGADPYKKD; | KVVDAFGADPYKKDN; |
| VVDAFGADPYKKDND; | VDAFGADPYKKDNDE; | DAFGADPYKKDNDES; |
| AFGADPYKKDNDESV; | FGADPYKKDNDESVQ; | GADPYKKDNDESVQK; |
| ADPYKKDNDESVQKP; | DPYKKDNDESVQKPV; | PYKKDNDESVQKPVK; |
| YKKDNDESVQKPVKC; | KKDNDESVQKPVKCN; | KDNDESVQKPVKCNE; |
| DNDESVQKPVKCNEE; | NDESVQKPVKCNEEI; | DESVQKPVKCNEEIF; |
| ESVQKPVKCNEEIFK; | SVQKPVKCNEEIFKV; | VQKPVKCNEEIFKVI; |
| QKPVKCNEEIFKVIK; | KPVKCNEEIFKVIKK; | PVKCNEEIFKVIKKV; |

VKCNEEIFKVIKKVL; KCNEEIFKVIKKVLT; CNEEIFKVIKKVLTE;
NEEIFKVIKKVLTES; EEIFKVIKKVLTESE; EIFKVIKKVLTESES;
IFKVIKKVLTESESN; FKVIKKVLTESESNN; KVIKKVLTESESNNE;
VIKKVLTESESNNEL; IKKVLTESESNNELK; KKVLTESESNNELKN;
KVLTESESNNELKNL; VLTESESNNELKNLK; LTESESNNELKNLKN;
TESESNNELKNLKNY; ESESNNELKNLKNYG; SESNNELKNLKNYGN;
ESNNELKNLKNYGNV;

16 mers:
MMQRISILLMLLAVFS; MQRISILLMLLAVFSC; QRISILLMLLAVFSCK;
RISILLMLLAVFSCKQ; ISILLMLLAVFSCKQF; SILLMLLAVFSCKQFG;
ILLMLLAVFSCKQFGD; LLMLLAVFSCKQFGDV; LMLLAVFSCKQFGDVK;
MLLAVFSCKQFGDVKS; LLAVFSCKQFGDVKSL; LAVFSCKQFGDVKSLT;
AVFSCKQFGDVKSLTE; VFSCKQFGDVKSLTEI; FSCKQFGDVKSLTEID;
SCKQFGDVKSLTEIDS; CKQFGDVKSLTEIDSG; KQFGDVKSLTEIDSGN;
QFGDVKSLTEIDSGNG; FGDVKSLTEIDSGNGI; GDVKSLTEIDSGNGIP;
DVKSLTEIDSGNGIPL; VKSLTEIDSGNGIPLV; KSLTEIDSGNGIPLVV;
SLTEIDSGNGIPLVVS; LTEIDSGNGIPLVVSD; TEIDSGNGIPLVVSDV;
EIDSGNGIPLVVSDVV; IDSGNGIPLVVSDVVK; DSGNGIPLVVSDVVKD;
SGNGIPLVVSDVVKDL; GNGIPLVVSDVVKDLI; NGIPLVVSDVVKDLIP;
GIPLVVSDVVKDLIPK; IPLVVSDVVKDLIPKE; PLVVSDVVKDLIPKEI;
LVVSDVVKDLIPKEIS; VVSDVVKDLIPKEISL; VSDVVKDLIPKEISLT;
SDVVKDLIPKEISLTP; DVVKDLIPKEISLTPE; VVKDLIPKEISLTPEE;
VKDLIPKEISLTPEEA; KDLIPKEISLTPEEAE; DLIPKEISLTPEEAEK;
LIPKEISLTPEEAEKL; IPKEISLTPEEAEKLE; PKEISLTPEEAEKLES;
KEISLTPEEAEKLESL; EISLTPEEAEKLESLK; ISLTPEEAEKLESLKV;
SLTPEEAEKLESLKVF; LTPEEAEKLESLKVFL; TPEEAEKLESLKVFLK;
PEEAEKLESLKVFLKD; EEAEKLESLKVFLKDA; EAEKLESLKVFLKDAM;
AEKLESLKVFLKDAMS; EKLESLKVFLKDAMSV; KLESLKVFLKDAMSVN;
LESLKVFLKDAMSVNG; ESLKVFLKDAMSVNGR; SLKVFLKDAMSVNGRE;
LKVFLKDAMSVNGREE; KVFLKDAMSVNGREEA; VFLKDAMSVNGREEAL;
FLKDAMSVNGREEALK; LKDAMSVNGREEALKA; KDAMSVNGREEALKAE;
DAMSVNGREEALKAEY; AMSVNGREEALKAEYE; MSVNGREEALKAEYEK;
SVNGREEALKAEYEKS; VNGREEALKAEYEKSY; NGREEALKAEYEKSYK;
GREEALKAEYEKSYKE; REEALKAEYEKSYKEF; EEALKAEYEKSYKEFF;
EALKAEYEKSYKEFFD; ALKAEYEKSYKEFFDW; LKAEYEKSYKEFFDWL;
KAEYEKSYKEFFDWLS; AEYEKSYKEFFDWLSK; EYEKSYKEFFDWLSKD;
YEKSYKEFFDWLSKDV; EKSYKEFFDWLSKDVN; KSYKEFFDWLSKDVNR;
SYKEFFDWLSKDVNRQ; YKEFFDWLSKDVNRQK; KEFFDWLSKDVNRQKE;
EFFDWLSKDVNRQKEF; FFDWLSKDVNRQKEFI; FDWLSKDVNRQKEFIS;
DWLSKDVNRQKEFISS; WLSKDVNRQKEFISSF; LSKDVNRQKEFISSFD;
SKDVNRQKEFISSFDN; KDVNRQKEFISSFDNI; DVNRQKEFISSFDNIS;
VNRQKEFISSFDNISS; NRQKEFISSFDNISSI; RQKEFISSFDNISSIV;
QKEFISSFDNISSIVS; KEFISSFDNISSIVSK; EFISSFDNISSIVSKA;
FISSFDNISSIVSKAV; ISSFDNISSIVSKAVD; SSFDNISSIVSKAVDA;
SFDNISSIVSKAVDAS; FDNISSIVSKAVDASK; DNISSIVSKAVDASKK;
NISSIVSKAVDASKKR; ISSIVSKAVDASKKRR; SSIVSKAVDASKKRRP;
SIVSKAVDASKKRRPT; IVSKAVDASKKRRPTE; VSKAVDASKKRRPTEQ;
SKAVDASKKRRPTEQQ; KAVDASKKRRPTEQQS; AVDASKKRRPTEQQSL;
VDASKKRRPTEQQSLG; DASKKRRPTEQQSLGF; ASKKRRPTEQQSLGFK;
SKKRRPTEQQSLGFKE; KKRRPTEQQSLGFKEY; KRRPTEQQSLGFKEYV;

| | |
|---|---|
| | RRPTEQQSLGFKEYVC; RPTEQQSLGFKEYVCY; PTEQQSLGFKEYVCYK;<br>TEQQSLGFKEYVCYKI; EQQSLGFKEYVCYKIK; QQSLGFKEYVCYKIKN;<br>QSLGFKEYVCYKIKNS; SLGFKEYVCYKIKNSK; LGFKEYVCYKIKNSKG;<br>GFKEYVCYKIKNSKGE; FKEYVCYKIKNSKGEA; KEYVCYKIKNSKGEAL;<br>EYVCYKIKNSKGEALS; YVCYKIKNSKGEALSL; VCYKIKNSKGEALSLF;<br>CYKIKNSKGEALSLFF; YKIKNSKGEALSLFFQ; KIKNSKGEALSLFFQK;<br>IKNSKGEALSLFFQKV; KNSKGEALSLFFQKVV; NSKGEALSLFFQKVVD;<br>SKGEALSLFFQKVVDA; KGEALSLFFQKVVDAF; GEALSLFFQKVVDAFG;<br>EALSLFFQKVVDAFGA; ALSLFFQKVVDAFGAD; LSLFFQKVVDAFGADP;<br>SLFFQKVVDAFGADPY; LFFQKVVDAFGADPYK; FFQKVVDAFGADPYKK;<br>FQKVVDAFGADPYKKD; QKVVDAFGADPYKKDN; KVVDAFGADPYKKDND;<br>VVDAFGADPYKKDNDE; VDAFGADPYKKDNDES; DAFGADPYKKDNDESV;<br>AFGADPYKKDNDESVQ; FGADPYKKDNDESVQK; GADPYKKDNDESVQKP;<br>ADPYKKDNDESVQKPV; DPYKKDNDESVQKPVK; PYKKDNDESVQKPVKC;<br>YKKDNDESVQKPVKCN; KKDNDESVQKPVKCNE; KDNDESVQKPVKCNEE;<br>DNDESVQKPVKCNEEI; NDESVQKPVKCNEEIF; DESVQKPVKCNEEIFK;<br>ESVQKPVKCNEEIFKV; SVQKPVKCNEEIFKVI; VQKPVKCNEEIFKVIK;<br>QKPVKCNEEIFKVIKK; KPVKCNEEIFKVIKKV; PVKCNEEIFKVIKKVL;<br>VKCNEEIFKVIKKVLT; KCNEEIFKVIKKVLTE; CNEEIFKVIKKVLTES;<br>NEEIFKVIKKVLTESE; EEIFKVIKKVLTESES; EIFKVIKKVLTESESN;<br>IFKVIKKVLTESESNN; FKVIKKVLTESESNNE; KVIKKVLTESESNNEL;<br>VIKKVLTESESNNELK; IKKVLTESESNNELKN; KKVLTESESNNELKNL;<br>KVLTESESNNELKNLK; VLTESESNNELKNLKN; LTESESNNELKNLKNY;<br>TESESNNELKNLKNYG; ESESNNELKNLKNYGN; SESNNELKNLKNYGNV; |
| Seq 33 | 13 mers:<br>MKRISILSILLLLL; KRISILSILLLLL; RISILSILLLLLL;<br>ISILSILLLLLLF; SILSILLLLLLFS; ILSILLLLLLFSC;<br>LSILLLLLLFSCK; SILLLLLLFSCKQ; ILLLLLLFSCKQY;<br>LLLLLLFSCKQYG; LLLLLFSCKQYGD; LLLLFSCKQYGDV;<br>LLLFSCKQYGDVK; LLFSCKQYGDVKS; LFSCKQYGDVKSL;<br>FSCKQYGDVKSLT; SCKQYGDVKSLTE; CKQYGDVKSLTEV;<br>KQYGDVKSLTEVA; QYGDVKSLTEVAT; YGDVKSLTEVATD;<br>GDVKSLTEVATDL; DVKSLTEVATDLE; VKSLTEVATDLED;<br>KSLTEVATDLEDD; SLTEVATDLEDDN; LTEVATDLEDDNS;<br>TEVATDLEDDNSF; EVATDLEDDNSFA; VATDLEDDNSFAS;<br>ATDLEDDNSFASG; TDLEDDNSFASGS; DLEDDNSFASGSV;<br>LEDDNSFASGSVE; EDDNSFASGSVES; DDNSFASGSVESK;<br>DNSFASGSVESKD; NSFASGSVESKDQ; SFASGSVESKDQI;<br>FASGSVESKDQII; ASGSVESKDQIIE; SGSVESKDQIIEK;<br>GSVESKDQIIEKG; SVESKDQIIEKGP; VESKDQIIEKGPV;<br>ESKDQIIEKGPVL; SKDQIIEKGPVLT; KDQIIEKGPVLTS;<br>DQIIEKGPVLTSE; QIIEKGPVLTSEE; IIEKGPVLTSEEF;<br>IEKGPVLTSEEFE; EKGPVLTSEEFER; KGPVLTSEEFERL;<br>GPVLTSEEFERLE; PVLTSEEFERLEA; VLTSEEFERLEAL;<br>LTSEEFERLEALK; TSEEFERLEALKT; SEEFERLEALKTF;<br>EEFERLEALKTFL; EFERLEALKTFLK; FERLEALKTFLKD;<br>ERLEALKTFLKDA; RLEALKTFLKDAM; LEALKTFLKDAMG;<br>EALKTFLKDAMGV; ALKTFLKDAMGVN; LKTFLKDAMGVNG;<br>KTFLKDAMGVNGR; TFLKDAMGVNGRE; FLKDAMGVNGREG; |

LKDAMGVNGREGD; KDAMGVNGREGDT; DAMGVNGREGDTK;
AMGVNGREGDTKA; MGVNGREGDTKAE; GVNGREGDTKAEY;
VNGREGDTKAEYE; NGREGDTKAEYEK; GREGDTKAEYEKS;
REGDTKAEYEKSY; EGDTKAEYEKSYK; GDTKAEYEKSYKE;
DTKAEYEKSYKEF; TKAEYEKSYKEFF; KAEYEKSYKEFFD;
AEYEKSYKEFFDW; EYEKSYKEFFDWL; YEKSYKEFFDWLS;
EKSYKEFFDWLSK; KSYKEFFDWLSKD; SYKEFFDWLSKDV;
YKEFFDWLSKDVN; KEFFDWLSKDVNR; EFFDWLSKDVNRQ;
FFDWLSKDVNRQK; FDWLSKDVNRQKE; DWLSKDVNRQKEF;
WLSKDVNRQKEFV; LSKDVNRQKEFVS; SKDVNRQKEFVSF;
KDVNRQKEFVSFF; DVNRQKEFVSFFN; VNRQKEFVSFFNN;
NRQKEFVSFFNNI; RQKEFVSFFNNIC; QKEFVSFFNNICG;
KEFVSFFNNICGI; EFVSFFNNICGII; FVSFFNNICGIIT;
VSFFNNICGIITK; SFFNNICGIITKA; FFNNICGIITKAV;
FNNICGIITKAVD; NNICGIITKAVDA; NICGIITKAVDAS;
ICGIITKAVDASK; CGIITKAVDASKK; GIITKAVDASKKR;
IITKAVDASKKRY; ITKAVDASKKRYN; TKAVDASKKRYNS;
KAVDASKKRYNSN; AVDASKKRYNSNP; VDASKKRYNSNPK;
DASKKRYNSNPKS; ASKKRYNSNPKSL; SKKRYNSNPKSLG;
KKRYNSNPKSLGF; KRYNSNPKSLGFN; RYNSNPKSLGFNE;
YNSNPKSLGFNEY; NSNPKSLGFNEYV; SNPKSLGFNEYVC;
NPKSLGFNEYVCY; PKSLGFNEYVCYD; KSLGFNEYVCYDI;
SLGFNEYVCYDIK; LGFNEYVCYDIKT; GFNEYVCYDIKTR;
FNEYVCYDIKTRT; NEYVCYDIKTRTG; EYVCYDIKTRTGD;
YVCYDIKTRTGDD; VCYDIKTRTGDDL; CYDIKTRTGDDLS;
YDIKTRTGDDLSL; DIKTRTGDDLSLF; IKTRTGDDLSLFF;
KTRTGDDLSLFFQ; TRTGDDLSLFFQK; RTGDDLSLFFQKV;
TGDDLSLFFQKVA; GDDLSLFFQKVAD; DDLSLFFQKVADA;
DLSLFFQKVADAF; LSLFFQKVADAFG; SLFFQKVADAFGT;
LFFQKVADAFGTQ; FFQKVADAFGTQE; FQKVADAFGTQEY;
QKVADAFGTQEYK; KVADAFGTQEYKN; VADAFGTQEYKNK;
ADAFGTQEYKNKD; DAFGTQEYKNKDE; AFGTQEYKNKDED;
FGTQEYKNKDEDD; GTQEYKNKDEDDE; TQEYKNKDEDDEN;
QEYKNKDEDDENN; EYKNKDEDDENNQ; YKNKDEDDENNQK;
KNKDEDDENNQKP; NKDEDDENNQKPE; KDEDDENNQKPEK;
DEDDENNQKPEKC; EDDENNQKPEKCN; DDENNQKPEKCNE;
DENNQKPEKCNEE; ENNQKPEKCNEEI; NNQKPEKCNEEIF;
NQKPEKCNEEIFK; QKPEKCNEEIFKV; KPEKCNEEIFKVI;
PEKCNEEIFKVIK; EKCNEEIFKVIKR; KCNEEIFKVIKRV;
CNEEIFKVIKRVF; NEEIFKVIKRVFT; EEIFKVIKRVFTE;
EIFKVIKRVFTES; IFKVIKRVFTESE; FKVIKRVFTESEN;
KVIKRVFTESENN; VIKRVFTESENNN; IKRVFTESENNNE;
KRVFTESENNNEL; RVFTESENNNELA; VFTESENNNELAN;
FTESENNNELANL; TESENNNELANLK; ESENNNELANLKN;
SENNNELANLKNL; ENNNELANLKNLN; NNNELANLKNLNS;
NNELANLKNLNSY; NELANLKNLNSYN; ELANLKNLNSYNL;
LANLKNLNSYNLN; ANLKNLNSYNLNS; NLKNLNSYNLNSN;
LKNLNSYNLNSNN; KNLNSYNLNSNNK;

14 mers:
MKRISILSILLLLL; KRISILSILLLLLL; RISILSILLLLLLF;

ISILSILLLLLLFS; SILSILLLLLLFSC; ILSILLLLLLFSCK;
LSILLLLLLFSCKQ; SILLLLLLFSCKQY; ILLLLLLFSCKQYG;
LLLLLLFSCKQYGD; LLLLLFSCKQYGDV; LLLLFSCKQYGDVK;
LLLFSCKQYGDVKS; LLFSCKQYGDVKSL; LFSCKQYGDVKSLT;
FSCKQYGDVKSLTE; SCKQYGDVKSLTEV; CKQYGDVKSLTEVA;
KQYGDVKSLTEVAT; QYGDVKSLTEVATD; YGDVKSLTEVATDL;
GDVKSLTEVATDLE; DVKSLTEVATDLED; VKSLTEVATDLEDD;
KSLTEVATDLEDDN; SLTEVATDLEDDNS; LTEVATDLEDDNSF;
TEVATDLEDDNSFA; EVATDLEDDNSFAS; VATDLEDDNSFASG;
ATDLEDDNSFASGS; TDLEDDNSFASGSV; DLEDDNSFASGSVE;
LEDDNSFASGSVES; EDDNSFASGSVESK; DDNSFASGSVESKD;
DNSFASGSVESKDQ; NSFASGSVESKDQI; SFASGSVESKDQII;
FASGSVESKDQIIE; ASGSVESKDQIIEK; SGSVESKDQIIEKG;
GSVESKDQIIEKGP; SVESKDQIIEKGPV; VESKDQIIEKGPVL;
ESKDQIIEKGPVLT; SKDQIIEKGPVLTS; KDQIIEKGPVLTSE;
DQIIEKGPVLTSEE; QIIEKGPVLTSEEF; IIEKGPVLTSEEFE;
IEKGPVLTSEEFER; EKGPVLTSEEFERL; KGPVLTSEEFERLE;
GPVLTSEEFERLEA; PVLTSEEFERLEAL; VLTSEEFERLEALK;
LTSEEFERLEALKT; TSEEFERLEALKTF; SEEFERLEALKTFL;
EEFERLEALKTFLK; EFERLEALKTFLKD; FERLEALKTFLKDA;
ERLEALKTFLKDAM; RLEALKTFLKDAMG; LEALKTFLKDAMGV;
EALKTFLKDAMGVN; ALKTFLKDAMGVNG; LKTFLKDAMGVNGR;
KTFLKDAMGVNGRE; TFLKDAMGVNGREG; FLKDAMGVNGREGD;
LKDAMGVNGREGDT; KDAMGVNGREGDTK; DAMGVNGREGDTKA;
AMGVNGREGDTKAE; MGVNGREGDTKAEY; GVNGREGDTKAEYE;
VNGREGDTKAEYEK; NGREGDTKAEYEKS; GREGDTKAEYEKSY;
REGDTKAEYEKSYK; EGDTKAEYEKSYKE; GDTKAEYEKSYKEF;
DTKAEYEKSYKEFF; TKAEYEKSYKEFFD; KAEYEKSYKEFFDW;
AEYEKSYKEFFDWL; EYEKSYKEFFDWLS; YEKSYKEFFDWLSK;
EKSYKEFFDWLSKD; KSYKEFFDWLSKDV; SYKEFFDWLSKDVN;
YKEFFDWLSKDVNR; KEFFDWLSKDVNRQ; EFFDWLSKDVNRQK;
FFDWLSKDVNRQKE; FDWLSKDVNRQKEF; DWLSKDVNRQKEFV;
WLSKDVNRQKEFVS; LSKDVNRQKEFVSF; SKDVNRQKEFVSFF;
KDVNRQKEFVSFFN; DVNRQKEFVSFFNN; VNRQKEFVSFFNNI;
NRQKEFVSFFNNIC; RQKEFVSFFNNICG; QKEFVSFFNNICGI;
KEFVSFFNNICGII; EFVSFFNNICGIIT; FVSFFNNICGIITK;
VSFFNNICGIITKA; SFFNNICGIITKAV; FFNNICGIITKAVD;
FNNICGIITKAVDA; NNICGIITKAVDAS; NICGIITKAVDASK;
ICGIITKAVDASKK; CGIITKAVDASKKR; GIITKAVDASKKRY;
IITKAVDASKKRYN; ITKAVDASKKRYNS; TKAVDASKKRYNSN;
KAVDASKKRYNSNP; AVDASKKRYNSNPK; VDASKKRYNSNPKS;
DASKKRYNSNPKSL; ASKKRYNSNPKSLG; SKKRYNSNPKSLGF;
KKRYNSNPKSLGFN; KRYNSNPKSLGFNE; RYNSNPKSLGFNEY;
YNSNPKSLGFNEYV; NSNPKSLGFNEYVC; SNPKSLGFNEYVCY;
NPKSLGFNEYVCYD; PKSLGFNEYVCYDI; KSLGFNEYVCYDIK;
SLGFNEYVCYDIKT; LGFNEYVCYDIKTR; GFNEYVCYDIKTRT;
FNEYVCYDIKTRTG; NEYVCYDIKTRTGD; EYVCYDIKTRTGDD;
YVCYDIKTRTGDDL; VCYDIKTRTGDDLS; CYDIKTRTGDDLSL;
YDIKTRTGDDLSLF; DIKTRTGDDLSLFF; IKTRTGDDLSLFFQ;
KTRTGDDLSLFFQK; TRTGDDLSLFFQKV; RTGDDLSLFFQKVA;
TGDDLSLFFQKVAD; GDDLSLFFQKVADA; DDLSLFFQKVADAF;

DLSLFFQKVADAFG; LSLFFQKVADAFGT; SLFFQKVADAFGTQ;
LFFQKVADAFGTQE; FFQKVADAFGTQEY; FQKVADAFGTQEYK;
QKVADAFGTQEYKN; KVADAFGTQEYKNK; VADAFGTQEYKNKD;
ADAFGTQEYKNKDE; DAFGTQEYKNKDED; AFGTQEYKNKDEDD;
FGTQEYKNKDEDDE; GTQEYKNKDEDDEN; TQEYKNKDEDDENN;
QEYKNKDEDDENNQ; EYKNKDEDDENNQK; YKNKDEDDENNQKP;
KNKDEDDENNQKPE; NKDEDDENNQKPEK; KDEDDENNQKPEKC;
DEDDENNQKPEKCN; EDDENNQKPEKCNE; DDENNQKPEKCNEE;
DENNQKPEKCNEEI; ENNQKPEKCNEEIF; NNQKPEKCNEEIFK;
NQKPEKCNEEIFKV; QKPEKCNEEIFKVI; KPEKCNEEIFKVIK;
PEKCNEEIFKVIKR; EKCNEEIFKVIKRV; KCNEEIFKVIKRVF;
CNEEIFKVIKRVFT; NEEIFKVIKRVFTE; EEIFKVIKRVFTES;
EIFKVIKRVFTESE; IFKVIKRVFTESEN; FKVIKRVFTESENN;
KVIKRVFTESENNN; VIKRVFTESENNNE; IKRVFTESENNNEL;
KRVFTESENNNELA; RVFTESENNNELAN; VFTESENNNELANL;
FTESENNNELANLK; TESENNNELANLKN; ESENNNELANLKNL;
SENNNELANLKNLN; ENNNELANLKNLNS; NNNELANLKNLNSY;
NNELANLKNLNSYN; NELANLKNLNSYNL; ELANLKNLNSYNLN;
LANLKNLNSYNLNS; ANLKNLNSYNLNSN; NLKNLNSYNLNSNN;
LKNLNSYNLNSNNK;

15 mers:
MKRISILSILLLLLL; KRISILSILLLLLLF; RISILSILLLLLLFS;
ISILSILLLLLLFSC; SILSILLLLLLFSCK; ILSILLLLLLFSCKQ;
LSILLLLLLFSCKQY; SILLLLLLFSCKQYG; ILLLLLLFSCKQYGD;
LLLLLLFSCKQYGDV; LLLLLFSCKQYGDVK; LLLLFSCKQYGDVKS;
LLLFSCKQYGDVKSL; LLFSCKQYGDVKSLTE; LFSCKQYGDVKSLTE;
FSCKQYGDVKSLTEV; SCKQYGDVKSLTEVA; CKQYGDVKSLTEVAT;
KQYGDVKSLTEVATD; QYGDVKSLTEVATDL; YGDVKSLTEVATDLE;
GDVKSLTEVATDLED; DVKSLTEVATDLEDD; VKSLTEVATDLEDDN;
KSLTEVATDLEDDNS; SLTEVATDLEDDNSF; LTEVATDLEDDNSFA;
TEVATDLEDDNSFAS; EVATDLEDDNSFASG; VATDLEDDNSFASGS;
ATDLEDDNSFASGSV; TDLEDDNSFASGSVE; DLEDDNSFASGSVES;
LEDDNSFASGSVESK; EDDNSFASGSVESKD; DDNSFASGSVESKDQ;
DNSFASGSVESKDQI; NSFASGSVESKDQII; SFASGSVESKDQIIE;
FASGSVESKDQIIEK; ASGSVESKDQIIEKG; SGSVESKDQIIEKGP;
GSVESKDQIIEKGPV; SVESKDQIIEKGPVL; VESKDQIIEKGPVLT;
ESKDQIIEKGPVLTS; SKDQIIEKGPVLTSE; KDQIIEKGPVLTSEE;
DQIIEKGPVLTSEEF; QIIEKGPVLTSEEFE; IIEKGPVLTSEEFER;
IEKGPVLTSEEFERL; EKGPVLTSEEFERLE; KGPVLTSEEFERLEA;
GPVLTSEEFERLEAL; PVLTSEEFERLEALK; VLTSEEFERLEALKT;
LTSEEFERLEALKTF; TSEEFERLEALKTFL; SEEFERLEALKTFLK;
EEFERLEALKTFLKD; EFERLEALKTFLKDA; FERLEALKTFLKDAM;
ERLEALKTFLKDAMG; RLEALKTFLKDAMGV; LEALKTFLKDAMGVN;
EALKTFLKDAMGVNG; ALKTFLKDAMGVNGR; LKTFLKDAMGVNGRE;
KTFLKDAMGVNGREG; TFLKDAMGVNGREGD; FLKDAMGVNGREGDT;
LKDAMGVNGREGDTK; KDAMGVNGREGDTKA; DAMGVNGREGDTKAE;
AMGVNGREGDTKAEY; MGVNGREGDTKAEYE; GVNGREGDTKAEYEK;
VNGREGDTKAEYEKS; NGREGDTKAEYEKSY; GREGDTKAEYEKSYK;
REGDTKAEYEKSYKE; EGDTKAEYEKSYKEF; GDTKAEYEKSYKEFF;
DTKAEYEKSYKEFFD; TKAEYEKSYKEFFDW; KAEYEKSYKEFFDWL;

AEYEKSYKEFFDWLS; EYEKSYKEFFDWLSK; YEKSYKEFFDWLSKD;
EKSYKEFFDWLSKDV; KSYKEFFDWLSKDVN; SYKEFFDWLSKDVNR;
YKEFFDWLSKDVNRQ; KEFFDWLSKDVNRQK; EFFDWLSKDVNRQKE;
FFDWLSKDVNRQKEF; FDWLSKDVNRQKEFV; DWLSKDVNRQKEFVS;
WLSKDVNRQKEFVSF; LSKDVNRQKEFVSFF; SKDVNRQKEFVSFFN;
KDVNRQKEFVSFFNN; DVNRQKEFVSFFNNI; VNRQKEFVSFFNNIC;
NRQKEFVSFFNNICG; RQKEFVSFFNNICGI; QKEFVSFFNNICGII;
KEFVSFFNNICGIIT; EFVSFFNNICGIITK; FVSFFNNICGIITKA;
VSFFNNICGIITKAV; SFFNNICGIITKAVD; FFNNICGIITKAVDA;
FNNICGIITKAVDAS; NNICGIITKAVDASK; NICGIITKAVDASKK;
ICGIITKAVDASKKR; CGIITKAVDASKKRY; GIITKAVDASKKRYN;
IITKAVDASKKRYNS; ITKAVDASKKRYNSN; TKAVDASKKRYNSNP;
KAVDASKKRYNSNPK; AVDASKKRYNSNPKS; VDASKKRYNSNPKSL;
DASKKRYNSNPKSLG; ASKKRYNSNPKSLGF; SKKRYNSNPKSLGFN;
KKRYNSNPKSLGFNE; KRYNSNPKSLGFNEY; RYNSNPKSLGFNEYV;
YNSNPKSLGFNEYVC; NSNPKSLGFNEYVCY; SNPKSLGFNEYVCYD;
NPKSLGFNEYVCYDI; PKSLGFNEYVCYDIK; KSLGFNEYVCYDIKT;
SLGFNEYVCYDIKTR; LGFNEYVCYDIKTRT; GFNEYVCYDIKTRTG;
FNEYVCYDIKTRTGD; NEYVCYDIKTRTGDD; EYVCYDIKTRTGDDL;
YVCYDIKTRTGDDLS; VCYDIKTRTGDDLSL; CYDIKTRTGDDLSLF;
YDIKTRTGDDLSLFF; DIKTRTGDDLSLFFQ; IKTRTGDDLSLFFQK;
KTRTGDDLSLFFQKV; TRTGDDLSLFFQKVA; RTGDDLSLFFQKVAD;
TGDDLSLFFQKVADA; GDDLSLFFQKVADAF; DDLSLFFQKVADAFG;
DLSLFFQKVADAFGT; LSLFFQKVADAFGTQ; SLFFQKVADAFGTQE;
LFFQKVADAFGTQEY; FFQKVADAFGTQEYK; FQKVADAFGTQEYKN;
QKVADAFGTQEYKNK; KVADAFGTQEYKNKD; VADAFGTQEYKNKDE;
ADAFGTQEYKNKDED; DAFGTQEYKNKDEDD; AFGTQEYKNKDEDDE;
FGTQEYKNKDEDDEN; GTQEYKNKDEDDENN; TQEYKNKDEDDENNQ;
QEYKNKDEDDENNQK; EYKNKDEDDENNQKP; YKNKDEDDENNQKPE;
KNKDEDDENNQKPEK; NKDEDDENNQKPEKC; KDEDDENNQKPEKCN;
DEDDENNQKPEKCNE; EDDENNQKPEKCNEE; DDENNQKPEKCNEEI;
DENNQKPEKCNEEIF; ENNQKPEKCNEEIFK; NNQKPEKCNEEIFKV;
NQKPEKCNEEIFKVI; QKPEKCNEEIFKVIK; KPEKCNEEIFKVIKR;
PEKCNEEIFKVIKRV; EKCNEEIFKVIKRVF; KCNEEIFKVIKRVFT;
CNEEIFKVIKRVFTE; NEEIFKVIKRVFTES; EEIFKVIKRVFTESE;
EIFKVIKRVFTESEN; IFKVIKRVFTESENN; FKVIKRVFTESENNN;
KVIKRVFTESENNNE; VIKRVFTESENNNEL; IKRVFTESENNNELA;
KRVFTESENNNELAN; RVFTESENNNELANL; VFTESENNNELANLK;
FTESENNNELANLKN; TESENNNELANLKNL; ESENNNELANLKNLN;
SENNNELANLKNLNS; ENNNELANLKNLNSY; NNNELANLKNLNSYN;
NNELANLKNLNSYNL; NELANLKNLNSYNLN; ELANLKNLNSYNLNS;
LANLKNLNSYNLNSN; ANLKNLNSYNLNSNN; NLKNLNSYNLNSNNK;

16 mers:
MKRISILSILLLLLLF; KRISILSILLLLLLFS; RISILSILLLLLLFSC;
ISILSILLLLLLFSCK; SILSILLLLLLFSCKQ; ILSILLLLLLFSCKQY;
LSILLLLLLFSCKQYG; SILLLLLLFSCKQYGD; ILLLLLLFSCKQYGDV;
LLLLLLFSCKQYGDVK; LLLLLFSCKQYGDVKS; LLLLFSCKQYGDVKSL;
LLLFSCKQYGDVKSLT; LLFSCKQYGDVKSLTE; LFSCKQYGDVKSLTEV;
FSCKQYGDVKSLTEVA; SCKQYGDVKSLTEVAT; CKQYGDVKSLTEVATD;
KQYGDVKSLTEVATDL; QYGDVKSLTEVATDLE; YGDVKSLTEVATDLED;

| | |
|---|---|
| GDVKSLTEVATDLEDD; DVKSLTEVATDLEDDN; VKSLTEVATDLEDDNS; | |
| KSLTEVATDLEDDNSF; SLTEVATDLEDDNSFA; LTEVATDLEDDNSFAS; | |
| TEVATDLEDDNSFASG; EVATDLEDDNSFASGS; VATDLEDDNSFASGSV; | |
| ATDLEDDNSFASGSVE; TDLEDDNSFASGSVES; DLEDDNSFASGSVESK; | |
| LEDDNSFASGSVESKD; EDDNSFASGSVESKDQ; DDNSFASGSVESKDQI; | |
| DNSFASGSVESKDQII; NSFASGSVESKDQIIE; SFASGSVESKDQIIEK; | |
| FASGSVESKDQIIEKG; ASGSVESKDQIIEKGP; SGSVESKDQIIEKGPV; | |
| GSVESKDQIIEKGPVL; SVESKDQIIEKGPVLT; VESKDQIIEKGPVLTS; | |
| ESKDQIIEKGPVLTSE; SKDQIIEKGPVLTSEE; KDQIIEKGPVLTSEEF; | |
| DQIIEKGPVLTSEEFE; QIIEKGPVLTSEEFER; IIEKGPVLTSEEFERL; | |
| IEKGPVLTSEEFERLE; EKGPVLTSEEFERLEA; KGPVLTSEEFERLEAL; | |
| GPVLTSEEFERLEALK; PVLTSEEFERLEALKT; VLTSEEFERLEALKTF; | |
| LTSEEFERLEALKTFL; TSEEFERLEALKTFLK; SEEFERLEALKTFLKD; | |
| EEFERLEALKTFLKDA; EFERLEALKTFLKDAM; FERLEALKTFLKDAMG; | |
| ERLEALKTFLKDAMGV; RLEALKTFLKDAMGVN; LEALKTFLKDAMGVNG; | |
| EALKTFLKDAMGVNGR; ALKTFLKDAMGVNGRE; LKTFLKDAMGVNGREG; | |
| KTFLKDAMGVNGREGD; TFLKDAMGVNGREGDT; FLKDAMGVNGREGDTK; | |
| LKDAMGVNGREGDTKA; KDAMGVNGREGDTKAE; DAMGVNGREGDTKAEY; | |
| AMGVNGREGDTKAEYE; MGVNGREGDTKAEYEK; GVNGREGDTKAEYEKS; | |
| VNGREGDTKAEYEKSY; NGREGDTKAEYEKSYK; GREGDTKAEYEKSYKE; | |
| REGDTKAEYEKSYKEF; EGDTKAEYEKSYKEFF; GDTKAEYEKSYKEFFD; | |
| DTKAEYEKSYKEFFDW; TKAEYEKSYKEFFDWL; KAEYEKSYKEFFDWLS; | |
| AEYEKSYKEFFDWLSK; EYEKSYKEFFDWLSKD; YEKSYKEFFDWLSKDV; | |
| EKSYKEFFDWLSKDVN; KSYKEFFDWLSKDVNR; SYKEFFDWLSKDVNRQ; | |
| YKEFFDWLSKDVNRQK; KEFFDWLSKDVNRQKE; EFFDWLSKDVNRQKEF; | |
| FFDWLSKDVNRQKEFV; FDWLSKDVNRQKEFVS; DWLSKDVNRQKEFVSF; | |
| WLSKDVNRQKEFVSFF; LSKDVNRQKEFVSFFN; SKDVNRQKEFVSFFNN; | |
| KDVNRQKEFVSFFNNI; DVNRQKEFVSFFNNIC; VNRQKEFVSFFNNICG; | |
| NRQKEFVSFFNNICGI; RQKEFVSFFNNICGII; QKEFVSFFNNICGIIT; | |
| KEFVSFFNNICGIITK; EFVSFFNNICGIITKA; FVSFFNNICGIITKAV; | |
| VSFFNNICGIITKAVD; SFFNNICGIITKAVDA; FFNNICGIITKAVDAS; | |
| FNNICGIITKAVDASK; NNICGIITKAVDASKK; NICGIITKAVDASKKR; | |
| ICGIITKAVDASKKRY; CGIITKAVDASKKRYN; GIITKAVDASKKRYNS; | |
| IITKAVDASKKRYNSN; ITKAVDASKKRYNSNP; TKAVDASKKRYNSNPK; | |
| KAVDASKKRYNSNPKS; AVDASKKRYNSNPKSL; VDASKKRYNSNPKSLG; | |
| DASKKRYNSNPKSLGF; ASKKRYNSNPKSLGFN; SKKRYNSNPKSLGFNE; | |
| KKRYNSNPKSLGFNEY; KRYNSNPKSLGFNEYV; RYNSNPKSLGFNEYVC; | |
| YNSNPKSLGFNEYVCY; NSNPKSLGFNEYVCYD; SNPKSLGFNEYVCYDI; | |
| NPKSLGFNEYVCYDIK; PKSLGFNEYVCYDIKT; KSLGFNEYVCYDIKTR; | |
| SLGFNEYVCYDIKTRT; LGFNEYVCYDIKTRTG; GFNEYVCYDIKTRTGD; | |
| FNEYVCYDIKTRTGDD; NEYVCYDIKTRTGDDL; EYVCYDIKTRTGDDLS; | |
| YVCYDIKTRTGDDLSL; VCYDIKTRTGDDLSLF; CYDIKTRTGDDLSLFF; | |
| YDIKTRTGDDLSLFFQ; DIKTRTGDDLSLFFQK; IKTRTGDDLSLFFQKV; | |
| KTRTGDDLSLFFQKVA; TRTGDDLSLFFQKVAD; RTGDDLSLFFQKVADA; | |
| TGDDLSLFFQKVADAF; GDDLSLFFQKVADAFG; DDLSLFFQKVADAFGT; | |
| DLSLFFQKVADAFGTQ; LSLFFQKVADAFGTQE; SLFFQKVADAFGTQEY; | |
| LFFQKVADAFGTQEYK; FFQKVADAFGTQEYKN; FQKVADAFGTQEYKNK; | |
| QKVADAFGTQEYKNKD; KVADAFGTQEYKNKDE; VADAFGTQEYKNKDED; | |
| ADAFGTQEYKNKDEDD; DAFGTQEYKNKDEDDE; AFGTQEYKNKDEDDEN; | |
| FGTQEYKNKDEDDENN; GTQEYKNKDEDDENNQ; TQEYKNKDEDDENNQK; | |
| QEYKNKDEDDENNQKP; EYKNKDEDDENNQKPE; YKNKDEDDENNQKPEK; | |

| | |
|---|---|
| | KNKDEDDENNQKPEKC; NKDEDDENNQKPEKCN; KDEDDENNQKPEKCNE; DEDDENNQKPEKCNEE; EDDENNQKPEKCNEEI; DDENNQKPEKCNEEIF; DENNQKPEKCNEEIFK; ENNQKPEKCNEEIFKV; NNQKPEKCNEEIFKVI; NQKPEKCNEEIFKVIK; QKPEKCNEEIFKVIKR; KPEKCNEEIFKVIKRV; PEKCNEEIFKVIKRVF; EKCNEEIFKVIKRVFT; KCNEEIFKVIKRVFTE; CNEEIFKVIKRVFTES; NEEIFKVIKRVFTESE; EEIFKVIKRVFTESEN; EIFKVIKRVFTESENN; IFKVIKRVFTESENNN; FKVIKRVFTESENNNE; KVIKRVFTESENNNEL; VIKRVFTESENNNELA; IKRVFTESENNNELAN; KRVFTESENNNELANL; RVFTESENNNELANLK; VFTESENNNELANLKN; FTESENNNELANLKNL; TESENNNELANLKNLN; ESENNNELANLKNLNS; SENNNELANLKNLNSY; ENNNELANLKNLNSYN; NNNELANLKNLNSYNL; NNELANLKNLNSYNLN; NELANLKNLNSYNLNS; ELANLKNLNSYNLNSN; LANLKNLNSYNLNSNN; ANLKNLNSYNLNSNNK; |
| Seq 34 | 13 mers: MKIKPLIQLKLLG; KIKPLIQLKLLGL; IKPLIQLKLLGLF; KPLIQLKLLGLFL; PLIQLKLLGLFLF; LIQLKLLGLFLFS; IQLKLLGLFLFSC; QLKLLGLFLFSCT; LKLLGLFLFSCTI; KLLGLFLFSCTID; LLGLFLFSCTIDA; LGLFLFSCTIDAN; GLFLFSCTIDANL; LFLFSCTIDANLN; FLFSCTIDANLNE; LFSCTIDANLNED; FSCTIDANLNEDY; SCTIDANLNEDYK; CTIDANLNEDYKN; TIDANLNEDYKNK; IDANLNEDYKNKV; DANLNEDYKNKVK; ANLNEDYKNKVKG; NLNEDYKNKVKGI; LNEDYKNKVKGIL; NEDYKNKVKGILN; EDYKNKVKGILNK; DYKNKVKGILNKA; YKNKVKGILNKAA; KNKVKGILNKAAD; NKVKGILNKAADD; KVKGILNKAADDQ; VKGILNKAADDQE; KGILNKAADDQET; GILNKAADDQETT; ILNKAADDQETTS; LNKAADDQETTSA; NKAADDQETTSAD; KAADDQETTSADT; AADDQETTSADTN; ADDQETTSADTNS; DDQETTSADTNSN; DQETTSADTNSNA; QETTSADTNSNAA; ETTSADTNSNAAK; TTSADTNSNAAKN; TSADTNSNAAKNI; SADTNSNAAKNIP; ADTNSNAAKNIPI; DTNSNAAKNIPIA; TNSNAAKNIPIAD; NSNAAKNIPIADN; SNAAKNIPIADND; NAAKNIPIADNDK; AAKNIPIADNDKV; AKNIPIADNDKVA; KNIPIADNDKVAA; NIPIADNDKVAAE; IPIADNDKVAAEL; PIADNDKVAAELK; IADNDKVAAELKK; ADNDKVAAELKKQ; DNDKVAAELKKQS; NDKVAAELKKQSQ; DKVAAELKKQSQA; KVAAELKKQSQAA; VAAELKKQSQAAK; AAELKKQSQAAKT; AELKKQSQAAKTV; ELKKQSQAAKTVA; LKKQSQAAKTVAA; KKQSQAAKTVAAA; KQSQAAKTVAAAP; QSQAAKTVAAAPN; SQAAKTVAAAPNK; QAAKTVAAAPNKG; AAKTVAAAPNKGS; AKTVAAAPNKGSQ; KTVAAAPNKGSQN; TVAAAPNKGSQNQ; VAAAPNKGSQNQP; AAAPNKGSQNQPQ; AAPNKGSQNQPQT; APNKGSQNQPQTT; PNKGSQNQPQTTP; NKGSQNQPQTTPN; KGSQNQPQTTPNK; GSQNQPQTTPNKG; SQNQPQTTPNKGS; QNQPQTTPNKGSQ; NQPQTTPNKGSQN; QPQTTPNKGSQNQ; PQTTPNKGSQNQQ; QTTPNKGSQNQQA; TTPNKGSQNQQAA; TPNKGSQNQQAAP; PNKGSQNQQAAPS; NKGSQNQQAAPSP; KGSQNQQAAPSPQ; GSQNQQAAPSPQL; SQNQQAAPSPQLQ; QNQQAAPSPQLQS; NQQAAPSPQLQSL; QQAAPSPQLQSLS; QAAPSPQLQSLSF; |

AAPSPQLQSLSFS; APSPQLQSLSFSA; PSPQLQSLSFSAD;
SPQLQSLSFSADL; PQLQSLSFSADLS; QLQSLSFSADLSN;
LQSLSFSADLSNL; QSLSFSADLSNLP; SLSFSADLSNLPK;
LSFSADLSNLPKT; SFSADLSNLPKTT; FSADLSNLPKTTA;
SADLSNLPKTTAA; ADLSNLPKTTAAR; DLSNLPKTTAARA;
LSNLPKTTAARAA; SNLPKTTAARAAS; NLPKTTAARAASL;
LPKTTAARAASLT; PKTTAARAASLTK; KTTAARAASLTKQ;
TTAARAASLTKQR; TAARAASLTKQRI; AARAASLTKQRIP;
ARAASLTKQRIPI; RAASLTKQRIPIQ; AASLTKQRIPIQA;
ASLTKQRIPIQAV; SLTKQRIPIQAVT; LTKQRIPIQAVTT;
TKQRIPIQAVTTV; KQRIPIQAVTTVP; QRIPIQAVTTVPG;
RIPIQAVTTVPGN; IPIQAVTTVPGNT; PIQAVTTVPGNTR;
IQAVTTVPGNTRT; QAVTTVPGNTRTF; AVTTVPGNTRTFN;
VTTVPGNTRTFNS; TTVPGNTRTFNSR; TVPGNTRTFNSRN;
VPGNTRTFNSRNS; PGNTRTFNSRNSG; GNTRTFNSRNSGL;
NTRTFNSRNSGLP; TRTFNSRNSGLPT; RTFNSRNSGLPTF;
TFNSRNSGLPTFA; FNSRNSGLPTFAL; NSRNSGLPTFALN;
SRNSGLPTFALNY; RNSGLPTFALNYS; NSGLPTFALNYSF;
SGLPTFALNYSFS; GLPTFALNYSFSQ; LPTFALNYSFSQP;
PTFALNYSFSQPT; TFALNYSFSQPTR; FALNYSFSQPTRQ;
ALNYSFSQPTRQQ; LNYSFSQPTRQQT; NYSFSQPTRQQTN;
YSFSQPTRQQTNS; SFSQPTRQQTNSS; FSQPTRQQTNSSS;
SQPTRQQTNSSSA; QPTRQQTNSSSAV; PTRQQTNSSSAVQ;
TRQQTNSSSAVQT; RQQTNSSSAVQTT; QQTNSSSAVQTTT;
QTNSSSAVQTTTS; TNSSSAVQTTTSS; NSSSAVQTTTSSG;
SSSAVQTTTSSGS; SSAVQTTTSSGSK; SAVQTTTSSGSKL;
AVQTTTSSGSKLQ; VQTTTSSGSKLQT; QTTTSSGSKLQTL;
TTTSSGSKLQTLK; TTSSGSKLQTLKN; TSSGSKLQTLKNE;
SSGSKLQTLKNEL; SGSKLQTLKNELI; GSKLQTLKNELIR;
SKLQTLKNELIRA; KLQTLKNELIRAI; LQTLKNELIRAIS;
QTLKNELIRAISE; TLKNELIRAISEE; LKNELIRAISEEK;
KNELIRAISEEKN; NELIRAISEEKNK; ELIRAISEEKNKT;
LIRAISEEKNKTQ; IRAISEEKNKTQN; RAISEEKNKTQNN;
AISEEKNKTQNNF; ISEEKNKTQNNFG; SEEKNKTQNNFGF;
EEKNKTQNNFGFR; EKNKTQNNFGFRE; KNKTQNNFGFRET;
NKTQNNFGFRETY; KTQNNFGFRETYD; TQNNFGFRETYDQ;
QNNFGFRETYDQF; NNFGFRETYDQFK; NFGFRETYDQFKM;
FGFRETYDQFKMK; GFRETYDQFKMKD; FRETYDQFKMKDS;
RETYDQFKMKDSA; ETYDQFKMKDSAF; TYDQFKMKDSAFE;
YDQFKMKDSAFEL; DQFKMKDSAFELL; QFKMKDSAFELLD;
FKMKDSAFELLDV; KMKDSAFELLDVI; MKDSAFELLDVIS;
KDSAFELLDVISS; DSAFELLDVISSA; SAFELLDVISSAK;
AFELLDVISSAKV; FELLDVISSAKVY; ELLDVISSAKVYD;
LLDVISSAKVYDR; LDVISSAKVYDRS; DVISSAKVYDRSY;
VISSAKVYDRSYA; ISSAKVYDRSYAP; SSAKVYDRSYAPQ;
SAKVYDRSYAPQL; AKVYDRSYAPQLN; KVYDRSYAPQLNS;
VYDRSYAPQLNSN; YDRSYAPQLNSNT; DRSYAPQLNSNTP;
RSYAPQLNSNTPE; SYAPQLNSNTPEA; YAPQLNSNTPEAE;
APQLNSNTPEAEN; PQLNSNTPEAENE; QLNSNTPEAENER;
LNSNTPEAENERN; NSNTPEAENERNK; SNTPEAENERNKF;
NTPEAENERNKFY; TPEAENERNKFYA; PEAENERNKFYAL;

EAENERNKFYALM; AENERNKFYALMD; ENERNKFYALMDF;
NERNKFYALMDFD; ERNKFYALMDFDQ; RNKFYALMDFDQY;
NKFYALMDFDQYK; KFYALMDFDQYKI; FYALMDFDQYKIE;
YALMDFDQYKIEQ; ALMDFDQYKIEQF; LMDFDQYKIEQFG;
MDFDQYKIEQFGS; DFDQYKIEQFGSI; FDQYKIEQFGSIM;
DQYKIEQFGSIME; QYKIEQFGSIMEA; YKIEQFGSIMEAL;
KIEQFGSIMEALY; IEQFGSIMEALYN; EQFGSIMEALYNE;
QFGSIMEALYNEN; FGSIMEALYNENQ; GSIMEALYNENQN;
SIMEALYNENQNH; IMEALYNENQNHS; MEALYNENQNHSL;
EALYNENQNHSLI; ALYNENQNHSLIR; LYNENQNHSLIRE;
YNENQNHSLIREL; NENQNHSLIRELM; ENQNHSLIRELMI;
NQNHSLIRELMIS; QNHSLIRELMISG; NHSLIRELMISGL;
HSLIRELMISGLG; SLIRELMISGLGT; LIRELMISGLGTQ;
IRELMISGLGTQI; RELMISGLGTQIS; ELMISGLGTQISF;
LMISGLGTQISFE; MISGLGTQISFEL; ISGLGTQISFELA;
SGLGTQISFELAL; GLGTQISFELALE; LGTQISFELALEE;
GTQISFELALEEI; TQISFELALEEIN; QISFELALEEINK;
ISFELALEEINKK; SFELALEEINKKI; FELALEEINKKIE;
ELALEEINKKIEI; LALEEINKKIEIF; ALEEINKKIEIFN;
LEEINKKIEIFNQ; EEINKKIEIFNQD; EINKKIEIFNQDY;
INKKIEIFNQDYL; NKKIEIFNQDYLN; KKIEIFNQDYLNA;
KIEIFNQDYLNAK; IEIFNQDYLNAKI; EIFNQDYLNAKIN;
IFNQDYLNAKINS; FNQDYLNAKINSF; NQDYLNAKINSFD;
QDYLNAKINSFDF; DYLNAKINSFDFT; YLNAKINSFDFTM;
LNAKINSFDFTMK; NAKINSFDFTMKL; AKINSFDFTMKLK;
KINSFDFTMKLKE; INSFDFTMKLKEL; NSFDFTMKLKELK;
SFDFTMKLKELKS; FDFTMKLKELKSK; DFTMKLKELKSKL;
FTMKLKELKSKLN; TMKLKELKSKLNQ; MKLKELKSKLNQI;
KLKELKSKLNQIL; LKELKSKLNQILD; KELKSKLNQILDK;
ELKSKLNQILDKR; LKSKLNQILDKRK; KSKLNQILDKRKE;
SKLNQILDKRKEW; KLNQILDKRKEWS; LNQILDKRKEWSR;
NQILDKRKEWSRQ; QILDKRKEWSRQA; ILDKRKEWSRQAD;
LDKRKEWSRQADG; DKRKEWSRQADGL; KRKEWSRQADGLI;
RKEWSRQADGLIA; KEWSRQADGLIAN; EWSRQADGLIANA;
WSRQADGLIANAS; SRQADGLIANASS; RQADGLIANASSN;
QADGLIANASSNS; ADGLIANASSNSS; DGLIANASSNSSL;
GLIANASSNSSLS; LIANASSNSSLSD; IANASSNSSLSDS;
ANASSNSSLSDSK; NASSNSSLSDSKS; ASSNSSLSDSKSL;
SSNSSLSDSKSLA; SNSSLSDSKSLAE; NSSLSDSKSLAEY;
SSLSDSKSLAEYI; SLSDSKSLAEYIK; LSDSKSLAEYIKK;
SDSKSLAEYIKKR; DSKSLAEYIKKRY; SKSLAEYIKKRYL;
KSLAEYIKKRYLD; SLAEYIKKRYLDN; LAEYIKKRYLDNM;
AEYIKKRYLDNMQ; EYIKKRYLDNMQN; YIKKRYLDNMQNA;
IKKRYLDNMQNAR; KKRYLDNMQNARQ; KRYLDNMQNARQS;
RYLDNMQNARQSV; YLDNMQNARQSVL; LDNMQNARQSVLE;
DNMQNARQSVLEA; NMQNARQSVLEAY; MQNARQSVLEAYI;
QNARQSVLEAYIS; NARQSVLEAYISI; ARQSVLEAYISIM;

14 mers:
MKIKPLIQLKLLGL; KIKPLIQLKLLGLF; IKPLIQLKLLGLFL;
KPLIQLKLLGLFLF; PLIQLKLLGLFLFS; LIQLKLLGLFLFSC;

IQLKLLGLFLFSCT; QLKLLGLFLFSCTI; LKLLGLFLFSCTID;
KLLGLFLFSCTIDA; LLGLFLFSCTIDAN; LGLFLFSCTIDANL;
GLFLFSCTIDANLN; LFLFSCTIDANLNE; FLFSCTIDANLNED;
LFSCTIDANLNEDY; FSCTIDANLNEDYK; SCTIDANLNEDYKN;
CTIDANLNEDYKNK; TIDANLNEDYKNKV; IDANLNEDYKNKVK;
DANLNEDYKNKVKG; ANLNEDYKNKVKGI; NLNEDYKNKVKGIL;
LNEDYKNKVKGILN; NEDYKNKVKGILNK; EDYKNKVKGILNKA;
DYKNKVKGILNKAA; YKNKVKGILNKAAD; KNKVKGILNKAADD;
NKVKGILNKAADDQ; KVKGILNKAADDQE; VKGILNKAADDQET;
KGILNKAADDQETT; GILNKAADDQETTS; ILNKAADDQETTSA;
LNKAADDQETTSAD; NKAADDQETTSADT; KAADDQETTSADTN;
AADDQETTSADTNS; ADDQETTSADTNSN; DDQETTSADTNSNA;
DQETTSADTNSNAA; QETTSADTNSNAAK; ETTSADTNSNAAKN;
TTSADTNSNAAKNI; TSADTNSNAAKNIP; SADTNSNAAKNIPI;
ADTNSNAAKNIPIA; DTNSNAAKNIPIAD; TNSNAAKNIPIADN;
NSNAAKNIPIADND; SNAAKNIPIADNDK; NAAKNIPIADNDKV;
AAKNIPIADNDKVA; AKNIPIADNDKVAA; KNIPIADNDKVAAE;
NIPIADNDKVAAEL; IPIADNDKVAAELK; PIADNDKVAAELKK;
IADNDKVAAELKKQ; ADNDKVAAELKKQS; DNDKVAAELKKQSQ;
NDKVAAELKKQSQA; DKVAAELKKQSQAA; KVAAELKKQSQAAK;
VAAELKKQSQAAKT; AAELKKQSQAAKTV; AELKKQSQAAKTVA;
ELKKQSQAAKTVAA; LKKQSQAAKTVAAA; KKQSQAAKTVAAAP;
KQSQAAKTVAAAPN; QSQAAKTVAAAPNK; SQAAKTVAAAPNKG;
QAAKTVAAAPNKGS; AAKTVAAAPNKGSQ; AKTVAAAPNKGSQN;
KTVAAAPNKGSQNQ; TVAAAPNKGSQNQP; VAAAPNKGSQNQPQ;
AAAPNKGSQNQPQT; AAPNKGSQNQPQTT; APNKGSQNQPQTTP;
PNKGSQNQPQTTPN; NKGSQNQPQTTPNK; KGSQNQPQTTPNKG;
GSQNQPQTTPNKGS; SQNQPQTTPNKGSQ; QNQPQTTPNKGSQN;
NQPQTTPNKGSQNQ; QPQTTPNKGSQNQQ; PQTTPNKGSQNQQA;
QTTPNKGSQNQQAA; TTPNKGSQNQQAAP; TPNKGSQNQQAAPS;
PNKGSQNQQAAPSP; NKGSQNQQAAPSPQ; KGSQNQQAAPSPQL;
GSQNQQAAPSPQLQ; SQNQQAAPSPQLQS; QNQQAAPSPQLQSL;
NQQAAPSPQLQSLS; QQAAPSPQLQSLSF; QAAPSPQLQSLSFS;
AAPSPQLQSLSFSA; APSPQLQSLSFSAD; PSPQLQSLSFSADL;
SPQLQSLSFSADLS; PQLQSLSFSADLSN; QLQSLSFSADLSNL;
LQSLSFSADLSNLP; QSLSFSADLSNLPK; SLSFSADLSNLPKT;
LSFSADLSNLPKTT; SFSADLSNLPKTTA; FSADLSNLPKTTAA;
SADLSNLPKTTAAR; ADLSNLPKTTAARA; DLSNLPKTTAARAA;
LSNLPKTTAARAAS; SNLPKTTAARAASL; NLPKTTAARAASLT;
LPKTTAARAASLTK; PKTTAARAASLTKQ; KTTAARAASLTKQR;
TTAARAASLTKQRI; TAARAASLTKQRIP; AARAASLTKQRIPI;
ARAASLTKQRIPIQ; RAASLTKQRIPIQA; AASLTKQRIPIQAV;
ASLTKQRIPIQAVT; SLTKQRIPIQAVTT; LTKQRIPIQAVTTV;
TKQRIPIQAVTTVP; KQRIPIQAVTTVPG; QRIPIQAVTTVPGN;
RIPIQAVTTVPGNT; IPIQAVTTVPGNTR; PIQAVTTVPGNTRT;
IQAVTTVPGNTRTF; QAVTTVPGNTRTFN; AVTTVPGNTRTFNS;
VTTVPGNTRTFNSR; TTVPGNTRTFNSRN; TVPGNTRTFNSRNS;
VPGNTRTFNSRNSG; PGNTRTFNSRNSGL; GNTRTFNSRNSGLP;
NTRTFNSRNSGLPT; TRTFNSRNSGLPTF; RTFNSRNSGLPTFA;
TFNSRNSGLPTFAL; FNSRNSGLPTFALN; NSRNSGLPTFALNY;
SRNSGLPTFALNYS; RNSGLPTFALNYSF; NSGLPTFALNYSFS;

SGLPTFALNYSFSQ; GLPTFALNYSFSQP; LPTFALNYSFSQPT;
PTFALNYSFSQPTR; TFALNYSFSQPTRQ; FALNYSFSQPTRQQ;
ALNYSFSQPTRQQT; LNYSFSQPTRQQTN; NYSFSQPTRQQTNS;
YSFSQPTRQQTNSS; SFSQPTRQQTNSSS; FSQPTRQQTNSSSA;
SQPTRQQTNSSSAV; QPTRQQTNSSSAVQ; PTRQQTNSSSAVQT;
TRQQTNSSSAVQTT; RQQTNSSSAVQTTT; QQTNSSSAVQTTTS;
QTNSSSAVQTTTSS; TNSSSAVQTTTSSG; NSSSAVQTTTSSGS;
SSSAVQTTTSSGSK; SSAVQTTTSSGSKL; SAVQTTTSSGSKLQ;
AVQTTTSSGSKLQT; VQTTTSSGSKLQTL; QTTTSSGSKLQTLK;
TTTSSGSKLQTLKN; TTSSGSKLQTLKNE; TSSGSKLQTLKNEL;
SSGSKLQTLKNELI; SGSKLQTLKNELIR; GSKLQTLKNELIRA;
SKLQTLKNELIRAI; KLQTLKNELIRAIS; LQTLKNELIRAISE;
QTLKNELIRAISEE; TLKNELIRAISEEK; LKNELIRAISEEKN;
KNELIRAISEEKNK; NELIRAISEEKNKT; ELIRAISEEKNKTQ;
LIRAISEEKNKTQN; IRAISEEKNKTQNN; RAISEEKNKTQNNF;
AISEEKNKTQNNFG; ISEEKNKTQNNFGF; SEEKNKTQNNFGFR;
EEKNKTQNNFGFRE; EKNKTQNNFGFRET; KNKTQNNFGFRETY;
NKTQNNFGFRETYD; KTQNNFGFRETYDQ; TQNNFGFRETYDQF;
QNNFGFRETYDQFK; NNFGFRETYDQFKM; NFGFRETYDQFKMK;
FGFRETYDQFKMKD; GFRETYDQFKMKDS; FRETYDQFKMKDSA;
RETYDQFKMKDSAF; ETYDQFKMKDSAFE; TYDQFKMKDSAFEL;
YDQFKMKDSAFELL; DQFKMKDSAFELLD; QFKMKDSAFELLDV;
FKMKDSAFELLDVI; KMKDSAFELLDVIS; MKDSAFELLDVISS;
KDSAFELLDVISSA; DSAFELLDVISSAK; SAFELLDVISSAKV;
AFELLDVISSAKVY; FELLDVISSAKVYD; ELLDVISSAKVYDR;
LLDVISSAKVYDRS; LDVISSAKVYDRSY; DVISSAKVYDRSYA;
VISSAKVYDRSYAP; ISSAKVYDRSYAPQ; SSAKVYDRSYAPQL;
SAKVYDRSYAPQLN; AKVYDRSYAPQLNS; KVYDRSYAPQLNSN;
VYDRSYAPQLNSNT; YDRSYAPQLNSNTP; DRSYAPQLNSNTPE;
RSYAPQLNSNTPEA; SYAPQLNSNTPEAE; YAPQLNSNTPEAEN;
APQLNSNTPEAENE; PQLNSNTPEAENER; QLNSNTPEAENERN;
LNSNTPEAENERNK; NSNTPEAENERNKF; SNTPEAENERNKFY;
NTPEAENERNKFYA; TPEAENERNKFYAL; PEAENERNKFYALM;
EAENERNKFYALMD; AENERNKFYALMDF; ENERNKFYALMDFD;
NERNKFYALMDFDQ; ERNKFYALMDFDQY; RNKFYALMDFDQYK;
NKFYALMDFDQYKI; KFYALMDFDQYKIE; FYALMDFDQYKIEQ;
YALMDFDQYKIEQF; ALMDFDQYKIEQFG; LMDFDQYKIEQFGS;
MDFDQYKIEQFGSI; DFDQYKIEQFGSIM; FDQYKIEQFGSIME;
DQYKIEQFGSIMEA; QYKIEQFGSIMEAL; YKIEQFGSIMEALY;
KIEQFGSIMEALYN; IEQFGSIMEALYNE; EQFGSIMEALYNEN;
QFGSIMEALYNENQ; FGSIMEALYNENQN; GSIMEALYNENQNH;
SIMEALYNENQNHS; IMEALYNENQNHSL; MEALYNENQNHSLI;
EALYNENQNHSLIR; ALYNENQNHSLIRE; LYNENQNHSLIREL;
YNENQNHSLIRELM; NENQNHSLIRELMI; ENQNHSLIRELMIS;
NQNHSLIRELMISG; QNHSLIRELMISGL; NHSLIRELMISGLG;
HSLIRELMISGLGT; SLIRELMISGLGTQ; LIRELMISGLGTQI;
IRELMISGLGTQIS; RELMISGLGTQISF; ELMISGLGTQISFE;
LMISGLGTQISFEL; MISGLGTQISFELA; ISGLGTQISFELAL;
SGLGTQISFELALE; GLGTQISFELALEE; LGTQISFELALEEI;
GTQISFELALEEIN; TQISFELALEEINK; QISFELALEEINKK;
ISFELALEEINKKI; SFELALEEINKKIE; FELALEEINKKIEI;

ELALEEINKKIEIF; LALEEINKKIEIFN; ALEEINKKIEIFNQ;
LEEINKKIEIFNQD; EEINKKIEIFNQDY; EINKKIEIFNQDYL;
INKKIEIFNQDYLN; NKKIEIFNQDYLNA; KKIEIFNQDYLNAK;
KIEIFNQDYLNAKI; IEIFNQDYLNAKIN; EIFNQDYLNAKINS;
IFNQDYLNAKINSF; FNQDYLNAKINSFD; NQDYLNAKINSFDF;
QDYLNAKINSFDFT; DYLNAKINSFDFTM; YLNAKINSFDFTMK;
LNAKINSFDFTMKL; NAKINSFDFTMKLK; AKINSFDFTMKLKE;
KINSFDFTMKLKEL; INSFDFTMKLKELK; NSFDFTMKLKELKS;
SFDFTMKLKELKSK; FDFTMKLKELKSKL; DFTMKLKELKSKLN;
FTMKLKELKSKLNQ; TMKLKELKSKLNQI; MKLKELKSKLNQIL;
KLKELKSKLNQILD; LKELKSKLNQILDK; KELKSKLNQILDKR;
ELKSKLNQILDKRK; LKSKLNQILDKRKE; KSKLNQILDKRKEW;
SKLNQILDKRKEWS; KLNQILDKRKEWSR; LNQILDKRKEWSRQ;
NQILDKRKEWSRQA; QILDKRKEWSRQAD; ILDKRKEWSRQADG;
LDKRKEWSRQADGL; DKRKEWSRQADGLI; KRKEWSRQADGLIA;
RKEWSRQADGLIAN; KEWSRQADGLIANA; EWSRQADGLIANAS;
WSRQADGLIANASS; SRQADGLIANASSN; RQADGLIANASSNS;
QADGLIANASSNSS; ADGLIANASSNSSL; DGLIANASSNSSLS;
GLIANASSNSSLSD; LIANASSNSSLSDS; IANASSNSSLSDSK;
ANASSNSSLSDSKS; NASSNSSLSDSKSL; ASSNSSLSDSKSLA;
SSNSSLSDSKSLAE; SNSSLSDSKSLAEY; NSSLSDSKSLAEYI;
SSLSDSKSLAEYIK; SLSDSKSLAEYIKK; LSDSKSLAEYIKKR;
SDSKSLAEYIKKRY; DSKSLAEYIKKRYL; SKSLAEYIKKRYLD;
KSLAEYIKKRYLDN; SLAEYIKKRYLDNM; LAEYIKKRYLDNMQ;
AEYIKKRYLDNMQN; EYIKKRYLDNMQNA; YIKKRYLDNMQNAR;
IKKRYLDNMQNARQ; KKRYLDNMQNARQS; KRYLDNMQNARQSV;
RYLDNMQNARQSVL; YLDNMQNARQSVLE; LDNMQNARQSVLEA;
DNMQNARQSVLEAY; NMQNARQSVLEAYI; MQNARQSVLEAYIS;
QNARQSVLEAYISI; NARQSVLEAYISIM;

15 mers:
MKIKPLIQLKLLGLF; KIKPLIQLKLLGLFL; IKPLIQLKLLGLFLF;
KPLIQLKLLGLFLFS; PLIQLKLLGLFLFSC; LIQLKLLGLFLFSCT;
IQLKLLGLFLFSCTI; QLKLLGLFLFSCTID; LKLLGLFLFSCTIDA;
KLLGLFLFSCTIDAN; LLGLFLFSCTIDANL; LGLFLFSCTIDANLN;
GLFLFSCTIDANLNE; LFLFSCTIDANLNED; FLFSCTIDANLNEDY;
LFSCTIDANLNEDYK; FSCTIDANLNEDYKN; SCTIDANLNEDYKNK;
CTIDANLNEDYKNKV; TIDANLNEDYKNKVK; IDANLNEDYKNKVKG;
DANLNEDYKNKVKGI; ANLNEDYKNKVKGIL; NLNEDYKNKVKGILN;
LNEDYKNKVKGILNK; NEDYKNKVKGILNKA; EDYKNKVKGILNKAA;
DYKNKVKGILNKAAD; YKNKVKGILNKAADD; KNKVKGILNKAADDQ;
NKVKGILNKAADDQE; KVKGILNKAADDQET; VKGILNKAADDQETT;
KGILNKAADDQETTS; GILNKAADDQETTSA; ILNKAADDQETTSAD;
LNKAADDQETTSADT; NKAADDQETTSADTN; KAADDQETTSADTNS;
AADDQETTSADTNSN; ADDQETTSADTNSNA; DDQETTSADTNSNAA;
DQETTSADTNSNAAK; QETTSADTNSNAAKN; ETTSADTNSNAAKNI;
TTSADTNSNAAKNIP; TSADTNSNAAKNIPI; SADTNSNAAKNIPIA;
ADTNSNAAKNIPIAD; DTNSNAAKNIPIADN; TNSNAAKNIPIADND;
NSNAAKNIPIADNDK; SNAAKNIPIADNDKV; NAAKNIPIADNDKVA;
AAKNIPIADNDKVAA; AKNIPIADNDKVAAE; KNIPIADNDKVAAEL;
NIPIADNDKVAAELK; IPIADNDKVAAELKK; PIADNDKVAAELKKQ;

IADNDKVAAELKKQS; ADNDKVAAELKKQSQ; DNDKVAAELKKQSQA;
NDKVAAELKKQSQAA; DKVAAELKKQSQAAK; KVAAELKKQSQAAKT;
VAAELKKQSQAAKTV; AAELKKQSQAAKTVA; AELKKQSQAAKTVAA;
ELKKQSQAAKTVAAA; LKKQSQAAKTVAAAP; KKQSQAAKTVAAAPN;
KQSQAAKTVAAAPNK; QSQAAKTVAAAPNKG; SQAAKTVAAAPNKGS;
QAAKTVAAAPNKGSQ; AAKTVAAAPNKGSQN; AKTVAAAPNKGSQNQ;
KTVAAAPNKGSQNQP; TVAAAPNKGSQNQPQ; VAAAPNKGSQNQPQT;
AAAPNKGSQNQPQTT; AAPNKGSQNQPQTTP; APNKGSQNQPQTTPN;
PNKGSQNQPQTTPNK; NKGSQNQPQTTPNKG; KGSQNQPQTTPNKGS;
GSQNQPQTTPNKGSQ; SQNQPQTTPNKGSQN; QNQPQTTPNKGSQNQ;
NQPQTTPNKGSQNQQ; QPQTTPNKGSQNQQA; PQTTPNKGSQNQQAA;
QTTPNKGSQNQQAAP; TTPNKGSQNQQAAPS; TPNKGSQNQQAAPSP;
PNKGSQNQQAAPSPQ; NKGSQNQQAAPSPQL; KGSQNQQAAPSPQLQ;
GSQNQQAAPSPQLQS; SQNQQAAPSPQLQSL; QNQQAAPSPQLQSLS;
NQQAAPSPQLQSLSF; QQAAPSPQLQSLSFS; QAAPSPQLQSLSFSA;
AAPSPQLQSLSFSAD; APSPQLQSLSFSADL; PSPQLQSLSFSADLS;
SPQLQSLSFSADLSN; PQLQSLSFSADLSNL; QLQSLSFSADLSNLP;
LQSLSFSADLSNLPK; QSLSFSADLSNLPKT; SLSFSADLSNLPKTT;
LSFSADLSNLPKTTA; SFSADLSNLPKTTAA; FSADLSNLPKTTAAR;
SADLSNLPKTTAARA; ADLSNLPKTTAARAA; DLSNLPKTTAARAAS;
LSNLPKTTAARAASL; SNLPKTTAARAASLT; NLPKTTAARAASLTK;
LPKTTAARAASLTKQ; PKTTAARAASLTKQR; KTTAARAASLTKQRI;
TTAARAASLTKQRIP; TAARAASLTKQRIPI; AARAASLTKQRIPIQ;
ARAASLTKQRIPIQA; RAASLTKQRIPIQAV; AASLTKQRIPIQAVT;
ASLTKQRIPIQAVTT; SLTKQRIPIQAVTTV; LTKQRIPIQAVTTVP;
TKQRIPIQAVTTVPG; KQRIPIQAVTTVPGN; QRIPIQAVTTVPGNT;
RIPIQAVTTVPGNTR; IPIQAVTTVPGNTRT; PIQAVTTVPGNTRTF;
IQAVTTVPGNTRTFN; QAVTTVPGNTRTFNS; AVTTVPGNTRTFNSR;
VTTVPGNTRTFNSRN; TTVPGNTRTFNSRNS; TVPGNTRTFNSRNSG;
VPGNTRTFNSRNSGL; PGNTRTFNSRNSGLP; GNTRTFNSRNSGLPT;
NTRTFNSRNSGLPTF; TRTFNSRNSGLPTFA; RTFNSRNSGLPTFAL;
TFNSRNSGLPTFALN; FNSRNSGLPTFALNY; NSRNSGLPTFALNYS;
SRNSGLPTFALNYSF; RNSGLPTFALNYSFS; NSGLPTFALNYSFSQ;
SGLPTFALNYSFSQP; GLPTFALNYSFSQPT; LPTFALNYSFSQPTR;
PTFALNYSFSQPTRQ; TFALNYSFSQPTRQQ; FALNYSFSQPTRQQT;
ALNYSFSQPTRQQTN; LNYSFSQPTRQQTNS; NYSFSQPTRQQTNSS;
YSFSQPTRQQTNSSS; SFSQPTRQQTNSSSA; FSQPTRQQTNSSSAV;
SQPTRQQTNSSSAVQ; QPTRQQTNSSSAVQT; PTRQQTNSSSAVQTT;
TRQQTNSSSAVQTTT; RQQTNSSSAVQTTTS; QQTNSSSAVQTTTSS;
QTNSSSAVQTTTSSG; TNSSSAVQTTTSSGS; NSSSAVQTTTSSGSK;
SSSAVQTTTSSGSKL; SSAVQTTTSSGSKLQ; SAVQTTTSSGSKLQT;
AVQTTTSSGSKLQTL; VQTTTSSGSKLQTLK; QTTTSSGSKLQTLKN;
TTTSSGSKLQTLKNE; TTSSGSKLQTLKNEL; TSSGSKLQTLKNELI;
SSGSKLQTLKNELIR; SGSKLQTLKNELIRA; GSKLQTLKNELIRAI;
SKLQTLKNELIRAIS; KLQTLKNELIRAISE; LQTLKNELIRAISEE;
QTLKNELIRAISEEK; TLKNELIRAISEEKN; LKNELIRAISEEKNK;
KNELIRAISEEKNKT; NELIRAISEEKNKTQ; ELIRAISEEKNKTQN;
LIRAISEEKNKTQNN; IRAISEEKNKTQNNF; RAISEEKNKTQNNFG;
AISEEKNKTQNNFGF; ISEEKNKTQNNFGFR; SEEKNKTQNNFGFRE;
EEKNKTQNNFGFRET; EKNKTQNNFGFRETY; KNKTQNNFGFRETYD;
NKTQNNFGFRETYDQ; KTQNNFGFRETYDQF; TQNNFGFRETYDQFK;

QNNFGFRETYDQFKM; NNFGFRETYDQFKMK; NFGFRETYDQFKMKD;
FGFRETYDQFKMKDS; GFRETYDQFKMKDSA; FRETYDQFKMKDSAF;
RETYDQFKMKDSAFE; ETYDQFKMKDSAFEL; TYDQFKMKDSAFELL;
YDQFKMKDSAFELLD; DQFKMKDSAFELLDV; QFKMKDSAFELLDVI;
FKMKDSAFELLDVIS; KMKDSAFELLDVISS; MKDSAFELLDVISSA;
KDSAFELLDVISSAK; DSAFELLDVISSAKV; SAFELLDVISSAKVY;
AFELLDVISSAKVYD; FELLDVISSAKVYDR; ELLDVISSAKVYDRS;
LLDVISSAKVYDRSY; LDVISSAKVYDRSYA; DVISSAKVYDRSYAP;
VISSAKVYDRSYAPQ; ISSAKVYDRSYAPQL; SSAKVYDRSYAPQLN;
SAKVYDRSYAPQLNS; AKVYDRSYAPQLNSN; KVYDRSYAPQLNSNT;
VYDRSYAPQLNSNTP; YDRSYAPQLNSNTPE; DRSYAPQLNSNTPEA;
RSYAPQLNSNTPEAE; SYAPQLNSNTPEAEN; YAPQLNSNTPEAENE;
APQLNSNTPEAENER; PQLNSNTPEAENERN; QLNSNTPEAENERNK;
LNSNTPEAENERNKF; NSNTPEAENERNKFY; SNTPEAENERNKFYA;
NTPEAENERNKFYAL; TPEAENERNKFYALM; PEAENERNKFYALMD;
EAENERNKFYALMDF; AENERNKFYALMDFD; ENERNKFYALMDFDQ;
NERNKFYALMDFDQY; ERNKFYALMDFDQYK; RNKFYALMDFDQYKI;
NKFYALMDFDQYKIE; KFYALMDFDQYKIEQ; FYALMDFDQYKIEQF;
YALMDFDQYKIEQFG; ALMDFDQYKIEQFGS; LMDFDQYKIEQFGSI;
MDFDQYKIEQFGSIM; DFDQYKIEQFGSIME; FDQYKIEQFGSIMEA;
DQYKIEQFGSIMEAL; QYKIEQFGSIMEALY; YKIEQFGSIMEALYN;
KIEQFGSIMEALYNE; IEQFGSIMEALYNEN; EQFGSIMEALYNENQ;
QFGSIMEALYNENQN; FGSIMEALYNENQNH; GSIMEALYNENQNHS;
SIMEALYNENQNHSL; IMEALYNENQNHSLI; MEALYNENQNHSLIR;
EALYNENQNHSLIRE; ALYNENQNHSLIREL; LYNENQNHSLIRELM;
YNENQNHSLIRELMI; NENQNHSLIRELMIS; ENQNHSLIRELMISG;
NQNHSLIRELMISGL; QNHSLIRELMISGLG; NHSLIRELMISGLGT;
HSLIRELMISGLGTQ; SLIRELMISGLGTQI; LIRELMISGLGTQIS;
IRELMISGLGTQISF; RELMISGLGTQISFE; ELMISGLGTQISFEL;
LMISGLGTQISFELA; MISGLGTQISFELAL; ISGLGTQISFELALE;
SGLGTQISFELALEE; GLGTQISFELALEEI; LGTQISFELALEEIN;
GTQISFELALEEINK; TQISFELALEEINKK; QISFELALEEINKKI;
ISFELALEEINKKIE; SFELALEEINKKIEI; FELALEEINKKIEIF;
ELALEEINKKIEIFN; LALEEINKKIEIFNQ; ALEEINKKIEIFNQD;
LEEINKKIEIFNQDY; EEINKKIEIFNQDYL; EINKKIEIFNQDYLN;
INKKIEIFNQDYLNA; NKKIEIFNQDYLNAK; KKIEIFNQDYLNAKI;
KIEIFNQDYLNAKIN; IEIFNQDYLNAKINS; EIFNQDYLNAKINSF;
IFNQDYLNAKINSFD; FNQDYLNAKINSFDF; NQDYLNAKINSFDFT;
QDYLNAKINSFDFTM; DYLNAKINSFDFTMK; YLNAKINSFDFTMKL;
LNAKINSFDFTMKLK; NAKINSFDFTMKLKE; AKINSFDFTMKLKEL;
KINSFDFTMKLKELK; INSFDFTMKLKELKS; NSFDFTMKLKELKSK;
SFDFTMKLKELKSKL; FDFTMKLKELKSKLN; DFTMKLKELKSKLNQ;
FTMKLKELKSKLNQI; TMKLKELKSKLNQIL; MKLKELKSKLNQILD;
KLKELKSKLNQILDK; LKELKSKLNQILDKR; KELKSKLNQILDKRK;
ELKSKLNQILDKRKE; LKSKLNQILDKRKEW; KSKLNQILDKRKEWS;
SKLNQILDKRKEWSR; KLNQILDKRKEWSRQ; LNQILDKRKEWSRQA;
NQILDKRKEWSRQAD; QILDKRKEWSRQADG; ILDKRKEWSRQADGL;
LDKRKEWSRQADGLI; DKRKEWSRQADGLIA; KRKEWSRQADGLIAN;
RKEWSRQADGLIANA; KEWSRQADGLIANAS; EWSRQADGLIANASS;
WSRQADGLIANASSN; SRQADGLIANASSNS; RQADGLIANASSNSS;
QADGLIANASSNSSL; ADGLIANASSNSSLS; DGLIANASSNSSLSD;

GLIANASSNSSLSDS; LIANASSNSSLSDSK; IANASSNSSLSDSKS;
ANASSNSSLSDSKSL; NASSNSSLSDSKSLA; ASSNSSLSDSKSLAE;
SSNSSLSDSKSLAEY; SNSSLSDSKSLAEYI; NSSLSDSKSLAEYIK;
SSLSDSKSLAEYIKK; SLSDSKSLAEYIKKR; LSDSKSLAEYIKKRY;
SDSKSLAEYIKKRYL; DSKSLAEYIKKRYLD; SKSLAEYIKKRYLDN;
KSLAEYIKKRYLDNM; SLAEYIKKRYLDNMQ; LAEYIKKRYLDNMQN;
AEYIKKRYLDNMQNA; EYIKKRYLDNMQNAR; YIKKRYLDNMQNARQ;
IKKRYLDNMQNARQS; KKRYLDNMQNARQSV; KRYLDNMQNARQSVL;
RYLDNMQNARQSVLE; YLDNMQNARQSVLEA; LDNMQNARQSVLEAY;
DNMQNARQSVLEAYI; NMQNARQSVLEAYIS; MQNARQSVLEAYISI;
QNARQSVLEAYISIM;

16 mers:
MKIKPLIQLKLLGLFL; KIKPLIQLKLLGLFLF; IKPLIQLKLLGLFLFS;
KPLIQLKLLGLFLFSC; PLIQLKLLGLFLFSCT; LIQLKLLGLFLFSCTI;
IQLKLLGLFLFSCTID; QLKLLGLFLFSCTIDA; LKLLGLFLFSCTIDAN;
KLLGLFLFSCTIDANL; LLGLFLFSCTIDANLN; LGLFLFSCTIDANLNE;
GLFLFSCTIDANLNED; LFLFSCTIDANLNEDY; FLFSCTIDANLNEDYK;
LFSCTIDANLNEDYKN; FSCTIDANLNEDYKNK; SCTIDANLNEDYKNKV;
CTIDANLNEDYKNKVK; TIDANLNEDYKNKVKG; IDANLNEDYKNKVKGI;
DANLNEDYKNKVKGIL; ANLNEDYKNKVKGILN; NLNEDYKNKVKGILNK;
LNEDYKNKVKGILNKA; NEDYKNKVKGILNKAA; EDYKNKVKGILNKAAD;
DYKNKVKGILNKAADD; YKNKVKGILNKAADDQ; KNKVKGILNKAADDQE;
NKVKGILNKAADDQET; KVKGILNKAADDQETT; VKGILNKAADDQETTS;
KGILNKAADDQETTSA; GILNKAADDQETTSAD; ILNKAADDQETTSADT;
LNKAADDQETTSADTN; NKAADDQETTSADTNS; KAADDQETTSADTNSN;
AADDQETTSADTNSNA; ADDQETTSADTNSNAA; DDQETTSADTNSNAAK;
DQETTSADTNSNAAKN; QETTSADTNSNAAKNI; ETTSADTNSNAAKNIP;
TTSADTNSNAAKNIPI; TSADTNSNAAKNIPIA; SADTNSNAAKNIPIAD;
ADTNSNAAKNIPIADN; DTNSNAAKNIPIADND; TNSNAAKNIPIADNDK;
NSNAAKNIPIADNDKV; SNAAKNIPIADNDKVA; NAAKNIPIADNDKVAA;
AAKNIPIADNDKVAAE; AKNIPIADNDKVAAEL; KNIPIADNDKVAAELK;
NIPIADNDKVAAELKK; IPIADNDKVAAELKKQ; PIADNDKVAAELKKQS;
IADNDKVAAELKKQSQ; ADNDKVAAELKKQSQA; DNDKVAAELKKQSQAA;
NDKVAAELKKQSQAAK; DKVAAELKKQSQAAKT; KVAAELKKQSQAAKTV;
VAAELKKQSQAAKTVA; AAELKKQSQAAKTVAA; AELKKQSQAAKTVAAA;
ELKKQSQAAKTVAAAP; LKKQSQAAKTVAAAPN; KKQSQAAKTVAAAPNK;
KQSQAAKTVAAAPNKG; QSQAAKTVAAAPNKGS; SQAAKTVAAAPNKGSQ;
QAAKTVAAAPNKGSQN; AAKTVAAAPNKGSQNQ; AKTVAAAPNKGSQNQP;
KTVAAAPNKGSQNQPQ; TVAAAPNKGSQNQPQT; VAAAPNKGSQNQPQTT;
AAAPNKGSQNQPQTTP; AAPNKGSQNQPQTTPN; APNKGSQNQPQTTPNK;
PNKGSQNQPQTTPNKG; NKGSQNQPQTTPNKGS; KGSQNQPQTTPNKGSQ;
GSQNQPQTTPNKGSQN; SQNQPQTTPNKGSQNQ; QNQPQTTPNKGSQNQQ;
NQPQTTPNKGSQNQQA; QPQTTPNKGSQNQQAA; PQTTPNKGSQNQQAAP;
QTTPNKGSQNQQAAPS; TTPNKGSQNQQAAPSP; TPNKGSQNQQAAPSPQ;
PNKGSQNQQAAPSPQL; NKGSQNQQAAPSPQLQ; KGSQNQQAAPSPQLQS;
GSQNQQAAPSPQLQSL; SQNQQAAPSPQLQSLS; QNQQAAPSPQLQSLSF;
NQQAAPSPQLQSLSFS; QQAAPSPQLQSLSFSA; QAAPSPQLQSLSFSAD;
AAPSPQLQSLSFSADL; APSPQLQSLSFSADLS; PSPQLQSLSFSADLSN;
SPQLQSLSFSADLSNL; PQLQSLSFSADLSNLP; QLQSLSFSADLSNLPK;
LQSLSFSADLSNLPKT; QSLSFSADLSNLPKTT; SLSFSADLSNLPKTTA;

| | |
|---|---|
| LSFSADLSNLPKTTAA; SFSADLSNLPKTTAAR; FSADLSNLPKTTAARA; SADLSNLPKTTAARAA; ADLSNLPKTTAARAAS; DLSNLPKTTAARAASL; LSNLPKTTAARAASLT; SNLPKTTAARAASLTK; NLPKTTAARAASLTKQ; LPKTTAARAASLTKQR; PKTTAARAASLTKQRI; KTTAARAASLTKQRIP; TTAARAASLTKQRIPI; TAARAASLTKQRIPIQ; AARAASLTKQRIPIQA; ARAASLTKQRIPIQAV; RAASLTKQRIPIQAVT; AASLTKQRIPIQAVTT; ASLTKQRIPIQAVTTV; SLTKQRIPIQAVTTVP; LTKQRIPIQAVTTVPG; TKQRIPIQAVTTVPGN; KQRIPIQAVTTVPGNT; QRIPIQAVTTVPGNTR; RIPIQAVTTVPGNTRT; IPIQAVTTVPGNTRTF; PIQAVTTVPGNTRTFN; IQAVTTVPGNTRTFNS; QAVTTVPGNTRTFNSR; AVTTVPGNTRTFNSRN; VTTVPGNTRTFNSRNS; TTVPGNTRTFNSRNSG; TVPGNTRTFNSRNSGL; VPGNTRTFNSRNSGLP; PGNTRTFNSRNSGLPT; GNTRTFNSRNSGLPTF; NTRTFNSRNSGLPTFA; TRTFNSRNSGLPTFAL; RTFNSRNSGLPTFALN; TFNSRNSGLPTFALNY; FNSRNSGLPTFALNYS; NSRNSGLPTFALNYSF; SRNSGLPTFALNYSFS; RNSGLPTFALNYSFSQ; NSGLPTFALNYSFSQP; SGLPTFALNYSFSQPT; GLPTFALNYSFSQPTR; LPTFALNYSFSQPTRQ; PTFALNYSFSQPTRQQ; TFALNYSFSQPTRQQT; FALNYSFSQPTRQQTN; ALNYSFSQPTRQQTNS; LNYSFSQPTRQQTNSS; NYSFSQPTRQQTNSSS; YSFSQPTRQQTNSSSA; SFSQPTRQQTNSSSAV; FSQPTRQQTNSSSAVQ; SQPTRQQTNSSSAVQT; QPTRQQTNSSSAVQTT; PTRQQTNSSSAVQTTT; TRQQTNSSSAVQTTTS; RQQTNSSSAVQTTTSS; QQTNSSSAVQTTTSSG; QTNSSSAVQTTTSSGS; TNSSSAVQTTTSSGSK; NSSSAVQTTTSSGSKL; SSSAVQTTTSSGSKLQ; SSAVQTTTSSGSKLQT; SAVQTTTSSGSKLQTL; AVQTTTSSGSKLQTLK; VQTTTSSGSKLQTLKN; QTTTSSGSKLQTLKNE; TTTSSGSKLQTLKNEL; TTSSGSKLQTLKNELI; TSSGSKLQTLKNELIR; SSGSKLQTLKNELIRA; SGSKLQTLKNELIRAI; GSKLQTLKNELIRAIS; SKLQTLKNELIRAISE; KLQTLKNELIRAISEE; LQTLKNELIRAISEEK; QTLKNELIRAISEEKN; TLKNELIRAISEEKNK; LKNELIRAISEEKNKT; KNELIRAISEEKNKTQ; NELIRAISEEKNKTQN; ELIRAISEEKNKTQNN; LIRAISEEKNKTQNNF; IRAISEEKNKTQNNFG; RAISEEKNKTQNNFGF; AISEEKNKTQNNFGFR; ISEEKNKTQNNFGFRE; SEEKNKTQNNFGFRET; EEKNKTQNNFGFRETY; EKNKTQNNFGFRETYD; KNKTQNNFGFRETYDQ; NKTQNNFGFRETYDQF; KTQNNFGFRETYDQFK; TQNNFGFRETYDQFKM; QNNFGFRETYDQFKMK; NNFGFRETYDQFKMKD; NFGFRETYDQFKMKDS; FGFRETYDQFKMKDSA; GFRETYDQFKMKDSAF; FRETYDQFKMKDSAFE; RETYDQFKMKDSAFEL; ETYDQFKMKDSAFELL; TYDQFKMKDSAFELLD; YDQFKMKDSAFELLDV; DQFKMKDSAFELLDVI; QFKMKDSAFELLDVIS; FKMKDSAFELLDVISS; KMKDSAFELLDVISSA; MKDSAFELLDVISSAK; KDSAFELLDVISSAKV; DSAFELLDVISSAKVY; SAFELLDVISSAKVYD; AFELLDVISSAKVYDR; FELLDVISSAKVYDRS; ELLDVISSAKVYDRSY; LLDVISSAKVYDRSYA; LDVISSAKVYDRSYAP; DVISSAKVYDRSYAPQ; VISSAKVYDRSYAPQL; ISSAKVYDRSYAPQLN; SSAKVYDRSYAPQLNS; SAKVYDRSYAPQLNSN; AKVYDRSYAPQLNSNT; KVYDRSYAPQLNSNTP; VYDRSYAPQLNSNTPE; YDRSYAPQLNSNTPEA; DRSYAPQLNSNTPEAE; RSYAPQLNSNTPEAEN; SYAPQLNSNTPEAENE; YAPQLNSNTPEAENER; APQLNSNTPEAENERN; PQLNSNTPEAENERNK; QLNSNTPEAENERNKF; LNSNTPEAENERNKFY; NSNTPEAENERNKFYA; SNTPEAENERNKFYAL; NTPEAENERNKFYALM; TPEAENERNKFYALMD; PEAENERNKFYALMDF; EAENERNKFYALMDFD; AENERNKFYALMDFDQ; ENERNKFYALMDFDQY; NERNKFYALMDFDQYK; ERNKFYALMDFDQYKI; RNKFYALMDFDQYKIE; NKFYALMDFDQYKIEQ; KFYALMDFDQYKIEQF; FYALMDFDQYKIEQFG; | |

| | |
|---|---|
| | YALMDFDQYKIEQFGS; ALMDFDQYKIEQFGSI; LMDFDQYKIEQFGSIM; MDFDQYKIEQFGSIME; DFDQYKIEQFGSIMEA; FDQYKIEQFGSIMEAL; DQYKIEQFGSIMEALY; QYKIEQFGSIMEALYN; YKIEQFGSIMEALYNE; KIEQFGSIMEALYNEN; IEQFGSIMEALYNENQ; EQFGSIMEALYNENQN; QFGSIMEALYNENQNH; FGSIMEALYNENQNHS; GSIMEALYNENQNHSL; SIMEALYNENQNHSLI; IMEALYNENQNHSLIR; MEALYNENQNHSLIRE; EALYNENQNHSLIREL; ALYNENQNHSLIRELM; LYNENQNHSLIRELMI; YNENQNHSLIRELMIS; NENQNHSLIRELMISG; ENQNHSLIRELMISGL; NQNHSLIRELMISGLG; QNHSLIRELMISGLGT; NHSLIRELMISGLGTQ; HSLIRELMISGLGTQI; SLIRELMISGLGTQIS; LIRELMISGLGTQISF; IRELMISGLGTQISFE; RELMISGLGTQISFEL; ELMISGLGTQISFELA; LMISGLGTQISFELAL; MISGLGTQISFELALE; ISGLGTQISFELALEE; SGLGTQISFELALEEI; GLGTQISFELALEEIN; LGTQISFELALEEINK; GTQISFELALEEINKK; TQISFELALEEINKKI; QISFELALEEINKKIE; ISFELALEEINKKIEI; SFELALEEINKKIEIF; FELALEEINKKIEIFN; ELALEEINKKIEIFNQ; LALEEINKKIEIFNQD; ALEEINKKIEIFNQDY; LEEINKKIEIFNQDYL; EEINKKIEIFNQDYLN; EINKKIEIFNQDYLNA; INKKIEIFNQDYLNAK; NKKIEIFNQDYLNAKI; KKIEIFNQDYLNAKIN; KIEIFNQDYLNAKINS; IEIFNQDYLNAKINSF; EIFNQDYLNAKINSFD; IFNQDYLNAKINSFDF; FNQDYLNAKINSFDFT; NQDYLNAKINSFDFTM; QDYLNAKINSFDFTMK; DYLNAKINSFDFTMKL; YLNAKINSFDFTMKLK; LNAKINSFDFTMKLKE; NAKINSFDFTMKLKEL; AKINSFDFTMKLKELK; KINSFDFTMKLKELKS; INSFDFTMKLKELKSK; NSFDFTMKLKELKSKL; SFDFTMKLKELKSKLN; FDFTMKLKELKSKLNQ; DFTMKLKELKSKLNQI; FTMKLKELKSKLNQIL; TMKLKELKSKLNQILD; MKLKELKSKLNQILDK; KLKELKSKLNQILDKR; LKELKSKLNQILDKRK; KELKSKLNQILDKRKE; ELKSKLNQILDKRKEW; LKSKLNQILDKRKEWS; KSKLNQILDKRKEWSR; SKLNQILDKRKEWSRQ; KLNQILDKRKEWSRQA; LNQILDKRKEWSRQAD; NQILDKRKEWSRQADG; QILDKRKEWSRQADGL; ILDKRKEWSRQADGLI; LDKRKEWSRQADGLIA; DKRKEWSRQADGLIAN; KRKEWSRQADGLIANA; RKEWSRQADGLIANAS; KEWSRQADGLIANASS; EWSRQADGLIANASSN; WSRQADGLIANASSNS; SRQADGLIANASSNSS; RQADGLIANASSNSSL; QADGLIANASSNSSLS; ADGLIANASSNSSLSD; DGLIANASSNSSLSDS; GLIANASSNSSLSDSK; LIANASSNSSLSDSKS; IANASSNSSLSDSKSL; ANASSNSSLSDSKSLA; NASSNSSLSDSKSLAE; ASSNSSLSDSKSLAEY; SSNSSLSDSKSLAEYI; SNSSLSDSKSLAEYIK; NSSLSDSKSLAEYIKK; SSLSDSKSLAEYIKKR; SLSDSKSLAEYIKKRY; LSDSKSLAEYIKKRYL; SDSKSLAEYIKKRYLD; DSKSLAEYIKKRYLDN; SKSLAEYIKKRYLDNM; KSLAEYIKKRYLDNMQ; SLAEYIKKRYLDNMQN; LAEYIKKRYLDNMQNA; AEYIKKRYLDNMQNAR; EYIKKRYLDNMQNARQ; YIKKRYLDNMQNARQS; IKKRYLDNMQNARQSV; KKRYLDNMQNARQSVL; KRYLDNMQNARQSVLE; RYLDNMQNARQSVLEA; YLDNMQNARQSVLEAY; LDNMQNARQSVLEAYI; DNMQNARQSVLEAYIS; NMQNARQSVLEAYISI; MQNARQSVLEAYISIM; |
| Seq 35 | 13 mers:<br>MSSCTIDANLNKD; SSCTIDANLNKDY; SCTIDANLNKDYK; CTIDANLNKDYKN; TIDANLNKDYKNK; IDANLNKDYKNKV; DANLNKDYKNKVE; ANLNKDYKNKVEE; NLNKDYKNKVEEL; LNKDYKNKVEELL; NKDYKNKVEELLN; KDYKNKVEELLNS; DYKNKVEELLNSS; YKNKVEELLNSST; KNKVEELLNSSTD; |

NKVEELLNSSTDD; KVEELLNSSTDDQ; VEELLNSSTDDQA;
EELLNSSTDDQAK; ELLNSSTDDQAKI; LLNSSTDDQAKIS;
LNSSTDDQAKISI; NSSTDDQAKISIN; SSTDDQAKISINT;
STDDQAKISINTG; TDDQAKISINTGS; DDQAKISINTGSN;
DQAKISINTGSNA; QAKISINTGSNAT; AKISINTGSNATK;
KISINTGSNATKN; ISINTGSNATKNK; SINTGSNATKNKT;
INTGSNATKNKTN; NTGSNATKNKTNI; TGSNATKNKTNIK;
GSNATKNKTNIKV; SNATKNKTNIKVA; NATKNKTNIKVAG;
ATKNKTNIKVAGL; TKNKTNIKVAGLQ; KNKTNIKVAGLQK;
NKTNIKVAGLQKN; KTNIKVAGLQKNT; TNIKVAGLQKNTQ;
NIKVAGLQKNTQS; IKVAGLQKNTQSK; KVAGLQKNTQSKK;
VAGLQKNTQSKKN; AGLQKNTQSKKNN; GLQKNTQSKKNNN;
LQKNTQSKKNNNL; QKNTQSKKNNNLQ; KNTQSKKNNNLQG;
NTQSKKNNNLQGL; TQSKKNNNLQGLN; QSKKNNNLQGLNP;
SKKNNNLQGLNPA; KKNNNLQGLNPAN; KNNNLQGLNPANQ;
NNNLQGLNPANQV; NNLQGLNPANQVN; NLQGLNPANQVNP;
LQGLNPANQVNPG; QGLNPANQVNPGN; GLNPANQVNPGNP;
LNPANQVNPGNPM; NPANQVNPGNPMQ; PANQVNPGNPMQI;
ANQVNPGNPMQIA; NQVNPGNPMQIAN; QVNPGNPMQIANQ;
VNPGNPMQIANQA; NPGNPMQIANQAN; PGNPMQIANQANQ;
GNPMQIANQANQA; NPMQIANQANQAN; PMQIANQANQANQ;
MQIANQANQANQA; QIANQANQANQAN; IANQANQANQANQ;
ANQANQANQANQA; NQANQANQANQAN; QANQANQANQANQ;
ANQANQANQANQA; NQANQANQANQAN; QANQANQANQANQ;
ANQANQANQANQA; NQANQANQANQAS; QANQANQANQASQ;
ANQANQANQASQA; NQANQANQASQAS; QANQANQASQASQ;
ANQANQASQASQA; NQANQASQASQAS; QANQASQASQASQ;
ANQASQASQASQV; NQASQASQASQVA; QASQASQASQVAS;
ASQASQASQVASS; SQASQASQVASSA; QASQASQVASSAS;
ASQASQVASSASQ; SQASQVASSASQA; QASQVASSASQAS;
ASQVASSASQASP; SQVASSASQASPV; QVASSASQASPVA;
VASSASQASPVAS; ASSASQASPVASP; SSASQASPVASPA;
SASQASPVASPAT; ASQASPVASPATN; SQASPVASPATNV;
QASPVASPATNVQ; ASPVASPATNVQA; SPVASPATNVQAT;
PVASPATNVQATP; VASPATNVQATPP; ASPATNVQATPPK;
SPATNVQATPPKQ; PATNVQATPPKQI; ATNVQATPPKQIA;
TNVQATPPKQIAS; NVQATPPKQIASA; VQATPPKQIASAQ;
QATPPKQIASAQA; ATPPKQIASAQAI; TPPKQIASAQAIQ;
PPKQIASAQAIQT; PKQIASAQAIQTV; KQIASAQAIQTVP;
QIASAQAIQTVPN; IASAQAIQTVPNN; ASAQAIQTVPNNT;
SAQAIQTVPNNTS; AQAIQTVPNNTST; QAIQTVPNNTSTP;
AIQTVPNNTSTPN; IQTVPNNTSTPNQ; QTVPNNTSTPNQS;
TVPNNTSTPNQSI; VPNNTSTPNQSII; PNNTSTPNQSIIK;
NNTSTPNQSIIKP; NTSTPNQSIIKPQ; TSTPNQSIIKPQQ;
STPNQSIIKPQQY; TPNQSIIKPQQYT; PNQSIIKPQQYTF;
NQSIIKPQQYTFS; QSIIKPQQYTFSS; SIIKPQQYTFSSS;
IIKPQQYTFSSSF; IKPQQYTFSSSFS; KPQQYTFSSSFSQ;
PQQYTFSSSFSQP; QQYTFSSSFSQPT; QYTFSSSFSQPTS;
YTFSSSFSQPTSQ; TFSSSFSQPTSQT; FSSSFSQPTSQTN;
SSSFSQPTSQTNF; SSFSQPTSQTNFN; SFSQPTSQTNFNN;
FSQPTSQTNFNNS; SQPTSQTNFNNSQ; QPTSQTNFNNSQS;

PTSQTNFNNSQSN; TSQTNFNNSQSNN; SQTNFNNSQSNNV;
QTNFNNSQSNNVL; TNFNNSQSNNVLT; NFNNSQSNNVLTN;
FNNSQSNNVLTNY; NNSQSNNVLTNYR; NSQSNNVLTNYRH;
SQSNNVLTNYRHQ; QSNNVLTNYRHQT; SNNVLTNYRHQTQ;
NNVLTNYRHQTQP; NVLTNYRHQTQPS; VLTNYRHQTQPSF;
LTNYRHQTQPSFV; TNYRHQTQPSFVV; NYRHQTQPSFVVP;
YRHQTQPSFVVPV; RHQTQPSFVVPVY; HQTQPSFVVPVYS;
QTQPSFVVPVYSG; TQPSFVVPVYSGN; QPSFVVPVYSGNS;
PSFVVPVYSGNSP; SFVVPVYSGNSPL; FVVPVYSGNSPLQ;
VVPVYSGNSPLQK; VPVYSGNSPLQKL; PVYSGNSPLQKLK;
VYSGNSPLQKLKN; YSGNSPLQKLKNN; SGNSPLQKLKNNL;
GNSPLQKLKNNLL; NSPLQKLKNNLLR; SPLQKLKNNLLRR;
PLQKLKNNLLRRI; LQKLKNNLLRRIA; QKLKNNLLRRIAE;
KLKNNLLRRIAEE; LKNNLLRRIAEEK; KNNLLRRIAEEKN;
NNLLRRIAEEKNK; NLLRRIAEEKNKT; LLRRIAEEKNKTH;
LRRIAEEKNKTHN; RRIAEEKNKTHNH; RIAEEKNKTHNHG;
IAEEKNKTHNHGF; AEEKNKTHNHGFR; EEKNKTHNHGFRE;
EKNKTHNHGFRET; KNKTHNHGFRETY; NKTHNHGFRETYD;
KTHNHGFRETYDQ; THNHGFRETYDQF; HNHGFRETYDQFK;
NHGFRETYDQFKM; HGFRETYDQFKMK; GFRETYDQFKMKD;
FRETYDQFKMKDS; RETYDQFKMKDSA; ETYDQFKMKDSAF;
TYDQFKMKDSAFT; YDQFKMKDSAFTL; DQFKMKDSAFTLL;
QFKMKDSAFTLLD; FKMKDSAFTLLDV; KMKDSAFTLLDVI;
MKDSAFTLLDVIS; KDSAFTLLDVISN; DSAFTLLDVISNI;
SAFTLLDVISNIS; AFTLLDVISNISV; FTLLDVISNISVF;
TLLDVISNISVFD; LLDVISNISVFDR; LDVISNISVFDRG;
DVISNISVFDRGS; VISNISVFDRGSA; ISNISVFDRGSAP;
SNISVFDRGSAPQ; NISVFDRGSAPQL; ISVFDRGSAPQLS;
SVFDRGSAPQLSS; VFDRGSAPQLSSN; FDRGSAPQLSSNT;
DRGSAPQLSSNTP; RGSAPQLSSNTPE; GSAPQLSSNTPEA;
SAPQLSSNTPEAE; APQLSSNTPEAES; PQLSSNTPEAESE;
QLSSNTPEAESER; LSSNTPEAESERN; SSNTPEAESERNR;
SNTPEAESERNRL; NTPEAESERNRLY; TPEAESERNRLYA;
PEAESERNRLYAM; EAESERNRLYAMM; AESERNRLYAMMD;
ESERNRLYAMMDF; SERNRLYAMMDFD; ERNRLYAMMDFDQ;
RNRLYAMMDFDQA; NRLYAMMDFDQAK; RLYAMMDFDQAKT;
LYAMMDFDQAKTT; YAMMDFDQAKTTE; AMMDFDQAKTTEF;
MMDFDQAKTTEFG; MDFDQAKTTEFGS; DFDQAKTTEFGSI;
FDQAKTTEFGSIM; DQAKTTEFGSIMN; QAKTTEFGSIMNI;
AKTTEFGSIMNIL; KTTEFGSIMNILY; TTEFGSIMNILYQ;
TEFGSIMNILYQE; EFGSIMNILYQEN; FGSIMNILYQENQ;
GSIMNILYQENQN; SIMNILYQENQNH; IMNILYQENQNHS;
MNILYQENQNHSL; NILYQENQNHSLI; ILYQENQNHSLIR;
LYQENQNHSLIRS; YQENQNHSLIRSL; QENQNHSLIRSLI;
ENQNHSLIRSLII; NQNHSLIRSLIIS; QNHSLIRSLIISG;
NHSLIRSLIISGL; HSLIRSLIISGLG; SLIRSLIISGLGI;
LIRSLIISGLGIQ; IRSLIISGLGIQI; RSLIISGLGIQIS;
SLIISGLGIQISL; LIISGLGIQISLE; IISGLGIQISLES;
ISGLGIQISLEST; SGLGIQISLESTL; GLGIQISLESTLE;
LGIQISLESTLEE; GIQISLESTLEEI; IQISLESTLEEIE;
QISLESTLEEIEK; ISLESTLEEIEKK; SLESTLEEIEKKI;

LESTLEEIEKKIE; ESTLEEIEKKIES; STLEEIEKKIESF;
TLEEIEKKIESFN; LEEIEKKIESFNT; EEIEKKIESFNTQ;
EIEKKIESFNTQY; IEKKIESFNTQYL; EKKIESFNTQYLN;
KKIESFNTQYLNT; KIESFNTQYLNTI; IESFNTQYLNTII;
ESFNTQYLNTIIN; SFNTQYLNTIINS; FNTQYLNTIINSY;
NTQYLNTIINSYT; TQYLNTIINSYTF; QYLNTIINSYTFK;
YLNTIINSYTFKD; LNTIINSYTFKDK; NTIINSYTFKDKL;
TIINSYTFKDLK; IINSYTFKDLKE; INSYTFKDKLKEL;
NSYTFKDKLKELE; SYTFKDKLKELES; YTFKDKLKELESK;
TFKDKLKELESKL; FKDKLKELESKLN; KDKLKELESKLNS;
DKLKELESKLNSI; KLKELESKLNSIL; LKELESKLNSILA;
KELESKLNSILAE; ELESKLNSILAEK; LESKLNSILAEKK;
ESKLNSILAEKKE; SKLNSILAEKKEW; KLNSILAEKKEWL;
LNSILAEKKEWLN; NSILAEKKEWLNY; SILAEKKEWLNYA;
ILAEKKEWLNYAD; LAEKKEWLNYADA; AEKKEWLNYADAI;
EKKEWLNYADAII; KKEWLNYADAIIT; KEWLNYADAIITN;
EWLNYADAIITNT; WLNYADAIITNTS; LNYADAIITNTSS;
NYADAIITNTSSN; YADAIITNTSSNS; ADAIITNTSSNSK;
DAIITNTSSNSKR; AIITNTSSNSKRN; IITNTSSNSKRND;
ITNTSSNSKRNDP; TNTSSNSKRNDPQ; NTSSNSKRNDPQS;
TSSNSKRNDPQSL; SSNSKRNDPQSLG; SNSKRNDPQSLGQ;
NSKRNDPQSLGQY; SKRNDPQSLGQYI; KRNDPQSLGQYIK;
RNDPQSLGQYIKN; NDPQSLGQYIKNK; DPQSLGQYIKNKY;
PQSLGQYIKNKYL; QSLGQYIKNKYLD; SLGQYIKNKYLDK;
LGQYIKNKYLDKM; GQYIKNKYLDKMQ; QYIKNKYLDKMQD;
YIKNKYLDKMQDA; IKNKYLDKMQDAR; KNKYLDKMQDARQ;
NKYLDKMQDARQS; KYLDKMQDARQSA; YLDKMQDARQSAL;
LDKMQDARQSALD; DKMQDARQSALDL; KMQDARQSALDLY;
MQDARQSALDLYL; QDARQSALDLYLN; DARQSALDLYLNI;
ARQSALDLYLNIT; RQSALDLYLNITE; QSALDLYLNITEI;
SALDLYLNITEIR;

14 mers:
MSSCTIDANLNKDY; SSCTIDANLNKDYK; SCTIDANLNKDYKN;
CTIDANLNKDYKNK; TIDANLNKDYKNKV; IDANLNKDYKNKVE;
DANLNKDYKNKVEE; ANLNKDYKNKVEEL; NLNKDYKNKVEELL;
LNKDYKNKVEELLN; NKDYKNKVEELLNS; KDYKNKVEELLNSS;
DYKNKVEELLNSST; YKNKVEELLNSSTD; KNKVEELLNSSTDD;
NKVEELLNSSTDDQ; KVEELLNSSTDDQA; VEELLNSSTDDQAK;
EELLNSSTDDQAKI; ELLNSSTDDQAKIS; LLNSSTDDQAKISI;
LNSSTDDQAKISIN; NSSTDDQAKISINT; SSTDDQAKISINTG;
STDDQAKISINTGS; TDDQAKISINTGSN; DDQAKISINTGSNA;
DQAKISINTGSNAT; QAKISINTGSNATK; AKISINTGSNATKN;
KISINTGSNATKNK; ISINTGSNATKNKT; SINTGSNATKNKTN;
INTGSNATKNKTNI; NTGSNATKNKTNIK; TGSNATKNKTNIKV;
GSNATKNKTNIKVA; SNATKNKTNIKVAG; NATKNKTNIKVAGL;
ATKNKTNIKVAGLQ; TKNKTNIKVAGLQK; KNKTNIKVAGLQKN;
NKTNIKVAGLQKNT; KTNIKVAGLQKNTQ; TNIKVAGLQKNTQS;
NIKVAGLQKNTQSK; IKVAGLQKNTQSKK; KVAGLQKNTQSKKN;
VAGLQKNTQSKKNN; AGLQKNTQSKKNNN; GLQKNTQSKKNNNL;
LQKNTQSKKNNNLQ; QKNTQSKKNNNLQG; KNTQSKKNNNLQGL;

NTQSKKNNNLQGLN; TQSKKNNNLQGLNP; QSKKNNNLQGLNPA;
SKKNNNLQGLNPAN; KKNNNLQGLNPANQ; KNNNLQGLNPANQV;
NNNLQGLNPANQVN; NNLQGLNPANQVNP; NLQGLNPANQVNPG;
LQGLNPANQVNPGN; QGLNPANQVNPGNP; GLNPANQVNPGNPM;
LNPANQVNPGNPMQ; NPANQVNPGNPMQI; PANQVNPGNPMQIA;
ANQVNPGNPMQIAN; NQVNPGNPMQIANQ; QVNPGNPMQIANQA;
VNPGNPMQIANQAN; NPGNPMQIANQANQ; PGNPMQIANQANQA;
GNPMQIANQANQAN; NPMQIANQANQANQ; PMQIANQANQANQA;
MQIANQANQANQAN; QIANQANQANQANQ; IANQANQANQANQA;
ANQANQANQANQAN; NQANQANQANQANQ; QANQANQANQANQA;
ANQANQANQANQAN; NQANQANQANQANQ; QANQANQANQANQA;
ANQANQANQANQAS; NQANQANQANQASQ; QANQANQANQASQA;
ANQANQANQASQAS; NQANQANQASQASQ; QANQANQASQASQA;
ANQANQASQASQAS; NQANQASQASQASQ; QANQASQASQASQV;
ANQASQASQASQVA; NQASQASQASQVAS; QASQASQASQVASS;
ASQASQASQVASSA; SQASQASQVASSAS; QASQASQVASSASQ;
ASQASQVASSASQA; SQASQVASSASQAS; QASQVASSASQASP;
ASQVASSASQASPV; SQVASSASQASPVA; QVASSASQASPVAS;
VASSASQASPVASP; ASSASQASPVASPA; SSASQASPVASPAT;
SASQASPVASPATN; ASQASPVASPATNV; SQASPVASPATNVQ;
QASPVASPATNVQA; ASPVASPATNVQAT; SPVASPATNVQATP;
PVASPATNVQATPP; VASPATNVQATPPK; ASPATNVQATPPKQ;
SPATNVQATPPKQI; PATNVQATPPKQIA; ATNVQATPPKQIAS;
TNVQATPPKQIASA; NVQATPPKQIASAQ; VQATPPKQIASAQA;
QATPPKQIASAQAI; ATPPKQIASAQAIQ; TPPKQIASAQAIQT;
PPKQIASAQAIQTV; PKQIASAQAIQTVP; KQIASAQAIQTVPN;
QIASAQAIQTVPNN; IASAQAIQTVPNNT; ASAQAIQTVPNNTS;
SAQAIQTVPNNTST; AQAIQTVPNNTSTP; QAIQTVPNNTSTPN;
AIQTVPNNTSTPNQ; IQTVPNNTSTPNQS; QTVPNNTSTPNQSI;
TVPNNTSTPNQSII; VPNNTSTPNQSIIK; PNNTSTPNQSIIKP;
NNTSTPNQSIIKPQ; NTSTPNQSIIKPQQ; TSTPNQSIIKPQQY;
STPNQSIIKPQQYT; TPNQSIIKPQQYTF; PNQSIIKPQQYTFS;
NQSIIKPQQYTFSS; QSIIKPQQYTFSSS; SIIKPQQYTFSSSF;
IIKPQQYTFSSSFS; IKPQQYTFSSSFSQ; KPQQYTFSSSFSQP;
PQQYTFSSSFSQPT; QQYTFSSSFSQPTS; QYTFSSSFSQPTSQ;
YTFSSSFSQPTSQT; TFSSSFSQPTSQTN; FSSSFSQPTSQTNF;
SSSFSQPTSQTNFN; SSFSQPTSQTNFNN; SFSQPTSQTNFNNS;
FSQPTSQTNFNNSQ; SQPTSQTNFNNSQS; QPTSQTNFNNSQSN;
PTSQTNFNNSQSNN; TSQTNFNNSQSNNV; SQTNFNNSQSNNVL;
QTNFNNSQSNNVLT; TNFNNSQSNNVLTN; NFNNSQSNNVLTNY;
FNNSQSNNVLTNYR; NNSQSNNVLTNYRH; NSQSNNVLTNYRHQ;
SQSNNVLTNYRHQT; QSNNVLTNYRHQTQ; SNNVLTNYRHQTQP;
NNVLTNYRHQTQPS; NVLTNYRHQTQPSF; VLTNYRHQTQPSFV;
LTNYRHQTQPSFVV; TNYRHQTQPSFVVP; NYRHQTQPSFVVPV;
YRHQTQPSFVVPVY; RHQTQPSFVVPVYS; HQTQPSFVVPVYSG;
QTQPSFVVPVYSGN; TQPSFVVPVYSGNS; QPSFVVPVYSGNSP;
PSFVVPVYSGNSPL; SFVVPVYSGNSPLQ; FVVPVYSGNSPLQK;
VVPVYSGNSPLQKL; VPVYSGNSPLQKLK; PVYSGNSPLQKLKN;
VYSGNSPLQKLKNN; YSGNSPLQKLKNNL; SGNSPLQKLKNNLL;
GNSPLQKLKNNLLR; NSPLQKLKNNLLRR; SPLQKLKNNLLRRI;
PLQKLKNNLLRRIA; LQKLKNNLLRRIAE; QKLKNNLLRRIAEE;

| | | |
|---|---|---|
| KLKNNLLRRIAEEK; | LKNNLLRRIAEEKN; | KNNLLRRIAEEKNK; |
| NNLLRRIAEEKNKT; | NLLRRIAEEKNKTH; | LLRRIAEEKNKTHN; |
| LRRIAEEKNKTHNH; | RRIAEEKNKTHNHG; | RIAEEKNKTHNHGF; |
| IAEEKNKTHNHGFR; | AEEKNKTHNHGFRE; | EEKNKTHNHGFRET; |
| EKNKTHNHGFRETY; | KNKTHNHGFRETYD; | NKTHNHGFRETYDQ; |
| KTHNHGFRETYDQF; | THNHGFRETYDQFK; | HNHGFRETYDQFKM; |
| NHGFRETYDQFKMK; | HGFRETYDQFKMKD; | GFRETYDQFKMKDS; |
| FRETYDQFKMKDSA; | RETYDQFKMKDSAF; | ETYDQFKMKDSAFT; |
| TYDQFKMKDSAFTL; | YDQFKMKDSAFTLL; | DQFKMKDSAFTLLD; |
| QFKMKDSAFTLLDV; | FKMKDSAFTLLDVI; | KMKDSAFTLLDVIS; |
| MKDSAFTLLDVISN; | KDSAFTLLDVISNI; | DSAFTLLDVISNIS; |
| SAFTLLDVISNISV; | AFTLLDVISNISVF; | FTLLDVISNISVFD; |
| TLLDVISNISVFDR; | LLDVISNISVFDRG; | LDVISNISVFDRGS; |
| DVISNISVFDRGSA; | VISNISVFDRGSAP; | ISNISVFDRGSAPQ; |
| SNISVFDRGSAPQL; | NISVFDRGSAPQLS; | ISVFDRGSAPQLSS; |
| SVFDRGSAPQLSSN; | VFDRGSAPQLSSNT; | FDRGSAPQLSSNTP; |
| DRGSAPQLSSNTPE; | RGSAPQLSSNTPEA; | GSAPQLSSNTPEAE; |
| SAPQLSSNTPEAES; | APQLSSNTPEAESE; | PQLSSNTPEAESER; |
| QLSSNTPEAESERN; | LSSNTPEAESERNR; | SSNTPEAESERNRL; |
| SNTPEAESERNRLY; | NTPEAESERNRLYA; | TPEAESERNRLYAM; |
| PEAESERNRLYAMM; | EAESERNRLYAMMD; | AESERNRLYAMMDF; |
| ESERNRLYAMMDFD; | SERNRLYAMMDFDQ; | ERNRLYAMMDFDQA; |
| RNRLYAMMDFDQAK; | NRLYAMMDFDQAKT; | RLYAMMDFDQAKTT; |
| LYAMMDFDQAKTTE; | YAMMDFDQAKTTEF; | AMMDFDQAKTTEFG; |
| MMDFDQAKTTEFGS; | MDFDQAKTTEFGSI; | DFDQAKTTEFGSIM; |
| FDQAKTTEFGSIMN; | DQAKTTEFGSIMNI; | QAKTTEFGSIMNIL; |
| AKTTEFGSIMNILY; | KTTEFGSIMNILYQ; | TTEFGSIMNILYQE; |
| TEFGSIMNILYQEN; | EFGSIMNILYQENQ; | FGSIMNILYQENQN; |
| GSIMNILYQENQNH; | SIMNILYQENQNHS; | IMNILYQENQNHSL; |
| MNILYQENQNHSLI; | NILYQENQNHSLIR; | ILYQENQNHSLIRS; |
| LYQENQNHSLIRSL; | YQENQNHSLIRSLI; | QENQNHSLIRSLII; |
| ENQNHSLIRSLIIS; | NQNHSLIRSLIISG; | QNHSLIRSLIISGL; |
| NHSLIRSLIISGLG; | HSLIRSLIISGLGI; | SLIRSLIISGLGIQ; |
| LIRSLIISGLGIQI; | IRSLIISGLGIQIS; | RSLIISGLGIQISL; |
| SLIISGLGIQISLE; | LIISGLGIQISLES; | IISGLGIQISLEST; |
| ISGLGIQISLESTL; | SGLGIQISLESTLE; | GLGIQISLESTLEE; |
| LGIQISLESTLEEI; | GIQISLESTLEEIE; | IQISLESTLEEIEK; |
| QISLESTLEEIEKK; | ISLESTLEEIEKKI; | SLESTLEEIEKKIE; |
| LESTLEEIEKKIES; | ESTLEEIEKKIESF; | STLEEIEKKIESFN; |
| TLEEIEKKIESFNT; | LEEIEKKIESFNTQ; | EEIEKKIESFNTQY; |
| EIEKKIESFNTQYL; | IEKKIESFNTQYLN; | EKKIESFNTQYLNT; |
| KKIESFNTQYLNTI; | KIESFNTQYLNTII; | IESFNTQYLNTIIN; |
| ESFNTQYLNTIINS; | SFNTQYLNTIINSY; | FNTQYLNTIINSYT; |
| NTQYLNTIINSYTF; | TQYLNTIINSYTFK; | QYLNTIINSYTFKD; |
| YLNTIINSYTFKDK; | LNTIINSYTFKDKL; | NTIINSYTFKDKLK; |
| TIINSYTFKDKLKE; | IINSYTFKDKLKEL; | INSYTFKDKLKELE; |
| NSYTFKDKLKELES; | SYTFKDKLKELESK; | YTFKDKLKELESKL; |
| TFKDKLKELESKLN; | FKDKLKELESKLNS; | KDKLKELESKLNSI; |
| DKLKELESKLNSIL; | KLKELESKLNSILA; | LKELESKLNSILAE; |
| KELESKLNSILAEK; | ELESKLNSILAEKK; | LESKLNSILAEKKE; |
| ESKLNSILAEKKEW; | SKLNSILAEKKEWL; | KLNSILAEKKEWLN; |

LNSILAEKKEWLNY; NSILAEKKEWLNYA; SILAEKKEWLNYAD;
ILAEKKEWLNYADA; LAEKKEWLNYADAI; AEKKEWLNYADAII;
EKKEWLNYADAIIT; KKEWLNYADAIITN; KEWLNYADAIITNT;
EWLNYADAIITNTS; WLNYADAIITNTSS; LNYADAIITNTSSN;
NYADAIITNTSSNS; YADAIITNTSSNSK; ADAIITNTSSNSKR;
DAIITNTSSNSKRN; AIITNTSSNSKRND; IITNTSSNSKRNDP;
ITNTSSNSKRNDPQ; TNTSSNSKRNDPQS; NTSSNSKRNDPQSL;
TSSNSKRNDPQSLG; SSNSKRNDPQSLGQ; SNSKRNDPQSLGQY;
NSKRNDPQSLGQYI; SKRNDPQSLGQYIK; KRNDPQSLGQYIKN;
RNDPQSLGQYIKNK; NDPQSLGQYIKNKY; DPQSLGQYIKNKYL;
PQSLGQYIKNKYLD; QSLGQYIKNKYLDK; SLGQYIKNKYLDKM;
LGQYIKNKYLDKMQ; GQYIKNKYLDKMQD; QYIKNKYLDKMQDA;
YIKNKYLDKMQDAR; IKNKYLDKMQDARQ; KNKYLDKMQDARQS;
NKYLDKMQDARQSA; KYLDKMQDARQSAL; YLDKMQDARQSALD;
LDKMQDARQSALDL; DKMQDARQSALDLY; KMQDARQSALDLYL;
MQDARQSALDLYLN; QDARQSALDLYLNI; DARQSALDLYLNIT;
ARQSALDLYLNITE; RQSALDLYLNITEI; QSALDLYLNITEIR;

15 mers:
MSSCTIDANLNKDYK; SSCTIDANLNKDYKN; SCTIDANLNKDYKNK;
CTIDANLNKDYKNKV; TIDANLNKDYKNKVE; IDANLNKDYKNKVEE;
DANLNKDYKNKVEEL; ANLNKDYKNKVEELL; NLNKDYKNKVEELLN;
LNKDYKNKVEELLNS; NKDYKNKVEELLNSS; KDYKNKVEELLNSST;
DYKNKVEELLNSSTD; YKNKVEELLNSSTDD; KNKVEELLNSSTDDQ;
NKVEELLNSSTDDQA; KVEELLNSSTDDQAK; VEELLNSSTDDQAKI;
EELLNSSTDDQAKIS; ELLNSSTDDQAKISI; LLNSSTDDQAKISIN;
LNSSTDDQAKISINT; NSSTDDQAKISINTG; SSTDDQAKISINTGS;
STDDQAKISINTGSN; TDDQAKISINTGSNA; DDQAKISINTGSNAT;
DQAKISINTGSNATK; QAKISINTGSNATKN; AKISINTGSNATKNK;
KISINTGSNATKNKT; ISINTGSNATKNKTN; SINTGSNATKNKTNI;
INTGSNATKNKTNIK; NTGSNATKNKTNIKV; TGSNATKNKTNIKVA;
GSNATKNKTNIKVAG; SNATKNKTNIKVAGL; NATKNKTNIKVAGLQ;
ATKNKTNIKVAGLQK; TKNKTNIKVAGLQKN; KNKTNIKVAGLQKNT;
NKTNIKVAGLQKNTQ; KTNIKVAGLQKNTQS; TNIKVAGLQKNTQSK;
NIKVAGLQKNTQSKK; IKVAGLQKNTQSKKN; KVAGLQKNTQSKKNN;
VAGLQKNTQSKKNNN; AGLQKNTQSKKNNNL; GLQKNTQSKKNNNLQ;
LQKNTQSKKNNNLQG; QKNTQSKKNNNLQGL; KNTQSKKNNNLQGLN;
NTQSKKNNNLQGLNP; TQSKKNNNLQGLNPA; QSKKNNNLQGLNPAN;
SKKNNNLQGLNPANQ; KKNNNLQGLNPANQV; KNNNLQGLNPANQVN;
NNNLQGLNPANQVNP; NNLQGLNPANQVNPG; NLQGLNPANQVNPGN;
LQGLNPANQVNPGNP; QGLNPANQVNPGNPM; GLNPANQVNPGNPMQ;
LNPANQVNPGNPMQI; NPANQVNPGNPMQIA; PANQVNPGNPMQIAN;
ANQVNPGNPMQIANQ; NQVNPGNPMQIANQA; QVNPGNPMQIANQAN;
VNPGNPMQIANQANQ; NPGNPMQIANQANQA; PGNPMQIANQANQAN;
GNPMQIANQANQANQ; NPMQIANQANQANQA; PMQIANQANQANQAN;
MQIANQANQANQANQ; QIANQANQANQANQA; IANQANQANQANQAN;
ANQANQANQANQANQ; NQANQANQANQANQA; QANQANQANQANQAN;
ANQANQANQANQANQ; NQANQANQANQANQA; QANQANQANQANQAS;
ANQANQANQANQASQ; NQANQANQANQASQA; QANQANQANQASQAS;
ANQANQANQASQASQ; NQANQANQASQASQA; QANQANQASQASQAS;
ANQANQASQASQASQ; NQANQASQASQASQV; QANQASQASQASQVA;

| | | |
|---|---|---|
| ANQASQASQASQVAS; | NQASQASQASQVASS; | QASQASQASQVASSA; |
| ASQASQASQVASSAS; | SQASQASQVASSASQ; | QASQASQVASSASQA; |
| ASQASQVASSASQAS; | SQASQVASSASQASP; | QASQVASSASQASPV; |
| ASQVASSASQASPVA; | SQVASSASQASPVAS; | QVASSASQASPVASP; |
| VASSASQASPVASPA; | ASSASQASPVASPAT; | SSASQASPVASPATN; |
| SASQASPVASPATNV; | ASQASPVASPATNVQ; | SQASPVASPATNVQA; |
| QASPVASPATNVQAT; | ASPVASPATNVQATP; | SPVASPATNVQATPP; |
| PVASPATNVQATPPK; | VASPATNVQATPPKQ; | ASPATNVQATPPKQI; |
| SPATNVQATPPKQIA; | PATNVQATPPKQIAS; | ATNVQATPPKQIASA; |
| TNVQATPPKQIASAQ; | NVQATPPKQIASAQA; | VQATPPKQIASAQAI; |
| QATPPKQIASAQAIQ; | ATPPKQIASAQAIQT; | TPPKQIASAQAIQTV; |
| PPKQIASAQAIQTVP; | PKQIASAQAIQTVPN; | KQIASAQAIQTVPNN; |
| QIASAQAIQTVPNNT; | IASAQAIQTVPNNTS; | ASAQAIQTVPNNTST; |
| SAQAIQTVPNNTSTP; | AQAIQTVPNNTSTPN; | QAIQTVPNNTSTPNQ; |
| AIQTVPNNTSTPNQS; | IQTVPNNTSTPNQSI; | QTVPNNTSTPNQSII; |
| TVPNNTSTPNQSIIK; | VPNNTSTPNQSIIKP; | PNNTSTPNQSIIKPQ; |
| NNTSTPNQSIIKPQQ; | NTSTPNQSIIKPQQY; | TSTPNQSIIKPQQYT; |
| STPNQSIIKPQQYTF; | TPNQSIIKPQQYTFS; | PNQSIIKPQQYTFSS; |
| NQSIIKPQQYTFSSS; | QSIIKPQQYTFSSSF; | SIIKPQQYTFSSSFS; |
| IIKPQQYTFSSSFSQ; | IKPQQYTFSSSFSQP; | KPQQYTFSSSFSQPT; |
| PQQYTFSSSFSQPTS; | QQYTFSSSFSQPTSQ; | QYTFSSSFSQPTSQT; |
| YTFSSSFSQPTSQTN; | TFSSSFSQPTSQTNF; | FSSSFSQPTSQTNFN; |
| SSSFSQPTSQTNFNN; | SSFSQPTSQTNFNNS; | SFSQPTSQTNFNNSQ; |
| FSQPTSQTNFNNSQS; | SQPTSQTNFNNSQSN; | QPTSQTNFNNSQSNN; |
| PTSQTNFNNSQSNNV; | TSQTNFNNSQSNNVL; | SQTNFNNSQSNNVLT; |
| QTNFNNSQSNNVLTN; | TNFNNSQSNNVLTNY; | NFNNSQSNNVLTNYR; |
| FNNSQSNNVLTNYRH; | NNSQSNNVLTNYRHQ; | NSQSNNVLTNYRHQT; |
| SQSNNVLTNYRHQTQ; | QSNNVLTNYRHQTQP; | SNNVLTNYRHQTQPS; |
| NNVLTNYRHQTQPSF; | NVLTNYRHQTQPSFV; | VLTNYRHQTQPSFVV; |
| LTNYRHQTQPSFVVP; | TNYRHQTQPSFVVPV; | NYRHQTQPSFVVPVY; |
| YRHQTQPSFVVPVYS; | RHQTQPSFVVPVYSG; | HQTQPSFVVPVYSGN; |
| QTQPSFVVPVYSGNS; | TQPSFVVPVYSGNSP; | QPSFVVPVYSGNSPL; |
| PSFVVPVYSGNSPLQ; | SFVVPVYSGNSPLQK; | FVVPVYSGNSPLQKL; |
| VVPVYSGNSPLQKLK; | VPVYSGNSPLQKLKN; | PVYSGNSPLQKLKNN; |
| VYSGNSPLQKLKNNL; | YSGNSPLQKLKNNLL; | SGNSPLQKLKNNLLR; |
| GNSPLQKLKNNLLRR; | NSPLQKLKNNLLRRI; | SPLQKLKNNLLRRIA; |
| PLQKLKNNLLRRIAE; | LQKLKNNLLRRIAEE; | QKLKNNLLRRIAEEK; |
| KLKNNLLRRIAEEKN; | LKNNLLRRIAEEKNK; | KNNLLRRIAEEKNKT; |
| NNLLRRIAEEKNKTH; | NLLRRIAEEKNKTHN; | LLRRIAEEKNKTHNH; |
| LRRIAEEKNKTHNHG; | RRIAEEKNKTHNHGF; | RIAEEKNKTHNHGFR; |
| IAEEKNKTHNHGFRE; | AEEKNKTHNHGFRET; | EEKNKTHNHGFRETY; |
| EKNKTHNHGFRETYD; | KNKTHNHGFRETYDQ; | NKTHNHGFRETYDQF; |
| KTHNHGFRETYDQFK; | THNHGFRETYDQFKM; | HNHGFRETYDQFKMK; |
| NHGFRETYDQFKMKD; | HGFRETYDQFKMKDS; | GFRETYDQFKMKDSA; |
| FRETYDQFKMKDSAF; | RETYDQFKMKDSAFT; | ETYDQFKMKDSAFTL; |
| TYDQFKMKDSAFTLL; | YDQFKMKDSAFTLLD; | DQFKMKDSAFTLLDV; |
| QFKMKDSAFTLLDVI; | FKMKDSAFTLLDVIS; | KMKDSAFTLLDVISN; |
| MKDSAFTLLDVISNI; | KDSAFTLLDVISNIS; | DSAFTLLDVISNISV; |
| SAFTLLDVISNISVF; | AFTLLDVISNISVFD; | FTLLDVISNISVFDR; |
| TLLDVISNISVFDRG; | LLDVISNISVFDRGS; | LDVISNISVFDRGSA; |
| DVISNISVFDRGSAP; | VISNISVFDRGSAPQ; | ISNISVFDRGSAPQL; |

| | |
|---|---|
| SNISVFDRGSAPQLS; NISVFDRGSAPQLSS; ISVFDRGSAPQLSSN;<br>SVFDRGSAPQLSSNT; VFDRGSAPQLSSNTP; FDRGSAPQLSSNTPE;<br>DRGSAPQLSSNTPEA; RGSAPQLSSNTPEAE; GSAPQLSSNTPEAES;<br>SAPQLSSNTPEAESE; APQLSSNTPEAESER; PQLSSNTPEAESERN;<br>QLSSNTPEAESERNR; LSSNTPEAESERNRL; SSNTPEAESERNRLY;<br>SNTPEAESERNRLYA; NTPEAESERNRLYAM; TPEAESERNRLYAMM;<br>PEAESERNRLYAMMD; EAESERNRLYAMMDF; AESERNRLYAMMDFD;<br>ESERNRLYAMMDFDQ; SERNRLYAMMDFDQA; ERNRLYAMMDFDQAK;<br>RNRLYAMMDFDQAKT; NRLYAMMDFDQAKTT; RLYAMMDFDQAKTTE;<br>LYAMMDFDQAKTTEF; YAMMDFDQAKTTEFG; AMMDFDQAKTTEFGS;<br>MMDFDQAKTTEFGSI; MDFDQAKTTEFGSIM; DFDQAKTTEFGSIMN;<br>FDQAKTTEFGSIMNI; DQAKTTEFGSIMNIL; QAKTTEFGSIMNILY;<br>AKTTEFGSIMNILYQ; KTTEFGSIMNILYQE; TTEFGSIMNILYQEN;<br>TEFGSIMNILYQENQ; EFGSIMNILYQENQN; FGSIMNILYQENQNH;<br>GSIMNILYQENQNHS; SIMNILYQENQNHSL; IMNILYQENQNHSLI;<br>MNILYQENQNHSLIR; NILYQENQNHSLIRS; ILYQENQNHSLIRSL;<br>LYQENQNHSLIRSLI; YQENQNHSLIRSLII; QENQNHSLIRSLIIS;<br>ENQNHSLIRSLIISG; NQNHSLIRSLIISGL; QNHSLIRSLIISGLG;<br>NHSLIRSLIISGLGI; HSLIRSLIISGLGIQ; SLIRSLIISGLGIQI;<br>LIRSLIISGLGIQIS; IRSLIISGLGIQISL; RSLIISGLGIQISLE;<br>SLIISGLGIQISLES; LIISGLGIQISLEST; IISGLGIQISLESTL;<br>ISGLGIQISLESTLE; SGLGIQISLESTLEE; GLGIQISLESTLEEI;<br>LGIQISLESTLEEIE; GIQISLESTLEEIEK; IQISLESTLEEIEKK;<br>QISLESTLEEIEKKI; ISLESTLEEIEKKIE; SLESTLEEIEKKIES;<br>LESTLEEIEKKIESF; ESTLEEIEKKIESFN; STLEEIEKKIESFNT;<br>TLEEIEKKIESFNTQ; LEEIEKKIESFNTQY; EEIEKKIESFNTQYL;<br>EIEKKIESFNTQYLN; IEKKIESFNTQYLNT; EKKIESFNTQYLNTI;<br>KKIESFNTQYLNTII; KIESFNTQYLNTIIN; IESFNTQYLNTIINS;<br>ESFNTQYLNTIINSY; SFNTQYLNTIINSYT; FNTQYLNTIINSYTF;<br>NTQYLNTIINSYTFK; TQYLNTIINSYTFKD; QYLNTIINSYTFKDK;<br>YLNTIINSYTFKDKL; LNTIINSYTFKDKLK; NTIINSYTFKDKLKE;<br>TIINSYTFKDKLKEL; IINSYTFKDKLKELE; INSYTFKDKLKELES;<br>NSYTFKDKLKELESK; SYTFKDKLKELESKL; YTFKDKLKELESKLN;<br>TFKDKLKELESKLNS; FKDKLKELESKLNSI; KDKLKELESKLNSIL;<br>DKLKELESKLNSILA; KLKELESKLNSILAE; LKELESKLNSILAEK;<br>KELESKLNSILAEKK; ELESKLNSILAEKKE; LESKLNSILAEKKEW;<br>ESKLNSILAEKKEWL; SKLNSILAEKKEWLN; KLNSILAEKKEWLNY;<br>LNSILAEKKEWLNYA; NSILAEKKEWLNYAD; SILAEKKEWLNYADA;<br>ILAEKKEWLNYADAI; LAEKKEWLNYADAII; AEKKEWLNYADAIIT;<br>EKKEWLNYADAIITN; KKEWLNYADAIITNT; KEWLNYADAIITNTS;<br>EWLNYADAIITNTSS; WLNYADAIITNTSSN; LNYADAIITNTSSNS;<br>NYADAIITNTSSNSK; YADAIITNTSSNSKR; ADAIITNTSSNSKRN;<br>DAIITNTSSNSKRND; AIITNTSSNSKRNDP; IITNTSSNSKRNDPQ;<br>ITNTSSNSKRNDPQS; TNTSSNSKRNDPQSL; NTSSNSKRNDPQSLG;<br>TSSNSKRNDPQSLGQ; SSNSKRNDPQSLGQY; SNSKRNDPQSLGQYI;<br>NSKRNDPQSLGQYIK; SKRNDPQSLGQYIKN; KRNDPQSLGQYIKNK;<br>RNDPQSLGQYIKNKY; NDPQSLGQYIKNKYL; DPQSLGQYIKNKYLD;<br>PQSLGQYIKNKYLDK; QSLGQYIKNKYLDKM; SLGQYIKNKYLDKMQ;<br>LGQYIKNKYLDKMQD; GQYIKNKYLDKMQDA; QYIKNKYLDKMQDAR;<br>YIKNKYLDKMQDARQ; IKNKYLDKMQDARQS; KNKYLDKMQDARQSA;<br>NKYLDKMQDARQSAL; KYLDKMQDARQSALD; YLDKMQDARQSALDL; |

LDKMQDARQSALDLY; DKMQDARQSALDLYL; KMQDARQSALDLYLN;
MQDARQSALDLYLNI; QDARQSALDLYLNIT; DARQSALDLYLNITE;
ARQSALDLYLNITEI; RQSALDLYLNITEIR;

16 mers:
MSSCTIDANLNKDYKN; SSCTIDANLNKDYKNK; SCTIDANLNKDYKNKV;
CTIDANLNKDYKNKVE; TIDANLNKDYKNKVEE; IDANLNKDYKNKVEEL;
DANLNKDYKNKVEELL; ANLNKDYKNKVEELLN; NLNKDYKNKVEELLNS;
LNKDYKNKVEELLNSS; NKDYKNKVEELLNSST; KDYKNKVEELLNSSTD;
DYKNKVEELLNSSTDD; YKNKVEELLNSSTDDQ; KNKVEELLNSSTDDQA;
NKVEELLNSSTDDQAK; KVEELLNSSTDDQAKI; VEELLNSSTDDQAKIS;
EELLNSSTDDQAKISI; ELLNSSTDDQAKISIN; LLNSSTDDQAKISINT;
LNSSTDDQAKISINTG; NSSTDDQAKISINTGS; SSTDDQAKISINTGSN;
STDDQAKISINTGSNA; TDDQAKISINTGSNAT; DDQAKISINTGSNATK;
DQAKISINTGSNATKN; QAKISINTGSNATKNK; AKISINTGSNATKNKT;
KISINTGSNATKNKTN; ISINTGSNATKNKTNI; SINTGSNATKNKTNIK;
INTGSNATKNKTNIKV; NTGSNATKNKTNIKVA; TGSNATKNKTNIKVAG;
GSNATKNKTNIKVAGL; SNATKNKTNIKVAGLQ; NATKNKTNIKVAGLQK;
ATKNKTNIKVAGLQKN; TKNKTNIKVAGLQKNT; KNKTNIKVAGLQKNTQ;
NKTNIKVAGLQKNTQS; KTNIKVAGLQKNTQSK; TNIKVAGLQKNTQSKK;
NIKVAGLQKNTQSKKN; IKVAGLQKNTQSKKNN; KVAGLQKNTQSKKNNN;
VAGLQKNTQSKKNNNL; AGLQKNTQSKKNNNLQ; GLQKNTQSKKNNNLQG;
LQKNTQSKKNNNLQGL; QKNTQSKKNNNLQGLN; KNTQSKKNNNLQGLNP;
NTQSKKNNNLQGLNPA; TQSKKNNNLQGLNPAN; QSKKNNNLQGLNPANQ;
SKKNNNLQGLNPANQV; KKNNNLQGLNPANQVN; KNNNLQGLNPANQVNP;
NNNLQGLNPANQVNPG; NNLQGLNPANQVNPGN; NLQGLNPANQVNPGNP;
LQGLNPANQVNPGNPM; QGLNPANQVNPGNPMQ; GLNPANQVNPGNPMQI;
LNPANQVNPGNPMQIA; NPANQVNPGNPMQIAN; PANQVNPGNPMQIANQ;
ANQVNPGNPMQIANQA; NQVNPGNPMQIANQAN; QVNPGNPMQIANQANQ;
VNPGNPMQIANQANQA; NPGNPMQIANQANQAN; PGNPMQIANQANQANQ;
GNPMQIANQANQANQA; NPMQIANQANQANQAN; PMQIANQANQANQANQ;
MQIANQANQANQANQA; QIANQANQANQANQAN; IANQANQANQANQANQ;
ANQANQANQANQANQA; NQANQANQANQANQAN; QANQANQANQANQANQ;
ANQANQANQANQANQA; NQANQANQANQANQAS; QANQANQANQANQASQ;
ANQANQANQANQASQA; NQANQANQANQASQAS; QANQANQANQASQASQ;
ANQANQANQASQASQA; NQANQANQASQASQAS; QANQANQASQASQASQ;
ANQANQASQASQASQV; NQANQASQASQASQVA; QANQASQASQASQVAS;
ANQASQASQASQVASS; NQASQASQASQVASSA; QASQASQASQVASSAS;
ASQASQASQVASSASQ; SQASQASQVASSASQA; QASQASQVASSASQAS;
ASQASQVASSASQASP; SQASQVASSASQASPV; QASQVASSASQASPVA;
ASQVASSASQASPVAS; SQVASSASQASPVASP; QVASSASQASPVASPA;
VASSASQASPVASPAT; ASSASQASPVASPATN; SSASQASPVASPATNV;
SASQASPVASPATNVQ; ASQASPVASPATNVQA; SQASPVASPATNVQAT;
QASPVASPATNVQATP; ASPVASPATNVQATPP; SPVASPATNVQATPPK;
PVASPATNVQATPPKQ; VASPATNVQATPPKQI; ASPATNVQATPPKQIA;
SPATNVQATPPKQIAS; PATNVQATPPKQIASA; ATNVQATPPKQIASAQ;
TNVQATPPKQIASAQA; NVQATPPKQIASAQAI; VQATPPKQIASAQAIQ;
QATPPKQIASAQAIQT; ATPPKQIASAQAIQTV; TPPKQIASAQAIQTVP;
PPKQIASAQAIQTVPN; PKQIASAQAIQTVPNN; KQIASAQAIQTVPNNT;
QIASAQAIQTVPNNTS; IASAQAIQTVPNNTST; ASAQAIQTVPNNTSTP;
SAQAIQTVPNNTSTPN; AQAIQTVPNNTSTPNQ; QAIQTVPNNTSTPNQS;

AIQTVPNNTSTPNQSI; IQTVPNNTSTPNQSII; QTVPNNTSTPNQSIIK;
TVPNNTSTPNQSIIKP; VPNNTSTPNQSIIKPQ; PNNTSTPNQSIIKPQQ;
NNTSTPNQSIIKPQQY; NTSTPNQSIIKPQQYT; TSTPNQSIIKPQQYTF;
STPNQSIIKPQQYTFS; TPNQSIIKPQQYTFSS; PNQSIIKPQQYTFSSS;
NQSIIKPQQYTFSSSF; QSIIKPQQYTFSSSFS; SIIKPQQYTFSSSFSQ;
IIKPQQYTFSSSFSQP; IKPQQYTFSSSFSQPT; KPQQYTFSSSFSQPTS;
PQQYTFSSSFSQPTSQ; QQYTFSSSFSQPTSQT; QYTFSSSFSQPTSQTN;
YTFSSSFSQPTSQTNF; TFSSSFSQPTSQTNFN; FSSSFSQPTSQTNFNN;
SSSFSQPTSQTNFNNS; SSFSQPTSQTNFNNSQ; SFSQPTSQTNFNNSQS;
FSQPTSQTNFNNSQSN; SQPTSQTNFNNSQSNN; QPTSQTNFNNSQSNNV;
PTSQTNFNNSQSNNVL; TSQTNFNNSQSNNVLT; SQTNFNNSQSNNVLTN;
QTNFNNSQSNNVLTNY; TNFNNSQSNNVLTNYR; NFNNSQSNNVLTNYRH;
FNNSQSNNVLTNYRHQ; NNSQSNNVLTNYRHQT; NSQSNNVLTNYRHQTQ;
SQSNNVLTNYRHQTQP; QSNNVLTNYRHQTQPS; SNNVLTNYRHQTQPSF;
NNVLTNYRHQTQPSFV; NVLTNYRHQTQPSFVV; VLTNYRHQTQPSFVVP;
LTNYRHQTQPSFVVPV; TNYRHQTQPSFVVPVY; NYRHQTQPSFVVPVYS;
YRHQTQPSFVVPVYSG; RHQTQPSFVVPVYSGN; HQTQPSFVVPVYSGNS;
QTQPSFVVPVYSGNSP; TQPSFVVPVYSGNSPL; QPSFVVPVYSGNSPLQ;
PSFVVPVYSGNSPLQK; SFVVPVYSGNSPLQKL; FVVPVYSGNSPLQKLK;
VVPVYSGNSPLQKLKN; VPVYSGNSPLQKLKNN; PVYSGNSPLQKLKNNL;
VYSGNSPLQKLKNNLL; YSGNSPLQKLKNNLLR; SGNSPLQKLKNNLLRR;
GNSPLQKLKNNLLRRI; NSPLQKLKNNLLRRIA; SPLQKLKNNLLRRIAE;
PLQKLKNNLLRRIAEE; LQKLKNNLLRRIAEEK; QKLKNNLLRRIAEEKN;
KLKNNLLRRIAEEKNK; LKNNLLRRIAEEKNKT; KNNLLRRIAEEKNKTH;
NNLLRRIAEEKNKTHN; NLLRRIAEEKNKTHNH; LLRRIAEEKNKTHNHG;
LRRIAEEKNKTHNHGF; RRIAEEKNKTHNHGFR; RIAEEKNKTHNHGFRE;
IAEEKNKTHNHGFRET; AEEKNKTHNHGFRETY; EEKNKTHNHGFRETYD;
EKNKTHNHGFRETYDQ; KNKTHNHGFRETYDQF; NKTHNHGFRETYDQFK;
KTHNHGFRETYDQFKM; THNHGFRETYDQFKMK; HNHGFRETYDQFKMKD;
NHGFRETYDQFKMKDS; HGFRETYDQFKMKDSA; GFRETYDQFKMKDSAF;
FRETYDQFKMKDSAFT; RETYDQFKMKDSAFTL; ETYDQFKMKDSAFTLL;
TYDQFKMKDSAFTLLD; YDQFKMKDSAFTLLDV; DQFKMKDSAFTLLDVI;
QFKMKDSAFTLLDVIS; FKMKDSAFTLLDVISN; KMKDSAFTLLDVISNI;
MKDSAFTLLDVISNIS; KDSAFTLLDVISNISV; DSAFTLLDVISNISVF;
SAFTLLDVISNISVFD; AFTLLDVISNISVFDR; FTLLDVISNISVFDRG;
TLLDVISNISVFDRGS; LLDVISNISVFDRGSA; LDVISNISVFDRGSAP;
DVISNISVFDRGSAPQ; VISNISVFDRGSAPQL; ISNISVFDRGSAPQLS;
SNISVFDRGSAPQLSS; NISVFDRGSAPQLSSN; ISVFDRGSAPQLSSNT;
SVFDRGSAPQLSSNTP; VFDRGSAPQLSSNTPE; FDRGSAPQLSSNTPEA;
DRGSAPQLSSNTPEAE; RGSAPQLSSNTPEAES; GSAPQLSSNTPEAESE;
SAPQLSSNTPEAESER; APQLSSNTPEAESERN; PQLSSNTPEAESERNR;
QLSSNTPEAESERNRL; LSSNTPEAESERNRLY; SSNTPEAESERNRLYA;
SNTPEAESERNRLYAM; NTPEAESERNRLYAMM; TPEAESERNRLYAMMD;
PEAESERNRLYAMMDF; EAESERNRLYAMMDFD; AESERNRLYAMMDFDQ;
ESERNRLYAMMDFDQA; SERNRLYAMMDFDQAK; ERNRLYAMMDFDQAKT;
RNRLYAMMDFDQAKTT; NRLYAMMDFDQAKTTE; RLYAMMDFDQAKTTEF;
LYAMMDFDQAKTTEFG; YAMMDFDQAKTTEFGS; AMMDFDQAKTTEFGSI;
MMDFDQAKTTEFGSIM; MDFDQAKTTEFGSIMN; DFDQAKTTEFGSIMNI;
FDQAKTTEFGSIMNIL; DQAKTTEFGSIMNILY; QAKTTEFGSIMNILYQ;
AKTTEFGSIMNILYQE; KTTEFGSIMNILYQEN; TTEFGSIMNILYQENQ;
TEFGSIMNILYQENQN; EFGSIMNILYQENQNH; FGSIMNILYQENQNHS;

| | |
|---|---|
| | GSIMNILYQENQNHSL; SIMNILYQENQNHSLI; IMNILYQENQNHSLIR;<br>MNILYQENQNHSLIRS; NILYQENQNHSLIRSL; ILYQENQNHSLIRSLI;<br>LYQENQNHSLIRSLII; YQENQNHSLIRSLIIS; QENQNHSLIRSLIISG;<br>ENQNHSLIRSLIISGL; NQNHSLIRSLIISGLG; QNHSLIRSLIISGLGI;<br>NHSLIRSLIISGLGIQ; HSLIRSLIISGLGIQI; SLIRSLIISGLGIQIS;<br>LIRSLIISGLGIQISL; IRSLIISGLGIQISLE; RSLIISGLGIQISLES;<br>SLIISGLGIQISLEST; LIISGLGIQISLESTL; IISGLGIQISLESTLE;<br>ISGLGIQISLESTLEE; SGLGIQISLESTLEEI; GLGIQISLESTLEEIE;<br>LGIQISLESTLEEIEK; GIQISLESTLEEIEKK; IQISLESTLEEIEKKI;<br>QISLESTLEEIEKKIE; ISLESTLEEIEKKIES; SLESTLEEIEKKIESF;<br>LESTLEEIEKKIESFN; ESTLEEIEKKIESFNT; STLEEIEKKIESFNTQ;<br>TLEEIEKKIESFNTQY; LEEIEKKIESFNTQYL; EEIEKKIESFNTQYLN;<br>EIEKKIESFNTQYLNT; IEKKIESFNTQYLNTI; EKKIESFNTQYLNTII;<br>KKIESFNTQYLNTIIN; KIESFNTQYLNTIINS; IESFNTQYLNTIINSY;<br>ESFNTQYLNTIINSYT; SFNTQYLNTIINSYTF; FNTQYLNTIINSYTFK;<br>NTQYLNTIINSYTFKD; TQYLNTIINSYTFKDK; QYLNTIINSYTFKDKL;<br>YLNTIINSYTFKDKLK; LNTIINSYTFKDKLKE; NTIINSYTFKDKLKEL;<br>TIINSYTFKDKLKELE; IINSYTFKDKLKELES; INSYTFKDKLKELESK;<br>NSYTFKDKLKELESKL; SYTFKDKLKELESKLN; YTFKDKLKELESKLNS;<br>TFKDKLKELESKLNSI; FKDKLKELESKLNSIL; KDKLKELESKLNSILA;<br>DKLKELESKLNSILAE; KLKELESKLNSILAEK; LKELESKLNSILAEKK;<br>KELESKLNSILAEKKE; ELESKLNSILAEKKEW; LESKLNSILAEKKEWL;<br>ESKLNSILAEKKEWLN; SKLNSILAEKKEWLNY; KLNSILAEKKEWLNYA;<br>LNSILAEKKEWLNYAD; NSILAEKKEWLNYADA; SILAEKKEWLNYADAI;<br>ILAEKKEWLNYADAII; LAEKKEWLNYADAIIT; AEKKEWLNYADAIITN;<br>EKKEWLNYADAIITNT; KKEWLNYADAIITNTS; KEWLNYADAIITNTSS;<br>EWLNYADAIITNTSSN; WLNYADAIITNTSSNS; LNYADAIITNTSSNSK;<br>NYADAIITNTSSNSKR; YADAIITNTSSNSKRN; ADAIITNTSSNSKRND;<br>DAIITNTSSNSKRNDP; AIITNTSSNSKRNDPQ; IITNTSSNSKRNDPQS;<br>ITNTSSNSKRNDPQSL; TNTSSNSKRNDPQSLG; NTSSNSKRNDPQSLGQ;<br>TSSNSKRNDPQSLGQY; SSNSKRNDPQSLGQYI; SNSKRNDPQSLGQYIK;<br>NSKRNDPQSLGQYIKN; SKRNDPQSLGQYIKNK; KRNDPQSLGQYIKNKY;<br>RNDPQSLGQYIKNKYL; NDPQSLGQYIKNKYLD; DPQSLGQYIKNKYLDK;<br>PQSLGQYIKNKYLDKM; QSLGQYIKNKYLDKMQ; SLGQYIKNKYLDKMQD;<br>LGQYIKNKYLDKMQDA; GQYIKNKYLDKMQDAR; QYIKNKYLDKMQDARQ;<br>YIKNKYLDKMQDARQS; IKNKYLDKMQDARQSA; KNKYLDKMQDARQSAL;<br>NKYLDKMQDARQSALD; KYLDKMQDARQSALDL; YLDKMQDARQSALDLY;<br>LDKMQDARQSALDLYL; DKMQDARQSALDLYLN; KMQDARQSALDLYLNI;<br>MQDARQSALDLYLNIT; QDARQSALDLYLNITE; DARQSALDLYLNITEI;<br>ARQSALDLYLNITEIR; |
| Seq 36 | 13 mers:<br>MSSCTIDANLNKD; SSCTIDANLNKDY; SCTIDANLNKDYK;<br>CTIDANLNKDYKN; TIDANLNKDYKNK; IDANLNKDYKNKV;<br>DANLNKDYKNKVE; ANLNKDYKNKVEE; NLNKDYKNKVEEL;<br>LNKDYKNKVEELL; NKDYKNKVEELLN; KDYKNKVEELLNS;<br>DYKNKVEELLNSS; YKNKVEELLNSST; KNKVEELLNSSTD;<br>NKVEELLNSSTDD; KVEELLNSSTDDQ; VEELLNSSTDDQA;<br>EELLNSSTDDQAK; ELLNSSTDDQAKI; LLNSSTDDQAKIS;<br>LNSSTDDQAKISI; NSSTDDQAKISIN; SSTDDQAKISINT; |

| | |
|---|---|
| | STDDQAKISINTG; TDDQAKISINTGS; DDQAKISINTGSN; DQAKISINTGSNA; QAKISINTGSNAT; AKISINTGSNATK; KISINTGSNATKN; ISINTGSNATKNK; SINTGSNATKNKT; INTGSNATKNKTN; NTGSNATKNKTNI; TGSNATKNKTNIK; GSNATKNKTNIKV; SNATKNKTNIKVA; NATKNKTNIKVAG; ATKNKTNIKVAGL; TKNKTNIKVAGLQ; KNKTNIKVAGLQK; NKTNIKVAGLQKN; KTNIKVAGLQKNT; TNIKVAGLQKNTQ; NIKVAGLQKNTQS; IKVAGLQKNTQSK; KVAGLQKNTQSKK; VAGLQKNTQSKKN; AGLQKNTQSKKNN; GLQKNTQSKKNNN; LQKNTQSKKNNNL; QKNTQSKKNNNLQ; KNTQSKKNNNLQG; NTQSKKNNNLQGL; TQSKKNNNLQGLN; QSKKNNNLQGLNP; SKKNNNLQGLNPA; KKNNNLQGLNPAN; KNNNLQGLNPANQ; NNNLQGLNPANQV; NNLQGLNPANQVN; NLQGLNPANQVNP; LQGLNPANQVNPG; QGLNPANQVNPGN; GLNPANQVNPGNP; LNPANQVNPGNPM; NPANQVNPGNPMQ; PANQVNPGNPMQI; ANQVNPGNPMQIA; NQVNPGNPMQIAN; QVNPGNPMQIANQ; VNPGNPMQIANQA; NPGNPMQIANQAN; PGNPMQIANQANQ; GNPMQIANQANQA; NPMQIANQANQAN; PMQIANQANQANQ; MQIANQANQANQA; QIANQANQANQAN; IANQANQANQANQ; ANQANQANQANQA; NQANQANQANQAN; QANQANQANQANQ; ANQANQANQANQA; NQANQANQANQAN; QANQANQANQANQ; ANQANQANQANQA; NQANQANQANQAS; QANQANQANQASQ; ANQANQANQASQA; NQANQANQASQAS; QANQANQASQASQ; ANQANQASQASQA; NQANQASQASQAS; QANQASQASQASQ; ANQASQASQASQV; NQASQASQASQVA; QASQASQASQVAS; ASQASQASQVASS; SQASQASQVASSA; QASQASQVASSAS; ASQASQVASSASQ; SQASQVASSASQA; QASQVASSASQAS; ASQVASSASQASP; SQVASSASQASPV; QVASSASQASPVA; VASSASQASPVAS; ASSASQASPVASP; SSASQASPVASPA; SASQASPVASPAT; ASQASPVASPATN; SQASPVASPATNV; QASPVASPATNVQ; ASPVASPATNVQA; SPVASPATNVQAT; PVASPATNVQATP; VASPATNVQATPP; ASPATNVQATPPK; SPATNVQATPPKQ; PATNVQATPPKQI; ATNVQATPPKQIA; TNVQATPPKQIAS; NVQATPPKQIASA; VQATPPKQIASAQ; QATPPKQIASAQA; ATPPKQIASAQAI; TPPKQIASAQAIQ; PPKQIASAQAIQT; PKQIASAQAIQTV; KQIASAQAIQTVP; QIASAQAIQTVPN; IASAQAIQTVPNN; ASAQAIQTVPNNT; SAQAIQTVPNNTS; AQAIQTVPNNTST; QAIQTVPNNTSTP; AIQTVPNNTSTPN; IQTVPNNTSTPNQ; QTVPNNTSTPNQS; TVPNNTSTPNQSI; VPNNTSTPNQSII; PNNTSTPNQSIIK; NNTSTPNQSIIKP; NTSTPNQSIIKPQ; TSTPNQSIIKPQQ; STPNQSIIKPQQY; TPNQSIIKPQQYT; PNQSIIKPQQYTF; NQSIIKPQQYTFS; QSIIKPQQYTFSS; SIIKPQQYTFSSS; IIKPQQYTFSSSF; IKPQQYTFSSSFS; KPQQYTFSSSFSQ; PQQYTFSSSFSQP; QQYTFSSSFSQPT; QYTFSSSFSQPTS; YTFSSSFSQPTSQ; TFSSSFSQPTSQT; FSSSFSQPTSQTN; SSSFSQPTSQTNF; SSFSQPTSQTNFN; SFSQPTSQTNFNN; FSQPTSQTNFNNS; SQPTSQTNFNNSQ; QPTSQTNFNNSQS; PTSQTNFNNSQSN; TSQTNFNNSQSNN; SQTNFNNSQSNNV; QTNFNNSQSNNVL; TNFNNSQSNNVLT; NFNNSQSNNVLTN; FNNSQSNNVLTNY; NNSQSNNVLTNYR; NSQSNNVLTNYRH; |

SQSNNVLTNYRHQ; QSNNVLTNYRHQT; SNNVLTNYRHQTQ;
NNVLTNYRHQTQP; NVLTNYRHQTQPS; VLTNYRHQTQPSF;
LTNYRHQTQPSFV; TNYRHQTQPSFVV; NYRHQTQPSFVVP;
YRHQTQPSFVVPV; RHQTQPSFVVPVY; HQTQPSFVVPVYS;
QTQPSFVVPVYSG; TQPSFVVPVYSGN; QPSFVVPVYSGNS;
PSFVVPVYSGNSP; SFVVPVYSGNSPL; FVVPVYSGNSPLQ;
VVPVYSGNSPLQK; VPVYSGNSPLQKL; PVYSGNSPLQKLK;
VYSGNSPLQKLKN; YSGNSPLQKLKNN; SGNSPLQKLKNNL;
GNSPLQKLKNNLL; NSPLQKLKNNLLR; SPLQKLKNNLLRR;
PLQKLKNNLLRRI; LQKLKNNLLRRIA; QKLKNNLLRRIAE;
KLKNNLLRRIAEE; LKNNLLRRIAEEK; KNNLLRRIAEEKN;
NNLLRRIAEEKNK; NLLRRIAEEKNKT; LLRRIAEEKNKTH;
LRRIAEEKNKTHN; RRIAEEKNKTHNH; RIAEEKNKTHNHG;
IAEEKNKTHNHGF; AEEKNKTHNHGFR; EEKNKTHNHGFRE;
EKNKTHNHGFRET; KNKTHNHGFRETY; NKTHNHGFRETYD;
KTHNHGFRETYDQ; THNHGFRETYDQF; HNHGFRETYDQFK;
NHGFRETYDQFKM; HGFRETYDQFKMK; GFRETYDQFKMKD;
FRETYDQFKMKDS; RETYDQFKMKDSA; ETYDQFKMKDSAF;
TYDQFKMKDSAFT; YDQFKMKDSAFTL; DQFKMKDSAFTLL;
QFKMKDSAFTLLD; FKMKDSAFTLLDV; KMKDSAFTLLDVI;
MKDSAFTLLDVIS; KDSAFTLLDVISN; DSAFTLLDVISNI;
SAFTLLDVISNIS; AFTLLDVISNISV; FTLLDVISNISVF;
TLLDVISNISVFD; LLDVISNISVFDR; LDVISNISVFDRG;
DVISNISVFDRGS; VISNISVFDRGSA; ISNISVFDRGSAP;
SNISVFDRGSAPQ; NISVFDRGSAPQL; ISVFDRGSAPQLS;
SVFDRGSAPQLSS; VFDRGSAPQLSSN; FDRGSAPQLSSNT;
DRGSAPQLSSNTP; RGSAPQLSSNTPE; GSAPQLSSNTPEA;
SAPQLSSNTPEAE; APQLSSNTPEAES; PQLSSNTPEAESE;
QLSSNTPEAESER; LSSNTPEAESERN; SSNTPEAESERNR;
SNTPEAESERNRL; NTPEAESERNRLY; TPEAESERNRLYA;
PEAESERNRLYAM; EAESERNRLYAMM; AESERNRLYAMMD;
ESERNRLYAMMDF; SERNRLYAMMDFD; ERNRLYAMMDFDQ;
RNRLYAMMDFDQA; NRLYAMMDFDQAK; RLYAMMDFDQAKT;
LYAMMDFDQAKTT; YAMMDFDQAKTTE; AMMDFDQAKTTEF;
MMDFDQAKTTEFG; MDFDQAKTTEFGS; DFDQAKTTEFGSI;
FDQAKTTEFGSIM; DQAKTTEFGSIMN; QAKTTEFGSIMNI;
AKTTEFGSIMNIL; KTTEFGSIMNILY; TTEFGSIMNILYQ;
TEFGSIMNILYQE; EFGSIMNILYQEN; FGSIMNILYQENQ;
GSIMNILYQENQN; SIMNILYQENQNH; IMNILYQENQNHS;
MNILYQENQNHSL; NILYQENQNHSLI; ILYQENQNHSLIR;
LYQENQNHSLIRS; YQENQNHSLIRSL; QENQNHSLIRSLI;
ENQNHSLIRSLII; NQNHSLIRSLIIS; QNHSLIRSLIISG;
NHSLIRSLIISGL; HSLIRSLIISGLG; SLIRSLIISGLGI;
LIRSLIISGLGIQ; IRSLIISGLGIQI; RSLIISGLGIQIS;
SLIISGLGIQISL; LIISGLGIQISLE; IISGLGIQISLES;
ISGLGIQISLEST; SGLGIQISLESTL; GLGIQISLESTLE;
LGIQISLESTLEE; GIQISLESTLEEI; IQISLESTLEEIE;
QISLESTLEEIEK; ISLESTLEEIEKK; SLESTLEEIEKKI;
LESTLEEIEKKIE; ESTLEEIEKKIES; STLEEIEKKIESF;
TLEEIEKKIESFN; LEEIEKKIESFNT; EEIEKKIESFNTQ;
EIEKKIESFNTQY; IEKKIESFNTQYL; EKKIESFNTQYLN;

KKIESFNTQYLNT; KIESFNTQYLNTI; IESFNTQYLNTII;
ESFNTQYLNTIIN; SFNTQYLNTIINS; FNTQYLNTIINSY;
NTQYLNTIINSYT; TQYLNTIINSYTF; QYLNTIINSYTFK;
YLNTIINSYTFKD; LNTIINSYTFKDK; NTIINSYTFKDKL;
TIINSYTFKDKLK; IINSYTFKDKLKE; INSYTFKDKLKEL;
NSYTFKDKLKELE; SYTFKDKLKELES; YTFKDKLKELESK;
TFKDKLKELESKL; FKDKLKELESKLN; KDKLKELESKLNS;
DKLKELESKLNSI; KLKELESKLNSIL; LKELESKLNSILA;
KELESKLNSILAE; ELESKLNSILAEK; LESKLNSILAEKK;
ESKLNSILAEKKE; SKLNSILAEKKEW; KLNSILAEKKEWL;
LNSILAEKKEWLN; NSILAEKKEWLNY; SILAEKKEWLNYA;
ILAEKKEWLNYAD; LAEKKEWLNYADA; AEKKEWLNYADAI;
EKKEWLNYADAII; KKEWLNYADAIIT; KEWLNYADAIITN;
EWLNYADAIITNT; WLNYADAIITNTS; LNYADAIITNTSS;
NYADAIITNTSSN; YADAIITNTSSNS; ADAIITNTSSNSK;
DAIITNTSSNSKR; AIITNTSSNSKRN; IITNTSSNSKRND;
ITNTSSNSKRNDP; TNTSSNSKRNDPQ; NTSSNSKRNDPQS;
TSSNSKRNDPQSL; SSNSKRNDPQSLG; SNSKRNDPQSLGQ;
NSKRNDPQSLGQY; SKRNDPQSLGQYI; KRNDPQSLGQYIK;
RNDPQSLGQYIKN; NDPQSLGQYIKNK; DPQSLGQYIKNKY;
PQSLGQYIKNKYL; QSLGQYIKNKYLD; SLGQYIKNKYLDK;
LGQYIKNKYLDKM; GQYIKNKYLDKMQ; QYIKNKYLDKMQD;
YIKNKYLDKMQDA; IKNKYLDKMQDAR; KNKYLDKMQDARQ;
NKYLDKMQDARQS; KYLDKMQDARQSA; YLDKMQDARQSAL;
LDKMQDARQSALD; DKMQDARQSALDL; KMQDARQSALDLY;
MQDARQSALDLYL; QDARQSALDLYLN; DARQSALDLYLNI;
ARQSALDLYLNIT; RQSALDLYLNITE; QSALDLYLNITEI;
SALDLYLNITEIR;

14 mers:
MSSCTIDANLNKDY; SSCTIDANLNKDYK; SCTIDANLNKDYKN;
CTIDANLNKDYKNK; TIDANLNKDYKNKV; IDANLNKDYKNKVE;
DANLNKDYKNKVEE; ANLNKDYKNKVEEL; NLNKDYKNKVEELL;
LNKDYKNKVEELLN; NKDYKNKVEELLNS; KDYKNKVEELLNSS;
DYKNKVEELLNSST; YKNKVEELLNSSTD; KNKVEELLNSSTDD;
NKVEELLNSSTDDQ; KVEELLNSSTDDQA; VEELLNSSTDDQAK;
EELLNSSTDDQAKI; ELLNSSTDDQAKIS; LLNSSTDDQAKISI;
LNSSTDDQAKISIN; NSSTDDQAKISINT; SSTDDQAKISINTG;
STDDQAKISINTGS; TDDQAKISINTGSN; DDQAKISINTGSNA;
DQAKISINTGSNAT; QAKISINTGSNATK; AKISINTGSNATKN;
KISINTGSNATKNK; ISINTGSNATKNKT; SINTGSNATKNKTN;
INTGSNATKNKTNI; NTGSNATKNKTNIK; TGSNATKNKTNIKV;
GSNATKNKTNIKVA; SNATKNKTNIKVAG; NATKNKTNIKVAGL;
ATKNKTNIKVAGLQ; TKNKTNIKVAGLQK; KNKTNIKVAGLQKN;
NKTNIKVAGLQKNT; KTNIKVAGLQKNTQ; TNIKVAGLQKNTQS;
NIKVAGLQKNTQSK; IKVAGLQKNTQSKK; KVAGLQKNTQSKKN;
VAGLQKNTQSKKNN; AGLQKNTQSKKNNN; GLQKNTQSKKNNNL;
LQKNTQSKKNNNLQ; QKNTQSKKNNNLQG; KNTQSKKNNNLQGL;
NTQSKKNNNLQGLN; TQSKKNNNLQGLNP; QSKKNNNLQGLNPA;
SKKNNNLQGLNPAN; KKNNNLQGLNPANQ; KNNNLQGLNPANQV;
NNNLQGLNPANQVN; NNLQGLNPANQVNP; NLQGLNPANQVNPG;

| | | |
|---|---|---|
| LQGLNPANQVNPGN; | QGLNPANQVNPGNP; | GLNPANQVNPGNPM; |
| LNPANQVNPGNPMQ; | NPANQVNPGNPMQI; | PANQVNPGNPMQIA; |
| ANQVNPGNPMQIAN; | NQVNPGNPMQIANQ; | QVNPGNPMQIANQA; |
| VNPGNPMQIANQAN; | NPGNPMQIANQANQ; | PGNPMQIANQANQA; |
| GNPMQIANQANQAN; | NPMQIANQANQANQ; | PMQIANQANQANQA; |
| MQIANQANQANQAN; | QIANQANQANQANQ; | IANQANQANQANQA; |
| ANQANQANQANQAN; | NQANQANQANQANQ; | QANQANQANQANQA; |
| ANQANQANQANQAN; | NQANQANQANQANQ; | QANQANQANQANQA; |
| ANQANQANQANQAS; | NQANQANQANQASQ; | QANQANQANQASQA; |
| ANQANQANQASQAS; | NQANQANQASQASQ; | QANQANQASQASQA; |
| ANQANQASQASQAS; | NQANQASQASQASQ; | QANQASQASQASQV; |
| ANQASQASQASQVA; | NQASQASQASQVAS; | QASQASQASQVASS; |
| ASQASQASQVASSA; | SQASQASQVASSAS; | QASQASQVASSASQ; |
| ASQASQVASSASQA; | SQASQVASSASQAS; | QASQVASSASQASP; |
| ASQVASSASQASPV; | SQVASSASQASPVA; | QVASSASQASPVAS; |
| VASSASQASPVASP; | ASSASQASPVASPA; | SSASQASPVASPAT; |
| SASQASPVASPATN; | ASQASPVASPATNV; | SQASPVASPATNVQ; |
| QASPVASPATNVQA; | ASPVASPATNVQAT; | SPVASPATNVQATP; |
| PVASPATNVQATPP; | VASPATNVQATPPK; | ASPATNVQATPPKQ; |
| SPATNVQATPPKQI; | PATNVQATPPKQIA; | ATNVQATPPKQIAS; |
| TNVQATPPKQIASA; | NVQATPPKQIASAQ; | VQATPPKQIASAQA; |
| QATPPKQIASAQAI; | ATPPKQIASAQAIQ; | TPPKQIASAQAIQT; |
| PPKQIASAQAIQTV; | PKQIASAQAIQTVP; | KQIASAQAIQTVPN; |
| QIASAQAIQTVPNN; | IASAQAIQTVPNNT; | ASAQAIQTVPNNTS; |
| SAQAIQTVPNNTST; | AQAIQTVPNNTSTP; | QAIQTVPNNTSTPN; |
| AIQTVPNNTSTPNQ; | IQTVPNNTSTPNQS; | QTVPNNTSTPNQSI; |
| TVPNNTSTPNQSII; | VPNNTSTPNQSIIK; | PNNTSTPNQSIIKP; |
| NNTSTPNQSIIKPQ; | NTSTPNQSIIKPQQ; | TSTPNQSIIKPQQY; |
| STPNQSIIKPQQYT; | TPNQSIIKPQQYTF; | PNQSIIKPQQYTFS; |
| NQSIIKPQQYTFSS; | QSIIKPQQYTFSSS; | SIIKPQQYTFSSSF; |
| IIKPQQYTFSSSFS; | IKPQQYTFSSSFSQ; | KPQQYTFSSSFSQP; |
| PQQYTFSSSFSQPT; | QQYTFSSSFSQPTS; | QYTFSSSFSQPTSQ; |
| YTFSSSFSQPTSQT; | TFSSSFSQPTSQTN; | FSSSFSQPTSQTNF; |
| SSSFSQPTSQTNFN; | SSFSQPTSQTNFNN; | SFSQPTSQTNFNNS; |
| FSQPTSQTNFNNSQ; | SQPTSQTNFNNSQS; | QPTSQTNFNNSQSN; |
| PTSQTNFNNSQSNN; | TSQTNFNNSQSNNV; | SQTNFNNSQSNNVL; |
| QTNFNNSQSNNVLT; | TNFNNSQSNNVLTN; | NFNNSQSNNVLTNY; |
| FNNSQSNNVLTNYR; | NNSQSNNVLTNYRH; | NSQSNNVLTNYRHQ; |
| SQSNNVLTNYRHQT; | QSNNVLTNYRHQTQ; | SNNVLTNYRHQTQP; |
| NNVLTNYRHQTQPS; | NVLTNYRHQTQPSF; | VLTNYRHQTQPSFV; |
| LTNYRHQTQPSFVV; | TNYRHQTQPSFVVP; | NYRHQTQPSFVVPV; |
| YRHQTQPSFVVPVY; | RHQTQPSFVVPVYS; | HQTQPSFVVPVYSG; |
| QTQPSFVVPVYSGN; | TQPSFVVPVYSGNS; | QPSFVVPVYSGNSP; |
| PSFVVPVYSGNSPL; | SFVVPVYSGNSPLQ; | FVVPVYSGNSPLQK; |
| VVPVYSGNSPLQKL; | VPVYSGNSPLQKLK; | PVYSGNSPLQKLKN; |
| VYSGNSPLQKLKNN; | YSGNSPLQKLKNNL; | SGNSPLQKLKNNLL; |
| GNSPLQKLKNNLLR; | NSPLQKLKNNLLRR; | SPLQKLKNNLLRRI; |
| PLQKLKNNLLRRIA; | LQKLKNNLLRRIAE; | QKLKNNLLRRIAEE; |
| KLKNNLLRRIAEEK; | LKNNLLRRIAEEKN; | KNNLLRRIAEEKNK; |
| NNLLRRIAEEKNKT; | NLLRRIAEEKNKTH; | LLRRIAEEKNKTHN; |
| LRRIAEEKNKTHNH; | RRIAEEKNKTHNHG; | RIAEEKNKTHNHGF; |

| | | |
|---|---|---|
| IAEEKNKTHNHGFR; | AEEKNKTHNHGFRE; | EEKNKTHNHGFRET; |
| EKNKTHNHGFRETY; | KNKTHNHGFRETYD; | NKTHNHGFRETYDQ; |
| KTHNHGFRETYDQF; | THNHGFRETYDQFK; | HNHGFRETYDQFKM; |
| NHGFRETYDQFKMK; | HGFRETYDQFKMKD; | GFRETYDQFKMKDS; |
| FRETYDQFKMKDSA; | RETYDQFKMKDSAF; | ETYDQFKMKDSAFT; |
| TYDQFKMKDSAFTL; | YDQFKMKDSAFTLL; | DQFKMKDSAFTLLD; |
| QFKMKDSAFTLLDV; | FKMKDSAFTLLDVI; | KMKDSAFTLLDVIS; |
| MKDSAFTLLDVISN; | KDSAFTLLDVISNI; | DSAFTLLDVISNIS; |
| SAFTLLDVISNISV; | AFTLLDVISNISVF; | FTLLDVISNISVFD; |
| TLLDVISNISVFDR; | LLDVISNISVFDRG; | LDVISNISVFDRGS; |
| DVISNISVFDRGSA; | VISNISVFDRGSAP; | ISNISVFDRGSAPQ; |
| SNISVFDRGSAPQL; | NISVFDRGSAPQLS; | ISVFDRGSAPQLSS; |
| SVFDRGSAPQLSSN; | VFDRGSAPQLSSNT; | FDRGSAPQLSSNTP; |
| DRGSAPQLSSNTPE; | RGSAPQLSSNTPEA; | GSAPQLSSNTPEAE; |
| SAPQLSSNTPEAES; | APQLSSNTPEAESE; | PQLSSNTPEAESER; |
| QLSSNTPEAESERN; | LSSNTPEAESERNR; | SSNTPEAESERNRL; |
| SNTPEAESERNRLY; | NTPEAESERNRLYA; | TPEAESERNRLYAM; |
| PEAESERNRLYAMM; | EAESERNRLYAMMD; | AESERNRLYAMMDF; |
| ESERNRLYAMMDFD; | SERNRLYAMMDFDQ; | ERNRLYAMMDFDQA; |
| RNRLYAMMDFDQAK; | NRLYAMMDFDQAKT; | RLYAMMDFDQAKTT; |
| LYAMMDFDQAKTTE; | YAMMDFDQAKTTEF; | AMMDFDQAKTTEFG; |
| MMDFDQAKTTEFGS; | MDFDQAKTTEFGSI; | DFDQAKTTEFGSIM; |
| FDQAKTTEFGSIMN; | DQAKTTEFGSIMNI; | QAKTTEFGSIMNIL; |
| AKTTEFGSIMNILY; | KTTEFGSIMNILYQ; | TTEFGSIMNILYQE; |
| TEFGSIMNILYQEN; | EFGSIMNILYQENQ; | FGSIMNILYQENQN; |
| GSIMNILYQENQNH; | SIMNILYQENQNHS; | IMNILYQENQNHSL; |
| MNILYQENQNHSLI; | NILYQENQNHSLIR; | ILYQENQNHSLIRS; |
| LYQENQNHSLIRSL; | YQENQNHSLIRSLI; | QENQNHSLIRSLII; |
| ENQNHSLIRSLIIS; | NQNHSLIRSLIISG; | QNHSLIRSLIISGL; |
| NHSLIRSLIISGLG; | HSLIRSLIISGLGI; | SLIRSLIISGLGIQ; |
| LIRSLIISGLGIQI; | IRSLIISGLGIQIS; | RSLIISGLGIQISL; |
| SLIISGLGIQISLE; | LIISGLGIQISLES; | IISGLGIQISLEST; |
| ISGLGIQISLESTL; | SGLGIQISLESTLE; | GLGIQISLESTLEE; |
| LGIQISLESTLEEI; | GIQISLESTLEEIE; | IQISLESTLEEIEK; |
| QISLESTLEEIEKK; | ISLESTLEEIEKKI; | SLESTLEEIEKKIE; |
| LESTLEEIEKKIES; | ESTLEEIEKKIESF; | STLEEIEKKIESFN; |
| TLEEIEKKIESFNT; | LEEIEKKIESFNTQ; | EEIEKKIESFNTQY; |
| EIEKKIESFNTQYL; | IEKKIESFNTQYLN; | EKKIESFNTQYLNT; |
| KKIESFNTQYLNTI; | KIESFNTQYLNTII; | IESFNTQYLNTIIN; |
| ESFNTQYLNTIINS; | SFNTQYLNTIINSY; | FNTQYLNTIINSYT; |
| NTQYLNTIINSYTF; | TQYLNTIINSYTFK; | QYLNTIINSYTFKD; |
| YLNTIINSYTFKDK; | LNTIINSYTFKDKL; | NTIINSYTFKDKLK; |
| TIINSYTFKDKLKE; | IINSYTFKDKLKEL; | INSYTFKDKLKELE; |
| NSYTFKDKLKELES; | SYTFKDKLKELESK; | YTFKDKLKELESKL; |
| TFKDKLKELESKLN; | FKDKLKELESKLNS; | KDKLKELESKLNSI; |
| DKLKELESKLNSIL; | KLKELESKLNSILA; | LKELESKLNSILAE; |
| KELESKLNSILAEK; | ELESKLNSILAEKK; | LESKLNSILAEKKE; |
| ESKLNSILAEKKEW; | SKLNSILAEKKEWL; | KLNSILAEKKEWLN; |
| LNSILAEKKEWLNY; | NSILAEKKEWLNYA; | SILAEKKEWLNYAD; |
| ILAEKKEWLNYADA; | LAEKKEWLNYADAI; | AEKKEWLNYADAII; |
| EKKEWLNYADAIIT; | KKEWLNYADAIITN; | KEWLNYADAIITNT; |

EWLNYADAIITNTS; WLNYADAIITNTSS; LNYADAIITNTSSN;
NYADAIITNTSSNS; YADAIITNTSSNSK; ADAIITNTSSNSKR;
DAIITNTSSNSKRN; AIITNTSSNSKRND; IITNTSSNSKRNDP;
ITNTSSNSKRNDPQ; TNTSSNSKRNDPQS; NTSSNSKRNDPQSL;
TSSNSKRNDPQSLG; SSNSKRNDPQSLGQ; SNSKRNDPQSLGQY;
NSKRNDPQSLGQYI; SKRNDPQSLGQYIK; KRNDPQSLGQYIKN;
RNDPQSLGQYIKNK; NDPQSLGQYIKNKY; DPQSLGQYIKNKYL;
PQSLGQYIKNKYLD; QSLGQYIKNKYLDK; SLGQYIKNKYLDKM;
LGQYIKNKYLDKMQ; GQYIKNKYLDKMQD; QYIKNKYLDKMQDA;
YIKNKYLDKMQDAR; IKNKYLDKMQDARQ; KNKYLDKMQDARQS;
NKYLDKMQDARQSA; KYLDKMQDARQSAL; YLDKMQDARQSALD;
LDKMQDARQSALDL; DKMQDARQSALDLY; KMQDARQSALDLYL;
MQDARQSALDLYLN; QDARQSALDLYLNI; DARQSALDLYLNIT;
ARQSALDLYLNITE; RQSALDLYLNITEI; QSALDLYLNITEIR;

15 mers:
MSSCTIDANLNKDYK; SSCTIDANLNKDYKN; SCTIDANLNKDYKNK;
CTIDANLNKDYKNKV; TIDANLNKDYKNKVE; IDANLNKDYKNKVEE;
DANLNKDYKNKVEEL; ANLNKDYKNKVEELL; NLNKDYKNKVEELLN;
LNKDYKNKVEELLNS; NKDYKNKVEELLNSS; KDYKNKVEELLNSST;
DYKNKVEELLNSSTD; YKNKVEELLNSSTDD; KNKVEELLNSSTDDQ;
NKVEELLNSSTDDQA; KVEELLNSSTDDQAK; VEELLNSSTDDQAKI;
EELLNSSTDDQAKIS; ELLNSSTDDQAKISI; LLNSSTDDQAKISIN;
LNSSTDDQAKISINT; NSSTDDQAKISINTG; SSTDDQAKISINTGS;
STDDQAKISINTGSN; TDDQAKISINTGSNA; DDQAKISINTGSNAT;
DQAKISINTGSNATK; QAKISINTGSNATKN; AKISINTGSNATKNK;
KISINTGSNATKNKT; ISINTGSNATKNKTN; SINTGSNATKNKTNI;
INTGSNATKNKTNIK; NTGSNATKNKTNIKV; TGSNATKNKTNIKVA;
GSNATKNKTNIKVAG; SNATKNKTNIKVAGL; NATKNKTNIKVAGLQ;
ATKNKTNIKVAGLQK; TKNKTNIKVAGLQKN; KNKTNIKVAGLQKNT;
NKTNIKVAGLQKNTQ; KTNIKVAGLQKNTQS; TNIKVAGLQKNTQSK;
NIKVAGLQKNTQSKK; IKVAGLQKNTQSKKN; KVAGLQKNTQSKKNN;
VAGLQKNTQSKKNNN; AGLQKNTQSKKNNNL; GLQKNTQSKKNNNLQ;
LQKNTQSKKNNNLQG; QKNTQSKKNNNLQGL; KNTQSKKNNNLQGLN;
NTQSKKNNNLQGLNP; TQSKKNNNLQGLNPA; QSKKNNNLQGLNPAN;
SKKNNNLQGLNPANQ; KKNNNLQGLNPANQV; KNNNLQGLNPANQVN;
NNNLQGLNPANQVNP; NNLQGLNPANQVNPG; NLQGLNPANQVNPGN;
LQGLNPANQVNPGNP; QGLNPANQVNPGNPM; GLNPANQVNPGNPMQ;
LNPANQVNPGNPMQI; NPANQVNPGNPMQIA; PANQVNPGNPMQIAN;
ANQVNPGNPMQIANQ; NQVNPGNPMQIANQA; QVNPGNPMQIANQAN;
VNPGNPMQIANQANQ; NPGNPMQIANQANQA; PGNPMQIANQANQAN;
GNPMQIANQANQANQ; NPMQIANQANQANQA; PMQIANQANQANQAN;
MQIANQANQANQANQ; QIANQANQANQANQA; IANQANQANQANQAN;
ANQANQANQANQANQ; NQANQANQANQANQA; QANQANQANQANQAN;
ANQANQANQANQANQ; NQANQANQANQANQA; QANQANQANQANQAS;
ANQANQANQANQASQ; NQANQANQANQASQA; QANQANQANQASQAS;
ANQANQANQASQASQ; NQANQANQASQASQA; QANQANQASQASQAS;
ANQANQASQASQASQ; NQANQASQASQASQV; QANQASQASQASQVA;
ANQASQASQASQVAS; NQASQASQASQVASS; QASQASQASQVASSA;
ASQASQASQVASSAS; SQASQASQVASSASQ; QASQASQVASSASQA;
ASQASQVASSASQAS; SQASQVASSASQASP; QASQVASSASQASPV;

ASQVASSASQASPVA; SQVASSASQASPVAS; QVASSASQASPVASP;
VASSASQASPVASPA; ASSASQASPVASPAT; SSASQASPVASPATN;
SASQASPVASPATNV; ASQASPVASPATNVQ; SQASPVASPATNVQA;
QASPVASPATNVQAT; ASPVASPATNVQATP; SPVASPATNVQATPP;
PVASPATNVQATPPK; VASPATNVQATPPKQ; ASPATNVQATPPKQI;
SPATNVQATPPKQIA; PATNVQATPPKQIAS; ATNVQATPPKQIASA;
TNVQATPPKQIASAQ; NVQATPPKQIASAQA; VQATPPKQIASAQAI;
QATPPKQIASAQAIQ; ATPPKQIASAQAIQT; TPPKQIASAQAIQTV;
PPKQIASAQAIQTVP; PKQIASAQAIQTVPN; KQIASAQAIQTVPNN;
QIASAQAIQTVPNNT; IASAQAIQTVPNNTS; ASAQAIQTVPNNTST;
SAQAIQTVPNNTSTP; AQAIQTVPNNTSTPN; QAIQTVPNNTSTPNQ;
AIQTVPNNTSTPNQS; IQTVPNNTSTPNQSI; QTVPNNTSTPNQSII;
TVPNNTSTPNQSIIK; VPNNTSTPNQSIIKP; PNNTSTPNQSIIKPQ;
NNTSTPNQSIIKPQQ; NTSTPNQSIIKPQQY; TSTPNQSIIKPQQYT;
STPNQSIIKPQQYTF; TPNQSIIKPQQYTFS; PNQSIIKPQQYTFSS;
NQSIIKPQQYTFSSS; QSIIKPQQYTFSSSF; SIIKPQQYTFSSSFS;
IIKPQQYTFSSSFSQ; IKPQQYTFSSSFSQP; KPQQYTFSSSFSQPT;
PQQYTFSSSFSQPTS; QQYTFSSSFSQPTSQ; QYTFSSSFSQPTSQT;
YTFSSSFSQPTSQTN; TFSSSFSQPTSQTNF; FSSSFSQPTSQTNFN;
SSSFSQPTSQTNFNN; SSFSQPTSQTNFNNS; SFSQPTSQTNFNNSQ;
FSQPTSQTNFNNSQS; SQPTSQTNFNNSQSN; QPTSQTNFNNSQSNN;
PTSQTNFNNSQSNNV; TSQTNFNNSQSNNVL; SQTNFNNSQSNNVLT;
QTNFNNSQSNNVLTN; TNFNNSQSNNVLTNY; NFNNSQSNNVLTNYR;
FNNSQSNNVLTNYRH; NNSQSNNVLTNYRHQ; NSQSNNVLTNYRHQT;
SQSNNVLTNYRHQTQ; QSNNVLTNYRHQTQP; SNNVLTNYRHQTQPS;
NNVLTNYRHQTQPSF; NVLTNYRHQTQPSFV; VLTNYRHQTQPSFVV;
LTNYRHQTQPSFVVP; TNYRHQTQPSFVVPV; NYRHQTQPSFVVPVY;
YRHQTQPSFVVPVYS; RHQTQPSFVVPVYSG; HQTQPSFVVPVYSGN;
QTQPSFVVPVYSGNS; TQPSFVVPVYSGNSP; QPSFVVPVYSGNSPL;
PSFVVPVYSGNSPLQ; SFVVPVYSGNSPLQK; FVVPVYSGNSPLQKL;
VVPVYSGNSPLQKLK; VPVYSGNSPLQKLKN; PVYSGNSPLQKLKNN;
VYSGNSPLQKLKNNL; YSGNSPLQKLKNNLL; SGNSPLQKLKNNLLR;
GNSPLQKLKNNLLRR; NSPLQKLKNNLLRRI; SPLQKLKNNLLRRIA;
PLQKLKNNLLRRIAE; LQKLKNNLLRRIAEE; QKLKNNLLRRIAEEK;
KLKNNLLRRIAEEKN; LKNNLLRRIAEEKNK; KNNLLRRIAEEKNKT;
NNLLRRIAEEKNKTH; NLLRRIAEEKNKTHN; LLRRIAEEKNKTHNH;
LRRIAEEKNKTHNHG; RRIAEEKNKTHNHGF; RIAEEKNKTHNHGFR;
IAEEKNKTHNHGFRE; AEEKNKTHNHGFRET; EEKNKTHNHGFRETY;
EKNKTHNHGFRETYD; KNKTHNHGFRETYDQ; NKTHNHGFRETYDQF;
KTHNHGFRETYDQFK; THNHGFRETYDQFKM; HNHGFRETYDQFKMK;
NHGFRETYDQFKMKD; HGFRETYDQFKMKDS; GFRETYDQFKMKDSA;
FRETYDQFKMKDSAF; RETYDQFKMKDSAFT; ETYDQFKMKDSAFTL;
TYDQFKMKDSAFTLL; YDQFKMKDSAFTLLD; DQFKMKDSAFTLLDV;
QFKMKDSAFTLLDVI; FKMKDSAFTLLDVIS; KMKDSAFTLLDVISN;
MKDSAFTLLDVISNI; KDSAFTLLDVISNIS; DSAFTLLDVISNISV;
SAFTLLDVISNISVF; AFTLLDVISNISVFD; FTLLDVISNISVFDR;
TLLDVISNISVFDRG; LLDVISNISVFDRGS; LDVISNISVFDRGSA;
DVISNISVFDRGSAP; VISNISVFDRGSAPQ; ISNISVFDRGSAPQL;
SNISVFDRGSAPQLS; NISVFDRGSAPQLSS; ISVFDRGSAPQLSSN;
SVFDRGSAPQLSSNT; VFDRGSAPQLSSNTP; FDRGSAPQLSSNTPE;
DRGSAPQLSSNTPEA; RGSAPQLSSNTPEAE; GSAPQLSSNTPEAES;

SAPQLSSNTPEAESE; APQLSSNTPEAESER; PQLSSNTPEAESERN;
QLSSNTPEAESERNR; LSSNTPEAESERNRL; SSNTPEAESERNRLY;
SNTPEAESERNRLYA; NTPEAESERNRLYAM; TPEAESERNRLYAMM;
PEAESERNRLYAMMD; EAESERNRLYAMMDF; AESERNRLYAMMDFD;
ESERNRLYAMMDFDQ; SERNRLYAMMDFDQA; ERNRLYAMMDFDQAK;
RNRLYAMMDFDQAKT; NRLYAMMDFDQAKTT; RLYAMMDFDQAKTTE;
LYAMMDFDQAKTTEF; YAMMDFDQAKTTEFG; AMMDFDQAKTTEFGS;
MMDFDQAKTTEFGSI; MDFDQAKTTEFGSIM; DFDQAKTTEFGSIMN;
FDQAKTTEFGSIMNI; DQAKTTEFGSIMNIL; QAKTTEFGSIMNILY;
AKTTEFGSIMNILYQ; KTTEFGSIMNILYQE; TTEFGSIMNILYQEN;
TEFGSIMNILYQENQ; EFGSIMNILYQENQN; FGSIMNILYQENQNH;
GSIMNILYQENQNHS; SIMNILYQENQNHSL; IMNILYQENQNHSLI;
MNILYQENQNHSLIR; NILYQENQNHSLIRS; ILYQENQNHSLIRSL;
LYQENQNHSLIRSLI; YQENQNHSLIRSLII; QENQNHSLIRSLIIS;
ENQNHSLIRSLIISG; NQNHSLIRSLIISGL; QNHSLIRSLIISGLG;
NHSLIRSLIISGLGI; HSLIRSLIISGLGIQ; SLIRSLIISGLGIQI;
LIRSLIISGLGIQIS; IRSLIISGLGIQISL; RSLIISGLGIQISLE;
SLIISGLGIQISLES; LIISGLGIQISLEST; IISGLGIQISLESTL;
ISGLGIQISLESTLE; SGLGIQISLESTLEE; GLGIQISLESTLEEI;
LGIQISLESTLEEIE; GIQISLESTLEEIEK; IQISLESTLEEIEKK;
QISLESTLEEIEKKI; ISLESTLEEIEKKIE; SLESTLEEIEKKIES;
LESTLEEIEKKIESF; ESTLEEIEKKIESFN; STLEEIEKKIESFNT;
TLEEIEKKIESFNTQ; LEEIEKKIESFNTQY; EEIEKKIESFNTQYL;
EIEKKIESFNTQYLN; IEKKIESFNTQYLNT; EKKIESFNTQYLNTI;
KKIESFNTQYLNTII; KIESFNTQYLNTIIN; IESFNTQYLNTIINS;
ESFNTQYLNTIINSY; SFNTQYLNTIINSYT; FNTQYLNTIINSYTF;
NTQYLNTIINSYTFK; TQYLNTIINSYTFKD; QYLNTIINSYTFKDK;
YLNTIINSYTFKDKL; LNTIINSYTFKDKLK; NTIINSYTFKDKLKE;
TIINSYTFKDKLKEL; IINSYTFKDKLKELE; INSYTFKDKLKELES;
NSYTFKDKLKELESK; SYTFKDKLKELESKL; YTFKDKLKELESKLN;
TFKDKLKELESKLNS; FKDKLKELESKLNSI; KDKLKELESKLNSIL;
DKLKELESKLNSILA; KLKELESKLNSILAE; LKELESKLNSILAEK;
KELESKLNSILAEKK; ELESKLNSILAEKKE; LESKLNSILAEKKEW;
ESKLNSILAEKKEWL; SKLNSILAEKKEWLN; KLNSILAEKKEWLNY;
LNSILAEKKEWLNYA; NSILAEKKEWLNYAD; SILAEKKEWLNYADA;
ILAEKKEWLNYADAI; LAEKKEWLNYADAII; AEKKEWLNYADAIIT;
EKKEWLNYADAIITN; KKEWLNYADAIITNT; KEWLNYADAIITNTS;
EWLNYADAIITNTSS; WLNYADAIITNTSSN; LNYADAIITNTSSNS;
NYADAIITNTSSNSK; YADAIITNTSSNSKR; ADAIITNTSSNSKRN;
DAIITNTSSNSKRND; AIITNTSSNSKRNDP; IITNTSSNSKRNDPQ;
ITNTSSNSKRNDPQS; TNTSSNSKRNDPQSL; NTSSNSKRNDPQSLG;
TSSNSKRNDPQSLGQ; SSNSKRNDPQSLGQY; SNSKRNDPQSLGQYI;
NSKRNDPQSLGQYIK; SKRNDPQSLGQYIKN; KRNDPQSLGQYIKNK;
RNDPQSLGQYIKNKY; NDPQSLGQYIKNKYL; DPQSLGQYIKNKYLD;
PQSLGQYIKNKYLDK; QSLGQYIKNKYLDKM; SLGQYIKNKYLDKMQ;
LGQYIKNKYLDKMQD; GQYIKNKYLDKMQDA; QYIKNKYLDKMQDAR;
YIKNKYLDKMQDARQ; IKNKYLDKMQDARQS; KNKYLDKMQDARQSA;
NKYLDKMQDARQSAL; KYLDKMQDARQSALD; YLDKMQDARQSALDL;
LDKMQDARQSALDLY; DKMQDARQSALDLYL; KMQDARQSALDLYLN;
MQDARQSALDLYLNI; QDARQSALDLYLNIT; DARQSALDLYLNITE;
ARQSALDLYLNITEI; RQSALDLYLNITEIR;

16 mers:

| | | |
|---|---|---|
| MSSCTIDANLNKDYKN; | SSCTIDANLNKDYKNK; | SCTIDANLNKDYKNKV; |
| CTIDANLNKDYKNKVE; | TIDANLNKDYKNKVEE; | IDANLNKDYKNKVEEL; |
| DANLNKDYKNKVEELL; | ANLNKDYKNKVEELLN; | NLNKDYKNKVEELLNS; |
| LNKDYKNKVEELLNSS; | NKDYKNKVEELLNSST; | KDYKNKVEELLNSSTD; |
| DYKNKVEELLNSSTDD; | YKNKVEELLNSSTDDQ; | KNKVEELLNSSTDDQA; |
| NKVEELLNSSTDDQAK; | KVEELLNSSTDDQAKI; | VEELLNSSTDDQAKIS; |
| EELLNSSTDDQAKISI; | ELLNSSTDDQAKISIN; | LLNSSTDDQAKISINT; |
| LNSSTDDQAKISINTG; | NSSTDDQAKISINTGS; | SSTDDQAKISINTGSN; |
| STDDQAKISINTGSNA; | TDDQAKISINTGSNAT; | DDQAKISINTGSNATK; |
| DQAKISINTGSNATKN; | QAKISINTGSNATKNK; | AKISINTGSNATKNKT; |
| KISINTGSNATKNKTN; | ISINTGSNATKNKTNI; | SINTGSNATKNKTNIK; |
| INTGSNATKNKTNIKV; | NTGSNATKNKTNIKVA; | TGSNATKNKTNIKVAG; |
| GSNATKNKTNIKVAGL; | SNATKNKTNIKVAGLQ; | NATKNKTNIKVAGLQK; |
| ATKNKTNIKVAGLQKN; | TKNKTNIKVAGLQKNT; | KNKTNIKVAGLQKNTQ; |
| NKTNIKVAGLQKNTQS; | KTNIKVAGLQKNTQSK; | TNIKVAGLQKNTQSKK; |
| NIKVAGLQKNTQSKKN; | IKVAGLQKNTQSKKNN; | KVAGLQKNTQSKKNNN; |
| VAGLQKNTQSKKNNNL; | AGLQKNTQSKKNNNLQ; | GLQKNTQSKKNNNLQG; |
| LQKNTQSKKNNNLQGL; | QKNTQSKKNNNLQGLN; | KNTQSKKNNNLQGLNP; |
| NTQSKKNNNLQGLNPA; | TQSKKNNNLQGLNPAN; | QSKKNNNLQGLNPANQ; |
| SKKNNNLQGLNPANQV; | KKNNNLQGLNPANQVN; | KNNNLQGLNPANQVNP; |
| NNNLQGLNPANQVNPG; | NNLQGLNPANQVNPGN; | NLQGLNPANQVNPGNP; |
| LQGLNPANQVNPGNPM; | QGLNPANQVNPGNPMQ; | GLNPANQVNPGNPMQI; |
| LNPANQVNPGNPMQIA; | NPANQVNPGNPMQIAN; | PANQVNPGNPMQIANQ; |
| ANQVNPGNPMQIANQA; | NQVNPGNPMQIANQAN; | QVNPGNPMQIANQANQ; |
| VNPGNPMQIANQANQA; | NPGNPMQIANQANQAN; | PGNPMQIANQANQANQ; |
| GNPMQIANQANQANQA; | NPMQIANQANQANQAN; | PMQIANQANQANQANQ; |
| MQIANQANQANQANQA; | QIANQANQANQANQAN; | IANQANQANQANQANQ; |
| ANQANQANQANQANQA; | NQANQANQANQANQAN; | QANQANQANQANQANQ; |
| ANQANQANQANQANQA; | NQANQANQANQANQAS; | QANQANQANQANQASQ; |
| ANQANQANQANQASQA; | NQANQANQANQASQAS; | QANQANQANQASQASQ; |
| ANQANQANQASQASQA; | NQANQANQASQASQAS; | QANQANQASQASQASQ; |
| ANQANQASQASQASQV; | NQANQASQASQASQVA; | QANQASQASQASQVAS; |
| ANQASQASQASQVASS; | NQASQASQASQVASSA; | QASQASQASQVASSAS; |
| ASQASQASQVASSASQ; | SQASQASQVASSASQA; | QASQASQVASSASQAS; |
| ASQASQVASSASQASP; | SQASQVASSASQASPV; | QASQVASSASQASPVA; |
| ASQVASSASQASPVAS; | SQVASSASQASPVASP; | QVASSASQASPVASPA; |
| VASSASQASPVASPAT; | ASSASQASPVASPATN; | SSASQASPVASPATNV; |
| SASQASPVASPATNVQ; | ASQASPVASPATNVQA; | SQASPVASPATNVQAT; |
| QASPVASPATNVQATP; | ASPVASPATNVQATPP; | SPVASPATNVQATPPK; |
| PVASPATNVQATPPKQ; | VASPATNVQATPPKQI; | ASPATNVQATPPKQIA; |
| SPATNVQATPPKQIAS; | PATNVQATPPKQIASA; | ATNVQATPPKQIASAQ; |
| TNVQATPPKQIASAQA; | NVQATPPKQIASAQAI; | VQATPPKQIASAQAIQ; |
| QATPPKQIASAQAIQT; | ATPPKQIASAQAIQTV; | TPPKQIASAQAIQTVP; |
| PPKQIASAQAIQTVPN; | PKQIASAQAIQTVPNN; | KQIASAQAIQTVPNNT; |
| QIASAQAIQTVPNNTS; | IASAQAIQTVPNNTST; | ASAQAIQTVPNNTSTP; |
| SAQAIQTVPNNTSTPN; | AQAIQTVPNNTSTPNQ; | QAIQTVPNNTSTPNQS; |
| AIQTVPNNTSTPNQSI; | IQTVPNNTSTPNQSII; | QTVPNNTSTPNQSIIK; |
| TVPNNTSTPNQSIIKP; | VPNNTSTPNQSIIKPQ; | PNNTSTPNQSIIKPQQ; |
| NNTSTPNQSIIKPQQY; | NTSTPNQSIIKPQQYT; | TSTPNQSIIKPQQYTF; |

STPNQSIIKPQQYTFS; TPNQSIIKPQQYTFSS; PNQSIIKPQQYTFSSS;
NQSIIKPQQYTFSSSF; QSIIKPQQYTFSSSFS; SIIKPQQYTFSSSFSQ;
IIKPQQYTFSSSFSQP; IKPQQYTFSSSFSQPT; KPQQYTFSSSFSQPTS;
PQQYTFSSSFSQPTSQ; QQYTFSSSFSQPTSQT; QYTFSSSFSQPTSQTN;
YTFSSSFSQPTSQTNF; TFSSSFSQPTSQTNFN; FSSSFSQPTSQTNFNN;
SSSFSQPTSQTNFNNS; SSFSQPTSQTNFNNSQ; SFSQPTSQTNFNNSQS;
FSQPTSQTNFNNSQSN; SQPTSQTNFNNSQSNN; QPTSQTNFNNSQSNNV;
PTSQTNFNNSQSNNVL; TSQTNFNNSQSNNVLT; SQTNFNNSQSNNVLTN;
QTNFNNSQSNNVLTNY; TNFNNSQSNNVLTNYR; NFNNSQSNNVLTNYRH;
FNNSQSNNVLTNYRHQ; NNSQSNNVLTNYRHQT; NSQSNNVLTNYRHQTQ;
SQSNNVLTNYRHQTQP; QSNNVLTNYRHQTQPS; SNNVLTNYRHQTQPSF;
NNVLTNYRHQTQPSFV; NVLTNYRHQTQPSFVV; VLTNYRHQTQPSFVVP;
LTNYRHQTQPSFVVPV; TNYRHQTQPSFVVPVY; NYRHQTQPSFVVPVYS;
YRHQTQPSFVVPVYSG; RHQTQPSFVVPVYSGN; HQTQPSFVVPVYSGNS;
QTQPSFVVPVYSGNSP; TQPSFVVPVYSGNSPL; QPSFVVPVYSGNSPLQ;
PSFVVPVYSGNSPLQK; SFVVPVYSGNSPLQKL; FVVPVYSGNSPLQKLK;
VVPVYSGNSPLQKLKN; VPVYSGNSPLQKLKNN; PVYSGNSPLQKLKNNL;
VYSGNSPLQKLKNNLL; YSGNSPLQKLKNNLLR; SGNSPLQKLKNNLLRR;
GNSPLQKLKNNLLRRI; NSPLQKLKNNLLRRIA; SPLQKLKNNLLRRIAE;
PLQKLKNNLLRRIAEE; LQKLKNNLLRRIAEEK; QKLKNNLLRRIAEEKN;
KLKNNLLRRIAEEKNK; LKNNLLRRIAEEKNKT; KNNLLRRIAEEKNKTH;
NNLLRRIAEEKNKTHN; NLLRRIAEEKNKTHNH; LLRRIAEEKNKTHNHG;
LRRIAEEKNKTHNHGF; RRIAEEKNKTHNHGFR; RIAEEKNKTHNHGFRE;
IAEEKNKTHNHGFRET; AEEKNKTHNHGFRETY; EEKNKTHNHGFRETYD;
EKNKTHNHGFRETYDQ; KNKTHNHGFRETYDQF; NKTHNHGFRETYDQFK;
KTHNHGFRETYDQFKM; THNHGFRETYDQFKMK; HNHGFRETYDQFKMKD;
NHGFRETYDQFKMKDS; HGFRETYDQFKMKDSA; GFRETYDQFKMKDSAF;
FRETYDQFKMKDSAFT; RETYDQFKMKDSAFTL; ETYDQFKMKDSAFTLL;
TYDQFKMKDSAFTLLD; YDQFKMKDSAFTLLDV; DQFKMKDSAFTLLDVI;
QFKMKDSAFTLLDVIS; FKMKDSAFTLLDVISN; KMKDSAFTLLDVISNI;
MKDSAFTLLDVISNIS; KDSAFTLLDVISNISV; DSAFTLLDVISNISVF;
SAFTLLDVISNISVFD; AFTLLDVISNISVFDR; FTLLDVISNISVFDRG;
TLLDVISNISVFDRGS; LLDVISNISVFDRGSA; LDVISNISVFDRGSAP;
DVISNISVFDRGSAPQ; VISNISVFDRGSAPQL; ISNISVFDRGSAPQLS;
SNISVFDRGSAPQLSS; NISVFDRGSAPQLSSN; ISVFDRGSAPQLSSNT;
SVFDRGSAPQLSSNTP; VFDRGSAPQLSSNTPE; FDRGSAPQLSSNTPEA;
DRGSAPQLSSNTPEAE; RGSAPQLSSNTPEAES; GSAPQLSSNTPEAESE;
SAPQLSSNTPEAESER; APQLSSNTPEAESERN; PQLSSNTPEAESERNR;
QLSSNTPEAESERNRL; LSSNTPEAESERNRLY; SSNTPEAESERNRLYA;
SNTPEAESERNRLYAM; NTPEAESERNRLYAMM; TPEAESERNRLYAMMD;
PEAESERNRLYAMMDF; EAESERNRLYAMMDFD; AESERNRLYAMMDFDQ;
ESERNRLYAMMDFDQA; SERNRLYAMMDFDQAK; ERNRLYAMMDFDQAKT;
RNRLYAMMDFDQAKTT; NRLYAMMDFDQAKTTE; RLYAMMDFDQAKTTEF;
LYAMMDFDQAKTTEFG; YAMMDFDQAKTTEFGS; AMMDFDQAKTTEFGSI;
MMDFDQAKTTEFGSIM; MDFDQAKTTEFGSIMN; DFDQAKTTEFGSIMNI;
FDQAKTTEFGSIMNIL; DQAKTTEFGSIMNILY; QAKTTEFGSIMNILYQ;
AKTTEFGSIMNILYQE; KTTEFGSIMNILYQEN; TTEFGSIMNILYQENQ;
TEFGSIMNILYQENQN; EFGSIMNILYQENQNH; FGSIMNILYQENQNHS;
GSIMNILYQENQNHSL; SIMNILYQENQNHSLI; IMNILYQENQNHSLIR;
MNILYQENQNHSLIRS; NILYQENQNHSLIRSL; ILYQENQNHSLIRSLI;
LYQENQNHSLIRSLII; YQENQNHSLIRSLIIS; QENQNHSLIRSLIISG;

| | |
|---|---|
| | ENQNHSLIRSLIISGL; NQNHSLIRSLIISGLG; QNHSLIRSLIISGLGI; NHSLIRSLIISGLGIQ; HSLIRSLIISGLGIQI; SLIRSLIISGLGIQIS; LIRSLIISGLGIQISL; IRSLIISGLGIQISLE; RSLIISGLGIQISLES; SLIISGLGIQISLEST; LIISGLGIQISLESTL; IISGLGIQISLESTLE; ISGLGIQISLESTLEE; SGLGIQISLESTLEEI; GLGIQISLESTLEEIE; LGIQISLESTLEEIEK; GIQISLESTLEEIEKK; IQISLESTLEEIEKKI; QISLESTLEEIEKKIE; ISLESTLEEIEKKIES; SLESTLEEIEKKIESF; LESTLEEIEKKIESFN; ESTLEEIEKKIESFNT; STLEEIEKKIESFNTQ; TLEEIEKKIESFNTQY; LEEIEKKIESFNTQYL; EEIEKKIESFNTQYLN; EIEKKIESFNTQYLNT; IEKKIESFNTQYLNTI; EKKIESFNTQYLNTII; KKIESFNTQYLNTIIN; KIESFNTQYLNTIINS; IESFNTQYLNTIINSY; ESFNTQYLNTIINSYT; SFNTQYLNTIINSYTF; FNTQYLNTIINSYTFK; NTQYLNTIINSYTFKD; TQYLNTIINSYTFKDK; QYLNTIINSYTFKDKL; YLNTIINSYTFKDKLK; LNTIINSYTFKDKLKE; NTIINSYTFKDKLKEL; TIINSYTFKDKLKELE; IINSYTFKDKLKELES; INSYTFKDKLKELESK; NSYTFKDKLKELESKL; SYTFKDKLKELESKLN; YTFKDKLKELESKLNS; TFKDKLKELESKLNSI; FKDKLKELESKLNSIL; KDKLKELESKLNSILA; DKLKELESKLNSILAE; KLKELESKLNSILAEK; LKELESKLNSILAEKK; KELESKLNSILAEKKE; ELESKLNSILAEKKEW; LESKLNSILAEKKEWL; ESKLNSILAEKKEWLN; SKLNSILAEKKEWLNY; KLNSILAEKKEWLNYA; LNSILAEKKEWLNYAD; NSILAEKKEWLNYADA; SILAEKKEWLNYADAI; ILAEKKEWLNYADAII; LAEKKEWLNYADAIIT; AEKKEWLNYADAIITN; EKKEWLNYADAIITNT; KKEWLNYADAIITNTS; KEWLNYADAIITNTSS; EWLNYADAIITNTSSN; WLNYADAIITNTSSNS; LNYADAIITNTSSNSK; NYADAIITNTSSNSKR; YADAIITNTSSNSKRN; ADAIITNTSSNSKRND; DAIITNTSSNSKRNDP; AIITNTSSNSKRNDPQ; IITNTSSNSKRNDPQS; ITNTSSNSKRNDPQSL; TNTSSNSKRNDPQSLG; NTSSNSKRNDPQSLGQ; TSSNSKRNDPQSLGQY; SSNSKRNDPQSLGQYI; SNSKRNDPQSLGQYIK; NSKRNDPQSLGQYIKN; SKRNDPQSLGQYIKNK; KRNDPQSLGQYIKNKY; RNDPQSLGQYIKNKYL; NDPQSLGQYIKNKYLD; DPQSLGQYIKNKYLDK; PQSLGQYIKNKYLDKM; QSLGQYIKNKYLDKMQ; SLGQYIKNKYLDKMQD; LGQYIKNKYLDKMQDA; GQYIKNKYLDKMQDAR; QYIKNKYLDKMQDARQ; YIKNKYLDKMQDARQS; IKNKYLDKMQDARQSA; KNKYLDKMQDARQSAL; NKYLDKMQDARQSALD; KYLDKMQDARQSALDL; YLDKMQDARQSALDLY; LDKMQDARQSALDLYL; DKMQDARQSALDLYLN; KMQDARQSALDLYLNI; MQDARQSALDLYLNIT; QDARQSALDLYLNITE; DARQSALDLYLNITEI; ARQSALDLYLNITEIR; |
| Seq 37 | 13 mers: MKKTKLNIIKLNI; KKTKLNIIKLNIL; KTKLNIIKLNILT; TKLNIIKLNILTT; KLNIIKLNILTTT; LNIIKLNILTTTL; NIIKLNILTTTLT; IIKLNILTTTLTL; IKLNILTTTLTLI; KLNILTTTLTLIC; LNILTTTLTLICI; NILTTTLTLICIS; ILTTTLTLICISC; LTTTLTLICISCA; TTTLTLICISCAV; TTLTLICISCAVN; TLTLICISCAVNK; LTLICISCAVNKI; TLICISCAVNKID; LICISCAVNKIDP; ICISCAVNKIDPE; CISCAVNKIDPEP; ISCAVNKIDPEPK; SCAVNKIDPEPKS; CAVNKIDPEPKSK; AVNKIDPEPKSKT; VNKIDPEPKSKTN; NKIDPEPKSKTNK; KIDPEPKSKTNKK; IDPEPKSKTNKKE; DPEPKSKTNKKEN; PEPKSKTNKKENI; EPKSKTNKKENIK; |

PKSKTNKKENIKN; KSKTNKKENIKNF; SKTNKKENIKNFV;
KTNKKENIKNFVN; TNKKENIKNFVNK; NKKENIKNFVNKF;
KKENIKNFVNKFQ; KENIKNFVNKFQD; ENIKNFVNKFQDL;
NIKNFVNKFQDLE; IKNFVNKFQDLEP; KNFVNKFQDLEPS;
NFVNKFQDLEPSK; FVNKFQDLEPSKK; VNKFQDLEPSKKQ;
NKFQDLEPSKKQN; KFQDLEPSKKQNK; FQDLEPSKKQNKD;
QDLEPSKKQNKDL; DLEPSKKQNKDLE; LEPSKKQNKDLEP;
EPSKKQNKDLEPL; PSKKQNKDLEPLR; SKKQNKDLEPLRE;
KKQNKDLEPLREK; KQNKDLEPLREKY; QNKDLEPLREKYP;
NKDLEPLREKYPE; KDLEPLREKYPEA; DLEPLREKYPEAT;
LEPLREKYPEATA; EPLREKYPEATAS; PLREKYPEATASK;
LREKYPEATASKL; REKYPEATASKLE; EKYPEATASKLET;
KYPEATASKLETT; YPEATASKLETTL; PEATASKLETTLK;
EATASKLETTLKI; ATASKLETTLKIL; TASKLETTLKILE;
ASKLETTLKILEA; SKLETTLKILEAQ; KLETTLKILEAQK;
LETTLKILEAQKE; ETTLKILEAQKEK; TTLKILEAQKEKE;
TLKILEAQKEKEN; LKILEAQKEKENI; KILEAQKEKENIE;
ILEAQKEKENIEI; LEAQKEKENIEIA; EAQKEKENIEIAK;
AQKEKENIEIAKI; QKEKENIEIAKID; KEKENIEIAKIDN;
EKENIEIAKIDNT; KENIEIAKIDNTQ; ENIEIAKIDNTQI;
NIEIAKIDNTQID; IEIAKIDNTQIDF; EIAKIDNTQIDFL;
IAKIDNTQIDFLK; AKIDNTQIDFLKT; KIDNTQIDFLKTF;
IDNTQIDFLKTFK; DNTQIDFLKTFKT; NTQIDFLKTFKTD;
TQIDFLKTFKTDP; QIDFLKTFKTDPH; IDFLKTFKTDPHD;
DFLKTFKTDPHDS; FLKTFKTDPHDSL; LKTFKTDPHDSLP;
KTFKTDPHDSLPE; TFKTDPHDSLPED; FKTDPHDSLPEDE;
KTDPHDSLPEDEK; TDPHDSLPEDEKM; DPHDSLPEDEKMQ;
PHDSLPEDEKMQM; HDSLPEDEKMQMK; DSLPEDEKMQMKK;
SLPEDEKMQMKKI; LPEDEKMQMKKII; PEDEKMQMKKIIY;
EDEKMQMKKIIYS; DEKMQMKKIIYSS; EKMQMKKIIYSSL;
KMQMKKIIYSSLN; MQMKKIIYSSLNY; QMKKIIYSSLNYE;
MKKIIYSSLNYET; KKIIYSSLNYETE; KIIYSSLNYETEK;
IIYSSLNYETEKI; IYSSLNYETEKIK; YSSLNYETEKIKI;
SSLNYETEKIKIL; SLNYETEKIKILQ; LNYETEKIKILQE;
NYETEKIKILQEI; YETEKIKILQEIL; ETEKIKILQEILE;
TEKIKILQEILEK; EKIKILQEILEKL; KIKILQEILEKLD;
IKILQEILEKLDK; KILQEILEKLDKN; ILQEILEKLDKNL;
LQEILEKLDKNLQ; QEILEKLDKNLQH; EILEKLDKNLQHK;
ILEKLDKNLQHKK; LEKLDKNLQHKKI; EKLDKNLQHKKIA;
KLDKNLQHKKIAK; LDKNLQHKKIAKN; DKNLQHKKIAKNF;
KNLQHKKIAKNFI; NLQHKKIAKNFIY; LQHKKIAKNFIYD;
QHKKIAKNFIYDI; HKKIAKNFIYDIP; KKIAKNFIYDIPI;
KIAKNFIYDIPII; IAKNFIYDIPIII; AKNFIYDIPIIIQ;
KNFIYDIPIIIQS; NFIYDIPIIIQSR; FIYDIPIIIQSRL;
IYDIPIIIQSRLD; YDIPIIIQSRLDI; DIPIIIQSRLDII;
IPIIIQSRLDIIS; PIIIQSRLDIISK; IIIQSRLDIISKV;
IIQSRLDIISKVI; IQSRLDIISKVIK; QSRLDIISKVIKN;
SRLDIISKVIKNP; RLDIISKVIKNPI; LDIISKVIKNPIK;
DIISKVIKNPIKD; IISKVIKNPIKDE; ISKVIKNPIKDEL;
SKVIKNPIKDELQ; KVIKNPIKDELQI; VIKNPIKDELQIL;
IKNPIKDELQILN; KNPIKDELQILNQ; NPIKDELQILNQK;

EP 2 254 592 B1

PIKDELQILNQKE; IKDELQILNQKEI; KDELQILNQKEIE;
DELQILNQKEIEE; ELQILNQKEIEEL; LQILNQKEIEELL;
QILNQKEIEELLM; ILNQKEIEELLMR; LNQKEIEELLMRI;
NQKEIEELLMRIE; QKEIEELLMRIES; KEIEELLMRIESE;
EIEELLMRIESEL; IEELLMRIESELK; EELLMRIESELKI;
ELLMRIESELKIK; LLMRIESELKIKE; LMRIESELKIKEN;
MRIESELKIKENF; RIESELKIKENFK; IESELKIKENFKK;
ESELKIKENFKKA; SELKIKENFKKAL; ELKIKENFKKALN;
LKIKENFKKALNK; KIKENFKKALNKT; IKENFKKALNKTI;
KENFKKALNKTID; ENFKKALNKTIDA; NFKKALNKTIDAY;
FKKALNKTIDAYN; KKALNKTIDAYNQ; KALNKTIDAYNQD;
ALNKTIDAYNQDS; LNKTIDAYNQDSE; NKTIDAYNQDSEN;
KTIDAYNQDSENI; TIDAYNQDSENIK; IDAYNQDSENIKT;
DAYNQDSENIKTS; AYNQDSENIKTSA; YNQDSENIKTSAE;
NQDSENIKTSAEQ; QDSENIKTSAEQL; DSENIKTSAEQLE;
SENIKTSAEQLEK; ENIKTSAEQLEKH; NIKTSAEQLEKHI;
IKTSAEQLEKHIN; KTSAEQLEKHINE; TSAEQLEKHINEN;
SAEQLEKHINENY; AEQLEKHINENYK; EQLEKHINENYKE;
QLEKHINENYKEF; LEKHINENYKEFN; EKHINENYKEFNS;
KHINENYKEFNSL; HINENYKEFNSLK; INENYKEFNSLKP;
NENYKEFNSLKPI; ENYKEFNSLKPIY;

14 mers:
MKKTKLNIIKLNIL; KKTKLNIIKLNILT; KTKLNIIKLNILTT;
TKLNIIKLNILTTT; KLNIIKLNILTTTL; LNIIKLNILTTTLT;
NIIKLNILTTTLTL; IIKLNILTTTLTLI; IKLNILTTTLTLIC;
KLNILTTTLTLICI; LNILTTTLTLICIS; NILTTTLTLICISC;
ILTTTLTLICISCA; LTTTLTLICISCAV; TTTLTLICISCAVN;
TTLTLICISCAVNK; TLTLICISCAVNKI; LTLICISCAVNKID;
TLICISCAVNKIDP; LICISCAVNKIDPE; ICISCAVNKIDPEP;
CISCAVNKIDPEPK; ISCAVNKIDPEPKS; SCAVNKIDPEPKSK;
CAVNKIDPEPKSKT; AVNKIDPEPKSKTN; VNKIDPEPKSKTNK;
NKIDPEPKSKTNKK; KIDPEPKSKTNKKE; IDPEPKSKTNKKEN;
DPEPKSKTNKKENI; PEPKSKTNKKENIK; EPKSKTNKKENIKN;
PKSKTNKKENIKNF; KSKTNKKENIKNFV; SKTNKKENIKNFVN;
KTNKKENIKNFVNK; TNKKENIKNFVNKF; NKKENIKNFVNKFQ;
KKENIKNFVNKFQD; KENIKNFVNKFQDL; ENIKNFVNKFQDLE;
NIKNFVNKFQDLEP; IKNFVNKFQDLEPS; KNFVNKFQDLEPSK;
NFVNKFQDLEPSKK; FVNKFQDLEPSKKQ; VNKFQDLEPSKKQN;
NKFQDLEPSKKQNK; KFQDLEPSKKQNKD; FQDLEPSKKQNKDL;
QDLEPSKKQNKDLE; DLEPSKKQNKDLEP; LEPSKKQNKDLEPL;
EPSKKQNKDLEPLR; PSKKQNKDLEPLRE; SKKQNKDLEPLREK;
KKQNKDLEPLREKY; KQNKDLEPLREKYP; QNKDLEPLREKYPE;
NKDLEPLREKYPEA; KDLEPLREKYPEAT; DLEPLREKYPEATA;
LEPLREKYPEATAS; EPLREKYPEATASK; PLREKYPEATASKL;
LREKYPEATASKLE; REKYPEATASKLET; EKYPEATASKLETT;
KYPEATASKLETTL; YPEATASKLETTLK; PEATASKLETTLKI;
EATASKLETTLKIL; ATASKLETTLKILE; TASKLETTLKILEA;
ASKLETTLKILEAQ; SKLETTLKILEAQK; KLETTLKILEAQKE;
LETTLKILEAQKEK; ETTLKILEAQKEKE; TTLKILEAQKEKEN;
TLKILEAQKEKENI; LKILEAQKEKENIE; KILEAQKEKENIEI;

1185

EP 2 254 592 B1

| | | |
|---|---|---|
| ILEAQKEKENIEIA; | LEAQKEKENIEIAK; | EAQKEKENIEIAKI; |
| AQKEKENIEIAKID; | QKEKENIEIAKIDN; | KEKENIEIAKIDNT; |
| EKENIEIAKIDNTQ; | KENIEIAKIDNTQI; | ENIEIAKIDNTQID; |
| NIEIAKIDNTQIDF; | IEIAKIDNTQIDFL; | EIAKIDNTQIDFLK; |
| IAKIDNTQIDFLKT; | AKIDNTQIDFLKTF; | KIDNTQIDFLKTFK; |
| IDNTQIDFLKTFKT; | DNTQIDFLKTFKTD; | NTQIDFLKTFKTDP; |
| TQIDFLKTFKTDPH; | QIDFLKTFKTDPHD; | IDFLKTFKTDPHDS; |
| DFLKTFKTDPHDSL; | FLKTFKTDPHDSLP; | LKTFKTDPHDSLPE; |
| KTFKTDPHDSLPED; | TFKTDPHDSLPEDE; | FKTDPHDSLPEDEK; |
| KTDPHDSLPEDEKM; | TDPHDSLPEDEKMQ; | DPHDSLPEDEKMQM; |
| PHDSLPEDEKMQMK; | HDSLPEDEKMQMKK; | DSLPEDEKMQMKKI; |
| SLPEDEKMQMKKII; | LPEDEKMQMKKIIY; | PEDEKMQMKKIIYS; |
| EDEKMQMKKIIYSS; | DEKMQMKKIIYSSL; | EKMQMKKIIYSSLN; |
| KMQMKKIIYSSLNY; | MQMKKIIYSSLNYE; | QMKKIIYSSLNYET; |
| MKKIIYSSLNYETE; | KKIIYSSLNYETEK; | KIIYSSLNYETEKI; |
| IIYSSLNYETEKIK; | IYSSLNYETEKIKI; | YSSLNYETEKIKIL; |
| SSLNYETEKIKILQ; | SLNYETEKIKILQE; | LNYETEKIKILQEI; |
| NYETEKIKILQEIL; | YETEKIKILQEILE; | ETEKIKILQEILEK; |
| TEKIKILQEILEKL; | EKIKILQEILEKLD; | KIKILQEILEKLDK; |
| IKILQEILEKLDKN; | KILQEILEKLDKNL; | ILQEILEKLDKNLQ; |
| LQEILEKLDKNLQH; | QEILEKLDKNLQHK; | EILEKLDKNLQHKK; |
| ILEKLDKNLQHKKI; | LEKLDKNLQHKKIA; | EKLDKNLQHKKIAK; |
| KLDKNLQHKKIAKN; | LDKNLQHKKIAKNF; | DKNLQHKKIAKNFI; |
| KNLQHKKIAKNFIY; | NLQHKKIAKNFIYD; | LQHKKIAKNFIYDI; |
| QHKKIAKNFIYDIP; | HKKIAKNFIYDIPI; | KKIAKNFIYDIPII; |
| KIAKNFIYDIPIII; | IAKNFIYDIPIIIQ; | AKNFIYDIPIIIQS; |
| KNFIYDIPIIIQSR; | NFIYDIPIIIQSRL; | FIYDIPIIIQSRLD; |
| IYDIPIIIQSRLDI; | YDIPIIIQSRLDII; | DIPIIIQSRLDIIS; |
| IPIIIQSRLDIISK; | PIIIQSRLDIISKV; | IIIQSRLDIISKVI; |
| IIQSRLDIISKVIK; | IQSRLDIISKVIKN; | QSRLDIISKVIKNP; |
| SRLDIISKVIKNPI; | RLDIISKVIKNPIK; | LDIISKVIKNPIKD; |
| DIISKVIKNPIKDE; | IISKVIKNPIKDEL; | ISKVIKNPIKDELQ; |
| SKVIKNPIKDELQI; | KVIKNPIKDELQIL; | VIKNPIKDELQILN; |
| IKNPIKDELQILNQ; | KNPIKDELQILNQK; | NPIKDELQILNQKE; |
| PIKDELQILNQKEI; | IKDELQILNQKEIE; | KDELQILNQKEIEE; |
| DELQILNQKEIEEL; | ELQILNQKEIEELL; | LQILNQKEIEELLM; |
| QILNQKEIEELLMR; | ILNQKEIEELLMRI; | LNQKEIEELLMRIE; |
| NQKEIEELLMRIES; | QKEIEELLMRIESE; | KEIEELLMRIESEL; |
| EIEELLMRIESELK; | IEELLMRIESELKI; | EELLMRIESELKIK; |
| ELLMRIESELKIKE; | LLMRIESELKIKEN; | LMRIESELKIKENF; |
| MRIESELKIKENFK; | RIESELKIKENFKK; | IESELKIKENFKKA; |
| ESELKIKENFKKAL; | SELKIKENFKKALN; | ELKIKENFKKALNK; |
| LKIKENFKKALNKT; | KIKENFKKALNKTI; | IKENFKKALNKTID; |
| KENFKKALNKTIDA; | ENFKKALNKTIDAY; | NFKKALNKTIDAYN; |
| FKKALNKTIDAYNQ; | KKALNKTIDAYNQD; | KALNKTIDAYNQDS; |
| ALNKTIDAYNQDSE; | LNKTIDAYNQDSEN; | NKTIDAYNQDSENI; |
| KTIDAYNQDSENIK; | TIDAYNQDSENIKT; | IDAYNQDSENIKTS; |
| DAYNQDSENIKTSA; | AYNQDSENIKTSAE; | YNQDSENIKTSAEQ; |
| NQDSENIKTSAEQL; | QDSENIKTSAEQLE; | DSENIKTSAEQLEK; |
| SENIKTSAEQLEKH; | ENIKTSAEQLEKHI; | NIKTSAEQLEKHIN; |
| IKTSAEQLEKHINE; | KTSAEQLEKHINEN; | TSAEQLEKHINENY; |

SAEQLEKHINENYK; AEQLEKHINENYKE; EQLEKHINENYKEF;
QLEKHINENYKEFN; LEKHINENYKEFNS; EKHINENYKEFNSL;
KHINENYKEFNSLK; HINENYKEFNSLKP; INENYKEFNSLKPI;
NENYKEFNSLKPIY;

15 mers:
MKKTKLNIIKLNILT; KKTKLNIIKLNILTT; KTKLNIIKLNILTTT;
TKLNIIKLNILTTTL; KLNIIKLNILTTTLT; LNIIKLNILTTTLTL;
NIIKLNILTTTLTLI; IIKLNILTTTLTLIC; IKLNILTTTLTLICI;
KLNILTTTLTLICIS; LNILTTTLTLICISC; NILTTTLTLICISCA;
ILTTTLTLICISCAV; LTTTLTLICISCAVN; TTTLTLICISCAVNK;
TTLTLICISCAVNKI; TLTLICISCAVNKID; LTLICISCAVNKIDP;
TLICISCAVNKIDPE; LICISCAVNKIDPEP; ICISCAVNKIDPEPK;
CISCAVNKIDPEPKS; ISCAVNKIDPEPKSK; SCAVNKIDPEPKSKT;
CAVNKIDPEPKSKTN; AVNKIDPEPKSKTNK; VNKIDPEPKSKTNKK;
NKIDPEPKSKTNKKE; KIDPEPKSKTNKKEN; IDPEPKSKTNKKENI;
DPEPKSKTNKKENIK; PEPKSKTNKKENIKN; EPKSKTNKKENIKNF;
PKSKTNKKENIKNFV; KSKTNKKENIKNFVN; SKTNKKENIKNFVNK;
KTNKKENIKNFVNKF; TNKKENIKNFVNKFQ; NKKENIKNFVNKFQD;
KKENIKNFVNKFQDL; KENIKNFVNKFQDLE; ENIKNFVNKFQDLEP;
NIKNFVNKFQDLEPS; IKNFVNKFQDLEPSK; KNFVNKFQDLEPSKK;
NFVNKFQDLEPSKKQ; FVNKFQDLEPSKKQN; VNKFQDLEPSKKQNK;
NKFQDLEPSKKQNKD; KFQDLEPSKKQNKDL; FQDLEPSKKQNKDLE;
QDLEPSKKQNKDLEP; DLEPSKKQNKDLEPL; LEPSKKQNKDLEPLR;
EPSKKQNKDLEPLRE; PSKKQNKDLEPLREK; SKKQNKDLEPLREKY;
KKQNKDLEPLREKYP; KQNKDLEPLREKYPE; QNKDLEPLREKYPEA;
NKDLEPLREKYPEAT; KDLEPLREKYPEATA; DLEPLREKYPEATAS;
LEPLREKYPEATASK; EPLREKYPEATASKL; PLREKYPEATASKLE;
LREKYPEATASKLET; REKYPEATASKLETT; EKYPEATASKLETTL;
KYPEATASKLETTLK; YPEATASKLETTLKI; PEATASKLETTLKIL;
EATASKLETTLKILE; ATASKLETTLKILEA; TASKLETTLKILEAQ;
ASKLETTLKILEAQK; SKLETTLKILEAQKE; KLETTLKILEAQKEK;
LETTLKILEAQKEKE; ETTLKILEAQKEKEN; TTLKILEAQKEKENI;
TLKILEAQKEKENIE; LKILEAQKEKENIEI; KILEAQKEKENIEIA;
ILEAQKEKENIEIAK; LEAQKEKENIEIAKI; EAQKEKENIEIAKID;
AQKEKENIEIAKIDN; QKEKENIEIAKIDNT; KEKENIEIAKIDNTQ;
EKENIEIAKIDNTQI; KENIEIAKIDNTQID; ENIEIAKIDNTQIDF;
NIEIAKIDNTQIDFL; IEIAKIDNTQIDFLK; EIAKIDNTQIDFLKT;
IAKIDNTQIDFLKTF; AKIDNTQIDFLKTFK; KIDNTQIDFLKTFKT;
IDNTQIDFLKTFKTD; DNTQIDFLKTFKTDP; NTQIDFLKTFKTDPH;
TQIDFLKTFKTDPHD; QIDFLKTFKTDPHDS; IDFLKTFKTDPHDSL;
DFLKTFKTDPHDSLP; FLKTFKTDPHDSLPE; LKTFKTDPHDSLPED;
KTFKTDPHDSLPEDE; TFKTDPHDSLPEDEK; FKTDPHDSLPEDEKM;
KTDPHDSLPEDEKMQ; TDPHDSLPEDEKMQM; DPHDSLPEDEKMQMK;
PHDSLPEDEKMQMKK; HDSLPEDEKMQMKKI; DSLPEDEKMQMKKII;
SLPEDEKMQMKKIIY; LPEDEKMQMKKIIYS; PEDEKMQMKKIIYSS;
EDEKMQMKKIIYSSL; DEKMQMKKIIYSSLN; EKMQMKKIIYSSLNY;
KMQMKKIIYSSLNYE; MQMKKIIYSSLNYET; QMKKIIYSSLNYETE;
MKKIIYSSLNYETEK; KKIIYSSLNYETEKI; KIIYSSLNYETEKIK;
IIYSSLNYETEKIKI; IYSSLNYETEKIKIL; YSSLNYETEKIKILQ;
SSLNYETEKIKILQE; SLNYETEKIKILQEI; LNYETEKIKILQEIL;

NYETEKIKILQEILE; YETEKIKILQEILEK; ETEKIKILQEILEKL;
TEKIKILQEILEKLD; EKIKILQEILEKLDK; KIKILQEILEKLDKN;
IKILQEILEKLDKNL; KILQEILEKLDKNLQ; ILQEILEKLDKNLQH;
LQEILEKLDKNLQHK; QEILEKLDKNLQHKK; EILEKLDKNLQHKKI;
ILEKLDKNLQHKKIA; LEKLDKNLQHKKIAK; EKLDKNLQHKKIAKN;
KLDKNLQHKKIAKNF; LDKNLQHKKIAKNFI; DKNLQHKKIAKNFIY;
KNLQHKKIAKNFIYD; NLQHKKIAKNFIYDI; LQHKKIAKNFIYDIP;
QHKKIAKNFIYDIPI; HKKIAKNFIYDIPII; KKIAKNFIYDIPIII;
KIAKNFIYDIPIIIQ; IAKNFIYDIPIIIQS; AKNFIYDIPIIIQSR;
KNFIYDIPIIIQSRL; NFIYDIPIIIQSRLD; FIYDIPIIIQSRLDI;
IYDIPIIIQSRLDII; YDIPIIIQSRLDIIS; DIPIIIQSRLDIISK;
IPIIIQSRLDIISKV; PIIIQSRLDIISKVI; IIIQSRLDIISKVIK;
IIQSRLDIISKVIKN; IQSRLDIISKVIKNP; QSRLDIISKVIKNPI;
SRLDIISKVIKNPIK; RLDIISKVIKNPIKD; LDIISKVIKNPIKDE;
DIISKVIKNPIKDEL; IISKVIKNPIKDELQ; ISKVIKNPIKDELQI;
SKVIKNPIKDELQIL; KVIKNPIKDELQILN; VIKNPIKDELQILNQ;
IKNPIKDELQILNQK; KNPIKDELQILNQKE; NPIKDELQILNQKEI;
PIKDELQILNQKEIE; IKDELQILNQKEIEE; KDELQILNQKEIEEL;
DELQILNQKEIEELL; ELQILNQKEIEELLM; LQILNQKEIEELLMR;
QILNQKEIEELLMRI; ILNQKEIEELLMRIE; LNQKEIEELLMRIES;
NQKEIEELLMRIESE; QKEIEELLMRIESEL; KEIEELLMRIESELK;
EIEELLMRIESELKI; IEELLMRIESELKIK; EELLMRIESELKIKE;
ELLMRIESELKIKEN; LLMRIESELKIKENF; LMRIESELKIKENFK;
MRIESELKIKENFKK; RIESELKIKENFKKA; IESELKIKENFKKAL;
ESELKIKENFKKALN; SELKIKENFKKALNK; ELKIKENFKKALNKT;
LKIKENFKKALNKTI; KIKENFKKALNKTID; IKENFKKALNKTIDA;
KENFKKALNKTIDAY; ENFKKALNKTIDAYN; NFKKALNKTIDAYNQ;
FKKALNKTIDAYNQD; KKALNKTIDAYNQDS; KALNKTIDAYNQDSE;
ALNKTIDAYNQDSEN; LNKTIDAYNQDSENI; NKTIDAYNQDSENIK;
KTIDAYNQDSENIKT; TIDAYNQDSENIKTS; IDAYNQDSENIKTSA;
DAYNQDSENIKTSAE; AYNQDSENIKTSAEQ; YNQDSENIKTSAEQL;
NQDSENIKTSAEQLE; QDSENIKTSAEQLEK; DSENIKTSAEQLEKH;
SENIKTSAEQLEKHI; ENIKTSAEQLEKHIN; NIKTSAEQLEKHINE;
IKTSAEQLEKHINEN; KTSAEQLEKHINENY; TSAEQLEKHINENYK;
SAEQLEKHINENYKE; AEQLEKHINENYKEF; EQLEKHINENYKEFN;
QLEKHINENYKEFNS; LEKHINENYKEFNSL; EKHINENYKEFNSLK;
KHINENYKEFNSLKP; HINENYKEFNSLKPI; INENYKEFNSLKPIY;

16 mers:
MKKTKLNIIKLNILTT; KKTKLNIIKLNILTTT; KTKLNIIKLNILTTTL;
TKLNIIKLNILTTTLT; KLNIIKLNILTTTLTL; LNIIKLNILTTTLTLI;
NIIKLNILTTTLTLIC; IIKLNILTTTLTLICI; IKLNILTTTLTLICIS;
KLNILTTTLTLICISC; LNILTTTLTLICISCA; NILTTTLTLICISCAV;
ILTTTLTLICISCAVN; LTTTLTLICISCAVNK; TTTLTLICISCAVNKI;
TTLTLICISCAVNKID; TLTLICISCAVNKIDP; LTLICISCAVNKIDPE;
TLICISCAVNKIDPEP; LICISCAVNKIDPEPK; ICISCAVNKIDPEPKS;
CISCAVNKIDPEPKSK; ISCAVNKIDPEPKSKT; SCAVNKIDPEPKSKTN;
CAVNKIDPEPKSKTNK; AVNKIDPEPKSKTNKK; VNKIDPEPKSKTNKKE;
NKIDPEPKSKTNKKEN; KIDPEPKSKTNKKENI; IDPEPKSKTNKKENIK;
DPEPKSKTNKKENIKN; PEPKSKTNKKENIKNF; EPKSKTNKKENIKNFV;
PKSKTNKKENIKNFVN; KSKTNKKENIKNFVNK; SKTNKKENIKNFVNKF;

KTNKKENIKNFVNKFQ; TNKKENIKNFVNKFQD; NKKENIKNFVNKFQDL;
KKENIKNFVNKFQDLE; KENIKNFVNKFQDLEP; ENIKNFVNKFQDLEPS;
NIKNFVNKFQDLEPSK; IKNFVNKFQDLEPSKK; KNFVNKFQDLEPSKKQ;
NFVNKFQDLEPSKKQN; FVNKFQDLEPSKKQNK; VNKFQDLEPSKKQNKD;
NKFQDLEPSKKQNKDL; KFQDLEPSKKQNKDLE; FQDLEPSKKQNKDLEP;
QDLEPSKKQNKDLEPL; DLEPSKKQNKDLEPLR; LEPSKKQNKDLEPLRE;
EPSKKQNKDLEPLREK; PSKKQNKDLEPLREKY; SKKQNKDLEPLREKYP;
KKQNKDLEPLREKYPE; KQNKDLEPLREKYPEA; QNKDLEPLREKYPEAT;
NKDLEPLREKYPEATA; KDLEPLREKYPEATAS; DLEPLREKYPEATASK;
LEPLREKYPEATASKL; EPLREKYPEATASKLE; PLREKYPEATASKLET;
LREKYPEATASKLETT; REKYPEATASKLETTL; EKYPEATASKLETTLK;
KYPEATASKLETTLKI; YPEATASKLETTLKIL; PEATASKLETTLKILE;
EATASKLETTLKILEA; ATASKLETTLKILEAQ; TASKLETTLKILEAQK;
ASKLETTLKILEAQKE; SKLETTLKILEAQKEK; KLETTLKILEAQKEKE;
LETTLKILEAQKEKEN; ETTLKILEAQKEKENI; TTLKILEAQKEKENIE;
TLKILEAQKEKENIEI; LKILEAQKEKENIEIA; KILEAQKEKENIEIAK;
ILEAQKEKENIEIAKI; LEAQKEKENIEIAKID; EAQKEKENIEIAKIDN;
AQKEKENIEIAKIDNT; QKEKENIEIAKIDNTQ; KEKENIEIAKIDNTQI;
EKENIEIAKIDNTQID; KENIEIAKIDNTQIDF; ENIEIAKIDNTQIDFL;
NIEIAKIDNTQIDFLK; IEIAKIDNTQIDFLKT; EIAKIDNTQIDFLKTF;
IAKIDNTQIDFLKTFK; AKIDNTQIDFLKTFKT; KIDNTQIDFLKTFKTD;
IDNTQIDFLKTFKTDP; DNTQIDFLKTFKTDPH; NTQIDFLKTFKTDPHD;
TQIDFLKTFKTDPHDS; QIDFLKTFKTDPHDSL; IDFLKTFKTDPHDSLP;
DFLKTFKTDPHDSLPE; FLKTFKTDPHDSLPED; LKTFKTDPHDSLPEDE;
KTFKTDPHDSLPEDEK; TFKTDPHDSLPEDEKM; FKTDPHDSLPEDEKMQ;
KTDPHDSLPEDEKMQM; TDPHDSLPEDEKMQMK; DPHDSLPEDEKMQMKK;
PHDSLPEDEKMQMKKI; HDSLPEDEKMQMKKII; DSLPEDEKMQMKKIIY;
SLPEDEKMQMKKIIYS; LPEDEKMQMKKIIYSS; PEDEKMQMKKIIYSSL;
EDEKMQMKKIIYSSLN; DEKMQMKKIIYSSLNY; EKMQMKKIIYSSLNYE;
KMQMKKIIYSSLNYET; MQMKKIIYSSLNYETE; QMKKIIYSSLNYETEK;
MKKIIYSSLNYETEKI; KKIIYSSLNYETEKIK; KIIYSSLNYETEKIKI;
IIYSSLNYETEKIKIL; IYSSLNYETEKIKILQ; YSSLNYETEKIKILQE;
SSLNYETEKIKILQEI; SLNYETEKIKILQEIL; LNYETEKIKILQEILE;
NYETEKIKILQEILEK; YETEKIKILQEILEKL; ETEKIKILQEILEKLD;
TEKIKILQEILEKLDK; EKIKILQEILEKLDKN; KIKILQEILEKLDKNL;
IKILQEILEKLDKNLQ; KILQEILEKLDKNLQH; ILQEILEKLDKNLQHK;
LQEILEKLDKNLQHKK; QEILEKLDKNLQHKKI; EILEKLDKNLQHKKIA;
ILEKLDKNLQHKKIAK; LEKLDKNLQHKKIAKN; EKLDKNLQHKKIAKNF;
KLDKNLQHKKIAKNFI; LDKNLQHKKIAKNFIY; DKNLQHKKIAKNFIYD;
KNLQHKKIAKNFIYDI; NLQHKKIAKNFIYDIP; LQHKKIAKNFIYDIPI;
QHKKIAKNFIYDIPII; HKKIAKNFIYDIPIII; KKIAKNFIYDIPIIIQ;
KIAKNFIYDIPIIIQS; IAKNFIYDIPIIIQSR; AKNFIYDIPIIIQSRL;
KNFIYDIPIIIQSRLD; NFIYDIPIIIQSRLDI; FIYDIPIIIQSRLDII;
IYDIPIIIQSRLDIIS; YDIPIIIQSRLDIISK; DIPIIIQSRLDIISKV;
IPIIIQSRLDIISKVI; PIIIQSRLDIISKVIK; IIIQSRLDIISKVIKN;
IIQSRLDIISKVIKNP; IQSRLDIISKVIKNPI; QSRLDIISKVIKNPIK;
SRLDIISKVIKNPIKD; RLDIISKVIKNPIKDE; LDIISKVIKNPIKDEL;
DIISKVIKNPIKDELQ; IISKVIKNPIKDELQI; ISKVIKNPIKDELQIL;
SKVIKNPIKDELQILN; KVIKNPIKDELQILNQ; VIKNPIKDELQILNQK;
IKNPIKDELQILNQKE; KNPIKDELQILNQKEI; NPIKDELQILNQKEIE;
PIKDELQILNQKEIEE; IKDELQILNQKEIEEL; KDELQILNQKEIEELL;

| | |
|---|---|
| | DELQILNQKEIEELLM; ELQILNQKEIEELLMR; LQILNQKEIEELLMRI;<br>QILNQKEIEELLMRIE; ILNQKEIEELLMRIES; LNQKEIEELLMRIESE;<br>NQKEIEELLMRIESEL; QKEIEELLMRIESELK; KEIEELLMRIESELKI;<br>EIEELLMRIESELKIK; IEELLMRIESELKIKE; EELLMRIESELKIKEN;<br>ELLMRIESELKIKENF; LLMRIESELKIKENFK; LMRIESELKIKENFKK;<br>MRIESELKIKENFKKA; RIESELKIKENFKKAL; IESELKIKENFKKALN;<br>ESELKIKENFKKALNK; SELKIKENFKKALNKT; ELKIKENFKKALNKTI;<br>LKIKENFKKALNKTID; KIKENFKKALNKTIDA; IKENFKKALNKTIDAY;<br>KENFKKALNKTIDAYN; ENFKKALNKTIDAYNQ; NFKKALNKTIDAYNQD;<br>FKKALNKTIDAYNQDS; KKALNKTIDAYNQDSE; KALNKTIDAYNQDSEN;<br>ALNKTIDAYNQDSENI; LNKTIDAYNQDSENIK; NKTIDAYNQDSENIKT;<br>KTIDAYNQDSENIKTS; TIDAYNQDSENIKTSA; IDAYNQDSENIKTSAE;<br>DAYNQDSENIKTSAEQ; AYNQDSENIKTSAEQL; YNQDSENIKTSAEQLE;<br>NQDSENIKTSAEQLEK; QDSENIKTSAEQLEKH; DSENIKTSAEQLEKHI;<br>SENIKTSAEQLEKHIN; ENIKTSAEQLEKHINE; NIKTSAEQLEKHINEN;<br>IKTSAEQLEKHINENY; KTSAEQLEKHINENYK; TSAEQLEKHINENYKE;<br>SAEQLEKHINENYKEF; AEQLEKHINENYKEFN; EQLEKHINENYKEFNS;<br>QLEKHINENYKEFNSL; LEKHINENYKEFNSLK; EKHINENYKEFNSLKP;<br>KHINENYKEFNSLKPI; HINENYKEFNSLKPIY; |
| Seq 38 | 13 mers:<br>MRILVGVCIIALA; RILVGVCIIALAL; ILVGVCIIALALL;<br>LVGVCIIALALLG; VGVCIIALALLGC; GVCIIALALLGCY;<br>VCIIALALLGCYL; CIIALALLGCYLP; IIALALLGCYLPD;<br>IALALLGCYLPDN; ALALLGCYLPDNQ; LALLGCYLPDNQE;<br>ALLGCYLPDNQEQ; LLGCYLPDNQEQA; LGCYLPDNQEQAV;<br>GCYLPDNQEQAVQ; CYLPDNQEQAVQT; YLPDNQEQAVQTF;<br>LPDNQEQAVQTFF; PDNQEQAVQTFFE; DNQEQAVQTFFEN;<br>NQEQAVQTFFENS; QEQAVQTFFENSE; EQAVQTFFENSES;<br>QAVQTFFENSESS; AVQTFFENSESSD; VQTFFENSESSDM;<br>QTFFENSESSDMG; TFFENSESSDMGS; FFENSESSDMGSD;<br>FENSESSDMGSDE; ENSESSDMGSDEI; NSESSDMGSDEIV;<br>SESSDMGSDEIVT; ESSDMGSDEIVTE; SSDMGSDEIVTEG;<br>SDMGSDEIVTEGI; DMGSDEIVTEGIF; MGSDEIVTEGIFS;<br>GSDEIVTEGIFSS; SDEIVTEGIFSSL; DEIVTEGIFSSLK;<br>EIVTEGIFSSLKL; IVTEGIFSSLKLY; VTEGIFSSLKLYA;<br>TEGIFSSLKLYAS; EGIFSSLKLYASE; GIFSSLKLYASEH;<br>IFSSLKLYASEHR; FSSLKLYASEHRL; SSLKLYASEHRLL;<br>SLKLYASEHRLLV; LKLYASEHRLLVE; KLYASEHRLLVEI;<br>LYASEHRLLVEIK; YASEHRLLVEIKK; ASEHRLLVEIKKT;<br>SEHRLLVEIKKTL; EHRLLVEIKKTLI; HRLLVEIKKTLIS;<br>RLLVEIKKTLISL; LLVEIKKTLISLK; LVEIKKTLISLKD;<br>VEIKKTLISLKDP; EIKKTLISLKDPN; IKKTLISLKDPNY;<br>KKTLISLKDPNYR; KTLISLKDPNYRG; TLISLKDPNYRGV;<br>LISLKDPNYRGVV; ISLKDPNYRGVVL; SLKDPNYRGVVLP;<br>LKDPNYRGVVLPV; KDPNYRGVVLPVS; DPNYRGVVLPVSD;<br>PNYRGVVLPVSDY; NYRGVVLPVSDYN; YRGVVLPVSDYNE;<br>RGVVLPVSDYNEE; GVVLPVSDYNEEY; VVLPVSDYNEEYF;<br>VLPVSDYNEEYFN; LPVSDYNEEYFNK; PVSDYNEEYFNKF;<br>VSDYNEEYFNKFF; SDYNEEYFNKFFL; DYNEEYFNKFFLD; |

YNEEYFNKFFLDL;    NEEYFNKFFLDLG;    EEYFNKFFLDLGS;
EYFNKFFLDLGSE;    YFNKFFLDLGSEQ;    FNKFFLDLGSEQS;
NKFFLDLGSEQSK;    KFFLDLGSEQSKD;    FFLDLGSEQSKDL;
FLDLGSEQSKDLI;    LDLGSEQSKDLIK;    DLGSEQSKDLIKL;
LGSEQSKDLIKLF;    GSEQSKDLIKLFI;    SEQSKDLIKLFIM;
EQSKDLIKLFIMV;    QSKDLIKLFIMVK;    SKDLIKLFIMVKN;
KDLIKLFIMVKNE;    DLIKLFIMVKNEQ;    LIKLFIMVKNEQN;
IKLFIMVKNEQNN;    KLFIMVKNEQNNN;    LFIMVKNEQNNNK;
FIMVKNEQNNNKF;    IMVKNEQNNNKFM;    MVKNEQNNNKFMR;
VKNEQNNNKFMRI;    KNEQNNNKFMRIV;    NEQNNNKFMRIVR;
EQNNNKFMRIVRW;    QNNNKFMRIVRWL;    NNNKFMRIVRWLY;
NNKFMRIVRWLYS;    NKFMRIVRWLYSC;    KFMRIVRWLYSCI;
FMRIVRWLYSCIE;    MRIVRWLYSCIEE;    RIVRWLYSCIEEL;
IVRWLYSCIEELY;    VRWLYSCIEELYS;    RWLYSCIEELYSP;
WLYSCIEELYSPD;    LYSCIEELYSPDI;    YSCIEELYSPDIK;
SCIEELYSPDIKY;    CIEELYSPDIKYS;    IEELYSPDIKYSG;
EELYSPDIKYSGE;    ELYSPDIKYSGEE;    LYSPDIKYSGEEG;
YSPDIKYSGEEGS;    SPDIKYSGEEGSP;    PDIKYSGEEGSPE;
DIKYSGEEGSPEY;    IKYSGEEGSPEYY;    KYSGEEGSPEYYR;
YSGEEGSPEYYRN;    SGEEGSPEYYRNM;    GEEGSPEYYRNMP;
EEGSPEYYRNMPR;    EGSPEYYRNMPRP;    GSPEYYRNMPRPT;
SPEYYRNMPRPTA;    PEYYRNMPRPTAY;    EYYRNMPRPTAYQ;
YYRNMPRPTAYQQ;    YRNMPRPTAYQQY;    RNMPRPTAYQQYL;
NMPRPTAYQQYLK;    MPRPTAYQQYLKV;    PRPTAYQQYLKVK;
RPTAYQQYLKVKR;    PTAYQQYLKVKRY;    TAYQQYLKVKRYD;
AYQQYLKVKRYDY;    YQQYLKVKRYDYN;    QQYLKVKRYDYNR;
QYLKVKRYDYNRP;    YLKVKRYDYNRPV;    LKVKRYDYNRPVP;
KVKRYDYNRPVPI;    VKRYDYNRPVPIL;    KRYDYNRPVPILP;
RYDYNRPVPILPT;

14 mers:
MRILVGVCIIALAL;    RILVGVCIIALALL;    ILVGVCIIALALLG;
LVGVCIIALALLGC;    VGVCIIALALLGCY;    GVCIIALALLGCYL;
VCIIALALLGCYLP;    CIIALALLGCYLPD;    IIALALLGCYLPDN;
IALALLGCYLPDNQ;    ALALLGCYLPDNQE;    LALLGCYLPDNQEQ;
ALLGCYLPDNQEQA;    LLGCYLPDNQEQAV;    LGCYLPDNQEQAVQ;
GCYLPDNQEQAVQT;    CYLPDNQEQAVQTF;    YLPDNQEQAVQTFF;
LPDNQEQAVQTFFE;    PDNQEQAVQTFFEN;    DNQEQAVQTFFENS;
NQEQAVQTFFENSE;    QEQAVQTFFENSES;    EQAVQTFFENSESS;
QAVQTFFENSESSD;    AVQTFFENSESSDM;    VQTFFENSESSDMG;
QTFFENSESSDMGS;    TFFENSESSDMGSD;    FFENSESSDMGSDE;
FENSESSDMGSDEI;    ENSESSDMGSDEIV;    NSESSDMGSDEIVT;
SESSDMGSDEIVTE;    ESSDMGSDEIVTEG;    SSDMGSDEIVTEGI;
SDMGSDEIVTEGIF;    DMGSDEIVTEGIFS;    MGSDEIVTEGIFSS;
GSDEIVTEGIFSSL;    SDEIVTEGIFSSLK;    DEIVTEGIFSSLKL;
EIVTEGIFSSLKLY;    IVTEGIFSSLKLYA;    VTEGIFSSLKLYAS;
TEGIFSSLKLYASE;    EGIFSSLKLYASEH;    GIFSSLKLYASEHR;
IFSSLKLYASEHRL;    FSSLKLYASEHRLL;    SSLKLYASEHRLLV;
SLKLYASEHRLLVE;    LKLYASEHRLLVEI;    KLYASEHRLLVEIK;
LYASEHRLLVEIKK;    YASEHRLLVEIKKT;    ASEHRLLVEIKKTL;
SEHRLLVEIKKTLI;    EHRLLVEIKKTLIS;    HRLLVEIKKTLISL;

RLLVEIKKTLISLK; LLVEIKKTLISLKD; LVEIKKTLISLKDP;
VEIKKTLISLKDPN; EIKKTLISLKDPNY; IKKTLISLKDPNYR;
KKTLISLKDPNYRG; KTLISLKDPNYRGV; TLISLKDPNYRGVV;
LISLKDPNYRGVVL; ISLKDPNYRGVVLP; SLKDPNYRGVVLPV;
LKDPNYRGVVLPVS; KDPNYRGVVLPVSD; DPNYRGVVLPVSDY;
PNYRGVVLPVSDYN; NYRGVVLPVSDYNE; YRGVVLPVSDYNEE;
RGVVLPVSDYNEEY; GVVLPVSDYNEEYF; VVLPVSDYNEEYFN;
VLPVSDYNEEYFNK; LPVSDYNEEYFNKF; PVSDYNEEYFNKFF;
VSDYNEEYFNKFFL; SDYNEEYFNKFFLD; DYNEEYFNKFFLDL;
YNEEYFNKFFLDLG; NEEYFNKFFLDLGS; EEYFNKFFLDLGSE;
EYFNKFFLDLGSEQ; YFNKFFLDLGSEQS; FNKFFLDLGSEQSK;
NKFFLDLGSEQSKD; KFFLDLGSEQSKDL; FFLDLGSEQSKDLI;
FLDLGSEQSKDLIK; LDLGSEQSKDLIKL; DLGSEQSKDLIKLF;
LGSEQSKDLIKLFI; GSEQSKDLIKLFIM; SEQSKDLIKLFIMV;
EQSKDLIKLFIMVK; QSKDLIKLFIMVKN; SKDLIKLFIMVKNE;
KDLIKLFIMVKNEQ; DLIKLFIMVKNEQN; LIKLFIMVKNEQNN;
IKLFIMVKNEQNNN; KLFIMVKNEQNNNK; LFIMVKNEQNNNKF;
FIMVKNEQNNNKFM; IMVKNEQNNNKFMR; MVKNEQNNNKFMRI;
VKNEQNNNKFMRIV; KNEQNNNKFMRIVR; NEQNNNKFMRIVRW;
EQNNNKFMRIVRWL; QNNNKFMRIVRWLY; NNNKFMRIVRWLYS;
NNKFMRIVRWLYSC; NKFMRIVRWLYSCI; KFMRIVRWLYSCIE;
FMRIVRWLYSCIEE; MRIVRWLYSCIEEL; RIVRWLYSCIEELY;
IVRWLYSCIEELYS; VRWLYSCIEELYSP; RWLYSCIEELYSPD;
WLYSCIEELYSPDI; LYSCIEELYSPDIK; YSCIEELYSPDIKY;
SCIEELYSPDIKYS; CIEELYSPDIKYSG; IEELYSPDIKYSGE;
EELYSPDIKYSGEE; ELYSPDIKYSGEEG; LYSPDIKYSGEEGS;
YSPDIKYSGEEGSP; SPDIKYSGEEGSPE; PDIKYSGEEGSPEY;
DIKYSGEEGSPEYY; IKYSGEEGSPEYYR; KYSGEEGSPEYYRN;
YSGEEGSPEYYRNM; SGEEGSPEYYRNMP; GEEGSPEYYRNMPR;
EEGSPEYYRNMPRP; EGSPEYYRNMPRPT; GSPEYYRNMPRPTA;
SPEYYRNMPRPTAY; PEYYRNMPRPTAYQ; EYYRNMPRPTAYQQ;
YYRNMPRPTAYQQY; YRNMPRPTAYQQYL; RNMPRPTAYQQYLK;
NMPRPTAYQQYLKV; MPRPTAYQQYLKVK; PRPTAYQQYLKVKR;
RPTAYQQYLKVKRY; PTAYQQYLKVKRYD; TAYQQYLKVKRYDY;
AYQQYLKVKRYDYN; YQQYLKVKRYDYNR; QQYLKVKRYDYNRP;
QYLKVKRYDYNRPV; YLKVKRYDYNRPVP; LKVKRYDYNRPVPI;
KVKRYDYNRPVPIL; VKRYDYNRPVPILP; KRYDYNRPVPILPT;

15 mers:
MRILVGVCIIALALL; RILVGVCIIALALLG; ILVGVCIIALALLGC;
LVGVCIIALALLGCY; VGVCIIALALLGCYL; GVCIIALALLGCYLP;
VCIIALALLGCYLPD; CIIALALLGCYLPDN; IIALALLGCYLPDNQ;
IALALLGCYLPDNQE; ALALLGCYLPDNQEQ; LALLGCYLPDNQEQA;
ALLGCYLPDNQEQAV; LLGCYLPDNQEQAVQ; LGCYLPDNQEQAVQT;
GCYLPDNQEQAVQTF; CYLPDNQEQAVQTFF; YLPDNQEQAVQTFFE;
LPDNQEQAVQTFFEN; PDNQEQAVQTFFENS; DNQEQAVQTFFENSE;
NQEQAVQTFFENSES; QEQAVQTFFENSESS; EQAVQTFFENSESSD;
QAVQTFFENSESSDM; AVQTFFENSESSDMG; VQTFFENSESSDMGS;
QTFFENSESSDMGSD; TFFENSESSDMGSDE; FFENSESSDMGSDEI;
FENSESSDMGSDEIV; ENSESSDMGSDEIVT; NSESSDMGSDEIVTE;
SESSDMGSDEIVTEG; ESSDMGSDEIVTEGI; SSDMGSDEIVTEGIF;

SDMGSDEIVTEGIFS;  DMGSDEIVTEGIFSS;  MGSDEIVTEGIFSSL;
GSDEIVTEGIFSSLK;  SDEIVTEGIFSSLKL;  DEIVTEGIFSSLKLY;
EIVTEGIFSSLKLYA;  IVTEGIFSSLKLYAS;  VTEGIFSSLKLYASE;
TEGIFSSLKLYASEH;  EGIFSSLKLYASEHR;  GIFSSLKLYASEHRL;
IFSSLKLYASEHRLL;  FSSLKLYASEHRLLV;  SSLKLYASEHRLLVE;
SLKLYASEHRLLVEI;  LKLYASEHRLLVEIK;  KLYASEHRLLVEIKK;
LYASEHRLLVEIKKT;  YASEHRLLVEIKKTL;  ASEHRLLVEIKKTLI;
SEHRLLVEIKKTLIS;  EHRLLVEIKKTLISL;  HRLLVEIKKTLISLK;
RLLVEIKKTLISLKD;  LLVEIKKTLISLKDP;  LVEIKKTLISLKDPN;
VEIKKTLISLKDPNY;  EIKKTLISLKDPNYR;  IKKTLISLKDPNYRG;
KKTLISLKDPNYRGV;  KTLISLKDPNYRGVV;  TLISLKDPNYRGVVL;
LISLKDPNYRGVVLP;  ISLKDPNYRGVVLPV;  SLKDPNYRGVVLPVS;
LKDPNYRGVVLPVSD;  KDPNYRGVVLPVSDY;  DPNYRGVVLPVSDYN;
PNYRGVVLPVSDYNE;  NYRGVVLPVSDYNEE;  YRGVVLPVSDYNEEY;
RGVVLPVSDYNEEYF;  GVVLPVSDYNEEYFN;  VVLPVSDYNEEYFNK;
VLPVSDYNEEYFNKF;  LPVSDYNEEYFNKFF;  PVSDYNEEYFNKFFL;
VSDYNEEYFNKFFLD;  SDYNEEYFNKFFLDL;  DYNEEYFNKFFLDLG;
YNEEYFNKFFLDLGS;  NEEYFNKFFLDLGSE;  EEYFNKFFLDLGSEQ;
EYFNKFFLDLGSEQS;  YFNKFFLDLGSEQSK;  FNKFFLDLGSEQSKD;
NKFFLDLGSEQSKDL;  KFFLDLGSEQSKDLI;  FFLDLGSEQSKDLIK;
FLDLGSEQSKDLIKL;  LDLGSEQSKDLIKLF;  DLGSEQSKDLIKLFI;
LGSEQSKDLIKLFIM;  GSEQSKDLIKLFIMV;  SEQSKDLIKLFIMVK;
EQSKDLIKLFIMVKN;  QSKDLIKLFIMVKNE;  SKDLIKLFIMVKNEQ;
KDLIKLFIMVKNEQN;  DLIKLFIMVKNEQNN;  LIKLFIMVKNEQNNN;
IKLFIMVKNEQNNNK;  KLFIMVKNEQNNNKF;  LFIMVKNEQNNNKFM;
FIMVKNEQNNNKFMR;  IMVKNEQNNNKFMRI;  MVKNEQNNNKFMRIV;
VKNEQNNNKFMRIVR;  KNEQNNNKFMRIVRW;  NEQNNNKFMRIVRWL;
EQNNNKFMRIVRWLY;  QNNNKFMRIVRWLYS;  NNNKFMRIVRWLYSC;
NNKFMRIVRWLYSCI;  NKFMRIVRWLYSCIE;  KFMRIVRWLYSCIEE;
FMRIVRWLYSCIEEL;  MRIVRWLYSCIEELY;  RIVRWLYSCIEELYS;
IVRWLYSCIEELYSP;  VRWLYSCIEELYSPD;  RWLYSCIEELYSPDI;
WLYSCIEELYSPDIK;  LYSCIEELYSPDIKY;  YSCIEELYSPDIKYS;
SCIEELYSPDIKYSG;  CIEELYSPDIKYSGE;  IEELYSPDIKYSGEE;
EELYSPDIKYSGEEG;  ELYSPDIKYSGEEGS;  LYSPDIKYSGEEGSP;
YSPDIKYSGEEGSPE;  SPDIKYSGEEGSPEY;  PDIKYSGEEGSPEYY;
DIKYSGEEGSPEYYR;  IKYSGEEGSPEYYRN;  KYSGEEGSPEYYRNM;
YSGEEGSPEYYRNMP;  SGEEGSPEYYRNMPR;  GEEGSPEYYRNMPRP;
EEGSPEYYRNMPRPT;  EGSPEYYRNMPRPTA;  GSPEYYRNMPRPTAY;
SPEYYRNMPRPTAYQ;  PEYYRNMPRPTAYQQ;  EYYRNMPRPTAYQQY;
YYRNMPRPTAYQQYL;  YRNMPRPTAYQQYLK;  RNMPRPTAYQQYLKV;
NMPRPTAYQQYLKVK;  MPRPTAYQQYLKVKR;  PRPTAYQQYLKVKRY;
RPTAYQQYLKVKRYD;  PTAYQQYLKVKRYDY;  TAYQQYLKVKRYDYN;
AYQQYLKVKRYDYNR;  YQQYLKVKRYDYNRP;  QQYLKVKRYDYNRPV;
QYLKVKRYDYNRPVP;  YLKVKRYDYNRPVPI;  LKVKRYDYNRPVPIL;
KVKRYDYNRPVPILP;  VKRYDYNRPVPILPT;

16 mers:
MRILVGVCIIALALLG;  RILVGVCIIALALLGC;  ILVGVCIIALALLGCY;
LVGVCIIALALLGCYL;  VGVCIIALALLGCYLP;  GVCIIALALLGCYLPD;
VCIIALALLGCYLPDN;  CIIALALLGCYLPDNQ;  IIALALLGCYLPDNQE;
IALALLGCYLPDNQEQ;  ALALLGCYLPDNQEQA;  LALLGCYLPDNQEQAV;

| | | |
|---|---|---|
| ALLGCYLPDNQEQAVQ; | LLGCYLPDNQEQAVQT; | LGCYLPDNQEQAVQTF; |
| GCYLPDNQEQAVQTFF; | CYLPDNQEQAVQTFFE; | YLPDNQEQAVQTFFEN; |
| LPDNQEQAVQTFFENS; | PDNQEQAVQTFFENSE; | DNQEQAVQTFFENSES; |
| NQEQAVQTFFENSESS; | QEQAVQTFFENSESSD; | EQAVQTFFENSESSDM; |
| QAVQTFFENSESSDMG; | AVQTFFENSESSDMGS; | VQTFFENSESSDMGSD; |
| QTFFENSESSDMGSDE; | TFFENSESSDMGSDEI; | FFENSESSDMGSDEIV; |
| FENSESSDMGSDEIVT; | ENSESSDMGSDEIVTE; | NSESSDMGSDEIVTEG; |
| SESSDMGSDEIVTEGI; | ESSDMGSDEIVTEGIF; | SSDMGSDEIVTEGIFS; |
| SDMGSDEIVTEGIFSS; | DMGSDEIVTEGIFSSL; | MGSDEIVTEGIFSSLK; |
| GSDEIVTEGIFSSLKL; | SDEIVTEGIFSSLKLY; | DEIVTEGIFSSLKLYA; |
| EIVTEGIFSSLKLYAS; | IVTEGIFSSLKLYASE; | VTEGIFSSLKLYASEH; |
| TEGIFSSLKLYASEHR; | EGIFSSLKLYASEHRL; | GIFSSLKLYASEHRLL; |
| IFSSLKLYASEHRLLV; | FSSLKLYASEHRLLVE; | SSLKLYASEHRLLVEI; |
| SLKLYASEHRLLVEIK; | LKLYASEHRLLVEIKK; | KLYASEHRLLVEIKKT; |
| LYASEHRLLVEIKKTL; | YASEHRLLVEIKKTLI; | ASEHRLLVEIKKTLIS; |
| SEHRLLVEIKKTLISL; | EHRLLVEIKKTLISLK; | HRLLVEIKKTLISLKD; |
| RLLVEIKKTLISLKDP; | LLVEIKKTLISLKDPN; | LVEIKKTLISLKDPNY; |
| VEIKKTLISLKDPNYR; | EIKKTLISLKDPNYRG; | IKKTLISLKDPNYRGV; |
| KKTLISLKDPNYRGVV; | KTLISLKDPNYRGVVL; | TLISLKDPNYRGVVLP; |
| LISLKDPNYRGVVLPV; | ISLKDPNYRGVVLPVS; | SLKDPNYRGVVLPVSD; |
| LKDPNYRGVVLPVSDY; | KDPNYRGVVLPVSDYN; | DPNYRGVVLPVSDYNE; |
| PNYRGVVLPVSDYNEE; | NYRGVVLPVSDYNEEY; | YRGVVLPVSDYNEEYF; |
| RGVVLPVSDYNEEYFN; | GVVLPVSDYNEEYFNK; | VVLPVSDYNEEYFNKF; |
| VLPVSDYNEEYFNKFF; | LPVSDYNEEYFNKFFL; | PVSDYNEEYFNKFFLD; |
| VSDYNEEYFNKFFLDL; | SDYNEEYFNKFFLDLG; | DYNEEYFNKFFLDLGS; |
| YNEEYFNKFFLDLGSE; | NEEYFNKFFLDLGSEQ; | EEYFNKFFLDLGSEQS; |
| EYFNKFFLDLGSEQSK; | YFNKFFLDLGSEQSKD; | FNKFFLDLGSEQSKDL; |
| NKFFLDLGSEQSKDLI; | KFFLDLGSEQSKDLIK; | FFLDLGSEQSKDLIKL; |
| FLDLGSEQSKDLIKLF; | LDLGSEQSKDLIKLFI; | DLGSEQSKDLIKLFIM; |
| LGSEQSKDLIKLFIMV; | GSEQSKDLIKLFIMVK; | SEQSKDLIKLFIMVKN; |
| EQSKDLIKLFIMVKNE; | QSKDLIKLFIMVKNEQ; | SKDLIKLFIMVKNEQN; |
| KDLIKLFIMVKNEQNN; | DLIKLFIMVKNEQNNN; | LIKLFIMVKNEQNNNK; |
| IKLFIMVKNEQNNNKF; | KLFIMVKNEQNNNKFM; | LFIMVKNEQNNNKFMR; |
| FIMVKNEQNNNKFMRI; | IMVKNEQNNNKFMRIV; | MVKNEQNNNKFMRIVR; |
| VKNEQNNNKFMRIVRW; | KNEQNNNKFMRIVRWL; | NEQNNNKFMRIVRWLY; |
| EQNNNKFMRIVRWLYS; | QNNNKFMRIVRWLYSC; | NNNKFMRIVRWLYSCI; |
| NNKFMRIVRWLYSCIE; | NKFMRIVRWLYSCIEE; | KFMRIVRWLYSCIEEL; |
| FMRIVRWLYSCIEELY; | MRIVRWLYSCIEELYS; | RIVRWLYSCIEELYSP; |
| IVRWLYSCIEELYSPD; | VRWLYSCIEELYSPDI; | RWLYSCIEELYSPDIK; |
| WLYSCIEELYSPDIKY; | LYSCIEELYSPDIKYS; | YSCIEELYSPDIKYSG; |
| SCIEELYSPDIKYSGE; | CIEELYSPDIKYSGEE; | IEELYSPDIKYSGEEG; |
| EELYSPDIKYSGEEGS; | ELYSPDIKYSGEEGSP; | LYSPDIKYSGEEGSPE; |
| YSPDIKYSGEEGSPEY; | SPDIKYSGEEGSPEYY; | PDIKYSGEEGSPEYYR; |
| DIKYSGEEGSPEYYRN; | IKYSGEEGSPEYYRNM; | KYSGEEGSPEYYRNMP; |
| YSGEEGSPEYYRNMPR; | SGEEGSPEYYRNMPRP; | GEEGSPEYYRNMPRPT; |
| EEGSPEYYRNMPRPTA; | EGSPEYYRNMPRPTAY; | GSPEYYRNMPRPTAYQ; |
| SPEYYRNMPRPTAYQQ; | PEYYRNMPRPTAYQQY; | EYYRNMPRPTAYQQYL; |
| YYRNMPRPTAYQQYLK; | YRNMPRPTAYQQYLKV; | RNMPRPTAYQQYLKVK; |
| NMPRPTAYQQYLKVKR; | MPRPTAYQQYLKVKRY; | PRPTAYQQYLKVKRYD; |
| RPTAYQQYLKVKRYDY; | PTAYQQYLKVKRYDYN; | TAYQQYLKVKRYDYNR; |
| AYQQYLKVKRYDYNRP; | YQQYLKVKRYDYNRPV; | QQYLKVKRYDYNRPVP; |

| | |
|---|---|
| | QYLKVKRYDYNRPVPI; YLKVKRYDYNRPVPIL; LKVKRYDYNRPVPILP; KVKRYDYNRPVPILPT; |
| Seq 39 | 13 mers:<br>MRNKNIFKLFFAS; RNKNIFKLFFASM; NKNIFKLFFASML;<br>KNIFKLFFASMLF; NIFKLFFASMLFV; IFKLFFASMLFVM;<br>FKLFFASMLFVMA; KLFFASMLFVMAC; LFFASMLFVMACK;<br>FFASMLFVMACKA; FASMLFVMACKAY; ASMLFVMACKAYV;<br>SMLFVMACKAYVE; MLFVMACKAYVEE; LFVMACKAYVEEK;<br>FVMACKAYVEEKK; VMACKAYVEEKKE; MACKAYVEEKKEI;<br>ACKAYVEEKKEID; CKAYVEEKKEIDS; KAYVEEKKEIDSL;<br>AYVEEKKEIDSLM; YVEEKKEIDSLME; VEEKKEIDSLMED;<br>EEKKEIDSLMEDV; EKKEIDSLMEDVL; KKEIDSLMEDVLA;<br>KEIDSLMEDVLAL; EIDSLMEDVLALV; IDSLMEDVLALVN;<br>DSLMEDVLALVND; SLMEDVLALVNDS; LMEDVLALVNDSS;<br>MEDVLALVNDSSG; EDVLALVNDSSGG; DVLALVNDSSGGK;<br>VLALVNDSSGGKF; LALVNDSSGGKFK; ALVNDSSGGKFKD;<br>LVNDSSGGKFKDY; VNDSSGGKFKDYK; NDSSGGKFKDYKD;<br>DSSGGKFKDYKDK; SSGGKFKDYKDKI; SGGKFKDYKDKIN;<br>GGKFKDYKDKINE; GKFKDYKDKINEL; KFKDYKDKINELK;<br>FKDYKDKINELKE; KDYKDKINELKEN; DYKDKINELKENL;<br>YKDKINELKENLK; KDKINELKENLKD; DKINELKENLKDI;<br>KINELKENLKDIG; INELKENLKDIGN; NELKENLKDIGNA;<br>ELKENLKDIGNAE; LKENLKDIGNAEL; KENLKDIGNAELK;<br>ENLKDIGNAELKE; NLKDIGNAELKEK; LKDIGNAELKEKL;<br>KDIGNAELKEKLL; DIGNAELKEKLLN; IGNAELKEKLLNL;<br>GNAELKEKLLNLQ; NAELKEKLLNLQN; AELKEKLLNLQNS;<br>ELKEKLLNLQNSF; LKEKLLNLQNSFQ; KEKLLNLQNSFQD;<br>EKLLNLQNSFQDK; KLLNLQNSFQDKL; LLNLQNSFQDKLA;<br>LNLQNSFQDKLAA; NLQNSFQDKLAAK; LQNSFQDKLAAKL;<br>QNSFQDKLAAKLA; NSFQDKLAAKLAA; SFQDKLAAKLAAL;<br>FQDKLAAKLAALK; QDKLAAKLAALKA; DKLAAKLAALKAA;<br>KLAAKLAALKAAK; LAAKLAALKAAKN; AAKLAALKAAKNT;<br>AKLAALKAAKNTI; KLAALKAAKNTIE; LAALKAAKNTIEN;<br>AALKAAKNTIENI; ALKAAKNTIENIT; LKAAKNTIENITD;<br>KAAKNTIENITDK; AAKNTIENITDKD; AKNTIENITDKDQ;<br>KNTIENITDKDQD; NTIENITDKDQDI; TIENITDKDQDIS;<br>IENITDKDQDISK; ENITDKDQDISKR; NITDKDQDISKRK;<br>ITDKDQDISKRKI; TDKDQDISKRKIW; DKDQDISKRKIWS;<br>KDQDISKRKIWSE; DQDISKRKIWSEA; QDISKRKIWSEAK;<br>DISKRKIWSEAKL; ISKRKIWSEAKLV; SKRKIWSEAKLVG;<br>KRKIWSEAKLVGV; RKIWSEAKLVGVT; KIWSEAKLVGVTV;<br>IWSEAKLVGVTVP; WSEAKLVGVTVPL; SEAKLVGVTVPLL;<br>EAKLVGVTVPLLG; AKLVGVTVPLLGS; KLVGVTVPLLGSN;<br>LVGVTVPLLGSNT; VGVTVPLLGSNTS; GVTVPLLGSNTSG;<br>VTVPLLGSNTSGN; TVPLLGSNTSGNG; VPLLGSNTSGNGD;<br>PLLGSNTSGNGDK; LLGSNTSGNGDKM; LGSNTSGNGDKMS;<br>GSNTSGNGDKMSK; SNTSGNGDKMSKN; NTSGNGDKMSKNA;<br>TSGNGDKMSKNAV; SGNGDKMSKNAVE; GNGDKMSKNAVEQ;<br>NGDKMSKNAVEQI; GDKMSKNAVEQID; DKMSKNAVEQIDK; |

KMSKNAVEQIDKV; MSKNAVEQIDKVI; SKNAVEQIDKVIK;
KNAVEQIDKVIKF; NAVEQIDKVIKFL; AVEQIDKVIKFLE;
VEQIDKVIKFLEE; EQIDKVIKFLEEG; QIDKVIKFLEEGT;
IDKVIKFLEEGTN;

14 mers:
MRNKNIFKLFFASM; RNKNIFKLFFASML; NKNIFKLFFASMLF;
KNIFKLFFASMLFV; NIFKLFFASMLFVM; IFKLFFASMLFVMA;
FKLFFASMLFVMAC; KLFFASMLFVMACK; LFFASMLFVMACKA;
FFASMLFVMACKAY; FASMLFVMACKAYV; ASMLFVMACKAYVE;
SMLFVMACKAYVEE; MLFVMACKAYVEEK; LFVMACKAYVEEKK;
FVMACKAYVEEKKE; VMACKAYVEEKKEI; MACKAYVEEKKEID;
ACKAYVEEKKEIDS; CKAYVEEKKEIDSL; KAYVEEKKEIDSLM;
AYVEEKKEIDSLME; YVEEKKEIDSLMED; VEEKKEIDSLMEDV;
EEKKEIDSLMEDVL; EKKEIDSLMEDVLA; KKEIDSLMEDVLAL;
KEIDSLMEDVLALV; EIDSLMEDVLALVN; IDSLMEDVLALVND;
DSLMEDVLALVNDS; SLMEDVLALVNDSS; LMEDVLALVNDSSG;
MEDVLALVNDSSGG; EDVLALVNDSSGGK; DVLALVNDSSGGKF;
VLALVNDSSGGKFK; LALVNDSSGGKFKD; ALVNDSSGGKFKDY;
LVNDSSGGKFKDYK; VNDSSGGKFKDYKD; NDSSGGKFKDYKDK;
DSSGGKFKDYKDKI; SSGGKFKDYKDKIN; SGGKFKDYKDKINE;
GGKFKDYKDKINEL; GKFKDYKDKINELK; KFKDYKDKINELKE;
FKDYKDKINELKEN; KDYKDKINELKENL; DYKDKINELKENLK;
YKDKINELKENLKD; KDKINELKENLKDI; DKINELKENLKDIG;
KINELKENLKDIGN; INELKENLKDIGNA; NELKENLKDIGNAE;
ELKENLKDIGNAEL; LKENLKDIGNAELK; KENLKDIGNAELKE;
ENLKDIGNAELKEK; NLKDIGNAELKEKL; LKDIGNAELKEKLL;
KDIGNAELKEKLLN; DIGNAELKEKLLNL; IGNAELKEKLLNLQ;
GNAELKEKLLNLQN; NAELKEKLLNLQNS; AELKEKLLNLQNSF;
ELKEKLLNLQNSFQ; LKEKLLNLQNSFQD; KEKLLNLQNSFQDK;
EKLLNLQNSFQDKL; KLLNLQNSFQDKLA; LLNLQNSFQDKLAA;
LNLQNSFQDKLAAK; NLQNSFQDKLAAKL; LQNSFQDKLAAKLA;
QNSFQDKLAAKLAA; NSFQDKLAAKLAAL; SFQDKLAAKLAALK;
FQDKLAAKLAALKA; QDKLAAKLAALKAA; DKLAAKLAALKAAK;
KLAAKLAALKAAKN; LAAKLAALKAAKNT; AAKLAALKAAKNTI;
AKLAALKAAKNTIE; KLAALKAAKNTIEN; LAALKAAKNTIENI;
AALKAAKNTIENIT; ALKAAKNTIENITD; LKAAKNTIENITDK;
KAAKNTIENITDKD; AAKNTIENITDKDQ; AKNTIENITDKDQD;
KNTIENITDKDQDI; NTIENITDKDQDIS; TIENITDKDQDISK;
IENITDKDQDISKR; ENITDKDQDISKRK; NITDKDQDISKRKI;
ITDKDQDISKRKIW; TDKDQDISKRKIWS; DKDQDISKRKIWSE;
KDQDISKRKIWSEA; DQDISKRKIWSEAK; QDISKRKIWSEAKL;
DISKRKIWSEAKLV; ISKRKIWSEAKLVG; SKRKIWSEAKLVGV;
KRKIWSEAKLVGVT; RKIWSEAKLVGVTV; KIWSEAKLVGVTVP;
IWSEAKLVGVTVPL; WSEAKLVGVTVPLL; SEAKLVGVTVPLLG;
EAKLVGVTVPLLGS; AKLVGVTVPLLGSN; KLVGVTVPLLGSNT;
LVGVTVPLLGSNTS; VGVTVPLLGSNTSG; GVTVPLLGSNTSGN;
VTVPLLGSNTSGNG; TVPLLGSNTSGNGD; VPLLGSNTSGNGDK;
PLLGSNTSGNGDKM; LLGSNTSGNGDKMS; LGSNTSGNGDKMSK;
GSNTSGNGDKMSKN; SNTSGNGDKMSKNA; NTSGNGDKMSKNAV;
TSGNGDKMSKNAVE; SGNGDKMSKNAVEQ; GNGDKMSKNAVEQI;

NGDKMSKNAVEQID; GDKMSKNAVEQIDK; DKMSKNAVEQIDKV;
KMSKNAVEQIDKVI; MSKNAVEQIDKVIK; SKNAVEQIDKVIKF;
KNAVEQIDKVIKFL; NAVEQIDKVIKFLE; AVEQIDKVIKFLEE;
VEQIDKVIKFLEEG; EQIDKVIKFLEEGT; QIDKVIKFLEEGTN;

15 mers:
MRNKNIFKLFFASML; RNKNIFKLFFASMLF; NKNIFKLFFASMLFV;
KNIFKLFFASMLFVM; NIFKLFFASMLFVMA; IFKLFFASMLFVMAC;
FKLFFASMLFVMACK; KLFFASMLFVMACKA; LFFASMLFVMACKAY;
FFASMLFVMACKAYV; FASMLFVMACKAYVE; ASMLFVMACKAYVEE;
SMLFVMACKAYVEEK; MLFVMACKAYVEEKK; LFVMACKAYVEEKKE;
FVMACKAYVEEKKEI; VMACKAYVEEKKEID; MACKAYVEEKKEIDS;
ACKAYVEEKKEIDSL; CKAYVEEKKEIDSLM; KAYVEEKKEIDSLME;
AYVEEKKEIDSLMED; YVEEKKEIDSLMEDV; VEEKKEIDSLMEDVL;
EEKKEIDSLMEDVLA; EKKEIDSLMEDVLAL; KKEIDSLMEDVLALV;
KEIDSLMEDVLALVN; EIDSLMEDVLALVND; IDSLMEDVLALVNDS;
DSLMEDVLALVNDSS; SLMEDVLALVNDSSG; LMEDVLALVNDSSGG;
MEDVLALVNDSSGGK; EDVLALVNDSSGGKF; DVLALVNDSSGGKFK;
VLALVNDSSGGKFKD; LALVNDSSGGKFKDY; ALVNDSSGGKFKDYK;
LVNDSSGGKFKDYKD; VNDSSGGKFKDYKDK; NDSSGGKFKDYKDKI;
DSSGGKFKDYKDKIN; SSGGKFKDYKDKINE; SGGKFKDYKDKINEL;
GGKFKDYKDKINELK; GKFKDYKDKINELKE; KFKDYKDKINELKEN;
FKDYKDKINELKENL; KDYKDKINELKENLK; DYKDKINELKENLKD;
YKDKINELKENLKDI; KDKINELKENLKDIG; DKINELKENLKDIGN;
KINELKENLKDIGNA; INELKENLKDIGNAE; NELKENLKDIGNAEL;
ELKENLKDIGNAELK; LKENLKDIGNAELKE; KENLKDIGNAELKEK;
ENLKDIGNAELKEKL; NLKDIGNAELKEKLL; LKDIGNAELKEKLLN;
KDIGNAELKEKLLNL; DIGNAELKEKLLNLQ; IGNAELKEKLLNLQN;
GNAELKEKLLNLQNS; NAELKEKLLNLQNSF; AELKEKLLNLQNSFQ;
ELKEKLLNLQNSFQD; LKEKLLNLQNSFQDK; KEKLLNLQNSFQDKL;
EKLLNLQNSFQDKLA; KLLNLQNSFQDKLAA; LLNLQNSFQDKLAAK;
LNLQNSFQDKLAAKL; NLQNSFQDKLAAKLA; LQNSFQDKLAAKLAA;
QNSFQDKLAAKLAAL; NSFQDKLAAKLAALK; SFQDKLAAKLAALKA;
FQDKLAAKLAALKAA; QDKLAAKLAALKAAK; DKLAAKLAALKAAKN;
KLAAKLAALKAAKNT; LAAKLAALKAAKNTI; AAKLAALKAAKNTIE;
AKLAALKAAKNTIEN; KLAALKAAKNTIENI; LAALKAAKNTIENIT;
AALKAAKNTIENITD; ALKAAKNTIENITDK; LKAAKNTIENITDKD;
KAAKNTIENITDKDQ; AAKNTIENITDKDQD; AKNTIENITDKDQDI;
KNTIENITDKDQDIS; NTIENITDKDQDISK; TIENITDKDQDISKR;
IENITDKDQDISKRK; ENITDKDQDISKRKI; NITDKDQDISKRKIW;
ITDKDQDISKRKIWS; TDKDQDISKRKIWSE; DKDQDISKRKIWSEA;
KDQDISKRKIWSEAK; DQDISKRKIWSEAKL; QDISKRKIWSEAKLV;
DISKRKIWSEAKLVG; ISKRKIWSEAKLVGV; SKRKIWSEAKLVGVT;
KRKIWSEAKLVGVTV; RKIWSEAKLVGVTVP; KIWSEAKLVGVTVPL;
IWSEAKLVGVTVPLL; WSEAKLVGVTVPLLG; SEAKLVGVTVPLLGS;
EAKLVGVTVPLLGSN; AKLVGVTVPLLGSNT; KLVGVTVPLLGSNTS;
LVGVTVPLLGSNTSG; VGVTVPLLGSNTSGN; GVTVPLLGSNTSGNG;
VTVPLLGSNTSGNGD; TVPLLGSNTSGNGDK; VPLLGSNTSGNGDKM;
PLLGSNTSGNGDKMS; LLGSNTSGNGDKMSK; LGSNTSGNGDKMSKN;
GSNTSGNGDKMSKNA; SNTSGNGDKMSKNAV; NTSGNGDKMSKNAVE;
TSGNGDKMSKNAVEQ; SGNGDKMSKNAVEQI; GNGDKMSKNAVEQID;

NGDKMSKNAVEQIDK; GDKMSKNAVEQIDKV; DKMSKNAVEQIDKVI;
KMSKNAVEQIDKVIK; MSKNAVEQIDKVIKF; SKNAVEQIDKVIKFL;
KNAVEQIDKVIKFLE; NAVEQIDKVIKFLEE; AVEQIDKVIKFLEEG;
VEQIDKVIKFLEEGT; EQIDKVIKFLEEGTN;

16 mers:
MRNKNIFKLFFASMLF; RNKNIFKLFFASMLFV; NKNIFKLFFASMLFVM;
KNIFKLFFASMLFVMA; NIFKLFFASMLFVMAC; IFKLFFASMLFVMACK;
FKLFFASMLFVMACKA; KLFFASMLFVMACKAY; LFFASMLFVMACKAYV;
FFASMLFVMACKAYVE; FASMLFVMACKAYVEE; ASMLFVMACKAYVEEK;
SMLFVMACKAYVEEKK; MLFVMACKAYVEEKKE; LFVMACKAYVEEKKEI;
FVMACKAYVEEKKEID; VMACKAYVEEKKEIDS; MACKAYVEEKKEIDSL;
ACKAYVEEKKEIDSLM; CKAYVEEKKEIDSLME; KAYVEEKKEIDSLMED;
AYVEEKKEIDSLMEDV; YVEEKKEIDSLMEDVL; VEEKKEIDSLMEDVLA;
EEKKEIDSLMEDVLAL; EKKEIDSLMEDVLALV; KKEIDSLMEDVLALVN;
KEIDSLMEDVLALVND; EIDSLMEDVLALVNDS; IDSLMEDVLALVNDSS;
DSLMEDVLALVNDSSG; SLMEDVLALVNDSSGG; LMEDVLALVNDSSGGK;
MEDVLALVNDSSGGKF; EDVLALVNDSSGGKFK; DVLALVNDSSGGKFKD;
VLALVNDSSGGKFKDY; LALVNDSSGGKFKDYK; ALVNDSSGGKFKDYKD;
LVNDSSGGKFKDYKDK; VNDSSGGKFKDYKDKI; NDSSGGKFKDYKDKIN;
DSSGGKFKDYKDKINE; SSGGKFKDYKDKINEL; SGGKFKDYKDKINELK;
GGKFKDYKDKINELKE; GKFKDYKDKINELKEN; KFKDYKDKINELKENL;
FKDYKDKINELKENLK; KDYKDKINELKENLKD; DYKDKINELKENLKDI;
YKDKINELKENLKDIG; KDKINELKENLKDIGN; DKINELKENLKDIGNA;
KINELKENLKDIGNAE; INELKENLKDIGNAEL; NELKENLKDIGNAELK;
ELKENLKDIGNAELKE; LKENLKDIGNAELKEK; KENLKDIGNAELKEKL;
ENLKDIGNAELKEKLL; NLKDIGNAELKEKLLN; LKDIGNAELKEKLLNL;
KDIGNAELKEKLLNLQ; DIGNAELKEKLLNLQN; IGNAELKEKLLNLQNS;
GNAELKEKLLNLQNSF; NAELKEKLLNLQNSFQ; AELKEKLLNLQNSFQD;
ELKEKLLNLQNSFQDK; LKEKLLNLQNSFQDKL; KEKLLNLQNSFQDKLA;
EKLLNLQNSFQDKLAA; KLLNLQNSFQDKLAAK; LLNLQNSFQDKLAAKL;
LNLQNSFQDKLAAKLA; NLQNSFQDKLAAKLAA; LQNSFQDKLAAKLAAL;
QNSFQDKLAAKLAALK; NSFQDKLAAKLAALKA; SFQDKLAAKLAALKAA;
FQDKLAAKLAALKAAK; QDKLAAKLAALKAAKN; DKLAAKLAALKAAKNT;
KLAAKLAALKAAKNTI; LAAKLAALKAAKNTIE; AAKLAALKAAKNTIEN;
AKLAALKAAKNTIENI; KLAALKAAKNTIENIT; LAALKAAKNTIENITD;
AALKAAKNTIENITDK; ALKAAKNTIENITDKD; LKAAKNTIENITDKDQ;
KAAKNTIENITDKDQD; AAKNTIENITDKDQDI; AKNTIENITDKDQDIS;
KNTIENITDKDQDISK; NTIENITDKDQDISKR; TIENITDKDQDISKRK;
IENITDKDQDISKRKI; ENITDKDQDISKRKIW; NITDKDQDISKRKIWS;
ITDKDQDISKRKIWSE; TDKDQDISKRKIWSEA; DKDQDISKRKIWSEAK;
KDQDISKRKIWSEAKL; DQDISKRKIWSEAKLV; QDISKRKIWSEAKLVG;
DISKRKIWSEAKLVGV; ISKRKIWSEAKLVGVT; SKRKIWSEAKLVGVTV;
KRKIWSEAKLVGVTVP; RKIWSEAKLVGVTVPL; KIWSEAKLVGVTVPLL;
IWSEAKLVGVTVPLLG; WSEAKLVGVTVPLLGS; SEAKLVGVTVPLLGSN;
EAKLVGVTVPLLGSNT; AKLVGVTVPLLGSNTS; KLVGVTVPLLGSNTSG;
LVGVTVPLLGSNTSGN; VGVTVPLLGSNTSGNG; GVTVPLLGSNTSGNGD;
VTVPLLGSNTSGNGDK; TVPLLGSNTSGNGDKM; VPLLGSNTSGNGDKMS;
PLLGSNTSGNGDKMSK; LLGSNTSGNGDKMSKN; LGSNTSGNGDKMSKNA;
GSNTSGNGDKMSKNAV; SNTSGNGDKMSKNAVE; NTSGNGDKMSKNAVEQ;
TSGNGDKMSKNAVEQI; SGNGDKMSKNAVEQID; GNGDKMSKNAVEQIDK;

| | |
|---|---|
| | NGDKMSKNAVEQIDKV; GDKMSKNAVEQIDKVI; DKMSKNAVEQIDKVIK; KMSKNAVEQIDKVIKF; MSKNAVEQIDKVIKFL; SKNAVEQIDKVIKFLE; KNAVEQIDKVIKFLEE; NAVEQIDKVIKFLEEG; AVEQIDKVIKFLEEGT; VEQIDKVIKFLEEGTN; |
| Seq 40 | 13 mers:<br>MKDNILKNNKLIA; KDNILKNNKLIAI; DNILKNNKLIAIF;<br>NILKNNKLIAIFL; ILKNNKLIAIFLL; LKNNKLIAIFLLH;<br>KNNKLIAIFLLHV; NNKLIAIFLLHVL; NKLIAIFLLHVLT;<br>KLIAIFLLHVLTV; LIAIFLLHVLTVL; IAIFLLHVLTVLI;<br>AIFLLHVLTVLIL; IFLLHVLTVLILI; FLLHVLTVLILIS;<br>LLHVLTVLILISC; LHVLTVLILISCS; HVLTVLILISCSL;<br>VLTVLILISCSLE; LTVLILISCSLEV; TVLILISCSLEVK;<br>VLILISCSLEVKD; LILISCSLEVKDS; ILISCSLEVKDSN;<br>LISCSLEVKDSNE; ISCSLEVKDSNES; SCSLEVKDSNESK;<br>CSLEVKDSNESKK; SLEVKDSNESKKH; LEVKDSNESKKHK;<br>EVKDSNESKKHKK; VKDSNESKKHKKE; KDSNESKKHKKEK;<br>DSNESKKHKKEKR; SNESKKHKKEKRK; NESKKHKKEKRKG;<br>ESKKHKKEKRKGK; SKKHKKEKRKGKV; KKHKKEKRKGKVE;<br>KHKKEKRKGKVEN; HKKEKRKGKVENL; KKEKRKGKVENLL;<br>KEKRKGKVENLLV; EKRKGKVENLLVA; KRKGKVENLLVAI;<br>RKGKVENLLVAIN; KGKVENLLVAINN; GKVENLLVAINNL;<br>KVENLLVAINNLK; VENLLVAINNLKN; ENLLVAINNLKNP;<br>NLLVAINNLKNPT; LLVAINNLKNPTK; LVAINNLKNPTKP;<br>VAINNLKNPTKPA; AINNLKNPTKPAA; INNLKNPTKPAAG;<br>NNLKNPTKPAAGK; NLKNPTKPAAGKN; LKNPTKPAAGKNK;<br>KNPTKPAAGKNKA; NPTKPAAGKNKAN; PTKPAAGKNKANS;<br>TKPAAGKNKANSK; KPAAGKNKANSKA; PAAGKNKANSKAS;<br>AAGKNKANSKASK; AGKNKANSKASKQ; GKNKANSKASKQK;<br>KNKANSKASKQKN; NKANSKASKQKNN; KANSKASKQKNNP;<br>ANSKASKQKNNPN; NSKASKQKNNPNA; SKASKQKNNPNAN;<br>KASKQKNNPNANA; ASKQKNNPNANAN; SKQKNNPNANANN;<br>KQKNNPNANANNA; QKNNPNANANNAP; KNNPNANANNAPK;<br>NNPNANANNAPKK; NPNANANNAPKKI; PNANANNAPKKIL;<br>NANANNAPKKILD; ANANNAPKKILDP; NANNAPKKILDPE;<br>ANNAPKKILDPEV; NNAPKKILDPEVA; NAPKKILDPEVAK;<br>APKKILDPEVAKL; PKKILDPEVAKLI; KKILDPEVAKLIQ;<br>KILDPEVAKLIQK; ILDPEVAKLIQKI; LDPEVAKLIQKIL;<br>DPEVAKLIQKILD; PEVAKLIQKILDR; EVAKLIQKILDRS;<br>VAKLIQKILDRSE; AKLIQKILDRSEN; KLIQKILDRSENI;<br>LIQKILDRSENII; IQKILDRSENIIQ; QKILDRSENIIQI;<br>KILDRSENIIQIS; ILDRSENIIQISE; LDRSENIIQISEM;<br>DRSENIIQISEMD; RSENIIQISEMDS; SENIIQISEMDSS;<br>ENIIQISEMDSSR; NIIQISEMDSSRG; IIQISEMDSSRGE;<br>IQISEMDSSRGEP; QISEMDSSRGEPN; ISEMDSSRGEPND;<br>SEMDSSRGEPNDQ; EMDSSRGEPNDQF; MDSSRGEPNDQFG;<br>DSSRGEPNDQFGM; SSRGEPNDQFGMR; SRGEPNDQFGMRA;<br>RGEPNDQFGMRAE; GEPNDQFGMRAEI; EPNDQFGMRAEIF;<br>PNDQFGMRAEIFS; NDQFGMRAEIFSK; DQFGMRAEIFSKI;<br>QFGMRAEIFSKIF; FGMRAEIFSKIFF; GMRAEIFSKIFFN; |

MRAEIFSKIFFNA; RAEIFSKIFFNAN; AEIFSKIFFNANS;
EIFSKIFFNANST; IFSKIFFNANSTV; FSKIFFNANSTVH;
SKIFFNANSTVHF; KIFFNANSTVHFD; IFFNANSTVHFDS;
FFNANSTVHFDSH; FNANSTVHFDSHE; NANSTVHFDSHEY;
ANSTVHFDSHEYT; NSTVHFDSHEYTE; STVHFDSHEYTEE;
TVHFDSHEYTEER; VHFDSHEYTEERR; HFDSHEYTEERRM;
FDSHEYTEERRML; DSHEYTEERRMLY; SHEYTEERRMLYT;
HEYTEERRMLYTS; EYTEERRMLYTSL; YTEERRMLYTSLN;
TEERRMLYTSLNF; EERRMLYTSLNFN; ERRMLYTSLNFNE;
RRMLYTSLNFNEG; RMLYTSLNFNEGK; MLYTSLNFNEGKI;
LYTSLNFNEGKIF; YTSLNFNEGKIFN; TSLNFNEGKIFNL;
SLNFNEGKIFNLG; LNFNEGKIFNLGQ; NFNEGKIFNLGQI;
FNEGKIFNLGQIL; NEGKIFNLGQILS; EGKIFNLGQILSK;
GKIFNLGQILSKL; KIFNLGQILSKLS; IFNLGQILSKLSQ;
FNLGQILSKLSQD; NLGQILSKLSQDS; LGQILSKLSQDSN;
GQILSKLSQDSNY; QILSKLSQDSNYR; ILSKLSQDSNYRG;
LSKLSQDSNYRGL; SKLSQDSNYRGLV; KLSQDSNYRGLVK;
LSQDSNYRGLVKE; SQDSNYRGLVKET; QDSNYRGLVKETL;
DSNYRGLVKETLI; SNYRGLVKETLIN; NYRGLVKETLINR;
YRGLVKETLINRG; RGLVKETLINRGF; GLVKETLINRGFS;
LVKETLINRGFSI; VKETLINRGFSIQ; KETLINRGFSIQL;
ETLINRGFSIQLA; TLINRGFSIQLAM; LINRGFSIQLAME;
INRGFSIQLAMEE; NRGFSIQLAMEEI; RGFSIQLAMEEIS;
GFSIQLAMEEISA; FSIQLAMEEISAK; SIQLAMEEISAKI;
IQLAMEEISAKIL; QLAMEEISAKILN; LAMEEISAKILNV;
AMEEISAKILNVK; MEEISAKILNVKD; EEISAKILNVKDK;
EISAKILNVKDKL; ISAKILNVKDKLQ; SAKILNVKDKLQQ;
AKILNVKDKLQQL; KILNVKDKLQQLN; ILNVKDKLQQLNK;
LNVKDKLQQLNKP; NVKDKLQQLNKPN; VKDKLQQLNKPNL;
KDKLQQLNKPNLE; DKLQQLNKPNLET; KLQQLNKPNLETL;
LQQLNKPNLETLY; QQLNKPNLETLYN; QLNKPNLETLYND;
LNKPNLETLYNDF; NKPNLETLYNDFE; KPNLETLYNDFEK;
PNLETLYNDFEKL; NLETLYNDFEKLT; LETLYNDFEKLTS;
ETLYNDFEKLTSL; TLYNDFEKLTSLK; LYNDFEKLTSLKE;
YNDFEKLTSLKEK; NDFEKLTSLKEKW; DFEKLTSLKEKWL;
FEKLTSLKEKWLK; EKLTSLKEKWLKD; KLTSLKEKWLKDT;
LTSLKEKWLKDTD; TSLKEKWLKDTDD; SLKEKWLKDTDDL;
LKEKWLKDTDDLI; KEKWLKDTDDLID; EKWLKDTDDLIDE;
KWLKDTDDLIDEY; WLKDTDDLIDEYN; LKDTDDLIDEYNT;
KDTDDLIDEYNTN; DTDDLIDEYNTNP; TDDLIDEYNTNPD;
DDLIDEYNTNPDL; DLIDEYNTNPDLQ; LIDEYNTNPDLQT;
IDEYNTNPDLQTD; DEYNTNPDLQTDV; EYNTNPDLQTDVS;
YNTNPDLQTDVSK; NTNPDLQTDVSKL; TNPDLQTDVSKLN;
NPDLQTDVSKLND; PDLQTDVSKLNDT; DLQTDVSKLNDTL;
LQTDVSKLNDTLR; QTDVSKLNDTLRS; TDVSKLNDTLRSK;
DVSKLNDTLRSKN; VSKLNDTLRSKNS; SKLNDTLRSKNSR;
KLNDTLRSKNSRA; LNDTLRSKNSRAQ; NDTLRSKNSRAQF;
DTLRSKNSRAQFA; TLRSKNSRAQFAN; LRSKNSRAQFANI;
RSKNSRAQFANIH; SKNSRAQFANIHD; KNSRAQFANIHDI;
NSRAQFANIHDII; SRAQFANIHDIIL; RAQFANIHDIILD;
AQFANIHDIILDL; QFANIHDIILDLV; FANIHDIILDLVN;

ANIHDIILDLVNT; NIHDIILDLVNTT; IHDIILDLVNTTT;
HDIILDLVNTTTN; DIILDLVNTTTNI; IILDLVNTTTNIL;
ILDLVNTTTNILA; LDLVNTTTNILAP; DLVNTTTNILAPI;
LVNTTTNILAPIQ;

14 mers:
MKDNILKNNKLIAI; KDNILKNNKLIAIF; DNILKNNKLIAIFL;
NILKNNKLIAIFLL; ILKNNKLIAIFLLH; LKNNKLIAIFLLHV;
KNNKLIAIFLLHVL; NNKLIAIFLLHVLT; NKLIAIFLLHVLTV;
KLIAIFLLHVLTVL; LIAIFLLHVLTVLI; IAIFLLHVLTVLIL;
AIFLLHVLTVLILI; IFLLHVLTVLILIS; FLLHVLTVLILISC;
LLHVLTVLILISCS; LHVLTVLILISCSL; HVLTVLILISCSLE;
VLTVLILISCSLEV; LTVLILISCSLEVK; TVLILISCSLEVKD;
VLILISCSLEVKDS; LILISCSLEVKDSN; ILISCSLEVKDSNE;
LISCSLEVKDSNES; ISCSLEVKDSNESK; SCSLEVKDSNESKK;
CSLEVKDSNESKKH; SLEVKDSNESKKHK; LEVKDSNESKKHKK;
EVKDSNESKKHKKE; VKDSNESKKHKKEK; KDSNESKKHKKEKR;
DSNESKKHKKEKRK; SNESKKHKKEKRKG; NESKKHKKEKRKGK;
ESKKHKKEKRKGKV; SKKHKKEKRKGKVE; KKHKKEKRKGKVEN;
KHKKEKRKGKVENL; HKKEKRKGKVENLL; KKEKRKGKVENLLV;
KEKRKGKVENLLVA; EKRKGKVENLLVAI; KRKGKVENLLVAIN;
RKGKVENLLVAINN; KGKVENLLVAINNL; GKVENLLVAINNLK;
KVENLLVAINNLKN; VENLLVAINNLKNP; ENLLVAINNLKNPT;
NLLVAINNLKNPTK; LLVAINNLKNPTKP; LVAINNLKNPTKPA;
VAINNLKNPTKPAA; AINNLKNPTKPAAG; INNLKNPTKPAAGK;
NNLKNPTKPAAGKN; NLKNPTKPAAGKNK; LKNPTKPAAGKNKA;
KNPTKPAAGKNKAN; NPTKPAAGKNKANS; PTKPAAGKNKANSK;
TKPAAGKNKANSKA; KPAAGKNKANSKAS; PAAGKNKANSKASK;
AAGKNKANSKASKQ; AGKNKANSKASKQK; GKNKANSKASKQKN;
KNKANSKASKQKNN; NKANSKASKQKNNP; KANSKASKQKNNPN;
ANSKASKQKNNPNA; NSKASKQKNNPNAN; SKASKQKNNPNANA;
KASKQKNNPNANAN; ASKQKNNPNANANN; SKQKNNPNANANNA;
KQKNNPNANANNAP; QKNNPNANANNAPK; KNNPNANANNAPKK;
NNPNANANNAPKKI; NPNANANNAPKKIL; PNANANNAPKKILD;
NANANNAPKKILDP; ANANNAPKKILDPE; NANNAPKKILDPEV;
ANNAPKKILDPEVA; NNAPKKILDPEVAK; NAPKKILDPEVAKL;
APKKILDPEVAKLI; PKKILDPEVAKLIQ; KKILDPEVAKLIQK;
KILDPEVAKLIQKI; ILDPEVAKLIQKIL; LDPEVAKLIQKILD;
DPEVAKLIQKILDR; PEVAKLIQKILDRS; EVAKLIQKILDRSE;
VAKLIQKILDRSEN; AKLIQKILDRSENI; KLIQKILDRSENII;
LIQKILDRSENIIQ; IQKILDRSENIIQI; QKILDRSENIIQIS;
KILDRSENIIQISE; ILDRSENIIQISEM; LDRSENIIQISEMD;
DRSENIIQISEMDS; RSENIIQISEMDSS; SENIIQISEMDSSR;
ENIIQISEMDSSRG; NIIQISEMDSSRGE; IIQISEMDSSRGEP;
IQISEMDSSRGEPN; QISEMDSSRGEPND; ISEMDSSRGEPNDQ;
SEMDSSRGEPNDQF; EMDSSRGEPNDQFG; MDSSRGEPNDQFGM;
DSSRGEPNDQFGMR; SSRGEPNDQFGMRA; SRGEPNDQFGMRAE;
RGEPNDQFGMRAEI; GEPNDQFGMRAEIF; EPNDQFGMRAEIFS;
PNDQFGMRAEIFSK; NDQFGMRAEIFSKI; DQFGMRAEIFSKIF;
QFGMRAEIFSKIFF; FGMRAEIFSKIFFN; GMRAEIFSKIFFNA;
MRAEIFSKIFFNAN; RAEIFSKIFFNANS; AEIFSKIFFNANST;

| | | |
|---|---|---|
| EIFSKIFFNANSTV; | IFSKIFFNANSTVH; | FSKIFFNANSTVHF; |
| SKIFFNANSTVHFD; | KIFFNANSTVHFDS; | IFFNANSTVHFDSH; |
| FFNANSTVHFDSHE; | FNANSTVHFDSHEY; | NANSTVHFDSHEYT; |
| ANSTVHFDSHEYTE; | NSTVHFDSHEYTEE; | STVHFDSHEYTEER; |
| TVHFDSHEYTEERR; | VHFDSHEYTEERRM; | HFDSHEYTEERRML; |
| FDSHEYTEERRMLY; | DSHEYTEERRMLYT; | SHEYTEERRMLYTS; |
| HEYTEERRMLYTSL; | EYTEERRMLYTSLN; | YTEERRMLYTSLNF; |
| TEERRMLYTSLNFN; | EERRMLYTSLNFNE; | ERRMLYTSLNFNEG; |
| RRMLYTSLNFNEGK; | RMLYTSLNFNEGKI; | MLYTSLNFNEGKIF; |
| LYTSLNFNEGKIFN; | YTSLNFNEGKIFNL; | TSLNFNEGKIFNLG; |
| SLNFNEGKIFNLGQ; | LNFNEGKIFNLGQI; | NFNEGKIFNLGQIL; |
| FNEGKIFNLGQILS; | NEGKIFNLGQILSK; | EGKIFNLGQILSKL; |
| GKIFNLGQILSKLS; | KIFNLGQILSKLSQ; | IFNLGQILSKLSQD; |
| FNLGQILSKLSQDS; | NLGQILSKLSQDSN; | LGQILSKLSQDSNY; |
| GQILSKLSQDSNYR; | QILSKLSQDSNYRG; | ILSKLSQDSNYRGL; |
| LSKLSQDSNYRGLV; | SKLSQDSNYRGLVK; | KLSQDSNYRGLVKE; |
| LSQDSNYRGLVKET; | SQDSNYRGLVKETL; | QDSNYRGLVKETLI; |
| DSNYRGLVKETLIN; | SNYRGLVKETLINR; | NYRGLVKETLINRG; |
| YRGLVKETLINRGF; | RGLVKETLINRGFS; | GLVKETLINRGFSI; |
| LVKETLINRGFSIQ; | VKETLINRGFSIQL; | KETLINRGFSIQLA; |
| ETLINRGFSIQLAM; | TLINRGFSIQLAME; | LINRGFSIQLAMEE; |
| INRGFSIQLAMEEI; | NRGFSIQLAMEEIS; | RGFSIQLAMEEISA; |
| GFSIQLAMEEISAK; | FSIQLAMEEISAKI; | SIQLAMEEISAKIL; |
| IQLAMEEISAKILN; | QLAMEEISAKILNV; | LAMEEISAKILNVK; |
| AMEEISAKILNVKD; | MEEISAKILNVKDK; | EEISAKILNVKDKL; |
| EISAKILNVKDKLQ; | ISAKILNVKDKLQQ; | SAKILNVKDKLQQL; |
| AKILNVKDKLQQLN; | KILNVKDKLQQLNK; | ILNVKDKLQQLNKP; |
| LNVKDKLQQLNKPN; | NVKDKLQQLNKPNL; | VKDKLQQLNKPNLE; |
| KDKLQQLNKPNLET; | DKLQQLNKPNLETL; | KLQQLNKPNLETLY; |
| LQQLNKPNLETLYN; | QQLNKPNLETLYND; | QLNKPNLETLYNDF; |
| LNKPNLETLYNDFE; | NKPNLETLYNDFEK; | KPNLETLYNDFEKL; |
| PNLETLYNDFEKLT; | NLETLYNDFEKLTS; | LETLYNDFEKLTSL; |
| ETLYNDFEKLTSLK; | TLYNDFEKLTSLKE; | LYNDFEKLTSLKEK; |
| YNDFEKLTSLKEKW; | NDFEKLTSLKEKWL; | DFEKLTSLKEKWLK; |
| FEKLTSLKEKWLKD; | EKLTSLKEKWLKDT; | KLTSLKEKWLKDTD; |
| LTSLKEKWLKDTDD; | TSLKEKWLKDTDDL; | SLKEKWLKDTDDLI; |
| LKEKWLKDTDDLID; | KEKWLKDTDDLIDE; | EKWLKDTDDLIDEY; |
| KWLKDTDDLIDEYN; | WLKDTDDLIDEYNT; | LKDTDDLIDEYNTN; |
| KDTDDLIDEYNTNP; | DTDDLIDEYNTNPD; | TDDLIDEYNTNPDL; |
| DDLIDEYNTNPDLQ; | DLIDEYNTNPDLQT; | LIDEYNTNPDLQTD; |
| IDEYNTNPDLQTDV; | DEYNTNPDLQTDVS; | EYNTNPDLQTDVSK; |
| YNTNPDLQTDVSKL; | NTNPDLQTDVSKLN; | TNPDLQTDVSKLND; |
| NPDLQTDVSKLNDT; | PDLQTDVSKLNDTL; | DLQTDVSKLNDTLR; |
| LQTDVSKLNDTLRS; | QTDVSKLNDTLRSK; | TDVSKLNDTLRSKN; |
| DVSKLNDTLRSKNS; | VSKLNDTLRSKNSR; | SKLNDTLRSKNSRA; |
| KLNDTLRSKNSRAQ; | LNDTLRSKNSRAQF; | NDTLRSKNSRAQFA; |
| DTLRSKNSRAQFAN; | TLRSKNSRAQFANI; | LRSKNSRAQFANIH; |
| RSKNSRAQFANIHD; | SKNSRAQFANIHDI; | KNSRAQFANIHDII; |
| NSRAQFANIHDIIL; | SRAQFANIHDIILD; | RAQFANIHDIILDL; |
| AQFANIHDIILDLV; | QFANIHDIILDLVN; | FANIHDIILDLVNT; |
| ANIHDIILDLVNTT; | NIHDIILDLVNTTT; | IHDIILDLVNTTTN; |

HDIILDLVNTTTNI; DIILDLVNTTTNIL; IILDLVNTTTNILA;
ILDLVNTTTNILAP; LDLVNTTTNILAPI; DLVNTTTNILAPIQ;

15 mers:
MKDNILKNNKLIAIF; KDNILKNNKLIAIFL; DNILKNNKLIAIFLL;
NILKNNKLIAIFLLH; ILKNNKLIAIFLLHV; LKNNKLIAIFLLHVL;
KNNKLIAIFLLHVLT; NNKLIAIFLLHVLTV; NKLIAIFLLHVLTVL;
KLIAIFLLHVLTVLI; LIAIFLLHVLTVLIL; IAIFLLHVLTVLILI;
AIFLLHVLTVLILIS; IFLLHVLTVLILISC; FLLHVLTVLILISCS;
LLHVLTVLILISCSL; LHVLTVLILISCSLE; HVLTVLILISCSLEV;
VLTVLILISCSLEVK; LTVLILISCSLEVKD; TVLILISCSLEVKDS;
VLILISCSLEVKDSN; LILISCSLEVKDSNE; ILISCSLEVKDSNES;
LISCSLEVKDSNESK; ISCSLEVKDSNESKK; SCSLEVKDSNESKKH;
CSLEVKDSNESKKHK; SLEVKDSNESKKHKK; LEVKDSNESKKHKKE;
EVKDSNESKKHKKEK; VKDSNESKKHKKEKR; KDSNESKKHKKEKRK;
DSNESKKHKKEKRKG; SNESKKHKKEKRKGK; NESKKHKKEKRKGKV;
ESKKHKKEKRKGKVE; SKKHKKEKRKGKVEN; KKHKKEKRKGKVENL;
KHKKEKRKGKVENLL; HKKEKRKGKVENLLV; KKEKRKGKVENLLVA;
KEKRKGKVENLLVAI; EKRKGKVENLLVAIN; KRKGKVENLLVAINN;
RKGKVENLLVAINNL; KGKVENLLVAINNLK; GKVENLLVAINNLKN;
KVENLLVAINNLKNP; VENLLVAINNLKNPT; ENLLVAINNLKNPTK;
NLLVAINNLKNPTKP; LLVAINNLKNPTKPA; LVAINNLKNPTKPAA;
VAINNLKNPTKPAAG; AINNLKNPTKPAAGK; INNLKNPTKPAAGKN;
NNLKNPTKPAAGKNK; NLKNPTKPAAGKNKA; LKNPTKPAAGKNKAN;
KNPTKPAAGKNKANS; NPTKPAAGKNKANSK; PTKPAAGKNKANSKA;
TKPAAGKNKANSKAS; KPAAGKNKANSKASK; PAAGKNKANSKASKQ;
AAGKNKANSKASKQK; AGKNKANSKASKQKN; GKNKANSKASKQKNN;
KNKANSKASKQKNNP; NKANSKASKQKNNPN; KANSKASKQKNNPNA;
ANSKASKQKNNPNAN; NSKASKQKNNPNANA; SKASKQKNNPNANAN;
KASKQKNNPNANANN; ASKQKNNPNANANNA; SKQKNNPNANANNAP;
KQKNNPNANANNAPK; QKNNPNANANNAPKK; KNNPNANANNAPKKI;
NNPNANANNAPKKIL; NPNANANNAPKKILD; PNANANNAPKKILDP;
NANANNAPKKILDPE; ANANNAPKKILDPEV; NANNAPKKILDPEVA;
ANNAPKKILDPEVAK; NNAPKKILDPEVAKL; NAPKKILDPEVAKLI;
APKKILDPEVAKLIQ; PKKILDPEVAKLIQK; KKILDPEVAKLIQKI;
KILDPEVAKLIQKIL; ILDPEVAKLIQKILD; LDPEVAKLIQKILDR;
DPEVAKLIQKILDRS; PEVAKLIQKILDRSE; EVAKLIQKILDRSEN;
VAKLIQKILDRSENI; AKLIQKILDRSENII; KLIQKILDRSENIIQ;
LIQKILDRSENIIQI; IQKILDRSENIIQIS; QKILDRSENIIQISE;
KILDRSENIIQISEM; ILDRSENIIQISEMD; LDRSENIIQISEMDS;
DRSENIIQISEMDSS; RSENIIQISEMDSSR; SENIIQISEMDSSRG;
ENIIQISEMDSSRGE; NIIQISEMDSSRGEP; IIQISEMDSSRGEPN;
IQISEMDSSRGEPND; QISEMDSSRGEPNDQ; ISEMDSSRGEPNDQF;
SEMDSSRGEPNDQFG; EMDSSRGEPNDQFGM; MDSSRGEPNDQFGMR;
DSSRGEPNDQFGMRA; SSRGEPNDQFGMRAE; SRGEPNDQFGMRAEI;
RGEPNDQFGMRAEIF; GEPNDQFGMRAEIFS; EPNDQFGMRAEIFSK;
PNDQFGMRAEIFSKI; NDQFGMRAEIFSKIF; DQFGMRAEIFSKIFF;
QFGMRAEIFSKIFFN; FGMRAEIFSKIFFNA; GMRAEIFSKIFFNAN;
MRAEIFSKIFFNANS; RAEIFSKIFFNANST; AEIFSKIFFNANSTV;
EIFSKIFFNANSTVH; IFSKIFFNANSTVHF; FSKIFFNANSTVHFD;
SKIFFNANSTVHFDS; KIFFNANSTVHFDSH; IFFNANSTVHFDSHE;

FFNANSTVHFDSHEY; FNANSTVHFDSHEYT; NANSTVHFDSHEYTE;
ANSTVHFDSHEYTEE; NSTVHFDSHEYTEER; STVHFDSHEYTEERR;
TVHFDSHEYTEERRM; VHFDSHEYTEERRML; HFDSHEYTEERRMLY;
FDSHEYTEERRMLYT; DSHEYTEERRMLYTS; SHEYTEERRMLYTSL;
HEYTEERRMLYTSLN; EYTEERRMLYTSLNF; YTEERRMLYTSLNFN;
TEERRMLYTSLNFNE; EERRMLYTSLNFNEG; ERRMLYTSLNFNEGK;
RRMLYTSLNFNEGKI; RMLYTSLNFNEGKIF; MLYTSLNFNEGKIFN;
LYTSLNFNEGKIFNL; YTSLNFNEGKIFNLG; TSLNFNEGKIFNLGQ;
SLNFNEGKIFNLGQI; LNFNEGKIFNLGQIL; NFNEGKIFNLGQILS;
FNEGKIFNLGQILSK; NEGKIFNLGQILSKL; EGKIFNLGQILSKLS;
GKIFNLGQILSKLSQ; KIFNLGQILSKLSQD; IFNLGQILSKLSQDS;
FNLGQILSKLSQDSN; NLGQILSKLSQDSNY; LGQILSKLSQDSNYR;
GQILSKLSQDSNYRG; QILSKLSQDSNYRGL; ILSKLSQDSNYRGLV;
LSKLSQDSNYRGLVK; SKLSQDSNYRGLVKE; KLSQDSNYRGLVKET;
LSQDSNYRGLVKETL; SQDSNYRGLVKETLI; QDSNYRGLVKETLIN;
DSNYRGLVKETLINR; SNYRGLVKETLINRG; NYRGLVKETLINRGF;
YRGLVKETLINRGFS; RGLVKETLINRGFSI; GLVKETLINRGFSIQ;
LVKETLINRGFSIQL; VKETLINRGFSIQLA; KETLINRGFSIQLAM;
ETLINRGFSIQLAME; TLINRGFSIQLAMEE; LINRGFSIQLAMEEI;
INRGFSIQLAMEEIS; NRGFSIQLAMEEISA; RGFSIQLAMEEISAK;
GFSIQLAMEEISAKI; FSIQLAMEEISAKIL; SIQLAMEEISAKILN;
IQLAMEEISAKILNV; QLAMEEISAKILNVK; LAMEEISAKILNVKD;
AMEEISAKILNVKDK; MEEISAKILNVKDKL; EEISAKILNVKDKLQ;
EISAKILNVKDKLQQ; ISAKILNVKDKLQQL; SAKILNVKDKLQQLN;
AKILNVKDKLQQLNK; KILNVKDKLQQLNKP; ILNVKDKLQQLNKPN;
LNVKDKLQQLNKPNL; NVKDKLQQLNKPNLE; VKDKLQQLNKPNLET;
KDKLQQLNKPNLETL; DKLQQLNKPNLETLY; KLQQLNKPNLETLYN;
LQQLNKPNLETLYND; QQLNKPNLETLYNDF; QLNKPNLETLYNDFE;
LNKPNLETLYNDFEK; NKPNLETLYNDFEKL; KPNLETLYNDFEKLT;
PNLETLYNDFEKLTS; NLETLYNDFEKLTSL; LETLYNDFEKLTSLK;
ETLYNDFEKLTSLKE; TLYNDFEKLTSLKEK; LYNDFEKLTSLKEKW;
YNDFEKLTSLKEKWL; NDFEKLTSLKEKWLK; DFEKLTSLKEKWLKD;
FEKLTSLKEKWLKDT; EKLTSLKEKWLKDTD; KLTSLKEKWLKDTDD;
LTSLKEKWLKDTDDL; TSLKEKWLKDTDDLI; SLKEKWLKDTDDLID;
LKEKWLKDTDDLIDE; KEKWLKDTDDLIDEY; EKWLKDTDDLIDEYN;
KWLKDTDDLIDEYNT; WLKDTDDLIDEYNTN; LKDTDDLIDEYNTNP;
KDTDDLIDEYNTNPD; DTDDLIDEYNTNPDL; TDDLIDEYNTNPDLQ;
DDLIDEYNTNPDLQT; DLIDEYNTNPDLQTD; LIDEYNTNPDLQTDV;
IDEYNTNPDLQTDVS; DEYNTNPDLQTDVSK; EYNTNPDLQTDVSKL;
YNTNPDLQTDVSKLN; NTNPDLQTDVSKLND; TNPDLQTDVSKLNDT;
NPDLQTDVSKLNDTL; PDLQTDVSKLNDTLR; DLQTDVSKLNDTLRS;
LQTDVSKLNDTLRSK; QTDVSKLNDTLRSKN; TDVSKLNDTLRSKNS;
DVSKLNDTLRSKNSR; VSKLNDTLRSKNSRA; SKLNDTLRSKNSRAQ;
KLNDTLRSKNSRAQF; LNDTLRSKNSRAQFA; NDTLRSKNSRAQFAN;
DTLRSKNSRAQFANI; TLRSKNSRAQFANIH; LRSKNSRAQFANIHD;
RSKNSRAQFANIHDI; SKNSRAQFANIHDII; KNSRAQFANIHDIIL;
NSRAQFANIHDIILD; SRAQFANIHDIILDL; RAQFANIHDIILDLV;
AQFANIHDIILDLVN; QFANIHDIILDLVNT; FANIHDIILDLVNTT;
ANIHDIILDLVNTTT; NIHDIILDLVNTTTN; IHDIILDLVNTTTNI;
HDIILDLVNTTTNIL; DIILDLVNTTTNILA; IILDLVNTTTNILAP;
ILDLVNTTTNILAPI; LDLVNTTTNILAPIQ;

16 mers:
MKDNILKNNKLIAIFL; KDNILKNNKLIAIFLL; DNILKNNKLIAIFLLH;
NILKNNKLIAIFLLHV; ILKNNKLIAIFLLHVL; LKNNKLIAIFLLHVLT;
KNNKLIAIFLLHVLTV; NNKLIAIFLLHVLTVL; NKLIAIFLLHVLTVLI;
KLIAIFLLHVLTVLIL; LIAIFLLHVLTVLILI; IAIFLLHVLTVLILIS;
AIFLLHVLTVLILISC; IFLLHVLTVLILISCS; FLLHVLTVLILISCSL;
LLHVLTVLILISCSLE; LHVLTVLILISCSLEV; HVLTVLILISCSLEVK;
VLTVLILISCSLEVKD; LTVLILISCSLEVKDS; TVLILISCSLEVKDSN;
VLILISCSLEVKDSNE; LILISCSLEVKDSNES; ILISCSLEVKDSNESK;
LISCSLEVKDSNESKK; ISCSLEVKDSNESKKH; SCSLEVKDSNESKKHK;
CSLEVKDSNESKKHKK; SLEVKDSNESKKHKKE; LEVKDSNESKKHKKEK;
EVKDSNESKKHKKEKR; VKDSNESKKHKKEKRK; KDSNESKKHKKEKRKG;
DSNESKKHKKEKRKGK; SNESKKHKKEKRKGKV; NESKKHKKEKRKGKVE;
ESKKHKKEKRKGKVEN; SKKHKKEKRKGKVENL; KKHKKEKRKGKVENLL;
KHKKEKRKGKVENLLV; HKKEKRKGKVENLLVA; KKEKRKGKVENLLVAI;
KEKRKGKVENLLVAIN; EKRKGKVENLLVAINN; KRKGKVENLLVAINNL;
RKGKVENLLVAINNLK; KGKVENLLVAINNLKN; GKVENLLVAINNLKNP;
KVENLLVAINNLKNPT; VENLLVAINNLKNPTK; ENLLVAINNLKNPTKP;
NLLVAINNLKNPTKPA; LLVAINNLKNPTKPAA; LVAINNLKNPTKPAAG;
VAINNLKNPTKPAAGK; AINNLKNPTKPAAGKN; INNLKNPTKPAAGKNK;
NNLKNPTKPAAGKNKA; NLKNPTKPAAGKNKAN; LKNPTKPAAGKNKANS;
KNPTKPAAGKNKANSK; NPTKPAAGKNKANSKA; PTKPAAGKNKANSKAS;
TKPAAGKNKANSKASK; KPAAGKNKANSKASKQ; PAAGKNKANSKASKQK;
AAGKNKANSKASKQKN; AGKNKANSKASKQKNN; GKNKANSKASKQKNNP;
KNKANSKASKQKNNPN; NKANSKASKQKNNPNA; KANSKASKQKNNPNAN;
ANSKASKQKNNPNANA; NSKASKQKNNPNANAN; SKASKQKNNPNANANN;
KASKQKNNPNANANNA; ASKQKNNPNANANNAP; SKQKNNPNANANNAPK;
KQKNNPNANANNAPKK; QKNNPNANANNAPKKI; KNNPNANANNAPKKIL;
NNPNANANNAPKKILD; NPNANANNAPKKILDP; PNANANNAPKKILDPE;
NANANNAPKKILDPEV; ANANNAPKKILDPEVA; NANNAPKKILDPEVAK;
ANNAPKKILDPEVAKL; NNAPKKILDPEVAKLI; NAPKKILDPEVAKLIQ;
APKKILDPEVAKLIQK; PKKILDPEVAKLIQKI; KKILDPEVAKLIQKIL;
KILDPEVAKLIQKILD; ILDPEVAKLIQKILDR; LDPEVAKLIQKILDRS;
DPEVAKLIQKILDRSE; PEVAKLIQKILDRSEN; EVAKLIQKILDRSENI;
VAKLIQKILDRSENII; AKLIQKILDRSENIIQ; KLIQKILDRSENIIQI;
LIQKILDRSENIIQIS; IQKILDRSENIIQISE; QKILDRSENIIQISEM;
KILDRSENIIQISEMD; ILDRSENIIQISEMDS; LDRSENIIQISEMDSS;
DRSENIIQISEMDSSR; RSENIIQISEMDSSRG; SENIIQISEMDSSRGE;
ENIIQISEMDSSRGEP; NIIQISEMDSSRGEPN; IIQISEMDSSRGEPND;
IQISEMDSSRGEPNDQ; QISEMDSSRGEPNDQF; ISEMDSSRGEPNDQFG;
SEMDSSRGEPNDQFGM; EMDSSRGEPNDQFGMR; MDSSRGEPNDQFGMRA;
DSSRGEPNDQFGMRAE; SSRGEPNDQFGMRAEI; SRGEPNDQFGMRAEIF;
RGEPNDQFGMRAEIFS; GEPNDQFGMRAEIFSK; EPNDQFGMRAEIFSKI;
PNDQFGMRAEIFSKIF; NDQFGMRAEIFSKIFF; DQFGMRAEIFSKIFFN;
QFGMRAEIFSKIFFNA; FGMRAEIFSKIFFNAN; GMRAEIFSKIFFNANS;
MRAEIFSKIFFNANST; RAEIFSKIFFNANSTV; AEIFSKIFFNANSTVH;
EIFSKIFFNANSTVHF; IFSKIFFNANSTVHFD; FSKIFFNANSTVHFDS;
SKIFFNANSTVHFDSH; KIFFNANSTVHFDSHE; IFFNANSTVHFDSHEY;
FFNANSTVHFDSHEYT; FNANSTVHFDSHEYTE; NANSTVHFDSHEYTEE;
ANSTVHFDSHEYTEER; NSTVHFDSHEYTEERR; STVHFDSHEYTEERRM;

| | TVHFDSHEYTEERRML; VHFDSHEYTEERRMLY; HFDSHEYTEERRMLYT; FDSHEYTEERRMLYTS; DSHEYTEERRMLYTSL; SHEYTEERRMLYTSLN; HEYTEERRMLYTSLNF; EYTEERRMLYTSLNFN; YTEERRMLYTSLNFNE; TEERRMLYTSLNFNEG; EERRMLYTSLNFNEGK; ERRMLYTSLNFNEGKI; RRMLYTSLNFNEGKIF; RMLYTSLNFNEGKIFN; MLYTSLNFNEGKIFNL; LYTSLNFNEGKIFNLG; YTSLNFNEGKIFNLGQ; TSLNFNEGKIFNLGQI; SLNFNEGKIFNLGQIL; LNFNEGKIFNLGQILS; NFNEGKIFNLGQILSK; FNEGKIFNLGQILSKL; NEGKIFNLGQILSKLS; EGKIFNLGQILSKLSQ; GKIFNLGQILSKLSQD; KIFNLGQILSKLSQDS; IFNLGQILSKLSQDSN; FNLGQILSKLSQDSNY; NLGQILSKLSQDSNYR; LGQILSKLSQDSNYRG; GQILSKLSQDSNYRGL; QILSKLSQDSNYRGLV; ILSKLSQDSNYRGLVK; LSKLSQDSNYRGLVKE; SKLSQDSNYRGLVKET; KLSQDSNYRGLVKETL; LSQDSNYRGLVKETLI; SQDSNYRGLVKETLIN; QDSNYRGLVKETLINR; DSNYRGLVKETLINRG; SNYRGLVKETLINRGF; NYRGLVKETLINRGFS; YRGLVKETLINRGFSI; RGLVKETLINRGFSIQ; GLVKETLINRGFSIQL; LVKETLINRGFSIQLA; VKETLINRGFSIQLAM; KETLINRGFSIQLAME; ETLINRGFSIQLAMEE; TLINRGFSIQLAMEEI; LINRGFSIQLAMEEIS; INRGFSIQLAMEEISA; NRGFSIQLAMEEISAK; RGFSIQLAMEEISAKI; GFSIQLAMEEISAKIL; FSIQLAMEEISAKILN; SIQLAMEEISAKILNV; IQLAMEEISAKILNVK; QLAMEEISAKILNVKD; LAMEEISAKILNVKDK; AMEEISAKILNVKDKL; MEEISAKILNVKDKLQ; EEISAKILNVKDKLQQ; EISAKILNVKDKLQQL; ISAKILNVKDKLQQLN; SAKILNVKDKLQQLNK; AKILNVKDKLQQLNKP; KILNVKDKLQQLNKPN; ILNVKDKLQQLNKPNL; LNVKDKLQQLNKPNLE; NVKDKLQQLNKPNLET; VKDKLQQLNKPNLETL; KDKLQQLNKPNLETLY; DKLQQLNKPNLETLYN; KLQQLNKPNLETLYND; LQQLNKPNLETLYNDF; QQLNKPNLETLYNDFE; QLNKPNLETLYNDFEK; LNKPNLETLYNDFEKL; NKPNLETLYNDFEKLT; KPNLETLYNDFEKLTS; PNLETLYNDFEKLTSL; NLETLYNDFEKLTSLK; LETLYNDFEKLTSLKE; ETLYNDFEKLTSLKEK; TLYNDFEKLTSLKEKW; LYNDFEKLTSLKEKWL; YNDFEKLTSLKEKWLK; NDFEKLTSLKEKWLKD; DFEKLTSLKEKWLKDT; FEKLTSLKEKWLKDTD; EKLTSLKEKWLKDTDD; KLTSLKEKWLKDTDDL; LTSLKEKWLKDTDDLI; TSLKEKWLKDTDDLID; SLKEKWLKDTDDLIDE; LKEKWLKDTDDLIDEY; KEKWLKDTDDLIDEYN; EKWLKDTDDLIDEYNT; KWLKDTDDLIDEYNTN; WLKDTDDLIDEYNTNP; LKDTDDLIDEYNTNPD; KDTDDLIDEYNTNPDL; DTDDLIDEYNTNPDLQ; TDDLIDEYNTNPDLQT; DDLIDEYNTNPDLQTD; DLIDEYNTNPDLQTDV; LIDEYNTNPDLQTDVS; IDEYNTNPDLQTDVSK; DEYNTNPDLQTDVSKL; EYNTNPDLQTDVSKLN; YNTNPDLQTDVSKLND; NTNPDLQTDVSKLNDT; TNPDLQTDVSKLNDTL; NPDLQTDVSKLNDTLR; PDLQTDVSKLNDTLRS; DLQTDVSKLNDTLRSK; LQTDVSKLNDTLRSKN; QTDVSKLNDTLRSKNS; TDVSKLNDTLRSKNSR; DVSKLNDTLRSKNSRA; VSKLNDTLRSKNSRAQ; SKLNDTLRSKNSRAQF; KLNDTLRSKNSRAQFA; LNDTLRSKNSRAQFAN; NDTLRSKNSRAQFANI; DTLRSKNSRAQFANIH; TLRSKNSRAQFANIHD; LRSKNSRAQFANIHDI; RSKNSRAQFANIHDII; SKNSRAQFANIHDIIL; KNSRAQFANIHDIILD; NSRAQFANIHDIILDL; SRAQFANIHDIILDLV; RAQFANIHDIILDLVN; AQFANIHDIILDLVNT; QFANIHDIILDLVNTT; FANIHDIILDLVNTTT; ANIHDIILDLVNTTTN; NIHDIILDLVNTTTNI; IHDIILDLVNTTTNIL; HDIILDLVNTTTNILA; DIILDLVNTTTNILAP; IILDLVNTTTNILAPI; ILDLVNTTTNILAPIQ; |
|---|---|
| Seq 41 | |

13 mers:

MKNNKLIAIFLLH; KNNKLIAIFLLHI; NNKLIAIFLLHIL;
NKLIAIFLLHILT; KLIAIFLLHILTG; LIAIFLLHILTGL;
IAIFLLHILTGLI; AIFLLHILTGLIL; IFLLHILTGLILL;
FLLHILTGLILLS; LLHILTGLILLSC; LHILTGLILLSCS;
HILTGLILLSCSL; ILTGLILLSCSLE; LTGLILLSCSLEV;
TGLILLSCSLEVN; GLILLSCSLEVNQ; LILLSCSLEVNQD;
ILLSCSLEVNQDD; LLSCSLEVNQDDN; LSCSLEVNQDDNQ;
SCSLEVNQDDNQE; CSLEVNQDDNQEK; SLEVNQDDNQEKQ;
LEVNQDDNQEKQK; EVNQDDNQEKQKK; VNQDDNQEKQKKA;
NQDDNQEKQKKAK; QDDNQEKQKKAKT; DDNQEKQKKAKTK;
DNQEKQKKAKTKT; NQEKQKKAKTKTS; QEKQKKAKTKTSK;
EKQKKAKTKTSKS; KQKKAKTKTSKSE; QKKAKTKTSKSEN;
KKAKTKTSKSENN; KAKTKTSKSENNS; AKTKTSKSENNSS;
KTKTSKSENNSSK; TKTSKSENNSSKM; KTSKSENNSSKMK;
TSKSENNSSKMKK; SKSENNSSKMKKL; KSENNSSKMKKLS;
SENNSSKMKKLSK; ENNSSKMKKLSKN; NNSSKMKKLSKNA;
NSSKMKKLSKNAK; SSKMKKLSKNAKN; SKMKKLSKNAKNK;
KMKKLSKNAKNKK; MKKLSKNAKNKKP; KKLSKNAKNKKPT;
KLSKNAKNKKPTV; LSKNAKNKKPTVD; SKNAKNKKPTVDN;
KNAKNKKPTVDNL; NAKNKKPTVDNLL; AKNKKPTVDNLLV;
KNKKPTVDNLLVA; NKKPTVDNLLVAI; KKPTVDNLLVAIN;
KPTVDNLLVAINT; PTVDNLLVAINTL; TVDNLLVAINTLK;
VDNLLVAINTLKN; DNLLVAINTLKNP; NLLVAINTLKNPP;
LLVAINTLKNPPK; LVAINTLKNPPKT; VAINTLKNPPKTA;
AINTLKNPPKTAG; INTLKNPPKTAGK; NTLKNPPKTAGKN;
TLKNPPKTAGKNK; LKNPPKTAGKNKS; KNPPKTAGKNKSN;
NPPKTAGKNKSNS; PPKTAGKNKSNSA; PKTAGKNKSNSAA;
KTAGKNKSNSAAA; TAGKNKSNSAAAL; AGKNKSNSAAALK;
GKNKSNSAAALKQ; KNKSNSAAALKQP; NKSNSAAALKQPN;
KSNSAAALKQPNN; SNSAAALKQPNNA; NSAAALKQPNNAN;
SAAALKQPNNANA; AAALKQPNNANAL; AALKQPNNANALK;
ALKQPNNANALKQ; LKQPNNANALKQI; KQPNNANALKQID;
QPNNANALKQIDP; PNNANALKQIDPE; NNANALKQIDPEA;
NANALKQIDPEAK; ANALKQIDPEAKE; NALKQIDPEAKEL;
ALKQIDPEAKELI; LKQIDPEAKELIQ; KQIDPEAKELIQK;
QIDPEAKELIQKI; IDPEAKELIQKIL; DPEAKELIQKILE;
PEAKELIQKILER; EAKELIQKILERS; AKELIQKILERSE;
KELIQKILERSED; ELIQKILERSEDI; LIQKILERSEDIV;
IQKILERSEDIVQ; QKILERSEDIVQI; KILERSEDIVQIS;
ILERSEDIVQISE; LERSEDIVQISEI; ERSEDIVQISEID;
RSEDIVQISEIDA; SEDIVQISEIDAN; EDIVQISEIDANK;
DIVQISEIDANKG; IVQISEIDANKGE; VQISEIDANKGEP;
QISEIDANKGEPD; ISEIDANKGEPDD; SEIDANKGEPDDQ;
EIDANKGEPDDQF; IDANKGEPDDQFE; DANKGEPDDQFEM;
ANKGEPDDQFEMK; NKGEPDDQFEMKA; KGEPDDQFEMKAE;
GEPDDQFEMKAEI; EPDDQFEMKAEIF; PDDQFEMKAEIFS;
DDQFEMKAEIFSK; DQFEMKAEIFSKI; QFEMKAEIFSKIF;
FEMKAEIFSKIFF; EMKAEIFSKIFFN; MKAEIFSKIFFNA;
KAEIFSKIFFNAG; AEIFSKIFFNAGS; EIFSKIFFNAGST;
IFSKIFFNAGSTV; FSKIFFNAGSTVT; SKIFFNAGSTVTF;

| | | |
|---|---|---|
| KIFFNAGSTVTFD; | IFFNAGSTVTFDD; | FFNAGSTVTFDDN; |
| FNAGSTVTFDDNE; | NAGSTVTFDDNEY; | AGSTVTFDDNEYV; |
| GSTVTFDDNEYVN; | STVTFDDNEYVNE; | TVTFDDNEYVNER; |
| VTFDDNEYVNERR; | TFDDNEYVNERRI; | FDDNEYVNERRIL; |
| DDNEYVNERRILY; | DNEYVNERRILYT; | NEYVNERRILYTS; |
| EYVNERRILYTSL; | YVNERRILYTSLN; | VNERRILYTSLNF; |
| NERRILYTSLNFN; | ERRILYTSLNFNE; | RRILYTSLNFNEN; |
| RILYTSLNFNENK; | ILYTSLNFNENKI; | LYTSLNFNENKIL; |
| YTSLNFNENKILN; | TSLNFNENKILNL; | SLNFNENKILNLG; |
| LNFNENKILNLGK; | NFNENKILNLGKI; | FNENKILNLGKIL; |
| NENKILNLGKILS; | ENKILNLGKILSK; | NKILNLGKILSKL; |
| KILNLGKILSKLS; | ILNLGKILSKLSQ; | LNLGKILSKLSQD; |
| NLGKILSKLSQDS; | LGKILSKLSQDSN; | GKILSKLSQDSNY; |
| KILSKLSQDSNYR; | ILSKLSQDSNYRS; | LSKLSQDSNYRSL; |
| SKLSQDSNYRSLV; | KLSQDSNYRSLVK; | LSQDSNYRSLVKE; |
| SQDSNYRSLVKEI; | QDSNYRSLVKEIL; | DSNYRSLVKEILI; |
| SNYRSLVKEILIN; | NYRSLVKEILINR; | YRSLVKEILINRG; |
| RSLVKEILINRGF; | SLVKEILINRGFS; | LVKEILINRGFSI; |
| VKEILINRGFSIQ; | KEILINRGFSIQL; | EILINRGFSIQLA; |
| ILINRGFSIQLAI; | LINRGFSIQLAIE; | INRGFSIQLAIEE; |
| NRGFSIQLAIEEI; | RGFSIQLAIEEIS; | GFSIQLAIEEISL; |
| FSIQLAIEEISLR; | SIQLAIEEISLRT; | IQLAIEEISLRTL; |
| QLAIEEISLRTLN; | LAIEEISLRTLNV; | AIEEISLRTLNVK; |
| IEEISLRTLNVKD; | EEISLRTLNVKDK; | EISLRTLNVKDKI; |
| ISLRTLNVKDKIQ; | SLRTLNVKDKIQH; | LRTLNVKDKIQHL; |
| RTLNVKDKIQHLN; | TLNVKDKIQHLNK; | LNVKDKIQHLNKP; |
| NVKDKIQHLNKPN; | VKDKIQHLNKPNL; | KDKIQHLNKPNLK; |
| DKIQHLNKPNLKT; | KIQHLNKPNLKTL; | IQHLNKPNLKTLY; |
| QHLNKPNLKTLYH; | HLNKPNLKTLYHD; | LNKPNLKTLYHDF; |
| NKPNLKTLYHDFN; | KPNLKTLYHDFNK; | PNLKTLYHDFNKL; |
| NLKTLYHDFNKLI; | LKTLYHDFNKLIP; | KTLYHDFNKLIPL; |
| TLYHDFNKLIPLK; | LYHDFNKLIPLKE; | YHDFNKLIPLKEK; |
| HDFNKLIPLKEKW; | DFNKLIPLKEKWL; | FNKLIPLKEKWLK; |
| NKLIPLKEKWLKD; | KLIPLKEKWLKDV; | LIPLKEKWLKDVD; |
| IPLKEKWLKDVDD; | PLKEKWLKDVDDI; | LKEKWLKDVDDII; |
| KEKWLKDVDDIIK; | EKWLKDVDDIIKD; | KWLKDVDDIIKDY; |
| WLKDVDDIIKDYN; | LKDVDDIIKDYNA; | KDVDDIIKDYNAN; |
| DVDDIIKDYNANP; | VDDIIKDYNANPE; | DDIIKDYNANPEL; |
| DIIKDYNANPELR; | IIKDYNANPELRT; | IKDYNANPELRTD; |
| KDYNANPELRTDI; | DYNANPELRTDIS; | YNANPELRTDISK; |
| NANPELRTDISKL; | ANPELRTDISKLN; | NPELRTDISKLND; |
| PELRTDISKLNDY; | ELRTDISKLNDYI; | LRTDISKLNDYII; |
| RTDISKLNDYIIS; | TDISKLNDYIISK; | DISKLNDYIISKN; |
| ISKLNDYIISKNS; | SKLNDYIISKNSK; | KLNDYIISKNSKA; |
| LNDYIISKNSKAQ; | NDYIISKNSKAQF; | DYIISKNSKAQFT; |
| YIISKNSKAQFTD; | IISKNSKAQFTDI; | ISKNSKAQFTDIH; |
| SKNSKAQFTDIHN; | KNSKAQFTDIHNI; | NSKAQFTDIHNII; |
| SKAQFTDIHNIIL; | KAQFTDIHNIILN; | AQFTDIHNIILNL; |
| QFTDIHNIILNLI; | FTDIHNIILNLIN; | TDIHNIILNLINT; |
| DIHNIILNLINTT; | IHNIILNLINTTT; | HNIILNLINTTTN; |
| NIILNLINTTTNI; | IILNLINTTTNIL; | ILNLINTTTNILA; |

LNLINTTTNILAP; NLINTTTNILAPI; LINTTTNILAPIQ;

14 mers:
MKNNKLIAIFLLHI; KNNKLIAIFLLHIL; NNKLIAIFLLHILT;
NKLIAIFLLHILTG; KLIAIFLLHILTGL; LIAIFLLHILTGLI;
IAIFLLHILTGLIL; AIFLLHILTGLILL; IFLLHILTGLILLS;
FLLHILTGLILLSC; LLHILTGLILLSCS; LHILTGLILLSCSL;
HILTGLILLSCSLE; ILTGLILLSCSLEV; LTGLILLSCSLEVN;
TGLILLSCSLEVNQ; GLILLSCSLEVNQD; LILLSCSLEVNQDD;
ILLSCSLEVNQDDN; LLSCSLEVNQDDNQ; LSCSLEVNQDDNQE;
SCSLEVNQDDNQEK; CSLEVNQDDNQEKQ; SLEVNQDDNQEKQK;
LEVNQDDNQEKQKK; EVNQDDNQEKQKKA; VNQDDNQEKQKKAK;
NQDDNQEKQKKAKT; QDDNQEKQKKAKTK; DDNQEKQKKAKTKT;
DNQEKQKKAKTKTS; NQEKQKKAKTKTSK; QEKQKKAKTKTSKS;
EKQKKAKTKTSKSE; KQKKAKTKTSKSEN; QKKAKTKTSKSENN;
KKAKTKTSKSENNS; KAKTKTSKSENNSS; AKTKTSKSENNSSK;
KTKTSKSENNSSKM; TKTSKSENNSSKMK; KTSKSENNSSKMKK;
TSKSENNSSKMKKL; SKSENNSSKMKKLS; KSENNSSKMKKLSK;
SENNSSKMKKLSKN; ENNSSKMKKLSKNA; NNSSKMKKLSKNAK;
NSSKMKKLSKNAKN; SSKMKKLSKNAKNK; SKMKKLSKNAKNKK;
KMKKLSKNAKNKKP; MKKLSKNAKNKKPT; KKLSKNAKNKKPTV;
KLSKNAKNKKPTVD; LSKNAKNKKPTVDN; SKNAKNKKPTVDNL;
KNAKNKKPTVDNLL; NAKNKKPTVDNLLV; AKNKKPTVDNLLVA;
KNKKPTVDNLLVAI; NKKPTVDNLLVAIN; KKPTVDNLLVAINT;
KPTVDNLLVAINTL; PTVDNLLVAINTLK; TVDNLLVAINTLKN;
VDNLLVAINTLKNP; DNLLVAINTLKNPP; NLLVAINTLKNPPK;
LLVAINTLKNPPKT; LVAINTLKNPPKTA; VAINTLKNPPKTAG;
AINTLKNPPKTAGK; INTLKNPPKTAGKN; NTLKNPPKTAGKNK;
TLKNPPKTAGKNKS; LKNPPKTAGKNKSN; KNPPKTAGKNKSNS;
NPPKTAGKNKSNSA; PPKTAGKNKSNSAA; PKTAGKNKSNSAAA;
KTAGKNKSNSAAAL; TAGKNKSNSAAALK; AGKNKSNSAAALKQ;
GKNKSNSAAALKQP; KNKSNSAAALKQPN; NKSNSAAALKQPNN;
KSNSAAALKQPNNA; SNSAAALKQPNNAN; NSAAALKQPNNANA;
SAAALKQPNNANAL; AAALKQPNNANALK; AALKQPNNANALKQ;
ALKQPNNANALKQI; LKQPNNANALKQID; KQPNNANALKQIDP;
QPNNANALKQIDPE; PNNANALKQIDPEA; NNANALKQIDPEAK;
NANALKQIDPEAKE; ANALKQIDPEAKEL; NALKQIDPEAKELI;
ALKQIDPEAKELIQ; LKQIDPEAKELIQK; KQIDPEAKELIQKI;
QIDPEAKELIQKIL; IDPEAKELIQKILE; DPEAKELIQKILER;
PEAKELIQKILERS; EAKELIQKILERSE; AKELIQKILERSED;
KELIQKILERSEDI; ELIQKILERSEDIV; LIQKILERSEDIVQ;
IQKILERSEDIVQI; QKILERSEDIVQIS; KILERSEDIVQISE;
ILERSEDIVQISEI; LERSEDIVQISEID; ERSEDIVQISEIDA;
RSEDIVQISEIDAN; SEDIVQISEIDANK; EDIVQISEIDANKG;
DIVQISEIDANKGE; IVQISEIDANKGEP; VQISEIDANKGEPD;
QISEIDANKGEPDD; ISEIDANKGEPDDQ; SEIDANKGEPDDQF;
EIDANKGEPDDQFE; IDANKGEPDDQFEM; DANKGEPDDQFEMK;
ANKGEPDDQFEMKA; NKGEPDDQFEMKAE; KGEPDDQFEMKAEI;
GEPDDQFEMKAEIF; EPDDQFEMKAEIFS; PDDQFEMKAEIFSK;
DDQFEMKAEIFSKI; DQFEMKAEIFSKIF; QFEMKAEIFSKIFF;
FEMKAEIFSKIFFN; EMKAEIFSKIFFNA; MKAEIFSKIFFNAG;

KAEIFSKIFFNAGS; AEIFSKIFFNAGST; EIFSKIFFNAGSTV;
IFSKIFFNAGSTVT; FSKIFFNAGSTVTF; SKIFFNAGSTVTFD;
KIFFNAGSTVTFDD; IFFNAGSTVTFDDN; FFNAGSTVTFDDNE;
FNAGSTVTFDDNEY; NAGSTVTFDDNEYV; AGSTVTFDDNEYVN;
GSTVTFDDNEYVNE; STVTFDDNEYVNER; TVTFDDNEYVNERR;
VTFDDNEYVNERRI; TFDDNEYVNERRIL; FDDNEYVNERRILY;
DDNEYVNERRILYT; DNEYVNERRILYTS; NEYVNERRILYTSL;
EYVNERRILYTSLN; YVNERRILYTSLNF; VNERRILYTSLNFN;
NERRILYTSLNFNE; ERRILYTSLNFNEN; RRILYTSLNFNENK;
RILYTSLNFNENKI; ILYTSLNFNENKIL; LYTSLNFNENKILN;
YTSLNFNENKILNL; TSLNFNENKILNLG; SLNFNENKILNLGK;
LNFNENKILNLGKI; NFNENKILNLGKIL; FNENKILNLGKILS;
NENKILNLGKILSK; ENKILNLGKILSKL; NKILNLGKILSKLS;
KILNLGKILSKLSQ; ILNLGKILSKLSQD; LNLGKILSKLSQDS;
NLGKILSKLSQDSN; LGKILSKLSQDSNY; GKILSKLSQDSNYR;
KILSKLSQDSNYRS; ILSKLSQDSNYRSL; LSKLSQDSNYRSLV;
SKLSQDSNYRSLVK; KLSQDSNYRSLVKE; LSQDSNYRSLVKEI;
SQDSNYRSLVKEIL; QDSNYRSLVKEILI; DSNYRSLVKEILIN;
SNYRSLVKEILINR; NYRSLVKEILINRG; YRSLVKEILINRGF;
RSLVKEILINRGFS; SLVKEILINRGFSI; LVKEILINRGFSIQ;
VKEILINRGFSIQL; KEILINRGFSIQLA; EILINRGFSIQLAI;
ILINRGFSIQLAIE; LINRGFSIQLAIEE; INRGFSIQLAIEEI;
NRGFSIQLAIEEIS; RGFSIQLAIEEISL; GFSIQLAIEEISLR;
FSIQLAIEEISLRT; SIQLAIEEISLRTL; IQLAIEEISLRTLN;
QLAIEEISLRTLNV; LAIEEISLRTLNVK; AIEEISLRTLNVKD;
IEEISLRTLNVKDK; EEISLRTLNVKDKI; EISLRTLNVKDKIQ;
ISLRTLNVKDKIQH; SLRTLNVKDKIQHL; LRTLNVKDKIQHLN;
RTLNVKDKIQHLNK; TLNVKDKIQHLNKP; LNVKDKIQHLNKPN;
NVKDKIQHLNKPNL; VKDKIQHLNKPNLK; KDKIQHLNKPNLKT;
DKIQHLNKPNLKTL; KIQHLNKPNLKTLY; IQHLNKPNLKTLYH;
QHLNKPNLKTLYHD; HLNKPNLKTLYHDF; LNKPNLKTLYHDFN;
NKPNLKTLYHDFNK; KPNLKTLYHDFNKL; PNLKTLYHDFNKLI;
NLKTLYHDFNKLIP; LKTLYHDFNKLIPL; KTLYHDFNKLIPLK;
TLYHDFNKLIPLKE; LYHDFNKLIPLKEK; YHDFNKLIPLKEKW;
HDFNKLIPLKEKWL; DFNKLIPLKEKWLK; FNKLIPLKEKWLKD;
NKLIPLKEKWLKDV; KLIPLKEKWLKDVD; LIPLKEKWLKDVDD;
IPLKEKWLKDVDDI; PLKEKWLKDVDDII; LKEKWLKDVDDIIK;
KEKWLKDVDDIIKD; EKWLKDVDDIIKDY; KWLKDVDDIIKDYN;
WLKDVDDIIKDYNA; LKDVDDIIKDYNAN; KDVDDIIKDYNANP;
DVDDIIKDYNANPE; VDDIIKDYNANPEL; DDIIKDYNANPELR;
DIIKDYNANPELRT; IIKDYNANPELRTD; IKDYNANPELRTDI;
KDYNANPELRTDIS; DYNANPELRTDISK; YNANPELRTDISKL;
NANPELRTDISKLN; ANPELRTDISKLND; NPELRTDISKLNDY;
PELRTDISKLNDYI; ELRTDISKLNDYII; LRTDISKLNDYIIS;
RTDISKLNDYIISK; TDISKLNDYIISKN; DISKLNDYIISKNS;
ISKLNDYIISKNSK; SKLNDYIISKNSKA; KLNDYIISKNSKAQ;
LNDYIISKNSKAQF; NDYIISKNSKAQFT; DYIISKNSKAQFTD;
YIISKNSKAQFTDI; IISKNSKAQFTDIH; ISKNSKAQFTDIHN;
SKNSKAQFTDIHNI; KNSKAQFTDIHNII; NSKAQFTDIHNIIL;
SKAQFTDIHNIILN; KAQFTDIHNIILNL; AQFTDIHNIILNLI;
QFTDIHNIILNLIN; FTDIHNIILNLINT; TDIHNIILNLINTT;

DIHNIILNLINTTT; IHNIILNLINTTTN; HNIILNLINTTTNI;
NIILNLINTTTNIL; IILNLINTTTNILA; ILNLINTTTNILAP;
LNLINTTTNILAPI; NLINTTTNILAPIQ;

15 mers:
MKNNKLIAIFLLHIL; KNNKLIAIFLLHILT; NNKLIAIFLLHILTG;
NKLIAIFLLHILTGL; KLIAIFLLHILTGLI; LIAIFLLHILTGLIL;
IAIFLLHILTGLILL; AIFLLHILTGLILLS; IFLLHILTGLILLSC;
FLLHILTGLILLSCS; LLHILTGLILLSCSL; LHILTGLILLSCSLE;
HILTGLILLSCSLEV; ILTGLILLSCSLEVN; LTGLILLSCSLEVNQ;
TGLILLSCSLEVNQD; GLILLSCSLEVNQDD; LILLSCSLEVNQDDN;
ILLSCSLEVNQDDNQ; LLSCSLEVNQDDNQE; LSCSLEVNQDDNQEK;
SCSLEVNQDDNQEKQ; CSLEVNQDDNQEKQK; SLEVNQDDNQEKQKK;
LEVNQDDNQEKQKKA; EVNQDDNQEKQKKAK; VNQDDNQEKQKKAKT;
NQDDNQEKQKKAKTK; QDDNQEKQKKAKTKT; DDNQEKQKKAKTKTS;
DNQEKQKKAKTKTSK; NQEKQKKAKTKTSKS; QEKQKKAKTKTSKSE;
EKQKKAKTKTSKSEN; KQKKAKTKTSKSENN; QKKAKTKTSKSENNS;
KKAKTKTSKSENNSS; KAKTKTSKSENNSSK; AKTKTSKSENNSSKM;
KTKTSKSENNSSKMK; TKTSKSENNSSKMKK; KTSKSENNSSKMKKL;
TSKSENNSSKMKKLS; SKSENNSSKMKKLSK; KSENNSSKMKKLSKN;
SENNSSKMKKLSKNA; ENNSSKMKKLSKNAK; NNSSKMKKLSKNAKN;
NSSKMKKLSKNAKNK; SSKMKKLSKNAKNKK; SKMKKLSKNAKNKKP;
KMKKLSKNAKNKKPT; MKKLSKNAKNKKPTV; KKLSKNAKNKKPTVD;
KLSKNAKNKKPTVDN; LSKNAKNKKPTVDNL; SKNAKNKKPTVDNLL;
KNAKNKKPTVDNLLV; NAKNKKPTVDNLLVA; AKNKKPTVDNLLVAI;
KNKKPTVDNLLVAIN; NKKPTVDNLLVAINT; KKPTVDNLLVAINTL;
KPTVDNLLVAINTLK; PTVDNLLVAINTLKN; TVDNLLVAINTLKNP;
VDNLLVAINTLKNPP; DNLLVAINTLKNPPK; NLLVAINTLKNPPKT;
LLVAINTLKNPPKTA; LVAINTLKNPPKTAG; VAINTLKNPPKTAGK;
AINTLKNPPKTAGKN; INTLKNPPKTAGKNK; NTLKNPPKTAGKNKS;
TLKNPPKTAGKNKSN; LKNPPKTAGKNKSNS; KNPPKTAGKNKSNSA;
NPPKTAGKNKSNSAA; PPKTAGKNKSNSAAA; PKTAGKNKSNSAAAL;
KTAGKNKSNSAAALK; TAGKNKSNSAAALKQ; AGKNKSNSAAALKQP;
GKNKSNSAAALKQPN; KNKSNSAAALKQPNN; NKSNSAAALKQPNNA;
KSNSAAALKQPNNAN; SNSAAALKQPNNANA; NSAAALKQPNNANAL;
SAAALKQPNNANALK; AAALKQPNNANALKQ; AALKQPNNANALKQI;
ALKQPNNANALKQID; LKQPNNANALKQIDP; KQPNNANALKQIDPE;
QPNNANALKQIDPEA; PNNANALKQIDPEAK; NNANALKQIDPEAKE;
NANALKQIDPEAKEL; ANALKQIDPEAKELI; NALKQIDPEAKELIQ;
ALKQIDPEAKELIQK; LKQIDPEAKELIQKI; KQIDPEAKELIQKIL;
QIDPEAKELIQKILE; IDPEAKELIQKILER; DPEAKELIQKILERS;
PEAKELIQKILERSE; EAKELIQKILERSED; AKELIQKILERSEDI;
KELIQKILERSEDIV; ELIQKILERSEDIVQ; LIQKILERSEDIVQI;
IQKILERSEDIVQIS; QKILERSEDIVQISE; KILERSEDIVQISEI;
ILERSEDIVQISEID; LERSEDIVQISEIDA; ERSEDIVQISEIDAN;
RSEDIVQISEIDANK; SEDIVQISEIDANKG; EDIVQISEIDANKGE;
DIVQISEIDANKGEP; IVQISEIDANKGEPD; VQISEIDANKGEPDD;
QISEIDANKGEPDDQ; ISEIDANKGEPDDQF; SEIDANKGEPDDQFE;
EIDANKGEPDDQFEM; IDANKGEPDDQFEMK; DANKGEPDDQFEMKA;
ANKGEPDDQFEMKAE; NKGEPDDQFEMKAEI; KGEPDDQFEMKAEIF;
GEPDDQFEMKAEIFS; EPDDQFEMKAEIFSK; PDDQFEMKAEIFSKI;

DDQFEMKAEIFSKIF; DQFEMKAEIFSKIFF; QFEMKAEIFSKIFFN;
FEMKAEIFSKIFFNA; EMKAEIFSKIFFNAG; MKAEIFSKIFFNAGS;
KAEIFSKIFFNAGST; AEIFSKIFFNAGSTV; EIFSKIFFNAGSTVT;
IFSKIFFNAGSTVTF; FSKIFFNAGSTVTFD; SKIFFNAGSTVTFDD;
KIFFNAGSTVTFDDN; IFFNAGSTVTFDDNE; FFNAGSTVTFDDNEY;
FNAGSTVTFDDNEYV; NAGSTVTFDDNEYVN; AGSTVTFDDNEYVNE;
GSTVTFDDNEYVNER; STVTFDDNEYVNERR; TVTFDDNEYVNERRI;
VTFDDNEYVNERRIL; TFDDNEYVNERRILY; FDDNEYVNERRILYT;
DDNEYVNERRILYTS; DNEYVNERRILYTSL; NEYVNERRILYTSLN;
EYVNERRILYTSLNF; YVNERRILYTSLNFN; VNERRILYTSLNFNE;
NERRILYTSLNFNEN; ERRILYTSLNFNENK; RRILYTSLNFNENKI;
RILYTSLNFNENKIL; ILYTSLNFNENKILN; LYTSLNFNENKILNL;
YTSLNFNENKILNLG; TSLNFNENKILNLGK; SLNFNENKILNLGKI;
LNFNENKILNLGKIL; NFNENKILNLGKILS; FNENKILNLGKILSK;
NENKILNLGKILSKL; ENKILNLGKILSKLS; NKILNLGKILSKLSQ;
KILNLGKILSKLSQD; ILNLGKILSKLSQDS; LNLGKILSKLSQDSN;
NLGKILSKLSQDSNY; LGKILSKLSQDSNYR; GKILSKLSQDSNYRS;
KILSKLSQDSNYRSL; ILSKLSQDSNYRSLV; LSKLSQDSNYRSLVK;
SKLSQDSNYRSLVKE; KLSQDSNYRSLVKEI; LSQDSNYRSLVKEIL;
SQDSNYRSLVKEILI; QDSNYRSLVKEILIN; DSNYRSLVKEILINR;
SNYRSLVKEILINRG; NYRSLVKEILINRGF; YRSLVKEILINRGFS;
RSLVKEILINRGFSI; SLVKEILINRGFSIQ; LVKEILINRGFSIQL;
VKEILINRGFSIQLA; KEILINRGFSIQLAI; EILINRGFSIQLAIE;
ILINRGFSIQLAIEE; LINRGFSIQLAIEEI; INRGFSIQLAIEEIS;
NRGFSIQLAIEEISL; RGFSIQLAIEEISLR; GFSIQLAIEEISLRT;
FSIQLAIEEISLRTL; SIQLAIEEISLRTLN; IQLAIEEISLRTLNV;
QLAIEEISLRTLNVK; LAIEEISLRTLNVKD; AIEEISLRTLNVKDK;
IEEISLRTLNVKDKI; EEISLRTLNVKDKIQ; EISLRTLNVKDKIQH;
ISLRTLNVKDKIQHL; SLRTLNVKDKIQHLN; LRTLNVKDKIQHLNK;
RTLNVKDKIQHLNKP; TLNVKDKIQHLNKPN; LNVKDKIQHLNKPNL;
NVKDKIQHLNKPNLK; VKDKIQHLNKPNLKT; KDKIQHLNKPNLKTL;
DKIQHLNKPNLKTLY; KIQHLNKPNLKTLYH; IQHLNKPNLKTLYHD;
QHLNKPNLKTLYHDF; HLNKPNLKTLYHDFN; LNKPNLKTLYHDFNK;
NKPNLKTLYHDFNKL; KPNLKTLYHDFNKLI; PNLKTLYHDFNKLIP;
NLKTLYHDFNKLIPL; LKTLYHDFNKLIPLK; KTLYHDFNKLIPLKE;
TLYHDFNKLIPLKEK; LYHDFNKLIPLKEKW; YHDFNKLIPLKEKWL;
HDFNKLIPLKEKWLK; DFNKLIPLKEKWLKD; FNKLIPLKEKWLKDV;
NKLIPLKEKWLKDVD; KLIPLKEKWLKDVDD; LIPLKEKWLKDVDDI;
IPLKEKWLKDVDDII; PLKEKWLKDVDDIIK; LKEKWLKDVDDIIKD;
KEKWLKDVDDIIKDY; EKWLKDVDDIIKDYN; KWLKDVDDIIKDYNA;
WLKDVDDIIKDYNAN; LKDVDDIIKDYNANP; KDVDDIIKDYNANPE;
DVDDIIKDYNANPEL; VDDIIKDYNANPELR; DDIIKDYNANPELRT;
DIIKDYNANPELRTD; IIKDYNANPELRTDI; IKDYNANPELRTDIS;
KDYNANPELRTDISK; DYNANPELRTDISKL; YNANPELRTDISKLN;
NANPELRTDISKLND; ANPELRTDISKLNDY; NPELRTDISKLNDYI;
PELRTDISKLNDYII; ELRTDISKLNDYIIS; LRTDISKLNDYIISK;
RTDISKLNDYIISKN; TDISKLNDYIISKNS; DISKLNDYIISKNSK;
ISKLNDYIISKNSKA; SKLNDYIISKNSKAQ; KLNDYIISKNSKAQF;
LNDYIISKNSKAQFT; NDYIISKNSKAQFTD; DYIISKNSKAQFTDI;
YIISKNSKAQFTDIH; IISKNSKAQFTDIHN; ISKNSKAQFTDIHNI;
SKNSKAQFTDIHNII; KNSKAQFTDIHNIIL; NSKAQFTDIHNIILN;

SKAQFTDIHNIILNL; KAQFTDIHNIILNLI; AQFTDIHNIILNLIN;
QFTDIHNIILNLINT; FTDIHNIILNLINTT; TDIHNIILNLINTTT;
DIHNIILNLINTTTN; IHNIILNLINTTTNI; HNIILNLINTTTNIL;
NIILNLINTTTNILA; IILNLINTTTNILAP; ILNLINTTTNILAPI;
LNLINTTTNILAPIQ;

16 mers:
MKNNKLIAIFLLHILT; KNNKLIAIFLLHILTG; NNKLIAIFLLHILTGL;
NKLIAIFLLHILTGLI; KLIAIFLLHILTGLIL; LIAIFLLHILTGLILL;
IAIFLLHILTGLILLS; AIFLLHILTGLILLSC; IFLLHILTGLILLSCS;
FLLHILTGLILLSCSL; LLHILTGLILLSCSLE; LHILTGLILLSCSLEV;
HILTGLILLSCSLEVN; ILTGLILLSCSLEVNQ; LTGLILLSCSLEVNQD;
TGLILLSCSLEVNQDD; GLILLSCSLEVNQDDN; LILLSCSLEVNQDDNQ;
ILLSCSLEVNQDDNQE; LLSCSLEVNQDDNQEK; LSCSLEVNQDDNQEKQ;
SCSLEVNQDDNQEKQK; CSLEVNQDDNQEKQKK; SLEVNQDDNQEKQKKA;
LEVNQDDNQEKQKKAK; EVNQDDNQEKQKKAKT; VNQDDNQEKQKKAKTK;
NQDDNQEKQKKAKTKT; QDDNQEKQKKAKTKTS; DDNQEKQKKAKTKTSK;
DNQEKQKKAKTKTSKS; NQEKQKKAKTKTSKSE; QEKQKKAKTKTSKSEN;
EKQKKAKTKTSKSENN; KQKKAKTKTSKSENNS; QKKAKTKTSKSENNSS;
KKAKTKTSKSENNSSK; KAKTKTSKSENNSSKM; AKTKTSKSENNSSKMK;
KTKTSKSENNSSKMKK; TKTSKSENNSSKMKKL; KTSKSENNSSKMKKLS;
TSKSENNSSKMKKLSK; SKSENNSSKMKKLSKN; KSENNSSKMKKLSKNA;
SENNSSKMKKLSKNAK; ENNSSKMKKLSKNAKN; NNSSKMKKLSKNAKNK;
NSSKMKKLSKNAKNKK; SSKMKKLSKNAKNKKP; SKMKKLSKNAKNKKPT;
KMKKLSKNAKNKKPTV; MKKLSKNAKNKKPTVD; KKLSKNAKNKKPTVDN;
KLSKNAKNKKPTVDNL; LSKNAKNKKPTVDNLL; SKNAKNKKPTVDNLLV;
KNAKNKKPTVDNLLVA; NAKNKKPTVDNLLVAI; AKNKKPTVDNLLVAIN;
KNKKPTVDNLLVAINT; NKKPTVDNLLVAINTL; KKPTVDNLLVAINTLK;
KPTVDNLLVAINTLKN; PTVDNLLVAINTLKNP; TVDNLLVAINTLKNPP;
VDNLLVAINTLKNPPK; DNLLVAINTLKNPPKT; NLLVAINTLKNPPKTA;
LLVAINTLKNPPKTAG; LVAINTLKNPPKTAGK; VAINTLKNPPKTAGKN;
AINTLKNPPKTAGKNK; INTLKNPPKTAGKNKS; NTLKNPPKTAGKNKSN;
TLKNPPKTAGKNKSNS; LKNPPKTAGKNKSNSA; KNPPKTAGKNKSNSAA;
NPPKTAGKNKSNSAAA; PPKTAGKNKSNSAAAL; PKTAGKNKSNSAAALK;
KTAGKNKSNSAAALKQ; TAGKNKSNSAAALKQP; AGKNKSNSAAALKQPN;
GKNKSNSAAALKQPNN; KNKSNSAAALKQPNNA; NKSNSAAALKQPNNAN;
KSNSAAALKQPNNANA; SNSAAALKQPNNANAL; NSAAALKQPNNANALK;
SAAALKQPNNANALKQ; AAALKQPNNANALKQI; AALKQPNNANALKQID;
ALKQPNNANALKQIDP; LKQPNNANALKQIDPE; KQPNNANALKQIDPEA;
QPNNANALKQIDPEAK; PNNANALKQIDPEAKE; NNANALKQIDPEAKEL;
NANALKQIDPEAKELI; ANALKQIDPEAKELIQ; NALKQIDPEAKELIQK;
ALKQIDPEAKELIQKI; LKQIDPEAKELIQKIL; KQIDPEAKELIQKILE;
QIDPEAKELIQKILER; IDPEAKELIQKILERS; DPEAKELIQKILERSE;
PEAKELIQKILERSED; EAKELIQKILERSEDI; AKELIQKILERSEDIV;
KELIQKILERSEDIVQ; ELIQKILERSEDIVQI; LIQKILERSEDIVQIS;
IQKILERSEDIVQISE; QKILERSEDIVQISEI; KILERSEDIVQISEID;
ILERSEDIVQISEIDA; LERSEDIVQISEIDAN; ERSEDIVQISEIDANK;
RSEDIVQISEIDANKG; SEDIVQISEIDANKGE; EDIVQISEIDANKGEP;
DIVQISEIDANKGEPD; IVQISEIDANKGEPDD; VQISEIDANKGEPDDQ;
QISEIDANKGEPDDQF; ISEIDANKGEPDDQFE; SEIDANKGEPDDQFEM;
EIDANKGEPDDQFEMK; IDANKGEPDDQFEMKA; DANKGEPDDQFEMKAE;

| | | |
|---|---|---|
| ANKGEPDDQFEMKAEI; | NKGEPDDQFEMKAEIF; | KGEPDDQFEMKAEIFS; |
| GEPDDQFEMKAEIFSK; | EPDDQFEMKAEIFSKI; | PDDQFEMKAEIFSKIF; |
| DDQFEMKAEIFSKIFF; | DQFEMKAEIFSKIFFN; | QFEMKAEIFSKIFFNA; |
| FEMKAEIFSKIFFNAG; | EMKAEIFSKIFFNAGS; | MKAEIFSKIFFNAGST; |
| KAEIFSKIFFNAGSTV; | AEIFSKIFFNAGSTVT; | EIFSKIFFNAGSTVTF; |
| IFSKIFFNAGSTVTFD; | FSKIFFNAGSTVTFDD; | SKIFFNAGSTVTFDDN; |
| KIFFNAGSTVTFDDNE; | IFFNAGSTVTFDDNEY; | FFNAGSTVTFDDNEYV; |
| FNAGSTVTFDDNEYVN; | NAGSTVTFDDNEYVNE; | AGSTVTFDDNEYVNER; |
| GSTVTFDDNEYVNERR; | STVTFDDNEYVNERRI; | TVTFDDNEYVNERRIL; |
| VTFDDNEYVNERRILY; | TFDDNEYVNERRILYT; | FDDNEYVNERRILYTS; |
| DDNEYVNERRILYTSL; | DNEYVNERRILYTSLN; | NEYVNERRILYTSLNF; |
| EYVNERRILYTSLNFN; | YVNERRILYTSLNFNE; | VNERRILYTSLNFNEN; |
| NERRILYTSLNFNENK; | ERRILYTSLNFNENKI; | RRILYTSLNFNENKIL; |
| RILYTSLNFNENKILN; | ILYTSLNFNENKILNL; | LYTSLNFNENKILNLG; |
| YTSLNFNENKILNLGK; | TSLNFNENKILNLGKI; | SLNFNENKILNLGKIL; |
| LNFNENKILNLGKILS; | NFNENKILNLGKILSK; | FNENKILNLGKILSKL; |
| NENKILNLGKILSKLS; | ENKILNLGKILSKLSQ; | NKILNLGKILSKLSQD; |
| KILNLGKILSKLSQDS; | ILNLGKILSKLSQDSN; | LNLGKILSKLSQDSNY; |
| NLGKILSKLSQDSNYR; | LGKILSKLSQDSNYRS; | GKILSKLSQDSNYRSL; |
| KILSKLSQDSNYRSLV; | ILSKLSQDSNYRSLVK; | LSKLSQDSNYRSLVKE; |
| SKLSQDSNYRSLVKEI; | KLSQDSNYRSLVKEIL; | LSQDSNYRSLVKEILI; |
| SQDSNYRSLVKEILIN; | QDSNYRSLVKEILINR; | DSNYRSLVKEILINRG; |
| SNYRSLVKEILINRGF; | NYRSLVKEILINRGFS; | YRSLVKEILINRGFSI; |
| RSLVKEILINRGFSIQ; | SLVKEILINRGFSIQL; | LVKEILINRGFSIQLA; |
| VKEILINRGFSIQLAI; | KEILINRGFSIQLAIE; | EILINRGFSIQLAIEE; |
| ILINRGFSIQLAIEEI; | LINRGFSIQLAIEEIS; | INRGFSIQLAIEEISL; |
| NRGFSIQLAIEEISLR; | RGFSIQLAIEEISLRT; | GFSIQLAIEEISLRTL; |
| FSIQLAIEEISLRTLN; | SIQLAIEEISLRTLNV; | IQLAIEEISLRTLNVK; |
| QLAIEEISLRTLNVKD; | LAIEEISLRTLNVKDK; | AIEEISLRTLNVKDKI; |
| IEEISLRTLNVKDKIQ; | EEISLRTLNVKDKIQH; | EISLRTLNVKDKIQHL; |
| ISLRTLNVKDKIQHLN; | SLRTLNVKDKIQHLNK; | LRTLNVKDKIQHLNKP; |
| RTLNVKDKIQHLNKPN; | TLNVKDKIQHLNKPNL; | LNVKDKIQHLNKPNLK; |
| NVKDKIQHLNKPNLKT; | VKDKIQHLNKPNLKTL; | KDKIQHLNKPNLKTLY; |
| DKIQHLNKPNLKTLYH; | KIQHLNKPNLKTLYHD; | IQHLNKPNLKTLYHDF; |
| QHLNKPNLKTLYHDFN; | HLNKPNLKTLYHDFNK; | LNKPNLKTLYHDFNKL; |
| NKPNLKTLYHDFNKLI; | KPNLKTLYHDFNKLIP; | PNLKTLYHDFNKLIPL; |
| NLKTLYHDFNKLIPLK; | LKTLYHDFNKLIPLKE; | KTLYHDFNKLIPLKEK; |
| TLYHDFNKLIPLKEKW; | LYHDFNKLIPLKEKWL; | YHDFNKLIPLKEKWLK; |
| HDFNKLIPLKEKWLKD; | DFNKLIPLKEKWLKDV; | FNKLIPLKEKWLKDVD; |
| NKLIPLKEKWLKDVDD; | KLIPLKEKWLKDVDDI; | LIPLKEKWLKDVDDII; |
| IPLKEKWLKDVDDIIK; | PLKEKWLKDVDDIIKD; | LKEKWLKDVDDIIKDY; |
| KEKWLKDVDDIIKDYN; | EKWLKDVDDIIKDYNA; | KWLKDVDDIIKDYNAN; |
| WLKDVDDIIKDYNANP; | LKDVDDIIKDYNANPE; | KDVDDIIKDYNANPEL; |
| DVDDIIKDYNANPELR; | VDDIIKDYNANPELRT; | DDIIKDYNANPELRTD; |
| DIIKDYNANPELRTDI; | IIKDYNANPELRTDIS; | IKDYNANPELRTDISK; |
| KDYNANPELRTDISKL; | DYNANPELRTDISKLN; | YNANPELRTDISKLND; |
| NANPELRTDISKLNDY; | ANPELRTDISKLNDYI; | NPELRTDISKLNDYII; |
| PELRTDISKLNDYIIS; | ELRTDISKLNDYIISK; | LRTDISKLNDYIISKN; |
| RTDISKLNDYIISKNS; | TDISKLNDYIISKNSK; | DISKLNDYIISKNSKA; |
| ISKLNDYIISKNSKAQ; | SKLNDYIISKNSKAQF; | KLNDYIISKNSKAQFT; |
| LNDYIISKNSKAQFTD; | NDYIISKNSKAQFTDI; | DYIISKNSKAQFTDIH; |

| | |
|---|---|
| | YIISKNSKAQFTDIHN; IISKNSKAQFTDIHNI; ISKNSKAQFTDIHNII; SKNSKAQFTDIHNIIL; KNSKAQFTDIHNIILN; NSKAQFTDIHNIILNL; SKAQFTDIHNIILNLI; KAQFTDIHNIILNLIN; AQFTDIHNIILNLINT; QFTDIHNIILNLINTT; FTDIHNIILNLINTTT; TDIHNIILNLINTTTN; DIHNIILNLINTTTNI; IHNIILNLINTTTNIL; HNIILNLINTTTNILA; NIILNLINTTTNILAP; IILNLINTTTNILAPI; ILNLINTTTNILAPIQ; |
| Seq 42 | 13 mers:<br>MKKVKSKYLALGL; KKVKSKYLALGLL; KVKSKYLALGLLF; VKSKYLALGLLFG; KSKYLALGLLFGF; SKYLALGLLFGFI; KYLALGLLFGFIS; YLALGLLFGFISC; LALGLLFGFISCD; ALGLLFGFISCDL; LGLLFGFISCDLF; GLLFGFISCDLFI; LLFGFISCDLFIR; LFGFISCDLFIRY; FGFISCDLFIRYE; GFISCDLFIRYEM; FISCDLFIRYEMK; ISCDLFIRYEMKE; SCDLFIRYEMKEE; CDLFIRYEMKEES; DLFIRYEMKEESP; LFIRYEMKEESPG; FIRYEMKEESPGL; IRYEMKEESPGLF; RYEMKEESPGLFD; YEMKEESPGLFDK; EMKEESPGLFDKG; MKEESPGLFDKGN; KEESPGLFDKGNS; EESPGLFDKGNSI; ESPGLFDKGNSIL; SPGLFDKGNSILE; PGLFDKGNSILET; GLFDKGNSILETS; LFDKGNSILETSE; FDKGNSILETSEE; DKGNSILETSEES; KGNSILETSEESI; GNSILETSEESIK; NSILETSEESIKK; SILETSEESIKKP; ILETSEESIKKPM; LETSEESIKKPMN; ETSEESIKKPMNK; TSEESIKKPMNKK; SEESIKKPMNKKG; EESIKKPMNKKGK; ESIKKPMNKKGKG; SIKKPMNKKGKGK; IKKPMNKKGKGKI; KKPMNKKGKGKIA; KPMNKKGKGKIAR; PMNKKGKGKIARK; MNKKGKGKIARKK; NKKGKGKIARKKG; KKGKGKIARKKGK; KGKGKIARKKGKS; GKGKIARKKGKSK; KGKIARKKGKSKV; GKIARKKGKSKVS; KIARKKGKSKVSR; IARKKGKSKVSRK; ARKKGKSKVSRKE; RKKGKSKVSRKEP; KKGKSKVSRKEPY; KGKSKVSRKEPYI; GKSKVSRKEPYIH; KSKVSRKEPYIHS; SKVSRKEPYIHSL; KVSRKEPYIHSLK; VSRKEPYIHSLKR; SRKEPYIHSLKRD; RKEPYIHSLKRDS; KEPYIHSLKRDSA; EPYIHSLKRDSAN; PYIHSLKRDSANK; YIHSLKRDSANKS; IHSLKRDSANKSN; HSLKRDSANKSNF; SLKRDSANKSNFL; LKRDSANKSNFLQ; KRDSANKSNFLQK; RDSANKSNFLQKN; DSANKSNFLQKNV; SANKSNFLQKNVI; ANKSNFLQKNVIL; NKSNFLQKNVILE; KSNFLQKNVILEE; SNFLQKNVILEEE; NFLQKNVILEEES; FLQKNVILEEESL; LQKNVILEEESLK; QKNVILEEESLKT; KNVILEEESLKTE; NVILEEESLKTEL; VILEEESLKTELL; ILEEESLKTELLK; LEEESLKTELLKE; EEESLKTELLKEQ; EESLKTELLKEQS; ESLKTELLKEQSE; SLKTELLKEQSET; LKTELLKEQSETR; KTELLKEQSETRK; TELLKEQSETRKE; ELLKEQSETRKEK; LLKEQSETRKEKI; LKEQSETRKEKIQ; KEQSETRKEKIQK; EQSETRKEKIQKQ; QSETRKEKIQKQQ; SETRKEKIQKQQD; ETRKEKIQKQQDE; TRKEKIQKQQDEY; RKEKIQKQQDEYK; KEKIQKQQDEYKG; EKIQKQQDEYKGM; KIQKQQDEYKGMT; IQKQQDEYKGMTQ; QKQQDEYKGMTQG; KQQDEYKGMTQGS; QQDEYKGMTQGSL; QDEYKGMTQGSLN; DEYKGMTQGSLNS; EYKGMTQGSLNSL; YKGMTQGSLNSLS; |

KGMTQGSLNSLSG; GMTQGSLNSLSGE; MTQGSLNSLSGES;
TQGSLNSLSGESG; QGSLNSLSGESGE; GSLNSLSGESGEL;
SLNSLSGESGELK; LNSLSGESGELKE; NSLSGESGELKET;
SLSGESGELKETI; LSGESGELKETIE; SGESGELKETIES;
GESGELKETIESN; ESGELKETIESNE; SGELKETIESNEI;
GELKETIESNEID; ELKETIESNEIDI; LKETIESNEIDIT;
KETIESNEIDITI; ETIESNEIDITID; TIESNEIDITIDS;
IESNEIDITIDSD; ESNEIDITIDSDL; SNEIDITIDSDLR;
NEIDITIDSDLRP; EIDITIDSDLRPK; IDITIDSDLRPKS;
DITIDSDLRPKSS; ITIDSDLRPKSSL; TIDSDLRPKSSLQ;
IDSDLRPKSSLQD; DSDLRPKSSLQDI; SDLRPKSSLQDIA;
DLRPKSSLQDIAG; LRPKSSLQDIAGS; RPKSSLQDIAGSN;
PKSSLQDIAGSNS; KSSLQDIAGSNSI; SSLQDIAGSNSIS;
SLQDIAGSNSISY; LQDIAGSNSISYT; QDIAGSNSISYTD;
DIAGSNSISYTDE; IAGSNSISYTDEI; AGSNSISYTDEIE;
GSNSISYTDEIEE; SNSISYTDEIEEE; NSISYTDEIEEED;
SISYTDEIEEEDY; ISYTDEIEEEDYA; SYTDEIEEEDYAR;
YTDEIEEEDYARY; TDEIEEEDYARYY; DEIEEEDYARYYL;
EIEEEDYARYYLD; IEEEDYARYYLDE; EEEDYARYYLDED;
EEDYARYYLDEDD; EDYARYYLDEDDE; DYARYYLDEDDED;
YARYYLDEDDEDD; ARYYLDEDDEDDE; RYYLDEDDEDDEY;
YYLDEDDEDDEYY; YLDEDDEDDEYYE; LDEDDEDDEYYED;
DEDDEDDEYYEDD; EDDEDDEYYEDDY; DDEDDEYYEDDYE;
DEDDEYYEDDYEE; EDDEYYEDDYEEI; DDEYYEDDYEEIR;
DEYYEDDYEEIRL; EYYEDDYEEIRLS; YYEDDYEEIRLSN;
YEDDYEEIRLSNR; EDDYEEIRLSNRY; DDYEEIRLSNRYQ;
DYEEIRLSNRYQS; YEEIRLSNRYQSY; EEIRLSNRYQSYL;
EIRLSNRYQSYLE; IRLSNRYQSYLEG; RLSNRYQSYLEGV;
LSNRYQSYLEGVK; SNRYQSYLEGVKY; NRYQSYLEGVKYN;
RYQSYLEGVKYNV; YQSYLEGVKYNVD; QSYLEGVKYNVDS;
SYLEGVKYNVDSA; YLEGVKYNVDSAI; LEGVKYNVDSAIN;
EGVKYNVDSAINT; GVKYNVDSAINTI; VKYNVDSAINTIN;
KYNVDSAINTINK; YNVDSAINTINKI; NVDSAINTINKIY;
VDSAINTINKIYD; DSAINTINKIYDT; SAINTINKIYDTY;
AINTINKIYDTYT; INTINKIYDTYTL; NTINKIYDTYTLF;
TINKIYDTYTLFS; INKIYDTYTLFST; NKIYDTYTLFSTK;
KIYDTYTLFSTKL; IYDTYTLFSTKLT; YDTYTLFSTKLTQ;
DTYTLFSTKLTQM; TYTLFSTKLTQMY; YTLFSTKLTQMYS;
TLFSTKLTQMYST; LFSTKLTQMYSTR; FSTKLTQMYSTRL;
STKLTQMYSTRLD; TKLTQMYSTRLDN; KLTQMYSTRLDNL;
LTQMYSTRLDNLA; TQMYSTRLDNLAK; QMYSTRLDNLAKA;
MYSTRLDNLAKAK; YSTRLDNLAKAKA; STRLDNLAKAKAK;
TRLDNLAKAKAKE; RLDNLAKAKAKEE; LDNLAKAKAKEEA;
DNLAKAKAKEEAA; NLAKAKAKEEAAK; LAKAKAKEEAAKF;
AKAKAKEEAAKFT; KAKAKEEAAKFTK; AKAKEEAAKFTKE;
KAKEEAAKFTKED; AKEEAAKFTKEDL; KEEAAKFTKEDLE;
EEAAKFTKEDLEK; EAAKFTKEDLEKN; AAKFTKEDLEKNF;
AKFTKEDLEKNFK; KFTKEDLEKNFKT; FTKEDLEKNFKTL;
TKEDLEKNFKTLL; KEDLEKNFKTLLN; EDLEKNFKTLLNY;
DLEKNFKTLLNYI; LEKNFKTLLNYIQ; EKNFKTLLNYIQV;
KNFKTLLNYIQVS; NFKTLLNYIQVSV; FKTLLNYIQVSVK;

KTLLNYIQVSVKT; TLLNYIQVSVKTA; LLNYIQVSVKTAA;
LNYIQVSVKTAAN; NYIQVSVKTAANF; YIQVSVKTAANFV;
IQVSVKTAANFVY; QVSVKTAANFVYI; VSVKTAANFVYIN;
SVKTAANFVYIND; VKTAANFVYINDT; KTAANFVYINDTH;
TAANFVYINDTHA; AANFVYINDTHAK; ANFVYINDTHAKR;
NFVYINDTHAKRK; FVYINDTHAKRKL; VYINDTHAKRKLE;
YINDTHAKRKLEN; INDTHAKRKLENI; NDTHAKRKLENIE;
DTHAKRKLENIEA; THAKRKLENIEAE; HAKRKLENIEAEI;
AKRKLENIEAEIK; KRKLENIEAEIKT; RKLENIEAEIKTL;
KLENIEAEIKTLI; LENIEAEIKTLIA; ENIEAEIKTLIAK;
NIEAEIKTLIAKI; IEAEIKTLIAKIK; EAEIKTLIAKIKE;
AEIKTLIAKIKEQ; EIKTLIAKIKEQS; IKTLIAKIKEQSN;
KTLIAKIKEQSNL; TLIAKIKEQSNLY; LIAKIKEQSNLYE;
IAKIKEQSNLYEA; AKIKEQSNLYEAY; KIKEQSNLYEAYK;
IKEQSNLYEAYKA; KEQSNLYEAYKAI; EQSNLYEAYKAIV;
QSNLYEAYKAIVT; SNLYEAYKAIVTS; NLYEAYKAIVTSI;
LYEAYKAIVTSIL; YEAYKAIVTSILL; EAYKAIVTSILLM;
AYKAIVTSILLMR; YKAIVTSILLMRD; KAIVTSILLMRDS;
AIVTSILLMRDSL; IVTSILLMRDSLK; VTSILLMRDSLKE;
TSILLMRDSLKEV; SILLMRDSLKEVQ; ILLMRDSLKEVQG;
LLMRDSLKEVQGI; LMRDSLKEVQGII; MRDSLKEVQGIID;
RDSLKEVQGIIDK; DSLKEVQGIIDKN; SLKEVQGIIDKNG;
LKEVQGIIDKNGV; KEVQGIIDKNGVW; EVQGIIDKNGVWY;

14 mers:
MKKVKSKYLALGLL; KKVKSKYLALGLLF; KVKSKYLALGLLFG;
VKSKYLALGLLFGF; KSKYLALGLLFGFI; SKYLALGLLFGFIS;
KYLALGLLFGFISC; YLALGLLFGFISCD; LALGLLFGFISCDL;
ALGLLFGFISCDLF; LGLLFGFISCDLFI; GLLFGFISCDLFIR;
LLFGFISCDLFIRY; LFGFISCDLFIRYE; FGFISCDLFIRYEM;
GFISCDLFIRYEMK; FISCDLFIRYEMKE; ISCDLFIRYEMKEE;
SCDLFIRYEMKEES; CDLFIRYEMKEESP; DLFIRYEMKEESPG;
LFIRYEMKEESPGL; FIRYEMKEESPGLF; IRYEMKEESPGLFD;
RYEMKEESPGLFDK; YEMKEESPGLFDKG; EMKEESPGLFDKGN;
MKEESPGLFDKGNS; KEESPGLFDKGNSI; EESPGLFDKGNSIL;
ESPGLFDKGNSILE; SPGLFDKGNSILET; PGLFDKGNSILETS;
GLFDKGNSILETSE; LFDKGNSILETSEE; FDKGNSILETSEES;
DKGNSILETSEESI; KGNSILETSEESIK; GNSILETSEESIKK;
NSILETSEESIKKP; SILETSEESIKKPM; ILETSEESIKKPMN;
LETSEESIKKPMNK; ETSEESIKKPMNKK; TSEESIKKPMNKKG;
SEESIKKPMNKKGK; EESIKKPMNKKGKG; ESIKKPMNKKGKGK;
SIKKPMNKKGKGKI; IKKPMNKKGKGKIA; KKPMNKKGKGKIAR;
KPMNKKGKGKIARK; PMNKKGKGKIARKK; MNKKGKGKIARKKG;
NKKGKGKIARKKGK; KKGKGKIARKKGKS; KGKGKIARKKGKSK;
GKGKIARKKGKSKV; KGKIARKKGKSKVS; GKIARKKGKSKVSR;
KIARKKGKSKVSRK; IARKKGKSKVSRKE; ARKKGKSKVSRKEP;
RKKGKSKVSRKEPY; KKGKSKVSRKEPYI; KGKSKVSRKEPYIH;
GKSKVSRKEPYIHS; KSKVSRKEPYIHSL; SKVSRKEPYIHSLK;
KVSRKEPYIHSLKR; VSRKEPYIHSLKRD; SRKEPYIHSLKRDS;
RKEPYIHSLKRDSA; KEPYIHSLKRDSAN; EPYIHSLKRDSANK;
PYIHSLKRDSANKS; YIHSLKRDSANKSN; IHSLKRDSANKSNF;

| | |
|---|---|
| HSLKRDSANKSNFL; SLKRDSANKSNFLQ; LKRDSANKSNFLQK; | |
| KRDSANKSNFLQKN; RDSANKSNFLQKNV; DSANKSNFLQKNVI; | |
| SANKSNFLQKNVIL; ANKSNFLQKNVILE; NKSNFLQKNVILEE; | |
| KSNFLQKNVILEEE; SNFLQKNVILEEES; NFLQKNVILEEESL; | |
| FLQKNVILEEESLK; LQKNVILEEESLKT; QKNVILEEESLKTE; | |
| KNVILEEESLKTEL; NVILEEESLKTELL; VILEEESLKTELLK; | |
| ILEEESLKTELLKE; LEEESLKTELLKEQ; EEESLKTELLKEQS; | |
| EESLKTELLKEQSE; ESLKTELLKEQSET; SLKTELLKEQSETR; | |
| LKTELLKEQSETRK; KTELLKEQSETRKE; TELLKEQSETRKEK; | |
| ELLKEQSETRKEKI; LLKEQSETRKEKIQ; LKEQSETRKEKIQK; | |
| KEQSETRKEKIQKQ; EQSETRKEKIQKQQ; QSETRKEKIQKQQD; | |
| SETRKEKIQKQQDE; ETRKEKIQKQQDEY; TRKEKIQKQQDEYK; | |
| RKEKIQKQQDEYKG; KEKIQKQQDEYKGM; EKIQKQQDEYKGMT; | |
| KIQKQQDEYKGMTQ; IQKQQDEYKGMTQG; QKQQDEYKGMTQGS; | |
| KQQDEYKGMTQGSL; QQDEYKGMTQGSLN; QDEYKGMTQGSLNS; | |
| DEYKGMTQGSLNSL; EYKGMTQGSLNSLS; YKGMTQGSLNSLSG; | |
| KGMTQGSLNSLSGE; GMTQGSLNSLSGES; MTQGSLNSLSGESG; | |
| TQGSLNSLSGESGE; QGSLNSLSGESGEL; GSLNSLSGESGELK; | |
| SLNSLSGESGELKE; LNSLSGESGELKET; NSLSGESGELKETI; | |
| SLSGESGELKETIE; LSGESGELKETIES; SGESGELKETIESN; | |
| GESGELKETIESNE; ESGELKETIESNEI; SGELKETIESNEID; | |
| GELKETIESNEIDI; ELKETIESNEIDIT; LKETIESNEIDITI; | |
| KETIESNEIDITID; ETIESNEIDITIDS; TIESNEIDITIDSD; | |
| IESNEIDITIDSDL; ESNEIDITIDSDLR; SNEIDITIDSDLRP; | |
| NEIDITIDSDLRPK; EIDITIDSDLRPKS; IDITIDSDLRPKSS; | |
| DITIDSDLRPKSSL; ITIDSDLRPKSSLQ; TIDSDLRPKSSLQD; | |
| IDSDLRPKSSLQDI; DSDLRPKSSLQDIA; SDLRPKSSLQDIAG; | |
| DLRPKSSLQDIAGS; LRPKSSLQDIAGSN; RPKSSLQDIAGSNS; | |
| PKSSLQDIAGSNSI; KSSLQDIAGSNSIS; SSLQDIAGSNSISY; | |
| SLQDIAGSNSISYT; LQDIAGSNSISYTD; QDIAGSNSISYTDE; | |
| DIAGSNSISYTDEI; IAGSNSISYTDEIE; AGSNSISYTDEIEE; | |
| GSNSISYTDEIEEE; SNSISYTDEIEEED; NSISYTDEIEEEDY; | |
| SISYTDEIEEEDYA; ISYTDEIEEEDYAR; SYTDEIEEEDYARY; | |
| YTDEIEEEDYARYY; TDEIEEEDYARYYL; DEIEEEDYARYYLD; | |
| EIEEEDYARYYLDE; IEEEDYARYYLDED; EEEDYARYYLDEDD; | |
| EEDYARYYLDEDDE; EDYARYYLDEDDED; DYARYYLDEDDEDD; | |
| YARYYLDEDDEDDE; ARYYLDEDDEDDEY; RYYLDEDDEDDEYY; | |
| YYLDEDDEDDEYYE; YLDEDDEDDEYYED; LDEDDEDDEYYEDD; | |
| DEDDEDDEYYEDDY; EDDEDDEYYEDDYE; DDEDDEYYEDDYEE; | |
| DEDDEYYEDDYEEI; EDDEYYEDDYEEIR; DDEYYEDDYEEIRL; | |
| DEYYEDDYEEIRLS; EYYEDDYEEIRLSN; YYEDDYEEIRLSNR; | |
| YEDDYEEIRLSNRY; EDDYEEIRLSNRYQ; DDYEEIRLSNRYQS; | |
| DYEEIRLSNRYQSY; YEEIRLSNRYQSYL; EEIRLSNRYQSYLE; | |
| EIRLSNRYQSYLEG; IRLSNRYQSYLEGV; RLSNRYQSYLEGVK; | |
| LSNRYQSYLEGVKY; SNRYQSYLEGVKYN; NRYQSYLEGVKYNV; | |
| RYQSYLEGVKYNVD; YQSYLEGVKYNVDS; QSYLEGVKYNVDSA; | |
| SYLEGVKYNVDSAI; YLEGVKYNVDSAIN; LEGVKYNVDSAINT; | |
| EGVKYNVDSAINTI; GVKYNVDSAINTIN; VKYNVDSAINTINK; | |
| KYNVDSAINTINKI; YNVDSAINTINKIY; NVDSAINTINKIYD; | |
| VDSAINTINKIYDT; DSAINTINKIYDTY; SAINTINKIYDTYT; | |
| AINTINKIYDTYTL; INTINKIYDTYTLF; NTINKIYDTYTLFS; | |

```
TINKIYDTYTLFST;   INKIYDTYTLFSTK;   NKIYDTYTLFSTKL;
KIYDTYTLFSTKLT;   IYDTYTLFSTKLTQ;   YDTYTLFSTKLTQM;
DTYTLFSTKLTQMY;   TYTLFSTKLTQMYS;   YTLFSTKLTQMYST;
TLFSTKLTQMYSTR;   LFSTKLTQMYSTRL;   FSTKLTQMYSTRLD;
STKLTQMYSTRLDN;   TKLTQMYSTRLDNL;   KLTQMYSTRLDNLA;
LTQMYSTRLDNLAK;   TQMYSTRLDNLAKA;   QMYSTRLDNLAKAK;
MYSTRLDNLAKAKA;   YSTRLDNLAKAKAK;   STRLDNLAKAKAKE;
TRLDNLAKAKAKEE;   RLDNLAKAKAKEEA;   LDNLAKAKAKEEAA;
DNLAKAKAKEEAAK;   NLAKAKAKEEAAKF;   LAKAKAKEEAAKFT;
AKAKAKEEAAKFTK;   KAKAKEEAAKFTKE;   AKAKEEAAKFTKED;
KAKEEAAKFTKEDL;   AKEEAAKFTKEDLE;   KEEAAKFTKEDLEK;
EEAAKFTKEDLEKN;   EAAKFTKEDLEKNF;   AAKFTKEDLEKNFK;
AKFTKEDLEKNFKT;   KFTKEDLEKNFKTL;   FTKEDLEKNFKTLL;
TKEDLEKNFKTLLN;   KEDLEKNFKTLLNY;   EDLEKNFKTLLNYI;
DLEKNFKTLLNYIQ;   LEKNFKTLLNYIQV;   EKNFKTLLNYIQVS;
KNFKTLLNYIQVSV;   NFKTLLNYIQVSVK;   FKTLLNYIQVSVKT;
KTLLNYIQVSVKTA;   TLLNYIQVSVKTAA;   LLNYIQVSVKTAAN;
LNYIQVSVKTAANF;   NYIQVSVKTAANFV;   YIQVSVKTAANFVY;
IQVSVKTAANFVYI;   QVSVKTAANFVYIN;   VSVKTAANFVYIND;
SVKTAANFVYINDT;   VKTAANFVYINDTH;   KTAANFVYINDTHA;
TAANFVYINDTHAK;   AANFVYINDTHAKR;   ANFVYINDTHAKRK;
NFVYINDTHAKRKL;   FVYINDTHAKRKLE;   VYINDTHAKRKLEN;
YINDTHAKRKLENI;   INDTHAKRKLENIE;   NDTHAKRKLENIEA;
DTHAKRKLENIEAE;   THAKRKLENIEAEI;   HAKRKLENIEAEIK;
AKRKLENIEAEIKT;   KRKLENIEAEIKTL;   RKLENIEAEIKTLI;
KLENIEAEIKTLIA;   LENIEAEIKTLIAK;   ENIEAEIKTLIAKI;
NIEAEIKTLIAKIK;   IEAEIKTLIAKIKE;   EAEIKTLIAKIKEQ;
AEIKTLIAKIKEQS;   EIKTLIAKIKEQSN;   IKTLIAKIKEQSNL;
KTLIAKIKEQSNLY;   TLIAKIKEQSNLYE;   LIAKIKEQSNLYEA;
IAKIKEQSNLYEAY;   AKIKEQSNLYEAYK;   KIKEQSNLYEAYKA;
IKEQSNLYEAYKAI;   KEQSNLYEAYKAIV;   EQSNLYEAYKAIVT;
QSNLYEAYKAIVTS;   SNLYEAYKAIVTSI;   NLYEAYKAIVTSIL;
LYEAYKAIVTSILL;   YEAYKAIVTSILLM;   EAYKAIVTSILLMR;
AYKAIVTSILLMRD;   YKAIVTSILLMRDS;   KAIVTSILLMRDSL;
AIVTSILLMRDSLK;   IVTSILLMRDSLKE;   VTSILLMRDSLKEV;
TSILLMRDSLKEVQ;   SILLMRDSLKEVQG;   ILLMRDSLKEVQGI;
LLMRDSLKEVQGII;   LMRDSLKEVQGIID;   MRDSLKEVQGIIDK;
RDSLKEVQGIIDKN;   DSLKEVQGIIDKNG;   SLKEVQGIIDKNGV;
LKEVQGIIDKNGVW;   KEVQGIIDKNGVWY;

15 mers:
MKKVKSKYLALGLLF;   KKVKSKYLALGLLFG;   KVKSKYLALGLLFGF;
VKSKYLALGLLFGFI;   KSKYLALGLLFGFIS;   SKYLALGLLFGFISC;
KYLALGLLFGFISCD;   YLALGLLFGFISCDL;   LALGLLFGFISCDLF;
ALGLLFGFISCDLFI;   LGLLFGFISCDLFIR;   GLLFGFISCDLFIRY;
LLFGFISCDLFIRYE;   LFGFISCDLFIRYEM;   FGFISCDLFIRYEMK;
GFISCDLFIRYEMKE;   FISCDLFIRYEMKEE;   ISCDLFIRYEMKEES;
SCDLFIRYEMKEESP;   CDLFIRYEMKEESPG;   DLFIRYEMKEESPGL;
LFIRYEMKEESPGLF;   FIRYEMKEESPGLFD;   IRYEMKEESPGLFDK;
RYEMKEESPGLFDKG;   YEMKEESPGLFDKGN;   EMKEESPGLFDKGNS;
MKEESPGLFDKGNSI;   KEESPGLFDKGNSIL;   EESPGLFDKGNSILE;
```

ESPGLFDKGNSILET; SPGLFDKGNSILETS; PGLFDKGNSILETSE;
GLFDKGNSILETSEE; LFDKGNSILETSEES; FDKGNSILETSEESI;
DKGNSILETSEESIK; KGNSILETSEESIKK; GNSILETSEESIKKP;
NSILETSEESIKKPM; SILETSEESIKKPMN; ILETSEESIKKPMNK;
LETSEESIKKPMNKK; ETSEESIKKPMNKKG; TSEESIKKPMNKKGK;
SEESIKKPMNKKGKG; EESIKKPMNKKGKGK; ESIKKPMNKKGKGKI;
SIKKPMNKKGKGKIA; IKKPMNKKGKGKIAR; KKPMNKKGKGKIARK;
KPMNKKGKGKIARKK; PMNKKGKGKIARKKG; MNKKGKGKIARKKGK;
NKKGKGKIARKKGKS; KKGKGKIARKKGKSK; KGKGKIARKKGKSKV;
GKGKIARKKGKSKVS; KGKIARKKGKSKVSR; GKIARKKGKSKVSRK;
KIARKKGKSKVSRKE; IARKKGKSKVSRKEP; ARKKGKSKVSRKEPY;
RKKGKSKVSRKEPYI; KKGKSKVSRKEPYIH; KGKSKVSRKEPYIHS;
GKSKVSRKEPYIHSL; KSKVSRKEPYIHSLK; SKVSRKEPYIHSLKR;
KVSRKEPYIHSLKRD; VSRKEPYIHSLKRDS; SRKEPYIHSLKRDSA;
RKEPYIHSLKRDSAN; KEPYIHSLKRDSANK; EPYIHSLKRDSANKS;
PYIHSLKRDSANKSN; YIHSLKRDSANKSNF; IHSLKRDSANKSNFL;
HSLKRDSANKSNFLQ; SLKRDSANKSNFLQK; LKRDSANKSNFLQKN;
KRDSANKSNFLQKNV; RDSANKSNFLQKNVI; DSANKSNFLQKNVIL;
SANKSNFLQKNVILE; ANKSNFLQKNVILEE; NKSNFLQKNVILEEE;
KSNFLQKNVILEEES; SNFLQKNVILEEESL; NFLQKNVILEEESLK;
FLQKNVILEEESLKT; LQKNVILEEESLKTE; QKNVILEEESLKTEL;
KNVILEEESLKTELL; NVILEEESLKTELLK; VILEEESLKTELLKE;
ILEEESLKTELLKEQ; LEEESLKTELLKEQS; EEESLKTELLKEQSE;
EESLKTELLKEQSET; ESLKTELLKEQSETR; SLKTELLKEQSETRK;
LKTELLKEQSETRKE; KTELLKEQSETRKEK; TELLKEQSETRKEKI;
ELLKEQSETRKEKIQ; LLKEQSETRKEKIQK; LKEQSETRKEKIQKQ;
KEQSETRKEKIQKQQ; EQSETRKEKIQKQQD; QSETRKEKIQKQQDE;
SETRKEKIQKQQDEY; ETRKEKIQKQQDEYK; TRKEKIQKQQDEYKG;
RKEKIQKQQDEYKGM; KEKIQKQQDEYKGMT; EKIQKQQDEYKGMTQ;
KIQKQQDEYKGMTQG; IQKQQDEYKGMTQGS; QKQQDEYKGMTQGSL;
KQQDEYKGMTQGSLN; QQDEYKGMTQGSLNS; QDEYKGMTQGSLNSL;
DEYKGMTQGSLNSLS; EYKGMTQGSLNSLSG; YKGMTQGSLNSLSGE;
KGMTQGSLNSLSGES; GMTQGSLNSLSGESG; MTQGSLNSLSGESGE;
TQGSLNSLSGESGEL; QGSLNSLSGESGELK; GSLNSLSGESGELKE;
SLNSLSGESGELKET; LNSLSGESGELKETI; NSLSGESGELKETIE;
SLSGESGELKETIES; LSGESGELKETIESN; SGESGELKETIESNE;
GESGELKETIESNEI; ESGELKETIESNEID; SGELKETIESNEIDI;
GELKETIESNEIDIT; ELKETIESNEIDITI; LKETIESNEIDITID;
KETIESNEIDITIDS; ETIESNEIDITIDSD; TIESNEIDITIDSDL;
IESNEIDITIDSDLR; ESNEIDITIDSDLRP; SNEIDITIDSDLRPK;
NEIDITIDSDLRPKS; EIDITIDSDLRPKSS; IDITIDSDLRPKSSL;
DITIDSDLRPKSSLQ; ITIDSDLRPKSSLQD; TIDSDLRPKSSLQDI;
IDSDLRPKSSLQDIA; DSDLRPKSSLQDIAG; SDLRPKSSLQDIAGS;
DLRPKSSLQDIAGSN; LRPKSSLQDIAGSNS; RPKSSLQDIAGSNSI;
PKSSLQDIAGSNSIS; KSSLQDIAGSNSISY; SSLQDIAGSNSISYT;
SLQDIAGSNSISYTD; LQDIAGSNSISYTDE; QDIAGSNSISYTDEI;
DIAGSNSISYTDEIE; IAGSNSISYTDEIEE; AGSNSISYTDEIEEE;
GSNSISYTDEIEEED; SNSISYTDEIEEEDY; NSISYTDEIEEEDYA;
SISYTDEIEEEDYAR; ISYTDEIEEEDYARY; SYTDEIEEEDYARYY;
YTDEIEEEDYARYYL; TDEIEEEDYARYYLD; DEIEEEDYARYYLDE;
EIEEEDYARYYLDED; IEEEDYARYYLDEDD; EEEDYARYYLDEDDE;

| | |
|---|---|
| EEDYARYYLDEDDED; EDYARYYLDEDDEDD; DYARYYLDEDDEDDE;<br>YARYYLDEDDEDDEY; ARYYLDEDDEDDEYY; RYYLDEDDEDDEYYE;<br>YYLDEDDEDDEYYED; YLDEDDEDDEYYEDD; LDEDDEDDEYYEDDY;<br>DEDDEDDEYYEDDYE; EDDEDDEYYEDDYEE; DDEDDEYYEDDYEEI;<br>DEDDEYYEDDYEEIR; EDDEYYEDDYEEIRL; DDEYYEDDYEEIRLS;<br>DEYYEDDYEEIRLSN; EYYEDDYEEIRLSNR; YYEDDYEEIRLSNRY;<br>YEDDYEEIRLSNRYQ; EDDYEEIRLSNRYQS; DDYEEIRLSNRYQSY;<br>DYEEIRLSNRYQSYL; YEEIRLSNRYQSYLE; EEIRLSNRYQSYLEG;<br>EIRLSNRYQSYLEGV; IRLSNRYQSYLEGVK; RLSNRYQSYLEGVKY;<br>LSNRYQSYLEGVKYN; SNRYQSYLEGVKYNV; NRYQSYLEGVKYNVD;<br>RYQSYLEGVKYNVDS; YQSYLEGVKYNVDSA; QSYLEGVKYNVDSAI;<br>SYLEGVKYNVDSAIN; YLEGVKYNVDSAINT; LEGVKYNVDSAINTI;<br>EGVKYNVDSAINTIN; GVKYNVDSAINTINK; VKYNVDSAINTINKI;<br>KYNVDSAINTINKIY; YNVDSAINTINKIYD; NVDSAINTINKIYDT;<br>VDSAINTINKIYDTY; DSAINTINKIYDTYT; SAINTINKIYDTYTL;<br>AINTINKIYDTYTLF; INTINKIYDTYTLFS; NTINKIYDTYTLFST;<br>TINKIYDTYTLFSTK; INKIYDTYTLFSTKL; NKIYDTYTLFSTKLT;<br>KIYDTYTLFSTKLTQ; IYDTYTLFSTKLTQM; YDTYTLFSTKLTQMY;<br>DTYTLFSTKLTQMYS; TYTLFSTKLTQMYST; YTLFSTKLTQMYSTR;<br>TLFSTKLTQMYSTRL; LFSTKLTQMYSTRLD; FSTKLTQMYSTRLDN;<br>STKLTQMYSTRLDNL; TKLTQMYSTRLDNLA; KLTQMYSTRLDNLAK;<br>LTQMYSTRLDNLAKA; TQMYSTRLDNLAKAK; QMYSTRLDNLAKAKA;<br>MYSTRLDNLAKAKAK; YSTRLDNLAKAKAKE; STRLDNLAKAKAKEE;<br>TRLDNLAKAKAKEEA; RLDNLAKAKAKEEAA; LDNLAKAKAKEEAAK;<br>DNLAKAKAKEEAAKF; NLAKAKAKEEAAKFT; LAKAKAKEEAAKFTK;<br>AKAKAKEEAAKFTKE; KAKAKEEAAKFTKED; AKAKEEAAKFTKEDL;<br>KAKEEAAKFTKEDLE; AKEEAAKFTKEDLEK; KEEAAKFTKEDLEKN;<br>EEAAKFTKEDLEKNF; EAAKFTKEDLEKNFK; AAKFTKEDLEKNFKT;<br>AKFTKEDLEKNFKTL; KFTKEDLEKNFKTLL; FTKEDLEKNFKTLLN;<br>TKEDLEKNFKTLLNY; KEDLEKNFKTLLNYI; EDLEKNFKTLLNYIQ;<br>DLEKNFKTLLNYIQV; LEKNFKTLLNYIQVS; EKNFKTLLNYIQVSV;<br>KNFKTLLNYIQVSVK; NFKTLLNYIQVSVKT; FKTLLNYIQVSVKTA;<br>KTLLNYIQVSVKTAA; TLLNYIQVSVKTAAN; LLNYIQVSVKTAANF;<br>LNYIQVSVKTAANFV; NYIQVSVKTAANFVY; YIQVSVKTAANFVYI;<br>IQVSVKTAANFVYIN; QVSVKTAANFVYIND; VSVKTAANFVYINDT;<br>SVKTAANFVYINDTH; VKTAANFVYINDTHA; KTAANFVYINDTHAK;<br>TAANFVYINDTHAKR; AANFVYINDTHAKRK; ANFVYINDTHAKRKL;<br>NFVYINDTHAKRKLE; FVYINDTHAKRKLEN; VYINDTHAKRKLENI;<br>YINDTHAKRKLENIE; INDTHAKRKLENIEA; NDTHAKRKLENIEAE;<br>DTHAKRKLENIEAEI; THAKRKLENIEAEIK; HAKRKLENIEAEIKT;<br>AKRKLENIEAEIKTL; KRKLENIEAEIKTLI; RKLENIEAEIKTLIA;<br>KLENIEAEIKTLIAK; LENIEAEIKTLIAKI; ENIEAEIKTLIAKIK;<br>NIEAEIKTLIAKIKE; IEAEIKTLIAKIKEQ; EAEIKTLIAKIKEQS;<br>AEIKTLIAKIKEQSN; EIKTLIAKIKEQSNL; IKTLIAKIKEQSNLY;<br>KTLIAKIKEQSNLYE; TLIAKIKEQSNLYEA; LIAKIKEQSNLYEAY;<br>IAKIKEQSNLYEAYK; AKIKEQSNLYEAYKA; KIKEQSNLYEAYKAI;<br>IKEQSNLYEAYKAIV; KEQSNLYEAYKAIVT; EQSNLYEAYKAIVTS;<br>QSNLYEAYKAIVTSI; SNLYEAYKAIVTSIL; NLYEAYKAIVTSILL;<br>LYEAYKAIVTSILLM; YEAYKAIVTSILLMR; EAYKAIVTSILLMRD;<br>AYKAIVTSILLMRDS; YKAIVTSILLMRDSL; KAIVTSILLMRDSLK;<br>AIVTSILLMRDSLKE; IVTSILLMRDSLKEV; VTSILLMRDSLKEVQ; |

TSILLMRDSLKEVQG; SILLMRDSLKEVQGI; ILLMRDSLKEVQGII;
LLMRDSLKEVQGIID; LMRDSLKEVQGIIDK; MRDSLKEVQGIIDKN;
RDSLKEVQGIIDKNG; DSLKEVQGIIDKNGV; SLKEVQGIIDKNGVW;
LKEVQGIIDKNGVWY;

16 mers:
MKKVKSKYLALGLLFG; KKVKSKYLALGLLFGF; KVKSKYLALGLLFGFI;
VKSKYLALGLLFGFIS; KSKYLALGLLFGFISC; SKYLALGLLFGFISCD;
KYLALGLLFGFISCDL; YLALGLLFGFISCDLF; LALGLLFGFISCDLFI;
ALGLLFGFISCDLFIR; LGLLFGFISCDLFIRY; GLLFGFISCDLFIRYE;
LLFGFISCDLFIRYEM; LFGFISCDLFIRYEMK; FGFISCDLFIRYEMKE;
GFISCDLFIRYEMKEE; FISCDLFIRYEMKEES; ISCDLFIRYEMKEESP;
SCDLFIRYEMKEESPG; CDLFIRYEMKEESPGL; DLFIRYEMKEESPGLF;
LFIRYEMKEESPGLFD; FIRYEMKEESPGLFDK; IRYEMKEESPGLFDKG;
RYEMKEESPGLFDKGN; YEMKEESPGLFDKGNS; EMKEESPGLFDKGNSI;
MKEESPGLFDKGNSIL; KEESPGLFDKGNSILE; EESPGLFDKGNSILET;
ESPGLFDKGNSILETS; SPGLFDKGNSILETSE; PGLFDKGNSILETSEE;
GLFDKGNSILETSEES; LFDKGNSILETSEESI; FDKGNSILETSEESIK;
DKGNSILETSEESIKK; KGNSILETSEESIKKP; GNSILETSEESIKKPM;
NSILETSEESIKKPMN; SILETSEESIKKPMNK; ILETSEESIKKPMNKK;
LETSEESIKKPMNKKG; ETSEESIKKPMNKKGK; TSEESIKKPMNKKGKG;
SEESIKKPMNKKGKGK; EESIKKPMNKKGKGKI; ESIKKPMNKKGKGKIA;
SIKKPMNKKGKGKIAR; IKKPMNKKGKGKIARK; KKPMNKKGKGKIARKK;
KPMNKKGKGKIARKKG; PMNKKGKGKIARKKGK; MNKKGKGKIARKKGKS;
NKKGKGKIARKKGKSK; KKGKGKIARKKGKSKV; KGKGKIARKKGKSKVS;
GKGKIARKKGKSKVSR; KGKIARKKGKSKVSRK; GKIARKKGKSKVSRKE;
KIARKKGKSKVSRKEP; IARKKGKSKVSRKEPY; ARKKGKSKVSRKEPYI;
RKKGKSKVSRKEPYIH; KKGKSKVSRKEPYIHS; KGKSKVSRKEPYIHSL;
GKSKVSRKEPYIHSLK; KSKVSRKEPYIHSLKR; SKVSRKEPYIHSLKRD;
KVSRKEPYIHSLKRDS; VSRKEPYIHSLKRDSA; SRKEPYIHSLKRDSAN;
RKEPYIHSLKRDSANK; KEPYIHSLKRDSANKS; EPYIHSLKRDSANKSN;
PYIHSLKRDSANKSNF; YIHSLKRDSANKSNFL; IHSLKRDSANKSNFLQ;
HSLKRDSANKSNFLQK; SLKRDSANKSNFLQKN; LKRDSANKSNFLQKNV;
KRDSANKSNFLQKNVI; RDSANKSNFLQKNVIL; DSANKSNFLQKNVILE;
SANKSNFLQKNVILEE; ANKSNFLQKNVILEEE; NKSNFLQKNVILEEES;
KSNFLQKNVILEEESL; SNFLQKNVILEEESLK; NFLQKNVILEEESLKT;
FLQKNVILEEESLKTE; LQKNVILEEESLKTEL; QKNVILEEESLKTELL;
KNVILEEESLKTELLK; NVILEEESLKTELLKE; VILEEESLKTELLKEQ;
ILEEESLKTELLKEQS; LEEESLKTELLKEQSE; EEESLKTELLKEQSET;
EESLKTELLKEQSETR; ESLKTELLKEQSETRK; SLKTELLKEQSETRKE;
LKTELLKEQSETRKEK; KTELLKEQSETRKEKI; TELLKEQSETRKEKIQ;
ELLKEQSETRKEKIQK; LLKEQSETRKEKIQKQ; LKEQSETRKEKIQKQQ;
KEQSETRKEKIQKQQD; EQSETRKEKIQKQQDE; QSETRKEKIQKQQDEY;
SETRKEKIQKQQDEYK; ETRKEKIQKQQDEYKG; TRKEKIQKQQDEYKGM;
RKEKIQKQQDEYKGMT; KEKIQKQQDEYKGMTQ; EKIQKQQDEYKGMTQG;
KIQKQQDEYKGMTQGS; IQKQQDEYKGMTQGSL; QKQQDEYKGMTQGSLN;
KQQDEYKGMTQGSLNS; QQDEYKGMTQGSLNSL; QDEYKGMTQGSLNSLS;
DEYKGMTQGSLNSLSG; EYKGMTQGSLNSLSGE; YKGMTQGSLNSLSGES;
KGMTQGSLNSLSGESG; GMTQGSLNSLSGESGE; MTQGSLNSLSGESGEL;
TQGSLNSLSGESGELK; QGSLNSLSGESGELKE; GSLNSLSGESGELKET;
SLNSLSGESGELKETI; LNSLSGESGELKETIE; NSLSGESGELKETIES;

| | |
|---|---|
| SLSGESGELKETIESN; LSGESGELKETIESNE; SGESGELKETIESNEI; GESGELKETIESNEID; ESGELKETIESNEIDI; SGELKETIESNEIDIT; GELKETIESNEIDITI; ELKETIESNEIDITID; LKETIESNEIDITIDS; KETIESNEIDITIDSD; ETIESNEIDITIDSDL; TIESNEIDITIDSDLR; IESNEIDITIDSDLRP; ESNEIDITIDSDLRPK; SNEIDITIDSDLRPKS; NEIDITIDSDLRPKSS; EIDITIDSDLRPKSSL; IDITIDSDLRPKSSLQ; DITIDSDLRPKSSLQD; ITIDSDLRPKSSLQDI; TIDSDLRPKSSLQDIA; IDSDLRPKSSLQDIAG; DSDLRPKSSLQDIAGS; SDLRPKSSLQDIAGSN; DLRPKSSLQDIAGSNS; LRPKSSLQDIAGSNSI; RPKSSLQDIAGSNSIS; PKSSLQDIAGSNSISY; KSSLQDIAGSNSISYT; SSLQDIAGSNSISYTD; SLQDIAGSNSISYTDE; LQDIAGSNSISYTDEI; QDIAGSNSISYTDEIE; DIAGSNSISYTDEIEE; IAGSNSISYTDEIEEE; AGSNSISYTDEIEEED; GSNSISYTDEIEEEDY; SNSISYTDEIEEEDYA; NSISYTDEIEEEDYAR; SISYTDEIEEEDYARY; ISYTDEIEEEDYARYY; SYTDEIEEEDYARYYL; YTDEIEEEDYARYYLD; TDEIEEEDYARYYLDE; DEIEEEDYARYYLDED; EIEEEDYARYYLDEDD; IEEEDYARYYLDEDDE; EEEDYARYYLDEDDED; EEDYARYYLDEDDEDD; EDYARYYLDEDDEDDE; DYARYYLDEDDEDDEY; YARYYLDEDDEDDEYY; ARYYLDEDDEDDEYYE; RYYLDEDDEDDEYYED; YYLDEDDEDDEYYEDD; YLDEDDEDDEYYEDDY; LDEDDEDDEYYEDDYE; DEDDEDDEYYEDDYEE; EDDEDDEYYEDDYEEI; DDEDDEYYEDDYEEIR; DEDDEYYEDDYEEIRL; EDDEYYEDDYEEIRLS; DDEYYEDDYEEIRLSN; DEYYEDDYEEIRLSNR; EYYEDDYEEIRLSNRY; YYEDDYEEIRLSNRYQ; YEDDYEEIRLSNRYQS; EDDYEEIRLSNRYQSY; DDYEEIRLSNRYQSYL; DYEEIRLSNRYQSYLE; YEEIRLSNRYQSYLEG; EEIRLSNRYQSYLEGV; EIRLSNRYQSYLEGVK; IRLSNRYQSYLEGVKY; RLSNRYQSYLEGVKYN; LSNRYQSYLEGVKYNV; SNRYQSYLEGVKYNVD; NRYQSYLEGVKYNVDS; RYQSYLEGVKYNVDSA; YQSYLEGVKYNVDSAI; QSYLEGVKYNVDSAIN; SYLEGVKYNVDSAINT; YLEGVKYNVDSAINTI; LEGVKYNVDSAINTIN; EGVKYNVDSAINTINK; GVKYNVDSAINTINKI; VKYNVDSAINTINKIY; KYNVDSAINTINKIYD; YNVDSAINTINKIYDT; NVDSAINTINKIYDTY; VDSAINTINKIYDTYT; DSAINTINKIYDTYTL; SAINTINKIYDTYTLF; AINTINKIYDTYTLFS; INTINKIYDTYTLFST; NTINKIYDTYTLFSTK; TINKIYDTYTLFSTKL; INKIYDTYTLFSTKLT; NKIYDTYTLFSTKLTQ; KIYDTYTLFSTKLTQM; IYDTYTLFSTKLTQMY; YDTYTLFSTKLTQMYS; DTYTLFSTKLTQMYST; TYTLFSTKLTQMYSTR; YTLFSTKLTQMYSTRL; TLFSTKLTQMYSTRLD; LFSTKLTQMYSTRLDN; FSTKLTQMYSTRLDNL; STKLTQMYSTRLDNLA; TKLTQMYSTRLDNLAK; KLTQMYSTRLDNLAKA; LTQMYSTRLDNLAKAK; TQMYSTRLDNLAKAKA; QMYSTRLDNLAKAKAK; MYSTRLDNLAKAKAKE; YSTRLDNLAKAKAKEE; STRLDNLAKAKAKEEA; TRLDNLAKAKAKEEAA; RLDNLAKAKAKEEAAK; LDNLAKAKAKEEAAKF; DNLAKAKAKEEAAKFT; NLAKAKAKEEAAKFTK; LAKAKAKEEAAKFTKE; AKAKAKEEAAKFTKED; KAKAKEEAAKFTKEDL; AKAKEEAAKFTKEDLE; KAKEEAAKFTKEDLEK; AKEEAAKFTKEDLEKN; KEEAAKFTKEDLEKNF; EEAAKFTKEDLEKNFK; EAAKFTKEDLEKNFKT; AAKFTKEDLEKNFKTL; AKFTKEDLEKNFKTLL; KFTKEDLEKNFKTLLN; FTKEDLEKNFKTLLNY; TKEDLEKNFKTLLNYI; KEDLEKNFKTLLNYIQ; EDLEKNFKTLLNYIQV; DLEKNFKTLLNYIQVS; LEKNFKTLLNYIQVSV; EKNFKTLLNYIQVSVK; KNFKTLLNYIQVSVKT; NFKTLLNYIQVSVKTA; FKTLLNYIQVSVKTAA; KTLLNYIQVSVKTAAN; TLLNYIQVSVKTAANF; LLNYIQVSVKTAANFV; LNYIQVSVKTAANFVY; NYIQVSVKTAANFVYI; YIQVSVKTAANFVYIN; IQVSVKTAANFVYIND; QVSVKTAANFVYINDT; VSVKTAANFVYINDTH; |

| | | SVKTAANFVYINDTHA; VKTAANFVYINDTHAK; KTAANFVYINDTHAKR; TAANFVYINDTHAKRK; AANFVYINDTHAKRKL; ANFVYINDTHAKRKLE; NFVYINDTHAKRKLEN; FVYINDTHAKRKLENI; VYINDTHAKRKLENIE; YINDTHAKRKLENIEA; INDTHAKRKLENIEAE; NDTHAKRKLENIEAEI; DTHAKRKLENIEAEIK; THAKRKLENIEAEIKT; HAKRKLENIEAEIKTL; AKRKLENIEAEIKTLI; KRKLENIEAEIKTLIA; RKLENIEAEIKTLIAK; KLENIEAEIKTLIAKI; LENIEAEIKTLIAKIK; ENIEAEIKTLIAKIKE; NIEAEIKTLIAKIKEQ; IEAEIKTLIAKIKEQS; EAEIKTLIAKIKEQSN; AEIKTLIAKIKEQSNL; EIKTLIAKIKEQSNLY; IKTLIAKIKEQSNLYE; KTLIAKIKEQSNLYEA; TLIAKIKEQSNLYEAY; LIAKIKEQSNLYEAYK; IAKIKEQSNLYEAYKA; AKIKEQSNLYEAYKAI; KIKEQSNLYEAYKAIV; IKEQSNLYEAYKAIVT; KEQSNLYEAYKAIVTS; EQSNLYEAYKAIVTSI; QSNLYEAYKAIVTSIL; SNLYEAYKAIVTSILL; NLYEAYKAIVTSILLM; LYEAYKAIVTSILLMR; YEAYKAIVTSILLMRD; EAYKAIVTSILLMRDS; AYKAIVTSILLMRDSL; YKAIVTSILLMRDSLK; KAIVTSILLMRDSLKE; AIVTSILLMRDSLKEV; IVTSILLMRDSLKEVQ; VTSILLMRDSLKEVQG; TSILLMRDSLKEVQGI; SILLMRDSLKEVQGII; ILLMRDSLKEVQGIID; LLMRDSLKEVQGIIDK; LMRDSLKEVQGIIDKN; MRDSLKEVQGIIDKNG; RDSLKEVQGIIDKNGV; DSLKEVQGIIDKNGVW; SLKEVQGIIDKNGVWY; |
| Seq 43 | | 13 mers: MKIKSKCLALGLL; KIKSKCLALGLLF; IKSKCLALGLLFG; KSKCLALGLLFGF; SKCLALGLLFGFI; KCLALGLLFGFIS; CLALGLLFGFISC; LALGLLFGFISCD; ALGLLFGFISCDL; LGLLFGFISCDLF; GLLFGFISCDLFI; LLFGFISCDLFIR; LFGFISCDLFIRD; FGFISCDLFIRDE; GFISCDLFIRDEI; FISCDLFIRDEIK; ISCDLFIRDEIKE; SCDLFIRDEIKEK; CDLFIRDEIKEKS; DLFIRDEIKEKSL; LFIRDEIKEKSLG; FIRDEIKEKSLGL; IRDEIKEKSLGLC; RDEIKEKSLGLCD; DEIKEKSLGLCDE; EIKEKSLGLCDEE; IKEKSLGLCDEES; KEKSLGLCDEESS; EKSLGLCDEESSI; KSLGLCDEESSIL; SLGLCDEESSILE; LGLCDEESSILET; GLCDEESSILETG; LCDEESSILETGD; CDEESSILETGDK; DEESSILETGDKS; EESSILETGDKSV; ESSILETGDKSVK; SSILETGDKSVKK; SILETGDKSVKKS; ILETGDKSVKKSL; LETGDKSVKKSLN; ETGDKSVKKSLNK; TGDKSVKKSLNKK; GDKSVKKSLNKKG; DKSVKKSLNKKGK; KSVKKSLNKKGKD; SVKKSLNKKGKDK; VKKSLNKKGKDKV; KKSLNKKGKDKVA; KSLNKKGKDKVAR; SLNKKGKDKVARK; LNKKGKDKVARKK; NKKGKDKVARKKV; KKGKDKVARKKVE; KGKDKVARKKVEG; GKDKVARKKVEGN; KDKVARKKVEGNA; DKVARKKVEGNAV; KVARKKVEGNAVK; VARKKVEGNAVKK; ARKKVEGNAVKKD; RKKVEGNAVKKDP; KKVEGNAVKKDPF; KVEGNAVKKDPFN; VEGNAVKKDPFNH; EGNAVKKDPFNHH; GNAVKKDPFNHHV; NAVKKDPFNHHVK; AVKKDPFNHHVKR; VKKDPFNHHVKRE; KKDPFNHHVKRES; KDPFNHHVKRESV; DPFNHHVKRESVN; PFNHHVKRESVNN; FNHHVKRESVNNS; NHHVKRESVNNSN; HHVKRESVNNSNL; HVKRESVNNSNLS; VKRESVNNSNLSQ; KRESVNNSNLSQK; RESVNNSNLSQKN; ESVNNSNLSQKNV; SVNNSNLSQKNVI; VNNSNLSQKNVIS; NNSNLSQKNVISE; NSNLSQKNVISEE; |

| | | |
|---|---|---|
| SNLSQKNVISEEE; | NLSQKNVISEEEI; | LSQKNVISEEEIL; |
| SQKNVISEEEILK; | QKNVISEEEILKT; | KNVISEEEILKTK; |
| NVISEEEILKTKL; | VISEEEILKTKLL; | ISEEEILKTKLLR; |
| SEEEILKTKLLRE; | EEEILKTKLLRER; | EEILKTKLLRERP; |
| EILKTKLLRERPE; | ILKTKLLRERPET; | LKTKLLRERPETR; |
| KTKLLRERPETRK; | TKLLRERPETRKE; | KLLRERPETRKEE; |
| LLRERPETRKEEI; | LRERPETRKEEIQ; | RERPETRKEEIQK; |
| ERPETRKEEIQKQ; | RPETRKEEIQKQQ; | PETRKEEIQKQQD; |
| ETRKEEIQKQQDE; | TRKEEIQKQQDEH; | RKEEIQKQQDEHK; |
| KEEIQKQQDEHKR; | EEIQKQQDEHKRM; | EIQKQQDEHKRML; |
| IQKQQDEHKRMLQ; | QKQQDEHKRMLQG; | KQQDEHKRMLQGS; |
| QQDEHKRMLQGSL; | QDEHKRMLQGSLS; | DEHKRMLQGSLSF; |
| EHKRMLQGSLSFL; | HKRMLQGSLSFLS; | KRMLQGSLSFLSG; |
| RMLQGSLSFLSGE; | MLQGSLSFLSGES; | LQGSLSFLSGESG; |
| QGSLSFLSGESGE; | GSLSFLSGESGEL; | SLSFLSGESGELK; |
| LSFLSGESGELKD; | SFLSGESGELKDT; | FLSGESGELKDTI; |
| LSGESGELKDTIE; | SGESGELKDTIES; | GESGELKDTIESN; |
| ESGELKDTIESNE; | SGELKDTIESNEI; | GELKDTIESNEID; |
| ELKDTIESNEIDF; | LKDTIESNEIDFT; | KDTIESNEIDFTI; |
| DTIESNEIDFTID; | TIESNEIDFTIDS; | IESNEIDFTIDSD; |
| ESNEIDFTIDSDL; | SNEIDFTIDSDLR; | NEIDFTIDSDLRL; |
| EIDFTIDSDLRLK; | IDFTIDSDLRLKS; | DFTIDSDLRLKSD; |
| FTIDSDLRLKSDL; | TIDSDLRLKSDLQ; | IDSDLRLKSDLQA; |
| DSDLRLKSDLQAI; | SDLRLKSDLQAIS; | DLRLKSDLQAISG; |
| LRLKSDLQAISGS; | RLKSDLQAISGSN; | LKSDLQAISGSNS; |
| KSDLQAISGSNSI; | SDLQAISGSNSIS; | DLQAISGSNSISY; |
| LQAISGSNSISYT; | QAISGSNSISYTD; | AISGSNSISYTDE; |
| ISGSNSISYTDEI; | SGSNSISYTDEIE; | GSNSISYTDEIEE; |
| SNSISYTDEIEEE; | NSISYTDEIEEED; | SISYTDEIEEEDY; |
| ISYTDEIEEEDYD; | SYTDEIEEEDYDQ; | YTDEIEEEDYDQY; |
| TDEIEEEDYDQYS; | DEIEEEDYDQYSL; | EIEEEDYDQYSLE; |
| IEEEDYDQYSLEE; | EEEDYDQYSLEED; | EEDYDQYSLEEDY; |
| EDYDQYSLEEDYY; | DYDQYSLEEDYYY; | YDQYSLEEDYYYD; |
| DQYSLEEDYYYDG; | QYSLEEDYYYDGE; | YSLEEDYYYDGET; |
| SLEEDYYYDGETR; | LEEDYYYDGETRL; | EEDYYYDGETRLS; |
| EDYYYDGETRLSN; | DYYYDGETRLSNR; | YYYDGETRLSNRY; |
| YYDGETRLSNRYE; | YDGETRLSNRYES; | DGETRLSNRYESY; |
| GETRLSNRYESYL; | ETRLSNRYESYLE; | TRLSNRYESYLEG; |
| RLSNRYESYLEGV; | LSNRYESYLEGVK; | SNRYESYLEGVKY; |
| NRYESYLEGVKYN; | RYESYLEGVKYNV; | YESYLEGVKYNVS; |
| ESYLEGVKYNVSS; | SYLEGVKYNVSSA; | YLEGVKYNVSSAI; |
| LEGVKYNVSSAIK; | EGVKYNVSSAIKT; | GVKYNVSSAIKTI; |
| VKYNVSSAIKTIV; | KYNVSSAIKTIVK; | YNVSSAIKTIVKI; |
| NVSSAIKTIVKIY; | VSSAIKTIVKIYD; | SSAIKTIVKIYDN; |
| SAIKTIVKIYDNY; | AIKTIVKIYDNYT; | IKTIVKIYDNYTL; |
| KTIVKIYDNYTLL; | TIVKIYDNYTLLS; | IVKIYDNYTLLST; |
| VKIYDNYTLLSTK; | KIYDNYTLLSTKQ; | IYDNYTLLSTKQT; |
| YDNYTLLSTKQTQ; | DNYTLLSTKQTQM; | NYTLLSTKQTQMY; |
| YTLLSTKQTQMYS; | TLLSTKQTQMYST; | LLSTKQTQMYSTR; |
| LSTKQTQMYSTRL; | STKQTQMYSTRLD; | TKQTQMYSTRLDN; |
| KQTQMYSTRLDNL; | QTQMYSTRLDNLA; | TQMYSTRLDNLAK; |

QMYSTRLDNLAKA; MYSTRLDNLAKAK; YSTRLDNLAKAKA;
STRLDNLAKAKAR; TRLDNLAKAKARE; RLDNLAKAKAREE;
LDNLAKAKAREEA; DNLAKAKAREEAK; NLAKAKAREEAKK;
LAKAKAREEAKKF; AKAKAREEAKKFT; KAKAREEAKKFTK;
AKAREEAKKFTKE; KAREEAKKFTKEE; AREEAKKFTKEEL;
REEAKKFTKEELE; EEAKKFTKEELEK; EAKKFTKEELEKD;
AKKFTKEELEKDL; KKFTKEELEKDLK; KFTKEELEKDLKT;
FTKEELEKDLKTL; TKEELEKDLKTLL; KEELEKDLKTLLN;
EELEKDLKTLLNY; ELEKDLKTLLNYI; LEKDLKTLLNYIQ;
EKDLKTLLNYIQV; KDLKTLLNYIQVS; DLKTLLNYIQVSA;
LKTLLNYIQVSAR; KTLLNYIQVSART; TLLNYIQVSARTA;
LLNYIQVSARTAT; LNYIQVSARTATN; NYIQVSARTATNF;
YIQVSARTATNFV; IQVSARTATNFVY; QVSARTATNFVYA;
VSARTATNFVYAR; SARTATNFVYARE; ARTATNFVYAREI;
RTATNFVYAREIY; TATNFVYAREIYS; ATNFVYAREIYSK;
TNFVYAREIYSKR; NFVYAREIYSKRK; FVYAREIYSKRKL;
VYAREIYSKRKLD; YAREIYSKRKLDA; AREIYSKRKLDAI;
REIYSKRKLDAIE; EIYSKRKLDAIET; IYSKRKLDAIETE;
YSKRKLDAIETEI; SKRKLDAIETEIK; KRKLDAIETEIKN;
RKLDAIETEIKNL; KLDAIETEIKNLI; LDAIETEIKNLIL;
DAIETEIKNLILK; AIETEIKNLILKI; IETEIKNLILKIK;
ETEIKNLILKIKG; TEIKNLILKIKGQ; EIKNLILKIKGQS;
IKNLILKIKGQSD; KNLILKIKGQSDL; NLILKIKGQSDLY;
LILKIKGQSDLYE; ILKIKGQSDLYEA; LKIKGQSDLYEAY;
KIKGQSDLYEAYK; IKGQSDLYEAYKA; KGQSDLYEAYKAI;
GQSDLYEAYKAIV; QSDLYEAYKAIVR; SDLYEAYKAIVRS;
DLYEAYKAIVRSI; LYEAYKAIVRSIL; YEAYKAIVRSILL;
EAYKAIVRSILLM; AYKAIVRSILLMK; YKAIVRSILLMKD;
KAIVRSILLMKDS; AIVRSILLMKDSL; IVRSILLMKDSLK;
VRSILLMKDSLKI; RSILLMKDSLKII; SILLMKDSLKIIE;
ILLMKDSLKIIEI; LLMKDSLKIIEIV; LMKDSLKIIEIVI;
MKDSLKIIEIVID; KDSLKIIEIVIDK; DSLKIIEIVIDKN;
SLKIIEIVIDKNG; LKIIEIVIDKNGV; KIIEIVIDKNGVW;
IIEIVIDKNGVWY;

14 mers:
MKIKSKCLALGLLF; KIKSKCLALGLLFG; IKSKCLALGLLFGF;
KSKCLALGLLFGFI; SKCLALGLLFGFIS; KCLALGLLFGFISC;
CLALGLLFGFISCD; LALGLLFGFISCDL; ALGLLFGFISCDLF;
LGLLFGFISCDLFI; GLLFGFISCDLFIR; LLFGFISCDLFIRD;
LFGFISCDLFIRDE; FGFISCDLFIRDEI; GFISCDLFIRDEIK;
FISCDLFIRDEIKE; ISCDLFIRDEIKEK; SCDLFIRDEIKEKS;
CDLFIRDEIKEKSL; DLFIRDEIKEKSLG; LFIRDEIKEKSLGL;
FIRDEIKEKSLGLC; IRDEIKEKSLGLCD; RDEIKEKSLGLCDE;
DEIKEKSLGLCDEE; EIKEKSLGLCDEES; IKEKSLGLCDEESS;
KEKSLGLCDEESSI; EKSLGLCDEESSIL; KSLGLCDEESSILE;
SLGLCDEESSILET; LGLCDEESSILETG; GLCDEESSILETGD;
LCDEESSILETGDK; CDEESSILETGDKS; DEESSILETGDKSV;
EESSILETGDKSVK; ESSILETGDKSVKK; SSILETGDKSVKKS;
SILETGDKSVKKSL; ILETGDKSVKKSLN; LETGDKSVKKSLNK;
ETGDKSVKKSLNKK; TGDKSVKKSLNKKG; GDKSVKKSLNKKGK;

| | | |
|---|---|---|
| DKSVKKSLNKKGKD; | KSVKKSLNKKGKDK; | SVKKSLNKKGKDKV; |
| VKKSLNKKGKDKVA; | KKSLNKKGKDKVAR; | KSLNKKGKDKVARK; |
| SLNKKGKDKVARKK; | LNKKGKDKVARKKV; | NKKGKDKVARKKVE; |
| KKGKDKVARKKVEG; | KGKDKVARKKVEGN; | GKDKVARKKVEGNA; |
| KDKVARKKVEGNAV; | DKVARKKVEGNAVK; | KVARKKVEGNAVKK; |
| VARKKVEGNAVKKD; | ARKKVEGNAVKKDP; | RKKVEGNAVKKDPF; |
| KKVEGNAVKKDPFN; | KVEGNAVKKDPFNH; | VEGNAVKKDPFNHH; |
| EGNAVKKDPFNHHV; | GNAVKKDPFNHHVK; | NAVKKDPFNHHVKR; |
| AVKKDPFNHHVKRE; | VKKDPFNHHVKRES; | KKDPFNHHVKRESV; |
| KDPFNHHVKRESVN; | DPFNHHVKRESVNN; | PFNHHVKRESVNNS; |
| FNHHVKRESVNNSN; | NHHVKRESVNNSNL; | HHVKRESVNNSNLS; |
| HVKRESVNNSNLSQ; | VKRESVNNSNLSQK; | KRESVNNSNLSQKN; |
| RESVNNSNLSQKNV; | ESVNNSNLSQKNVI; | SVNNSNLSQKNVIS; |
| VNNSNLSQKNVISE; | NNSNLSQKNVISEE; | NSNLSQKNVISEEE; |
| SNLSQKNVISEEEI; | NLSQKNVISEEEIL; | LSQKNVISEEEILK; |
| SQKNVISEEEILKT; | QKNVISEEEILKTK; | KNVISEEEILKTKL; |
| NVISEEEILKTKLL; | VISEEEILKTKLLR; | ISEEEILKTKLLRE; |
| SEEEILKTKLLRER; | EEEILKTKLLRERP; | EEILKTKLLRERPE; |
| EILKTKLLRERPET; | ILKTKLLRERPETR; | LKTKLLRERPETRK; |
| KTKLLRERPETRKE; | TKLLRERPETRKEE; | KLLRERPETRKEEI; |
| LLRERPETRKEEIQ; | LRERPETRKEEIQK; | RERPETRKEEIQKQ; |
| ERPETRKEEIQKQQ; | RPETRKEEIQKQQD; | PETRKEEIQKQQDE; |
| ETRKEEIQKQQDEH; | TRKEEIQKQQDEHK; | RKEEIQKQQDEHKR; |
| KEEIQKQQDEHKRM; | EEIQKQQDEHKRML; | EIQKQQDEHKRMLQ; |
| IQKQQDEHKRMLQG; | QKQQDEHKRMLQGS; | KQQDEHKRMLQGSL; |
| QQDEHKRMLQGSLS; | QDEHKRMLQGSLSF; | DEHKRMLQGSLSFL; |
| EHKRMLQGSLSFLS; | HKRMLQGSLSFLSG; | KRMLQGSLSFLSGE; |
| RMLQGSLSFLSGES; | MLQGSLSFLSGESG; | LQGSLSFLSGESGE; |
| QGSLSFLSGESGEL; | GSLSFLSGESGELK; | SLSFLSGESGELKD; |
| LSFLSGESGELKDT; | SFLSGESGELKDTI; | FLSGESGELKDTIE; |
| LSGESGELKDTIES; | SGESGELKDTIESN; | GESGELKDTIESNE; |
| ESGELKDTIESNEI; | SGELKDTIESNEID; | GELKDTIESNEIDF; |
| ELKDTIESNEIDFT; | LKDTIESNEIDFTI; | KDTIESNEIDFTID; |
| DTIESNEIDFTIDS; | TIESNEIDFTIDSD; | IESNEIDFTIDSDL; |
| ESNEIDFTIDSDLR; | SNEIDFTIDSDLRL; | NEIDFTIDSDLRLK; |
| EIDFTIDSDLRLKS; | IDFTIDSDLRLKSD; | DFTIDSDLRLKSDL; |
| FTIDSDLRLKSDLQ; | TIDSDLRLKSDLQA; | IDSDLRLKSDLQAI; |
| DSDLRLKSDLQAIS; | SDLRLKSDLQAISG; | DLRLKSDLQAISGS; |
| LRLKSDLQAISGSN; | RLKSDLQAISGSNS; | LKSDLQAISGSNSI; |
| KSDLQAISGSNSIS; | SDLQAISGSNSISY; | DLQAISGSNSISYT; |
| LQAISGSNSISYTD; | QAISGSNSISYTDE; | AISGSNSISYTDEI; |
| ISGSNSISYTDEIE; | SGSNSISYTDEIEE; | GSNSISYTDEIEEE; |
| SNSISYTDEIEEED; | NSISYTDEIEEEDY; | SISYTDEIEEEDYD; |
| ISYTDEIEEEDYDQ; | SYTDEIEEEDYDQY; | YTDEIEEEDYDQYS; |
| TDEIEEEDYDQYSL; | DEIEEEDYDQYSLE; | EIEEEDYDQYSLEE; |
| IEEEDYDQYSLEED; | EEEDYDQYSLEEDY; | EEDYDQYSLEEDYY; |
| EDYDQYSLEEDYYY; | DYDQYSLEEDYYYD; | YDQYSLEEDYYYDG; |
| DQYSLEEDYYYDGE; | QYSLEEDYYYDGET; | YSLEEDYYYDGETR; |
| SLEEDYYYDGETRL; | LEEDYYYDGETRLS; | EEDYYYDGETRLSN; |
| EDYYYDGETRLSNR; | DYYYDGETRLSNRY; | YYYDGETRLSNRYE; |
| YYDGETRLSNRYES; | YDGETRLSNRYESY; | DGETRLSNRYESYL; |

GETRLSNRYESYLE; ETRLSNRYESYLEG; TRLSNRYESYLEGV;
RLSNRYESYLEGVK; LSNRYESYLEGVKY; SNRYESYLEGVKYN;
NRYESYLEGVKYNV; RYESYLEGVKYNVS; YESYLEGVKYNVSS;
ESYLEGVKYNVSSA; SYLEGVKYNVSSAI; YLEGVKYNVSSAIK;
LEGVKYNVSSAIKT; EGVKYNVSSAIKTI; GVKYNVSSAIKTIV;
VKYNVSSAIKTIVK; KYNVSSAIKTIVKI; YNVSSAIKTIVKIY;
NVSSAIKTIVKIYD; VSSAIKTIVKIYDN; SSAIKTIVKIYDNY;
SAIKTIVKIYDNYT; AIKTIVKIYDNYTL; IKTIVKIYDNYTLL;
KTIVKIYDNYTLLS; TIVKIYDNYTLLST; IVKIYDNYTLLSTK;
VKIYDNYTLLSTKQ; KIYDNYTLLSTKQT; IYDNYTLLSTKQTQ;
YDNYTLLSTKQTQM; DNYTLLSTKQTQMY; NYTLLSTKQTQMYS;
YTLLSTKQTQMYST; TLLSTKQTQMYSTR; LLSTKQTQMYSTRL;
LSTKQTQMYSTRLD; STKQTQMYSTRLDN; TKQTQMYSTRLDNL;
KQTQMYSTRLDNLA; QTQMYSTRLDNLAK; TQMYSTRLDNLAKA;
QMYSTRLDNLAKAK; MYSTRLDNLAKAKA; YSTRLDNLAKAKAR;
STRLDNLAKAKARE; TRLDNLAKAKAREE; RLDNLAKAKAREEA;
LDNLAKAKAREEAK; DNLAKAKAREEAKK; NLAKAKAREEAKKF;
LAKAKAREEAKKFT; AKAKAREEAKKFTK; KAKAREEAKKFTKE;
AKAREEAKKFTKEE; KAREEAKKFTKEEL; AREEAKKFTKEELE;
REEAKKFTKEELEK; EEAKKFTKEELEKD; EAKKFTKEELEKDL;
AKKFTKEELEKDLK; KKFTKEELEKDLKT; KFTKEELEKDLKTL;
FTKEELEKDLKTLL; TKEELEKDLKTLLN; KEELEKDLKTLLNY;
EELEKDLKTLLNYI; ELEKDLKTLLNYIQ; LEKDLKTLLNYIQV;
EKDLKTLLNYIQVS; KDLKTLLNYIQVSA; DLKTLLNYIQVSAR;
LKTLLNYIQVSART; KTLLNYIQVSARTA; TLLNYIQVSARTAT;
LLNYIQVSARTATN; LNYIQVSARTATNF; NYIQVSARTATNFV;
YIQVSARTATNFVY; IQVSARTATNFVYA; QVSARTATNFVYAR;
VSARTATNFVYARE; SARTATNFVYAREI; ARTATNFVYAREIY;
RTATNFVYAREIYS; TATNFVYAREIYSK; ATNFVYAREIYSKR;
TNFVYAREIYSKRK; NFVYAREIYSKRKL; FVYAREIYSKRKLD;
VYAREIYSKRKLDA; YAREIYSKRKLDAI; AREIYSKRKLDAIE;
REIYSKRKLDAIET; EIYSKRKLDAIETE; IYSKRKLDAIETEI;
YSKRKLDAIETEIK; SKRKLDAIETEIKN; KRKLDAIETEIKNL;
RKLDAIETEIKNLI; KLDAIETEIKNLIL; LDAIETEIKNLILK;
DAIETEIKNLILKI; AIETEIKNLILKIK; IETEIKNLILKIKG;
ETEIKNLILKIKGQ; TEIKNLILKIKGQS; EIKNLILKIKGQSD;
IKNLILKIKGQSDL; KNLILKIKGQSDLY; NLILKIKGQSDLYE;
LILKIKGQSDLYEA; ILKIKGQSDLYEAY; LKIKGQSDLYEAYK;
KIKGQSDLYEAYKA; IKGQSDLYEAYKAI; KGQSDLYEAYKAIV;
GQSDLYEAYKAIVR; QSDLYEAYKAIVRS; SDLYEAYKAIVRSI;
DLYEAYKAIVRSIL; LYEAYKAIVRSILL; YEAYKAIVRSILLM;
EAYKAIVRSILLMK; AYKAIVRSILLMKD; YKAIVRSILLMKDS;
KAIVRSILLMKDSL; AIVRSILLMKDSLK; IVRSILLMKDSLKI;
VRSILLMKDSLKII; RSILLMKDSLKIIE; SILLMKDSLKIIEI;
ILLMKDSLKIIEIV; LLMKDSLKIIEIVI; LMKDSLKIIEIVID;
MKDSLKIIEIVIDK; KDSLKIIEIVIDKN; DSLKIIEIVIDKNG;
SLKIIEIVIDKNGV; LKIIEIVIDKNGVW; KIIEIVIDKNGVWY;

15 mers:
MKIKSKCLALGLLFG; KIKSKCLALGLLFGF; IKSKCLALGLLFGFI;
KSKCLALGLLFGFIS; SKCLALGLLFGFISC; KCLALGLLFGFISCD;

CLALGLLFGFISCDL; LALGLLFGFISCDLF; ALGLLFGFISCDLFI;
LGLLFGFISCDLFIR; GLLFGFISCDLFIRD; LLFGFISCDLFIRDE;
LFGFISCDLFIRDEI; FGFISCDLFIRDEIK; GFISCDLFIRDEIKE;
FISCDLFIRDEIKEK; ISCDLFIRDEIKEKS; SCDLFIRDEIKEKSL;
CDLFIRDEIKEKSLG; DLFIRDEIKEKSLGL; LFIRDEIKEKSLGLC;
FIRDEIKEKSLGLCD; IRDEIKEKSLGLCDE; RDEIKEKSLGLCDEE;
DEIKEKSLGLCDEES; EIKEKSLGLCDEESS; IKEKSLGLCDEESSI;
KEKSLGLCDEESSIL; EKSLGLCDEESSILE; KSLGLCDEESSILET;
SLGLCDEESSILETG; LGLCDEESSILETGD; GLCDEESSILETGDK;
LCDEESSILETGDKS; CDEESSILETGDKSV; DEESSILETGDKSVK;
EESSILETGDKSVKK; ESSILETGDKSVKKS; SSILETGDKSVKKSL;
SILETGDKSVKKSLN; ILETGDKSVKKSLNK; LETGDKSVKKSLNKK;
ETGDKSVKKSLNKKG; TGDKSVKKSLNKKGK; GDKSVKKSLNKKGKD;
DKSVKKSLNKKGKDK; KSVKKSLNKKGKDKV; SVKKSLNKKGKDKVA;
VKKSLNKKGKDKVAR; KKSLNKKGKDKVARK; KSLNKKGKDKVARKK;
SLNKKGKDKVARKKV; LNKKGKDKVARKKVE; NKKGKDKVARKKVEG;
KKGKDKVARKKVEGN; KGKDKVARKKVEGNA; GKDKVARKKVEGNAV;
KDKVARKKVEGNAVK; DKVARKKVEGNAVKK; KVARKKVEGNAVKKD;
VARKKVEGNAVKKDP; ARKKVEGNAVKKDPF; RKKVEGNAVKKDPFN;
KKVEGNAVKKDPFNH; KVEGNAVKKDPFNHH; VEGNAVKKDPFNHHV;
EGNAVKKDPFNHHVK; GNAVKKDPFNHHVKR; NAVKKDPFNHHVKRE;
AVKKDPFNHHVKRES; VKKDPFNHHVKRESV; KKDPFNHHVKRESVN;
KDPFNHHVKRESVNN; DPFNHHVKRESVNNS; PFNHHVKRESVNNSN;
FNHHVKRESVNNSNL; NHHVKRESVNNSNLS; HHVKRESVNNSNLSQ;
HVKRESVNNSNLSQK; VKRESVNNSNLSQKN; KRESVNNSNLSQKNV;
RESVNNSNLSQKNVI; ESVNNSNLSQKNVIS; SVNNSNLSQKNVISE;
VNNSNLSQKNVISEE; NNSNLSQKNVISEEE; NSNLSQKNVISEEEI;
SNLSQKNVISEEEIL; NLSQKNVISEEEILK; LSQKNVISEEEILKT;
SQKNVISEEEILKTK; QKNVISEEEILKTKL; KNVISEEEILKTKLL;
NVISEEEILKTKLLR; VISEEEILKTKLLRE; ISEEEILKTKLLRER;
SEEEILKTKLLRERP; EEEILKTKLLRERPE; EEILKTKLLRERPET;
EILKTKLLRERPETR; ILKTKLLRERPETRK; LKTKLLRERPETRKE;
KTKLLRERPETRKEE; TKLLRERPETRKEEI; KLLRERPETRKEEIQ;
LLRERPETRKEEIQK; LRERPETRKEEIQKQ; RERPETRKEEIQKQQ;
ERPETRKEEIQKQQD; RPETRKEEIQKQQDE; PETRKEEIQKQQDEH;
ETRKEEIQKQQDEHK; TRKEEIQKQQDEHKR; RKEEIQKQQDEHKRM;
KEEIQKQQDEHKRML; EEIQKQQDEHKRMLQ; EIQKQQDEHKRMLQG;
IQKQQDEHKRMLQGS; QKQQDEHKRMLQGSL; KQQDEHKRMLQGSLS;
QQDEHKRMLQGSLSF; QDEHKRMLQGSLSFL; DEHKRMLQGSLSFLS;
EHKRMLQGSLSFLSG; HKRMLQGSLSFLSGE; KRMLQGSLSFLSGES;
RMLQGSLSFLSGESG; MLQGSLSFLSGESGE; LQGSLSFLSGESGEL;
QGSLSFLSGESGELK; GSLSFLSGESGELKD; SLSFLSGESGELKDT;
LSFLSGESGELKDTI; SFLSGESGELKDTIE; FLSGESGELKDTIES;
LSGESGELKDTIESN; SGESGELKDTIESNE; GESGELKDTIESNEI;
ESGELKDTIESNEID; SGELKDTIESNEIDF; GELKDTIESNEIDFT;
ELKDTIESNEIDFTI; LKDTIESNEIDFTID; KDTIESNEIDFTIDS;
DTIESNEIDFTIDSD; TIESNEIDFTIDSDL; IESNEIDFTIDSDLR;
ESNEIDFTIDSDLRL; SNEIDFTIDSDLRLK; NEIDFTIDSDLRLKS;
EIDFTIDSDLRLKSD; IDFTIDSDLRLKSDL; DFTIDSDLRLKSDLQ;
FTIDSDLRLKSDLQA; TIDSDLRLKSDLQAI; IDSDLRLKSDLQAIS;
DSDLRLKSDLQAISG; SDLRLKSDLQAISGS; DLRLKSDLQAISGSN;

LRLKSDLQAISGSNS; RLKSDLQAISGSNSI; LKSDLQAISGSNSIS;
KSDLQAISGSNSISY; SDLQAISGSNSISYT; DLQAISGSNSISYTD;
LQAISGSNSISYTDE; QAISGSNSISYTDEI; AISGSNSISYTDEIE;
ISGSNSISYTDEIEE; SGSNSISYTDEIEEE; GSNSISYTDEIEEED;
SNSISYTDEIEEEDY; NSISYTDEIEEEDYD; SISYTDEIEEEDYDQ;
ISYTDEIEEEDYDQY; SYTDEIEEEDYDQYS; YTDEIEEEDYDQYSL;
TDEIEEEDYDQYSLE; DEIEEEDYDQYSLEE; EIEEEDYDQYSLEED;
IEEEDYDQYSLEEDY; EEEDYDQYSLEEDYY; EEDYDQYSLEEDYYY;
EDYDQYSLEEDYYYD; DYDQYSLEEDYYYDG; YDQYSLEEDYYYDGE;
DQYSLEEDYYYDGET; QYSLEEDYYYDGETR; YSLEEDYYYDGETRL;
SLEEDYYYDGETRLS; LEEDYYYDGETRLSN; EEDYYYDGETRLSNR;
EDYYYDGETRLSNRY; DYYYDGETRLSNRYE; YYYDGETRLSNRYES;
YYDGETRLSNRYESY; YDGETRLSNRYESYL; DGETRLSNRYESYLE;
GETRLSNRYESYLEG; ETRLSNRYESYLEGV; TRLSNRYESYLEGVK;
RLSNRYESYLEGVKY; LSNRYESYLEGVKYN; SNRYESYLEGVKYNV;
NRYESYLEGVKYNVS; RYESYLEGVKYNVSS; YESYLEGVKYNVSSA;
ESYLEGVKYNVSSAI; SYLEGVKYNVSSAIK; YLEGVKYNVSSAIKT;
LEGVKYNVSSAIKTI; EGVKYNVSSAIKTIV; GVKYNVSSAIKTIVK;
VKYNVSSAIKTIVKI; KYNVSSAIKTIVKIY; YNVSSAIKTIVKIYD;
NVSSAIKTIVKIYDN; VSSAIKTIVKIYDNY; SSAIKTIVKIYDNYT;
SAIKTIVKIYDNYTL; AIKTIVKIYDNYTLL; IKTIVKIYDNYTLLS;
KTIVKIYDNYTLLST; TIVKIYDNYTLLSTK; IVKIYDNYTLLSTKQ;
VKIYDNYTLLSTKQT; KIYDNYTLLSTKQTQ; IYDNYTLLSTKQTQM;
YDNYTLLSTKQTQMY; DNYTLLSTKQTQMYS; NYTLLSTKQTQMYST;
YTLLSTKQTQMYSTR; TLLSTKQTQMYSTRL; LLSTKQTQMYSTRLD;
LSTKQTQMYSTRLDN; STKQTQMYSTRLDNL; TKQTQMYSTRLDNLA;
KQTQMYSTRLDNLAK; QTQMYSTRLDNLAKA; TQMYSTRLDNLAKAK;
QMYSTRLDNLAKAKA; MYSTRLDNLAKAKAR; YSTRLDNLAKAKARE;
STRLDNLAKAKAREE; TRLDNLAKAKAREEA; RLDNLAKAKAREEAK;
LDNLAKAKAREEAKK; DNLAKAKAREEAKKF; NLAKAKAREEAKKFT;
LAKAKAREEAKKFTK; AKAKAREEAKKFTKE; KAKAREEAKKFTKEE;
AKAREEAKKFTKEEL; KAREEAKKFTKEELE; AREEAKKFTKEELEK;
REEAKKFTKEELEKD; EEAKKFTKEELEKDL; EAKKFTKEELEKDLK;
AKKFTKEELEKDLKT; KKFTKEELEKDLKTL; KFTKEELEKDLKTLL;
FTKEELEKDLKTLLN; TKEELEKDLKTLLNY; KEELEKDLKTLLNYI;
EELEKDLKTLLNYIQ; ELEKDLKTLLNYIQV; LEKDLKTLLNYIQVS;
EKDLKTLLNYIQVSA; KDLKTLLNYIQVSAR; DLKTLLNYIQVSART;
LKTLLNYIQVSARTA; KTLLNYIQVSARTAT; TLLNYIQVSARTATN;
LLNYIQVSARTATNF; LNYIQVSARTATNFV; NYIQVSARTATNFVY;
YIQVSARTATNFVYA; IQVSARTATNFVYAR; QVSARTATNFVYARE;
VSARTATNFVYAREI; SARTATNFVYAREIY; ARTATNFVYAREIYS;
RTATNFVYAREIYSK; TATNFVYAREIYSKR; ATNFVYAREIYSKRK;
TNFVYAREIYSKRKL; NFVYAREIYSKRKLD; FVYAREIYSKRKLDA;
VYAREIYSKRKLDAI; YAREIYSKRKLDAIE; AREIYSKRKLDAIET;
REIYSKRKLDAIETE; EIYSKRKLDAIETEI; IYSKRKLDAIETEIK;
YSKRKLDAIETEIKN; SKRKLDAIETEIKNL; KRKLDAIETEIKNLI;
RKLDAIETEIKNLIL; KLDAIETEIKNLILK; LDAIETEIKNLILKI;
DAIETEIKNLILKIK; AIETEIKNLILKIKG; IETEIKNLILKIKGQ;
ETEIKNLILKIKGQS; TEIKNLILKIKGQSD; EIKNLILKIKGQSDL;
IKNLILKIKGQSDLY; KNLILKIKGQSDLYE; NLILKIKGQSDLYEA;
LILKIKGQSDLYEAY; ILKIKGQSDLYEAYK; LKIKGQSDLYEAYKA;

KIKGQSDLYEAYKAI; IKGQSDLYEAYKAIV; KGQSDLYEAYKAIVR;
GQSDLYEAYKAIVRS; QSDLYEAYKAIVRSI; SDLYEAYKAIVRSIL;
DLYEAYKAIVRSILL; LYEAYKAIVRSILLM; YEAYKAIVRSILLMK;
EAYKAIVRSILLMKD; AYKAIVRSILLMKDS; YKAIVRSILLMKDSL;
KAIVRSILLMKDSLK; AIVRSILLMKDSLKI; IVRSILLMKDSLKII;
VRSILLMKDSLKIIE; RSILLMKDSLKIIEI; SILLMKDSLKIIEIV;
ILLMKDSLKIIEIVI; LLMKDSLKIIEIVID; LMKDSLKIIEIVIDK;
MKDSLKIIEIVIDKN; KDSLKIIEIVIDKNG; DSLKIIEIVIDKNGV;
SLKIIEIVIDKNGVW; LKIIEIVIDKNGVWY;

16 mers:
MKIKSKCLALGLLFGF; KIKSKCLALGLLFGFI; IKSKCLALGLLFGFIS;
KSKCLALGLLFGFISC; SKCLALGLLFGFISCD; KCLALGLLFGFISCDL;
CLALGLLFGFISCDLF; LALGLLFGFISCDLFI; ALGLLFGFISCDLFIR;
LGLLFGFISCDLFIRD; GLLFGFISCDLFIRDE; LLFGFISCDLFIRDEI;
LFGFISCDLFIRDEIK; FGFISCDLFIRDEIKE; GFISCDLFIRDEIKEK;
FISCDLFIRDEIKEKS; ISCDLFIRDEIKEKSL; SCDLFIRDEIKEKSLG;
CDLFIRDEIKEKSLGL; DLFIRDEIKEKSLGLC; LFIRDEIKEKSLGLCD;
FIRDEIKEKSLGLCDE; IRDEIKEKSLGLCDEE; RDEIKEKSLGLCDEES;
DEIKEKSLGLCDEESS; EIKEKSLGLCDEESSI; IKEKSLGLCDEESSIL;
KEKSLGLCDEESSILE; EKSLGLCDEESSILET; KSLGLCDEESSILETG;
SLGLCDEESSILETGD; LGLCDEESSILETGDK; GLCDEESSILETGDKS;
LCDEESSILETGDKSV; CDEESSILETGDKSVK; DEESSILETGDKSVKK;
EESSILETGDKSVKKS; ESSILETGDKSVKKSL; SSILETGDKSVKKSLN;
SILETGDKSVKKSLNK; ILETGDKSVKKSLNKK; LETGDKSVKKSLNKKG;
ETGDKSVKKSLNKKGK; TGDKSVKKSLNKKGKD; GDKSVKKSLNKKGKDK;
DKSVKKSLNKKGKDKV; KSVKKSLNKKGKDKVA; SVKKSLNKKGKDKVAR;
VKKSLNKKGKDKVARK; KKSLNKKGKDKVARKK; KSLNKKGKDKVARKKV;
SLNKKGKDKVARKKVE; LNKKGKDKVARKKVEG; NKKGKDKVARKKVEGN;
KKGKDKVARKKVEGNA; KGKDKVARKKVEGNAV; GKDKVARKKVEGNAVK;
KDKVARKKVEGNAVKK; DKVARKKVEGNAVKKD; KVARKKVEGNAVKKDP;
VARKKVEGNAVKKDPF; ARKKVEGNAVKKDPFN; RKKVEGNAVKKDPFNH;
KKVEGNAVKKDPFNHH; KVEGNAVKKDPFNHHV; VEGNAVKKDPFNHHVK;
EGNAVKKDPFNHHVKR; GNAVKKDPFNHHVKRE; NAVKKDPFNHHVKRES;
AVKKDPFNHHVKRESV; VKKDPFNHHVKRESVN; KKDPFNHHVKRESVNN;
KDPFNHHVKRESVNNS; DPFNHHVKRESVNNSN; PFNHHVKRESVNNSNL;
FNHHVKRESVNNSNLS; NHHVKRESVNNSNLSQ; HHVKRESVNNSNLSQK;
HVKRESVNNSNLSQKN; VKRESVNNSNLSQKNV; KRESVNNSNLSQKNVI;
RESVNNSNLSQKNVIS; ESVNNSNLSQKNVISE; SVNNSNLSQKNVISEE;
VNNSNLSQKNVISEEE; NNSNLSQKNVISEEEI; NSNLSQKNVISEEEIL;
SNLSQKNVISEEEILK; NLSQKNVISEEEILKT; LSQKNVISEEEILKTK;
SQKNVISEEEILKTKL; QKNVISEEEILKTKLL; KNVISEEEILKTKLLR;
NVISEEEILKTKLLRE; VISEEEILKTKLLRER; ISEEEILKTKLLRERP;
SEEEILKTKLLRERPE; EEEILKTKLLRERPET; EEILKTKLLRERPETR;
EILKTKLLRERPETRK; ILKTKLLRERPETRKE; LKTKLLRERPETRKEE;
KTKLLRERPETRKEEI; TKLLRERPETRKEEIQ; KLLRERPETRKEEIQK;
LLRERPETRKEEIQKQ; LRERPETRKEEIQKQQ; RERPETRKEEIQKQQD;
ERPETRKEEIQKQQDE; RPETRKEEIQKQQDEH; PETRKEEIQKQQDEHK;
ETRKEEIQKQQDEHKR; TRKEEIQKQQDEHKRM; RKEEIQKQQDEHKRML;
KEEIQKQQDEHKRMLQ; EEIQKQQDEHKRMLQG; EIQKQQDEHKRMLQGS;
IQKQQDEHKRMLQGSL; QKQQDEHKRMLQGSLS; KQQDEHKRMLQGSLSF;

QQDEHKRMLQGSLSFL; QDEHKRMLQGSLSFLS; DEHKRMLQGSLSFLSG;
EHKRMLQGSLSFLSGE; HKRMLQGSLSFLSGES; KRMLQGSLSFLSGESG;
RMLQGSLSFLSGESGE; MLQGSLSFLSGESGEL; LQGSLSFLSGESGELK;
QGSLSFLSGESGELKD; GSLSFLSGESGELKDT; SLSFLSGESGELKDTI;
LSFLSGESGELKDTIE; SFLSGESGELKDTIES; FLSGESGELKDTIESN;
LSGESGELKDTIESNE; SGESGELKDTIESNEI; GESGELKDTIESNEID;
ESGELKDTIESNEIDF; SGELKDTIESNEIDFT; GELKDTIESNEIDFTI;
ELKDTIESNEIDFTID; LKDTIESNEIDFTIDS; KDTIESNEIDFTIDSD;
DTIESNEIDFTIDSDL; TIESNEIDFTIDSDLR; IESNEIDFTIDSDLRL;
ESNEIDFTIDSDLRLK; SNEIDFTIDSDLRLKS; NEIDFTIDSDLRLKSD;
EIDFTIDSDLRLKSDL; IDFTIDSDLRLKSDLQ; DFTIDSDLRLKSDLQA;
FTIDSDLRLKSDLQAI; TIDSDLRLKSDLQAIS; IDSDLRLKSDLQAISG;
DSDLRLKSDLQAISGS; SDLRLKSDLQAISGSN; DLRLKSDLQAISGSNS;
LRLKSDLQAISGSNSI; RLKSDLQAISGSNSIS; LKSDLQAISGSNSISY;
KSDLQAISGSNSISYT; SDLQAISGSNSISYTD; DLQAISGSNSISYTDE;
LQAISGSNSISYTDEI; QAISGSNSISYTDEIE; AISGSNSISYTDEIEE;
ISGSNSISYTDEIEEE; SGSNSISYTDEIEEED; GSNSISYTDEIEEEDY;
SNSISYTDEIEEEDYD; NSISYTDEIEEEDYDQ; SISYTDEIEEEDYDQY;
ISYTDEIEEEDYDQYS; SYTDEIEEEDYDQYSL; YTDEIEEEDYDQYSLE;
TDEIEEEDYDQYSLEE; DEIEEEDYDQYSLEED; EIEEEDYDQYSLEEDY;
IEEEDYDQYSLEEDYY; EEEDYDQYSLEEDYYY; EEDYDQYSLEEDYYYD;
EDYDQYSLEEDYYYDG; DYDQYSLEEDYYYDGE; YDQYSLEEDYYYDGET;
DQYSLEEDYYYDGETR; QYSLEEDYYYDGETRL; YSLEEDYYYDGETRLS;
SLEEDYYYDGETRLSN; LEEDYYYDGETRLSNR; EEDYYYDGETRLSNRY;
EDYYYDGETRLSNRYE; DYYYDGETRLSNRYES; YYYDGETRLSNRYESY;
YYDGETRLSNRYESYL; YDGETRLSNRYESYLE; DGETRLSNRYESYLEG;
GETRLSNRYESYLEGV; ETRLSNRYESYLEGVK; TRLSNRYESYLEGVKY;
RLSNRYESYLEGVKYN; LSNRYESYLEGVKYNV; SNRYESYLEGVKYNVS;
NRYESYLEGVKYNVSS; RYESYLEGVKYNVSSA; YESYLEGVKYNVSSAI;
ESYLEGVKYNVSSAIK; SYLEGVKYNVSSAIKT; YLEGVKYNVSSAIKTI;
LEGVKYNVSSAIKTIV; EGVKYNVSSAIKTIVK; GVKYNVSSAIKTIVKI;
VKYNVSSAIKTIVKIY; KYNVSSAIKTIVKIYD; YNVSSAIKTIVKIYDN;
NVSSAIKTIVKIYDNY; VSSAIKTIVKIYDNYT; SSAIKTIVKIYDNYTL;
SAIKTIVKIYDNYTLL; AIKTIVKIYDNYTLLS; IKTIVKIYDNYTLLST;
KTIVKIYDNYTLLSTK; TIVKIYDNYTLLSTKQ; IVKIYDNYTLLSTKQT;
VKIYDNYTLLSTKQTQ; KIYDNYTLLSTKQTQM; IYDNYTLLSTKQTQMY;
YDNYTLLSTKQTQMYS; DNYTLLSTKQTQMYST; NYTLLSTKQTQMYSTR;
YTLLSTKQTQMYSTRL; TLLSTKQTQMYSTRLD; LLSTKQTQMYSTRLDN;
LSTKQTQMYSTRLDNL; STKQTQMYSTRLDNLA; TKQTQMYSTRLDNLAK;
KQTQMYSTRLDNLAKA; QTQMYSTRLDNLAKAK; TQMYSTRLDNLAKAKA;
QMYSTRLDNLAKAKAR; MYSTRLDNLAKAKARE; YSTRLDNLAKAKAREE;
STRLDNLAKAKAREEA; TRLDNLAKAKAREEAK; RLDNLAKAKAREEAKK;
LDNLAKAKAREEAKKF; DNLAKAKAREEAKKFT; NLAKAKAREEAKKFTK;
LAKAKAREEAKKFTKE; AKAKAREEAKKFTKEE; KAKAREEAKKFTKEEL;
AKAREEAKKFTKEELE; KAREEAKKFTKEELEK; AREEAKKFTKEELEKD;
REEAKKFTKEELEKDL; EEAKKFTKEELEKDLK; EAKKFTKEELEKDLKT;
AKKFTKEELEKDLKTL; KKFTKEELEKDLKTLL; KFTKEELEKDLKTLLN;
FTKEELEKDLKTLLNY; TKEELEKDLKTLLNYI; KEELEKDLKTLLNYIQ;
EELEKDLKTLLNYIQV; ELEKDLKTLLNYIQVS; LEKDLKTLLNYIQVSA;
EKDLKTLLNYIQVSAR; KDLKTLLNYIQVSART; DLKTLLNYIQVSARTA;
LKTLLNYIQVSARTAT; KTLLNYIQVSARTATN; TLLNYIQVSARTATNF;

| | |
|---|---|
| | LLNYIQVSARTATNFV; LNYIQVSARTATNFVY; NYIQVSARTATNFVYA; YIQVSARTATNFVYAR; IQVSARTATNFVYARE; QVSARTATNFVYAREI; VSARTATNFVYAREIY; SARTATNFVYAREIYS; ARTATNFVYAREIYSK; RTATNFVYAREIYSKR; TATNFVYAREIYSKRK; ATNFVYAREIYSKRKL; TNFVYAREIYSKRKLD; NFVYAREIYSKRKLDA; FVYAREIYSKRKLDAI; VYAREIYSKRKLDAIE; YAREIYSKRKLDAIET; AREIYSKRKLDAIETE; REIYSKRKLDAIETEI; EIYSKRKLDAIETEIK; IYSKRKLDAIETEIKN; YSKRKLDAIETEIKNL; SKRKLDAIETEIKNLI; KRKLDAIETEIKNLIL; RKLDAIETEIKNLILK; KLDAIETEIKNLILKI; LDAIETEIKNLILKIK; DAIETEIKNLILKIKG; AIETEIKNLILKIKGQ; IETEIKNLILKIKGQS; ETEIKNLILKIKGQSD; TEIKNLILKIKGQSDL; EIKNLILKIKGQSDLY; IKNLILKIKGQSDLYE; KNLILKIKGQSDLYEA; NLILKIKGQSDLYEAY; LILKIKGQSDLYEAYK; ILKIKGQSDLYEAYKA; LKIKGQSDLYEAYKAI; KIKGQSDLYEAYKAIV; IKGQSDLYEAYKAIVR; KGQSDLYEAYKAIVRS; GQSDLYEAYKAIVRSI; QSDLYEAYKAIVRSIL; SDLYEAYKAIVRSILL; DLYEAYKAIVRSILLM; LYEAYKAIVRSILLMK; YEAYKAIVRSILLMKD; EAYKAIVRSILLMKDS; AYKAIVRSILLMKDSL; YKAIVRSILLMKDSLK; KAIVRSILLMKDSLKI; AIVRSILLMKDSLKII; IVRSILLMKDSLKIIE; VRSILLMKDSLKIIEI; RSILLMKDSLKIIEIV; SILLMKDSLKIIEIVI; ILLMKDSLKIIEIVID; LLMKDSLKIIEIVIDK; LMKDSLKIIEIVIDKN; MKDSLKIIEIVIDKNG; KDSLKIIEIVIDKNGV; DSLKIIEIVIDKNGVW; SLKIIEIVIDKNGVWY; |
| Seq 44 | 13 mers:<br>MKKVKSKYLALGL; KKVKSKYLALGLL; KVKSKYLALGLLF; VKSKYLALGLLFG; KSKYLALGLLFGF; SKYLALGLLFGFI; KYLALGLLFGFIS; YLALGLLFGFISC; LALGLLFGFISCD; ALGLLFGFISCDL; LGLLFGFISCDLF; GLLFGFISCDLFI; LLFGFISCDLFIR; LFGFISCDLFIRY; FGFISCDLFIRYE; GFISCDLFIRYEM; FISCDLFIRYEMK; ISCDLFIRYEMKE; SCDLFIRYEMKEE; CDLFIRYEMKEES; DLFIRYEMKEESP; LFIRYEMKEESPG; FIRYEMKEESPGL; IRYEMKEESPGLF; RYEMKEESPGLFD; YEMKEESPGLFDK; EMKEESPGLFDKG; MKEESPGLFDKGN; KEESPGLFDKGNS; EESPGLFDKGNSI; ESPGLFDKGNSIL; SPGLFDKGNSILE; PGLFDKGNSILET; GLFDKGNSILETS; LFDKGNSILETSE; FDKGNSILETSEE; DKGNSILETSEES; KGNSILETSEESI; GNSILETSEESIK; NSILETSEESIKK; SILETSEESIKKP; ILETSEESIKKPM; LETSEESIKKPMN; ETSEESIKKPMNK; TSEESIKKPMNKK; SEESIKKPMNKKG; EESIKKPMNKKGK; ESIKKPMNKKGKG; SIKKPMNKKGKGK; IKKPMNKKGKGKI; KKPMNKKGKGKIA; KPMNKKGKGKIAR; PMNKKGKGKIARK; MNKKGKGKIARKN; NKKGKGKIARKNG; KKGKGKIARKNGK; KGKGKIARKNGKS; GKGKIARKNGKSK; KGKIARKNGKSKV; GKIARKNGKSKVS; KIARKNGKSKVSG; IARKNGKSKVSGK; ARKNGKSKVSGKE; RKNGKSKVSGKEP; KNGKSKVSGKEPF; NGKSKVSGKEPFI; GKSKVSGKEPFIH; KSKVSGKEPFIHS; SKVSGKEPFIHSF; KVSGKEPFIHSFK; VSGKEPFIHSFKR; SGKEPFIHSFKRD; GKEPFIHSFKRDA; KEPFIHSFKRDAA; EPFIHSFKRDAAN; PFIHSFKRDAANK; FIHSFKRDAANKS; IHSFKRDAANKSN; |

HSFKRDAANKSNF; SFKRDAANKSNFL; FKRDAANKSNFLQ;
KRDAANKSNFLQK; RDAANKSNFLQKN; DAANKSNFLQKNV;
AANKSNFLQKNVM; ANKSNFLQKNVML; NKSNFLQKNVMLE;
KSNFLQKNVMLEE; SNFLQKNVMLEEE; NFLQKNVMLEEES;
FLQKNVMLEEESL; LQKNVMLEEESLK; QKNVMLEEESLKT;
KNVMLEEESLKTE; NVMLEEESLKTEL; VMLEEESLKTELL;
MLEEESLKTELLK; LEEESLKTELLKE; EEESLKTELLKEQ;
EEESLKTELLKEQS; ESLKTELLKEQSE; SLKTELLKEQSET;
LKTELLKEQSETR; KTELLKEQSETRK; TELLKEQSETRKE;
ELLKEQSETRKEK; LLKEQSETRKEKI; LKEQSETRKEKIQ;
KEQSETRKEKIQK; EQSETRKEKIQKQ; QSETRKEKIQKQQ;
SETRKEKIQKQQD; ETRKEKIQKQQDE; TRKEKIQKQQDEY;
RKEKIQKQQDEYK; KEKIQKQQDEYKG; EKIQKQQDEYKGM;
KIQKQQDEYKGMT; IQKQQDEYKGMTK; QKQQDEYKGMTKG;
KQQDEYKGMTKGS; QQDEYKGMTKGSL; QDEYKGMTKGSLN;
DEYKGMTKGSLNS; EYKGMTKGSLNSL; YKGMTKGSLNSLS;
KGMTKGSLNSLSG; GMTKGSLNSLSGE; MTKGSLNSLSGES;
TKGSLNSLSGESG; KGSLNSLSGESGE; GSLNSLSGESGEL;
SLNSLSGESGELK; LNSLSGESGELKE; NSLSGESGELKET;
SLSGESGELKETI; LSGESGELKETIE; SGESGELKETIES;
GESGELKETIESN; ESGELKETIESNE; SGELKETIESNEI;
GELKETIESNEID; ELKETIESNEIDI; LKETIESNEIDIT;
KETIESNEIDITI; ETIESNEIDITID; TIESNEIDITIDS;
IESNEIDITIDSD; ESNEIDITIDSDL; SNEIDITIDSDLR;
NEIDITIDSDLRP; EIDITIDSDLRPK; IDITIDSDLRPKS;
DITIDSDLRPKSS; ITIDSDLRPKSSL; TIDSDLRPKSSLQ;
IDSDLRPKSSLQD; DSDLRPKSSLQDI; SDLRPKSSLQDIA;
DLRPKSSLQDIAG; LRPKSSLQDIAGS; RPKSSLQDIAGSN;
PKSSLQDIAGSNS; KSSLQDIAGSNSI; SSLQDIAGSNSIS;
SLQDIAGSNSISY; LQDIAGSNSISYT; QDIAGSNSISYTD;
DIAGSNSISYTDE; IAGSNSISYTDEI; AGSNSISYTDEIE;
GSNSISYTDEIEE; SNSISYTDEIEEE; NSISYTDEIEEED;
SISYTDEIEEEDY; ISYTDEIEEEDYA; SYTDEIEEEDYAR;
YTDEIEEEDYARY; TDEIEEEDYARYY; DEIEEEDYARYYL;
EIEEEDYARYYLD; IEEEDYARYYLDE; EEEDYARYYLDED;
EEDYARYYLDEDD; EDYARYYLDEDDE; DYARYYLDEDDED;
YARYYLDEDDEDD; ARYYLDEDDEDDE; RYYLDEDDEDDEY;
YYLDEDDEDDEYY; YLDEDDEDDEYYE; LDEDDEDDEYYED;
DEDDEDDEYYEDD; EDDEDDEYYEDDY; DDEDDEYYEDDYE;
DEDDEYYEDDYEE; EDDEYYEDDYEEI; DDEYYEDDYEEIR;
DEYYEDDYEEIRL; EYYEDDYEEIRLS; YYEDDYEEIRLSN;
YEDDYEEIRLSNR; EDDYEEIRLSNRY; DDYEEIRLSNRYQ;
DYEEIRLSNRYQS; YEEIRLSNRYQSY; EEIRLSNRYQSYL;
EIRLSNRYQSYLE; IRLSNRYQSYLEG; RLSNRYQSYLEGV;
LSNRYQSYLEGVK; SNRYQSYLEGVKY; NRYQSYLEGVKYN;
RYQSYLEGVKYNV; YQSYLEGVKYNVD; QSYLEGVKYNVDS;
SYLEGVKYNVDSA; YLEGVKYNVDSAI; LEGVKYNVDSAIN;
EGVKYNVDSAINT; GVKYNVDSAINTI; VKYNVDSAINTIN;
KYNVDSAINTINK; YNVDSAINTINKI; NVDSAINTINKIY;
VDSAINTINKIYD; DSAINTINKIYDT; SAINTINKIYDTY;
AINTINKIYDTYT; INTINKIYDTYTL; NTINKIYDTYTLF;

TINKIYDTYTLFS; INKIYDTYTLFST; NKIYDTYTLFSTK;
KIYDTYTLFSTKL; IYDTYTLFSTKLT; YDTYTLFSTKLTQ;
DTYTLFSTKLTQM; TYTLFSTKLTQMY; YTLFSTKLTQMYS;
TLFSTKLTQMYST; LFSTKLTQMYSTR; FSTKLTQMYSTRL;
STKLTQMYSTRLD; TKLTQMYSTRLDN; KLTQMYSTRLDNL;
LTQMYSTRLDNLA; TQMYSTRLDNLAK; QMYSTRLDNLAKA;
MYSTRLDNLAKAK; YSTRLDNLAKAKA; STRLDNLAKAKAK;
TRLDNLAKAKAKE; RLDNLAKAKAKEE; LDNLAKAKAKEEA;
DNLAKAKAKEEAA; NLAKAKAKEEAAK; LAKAKAKEEAAKF;
AKAKAKEEAAKFT; KAKAKEEAAKFTK; AKAKEEAAKFTKE;
KAKEEAAKFTKED; AKEEAAKFTKEDL; KEEAAKFTKEDLE;
EEAAKFTKEDLEK; EAAKFTKEDLEKN; AAKFTKEDLEKNF;
AKFTKEDLEKNFK; KFTKEDLEKNFKT; FTKEDLEKNFKTL;
TKEDLEKNFKTLL; KEDLEKNFKTLLN; EDLEKNFKTLLNY;
DLEKNFKTLLNYI; LEKNFKTLLNYIQ; EKNFKTLLNYIQV;
KNFKTLLNYIQVS; NFKTLLNYIQVSV; FKTLLNYIQVSVK;
KTLLNYIQVSVKT; TLLNYIQVSVKTA; LLNYIQVSVKTAT;
LNYIQVSVKTATN; NYIQVSVKTATNF; YIQVSVKTATNFV;
IQVSVKTATNFVY; QVSVKTATNFVYI; VSVKTATNFVYIN;
SVKTATNFVYINE; VKTATNFVYINEM; KTATNFVYINEMH;
TATNFVYINEMHA; ATNFVYINEMHAK; TNFVYINEMHAKR;
NFVYINEMHAKRK; FVYINEMHAKRKL; VYINEMHAKRKLE;
YINEMHAKRKLEN; INEMHAKRKLENI; NEMHAKRKLENIE;
EMHAKRKLENIEA; MHAKRKLENIEAK; HAKRKLENIEAKI;
AKRKLENIEAKIK; KRKLENIEAKIKT; RKLENIEAKIKTL;
KLENIEAKIKTLI; LENIEAKIKTLIA; ENIEAKIKTLIAK;
NIEAKIKTLIAKI; IEAKIKTLIAKIK; EAKIKTLIAKIKE;
AKIKTLIAKIKEK; KIKTLIAKIKEKS; IKTLIAKIKEKSN;
KTLIAKIKEKSNL; TLIAKIKEKSNLY; LIAKIKEKSNLYS;
IAKIKEKSNLYSA; AKIKEKSNLYSAY; KIKEKSNLYSAYK;
IKEKSNLYSAYKA; KEKSNLYSAYKAI; EKSNLYSAYKAIV;
KSNLYSAYKAIVS; SNLYSAYKAIVSS; NLYSAYKAIVSSI;
LYSAYKAIVSSIL; YSAYKAIVSSILL; SAYKAIVSSILLM;
AYKAIVSSILLMR; YKAIVSSILLMRD; KAIVSSILLMRDS;
AIVSSILLMRDSL; IVSSILLMRDSLK; VSSILLMRDSLKE;
SSILLMRDSLKEV; SILLMRDSLKEVQ; ILLMRDSLKEVQY;
LLMRDSLKEVQYA; LMRDSLKEVQYAI; MRDSLKEVQYAID;
RDSLKEVQYAIDK; DSLKEVQYAIDKN; SLKEVQYAIDKNG;
LKEVQYAIDKNGI; KEVQYAIDKNGIW; EVQYAIDKNGIWY;
VQYAIDKNGIWYR; QYAIDKNGIWYRK; YAIDKNGIWYRKL;
AIDKNGIWYRKLD; IDKNGIWYRKLDA; DKNGIWYRKLDAI;
KNGIWYRKLDAIE; NGIWYRKLDAIET; GIWYRKLDAIETE;
IWYRKLDAIETEI; WYRKLDAIETEIK; YRKLDAIETEIKN;
RKLDAIETEIKNL; KLDAIETEIKNLI; LDAIETEIKNLIL;
DAIETEIKNLILK; AIETEIKNLILKI; IETEIKNLILKIK;
ETEIKNLILKIKG; TEIKNLILKIKGQ; EIKNLILKIKGQS;
IKNLILKIKGQSD; KNLILKIKGQSDL; NLILKIKGQSDLY;
LILKIKGQSDLYE; ILKIKGQSDLYEA; LKIKGQSDLYEAY;
KIKGQSDLYEAYK; IKGQSDLYEAYKA; KGQSDLYEAYKAI;
GQSDLYEAYKAIV; QSDLYEAYKAIVR; SDLYEAYKAIVRS;
DLYEAYKAIVRSI; LYEAYKAIVRSIL; YEAYKAIVRSILL;

EAYKAIVRSILLM; AYKAIVRSILLMK; YKAIVRSILLMKD;
KAIVRSILLMKDS; AIVRSILLMKDSL; IVRSILLMKDSLK;
VRSILLMKDSLKI; RSILLMKDSLKII; SILLMKDSLKIIE;
ILLMKDSLKIIEI; LLMKDSLKIIEIV; LMKDSLKIIEIVI;
MKDSLKIIEIVID; KDSLKIIEIVIDK; DSLKIIEIVIDKN;
SLKIIEIVIDKNG; LKIIEIVIDKNGV; KIIEIVIDKNGVW;
IIEIVIDKNGVWY;

14 mers:
MKKVKSKYLALGLL; KKVKSKYLALGLLF; KVKSKYLALGLLFG;
VKSKYLALGLLFGF; KSKYLALGLLFGFI; SKYLALGLLFGFIS;
KYLALGLLFGFISC; YLALGLLFGFISCD; LALGLLFGFISCDL;
ALGLLFGFISCDLF; LGLLFGFISCDLFI; GLLFGFISCDLFIR;
LLFGFISCDLFIRY; LFGFISCDLFIRYE; FGFISCDLFIRYEM;
GFISCDLFIRYEMK; FISCDLFIRYEMKE; ISCDLFIRYEMKEE;
SCDLFIRYEMKEES; CDLFIRYEMKEESP; DLFIRYEMKEESPG;
LFIRYEMKEESPGL; FIRYEMKEESPGLF; IRYEMKEESPGLFD;
RYEMKEESPGLFDK; YEMKEESPGLFDKG; EMKEESPGLFDKGN;
MKEESPGLFDKGNS; KEESPGLFDKGNSI; EESPGLFDKGNSIL;
ESPGLFDKGNSILE; SPGLFDKGNSILET; PGLFDKGNSILETS;
GLFDKGNSILETSE; LFDKGNSILETSEE; FDKGNSILETSEES;
DKGNSILETSEESI; KGNSILETSEESIK; GNSILETSEESIKK;
NSILETSEESIKKP; SILETSEESIKKPM; ILETSEESIKKPMN;
LETSEESIKKPMNK; ETSEESIKKPMNKK; TSEESIKKPMNKKG;
SEESIKKPMNKKGK; EESIKKPMNKKGKG; ESIKKPMNKKGKGK;
SIKKPMNKKGKGKI; IKKPMNKKGKGKIA; KKPMNKKGKGKIAR;
KPMNKKGKGKIARK; PMNKKGKGKIARKN; MNKKGKGKIARKNG;
NKKGKGKIARKNGK; KKGKGKIARKNGKS; KGKGKIARKNGKSK;
GKGKIARKNGKSKV; KGKIARKNGKSKVS; GKIARKNGKSKVSG;
KIARKNGKSKVSGK; IARKNGKSKVSGKE; ARKNGKSKVSGKEP;
RKNGKSKVSGKEPF; KNGKSKVSGKEPFI; NGKSKVSGKEPFIH;
GKSKVSGKEPFIHS; KSKVSGKEPFIHSF; SKVSGKEPFIHSFK;
KVSGKEPFIHSFKR; VSGKEPFIHSFKRD; SGKEPFIHSFKRDA;
GKEPFIHSFKRDAA; KEPFIHSFKRDAAN; EPFIHSFKRDAANK;
PFIHSFKRDAANKS; FIHSFKRDAANKSN; IHSFKRDAANKSNF;
HSFKRDAANKSNFL; SFKRDAANKSNFLQ; FKRDAANKSNFLQK;
KRDAANKSNFLQKN; RDAANKSNFLQKNV; DAANKSNFLQKNVM;
AANKSNFLQKNVML; ANKSNFLQKNVMLE; NKSNFLQKNVMLEE;
KSNFLQKNVMLEEE; SNFLQKNVMLEEES; NFLQKNVMLEEESL;
FLQKNVMLEEESLK; LQKNVMLEEESLKT; QKNVMLEEESLKTE;
KNVMLEEESLKTEL; NVMLEEESLKTELL; VMLEEESLKTELLK;
MLEEESLKTELLKE; LEEESLKTELLKEQ; EEESLKTELLKEQS;
EESLKTELLKEQSE; ESLKTELLKEQSET; SLKTELLKEQSETR;
LKTELLKEQSETRK; KTELLKEQSETRKE; TELLKEQSETRKEK;
ELLKEQSETRKEKI; LLKEQSETRKEKIQ; LKEQSETRKEKIQK;
KEQSETRKEKIQKQ; EQSETRKEKIQKQQ; QSETRKEKIQKQQD;
SETRKEKIQKQQDE; ETRKEKIQKQQDEY; TRKEKIQKQQDEYK;
RKEKIQKQQDEYKG; KEKIQKQQDEYKGM; EKIQKQQDEYKGMT;
KIQKQQDEYKGMTK; IQKQQDEYKGMTKG; QKQQDEYKGMTKGS;
KQQDEYKGMTKGSL; QQDEYKGMTKGSLN; QDEYKGMTKGSLNS;
DEYKGMTKGSLNSL; EYKGMTKGSLNSLS; YKGMTKGSLNSLSG;

| | | |
|---|---|---|
| KGMTKGSLNSLSGE; | GMTKGSLNSLSGES; | MTKGSLNSLSGESG; |
| TKGSLNSLSGESGE; | KGSLNSLSGESGEL; | GSLNSLSGESGELK; |
| SLNSLSGESGELKE; | LNSLSGESGELKET; | NSLSGESGELKETI; |
| SLSGESGELKETIE; | LSGESGELKETIES; | SGESGELKETIESN; |
| GESGELKETIESNE; | ESGELKETIESNEI; | SGELKETIESNEID; |
| GELKETIESNEIDI; | ELKETIESNEIDIT; | LKETIESNEIDITI; |
| KETIESNEIDITID; | ETIESNEIDITIDS; | TIESNEIDITIDSD; |
| IESNEIDITIDSDL; | ESNEIDITIDSDLR; | SNEIDITIDSDLRP; |
| NEIDITIDSDLRPK; | EIDITIDSDLRPKS; | IDITIDSDLRPKSS; |
| DITIDSDLRPKSSL; | ITIDSDLRPKSSLQ; | TIDSDLRPKSSLQD; |
| IDSDLRPKSSLQDI; | DSDLRPKSSLQDIA; | SDLRPKSSLQDIAG; |
| DLRPKSSLQDIAGS; | LRPKSSLQDIAGSN; | RPKSSLQDIAGSNS; |
| PKSSLQDIAGSNSI; | KSSLQDIAGSNSIS; | SSLQDIAGSNSISY; |
| SLQDIAGSNSISYT; | LQDIAGSNSISYTD; | QDIAGSNSISYTDE; |
| DIAGSNSISYTDEI; | IAGSNSISYTDEIE; | AGSNSISYTDEIEE; |
| GSNSISYTDEIEEE; | SNSISYTDEIEEED; | NSISYTDEIEEEDY; |
| SISYTDEIEEEDYA; | ISYTDEIEEEDYAR; | SYTDEIEEEDYARY; |
| YTDEIEEEDYARYY; | TDEIEEEDYARYYL; | DEIEEEDYARYYLD; |
| EIEEEDYARYYLDE; | IEEEDYARYYLDED; | EEEDYARYYLDEDD; |
| EEDYARYYLDEDDE; | EDYARYYLDEDDED; | DYARYYLDEDDEDD; |
| YARYYLDEDDEDDE; | ARYYLDEDDEDDEY; | RYYLDEDDEDDEYY; |
| YYLDEDDEDDEYYE; | YLDEDDEDDEYYED; | LDEDDEDDEYYEDD; |
| DEDDEDDEYYEDDY; | EDDEDDEYYEDDYE; | DDEDDEYYEDDYEE; |
| DEDDEYYEDDYEEI; | EDDEYYEDDYEEIR; | DDEYYEDDYEEIRL; |
| DEYYEDDYEEIRLS; | EYYEDDYEEIRLSN; | YYEDDYEEIRLSNR; |
| YEDDYEEIRLSNRY; | EDDYEEIRLSNRYQ; | DDYEEIRLSNRYQS; |
| DYEEIRLSNRYQSY; | YEEIRLSNRYQSYL; | EEIRLSNRYQSYLE; |
| EIRLSNRYQSYLEG; | IRLSNRYQSYLEGV; | RLSNRYQSYLEGVK; |
| LSNRYQSYLEGVKY; | SNRYQSYLEGVKYN; | NRYQSYLEGVKYNV; |
| RYQSYLEGVKYNVD; | YQSYLEGVKYNVDS; | QSYLEGVKYNVDSA; |
| SYLEGVKYNVDSAI; | YLEGVKYNVDSAIN; | LEGVKYNVDSAINT; |
| EGVKYNVDSAINTI; | GVKYNVDSAINTIN; | VKYNVDSAINTINK; |
| KYNVDSAINTINKI; | YNVDSAINTINKIY; | NVDSAINTINKIYD; |
| VDSAINTINKIYDT; | DSAINTINKIYDTY; | SAINTINKIYDTYT; |
| AINTINKIYDTYTL; | INTINKIYDTYTLF; | NTINKIYDTYTLFS; |
| TINKIYDTYTLFST; | INKIYDTYTLFSTK; | NKIYDTYTLFSTKL; |
| KIYDTYTLFSTKLT; | IYDTYTLFSTKLTQ; | YDTYTLFSTKLTQM; |
| DTYTLFSTKLTQMY; | TYTLFSTKLTQMYS; | YTLFSTKLTQMYST; |
| TLFSTKLTQMYSTR; | LFSTKLTQMYSTRL; | FSTKLTQMYSTRLD; |
| STKLTQMYSTRLDN; | TKLTQMYSTRLDNL; | KLTQMYSTRLDNLA; |
| LTQMYSTRLDNLAK; | TQMYSTRLDNLAKA; | QMYSTRLDNLAKAK; |
| MYSTRLDNLAKAKA; | YSTRLDNLAKAKAK; | STRLDNLAKAKAKE; |
| TRLDNLAKAKAKEE; | RLDNLAKAKAKEEA; | LDNLAKAKAKEEAA; |
| DNLAKAKAKEEAAK; | NLAKAKAKEEAAKF; | LAKAKAKEEAAKFT; |
| AKAKAKEEAAKFTK; | KAKAKEEAAKFTKE; | AKAKEEAAKFTKED; |
| KAKEEAAKFTKEDL; | AKEEAAKFTKEDLE; | KEEAAKFTKEDLEK; |
| EEAAKFTKEDLEKN; | EAAKFTKEDLEKNF; | AAKFTKEDLEKNFK; |
| AKFTKEDLEKNFKT; | KFTKEDLEKNFKTL; | FTKEDLEKNFKTLL; |
| TKEDLEKNFKTLLN; | KEDLEKNFKTLLNY; | EDLEKNFKTLLNYI; |
| DLEKNFKTLLNYIQ; | LEKNFKTLLNYIQV; | EKNFKTLLNYIQVS; |
| KNFKTLLNYIQVSV; | NFKTLLNYIQVSVK; | FKTLLNYIQVSVKT; |

KTLLNYIQVSVKTA; TLLNYIQVSVKTAT; LLNYIQVSVKTATN;
LNYIQVSVKTATNF; NYIQVSVKTATNFV; YIQVSVKTATNFVY;
IQVSVKTATNFVYI; QVSVKTATNFVYIN; VSVKTATNFVYINE;
SVKTATNFVYINEM; VKTATNFVYINEMH; KTATNFVYINEMHA;
TATNFVYINEMHAK; ATNFVYINEMHAKR; TNFVYINEMHAKRK;
NFVYINEMHAKRKL; FVYINEMHAKRKLE; VYINEMHAKRKLEN;
YINEMHAKRKLENI; INEMHAKRKLENIE; NEMHAKRKLENIEA;
EMHAKRKLENIEAK; MHAKRKLENIEAKI; HAKRKLENIEAKIK;
AKRKLENIEAKIKT; KRKLENIEAKIKTL; RKLENIEAKIKTLI;
KLENIEAKIKTLIA; LENIEAKIKTLIAK; ENIEAKIKTLIAKI;
NIEAKIKTLIAKIK; IEAKIKTLIAKIKE; EAKIKTLIAKIKEK;
AKIKTLIAKIKEKS; KIKTLIAKIKEKSN; IKTLIAKIKEKSNL;
KTLIAKIKEKSNLY; TLIAKIKEKSNLYS; LIAKIKEKSNLYSA;
IAKIKEKSNLYSAY; AKIKEKSNLYSAYK; KIKEKSNLYSAYKA;
IKEKSNLYSAYKAI; KEKSNLYSAYKAIV; EKSNLYSAYKAIVS;
KSNLYSAYKAIVSS; SNLYSAYKAIVSSI; NLYSAYKAIVSSIL;
LYSAYKAIVSSILL; YSAYKAIVSSILLM; SAYKAIVSSILLMR;
AYKAIVSSILLMRD; YKAIVSSILLMRDS; KAIVSSILLMRDSL;
AIVSSILLMRDSLK; IVSSILLMRDSLKE; VSSILLMRDSLKEV;
SSILLMRDSLKEVQ; SILLMRDSLKEVQY; ILLMRDSLKEVQYA;
LLMRDSLKEVQYAI; LMRDSLKEVQYAID; MRDSLKEVQYAIDK;
RDSLKEVQYAIDKN; DSLKEVQYAIDKNG; SLKEVQYAIDKNGI;
LKEVQYAIDKNGIW; KEVQYAIDKNGIWY; EVQYAIDKNGIWYR;
VQYAIDKNGIWYRK; QYAIDKNGIWYRKL; YAIDKNGIWYRKLD;
AIDKNGIWYRKLDA; IDKNGIWYRKLDAI; DKNGIWYRKLDAIE;
KNGIWYRKLDAIET; NGIWYRKLDAIETE; GIWYRKLDAIETEI;
IWYRKLDAIETEIK; WYRKLDAIETEIKN; YRKLDAIETEIKNL;
RKLDAIETEIKNLI; KLDAIETEIKNLIL; LDAIETEIKNLILK;
DAIETEIKNLILKI; AIETEIKNLILKIK; IETEIKNLILKIKG;
ETEIKNLILKIKGQ; TEIKNLILKIKGQS; EIKNLILKIKGQSD;
IKNLILKIKGQSDL; KNLILKIKGQSDLY; NLILKIKGQSDLYE;
LILKIKGQSDLYEA; ILKIKGQSDLYEAY; LKIKGQSDLYEAYK;
KIKGQSDLYEAYKA; IKGQSDLYEAYKAI; KGQSDLYEAYKAIV;
GQSDLYEAYKAIVR; QSDLYEAYKAIVRS; SDLYEAYKAIVRSI;
DLYEAYKAIVRSIL; LYEAYKAIVRSILL; YEAYKAIVRSILLM;
EAYKAIVRSILLMK; AYKAIVRSILLMKD; YKAIVRSILLMKDS;
KAIVRSILLMKDSL; AIVRSILLMKDSLK; IVRSILLMKDSLKI;
VRSILLMKDSLKII; RSILLMKDSLKIIE; SILLMKDSLKIIEI;
ILLMKDSLKIIEIV; LLMKDSLKIIEIVI; LMKDSLKIIEIVID;
MKDSLKIIEIVIDK; KDSLKIIEIVIDKN; DSLKIIEIVIDKNG;
SLKIIEIVIDKNGV; LKIIEIVIDKNGVW; KIIEIVIDKNGVWY;

15 mers:
MKKVKSKYLALGLLF; KKVKSKYLALGLLFG; KVKSKYLALGLLFGF;
VKSKYLALGLLFGFI; KSKYLALGLLFGFIS; SKYLALGLLFGFISC;
KYLALGLLFGFISCD; YLALGLLFGFISCDL; LALGLLFGFISCDLF;
ALGLLFGFISCDLFI; LGLLFGFISCDLFIR; GLLFGFISCDLFIRY;
LLFGFISCDLFIRYE; LFGFISCDLFIRYEM; FGFISCDLFIRYEMK;
GFISCDLFIRYEMKE; FISCDLFIRYEMKEE; ISCDLFIRYEMKEES;
SCDLFIRYEMKEESP; CDLFIRYEMKEESPG; DLFIRYEMKEESPGL;
LFIRYEMKEESPGLF; FIRYEMKEESPGLFD; IRYEMKEESPGLFDK;

RYEMKEESPGLFDKG; YEMKEESPGLFDKGN; EMKEESPGLFDKGNS;
MKEESPGLFDKGNSI; KEESPGLFDKGNSIL; EESPGLFDKGNSILE;
ESPGLFDKGNSILET; SPGLFDKGNSILETS; PGLFDKGNSILETSE;
GLFDKGNSILETSEE; LFDKGNSILETSEES; FDKGNSILETSEESI;
DKGNSILETSEESIK; KGNSILETSEESIKK; GNSILETSEESIKKP;
NSILETSEESIKKPM; SILETSEESIKKPMN; ILETSEESIKKPMNK;
LETSEESIKKPMNKK; ETSEESIKKPMNKKG; TSEESIKKPMNKKGK;
SEESIKKPMNKKGKG; EESIKKPMNKKGKGK; ESIKKPMNKKGKGKI;
SIKKPMNKKGKGKIA; IKKPMNKKGKGKIAR; KKPMNKKGKGKIARK;
KPMNKKGKGKIARKN; PMNKKGKGKIARKNG; MNKKGKGKIARKNGK;
NKKGKGKIARKNGKS; KKGKGKIARKNGKSK; KGKGKIARKNGKSKV;
GKGKIARKNGKSKVS; KGKIARKNGKSKVSG; GKIARKNGKSKVSGK;
KIARKNGKSKVSGKE; IARKNGKSKVSGKEP; ARKNGKSKVSGKEPF;
RKNGKSKVSGKEPFI; KNGKSKVSGKEPFIH; NGKSKVSGKEPFIHS;
GKSKVSGKEPFIHSF; KSKVSGKEPFIHSFK; SKVSGKEPFIHSFKR;
KVSGKEPFIHSFKRD; VSGKEPFIHSFKRDA; SGKEPFIHSFKRDAA;
GKEPFIHSFKRDAAN; KEPFIHSFKRDAANK; EPFIHSFKRDAANKS;
PFIHSFKRDAANKSN; FIHSFKRDAANKSNF; IHSFKRDAANKSNFL;
HSFKRDAANKSNFLQ; SFKRDAANKSNFLQK; FKRDAANKSNFLQKN;
KRDAANKSNFLQKNV; RDAANKSNFLQKNVM; DAANKSNFLQKNVML;
AANKSNFLQKNVMLE; ANKSNFLQKNVMLEE; NKSNFLQKNVMLEEE;
KSNFLQKNVMLEEES; SNFLQKNVMLEEESL; NFLQKNVMLEEESLK;
FLQKNVMLEEESLKT; LQKNVMLEEESLKTE; QKNVMLEEESLKTEL;
KNVMLEEESLKTELL; NVMLEEESLKTELLK; VMLEEESLKTELLKE;
MLEEESLKTELLKEQ; LEEESLKTELLKEQS; EEESLKTELLKEQSE;
EESLKTELLKEQSET; ESLKTELLKEQSETR; SLKTELLKEQSETRK;
LKTELLKEQSETRKE; KTELLKEQSETRKEK; TELLKEQSETRKEKI;
ELLKEQSETRKEKIQ; LLKEQSETRKEKIQK; LKEQSETRKEKIQKQ;
KEQSETRKEKIQKQQ; EQSETRKEKIQKQQD; QSETRKEKIQKQQDE;
SETRKEKIQKQQDEY; ETRKEKIQKQQDEYK; TRKEKIQKQQDEYKG;
RKEKIQKQQDEYKGM; KEKIQKQQDEYKGMT; EKIQKQQDEYKGMTK;
KIQKQQDEYKGMTKG; IQKQQDEYKGMTKGS; QKQQDEYKGMTKGSL;
KQQDEYKGMTKGSLN; QQDEYKGMTKGSLNS; QDEYKGMTKGSLNSL;
DEYKGMTKGSLNSLS; EYKGMTKGSLNSLSG; YKGMTKGSLNSLSGE;
KGMTKGSLNSLSGES; GMTKGSLNSLSGESS; MTKGSLNSLSGESGE;
TKGSLNSLSGESGEL; KGSLNSLSGESGELK; GSLNSLSGESGELKE;
SLNSLSGESGELKET; LNSLSGESGELKETI; NSLSGESGELKETIE;
SLSGESGELKETIES; LSGESGELKETIESN; SGESGELKETIESNE;
GESGELKETIESNEI; ESGELKETIESNEID; SGELKETIESNEIDI;
GELKETIESNEIDIT; ELKETIESNEIDITI; LKETIESNEIDITID;
KETIESNEIDITIDS; ETIESNEIDITIDSD; TIESNEIDITIDSDL;
IESNEIDITIDSDLR; ESNEIDITIDSDLRP; SNEIDITIDSDLRPK;
NEIDITIDSDLRPKS; EIDITIDSDLRPKSS; IDITIDSDLRPKSSL;
DITIDSDLRPKSSLQ; ITIDSDLRPKSSLQD; TIDSDLRPKSSLQDI;
IDSDLRPKSSLQDIA; DSDLRPKSSLQDIAG; SDLRPKSSLQDIAGS;
DLRPKSSLQDIAGSN; LRPKSSLQDIAGSNS; RPKSSLQDIAGSNSI;
PKSSLQDIAGSNSIS; KSSLQDIAGSNSISY; SSLQDIAGSNSISYT;
SLQDIAGSNSISYTD; LQDIAGSNSISYTDE; QDIAGSNSISYTDEI;
DIAGSNSISYTDEIE; IAGSNSISYTDEIEE; AGSNSISYTDEIEEE;
GSNSISYTDEIEEED; SNSISYTDEIEEEDY; NSISYTDEIEEEDYA;
SISYTDEIEEEDYAR; ISYTDEIEEEDYARY; SYTDEIEEEDYARYY;

YTDEIEEEDYARYYL; TDEIEEEDYARYYLD; DEIEEEDYARYYLDE;
EIEEEDYARYYLDED; IEEEDYARYYLDEDD; EEEDYARYYLDEDDE;
EEDYARYYLDEDDED; EDYARYYLDEDDEDD; DYARYYLDEDDEDDE;
YARYYLDEDDEDDEY; ARYYLDEDDEDDEYY; RYYLDEDDEDDEYYE;
YYLDEDDEDDEYYED; YLDEDDEDDEYYEDD; LDEDDEDDEYYEDDY;
DEDDEDDEYYEDDYE; EDDEDDEYYEDDYEE; DDEDDEYYEDDYEEI;
DEDDEYYEDDYEEIR; EDDEYYEDDYEEIRL; DDEYYEDDYEEIRLS;
DEYYEDDYEEIRLSN; EYYEDDYEEIRLSNR; YYEDDYEEIRLSNRY;
YEDDYEEIRLSNRYQ; EDDYEEIRLSNRYQS; DDYEEIRLSNRYQSY;
DYEEIRLSNRYQSYL; YEEIRLSNRYQSYLE; EEIRLSNRYQSYLEG;
EIRLSNRYQSYLEGV; IRLSNRYQSYLEGVK; RLSNRYQSYLEGVKY;
LSNRYQSYLEGVKYN; SNRYQSYLEGVKYNV; NRYQSYLEGVKYNVD;
RYQSYLEGVKYNVDS; YQSYLEGVKYNVDSA; QSYLEGVKYNVDSAI;
SYLEGVKYNVDSAIN; YLEGVKYNVDSAINT; LEGVKYNVDSAINTI;
EGVKYNVDSAINTIN; GVKYNVDSAINTINK; VKYNVDSAINTINKI;
KYNVDSAINTINKIY; YNVDSAINTINKIYD; NVDSAINTINKIYDT;
VDSAINTINKIYDTY; DSAINTINKIYDTYT; SAINTINKIYDTYTL;
AINTINKIYDTYTLF; INTINKIYDTYTLFS; NTINKIYDTYTLFST;
TINKIYDTYTLFSTK; INKIYDTYTLFSTKL; NKIYDTYTLFSTKLT;
KIYDTYTLFSTKLTQ; IYDTYTLFSTKLTQM; YDTYTLFSTKLTQMY;
DTYTLFSTKLTQMYS; TYTLFSTKLTQMYST; YTLFSTKLTQMYSTR;
TLFSTKLTQMYSTRL; LFSTKLTQMYSTRLD; FSTKLTQMYSTRLDN;
STKLTQMYSTRLDNL; TKLTQMYSTRLDNLA; KLTQMYSTRLDNLAK;
LTQMYSTRLDNLAKA; TQMYSTRLDNLAKAK; QMYSTRLDNLAKAKA;
MYSTRLDNLAKAKAK; YSTRLDNLAKAKAKE; STRLDNLAKAKAKEE;
TRLDNLAKAKAKEEA; RLDNLAKAKAKEEAA; LDNLAKAKAKEEAAK;
DNLAKAKAKEEAAKF; NLAKAKAKEEAAKFT; LAKAKAKEEAAKFTK;
AKAKAKEEAAKFTKE; KAKAKEEAAKFTKED; AKAKEEAAKFTKEDL;
KAKEEAAKFTKEDLE; AKEEAAKFTKEDLEK; KEEAAKFTKEDLEKN;
EEAAKFTKEDLEKNF; EAAKFTKEDLEKNFK; AAKFTKEDLEKNFKT;
AKFTKEDLEKNFKTL; KFTKEDLEKNFKTLL; FTKEDLEKNFKTLLN;
TKEDLEKNFKTLLNY; KEDLEKNFKTLLNYI; EDLEKNFKTLLNYIQ;
DLEKNFKTLLNYIQV; LEKNFKTLLNYIQVS; EKNFKTLLNYIQVSV;
KNFKTLLNYIQVSVK; NFKTLLNYIQVSVKT; FKTLLNYIQVSVKTA;
KTLLNYIQVSVKTAT; TLLNYIQVSVKTATN; LLNYIQVSVKTATNF;
LNYIQVSVKTATNFV; NYIQVSVKTATNFVY; YIQVSVKTATNFVYI;
IQVSVKTATNFVYIN; QVSVKTATNFVYINE; VSVKTATNFVYINEM;
SVKTATNFVYINEMH; VKTATNFVYINEMHA; KTATNFVYINEMHAK;
TATNFVYINEMHAKR; ATNFVYINEMHAKRK; TNFVYINEMHAKRKL;
NFVYINEMHAKRKLE; FVYINEMHAKRKLEN; VYINEMHAKRKLENI;
YINEMHAKRKLENIE; INEMHAKRKLENIEA; NEMHAKRKLENIEAK;
EMHAKRKLENIEAKI; MHAKRKLENIEAKIK; HAKRKLENIEAKIKT;
AKRKLENIEAKIKTL; KRKLENIEAKIKTLI; RKLENIEAKIKTLIA;
KLENIEAKIKTLIAK; LENIEAKIKTLIAKI; ENIEAKIKTLIAKIK;
NIEAKIKTLIAKIKE; IEAKIKTLIAKIKEK; EAKIKTLIAKIKEKS;
AKIKTLIAKIKEKSN; KIKTLIAKIKEKSNL; IKTLIAKIKEKSNLY;
KTLIAKIKEKSNLYS; TLIAKIKEKSNLYSA; LIAKIKEKSNLYSAY;
IAKIKEKSNLYSAYK; AKIKEKSNLYSAYKA; KIKEKSNLYSAYKAI;
IKEKSNLYSAYKAIV; KEKSNLYSAYKAIVS; EKSNLYSAYKAIVSS;
KSNLYSAYKAIVSSI; SNLYSAYKAIVSSIL; NLYSAYKAIVSSILL;
LYSAYKAIVSSILLM; YSAYKAIVSSILLMR; SAYKAIVSSILLMRD;

AYKAIVSSILLMRDS; YKAIVSSILLMRDSL; KAIVSSILLMRDSLK;
AIVSSILLMRDSLKE; IVSSILLMRDSLKEV; VSSILLMRDSLKEVQ;
SSILLMRDSLKEVQY; SILLMRDSLKEVQYA; ILLMRDSLKEVQYAI;
LLMRDSLKEVQYAID; LMRDSLKEVQYAIDK; MRDSLKEVQYAIDKN;
RDSLKEVQYAIDKNG; DSLKEVQYAIDKNGI; SLKEVQYAIDKNGIW;
LKEVQYAIDKNGIWY; KEVQYAIDKNGIWYR; EVQYAIDKNGIWYRK;
VQYAIDKNGIWYRKL; QYAIDKNGIWYRKLD; YAIDKNGIWYRKLDA;
AIDKNGIWYRKLDAI; IDKNGIWYRKLDAIE; DKNGIWYRKLDAIET;
KNGIWYRKLDAIETE; NGIWYRKLDAIETEI; GIWYRKLDAIETEIK;
IWYRKLDAIETEIKN; WYRKLDAIETEIKNL; YRKLDAIETEIKNLI;
RKLDAIETEIKNLIL; KLDAIETEIKNLILK; LDAIETEIKNLILKI;
DAIETEIKNLILKIK; AIETEIKNLILKIKG; IETEIKNLILKIKGQ;
ETEIKNLILKIKGQS; TEIKNLILKIKGQSD; EIKNLILKIKGQSDL;
IKNLILKIKGQSDLY; KNLILKIKGQSDLYE; NLILKIKGQSDLYEA;
LILKIKGQSDLYEAY; ILKIKGQSDLYEAYK; LKIKGQSDLYEAYKA;
KIKGQSDLYEAYKAI; IKGQSDLYEAYKAIV; KGQSDLYEAYKAIVR;
GQSDLYEAYKAIVRS; QSDLYEAYKAIVRSI; SDLYEAYKAIVRSIL;
DLYEAYKAIVRSILL; LYEAYKAIVRSILLM; YEAYKAIVRSILLMK;
EAYKAIVRSILLMKD; AYKAIVRSILLMKDS; YKAIVRSILLMKDSL;
KAIVRSILLMKDSLK; AIVRSILLMKDSLKI; IVRSILLMKDSLKII;
VRSILLMKDSLKIIE; RSILLMKDSLKIIEI; SILLMKDSLKIIEIV;
ILLMKDSLKIIEIVI; LLMKDSLKIIEIVID; LMKDSLKIIEIVIDK;
MKDSLKIIEIVIDKN; KDSLKIIEIVIDKNG; DSLKIIEIVIDKNGV;
SLKIIEIVIDKNGVW; LKIIEIVIDKNGVWY;

16 mers:
MKKVKSKYLALGLLFG; KKVKSKYLALGLLFGF; KVKSKYLALGLLFGFI;
VKSKYLALGLLFGFIS; KSKYLALGLLFGFISC; SKYLALGLLFGFISCD;
KYLALGLLFGFISCDL; YLALGLLFGFISCDLF; LALGLLFGFISCDLFI;
ALGLLFGFISCDLFIR; LGLLFGFISCDLFIRY; GLLFGFISCDLFIRYE;
LLFGFISCDLFIRYEM; LFGFISCDLFIRYEMK; FGFISCDLFIRYEMKE;
GFISCDLFIRYEMKEE; FISCDLFIRYEMKEES; ISCDLFIRYEMKEESP;
SCDLFIRYEMKEESPG; CDLFIRYEMKEESPGL; DLFIRYEMKEESPGLF;
LFIRYEMKEESPGLFD; FIRYEMKEESPGLFDK; IRYEMKEESPGLFDKG;
RYEMKEESPGLFDKGN; YEMKEESPGLFDKGNS; EMKEESPGLFDKGNSI;
MKEESPGLFDKGNSIL; KEESPGLFDKGNSILE; EESPGLFDKGNSILET;
ESPGLFDKGNSILETS; SPGLFDKGNSILETSE; PGLFDKGNSILETSEE;
GLFDKGNSILETSEES; LFDKGNSILETSEESI; FDKGNSILETSEESIK;
DKGNSILETSEESIKK; KGNSILETSEESIKKP; GNSILETSEESIKKPM;
NSILETSEESIKKPMN; SILETSEESIKKPMNK; ILETSEESIKKPMNKK;
LETSEESIKKPMNKKG; ETSEESIKKPMNKKGK; TSEESIKKPMNKKGKG;
SEESIKKPMNKKGKGK; EESIKKPMNKKGKGKI; ESIKKPMNKKGKGKIA;
SIKKPMNKKGKGKIAR; IKKPMNKKGKGKIARK; KKPMNKKGKGKIARKN;
KPMNKKGKGKIARKNG; PMNKKGKGKIARKNGK; MNKKGKGKIARKNGKS;
NKKGKGKIARKNGKSK; KKGKGKIARKNGKSKV; KGKGKIARKNGKSKVS;
GKGKIARKNGKSKVSG; KGKIARKNGKSKVSGK; GKIARKNGKSKVSGKE;
KIARKNGKSKVSGKEP; IARKNGKSKVSGKEPF; ARKNGKSKVSGKEPFI;
RKNGKSKVSGKEPFIH; KNGKSKVSGKEPFIHS; NGKSKVSGKEPFIHSF;
GKSKVSGKEPFIHSFK; KSKVSGKEPFIHSFKR; SKVSGKEPFIHSFKRD;
KVSGKEPFIHSFKRDA; VSGKEPFIHSFKRDAA; SGKEPFIHSFKRDAAN;
GKEPFIHSFKRDAANK; KEPFIHSFKRDAANKS; EPFIHSFKRDAANKSN;

PFIHSFKRDAANKSNF; FIHSFKRDAANKSNFL; IHSFKRDAANKSNFLQ;
HSFKRDAANKSNFLQK; SFKRDAANKSNFLQKN; FKRDAANKSNFLQKNV;
KRDAANKSNFLQKNVM; RDAANKSNFLQKNVML; DAANKSNFLQKNVMLE;
AANKSNFLQKNVMLEE; ANKSNFLQKNVMLEEE; NKSNFLQKNVMLEEES;
KSNFLQKNVMLEEESL; SNFLQKNVMLEEESLK; NFLQKNVMLEEESLKT;
FLQKNVMLEEESLKTE; LQKNVMLEEESLKTEL; QKNVMLEEESLKTELL;
KNVMLEEESLKTELLK; NVMLEEESLKTELLKE; VMLEEESLKTELLKEQ;
MLEEESLKTELLKEQS; LEEESLKTELLKEQSE; EEESLKTELLKEQSET;
EESLKTELLKEQSETR; ESLKTELLKEQSETRK; SLKTELLKEQSETRKE;
LKTELLKEQSETRKEK; KTELLKEQSETRKEKI; TELLKEQSETRKEKIQ;
ELLKEQSETRKEKIQK; LLKEQSETRKEKIQKQ; LKEQSETRKEKIQKQQ;
KEQSETRKEKIQKQQD; EQSETRKEKIQKQQDE; QSETRKEKIQKQQDEY;
SETRKEKIQKQQDEYK; ETRKEKIQKQQDEYKG; TRKEKIQKQQDEYKGM;
RKEKIQKQQDEYKGMT; KEKIQKQQDEYKGMTK; EKIQKQQDEYKGMTKG;
KIQKQQDEYKGMTKGS; IQKQQDEYKGMTKGSL; QKQQDEYKGMTKGSLN;
KQQDEYKGMTKGSLNS; QQDEYKGMTKGSLNSL; QDEYKGMTKGSLNSLS;
DEYKGMTKGSLNSLSG; EYKGMTKGSLNSLSGE; YKGMTKGSLNSLSGES;
KGMTKGSLNSLSGESG; GMTKGSLNSLSGESGE; MTKGSLNSLSGESGEL;
TKGSLNSLSGESGELK; KGSLNSLSGESGELKE; GSLNSLSGESGELKET;
SLNSLSGESGELKETI; LNSLSGESGELKETIE; NSLSGESGELKETIES;
SLSGESGELKETIESN; LSGESGELKETIESNE; SGESGELKETIESNEI;
GESGELKETIESNEID; ESGELKETIESNEIDI; SGELKETIESNEIDIT;
GELKETIESNEIDITI; ELKETIESNEIDITID; LKETIESNEIDITIDS;
KETIESNEIDITIDSD; ETIESNEIDITIDSDL; TIESNEIDITIDSDLR;
IESNEIDITIDSDLRP; ESNEIDITIDSDLRPK; SNEIDITIDSDLRPKS;
NEIDITIDSDLRPKSS; EIDITIDSDLRPKSSL; IDITIDSDLRPKSSLQ;
DITIDSDLRPKSSLQD; ITIDSDLRPKSSLQDI; TIDSDLRPKSSLQDIA;
IDSDLRPKSSLQDIAG; DSDLRPKSSLQDIAGS; SDLRPKSSLQDIAGSN;
DLRPKSSLQDIAGSNS; LRPKSSLQDIAGSNSI; RPKSSLQDIAGSNSIS;
PKSSLQDIAGSNSISY; KSSLQDIAGSNSISYT; SSLQDIAGSNSISYTD;
SLQDIAGSNSISYTDE; LQDIAGSNSISYTDEI; QDIAGSNSISYTDEIE;
DIAGSNSISYTDEIEE; IAGSNSISYTDEIEEE; AGSNSISYTDEIEEED;
GSNSISYTDEIEEEDY; SNSISYTDEIEEEDYA; NSISYTDEIEEEDYAR;
SISYTDEIEEEDYARY; ISYTDEIEEEDYARYY; SYTDEIEEEDYARYYL;
YTDEIEEEDYARYYLD; TDEIEEEDYARYYLDE; DEIEEEDYARYYLDED;
EIEEEDYARYYLDEDD; IEEEDYARYYLDEDDE; EEEDYARYYLDEDDED;
EEDYARYYLDEDDEDD; EDYARYYLDEDDEDDE; DYARYYLDEDDEDDEY;
YARYYLDEDDEDDEYY; ARYYLDEDDEDDEYYE; RYYLDEDDEDDEYYED;
YYLDEDDEDDEYYEDD; YLDEDDEDDEYYEDDY; LDEDDEDDEYYEDDYE;
DEDDEDDEYYEDDYEE; EDDEDDEYYEDDYEEI; DDEDDEYYEDDYEEIR;
DEDDEYYEDDYEEIRL; EDDEYYEDDYEEIRLS; DDEYYEDDYEEIRLSN;
DEYYEDDYEEIRLSNR; EYYEDDYEEIRLSNRY; YYEDDYEEIRLSNRYQ;
YEDDYEEIRLSNRYQS; EDDYEEIRLSNRYQSY; DDYEEIRLSNRYQSYL;
DYEEIRLSNRYQSYLE; YEEIRLSNRYQSYLEG; EEIRLSNRYQSYLEGV;
EIRLSNRYQSYLEGVK; IRLSNRYQSYLEGVKY; RLSNRYQSYLEGVKYN;
LSNRYQSYLEGVKYNV; SNRYQSYLEGVKYNVD; NRYQSYLEGVKYNVDS;
RYQSYLEGVKYNVDSA; YQSYLEGVKYNVDSAI; QSYLEGVKYNVDSAIN;
SYLEGVKYNVDSAINT; YLEGVKYNVDSAINTI; LEGVKYNVDSAINTIN;
EGVKYNVDSAINTINK; GVKYNVDSAINTINKI; VKYNVDSAINTINKIY;
KYNVDSAINTINKIYD; YNVDSAINTINKIYDT; NVDSAINTINKIYDTY;
VDSAINTINKIYDTYT; DSAINTINKIYDTYTL; SAINTINKIYDTYTLF;

| | | |
|---|---|---|
| AINTINKIYDTYTLFS; | INTINKIYDTYTLFST; | NTINKIYDTYTLFSTK; |
| TINKIYDTYTLFSTKL; | INKIYDTYTLFSTKLT; | NKIYDTYTLFSTKLTQ; |
| KIYDTYTLFSTKLTQM; | IYDTYTLFSTKLTQMY; | YDTYTLFSTKLTQMYS; |
| DTYTLFSTKLTQMYST; | TYTLFSTKLTQMYSTR; | YTLFSTKLTQMYSTRL; |
| TLFSTKLTQMYSTRLD; | LFSTKLTQMYSTRLDN; | FSTKLTQMYSTRLDNL; |
| STKLTQMYSTRLDNLA; | TKLTQMYSTRLDNLAK; | KLTQMYSTRLDNLAKA; |
| LTQMYSTRLDNLAKAK; | TQMYSTRLDNLAKAKA; | QMYSTRLDNLAKAKAK; |
| MYSTRLDNLAKAKAKE; | YSTRLDNLAKAKAKEE; | STRLDNLAKAKAKEEA; |
| TRLDNLAKAKAKEEAA; | RLDNLAKAKAKEEAAK; | LDNLAKAKAKEEAAKF; |
| DNLAKAKAKEEAAKFT; | NLAKAKAKEEAAKFTK; | LAKAKAKEEAAKFTKE; |
| AKAKAKEEAAKFTKED; | KAKAKEEAAKFTKEDL; | AKAKEEAAKFTKEDLE; |
| KAKEEAAKFTKEDLEK; | AKEEAAKFTKEDLEKN; | KEEAAKFTKEDLEKNF; |
| EEAAKFTKEDLEKNFK; | EAAKFTKEDLEKNFKT; | AAKFTKEDLEKNFKTL; |
| AKFTKEDLEKNFKTLL; | KFTKEDLEKNFKTLLN; | FTKEDLEKNFKTLLNY; |
| TKEDLEKNFKTLLNYI; | KEDLEKNFKTLLNYIQ; | EDLEKNFKTLLNYIQV; |
| DLEKNFKTLLNYIQVS; | LEKNFKTLLNYIQVSV; | EKNFKTLLNYIQVSVK; |
| KNFKTLLNYIQVSVKT; | NFKTLLNYIQVSVKTA; | FKTLLNYIQVSVKTAT; |
| KTLLNYIQVSVKTATN; | TLLNYIQVSVKTATNF; | LLNYIQVSVKTATNFV; |
| LNYIQVSVKTATNFVY; | NYIQVSVKTATNFVYI; | YIQVSVKTATNFVYIN; |
| IQVSVKTATNFVYINE; | QVSVKTATNFVYINEM; | VSVKTATNFVYINEMH; |
| SVKTATNFVYINEMHA; | VKTATNFVYINEMHAK; | KTATNFVYINEMHAKR; |
| TATNFVYINEMHAKRK; | ATNFVYINEMHAKRKL; | TNFVYINEMHAKRKLE; |
| NFVYINEMHAKRKLEN; | FVYINEMHAKRKLENI; | VYINEMHAKRKLENIE; |
| YINEMHAKRKLENIEA; | INEMHAKRKLENIEAK; | NEMHAKRKLENIEAKI; |
| EMHAKRKLENIEAKIK; | MHAKRKLENIEAKIKT; | HAKRKLENIEAKIKTL; |
| AKRKLENIEAKIKTLI; | KRKLENIEAKIKTLIA; | RKLENIEAKIKTLIAK; |
| KLENIEAKIKTLIAKI; | LENIEAKIKTLIAKIK; | ENIEAKIKTLIAKIKE; |
| NIEAKIKTLIAKIKEK; | IEAKIKTLIAKIKEKS; | EAKIKTLIAKIKEKSN; |
| AKIKTLIAKIKEKSNL; | KIKTLIAKIKEKSNLY; | IKTLIAKIKEKSNLYS; |
| KTLIAKIKEKSNLYSA; | TLIAKIKEKSNLYSAY; | LIAKIKEKSNLYSAYK; |
| IAKIKEKSNLYSAYKA; | AKIKEKSNLYSAYKAI; | KIKEKSNLYSAYKAIV; |
| IKEKSNLYSAYKAIVS; | KEKSNLYSAYKAIVSS; | EKSNLYSAYKAIVSSI; |
| KSNLYSAYKAIVSSIL; | SNLYSAYKAIVSSILL; | NLYSAYKAIVSSILLM; |
| LYSAYKAIVSSILLMR; | YSAYKAIVSSILLMRD; | SAYKAIVSSILLMRDS; |
| AYKAIVSSILLMRDSL; | YKAIVSSILLMRDSLK; | KAIVSSILLMRDSLKE; |
| AIVSSILLMRDSLKEV; | IVSSILLMRDSLKEVQ; | VSSILLMRDSLKEVQY; |
| SSILLMRDSLKEVQYA; | SILLMRDSLKEVQYAI; | ILLMRDSLKEVQYAID; |
| LLMRDSLKEVQYAIDK; | LMRDSLKEVQYAIDKN; | MRDSLKEVQYAIDKNG; |
| RDSLKEVQYAIDKNGI; | DSLKEVQYAIDKNGIW; | SLKEVQYAIDKNGIWY; |
| LKEVQYAIDKNGIWYR; | KEVQYAIDKNGIWYRK; | EVQYAIDKNGIWYRKL; |
| VQYAIDKNGIWYRKLD; | QYAIDKNGIWYRKLDA; | YAIDKNGIWYRKLDAI; |
| AIDKNGIWYRKLDAIE; | IDKNGIWYRKLDAIET; | DKNGIWYRKLDAIETE; |
| KNGIWYRKLDAIETEI; | NGIWYRKLDAIETEIK; | GIWYRKLDAIETEIKN; |
| IWYRKLDAIETEIKNL; | WYRKLDAIETEIKNLI; | YRKLDAIETEIKNLIL; |
| RKLDAIETEIKNLILK; | KLDAIETEIKNLILKI; | LDAIETEIKNLILKIK; |
| DAIETEIKNLILKIKG; | AIETEIKNLILKIKGQ; | IETEIKNLILKIKGQS; |
| ETEIKNLILKIKGQSD; | TEIKNLILKIKGQSDL; | EIKNLILKIKGQSDLY; |
| IKNLILKIKGQSDLYE; | KNLILKIKGQSDLYEA; | NLILKIKGQSDLYEAY; |
| LILKIKGQSDLYEAYK; | ILKIKGQSDLYEAYKA; | LKIKGQSDLYEAYKAI; |
| KIKGQSDLYEAYKAIV; | IKGQSDLYEAYKAIVR; | KGQSDLYEAYKAIVRS; |
| GQSDLYEAYKAIVRSI; | QSDLYEAYKAIVRSIL; | SDLYEAYKAIVRSILL; |

| | DLYEAYKAIVRSILLM; LYEAYKAIVRSILLMK; YEAYKAIVRSILLMKD; EAYKAIVRSILLMKDS; AYKAIVRSILLMKDSL; YKAIVRSILLMKDSLK; KAIVRSILLMKDSLKI; AIVRSILLMKDSLKII; IVRSILLMKDSLKIIE; VRSILLMKDSLKIIEI; RSILLMKDSLKIIEIV; SILLMKDSLKIIEIVI; ILLMKDSLKIIEIVID; LLMKDSLKIIEIVIDK; LMKDSLKIIEIVIDKN; MKDSLKIIEIVIDKNG; KDSLKIIEIVIDKNGV; DSLKIIEIVIDKNGVW; SLKIIEIVIDKNGVWY; |
|---|---|

**Fig. 34.**

| Antigen designation | HLA-chain Class 2 | Amino acid sequence 15 mer peptide | 9mer core peptide |
|---|---|---|---|
| NP_212517.1 Basic membrane protein A (bmpA) [Borrelia burgdorferi B31 Seq1 | HLA-DRB1*0101 | GSFADLEAGRSVATR | LEAGRSVAT |
| | | IGSFADLEAGRSVAT | FADLEAGRS |
| | | FADLEAGRSVATRMY | LEAGRSVAT |
| | | SFADLEAGRSVATRM | LEAGRSVAT |
| | | ADLEAGRSVATRMYS | LEAGRSVAT |
| | | AFLTGYIAAKLSKTG | YIAAKLSKT |
| | | FLTGYIAAKLSKTGK | IAAKLSKTG |
| | | LTGYIAAKLSKTGKI | IAAKLSKTG |
| | | TGYIAAKLSKTGKIG | IAAKLSKTG |
| | | DLEAGRSVATRMYSD | LEAGRSVAT |
| | | LEAGRSVATRMYSDE | LEAGRSVAT |
| | | GAFLTGYIAAKLSKT | LTGYIAAKL |
| | | GYIAAKLSKTGKIGF | IAAKLSKTG |
| | | IHHAAGLGGIGAIEV | IHHAAGLGG |
| | | LVGMTFRAQEGAFLT | FRAQEGAFL |
| | | NLVGMTFRAQEGAFL | LVGMTFRAQ |
| | | HHAAGLGGIGAIEVA | LGGIGAIEV |
| | | HAAGLGGIGAIEVAK | LGGIGAIEV |
| | | AAGLGGIGAIEVAKE | LGGIGAIEV |
| | | AGLGGIGAIEVAKEL | LGGIGAIEV |
| | | EGAFLTGYIAAKLSK | LTGYIAAKL |
| | | YIAAKLSKTGKIGFL | IAAKLSKTG |
| | | GMTFRAQEGAFLTGY | FRAQEGAFL |
| | | QEGAFLTGYIAAKLS | LTGYIAAKL |
| | | EKEIDNLSSKIINKE | IDNLSSKII |
| | | LEKEIDNLSSKIINK | IDNLSSKII |
| | | KEIDNLSSKIINKEI | IDNLSSKII |
| | | MTFRAQEGAFLTGYI | FRAQEGAFL |
| | | ELEKEIDNLSSKIIN | IDNLSSKII |
| | | VGMTFRAQEGAFLTG | FRAQEGAFL |
| | | FELEKEIDNLSSKII | FELEKEIDN |
| | | DMKYAIIDPIYSNDP | YAIIDPIYS |
| | | MKYAIIDPIYSNDPI | YAIIDPIYS |
| | | DPIPANLVGMTFRAQ | IPANLVGMT |
| | | TQYIGSFADLEAGRS | IGSFADLEA |
| | | QYIGSFADLEAGRSV | FADLEAGRS |
| | | YIGSFADLEAGRSVA | FADLEAGRS |
| | | IPANLVGMTFRAQEG | LVGMTFRAQ |
| | | PANLVGMTFRAQEGA | LVGMTFRAQ |
| | | PIPANLVGMTFRAQE | LVGMTFRAQ |
| | | AQEGAFLTGYIAAKL | FLTGYIAAK |
| | | SDLIWLIGYRFSDVA | LIWLIGYRF |
| | | ANLVGMTFRAQEGAF | LVGMTFRAQ |
| | | GSDLIWLIGYRFSDV | LIWLIGYRF |
| | | VAKVAALQNPDMKYA | VAALQNPDM |
| | | DAGSDLIWLIGYRFS | LIWLIGYRF |
| | | AGSDLIWLIGYRFSD | LIWLIGYRF |
| | | IDIIHHAAGLGGIGA | IHHAAGLGG |
| | | PDMKYAIIDPIYSND | YAIIDPIYS |
| | | NPDMKYAIIDPIYSN | YAIIDPIYS |
| | | AKVAALQNPDMKYAI | LQNPDMKYA |
| | | QNPDMKYAIIDPIYS | MKYAIIDPI |

| | | | |
|---|---|---|---|
| | | FSDVAKVAALQNPDM | VAKVAALQN |
| | | EIDIIHHAAGLGGIG | IHHAAGLGG |
| | | DEIDIIHHAAGLGGI | IHHAAGLGG |
| | | SDVAKVAALQNPDMK | VAKVAALQN |
| | | SDEIDIIHHAAGLGG | IIHHAAGLG |
| | | YLAPDNVITSTTKDV | YLAPDNVIT |
| | | DPIYSNDPIPANLVG | NDPIPANLV |
| | | IDPIYSNDPIPANLV | IYSNDPIPA |
| | | LIWLIGYRFSDVAKV | LIWLIGYRF |
| | | VAALQNPDMKYAIID | LQNPDMKYA |
| | | KVAALQNPDMKYAII | LQNPDMKYA |
| | | DIIHHAAGLGGIGAI | IHHAAGLGG |
| | | GYRFSDVAKVAALQN | YRFSDVAKV |
| | | YRFSDVAKVAALQNP | VAKVAALQN |
| | | YAIIDPIYSNDPIPA | YAIIDPIYS |
| | | TFRAQEGAFLTGYIA | FRAQEGAFL |
| | | KDAGSDLIWLIGYRF | GSDLIWLIG |
| | | GLGGIGAIEVAKELG | LGGIGAIEV |
| | | IYSNDPIPANLVGMT | NDPIPANLV |
| | | LGGIGAIEVAKELGS | LGGIGAIEV |
| | | IAAKLSKTGKIGFLG | IAAKLSKTG |
| | | CSGKGSLGSEIPKVS | KGSLGSEIP |
| | | FRAQEGAFLTGYIAA | FRAQEGAFL |
| | | PIYSNDPIPANLVGM | YSNDPIPAN |
| | | DQAYLAPDNVITSTT | YLAPDNVIT |
| | | LAPDNVITSTTKDVG | VITSTTKDV |
| | | QAYLAPDNVITSTTK | YLAPDNVIT |
| | | AALQNPDMKYAIIDP | LQNPDMKYA |
| | | DLIWLIGYRFSDVAK | LIWLIGYRF |
| | | PDNVITSTTKDVGRA | VITSTTKDV |
| | | SGKGSLGSEIPKVSL | LGSEIPKVS |
| | | LSDLEGLKDAGSDLI | LEGLKDAGS |
| | | RFSDVAKVAALQNPD | VAKVAALQN |
| | | KYAIIDPIYSNDPIP | YAIIDPIYS |
| | | APDNVITSTTKDVGR | VITSTTKDV |
| | | AIIDPIYSNDPIPAN | IYSNDPIPA |
| | | SDLEGLKDAGSDLIW | LKDAGSDLI |
| | | KGSLGSEIPKVSLII | LGSEIPKVS |
| | | EDQAYLAPDNVITST | YLAPDNVIT |
| | | YEAGAKYANKDIKIS | YEAGAKYAN |
| | | DEDQAYLAPDNVITS | YLAPDNVIT |
| | | VGFVRNPKMISFELE | FVRNPKMIS |
| | | VVGFVRNPKMISFEL | FVRNPKMIS |
| | | YSNDPIPANLVGMTF | IPANLVGMT |
| | | DVAKVAALQNPDMKY | VAALQNPDM |
| | | EIDNLSSKIINKEII | IDNLSSKII |
| | | VDEDQAYLAPDNVIT | VDEDQAYLA |
| | | IDNLSSKIINKEIIV | IDNLSSKII |
| | | DNVITSTTKDVGRAL | VITSTTKDV |
| | | GVVGFVRNPKMISFE | FVRNPKMIS |
| | | AKYANKDIKISTQYI | YANKDIKIS |
| | | EGVVGFVRNPKMISF | FVRNPKMIS |
| | | GKGSLGSEIPKVSLI | LGSEIPKVS |
| | | MNKILLLILLESIVF | MNKILLLIL |
| | | KDVGRALNIFTSNHL | VGRALNIFT |
| | | GYRFSDVAKVAALQ | YRFSDVAKV |
| | | GSLGSEIPKVSLIID | LGSEIPKVS |

| | | | |
|---|---|---|---|
| | | LEGLKDAGSDLIWLI | LKDAGSDLI |
| | | NKILLLILLESIVFL | LILLESIVF |
| | | IIDPIYSNDPIPANL | IYSNDPIPA |
| | | EGLKDAGSDLIWLIG | LKDAGSDLI |
| | | AGAKYANKDIKISTQ | YANKDIKIS |
| | | IIHHAAGLGGIGAIE | IHHAAGLGG |
| | | EAGAKYANKDIKIST | YANKDIKIS |
| | | GAKYANKDIKISTQY | YANKDIKIS |
| | | IWLIGYRFSDVAKVA | YRFSDVAKV |
| | | LIGYRFSDVAKVAAL | YRFSDVAKV |
| | | WLIGYRFSDVAKVAA | YRFSDVAKV |
| | | GGKLINYGLKEGVVG | LINYGLKEG |
| | | GKLINYGLKEGVVGF | YGLKEGVVG |
| | | KILLLILLESIVFLS | LILLESIVF |
| | | IELVLKESSSNSYLS | LKESSSNSY |
| | | KIELVLKESSSNSYL | LKESSSNSY |
| | | VGRALNIFTSNHLKT | LNIFTSNHL |
| | | FKIELVLKESSSNSY | LVLKESSSN |
| | | KLINYGLKEGVVGFV | YGLKEGVVG |
| | | ILLLILLESIVFLSC | LILLESIVF |
| | | GRALNIFTSNHLKTN | LNIFTSNHL |
| | | LINYGLKEGVVGFVR | YGLKEGVVG |
| | | DVGRALNIFTSNHLK | LNIFTSNHL |
| | | NDPIPANLVGMTFRA | IPANLVGMT |
| | | DKSFNESALNGVKKV | FNESALNGV |
| | | RALNIFTSNHLKTNT | LNIFTSNHL |
| | | GFVRNPKMISFELEK | VRNPKMISF |
| | | ELVLKESSSNSYLSD | LKESSSNSY |
| | | SNDPIPANLVGMTFR | IPANLVGMT |
| | | DDKSFNESALNGVKK | FNESALNGV |
| | | LLLILLESIVFLSCS | LILLESIVF |
| | | AYLAPDNVITSTTKD | YLAPDNVIT |
| | | ALQNPDMKYAIIDPI | LQNPDMKYA |
| | | FDDKSFNESALNGVK | FNESALNGV |
| | | TFDDKSFNESALNGV | SFNESALNG |
| | | LQNPDMKYAIIDPIY | LQNPDMKYA |
| | | TKDVGRALNIFTSNH | VGRALNIFT |
| | | TTKDVGRALNIFTSN | VGRALNIFT |
| | | KEGVVGFVRNPKMIS | VGFVRNPKM |
| | | SIVFLSCSGKGSLGS | FLSCSGKGS |
| | | YSDEIDIIHHAAGLG | EIDIIHHAA |
| | | INYGLKEGVVGFVRN | YGLKEGVVG |
| | | STTKDVGRALNIFTS | VGRALNIFT |
| | HLA-DRB1*0301 | None | |
| | HLA-DRB1*0401 | FKIELVLKESSSNSY | VLKESSSNS |
| | | IELVLKESSSNSYLS | LKESSSNSY |
| | | ELVLKESSSNSYLSD | LKESSSNSY |
| | | KIELVLKESSSNSYL | LKESSSNSY |
| | | LVLKESSSNSYLSDL | LKESSSNSY |
| | | KIINKEIIVPSNKES | INKEIIVPS |
| | | SKIINKEIIVPSNKE | INKEIIVPS |
| | | SSKIINKEIIVPSNK | INKEIIVPS |
| | | LSSKIINKEIIVPSN | INKEIIVPS |
| | | NLSSKIINKEIIVPS | LSSKIINKE |
| | | EGVVGFVRNPKMISF | FVRNPKMIS |
| | | KEGVVGFVRNPKMIS | VVGFVRNPK |

| | | | |
|---|---|---|---|
| | | ISFELEKEIDNLSSK | LEKEIDNLS |
| | | MISFELEKEIDNLSS | LEKEIDNLS |
| | | GVVGFVRNPKMISFE | FVRNPKMIS |
| | | FELEKEIDNLSSKII | LEKEIDNLS |
| | | SFELEKEIDNLSSKI | LEKEIDNLS |
| | | AIIDPIYSNDPIPAN | IYSNDPIPA |
| | | VVGFVRNPKMISFEL | FVRNPKMIS |
| | | VGFVRNPKMISFELE | FVRNPKMIS |
| | | IIDPIYSNDPIPANL | IYSNDPIPA |
| | | IDPIYSNDPIPANLV | IYSNDPIPA |
| | | KMISFELEKEIDNLS | MISFELEKE |
| | | DPIYSNDPIPANLVG | IYSNDPIPA |
| | | VLKESSSNSYLSDLE | LKESSSNSY |
| | | LKESSSNSYLSDLEG | LKESSSNSY |
| | | RALNIFTSNHLKTNT | LNIFTSNHL |
| | | GRALNIFTSNHLKTN | LNIFTSNHL |
| | | VGRALNIFTSNHLKT | LNIFTSNHL |
| | | IINKEIIVPSNKESY | INKEIIVPS |
| | | KDVGRALNIFTSNHL | VGRALNIFT |
| | | INKEIIVPSNKESYE | INKEIIVPS |
| | | DVGRALNIFTSNHLK | LNIFTSNHL |
| | | YAIIDPIYSNDPIPA | IDPIYSNDP |
| | HLA-DRB1*0404 | VGRALNIFTSNHLKT | ALNIFTSNH |
| | | GRALNIFTSNHLKTN | ALNIFTSNH |
| | | KDVGRALNIFTSNHL | ALNIFTSNH |
| | | DVGRALNIFTSNHLK | ALNIFTSNH |
| | | TKDVGRALNIFTSNH | VGRALNIFT |
| | | RALNIFTSNHLKTNT | ALNIFTSNH |
| | | ALNIFTSNHLKTNTF | ALNIFTSNH |
| | | FKIELVLKESSSNSY | VLKESSSNS |
| | | KIELVLKESSSNSYL | VLKESSSNS |
| | | IELVLKESSSNSYLS | VLKESSSNS |
| | | ELVLKESSSNSYLSD | VLKESSSNS |
| | HLA-DRB1*0405 | AIIDPIYSNDPIPAN | IYSNDPIPA |
| | | IIDPIYSNDPIPANL | YSNDPIPAN |
| | | IDPIYSNDPIPANLV | YSNDPIPAN |
| | | DPIYSNDPIPANLVG | YSNDPIPAN |
| | | PIYSNDPIPANLVGM | YSNDPIPAN |
| | | KIINKEIIVPSNKES | EIIVPSNKE |
| | | KEGVVGFVRNPKMIS | VVGFVRNPK |
| | | EGVVGFVRNPKMISF | FVRNPKMIS |
| | | IYSNDPIPANLVGMT | YSNDPIPAN |
| | | INKEIIVPSNKESYE | EIIVPSNKE |
| | | SKIINKEIIVPSNKE | INKEIIVPS |
| | | GVVGFVRNPKMISFE | FVRNPKMIS |
| | | IINKEIIVPSNKESY | EIIVPSNKE |
| | HLA-DRB1*0701 | VGRALNIFTSNHLKT | LNIFTSNHL |
| | | GRALNIFTSNHLKTN | LNIFTSNHL |
| | | DVGRALNIFTSNHLK | LNIFTSNHL |
| | | KDVGRALNIFTSNHL | ALNIFTSNH |
| | | RALNIFTSNHLKTNT | LNIFTSNHL |
| | | LAPDNVITSTTKDVG | VITSTTKDV |
| | | APDNVITSTTKDVGR | VITSTTKDV |
| | | PDNVITSTTKDVGRA | VITSTTKDV |
| | | DNVITSTTKDVGRAL | VITSTTKDV |
| | HLA-DRB1*0802 | None | |

| | HLA-DRB1*0901 | WLIGYRFSDVAKVAA | YRFSDVAKV |
|---|---|---|---|
| | | LIGYRFSDVAKVAAL | YRFSDVAKV |
| | | IGYRFSDVAKVAALQ | YRFSDVAKV |
| | | LEGLKDAGSDLIWLI | LKDAGSDLI |
| | | GAFLTGYIAAKLSKT | LTGYIAAKL |
| | | DLEGLKDAGSDLIWL | LKDAGSDLI |
| | | SDLEGLKDAGSDLIW | LKDAGSDLI |
| | | LSDLEGLKDAGSDLI | LEGLKDAGS |
| | | LIWLIGYRFSDVAKV | LIGYRFSDV |
| | | EGLKDAGSDLIWLIG | LKDAGSDLI |
| | | IWLIGYRFSDVAKVA | YRFSDVAKV |
| | | AFLTGYIAAKLSKTG | YIAAKLSKT |
| | HLA-DRB1*1101 | GSDLIWLIGYRFSDV | LIWLIGYRF |
| | | SDLIWLIGYRFSDVA | LIWLIGYRF |
| | | AGSDLIWLIGYRFSD | LIWLIGYRF |
| | | DAGSDLIWLIGYRFS | LIWLIGYRF |
| | | PIPANLVGMTFRAQE | LVGMTFRAQ |
| | | PANLVGMTFRAQEGA | LVGMTFRAQ |
| | | IPANLVGMTFRAQEG | LVGMTFRAQ |
| | | DLIWLIGYRFSDVAK | LIWLIGYRF |
| | | ANLVGMTFRAQEGAF | LVGMTFRAQ |
| | | DPIPANLVGMTFRAQ | PANLVGMTF |
| | HLA-DRB1*1302 | KYANKDIKISTQYIG | YANKDIKIS |
| | | NLSSKIINKEIIVPS | IINKEIIVP |
| | | LSSKIINKEIIVPSN | INKEIIVPS |
| | | YANKDIKISTQYIGS | IKISTQYIG |
| | | SSKIINKEIIVPSNK | IINKEIIVP |
| | | SKIINKEIIVPSNKE | IINKEIIVP |
| | | ANKDIKISTQYIGSF | IKISTQYIG |
| | | NKDIKISTQYIGSFA | IKISTQYIG |
| | | KDIKISTQYIGSFAD | IKISTQYIG |
| | | VGRALNIFTSNHLKT | LNIFTSNHL |
| | | KIINKEIIVPSNKES | INKEIIVPS |
| | | EGVVGFVRNPKMISF | FVRNPKMIS |
| | | MNKILLLILLESIVF | ILLLILLES |
| | | KDVGRALNIFTSNHL | VGRALNIFT |
| | | DNLSSKIINKEIIVP | LSSKIINKE |
| | | RALNIFTSNHLKTNT | LNIFTSNHL |
| | | KEGVVGFVRNPKMIS | VGFVRNPKM |
| | | NKILLLILLESIVFL | ILLLILLES |
| | | GVVGFVRNPKMISFE | FVRNPKMIS |
| | | GRALNIFTSNHLKTN | LNIFTSNHL |
| | | GGKLINYGLKEGVVG | LINYGLKEG |
| | | VVGFVRNPKMISFEL | FVRNPKMIS |
| | | DVGRALNIFTSNHLK | LNIFTSNHL |
| | | ILLLILLESIVFLSC | LILLESIVF |
| | | KILLLILLESIVFLS | ILLLILLES |
| | | ELEKEIDNLSSKIIN | IDNLSSKII |
| | | GKLINYGLKEGVVGF | LINYGLKEG |
| | | LEKEIDNLSSKIINK | IDNLSSKII |
| | | EKEIDNLSSKIINKE | IDNLSSKII |
| | | KEIDNLSSKIINKEI | IDNLSSKII |
| | | FELEKEIDNLSSKII | LEKEIDNLS |
| | | IINKEIIVPSNKESY | IINKEIIVP |
| | | ALNIFTSNHLKTNTF | LNIFTSNHL |
| | | LNIFTSNHLKTNTFE | LNIFTSNHL |
| | | VGFVRNPKMISFELE | FVRNPKMIS |

|  |  |  |  |
|---|---|---|---|
|  |  | IDNLSSKIINKEIIV | IDNLSSKII |
|  |  | DIKISTQYIGSFADL | IKISTQYIG |
|  |  | KLINYGLKEGVVGFV | YGLKEGVVG |
|  |  | EIDNLSSKIINKEII | IDNLSSKII |
|  |  | FEGGKLINYGLKEGV | LINYGLKEG |
|  |  | DAGSDLIWLIGYRFS | LIWLIGYRF |
|  |  | AGSDLIWLIGYRFSD | LIWLIGYRF |
|  |  | SDLIWLIGYRFSDVA | LIWLIGYRF |
|  |  | LINYGLKEGVVGFVR | YGLKEGVVG |
|  |  | IFTSNHLKTNTFEGG | SNHLKTNTF |
|  |  | GSDLIWLIGYRFSDV | LIWLIGYRF |
|  |  | LLLILLESIVFLSCS | LILLESIVF |
|  |  | EGGKLINYGLKEGVV | LINYGLKEG |
|  |  | TFEGGKLINYGLKEG | GGKLINYGL |
|  |  | AKYANKDIKISTQYI | YANKDIKIS |
|  |  | IKISTQYIGSFADLE | IKISTQYIG |
|  |  | GSLGSEIPKVSLIID | LGSEIPKVS |
|  |  | AGAKYANKDIKISTQ | YANKDIKIS |
|  |  | GAKYANKDIKISTQY | YANKDIKIS |
|  |  | EAGAKYANKDIKIST | YANKDIKIS |
|  |  | PDMKYAIIDPIYSND | MKYAIIDPI |
|  |  | QNPDMKYAIIDPIYS | MKYAIIDPI |
|  |  | NPDMKYAIIDPIYSN | MKYAIIDPI |
|  |  | KDAGSDLIWLIGYRF | AGSDLIWLI |
|  |  | FTSNHLKTNTFEGGK | SNHLKTNTF |
|  | HLA-DRB1*1501 | NKILLLILLESIVFL | ILLLILLES |
|  |  | MNKILLLILLESIVF | ILLLILLES |
|  |  | KILLLILLESIVFLS | LILLESIVF |
|  |  | GSDLIWLIGYRFSDV | DLIWLIGYR |
|  |  | SDLIWLIGYRFSDVA | LIGYRFSDV |
|  |  | DLIWLIGYRFSDVAK | LIGYRFSDV |
|  |  | ILLLILLESIVFLSC | LILLESIVF |
|  |  | LIWLIGYRFSDVAKV | LIGYRFSDV |
|  |  | IWLIGYRFSDVAKVA | LIGYRFSDV |
|  |  | EGVVGFVRNPKMISF | VVGFVRNPK |
|  |  | AGSDLIWLIGYRFSD | DLIWLIGYR |
|  |  | KEGVVGFVRNPKMIS | VVGFVRNPK |
|  |  | LLLILLESIVFLSCS | LILLESIVF |
|  |  | LKEGVVGFVRNPKMI | VVGFVRNPK |
|  |  | DVGRALNIFTSNHLK | LNIFTSNHL |
|  |  | KDVGRALNIFTSNHL | GRALNIFTS |
|  |  | LILLESIVFLSCSGK | LILLESIVF |
|  |  | GLKEGVVGFVRNPKM | VVGFVRNPK |
|  |  | DAGSDLIWLIGYRFS | DLIWLIGYR |
|  |  | AFLTGYIAAKLSKTG | FLTGYIAAK |
|  |  | FLTGYIAAKLSKTGK | IAAKLSKTG |
|  | HLA-DRB3*0101 | None |  |
|  | HLA-DRB4*0101 | SDLIWLIGYRFSDVA | LIWLIGYRF |
|  |  | GSDLIWLIGYRFSDV | LIWLIGYRF |
|  |  | DAGSDLIWLIGYRFS | LIWLIGYRF |
|  |  | AGSDLIWLIGYRFSD | LIWLIGYRF |
|  |  | DLIWLIGYRFSDVAK | LIWLIGYRF |
|  | HLA-DRB5*0101 | None |  |
|  |  |  |  |
| NP_212516.1<br>Basic membrane protein B | HLA-DRB1*0101 | WLVGYMLTDASLLVS | MLTDASLLV |
|  |  | IWLVGYMLTDASLLV | YMLTDASLL |

| | | | |
|---|---|---|---|
| (bmpB) [Borrelia burgdorferi B31<br><br>Seq2 | | LVGYMLTDASLLVSS | MLTDASLLV |
| | | VGYMLTDASLLVSSE | MLTDASLLV |
| | | SDLIWLVGYMLTDAS | LIWLVGYML |
| | | GYMLTDASLLVSSEN | MLTDASLLV |
| | | GSDLIWLVGYMLTDA | LIWLVGYML |
| | | LIWLVGYMLTDASLL | LIWLVGYML |
| | | DLIWLVGYMLTDASL | LIWLVGYML |
| | | NGSDLIWLVGYMLTD | LIWLVGYML |
| | | IDVIHFAAGLAGIGV | FAAGLAGIG |
| | | DVIHFAAGLAGIGVI | FAAGLAGIG |
| | | IHFAAGLAGIGVIET | FAAGLAGIG |
| | | RNGSDLIWLVGYMLT | LIWLVGYML |
| | | YMLTDASLLVSSENP | LTDASLLVS |
| | | GIDVIHFAAGLAGIG | IHFAAGLAG |
| | | VIHFAAGLAGIGVIE | FAAGLAGIG |
| | | WEGGKVVQMGLRDGV | VVQMGLRDG |
| | | GGKVVQMGLRDGVIG | VVQMGLRDG |
| | | GKVVQMGLRDGVIGL | VVQMGLRDG |
| | | FIFGILLTSCFSRNG | FGILLTSCF |
| | | HFAAGLAGIGVIETA | LAGIGVIET |
| | | IVIFIFGILLTSCFS | FGILLTSCF |
| | | KRNGSDLIWLVGYML | GSDLIWLVG |
| | | RIVIFIFGILLTSCF | IFGILLTSC |
| | | VWEGGKVVQMGLRDG | WEGGKVVQM |
| | | IFIFGILLTSCFSRN | FGILLTSCF |
| | | EGGKVVQMGLRDGVI | VVQMGLRDG |
| | | VIFIFGILLTSCFSR | FGILLTSCF |
| | | FAAGLAGIGVIETAK | LAGIGVIET |
| | | MLTDASLLVSSENPK | MLTDASLLV |
| | | AAGLAGIGVIETAKN | LAGIGVIET |
| | | IGFIGGMKGNIVDAF | IGGMKGNIV |
| | | GFIGGMKGNIVDAFR | IGGMKGNIV |
| | | AGLAGIGVIETAKNL | LAGIGVIET |
| | | IFGILLTSCFSRNGI | FGILLTSCF |
| | | SGKIGFIGGMKGNIV | IGFIGGMKG |
| | | GKIGFIGGMKGNIVD | IGGMKGNIV |
| | | KIGFIGGMKGNIVDA | IGGMKGNIV |
| | | SDVDIGRTIASKMYS | VDIGRTIAS |
| | | KGIDVIHFAAGLAGI | IHFAAGLAG |
| | | FSDVDIGRTIASKMY | VDIGRTIAS |
| | | AFLAGYIAAKKSFSG | AGYIAAKKS |
| | | KVVQMGLRDGVIGLP | VVQMGLRDG |
| | | FLAGYIAAKKSFSGK | IAAKKSFSG |
| | | DKSFNSSANEALLRL | FNSSANEAL |
| | | FGILLTSCFSRNGIE | FGILLTSCF |
| | | KSFNSSANEALLRLK | FNSSANEAL |
| | | VVFRVEQGAFLAGYI | FRVEQGAFL |
| | | AVVFRVEQGAFLAGY | FRVEQGAFL |
| | | VVQMGLRDGVIGLPN | VVQMGLRDG |
| | | AGYIAAKKSFSGKIG | IAAKKSFSG |
| | | SKGIDVIHFAAGLAG | VIHFAAGLA |
| | | LAGYIAAKKSFSGKI | IAAKKSFSG |
| | | VDIGRTIASKMYSKG | IGRTIASKM |
| | | VFSCAISGVYSSYVS | ISGVYSSYV |
| | | SCAISGVYSSYVSDL | ISGVYSSYV |
| | | EVFSCAISGVYSSYV | SCAISGVYS |
| | | NNNVWEGGKVVQMGL | WEGGKVVQM |

| | | | |
|---|---|---|---|
| | | FSCAISGVYSSYVSD | ISGVYSSYV |
| | | CAISGVYSSYVSDLD | ISGVYSSYV |
| | | NNVWEGGKVVQMGLR | WEGGKVVQM |
| | | NVWEGGKVVQMGLRD | WEGGKVVQM |
| | | KNNNVWEGGKVVQMG | WEGGKVVQM |
| | | IKNNNVWEGGKVVQM | NVWEGGKVV |
| | | MRIVIFIFGILLTSC | FIFGILLTS |
| | | LDDKSFNSSANEALL | FNSSANEAL |
| | | DDKSFNSSANEALLR | FNSSANEAL |
| | | IAVVFRVEQGAFLAG | FRVEQGAFL |
| | | DVDIGRTIASKMYSK | IGRTIASKM |
| | | VLDDKSFNSSANEAL | SFNSSANEA |
| | | LTDASLLVSSENPKI | LTDASLLVS |
| | | GYIAAKKSFSGKIGF | IAAKKSFSG |
| | | SSKKIKISMLVDGVL | IKISMLVDG |
| | | SFSDVDIGRTIASKM | VDIGRTIAS |
| | | FRVEQGAFLAGYIAA | VEQGAFLAG |
| | | VFRVEQGAFLAGYIA | VEQGAFLAG |
| | | SSSKKIKISMLVDGV | IKISMLVDG |
| | | ESSSKKIKISMLVDG | KKIKISMLV |
| | | GAFLAGYIAAKKSFS | LAGYIAAKK |
| | | FIGGMKGNIVDAFRY | IGGMKGNIV |
| | | IGGMKGNIVDAFRYG | IGGMKGNIV |
| | | GLAGIGVIETAKNLG | LAGIGVIET |
| | | LAGIGVIETAKNLGD | LAGIGVIET |
| | | KISYGIIDPIYGDDV | YGIIDPIYG |
| | | ENPKISYGIIDPIYG | ENPKISYGI |
| | | ISYGIIDPIYGDDVQ | YGIIDPIYG |
| | | SKKIKISMLVDGVLD | IKISMLVDG |
| | | SVIKNIGDALYLITG | IKNIGDALY |
| | | KKIKISMLVDGVLDD | IKISMLVDG |
| | | EQGAFLAGYIAAKKS | LAGYIAAKK |
| | | PKISYGIIDPIYGDD | YGIIDPIYG |
| | | TSVIKNIGDALYLIT | IKNIGDALY |
| | | SFNSSANEALLRLKK | FNSSANEAL |
| | | FNSSANEALLRLKKD | FNSSANEAL |
| | | NPKISYGIIDPIYGD | YGIIDPIYG |
| | | NSFSDVDIGRTIASK | VDIGRTIAS |
| | | DIGRTIASKMYSKGI | GRTIASKMY |
| | | QGAFLAGYIAAKKSF | LAGYIAAKK |
| | | SNSFSDVDIGRTIAS | FSDVDIGRT |
| | | GILLTSCFSRNGIES | LTSCFSRNG |
| | | VEQGAFLAGYIAAKK | FLAGYIAAK |
| | | ILLTSCFSRNGIESS | LTSCFSRNG |
| | | IGRTIASKMYSKGID | IGRTIASKM |
| | | LIAVVFRVEQGAFLA | FRVEQGAFL |
| | | VIKNIGDALYLITGE | IGDALYLIT |
| | | DDVQIPENLIAVVFR | VQIPENLIA |
| | | RVEQGAFLAGYIAAK | VEQGAFLAG |
| | | GDDVQIPENLIAVVF | VQIPENLIA |
| | HLA-DRB1*0301 | None | |
| | HLA-DRB1*0401 | ANKDIEIISEYSNSF | ANKDIEIIS |
| | | NKDIEIISEYSNSFS | IISEYSNSF |
| | | KDIEIISEYSNSFSD | IISEYSNSF |
| | | DIEIISEYSNSFSDV | IISEYSNSF |
| | | IEIISEYSNSFSDVD | IISEYSNSF |
| | | EIISEYSNSFSDVDI | IISEYSNSF |

|  |  | IISEYSNSFSDVDIG | IISEYSNSF |
|---|---|---|---|
|  |  | WLVGYMLTDASLLVS | MLTDASLLV |
|  |  | IWLVGYMLTDASLLV | VGYMLTDAS |
|  |  | VGYMLTDASLLVSSE | MLTDASLLV |
|  |  | LVGYMLTDASLLVSS | MLTDASLLV |
|  |  | GYMLTDASLLVSSEN | MLTDASLLV |
|  | HLA-DRB1*0404 | LAGIGVIETAKNLGD | VIETAKNLG |
|  |  | GLAGIGVIETAKNLG | IGVIETAKN |
|  |  | AGIGVIETAKNLGDG | VIETAKNLG |
|  |  | IGVIETAKNLGDGYY | VIETAKNLG |
|  |  | LIWLVGYMLTDASLL | VGYMLTDAS |
|  |  | IWLVGYMLTDASLLV | YMLTDASLL |
|  |  | GIGVIETAKNLGDGY | VIETAKNLG |
|  |  | WLVGYMLTDASLLVS | YMLTDASLL |
|  |  | LVGYMLTDASLLVSS | YMLTDASLL |
|  |  | VGYMLTDASLLVSSE | YMLTDASLL |
|  |  | SDLIWLVGYMLTDAS | WLVGYMLTD |
|  |  | DLIWLVGYMLTDASL | VGYMLTDAS |
|  | HLA-DRB1*0405 | GVYSSYVSDLDNLKR | YSSYVSDLD |
|  |  | SGVYSSYVSDLDNLK | YSSYVSDLD |
|  |  | CAISGVYSSYVSDLD | ISGVYSSYV |
|  |  | AISGVYSSYVSDLDN | YSSYVSDLD |
|  |  | ISGVYSSYVSDLDNL | YSSYVSDLD |
|  |  | VYSSYVSDLDNLKRN | YSSYVSDLD |
|  |  | YSSYVSDLDNLKRNG | YSSYVSDLD |
|  |  | LAGIGVIETAKNLGD | IGVIETAKN |
|  |  | AGIGVIETAKNLGDG | IETAKNLGD |
|  |  | MGLRDGVIGLPNANE | RDGVIGLPN |
|  |  | EIISEYSNSFSDVDI | YSNSFSDVD |
|  |  | GIGVIETAKNLGDGY | IETAKNLGD |
|  |  | IGVIETAKNLGDGYY | IETAKNLGD |
|  |  | GLRDGVIGLPNANEF | VIGLPNANE |
|  |  | IEIISEYSNSFSDVD | ISEYSNSFS |
|  |  | LRDGVIGLPNANEFE | VIGLPNANE |
|  |  | RDGVIGLPNANEFEY | VIGLPNANE |
|  | HLA-DRB1*0701 | EVFSCAISGVYSSYV | FSCAISGVY |
|  |  | EEVFSCAISGVYSSY | FSCAISGVY |
|  |  | ENIEEVFSCAISGVY | VFSCAISGV |
|  |  | NIEEVFSCAISGVYS | FSCAISGVY |
|  |  | IEEVFSCAISGVYSS | FSCAISGVY |
|  |  | VFSCAISGVYSSYVS | FSCAISGVY |
|  |  | FSCAISGVYSSYVSD | FSCAISGVY |
|  |  | VGYMLTDASLLVSSE | YMLTDASLL |
|  |  | WLVGYMLTDASLLVS | YMLTDASLL |
|  |  | IWLVGYMLTDASLLV | YMLTDASLL |
|  |  | LVGYMLTDASLLVSS | YMLTDASLL |
|  | HLA-DRB1*0802 | None |  |
|  | HLA-DRB1*0901 | LDDKSFNSSANEALL | FNSSANEAL |
|  |  | DKSFNSSANEALLRL | FNSSANEAL |
|  |  | DDKSFNSSANEALLR | FNSSANEAL |
|  |  | VLDDKSFNSSANEAL | SFNSSANEA |
|  |  | KSFNSSANEALLRLK | FNSSANEAL |
|  |  | GIDVIHFAAGLAGIG | VIHFAAGLA |
|  |  | IDVIHFAAGLAGIGV | VIHFAAGLA |
|  |  | YLAPKNFITSVIKNI | YLAPKNFIT |

| | HLA-DRB1*1101 | None | |
|---|---|---|---|
| | HLA-DRB1*1302 | VSDLDNLKRNGSDLI | LDNLKRNGS |
| | | SDLDNLKRNGSDLIW | LKRNGSDLI |
| | | RIVIFIFGILLTSCF | IFIFGILLT |
| | | NFITSVIKNIGDALY | ITSVIKNIG |
| | | IVIFIFGILLTSCFS | FGILLTSCF |
| | | LAPKNFITSVIKNIG | PKNFITSVI |
| | | LDNLKRNGSDLIWLV | LKRNGSDLI |
| | | KNFITSVIKNIGDAL | ITSVIKNIG |
| | | LITGEYIKNNNVWEG | YIKNNNVWE |
| | | DLDNLKRNGSDLIWL | LKRNGSDLI |
| | | ITGEYIKNNNVWEGG | IKNNNVWEG |
| | | GEYIKNNNVWEGGKV | IKNNNVWEG |
| | | TGEYIKNNNVWEGGK | IKNNNVWEG |
| | | VIFIFGILLTSCFSR | FGILLTSCF |
| | | IFIFGILLTSCFSRN | FGILLTSCF |
| | | EYIKNNNVWEGGKVV | IKNNNVWEG |
| | | FITSVIKNIGDALYL | ITSVIKNIG |
| | | ITSVIKNIGDALYLI | ITSVIKNIG |
| | | APKNFITSVIKNIGD | ITSVIKNIG |
| | | PKNFITSVIKNIGDA | ITSVIKNIG |
| | | SSKKIKISMLVDGVL | IKISMLVDG |
| | | FIFGILLTSCFSRNG | FGILLTSCF |
| | | MRIVIFIFGILLTSC | IFIFGILLT |
| | | DNLKRNGSDLIWLVG | LKRNGSDLI |
| | | SKKIKISMLVDGVLD | IKISMLVDG |
| | | VLERKIINKEIIVPC | IINKEIIVP |
| | | LERKIINKEIIVPCN | IINKEIIVP |
| | | KVLERKIINKEIIVP | LERKIINKE |
| | | KKIKISMLVDGVLDD | IKISMLVDG |
| | | SSSKKIKISMLVDGV | IKISMLVDG |
| | | TSVIKNIGDALYLIT | IKNIGDALY |
| | | ESSSKKIKISMLVDG | KKIKISMLV |
| | | FEYIKVLERKIINKE | IKVLERKII |
| | | EYIKVLERKIINKEI | IKVLERKII |
| | | SDLIWLVGYMLTDAS | LIWLVGYML |
| | | YIKNNNVWEGGKVVQ | IKNNNVWEG |
| | | IKNNNVWEGGKVVQM | IKNNNVWEG |
| | | ERKIINKEIIVPCNQ | IINKEIIVP |
| | | IKVLERKIINKEIIV | IKVLERKII |
| | | SSYVSDLDNLKRNGS | YVSDLDNLK |
| | | RKIINKEIIVPCNQE | IINKEIIVP |
| | | YVSDLDNLKRNGSDL | LDNLKRNGS |
| | | GKVVQMGLRDGVIGL | MGLRDGVIG |
| | | SVIKNIGDALYLITG | IKNIGDALY |
| | | YIKVLERKIINKEII | IKVLERKII |
| | | GGKVVQMGLRDGVIG | VQMGLRDGV |
| | | SYVSDLDNLKRNGSD | LDNLKRNGS |
| | | DLIWLVGYMLTDASL | VGYMLTDAS |
| | | KVVQMGLRDGVIGLP | MGLRDGVIG |
| | | IGFIGGMKGNIVDAF | IGGMKGNIV |
| | | SGKIGFIGGMKGNIV | IGFIGGMKG |
| | | VDIGRTIASKMYSKG | IGRTIASKM |
| | | GKIGFIGGMKGNIVD | IGGMKGNIV |
| | | IFGILLTSCFSRNGI | FGILLTSCF |
| | | LIWLVGYMLTDASLL | VGYMLTDAS |
| | | NGSDLIWLVGYMLTD | LIWLVGYML |

| | | RNGSDLIWLVGYMLT | LIWLVGYML |
|---|---|---|---|
| | | WLVGYMLTDASLLVS | VGYMLTDAS |
| | | FGILLTSCFSRNGIE | FGILLTSCF |
| | | GSDLIWLVGYMLTDA | LIWLVGYML |
| | | ANEFEYIKVLERKII | FEYIKVLER |
| | | NEFEYIKVLERKIIN | IKVLERKII |
| | | EFEYIKVLERKIINK | IKVLERKII |
| | | IWLVGYMLTDASLLV | VGYMLTDAS |
| | | VVQMGLRDGVIGLPN | MGLRDGVIG |
| | | KIKISMLVDGVLDDK | IKISMLVDG |
| | | NLKRNGSDLIWLVGY | LKRNGSDLI |
| | | IKISMLVDGVLDDKS | IKISMLVDG |
| | | LKRNGSDLIWLVGYM | LKRNGSDLI |
| | | YLAPKNFITSVIKNI | PKNFITSVI |
| | | GFIGGMKGNIVDAFR | IGGMKGNIV |
| | | ENPKISYGIIDPIYG | ISYGIIDPI |
| | | VIKNIGDALYLITGE | IKNIGDALY |
| | | SDVDIGRTIASKMYS | IGRTIASKM |
| | | VQMGLRDGVIGLPNA | MGLRDGVIG |
| | | KIGFIGGMKGNIVDA | IGGMKGNIV |
| | | NPKISYGIIDPIYGD | YGIIDPIYG |
| | | PKISYGIIDPIYGDD | YGIIDPIYG |
| | | DVDIGRTIASKMYSK | IGRTIASKM |
| | | SYLAPKNFITSVIKN | PKNFITSV |
| | | FSDVDIGRTIASKMY | IGRTIASKM |
| | | QSYLAPKNFITSVIK | PKNFITSVI |
| | | SFSDVDIGRTIASKM | VDIGRTIAS |
| | | NQEEYEIFIKQILKL | YEIFIKQIL |
| | | LVGYMLTDASLLVSS | VGYMLTDAS |
| | | IGRTIASKMYSKGID | IGRTIASKM |
| | | VGYMLTDASLLVSSE | VGYMLTDAS |
| | | DQSYLAPKNFITSVI | LAPKNFITS |
| | | KIINKEIIVPCNQEE | IINKEIIVP |
| | | FIGGMKGNIVDAFRY | IGGMKGNIV |
| | HLA-DRB1*1501 | MRIVIFIFGILLTSC | FIFGILLTS |
| | | VIFIFGILLTSCFSR | FIFGILLTS |
| | | RIVIFIFGILLTSCF | FIFGILLTS |
| | | IVIFIFGILLTSCFS | FIFGILLTS |
| | | AFLAGYIAAKKSFSG | FLAGYIAAK |
| | | IFIFGILLTSCFSRN | ILLTSCFSR |
| | | FLAGYIAAKKSFSGK | IAAKKSFSG |
| | | SDLIWLVGYMLTDAS | LVGYMLTDA |
| | | DLIWLVGYMLTDASL | LVGYMLTDA |
| | | LIWLVGYMLTDASLL | LVGYMLTDA |
| | | GKVVQMGLRDGVIGL | VVQMGLRDG |
| | | GSDLIWLVGYMLTDA | LIWLVGYML |
| | | GGKVVQMGLRDGVIG | VVQMGLRDG |
| | | IWLVGYMLTDASLLV | LVGYMLTDA |
| | | WEGGKVVQMGLRDGV | VVQMGLRDG |
| | | VWEGGKVVQMGLRDG | GKVVQMGLR |
| | HLA-DRB3*0101 | None | |
| | HLA-DRB4*0101 | SDLIWLVGYMLTDAS | LIWLVGYML |
| | | GSDLIWLVGYMLTDA | LIWLVGYML |
| | | NGSDLIWLVGYMLTD | LIWLVGYML |
| | | RNGSDLIWLVGYMLT | LIWLVGYML |
| | | DLIWLVGYMLTDASL | IWLVGYMLT |

|  | HLA-DRB5*0101 | None | |
|---|---|---|---|
|  |  |  | |
| NP_212518.1 Basic membrane protein C (bmpC) [Borrelia burgdorferi B31 Seq3 | HLA-DRB1*0101 | AGIFYANPKLRLVSK | YANPKLRLV |
|  |  | GIFYANPKLRLVSKK | YANPKLRLV |
|  |  | FKAGIFYANPKLRLV | FYANPKLRL |
|  |  | KAGIFYANPKLRLVS | YANPKLRLV |
|  |  | LAIKFRNEEAAFLAG | FRNEEAAFL |
|  |  | AIKFRNEEAAFLAGY | FRNEEAAFL |
|  |  | IFYANPKLRLVSKKA | YANPKLRLV |
|  |  | SLAIKFRNEEAAFLA | FRNEEAAFL |
|  |  | IKFRNEEAAFLAGYI | FRNEEAAFL |
|  |  | NSLAIKFRNEEAAFL | NSLAIKFRN |
|  |  | FPIAGITGLGVYDAA | ITGLGVYDA |
|  |  | IFPIAGITGLGVYDA | IAGITGLGV |
|  |  | EKIGFLTGPMSEHVK | LTGPMSEHV |
|  |  | KEKIGFLTGPMSEHV | FLTGPMSEH |
|  |  | KIGFLTGPMSEHVKD | LTGPMSEHV |
|  |  | IGFLTGPMSEHVKDF | LTGPMSEHV |
|  |  | IKSDKVVVGVLAHGS | VVVGVLAHG |
|  |  | IAGITGLGVYDAAKE | ITGLGVYDA |
|  |  | KSDKVVVGVLAHGSF | VVGVLAHGS |
|  |  | DKVVVGVLAHGSFYD | VVGVLAHGS |
|  |  | PIAGITGLGVYDAAK | ITGLGVYDA |
|  |  | SDKVVVGVLAHGSFY | VVGVLAHGS |
|  |  | GFLTGPMSEHVKDFK | LTGPMSEHV |
|  |  | KVVVGVLAHGSFYDK | VVGVLAHGS |
|  |  | FKRFIFITLSLLVFA | IFITLSLLV |
|  |  | AGITGLGVYDAAKEL | ITGLGVYDA |
|  |  | KRFIFITLSLLVFAC | IFITLSLLV |
|  |  | RFIFITLSLLVFACF | IFITLSLLV |
|  |  | MFKRFIFITLSLLVF | IFITLSLLV |
|  |  | FYANPKLRLVSKKAP | YANPKLRLV |
|  |  | NPKLRLVSKKAPSLF | LVSKKAPSL |
|  |  | ANPKLRLVSKKAPSL | KLRLVSKKA |
|  |  | PKLRLVSKKAPSLFD | LVSKKAPSL |
|  |  | AAFLAGYIAAKMSRK | LAGYIAAKM |
|  |  | KLRLVSKKAPSLFDK | LVSKKAPSL |
|  |  | AFLAGYIAAKMSRKE | LAGYIAAKM |
|  |  | FIFITLSLLVFACFK | FITLSLLVF |
|  |  | EAAFLAGYIAAKMSR | LAGYIAAKM |
|  |  | EEAAFLAGYIAAKMS | LAGYIAAKM |
|  |  | YANPKLRLVSKKAPS | YANPKLRLV |
|  |  | LRLVSKKAPSLFDKE | LVSKKAPSL |
|  |  | KFRNEEAAFLAGYIA | FRNEEAAFL |
|  |  | FRNEEAAFLAGYIAA | FRNEEAAFL |
|  |  | VGVIFPIAGITGLGV | FPIAGITGL |
|  |  | GVIFPIAGITGLGVY | IAGITGLGV |
|  |  | KNPLNLFWLIGYRFS | LFWLIGYRF |
|  |  | NEEAAFLAGYIAAKM | FLAGYIAAK |
|  |  | PLNLFWLIGYRFSDL | LFWLIGYRF |
|  |  | NPLNLFWLIGYRFSD | LFWLIGYRF |
|  |  | LNLFWLIGYRFSDLS | LFWLIGYRF |
|  |  | VIFPIAGITGLGVYD | IAGITGLGV |
|  |  | QSYIAPQNVITSIIK | YIAPQNVIT |
|  |  | FLTGPMSEHVKDFKF | LTGPMSEHV |
|  |  | DQSYIAPQNVITSII | YIAPQNVIT |

| | | VVVGVLAHGSFYDKG | VVGVLAHGS |
|---|---|---|---|
| | | IFITLSLLVFACFKS | IFITLSLLV |
| | | NQDQSYIAPQNVITS | YIAPQNVIT |
| | | VVGVLAHGSFYDKGY | VVGVLAHGS |
| | | QDQSYIAPQNVITSI | YIAPQNVIT |
| | | GITGLGVYDAAKELG | ITGLGVYDA |
| | | FLAGYIAAKMSRKEK | LAGYIAAKM |
| | | ITGLGVYDAAKELGP | ITGLGVYDA |
| | | LNQDQSYIAPQNVIT | LNQDQSYIA |
| | | GYRFSDLSVKLSYER | FSDLSVKLS |
| | | GFKAGIFYANPKLRL | FKAGIFYAN |
| | | LTGPMSEHVKDFKFG | LTGPMSEHV |
| | | YRFSDLSVKLSYERP | FSDLSVKLS |
| | | WLIGYRFSDLSVKLS | RFSDLSVKL |
| | | DKVGVIFPIAGITGL | IFPIAGITG |
| | | IGYRFSDLSVKLSYE | FSDLSVKLS |
| | | LAGYIAAKMSRKEKI | LAGYIAAKM |
| | | LIGYRFSDLSVKLSY | FSDLSVKLS |
| | | GKVIYSISSEYINNR | IYSISSEYI |
| | | KVGVIFPIAGITGLG | FPIAGITGL |
| | | DIGKVIYSISSEYIN | IYSISSEYI |
| | | IGKVIYSISSEYINN | IYSISSEYI |
| | | KDIGKVIYSISSEYI | VIYSISSEY |
| | | KVIYSISSEYINNRV | IYSISSEYI |
| | | LRPYPIEGKRLLTVD | IEGKRLLTV |
| | | SLRPYPIEGKRLLTV | YPIEGKRLL |
| | | DNFGIKLITKSLRPY | FGIKLITKS |
| | | FGIKLITKSLRPYPI | LITKSLRPY |
| | | RLVSKKAPSLFDKEK | LVSKKAPSL |
| | | RPYPIEGKRLLTVDE | IEGKRLLTV |
| | | EDKVGVIFPIAGITG | VGVIFPIAG |
| | | QKNPLNLFWLIGYRF | PLNLFWLIG |
| | | SYIAPQNVITSIIKD | YIAPQNVIT |
| | | NLFWLIGYRFSDLSV | LFWLIGYRF |
| | | PYPIEGKRLLTVDEA | IEGKRLLTV |
| | | KYYVIGLNQDQSYIA | YVIGLNQDQ |
| | | FGFKAGIFYANPKLR | FKAGIFYAN |
| | | YPIEGKRLLTVDEAM | IEGKRLLTV |
| | | LVSKKAPSLFDKEKG | LVSKKAPSL |
| | | RDNFGIKLITKSLRP | FGIKLITKS |
| | | LRDNFGIKLITKSLR | FGIKLITKS |
| | | IKLITKSLRPYPIEG | TKSLRPYPI |
| | | KLITKSLRPYPIEGK | TKSLRPYPI |
| | | KDFKFGFKAGIFYAN | GFKAGIFYA |
| | | GIKLITKSLRPYPIE | TKSLRPYPI |
| | | YYVIGLNQDQSYIAP | LNQDQSYIA |
| | | AGYIAAKMSRKEKIG | IAAKMSRKE |
| | | FKFGFKAGIFYANPK | FKAGIFYAN |
| | | LITKSLRPYPIEGKR | TKSLRPYPI |
| | | DFKFGFKAGIFYANP | FKAGIFYAN |
| | | KLRDNFGIKLITKSL | FGIKLITKS |
| | | KFGFKAGIFYANPKL | FKAGIFYAN |
| | | YVIGLNQDQSYIAPQ | LNQDQSYIA |
| | | LFWLIGYRFSDLSVK | LFWLIGYRF |
| | | IGLNQDQSYIAPQNV | LNQDQSYIA |
| | | FITLSLLVFACFKSN | FITLSLLVF |
| | | YIAPQNVITSIIKDI | YIAPQNVIT |

| | | | |
|---|---|---|---|
| | | RFSDLSVKLSYERPD | FSDLSVKLS |
| | | FDYGDIQVPKNSLAI | IQVPKNSLA |
| | | VKLRDNFGIKLITKS | LRDNFGIKL |
| | | VIGLNQDQSYIAPQN | LNQDQSYIA |
| | | FSDLSVKLSYERPDI | FSDLSVKLS |
| | | NFGIKLITKSLRPYP | LITKSLRPY |
| | | RKEKIGFLTGPMSEH | IGFLTGPMS |
| | | SNKKSIKSDKVVVGV | IKSDKVVVG |
| | | KKSIKSDKVVVGVLA | IKSDKVVVG |
| | | KSNKKSIKSDKVVVG | KKSIKSDKV |
| | | YINNRVFKGGIIIDR | YINNRVFKG |
| | | KSIKSDKVVVGVLAH | IKSDKVVVG |
| | | NRVFKGGIIIDRGLK | FKGGIIIDR |
| | | SEYINNRVFKGGIII | YINNRVFKG |
| | | RVFKGGIIIDRGLKE | FKGGIIIDR |
| | | SSEYINNRVFKGGII | YINNRVFKG |
| | | ISSEYINNRVFKGGI | YINNRVFKG |
| | | NNRVFKGGIIIDRGL | FKGGIIIDR |
| | | NKKSIKSDKVVVGVL | IKSDKVVVG |
| | | SISSEYINNRVFKGG | YINNRVFKG |
| | | INNRVFKGGIIIDRG | FKGGIIIDR |
| | | YGDIQVPKNSLAIKF | IQVPKNSLA |
| | | DAYEVQKNPLNLFWL | VQKNPLNLF |
| | | SIKSDKVVVGVLAHG | IKSDKVVVG |
| | | EDAYEVQKNPLNLFW | VQKNPLNLF |
| | | DYGDIQVPKNSLAIK | IQVPKNSLA |
| | | TEDAYEVQKNPLNLF | YEVQKNPLN |
| | | GDIQVPKNSLAIKFR | IQVPKNSLA |
| | | GSFYDKGYNQSVHDG | YDKGYNQSV |
| | | GYIAAKMSRKEKIGF | IAAKMSRKE |
| | | AYEVQKNPLNLFWLI | VQKNPLNLF |
| | | IEGKRLLTVDEAMTE | IEGKRLLTV |
| | | AFDYGDIQVPKNSLA | YGDIQVPKN |
| | | KEDKVGVIFPIAGIT | VGVIFPIAG |
| | | TKSLRPYPIEGKRLL | TKSLRPYPI |
| | | YEVQKNPLNLFWLIG | VQKNPLNLF |
| | | PIEGKRLLTVDEAMT | IEGKRLLTV |
| | | VIYSISSEYINNRVF | IYSISSEYI |
| | HLA-DRB1*0301 | None | |
| | HLA-DRB1*0401 | FKRFIFITLSLLVFA | IFITLSLLV |
| | | KRFIFITLSLLVFAC | IFITLSLLV |
| | | MFKRFIFITLSLLVF | IFITLSLLV |
| | | RFIFITLSLLVFACF | IFITLSLLV |
| | | KDIGKVIYSISSEYI | GKVIYSISS |
| | | DIGKVIYSISSEYIN | GKVIYSISS |
| | | IKDIGKVIYSISSEY | GKVIYSISS |
| | | GKVIYSISSEYINNR | VIYSISSEY |
| | | FIFITLSLLVFACFK | IFITLSLLV |
| | | IFITLSLLVFACFKS | IFITLSLLV |
| | | IGKVIYSISSEYINN | VIYSISSEY |
| | HLA-DRB1*0404 | FKRFIFITLSLLVFA | IFITLSLLV |
| | | MFKRFIFITLSLLVF | IFITLSLLV |
| | HLA-DRB1*0405 | EGVIEIVKDPDVLNN | IVKDPDVLN |
| | | KEGVIEIVKDPDVLN | VIEIVKDPD |
| | | GVIEIVKDPDVLNNR | IVKDPDVLN |
| | | IEIVKDPDVLNNRLV | IVKDPDVLN |

| | | | |
|---|---|---|---|
| | | VIEIVKDPDVLNNRL | IVKDPDVLN |
| | | TEDAYEVQKNPLNLF | YEVQKNPLN |
| | | EDAYEVQKNPLNLFW | YEVQKNPLN |
| | | DAYEVQKNPLNLFWL | YEVQKNPLN |
| | | AMTEDAYEVQKNPLN | EDAYEVQKN |
| | | MTEDAYEVQKNPLNL | YEVQKNPLN |
| | | LRLVSKKAPSLFDKE | SKKAPSLFD |
| | | RLVSKKAPSLFDKEK | SKKAPSLFD |
| | | PKLRLVSKKAPSLFD | LRLVSKKAP |
| | | KLRLVSKKAPSLFDK | SKKAPSLFD |
| | | LVSKKAPSLFDKEKG | SKKAPSLFD |
| | | EIVKDPDVLNNRLVD | IVKDPDVLN |
| | | IVKDPDVLNNRLVDE | IVKDPDVLN |
| | | AYEVQKNPLNLFWLI | YEVQKNPLN |
| | | YEVQKNPLNLFWLIG | YEVQKNPLN |
| | HLA-DRB1*0701 | MFKRFIFITLSLLVF | IFITLSLLV |
| | | FKRFIFITLSLLVFA | IFITLSLLV |
| | | KRFIFITLSLLVFAC | IFITLSLLV |
| | | RFIFITLSLLVFACF | IFITLSLLV |
| | | PKLRLVSKKAPSLFD | VSKKAPSLF |
| | HLA-DRB1*0802 | FGIKLITKSLRPYPI | ITKSLRPYP |
| | | IKLITKSLRPYPIEG | ITKSLRPYP |
| | | GIKLITKSLRPYPIE | ITKSLRPYP |
| | | KLITKSLRPYPIEGK | ITKSLRPYP |
| | HLA-DRB1*0901 | None | |
| | HLA-DRB1*1101 | PLNLFWLIGYRFSDL | FWLIGYRFS |
| | | LNLFWLIGYRFSDLS | FWLIGYRFS |
| | | NPLNLFWLIGYRFSD | FWLIGYRFS |
| | | KNPLNLFWLIGYRFS | LFWLIGYRF |
| | | NLFWLIGYRFSDLSV | FWLIGYRFS |
| | | SSEYINNRVFKGGII | YINNRVFKG |
| | | ISSEYINNRVFKGGI | YINNRVFKG |
| | | SISSEYINNRVFKGG | YINNRVFKG |
| | | YSISSEYINNRVFKG | EYINNRVFK |
| | HLA-DRB1*1302 | DNFGIKLITKSLRPY | IKLITKSLR |
| | | LRDNFGIKLITKSLR | FGIKLITKS |
| | | RDNFGIKLITKSLRP | IKLITKSLR |
| | | NFGIKLITKSLRPYP | IKLITKSLR |
| | | FGIKLITKSLRPYPI | IKLITKSLR |
| | | GIKLITKSLRPYPIE | IKLITKSLR |
| | | IKLITKSLRPYPIEG | IKLITKSLR |
| | | TSIIKDIGKVIYSIS | IKDIGKVIY |
| | | SIIKDIGKVIYSISS | IKDIGKVIY |
| | | IKDIGKVIYSISSEY | IGKVIYSIS |
| | | NVITSIIKDIGKVIY | ITSIIKDIG |
| | | SSEYINNRVFKGGII | YINNRVFKG |
| | | IIKDIGKVIYSISSE | IGKVIYSIS |
| | | SEYINNRVFKGGIII | YINNRVFKG |
| | | ISSEYINNRVFKGGI | YINNRVFKG |
| | | VITSIIKDIGKVIYS | IKDIGKVIY |
| | | SISSEYINNRVFKGG | YINNRVFKG |
| | | ITSIIKDIGKVIYSI | IKDIGKVIY |
| | | DAYEVQKNPLNLFWL | VQKNPLNLF |
| | | AYEVQKNPLNLFWLI | VQKNPLNLF |
| | | YEVQKNPLNLFWLIG | VQKNPLNLF |
| | | TEDAYEVQKNPLNLF | YEVQKNPLN |

|  |  | EDAYEVQKNPLNLFW | VQKNPLNLF |
|---|---|---|---|
|  |  | FKRFIFITLSLLVFA | FITLSLLVF |
|  |  | IAPQNVITSIIKDIG | PQNVITSII |
|  |  | MFKRFIFITLSLLVF | FIFITLSLL |
|  |  | YSISSEYINNRVFKG | EYINNRVFK |
|  |  | KDIGKVIYSISSEYI | IGKVIYSIS |
|  |  | RFIFITLSLLVFACF | FITLSLLVF |
|  |  | KRFIFITLSLLVFAC | FITLSLLVF |
|  |  | LVFACFKSNKKSIKS | FKSNKKSIK |
|  |  | EVQKNPLNLFWLIGY | VQKNPLNLF |
|  |  | LLVFACFKSNKKSIK | FACFKSNKK |
|  |  | VFACFKSNKKSIKSD | FKSNKKSIK |
|  |  | FACFKSNKKSIKSDK | FKSNKKSIK |
|  |  | VQKNPLNLFWLIGYR | VQKNPLNLF |
|  |  | ACFKSNKKSIKSDKV | FKSNKKSIK |
|  |  | EYINNRVFKGGIIID | YINNRVFKG |
|  |  | VKLRDNFGIKLITKS | LRDNFGIKL |
|  |  | FIFITLSLLVFACFK | FITLSLLVF |
|  |  | KNPLNLFWLIGYRFS | LFWLIGYRF |
|  |  | YIAPQNVITSIIKDI | PQNVITSII |
|  |  | QSYIAPQNVITSIIK | PQNVITSII |
|  |  | KLRDNFGIKLITKSL | FGIKLITKS |
|  |  | NPLNLFWLIGYRFSD | LFWLIGYRF |
|  |  | FKAGIFYANPKLRLV | AGIFYANPK |
|  |  | SYIAPQNVITSIIKD | PQNVITSII |
|  |  | DQSYIAPQNVITSII | IAPQNVITS |
|  |  | QNVITSIIKDIGKVI | ITSIIKDIG |
|  |  | APQNVITSIIKDIGK | PQNVITSII |
|  |  | LNLFWLIGYRFSDLS | LFWLIGYRF |
|  |  | DIGKVIYSISSEYIN | IGKVIYSIS |
|  |  | NPKLRLVSKKAPSLF | LRLVSKKAP |
|  |  | PKLRLVSKKAPSLFD | LRLVSKKAP |
|  |  | PQNVITSIIKDIGKV | PQNVITSII |
|  |  | IGKVIYSISSEYINN | IGKVIYSIS |
|  |  | YINNRVFKGGIIIDR | YINNRVFKG |
|  |  | QKNPLNLFWLIGYRF | LNLFWLIGY |
|  |  | KAGIFYANPKLRLVS | YANPKLRLV |
|  |  | FYANPKLRLVSKKAP | YANPKLRLV |
|  |  | AGIFYANPKLRLVSK | YANPKLRLV |
|  |  | PLNLFWLIGYRFSDL | LFWLIGYRF |
|  |  | YANPKLRLVSKKAPS | LRLVSKKAP |
|  |  | IFITLSLLVFACFKS | FITLSLLVF |
|  |  | ANPKLRLVSKKAPSL | LRLVSKKAP |
|  |  | EVIDLENKIISGEII | LENKIISGE |
|  |  | GIFYANPKLRLVSKK | FYANPKLRL |
|  |  | GFKAGIFYANPKLRL | IFYANPKLR |
|  |  | DKVGVIFPIAGITGL | VGVIFPIAG |
|  |  | KLRLVSKKAPSLFDK | LRLVSKKAP |
|  |  | CFKSNKKSIKSDKVV | FKSNKKSIK |
|  | HLA-DRB1*1501 | KNPLNLFWLIGYRFS | NLFWLIGYR |
|  |  | QKNPLNLFWLIGYRF | NLFWLIGYR |
|  |  | VQKNPLNLFWLIGYR | LNLFWLIGY |
|  |  | LFWLIGYRFSDLSVK | GYRFSDLSV |
|  |  | NPLNLFWLIGYRFSD | NLFWLIGYR |
|  |  | FWLIGYRFSDLSVKL | YRFSDLSVK |
|  |  | PLNLFWLIGYRFSDL | NLFWLIGYR |
|  |  | WLIGYRFSDLSVKLS | YRFSDLSVK |

| | | | |
|---|---|---|---|
| | | LIGYRFSDLSVKLSY | YRFSDLSVK |
| | | DNFGIKLITKSLRPY | IKLITKSLR |
| | | MFKRFIFITLSLLVF | IFITLSLLV |
| | | NFGIKLITKSLRPYP | IKLITKSLR |
| | | FKRFIFITLSLLVFA | IFITLSLLV |
| | | GYRFSDLSVKLSYER | YRFSDLSVK |
| | | LNLFWLIGYRFSDLS | NLFWLIGYR |
| | | FGIKLITKSLRPYPI | IKLITKSLR |
| | | KRFIFITLSLLVFAC | IFITLSLLV |
| | | IGYRFSDLSVKLSYE | YRFSDLSVK |
| | | YRFSDLSVKLSYERP | LSVKLSYER |
| | | RDNFGIKLITKSLRP | IKLITKSLR |
| | | LRDNFGIKLITKSLR | NFGIKLITK |
| | | NLFWLIGYRFSDLSV | NLFWLIGYR |
| | | SLRPYPIEGKRLLTV | LRPYPIEGK |
| | | LRPYPIEGKRLLTVD | IEGKRLLTV |
| | | YINNRVFKGGIIIDR | NRVFKGGII |
| | | RFIFITLSLLVFACF | IFITLSLLV |
| | | IFITLSLLVFACFKS | IFITLSLLV |
| | | LSLLVFACFKSNKKS | LLVFACFKS |
| | | ANPKLRLVSKKAPSL | LRLVSKKAP |
| | | NPKLRLVSKKAPSLF | LVSKKAPSL |
| | | INNRVFKGGIIIDRG | FKGGIIIDR |
| | | IKLITKSLRPYPIEG | LITKSLRPY |
| | | TLSLLVFACFKSNKK | LLVFACFKS |
| | | PYPIEGKRLLTVDEA | IEGKRLLTV |
| | | RPYPIEGKRLLTVDE | IEGKRLLTV |
| | | GIKLITKSLRPYPIE | LITKSLRPY |
| | | YPIEGKRLLTVDEAM | IEGKRLLTV |
| | | LLVFACFKSNKKSIK | FACFKSNKK |
| | | SLLVFACFKSNKKSI | FACFKSNKK |
| | | PKLRLVSKKAPSLFD | LVSKKAPSL |
| | | KAGIFYANPKLRLVS | IFYANPKLR |
| | HLA-DRB3*0101 | None | |
| | HLA-DRB4*0101 | RFIFITLSLLVFACF | FITLSLLVF |
| | | FIFITLSLLVFACFK | LSLLVFACF |
| | | FITLSLLVFACFKSN | LSLLVFACF |
| | | IFITLSLLVFACFKS | LSLLVFACF |
| | | ITLSLLVFACFKSNK | LSLLVFACF |
| | | NPKLRLVSKKAPSLF | LRLVSKKAP |
| | | PKLRLVSKKAPSLFD | VSKKAPSLF |
| | | KLRLVSKKAPSLFDK | VSKKAPSLF |
| | | LRLVSKKAPSLFDKE | VSKKAPSLF |
| | | LSVKLSYERPDIYYG | LSYERPDIY |
| | | SDLSVKLSYERPDIY | LSVKLSYER |
| | | VKLSYERPDIYYGII | LSYERPDIY |
| | | SVKLSYERPDIYYGI | LSYERPDIY |
| | | DLSVKLSYERPDIYY | LSYERPDIY |
| | | RLVSKKAPSLFDKEK | VSKKAPSLF |
| | | TLSLLVFACFKSNKK | LSLLVFACF |
| | | LSLLVFACFKSNKKS | LSLLVFACF |
| | | TEDAYEVQKNPLNLF | YEVQKNPLN |
| | | EDAYEVQKNPLNLFW | VQKNPLNLF |
| | | DAYEVQKNPLNLFWL | VQKNPLNLF |
| | | AYEVQKNPLNLFWLI | VQKNPLNLF |
| | | FGIKLITKSLRPYPI | IKLITKSLR |

| | HLA-DRB5*0101 | AGYIAAKMSRKEKIG | YIAAKMSRK |
|---|---|---|---|
| | | FLAGYIAAKMSRKEK | YIAAKMSRK |
| | | LAGYIAAKMSRKEKI | YIAAKMSRK |
| | | AAFLAGYIAAKMSRK | GYIAAKMSR |
| | | AFLAGYIAAKMSRKE | YIAAKMSRK |
| | | KAGIFYANPKLRLVS | IFYANPKLR |
| | | FKAGIFYANPKLRLV | IFYANPKLR |
| | | AGIFYANPKLRLVSK | IFYANPKLR |
| | | FGFKAGIFYANPKLR | FKAGIFYAN |
| | | GFKAGIFYANPKLRL | IFYANPKLR |
| | | GYIAAKMSRKEKIGF | YIAAKMSRK |
| | | YIAAKMSRKEKIGFL | YIAAKMSRK |
| | | GIFYANPKLRLVSKK | IFYANPKLR |
| | | IFYANPKLRLVSKKA | IFYANPKLR |
| | | SLFDKEKGKAMALFM | FDKEKGKAM |
| | | KKAPSLFDKEKGKAM | KKAPSLFDK |
| | | PSLFDKEKGKAMALF | FDKEKGKAM |
| | | KAPSLFDKEKGKAMA | FDKEKGKAM |
| | | APSLFDKEKGKAMAL | FDKEKGKAM |
| | | | |
| NP_212519.1B<br>Basic membrane protein D (bmpD) [Borrelia burgdorferi B31<br><br>Seq4 | HLA-DRB1*0101 | AFLAGYFASKASKTG | YFASKASKT |
| | | FLAGYFASKASKTGK | FASKASKTG |
| | | LAGYFASKASKTGKI | FASKASKTG |
| | | AGYFASKASKTGKIG | FASKASKTG |
| | | GYFASKASKTGKIGF | FASKASKTG |
| | | FGDFGLGRSTASNMY | FGLGRSTAS |
| | | TFGDFGLGRSTASNM | FGLGRSTAS |
| | | GTFGDFGLGRSTASN | FGLGRSTAS |
| | | GDFGLGRSTASNMYR | FGLGRSTAS |
| | | VGTFGDFGLGRSTAS | DFGLGRSTA |
| | | DGVDIIFAAAGLSGI | IFAAAGLSG |
| | | GVDIIFAAAGLSGIG | IFAAAGLSG |
| | | VDIIFAAAGLSGIGV | IFAAAGLSG |
| | | YFASKASKTGKIGFV | FASKASKTG |
| | | VAFLAGYFASKASKT | LAGYFASKA |
| | | DIIFAAAGLSGIGVI | IFAAAGLSG |
| | | RDGVDIIFAAAGLSG | IIFAAAGLS |
| | | SNLIWGIGFRLSDIL | IWGIGFRLS |
| | | NSNLIWGIGFRLSDI | IWGIGFRLS |
| | | GNSNLIWGIGFRLSD | IWGIGFRLS |
| | | DGNSNLIWGIGFRLS | LIWGIGFRL |
| | | DFGLGRSTASNMYRD | FGLGRSTAS |
| | | IIFAAAGLSGIGVIE | FAAAGLSGI |
| | | FGLGRSTASNMYRDG | FGLGRSTAS |
| | | IYNKSLKIGQSIMNG | LKIGQSIMN |
| | | EIYNKSLKIGQSIMN | IYNKSLKIG |
| | | FASKASKTGKIGFVG | FASKASKTG |
| | | NLIWGIGFRLSDILF | IWGIGFRLS |
| | | NKSLKIGQSIMNGII | LKIGQSIMN |
| | | YNKSLKIGQSIMNGI | LKIGQSIMN |
| | | TGVLDGGKTMFLGLK | LDGGKTMFL |
| | | KSLKIGQSIMNGIIK | LKIGQSIMN |
| | | GVLDGGKTMFLGLKE | LDGGKTMFL |
| | | LETGVLDGGKTMFLG | LDGGKTMFL |
| | | EEVAFLAGYFASKAS | VAFLAGYFA |
| | | YLETGVLDGGKTMFL | TGVLDGGKT |
| | | SEEVAFLAGYFASKA | VAFLAGYFA |

| | | | |
|---|---|---|---|
| | | ETGVLDGGKTMFLGL | LDGGKTMF |
| | | IFAAAGLSGIGVIEA | IFAAAGLSG |
| | | LSDILFQRASENVSV | LFQRASENV |
| | | EVAFLAGYFASKASK | LAGYFASKA |
| | | YRDGVDIIFAAAGLS | VDIIFAAAG |
| | | SDILFQRASENVSVN | LFQRASENV |
| | | DKGFNESSSKAIRKL | FNESSSKAI |
| | | LIWGIGFRLSDILFQ | IWGIGFRLS |
| | | RLSDILFQRASENVS | LFQRASENV |
| | | DDKGFNESSSKAIRK | FNESSSKAI |
| | | FDDKGFNESSSKAIR | FNESSSKAI |
| | | DILFQRASENVSVNY | LFQRASENV |
| | | AFDDKGFNESSSKAI | GFNESSSKA |
| | | IGFVGGVRGKVLESF | VGGVRGKVL |
| | | KGFNESSSKAIRKLK | FNESSSKAI |
| | | GFVGGVRGKVLESFM | VGGVRGKVL |
| | | FRLSDILFQRASENV | LSDILFQRA |
| | | LKIGQSIMNGIIKVP | LKIGQSIMN |
| | | LLNISFRSEEVAFLA | FRSEEVAFL |
| | | NLLNISFRSEEVAFL | NISFRSEEV |
| | | QDQSYLAPNNVIVSA | LAPNNVIVS |
| | | LNISFRSEEVAFLAG | FRSEEVAFL |
| | | NISFRSEEVAFLAGY | FRSEEVAFL |
| | | DQDQSYLAPNNVIVS | YLAPNNVIv |
| | | AKYANSNIKVVSQYV | YANSNIKVV |
| | | ISFRSEEVAFLAGYF | FRSEEVAFL |
| | | FRSEEVAFLAGYFAS | VAFLAGYFA |
| | | FAAAGLSGIGVIEAA | FAAAGLSGI |
| | | DQSYLAPNNVIVSAV | LAPNNVIVS |
| | | SFRSEEVAFLAGYFA | FRSEEVAFL |
| | | QSYLAPNNVIVSAVK | LAPNNVIVS |
| | | YSEIYNKSLKIGQSI | IYNKSLKIG |
| | | NYSEIYNKSLKIGQS | IYNKSLKIG |
| | | SNYSEIYNKSLKIGQ | IYNKSLKIG |
| | | YEAGAKYANSNIKVV | AGAKYANSN |
| | | SLKIGQSIMNGIIKV | LKIGQSIMN |
| | | VSAVKKVDSLMYSLT | VKKVDSLMY |
| | | SAVKKVDSLMYSLTK | VKKVDSLMY |
| | | ILFQRASENVSVNYA | FQRASENVS |
| | | KSNYSEIYNKSLKIG | YSEIYNKSL |
| | | FVGGVRGKVLESFMY | VRGKVLESF |
| | | RSEEVAFLAGYFASK | VAFLAGYFA |
| | | AGAKYANSNIKVVSQ | YANSNIKVV |
| | | VGGVRGKVLESFMYG | VRGKVLESF |
| | | YLAPNNVIVSAVKKV | LAPNNVIVS |
| | | EAGAKYANSNIKVVS | YANSNIKVV |
| | | VLDGGKTMFLGLKED | LDGGKTMFL |
| | | LDGGKTMFLGLKEDG | LDGGKTMFL |
| | | VDQDQSYLAPNNVIV | VDQDQSYLA |
| | | SEIYNKSLKIGQSIM | IYNKSLKIG |
| | | VIVSAVKKVDSLMYS | VKKVDSLMY |
| | | GAKYANSNIKVVSQY | YANSNIKVV |
| | | NVIVSAVKKVDSLMY | VIVSAVKKV |
| | | KIGFVGGVRGKVLES | VGGVRGKVL |
| | | IWGIGFRLSDILFQR | IWGIGFRLS |
| | | TGKIGFVGGVRGKVL | IGFVGGVRG |
| | | LAPNNVIVSAVKKVD | VIVSAVKKV |

| | | | |
|---|---|---|---|
| | | VKKVDSLMYSLTKKY | VDSLMYSLT |
| | | GKIGFVGGVRGKVLE | VGGVRGKVL |
| | | KKVDSLMYSLTKKYL | LMYSLTKKY |
| | | DSLMYSLTKKYLETG | LMYSLTKKY |
| | | SYLAPNNVIVSAVKK | LAPNNVIVS |
| | | LVLNENLKSNYSEIY | NENLKSNYS |
| | | GQSIMNGIIKVPYDK | IMNGIIKVP |
| | | LGLVLNENLKSNYSE | NENLKSNYS |
| | | KIGQSIMNGIIKVPY | IMNGIIKVP |
| | | IGQSIMNGIIKVPYD | IMNGIIKVP |
| | | GLGLVLNENLKSNYS | LVLNENLKS |
| | | VLNENLKSNYSEIYN | LKSNYSEIY |
| | | QSIMNGIIKVPYDKV | IMNGIIKVP |
| | | INIIEKASTGNSYLG | IIEKASTGN |
| | | YFLIFLFIVACSSSD | FLFIVACSS |
| | | FLIFLFIVACSSSDD | FLFIVACSS |
| | | SKAIRKLKADLNINI | IRKLKADLN |
| | | IVSAVKKVDSLMYSL | VKKVDSLMY |
| | | GFNESSSKAIRKLKA | FNESSSKAI |
| | | LIFLFIVACSSSDDG | FLFIVACSS |
| | | KAIRKLKADLNINII | IRKLKADLN |
| | | APNNVIVSAVKKVDS | VIVSAVKKV |
| | | PNNVIVSAVKKVDSL | VIVSAVKKV |
| | | DLNINIIEKASTGNS | IIEKASTGN |
| | | LNINIIEKASTGNSY | IIEKASTGN |
| | | IGVDQDQSYLAPNNV | VDQDQSYLA |
| | | NNVIVSAVKKVDSLM | VIVSAVKKV |
| | | EDGNSNLIWGIGFRL | NSNLIWGIG |
| | | LFQRASENVSVNYAI | LFQRASENV |
| | | KVDSLMYSLTKKYLE | LMYSLTKKY |
| | | NINIIEKASTGNSYL | IIEKASTGN |
| | | AVKKVDSLMYSLTKK | VDSLMYSLT |
| | | VDSLMYSLTKKYLET | LMYSLTKKY |
| | | AAGLSGIGVIEAAKE | LSGIGVIEA |
| | | DHYIIGVDQDQSYLA | YIIGVDQDQ |
| | | ESFMYGYEAGAKYAN | FMYGYEAGA |
| | | SIMNGIIKVPYDKVS | IMNGIIKVP |
| | | KYANSNIKVVSQYVG | YANSNIKVV |
| | | YANSNIKVVSQYVGT | YANSNIKVV |
| | | FNESSSKAIRKLKAD | FNESSSKAI |
| | HLA-DRB1*0301 | None | |
| | HLA-DRB1*0401 | GDFGLGRSTASNMYR | GRSTASNMY |
| | | FGDFGLGRSTASNMY | FGLGRSTAS |
| | | LVLNENLKSNYSEIY | NENLKSNYS |
| | | VLNENLKSNYSEIYN | LKSNYSEIY |
| | HLA-DRB1*0404 | IKVVSQYVGTFGDFG | VVSQYVGTF |
| | | VVSQYVGTFGDFGLG | YVGTFGDFG |
| | | KVVSQYVGTFGDFGL | YVGTFGDFG |
| | | SQYVGTFGDFGLGRS | YVGTFGDFG |
| | | VSQYVGTFGDFGLGR | YVGTFGDFG |
| | | VAFLAGYFASKASKT | LAGYFASKA |
| | | EEVAFLAGYFASKAS | LAGYFASKA |
| | | SEEVAFLAGYFASKA | FLAGYFASK |
| | | EVAFLAGYFASKASK | LAGYFASKA |
| | | YVGTFGDFGLGRSTA | YVGTFGDFG |
| | | QYVGTFGDFGLGRST | YVGTFGDFG |

|  |  | AFLAGYFASKASKTG | LAGYFASKA |
|---|---|---|---|
|  | HLA-DRB1*0405 | EGVYDEIQIPKNLLN | YDEIQIPKN |
|  |  | GVYDEIQIPKNLLNI | IQIPKNLLN |
|  |  | VYDEIQIPKNLLNIS | IQIPKNLLN |
|  |  | YDEIQIPKNLLNISF | IQIPKNLLN |
|  |  | DEIQIPKNLLNISFR | IQIPKNLLN |
|  | HLA-DRB1*0701 | YEAGAKYANSNIKVV | KYANSNIKV |
|  |  | EAGAKYANSNIKVVS | YANSNIKVV |
|  |  | AGAKYANSNIKVVSQ | YANSNIKVV |
|  |  | GAKYANSNIKVVSQY | YANSNIKVV |
|  |  | AKYANSNIKVVSQYV | YANSNIKVV |
|  |  | AGYFASKASKTGKIG | YFASKASKT |
|  |  | LAGYFASKASKTGKI | YFASKASKT |
|  |  | AFLAGYFASKASKTG | YFASKASKT |
|  |  | FLAGYFASKASKTGK | YFASKASKT |
|  |  | AFDDKGFNESSSKAI | GFNESSSKA |
|  |  | DKGFNESSSKAIRKL | FNESSSKAI |
|  |  | FDDKGFNESSSKAIR | FNESSSKAI |
|  |  | DDKGFNESSSKAIRK | FNESSSKAI |
|  |  | VAFLAGYFASKASKT | VAFLAGYFA |
|  |  | KGFNESSSKAIRKLK | FNESSSKAI |
|  |  | GIIKVPYDKVSYFVL | PYDKVSYFV |
|  |  | KEDIFMLKKVYYFLI | MLKKVYYFL |
|  |  | EDIFMLKKVYYFLIF | MLKKVYYFL |
|  |  | IIKVPYDKVSYFVLQ | YDKVSYFVL |
|  |  | IKVPYDKVSYFVLQM | YDKVSYFVL |
|  |  | KVPYDKVSYFVLQME | YDKVSYFVL |
|  |  | DQSYLAPNNVIVSAV | YLAPNNVIV |
|  |  | FGDFGLGRSTASNMY | LGRSTASNM |
|  |  | GDFGLGRSTASNMYR | LGRSTASNM |
|  |  | KYANSNIKVVSQYVG | YANSNIKVV |
|  |  | YANSNIKVVSQYVGT | YANSNIKVV |
|  |  | DIFMLKKVYYFLIFL | MLKKVYYFL |
|  | HLA-DRB1*0802 | None |  |
|  | HLA-DRB1*0901 | GVDIIFAAAGLSGIG | IIFAAAGLS |
|  |  | DGVDIIFAAAGLSGI | IIFAAAGLS |
|  |  | VDIIFAAAGLSGIGV | IIFAAAGLS |
|  |  | DKGFNESSSKAIRKL | FNESSSKAI |
|  |  | KGFNESSSKAIRKLK | FNESSSKAI |
|  |  | AFDDKGFNESSSKAI | GFNESSSKA |
|  |  | FDDKGFNESSSKAIR | FNESSSKAI |
|  |  | DDKGFNESSSKAIRK | FNESSSKAI |
|  |  | RDGVDIIFAAAGLSG | IIFAAAGLS |
|  |  | YRDGVDIIFAAAGLS | YRDGVDIIF |
|  |  | EAGAKYANSNIKVVS | YANSNIKVV |
|  |  | YEAGAKYANSNIKVV | AKYANSNIK |
|  |  | AGAKYANSNIKVVSQ | YANSNIKVV |
|  |  | DIIFAAAGLSGIGVI | IIFAAAGLS |
|  |  | IIFAAAGLSGIGVIE | IIFAAAGLS |
|  |  | GAKYANSNIKVVSQY | YANSNIKVV |
|  |  | LAGYFASKASKTGKI | YFASKASKT |
|  |  | AKYANSNIKVVSQYV | YANSNIKVV |
|  |  | AGYFASKASKTGKIG | YFASKASKT |
|  |  | AFLAGYFASKASKTG | YFASKASKT |
|  |  | FLAGYFASKASKTGK | YFASKASKT |

|  |  |  |  |
|---|---|---|---|
|  |  | VAFLAGYFASKASKT | LAGYFASKA |
|  |  | LSDILFQRASENVSV | FQRASENVS |
|  |  | SDILFQRASENVSVN | FQRASENVS |
|  |  | DILFQRASENVSVNY | FQRASENVS |
|  |  | RLSDILFQRASENVS | LFQRASENV |
|  |  | LIWGIGFRLSDILFQ | IGFRLSDIL |
|  |  | IWGIGFRLSDILFQR | FRLSDILFQ |
|  |  | WGIGFRLSDILFQRA | FRLSDILFQ |
|  |  | ILFQRASENVSVNYA | FQRASENVS |
|  |  | GFNESSSKAIRKLKA | FNESSSKAI |
|  |  | GIGFRLSDILFQRAS | FRLSDILFQ |
|  |  | FNESSSKAIRKLKAD | FNESSSKAI |
|  |  | IGFRLSDILFQRASE | FRLSDILFQ |
|  |  | GYFASKASKTGKIGF | YFASKASKT |
|  | HLA-DRB1*1101 | None |  |
|  | HLA-DRB1*1302 | KYANSNIKVVSQYVG | ANSNIKVVS |
|  |  | KIGQSIMNGIIKVPY | IGQSIMNGI |
|  |  | IGQSIMNGIIKVPYD | IGQSIMNGI |
|  |  | LKIGQSIMNGIIKVP | IGQSIMNGI |
|  |  | YANSNIKVVSQYVGT | IKVVSQYVG |
|  |  | ANSNIKVVSQYVGTF | IKVVSQYVG |
|  |  | SLKIGQSIMNGIIKV | IGQSIMNGI |
|  |  | KSLKIGQSIMNGIIK | IGQSIMNGI |
|  |  | NSNIKVVSQYVGTFG | IKVVSQYVG |
|  |  | SNIKVVSQYVGTFGD | IKVVSQYVG |
|  |  | NKSLKIGQSIMNGII | IGQSIMNGI |
|  |  | YNKSLKIGQSIMNGI | LKIGQSIMN |
|  |  | KEDIFMLKKVYYFLI | IFMLKKVYY |
|  |  | EDIFMLKKVYYFLIF | IFMLKKVYY |
|  |  | AKYANSNIKVVSQYV | YANSNIKVV |
|  |  | GAKYANSNIKVVSQY | YANSNIKVV |
|  |  | GQSIMNGIIKVPYDK | MNGIIKVPY |
|  |  | EAGAKYANSNIKVVS | YANSNIKVV |
|  |  | AGAKYANSNIKVVSQ | YANSNIKVV |
|  |  | LKADLNINIIEKAST | LNINIIEKA |
|  |  | QSIMNGIIKVPYDKV | MNGIIKVPY |
|  |  | EIQIPKNLLNISFRS | IPKNLLNIS |
|  |  | IQIPKNLLNISFRSE | IPKNLLNIS |
|  |  | SKEDIFMLKKVYYFL | IFMLKKVYY |
|  |  | KADLNINIIEKASTG | LNINIIEKA |
|  |  | DEIQIPKNLLNISFR | IPKNLLNIS |
|  |  | KSKEDIFMLKKVYYF | IFMLKKVYY |
|  |  | ADLNINIIEKASTGN | LNINIIEKA |
|  |  | YDEIQIPKNLLNISF | IPKNLLNIS |
|  |  | VYDEIQIPKNLLNIS | IQIPKNLLN |
|  |  | RKLKADLNINIIEKA | KADLNINII |
|  |  | DIFMLKKVYYFLIFL | IFMLKKVYY |
|  |  | KLKADLNINIIEKAS | LNINIIEKA |
|  |  | QIPKNLLNISFRSEE | IPKNLLNIS |
|  |  | IPKNLLNISFRSEEV | IPKNLLNIS |
|  |  | SIMNGIIKVPYDKVS | MNGIIKVPY |
|  |  | GLGLVLNENLKSNYS | LNENLKSNY |
|  |  | GKIGFVGGVRGKVLE | IGFVGGVRG |
|  |  | NYSEIYNKSLKIGQS | IYNKSLKIG |
|  |  | IFMLKKVYYFLIFLF | IFMLKKVYY |
|  |  | YSEIYNKSLKIGQSI | IYNKSLKIG |
|  |  | LGLVLNENLKSNYSE | LNENLKSNY |

|  |  |  |  |
|---|---|---|---|
|  |  | SNYSEIYNKSLKIGQ | IYNKSLKIG |
|  |  | LAPNNVIVSAVKKVD | PNNVIVSAV |
|  |  | LNINIIEKASTGNSY | LNINIIEKA |
|  |  | SEIYNKSLKIGQSIM | IYNKSLKIG |
|  |  | NIKVVSQYVGTFGDF | IKVVSQYVG |
|  |  | YLAPNNVIVSAVKKV | PNNVIVSAV |
|  |  | DGLGLVLNENLKSNY | LVLNENLKS |
|  |  | DLNINIIEKASTGNS | LNINIIEKA |
|  |  | SYLAPNNVIVSAVKK | LAPNNVIVS |
|  |  | EIYNKSLKIGQSIMN | IYNKSLKIG |
|  |  | TGKIGFVGGVRGKVL | IGFVGGVRG |
|  |  | DQSYLAPNNVIVSAV | LAPNNVIVS |
|  |  | IKSKEDIFMLKKVYY | IKSKEDIFM |
|  |  | QSYLAPNNVIVSAVK | LAPNNVIVS |
|  |  | KSNYSEIYNKSLKIG | SEIYNKSLK |
|  |  | IKVVSQYVGTFGDFG | IKVVSQYVG |
|  |  | LVLNENLKSNYSEIY | LNENLKSNY |
|  |  | GLVLNENLKSNYSEI | LNENLKSNY |
|  |  | IYNKSLKIGQSIMNG | IYNKSLKIG |
|  |  | VSAVKKVDSLMYSLT | VKKVDSLMY |
|  |  | NVIVSAVKKVDSLMY | IVSAVKKVD |
|  |  | YEAGAKYANSNIKVV | AKYANSNIK |
|  |  | KTGKIGFVGGVRGKV | IGFVGGVRG |
|  |  | SAVKKVDSLMYSLTK | VKKVDSLMY |
|  |  | VIVSAVKKVDSLMYS | VKKVDSLMY |
|  |  | IMNGIIKVPYDKVSY | MNGIIKVPY |
|  |  | APNNVIVSAVKKVDS | VIVSAVKKV |
|  |  | ASKTGKIGFVGGVRG | KIGFVGGVR |
|  |  | QDQSYLAPNNVIVSA | LAPNNVIVS |
|  |  | SKTGKIGFVGGVRGK | IGFVGGVRG |
|  |  | PNNVIVSAVKKVDSL | VIVSAVKKV |
|  |  | KIGFVGGVRGKVLES | IGFVGGVRG |
|  |  | IGFVGGVRGKVLESF | IGFVGGVRG |
|  |  | VKKVDSLMYSLTKKY | VDSLMYSLT |
|  |  | AVKKVDSLMYSLTKK | VDSLMYSLT |
|  | HLA-DRB1*1501 | KVYYFLIFLFIVACS | FLIFLFIVA |
|  |  | KKVYYFLIFLFIVAC | FLIFLFIVA |
|  |  | VYYFLIFLFIVACSS | FLIFLFIVA |
|  |  | LKKVYYFLIFLFIVA | YFLIFLFIV |
|  |  | YYFLIFLFIVACSSS | FLIFLFIVA |
|  |  | YEIYFLYFFIKSKED | FLYFFIKSK |
|  |  | YYEIYFLYFFIKSKE | FLYFFIKSK |
|  |  | AFLAGYFASKASKTG | LAGYFASKA |
|  |  | NYYEIYFLYFFIKSK | IYFLYFFIK |
|  |  | EIYFLYFFIKSKEDI | FLYFFIKSK |
|  |  | FLAGYFASKASKTGK | FASKASKTG |
|  |  | VAFLAGYFASKASKT | FLAGYFASK |
|  |  | YFLIFLFIVACSSSD | FLIFLFIVA |
|  | HLA-DRB3*0101 | None |  |
|  | HLA-DRB4*0101 | IQIPKNLLNISFRSE | IPKNLLNIS |
|  | HLA-DRB5*0101 | None |  |
|  |  |  |  |
| AAC70056.1<br>Decorin binding protein A | HLA-DRB1*0101 | ILTLTLLASLLAACS | LTLLASLLA |
|  |  | LTLTLLASLLAACSL | LTLLASLLA |

| DbpA [Borrelia afzelii | | LTLLASLLAACSLTG | LASLLAACS |
|---|---|---|---|
| | | TLTLLASLLAACSLT | LASLLAACS |
| Seq5 | | TLLASLLAACSLTGK | LASLLAACS |
| | | NKIILTLTLLASLLA | ILTLTLLAS |
| | | KIILTLTLLASLLAA | LTLLASLLA |
| | | IILTLTLLASLLAAC | LTLLASLLA |
| | | LASLLAACSLTGKAR | LLAACSLTG |
| | | LLASLLAACSLTGKA | LASLLAACS |
| | | IKYNKIILTLTLLAS | YNKIILTLT |
| | | ASLLAACSLTGKARL | LLAACSLTG |
| | | YNKIILTLTLLASLL | ILTLTLLAS |
| | | KYNKIILTLTLLASL | ILTLTLLAS |
| | | MIKYNKIILTLTLLA | YNKIILTLT |
| | | VGGSQIRAAKIRVAD | IRAAKIRVA |
| | | KVGGSQIRAAKIRVA | VGGSQIRAA |
| | | GSQIRAAKIRVADLT | IRAAKIRVA |
| | | GGSQIRAAKIRVADL | IRAAKIRVA |
| | | SQIRAAKIRVADLTI | IRAAKIRVA |
| | | IYDLILNAAKAVEKI | LILNAAKAV |
| | | GIYDLILNAAKAVEK | LILNAAKAV |
| | | FSGIYDLILNAAKAV | IYDLILNAA |
| | | SGIYDLILNAAKAVE | LILNAAKAV |
| | | YDLILNAAKAVEKIG | LILNAAKAV |
| | | LLAACSLTGKARLES | LLAACSLTG |
| | | SLLAACSLTGKARLE | LLAACSLTG |
| | | TGGKVGGSQIRAAKI | VGGSQIRAA |
| | | DLILNAAKAVEKIGM | LILNAAKAV |
| | | GGKVGGSQIRAAKIR | VGGSQIRAA |
| | | QIRAAKIRVADLTIK | IRAAKIRVA |
| | | GKVGGSQIRAAKIRV | VGGSQIRAA |
| | | LILNAAKAVEKIGMQ | LILNAAKAV |
| | | IRAAKIRVADLTIKF | IRAAKIRVA |
| | | QTGGKVGGSQIRAAK | VGGSQIRAA |
| | | TQTGGKVGGSQIRAA | GKVGGSQIR |
| | | KAVEKIGMQGMKQAV | IGMQGMKQA |
| | | DGIIAIVKVMKAKVE | IAIVKVMKA |
| | | GIIAIVKVMKAKVEN | VKVMKAKVE |
| | | VEKIGMQGMKQAVEE | IGMQGMKQA |
| | | AVEKIGMQGMKQAVE | IGMQGMKQA |
| | | AKAVEKIGMQGMKQA | VEKIGMQGM |
| | | EKIGMQGMKQAVEEA | IGMQGMKQA |
| | | IAIVKVMKAKVENIK | VKVMKAKVE |
| | | IIAIVKVMKAKVENI | VKVMKAKVE |
| | | ADGIIAIVKVMKAKV | IAIVKVMKA |
| | | AIVKVMKAKVENIKE | VKVMKAKVE |
| | | TTADGIIAIVKVMKA | DGIIAIVKV |
| | | KIRVADLTIKFLEAT | VADLTIKFL |
| | | IRVADLTIKFLEATE | LTIKFLEAT |
| | | TADGIIAIVKVMKAK | IAIVKVMKA |
| | | KIGMQGMKQAVEEAA | IGMQGMKQA |
| | | IGMQGMKQAVEEAAK | IGMQGMKQA |
| | | VADLTIKFLEATEEE | LTIKFLEAT |
| | | RVADLTIKFLEATEE | LTIKFLEAT |
| | | ADLTIKFLEATEEET | LTIKFLEAT |
| | | EDFSGIYDLILNAAK | IYDLILNAA |
| | | DAGVKTDAFTETQTG | VKTDAFTET |
| | | AGVKTDAFTETQTGG | VKTDAFTET |

| | | | |
|---|---|---|---|
| | | EDAGVKTDAFTETQT | VKTDAFTET |
| | | AEDAGVKTDAFTETQ | VKTDAFTET |
| | | RAAKIRVADLTIKFL | IRVADLTIK |
| | | EEDFSGIYDLILNAA | FSGIYDLIL |
| | | EAEDAGVKTDAFTET | AGVKTDAFT |
| | | AAKIRVADLTIKFLE | VADLTIKFL |
| | | AKIRVADLTIKFLEA | VADLTIKFL |
| | | LNAAKAVEKIGMQGM | LNAAKAVEK |
| | HLA-DRB1*0301 | None | |
| | HLA-DRB1*0401 | NKIILTLTLLASLLA | IILTLTLLA |
| | | IKYNKIILTLTLLAS | IILTLTLLA |
| | | MIKYNKIILTLTLLA | YNKIILTLT |
| | | KYNKIILTLTLLASL | IILTLTLLA |
| | | KIILTLTLLASLLAA | LTLLASLLA |
| | | IILTLTLLASLLAAC | LTLLASLLA |
| | | YNKIILTLTLLASLL | IILTLTLLA |
| | | ILTLTLLASLLAACS | LTLLASLLA |
| | | LTLTLLASLLAACSL | LTLLASLLA |
| | | LTLLASLLAACSLTG | LTLLASLLA |
| | | TLTLLASLLAACSLT | LTLLASLLA |
| | HLA-DRB1*0404 | NKIILTLTLLASLLA | IILTLTLLA |
| | | IKYNKIILTLTLLAS | IILTLTLLA |
| | | KYNKIILTLTLLASL | IILTLTLLA |
| | | YNKIILTLTLLASLL | IILTLTLLA |
| | | MIKYNKIILTLTLLA | YNKIILTLT |
| | | KIILTLTLLASLLAA | IILTLTLLA |
| | | IILTLTLLASLLAAC | IILTLTLLA |
| | | LTLLASLLAACSLTG | LASLLAACS |
| | | LLASLLAACSLTGKA | LLAACSLTG |
| | | TLLASLLAACSLTGK | LLAACSLTG |
| | | LASLLAACSLTGKAR | LLAACSLTG |
| | | ASLLAACSLTGKARL | LLAACSLTG |
| | HLA-DRB1*0405 | NKIILTLTLLASLLA | IILTLTLLA |
| | | IKYNKIILTLTLLAS | IILTLTLLA |
| | | KYNKIILTLTLLASL | IILTLTLLA |
| | | MIKYNKIILTLTLLA | YNKIILTLT |
| | | YNKIILTLTLLASLL | IILTLTLLA |
| | HLA-DRB1*0701 | KVGGSQIRAAKIRVA | GSQIRAAKI |
| | | VGGSQIRAAKIRVAD | IRAAKIRVA |
| | | GGSQIRAAKIRVADL | IRAAKIRVA |
| | | GSQIRAAKIRVADLT | IRAAKIRVA |
| | | SQIRAAKIRVADLTI | IRAAKIRVA |
| | HLA-DRB1*0802 | None | |
| | HLA-DRB1*0901 | YDLILNAAKAVEKIG | LILNAAKAV |
| | | GIYDLILNAAKAVEK | LILNAAKAV |
| | | IYDLILNAAKAVEKI | LILNAAKAV |
| | HLA-DRB1*1101 | None | |
| | HLA-DRB1*1302 | IKYNKIILTLTLLAS | YNKIILTLT |
| | | MIKYNKIILTLTLLA | YNKIILTLT |
| | | KYNKIILTLTLLASL | YNKIILTLT |
| | | YNKIILTLTLLASLL | YNKIILTLT |
| | | NKIILTLTLLASLLA | ILTLTLLAS |
| | | KIILTLTLLASLLAA | LTLLASLLA |
| | | DGIIAIVKVMKAKVE | IAIVKVMKA |
| | | ADGIIAIVKVMKAKV | IAIVKVMKA |
| | | DLILNAAKAVEKIGM | ILNAAKAVE |

| | | | |
|---|---|---|---|
| | | IYDLILNAAKAVEKI | ILNAAKAVE |
| | | GIYDLILNAAKAVEK | ILNAAKAVE |
| | | IILTLTLLASLLAAC | LTLLASLLA |
| | | GIIAIVKVMKAKVEN | IAIVKVMKA |
| | | YDLILNAAKAVEKIG | ILNAAKAVE |
| | | TTADGIIAIVKVMKA | IIAIVKVMK |
| | | ILTLTLLASLLAACS | LTLLASLLA |
| | | TADGIIAIVKVMKAK | IAIVKVMKA |
| | | SGIYDLILNAAKAVE | LILNAAKAV |
| | | AKAVEKIGMQGMKQA | VEKIGMQGM |
| | | LILNAAKAVEKIGMQ | ILNAAKAVE |
| | | KAVEKIGMQGMKQAV | IGMQGMKQA |
| | | IAIVKVMKAKVENIK | VKVMKAKVE |
| | | IIAIVKVMKAKVENI | VKVMKAKVE |
| | HLA-DRB1*1501 | KIILTLTLLASLLAA | ILTLTLLAS |
| | | NKIILTLTLLASLLA | ILTLTLLAS |
| | | KYNKIILTLTLLASL | ILTLTLLAS |
| | | IKYNKIILTLTLLAS | KYNKIILTL |
| | | YNKIILTLTLLASLL | ILTLTLLAS |
| | | QIRAAKIRVADLTIK | IRAAKIRVA |
| | | IRAAKIRVADLTIKF | IRVADLTIK |
| | | ILTLTLLASLLAACS | ILTLTLLAS |
| | | IILTLTLLASLLAAC | ILTLTLLAS |
| | | TLLASLLAACSLTGK | LLAACSLTG |
| | | LASLLAACSLTGKAR | LLAACSLTG |
| | | LLASLLAACSLTGKA | LLAACSLTG |
| | HLA-DRB3*0101 | None | |
| | HLA-DRB4*0101 | None | |
| | HLA-DRB5*0101 | GIYDLILNAAKAVEK | YDLILNAAK |
| | | IYDLILNAAKAVEKI | LNAAKAVEK |
| | | YDLILNAAKAVEKIG | LNAAKAVEK |
| | | | |
| AAC70057.1 Decorin binding protein A DbpA [Borrelia garinii] Seq6 | HLA-DRB1*0101 | EIGIQKMTGTVTKEA | IQKMTGTVT |
| | | IGIQKMTGTVTKEAE | IQKMTGTVT |
| | | LEEIGIQKMTGTVTK | IQKMTGTVT |
| | | EEIGIQKMTGTVTKE | IQKMTGTVT |
| | | PLEEIGIQKMTGTVT | IGIQKMTGT |
| | | LLKLSLIVSLLVACG | LSLIVSLLV |
| | | LKLSLIVSLLVACGL | IVSLLVACG |
| | | LSLIVSLLVACGLTG | IVSLLVACG |
| | | IQKMTGTVTKEAEKT | IQKMTGTVT |
| | | SLIVSLLVACGLTGE | IVSLLVACG |
| | | KLSLIVSLLVACGLT | IVSLLVACG |
| | | GIQKMTGTVTKEAEK | IQKMTGTVT |
| | | NKILLKLSLIVSLLV | LKLSLIVSL |
| | | IKYNKILLKLSLIVS | YNKILLKLS |
| | | KILLKLSLIVSLLVA | LKLSLIVSL |
| | | YNKILLKLSLIVSLL | LKLSLIVSL |
| | | KYNKILLKLSLIVSL | ILLKLSLIV |
| | | MIKYNKILLKLSLIV | YNKILLKLS |
| | | ILLKLSLIVSLLVAC | LKLSLIVSL |
| | | LIVSLLVACGLTGET | IVSLLVACG |
| | | IVSLLVACGLTGETK | IVSLLVACG |
| | | QKMTGTVTKEAEKTP | MTGTVTKEA |
| | | AEKLKKSGSSGAFSA | LKKSGSSGA |
| | | EAEKLKKSGSSGAFS | LKKSGSSGA |

| | | | |
|---|---|---|---|
| | | EKLKKSGSSGAFSAM | LKKSGSSGA |
| | | EEAEKLKKSGSSGAF | LKKSGSSGA |
| | | KEEAEKLKKSGSSGA | AEKLKKSGS |
| | | LKNLEEKANTAATTT | LEEKANTAA |
| | | FILKAKIKAIQVAER | AKIKAIQVA |
| | | ILKAKIKAIQVAERF | IKAIQVAER |
| | | VSLLVACGLTGETKI | LLVACGLTG |
| | | SGSSGAFSAMYDLMI | SSGAFSAMY |
| | | LKAKIKAIQVAERFV | IKAIQVAER |
| | | ADGIIAIAQAMEEKL | IAIAQAMEE |
| | | FSAMYDLMIDVSKPL | FSAMYDLMI |
| | | KMTGTVTKEAEKTPP | MTGTVTKEA |
| | | MTGTVTKEAEKTPPT | MTGTVTKEA |
| | | ALKNLEEKANTAATT | LEEKANTAA |
| | | SGAFSAMYDLMIDVS | FSAMYDLMI |
| | | TTADGIIAIAQAMEE | GIIAIAQAM |
| | | GAFSAMYDLMIDVSK | FSAMYDLMI |
| | | TADGIIAIAQAMEEK | IAIAQAMEE |
| | | LLVACGLTGETKIRL | LLVACGLTG |
| | | KLKKSGSSGAFSAMY | LKKSGSSGA |
| | | GSSGAFSAMYDLMID | FSAMYDLMI |
| | | LKKSGSSGAFSAMYD | LKKSGSSGA |
| | | KAKIKAIQVAERFVK | IKAIQVAER |
| | | VKFEAFTNTQTGSKI | FEAFTNTQT |
| | | DGIIAIAQAMEEKLN | IAIAQAMEE |
| | | SSGAFSAMYDLMIDV | FSAMYDLMI |
| | | AKIKAIQVAERFVKA | IKAIQVAER |
| | | GVKFEAFTNTQTGSK | FEAFTNTQT |
| | | GIIAIAQAMEEKLNN | IAIAQAMEE |
| | | PEFILKAKIKAIQVA | ILKAKIKAI |
| | HLA-DRB1*0301 | None | |
| | HLA-DRB1*0401 | LLKLSLIVSLLVACG | LSLIVSLLV |
| | | LKLSLIVSLLVACGL | LSLIVSLLV |
| | | NKILLKLSLIVSLLV | ILLKLSLIV |
| | | ILLKLSLIVSLLVAC | LSLIVSLLV |
| | | KILLKLSLIVSLLVA | LSLIVSLLV |
| | HLA-DRB1*0404 | GVKFEAFTNTQTGSK | KFEAFTNTQ |
| | | VKFEAFTNTQTGSKI | KFEAFTNTQ |
| | | EGVKFEAFTNTQTGS | KFEAFTNTQ |
| | | KEGVKFEAFTNTQTG | KFEAFTNTQ |
| | | KFEAFTNTQTGSKIS | KFEAFTNTQ |
| | | KKEGVKFEAFTNTQT | KFEAFTNTQ |
| | | AKKEGVKFEAFTNTQ | VKFEAFTNT |
| | | LIVSLLVACGLTGET | LLVACGLTG |
| | | IVSLLVACGLTGETK | LLVACGLTG |
| | | LSLIVSLLVACGLTG | IVSLLVACG |
| | | SLIVSLLVACGLTGE | LLVACGLTG |
| | | VSLLVACGLTGETKI | LLVACGLTH |
| | HLA-DRB1*0405 | GVKFEAFTNTQTGSK | FEAFTNTQT |
| | | KEGVKFEAFTNTQTG | FEAFTNTQT |
| | | VKFEAFTNTQTGSKI | FEAFTNTQT |
| | | KKEGVKFEAFTNTQT | GVKFEAFTN |
| | | EGVKFEAFTNTQTGS | FEAFTNTQT |
| | HLA-DRB1*0701 | NKILLKLSLIVSLLV | ILLKLSLIV |
| | | KILLKLSLIVSLLVA | LSLIVSLLV |
| | | ILLKLSLIVSLLVAC | LSLIVSLLV |

| | HLA-DRB1*0802 | None | |
|---|---|---|---|
| | HLA-DRB1*0901 | None | |
| | HLA-DRB1*1101 | None | |
| | HLA-DRB1*1302 | LEEIGIQKMTGTVTK | IGIQKMTGT |
| | | EEIGIQKMTGTVTKE | IGIQKMTGT |
| | | PLEEIGIQKMTGTVT | IGIQKMTGT |
| | | EIGIQKMTGTVTKEA | IGIQKMTGT |
| | | IGIQKMTGTVTKEAE | IGIQKMTGT |
| | | NKILLKLSLIVSLLV | LLKLSLIVS |
| | | KILLKLSLIVSLLVA | LLKLSLIVS |
| | | IKYNKILLKLSLIVS | IKYNKILLK |
| | | KPLEEIGIQKMTGTV | IGIQKMTGT |
| | | YNKILLKLSLIVSLL | LLKLSLIVS |
| | | SKPLEEIGIQKMTGT | LEEIGIQKM |
| | | KYNKILLKLSLIVSL | LLKLSLIVS |
| | | MIKYNKILLKLSLIV | IKYNKILLK |
| | | ILLKLSLIVSLLVAC | LLKLSLIVS |
| | | LLKLSLIVSLLVACG | LLKLSLIVS |
| | | LKLSLIVSLLVACGL | LSLIVSLLV |
| | | AMEEKLNNVNKKQHD | LNNVNKKQH |
| | | MEEKLNNVNKKQHDA | LNNVNKKQH |
| | | EEKLNNVNKKQHDAL | LNNVNKKQH |
| | | EKLNNVNKKQHDALK | LNNVNKKQH |
| | | KDEINKIKANAKKEG | INKIKANAK |
| | | DEINKIKANAKKEGV | IKANAKKEG |
| | | KLSLIVSLLVACGLT | LSLIVSLLV |
| | | LSLIVSLLVACGLTG | LSLIVSLLV |
| | | EINKIKANAKKEGVK | IKANAKKEG |
| | | PEFILKAKIKAIQVA | FILKAKIKA |
| | | KPEFILKAKIKAIQV | FILKAKIKA |
| | | INKIKANAKKEGVKF | IKANAKKEG |
| | | EKPEFILKAKIKAIQ | FILKAKIKA |
| | | GIQKMTGTVTKEAEK | IQKMTGTVT |
| | HLA-DRB1*1501 | IKYNKILLKLSLIVS | ILLKLSLIV |
| | | NKILLKLSLIVSLLV | LLKLSLIVS |
| | | KYNKILLKLSLIVSL | LLKLSLIVS |
| | | YNKILLKLSLIVSLL | LLKLSLIVS |
| | | KILLKLSLIVSLLVA | LLKLSLIVS |
| | | ILLKLSLIVSLLVAC | LLKLSLIVS |
| | | LLKLSLIVSLLVACG | LLKLSLIVS |
| | | MIKYNKILLKLSLIV | IKYNKILLK |
| | HLA-DRB3*0101 | None | |
| | HLA-DRB4*0101 | None | |
| | HLA-DRB5*0101 | GVKFEAFTNTQTGSK | FEAFTNTQT |
| | | KAKIKAIQVAERFVK | IKAIQVAER |
| | | VKFEAFTNTQTGSKI | FTNTQTGSK |
| | | AKIKAIQVAERFVKA | IQVAERFVK |
| | | | |
| AAC70021.1<br>Decorin binding protein B<br>DbpB [Borrelia burgdorferi]<br><br>Seq7 | HLA-DRB1*0101 | LEDVGIIGLKARVLE | IIGLKARVL |
| | | EDVGIIGLKARVLEE | IIGLKARVL |
| | | DVGIIGLKARVLEES | IIGLKARVL |
| | | VGIIGLKARVLEESK | IIGLKARVL |
| | | ALFFKLLVACSIGLV | FKLLVACSI |
| | | IALFFKLLVACSIGL | FKLLVACSI |
| | | GIIGLKARVLEESKN | IIGLKARVL |

| | | | |
|---|---|---|---|
| | | IIGLKARVLEESKNN | IIGLKARVL |
| | | IVIALFFKLLVACSI | FFKLLVACS |
| | | VIALFFKLLVACSIG | FKLLVACSI |
| | | LFFKLLVACSIGLVE | FKLLVACSI |
| | | SGQFLAMFDLMLEVV | FLAMFDLML |
| | | GQFLAMFDLMLEVVE | FLAMFDLML |
| | | SIVIALFFKLLVACS | VIALFFKLL |
| | | FFKLLVACSIGLVER | LLVACSIGL |
| | | GSKVTSGGLALREAK | VTSGGLALR |
| | | TGSKVTSGGLALREA | VTSGGLALR |
| | | SKVTSGGLALREAKV | VTSGGLALR |
| | | FKLLVACSIGLVERT | LLVACSIGL |
| | | FLAMFDLMLEVVESL | FLAMFDLML |
| | | QFLAMFDLMLEVVES | FLAMFDLML |
| | | FEAFTGLKTGSKVTS | FTGLKTGSK |
| | | TSGGLALREAKVQAI | LALREAKVQ |
| | | SGGLALREAKVQAIV | LREAKVQAI |
| | | EAFTGLKTGSKVTSG | LKTGSKVTS |
| | | LKTGSKVTSGGLALR | LKTGSKVTS |
| | | GNSGQFLAMFDLMLE | FLAMFDLML |
| | | NSGQFLAMFDLMLEV | FLAMFDLML |
| | | KTGSKVTSGGLALRE | VTSGGLALR |
| | | AFTGLKTGSKVTSGG | LKTGSKVTS |
| | | FTGLKTGSKVTSGGL | LKTGSKVTS |
| | | KVTSGGLALREAKVQ | VTSGGLALR |
| | | GGLALREAKVQAIVE | LREAKVQAI |
| | | VTSGGLALREAKVQA | VTSGGLALR |
| | | GLALREAKVQAIVET | LREAKVQAI |
| | | LALREAKVQAIVETG | LREAKVQAI |
| | | VLFEAFTGLKTGSKV | FTGLKTGSK |
| | | GVLFEAFTGLKTGSK | FEAFTGLKT |
| | | TGLKTGSKVTSGGLA | LKTGSKVTS |
| | | TGNSGQFLAMFDLML | GQFLAMFDL |
| | | INTAERLLAAKAQIE | AERLLAAKA |
| | | LFEAFTGLKTGSKVT | FTGLKTGSK |
| | | NTAERLLAAKAQIEN | LLAAKAQIE |
| | | TAERLLAAKAQIENQ | LLAAKAQIE |
| | | ILKIKKEATGKGVLF | IKKEATGKG |
| | | LAMFDLMLEVVESLE | MFDLMLEVV |
| | | AERLLAAKAQIENQL | LLAAKAQIE |
| | | NKILKIKKEATGKGV | IKKEATGKG |
| | | LKIKKEATGKGVLFE | IKKEATGKG |
| | | KILKIKKEATGKGVL | IKKEATGKG |
| | | KNKILKIKKEATGKG | ILKIKKEAT |
| | | KLLVACSIGLVERTN | LLVACSIGL |
| | | NPINTAERLLAAKAQ | INTAERLLA |
| | | IGLVERTNAALESSS | RTNAALESS |
| | | SIGLVERTNAALESS | ERTNAALES |
| | | ERLLAAKAQIENQLK | LLAAKAQIE |
| | | NNPINTAERLLAAKA | INTAERLLA |
| | | ALREAKVQAIVETGK | LREAKVQAI |
| | | GLVERTNAALESSSK | RTNAALESS |
| | | GLKTGSKVTSGGLAL | LKTGSKVTS |
| | | LREAKVQAIVETGKF | LREAKVQAI |
| | | LLVACSIGLVERTNA | LLVACSIGL |
| | HLA-DRB1*0301 | None | |

| | | | |
|---|---|---|---|
| | HLA-DRB1*0401 | IVIALFFKLLVACSI<br>SIVIALFFKLLVACS<br>VIALFFKLLVACSIG<br>IALFFKLLVACSIGL<br>ALFFKLLVACSIGLV | FFKLLVACS<br>IALFFKLLV<br>FFKLLVACS<br>FFKLLVACS<br>FFKLLVACS |
| | HLA-DRB1*0404 | GVLFEAFTGLKTGSK<br>ARVLEESKNNPINTA<br>KGVLFEAFTGLKTGS<br>GKGVLFEAFTGLKTG<br>IGLKARVLEESKNNP<br>LKARVLEESKNNPIN<br>KARVLEESKNNPINT<br>GLKARVLEESKNNPI<br>TGKGVLFEAFTGLKT<br>ATGKGVLFEAFTGLK<br>LFEAFTGLKTGSKVT<br>VLFEAFTGLKTGSKV<br>RVLEESKNNPINTAE<br>VLEESKNNPINTAER | LFEAFTGLK<br>VLEESKNNP<br>LFEAFTGLK<br>LFEAFTGLK<br>ARVLEESKN<br>VLEESKNNP<br>VLEESKNNP<br>VLEESKNNP<br>LFEAFTGLK<br>KGVLFEAFT<br>LFEAFTGLK<br>LFEAFTGLK<br>VLEESKNNP<br>VLEESKNNP |
| | HLA-DRB1*0405 | IVIALFFKLLVACSI<br>SIVIALFFKLLVACS<br>AMFDLMLEVVESLED | IALFFKLLV<br>IALFFKLLV<br>MLEVVESLE |
| | HLA-DRB1*0701 | EDVGIIGLKARVLEE<br>DVGIIGLKARVLEES<br>SLEDVGIIGLKARVL<br>LEDVGIIGLKARVLE<br>VGIIGLKARVLEESK | IIGLKARVL<br>IIGLKARVL<br>GIIGLKARV<br>IIGLKARVL<br>IIGLKARVL |
| | HLA-DRB1*0802 | None | |
| | HLA-DRB1*0901 | None | |
| | HLA-DRB1*1101 | None | |
| | HLA-DRB1*1302 | LEDVGIIGLKARVLE<br>EDVGIIGLKARVLEE<br>ESSSKDLKNKILKIK<br>SSSKDLKNKILKIKK<br>SSKDLKNKILKIKKE<br>KDLKNKILKIKKEAT<br>SKDLKNKILKIKKEA<br>DVGIIGLKARVLEES<br>VGIIGLKARVLEESK<br>DLKNKILKIKKEATG<br>LKNKILKIKKEATGK<br>GIIGLKARVLEESKN<br>SLEDVGIIGLKARVL<br>ESLEDVGIIGLKARV<br>VESLEDVGIIGLKAR<br>AQIENQLKVVKEKQN<br>KAQIENQLKVVKEKQ<br>AAKAQIENQLKVVKE<br>AKAQIENQLKVVKEK<br>IIGLKARVLEESKNN<br>AFTGLKTGSKVTSGG<br>FTGLKTGSKVTSGGL | VGIIGLKAR<br>IGLKARVLE<br>SSKDLKNKI<br>LKNKILKIK<br>LKNKILKIK<br>LKNKILKIK<br>LKNKILKIK<br>IGLKARVLE<br>IGLKARVLE<br>LKNKILKIK<br>LKNKILKIK<br>IGLKARVLE<br>VGIIGLKAR<br>VGIIGLKAR<br>LEDVGIIGL<br>IENQLKVVK<br>IENQLKVVK<br>IENQLKVVK<br>IENQLKVVK<br>IGLKARVLE<br>LKTGSKVTS<br>LKTGSKVTS |

| | | | |
|---|---|---|---|
| | | TGLKTGSKVTSGGLA | LKTGSKVTS |
| | | IGLKARVLEESKNNP | IGLKARVLE |
| | | FEAFTGLKTGSKVTS | TGLKTGSKV |
| | | EAFTGLKTGSKVTSG | LKTGSKVTS |
| | | LAAKAQIENQLKVVK | AKAQIENQL |
| | HLA-DRB1*1501 | SLEDVGIIGLKARVL | VGIIGLKAR |
| | | LEDVGIIGLKARVLE | VGIIGLKAR |
| | | EDVGIIGLKARVLEE | VGIIGLKAR |
| | | SIVIALFFKLLVACS | ALFFKLLVA |
| | | IVIALFFKLLVACSI | ALFFKLLVA |
| | | GVLFEAFTGLKTGSK | FEAFTGLKT |
| | | DVGIIGLKARVLEES | IIGLKARVL |
| | | VGIIGLKARVLEESK | IIGLKARVL |
| | | VLFEAFTGLKTGSKV | FTGLKTGSK |
| | | VIALFFKLLVACSIG | ALFFKLLVA |
| | | GKGVLFEAFTGLKTG | VLFEAFTGL |
| | | LFEAFTGLKTGSKVT | FTGLKTGSK |
| | HLA-DRB3*0101 | None | |
| | HLA-DRB4*0101 | None | |
| | HLA-DRB5*0101 | None | |
| | | | |
| NP_212694.1 Heat shock protein 90 [Borrelia burgdorferi B31] Seq8 | HLA-DRB1*0101 | NDPTYQMQKIMLSMG | YQMQKIMLS |
| | | DPTYQMQKIMLSMGQ | YQMQKIMLS |
| | | PTYQMQKIMLSMGQE | YQMQKIMLS |
| | | DSNDPTYQMQKIMLS | DPTYQMQKI |
| | | SNDPTYQMQKIMLSM | YQMQKIMLS |
| | | TYQMQKIMLSMGQEV | YQMQKIMLS |
| | | YQMQKIMLSMGQEVK | YQMQKIMLS |
| | | IVQNLKNLEPEKLEK | LKNLEPEKL |
| | | VQNLKNLEPEKLEKI | LKNLEPEKL |
| | | NKIVQNLKNLEPEKL | VQNLKNLEP |
| | | KIVQNLKNLEPEKLE | LKNLEPEKL |
| | | QNLKNLEPEKLEKIS | LKNLEPEKL |
| | | YTNLFYVPSKAPYDL | YTNLFYVPS |
| | | NLEYTNLFYVPSKAP | YTNLFYVPS |
| | | LEYTNLFYVPSKAPY | YTNLFYVPS |
| | | EIFLRELISNASDAI | LRELISNAS |
| | | IFLRELISNASDAID | LRELISNAS |
| | | KEIFLRELISNASDA | LRELISNAS |
| | | SILLFEEAMLTSGMP | LLFEEAMLT |
| | | HKEIFLRELISNASD | LRELISNAS |
| | | SHKEIFLRELISNAS | SHKEIFLRE |
| | | NLKNLEPEKLEKISI | LKNLEPEKL |
| | | LKNLEPEKLEKISIL | LKNLEPEKL |
| | | ISILLFEEAMLTSGM | LLFEEAMLT |
| | | TNLFYVPSKAPYDLY | VPSKAPYDL |
| | | EYTNLFYVPSKAPYD | YTNLFYVPS |
| | | LIFYFKQIALFIIFR | FKQIALFII |
| | | MILIFYFKQIALFII | YFKQIALFI |
| | | DLLYLIIHSLYSHKE | YLIIHSLYS |
| | | KDHIKEVNLSATLIK | EVNLSATLI |
| | | DHIKEVNLSATLIKE | EVNLSATLI |
| | | IFYFKQIALFIIFRL | FKQIALFII |
| | | LLYLIIHSLYSHKEI | YLIIHSLYS |

| | | ILIFYFKQIALFIIF | FKQIALFII |
|---|---|---|---|
| | | NDLLYLIIHSLYSHK | YLIIHSLY |
| | | NLSATLIKEPSAIII | LIKEPSAII |
| | | SATLIKEPSAIIIDS | LIKEPSAII |
| | | KISILLFEEAMLTSG | LLFEEAMLT |
| | | VNDLLYLIIHSLYSH | YLIIHSLYS |
| | | LSATLIKEPSAIIID | LIKEPSAII |
| | | GNLEYTNLFYVPSKA | YTNLFYVPS |
| | | ATLIKEPSAIIIDSN | LIKEPSAII |
| | | IPEYEGLKLKAINKN | YEGLKLKAI |
| | | QDLTNHLGVIAKSGT | LTNHLGVIA |
| | | EGNLEYTNLFYVPSK | YTNLFYVPS |
| | | HIKEVNLSATLIKEP | EVNLSATLI |
| | | IKEVNLSATLIKEPS | EVNLSATLI |
| | | PEYEGLKLKAINKNE | YEGLKLKAI |
| | | NLFYVPSKAPYDLYY | VPSKAPYDL |
| | | EVNDLLYLIIHSLYS | LLYLIIHSL |
| | | AEGNLEYTNLFYVPS | EGNLEYTNL |
| | | NKILSKIKSSSVKKI | LSKIKSSSV |
| | | KILSKIKSSSVKKIL | IKSSSVKKI |
| | | LFYVPSKAPYDLYYP | VPSKAPYDL |
| | | EQDLTNHLGVIAKSG | LTNHLGVIA |
| | | EKISILLFEEAMLTS | LLFEEAMLT |
| | | LLFEEAMLTSGMPSK | LLFEEAMLT |
| | | FEEAMLTSGMPSKNP | LTSGMPSKN |
| | | ILLFEEAMLTSGMPS | LLFEEAMLT |
| | | LEKISILLFEEAMLT | ISILLFEEA |
| | | EEAMLTSGMPSKNPG | LTSGMPSKN |
| | | FYFKQIALFIIFRLC | FKQIALFII |
| | | EAMLTSGMPSKNPGK | LTSGMPSKN |
| | | FLRELISNASDAIDK | LRELISNAS |
| | | LKDHIKEVNLSATLI | IKEVNLSAT |
| | | LRELISNASDAIDKL | LRELISNAS |
| | | LIPEYEGLKLKAINK | YEGLKLKAI |
| | | ILSKIKSSSVKKILS | IKSSSVKKI |
| | | LSKIKSSSVKKILSE | IKSSSVKKI |
| | | AMLTSGMPSKNPGKF | LTSGMPSKN |
| | | NLIPEYEGLKLKAIN | YEGLKLKAI |
| | | LNLIPEYEGLKLKAI | LNLIPEYEG |
| | | DEQDLTNHLGVIAKS | LTNHLGVIA |
| | | LFEEAMLTSGMPSKN | EAMLTSGMP |
| | | MDEQDLTNHLGVIAK | LTNHLGVIA |
| | | GMDEQDLTNHLGVIA | DEQDLTNHL |
| | | ENILKENPIVAAYKE | LKENPIVAA |
| | | QMQKIMLSMGQEVKE | IMLSMGQEV |
| | | KVEVTSKKALESDAY | VTSKKALES |
| | | KENILKENPIVAAYK | LKENPIVAA |
| | | SEKVEVTSKKALESD | VTSKKALES |
| | | VSEKVEVTSKKALES | KVEVTSKKA |
| | | EKVEVTSKKALESDA | VTSKKALES |
| | | TLIKEPSAIIIDSND | IKEPSAIII |
| | | VEVTSKKALESDAYI | VTSKKALES |
| | | VNLSATLIKEPSAII | SATLIKEPS |
| | | NILKENPIVAAYKEK | LKENPIVAA |
| | | GGKENILKENPIVAA | ILKENPIVA |
| | | GKENILKENPIVAAY | LKENPIVAA |
| | | LYLIIHSLYSHKEIF | YLIIHSLYS |

|  |  | YEGLKLKAINKNETS | YEGLKLKAI |
|---|---|---|---|
|  |  | EYEGLKLKAINKNET | YEGLKLKAI |
|  |  | LTNHLGVIAKSGTKE | LTNHLGVIA |
|  |  | KEVNLSATLIKEPSA | EVNLSATLI |
|  |  | SKIKSSSVKKILSEL | IKSSSVKKI |
|  |  | DLTNHLGVIAKSGTK | LTNHLGVIA |
|  |  | YLIIHSLYSHKEIFL | YLIIHSLYS |
|  |  | MQKIMLSMGQEVKEI | IMLSMGQEV |
|  |  | FYVPSKAPYDLYYPN | VPSKAPYDL |
|  |  | IKSSSVKKILSELEK | VKKILSELE |
|  |  | KIKSSSVKKILSELE | IKSSSVKKI |
|  |  | MLTSGMPSKNPGKFI | LTSGMPSKN |
|  |  | EVNLSATLIKEPSAI | EVNLSATLI |
|  |  | YFKQIALFIIFRLCY | FKQIALFII |
|  |  | LTNEKFKNIALEPKI | FKNIALEPK |
|  |  | NEKFKNIALEPKIEI | FKNIALEPK |
|  |  | LIKEPSAIIIDSNDP | LIKEPSAII |
|  |  | EKFKNIALEPKIEIS | FKNIALEPK |
|  |  | TNEKFKNIALEPKIE | FKNIALEPK |
|  |  | QKIMLSMGQEVKEIK | IMLSMGQEV |
|  |  | ILKENPIVAAYKEKG | LKENPIVAA |
|  |  | SLTNEKFKNIALEPK | LTNEKFKNI |
|  |  | LKENPIVAAYKEKGF | LKENPIVAA |
|  |  | QQNKILSKIKSSSVK | LSKIKSSSV |
|  |  | KEEKLNETTALWTKN | LNETTALWT |
|  |  | FYSAFIVSEKVEVTS | IVSEKVEVT |
|  |  | GFYSAFIVSEKVEVT | SAFIVSEKV |
|  |  | LQQNKILSKIKSSSV | NKILSKIKS |
|  |  | QNKILSKIKSSSVKK | LSKIKSSSV |
|  |  | EEKLNETTALWTKNK | LNETTALWT |
|  |  | EKEEKLNETTALWTK | LNETTALWT |
|  |  | IIDCQDLPLNVSREI | CQDLPLNVS |
|  |  | IDCQDLPLNVSREIL | LPLNVSREI |
|  |  | EEKEEKLNETTALWT | KLNETTALW |
|  |  | SAFIVSEKVEVTSKK | IVSEKVEVT |
|  |  | LTSGMPSKNPGKFIN | LTSGMPSKN |
|  |  | YSAFIVSEKVEVTSK | IVSEKVEVT |
|  |  | TNHLGVIAKSGTKEF | HLGVIAKSG |
|  |  | AFIVSEKVEVTSKKA | IVSEKVEVT |
|  |  | EKLNETTALWTKNKS | LNETTALWT |
|  |  | EVTSKKALESDAYIW | VTSKKALES |
|  |  | VTSKKALESDAYIWS | VTSKKALES |
|  | HLA-DRB1*0301 | None |  |
|  | HLA-DRB1*0401 | SHKEIFLRELISNAS | FLRELISNA |
|  |  | HKEIFLRELISNASD | LRELISNAS |
|  |  | KEIFLRELISNASDA | LRELISNAS |
|  |  | EIFLRELISNASDAI | LRELISNAS |
|  |  | IFLRELISNASDAID | LRELISNAS |
|  |  | DSNDPTYQMQKIMLS | DSNDPTYQM |
|  |  | SNDPTYQMQKIMLSM | YQMQKIMLS |
|  |  | DPTYQMQKIMLSMGQ | YQMQKIMLS |
|  |  | PTYQMQKIMLSMGQE | YQMQKIMLS |
|  |  | FLRELISNASDAIDK | LRELISNAS |
|  |  | NDPTYQMQKIMLSMG | YQMQKIMLS |
|  |  | WKIQEIIKKYSNHIN | IIKKYSNHI |
|  |  | KWKIQEIIKKYSNHI | WKIQEIIKK |

| | | | |
|---|---|---|---|
| | | LRELISNASDAIDKL | LRELISNAS |
| | | QEIIKKYSNHINYPI | IIKKYSNHI |
| | | IQEIIKKYSNHINYP | IIKKYSNHI |
| | | KIQEIIKKYSNHINY | IIKKYSNHI |
| | | YQMQKIMLSMGQEVK | YQMQKIMLS |
| | | NDLLYLIIHSLYSHK | YLIIHSLYS |
| | | TYQMQKIMLSMGQEV | YQMQKIMLS |
| | | DLLYLIIHSLYSHKE | YLIIHSLYS |
| | | VNDLLYLIIHSLYSH | YLIIHSLYS |
| | | EVNDLLYLIIHSLYS | LYLIIHSLY |
| | | EGLKLKAINKNETSN | LKLKAINKN |
| | | YEGLKLKAINKNETS | LKLKAINKN |
| | | EYEGLKLKAINKNET | LKLKAINKN |
| | | LLYLIIHSLYSHKEI | YLIIHSLYS |
| | | LKLKAINKNETSNEL | LKAINKNET |
| | | EIIKKYSNHINYPIY | IIKKYSNHI |
| | | IIKKYSNHINYPIYI | IIKKYSNHI |
| | | EEYNEFYKNTTFDYE | FYKNTTFDY |
| | HLA-DRB1*0404 | KEIFLRELISNASDA | FLRELISNA |
| | | EIFLRELISNASDAI | FLRELISNA |
| | | SHKEIFLRELISNAS | FLRELISNA |
| | | HKEIFLRELISNASD | FLRELISNA |
| | | YSHKEIFLRELISNA | IFLRELISN |
| | | KEIKPILELNPNNKI | ILELNPNNK |
| | | VKEIKPILELNPNNK | IKPILELNP |
| | | NDLLYLIIHSLYSHK | YLIIHSLYS |
| | | EIKPILELNPNNKIV | ILELNPNNK |
| | | IKPILELNPNNKIVQ | ILELNPNNK |
| | | DLLYLIIHSLYSHKE | YLIIHSLYS |
| | | LLYLIIHSLYSHKEI | YLIIHSLYS |
| | | IFLRELISNASDAID | FLRELISNA |
| | | VNDLLYLIIHSLYSH | YLIIHSLYS |
| | | KPILELNPNNKIVQN | ILELNPNNK |
| | | EVNDLLYLIIHSLYS | LLYLIIHSL |
| | | FLRELISNASDAIDK | FLRELISNA |
| | | FEEAMLTSGMPSKNP | MLTSGMPSK |
| | | EEAMLTSGMPSKNPG | MLTSGMPSK |
| | HLA-DRB1*0405 | DSNDPTYQMQKIMLS | DSNDPTYQM |
| | | NPNNKIVQNLKNLEP | NKIVQNLKN |
| | | PNNKIVQNLKNLEPE | VQNLKNLEP |
| | | PTYQMQKIMLSMGQE | YQMQKIMLS |
| | | DPTYQMQKIMLSMGQ | YQMQKIMLS |
| | | SNDPTYQMQKIMLSM | YQMQKIMLS |
| | | NDPTYQMQKIMLSMG | YQMQKIMLS |
| | | LLYLIIHSLYSHKEI | YLIIHSLYS |
| | | DLLYLIIHSLYSHKE | YLIIHSLYS |
| | | NNKIVQNLKNLEPEK | VQNLKNLEP |
| | | NKIVQNLKNLEPEKL | VQNLKNLEP |
| | | VNDLLYLIIHSLYSH | YLIIHSLYS |
| | | EVNDLLYLIIHSLYS | LYLIIHSLY |
| | | NDLLYLIIHSLYSHK | YLIIHSLYS |
| | | IFLRELISNASDAID | LRELISNAS |
| | | KIVQNLKNLEPEKLE | VQNLKNLEP |
| | | HKEIFLRELISNASD | LRELISNAS |
| | | KEIFLRELISNASDA | LRELISNAS |
| | | EIFLRELISNASDAI | LRELISNAS |
| | | EKLISLIRFKSSSVD | LIRFKSSSV |

| | | | |
|---|---|---|---|
| | | KLISLIRFKSSSVDG | IRFKSSSVD |
| | | LISLIRFKSSSVDGF | IRFKSSSVD |
| | | SHKEIFLRELISNAS | EIFLRELIS |
| | HLA-DRB1*0701 | NKILSKIKSSSVKKI | LSKIKSSSV |
| | | KILSKIKSSSVKKIL | LSKIKSSSV |
| | | LQQNKILSKIKSSSV | LQQNKILSK |
| | | QQNKILSKIKSSSVK | LSKIKSSSV |
| | | QNKILSKIKSSSVKK | LSKIKSSSV |
| | | LSKIKSSSVKKILSE | LSKIKSSSV |
| | | ILSKIKSSSVKKILS | LSKIKSSSV |
| | | DLYYPNTKPGVKLFI | YPNTKPGVK |
| | | YDLYYPNTKPGVKLF | YPNTKPGVK |
| | | ISLIRFKSSSVDGFV | LIRFKSSSV |
| | | PYDLYYPNTKPGVKL | YPNTKPGVK |
| | | APYDLYYPNTKPGVK | YYPNTKPGV |
| | | LISLIRFKSSSVDGF | LIRFKSSSV |
| | | KLISLIRFKSSSVDG | LIRFKSSSV |
| | | EKLISLIRFKSSSVD | LIRFKSSSV |
| | | EEYNEFYKNTTFDYE | FYKNTTFDY |
| | | EYNEFYKNTTFDYEN | FYKNTTFDY |
| | | QEIIKKYSNHINYPI | IIKKYSNHI |
| | | LYYPNTKPGVKLFIN | YPNTKPGVK |
| | | YNEFYKNTTFDYENP | FYKNTTFDY |
| | | NEFYKNTTFDYENPL | FYKNTTFDY |
| | | IFYFKQIALFIIFRL | YFKQIALFI |
| | | ILIFYFKQIALFIIF | YFKQIALFI |
| | | MILIFYFKQIALFII | YFKQIALFI |
| | | LIFYFKQIALFIIFR | YFKQIALFI |
| | | AEEYNEFYKNTTFDY | EFYKNTTFD |
| | | REKLISLIRFKSSSV | ISLIRFKSS |
| | HLA-DRB1*0802 | CYIIKKVKIKLKRKS | IIKKVKIKL |
| | | IIKKVKIKLKRKSCM | VKIKLKRKS |
| | | YIIKKVKIKLKRKSC | VKIKLKRKS |
| | | KKVKIKLKRKSCMKK | VKIKLKRKS |
| | HLA-DRB1*0901 | INYPIYIKYSEPIMK | YIKYSEPIM |
| | | YPIYIKYSEPIMKDG | YIKYSEPIM |
| | | NYPIYIKYSEPIMKD | YIKYSEPIM |
| | | PIYIKYSEPIMKDGK | YIKYSEPIM |
| | | HINYPIYIKYSEPIM | IYIKYSEPI |
| | | KILSKIKSSSVKKIL | IKSSSVKKI |
| | | YTNLFYVPSKAPYDL | YVPSKAPYD |
| | | TNLFYVPSKAPYDLY | YVPSKAPYD |
| | | ILSKIKSSSVKKILS | IKSSSVKKI |
| | | NKILSKIKSSSVKKI | LSKIKSSSV |
| | | LSKIKSSSVKKILSE | IKSSSVKKI |
| | | NLFYVPSKAPYDLYY | YVPSKAPYD |
| | | SKIKSSSVKKILSEL | IKSSSVKKI |
| | | EYTNLFYVPSKAPYD | FYVPSKAPY |
| | | MILIFYFKQIALFII | YFKQIALFI |
| | | ILIFYFKQIALFIIF | YFKQIALFI |
| | | LIFYFKQIALFIIFR | YFKQIALFI |
| | | IYIKYSEPIMKDGKQ | YIKYSEPIM |
| | | IFYFKQIALFIIFRL | YFKQIALFI |
| | | LFYVPSKAPYDLYYP | YVPSKAPYD |
| | | ISLIRFKSSSVDGFV | FKSSSVDGF |
| | | LISLIRFKSSSVDGF | RFKSSSVDG |

| | | | |
|---|---|---|---|
| | HLA-DRB1*1101 | EGNLEYTNLFYVPSK | YTNLFYVPS |
| | | AEGNLEYTNLFYVPS | LEYTNLFYV |
| | | GNLEYTNLFYVPSKA | YTNLFYVPS |
| | | LEYTNLFYVPSKAPY | YTNLFYVPS |
| | | NLEYTNLFYVPSKAP | YTNLFYVPS |
| | | EKLISLIRFKSSSVD | LISLIRFKS |
| | | NREKLISLIRFKSSS | LISLIRFKS |
| | | ENREKLISLIRFKSS | LISLIRFKS |
| | | REKLISLIRFKSSSV | LISLIRFKS |
| | | FENREKLISLIRFKS | EKLISLIRF |
| | | YTNLFYVPSKAPYDL | YTNLFYVPS |
| | | EYTNLFYVPSKAPYD | YTNLFYVPS |
| | | KKVKIKLKRKSCMKK | VKIKLKRKS |
| | HLA-DRB1*1302 | EGLKLKAINKNETSN | LKAINKNET |
| | | YEGLKLKAINKNETS | LKAINKNET |
| | | GLKLKAINKNETSNE | LKAINKNET |
| | | LKLKAINKNETSNEL | LKAINKNET |
| | | EYEGLKLKAINKNET | LKLKAINKN |
| | | IIKKVKIKLKRKSCM | VKIKLKRKS |
| | | QDLTNHLGVIAKSGT | LTNHLGVIA |
| | | IKPILELNPNNKIVQ | LELNPNNKI |
| | | KPILELNPNNKIVQN | LNPNNKIVQ |
| | | IKKVKIKLKRKSCMK | IKLKRKSCM |
| | | KDHIKEVNLSATLIK | IKEVNLSAT |
| | | DHIKEVNLSATLIKE | VNLSATLIK |
| | | PGVKLFINRIFITDS | LFINRIFIT |
| | | PILELNPNNKIVQNL | LNPNNKIVQ |
| | | LTNHLGVIAKSGTKE | LGVIAKSGT |
| | | NKIVQNLKNLEPEKL | IVQNLKNLE |
| | | TNHLGVIAKSGTKEF | LGVIAKSGT |
| | | LNPNNKIVQNLKNLE | LNPNNKIVQ |
| | | KPGVKLFINRIFITD | LFINRIFIT |
| | | REILQQNKILSKIKS | ILQQNKILS |
| | | NHLGVIAKSGTKEFI | LGVIAKSGT |
| | | KKVKIKLKRKSCMKK | IKLKRKSCM |
| | | GVKLFINRIFITDSE | LFINRIFIT |
| | | DLTNHLGVIAKSGTK | LGVIAKSGT |
| | | VKLFINRIFITDSEG | LFINRIFIT |
| | | IKEVNLSATLIKEPS | VNLSATLIK |
| | | HIKEVNLSATLIKEP | VNLSATLIK |
| | | ILELNPNNKIVQNLK | LNPNNKIVQ |
| | | CYIIKKVKIKLKRKS | IKKVKIKLK |
| | | NPNNKIVQNLKNLEP | IVQNLKNLE |
| | | PNNKIVQNLKNLEPE | IVQNLKNLE |
| | | KIVQNLKNLEPEKLE | VQNLKNLEP |
| | | EILQQNKILSKIKSS | NKILSKIKS |
| | | FENREKLISLIRFKS | EKLISLIRF |
| | | NNKIVQNLKNLEPEK | IVQNLKNLE |
| | | YIIKKVKIKLKRKSC | VKIKLKRKS |
| | | TKPGVKLFINRIFIT | VKLFINRIF |
| | | ILQQNKILSKIKSSS | NKILSKIKS |
| | | KVKIKLKRKSCMKKQ | IKLKRKSCM |
| | | FRLCYIIKKVKIKLK | YIIKKVKIK |
| | | PTYQMQKIMLSMGQE | YQMQKIMLS |
| | | RLCYIIKKVKIKLKR | IKKVKIKLK |
| | | VKIKLKRKSCMKKQF | IKLKRKSCM |

| | | | |
|---|---|---|---|
| | | DPTYQMKIMLSMGQ | YQMQKIMLS |
| | | NDPTYQMKIMLSMG | YQMQKIMLS |
| | | LELNPNNKIVQNLKN | LNPNNKIVQ |
| | | VSREILQQNKILSKI | ILQQNKILS |
| | | ENREKLISLIRFKSS | LISLIRFKS |
| | | LCYIIKKVKIKLKRK | IKKVKIKLK |
| | | NREKLISLIRFKSSS | LISLIRFKS |
| | | SREILQQNKILSKIK | ILQQNKILS |
| | | EKLISLIRFKSSSVD | LISLIRFKS |
| | | REKLISLIRFKSSSV | LISLIRFKS |
| | | IVQNLKNLEPEKLEK | IVQNLKNLE |
| | | KLKAINKNETSNELK | LKAINKNET |
| | | NKILSKIKSSSVKKI | NKILSKIKS |
| | | NVSREILQQNKILSK | ILQQNKILS |
| | | LGVIAKSGTKEFINN | LGVIAKSGT |
| | | HLGVIAKSGTKEFIN | LGVIAKSGT |
| | | LKAINKNETSNELKD | LKAINKNET |
| | | SLLPNYLRFIKGIID | LPNYLRFIK |
| | | LQQNKILSKIKSSSV | NKILSKIKS |
| | | VNDLLYLIIHSLYSH | LYLIIHSLY |
| | | YQMQKIMLSMGQEVK | YQMQKIMLS |
| | | KLFINRIFITDSEGS | LFINRIFIT |
| | | LNVSREILQQNKILS | LNVSREILQ |
| | | IFYFKQIALFIIFRL | FKQIALFII |
| | | NDLLYLIIHSLYSHK | LYLIIHSLY |
| | | FYFKQIALFIIFRLC | FKQIALFII |
| | | KEVNLSATLIKEPSA | VNLSATLIK |
| | | GSLLPNYLRFIKGII | LPNYLRFIK |
| | | EIKPILELNPNNKIV | LELNPNNKI |
| | | QQNKILSKIKSSSVK | NKILSKIKS |
| | | LKDHIKEVNLSATLI | IKEVNLSAT |
| | | KEIKPILELNPNNKI | ILELNPNNK |
| | | TYQMQKIMLSMGQEV | YQMQKIMLS |
| | | DLLYLIIHSLYSHKE | LYLIIHSLY |
| | | IKKYSNHINYPIYIK | YSNHINYPI |
| | | IFRLCYIIKKVKIKL | YIIKKVKIK |
| | | SNDPTYQMQKIMLSM | YQMQKIMLS |
| | | QEIIKKYSNHINYPI | IKKYSNHIN |
| | | IIKKYSNHINYPIYI | YSNHINYPI |
| | | KKYSNHINYPIYIKY | YSNHINYPI |
| | | LLPNYLRFIKGIIDC | YLRFIKGII |
| | | DSNDPTYQMQKIMLS | TYQMQKIML |
| | | DDKSILIKDNGIGMD | LIKDNGIGM |
| | | EIIKKYSNHINYPIY | YSNHINYPI |
| | | LPNYLRFIKGIIDCQ | YLRFIKGII |
| | | DEQDLTNHLGVIAKS | LTNHLGVIA |
| | | MDEQDLTNHLGVIAK | LTNHLGVIA |
| | | PEYEGLKLKAINKNE | LKLKAINKN |
| | | IPEYEGLKLKAINKN | YEGLKLKAI |
| | | FKQIALFIIFRLCYI | IALFIIFRL |
| | | LFINRIFITDSEGSL | LFINRIFIT |
| | | YFKQIALFIIFRLCY | IALFIIFRL |
| | | EILKDHIKEVNLSAT | LKDHIKEVN |
| | | DKSILIKDNGIGMDE | IKDNGIGMD |
| | | EQDLTNHLGVIAKSG | LTNHLGVIA |
| | | KSILIKDNGIGMDEQ | LIKDNGIGM |
| | | EVNDLLYLIIHSLYS | LYLIIHSLY |

| | | | |
|---|---|---|---|
| | | TEVNDLLYLIIHSLY | LLYLIIHSL |
| | | LLYLIIHSLYSHKEI | LIIHSLYSH |
| | | ILKDHIKEVNLSATL | IKEVNLSAT |
| | | LDEAILNLIPEYEGL | ILNLIPEYE |
| | | KILSKIKSSSVKKIL | IKSSSVKKI |
| | | ELDEAILNLIPEYEG | ILNLIPEYE |
| | | ILSKIKSSSVKKILS | IKSSSVKKI |
| | | IIFRLCYIIKKVKIK | CYIIKKVKI |
| | | LISLIRFKSSSVDGF | LISLIRFKS |
| | | YSNHINYPIYIKYSE | YSNHINYPI |
| | | PGKFINIINEFIEKD | INIINEFIE |
| | | ILIFYFKQIALFIIF | FKQIALFII |
| | | QNKILSKIKSSSVKK | NKILSKIKS |
| | | IFLRELISNASDAID | LRELISNAS |
| | | SEGSLLPNYLRFIKG | LPNYLRFIK |
| | | GMDEQDLTNHLGVIA | DLTNHLGVI |
| | | KNPGKFINIINEFIE | KFINIINEF |
| | | NPGKFINIINEFIEK | INIINEFIE |
| | | NEKFKNIALEPKIEI | FKNIALEPK |
| | | MILIFYFKQIALFII | IFYFKQIAL |
| | | DSEGSLLPNYLRFIK | EGSLLPNYL |
| | | EVNLSATLIKEPSAI | VNLSATLIK |
| | | EKFKNIALEPKIEIS | FKNIALEPK |
| | | KQIALFIIFRLCYII | IALFIIFRL |
| | | SGTKEFINNLKQDEK | FINNLKQDE |
| | | EGSLLPNYLRFIKGI | LPNYLRFIK |
| | | LYLIIHSLYSHKEIF | LIIHSLYSH |
| | | LIFYFKQIALFIIFR | FKQIALFII |
| | | KLISLIRFKSSSVDG | LISLIRFKS |
| | | VQNLKNLEPEKLEKI | LKNLEPEKL |
| | | EIFLRELISNASDAI | LRELISNAS |
| | | EEYNEFYKNTTFDYE | YNEFYKNTT |
| | | LSKIKSSSVKKILSE | IKSSSVKKI |
| | | SKIKSSSVKKILSEL | IKSSSVKKI |
| | | WKIQEIIKKYSNHIN | IQEIIKKYS |
| | | GKFINIINEFIEKDF | INIINEFIE |
| | | KYSNHINYPIYIKYS | YSNHINYPI |
| | | GTKEFINNLKQDEKK | INNLKQDEK |
| | | SILIKDNGIGMDEQD | LIKDNGIGM |
| | | DELDEAILNLIPEYE | LDEAILNLI |
| | | SDFENREKLISLIRF | FENREKLIS |
| | | TKEFINNLKQDEKKS | INNLKQDEK |
| | HLA-DRB1*1501 | KLISLIRFKSSSVDG | LIRFKSSSV |
| | | LISLIRFKSSSVDGF | LIRFKSSSV |
| | | ISLIRFKSSSVDGFV | LIRFKSSSV |
| | | REKLISLIRFKSSSV | ISLIRFKSS |
| | | SLIRFKSSSVDGFVS | LIRFKSSSV |
| | | NDLLYLIIHSLYSHK | LIIHSLYSH |
| | | LIRFKSSSVDGFVSF | LIRFKSSSV |
| | | DLLYLIIHSLYSHKE | LIIHSLYSH |
| | | LLYLIIHSLYSHKEI | LIIHSLYSH |
| | | LYLIIHSLYSHKEIF | LIIHSLYSH |
| | | VNDLLYLIIHSLYSH | YLIIHSLYS |
| | | YLIIHSLYSHKEIFL | LIIHSLYSH |
| | | SHKEIFLRELISNAS | FLRELISNA |
| | | KEIFLRELISNASDA | LRELISNAS |

| | | HKEIFLRELISNASD | LRELISNAS |
|---|---|---|---|
| | | EIFLRELISNASDAI | LRELISNAS |
| | | LIIHSLYSHKEIFLR | LIIHSLYSH |
| | | IALFIIFRLCYIIKK | FIIFRLCYI |
| | | ALFIIFRLCYIIKKV | FIIFRLCYI |
| | | IFLRELISNASDAID | LRELISNAS |
| | | QIALFIIFRLCYIIK | FIIFRLCYI |
| | | LFIIFRLCYIIKKVK | IFRLCYIIK |
| | | CYIIKKVKIKLKRKS | IKKVKIKLK |
| | | FIIFRLCYIIKKVKI | IFRLCYIIK |
| | | LCYIIKKVKIKLKRK | IKKVKIKLK |
| | | YIIKKVKIKLKRKSC | IKKVKIKLK |
| | | RLCYIIKKVKIKLKR | IKKVKIKLK |
| | | KKVKIKLKRKSCMKK | IKLKRKSCM |
| | | IKKVKIKLKRKSCMK | VKIKLKRKS |
| | | IIKKVKIKLKRKSCM | KKVKIKLKR |
| | | IIFRLCYIIKKVKIK | FRLCYIIKK |
| | | FRLCYIIKKVKIKLK | YIIKKVKIK |
| | | FKQIALFIIFRLCYI | LFIIFRLCY |
| | | KVKIKLKRKSCMKKQ | IKLKRKSCM |
| | | KQIALFIIFRLCYII | FIIFRLCYI |
| | | IFRLCYIIKKVKIKL | YIIKKVKIK |
| | | VKIKLKRKSCMKKQF | IKLKRKSCM |
| | HLA-DRB3*0101 | None | |
| | HLA-DRB4*0101 | KEIKPILELNPNNKI | ILELNPNNK |
| | | EIKPILELNPNNKIV | ILELNPNNK |
| | | IKPILELNPNNKIVQ | ILELNPNNK |
| | | KPILELNPNNKIVQN | ILELNPNNK |
| | | PILELNPNNKIVQNL | ILELNPNNK |
| | | VKEIKPILELNPNNK | VKEIKPILE |
| | | ILELNPNNKIVQNLK | ILELNPNNK |
| | | KKVKIKLKRKSCMKK | IKLKRKSCM |
| | | DLLYLIIHSLYSHKE | LIIHSLYSH |
| | | NKIVQNLKNLEPEKL | IVQNLKNLE |
| | | VNDLLYLIIHSLYSH | LYLIIHSLY |
| | | NDLLYLIIHSLYSHK | LIIHSLYSH |
| | | KVKIKLKRKSCMKKQ | IKLKRKSCM |
| | | EYEGLKLKAINKNET | LKLKAINKN |
| | | YEGLKLKAINKNETS | LKLKAINKN |
| | | EGLKLKAINKNETSN | LKLKAINKN |
| | | LLYLIIHSLYSHKEI | LIIHSLYSH |
| | | VKIKLKRKSCMKKQF | IKLKRKSCM |
| | | LYLIIHSLYSHKEIF | LIIHSLYSH |
| | | LSELEKLSKKNPEKF | LSELEKLSK |
| | | IPEYEGLKLKAINKN | YEGLKLKAI |
| | | KIVQNLKNLEPEKLE | LKNLEPEKL |
| | | PEYEGLKLKAINKNE | LKLKAINKN |
| | | IVQNLKNLEPEKLEK | LKNLEPEKL |
| | | PTYQMQKIMLSMGQE | YQMQKIMLS |
| | | NDPTYQMQKIMLSMG | YQMQKIMLS |
| | | DPTYQMQKIMLSMGQ | YQMQKIMLS |
| | | VPSKAPYDLYYPNTK | VPSKAPYDL |
| | | SNDPTYQMQKIMLSM | YQMQKIMLS |
| | | SELEKLSKKNPEKFS | LSKKNPEKF |

| | HLA-DRB5*0101 | APYDLYYPNTKPGVK | YDLYYPNTK |
|---|---|---|---|
| | | PYDLYYPNTKPGVKL | YPNTKPGVK |
| | | YDLYYPNTKPGVKLF | YPNTKPGVK |
| | | DLYYPNTKPGVKLFI | YPNTKPGVK |
| | | LYYPNTKPGVKLFIN | YPNTKPGVK |
| | | LEYANKWKIQEIIKK | YANKWKIQE |
| | | YANKWKIQEIIKKYS | WKIQEIIKK |
| | | EYANKWKIQEIIKKY | WKIQEIIKK |
| | | ANKWKIQEIIKKYSN | WKIQEIIKK |
| | | NKWKIQEIIKKYSNH | WKIQEIIKK |
| | | YYPNTKPGVKLFINR | YPNTKPGVK |
| | | YPNTKPGVKLFINRI | YPNTKPGVK |
| | | KKIEEEFKDTLTKVK | EEFKDTLTK |
| | | KIEEEFKDTLTKVKE | FKDTLTKVK |
| | | IEEEFKDTLTKVKEI | FKDTLTKVK |
| | | EEEFKDTLTKVKEIL | FKDTLTKVK |
| | | EEFKDTLTKVKEILK | FKDTLTKVK |
| CAA44492.1\| Outer surface protein A [Borrelia burgdorferi]<br><br>Seq9 | HLA-DRB1*0101 | KKYLLGIGLILALIA | LLGIGLILA |
| | | KYLLGIGLILALIAC | LLGIGLILA |
| | | MKKYLLGIGLILALI | LLGIGLILA |
| | | KTGKWDSKTSTLTIS | WDSKTSTLT |
| | | TGKWDSKTSTLTISV | WDSKTSTLT |
| | | TKKTGKWDSKTSTLT | TGKWDSKTS |
| | | KKTGKWDSKTSTLTI | WDSKTSTLT |
| | | GKWDSKTSTLTISVN | WDSKTSTLT |
| | | LKDFTLEGTLAADGK | FTLEGTLAA |
| | | VLKDFTLEGTLAADG | FTLEGTLAA |
| | | KDFTLEGTLAADGKT | FTLEGTLAA |
| | | SVSVDLPGGMTELVS | LPGGMTELV |
| | | YLLGIGLILALIACK | IGLILALIA |
| | | NSVSVDLPGGMTELV | SVDLPGGMT |
| | | SVDLPGGMTELVSKE | LPGGMTELV |
| | | VSVDLPGGMTELVSK | LPGGMTELV |
| | | VDLPGGMTELVSKEK | LPGGMTELV |
| | | LLGIGLILALIACKQ | IGLILALIA |
| | | KEVLKDFTLEGTLAA | LKDFTLEGT |
| | | EVLKDFTLEGTLAAD | FTLEGTLAA |
| | | TSEKTIVRANGTRLE | IVRANGTRL |
| | | SEKTIVRANGTRLEY | IVRANGTRL |
| | | TTLKVTEGTVVLSKN | VTEGTVVLS |
| | | KTTLKVTEGTVVLSK | VTEGTVVLS |
| | | EKTIVRANGTRLEYT | IVRANGTRL |
| | | GKTTLKVTEGTVVLS | LKVTEGTVV |
| | | KTIVRANGTRLEYTD | IVRANGTRL |
| | | KNILKSGEITVALDD | LKSGEITVA |
| | | LSKNILKSGEITVAL | LKSGEITVA |
| | | SKNILKSGEITVALD | LKSGEITVA |
| | | NILKSGEITVALDDS | LKSGEITVA |
| | | DFTLEGTLAADGKTT | FTLEGTLAA |
| | | FTLEGTLAADGKTTL | FTLEGTLAA |
| | | TLKVTEGTVVLSKNI | VTEGTVVLS |
| | | LGIGLILALIACKQN | IGLILALIA |
| | | VLSKNILKSGEITVA | KNILKSGEI |
| | | KEDAKTLVSKKVTLK | AKTLVSKKV |
| | | EDAKTLVSKKVTLKD | AKTLVSKKV |

| | | | |
|---|---|---|---|
| | | LKVTEGTVVLSKNIL | VTEGTVVLS |
| | | TIVRANGTRLEYTDI | VRANGTRLE |
| | | EIFKEDAKTLVSKKV | FKEDAKTLV |
| | | SAGTNLEGKAVEITT | LEGKAVEIT |
| | | DSAGTNLEGKAVEIT | GTNLEGKAV |
| | | KWDSKTSTLTISVNS | WDSKTSTLT |
| | | WDSKTSTLTISVNSQ | WDSKTSTLT |
| | | IFKEDAKTLVSKKVT | AKTLVSKKV |
| | | GTNLEGKAVEITTLK | LEGKAVEIT |
| | | AGTNLEGKAVEITTL | LEGKAVEIT |
| | | DLPGGMTELVSKEKD | LPGGMTELV |
| | | FKEDAKTLVSKKVTL | AKTLVSKKV |
| | | DGKTTLKVTEGTVVL | LKVTEGTVV |
| | | LPGGMTELVSKEKDK | LPGGMTELV |
| | | ITVQKYDSAGTNLEG | VQKYDSAGT |
| | | TITVQKYDSAGTNLE | VQKYDSAGT |
| | | TNLEGKAVEITTLKE | LEGKAVEIT |
| | | NLVFTKEDTITVQKY | FTKEDTITV |
| | | AKTLVSKKVTLKDKS | AKTLVSKKV |
| | | DAKTLVSKKVTLKDK | AKTLVSKKV |
| | | KNLVFTKEDTITVQK | FTKEDTITV |
| | | GIGLILALIACKQNV | ILALIACKQ |
| | | ETSEKTIVRANGTRL | KTIVRANGT |
| | | TKNLVFTKEDTITVQ | FTKEDTITV |
| | | IGLILALIACKQNVS | ILALIACKQ |
| | | KTKNLVFTKEDTITV | VFTKEDTIT |
| | | IVRANGTRLEYTDIK | IVRANGTRL |
| | | ADGKTTLKVTEGTVV | ADGKTTLKV |
| | | LVFTKEDTITVQKYD | FTKEDTITV |
| | | LKSGEITVALDDSDT | LKSGEITVA |
| | | ILKSGEITVALDDSD | LKSGEITVA |
| | | DTITVQKYDSAGTNL | VQKYDSAGT |
| | | EDTITVQKYDSAGTN | VQKYDSAGT |
| | | KEDTITVQKYDSAGT | ITVQKYDSA |
| | | GLILALIACKQNVST | ILALIACKQ |
| | | LEGTLAADGKTTLKV | LEGTLAADG |
| | | TISVNSQKTKNLVFT | VNSQKTKNL |
| | | LEYTDIKSDGSGKAK | IKSDGSGKA |
| | | RLEYTDIKSDGSGKA | YTDIKSDGS |
| | | TLTISVNSQKTKNLV | VNSQKTKNL |
| | | STLTISVNSQKTKNL | TISVNSQKT |
| | | ISVNSQKTKNLVFTK | VNSQKTKNL |
| | | TVQKYDSAGTNLEGK | VQKYDSAGT |
| | | VQKYDSAGTNLEGKA | VQKYDSAGT |
| | | VTEGTVVLSKNILKS | VTEGTVVLS |
| | | VALDDSDTTQATKKT | LDDSDTTQA |
| | | YTDIKSDGSGKAKEV | IKSDGSGKA |
| | | QKYDSAGTNLEGKAV | YDSAGTNLE |
| | | TLEGTLAADGKTTLK | LEGTLAADG |
| | | LTISVNSQKTKNLVF | VNSQKTKNL |
| | | KVTEGTVVLSKNILK | VTEGTVVLS |
| | | EYTDIKSDGSGKAKE | IKSDGSGKA |
| | | LILALIACKQNVSTL | IACKQNVST |
| | | FEIFKEDAKTLVSKK | IFKEDAKTL |
| | | KFEIFKEDAKTLVSK | IFKEDAKTL |
| | | VRANGTRLEYTDIKS | VRANGTRLE |
| | | NLEGKAVEITTLKEL | LEGKAVEIT |

| | HLA-DRB1*0301 | None | |
|---|---|---|---|
| | HLA-DRB1*0401 | KEVLKDFTLEGTLAA | VLKDFTLEG |
| | | EVLKDFTLEGTLAAD | FTLEGTLAA |
| | | VLKDFTLEGTLAADG | FTLEGTLAA |
| | | LKDFTLEGTLAADGK | FTLEGTLAA |
| | | KDFTLEGTLAADGKT | FTLEGTLAA |
| | | KNLVFTKEDTITVQK | FTKEDTITV |
| | | NLVFTKEDTITVQKY | FTKEDTITV |
| | | LVFTKEDTITVQKYD | FTKEDTITV |
| | | DFTLEGTLAADGKTT | FTLEGTLAA |
| | | LEYTDIKSDGSGKAK | IKSDGSGKA |
| | | TKNLVFTKEDTITVQ | FTKEDTITV |
| | | FTLEGTLAADGKTTL | FTLEGTLAA |
| | | RLEYTDIKSDGSGKA | YTDIKSDGS |
| | | KTKNLVFTKEDTITV | KTKNLVFTK |
| | | AKEVLKDFTLEGTLA | VLKDFTLEG |
| | HLA-DRB1*0404 | KEVLKDFTLEGTLAA | VLKDFTLEG |
| | | GKAKEVLKDFTLEGT | VLKDFTLEG |
| | | AKEVLKDFTLEGTLA | VLKDFTLEG |
| | | KAKEVLKDFTLEGTL | VLKDFTLEG |
| | | SGKAKEVLKDFTLEG | AKEVLKDFT |
| | HLA-DRB1*0405 | TITVQKYDSAGTNLE | VQKYDSAGT |
| | | ITVQKYDSAGTNLEG | YDSAGTNLE |
| | | TVQKYDSAGTNLEGK | YDSAGTNLE |
| | | VQKYDSAGTNLEGKA | YDSAGTNLE |
| | | QKYDSAGTNLEGKAV | YDSAGTNLE |
| | HLA-DRB1*0701 | DGKTTLKVTEGTVVL | LKVTEGTVV |
| | | GKTTLKVTEGTVVLS | LKVTEGTVV |
| | | KTTLKVTEGTVVLSK | LKVTEGTVV |
| | | ADGKTTLKVTEGTVV | TLKVTEGTV |
| | | TTLKVTEGTVVLSKN | LKVTEGTVV |
| | HLA-DRB1*0802 | None | |
| | HLA-DRB1*0901 | None | |
| | HLA-DRB1*1101 | None | |
| | HLA-DRB1*1302 | TEGTVVLSKNILKSG | TVVLSKNIL |
| | | EGTVVLSKNILKSGE | TVVLSKNIL |
| | | GTVVLSKNILKSGEI | VVLSKNILK |
| | | VTEGTVVLSKNILKS | TVVLSKNIL |
| | | TVVLSKNILKSGEIT | TVVLSKNIL |
| | | SEKTIVRANGTRLEY | IVRANGTRL |
| | | TSEKTIVRANGTRLE | IVRANGTRL |
| | | EKTIVRANGTRLEYT | IVRANGTRL |
| | | KTIVRANGTRLEYTD | IVRANGTRL |
| | | TLTISVNSQKTKNLV | VNSQKTKNL |
| | | STLTISVNSQKTKNL | ISVNSQKTK |
| | | LTISVNSQKTKNLVF | VNSQKTKNL |
| | | KVTEGTVVLSKNILK | TVVLSKNIL |
| | | TISVNSQKTKNLVFT | VNSQKTKNL |
| | | LKVTEGTVVLSKNIL | LKVTEGTVV |
| | | TIVRANGTRLEYTDI | VRANGTRLE |
| | | ISVNSQKTKNLVFTK | VNSQKTKNL |

| | | | |
|---|---|---|---|
| | | VVLSKNILKSGEITV | VVLSKNILK |
| | | ETSEKTIVRANGTRL | KTIVRANGT |
| | | EDAKTLVSKKVTLKD | LVSKKVTLK |
| | | DAKTLVSKKVTLKDK | LVSKKVTLK |
| | HLA-DRB1*1501 | TEGTVVLSKNILKSG | VLSKNILKS |
| | | EGTVVLSKNILKSGE | VLSKNILKS |
| | HLA-DRB3*0101 | None | |
| | HLA-DRB4*0101 | None | |
| | HLA-DRB5*0101 | KDKDGKYSLEATVDK | KDKDGKYSL |
| | | DKDGKYSLEATVDKL | YSLEATVDK |
| | | KDGKYSLEATVDKLE | YSLEATVDK |
| | | DGKYSLEATVDKLEL | YSLEATVDK |
| | | GKYSLEATVDKLELK | YSLEATVDK |
| | | | |
| \|BAA22351.1\| Outer surface protein B [Borrelia garinii]<br><br>Seq10 | HLA-DRB1*0101 | KKYLLGFALVLALIA | LLGFALVLA |
| | | ITVQNYDTAGTKLAG | YDTAGTKLA |
| | | TITVQNYDTAGTKLA | VQNYDTAGT |
| | | QNYDTAGTKLAGTAT | YDTAGTKLA |
| | | KYLLGFALVLALIAC | LLGFALVLA |
| | | MKKYLLGFALVLALI | LLGFALVLA |
| | | TVQNYDTAGTKLAGT | YDTAGTKLA |
| | | VQNYDTAGTKLAGTA | YDTAGTKLA |
| | | ATEIKDLEALKAALK | IKDLEALKA |
| | | TATEIKDLEALKAAL | IKDLEALKA |
| | | YLLGFALVLALIACG | FALVLALIA |
| | | GTATEIKDLEALKAA | IKDLEALKA |
| | | AGTATEIKDLEALKA | AGTATEIKD |
| | | LLGFALVLALIACGQ | FALVLALIA |
| | | LGFALVLALIACGQK | FALVLALIA |
| | | YDTAGTKLAGTATEI | YDTAGTKLA |
| | | NYDTAGTKLAGTATE | YDTAGTKLA |
| | | EGVKSDQSKVTMSIT | VKSDQSKVT |
| | | LEGVKSDQSKVTMSI | VKSDQSKVT |
| | | GKLEGVKSDQSKVTM | VKSDQSKVT |
| | | GGKLEGVKSDQSKVT | LEGVKSDQS |
| | | KLEGVKSDQSKVTMS | VKSDQSKVT |
| | | DGTITVQNYDTAGTK | VQNYDTAGT |
| | | GTITVQNYDTAGTKL | VQNYDTAGT |
| | | SKKDLPLVTEDTVKL | DLPLVTEDT |
| | | GFALVLALIACGQKG | LVLALIACG |
| | | TDGTITVQNYDTAGT | TITVQNYDT |
| | | KKDLPLVTEDTVKLF | LVTEDTVKL |
| | | FALVLALIACGQKGA | LVLALIACG |
| | | MLEGNLVGGKTSVEI | LVGGKTSVE |
| | | KDLPLVTEDTVKLFN | LVTEDTVKL |
| | | DLPLVTEDTVKLFND | LVTEDTVKL |
| | | LEGNLVGGKTSVEIK | VGGKTSVEI |
| | | EGNLVGGKTSVEIKE | VGGKTSVEI |
| | | GNLVGGKTSVEIKEG | VGGKTSVEI |
| | | LPLVTEDTVKLFNDT | LVTEDTVKL |
| | | NLVGGKTSVEIKEGT | VGGKTSVEI |
| | | ETLKNGIMLEGNLVG | LKNGIMLEG |
| | | YKTGKLSTKKITRTN | LSTKKITRT |

| | | TYKTGKLSTKKITRT | TGKLSTKKI |
|---|---|---|---|
| | | TGKLSTKKITRTNGT | LSTKKITRT |
| | | VETLKNGIMLEGNLV | LKNGIMLEG |
| | | KKEIEKAGTVKLFLD | IEKAGTVKL |
| | | LKKEIEKAGTVKLFL | IEKAGTVKL |
| | | KEIEKAGTVKLFLDD | IEKAGTVKL |
| | | KDFVFLTDGTITVQN | FLTDGTITV |
| | | VTLKKEIEKAGTVKL | LKKEIEKAG |
| | | TLKKEIEKAGTVKLF | IEKAGTVKL |
| | | KTGKLSTKKITRTNG | LSTKKITRT |
| | | GKLSTKKITRTNGTT | LSTKKITRT |
| | | DTAGTKLAGTATEIK | GTKLAGTAT |
| | | GVKSDQSKVTMSITD | VKSDQSKVT |
| | | VKSDQSKVTMSITDD | VKSDQSKVT |
| | | DFVFLTDGTITVQNY | FLTDGTITV |
| | | TAGTKLAGTATEIKD | LAGTATEIK |
| | | IKDFVFLTDGTITVQ | FLTDGTITV |
| | | DGKYELRATVDTVEL | YELRATVDT |
| | | FVFLTDGTITVQNYD | FLTDGTITV |
| | | GKYELRATVDTVELK | YELRATVDT |
| | | ALVLALIACGQKGAE | LVLALIACG |
| | | NKDGKYELRATVDTV | YELRATVDT |
| | | KNKDGKYELRATVDT | DGKYELRAT |
| | | KDGKYELRATVDTVE | YELRATVDT |
| | | KAVETLKNGIMLEGN | LKNGIMLEG |
| | | AVETLKNGIMLEGNL | LKNGIMLEG |
| | | ASKVFKKQGSLTEET | FKKQGSLTE |
| | | TLKNGIMLEGNLVGG | LKNGIMLEG |
| | | SKVFKKQGSLTEETE | FKKQGSLTE |
| | | KIKDFVFLTDGTITV | FVFLTDGTI |
| | | LKNGIMLEGNLVGGK | LKNGIMLEG |
| | | AGTKLAGTATEIKDL | LAGTATEIK |
| | | GTKLAGTATEIKDLE | LAGTATEIK |
| | | TKAVETLKNGIMLEG | VETLKNGIM |
| | | KTAVWSDTSNTLTVS | WSDTSNTLT |
| | | KVFKKQGSLTEETEE | FKKQGSLTE |
| | | VASKVFKKQGSLTEE | FKKQGSLTE |
| | | KVASKVFKKQGSLTE | VFKKQGSLT |
| | | VGGKTSVEIKEGTVT | VGGKTSVEI |
| | | TAVWSDTSNTLTVSA | VWSDTSNTL |
| | | LVLALIACGQKGAEP | LVLALIACG |
| | | TKKTAVWSDTSNTLT | VWSDTSNTL |
| | | KKTAVWSDTSNTLTV | WSDTSNTLT |
| | | LVGGKTSVEIKEGTV | VGGKTSVEI |
| | | KNGIMLEGNLVGGKT | IMLEGNLVG |
| | | ETYKTGKLSTKKITR | YKTGKLSTK |
| | | LVTEDTVKLFNDTKI | LVTEDTVKL |
| | | EETYKTGKLSTKKIT | YKTGKLSTK |
| | | TEETYKTGKLSTKKI | YKTGKLSTK |
| | | LSTKKITRTNGTTLE | LSTKKITRT |
| | | PLVTEDTVKLFNDTK | LVTEDTVKL |
| | | VFLTDGTITVQNYDT | FLTDGTITV |
| | | KLSTKKITRTNGTTL | LSTKKITRT |
| | | VETYDSSNTKVASKV | YDSSNTKVA |
| | | YELRATVDTVELKGV | YELRATVDT |
| | | TITVETYDSSNTKVA | VETYDSSNT |
| | | ITVETYDSSNTKVAS | YDSSNTKVA |

|  |  | AVWSDTSNTLTVSAD | WSDTSNTLT |
|---|---|---|---|
|  |  | ETYDSSNTKVASKVF | YDSSNTKVA |
|  |  | IMLEGNLVGGKTSVE | IMLEGNLVG |
|  |  | EIEKAGTVKLFLDDT | IEKAGTVKL |
|  |  | NGIMLEGNLVGGKTS | IMLEGNLVG |
|  |  | IEKAGTVKLFLDDTS | IEKAGTVKL |
|  |  | FLTDGTITVQNYDTA | FLTDGTITV |
|  |  | KLFLDDTSSGSTKKT | FLDDTSSGS |
|  |  | KYELRATVDTVELKG | YELRATVDT |
|  | HLA-DRB1*0301 | DTVKLFNDTKIFISK | LFNDTKIFI |
|  |  | EDTVKLFNDTKIFIS | LFNDTKIFI |
|  |  | TVKLFNDTKIFISKE | LFNDTKIFI |
|  |  | TEDTVKLFNDTKIFI | KLFNDTKIF |
|  |  | VKLFNDTKIFISKEK | LFNDTKIFI |
|  | HLA-DRB1*0401 | SKVFKKQGSLTEETE | FKKQGSLTE |
|  |  | VASKVFKKQGSLTEE | FKKQGSLTE |
|  |  | ASKVFKKQGSLTEET | FKKQGSLTE |
|  |  | KVASKVFKKQGSLTE | VFKKQGSLT |
|  |  | KVFKKQGSLTEETEE | FKKQGSLTE |
|  |  | TAVWSDTSNTLTVSA | VWSDTSNTL |
|  | HLA-DRB1*0404 | TAVWSDTSNTLTVSA | WSDTSNTLT |
|  |  | AVWSDTSNTLTVSAD | TSNTLTVSA |
|  | HLA-DRB1*0405 | TLEYSDMTNDENATK | YSDMTNDEN |
|  |  | GTTLEYSDMTNDENA | YSDMTNDEN |
|  |  | NGTTLEYSDMTNDEN | EYSDMTNDE |
|  |  | TTLEYSDMTNDENAT | YSDMTNDEN |
|  |  | LEYSDMTNDENATKA | YSDMTNDEN |
|  | HLA-DRB1*0701 | ETYDSSNTKVASKVF | YDSSNTKVA |
|  |  | TYDSSNTKVASKVFK | NTKVASKVF |
|  |  | YDSSNTKVASKVFKK | NTKVASKVF |
|  | HLA-DRB1*0802 | None |  |
|  | HLA-DRB1*0901 | MKKYLLGFALVLALI | LGFALVLAL |
|  |  | KKYLLGFALVLALIA | LGFALVLAL |
|  | HLA-DRB1*1101 | None |  |
|  | HLA-DRB1*1302 | TKAVETLKNGIMLEG | VETLKNGIM |
|  |  | KAVETLKNGIMLEGN | LKNGIMLEG |
|  |  | VETLKNGIMLEGNLV | LKNGIMLEG |
|  |  | AVETLKNGIMLEGNL | LKNGIMLEG |
|  |  | ETLKNGIMLEGNLVG | LKNGIMLEG |
|  |  | LSTKKITRTNGTTLE | KKITRTNGT |
|  |  | EDTVKLFNDTKIFIS | VKLFNDTKI |
|  |  | DTVKLFNDTKIFISK | VKLFNDTKI |
|  |  | TYKTGKLSTKKITRT | GKLSTKKIT |
|  |  | YKTGKLSTKKITRTN | LSTKKITRT |
|  |  | TGKLSTKKITRTNGT | LSTKKITRT |
|  |  | GKLSTKKITRTNGTT | LSTKKITRT |
|  |  | STKKITRTNGTTLEY | ITRTNGTTL |

| | | KTGKLSTKKITRTNG | LSTKKITRT |
|---|---|---|---|
| | | KLSTKKITRTNGTTL | KKITRTNGT |
| | | NGIMLEGNLVGGKTS | EGNLVGGKT |
| | | VKLFNDTKIFISKEK | LFNDTKIFI |
| | | IMLEGNLVGGKTSVE | EGNLVGGKT |
| | HLA-DRB1*1501 | MKKYLLGFALVLALI | LLGFALVLA |
| | | KKYLLGFALVLALIA | LLGFALVLA |
| | | KYLLGFALVLALIAC | LLGFALVLA |
| | HLA-DRB3*0101 | None | |
| | HLA-DRB4*0101 | None | |
| | HLA-DRB5*0101 | None | |
| | | | |
| AAM22469.1\| Outer surface protein C [Borrelia afzelii]<br><br>Seq11 | HLA-DRB1*0101 | TNSVKELTSPVVAES | VKELTSPVV |
| | | NSVKELTSPVVAESP | VKELTSPVV |
| | | ALTNSVKELTSPVVA | VKELTSPVV |
| | | LTNSVKELTSPVVAE | VKELTSPVV |
| | | EALTNSVKELTSPVV | LTNSVKELT |
| | | EGLVKAAQEALTNSV | VKAAQEALT |
| | | SVKELTSPVVAESPK | VKELTSPVV |
| | | VKELTSPVVAESPKK | VKELTSPVV |
| | | GLVKAAQEALTNSVK | VKAAQEALT |
| | | VEGLVKAAQEALTNS | VKAAQEALT |
| | | LAGVHEISTLITEKL | VHEISTLIT |
| | | AGVHEISTLITEKLS | VHEISTLIT |
| | | SVEGLVKAAQEALTN | VKAAQEALT |
| | | KSVEGLVKAAQEALT | LVKAAQEAL |
| | | HEISTLITEKLSKLK | ISTLITEKL |
| | | VHEISTLITEKLSKL | ISTLITEKL |
| | | MKKNTLSAILMTLFL | KNTLSAILM |
| | | NTLSAILMTLFLFIS | LSAILMTLF |
| | | GVHEISTLITEKLSK | ISTLITEKL |
| | | ESLLAGVHEISTLIT | LLAGVHEIS |
| | | KNTLSAILMTLFLFI | LSAILMTLF |
| | | SLLAGVHEISTLITE | VHEISTLIT |
| | | LLAGVHEISTLITEK | VHEISTLIT |
| | | TLSAILMTLFLFISC | LMTLFLFIS |
| | | KELTSPVVAESPKKP | LTSPVVAES |
| | | LSAILMTLFLFISCN | LMTLFLFIS |
| | | LVKAAQEALTNSVKE | VKAAQEALT |
| | | SAILMTLFLFISCNN | LMTLFLFIS |
| | | ISTLITEKLSKLKNS | ISTLITEKL |
| | | EISTLITEKLSKLKN | ISTLITEKL |
| | | VKAAQEALTNSVKEL | VKAAQEALT |
| | | KKNTLSAILMTLFLF | LSAILMTLF |
| | | VLAVKEVETLVSSID | VKEVETLVS |
| | | LAVKEVETLVSSIDE | VKEVETLVS |
| | | AFVLAVKEVETLVSS | VKEVETLVS |
| | | AILMTLFLFISCNNS | LMTLFLFIS |
| | | NAFVLAVKEVETLVS | LAVKEVETL |
| | | FVLAVKEVETLVSSI | VKEVETLVS |
| | | FKSVEGLVKAAQEAL | FKSVEGLVK |
| | | EEFTNKLRVSHADLG | FTNKLRVSH |
| | | VKEVETLVSSIDELA | VKEVETLVS |
| | | EFTNKLRVSHADLGK | LRVSHADLG |

| | | | |
|---|---|---|---|
| | | GKLFKSVEGLVKAAQ | FKSVEGLVK |
| | | NNSGKGGDSASTNPA | GKGGDSAST |
| | | KLFKSVEGLVKAAQE | FKSVEGLVK |
| | | NSGKGGDSASTNPAD | GKGGDSAST |
| | HLA-DRB1*0301 | None | |
| | HLA-DRB1*0401 | None | |
| | HLA-DRB1*0404 | MTLFLFISCNNSGKG | LFISCNNSG |
| | | TLFLFISCNNSGKGG | LFISCNNSG |
| | | ILMTLFLFISCNNSG | FLFISCNNS |
| | | KKAILKTNADKTKGA | ILKTNADKT |
| | | LMTLFLFISCNNSGK | LFISCNNSG |
| | | AKKAILKTNADKTKG | ILKTNADKT |
| | | KAILKTNADKTKGAE | ILKTNADKT |
| | | LFLFISCNNSGKGGD | LFISCNNSG |
| | | DAKKAILKTNADKTK | ILKTNADKT |
| | | DDAKKAILKTNADKT | AILKTNADK |
| | HLA-DRB1*0405 | LAVKEVETLVSSIDE | VKEVETLVS |
| | | VLAVKEVETLVSSID | VKEVETLVS |
| | | VKEVETLVSSIDELA | VKEVETLVS |
| | | AVKEVETLVSSIDEL | VKEVETLVS |
| | | NAFVLAVKEVETLVS | FVLAVKEVE |
| | | TNSVKELTSPVVAES | VKELTSPVV |
| | | LTNSVKELTSPVVAE | VKELTSPVV |
| | | NSVKELTSPVVAESP | VKELTSPVV |
| | | AFVLAVKEVETLVSS | VKEVETLVS |
| | | FVLAVKEVETLVSSI | VKEVETLVS |
| | HLA-DRB1*0701 | ALTNSVKELTSPVVA | VKELTSPVV |
| | | EALTNSVKELTSPVV | SVKELTSPV |
| | | LTNSVKELTSPVVAE | VKELTSPVV |
| | | TNSVKELTSPVVAES | VKELTSPVV |
| | | NSVKELTSPVVAESP | VKELTSPVV |
| | | SVKELTSPVVAESPK | VKELTSPVV |
| | | VKELTSPVVAESPKK | VKELTSPVV |
| | | EISKKITDSNAFVLA | ITDSNAFVL |
| | | TEISKKITDSNAFVL | SKKITDSNA |
| | | ISKKITDSNAFVLAV | ITDSNAFVL |
| | | SKKITDSNAFVLAVK | ITDSNAFVL |
| | | KKITDSNAFVLAVKE | ITDSNAFVL |
| | | KITDSNAFVLAVKEV | ITDSNAFVL |
| | | ITDSNAFVLAVKEVE | ITDSNAFVL |
| | HLA-DRB1*0802 | None | |
| | HLA-DRB1*0901 | EGLVKAAQEALTNSV | LVKAAQEAL |
| | | KSVEGLVKAAQEALT | LVKAAQEAL |
| | | SVEGLVKAAQEALTN | LVKAAQEAL |
| | | VEGLVKAAQEALTNS | LVKAAQEAL |
| | | FKSVEGLVKAAQEAL | VEGLVKAAQ |
| | HLA-DRB1*1101 | KKCSEEFTNKLRVSH | EFTNKLRVS |
| | | KCSEEFTNKLRVSHA | FTNKLRVSH |
| | | CSEEFTNKLRVSHAD | FTNKLRVSH |
| | | SEEFTNKLRVSHADL | FTNKLRVSH |
| | | EEFTNKLRVSHADLG | FTNKLRVSH |
| | | EFTNKLRVSHADLGK | FTNKLRVSH |

| | | FTNKLRVSHADLGKQ | FTNKLRVSH |
|---|---|---|---|
| | HLA-DRB1*1302 | EALTNSVKELTSPVV | LTNSVKELT |
| | | QEALTNSVKELTSPV | LTNSVKELT |
| | | AAQEALTNSVKELTS | LTNSVKELT |
| | | AQEALTNSVKELTSP | LTNSVKELT |
| | | ALTNSVKELTSPVVA | LTNSVKELT |
| | | KAAQEALTNSVKELT | ALTNSVKEL |
| | | LTNSVKELTSPVVAE | LTNSVKELT |
| | | MKKNTLSAILMTLFL | MKKNTLSAI |
| | | LAGVHEISTLITEKL | VHEISTLIT |
| | | AGVHEISTLITEKLS | ISTLITEKL |
| | HLA-DRB1*1501 | ALTNSVKELTSPVVA | VKELTSPVV |
| | | EALTNSVKELTSPVV | NSVKELTSP |
| | | LTNSVKELTSPVVAE | VKELTSPVV |
| | | TNSVKELTSPVVAES | VKELTSPVV |
| | | NSVKELTSPVVAESP | VKELTSPVV |
| | | VKELTSPVVAESPKK | VKELTSPVV |
| | HLA-DRB3*0101 | None | |
| | HLA-DRB4*0101 | None | |
| | HLA-DRB5*0101 | None | |
| | | | |
| AAC62927.1\| OspE-related lipoprotein [Borrelia garinii]  Seq12 | HLA-DRB1*0101 | NNFVKAMTNVGSFKT | VKAMTNVGS |
| | | NFVKAMTNVGSFKTS | VKAMTNVGS |
| | | AEVNNFVKAMTNVGS | FVKAMTNVG |
| | | VNNFVKAMTNVGSFK | VKAMTNVGS |
| | | EVNNFVKAMTNVGSF | VKAMTNVGS |
| | | FVKAMTNVGSFKTSL | MTNVGSFKT |
| | | VKAMTNVGSFKTSLY | MTNVGSFKT |
| | | NGDWSNLGTLVIRKE | WSNLGTLVI |
| | | GTINGQLRGHSATFF | NGQLRGHSA |
| | | GDWSNLGTLVIRKEQ | WSNLGTLVI |
| | | NNGDWSNLGTLVIRK | WSNLGTLVI |
| | | TINGQLRGHSATFFC | LRGHSATFF |
| | | DNNGDWSNLGTLVIR | WSNLGTLVI |
| | | KDNNGDWSNLGTLVI | DWSNLGTLV |
| | | INGQLRGHSATFFCI | LRGHSATFF |
| | | NGQLRGHSATFFCIE | LRGHSATFF |
| | | LSDQGGLSGQASSDT | QGGLSGQAS |
| | | SDQGGLSGQASSDTI | LSGQASSDT |
| | | GQLRGHSATFFCIEE | LRGHSATFF |
| | | DQGGLSGQASSDTIK | LSGQASSDT |
| | | QGGLSGQASSDTIKF | LSGQASSDT |
| | | GGLSGQASSDTIKFS | LSGQASSDT |
| | | KAMTNVGSFKTSLYY | MTNVGSFKT |
| | | EAEVNNFVKAMTNVG | VNNFVKAMT |
| | | KMKMFIICAVFVLIS | MFIICAVFV |
| | | MKMFIICAVFVLISS | MFIICAVFV |
| | | KKMKMFIICAVFVLI | MFIICAVFV |
| | | NKKMKMFIICAVFVL | MFIICAVFV |
| | | MNKKMKMFIICAVFV | MKMFIICAV |
| | | MFIICAVFVLISSCG | MFIICAVFV |
| | | DWSNLGTLVIRKEQD | WSNLGTLVI |
| | | WSNLGTLVIRKEQDG | WSNLGTLVI |

| | | FIICAVFVLISSCGN | VFVLISSCG |
|---|---|---|---|
| | | ICAVFVLISSCGNFR | VFVLISSCG |
| | | MTNVGSFKTSLYYGY | MTNVGSFKT |
| | | CAVFVLISSCGNFRS | VFVLISSCG |
| | | KMFIICAVFVLISSC | MFIICAVFV |
| | | IICAVFVLISSCGNF | VFVLISSCG |
| | | QLRGHSATFFCIEEA | LRGHSATFF |
| | | LRGHSATFFCIEEAE | LRGHSATFF |
| | | AMTNVGSFKTSLYYG | MTNVGSFKT |
| | | SSCGNFRSSLSDQGS | FRSSLSDQG |
| | | GLSGQASSDTIKFSE | LSGQASSDT |
| | | LSGQASSDTIKFSEF | LSGQASSDT |
| | | ISSCGNFRSSLSDQG | CGNFRSSLS |
| | | CGNFRSSLSDQGSLS | FRSSLSDQG |
| | | SCGNFRSSLSDQGSL | FRSSLSDQG |
| | | GNFRSSLSDQGSLSD | FRSSLSDQG |
| | | AVFVLISSCGNFRSS | VFVLISSCG |
| | | DGVETGLNVIGTING | VETGLNVIG |
| | | ETGLNVIGTINGQLR | LNVIGTING |
| | | TGLNVIGTINGQLRG | LNVIGTING |
| | | VFVLISSCGNFRSSL | VFVLISSCG |
| | | GVETGLNVIGTINGQ | LNVIGTING |
| | | VETGLNVIGTINGQL | LNVIGTING |
| | | LNVIGTINGQLRGHS | IGTINGQLR |
| | | NVIGTINGQLRGHSA | IGTINGQLR |
| | | GSLSDQGGLSGQASS | LSDQGGLSG |
| | | QGSLSDQGGLSGQAS | LSDQGGLSG |
| | | QDGVETGLNVIGTIN | VETGLNVIG |
| | | GLNVIGTINGQLRGH | IGTINGQLR |
| | | EQDGVETGLNVIGTI | VETGLNVIG |
| | | KEQDGVETGLNVIGT | VETGLNVIG |
| | | NFRSSLSDQGSLSDQ | FRSSLSDQG |
| | | FVLISSCGNFRSSLS | FVLISSCGN |
| | HLA-DRB1*0301 | None | |
| | HLA-DRB1*0401 | AEVNNFVKAMTNVGS | FVKAMTNVG |
| | | VNNFVKAMTNVGSFK | FVKAMTNVG |
| | | NNFVKAMTNVGSFKT | FVKAMTNVG |
| | | EVNNFVKAMTNVGSF | FVKAMTNVG |
| | | EAEVNNFVKAMTNVG | VNNFVKAMT |
| | | NFVKAMTNVGSFKTS | FVKAMTNVG |
| | | FVKAMTNVGSFKTSL | FVKAMTNVG |
| | | IKNKKDNNGDWSNLG | IKNKKDNNG |
| | | KNKKDNNGDWSNLGT | NNGDWSNLG |
| | | NKKDNNGDWSNLGTL | NNGDWSNLG |
| | | KKDNNGDWSNLGTLV | NNGDWSNLG |
| | | KDNNGDWSNLGTLVI | NNGDWSNLG |
| | HLA-DRB1*0404 | AEVNNFVKAMTNVGS | FVKAMTNVG |
| | | NNFVKAMTNVGSFKT | FVKAMTNVG |
| | | VNNFVKAMTNVGSFK | FVKAMTNVG |
| | | EVNNFVKAMTNVGSF | FVKAMTNVG |
| | | EAEVNNFVKAMTNVG | VNNFVKAMT |
| | | CAVFVLISSCGNFRS | VFVLISSCG |
| | | ICAVFVLISSCGNFR | VFVLISSCG |
| | | NFVKAMTNVGSFKTS | FVKAMTNVG |

| | HLA-DRB1*0405 | AEVNNFVKAMTNVGS | FVKAMTNVG |
|---|---|---|---|
| | | VNNFVKAMTNVGSFK | VKAMTNVGS |
| | | NNFVKAMTNVGSFKT | VKAMTNVGS |
| | | EVNNFVKAMTNVGSF | VKAMTNVGS |
| | | NFVKAMTNVGSFKTS | VKAMTNVGS |
| | | KDNNGDWSNLGTLVI | NNGDWSNLG |
| | | IKFSEFTVNIKNKKD | FSEFTVNIK |
| | | VKAMTNVGSFKTSLY | VKAMTNVGS |
| | | DNNGDWSNLGTLVIR | WSNLGTLVI |
| | | NNGDWSNLGTLVIRK | WSNLGTLVI |
| | HLA-DRB1*0701 | KDNNGDWSNLGTLVI | DWSNLGTLV |
| | | DNNGDWSNLGTLVIR | WSNLGTLVI |
| | | NNGDWSNLGTLVIRK | WSNLGTLVI |
| | | NGDWSNLGTLVIRKE | WSNLGTLVI |
| | | GDWSNLGTLVIRKEQ | WSNLGTLVI |
| | | KKMKMFIICAVFVLI | FIICAVFVL |
| | | KMKMFIICAVFVLIS | FIICAVFVL |
| | | MKMFIICAVFVLISS | FIICAVFVL |
| | | NKKMKMFIICAVFVL | MFIICAVFV |
| | | KMFIICAVFVLISSC | FIICAVFVL |
| | | MFIICAVFVLISSCG | FIICAVFVL |
| | HLA-DRB1*0802 | None | |
| | HLA-DRB1*0901 | NNFVKAMTNVGSFKT | FVKAMTNVG |
| | | AEVNNFVKAMTNVGS | FVKAMTNVG |
| | | EVNNFVKAMTNVGSF | FVKAMTNVG |
| | | VNNFVKAMTNVGSFK | FVKAMTNVG |
| | | EAEVNNFVKAMTNVG | VNNFVKAMT |
| | | NFVKAMTNVGSFKTS | FVKAMTNVG |
| | | FVKAMTNVGSFKTSL | FVKAMTNVG |
| | HLA-DRB1*1101 | NNFVKAMTNVGSFKT | VKAMTNVGS |
| | | NFVKAMTNVGSFKTS | MTNVGSFKT |
| | HLA-DRB1*1302 | AEVNNFVKAMTNVGS | VNNFVKAMT |
| | | EAEVNNFVKAMTNVG | VNNFVKAMT |
| | | VKAMTNVGSFKTSLY | MTNVGSFKT |
| | | ETGLNVIGTINGQLR | LNVIGTING |
| | | TGLNVIGTINGQLRG | LNVIGTING |
| | | KAMTNVGSFKTSLYY | VGSFKTSLY |
| | | SEFTVNIKNKKDNNG | FTVNIKNKK |
| | | EVNNFVKAMTNVGSF | FVKAMTNVG |
| | | VNNFVKAMTNVGSFK | FVKAMTNVG |
| | | NGDWSNLGTLVIRKE | WSNLGTLVI |
| | | NNFVKAMTNVGSFKT | FVKAMTNVG |
| | | KFSEFTVNIKNKKDN | FTVNIKNKK |
| | | FSEFTVNIKNKKDNN | FTVNIKNKK |
| | | GDWSNLGTLVIRKEQ | WSNLGTLVI |
| | | MTNVGSFKTSLYYGY | VGSFKTSLY |
| | | DNNGDWSNLGTLVIR | WSNLGTLVI |
| | | NNGDWSNLGTLVIRK | WSNLGTLVI |
| | | LNVIGTINGQLRGHS | IGTINGQLR |
| | | AMTNVGSFKTSLYYG | VGSFKTSLY |
| | | GLNVIGTINGQLRGH | IGTINGQLR |
| | | NFVKAMTNVGSFKTS | MTNVGSFKT |

| | | | |
|---|---|---|---|
| | | KDNNGDWSNLGTLVI | DWSNLGTLV |
| | | FTVNIKNKKDNNGDW | FTVNIKNKK |
| | | EFTVNIKNKKDNNGD | FTVNIKNKK |
| | | TIKFSEFTVNIKNKK | EFTVNIKNK |
| | | IKFSEFTVNIKNKKD | FTVNIKNKK |
| | | FVKAMTNVGSFKTSL | MTNVGSFKT |
| | | EEAEVNNFVKAMTNV | VNNFVKAMT |
| | | DWSNLGTLVIRKEQD | WSNLGTLVI |
| | | VETGLNVIGTINGQL | LNVIGTING |
| | | DGVETGLNVIGTING | TGLNVIGTI |
| | | CIEEAEVNNFVKAMT | EVNNFVKAM |
| | | GVETGLNVIGTINGQ | LNVIGTING |
| | | IEEAEVNNFVKAMTN | VNNFVKAMT |
| | | WSNLGTLVIRKEQDG | WSNLGTLVI |
| | | NVIGTINGQLRGHSA | IGTINGQLR |
| | | TNVGSFKTSLYYGYK | VGSFKTSLY |
| | | VIGTINGQLRGHSAT | IGTINGQLR |
| | | IGTINGQLRGHSATF | IGTINGQLR |
| | | NVGSFKTSLYYGYKE | VGSFKTSLY |
| | | VGSFKTSLYYGYKEE | VGSFKTSLY |
| | | TVNIKNKKDNNGDWS | IKNKKDNNG |
| | | VNIKNKKDNNGDWSN | IKNKKDNNG |
| | | TTKIETINNSEHITF | TINNSEHIT |
| | | TKIETINNSEHITFS | TINNSEHIT |
| | | CAVFVLISSCGNFRS | VFVLISSCG |
| | HLA-DRB1*1501 | ICAVFVLISSCGNFR | VFVLISSCG |
| | | CAVFVLISSCGNFRS | VFVLISSCG |
| | | MFIICAVFVLISSCG | CAVFVLISS |
| | | IICAVFVLISSCGNF | VFVLISSCG |
| | | FIICAVFVLISSCGN | VFVLISSCG |
| | | AVFVLISSCGNFRSS | VFVLISSCG |
| | | SSTNGIKGKEITTKI | IKGKEITTK |
| | | STNGIKGKEITTKIE | IKGKEITTK |
| | | NGIKGKEITTKIETI | IKGKEITTK |
| | | QSSTNGIKGKEITTK | STNGIKGKE |
| | | TNGIKGKEITTKIET | IKGKEITTK |
| | | VFVLISSCGNFRSSL | VFVLISSCG |
| | | EVNNFVKAMTNVGSF | VKAMTNVGS |
| | | VNNFVKAMTNVGSFK | VKAMTNVGS |
| | | NNFVKAMTNVGSFKT | VKAMTNVGS |
| | | AEVNNFVKAMTNVGS | FVKAMTNVG |
| | | NKKMKMFIICAVFVL | MKMFIICAV |
| | | NFVKAMTNVGSFKTS | VKAMTNVGS |
| | | KKMKMFIICAVFVLI | MKMFIICAV |
| | | MNKKMKMFIICAVFV | MKMFIICAV |
| | HLA-DRB3*0101 | None | |
| | HLA-DRB4*0101 | None | |
| | HLA-DRB5*0101 | TSLYYGYKEEQSSTN | YGYKEEQSS |
| | | SLYYGYKEEQSSTNG | YKEEQSSTN |
| | | YGYKEEQSSTNGIKG | YKEEQSSTN |
| | | YYGYKEEQSSTNGIK | YKEEQSSTN |
| | | LYYGYKEEQSSTNGI | YKEEQSSTN |
| | | | |

| CAA57806.1\| Outer surface protein G [Borrelia burgdorferi]<br><br>Seq13 | HLA-DRB1*0101 | PNNSLFNPPPVLPAS | LFNPPPVLP |
| --- | --- | --- | --- |
| | | NNSLFNPPPVLPASS | LFNPPPVLP |
| | | NSLFNPPPVLPASSH | LFNPPPVLP |
| | | DPNNSLFNPPPVLPA | LFNPPPVLP |
| | | SLFNPPPVLPASSHD | FNPPPVLPA |
| | | DDPNNSLFNPPPVLP | DDPNNSLFN |
| | | LFNPPPVLPASSHDN | LFNPPPVLP |
| | | DNTPVLKAVQAKDGG | LKAVQAKDG |
| | | TPVLKAVQAKDGGQQ | LKAVQAKDG |
| | | HDNTPVLKAVQAKDG | TPVLKAVQA |
| | | NTPVLKAVQAKDGGQ | LKAVQAKDG |
| | | PVLKAVQAKDGGQQE | LKAVQAKDG |
| | | FNPPPVLPASSHDNT | FNPPPVLPA |
| | | NKKMKNLIICAVFVL | MKNLIICAV |
| | | MNKKMKNLIICAVFV | MKNLIICAV |
| | | KKMKNLIICAVFVLI | MKNLIICAV |
| | | YAKELGVNGSYSVND | LGVNGSYSV |
| | | AKELGVNGSYSVNDG | LGVNGSYSV |
| | | LKYAKELGVNGSYSV | YAKELGVNG |
| | | KELGVNGSYSVNDGT | LGVNGSYSV |
| | | KYAKELGVNGSYSVN | LGVNGSYSV |
| | | KMKNLIICAVFVLII | MKNLIICAV |
| | | MKNLIICAVFVLIIS | MKNLIICAV |
| | | VKKQGNIGQKALKYA | KQGNIGQKA |
| | | VLKAVQAKDGGQQEG | LKAVQAKDG |
| | | LKAVQAKDGGQQEGK | LKAVQAKDG |
| | | PPPVLPASSHDNTPV | VLPASSHDN |
| | | NPPPVLPASSHDNTP | VLPASSHDN |
| | | KQGNIGQKALKYAKE | IGQKALKYA |
| | | KKQGNIGQKALKYAK | IGQKALKYA |
| | | QGNIGQKALKYAKEL | IGQKALKYA |
| | | PPVLPASSHDNTPVL | VLPASSHDN |
| | | GNIGQKALKYAKELG | IGQKALKYA |
| | | LGVNGSYSVNDGTNT | LGVNGSYSV |
| | | ELGVNGSYSVNDGTN | LGVNGSYSV |
| | | KEQVESATGESTEKV | VESATGEST |
| | | EQVESATGESTEKVK | VESATGEST |
| | | QKALKYAKELGVNGS | YAKELGVNG |
| | | KALKYAKELGVNGSY | YAKELGVNG |
| | | FLDKELMQGDDPNNS | FLDKELMQG |
| | | LIICAVFVLIISCKI | ICAVFVLII |
| | | ALKYAKELGVNGSYS | YAKELGVNG |
| | | NIGQKALKYAKELGV | IGQKALKYA |
| | | KELMQGDDPNNSLFN | MQGDDPNNS |
| | | IGQKALKYAKELGVN | IGQKALKYA |
| | | QEFKEQVESATGEST | FKEQVESAT |
| | | LDKELMQGDDPNNSL | MQGDDPNNS |
| | | DKELMQGDDPNNSLF | MQGDDPNNS |
| | | FKEQVESATGESTEK | VESATGEST |
| | | ELMQGDDPNNSLFNP | MQGDDPNNS |
| | HLA-DRB1*0301 | None | |
| | HLA-DRB1*0401 | FLDKELMQGDDPNNS | FLDKELMQG |
| | | LDKELMQGDDPNNSL | MQGDDPNNS |
| | | KELMQGDDPNNSLFN | MQGDDPNNS |
| | | DKELMQGDDPNNSLF | MQGDDPNNS |
| | | ELMQGDDPNNSLFNP | MQGDDPNNS |

| | HLA-DRB1*0404 | None | |
|---|---|---|---|
| | HLA-DRB1*0405 | DPNNSLFNPPPVLPA<br>PNNSLFNPPPVLPAS | NNSLFNPPP<br>FNPPPVLPA |
| | HLA-DRB1*0701 | KMKNLIICAVFVLII<br>MKNLIICAVFVLIIS<br>KNLIICAVFVLIISC<br>KKMKNLIICAVFVLI<br>NKKMKNLIICAVFVL | LIICAVFVL<br>LIICAVFVL<br>LIICAVFVL<br>LIICAVFVL<br>MKNLIICAV |
| | HLA-DRB1*0802 | None | |
| | HLA-DRB1*0901 | None | |
| | HLA-DRB1*1101 | None | |
| | HLA-DRB1*1302 | MNKKMKNLIICAVFV<br>KKMKNLIICAVFVLI<br>NKKMKNLIICAVFVL<br>AKELGVNGSYSVNDG<br>LKYAKELGVNGSYSV<br>YAKELGVNGSYSVND<br>KYAKELGVNGSYSVN<br>KELGVNGSYSVNDGT<br>MKNLIICAVFVLIIS<br>LGVNGSYSVNDGTNT<br>KMKNLIICAVFVLII<br>LIICAVFVLIISCKI<br>ICAVFVLIISCKIDA<br>CAVFVLIISCKIDAS<br>IICAVFVLIISCKID | MNKKMKNLI<br>MKNLIICAV<br>MKNLIICAV<br>LGVNGSYSV<br>AKELGVNGS<br>LGVNGSYSV<br>LGVNGSYSV<br>LGVNGSYSV<br>MKNLIICAV<br>LGVNGSYSV<br>MKNLIICAV<br>VFVLIISCK<br>VFVLIISCK<br>VFVLIISCK<br>VFVLIISCK |
| | HLA-DRB1*1501 | CAVFVLIISCKIDAS<br>IICAVFVLIISCKID<br>ICAVFVLIISCKIDA<br>LIICAVFVLIISCKI<br>NLIICAVFVLIISCK<br>KKMKNLIICAVFVLI<br>NKKMKNLIICAVFVL<br>MNKKMKNLIICAVFV | VFVLIISCK<br>VFVLIISCK<br>VFVLIISCK<br>VFVLIISCK<br>CAVFVLIIS<br>MKNLIICAV<br>MKNLIICAV<br>MKNLIICAV |
| | HLA-DRB3*0101 | None | |
| | HLA-DRB4*0101 | None | |
| | HLA-DRB5*0101 | None | |
| | | | |
| AAC44770.1\| FlaA protein (Borrelia burgdorferi)<br><br>Seq14 | HLA-DRB1*0101 | LFPSYSQSSAMIMPP<br>NWSVLLTPSARLQAY<br>WSVLLTPSARLQAYV<br>SNPNYIPNISSRIIK<br>NPNYIPNISSRIIKD<br>PNYIPNISSRIIKDD<br>WSNPNYIPNISSRII<br>FPSYSQSSAMIMPPF<br>GINNWSVLLTPSARL<br>PSYSQSSAMIMPPFK<br>TESLRKLKAHETFKR<br>AYVKNSVVAPAVVKS<br>ILGVRVLFPSYSQSS<br>LGVRVLFPSYSQSSA | YSQSSAMIM<br>LLTPSARLQ<br>LLTPSARLQ<br>IPNISSRII<br>IPNISSRII<br>IPNISSRII<br>YIPNISSRI<br>YSQSSAMIM<br>WSVLLTPSA<br>YSQSSAMIM<br>LRKLKAHET<br>VKNSVVAPA<br>VRVLFPSYS<br>LFPSYSQSS |

| | | | |
|---|---|---|---|
| | | ESLRKLKAHETFKRV | LRKLKAHET |
| | | AKSILFFLLSTVLFA | FFLLSTVLF |
| | | KSILFFLLSTVLFAQ | FFLLSTVLF |
| | | SILFFLLSTVLFAQE | FFLLSTVLF |
| | | EDMNGMEYAYSMGTL | MEYAYSMGT |
| | | YVKNSVVAPAVVKSE | VVAPAVVKS |
| | | VKNSVVAPAVVKSES | VVAPAVVKS |
| | | KNSVVAPAVVKSESK | VVAPAVVKS |
| | | NSVVAPAVVKSESKR | VVAPAVVKS |
| | | FEDMNGMEYAYSMGT | MNGMEYAYS |
| | | GTESLRKLKAHETFK | LRKLKAHET |
| | | DLGINNWSVLLTPSA | INNWSVLLT |
| | | MRFKAFRVSKSHSSK | FRVSKSHSS |
| | | TSGTESLRKLKAHET | TESLRKLKA |
| | | SGTESLRKLKAHETF | LRKLKAHET |
| | | LGINNWSVLLTPSAR | INNWSVLLT |
| | | NYIPNISSRIIKDDV | IPNISSRII |
| | | KMRFKAFRVSKSHSS | FKAFRVSKS |
| | | KAKSILFFLLSTVLF | LFFLLSTVL |
| | | KDDVPNYPLASSKMR | NYPLASSKM |
| | | DDVPNYPLASSKMRF | YPLASSKMR |
| | | SVLLTPSARLQAYVK | LLTPSARLQ |
| | | VRVLFPSYSQSSAMI | LFPSYSQSS |
| | | GVRVLFPSYSQSSAM | LFPSYSQSS |
| | | MNGMEYAYSMGTLKF | MEYAYSMGT |
| | | FKAFRVSKSHSSKVK | FRVSKSHSS |
| | | ILFFLLSTVLFAQET | FFLLSTVLF |
| | | VPNYPLASSKMRFKA | YPLASSKMR |
| | | RFKAFRVSKSHSSKV | FRVSKSHSS |
| | | DVPNYPLASSKMRFK | YPLASSKMR |
| | | YSQSSAMIMPPFKIP | YSQSSAMIM |
| | | DMNGMEYAYSMGTLK | MEYAYSMGT |
| | | KAFRVSKSHSSKVKN | FRVSKSHSS |
| | | NGMEYAYSMGTLKFK | MEYAYSMGT |
| | | SYSQSSAMIMPPFKI | YSQSSAMIM |
| | | GDTILGVRVLFPSYS | ILGVRVLFP |
| | | DTILGVRVLFPSYSQ | VRVLFPSYS |
| | | PNYPLASSKMRFKAF | YPLASSKMR |
| | | TILGVRVLFPSYSQS | VRVLFPSYS |
| | | VDLGINNWSVLLTPS | INNWSVLLT |
| | | MVVDLGINNWSVLLT | VVDLGINNW |
| | | YIPNISSRIIKDDVP | IPNISSRII |
| | | VVDLGINNWSVLLTP | INNWSVLLT |
| | | LFFLLSTVLFAQETD | FFLLSTVLF |
| | | NYVDYVYSGASGIVK | VYSGASGIV |
| | | VLLTPSARLQAYVKN | LLTPSARLQ |
| | | QAYVKNSVVAPAVVK | VKNSVVAPA |
| | | SLRKLKAHETFKRVL | LRKLKAHET |
| | | LRKLKAHETFKRVLK | LRKLKAHET |
| | | IWSNPNYIPNISSRI | IWSNPNYIP |
| | | YVDYVYSGASGIVKP | VYSGASGIV |
| | | VDYVYSGASGIVKPE | VYSGASGIV |
| | | VVAPAVVKSESKRYA | VVAPAVVKS |
| | | LQAYVKNSVVAPAVV | VKNSVVAPA |
| | | RLQAYVKNSVVAPAV | VKNSVVAPA |
| | | SVVAPAVVKSESKRY | VVAPAVVKS |
| | | RKAKSILFFLLSTVL | RKAKSILFF |

| | | | |
|---|---|---|---|
| | | ARLQAYVKNSVVAPA | YVKNSVVAP |
| | | SVFKVYETSGTESLR | YETSGTESL |
| | | ESVFKVYETSGTESL | FKVYETSGT |
| | | SSKMRFKAFRVSKSH | FKAFRVSKS |
| | | SKMRFKAFRVSKSHS | FKAFRVSKS |
| | | ASSKMRFKAFRVSKS | MRFKAFRVS |
| | | TNYVDYVYSGASGIV | YVYSGASGI |
| | | IKDDVPNYPLASSKM | VPNYPLASS |
| | | GMEYAYSMGTLKFKG | MEYAYSMGT |
| | | MEYAYSMGTLKFKGW | MEYAYSMGT |
| | | LFEDMNGMEYAYSMG | MNGMEYAYS |
| | | DYVYSGASGIVKPED | VYSGASGIV |
| | | VLFEDMNGMEYAYSM | MNGMEYAYS |
| | | KDLRVLYDKLSVSID | LYDKLSVSI |
| | | VKDLRVLYDKLSVSI | LRVLYDKLS |
| | | FFLLSTVLFAQETDG | FFLLSTVLF |
| | | EVLFEDMNGMEYAYS | FEDMNGMEY |
| | | FKVYETSGTESLRKL | YETSGTESL |
| | | AFRVSKSHSSKVKNF | FRVSKSHSS |
| | | VFKVYETSGTESLRK | YETSGTESL |
| | | NYPLASSKMRFKAFR | YPLASSKMR |
| | | FRVSKSHSSKVKNFI | FRVSKSHSS |
| | | IFYVKDLRVLYDKLS | VKDLRVLYD |
| | | LRVLYDKLSVSIDSD | LYDKLSVSI |
| | | DLRVLYDKLSVSIDS | LYDKLSVSI |
| | | SKRYAGDTILGVRVL | YAGDTILGV |
| | | FAELARDPSSTRLDL | ARDPSSTRL |
| | | LDFAELARDPSSTRL | LARDPSSTR |
| | | FYVKDLRVLYDKLSV | LRVLYDKLS |
| | | DFAELARDPSSTRLD | ARDPSSTRL |
| | | AELARDPSSTRLDLT | ARDPSSTRL |
| | | LLTPSARLQAYVKNS | LLTPSARLQ |
| | | ESKRYAGDTILGVRV | YAGDTILGV |
| | | RIIKDDVPNYPLASS | IKDDVPNYP |
| | | QSSAMIMPPFKIPFY | MIMPPFKIP |
| | | IPNISSRIIKDDVPN | IPNISSRII |
| | | SQSSAMIMPPFKIPF | MIMPPFKIP |
| | | SSAMIMPPFKIPFYS | MIMPPFKIP |
| | | YVYSGASGIVKPEDM | VYSGASGIV |
| | | IIKDDVPNYPLASSK | VPNYPLASS |
| | | KRYAGDTILGVRVLF | YAGDTILGV |
| | | GLIDNIKTMKEIKVS | IKTMKEIKV |
| | | KVYETSGTESLRKLK | YETSGTESL |
| | | RVLYDKLSVSIDSDI | LYDKLSVSI |
| | | KGLIDNIKTMKEIKV | IDNIKTMKE |
| | | IDNIKTMKEIKVSVY | IKTMKEIKV |
| | | SESVFKVYETSGTES | FKVYETSGT |
| | | ADLIWSNPNYIPNIS | LIWSNPNYI |
| | | SARLQAYVKNSVVAP | AYVKNSVVA |
| | | NFIFYVKDLRVLYDK | FYVKDLRVL |
| | | IDSESVFKVYETSGT | IDSESVFKV |
| | | SESKRYAGDTILGVR | YAGDTILGV |
| | | KNFIFYVKDLRVLYD | FYVKDLRVL |
| | | KSESKRYAGDTILGV | RYAGDTILG |
| | | DSESVFKVYETSGTE | FKVYETSGT |
| | | KGWADLIWSNPNYIP | LIWSNPNYI |
| | | GWADLIWSNPNYIPN | LIWSNPNYI |

| | | | |
|---|---|---|---|
| | | LIDNIKTMKEIKVSV | IKTMKEIKV |
| | | RYAGDTILGVRVLFP | YAGDTILGV |
| | | WADLIWSNPNYIPNI | LIWSNPNYI |
| | | DNIKTMKEIKVSVYS | IKTMKEIKV |
| | | YAGDTILGVRVLFPS | ILGVRVLFP |
| | | FIFYVKDLRVLYDKL | FYVKDLRVL |
| | | ELARDPSSTRLDLTN | ARDPSSTRL |
| | | YVKDLRVLYDKLSVS | LRVLYDKLS |
| | | APAVVKSESKRYAGD | VVKSESKRY |
| | | VAPAVVKSESKRYAG | VVKSESKRY |
| | | PAVVKSESKRYAGDT | VVKSESKRY |
| | | LASSKMRFKAFRVSK | MRFKAFRVS |
| | | FKGWADLIWSNPNYI | FKGWADLIW |
| | | EYAYSMGTLKFKGWA | AYSMGTLKF |
| | | SAMIMPPFKIPFYSG | MIMPPFKIP |
| | | PLASSKMRFKAFRVS | KMRFKAFRV |
| | | YPLASSKMRFKAFRV | YPLASSKMR |
| | | LLSTVLFAQETDGLA | STVLFAQET |
| | | FLLSTVLFAQETDGL | STVLFAQET |
| | | LTNYVDYVYSGASGI | VDYVYSGAS |
| | | VKNFIFYVKDLRVLY | FYVKDLRVL |
| | | KVKNFIFYVKDLRVL | IFYVKDLRV |
| | | VYSGASGIVKPEDMV | VYSGASGIV |
| | | DLIWSNPNYIPNISS | LIWSNPNYI |
| | | AGDTILGVRVLFPSY | ILGVRVLFP |
| | | VLDFAELARDPSSTR | FAELARDPS |
| | HLA-DRB1*0301 | VKNFIFYVKDLRVLY | FIFYVKDLR |
| | | KNFIFYVKDLRVLYD | YVKDLRVLY |
| | | NFIFYVKDLRVLYDK | YVKDLRVLY |
| | | IFYVKDLRVLYDKLS | YVKDLRVLY |
| | HLA-DRB1*0401 | KNFIFYVKDLRVLYD | YVKDLRVLY |
| | | NFIFYVKDLRVLYDK | YVKDLRVLY |
| | | VKNFIFYVKDLRVLY | FIFYVKDLR |
| | | FIFYVKDLRVLYDKL | YVKDLRVLY |
| | | IFYVKDLRVLYDKLS | YVKDLRVLY |
| | | ILFFLLSTVLFAQET | FFLLSTVLF |
| | | KSILFFLLSTVLFAQ | FFLLSTVLF |
| | | SILFFLLSTVLFAQE | FFLLSTVLF |
| | | AKSILFFLLSTVLFA | FFLLSTVLF |
| | | KAKSILFFLLSTVLF | LFFLLSTVL |
| | | VLDFAELARDPSSTR | FAELARDPS |
| | | LFFLLSTVLFAQETD | FLLSTVLFA |
| | | LDFAELARDPSSTRL | LARDPSSTR |
| | | ARLQAYVKNSVVAPA | YVKNSVVAP |
| | | KMRFKAFRVSKSHSS | FKAFRVSKS |
| | | RLQAYVKNSVVAPAV | VKNSVVAPA |
| | | MRFKAFRVSKSHSSK | FRVSKSHSS |
| | | DFAELARDPSSTRLD | LARDPSSTR |
| | | LQAYVKNSVVAPAVV | VKNSVVAPA |
| | | FYVKDLRVLYDKLSV | YVKDLRVLY |
| | | FAELARDPSSTRLDL | LARDPSSTR |
| | | YVKDLRVLYDKLSVS | YVKDLRVLY |
| | | QAYVKNSVVAPAVVK | VKNSVVAPA |
| | | AELARDPSSTRLDLT | LARDPSSTR |
| | | INNWSVLLTPSARLQ | WSVLLTPSA |
| | | GINNWSVLLTPSARL | WSVLLTPSA |

| | | | |
|---|---|---|---|
| | | NNWSVLLTPSARLQA | WSVLLTPSA |
| | | AYVKNSVVAPAVVKS | VKNSVVAPA |
| | | DLGINNWSVLLTPSA | GINNWSVLL |
| | | FFLLSTVLFAQETDG | FFLLSTVLF |
| | | LGINNWSVLLTPSAR | WSVLLTPSA |
| | | FKAFRVSKSHSSKVK | FRVSKSHSS |
| | | RFKAFRVSKSHSSKV | FRVSKSHSS |
| | | LGVRVLFPSYSQSSA | LFPSYSQSS |
| | | ILGVRVLFPSYSQSS | VRVLFPSYS |
| | | GVRVLFPSYSQSSAM | LFPSYSQSS |
| | | NGMEYAYSMGTLKFK | MEYAYSMGT |
| | | MNGMEYAYSMGTLKF | MEYAYSMGT |
| | HLA-DRB1*0404 | INNWSVLLTPSARLQ | VLLTPSARL |
| | | NNWSVLLTPSARLQA | VLLTPSARL |
| | | GINNWSVLLTPSARL | WSVLLTPSA |
| | | NWSVLLTPSARLQAY | VLLTPSARL |
| | | WSVLLTPSARLQAYV | VLLTPSARL |
| | | KGWADLIWSNPNYIP | WADLIWSNP |
| | | FKGWADLIWSNPNYI | WADLIWSNP |
| | | ILGVRVLFPSYSQSS | ILGVRVLFP |
| | | KSILFFLLSTVLFAQ | FLLSTVLFA |
| | | LGINNWSVLLTPSAR | WSVLLTPSA |
| | | DLGINNWSVLLTPSA | GINNWSVLL |
| | | AKSILFFLLSTVLFA | ILFFLLSTV |
| | | GWADLIWSNPNYIPN | LIWSNPNYI |
| | | WADLIWSNPNYIPNI | LIWSNPNYI |
| | | ILFFLLSTVLFAQET | FLLSTVLFA |
| | | SILFFLLSTVLFAQE | FLLSTVLFA |
| | | DTILGVRVLFPSYSQ | ILGVRVLFP |
| | | GDTILGVRVLFPSYS | ILGVRVLFP |
| | | AGDTILGVRVLFPSY | ILGVRVLFP |
| | | RYAGDTILGVRVLFP | YAGDTILGV |
| | | YAGDTILGVRVLFPS | ILGVRVLFP |
| | | LFFLLSTVLFAQETD | FLLSTVLFA |
| | | SVLLTPSARLQAYVK | VLLTPSARL |
| | HLA-DRB1*0405 | IFYVKDLRVLYDKLS | VKDLRVLYD |
| | | FYVKDLRVLYDKLSV | VKDLRVLYD |
| | | KNFIFYVKDLRVLYD | YVKDLRVLY |
| | | NFIFYVKDLRVLYDK | VKDLRVLYD |
| | | FIFYVKDLRVLYDKL | VKDLRVLYD |
| | | FKGWADLIWSNPNYI | ADLIWSNPN |
| | | GWADLIWSNPNYIPN | LIWSNPNYI |
| | | DLGINNWSVLLTPSA | INNWSVLLT |
| | | KGWADLIWSNPNYIP | ADLIWSNPN |
| | | LGINNWSVLLTPSAR | INNWSVLLT |
| | | DLIWSNPNYIPNISS | LIWSNPNYI |
| | | KMRFKAFRVSKSHSS | FKAFRVSKS |
| | | VVDLGINNWSVLLTP | INNWSVLLT |
| | | VDLGINNWSVLLTPS | INNWSVLLT |
| | | MRFKAFRVSKSHSSK | FKAFRVSKS |
| | | MVVDLGINNWSVLLT | GINNWSVLL |
| | | ASSKMRFKAFRVSKS | SKMRFKAFR |
| | | SSKMRFKAFRVSKSH | FKAFRVSKS |
| | | SKMRFKAFRVSKSHS | FKAFRVSKS |
| | | WADLIWSNPNYIPNI | LIWSNPNYI |

| | | | |
|---|---|---|---|
| | | LIWSNPNYIPNISSR | PNYIPNISS |
| | | GINNWSVLLTPSARL | INNWSVLLT |
| | | ADLIWSNPNYIPNIS | LIWSNPNYI |
| | | INNWSVLLTPSARLQ | INNWSVLLT |
| | | LKFKGWADLIWSNPN | FKGWADLIW |
| | | YVKDLRVLYDKLSVS | VKDLRVLYD |
| | | VLDFAELARDPSSTR | FAELARDPS |
| | | VKDLRVLYDKLSVSI | VKDLRVLYD |
| | | LDFAELARDPSSTRL | LARDPSSTR |
| | | KFKGWADLIWSNPNY | ADLIWSNPN |
| | | DFAELARDPSSTRLD | LARDPSSTR |
| | | DTILGVRVLFPSYSQ | VRVLFPSYS |
| | | FAELARDPSSTRLDL | LARDPSSTR |
| | | ILGVRVLFPSYSQSS | RVLFPSYSQ |
| | | TILGVRVLFPSYSQS | RVLFPSYSQ |
| | | AELARDPSSTRLDLT | LARDPSSTR |
| | | LGVRVLFPSYSQSSA | RVLFPSYSQ |
| | HLA-DRB1*0701 | WSNPNYIPNISSRII | YIPNISSRI |
| | | SNPNYIPNISSRIIK | IPNISSRII |
| | | NPNYIPNISSRIIKD | IPNISSRII |
| | | PNYIPNISSRIIKDD | IPNISSRII |
| | | NYIPNISSRIIKDDV | IPNISSRII |
| | | YIPNISSRIIKDDVP | IPNISSRII |
| | | NWSVLLTPSARLQAY | VLLTPSARL |
| | | GINNWSVLLTPSARL | SVLLTPSAR |
| | | WSVLLTPSARLQAYV | VLLTPSARL |
| | | INNWSVLLTPSARLQ | VLLTPSARL |
| | | NNWSVLLTPSARLQA | VLLTPSARL |
| | | IWSNPNYIPNISSRI | WSNPNYIPN |
| | | RFKAFRVSKSHSSKV | FRVSKSHSS |
| | | FKAFRVSKSHSSKVK | VSKSHSSKV |
| | | KAFRVSKSHSSKVKN | VSKSHSSKV |
| | | IKTMKEIKVSVYSLG | MKEIKVSVY |
| | | KTMKEIKVSVYSLGY | MKEIKVSVY |
| | | NIKTMKEIKVSVYSL | MKEIKVSVY |
| | | KSILFFLLSTVLFAQ | LFFLLSTVL |
| | | SILFFLLSTVLFAQE | LFFLLSTVL |
| | | AKSILFFLLSTVLFA | LFFLLSTVL |
| | | AFRVSKSHSSKVKNF | VSKSHSSKV |
| | | FRVSKSHSSKVKNFI | VSKSHSSKV |
| | | IDNIKTMKEIKVSVY | TMKEIKVSV |
| | | DNIKTMKEIKVSVYS | MKEIKVSVY |
| | | MVVDLGINNWSVLLT | LGINNWSVL |
| | | KMRFKAFRVSKSHSS | FKAFRVSKS |
| | | VVDLGINNWSVLLTP | INNWSVLLT |
| | | SVLLTPSARLQAYVK | VLLTPSARL |
| | | VLLTPSARLQAYVKN | VLLTPSARL |
| | | KAKSILFFLLSTVLF | LFFLLSTVL |
| | | MRFKAFRVSKSHSSK | FRVSKSHSS |
| | | VDLGINNWSVLLTPS | LGINNWSVL |
| | | RVLFPSYSQSSAMIM | LFPSYSQSS |
| | | VVAPAVVKSESKRYA | VVKSESKRY |
| | | VAPAVVKSESKRYAG | VVKSESKRY |
| | | IPNISSRIIKDDVPN | IPNISSRII |
| | | APAVVKSESKRYAGD | VVKSESKRY |
| | | DMVVDLGINNWSVLL | LGINNWSVL |

| | | | |
|---|---|---|---|
| | | SVVAPAVVKSESKRY | SVVAPAVVK |
| | | RKAKSILFFLLSTVL | AKSILFFLL |
| | | PAVVKSESKRYAGDT | VVKSESKRY |
| | | VLFPSYSQSSAMIMP | YSQSSAMIM |
| | | ILFFLLSTVLFAQET | LFFLLSTVL |
| | HLA-DRB1*0802 | None | |
| | HLA-DRB1*0901 | NNFVKAMTNVGSFKT | FVKAMTNVG |
| | | AEVNNFVKAMTNVGS | FVKAMTNVG |
| | | EVNNFVKAMTNVGSF | FVKAMTNVG |
| | | VNNFVKAMTNVGSFK | FVKAMTNVG |
| | | EAEVNNFVKAMTNVG | VNNFVKAMT |
| | | NFVKAMTNVGSFKTS | FVKAMTNVG |
| | | FVKAMTNVGSFKTSL | FVKAMTNVG |
| | HLA-DRB1*1101 | ASSKMRFKAFRVSKS | MRFKAFRVS |
| | | LASSKMRFKAFRVSK | MRFKAFRVS |
| | | SSKMRFKAFRVSKSH | MRFKAFRVS |
| | | SKMRFKAFRVSKSHS | MRFKAFRVS |
| | | PLASSKMRFKAFRVS | ASSKMRFKA |
| | HLA-DRB1*1302 | MVVDLGINNWSVLLT | LGINNWSVL |
| | | VVDLGINNWSVLLTP | INNWSVLLT |
| | | VDLGINNWSVLLTPS | INNWSVLLT |
| | | DLGINNWSVLLTPSA | INNWSVLLT |
| | | LGINNWSVLLTPSAR | INNWSVLLT |
| | | DMVVDLGINNWSVLL | LGINNWSVL |
| | | EDMVVDLGINNWSVL | VVDLGINNW |
| | | GINNWSVLLTPSARL | INNWSVLLT |
| | | INNWSVLLTPSARLQ | INNWSVLLT |
| | | SNPNYIPNISSRIIK | IPNISSRII |
| | | NPNYIPNISSRIIKD | IPNISSRII |
| | | AKSILFFLLSTVLFA | FFLLSTVLF |
| | | KSILFFLLSTVLFAQ | FFLLSTVLF |
| | | SILFFLLSTVLFAQE | FFLLSTVLF |
| | | KAKSILFFLLSTVLF | ILFFLLSTV |
| | | WSNPNYIPNISSRII | YIPNISSRI |
| | | ILFFLLSTVLFAQET | FFLLSTVLF |
| | | PNYIPNISSRIIKDD | IPNISSRII |
| | | VKNFIFYVKDLRVLY | VKNFIFYVK |
| | | SKVKNFIFYVKDLRV | VKNFIFYVK |
| | | NYIPNISSRIIKDDV | IPNISSRII |
| | | SSKVKNFIFYVKDLR | VKNFIFYVK |
| | | KSHSSKVKNFIFYVK | SKVKNFIFY |
| | | ARLQAYVKNSVVAPA | YVKNSVVAP |
| | | IWSNPNYIPNISSRI | NPNYIPNIS |
| | | HSSKVKNFIFYVKDL | VKNFIFYVK |
| | | SHSSKVKNFIFYVKD | VKNFIFYVK |
| | | LQAYVKNSVVAPAVV | VKNSVVAPA |
| | | RLQAYVKNSVVAPAV | VKNSVVAPA |
| | | QAYVKNSVVAPAVVK | VKNSVVAPA |
| | | GDTILGVRVLFPSYS | LGVRVLFPS |
| | | YIPNISSRIIKDDVP | IPNISSRII |
| | | IKTMKEIKVSVYSLG | MKEIKVSVY |
| | | ADLIWSNPNYIPNIS | LIWSNPNYI |
| | | DTILGVRVLFPSYSQ | LGVRVLFPS |

| | | | |
|---|---|---|---|
| | | LFFLLSTVLFAQETD | FFLLSTVLF |
| | | AYVKNSVVAPAVVKS | VKNSVVAPA |
| | | KGLIDNIKTMKEIKV | LIDNIKTMK |
| | | TILGVRVLFPSYSQS | LGVRVLFPS |
| | | YAGDTILGVRVLFPS | TILGVRVLF |
| | | AGDTILGVRVLFPSY | LGVRVLFPS |
| | | FFLLSTVLFAQETDG | FFLLSTVLF |
| | | LGKGLIDNIKTMKEI | LIDNIKTMK |
| | | KTMKEIKVSVYSLGY | IKVSVYSLG |
| | | KVKNFIFYVKDLRVL | VKNFIFYVK |
| | | DLIWSNPNYIPNISS | NPNYIPNIS |
| | | FLGKGLIDNIKTMKE | LIDNIKTMK |
| | | GKGLIDNIKTMKEIK | LIDNIKTMK |
| | | TMKEIKVSVYSLGYE | IKVSVYSLG |
| | | MKEIKVSVYSLGYEI | IKVSVYSLG |
| | | LIWSNPNYIPNISSR | NPNYIPNIS |
| | | QFLGKGLIDNIKTMK | KGLIDNIKT |
| | | KNFIFYVKDLRVLYD | YVKDLRVLY |
| | | WADLIWSNPNYIPNI | LIWSNPNYI |
| | | SARLQAYVKNSVVAP | LQAYVKNSV |
| | | KEIKVSVYSLGYEID | IKVSVYSLG |
| | | GWADLIWSNPNYIPN | LIWSNPNYI |
| | | GLIDNIKTMKEIKVS | LIDNIKTMK |
| | | YVKNSVVAPAVVKSE | VKNSVVAPA |
| | | KGWADLIWSNPNYIP | LIWSNPNYI |
| | | LGVRVLFPSYSQSSA | LGVRVLFPS |
| | | NFIFYVKDLRVLYDK | YVKDLRVLY |
| | | IFYVKDLRVLYDKLS | YVKDLRVLY |
| | | ILGVRVLFPSYSQSS | LGVRVLFPS |
| | | LIDNIKTMKEIKVSV | LIDNIKTMK |
| | | FIFYVKDLRVLYDKL | YVKDLRVLY |
| | | SSTRLDLTNYVDYVY | TRLDLTNYV |
| | | STRLDLTNYVDYVYS | LTNYVDYVY |
| | HLA-DRB1*1501 | KSILFFLLSTVLFAQ | ILFFLLSTV |
| | | AKSILFFLLSTVLFA | ILFFLLSTV |
| | | SKVKNFIFYVKDLRV | VKNFIFYVK |
| | | RKAKSILFFLLSTVL | ILFFLLSTV |
| | | KAKSILFFLLSTVLF | ILFFLLSTV |
| | | SSKVKNFIFYVKDLR | VKNFIFYVK |
| | | KVKNFIFYVKDLRVL | VKNFIFYVK |
| | | KSHSSKVKNFIFYVK | KVKNFIFYV |
| | | SHSSKVKNFIFYVKD | VKNFIFYVK |
| | | HSSKVKNFIFYVKDL | VKNFIFYVK |
| | | VKNFIFYVKDLRVLY | VKNFIFYVK |
| | | ASSKMRFKAFRVSKS | MRFKAFRVS |
| | | SSKMRFKAFRVSKSH | MRFKAFRVS |
| | | SILFFLLSTVLFAQE | LFFLLSTVL |
| | | KRKAKSILFFLLSTV | SILFFLLST |
| | | SKMRFKAFRVSKSHS | MRFKAFRVS |
| | | LASSKMRFKAFRVSK | MRFKAFRVS |
| | | PLASSKMRFKAFRVS | KMRFKAFRV |
| | | KMRFKAFRVSKSHSS | MRFKAFRVS |
| | | ILGVRVLFPSYSQSS | VRVLFPSYS |
| | | DTILGVRVLFPSYSQ | VRVLFPSYS |
| | | ILFFLLSTVLFAQET | ILFFLLSTV |
| | | TILGVRVLFPSYSQS | VRVLFPSYS |

|  |  |  |  |
|---|---|---|---|
|  |  | MRFKAFRVSKSHSSK | FRVSKSHSS |
|  |  | LGVRVLFPSYSQSSA | VRVLFPSYS |
|  |  | GDTILGVRVLFPSYS | LGVRVLFPS |
|  |  | VKDLRVLYDKLSVSI | VLYDKLSVS |
|  |  | KNFIFYVKDLRVLYD | FIFYVKDLR |
|  |  | YVKDLRVLYDKLSVS | LRVLYDKLS |
|  |  | KDLRVLYDKLSVSID | VLYDKLSVS |
|  |  | VLDFAELARDPSSTR | VLDFAELAR |
|  |  | DLRVLYDKLSVSIDS | VLYDKLSVS |
|  |  | LDFAELARDPSSTRL | LARDPSSTR |
|  |  | TFKRVLKLREKISIA | KRVLKLREK |
|  |  | GVRVLFPSYSQSSAM | VRVLFPSYS |
|  |  | NFIFYVKDLRVLYDK | FYVKDLRVL |
|  |  | ETFKRVLKLREKISI | KRVLKLREK |
|  |  | RFKAFRVSKSHSSKV | FRVSKSHSS |
|  |  | LRVLYDKLSVSIDSD | VLYDKLSVS |
|  |  | LGINNWSVLLTPSAR | INNWSVLLT |
|  |  | AELARDPSSTRLDLT | LARDPSSTR |
|  |  | DFAELARDPSSTRLD | LARDPSSTR |
|  |  | GINNWSVLLTPSARL | SVLLTPSAR |
|  | HLA-DRB3*0101 | None |  |
|  | HLA-DRB4*0101 | ADLIWSNPNYIPNIS | LIWSNPNYI |
|  |  | KGWADLIWSNPNYIP | LIWSNPNYI |
|  |  | WADLIWSNPNYIPNI | LIWSNPNYI |
|  |  | GWADLIWSNPNYIPN | LIWSNPNYI |
|  |  | FKGWADLIWSNPNYI | FKGWADLIW |
|  |  | DLIWSNPNYIPNISS | LIWSNPNYI |
|  |  | LIWSNPNYIPNISSR | LIWSNPNYI |
|  |  | TILGVRVLFPSYSQS | VRVLFPSYS |
|  |  | DTILGVRVLFPSYSQ | VRVLFPSYS |
|  |  | GDTILGVRVLFPSYS | LGVRVLFPS |
|  |  | ILGVRVLFPSYSQSS | VRVLFPSYS |
|  |  | RIIKDDVPNYPLASS | IKDDVPNYP |
|  |  | LGVRVLFPSYSQSSA | VRVLFPSYS |
|  |  | PLASSKMRFKAFRVS | LASSKMRFK |
|  |  | LASSKMRFKAFRVSK | MRFKAFRVS |
|  |  | ASSKMRFKAFRVSKS | MRFKAFRVS |
|  |  | SSKMRFKAFRVSKSH | MRFKAFRVS |
|  |  | SKMRFKAFRVSKSHS | MRFKAFRVS |
|  |  | ISSRIIKDDVPNYPL | IKDDVPNYP |
|  |  | NISSRIIKDDVPNYP | SRIIKDDVP |
|  |  | SSRIIKDDVPNYPLA | IKDDVPNYP |
|  |  | SRIIKDDVPNYPLAS | IKDDVPNYP |
|  |  | IIKDDVPNYPLASSK | IKDDVPNYP |
|  |  | IKDDVPNYPLASSKM | IKDDVPNYP |
|  | HLA-DRB5*0101 | DVPNYPLASSKMRFK | YPLASSKMR |
|  |  | VPNYPLASSKMRFKA | YPLASSKMR |
|  |  | PNYPLASSKMRFKAF | YPLASSKMR |
|  |  | KDDVPNYPLASSKMR | NYPLASSKM |
|  |  | DDVPNYPLASSKMRF | YPLASSKMR |
|  |  | ESLRKLKAHETFKRV | LKAHETFKR |
|  |  | LRKLKAHETFKRVLK | LKAHETFKR |
|  |  | SLRKLKAHETFKRVL | LKAHETFKR |
|  |  | RKLKAHETFKRVLKL | LKAHETFKR |

| | | | |
|---|---|---|---|
| | | TESLRKLKAHETFKR | LRKLKAHET |
| | | NYPLASSKMRFKAFR | YPLASSKMR |
| | | YPLASSKMRFKAFRV | YPLASSKMR |
| | | WSNPNYIPNISSRII | YIPNISSRI |
| | | IWSNPNYIPNISSRI | NYIPNISSR |
| | | SNPNYIPNISSRIIK | YIPNISSRI |
| | | NPNYIPNISSRIIKD | YIPNISSRI |
| | | | |
| BAD18055.1 FlaB protein [Borrelia garinii] Seq15 | HLA-DRB1*0101 | QPAKINTPASLSGSQ | INTPASLSG |
| | | PAKINTPASLSGSQA | INTPASLSG |
| | | MQPAKINTPASLSGS | INTPASLSG |
| | | AKINTPASLSGSQAS | INTPASLSG |
| | | ANLFSGEGAQAAQTA | FSGEGAQAA |
| | | VANLFSGEGAQAAQT | FSGEGAQAA |
| | | NLFSGEGAQAAQTAP | FSGEGAQAA |
| | | GMQPAKINTPASLSG | KINTPASLS |
| | | ANVANLFSGEGAQAA | VANLFSGEG |
| | | NVANLFSGEGAQAAQ | FSGEGAQAA |
| | | YNQMHMLSNKSASQN | MHMLSNKSA |
| | | EKVLVRMKELAVQSG | VRMKELAVQ |
| | | KVLVRMKELAVQSGN | MKELAVQSG |
| | | QAQYNQMHMLSNKSA | YNQMHMLSN |
| | | AQYNQMHMLSNKSAS | MHMLSNKSA |
| | | LFSGEGAQAAQTAPV | FSGEGAQAA |
| | | VLVRMKELAVQSGNG | MKELAVQSG |
| | | NQMHMLSNKSASQNV | MHMLSNKSA |
| | | AEELGMQPAKINTPA | LGMQPAKIN |
| | | FSGEGAQAAQTAPVQ | FSGEGAQAA |
| | | TAEELGMQPAKINTP | LGMQPAKIN |
| | | LVRMKELAVQSGNGT | MKELAVQSG |
| | | QYNQMHMLSNKSASQ | MHMLSNKSA |
| | | VRMKELAVQSGNGTY | MKELAVQSG |
| | | RTAEELGMQPAKINT | LGMQPAKIN |
| | | EELGMQPAKINTPAS | LGMQPAKIN |
| | | VRTAEELGMQPAKIN | ELGMQPAKI |
| | | INTPASLSGSQASWT | INTPASLSG |
| | | KINTPASLSGSQASW | INTPASLSG |
| | | VQQEGAQQPAPATAP | VQQEGAQQP |
| | | RMKELAVQSGNGTYS | MKELAVQSG |
| | | MKELAVQSGNGTYSD | MKELAVQSG |
| | | EGVQQEGAQQPAPAT | VQQEGAQQP |
| | | QMHMLSNKSASQNVR | MHMLSNKSA |
| | | MHMLSNKSASQNVRT | MHMLSNKSA |
| | | AIAVNIYAANVANLF | VNIYAANVA |
| | | LGMQPAKINTPASLS | LGMQPAKIN |
| | | IAVNIYAANVANLFS | YAANVANLF |
| | | ELGMQPAKINTPASL | LGMQPAKIN |
| | | QEGVQQEGAQQPAPA | VQQEGAQQP |
| | | AVNIYAANVANLFSG | YAANVANLF |
| | | PVQEGVQQEGAQQPA | VQQEGAQQP |
| | | APVQEGVQQEGAQQP | QEGVQQEGA |
| | | VQEGVQQEGAQQPAP | VQQEGAQQP |
| | | NIYAANVANLFSGEG | YAANVANLF |
| | | QEGAQQPAPATAPSQ | QQPAPATAP |
| | | VNIYAANVANLFSGE | YAANVANLF |
| | | QQEGAQQPAPATAPS | QQPAPATAP |

| | | | |
|---|---|---|---|
| | | GVQQEGAQQPAPATA | VQQEGAQQP |
| | | EGAQQPAPATAPSQG | QQPAPATAP |
| | | EAIAVNIYAANVANL | VNIYAANVA |
| | | VEKVLVRMKELAVQS | VRMKELAVQ |
| | | GAQQPAPATAPSQGG | QQPAPATAP |
| | | EVEKVLVRMKELAVQ | LVRMKELAV |
| | | AANVANLFSGEGAQA | VANLFSGEG |
| | | KELAVQSGNGTYSDA | VQSGNGTYS |
| | | DEAIAVNIYAANVAN | VNIYAANVA |
| | | PSQGGVNSPVNVTTT | VNSPVNVTT |
| | | DQAQYNQMHMLSNKS | YNQMHMLSN |
| | | SQGGVNSPVNVTTTV | VNSPVNVTT |
| | | QGGVNSPVNVTTTVD | VNSPVNVTT |
| | | APSQGGVNSPVNVTT | GVNSPVNVT |
| | | QDEAIAVNIYAANVA | AVNIYAANV |
| | | PASLSGSQASWTLRV | LSGSQASWT |
| | | ASLSGSQASWTLRVH | LSGSQASWT |
| | | IYAANVANLFSGEGA | VANLFSGEG |
| | | GGVNSPVNVTTTVDA | VNSPVNVTT |
| | | ELAVQSGNGTYSDAD | VQSGNGTYS |
| | | NTPASLSGSQASWTL | LSGSQASWT |
| | | GEGAQAAQTAPVQEG | AQAAQTAPV |
| | | LAVQSGNGTYSDADR | VQSGNGTYS |
| | | EGAQAAQTAPVQEGV | AQAAQTAPV |
| | | YAANVANLFSGEGAQ | VANLFSGEG |
| | | TPASLSGSQASWTLR | LSGSQASWT |
| | | AQQPAPATAPSQGGV | PATAPSQGG |
| | | ADQAQYNQMHMLSNK | YNQMHMLSN |
| | | HMLSNKSASQNVRTA | LSNKSASQN |
| | | IADQAQYNQMHMLSN | QYNQMHMLS |
| | | SGEGAQAAQTAPVQE | AQAAQTAPV |
| | | VNVTTTVDANTSLAK | VTTTVDANT |
| | | QQPAPATAPSQGGVN | PATAPSQGG |
| | | GVNSPVNVTTTVDAN | VNVTTTVDA |
| | | AQAAQTAPVQEGVQQ | AQTAPVQEG |
| | | GAQAAQTAPVQEGVQ | AQTAPVQEG |
| | | VNSPVNVTTTVDANT | VNVTTTVDA |
| | | SLSGSQASWTLRVHV | LSGSQASWT |
| | | LSGSQASWTLRVHVG | LSGSQASWT |
| | | SKAINFIQTTEGNLN | INFIQTTEG |
| | | NSPVNVTTTVDANTS | VNVTTTVDA |
| | | SPVNVTTTVDANTSL | VNVTTTVDA |
| | | NVTTTVDANTSLAKI | VDANTSLAK |
| | | KAINFIQTTEGNLNE | INFIQTTEG |
| | | QPAPATAPSQGGVNS | PATAPSQGG |
| | | MLSNKSASQNVRTAE | LSNKSASQN |
| | | PAPATAPSQGGVNSP | PATAPSQGG |
| | | VTTTVDANTSLAKIE | VDANTSLAK |
| | | PVNVTTTVDANTSLA | VNVTTTVDA |
| | | QAAQTAPVQEGVQQE | AQTAPVQEG |
| | HLA-DRB1*0301 | None | |
| | HLA-DRB1*0401 | QYNQMHMLSNKSASQ | QMHMLSNKS |
| | | YNQMHMLSNKSASQN | QMHMLSNKS |
| | | IAVNIYAANVANLFS | YAANVANLF |
| | | QAQYNQMHMLSNKSA | QMHMLSNKS |
| | | NQMHMLSNKSASQNV | MLSNKSASQ |

| | | AIAVNIYAANVANLF | NIYAANVAN |
|---|---|---|---|
| | | NIYAANVANLFSGEG | YAANVANLF |
| | | AVNIYAANVANLFSG | YAANVANLF |
| | | AQYNQMHMLSNKSAS | QMHMLSNKS |
| | | VNIYAANVANLFSGE | YAANVANLF |
| | | QMHMLSNKSASQNVR | MLSNKSASQ |
| | | GVNSPVNVTTTVDAN | VNVTTTVDA |
| | | GGVNSPVNVTTTVDA | VNSPVNVTT |
| | | NSPVNVTTTVDANTS | VNVTTTVDA |
| | | VNSPVNVTTTVDANT | VNVTTTVDA |
| | HLA-DRB1*0404 | ANVANLFSGEGAQAA | VANLFSGEG |
| | | AANVANLFSGEGAQA | VANLFSGEG |
| | | NIYAANVANLFSGEG | YAANVANLF |
| | | IYAANVANLFSGEGA | VANLFSGEG |
| | | YAANVANLFSGEGAQ | VANLFSGEG |
| | | YNQMHMLSNKSASQN | MLSNKSASQ |
| | | QYNQMHMLSNKSASQ | QMHMLSNKS |
| | | NVANLFSGEGAQAAQ | VANLFSGEG |
| | | VANLFSGEGAQAAQT | VANLFSGEG |
| | | NQMHMLSNKSASQNV | MLSNKSASQ |
| | | QMHMLSNKSASQNVR | MLSNKSASQ |
| | | VNVTTTVDANTSLAK | TVDANTSLA |
| | | PVNVTTTVDANTSLA | VNVTTTVDA |
| | | AQYNQMHMLSNKSAS | QMHMLSNKS |
| | | QAQYNQMHMLSNKSA | QMHMLSNKS |
| | | DQAQYNQMHMLSNKS | YNQMHMLSN |
| | HLA-DRB1*0405 | EELGMQPAKINTPAS | LGMQPAKIN |
| | | LGMQPAKINTPASLS | PAKINTPAS |
| | | GGVNSPVNVTTTVDA | PVNVTTTVD |
| | | GVNSPVNVTTTVDAN | VNVTTTVDA |
| | | VNSPVNVTTTVDANT | VNVTTTVDA |
| | | ELGMQPAKINTPASL | PAKINTPAS |
| | | NSPVNVTTTVDANTS | VNVTTTVDA |
| | HLA-DRB1*0701 | GGVNSPVNVTTTVDA | PVNVTTTVD |
| | | GVNSPVNVTTTVDAN | VNVTTTVDA |
| | HLA-DRB1*0802 | None | |
| | HLA-DRB1*0901 | IAVNIYAANVANLFS | VNIYAANVA |
| | | AIAVNIYAANVANLF | VNIYAANVA |
| | | EAIAVNIYAANVANL | VNIYAANVA |
| | | AVNIYAANVANLFSG | IYAANVANL |
| | | VNIYAANVANLFSGE | IYAANVANL |
| | HLA-DRB1*1101 | None | |
| | HLA-DRB1*1302 | NTSLAKIENAIRMIS | AKIENAIRM |
| | | TSLAKIENAIRMISD | IENAIRMIS |
| | | SLAKIENAIRMISDQ | IENAIRMIS |
| | | RMKELAVQSGNGTYS | LAVQSGNGT |
| | | AIAVNIYAANVANLF | VNIYAANVA |
| | | IAVNIYAANVANLFS | VNIYAANVA |
| | | MKELAVQSGNGTYSD | LAVQSGNGT |
| | | KELAVQSGNGTYSDA | LAVQSGNGT |
| | | LVRMKELAVQSGNGT | MKELAVQSG |
| | | VRMKELAVQSGNGTY | LAVQSGNGT |

| | | | |
|---|---|---|---|
| | | AVNIYAANVANLFSG | YAANVANLF |
| | | VNIYAANVANLFSGE | YAANVANLF |
| | | SQGGVNSPVNVTTTV | GVNSPVNVT |
| | | QGGVNSPVNVTTTVD | GVNSPVNVT |
| | | GGVNSPVNVTTTVDA | GVNSPVNVT |
| | | EAIAVNIYAANVANL | VNIYAANVA |
| | | PSQGGVNSPVNVTTT | GVNSPVNVT |
| | | APSQGGVNSPVNVTT | GVNSPVNVT |
| | | QDEAIAVNIYAANVA | IAVNIYAAN |
| | | DEAIAVNIYAANVAN | VNIYAANVA |
| | | GVNSPVNVTTTVDAN | GVNSPVNVT |
| | | ELAVQSGNGTYSDAD | LAVQSGNGT |
| | | LAVQSGNGTYSDADR | LAVQSGNGT |
| | | NIYAANVANLFSGEG | YAANVANLF |
| | | SKAINFIQTTEGNLN | INFIQTTEG |
| | HLA-DRB1*1501 | EKVLVRMKELAVQSG | LVRMKELAV |
| | | KVLVRMKELAVQSGN | LVRMKELAV |
| | | VEKVLVRMKELAVQS | LVRMKELAV |
| | | EVEKVLVRMKELAVQ | LVRMKELAV |
| | | VLVRMKELAVQSGNG | VRMKELAVQ |
| | | LVRMKELAVQSGNGT | VRMKELAVQ |
| | | NEVEKVLVRMKELAV | VLVRMKELA |
| | | QYNQMHMLSNKSASQ | MHMLSNKSA |
| | | YNQMHMLSNKSASQN | MHMLSNKSA |
| | | AQYNQMHMLSNKSAS | MHMLSNKSA |
| | | VRMKELAVQSGNGTY | VRMKELAVQ |
| | | QAQYNQMHMLSNKSA | QMHMLSNKS |
| | | NQMHMLSNKSASQNV | MHMLSNKSA |
| | | QASWTLRVHVGANQD | LRVHVGANQ |
| | | SQASWTLRVHVGANQ | QASWTLRVH |
| | HLA-DRB3*0101 | None | |
| | HLA-DRB4*0101 | EVEKVLVRMKELAVQ | VEKVLVRMK |
| | | VEKVLVRMKELAVQS | VRMKELAVQ |
| | | EKVLVRMKELAVQSG | VRMKELAVQ |
| | | KVLVRMKELAVQSGN | VRMKELAVQ |
| | | VLVRMKELAVQSGNG | VRMKELAVQ |
| | | VRMKELAVQSGNGTY | VRMKELAVQ |
| | | LVRMKELAVQSGNGT | VRMKELAVQ |
| | HLA-DRB5*0101 | NTSLAKIENAIRMIS | AKIENAIRM |
| | | TSLAKIENAIRMISD | AKIENAIRM |
| | | DANTSLAKIENAIRM | LAKIENAIR |
| | | ANTSLAKIENAIRMI | AKIENAIRM |
| | | SLAKIENAIRMISDQ | AKIENAIRM |
| | | | |
| AAU07005.1\| flagellar filament 41 kDa core protein [Borrelia garinii PBi]<br><br>Seq16 | HLA-DRB1*0101 | TTNSILTQSAMAMIA | ILTQSAMAM |
| | | TNSILTQSAMAMIAQ | ILTQSAMAM |
| | | NSILTQSAMAMIAQA | ILTQSAMAM |
| | | STTNSILTQSAMAMI | ILTQSAMAM |
| | | ASTTNSILTQSAMAM | TNSILTQSA |
| | | QPAKINTPASLSGSQ | INTPASLSG |
| | | PAKINTPASLSGSQA | INTPASLSG |
| | | SILTQSAMAMIAQAN | ILTQSAMAM |

| | | | |
|---|---|---|---|
| | | MQPAKINTPASLSGS | INTPASLSG |
| | | AKINTPASLSGSQAS | INTPASLSG |
| | | ILTQSAMAMIAQANQ | ILTQSAMAM |
| | | GMQPAKINTPASLSG | KINTPASLS |
| | | NAIRMISDQRANLGA | IRMISDQRA |
| | | ANVANLFSGEGSQAA | VANLFSGEG |
| | | ANLFSGEGSQAAQTA | FSGEGSQAA |
| | | ENAIRMISDQRANLG | IRMISDQRA |
| | | VANLFSGEGSQAAQT | FSGEGSQAA |
| | | NLFSGEGSQAAQTAP | FSGEGSQAA |
| | | NVANLFSGEGSQAAQ | FSGEGSQAA |
| | | YNQMHMLSNKSASQN | MHMLSNKSA |
| | | EKVLVRMKELAVQSG | VRMKELAVQ |
| | | KVLVRMKELAVQSGN | MKELAVQSG |
| | | QAQYNQMHMLSNKSA | YNQMHMLSN |
| | | AQYNQMHMLSNKSAS | MHMLSNKSA |
| | | NQMHMLSNKSASQNV | MHMLSNKSA |
| | | AEELGMQPAKINTPA | LGMQPAKIN |
| | | VLVRMKELAVQSGNG | MKELAVQSG |
| | | TAEELGMQPAKINTP | LGMQPAKIN |
| | | QYNQMHMLSNKSASQ | MHMLSNKSA |
| | | AKIENAIRMISDQRA | IENAIRMIS |
| | | LVRMKELAVQSGNGT | MKELAVQSG |
| | | RTAEELGMQPAKINT | LGMQPAKIN |
| | | EELGMQPAKINTPAS | LGMQPAKIN |
| | | VRMKELAVQSGNGTY | MKELAVQSG |
| | | VRTAEELGMQPAKIN | ELGMQPAKI |
| | | IENAIRMISDQRANL | IRMISDQRA |
| | | KIENAIRMISDQRAN | IRMISDQRA |
| | | INTPASLSGSQASWT | INTPASLSG |
| | | EYAIENLKASYAQIK | IENLKASYA |
| | | LFSGEGSQAAQTAPV | FSGEGSQAA |
| | | YAIENLKASYAQIKD | IENLKASYA |
| | | FSGEGSQAAQTAPVQ | FSGEGSQAA |
| | | KINTPASLSGSQASW | INTPASLSG |
| | | AIRMISDQRANLGAF | IRMISDQRA |
| | | IRMISDQRANLGAFQ | IRMISDQRA |
| | | RMKELAVQSGNGTYS | MKELAVQSG |
| | | MKELAVQSGNGTYSD | MKELAVQSG |
| | | STEYAIENLKASYAQ | IENLKASYA |
| | | DSTEYAIENLKASYA | YAIENLKAS |
| | | TEYAIENLKASYAQI | IENLKASYA |
| | | QMHMLSNKSASQNVR | MHMLSNKSA |
| | | MHMLSNKSASQNVRT | MHMLSNKSA |
| | | TQSAMAMIAQANQVP | TQSAMAMIA |
| | | AIAVNIYAANVANLF | VNIYAANVA |
| | | LGMQPAKINTPASLS | LGMQPAKIN |
| | | IAVNIYAANVANLFS | YAANVANLF |
| | | AIENLKASYAQIKDA | LKASYAQIK |
| | | IENLKASYAQIKDAT | LKASYAQIK |
| | | ELGMQPAKINTPASL | LGMQPAKIN |
| | | LTQSAMAMIAQANQV | AMAMIAQAN |
| | | QSAMAMIAQANQVPQ | MIAQANQVP |
| | | AVNIYAANVANLFSG | YAANVANLF |
| | | NIYAANVANLFSGEG | YAANVANLF |
| | | QEGAQQPAPATAPSQ | QQPAPATAP |
| | | ATMTDEVVASTTNSI | MTDEVVAST |

| | | | |
|---|---|---|---|
| | | VNIYAANVANLFSGE | YAANVANLF |
| | | QQEGAQQPAPATAPS | QQPAPATAP |
| | | AQQEGAQQPAPATAP | AQQPAPATA |
| | | MTDEVVASTTNSILT | VVASTTNSI |
| | | TMTDEVVASTTNSIL | VVASTTNSI |
| | | TDEVVASTTNSILTQ | VVASTTNSI |
| | | EGAQQPAPATAPSQG | QQPAPATAP |
| | | AMAMIAQANVPQYV | MIAQANQVP |
| | | EAIAVNIYAANVANL | VNIYAANVA |
| | | DEVVASTTNSILTQS | VVASTTNSI |
| | | ENLKASYAQIKDATM | LKASYAQIK |
| | | VEKVLVRMKELAVQS | VRMKELAVQ |
| | | SAMAMIAQANQVPQY | MIAQANQVP |
| | | GAQQPAPATAPSQGG | QQPAPATAP |
| | | EVEKVLVRMKELAVQ | LVRMKELAV |
| | | NAQIRGLSQASRNTS | IRGLSQASR |
| | | KELAVQSGNGTYSDA | VQSGNGTYS |
| | | NLKASYAQIKDATMT | LKASYAQIK |
| | | LKASYAQIKDATMTD | LKASYAQIK |
| | | INAQIRGLSQASRNT | IRGLSQASR |
| | | AQIRGLSQASRNTSK | IRGLSQASR |
| | | DEAIAVNIYAANVAN | VNIYAANVA |
| | | PSQGGVNSPVNVTTT | VNSPVNVTT |
| | | DQAQYNQMHMLSNKS | YNQMHMLSN |
| | | SQGGVNSPVNVTTTV | VNSPVNVTT |
| | | QGGVNSPVNVTTTVD | VNSPVNVTT |
| | | APSQGGVNSPVNVTT | GVNSPVNVT |
| | | QDEAIAVNIYAANVA | AVNIYAANV |
| | | PASLSGSQASWTLRV | LSGSQASWT |
| | | ASLSGSQASWTLRVH | LSGSQASWT |
| | | AANVANLFSGEGSQA | VANLFSGEG |
| | | MAMIAQANQVPQYVL | MIAQANQVP |
| | | IYAANVANLFSGEGS | VANLFSGEG |
| | | GGVNSPVNVTTTVDA | VNSPVNVTT |
| | | ELAVQSGNGTYSDAD | VQSGNGTYS |
| | | NTPASLSGSQASWTL | LSGSQASWT |
| | | LAVQSGNGTYSDADR | VQSGNGTYS |
| | | NNSINAANLSKTQEK | INAANLSKT |
| | | RNNSINAANLSKTQE | INAANLSKT |
| | | SRNNSINAANLSKTQ | INAANLSKT |
| | | NSINAANLSKTQEKL | INAANLSKT |
| | | YAANVANLFSGEGSQ | VANLFSGEG |
| | | ASRNNSINAANLSKT | NSINAANLS |
| | | TPASLSGSQASWTLR | LSGSQASWT |
| | | EVVASTTNSILTQSA | VVASTTNSI |
| | | KINAQIRGLSQASRN | IRGLSQASR |
| | | GKINAQIRGLSQASR | QIRGLSQAS |
| | | VVASTTNSILTQSAM | VVASTTNSI |
| | | AQQPAPATAPSQGGV | PATAPSQGG |
| | | EGSQAAQTAPVQEGA | AQTAPVQEG |
| | | GEGSQAAQTAPVQEG | SQAAQTAPV |
| | | ADQAQYNQMHMLSNK | YNQMHMLSN |
| | | HMLSNKSASQNVRTA | LSNKSASQN |
| | | IADQAQYNQMHMLSN | QYNQMHMLS |
| | | VNVTTTVDANTSLAK | VTTTVDANT |
| | | KASYAQIKDATMTDE | AQIKDATMT |
| | | QIRGLSQASRNTSKA | IRGLSQASR |

| | | | |
|---|---|---|---|
| | | ASYAQIKDATMTDEV | AQIKDATMT |
| | | QQPAPATAPSQGGVN | PATAPSQGG |
| | | AMIAQANQVPQYVLS | MIAQANQVP |
| | | GVNSPVNVTTTVDAN | VNVTTTVDA |
| | | IRGLSQASRNTSKAI | IRGLSQASR |
| | | RMISDQRANLGAFQN | ISDQRANLG |
| | | GAQQEGAQQPAPATA | GAQQPAPAT |
| | | MIAQANQVPQYVLSL | QANQVPQYV |
| | | GSQAAQTAPVQEGAQ | AQTAPVQEG |
| | | SQAAQTAPVQEGAQQ | AQTAPVQEG |
| | | DATMTDEVVASTTNS | MTDEVVAST |
| | | VNSPVNVTTTVDANT | VNVTTTVDA |
| | | SLSGSQASWTLRVHV | LSGSQASWT |
| | | LSGSQASWTLRVHVG | LSGSQASWT |
| | | SKAINFIQTTEGNLN | INFIQTTEG |
| | | MIINHNTSAINASRN | INHNTSAIN |
| | | SYAQIKDATMTDEVV | AQIKDATMT |
| | | NSPVNVTTTVDANTS | VNVTTTVDA |
| | | SPVNVTTTVDANTSL | VNVTTTVDA |
| | | NVTTTVDANTSLAKI | VDANTSLAK |
| | | KAINFIQTTEGNLNE | INFIQTTEG |
| | | SGEGSQAAQTAPVQE | SQAAQTAPV |
| | | QPAPATAPSQGGVNS | PATAPSQGG |
| | | MLSNKSASQNVRTAE | LSNKSASQN |
| | | IKDATMTDEVVASTT | MTDEVVAST |
| | | KDATMTDEVVASTTN | MTDEVVAST |
| | | PAPATAPSQGGVNSP | PATAPSQGG |
| | | VTTTVDANTSLAKIE | VDANTSLAK |
| | | QIKDATMTDEVVAST | DATMTDEVV |
| | | PVNVTTTVDANTSLA | VNVTTTVDA |
| | | QAAQTAPVQEGAQQE | AQTAPVQEG |
| | | EGAQQEGAQQPAPAT | AQQEGAQQP |
| | HLA-DRB1*0301 | None | |
| | HLA-DRB1*0401 | QYNQMHMLSNKSASQ | QMHMLSNKS |
| | | YNQMHMLSNKSASQN | QMHMLSNKS |
| | | IAVNIYAANVANLFS | YAANVANLF |
| | | QAQYNQMHMLSNKSA | QMHMLSNKS |
| | | NQMHMLSNKSASQNV | MLSNKSASQ |
| | | AIAVNIYAANVANLF | NIYAANVAN |
| | | NIYAANVANLFSGEG | YAANVANLF |
| | | AVNIYAANVANLFSG | YAANVANLF |
| | | AQYNQMHMLSNKSAS | QMHMLSNKS |
| | | VNIYAANVANLFSGE | YAANVANLF |
| | | QMHMLSNKSASQNVR | MLSNKSASQ |
| | | GVNSPVNVTTTVDAN | VNVTTTVDA |
| | | GGVNSPVNVTTTVDA | VNSPVNVTT |
| | | NSPVNVTTTVDANTS | VNVTTTVDA |
| | | VNSPVNVTTTVDANT | VNVTTTVDA |
| | HLA-DRB1*0404 | ANVANLFSGEGSQAA | VANLFSGEG |
| | | AANVANLFSGEGSQA | VANLFSGEG |
| | | YAANVANLFSGEGSQ | VANLFSGEG |
| | | NIYAANVANLFSGEG | YAANVANLF |
| | | IYAANVANLFSGEGS | VANLFSGEG |
| | | YNQMHMLSNKSASQN | MLSNKSASQ |
| | | QYNQMHMLSNKSASQ | QMHMLSNKS |

| | | | |
|---|---|---|---|
| | | NVANLFSGEGSQAAQ | VANLFSGEG |
| | | VANLFSGEGSQAAQT | VANLFSGEG |
| | | ENAIRMISDQRANLG | AIRMISDQR |
| | | NQMHMLSNKSASQNV | MLSNKSASQ |
| | | NAIRMISDQRANLGA | ISDQRANLG |
| | | QMHMLSNKSASQNVR | MLSNKSASQ |
| | | AIRMISDQRANLGAF | ISDQRANLG |
| | | VNVTTTVDANTSLAK | TVDANTSLA |
| | | PVNVTTTVDANTSLA | VNVTTTVDA |
| | | IRMISDQRANLGAFQ | ISDQRANLG |
| | | AQYNQMHMLSNKSAS | QMHMLSNKS |
| | | QAQYNQMHMLSNKSA | QMHMLSNKS |
| | | DQAQYNQMHMLSNKS | YNQMHMLSN |
| | | RMISDQRANLGAFQN | ISDQRANLG |
| | HLA-DRB1*0405 | EELGMQPAKINTPAS | LGMQPAKIN |
| | | LGMQPAKINTPASLS | PAKINTPAS |
| | | GGVNSPVNVTTTVDA | PVNVTTTVD |
| | | GVNSPVNVTTTVDAN | VNVTTTVDA |
| | | VNSPVNVTTTVDANT | VNVTTTVDA |
| | | ELGMQPAKINTPASL | PAKINTPAS |
| | | NSPVNVTTTVDANTS | VNVTTTVDA |
| | HLA-DRB1*0701 | TMTDEVVASTTNSIL | VVASTTNSI |
| | | MTDEVVASTTNSILT | VVASTTNSI |
| | | TDEVVASTTNSILTQ | VVASTTNSI |
| | | DEVVASTTNSILTQS | VVASTTNSI |
| | | ATMTDEVVASTTNSI | TDEVVASTT |
| | | EVVASTTNSILTQSA | VASTTNSIL |
| | | NHNTSAINASRNNSI | TSAINASRN |
| | | HNTSAINASRNNSIN | INASRNNSI |
| | | TSAINASRNNSINAA | INASRNNSI |
| | | NTSAINASRNNSINA | INASRNNSI |
| | | SAINASRNNSINAAN | INASRNNSI |
| | | VVASTTNSILTQSAM | VVASTTNSI |
| | | GGVNSPVNVTTTVDA | PVNVTTTVD |
| | | GVNSPVNVTTTVDAN | VNVTTTVDA |
| | | MIINHNTSAINASRN | IINHNTSAI |
| | HLA-DRB1*0802 | None | |
| | HLA-DRB1*0901 | IAVNIYAANVANLFS | VNIYAANVA |
| | | AIAVNIYAANVANLF | VNIYAANVA |
| | | TTNSILTQSAMAMIA | LTQSAMAMI |
| | | TNSILTQSAMAMIAQ | LTQSAMAMI |
| | | NSILTQSAMAMIAQA | LTQSAMAMI |
| | | SILTQSAMAMIAQAN | LTQSAMAMI |
| | | STTNSILTQSAMAMI | ILTQSAMAM |
| | | EAIAVNIYAANVANL | VNIYAANVA |
| | | AVNIYAANVANLFSG | IYAANVANL |
| | | VNIYAANVANLFSGE | IYAANVANL |
| | HLA-DRB1*1101 | None | |
| | HLA-DRB1*1302 | NTSLAKIENAIRMIS | AKIENAIRM |
| | | TSLAKIENAIRMISD | IENAIRMIS |
| | | SLAKIENAIRMISDQ | IENAIRMIS |
| | | RMKELAVQSGNGTYS | LAVQSGNGT |

|  |  | AIAVNIYAANVANLF | VNIYAANVA |
|---|---|---|---|
|  |  | IAVNIYAANVANLFS | VNIYAANVA |
|  |  | MKELAVQSGNGTYSD | LAVQSGNGT |
|  |  | KELAVQSGNGTYSDA | LAVQSGNGT |
|  |  | LVRMKELAVQSGNGT | MKELAVQSG |
|  |  | VRMKELAVQSGNGTY | LAVQSGNGT |
|  |  | AVNIYAANVANLFSG | YAANVANLF |
|  |  | VNIYAANVANLFSGE | YAANVANLF |
|  |  | SQGGVNSPVNVTTTV | GVNSPVNVT |
|  |  | QGGVNSPVNVTTTVD | GVNSPVNVT |
|  |  | GGVNSPVNVTTTVDA | GVNSPVNVT |
|  |  | EAIAVNIYAANVANL | VNIYAANVA |
|  |  | PSQGGVNSPVNVTTT | GVNSPVNVT |
|  |  | APSQGGVNSPVNVTT | GVNSPVNVT |
|  |  | QDEAIAVNIYAANVA | IAVNIYAAN |
|  |  | DEAIAVNIYAANVAN | VNIYAANVA |
|  |  | GVNSPVNVTTTVDAN | GVNSPVNVT |
|  |  | ELAVQSGNGTYSDAD | LAVQSGNGT |
|  |  | LAVQSGNGTYSDADR | LAVQSGNGT |
|  |  | NIYAANVANLFSGEG | YAANVANLF |
|  |  | SKAINFIQTTEGNLN | INFIQTTEG |
|  | HLA-DRB1*1501 | EKVLVRMKELAVQSG | LVRMKELAV |
|  |  | KVLVRMKELAVQSGN | LVRMKELAV |
|  |  | VEKVLVRMKELAVQS | LVRMKELAV |
|  |  | EVEKVLVRMKELAVQ | LVRMKELAV |
|  |  | VLVRMKELAVQSGNG | VRMKELAVQ |
|  |  | LVRMKELAVQSGNGT | VRMKELAVQ |
|  |  | INAQIRGLSQASRNT | IRGLSQASR |
|  |  | GKINAQIRGLSQASR | QIRGLSQAS |
|  |  | NAQIRGLSQASRNTS | IRGLSQASR |
|  |  | KINAQIRGLSQASRN | IRGLSQASR |
|  |  | AQIRGLSQASRNTSK | IRGLSQASR |
|  |  | NEVEKVLVRMKELAV | VLVRMKELA |
|  |  | KIENAIRMISDQRAN | AIRMISDQR |
|  |  | IENAIRMISDQRANL | AIRMISDQR |
|  |  | AKIENAIRMISDQRA | AIRMISDQR |
|  |  | ENAIRMISDQRANLG | AIRMISDQR |
|  |  | LAKIENAIRMISDQR | LAKIENAIR |
|  |  | QYNQMHMLSNKSASQ | MHMLSNKSA |
|  |  | QIRGLSQASRNTSKA | IRGLSQASR |
|  |  | YNQMHMLSNKSASQN | MHMLSNKSA |
|  |  | IRGLSQASRNTSKAI | IRGLSQASR |
|  |  | AQYNQMHMLSNKSAS | MHMLSNKSA |
|  |  | VRMKELAVQSGNGTY | VRMKELAVQ |
|  |  | QAQYNQMHMLSNKSA | QMHMLSNKS |
|  |  | AIRMISDQRANLGAF | AIRMISDQR |
|  |  | NQMHMLSNKSASQNV | MHMLSNKSA |
|  |  | QASWTLRVHVGANQD | LRVHVGANQ |
|  |  | NAIRMISDQRANLGA | AIRMISDQR |
|  |  | SQASWTLRVHVGANQ | QASWTLRVH |
|  | HLA-DRB3*0101 | None |  |
|  | HLA-DRB4*0101 | EVEKVLVRMKELAVQ | VEKVLVRMK |
|  |  | VEKVLVRMKELAVQS | VRMKELAVQ |
|  |  | EKVLVRMKELAVQSG | VRMKELAVQ |
|  |  | KVLVRMKELAVQSGN | VRMKELAVQ |

|  |  | | |
|---|---|---|---|
|  |  | VLVRMKELAVQSGNG | VRMKELAVQ |
|  |  | ENAIRMISDQRANLG | IRMISDQRA |
|  |  | NAIRMISDQRANLGA | IRMISDQRA |
|  |  | VRMKELAVQSGNGTY | VRMKELAVQ |
|  |  | LVRMKELAVQSGNGT | VRMKELAVQ |
|  |  | AIRMISDQRANLGAF | ISDQRANLG |
|  |  | IRMISDQRANLGAFQ | ISDQRANLG |
|  | HLA-DRB5*0101 | NTSLAKIENAIRMIS | AKIENAIRM |
|  |  | TSLAKIENAIRMISD | AKIENAIRM |
|  |  | DANTSLAKIENAIRM | LAKIENAIR |
|  |  | ANTSLAKIENAIRMI | AKIENAIRM |
|  |  | SLAKIENAIRMISDQ | AKIENAIRM |
|  |  | | |
| 1L8W\|A Chain A, Crystal Structure Of Lyme Disease Variable Surface Antigen VlsE1 Of Borrelia Burgdorferi<br><br>Seq17 | HLA-DRB1*0101 | DVFTSFGGLVAEAFG | FGGLVAEAF |
|  |  | SDVKTYFTTVAAKLE | YFTTVAAKL |
|  |  | LDVFTSFGGLVAEAF | FTSFGGLVA |
|  |  | VKTYFTTVAAKLEKT | YFTTVAAKL |
|  |  | KTYFTTVAAKLEKTK | FTTVAAKLE |
|  |  | KSDVKTYFTTVAAKL | VKTYFTTVA |
|  |  | DVKTYFTTVAAKLEK | YFTTVAAKL |
|  |  | FTSFGGLVAEAFGFK | FGGLVAEAF |
|  |  | VKAVKTAEGASSGTA | VKTAEGASS |
|  |  | VFTSFGGLVAEAFGF | FGGLVAEAF |
|  |  | KAVKTAEGASSGTAA | VKTAEGASS |
|  |  | KLVKAVKTAEGASSG | VKTAEGASS |
|  |  | DKLVKAVKTAEGASS | VKAVKTAEG |
|  |  | LVKAVKTAEGASSGT | VKTAEGASS |
|  |  | TYFTTVAAKLEKTKT | FTTVAAKLE |
|  |  | TSFGGLVAEAFGFKS | FGGLVAEAF |
|  |  | KKSDVKTYFTTVAAK | VKTYFTTVA |
|  |  | AAKVADKASVKGIAK | VADKASVKG |
|  |  | ADAAKVADKASVKGI | VADKASVKG |
|  |  | PKKSDVKTYFTTVAA | VKTYFTTVA |
|  |  | DADAAKVADKASVKG | AAKVADKAS |
|  |  | DAAKVADKASVKGIA | VADKASVKG |
|  |  | AKVADKASVKGIAKG | VADKASVKG |
|  |  | DPKKSDVKTYFTTVA | KSDVKTYFT |
|  |  | AVKTAEGASSGTAAI | VKTAEGASS |
|  |  | VKTAEGASSGTAAIG | VKTAEGASS |
|  |  | YFTTVAAKLEKTKTD | FTTVAAKLE |
|  |  | GEVVADADAAKVADK | VADADAAKV |
|  |  | EVVADADAAKVADKA | VADADAAKV |
|  |  | IGEVVADADAAKVAD | VADADAAKV |
|  |  | AIGEVVADADAAKVA | VADADAAKV |
|  |  | AAIGEVVADADAAKV | VVADADAAK |
|  |  | LLDKLVKAVKTAEGA | VKAVKTAEG |
|  |  | ELLDKLVKAVKTAEG | DKLVKAVKT |
|  |  | LDKLVKAVKTAEGAS | VKAVKTAEG |
|  |  | FTTVAAKLEKTKTDL | FTTVAAKLE |
|  |  | SFGGLVAEAFGFKSD | FGGLVAEAF |
|  |  | FGGLVAEAFGFKSDP | FGGLVAEAF |
|  |  | PTNKFYQSVIQLGNG | FYQSVIQLG |
|  |  | NKFYQSVIQLGNGFL | FYQSVIQLG |
|  |  | DPTNKFYQSVIQLGN | FYQSVIQLG |
|  |  | TNKFYQSVIQLGNGF | FYQSVIQLG |
|  |  | NGFLDVFTSFGGLVA | FLDVFTSFG |

| | | | |
|---|---|---|---|
| | | KVADKASVKGIAKGI | VADKASVKG |
| | | VADKASVKGIAKGIK | VADKASVKG |
| | | DDPTNKFYQSVIQLG | NKFYQSVIQ |
| | | GFLDVFTSFGGLVAE | FTSFGGLVA |
| | | FLDVFTSFGGLVAEA | FTSFGGLVA |
| | | VVADADAAKVADKAS | VADADAAKV |
| | | VADADAAKVADKASV | VADADAAKV |
| | | VSELLDKLVKAVKTA | LLDKLVKAV |
| | | SELLDKLVKAVKTAE | LLDKLVKAV |
| | | EVSELLDKLVKAVKT | LLDKLVKAV |
| | | KTDLNSLPKEKSDIS | LNSLPKEKS |
| | | TDLNSLPKEKSDISS | LNSLPKEKS |
| | HLA-DRB1*0301 | None | |
| | HLA-DRB1*0401 | None | |
| | HLA-DRB1*0404 | NGFLDVFTSFGGLVA | FLDVFTSFG |
| | | QLGNGFLDVFTSFGG | FLDVFTSFG |
| | | GNGFLDVFTSFGGLV | FLDVFTSFG |
| | | IQLGNGFLDVFTSFG | QLGNGFLDV |
| | | LGNGFLDVFTSFGGL | FLDVFTSFG |
| | | GFLDVFTSFGGLVAE | FLDVFTSFG |
| | | FLDVFTSFGGLVAEA | FLDVFTSFG |
| | HLA-DRB1*0405 | VAEAFGFKSDPKKSD | AFGFKSDPK |
| | | AEAFGFKSDPKKSDV | FKSDPKKSD |
| | | EAFGFKSDPKKSDVK | FKSDPKKSD |
| | | AFGFKSDPKKSDVKT | FKSDPKKSD |
| | | FGFKSDPKKSDVKTY | FKSDPKKSD |
| | | DPTNKFYQSVIQLGN | TNKFYQSVI |
| | | PTNKFYQSVIQLGNG | YQSVIQLGN |
| | | KFYQSVIQLGNGFLD | YQSVIQLGN |
| | | TNKFYQSVIQLGNGF | YQSVIQLGN |
| | | SDVKTYFTTVAAKLE | VKTYFTTVA |
| | HLA-DRB1*0701 | KSDVKTYFTTVAAKL | DVKTYFTTV |
| | | SDVKTYFTTVAAKLE | YFTTVAAKL |
| | | DVKTYFTTVAAKLEK | YFTTVAAKL |
| | | VKTYFTTVAAKLEKT | YFTTVAAKL |
| | | KTYFTTVAAKLEKTK | YFTTVAAKL |
| | HLA-DRB1*0802 | None | |
| | HLA-DRB1*0901 | VKTYFTTVAAKLEKT | YFTTVAAKL |
| | | SDVKTYFTTVAAKLE | YFTTVAAKL |
| | | DVKTYFTTVAAKLEK | YFTTVAAKL |
| | | KTYFTTVAAKLEKTK | YFTTVAAKL |
| | | NKFYQSVIQLGNGFL | FYQSVIQLG |
| | | PTNKFYQSVIQLGNG | FYQSVIQLG |
| | | DPTNKFYQSVIQLGN | FYQSVIQLG |
| | | DDPTNKFYQSVIQLG | NKFYQSVIQ |
| | HLA-DRB1*1101 | None | |
| | HLA-DRB1*1302 | None | |
| | HLA-DRB1*1501 | GLVAEAFGFKSDPKK | AFGFKSDPK |
| | | LVAEAFGFKSDPKKS | AFGFKSDPK |
| | | VAEAFGFKSDPKKSD | AFGFKSDPK |
| | HLA-DRB3*0101 | None | |
| | HLA-DRB4*0101 | None | |
| | HLA-DRB5*0101 | None | |
| | | | |
| CAA57807.1 Associated protein A | HLA-DRB1*0101 | IFLFLFVVSLSANIE | FVVSLSANI |
| | | FLFLFVVSLSANIEE | VVSLSANIE |

| (BapA)[Borrelia burgdorferi] Seq18 | | LFLFVVSLSANIEEN | FVVSLSANI |
|---|---|---|---|
| | | FLFVVSLSANIEENY | FVVSLSANI |
| | | LIFLFLFVVSLSANI | FLFVVSLSA |
| | | EAIEYLIKIKISTDS | IEYLIKIKI |
| | | AIEYLIKIKISTDSI | IKIKISTDS |
| | | IEYLIKIKISTDSIF | IKIKISTDS |
| | | EYLIKIKISTDSIFL | IKIKISTDS |
| | | YLIKIKISTDSIFLS | IKIKISTDS |
| | | LFVVSLSANIEENYT | VVSLSANIE |
| | | LLIFLFLFVVSLSAN | FLFVVSLSA |
| | | SLLIFLFLFVVSLSA | FLFLFVVSL |
| | | NTVKQILKQILADLP | VKQILKQIL |
| | | VKQILKQILADLPKD | LKQILADLP |
| | | TVKQILKQILADLPK | LKQILADLP |
| | | ISLLIFLFLFVVSLS | FLFLFVVSL |
| | | KISLLIFLFLFVVSL | LIFLFLFVV |
| | | LIKIKISTDSIFLSE | IKIKISTDS |
| | | IKIKISTDSIFLSED | IKIKISTDS |
| | | EDMIRLIGSYPDSIF | IRLIGSYPD |
| | | FVVSLSANIEENYTE | FVVSLSANI |
| | | FLSEDMIRLIGSYPD | FLSEDMIRL |
| | | SEDMIRLIGSYPDSI | IRLIGSYPD |
| | | DMIRLIGSYPDSIFN | IRLIGSYPD |
| | | DSIFLSEDMIRLIGS | FLSEDMIRL |
| | | KSHVFSDAPRIRGDL | VFSDAPRIR |
| | | EKSHVFSDAPRIRGD | VFSDAPRIR |
| | | YEKSHVFSDAPRIRG | VFSDAPRIR |
| | | SIFLSEDMIRLIGSY | FLSEDMIRL |
| | | SHVFSDAPRIRGDLR | VFSDAPRIR |
| | | DSIFNYLIQLNSDKI | IFNYLIQLN |
| | HLA-DRB1*0301 | None | |
| | HLA-DRB1*0401 | LLIFLFLFVVSLSAN | FLFVVSLSA |
| | | SLLIFLFLFVVSLSA | LFLFVVSLS |
| | | LIFLFLFVVSLSANI | FLFVVSLSA |
| | | IFLFLFVVSLSANIE | FLFVVSLSA |
| | | FLFLFVVSLSANIEE | FLFVVSLSA |
| | | LFLFVVSLSANIEEN | FLFVVSLSA |
| | | FLFVVSLSANIEENY | FLFVVSLSA |
| | | DNARNNFKKDYSEDK | RNNFKKDYS |
| | | NNFKKDYSEDKANTV | FKKDYSEDK |
| | | NARNNFKKDYSEDKA | FKKDYSEDK |
| | | RNNFKKDYSEDKANT | FKKDYSEDK |
| | | ARNNFKKDYSEDKAN | FKKDYSEDK |
| | | EAIEYLIKIKISTDS | YLIKIKIST |
| | | AIEYLIKIKISTDSI | IKIKISTDS |
| | | IEYLIKIKISTDSIF | IKIKISTDS |
| | | YLIKIKISTDSIFLS | IKIKISTDS |
| | | EYLIKIKISTDSIFL | IKIKISTDS |
| | HLA-DRB1*0404 | LIFLFLFVVSLSANI | FLFVVSLSA |
| | | IFLFLFVVSLSANIE | FVVSLSANI |
| | | FLFLFVVSLSANIEE | FVVSLSANI |
| | | LLIFLFLFVVSLSAN | LFLFVVSLS |
| | | SLLIFLFLFVVSLSA | LFLFVVSLS |
| | | LFLFVVSLSANIEEN | FVVSLSANI |
| | | ISLLIFLFLFVVSLS | LLIFLFLFV |
| | | FLFVVSLSANIEENY | FVVSLSANI |

| | | |
|---|---|---|
| | HLA-DRB1*0405 | FLSEDMIRLIGSYPD EDMIRLIGS |
| | | LSEDMIRLIGSYPDS IRLIGSYPD |
| | | EDMIRLIGSYPDSIF IRLIGSYPD |
| | | SEDMIRLIGSYPDSI IRLIGSYPD |
| | | DMIRLIGSYPDSIFN IRLIGSYPD |
| | | MIRLIGSYPDSIFNY IRLIGSYPD |
| | | IRLIGSYPDSIFNYL IRLIGSYPD |
| | | FLFLFVVSLSANIEE VVSLSANIE |
| | HLA-DRB1*0701 | None |
| | HLA-DRB1*0802 | None |
| | HLA-DRB1*0901 | IFLFLFVVSLSANIE FVVSLSANI |
| | | LIFLFLFVVSLSANI LFVVSLSAN |
| | | FLFLFVVSLSANIEE FVVSLSANI |
| | | LFLFVVSLSANIEEN FVVSLSANI |
| | | FLFVVSLSANIEENY FVVSLSANI |
| | | LFVVSLSANIEENYT FVVSLSANI |
| | HLA-DRB1*1101 | None |
| | HLA-DRB1*1302 | IEYLIKIKISTDSIF YLIKIKIST |
| | | EAIEYLIKIKISTDS YLIKIKIST |
| | | AIEYLIKIKISTDSI IKIKISTDS |
| | | YLIKIKISTDSIFLS IKIKISTDS |
| | | EYLIKIKISTDSIFL IKIKISTDS |
| | | IFLFLFVVSLSANIE LFLFVVSLS |
| | | LEAIEYLIKIKISTD YLIKIKIST |
| | | ALEAIEYLIKIKIST IEYLIKIKI |
| | | FLFLFVVSLSANIEE LFLFVVSLS |
| | | LIKIKISTDSIFLSE IKISTDSIF |
| | | LFLFVVSLSANIEEN LFLFVVSLS |
| | | FLSEDMIRLIGSYPD MIRLIGSYP |
| | | EDMIRLIGSYPDSIF MIRLIGSYP |
| | | IKIKISTDSIFLSED IKISTDSIF |
| | | LSEDMIRLIGSYPDS MIRLIGSYP |
| | | SEDMIRLIGSYPDSI MIRLIGSYP |
| | | KEDFNLINKRLDNYD FNLINKRLD |
| | | IFNYLIQLNSDKIDY LIQLNSDKI |
| | | SIFNYLIQLNSDKID LIQLNSDKI |
| | | SKEDFNLINKRLDNY FNLINKRLD |
| | | EDFNLINKRLDNYDF FNLINKRLD |
| | | DLRKIGIKEKSVFLD IGIKEKSVF |
| | | LIFLFLFVVSLSANI LFLFVVSLS |
| | | DSIFNYLIQLNSDKI IFNYLIQLN |
| | | RKIGIKEKSVFLDAL IGIKEKSVF |
| | | LLIFLFLFVVSLSAN LFLFVVSLS |
| | | LRKIGIKEKSVFLDA IGIKEKSVF |
| | | ISLLIFLFLFVVSLS ISLLIFLFL |
| | | GDLRKIGIKEKSVFL IGIKEKSVF |
| | | RGDLRKIGIKEKSVF LRKIGIKEK |
| | | FNYLIQLNSDKIDYA LIQLNSDKI |
| | | SLLIFLFLFVVSLSA LFLFVVSLS |
| | | EKYGDNARNNFKKDY YGDNARNNF |
| | | DMIRLIGSYPDSIFN IRLIGSYPD |
| | | NYLIQLNSDKIDYAE LIQLNSDKI |
| | | NTVKQILKQILADLP VKQILKQIL |
| | | YAEKYGDNARNNFKK YGDNARNNF |
| | | AEKYGDNARNNFKKD YGDNARNNF |
| | | FSKEDFNLINKRLDN FNLINKRLD |

| | | | |
|---|---|---|---|
| | | AFSKEDFNLINKRLD | DFNLINKRL |
| | | DALEAIEYLIKIKIS | IEYLIKIKI |
| | | DFNLINKRLDNYDFK | FNLINKRLD |
| | | DYAEKYGDNARNNFK | YGDNARNNF |
| | | IDYAEKYGDNARNNF | KYGDNARNN |
| | HLA-DRB1*1501 | ISLLIFLFLFVVSLS | LIFLFLFVV |
| | | KISLLIFLFLFVVSL | LIFLFLFVV |
| | | KKISLLIFLFLFVVS | LIFLFLFVV |
| | | SLLIFLFLFVVSLSA | LIFLFLFVV |
| | | MKKISLLIFLFLFVV | LLIFLFLFV |
| | | LLIFLFLFVVSLSAN | LIFLFLFVV |
| | | LIFLFLFVVSLSANI | LIFLFLFVV |
| | | IFLFLFVVSLSANIE | FLFVVSLSA |
| | | FLFLFVVSLSANIEE | FLFVVSLSA |
| | | SKEDFNLINKRLDNY | DFNLINKRL |
| | | KEDFNLINKRLDNYD | LINKRLDNY |
| | | EDFNLINKRLDNYDF | LINKRLDNY |
| | | DFNLINKRLDNYDFK | LINKRLDNY |
| | | SEDMIRLIGSYPDSI | IRLIGSYPD |
| | | EDMIRLIGSYPDSIF | IRLIGSYPD |
| | | FNLINKRLDNYDFKN | LINKRLDNY |
| | | LFLFVVSLSANIEEN | FVVSLSANI |
| | HLA-DRB3*0101 | None | |
| | HLA-DRB4*0101 | IRLIGSYPDSIFNYL | LIGSYPDSI |
| | HLA-DRB5*0101 | FNYLIQLNSDKIDYA | YLIQLNSDK |
| | | DSIFNYLIQLNSDKI | YLIQLNSDK |
| | | IFNYLIQLNSDKIDY | YLIQLNSDK |
| | | SIFNYLIQLNSDKID | YLIQLNSDK |
| | | PDSIFNYLIQLNSDK | IFNYLIQLN |
| | | DNYDFKNEYEKSHVF | FKNEYEKSH |
| | | RLDNYDFKNEYEKSH | YDFKNEYEK |
| | | LDNYDFKNEYEKSHV | FKNEYEKSH |
| | | | |
| AAL25643.1, P37-47 [Borrelia burgdorferi] Seq19 | HLA-DRB1*0101 | SSFLTQGTSPSITST | LTQGTSPSI |
| | | ISSFLTQGTSPSITS | LTQGTSPSI |
| | | RPISSFLTQGTSPSI | FLTQGTSPS |
| | | PISSFLTQGTSPSIT | LTQGTSPSI |
| | | SFLTQGTSPSITSTI | LTQGTSPSI |
| | | PRPISSFLTQGTSPS | ISSFLTQGT |
| | | FLTQGTSPSITSTIK | LTQGTSPSI |
| | | NFFTNLEEVRSSIRT | FTNLEEVRS |
| | | PEITEEVIMPIPQTI | EVIMPIPQT |
| | | EITEEVIMPIPQTID | VIMPIPQTI |
| | | ITEEVIMPIPQTIDF | VIMPIPQTI |
| | | TEEVIMPIPQTIDFY | VIMPIPQTI |
| | | SNFFTNLEEVRSSIR | FTNLEEVRS |
| | | DSNFFTNLEEVRSSI | FTNLEEVRS |
| | | GDSNFFTNLEEVRSS | FTNLEEVRS |
| | | GGDSNFFTNLEEVRS | GGDSNFFTN |
| | | EEVIMPIPQTIDFYI | VIMPIPQTI |
| | | IDFYIEPRPISSFLT | YIEPRPISS |
| | | LTQGTSPSITSTIKS | LTQGTSPSI |
| | | DFYIEPRPISSFLTQ | YIEPRPISS |
| | | YIEPRPISSFLTQGT | PRPISSFLT |
| | | IEPRPISSFLTQGTS | ISSFLTQGT |
| | | FYIEPRPISSFLTQG | YIEPRPISS |

| | | | |
|---|---|---|---|
| | | FTNLEEVRSSIRTKI | FTNLEEVRS |
| | | EPRPISSFLTQGTSP | ISSFLTQGT |
| | | FFTNLEEVRSSIRTK | FTNLEEVRS |
| | | EVIMPIPQTIDFYIE | VIMPIPQTI |
| | | TIDFYIEPRPISSFL | YIEPRPISS |
| | | INNGLNIVQKITQNI | INNGLNIVQ |
| | | QTIDFYIEPRPISSF | YIEPRPISS |
| | | NNGLNIVQKITQNID | IVQKITQNI |
| | | LNIVQKITQNIDNIT | IVQKITQNI |
| | | NGLNIVQKITQNIDN | IVQKITQNI |
| | | GLNIVQKITQNIDNI | IVQKITQNI |
| | | PQTIDFYIEPRPISS | FYIEPRPIS |
| | | VIMPIPQTIDFYIEP | VIMPIPQTI |
| | | KLSQVAQHAPNSKIE | VAQHAPNSK |
| | | EKLSQVAQHAPNSKI | VAQHAPNSK |
| | | LSQVAQHAPNSKIEK | VAQHAPNSK |
| | | HEKLSQVAQHAPNSK | KLSQVAQHA |
| | | SQVAQHAPNSKIEKV | VAQHAPNSK |
| | | VNCKFDSLNLSTKSV | FDSLNLSTK |
| | | CKFDSLNLSTKSVDD | SLNLSTKSV |
| | | NCKFDSLNLSTKSVD | SLNLSTKSV |
| | HLA-DRB1*0301 | None | |
| | HLA-DRB1*0401 | EKLKKYLKDTNNLSA | YLKDTNNLS |
| | | KLKKYLKDTNNLSAI | YLKDTNNLS |
| | | LKKYLKDTNNLSAIE | YLKDTNNLS |
| | | KKYLKDTNNLSAIEE | YLKDTNNLS |
| | | LEKLKKYLKDTNNLS | LEKLKKYLK |
| | | KYLKDTNNLSAIEES | YLKDTNNLS |
| | | YLKDTNNLSAIEESV | YLKDTNNLS |
| | | PQTIDFYIEPRPISS | FYIEPRPIS |
| | | QTIDFYIEPRPISSF | FYIEPRPIS |
| | | IDFYIEPRPISSFLT | FYIEPRPIS |
| | | TIDFYIEPRPISSFL | FYIEPRPIS |
| | | IPQTIDFYIEPRPIS | TIDFYIEPR |
| | | ISSFLTQGTSPSITS | FLTQGTSPS |
| | | SSFLTQGTSPSITST | FLTQGTSPS |
| | | RPISSFLTQGTSPSI | FLTQGTSPS |
| | | PISSFLTQGTSPSIT | FLTQGTSPS |
| | | PRPISSFLTQGTSPS | PRPISSFLT |
| | | DFYIEPRPISSFLTQ | FYIEPRPIS |
| | HLA-DRB1*0404 | EKLKKYLKDTNNLSA | YLKDTNNLS |
| | | KLKKYLKDTNNLSAI | LKDTNNLSA |
| | | LKKYLKDTNNLSAIE | LKDTNNLSA |
| | | KKYLKDTNNLSAIEE | LKDTNNLSA |
| | | EKITHPIFDHITGSG | ITHPIFDHI |
| | | KYLKDTNNLSAIEES | LKDTNNLSA |
| | | KITHPIFDHITGSGN | IFDHITGSG |
| | | ITHPIFDHITGSGNN | IFDHITGSG |
| | HLA-DRB1*0405 | KKYLKDTNNLSAIEE | YLKDTNNLS |
| | | EKLKKYLKDTNNLSA | YLKDTNNLS |
| | | LKKYLKDTNNLSAIE | YLKDTNNLS |
| | | KLKKYLKDTNNLSAI | YLKDTNNLS |
| | | NGLNIVQKITQNIDN | VQKITQNID |
| | | NNGLNIVQKITQNID | IVQKITQNI |
| | | GLNIVQKITQNIDNI | VQKITQNID |

|  |  | NIVQKITQNIDNITE | VQKITQNID |
| --- | --- | --- | --- |
|  |  | LNIVQKITQNIDNIT | VQKITQNID |
|  |  | LEKLKKYLKDTNNLS | LKKYLKDTN |
|  |  | EITEEVIMPIPQTID | EVIMPIPQT |
|  |  | ITEEVIMPIPQTIDF | IMPIPQTID |
|  |  | TEEVIMPIPQTIDFY | IMPIPQTID |
|  |  | EEVIMPIPQTIDFYI | IMPIPQTID |
|  |  | EVIMPIPQTIDFYIE | IMPIPQTID |
|  |  | GGDSNFFTNLEEVRS | SNFFTNLEE |
|  |  | LEIGGDSNFFTNLEE | DSNFFTNLE |
|  |  | IGGDSNFFTNLEEVR | SNFFTNLEE |
|  |  | KYLKDTNNLSAIEES | YLKDTNNLS |
|  |  | EIGGDSNFFTNLEEV | SNFFTNLEE |
|  |  | IVQKITQNIDNITEN | VQKITQNID |
|  |  | YLKDTNNLSAIEESV | YLKDTNNLS |
|  |  | VQKITQNIDNITENL | VQKITQNID |
|  |  | GDSNFFTNLEEVRSS | SNFFTNLEE |
|  |  | IDFYIEPRPISSFLT | FYIEPRPIS |
|  |  | PQTIDFYIEPRPISS | FYIEPRPIS |
|  |  | RPISSFLTQGTSPSI | FLTQGTSPS |
|  |  | PRPISSFLTQGTSPS | ISSFLTQGT |
|  |  | QTIDFYIEPRPISSF | FYIEPRPIS |
|  |  | TIDFYIEPRPISSFL | FYIEPRPIS |
|  |  | ISSFLTQGTSPSITS | FLTQGTSPS |
|  | HLA-DRB1*0701 | TNLEEVRSSIRTKIK | LEEVRSSIR |
|  |  | FTNLEEVRSSIRTKI | LEEVRSSIR |
|  |  | SFLTQGTSPSITSTI | LTQGTSPSI |
|  | HLA-DRB1*0802 | None |  |
|  | HLA-DRB1*0901 | KDIYNSYIPEVKEEI | IYNSYIPEV |
|  |  | SSNKDIYNSYIPEVK | IYNSYIPEV |
|  |  | SNKDIYNSYIPEVKE | IYNSYIPEV |
|  |  | NKDIYNSYIPEVKEE | IYNSYIPEV |
|  | HLA-DRB1*1101 | None |  |
|  | HLA-DRB1*1302 | EKINNGLNIVQKITQ | KINNGLNIV |
|  |  | LNIVQKITQNIDNIT | LNIVQKITQ |
|  |  | KINNGLNIVQKITQN | LNIVQKITQ |
|  |  | INNGLNIVQKITQNI | LNIVQKITQ |
|  |  | NNGLNIVQKITQNID | LNIVQKITQ |
|  |  | NGLNIVQKITQNIDN | LNIVQKITQ |
|  |  | AKEKINNGLNIVQKI | KINNGLNIV |
|  |  | KEKINNGLNIVQKIT | KINNGLNIV |
|  |  | ELAKEKINNGLNIVQ | KINNGLNIV |
|  |  | LAKEKINNGLNIVQK | KINNGLNIV |
|  |  | GLNIVQKITQNIDNI | LNIVQKITQ |
|  |  | NIVQKITQNIDNITE | ITQNIDNIT |
|  |  | KELAKEKINNGLNIV | EKINNGLNI |
|  |  | MCAFLLLNLVNCKFD | LLLNLVNCK |
|  |  | IVQKITQNIDNITEN | ITQNIDNIT |
|  |  | CAFLLLNLVNCKFDS | LLLNLVNCK |
|  |  | VQKITQNIDNITENL | ITQNIDNIT |
|  |  | AFLLLNLVNCKFDSL | LLLNLVNCK |
|  |  | QKITQNIDNITENLN | ITQNIDNIT |
|  |  | NSIAKLLQHLSKSED | IAKLLQHLS |
|  |  | NCKFDSLNLSTKSVD | SLNLSTKSV |
|  |  | CKFDSLNLSTKSVDD | LNLSTKSVD |
|  |  | RPISSFLTQGTSPSI | FLTQGTSPS |

| | | | |
|---|---|---|---|
| | | KFDSLNLSTKSVDDK | LNLSTKSVD |
| | | FLLLNLVNCKFDSLN | LLLNLVNCK |
| | | PISSFLTQGTSPSIT | FLTQGTSPS |
| | | ISSFLTQGTSPSITS | FLTQGTSPS |
| | | PRPISSFLTQGTSPS | ISSFLTQGT |
| | | FDSLNLSTKSVDDKN | LNLSTKSVD |
| | | FTNLEEVRSSIRTKI | LEEVRSSIR |
| | | SSFLTQGTSPSITST | FLTQGTSPS |
| | | SIAKLLQHLSKSEDQ | LQHLSKSED |
| | | IAKLLQHLSKSEDQA | LQHLSKSED |
| | | KITQNIDNITENLNS | ITQNIDNIT |
| | | ITQNIDNITENLNSK | ITQNIDNIT |
| | | DDKNNSIAKLLQHLS | DKNNSIAKL |
| | | TNLEEVRSSIRTKIK | VRSSIRTKI |
| | HLA-DRB1*1501 | FTNLEEVRSSIRTKI | LEEVRSSIR |
| | | TNLEEVRSSIRTKIK | LEEVRSSIR |
| | | FFTNLEEVRSSIRTK | LEEVRSSIR |
| | HLA-DRB3*0101 | None | |
| | HLA-DRB4*0101 | EKLSQVAQHAPNSKI | VAQHAPNSK |
| | | HEKLSQVAQHAPNSK | EKLSQVAQH |
| | | KLSQVAQHAPNSKIE | VAQHAPNSK |
| | | LSQVAQHAPNSKIEK | VAQHAPNSK |
| | | SQVAQHAPNSKIEKV | VAQHAPNSK |
| | | QVAQHAPNSKIEKVK | VAQHAPNSK |
| | | VAQHAPNSKIEKVKS | VAQHAPNSK |
| | HLA-DRB5*0101 | None | |
| | | | |
| NP_212481.1\| fibronectin/fibrinogen-binding protein, putative [Borrelia burgdorferi B31]  Seq20 | HLA-DRB1*0101 | LDKFNLIQSKITMLK | FNLIQSKIT |
| | | DKFNLIQSKITMLKV | FNLIQSKIT |
| | | LCVFYTKLAKKSGKA | FYTKLAKKS |
| | | CVFYTKLAKKSGKAD | FYTKLAKKS |
| | | NSNFKILDAYYRRPK | FKILDAYYR |
| | | SNFKILDAYYRRPKI | FKILDAYYR |
| | | GNLCVFYTKLAKKSG | FYTKLAKKS |
| | | NLCVFYTKLAKKSGK | FYTKLAKKS |
| | | TNSNFKILDAYYRRP | FKILDAYYR |
| | | IATNSNFKILDAYYR | NSNFKILDA |
| | | ATNSNFKILDAYYRR | FKILDAYYR |
| | | KFNLIQSKITMLKVE | IQSKITMLK |
| | | FNLIQSKITMLKVEN | IQSKITMLK |
| | | NLDKFNLIQSKITML | FNLIQSKIT |
| | | AGNLCVFYTKLAKKS | LCVFYTKLA |
| | | NLIQSKITMLKVENL | IQSKITMLK |
| | | DNLDKFNLIQSKITM | FNLIQSKIT |
| | | KDNLDKFNLIQSKIT | LDKFNLIQS |
| | | VFYTKLAKKSGKADL | FYTKLAKKS |
| | | IKEIPFTNSLITKII | FTNSLITKI |
| | | KEIPFTNSLITKIIQ | FTNSLITKI |
| | | LIQSKITMLKVENLI | ITMLKVENL |
| | | LDVLLGAGNLCVFYT | LLGAGNLCV |
| | | DVLLGAGNLCVFYTK | LLGAGNLCV |
| | | ILFIKLWPSSPNIIA | LWPSSPNII |
| | | IQSKITMLKVENLIP | ITMLKVENL |
| | | LIKEIPFTNSLITKI | IPFTNSLIT |
| | | EIPFTNSLITKIIQP | FTNSLITKI |
| | | IPFTNSLITKIIQPD | FTNSLITKI |

| | | | |
|---|---|---|---|
| | | FYTKLAKKSGKADLY | FYTKLAKKS |
| | | NFKILDAYYRRPKIK | FKILDAYYR |
| | | FKILDAYYRRPKIKE | FKILDAYYR |
| | | LFIKLWPSSPNIIAT | LWPSSPNII |
| | | PSLDVLLGAGNLCVF | LLGAGNLCV |
| | | SLDVLLGAGNLCVFY | LLGAGNLCV |
| | | TPSLDVLLGAGNLCV | DVLLGAGNL |
| | | IIKAFQMKNERIISL | FQMKNERII |
| | | IKAFQMKNERIISLE | MKNERIISL |
| | | IILFIKLWPSSPNII | IKLWPSSPN |
| | | KAFQMKNERIISLEI | MKNERIISL |
| | | KKNFKKNALKLRFSD | FKKNALKLR |
| | | TKKNFKKNALKLRFS | FKKNALKLR |
| | | FIKLWPSSPNIIATN | LWPSSPNII |
| | | KNFKKNALKLRFSDF | FKKNALKLR |
| | | AFQMKNERIISLEIL | MKNERIISL |
| | | FKILICLNPNTTRFH | LICLNPNTT |
| | | KILICLNPNTTRFHI | LICLNPNTT |
| | | IKLWPSSPNIIATNS | LWPSSPNII |
| | | VKNLRRVKNKKLGLV | LRRVKNKKL |
| | | EKIKISLNQSLSPKE | IKISLNQSL |
| | | FQMKNERIISLEILQ | MKNERIISL |
| | | LLGAGNLCVFYTKLA | LLGAGNLCV |
| | | EEKIKISLNQSLSPK | IKISLNQSL |
| | | KEEKIKISLNQSLSP | IKISLNQSL |
| | | KSKIQNGKIIKAFQM | IQNGKIIKA |
| | | SKIQNGKIIKAFQMK | IQNGKIIKA |
| | | ITKKNFKKNALKLRF | FKKNALKLR |
| | | MIKMSLNYTEINTLI | MIKMSLNYT |
| | | FLKSKIQNGKIIKAF | IQNGKIIKA |
| | | LKSKIQNGKIIKAFQ | IQNGKIIKA |
| | | DFLKSKIQNGKIIKA | KSKIQNGKI |
| | | KNLRRVKNKKLGLVI | VKNKKLGLV |
| | | ILICLNPNTTRFHIT | LNPNTTRFH |
| | | VLLGAGNLCVFYTKL | LLGAGNLCV |
| | | LRRVKNKKLGLVIPK | VKNKKLGLV |
| | | YKEEKIKISLNQSLS | IKISLNQSL |
| | | NLRRVKNKKLGLVIP | VKNKKLGLV |
| | | KKFKILICLNPNTTR | LICLNPNTT |
| | | KFKILICLNPNTTRF | LICLNPNTT |
| | | NYKEEKIKISLNQSL | KIKISLNQS |
| | | DLYYTQVKNLRRVKN | YTQVKNLRR |
| | | HITKKNFKKNALKLR | NFKKNALKL |
| | | LYYTQVKNLRRVKNK | VKNLRRVKN |
| | | RRVKNKKLGLVIPKA | VKNKKLGLV |
| | | LICLNPNTTRFHITK | LNPNTTRFH |
| | | KIKISLNQSLSPKEN | IKISLNQSL |
| | | QSKITMLKVENLIPE | ITMLKVENL |
| | | IKISLNQSLSPKENA | IKISLNQSL |
| | | SKITMLKVENLIPEE | ITMLKVENL |
| | | MIILFIKLWPSSPNI | IKLWPSSPN |
| | | KKGKNSFKTIQNQLK | KKGKNSFKT |
| | | PFTNSLITKIIQPDY | FTNSLITKI |
| | | NFKKNALKLRFSDFL | FKKNALKLR |
| | | YTQVKNLRRVKNKKL | VKNLRRVKN |
| | | DMIILFIKLWPSSPN | FIKLWPSSP |
| | | FKKNALKLRFSDFLK | FKKNALKLR |

| | | | |
|---|---|---|---|
| | | KLWPSSPNIIATNSN | LWPSSPNII |
| | | TQVKNLRRVKNKKLG | VKNLRRVKN |
| | | KIIQPDYKSLVLEIY | IIQPDYKSL |
| | | QPDYKSLVLEIYNKI | YKSLVLEIY |
| | | IIQPDYKSLVLEIYN | YKSLVLEIY |
| | | IQPDYKSLVLEIYNK | YKSLVLEIY |
| | | PDYKSLVLEIYNKID | YKSLVLEIY |
| | | FTNSLITKIIQPDYK | FTNSLITKI |
| | | YYTQVKNLRRVKNKK | VKNLRRVKN |
| | | KDMIILFIKLWPSSP | MIILFIKLW |
| | | KNSFKTIQNQLKDNL | FKTIQNQLK |
| | | NSFKTIQNQLKDNLD | FKTIQNQLK |
| | | NTLIKEIPFTNSLIT | IKEIPFTNS |
| | | FTYCGFEILIGRNAK | YCGFEILIG |
| | | KEKTPKIGLHFTYCG | TPKIGLHFT |
| | | HFTYCGFEILIGRNA | YCGFEILIG |
| | | TLIKEIPFTNSLITK | IKEIPFTNS |
| | | GKNSFKTIQNQLKDN | FKTIQNQLK |
| | | EKTPKIGLHFTYCGF | IGLHFTYCG |
| | | KGKNSFKTIQNQLKD | FKTIQNQLK |
| | | ICLNPNTTRFHITKK | LNPNTTRFH |
| | | LWPSSPNIIATNSNF | LWPSSPNII |
| | | EIFLKAKEIHESNKM | LKAKEIHES |
| | | IFLKAKEIHESNKMS | LKAKEIHES |
| | | ADLYYTQVKNLRRVK | YTQVKNLRR |
| | | MKNERIISLEILQKD | MKNERIISL |
| | | YCGFEILIGRNAKEN | FEILIGRNA |
| | | LGAGNLCVFYTKLAK | LCVFYTKLA |
| | | QMKNERIISLEILQK | MKNERIISL |
| | | KADLYYTQVKNLRRV | YTQVKNLRR |
| | | NKKFKILICLNPNTT | FKILICLNP |
| | | RVKNKKLGLVIPKAE | VKNKKLGLV |
| | | TPKIGLHFTYCGFEI | IGLHFTYCG |
| | | KIQNGKIIKAFQMKN | IQNGKIIKA |
| | | GKIIKAFQMKNERII | AFQMKNERI |
| | | KTPKIGLHFTYCGFE | IGLHFTYCG |
| | | VKNKKLGLVIPKAEK | VKNKKLGLV |
| | | IQNGKIIKAFQMKNE | IQNGKIIKA |
| | | TEINTLIKEIPFTNS | INTLIKEIP |
| | | INTLIKEIPFTNSLI | IKEIPFTNS |
| | | EINTLIKEIPFTNSL | IKEIPFTNS |
| | | TGEIFLKAKEIHESN | LKAKEIHES |
| | | TTGEIFLKAKEIHES | EIFLKAKEI |
| | | GEIFLKAKEIHESNK | LKAKEIHES |
| | | MELKEEYNNTSYTSY | EYNNTSYTS |
| | | KIIKAFQMKNERIIS | FQMKNERII |
| | HLA-DRB1*0301 | None | |
| | HLA-DRB1*0401 | DLYYTQVKNLRRVKN | YYTQVKNLR |
| | | SGKADLYYTQVKNLR | SGKADLYYT |
| | | GKADLYYTQVKNLRR | YYTQVKNLR |
| | | KADLYYTQVKNLRRV | YYTQVKNLR |
| | | ADLYYTQVKNLRRVK | YYTQVKNLR |
| | | LYYTQVKNLRRVKNK | YYTQVKNLR |
| | | YYTQVKNLRRVKNKK | YYTQVKNLR |
| | | MELKEEYNNTSYTSY | LKEEYNNTS |
| | | IMELKEEYNNTSYTS | LKEEYNNTS |
| | | DKKIMELKEEYNNTS | KIMELKEEY |

| | | | |
|---|---|---|---|
| | | KKIMELKEEYNNTSY | LKEEYNNTS |
| | | KIMELKEEYNNTSYT | LKEEYNNTS |
| | | DMIILFIKLWPSSPN | LFIKLWPSS |
| | | MIILFIKLWPSSPNI | LFIKLWPSS |
| | | IILFIKLWPSSPNII | LFIKLWPSS |
| | | ILFIKLWPSSPNIIA | IKLWPSSPN |
| | | ELKEEYNNTSYTSYS | LKEEYNNTS |
| | | LKEEYNNTSYTSYSE | LKEEYNNTS |
| | | WPSSPNIIATNSNFK | PNIIATNSN |
| | | PSSPNIIATNSNFKI | IIATNSNFK |
| | | LFIKLWPSSPNIIAT | IKLWPSSPN |
| | | SSPNIIATNSNFKIL | IIATNSNFK |
| | | GAYVFIKNQKNKTPS | FIKNQKNKT |
| | | PGAYVFIKNQKNKTP | FIKNQKNKT |
| | | PNIIATNSNFKILDA | IIATNSNFK |
| | | SPNIIATNSNFKILD | IIATNSNFK |
| | | KDMIILFIKLWPSSP | LFIKLWPSS |
| | | YPGAYVFIKNQKNKT | YVFIKNQKN |
| | | YVFIKNQKNKTPSLD | FIKNQKNKT |
| | | AYVFIKNQKNKTPSL | FIKNQKNKT |
| | | LIEKYKKELIVLEKR | YKKELIVLE |
| | HLA-DRB1*0404 | WPSSPNIIATNSNFK | PNIIATNSN |
| | | PSSPNIIATNSNFKI | IIATNSNFK |
| | | FKILICLNPNTTRFH | ILICLNPNT |
| | | SSPNIIATNSNFKIL | IIATNSNFK |
| | | KFKILICLNPNTTRF | ILICLNPNT |
| | | PNIIATNSNFKILDA | IIATNSNFK |
| | | SPNIIATNSNFKILD | IIATNSNFK |
| | | KKFKILICLNPNTTR | ILICLNPNT |
| | | NKKFKILICLNPNTT | ILICLNPNT |
| | | MIILFIKLWPSSPNI | FIKLWPSSP |
| | | KILICLNPNTTRFHI | LICLNPNTT |
| | | IILFIKLWPSSPNII | KLWPSSPNI |
| | | ILFIKLWPSSPNIIA | KLWPSSPNI |
| | | SNFKILDAYYRRPKI | ILDAYYRRP |
| | | TNSNFKILDAYYRRP | FKILDAYYR |
| | | NSNFKILDAYYRRPK | ILDAYYRRP |
| | | NFKILDAYYRRPKIK | ILDAYYRRP |
| | | FKILDAYYRRPKIKE | ILDAYYRRP |
| | | KDMIILFIKLWPSSP | MIILFIKLW |
| | | ILICLNPNTTRFHIT | ILICLNPNT |
| | | DNKKFKILICLNPNT | FKILICLNP |
| | | NIIATNSNFKILDAY | IIATNSNFK |
| | | IIATNSNFKILDAYY | IIATNSNFK |
| | | DMIILFIKLWPSSPN | FIKLWPSSP |
| | HLA-DRB1*0405 | DMIILFIKLWPSSPN | FIKLWPSSP |
| | | MIILFIKLWPSSPNI | IKLWPSSPN |
| | | IILFIKLWPSSPNII | IKLWPSSPN |
| | | ILFIKLWPSSPNIIA | IKLWPSSPN |
| | | LFIKLWPSSPNIIAT | IKLWPSSPN |
| | | FIKLWPSSPNIIATN | IKLWPSSPN |
| | | IKLWPSSPNIIATNS | IKLWPSSPN |
| | | DLYYTQVKNLRRVKN | YTQVKNLRR |
| | | LYYTQVKNLRRVKNK | YTQVKNLRR |
| | | GKADLYYTQVKNLRR | YYTQVKNLR |
| | | ADLYYTQVKNLRRVK | YTQVKNLRR |

|  |  | KADLYYTQVKNLRRV | YTQVKNLRR |
| --- | --- | --- | --- |
|  |  | KKGKNSFKTIQNQLK | KNSFKTIQN |
|  |  | KGKNSFKTIQNQLKD | KNSFKTIQN |
|  |  | KDMIILFIKLWPSSP | ILFIKLWPS |
|  |  | KLWPSSPNIIATNSN | WPSSPNIIA |
|  |  | YYTQVKNLRRVKNKK | YTQVKNLRR |
|  |  | GKNSFKTIQNQLKDN | FKTIQNQLK |
|  |  | KNSFKTIQNQLKDNL | FKTIQNQLK |
|  |  | DSLKQQIKLLENIEN | LKQQIKLLE |
|  |  | IKMSLNYTEINTLIK | IKMSLNYTE |
|  |  | SLKQQIKLLENIENE | IKLLENIEN |
|  |  | LKQQIKLLENIENEK | IKLLENIEN |
|  |  | WPSSPNIIATNSNFK | WPSSPNIIA |
|  |  | KQQIKLLENIENEKE | IKLLENIEN |
|  |  | KMSLNYTEINTLIKE | YTEINTLIK |
|  |  | MSLNYTEINTLIKEI | YTEINTLIK |
|  |  | SLNYTEINTLIKEIP | YTEINTLIK |
|  |  | QQIKLLENIENEKEK | IKLLENIEN |
|  |  | KAYKKGKNSFKTIQN | YKKGKNSFK |
|  |  | LNYTEINTLIKEIPF | YTEINTLIK |
|  |  | AYKKGKNSFKTIQNQ | KNSFKTIQN |
|  |  | YTQVKNLRRVKNKKL | YTQVKNLRR |
|  |  | LQKDMIILFIKLWPS | IILFIKLWP |
|  |  | YKKGKNSFKTIQNQL | KNSFKTIQN |
|  |  | LWPSSPNIIATNSNF | WPSSPNIIA |
|  |  | QKDMIILFIKLWPSS | ILFIKLWPS |
|  |  | NSFKTIQNQLKDNLD | FKTIQNQLK |
|  | HLA-DRB1*0701 | ILICLNPNTTRFHIT | LNPNTTRFH |
|  |  | LICLNPNTTRFHITK | LNPNTTRFH |
|  |  | KILICLNPNTTRFHI | LNPNTTRFH |
|  |  | ICLNPNTTRFHITKK | LNPNTTRFH |
|  |  | MELKEEYNNTSYTSY | EEYNNTSYT |
|  |  | ELKEEYNNTSYTSYS | YNNTSYTSY |
|  |  | KEEYNNTSYTSYSEF | YNNTSYTSY |
|  |  | LKEEYNNTSYTSYSE | YNNTSYTSY |
|  |  | EEYNNTSYTSYSEFL | YNNTSYTSY |
|  |  | CLNPNTTRFHITKKN | NPNTTRFHI |
|  |  | SSPNIIATNSNFKIL | IIATNSNFK |
|  |  | FKILICLNPNTTRFH | CLNPNTTRF |
|  |  | SPNIIATNSNFKILD | IIATNSNFK |
|  |  | IKEIPFTNSLITKII | IPFTNSLIT |
|  |  | LIKEIPFTNSLITKI | IPFTNSLIT |
|  |  | PNIIATNSNFKILDA | IIATNSNFK |
|  |  | KEIPFTNSLITKIIQ | IPFTNSLIT |
|  |  | PSSPNIIATNSNFKI | IIATNSNFK |
|  |  | LNPNTTRFHITKKNF | LNPNTTRFH |
|  |  | WPSSPNIIATNSNFK | NIIATNSNF |
|  | HLA-DRB1*0802 | DMIILFIKLWPSSPN | FIKLWPSSP |
|  |  | MIILFIKLWPSSPNI | FIKLWPSSP |
|  |  | IILFIKLWPSSPNII | FIKLWPSSP |
|  |  | KDMIILFIKLWPSSP | LFIKLWPSS |
|  |  | GNLCVFYTKLAKKSG | FYTKLAKKS |
|  |  | NLCVFYTKLAKKSGK | YTKLAKKSG |
|  |  | ILFIKLWPSSPNIIA | FIKLWPSSP |
|  |  | LCVFYTKLAKKSGKA | YTKLAKKSG |
|  |  | CVFYTKLAKKSGKAD | YTKLAKKSG |
|  |  | VFYTKLAKKSGKADL | YTKLAKKSG |

| | | LFIKLWPSSPNIIAT | FIKLWPSSP |
| --- | --- | --- | --- |
| | | PGAYVFIKNQKNKTP | FIKNQKNKT |
| | | GAYVFIKNQKNKTPS | FIKNQKNKT |
| | | AYVFIKNQKNKTPSL | FIKNQKNKT |
| | | YVFIKNQKNKTPSLD | FIKNQKNKT |
| | | YPGAYVFIKNQKNKT | YVFIKNQKN |
| | HLA-DRB1*0901 | ILFIKLWPSSPNIIA | LWPSSPNII |
| | | LFIKLWPSSPNIIAT | LWPSSPNII |
| | | FIKLWPSSPNIIATN | LWPSSPNII |
| | | IKLWPSSPNIIATNS | LWPSSPNII |
| | | IILFIKLWPSSPNII | KLWPSSPNI |
| | | KLWPSSPNIIATNSN | LWPSSPNII |
| | | LWPSSPNIIATNSNF | LWPSSPNII |
| | | IKEIPFTNSLITKII | FTNSLITKI |
| | | KEIPFTNSLITKIIQ | FTNSLITKI |
| | | LIKEIPFTNSLITKI | IPFTNSLIT |
| | HLA-DRB1*1101 | DLYYTQVKNLRRVKN | QVKNLRRVK |
| | | LYYTQVKNLRRVKNK | VKNLRRVKN |
| | | YYTQVKNLRRVKNKK | VKNLRRVKN |
| | | YTQVKNLRRVKNKKL | VKNLRRVKN |
| | | TQVKNLRRVKNKKLG | VKNLRRVKN |
| | | QVKNLRRVKNKKLGL | VKNLRRVKN |
| | | VKNLRRVKNKKLGLV | VKNLRRVKN |
| | | NLCVFYTKLAKKSGK | YTKLAKKSG |
| | | LCVFYTKLAKKSGKA | YTKLAKKSG |
| | | GNLCVFYTKLAKKSG | FYTKLAKKS |
| | | CVFYTKLAKKSGKAD | YTKLAKKSG |
| | | VFYTKLAKKSGKADL | YTKLAKKSG |
| | | FYTKLAKKSGKADLY | YTKLAKKSG |
| | HLA-DRB1*1302 | KGELILLNINKIQKG | LILLNINKI |
| | | GELILLNINKIQKGI | LILLNINKI |
| | | ELILLNINKIQKGIK | LNINKIQKG |
| | | LILLNINKIQKGIKE | LNINKIQKG |
| | | EKGELILLNINKIQK | LILLNINKI |
| | | KEKGELILLNINKIQ | LILLNINKI |
| | | DLYYTQVKNLRRVKN | YYTQVKNLR |
| | | VKNLRRVKNKKLGLV | VKNLRRVKN |
| | | AYVFIKNQKNKTPSL | FIKNQKNKT |
| | | YVFIKNQKNKTPSLD | FIKNQKNKT |
| | | GAYVFIKNQKNKTPS | FIKNQKNKT |
| | | LYYTQVKNLRRVKNK | VKNLRRVKN |
| | | ILLNINKIQKGIKEI | LNINKIQKG |
| | | EKEKGELILLNINKI | KGELILLNI |
| | | YYTQVKNLRRVKNKK | VKNLRRVKN |
| | | TQVKNLRRVKNKKLG | VKNLRRVKN |
| | | YTQVKNLRRVKNKKL | VKNLRRVKN |
| | | PGAYVFIKNQKNKTP | FIKNQKNKT |
| | | ITKKNFKKNALKLRF | FKKNALKLR |
| | | HITKKNFKKNALKLR | KKNFKKNAL |
| | | VFIKNQKNKTPSLDV | FIKNQKNKT |
| | | LDKFNLIQSKITMLK | FNLIQSKIT |
| | | DKFNLIQSKITMLKV | IQSKITMLK |
| | | KNLRRVKNKKLGLVI | VKNKKLGLV |
| | | NLRRVKNKKLGLVIP | VKNKKLGLV |
| | | TKKNFKKNALKLRFS | FKKNALKLR |
| | | LRRVKNKKLGLVIPK | VKNKKLGLV |

| | | | |
|---|---|---|---|
| | | KKNFKKNALKLRFSD | FKKNALKLR |
| | | FIKNQKNKTPSLDVL | FIKNQKNKT |
| | | KFNLIQSKITMLKVE | IQSKITMLK |
| | | QVKNLRRVKNKKLGL | VKNLRRVKN |
| | | LLNINKIQKGIKEIN | LNINKIQKG |
| | | FNLIQSKITMLKVEN | IQSKITMLK |
| | | RRVKNKKLGLVIPKA | VKNKKLGLV |
| | | YPGAYVFIKNQKNKT | VFIKNQKNK |
| | | ISLEILQKDMIILFI | LEILQKDMI |
| | | IISLEILQKDMIILF | LEILQKDMI |
| | | RVKNKKLGLVIPKAE | VKNKKLGLV |
| | | VKNKKLGLVIPKAEK | LGLVIPKAE |
| | | KNFKKNALKLRFSDF | FKKNALKLR |
| | | IPFTNSLITKIIQPD | FTNSLITKI |
| | | NLDKFNLIQSKITML | FNLIQSKIT |
| | | FKILICLNPNTTRFH | LICLNPNTT |
| | | KKFKILICLNPNTTR | LICLNPNTT |
| | | IKEIPFTNSLITKII | FTNSLITKI |
| | | KEIPFTNSLITKIIQ | FTNSLITKI |
| | | NLIQSKITMLKVENL | IQSKITMLK |
| | | KILICLNPNTTRFHI | LICLNPNTT |
| | | KFKILICLNPNTTRF | LICLNPNTT |
| | | LEILQKDMIILFIKL | LQKDMIILF |
| | | EIPFTNSLITKIIQP | FTNSLITKI |
| | | SLEILQKDMIILFIK | LQKDMIILF |
| | | LNINKIQKGIKEINL | LNINKIQKG |
| | | IKLDENLIKKIKNQT | ENLIKKIKN |
| | | LNYTEINTLIKEIPF | INTLIKEIP |
| | | KADLYYTQVKNLRRV | YYTQVKNLR |
| | | ADLYYTQVKNLRRVK | YYTQVKNLR |
| | | SLNYTEINTLIKEIP | TEINTLIKE |
| | | PFTNSLITKIIQPDY | FTNSLITKI |
| | | DNLDKFNLIQSKITM | FNLIQSKIT |
| | | DFLKSKIQNGKIIKA | LKSKIQNGK |
| | | QPDYKSLVLEIYNKI | YKSLVLEIY |
| | | NYTEINTLIKEIPFT | INTLIKEIP |
| | | PDYKSLVLEIYNKID | YKSLVLEIY |
| | | YTEINTLIKEIPFTN | INTLIKEIP |
| | | RIISLEILQKDMIIL | LEILQKDMI |
| | | GKADLYYTQVKNLRR | YYTQVKNLR |
| | | TEINTLIKEIPFTNS | INTLIKEIP |
| | | NKKFKILICLNPNTT | FKILICLNP |
| | | IATNSNFKILDAYYR | IATNSNFKI |
| | | YKSLVLEIYNKIDNK | YKSLVLEIY |
| | | KDNLDKFNLIQSKIT | KFNLIQSKI |
| | | ILICLNPNTTRFHIT | LICLNPNTT |
| | | LIKEIPFTNSLITKI | IPFTNSLIT |
| | | DYKSLVLEIYNKIDN | YKSLVLEIY |
| | | LKSKIQNGKIIKAFQ | IQNGKIIKA |
| | | FLKSKIQNGKIIKAF | IQNGKIIKA |
| | | IKNQKNKTPSLDVLL | IKNQKNKTP |
| | | MIKMSLNYTEINTLI | MIKMSLNYT |
| | | NFKKNALKLRFSDFL | FKKNALKLR |
| | | FTNSLITKIIQPDYK | FTNSLITKI |
| | | LDVLLGAGNLCVFYT | VLLGAGNLC |
| | | DSLKQQIKLLENIEN | LKQQIKLLE |
| | | LVLEIYNKIDNKKFK | IYNKIDNKK |

| | | | |
|---|---|---|---|
| | | LIQSKITMLKVENLI | IQSKITMLK |
| | | SLVLEIYNKIDNKKF | IYNKIDNKK |
| | | SGKADLYYTQVKNLR | LYYTQVKNL |
| | | KIIQPDYKSLVLEIY | IQPDYKSLV |
| | | DMIILFIKLWPSSPN | IILFIKLWP |
| | | LICLNPNTTRFHITK | LICLNPNTT |
| | | KITMLKVENLIPEEE | LKVENLIPE |
| | | FKKNALKLRFSDFLK | FKKNALKLR |
| | | EILQKDMIILFIKLW | LQKDMIILF |
| | | NERIISLEILQKDMI | IISLEILQK |
| | | ERIISLEILQKDMII | LEILQKDMI |
| | | IILFIKLWPSSPNII | IKLWPSSPN |
| | | KKLGLVIPKAEKNLH | LGLVIPKAE |
| | | KSLVLEIYNKIDNKK | LVLEIYNKI |
| | | DVLLGAGNLCVFYTK | AGNLCVFYT |
| | | ILFIKLWPSSPNIIA | IKLWPSSPN |
| | | LNDQIKKTNIKELLI | DQIKKTNIK |
| | | ITMLKVENLIPEEEY | LKVENLIPE |
| | | SPNIIATNSNFKILD | IATNSNFKI |
| | | SSPNIIATNSNFKIL | IATNSNFKI |
| | | LEIYNKIDNKKFKIL | IYNKIDNKK |
| | | KNKKLGLVIPKAEKN | LGLVIPKAE |
| | | SLNDQIKKTNIKELL | DQIKKTNIK |
| | | VLLGAGNLCVFYTKL | AGNLCVFYT |
| | | PNIIATNSNFKILDA | IATNSNFKI |
| | | IQSKITMLKVENLIP | IQSKITMLK |
| | | ILQKDMIILFIKLWP | LQKDMIILF |
| | | IQKGIKEINLLNYKE | IKEINLLNY |
| | | MIILFIKLWPSSPNI | IKLWPSSPN |
| | | LFIKLWPSSPNIIAT | IKLWPSSPN |
| | | KIQKGIKEINLLNYK | IKEINLLNY |
| | | NKKLGLVIPKAEKNL | LGLVIPKAE |
| | | NKIQKGIKEINLLNY | KGIKEINLL |
| | | SDFLKSKIQNGKIIK | LKSKIQNGK |
| | | NSFKTIQNQLKDNLD | IQNQLKDNL |
| | | QSKITMLKVENLIPE | ITMLKVENL |
| | | NIIATNSNFKILDAY | IATNSNFKI |
| | | SKITMLKVENLIPEE | LKVENLIPE |
| | | IIQPDYKSLVLEIYN | YKSLVLEIY |
| | | ATNSNFKILDAYYRR | FKILDAYYR |
| | | KNSFKTIQNQLKDNL | FKTIQNQLK |
| | | EKRIDSLKQQIKLLE | IDSLKQQIK |
| | | IDSLKQQIKLLENIE | LKQQIKLLE |
| | | PSSPNIIATNSNFKI | PNIIATNSN |
| | | LQKDMIILFIKLWPS | IILFIKLWP |
| | | RIDSLKQQIKLLENI | LKQQIKLLE |
| | | QKGIKEINLLNYKEE | IKEINLLNY |
| | | IQPDYKSLVLEIYNK | YKSLVLEIY |
| | HLA-DRB1*1501 | DMIILFIKLWPSSPN | LFIKLWPSS |
| | | KDMIILFIKLWPSSP | LFIKLWPSS |
| | | MIILFIKLWPSSPNI | LFIKLWPSS |
| | | QKDMIILFIKLWPSS | ILFIKLWPS |
| | | IILFIKLWPSSPNII | LFIKLWPSS |
| | | VKNLRRVKNKKLGLV | LRRVKNKKL |
| | | ILFIKLWPSSPNIIA | LFIKLWPSS |
| | | KNLRRVKNKKLGLVI | VKNKKLGLV |
| | | NLRRVKNKKLGLVIP | VKNKKLGLV |

| | | | |
|---|---|---|---|
| | | LRRVKNKKLGLVIPK | VKNKKLGLV |
| | | LQKDMIILFIKLWPS | IILFIKLWP |
| | | RRVKNKKLGLVIPKA | VKNKKLGLV |
| | | LFIKLWPSSPNIIAT | LFIKLWPSS |
| | | KENALQYFKAYKKGK | LQYFKAYKK |
| | | DLYYTQVKNLRRVKN | YTQVKNLRR |
| | | ENALQYFKAYKKGKN | LQYFKAYKK |
| | | ADLYYTQVKNLRRVK | YTQVKNLRR |
| | | LYYTQVKNLRRVKNK | YTQVKNLRR |
| | | NALQYFKAYKKGKNS | LQYFKAYKK |
| | | KADLYYTQVKNLRRV | YTQVKNLRR |
| | | YTQVKNLRRVKNKKL | YTQVKNLRR |
| | | ATNSNFKILDAYYRR | FKILDAYYR |
| | | GNLCVFYTKLAKKSG | VFYTKLAKK |
| | | TNSNFKILDAYYRRP | FKILDAYYR |
| | | KGELILLNINKIQKG | LLNINKIQK |
| | | IATNSNFKILDAYYR | SNFKILDAY |
| | | NLCVFYTKLAKKSGK | VFYTKLAKK |
| | | LCVFYTKLAKKSGKA | VFYTKLAKK |
| | | SNFKILDAYYRRPKI | FKILDAYYR |
| | | NSNFKILDAYYRRPK | FKILDAYYR |
| | | AGNLCVFYTKLAKKS | VFYTKLAKK |
| | | EKGELILLNINKIQK | LILLNINKI |
| | | TQVKNLRRVKNKKLG | LRRVKNKKL |
| | | YYTQVKNLRRVKNKK | YTQVKNLRR |
| | | GELILLNINKIQKGI | LLNINKIQK |
| | | PKENALQYFKAYKKG | LQYFKAYKK |
| | | VKNKKLGLVIPKAEK | VKNKKLGLV |
| | | RVKNKKLGLVIPKAE | VKNKKLGLV |
| | HLA-DRB3*0101 | None | |
| | HLA-DRB4*0101 | ILFIKLWPSSPNIIA | LWPSSPNII |
| | | IILFIKLWPSSPNII | FIKLWPSSP |
| | | LFIKLWPSSPNIIAT | LWPSSPNII |
| | | FIKLWPSSPNIIATN | LWPSSPNII |
| | | IKLWPSSPNIIATNS | LWPSSPNII |
| | | KKFKILICLNPNTTR | LICLNPNTT |
| | | FKILICLNPNTTRFH | LICLNPNTT |
| | | KILICLNPNTTRFHI | LICLNPNTT |
| | | KFKILICLNPNTTRF | LICLNPNTT |
| | | RIISLEILQKDMIIL | LEILQKDMI |
| | | NKKFKILICLNPNTT | ILICLNPNT |
| | | IISLEILQKDMIILF | LEILQKDMI |
| | | ISLEILQKDMIILFI | LEILQKDMI |
| | | MIILFIKLWPSSPNI | IKLWPSSPN |
| | | KKLGLVIPKAEKNLH | LGLVIPKAE |
| | | DMIILFIKLWPSSPN | FIKLWPSSP |
| | | KLGLVIPKAEKNLHI | IPKAEKNLH |
| | | LGLVIPKAEKNLHIK | IPKAEKNLH |
| | | GLVIPKAEKNLHIKL | IPKAEKNLH |
| | | LVIPKAEKNLHIKLD | IPKAEKNLH |
| | | ERIISLEILQKDMII | LEILQKDMI |
| | | NERIISLEILQKDMI | ISLEILQKD |
| | | GKADLYYTQVKNLRR | YYTQVKNLR |
| | | KADLYYTQVKNLRRV | YYTQVKNLR |
| | | ADLYYTQVKNLRRVK | YYTQVKNLR |
| | | KLWPSSPNIIATNSN | LWPSSPNII |
| | | DLYYTQVKNLRRVKN | YYTQVKNLR |

| | HLA-DRB5*0101 | DLYYTQVKNLRRVKN | YYTQVKNLR |
|---|---|---|---|
| | | KNSFKTIQNQLKDNL | FKTIQNQLK |
| | | GKADLYYTQVKNLRR | YYTQVKNLR |
| | | NSFKTIQNQLKDNLD | FKTIQNQLK |
| | | ADLYYTQVKNLRRVK | YYTQVKNLR |
| | | KADLYYTQVKNLRRV | YYTQVKNLR |
| | | KKGKNSFKTIQNQLK | KKGKNSFKT |
| | | KGKNSFKTIQNQLKD | FKTIQNQLK |
| | | GKNSFKTIQNQLKDN | FKTIQNQLK |
| | | LYYTQVKNLRRVKNK | YTQVKNLRR |
| | | LQYFKAYKKGKNSFK | YFKAYKKGK |
| | | NSNFKILDAYYRRPK | FKILDAYYR |
| | | SNFKILDAYYRRPKI | FKILDAYYR |
| | | TNSNFKILDAYYRRP | FKILDAYYR |
| | | YYTQVKNLRRVKNKK | YYTQVKNLR |
| | | QYFKAYKKGKNSFKT | YKKGKNSFK |
| | | YFKAYKKGKNSFKTI | YKKGKNSFK |
| | | GAYVFIKNQKNKTPS | IKNQKNKTP |
| | | PGAYVFIKNQKNKTP | YVFIKNQKN |
| | | ENALQYFKAYKKGKN | YFKAYKKGK |
| | | NALQYFKAYKKGKNS | YFKAYKKGK |
| | | SGKADLYYTQVKNLR | ADLYYTQVK |
| | | SFKTIQNQLKDNLDK | FKTIQNQLK |
| | | ATNSNFKILDAYYRR | FKILDAYYR |
| | | FKTIQNQLKDNLDKF | FKTIQNQLK |
| | | LIPEEEYNQEKTAIK | LIPEEEYNQ |
| | | FKAYKKGKNSFKTIQ | YKKGKNSFK |
| | | AYVFIKNQKNKTPSL | IKNQKNKTP |
| | | KAYKKGKNSFKTIQN | YKKGKNSFK |
| | | IATNSNFKILDAYYR | IATNSNFKI |
| | | NTTRFHITKKNFKKN | FHITKKNFK |
| | | KENALQYFKAYKKGK | LQYFKAYKK |
| | | PNTTRFHITKKNFKK | FHITKKNFK |
| | | TTRFHITKKNFKKNA | FHITKKNFK |
| | | YVFIKNQKNKTPSLD | IKNQKNKTP |
| | | YTQVKNLRRVKNKKL | YTQVKNLRR |
| | | ALQYFKAYKKGKNSF | YFKAYKKGK |
| | | NFKILDAYYRRPKIK | FKILDAYYR |
| | | TRFHITKKNFKKNAL | FHITKKNFK |
| | | FKILDAYYRRPKIKE | FKILDAYYR |
| | | PEEEYNQEKTAIKEK | YNQEKTAIK |
| | | NPNTTRFHITKKNFK | RFHITKKNF |
| | | IPEEEYNQEKTAIKE | YNQEKTAIK |
| | | EEEYNQEKTAIKEKE | YNQEKTAIK |
| | | EEYNQEKTAIKEKEK | YNQEKTAIK |
| | | VFIKNQKNKTPSLDV | IKNQKNKTP |
| | | YPGAYVFIKNQKNKT | YVFIKNQKN |
| | | | |
| AAC44656.1\| P30 Borrelia burgdorferi <br><br> Seq21 | HLA-DRB1*0101 | VHQSFIPVPVHVTDK | FIPVPVHVT |
| | | LLVHQSFIPVPVHVT | HQSFIPVPV |
| | | LVHQSFIPVPVHVTD | FIPVPVHVT |
| | | HQSFIPVPVHVTDKY | FIPVPVHVT |
| | | QSFIPVPVHVTDKYG | FIPVPVHVT |
| | | ELGIRAIDSKTLEIT | IRAIDSKTL |
| | | LGIRAIDSKTLEITL | IRAIDSKTL |
| | | DSELGIRAIDSKTLE | IRAIDSKTL |
| | | TDSELGIRAIDSKTL | ELGIRAIDS |

| | | | |
|---|---|---|---|
| | | SELGIRAIDSKTLEI | IRAIDSKTL |
| | | TLYRGIVTGDPNTGG | IVTGDPNTG |
| | | DTLYRGIVTGDPNTG | YRGIVTGDP |
| | | MKLQRSLSLIIFSLT | LQRSLSLII |
| | | YRGIVTGDPNTGGHK | IVTGDPNTG |
| | | LYRGIVTGDPNTGGH | IVTGDPNTG |
| | | ITFYTTNDSSTAYKM | YTTNDSSTA |
| | | EEITFYTTNDSSTAY | YTTNDSSTA |
| | | SFIPVPVHVTDKYGQ | FIPVPVHVT |
| | | EITFYTTNDSSTAYK | YTTNDSSTA |
| | | LEEITFYTTNDSSTA | ITFYTTNDS |
| | | TFYTTNDSSTAYKMY | YTTNDSSTA |
| | | LRDNLTWSDGVAITA | LTWSDGVAI |
| | | FIPVPVHVTDKYGQN | FIPVPVHVT |
| | | RDNLTWSDGVAITAE | LTWSDGVAI |
| | | RGIVTGDPNTGGHKP | IVTGDPNTG |
| | | DNLTWSDGVAITAEG | LTWSDGVAI |
| | | TSPENMVTSGPFKLK | MVTSGPFKL |
| | | GIRAIDSKTLEITLA | IRAIDSKTL |
| | | NLTWSDGVAITAEGI | LTWSDGVAI |
| | | IRAIDSKTLEITLAS | IRAIDSKTL |
| | | LTWSDGVAITAEGIR | LTWSDGVAI |
| | | PENMVTSGPFKLKER | MVTSGPFKL |
| | | SPENMVTSGPFKLKE | MVTSGPFKL |
| | | WSDGVAITAEGIRKS | GVAITAEGI |
| | | GQNWTSPENMVTSGP | WTSPENMVT |
| | | SDGVAITAEGIRKSY | ITAEGIRKS |
| | | WTSPENMVTSGPFKL | WTSPENMVT |
| | | QNWTSPENMVTSGPF | WTSPENMVT |
| | | FYLRDNLTWSDGVAI | LRDNLTWSD |
| | | YLRDNLTWSDGVAIT | LTWSDGVAI |
| | | TLEITLASPKPYFID | LASPKPYFI |
| | | KTLEITLASPKPYFI | ITLASPKPY |
| | | ENMVTSGPFKLKERI | MVTSGPFKL |
| | | DKYGQNWTSPENMVT | GQNWTSPEN |
| | | KYGQNWTSPENMVTS | WTSPENMVT |
| | | GVAITAEGIRKSYLR | ITAEGIRKS |
| | | LEITLASPKPYFIDL | LASPKPYFI |
| | | DGVAITAEGIRKSYL | ITAEGIRKS |
| | | YGQNWTSPENMVTSG | WTSPENMVT |
| | | KSYLRILNKETGSTY | ILNKETGST |
| | | PYFIDLLVHQSFIPV | FIDLLVHQS |
| | | RKSYLRILNKETGST | YLRILNKET |
| | | GIVTGDPNTGGHKPG | IVTGDPNTG |
| | | EITLASPKPYFIDLL | LASPKPYFI |
| | | KPYFIDLLVHQSFIP | FIDLLVHQS |
| | | GAEPRSLDPQLAEDN | PRSLDPQLA |
| | | AEPRSLDPQLAEDNV | PRSLDPQLA |
| | | SPKPYFIDLLVHQSF | FIDLLVHQS |
| | | PKPYFIDLLVHQSFI | FIDLLVHQS |
| | | ASPKPYFIDLLVHQS | YFIDLLVHQ |
| | | FYTTNDSSTAYKMYV | YTTNDSSTA |
| | | EGVSTKISLGAEPRS | VSTKISLGA |
| | | VAITAEGIRKSYLRI | ITAEGIRKS |
| | | SYLRILNKETGSTYV | ILNKETGST |
| | | ITLASPKPYFIDLLV | LASPKPYFI |
| | | KLQRSLSLIIFSLTV | LQRSLSLII |

| | | | |
|---|---|---|---|
| | | LQRSLSLIIFSLTVL | LQRSLSLII |
| | | KERKEGVSTKISLGA | RKEGVSTKI |
| | | YLRILNKETGSTYVD | ILNKETGST |
| | | NMVTSGPFKLKERIP | MVTSGPFKL |
| | | ERKEGVSTKISLGAE | VSTKISLGA |
| | | YTTNDSSTAYKMYVN | YTTNDSSTA |
| | | KEGVSTKISLGAEPR | VSTKISLGA |
| | | AITAEGIRKSYLRIL | ITAEGIRKS |
| | | ITAEGIRKSYLRILN | ITAEGIRKS |
| | | RKEGVSTKISLGAEP | VSTKISLGA |
| | | FIDLLVHQSFIPVPV | FIDLLVHQS |
| | | LRILNKETGSTYVDM | ILNKETGST |
| | | ISLGAEPRSLDPQLA | LGAEPRSLD |
| | | IVTGDPNTGGHKPGL | IVTGDPNTG |
| | | NWTSPENMVTSGPFK | WTSPENMVT |
| | | VSTKISLGAEPRSLD | VSTKISLGA |
| | | EGIRKSYLRILNKET | IRKSYLRIL |
| | | GVSTKISLGAEPRSL | VSTKISLGA |
| | | RSLSLIIFSLTVLCC | LIIFSLTVL |
| | | DSKTLEITLASPKPY | EITLASPKP |
| | | SKTLEITLASPKPYF | ITLASPKPY |
| | | YFIDLLVHQSFIPVP | FIDLLVHQS |
| | | MIDTLYRGIVTGDPN | YRGIVTGDP |
| | | MVTSGPFKLKERIPS | MVTSGPFKL |
| | | KMIDTLYRGIVTGDP | MIDTLYRGI |
| | | SLSLIIFSLTVLCCD | IFSLTVLCC |
| | | PRSLDPQLAEDNVAS | PRSLDPQLA |
| | | LSLIIFSLTVLCCDN | IFSLTVLCC |
| | | IDTLYRGIVTGDPNT | YRGIVTGDP |
| | | EPRSLDPQLAEDNVA | PRSLDPQLA |
| | | SLGAEPRSLDPQLAE | PRSLDPQLA |
| | | IRKSYLRILNKETGS | YLRILNKET |
| | | GIRKSYLRILNKETG | IRKSYLRIL |
| | | DLLVHQSFIPVPHV | LVHQSFIPV |
| | | LGAEPRSLDPQLAED | PRSLDPQLA |
| | | QRSLSLIIFSLTVLC | LIIFSLTVL |
| | | SLIIFSLTVLCCDNK | IFSLTVLCC |
| | | IDLLVHQSFIPVPVH | LVHQSFIPV |
| | HLA-DRB1*0301 | None | |
| | HLA-DRB1*0401 | ASPKPYFIDLLVHQS | YFIDLLVHQ |
| | | SPKPYFIDLLVHQSF | YFIDLLVHQ |
| | | KPYFIDLLVHQSFIP | YFIDLLVHQ |
| | | PKPYFIDLLVHQSFI | YFIDLLVHQ |
| | | LASPKPYFIDLLVHQ | PYFIDLLVH |
| | | PYFIDLLVHQSFIPV | YFIDLLVHQ |
| | | YFIDLLVHQSFIPVP | YFIDLLVHQ |
| | | EEITFYTTNDSSTAY | YTTNDSSTA |
| | | EKYVFEKDNKYYDSN | YVFEKDNKY |
| | | SEKYVFEKDNKYYDS | YVFEKDNKY |
| | | PSEKYVFEKDNKYYD | YVFEKDNKY |
| | | RIPSEKYVFEKDNKY | RIPSEKYVF |
| | | IPSEKYVFEKDNKYY | YVFEKDNKY |
| | | LEEITFYTTNDSSTA | ITFYTTNDS |
| | | EITFYTTNDSSTAYK | YTTNDSSTA |
| | | ITFYTTNDSSTAYKM | YTTNDSSTA |

| | HLA-DRB1*0404 | RKSYLRILNKETGST | YLRILNKET |
|---|---|---|---|
| | | KSYLRILNKETGSTY | ILNKETGST |
| | | SYLRILNKETGSTYV | ILNKETGST |
| | | YLRILNKETGSTYVD | ILNKETGST |
| | | LRILNKETGSTYVDM | ILNKETGST |
| | HLA-DRB1*0405 | VTDKYGQNWTSPENM | GQNWTSPEN |
| | | HVTDKYGQNWTSPEN | YGQNWTSPE |
| | | TDKYGQNWTSPENMV | GQNWTSPEN |
| | | DKYGQNWTSPENMVT | GQNWTSPEN |
| | | KYGQNWTSPENMVTS | GQNWTSPEN |
| | | YGQNWTSPENMVTSG | GQNWTSPEN |
| | | GQNWTSPENMVTSGP | GQNWTSPEN |
| | | LVHQSFIPVPVHVTD | HQSFIPVPV |
| | | ELEEITFYTTNDSST | ITFYTTNDS |
| | | LEEITFYTTNDSSTA | ITFYTTNDS |
| | | EVELEEITFYTTNDS | EITFYTTND |
| | | LLVHQSFIPVPVHVT | HQSFIPVPV |
| | | VELEEITFYTTNDSS | ITFYTTNDS |
| | | YPFYLRDNLTWSDGV | LRDNLTWSD |
| | | PFYLRDNLTWSDGVA | LRDNLTWSD |
| | | TAYPFYLRDNLTWSD | YLRDNLTWS |
| | | FYLRDNLTWSDGVAI | LRDNLTWSD |
| | | EEITFYTTNDSSTAY | ITFYTTNDS |
| | | AYPFYLRDNLTWSDG | LRDNLTWSD |
| | HLA-DRB1*0701 | LLVHQSFIPVPVHVT | SFIPVPHV |
| | | LVHQSFIPVPVHVTD | FIPVPHVT |
| | | LGIRAIDSKTLEITL | IRAIDSKTL |
| | | VHQSFIPVPVHVTDK | FIPVPHVT |
| | | HQSFIPVPVHVTDKY | FIPVPHVT |
| | | ELGIRAIDSKTLEIT | IRAIDSKTL |
| | | MKLQRSLSLIIFSLT | MKLQRSLSL |
| | | SELGIRAIDSKTLEI | IRAIDSKTL |
| | | TDSELGIRAIDSKTL | ELGIRAIDS |
| | | DSELGIRAIDSKTLE | IRAIDSKTL |
| | | QSFIPVPVHVTDKYG | FIPVPHVT |
| | HLA-DRB1*0802 | MVTSGPFKLKERIPS | MVTSGPFKL |
| | | VTSGPFKLKERIPSE | FKLKERIPS |
| | | SGPFKLKERIPSEKY | FKLKERIPS |
| | | GPFKLKERIPSEKYV | FKLKERIPS |
| | | TSGPFKLKERIPSEK | FKLKERIPS |
| | | SKTLEITLASPKPYF | ITLASPKPY |
| | | KTLEITLASPKPYFI | ITLASPKPY |
| | | DSKTLEITLASPKPY | LEITLASPK |
| | | TLEITLASPKPYFID | ITLASPKPY |
| | | LEITLASPKPYFIDL | ITLASPKPY |
| | HLA-DRB1*0901 | MKLQRSLSLIIFSLT | LQRSLSLII |
| | | KTLEITLASPKPYFI | ITLASPKPY |
| | HLA-DRB1*1101 | MVTSGPFKLKERIPS | VTSGPFKLK |
| | | VTSGPFKLKERIPSE | FKLKERIPS |
| | | TSGPFKLKERIPSEK | FKLKERIPS |
| | | SGPFKLKERIPSEKY | FKLKERIPS |
| | | GPFKLKERIPSEKYV | FKLKERIPS |
| | HLA-DRB1*1302 | QRSLSLIIFSLTVLC | LSLIIFSLT |
| | | RSLSLIIFSLTVLCC | LSLIIFSLT |
| | | LQRSLSLIIFSLTVL | LSLIIFSLT |
| | | LSLIIFSLTVLCCDN | LIIFSLTVL |

| | | | |
|---|---|---|---|
| | | SLSLIIFSLTVLCCD | LIIFSLTVL |
| | | STYVDMVKSIIKNGQ | YVDMVKSII |
| | | TYVDMVKSIIKNGQE | VKSIIKNGQ |
| | | YVDMVKSIIKNGQEY | VKSIIKNGQ |
| | | MKLQRSLSLIIFSLT | LQRSLSLII |
| | | VDMVKSIIKNGQEYF | VKSIIKNGQ |
| | | KSYLRILNKETGSTY | LRILNKETG |
| | | KLQRSLSLIIFSLTV | LSLIIFSLT |
| | | SYLRILNKETGSTYV | LRILNKETG |
| | | DMVKSIIKNGQEYFD | VKSIIKNGQ |
| | | SLIIFSLTVLCCDNK | LIIFSLTVL |
| | | KTLEITLASPKPYFI | ITLASPKPY |
| | | GSTYVDMVKSIIKNG | YVDMVKSII |
| | | DSKTLEITLASPKPY | KTLEITLAS |
| | | SKTLEITLASPKPYF | ITLASPKPY |
| | | TLEITLASPKPYFID | ITLASPKPY |
| | | LEITLASPKPYFIDL | ITLASPKPY |
| | | RKSYLRILNKETGST | LRILNKETG |
| | | LIIFSLTVLCCDNKE | LIIFSLTVL |
| | | YLRILNKETGSTYVD | LRILNKETG |
| | | LRILNKETGSTYVDM | LRILNKETG |
| | | IRKSYLRILNKETGS | LRILNKETG |
| | | ETGSTYVDMVKSIIK | YVDMVKSII |
| | | TGSTYVDMVKSIIKN | YVDMVKSII |
| | | GIRKSYLRILNKETG | YLRILNKET |
| | | MVTSGPFKLKERIPS | MVTSGPFKL |
| | | WTSPENMVTSGPFKL | ENMVTSGPF |
| | | TSPENMVTSGPFKLK | MVTSGPFKL |
| | | MVKSIIKNGQEYFDG | VKSIIKNGQ |
| | | SPENMVTSGPFKLKE | MVTSGPFKL |
| | | GPFKLKERIPSEKYV | FKLKERIPS |
| | | VKSIIKNGQEYFDGQ | VKSIIKNGQ |
| | | SGPFKLKERIPSEKY | FKLKERIPS |
| | | TSGPFKLKERIPSEK | FKLKERIPS |
| | HLA-DRB1*1501 | RKSYLRILNKETGST | LRILNKETG |
| | | KSYLRILNKETGSTY | LRILNKETG |
| | | GIRKSYLRILNKETG | YLRILNKET |
| | | IRKSYLRILNKETGS | LRILNKETG |
| | | SYLRILNKETGSTYV | LRILNKETG |
| | | LQRSLSLIIFSLTVL | SLIIFSLTV |
| | | QRSLSLIIFSLTVLC | LIIFSLTVL |
| | | RSLSLIIFSLTVLCC | LIIFSLTVL |
| | | SLSLIIFSLTVLCCD | LIIFSLTVL |
| | | YPFYLRDNLTWSDGV | YLRDNLTWS |
| | | LSLIIFSLTVLCCDN | LIIFSLTVL |
| | | PFYLRDNLTWSDGVA | YLRDNLTWS |
| | | YLRILNKETGSTYVD | LRILNKETG |
| | | AYPFYLRDNLTWSDG | YLRDNLTWS |
| | | GTAYPFYLRDNLTWS | FYLRDNLTW |
| | | LRILNKETGSTYVDM | LRILNKETG |
| | | TAYPFYLRDNLTWSD | YLRDNLTWS |
| | HLA-DRB3*0101 | None | |
| | HLA-DRB4*0101 | YFIDLLVHQSFIPVP | IDLLVHQSF |
| | | FIDLLVHQSFIPVPV | LVHQSFIPV |
| | | IDLLVHQSFIPVPVH | LVHQSFIPV |
| | | PYFIDLLVHQSFIPV | IDLLVHQSF |

| | HLA-DRB5*0101 | None | |
|---|---|---|---|
| | | | |
| NP_045619.1\| immunogenic protein P37, putative [Borrelia burgdorferi B31]<br><br>Seq22 | HLA-DRB1*0101 | ASLDKIKSLLSTAKS | IKSLLSTAK |
| | | KASLDKIKSLLSTAK | LDKIKSLLS |
| | | LDKIKSLLSTAKSYL | IKSLLSTAK |
| | | SLDKIKSLLSTAKSY | IKSLLSTAK |
| | | DKIKSLLSTAKSYLE | IKSLLSTAK |
| | | QFMADAKASMSSTKS | MADAKASMS |
| | | NKLFILTILFSSVIS | LTILFSSVI |
| | | KIKSLLSTAKSYLEQ | IKSLLSTAK |
| | | INKLFILTILFSSVI | LFILTILFS |
| | | LFILTILFSSVISCK | LTILFSSVI |
| | | IKSLLSTAKSYLEQT | IKSLLSTAK |
| | | RGVGSSKANLALLPS | VGSSKANLA |
| | | KLFILTILFSSVISC | LTILFSSVI |
| | | YHQFMADAKASMSST | MADAKASMS |
| | | GYHQFMADAKASMSS | MADAKASMS |
| | | QTRRGVGSSKANLAL | VGSSKANLA |
| | | EQTRRGVGSSKANLA | RRGVGSSKA |
| | | HQFMADAKASMSSTK | MADAKASMS |
| | | FILTILFSSVISCKL | LTILFSSVI |
| | | RRGVGSSKANLALLP | VGSSKANLA |
| | | AGYHQFMADAKASMS | QFMADAKAS |
| | | MNLINKLFILTILFS | LINKLFILT |
| | | TRRGVGSSKANLALL | VGSSKANLA |
| | | FMADAKASMSSTKSL | MADAKASMS |
| | | MADAKASMSSTKSLL | MADAKASMS |
| | | LINKLFILTILFSSV | LFILTILFS |
| | | GVGSSKANLALLPSL | VGSSKANLA |
| | | VGSSKANLALLPSLE | VGSSKANLA |
| | | GSSKANLALLPSLEE | KANLALLPS |
| | | NLINKLFILTILFSS | LINKLFILT |
| | | SSKANLALLPSLEEA | LALLPSLEE |
| | | ILTILFSSVISCKLY | LTILFSSVI |
| | | ADAKASMSSTKSLLE | KASMSSTKS |
| | | VKASLDKIKSLLSTA | LDKIKSLLS |
| | | ELNDIYKKLQDMDSR | YKKLQDMDS |
| | | KVKASLDKIKSLLST | LDKIKSLLS |
| | | QELNDIYKKLQDMDS | LNDIYKKLQ |
| | | DAKASMSSTKSLLEV | KASMSSTKS |
| | | SKANLALLPSLEEAI | LALLPSLEE |
| | | KANLALLPSLEEAIA | LALLPSLEE |
| | | LTILFSSVISCKLYK | LTILFSSVI |
| | | EAIAKVKSNHASADT | IAKVKSNHA |
| | | AKVKASLDKIKSLLS | VKASLDKIK |
| | | YAGYHQFMADAKASM | QFMADAKAS |
| | | EEAIAKVKSNHASAD | IAKVKSNHA |
| | | NDFEYAQRKADRALE | YAQRKADRA |
| | | DFEYAQRKADRALEE | YAQRKADRA |
| | | KNDFEYAQRKADRAL | YAQRKADRA |
| | | YYAGYHQFMADAKAS | YYAGYHQFM |
| | | FEYAQRKADRALEEA | YAQRKADRA |
| | | SLEEAIAKVKSNHAS | IAKVKSNHA |
| | | GSFNTLKSNDDSKRS | FNTLKSNDD |
| | | ANLALLPSLEEAIAK | LALLPSLEE |
| | | LEEAIAKVKSNHASA | IAKVKSNHA |

| | | | |
|---|---|---|---|
| | | AKNDFEYAQRKADRA | FEYAQRKAD |
| | | AIAKVKSNHASADTH | VKSNHASAD |
| | | PSLEEAIAKVKSNHA | LEEAIAKVK |
| | | NGSFNTLKSNDDSKR | FNTLKSNDD |
| | | IAKVKSNHASADTHC | VKSNHASAD |
| | | KSLLSTAKSYLEQTR | LSTAKSYLE |
| | | CKLYKKITYNADQVI | LYKKITYNA |
| | | RALEEALSNSNASRH | LEEALSNSN |
| | | AKASMSSTKSLLEVA | KASMSSTKS |
| | HLA-DRB1*0301 | None | |
| | HLA-DRB1*0401 | PSLEEAIAKVKSNHA | PSLEEAIAK |
| | | SLEEAIAKVKSNHAS | IAKVKSNHA |
| | | LEEAIAKVKSNHASA | IAKVKSNHA |
| | | EEAIAKVKSNHASAD | IAKVKSNHA |
| | | EAIAKVKSNHASADT | IAKVKSNHA |
| | | IAKVKSNHASADTHC | IAKVKSNHA |
| | | AIAKVKSNHASADTH | IAKVKSNHA |
| | | RALEEALSNSNASRH | EALSNSNAS |
| | | DRALEEALSNSNASR | EALSNSNAS |
| | | ADRALEEALSNSNAS | LEEALSNSN |
| | | KASLDKIKSLLSTAK | LDKIKSLLS |
| | | ASLDKIKSLLSTAKS | LDKIKSLLS |
| | | LEEALSNSNASRHES | EALSNSNAS |
| | | ALEEALSNSNASRHE | EALSNSNAS |
| | | ISCKLYKKITYNADQ | LYKKITYNA |
| | | VISCKLYKKITYNAD | LYKKITYNA |
| | | SCKLYKKITYNADQV | LYKKITYNA |
| | | CKLYKKITYNADQVI | LYKKITYNA |
| | | NLINKLFILTILFSS | LFILTILFS |
| | | ADMQPDMQNDNSSSN | MQPDMQNDN |
| | | LINKLFILTILFSSV | LFILTILFS |
| | | NKLFILTILFSSVIS | LFILTILFS |
| | | INKLFILTILFSSVI | LFILTILFS |
| | HLA-DRB1*0404 | RSVDNTYMDQDTGKK | VDNTYMDQD |
| | | SVDNTYMDQDTGKKP | YMDQDTGKK |
| | | VDNTYMDQDTGKKPL | YMDQDTGKK |
| | | NTYMDQDTGKKPLMA | YMDQDTGKK |
| | | DNTYMDQDTGKKPLM | YMDQDTGKK |
| | | NKLFILTILFSSVIS | ILTILFSSV |
| | | INKLFILTILFSSVI | ILTILFSSV |
| | | LINKLFILTILFSSV | LFILTILFS |
| | | LFILTILFSSVISCK | ILTILFSSV |
| | | KLFILTILFSSVISC | ILTILFSSV |
| | | EEAIAKVKSNHASAD | IAKVKSNHA |
| | | EAIAKVKSNHASADT | IAKVKSNHA |
| | | RALEEALSNSNASRH | LEEALSNSN |
| | HLA-DRB1*0405 | GSSKANLALLPSLEE | KANLALLPS |
| | | SSKANLALLPSLEEA | LALLPSLEE |
| | | KANLALLPSLEEAIA | LALLPSLEE |
| | | SKANLALLPSLEEAI | LALLPSLEE |
| | | ANLALLPSLEEAIAK | LALLPSLEE |
| | | NLALLPSLEEAIAKV | LALLPSLEE |
| | | LALLPSLEEAIAKVK | LALLPSLEE |
| | | KSNNGSFNTLKSNDD | NGSFNTLKS |
| | | SNNGSFNTLKSNDDS | FNTLKSNDD |

|  |  | NNGSFNTLKSNDDSK | FNTLKSNDD |
|---|---|---|---|
|  |  | NGSFNTLKSNDDSKR | FNTLKSNDD |
|  |  | GSFNTLKSNDDSKRS | FNTLKSNDD |
|  |  | ADAKASMSSTKSLLE | KASMSSTKS |
|  |  | DAKASMSSTKSLLEV | MSSTKSLLE |
|  |  | KASMSSTKSLLEVAK | MSSTKSLLE |
|  |  | ASMSSTKSLLEVAKN | MSSTKSLLE |
|  |  | AKASMSSTKSLLEVA | MSSTKSLLE |
|  |  | KELNENMTKTNKDFQ | ENMTKTNKD |
|  |  | KQKELNENMTKTNKD | LNENMTKTN |
|  |  | QKELNENMTKTNKDF | ENMTKTNKD |
|  |  | ELNENMTKTNKDFQE | ENMTKTNKD |
|  |  | LNENMTKTNKDFQEL | ENMTKTNKD |
|  |  | ASLDKIKSLLSTAKS | LDKIKSLLS |
|  |  | KASLDKIKSLLSTAK | LDKIKSLLS |
|  |  | SFNTLKSNDDSKRSG | FNTLKSNDD |
|  |  | FNTLKSNDDSKRSGR | FNTLKSNDD |
|  | HLA-DRB1*0701 | RALEEALSNSNASRH | RALEEALSN |
|  |  | ALEEALSNSNASRHE | LSNSNASRH |
|  |  | EEALSNSNASRHESY | LSNSNASRH |
|  |  | EALSNSNASRHESYY | LSNSNASRH |
|  |  | LEEALSNSNASRHES | LSNSNASRH |
|  |  | ALSNSNASRHESYYY | LSNSNASRH |
|  |  | LSNSNASRHESYYYA | LSNSNASRH |
|  |  | DKIKSLLSTAKSYLE | LLSTAKSYL |
|  |  | KIKSLLSTAKSYLEQ | LLSTAKSYL |
|  |  | IKSLLSTAKSYLEQT | LLSTAKSYL |
|  |  | KSLLSTAKSYLEQTR | LLSTAKSYL |
|  |  | FILTILFSSVISCKL | LTILFSSVI |
|  |  | INKLFILTILFSSVI | ILTILFSSV |
|  |  | NKLFILTILFSSVIS | LTILFSSVI |
|  |  | LFILTILFSSVISCK | LTILFSSVI |
|  |  | KLFILTILFSSVISC | LTILFSSVI |
|  |  | LDKIKSLLSTAKSYL | SLLSTAKSY |
|  |  | SLLSTAKSYLEQTRR | LLSTAKSYL |
|  | HLA-DRB1*0802 | RALEEALSNSNASRH | RALEEALSN |
|  |  | ALEEALSNSNASRHE | LSNSNASRH |
|  |  | EEALSNSNASRHESY | LSNSNASRH |
|  |  | EALSNSNASRHESYY | LSNSNASRH |
|  |  | LEEALSNSNASRHES | LSNSNASRH |
|  |  | ALSNSNASRHESYYY | LSNSNASRH |
|  |  | LSNSNASRHESYYYA | LSNSNASRH |
|  |  | DKIKSLLSTAKSYLE | LLSTAKSYL |
|  |  | KIKSLLSTAKSYLEQ | LLSTAKSYL |
|  |  | IKSLLSTAKSYLEQT | LLSTAKSYL |
|  |  | KSLLSTAKSYLEQTR | LLSTAKSYL |
|  |  | FILTILFSSVISCKL | LTILFSSVI |
|  |  | INKLFILTILFSSVI | ILTILFSSV |
|  |  | NKLFILTILFSSVIS | LTILFSSVI |
|  |  | LFILTILFSSVISCK | LTILFSSVI |
|  |  | KLFILTILFSSVISC | LTILFSSVI |
|  |  | LDKIKSLLSTAKSYL | SLLSTAKSY |
|  |  | SLLSTAKSYLEQTRR | LLSTAKSYL |
|  | HLA-DRB1*0901 | None |  |
|  | HLA-DRB1*1101 | None |  |
|  | HLA-DRB1*1302 | MNLINKLFILTILFS | LINKLFILT |

| | | | |
|---|---|---|---|
| | | NLINKLFILTILFSS | LINKLFILT |
| | | LINKLFILTILFSSV | LINKLFILT |
| | | INKLFILTILFSSVI | LFILTILFS |
| | | NKLFILTILFSSVIS | LFILTILFS |
| | | QVIDKLKSNNGSFNT | LKSNNGSFN |
| | | VIDKLKSNNGSFNTL | LKSNNGSFN |
| | | KLFILTILFSSVISC | LTILFSSVI |
| | | IDKLKSNNGSFNTLK | LKSNNGSFN |
| | | DKLKSNNGSFNTLKS | LKSNNGSFN |
| | | LFILTILFSSVISCK | LTILFSSVI |
| | | DQVIDKLKSNNGSFN | IDKLKSNNG |
| | | RGVGSSKANLALLPS | VGSSKANLA |
| | HLA-DRB1*1501 | NKLFILTILFSSVIS | LFILTILFS |
| | | INKLFILTILFSSVI | LFILTILFS |
| | | LINKLFILTILFSSV | LFILTILFS |
| | | NLINKLFILTILFSS | LFILTILFS |
| | | LFILTILFSSVISCK | LTILFSSVI |
| | | KLFILTILFSSVISC | FILTILFSS |
| | | ASLDKIKSLLSTAKS | IKSLLSTAK |
| | | KASLDKIKSLLSTAK | LDKIKSLLS |
| | | LDKIKSLLSTAKSYL | IKSLLSTAK |
| | | MNLINKLFILTILFS | LINKLFILT |
| | | DKIKSLLSTAKSYLE | IKSLLSTAK |
| | | FILTILFSSVISCKL | LTILFSSVI |
| | | SLDKIKSLLSTAKSY | IKSLLSTAK |
| | HLA-DRB3*0101 | None | |
| | HLA-DRB4*0101 | GKKPLMADMQPDMQN | MADMQPDMQ |
| | | KKPLMADMQPDMQND | MADMQPDMQ |
| | | KPLMADMQPDMQNDN | MADMQPDMQ |
| | | PLMADMQPDMQNDNS | MADMQPDMQ |
| | | TGKKPLMADMQPDMQ | LMADMQPDM |
| | | LMADMQPDMQNDNSS | MADMQPDMQ |
| | | MADMQPDMQNDNSSS | MADMQPDMQ |
| | HLA-DRB5*0101 | None | |
| | | | |
| NP_212281.1\| flagellin [Borrelia burgdorferi B31] Seq23 | HLA-DRB1*0101 | TTNSILTQSAMAMIA | ILTQSAMAM |
| | | TNSILTQSAMAMIAQ | ILTQSAMAM |
| | | NSILTQSAMAMIAQA | ILTQSAMAM |
| | | ATTNSILTQSAMAMI | ILTQSAMAM |
| | | AATTNSILTQSAMAM | TNSILTQSA |
| | | QPAKINTPASLSGSQ | INTPASLSG |
| | | PAKINTPASLSGSQA | INTPASLSG |
| | | SILTQSAMAMIAQAN | ILTQSAMAM |
| | | MQPAKINTPASLSGS | INTPASLSG |
| | | AKINTPASLSGSQAS | INTPASLSG |
| | | ILTQSAMAMIAQANQ | ILTQSAMAM |
| | | GMQPAKINTPASLSG | KINTPASLS |
| | | NAIRMISDQRANLGA | IRMISDQRA |
| | | ENAIRMISDQRANLG | IRMISDQRA |
| | | ANLFSGEGAQTAQAA | FSGEGAQTA |
| | | ANVANLFSGEGAQTA | VANLFSGEG |
| | | VANLFSGEGAQTAQA | FSGEGAQTA |
| | | NLFSGEGAQTAQAAP | FSGEGAQTA |
| | | YNQMHMLSNKSASQN | MHMLSNKSA |
| | | NVANLFSGEGAQTAQ | FSGEGAQTA |
| | | EKVLVRMKELAVQSG | VRMKELAVQ |

| | | KVLVRMKELAVQSGN | MKELAVQSG |
| --- | --- | --- | --- |
| | | QAQYNQMHMLSNKSA | YNQMHMLSN |
| | | AQYNQMHMLSNKSAS | MHMLSNKSA |
| | | NQMHMLSNKSASQNV | MHMLSNKSA |
| | | AEELGMQPAKINTPA | LGMQPAKIN |
| | | VLVRMKELAVQSGNG | MKELAVQSG |
| | | TAEELGMQPAKINTP | LGMQPAKIN |
| | | QYNQMHMLSNKSASQ | MHMLSNKSA |
| | | AKIENAIRMISDQRA | IENAIRMIS |
| | | ATMTDEVVAATTNSI | MTDEVVAAT |
| | | LVRMKELAVQSGNGT | MKELAVQSG |
| | | MTDEVVAATTNSILT | VVAATTNSI |
| | | TDEVVAATTNSILTQ | VVAATTNSI |
| | | DEVVAATTNSILTQS | VVAATTNSI |
| | | RTAEELGMQPAKINT | LGMQPAKIN |
| | | EELGMQPAKINTPAS | LGMQPAKIN |
| | | TMTDEVVAATTNSIL | VVAATTNSI |
| | | VRMKELAVQSGNGTY | MKELAVQSG |
| | | VRTAEELGMQPAKIN | ELGMQPAKI |
| | | IENAIRMISDQRANL | IRMISDQRA |
| | | KIENAIRMISDQRAN | IRMISDQRA |
| | | INTPASLSGSQASWT | INTPASLSG |
| | | EYAIENLKASYAQIK | IENLKASYA |
| | | YAIENLKASYAQIKD | IENLKASYA |
| | | KINTPASLSGSQASW | INTPASLSG |
| | | LFSGEGAQTAQAAPV | FSGEGAQTA |
| | | FSGEGAQTAQAAPVQ | FSGEGAQTA |
| | | AIRMISDQRANLGAF | IRMISDQRA |
| | | IRMISDQRANLGAFQ | IRMISDQRA |
| | | VQQEGAQQPAPATAP | VQQEGAQQP |
| | | RMKELAVQSGNGTYS | MKELAVQSG |
| | | MKELAVQSGNGTYSD | MKELAVQSG |
| | | EGVQQEGAQQPAPAT | VQQEGAQQP |
| | | STEYAIENLKASYAQ | IENLKASYA |
| | | NSTEYAIENLKASYA | YAIENLKAS |
| | | TEYAIENLKASYAQI | IENLKASYA |
| | | QMHMLSNKSASQNVR | MHMLSNKSA |
| | | MHMLSNKSASQNVRT | MHMLSNKSA |
| | | TQSAMAMIAQANQVP | TQSAMAMIA |
| | | AIAVNIYAANVANLF | VNIYAANVA |
| | | LGMQPAKINTPASLS | LGMQPAKIN |
| | | IAVNIYAANVANLFS | YAANVANLF |
| | | AIENLKASYAQIKDA | LKASYAQIK |
| | | IENLKASYAQIKDAT | LKASYAQIK |
| | | ELGMQPAKINTPASL | LGMQPAKIN |
| | | LTQSAMAMIAQANQV | AMAMIAQAN |
| | | QEGVQQEGAQQPAPA | VQQEGAQQP |
| | | QSAMAMIAQANQVPQ | MIAQANQVP |
| | | EVVAATTNSILTQSA | VVAATTNSI |
| | | AVNIYAANVANLFSG | YAANVANLF |
| | | PVQEGVQQEGAQQPA | VQQEGAQQP |
| | | VVAATTNSILTQSAM | VVAATTNSI |
| | | APVQEGVQQEGAQQP | QEGVQQEGA |
| | | VQEGVQQEGAQQPAP | VQQEGAQQP |
| | | NIYAANVANLFSGEG | YAANVANLF |
| | | QEGAQQPAPATAPSQ | QQPAPATAP |
| | | VNIYAANVANLFSGE | YAANVANLF |

| | | | |
|---|---|---|---|
| | | QQEGAQQPAPATAPS | QQPAPATAP |
| | | GVQQEGAQQPAPATA | VQQEGAQQP |
| | | AANVANLFSGEGAQT | VANLFSGEG |
| | | EGAQQPAPATAPSQG | QQPAPATAP |
| | | AMAMIAQANQVPQYV | MIAQANQVP |
| | | EAIAVNIYAANVANL | VNIYAANVA |
| | | ENLKASYAQIKDATM | LKASYAQIK |
| | | VEKVLVRMKELAVQS | VRMKELAVQ |
| | | SAMAMIAQANQVPQY | MIAQANQVP |
| | | GAQQPAPATAPSQGG | QQPAPATAP |
| | | EVEKVLVRMKELAVQ | LVRMKELAV |
| | | NAQIRGLSQASRNTS | IRGLSQASR |
| | | KELAVQSGNGTYSDA | VQSGNGTYS |
| | | NLKASYAQIKDATMT | LKASYAQIK |
| | | LKASYAQIKDATMTD | LKASYAQIK |
| | | INAQIRGLSQASRNT | IRGLSQASR |
| | | AQIRGLSQASRNTSK | IRGLSQASR |
| | | DEAIAVNIYAANVAN | VNIYAANVA |
| | | GEGAQTAQAAPVQEG | AQTAQAAPV |
| | | PSQGGVNSPVNVTTT | VNSPVNVTT |
| | | DQAQYNQMHMLSNKS | YNQMHMLSN |
| | | EGAQTAQAAPVQEGV | AQTAQAAPV |
| | | SQGGVNSPVNVTTTV | VNSPVNVTT |
| | | QGGVNSPVNVTTTVD | VNSPVNVTT |
| | | APSQGGVNSPVNVTT | GVNSPVNVT |
| | | QDEAIAVNIYAANVA | AVNIYAANV |
| | | PASLSGSQASWTLRV | LSGSQASWT |
| | | ASLSGSQASWTLRVH | LSGSQASWT |
| | | MAMIAQANQVPQYVL | MIAQANQVP |
| | | IYAANVANLFSGEGA | VANLFSGEG |
| | | GGVNSPVNVTTTVDA | VNSPVNVTT |
| | | SRNNGINAANLSKTQ | INAANLSKT |
| | | RNNGINAANLSKTQE | INAANLSKT |
| | | ASRNNGINAANLSKT | NGINAANLS |
| | | ELAVQSGNGTYSDAD | VQSGNGTYS |
| | | NNGINAANLSKTQEK | INAANLSKT |
| | | NTPASLSGSQASWTL | LSGSQASWT |
| | | LAVQSGNGTYSDADR | VQSGNGTYS |
| | | NGINAANLSKTQEKL | INAANLSKT |
| | | YAANVANLFSGEGAQ | VANLFSGEG |
| | | TPASLSGSQASWTLR | LSGSQASWT |
| | | KINAQIRGLSQASRN | IRGLSQASR |
| | | GKINAQIRGLSQASR | QIRGLSQAS |
| | | SGEGAQTAQAAPVQE | AQTAQAAPV |
| | | AQQPAPATAPSQGGV | PATAPSQGG |
| | | ADQAQYNQMHMLSNK | YNQMHMLSN |
| | | HMLSNKSASQNVRTA | LSNKSASQN |
| | | IADQAQYNQMHMLSN | QYNQMHMLS |
| | | VNVTTTVDANTSLAK | VTTTVDANT |
| | | KASYAQIKDATMTDE | AQIKDATMT |
| | | QIRGLSQASRNTSKA | IRGLSQASR |
| | | ASYAQIKDATMTDEV | AQIKDATMT |
| | | GAQTAQAAPVQEGVQ | AQAAPVQEG |
| | | QQPAPATAPSQGGVN | PATAPSQGG |
| | | AMIAQANQVPQYVLS | MIAQANQVP |
| | | GVNSPVNVTTTVDAN | VNVTTTVDA |
| | | IRGLSQASRNTSKAI | IRGLSQASR |

| | | | |
|---|---|---|---|
| | | DATMTDEVVAATTNS | MTDEVVAAT |
| | | AQTAQAAPVQEGVQQ | AQAAPVQEG |
| | | RMISDQRANLGAFQN | ISDQRANLG |
| | | MIAQANQVPQYVLSL | QANQVPQYV |
| | | VNSPVNVTTTVDANT | VNVTTTVDA |
| | | SLSGSQASWTLRVHV | LSGSQASWT |
| | | LSGSQASWTLRVHVG | LSGSQASWT |
| | | SKAINFIQTTEGNLN | INFIQTTEG |
| | | MIINHNTSAINASRN | INHNTSAIN |
| | | SYAQIKDATMTDEVV | AQIKDATMT |
| | | NSPVNVTTTVDANTS | VNVTTTVDA |
| | | SPVNVTTTVDANTSL | VNVTTTVDA |
| | | NVTTTVDANTSLAKI | VDANTSLAK |
| | | KAINFIQTTEGNLNE | INFIQTTEG |
| | | QPAPATAPSQGGVNS | PATAPSQGG |
| | | IKDATMTDEVVAATT | MTDEVVAAT |
| | | MLSNKSASQNVRTAE | LSNKSASQN |
| | | KDATMTDEVVAATTN | MTDEVVAAT |
| | | QIKDATMTDEVVAAT | DATMTDEVV |
| | | PAPATAPSQGGVNSP | PATAPSQGG |
| | | QTAQAAPVQEGVQQE | AQAAPVQEG |
| | | VTTTVDANTSLAKIE | VDANTSLAK |
| | | PVNVTTTVDANTSLA | VNVTTTVDA |
| | | VAATTNSILTQSAMA | TNSILTQSA |
| | HLA-DRB1*0301 | None | |
| | HLA-DRB1*0401 | QYNQMHMLSNKSASQ | QMHMLSNKS |
| | | YNQMHMLSNKSASQN | QMHMLSNKS |
| | | IAVNIYAANVANLFS | YAANVANLF |
| | | QAQYNQMHMLSNKSA | QMHMLSNKS |
| | | NQMHMLSNKSASQNV | MLSNKSASQ |
| | | AIAVNIYAANVANLF | NIYAANVAN |
| | | NIYAANVANLFSGEG | YAANVANLF |
| | | AVNIYAANVANLFSG | YAANVANLF |
| | | AQYNQMHMLSNKSAS | QMHMLSNKS |
| | | VNIYAANVANLFSGE | YAANVANLF |
| | | QMHMLSNKSASQNVR | MLSNKSASQ |
| | | GVNSPVNVTTTVDAN | VNVTTTVDA |
| | | GGVNSPVNVTTTVDA | VNSPVNVTT |
| | | NSPVNVTTTVDANTS | VNVTTTVDA |
| | | VNSPVNVTTTVDANT | VNVTTTVDA |
| | HLA-DRB1*0404 | ANVANLFSGEGAQTA | VANLFSGEG |
| | | AANVANLFSGEGAQT | VANLFSGEG |
| | | NIYAANVANLFSGEG | YAANVANLF |
| | | IYAANVANLFSGEGA | VANLFSGEG |
| | | YAANVANLFSGEGAQ | VANLFSGEG |
| | | YNQMHMLSNKSASQN | MLSNKSASQ |
| | | QYNQMHMLSNKSASQ | QMHMLSNKS |
| | | NVANLFSGEGAQTAQ | VANLFSGEG |
| | | VANLFSGEGAQTAQA | VANLFSGEG |
| | | ENAIRMISDQRANLG | AIRMISDQR |
| | | NQMHMLSNKSASQNV | MLSNKSASQ |
| | | NAIRMISDQRANLGA | ISDQRANLG |
| | | QMHMLSNKSASQNVR | MLSNKSASQ |
| | | AIRMISDQRANLGAF | ISDQRANLG |
| | | VNVTTTVDANTSLAK | TVDANTSLA |

EP 2 254 592 B1

| | | PVNVTTTVDANTSLA | VNVTTTVDA |
|---|---|---|---|
| | | IRMISDQRANLGAFQ | ISDQRANLG |
| | | AQYNQMHMLSNKSAS | QMHMLSNKS |
| | | QAQYNQMHMLSNKSA | QMHMLSNKS |
| | | DQAQYNQMHMLSNKS | YNQMHMLSN |
| | | RMISDQRANLGAFQN | ISDQRANLG |
| | | MTDEVVAATTNSILT | VVAATTNSI |
| | HLA-DRB1*0405 | EELGMQPAKINTPAS | LGMQPAKIN |
| | | LGMQPAKINTPASLS | PAKINTPAS |
| | | GGVNSPVNVTTTVDA | PVNVTTTVD |
| | | GVNSPVNVTTTVDAN | VNVTTTVDA |
| | | VNSPVNVTTTVDANT | VNVTTTVDA |
| | | ELGMQPAKINTPASL | PAKINTPAS |
| | | NSPVNVTTTVDANTS | VNVTTTVDA |
| | HLA-DRB1*0701 | TMTDEVVAATTNSIL | VVAATTNSI |
| | | MTDEVVAATTNSILT | VVAATTNSI |
| | | TDEVVAATTNSILTQ | VVAATTNSI |
| | | DEVVAATTNSILTQS | VVAATTNSI |
| | | EVVAATTNSILTQSA | VAATTNSIL |
| | | ATMTDEVVAATTNSI | ATMTDEVVA |
| | | VVAATTNSILTQSAM | VVAATTNSI |
| | | QNRLESIKNSTEYAI | LESIKNSTE |
| | | NRLESIKNSTEYAIE | IKNSTEYAI |
| | | ESIKNSTEYAIENLK | IKNSTEYAI |
| | | RLESIKNSTEYAIEN | IKNSTEYAI |
| | | LESIKNSTEYAIENL | IKNSTEYAI |
| | | GGVNSPVNVTTTVDA | PVNVTTTVD |
| | | GVNSPVNVTTTVDAN | VNVTTTVDA |
| | | MIINHNTSAINASRN | IINHNTSAI |
| | HLA-DRB1*0802 | None | |
| | HLA-DRB1*0901 | IAVNIYAANVANLFS | VNIYAANVA |
| | | AIAVNIYAANVANLF | VNIYAANVA |
| | | TTNSILTQSAMAMIA | LTQSAMAMI |
| | | TNSILTQSAMAMIAQ | LTQSAMAMI |
| | | NSILTQSAMAMIAQA | LTQSAMAMI |
| | | SILTQSAMAMIAQAN | LTQSAMAMI |
| | | ATTNSILTQSAMAMI | ILTQSAMAM |
| | | EAIAVNIYAANVANL | VNIYAANVA |
| | | AVNIYAANVANLFSG | IYAANVANL |
| | | VNIYAANVANLFSGE | IYAANVANL |
| | HLA-DRB1*1101 | None | |
| | HLA-DRB1*1302 | NTSLAKIENAIRMIS | AKIENAIRM |
| | | TSLAKIENAIRMISD | IENAIRMIS |
| | | SLAKIENAIRMISDQ | IENAIRMIS |
| | | ASRNNGINAANLSKT | NGINAANLS |
| | | SRNNGINAANLSKTQ | INAANLSKT |
| | | LAKIENAIRMISDQR | IENAIRMIS |
| | | AKIENAIRMISDQRA | IENAIRMIS |
| | | RNNGINAANLSKTQE | INAANLSKT |
| | | MGVSGKINAQIRGLS | MGVSGKINA |
| | | MIINHNTSAINASRN | IINHNTSAI |
| | | NNGINAANLSKTQEK | INAANLSKT |
| | | NGINAANLSKTQEKL | INAANLSKT |
| | | IINHNTSAINASRNN | IINHNTSAI |
| | | GVSGKINAQIRGLSQ | INAQIRGLS |
| | | VSGKINAQIRGLSQA | INAQIRGLS |

1343

| | | RMKELAVQSGNGTYS | LAVQSGNGT |
|---|---|---|---|
| | | AIAVNIYAANVANLF | VNIYAANVA |
| | | IENAIRMISDQRANL | IENAIRMIS |
| | | IAVNIYAANVANLFS | VNIYAANVA |
| | | MKELAVQSGNGTYSD | LAVQSGNGT |
| | | SGKINAQIRGLSQAS | INAQIRGLS |
| | | GKINAQIRGLSQASR | INAQIRGLS |
| | | KIENAIRMISDQRAN | IENAIRMIS |
| | | KELAVQSGNGTYSDA | LAVQSGNGT |
| | | LVRMKELAVQSGNGT | MKELAVQSG |
| | | VRMKELAVQSGNGTY | LAVQSGNGT |
| | | AGMGVSGKINAQIRG | MGVSGKINA |
| | | AVNIYAANVANLFSG | YAANVANLF |
| | | VNIYAANVANLFSGE | YAANVANLF |
| | | SQGGVNSPVNVTTTV | GVNSPVNVT |
| | | QGGVNSPVNVTTTVD | GVNSPVNVT |
| | | GGVNSPVNVTTTVDA | GVNSPVNVT |
| | | INASRNNGINAANLS | SRNNGINAA |
| | | EAIAVNIYAANVANL | VNIYAANVA |
| | | PSQGGVNSPVNVTTT | GVNSPVNVT |
| | | APSQGGVNSPVNVTT | GVNSPVNVT |
| | | QDEAIAVNIYAANVA | IAVNIYAAN |
| | | DEAIAVNIYAANVAN | VNIYAANVA |
| | | GVNSPVNVTTTVDAN | GVNSPVNVT |
| | | NASRNNGINAANLSK | NGINAANLS |
| | | RGLSQASRNTSKAIN | LSQASRNTS |
| | | ELAVQSGNGTYSDAD | LAVQSGNGT |
| | | IRGLSQASRNTSKAI | LSQASRNTS |
| | | LAVQSGNGTYSDADR | LAVQSGNGT |
| | | TSAINASRNNGINAA | AINASRNNG |
| | | QIRGLSQASRNTSKA | LSQASRNTS |
| | | INHNTSAINASRNNG | INHNTSAIN |
| | | NAQIRGLSQASRNTS | RGLSQASRN |
| | | AQIRGLSQASRNTSK | LSQASRNTS |
| | | QNRLESIKNSTEYAI | LESIKNSTE |
| | | NIYAANVANLFSGEG | YAANVANLF |
| | | NRLESIKNSTEYAIE | IKNSTEYAI |
| | | GMGVSGKINAQIRGL | MGVSGKINA |
| | | SKAINFIQTTEGNLN | INFIQTTEG |
| | HLA-DRB1*1501 | EKVLVRMKELAVQSG | LVRMKELAV |
| | | KVLVRMKELAVQSGN | LVRMKELAV |
| | | VEKVLVRMKELAVQS | LVRMKELAV |
| | | EVEKVLVRMKELAVQ | LVRMKELAV |
| | | VLVRMKELAVQSGNG | VRMKELAVQ |
| | | LVRMKELAVQSGNGT | VRMKELAVQ |
| | | INAQIRGLSQASRNT | IRGLSQASR |
| | | GKINAQIRGLSQASR | QIRGLSQAS |
| | | NAQIRGLSQASRNTS | IRGLSQASR |
| | | KINAQIRGLSQASRN | IRGLSQASR |
| | | AQIRGLSQASRNTSK | IRGLSQASR |
| | | NEVEKVLVRMKELAV | VLVRMKELA |
| | | KIENAIRMISDQRAN | AIRMISDQR |
| | | IENAIRMISDQRANL | AIRMISDQR |
| | | AKIENAIRMISDQRA | AIRMISDQR |
| | | ENAIRMISDQRANLG | AIRMISDQR |
| | | LAKIENAIRMISDQR | LAKIENAIR |
| | | QYNQMHMLSNKSASQ | MHMLSNKSA |

| | | | |
|---|---|---|---|
| | | QIRGLSQASRNTSKA | IRGLSQASR |
| | | YNQMHMLSNKSASQN | MHMLSNKSA |
| | | IRGLSQASRNTSKAI | IRGLSQASR |
| | | AQYNQMHMLSNKSAS | MHMLSNKSA |
| | | VRMKELAVQSGNGTY | VRMKELAVQ |
| | | QAQYNQMHMLSNKSA | QMHMLSNKS |
| | | AIRMISDQRANLGAF | AIRMISDQR |
| | | NQMHMLSNKSASQNV | MHMLSNKSA |
| | | QASWTLRVHVGANQD | LRVHVGANQ |
| | | NAIRMISDQRANLGA | AIRMISDQR |
| | | SQASWTLRVHVGANQ | QASWTLRVH |
| | HLA-DRB3*0101 | None | |
| | HLA-DRB4*0101 | EVEKVLVRMKELAVQ | VEKVLVRMK |
| | | VEKVLVRMKELAVQS | VRMKELAVQ |
| | | EKVLVRMKELAVQSG | VRMKELAVQ |
| | | KVLVRMKELAVQSGN | VRMKELAVQ |
| | | VLVRMKELAVQSGNG | VRMKELAVQ |
| | | ENAIRMISDQRANLG | IRMISDQRA |
| | | NAIRMISDQRANLGA | IRMISDQRA |
| | | VRMKELAVQSGNGTY | VRMKELAVQ |
| | | LVRMKELAVQSGNGT | VRMKELAVQ |
| | | AIRMISDQRANLGAF | ISDQRANLG |
| | | IRMISDQRANLGAFQ | ISDQRANLG |
| | HLA-DRB5*0101 | NTSLAKIENAIRMIS | AKIENAIRM |
| | | TSLAKIENAIRMISD | AKIENAIRM |
| | | DANTSLAKIENAIRM | LAKIENAIR |
| | | ANTSLAKIENAIRMI | AKIENAIRM |
| | | SLAKIENAIRMISDQ | AKIENAIRM |
| | | | |
| NP_212463.1| oligopeptide ABC transporter, periplasmic oligopeptide-binding protein (oppA-2) [Borrelia burgdorferi B31]<br><br>Seq24 | HLA-DRB1*0101 | LTFLSIFTQGYTQFS | FLSIFTQGY |
| | | TFLSIFTQGYTQFSS | FTQGYTQFS |
| | | FLSIFTQGYTQFSSH | FTQGYTQFS |
| | | LSIFTQGYTQFSSHN | FTQGYTQFS |
| | | SIFTQGYTQFSSHNY | FTQGYTQFS |
| | | DNVKIRKALTLAIDR | IRKALTLAI |
| | | LDNVKIRKALTLAID | IRKALTLAI |
| | | PLDNVKIRKALTLAI | VKIRKALTL |
| | | VHQSFIPVPVHVTEK | FIPVPVHVT |
| | | NVKIRKALTLAIDRE | IRKALTLAI |
| | | VKIRKALTLAIDRET | IRKALTLAI |
| | | MLVHQSFIPVPVHVT | HQSFIPVPV |
| | | LVHQSFIPVPVHVTE | FIPVPVHVT |
| | | HQSFIPVPVHVTEKY | FIPVPVHVT |
| | | QSFIPVPVHVTEKYG | FIPVPVHVT |
| | | IFTQGYTQFSSHNYS | FTQGYTQFS |
| | | TMFRGIVTGDPNTGG | IVTGDPNTG |
| | | FTQGYTQFSSHNYSN | FTQGYTQFS |
| | | DTMFRGIVTGDPNTG | FRGIVTGDP |
| | | FRGIVTGDPNTGGNK | IVTGDPNTG |
| | | MFRGIVTGDPNTGGN | IVTGDPNTG |
| | | SLELFNPEIAKTLLA | FNPEIAKTL |
| | | LELFNPEIAKTLLAE | FNPEIAKTL |
| | | KSLELFNPEIAKTLL | FNPEIAKTL |
| | | AKSLELFNPEIAKTL | LFNPEIAKT |
| | | KIRKALTLAIDRETL | IRKALTLAI |
| | | ITFYTTNDSSTAYKM | YTTNDSSTA |

| | | EEITFYTTNDSSTAY | YTTNDSSTA |
|---|---|---|---|
| | | EITFYTTNDSSTAYK | YTTNDSSTA |
| | | IRKALTLAIDRETLT | IRKALTLAI |
| | | ELFNPEIAKTLLAEA | FNPEIAKTL |
| | | SFIPVPVHVTEKYGQ | FIPVPVHVT |
| | | LEEITFYTTNDSSTA | ITFYTTNDS |
| | | TFYTTNDSSTAYKMY | YTTNDSSTA |
| | | FIPVPVHVTEKYGQN | FIPVPVHVT |
| | | RGIVTGDPNTGGNKP | IVTGDPNTG |
| | | TSPENMVTSGPFKLK | MVTSGPFKL |
| | | REKITWSDGVAITAE | ITWSDGVAI |
| | | LREKITWSDGVAITA | ITWSDGVAI |
| | | EKITWSDGVAITAEG | ITWSDGVAI |
| | | YKVLDNGTTPTRRAT | LDNGTTPTR |
| | | DAIFGSIPPDLIKNL | FGSIPPDLI |
| | | KITWSDGVAITAEGI | ITWSDGVAI |
| | | PYFIDMLVHQSFIPV | FIDMLVHQS |
| | | PENMVTSGPFKLKER | MVTSGPFKL |
| | | LDAIFGSIPPDLIKN | FGSIPPDLI |
| | | TYKVLDNGTTPTRRA | LDNGTTPTR |
| | | IKPLDNVKIRKALTL | IKPLDNVKI |
| | | KPYFIDMLVHQSFIP | FIDMLVHQS |
| | | SPENMVTSGPFKLKE | MVTSGPFKL |
| | | ELDAIFGSIPPDLIK | FGSIPPDLI |
| | | EELDAIFGSIPPDLI | IFGSIPPDL |
| | | ITWSDGVAITAEGIR | ITWSDGVAI |
| | | WSDGVAITAEGIRKS | GVAITAEGI |
| | | LTYKVLDNGTTPTRR | VLDNGTTPT |
| | | GQNWTSPENMVTSGP | WTSPENMVT |
| | | TLTYKVLDNGTTPTR | YKVLDNGTT |
| | | SDGVAITAEGIRKSY | ITAEGIRKS |
| | | PKPYFIDMLVHQSFI | FIDMLVHQS |
| | | WTSPENMVTSGPFKL | WTSPENMVT |
| | | LFNPEIAKTLLAEAG | FNPEIAKTL |
| | | SPKPYFIDMLVHQSF | FIDMLVHQS |
| | | QNWTSPENMVTSGPF | WTSPENMVT |
| | | NTHIKPLDNVKIRKA | IKPLDNVKI |
| | | THIKPLDNVKIRKAL | IKPLDNVKI |
| | | ENMVTSGPFKLKERI | MVTSGPFKL |
| | | AIFGSIPPDLIKNLK | FGSIPPDLI |
| | | FNPEIAKTLLAEAGY | FNPEIAKTL |
| | | ESPKPYFIDMLVHQS | YFIDMLVHQ |
| | | PDLIKNLKLRSDYYS | IKNLKLRSD |
| | | KPLDNVKIRKALTLA | VKIRKALTL |
| | | EKYGQNWTSPENMVT | GQNWTSPEN |
| | | KYGQNWTSPENMVTS | WTSPENMVT |
| | | VSFKISLGAEPSSLD | FKISLGAEP |
| | | GVAITAEGIRKSYLR | ITAEGIRKS |
| | | DLIKNLKLRSDYYSS | IKNLKLRSD |
| | | DGVAITAEGIRKSYL | ITAEGIRKS |
| | | PEIAKTLLAEAGYPN | AKTLLAEAG |
| | | YGQNWTSPENMVTSG | WTSPENMVT |
| | | EIAKTLLAEAGYPNG | AKTLLAEAG |
| | | LKLRSDYYSSAVNAI | LKLRSDYYS |
| | | FNTHIKPLDNVKIRK | IKPLDNVKI |
| | | YAFNTHIKPLDNVKI | FNTHIKPLD |
| | | AFNTHIKPLDNVKIR | IKPLDNVKI |

| | | | |
|---|---|---|---|
| | | KVLDNGTTPTRRATP | LDNGTTPTR |
| | | NLREKITWSDGVAIT | ITWSDGVAI |
| | | MKLQRSLFLIIFFLT | MKLQRSLFL |
| | | GIVTGDPNTGGNKPG | IVTGDPNTG |
| | | SFKISLGAEPSSLDP | LGAEPSSLD |
| | | FNLREKITWSDGVAI | LREKITWSD |
| | | NEEWTTYLNTKANGN | WTTYLNTKA |
| | | EEWTTYLNTKANGNY | YLNTKANGN |
| | | IAPIYIYGNSYLFRN | YIYGNSYLF |
| | | KLRSDYYSSAVNAIY | YYSSAVNAI |
| | | FIDMLVHQSFIPVPV | FIDMLVHQS |
| | | ETLTYKVLDNGTTPT | YKVLDNGTT |
| | | LRSDYYSSAVNAIYF | YYSSAVNAI |
| | | APIYIYGNSYLFRND | YIYGNSYLF |
| | | YFIDMLVHQSFIPVP | FIDMLVHQS |
| | | RSDYYSSAVNAIYFY | YYSSAVNAI |
| | | FKISLGAEPSSLDPQ | LGAEPSSLD |
| | | VAITAEGIRKSYLRI | ITAEGIRKS |
| | | SDYYSSAVNAIYFYA | YYSSAVNAI |
| | | IGDYADPLTFLSIFT | YADPLTFLS |
| | | AGWIGDYADPLTFLS | IGDYADPLT |
| | | PIYIYGNSYLFRNDK | YIYGNSYLF |
| | | FYTTNDSSTAYKMYE | YTTNDSSTA |
| | | GDYADPLTFLSIFTQ | YADPLTFLS |
| | | GWIGDYADPLTFLSI | YADPLTFLS |
| | | SIPPDLIKNLKLRSD | LIKNLKLRS |
| | | YADPLTFLSIFTQGY | YADPLTFLS |
| | | IPPDLIKNLKLRSDY | IKNLKLRSD |
| | | NMVTSGPFKLKERIP | MVTSGPFKL |
| | | NPEIAKTLLAEAGYP | AKTLLAEAG |
| | | LIKNLKLRSDYYSSA | LKLRSDYYS |
| | | PPDLIKNLKLRSDYY | IKNLKLRSD |
| | | VLDNGTTPTRRATPN | NGTTPTRRA |
| | | WIGDYADPLTFLSIF | YADPLTFLS |
| | | ISLGAEPSSLDPQLA | LGAEPSSLD |
| | | AITAEGIRKSYLRIL | ITAEGIRKS |
| | | ITAEGIRKSYLRILN | ITAEGIRKS |
| | | KISLGAEPSSLDPQL | LGAEPSSLD |
| | | YTTNDSSTAYKMYEN | YTTNDSSTA |
| | | GVSFKISLGAEPSSL | FKISLGAEP |
| | | LAKGWDISSDGTVYT | GWDISSDGT |
| | | IKNLKLRSDYYSSAV | LKLRSDYYS |
| | | EKDFPIAPIYIYGNS | FPIAPIYIY |
| | | IVTGDPNTGGNKPGL | IVTGDPNTG |
| | | PLTFLSIFTQGYTQF | FLSIFTQGY |
| | | IEKDFPIAPIYIYGN | FPIAPIYIY |
| | | AKGWDISSDGTVYTF | ISSDGTVYT |
| | | GWDISSDGTVYTFNL | ISSDGTVYT |
| | | EGVSFKISLGAEPSS | FKISLGAEP |
| | | IAKTLLAEAGYPNGN | AKTLLAEAG |
| | | KGWDISSDGTVYTFN | ISSDGTVYT |
| | | EWTTYLNTKANGNYE | YLNTKANGN |
| | | HIKPLDNVKIRKALT | IKPLDNVKI |
| | | NWTSPENMVTSGPFK | WTSPENMVT |
| | | EGIRKSYLRILNKET | IRKSYLRIL |
| | | AKTLLAEAGYPNGNG | AKTLLAEAG |
| | | WDISSDGTVYTFNLR | ISSDGTVYT |

| | | KEGVSFKISLGAEPS | FKISLGAEP |
|---|---|---|---|
| | | WTTYLNTKANGNYEI | YLNTKANGN |
| | | LDNGTTPTRRATPNF | NGTTPTRRA |
| | | MIDTMFRGIVTGDPN | FRGIVTGDP |
| | | ADPLTFLSIFTQGYT | FLSIFTQGY |
| | | FPIAPIYIYGNSYLF | IAPIYIYGN |
| | | KMIDTMFRGIVTGDP | DTMFRGIVT |
| | | IIEKDFPIAPIYIYG | FPIAPIYIY |
| | | IIIEKDFPIAPIYIY | DFPIAPIYI |
| | | PNFSSYSYAKSLELF | YSYAKSLEL |
| | | DPLTFLSIFTQGYTQ | FLSIFTQGY |
| | | TPNFSSYSYAKSLEL | FSSYSYAKS |
| | | IFGSIPPDLIKNLKL | FGSIPPDLI |
| | | PIAPIYIYGNSYLFR | YIYGNSYLF |
| | | KDFPIAPIYIYGNSY | FPIAPIYIY |
| | | IYIYGNSYLFRNDKW | YGNSYLFRN |
| | | IDTMFRGIVTGDPNT | FRGIVTGDP |
| | | FSSYSYAKSLELFNP | YSYAKSLEL |
| | | NFSSYSYAKSLELFN | YSYAKSLEL |
| | | YAKSLELFNPEIAKT | YAKSLELFN |
| | | MVTSGPFKLKERIPN | MVTSGPFKL |
| | | NDKWTGWNTNILERF | WTGWNTNIL |
| | | DKWTGWNTNILERFD | WTGWNTNIL |
| | | DYYSSAVNAIYFYAF | YYSSAVNAI |
| | | SSYSYAKSLELFNPE | YSYAKSLEL |
| | | TTYLNTKANGNYEIA | YLNTKANGN |
| | | YIYGNSYLFRNDKWT | YGNSYLFRN |
| | | FGSIPPDLIKNLKLR | FGSIPPDLI |
| | | SKYVEMVKSVIKNGQ | VEMVKSVIK |
| | | IRKSYLRILNKETGS | YLRILNKET |
| | | TLEITLESPKPYFID | LESPKPYFI |
| | | GSKYVEMVKSVIKNG | VEMVKSVIK |
| | | KTLEITLESPKPYFI | ITLESPKPY |
| | | GIRKSYLRILNKETG | IRKSYLRIL |
| | | NEELDAIFGSIPPDL | LDAIFGSIP |
| | | IDMLVHQSFIPVPVH | LVHQSFIPV |
| | | DMLVHQSFIPVPVHV | LVHQSFIPV |
| | | YYSSAVNAIYFYAFN | YYSSAVNAI |
| | | ILKLKYNTNEANKKI | YNTNEANKK |
| | | SLGAEPSSLDPQLAE | LGAEPSSLD |
| | | DFPIAPIYIYGNSYL | IAPIYIYGN |
| | | LKLKYNTNEANKKIC | YNTNEANKK |
| HLA-DRB1*0301 | | None | |
| HLA-DRB1*0401 | | RIPNEKYVFEKNNKY | RIPNEKYVF |
| | | IPNEKYVFEKNNKYY | YVFEKNNKY |
| | | PNEKYVFEKNNKYYD | YVFEKNNKY |
| | | NEKYVFEKNNKYYDS | YVFEKNNKY |
| | | EKYVFEKNNKYYDSN | YVFEKNNKY |
| | | ESPKPYFIDMLVHQS | YFIDMLVHQ |
| | | SPKPYFIDMLVHQSF | YFIDMLVHQ |
| | | KPYFIDMLVHQSFIP | YFIDMLVHQ |
| | | PKPYFIDMLVHQSFI | YFIDMLVHQ |
| | | LESPKPYFIDMLVHQ | PYFIDMLVH |
| | | KYVFEKNNKYYDSNE | YVFEKNNKY |
| | | YVFEKNNKYYDSNEV | YVFEKNNKY |
| | | PYFIDMLVHQSFIPV | YFIDMLVHQ |
| | | YFIDMLVHQSFIPVP | YFIDMLVHQ |

| | | | |
|---|---|---|---|
| | | LDPIKRQDILRQAEE | IKRQDILRQ |
| | | DLELDPIKRQDILRQ | DLELDPIKR |
| | | ELDPIKRQDILRQAE | IKRQDILRQ |
| | | LELDPIKRQDILRQA | IKRQDILRQ |
| | | DPIKRQDILRQAEEI | IKRQDILRQ |
| | | EEITFYTTNDSSTAY | YTTNDSSTA |
| | | LEEITFYTTNDSSTA | ITFYTTNDS |
| | | EITFYTTNDSSTAYK | YTTNDSSTA |
| | | ITFYTTNDSSTAYKM | YTTNDSSTA |
| | | KLRSDYYSSAVNAIY | YYSSAVNAI |
| | | LRSDYYSSAVNAIYF | YYSSAVNAI |
| | HLA-DRB1*0404 | LTFLSIFTQGYTQFS | FLSIFTQGY |
| | | ADPLTFLSIFTQGYT | FLSIFTQGY |
| | | RKSYLRILNKETGSK | YLRILNKET |
| | | KSYLRILNKETGSKY | ILNKETGSK |
| | | YADPLTFLSIFTQGY | PLTFLSIFT |
| | | VNAIYFYAFNTHIKP | YFYAFNTHI |
| | | DPLTFLSIFTQGYTQ | FLSIFTQGY |
| | | AIYFYAFNTHIKPLD | YFYAFNTHI |
| | | AVNAIYFYAFNTHIK | YFYAFNTHI |
| | | NAIYFYAFNTHIKPL | YFYAFNTHI |
| | | PLTFLSIFTQGYTQF | FLSIFTQGY |
| | | SAVNAIYFYAFNTHI | IYFYAFNTH |
| | | SYLRILNKETGSKYV | ILNKETGSK |
| | HLA-DRB1*0405 | VTEKYGQNWTSPENM | GQNWTSPEN |
| | | HVTEKYGQNWTSPEN | YGQNWTSPE |
| | | TEKYGQNWTSPENMV | GQNWTSPEN |
| | | EKYGQNWTSPENMVT | GQNWTSPEN |
| | | KYGQNWTSPENMVTS | GQNWTSPEN |
| | | RNDKWTGWNTNILER | WTGWNTNIL |
| | | LFRNDKWTGWNTNIL | DKWTGWNTN |
| | | FRNDKWTGWNTNILE | WTGWNTNIL |
| | | NDKWTGWNTNILERF | WTGWNTNIL |
| | | DKWTGWNTNILERFD | WTGWNTNIL |
| | | YGQNWTSPENMVTSG | GQNWTSPEN |
| | | VSFKISLGAEPSSLD | VSFKISLGA |
| | | SFKISLGAEPSSLDP | LGAEPSSLD |
| | | FKISLGAEPSSLDPQ | LGAEPSSLD |
| | | ISLGAEPSSLDPQLA | LGAEPSSLD |
| | | KISLGAEPSSLDPQL | LGAEPSSLD |
| | | FNLREKITWSDGVAI | LREKITWSD |
| | | ELENEEWTTYLNTKA | NEEWTTYLN |
| | | TVYTFNLREKITWSD | TVYTFNLRE |
| | | SLGAEPSSLDPQLAE | LGAEPSSLD |
| | | LGAEPSSLDPQLAED | LGAEPSSLD |
| | | YTFNLREKITWSDGV | LREKITWSD |
| | | LENEEWTTYLNTKAN | NEEWTTYLN |
| | | TFNLREKITWSDGVA | LREKITWSD |
| | | IYFYAFNTHIKPLDN | FNTHIKPLD |
| | | VYTFNLREKITWSDG | LREKITWSD |
| | | KWTGWNTNILERFDL | WTGWNTNIL |
| | | AIYFYAFNTHIKPLD | FYAFNTHIK |
| | | EELDAIFGSIPPDLI | DAIFGSIPP |
| | | ELDAIFGSIPPDLIK | FGSIPPDLI |
| | | GQNWTSPENMVTSGP | GQNWTSPEN |
| | | EFIQNQWKKNLNIDV | QNQWKKNLN |

| | | | |
|---|---|---|---|
| | | ELEEITFYTTNDSST | ITFYTTNDS |
| | | VELENEEWTTYLNTK | NEEWTTYLN |
| | | LEEITFYTTNDSSTA | ITFYTTNDS |
| | | IDVELENEEWTTYLN | ENEEWTTYL |
| | | DVELENEEWTTYLNT | NEEWTTYLN |
| | | LVHQSFIPVPVHVTE | HQSFIPVPV |
| | | MLVHQSFIPVPVHVT | HQSFIPVPV |
| | | EVELEEITFYTTNDS | EITFYTTND |
| | | VELEEITFYTTNDSS | ITFYTTNDS |
| | | FIQNQWKKNLNIDVE | QNQWKKNLN |
| | | LDAIFGSIPPDLIKN | FGSIPPDLI |
| | | WTGWNTNILERFDLS | WTGWNTNIL |
| | | DAIFGSIPPDLIKNL | FGSIPPDLI |
| | | YFYAFNTHIKPLDNV | FNTHIKPLD |
| | | FYAFNTHIKPLDNVK | FNTHIKPLD |
| | | EEITFYTTNDSSTAY | ITFYTTNDS |
| | | ENEEWTTYLNTKANG | NEEWTTYLN |
| | | NEEWTTYLNTKANGN | NEEWTTYLN |
| | HLA-DRB1*0701 | LFRNDKWTGWNTNIL | NDKWTGWNT |
| | | FRNDKWTGWNTNILE | WTGWNTNIL |
| | | RSDYYSSAVNAIYFY | YYSSAVNAI |
| | | SDYYSSAVNAIYFYA | YYSSAVNAI |
| | | KLRSDYYSSAVNAIY | YYSSAVNAI |
| | | LRSDYYSSAVNAIYF | YYSSAVNAI |
| | | NDKWTGWNTNILERF | WTGWNTNIL |
| | | RNDKWTGWNTNILER | WTGWNTNIL |
| | | PLDNVKIRKALTLAI | VKIRKALTL |
| | | DKWTGWNTNILERFD | WTGWNTNIL |
| | | LDNVKIRKALTLAID | IRKALTLAI |
| | | DNVKIRKALTLAIDR | IRKALTLAI |
| | | MLVHQSFIPVPVHVT | SFIPVPVHV |
| | | LVHQSFIPVPVHVTE | FIPVPVHVT |
| | | LKLRSDYYSSAVNAI | RSDYYSSAV |
| | | VHQSFIPVPVHVTEK | FIPVPVHVT |
| | | NVKIRKALTLAIDRE | IRKALTLAI |
| | | HQSFIPVPVHVTEKY | FIPVPVHVT |
| | | VKIRKALTLAIDRET | IRKALTLAI |
| | | DYYSSAVNAIYFYAF | YYSSAVNAI |
| | | QSFIPVPVHVTEKYG | FIPVPVHVT |
| | | MKLQRSLFLIIFFLT | MKLQRSLFL |
| | HLA-DRB1*0802 | PLDNVKIRKALTLAI | VKIRKALTL |
| | | LDNVKIRKALTLAID | IRKALTLAI |
| | | DNVKIRKALTLAIDR | IRKALTLAI |
| | | NVKIRKALTLAIDRE | IRKALTLAI |
| | HLA-DRB1*0901 | None | |
| | HLA-DRB1*1101 | SDGTVYTFNLREKIT | YTFNLREKI |
| | | GTVYTFNLREKITWS | YTFNLREKI |
| | | DGTVYTFNLREKITW | YTFNLREKI |
| | | SSDGTVYTFNLREKI | GTVYTFNLR |
| | | TVYTFNLREKITWSD | YTFNLREKI |
| | | SGPFKLKERIPNEKY | FKLKERIPN |
| | | MVTSGPFKLKERIPN | VTSGPFKLK |
| | | VTSGPFKLKERIPNE | FKLKERIPN |
| | | GPFKLKERIPNEKYV | FKLKERIPN |
| | | TSGPFKLKERIPNEK | FKLKERIPN |
| | HLA-DRB1*1302 | SKYVEMVKSVIKNGQ | VEMVKSVIK |

| | | | |
|---|---|---|---|
| | | KYVEMVKSVIKNGQK | VKSVIKNGQ |
| | | EMVKSVIKNGQKYFD | VKSVIKNGQ |
| | | YVEMVKSVIKNGQKY | VKSVIKNGQ |
| | | ETLTYKVLDNGTTPT | YKVLDNGTT |
| | | TLTYKVLDNGTTPTR | VLDNGTTPT |
| | | LTYKVLDNGTTPTRR | VLDNGTTPT |
| | | VEMVKSVIKNGQKYF | VKSVIKNGQ |
| | | PDLIKNLKLRSDYYS | LIKNLKLRS |
| | | SIPPDLIKNLKLRSD | LIKNLKLRS |
| | | IPPDLIKNLKLRSDY | LIKNLKLRS |
| | | PPDLIKNLKLRSDYY | LIKNLKLRS |
| | | TYKVLDNGTTPTRRA | VLDNGTTPT |
| | | YKVLDNGTTPTRRAT | VLDNGTTPT |
| | | NDKWTGWNTNILERF | WTGWNTNIL |
| | | DKWTGWNTNILERFD | WNTNILERF |
| | | KSYLRILNKETGSKY | LRILNKETG |
| | | DLIKNLKLRSDYYSS | IKNLKLRSD |
| | | SYLRILNKETGSKYV | LRILNKETG |
| | | MVKSVIKNGQKYFDG | VKSVIKNGQ |
| | | VKSVIKNGQKYFDGQ | VKSVIKNGQ |
| | | GSKYVEMVKSVIKNG | VEMVKSVIK |
| | | KWTGWNTNILERFDL | WNTNILERF |
| | | WTGWNTNILERFDLS | WNTNILERF |
| | | GSIPPDLIKNLKLRS | PPDLIKNLK |
| | | LIKNLKLRSDYYSSA | LIKNLKLRS |
| | | TGSKYVEMVKSVIKN | VEMVKSVIK |
| | | ETGSKYVEMVKSVIK | YVEMVKSVI |
| | | MKLQRSLFLIIFFLT | LQRSLFLII |
| | | EFIQNQWKKNLNIDV | IQNQWKKNL |
| | | LQRSLFLIIFFLTFL | LFLIIFFLT |
| | | YLRILNKETGSKYVE | LRILNKETG |
| | | RKSYLRILNKETGSK | LRILNKETG |
| | | IRKSYLRILNKETGS | LRILNKETG |
| | | LRILNKETGSKYVEM | LRILNKETG |
| | | GIRKSYLRILNKETG | YLRILNKET |
| | | WTSPENMVTSGPFKL | ENMVTSGPF |
| | | TSPENMVTSGPFKLK | MVTSGPFKL |
| | | IKPLDNVKIRKALTL | LDNVKIRKA |
| | | QRSLFLIIFFLTFLC | LFLIIFFLT |
| | | FIQNQWKKNLNIDVE | IQNQWKKNL |
| | | RSLFLIIFFLTFLCC | LFLIIFFLT |
| | | KLQRSLFLIIFFLTF | LFLIIFFLT |
| | | IQNQWKKNLNIDVEL | IQNQWKKNL |
| | | IAPIYIYGNSYLFRN | IYIYGNSYL |
| | | APIYIYGNSYLFRND | YGNSYLFRN |
| | | PLDNVKIRKALTLAI | VKIRKALTL |
| | | SPENMVTSGPFKLKE | MVTSGPFKL |
| | | LDNVKIRKALTLAID | VKIRKALTL |
| | | PYFIDMLVHQSFIPV | FIDMLVHQS |
| | | TGWNTNILERFDLSQ | WNTNILERF |
| | | YAFNTHIKPLDNVKI | FNTHIKPLD |
| | | KVLDNGTTPTRRATP | VLDNGTTPT |
| | | KPLDNVKIRKALTLA | VKIRKALTL |
| | | IKNLKLRSDYYSSAV | LKLRSDYYS |
| | HLA-DRB1*1501 | RKSYLRILNKETGSK | LRILNKETG |
| | | KSYLRILNKETGSKY | LRILNKETG |
| | | SYLRILNKETGSKYV | LRILNKETG |

| | | | |
|---|---|---|---|
| | | GIRKSYLRILNKETG | YLRILNKET |
| | | IRKSYLRILNKETGS | LRILNKETG |
| | | YLRILNKETGSKYVE | LRILNKETG |
| | | LRILNKETGSKYVEM | LRILNKETG |
| | | PPDLIKNLKLRSDYY | IKNLKLRSD |
| | | PDLIKNLKLRSDYYS | IKNLKLRSD |
| | | DLIKNLKLRSDYYSS | IKNLKLRSD |
| | | SIPPDLIKNLKLRSD | DLIKNLKLR |
| | | IPPDLIKNLKLRSDY | IKNLKLRSD |
| | | LIKNLKLRSDYYSSA | IKNLKLRSD |
| | | IKNLKLRSDYYSSAV | IKNLKLRSD |
| | | GTVYTFNLREKITWS | VYTFNLREK |
| | | IAPIYIYGNSYLFRN | IYGNSYLFR |
| | | YGNSYLFRNDKWTGW | LFRNDKWTG |
| | | PIAPIYIYGNSYLFR | IYIYGNSYL |
| | HLA-DRB3*0101 | None | |
| | HLA-DRB4*0101 | FKLKERIPNEKYVFE | LKERIPNEK |
| | | PFKLKERIPNEKYVF | LKERIPNEK |
| | | GPFKLKERIPNEKYV | LKERIPNEK |
| | | SGPFKLKERIPNEKY | LKERIPNEK |
| | | TSGPFKLKERIPNEK | TSGPFKLKE |
| | | VNAIYFYAFNTHIKP | IYFYAFNTH |
| | | SAVNAIYFYAFNTHI | IYFYAFNTH |
| | | SSAVNAIYFYAFNTH | VNAIYFYAF |
| | | NAIYFYAFNTHIKPL | IYFYAFNTH |
| | | AVNAIYFYAFNTHIK | IYFYAFNTH |
| | | YFIDMLVHQSFIPVP | IDMLVHQSF |
| | | PYFIDMLVHQSFIPV | IDMLVHQSF |
| | | AIYFYAFNTHIKPLD | IYFYAFNTH |
| | | IYFYAFNTHIKPLDN | IYFYAFNTH |
| | | FIDMLVHQSFIPVPV | LVHQSFIPV |
| | | IDMLVHQSFIPVPVH | LVHQSFIPV |
| | | KLKERIPNEKYVFEK | LKERIPNEK |
| | | LKERIPNEKYVFEKN | LKERIPNEK |
| | | FLIIFFLTFLCCNNK | IFFLTFLCC |
| | | RSLFLIIFFLTFLCC | IIFFLTFLC |
| | | SLFLIIFFLTFLCCN | IFFLTFLCC |
| | HLA-DRB5*0101 | LTYKVLDNGTTPTRR | LDNGTTPTR |
| | | YKVLDNGTTPTRRAT | LDNGTTPTR |
| | | TYKVLDNGTTPTRRA | LDNGTTPTR |
| | | AKSLELFNPEIAKTL | LFNPEIAKT |
| | | KSLELFNPEIAKTLL | FNPEIAKTL |
| | | SLELFNPEIAKTLLA | FNPEIAKTL |
| | | LELFNPEIAKTLLAE | FNPEIAKTL |
| | | ELFNPEIAKTLLAEA | FNPEIAKTL |
| | | TLTYKVLDNGTTPTR | YKVLDNGTT |
| | | KVLDNGTTPTRRATP | LDNGTTPTR |
| | | KSYLRILNKETGSKY | ILNKETGSK |
| | | | |
| AAC44381.1\| outer membrane porin protein Oms28 precursor  Seq25 | HLA-DRB1*0101 | | |
| | | VKKFVGSMSLMSDVA | FVGSMSLMS |
| | | GVVKKFVGSMSLMSD | FVGSMSLMS |
| | | VVKKFVGSMSLMSDV | FVGSMSLMS |
| | | KKFVGSMSLMSDVAK | FVGSMSLMS |
| | | AGVVKKFVGSMSLMS | AGVVKKFVG |
| | | FVGSMSLMSDVAKGT | FVGSMSLMS |
| | | KFVGSMSLMSDVAKG | FVGSMSLMS |

| | | | |
|---|---|---|---|
| | | KKDVRKAISNVVKVA | VRKAISNVV |
| | | AKCSGMVAEGANKVV | MVAEGANKV |
| | | VLAKKDVRKAISNVV | LAKKDVRKA |
| | | LAKKDVRKAISNVVK | VRKAISNVV |
| | | AKKDVRKAISNVVKV | VRKAISNVV |
| | | KDVRKAISNVVKVAQ | VRKAISNVV |
| | | KCSGMVAEGANKVVE | MVAEGANKV |
| | | CSGMVAEGANKVVEM | MVAEGANKV |
| | | VAKCSGMVAEGANKV | CSGMVAEGA |
| | | AKGTVVASQEATIVA | VASQEATIV |
| | | VAKGTVVASQEATIV | GTVVASQEA |
| | | KGTVVASQEATIVAK | VASQEATIV |
| | | GTVVASQEATIVAKC | VASQEATIV |
| | | SGMVAEGANKVVEMS | MVAEGANKV |
| | | ARDLTKVMAISLYMR | LTKVMAISL |
| | | TVVASQEATIVAKCS | VASQEATIV |
| | | ATIVAKCSGMVAEGA | IVAKCSGMV |
| | | SQEATIVAKCSGMVA | IVAKCSGMV |
| | | EATIVAKCSGMVAEG | IVAKCSGMV |
| | | MSLMSDVAKGTVVAS | LMSDVAKGT |
| | | QEATIVAKCSGMVAE | IVAKCSGMV |
| | | TQKAVSVAGEATFLI | SVAGEATFL |
| | | QKAVSVAGEATFLIE | VAGEATFLI |
| | | AVSVAGEATFLIEKQ | VAGEATFLI |
| | | KAVSVAGEATFLIEK | VAGEATFLI |
| | | LMSDVAKGTVVASQE | VAKGTVVAS |
| | | SLMSDVAKGTVVASQ | VAKGTVVAS |
| | | GARDLTKVMAISLYM | LTKVMAISL |
| | | MSDVAKGTVVASQEA | VAKGTVVAS |
| | | ASQEATIVAKCSGMV | EATIVAKCS |
| | | TIVAKCSGMVAEGAN | IVAKCSGMV |
| | | VSVAGEATFLIEKQI | VAGEATFLI |
| | | DVRKAISNVVKVAQG | VRKAISNVV |
| | | GMVAEGANKVVEMSK | MVAEGANKV |
| | | VRKAISNVVKVAQGA | VRKAISNVV |
| | | SDVAKGTVVASQEAT | VAKGTVVAS |
| | | SMSLMSDVAKGTVVA | LMSDVAKGT |
| | | VVASQEATIVAKCSG | VASQEATIV |
| | | IVAKCSGMVAEGANK | IVAKCSGMV |
| | | VASQEATIVAKCSGM | VASQEATIV |
| | | MVAEGANKVVEMSKK | MVAEGANKV |
| | | SNNANILKPQSNVLE | ILKPQSNVL |
| | | NNANILKPQSNVLEH | ILKPQSNVL |
| | | LIINGLLFGFVSLNV | INGLLFGFV |
| | | NANILKPQSNVLEHS | ILKPQSNVL |
| | | ANILKPQSNVLEHSD | ILKPQSNVL |
| | | VGSMSLMSDVAKGTV | LMSDVAKGT |
| | | QGARDLTKVMAISLY | LTKVMAISL |
| | | AQGARDLTKVMAISL | ARDLTKVMA |
| | | MTKIFSNLIINGLLF | FSNLIINGL |
| | | GSMSLMSDVAKGTVV | LMSDVAKGT |
| | | INGLLFGFVSLNVFA | LFGFVSLNV |
| | | IINGLLFGFVSLNVF | LFGFVSLNV |
| | | DVAKGTVVASQEATI | VAKGTVVAS |
| | | KIFSNLIINGLLFGF | FSNLIINGL |
| | | TKIFSNLIINGLLFG | FSNLIINGL |
| | | VKETLMASERALDET | ETLMASERA |

| | | | |
|---|---|---|---|
| | | EVKETLMASERALDE | ETLMASERA |
| | | FSNLIINGLLFGFVS | INGLLFGFV |
| | | IFSNLIINGLLFGFV | LIINGLLFG |
| | | ANKVVEMSKKAVQET | VVEMSKKAV |
| | | NILKPQSNVLEHSDQ | LKPQSNVLE |
| | | NKVVEMSKKAVQETQ | MSKKAVQET |
| | | NGLLFGFVSLNVFAD | LFGFVSLNV |
| | | GLLFGFVSLNVFADS | LFGFVSLNV |
| | | SVAGEATFLIEKQIM | VAGEATFLI |
| | | SNLIINGLLFGFVSL | INGLLFGFV |
| | | DSNNANILKPQSNVL | ANILKPQSN |
| | | VVEMSKKAVQETQKA | MSKKAVQET |
| | | NLIINGLLFGFVSLN | INGLLFGFV |
| | | KVVEMSKKAVQETQK | MSKKAVQET |
| | | VAGEATFLIEKQIML | VAGEATFLI |
| | | ISNVVKVAQGARDLT | VKVAQGARD |
| | | QETQKAVSVAGEATF | TQKAVSVAG |
| | | DEVKETLMASERALD | ETLMASERA |
| | | SNVVKVAQGARDLTK | VKVAQGARD |
| | | VQETQKAVSVAGEAT | TQKAVSVAG |
| | | NVVKVAQGARDLTKV | VKVAQGARD |
| | | VDEVKETLMASERAL | ETLMASERA |
| | | QKVLNMVNGLNPSNK | MVNGLNPSN |
| | | LIEKQIMLNKSPNNK | IMLNKSPNN |
| | | KAVQETQKAVSVAGE | TQKAVSVAG |
| | | KKAVQETQKAVSVAG | QETQKAVSV |
| | | KVDEVKETLMASERA | VKETLMASE |
| | | VEMSKKAVQETQKAV | MSKKAVQET |
| | | AVQETQKAVSVAGEA | TQKAVSVAG |
| | | KVLNMVNGLNPSNKD | MVNGLNPSN |
| | | SKLEGVRESSLELVE | VRESSLELV |
| | | SSKLEGVRESSLELV | LEGVRESSL |
| | | KETLMASERALDETV | ETLMASERA |
| | | LEGVRESSLELVESN | VRESSLELV |
| | | VLNMVNGLNPSNKDQ | MVNGLNPSN |
| | | KLEGVRESSLELVES | VRESSLELV |
| | | AISNVVKVAQGARDL | VKVAQGARD |
| | | ETQKAVSVAGEATFL | TQKAVSVAG |
| | | ILKPQSNVLEHSDQK | ILKPQSNVL |
| | | ETLMASERALDETVQ | ETLMASERA |
| | | LNMVNGLNPSNKDQV | MVNGLNPSN |
| | | AQKVLNMVNGLNPSN | VLNMVNGLN |
| HLA-DRB1*0301 | | None | |
| HLA-DRB1*0401 | | AGVVKKFVGSMSLMS | VKKFVGSMS |
| | | GVVKKFVGSMSLMSD | FVGSMSLMS |
| | | VVKKFVGSMSLMSDV | FVGSMSLMS |
| | | KKFVGSMSLMSDVAK | FVGSMSLMS |
| | | VKKFVGSMSLMSDVA | FVGSMSLMS |
| | | KFVGSMSLMSDVAKG | FVGSMSLMS |
| | | FVGSMSLMSDVAKGT | FVGSMSLMS |
| | | LAKKDVRKAISNVVK | VRKAISNVV |
| | | VLAKKDVRKAISNVV | AKKDVRKAI |
| | | KDVRKAISNVVKVAQ | VRKAISNVV |
| | | AKKDVRKAISNVVKV | VRKAISNVV |
| | | KKDVRKAISNVVKVA | VRKAISNVV |
| | | MTKIFSNLIINGLLF | MTKIFSNLI |
| | | INGLLFGFVSLNVFA | LFGFVSLNV |

| | | NGLLFGFVSLNVFAD | LFGFVSLNV |
|---|---|---|---|
| | | GLLFGFVSLNVFADS | LFGFVSLNV |
| | HLA-DRB1*0404 | QKVLNMVNGLNPSNK | VLNMVNGLN |
| | | EAQKVLNMVNGLNPS | VLNMVNGLN |
| | | QEAQKVLNMVNGLNP | VLNMVNGLN |
| | | AQKVLNMVNGLNPSN | VLNMVNGLN |
| | | VQEAQKVLNMVNGLN | QKVLNMVNG |
| | | KVLNMVNGLNPSNKD | VLNMVNGLN |
| | | VLNMVNGLNPSNKDQ | VLNMVNGLN |
| | | LIINGLLFGFVSLNV | LLFGFVSLN |
| | | INGLLFGFVSLNVFA | LFGFVSLNV |
| | | NGLLFGFVSLNVFAD | LFGFVSLNV |
| | | IINGLLFGFVSLNVF | LFGFVSLNV |
| | | IEKQIMLNKSPNNKE | IMLNKSPNN |
| | | LIEKQIMLNKSPNNK | IMLNKSPNN |
| | | EKQIMLNKSPNNKEL | IMLNKSPNN |
| | | KQIMLNKSPNNKELE | IMLNKSPNN |
| | | GLLFGFVSLNVFADS | LFGFVSLNV |
| | HLA-DRB1*0405 | IEKQIMLNKSPNNKE | IMLNKSPNN |
| | | EKQIMLNKSPNNKEL | LNKSPNNKE |
| | | KQIMLNKSPNNKELE | LNKSPNNKE |
| | | QIMLNKSPNNKELEL | LNKSPNNKE |
| | | IMLNKSPNNKELELT | LNKSPNNKE |
| | | LNKSPNNKELELTKE | LNKSPNNKE |
| | | MLNKSPNNKELELTK | LNKSPNNKE |
| | HLA-DRB1*0701 | VLAKKDVRKAISNVV | VLAKKDVRK |
| | | AKKDVRKAISNVVKV | VRKAISNVV |
| | | KKDVRKAISNVVKVA | VRKAISNVV |
| | | LAKKDVRKAISNVVK | VRKAISNVV |
| | | KDVRKAISNVVKVAQ | VRKAISNVV |
| | | DVRKAISNVVKVAQG | VRKAISNVV |
| | | VRKAISNVVKVAQGA | VRKAISNVV |
| | HLA-DRB1*0802 | None | |
| | HLA-DRB1*0901 | KDVRKAISNVVKVAQ | VRKAISNVV |
| | | KKDVRKAISNVVKVA | VRKAISNVV |
| | | AKKDVRKAISNVVKV | VRKAISNVV |
| | | LAKKDVRKAISNVVK | VRKAISNVV |
| | | VLAKKDVRKAISNVV | AKKDVRKAI |
| | | VVKKFVGSMSLMSDV | FVGSMSLMS |
| | | AGVVKKFVGSMSLMS | KKFVGSMSL |
| | | GVVKKFVGSMSLMSD | FVGSMSLMS |
| | HLA-DRB1*1101 | None | |
| | HLA-DRB1*1302 | TKIFSNLIINGLLFG | FSNLIINGL |
| | | KIFSNLIINGLLFGF | FSNLIINGL |
| | | IFSNLIINGLLFGFV | FSNLIINGL |
| | | FSNLIINGLLFGFVS | FSNLIINGL |
| | | MTKIFSNLIINGLLF | FSNLIINGL |
| | | EAQKVLNMVNGLNPS | LNMVNGLNP |
| | | AQKVLNMVNGLNPSN | LNMVNGLNP |
| | | QEAQKVLNMVNGLNP | KVLNMVNGL |
| | | QKVLNMVNGLNPSNK | LNMVNGLNP |
| | | KVLNMVNGLNPSNKD | LNMVNGLNP |
| | | KDVRKAISNVVKVAQ | VRKAISNVV |
| | | VLNMVNGLNPSNKDQ | LNMVNGLNP |
| | | GLLFGFVSLNVFADS | FGFVSLNVF |

| | | | |
|---|---|---|---|
| | | IEKQIMLNKSPNNKE | MLNKSPNNK |
| | | EKQIMLNKSPNNKEL | MLNKSPNNK |
| | | DVRKAISNVVKVAQG | ISNVVKVAQ |
| | | VRKAISNVVKVAQGA | ISNVVKVAQ |
| | | LLFGFVSLNVFADSN | FGFVSLNVF |
| | | LIEKQIMLNKSPNNK | QIMLNKSPN |
| | | NGLLFGFVSLNVFAD | FGFVSLNVF |
| | | SNLIINGLLFGFVSL | LIINGLLFG |
| | | KAISNVVKVAQGARD | ISNVVKVAQ |
| | | VQEAQKVLNMVNGLN | KVLNMVNGL |
| | | IINGLLFGFVSLNVF | IINGLLFGF |
| | | KQIMLNKSPNNKELE | MLNKSPNNK |
| | | QIMLNKSPNNKELEL | MLNKSPNNK |
| | | GVVKKFVGSMSLMSD | VKKFVGSMS |
| | | INGLLFGFVSLNVFA | FGFVSLNVF |
| | | RKAISNVVKVAQGAR | ISNVVKVAQ |
| | | ARDLTKVMAISLYMR | TKVMAISLY |
| | | LFGFVSLNVFADSNN | FGFVSLNVF |
| | | LNMVNGLNPSNKDQV | LNMVNGLNP |
| | | FGFVSLNVFADSNNA | FGFVSLNVF |
| | | AGVVKKFVGSMSLMS | VKKFVGSMS |
| | | ISNVVKVAQGARDLT | ISNVVKVAQ |
| | | DAGVVKKFVGSMSLM | VKKFVGSMS |
| | | NDAGVVKKFVGSMSL | VKKFVGSMS |
| | | SNDAGVVKKFVGSMS | AGVVKKFVG |
| | | VVKKFVGSMSLMSDV | VKKFVGSMS |
| | | VKKFVGSMSLMSDVA | VKKFVGSMS |
| | | AISNVVKVAQGARDL | ISNVVKVAQ |
| | | NLIINGLLFGFVSLN | LIINGLLFG |
| | | GARDLTKVMAISLYM | TKVMAISLY |
| | | KKDVRKAISNVVKVA | VRKAISNVV |
| | | IMLNKSPNNKELELT | MLNKSPNNK |
| | | QGARDLTKVMAISLY | LTKVMAISL |
| | HLA-DRB1*1501 | ARDLTKVMAISLYMR | KVMAISLYM |
| | | INGLLFGFVSLNVFA | LFGFVSLNV |
| | | IINGLLFGFVSLNVF | LFGFVSLNV |
| | | NGLLFGFVSLNVFAD | LFGFVSLNV |
| | | GLLFGFVSLNVFADS | LFGFVSLNV |
| | | LIINGLLFGFVSLNV | INGLLFGFV |
| | | VLAKKDVRKAISNVV | VLAKKDVRK |
| | | LLFGFVSLNVFADSN | LFGFVSLNV |
| | | LFGFVSLNVFADSNN | LFGFVSLNV |
| | | LAKKDVRKAISNVVK | VRKAISNVV |
| | | KKDVRKAISNVVKVA | VRKAISNVV |
| | | AKKDVRKAISNVVKV | VRKAISNVV |
| | | KDVRKAISNVVKVAQ | VRKAISNVV |
| | | QKVLNMVNGLNPSNK | VLNMVNGLN |
| | | AGVVKKFVGSMSLMS | VKKFVGSMS |
| | | GVVKKFVGSMSLMSD | FVGSMSLMS |
| | | LIEKQIMLNKSPNNK | IMLNKSPNN |
| | | KVLNMVNGLNPSNKD | VNGLNPSNK |
| | | IEKQIMLNKSPNNKE | MLNKSPNNK |
| | | EKQIMLNKSPNNKEL | MLNKSPNNK |
| | | KQIMLNKSPNNKELE | MLNKSPNNK |
| | | VLNMVNGLNPSNKDQ | VNGLNPSNK |
| | | VVKKFVGSMSLMSDV | FVGSMSLMS |
| | | QIMLNKSPNNKELEL | MLNKSPNNK |

| | | VKKFVGSMSLMSDVA | FVGSMSLMS |
|---|---|---|---|
| | HLA-DRB3*0101 | None | |
| | HLA-DRB4*0101 | LIEKQIMLNKSPNNK | LIEKQIMLN |
| | | EKQIMLNKSPNNKEL | MLNKSPNNK |
| | | IEKQIMLNKSPNNKE | MLNKSPNNK |
| | | KQIMLNKSPNNKELE | MLNKSPNNK |
| | | QIMLNKSPNNKELEL | MLNKSPNNK |
| | | IMLNKSPNNKELELT | MLNKSPNNK |
| | | MLNKSPNNKELELTK | MLNKSPNNK |
| | HLA-DRB5*0101 | IEKQIMLNKSPNNKE | MLNKSPNNK |
| | | | |
| CAA49829.1\| p93 [Borrelia burgdorferi]<br><br>Seq26 | HLA-DRB1*0101 | IDPITNLGTLQLIDL | ITNLGTLQL |
| | | LEVIDPITNLGTLQL | VIDPITNLG |
| | | EVIDPITNLGTLQLI | ITNLGTLQL |
| | | VIDPITNLGTLQLID | ITNLGTLQL |
| | | DPITNLGTLQLIDLN | ITNLGTLQL |
| | | DYYKGFYIEPALKSL | FYIEPALKS |
| | | YKGFYIEPALKSLTK | FYIEPALKS |
| | | YYKGFYIEPALKSLT | FYIEPALKS |
| | | KGFYIEPALKSLTKE | FYIEPALKS |
| | | LDYYKGFYIEPALKS | YKGFYIEPA |
| | | ITNLGTLQLIDLNTG | ITNLGTLQL |
| | | PITNLGTLQLIDLNT | ITNLGTLQL |
| | | EKLKDFVNMDLEFVN | FVNMDLEFV |
| | | LKDFVNMDLEFVNYK | FVNMDLEFV |
| | | KDFVNMDLEFVNYKG | FVNMDLEFV |
| | | KLKDFVNMDLEFVNY | FVNMDLEFV |
| | | KEKLKDFVNMDLEFV | KEKLKDFVN |
| | | FLIFLNGFPLNARKV | FLNGFPLNA |
| | | LIFLNGFPLNARKVD | FLNGFPLNA |
| | | DELKNLVILDVNTLK | LKNLVILDV |
| | | QDELKNLVILDVNTL | LKNLVILDV |
| | | EEEITKGKSRASLGD | ITKGKSRAS |
| | | LQDELKNLVILDVNT | LKNLVILDV |
| | | KEEEITKGKSRASLG | ITKGKSRAS |
| | | EEITKGKSRASLGDL | ITKGKSRAS |
| | | YLQDELKNLVILDVN | LKNLVILDV |
| | | VKEEEITKGKSRASL | ITKGKSRAS |
| | | EFLARPLTNSNSNSS | FLARPLTNS |
| | | IYLQDELKNLVILDV | IYLQDELKN |
| | | KVKEEEITKGKSRAS | EITKGKSRA |
| | | IGEFLARPLTNSNSN | FLARPLTNS |
| | | GEFLARPLTNSNSNS | FLARPLTNS |
| | | FLARPLTNSNSNSSY | LTNSNSNSS |
| | | FFLIFLNGFPLNARK | FLNGFPLNA |
| | | VGIGEFLARPLTNSN | FLARPLTNS |
| | | GIGEFLARPLTNSNS | FLARPLTNS |
| | | SFFLIFLNGFPLNAR | FLNGFPLNA |
| | | GFYIEPALKSLTKEN | FYIEPALKS |
| | | FYIEPALKSLTKENA | FYIEPALKS |
| | | NFEINKNSSLYVDSK | INKNSSLYV |
| | | SNFEINKNSSLYVDS | INKNSSLYV |
| | | IVGIGEFLARPLTNS | IGEFLARPL |
| | | IFLNGFPLNARKVDK | FLNGFPLNA |
| | | ESNFEINKNSSLYVD | INKNSSLYV |
| | | SESNFEINKNSSLYV | EINKNSSLY |

| | | | |
|---|---|---|---|
| | | FEINKNSSLYVDSKM | INKNSSLYV |
| | | LARPLTNSNSNSSYY | LTNSNSNSS |
| | | FSFFLIFLNGFPLNA | FFLIFLNGF |
| | | ARPLTNSNSNSSYYG | LTNSNSNSS |
| | | DFVNMDLEFVNYKGP | FVNMDLEFV |
| | | FVNMDLEFVNYKGPY | FVNMDLEFV |
| | | RPLTNSNSNSSYYGK | LTNSNSNSS |
| | | FLNGFPLNARKVDKE | FLNGFPLNA |
| | | LKNLVILDVNTLKKV | LKNLVILDV |
| | | ELKNLVILDVNTLKK | LKNLVILDV |
| | | LVVIKMDSGKAKLQI | VIKMDSGKA |
| | | DLVVIKMDSGKAKLQ | VIKMDSGKA |
| | | DSILNLRRILTGYII | ILNLRRILT |
| | | LDSILNLRRILTGYI | ILNLRRILT |
| | | EDLDKDLTTMSIDSS | DKDLTTMSI |
| | | EITKGKSRASLGDLN | ITKGKSRAS |
| | | DLDKDLTTMSIDSSS | LTTMSIDSS |
| | | ITKGKSRASLGDLNN | ITKGKSRAS |
| | | KDLVVIKMDSGKAKL | VIKMDSGKA |
| | | IDSLVTDKVIAALLS | LVTDKVIAA |
| | | EKDLVVIKMDSGKAK | VIKMDSGKA |
| | | DSLVTDKVIAALLSE | DKVIAALLS |
| | | ILNLRRILTGYIIKS | LRRILTGYI |
| | | DKDLTTMSIDSSSPV | LTTMSIDSS |
| | | MKKMLLIFSFFLIFL | MKKMLLIFS |
| | | LVTDKVIAALLSENE | DKVIAALLS |
| | | LDKDLTTMSIDSSSP | LTTMSIDSS |
| | | DLTTMSIDSSSPVFL | LTTMSIDSS |
| | | LTTMSIDSSSPVFLE | IDSSSPVFL |
| | | VTDKVIAALLSENEA | DKVIAALLS |
| | | LNLRRILTGYIIKSF | LRRILTGYI |
| | | SLVTDKVIAALLSEN | DKVIAALLS |
| | | KDLTTMSIDSSSPVF | LTTMSIDSS |
| | | TNLGTLQLIDLNTGV | LGTLQLIDL |
| | | SILNLRRILTGYIIK | LRRILTGYI |
| | | VVIKMDSGKAKLQIL | IKMDSGKAK |
| | | SELDSILNLRRILTG | ILNLRRILT |
| | | ENKILPFTSFSVRKN | ILPFTSFSV |
| | | LGTLQLIDLNTGVRL | LQLIDLNTG |
| | | EINKNSSLYVDSKMI | INKNSSLYV |
| | | NKILPFTSFSVRKNF | FTSFSVRKN |
| | | ELDSILNLRRILTGY | ILNLRRILT |
| | | SLYVDSKMILAAVRD | YVDSKMILA |
| | | INKNSSLYVDSKMIL | INKNSSLYV |
| | | LYVDSKMILAAVRDK | SKMILAAVR |
| | | NLRRILTGYIIKSFD | ILTGYIIKS |
| | | IDIDSLVTDKVIAAL | LVTDKVIAA |
| | | VIKMDSGKAKLQILN | VIKMDSGKA |
| | | KSELDSILNLRRILT | LDSILNLRR |
| | | TDKVIAALLSENEAG | IAALLSENE |
| | | DKVIAALLSENEAGV | IAALLSENE |
| | | SSLYVDSKMILAAVR | YVDSKMILA |
| | | PVFLEVIDPITNLGT | VIDPITNLG |
| | | PLTNSNSNSSYYGKY | LTNSNSNSS |
| | | VFLEVIDPITNLGTL | VIDPITNLG |
| | | LQILNKLENLKVVSE | LNKLENLKV |
| | | LTNSNSNSSYYGKYF | LTNSNSNSS |

| | | | |
|---|---|---|---|
| | | LRRILTGYIIKSFDY | ILTGYIIKS |
| | | EELLKSKDGKVSKDY | LKSKDGKVS |
| | | RDEELLKSKDGKVSK | LKSKDGKVS |
| | | QILNKLENLKVVSES | LNKLENLKV |
| | | TTMSIDSSSPVFLEV | IDSSSPVFL |
| | | KRDEELLKSKDGKVS | EELLKSKDG |
| | | FLEVIDPITNLGTLQ | VIDPITNLG |
| | | ELLKSKDGKVSKDYE | LKSKDGKVS |
| | | KNLVILDVNTLKKVK | LVILDVNTL |
| | | YVDSKMILAAVRDKD | MILAAVRDK |
| | | NEDLDKDLTTMSIDS | LDKDLTTMS |
| | | LNGFPLNARKVDKEK | GFPLNARKV |
| | | DEELLKSKDGKVSKD | LKSKDGKVS |
| | | IDLNTGVRLKESTQQ | IDLNTGVRL |
| | | LNEDLDKDLTTMSID | DKDLTTMSI |
| | | SLNEDLDKDLTTMSI | LDKDLTTMS |
| | | TMSIDSSSPVFLEVI | IDSSSPVFL |
| | | LEFVNYKGPYDSTNT | VNYKGPYDS |
| | | GTLQLIDLNTGVRLK | LQLIDLNTG |
| | | NLGTLQLIDLNTGVR | LQLIDLNTG |
| | | VDSKMILAAVRDKDD | MILAAVRDK |
| | | EFVNYKGPYDSTNTY | VNYKGPYDS |
| | | DLNTGVRLKESTQQG | VRLKESTQQ |
| | | KSLTKENAGLSRVYS | LTKENAGLS |
| | | MSIDSSSPVFLEVID | IDSSSPVFL |
| | | DIDSLVTDKVIAALL | LVTDKVIAA |
| | | DPKVKDQTTSLNEDL | VKDQTTSLN |
| | | LKSLTKENAGLSRVY | LTKENAGLS |
| | | NTGVRLKESTQQGIQ | VRLKESTQQ |
| | | DIDIDSLVTDKVIAA | IDSLVTDKV |
| | | LNTGVRLKESTQQGI | VRLKESTQQ |
| | | ILPFTSFSVRKNFIY | FTSFSVRKN |
| | | LSENKILPFTSFSVR | ILPFTSFSV |
| | | SENKILPFTSFSVRK | ILPFTSFSV |
| | | IDPKVKDQTTSLNED | VKDQTTSLN |
| | | QKIDPKVKDQTTSLN | KVKDQTTSL |
| | | KIDPKVKDQTTSLNE | VKDQTTSLN |
| | | KILPFTSFSVRKNFI | FTSFSVRKN |
| | | ALKSLTKENAGLSRV | LTKENAGLS |
| | | REKDLVVIKMDSGKA | LVVIKMDSG |
| | | PKVKDQTTSLNEDLD | VKDQTTSLN |
| | | RRILTGYIIKSFDYD | ILTGYIIKS |
| | | SFDYDRSSAELIAKV | YDRSSAELI |
| | | TGVRLKESTQQGIQR | VRLKESTQQ |
| | | IKSFDYDRSSAELIA | YDRSSAELI |
| | | AGLSRVYSQWAGKTQ | LSRVYSQWA |
| | | KSFDYDRSSAELIAK | YDRSSAELI |
| | | FDYDRSSAELIAKVI | YDRSSAELI |
| | | SRVYSQWAGKTQIFI | VYSQWAGKT |
| | | NSSLYVDSKMILAAV | YVDSKMILA |
| | | AKLQILNKLENLKVV | LNKLENLKV |
| | | IKMDSGKAKLQILNK | MDSGKAKLQ |
| | | ILSENKILPFTSFSV | NKILPFTSF |
| | | EQIVGIGEFLARPLT | IGEFLARPL |
| | | IIKSFDYDRSSAELI | DYDRSSAEL |
| | | YEQIVGIGEFLARPL | IVGIGEFLA |
| | | LSRVYSQWAGKTQIF | VYSQWAGKT |

| | | | |
|---|---|---|---|
| | | KLQILNKLENLKVVS | LNKLENLKV |
| | | NAGLSRVYSQWAGKT | LSRVYSQWA |
| | | KAKLQILNKLENLKV | LQILNKLEN |
| | | LQLIDLNTGVRLKES | IDLNTGVRL |
| | | DLEFVNYKGPYDSTN | VNYKGPYDS |
| | | GLSRVYSQWAGKTQI | VYSQWAGKT |
| | | ILNKLENLKVVSESN | LENLKVVSE |
| | | PALKSLTKENAGLSR | LTKENAGLS |
| | | ELIAKVITIYNAVYR | IAKVITIYN |
| | HLA-DRB1*0301 | None | |
| | HLA-DRB1*0401 | EFLARPLTNSNSNSS | LARPLTNSN |
| | | FLARPLTNSNSNSSY | LTNSNSNSS |
| | | LARPLTNSNSNSSYY | LTNSNSNSS |
| | | ARPLTNSNSNSSYYG | LTNSNSNSS |
| | | RPLTNSNSNSSYYGK | LTNSNSNSS |
| | | LDYYKGFYIEPALKS | YKGFYIEPA |
| | | DYYKGFYIEPALKSL | FYIEPALKS |
| | | VNFARDITDIQGETH | FARDITDIQ |
| | | NEAGVNFARDITDIQ | NEAGVNFAR |
| | | GVNFARDITDIQGET | FARDITDIQ |
| | | EAGVNFARDITDIQG | FARDITDIQ |
| | | AGVNFARDITDIQGE | FARDITDIQ |
| | | YKGFYIEPALKSLTK | FYIEPALKS |
| | | YYKGFYIEPALKSLT | FYIEPALKS |
| | | KGFYIEPALKSLTKE | FYIEPALKS |
| | | LTNSNSNSSYYGKYF | LTNSNSNSS |
| | | PLTNSNSNSSYYGKY | LTNSNSNSS |
| | | NFARDITDIQGETHK | FARDITDIQ |
| | | FARDITDIQGETHKA | FARDITDIQ |
| | | FYIEPALKSLTKENA | FYIEPALKS |
| | | GFYIEPALKSLTKEN | FYIEPALKS |
| | | FSFFLIFLNGFPLNA | FFLIFLNGF |
| | | FFLIFLNGFPLNARK | FLNGFPLNA |
| | | SFFLIFLNGFPLNAR | FLNGFPLNA |
| | HLA-DRB1*0404 | SFFLIFLNGFPLNAR | FLIFLNGFP |
| | | FSFFLIFLNGFPLNA | FLIFLNGFP |
| | | LEVIDPITNLGTLQL | VIDPITNLG |
| | | SPVFLEVIDPITNLG | VFLEVIDPI |
| | | PVFLEVIDPITNLGT | VIDPITNLG |
| | | VFLEVIDPITNLGTL | VIDPITNLG |
| | | FLEVIDPITNLGTLQ | VIDPITNLG |
| | | FFLIFLNGFPLNARK | FLNGFPLNA |
| | | FLIFLNGFPLNARKV | FLNGFPLNA |
| | | IFSFFLIFLNGFPLN | FLIFLNGFP |
| | | LLIFSFFLIFLNGFP | SFFLIFLNG |
| | | LIFSFFLIFLNGFPL | FLIFLNGFP |
| | | EVIDPITNLGTLQLI | VIDPITNLG |
| | | VIDPITNLGTLQLID | VIDPITNLG |
| | | LIFLNGFPLNARKVD | FLNGFPLNA |
| | | EFLARPLTNSNSNSS | LARPLTNSN |
| | | ELKNLVILDVNTLKK | VILDVNTLK |
| | | LKNLVILDVNTLKKV | ILDVNTLKK |
| | | EKDLVVIKMDSGKAK | VIKMDSGKA |
| | | REKDLVVIKMDSGKA | LVVIKMDSG |
| | | FLARPLTNSNSNSSY | LTNSNSNSS |
| | | KNLVILDVNTLKKVK | ILDVNTLKK |

| | | | |
|---|---|---|---|
| | | KDLVVIKMDSGKAKL | VIKMDSGKA |
| | | LARPLTNSNSNSSYY | LTNSNSNSS |
| | | GTLQLIDLNTGVRLK | LIDLNTGVR |
| | | NLGTLQLIDLNTGVR | LQLIDLNTG |
| | | IFLNGFPLNARKVDK | FLNGFPLNA |
| | | LGTLQLIDLNTGVRL | LIDLNTGVR |
| | | IGEFLARPLTNSNSN | LARPLTNSN |
| | | GEFLARPLTNSNSNS | LARPLTNSN |
| | | GIGEFLARPLTNSNS | LARPLTNSN |
| | | DLVVIKMDSGKAKLQ | VIKMDSGKA |
| | | LVVIKMDSGKAKLQI | VIKMDSGKA |
| | | TLQLIDLNTGVRLKE | LIDLNTGVR |
| | | VGIGEFLARPLTNSN | FLARPLTNS |
| | | LQLIDLNTGVRLKES | LIDLNTGVR |
| | HLA-DRB1*0405 | ELIAKVITIYNAVYR | IAKVITIYN |
| | | AELIAKVITIYNAVY | IAKVITIYN |
| | | LDYYKGFYIEPALKS | YKGFYIEPA |
| | | DYYKGFYIEPALKSL | FYIEPALKS |
| | | YYKGFYIEPALKSLT | FYIEPALKS |
| | | YKGFYIEPALKSLTK | FYIEPALKS |
| | | IDPKVKDQTTSLNED | VKDQTTSLN |
| | | PKVKDQTTSLNEDLD | VKDQTTSLN |
| | | INEINKEKNLPKPGD | INKEKNLPK |
| | | DPKVKDQTTSLNEDL | VKDQTTSLN |
| | | NEINKEKNLPKPGDV | INKEKNLPK |
| | | KGFYIEPALKSLTKE | FYIEPALKS |
| | | SFFLIFLNGFPLNAR | IFLNGFPLN |
| | | FFLIFLNGFPLNARK | LNGFPLNAR |
| | | IAKVITIYNAVYRGD | IAKVITIYN |
| | | FLIFLNGFPLNARKV | LNGFPLNAR |
| | | RSSAELIAKVITIYN | LIAKVITIY |
| | | SSAELIAKVITIYNA | IAKVITIYN |
| | | SAELIAKVITIYNAV | IAKVITIYN |
| | | LIAKVITIYNAVYRG | IAKVITIYN |
| | | LIFLNGFPLNARKVD | LNGFPLNAR |
| | | IFLNGFPLNARKVDK | LNGFPLNAR |
| | | QKIDPKVKDQTTSLN | QKIDPKVKD |
| | | KIDPKVKDQTTSLNE | VKDQTTSLN |
| | | EINKEKNLPKPGDVS | EKNLPKPGD |
| | | INKEKNLPKPGDVSS | EKNLPKPGD |
| | | SNITETIENLRDQLE | TETIENLRD |
| | | NAWRLAKFSPKNLDE | AKFSPKNLD |
| | HLA-DRB1*0701 | LSENKILPFTSFSVR | ILPFTSFSV |
| | | SENKILPFTSFSVRK | ILPFTSFSV |
| | | ENKILPFTSFSVRKN | ILPFTSFSV |
| | | NKILPFTSFSVRKNF | ILPFTSFSV |
| | | ILSENKILPFTSFSV | ILSENKILP |
| | | ILPFTSFSVRKNFIY | ILPFTSFSV |
| | | KILPFTSFSVRKNFI | ILPFTSFSV |
| | | LARPLTNSNSNSSYY | LTNSNSNSS |
| | | ARPLTNSNSNSSYYG | LTNSNSNSS |
| | | RPLTNSNSNSSYYGK | LTNSNSNSS |
| | | SESNFEINKNSSLYV | EINKNSSLY |
| | | LPFTSFSVRKNFIYL | FSVRKNFIY |
| | | ESNFEINKNSSLYVD | INKNSSLYV |
| | HLA-DRB1*0802 | None | |

| | HLA-DRB1*0901 | FDYDRSSAELIAKVI | YDRSSAELI |
|---|---|---|---|
| | | SFDYDRSSAELIAKV | YDRSSAELI |
| | | KSFDYDRSSAELIAK | YDRSSAELI |
| | | IKSFDYDRSSAELIA | YDRSSAELI |
| | | IIKSFDYDRSSAELI | IIKSFDYDR |
| | | SVDVFSISSKSELDS | FSISSKSEL |
| | | VDVFSISSKSELDSI | FSISSKSEL |
| | | ASVDVFSISSKSELD | FSISSKSEL |
| | | DVFSISSKSELDSIL | FSISSKSEL |
| | | KASVDVFSISSKSEL | DVFSISSKS |
| | | YDRSSAELIAKVITI | YDRSSAELI |
| | | DYDRSSAELIAKVIT | YDRSSAELI |
| | HLA-DRB1*1101 | None | |
| | HLA-DRB1*1302 | SPVFLEVIDPITNLG | VFLEVIDPI |
| | | LNKLENLKVVSESNF | LENLKVVSE |
| | | IAKVITIYNAVYRGD | ITIYNAVYR |
| | | AKVITIYNAVYRGDL | ITIYNAVYR |
| | | ILNKLENLKVVSESN | LENLKVVSE |
| | | GDLNNDKNLMLPEDQ | LNNDKNLML |
| | | SKEEVDNKAINLQKI | VDNKAINLQ |
| | | EINKNSSLYVDSKMI | INKNSSLYV |
| | | KDLVVIKMDSGKAKL | IKMDSGKAK |
| | | KEEVDNKAINLQKID | VDNKAINLQ |
| | | INKNSSLYVDSKMIL | INKNSSLYV |
| | | VFLEVIDPITNLGTL | VIDPITNLG |
| | | KVITIYNAVYRGDLD | ITIYNAVYR |
| | | PVFLEVIDPITNLGT | VIDPITNLG |
| | | NKLENLKVVSESNFE | LKVVSESNF |
| | | IFSFFLIFLNGFPLN | LIFLNGFPL |
| | | FSFFLIFLNGFPLNA | LIFLNGFPL |
| | | EFILSENKILPFTSF | ILSENKILP |
| | | AINLQKIDPKVKDQT | INLQKIDPK |
| | | DLVVIKMDSGKAKLQ | IKMDSGKAK |
| | | MKKMLLIFSFFLIFL | LLIFSFFLI |
| | | DKDLTTMSIDSSSPV | LTTMSIDSS |
| | | SRASLGDLNNDKNLM | LGDLNNDKN |
| | | FLIFLNGFPLNARKV | LIFLNGFPL |
| | HLA-DRB1*1501 | SFFLIFLNGFPLNAR | FLIFLNGFP |
| | | FFLIFLNGFPLNARK | LNGFPLNAR |
| | | FLIFLNGFPLNARKV | LNGFPLNAR |
| | | LIFLNGFPLNARKVD | LNGFPLNAR |
| | | SENKILPFTSFSVRK | ILPFTSFSV |
| | | ENKILPFTSFSVRKN | ILPFTSFSV |
| | | LSENKILPFTSFSVR | ILPFTSFSV |
| | | NKILPFTSFSVRKNF | ILPFTSFSV |
| | | LDSILNLRRILTGYI | LNLRRILTG |
| | | DSILNLRRILTGYII | LRRILTGYI |
| | | IFLNGFPLNARKVDK | LNGFPLNAR |
| | | SILNLRRILTGYIIK | LRRILTGYI |
| | | KILPFTSFSVRKNFI | LPFTSFSVR |
| | | ILNLRRILTGYIIKS | LRRILTGYI |
| | | SELDSILNLRRILTG | LDSILNLRR |
| | | ELDSILNLRRILTGY | LNLRRILTG |
| | | LGTLQLIDLNTGVRL | LIDLNTGVR |
| | | GTLQLIDLNTGVRLK | LIDLNTGVR |
| | | LNLRRILTGYIIKSF | LRRILTGYI |

| | | | |
|---|---|---|---|
| | | NLGTLQLIDLNTGVR | LQLIDLNTG |
| | | EKDLVVIKMDSGKAK | LVVIKMDSG |
| | | KDLVVIKMDSGKAKL | LVVIKMDSG |
| | | ILPFTSFSVRKNFIY | ILPFTSFSV |
| | | FLNGFPLNARKVDKE | LNGFPLNAR |
| | | QQGIQRYGIYEREKD | IQRYGIYER |
| | | QGIQRYGIYEREKDL | IQRYGIYER |
| | | TQQGIQRYGIYEREK | IQRYGIYER |
| | | TLQLIDLNTGVRLKE | LIDLNTGVR |
| | | ESTQQGIQRYGIYER | QGIQRYGIY |
| | | NSSYYGKYFINRFID | YYGKYFINR |
| | | SSYYGKYFINRFIDD | YYGKYFINR |
| | | LQLIDLNTGVRLKES | LIDLNTGVR |
| | | STQQGIQRYGIYERE | IQRYGIYER |
| | | SNSSYYGKYFINRFI | YYGKYFINR |
| | | DELKNLVILDVNTLK | LKNLVILDV |
| | | LNGFPLNARKVDKEK | LNGFPLNAR |
| | | SNAWRLAKFSPKNLD | WRLAKFSPK |
| | | DSNAWRLAKFSPKNL | WRLAKFSPK |
| | | NAWRLAKFSPKNLDE | WRLAKFSPK |
| | | DLVVIKMDSGKAKLQ | VIKMDSGKA |
| | | FSFFLIFLNGFPLNA | LIFLNGFPL |
| | | IFSFFLIFLNGFPLN | LIFLNGFPL |
| | | QDELKNLVILDVNTL | LKNLVILDV |
| | | LIFSFFLIFLNGFPL | FFLIFLNGF |
| | | REKDLVVIKMDSGKA | LVVIKMDSG |
| | | ILSENKILPFTSFSV | SENKILPFT |
| | | LVVIKMDSGKAKLQI | VIKMDSGKA |
| | | LLIFSFFLIFLNGFP | LLIFSFFLI |
| | | NSNSSYYGKYFINRF | YYGKYFINR |
| | | ELIAKVITIYNAVYR | VITIYNAVY |
| | | LIAKVITIYNAVYRG | ITIYNAVYR |
| | HLA-DRB3*0101 | DLTTMSIDSSSPVFL | MSIDSSSPV |
| | | LTTMSIDSSSPVFLE | MSIDSSSPV |
| | | TTMSIDSSSPVFLEV | MSIDSSSPV |
| | | KDLTTMSIDSSSPVF | MSIDSSSPV |
| | | DKDLTTMSIDSSSPV | TMSIDSSSP |
| | HLA-DRB4*0101 | None | |
| | HLA-DRB5*0101 | AINLQKIDPKVKDQT | LQKIDPKVK |
| | | INLQKIDPKVKDQTT | LQKIDPKVK |
| | | KSELDSILNLRRILT | LDSILNLRR |
| | | SELDSILNLRRILTG | LDSILNLRR |
| | | VNYKGPYDSTNTYEQ | VNYKGPYDS |
| | | KKEFKPVSEVEKLDK | FKPVSEVEK |
| | | LDGKKEFKPVSEVEK | LDGKKEFKP |
| | | KEFKPVSEVEKLDKI | FKPVSEVEK |
| | | DGKKEFKPVSEVEKL | FKPVSEVEK |
| | | GKKEFKPVSEVEKLD | FKPVSEVEK |
| | | | |
| NP_212375.1\| glycerol kinase (glpK) [Borrelia burgdorferi B31]<br><br>Seq27 | HLA-DRB1*0101 | VYFVPAFVGLGAPHW | FVPAFVGLG |
| | | YFVPAFVGLGAPHWD | FVGLGAPHW |
| | | FVPAFVGLGAPHWDS | FVGLGAPHW |
| | | VPAFVGLGAPHWDSY | FVGLGAPHW |
| | | PAFVGLGAPHWDSYA | FVGLGAPHW |
| | | AAYLAGLATGYWQSA | YLAGLATGY |

| | | | |
|---|---|---|---|
| | | QKEFTQIYPQPSWVE | FTQIYPQPS |
| | | KEFTQIYPQPSWVEH | FTQIYPQPS |
| | | ALGAAYLAGLATGYW | YLAGLATGY |
| | | LGAAYLAGLATGYWQ | YLAGLATGY |
| | | GAAYLAGLATGYWQS | YLAGLATGY |
| | | AYLAGLATGYWQSAE | LAGLATGYW |
| | | FAQKEFTQIYPQPSW | FTQIYPQPS |
| | | GFAQKEFTQIYPQPS | FAQKEFTQI |
| | | TALGAAYLAGLATGY | LGAAYLAGL |
| | | AQKEFTQIYPQPSWV | FTQIYPQPS |
| | | ACLKKGMAKNTYGTG | LKKGMAKNT |
| | | AFVGLGAPHWDSYAR | FVGLGAPHW |
| | | QACLKKGMAKNTYGT | LKKGMAKNT |
| | | EFTQIYPQPSWVEHD | FTQIYPQPS |
| | | FVGLGAPHWDSYARG | FVGLGAPHW |
| | | FGQACLKKGMAKNTY | LKKGMAKNT |
| | | FTQIYPQPSWVEHDP | FTQIYPQPS |
| | | KVVRPKITETTALGA | VRPKITETT |
| | | YLAGLATGYWQSAEE | YLAGLATGY |
| | | TFGQACLKKGMAKNT | FGQACLKKG |
| | | GQACLKKGMAKNTYG | LKKGMAKNT |
| | | EGSVFIGGAVIQWLR | FIGGAVIQW |
| | | GSVFIGGAVIQWLRD | FIGGAVIQW |
| | | LEGSVFIGGAVIQWL | FIGGAVIQW |
| | | CKVVRPKITETTALG | VRPKITETT |
| | | LECKVVRPKITETTA | VRPKITETT |
| | | ECKVVRPKITETTAL | VRPKITETT |
| | | LLECKVVRPKITETT | CKVVRPKIT |
| | | TETTALGAAYLAGLA | TTALGAAYL |
| | | ITETTALGAAYLAGL | TTALGAAYL |
| | | SVFIGGAVIQWLRDG | FIGGAVIQW |
| | | ETTALGAAYLAGLAT | LGAAYLAGL |
| | | TTALGAAYLAGLATG | LGAAYLAGL |
| | | LAGLATGYWQSAEEI | LATGYWQSA |
| | | KALFGAEIPIAGIAG | FGAEIPIAG |
| | | DKALFGAEIPIAGIA | FGAEIPIAG |
| | | KTDKALFGAEIPIAG | LFGAEIPIA |
| | | TDKALFGAEIPIAGI | FGAEIPIAG |
| | | GVITEAMANARILPN | ITEAMANAR |
| | | QLGVITEAMANARIL | ITEAMANAR |
| | | LGVITEAMANARILP | ITEAMANAR |
| | | NGGVYFVPAFVGLGA | FVPAFVGLG |
| | | GGVYFVPAFVGLGAP | FVPAFVGLG |
| | | SVTYVLEGSVFIGGA | YVLEGSVFI |
| | | PKITETTALGAAYLA | TTALGAAYL |
| | | TEIWGSQLGVITEAM | WGSQLGVIT |
| | | KSVTYVLEGSVFIGG | YVLEGSVFI |
| | | VTYVLEGSVFIGGAV | YVLEGSVFI |
| | | GSTKAHITRAALESI | TKAHITRAA |
| | | KKSVTYVLEGSVFIG | YVLEGSVFI |
| | | PTEIWGSQLGVITEA | WGSQLGVIT |
| | | DQFAATFGQACLKKG | FAATFGQAC |
| | | RKKSVTYVLEGSVFI | VTYVLEGSV |
| | | VFIGGAVIQWLRDGL | IGGAVIQWL |
| | | DPTEIWGSQLGVITE | WGSQLGVIT |
| | | VLEGSVFIGGAVIQW | VFIGGAVIQ |
| | | DNGGVYFVPAFVGLG | YFVPAFVGL |

| | | | |
|---|---|---|---|
| | | HDPTEIWGSQLGVIT | IWGSQLGVI |
| | | ALFGAEIPIAGIAGD | FGAEIPIAG |
| | | GVYFVPAFVGLGAPH | FVPAFVGLG |
| | | LCFGTIDTWILWNLT | FGTIDTWIL |
| | | SQLGVITEAMANARI | ITEAMANAR |
| | | RPKITETTALGAAYL | ITETTALGA |
| | | CLKKGMAKNTYGTGC | LKKGMAKNT |
| | | VITEAMANARILPNE | MANARILPN |
| | | VRPKITETTALGAAY | VRPKITETT |
| | | KITETTALGAAYLAG | TTALGAAYL |
| | | LKKGMAKNTYGTGCF | LKKGMAKNT |
| | | FAATFGQACLKKGMA | FGQACLKKG |
| | | QFAATFGQACLKKGM | FGQACLKKG |
| | | KAHITRAALESIAFQ | ITRAALESI |
| | | ELCFGTIDTWILWNL | FGTIDTWIL |
| | | IQELRVDGGASQNNL | VDGGASQNN |
| | | ENGELCFGTIDTWIL | LCFGTIDTW |
| | | GELCFGTIDTWILWN | FGTIDTWIL |
| | | QELRVDGGASQNNLL | VDGGASQNN |
| | | NGELCFGTIDTWILW | FGTIDTWIL |
| | | DELLSILNVPRAILP | LSILNVPRA |
| | | EIQELRVDGGASQNN | LRVDGGASQ |
| | | IYNAIVWQDRRTAKI | IVWQDRRTA |
| | | AATFGQACLKKGMAK | FGQACLKKG |
| | | ELRVDGGASQNNLLM | VDGGASQNN |
| | | ELLSILNVPRAILPE | LNVPRAILP |
| | | ITEAMANARILPNEI | MANARILPN |
| | | ATFGQACLKKGMAKN | FGQACLKKG |
| | | LRVDGGASQNNLLMQ | VDGGASQNN |
| | | YNAIVWQDRRTAKIC | WQDRRTAKI |
| | | AHITRAALESIAFQS | ITRAALESI |
| | | GSQLGVITEAMANAR | VITEAMANA |
| | | YVLEGSVFIGGAVIQ | YVLEGSVFI |
| | | RGTIIGITRGSTKAH | GITRGSTKA |
| | | LLSILNVPRAILPEL | LNVPRAILP |
| | | LSILNVPRAILPELK | LNVPRAILP |
| | | AGLATGYWQSAEEIV | LATGYWQSA |
| | | KNTYGTGCFLTVNIG | YGTGCFLTV |
| | | GMAKNTYGTGCFLTV | GMAKNTYGT |
| | | WGSQLGVITEAMANA | WGSQLGVIT |
| | | YILSIDQGTTSSRAM | IDQGTTSSR |
| | | GTIIGITRGSTKAHI | ITRGSTKAH |
| | | NAIVWQDRRTAKICD | WQDRRTAKI |
| | | NTYGTGCFLTVNIGK | YGTGCFLTV |
| | | TIIGITRGSTKAHIT | ITRGSTKAH |
| | | AKNTYGTGCFLTVNI | YGTGCFLTV |
| | | MAKNTYGTGCFLTVN | YGTGCFLTV |
| | | IIGITRGSTKAHITR | ITRGSTKAH |
| | | DKLLTSIAWGRKKSV | LLTSIAWGR |
| | | SILNVPRAILPELKE | LNVPRAILP |
| | | HATDYSNASRTLLLN | YSNASRTLL |
| | | ATDYSNASRTLLLNI | YSNASRTLL |
| | | EHATDYSNASRTLLL | YSNASRTLL |
| | | KYILSIDQGTTSSRA | IDQGTTSSR |
| | | GTIDTWILWNLTQKK | DTWILWNLT |
| | | TDYSNASRTLLLNIK | YSNASRTLL |
| | | LSIDQGTTSSRAMVF | IDQGTTSSR |

| | | | |
|---|---|---|---|
| | | TIDTWILWNLTQKKE | ILWNLTQKK |
| | | IGITRGSTKAHITRA | ITRGSTKAH |
| | | FIGGAVIQWLRDGLE | FIGGAVIQW |
| | | TEAMANARILPNEID | MANARILPN |
| | | ILSIDQGTTSSRAMV | IDQGTTSSR |
| | | AIVWQDRRTAKICDQ | WQDRRTAKI |
| | | LFGAEIPIAGIAGDQ | FGAEIPIAG |
| | | GITRGSTKAHITRAA | ITRGSTKAH |
| | | KEHATDYSNASRTLL | DYSNASRTL |
| | | LATGYWQSAEEIVSL | LATGYWQSA |
| | | IVWQDRRTAKICDQL | WQDRRTAKI |
| | | GLATGYWQSAEEIVS | LATGYWQSA |
| | | RAILPELKESSTIYG | LPELKESST |
| | | LLTSIAWGRKKSVTY | IAWGRKKSV |
| | | AILPELKESSTIYGK | LKESSTIYG |
| | | HITRAALESIAFQSF | ITRAALESI |
| | | ITRGSTKAHITRAAL | TKAHITRAA |
| | | ILPELKESSTIYGKT | LKESSTIYG |
| | | LPELKESSTIYGKTD | LKESSTIYG |
| | | IDTWILWNLTQKKEH | ILWNLTQKK |
| | | DTWILWNLTQKKEHA | ILWNLTQKK |
| | | LTSIAWGRKKSVTYV | IAWGRKKSV |
| | | ISHDKLLTSIAWGRK | LLTSIAWGR |
| | | TSIAWGRKKSVTYVL | IAWGRKKSV |
| | | IWGSQLGVITEAMAN | WGSQLGVIT |
| | | FGTIDTWILWNLTQK | FGTIDTWIL |
| | | IISHDKLLTSIAWGR | DKLLTSIAW |
| | | TQIYPQPSWVEHDPT | IYPQPSWVE |
| | | GKTDKALFGAEIPIA | DKALFGAEI |
| | | TYVLEGSVFIGGAVI | YVLEGSVFI |
| | | ITRAALESIAFQSFD | ITRAALESI |
| | | KPIYNAIVWQDRRTA | YNAIVWQDR |
| | | SHDKLLTSIAWGRKK | LLTSIAWGR |
| | | CFGTIDTWILWNLTQ | FGTIDTWIL |
| | | PELKESSTIYGKTDK | LKESSTIYG |
| | | KLLTSIAWGRKKSVT | IAWGRKKSV |
| | | TWILWNLTQKKEHAT | ILWNLTQKK |
| | | SDNGGVYFVPAFVGL | VYFVPAFVG |
| | | DDELLSILNVPRAIL | LSILNVPRA |
| | | SSDAEALASSVSDNG | AEALASSVS |
| | | SIDQGTTSSRAMVFD | DQGTTSSRA |
| | | EAMANARILPNEIDA | MANARILPN |
| | | FRKSSDAEALASSVS | FRKSSDAEA |
| | | SDAEALASSVSDNGG | AEALASSVS |
| | | FGAEIPIAGIAGDQF | FGAEIPIAG |
| | | PIYNAIVWQDRRTAK | YNAIVWQDR |
| | | MKYILSIDQGTTSSR | MKYILSIDQ |
| | | HDKLLTSIAWGRKKS | LLTSIAWGR |
| | | WDDELLSILNVPRAI | LSILNVPRA |
| | | VDGGASQNNLLMQFQ | VDGGASQNN |
| | | EWDDELLSILNVPRA | LLSILNVPR |
| | | RVDGGASQNNLLMQF | VDGGASQNN |
| | | SIAFQSFDILNTMKK | FQSFDILNT |
| | | DYSNASRTLLLNIKT | YSNASRTLL |
| | | IAFQSFDILNTMKKS | FQSFDILNT |
| | | LKESSTIYGKTDKAL | LKESSTIYG |
| | | AIGITNQRETTVIWE | ITNQRETTV |

1366

| | | | |
|---|---|---|---|
| | | IDQGTTSSRAMVFDK | IDQGTTSSR |
| | | ENWNKAVGKAKSWIQ | WNKAVGKAK |
| | | DAIGITNQRETTVIW | ITNQRETTV |
| | | IGITNQRETTVIWEK | ITNQRETTV |
| | | ILNVPRAILPELKES | LNVPRAILP |
| | | IDAIGITNQRETTVI | ITNQRETTV |
| | | SIAWGRKKSVTYVLE | WGRKKSVTY |
| | | RGSTKAHITRAALES | TKAHITRAA |
| | | WDSYARGTIIGITRG | YARGTIIGI |
| | | IAWGRKKSVTYVLEG | WGRKKSVTY |
| | | EIDAIGITNQRETTV | IDAIGITNQ |
| | | DSYARGTIIGITRGS | YARGTIIGI |
| | | LNVPRAILPELKESS | LNVPRAILP |
| | | TYGTGCFLTVNIGKE | YGTGCFLTV |
| | | NTGKPIYNAIVWQDR | IYNAIVWQD |
| | | IYPQPSWVEHDPTEI | IYPQPSWVE |
| | HLA-DRB1*0301 | None | |
| | HLA-DRB1*0401 | TQKKEHATDYSNASR | HATDYSNAS |
| | | QKKEHATDYSNASRT | HATDYSNAS |
| | | LTQKKEHATDYSNAS | QKKEHATDY |
| | | KKEHATDYSNASRTL | HATDYSNAS |
| | | KEHATDYSNASRTLL | HATDYSNAS |
| | | EHATDYSNASRTLLL | HATDYSNAS |
| | | HATDYSNASRTLLLN | HATDYSNAS |
| | | FDILNTMKKSIPNFE | FDILNTMKK |
| | | MKYILSIDQGTTSSR | MKYILSIDQ |
| | | DILNTMKKSIPNFEI | MKKSIPNFE |
| | | ILNTMKKSIPNFEIQ | MKKSIPNFE |
| | | LNTMKKSIPNFEIQE | MKKSIPNFE |
| | HLA-DRB1*0404 | VYFVPAFVGLGAPHW | FVPAFVGLG |
| | | YFVPAFVGLGAPHWD | FVGLGAPHW |
| | | FVPAFVGLGAPHWDS | FVGLGAPHW |
| | | TIDTWILWNLTQKKE | ILWNLTQKK |
| | | DTWILWNLTQKKEHA | ILWNLTQKK |
| | | GTIDTWILWNLTQKK | DTWILWNLT |
| | | IDTWILWNLTQKKEH | ILWNLTQKK |
| | | TWILWNLTQKKEHAT | ILWNLTQKK |
| | | NGGVYFVPAFVGLGA | FVPAFVGLG |
| | | GVYFVPAFVGLGAPH | FVPAFVGLG |
| | | GGVYFVPAFVGLGAP | FVPAFVGLG |
| | | DNGGVYFVPAFVGLG | GVYFVPAFV |
| | | NASRTLLLNIKTLEW | LLLNIKTLE |
| | | ASRTLLLNIKTLEWD | LLNIKTLEW |
| | | SRTLLLNIKTLEWDD | LLNIKTLEW |
| | | RTLLLNIKTLEWDDE | LLNIKTLEW |
| | | TKIMWILDNVEGARQ | IMWILDNVE |
| | | GTKIMWILDNVEGAR | IMWILDNVE |
| | | EWDDELLSILNVPRA | LLSILNVPR |
| | | DDELLSILNVPRAIL | LSILNVPRA |
| | | WDDELLSILNVPRAI | LSILNVPRA |
| | | DELLSILNVPRAILP | LSILNVPRA |
| | | TLLLNIKTLEWDDEL | LLNIKTLEW |
| | | AAYLAGLATGYWQSA | LAGLATGYW |
| | | AYLAGLATGYWQSAE | LAGLATGYW |
| | | GSQLGVITEAMANAR | VITEAMANA |
| | | VPAFVGLGAPHWDSY | FVGLGAPHW |

|  |  | LGVITEAMANARILP | VITEAMANA |
|---|---|---|---|
|  |  | WILWNLTQKKEHATD | ILWNLTQKK |
|  |  | SQLGVITEAMANARI | VITEAMANA |
|  |  | ILWNLTQKKEHATDY | ILWNLTQKK |
|  |  | QLGVITEAMANARIL | VITEAMANA |
|  |  | ELLSILNVPRAILPE | LSILNVPRA |
|  |  | PAFVGLGAPHWDSYA | FVGLGAPHW |
|  |  | SDAEALASSVSDNGG | LASSVSDNG |
|  |  | SGTKIMWILDNVEGA | IMWILDNVE |
|  | HLA-DRB1*0405 | QKEFTQIYPQPSWVE | FTQIYPQPS |
|  |  | KEFTQIYPQPSWVEH | IYPQPSWVE |
|  |  | EFTQIYPQPSWVEHD | IYPQPSWVE |
|  |  | FTQIYPQPSWVEHDP | IYPQPSWVE |
|  |  | TEAMANARILPNEID | MANARILPN |
|  |  | EAMANARILPNEIDA | ARILPNEID |
|  |  | TQIYPQPSWVEHDPT | IYPQPSWVE |
|  |  | AMANARILPNEIDAI | ARILPNEID |
|  |  | MANARILPNEIDAIG | ARILPNEID |
|  |  | IYPQPSWVEHDPTEI | IYPQPSWVE |
|  |  | ANARILPNEIDAIGI | ARILPNEID |
|  |  | QIYPQPSWVEHDPTE | IYPQPSWVE |
|  |  | GVITEAMANARILPN | ITEAMANAR |
|  |  | GTIDTWILWNLTQKK | WILWNLTQK |
|  |  | ITEAMANARILPNEI | MANARILPN |
|  |  | FGTIDTWILWNLTQK | TWILWNLTQ |
|  |  | TIDTWILWNLTQKKE | WILWNLTQK |
|  |  | FAQKEFTQIYPQPSW | FTQIYPQPS |
|  |  | FIGGAVIQWLRDGLE | GAVIQWLRD |
|  |  | VITEAMANARILPNE | MANARILPN |
|  |  | IYNAIVWQDRRTAKI | IYNAIVWQD |
|  |  | SGTKIMWILDNVEGA | IMWILDNVE |
|  |  | IDTWILWNLTQKKEH | WILWNLTQK |
|  |  | IGGAVIQWLRDGLEF | IQWLRDGLE |
|  |  | NARILPNEIDAIGIT | ARILPNEID |
|  |  | FSGTKIMWILDNVEG | IMWILDNVE |
|  |  | ARILPNEIDAIGITN | ARILPNEID |
|  |  | AQKEFTQIYPQPSWV | FTQIYPQPS |
|  |  | YPQPSWVEHDPTEIW | WVEHDPTEI |
|  |  | TKIMWILDNVEGARQ | IMWILDNVE |
|  |  | NTGKPIYNAIVWQDR | IYNAIVWQD |
|  |  | GTKIMWILDNVEGAR | IMWILDNVE |
|  |  | GGAVIQWLRDGLEFF | IQWLRDGLE |
|  |  | PQPSWVEHDPTEIWG | VEHDPTEIW |
|  |  | GFAQKEFTQIYPQPS | QKEFTQIYP |
|  | HLA-DRB1*0701 | EHATDYSNASRTLLL | YSNASRTLL |
|  |  | HATDYSNASRTLLLN | YSNASRTLL |
|  |  | ATDYSNASRTLLLNI | YSNASRTLL |
|  |  | TDYSNASRTLLLNIK | YSNASRTLL |
|  |  | DAIGITNQRETTVIW | ITNQRETTV |
|  |  | LSIDQGTTSSRAMVF | DQGTTSSRA |
|  |  | IDAIGITNQRETTVI | ITNQRETTV |
|  |  | AIGITNQRETTVIWE | ITNQRETTV |
|  |  | IGITNQRETTVIWEK | ITNQRETTV |
|  |  | SIDQGTTSSRAMVFD | TTSSRAMVF |
|  |  | TSIAWGRKKSVTYVL | WGRKKSVTY |
|  |  | SIAWGRKKSVTYVLE | RKKSVTYVL |
|  |  | IAWGRKKSVTYVLEG | RKKSVTYVL |

| | | | |
|---|---|---|---|
| | | KEHATDYSNASRTLL | DYSNASRTL |
| | | IDQGTTSSRAMVFDK | TTSSRAMVF |
| | | AWGRKKSVTYVLEGS | RKKSVTYVL |
| | | KEFTQIYPQPSWVEH | IYPQPSWVE |
| | | QKEFTQIYPQPSWVE | QIYPQPSWV |
| | | EFTQIYPQPSWVEHD | IYPQPSWVE |
| | | WGRKKSVTYVLEGSV | RKKSVTYVL |
| | | TQIYPQPSWVEHDPT | IYPQPSWVE |
| | | DQGTTSSRAMVFDKN | TTSSRAMVF |
| | | FTQIYPQPSWVEHDP | IYPQPSWVE |
| | | EIDAIGITNQRETTV | IGITNQRET |
| | | QGTTSSRAMVFDKNA | TTSSRAMVF |
| | HLA-DRB1*0802 | EKLLENWNKAVGKAK | LENWNKAVG |
| | | KLLENWNKAVGKAKS | WNKAVGKAK |
| | | LLENWNKAVGKAKSW | WNKAVGKAK |
| | | LENWNKAVGKAKSWI | WNKAVGKAK |
| | | ENWNKAVGKAKSWIQ | WNKAVGKAK |
| | | DKLLTSIAWGRKKSV | SIAWGRKKS |
| | | KLLTSIAWGRKKSVT | SIAWGRKKS |
| | | LLTSIAWGRKKSVTY | SIAWGRKKS |
| | | LTSIAWGRKKSVTYV | SIAWGRKKS |
| | | IDTWILWNLTQKKEH | WILWNLTQK |
| | HLA-DRB1*0901 | LATGYWQSAEEIVSL | YWQSAEEIV |
| | | GLATGYWQSAEEIVS | YWQSAEEIV |
| | | ATGYWQSAEEIVSLW | YWQSAEEIV |
| | | TGYWQSAEEIVSLWQ | YWQSAEEIV |
| | | AGLATGYWQSAEEIV | GYWQSAEEI |
| | | HATDYSNASRTLLLN | YSNASRTLL |
| | | EHATDYSNASRTLLL | YSNASRTLL |
| | | WQVDKIFEPSMPKNQ | IFEPSMPKN |
| | | ATDYSNASRTLLLNI | YSNASRTLL |
| | | TDYSNASRTLLLNIK | YSNASRTLL |
| | | GLEFFRKSSDAEALA | FRKSSDAEA |
| | | LEFFRKSSDAEALAS | FRKSSDAEA |
| | | DGLEFFRKSSDAEAL | FRKSSDAEA |
| | | QVDKIFEPSMPKNQK | FEPSMPKNQ |
| | | VDKIFEPSMPKNQKE | FEPSMPKNQ |
| | | DKIFEPSMPKNQKEK | FEPSMPKNQ |
| | | KEHATDYSNASRTLL | TDYSNASRT |
| | | EFFRKSSDAEALASS | FRKSSDAEA |
| | | RDGLEFFRKSSDAEA | FFRKSSDAE |
| | | GYWQSAEEIVSLWQV | YWQSAEEIV |
| | | KIFEPSMPKNQKEKL | FEPSMPKNQ |
| | | PHWDSYARGTIIGIT | WDSYARGTI |
| | | APHWDSYARGTIIGI | WDSYARGTI |
| | HLA-DRB1*1101 | KLLENWNKAVGKAKS | LENWNKAVG |
| | | EKLLENWNKAVGKAK | LENWNKAVG |
| | | IYNAIVWQDRRTAKI | IVWQDRRTA |
| | HLA-DRB1*1302 | FDILNTMKKSIPNFE | LNTMKKSIP |
| | | TDYSNASRTLLLNIK | YSNASRTLL |
| | | FQSFDILNTMKKSIP | FDILNTMKK |
| | | QSFDILNTMKKSIPN | LNTMKKSIP |
| | | DILNTMKKSIPNFEI | LNTMKKSIP |
| | | ATDYSNASRTLLLNI | YSNASRTLL |
| | | EHATDYSNASRTLLL | YSNASRTLL |
| | | HATDYSNASRTLLLN | YSNASRTLL |

| | | | |
|---|---|---|---|
| | | SFDILNTMKKSIPNF | LNTMKKSIP |
| | | ASRTLLLNIKTLEWD | LLLNIKTLE |
| | | KEHATDYSNASRTLL | HATDYSNAS |
| | | SRTLLLNIKTLEWDD | LLLNIKTLE |
| | | RTLLLNIKTLEWDDE | LLLNIKTLE |
| | | NASRTLLLNIKTLEW | LLLNIKTLE |
| | | SNASRTLLLNIKTLE | SRTLLLNIK |
| | | DELLSILNVPRAILP | LSILNVPRA |
| | | ELLSILNVPRAILPE | LSILNVPRA |
| | | ILNTMKKSIPNFEIQ | LNTMKKSIP |
| | | DDELLSILNVPRAIL | LSILNVPRA |
| | | DYSNASRTLLLNIKT | YSNASRTLL |
| | | YSNASRTLLLNIKTL | YSNASRTLL |
| | | LNTMKKSIPNFEIQE | LNTMKKSIP |
| | | LLSILNVPRAILPEL | ILNVPRAIL |
| | | TLLLNIKTLEWDDEL | LLLNIKTLE |
| | | TGCFLTVNIGKEPII | LTVNIGKEP |
| | | LSILNVPRAILPELK | ILNVPRAIL |
| | | GCFLTVNIGKEPIIS | LTVNIGKEP |
| | | LLLNIKTLEWDDELL | LLLNIKTLE |
| | | AFQSFDILNTMKKSI | FDILNTMKK |
| | | CFLTVNIGKEPIISH | LTVNIGKEP |
| | | GTGCFLTVNIGKEPI | LTVNIGKEP |
| | | WDDELLSILNVPRAI | LSILNVPRA |
| | | EIWGSQLGVITEAMA | WGSQLGVIT |
| | | TEIWGSQLGVITEAM | WGSQLGVIT |
| | | HDPTEIWGSQLGVIT | TEIWGSQLG |
| | | DPTEIWGSQLGVITE | WGSQLGVIT |
| | | PTEIWGSQLGVITEA | WGSQLGVIT |
| | | EWDDELLSILNVPRA | ELLSILNVP |
| | | KLLENWNKAVGKAKS | LENWNKAVG |
| | | YGTGCFLTVNIGKEP | FLTVNIGKE |
| | | FLTVNIGKEPIISHD | LTVNIGKEP |
| | | IDAIGITNQRETTVI | IGITNQRET |
| | | DAIGITNQRETTVIW | IGITNQRET |
| | | IAFQSFDILNTMKKS | FDILNTMKK |
| | | IAWGRKKSVTYVLEG | WGRKKSVTY |
| | | SIAWGRKKSVTYVLE | WGRKKSVTY |
| | | EKLLENWNKAVGKAK | LENWNKAVG |
| | | TSIAWGRKKSVTYVL | WGRKKSVTY |
| | | LLTSIAWGRKKSVTY | LTSIAWGRK |
| | | YARGTIIGITRGSTK | RGTIIGITR |
| | | SIAFQSFDILNTMKK | FQSFDILNT |
| | HLA-DRB1*1501 | IDTWILWNLTQKKEH | WILWNLTQK |
| | | DTWILWNLTQKKEHA | WILWNLTQK |
| | | TIDTWILWNLTQKKE | WILWNLTQK |
| | | GTIDTWILWNLTQKK | WILWNLTQK |
| | | FGTIDTWILWNLTQK | IDTWILWNL |
| | | DKLLTSIAWGRKKSV | LLTSIAWGR |
| | | HDKLLTSIAWGRKKS | LLTSIAWGR |
| | | ISHDKLLTSIAWGRK | LLTSIAWGR |
| | | SHDKLLTSIAWGRKK | LLTSIAWGR |
| | | TWILWNLTQKKEHAT | WILWNLTQK |
| | | LLTSIAWGRKKSVTY | LTSIAWGRK |
| | | WILWNLTQKKEHATD | WILWNLTQK |
| | | KLLTSIAWGRKKSVT | LTSIAWGRK |
| | | LTSIAWGRKKSVTYV | WGRKKSVTY |

| | | | |
|---|---|---|---|
| | | WDDELLSILNVPRAI | LLSILNVPR |
| | | DDELLSILNVPRAIL | LLSILNVPR |
| | | EWDDELLSILNVPRA | LLSILNVPR |
| | | DELLSILNVPRAILP | LLSILNVPR |
| | | IISHDKLLTSIAWGR | KLLTSIAWG |
| | | TSIAWGRKKSVTYVL | WGRKKSVTY |
| | | EKLLENWNKAVGKAK | LENWNKAVG |
| | | QKEKLLENWNKAVGK | LENWNKAVG |
| | | NQKEKLLENWNKAVG | LLENWNKAV |
| | | SIAWGRKKSVTYVLE | WGRKKSVTY |
| | | KEKLLENWNKAVGKA | LENWNKAVG |
| | | VYFVPAFVGLGAPHW | VYFVPAFVG |
| | | AFVGLGAPHWDSYAR | FVGLGAPHW |
| | | LEWDDELLSILNVPR | DELLSILNV |
| | HLA-DRB3*0101 | None | |
| | HLA-DRB4*0101 | EEIVSLWQVDKIFEP | VSLWQVDKI |
| | | AEEIVSLWQVDKIFE | VSLWQVDKI |
| | | EIVSLWQVDKIFEPS | VSLWQVDKI |
| | | SAEEIVSLWQVDKIF | VSLWQVDKI |
| | | FSGTKIMWILDNVEG | KIMWILDNV |
| | | SGTKIMWILDNVEGA | KIMWILDNV |
| | | GTKIMWILDNVEGAR | KIMWILDNV |
| | | YFSGTKIMWILDNVE | KIMWILDNV |
| | | IDTWILWNLTQKKEH | WILWNLTQK |
| | | DTWILWNLTQKKEHA | WILWNLTQK |
| | | IVSLWQVDKIFEPSM | VSLWQVDKI |
| | | TIDTWILWNLTQKKE | WILWNLTQK |
| | | GTIDTWILWNLTQKK | WILWNLTQK |
| | | VSLWQVDKIFEPSMP | VSLWQVDKI |
| | | QSAEEIVSLWQVDKI | AEEIVSLWQ |
| | | TKIMWILDNVEGARQ | IMWILDNVE |
| | | AALESIAFQSFDILN | IAFQSFDIL |
| | | RAALESIAFQSFDIL | LESIAFQSF |
| | | TWILWNLTQKKEHAT | WILWNLTQK |
| | | KIMWILDNVEGARQR | KIMWILDNV |
| | | LESIAFQSFDILNTM | IAFQSFDIL |
| | | ALESIAFQSFDILNT | IAFQSFDIL |
| | HLA-DRB5*0101 | EKLLENWNKAVGKAK | LENWNKAVG |
| | | KLLENWNKAVGKAKS | WNKAVGKAK |
| | | LENWNKAVGKAKSWI | WNKAVGKAK |
| | | LLENWNKAVGKAKSW | WNKAVGKAK |
| | | ENWNKAVGKAKSWIQ | WNKAVGKAK |
| | | PIYNAIVWQDRRTAK | YNAIVWQDR |
| | | KPIYNAIVWQDRRTA | YNAIVWQDR |
| | | GKPIYNAIVWQDRRT | YNAIVWQDR |
| | | TGKPIYNAIVWQDRR | YNAIVWQDR |
| | | NTGKPIYNAIVWQDR | GKPIYNAIV |
| | | IYNAIVWQDRRTAKI | YNAIVWQDR |
| | | YNAIVWQDRRTAKIC | YNAIVWQDR |
| | | HDKLLTSIAWGRKKS | LTSIAWGRK |
| | | DKLLTSIAWGRKKSV | LTSIAWGRK |
| | | SHDKLLTSIAWGRKK | LTSIAWGRK |
| | | DKIFEPSMPKNQKEK | FEPSMPKNQ |
| | | IDTWILWNLTQKKEH | WILWNLTQK |
| | | KIFEPSMPKNQKEKL | FEPSMPKNQ |
| | | DTWILWNLTQKKEHA | WILWNLTQK |

| | | | |
|---|---|---|---|
| | | NWNKAVGKAKSWIQN | WNKAVGKAK |
| | | WNKAVGKAKSWIQNS | WNKAVGKAK |
| | | GTIDTWILWNLTQKK | WILWNLTQK |
| | | ISHDKLLTSIAWGRK | LLTSIAWGR |
| | | KLLTSIAWGRKKSVT | LTSIAWGRK |
| | | TIDTWILWNLTQKKE | WILWNLTQK |
| | | QVDKIFEPSMPKNQK | FEPSMPKNQ |
| | | VDKIFEPSMPKNQKE | FEPSMPKNQ |
| | | KEFTQIYPQPSWVEH | IYPQPSWVE |
| | | | |
| NP_212342.1\| hypothetical protein BB0208 [Borrelia burgdorferi B31]  Seq28 | HLA-DRB1*0101 | GTTYKLKSSNFLKLI | YKLKSSNFL |
| | | LGTTYKLKSSNFLKL | YKLKSSNFL |
| | | TTYKLKSSNFLKLIE | YKLKSSNFL |
| | | KLGTTYKLKSSNFLK | YKLKSSNFL |
| | | KKLGTTYKLKSSNFL | LGTTYKLKS |
| | | DIYKQIILNFSSNIN | YKQIILNFS |
| | | KDIYKQIILNFSSNI | YKQIILNFS |
| | | KKDIYKQIILNFSSN | YKQIILNFS |
| | | EKKDIYKQIILNFSS | YKQIILNFS |
| | | QEKKDIYKQIILNFS | IYKQIILNF |
| | | TYKLKSSNFLKLIEI | YKLKSSNFL |
| | | EQTIDILVAVRNRNK | IDILVAVRN |
| | | YKLKSSNFLKLIEIQ | YKLKSSNFL |
| | | QTIDILVAVRNRNKI | IDILVAVRN |
| | | SEFYDSLATLKKHIN | YDSLATLKK |
| | | DSEFYDSLATLKKHI | YDSLATLKK |
| | | DSLEQTIDILVAVRN | LEQTIDILV |
| | | KDSEFYDSLATLKKH | YDSLATLKK |
| | | KKDSEFYDSLATLKK | FYDSLATLK |
| | | SLEQTIDILVAVRNR | IDILVAVRN |
| | | LEQTIDILVAVRNRN | IDILVAVRN |
| | | IYKQIILNFSSNINI | YKQIILNFS |
| | | YKQIILNFSSNINID | YKQIILNFS |
| | | EFYDSLATLKKHINK | YDSLATLKK |
| | | FLKLIEIQNSPYYSQ | LKLIEIQNS |
| | | LKLIEIQNSPYYSQE | IQNSPYYSQ |
| | | KVLKYSISSQLYKLE | LKYSISSQL |
| | | LIEIQNSPYYSQEKK | IQNSPYYSQ |
| | | KLIEIQNSPYYSQEK | IQNSPYYSQ |
| | | EEKVLKYSISSQLYK | LKYSISSQL |
| | | EKVLKYSISSQLYKL | LKYSISSQL |
| | | IEEKVLKYSISSQLY | LKYSISSQL |
| | | IDILVAVRNRNKIKI | IDILVAVRN |
| | | IEIQNSPYYSQEKKD | IQNSPYYSQ |
| | | TIDILVAVRNRNKIK | IDILVAVRN |
| | | ECYYKNTQNEAIKKW | YKNTQNEAI |
| | | YIEEKVLKYSISSQL | KVLKYSISS |
| | | CYYKNTQNEAIKKWI | YKNTQNEAI |
| | | YDSLATLKKHINKYI | YDSLATLKK |
| | | KQIILNFSSNINIDS | ILNFSSNIN |
| | | FYDSLATLKKHINKY | YDSLATLKK |
| | | EECYYKNTQNEAIKK | YKNTQNEAI |
| | | LEECYYKNTQNEAIK | YKNTQNEAI |
| | | SLEECYYKNTQNEAI | YYKNTQNEA |
| | | QIILNFSSNINIDSL | ILNFSSNIN |
| | HLA-DRB1*0301 | None | |

| | | | |
|---|---|---|---|
| | HLA-DRB1*0401 | EECYYKNTQNEAIKK | YYKNTQNEA |
| | | ECYYKNTQNEAIKKW | YYKNTQNEA |
| | | LEECYYKNTQNEAIK | YYKNTQNEA |
| | | SLEECYYKNTQNEAI | YYKNTQNEA |
| | | TSLEECYYKNTQNEA | TSLEECYYK |
| | | DIYKQIILNFSSNIN | YKQIILNFS |
| | | KKDIYKQIILNFSSN | YKQIILNFS |
| | | QEKKDIYKQIILNFS | IYKQIILNF |
| | | EKKDIYKQIILNFSS | YKQIILNFS |
| | | KDIYKQIILNFSSNI | YKQIILNFS |
| | | SNFLKLIEIQNSPYY | LIEIQNSPY |
| | | NFLKLIEIQNSPYYS | IEIQNSPYY |
| | | FLKLIEIQNSPYYSQ | IEIQNSPYY |
| | | LKLIEIQNSPYYSQE | IEIQNSPYY |
| | | IYKQIILNFSSNINI | YKQIILNFS |
| | | CYYKNTQNEAIKKWI | YYKNTQNEA |
| | | KLIEIQNSPYYSQEK | IEIQNSPYY |
| | | YYKNTQNEAIKKWIL | YYKNTQNEA |
| | | YKQIILNFSSNINID | YKQIILNFS |
| | | KKLGTTYKLKSSNFL | LGTTYKLKS |
| | | LGTTYKLKSSNFLKL | YKLKSSNFL |
| | | KLGTTYKLKSSNFLK | YKLKSSNFL |
| | | TTYKLKSSNFLKLIE | YKLKSSNFL |
| | | GTTYKLKSSNFLKLI | YKLKSSNFL |
| | HLA-DRB1*0404 | DIYKQIILNFSSNIN | IILNFSSNI |
| | | IYKQIILNFSSNINI | ILNFSSNIN |
| | | YKQIILNFSSNINID | ILNFSSNIN |
| | | KQIILNFSSNINIDS | ILNFSSNIN |
| | | QIILNFSSNINIDSL | ILNFSSNIN |
| | | EQTIDILVAVRNRNK | ILVAVRNRN |
| | | QTIDILVAVRNRNKI | ILVAVRNRN |
| | | TIDILVAVRNRNKIK | ILVAVRNRN |
| | | LEQTIDILVAVRNRN | TIDILVAVR |
| | | IDILVAVRNRNKIKI | ILVAVRNRN |
| | | KDIYKQIILNFSSNI | YKQIILNFS |
| | | IILNFSSNINIDSLE | ILNFSSNIN |
| | HLA-DRB1*0405 | KQIILNFSSNINIDS | ILNFSSNIN |
| | | DIYKQIILNFSSNIN | YKQIILNFS |
| | | QIILNFSSNINIDSL | ILNFSSNIN |
| | | YKQIILNFSSNINID | ILNFSSNIN |
| | | IYKQIILNFSSNINI | ILNFSSNIN |
| | | ILNFSSNINIDSLEQ | ILNFSSNIN |
| | | IILNFSSNINIDSLE | ILNFSSNIN |
| | HLA-DRB1*0701 | GTTYKLKSSNFLKLI | YKLKSSNFL |
| | | LGTTYKLKSSNFLKL | YKLKSSNFL |
| | | KKLGTTYKLKSSNFL | TYKLKSSNF |
| | | KLGTTYKLKSSNFLK | YKLKSSNFL |
| | | TTYKLKSSNFLKLIE | YKLKSSNFL |
| | HLA-DRB1*0802 | None | |
| | HLA-DRB1*0901 | GTTYKLKSSNFLKLI | YKLKSSNFL |
| | | TTYKLKSSNFLKLIE | YKLKSSNFL |
| | | LGTTYKLKSSNFLKL | YKLKSSNFL |
| | | EKVLKYSISSQLYKL | LKYSISSQL |
| | | EEKVLKYSISSQLYK | LKYSISSQL |
| | | KVLKYSISSQLYKLE | LKYSISSQL |
| | | KLGTTYKLKSSNFLK | YKLKSSNFL |

| | | | |
|---|---|---|---|
| | | KKLGTTYKLKSSNFL | LGTTYKLKS |
| | | IEEKVLKYSISSQLY | LKYSISSQL |
| | | YIEEKVLKYSISSQL | VLKYSISSQ |
| | | VLKYSISSQLYKLEK | YSISSQLYK |
| | | IYKQIILNFSSNINI | ILNFSSNIN |
| | | YKQIILNFSSNINID | LNFSSNINI |
| | | TYKLKSSNFLKLIEI | YKLKSSNFL |
| | | KQIILNFSSNINIDS | LNFSSNINI |
| | | QIILNFSSNINIDSL | LNFSSNINI |
| | | YKLKSSNFLKLIEIQ | YKLKSSNFL |
| | | LKYSISSQLYKLEKP | YSISSQLYK |
| HLA-DRB1*1101 | | None | |
| HLA-DRB1*1302 | | RNKIKILNILNKNLK | IKILNILNK |
| | | VRNRNKIKILNILNK | VRNRNKIKI |
| | | ILVAVRNRNKIKILN | VRNRNKIKI |
| | | NRNKIKILNILNKNL | IKILNILNK |
| | | DILVAVRNRNKIKIL | VRNRNKIKI |
| | | DIYKQIILNFSSNIN | YKQIILNFS |
| | | IDILVAVRNRNKIKI | VAVRNRNKI |
| | | RNRNKIKILNILNKN | IKILNILNK |
| | | IYKQIILNFSSNINI | ILNFSSNIN |
| | | VAVRNRNKIKILNIL | VRNRNKIKI |
| | | YKQIILNFSSNINID | ILNFSSNIN |
| | | LVAVRNRNKIKILNI | VRNRNKIKI |
| | | KQIILNFSSNINIDS | ILNFSSNIN |
| | | QIILNFSSNINIDSL | ILNFSSNIN |
| | | AVRNRNKIKILNILN | VRNRNKIKI |
| | | KDIYKQIILNFSSNI | YKQIILNFS |
| | | QTIDILVAVRNRNKI | ILVAVRNRN |
| | | TIDILVAVRNRNKIK | VAVRNRNKI |
| | | IILNFSSNINIDSLE | ILNFSSNIN |
| | | LGTTYKLKSSNFLKL | YKLKSSNFL |
| | | KKDIYKQIILNFSSN | YKQIILNFS |
| | | QEKKDIYKQIILNFS | IYKQIILNF |
| | | EKKDIYKQIILNFSS | YKQIILNFS |
| | | KEANLNTKKLGTTYK | ANLNTKKLG |
| | | ISKEANLNTKKLGTT | ANLNTKKLG |
| | | KKLGTTYKLKSSNFL | LGTTYKLKS |
| | | GTTYKLKSSNFLKLI | YKLKSSNFL |
| | | SKEANLNTKKLGTTY | ANLNTKKLG |
| | | TTYKLKSSNFLKLIE | YKLKSSNFL |
| | | ILNFSSNINIDSLEQ | ILNFSSNIN |
| | | EANLNTKKLGTTYKL | LNTKKLGTT |
| | | KLGTTYKLKSSNFLK | YKLKSSNFL |
| | | ANLNTKKLGTTYKLK | LNTKKLGTT |
| | | KWILEIVRNKNFIQI | IVRNKNFIQ |
| | | ILEIVRNKNFIQISK | VRNKNFIQI |
| | | TYKLKSSNFLKLIEI | YKLKSSNFL |
| | | WILEIVRNKNFIQIS | IVRNKNFIQ |
| | | YKLKSSNFLKLIEIQ | YKLKSSNFL |
| | | LEIVRNKNFIQISKE | VRNKNFIQI |
| | | FLKLIEIQNSPYYSQ | IEIQNSPYY |
| | | LEQTIDILVAVRNRN | IDILVAVRN |
| | | IQISKEANLNTKKLG | EANLNTKKL |
| | | LKLIEIQNSPYYSQE | IEIQNSPYY |
| | | DSLATLKKHINKYIE | LATLKKHIN |
| | | YDSLATLKKHINKYI | LATLKKHIN |

| | | | |
|---|---|---|---|
| | | NLNTKKLGTTYKLKS | LNTKKLGTT |
| | | EQTIDILVAVRNRNK | ILVAVRNRN |
| | | LNTKKLGTTYKLKSS | LNTKKLGTT |
| | HLA-DRB1*1501 | RNKIKILNILNKNLK | IKILNILNK |
| | | NRNKIKILNILNKNL | IKILNILNK |
| | | VRNRNKIKILNILNK | VRNRNKIKI |
| | | RNRNKIKILNILNKN | IKILNILNK |
| | | IDILVAVRNRNKIKI | LVAVRNRNK |
| | | DILVAVRNRNKIKIL | LVAVRNRNK |
| | | NEAIKKWILEIVRNK | IKKWILEIV |
| | | EAIKKWILEIVRNKN | IKKWILEIV |
| | | KWILEIVRNKNFIQI | IVRNKNFIQ |
| | | WILEIVRNKNFIQIS | VRNKNFIQI |
| | | QNEAIKKWILEIVRN | IKKWILEIV |
| | | TIDILVAVRNRNKIK | LVAVRNRNK |
| | | LVAVRNRNKIKILNI | LVAVRNRNK |
| | | ILVAVRNRNKIKILN | LVAVRNRNK |
| | | QTIDILVAVRNRNKI | LVAVRNRNK |
| | | TQNEAIKKWILEIVR | IKKWILEIV |
| | | NTQNEAIKKWILEIV | EAIKKWILE |
| | | ILEIVRNKNFIQISK | VRNKNFIQI |
| | HLA-DRB3*0101 | None | |
| | HLA-DRB4*0101 | SISSQLYKLEKPEII | LYKLEKPEI |
| | | SQLYKLEKPEIIELI | YKLEKPEII |
| | | SSQLYKLEKPEIIEL | YKLEKPEII |
| | | ISSQLYKLEKPEIIE | YKLEKPEII |
| | HLA-DRB5*0101 | EQTIDILVAVRNRNK | ILVAVRNRN |
| | | QTIDILVAVRNRNKI | LVAVRNRNK |
| | | IDILVAVRNRNKIKI | LVAVRNRNK |
| | | TIDILVAVRNRNKIK | LVAVRNRNK |
| | | IKISNDYEKEKNAFN | ISNDYEKEK |
| | | | |
| NP_045482.1\| hypothetical protein BBG22 [Borrelia burgdorferi B31]<br><br>Seq29 | HLA-DRB1*0101 | | |
| | | GTLTAKFAMEGSILT | LTAKFAMEG |
| | | LTAKFAMEGSILTRS | FAMEGSILT |
| | | TAKFAMEGSILTRSK | FAMEGSILT |
| | | AKFAMEGSILTRSKL | FAMEGSILT |
| | | TLTAKFAMEGSILTR | FAMEGSILT |
| | | KRMTMIYRSADTWYT | KRMTMIYRS |
| | | RMTMIYRSADTWYTW | YRSADTWYT |

| | | | |
|---|---|---|---|
| | | PVGTLTAKFAMEGSI | LTAKFAMEG |
| | | FLEKTPVGTLTAKFA | FLEKTPVGT |
| | | LEKTPVGTLTAKFAM | VGTLTAKFA |
| | | EKTPVGTLTAKFAME | VGTLTAKFA |
| | | YAYKVLGISSAPKLS | YKVLGISSA |
| | HLA-DRB1*0301 | None | |
| | HLA-DRB1*0401 | TAKFAMEGSILTRSK | FAMEGSILT |
| | | AKFAMEGSILTRSKL | FAMEGSILT |
| | | LTAKFAMEGSILTRS | FAMEGSILT |
| | | TLTAKFAMEGSILTR | FAMEGSILT |
| | | GTLTAKFAMEGSILT | GTLTAKFAM |
| | | EHMILFFLEKTPVGT | FFLEKTPVG |
| | | LEHMILFFLEKTPVG | LFFLEKTPV |
| | | HMILFFLEKTPVGTL | FFLEKTPVG |
| | | MILFFLEKTPVGTLT | FFLEKTPVG |
| | | ILFFLEKTPVGTLTA | FFLEKTPVG |
| | | KFAMEGSILTRSKLI | FAMEGSILT |
| | | FAMEGSILTRSKLIQ | FAMEGSILT |
| | | RKEYEITKNAPSLDS | YEITKNAPS |
| | | KEYEITKNAPSLDSL | YEITKNAPS |
| | | TRSKLIQAFTNTNKF | LIQAFTNTN |
| | | RSKLIQAFTNTNKFC | LIQAFTNTN |
| | | FKGKLVKRMTMIYRS | LVKRMTMIY |
| | | NFKGKLVKRMTMIYR | LVKRMTMIY |
| | | KGKLVKRMTMIYRSA | LVKRMTMIY |
| | | SKLIQAFTNTNKFCI | LIQAFTNTN |
| | | GKLVKRMTMIYRSAD | LVKRMTMIY |
| | | IDNRKEYEITKNAPS | EYEITKNAP |
| | | DNRKEYEITKNAPSL | YEITKNAPS |
| | | NRKEYEITKNAPSLD | YEITKNAPS |
| | | LFFLEKTPVGTLTAK | FFLEKTPVG |
| | | INFKGKLVKRMTMIY | FKGKLVKRM |
| | HLA-DRB1*0404 | LTRSKLIQAFTNTNK | LIQAFTNTN |
| | | TRSKLIQAFTNTNKF | LIQAFTNTN |
| | | RSKLIQAFTNTNKFC | LIQAFTNTN |
| | | ILTRSKLIQAFTNTN | KLIQAFTNT |
| | | SKLIQAFTNTNKFCI | LIQAFTNTN |
| | | KLIQAFTNTNKFCIP | LIQAFTNTN |
| | | IDPEYLLPPFTSQSG | LLPPFTSQS |
| | | PEYLLPPFTSQSGSK | LLPPFTSQS |
| | | DPEYLLPPFTSQSGS | LLPPFTSQS |
| | | LIQAFTNTNKFCIPD | LIQAFTNTN |
| | | EYLLPPFTSQSGSKE | LLPPFTSQS |
| | | EIDPEYLLPPFTSQS | YLLPPFTSQ |
| | | LEHMILFFLEKTPVG | ILFFLEKTP |
| | | EHMILFFLEKTPVGT | FFLEKTPVG |
| | | HMILFFLEKTPVGTL | FFLEKTPVG |
| | | YLLPPFTSQSGSKET | LLPPFTSQS |
| | HLA-DRB1*0405 | YRSADTWYTWYWAEE | YRSADTWYT |
| | | KRMTMIYRSADTWYT | MTMIYRSAD |
| | | ADTWYTWYWAEEIMS | WYTWYWAEE |
| | | RKEYEITKNAPSLDS | YEITKNAPS |
| | | KEYEITKNAPSLDSL | YEITKNAPS |
| | | RSADTWYTWYWAEEI | WYTWYWAEE |
| | | RMTMIYRSADTWYTW | YRSADTWYT |
| | | MTMIYRSADTWYTWY | YRSADTWYT |

|  |  | TMIYRSADTWYTWYW | YRSADTWYT |
|---|---|---|---|
|  |  | DTWYTWYWAEEIMSP | WYWAEEIMS |
|  |  | MIYRSADTWYTWYWA | YRSADTWYT |
|  |  | TRSKLIQAFTNTNKF | IQAFTNTNK |
|  |  | LTRSKLIQAFTNTNK | LIQAFTNTN |
|  |  | RSKLIQAFTNTNKFC | IQAFTNTNK |
|  |  | SKLIQAFTNTNKFCI | IQAFTNTNK |
|  |  | SADTWYTWYWAEEIM | YTWYWAEEI |
|  |  | TWYTWYWAEEIMSPF | YTWYWAEEI |
|  |  | EYEITKNAPSLDSLI | TKNAPSLDS |
|  |  | YEITKNAPSLDSLIE | TKNAPSLDS |
|  |  | KLIQAFTNTNKFCIP | IQAFTNTNK |
|  |  | WYTWYWAEEIMSPFI | WYWAEEIMS |
|  |  | IYRSADTWYTWYWAE | YRSADTWYT |
|  |  | NRKEYEITKNAPSLD | YEITKNAPS |
|  |  | IDNRKEYEITKNAPS | EYEITKNAP |
|  |  | DNRKEYEITKNAPSL | YEITKNAPS |
|  |  | LIQAFTNTNKFCIPD | IQAFTNTNK |
|  |  | GKLVKRMTMIYRSAD | VKRMTMIYR |
|  |  | KLVKRMTMIYRSADT | VKRMTMIYR |
|  |  | YTWYWAEEIMSPFIT | WYWAEEIMS |
|  | HLA-DRB1*0701 | SKLIQAFTNTNKFCI | IQAFTNTNK |
|  |  | KLIQAFTNTNKFCIP | FTNTNKFCI |
|  |  | LIQAFTNTNKFCIPD | FTNTNKFCI |
|  |  | IQAFTNTNKFCIPDG | FTNTNKFCI |
|  |  | QAFTNTNKFCIPDGR | FTNTNKFCI |
|  |  | IKKKLENTKTRIQFR | LENTKTRIQ |
|  |  | KKKLENTKTRIQFRN | LENTKTRIQ |
|  |  | KKLENTKTRIQFRNG | LENTKTRIQ |
|  |  | EIKKKLENTKTRIQF | LENTKTRIQ |
|  |  | TEIKKKLENTKTRIQ | KLENTKTRI |
|  |  | KFAMEGSILTRSKLI | GSILTRSKL |
|  |  | MEGSILTRSKLIQAF | SILTRSKLI |
|  |  | AMEGSILTRSKLIQA | SILTRSKLI |
|  |  | FAMEGSILTRSKLIQ | SILTRSKLI |
|  |  | EGSILTRSKLIQAFT | SILTRSKLI |
|  |  | RMTMIYRSADTWYTW | YRSADTWYT |
|  | HLA-DRB1*0802 | None |  |
|  | HLA-DRB1*0901 | YAYKVLGISSAPKLS | LGISSAPKL |
|  |  | AYKVLGISSAPKLSG | LGISSAPKL |
|  |  | YKVLGISSAPKLSGR | LGISSAPKL |
|  |  | CYAYKVLGISSAPKL | VLGISSAPK |
|  |  | KVLGISSAPKLSGRF | LGISSAPKL |
|  |  | VLGISSAPKLSGRFL | LGISSAPKL |
|  |  | LGISSAPKLSGRFLR | LGISSAPKL |
|  | HLA-DRB1*1101 | None |  |
|  | HLA-DRB1*1302 | NFKGKLVKRMTMIYR | LVKRMTMIY |
|  |  | FKGKLVKRMTMIYRS | VKRMTMIYR |
|  |  | KGKLVKRMTMIYRSA | VKRMTMIYR |
|  |  | GKLVKRMTMIYRSAD | VKRMTMIYR |
|  |  | KLVKRMTMIYRSADT | VKRMTMIYR |
|  |  | INFKGKLVKRMTMIY | GKLVKRMTM |
|  |  | CYAYKVLGISSAPKL | YKVLGISSA |
|  |  | YAYKVLGISSAPKLS | LGISSAPKL |
|  |  | LVKRMTMIYRSADTW | VKRMTMIYR |
|  |  | TRIQFRNGNKIDSLG | IQFRNGNKI |

| | | | |
|---|---|---|---|
| | | SRINFKGKLVKRMTM | INFKGKLVK |
| | | AYKVLGISSAPKLSG | LGISSAPKL |
| | | YKVLGISSAPKLSGR | LGISSAPKL |
| | | IQFRNGNKIDSLGYQ | FRNGNKIDS |
| | | RINFKGKLVKRMTMI | GKLVKRMTM |
| | | VKRMTMIYRSADTWY | VKRMTMIYR |
| | | KTRIQFRNGNKIDSL | IQFRNGNKI |
| | | RIQFRNGNKIDSLGY | IQFRNGNKI |
| | | TKTRIQFRNGNKIDS | IQFRNGNKI |
| | | FSRINFKGKLVKRMT | INFKGKLVK |
| | | SKLIQAFTNTNKFCI | IQAFTNTNK |
| | | KEYEITKNAPSLDSL | ITKNAPSLD |
| | | RKEYEITKNAPSLDS | ITKNAPSLD |
| | | KLIQAFTNTNKFCIP | FTNTNKFCI |
| | | FAMEGSILTRSKLIQ | FAMEGSILT |
| | | EYEITKNAPSLDSLI | ITKNAPSLD |
| | | YEITKNAPSLDSLIE | ITKNAPSLD |
| | | NCYAYKVLGISSAPK | YKVLGISSA |
| | HLA-DRB1*1501 | FKGKLVKRMTMIYRS | VKRMTMIYR |
| | | KLVKRMTMIYRSADT | VKRMTMIYR |
| | | GKLVKRMTMIYRSAD | VKRMTMIYR |
| | | NFKGKLVKRMTMIYR | FKGKLVKRM |
| | | KGKLVKRMTMIYRSA | VKRMTMIYR |
| | | VKRMTMIYRSADTWY | VKRMTMIYR |
| | | LVKRMTMIYRSADTW | VKRMTMIYR |
| | | NCYAYKVLGISSAPK | YKVLGISSA |
| | | YAYKVLGISSAPKLS | VLGISSAPK |
| | | CYAYKVLGISSAPKL | VLGISSAPK |
| | | AYKVLGISSAPKLSG | VLGISSAPK |
| | | LEHMILFFLEKTPVG | LFFLEKTPV |
| | | YKVLGISSAPKLSGR | VLGISSAPK |
| | | EHMILFFLEKTPVGT | LFFLEKTPV |
| | | HMILFFLEKTPVGTL | LFFLEKTPV |
| | | MILFFLEKTPVGTLT | LFFLEKTPV |
| | | DFSRINFKGKLVKRM | INFKGKLVK |
| | | FSRINFKGKLVKRMT | INFKGKLVK |
| | | LDFSRINFKGKLVKR | INFKGKLVK |
| | | YLEHMILFFLEKTPV | ILFFLEKTP |
| | | SRINFKGKLVKRMTM | INFKGKLVK |
| | | LSGRFLRHYDSGGST | LRHYDSGGS |
| | | KLSGRFLRHYDSGGS | FLRHYDSGG |
| | | SGRFLRHYDSGGSTD | LRHYDSGGS |
| | | GRFLRHYDSGGSTDS | LRHYDSGGS |
| | | DLDFSRINFKGKLVK | RINFKGKLV |
| | | ILFFLEKTPVGTLTA | LFFLEKTPV |
| | | RINFKGKLVKRMTMI | INFKGKLVK |
| | | RFLRHYDSGGSTDSD | LRHYDSGGS |
| | | LTRSKLIQAFTNTNK | LIQAFTNTN |
| | HLA-DRB3*0101 | None | |
| | HLA-DRB4*0101 | None | |
| | HLA-DRB5*0101 | FSRINFKGKLVKRMT | FKGKLVKRM |
| | | DFSRINFKGKLVKRM | INFKGKLVK |
| | | SRINFKGKLVKRMTM | FKGKLVKRM |
| | | RINFKGKLVKRMTMI | FKGKLVKRM |
| | | INFKGKLVKRMTMIY | FKGKLVKRM |
| | | FKGKLVKRMTMIYRS | FKGKLVKRM |

| | | | |
|---|---|---|---|
| | | NFKGKLVKRMTMIYR | FKGKLVKRM |
| | | KRMTMIYRSADTWYT | MTMIYRSAD |
| | | AKFAMEGSILTRSKL | FAMEGSILT |
| | | TAKFAMEGSILTRSK | FAMEGSILT |
| | | RMTMIYRSADTWYTW | YRSADTWYT |
| | | MTMIYRSADTWYTWY | YRSADTWYT |
| | | TMIYRSADTWYTWYW | YRSADTWYT |
| | | MIYRSADTWYTWYWA | YRSADTWYT |
| | | LTAKFAMEGSILTRS | FAMEGSILT |
| | | TLTAKFAMEGSILTR | FAMEGSILT |
| | | | |
| NP_212885.1\| hypothetical protein BB0751 [Borrelia burgdorferi B31]<br><br>Seq30 | HLA-DRB1*0101 | KILIIVVLTSTFLLG | IVVLTSTFL |
| | | IKILIIVVLTSTFLL | IVVLTSTFL |
| | | ILIIVVLTSTFLLGI | IVVLTSTFL |
| | | LIIVVLTSTFLLGII | IVVLTSTFL |
| | | KIKILIIVVLTSTFL | IKILIIVVL |
| | | IIVVLTSTFLLGIIF | IVVLTSTFL |
| | | IVVLTSTFLLGIIFS | IVVLTSTFL |
| | | KEKIKILIIVVLTST | IKILIIVVL |
| | | EKIKILIIVVLTSTF | IKILIIVVL |
| | | NKEKIKILIIVVLTS | IKILIIVVL |
| | | INKEKIKILIIVVLT | IKILIIVVL |
| | | SINKEKIKILIIVVL | INKEKIKIL |
| | | KIETELEGTLTKLGK | LEGTLTKLG |
| | | SKIETELEGTLTKLG | ETELEGTLT |
| | | IETELEGTLTKLGKD | LEGTLTKLG |
| | | ETELEGTLTKLGKDW | LEGTLTKLG |
| | | TELEGTLTKLGKDWI | LEGTLTKLG |
| | | NKNKGVFMTKPKIFS | VFMTKPKIF |
| | | NKGVFMTKPKIFSIN | FMTKPKIFS |
| | | KGVFMTKPKIFSINK | FMTKPKIFS |
| | | KNKGVFMTKPKIFSI | FMTKPKIFS |
| | | KDEYYYTTSKWTFFD | YYYTTSKWT |
| | | DKDEYYYTTSKWTFF | YYYTTSKWT |
| | | DEYYYTTSKWTFFDV | YYYTTSKWT |
| | | GVFMTKPKIFSINKE | FMTKPKIFS |
| | | LEGTLTKLGKDWILT | LEGTLTKLG |
| | | ELEGTLTKLGKDWIL | LEGTLTKLG |
| | | KQINKNKGVFMTKPK | INKNKGVFM |
| | | EKQINKNKGVFMTKP | INKNKGVFM |
| | | KEKQINKNKGVFMTK | INKNKGVFM |
| | | LNNQKMTKERPLNII | MTKERPLNI |
| | | EKEKQINKNKGVFMT | INKNKGVFM |
| | HLA-DRB1*0301 | None | |
| | HLA-DRB1*0401 | YNLQYKIEVKWSNKS | YKIEVKWSN |
| | | LQYKIEVKWSNKSVN | IEVKWSNKS |
| | | QYKIEVKWSNKSVNN | IEVKWSNKS |
| | | NLQYKIEVKWSNKSV | IEVKWSNKS |
| | | YKIEVKWSNKSVNNI | IEVKWSNKS |
| | | KIEVKWSNKSVNNIE | IEVKWSNKS |
| | | IEVKWSNKSVNNIEV | IEVKWSNKS |
| | | DKDEYYYTTSKWTFF | YYYTTSKWT |
| | | FLLGIIFSNENKVAR | LGIIFSNEN |
| | | KDEYYYTTSKWTFFD | YYYTTSKWT |
| | | DEYYYTTSKWTFFDV | YYYTTSKWT |

| | | | |
|---|---|---|---|
| | | KDKDEYYYTTSKWTF | YYYTTSKWT |
| | HLA-DRB1*0404 | KILIIVVLTSTFLLG | IIVVLTSTF |
| | | ILIIVVLTSTFLLGI | VLTSTFLLG |
| | | TFLLGIIFSNENKVA | LLGIIFSNE |
| | | STFLLGIIFSNENKV | LLGIIFSNE |
| | | FLLGIIFSNENKVAR | LLGIIFSNE |
| | | TSTFLLGIIFSNENK | LLGIIFSNE |
| | | LTSTFLLGIIFSNEN | LLGIIFSNE |
| | | KIKILIIVVLTSTFL | ILIIVVLTS |
| | | IKILIIVVLTSTFLL | ILIIVVLTS |
| | | LIIVVLTSTFLLGII | VLTSTFLLG |
| | | VLTSTFLLGIIFSNE | VLTSTFLLG |
| | | LLGIIFSNENKVARI | LLGIIFSNE |
| | HLA-DRB1*0405 | KDWILTYNKQNIPVD | WILTYNKQN |
| | | DWILTYNKQNIPVDN | YNKQNIPVD |
| | | WILTYNKQNIPVDNK | YNKQNIPVD |
| | | LQYKIEVKWSNKSVN | IEVKWSNKS |
| | | QYKIEVKWSNKSVNN | IEVKWSNKS |
| | | YKIEVKWSNKSVNNI | IEVKWSNKS |
| | | IEVKWSNKSVNNIEV | IEVKWSNKS |
| | | ILTYNKQNIPVDNKK | YNKQNIPVD |
| | | KIEVKWSNKSVNNIE | IEVKWSNKS |
| | | LTYNKQNIPVDNKKV | YNKQNIPVD |
| | | NLQYKIEVKWSNKSV | IEVKWSNKS |
| | | YNLQYKIEVKWSNKS | YKIEVKWSN |
| | | TKLGKDWILTYNKQN | KDWILTYNK |
| | | KFFDFDFNLISKIET | FDFDFNLIS |
| | | KLGKDWILTYNKQNI | WILTYNKQN |
| | | LGKDWILTYNKQNIP | WILTYNKQN |
| | | IGENPSFKLFDNNNK | ENPSFKLFD |
| | | GKDWILTYNKQNIPV | WILTYNKQN |
| | HLA-DRB1*0701 | LIIVVLTSTFLLGII | IVVLTSTFL |
| | | ILIIVVLTSTFLLGI | IVVLTSTFL |
| | | IKILIIVVLTSTFLL | IVVLTSTFL |
| | | KILIIVVLTSTFLLG | IVVLTSTFL |
| | | DEYYYTTSKWTFFDV | YYTTSKWTF |
| | | DKDEYYYTTSKWTFF | YYTTSKWTF |
| | | KDEYYYTTSKWTFFD | YYTTSKWTF |
| | | KIKILIIVVLTSTFL | IIVVLTSTF |
| | | EYYYTTSKWTFFDVF | YYTTSKWTF |
| | | IIVVLTSTFLLGIIF | IVVLTSTFL |
| | | KDKDEYYYTTSKWTF | YYYTTSKWT |
| | | IVVLTSTFLLGIIFS | IVVLTSTFL |
| | | YYYTTSKWTFFDVFD | YTTSKWTFF |
| | | YYTTSKWTFFDVFDL | YYTTSKWTF |
| | | KNKGVFMTKPKIFSI | VFMTKPKIF |
| | | NKGVFMTKPKIFSIN | VFMTKPKIF |
| | | KGVFMTKPKIFSINK | VFMTKPKIF |
| | | INKNKGVFMTKPKIF | INKNKGVFM |
| | | KKDKDEYYYTTSKWT | EYYYTTSKW |
| | | NKNKGVFMTKPKIFS | VFMTKPKIF |
| | HLA-DRB1*0802 | None | |
| | HLA-DRB1*0901 | DKDEYYYTTSKWTFF | YYYTTSKWT |
| | | KDEYYYTTSKWTFFD | YYYTTSKWT |
| | | DEYYYTTSKWTFFDV | YYYTTSKWT |
| | | KDKDEYYYTTSKWTF | YYYTTSKWT |

| | HLA-DRB1*1101 | DENVYLTKNIFLSYK | LTKNIFLSY |
|---|---|---|---|
| | | SDENVYLTKNIFLSY | NVYLTKNIF |
| | | ENVYLTKNIFLSYKG | LTKNIFLSY |
| | | NVYLTKNIFLSYKGN | LTKNIFLSY |
| | HLA-DRB1*1302 | EKIKILIIVVLTSTF | IKILIIVVL |
| | | KEKIKILIIVVLTST | IKILIIVVL |
| | | SINKEKIKILIIVVL | EKIKILIIV |
| | | INKEKIKILIIVVLT | IKILIIVVL |
| | | NKEKIKILIIVVLTS | IKILIIVVL |
| | | IKILIIVVLTSTFLL | IKILIIVVL |
| | | KIKILIIVVLTSTFL | IKILIIVVL |
| | | DGLYFLNNQKMTKER | LNNQKMTKE |
| | | KILIIVVLTSTFLLG | LIIVVLTST |
| | | SFKLFDNNNKLLTEI | FDNNNKLLT |
| | | FKLFDNNNKLLTEIF | FDNNNKLLT |
| | | KDGLYFLNNQKMTKE | YFLNNQKMT |
| | | PSFKLFDNNNKLLTE | FDNNNKLLT |
| | | NPSFKLFDNNNKLLT | LFDNNNKLL |
| | | KLFDNNNKLLTEIFV | FDNNNKLLT |
| | | GLYFLNNQKMTKERP | LNNQKMTKE |
| | | EKEKQINKNKGVFMT | INKNKGVFM |
| | | KEKQINKNKGVFMTK | INKNKGVFM |
| | | KQINKNKGVFMTKPK | INKNKGVFM |
| | | EKQINKNKGVFMTKP | INKNKGVFM |
| | | LYFLNNQKMTKERPL | LNNQKMTKE |
| | | ILIIVVLTSTFLLGI | IIVVLTSTF |
| | | LIIVVLTSTFLLGII | VVLTSTFLL |
| | | YFLNNQKMTKERPLN | LNNQKMTKE |
| | | YKIEVKWSNKSVNNI | VKWSNKSVN |
| | | QINKNKGVFMTKPKI | INKNKGVFM |
| | | LEKEKQINKNKGVFM | QINKNKGVF |
| | | IEVKWSNKSVNNIEV | WSNKSVNNI |
| | | LFDNNNKLLTEIFVG | FDNNNKLLT |
| | | KIEVKWSNKSVNNIE | WSNKSVNNI |
| | | FDFDFNLISKIETEL | FNLISKIET |
| | | KFFDFDFNLISKIET | FDFNLISKI |
| | | FFDFDFNLISKIETE | FNLISKIET |
| | | FDNNNKLLTEIFVGK | FDNNNKLLT |
| | | KENENNINNNYEIIS | NENNINNNY |
| | | DFDFNLISKIETELE | FNLISKIET |
| | | FDFNLISKIETELEG | FNLISKIET |
| | | SKDGLYFLNNQKMTK | YFLNNQKMT |
| | | VKWSNKSVNNIEVYF | WSNKSVNNI |
| | | IIVVLTSTFLLGIIF | VVLTSTFLL |
| | | EVKWSNKSVNNIEVY | WSNKSVNNI |
| | | INKNKGVFMTKPKIF | INKNKGVFM |
| | | ENENNINNNYEIISK | INNNYEIIS |
| | | NENNINNNYEIISKD | INNNYEIIS |
| | | NNINNNYEIISKDGL | INNNYEIIS |
| | | ENNINNNYEIISKDG | INNNYEIIS |
| | | LENLSFKIIRKLNKE | FKIIRKLNK |
| | | KLENLSFKIIRKLNK | LENLSFKII |
| | | SDENVYLTKNIFLSY | YLTKNIFLS |
| | | FLNNQKMTKERPLNI | LNNQKMTKE |
| | | DENVYLTKNIFLSYK | YLTKNIFLS |
| | | EKNTKLENLSFKIIR | LENLSFKII |
| | | QEKNTKLENLSFKII | KNTKLENLS |

| | | | |
|---|---|---|---|
| | | ENVYLTKNIFLSYKG | YLTKNIFLS |
| | | KNTKLENLSFKIIRK | LENLSFKII |
| | | NTKLENLSFKIIRKL | LENLSFKII |
| | | KWSNKSVNNIEVYFN | WSNKSVNNI |
| | | TKLENLSFKIIRKLN | LENLSFKII |
| | | PVDNKKVNSLIKALD | KKVNSLIKA |
| | | NVYLTKNIFLSYKGN | YLTKNIFLS |
| | | WSNKSVNNIEVYFNK | WSNKSVNNI |
| | | VDNKKVNSLIKALDE | VNSLIKALD |
| | | INNNYEIISKDGLYF | INNNYEIIS |
| | | DWILTYNKQNIPVDN | LTYNKQNIP |
| | | ISKDGLYFLNNQKMT | LYFLNNQKM |
| | | KDWILTYNKQNIPVD | LTYNKQNIP |
| | | IVVLTSTFLLGIIFS | VVLTSTFLL |
| | | NINNNYEIISKDGLY | INNNYEIIS |
| | | FNLISKIETELEGTL | FNLISKIET |
| | | DFNLISKIETELEGT | FNLISKIET |
| | | LQYKIEVKWSNKSVN | IEVKWSNKS |
| | | GSDENVYLTKNIFLS | VYLTKNIFL |
| | | WILTYNKQNIPVDNK | LTYNKQNIP |
| | | GKDWILTYNKQNIPV | LTYNKQNIP |
| | | VYLTKNIFLSYKGNS | YLTKNIFLS |
| | | DNKKVNSLIKALDEL | VNSLIKALD |
| | | KALDELQKNKLVSRD | LQKNKLVSR |
| | | QYKIEVKWSNKSVNN | VKWSNKSVN |
| | | LGKDWILTYNKQNIP | ILTYNKQNI |
| | | FLLGIIFSNENKVAR | LGIIFSNEN |
| | | IKALDELQKNKLVSR | LDELQKNKL |
| | | LGIIFSNENKVARIL | FSNENKVAR |
| | | LLGIIFSNENKVARI | FSNENKVAR |
| | | YLTKNIFLSYKGNSY | YLTKNIFLS |
| | | LDELQKNKLVSRDQK | LQKNKLVSR |
| | | IPVDNKKVNSLIKAL | VDNKKVNSL |
| | | VVLTSTFLLGIIFSN | VVLTSTFLL |
| | | NIPVDNKKVNSLIKA | VDNKKVNSL |
| | | ALDELQKNKLVSRDQ | LQKNKLVSR |
| | | NKKVNSLIKALDELQ | VNSLIKALD |
| | | LNNQKMTKERPLNII | LNNQKMTKE |
| | HLA-DRB1*1501 | KILIIVVLTSTFLLG | IVVLTSTFL |
| | | KIKILIIVVLTSTFL | ILIIVVLTS |
| | | IKILIIVVLTSTFLL | IVVLTSTFL |
| | | ILIIVVLTSTFLLGI | IVVLTSTFL |
| | | KLENLSFKIIRKLNK | NLSFKIIRK |
| | | LENLSFKIIRKLNKE | FKIIRKLNK |
| | | YLTKNIFLSYKGNSY | TKNIFLSYK |
| | | LIIVVLTSTFLLGII | IVVLTSTFL |
| | | ENLSFKIIRKLNKEN | FKIIRKLNK |
| | HLA-DRB3*0101 | None | |
| | HLA-DRB4*0101 | YKIEVKWSNKSVNNI | IEVKWSNKS |
| | HLA-DRB5*0101 | FLLGIIFSNENKVAR | LGIIFSNEN |
| | | LLGIIFSNENKVARI | FSNENKVAR |
| | | LGIIFSNENKVARIL | FSNENKVAR |
| | | GIIFSNENKVARILE | FSNENKVAR |
| | | IIFSNENKVARILEE | FSNENKVAR |
| | | LQYKIEVKWSNKSVN | YKIEVKWSN |
| | | YNLQYKIEVKWSNKS | YKIEVKWSN |

| | | | |
|---|---|---|---|
| | | NLQYKIEVKWSNKSV | YKIEVKWSN |
| | | DKDEYYYTTSKWTFF | YYYTTSKWT |
| | | KDEYYYTTSKWTFFD | YYYTTSKWT |
| | | DEYYYTTSKWTFFDV | YYYTTSKWT |
| | | KDGLYFLNNQKMTKE | LYFLNNQKM |
| | | DGLYFLNNQKMTKER | LYFLNNQKM |
| | | KDKDEYYYTTSKWTF | YYYTTSKWT |
| | | DYNLQYKIEVKWSNK | YKIEVKWSN |
| | | SKDGLYFLNNQKMTK | LYFLNNQKM |
| | | DDYNLQYKIEVKWSN | YNLQYKIEV |
| | | FSNENKVARILEEKF | FSNENKVAR |
| | | IFSNENKVARILEEK | FSNENKVAR |
| | | EYYYTTSKWTFFDVF | YYYTTSKWT |
| | | YKIEVKWSNKSVNNI | YKIEVKWSN |
| | | QYKIEVKWSNKSVNN | YKIEVKWSN |
| | | GLYFLNNQKMTKERP | LYFLNNQKM |
| | | ENLSFKIIRKLNKEN | FKIIRKLNK |
| | | NLSFKIIRKLNKENE | FKIIRKLNK |
| | | LSFKIIRKLNKENEN | FKIIRKLNK |
| | | LYFLNNQKMTKERPL | LYFLNNQKM |
| | | KKDKDEYYYTTSKWT | DEYYYTTSK |
| | | KLENLSFKIIRKLNK | LSFKIIRKL |
| | | LENLSFKIIRKLNKE | FKIIRKLNK |
| | | | |
| AAC67038.1| predicted coding region BB0689 [Borrelia burgdorferi B31]  Seq31 | HLA-DRB1*0101 | THTLFGTTPMQRIHK | FGTTPMQRI |
| | | HTLFGTTPMQRIHKY | FGTTPMQRI |
| | | TITHTLFGTTPMQRI | LFGTTPMQR |
| | | ITHTLFGTTPMQRIH | FGTTPMQRI |
| | | TLFGTTPMQRIHKYD | FGTTPMQRI |
| | | KEDMKILYSEIAELR | MKILYSEIA |
| | | EDMKILYSEIAELRK | MKILYSEIA |
| | | IDTKEDMKILYSEIA | IDTKEDMKI |
| | | TKEDMKILYSEIAEL | MKILYSEIA |
| | | DTKEDMKILYSEIAE | MKILYSEIA |
| | | IIFTLFLSQACNLST | FLSQACNLS |
| | | IFTLFLSQACNLSTM | LSQACNLST |
| | | IIIFTLFLSQACNLS | IFTLFLSQA |
| | | KKLIIIFTLFLSQAC | IFTLFLSQA |
| | | LFGTTPMQRIHKYDQ | FGTTPMQRI |
| | | TLFLSQACNLSTMHK | LSQACNLST |
| | | MKKLIIIFTLFLSQA | LIIIFTLFL |
| | | FTLFLSQACNLSTMH | LSQACNLST |
| | | DMKILYSEIAELRKK | MKILYSEIA |
| | | MKILYSEIAELRKKL | MKILYSEIA |
| | | LIIIFTLFLSQACNL | IFTLFLSQA |
| | | KLIIIFTLFLSQACN | IFTLFLSQA |
| | | NRVVNAWLNSPSHKE | VVNAWLNSP |
| | | FGTTPMQRIHKYDQS | FGTTPMQRI |
| | | RTITHTLFGTTPMQR | THTLFGTTP |
| | | LFLSQACNLSTMHKI | LSQACNLST |
| | | RVVNAWLNSPSHKEA | WLNSPSHKE |
| | | VVNAWLNSPSHKEAL | WLNSPSHKE |
| | | VNAWLNSPSHKEALI | WLNSPSHKE |
| | | TDKIGGYRLKTTDNI | IGGYRLKTT |
| | | DKIGGYRLKTTDNID | IGGYRLKTT |
| | | VAKEYAIKLGENRTI | VAKEYAIKL |
| | | NAWLNSPSHKEALIN | WLNSPSHKE |

|  |  | FLSQACNLSTMHKID | LSQACNLST |
|---|---|---|---|
|  |  | TDTDKIGGYRLKTTD | IGGYRLKTT |
|  |  | DTDKIGGYRLKTTDN | IGGYRLKTT |
|  |  | NTDTDKIGGYRLKTT | TDKIGGYRL |
|  |  | IELNRVVNAWLNSPS | VVNAWLNSP |
|  |  | LSQACNLSTMHKIDT | LSQACNLST |
|  |  | KIGGYRLKTTDNIDI | IGGYRLKTT |
|  |  | FNLTREILASGIELN | LTREILASG |
|  |  | IGGYRLKTTDNIDIF | IGGYRLKTT |
|  |  | LNRVVNAWLNSPSHK | VVNAWLNSP |
|  |  | ELNRVVNAWLNSPSH | VVNAWLNSP |
|  |  | EYAIKLGENRTITHT | IKLGENRTI |
|  |  | DQSFNLTREILASGI | LTREILASG |
|  |  | KEYAIKLGENRTITH | IKLGENRTI |
|  |  | QSFNLTREILASGIE | LTREILASG |
|  |  | EKVAKEYAIKLGENR | VAKEYAIKL |
|  |  | YAIKLGENRTITHTL | IKLGENRTI |
|  |  | GIELNRVVNAWLNSP | IELNRVVNA |
|  |  | LTREILASGIELNRV | ILASGIELN |
|  |  | SFNLTREILASGIEL | LTREILASG |
|  |  | DTLEKVAKEYAIKLG | VAKEYAIKL |
|  |  | LEKVAKEYAIKLGEN | VAKEYAIKL |
|  | HLA-DRB1*0301 | None |  |
|  | HLA-DRB1*0401 | KEDMKILYSEIAELR | MKILYSEIA |
|  |  | EDMKILYSEIAELRK | MKILYSEIA |
|  |  | IDTKEDMKILYSEIA | TKEDMKILY |
|  |  | DTKEDMKILYSEIAE | MKILYSEIA |
|  |  | TKEDMKILYSEIAEL | MKILYSEIA |
|  |  | DMKILYSEIAELRKK | MKILYSEIA |
|  |  | MKILYSEIAELRKKL | MKILYSEIA |
|  |  | IIIFTLFLSQACNLS | IIFTLFLSQ |
|  |  | IIFTLFLSQACNLST | FLSQACNLS |
|  |  | IFTLFLSQACNLSTM | FLSQACNLS |
|  |  | FTLFLSQACNLSTMH | FLSQACNLS |
|  |  | TLFLSQACNLSTMHK | FLSQACNLS |
|  | HLA-DRB1*0404 | NRVVNAWLNSPSHKE | VVNAWLNSP |
|  |  | ELNRVVNAWLNSPSH | VVNAWLNSP |
|  |  | LNRVVNAWLNSPSHK | VVNAWLNSP |
|  |  | GIELNRVVNAWLNSP | ELNRVVNAW |
|  |  | IELNRVVNAWLNSPS | VVNAWLNSP |
|  |  | RVVNAWLNSPSHKEA | VVNAWLNSP |
|  |  | VVNAWLNSPSHKEAL | VVNAWLNSP |
|  |  | IIIFTLFLSQACNLS | IFTLFLSQA |
|  |  | IIFTLFLSQACNLST | FLSQACNLS |
|  |  | RTITHTLFGTTPMQR | ITHTLFGTT |
|  |  | TLFLSQACNLSTMHK | FLSQACNLS |
|  |  | MKKLIIIFTLFLSQA | LIIIFTLFL |
|  |  | KKLIIIFTLFLSQAC | LIIIFTLFL |
|  |  | IFTLFLSQACNLSTM | FLSQACNLS |
|  |  | HKEALINTDTDKIGG | LINTDTDKI |
|  |  | FTLFLSQACNLSTMH | FLSQACNLS |
|  |  | SHKEALINTDTDKIG | LINTDTDKI |
|  |  | NRTITHTLFGTTPMQ | ITHTLFGTT |
|  |  | TITHTLFGTTPMQRI | ITHTLFGTT |
|  |  | PSHKEALINTDTDKI | EALINTDTD |

|  |  | KEALINTDTDKIGGY | LINTDTDKI |
|---|---|---|---|
|  |  | EALINTDTDKIGGYR | LINTDTDKI |
|  |  | GENRTITHTLFGTTP | ITHTLFGTT |
|  | HLA-DRB1*0405 | IELNRVVNAWLNSPS | NRVVNAWLN |
|  |  | ELNRVVNAWLNSPSH | VNAWLNSPS |
|  |  | NRVVNAWLNSPSHKE | VNAWLNSPS |
|  |  | LNRVVNAWLNSPSHK | VNAWLNSPS |
|  |  | RVVNAWLNSPSHKEA | VNAWLNSPS |
|  |  | VVNAWLNSPSHKEAL | VNAWLNSPS |
|  |  | VNAWLNSPSHKEALI | VNAWLNSPS |
|  |  | IIIFTLFLSQACNLS | FTLFLSQAC |
|  |  | KKLIIIFTLFLSQAC | IIIFTLFLS |
|  |  | IIFTLFLSQACNLST | FLSQACNLS |
|  |  | GIELNRVVNAWLNSP | NRVVNAWLN |
|  |  | KLIIIFTLFLSQACN | IIIFTLFLS |
|  |  | HKYDQSFNLTREILA | YDQSFNLTR |
|  |  | FTLFLSQACNLSTMH | FLSQACNLS |
|  |  | IFTLFLSQACNLSTM | FLSQACNLS |
|  | HLA-DRB1*0701 | None |  |
|  | HLA-DRB1*0802 | None |  |
|  | HLA-DRB1*0901 | VNAWLNSPSHKEALI | WLNSPSHKE |
|  |  | VVNAWLNSPSHKEAL | WLNSPSHKE |
|  |  | NAWLNSPSHKEALIN | WLNSPSHKE |
|  |  | RVVNAWLNSPSHKEA | WLNSPSHKE |
|  |  | NRVVNAWLNSPSHKE | VNAWLNSPS |
|  | HLA-DRB1*1101 | None |  |
|  | HLA-DRB1*1302 | MKKLIIIFTLFLSQA | IIIFTLFLS |
|  |  | KKLIIIFTLFLSQAC | IIIFTLFLS |
|  |  | IELNRVVNAWLNSPS | VVNAWLNSP |
|  |  | KLIIIFTLFLSQACN | IIIFTLFLS |
|  |  | ELNRVVNAWLNSPSH | VVNAWLNSP |
|  |  | LNRVVNAWLNSPSHK | VVNAWLNSP |
|  |  | NRVVNAWLNSPSHKE | VVNAWLNSP |
|  |  | GIELNRVVNAWLNSP | LNRVVNAWL |
|  |  | IIIFTLFLSQACNLS | IIIFTLFLS |
|  |  | RVVNAWLNSPSHKEA | VNAWLNSPS |
|  |  | LIIIFTLFLSQACNL | IIIFTLFLS |
|  |  | DNIDIFVVLFGKRKY | IFVVLFGKR |
|  | HLA-DRB1*1501 | NIDIFVVLFGKRKYK | IFVVLFGKR |
|  |  | DNIDIFVVLFGKRKY | IFVVLFGKR |
|  |  | IDIFVVLFGKRKYKN | IFVVLFGKR |
|  |  | LNRVVNAWLNSPSHK | VNAWLNSPS |
|  |  | ELNRVVNAWLNSPSH | VNAWLNSPS |
|  |  | NRVVNAWLNSPSHKE | VNAWLNSPS |
|  |  | TDNIDIFVVLFGKRK | IFVVLFGKR |
|  |  | RVVNAWLNSPSHKEA | VNAWLNSPS |
|  |  | IELNRVVNAWLNSPS | LNRVVNAWL |
|  |  | KKLIIIFTLFLSQAC | IIIFTLFLS |
|  |  | KLIIIFTLFLSQACN | IIIFTLFLS |
|  |  | MKKLIIIFTLFLSQA | IIIFTLFLS |
|  |  | SEIAELRKKLNLNHL | LRKKLNLNH |
|  |  | YSEIAELRKKLNLNH | ELRKKLNLN |
|  |  | EIAELRKKLNLNHLE | LRKKLNLNH |
|  |  | IAELRKKLNLNHLEI | LRKKLNLNH |
|  |  | AELRKKLNLNHLEID | LRKKLNLNH |
|  |  | TTDNIDIFVVLFGKR | DIFVVLFGK |

| | | VVNAWLNSPSHKEAL | VNAWLNSPS |
|---|---|---|---|
| | | RTITHTLFGTTPMQR | ITHTLFGTT |
| | | LIIIFTLFLSQACNL | IIIFTLFLS |
| | | TITHTLFGTTPMQRI | LFGTTPMQR |
| | HLA-DRB3*0101 | None | |
| | HLA-DRB4*0101 | None | |
| | HLA-DRB5*0101 | HTLFGTTPMQRIHKY | LFGTTPMQR |
| | | THTLFGTTPMQRIHK | LFGTTPMQR |
| | | | |
| NP_045709.1\| lipoprotein BBA36[Borrelia burgdorferi B31]<br><br>Seq32 | HLA-DRB1*0101 | SLTPEEAEKLESLKV | LTPEEAEKL |
| | | MQRISILLMLLAVFS | MQRISILLM |
| | | ISILLMLLAVFSCKQ | LLMLLAVFS |
| | | QRISILLMLLAVFSC | LLMLLAVFS |
| | | RISILLMLLAVFSCK | LLMLLAVFS |
| | | SILLMLLAVFSCKQF | LLMLLAVFS |
| | | ILLMLLAVFSCKQFG | LLMLLAVFS |
| | | LLMLLAVFSCKQFGD | LLMLLAVFS |
| | | ISSFDNISSIVSKAV | FDNISSIVS |
| | | SSFDNISSIVSKAVD | FDNISSIVS |
| | | KEFISSFDNISSIVS | FISSFDNIS |
| | | FISSFDNISSIVSKA | FDNISSIVS |
| | | SLKVFLKDAMSVNGR | LKDAMSVNG |
| | | LKVFLKDAMSVNGRE | LKDAMSVNG |
| | | EFISSFDNISSIVSK | FDNISSIVS |
| | | ESLKVFLKDAMSVNG | VFLKDAMSV |
| | | KVFLKDAMSVNGREE | LKDAMSVNG |
| | | FDNISSIVSKAVDAS | ISSIVSKAV |
| | | VFLKDAMSVNGREEA | LKDAMSVNG |
| | | SFDNISSIVSKAVDA | ISSIVSKAV |
| | | MMQRISILLMLLAVF | MMQRISILL |
| | | DNISSIVSKAVDASK | ISSIVSKAV |
| | | LESLKVFLKDAMSVN | VFLKDAMSV |
| | | KLESLKVFLKDAMSV | SLKVFLKDA |
| | | DVVKDLIPKEISLTP | VKDLIPKEI |
| | | IPKEISLTPEEAEKL | IPKEISLTP |
| | | SDVVKDLIPKEISLT | VKDLIPKEI |
| | | PKEISLTPEEAEKLE | LTPEEAEKL |
| | | EISLTPEEAEKLESL | LTPEEAEKL |
| | | FLKDAMSVNGREEAL | LKDAMSVNG |
| | | KEISLTPEEAEKLES | LTPEEAEKL |
| | | ISLTPEEAEKLESLK | LTPEEAEKL |
| | | VVKDLIPKEISLTPE | LIPKEISLT |
| | | VKDLIPKEISLTPEE | LIPKEISLT |
| | | ISSIVSKAVDASKKR | ISSIVSKAV |
| | | NISSIVSKAVDASKK | ISSIVSKAV |
| | | EEIFKVIKKVLTESE | FKVIKKVLT |
| | | EIFKVIKKVLTESES | FKVIKKVLT |
| | | LKDAMSVNGREEALK | LKDAMSVNG |
| | | VCYKIKNSKGEALSL | IKNSKGEAL |
| | | KDLIPKEISLTPEEA | LIPKEISLT |
| | | YKIKNSKGEALSLFF | IKNSKGEAL |
| | | CYKIKNSKGEALSLF | IKNSKGEAL |
| | | NEEIFKVIKKVLTES | FKVIKKVLT |
| | | YVCYKIKNSKGEALS | IKNSKGEAL |
| | | EYVCYKIKNSKGEAL | YKIKNSKGE |
| | | IDSGNGIPLVVSDVV | GNGIPLVVS |

| | | | |
|---|---|---|---|
| | | LTEIDSGNGIPLVVS | IDSGNGIPL |
| | | TEIDSGNGIPLVVSD | GNGIPLVVS |
| | | VSDVVKDLIPKEISL | VKDLIPKEI |
| | | VVSDVVKDLIPKEIS | VKDLIPKEI |
| | | CNEEIFKVIKKVLTE | FKVIKKVLT |
| | | KCNEEIFKVIKKVLT | IFKVIKKVL |
| | | LVVSDVVKDLIPKEI | LVVSDVVKD |
| | | LTPEEAEKLESLKVF | LTPEEAEKL |
| | | YKEFFDWLSKDVNRQ | FFDWLSKDV |
| | | IFKVIKKVLTESESN | FKVIKKVLT |
| | | FKVIKKVLTESESNN | FKVIKKVLT |
| | | KEFFDWLSKDVNRQK | WLSKDVNRQ |
| | | DSGNGIPLVVSDVVK | GNGIPLVVS |
| | | DLIPKEISLTPEEAE | LIPKEISLT |
| | | EIDSGNGIPLVVSDV | GNGIPLVVS |
| | | ESNNELKNLKNYGNV | LKNLKNYGN |
| | HLA-DRB1*0301 | None | |
| | HLA-DRB1*0401 | KEFISSFDNISSIVS | FISSFDNIS |
| | | EIFKVIKKVLTESES | FKVIKKVLT |
| | | EEIFKVIKKVLTESE | FKVIKKVLT |
| | | NEEIFKVIKKVLTES | FKVIKKVLT |
| | | NRQKEFISSFDNISS | FISSFDNIS |
| | | RQKEFISSFDNISSI | FISSFDNIS |
| | | QKEFISSFDNISSIV | FISSFDNIS |
| | HLA-DRB1*0404 | ISILLMLLAVFSCKQ | MLLAVFSCK |
| | | SLFFQKVVDAFGADP | FQKVVDAFG |
| | | LFFQKVVDAFGADPY | VVDAFGADP |
| | | FQKVVDAFGADPYKK | VVDAFGADP |
| | | FFQKVVDAFGADPYK | VVDAFGADP |
| | | SILLMLLAVFSCKQF | LLAVFSCKQ |
| | | ILLMLLAVFSCKQFG | LLAVFSCKQ |
| | | QKVVDAFGADPYKKD | VVDAFGADP |
| | | ESLKVFLKDAMSVNG | FLKDAMSVN |
| | | SLKVFLKDAMSVNGR | LKDAMSVNG |
| | | LKVFLKDAMSVNGRE | LKDAMSVNG |
| | HLA-DRB1*0405 | KSYKEFFDWLSKDVN | YKEFFDWLS |
| | | EKSYKEFFDWLSKDV | YKEFFDWLS |
| | | YEKSYKEFFDWLSKD | YKEFFDWLS |
| | | YKEFFDWLSKDVNRQ | YKEFFDWLS |
| | | EYEKSYKEFFDWLSK | YKEFFDWLS |
| | | AEYEKSYKEFFDWLS | YEKSYKEFF |
| | | SYKEFFDWLSKDVNR | YKEFFDWLS |
| | | KEFISSFDNISSIVS | ISSFDNISS |
| | | EFISSFDNISSIVSK | FDNISSIVS |
| | | NRQKEFISSFDNISS | FISSFDNIS |
| | | RQKEFISSFDNISSI | ISSFDNISS |
| | | QKEFISSFDNISSIV | ISSFDNISS |
| | | FISSFDNISSIVSKA | FDNISSIVS |
| | | KEFFDWLSKDVNRQK | FDWLSKDVN |
| | | EFFDWLSKDVNRQKE | FDWLSKDVN |
| | | ISSFDNISSIVSKAV | FDNISSIVS |
| | HLA-DRB1*0701 | ISSFDNISSIVSKAV | SFDNISSIV |
| | | SSFDNISSIVSKAVD | ISSIVSKAV |
| | | SFDNISSIVSKAVDA | ISSIVSKAV |
| | | FDNISSIVSKAVDAS | ISSIVSKAV |

| | | DNISSIVSKAVDASK | ISSIVSKAV |
|---|---|---|---|
| | HLA-DRB1*0802 | None | |
| | HLA-DRB1*0901 | None | |
| | HLA-DRB1*1101 | None | |
| | HLA-DRB1*1302 | NEEIFKVIKKVLTES | FKVIKKVLT |
| | | EEIFKVIKKVLTESE | FKVIKKVLT |
| | | KCNEEIFKVIKKVLT | IFKVIKKVL |
| | | CNEEIFKVIKKVLTE | FKVIKKVLT |
| | | EIFKVIKKVLTESES | FKVIKKVLT |
| | | IFKVIKKVLTESESN | FKVIKKVLT |
| | | FKVIKKVLTESESNN | FKVIKKVLT |
| | | MQRISILLMLLAVFS | MQRISILLM |
| | | MMQRISILLMLLAVF | MQRISILLM |
| | | SESNNELKNLKNYGN | SNNELKNLK |
| | | QRISILLMLLAVFSC | ISILLMLLA |
| | | ESNNELKNLKNYGNV | LKNLKNYGN |
| | | RISILLMLLAVFSCK | ISILLMLLA |
| | | ISILLMLLAVFSCKQ | ISILLMLLA |
| | | ESLKVFLKDAMSVNG | LKVFLKDAM |
| | | LESLKVFLKDAMSVN | LKVFLKDAM |
| | | EYVCYKIKNSKGEAL | YKIKNSKGE |
| | | ISSFDNISSIVSKAV | FDNISSIVS |
| | | SSFDNISSIVSKAVD | FDNISSIVS |
| | | AEKLESLKVFLKDAM | LESLKVFLK |
| | | EFISSFDNISSIVSK | FDNISSIVS |
| | | YVCYKIKNSKGEALS | IKNSKGEAL |
| | | FISSFDNISSIVSKA | FDNISSIVS |
| | | VCYKIKNSKGEALSL | IKNSKGEAL |
| | | EKLESLKVFLKDAMS | LKVFLKDAM |
| | | LVVSDVVKDLIPKEI | VSDVVKDLI |
| | | NGIPLVVSDVVKDLI | LVVSDVVKD |
| | | KEFISSFDNISSIVS | SFDNISSIV |
| | HLA-DRB1*1501 | MQRISILLMLLAVFS | ILLMLLAVF |
| | | RISILLMLLAVFSCK | ILLMLLAVF |
| | | MMQRISILLMLLAVF | MMQRISILL |
| | | ISILLMLLAVFSCKQ | ILLMLLAVF |
| | | QRISILLMLLAVFSC | ILLMLLAVF |
| | | SILLMLLAVFSCKQF | LLMLLAVFS |
| | HLA-DRB3*0101 | None | |
| | HLA-DRB4*0101 | RISILLMLLAVFSCK | ILLMLLAVF |
| | | ISILLMLLAVFSCKQ | MLLAVFSCK |
| | | SILLMLLAVFSCKQF | MLLAVFSCK |
| | | ILLMLLAVFSCKQFG | MLLAVFSCK |
| | | YKEFFDWLSKDVNRQ | FDWLSKDVN |
| | | KEFFDWLSKDVNRQK | FDWLSKDVN |
| | HLA-DRB5*0101 | None | |
| | | | |
| AAT93789.1\| lipoprotein BBA36 (homolog 67%)[Borrelia garinii PBi]<br><br>Seq33 | HLA-DRB1*0101 | VSFFNNICGIITKAV | FNNICGIIT |
| | | SFFNNICGIITKAVD | ICGIITKAV |
| | | NNELANLKNLNSYNL | LANLKNLNS |
| | | NELANLKNLNSYNLN | LKNLNSYNL |
| | | LANLKNLNSYNLNSN | LKNLNSYNL |
| | | ELANLKNLNSYNLNS | LKNLNSYNL |
| | | ANLKNLNSYNLNSNN | LKNLNSYNL |
| | | FNNICGIITKAVDAS | ICGIITKAV |
| | | FFNNICGIITKAVDA | ICGIITKAV |

| | | | |
|---|---|---|---|
| | | SEEFERLEALKTFLK | FERLEALKT |
| | | EEFERLEALKTFLKD | FERLEALKT |
| | | NNICGIITKAVDASK | ICGIITKAV |
| | | TSEEFERLEALKTFL | FERLEALKT |
| | | VLTSEEFERLEALKT | VLTSEEFER |
| | | LTSEEFERLEALKTF | FERLEALKT |
| | | KDQIIEKGPVLTSEE | IIEKGPVLT |
| | | DQIIEKGPVLTSEEF | IIEKGPVLT |
| | | MKRISILSILLLLLL | ILSILLLLL |
| | | NLKNLNSYNLNSNNK | LKNLNSYNL |
| | | SKDQIIEKGPVLTSE | IIEKGPVLT |
| | | ESKDQIIEKGPVLTS | IIEKGPVLT |
| | | KRISILSILLLLLLF | ILSILLLLL |
| | | RISILSILLLLLLFS | ILSILLLLL |
| | | ISILSILLLLLLFSC | ILSILLLLL |
| | | FVSFFNNICGIITKA | FNNICGIIT |
| | | EFVSFFNNICGIITK | FNNICGIIT |
| | | YGDVKSLTEVATDLE | VKSLTEVAT |
| | | QYGDVKSLTEVATDL | VKSLTEVAT |
| | | KEFVSFFNNICGIIT | VSFFNNICG |
| | | GDVKSLTEVATDLED | VKSLTEVAT |
| | | KQYGDVKSLTEVATD | VKSLTEVAT |
| | | CKQYGDVKSLTEVAT | YGDVKSLTE |
| | | EFERLEALKTFLKDA | FERLEALKT |
| | | FERLEALKTFLKDAM | FERLEALKT |
| | | SILSILLLLLLFSCK | LSILLLLLL |
| | | NICGIITKAVDASKK | ICGIITKAV |
| | | ICGIITKAVDASKKR | ICGIITKAV |
| | | VESKDQIIEKGPVLT | DQIIEKGPV |
| | | QIIEKGPVLTSEEFE | IIEKGPVLT |
| | | ILSILLLLLLFSCKQ | ILLLLLLFS |
| | | LSILLLLLLFSCKQY | ILLLLLLFS |
| | | IIEKGPVLTSEEFER | IIEKGPVLT |
| | | SLFFQKVADAFGTQE | FQKVADAFG |
| | | DLSLFFQKVADAFGT | FQKVADAFG |
| | | LSLFFQKVADAFGTQ | FQKVADAFG |
| | | DVKSLTEVATDLEDD | VKSLTEVAT |
| | | VKSLTEVATDLEDDN | VKSLTEVAT |
| | | ALKTFLKDAMGVNGR | LKDAMGVNG |
| | | LKTFLKDAMGVNGRE | LKDAMGVNG |
| | | LFFQKVADAFGTQEY | FQKVADAFG |
| | | EALKTFLKDAMGVNG | TFLKDAMGV |
| | | KTFLKDAMGVNGREG | LKDAMGVNG |
| | | TFLKDAMGVNGREGD | LKDAMGVNG |
| | | YKEFFDWLSKDVNRQ | FFDWLSKDV |
| | | DDLSLFFQKVADAFG | FFQKVADAF |
| | | LEDDNSFASGSVESK | SFASGSVES |
| | | EDDNSFASGSVESKD | FASGSVESK |
| | | KEFFDWLSKDVNRQK | WLSKDVNRQ |
| | | DDNSFASGSVESKDQ | FASGSVESK |
| | | DNSFASGSVESKDQI | FASGSVESK |
| | | SENNNELANLKNLNS | NNNELANLK |
| | HLA-DRB1*0301 | LGFNEYVCYDIKTRT | LGFNEYVCY |
| | | EYVCYDIKTRTGDDL | VCYDIKTRT |
| | | FNEYVCYDIKTRTGD | VCYDIKTRT |
| | | NEYVCYDIKTRTGDD | VCYDIKTRT |

| | | GFNEYVCYDIKTRTG | VCYDIKTRT |
|---|---|---|---|
| | HLA-DRB1*0401 | None | |
| | HLA-DRB1*0404 | ILSILLLLLLFSCKQ | ILLLLLLFS |
| | | SILLLLLLFSCKQYG | LLLLFSCKQ |
| | | ILLLLLLFSCKQYGD | LLLLFSCKQ |
| | | LSILLLLLLFSCKQY | LLLLFSCKQ |
| | | LLLLLLLFSCKQYGDV | LLLLFSCKQ |
| | HLA-DRB1*0405 | KSYKEFFDWLSKDVN | YKEFFDWLS |
| | | NRQKEFVSFFNNICG | FVSFFNNIC |
| | | VNRQKEFVSFFNNIC | QKEFVSFFN |
| | | RQKEFVSFFNNICGI | FVSFFNNIC |
| | | QKEFVSFFNNICGII | FVSFFNNIC |
| | | KEFVSFFNNICGIIT | FVSFFNNIC |
| | | EKSYKEFFDWLSKDV | YKEFFDWLS |
| | | YEKSYKEFFDWLSKD | YKEFFDWLS |
| | | YKEFFDWLSKDVNRQ | YKEFFDWLS |
| | | EYEKSYKEFFDWLSK | YKEFFDWLS |
| | | AEYEKSYKEFFDWLS | YEKSYKEFF |
| | | SYKEFFDWLSKDVNR | YKEFFDWLS |
| | | EFVSFFNNICGIITK | FVSFFNNIC |
| | | FVSFFNNICGIITKA | FVSFFNNIC |
| | | KVIKRVFTESENNNE | IKRVFTESE |
| | | EIFKVIKRVFTESEN | IKRVFTESE |
| | | KEFFDWLSKDVNRQK | FDWLSKDVN |
| | | FKVIKRVFTESENNN | IKRVFTESE |
| | | IFKVIKRVFTESENN | IKRVFTESE |
| | | EEIFKVIKRVFTESE | FKVIKRVFT |
| | | EFFDWLSKDVNRQKE | FDWLSKDVN |
| | | VIKRVFTESENNNEL | IKRVFTESE |
| | HLA-DRB1*0701 | NNELANLKNLSYNL | NLKNLNSYN |
| | | ANLKNLNSYNLNSNN | LKNLNSYNL |
| | | NELANLKNLSYNLN | LKNLNSYNL |
| | | ELANLKNLSYNLNS | LKNLNSYNL |
| | | LANLKNLSYNLNSN | LKNLNSYNL |
| | | CNEEIFKVIKRVFTE | IFKVIKRVF |
| | | NEEIFKVIKRVFTES | IFKVIKRVF |
| | | KCNEEIFKVIKRVFT | IFKVIKRVF |
| | | EEIFKVIKRVFTESE | IFKVIKRVF |
| | HLA-DRB1*0802 | None | |
| | HLA-DRB1*0901 | None | |
| | HLA-DRB1*1101 | None | |
| | HLA-DRB1*1302 | NNELANLKNLSYNL | LANLKNLNS |
| | | ELANLKNLSYNLNS | LKNLNSYNL |
| | | LANLKNLSYNLNSN | LKNLNSYNL |
| | | ANLKNLNSYNLNSNN | LKNLNSYNL |
| | | KEFVSFFNNICGIIT | FFNNICGII |
| | | VSFFNNICGIITKAV | FNNICGIIT |
| | | EFVSFFNNICGIITK | FNNICGIIT |
| | | FVSFFNNICGIITKA | FNNICGIIT |
| | | MKRISILSILLLLLL | ISILSILLL |
| | | SFFNNICGIITKAVD | FNNICGIIT |
| | | KRISILSILLLLLLF | ISILSILLL |
| | | NLKNLNSYNLNSNNK | LKNLNSYNL |
| | | ISILSILLLLLLFSC | ISILSILLL |
| | | RISILSILLLLLLFS | ISILSILLL |

EP 2 254 592 B1

| | | | |
|---|---|---|---|
| | | NEEIFKVIKRVFTES | FKVIKRVFT |
| | | EEIFKVIKRVFTESE | FKVIKRVFT |
| | | FFNNICGIITKAVDA | FNNICGIIT |
| | | ASKKRYNSNPKSLGF | YNSNPKSLG |
| | | KCNEEIFKVIKRVFT | IFKVIKRVF |
| | | DASKKRYNSNPKSLG | KKRYNSNPK |
| | | CNEEIFKVIKRVFTE | FKVIKRVFT |
| | | SKKRYNSNPKSLGFN | YNSNPKSLG |
| | | FNNICGIITKAVDAS | FNNICGIIT |
| | | EIFKVIKRVFTESEN | FKVIKRVFT |
| | | KKRYNSNPKSLGFNE | YNSNPKSLG |
| | | KRYNSNPKSLGFNEY | YNSNPKSLG |
| | | SILSILLLLLLFSCK | LSILLLLLL |
| | | IFKVIKRVFTESENN | FKVIKRVFT |
| | | FERLEALKTFLKDAM | LEALKTFLK |
| | | ERLEALKTFLKDAMG | LEALKTFLK |
| | | ILSILLLLLLFSCKQ | LSILLLLLL |
| | | ENNNELANLKNLNSY | LANLKNLNS |
| | | NNNELANLKNLNSYN | LANLKNLNS |
| | | LSILLLLLLFSCKQY | LSILLLLLL |
| | | FKVIKRVFTESENNN | FKVIKRVFT |
| | | EALKTFLKDAMGVNG | LKTFLKDAM |
| | | ESKDQIIEKGPVLTS | IIEKGPVLT |
| | | LEALKTFLKDAMGVN | LKTFLKDAM |
| | HLA-DRB1*1501 | NNELANLKNLNSYNL | LANLKNLNS |
| | | NELANLKNLNSYNLN | LANLKNLNS |
| | | ELANLKNLNSYNLNS | LKNLNSYNL |
| | | LANLKNLNSYNLNSN | LKNLNSYNL |
| | | ENNNELANLKNLNSY | LANLKNLNS |
| | | NNNELANLKNLNSYN | LANLKNLNS |
| | | ANLKNLNSYNLNSNN | LKNLNSYNL |
| | | LSILLLLLLFSCKQY | LLLLLFSCK |
| | | SENNNELANLKNLNS | NELANLKN |
| | | SILLLLLLFSCKQYG | LLLLLFSCK |
| | | ILSILLLLLLFSCKQ | LLLLLFSCK |
| | HLA-DRB3*0101 | None | |
| | HLA-DRB4*0101 | SILSILLLLLLFSCK | LLLLLLFSC |
| | | LSILLLLLLFSCKQY | LLLLLFSCK |
| | | ILSILLLLLLFSCKQ | LLLLLFSCK |
| | | SILLLLLLFSCKQYG | LLLLLFSCK |
| | | ILLLLLLFSCKQYGD | LLLLLFSCK |
| | | YKEFFDWLSKDVNRQ | FDWLSKDVN |
| | | KEFFDWLSKDVNRQK | FDWLSKDVN |
| | HLA-DRB5*0101 | None | |
| | | | |
| NP_045739.1\| BBA66 antigen, P35, putative [Borrelia burgdorferi B31]<br><br>Seq34 | HLA-DRB1*0101 | DGLIANASSNSSLSD | IANASSNSS |
| | | RQADGLIANASSNSS | LIANASSNS |
| | | QADGLIANASSNSSL | IANASSNSS |
| | | ADGLIANASSNSSLS | IANASSNSS |
| | | GLIANASSNSSLSDS | IANASSNSS |
| | | LIRELMISGLGTQIS | MISGLGTQI |
| | | ELMISGLGTQISFEL | ISGLGTQIS |
| | | IRELMISGLGTQISF | ISGLGTQIS |
| | | RELMISGLGTQISFE | ISGLGTQIS |
| | | LMISGLGTQISFELA | ISGLGTQIS |
| | | KQRIPIQAVTTVPGN | IQAVTTVPG |

1391

| | | | |
|---|---|---|---|
| | | TKQRIPIQAVTTVPG | IPIQAVTTV |
| | | QRIPIQAVTTVPGNT | IQAVTTVPG |
| | | IPIQAVTTVPGNTRT | IQAVTTVPG |
| | | RIPIQAVTTVPGNTR | IQAVTTVPG |
| | | LIANASSNSSLSDSK | IANASSNSS |
| | | IANASSNSSLSDSKS | IANASSNSS |
| | | MISGLGTQISFELAL | ISGLGTQIS |
| | | ISGLGTQISFELALE | ISGLGTQIS |
| | | SNLPKTTAARAASLT | LPKTTAARA |
| | | LSNLPKTTAARAASL | LPKTTAARA |
| | | AKTVAAAPNKGSQNQ | VAAAPNKGS |
| | | SQAAKTVAAAPNKGS | TVAAAPNKG |
| | | AAKTVAAAPNKGSQN | VAAAPNKGS |
| | | QAAKTVAAAPNKGSQ | VAAAPNKGS |
| | | GSKLQTLKNELIRAI | LQTLKNELI |
| | | IQAVTTVPGNTRTFN | IQAVTTVPG |
| | | PIQAVTTVPGNTRTF | IQAVTTVPG |
| | | SKLQTLKNELIRAIS | LKNELIRAI |
| | | ADLSNLPKTTAARAA | LPKTTAARA |
| | | SSAVQTTTSSGSKLQ | VQTTTSSGS |
| | | KNKVKGILNKAADDQ | VKGILNKAA |
| | | TNSSSAVQTTTSSGS | TNSSSAVQT |
| | | NLPKTTAARAASLTK | TTAARAASL |
| | | SAVQTTTSSGSKLQT | VQTTTSSGS |
| | | NKVKGILNKAADDQE | VKGILNKAA |
| | | TMKLKELKSKLNQIL | LKELKSKLN |
| | | SSSAVQTTTSSGSKL | VQTTTSSGS |
| | | MKLKELKSKLNQILD | LKELKSKLN |
| | | NSSSAVQTTTSSGSK | VQTTTSSGS |
| | | KTVAAAPNKGSQNQP | VAAAPNKGS |
| | | LPKTTAARAASLTKQ | TTAARAASL |
| | | SADLSNLPKTTAARA | LSNLPKTTA |
| | | SLTKQRIPIQAVTTV | RIPIQAVTT |
| | | LTKQRIPIQAVTTVP | IPIQAVTTV |
| | | KLQTLKNELIRAISE | LKNELIRAI |
| | | LQTLKNELIRAISEE | LKNELIRAI |
| | | SRQADGLIANASSNS | ADGLIANAS |
| | | DLSNLPKTTAARAAS | LPKTTAARA |
| | | EDYKNKVKGILNKAA | YKNKVKGIL |
| | | YKNKVKGILNKAADD | VKGILNKAA |
| | | DYKNKVKGILNKAAD | VKGILNKAA |
| | | FALNYSFSQPTRQQT | LNYSFSQPT |
| | | MKIKPLIQLKLLGLF | MKIKPLIQL |
| | | TFALNYSFSQPTRQQ | LNYSFSQPT |
| | | QTLKNELIRAISEEK | LKNELIRAI |
| | | SLIRELMISGLGTQI | IRELMISGL |
| | | LKELKSKLNQILDKR | LKSKLNQIL |
| | | KLKELKSKLNQILDK | LKSKLNQIL |
| | | PKTTAARAASLTKQR | TTAARAASL |
| | | PTFALNYSFSQPTRQ | LNYSFSQPT |
| | | KVYDRSYAPQLNSNT | YDRSYAPQL |
| | | LPTFALNYSFSQPTR | LNYSFSQPT |
| | | KGSQNQQAAPSPQLQ | QQAAPSPQL |
| | | GLPTFALNYSFSQPT | LPTFALNYS |
| | | NKGSQNQQAAPSPQL | SQNQQAAPS |
| | | GSQNQQAAPSPQLQS | QQAAPSPQL |
| | | LSFSADLSNLPKTTA | FSADLSNLP |

| | | | |
|---|---|---|---|
| | | SQNQQAAPSPQLQSL | QQAAPSPQL |
| | | DSAFELLDVISSAKV | FELLDVISS |
| | | YDQFKMKDSAFELLD | FKMKDSAFE |
| | | TYDQFKMKDSAFELL | FKMKDSAFE |
| | | SAFELLDVISSAKVY | LDVISSAKV |
| | | QNQQAAPSPQLQSLS | QQAAPSPQL |
| | | DQFKMKDSAFELLDV | FKMKDSAFE |
| | | FDFTMKLKELKSKLN | FTMKLKELK |
| | | KVKGILNKAADDQET | VKGILNKAA |
| | | VKGILNKAADDQETT | VKGILNKAA |
| | | DFTMKLKELKSKLNQ | LKELKSKLN |
| | | TVAAAPNKGSQNQPQ | VAAAPNKGS |
| | | FTMKLKELKSKLNQI | LKELKSKLN |
| | | AELKKQSQAAKTVAA | KQSQAAKTV |
| | | FELLDVISSAKVYDR | LDVISSAKV |
| | | IKPLIQLKLLGLFLF | LIQLKLLGL |
| | | VYDRSYAPQLNSNTP | YAPQLNSNT |
| | | KPLIQLKLLGLFLFS | LIQLKLLGL |
| | | ALNYSFSQPTRQQTN | LNYSFSQPT |
| | | LNYSFSQPTRQQTNS | LNYSFSQPT |
| | | SSGSKLQTLKNELIR | LQTLKNELI |
| | | FSADLSNLPKTTAAR | LSNLPKTTA |
| | | ETYDQFKMKDSAFEL | FKMKDSAFE |
| | | SGSKLQTLKNELIRA | LQTLKNELI |
| | | TSSGSKLQTLKNELI | GSKLQTLKN |
| | | AFELLDVISSAKVYD | LDVISSAKV |
| | | ELKKQSQAAKTVAAA | SQAAKTVAA |
| | | AVQTTTSSGSKLQTL | VQTTTSSGS |
| | | SFSADLSNLPKTTAA | LSNLPKTTA |
| | | RETYDQFKMKDSAFE | QFKMKDSAF |
| | | YDRSYAPQLNSNTPE | YAPQLNSNT |
| | | KRYLDNMQNARQSVL | LDNMQNARQ |
| | | RYLDNMQNARQSVLE | MQNARQSVL |
| | | VQTTTSSGSKLQTLK | VQTTTSSGS |
| | | ELLDVISSAKVYDRS | LDVISSAKV |
| | | NSRNSGLPTFALNYS | NSGLPTFAL |
| | | KTTAARAASLTKQRI | TAARAASLT |
| | | ASLTKQRIPIQAVTT | LTKQRIPIQ |
| | | LKKQSQAAKTVAAAP | SQAAKTVAA |
| | | KIKPLIQLKLLGLFL | LIQLKLLGL |
| | | TLKNELIRAISEEKN | LKNELIRAI |
| | | LKNELIRAISEEKNK | LKNELIRAI |
| | | KELKSKLNQILDKRK | LKSKLNQIL |
| | | QAVTTVPGNTRTFNS | VTTVPGNTR |
| | | RTFNSRNSGLPTFAL | FNSRNSGLP |
| | | SRNSGLPTFALNYSF | LPTFALNYS |
| | | YLDNMQNARQSVLEA | MQNARQSVL |
| | | VAAAPNKGSQNQPQT | VAAAPNKGS |
| | | QPTRQQTNSSSAVQT | QQTNSSSAV |
| | | PTRQQTNSSSAVQTT | TNSSSAVQT |
| | | QSQAAKTVAAAPNKG | SQAAKTVAA |
| | | TRQQTNSSSAVQTTT | TNSSSAVQT |
| | | RSYAPQLNSNTPEAE | YAPQLNSNT |
| | | FKMKDSAFELLDVIS | FKMKDSAFE |
| | | LIQLKLLGLFLFSCT | LKLLGLFLF |
| | | KKQSQAAKTVAAAPN | SQAAKTVAA |
| | | LDNMQNARQSVLEAY | MQNARQSVL |

| | | | |
|---|---|---|---|
| | | VAAELKKQSQAAKTV | KKQSQAAKT |
| | | KQSQAAKTVAAAPNK | SQAAKTVAA |
| | | DRSYAPQLNSNTPEA | YAPQLNSNT |
| | | AAELKKQSQAAKTVA | KQSQAAKTV |
| | | HSLIRELMISGLGTQ | IRELMISGL |
| | | PLIQLKLLGLFLFSC | LKLLGLFLF |
| | | NSGLPTFALNYSFSQ | LPTFALNYS |
| | | QFKMKDSAFELLDVI | FKMKDSAFE |
| | | RQQTNSSSAVQTTTS | TNSSSAVQT |
| | | NHSLIRELMISGLGT | IRELMISGL |
| | | DNMQNARQSVLEAYI | MQNARQSVL |
| | | TTAARAASLTKQRIP | TTAARAASL |
| | | QQTNSSSAVQTTTSS | TNSSSAVQT |
| | | RNSGLPTFALNYSFS | LPTFALNYS |
| | | AKVYDRSYAPQLNSN | YDRSYAPQL |
| | | TFNSRNSGLPTFALN | FNSRNSGLP |
| | | SLSFSADLSNLPKTT | FSADLSNLP |
| | | FNSRNSGLPTFALNY | FNSRNSGLP |
| | | MKDSAFELLDVISSA | FELLDVISS |
| | | QLQSLSFSADLSNLP | LQSLSFSAD |
| | | IQLKLLGLFLFSCTI | LKLLGLFLF |
| | | LQSLSFSADLSNLPK | FSADLSNLP |
| | | SSAKVYDRSYAPQLN | YDRSYAPQL |
| | | KMKDSAFELLDVISS | MKDSAFELL |
| | | AVTTVPGNTRTFNSR | VTTVPGNTR |
| | | KKRYLDNMQNARQSV | LDNMQNARQ |
| | | NQQAAPSPQLQSLSF | QQAAPSPQL |
| | | NYSFSQPTRQQTNSS | FSQPTRQQT |
| | | IKKRYLDNMQNARQS | LDNMQNARQ |
| | | LKLLGLFLFSCTIDA | LLGLFLFSC |
| | | QSLSFSADLSNLPKT | FSADLSNLP |
| | | NQNHSLIRELMISGL | LIRELMISG |
| | | QNHSLIRELMISGLG | IRELMISGL |
| | | AASLTKQRIPIQAVT | LTKQRIPIQ |
| | HLA-DRB1*0301 | None | |
| | HLA-DRB1*0401 | GSIMEALYNENQNHS | MEALYNENQ |
| | | SIMEALYNENQNHSL | LYNENQNHS |
| | | MEALYNENQNHSLIR | LYNENQNHS |
| | | IMEALYNENQNHSLI | LYNENQNHS |
| | | EALYNENQNHSLIRE | LYNENQNHS |
| | | RQADGLIANASSNSS | LIANASSNS |
| | | QADGLIANASSNSSL | IANASSNSS |
| | | ADGLIANASSNSSLS | IANASSNSS |
| | | DGLIANASSNSSLSD | IANASSNSS |
| | | GLIANASSNSSLSDS | IANASSNSS |
| | | LSFSADLSNLPKTTA | FSADLSNLP |
| | | LQSLSFSADLSNLPK | FSADLSNLP |
| | | QSLSFSADLSNLPKT | FSADLSNLP |
| | | SLSFSADLSNLPKTT | FSADLSNLP |
| | | QLQSLSFSADLSNLP | LSFSADLSN |
| | | LYNENQNHSLIRELM | LYNENQNHS |
| | | ALYNENQNHSLIREL | LYNENQNHS |
| | | LIANASSNSSLSDSK | IANASSNSS |
| | | IANASSNSSLSDSKS | IANASSNSS |
| | | FSADLSNLPKTTAAR | FSADLSNLP |
| | | SFSADLSNLPKTTAA | FSADLSNLP |
| | | PTFALNYSFSQPTRQ | FALNYSFSQ |

| | | | |
|---|---|---|---|
| | | LPTFALNYSFSQPTR | FALNYSFSQ |
| | | EIFNQDYLNAKINSF | FNQDYLNAK |
| | | GLPTFALNYSFSQPT | FALNYSFSQ |
| | | SGLPTFALNYSFSQP | FALNYSFSQ |
| | | NSGLPTFALNYSFSQ | LPTFALNYS |
| | | IFNQDYLNAKINSFD | YLNAKINSF |
| | | FALNYSFSQPTRQQT | FALNYSFSQ |
| | | TFALNYSFSQPTRQQ | FALNYSFSQ |
| | | FNQDYLNAKINSFDF | YLNAKINSF |
| | HLA-DRB1*0404 | RQADGLIANASSNSS | LIANASSNS |
| | | ADGLIANASSNSSLS | IANASSNSS |
| | | DGLIANASSNSSLSD | IANASSNSS |
| | | QADGLIANASSNSSL | IANASSNSS |
| | | GLIANASSNSSLSDS | IANASSNSS |
| | | DRSYAPQLNSNTPEA | YAPQLNSNT |
| | | RSYAPQLNSNTPEAE | QLNSNTPEA |
| | | SYAPQLNSNTPEAEN | QLNSNTPEA |
| | | HSLIRELMISGLGTQ | LIRELMISG |
| | | LIRELMISGLGTQIS | LMISGLGTQ |
| | | YAPQLNSNTPEAENE | QLNSNTPEA |
| | | LSFSADLSNLPKTTA | FSADLSNLP |
| | | SLIRELMISGLGTQI | LMISGLGTQ |
| | | RELMISGLGTQISFE | LMISGLGTQ |
| | | LIANASSNSSLSDSK | IANASSNSS |
| | | IANASSNSSLSDSKS | IANASSNSS |
| | | IRELMISGLGTQISF | LMISGLGTQ |
| | | APQLNSNTPEAENER | QLNSNTPEA |
| | | LQSLSFSADLSNLPK | FSADLSNLP |
| | HLA-DRB1*0405 | RIPIQAVTTVPGNTR | IQAVTTVPG |
| | | IPIQAVTTVPGNTRT | IQAVTTVPG |
| | | KQRIPIQAVTTVPGN | IQAVTTVPG |
| | | TKQRIPIQAVTTVPG | KQRIPIQAV |
| | | QRIPIQAVTTVPGNT | IQAVTTVPG |
| | | PIQAVTTVPGNTRTF | IQAVTTVPG |
| | | IQAVTTVPGNTRTFN | IQAVTTVPG |
| | | SQAAKTVAAAPNKGS | AKTVAAAPN |
| | | AKTVAAAPNKGSQNQ | VAAAPNKGS |
| | | QAAKTVAAAPNKGSQ | VAAAPNKGS |
| | | AAKTVAAAPNKGSQN | VAAAPNKGS |
| | | KTVAAAPNKGSQNQP | VAAAPNKGS |
| | | LQSLSFSADLSNLPK | FSADLSNLP |
| | HLA-DRB1*0701 | DNMQNARQSVLEAYI | MQNARQSVL |
| | | KRYLDNMQNARQSVL | DNMQNARQS |
| | | YLDNMQNARQSVLEA | MQNARQSVL |
| | | LDNMQNARQSVLEAY | MQNARQSVL |
| | | RYLDNMQNARQSVLE | MQNARQSVL |
| | | SNLPKTTAARAASLT | LPKTTAARA |
| | | LSNLPKTTAARAASL | LPKTTAARA |
| | | NLPKTTAARAASLTK | TTAARAASL |
| | | LPKTTAARAASLTKQ | TTAARAASL |
| | | ADLSNLPKTTAARAA | LPKTTAARA |
| | | DLSNLPKTTAARAAS | LPKTTAARA |
| | | SADLSNLPKTTAARA | LSNLPKTTA |
| | | MQNARQSVLEAYISI | MQNARQSVL |
| | | NMQNARQSVLEAYIS | MQNARQSVL |
| | | PKTTAARAASLTKQR | TTAARAASL |

| | HLA-DRB1*0802 | None | |
|---|---|---|---|
| | HLA-DRB1*0901 | DNMQNARQSVLEAYI | MQNARQSVL |
| | | LDNMQNARQSVLEAY | MQNARQSVL |
| | | YLDNMQNARQSVLEA | MQNARQSVL |
| | | RYLDNMQNARQSVLE | MQNARQSVL |
| | | KRYLDNMQNARQSVL | LDNMQNARQ |
| | HLA-DRB1*1101 | None | |
| | HLA-DRB1*1302 | LIQLKLLGLFLFSCT | LKLLGLFLF |
| | | IKPLIQLKLLGLFLF | IQLKLLGLF |
| | | IQLKLLGLFLFSCTI | LKLLGLFLF |
| | | KPLIQLKLLGLFLFS | LKLLGLFLF |
| | | PLIQLKLLGLFLFSC | LKLLGLFLF |
| | | ANLNEDYKNKVKGIL | LNEDYKNKV |
| | | QLKLLGLFLFSCTID | LKLLGLFLF |
| | | NLNEDYKNKVKGILN | YKNKVKGIL |
| | | LNEDYKNKVKGILNK | YKNKVKGIL |
| | | LKLLGLFLFSCTIDA | LKLLGLFLF |
| | | RIPIQAVTTVPGNTR | IQAVTTVPG |
| | | IPIQAVTTVPGNTRT | IQAVTTVPG |
| | | EDYKNKVKGILNKAA | YKNKVKGIL |
| | | NEDYKNKVKGILNKA | YKNKVKGIL |
| | | TKQRIPIQAVTTVPG | IPIQAVTTV |
| | | QRIPIQAVTTVPGNT | IQAVTTVPG |
| | | KQRIPIQAVTTVPGN | IQAVTTVPG |
| | | QADGLIANASSNSSL | IANASSNSS |
| | | ADGLIANASSNSSLS | IANASSNSS |
| | | DGLIANASSNSSLSD | IANASSNSS |
| | | RQADGLIANASSNSS | LIANASSNS |
| | | IQAVTTVPGNTRTFN | IQAVTTVPG |
| | | DYKNKVKGILNKAAD | YKNKVKGIL |
| | | GLIANASSNSSLSDS | IANASSNSS |
| | | PIQAVTTVPGNTRTF | IQAVTTVPG |
| | | YKNKVKGILNKAADD | YKNKVKGIL |
| | | MKIKPLIQLKLLGLF | IKPLIQLKL |
| | | LSFSADLSNLPKTTA | FSADLSNLP |
| | | NQDYLNAKINSFDFT | YLNAKINSF |
| | | SFSADLSNLPKTTAA | LSNLPKTTA |
| | | KRYLDNMQNARQSVL | YLDNMQNAR |
| | | FSADLSNLPKTTAAR | LSNLPKTTA |
| | | ELMISGLGTQISFEL | ISGLGTQIS |
| | | QDYLNAKINSFDFTM | YLNAKINSF |
| | | KIKPLIQLKLLGLFL | IQLKLLGLF |
| | | LIANASSNSSLSDSK | IANASSNSS |
| | | DYLNAKINSFDFTMK | YLNAKINSF |
| | | LIRELMISGLGTQIS | IRELMISGL |
| | | RYLDNMQNARQSVLE | MQNARQSVL |
| | | YLNAKINSFDFTMKL | YLNAKINSF |
| | | SADLSNLPKTTAARA | LSNLPKTTA |
| | | LMISGLGTQISFELA | LGTQISFEL |
| | | IRELMISGLGTQISF | ISGLGTQIS |
| | | ADLSNLPKTTAARAA | LSNLPKTTA |
| | | FNQDYLNAKINSFDF | YLNAKINSF |
| | | IFNQDYLNAKINSFD | YLNAKINSF |
| | | EIFNQDYLNAKINSF | FNQDYLNAK |
| | | YLDNMQNARQSVLEA | MQNARQSVL |
| | HLA-DRB1*1501 | IKPLIQLKLLGLFLF | LIQLKLLGL |

|  |  | FTMKLKELKSKLNQI | MKLKELKSK |
|---|---|---|---|
|  |  | FDFTMKLKELKSKLN | MKLKELKSK |
|  |  | KPLIQLKLLGLFLFS | LIQLKLLGL |
|  |  | DFTMKLKELKSKLNQ | MKLKELKSK |
|  |  | LIQLKLLGLFLFSCT | LIQLKLLGL |
|  |  | PLIQLKLLGLFLFSC | LIQLKLLGL |
|  |  | SFDFTMKLKELKSKL | MKLKELKSK |
|  |  | NSFDFTMKLKELKSK | TMKLKELKS |
|  |  | MKIKPLIQLKLLGLF | LIQLKLLGL |
|  | HLA-DRB3*0101 | None |  |
|  | HLA-DRB4*0101 | LIQLKLLGLFLFSCT | LKLLGLFLF |
|  |  | KPLIQLKLLGLFLFS | LKLLGLFLF |
|  |  | IKPLIQLKLLGLFLF | LIQLKLLGL |
|  |  | IQLKLLGLFLFSCTI | LKLLGLFLF |
|  |  | PLIQLKLLGLFLFSC | LKLLGLFLF |
|  |  | LKLLGLFLFSCTIDA | LKLLGLFLF |
|  |  | QLKLLGLFLFSCTID | LKLLGLFLF |
|  |  | SGLPTFALNYSFSQP | LPTFALNYS |
|  |  | FALNYSFSQPTRQQT | YSFSQPTRQ |
|  |  | LNYSFSQPTRQQTNS | YSFSQPTRQ |
|  |  | PTFALNYSFSQPTRQ | FALNYSFSQ |
|  |  | NSGLPTFALNYSFSQ | LPTFALNYS |
|  |  | ALNYSFSQPTRQQTN | YSFSQPTRQ |
|  |  | TFALNYSFSQPTRQQ | YSFSQPTRQ |
|  |  | SRNSGLPTFALNYSF | LPTFALNYS |
|  |  | RNSGLPTFALNYSFS | LPTFALNYS |
|  |  | NSRNSGLPTFALNYS | GLPTFALNY |
|  | HLA-DRB5*0101 | IKKRYLDNMQNARQS | LDNMQNARQ |
|  |  | KRYLDNMQNARQSVL | LDNMQNARQ |
|  |  | KKRYLDNMQNARQSV | LDNMQNARQ |
|  |  | YIKKRYLDNMQNARQ | IKKRYLDNM |
|  |  | RYLDNMQNARQSVLE | LDNMQNARQ |
|  |  | VYDRSYAPQLNSNTP | YAPQLNSNT |
|  |  | KVYDRSYAPQLNSNT | YDRSYAPQL |
|  |  | DRSYAPQLNSNTPEA | YAPQLNSNT |
|  |  | RSYAPQLNSNTPEAE | YAPQLNSNT |
|  |  | YDRSYAPQLNSNTPE | YAPQLNSNT |
|  |  | FALNYSFSQPTRQQT | YSFSQPTRQ |
|  |  | ALNYSFSQPTRQQTN | YSFSQPTRQ |
|  |  | LNYSFSQPTRQQTNS | YSFSQPTRQ |
|  |  | TFALNYSFSQPTRQQ | YSFSQPTRQ |
|  |  | PTFALNYSFSQPTRQ | NYSFSQPTR |
|  |  | FDFTMKLKELKSKLN | FTMKLKELK |
|  |  |  |  |
| AAT93824.1\|BBA66 antigen, P35, putative [Borrelia garinii PBi]  Seq35 | HLA-DRB1*0101 | TYDQFKMKDSAFTLL | FKMKDSAFT |
|  |  | YDQFKMKDSAFTLLD | FKMKDSAFT |
|  |  | DQFKMKDSAFTLLDV | FKMKDSAFT |
|  |  | RETYDQFKMKDSAFT | RETYDQFKM |
|  |  | ETYDQFKMKDSAFTL | FKMKDSAFT |
|  |  | ASQASQVASSASQAS | ASQVASSAS |
|  |  | ASQVASSASQASPVA | VASSASQAS |
|  |  | SQVASSASQASPVAS | VASSASQAS |
|  |  | SQASQVASSASQASP | VASSASQAS |
|  |  | QASQVASSASQASPV | VASSASQAS |
|  |  | QFKMKDSAFTLLDVI | FKMKDSAFT |
|  |  | FKMKDSAFTLLDVIS | FKMKDSAFT |

| | | ATPPKQIASAQAIQT | PKQIASAQA |
|---|---|---|---|
| | | TPPKQIASAQAIQTV | IASAQAIQT |
| | | PKQIASAQAIQTVPN | IASAQAIQT |
| | | PPKQIASAQAIQTVP | IASAQAIQT |
| | | KQIASAQAIQTVPNN | IASAQAIQT |
| | | SPATNVQATPPKQIA | VQATPPKQI |
| | | ASPATNVQATPPKQI | ATNVQATPP |
| | | ATNVQATPPKQIASA | VQATPPKQI |
| | | PATNVQATPPKQIAS | VQATPPKQI |
| | | VASSASQASPVASPA | VASSASQAS |
| | | SPLQKLKNNLLRRIA | LKNNLLRRI |
| | | QVASSASQASPVASP | VASSASQAS |
| | | TNVQATPPKQIASAQ | VQATPPKQI |
| | | NSPLQKLKNNLLRRI | LQKLKNNLL |
| | | IASAQAIQTVPNNTS | IASAQAIQT |
| | | PLQKLKNNLLRRIAE | LKNNLLRRI |
| | | LQKLKNNLLRRIAEE | LKNNLLRRI |
| | | QKLKNNLLRRIAEEK | LKNNLLRRI |
| | | QIASAQAIQTVPNNT | IASAQAIQT |
| | | VQATPPKQIASAQAI | VQATPPKQI |
| | | YTFSSSFSQPTSQTN | FSSSFSQPT |
| | | SLIISGLGIQISLES | ISGLGIQIS |
| | | NVQATPPKQIASAQA | VQATPPKQI |
| | | QYTFSSSFSQPTSQT | FSSSFSQPT |
| | | QTNFNNSQSNNVLTN | FNNSQSNNV |
| | | TNFNNSQSNNVLTNY | FNNSQSNNV |
| | | LIISGLGIQISLEST | ISGLGIQIS |
| | | SQTNFNNSQSNNVLT | FNNSQSNNV |
| | | TSQTNFNNSQSNNVL | FNNSQSNNV |
| | | PTSQTNFNNSQSNNV | QTNFNNSQS |
| | | SVFDRGSAPQLSSNT | FDRGSAPQL |
| | | IRSLIISGLGIQISL | ISGLGIQIS |
| | | ISVFDRGSAPQLSSN | FDRGSAPQL |
| | | LIRSLIISGLGIQIS | IISGLGIQI |
| | | PQQYTFSSSFSQPTS | FSSSFSQPT |
| | | QQYTFSSSFSQPTSQ | FSSSFSQPT |
| | | NISVFDRGSAPQLSS | FDRGSAPQL |
| | | KPQQYTFSSSFSQPT | YTFSSSFSQ |
| | | RSLIISGLGIQISLE | ISGLGIQIS |
| | | SNISVFDRGSAPQLS | FDRGSAPQL |
| | | AQAIQTVPNNTSTPN | IQTVPNNTS |
| | | QAIQTVPNNTSTPNQ | IQTVPNNTS |
| | | NQVNPGNPMQIANQA | VNPGNPMQI |
| | | KEWLNYADAIITNTS | LNYADAIIT |
| | | ANQVNPGNPMQIANQ | VNPGNPMQI |
| | | EWLNYADAIITNTSS | LNYADAIIT |
| | | PANQVNPGNPMQIAN | VNPGNPMQI |
| | | NPANQVNPGNPMQIA | VNPGNPMQI |
| | | FSSSFSQPTSQTNFN | FSSSFSQPT |
| | | TFSSSFSQPTSQTNF | FSSSFSQPT |
| | | KKEWLNYADAIITNT | LNYADAIIT |
| | | ASQASPVASPATNVQ | ASPVASPAT |
| | | IISGLGIQISLESTL | LGIQISLES |
| | | ASAQAIQTVPNNTST | IQTVPNNTS |
| | | SQASPVASPATNVQA | VASPATNVQ |
| | | VFDRGSAPQLSSNTP | DRGSAPQLS |
| | | ISGLGIQISLESTLE | LGIQISLES |

| | | KLKNNLLRRIAEEKN | LKNNLLRRI |
|---|---|---|---|
| | | LNPANQVNPGNPMQI | NQVNPGNPM |
| | | DSAFTLLDVISNISV | FTLLDVISN |
| | | NYRHQTQPSFVVPVY | HQTQPSFVV |
| | | PNQSIIKPQQYTFSS | IKPQQYTFS |
| | | PSFVVPVYSGNSPLQ | VVPVYSGNS |
| | | IIKPQQYTFSSSFSQ | IKPQQYTFS |
| | | SAFTLLDVISNISVF | FTLLDVISN |
| | | TPNQSIIKPQQYTFS | QSIIKPQQY |
| | | QIANQANQANQANQA | IANQANQAN |
| | | IANQANQANQANQAN | IANQANQAN |
| | | IKPQQYTFSSSFSQP | IKPQQYTFS |
| | | YSGNSPLQKLKNNLL | YSGNSPLQK |
| | | YRHQTQPSFVVPVYS | HQTQPSFVV |
| | | PVYSGNSPLQKLKNN | YSGNSPLQK |
| | | IESFNTQYLNTIINS | FNTQYLNTI |
| | | ILYQENQNHSLIRSL | YQENQNHSL |
| | | KIESFNTQYLNTIIN | FNTQYLNTI |
| | | SFNTQYLNTIINSYT | FNTQYLNTI |
| | | PVASPATNVQATPPK | VASPATNVQ |
| | | NLQGLNPANQVNPGN | LQGLNPANQ |
| | | NILYQENQNHSLIRS | YQENQNHSL |
| | | FNTQYLNTIINSYTF | LNTIINSYT |
| | | QTVPNNTSTPNQSII | VPNNTSTPN |
| | | KDSAFTLLDVISNIS | FTLLDVISN |
| | | ESFNTQYLNTIINSY | FNTQYLNTI |
| | | YAMMDFDQAKTTEFG | FDQAKTTEF |
| | | QASQASQASQVASSA | QASQASQVA |
| | | SGNSPLQKLKNNLLR | LQKLKNNLL |
| | | SASQASPVASPATNV | ASPVASPAT |
| | | NQANQASQASQASQV | QASQASQAS |
| | | NQASQASQASQVASS | QASQASQVA |
| | | QATPPKQIASAQAIQ | KQIASAQAI |
| | | KLKELESKLNSILAE | LKELESKLN |
| | | ASQASQASQVASSAS | QASQASQVA |
| | | LKELESKLNSILAEK | LKELESKLN |
| | | KYLDKMQDARQSALD | DKMQDARQS |
| | | KISINTGSNATKNKT | INTGSNATK |
| | | ASSASQASPVASPAT | ASQASPVAS |
| | | NKYLDKMQDARQSAL | DKMQDARQS |
| | | LQGLNPANQVNPGNP | LQGLNPANQ |
| | | SFSQPTSQTNFNNSQ | FSQPTSQTN |
| | | TQYLNTIINSYTFKD | LNTIINSYT |
| | | YLDKMQDARQSALDL | DKMQDARQS |
| | | SERNRLYAMMDFDQA | RNRLYAMMD |
| | | AFTLLDVISNISVFD | LLDVISNIS |
| | | FTLLDVISNISVFDR | LLDVISNIS |
| | | AKISINTGSNATKNK | INTGSNATK |
| | | GNSPLQKLKNNLLRR | LQKLKNNLL |
| | | IKVAGLQKNTQSKKN | IKVAGLQKN |
| | | QAKISINTGSNATKN | INTGSNATK |
| | | DQAKISINTGSNATK | SINTGSNAT |
| | | NIKVAGLQKNTQSKK | IKVAGLQKN |
| | | NRLYAMMDFDQAKTT | YAMMDFDQA |
| | | LYAMMDFDQAKTTEF | YAMMDFDQA |
| | | RLYAMMDFDQAKTTE | YAMMDFDQA |
| | | NTQYLNTIINSYTFK | LNTIINSYT |

| | | |
|---|---|---|
| | | SSASQASPVASPATN ASQASPVAS<br>RNRLYAMMDFDQAKT YAMMDFDQA<br>VASPATNVQATPPKQ VASPATNVQ<br>ERNRLYAMMDFDQAK YAMMDFDQA<br>YQENQNHSLIRSLII YQENQNHSL<br>AMMDFDQAKTTEFGS FDQAKTTEF<br>VYSGNSPLQKLKNNL YSGNSPLQK<br>IITNTSSNSKRNDPQ ITNTSSNSK<br>IKNKYLDKMQDARQS IKNKYLDKM<br>QYLNTIINSYTFKDK LNTIINSYT<br>ENQNHSLIRSLIISG NQNHSLIRS |
| HLA-DRB1*0301 | None | |
| HLA-DRB1*0401 | ESFNTQYLNTIINSY FNTQYLNTI<br>SFNTQYLNTIINSYT YLNTIINSY<br>FNTQYLNTIINSYTF YLNTIINSY<br>TQYLNTIINSYTFKD YLNTIINSY<br>NTQYLNTIINSYTFK YLNTIINSY<br>GSIMNILYQENQNHS MNILYQENQ<br>SIMNILYQENQNHSL LYQENQNHS<br>MNILYQENQNHSLIR LYQENQNHS<br>NILYQENQNHSLIRS LYQENQNHS<br>IMNILYQENQNHSLI LYQENQNHS<br>QYLNTIINSYTFKDK YLNTIINSY<br>YLNTIINSYTFKDKL YLNTIINSY<br>SFVVPVYSGNSPLQK VYSGNSPLQ<br>PSFVVPVYSGNSPLQ VVPVYSGNS<br>VPVYSGNSPLQKLKN VYSGNSPLQ<br>VVPVYSGNSPLQKLK VYSGNSPLQ<br>FVVPVYSGNSPLQKL VYSGNSPLQ<br>SLIISGLGIQISLES ISGLGIQIS<br>LIISGLGIQISLEST LGIQISLES<br>SGLGIQISLESTLEE LGIQISLES<br>ISGLGIQISLESTLE LGIQISLES<br>RETYDQFKMKDSAFT TYDQFKMKD<br>IISGLGIQISLESTL LGIQISLES<br>NYADAIITNTSSNSK IITNTSSNS<br>PTSQTNFNNSQSNNV SQTNFNNSQ<br>YADAIITNTSSNSKR ITNTSSNSK<br>ADAIITNTSSNSKRN ITNTSSNSK<br>ETYDQFKMKDSAFTL FKMKDSAFT<br>TSQTNFNNSQSNNVL FNNSQSNNV<br>TYDQFKMKDSAFTLL FKMKDSAFT<br>SQTNFNNSQSNNVLT FNNSQSNNV<br>QTNFNNSQSNNVLTN FNNSQSNNV<br>DQFKMKDSAFTLLDV FKMKDSAFT<br>TNFNNSQSNNVLTNY FNNSQSNNV<br>YDQFKMKDSAFTLLD FKMKDSAFT<br>DAIITNTSSNSKRND ITNTSSNSK<br>AIITNTSSNSKRNDP ITNTSSNSK<br>ILYQENQNHSLIRSL LYQENQNHS<br>LYQENQNHSLIRSLI LYQENQNHS<br>ANLNKDYKNKVEELL LNKDYKNKV<br>TIDANLNKDYKNKVE LNKDYKNKV<br>CTIDANLNKDYKNKV DANLNKDYK<br>IDANLNKDYKNKVEE LNKDYKNKV<br>DANLNKDYKNKVEEL LNKDYKNKV<br>KPQQYTFSSSFSQPT YTFSSSFSQ |

| | | | |
|---|---|---|---|
| | | QQYTFSSSFSQPTSQ | YTFSSSFSQ |
| | | PQQYTFSSSFSQPTS | YTFSSSFSQ |
| | | VYSGNSPLQKLKNNL | VYSGNSPLQ |
| | | FTLLDVISNISVFDR | LLDVISNIS |
| | HLA-DRB1*0404 | TQYLNTIINSYTFKD | YLNTIINSY |
| | | SFNTQYLNTIINSYT | YLNTIINSY |
| | | ESFNTQYLNTIINSY | SFNTQYLNT |
| | | FNTQYLNTIINSYTF | YLNTIINSY |
| | | NTQYLNTIINSYTFK | YLNTIINSY |
| | | NKVEELLNSSTDDQA | LLNSSTDDQ |
| | | KNKVEELLNSSTDDQ | VEELLNSST |
| | | KVEELLNSSTDDQAK | LLNSSTDDQ |
| | | VEELLNSSTDDQAKI | LLNSSTDDQ |
| | | EELLNSSTDDQAKIS | LLNSSTDDQ |
| | | QYLNTIINSYTFKDK | YLNTIINSY |
| | | YLNTIINSYTFKDKL | YLNTIINSY |
| | | NYADAIITNTSSNSK | IITNTSSNS |
| | | YADAIITNTSSNSKR | ITNTSSNSK |
| | | ADAIITNTSSNSKRN | ITNTSSNSK |
| | | DAIITNTSSNSKRND | ITNTSSNSK |
| | | AIITNTSSNSKRNDP | ITNTSSNSK |
| | | QAIQTVPNNTSTPNQ | TVPNNTSTP |
| | | ELLNSSTDDQAKISI | LLNSSTDDQ |
| | | GSIMNILYQENQNHS | ILYQENQNH |
| | | LLNSSTDDQAKISIN | LLNSSTDDQ |
| | | FGSIMNILYQENQNH | IMNILYQEN |
| | | AIQTVPNNTSTPNQS | TVPNNTSTP |
| | | LNYADAIITNTSSNS | YADAIITNT |
| | | IQTVPNNTSTPNQSI | TVPNNTSTP |
| | | AQAIQTVPNNTSTPN | TVPNNTSTP |
| | | SIMNILYQENQNHSL | ILYQENQNH |
| | | SAQAIQTVPNNTSTP | IQTVPNNTS |
| | | IMNILYQENQNHSLI | ILYQENQNH |
| | | SQASPVASPATNVQA | VASPATNVQ |
| | | ASQASPVASPATNVQ | PVASPATNV |
| | | ASPVASPATNVQATP | VASPATNVQ |
| | HLA-DRB1*0405 | IASAQAIQTVPNNTS | AQAIQTVPN |
| | | ASAQAIQTVPNNTST | IQTVPNNTS |
| | | SAQAIQTVPNNTSTP | IQTVPNNTS |
| | | AQAIQTVPNNTSTPN | IQTVPNNTS |
| | | QAIQTVPNNTSTPNQ | IQTVPNNTS |
| | | AIQTVPNNTSTPNQS | IQTVPNNTS |
| | | IQTVPNNTSTPNQSI | IQTVPNNTS |
| | | ASPATNVQATPPKQI | TNVQATPPK |
| | | SGLGIQISLESTLEE | LGIQISLES |
| | | SPATNVQATPPKQIA | VQATPPKQI |
| | | PATNVQATPPKQIAS | VQATPPKQI |
| | | KMKDSAFTLLDVISN | KDSAFTLLD |
| | | LGIQISLESTLEEIE | ISLESTLEE |
| | | LTNYRHQTQPSFVVP | HQTQPSFVV |
| | | GIQISLESTLEEIEK | ISLESTLEE |
| | | VLTNYRHQTQPSFVV | YRHQTQPSF |
| | | GLGIQISLESTLEEI | ISLESTLEE |
| | | ATNVQATPPKQIASA | VQATPPKQI |
| | | EKKIESFNTQYLNTI | IESFNTQYL |
| | | IQISLESTLEEIEKK | ISLESTLEE |
| | | TNVQATPPKQIASAQ | VQATPPKQI |

|  |  | KKIESFNTQYLNTII | IESFNTQYL |
|---|---|---|---|
|  |  | TNYRHQTQPSFVVPV | HQTQPSFVV |
|  |  | DQFKMKDSAFTLLDV | KDSAFTLLD |
|  |  | YDQFKMKDSAFTLLD | FKMKDSAFT |
|  |  | TQYLNTIINSYTFKD | LNTIINSYT |
|  | HLA-DRB1*0701 | TQYLNTIINSYTFKD | YLNTIINSY |
|  |  | NQNHSLIRSLIISGL | HSLIRSLII |
|  |  | QNHSLIRSLIISGLG | HSLIRSLII |
|  |  | FNTQYLNTIINSYTF | YLNTIINSY |
|  |  | NTQYLNTIINSYTFK | YLNTIINSY |
|  |  | ADAIITNTSSNSKRN | ITNTSSNSK |
|  |  | NYADAIITNTSSNSK | IITNTSSNS |
|  |  | YADAIITNTSSNSKR | ITNTSSNSK |
|  |  | VLTNYRHQTQPSFVV | RHQTQPSFV |
|  |  | TNYRHQTQPSFVVPV | HQTQPSFVV |
|  |  | YQENQNHSLIRSLII | NHSLIRSLI |
|  |  | QENQNHSLIRSLIIS | HSLIRSLII |
|  |  | SQTNFNNSQSNNVLT | FNNSQSNNV |
|  |  | DAIITNTSSNSKRND | ITNTSSNSK |
|  |  | ENQNHSLIRSLIISG | HSLIRSLII |
|  |  | TSQTNFNNSQSNNVL | FNNSQSNNV |
|  |  | QTNFNNSQSNNVLTN | FNNSQSNNV |
|  |  | TNFNNSQSNNVLTNY | FNNSQSNNV |
|  |  | LTNYRHQTQPSFVVP | HQTQPSFVV |
|  |  | SFNTQYLNTIINSYT | YLNTIINSY |
|  |  | PTSQTNFNNSQSNNV | NFNNSQSNN |
|  | HLA-DRB1*0802 | None |  |
|  | HLA-DRB1*0901 | QQYTFSSSFSQPTSQ | YTFSSSFSQ |
|  |  | KPQQYTFSSSFSQPT | YTFSSSFSQ |
|  |  | PQQYTFSSSFSQPTS | YTFSSSFSQ |
|  |  | IIKPQQYTFSSSFSQ | IIKPQQYTF |
|  |  | IKPQQYTFSSSFSQP | YTFSSSFSQ |
|  |  | KKEWLNYADAIITNT | WLNYADAII |
|  |  | AEKKEWLNYADAIIT | WLNYADAII |
|  |  | EKKEWLNYADAIITN | WLNYADAII |
|  |  | KEWLNYADAIITNTS | WLNYADAII |
|  |  | QYTFSSSFSQPTSQT | YTFSSSFSQ |
|  |  | YTFSSSFSQPTSQTN | YTFSSSFSQ |
|  |  | LAEKKEWLNYADAII | LAEKKEWLN |
|  |  | TPPKQIASAQAIQTV | KQIASAQAI |
|  |  | ATPPKQIASAQAIQT | KQIASAQAI |
|  |  | HSLIRSLIISGLGIQ | LIRSLIISG |
|  |  | PPKQIASAQAIQTVP | KQIASAQAI |
|  |  | SLIRSLIISGLGIQI | LIISGLGIQ |
|  |  | LIRSLIISGLGIQIS | LIISGLGIQ |
|  |  | IRSLIISGLGIQISL | LIISGLGIQ |
|  |  | RSLIISGLGIQISLE | LIISGLGIQ |
|  |  | QTNFNNSQSNNVLTN | FNNSQSNNV |
|  |  | SQTNFNNSQSNNVLT | FNNSQSNNV |
|  |  | TNFNNSQSNNVLTNY | FNNSQSNNV |
|  | HLA-DRB1*1101 | None |  |
|  | HLA-DRB1*1302 | NSPLQKLKNNLLRRI | LQKLKNNLL |
|  |  | SPLQKLKNNLLRRIA | LKNNLLRRI |
|  |  | PLQKLKNNLLRRIAE | LKNNLLRRI |
|  |  | LQKLKNNLLRRIAEE | LKNNLLRRI |
|  |  | QSLGQYIKNKYLDKM | LGQYIKNKY |

| | | | |
|---|---|---|---|
| | | QKLKNNLLRRIAEEK | LKNNLLRRI |
| | | SLGQYIKNKYLDKMQ | LGQYIKNKY |
| | | LGQYIKNKYLDKMQD | LGQYIKNKY |
| | | PQSLGQYIKNKYLDK | LGQYIKNKY |
| | | DPQSLGQYIKNKYLD | LGQYIKNKY |
| | | TQYLNTIINSYTFKD | LNTIINSYT |
| | | NDPQSLGQYIKNKYL | LGQYIKNKY |
| | | FNTQYLNTIINSYTF | YLNTIINSY |
| | | RNDPQSLGQYIKNKY | PQSLGQYIK |
| | | SFNTQYLNTIINSYT | YLNTIINSY |
| | | KLKNNLLRRIAEEKN | LKNNLLRRI |
| | | NTQYLNTIINSYTFK | YLNTIINSY |
| | | QYLNTIINSYTFKDK | LNTIINSYT |
| | | AQAIQTVPNNTSTPN | IQTVPNNTS |
| | | QAIQTVPNNTSTPNQ | VPNNTSTPN |
| | | LKNNLLRRIAEEKNK | LKNNLLRRI |
| | | FTLLDVISNISVFDR | LLDVISNIS |
| | | ESFNTQYLNTIINSY | FNTQYLNTI |
| | | HSLIRSLIISGLGIQ | IRSLIISGL |
| | | YLNTIINSYTFKDKL | IINSYTFKD |
| | | SLIISGLGIQISLES | LIISGLGIQ |
| | | SLIRSLIISGLGIQI | IRSLIISGL |
| | | LIISGLGIQISLEST | LGIQISLES |
| | | IQTVPNNTSTPNQSI | VPNNTSTPN |
| | | AIQTVPNNTSTPNQS | VPNNTSTPN |
| | | TLLDVISNISVFDRG | ISNISVFDR |
| | | IISGLGIQISLESTL | LGIQISLES |
| | | LLDVISNISVFDRGS | ISNISVFDR |
| | | ISGLGIQISLESTLE | LGIQISLES |
| | | GNSPLQKLKNNLLRR | LQKLKNNLL |
| | | LIRSLIISGLGIQIS | LIISGLGIQ |
| | | IRSLIISGLGIQISL | LIISGLGIQ |
| | | YADAIITNTSSNSKR | IITNTSSNS |
| | | NYADAIITNTSSNSK | IITNTSSNS |
| | | RQSALDLYLNITEIR | ALDLYLNIT |
| | | ADAIITNTSSNSKRN | IITNTSSNS |
| | | SGLGIQISLESTLEE | LGIQISLES |
| | | DAIITNTSSNSKRND | IITNTSSNS |
| | | SGNSPLQKLKNNLLR | LQKLKNNLL |
| | | TEFGSIMNILYQENQ | FGSIMNILY |
| | | LNTIINSYTFKDKLK | LNTIINSYT |
| | | YSGNSPLQKLKNNLL | YSGNSPLQK |
| | | AFTLLDVISNISVFD | LLDVISNIS |
| | | QGLNPANQVNPGNPM | LNPANQVNP |
| | | TTEFGSIMNILYQEN | FGSIMNILY |
| | | DQAKISINTGSNATK | ISINTGSNA |
| | | NQNHSLIRSLIISGL | LIRSLIISG |
| | | LNYADAIITNTSSNS | LNYADAIIT |
| | | KTTEFGSIMNILYQE | FGSIMNILY |
| | | DDQAKISINTGSNAT | ISINTGSNA |
| | | AKISINTGSNATKNK | ISINTGSNA |
| | | QAKISINTGSNATKN | ISINTGSNA |
| | | QTVPNNTSTPNQSII | VPNNTSTPN |
| | | NHSLIRSLIISGLGI | IRSLIISGL |
| | | TNFNNSQSNNVLTNY | FNNSQSNNV |
| | | LNPANQVNPGNPMQI | NQVNPGNPM |
| | | LDVISNISVFDRGSA | ISNISVFDR |

|  |  | TKNKTNIKVAGLQKN | NKTNIKVAG |
|---|---|---|---|
|  |  | NFNNSQSNNVLTNYR | FNNSQSNNV |
|  |  | QNHSLIRSLIISGLG | IRSLIISGL |
|  |  | FNNSQSNNVLTNYRH | FNNSQSNNV |
|  |  | GQYIKNKYLDKMQDA | IKNKYLDKM |
|  |  | QYIKNKYLDKMQDAR | IKNKYLDKM |
|  |  | SAFTLLDVISNISVF | LLDVISNIS |
|  |  | GLNPANQVNPGNPMQ | NQVNPGNPM |
|  |  | RSLIISGLGIQISLE | LIISGLGIQ |
|  |  | AIITNTSSNSKRNDP | IITNTSSNS |
|  |  | AKTTEFGSIMNILYQ | FGSIMNILY |
|  |  | KISINTGSNATKNKT | ISINTGSNA |
|  |  | QTNFNNSQSNNVLTN | FNNSQSNNV |
|  |  | DSAFTLLDVISNISV | LLDVISNIS |
|  |  | SQTNFNNSQSNNVLT | FNNSQSNNV |
|  |  | TSQTNFNNSQSNNVL | FNNSQSNNV |
|  |  | QAKTTEFGSIMNILY | EFGSIMNIL |
|  |  | GLGIQISLESTLEEI | LGIQISLES |
|  |  | LGIQISLESTLEEIE | LGIQISLES |
|  |  | KDSAFTLLDVISNIS | FTLLDVISN |
|  |  | EFGSIMNILYQENQN | FGSIMNILY |
|  |  | NKTNIKVAGLQKNTQ | IKVAGLQKN |
|  |  | ANLNKDYKNKVEELL | LNKDYKNKV |
|  |  | PTSQTNFNNSQSNNV | NFNNSQSNN |
|  |  | DVISNISVFDRGSAP | ISNISVFDR |
|  |  | PANQVNPGNPMQIAN | NQVNPGNPM |
|  |  | NPANQVNPGNPMQIA | NQVNPGNPM |
|  |  | TDDQAKISINTGSNA | AKISINTGS |
|  |  | SQSNNVLTNYRHQTQ | SNNVLTNYR |
|  |  | ISINTGSNATKNKTN | ISINTGSNA |
|  |  | SAQAIQTVPNNTSTP | IQTVPNNTS |
|  |  | NSQSNNVLTNYRHQT | SNNVLTNYR |
|  |  | NIKVAGLQKNTQSKK | IKVAGLQKN |
|  | HLA-DRB1*1501 | NSPLQKLKNNLLRRI | QKLKNNLLR |
|  |  | SPLQKLKNNLLRRIA | QKLKNNLLR |
|  |  | HSLIRSLIISGLGIQ | LIRSLIISG |
|  |  | PLQKLKNNLLRRIAE | QKLKNNLLR |
|  |  | NQNHSLIRSLIISGL | LIRSLIISG |
|  |  | GNSPLQKLKNNLLRR | LQKLKNNLL |
|  |  | QNHSLIRSLIISGLG | LIRSLIISG |
|  |  | NHSLIRSLIISGLGI | LIRSLIISG |
|  |  | TQYLNTIINSYTFKD | YLNTIINSY |
|  | HLA-DRB3*0101 | None |  |
|  | HLA-DRB4*0101 | YSGNSPLQKLKNNLL | SPLQKLKNN |
|  |  | NSPLQKLKNNLLRRI | LQKLKNNLL |
|  |  | SPLQKLKNNLLRRIA | LQKLKNNLL |
|  |  | GNSPLQKLKNNLLRR | LQKLKNNLL |
|  |  | SGNSPLQKLKNNLLR | LQKLKNNLL |
|  | HLA-DRB5*0101 | RETYDQFKMKDSAFT | YDQFKMKDS |
|  |  | ETYDQFKMKDSAFTL | FKMKDSAFT |
|  |  | TYDQFKMKDSAFTLL | FKMKDSAFT |
|  |  | YDQFKMKDSAFTLLD | FKMKDSAFT |
|  |  | DQFKMKDSAFTLLDV | FKMKDSAFT |
|  |  |  |  |
| NP_045742.1| hypothetical protein BBA69 [Borrelia | HLA-DRB1*0101 | IKINIITMILTLICI | IITMILTLI |
|  |  | IIKINIITMILTLIC | IITMILTLI |

| | | | |
|---|---|---|---|
| burgdorferi B31] | | KINIITMILTLICIS | IITMILTLI |
| | | INIITMILTLICISC | IITMILTLI |
| Seq36 | | NIIKINIITMILTLI | INIITMILT |
| | | SDEKFMGTTASELKA | FMGTTASEL |
| | | ASDEKFMGTTASELK | FMGTTASEL |
| | | DEKFMGTTASELKAI | FMGTTASEL |
| | | EKFMGTTASELKAIG | FMGTTASEL |
| | | AKITNEESNLLDTYI | ITNEESNLL |
| | | NIITMILTLICISCA | IITMILTLI |
| | | QIAKITNEESNLLDT | ITNEESNLL |
| | | IAKITNEESNLLDTY | ITNEESNLL |
| | | IQIAKITNEESNLLD | ITNEESNLL |
| | | DIQIAKITNEESNLL | IAKITNEES |
| | | GASDEKFMGTTASEL | EKFMGTTAS |
| | | IITMILTLICISCAP | IITMILTLI |
| | | NKYYKDSDSLQSAFY | YKDSDSLQS |
| | | HFNKYYKDSDSLQSA | YKDSDSLQS |
| | | FNKYYKDSDSLQSAF | YKDSDSLQS |
| | | EHFNKYYKDSDSLQS | NKYYKDSDS |
| | | LNIIKINIITMILTL | INIITMILT |
| | | KLNIIKINIITMILT | IKINIITMI |
| | | KFMGTTASELKAIGK | FMGTTASEL |
| | | IAANFLYRIALDIQL | IAANFLYRI |
| | | KITNEESNLLDTYIR | ITNEESNLL |
| | | FMGTTASELKAIGKE | FMGTTASEL |
| | | ITNEESNLLDTYIRA | ITNEESNLL |
| | | PKIAANFLYRIALDI | IAANFLYRI |
| | | ENDPKIAANFLYRIA | IAANFLYRI |
| | | YENDPKIAANFLYRI | DPKIAANFL |
| | | KNQYDIQIAKITNEE | YDIQIAKIT |
| | | NQYDIQIAKITNEES | YDIQIAKIT |
| | | RKNQYDIQIAKITNE | YDIQIAKIT |
| | | NDPKIAANFLYRIAL | IAANFLYRI |
| | | EDRKNQYDIQIAKIT | RKNQYDIQI |
| | | DRKNQYDIQIAKITN | YDIQIAKIT |
| | | DPKIAANFLYRIALD | IAANFLYRI |
| | | AKLNIIKINIITMIL | IKINIITMI |
| | | ANFLYRIALDIQLKL | YRIALDIQL |
| | | AANFLYRIALDIQLK | YRIALDIQL |
| | | NFLYRIALDIQLKLE | YRIALDIQL |
| | | ITMILTLICISCAPF | ITMILTLIC |
| | | ENEKMLLKRFLLSSL | MLLKRFLLS |
| | | EKMLLKRFLLSSLDY | MLLKRFLLS |
| | | FLYRIALDIQLKLEK | YRIALDIQL |
| | | NEKMLLKRFLLSSLD | MLLKRFLLS |
| | | APFNKINPKANENTK | INPKANENT |
| | | CAPFNKINPKANENT | FNKINPKAN |
| | | TMILTLICISCAPFN | LTLICISCA |
| | | FNKINPKANENTKLK | INPKANENT |
| | | KIAANFLYRIALDIQ | IAANFLYRI |
| | | PFNKINPKANENTKL | INPKANENT |
| | | KMLLKRFLLSSLDYK | LKRFLLSSL |
| | | MLLKRFLLSSLDYKK | LKRFLLSSL |
| | | MILTLICISCAPFNK | ICISCAPFN |
| | | LQLQEKFKKTLNKTL | KFKKTLNKT |
| | | LQEKFKKTLNKTLED | FKKTLNKTL |
| | | QLQEKFKKTLNKTLE | FKKTLNKTL |

| | | | |
|---|---|---|---|
| | | NENEKMLLKRFLLSS | MLLKRFLLS |
| | | QEKFKKTLNKTLEDY | FKKTLNKTL |
| | HLA-DRB1*0301 | None | |
| | HLA-DRB1*0401 | EDYRKNTNNIQENKV | YRKNTNNIQ |
| | | LEDYRKNTNNIQENK | YRKNTNNIQ |
| | | TLEDYRKNTNNIQEN | YRKNTNNIQ |
| | | KTLEDYRKNTNNIQE | YRKNTNNIQ |
| | | NKTLEDYRKNTNNIQ | LEDYRKNTN |
| | | YRKNTNNIQENKVLA | YRKNTNNIQ |
| | | DYRKNTNNIQENKVL | YRKNTNNIQ |
| | | EHFNKYYKDSDSLQS | YYKDSDSLQ |
| | | HFNKYYKDSDSLQSA | YKDSDSLQS |
| | | FNKYYKDSDSLQSAF | YKDSDSLQS |
| | | NKYYKDSDSLQSAFY | YKDSDSLQS |
| | | EKLINNYENDPKIAA | LINNYENDP |
| | | KLINNYENDPKIAAN | YENDPKIAA |
| | | LINNYENDPKIAANF | YENDPKIAA |
| | | NNYENDPKIAANFLY | YENDPKIAA |
| | | INNYENDPKIAANFL | YENDPKIAA |
| | | DIQIAKITNEESNLL | IAKITNEES |
| | | IQIAKITNEESNLLD | ITNEESNLL |
| | HLA-DRB1*0404 | None | |
| | HLA-DRB1*0405 | IQIAKITNEESNLLD | IAKITNEES |
| | | DIQIAKITNEESNLL | IAKITNEES |
| | | NQYDIQIAKITNEES | QIAKITNEE |
| | | QYDIQIAKITNEESN | IAKITNEES |
| | | YDIQIAKITNEESNL | IAKITNEES |
| | | IIKINIITMILTLIC | INIITMILT |
| | | IKINIITMILTLICI | ITMILTLIC |
| | | QIAKITNEESNLLDT | IAKITNEES |
| | | KINIITMILTLICIS | ITMILTLIC |
| | | INIITMILTLICISC | ITMILTLIC |
| | | IAKITNEESNLLDTY | IAKITNEES |
| | | NIITMILTLICISCA | ITMILTLIC |
| | | LEDYRKNTNNIQENK | YRKNTNNIQ |
| | | EDYRKNTNNIQENKV | YRKNTNNIQ |
| | | TLEDYRKNTNNIQEN | YRKNTNNIQ |
| | | KTLEDYRKNTNNIQE | YRKNTNNIQ |
| | | EILEKLINNYENDPK | LEKLINNYE |
| | | EKLINNYENDPKIAA | INNYENDPK |
| | | KEILEKLINNYENDP | LEKLINNYE |
| | | LKEILEKLINNYEND | LEKLINNYE |
| | | TLKEILEKLINNYEN | LEKLINNYE |
| | | EHFNKYYKDSDSLQS | FNKYYKDSD |
| | HLA-DRB1*0701 | DEKFMGTTASELKAI | FMGTTASEL |
| | | EKFMGTTASELKAIG | FMGTTASEL |
| | | GASDEKFMGTTASEL | KFMGTTASE |
| | | ASDEKFMGTTASELK | FMGTTASEL |
| | | SDEKFMGTTASELKA | FMGTTASEL |
| | | KFMGTTASELKAIGK | FMGTTASEL |
| | | FMGTTASELKAIGKE | FMGTTASEL |
| | | LQLQEKFKKTLNKTL | KFKKTLNKT |
| | | QEKFKKTLNKTLEDY | FKKTLNKTL |
| | | QLQEKFKKTLNKTLE | FKKTLNKTL |
| | | LQEKFKKTLNKTLED | FKKTLNKTL |

|  |  | EKFKKTLNKTLEDYR | FKKTLNKTL |
|---|---|---|---|
|  | HLA-DRB1*0802 | TKLKKNTRLKKPANP | LKKNTRLKK |
|  |  | ENTKLKKNTRLKKPA | LKKNTRLKK |
|  |  | NTKLKKNTRLKKPAN | LKKNTRLKK |
|  |  | ANENTKLKKNTRLKK | ENTKLKKNT |
|  |  | NENTKLKKNTRLKKP | LKKNTRLKK |
|  | HLA-DRB1*0901 | None |  |
|  | HLA-DRB1*1101 | ENTKLKKNTRLKKPA | LKKNTRLKK |
|  |  | ANENTKLKKNTRLKK | ANENTKLKK |
|  |  | NTKLKKNTRLKKPAN | LKKNTRLKK |
|  |  | NENTKLKKNTRLKKP | LKKNTRLKK |
|  |  | TKLKKNTRLKKPANP | LKKNTRLKK |
|  |  | KLKKNTRLKKPANPG | LKKNTRLKK |
|  |  | LKKNTRLKKPANPGE | LKKNTRLKK |
|  | HLA-DRB1*1302 | KLNIIKINIITMILT | IKINIITMI |
|  |  | LNIIKINIITMILTL | IKINIITMI |
|  |  | NIIKINIITMILTLI | IKINIITMI |
|  |  | IIKINIITMILTLIC | INIITMILT |
|  |  | IKINIITMILTLICI | INIITMILT |
|  |  | AKLNIIKINIITMIL | IKINIITMI |
|  |  | KAKLNIIKINIITMI | LNIIKINII |
|  |  | KINIITMILTLICIS | INIITMILT |
|  |  | INIITMILTLICISC | INIITMILT |
|  |  | KKAKLNIIKINIITM | LNIIKINII |
|  |  | MKKAKLNIIKINIIT | LNIIKINII |
|  |  | NIITMILTLICISCA | IITMILTLI |
|  |  | IITMILTLICISCAP | IITMILTLI |
|  |  | ANENTKLKKNTRLKK | NTKLKKNTR |
|  |  | NENTKLKKNTRLKKP | LKKNTRLKK |
|  |  | ENTKLKKNTRLKKPA | LKKNTRLKK |
|  |  | NTKLKKNTRLKKPAN | LKKNTRLKK |
|  |  | TKLKKNTRLKKPANP | LKKNTRLKK |
|  |  | RKNQYDIQIAKITNE | YDIQIAKIT |
|  |  | NQYDIQIAKITNEES | IQIAKITNE |
|  |  | KNQYDIQIAKITNEE | IQIAKITNE |
|  | HLA-DRB1*1501 | KMLLKRFLLSSLDYK | LKRFLLSSL |
|  |  | MLLKRFLLSSLDYKK | LKRFLLSSL |
|  |  | ENEKMLLKRFLLSSL | MLLKRFLLS |
|  |  | NEKMLLKRFLLSSLD | LKRFLLSSL |
|  |  | EKMLLKRFLLSSLDY | LKRFLLSSL |
|  |  | LLKRFLLSSLDYKKE | LKRFLLSSL |
|  |  | LKRFLLSSLDYKKEN | LKRFLLSSL |
|  |  | NENEKMLLKRFLLSS | MLLKRFLLS |
|  |  | INIITMILTLICISC | IITMILTLI |
|  | HLA-DRB3*0101 | None |  |
|  | HLA-DRB4*0101 | NFLYRIALDIQLKLE | LYRIALDIQ |
|  |  | ANFLYRIALDIQLKL | LYRIALDIQ |
|  |  | IAANFLYRIALDIQL | LYRIALDIQ |
|  |  | AANFLYRIALDIQLK | LYRIALDIQ |
|  |  | KIAANFLYRIALDIQ | FLYRIALDI |
|  |  | LYRIALDIQLKLEKH | LYRIALDIQ |
|  |  | FLYRIALDIQLKLEK | LYRIALDIQ |
|  |  | NKTLEDYRKNTNNIQ | LEDYRKNTN |
|  |  | KTLEDYRKNTNNIQE | YRKNTNNIQ |
|  | HLA-DRB5*0101 | APFNKINPKANENTK | FNKINPKAN |
|  |  | CAPFNKINPKANENT | FNKINPKAN |

| | | CISCAPFNKINPKAN | ISCAPFNKI |
|---|---|---|---|
| | | ISCAPFNKINPKANE | FNKINPKAN |
| | | SCAPFNKINPKANEN | FNKINPKAN |
| | | FNKINPKANENTKLK | FNKINPKAN |
| | | | |
| AAT93826.1\| conserved hypothetical protein BBA69[Borrelia garinii PBi] Seq37 | HLA-DRB1*0101 | KLNIIKLNILTTTLT | KLNILTTTL |
| | | LNIIKLNILTTTLTL | LNILTTTLT |
| | | NIIKLNILTTTLTLI | LNILTTTLT |
| | | IIKLNILTTTLTLIC | LNILTTTLT |
| | | IKLNILTTTLTLICI | LNILTTTLT |
| | | KLNILTTTLTLICIS | LNILTTTLT |
| | | LNILTTTLTLICISC | LNILTTTLT |
| | | EPLREKYPEATASKL | LREKYPEAT |
| | | DEKMQMKKIIYSSLN | MQMKKIIYS |
| | | EDEKMQMKKIIYSSL | MQMKKIIYS |
| | | EKMQMKKIIYSSLNY | MQMKKIIYS |
| | | TKLNIIKLNILTTTL | IKLNILTTT |
| | | PEDEKMQMKKIIYSS | MQMKKIIYS |
| | | LPEDEKMQMKKIIYS | DEKMQMKKI |
| | | IKENFKKALNKTIDA | FKKALNKTI |
| | | LREKYPEATASKLET | YPEATASKL |
| | | KTKLNIIKLNILTTT | IIKLNILTT |
| | | KENFKKALNKTIDAY | FKKALNKTI |
| | | PLREKYPEATASKLE | YPEATASKL |
| | | KLETTLKILEAQKEK | LKILEAQKE |
| | | KIKENFKKALNKTID | FKKALNKTI |
| | | SKLETTLKILEAQKE | ETTLKILEA |
| | | LKIKENFKKALNKTI | NFKKALNKT |
| | | ETTLKILEAQKEKEN | LKILEAQKE |
| | | LETTLKILEAQKEKE | LKILEAQKE |
| | | NILTTTLTLICISCA | LTTTLTLIC |
| | | TTLKILEAQKEKENI | LKILEAQKE |
| | | ENFKKALNKTIDAYN | FKKALNKTI |
| | | MQMKKIIYSSLNYET | MQMKKIIYS |
| | | ILTTTLTLICISCAV | LTLICISCA |
| | | LEPLREKYPEATASK | LREKYPEAT |
| | | REKYPEATASKLETT | YPEATASKL |
| | | EKYPEATASKLETTL | YPEATASKL |
| | | KMQMKKIIYSSLNYE | MQMKKIIYS |
| | | DLEPLREKYPEATAS | LREKYPEAT |
| | | KDLEPLREKYPEATA | LREKYPEAT |
| | | NKDLEPLREKYPEAT | LEPLREKYP |
| | | LTTTLTLICISCAVN | LTLICISCA |
| | | KKTKLNIIKLNILTT | LNIIKLNIL |
| | | TTTLTLICISCAVNK | LTLICISCA |
| | | NFKKALNKTIDAYNQ | FKKALNKTI |
| | HLA-DRB1*0301 | None | |
| | HLA-DRB1*0401 | QEILEKLDKNLQHKK | LEKLDKNLQ |
| | | EILEKLDKNLQHKKI | LEKLDKNLQ |
| | | QIDFLKTFKTDPHDS | LKTFKTDPH |
| | | LQEILEKLDKNLQHK | LEKLDKNLQ |
| | | IIKLNILTTTLTLIC | LNILTTTLT |
| | | NTQIDFLKTFKTDPH | FLKTFKTDP |
| | | IDFLKTFKTDPHDSL | LKTFKTDPH |
| | | ILQEILEKLDKNLQH | LEKLDKNLQ |

|  |  | | |
|---|---|---|---|
|  |  | TQIDFLKTFKTDPHD | LKTFKTDPH |
|  |  | IKLNILTTTLTLICI | LNILTTTLT |
|  |  | KILQEILEKLDKNLQ | EILEKLDKN |
|  |  | KLNIIKLNILTTTLT | IIKLNILTT |
|  |  | LNIIKLNILTTTLTL | LNILTTTLT |
|  |  | NIIKLNILTTTLTLI | LNILTTTLT |
|  |  | DFLKTFKTDPHDSLP | LKTFKTDPH |
|  |  | INENYKEFNSLKPIY | YKEFNSLKP |
|  | HLA-DRB1*0404 | IDFLKTFKTDPHDSL | FLKTFKTDP |
|  |  | NTQIDFLKTFKTDPH | FLKTFKTDP |
|  |  | TQIDFLKTFKTDPHD | FLKTFKTDP |
|  |  | QIDFLKTFKTDPHDS | FLKTFKTDP |
|  |  | DNTQIDFLKTFKTDP | IDFLKTFKT |
|  |  | DFLKTFKTDPHDSLP | FLKTFKTDP |
|  |  | IIKLNILTTTLTLIC | KLNILTTTL |
|  |  | FLKTFKTDPHDSLPE | FLKTFKTDP |
|  |  | NIIKLNILTTTLTLI | KLNILTTTL |
|  |  | KLNIIKLNILTTTLT | KLNILTTTL |
|  |  | LNIIKLNILTTTLTL | KLNILTTTL |
|  | HLA-DRB1*0405 | KHINENYKEFNSLKP | NYKEFNSLK |
|  |  | HINENYKEFNSLKPI | YKEFNSLKP |
|  |  | INENYKEFNSLKPIY | YKEFNSLKP |
|  |  | ENIKNFVNKFQDLEP | IKNFVNKFQ |
|  |  | MQMKKIIYSSLNYET | KKIIYSSLN |
|  | HLA-DRB1*0701 | IIKLNILTTTLTLIC | ILTTTLTLI |
|  |  | IKLNILTTTLTLICI | ILTTTLTLI |
|  |  | NIIKLNILTTTLTLI | LNILTTTLT |
|  |  | EPLREKYPEATASKL | LREKYPEAT |
|  |  | PLREKYPEATASKLE | YPEATASKL |
|  |  | LREKYPEATASKLET | YPEATASKL |
|  |  | REKYPEATASKLETT | YPEATASKL |
|  |  | EKYPEATASKLETTL | YPEATASKL |
|  |  | KLNILTTTLTLICIS | ILTTTLTLI |
|  | HLA-DRB1*0802 | None | |
|  | HLA-DRB1*0901 | LKIKENFKKALNKTI | IKENFKKAL |
|  |  | KENFKKALNKTIDAY | FKKALNKTI |
|  |  | IKENFKKALNKTIDA | FKKALNKTI |
|  |  | KIKENFKKALNKTID | FKKALNKTI |
|  |  | ENFKKALNKTIDAYN | FKKALNKTI |
|  | HLA-DRB1*1101 | None | |
|  | HLA-DRB1*1302 | KLNIIKLNILTTTLT | IKLNILTTT |
|  |  | LNIIKLNILTTTLTL | LNILTTTLT |
|  |  | NIIKLNILTTTLTLI | LNILTTTLT |
|  |  | IIKLNILTTTLTLIC | LNILTTTLT |
|  |  | IKLNILTTTLTLICI | LNILTTTLT |
|  |  | KTKLNIIKLNILTTT | LNIIKLNIL |
|  |  | TKLNIIKLNILTTTL | IKLNILTTT |
|  |  | KLNILTTTLTLICIS | LNILTTTLT |
|  |  | LNILTTTLTLICISC | LNILTTTLT |
|  |  | KKTKLNIIKLNILTT | LNIIKLNIL |
|  |  | MKKTKLNIIKLNILT | LNIIKLNIL |
|  |  | QSRLDIISKVIKNPI | LDIISKVIK |
|  |  | SRLDIISKVIKNPIK | ISKVIKNPI |
|  |  | RLDIISKVIKNPIKD | ISKVIKNPI |
|  |  | DIISKVIKNPIKDEL | ISKVIKNPI |
|  |  | LDIISKVIKNPIKDE | ISKVIKNPI |

| | | | |
|---|---|---|---|
| | | IISKVIKNPIKDELQ | IKNPIKDEL |
| | | ISKVIKNPIKDELQI | IKNPIKDEL |
| | | YDIPIIIQSRLDIIS | IPIIIQSRL |
| | | FIYDIPIIIQSRLDI | IPIIIQSRL |
| | | IYDIPIIIQSRLDII | IPIIIQSRL |
| | | IIIQSRLDIISKVIK | IIIQSRLDI |
| | | IPIIIQSRLDIISKV | IIIQSRLDI |
| | | IQSRLDIISKVIKNP | LDIISKVIK |
| | | EDEKMQMKKIIYSSL | MQMKKIIYS |
| | | DIPIIIQSRLDIISK | IIIQSRLDI |
| | | IIQSRLDIISKVIKN | LDIISKVIK |
| | | EKMQMKKIIYSSLNY | MQMKKIIYS |
| | | KNFIYDIPIIIQSRL | FIYDIPIII |
| | | DEKMQMKKIIYSSLN | MQMKKIIYS |
| | | MQMKKIIYSSLNYET | MQMKKIIYS |
| | | KMQMKKIIYSSLNYE | MQMKKIIYS |
| | | PEDEKMQMKKIIYSS | MQMKKIIYS |
| | | NFIYDIPIIIQSRLD | IPIIIQSRL |
| | | LPEDEKMQMKKIIYS | EKMQMKKII |
| | | NPIKDELQILNQKEI | IKDELQILN |
| | | IKDELQILNQKEIEE | LQILNQKEI |
| | | PIKDELQILNQKEIE | LQILNQKEI |
| | | KDELQILNQKEIEEL | LQILNQKEI |
| | HLA-DRB1*1501 | IEELLMRIESELKIK | LMRIESELK |
| | | EIEELLMRIESELKI | LMRIESELK |
| | | KEIEELLMRIESELK | LLMRIESEL |
| | | EELLMRIESELKIKE | LMRIESELK |
| | | ELLMRIESELKIKEN | LMRIESELK |
| | HLA-DRB3*0101 | None | |
| | HLA-DRB4*0101 | ILQEILEKLDKNLQH | LEKLDKNLQ |
| | | LQEILEKLDKNLQHK | LEKLDKNLQ |
| | | QEILEKLDKNLQHKK | LEKLDKNLQ |
| | | EILEKLDKNLQHKKI | LEKLDKNLQ |
| | | KILQEILEKLDKNLQ | ILQEILEKL |
| | HLA-DRB5*0101 | LKIKENFKKALNKTI | LKIKENFKK |
| | | NKFQDLEPSKKQNKD | FQDLEPSKK |
| | | VNKFQDLEPSKKQNK | FQDLEPSKK |
| | | ESELKIKENFKKALN | LKIKENFKK |
| | | IESELKIKENFKKAL | LKIKENFKK |
| | | SELKIKENFKKALNK | LKIKENFKK |
| | | RIESELKIKENFKKA | LKIKENFKK |
| | | MRIESELKIKENFKK | IESELKIKE |
| | | KFQDLEPSKKQNKDL | LEPSKKQNK |
| | | | |
| NP_045573.1\| hypothetical protein BBI42 [Borrelia burgdorferi B31]<br><br>Seq38 | HLA-DRB1*0101 | PEYYRNMPRPTAYQQ | YRNMPRPTA |
| | | SPEYYRNMPRPTAYQ | YRNMPRPTA |
| | | GSPEYYRNMPRPTAY | YRNMPRPTA |
| | | EGSPEYYRNMPRPTA | YYRNMPRPT |
| | | EYYRNMPRPTAYQQY | YRNMPRPTA |
| | | MRILVGVCIIALALL | MRILVGVCI |
| | | YYRNMPRPTAYQQYL | YRNMPRPTA |
| | | LKDPNYRGVVLPVSD | YRGVVLPVS |
| | | DPNYRGVVLPVSDYN | YRGVVLPVS |
| | | SLKDPNYRGVVLPVS | LKDPNYRGV |
| | | KDPNYRGVVLPVSDY | YRGVVLPVS |
| | | PNYRGVVLPVSDYNE | YRGVVLPVS |

| | | | |
|---|---|---|---|
| | | YRNMPRPTAYQQYLK | YRNMPRPTA |
| | | NKFFLDLGSEQSKDL | FLDLGSEQS |
| | | KFFLDLGSEQSKDLI | FLDLGSEQS |
| | | ILVGVCIIALALLGC | VCIIALALL |
| | | LVGVCIIALALLGCY | VCIIALALL |
| | | RILVGVCIIALALLG | VCIIALALL |
| | | VGVCIIALALLGCYL | VCIIALALL |
| | | EGIFSSLKLYASEHR | FSSLKLYAS |
| | | GIFSSLKLYASEHRL | FSSLKLYAS |
| | | FNKFFLDLGSEQSKD | FLDLGSEQS |
| | | YFNKFFLDLGSEQSK | FLDLGSEQS |
| | | EYFNKFFLDLGSEQS | FNKFFLDLG |
| | | TEGIFSSLKLYASEH | FSSLKLYAS |
| | | GVCIIALALLGCYLP | IIALALLGC |
| | | IVTEGIFSSLKLYAS | IVTEGIFSS |
| | | VTEGIFSSLKLYASE | FSSLKLYAS |
| | | VCIIALALLGCYLPD | IIALALLGC |
| | | VKRYDYNRPVPILPT | YNRPVPILP |
| | | NYRGVVLPVSDYNEE | YRGVVLPVS |
| | | FFLDLGSEQSKDLIK | FLDLGSEQS |
| | | FLDLGSEQSKDLIKL | FLDLGSEQS |
| | | YRGVVLPVSDYNEEY | YRGVVLPVS |
| | | FSSLKLYASEHRLLV | FSSLKLYAS |
| | | IFSSLKLYASEHRLL | FSSLKLYAS |
| | | KVKRYDYNRPVPILP | YDYNRPVPI |
| | | EEGSPEYYRNMPRPT | EYYRNMPRP |
| | | NKFMRIVRWLYSCIE | MRIVRWLYS |
| | | KFMRIVRWLYSCIEE | VRWLYSCIE |
| | | CIIALALLGCYLPDN | IIALALLGC |
| | | SSLKLYASEHRLLVE | LKLYASEHR |
| | | IIALALLGCYLPDNQ | IIALALLGC |
| | | FMRIVRWLYSCIEEL | VRWLYSCIE |
| | | MRIVRWLYSCIEELY | VRWLYSCIE |
| | | QTFFENSESSDMGSD | FENSESSDM |
| | | TFFENSESSDMGSDE | FENSESSDM |
| | | VQTFFENSESSDMGS | FENSESSDM |
| | | AVQTFFENSESSDMG | FENSESSDM |
| | | QAVQTFFENSESSDM | TFFENSESS |
| | | RNMPRPTAYQQYLKV | MPRPTAYQQ |
| | | LDLGSEQSKDLIKLF | LGSEQSKDL |
| | HLA-DRB1*0301 | None | |
| | HLA-DRB1*0401 | DLIKLFIMVKNEQNN | LFIMVKNEQ |
| | | IKLFIMVKNEQNNNK | LFIMVKNEQ |
| | | KDLIKLFIMVKNEQN | LFIMVKNEQ |
| | | LIKLFIMVKNEQNNN | LFIMVKNEQ |
| | | SKDLIKLFIMVKNEQ | IKLFIMVKN |
| | | PEYYRNMPRPTAYQQ | YRNMPRPTA |
| | | SPEYYRNMPRPTAYQ | YRNMPRPTA |
| | | EGSPEYYRNMPRPTA | YYRNMPRPT |
| | | GSPEYYRNMPRPTAY | YRNMPRPTA |
| | | KLFIMVKNEQNNNKF | LFIMVKNEQ |
| | | EYYRNMPRPTAYQQY | YRNMPRPTA |
| | | LFIMVKNEQNNNKFM | LFIMVKNEQ |
| | | EYFNKFFLDLGSEQS | FFLDLGSEQ |
| | | YYRNMPRPTAYQQYL | YRNMPRPTA |
| | | QNNNKFMRIVRWLYS | FMRIVRWLY |

| | | | |
|---|---|---|---|
| | | NNNKFMRIVRWLYSC | FMRIVRWLY |
| | | YFNKFFLDLGSEQSK | FFLDLGSEQ |
| | HLA-DRB1*0404 | IKLFIMVKNEQNNNK | LFIMVKNEQ |
| | | DLIKLFIMVKNEQNN | LFIMVKNEQ |
| | | LIKLFIMVKNEQNNN | LFIMVKNEQ |
| | | KLFIMVKNEQNNNKF | VKNEQNNNK |
| | | LFIMVKNEQNNNKFM | VKNEQNNNK |
| | | KDLIKLFIMVKNEQN | LFIMVKNEQ |
| | | SKDLIKLFIMVKNEQ | IKLFIMVKN |
| | HLA-DRB1*0405 | NKFMRIVRWLYSCIE | MRIVRWLYS |
| | | KFMRIVRWLYSCIEE | MRIVRWLYS |
| | | FMRIVRWLYSCIEEL | VRWLYSCIE |
| | | PEYYRNMPRPTAYQQ | YRNMPRPTA |
| | | SPEYYRNMPRPTAYQ | YRNMPRPTA |
| | | KDLIKLFIMVKNEQN | IKLFIMVKN |
| | | GSPEYYRNMPRPTAY | YRNMPRPTA |
| | | EYYRNMPRPTAYQQY | YRNMPRPTA |
| | | EGSPEYYRNMPRPTA | YYRNMPRPT |
| | | MRIVRWLYSCIEELY | VRWLYSCIE |
| | | DLIKLFIMVKNEQNN | FIMVKNEQN |
| | | IKLFIMVKNEQNNNK | FIMVKNEQN |
| | | LIKLFIMVKNEQNNN | FIMVKNEQN |
| | | KLFIMVKNEQNNNKF | FIMVKNEQN |
| | | RIVRWLYSCIEELYS | VRWLYSCIE |
| | | NNKFMRIVRWLYSCI | MRIVRWLYS |
| | | NNNKFMRIVRWLYSC | MRIVRWLYS |
| | | QNNNKFMRIVRWLYS | FMRIVRWLY |
| | | YYRNMPRPTAYQQYL | YRNMPRPTA |
| | | YRNMPRPTAYQQYLK | YRNMPRPTA |
| | | LFIMVKNEQNNNKFM | FIMVKNEQN |
| | | IVRWLYSCIEELYSP | VRWLYSCIE |
| | HLA-DRB1*0701 | NNKFMRIVRWLYSCI | FMRIVRWLY |
| | | NKFMRIVRWLYSCIE | FMRIVRWLY |
| | | QNNNKFMRIVRWLYS | FMRIVRWLY |
| | | NNNKFMRIVRWLYSC | FMRIVRWLY |
| | | KFMRIVRWLYSCIEE | MRIVRWLYS |
| | HLA-DRB1*0802 | None | |
| | HLA-DRB1*0901 | None | |
| | HLA-DRB1*1101 | EYYRNMPRPTAYQQY | YRNMPRPTA |
| | | PEYYRNMPRPTAYQQ | YRNMPRPTA |
| | | EGSPEYYRNMPRPTA | YYRNMPRPT |
| | | GSPEYYRNMPRPTAY | YRNMPRPTA |
| | | SPEYYRNMPRPTAYQ | YRNMPRPTA |
| | | YYRNMPRPTAYQQYL | YRNMPRPTA |
| | | YRNMPRPTAYQQYLK | YRNMPRPTA |
| | HLA-DRB1*1302 | SKDLIKLFIMVKNEQ | IKLFIMVKN |
| | | KDLIKLFIMVKNEQN | IKLFIMVKN |
| | | DLIKLFIMVKNEQNN | IKLFIMVKN |
| | | IKLFIMVKNEQNNNK | LFIMVKNEQ |
| | | HRLLVEIKKTLISLK | VEIKKTLIS |
| | | LIKLFIMVKNEQNNN | LFIMVKNEQ |
| | | EQSKDLIKLFIMVKN | SKDLIKLFI |
| | | RLLVEIKKTLISLKD | VEIKKTLIS |
| | | QSKDLIKLFIMVKNE | IKLFIMVKN |
| | | GSPEYYRNMPRPTAY | YYRNMPRPT |
| | | SPEYYRNMPRPTAYQ | YYRNMPRPT |

|  |  | PEYYRNMPRPTAYQQ | YYRNMPRPT |
|---|---|---|---|
|  |  | LLVEIKKTLISLKDP | VEIKKTLIS |
|  |  | EHRLLVEIKKTLISL | VEIKKTLIS |
|  |  | SEHRLLVEIKKTLIS | LVEIKKTLI |
|  |  | EGSPEYYRNMPRPTA | YYRNMPRPT |
|  |  | MRILVGVCIIALALL | MRILVGVCI |
|  |  | EYYRNMPRPTAYQQY | YYRNMPRPT |
|  |  | IMVKNEQNNNKFMRI | EQNNNKFMR |
|  |  | GVCIIALALLGCYLP | IALALLGCY |
|  |  | VCIIALALLGCYLPD | LALLGCYLP |
|  |  | VKNEQNNNKFMRIVR | EQNNNKFMR |
|  |  | MVKNEQNNNKFMRIV | EQNNNKFMR |
|  |  | LVEIKKTLISLKDPN | VEIKKTLIS |
|  |  | FIMVKNEQNNNKFMR | VKNEQNNNK |
|  |  | YYRNMPRPTAYQQYL | YYRNMPRPT |
|  |  | KNEQNNNKFMRIVRW | EQNNNKFMR |
|  |  | EQNNNKFMRIVRWLY | EQNNNKFMR |
|  |  | LVGVCIIALALLGCY | VCIIALALL |
|  |  | CIIALALLGCYLPDN | LALLGCYLP |
|  |  | VGVCIIALALLGCYL | VCIIALALL |
|  |  | VEIKKTLISLKDPNY | VEIKKTLIS |
|  |  | QNNNKFMRIVRWLYS | FMRIVRWLY |
|  | HLA-DRB1*1501 | NKFMRIVRWLYSCIE | MRIVRWLYS |
|  |  | NNKFMRIVRWLYSCI | MRIVRWLYS |
|  |  | KFMRIVRWLYSCIEE | MRIVRWLYS |
|  |  | NNNKFMRIVRWLYSC | MRIVRWLYS |
|  |  | QNNNKFMRIVRWLYS | FMRIVRWLY |
|  |  | FMRIVRWLYSCIEEL | MRIVRWLYS |
|  |  | MRIVRWLYSCIEELY | MRIVRWLYS |
|  |  | EGIFSSLKLYASEHR | FSSLKLYAS |
|  |  | GIFSSLKLYASEHRL | LKLYASEHR |
|  |  | FSSLKLYASEHRLLV | LKLYASEHR |
|  |  | IFSSLKLYASEHRLL | LKLYASEHR |
|  |  | SSLKLYASEHRLLVE | LKLYASEHR |
|  |  | PNYRGVVLPVSDYNE | YRGVVLPVS |
|  |  | LKDPNYRGVVLPVSD | YRGVVLPVS |
|  |  | KDPNYRGVVLPVSDY | YRGVVLPVS |
|  |  | SLKDPNYRGVVLPVS | PNYRGVVLP |
|  |  | DPNYRGVVLPVSDYN | YRGVVLPVS |
|  |  | SKDLIKLFIMVKNEQ | LIKLFIMVK |
|  |  | KDLIKLFIMVKNEQN | LIKLFIMVK |
|  |  | EQSKDLIKLFIMVKN | LIKLFIMVK |
|  |  | QSKDLIKLFIMVKNE | LIKLFIMVK |
|  | HLA-DRB3*0101 | None |  |
|  | HLA-DRB4*0101 | EIKKTLISLKDPNYR | LISLKDPNY |
|  |  | KKTLISLKDPNYRGV | ISLKDPNYR |
|  |  | KTLISLKDPNYRGVV | ISLKDPNYR |
|  |  | IKKTLISLKDPNYRG | ISLKDPNYR |
|  |  | TLISLKDPNYRGVVL | ISLKDPNYR |
|  |  | LISLKDPNYRGVVLP | ISLKDPNYR |
|  |  | ISLKDPNYRGVVLPV | ISLKDPNYR |
|  |  | IIALALLGCYLPDNQ | LALLGCYLP |
|  |  | IALALLGCYLPDNQE | LGCYLPDNQ |
|  |  | ALALLGCYLPDNQEQ | LGCYLPDNQ |
|  |  | LALLGCYLPDNQEQA | LGCYLPDNQ |
|  |  | ALLGCYLPDNQEQAV | LGCYLPDNQ |

| | | | |
|---|---|---|---|
| | | NKFMRIVRWLYSCIE | IVRWLYSCI |
| | | KFMRIVRWLYSCIEE | IVRWLYSCI |
| | | FMRIVRWLYSCIEEL | IVRWLYSCI |
| | HLA-DRB5*0101 | GSPEYYRNMPRPTAY | YRNMPRPTA |
| | | SPEYYRNMPRPTAYQ | YRNMPRPTA |
| | | PEYYRNMPRPTAYQQ | YRNMPRPTA |
| | | EYYRNMPRPTAYQQY | YRNMPRPTA |
| | | EGSPEYYRNMPRPTA | YYRNMPRPT |
| | | IKLFIMVKNEQNNNK | LFIMVKNEQ |
| | | KLFIMVKNEQNNNKF | VKNEQNNNK |
| | | LFIMVKNEQNNNKFM | VKNEQNNNK |
| | | IMVKNEQNNNKFMRI | VKNEQNNNK |
| | | FIMVKNEQNNNKFMR | VKNEQNNNK |
| | | TAYQQYLKVKRYDYN | YQQYLKVKR |
| | | YYRNMPRPTAYQQYL | YRNMPRPTA |
| | | YRNMPRPTAYQQYLK | YRNMPRPTA |
| | | | |
| NP_051318.1| revA protein[Borrelia burgdorferi B31]<br><br>Seq39 | HLA-DRB1*0101 | NIFKLFFASMLFVMA | FFASMLFVM |
| | | IFKLFFASMLFVMAC | FASMLFVMA |
| | | FKLFFASMLFVMACK | FASMLFVMA |
| | | KLFFASMLFVMACKA | FASMLFVMA |
| | | LFFASMLFVMACKAY | FASMLFVMA |
| | | NSFQDKLAAKLAALK | FQDKLAAKL |
| | | DKLAAKLAALKAAKN | LAAKLAALK |
| | | SFQDKLAAKLAALKA | LAAKLAALK |
| | | FQDKLAAKLAALKAA | LAAKLAALK |
| | | QNSFQDKLAAKLAAL | FQDKLAAKL |
| | | LQNSFQDKLAAKLAA | FQDKLAAKL |
| | | KLAAKLAALKAAKNT | LAALKAAKN |
| | | NLQNSFQDKLAAKLA | FQDKLAAKL |
| | | FFASMLFVMACKAYV | FASMLFVMA |
| | | FASMLFVMACKAYVE | FASMLFVMA |
| | | QDKLAAKLAALKAAK | LAAKLAALK |
| | | LAAKLAALKAAKNTI | LAALKAAKN |
| | | AKLVGVTVPLLGSNT | LVGVTVPLL |
| | | AAKLAALKAAKNTIE | LAALKAAKN |
| | | EAKLVGVTVPLLGSN | LVGVTVPLL |
| | | LNLQNSFQDKLAAKL | LNLQNSFQD |
| | | SEAKLVGVTVPLLGS | LVGVTVPLL |
| | | KLVGVTVPLLGSNTS | LVGVTVPLL |
| | | AKLAALKAAKNTIEN | LAALKAAKN |
| | | WSEAKLVGVTVPLLG | LVGVTVPLL |
| | | LVGVTVPLLGSNTSG | LVGVTVPLL |
| | | IWSEAKLVGVTVPLL | EAKLVGVTV |
| | | GVTVPLLGSNTSGNG | VPLLGSNTS |
| | | VGVTVPLLGSNTSGN | VPLLGSNTS |
| | | VTVPLLGSNTSGNGD | VPLLGSNTS |
| | | SMLFVMACKAYVEEK | FVMACKAYV |
| | | ASMLFVMACKAYVEE | FVMACKAYV |
| | | MLFVMACKAYVEEKK | FVMACKAYV |
| | | KNIFKLFFASMLFVM | FKLFFASML |
| | | NKNIFKLFFASMLFV | FKLFFASML |
| | | KLAALKAAKNTIENI | LAALKAAKN |
| | | LAALKAAKNTIENIT | LAALKAAKN |
| | | MRNKNIFKLFFASML | IFKLFFASM |
| | | RNKNIFKLFFASMLF | FKLFFASML |

| | | | |
|---|---|---|---|
| | | TVPLLGSNTSGNGDK | VPLLGSNTS |
| | | VPLLGSNTSGNGDKM | VPLLGSNTS |
| | | LFVMACKAYVEEKKE | FVMACKAYV |
| | | FVMACKAYVEEKKEI | FVMACKAYV |
| | HLA-DRB1*0301 | None | |
| | HLA-DRB1*0401 | None | |
| | HLA-DRB1*0404 | AELKEKLLNLQNSFQ | LKEKLLNLQ |
| | | LKEKLLNLQNSFQDK | LLNLQNSFQ |
| | | ELKEKLLNLQNSFQD | LLNLQNSFQ |
| | | KEKLLNLQNSFQDKL | LLNLQNSFQ |
| | | EKLLNLQNSFQDKLA | LLNLQNSFQ |
| | | KLLNLQNSFQDKLAA | LLNLQNSFQ |
| | | LLNLQNSFQDKLAAK | LLNLQNSFQ |
| | | GVTVPLLGSNTSGNG | LLGSNTSGN |
| | | VTVPLLGSNTSGNGD | LLGSNTSGN |
| | | VGVTVPLLGSNTSGN | VPLLGSNTS |
| | | TVPLLGSNTSGNGDK | LLGSNTSGN |
| | | VPLLGSNTSGNGDKM | LLGSNTSGN |
| | HLA-DRB1*0405 | None | |
| | HLA-DRB1*0701 | None | |
| | HLA-DRB1*0802 | FFASMLFVMACKAYV | LFVMACKAY |
| | | FASMLFVMACKAYVE | FVMACKAYV |
| | | ASMLFVMACKAYVEE | FVMACKAYV |
| | | SMLFVMACKAYVEEK | FVMACKAYV |
| | | MLFVMACKAYVEEKK | FVMACKAYV |
| | HLA-DRB1*0901 | KNIFKLFFASMLFVM | LFFASMLFV |
| | | NIFKLFFASMLFVMA | LFFASMLFV |
| | | IFKLFFASMLFVMAC | LFFASMLFV |
| | | FKLFFASMLFVMACK | LFFASMLFV |
| | | NKNIFKLFFASMLFV | FKLFFASML |
| | | KLFFASMLFVMACKA | FFASMLFVM |
| | HLA-DRB1*1101 | None | |
| | HLA-DRB1*1302 | KLVGVTVPLLGSNTS | VGVTVPLLG |
| | | GVTVPLLGSNTSGNG | VPLLGSNTS |
| | | VTVPLLGSNTSGNGD | VPLLGSNTS |
| | | VGVTVPLLGSNTSGN | VPLLGSNTS |
| | | LVGVTVPLLGSNTSG | VPLLGSNTS |
| | | WSEAKLVGVTVPLLG | AKLVGVTVP |
| | | SEAKLVGVTVPLLGS | VGVTVPLLG |
| | | VPLLGSNTSGNGDKM | VPLLGSNTS |
| | | TVPLLGSNTSGNGDK | VPLLGSNTS |
| | | EAKLVGVTVPLLGSN | VGVTVPLLG |
| | | AKLVGVTVPLLGSNT | VGVTVPLLG |
| | | ELKEKLLNLQNSFQD | LLNLQNSFQ |
| | | LKEKLLNLQNSFQDK | LLNLQNSFQ |
| | | AELKEKLLNLQNSFQ | LKEKLLNLQ |
| | | KEKLLNLQNSFQDKL | LLNLQNSFQ |
| | | EKLLNLQNSFQDKLA | LLNLQNSFQ |
| | HLA-DRB1*1501 | MEDVLALVNDSSGGK | VLALVNDSS |
| | | EDVLALVNDSSGGKF | VLALVNDSS |
| | | DVLALVNDSSGGKFK | LVNDSSGGK |
| | | KNIFKLFFASMLFVM | IFKLFFASM |
| | | VLALVNDSSGGKFKD | LVNDSSGGK |
| | HLA-DRB3*0101 | None | |
| | HLA-DRB4*0101 | AELKEKLLNLQNSFQ | LKEKLLNLQ |

| | | DIGNAELKEKLLNLQ | DIGNAELKE |
|---|---|---|---|
| | | IGNAELKEKLLNLQN | LKEKLLNLQ |
| | | GNAELKEKLLNLQNS | LKEKLLNLQ |
| | | NAELKEKLLNLQNSF | LKEKLLNLQ |
| | | ELKEKLLNLQNSFQD | LKEKLLNLQ |
| | | LKEKLLNLQNSFQDK | LKEKLLNLQ |
| | | KLVGVTVPLLGSNTS | VGVTVPLLG |
| | HLA-DRB5*0101 | None | |
| | | | |
| NP_045737.1\| BBA64 antigen, P35 [Borrelia burgdorferi B31]<br><br>Seq40 | HLA-DRB1*0101 | IFNLGQILSKLSQDS | LGQILSKLS |
| | | FNLGQILSKLSQDSN | LGQILSKLS |
| | | GKIFNLGQILSKLSQ | LGQILSKLS |
| | | EGKIFNLGQILSKLS | IFNLGQILS |
| | | KIFNLGQILSKLSQD | LGQILSKLS |
| | | LGQILSKLSQDSNYR | ILSKLSQDS |
| | | SKIFFNANSTVHFDS | FFNANSTVH |
| | | KIFFNANSTVHFDSH | FNANSTVHF |
| | | NLGQILSKLSQDSNY | ILSKLSQDS |
| | | FSKIFFNANSTVHFD | FFNANSTVH |
| | | IFSKIFFNANSTVHF | FFNANSTVH |
| | | LIAIFLLHVLTVLIL | LLHVLTVLI |
| | | IFFNANSTVHFDSHE | FNANSTVHF |
| | | DIILDLVNTTTNILA | LVNTTTNIL |
| | | IAIFLLHVLTVLILI | LLHVLTVLI |
| | | NDTLRSKNSRAQFAN | LRSKNSRAQ |
| | | AIFLLHVLTVLILIS | LLHVLTVLI |
| | | LNDTLRSKNSRAQFA | LRSKNSRAQ |
| | | DTLRSKNSRAQFANI | LRSKNSRAQ |
| | | IILDLVNTTTNILAP | LVNTTTNIL |
| | | ILDLVNTTTNILAPI | LVNTTTNIL |
| | | IFLLHVLTVLILISC | LLHVLTVLI |
| | | HDIILDLVNTTTNIL | ILDLVNTTT |
| | | KLIAIFLLHVLTVLI | IFLLHVLTV |
| | | LDLVNTTTNILAPIQ | LVNTTTNIL |
| | | GQILSKLSQDSNYRG | ILSKLSQDS |
| | | EIFSKIFFNANSTVH | IFFNANSTV |
| | | YNTNPDLQTDVSKLN | YNTNPDLQT |
| | | SKLNDTLRSKNSRAQ | NDTLRSKNS |
| | | KETLINRGFSIQLAM | INRGFSIQL |
| | | VKETLINRGFSIQLA | INRGFSIQL |
| | | NKLIAIFLLHVLTVL | LIAIFLLHV |
| | | ETLINRGFSIQLAME | INRGFSIQL |
| | | TLINRGFSIQLAMEE | INRGFSIQL |
| | | KLNDTLRSKNSRAQF | LRSKNSRAQ |
| | | NNKLIAIFLLHVLTV | LIAIFLLHV |
| | | LKNNKLIAIFLLHVL | LIAIFLLHV |
| | | FFNANSTVHFDSHEY | FNANSTVHF |
| | | FLLHVLTVLILISCS | LHVLTVLIL |
| | | TLRSKNSRAQFANIH | LRSKNSRAQ |
| | | LLHVLTVLILISCSL | LLHVLTVLI |
| | | LRSKNSRAQFANIHD | LRSKNSRAQ |
| | | ILKNNKLIAIFLLHV | LKNNKLIAI |
| | | KNNKLIAIFLLHVLT | LIAIFLLHV |
| | | VLTVLILISCSLEVK | ILISCSLEV |
| | | HVLTVLILISCSLEV | VLILISCSL |
| | | LTVLILISCSLEVKD | ILISCSLEV |

| | | | |
|---|---|---|---|
| | | SNYRGLVKETLINRG | YRGLVKETL |
| | | LVKETLINRGFSIQL | LINRGFSIQ |
| | | IDEYNTNPDLQTDVS | YNTNPDLQT |
| | | DEYNTNPDLQTDVSK | YNTNPDLQT |
| | | LHVLTVLILISCSLE | VLILISCSL |
| | | DNILKNNKLIAIFLL | LKNNKLIAI |
| | | NILKNNKLIAIFLLH | LKNNKLIAI |
| | | KDNILKNNKLIAIFL | LKNNKLIAI |
| | | LIDEYNTNPDLQTDV | YNTNPDLQT |
| | | DLIDEYNTNPDLQTD | YNTNPDLQT |
| | | ENIIQISEMDSSRGE | ISEMDSSRG |
| | | DSNYRGLVKETLINR | YRGLVKETL |
| | | SENIIQISEMDSSRG | IIQISEMDS |
| | | DDLIDEYNTNPDLQT | LIDEYNTNP |
| | | IIQISEMDSSRGEPN | ISEMDSSRG |
| | | LINRGFSIQLAMEEI | INRGFSIQL |
| | | NIIQISEMDSSRGEP | ISEMDSSRG |
| | | TVLILISCSLEVKDS | ILISCSLEV |
| | | QILSKLSQDSNYRGL | ILSKLSQDS |
| | | ILSKLSQDSNYRGLV | ILSKLSQDS |
| | | LSQDSNYRGLVKETL | LSQDSNYRG |
| | | IQISEMDSSRGEPND | ISEMDSSRG |
| | | PNDQFGMRAEIFSKI | FGMRAEIFS |
| | | VLILISCSLEVKDSN | ILISCSLEV |
| | | NDQFGMRAEIFSKIF | FGMRAEIFS |
| | | MKDNILKNNKLIAIF | LKNNKLIAI |
| | | NPDLQTDVSKLNDTL | LQTDVSKLN |
| | | NTNPDLQTDVSKLND | LQTDVSKLN |
| | | TNPDLQTDVSKLNDT | LQTDVSKLN |
| | | PDLQTDVSKLNDTLR | LQTDVSKLN |
| | | INRGFSIQLAMEEIS | INRGFSIQL |
| | | NAPKKILDPEVAKLI | KKILDPEVA |
| | | APKKILDPEVAKLIQ | LDPEVAKLI |
| | | NEGKIFNLGQILSKL | IFNLGQILS |
| | | QDSNYRGLVKETLIN | YRGLVKETL |
| | | KKILDPEVAKLIQKI | LDPEVAKLI |
| | | FNEGKIFNLGQILSK | IFNLGQILS |
| | | YRGLVKETLINRGFS | YRGLVKETL |
| | | NYRGLVKETLINRGF | YRGLVKETL |
| | | PKKILDPEVAKLIQK | LDPEVAKLI |
| | | SQDSNYRGLVKETLI | YRGLVKETL |
| | | GEPNDQFGMRAEIFS | QFGMRAEIF |
| | | EPNDQFGMRAEIFSK | FGMRAEIFS |
| | | DQFGMRAEIFSKIFF | FGMRAEIFS |
| | | FNANSTVHFDSHEYT | FNANSTVHF |
| | | EERRMLYTSLNFNEG | RRMLYTSLN |
| | | KILDPEVAKLIQKIL | LDPEVAKLI |
| | | VAINNLKNPTKPAAG | LKNPTKPAA |
| | | INNLKNPTKPAAGKN | LKNPTKPAA |
| | | NNLKNPTKPAAGKNK | LKNPTKPAA |
| | | NIHDIILDLVNTTTN | ILDLVNTTT |
| | | IHDIILDLVNTTTNI | ILDLVNTTT |
| | | AINNLKNPTKPAAGK | LKNPTKPAA |
| | | GKVENLLVAINNLKN | VENLLVAIN |
| | | LVAINNLKNPTKPAA | INNLKNPTK |
| | | ANIHDIILDLVNTTT | IILDLVNTT |
| | | LQTDVSKLNDTLRSK | LQTDVSKLN |

| | | DLQTDVSKLNDTLRS | LQTDVSKLN |
|---|---|---|---|
| | HLA-DRB1*0301 | None | |
| | HLA-DRB1*0401 | IFSKIFFNANSTVHF | FFNANSTVH |
| | | FSKIFFNANSTVHFD | FFNANSTVH |
| | | SKIFFNANSTVHFDS | FFNANSTVH |
| | | KIFFNANSTVHFDSH | FFNANSTVH |
| | | EIFSKIFFNANSTVH | FSKIFFNAN |
| | | IILDLVNTTTNILAP | LVNTTTNIL |
| | | DIILDLVNTTTNILA | LVNTTTNIL |
| | | LDLVNTTTNILAPIQ | LVNTTTNIL |
| | | ILDLVNTTTNILAPI | LVNTTTNIL |
| | | HDIILDLVNTTTNIL | IILDLVNTT |
| | | IFFNANSTVHFDSHE | FFNANSTVH |
| | | FFNANSTVHFDSHEY | FFNANSTVH |
| | | ETLYNDFEKLTSLKE | YNDFEKLTS |
| | | TLYNDFEKLTSLKEK | FEKLTSLKE |
| | | ENLLVAINNLKNPTK | LLVAINNLK |
| | | LYNDFEKLTSLKEKW | FEKLTSLKE |
| | | VENLLVAINNLKNPT | LVAINNLKN |
| | | SCSLEVKDSNESKKH | LEVKDSNES |
| | | CSLEVKDSNESKKHK | LEVKDSNES |
| | | ISCSLEVKDSNESKK | LEVKDSNES |
| | | LISCSLEVKDSNESK | LEVKDSNES |
| | | KVENLLVAINNLKNP | LLVAINNLK |
| | | YNDFEKLTSLKEKWL | FEKLTSLKE |
| | | GKVENLLVAINNLKN | LLVAINNLK |
| | | ILISCSLEVKDSNES | ILISCSLEV |
| | | NDFEKLTSLKEKWLK | FEKLTSLKE |
| | | EERRMLYTSLNFNEG | MLYTSLNFN |
| | | NLLVAINNLKNPTKP | LVAINNLKN |
| | HLA-DRB1*0404 | ENLLVAINNLKNPTK | LLVAINNLK |
| | | HDIILDLVNTTTNIL | ILDLVNTTT |
| | | DIILDLVNTTTNILA | ILDLVNTTT |
| | | VENLLVAINNLKNPT | LLVAINNLK |
| | | KVENLLVAINNLKNP | LLVAINNLK |
| | | GKVENLLVAINNLKN | LLVAINNLK |
| | | KGKVENLLVAINNLK | VENLLVAIN |
| | | ANIHDIILDLVNTTT | IILDLVNTT |
| | | NIHDIILDLVNTTTN | ILDLVNTTT |
| | | IHDIILDLVNTTTNI | ILDLVNTTT |
| | | IILDLVNTTTNILAP | LVNTTTNIL |
| | | ILDLVNTTTNILAPI | LVNTTTNIL |
| | | NLLVAINNLKNPTKP | LLVAINNLK |
| | | LLVAINNLKNPTKPA | LLVAINNLK |
| | | RRMLYTSLNFNEGKI | MLYTSLNFN |
| | | EERRMLYTSLNFNEG | MLYTSLNFN |
| | | ERRMLYTSLNFNEGK | MLYTSLNFN |
| | | DDLIDEYNTNPDLQT | LIDEYNTNP |
| | | EIFSKIFFNANSTVH | FSKIFFNAN |
| | | TDDLIDEYNTNPDLQ | LIDEYNTNP |
| | | KDTDDLIDEYNTNPD | LIDEYNTNP |
| | | GLVKETLINRGFSIQ | VKETLINRG |
| | | DTDDLIDEYNTNPDL | LIDEYNTNP |
| | | LKDTDDLIDEYNTNP | LKDTDDLID |
| | | SNYRGLVKETLINRG | LVKETLINR |
| | | IFSKIFFNANSTVHF | FFNANSTVH |

| | | | |
|---|---|---|---|
| | | YRGLVKETLINRGFS | VKETLINRG |
| | | NYRGLVKETLINRGF | VKETLINRG |
| | | KLIAIFLLHVLTVLI | IFLLHVLTV |
| | | RGLVKETLINRGFSI | VKETLINRG |
| | | NIIQISEMDSSRGEP | ISEMDSSRG |
| | | FSKIFFNANSTVHFD | FFNANSTVH |
| | | YTEERRMLYTSLNFN | ERRMLYTSL |
| | | IIQISEMDSSRGEPN | ISEMDSSRG |
| | | TEERRMLYTSLNFNE | MLYTSLNFN |
| | | IQISEMDSSRGEPND | ISEMDSSRG |
| | | LIAIFLLHVLTVLIL | LLHVLTVLI |
| | | SENIIQISEMDSSRG | IQISEMDSS |
| | | ENIIQISEMDSSRGE | ISEMDSSRG |
| | | SKIFFNANSTVHFDS | FFNANSTVH |
| | | IAIFLLHVLTVLILI | LLHVLTVLI |
| | | KIFFNANSTVHFDSH | FFNANSTVH |
| | | LDLVNTTTNILAPIQ | LVNTTTNIL |
| | | RMLYTSLNFNEGKIF | MLYTSLNFN |
| | | MLYTSLNFNEGKIFN | MLYTSLNFN |
| | HLA-DRB1*0405 | ENLLVAINNLKNPTK | LVAINNLKN |
| | | DDLIDEYNTNPDLQT | IDEYNTNPD |
| | | DLIDEYNTNPDLQTD | IDEYNTNPD |
| | | NLLVAINNLKNPTKP | LVAINNLKN |
| | | GKVENLLVAINNLKN | VENLLVAIN |
| | | KVENLLVAINNLKNP | LVAINNLKN |
| | | VENLLVAINNLKNPT | LVAINNLKN |
| | | ETLYNDFEKLTSLKE | YNDFEKLTS |
| | | TLYNDFEKLTSLKEK | FEKLTSLKE |
| | | IILDLVNTTTNILAP | VNTTTNILA |
| | | KDTDDLIDEYNTNPD | LIDEYNTNP |
| | | DIILDLVNTTTNILA | LVNTTTNIL |
| | | TDDLIDEYNTNPDLQ | IDEYNTNPD |
| | | EERRMLYTSLNFNEG | RRMLYTSLN |
| | | DTDDLIDEYNTNPDL | IDEYNTNPD |
| | | LYNDFEKLTSLKEKW | FEKLTSLKE |
| | | LDLVNTTTNILAPIQ | VNTTTNILA |
| | | ILDLVNTTTNILAPI | VNTTTNILA |
| | | YNDFEKLTSLKEKWL | FEKLTSLKE |
| | | TEERRMLYTSLNFNE | RRMLYTSLN |
| | | HEYTEERRMLYTSLN | YTEERRMLY |
| | | LLVAINNLKNPTKPA | LVAINNLKN |
| | | NDFEKLTSLKEKWLK | FEKLTSLKE |
| | | LIDEYNTNPDLQTDV | IDEYNTNPD |
| | | YTEERRMLYTSLNFN | RRMLYTSLN |
| | | IDEYNTNPDLQTDVS | IDEYNTNPD |
| | | LVAINNLKNPTKPAA | LVAINNLKN |
| | | EYTEERRMLYTSLNF | RRMLYTSLN |
| | | YNTNPDLQTDVSKLN | YNTNPDLQT |
| | | ERRMLYTSLNFNEGK | RRMLYTSLN |
| | HLA-DRB1*0701 | EIFSKIFFNANSTVH | IFFNANSTV |
| | | IFSKIFFNANSTVHF | FFNANSTVH |
| | | FSKIFFNANSTVHFD | FFNANSTVH |
| | | SKIFFNANSTVHFDS | FFNANSTVH |
| | | KIFFNANSTVHFDSH | FFNANSTVH |
| | | IFFNANSTVHFDSHE | FFNANSTVH |
| | | FFNANSTVHFDSHEY | FFNANSTVH |
| | | HDIILDLVNTTTNIL | DLVNTTTNI |

| | | | |
|---|---|---|---|
| | | DIILDLVNTTTNILA | LVNTTTNIL |
| | | ILDLVNTTTNILAPI | LVNTTTNIL |
| | | LDLVNTTTNILAPIQ | LVNTTTNIL |
| | | IILDLVNTTTNILAP | LVNTTTNIL |
| | | LNDTLRSKNSRAQFA | LRSKNSRAQ |
| | | NDTLRSKNSRAQFAN | LRSKNSRAQ |
| | | DTLRSKNSRAQFANI | LRSKNSRAQ |
| | | LIAIFLLHVLTVLIL | LLHVLTVLI |
| | | IAIFLLHVLTVLILI | LHVLTVLIL |
| | | TLRSKNSRAQFANIH | SKNSRAQFA |
| | | LRSKNSRAQFANIHD | SKNSRAQFA |
| | HLA-DRB1*0802 | None | |
| | HLA-DRB1*0901 | None | |
| | HLA-DRB1*1101 | RMLYTSLNFNEGKIF | YTSLNFNEG |
| | | RRMLYTSLNFNEGKI | YTSLNFNEG |
| | | ERRMLYTSLNFNEGK | YTSLNFNEG |
| | | MLYTSLNFNEGKIFN | YTSLNFNEG |
| | | EERRMLYTSLNFNEG | MLYTSLNFN |
| | HLA-DRB1*1302 | MKDNILKNNKLIAIF | LKNNKLIAI |
| | | KDNILKNNKLIAIFL | LKNNKLIAI |
| | | DNILKNNKLIAIFLL | LKNNKLIAI |
| | | NILKNNKLIAIFLLH | LKNNKLIAI |
| | | ENLLVAINNLKNPTK | AINNLKNPT |
| | | DIILDLVNTTTNILA | LVNTTTNIL |
| | | HDIILDLVNTTTNIL | IILDLVNTT |
| | | IILDLVNTTTNILAP | LVNTTTNIL |
| | | NLLVAINNLKNPTKP | AINNLKNPT |
| | | VENLLVAINNLKNPT | VAINNLKNP |
| | | ILDLVNTTTNILAPI | LVNTTTNIL |
| | | LLVAINNLKNPTKPA | AINNLKNPT |
| | | LDLVNTTTNILAPIQ | LVNTTTNIL |
| | | GKIFNLGQILSKLSQ | IFNLGQILS |
| | | EGKIFNLGQILSKLS | IFNLGQILS |
| | | ILKNNKLIAIFLLHV | LKNNKLIAI |
| | | LVAINNLKNPTKPAA | AINNLKNPT |
| | | LKNNKLIAIFLLHVL | LKNNKLIAI |
| | | NEGKIFNLGQILSKL | IFNLGQILS |
| | | FNEGKIFNLGQILSK | IFNLGQILS |
| | | NFNEGKIFNLGQILS | GKIFNLGQI |
| | | KVENLLVAINNLKNP | LLVAINNLK |
| | | IFLLHVLTVLILISC | LLHVLTVLI |
| | | AIFLLHVLTVLILIS | LLHVLTVLI |
| | | KIFNLGQILSKLSQD | IFNLGQILS |
| | | IFNLGQILSKLSQDS | IFNLGQILS |
| | | VAINNLKNPTKPAAG | AINNLKNPT |
| | | IAIFLLHVLTVLILI | LLHVLTVLI |
| | | LIAIFLLHVLTVLIL | LLHVLTVLI |
| | | FLLHVLTVLILISCS | LLHVLTVLI |
| | | KLIAIFLLHVLTVLI | IAIFLLHVL |
| | | IFSKIFFNANSTVHF | FFNANSTVH |
| | | FSKIFFNANSTVHFD | FNANSTVHF |
| | | SKIFFNANSTVHFDS | FNANSTVHF |
| | | LLHVLTVLILISCSL | LLHVLTVLI |
| | | KIFFNANSTVHFDSH | FNANSTVHF |
| | | IHDIILDLVNTTTNI | IILDLVNTT |
| | | IFFNANSTVHFDSHE | FNANSTVHF |

| | | GKVENLLVAINNLKN | LLVAINNLK |
|---|---|---|---|
| | | KGKVENLLVAINNLK | VENLLVAIN |
| | | AINNLKNPTKPAAGK | AINNLKNPT |
| | | GLVKETLINRGFSIQ | VKETLINRG |
| | | NIHDIILDLVNTTTN | IILDLVNTT |
| | | LHVLTVLILISCSLE | VLTVLILIS |
| | | LVKETLINRGFSIQL | LINRGFSIQ |
| | | VKETLINRGFSIQLA | LINRGFSIQ |
| | | ANIHDIILDLVNTTT | IILDLVNTT |
| | | HVLTVLILISCSLEV | LTVLILISC |
| | | KETLINRGFSIQLAM | LINRGFSIQ |
| | | FANIHDIILDLVNTT | IHDIILDLV |
| | | ETLINRGFSIQLAME | LINRGFSIQ |
| | | NKLIAIFLLHVLTVL | IAIFLLHVL |
| | | DKLQQLNKPNLETLY | LQQLNKPNL |
| | | VKDKLQQLNKPNLET | LQQLNKPNL |
| | | NVKDKLQQLNKPNLE | LQQLNKPNL |
| | | VLTVLILISCSLEVK | LTVLILISC |
| | | FFNANSTVHFDSHEY | FNANSTVHF |
| | | KDKLQQLNKPNLETL | LQQLNKPNL |
| | | EEISAKILNVKDKLQ | ISAKILNVK |
| | | DPEVAKLIQKILDRS | VAKLIQKIL |
| | | PEVAKLIQKILDRSE | LIQKILDRS |
| | | ISAKILNVKDKLQQL | ISAKILNVK |
| | | EISAKILNVKDKLQQ | ISAKILNVK |
| | | EVAKLIQKILDRSEN | LIQKILDRS |
| | | LNVKDKLQQLNKPNL | DKLQQLNKP |
| | HLA-DRB1*1501 | ENLLVAINNLKNPTK | LLVAINNLK |
| | | NLLVAINNLKNPTKP | INNLKNPTK |
| | | LLVAINNLKNPTKPA | INNLKNPTK |
| | | NKLIAIFLLHVLTVL | LIAIFLLHV |
| | | LIAIFLLHVLTVLIL | IFLLHVLTV |
| | | EIFSKIFFNANSTVH | SKIFFNANS |
| | | IFSKIFFNANSTVHF | FFNANSTVH |
| | | LVAINNLKNPTKPAA | INNLKNPTK |
| | | VAINNLKNPTKPAAG | INNLKNPTK |
| | | IAIFLLHVLTVLILI | IFLLHVLTV |
| | | SNYRGLVKETLINRG | LVKETLINR |
| | | KLIAIFLLHVLTVLI | IFLLHVLTV |
| | | FSKIFFNANSTVHFD | FFNANSTVH |
| | | SKIFFNANSTVHFDS | FFNANSTVH |
| | | YRGLVKETLINRGFS | LVKETLINR |
| | | RGLVKETLINRGFSI | LVKETLINR |
| | | NYRGLVKETLINRGF | LVKETLINR |
| | | NNKLIAIFLLHVLTV | LIAIFLLHV |
| | | DSNYRGLVKETLINR | YRGLVKETL |
| | | AIFLLHVLTVLILIS | LHVLTVLIL |
| | | KIFFNANSTVHFDSH | FFNANSTVH |
| | | IFLLHVLTVLILISC | LHVLTVLIL |
| | | LKNNKLIAIFLLHVL | LIAIFLLHV |
| | | VLTVLILISCSLEVK | VLILISCSL |
| | | GLVKETLINRGFSIQ | LVKETLINR |
| | | LTVLILISCSLEVKD | VLILISCSL |
| | | LVKETLINRGFSIQL | LVKETLINR |
| | | VENLLVAINNLKNPT | LLVAINNLK |
| | | FLLHVLTVLILISCS | LHVLTVLIL |
| | | KVENLLVAINNLKNP | LLVAINNLK |

| | | | |
|---|---|---|---|
| | | HVLTVLILISCSLEV | VLILISCSL |
| | | LLHVLTVLILISCSL | LTVLILISC |
| | | KNNKLIAIFLLHVLT | LIAIFLLHV |
| | | LHVLTVLILISCSLE | VLILISCSL |
| | | AINNLKNPTKPAAGK | INNLKNPTK |
| | | ILKNNKLIAIFLLHV | LKNNKLIAI |
| | | SKLNDTLRSKNSRAQ | TLRSKNSRA |
| | HLA-DRB3*0101 | None | |
| | HLA-DRB4*0101 | AKLIQKILDRSENII | LIQKILDRS |
| | HLA-DRB5*0101 | VAINNLKNPTKPAAG | INNLKNPTK |
| | | ENLLVAINNLKNPTK | LLVAINNLK |
| | | LVAINNLKNPTKPAA | INNLKNPTK |
| | | NLLVAINNLKNPTKP | INNLKNPTK |
| | | SKLNDTLRSKNSRAQ | LNDTLRSKN |
| | | LLVAINNLKNPTKPA | INNLKNPTK |
| | | KLNDTLRSKNSRAQF | LRSKNSRAQ |
| | | LNDTLRSKNSRAQFA | LRSKNSRAQ |
| | | | |
| AAT93822.1| BBA64 antigen, P35 [Borrelia garinii PBi]<br><br>Seq41 | HLA-DRB1*0101 | IFSKIFFNAGSTVTF | FFNAGSTVT |
| | | FSKIFFNAGSTVTFD | FFNAGSTVT |
| | | SKIFFNAGSTVTFDD | FFNAGSTVT |
| | | KIFFNAGSTVTFDDN | FFNAGSTVT |
| | | EIFSKIFFNAGSTVT | EIFSKIFFN |
| | | IFFNAGSTVTFDDNE | FFNAGSTVT |
| | | FFNAGSTVTFDDNEY | FFNAGSTVT |
| | | ILNLGKILSKLSQDS | LGKILSKLS |
| | | LNLGKILSKLSQDSN | LGKILSKLS |
| | | NKILNLGKILSKLSQ | LGKILSKLS |
| | | ENKILNLGKILSKLS | ILNLGKILS |
| | | FLLHILTGLILLSCS | LTGLILLSC |
| | | IFLLHILTGLILLSC | ILTGLILLS |
| | | LGKILSKLSQDSNYR | ILSKLSQDS |
| | | NIILNLINTTTNILA | ILNLINTTT |
| | | NLGKILSKLSQDSNY | ILSKLSQDS |
| | | LHILTGLILLSCSLE | ILTGLILLS |
| | | LLHILTGLILLSCSL | ILTGLILLS |
| | | HNIILNLINTTTNIL | ILNLINTTT |
| | | KILNLGKILSKLSQD | LGKILSKLS |
| | | TDIHNIILNLINTTT | IHNIILNLI |
| | | IILNLINTTTNILAP | LINTTTNIL |
| | | AIFLLHILTGLILLS | LHILTGLIL |
| | | ILNLINTTTNILAPI | LINTTTNIL |
| | | GKILSKLSQDSNYRS | ILSKLSQDS |
| | | DIHNIILNLINTTTN | ILNLINTTT |
| | | IHNIILNLINTTTNI | ILNLINTTT |
| | | HILTGLILLSCSLEV | LTGLILLSC |
| | | VKEILINRGFSIQLA | INRGFSIQL |
| | | IKDYNANPELRTDIS | YNANPELRT |
| | | LVAINTLKNPPKTAG | LVAINTLKN |
| | | KEILINRGFSIQLAI | INRGFSIQL |
| | | DIIKDYNANPELRTD | YNANPELRT |
| | | IIKDYNANPELRTDI | YNANPELRT |
| | | KDYNANPELRTDISK | YNANPELRT |
| | | DDIIKDYNANPELRT | DYNANPELR |
| | | EILINRGFSIQLAIE | INRGFSIQL |
| | | INTLKNPPKTAGKNK | LKNPPKTAG |

| | | | |
|---|---|---|---|
| | | YNANPELRTDISKLN | YNANPELRT |
| | | AINTLKNPPKTAGKN | LKNPPKTAG |
| | | LNLINTTTNILAPIQ | LINTTTNIL |
| | | NTLKNPPKTAGKNKS | LKNPPKTAG |
| | | ILINRGFSIQLAIEE | INRGFSIQL |
| | | VAINTLKNPPKTAGK | LKNPPKTAG |
| | | LVKEILINRGFSIQL | ILINRGFSI |
| | | LIAIFLLHILTGLIL | LLHILTGLI |
| | | IAIFLLHILTGLILL | LHILTGLIL |
| | | ILTGLILLSCSLEVN | ILTGLILLS |
| | | FNAGSTVTFDDNEYV | FNAGSTVTF |
| | | PTVDNLLVAINTLKN | VDNLLVAIN |
| | | TVDNLLVAINTLKNP | LVAINTLKN |
| | | NDYIISKNSKAQFTD | SKNSKAQFT |
| | | LNDYIISKNSKAQFT | IISKNSKAQ |
| | | DNLLVAINTLKNPPK | LVAINTLKN |
| | | VDNLLVAINTLKNPP | LVAINTLKN |
| | | DYIISKNSKAQFTDI | SKNSKAQFT |
| | | FTDIHNIILNLINTT | IHNIILNLI |
| | | LTGLILLSCSLEVNQ | LTGLILLSC |
| | | AQFTDIHNIILNLIN | IHNIILNLI |
| | | KILSKLSQDSNYRSL | ILSKLSQDS |
| | | MKNNKLIAIFLLHIL | LIAIFLLHI |
| | | NLLVAINTLKNPPKT | LVAINTLKN |
| | | ILSKLSQDSNYRSLV | ILSKLSQDS |
| | | QFTDIHNIILNLINT | IHNIILNLI |
| | | KAQFTDIHNIILNLI | FTDIHNIIL |
| | | NKLIAIFLLHILTGL | LIAIFLLHI |
| | | SLVKEILINRGFSIQ | VKEILINRG |
| | | NNKLIAIFLLHILTG | LIAIFLLHI |
| | | KLIAIFLLHILTGLI | IAIFLLHIL |
| | | SNYRSLVKEILINRG | YRSLVKEIL |
| | | DYNANPELRTDISKL | YNANPELRT |
| | | LINRGFSIQLAIEEI | INRGFSIQL |
| | | KNNKLIAIFLLHILT | LIAIFLLHI |
| | | YIISKNSKAQFTDIH | SKNSKAQFT |
| | | TLKNPPKTAGKNKSN | LKNPPKTAG |
| | | NANPELRTDISKLND | LRTDISKLN |
| | | RSLVKEILINRGFSI | VKEILINRG |
| | | LKNPPKTAGKNKSNS | LKNPPKTAG |
| | | PELRTDISKLNDYII | LRTDISKLN |
| | | NPELRTDISKLNDYI | LRTDISKLN |
| | | ANPELRTDISKLNDY | LRTDISKLN |
| | | IISKNSKAQFTDIHN | SKNSKAQFT |
| | | KPTVDNLLVAINTLK | VDNLLVAIN |
| | | AIEEISLRTLNVKDK | EISLRTLNV |
| | | IEEISLRTLNVKDKI | LRTLNVKDK |
| | | NENKILNLGKILSKL | ILNLGKILS |
| | | YRSLVKEILINRGFS | VKEILINRG |
| | | TGLILLSCSLEVNQD | ILLSCSLEV |
| | | NYRSLVKEILINRGF | VKEILINRG |
| | | INRGFSIQLAIEEIS | INRGFSIQL |
| | | FNENKILNLGKILSK | ILNLGKILS |
| | | LLVAINTLKNPPKTA | LVAINTLKN |
| | | GLILLSCSLEVNQDD | ILLSCSLEV |
| | | IQLAIEEISLRTLNV | LAIEEISLR |
| | | SKLNDYIISKNSKAQ | YIISKNSKA |

| | | | |
|---|---|---|---|
| | | KLNDYIISKNSKAQF | IISKNSKAQ |
| | | AKELIQKILERSEDI | LIQKILERS |
| | | EAKELIQKILERSED | LIQKILERS |
| | | PEAKELIQKILERSE | LIQKILERS |
| | | KELIQKILERSEDIV | LIQKILERS |
| | HLA-DRB1*0301 | None | |
| | HLA-DRB1*0401 | IILNLINTTTNILAP | LINTTTNIL |
| | | NIILNLINTTTNILA | LINTTTNIL |
| | | LNLINTTTNILAPIQ | LINTTTNIL |
| | | ILNLINTTTNILAPI | LINTTTNIL |
| | | HNIILNLINTTTNIL | LNLINTTTN |
| | | DNLLVAINTLKNPPK | LVAINTLKN |
| | | VDNLLVAINTLKNPP | LVAINTLKN |
| | | NLLVAINTLKNPPKT | LVAINTLKN |
| | | TVDNLLVAINTLKNP | LVAINTLKN |
| | | IFSKIFFNAGSTVTF | FFNAGSTVT |
| | | PTVDNLLVAINTLKN | LLVAINTLK |
| | | FSKIFFNAGSTVTFD | FFNAGSTVT |
| | | SKIFFNAGSTVTFDD | FFNAGSTVT |
| | | KIFFNAGSTVTFDDN | FFNAGSTVT |
| | | EIFSKIFFNAGSTVT | FSKIFFNAG |
| | | NEYVNERRILYTSLN | YVNERRILY |
| | | LVAINTLKNPPKTAG | LVAINTLKN |
| | | DIHNIILNLINTTTN | IILNLINTT |
| | | IHNIILNLINTTTNI | IILNLINTT |
| | | LLVAINTLKNPPKTA | LVAINTLKN |
| | HLA-DRB1*0404 | HNIILNLINTTTNIL | ILNLINTTT |
| | | NIILNLINTTTNILA | ILNLINTTT |
| | | TDIHNIILNLINTTT | IILNLINTT |
| | | DIHNIILNLINTTTN | ILNLINTTT |
| | | IHNIILNLINTTTNI | ILNLINTTT |
| | | IILNLINTTTNILAP | ILNLINTTT |
| | | KLIAIFLLHILTGLI | IFLLHILTG |
| | | ILNLINTTTNILAPI | ILNLINTTT |
| | | LIAIFLLHILTGLIL | LLHILTGLI |
| | | IAIFLLHILTGLILL | LLHILTGLI |
| | | IFLLHILTGLILLSC | LLHILTGLI |
| | | AIFLLHILTGLILLS | LLHILTGLI |
| | | VDNLLVAINTLKNPP | LLVAINTLK |
| | | DNLLVAINTLKNPPK | LLVAINTLK |
| | | NLLVAINTLKNPPKT | LLVAINTLK |
| | | RRILYTSLNFNENKI | ILYTSLNFN |
| | | ERRILYTSLNFNENK | ILYTSLNFN |
| | | NERRILYTSLNFNEN | ILYTSLNFN |
| | | YVNERRILYTSLNFN | ERRILYTSL |
| | | EIFSKIFFNAGSTVT | FSKIFFNAG |
| | | TVDNLLVAINTLKNP | LLVAINTLK |
| | | VNERRILYTSLNFNE | ILYTSLNFN |
| | | LLVAINTLKNPPKTA | LLVAINTLK |
| | | PTVDNLLVAINTLKN | LLVAINTLK |
| | | FLLHILTGLILLSCS | LLHILTGLI |
| | | LLHILTGLILLSCSL | LLHILTGLI |
| | | LNLINTTTNILAPIQ | LINTTTNIL |
| | | IFSKIFFNAGSTVTF | FFNAGSTVT |
| | | DDIIKDYNANPELRT | IIKDYNANP |

|  |  | KDVDDIIKDYNANPE | IIKDYNANP |
|---|---|---|---|
|  |  | VDDIIKDYNANPELR | IIKDYNANP |
|  |  | DVDDIIKDYNANPEL | IIKDYNANP |
|  |  | FSKIFFNAGSTVTFD | FFNAGSTVT |
|  |  | SLVKEILINRGFSIQ | VKEILINRG |
|  |  | KPTVDNLLVAINTLK | VDNLLVAIN |
|  |  | RILYTSLNFNENKIL | ILYTSLNFN |
|  | HLA-DRB1*0405 | DNLLVAINTLKNPPK | LVAINTLKN |
|  |  | NLLVAINTLKNPPKT | LVAINTLKN |
|  |  | LLVAINTLKNPPKTA | LVAINTLKN |
|  |  | LVAINTLKNPPKTAG | LVAINTLKN |
|  |  | PTVDNLLVAINTLKN | VDNLLVAIN |
|  |  | VDNLLVAINTLKNPP | LVAINTLKN |
|  |  | TVDNLLVAINTLKNP | LVAINTLKN |
|  |  | IILNLINTTTNILAP | INTTTNILA |
|  |  | NIILNLINTTTNILA | LNLINTTTN |
|  |  | LNLINTTTNILAPIQ | INTTTNILA |
|  |  | ILNLINTTTNILAPI | INTTTNILA |
|  |  | NERRILYTSLNFNEN | RRILYTSLN |
|  |  | KTLYHDFNKLIPLKE | LYHDFNKLI |
|  |  | ERRILYTSLNFNENK | YTSLNFNEN |
|  |  | TLYHDFNKLIPLKEK | FNKLIPLKE |
|  |  | RRILYTSLNFNENKI | YTSLNFNEN |
|  |  | HNIILNLINTTTNIL | LNLINTTTN |
|  |  | VNERRILYTSLNFNE | RRILYTSLN |
|  |  | VAINTLKNPPKTAGK | INTLKNPPK |
|  |  | YNANPELRTDISKLN | YNANPELRT |
|  |  | LYHDFNKLIPLKEKW | FNKLIPLKE |
|  |  | NEYVNERRILYTSLN | YVNERRILY |
|  | HLA-DRB1*0701 | HNIILNLINTTTNIL | ILNLINTTT |
|  |  | NIILNLINTTTNILA | LINTTTNIL |
|  |  | ILNLINTTTNILAPI | LINTTTNIL |
|  |  | LNLINTTTNILAPIQ | LINTTTNIL |
|  |  | IFSKIFFNAGSTVTF | FFNAGSTVT |
|  |  | FSKIFFNAGSTVTFD | FFNAGSTVT |
|  |  | IILNLINTTTNILAP | LINTTTNIL |
|  |  | EIFSKIFFNAGSTVT | IFFNAGSTV |
|  |  | SKIFFNAGSTVTFDD | FFNAGSTVT |
|  |  | KIFFNAGSTVTFDDN | FFNAGSTVT |
|  |  | IFFNAGSTVTFDDNE | FFNAGSTVT |
|  |  | FFNAGSTVTFDDNEY | FFNAGSTVT |
|  | HLA-DRB1*0802 | NNSSKMKKLSKNAKN | MKKLSKNAK |
|  |  | ENNSSKMKKLSKNAK | NSSKMKKLS |
|  |  | SKMKKLSKNAKNKKP | MKKLSKNAK |
|  |  | NSSKMKKLSKNAKNK | MKKLSKNAK |
|  |  | SSKMKKLSKNAKNKK | MKKLSKNAK |
|  |  | HDFNKLIPLKEKWLK | FNKLIPLKE |
|  |  | YHDFNKLIPLKEKWL | FNKLIPLKE |
|  |  | LYHDFNKLIPLKEKW | FNKLIPLKE |
|  |  | KTLYHDFNKLIPLKE | YHDFNKLIP |
|  |  | TLYHDFNKLIPLKEK | FNKLIPLKE |
|  |  | KMKKLSKNAKNKKPT | MKKLSKNAK |
|  |  | MKKLSKNAKNKKPTV | MKKLSKNAK |
|  | HLA-DRB1*0901 | IFSKIFFNAGSTVTF | FFNAGSTVT |
|  |  | FSKIFFNAGSTVTFD | FFNAGSTVT |
|  |  | SKIFFNAGSTVTFDD | FFNAGSTVT |

| | | | |
|---|---|---|---|
| | | KIFFNAGSTVTFDDN | FFNAGSTVT |
| | | EIFSKIFFNAGSTVT | KIFFNAGST |
| | | IFFNAGSTVTFDDNE | FFNAGSTVT |
| | HLA-DRB1*1101 | SSKMKKLSKNAKNKK | MKKLSKNAK |
| | | SKMKKLSKNAKNKKP | MKKLSKNAK |
| | | ENNSSKMKKLSKNAK | SKMKKLSKN |
| | | NSSKMKKLSKNAKNK | MKKLSKNAK |
| | | NNSSKMKKLSKNAKN | MKKLSKNAK |
| | HLA-DRB1*1302 | NFNENKILNLGKILS | NKILNLGKI |
| | | FNENKILNLGKILSK | ILNLGKILS |
| | | NENKILNLGKILSKL | ILNLGKILS |
| | | ENKILNLGKILSKLS | ILNLGKILS |
| | | NKILNLGKILSKLSQ | ILNLGKILS |
| | | HNIILNLINTTTNIL | ILNLINTTT |
| | | DIHNIILNLINTTTN | IILNLINTT |
| | | IHNIILNLINTTTNI | IILNLINTT |
| | | NIILNLINTTTNILA | ILNLINTTT |
| | | TDIHNIILNLINTTT | IHNIILNLI |
| | | IILNLINTTTNILAP | LINTTTNIL |
| | | FTDIHNIILNLINTT | IHNIILNLI |
| | | ILNLINTTTNILAPI | LINTTTNIL |
| | | KILNLGKILSKLSQD | ILNLGKILS |
| | | ILNLGKILSKLSQDS | ILNLGKILS |
| | | LNLINTTTNILAPIQ | LINTTTNIL |
| | | QFTDIHNIILNLINT | IHNIILNLI |
| | | KAQFTDIHNIILNLI | DIHNIILNL |
| | | AQFTDIHNIILNLIN | IHNIILNLI |
| | | MKNNKLIAIFLLHIL | MKNNKLIAI |
| | | DNLLVAINTLKNPPK | VAINTLKNP |
| | | IFSKIFFNAGSTVTF | FFNAGSTVT |
| | | FSKIFFNAGSTVTFD | FFNAGSTVT |
| | | SLVKEILINRGFSIQ | VKEILINRG |
| | | SKIFFNAGSTVTFDD | FFNAGSTVT |
| | | TVDNLLVAINTLKNP | LLVAINTLK |
| | | VDNLLVAINTLKNPP | VAINTLKNP |
| | | NLLVAINTLKNPPKT | VAINTLKNP |
| | | KIFFNAGSTVTFDDN | FFNAGSTVT |
| | | LVKEILINRGFSIQL | LINRGFSIQ |
| | | VKEILINRGFSIQLA | LINRGFSIQ |
| | | SKMKKLSKNAKNKKP | MKKLSKNAK |
| | | SSKMKKLSKNAKNKK | MKKLSKNAK |
| | | LLVAINTLKNPPKTA | VAINTLKNP |
| | | LVAINTLKNPPKTAG | VAINTLKNP |
| | | KEILINRGFSIQLAI | LINRGFSIQ |
| | | IAIFLLHILTGLILL | LHILTGLIL |
| | | MKKLSKNAKNKKPTV | LSKNAKNKK |
| | | KMKKLSKNAKNKKPT | LSKNAKNKK |
| | | LIAIFLLHILTGLIL | LLHILTGLI |
| | | AIFLLHILTGLILLS | LHILTGLIL |
| | | EIFSKIFFNAGSTVT | IFFNAGSTV |
| | | PTVDNLLVAINTLKN | LLVAINTLK |
| | | ENNSSKMKKLSKNAK | SKMKKLSKN |
| | | NNSSKMKKLSKNAKN | MKKLSKNAK |
| | | NSSKMKKLSKNAKNK | MKKLSKNAK |
| | | IFLLHILTGLILLSC | LHILTGLIL |
| | | IFFNAGSTVTFDDNE | FFNAGSTVT |
| | | LNFNENKILNLGKIL | NENKILNLG |

| | | | |
|---|---|---|---|
| | | VAINTLKNPPKTAGK | LKNPPKTAG |
| | | SLNFNENKILNLGKI | NENKILNLG |
| | | KLIAIFLLHILTGLI | IAIFLLHIL |
| | | EILINRGFSIQLAIE | LINRGFSIQ |
| | | FLLHILTGLILLSCS | LHILTGLIL |
| | | KKLSKNAKNKKPTVD | LSKNAKNKK |
| | | VKDKIQHLNKPNLKT | IQHLNKPNL |
| | | LNVKDKIQHLNKPNL | VKDKIQHLN |
| | | DKIQHLNKPNLKTLY | IQHLNKPNL |
| | | NEYVNERRILYTSLN | YVNERRILY |
| | | NVKDKIQHLNKPNLK | IQHLNKPNL |
| | | RSLVKEILINRGFSI | VKEILINRG |
| | | KPTVDNLLVAINTLK | VDNLLVAIN |
| | | DNEYVNERRILYTSL | YVNERRILY |
| | | YTSLNFNENKILNLG | LNFNENKIL |
| | | SNYRSLVKEILINRG | YRSLVKEIL |
| | | DDNEYVNERRILYTS | YVNERRILY |
| | | FDDNEYVNERRILYT | YVNERRILY |
| | | TSLNFNENKILNLGK | NENKILNLG |
| | | KDKIQHLNKPNLKTL | IQHLNKPNL |
| | | FFNAGSTVTFDDNEY | FFNAGSTVT |
| | | YRSLVKEILINRGFS | VKEILINRG |
| | | | |
| | | TFDDNEYVNERRILY | EYVNERRIL |
| | | NKLIAIFLLHILTGL | IAIFLLHIL |
| | | KIQHLNKPNLKTLYH | IQHLNKPNL |
| | | LLHILTGLILLSCSL | LHILTGLIL |
| | | IEEISLRTLNVKDKI | ISLRTLNVK |
| | | NYRSLVKEILINRGF | VKEILINRG |
| | | EEISLRTLNVKDKIQ | ISLRTLNVK |
| | | ILINRGFSIQLAIEE | LINRGFSIQ |
| | | YVNERRILYTSLNFN | YVNERRILY |
| | | IQHLNKPNLKTLYHD | IQHLNKPNL |
| | | AINTLKNPPKTAGKN | LKNPPKTAG |
| | | LHILTGLILLSCSLE | LHILTGLIL |
| | | EYVNERRILYTSLNF | YVNERRILY |
| | | KLSKNAKNKKPTVDN | LSKNAKNKK |
| | | LNLGKILSKLSQDSN | LGKILSKLS |
| | | LSKNAKNKKPTVDNL | LSKNAKNKK |
| | | ISLRTLNVKDKIQHL | ISLRTLNVK |
| | HLA-DRB1*1501 | IAIFLLHILTGLILL | IFLLHILTG |
| | | LIAIFLLHILTGLIL | IFLLHILTG |
| | | DNLLVAINTLKNPPK | LLVAINTLK |
| | | IFLLHILTGLILLSC | IFLLHILTG |
| | | RSLVKEILINRGFSI | LVKEILINR |
| | | ENKILNLGKILSKLS | ILNLGKILS |
| | | AIFLLHILTGLILLS | IFLLHILTG |
| | | NKILNLGKILSKLSQ | ILNLGKILS |
| | | KLIAIFLLHILTGLI | IFLLHILTG |
| | | NENKILNLGKILSKL | ILNLGKILS |
| | | NKLIAIFLLHILTGL | IFLLHILTG |
| | | FNENKILNLGKILSK | ILNLGKILS |
| | | SLVKEILINRGFSIQ | LVKEILINR |
| | | SNYRSLVKEILINRG | LVKEILINR |
| | | YRSLVKEILINRGFS | LVKEILINR |
| | | NYRSLVKEILINRGF | LVKEILINR |
| | | LVKEILINRGFSIQL | LVKEILINR |

| | | | |
|---|---|---|---|
| | | TDIHNIILNLINTTT | IHNIILNLI |
| | | NNKLIAIFLLHILTG | LIAIFLLHI |
| | | NLLVAINTLKNPPKT | INTLKNPPK |
| | | LVAINTLKNPPKTAG | INTLKNPPK |
| | | LLVAINTLKNPPKTA | INTLKNPPK |
| | | IHNIILNLINTTTNI | ILNLINTTT |
| | | DSNYRSLVKEILINR | YRSLVKEIL |
| | | DIHNIILNLINTTTN | ILNLINTTT |
| | | KILNLGKILSKLSQD | LNLGKILSK |
| | | VAINTLKNPPKTAGK | INTLKNPPK |
| | | ILNLGKILSKLSQDS | ILNLGKILS |
| | | HNIILNLINTTTNIL | ILNLINTTT |
| | | VDNLLVAINTLKNPP | LLVAINTLK |
| | | NIILNLINTTTNILA | ILNLINTTT |
| | | FLLHILTGLILLSCS | LHILTGLIL |
| | | IQLAIEEISLRTLNV | LAIEEISLR |
| | HLA-DRB3*0101 | None | |
| | HLA-DRB4*0101 | IEEISLRTLNVKDKI | ISLRTLNVK |
| | | QLAIEEISLRTLNVK | IEEISLRTL |
| | | AIEEISLRTLNVKDK | ISLRTLNVK |
| | | LAIEEISLRTLNVKD | ISLRTLNVK |
| | | EEISLRTLNVKDKIQ | ISLRTLNVK |
| | | EISLRTLNVKDKIQH | ISLRTLNVK |
| | | ISLRTLNVKDKIQHL | ISLRTLNVK |
| | | SLVKEILINRGFSIQ | VKEILINRG |
| | | | |
| | HLA-DRB5*0101 | FDDNEYVNERRILYT | YVNERRILY |
| | | | |
| AAL84596.1\| BBK32 [Borrelia burgdorferi]  Seq42 | HLA-DRB1*0101 | TLFSTKLTQMYSTRL | FSTKLTQMY |
| | | LFSTKLTQMYSTRLD | LTQMYSTRL |
| | | FSTKLTQMYSTRLDN | LTQMYSTRL |
| | | STKLTQMYSTRLDNL | LTQMYSTRL |
| | | TKLTQMYSTRLDNLA | LTQMYSTRL |
| | | KLTQMYSTRLDNLAK | LTQMYSTRL |
| | | LTQMYSTRLDNLAKA | LTQMYSTRL |
| | | YDTYTLFSTKLTQMY | YTLFSTKLT |
| | | DTYTLFSTKLTQMYS | YTLFSTKLT |
| | | NLYEAYKAIVTSILL | YKAIVTSIL |
| | | SNLYEAYKAIVTSIL | YEAYKAIVT |
| | | YEAYKAIVTSILLMR | YKAIVTSIL |
| | | EAYKAIVTSILLMRD | YKAIVTSIL |
| | | LYEAYKAIVTSILLM | YKAIVTSIL |
| | | KKVKSKYLALGLLFG | VKSKYLALG |
| | | IYDTYTLFSTKLTQM | YTLFSTKLT |
| | | NKIYDTYTLFSTKLT | TYTLFSTKL |
| | | KIYDTYTLFSTKLTQ | YTLFSTKLT |
| | | TYTLFSTKLTQMYST | YTLFSTKLT |
| | | KVKSKYLALGLLFGF | YLALGLLFG |
| | | VKSKYLALGLLFGFI | YLALGLLFG |
| | | KSKYLALGLLFGFIS | YLALGLLFG |
| | | SKYLALGLLFGFISC | YLALGLLFG |
| | | YTLFSTKLTQMYSTR | YTLFSTKLT |
| | | QDEYKGMTQGSLNSL | YKGMTQGSL |
| | | DEYKGMTQGSLNSLS | MTQGSLNSL |
| | | AYKAIVTSILLMRDS | YKAIVTSIL |
| | | YKAIVTSILLMRDSL | YKAIVTSIL |

| | | | |
|---|---|---|---|
| | | EYKGMTQGSLNSLSG | MTQGSLNSL |
| | | YKGMTQGSLNSLSGE | MTQGSLNSL |
| | | MKKVKSKYLALGLLF | VKSKYLALG |
| | | GMTQGSLNSLSGESG | MTQGSLNSL |
| | | MTQGSLNSLSGESGE | LNSLSGESG |
| | | KGMTQGSLNSLSGES | MTQGSLNSL |
| | | YLALGLLFGFISCDL | YLALGLLFG |
| | | AEIKTLIAKIKEQSN | IKTLIAKIK |
| | | EAEIKTLIAKIKEQS | IKTLIAKIK |
| | | ENIEAEIKTLIAKIK | EAEIKTLIA |
| | | NIEAEIKTLIAKIKE | IKTLIAKIK |
| | | IEAEIKTLIAKIKEQ | IKTLIAKIK |
| | | KYLALGLLFGFISCD | YLALGLLFG |
| | | FKTLLNYIQVSVKTA | FKTLLNYIQ |
| | | QGSLNSLSGESGELK | LNSLSGESG |
| | | TQGSLNSLSGESGEL | LNSLSGESG |
| | | LNYIQVSVKTAANFV | YIQVSVKTA |
| | | LLNYIQVSVKTAANF | YIQVSVKTA |
| | | QSNLYEAYKAIVTSI | YEAYKAIVT |
| | | EQSNLYEAYKAIVTS | YEAYKAIVT |
| | | GSLNSLSGESGELKE | LNSLSGESG |
| | | EKNFKTLLNYIQVSV | FKTLLNYIQ |
| | | TLLNYIQVSVKTAAN | YIQVSVKTA |
| | | KEQSNLYEAYKAIVT | LYEAYKAIV |
| | | KNFKTLLNYIQVSVK | FKTLLNYIQ |
| | | KTLLNYIQVSVKTAA | YIQVSVKTA |
| | | QQDEYKGMTQGSLNS | YKGMTQGSL |
| | | NYIQVSVKTAANFVY | YIQVSVKTA |
| | | DLEKNFKTLLNYIQV | FKTLLNYIQ |
| | | EDLEKNFKTLLNYIQ | EKNFKTLLN |
| | | YIQVSVKTAANFVYI | YIQVSVKTA |
| | | INKIYDTYTLFSTKL | YDTYTLFST |
| | | KAIVTSILLMRDSLK | VTSILLMRD |
| | | EGVKYNVDSAINTIN | YNVDSAINT |
| | | LEKNFKTLLNYIQVS | FKTLLNYIQ |
| | | EPYIHSLKRDSANKS | IHSLKRDSA |
| | | HSLKRDSANKSNFLQ | LKRDSANKS |
| | | IHSLKRDSANKSNFL | LKRDSANKS |
| | | PYIHSLKRDSANKSN | LKRDSANKS |
| | | GVKYNVDSAINTINK | YNVDSAINT |
| | | LEGVKYNVDSAINTI | YNVDSAINT |
| | | YLEGVKYNVDSAINT | VKYNVDSAI |
| | | YIHSLKRDSANKSNF | LKRDSANKS |
| | HLA-DRB1*0301 | NEIDITIDSDLRPKS | ITIDSDLRP |
| | | EIDITIDSDLRPKSS | ITIDSDLRP |
| | | IDITIDSDLRPKSSL | ITIDSDLRP |
| | | ITIDSDLRPKSSLQD | IDSDLRPKS |
| | | DITIDSDLRPKSSLQ | IDSDLRPKS |
| | HLA-DRB1*0401 | PYIHSLKRDSANKSN | LKRDSANKS |
| | | EPYIHSLKRDSANKS | IHSLKRDSA |
| | | YIHSLKRDSANKSNF | LKRDSANKS |
| | | IHSLKRDSANKSNFL | LKRDSANKS |
| | | HSLKRDSANKSNFLQ | LKRDSANKS |
| | | SLKRDSANKSNFLQK | LKRDSANKS |
| | | LKRDSANKSNFLQKN | LKRDSANKS |
| | | NKIYDTYTLFSTKLT | IYDTYTLFS |

| | | | |
|---|---|---|---|
| | | AANFVYINDTHAKRK | FVYINDTHA |
| | | ANFVYINDTHAKRKL | FVYINDTHA |
| | | FKTLLNYIQVSVKTA | NYIQVSVKT |
| | | TAANFVYINDTHAKR | FVYINDTHA |
| | | TINKIYDTYTLFSTK | IYDTYTLFS |
| | | NTINKIYDTYTLFST | IYDTYTLFS |
| | | INTINKIYDTYTLFS | INKIYDTYT |
| | | INKIYDTYTLFSTKL | IYDTYTLFS |
| | | IKTLIAKIKEQSNLY | LIAKIKEQS |
| | | KTLIAKIKEQSNLYE | KIKEQSNLY |
| | | NFKTLLNYIQVSVKT | FKTLLNYIQ |
| | | TLLNYIQVSVKTAAN | NYIQVSVKT |
| | | LLNYIQVSVKTAANF | NYIQVSVKT |
| | | KTLLNYIQVSVKTAA | NYIQVSVKT |
| | | TLIAKIKEQSNLYEA | KIKEQSNLY |
| | | LIAKIKEQSNLYEAY | KIKEQSNLY |
| | | IAKIKEQSNLYEAYK | KIKEQSNLY |
| | HLA-DRB1*0404 | AANFVYINDTHAKRK | FVYINDTHA |
| | | ANFVYINDTHAKRKL | FVYINDTHA |
| | HLA-DRB1*0405 | EDLEKNFKTLLNYIQ | EKNFKTLLN |
| | | DLEKNFKTLLNYIQV | EKNFKTLLN |
| | | FTKEDLEKNFKTLLN | LEKNFKTLL |
| | | TKEDLEKNFKTLLNY | EKNFKTLLN |
| | | NKIYDTYTLFSTKLT | IYDTYTLFS |
| | | KEDLEKNFKTLLNYI | EKNFKTLLN |
| | | KTLIAKIKEQSNLYE | IAKIKEQSN |
| | | TLIAKIKEQSNLYEA | IKEQSNLYE |
| | | IAKIKEQSNLYEAYK | IKEQSNLYE |
| | | AKIKEQSNLYEAYKA | IKEQSNLYE |
| | | LIAKIKEQSNLYEAY | IKEQSNLYE |
| | | LEKNFKTLLNYIQVS | EKNFKTLLN |
| | | EKNFKTLLNYIQVSV | EKNFKTLLN |
| | | IYDTYTLFSTKLTQM | YTLFSTKLT |
| | | KIYDTYTLFSTKLTQ | YTLFSTKLT |
| | | INTINKIYDTYTLFS | INTINKIYD |
| | | YDTYTLFSTKLTQMY | YTLFSTKLT |
| | | ITIDSDLRPKSSLQD | ITIDSDLRP |
| | | DTYTLFSTKLTQMYS | YTLFSTKLT |
| | | IKEQSNLYEAYKAIV | IKEQSNLYE |
| | | TIDSDLRPKSSLQDI | LRPKSSLQD |
| | | IDSDLRPKSSLQDIA | LRPKSSLQD |
| | HLA-DRB1*0701 | YIQVSVKTAANFVYI | VKTAANFVY |
| | | LYEAYKAIVTSILLM | YKAIVTSIL |
| | | YEAYKAIVTSILLMR | YKAIVTSIL |
| | | IQVSVKTAANFVYIN | VKTAANFVY |
| | | NYIQVSVKTAANFVY | SVKTAANFV |
| | | QVSVKTAANFVYIND | VKTAANFVY |
| | | EAYKAIVTSILLMRD | YKAIVTSIL |
| | | NLYEAYKAIVTSILL | YKAIVTSIL |
| | | SNLYEAYKAIVTSIL | AYKAIVTSI |
| | | VSVKTAANFVYINDT | VKTAANFVY |
| | | AYKAIVTSILLMRDS | YKAIVTSIL |
| | | YKAIVTSILLMRDSL | YKAIVTSIL |
| | | SVKTAANFVYINDTH | VKTAANFVY |
| | | LNYIQVSVKTAANFV | VSVKTAANF |
| | HLA-DRB1*0802 | ESIKKPMNKKGKGKI | SIKKPMNKK |

| | HLA-DRB1*0901 | SNLYEAYKAIVTSIL | YEAYKAIVT |
|---|---|---|---|
| | | NLYEAYKAIVTSILL | YEAYKAIVT |
| | | QSNLYEAYKAIVTSI | YEAYKAIVT |
| | | EQSNLYEAYKAIVTS | YEAYKAIVT |
| | | KEQSNLYEAYKAIVT | LYEAYKAIV |
| | | YDTYTLFSTKLTQMY | YTLFSTKLT |
| | | DTYTLFSTKLTQMYS | YTLFSTKLT |
| | | YEAYKAIVTSILLMR | YEAYKAIVT |
| | | IYDTYTLFSTKLTQM | YTLFSTKLT |
| | | KIYDTYTLFSTKLTQ | YTLFSTKLT |
| | | LYEAYKAIVTSILLM | YEAYKAIVT |
| | | NKIYDTYTLFSTKLT | IYDTYTLFS |
| | HLA-DRB1*1101 | None | |
| | HLA-DRB1*1302 | EAYKAIVTSILLMRD | YKAIVTSIL |
| | | YEAYKAIVTSILLMR | YKAIVTSIL |
| | | YDTYTLFSTKLTQMY | YTLFSTKLT |
| | | DTYTLFSTKLTQMYS | YTLFSTKLT |
| | | IYDTYTLFSTKLTQM | YTLFSTKLT |
| | | NKIYDTYTLFSTKLT | NKIYDTYTL |
| | | AYKAIVTSILLMRDS | IVTSILLMR |
| | | YKAIVTSILLMRDSL | IVTSILLMR |
| | | KIYDTYTLFSTKLTQ | YTLFSTKLT |
| | | NYIQVSVKTAANFVY | IQVSVKTAA |
| | | YTLFSTKLTQMYSTR | YTLFSTKLT |
| | | LYEAYKAIVTSILLM | YKAIVTSIL |
| | | TYTLFSTKLTQMYST | YTLFSTKLT |
| | | KTLLNYIQVSVKTAA | LLNYIQVSV |
| | | KNFKTLLNYIQVSVK | LLNYIQVSV |
| | | ENIEAEIKTLIAKIK | IEAEIKTLI |
| | | NLYEAYKAIVTSILL | YKAIVTSIL |
| | | FKTLLNYIQVSVKTA | LLNYIQVSV |
| | | EKNFKTLLNYIQVSV | FKTLLNYIQ |
| | | SNLYEAYKAIVTSIL | YEAYKAIVT |
| | | NFKTLLNYIQVSVKT | LNYIQVSVK |
| | | TLLNYIQVSVKTAAN | IQVSVKTAA |
| | | IEAEIKTLIAKIKEQ | IKTLIAKIK |
| | | NIEAEIKTLIAKIKE | IKTLIAKIK |
| | | DYEEIRLSNRYQSYL | IRLSNRYQS |
| | | KSKYLALGLLFGFIS | YLALGLLFG |
| | | KAIVTSILLMRDSLK | IVTSILLMR |
| | | YEEIRLSNRYQSYLE | IRLSNRYQS |
| | | YIQVSVKTAANFVYI | VKTAANFVY |
| | | DDYEEIRLSNRYQSY | IRLSNRYQS |
| | | EDDYEEIRLSNRYQS | EEIRLSNRY |
| | | SKYLALGLLFGFISC | LGLLFGFIS |
| | | EEIRLSNRYQSYLEG | IRLSNRYQS |
| | | LLNYIQVSVKTAANF | IQVSVKTAA |
| | | EAEIKTLIAKIKEQS | IKTLIAKIK |
| | | AEIKTLIAKIKEQSN | IKTLIAKIK |
| | | EDLEKNFKTLLNYIQ | EKNFKTLLN |
| | | IQVSVKTAANFVYIN | VKTAANFVY |
| | | KYLALGLLFGFISCD | LGLLFGFIS |
| | | VDSAINTINKIYDTY | INTINKIYD |
| | | YNVDSAINTINKIYD | SAINTINKI |
| | | DLEKNFKTLLNYIQV | FKTLLNYIQ |
| | | LALGLLFGFISCDLF | LGLLFGFIS |
| | | SAINTINKIYDTYTL | INTINKIYD |

|  |  |  |  |
|---|---|---|---|
|  |  | YLALGLLFGFISCDL | LGLLFGFIS |
|  |  | NVDSAINTINKIYDT | INTINKIYD |
|  |  | LNYIQVSVKTAANFV | IQVSVKTAA |
|  |  | DSAINTINKIYDTYT | INTINKIYD |
|  |  | ANKSNFLQKNVILEE | SNFLQKNVI |
|  |  | NKSNFLQKNVILEEE | SNFLQKNVI |
|  |  | LEKNFKTLLNYIQVS | FKTLLNYIQ |
|  |  | EPYIHSLKRDSANKS | IHSLKRDSA |
|  | HLA-DRB1*1501 | KAIVTSILLMRDSLK | IVTSILLMR |
|  |  | IVTSILLMRDSLKEV | IVTSILLMR |
|  |  | YEAYKAIVTSILLMR | YEAYKAIVT |
|  |  | AIVTSILLMRDSLKE | IVTSILLMR |
|  |  | KGKIARKKGKSKVSR | IARKKGKSK |
|  |  | GKIARKKGKSKVSRK | IARKKGKSK |
|  |  | EAYKAIVTSILLMRD | IVTSILLMR |
|  |  | AYKAIVTSILLMRDS | IVTSILLMR |
|  |  | YKAIVTSILLMRDSL | IVTSILLMR |
|  |  | KKGKGKIARKKGKSK | KKGKGKIAR |
|  |  | GKGKIARKKGKSKVS | IARKKGKSK |
|  |  | KGKGKIARKKGKSKV | IARKKGKSK |
|  |  | TSILLMRDSLKEVQG | ILLMRDSLK |
|  |  | VTSILLMRDSLKEVQ | ILLMRDSLK |
|  | HLA-DRB3*0101 | None |  |
|  | HLA-DRB4*0101 | GKSKVSRKEPYIHSL | VSRKEPYIH |
|  |  | KSKVSRKEPYIHSLK | VSRKEPYIH |
|  |  | KKGKSKVSRKEPYIH | KKGKSKVSR |
|  |  | KGKSKVSRKEPYIHS | VSRKEPYIH |
|  |  | SKVSRKEPYIHSLKR | VSRKEPYIH |
|  |  | VSRKEPYIHSLKRDS | VSRKEPYIH |
|  |  | KVSRKEPYIHSLKRD | VSRKEPYIH |
|  | HLA-DRB5*0101 | KTELLKEQSETRKEK | LLKEQSETR |
|  |  | TELLKEQSETRKEKI | LLKEQSETR |
|  |  | TSEESIKKPMNKKGK | IKKPMNKKG |
|  |  | SEESIKKPMNKKGKG | KKPMNKKG |
|  |  | SLKTELLKEQSETRK | LLKEQSETR |
|  |  | LKTELLKEQSETRKE | LLKEQSETR |
|  |  | ESIKKPMNKKGKGKI | KKPMNKKG |
|  |  | EESIKKPMNKKGKGK | KKPMNKKG |
|  |  | AANFVYINDTHAKRK | YINDTHAKR |
|  |  | ANFVYINDTHAKRKL | INDTHAKRK |
|  |  | NFVYINDTHAKRKLE | INDTHAKRK |
|  |  | FVYINDTHAKRKLEN | INDTHAKRK |
|  |  | ELLKEQSETRKEKIQ | LKEQSETRK |
|  |  |  |  |
| AAL84590.1\| BBK32 [Borrelia afzelii]<br><br>Seq43 | HLA-DRB1*0101 | YDNYTLLSTKQTQMY | YTLLSTKQT |
|  |  | VKIYDNYTLLSTKQT | YDNYTLLST |
|  |  | KIYDNYTLLSTKQTQ | YTLLSTKQT |
|  |  | DNYTLLSTKQTQMYS | YTLLSTKQT |
|  |  | IYDNYTLLSTKQTQM | YTLLSTKQT |
|  |  | DEHKRMLQGSLSFLS | KRMLQGSLS |
|  |  | EHKRMLQGSLSFLSG | LQGSLSFLS |
|  |  | HKRMLQGSLSFLSGE | LQGSLSFLS |
|  |  | KRMLQGSLSFLSGES | LQGSLSFLS |
|  |  | RMLQGSLSFLSGESG | LQGSLSFLS |
|  |  | NYTLLSTKQTQMYST | YTLLSTKQT |
|  |  | YTLLSTKQTQMYSTR | YTLLSTKQT |

| | | | |
|---|---|---|---|
| | | LLNYIQVSARTATNF | YIQVSARTA |
| | | YLEGVKYNVSSAIKT | VKYNVSSAI |
| | | LEGVKYNVSSAIKTI | YNVSSAIKT |
| | | EGVKYNVSSAIKTIV | YNVSSAIKT |
| | | TLLNYIQVSARTATN | YIQVSARTA |
| | | LNYIQVSARTATNFV | YIQVSARTA |
| | | KTLLNYIQVSARTAT | YIQVSARTA |
| | | LKTLLNYIQVSARTA | NYIQVSART |
| | | GVKYNVSSAIKTIVK | YNVSSAIKT |
| | | VKYNVSSAIKTIVKI | YNVSSAIKT |
| | | NYIQVSARTATNFVY | YIQVSARTA |
| | | MKIKSKCLALGLLFG | IKSKCLALG |
| | | TLLSTKQTQMYSTRL | LSTKQTQMY |
| | | LQGSLSFLSGESGEL | LQGSLSFLS |
| | | MLQGSLSFLSGESGE | LQGSLSFLS |
| | | YIQVSARTATNFVYA | YIQVSARTA |
| | | EDYYYDGETRLSNRY | YYDGETRLS |
| | | EEDYYYDGETRLSNR | YYDGETRLS |
| | | DYYYDGETRLSNRYE | YYDGETRLS |
| | | LEEDYYYDGETRLSN | YYDGETRLS |
| | | LSTKQTQMYSTRLDN | QTQMYSTRL |
| | | KTIVKIYDNYTLLST | IYDNYTLLS |
| | | LLSTKQTQMYSTRLD | QTQMYSTRL |
| | | AIVRSILLMKDSLKI | VRSILLMKD |
| | | VRSILLMKDSLKIIE | LLMKDSLKI |
| | | TKQTQMYSTRLDNLA | QTQMYSTRL |
| | | IVRSILLMKDSLKII | LLMKDSLKI |
| | | RSILLMKDSLKIIEI | LLMKDSLKI |
| | | SLEEDYYYDGETRLS | YYYDGETRL |
| | | KYNVSSAIKTIVKIY | YNVSSAIKT |
| | | TIVKIYDNYTLLSTK | YDNYTLLST |
| | | SILLMKDSLKIIEIV | LLMKDSLKI |
| | | YNVSSAIKTIVKIYD | YNVSSAIKT |
| | | IVKIYDNYTLLSTKQ | YDNYTLLST |
| | | KIKSKCLALGLLFGF | IKSKCLALG |
| | | IKSKCLALGLLFGFI | IKSKCLALG |
| | | STKQTQMYSTRLDNL | QTQMYSTRL |
| | | KSDLQAISGSNSISY | LQAISGSNS |
| | | SDLQAISGSNSISYT | LQAISGSNS |
| | | KQTQMYSTRLDNLAK | QTQMYSTRL |
| | | QTQMYSTRLDNLAKA | QTQMYSTRL |
| | | YYYDGETRLSNRYES | YYDGETRLS |
| | | QDEHKRMLQGSLSFL | KRMLQGSLS |
| | | LRLKSDLQAISGSNS | KSDLQAISG |
| | | RLKSDLQAISGSNSI | LQAISGSNS |
| | | LKSDLQAISGSNSIS | LQAISGSNS |
| | | SEEEILKTKLLRERP | LKTKLLRER |
| | | YYDGETRLSNRYESY | YYDGETRLS |
| | | EEILKTKLLRERPET | LKTKLLRER |
| | | EEEILKTKLLRERPE | LKTKLLRER |
| | | ISEEEILKTKLLRER | EEILKTKLL |
| | | ILLMKDSLKIIEIVI | LLMKDSLKI |
| | | KDLKTLLNYIQVSAR | LKTLLNYIQ |
| | | EILKTKLLRERPETR | LKTKLLRER |
| | | EKDLKTLLNYIQVSA | LKTLLNYIQ |
| | | DLKTLLNYIQVSART | LKTLLNYIQ |
| | | IKTIVKIYDNYTLLS | VKIYDNYTL |

| | | | |
|---|---|---|---|
| | | LLMKDSLKIIEIVID | LLMKDSLKI |
| | | ELEKDLKTLLNYIQV | LKTLLNYIQ |
| | | EELEKDLKTLLNYIQ | EKDLKTLLN |
| | | EAYKAIVRSILLMKD | YKAIVRSIL |
| | | LEKDLKTLLNYIQVS | LKTLLNYIQ |
| | | SYLEGVKYNVSSAIK | VKYNVSSAI |
| | | DLYEAYKAIVRSILL | YEAYKAIVR |
| | | DLQAISGSNSISYTD | LQAISGSNS |
| | | SDLYEAYKAIVRSIL | YEAYKAIVR |
| | | LQAISGSNSISYTDE | LQAISGSNS |
| | | QMYSTRLDNLAKAKA | YSTRLDNLA |
| | | ETEIKNLILKIKGQS | IKNLILKIK |
| | | TEIKNLILKIKGQSD | IKNLILKIK |
| | | IETEIKNLILKIKGQ | IKNLILKIK |
| | | AIETEIKNLILKIKG | IKNLILKIK |
| | | QGSLSFLSGESGELK | FLSGESGEL |
| | | AYKAIVRSILLMKDS | VRSILLMKD |
| | | DAIETEIKNLILKIK | EIKNLILKI |
| | | IQVSARTATNFVYAR | VSARTATNF |
| | | YKAIVRSILLMKDSL | VRSILLMKD |
| | | KAIVRSILLMKDSLK | VRSILLMKD |
| | | GSLSFLSGESGELKD | FLSGESGEL |
| | | ESYLEGVKYNVSSAI | LEGVKYNVS |
| | | STRLDNLAKAKAREE | LDNLAKAKA |
| | | TRLDNLAKAKAREEA | LDNLAKAKA |
| | HLA-DRB1*0301 | NEIDFTIDSDLRLKS | FTIDSDLRL |
| | | IDFTIDSDLRLKSDL | FTIDSDLRL |
| | | EIDFTIDSDLRLKSD | FTIDSDLRL |
| | | FTIDSDLRLKSDLQA | IDSDLRLKS |
| | | DFTIDSDLRLKSDLQ | IDSDLRLKS |
| | | ESNEIDFTIDSDLRL | NEIDFTIDS |
| | | SNEIDFTIDSDLRLK | FTIDSDLRL |
| | HLA-DRB1*0401 | NAVKKDPFNHHVKRE | VKKDPFNHH |
| | | GNAVKKDPFNHHVKR | VKKDPFNHH |
| | | KVEGNAVKKDPFNHH | KVEGNAVKK |
| | | EGNAVKKDPFNHHVK | VKKDPFNHH |
| | | VEGNAVKKDPFNHHV | VKKDPFNHH |
| | | NHHVKRESVNNSNLS | VKRESVNNS |
| | | HHVKRESVNNSNLSQ | VKRESVNNS |
| | | DPFNHHVKRESVNNS | FNHHVKRES |
| | | PFNHHVKRESVNNSN | VKRESVNNS |
| | | VKIYDNYTLLSTKQT | IYDNYTLLS |
| | | IVKIYDNYTLLSTKQ | IYDNYTLLS |
| | | FNHHVKRESVNNSNL | VKRESVNNS |
| | | IKTIVKIYDNYTLLS | IVKIYDNYT |
| | | KTIVKIYDNYTLLST | IYDNYTLLS |
| | | TIVKIYDNYTLLSTK | IYDNYTLLS |
| | | VKKDPFNHHVKRESV | VKKDPFNHH |
| | | AVKKDPFNHHVKRES | VKKDPFNHH |
| | | HVKRESVNNSNLSQK | VKRESVNNS |
| | | VKRESVNNSNLSQKN | VKRESVNNS |
| | HLA-DRB1*0404 | None | |
| | HLA-DRB1*0405 | IKTIVKIYDNYTLLS | IKTIVKIYD |
| | | VKIYDNYTLLSTKQT | IYDNYTLLS |
| | | SSAIKTIVKIYDNYT | IKTIVKIYD |
| | | KTIVKIYDNYTLLST | IYDNYTLLS |

|  |  | SAIKTIVKIYDNYTL | IKTIVKIYD |  |
|---|---|---|---|---|
|  | HLA-DRB1*0701 | NYIQVSARTATNFVY | VSARTATNF |  |
|  |  | YIQVSARTATNFVYA | ARTATNFVY |  |
|  |  | IQVSARTATNFVYAR | ARTATNFVY |  |
|  |  | DLYEAYKAIVRSILL | YKAIVRSIL |  |
|  |  | LYEAYKAIVRSILLM | YKAIVRSIL |  |
|  |  | YEAYKAIVRSILLMK | YKAIVRSIL |  |
|  |  | SDLYEAYKAIVRSIL | YEAYKAIVR |  |
|  |  | QVSARTATNFVYARE | ARTATNFVY |  |
|  |  | EAYKAIVRSILLMKD | YKAIVRSIL |  |
|  |  | VSARTATNFVYAREI | ARTATNFVY |  |
|  |  | LNYIQVSARTATNFV | VSARTATNF |  |
|  |  | EGVKYNVSSAIKTIV | VKYNVSSAI |  |
|  |  | LLNYIQVSARTATNF | IQVSARTAT |  |
|  | HLA-DRB1*0802 | None |  |  |
|  | HLA-DRB1*0901 | EGVKYNVSSAIKTIV | YNVSSAIKT |  |
|  |  | LEGVKYNVSSAIKTI | YNVSSAIKT |  |
|  |  | GVKYNVSSAIKTIVK | YNVSSAIKT |  |
|  |  | VKYNVSSAIKTIVKI | YNVSSAIKT |  |
|  |  | YLEGVKYNVSSAIKT | VKYNVSSAI |  |
|  |  | KTLLNYIQVSARTAT | YIQVSARTA |  |
|  |  | LNYIQVSARTATNFV | YIQVSARTA |  |
|  |  | KYNVSSAIKTIVKIY | YNVSSAIKT |  |
|  |  | TLLNYIQVSARTATN | YIQVSARTA |  |
|  |  | LLNYIQVSARTATNF | YIQVSARTA |  |
|  |  | YNVSSAIKTIVKIYD | YNVSSAIKT |  |
|  | HLA-DRB1*1101 | None |  |  |
|  | HLA-DRB1*1302 | DAIETEIKNLILKIK | IETEIKNLI |  |
|  |  | AIETEIKNLILKIKG | IKNLILKIK |  |
|  |  | IETEIKNLILKIKGQ | IKNLILKIK |  |
|  |  | ETEIKNLILKIKGQS | IKNLILKIK |  |
|  |  | TEIKNLILKIKGQSD | IKNLILKIK |  |
|  |  | EIKNLILKIKGQSDL | IKNLILKIK |  |
|  |  | IKNLILKIKGQSDLY | IKNLILKIK |  |
|  |  | EGVKYNVSSAIKTIV | YNVSSAIKT |  |
|  |  | LEGVKYNVSSAIKTI | YNVSSAIKT |  |
|  |  | YLEGVKYNVSSAIKT | VKYNVSSAI |  |
|  |  | GVKYNVSSAIKTIVK | YNVSSAIKT |  |
|  |  | VKYNVSSAIKTIVKI | YNVSSAIKT |  |
|  |  | VKIYDNYTLLSTKQT | IYDNYTLLS |  |
|  |  | KTLLNYIQVSARTAT | LLNYIQVSA |  |
|  |  | EAYKAIVRSILLMKD | YKAIVRSIL |  |
|  |  | TLLNYIQVSARTATN | IQVSARTAT |  |
|  |  | YEAYKAIVRSILLMK | YKAIVRSIL |  |
|  |  | HVKRESVNNSNLSQK | ESVNNSNLS |  |
|  |  | VKRESVNNSNLSQKN | VNNSNLSQK |  |
|  |  | LLNYIQVSARTATNF | IQVSARTAT |  |
|  |  | LNYIQVSARTATNFV | IQVSARTAT |  |
|  |  | NYIQVSARTATNFVY | IQVSARTAT |  |
|  |  | KIYDNYTLLSTKQTQ | YTLLSTKQT |  |
|  |  | IYDNYTLLSTKQTQM | YTLLSTKQT |  |
|  |  | YDNYTLLSTKQTQMY | YTLLSTKQT |  |
|  |  | KRESVNNSNLSQKNV | VNNSNLSQK |  |
|  |  | RESVNNSNLSQKNVI | VNNSNLSQK |  |
|  |  | YKAIVRSILLMKDSL | IVRSILLMK |  |
|  |  | AYKAIVRSILLMKDS | IVRSILLMK |  |

|  |  |  |  |  |
|---|---|---|---|---|
|  |  | DNYTLLSTKQTQMYS | YTLLSTKQT |  |
|  |  | KDLKTLLNYIQVSAR | LKTLLNYIQ |  |
|  |  | ESVNNSNLSQKNVIS | VNNSNLSQK |  |
|  |  | YNVSSAIKTIVKIYD | YNVSSAIKT |  |
|  |  | KNLILKIKGQSDLYE | LKIKGQSDL |  |
|  |  | LYEAYKAIVRSILLM | YKAIVRSIL |  |
|  |  | DLKTLLNYIQVSART | LNYIQVSAR |  |
|  |  | EKDLKTLLNYIQVSA | LKTLLNYIQ |  |
|  |  | LKTLLNYIQVSARTA | LNYIQVSAR |  |
|  |  | LDAIETEIKNLILKI | IETEIKNLI |  |
|  |  | KYNVSSAIKTIVKIY | YNVSSAIKT |  |
|  |  | KTIVKIYDNYTLLST | VKIYDNYTL |  |
|  |  | HHVKRESVNNSNLSQ | VKRESVNNS |  |
|  |  | IKTIVKIYDNYTLLS | VKIYDNYTL |  |
|  |  | SAIKTIVKIYDNYTL | IKTIVKIYD |  |
|  |  | NHHVKRESVNNSNLS | VKRESVNNS |  |
|  |  | DLYEAYKAIVRSILL | YKAIVRSIL |  |
|  |  | KAIVRSILLMKDSLK | IVRSILLMK |  |
|  |  | SYLEGVKYNVSSAIK | VKYNVSSAI |  |
|  |  | YIQVSARTATNFVYA | IQVSARTAT |  |
|  |  | TIVKIYDNYTLLSTK | VKIYDNYTL |  |
|  |  | KLDAIETEIKNLILK | IETEIKNLI |  |
|  |  | IQVSARTATNFVYAR | IQVSARTAT |  |
|  |  | YTLLSTKQTQMYSTR | YTLLSTKQT |  |
|  |  | LALGLLFGFISCDLF | LGLLFGFIS |  |
|  |  | ESYLEGVKYNVSSAI | LEGVKYNVS |  |
|  |  | SDLYEAYKAIVRSIL | EAYKAIVRS |  |
|  |  | NYTLLSTKQTQMYST | YTLLSTKQT |  |
|  |  | KSKCLALGLLFGFIS | LALGLLFGF |  |
|  |  | SKCLALGLLFGFISC | LGLLFGFIS |  |
|  |  | EELEKDLKTLLNYIQ | LEKDLKTLL |  |
|  |  | KCLALGLLFGFISCD | LGLLFGFIS |  |
|  |  | CLALGLLFGFISCDL | LGLLFGFIS |  |
|  |  | AIVRSILLMKDSLKI | ILLMKDSLK |  |
|  |  | LEKDLKTLLNYIQVS | LKTLLNYIQ |  |
|  |  | IVRSILLMKDSLKII | ILLMKDSLK |  |
|  |  | ELEKDLKTLLNYIQV | LKTLLNYIQ |  |
|  |  | NLILKIKGQSDLYEA | LKIKGQSDL |  |
|  |  | IVKIYDNYTLLSTKQ | IYDNYTLLS |  |
|  |  | LILKIKGQSDLYEAY | LKIKGQSDL |  |
|  |  | VRSILLMKDSLKIIE | ILLMKDSLK |  |
|  |  | SVNNSNLSQKNVISE | VNNSNLSQK |  |
|  |  | MKIKSKCLALGLLFG | MKIKSKCLA |  |
|  |  | RKLDAIETEIKNLIL | IETEIKNLI |  |
|  |  | AIKTIVKIYDNYTLL | VKIYDNYTL |  |
|  |  | DPFNHHVKRESVNNS | FNHHVKRES |  |
|  |  | KRKLDAIETEIKNLI | KLDAIETEI |  |
|  | HLA-DRB1*1501 | EILKTKLLRERPETR | LKTKLLRER |  |
|  |  | ILKTKLLRERPETRK | LLRERPETR |  |
|  |  | LKTKLLRERPETRKE | LLRERPETR |  |
|  |  | KTKLLRERPETRKEE | LLRERPETR |  |
|  |  | TKLLRERPETRKEEI | LLRERPETR |  |
|  |  | KAIVRSILLMKDSLK | IVRSILLMK |  |
|  |  | YEAYKAIVRSILLMK | YEAYKAIVR |  |
|  |  | AIVRSILLMKDSLKI | IVRSILLMK |  |
|  |  | IVRSILLMKDSLKII | IVRSILLMK |  |
|  |  | AYKAIVRSILLMKDS | IVRSILLMK |  |

| | | | |
|---|---|---|---|
| | | EAYKAIVRSILLMKD | IVRSILLMK |
| | | YKAIVRSILLMKDSL | IVRSILLMK |
| | | KLLRERPETRKEEIQ | LLRERPETR |
| | | ETEIKNLILKIKGQS | IKNLILKIK |
| | | TEIKNLILKIKGQSD | IKNLILKIK |
| | | LLRERPETRKEEIQK | LLRERPETR |
| | | ISEEEILKTKLLRER | EILKTKLLR |
| | | SEEEILKTKLLRERP | LKTKLLRER |
| | | EEEILKTKLLRERPE | LKTKLLRER |
| | | IETEIKNLILKIKGQ | IKNLILKIK |
| | | EEILKTKLLRERPET | LKTKLLRER |
| | | AIETEIKNLILKIKG | IKNLILKIK |
| | | DAIETEIKNLILKIK | TEIKNLILK |
| | | VRSILLMKDSLKIIE | LLMKDSLKI |
| | | RSILLMKDSLKIIEI | LLMKDSLKI |
| | | ATNFVYAREIYSKRK | VYAREIYSK |
| | | TNFVYAREIYSKRKL | VYAREIYSK |
| | | KDLKTLLNYIQVSAR | LLNYIQVSA |
| | | TATNFVYAREIYSKR | VYAREIYSK |
| | | NFVYAREIYSKRKLD | VYAREIYSK |
| | HLA-DRB3*0101 | None | |
| | HLA-DRB4*0101 | ILKTKLLRERPETRK | LLRERPETR |
| | | LKTKLLRERPETRKE | LRERPETRK |
| | HLA-DRB5*0101 | ATNFVYAREIYSKRK | YAREIYSKR |
| | | TNFVYAREIYSKRKL | YAREIYSKR |
| | | NFVYAREIYSKRKLD | YAREIYSKR |
| | | FVYAREIYSKRKLDA | YAREIYSKR |
| | | EILKTKLLRERPETR | LKTKLLRER |
| | | VYAREIYSKRKLDAI | YAREIYSKR |
| | | TATNFVYAREIYSKR | VYAREIYSK |
| | | ILKTKLLRERPETRK | LLRERPETR |
| | | KTKLLRERPETRKEE | LLRERPETR |
| | | LKTKLLRERPETRKE | LLRERPETR |
| | | TKLLRERPETRKEEI | LLRERPETR |
| | | | |
| AAL84595.1\| BBK32 [Borrelia garinii]  Seq44 | HLA-DRB1*0101 | TLFSTKLTQMYSTRL | FSTKLTQMY |
| | | LFSTKLTQMYSTRLD | LTQMYSTRL |
| | | FSTKLTQMYSTRLDN | LTQMYSTRL |
| | | STKLTQMYSTRLDNL | LTQMYSTRL |
| | | TKLTQMYSTRLDNLA | LTQMYSTRL |
| | | KLTQMYSTRLDNLAK | LTQMYSTRL |
| | | LTQMYSTRLDNLAKA | LTQMYSTRL |
| | | YSAYKAIVSSILLMR | YKAIVSSIL |
| | | NLYSAYKAIVSSILL | YKAIVSSIL |
| | | LYSAYKAIVSSILLM | YKAIVSSIL |
| | | SAYKAIVSSILLMRD | YKAIVSSIL |
| | | SNLYSAYKAIVSSIL | YSAYKAIVS |
| | | YDTYTLFSTKLTQMY | YTLFSTKLT |
| | | DTYTLFSTKLTQMYS | YTLFSTKLT |
| | | KKVKSKYLALGLLFG | VKSKYLALG |
| | | IYDTYTLFSTKLTQM | YTLFSTKLT |
| | | NKIYDTYTLFSTKLT | TYTLFSTKL |
| | | KIYDTYTLFSTKLTQ | YTLFSTKLT |
| | | TYTLFSTKLTQMYST | YTLFSTKLT |
| | | KVKSKYLALGLLFGF | YLALGLLFG |
| | | VKSKYLALGLLFGFI | YLALGLLFG |

| | | | |
|---|---|---|---|
| | | KSKYLALGLLFGFIS | YLALGLLFG |
| | | SKYLALGLLFGFISC | YLALGLLFG |
| | | YTLFSTKLTQMYSTR | YTLFSTKLT |
| | | AYKAIVSSILLMRDS | YKAIVSSIL |
| | | YKAIVSSILLMRDSL | YKAIVSSIL |
| | | HSFKRDAANKSNFLQ | FKRDAANKS |
| | | IHSFKRDAANKSNFL | FKRDAANKS |
| | | DEYKGMTKGSLNSLS | MTKGSLNSL |
| | | PFIHSFKRDAANKSN | FKRDAANKS |
| | | MKKVKSKYLALGLLF | VKSKYLALG |
| | | QDEYKGMTKGSLNSL | YKGMTKGSL |
| | | EPFIHSFKRDAANKS | FIHSFKRDA |
| | | FIHSFKRDAANKSNF | FKRDAANKS |
| | | GMTKGSLNSLSGESG | MTKGSLNSL |
| | | EYKGMTKGSLNSLSG | MTKGSLNSL |
| | | YKGMTKGSLNSLSGE | MTKGSLNSL |
| | | MTKGSLNSLSGESGE | LNSLSGESG |
| | | YLALGLLFGFISCDL | YLALGLLFG |
| | | ENIEAKIKTLIAKIK | EAKIKTLIA |
| | | NIEAKIKTLIAKIKE | IKTLIAKIK |
| | | EAKIKTLIAKIKEKS | IKTLIAKIK |
| | | AKIKTLIAKIKEKSN | IKTLIAKIK |
| | | IEAKIKTLIAKIKEK | IKTLIAKIK |
| | | KYLALGLLFGFISCD | YLALGLLFG |
| | | FKTLLNYIQVSVKTA | FKTLLNYIQ |
| | | KGSLNSLSGESGELK | LNSLSGESG |
| | | LLNYIQVSVKTATNF | YIQVSVKTA |
| | | TKGSLNSLSGESGEL | LNSLSGESG |
| | | LNYIQVSVKTATNFV | YIQVSVKTA |
| | | KGMTKGSLNSLSGES | MTKGSLNSL |
| | | GSLNSLSGESGELKE | LNSLSGESG |
| | | EKNFKTLLNYIQVSV | FKTLLNYIQ |
| | | TLLNYIQVSVKTATN | YIQVSVKTA |
| | | KNFKTLLNYIQVSVK | FKTLLNYIQ |
| | | KTLLNYIQVSVKTAT | YIQVSVKTA |
| | | DLEKNFKTLLNYIQV | FKTLLNYIQ |
| | | EDLEKNFKTLLNYIQ | EKNFKTLLN |
| | | SFKRDAANKSNFLQK | FKRDAANKS |
| | | INKIYDTYTLFSTKL | YDTYTLFST |
| | | QQDEYKGMTKGSLNS | YKGMTKGSL |
| | | FKRDAANKSNFLQKN | FKRDAANKS |
| | | EGVKYNVDSAINTIN | YNVDSAINT |
| | | LEKNFKTLLNYIQVS | FKTLLNYIQ |
| | | GVKYNVDSAINTINK | YNVDSAINT |
| | | LEGVKYNVDSAINTI | YNVDSAINT |
| | | YLEGVKYNVDSAINT | VKYNVDSAI |
| | | NYIQVSVKTATNFVY | YIQVSVKTA |
| | | VKYNVDSAINTINKI | YNVDSAINT |
| | | SSLQDIAGSNSISYT | IAGSNSISY |
| | | KSSLQDIAGSNSISY | LQDIAGSNS |
| | | EKSNLYSAYKAIVSS | YSAYKAIVS |
| | | KSNLYSAYKAIVSSI | YSAYKAIVS |
| | | ITIDSDLRPKSSLQD | DSDLRPKSS |
| | | KEKSNLYSAYKAIVS | LYSAYKAIV |
| | | TIDSDLRPKSSLQDI | LRPKSSLQD |
| | | DSDLRPKSSLQDIAG | LRPKSSLQD |
| | | NFKTLLNYIQVSVKT | FKTLLNYIQ |

| | | | |
|---|---|---|---|
| | | IDSDLRPKSSLQDIA | LRPKSSLQD |
| | | NTINKIYDTYTLFST | INKIYDTYT |
| | | YIQVSVKTATNFVYI | YIQVSVKTA |
| | | KQQDEYKGMTKGSLN | YKGMTKGSL |
| | | SDLRPKSSLQDIAGS | LRPKSSLQD |
| | | QKQQDEYKGMTKGSL | QKQQDEYKG |
| | | SLQDIAGSNSISYTD | IAGSNSISY |
| | | LRPKSSLQDIAGSNS | LRPKSSLQD |
| | | LQDIAGSNSISYTDE | IAGSNSISY |
| | | KIKTLIAKIKEKSNL | IKTLIAKIK |
| | | IKTLIAKIKEKSNLY | IKTLIAKIK |
| | | TINKIYDTYTLFSTK | YDTYTLFST |
| | | QMYSTRLDNLAKAKA | YSTRLDNLA |
| | | KAIVSSILLMRDSLK | IVSSILLMR |
| | | TNFVYINEMHAKRKL | INEMHAKRK |
| | | ATNFVYINEMHAKRK | FVYINEMHA |
| | | SLNSLSGESGELKET | LNSLSGESG |
| | | LNSLSGESGELKETI | LNSLSGESG |
| | | FVYINEMHAKRKLEN | INEMHAKRK |
| | | NFVYINEMHAKRKLE | INEMHAKRK |
| | | AIVSSILLMRDSLKE | VSSILLMRD |
| | | QDIAGSNSISYTDEI | IAGSNSISY |
| | HLA-DRB1*0301 | NEIDITIDSDLRPKS | ITIDSDLRP |
| | | EIDITIDSDLRPKSS | ITIDSDLRP |
| | | IDITIDSDLRPKSSL | ITIDSDLRP |
| | | ITIDSDLRPKSSLQD | IDSDLRPKS |
| | | DITIDSDLRPKSSLQ | IDSDLRPKS |
| | HLA-DRB1*0401 | PFIHSFKRDAANKSN | FKRDAANKS |
| | | EPFIHSFKRDAANKS | IHSFKRDAA |
| | | FIHSFKRDAANKSNF | FKRDAANKS |
| | | HSFKRDAANKSNFLQ | FKRDAANKS |
| | | IHSFKRDAANKSNFL | FKRDAANKS |
| | | SFKRDAANKSNFLQK | FKRDAANKS |
| | | FKRDAANKSNFLQKN | FKRDAANKS |
| | | TATNFVYINEMHAKR | FVYINEMHA |
| | | TNFVYINEMHAKRKL | FVYINEMHA |
| | | ATNFVYINEMHAKRK | FVYINEMHA |
| | | KTATNFVYINEMHAK | FVYINEMHA |
| | | VKTATNFVYINEMHA | TNFVYINEM |
| | | KTLIAKIKEKSNLYS | KIKEKSNLY |
| | | IKTLIAKIKEKSNLY | LIAKIKEKS |
| | | TLIAKIKEKSNLYSA | KIKEKSNLY |
| | | LIAKIKEKSNLYSAY | KIKEKSNLY |
| | | IAKIKEKSNLYSAYK | KIKEKSNLY |
| | | NKIYDTYTLFSTKLT | IYDTYTLFS |
| | | FKTLLNYIQVSVKTA | NYIQVSVKT |
| | | TINKIYDTYTLFSTK | IYDTYTLFS |
| | | NTINKIYDTYTLFST | IYDTYTLFS |
| | | INTINKIYDTYTLFS | INKIYDTYT |
| | | INKIYDTYTLFSTKL | IYDTYTLFS |
| | | FVYINEMHAKRKLEN | FVYINEMHA |
| | | NFKTLLNYIQVSVKT | FKTLLNYIQ |
| | | NFVYINEMHAKRKLE | FVYINEMHA |
| | | LLNYIQVSVKTATNF | NYIQVSVKT |
| | | TLLNYIQVSVKTATN | NYIQVSVKT |

| | | | |
|---|---|---|---|
| | | SAYKAIVSSILLMRD | YKAIVSSIL |
| | | YSAYKAIVSSILLMR | YKAIVSSIL |
| | | KTLLNYIQVSVKTAT | NYIQVSVKT |
| | HLA-DRB1*0404 | ATNFVYINEMHAKRK | FVYINEMHA |
| | | TNFVYINEMHAKRKL | FVYINEMHA |
| | | TATNFVYINEMHAKR | FVYINEMHA |
| | HLA-DRB1*0405 | EDLEKNFKTLLNYIQ | EKNFKTLLN |
| | | DLEKNFKTLLNYIQV | EKNFKTLLN |
| | | FTKEDLEKNFKTLLN | LEKNFKTLL |
| | | TKEDLEKNFKTLLNY | EKNFKTLLN |
| | | NKIYDTYTLFSTKLT | IYDTYTLFS |
| | | KEDLEKNFKTLLNYI | EKNFKTLLN |
| | | LEKNFKTLLNYIQVS | EKNFKTLLN |
| | | EKNFKTLLNYIQVSV | EKNFKTLLN |
| | | IYDTYTLFSTKLTQM | YTLFSTKLT |
| | | KIYDTYTLFSTKLTQ | YTLFSTKLT |
| | | INTINKIYDTYTLFS | INTINKIYD |
| | | YDTYTLFSTKLTQMY | YTLFSTKLT |
| | | ITIDSDLRPKSSLQD | ITIDSDLRP |
| | | DTYTLFSTKLTQMYS | YTLFSTKLT |
| | | TIDSDLRPKSSLQDI | LRPKSSLQD |
| | | IDSDLRPKSSLQDIA | LRPKSSLQD |
| | HLA-DRB1*0701 | LYSAYKAIVSSILLM | YKAIVSSIL |
| | | YSAYKAIVSSILLMR | YKAIVSSIL |
| | | SAYKAIVSSILLMRD | YKAIVSSIL |
| | | NLYSAYKAIVSSILL | YKAIVSSIL |
| | | SNLYSAYKAIVSSIL | YSAYKAIVS |
| | | YIQVSVKTATNFVYI | VKTATNFVY |
| | | IQVSVKTATNFVYIN | VKTATNFVY |
| | | NYIQVSVKTATNFVY | SVKTATNFV |
| | | QVSVKTATNFVYINE | VKTATNFVY |
| | | VSVKTATNFVYINEM | VKTATNFVY |
| | | AYKAIVSSILLMRDS | YKAIVSSIL |
| | | YKAIVSSILLMRDSL | YKAIVSSIL |
| | | SVKTATNFVYINEMH | VKTATNFVY |
| | | LNYIQVSVKTATNFV | VSVKTATNF |
| | | VKTATNFVYINEMHA | VKTATNFVY |
| | HLA-DRB1*0802 | ESIKKPMNKKGKGKI | SIKKPMNKK |
| | HLA-DRB1*0901 | SNLYSAYKAIVSSIL | YSAYKAIVS |
| | | LYSAYKAIVSSILLM | YKAIVSSIL |
| | | YSAYKAIVSSILLMR | AIVSSILLM |
| | | YDTYTLFSTKLTQMY | YTLFSTKLT |
| | | DTYTLFSTKLTQMYS | YTLFSTKLT |
| | | IYDTYTLFSTKLTQM | YTLFSTKLT |
| | | NLYSAYKAIVSSILL | YSAYKAIVS |
| | | KIYDTYTLFSTKLTQ | YTLFSTKLT |
| | | NKIYDTYTLFSTKLT | IYDTYTLFS |
| | HLA-DRB1*1101 | None | |
| | HLA-DRB1*1302 | NYIQVSVKTATNFVY | IQVSVKTAT |
| | | KTLLNYIQVSVKTAT | LLNYIQVSV |
| | | YDTYTLFSTKLTQMY | YTLFSTKLT |
| | | DTYTLFSTKLTQMYS | YTLFSTKLT |
| | | TLLNYIQVSVKTATN | IQVSVKTAT |
| | | LLNYIQVSVKTATNF | IQVSVKTAT |
| | | LNYIQVSVKTATNFV | IQVSVKTAT |
| | | IYDTYTLFSTKLTQM | YTLFSTKLT |

| | | | |
|---|---|---|---|
| | | YIQVSVKTATNFVYI | IQVSVKTAT |
| | | IQVSVKTATNFVYIN | IQVSVKTAT |
| | | NKIYDTYTLFSTKLT | NKIYDTYTL |
| | | ENIEAKIKTLIAKIK | IEAKIKTLI |
| | | KIYDTYTLFSTKLTQ | YTLFSTKLT |
| | | NIEAKIKTLIAKIKE | IKTLIAKIK |
| | | IEAKIKTLIAKIKEK | IKTLIAKIK |
| | | YTLFSTKLTQMYSTR | YTLFSTKLT |
| | | TYTLFSTKLTQMYST | YTLFSTKLT |
| | | SAYKAIVSSILLMRD | IVSSILLMR |
| | | KNFKTLLNYIQVSVK | LLNYIQVSV |
| | | YSAYKAIVSSILLMR | YKAIVSSIL |
| | | FKTLLNYIQVSVKTA | LLNYIQVSV |
| | | EKNFKTLLNYIQVSV | FKTLLNYIQ |
| | | EAKIKTLIAKIKEKS | IKTLIAKIK |
| | | NFKTLLNYIQVSVKT | LNYIQVSVK |
| | | AKIKTLIAKIKEKSN | IKTLIAKIK |
| | | AYKAIVSSILLMRDS | IVSSILLMR |
| | | DYEEIRLSNRYQSYL | IRLSNRYQS |
| | | KSKYLALGLLFGFIS | YLALGLLFG |
| | | YKAIVSSILLMRDSL | IVSSILLMR |
| | | YEEIRLSNRYQSYLE | IRLSNRYQS |
| | | DDYEEIRLSNRYQSY | IRLSNRYQS |
| | | EDDYEEIRLSNRYQS | EEIRLSNRY |
| | | SKYLALGLLFGFISC | LGLLFGFIS |
| | | EEIRLSNRYQSYLEG | IRLSNRYQS |
| | | ANKSNFLQKNVMLEE | SNFLQKNVM |
| | | EDLEKNFKTLLNYIQ | EKNFKTLLN |
| | | NKSNFLQKNVMLEEE | SNFLQKNVM |
| | | KYLALGLLFGFISCD | LGLLFGFIS |
| | | VDSAINTINKIYDTY | INTINKIYD |
| | | YNVDSAINTINKIYD | SAINTINKI |
| | | DLEKNFKTLLNYIQV | FKTLLNYIQ |
| | | LALGLLFGFISCDLF | LGLLFGFIS |
| | | SAINTINKIYDTYTL | INTINKIYD |
| | | KAIVSSILLMRDSLK | IVSSILLMR |
| | | YLALGLLFGFISCDL | LGLLFGFIS |
| | | NVDSAINTINKIYDT | INTINKIYD |
| | | DSAINTINKIYDTYT | INTINKIYD |
| | | RKLENIEAKIKTLIA | IEAKIKTLI |
| | | KLENIEAKIKTLIAK | IEAKIKTLI |
| | | KRKLENIEAKIKTLI | ENIEAKIKT |
| | | LEKNFKTLLNYIQVS | FKTLLNYIQ |
| | | LENIEAKIKTLIAKI | IEAKIKTLI |
| | | LYSAYKAIVSSILLM | YKAIVSSIL |
| | | IKTLIAKIKEKSNLY | IKTLIAKIK |
| | | KSNFLQKNVMLEEES | SNFLQKNVM |
| | HLA-DRB1*1501 | KAIVSSILLMRDSLK | IVSSILLMR |
| | | IVSSILLMRDSLKEV | IVSSILLMR |
| | | AIVSSILLMRDSLKE | IVSSILLMR |
| | | YSAYKAIVSSILLMR | YKAIVSSIL |
| | | SAYKAIVSSILLMRD | IVSSILLMR |
| | | AYKAIVSSILLMRDS | IVSSILLMR |
| | | YKAIVSSILLMRDSL | IVSSILLMR |
| | | KKGKGKIARKNGKSK | KKGKGKIAR |
| | | VSSILLMRDSLKEVQ | ILLMRDSLK |
| | | SSILLMRDSLKEVQY | ILLMRDSLK |

|  |  | GKGKIARKNGKSKVS | IARKNGKSK |
|---|---|---|---|
|  |  | KGKIARKNGKSKVSG | IARKNGKSK |
|  | HLA-DRB3*0101 | None | |
|  | HLA-DRB4*0101 | GKSKVSGKEPFIHSF | VSGKEPFIH |
|  |  | KNGKSKVSGKEPFIH | SKVSGKEPF |
|  |  | NGKSKVSGKEPFIHS | VSGKEPFIH |
|  |  | KSKVSGKEPFIHSFK | VSGKEPFIH |
|  |  | SKVSGKEPFIHSFKR | VSGKEPFIH |
|  |  | VSGKEPFIHSFKRDA | VSGKEPFIH |
|  | HLA-DRB5*0101 | ATNFVYINEMHAKRK | YINEMHAKR |
|  |  | TNFVYINEMHAKRKL | INEMHAKRK |
|  |  | NFVYINEMHAKRKLE | INEMHAKRK |
|  |  | FVYINEMHAKRKLEN | INEMHAKRK |
|  |  | VYINEMHAKRKLENI | INEMHAKRK |
|  |  | YINEMHAKRKLENIE | INEMHAKRK |
|  |  | KTELLKEQSETRKEK | LLKEQSETR |
|  |  | TELLKEQSETRKEKI | LLKEQSETR |
|  |  | TSEESIKKPMNKKGK | IKKPMNKKG |
|  |  | SEESIKKPMNKKGKG | KKPMNKKGK |
|  |  | SLKTELLKEQSETRK | LLKEQSETR |
|  |  | LKTELLKEQSETRKE | LLKEQSETR |
|  |  | ESIKKPMNKKGKGKI | KKPMNKKGK |
|  |  | EESIKKPMNKKGKGK | KKPMNKKGK |
|  |  | ELLKEQSETRKEKIQ | LKEQSETRK |
|  |  |  | |

**Fig. 35. Summary flow chart ELISPOT**

# Fig. 36. Prediction of cancer antigen BclX(L) specific MHC class 1, 8-, 9-, 10, 11-mer peptide binders

| pos | peptide | logscore | affinity(nM) | Bind Level | Protein Name | Allele |
|---|---|---|---|---|---|---|
| | **8-mers** | | | | | |
| 57 | HLADSPAV | 0.691 | 28 | SB | Sequence | A0201 |
| 213 | FLTGMTVA | 0.687 | 29 | SB | Sequence | A0201 |
| 166 | AAWMATYL | 0.477 | 285 | WB | Sequence | A0201 |
| 160 | VLVSRIAA | 0.463 | 333 | WB | Sequence | A0201 |
| 119 | YQSFEQVV | 0.436 | 448 | WB | Sequence | A0201 |
| 147 | GALCVESV | 0.431 | 472 | WB | Sequence | A0201 |
| 223 | VLLGSLFS | 0.427 | 494 | WB | Sequence | A0201 |
| 213 | FLTGMTVA | 0.777 | 11 | SB | Sequence | A0202 |
| 57 | HLADSPAV | 0.771 | 11 | SB | Sequence | A0202 |
| 119 | YQSFEQVV | 0.590 | 84 | WB | Sequence | A0202 |
| 218 | TVAGVVLL | 0.565 | 110 | WB | Sequence | A0202 |
| 11 | FLSYKLSQ | 0.545 | 137 | WB | Sequence | A0202 |
| 82 | MAAVKQAL | 0.512 | 195 | WB | Sequence | A0202 |
| 73 | SLDAREVI | 0.475 | 294 | WB | Sequence | A0202 |
| 192 | ELYGNNAA | 0.444 | 410 | WB | Sequence | A0202 |
| 217 | MTVAGVVL | 0.440 | 425 | WB | Sequence | A0202 |
| 160 | VLVSRIAA | 0.434 | 454 | WB | Sequence | A0202 |
| 1 | SQSNRELV | 0.434 | 457 | WB | Sequence | A0202 |
| 213 | FLTGMTVA | 0.852 | 4 | SB | Sequence | A0203 |
| 57 | HLADSPAV | 0.831 | 6 | SB | Sequence | A0203 |
| 160 | VLVSRIAA | 0.642 | 48 | SB | Sequence | A0203 |
| 158 | MQVLVSRI | 0.602 | 74 | WB | Sequence | A0203 |
| 11 | FLSYKLSQ | 0.582 | 92 | WB | Sequence | A0203 |
| 133 | GVNWGRIV | 0.581 | 92 | WB | Sequence | A0203 |
| 216 | GMTVAGVV | 0.579 | 94 | WB | Sequence | A0203 |
| 119 | YQSFEQVV | 0.578 | 96 | WB | Sequence | A0203 |
| 164 | RIAAWMAT | 0.573 | 101 | WB | Sequence | A0203 |
| 78 | EVIPMAAV | 0.486 | 261 | WB | Sequence | A0203 |
| 1 | SQSNRELV | 0.481 | 274 | WB | Sequence | A0203 |
| 217 | MTVAGVVL | 0.467 | 318 | WB | Sequence | A0203 |
| 147 | GALCVESV | 0.464 | 328 | WB | Sequence | A0203 |
| 221 | GVVLLGSL | 0.443 | 412 | WB | Sequence | A0203 |
| 218 | TVAGVVLL | 0.440 | 429 | WB | Sequence | A0203 |
| 57 | HLADSPAV | 0.555 | 122 | WB | Sequence | A0204 |
| 153 | SVDKEMQV | 0.431 | 469 | WB | Sequence | A0204 |
| 57 | HLADSPAV | 0.780 | 10 | SB | Sequence | A0206 |
| 158 | MQVLVSRI | 0.733 | 18 | SB | Sequence | A0206 |
| 213 | FLTGMTVA | 0.682 | 31 | SB | Sequence | A0206 |
| 1 | SQSNRELV | 0.677 | 32 | SB | Sequence | A0206 |
| 119 | YQSFEQVV | 0.677 | 33 | SB | Sequence | A0206 |
| 138 | RIVAFFSF | 0.653 | 42 | SB | Sequence | A0206 |
| 164 | RIAAWMAT | 0.575 | 99 | WB | Sequence | A0206 |
| 147 | GALCVESV | 0.568 | 106 | WB | Sequence | A0206 |
| 166 | AAWMATYL | 0.567 | 108 | WB | Sequence | A0206 |
| 217 | MTVAGVVL | 0.563 | 112 | WB | Sequence | A0206 |
| 160 | VLVSRIAA | 0.517 | 185 | WB | Sequence | A0206 |
| 42 | SEMETPSA | 0.514 | 191 | WB | Sequence | A0206 |
| 78 | EVIPMAAV | 0.496 | 233 | WB | Sequence | A0206 |
| 153 | SVDKEMQV | 0.493 | 240 | WB | Sequence | A0206 |
| 57 | HLADSPAV | 0.955 | 1 | SB | Sequence | A0211 |
| 153 | SVDKEMQV | 0.898 | 3 | SB | Sequence | A0211 |
| 213 | FLTGMTVA | 0.893 | 3 | SB | Sequence | A0211 |
| 73 | SLDAREVI | 0.877 | 3 | SB | Sequence | A0211 |
| 192 | ELYGNNAA | 0.834 | 6 | SB | Sequence | A0211 |

| 218 | TVAGVVLL | 0.797 | 8 | SB | Sequence | A0211 |
|-----|----------|-------|-----|-----|----------|-------|
| 172 | YLNDHLEP | 0.751 | 14 | SB | Sequence | A0211 |
| 78 | EVIPMAAV | 0.739 | 16 | SB | Sequence | A0211 |
| 216 | GMTVAGVV | 0.718 | 21 | SB | Sequence | A0211 |
| 160 | VLVSRIAA | 0.684 | 30 | SB | Sequence | A0211 |
| 223 | VLLGSLFS | 0.683 | 30 | SB | Sequence | A0211 |
| 133 | GVNWGRIV | 0.668 | 36 | SB | Sequence | A0211 |
| 212 | WFLTGMTV | 0.668 | 36 | SB | Sequence | A0211 |
| 144 | SFGGALCV | 0.591 | 83 | WB | Sequence | A0211 |
| 72 | SSLDAREV | 0.590 | 84 | WB | Sequence | A0211 |
| 106 | DLTSQLHI | 0.564 | 111 | WB | Sequence | A0211 |
| 119 | YQSFEQVV | 0.545 | 136 | WB | Sequence | A0211 |
| 81 | PMAAVKQA | 0.532 | 158 | WB | Sequence | A0211 |
| 11 | FLSYKLSQ | 0.511 | 198 | WB | Sequence | A0211 |
| 166 | AAWMATYL | 0.456 | 360 | WB | Sequence | A0211 |
| 1 | SQSNRELV | 0.439 | 431 | WB | Sequence | A0211 |
| 147 | GALCVESV | 0.439 | 434 | WB | Sequence | A0211 |
| | | | | | | |
| 57 | HLADSPAV | 0.915 | 2 | SB | Sequence | A0212 |
| 192 | ELYGNNAA | 0.813 | 7 | SB | Sequence | A0212 |
| 213 | FLTGMTVA | 0.801 | 8 | SB | Sequence | A0212 |
| 153 | SVDKEMQV | 0.732 | 18 | SB | Sequence | A0212 |
| 73 | SLDAREVI | 0.714 | 22 | SB | Sequence | A0212 |
| 160 | VLVSRIAA | 0.662 | 38 | SB | Sequence | A0212 |
| 172 | YLNDHLEP | 0.662 | 38 | SB | Sequence | A0212 |
| 119 | YQSFEQVV | 0.586 | 88 | WB | Sequence | A0212 |
| 78 | EVIPMAAV | 0.585 | 88 | WB | Sequence | A0212 |
| 223 | VLLGSLFS | 0.582 | 92 | WB | Sequence | A0212 |
| 11 | FLSYKLSQ | 0.573 | 101 | WB | Sequence | A0212 |
| 212 | WFLTGMTV | 0.541 | 142 | WB | Sequence | A0212 |
| 216 | GMTVAGVV | 0.466 | 321 | WB | Sequence | A0212 |
| | | | | | | |
| 57 | HLADSPAV | 0.892 | 3 | SB | Sequence | A0216 |
| 153 | SVDKEMQV | 0.817 | 7 | SB | Sequence | A0216 |
| 213 | FLTGMTVA | 0.761 | 13 | SB | Sequence | A0216 |
| 192 | ELYGNNAA | 0.715 | 21 | SB | Sequence | A0216 |
| 78 | EVIPMAAV | 0.666 | 37 | SB | Sequence | A0216 |
| 218 | TVAGVVLL | 0.657 | 41 | SB | Sequence | A0216 |
| 73 | SLDAREVI | 0.640 | 49 | SB | Sequence | A0216 |
| 144 | SFGGALCV | 0.630 | 54 | WB | Sequence | A0216 |
| 216 | GMTVAGVV | 0.613 | 65 | WB | Sequence | A0216 |
| 166 | AAWMATYL | 0.603 | 73 | WB | Sequence | A0216 |
| 160 | VLVSRIAA | 0.583 | 91 | WB | Sequence | A0216 |
| 212 | WFLTGMTV | 0.565 | 110 | WB | Sequence | A0216 |
| 11 | FLSYKLSQ | 0.488 | 255 | WB | Sequence | A0216 |
| 106 | DLTSQLHI | 0.487 | 258 | WB | Sequence | A0216 |
| 133 | GVNWGRIV | 0.470 | 308 | WB | Sequence | A0216 |
| 81 | PMAAVKQA | 0.469 | 311 | WB | Sequence | A0216 |
| 118 | AYQSFEQV | 0.461 | 342 | WB | Sequence | A0216 |
| 223 | VLLGSLFS | 0.442 | 417 | WB | Sequence | A0216 |
| 147 | GALCVESV | 0.438 | 436 | WB | Sequence | A0216 |
| | | | | | | |
| 57 | HLADSPAV | 0.924 | 2 | SB | Sequence | A0219 |
| 213 | FLTGMTVA | 0.668 | 36 | SB | Sequence | A0219 |
| 153 | SVDKEMQV | 0.597 | 78 | WB | Sequence | A0219 |
| 73 | SLDAREVI | 0.576 | 98 | WB | Sequence | A0219 |
| 218 | TVAGVVLL | 0.517 | 185 | WB | Sequence | A0219 |
| 192 | ELYGNNAA | 0.486 | 259 | WB | Sequence | A0219 |
| 212 | WFLTGMTV | 0.458 | 352 | WB | Sequence | A0219 |
| 166 | AAWMATYL | 0.455 | 362 | WB | Sequence | A0219 |
| 106 | DLTSQLHI | 0.448 | 390 | WB | Sequence | A0219 |
| 223 | VLLGSLFS | 0.431 | 471 | WB | Sequence | A0219 |
| | | | | | | |
| 12 | LSYKLSQK | 0.761 | 13 | SB | Sequence | A0301 |
| 8 | VVDFLSYK | 0.551 | 128 | WB | Sequence | A0301 |
| 224 | LLGSLFSR | 0.487 | 257 | WB | Sequence | A0301 |

| | | | | | | |
|---|---|---|---|---|---|---|
| 8 | VVDFLSYK | 0.751 | 14 | SB | Sequence | A1101 |
| 12 | LSYKLSQK | 0.721 | 20 | SB | Sequence | A1101 |
| 79 | VIPMAAVK | 0.509 | 203 | WB | Sequence | A1101 |
| 124 | QVVNELFR | 0.472 | 302 | WB | Sequence | A1101 |
| 7 | LVVDFLSY | 0.457 | 355 | WB | Sequence | A1101 |
| 197 | NAAAESRK | 0.455 | 363 | WB | Sequence | A1101 |
| | | | | | | |
| 135 | NWGRIVAF | 0.600 | 75 | WB | Sequence | A2301 |
| 138 | RIVAFFSF | 0.466 | 321 | WB | Sequence | A2301 |
| 222 | VVLLGSLF | 0.461 | 339 | WB | Sequence | A2301 |
| | | | | | | |
| 135 | NWGRIVAF | 0.617 | 62 | WB | Sequence | A2402 |
| | | | | | | |
| 118 | AYQSFEQV | 0.569 | 105 | WB | Sequence | A2403 |
| | | | | | | |
| 78 | EVIPMAAV | 0.598 | 77 | WB | Sequence | A2601 |
| 7 | LVVDFLSY | 0.541 | 144 | WB | Sequence | A2601 |
| | | | | | | |
| 78 | EVIPMAAV | 0.862 | 4 | SB | Sequence | A2602 |
| 7 | LVVDFLSY | 0.797 | 9 | SB | Sequence | A2602 |
| 112 | HITPGTAY | 0.755 | 14 | SB | Sequence | A2602 |
| 97 | ELRYRRAF | 0.589 | 85 | WB | Sequence | A2602 |
| 138 | RIVAFFSF | 0.529 | 164 | WB | Sequence | A2602 |
| | | | | | | |
| 112 | HITPGTAY | 0.597 | 78 | WB | Sequence | A2902 |
| 7 | LVVDFLSY | 0.480 | 276 | WB | Sequence | A2902 |
| | | | | | | |
| 204 | KGQERFNR | 0.743 | 16 | SB | Sequence | A3101 |
| 224 | LLGSLFSR | 0.697 | 26 | SB | Sequence | A3101 |
| 157 | EMQVLVSR | 0.583 | 90 | WB | Sequence | A3101 |
| 70 | HSSSLDAR | 0.577 | 97 | WB | Sequence | A3101 |
| 83 | AAVKQALR | 0.539 | 146 | WB | Sequence | A3101 |
| 95 | EFELRYRR | 0.509 | 201 | WB | Sequence | A3101 |
| 124 | QVVNELFR | 0.453 | 369 | WB | Sequence | A3101 |
| 12 | LSYKLSQK | 0.447 | 397 | WB | Sequence | A3101 |
| | | | | | | |
| 95 | EFELRYRR | 0.823 | 6 | SB | Sequence | A3301 |
| 157 | EMQVLVSR | 0.738 | 16 | SB | Sequence | A3301 |
| 94 | DEFELRYR | 0.650 | 43 | SB | Sequence | A3301 |
| 201 | ESRKGQER | 0.606 | 71 | WB | Sequence | A3301 |
| 224 | LLGSLFSR | 0.538 | 148 | WB | Sequence | A3301 |
| 70 | HSSSLDAR | 0.463 | 332 | WB | Sequence | A3301 |
| | | | | | | |
| 124 | QVVNELFR | 0.803 | 8 | SB | Sequence | A6801 |
| 70 | HSSSLDAR | 0.775 | 11 | SB | Sequence | A6801 |
| 197 | NAAAESRK | 0.681 | 31 | SB | Sequence | A6801 |
| 12 | LSYKLSQK | 0.647 | 45 | SB | Sequence | A6801 |
| 157 | EMQVLVSR | 0.599 | 76 | WB | Sequence | A6801 |
| 196 | NNAAAESR | 0.585 | 88 | WB | Sequence | A6801 |
| 83 | AAVKQALR | 0.535 | 153 | WB | Sequence | A6801 |
| 201 | ESRKGQER | 0.532 | 158 | WB | Sequence | A6801 |
| 165 | IAAWMATY | 0.532 | 158 | WB | Sequence | A6801 |
| 95 | EFELRYRR | 0.511 | 198 | WB | Sequence | A6801 |
| 117 | TAYQSFEQ | 0.507 | 208 | WB | Sequence | A6801 |
| 8 | VVDFLSYK | 0.481 | 273 | WB | Sequence | A6801 |
| 94 | DEFELRYR | 0.457 | 357 | WB | Sequence | A6801 |
| 26 | FSDVEENR | 0.442 | 418 | WB | Sequence | A6801 |
| 224 | LLGSLFSR | 0.430 | 476 | WB | Sequence | A6801 |
| | | | | | | |
| 78 | EVIPMAAV | 0.888 | 3 | SB | Sequence | A6802 |
| 218 | TVAGVVLL | 0.790 | 9 | SB | Sequence | A6802 |
| 215 | TGMTVAGV | 0.742 | 16 | SB | Sequence | A6802 |
| 217 | MTVAGVVL | 0.697 | 26 | SB | Sequence | A6802 |
| 82 | MAAVKQAL | 0.633 | 52 | WB | Sequence | A6802 |
| 57 | HLADSPAV | 0.549 | 131 | WB | Sequence | A6802 |
| 207 | ERFNRWFL | 0.481 | 273 | WB | Sequence | A6802 |
| 60 | DSPAVNGA | 0.473 | 300 | WB | Sequence | A6802 |

| | | | | | | |
|---|---|---|---|---|---|---|
| 192 | ELYGNNAA | 0.447 | 395 | WB | Sequence | A6802 |
| 91 | EAGDEFEL | 0.436 | 444 | WB | Sequence | A6802 |
| | | | | | | |
| 78 | EVIPMAAV | 0.812 | 7 | SB | Sequence | A6901 |
| 57 | HLADSPAV | 0.740 | 16 | SB | Sequence | A6901 |
| 192 | ELYGNNAA | 0.570 | 104 | WB | Sequence | A6901 |
| 217 | MTVAGVVL | 0.544 | 138 | WB | Sequence | A6901 |
| 218 | TVAGVVLL | 0.507 | 206 | WB | Sequence | A6901 |
| 91 | EAGDEFEL | 0.489 | 252 | WB | Sequence | A6901 |
| 153 | SVDKEMQV | 0.437 | 441 | WB | Sequence | A6901 |
| 212 | WFLTGMTV | 0.436 | 445 | WB | Sequence | A6901 |
| | | | | | | |
| 61 | SPAVNGAT | 0.657 | 41 | SB | Sequence | B0702 |
| 82 | MAAVKQAL | 0.468 | 316 | WB | Sequence | B0702 |
| 166 | AAWMATYL | 0.430 | 477 | WB | Sequence | B0702 |
| | | | | | | |
| 97 | ELRYRRAF | 0.589 | 85 | WB | Sequence | B0801 |
| | | | | | | |
| 7 | LVVDFLSY | 0.511 | 198 | WB | Sequence | B1501 |
| 138 | RIVAFFSF | 0.493 | 240 | WB | Sequence | B1501 |
| 112 | HITPGTAY | 0.492 | 243 | WB | Sequence | B1501 |
| 165 | IAAWMATY | 0.473 | 300 | WB | Sequence | B1501 |
| 97 | ELRYRRAF | 0.439 | 430 | WB | Sequence | B1501 |
| 222 | VVLLGSLF | 0.433 | 461 | WB | Sequence | B1501 |
| | | | | | | |
| 206 | QERFNRWF | 0.528 | 165 | WB | Sequence | B1801 |
| 5 | RELVVDFL | 0.517 | 185 | WB | Sequence | B1801 |
| 122 | FEQVVNEL | 0.508 | 205 | WB | Sequence | B1801 |
| | | | | | | |
| 210 | NRWFLTGM | 0.510 | 200 | WB | Sequence | B2705 |
| | | | | | | |
| 165 | IAAWMATY | 0.806 | 8 | SB | Sequence | B3501 |
| 7 | LVVDFLSY | 0.629 | 55 | WB | Sequence | B3501 |
| 82 | MAAVKQAL | 0.591 | 83 | WB | Sequence | B3501 |
| 112 | HITPGTAY | 0.543 | 140 | WB | Sequence | B3501 |
| 75 | DAREVIPM | 0.516 | 187 | WB | Sequence | B3501 |
| 142 | FFSFGGAL | 0.499 | 226 | WB | Sequence | B3501 |
| 61 | SPAVNGAT | 0.478 | 283 | WB | Sequence | B3501 |
| 166 | AAWMATYL | 0.476 | 289 | WB | Sequence | B3501 |
| 217 | MTVAGVVL | 0.470 | 307 | WB | Sequence | B3501 |
| | | | | | | |
| 5 | RELVVDFL | 0.624 | 58 | WB | Sequence | B4001 |
| 122 | FEQVVNEL | 0.618 | 62 | WB | Sequence | B4001 |
| | | | | | | |
| 5 | RELVVDFL | 0.442 | 420 | WB | Sequence | B4002 |
| | | | | | | |
| 156 | KEMQVLVS | 0.430 | 478 | WB | Sequence | B4403 |
| | | | | | | |
| 77 | REVIPMAA | 0.434 | 456 | WB | Sequence | B4501 |
| | | | | | | |
| 161 | LVSRIAAW | 0.626 | 57 | WB | Sequence | B5801 |
| 165 | IAAWMATY | 0.593 | 81 | WB | Sequence | B5801 |
| 16 | LSQKGYSW | 0.586 | 88 | WB | Sequence | B5801 |
| 19 | KGYSWSQF | 0.543 | 141 | WB | Sequence | B5801 |
| 138 | RIVAFFSF | 0.467 | 320 | WB | Sequence | B5801 |
| 49 | AINGNPSW | 0.447 | 394 | WB | Sequence | B5801 |
| | | | | | | |
| | **9.mers** | | | | | |
| | | | | | | |
| 104 | FSDLTSQLH | 0.482 | 270 | WB | Sequence | A0101 |
| | | | | | | |
| 143 | FSFGGALCV | 0.518 | 183 | WB | Sequence | A0201 |
| 217 | MTVAGVVLL | 0.478 | 282 | WB | Sequence | A0201 |
| | | | | | | |
| 172 | YLNDHLEPW | 0.739 | 16 | SB | Sequence | A0202 |
| 217 | MTVAGVVLL | 0.604 | 72 | WB | Sequence | A0202 |
| 165 | IAAWMATYL | 0.568 | 107 | WB | Sequence | A0202 |

| | | | | | | |
|---|---|---|---|---|---|---|
| 213 | FLTGMTVAG | 0.564 | 111 | WB | Sequence | A0202 |
| 11 | FLSYKLSQK | 0.520 | 179 | WB | Sequence | A0202 |
| 161 | LVSRIAAWM | 0.450 | 382 | WB | Sequence | A0202 |
| 8 | VVDFLSYKL | 0.449 | 387 | WB | Sequence | A0202 |
| 192 | ELYGNNAAA | 0.447 | 394 | WB | Sequence | A0202 |
| 81 | PMAAVKQAL | 0.437 | 441 | WB | Sequence | A0202 |
| 216 | GMTVAGVVL | 0.436 | 448 | WB | Sequence | A0202 |
| | | | | | | |
| 214 | LTGMTVAGV | 0.691 | 28 | SB | Sequence | A0203 |
| 217 | MTVAGVVLL | 0.609 | 69 | WB | Sequence | A0203 |
| 165 | IAAWMATYL | 0.530 | 161 | WB | Sequence | A0203 |
| 84 | AVKQALREA | 0.518 | 183 | WB | Sequence | A0203 |
| 110 | QLHITPGTA | 0.507 | 206 | WB | Sequence | A0203 |
| 172 | YLNDHLEPW | 0.493 | 240 | WB | Sequence | A0203 |
| 117 | TAYQSFEQV | 0.473 | 300 | WB | Sequence | A0203 |
| 11 | FLSYKLSQK | 0.447 | 396 | WB | Sequence | A0203 |
| | | | | | | |
| 214 | LTGMTVAGV | 0.504 | 213 | WB | Sequence | A0204 |
| 217 | MTVAGVVLL | 0.475 | 291 | WB | Sequence | A0204 |
| | | | | | | |
| 109 | SQLHITPGT | 0.712 | 22 | SB | Sequence | A0206 |
| 217 | MTVAGVVLL | 0.675 | 33 | SB | Sequence | A0206 |
| 117 | TAYQSFEQV | 0.650 | 43 | SB | Sequence | A0206 |
| 1 | SQSNRELVV | 0.648 | 45 | SB | Sequence | A0206 |
| 143 | FSFGGALCV | 0.584 | 90 | WB | Sequence | A0206 |
| 77 | REVIPMAAV | 0.572 | 103 | WB | Sequence | A0206 |
| 165 | IAAWMATYL | 0.551 | 128 | WB | Sequence | A0206 |
| 158 | MQVLVSRIA | 0.544 | 138 | WB | Sequence | A0206 |
| 214 | LTGMTVAGV | 0.492 | 244 | WB | Sequence | A0206 |
| 172 | YLNDHLEPW | 0.464 | 331 | WB | Sequence | A0206 |
| 42 | SEMETPSAI | 0.440 | 426 | WB | Sequence | A0206 |
| | | | | | | |
| 192 | ELYGNNAAA | 0.863 | 4 | SB | Sequence | A0211 |
| 143 | FSFGGALCV | 0.797 | 8 | SB | Sequence | A0211 |
| 81 | PMAAVKQAL | 0.794 | 9 | SB | Sequence | A0211 |
| 172 | YLNDHLEPW | 0.715 | 21 | SB | Sequence | A0211 |
| 153 | SVDKEMQVL | 0.703 | 24 | SB | Sequence | A0211 |
| 8 | VVDFLSYKL | 0.696 | 26 | SB | Sequence | A0211 |
| 217 | MTVAGVVLL | 0.634 | 52 | WB | Sequence | A0211 |
| 112 | HITPGTAYQ | 0.618 | 62 | WB | Sequence | A0211 |
| 117 | TAYQSFEQV | 0.617 | 63 | WB | Sequence | A0211 |
| 223 | VLLGSLFSR | 0.581 | 93 | WB | Sequence | A0211 |
| 213 | FLTGMTVAG | 0.581 | 93 | WB | Sequence | A0211 |
| 133 | GVNWGRIVA | 0.575 | 99 | WB | Sequence | A0211 |
| 216 | GMTVAGVVL | 0.553 | 126 | WB | Sequence | A0211 |
| 185 | GGWDTFVEL | 0.550 | 130 | WB | Sequence | A0211 |
| 103 | AFSDLTSQL | 0.472 | 302 | WB | Sequence | A0211 |
| 176 | HLEPWIQEN | 0.427 | 493 | WB | Sequence | A0211 |
| | | | | | | |
| 192 | ELYGNNAAA | 0.845 | 5 | SB | Sequence | A0212 |
| 81 | PMAAVKQAL | 0.789 | 9 | SB | Sequence | A0212 |
| 143 | FSFGGALCV | 0.702 | 25 | SB | Sequence | A0212 |
| 172 | YLNDHLEPW | 0.673 | 34 | SB | Sequence | A0212 |
| 223 | VLLGSLFSR | 0.573 | 101 | WB | Sequence | A0212 |
| 8 | VVDFLSYKL | 0.561 | 115 | WB | Sequence | A0212 |
| 153 | SVDKEMQVL | 0.535 | 153 | WB | Sequence | A0212 |
| 213 | FLTGMTVAG | 0.521 | 178 | WB | Sequence | A0212 |
| 118 | AYQSFEQVV | 0.476 | 290 | WB | Sequence | A0212 |
| | | | | | | |
| 192 | ELYGNNAAA | 0.741 | 16 | SB | Sequence | A0216 |
| 81 | PMAAVKQAL | 0.710 | 22 | SB | Sequence | A0216 |
| 143 | FSFGGALCV | 0.652 | 42 | SB | Sequence | A0216 |
| 117 | TAYQSFEQV | 0.593 | 81 | WB | Sequence | A0216 |
| 112 | HITPGTAYQ | 0.512 | 196 | WB | Sequence | A0216 |
| 216 | GMTVAGVVL | 0.430 | 479 | WB | Sequence | A0216 |
| | | | | | | |
| 81 | PMAAVKQAL | 0.675 | 33 | SB | Sequence | A0219 |

| 143 | FSFGGALCV | 0.652 | 43 | SB | Sequence | A0219 |
| 192 | ELYGNNAAA | 0.541 | 142 | WB | Sequence | A0219 |
| 117 | TAYQSFEQV | 0.497 | 232 | WB | Sequence | A0219 |
| 172 | YLNDHLEPW | 0.459 | 348 | WB | Sequence | A0219 |
| 223 | VLLGSLFSR | 0.456 | 361 | WB | Sequence | A0219 |
| 214 | LTGMTVAGV | 0.450 | 384 | WB | Sequence | A0219 |
| --------------- | | | | | | |
| 224 | LLGSLFSRK | 0.762 | 13 | SB | Sequence | A0301 |
| 11 | FLSYKLSQK | 0.710 | 23 | SB | Sequence | A0301 |
| 164 | RIAAWMATY | 0.698 | 26 | SB | Sequence | A0301 |
| 223 | VLLGSLFSR | 0.615 | 64 | WB | Sequence | A0301 |
| 7 | LVVDFLSYK | 0.497 | 231 | WB | Sequence | A0301 |
| | | | | | | |
| 7 | LVVDFLSYK | 0.767 | 12 | SB | Sequence | A1101 |
| 224 | LLGSLFSRK | 0.612 | 66 | WB | Sequence | A1101 |
| 223 | VLLGSLFSR | 0.595 | 79 | WB | Sequence | A1101 |
| 164 | RIAAWMATY | 0.575 | 99 | WB | Sequence | A1101 |
| 148 | ALCVESVDK | 0.529 | 163 | WB | Sequence | A1101 |
| 78 | EVIPMAAVK | 0.509 | 201 | WB | Sequence | A1101 |
| 11 | FLSYKLSQK | 0.430 | 477 | WB | Sequence | A1101 |
| | | | | | | |
| 99 | RYRRAFSDL | 0.690 | 28 | SB | Sequence | A2301 |
| 135 | NWGRIVAFF | 0.644 | 47 | SB | Sequence | A2301 |
| 137 | GRIVAFFSF | 0.459 | 346 | WB | Sequence | A2301 |
| | | | | | | |
| 135 | NWGRIVAFF | 0.739 | 16 | SB | Sequence | A2402 |
| 99 | RYRRAFSDL | 0.550 | 129 | WB | Sequence | A2402 |
| | | | | | | |
| 99 | RYRRAFSDL | 0.748 | 15 | SB | Sequence | A2403 |
| 121 | SFEQVVNEL | 0.557 | 120 | WB | Sequence | A2403 |
| 118 | AYQSFEQVV | 0.487 | 256 | WB | Sequence | A2403 |
| | | | | | | |
| 6 | ELVVDFLSY | 0.532 | 158 | WB | Sequence | A2601 |
| 164 | RIAAWMATY | 0.495 | 235 | WB | Sequence | A2601 |
| | | | | | | |
| 164 | RIAAWMATY | 0.923 | 2 | SB | Sequence | A2602 |
| 6 | ELVVDFLSY | 0.873 | 3 | SB | Sequence | A2602 |
| 161 | LVSRIAAWM | 0.677 | 32 | SB | Sequence | A2602 |
| 153 | SVDKEMQVL | 0.639 | 49 | SB | Sequence | A2602 |
| 78 | EVIPMAAVK | 0.496 | 234 | WB | Sequence | A2602 |
| 217 | MTVAGVVLL | 0.481 | 273 | WB | Sequence | A2602 |
| | | | | | | |
| 111 | LHITPGTAY | 0.553 | 125 | WB | Sequence | A2902 |
| 6 | ELVVDFLSY | 0.539 | 146 | WB | Sequence | A2902 |
| | | | | | | |
| 164 | RIAAWMATY | 0.463 | 334 | WB | Sequence | A3002 |
| | | | | | | |
| 82 | MAAVKQALR | 0.766 | 12 | SB | Sequence | A3101 |
| 223 | VLLGSLFSR | 0.686 | 30 | SB | Sequence | A3101 |
| 7 | LVVDFLSYK | 0.573 | 101 | WB | Sequence | A3101 |
| 156 | KEMQVLVSR | 0.474 | 296 | WB | Sequence | A3101 |
| | | | | | | |
| 94 | DEFELRYRR | 0.721 | 20 | SB | Sequence | A3301 |
| 82 | MAAVKQALR | 0.665 | 37 | SB | Sequence | A3301 |
| 97 | ELRYRRAFS | 0.614 | 65 | WB | Sequence | A3301 |
| 223 | VLLGSLFSR | 0.581 | 92 | WB | Sequence | A3301 |
| 91 | EAGDEFELR | 0.532 | 157 | WB | Sequence | A3301 |
| 25 | QFSDVEENR | 0.531 | 159 | WB | Sequence | A3301 |
| | | | | | | |
| 78 | EVIPMAAVK | 0.848 | 5 | SB | Sequence | A6801 |
| 82 | MAAVKQALR | 0.813 | 7 | SB | Sequence | A6801 |
| 7 | LVVDFLSYK | 0.786 | 10 | SB | Sequence | A6801 |
| 91 | EAGDEFELR | 0.710 | 23 | SB | Sequence | A6801 |
| 123 | EQVVNELFR | 0.635 | 51 | WB | Sequence | A6801 |
| 11 | FLSYKLSQK | 0.558 | 119 | WB | Sequence | A6801 |
| 94 | DEFELRYRR | 0.544 | 139 | WB | Sequence | A6801 |
| 25 | QFSDVEENR | 0.540 | 145 | WB | Sequence | A6801 |

| 196 | NNAAAESRK | 0.474 | 295 | WB | Sequence | A6801 |
|-----|-----------|-------|-----|----|----------|-------|
| 223 | VLLGSLFSR | 0.430 | 477 | WB | Sequence | A6801 |
| | | | | | | |
| 217 | MTVAGVVLL | 0.796 | 9 | SB | Sequence | A6802 |
| 117 | TAYQSFEQV | 0.729 | 18 | SB | Sequence | A6802 |
| 215 | TGMTVAGVV | 0.654 | 42 | SB | Sequence | A6802 |
| 0 | MSQSNRELV | 0.587 | 86 | WB | Sequence | A6802 |
| 21 | YSWSQFSDV | 0.549 | 131 | WB | Sequence | A6802 |
| 143 | FSFGGALCV | 0.526 | 169 | WB | Sequence | A6802 |
| 152 | ESVDKEMQV | 0.525 | 171 | WB | Sequence | A6802 |
| 169 | MATYLNDHL | 0.520 | 180 | WB | Sequence | A6802 |
| 192 | ELYGNNAAA | 0.509 | 202 | WB | Sequence | A6802 |
| 140 | VAFFSFGGA | 0.500 | 222 | WB | Sequence | A6802 |
| 214 | LTGMTVAGV | 0.464 | 330 | WB | Sequence | A6802 |
| 165 | IAAWMATYL | 0.451 | 378 | WB | Sequence | A6802 |
| | | | | | | |
| 217 | MTVAGVVLL | 0.705 | 24 | SB | Sequence | A6901 |
| 117 | TAYQSFEQV | 0.623 | 58 | WB | Sequence | A6901 |
| 192 | ELYGNNAAA | 0.604 | 72 | WB | Sequence | A6901 |
| 143 | FSFGGALCV | 0.589 | 85 | WB | Sequence | A6901 |
| 214 | LTGMTVAGV | 0.557 | 120 | WB | Sequence | A6901 |
| 21 | YSWSQFSDV | 0.489 | 252 | WB | Sequence | A6901 |
| | | | | | | |
| 36 | APEGTESEM | 0.519 | 181 | WB | Sequence | B0702 |
| 61 | SPAVNGATG | 0.454 | 369 | WB | Sequence | B0702 |
| 114 | TPGTAYQSF | 0.450 | 382 | WB | Sequence | B0702 |
| | | | | | | |
| 96 | FELRYRRAF | 0.497 | 229 | WB | Sequence | B0801 |
| | | | | | | |
| 164 | RIAAWMATY | 0.586 | 87 | WB | Sequence | B1501 |
| 88 | ALREAGDEF | 0.520 | 180 | WB | Sequence | B1501 |
| | | | | | | |
| 96 | FELRYRRAF | 0.752 | 14 | SB | Sequence | B1801 |
| 206 | QERFNRWFL | 0.592 | 82 | WB | Sequence | B1801 |
| 122 | FEQVVNELF | 0.523 | 174 | WB | Sequence | B1801 |
| 182 | QENGGWDTF | 0.476 | 290 | WB | Sequence | B1801 |
| | | | | | | |
| 137 | GRIVAFFSF | 0.554 | 124 | WB | Sequence | B2705 |
| 101 | RRAFSDLTS | 0.434 | 459 | WB | Sequence | B2705 |
| | | | | | | |
| 114 | TPGTAYQSF | 0.705 | 24 | SB | Sequence | B3501 |
| 165 | IAAWMATYL | 0.649 | 44 | SB | Sequence | B3501 |
| 36 | APEGTESEM | 0.540 | 144 | WB | Sequence | B3501 |
| 6 | ELVVDFLSY | 0.531 | 159 | WB | Sequence | B3501 |
| 111 | LHITPGTAY | 0.437 | 441 | WB | Sequence | B3501 |
| 53 | NPSWHLADS | 0.429 | 480 | WB | Sequence | B3501 |
| 164 | RIAAWMATY | 0.428 | 485 | WB | Sequence | B3501 |
| | | | | | | |
| 90 | REAGDEFEL | 0.788 | 9 | SB | Sequence | B4001 |
| 206 | QERFNRWFL | 0.597 | 78 | WB | Sequence | B4001 |
| 182 | QENGGWDTF | 0.525 | 170 | WB | Sequence | B4001 |
| 122 | FEQVVNELF | 0.453 | 370 | WB | Sequence | B4001 |
| 96 | FELRYRRAF | 0.446 | 399 | WB | Sequence | B4001 |
| | | | | | | |
| 42 | SEMETPSAI | 0.504 | 215 | WB | Sequence | B4002 |
| 96 | FELRYRRAF | 0.473 | 299 | WB | Sequence | B4002 |
| | | | | | | |
| 182 | QENGGWDTF | 0.434 | 455 | WB | Sequence | B4402 |
| | | | | | | |
| 42 | SEMETPSAI | 0.467 | 319 | WB | Sequence | B4403 |
| 5 | RELVVDFLS | 0.444 | 407 | WB | Sequence | B4403 |
| | | | | | | |
| 77 | REVIPMAAV | 0.438 | 438 | WB | Sequence | B4501 |
| | | | | | | |
| 165 | IAAWMATYL | 0.442 | 416 | WB | Sequence | B5301 |
| | | | | | | |
| 80 | IPMAAVKQA | 0.716 | 21 | SB | Sequence | B5401 |

| | | | | | | |
|---|---|---|---|---|---|---|
| 48 | SAINGNPSW | 0.641 | 48 | SB | Sequence | B5801 |
| 15 | KLSQKGYSW | 0.596 | 79 | WB | Sequence | B5801 |
| 165 | IAAWMATYL | 0.559 | 118 | WB | Sequence | B5801 |
| 172 | YLNDHLEPW | 0.506 | 208 | WB | Sequence | B5801 |
| | **10-mers** | | | | | |
| 104 | FSDLTSQLHI | 0.427 | 492 | WB | Sequence | A0101 |
| 172 | YLNDHLEPWI | 0.866 | 4 | SB | Sequence | A0201 |
| 213 | FLTGMTVAGV | 0.841 | 5 | SB | Sequence | A0201 |
| 164 | RIAAWMATYL | 0.651 | 43 | SB | Sequence | A0201 |
| 168 | WMATYLNDHL | 0.573 | 101 | WB | Sequence | A0201 |
| 7 | LVVDFLSYKL | 0.524 | 173 | WB | Sequence | A0201 |
| 73 | SLDAREVIPM | 0.491 | 246 | WB | Sequence | A0201 |
| 160 | VLVSRIAAWM | 0.486 | 259 | WB | Sequence | A0201 |
| 153 | SVDKEMQVLV | 0.473 | 298 | WB | Sequence | A0201 |
| 216 | GMTVAGVVLL | 0.444 | 411 | WB | Sequence | A0201 |
| 213 | FLTGMTVAGV | 0.811 | 7 | SB | Sequence | A0202 |
| 168 | WMATYLNDHL | 0.772 | 11 | SB | Sequence | A0202 |
| 164 | RIAAWMATYL | 0.763 | 13 | SB | Sequence | A0202 |
| 7 | LVVDFLSYKL | 0.651 | 43 | SB | Sequence | A0202 |
| 102 | RAFSDLTSQL | 0.617 | 63 | WB | Sequence | A0202 |
| 73 | SLDAREVIPM | 0.616 | 63 | WB | Sequence | A0202 |
| 172 | YLNDHLEPWI | 0.587 | 87 | WB | Sequence | A0202 |
| 216 | GMTVAGVVLL | 0.496 | 233 | WB | Sequence | A0202 |
| 145 | FGGALCVESV | 0.480 | 276 | WB | Sequence | A0202 |
| 160 | VLVSRIAAWM | 0.430 | 476 | WB | Sequence | A0202 |
| 213 | FLTGMTVAGV | 0.936 | 2 | SB | Sequence | A0203 |
| 172 | YLNDHLEPWI | 0.891 | 3 | SB | Sequence | A0203 |
| 164 | RIAAWMATYL | 0.837 | 5 | SB | Sequence | A0203 |
| 168 | WMATYLNDHL | 0.647 | 45 | SB | Sequence | A0203 |
| 160 | VLVSRIAAWM | 0.613 | 66 | WB | Sequence | A0203 |
| 139 | IVAFFSFGGA | 0.596 | 79 | WB | Sequence | A0203 |
| 7 | LVVDFLSYKL | 0.581 | 92 | WB | Sequence | A0203 |
| 125 | VVNELFRDGV | 0.570 | 105 | WB | Sequence | A0203 |
| 216 | GMTVAGVVLL | 0.476 | 289 | WB | Sequence | A0203 |
| 102 | RAFSDLTSQL | 0.470 | 308 | WB | Sequence | A0203 |
| 214 | LTGMTVAGVV | 0.468 | 315 | WB | Sequence | A0203 |
| 116 | GTAYQSFEQV | 0.463 | 335 | WB | Sequence | A0203 |
| 213 | FLTGMTVAGV | 0.697 | 26 | SB | Sequence | A0204 |
| 172 | YLNDHLEPWI | 0.664 | 37 | SB | Sequence | A0204 |
| 73 | SLDAREVIPM | 0.477 | 287 | WB | Sequence | A0204 |
| 164 | RIAAWMATYL | 0.476 | 290 | WB | Sequence | A0204 |
| 7 | LVVDFLSYKL | 0.468 | 317 | WB | Sequence | A0204 |
| 49 | AINGNPSWHL | 0.452 | 374 | WB | Sequence | A0204 |
| 213 | FLTGMTVAGV | 0.869 | 4 | SB | Sequence | A0206 |
| 164 | RIAAWMATYL | 0.809 | 7 | SB | Sequence | A0206 |
| 172 | YLNDHLEPWI | 0.722 | 20 | SB | Sequence | A0206 |
| 158 | MQVLVSRIAA | 0.689 | 28 | SB | Sequence | A0206 |
| 7 | LVVDFLSYKL | 0.684 | 30 | SB | Sequence | A0206 |
| 168 | WMATYLNDHL | 0.680 | 31 | SB | Sequence | A0206 |
| 109 | SQLHITPGTA | 0.652 | 43 | SB | Sequence | A0206 |
| 116 | GTAYQSFEQV | 0.572 | 102 | WB | Sequence | A0206 |
| 153 | SVDKEMQVLV | 0.558 | 119 | WB | Sequence | A0206 |
| 102 | RAFSDLTSQL | 0.543 | 140 | WB | Sequence | A0206 |
| 156 | KEMQVLVSRI | 0.508 | 204 | WB | Sequence | A0206 |
| 181 | IQENGGWDTF | 0.508 | 205 | WB | Sequence | A0206 |
| 139 | IVAFFSFGGA | 0.495 | 235 | WB | Sequence | A0206 |
| 125 | VVNELFRDGV | 0.483 | 269 | WB | Sequence | A0206 |
| 117 | TAYQSFEQVV | 0.450 | 383 | WB | Sequence | A0206 |

| | | | | | |
|---|---|---|---|---|---|
| 213 FLTGMTVAGV | 0.966 | 1 | SB | Sequence | A0211 |
| 172 YLNDHLEPWI | 0.951 | 1 | SB | Sequence | A0211 |
| 153 SVDKEMQVLV | 0.905 | 2 | SB | Sequence | A0211 |
| 73 SLDAREVIPM | 0.826 | 6 | SB | Sequence | A0211 |
| 216 GMTVAGVVLL | 0.737 | 17 | SB | Sequence | A0211 |
| 7 LVVDFLSYKL | 0.730 | 18 | SB | Sequence | A0211 |
| 164 RIAAWMATYL | 0.711 | 22 | SB | Sequence | A0211 |
| 125 VVNELFRDGV | 0.687 | 29 | SB | Sequence | A0211 |
| 49 AINGNPSWHL | 0.686 | 29 | SB | Sequence | A0211 |
| 168 WMATYLNDHL | 0.685 | 30 | SB | Sequence | A0211 |
| 117 TAYQSFEQVV | 0.633 | 52 | WB | Sequence | A0211 |
| 160 VLVSRIAAWM | 0.632 | 53 | WB | Sequence | A0211 |
| 142 FFSFGGALCV | 0.567 | 107 | WB | Sequence | A0211 |
| 223 VLLGSLFSRK | 0.498 | 228 | WB | Sequence | A0211 |
| 102 RAFSDLTSQL | 0.453 | 372 | WB | Sequence | A0211 |
| 116 GTAYQSFEQV | 0.429 | 481 | WB | Sequence | A0211 |
| | | | | | |
| 213 FLTGMTVAGV | 0.932 | 2 | SB | Sequence | A0212 |
| 172 YLNDHLEPWI | 0.916 | 2 | SB | Sequence | A0212 |
| 153 SVDKEMQVLV | 0.742 | 16 | SB | Sequence | A0212 |
| 168 WMATYLNDHL | 0.697 | 26 | SB | Sequence | A0212 |
| 125 VVNELFRDGV | 0.695 | 27 | SB | Sequence | A0212 |
| 7 LVVDFLSYKL | 0.648 | 45 | SB | Sequence | A0212 |
| 160 VLVSRIAAWM | 0.604 | 72 | WB | Sequence | A0212 |
| 73 SLDAREVIPM | 0.594 | 80 | WB | Sequence | A0212 |
| 49 AINGNPSWHL | 0.570 | 104 | WB | Sequence | A0212 |
| 164 RIAAWMATYL | 0.550 | 129 | WB | Sequence | A0212 |
| 142 FFSFGGALCV | 0.494 | 238 | WB | Sequence | A0212 |
| 223 VLLGSLFSRK | 0.487 | 258 | WB | Sequence | A0212 |
| 117 TAYQSFEQVV | 0.482 | 270 | WB | Sequence | A0212 |
| 192 ELYGNNAAAE | 0.475 | 293 | WB | Sequence | A0212 |
| 216 GMTVAGVVLL | 0.440 | 426 | WB | Sequence | A0212 |
| | | | | | |
| 213 FLTGMTVAGV | 0.911 | 2 | SB | Sequence | A0216 |
| 172 YLNDHLEPWI | 0.869 | 4 | SB | Sequence | A0216 |
| 153 SVDKEMQVLV | 0.772 | 11 | SB | Sequence | A0216 |
| 168 WMATYLNDHL | 0.696 | 26 | SB | Sequence | A0216 |
| 164 RIAAWMATYL | 0.695 | 27 | SB | Sequence | A0216 |
| 49 AINGNPSWHL | 0.680 | 31 | SB | Sequence | A0216 |
| 160 VLVSRIAAWM | 0.657 | 41 | SB | Sequence | A0216 |
| 7 LVVDFLSYKL | 0.643 | 47 | SB | Sequence | A0216 |
| 73 SLDAREVIPM | 0.617 | 62 | WB | Sequence | A0216 |
| 216 GMTVAGVVLL | 0.588 | 85 | WB | Sequence | A0216 |
| 117 TAYQSFEQVV | 0.530 | 161 | WB | Sequence | A0216 |
| 142 FFSFGGALCV | 0.487 | 256 | WB | Sequence | A0216 |
| 116 GTAYQSFEQV | 0.444 | 408 | WB | Sequence | A0216 |
| | | | | | |
| 213 FLTGMTVAGV | 0.927 | 2 | SB | Sequence | A0219 |
| 172 YLNDHLEPWI | 0.884 | 3 | SB | Sequence | A0219 |
| 168 WMATYLNDHL | 0.611 | 67 | WB | Sequence | A0219 |
| 49 AINGNPSWHL | 0.543 | 140 | WB | Sequence | A0219 |
| 7 LVVDFLSYKL | 0.539 | 146 | WB | Sequence | A0219 |
| 153 SVDKEMQVLV | 0.533 | 156 | WB | Sequence | A0219 |
| 164 RIAAWMATYL | 0.449 | 387 | WB | Sequence | A0219 |
| 73 SLDAREVIPM | 0.445 | 404 | WB | Sequence | A0219 |
| 160 VLVSRIAAWM | 0.441 | 421 | WB | Sequence | A0219 |
| | | | | | |
| 223 VLLGSLFSRK | 0.767 | 12 | SB | Sequence | A0301 |
| 6 ELVVDFLSYK | 0.504 | 213 | WB | Sequence | A0301 |
| 147 GALCVESVDK | 0.488 | 253 | WB | Sequence | A0301 |
| 222 VVLLGSLFSR | 0.457 | 356 | WB | Sequence | A0301 |
| 77 REVIPMAAVK | 0.453 | 372 | WB | Sequence | A0301 |
| | | | | | |
| 223 VLLGSLFSRK | 0.742 | 16 | SB | Sequence | A1101 |
| 222 VVLLGSLFSR | 0.681 | 31 | SB | Sequence | A1101 |
| 147 GALCVESVDK | 0.515 | 190 | WB | Sequence | A1101 |
| 24 SQFSDVEENR | 0.470 | 310 | WB | Sequence | A1101 |

| | | | | | |
|---|---|---|---|---|---|
| 121 SFEQVVNELF | 0.581 | 92 | WB | Sequence | A2301 |
| 171 TYLNDHLEPW | 0.547 | 134 | WB | Sequence | A2301 |
| | | | | | |
| 121 SFEQVVNELF | 0.528 | 165 | WB | Sequence | A2402 |
| 171 TYLNDHLEPW | 0.520 | 180 | WB | Sequence | A2402 |
| 113 ITPGTAYQSF | 0.460 | 343 | WB | Sequence | A2402 |
| | | | | | |
| 171 TYLNDHLEPW | 0.739 | 16 | SB | Sequence | A2403 |
| 121 SFEQVVNELF | 0.546 | 136 | WB | Sequence | A2403 |
| 113 ITPGTAYQSF | 0.508 | 204 | WB | Sequence | A2403 |
| | | | | | |
| 35 EAPEGTESEM | 0.448 | 390 | WB | Sequence | A2601 |
| | | | | | |
| 164 RIAAWMATYL | 0.626 | 57 | WB | Sequence | A2602 |
| 113 ITPGTAYQSF | 0.581 | 92 | WB | Sequence | A2602 |
| 160 VLVSRIAAWM | 0.553 | 126 | WB | Sequence | A2602 |
| 35 EAPEGTESEM | 0.507 | 207 | WB | Sequence | A2602 |
| 152 ESVDKEMQVL | 0.490 | 249 | WB | Sequence | A2602 |
| 95 EFELRYRRAF | 0.483 | 268 | WB | Sequence | A2602 |
| | | | | | |
| 110 QLHITPGTAY | 0.506 | 209 | WB | Sequence | A2902 |
| | | | | | |
| 222 VVLLGSLFSR | 0.683 | 30 | SB | Sequence | A3101 |
| 129 LFRDGVNWGR | 0.667 | 36 | SB | Sequence | A3101 |
| 202 SRKGQERFNR | 0.608 | 69 | WB | Sequence | A3101 |
| 81 PMAAVKQALR | 0.521 | 177 | WB | Sequence | A3101 |
| | | | | | |
| 222 VVLLGSLFSR | 0.572 | 103 | WB | Sequence | A3301 |
| 129 LFRDGVNWGR | 0.553 | 126 | WB | Sequence | A3301 |
| 10 DFLSYKLSQK | 0.470 | 308 | WB | Sequence | A3301 |
| | | | | | |
| 6 ELVVDFLSYK | 0.702 | 25 | SB | Sequence | A6801 |
| 24 SQFSDVEENR | 0.532 | 158 | WB | Sequence | A6801 |
| 222 VVLLGSLFSR | 0.516 | 188 | WB | Sequence | A6801 |
| 194 YGNNAAAESR | 0.493 | 240 | WB | Sequence | A6801 |
| 78 EVIPMAAVKQ | 0.454 | 368 | WB | Sequence | A6801 |
| 169 MATYLNDHLE | 0.448 | 394 | WB | Sequence | A6801 |
| | | | | | |
| 139 IVAFFSFGGA | 0.742 | 16 | SB | Sequence | A6802 |
| 116 GTAYQSFEQV | 0.673 | 34 | SB | Sequence | A6802 |
| 7 LVVDFLSYKL | 0.659 | 39 | SB | Sequence | A6802 |
| 120 QSFEQVVNEL | 0.618 | 62 | WB | Sequence | A6802 |
| 213 FLTGMTVAGV | 0.577 | 96 | WB | Sequence | A6802 |
| 117 TAYQSFEQVV | 0.561 | 115 | WB | Sequence | A6802 |
| 164 RIAAWMATYL | 0.519 | 182 | WB | Sequence | A6802 |
| 65 NGATGHSSSL | 0.496 | 234 | WB | Sequence | A6802 |
| 218 TVAGVVLLGS | 0.491 | 246 | WB | Sequence | A6802 |
| 145 FGGALCVESV | 0.474 | 294 | WB | Sequence | A6802 |
| 125 VVNELFRDGV | 0.465 | 328 | WB | Sequence | A6802 |
| 161 LVSRIAAWMA | 0.451 | 380 | WB | Sequence | A6802 |
| 215 TGMTVAGVVL | 0.450 | 382 | WB | Sequence | A6802 |
| | | | | | |
| 153 SVDKEMQVLV | 0.534 | 155 | WB | Sequence | A6901 |
| 213 FLTGMTVAGV | 0.527 | 166 | WB | Sequence | A6901 |
| 117 TAYQSFEQVV | 0.466 | 324 | WB | Sequence | A6901 |
| 7 LVVDFLSYKL | 0.451 | 378 | WB | Sequence | A6901 |
| 164 RIAAWMATYL | 0.443 | 412 | WB | Sequence | A6901 |
| 116 GTAYQSFEQV | 0.427 | 493 | WB | Sequence | A6901 |
| | | | | | |
| 80 IPMAAVKQAL | 0.704 | 24 | SB | Sequence | B0702 |
| | | | | | |
| 17 SQKGYSWSQF | 0.572 | 102 | WB | Sequence | B1501 |
| 110 QLHITPGTAY | 0.557 | 121 | WB | Sequence | B1501 |
| 133 GVNWGRIVAF | 0.548 | 132 | WB | Sequence | B1501 |
| 181 IQENGGWDTF | 0.522 | 175 | WB | Sequence | B1501 |
| 12 LSYKLSQKGY | 0.455 | 364 | WB | Sequence | B1501 |

| | | | | | |
|---|---|---|---|---|---|
| 5 RELVVDFLSY | 0.759 | 13 | SB | Sequence | B1801 |
| 163 SRIAAWMATY | 0.588 | 86 | WB | Sequence | B2705 |
| 101 RRAFSDLTSQ | 0.461 | 340 | WB | Sequence | B2705 |
| 80 IPMAAVKQAL | 0.609 | 69 | WB | Sequence | B3501 |
| 178 EPWIQENGGW | 0.604 | 72 | WB | Sequence | B3501 |
| 91 EAGDEFELRY | 0.566 | 109 | WB | Sequence | B3501 |
| 35 EAPEGTESEM | 0.563 | 112 | WB | Sequence | B3501 |
| 87 QALREAGDEF | 0.508 | 206 | WB | Sequence | B3501 |
| 61 SPAVNGATGH | 0.490 | 249 | WB | Sequence | B3501 |
| 46 TPSAINGNPS | 0.483 | 269 | WB | Sequence | B3501 |
| 133 GVNWGRIVAF | 0.455 | 362 | WB | Sequence | B3501 |
| 140 VAFFSFGGAL | 0.454 | 367 | WB | Sequence | B3501 |
| 190 FVELYGNNAA | 0.439 | 430 | WB | Sequence | B3501 |
| 200 AESRKGQERF | 0.453 | 370 | WB | Sequence | B4501 |
| 178 EPWIQENGGW | 0.603 | 73 | WB | Sequence | B5301 |
| 2 QSNRELVVDF | 0.474 | 295 | WB | Sequence | B5801 |
| 159 QVLVSRIAAW | 0.467 | 320 | WB | Sequence | B5801 |
| 47 PSAINGNPSW | 0.437 | 444 | WB | Sequence | B5801 |
| **11-mers** | | | | | |
| 213 FLTGMTVAGVV | 0.627 | 56 | WB | Sequence | A0201 |
| 73 SLDAREVIPMA | 0.561 | 115 | WB | Sequence | A0201 |
| 160 VLVSRIAAWMA | 0.547 | 135 | WB | Sequence | A0201 |
| 57 HLADSPAVNGA | 0.539 | 147 | WB | Sequence | A0201 |
| 172 YLNDHLEPWIQ | 0.480 | 278 | WB | Sequence | A0201 |
| 88 ALREAGDEFEL | 0.470 | 309 | WB | Sequence | A0201 |
| 119 YQSFEQVVNEL | 0.426 | 497 | WB | Sequence | A0201 |
| 57 HLADSPAVNGA | 0.763 | 12 | SB | Sequence | A0202 |
| 213 FLTGMTVAGVV | 0.754 | 14 | SB | Sequence | A0202 |
| 119 YQSFEQVVNEL | 0.734 | 17 | SB | Sequence | A0202 |
| 88 ALREAGDEFEL | 0.679 | 32 | SB | Sequence | A0202 |
| 172 YLNDHLEPWIQ | 0.611 | 67 | WB | Sequence | A0202 |
| 139 IVAFFSFGGAL | 0.558 | 119 | WB | Sequence | A0202 |
| 218 TVAGVVLLGSL | 0.537 | 149 | WB | Sequence | A0202 |
| 160 VLVSRIAAWMA | 0.525 | 170 | WB | Sequence | A0202 |
| 15 KLSQKGYSWSQ | 0.450 | 382 | WB | Sequence | A0202 |
| 6 ELVVDFLSYKL | 0.449 | 387 | WB | Sequence | A0202 |
| 73 SLDAREVIPMA | 0.446 | 401 | WB | Sequence | A0202 |
| 213 FLTGMTVAGVV | 0.864 | 4 | SB | Sequence | A0203 |
| 57 HLADSPAVNGA | 0.844 | 5 | SB | Sequence | A0203 |
| 138 RIVAFFSFGGA | 0.752 | 14 | SB | Sequence | A0203 |
| 160 VLVSRIAAWMA | 0.649 | 44 | SB | Sequence | A0203 |
| 218 TVAGVVLLGSL | 0.619 | 61 | WB | Sequence | A0203 |
| 88 ALREAGDEFEL | 0.604 | 72 | WB | Sequence | A0203 |
| 119 YQSFEQVVNEL | 0.567 | 108 | WB | Sequence | A0203 |
| 172 YLNDHLEPWIQ | 0.565 | 110 | WB | Sequence | A0203 |
| 49 AINGNPSWHLA | 0.562 | 114 | WB | Sequence | A0203 |
| 209 FNRWFLTGMTV | 0.494 | 239 | WB | Sequence | A0203 |
| 139 IVAFFSFGGAL | 0.487 | 257 | WB | Sequence | A0203 |
| 73 SLDAREVIPMA | 0.480 | 276 | WB | Sequence | A0203 |
| 82 MAAVKQALREA | 0.430 | 477 | WB | Sequence | A0203 |
| 124 QVVNELFRDGV | 0.426 | 496 | WB | Sequence | A0203 |
| 213 FLTGMTVAGVV | 0.584 | 90 | WB | Sequence | A0204 |
| 88 ALREAGDEFEL | 0.526 | 168 | WB | Sequence | A0204 |
| 160 VLVSRIAAWMA | 0.517 | 185 | WB | Sequence | A0204 |
| 172 YLNDHLEPWIQ | 0.517 | 186 | WB | Sequence | A0204 |

| | | | | | |
|---|---|---|---|---|---|
| 73 | SLDAREVIPMA | 0.485 | 263 | WB | Sequence | A0204 |
| | | | | | |
| 213 | FLTGMTVAGVV | 0.746 | 15 | SB | Sequence | A0206 |
| 138 | RIVAFFSFGGA | 0.725 | 19 | SB | Sequence | A0206 |
| 181 | IQENGGWDTFV | 0.704 | 24 | SB | Sequence | A0206 |
| 119 | YQSFEQVVNEL | 0.671 | 35 | SB | Sequence | A0206 |
| 48 | SAINGNPSWHL | 0.664 | 38 | SB | Sequence | A0206 |
| 124 | QVVNELFRDGV | 0.619 | 62 | WB | Sequence | A0206 |
| 160 | VLVSRIAAWMA | 0.584 | 90 | WB | Sequence | A0206 |
| 86 | KQALREAGDEF | 0.547 | 134 | WB | Sequence | A0206 |
| 57 | HLADSPAVNGA | 0.509 | 201 | WB | Sequence | A0206 |
| 218 | TVAGVVLLGSL | 0.456 | 358 | WB | Sequence | A0206 |
| 88 | ALREAGDEFEL | 0.442 | 420 | WB | Sequence | A0206 |
| 109 | SQLHITPGTAY | 0.441 | 422 | WB | Sequence | A0206 |
| | | | | | |
| 213 | FLTGMTVAGVV | 0.943 | 1 | SB | Sequence | A0211 |
| 73 | SLDAREVIPMA | 0.876 | 3 | SB | Sequence | A0211 |
| 172 | YLNDHLEPWIQ | 0.852 | 4 | SB | Sequence | A0211 |
| 88 | ALREAGDEFEL | 0.799 | 8 | SB | Sequence | A0211 |
| 57 | HLADSPAVNGA | 0.787 | 10 | SB | Sequence | A0211 |
| 160 | VLVSRIAAWMA | 0.759 | 13 | SB | Sequence | A0211 |
| 15 | KLSQKGYSWSQ | 0.743 | 16 | SB | Sequence | A0211 |
| 6 | ELVVDFLSYKL | 0.682 | 31 | SB | Sequence | A0211 |
| 218 | TVAGVVLLGSL | 0.628 | 55 | WB | Sequence | A0211 |
| 49 | AINGNPSWHLA | 0.612 | 66 | WB | Sequence | A0211 |
| 212 | WFLTGMTVAGV | 0.577 | 97 | WB | Sequence | A0211 |
| 141 | AFFSFGGALCV | 0.571 | 103 | WB | Sequence | A0211 |
| 144 | SFGGALCVESV | 0.569 | 105 | WB | Sequence | A0211 |
| 79 | VIPMAAVKQAL | 0.568 | 107 | WB | Sequence | A0211 |
| 124 | QVVNELFRDGV | 0.535 | 152 | WB | Sequence | A0211 |
| 130 | FRDGVNWGRIV | 0.516 | 187 | WB | Sequence | A0211 |
| 48 | SAINGNPSWHL | 0.515 | 189 | WB | Sequence | A0211 |
| 192 | ELYGNNAAAES | 0.504 | 214 | WB | Sequence | A0211 |
| 150 | CVESVDKEMQV | 0.489 | 252 | WB | Sequence | A0211 |
| 116 | GTAYQSFEQVV | 0.452 | 376 | WB | Sequence | A0211 |
| 139 | IVAFFSFGGAL | 0.444 | 411 | WB | Sequence | A0211 |
| | | | | | |
| 213 | FLTGMTVAGVV | 0.824 | 6 | SB | Sequence | A0212 |
| 172 | YLNDHLEPWIQ | 0.812 | 7 | SB | Sequence | A0212 |
| 88 | ALREAGDEFEL | 0.779 | 10 | SB | Sequence | A0212 |
| 73 | SLDAREVIPMA | 0.745 | 15 | SB | Sequence | A0212 |
| 57 | HLADSPAVNGA | 0.714 | 22 | SB | Sequence | A0212 |
| 160 | VLVSRIAAWMA | 0.681 | 31 | SB | Sequence | A0212 |
| 79 | VIPMAAVKQAL | 0.604 | 72 | WB | Sequence | A0212 |
| 15 | KLSQKGYSWSQ | 0.568 | 107 | WB | Sequence | A0212 |
| 212 | WFLTGMTVAGV | 0.504 | 213 | WB | Sequence | A0212 |
| 6 | ELVVDFLSYKL | 0.451 | 380 | WB | Sequence | A0212 |
| 130 | FRDGVNWGRIV | 0.431 | 473 | WB | Sequence | A0212 |
| | | | | | |
| 213 | FLTGMTVAGVV | 0.862 | 4 | SB | Sequence | A0216 |
| 88 | ALREAGDEFEL | 0.821 | 6 | SB | Sequence | A0216 |
| 73 | SLDAREVIPMA | 0.744 | 16 | SB | Sequence | A0216 |
| 160 | VLVSRIAAWMA | 0.649 | 44 | SB | Sequence | A0216 |
| 150 | CVESVDKEMQV | 0.641 | 48 | SB | Sequence | A0216 |
| 57 | HLADSPAVNGA | 0.595 | 79 | WB | Sequence | A0216 |
| 144 | SFGGALCVESV | 0.591 | 83 | WB | Sequence | A0216 |
| 172 | YLNDHLEPWIQ | 0.581 | 92 | WB | Sequence | A0216 |
| 15 | KLSQKGYSWSQ | 0.561 | 115 | WB | Sequence | A0216 |
| 6 | ELVVDFLSYKL | 0.544 | 138 | WB | Sequence | A0216 |
| 218 | TVAGVVLLGSL | 0.534 | 154 | WB | Sequence | A0216 |
| 141 | AFFSFGGALCV | 0.526 | 168 | WB | Sequence | A0216 |
| 181 | IQENGGWDTFV | 0.497 | 231 | WB | Sequence | A0216 |
| 124 | QVVNELFRDGV | 0.490 | 249 | WB | Sequence | A0216 |
| 79 | VIPMAAVKQAL | 0.487 | 257 | WB | Sequence | A0216 |
| 192 | ELYGNNAAAES | 0.478 | 283 | WB | Sequence | A0216 |
| 48 | SAINGNPSWHL | 0.468 | 315 | WB | Sequence | A0216 |
| 212 | WFLTGMTVAGV | 0.437 | 441 | WB | Sequence | A0216 |

| 49 AINGNPSWHLA | 0.436 | 447 | WB | Sequence | A0216 |
|---|---|---|---|---|---|
| | | | | | |
| 213 FLTGMTVAGVV | 0.781 | 10 | SB | Sequence | A0219 |
| 57 HLADSPAVNGA | 0.730 | 18 | SB | Sequence | A0219 |
| 172 YLNDHLEPWIQ | 0.695 | 27 | SB | Sequence | A0219 |
| 73 SLDAREVIPMA | 0.609 | 68 | WB | Sequence | A0219 |
| 88 ALREAGDEFEL | 0.549 | 131 | WB | Sequence | A0219 |
| 212 WFLTGMTVAGV | 0.524 | 172 | WB | Sequence | A0219 |
| 160 VLVSRIAAWMA | 0.499 | 225 | WB | Sequence | A0219 |
| | | | | | |
| 222 VVLLGSLFSRK | 0.688 | 29 | SB | Sequence | A0301 |
| | | | | | |
| 222 VVLLGSLFSRK | 0.786 | 10 | SB | Sequence | A1101 |
| 221 GVVLLGSLFSR | 0.596 | 78 | WB | Sequence | A1101 |
| 67 ATGHSSSLDAR | 0.505 | 212 | WB | Sequence | A1101 |
| 5 RELVVDFLSYK | 0.428 | 489 | WB | Sequence | A1101 |
| | | | | | |
| 135 NWGRIVAFFSF | 0.695 | 26 | SB | Sequence | A2301 |
| 171 TYLNDHLEPWI | 0.576 | 98 | WB | Sequence | A2301 |
| 167 AWMATYLNDHL | 0.534 | 154 | WB | Sequence | A2301 |
| 13 SYKLSQKGYSW | 0.531 | 159 | WB | Sequence | A2301 |
| | | | | | |
| 135 NWGRIVAFFSF | 0.746 | 15 | SB | Sequence | A2402 |
| 171 TYLNDHLEPWI | 0.746 | 15 | SB | Sequence | A2402 |
| 167 AWMATYLNDHL | 0.520 | 180 | WB | Sequence | A2402 |
| | | | | | |
| 171 TYLNDHLEPWI | 0.660 | 39 | SB | Sequence | A2403 |
| 167 AWMATYLNDHL | 0.523 | 174 | WB | Sequence | A2403 |
| 13 SYKLSQKGYSW | 0.523 | 174 | WB | Sequence | A2403 |
| 112 HITPGTAYQSF | 0.431 | 473 | WB | Sequence | A2403 |
| | | | | | |
| 159 QVLVSRIAAWM | 0.640 | 49 | SB | Sequence | A2602 |
| 112 HITPGTAYQSF | 0.573 | 101 | WB | Sequence | A2602 |
| 180 WIQENGGWDTF | 0.518 | 183 | WB | Sequence | A2602 |
| 11 FLSYKLSQKGY | 0.516 | 188 | WB | Sequence | A2602 |
| 132 DGVNWGRIVAF | 0.462 | 336 | WB | Sequence | A2602 |
| 218 TVAGVVLLGSL | 0.443 | 415 | WB | Sequence | A2602 |
| | | | | | |
| 109 SQLHITPGTAY | 0.516 | 188 | WB | Sequence | A2902 |
| | | | | | |
| 222 VVLLGSLFSRK | 0.435 | 453 | WB | Sequence | A3001 |
| 99 RYRRAFSDLTS | 0.428 | 486 | WB | Sequence | A3001 |
| | | | | | |
| 221 GVVLLGSLFSR | 0.598 | 77 | WB | Sequence | A3101 |
| 121 SFEQVVNELFR | 0.552 | 127 | WB | Sequence | A3101 |
| 201 ESRKGQERFNR | 0.511 | 198 | WB | Sequence | A3101 |
| 67 ATGHSSSLDAR | 0.475 | 292 | WB | Sequence | A3101 |
| 5 RELVVDFLSYK | 0.442 | 417 | WB | Sequence | A3101 |
| 193 LYGNNAAAESR | 0.435 | 449 | WB | Sequence | A3101 |
| | | | | | |
| 201 ESRKGQERFNR | 0.690 | 28 | SB | Sequence | A3301 |
| 128 ELFRDGVNWGR | 0.634 | 52 | WB | Sequence | A3301 |
| 91 EAGDEFELRYR | 0.538 | 148 | WB | Sequence | A3301 |
| 121 SFEQVVNELFR | 0.472 | 303 | WB | Sequence | A3301 |
| 221 GVVLLGSLFSR | 0.447 | 396 | WB | Sequence | A3301 |
| 95 EFELRYRRAFS | 0.435 | 449 | WB | Sequence | A3301 |
| | | | | | |
| 128 ELFRDGVNWGR | 0.739 | 16 | SB | Sequence | A6801 |
| 23 WSQFSDVEENR | 0.667 | 36 | SB | Sequence | A6801 |
| 201 ESRKGQERFNR | 0.666 | 36 | SB | Sequence | A6801 |
| 91 EAGDEFELRYR | 0.643 | 47 | SB | Sequence | A6801 |
| 221 GVVLLGSLFSR | 0.638 | 49 | SB | Sequence | A6801 |
| 80 IPMAAVKQALR | 0.566 | 109 | WB | Sequence | A6801 |
| 121 SFEQVVNELFR | 0.536 | 151 | WB | Sequence | A6801 |
| | | | | | |
| 218 TVAGVVLLGSL | 0.796 | 9 | SB | Sequence | A6802 |
| 124 QVVNELFRDGV | 0.712 | 22 | SB | Sequence | A6802 |

| | | | | | |
|---|---|---|---|---|---|
| 139 IVAFFSFGGAL | 0.684 | 30 | SB | Sequence | A6802 |
| 152 ESVDKEMQVLV | 0.613 | 65 | WB | Sequence | A6802 |
| 215 TGMTVAGVVLL | 0.561 | 116 | WB | Sequence | A6802 |
| 6 ELVVDFLSYKL | 0.551 | 129 | WB | Sequence | A6802 |
| 138 RIVAFFSFGGA | 0.548 | 132 | WB | Sequence | A6802 |
| 188 DTFVELYGNNA | 0.530 | 161 | WB | Sequence | A6802 |
| 78 EVIPMAAVKQA | 0.516 | 188 | WB | Sequence | A6802 |
| 116 GTAYQSFEQVV | 0.514 | 191 | WB | Sequence | A6802 |
| 75 DAREVIPMAAV | 0.509 | 203 | WB | Sequence | A6802 |
| 57 HLADSPAVNGA | 0.508 | 206 | WB | Sequence | A6802 |
| 217 MTVAGVVLLGS | 0.480 | 277 | WB | Sequence | A6802 |
| 45 ETPSAINGNPS | 0.479 | 279 | WB | Sequence | A6802 |
| 213 FLTGMTVAGVV | 0.470 | 308 | WB | Sequence | A6802 |
| 70 HSSSLDAREVI | 0.447 | 397 | WB | Sequence | A6802 |
| | | | | | |
| 48 SAINGNPSWHL | 0.541 | 143 | WB | Sequence | A6901 |
| 75 DAREVIPMAAV | 0.527 | 166 | WB | Sequence | A6901 |
| 152 ESVDKEMQVLV | 0.511 | 199 | WB | Sequence | A6901 |
| 57 HLADSPAVNGA | 0.485 | 263 | WB | Sequence | A6901 |
| 54 PSWHLADSPAV | 0.469 | 312 | WB | Sequence | A6901 |
| 212 WFLTGMTVAGV | 0.453 | 369 | WB | Sequence | A6901 |
| 78 EVIPMAAVKQA | 0.442 | 417 | WB | Sequence | A6901 |
| 6 ELVVDFLSYKL | 0.442 | 417 | WB | Sequence | A6901 |
| 218 TVAGVVLLGSL | 0.430 | 475 | WB | Sequence | A6901 |
| 188 DTFVELYGNNA | 0.427 | 494 | WB | Sequence | A6901 |
| | | | | | |
| 139 IVAFFSFGGAL | 0.518 | 183 | WB | Sequence | B0702 |
| 53 NPSWHLADSPA | 0.499 | 224 | WB | Sequence | B0702 |
| 61 SPAVNGATGHS | 0.457 | 355 | WB | Sequence | B0702 |
| | | | | | |
| 109 SQLHITPGTAY | 0.605 | 72 | WB | Sequence | B1501 |
| 86 KQALREAGDEF | 0.567 | 108 | WB | Sequence | B1501 |
| 1 SQSNRELVVDF | 0.540 | 144 | WB | Sequence | B1501 |
| 119 YQSFEQVVNEL | 0.515 | 189 | WB | Sequence | B1501 |
| 158 MQVLVSRIAAW | 0.512 | 196 | WB | Sequence | B1501 |
| 162 VSRIAAWMATY | 0.477 | 285 | WB | Sequence | B1501 |
| 11 FLSYKLSQKGY | 0.474 | 294 | WB | Sequence | B1501 |
| 180 WIQENGGWDTF | 0.473 | 299 | WB | Sequence | B1501 |
| 120 QSFEQVVNELF | 0.450 | 386 | WB | Sequence | B1501 |
| 112 HITPGTAYQSF | 0.447 | 394 | WB | Sequence | B1501 |
| 133 GVNWGRIVAFF | 0.433 | 463 | WB | Sequence | B1501 |
| | | | | | |
| 94 DEFELRYRRAF | 0.829 | 6 | SB | Sequence | B1801 |
| 90 REAGDEFELRY | 0.560 | 116 | WB | Sequence | B1801 |
| 109 SQLHITPGTAY | 0.539 | 146 | WB | Sequence | B1801 |
| 151 VESVDKEMQVL | 0.476 | 288 | WB | Sequence | B1801 |
| 177 LEPWIQENGGW | 0.466 | 321 | WB | Sequence | B1801 |
| 209 FNRWFLTGMTV | 0.444 | 409 | WB | Sequence | B1801 |
| | | | | | |
| 101 RRAFSDLTSQL | 0.563 | 113 | WB | Sequence | B2705 |
| 163 SRIAAWMATYL | 0.470 | 310 | WB | Sequence | B2705 |
| | | | | | |
| 53 NPSWHLADSPA | 0.623 | 59 | WB | Sequence | B3501 |
| 46 TPSAINGNPSW | 0.485 | 263 | WB | Sequence | B3501 |
| 180 WIQENGGWDTF | 0.474 | 297 | WB | Sequence | B3501 |
| 132 DGVNWGRIVAF | 0.442 | 419 | WB | Sequence | B3501 |
| | | | | | |
| 119 YQSFEQVVNEL | 0.610 | 68 | WB | Sequence | B3901 |
| | | | | | |
| 151 VESVDKEMQVL | 0.492 | 243 | WB | Sequence | B4001 |
| | | | | | |
| 40 TESEMETPSAI | 0.446 | 402 | WB | Sequence | B4002 |
| | | | | | |
| 30 EENRTEAPEGT | 0.498 | 228 | WB | Sequence | B4501 |
| | | | | | |
| 46 TPSAINGNPSW | 0.772 | 11 | SB | Sequence | B5301 |

| 53 NPSWHLADSPA | 0.430 | 474 | WB | Sequence | B5401 |
|---|---|---|---|---|---|
|  |  |  |  |  |  |
| 170 ATYLNDHLEPW | 0.540 | 145 | WB | Sequence | B5701 |
|  |  |  |  |  |  |
| 170 ATYLNDHLEPW | 0.537 | 150 | WB | Sequence | B5801 |
| 120 QSFEQVVNELF | 0.495 | 235 | WB | Sequence | B5801 |

# Fig. 37. Prediction of cancer antigen BclX(L) specific MHC class 2, 15-mer peptide binders.

| Allele | pos | peptide | core | 1-log50k(aff) | affinity(nM) | Bind Level | Identity |
|---|---|---|---|---|---|---|---|
| DRB1_0101 | 212 | RWFLTGMTVAGVVLL | LTGMTVAGV | 0.8028 | 8 | SB | BclX(L) |
| DRB1_0101 | 209 | RFNRWFLTGMTVAGV | FLTGMTVAG | 0.7932 | 9 | SB | BclX(L) |
| DRB1_0101 | 210 | FNRWFLTGMTVAGVV | LTGMTVAGV | 0.7940 | 9 | SB | BclX(L) |
| DRB1_0101 | 211 | NRWFLTGMTVAGVVL | LTGMTVAGV | 0.7970 | 9 | SB | BclX(L) |
| DRB1_0101 | 213 | WFLTGMTVAGVVLLG | LTGMTVAGV | 0.7753 | 11 | SB | BclX(L) |
| DRB1_0101 | 76 | DAREVIPMAAVKQAL | VIPMAAVKQ | 0.7755 | 11 | SB | BclX(L) |
| DRB1_0101 | 77 | AREVIPMAAVKQALR | VIPMAAVKQ | 0.7788 | 11 | SB | BclX(L) |
| DRB1_0101 | 78 | REVIPMAAVKQALRE | VIPMAAVKQ | 0.7772 | 11 | SB | BclX(L) |
| DRB1_0101 | 75 | LDAREVIPMAAVKQA | VIPMAAVKQ | 0.7730 | 12 | SB | BclX(L) |
| DRB1_0101 | 157 | KEMQVLVSRIAAWMA | MQVLVSRIA | 0.7458 | 16 | SB | BclX(L) |
| DRB1_0101 | 108 | LTSQLHITPGTAYQS | LHITPGTAY | 0.7338 | 18 | SB | BclX(L) |
| DRB1_0101 | 109 | TSQLHITPGTAYQSF | ITPGTAYQS | 0.7313 | 18 | SB | BclX(L) |
| DRB1_0101 | 74 | SLDAREVIPMAAVKQ | AREVIPMAA | 0.7348 | 18 | SB | BclX(L) |
| DRB1_0101 | 110 | SQLHITPGTAYQSFE | ITPGTAYQS | 0.7287 | 19 | SB | BclX(L) |
| DRB1_0101 | 214 | FLTGMTVAGVVLLGS | LTGMTVAGV | 0.7282 | 19 | SB | BclX(L) |
| DRB1_0101 | 156 | DKEMQVLVSRIAAWM | MQVLVSRIA | 0.7226 | 20 | SB | BclX(L) |
| DRB1_0101 | 111 | QLHITPGTAYQSFEQ | ITPGTAYQS | 0.7202 | 21 | SB | BclX(L) |
| DRB1_0101 | 112 | LHITPGTAYQSFEQV | ITPGTAYQS | 0.7189 | 21 | SB | BclX(L) |
| DRB1_0101 | 154 | SVDKEMQVLVSRIAA | MQVLVSRIA | 0.7165 | 21 | SB | BclX(L) |
| DRB1_0101 | 79 | EVIPMAAVKQALREA | IPMAAVKQA | 0.7208 | 21 | SB | BclX(L) |
| DRB1_0101 | 153 | ESVDKEMQVLVSRIA | VDKEMQVLV | 0.7145 | 22 | SB | BclX(L) |
| DRB1_0101 | 155 | VDKEMQVLVSRIAAW | MQVLVSRIA | 0.7141 | 22 | SB | BclX(L) |
| DRB1_0101 | 215 | LTGMTVAGVVLLGSL | LTGMTVAGV | 0.7090 | 23 | SB | BclX(L) |
| DRB1_0101 | 208 | ERFNRWFLTGMTVAG | FNRWFLTGM | 0.7077 | 24 | SB | BclX(L) |
| DRB1_0101 | 46 | ETPSAINGNPSWHLA | INGNPSWHL | 0.7051 | 24 | SB | BclX(L) |
| DRB1_0101 | 47 | TPSAINGNPSWHLAD | INGNPSWHL | 0.7051 | 24 | SB | BclX(L) |
| DRB1_0101 | 48 | PSAINGNPSWHLADS | INGNPSWHL | 0.7076 | 24 | SB | BclX(L) |
| DRB1_0101 | 49 | SAINGNPSWHLADSP | INGNPSWHL | 0.7072 | 24 | SB | BclX(L) |
| DRB1_0101 | 45 | METPSAINGNPSWHL | PSAINGNPS | 0.7034 | 25 | SB | BclX(L) |
| DRB1_0101 | 158 | EMQVLVSRIAAWMAT | MQVLVSRIA | 0.6845 | 30 | SB | BclX(L) |
| DRB1_0101 | 80 | VIPMAAVKQALREAG | VIPMAAVKQ | 0.6856 | 30 | SB | BclX(L) |
| DRB1_0101 | 159 | MQVLVSRIAAWMATY | MQVLVSRIA | 0.6834 | 31 | SB | BclX(L) |
| DRB1_0101 | 161 | VLVSRIAAWMATYLN | IAAWMATYL | 0.6838 | 31 | SB | BclX(L) |
| DRB1_0101 | 217 | GMTVAGVVLLGSLFS | MTVAGVVLL | 0.6800 | 32 | SB | BclX(L) |
| DRB1_0101 | 218 | MTVAGVVLLGSLFSR | VVLLGSLFS | 0.6800 | 32 | SB | BclX(L) |
| DRB1_0101 | 51 | INGNPSWHLADSPAV | INGNPSWHL | 0.6778 | 33 | SB | BclX(L) |
| DRB1_0101 | 192 | VELYGNNAAAESRKG | YGNNAAAES | 0.6693 | 36 | SB | BclX(L) |
| DRB1_0101 | 219 | TVAGVVLLGSLFSRK | VVLLGSLFS | 0.6677 | 36 | SB | BclX(L) |
| DRB1_0101 | 160 | QVLVSRIAAWMATYL | VSRIAAWMA | 0.6652 | 37 | SB | BclX(L) |
| DRB1_0101 | 191 | FVELYGNNAAAESRK | YGNNAAAES | 0.6658 | 37 | SB | BclX(L) |
| DRB1_0101 | 193 | ELYGNNAAAESRKGQ | YGNNAAAES | 0.6661 | 37 | SB | BclX(L) |
| DRB1_0101 | 99 | LRYRRAFSDLTSQLH | YRRAFSDLT | 0.6657 | 37 | SB | BclX(L) |
| DRB1_0101 | 162 | LVSRIAAWMATYLND | IAAWMATYL | 0.6627 | 38 | SB | BclX(L) |
| DRB1_0101 | 190 | TFVELYGNNAAAESR | YGNNAAAES | 0.6628 | 38 | SB | BclX(L) |
| DRB1_0101 | 189 | DTFVELYGNNAAAES | FVELYGNNA | 0.6613 | 39 | SB | BclX(L) |
| DRB1_0101 | 54 | NPSWHLADSPAVNGA | WHLADSPAV | 0.6617 | 39 | SB | BclX(L) |
| DRB1_0101 | 55 | PSWHLADSPAVNGAT | WHLADSPAV | 0.6611 | 39 | SB | BclX(L) |
| DRB1_0101 | 216 | TGMTVAGVVLLGSLF | MTVAGVVLL | 0.6543 | 42 | SB | BclX(L) |
| DRB1_0101 | 163 | VSRIAAWMATYLNDH | IAAWMATYL | 0.6531 | 43 | SB | BclX(L) |
| DRB1_0101 | 53 | GNPSWHLADSPAVNG | WHLADSPAV | 0.6532 | 43 | SB | BclX(L) |
| DRB1_0101 | 59 | LADSPAVNGATGHSS | LADSPAVNG | 0.6361 | 51 | WB | BclX(L) |
| DRB1_0101 | 98 | ELRYRRAFSDLTSQL | YRRAFSDLT | 0.6357 | 51 | WB | BclX(L) |
| DRB1_0101 | 164 | SRIAAWMATYLNDHL | IAAWMATYL | 0.6357 | 52 | WB | BclX(L) |
| DRB1_0101 | 97 | FELRYRRAFSDLTSQ | YRRAFSDLT | 0.6337 | 53 | WB | BclX(L) |
| DRB1_0101 | 96 | EFELRYRRAFSDLTS | YRRAFSDLT | 0.6321 | 54 | WB | BclX(L) |
| DRB1_0101 | 95 | DEFELRYRRAFSDLT | LRYRRAFSD | 0.6294 | 55 | WB | BclX(L) |
| DRB1_0101 | 140 | IVAFFSFGGALCVES | FSFGGALCV | 0.6203 | 61 | WB | BclX(L) |
| DRB1_0101 | 56 | SWHLADSPAVNGATG | LADSPAVNG | 0.6196 | 61 | WB | BclX(L) |
| DRB1_0101 | 61 | DSPAVNGATGHSSSL | VNGATGHSS | 0.6197 | 61 | WB | BclX(L) |
| DRB1_0101 | 62 | SPAVNGATGHSSSLD | VNGATGHSS | 0.6205 | 61 | WB | BclX(L) |

| | | | | | | | |
|---|---|---|---|---|---|---|---|
| DRB1_0101 | 141 | VAFFSFGGALCVESV | FSFGGALCV | 0.6191 | 62 | WB | BclX(L) |
| DRB1_0101 | 60 | ADSPAVNGATGHSSS | VNGATGHSS | 0.6174 | 63 | WB | BclX(L) |
| DRB1_0101 | 142 | AFFSFGGALCVESVD | FSFGGALCV | 0.6153 | 64 | WB | BclX(L) |
| DRB1_0101 | 57 | WHLADSPAVNGATGH | LADSPAVNG | 0.6162 | 64 | WB | BclX(L) |
| DRB1_0101 | 113 | HITPGTAYQSFEQVV | ITPGTAYQS | 0.6121 | 66 | WB | BclX(L) |
| DRB1_0101 | 114 | ITPGTAYQSFEQVVN | ITPGTAYQS | 0.6131 | 66 | WB | BclX(L) |
| DRB1_0101 | 50 | AINGNPSWHLADSPA | INGNPSWHL | 0.6133 | 66 | WB | BclX(L) |
| DRB1_0101 | 63 | PAVNGATGHSSSLDA | VNGATGHSS | 0.6078 | 70 | WB | BclX(L) |
| DRB1_0101 | 100 | RYRRAFSDLTSQLHI | YRRAFSDLT | 0.6026 | 74 | WB | BclX(L) |
| DRB1_0101 | 101 | YRRAFSDLTSQLHIT | YRRAFSDLT | 0.6021 | 74 | WB | BclX(L) |
| DRB1_0101 | 52 | NGNPSWHLADSPAVN | WHLADSPAV | 0.6004 | 75 | WB | BclX(L) |
| DRB1_0101 | 138 | GRIVAFFSFGGALCV | IVAFFSFGG | 0.5864 | 88 | WB | BclX(L) |
| DRB1_0101 | 194 | LYGNNAAAESRKGQE | YGNNAAAES | 0.5808 | 93 | WB | BclX(L) |
| DRB1_0101 | 165 | RIAAWMATYLNDHLE | AAWMATYLN | 0.5762 | 98 | WB | BclX(L) |
| DRB1_0101 | 195 | YGNNAAAESRKGQER | YGNNAAAES | 0.5731 | 101 | WB | BclX(L) |
| DRB1_0101 | 139 | RIVAFFSFGGALCVE | FSFGGALCV | 0.5729 | 102 | WB | BclX(L) |
| DRB1_0101 | 131 | FRDGVNWGRIVAFFS | VNWGRIVAF | 0.5627 | 114 | WB | BclX(L) |
| DRB1_0101 | 143 | FFSFGGALCVESVDK | FSFGGALCV | 0.5613 | 115 | WB | BclX(L) |
| DRB1_0101 | 144 | FSFGGALCVESVDKE | FSFGGALCV | 0.5583 | 119 | WB | BclX(L) |
| DRB1_0101 | 132 | RDGVNWGRIVAFFSF | VNWGRIVAF | 0.5561 | 122 | WB | BclX(L) |
| DRB1_0101 | 133 | DGVNWGRIVAFFSFG | VNWGRIVAF | 0.5558 | 122 | WB | BclX(L) |
| DRB1_0101 | 81 | IPMAAVKQALREAGD | IPMAAVKQA | 0.5527 | 126 | WB | BclX(L) |
| DRB1_0101 | 102 | RRAFSDLTSQLHITP | FSDLTSQLH | 0.5334 | 156 | WB | BclX(L) |
| DRB1_0101 | 103 | RAFSDLTSQLHITPG | FSDLTSQLH | 0.5314 | 159 | WB | BclX(L) |
| DRB1_0101 | 58 | HLADSPAVNGATGHS | LADSPAVNG | 0.5293 | 163 | WB | BclX(L) |
| DRB1_0101 | 134 | GVNWGRIVAFFSFGG | VNWGRIVAF | 0.5282 | 165 | WB | BclX(L) |
| DRB1_0101 | 166 | IAAWMATYLNDHLEP | IAAWMATYL | 0.5271 | 167 | WB | BclX(L) |
| DRB1_0101 | 64 | AVNGATGHSSSLDAR | VNGATGHSS | 0.5266 | 168 | WB | BclX(L) |
| DRB1_0101 | 135 | VNWGRIVAFFSFGGA | VNWGRIVAF | 0.5248 | 171 | WB | BclX(L) |
| DRB1_0101 | 7 | ELVVDFLSYKLSQKG | VVDFLSYKL | 0.5243 | 172 | WB | BclX(L) |
| DRB1_0101 | 129 | ELFRDGVNWGRIVAF | FRDGVNWGR | 0.5154 | 189 | WB | BclX(L) |
| DRB1_0101 | 65 | VNGATGHSSSLDARE | VNGATGHSS | 0.5156 | 189 | WB | BclX(L) |
| DRB1_0101 | 130 | LFRDGVNWGRIVAFF | VNWGRIVAF | 0.5023 | 218 | WB | BclX(L) |
| DRB1_0101 | 8 | LVVDFLSYKLSQKGY | LSYKLSQKG | 0.4921 | 244 | WB | BclX(L) |
| DRB1_0101 | 9 | VVDFLSYKLSQKGYS | LSYKLSQKG | 0.4892 | 251 | WB | BclX(L) |
| DRB1_0101 | 207 | QERFNRWFLTGMTVA | FNRWFLTGM | 0.4845 | 264 | WB | BclX(L) |
| DRB1_0101 | 42 | ESEMETPSAINGNPS | METPSAING | 0.4626 | 335 | WB | BclX(L) |
| DRB1_0101 | 40 | GTESEMETPSAINGN | METPSAING | 0.4622 | 337 | WB | BclX(L) |
| DRB1_0101 | 107 | DLTSQLHITPGTAYQ | LHITPGTAY | 0.4593 | 347 | WB | BclX(L) |
| DRB1_0101 | 39 | EGTESEMETPSAING | ESEMETPSA | 0.4591 | 348 | WB | BclX(L) |
| DRB1_0101 | 43 | SEMETPSAINGNPSW | METPSAING | 0.4590 | 348 | WB | BclX(L) |
| DRB1_0101 | 106 | SDLTSQLHITPGTAY | SQLHITPGT | 0.4569 | 356 | WB | BclX(L) |
| DRB1_0101 | 10 | VDFLSYKLSQKGYSW | LSYKLSQKG | 0.4565 | 358 | WB | BclX(L) |
| DRB1_0101 | 41 | TESEMETPSAINGNP | METPSAING | 0.4564 | 358 | WB | BclX(L) |
| DRB1_0101 | 167 | AAWMATYLNDHLEPW | AAWMATYLN | 0.4556 | 361 | WB | BclX(L) |
| DRB1_0101 | 11 | DFLSYKLSQKGYSWS | LSYKLSQKG | 0.4515 | 378 | WB | BclX(L) |
| DRB1_0101 | 104 | AFSDLTSQLHITPGT | FSDLTSQLH | 0.4453 | 404 | WB | BclX(L) |
| DRB1_0101 | 105 | FSDLTSQLHITPGTA | FSDLTSQLH | 0.4451 | 405 | WB | BclX(L) |
| DRB1_0101 | 137 | WGRIVAFFSFGGALC | IVAFFSFGG | 0.4311 | 471 | WB | BclX(L) |
| DRB1_0101 | 206 | GQERFNRWFLTGMTV | FNRWFLTGM | 0.4277 | 489 | WB | BclX(L) |
| | | | | | | | |
| DRB1_0401 | 99 | LRYRRAFSDLTSQLH | YRRAFSDLT | 0.5618 | 115 | WB | BclX(L) |
| DRB1_0401 | 97 | FELRYRRAFSDLTSQ | YRRAFSDLT | 0.5300 | 162 | WB | BclX(L) |
| DRB1_0401 | 96 | EFELRYRRAFSDLTS | YRRAFSDLT | 0.5284 | 164 | WB | BclX(L) |
| DRB1_0401 | 95 | DEFELRYRRAFSDLT | DEFELRYRR | 0.5275 | 166 | WB | BclX(L) |
| DRB1_0401 | 98 | ELRYRRAFSDLTSQL | YRRAFSDLT | 0.5259 | 169 | WB | BclX(L) |
| DRB1_0401 | 185 | NGGWDTFVELYGNNA | WDTFVELYG | 0.5189 | 182 | WB | BclX(L) |
| DRB1_0401 | 186 | GGWDTFVELYGNNAA | FVELYGNNA | 0.5180 | 184 | WB | BclX(L) |
| DRB1_0401 | 188 | WDTFVELYGNNAAAE | FVELYGNNA | 0.5159 | 188 | WB | BclX(L) |
| DRB1_0401 | 187 | GWDTFVELYGNNAAA | FVELYGNNA | 0.5157 | 189 | WB | BclX(L) |
| DRB1_0401 | 189 | DTFVELYGNNAAAES | FVELYGNNA | 0.5154 | 189 | WB | BclX(L) |
| DRB1_0401 | 100 | RYRRAFSDLTSQLHI | YRRAFSDLT | 0.4844 | 265 | WB | BclX(L) |
| DRB1_0401 | 101 | YRRAFSDLTSQLHIT | YRRAFSDLT | 0.4813 | 274 | WB | BclX(L) |
| DRB1_0401 | 153 | ESVDKEMQVLVSRIA | KEMQVLVSR | 0.4561 | 360 | WB | BclX(L) |
| DRB1_0401 | 155 | VDKEMQVLVSRIAAW | MQVLVSRIA | 0.4519 | 376 | WB | BclX(L) |
| DRB1_0401 | 208 | ERFNRWFLTGMTVAG | WFLTGMTVA | 0.4512 | 379 | WB | BclX(L) |
| DRB1_0401 | 154 | SVDKEMQVLVSRIAA | MQVLVSRIA | 0.4495 | 386 | WB | BclX(L) |

| | | | | | | | |
|---|---|---|---|---|---|---|---|
| DRB1_0401 | 209 | RFNRWFLTGMTVAGV | FLTGMTVAG | 0.4467 | 398 | WB | BclX(L) |
| DRB1_0401 | 210 | FNRWFLTGMTVAGVV | FLTGMTVAG | 0.4427 | 416 | WB | BclX(L) |
| DRB1_0401 | 157 | KEMQVLVSRIAAWMA | MQVLVSRIA | 0.4419 | 419 | WB | BclX(L) |
| DRB1_0401 | 156 | DKEMQVLVSRIAAWM | MQVLVSRIA | 0.4413 | 422 | WB | BclX(L) |
| DRB1_0401 | 211 | NRWFLTGMTVAGVVL | FLTGMTVAG | 0.4327 | 463 | WB | BclX(L) |
| | | | | | | | |
| DRB1_0404 | 167 | AAWMATYLNDHLEPW | WMATYLNDH | 0.5484 | 132 | WB | BclX(L) |
| DRB1_0404 | 164 | SRIAAWMATYLNDHL | WMATYLNDH | 0.5424 | 141 | WB | BclX(L) |
| DRB1_0404 | 165 | RIAAWMATYLNDHLE | WMATYLNDH | 0.5417 | 142 | WB | BclX(L) |
| DRB1_0404 | 166 | IAAWMATYLNDHLEP | WMATYLNDH | 0.5330 | 156 | WB | BclX(L) |
| DRB1_0404 | 163 | VSRIAAWMATYLNDH | AAWMATYLN | 0.5217 | 177 | WB | BclX(L) |
| DRB1_0404 | 219 | TVAGVVLLGSLFSRK | VVLLGSLFS | 0.5094 | 202 | WB | BclX(L) |
| DRB1_0404 | 209 | RFNRWFLTGMTVAGV | FLTGMTVAG | 0.4902 | 249 | WB | BclX(L) |
| DRB1_0404 | 210 | FNRWFLTGMTVAGVV | FLTGMTVAG | 0.4853 | 262 | WB | BclX(L) |
| DRB1_0404 | 211 | NRWFLTGMTVAGVVL | FLTGMTVAG | 0.4826 | 270 | WB | BclX(L) |
| DRB1_0404 | 208 | ERFNRWFLTGMTVAG | WFLTGMTVA | 0.4761 | 290 | WB | BclX(L) |
| DRB1_0404 | 168 | AWMATYLNDHLEPWI | WMATYLNDH | 0.4694 | 311 | WB | BclX(L) |
| DRB1_0404 | 212 | RWFLTGMTVAGVVLL | FLTGMTVAG | 0.4547 | 365 | WB | BclX(L) |
| DRB1_0404 | 189 | DTFVELYGNNAAAES | FVELYGNNA | 0.4505 | 382 | WB | BclX(L) |
| DRB1_0404 | 187 | GWDTFVELYGNNAAA | FVELYGNNA | 0.4498 | 385 | WB | BclX(L) |
| DRB1_0404 | 169 | WMATYLNDHLEPWIQ | WMATYLNDH | 0.4478 | 393 | WB | BclX(L) |
| DRB1_0404 | 188 | WDTFVELYGNNAAAE | FVELYGNNA | 0.4462 | 400 | WB | BclX(L) |
| DRB1_0404 | 186 | GGWDTFVELYGNNAA | FVELYGNNA | 0.4437 | 411 | WB | BclX(L) |
| DRB1_0404 | 185 | NGGWDTFVELYGNNA | GWDTFVELY | 0.4388 | 434 | WB | BclX(L) |
| | | | | | | | |
| DRB1_0405 | 118 | TAYQSFEQVVNELFR | YQSFEQVVN | 0.5794 | 95 | WB | BclX(L) |
| DRB1_0405 | 117 | GTAYQSFEQVVNELF | YQSFEQVVN | 0.5772 | 97 | WB | BclX(L) |
| DRB1_0405 | 115 | TPGTAYQSFEQVVNE | YQSFEQVVN | 0.5541 | 124 | WB | BclX(L) |
| DRB1_0405 | 116 | PGTAYQSFEQVVNEL | YQSFEQVVN | 0.5538 | 125 | WB | BclX(L) |
| DRB1_0405 | 114 | ITPGTAYQSFEQVVN | AYQSFEQVV | 0.5505 | 129 | WB | BclX(L) |
| DRB1_0405 | 163 | VSRIAAWMATYLNDH | AAWMATYLN | 0.5510 | 129 | WB | BclX(L) |
| DRB1_0405 | 162 | LVSRIAAWMATYLND | AAWMATYLN | 0.5473 | 134 | WB | BclX(L) |
| DRB1_0405 | 161 | VLVSRIAAWMATYLN | IAAWMATYL | 0.5442 | 139 | WB | BclX(L) |
| DRB1_0405 | 164 | SRIAAWMATYLNDHL | AAWMATYLN | 0.5402 | 145 | WB | BclX(L) |
| DRB1_0405 | 99 | LRYRRAFSDLTSQLH | YRRAFSDLT | 0.5100 | 201 | WB | BclX(L) |
| DRB1_0405 | 97 | FELRYRRAFSDLTSQ | YRRAFSDLT | 0.5085 | 204 | WB | BclX(L) |
| DRB1_0405 | 96 | EFELRYRRAFSDLTS | YRRAFSDLT | 0.5069 | 208 | WB | BclX(L) |
| DRB1_0405 | 165 | RIAAWMATYLNDHLE | AAWMATYLN | 0.5055 | 211 | WB | BclX(L) |
| DRB1_0405 | 119 | AYQSFEQVVNELFRD | YQSFEQVVN | 0.4974 | 230 | WB | BclX(L) |
| DRB1_0405 | 120 | YQSFEQVVNELFRDG | YQSFEQVVN | 0.4968 | 231 | WB | BclX(L) |
| DRB1_0405 | 98 | ELRYRRAFSDLTSQL | YRRAFSDLT | 0.4923 | 243 | WB | BclX(L) |
| DRB1_0405 | 18 | SQKGYSWSQFSDVEE | GYSWSQFSD | 0.4575 | 354 | WB | BclX(L) |
| DRB1_0405 | 95 | DEFELRYRRAFSDLT | LRYRRAFSD | 0.4574 | 355 | WB | BclX(L) |
| DRB1_0405 | 19 | QKGYSWSQFSDVEEN | WSQFSDVEE | 0.4562 | 359 | WB | BclX(L) |
| DRB1_0405 | 100 | RYRRAFSDLTSQLHI | YRRAFSDLT | 0.4475 | 395 | WB | BclX(L) |
| DRB1_0405 | 20 | KGYSWSQFSDVEENR | WSQFSDVEE | 0.4430 | 414 | WB | BclX(L) |
| DRB1_0405 | 166 | IAAWMATYLNDHLEP | AAWMATYLN | 0.4423 | 418 | WB | BclX(L) |
| DRB1_0405 | 21 | GYSWSQFSDVEENRT | WSQFSDVEE | 0.4353 | 450 | WB | BclX(L) |
| | | | | | | | |
| DRB1_0701 | 157 | KEMQVLVSRIAAWMA | VLVSRIAAW | 0.5228 | 175 | WB | BclX(L) |
| DRB1_0701 | 159 | MQVLVSRIAAWMATY | VLVSRIAAW | 0.5194 | 181 | WB | BclX(L) |
| DRB1_0701 | 158 | EMQVLVSRIAAWMAT | VLVSRIAAW | 0.5191 | 182 | WB | BclX(L) |
| DRB1_0701 | 156 | DKEMQVLVSRIAAWM | VLVSRIAAW | 0.4971 | 231 | WB | BclX(L) |
| DRB1_0701 | 160 | QVLVSRIAAWMATYL | VLVSRIAAW | 0.4833 | 268 | WB | BclX(L) |
| DRB1_0701 | 46 | ETPSAINGNPSWHLA | INGNPSWHL | 0.4783 | 283 | WB | BclX(L) |
| DRB1_0701 | 45 | METPSAINGNPSWHL | AINGNPSWH | 0.4779 | 284 | WB | BclX(L) |
| DRB1_0701 | 155 | VDKEMQVLVSRIAAW | MQVLVSRIA | 0.4772 | 286 | WB | BclX(L) |
| DRB1_0701 | 47 | TPSAINGNPSWHLAD | INGNPSWHL | 0.4774 | 286 | WB | BclX(L) |
| DRB1_0701 | 48 | PSAINGNPSWHLADS | INGNPSWHL | 0.4764 | 289 | WB | BclX(L) |
| DRB1_0701 | 161 | VLVSRIAAWMATYLN | VLVSRIAAW | 0.4745 | 295 | WB | BclX(L) |
| DRB1_0701 | 49 | SAINGNPSWHLADSP | INGNPSWHL | 0.4718 | 303 | WB | BclX(L) |
| DRB1_0701 | 99 | LRYRRAFSDLTSQLH | YRRAFSDLT | 0.4627 | 335 | WB | BclX(L) |
| DRB1_0701 | 100 | RYRRAFSDLTSQLHI | FSDLTSQLH | 0.4416 | 420 | WB | BclX(L) |
| DRB1_0701 | 101 | YRRAFSDLTSQLHIT | FSDLTSQLH | 0.4344 | 455 | WB | BclX(L) |
| DRB1_0701 | 51 | INGNPSWHLADSPAV | INGNPSWHL | 0.4291 | 481 | WB | BclX(L) |
| | | | | | | | |
| DRB1_0901 | 55 | PSWHLADSPAVNGAT | WHLADSPAV | 0.5482 | 133 | WB | BclX(L) |

| DRB1_0901 | 54 | NPSWHLADSPAVNGA | WHLADSPAV | 0.5414 | 143 | WB | BclX(L) |
|---|---|---|---|---|---|---|---|
| DRB1_0901 | 53 | GNPSWHLADSPAVNG | WHLADSPAV | 0.5408 | 144 | WB | BclX(L) |
| DRB1_0901 | 51 | INGNPSWHLADSPAV | SWHLADSPA | 0.5289 | 164 | WB | BclX(L) |
| DRB1_0901 | 52 | NGNPSWHLADSPAVN | WHLADSPAV | 0.5284 | 164 | WB | BclX(L) |
| DRB1_0901 | 56 | SWHLADSPAVNGATG | WHLADSPAV | 0.4678 | 317 | WB | BclX(L) |
| DRB1_0901 | 57 | WHLADSPAVNGATGH | WHLADSPAV | 0.4636 | 331 | WB | BclX(L) |
| DRB1_0901 | 212 | RWFLTGMTVAGVVLL | FLTGMTVAG | 0.4483 | 391 | WB | BclX(L) |
| DRB1_0901 | 213 | WFLTGMTVAGVVLLG | MTVAGVVLL | 0.4445 | 408 | WB | BclX(L) |
| DRB1_0901 | 214 | FLTGMTVAGVVLLGS | MTVAGVVLL | 0.4327 | 463 | WB | BclX(L) |
| | | | | | | | |
| DRB1_1101 | 157 | KEMQVLVSRIAAWMA | MQVLVSRIA | 0.4319 | 467 | WB | BclX(L) |
| DRB1_1101 | 131 | FRDGVNWGRIVAFFS | GVNWGRIVA | 0.4310 | 472 | WB | BclX(L) |
| DRB1_1101 | 156 | DKEMQVLVSRIAAWM | MQVLVSRIA | 0.4308 | 473 | WB | BclX(L) |
| DRB1_1101 | 155 | VDKEMQVLVSRIAAW | MQVLVSRIA | 0.4292 | 481 | WB | BclX(L) |
| DRB1_1101 | 132 | RDGVNWGRIVAFFSF | GVNWGRIVA | 0.4283 | 486 | WB | BclX(L) |
| DRB1_1101 | 154 | SVDKEMQVLVSRIAA | MQVLVSRIA | 0.4267 | 494 | WB | BclX(L) |
| | | | | | | | |
| DRB1_1302 | 218 | MTVAGVVLLGSLFSR | VVLLGSLFS | 0.4945 | 237 | WB | BclX(L) |
| DRB1_1302 | 216 | TGMTVAGVVLLGSLF | MTVAGVVLL | 0.4855 | 262 | WB | BclX(L) |
| DRB1_1302 | 214 | FLTGMTVAGVVLLGS | MTVAGVVLL | 0.4842 | 265 | WB | BclX(L) |
| DRB1_1302 | 217 | GMTVAGVVLLGSLFS | MTVAGVVLL | 0.4840 | 266 | WB | BclX(L) |
| DRB1_1302 | 212 | RWFLTGMTVAGVVLL | FLTGMTVAG | 0.4832 | 268 | WB | BclX(L) |
| DRB1_1302 | 215 | LTGMTVAGVVLLGSL | MTVAGVVLL | 0.4775 | 285 | WB | BclX(L) |
| DRB1_1302 | 213 | WFLTGMTVAGVVLLG | MTVAGVVLL | 0.4716 | 304 | WB | BclX(L) |
| DRB1_1302 | 189 | DTFVELYGNNAAAES | VELYGNNAA | 0.4688 | 313 | WB | BclX(L) |
| DRB1_1302 | 190 | TFVELYGNNAAAESR | YGNNAAAES | 0.4615 | 339 | WB | BclX(L) |
| DRB1_1302 | 191 | FVELYGNNAAAESRK | YGNNAAAES | 0.4526 | 373 | WB | BclX(L) |
| DRB1_1302 | 192 | VELYGNNAAAESRKG | YGNNAAAES | 0.4415 | 421 | WB | BclX(L) |
| DRB1_1302 | 219 | TVAGVVLLGSLFSRK | VVLLGSLFS | 0.4384 | 436 | WB | BclX(L) |
| DRB1_1302 | 193 | ELYGNNAAAESRKGQ | YGNNAAAES | 0.4275 | 490 | WB | BclX(L) |
| | | | | | | | |
| DRB1_1501 | 219 | TVAGVVLLGSLFSRK | VLLGSLFSR | 0.6681 | 36 | SB | BclX(L) |
| DRB1_1501 | 218 | MTVAGVVLLGSLFSR | VVLLGSLFS | 0.6441 | 47 | SB | BclX(L) |
| DRB1_1501 | 6 | RELVVDFLSYKLSQK | LVVDFLSYK | 0.5208 | 179 | WB | BclX(L) |
| DRB1_1501 | 7 | ELVVDFLSYKLSQKG | FLSYKLSQK | 0.5009 | 221 | WB | BclX(L) |
| DRB1_1501 | 157 | KEMQVLVSRIAAWMA | MQVLVSRIA | 0.4968 | 231 | WB | BclX(L) |
| DRB1_1501 | 156 | DKEMQVLVSRIAAWM | MQVLVSRIA | 0.4965 | 232 | WB | BclX(L) |
| DRB1_1501 | 209 | RFNRWFLTGMTVAGV | FNRWFLTGM | 0.4870 | 257 | WB | BclX(L) |
| DRB1_1501 | 164 | SRIAAWMATYLNDHL | WMATYLNDH | 0.4849 | 263 | WB | BclX(L) |
| DRB1_1501 | 210 | FNRWFLTGMTVAGVV | LTGMTVAGV | 0.4836 | 267 | WB | BclX(L) |
| DRB1_1501 | 8 | LVVDFLSYKLSQKGY | FLSYKLSQK | 0.4778 | 284 | WB | BclX(L) |
| DRB1_1501 | 5 | NRELVVDFLSYKLSQ | LVVDFLSYK | 0.4777 | 285 | WB | BclX(L) |
| DRB1_1501 | 135 | VNWGRIVAFFSFGGA | IVAFFSFGG | 0.4756 | 291 | WB | BclX(L) |
| DRB1_1501 | 4 | SNRELVVDFLSYKLS | LVVDFLSYK | 0.4755 | 291 | WB | BclX(L) |
| DRB1_1501 | 159 | MQVLVSRIAAWMATY | LVSRIAAWM | 0.4693 | 312 | WB | BclX(L) |
| DRB1_1501 | 163 | VSRIAAWMATYLNDH | IAAWMATYL | 0.4691 | 312 | WB | BclX(L) |
| DRB1_1501 | 158 | EMQVLVSRIAAWMAT | VLVSRIAAW | 0.4683 | 315 | WB | BclX(L) |
| DRB1_1501 | 165 | RIAAWMATYLNDHLE | WMATYLNDH | 0.4685 | 315 | WB | BclX(L) |
| DRB1_1501 | 128 | NELFRDGVNWGRIVA | LFRDGVNWG | 0.4675 | 318 | WB | BclX(L) |
| DRB1_1501 | 125 | QVVNELFRDGVNWGR | LFRDGVNWG | 0.4668 | 320 | WB | BclX(L) |
| DRB1_1501 | 126 | VVNELFRDGVNWGRI | LFRDGVNWG | 0.4668 | 320 | WB | BclX(L) |
| DRB1_1501 | 166 | IAAWMATYLNDHLEP | WMATYLNDH | 0.4649 | 327 | WB | BclX(L) |
| DRB1_1501 | 137 | WGRIVAFFSFGGALC | IVAFFSFGG | 0.4643 | 329 | WB | BclX(L) |
| DRB1_1501 | 136 | NWGRIVAFFSFGGAL | IVAFFSFGG | 0.4638 | 331 | WB | BclX(L) |
| DRB1_1501 | 207 | QERFNRWFLTGMTVA | FNRWFLTGM | 0.4608 | 342 | WB | BclX(L) |
| DRB1_1501 | 208 | ERFNRWFLTGMTVAG | FNRWFLTGM | 0.4602 | 344 | WB | BclX(L) |
| DRB1_1501 | 138 | GRIVAFFSFGGALCV | IVAFFSFGG | 0.4587 | 350 | WB | BclX(L) |
| DRB1_1501 | 9 | VVDFLSYKLSQKGYS | FLSYKLSQK | 0.4584 | 351 | WB | BclX(L) |
| DRB1_1501 | 155 | VDKEMQVLVSRIAAW | MQVLVSRIA | 0.4570 | 356 | WB | BclX(L) |
| DRB1_1501 | 167 | AAWMATYLNDHLEPW | WMATYLNDH | 0.4551 | 364 | WB | BclX(L) |
| DRB1_1501 | 3 | QSNRELVVDFLSYKL | LVVDFLSYK | 0.4543 | 367 | WB | BclX(L) |
| DRB1_1501 | 127 | VNELFRDGVNWGRIV | LFRDGVNWG | 0.4431 | 414 | WB | BclX(L) |
| DRB1_1501 | 134 | GVNWGRIVAFFSFGG | VNWGRIVAF | 0.4391 | 432 | WB | BclX(L) |
| DRB1_1501 | 129 | ELFRDGVNWGRIVAF | LFRDGVNWG | 0.4390 | 433 | WB | BclX(L) |
| DRB1_1501 | 217 | GMTVAGVVLLGSLFS | VAGVVLLGS | 0.4377 | 439 | WB | BclX(L) |
| DRB1_1501 | 124 | EQVVNELFRDGVNWG | VVNELFRDG | 0.4373 | 441 | WB | BclX(L) |
| DRB1_1501 | 139 | RIVAFFSFGGALCVE | IVAFFSFGG | 0.4300 | 477 | WB | BclX(L) |

| DRB1_1501 | 211 | NRWFLTGMTVAGVVL | LTGMTVAGV | 0.4264 | 496 | WB | BclX(L) |
|-----------|-----|-----------------|-----------|--------|-----|----|---------|
|           |     |                 |           |        |     |    |         |
| DRB4_0101 | 99  | LRYRRAFSDLTSQLH  | YRRAFSDLT | 0.4654 | 325 | WB | BclX(L) |
| DRB4_0101 | 100 | RYRRAFSDLTSQLHI  | FSDLTSQLH | 0.4328 | 463 | WB | BclX(L) |

# Fig. 38.

| Antigen | Sequence |
|---|---|
| <ABX71745 CRASP-2,Borrelia Burgdorferi><br><br>Seq45 | <ABX71745 CRASP-2;Protein;Borrelia burgdorferi><br>mkksflsiymlisisllscdvsrlnqrnidelkifvekakyysikldaiyseytgayndimtyimtysegts sdkskvnqaisilkkdnkivnkfkelekiieeykpmflskliddfaieldqavdndvsnarhvadsye klrksvalayiesfdvisskfvdskfveaskkfvnkakefveendlialkcivktigdmvndreinsrsry nnfykkeadflgaavelegaykaikqtll |
| <CAH10086 CRASP-1,Borrelia garinii><br><br>Seq 46 | <CAH10086 CRASP-1;Protein;Borrelia garinii><br>mkktklniiklnilttiltliciscavdkidpesksktntkdfenksqdlepsntknkdlepleknsketiiskl ekmikdledqkdqedteiakitntqidflenfksephdtisediemkikriiysslnyeiekintlkeildkl yknhkykttarnfilnisikiqfqienalelmkeeiedaseilnqeryeillkhvepslnlkqkfekilnetik aynqdldniksnedqlahmdenykefdplnldy |
| <AAU07022 4-alpha-glucanotransf erase,Borreli a garinii PBi><br><br>Seq 47 | <AAU07022 4-alpha-glucanotransferase;Protein;Borrelia garinii PBi><br>Mkykkirinlnlkrksgillnisslpskygigdlgkgaykfidflfassqsywqmfayspidftrsppysif safagnvyyidlealdkfidsdlnllkenetrysdlkklsfkdkflkeaalnfinrasvdevrsfekfkkks sywlldfasfvafkeyflkdsrdafnvlfdrgilirnekdlfklrnilskeikvqevlqyfffsqfqalkryand kgielvmnlplfiaydsadvwahqkyfklrfdaskdkvagispdyfleqeqawdspayswnvlknv kyewwakrigilrkyidvikidhfrgfvstwevgvgeayafnglwvkspgrdffnfilneikdlkiwvedf endledvsrlrdffnfpgmrimklafdfdsdnqnlphnyikncivytgigdnstirefvnslddlhkkyif dylntnedfvvwdmirsamssvsdsviipmqdyinlgnefranipkntlnnwifrllesdldatlsknis fitrlygrt |
| <CAA59725 outer surface protein A,Borrelia afzelii><br><br>Seq 48 | <CAA59725 outer surface protein A;Protein;Borrelia afzelii><br>mkkyllgiglilaliackqnvssldeknsasvdlpgemkvlvskekdkdgkyslkatvdkielkgtsdk dngsgvlegtkddkskakltiaddlskttfelfkedgktlvsrkvsskdktstdemfnekgelsaktmtr engtkleytemksdgtgkakevlknftlegkvandkvtlevkegtvtlskeiaksgevtvalndtnttq atkktgawdsktstltisvnskkttqlvftkqdtitvqkydsagtnlegtaveiktldelknalk |
| <CAA59727 outer surface protein A,Borrelia garinii><br><br>Seq 49 | <CAA59727 outer surface protein A;Protein;Borrelia garinii><br>Mkkyllgiglilaliackqnvssldeknsvsvdlpggmkvlvskekdkdgkyslmatveklelkgtsd knngsgtlegektdkskvkltiaedlskttfeifkedgktlvskkvtlkdkssteekfnekgeisektivra ngtrleytdiksdgsgkakevlkdftlegtlaadgkttlkvtegtvvlsknilksgeitvalddsdttqatkkt gkwdsktstltisvnsqktknlvftkedtitvqkydsagtnlegkaveittleklkdalk |
| <YP_853838 outer surface protein B,Borrelia afzelii PKo><br><br>Seq 50 | <YP_853838 outer surface protein B;Protein;Borrelia afzelii PKo><br>Mkqyllvfalvlaliacsqkgtepkstsqdhndqeiinsdntpkdskkdltvlaeensvplfngnkifvs keknsagkyelratvdtvelkgvsdknngsgklegtkadktkvamtiaddlntitvetydasnkktgs evvkkqgsvikesykankldskkitrenettleysemtdssnatkavetlkngiklegslvggkttvklt egtitltreieqdgkvkiylndttsgstkktatwnettntltisadskktkdfvfltdgtitvqaydtagtkleg nsseikdlaalkaalk |
| <NP_045689 outer surface protein B,Borrelia burgdorferi | <NP_045689 outer surface protein B;Protein;Borrelia burgdorferi B31><br>mrlligfalalaligcaqkgaesigsqkendlnledsskkshqnakqdlpavtedsvslfngnkifvsk eknssgkydlratidqvelkgtsdknngsgtlegskpdkskvkltvsadlntvtleafdasnqkisskv tkkqgsitxetlkankldskkltrsngttleysqitdadnatkavetlknsiklegslvggkttveikegtvtl kreiekdgkvkvflndtagsnkktgkwedststltisadskktkdlvfltdgtitvqqyntagtslegsas |

| B31>

Seq 51 | eiknlselknalk |
|---|---|
| AAN87995 OspC,Borrelia garinii>

Seq 52 | <AAN87995 Outer Surface Protein C;Protein;Borrelia garinii> ilmtlflfiscnnsggdtastnpdesakgpnltviskkitdsnafvlavkevealissidelankaigkvih qnnglnanagqngsllagayaistliteklsklknseelnkkieeaknhseaftnrlkgshaqlgvaaa tddhakeailksnptkdkgakelkdlsesveslakaaqealansvkeltnpvvaespk |
| <NP_047005 outer surface protein C, Borrelia burgdorferi B31>

Seq 53 | <NP_047005 outer surface protein C;Protein;Borrelia burgdorferi B31> Mkkntlsailmtlflfiscnnsgkdgntsansadesvkgpnlteiskkitdsnavllavkeveallsside iaakaigkkihqnngldtennhngsllagayaistlikqkldglkneglkekidaakkcsetftnklkek htdlgkegvtdadakeailktngtktkgaeelgklfesvevlskaakemlansvkeltspvvaespk kp |
| <CAF34024 outer surface protein VlsE,Borrelia garinii>

Seq 54 | <CAF34024 outer surface protein VlsE;Protein;Borrelia garinii> mkkissaifivaflaligcknnvggddkkdtaasifyqsiinlgngfievfnafsglvadafskadpkks dvktyfdsitktlkdtktklediskektggektpavegiaevvktvgewldglikaaegggkaadggg sdkignvaagggagadkesvngiagaikgiveaakkvegvkfapkaaadaaaadgnkkagklf gtaagadagdvkdaaaavgavsgeqilnaivtaagqagqagkkadeaknaieaaigaagdadf gddikkkndqiaaalvlrgvakdgkfaga |
| <CAF34027 outer surface protein VlsE,Borrelia afzelii PKo>

Seq 55 | <CAF34027 outer surface protein VlsE;Protein;Borrelia afzelii PKo> Mkkissaifltallvfincknnavgkgnddkdsvktfyesiinlgngfidvfnafsglvadtffksdpkks dvktyfesisstlkatkgkldelvsakkgeggsvkasvesavdevskwleemikaaeeaakvggt ggdgkigdsaanhgakadkdsvkgiakgikgivdaagkalgekgalkdvkaaaddeanadagk lfagnanaavgaaadiakaagavtavsgeqilkaiveaagdpanqagkkaeeaknpiaaaigtd ddngaafkdemkksdkiaaaivlrgvakdgkfavk |

# Fig. 39.

**<ABX71745 CRASP-2;Protein;Borrelia burgdorferi>**

8 mers:

mkksflsi;kksflsiy;ksflsiym;sflsiyml;flsiymli;lsiymlis;siymlisi;iymlisis;ymlisisl;mlisisll;lis
islls;isisllsc;sisllscd;isllscdv;sllscdvs;llscdvsr;lscdvsrl;scdvsrln;cdvsrlnq;dvsrlnqr;vsrlnqrn
;srlnqrni;rlnqrnid;lnqrnide;nqrnidel;qrnidelk;rnidelki;nidelkif;idelkifv;delkifve;elkifvek;l
kifveka;kifvekak;ifvekaky;fvekakyy;vekakyys;ekakyysi;kakyysik;akyysikl;kyysikld;yysi
klda;ysikldai;sikldaiy;ikldaiys;kldaiyse;ldaiysey;daiyseyt;aiyseytg;iyseytga;yseytgay;seyt
gayn;eytgaynd;ytgayndi;tgayndim;gayndimt;ayndimty;yndimtyi;ndimtyim;dimtyimt;imty
imty;mtyimtys;tyimtyse;yimtyseg;imtysegt;mtysegts;tysegtss;ysegtssd;segtssdk;egtssdks;
gtssdksk;tssdkskv;ssdkskvn;sdkskvnq;dkskvnqa;kskvnqai;skvnqais;kvnqaisi;vnqaisil;nqa
isilk;qaisilkk;aisilkkd;isilkkdn;silkkdnk;ilkkdnki;lkkdnkiv;kkdnkivn;kdnkivnk;dnkivnkf;
nkivnkfk;kivnkfke;ivnkfkel;vnkfkele;nkfkelek;kfkeleki;fkelekii;kelekiie;elekiiee;lekiieey
;ekiieeyk;kiieeykp;iieeykpm;ieeykpmf;eeykpmfl;eykpmfls;ykpmflsk;kpmflskl;pmflskli;
mflsklid;flsklidd;lskliddf;skliddfa;kliddfai;liddfaie;iddfaiel;ddfaield;dfaieldq;faieldqa;aiel
dqav;ieldqavd;eldqavdn;ldqavdnd;dqavdndv;qavdndvs;avdndvsn;vdndvsna;dndvsnar;ndv
snarh;dvsnarhv;vsnarhva;snarhvad;narhvads;arhvadsy;rhvadsye;hvadsyek;vadsyekl;adsye
klr;dsyeklrk;syeklrks;yeklrksv;eklrksva;klrksval;lrksvala;rksvalay;ksvalayi;svalayie;vala
yies;alayiesf;layiesfd;ayiesfdv;yiesfdvi;iesfdvis;esfdviss;sfdvissk;fdvisskf;dvisskfv;visskf
vd;isskfvds;sskfvdsk;skfvdskf;kfvdskfv;fvdskfve;vdskfvea;dskfveas;skfveask;kfveaskk;f
veaskkf;veaskkfv;easkkfvn;askkfvnk;skkfvnka;kkfvnkak;kfvnkake;fvnkakef;vnkakefv;nk
akefve;kakefvee;akefveen;kefveend;efveendl;fveendli;veendlia;eendlial;endlialk;ndlialkc
;dlialkci;lialkciv;ialkcivk;alkcivkt;lkcivkti;kcivktig;civktigd;ivktigdm;vktigdmv;ktigdmv
n;tigdmvnd;igdmvndr;gdmvndre;dmvndrei;mvndrein;vndreins;ndreinsr;dreinsrs;reinsrsr;
einsrsry;insrsryn;nsrsrynn;srsrynnf;rsrynnfy;srynnfyk;rynnfykk;ynnfykke;nnfykkea;nfyk
kead;fykkeadf;ykkeadfl;kkeadflg;keadflga;eadflgaa;adflgaav;dflgaave;flgaavel;lgaavele;g
aaveleg;aavelega;avelegay;velegayk;elegayka;legaykai;egaykaik;gaykaikq;aykaikqt;ykai
kqtl;kaikqtll;

9 mers:

mkksflsiy;kksflsiym;ksflsiyml;sflsiymli;flsiymlis;lsiymlisi;siymlisis;iymlisisl;ymlisisll;m
lisislls;lisisllsc;isisllscd;sisllscdv;isllscdvs;sllscdvsr;llscdvsrl;lscdvsrln;scdvsrlnq;cdvsrlnq
r;dvsrlnqrn;vsrlnqrni;srlnqrnid;rlnqrnide;lnqrnidel;nqrnidelk;qrnidelki;rnidelkif;nidelkifv;
idelkifve;delkifvek;elkifveka;lkifvekak;kifvekaky;ifvekakyy;fvekakyys;vekakyysi;ekaky
ysik;kakyysikl;akyysikld;kyysiklda;yysikldai;ysikldaiy;sikldaiys;ikldaiyse;kldaiysey;ldai
yseyt;daiyseytg;aiyseytga;iyseytgay;yseytgayn;seytgaynd;eytgayndi;ytgayndim;tgayndim
t;gayndimty;ayndimtyi;yndimtyim;ndimtyimt;dimtyimty;imtyimtys;mtyimtyse;tyimtyseg
;yimtysegt;imtysegts;mtysegtss;tysegtssd;ysegtssdk;segtssdks;egtssdksk;gtssdkskv;tssdks
kvn;ssdkskvnq;sdkskvnqa;dkskvnqai;kskvnqais;skvnqaisi;kvnqaisil;vnqaisilk;nqaisilkk;q
aisilkkd;aisilkkdn;isilkkdnk;silkkdnki;ilkkdnkiv;lkkdnkivn;kkdnkivnk;kdnkivnkf;dnkivn
kfk;nkivnkfke;kivnkfkel;ivnkfkele;vnkfkelek;nkfkeleki;kfkelekii;fkelekiie;kelekiiee;eleki
ieey;lekiieeyk;ekiieeykp;kiieeykpm;iieeykpmf;ieeykpmfl;eeykpmfls;eykpmflsk;ykpmflsk
l;kpmflskli;pmflsklid;mflsklidd;flskliddf;lskliddfa;skliddfai;kliddfaie;liddfaiel;iddfaield;d
dfaieldq;dfaieldqa;faieldqav;aieldqavd;ieldqavdn;eldqavdnd;ldqavdndv;dqavdndvs;qavdn

dvsn;avdndvsna;vdndvsnar;dndvsnarh;ndvsnarhv;dvsnarhva;vsnarhvad;snarhvads;narhva
dsy;arhvadsye;rhvadsyek;hvadsyekl;vadsyeklr;adsyeklrk;dsyeklrks;syeklrksv;yeklrksva;e
klrksval;klrksvala;lrksvalay;rksvalayi;ksvalayie;svalayies;valayiesf;alayiesfd;layiesfdv;ay
iesfdvi;yiesfdvis;iesfdviss;esfdvissk;sfdvisskf;fdvisskfv;dvisskfvd;visskfvds;isskfvdsk;ss
kfvdskf;skfvdskfv;kfvdskfve;fvdskfvea;vdskfveas;dskfveask;skfveaskk;kfveaskkf;fveask
kfv;veaskkfvn;easkkfvnk;askkfvnka;skkfvnkak;kkfvnkake;kfvnkakef;fvnkakefv;vnkakef
ve;nkakefvee;kakefveen;akefveend;kefveendl;efveendli;fveendlia;veendlial;eendlialk;end
lialkc;ndlialkci;dlialkciv;lialkcivk;ialkcivkt;alkcivkti;lkcivktig;kcivktigd;civktigdm;ivktig
dmv;vktigdmvn;ktigdmvnd;tigdmvndr;igdmvndre;gdmvndrei;dmvndrein;mvndreins;vndr
einsr;ndreinsrs;dreinsrsr;reinsrsry;einsrsryn;insrsrynn;nsrsrynnf;srsrynnfy;rsrynnfyk;sryn
nfykk;rynnfykke;ynnfykkea;nnfykkead;nfykkeadf;fykkeadfl;ykkeadflg;kkeadflga;keadflg
aa;eadflgaav;adflgaave;dflgaavel;flgaavele;lgaaveleg;gaavelega;aavelegay;avelegayk;vel
egayka;elegaykai;legaykaik;egaykaikq;gaykaikqt;aykaikqtl;ykaikqtll;

10 mers:
mkksflsiym;kksflsiyml;ksflsiymli;sflsiymlis;flsiymlisi;lsiymlisis;siymlisisl;iymlisisll;yml
isislls;mlisisllsc;lisisllscd;isisllscdv;sisllscdvs;isllscdvsr;sllscdvsrl;llscdvsrln;lscdvsrlnq;s
cdvsrlnqr;cdvsrlnqrn;dvsrlnqrni;vsrlnqrnid;srlnqrnide;rlnqrnidel;lnqrnidelk;nqrnidelki;qr
nidelkif;rnidelkifv;nidelkifve;idelkifvek;delkifveka;elkifvekak;lkifvekaky;kifvekakyy;ifv
ekakyys;fvekakyysi;vekakyysik;ekakyysikl;kakyysikld;akyysiklda;kyysikldai;yysikldaiy;
ysikldaiys;sikldaiyse;ikldaiysey;kldaiyseyt;ldaiyseytg;daiyseytga;aiyseytgay;iyseytgayn;y
seytgaynd;seytgayndi;eytgayndim;ytgayndimt;tgayndimty;gayndimtyi;ayndimtyim;yndi
mtyimt;ndimtyimty;dimtyimtys;imtyimtyse;mtyimtyseg;tyimtysegt;yimtysegts;imtysegts
s;mtysegtssd;tysegtssdk;ysegtssdks;segtssdksk;egtssdkskv;gtssdkskvn;tssdkskvnq;ssdksk
vnqa;sdkskvnqai;dkskvnqais;kskvnqaisi;skvnqaisil;kvnqaisilk;vnqaisilkk;nqaisilkkd;qaisi
lkkdn;aisilkkdnk;isilkkdnki;silkkdnkiv;ilkkdnkivn;lkkdnkivnk;kkdnkivnkf;kdnkivnkfk;d
nkivnkfke;nkivnkfkel;kivnkfkele;ivnkfkelek;vnkfkeleki;nkfkelekii;kfkelekiie;fkelekiiee;k
elekiieey;elekiieeyk;lekiieeykp;ekiieeykpm;kiieeykpmf;iieeykpmfl;ieeykpmfls;eeykpmfls
k;eykpmflskl;ykpmflskli;kpmflsklid;pmflsklidd;mflskliddf;flskliddfa;lskliddfai;skliddfaie
;kliddfaiel;liddfaield;iddfaieldq;ddfaieldqa;dfaieldqav;faieldqavd;aieldqavdn;ieldqavdnd;
eldqavdndv;ldqavdndvs;dqavdndvsn;qavdndvsna;avdndvsnar;vdndvsnarh;dndvsnarhv;nd
vsnarhva;dvsnarhvad;vsnarhvads;snarhvadsy;narhvadsye;arhvadsyek;rhvadsyekl;hvadsye
klr;vadsyeklrk;adsyeklrks;dsyeklrksv;syeklrksva;yeklrksval;eklrksvala;klrksvalay;lrksval
ayi;rksvalayie;ksvalayies;svalayiesf;valayiesfd;alayiesfdv;layiesfdvi;ayiesfdvis;yiesfdviss
;iesfdvissk;esfdvisskf;sfdvisskfv;fdvisskfvd;dvisskfvds;visskfvdsk;isskfvdskf;sskfvdskfv;
skfvdskfve;kfvdskfvea;fvdskfveas;vdskfveask;dskfveaskk;skfveaskkf;kfveaskkfv;fveaskk
fvn;veaskkfvnk;easkkfvnka;askkfvnkak;skkfvnkake;kkfvnkakef;kfvnkakefv;fvnkakefve;
vnkakefvee;nkakefveen;kakefveend;akefveendl;kefveendli;efveendlia;fveendlial;veendlia
lk;eendlialkc;endlialkci;ndlialkciv;dlialkcivk;lialkcivkt;ialkcivkti;alkcivktig;lkcivktigd;kc
ivktigdm;civktigdmv;ivktigdmvn;vktigdmvnd;ktigdmvndr;tigdmvndre;igdmvndrei;gdmv
ndrein;dmvndreins;mvndreinsr;vndreinsrs;ndreinsrsr;dreinsrsry;reinsrsryn;einsrsrynn;insr
srynnf;nsrsrynnfy;srsrynnfyk;rsrynnfykk;srynnfykke;rynnfykkea;ynnfykkead;nnfykkeadf
;nfykkeadfl;fykkeadflg;ykkeadflga;kkeadflgaa;keadflgaav;eadflgaave;adflgaavel;dflgaav
ele;flgaaveleg;lgaavelega;gaavelegay;aavelegayk;avelegayka;velegaykai;elegaykaik;lega
ykaikq;egaykaikqt;gaykaikqtl;aykaikqtll;

11 mers:

mkksflsiyml;kksflsiymli;ksflsiymlis;sflsiymlisi;flsiymlisis;lsiymlisisl;siymlisisll;iymlisisl
ls;ymlisisllsc;mlisisllscd;lisisllscdv;isisllscdvs;sisllscdvsr;isllscdvsrl;sllscdvsrln;llscdvsrln
q;lscdvsrlnqr;scdvsrlnqrn;cdvsrlnqrni;dvsrlnqrnid;vsrlnqrnide;srlnqrnidel;rlnqrnidelk;lnqr
nidelki;nqrnidelkif;qrnidelkifv;rnidelkifve;nidelkifvek;idelkifveka;delkifvekak;elkifvekak
y;lkifvekakyy;kifvekakyys;ifvekakyysi;fvekakyysik;vekakyysikl;ekakyysikld;kakyysikld
a;akyysikldai;kyysikldaiy;yysikldaiys;ysikldaiyse;sikldaiysey;ikldaiyseyt;kldaiyseytg;ldai
yseytga;daiyseytgay;aiyseytgayn;iyseytgaynd;yseytgayndi;seytgayndim;eytgayndimt;ytg
ayndimty;tgayndimtyi;gayndimtyim;ayndimtyimt;yndimtyimty;ndimtyimtys;dimtyimtyse
;imtyimtyseg;mtyimtysegt;tyimtysegts;yimtysegtss;imtysegtssd;mtysegtssdk;tysegtssdks;
ysegtssdksk;segtssdkskv;egtssdkskvn;gtssdkskvnq;tssdkskvnqa;ssdkskvnqai;sdkskvnqais
;dkskvnqaisi;kskvnqaisil;skvnqaisilk;kvnqaisilkk;vnqaisilkkd;nqaisilkkdn;qaisilkkdnk;ais
ilkkdnki;isilkkdnkiv;silkkdnkivn;ilkkdnkivnk;lkkdnkivnkf;kkdnkivnkfk;kdnkivnkfke;dnk
ivnkfkel;nkivnkfkele;kivnkfkelek;ivnkfkeleki;vnkfkelekii;nkfkelekiie;kfkelekiiee;fkelekii
eey;kelekiieeyk;elekiieeykp;lekiieeykpm;ekiieeykpmf;kiieeykpmfl;iieeykpmfls;ieeykpmf
lsk;eeykpmflskl;eykpmflskli;ykpmflsklid;kpmflsklidd;pmflskliddf;mflskliddfa;flskliddfai
;lskliddfaie;skliddfaiel;kliddfaield;liddfaieldq;iddfaieldqa;ddfaieldqav;dfaieldqavd;faield
qavdn;aieldqavdnd;ieldqavdndv;eldqavdndvs;ldqavdndvsn;dqavdndvsna;qavdndvsnar;av
dndvsnarh;vdndvsnarhv;dndvsnarhva;ndvsnarhvad;dvsnarhvads;vsnarhvadsy;snarhvadsy
e;narhvadsyek;arhvadsyekl;rhvadsyeklr;hvadsyeklrk;vadsyeklrks;adsyeklrksv;dsyeklrksv
a;syeklrksval;yeklrksvala;eklrksvalay;klrksvalayi;lrksvalayie;rksvalayies;ksvalayiesf;sval
ayiesfd;valayiesfdv;alayiesfdvi;layiesfdvis;ayiesfdviss;yiesfdvissk;iesfdvisskf;esfdvisskfv
;sfdvisskfvd;fdvisskfvds;dvisskfvdsk;visskfvdskf;isskfvdskfv;sskfvdskfve;skfvdskfvea;kf
vdskfveas;fvdskfveask;vdskfveaskk;dskfveaskkf;skfveaskkfv;kfveaskkfvn;fveaskkfvnk;v
easkkfvnka;easkkfvnkak;askkfvnkake;skkfvnkakef;kkfvnkakefv;kfvnkakefve;fvnkakefve
e;vnkakefveen;nkakefveend;kakefveendl;akefveendli;kefveendlia;efveendlial;fveendlialk
;veendlialkc;eendlialkci;endlialkciv;ndlialkcivk;dlialkcivkt;lialkcivkti;ialkcivktig;alkcivkt
igd;lkcivktigdm;kcivktigdmv;civktigdmvn;ivktigdmvnd;vktigdmvndr;ktigdmvndre;tigdm
vndrei;igdmvndrein;gdmvndreins;dmvndreinsr;mvndreinsrs;vndreinsrsr;ndreinsrsry;drein
srsryn;reinsrsrynn;einsrsrynnf;insrsrynnfy;nsrsrynnfyk;srsrynnfykk;rsrynnfykke;srynnfyk
kea;rynnfykkead;ynnfykkeadf;nnfykkeadfl;nfykkeadflg;fykkeadflga;ykkeadflgaa;kkeadfl
gaav;keadflgaave;eadflgaavel;adflgaavele;dflgaaveleg;flgaavelega;lgaavelegay;gaavelega
yk;aavelegayka;avelegaykai;velegaykaik;elegaykaikq;legaykaikqt;egaykaikqtl;gaykaikqtl
l;

13 mers:

mkksflsiymlis;kksflsiymlisi;ksflsiymlisis;sflsiymlisisl;flsiymlisisll;lsiymlisislls;siymlisisl
lsc;iymlisisllscd;ymlisisllscdv;mlisisllscdvs;lisisllscdvsr;isisllscdvsrl;sisllscdvsrln;isllscd
vsrlnq;sllscdvsrlnqr;llscdvsrlnqrn;lscdvsrlnqrni;scdvsrlnqrnid;cdvsrlnqrnide;dvsrlnqrnidel
;vsrlnqrnidelk;srlnqrnidelki;rlnqrnidelkif;lnqrnidelkifv;nqrnidelkifve;qrnidelkifvek;rnidel
kifveka;nidelkifvekak;idelkifvekaky;delkifvekakyy;elkifvekakyys;lkifvekakyysi;kifvekak
yysik;ifvekakyysikl;fvekakyysikld;vekakyysiklda;ekakyysikldai;kakyysikldaiy;akyysikld
aiys;kyysikldaiyse;yysikldaiysey;ysikldaiyseyt;sikldaiyseytg;ikldaiyseytga;kldaiyseytgay;
ldaiyseytgayn;daiyseytgaynd;aiyseytgayndi;iyseytgayndim;yseytgayndimt;seytgayndimty
;eytgayndimtyi;ytgayndimtyim;tgayndimtyimt;gayndimtyimty;ayndimtyimtys;yndimtyim
tyse;ndimtyimtyseg;dimtyimtysegt;imtyimtysegts;mtyimtysegtss;tyimtysegtssd;yimtysegt

ssdk;imtysegtssdks;mtysegtssdksk;tysegtssdkskv;ysegtssdkskvn;segtssdkskvnq;egtssdksk
vnqa;gtssdkskvnqai;tssdkskvnqais;ssdkskvnqaisi;sdkskvnqaisil;dkskvnqaisilk;kskvnqaisil
kk;skvnqaisilkkd;kvnqaisilkkdn;vnqaisilkkdnk;nqaisilkkdnki;qaisilkkdnkiv;aisilkkdnkivn
;isilkkdnkivnk;silkkdnkivnkf;ilkkdnkivnkfk;lkkdnkivnkfke;kkdnkivnkfkel;kdnkivnkfkele
;dnkivnkfkelek;nkivnkfkeleki;kivnkfkelekii;ivnkfkelekiie;vnkfkelekiiee;nkfkelekiieey;kf
kelekiieeyk;fkelekiieeykp;kelekiieeykpm;elekiieeykpmf;lekiieeykpmfl;ekiieeykpmfls;kii
eeykpmflsk;iieeykpmflskl;ieeykpmflskli;eeykpmflsklid;eykpmflsklidd;ykpmflskliddf;kp
mflskliddfa;pmflskliddfai;mflskliddfaie;flskliddfaiel;lskliddfaield;skliddfaieldq;kliddfaiel
dqa;liddfaieldqav;iddfaieldqavd;ddfaieldqavdn;dfaieldqavdnd;faieldqavdndv;aieldqavdnd
vs;ieldqavdndvsn;eldqavdndvsna;ldqavdndvsnar;dqavdndvsnarh;qavdndvsnarhv;avdndvs
narhva;vdndvsnarhvad;dndvsnarhvads;ndvsnarhvadsy;dvsnarhvadsye;vsnarhvadsyek;sna
rhvadsyekl;narhvadsyeklr;arhvadsyeklrk;rhvadsyeklrks;hvadsyeklrksv;vadsyeklrksva;ads
yeklrksval;dsyeklrksvala;syeklrksvalay;yeklrksvalayi;eklrksvalayie;klrksvalayies;lrksvala
yiesf;rksvalayiesfd;ksvalayiesfdv;svalayiesfdvi;valayiesfdvis;alayiesfdviss;layiesfdvissk;
ayiesfdvisskf;yiesfdvisskfv;iesfdvisskfvd;esfdvisskfvds;sfdvisskfvdsk;fdvisskfvdskf;dvis
skfvdskfv;visskfvdskfve;isskfvdskfvea;sskfvdskfveas;skfvdskfveask;kfvdskfveaskk;fvds
kfveaskkf;vdskfveaskkfv;dskfveaskkfvn;skfveaskkfvnk;kfveaskkfvnka;fveaskkfvnkak;ve
askkfvnkake;easkkfvnkakef;askkfvnkakefv;skkfvnkakefve;kkfvnkakefvee;kfvnkakefveen
;fvnkakefveend;vnkakefveendl;nkakefveendli;kakefveendlia;akefveendlial;kefveendlialk;
efveendlialkc;fveendlialkci;veendlialkciv;eendlialkcivk;endlialkcivkt;ndlialkcivkti;dlialk
civktig;lialkcivktigd;ialkcivktigdm;alkcivktigdmv;lkcivktigdmvn;kcivktigdmvnd;civktigd
mvndr;ivktigdmvndre;vktigdmvndrei;ktigdmvndrein;tigdmvndreins;igdmvndreinsr;gdmv
ndreinsrs;dmvndreinsrsr;mvndreinsrsry;vndreinsrsryn;ndreinsrsrynn;dreinsrsrynnf;reinsrs
rynnfy;einsrsrynnfyk;insrsrynnfykk;nsrsrynnfykke;srsrynnfykkea;rsrynnfykkead;srynnfy
kkeadf;rynnfykkeadfl;ynnfykkeadflg;nnfykkeadflga;nfykkeadflgaa;fykkeadflgaav;ykkead
flgaave;kkeadflgaavel;keadflgaavele;eadflgaaveleg;adflgaavelega;dflgaavelegay;flgaavel
egayk;lgaavelegayka;gaavelegaykai;aavelegaykaik;avelegaykaikq;velegaykaikqt;elegayk
aikqtl;legaykaikqtll;

14 mers:

mkksflsiymlisi;kksflsiymlisis;ksflsiymlisisl;sflsiymlisisll;flsiymlisislls;lsiymlisisllsc;siym
lisisllscd;iymlisisllscdv;ymlisisllscdvs;mlisisllscdvsr;lisisllscdvsrl;isisllscdvsrln;sisllscdvs
rlnq;isllscdvsrlnqr;sllscdvsrlnqrn;llscdvsrlnqrni;lscdvsrlnqrnid;scdvsrlnqrnide;cdvsrlnqrni
del;dvsrlnqrnidelk;vsrlnqrnidelki;srlnqrnidelkif;rlnqrnidelkifv;lnqrnidelkifve;nqrnidelkifv
ek;qrnidelkifveka;rnidelkifvekak;nidelkifvekaky;idelkifvekakyy;delkifvekakyys;elkifvek
akyysi;lkifvekakyysik;kifvekakyysikl;ifvekakyysikld;fvekakyysiklda;vekakyysikldai;eka
kyysikldaiy;kakyysikldaiys;akyysikldaiyse;kyysikldaiysey;yysikldaiyseyt;ysikldaiyseytg;
sikldaiyseytga;ikldaiyseytgay;kldaiyseytgayn;ldaiyseytgaynd;daiyseytgayndi;aiyseytgayn
dim;iyseytgayndimt;yseytgayndimty;seytgayndimtyi;eytgayndimtyim;ytgayndimtyimt;tg
ayndimtyimty;gayndimtyimtys;ayndimtyimtyse;yndimtyimtyseg;ndimtyimtysegt;dimtyi
mtysegts;imtyimtysegtss;mtyimtysegtssd;tyimtysegtssdk;yimtysegtssdks;imtysegtssdksk;
mtysegtssdksv;tysegtssdkskvn;ysegtssdkskvnq;segtssdkskvnqa;egtssdkskvnqai;gtssdksk
vnqais;tssdkskvnqaisi;ssdkskvnqaisil;sdkskvnqaisilk;dkskvnqaisilkk;kskvnqaisilkkd;skvn
qaisilkkdn;kvnqaisilkkdnk;vnqaisilkkdnki;nqaisilkkdnkiv;qaisilkkdnkivn;aisilkkdnkivnk;
isilkkdnkivnkf;silkkdnkivnkfk;ilkkdnkivnkfke;lkkdnkivnkfkel;kkdnkivnkfkele;kdnkivnkf
kelek;dnkivnkfkeleki;nkivnkfkelekii;kivnkfkelekiie;ivnkfkelekiiee;vnkfkelekiieey;nkfkel

ekiieeyk;kfkelekiieeykp;fkelekiieeykpm;kelekiieeykpmf;elekiieeykpmfl;lekiieeykpmfls;e
kiieeykpmflsk;kiieeykpmflskl;iieeykpmflskli;ieeykpmflsklid;eeykpmflsklidd;eykpmflskli
ddf;ykpmflskliddfa;kpmflskliddfai;pmflskliddfaie;mflskliddfaiel;flskliddfaield;lskliddfaie
ldq;skliddfaieldqa;kliddfaieldqav;liddfaieldqavd;iddfaieldqavdn;ddfaieldqavdnd;dfaieldq
avdndv;faieldqavdndvs;aieldqavdndvsn;ieldqavdndvsna;eldqavdndvsnar;ldqavdndvsnarh;
dqavdndvsnarhv;qavdndvsnarhva;avdndvsnarhvad;vdndvsnarhvads;dndvsnarhvadsy;ndvs
narhvadsye;dvsnarhvadsyek;vsnarhvadsyekl;snarhvadsyeklr;narhvadsyeklrk;arhvadsyeklr
ks;rhvadsyeklrksv;hvadsyeklrksva;vadsyeklrksval;adsyeklrksvala;dsyeklrksvalay;syeklrk
svalayi;yeklrksvalayie;eklrksvalayies;klrksvalayiesf;lrksvalayiesfd;rksvalayiesfdv;ksvala
yiesfdvi;svalayiesfdvis;valayiesfdviss;alayiesfdvissk;layiesfdvisskf;ayiesfdvisskfv;yiesfd
visskfvd;iesfdvisskfvds;esfdvisskfvdsk;sfdvisskfvdskf;fdvisskfvdskfv;dvisskfvdskfve;vis
skfvdskfvea;isskfvdskfveas;sskfvdskfveask;skfvdskfveaskk;kfvdskfveaskkf;fvdskfveaskk
fv;vdskfveaskkfvn;dskfveaskkfvnk;skfveaskkfvnka;kfveaskkfvnkak;fveaskkfvnkake;veas
kkfvnkakef;easkkfvnkakefv;askkfvnkakefve;skkfvnkakefvee;kkfvnkakefveen;kfvnkakefv
eend;fvnkakefveendl;vnkakefveendli;nkakefveendlia;kakefveendlial;akefveendlialk;kefv
eendlialkc;efveendlialkci;fveendlialkciv;veendlialkcivk;eendlialkcivkt;endlialkcivkti;ndli
alkcivktig;dlialkcivktigd;lialkcivktigdm;ialkcivktigdmv;alkcivktigdmvn;lkcivktigdmvnd;
kcivktigdmvndr;civktigdmvndre;ivktigdmvndrei;vktigdmvndrein;ktigdmvndreins;tigdmv
ndreinsr;igdmvndreinsrs;gdmvndreinsrsr;dmvndreinsrsry;mvndreinsrsryn;vndreinsrsrynn;
ndreinsrsrynnf;dreinsrsrynnfy;reinsrsrynnfyk;einsrsrynnfykk;insrsrynnfykke;nsrsrynnfyk
kea;srsrynnfykkead;rsrynnfykkeadf;srynnfykkeadfl;rynnfykkeadflg;ynnfykkeadflga;nnfy
kkeadflgaa;nfykkeadflgaav;fykkeadflgaave;ykkeadflgaavel;kkeadflgaavele;keadflgaavele
g;eadflgaavelega;adflgaavelegay;dflgaavelegayk;flgaavelegayka;lgaavelegaykai;gaaveleg
aykaik;aavelegaykaikq;avelegaykaikqt;velegaykaikqtl;elegaykaikqtll;

15 mers:
mkksflsiymlisis;kksflsiymlisisl;ksflsiymlisisll;sflsiymlisislls;flsiymlisisllsc;lsiymlisisllscd
;siymlisisllscdv;iymlisisllscdvs;ymlisisllscdvsr;mlisisllscdvsrl;lisisllscdvsrln;isisllscdvsrl
nq;sisllscdvsrlnqr;isllscdvsrlnqrn;sllscdvsrlnqrni;llscdvsrlnqrnid;lscdvsrlnqrnide;scdvsrln
qrnidel;cdvsrlnqrnidelk;dvsrlnqrnidelki;vsrlnqrnidelkif;srlnqrnidelkifv;rlnqrnidelkifve;ln
qrnidelkifvek;nqrnidelkifveka;qrnidelkifvekak;rnidelkifvekaky;nidelkifvekakyy;idelkifve
kakyys;delkifvekakyysi;elkifvekakyysik;lkifvekakyysikl;kifvekakyysikld;ifvekakyysikld
a;fvekakyysikldai;vekakyysikldaiy;ekakyysikldaiys;kakyysikldaiyse;akyysikldaiysey;kyy
sikldaiyseyt;yysikldaiyseytg;ysikldaiyseytga;sikldaiyseytgay;ikldaiyseytgayn;kldaiyseytg
aynd;ldaiyseytgayndi;daiyseytgayndim;aiyseytgayndimt;iyseytgayndimty;yseytgayndimt
yi;seytgayndimtyim;eytgayndimtyimt;ytgayndimtyimty;tgayndimtyimtys;gayndimtyimty
se;ayndimtyimtyseg;yndimtyimtysegt;ndimtyimtysegts;dimtyimtysegtss;imtyimtysegtssd
;mtyimtysegtssdk;tyimtysegtssdks;yimtysegtssdksk;imtysegtssdkskv;mtysegtssdkskvn;ty
segtssdkskvnq;ysegtssdkskvnqa;segtssdkskvnqai;egtssdkskvnqais;gtssdkskvnqaisi;tssdks
kvnqaisil;ssdkskvnqaisilk;sdkskvnqaisilkk;dkskvnqaisilkkd;kskvnqaisilkkdn;skvnqaisilk
kdnk;kvnqaisilkkdnki;vnqaisilkkdnkiv;nqaisilkkdnkivn;qaisilkkdnkivnk;aisilkkdnkivnkf;i
silkkdnkivnkfk;silkkdnkivnkfke;ilkkdnkivnkfkel;lkkdnkivnkfkele;kkdnkivnkfkelek;kdnki
vnkfkeleki;dnkivnkfkelekii;nkivnkfkelekiie;kivnkfkelekiiee;ivnkfkelekiieey;vnkfkelekiie
eyk;nkfkelekiieeykp;kfkelekiieeykpm;fkelekiieeykpmf;kelekiieeykpmfl;elekiieeykpmfls;l
ekiieeykpmflsk;ekiieeykpmflskl;kiieeykpmflskli;iieeykpmflsklid;ieeykpmflsklidd;eeykp
mflskliddf;eykpmflskliddfa;ykpmflskliddfai;kpmflskliddfaie;pmflskliddfaiel;mflskliddfai

eld;flskliddfaieldq;lskliddfaieldqa;skliddfaieldqav;kliddfaieldqavd;liddfaieldqavdn;iddfai
eldqavdnd;ddfaieldqavdndv;dfaieldqavdndvs;faieldqavdndvsn;aieldqavdndvsna;ieldqavd
ndvsnar;eldqavdndvsnarh;ldqavdndvsnarhv;dqavdndvsnarhva;qavdndvsnarhvad;avdndvs
narhvads;vdndvsnarhvadsy;dndvsnarhvadsye;ndvsnarhvadsyek;dvsnarhvadsyekl;vsnarhv
adsyeklr;snarhvadsyeklrk;narhvadsyeklrks;arhvadsyeklrksv;rhvadsyeklrksva;hvadsyeklrk
sval;vadsyeklrksvala;adsyeklrksvalay;dsyeklrksvalayi;syeklrksvalayie;yeklrksvalayies;ek
lrksvalayiesf;klrksvalayiesfd;lrksvalayiesfdv;rksvalayiesfdvi;ksvalayiesfdvis;svalayiesfdv
iss;valayiesfdvissk;alayiesfdvisskf;layiesfdvisskfv;ayiesfdvisskfvd;yiesfdvisskfvds;iesfdv
isskfvdsk;esfdvisskfvdskf;sfdvisskfvdskfv;fdvisskfvdskfve;dvisskfvdskfvea;visskfvdskfv
eas;isskfvdskfveask;sskfvdskfveaskk;skfvdskfveaskkf;kfvdskfveaskkfv;fvdskfveaskkfvn;
vdskfveaskkfvnk;dskfveaskkfvnka;skfveaskkfvnkak;kfveaskkfvnkake;fveaskkfvnkakef;v
easkkfvnkakefv;easkkfvnkakefve;askkfvnkakefvee;skkfvnkakefveen;kkfvnkakefveend;kf
vnkakefveendl;fvnkakefveendli;vnkakefveendlia;nkakefveendlial;kakefveendlialk;akefve
endlialkc;kefveendlialkci;efveendlialkciv;fveendlialkcivk;veendlialkcivkt;eendlialkcivkti;
endlialkcivktig;ndlialkcivktigd;dlialkcivktigdm;lialkcivktigdmv;ialkcivktigdmvn;alkcivkt
igdmvnd;lkcivktigdmvndr;kcivktigdmvndre;civktigdmvndrei;ivktigdmvndrein;vktigdmv
ndreins;ktigdmvndreinsr;tigdmvndreinsrs;igdmvndreinsrsr;gdmvndreinsrsry;dmvndreinsr
sryn;mvndreinsrsrynn;vndreinsrsrynnf;ndreinsrsrynnfy;dreinsrsrynnfyk;reinsrsrynnfykk;e
insrsrynnfykke;insrsrynnfykkea;nsrsrynnfykkead;srsrynnfykkeadf;rsrynnfykkeadfl;srynnf
ykkeadflg;rynnfykkeadflga;ynnfykkeadflgaa;nnfykkeadflgaav;nfykkeadflgaave;fykkeadfl
gaavel;ykkeadflgaavele;kkeadflgaaveleg;keadflgaavelega;eadflgaavelegay;adflgaavelega
yk;dflgaavelegayka;flgaavelegaykai;lgaavelegaykaik;gaavelegaykaikq;aavelegaykaikqt;a
velegaykaikqtl;velegaykaikqtll;

16 mers:

mkksflsiymlisisl;kksflsiymlisisll;ksflsiymlisislls;sflsiymlisisllsc;flsiymlisisllscd;lsiymlisis
llscdv;siymlisisllscdvs;iymlisisllscdvsr;ymlisisllscdvsrl;mlisisllscdvsrln;lisisllscdvsrlnq;is
isllscdvsrlnqr;sisllscdvsrlnqrn;isllscdvsrlnqrni;sllscdvsrlnqrnid;llscdvsrlnqrnide;lscdvsrln
qrnidel;scdvsrlnqrnidelk;cdvsrlnqrnidelki;dvsrlnqrnidelkif;vsrlnqrnidelkifv;srlnqrnidelkif
ve;rlnqrnidelkifvek;lnqrnidelkifveka;nqrnidelkifvekak;qrnidelkifvekaky;rnidelkifvekakyy
;nidelkifvekakyys;idelkifvekakyysi;delkifvekakyysik;elkifvekakyysikl;lkifvekakyysikld;k
ifvekakyysiklda;ifvekakyysikldai;fvekakyysikldaiy;vekakyysikldaiys;ekakyysikldaiyse;k
akyysikldaiysey;akyysikldaiyseyt;kyysikldaiyseytg;yysikldaiyseytga;ysikldaiyseytgay;sik
ldaiyseytgayn;ikldaiyseytgaynd;kldaiyseytgayndi;ldaiyseytgayndim;daiyseytgayndimt;ai
yseytgayndimty;iyseytgayndimtyi;yseytgayndimtyim;seytgayndimtyimt;eytgayndimtyimt
y;ytgayndimtyimtys;tgayndimtyimtyse;gayndimtyimtyseg;ayndimtyimtysegt;yndimtyimt
ysegts;ndimtyimtysegtss;dimtyimtysegtssd;imtyimtysegtssdk;mtyimtysegtssdks;tyimtyse
gtssdksk;yimtysegtssdkskv;imtysegtssdkskvn;mtysegtssdkskvnq;tysegtssdkskvnqa;ysegts
sdkskvnqai;segtssdkskvnqais;egtssdkskvnqaisi;gtssdkskvnqaisil;tssdkskvnqaisilk;ssdkskv
nqaisilkk;sdkskvnqaisilkkd;dkskvnqaisilkkdn;kskvnqaisilkkdnk;skvnqaisilkkdnki;kvnqai
silkkdnkiv;vnqaisilkkdnkivn;nqaisilkkdnkivnk;qaisilkkdnkivnkf;aisilkkdnkivnkfk;isilkkd
nkivnkfke;silkkdnkivnkfkel;ilkkdnkivnkfkele;lkkdnkivnkfkelek;kkdnkivnkfkeleki;kdnkiv
nkfkelekii;dnkivnkfkelekiie;nkivnkfkelekiiee;kivnkfkelekiieey;ivnkfkelekiieeyk;vnkfkele
kiieeykp;nkfkelekiieeykpm;kfkelekiieeykpmf;fkelekiieeykpmfl;kelekiieeykpmfls;elekiiee
ykpmflsk;lekiieeykpmflskl;ekiieeykpmflskli;kiieeykpmflsklid;iieeykpmflsklidd;ieeykpmf
lskliddf;eeykpmflskliddfa;eykpmflskliddfai;ykpmflskliddfaie;kpmflskliddfaiel;pmflsklid

dfaield;mflskliddfaieldq;flskliddfaieldqa;lskliddfaieldqav;skliddfaieldqavd;kliddfaieldqav
dn;liddfaieldqavdnd;iddfaieldqavdndv;ddfaieldqavdndvs;dfaieldqavdndvsn;faieldqavdnd
vsna;aieldqavdndvsnar;ieldqavdndvsnarh;eldqavdndvsnarhv;ldqavdndvsnarhva;dqavdndv
snarhvad;qavdndvsnarhvads;avdndvsnarhvadsy;vdndvsnarhvadsye;dndvsnarhvadsyek;nd
vsnarhvadsyekl;dvsnarhvadsyeklr;vsnarhvadsyeklrk;snarhvadsyeklrks;narhvadsyeklrksv;
arhvadsyeklrksva;rhvadsyeklrksval;hvadsyeklrksvala;vadsyeklrksvalay;adsyeklrksvalayi;
dsyeklrksvalayie;syeklrksvalayies;yeklrksvalayiesf;eklrksvalayiesfd;klrksvalayiesfdv;lrks
valayiesfdvi;rksvalayiesfdvis;ksvalayiesfdviss;svalayiesfdvissk;valayiesfdvisskf;alayiesfd
visskfv;layiesfdvisskfvd;ayiesfdvisskfvds;yiesfdvisskfvdsk;iesfdvisskfvdskf;esfdvisskfvd
skfv;sfdvisskfvdskfve;fdvisskfvdskfvea;dvisskfvdskfveas;visskfvdskfveask;isskfvdskfvea
skk;sskfvdskfveaskkf;skfvdskfveaskkfv;kfvdskfveaskkfvn;fvdskfveaskkfvnk;vdskfveask
kfvnka;dskfveaskkfvnkak;skfveaskkfvnkake;kfveaskkfvnkakef;fveaskkfvnkakefv;veaskk
fvnkakefve;easkkfvnkakefvee;askkfvnkakefveen;skkfvnkakefveend;kkfvnkakefveendl;kf
vnkakefveendli;fvnkakefveendlia;vnkakefveendlial;nkakefveendlialk;kakefveendlialkc;a
kefveendlialkci;kefveendlialkciv;efveendlialkcivk;fveendlialkcivkt;veendlialkcivkti;eendl
ialkcivktig;endlialkcivktigd;ndlialkcivktigdm;dlialkcivktigdmv;lialkcivktigdmvn;ialkcivk
tigdmvnd;alkcivktigdmvndr;lkcivktigdmvndre;kcivktigdmvndrei;civktigdmvndrein;ivktig
dmvndreins;vktigdmvndreinsr;ktigdmvndreinsrs;tigdmvndreinsrsr;igdmvndreinsrsry;gdm
vndreinsrsryn;dmvndreinsrsrynn;mvndreinsrsrynnf;vndreinsrsrynnfy;ndreinsrsrynnfyk;dr
einsrsrynnfykk;reinsrsrynnfykke;einsrsrynnfykkea;insrsrynnfykkead;nsrsrynnfykkeadf;sr
srynnfykkeadfl;rsrynnfykkeadflg;srynnfykkeadflga;rynnfykkeadflgaa;ynnfykkeadflgaav;
nnfykkeadflgaave;nfykkeadflgaavel;fykkeadflgaavele;ykkeadflgaaveleg;kkeadflgaaveleg
a;keadflgaavelegay;eadflgaavelegayk;adflgaavelegayka;dflgaavelegaykai;flgaavelegaykai
k;lgaavelegaykaikq;gaavelegaykaikqt;aavelegaykaikqtl;avelegaykaikqtll;

**<CAH10086 CRASP-1;Protein;Borrelia garinii>**

8 mers:
mkktklni;kktklnii;ktklniik;tklniikl;klniikln;lniiklni;niiklnil;iiklnilt;iklniltt;klniltti;lnilttil;ni
lttilt;ilttiltl;lttiltli;ttiltlic;tiltlici;iltlicis;ltlicisc;tlicisca;liciscav;iciscavd;ciscavdk;iscavdki;s
cavdkid;cavdkidp;avdkidpe;vdkidpes;dkidpesk;kidpesks;idpesksk;dpeskskt;pesksktn;esks
ktnt;sksktntk;ksktntkd;sktntkdf;ktntkdfe;tntkdfen;ntkdfenk;tkdfenks;kdfenksq;dfenksqd;f
enksqdl;enksqdle;nksqdlep;ksqdleps;sqdlepsn;qdlepsnt;dlepsntk;lepsntkn;epsntknk;psntk
nkd;sntknkdl;ntknkdle;tknkdlep;knkdlepl;nkdleple;kdleplek;dleplekn;leplekns;epleknsk;p
leknske;leknsket;eknsketi;knsketii;nsketiis;sketiisk;ketiiskl;etiiskle;tiisklek;iisklekm;iskle
kmi;sklekmik;klekmikd;lekmikdl;ekmikdle;kmikdled;mikdledq;ikdledqk;kdledqkd;dledq
kdq;ledqkdqe;edqkdqed;dqkdqedt;qkdqedte;kdqedtei;dqedteia;qedteiak;edteiaki;dteiakit;t
eiakitn;eiakitnt;iakitntq;akitntqi;kitntqid;itntqidf;tntqidfl;ntqidfle;tqidflen;qidflenf;idflenf
k;dflenfks;flenfkse;lenfksep;enfkseph;nfksephd;fksephdt;ksephdti;sephdtis;ephdtise;phdti
sed;hdtisedi;dtisedie;tisediem;isediemk;sediemki;ediemkik;diemkikr;iemkikri;emkikrii;m
kikriiy;kikriiys;ikriiyss;kriiyssl;riiyssln;iiysslny;iysslnye;ysslnyei;sslnyeie;slnyeiek;lnyeie
ki;nyeiekin;yeiekint;eiekintl;iekintlk;ekintlke;kintlkei;intlkeil;ntlkeild;tlkeildk;lkeildkl;ke
ildkly;eildklyk;ildklykn;ldklyknh;dklyknhk;klyknhky;lyknhkyk;yknhkykt;knhkykt;nhky
ktta;hkykttar;kykttarn;ykttarnf;kttarnfi;ttarnfil;tarnfiln;arnfilni;rnfilnis;nfilnisi;filnisik;ilni
siki;lnisikiq;nisikiqf;isikiqfq;sikiqfqi;ikiqfqie;kiqfqien;iqfqiena;qfqienal;fqienale;qienalel
;ienalelm;enalelmk;nalelmke;alelmkee;lelmkeei;elmkeeie;lmkeeied;mkeeieda;keeiedas;e

eiedase;eiedasei;iedaseil;edaseiln;daseilnq;aseilnqe;seilnqer;eilnqery;ilnqerye;lnqeryei;nq
eryeil;qeryeill;eryeillk;ryeillkh;yeillkhv;eillkhve;illkhvep;llkhveps;lkhvepsl;khvepsln;hve
pslnl;vepslnlk;epslnlkq;pslnlkqk;slnlkqkf;lnlkqkfe;nlkqkfek;lkqkfeki;kqkfekil;qkfekiln;kf
ekilne;fekilnet;ekilneti;kilnetik;ilnetika;lnetikay;netikayn;etikaynq;tikaynqd;ikaynqdl;kay
nqdld;aynqdldn;ynqdldni;nqdldnik;qdldniks;dldniksn;ldniksne;dniksned;niksnedq;iksned
ql;ksnedqla;snedqlah;nedqlahm;edqlahmd;dqlahmde;qlahmden;lahmdeny;ahmdenyk;hm
denyke;mdenykef;denykefd;enykefdp;nykefdpl;ykefdpln;kefdplnl;efdplnld;fdplnldy;

9 mers:
mkktklnii;kktklniik;ktklniikl;tklniikln;klniiklni;lniiklnil;niiklnilt;iiklniltt;iklniltti;klnilttil;l
nilttilt;nilttiltl;ilttiltli;lttiltlic;ttiltlici;tiltlicis;iltlicisc;ltliciscá;tliciscav;liciscavd;iciscavdk;c
iscavdki;iscavdkid;scavdkidp;cavdkidpe;avdkidpes;vdkidpesk;dkidpesks;kidpesksk;idpes
kskt;dpesksktn;pesksktnt;esksktntk;sksktntkd;ksktntkdf;sktntkdfe;ktntkdfen;tntkdfenk;ntk
dfenks;tkdfenksq;kdfenksqd;dfenksqdl;fenksqdle;enksqdlep;nksqdleps;ksqdlepsn;sqdleps
nt;qdlepsntk;dlepsntkn;lepsntknk;epsntknkd;psntknkdl;sntknkdle;ntknkdlep;tknkdlepl;kn
kdleple;nkdleplek;kdleplekn;dleplekns;lepleknsk;epleknske;pleknsket;leknsketi;eknsketii
;knsketiis;nsketiisk;sketiiskl;ketiiskle;etiisklek;tiisklekm;iisklekmi;isklekmik;sklekmikd;
klekmikdl;lekmikdle;ekmikdled;kmikdledq;mikdledqk;ikdledqkd;kdledqkdq;dledqkdqe;l
edqkdqed;edqkdqedt;dqkdqedte;qkdqedtei;kdqedteia;dqedteiak;qedteiaki;edteiakit;dteiaki
tn;teiakitnt;eiakitntq;iakitntqi;akitntqid;kitntqidf;itntqidfl;tntqidfle;ntqidflen;tqidflenf;qidf
lenfk;idflenfks;dflenfkse;flenfksep;lenfkseph;enfksephd;nfksephdt;fksephdti;ksephdtis;se
phdtise;ephdtised;phdtisedi;hdtisedie;dtisediem;tisediemk;isediemki;sediemkik;ediemkik
r;diemkikri;iemkikrii;emkikriiy;mkikriiys;kikriiyss;ikriiyssl;kriiyssln;riiysslny;iiysslnye;i
ysslnyei;ysslnyeie;sslnyeiek;slnyeieki;lnyeiekin;nyeiekint;yeiekintl;eiekintlk;iekintlke;eki
ntlkei;kintlkeil;intlkeild;ntlkeildk;tlkeildkl;lkeildkly;keildklyk;eildklykn;ildklyknh;ldklyk
nhk;dklyknhky;klyknhkyk;lyknhkykt;yknhkyktt;knhkyktta;nhkykttar;hkykttarn;kykttarnf;
ykttarnfi;kttarnfil;ttarnfiln;tarnfilni;arnfilnis;rnfilnisi;nfilnisik;filnisiki;ilnisikiq;lnisikiqf;n
isikiqfq;isikiqfqi;sikiqfqie;ikiqfqien;kiqfqiena;iqfqienal;qfqienale;fqienalel;qienalelm;ien
alelmk;enalelmke;nalelmkee;alelmkeei;lelmkeeie;elmkeeied;lmkeeieda;mkeeiedas;keeied
ase;eeiedasei;eiedaseil;iedaseiln;edaseilnq;daseilnqe;aseilnqer;seilnqery;eilnqerye;ilnqery
ei;lnqeryeil;nqeryeill;qeryeillk;eryeillkh;ryeillkhv;yeillkhve;eillkhvep;illkhveps;llkhvepsl
;lkhvepsln;khvepslnl;hvepslnlk;vepslnlkq;epslnlkqk;pslnlkqkf;slnlkqkfe;lnlkqkfek;nlkqkf
eki;lkqkfekil;kqkfekiln;qkfekilne;kfekilnet;fekilneti;ekilnetik;kilnetika;ilnetikay;lnetikay
n;netikaynq;etikaynqd;tikaynqdl;ikaynqdld;kaynqdldn;aynqdldni;ynqdldnik;nqdldniks;qd
ldniksn;dldniksne;ldniksned;dniksnedq;niksnedql;iksnedqla;ksnedqlah;snedqlahm;nedqla
hmd;edqlahmde;dqlahmden;qlahmdeny;lahmdenyk;ahmdenyke;hmdenykef;mdenykefd;d
enykefdp;enykefdpl;nykefdpln;ykefdplnl;kefdplnld;efdplnldy;

10 mers:
mkktklniik;kktklniikl;ktklniikln;tklniiklni;klniiklnil;lniiklnilt;niiklniltt;iiklniltti;iklnilttil;k
lnilttilt;lnilttiltl;nilttiltli;ilttiltlic;lttiltlici;ttiltlicis;tiltlicisc;iltlicisca;ltliciscav;tliciscavd;lici
scavdk;iciscavdki;ciscavdkid;iscavdkidp;scavdkidpe;cavdkidpes;avdkidpesk;vdkidpesk;
dkidpesksk;kidpesksкt;idpesksktn;dpesksktnt;pesksktntk;esksktntkd;sksktntkdf;ksktntkdf
e;sktntkdfen;ktntkdfenk;tntkdfenks;ntkdfenksq;tkdfenksqd;kdfenksqdl;dfenksqdle;fenksq
dlep;enksqdleps;nksqdlepsn;ksqdlepsnt;sqdlepsntk;qdlepsntkn;dlepsntknk;lepsntknkd;eps
ntknkdl;psntknkdle;sntknkdlep;ntknkdlepl;tknkdleple;knkdleplek;nkdleplekn;kdleplekns;

dlepleknsk;lepleknske;epleknsket;pleknsketi;leknsketii;eknsketiis;knsketiisk;nsketiiskl;sk
etiiskle;ketiisklek;etiisklekm;tiisklekmi;iisklekmik;isklekmikd;sklekmikdl;klekmikdle;lek
mikdled;ekmikdledq;kmikdledqk;mikdledqkd;ikdledqkdq;kdledqkdqe;dledqkdqed;ledqk
dqedt;edqkdqedte;dqkdqedtei;qkdqedteia;kdqedteiak;dqedteiaki;qedteiakit;edteiakitn;dtei
akitnt;teiakitntq;eiakitntqi;iakitntqid;akitntqidf;kitntqidfl;itntqidfle;tntqidflen;ntqidflenf;t
qidflenfk;qidflenfks;idflenfkse;dflenfksep;flenfkseph;lenfksephd;enfksephdt;nfksephdti;f
ksephdtis;ksephdtise;sephdtised;ephdtisedi;phdtisedie;hdtisediem;dtisediemk;tisediemki;i
sediemkik;sediemkikr;ediemkikri;diemkikrii;iemkikriiy;emkikriiys;mkikriiyss;kikriiyssl;i
kriiyssln;kriiysslny;riiysslnye;iiysslnyei;iysslnyeie;ysslnyeiek;sslnyeieki;slnyeiekin;lnyei
ekint;nyeiekintl;yeiekintlk;eiekintlke;iekintlkei;ekintlkeil;kintlkeild;intlkeildk;ntlkeildkl;t
lkeildkly;lkeildklyk;keildklykn;eildklyknh;ildklyknhk;ldklyknhky;dklyknhkyk;klyknhky
kt;lyknhkyktt;yknhkyktta;knhkykttar;nhkykttarn;hkykttarnf;kykttarnfi;ykttarnfil;kttarnfil
n;ttarnfilni;tarnfilnis;arnfilnisi;rnfilnisik;nfilnisiki;filnisikiq;ilnisikiqf;lnisikiqfq;nisikiqfqi
;isikiqfqie;sikiqfqien;ikiqfqiena;kiqfqienal;iqfqienale;qfqienalel;fqienalelm;qienalelmk;ie
nalelmke;enalelmkee;nalelmkeei;alelmkeeie;lelmkeeied;elmkeeieda;lmkeeiedas;mkeeied
ase;keeiedasei;eeiedaseil;eiedaseiln;iedaseilnq;edaseilnqe;daseilnqer;aseilnqery;seilnqery
e;eilnqeryei;ilnqeryeil;lnqeryeill;nqeryeillk;qeryeillkh;eryeillkhv;ryeillkhve;yeillkhvep;ei
llkhveps;illkhvepsl;llkhvepsln;lkhvepslnl;khvepslnlk;hvepslnlkq;vepslnlkqk;epslnlkqkf;p
slnlkqkfe;slnlkqkfek;lnlkqkfeki;nlkqkfekil;lkqkfekiln;kqkfekilne;qkfekilnet;kfekilneti;fek
ilnetik;ekilnetika;kilnetikay;ilnetikayn;lnetikaynq;netikaynqd;etikaynqdl;tikaynqdld;ikay
nqdldn;kaynqdldni;aynqdldnik;ynqdldniks;nqdldniksn;qdldniksne;dldniksned;ldniksnedq
;dniksnedql;niksnedqla;iksnedqlah;ksnedqlahm;snedqlahmd;nedqlahmde;edqlahmden;dql
ahmdeny;qlahmdenyk;lahmdenyke;ahmdenykef;hmdenykefd;mdenykefdp;denykefdpl;en
ykefdpln;nykefdplnl;ykefdplnld;kefdplnldy;

11 mers:
mkktklniikl;kktklniikln;ktklniiklni;tklniiklnil;klniiklnilt;lniiklniltt;niiklniltti;iiklnilttil;ikln
ilttilt;klnilttiltl;lnilttiltli;nilttiltlic;ilttiltlici;lttiltlicis;ttiltlicisc;tiltlicisca;iltliciscav;ltliciscav
d;tliciscavdk;liciscavdki;iciscavdkid;ciscavdkidp;iscavdkidpe;scavdkidpes;cavdkidpesk;a
vdkidpesks;vdkidpesksk;dkidpeskskt;kidpesksktn;idpesksktnt;dpesksktntk;pesksktntkd;es
ksktntkdf;sksktntkdfe;ksktntkdfen;sktntkdfenk;ktntkdfenks;tntkdfenksq;ntkdfenksqd;tkdf
enksqdl;kdfenksqdle;dfenksqdlep;fenksqdleps;enksqdlepsn;nksqdlepsnt;ksqdlepsntk;sqdl
epsntkn;qdlepsntknk;dlepsntknkd;lepsntknkdl;epsntknkdle;psntknkdlep;sntknkdlepl;ntkn
kdleple;tknkdleplek;knkdleplekn;nkdleplekns;kdlepleknsk;dlepleknske;lepleknsket;eplek
nsketi;pleknsketii;leknsketiis;eknsketiisk;knsketiiskl;nsketiiskle;sketiisklek;ketiisklekm;e
tiisklekmi;tiisklekmik;iisklekmikd;isklekmikdl;sklekmikdle;klekmikdled;lekmikdledq;ek
mikdledqk;kmikdledqkd;mikdledqkdq;ikdledqkdqe;kdledqkdqed;dledqkdqedt;ledqkdqedt
e;edqkdqedtei;dqkdqedteia;qkdqedteiak;kdqedteiaki;dqedteiakit;qedteiakitn;edteiakitnt;dt
eiakitntq;teiakitntqi;eiakitntqid;iakitntqidf;akitntqidfl;kitntqidfle;itntqidflen;tntqidflenf;nt
qidflenfk;tqidflenfks;qidflenfkse;idflenfksep;dflenfkseph;flenfksephd;lenfksephdt;enfkse
phdti;nfksephdtis;fksephdtise;ksephdtised;sephdtisedi;ephdtisedie;phdtisediem;hdtisedie
mk;dtisediemki;tisediemkik;isediemkikr;sediemkikri;ediemkikrii;diemkikriiy;iemkikriiys
;emkikriiyss;mkikriiyssl;kikriiyssln;ikriiysslny;kriiysslnye;riiysslnyei;iiysslnyeie;iysslnye
iek;ysslnyeieki;sslnyeiekin;slnyeiekint;lnyeiekintl;nyeiekintlk;yeiekintlke;eiekintlkei;ieki
ntlkeil;ekintlkeild;kintlkeildk;intlkeildkl;ntlkeildkly;tlkeildklyk;lkeildklykn;keildklyknh;e
ildklyknhk;ildklyknhky;ldklyknhkyk;dklyknhkykt;klyknhkyktt;lyknhkyktta;yknhkykttar;

knhkykttarn;nhkykttarnf;hkykttarnfi;kykttarnfil;ykttarnfiln;kttarnfilni;ttarnfilnis;tarnfilnis
i;arnfilnisik;rnfilnisiki;nfilnisikiq;filnisikiqf;ilnisikiqfq;lnisikiqfqi;nisikiqfqie;isikiqfqien;
sikiqfqiena;ikiqfqienal;kiqfqienale;iqfqienalel;qfqienalelm;fqienalelmk;qienalelmke;ienal
elmkee;enalelmkeei;nalelmkeeie;alelmkeeied;lelmkeeieda;elmkeeiedas;lmkeeiedase;mke
eiedasei;keeiedaseil;eeiedaseiln;eiedaseilnq;iedaseilnqe;edaseilnqer;daseilnqery;aseilnqer
ye;seilnqeryei;eilnqeryeil;ilnqeryeill;lnqeryeillk;nqeryeillkh;qeryeillkhv;eryeillkhve;ryeill
khvep;yeillkhveps;eillkhvepsl;illkhvepsln;llkhvepslnl;lkhvepslnlk;khvepslnlkq;hvepslnlk
qk;vepslnlkqkf;epslnlkqkfe;pslnlkqkfek;slnlkqkfeki;lnlkqkfekil;nlkqkfekiln;lkqkfekilne;k
qkfekilnet;qkfekilneti;kfekilnetik;fekilnetika;ekilnetikay;kilnetikayn;ilnetikaynq;lnetikay
nqd;netikaynqdl;etikaynqdld;tikaynqdldn;ikaynqdldni;kaynqdldnik;aynqdldniks;ynqdldni
ksn;nqdldniksne;qdldniksned;dldniksnedq;ldniksnedql;dniksnedqla;niksnedqlah;iksnedql
ahm;ksnedqlahmd;snedqlahmde;nedqlahmden;edqlahmdeny;dqlahmdenyk;qlahmdenyke;
lahmdenykef;ahmdenykefd;hmdenykefdp;mdenykefdpl;denykefdpln;enykefdplnl;nykefd
plnld;ykefdplnldy;

13 mers:
mkktklniiklni;kktklniiklnil;ktklniiklnilt;tklniiklnilt;klniiklniltti;lniiklnilttil;niiklnilttilt;iikl
nilttiltl;iklnilttiltli;klnilttiltlic;lnilttiltlici;nilttiltlicis;ilttiltlicisc;lttiltlicisca;ttiltliciscav;tiltli
ciscavd;iltliciscavdk;ltliciscavdki;tliciscavdkid;liciscavdkidp;iciscavdkidpe;ciscavdkidpes
;iscavdkidpesk;scavdkidpesks;cavdkidpessk;avdkidpeskskt;vdkidpesksktn;dkidpesksktn
t;kidpesksktntk;idpesksktntkd;dpesksktntkdf;pesksktntkdfe;esksktntkdfen;sksktntkdfenk;
ksktntkdfenks;sktntkdfenksq;ktntkdfenksqd;tntkdfenksqdl;ntkdfenksqdle;tkdfenksqdlep;k
dfenksqdleps;dfenksqdlepsn;fenksqdlepsnt;enksqdlepsntk;nksqdlepsntkn;ksqdlepsntknk;s
qdlepsntknkd;qdlepsntknkdl;dlepsntknkdle;lepsntknkdlep;epsntknkdlepl;psntknkdleple;s
ntknkdleplek;ntknkdleplekn;tknkdleplekns;knkdlepleknsk;nkdlepleknske;kdleplekensket;d
leplekensketi;leplekensketii;eplekensketiis;pleknsketiisk;leknsketiiskl;eknsketiiskle;knsketiis
klek;nsketiisklekm;sketiisklekmi;ketiisklekmik;etiisklekmikd;tiisklekmikdl;iisklekmikdle
;isklekmikdled;sklekmikdledq;klekmikdledqk;lekmikdledqkd;ekmikdledqkdq;kmikdledq
kdqe;mikdledqkdqed;ikdledqkdqedt;kdledqkdqedte;dledqkdqedtei;ledqkdqedteia;edqkdq
edteiak;dqkdqedteiaki;qkdqedteiakit;kdqedteiakitn;dqedteiakitnt;qedteiakitntq;edteiakitnt
qi;dteiakitntqid;teiakitntqidf;eiakitntqidfl;iakitntqidfle;akitntqidflen;kitntqidflenf;itntqidfl
enfk;tntqidflenfks;ntqidflenfkse;tqidflenfksep;qidflenfkseph;idflenfksephd;dflenfksephdt;
flenfksephdti;lenfksephdtis;enfksephdtise;nfksephdtised;fksephdtisedi;ksephdtisedie;seph
dtisediem;ephdtisediemk;phdtisediemki;hdtisediemkik;dtisediemkikr;tisediemkikri;isedie
mkikrii;sediemkikriiy;ediemkikriiys;diemkikriiyss;iemkikriiyssl;emkikriiyssln;mkikriiyss
lny;kikriiysslnye;ikriiysslnyei;kriiysslnyeie;riiysslnyeiek;iiysslnyeieki;iysslnyeiekin;yssln
yeiekint;sslnyeiekintl;slnyeiekintlk;lnyeiekintlke;nyeiekintlkei;yeiekintlkeil;eiekintlkeild;
iekintlkeildk;ekintlkeildkl;kintlkeildkly;intlkeildklyk;ntlkeildklykn;tlkeildklyknh;lkeildkl
yknhk;keildklyknhky;eildklyknhkyk;ildklyknhkykt;ldklyknhkyktt;dklyknhkyktta;klyknhk
ykttar;lyknhkykttarn;yknhkykttarnf;knhkykttarnfi;nhkykttarnfil;hkykttarnfiln;kykttarnfiln
i;ykttarnfilnis;kttarnfilnisi;ttarnfilnisik;tarnfilnisiki;arnfilnisikiq;rnfilnisikiqf;nfilnisikiqfq;
filnisikiqfqi;ilnisikiqfqie;lnisikiqfqien;nisikiqfqiena;isikiqfqienal;sikiqfqienale;ikiqfqienal
el;kiqfqienalelm;iqfqienalelmk;qfqienalelmke;fqienalelmkee;qienalelmkeei;ienalelmkeei
e;enalelmkeeied;nalelmkeeieda;alelmkeeiedas;lelmkeeiedase;elmkeeiedasei;lmkeeiedasei
l;mkeeiedaseiln;keeiedaseilnq;eeiedaseilnqe;eiedaseilnqer;iedaseilnqery;edaseilnqerye;da
seilnqeryei;aseilnqeryeil;seilnqeryeill;eilnqeryeillk;ilnqeryeillkh;lnqeryeillkhv;nqeryeillk

hve;qeryeillkhvep;eryeillkhveps;ryeillkhvepsl;yeillkhvepsln;eillkhvepslnl;illkhvepslnlk;ll
khvepslnlkq;lkhvepslnlkqk;khvepslnlkqkf;hvepslnlkqkfe;vepslnlkqkfek;epslnlkqkfeki;psl
nlkqkfekil;slnlkqkfekiln;lnlkqkfekilne;nlkqkfekilnet;lkqkfekilneti;kqkfekilnetik;qkfekilne
tika;kfekilnetikay;fekilnetikayn;ekilnetikaynq;kilnetikaynqd;ilnetikaynqdl;lnetikaynqdld;
netikaynqdldn;etikaynqdldni;tikaynqdldnik;ikaynqdldniks;kaynqdldniksn;aynqdldniksne;
ynqdldniksned;nqdldniksnedq;qdldniksnedql;dldniksnedqla;ldniksnedqlah;dniksnedqlah
m;niksnedqlahmd;iksnedqlahmde;ksnedqlahmden;snedqlahmdeny;nedqlahmdenyk;edqla
hmdenyke;dqlahmdenykef;qlahmdenykefd;lahmdenykefdp;ahmdenykefdpl;hmdenykefdp
ln;mdenykefdplnl;denykefdplnld;enykefdplnldy;

14 mers:
mkktklniiklnil;kktklniiklnilt;ktklniiklniltt;tklniiklniltti;klniiklnilttil;lniiklnilttilt;niiklnilttil
tl;iiklnilttiltli;iklnilttiltlic;klnilttiltlici;lnilttiltlicis;nilttiltlicisc;ilttiltlicisca;lttiltliciscav;ttiltl
iciscavd;tiltliciscavdk;iltliciscavdki;ltliciscavdkid;tliciscavdkidp;liciscavdkidpe;iciscavdk
idpes;ciscavdkidpesk;iscavdkidpesks;scavdkidpesksk;cavdkidpeskskt;avdkidpesksktn;vd
kidpesksktnt;dkidpesksktntk;kidpesksktntkd;idpesksktntkdf;dpesksktntkdfe;pesksktntkdf
en;esksktntkdfenk;sksktntkdfenks;ksktntkdfenksq;sktntkdfenksqd;ktntkdfenksqdl;tntkdfe
nksqdle;ntkdfenksqdlep;tkdfenksqdleps;kdfenksqdlepsn;dfenksqdlepsnt;fenksqdlepsntk;e
nksqdlepsntkn;nksqdlepsntknk;ksqdlepsntknkd;sqdlepsntknkdl;qdlepsntknkdle;dlepsntkn
kdlep;lepsntknkdlepl;epsntknkdleple;psntknkdleplek;sntknkdleplekn;ntknkdleplekns;tknk
dlepleknsk;knkdlepleknske;nkdleplekinsket;kdlepleknsketi;dlepleknsketii;lepleknsketiis;e
plepleknsketiisk;plepleknsketiiskl;leknsketiiskle;eknsketiisklek;knsketiisklekm;nsketiisklekmi;s
ketiisklekmik;ketiisklekmikd;etiisklekmikdl;tiisklekmikdle;iisklekmikdled;isklekmikdled
q;sklekmikdledqk;klekmikdledqkd;lekmikdledqkdq;ekmikdledqkdqe;kmikdledqkdqed;mi
kdledqkdqedt;ikdledqkdqedte;kdledqkdqedtei;dledqkdqedteia;ledqkdqedteiak;edqkdqedte
iaki;dqkdqedteiakit;qkdqedteiakitn;kdqedteiakitnt;dqedteiakitntq;qedteiakitntqi;edteiakitn
tqid;dteiakitntqidf;teiakitntqidfl;eiakitntqidfle;iakitntqidflen;akitntqidflenf;kitntqidflenfk;i
tntqidflenfks;tntqidflenfkse;ntqidflenfksep;tqidflenfkseph;qidflenfksephd;idflenfksephdt;
dflenfksephdti;flenfksephdtis;lenfksephdtise;enfksephdtised;nfksephdtisedi;fksephdtisedi
e;ksephdtisediem;sephdtisediemk;ephdtisediemki;phdtisediemkik;hdtisediemkikr;dtisedie
mkikri;tisediemkikrii;isediemkikriiy;sediemkikriiys;ediemkikriiyss;diemkikriiyssl;iemkik
riiyssln;emkikriiysslny;mkikriiysslnye;kikriiysslnyei;ikriiysslnyeie;kriiysslnyeiek;riiyssln
yeieki;iiysslnyeiekin;iysslnyeiekint;ysslnyeiekintl;sslnyeiekintlk;slnyeiekintlke;lnyeiekint
lkei;nyeiekintlkeil;yeiekintlkeild;eiekintlkeildk;iekintlkeildkl;ekintlkeildkly;kintlkeildkly
k;intlkeildklykn;ntlkeildklyknh;tlkeildklyknhk;lkeildklyknhky;keildklyknhkyk;eildklykn
hkykt;ildklyknhkyktt;ldklyknhkyktta;dklyknhkykttar;klyknhkykttarn;lyknhkykttarnf;ykn
hkykttarnfi;knhkykttarnfil;nhkykttarnfiln;hkykttarnfilni;kykttarnfilnis;ykttarnfilnisi;kttarn
filnisik;ttarnfilnisiki;tarnfilnisikiq;arnfilnisikiqf;rnfilnisikiqfq;nfilnisikiqfqi;filnisikiqfqie;
ilnisikiqfqien;lnisikiqfqiena;nisikiqfqienal;isikiqfqienale;sikiqfqienalel;ikiqfqienalelm;kiq
fqienalelmk;iqfqienalelmke;qfqienalelmkee;fqienalelmkeei;qienalelmkeeie;ienalelmkeeie
d;enalelmkeeieda;nalelmkeeiedas;alelmkeeiedase;lelmkeeiedasei;elmkeeiedaseil;lmkeeie
daseiln;mkeeiedaseilnq;keeiedaseilnqe;eeiedaseilnqer;eiedaseilnqery;iedaseilnqerye;edas
eilnqeryei;daseilnqeryeil;aseilnqeryeill;seilnqeryeillk;eilnqeryeillkh;ilnqeryeillkhv;lnqery
eillkhve;nqeryeillkhvep;qeryeillkhveps;eryeillkhvepsl;ryeillkhvepsln;yeillkhvepslnl;eillk
hvepslnlk;illkhvepslnlkq;llkhvepslnlkqk;lkhvepslnlkqkf;khvepslnlkqkfe;hvepslnlkqkfek;
vepslnlkqkfeki;epslnlkqkfekil;pslnlkqkfekiln;slnlkqkfekilne;lnlkqkfekilnet;nlkqkfekilneti

;lkqkfekilnetik;kqkfekilnetika;qkfekilnetikay;kfekilnetikayn;fekilnetikaynq;ekilnetikaynq
d;kilnetikaynqdl;ilnetikaynqdld;lnetikaynqdldn;netikaynqdldni;etikaynqdldnik;tikaynqdld
niks;ikaynqdldniksn;kaynqdldniksne;aynqdldniksned;ynqdldniksnedq;nqdldniksnedql;qdl
dniksnedqla;dldniksnedqlah;ldniksnedqlahm;dniksnedqlahmd;niksnedqlahmde;iksnedqla
hmden;ksnedqlahmdeny;snedqlahmdenyk;nedqlahmdenyke;edqlahmdenykef;dqlahmden
ykefd;qlahmdenykefdp;lahmdenykefdpl;ahmdenykefdpln;hmdenykefdplnl;mdenykefdpln
ld;denykefdplnldy;

15 mers:
mkktklniiklnilt;kktklniiklniltt;ktklniiklniltti;tklniiklnilttil;klniiklnilttilt;lniiklnilttiltl;niikln
ilttiltli;iiklnilttiltlic;iklnilttiltlici;klnilttiltlicis;lnilttiltlicisc;nilttiltlicisca;ilttiltliciscav;lttiltli
ciscavd;ttiltliciscavdk;tiltliciscavdki;iltliciscavdkid;ltliciscavdkidp;tliciscavdkidpe;licisca
vdkidpes;iciscavdkidpesk;ciscavdkidpesks;iscavdkidpesksk;scavdkidpeskskt;cavdkidpesk
sktn;avdkidpesksktnt;vdkidpesksktntk;dkidpesksktntkd;kidpesksktntkdf;idpesksktntkdfe;
dpesksktntkdfen;pesksktntkdfenk;esksktntkdfenks;sksktntkdfenksq;ksktntkdfenksqd;sktnt
kdfenksqdl;ktntkdfenksqdle;tntkdfenksqdlep;ntkdfenksqdleps;tkdfenksqdlepsn;kdfenksqd
lepsnt;dfenksqdlepsntk;fenksqdlepsntkn;enksqdlepsntknk;nksqdlepsntknkd;ksqdlepsntkn
kdl;sqdlepsntknkdle;qdlepsntknkdlep;dlepsntknkdlepl;lepsntknkdleple;epsntknkdleplek;p
sntknkdleplekn;sntknkdleplekns;ntknkdlepleknsk;tknkdlepleknske;knkdlepleknsket;nkdle
pleknsketi;kdlepleknsketii;dlepleknsketiis;lepleknsketiisk;epleknsketiiskl;pleknsketiiskle;
leknsketiisklek;eknsketiisklekm;knsketiisklekmi;nsketiisklekmik;sketiisklekmikd;ketiiskl
ekmikdl;etiisklekmikdle;tiisklekmikdled;iisklekmikdledq;isklekmikdledqk;sklekmikdled
qkd;klekmikdledqkdq;lekmikdledqkdqe;ekmikdledqkdqed;kmikdledqkdqedt;mikdledqkd
qedte;ikdledqkdqedtei;kdledqkdqedteia;dledqkdqedteiak;ledqkdqedteiaki;edqkdqedteiakit
;dqkdqedteiakitn;qkdqedteiakitnt;kdqedteiakitntq;dqedteiakitntqi;qedteiakitntqid;edteiakit
ntqidf;dteiakitntqidfl;teiakitntqidfle;eiakitntqidflen;iakitntqidflenf;akitntqidflenfk;kitntqid
flenfks;itntqidflenfkse;tntqidflenfksep;ntqidflenfkseph;tqidflenfksephd;qidflenfksephdt;id
flenfksephdti;dflenfksephdtis;flenfksephdtise;lenfksephdtised;enfksephdtisedi;nfksephdti
sedie;fksephdtisediem;ksephdtisediemk;sephdtisediemki;ephdtisediemkik;phdtisediemkik
r;hdtisediemkikri;dtisediemkikrii;tisediemkikriiy;isediemkikriiys;sediemkikriiyss;ediemk
ikriiyssl;diemkikriiyssln;iemkikriiysslny;emkikriiysslnye;mkikriiysslnyei;kikriiysslnyeie;
ikriiysslnyeiek;kriiysslnyeieki;riiysslnyeiekin;iiysslnyeiekint;iysslnyeiekintl;ysslnyeiekint
lk;sslnyeiekintlke;slnyeiekintlkei;lnyeiekintlkeil;nyeiekintlkeild;yeiekintlkeildk;eiekintlk
eildkl;iekintlkeildkly;ekintlkeildklyk;kintlkeildklykn;intlkeildklyknh;ntlkeildklyknhk;tlke
ildklyknhky;lkeildklyknhkyk;keildklyknhkykt;eildklyknhkytt;ildklyknhkytta;ldklyknh
kykttar;dklyknhkykttarn;klyknhkykttarnf;lyknhkykttarnfi;yknhkykttarnfil;knhkykttarnfiln
;nhkykttarnfilni;hkykttarnfilnis;kykttarnfilnisi;ykttarnfilnisik;kttarnfilnisiki;ttarnfilnisikiq
;tarnfilnisikiqf;arnfilnisikiqfq;rnfilnisikiqfqi;nfilnisikiqfqie;filnisikiqfqien;ilnisikiqfqiena;
lnisikiqfqienal;nisikiqfqienale;isikiqfqienalel;sikiqfqienalelm;ikiqfqienalelmk;kiqfqienale
lmke;iqfqienalelmkee;qfqienalelmkeei;fqienalelmkeeie;qienalelmkeeied;ienalelmkeeieda;
enalelmkeeiedas;nalelmkeeiedase;alelmkeeiedasei;lelmkeeiedaseil;elmkeeiedaseiln;lmke
eiedaseilnq;mkeeiedaseilnqe;keeiedaseilnqer;eeiedaseilnqery;eiedaseilnqerye;iedaseilnqe
ryei;edaseilnqeryeil;daseilnqeryeill;aseilnqeryeillk;seilnqeryeillkh;eilnqeryeillkhv;ilnqery
eillkhve;lnqeryeillkhvep;nqeryeillkhveps;qeryeillkhvepsl;eryeillkhvepsln;ryeillkhvepslnl;
yeillkhvepslnlk;eillkhvepslnlkq;illkhvepslnlkqk;llkhvepslnlkqkf;lkhvepslnlkqkfe;khvepsl
nlkqkfek;hvepslnlkqkfeki;vepslnlkqkfekil;epslnlkqkfekiln;pslnlkqkfekilne;slnlkqkfekilne

t;lnlkqkfekilneti;nlkqkfekilnetik;lkqkfekilnetika;kqkfekilnetikay;qkfekilnetikayn;kfekilne
tikaynq;fekilnetikaynqd;ekilnetikaynqdl;kilnetikaynqdld;ilnetikaynqdldn;lnetikaynqdldni;
netikaynqdldnik;etikaynqdldniks;tikaynqdldniksn;ikaynqdldniksne;kaynqdldniksned;ayn
qdldniksnedq;ynqdldniksnedql;nqdldniksnedqla;qdldniksnedqlah;dldniksnedqlahm;ldniks
nedqlahmd;dniksnedqlahmde;niksnedqlahmden;iksnedqlahmdeny;ksnedqlahmdenyk;sne
dqlahmdenyke;nedqlahmdenykef;edqlahmdenykefd;dqlahmdenykefdp;qlahmdenykefdpl;
lahmdenykefdpln;ahmdenykefdplnl;hmdenykefdplnld;mdenykefdplnldy;

16 mers:
mkktklniiklniltt;kktklniiklniltti;ktklniiklnilttil;tklniiklnilttilt;klniiklnilttiltl;lniiklnilttiltli;ni
iklnilttiltlic;iiklnilttiltlici;iklnilttiltlicis;klnilttiltlicisc;lnilttiltlicisca;nilttiltliciscav;ilttiltlici
scavd;lttiltliciscavdk;ttiltliciscavdki;tiltliciscavdkid;iltliciscavdkidp;ltliciscavdkidpe;tlicis
cavdkidpes;liciscavdkidpesk;iciscavdkidpesks;ciscavdkidpesksk;iscavdkidpeskskt;scavdk
idpeskskin;cavdkidpesksktnt;avdkidpesksktntk;vdkidpesksktntkd;dkidpesksktntkdf;kidpe
sksktntkdfe;idpesksktntkdfen;dpesksktntkdfenk;pesksktntkdfenks;esksktntkdfenksq;skskt
ntkdfenksqd;ksktntkdfenksqdl;sktntkdfenksqdle;ktntkdfenksqdlep;tntkdfenksqdleps;ntkdf
enksqdlepsn;tkdfenksqdlepsnt;kdfenksqdlepsntk;dfenksqdlepsntkn;fenksqdlepsntknk;enk
sqdlepsntknkd;nksqdlepsntknkdl;ksqdlepsntknkdle;sqdlepsntknkdlep;qdlepsntknkdlepl;dl
epsntknkdleple;lepsntknkdleplek;epsntknkdleplekn;psntknkdleplekns;sntknkdleplekns;n
tknkdleplekns;tknkdleplekns;knkdlepleksneti;nkdleplekns;kdleplekns;dle
pleknsketiisk;lepleknsketiisk;epleknsketiiskle;pleknsketiisklek;leknsketiisklekm;eknsketi
isklekmi;knsketiisklekmik;nsketiisklekmikd;sketiisklekmikdl;ketiisklekmikdle;etiisklekm
ikdled;tiisklekmikdledq;iisklekmikdledqk;isklekmikdledqkd;sklekmikdledqkdq;klekmikd
ledqkdqe;lekmikdledqkdqed;ekmikdledqkdqedt;kmikdledqkdqedte;mikdledqkdqedtei;ikd
ledqkdqedteia;kdledqkdqedteiak;dledqkdqedteiaki;ledqkdqedteiakit;edqkdqedteiakitn;dq
kdqedteiakitnt;qkdqedteiakitntq;kdqedteiakitntqi;dqedteiakitntqid;qedteiakitntqidf;edteia
kitntqidfl;dteiakitntqidfle;teiakitntqidflen;eiakitntqidflenf;iakitntqidflenfk;akitntqidflenfk
s;kitntqidflenfkse;itntqidflenfksep;tntqidflenfkseph;ntqidflenfksephd;tqidflenfksephdt;qid
flenfksephdti;idflenfksephdtis;dflenfksephdtise;flenfksephdtised;lenfksephdtisedi;enfksep
hdtisedie;nfksephdtisediem;fksephdtisediemk;ksephdtisediemki;sephdtisediemkik;ephdtis
ediemkikr;phdtisediemkikri;hdtisediemkikrii;dtisediemkikriiy;tisediemkikriiys;isediemki
kriiyss;sediemkikriiyssl;ediemkikriiyssln;diemkikriiysslny;iemkikriiysslnye;emkikriiyssl
nyei;mkikriiysslnyeie;kikriiysslnyeiek;ikriiysslnyeieki;kriiysslnyeiekin;riiysslnyeiekint;ii
ysslnyeiekintl;iysslnyeiekintlk;ysslnyeiekintlke;sslnyeiekintlkei;slnyeiekintlkeil;lnyeiekin
tlkeild;nyeiekintlkeildk;yeiekintlkeildkl;eiekintlkeildkly;iekintlkeildklyk;ekintlkeildklykn
;kintlkeildklyknh;intlkeildklyknhk;ntlkeildklyknhky;tlkeildklyknhkyk;lkeildklyknhkykt;k
eildklyknhkyktt;eildklyknhkyktta;ildklyknhkykttar;ldklyknhkykttarn;dklyknhkykttarnf;kl
yknhkykttarnfi;lyknhkykttarnfil;yknhkykttarnfiln;knhkykttarnfilni;nhkykttarnfilnis;hkyktt
arnfilnisi;kykttarnfilnisik;ykttarnfilnisiki;kttarnfilnisikiq;ttarnfilnisikiqf;tarnfilnisikiqfq;ar
nfilnisikiqfqi;rnfilnisikiqfqie;nfilnisikiqfqien;filnisikiqfqiena;ilnisikiqfqienal;lnisikiqfqien
ale;nisikiqfqienalel;isikiqfqienalelm;sikiqfqienalelmk;ikiqfqienalelmke;kiqfqienalelmkee;
iqfqienalelmkeei;qfqienalelmkeeie;fqienalelmkeeied;qienalelmkeeieda;ienalelmkeeiedas;
enalelmkeeiedase;nalelmkeeiedasei;alelmkeeiedaseil;lelmkeeiedaseiln;elmkeeiedaseilnq;l
mkeeiedaseilnqe;mkeeiedaseilnqer;keeiedaseilnqery;eeiedaseilnqerye;eiedaseilnqeryei;ie
daseilnqeryeil;edaseilnqeryeill;daseilnqeryeillk;aseilnqeryeillkh;seilnqeryeillkhv;eilnqery
eillkhve;ilnqeryeillkhvep;lnqeryeillkhveps;nqeryeillkhvepsl;qeryeillkhvepsln;eryeillkhve

pslnl;ryeillkhvepslnlk;yeillkhvepslnlkq;eillkhvepslnlkqk;illkhvepslnlkqkf;llkhvepslnlkqkf
e;lkhvepslnlkqkfek;khvepslnlkqkfeki;hvepslnlkqkfekil;vepslnlkqkfekiln;epslnlkqkfekilne
;pslnlkqkfekilnet;slnlkqkfekilneti;lnlkqkfekilnetik;nlkqkfekilnetika;lkqkfekilnetikay;kqkf
ekilnetikayn;qkfekilnetikaynq;kfekilnetikaynqd;fekilnetikaynqdl;ekilnetikaynqdld;kilneti
kaynqdldn;ilnetikaynqdldni;lnetikaynqdldnik;netikaynqdldniks;etikaynqdldniksn;tikaynq
dldniksne;ikaynqdldniksned;kaynqdldniksnedq;aynqdldniksnedql;ynqdldniksnedqla;nqdl
dniksnedqlah;qdldniksnedqlahm;dldniksnedqlahmd;ldniksnedqlahmde;dniksnedqlahmde
n;niksnedqlahmdeny;iksnedqlahmdenyk;ksnedqlahmdenyke;snedqlahmdenykef;nedqlah
mdenykefd;edqlahmdenykefdp;dqlahmdenykefdpl;qlahmdenykefdpln;lahmdenykefdplnl;
ahmdenykefdplnld;hmdenykefdplnldy;

## \<AAU07022 4-alpha-glucanotransferase;Protein;Borrelia garinii PBi\>

8 mers:

mkykkiri;kykkirin;ykkirinl;kkirinln;kirinlnl;irinlnlk;rinlnlkr;inlnlkrk;nlnlkrks;lnlkrksg;nl
krksgi;lkrksgil;krksgill;rksgilln;ksgillni;sgillnis;gillniss;illnissl;llnisslp;lnisslps;nisslpsk;is
slpsky;sslpskyg;slpskygi;lpskygig;pskygigd;skygigdl;kygigdlg;ygigdlgk;gigdlgkg;igdlgk
ga;gdlgkgay;dlgkgayk;lgkgaykf;gkgaykfi;kgaykfid;gaykfidf;aykfidfl;ykfidflf;kfidflfa;fidf
lfas;idflfass;dflfassq;flfassqs;lfassqsy;fassqsyw;assqsywq;ssqsywqm;sqsywqmf;qsywqmf
a;sywqmfay;ywqmfays;wqmfaysp;qmfayspi;mfayspid;fayspidf;ayspidft;yspidftr;spidftrs;
pidftrsp;idftrspp;dftrsppy;ftrsppys;trsppysi;rsppysif;sppysifs;ppysifsa;pysifsaf;ysifsafa;sif
safag;ifsafagn;fsafagnv;safagnvy;afagnvyy;fagnvyyi;agnvyyid;gnvyyidl;nvyyidle;vyyidle
a;yyidleal;yidleald;idlealdk;dlealdkf;lealdkfi;ealdkfid;aldkfids;ldkfidsd;dkfidsdl;kfidsdln;
fidsdlnl;idsdlnll;dsdlnllk;sdlnllke;dlnllken;lnllkene;nllkenet;llkenetr;lkenetry;kenetrys;en
etrysd;netrysdl;etrysdlk;trysdlkk;rysdlkkl;ysdlkkls;sdlkklsf;dlkklsfk;lkklsfkd;kklsfkdk;kls
fkdkf;lsfkdkfl;sfkdkflk;fkdkflke;kdkflkea;dkflkeaa;kflkeaal;flkeaaln;lkeaalnf;keaalnfi;eaa
lnfin;aalnfinr;alnfinra;lnfinras;nfinrasv;finrasvd;inrasvde;nrasvdev;rasvdevr;asvdevrs;svd
evrsf;vdevrsfe;devrsfek;evrsfekf;vrsfekfk;rsfekfkk;sfekfkkk;fekfkkks;ekfkkkss;kfkkkssy;
fkkkssyw;kkkssywl;kkssywll;kssywlld;ssywlldf;sywlldfa;ywlldfas;wlldfasf;lldfasfv;ldfas
fva;dfasfvaf;fasfvafk;asfvafke;sfvafkey;fvafkeyf;vafkeyfl;afkeyflk;fkeyflkd;keyflkds;eyfl
kdsr;yflkdsrd;flkdsrda;lkdsrdaf;kdsrdafn;dsrdafnv;srdafnvl;rdafnvlf;dafnvlfd;afnvlfdr;fnv
lfdrg;nvlfdrgi;vlfdrgil;lfdrgili;fdrgilir;drgilirn;rgilirne;gilirnek;ilirnekd;lirnekdl;irnekdlf;r
nekdlfk;nekdlfkl;ekdlfklr;kdlfklrn;dlfklrni;lfklrnil;fklrnils;klrnilsk;lrnilske;rnilskei;nilskei
k;ilskeikv;lskeikvq;skeikvqe;keikvqev;eikvqevl;ikvqevlq;kvqevlqy;vqevlqyf;qevlqyff;evl
qyfff;vlqyfffs;lqyfffsq;qyfffsqf;yfffsqfq;fffsqfqa;ffsqfqal;fsqfqalk;sqfqalkr;qfqalkry;fqalk
rya;qalkryan;alkryand;lkryandk;kryandkg;ryandkgi;yandkgie;andkgiel;ndkgielv;dkgielv
m;kgielvmn;gielvmnl;ielvmnlp;elvmnlpl;lvmnlplf;vmnlplfi;mnlplfia;nlplfiay;lplfiayd;plfi
ayds;lfiaydsa;fiaydsad;iaydsadv;aydsadvw;ydsadvwa;dsadvwah;sadvwahq;advwahqk;dv
wahqky;vwahqkyf;wahqkyfk;ahqkyfkl;hqkyfklr;qkyfklrf;kyfklrfd;yfklrfda;fklrfdas;klrfda
sk;lrfdaskd;rfdaskdk;fdaskdkv;daskdkva;askdkvag;skdkvagi;kdkvagis;dkvagisp;kvagispd
;vagispdy;agispdyf;gispdyfl;ispdyfle;spdyfleq;pdyfleqe;dyfleqeq;yfleqeqa;fleqeqaw;leqe
qawd;eqeqawds;qeqawdsp;eqawdspa;qawdspay;awdspays;wdspaysw;dspayswn;spayswn
v;payswnvl;ayswnvlk;yswnvlkn;swnvlknv;wnvlknvk;nvlknvky;vlknvkye;lknvkyew;knvk
yeww;nvkyewwa;vkyewwak;kyewwakr;yewwakri;ewwakrig;wwakrigi;wakrigil;akrigilr;
krigilrk;rigilrky;igilrkyi;gilrkyid;ilrkyidv;lrkyidvi;rkyidvik;kyidviki;yidvikid;idvikidh;dvi
kidhf;vikidhfr;ikidhfrg;kidhfrgf;idhfrgfv;dhfrgfvs;hfrgfvst;frgfvstw;rgfvstwe;gfvstwev;fv

stwevg;vstwevgv;stwevgvg;twevgvge;wevgvgea;evgvgeay;vgvgeaya;gvgeayaf;vgeayafn;
geayafng;eayafngl;ayafnglw;yafnglwv;afnglwvk;fnglwvks;nglwvksp;glwvkspg;lwvkspgr
;wvkspgrd;vkspgrdf;kspgrdff;spgrdffn;pgrdffnf;grdffnfi;rdffnfil;dffnfiln;ffnfilne;fnfilnei;
nfilneik;filneikd;ilneikdl;lneikdlk;neikdlki;eikdlkiw;ikdlkiwv;kdlkiwve;dlkiwved;lkiwve
df;kiwvedfe;iwvedfen;wvedfend;vedfendl;edfendle;dfendled;fendledv;endledvs;ndledvsr;
dledvsrl;ledvsrlr;edvsrlrd;dvsrlrdf;vsrlrdff;srlrdffn;rlrdffnf;lrdffnfp;rdffnfpg;dffnfpgm;ffn
fpgmr;fnfpgmri;nfpgmrim;fpgmrimk;pgmrimkl;gmrimkla;mrimklaf;rimklafd;imklafdf;m
klafdfd;klafdfds;lafdfdsd;afdfdsdn;fdfdsdnq;dfdsdnqn;fdsdnqnl;dsdnqnlp;sdnqnlph;dnqnl
phn;nqnlphny;qnlphnyi;nlphnyik;lphnyikn;phnyiknc;hnyiknci;nyikinciv;yikncivy;ikncivyt
;kncivytg;ncivytgi;civytgig;ivytgigd;vytgigdn;ytgigdns;tgigdnst;gigdnsti;igdnstir;gdnstire
;dnstiref;nstirefv;stirefvn;tirefvns;irefvnsl;refvnsld;efvnsldd;fvnslddl;vnslddlh;nslddlhk;sl
ddlhkk;lddlhkky;ddlhkkyi;dlhkkyif;lhkkyifd;hkkyifdy;kkyifdyl;kyifdyln;yifdylnt;ifdylntn
;fdylntne;dylntned;ylntnedf;lntnedfv;ntnedfvv;tnedfvvw;nedfvvwd;edfvvwdm;dfvvwdmi;
fvvwdmir;vvwdmirs;vwdmirsa;wdmirsam;dmirsams;mirsamss;irsamssv;rsamssvs;samssv
sd;amssvsds;mssvsdsv;ssvsdsvi;svsdsvii;vsdsviip;sdsviipm;dsviipmq;sviipmqd;viipmqdy
;iipmqdyi;ipmqdyin;pmqdyinl;mqdyinlg;qdyinlgn;dyinlgne;yinlgnef;inlgnefr;nlgnefra;lg
nefran;gnefrani;nefranip;efranipk;franipkn;ranipknt;anipkntl;nipkntln;ipkntlnn;pkntlnnw;
kntlnnwi;ntlnnwif;tlnnwifr;lnnwifrl;nnwifrll;nwifrlle;wifrlles;ifrllesd;frllesdl;rllesdld;lles
dlda;lesdldat;esdldatl;sdldatls;dldatlsk;ldatlskn;datlskni;atlsknis;tlsknisf;lsknisfi;sknisfit;k
nisfitr;nisfitrl;isfitrly;sfitrlyg;fitrlygr;itrlygrt;

9 mers:
mkykkirin;kykkirinl;ykkirinln;kkirinlnl;kirinlnlk;irinlnlkr;rinlnlkrk;inlnlkrks;nlnlkrksg;ln
lkrksgi;nlkrksgil;lkrksgill;krksgilln;rksgillni;ksgillnis;sgillniss;gillnissl;illnisslp;llnisslps;l
nisslpsk;nisslpsky;isslpskyg;sslpskygi;slpskygig;lpskygigd;pskygigdl;skygigdlg;kygigdlg
k;ygigdlgkg;gigdlgkga;igdlgkgay;gdlgkgayk;dlgkgaykf;lgkgaykfi;gkgaykfid;kgaykfidf;g
aykfidfl;aykfidflf;ykfidflfa;kfidflfas;fidflfass;idflfassq;dflfassqs;flfassqsy;lfassqsyw;fassq
sywq;assqsywqm;ssqsywqmf;sqsywqmfa;qsywqmfay;sywqmfays;ywqmfaysp;wqmfaysp
i;qmfayspid;mfayspidf;fayspidft;ayspidftr;yspidftrs;spidftrsp;pidftrspp;idftrsppy;dftrsppy
s;ftrsppysi;trsppysif;rsppysifs;sppysifsa;ppysifsaf;pysifsafa;ysifsafag;sifsafagn;ifsafagnv;
fsafagnvy;safagnvyy;afagnvyyi;fagnvyyid;agnvyyidl;gnvyyidle;nvyyidlea;vyyidleal;yyid
leald;yidlealdk;idlealdkf;dlealdkfi;lealdkfid;ealdkfids;aldkfidsd;ldkfidsdl;dkfidsdln;kfids
dlnl;fidsdlnll;idsdlnllk;dsdlnllke;sdlnllken;dlnllkene;lnllkenet;nllkenetr;llkenetry;lkenetry
s;kenetrysd;enetrysdl;netrysdlk;etrysdlkk;trysdlkkl;rysdlkkls;ysdlkklsf;sdlkklsfk;dlkklsfk
d;lkklsfkdk;kklsfkdkf;klsfkdkfl;lsfkdkflk;sfkdkflke;fkdkflkea;kdkflkeaa;dkflkeaal;kflkea
aln;flkeaalnf;lkeaalnfi;keaalnfin;eaalnfinr;aalnfinra;alnfinras;lnfinrasv;nfinrasvd;finrasvd
e;inrasvdev;nrasvdevr;rasvdevrs;asvdevrsf;svdevrsfe;vdevrsfek;devrsfekf;evrsfekfk;vrsfe
kfkk;rsfekfkkk;sfekfkkks;fekfkkkss;ekfkkkssy;kfkkkssyw;fkkkssywl;kkkssywll;kkssywll
d;kssywlldf;ssywlldfa;sywlldfas;ywlldfasf;wlldfasfv;lldfasfva;ldfasfvaf;dfasfvafk;fasfvaf
ke;asfvafkey;sfvafkeyf;fvafkeyfl;vafkeyflk;afkeyflkd;fkeyflkds;keyflkdsr;eyflkdsrd;yflkd
srda;flkdsrdaf;lkdsrdafn;kdsrdafnv;dsrdafnvl;srdafnvlf;rdafnvlfd;dafnvlfdr;afnvlfdrg;fnvl
fdrgi;nvlfdrgil;vlfdrgili;lfdrgilir;fdrgilirn;drgilirne;rgilirnek;gilirnekd;ilirnekdl;lirnekdlf;ir
nekdlfk;rnekdlfkl;nekdlfklr;ekdlfklrn;kdlfklrni;dlfklrnil;lfklrnils;fklrnilsk;klrnilske;lrnilsk
ei;rnilskeik;nilskeikv;ilskeikvq;lskeikvqe;skeikvqev;keikvqevl;eikvqevlq;ikvqevlqy;kvqe
vlqyf;vqevlqyff;qevlqyfff;evlqyfffs;vlqyfffsq;lqyfffsqf;qyfffsqfq;yfffsqfqa;fffsqfqal;ffsqf
qalk;fsqfqalkr;sqfqalkry;qfqalkrya;fqalkryan;qalkryand;alkryandk;lkryandkg;kryandkgi;r

yandkgie;yandkgiel;andkgielv;ndkgielvm;dkgielvmn;kgielvmnl;gielvmnlp;ielvmnlpl;elv
mnlplf;lvmnlplfi;vmnlplfia;mnlplfiay;nlplfiayd;lplfiayds;plfiaydsa;lfiaydsad;fiaydsadv;ia
ydsadvw;aydsadvwa;ydsadvwah;dsadvwahq;sadvwahqk;advwahqky;dvwahqkyf;vwahqk
yfk;wahqkyfkl;ahqkyfklr;hqkyfklrf;qkyfklrfd;kyfklrfda;yfklrfdas;fklrfdask;klrfdaskd;lrfd
askdk;rfdaskdkv;fdaskdkva;daskdkvag;askdkvagi;skdkvagis;kdkvagisp;dkvagispd;kvagis
pdy;vagispdyf;agispdyfl;gispdyfle;ispdyfleq;spdyfleqe;pdyfleqeq;dyfleqeqa;yfleqeqaw;fl
eqeqawd;leqeqawds;eqeqawdsp;qeqawdspa;eqawdspay;qawdspays;awdspaysw;wdspays
wn;dspayswnv;spayswnvl;payswnvlk;ayswnvlkn;yswnvlknv;swnvlknvk;wnvlknvky;nvlk
nvkye;vlknvkyew;lknvkyeww;knvkyewwa;nvkyewwak;vkyewwakr;kyewwakri;yewwakr
ig;ewwakrigi;wwakrigil;wakrigilr;akrigilrk;krigilrky;rigilrkyi;igilrkyid;gilrkyidv;ilrkyidvi
;lrkyidvik;rkyidviki;kyidvikid;yidvikidh;idvikidhf;dvikidhfr;vikidhfrg;ikidhfrgf;kidhfrgfv
;idhfrgfvs;dhfrgfvst;hfrgfvstw;frgfvstwe;rgfvstwev;gfvstwevg;fvstwevgv;vstwevgvg;stw
evgvge;twevgvgea;wevgvgeay;evgvgeaya;vgvgeayaf;gvgeayafn;vgeayafng;geayafngl;ea
yafnglw;ayafnglwv;yafnglwvk;afnglwvks;fnglwvksp;nglwvkspg;glwvkspgr;lwvkspgrd;w
vkspgrdf;vkspgrdff;kspgrdffn;spgrdffnf;pgrdffnfi;grdffnfil;rdffnfiln;dffnfilne;ffnfilnei;fnf
ilneik;nfilneikd;filneikdl;ilneikdlk;lneikdlki;neikdlkiw;eikdlkiwv;ikdlkiwve;kdlkiwved;dl
kiwvedf;lkiwvedfe;kiwvedfen;iwvedfend;wvedfendl;vedfendle;edfendled;dfendledv;fend
ledvs;endledvsr;ndledvsrl;dledvsrlr;ledvsrlrd;edvsrlrdf;dvsrlrdff;vsrlrdffn;srlrdffnf;rlrdffn
fp;lrdffnfpg;rdffnfpgm;dffnfpgmr;ffnfpgmri;fnfpgmrim;nfpgmrimk;fpgmrimkl;pgmrimkl
a;gmrimklaf;mrimklafd;rimklafdf;imklafdfd;mklafdfds;klafdfdsd;lafdfdsdn;afdfdsdnq;fdf
dsdnqn;dfdsdnqnl;fdsdnqnlp;dsdnqnlph;sdnqnlphn;dnqnlphny;nqnlphnyi;qnlphnyik;nlph
nyikn;lphnyiknc;phnyiknci;hnyiknciv;nyikncivy;yikncivyt;ikncivytg;kncivytgi;ncivytgig;
civytgigd;ivytgigdn;vytgigdns;ytgigdnst;tgigdnsti;gigdnstir;igdnstire;gdnstiref;dnstirefv;n
stirefvn;stirefvns;tirefvnsl;irefvnsld;refvnsldd;efvnslddl;fvnslddlh;vnslddlhk;nslddlhkk;sl
ddlhkky;lddlhkkyi;ddlhkkyif;dlhkkyifd;lhkkyifdy;hkkyifdyl;kkyifdyln;kyifdylnt;yifdylnt
n;ifdylntne;fdylntned;dylntnedf;ylntnedfv;lntnedfvv;ntnedfvvw;tnedfvvwd;nedfvvwdm;e
dfvvwdmi;dfvvwdmir;fvvwdmirs;vvwdmirsa;vwdmirsam;wdmirsams;dmirsamss;mirsam
ssv;irsamssvs;rsamssvsd;samssvsds;amssvsdsv;mssvsdsvi;ssvsdsvii;svsdsviip;vsdsviipm;
sdsviipmq;dsviipmqd;sviipmqdy;viipmqdyi;iipmqdyin;ipmqdyinl;pmqdyinlg;mqdyinlgn;
qdyinlgne;dyinlgnef;yinlgnefr;inlgnefra;nlgnefran;lgnefrani;gnefranip;nefranipk;efranipk
n;franipknt;ranipkntl;anipkntln;nipkntlnn;ipkntlnnw;pkntlnnwi;kntlnnwif;ntlnnwifr;tlnnw
ifrl;lnnwifrll;nnwifrlle;nwifrlles;wifrllesd;ifrllesdl;frllesdld;rllesdlda;llesdldat;lesdldatl;es
dldatls;sdldatlsk;dldatlskn;ldatlskni;datlsknis;atlsknisf;tlsknisfi;lsknisfit;sknisfitr;knisfitrl;
nisfitrly;isfitrlyg;sfitrlygr;fitrlygrt;

10 mers:

mkykkirinl;kykkirinln;ykkirinlnl;kkirinlnlk;kirinlnlkr;irinlnlkrk;rinlnlkrks;inlnlkrksg;nlnl
krksgi;lnlkrksgil;nlkrksgill;lkrksgilln;krksgillni;rksgillnis;ksgillniss;sgillnissl;gillnisslp;ill
nisslps;llnisslpsk;lnisslpsky;nisslpskyg;isslpskygi;sslpskygig;slpskygigd;lpskygigdl;psky
gigdlg;skygigdlgk;kygigdlgkg;ygigdlgkga;gigdlgkgay;igdlgkgayk;gdlgkgaykf;dlgkgaykfi
;lgkgaykfid;gkgaykfidf;kgaykfidfl;gaykfidflf;aykfidflfa;ykfidflfas;kfidflfass;fidflfassq;idf
lfassqs;dflfassqsy;flfassqsyw;lfassqsywq;fassqsywqm;assqsywqmf;ssqsywqmfa;sqsywq
mfay;qsywqmfays;sywqmfaysp;ywqmfayspi;wqmfayspid;qmfayspidf;mfayspidft;fayspid
ftr;ayspidftrs;yspidftrsp;spidftrspp;pidftrsppy;idftrsppys;dftrsppysi;ftrsppysif;trsppysifs;rs
ppysifsa;sppysifsaf;ppysifsafa;pysifsafag;ysifsafagn;sifsafagnv;ifsafagnvy;fsafagnvyy;saf
agnvyyi;afagnvyyid;fagnvyyidl;agnvyyidle;gnvyyidlea;nvyyidleal;vyyidleald;yyidlealdk;

yidlealdkf;idlealdkfi;dlealdkfid;lealdkfids;ealdkfidsd;aldkfidsdl;ldkfidsdln;dkfidsdlnl;kfid
sdlnll;fidsdlnllk;idsdlnllke;dsdlnllken;sdlnllkene;dlnllkenet;lnllkenetr;nllkenetry;llkenetry
s;lkenetrysd;kenetrysdl;enetrysdlk;netrysdlkk;etrysdlkkl;trysdlkkls;rysdlkklsf;ysdlkklsfk;
sdlkklsfkd;dlkklsfkdk;lkklsfkdkf;kklsfkdkfl;klsfkdkflk;lsfkdkflke;sfkdkflkea;fkdkflkeaa;
kdkflkeaal;dkflkeaaln;kflkeaalnf;flkeaalnfi;lkeaalnfin;keaalnfinr;eaalnfinra;aalnfinras;aln
finrasv;lnfinrasvd;nfinrasvde;finrasvdev;inrasvdevr;nrasvdevrs;rasvdevrsf;asvdevrsfe;svd
evrsfek;vdevrsfekf;devrsfekfk;evrsfekfkk;vrsfekfkkk;rsfekfkkks;sfekfkkkss;fekfkkkssy;e
kfkkkssyw;kfkkkssywl;fkkkssywll;kkkssywlld;kkssywlldf;kssywlldfa;ssywlldfas;sywlldf
asf;ywlldfasfv;wlldfasfva;lldfasfvaf;ldfasfvafk;dfasfvafke;fasfvafkey;asfvafkeyf;sfvafkey
fl;fvafkeyflk;vafkeyflkd;afkeyflkds;fkeyflkdsr;keyflkdsrd;eyflkdsrda;yflkdsrdaf;flkdsrdaf
n;lkdsrdafnv;kdsrdafnvl;dsrdafnvlf;srdafnvlfd;rdafnvlfdr;dafnvlfdrg;afnvlfdrgi;fnvlfdrgil;
nvlfdrgili;vlfdrgilir;lfdrgilirn;fdrgilirne;drgilirnek;rgilirnekd;gilirnekdl;ilirnekdlf;lirnekdlf
k;irnekdlfkl;rnekdlfklr;nekdlfklrn;ekdlfklrni;kdlfklrnil;dlfklrnils;lfklrnilsk;fklrnilske;klrni
lskei;lrnilskeik;rnilskeikv;nilskeikvq;ilskeikvqe;lskeikvqev;skeikvqevl;keikvqevlq;eikvqe
vlqy;ikvqevlqyf;kvqevlqyff;vqevlqyfff;qevlqyffs;evlqyfffsq;vlqyfffsqf;lqyfffsqfq;qyfffs
qfqa;yfffsqfqal;fffsqfqalk;ffsqfqalkr;fsqfqalkry;sqfqalkrya;qfqalkryan;fqalkryand;qalkrya
ndk;alkryandkg;lkryandkgi;kryandkgie;ryandkgiel;yandkgielv;andkgielvm;ndkgielvmn;d
kgielvmnl;kgielvmnlp;gielvmnlpl;ielvmnlplf;elvmnlplfi;lvmnlplfia;vmnlplfiay;mnlplfiay
d;nlplfiayds;lplfiaydsa;plfiaydsad;lfiaydsadv;fiaydsadvw;iaydsadvwa;aydsadvwah;ydsad
vwahq;dsadvwahqk;sadvwahqky;advwahqkyf;dvwahqkyfk;vwahqkyfkl;wahqkyfklr;ahqk
yfklrf;hqkyfklrfd;qkyfklrfda;kyfklrfdas;yfklrfdask;fklrfdaskd;klrfdaskdk;lrfdaskdkv;rfdas
kdkva;fdaskdkvag;daskdkvagi;askdkvagis;skdkvagisp;kdkvagispd;dkvagispdy;kvagispdy
f;vagispdyfl;agispdyfle;gispdyfleq;ispdyfleqe;spdyfleqeq;pdyfleqeqa;dyfleqeqaw;yfleqeq
awd;fleqeqawds;leqeqawdsp;eqeqawdspa;qeqawdspay;eqawdspays;qawdspaysw;awdspa
yswn;wdspayswnv;dspayswnvl;spayswnvlk;payswnvlkn;ayswnvlknv;yswnvlknvk;swnvl
knvky;wnvlknvkye;nvlknvkyew;vlknvkyeww;lknvkyewwa;knvkyewwak;nvkyewwakr;v
kyewwakri;kyewwakrig;yewwakrigi;ewwakrigil;wwakrigilr;wakrigilrk;akrigilrky;krigilr
kyi;rigilrkyid;igilrkyidv;gilrkyidvi;ilrkyidvik;lrkyidviki;rkyidvikid;kyidvikidh;yidvikidhf;
idvikidhfr;dvikidhfrg;vikidhfrgf;ikidhfrgfv;kidhfrgfvs;idhfrgfvst;dhfrgfvstw;hfrgfvstwe;f
rgfvstwev;rgfvstwevg;gfvstwevgv;fvstwevgvg;vstwevgvge;stwevgvgea;twevgvgeay;wev
gvgeaya;evgvgeayaf;vgvgeayafn;gvgeayafng;vgeayafngl;geayafnglw;eayafnglwv;ayafngl
wvk;yafnglwvks;afnglwvksp;fnglwvkspg;nglwvkspgr;glwvkspgrd;lwvkspgrdf;wvkspgrdf
f;vkspgrdffn;kspgrdffnf;spgrdffnfi;pgrdffnfil;grdffnfiln;rdffnfilne;dffnfilnei;ffnfilneik;fnf
ilneikd;nfilneikdl;filneikdlk;ilneikdlki;lneikdlkiw;neikdlkiwv;eikdlkiwve;ikdlkiwved;kdl
kiwvedf;dlkiwvedfe;lkiwvedfen;kiwvedfend;iwvedfendl;wvedfendle;vedfendled;edfendle
dv;dfendledvs;fendledvsr;endledvsrl;ndledvsrlr;dledvsrlrd;ledvsrlrdf;edvsrlrdff;dvsrlrdffn
;vsrlrdffnf;srlrdffnfp;rlrdffnfpg;lrdffnfpgm;rdffnfpgmr;dffnfpgmri;ffnfpgmrim;fnfpgmri
mk;nfpgmrimkl;fpgmrimkla;pgmrimklaf;gmrimklafd;mrimklafdf;rimklafdfd;imklafdfds;
mklafdfdsd;klafdfdsdn;lafdfdsdnq;afdfdsdnqn;fdfdsdnqnl;dfdsdnqnlp;fdsdnqnlph;dsdnqn
lphn;sdnqnlphny;dnqnlphnyi;nqnlphnyik;qnlphnyikn;nlphnyiknc;lphnyiknci;phnyikncicv;
hnyikncivy;nyikncivyt;yikncivytg;ikncivytgi;kncivytgig;ncivytgigd;civytgigdn;ivytgigdn
s;vytgigdnst;ytgigdnsti;tgigdnstir;gigdnstire;igdnstiref;gdnstirefv;dnstirefvn;nstirefvns;sti
refvnsl;tirefvnsld;irefvnsldd;refvnslddl;efvnslddlh;fvnslddlhk;vnslddlhkk;nslddlhkky;sldd
lhkkyi;lddlhkkyif;ddlhkkyifd;dlhkkyifdy;lhkkyifdyl;hkkyifdyln;kkyifdylnt;kyifdylntn;yif
dylntne;ifdylntned;fdylntnedf;dylntnedfv;ylntnedfvv;lntnedfvvw;ntnedfvvwd;tnedfvvwd
m;nedfvvwdmi;edfvvwdmir;dfvvwdmirs;fvvwdmirsa;vvwdmirsam;vwdmirsams;wdmirs

amss;dmirsamssv;mirsamssvs;irsamssvsd;rsamssvsds;samssvsdsv;amssvsdsvi;mssvsdsvii
;ssvsdsviip;svsdsviipm;vsdsviipmq;sdsviipmqd;dsviipmqdy;sviipmqdyi;viipmqdyin;iipm
qdyinl;ipmqdyinlg;pmqdyinlgn;mqdyinlgne;qdyinlgnef;dyinlgnefr;yinlgnefra;inlgnefran;
nlgnefrani;lgnefranip;gnefranipk;nefranipkn;efranipknt;franipkntl;ranipkntln;anipkntlnn;
nipkntlnnw;ipkntlnnwi;pkntlnnwif;kntlnnwifr;ntlnnwifrl;tlnnwifrll;lnnwifrlle;nnwifrlles;n
wifrllesd;wifrllesdl;ifrllesdld;frllesdlda;rllesdldat;llesdldatl;lesdldatls;esdldatlsk;sdldatlsk
n;dldatlskni;ldatlsknis;datlsknisf;atlsknisfi;tlsknisfit;lsknisfitr;sknisfitrl;knisfitrly;nisfitrly
g;isfitrlygr;sfitrlygrt;

11 mers:
mkykkirinln;kykkirinlnl;ykkirinlnlk;kkirinlnlkr;kirinlnlkrk;irinlnlkrks;rinlnlkrksg;inlnlkr
ksgi;nlnlkrksgil;lnlkrksgill;nlkrksgilln;lkrksgillni;krksgillnis;rksgillniss;ksgillnissl;sgillni
sslp;gillnisslps;illnisslpsk;llnisslpsky;lnisslpskyg;nisslpskygi;isslpskygig;sslpskygigd;slps
kygigdl;lpskygigdlg;pskygigdlgk;skygigdlgkg;kygigdlgkga;ygigdlgkgay;gigdlgkgayk;igd
lgkgaykf;gdlgkgaykfi;dlgkgaykfid;lgkgaykfidf;gkgaykfidfl;kgaykfidflf;gaykfidflfa;aykfid
flfas;ykfidflfass;kfidflfassq;fidflfassqs;idflfassqsy;dflfassqsyw;flfassqsywq;lfassqsywqm;
fassqsywqmf;assqsywqmfa;ssqsywqmfay;sqsywqmfays;qsywqmfaysp;sywqmfayspi;ywq
mfayspid;wqmfayspidf;qmfayspidft;mfayspidftr;fayspidftrs;ayspidftrsp;yspidftrspp;spidft
rsppy;pidftrsppys;idftrsppysi;dftrsppysif;ftrsppysifs;trsppysifsa;rsppysifsaf;sppysifsafa;pp
ysifsafag;pysifsafagn;ysifsafagnv;sifsafagnvy;ifsafagnvyy;fsafagnvyyi;safagnvyyid;afagn
vyyidl;fagnvyyidle;agnvyyidlea;gnvyyidleal;nvyyidleald;vyyidlealdk;yyidlealdkf;yidleal
dkfi;idlealdkfid;dlealdkfids;lealdkfidsd;ealdkfidsdl;aldkfidsdln;ldkfidsdlnl;dkfidsdlnll;kfi
dsdlnllk;fidsdlnllke;idsdlnllken;dsdlnllkene;sdlnllkenet;dlnllkenetr;lnllkenetry;nllkenetrys
;llkenetrysd;lkenetrysdl;kenetrysdlk;enetrysdlkk;netrysdlkkl;etrysdlkkls;trysdlkklsf;rysdl
kklsfk;ysdlkklsfkd;sdlkklsfkdk;dlkklsfkdkf;lkklsfkdkfl;kklsfkdkflk;klsfkdkflke;lsfkdkflke
a;sfkdkflkeaa;fkdkflkeaal;kdkflkeaaln;dkflkeaalnf;kflkeaalnfi;flkeaalnfin;lkeaalnfinr;keaa
lnfinra;eaalnfinras;aalnfinrasv;alnfinrasvd;lnfinrasvde;nfinrasvdev;finrasvdevr;inrasvdevr
s;nrasvdevrsf;rasvdevrsfe;asvdevrsfek;svdevrsfekf;vdevrsfekfk;devrsfekfkk;evrsfekfkkk;
vrsfekfkkks;rsfekfkkkss;sfekfkkkssy;fekfkkkssyw;ekfkkkssywl;kfkkkssywll;fkkkssywlld;
kkkssywlldf;kkssywlldfa;kssywlldfas;ssywlldfasf;sywlldfasfv;ywlldfasfva;wlldfasfvaf;lld
fasfvafk;ldfasfvafke;dfasfvafkey;fasfvafkeyf;asfvafkeyfl;sfvafkeyflk;fvafkeyflkd;vafkeyf
lkds;afkeyflkdsr;fkeyflkdsrd;keyflkdsrda;eyflkdsrdaf;yflkdsrdafn;flkdsrdafnv;lkdsrdafnvl
;kdsrdafnvlf;dsrdafnvlfd;srdafnvlfdr;rdafnvlfdrg;dafnvlfdrgi;afnvlfdrgil;fnvlfdrgili;nvlfdr
gilir;vlfdrgilirn;lfdrgilirne;fdrgilirnek;drgilirnekd;rgilirnekdl;gilirnekdlf;ilirnekdlfk;lirnek
dlfkl;irnekdlfklr;rnekdlfklrn;nekdlfklrni;ekdlfklrnil;kdlfklrnils;dlfklrnilsk;lfklrnilske;fklrn
ilskei;klrnilskeik;lrnilskeikv;rnilskeikvq;nilskeikvqe;ilskeikvqev;lskeikvqevl;skeikvqevlq
;keikvqevlqy;eikvqevlqyf;ikvqevlqyff;kvqevlqyfff;vqevlqyfffs;qevlqyfffsq;evlqyfffsqf;vl
qyfffsqfq;lqyfffsqfqa;qyfffsqfqal;yfffsqfqalk;fffsqfqalkr;ffsqfqalkry;fsqfqalkrya;sqfqalkr
yan;qfqalkryand;fqalkryandk;qalkryandkg;alkryandkgi;lkryandkgie;kryandkgiel;ryandkgi
elv;yandkgielvm;andkgielvmn;ndkgielvmnl;dkgielvmnlp;kgielvmnlpl;gielvmnlplf;ielvmn
lplfi;elvmnlplfia;lvmnlplfiay;vmnlplfiayd;mnlplfiayds;nlplfiaydsa;lplfiaydsad;plfiaydsad
v;lfiaydsadvw;fiaydsadvwa;iaydsadvwah;aydsadvwahq;ydsadvwahqk;dsadvwahqky;sadv
wahqkyf;advwahqkyfk;dvwahqkyfkl;vwahqkyfklr;wahqkyfklrf;ahqkyfklrfd;hqkyfklrfda;
qkyfklrfdas;kyfklrfdask;yfklrfdaskd;fklrfdaskdk;klrfdaskdkv;lrfdaskdkva;rfdaskdkvag;fd
askdkvagi;daskdkvagis;askdkvagisp;skdkvagispd;kdkvagispdy;dkvagispdyf;kvagispdyfl;
vagispdyfle;agispdyfleq;gispdyfleqe;ispdyfleqeq;spdyfleqeqa;pdyfleqeqaw;dyfleqeqawd;

yfleqeqawds;fleqeqawdsp;leqeqawdspa;eqeqawdspay;qeqawdspays;eqawdspaysw;qawds
payswn;awdspayswnv;wdspayswnvl;dspayswnvlk;spayswnvlkn;payswnvlknv;ayswnvlkn
vk;yswnvlknvky;swnvlknvkye;wnvlknvkyew;nvlknvkyeww;vlknvkyewwa;lknvkyewwak
;knvkyewwakr;nvkyewwakri;vkyewwakrig;kyewwakrigi;yewwakrigil;ewwakrigilr;wwak
rigilrk;wakrigilrky;akrigilrkyi;krigilrkyid;rigilrkyidv;igilrkyidvi;gilrkyidvik;ilrkyidviki;lr
kyidvikid;rkyidvikidh;kyidvikidhf;yidvikidhfr;idvikidhfrg;dvikidhfrgf;vikidhfrgfv;ikidhfr
gfvs;kidhfrgfvst;idhfrgfvstw;dhfrgfvstwe;hfrgfvstwev;frgfvstwevg;rgfvstwevgv;gfvstwev
gvg;fvstwevgvge;vstwevgvgea;stwevgvgeay;twevgvgeaya;wevgvgeayaf;evgvgeayafn;vg
vgeayafng;gvgeayafngl;vgeayafnglw;geayafnglwv;eayafnglwvk;ayafnglwvks;yafnglwvk
sp;afnglwvkspg;fnglwvkspgr;nglwvkspgrd;glwvkspgrdf;lwvkspgrdff;wvkspgrdffn;vkspg
rdffnf;kspgrdffnfi;spgrdffnfil;pgrdffnfiln;grdffnfilne;rdffnfilnei;dffnfilneik;ffnfilneikd;fnf
ilneikdl;nfilneikdlk;filneikdlki;ilneikdlkiw;lneikdlkiwv;neikdlkiwve;eikdlkiwved;ikdlkiw
vedf;kdlkiwvedfe;dlkiwvedfen;lkiwvedfend;kiwvedfendl;iwvedfendle;wvedfendled;vedf
endledv;edfendledvs;dfendledvsr;fendledvsrl;endledvsrlr;ndledvsrlrd;dledvsrlrdf;ledvsrlr
dff;edvsrlrdffn;dvsrlrdffnf;vsrlrdffnfp;srlrdffnfpg;rlrdffnfpgm;lrdffnfpgmr;rdffnfpgmri;df
fnfpgmrim;ffnfpgmrimk;fnfpgmrimkl;nfpgmrimkla;fpgmrimklaf;pgmrimklafd;gmrimklaf
df;mrimklafdfd;rimklafdfds;imklafdfdsd;mklafdfdsdn;klafdfdsdnq;lafdfdsdnqn;afdfdsdnq
nl;fdfdsdnqnlp;dfdsdnqnlph;fdsdnqnlphn;dsdnqnlphny;sdnqnlphnyi;dnqnlphnyik;nqnlph
nyikn;qnlphnyiknc;nlphnyiknci;lphnyikneiv;phnyiknivy;hnyiknivyt;nyiknivytg;yiknci
vytgi;ikncivytgig;knivytgigd;ncivytgigdn;civytgigdns;ivytgigdnst;vytgigdnsti;ytgigdnsti
r;tgigdnstire;gigdnstiref;igdnstirefv;gdnstirefvn;dnstirefvns;nstirefvnsl;stirefvnsld;tirefvns
ldd;irefvnslddl;refvnslddlh;efvnslddlhk;fvnslddlhkk;vnslddlhkky;nslddlhkkyi;slddlhkkyif
;lddlhkkyifd;ddlhkkyifdy;dlhkkyifdyl;lhkkyifdyln;hkkyifdylnt;kkyifdylntn;kyifdylntne;yi
fdylntned;ifdylntnedf;fdylntnedfv;dylntnedfvv;ylntnedfvvw;lntnedfvvwd;ntnedfvvwdm;t
nedfvvwdmi;nedfvvwdmir;edfvvwdmirs;dfvvwdmirsa;fvvwdmirsam;vvwdmirsams;vwd
mirsamss;wdmirsamssv;dmirsamssvs;mirsamssvsd;irsamssvsds;rsamssvsdsv;samssvsdsv
i;amssvsdsvii;mssvsdsviip;ssvsdsviipm;svsdsviipmq;vsdsviipmqd;sdsviipmqdy;dsviipmq
dyi;sviipmqdyin;viipmqdyinl;iipmqdyinlg;ipmqdyinlgn;pmqdyinlgne;mqdyinlgnef;qdyin
lgnefr;dyinlgnefra;yinlgnefran;inlgnefrani;nlgnefranip;lgnefranipk;gnefranipkn;nefranipk
nt;efranipkntl;franipkntln;ranipkntlnn;anipkntlnnw;nipkntlnnwi;ipkntlnnwif;pkntlnnwifr;
kntlnnwifrl;ntlnnwifrll;tlnnwifrlle;lnnwifrlles;nnwifrllesd;nwifrllesdl;wifrllesdld;ifrllesdl
da;frllesdldat;rllesdldatl;llesdldatls;lesdldatlsk;esdldatlskn;sdldatlskni;dldatlsknis;ldatlskn
isf;datlsknisfi;atlsknisfit;tlsknisfitr;lsknisfitrl;sknisfitrly;knisfitrlyg;nisfitrlygr;isfitrlygrt;

13 mers:
mkykkirinlnlk;kykkirinlnlkr;ykkirinlnlkrk;kkirinlnlkrks;kirinlnlkrksg;irinlnlkrksgi;rinlnlk
rksgil;inlnlkrksgill;nlnlkrksgilln;lnlkrksgillni;nlkrksgillnis;lkrksgillniss;krksgillnissl;rksgi
llnisslp;ksgillnisslps;sgillnisslpsk;gillnisslpsky;illnisslpskyg;llnisslpskygi;lnisslpskygig;ni
sslpskygigd;isslpskygigdl;sslpskygigdlg;slpskygigdlgk;lpskygigdlgkg;pskygigdlgkga;sky
gigdlgkgay;kygigdlgkgayk;ygigdlgkgaykf;gigdlgkgaykfi;igdlgkgaykfid;gdlgkgaykfidf;dl
gkgaykfidfl;lgkgaykfidflf;gkgaykfidflfa;kgaykfidflfas;gaykfidflfass;aykfidflfassq;ykfidflf
assqs;kfidflfassqsy;fidflfassqsyw;idflfassqsywq;dflfassqsywqm;flfassqsywqmf;lfassqsyw
qmfa;fassqsywqmfay;assqsywqmfays;ssqsywqmfaysp;sqsywqmfayspi;qsywqmfayspid;sy
wqmfayspidf;ywqmfayspidft;wqmfayspidftr;qmfayspidftrs;mfayspidftrsp;fayspidftrspp;a
yspidftrsppy;yspidftrsppys;spidftrsppysi;pidftrsppysif;idftrsppysifs;dftrsppysifsa;ftrsppysi
fsaf;trsppysifsafa;rsppysifsafag;sppysifsafagn;ppysifsafagnv;pysifsafagnvy;ysifsafagnvyy

;sifsafagnvyyi;ifsafagnvyyid;fsafagnvyyidl;safagnvyyidle;afagnvyyidlea;fagnvyyidleal;a
gnvyyidleald;gnvyyidlealdk;nvyyidlealdkf;vyyidlealdkfi;yyidlealdkfid;yidlealdkfids;idlea
ldkfidsd;dlealdkfidsdl;lealdkfidsdln;ealdkfidsdlnl;aldkfidsdlnll;ldkfidsdlnllk;dkfidsdlnllk
e;kfidsdlnllken;fidsdlnllkene;idsdlnllkenet;dsdlnllkenetr;sdlnllkenetry;dlnllkenetrys;lnllke
netrysd;nllkenetrysdl;llkenetrysdlk;lkenetrysdlkk;kenetrysdlkkl;enetrysdlkkls;netrysdlkkl
sf;etrysdlkklsfk;trysdlkklsfkd;rysdlkklsfkdk;ysdlkklsfkdkf;sdlkklsfkdkfl;dlkklsfkdkflk;lk
klsfkdkflke;kklsfkdkflkea;klsfkdkflkeaa;lsfkdkflkeaal;sfkdkflkeaaln;fkdkflkeaalnf;kdkflk
eaalnfi;dkflkeaalnfin;kflkeaalnfinr;flkeaalnfinra;lkeaalnfinras;keaalnfinrasv;eaalnfinrasvd
;aalnfinrasvde;alnfinrasvdev;lnfinrasvdevr;nfinrasvdevrs;finrasvdevrsf;inrasvdevrsfe;nras
vdevrsfek;rasvdevrsfekf;asvdevrsfekfk;svdevrsfekfkk;vdevrsfekfkkk;devrsfekfkkks;evrsf
ekfkkkss;vrsfekfkkkssy;rsfekfkkkssyw;sfekfkkkssywl;fekfkkkssywll;ekfkkkssywlld;kfkk
kssywlldf;fkkkssywlldfa;kkkssywlldfas;kkssywlldfasf;kssywlldfasfv;ssywlldfasfva;sywll
dfasfvaf;ywlldfasfvafk;wlldfasfvafke;lldfasfvafkey;ldfasfvafkeyf;dfasfvafkeyfl;fasfvafke
yflk;asfvafkeyflkd;sfvafkeyflkds;fvafkeyflkdsr;vafkeyflkdsrd;afkeyflkdsrda;fkeyflkdsrda
f;keyflkdsrdafn;eyflkdsrdafnv;yflkdsrdafnvl;flkdsrdafnvlf;lkdsrdafnvlfd;kdsrdafnvlfdr;ds
rdafnvlfdrg;srdafnvlfdrgi;rdafnvlfdrgil;dafnvlfdrgili;afnvlfdrgilir;fnvlfdrgilirn;nvlfdrgilir
ne;vlfdrgilirnek;lfdrgilirnekd;fdrgilirnekdl;drgilirnekdlf;rgilirnekdlfk;gilirnekdlfkl;ilirnek
dlfklr;lirnekdlfklrn;irnekdlfklrni;rnekdlfklrnil;nekdlfklrnils;ekdlfklrnilsk;kdlfklrnilske;dlf
klrnilskei;lfklrnilskeik;fklrnilskeikv;klrnilskeikvq;lrnilskeikvqe;rnilskeikvqev;nilskeikvqe
vl;ilskeikvqevlq;lskeikvqevlqy;skeikvqevlqyf;keikvqevlqyff;eikvqevlqyfff;ikvqevlqyffs;
kvqevlqyfffsq;vqevlqyfffsqf;qevlqyfffsqfq;evlqyfffsqfqa;vlqyfffsqfqal;lqyfffsqfqalk;qyfff
sqfqalkr;yfffsqfqalkry;fffsqfqalkrya;ffsqfqalkryan;fsqfqalkryand;sqfqalkryandk;qfqalkrya
ndkg;fqalkryandkgi;qalkryandkgie;alkryandkgiel;lkryandkgielv;kryandkgielvm;ryandkgi
elvmn;yandkgielvmnl;andkgielvmnlp;ndkgielvmnlpl;dkgielvmnlplf;kgielvmnlplfi;gielvm
nlplfia;ielvmnlplfiay;elvmnlplfiayd;lvmnlplfiayds;vmnlplfiaydsa;mnlplfiaydsad;nlplfiayd
sadv;lplfiaydsadvw;plfiaydsadvwa;lfiaydsadvwah;fiaydsadvwahq;iaydsadvwahqk;aydsad
vwahqky;ydsadvwahqkyf;dsadvwahqkyfk;sadvwahqkyfkl;advwahqkyfklr;dvwahqkyfklrf
;vwahqkyfklrfd;wahqkyfklrfda;ahqkyfklrfdas;hqkyfklrfdask;qkyfklrfdaskd;kyfklrfdaskdk
;yfklrfdaskdkv;fklrfdaskdkva;klrfdaskdkvag;lrfdaskdkvagi;rfdaskdkvagis;fdaskdkvagisp;
daskdkvagispd;askdkvagispdy;skdkvagispdyf;kdkvagispdyfl;dkvagispdyfle;kvagispdyfle
q;vagispdyfleqe;agispdyfleqeq;gispdyfleqeqa;ispdyfleqeqaw;spdyfleqeqawd;pdyfleqeqa
wds;dyfleqeqawdsp;yfleqeqawdspa;fleqeqawdspay;leqeqawdspays;eqeqawdspaysw;qeqa
wdspayswn;eqawdspayswnv;qawdspayswnvl;awdspayswnvlk;wdspayswnvlkn;dspayswn
vlknv;spayswnvlknvk;payswnvlknvky;ayswnvlknvkye;yswnvlknvkyew;swnvlknvkyeww
;wnvlknvkyewwa;nvlknvkyewwak;vlknvkyewwakr;lknvkyewwakri;knvkyewwakrig;nvk
yewwakrigi;vkyewwakrigil;kyewwakrigilr;yewwakrigilrk;ewwakrigilrky;wwakrigilrkyi;
wakrigilrkyid;akrigilrkyidv;krigilrkyidvi;rigilrkyidvik;igilrkyidviki;gilrkyidvikid;ilrkyidv
ikidh;lrkyidvikidhf;rkyidvikidhfr;kyidvikidhfrg;yidvikidhfrgf;idvikidhfrgfv;dvikidhfrgfvs
;vikidhfrgfvst;ikidhfrgfvstw;kidhfrgfvstwe;idhfrgfvstwev;dhfrgfvstwevg;hfrgfvstwevgv;f
rgfvstwevgvg;rgfvstwevgvge;gfvstwevgvgea;fvstwevgvgeay;vstwevgvgeaya;stwevgvgea
yaf;twevgvgeayafn;wevgvgeayafng;evgvgeayafngl;vgvgeayafnglw;gvgeayafnglwv;vgeay
afnglwvk;geayafnglwvks;eayafnglwvksp;ayafnglwvkspg;yafnglwvkspgr;afnglwvkspgrd;f
nglwvkspgrdf;nglwvkspgrdff;glwvkspgrdffn;lwvkspgrdffnf;wvkspgrdffnfi;vkspgrdffnfil;
kspgrdffnfiln;spgrdffnfilne;pgrdffnfilnei;grdffnfilneik;rdffnfilneikd;dffnfilneikdl;ffnfilnei
kdlk;fnfilneikdlki;nfilneikdlkiw;filneikdlkiwv;ilneikdlkiwve;lneikdlkiwved;neikdlkiwved
f;eikdlkiwvedfe;ikdlkiwvedfen;kdlkiwvedfend;dlkiwvedfendl;lkiwvedfendle;kiwvedfendl

ed;iwvedfendledv;wvedfendledvs;vedfendledvsr;edfendledvsrl;dfendledvsrlr;fendledvsrlr
d;endledvsrlrdf;ndledvsrlrdff;dledvsrlrdffn;ledvsrlrdffnf;edvsrlrdffnfp;dvsrlrdffnfpg;vsrlr
dffnfpgm;srlrdffnfpgmr;rlrdffnfpgmri;lrdffnfpgmrim;rdffnfpgmrimk;dffnfpgmrimkl;ffnfp
gmrimkla;fnfpgmrimklaf;nfpgmrimklafd;fpgmrimklafdf;pgmrimklafdfd;gmrimklafdfds;
mrimklafdfdsd;rimklafdfdsdn;imklafdfdsdnq;mklafdfdsdnqn;klafdfdsdnqnl;lafdfdsdnqnlp
;afdfdsdnqnlph;fdfdsdnqnlphn;dfdsdnqnlphny;fdsdnqnlphnyi;dsdnqnlphnyik;sdnqnlphnyi
kn;dnqnlphnyiknc;nqnlphnyiknci;qnlphnyiknciv;nlphnyikncivy;lphnyikncivyt;phnyiknci
vytg;hnyikncivytgi;nyikncivytgig;yikncivytgigd;ikncivytgigdn;kncivytgigdns;ncivytgigd
nst;civytgigdnsti;ivytgigdnstir;vytgigdnstire;ytgigdnstiref;tgigdnstirefv;gigdnstirefvn;igd
nstirefvns;gdnstirefvnsl;dnstirefvnsld;nstirefvnsldd;stirefvnslddl;tirefvnslddlh;irefvnslddl
hk;refvnslddlhkk;efvnslddlhkky;fvnslddlhkkyi;vnslddlhkkyif;nslddlhkkyifd;slddlhkkyifd
y;lddlhkkyifdyl;ddlhkkyifdyln;dlhkkyifdylnt;lhkkyifdylntn;hkkyifdylntne;kkyifdylntned;
kyifdylntnedf;yifdylntnedfv;ifdylntnedfvv;fdylntnedfvvw;dylntnedfvvwd;ylntnedfvvwdm
;lntnedfvvwdmi;ntnedfvvwdmir;tnedfvvwdmirs;nedfvvwdmirsa;edfvvwdmirsam;dfvvwd
mirsams;fvvwdmirsamss;vvwdmirsamssv;vwdmirsamssvs;wdmirsamssvsd;dmirsamssvs
ds;mirsamssvsdsv;irsamssvsdsvi;rsamssvsdsvii;samssvsdsviip;amssvsdsviipm;mssvsdsvii
pmq;ssvsdsviipmqd;svsdsviipmqdy;vsdsviipmqdyi;sdsviipmqdyin;dsviipmqdyinl;sviipm
qdyinlg;viipmqdyinlgn;iipmqdyinlgne;ipmqdyinlgnef;pmqdyinlgnefr;mqdyinlgnefra;qdyi
nlgnefran;dyinlgnefrani;yinlgnefranip;inlgnefranipk;nlgnefranipkn;lgnefranipknt;gnefrani
pkntl;nefranipkntln;efranipkntlnn;franipkntlnnw;ranipkntlnnwi;anipkntlnnwif;nipkntlnnw
ifr;ipkntlnnwifrl;pkntlnnwifrll;kntlnnwifrlle;ntlnnwifrlles;tlnnwifrllesd;lnnwifrllesdl;nnwi
frllesdld;nwifrllesdlda;wifrllesdldat;ifrllesdldatl;frllesdldatls;rllesdldatlsk;llesdldatlskn;les
dldatlskni;esdldatlsknis;sdldatlsknisf;dldatlsknisfi;ldatlsknisfit;datlsknisfitr;atlsknisfitrl;tl
sknisfitrly;lsknisfitrlyg;sknisfitrlygr;knisfitrlygrt;

14 mers:

mkykkirinlnlkr;kykkirinlnlkrk;ykkirinlnlkrks;kkirinlnlkrksg;kirinlnlkrksgi;irinlnlkrksgil;r
inlnlkrksgill;inlnlkrksgilln;nlnlkrksgillni;lnlkrksgillnis;nlkrksgillniss;lkrksgillnissl;krksgil
lnisslp;rksgillnisslps;ksgillnisslpsk;sgillnisslpsky;gillnisslpskyg;illnisslpskygi;llnisslpsky
gig;lnisslpskygigd;nisslpskygigdl;isslpskygigdlg;sslpskygigdlgk;slpskygigdlgkg;lpskygig
dlgkga;pskygigdlgkgay;skygigdlgkgayk;kygigdlgkgaykf;ygigdlgkgaykfi;gigdlgkgaykfid;i
gdlgkgaykfidf;gdlgkgaykfidfl;dlgkgaykfidflf;lgkgaykfidflfa;gkgaykfidflfas;kgaykfidflfas
s;gaykfidflfassq;aykfidflfassqs;ykfidflfassqsy;kfidflfassqsyw;fidflfassqsywq;idflfassqsyw
qm;dflfassqsywqmf;flfassqsywqmfa;lfassqsywqmfay;fassqsywqmfays;assqsywqmfaysp;s
sqsywqmfayspi;sqsywqmfayspid;qsywqmfayspidf;sywqmfayspidft;ywqmfayspidftr;wqm
fayspidftrs;qmfayspidftrsp;mfayspidftrspp;fayspidftrsppy;ayspidftrsppys;yspidftrsppysi;s
pidftrsppysif;pidftrsppysifs;idftrsppysifsa;dftrsppysifsaf;ftrsppysifsafa;trsppysifsafag;rspp
ysifsafagn;sppysifsafagnv;ppysifsafagnvy;pysifsafagnvyy;ysifsafagnvyyi;sifsafagnvyyid;
ifsafagnvyyidl;fsafagnvyyidle;safagnvyyidlea;afagnvyyidleal;fagnvyyidleald;agnvyyidlea
ldk;gnvyyidlealdkf;nvyyidlealdkfi;vyyidlealdkfid;yyidlealdkfids;yidlealdkfidsd;idlealdkfi
dsdl;dlealdkfidsdln;lealdkfidsdlnl;ealdkfidsdlnll;aldkfidsdlnllk;ldkfidsdlnllke;dkfidsdlnllk
en;kfidsdlnllkene;fidsdlnllkenet;idsdlnllkenetr;dsdlnllkenetry;sdlnllkenetrys;dlnllkenetrys
d;lnllkenetrysdl;nllkenetrysdlk;llkenetrysdlkk;lkenetrysdlkkl;kenetrysdlkkls;enetrysdlkkls
f;netrysdlkklsfk;etrysdlkklsfkd;trysdlkklsfkdk;rysdlkklsfkdkf;ysdlkklsfkdkfl;sdlkklsfkdkf
lk;dlkklsfkdkflke;lkklsfkdkflkea;kklsfkdkflkeaa;klsfkdkflkeaal;lsfkdkflkeaaln;sfkdkflkea
alnf;fkdkflkeaalnfi;kdkflkeaalnfin;dkflkeaalnfinr;kflkeaalnfinra;flkeaalnfinras;lkeaalnfinr

asv;keaalnfinrasvd;eaalnfinrasvde;aalnfinrasvdev;alnfinrasvdevr;lnfinrasvdevrs;nfinrasvd
evrsf;finrasvdevrsfe;inrasvdevrsfek;nrasvdevrsfekf;rasvdevrsfekfk;asvdevrsfekfkk;svdevr
sfekfkkk;vdevrsfekfkkks;devrsfekfkkkss;evrsfekfkkkssy;vrsfekfkkkssyw;rsfekfkkkssywl;
sfekfkkkssywll;fekfkkkssywlld;ekfkkkssywlldf;kfkkkssywlldfa;fkkkssywlldfas;kkkssywl
ldfasf;kkssywlldfasfv;kssywlldfasfva;ssywlldfasfvaf;sywlldfasfvafk;ywlldfasfvafke;wlldf
asfvafkey;lldfasfvafkeyf;ldfasfvafkeyfl;dfasfvafkeyflk;fasfvafkeyflkd;asfvafkeyflkds;sfva
fkeyflkdsr;fvafkeyflkdsrd;vafkeyflkdsrda;afkeyflkdsrdaf;fkeyflkdsrdafn;keyflkdsrdafnv;e
yflkdsrdafnvl;yflkdsrdafnvlf;flkdsrdafnvlfd;lkdsrdafnvlfdr;kdsrdafnvlfdrg;dsrdafnvlfdrgi;
srdafnvlfdrgil;rdafnvlfdrgili;dafnvlfdrgilir;afnvlfdrgilirn;fnvlfdrgilirne;nvlfdrgilirnek;vlfd
rgilirnekd;lfdrgilirnekdl;fdrgilirnekdlf;drgilirnekdlfk;rgilirnekdlfkl;gilirnekdlfklr;ilirnekdl
fklrn;lirnekdlfklrni;irnekdlfklrnil;rnekdlfklrnils;nekdlfklrnilsk;ekdlfklrnilske;kdlfklrnilske
i;dlfklrnilskeik;lfklrnilskeikv;fklrnilskeikvq;klrnilskeikvqe;lrnilskeikvqev;rnilskeikvqevl;
nilskeikvqevlq;ilskeikvqevlqy;lskeikvqevlqyf;skeikvqevlqyff;keikvqevlqyfff;eikvqevlqyf
ffs;ikvqevlqyfffsq;kvqevlqyfffsqf;vqevlqyfffsqfq;qevlqyfffsqfqa;evlqyfffsqfqal;vlqyfffsq
fqalk;lqyfffsqfqalkr;qyfffsqfqalkry;yfffsqfqalkrya;fffsqfqalkryan;ffsqfqalkryand;fsqfqalkr
yandk;sqfqalkryandkg;qfqalkryandkgi;fqalkryandkgie;qalkryandkgiel;alkryandkgielv;lkr
yandkgielvm;kryandkgielvmn;ryandkgielvmnl;yandkgielvmnlp;andkgielvmnlpl;ndkgielv
mnlplf;dkgielvmnlplfi;kgielvmnlplfia;gielvmnlplfiay;ielvmnlplfiayd;elvmnlplfiayds;lvmn
lplfiaydsa;vmnlplfiaydsad;mnlplfiaydsadv;nlplfiaydsadvw;lplfiaydsadvwa;plfiaydsadvwa
h;lfiaydsadvwahq;fiaydsadvwahqk;iaydsadvwahqky;aydsadvwahqkyf;ydsadvwahqkyfk;d
sadvwahqkyfkl;sadvwahqkyfklr;advwahqkyfklrf;dvwahqkyfklrfd;vwahqkyfklrfda;wahqk
yfklrfdas;ahqkyfklrfdask;hqkyfklrfdaskd;qkyfklrfdaskdk;kyfklrfdaskdkv;yfklrfdaskdkva;
fklrfdaskdkvag;klrfdaskdkvagi;lrfdaskdkvagis;rfdaskdkvagisp;fdaskdkvagispd;daskdkvag
ispdy;askdkvagispdyf;skdkvagispdyfl;kdkvagispdyfle;dkvagispdyfleq;kvagispdyfleqe;va
gispdyfleqeq;agispdyfleqeqa;gispdyfleqeqaw;ispdyfleqeqawd;spdyfleqeqawds;pdyfleqeq
awdsp;dyfleqeqawdspa;yfleqeqawdspay;fleqeqawdspays;leqeqawdspaysw;eqeqawdspays
wn;qeqawdspayswnv;eqawdspayswnvl;qawdspayswnvlk;awdspayswnvlkn;wdspayswnvl
knv;dspayswnvlknvk;spayswnvlknvky;payswnvlknvkye;ayswnvlknvkyew;yswnvlknvkye
ww;swnvlknvkyewwa;wnvlknvkyewwak;nvlknvkyewwakr;vlknvkyewwakri;lknvkyeww
akrig;knvkyewwakrigi;nvkyewwakrigil;vkyewwakrigilr;kyewwakrigilrk;yewwakrigilrky;
ewwakrigilrkyi;wwakrigilrkyid;wakrigilrkyidv;akrigilrkyidvi;krigilrkyidvik;rigilrkyidviki
;igilrkyidvikid;gilrkyidvikidh;ilrkyidvikidhf;lrkyidvikidhfr;rkyidvikidhfrg;kyidvikidhfrgf;
yidvikidhfrgfv;idvikidhfrgfvs;dvikidhfrgfvst;vikidhfrgfvstw;ikidhfrgfvstwe;kidhfrgfvstw
ev;idhfrgfvstwevg;dhfrgfvstwevgv;hfrgfvstwevgvg;frgfvstwevgvge;rgfvstwevgvgea;gfvs
twevgvgeay;fvstwevgvgeaya;vstwevgvgeayaf;stwevgvgeayafn;twevgvgeayafng;wevgvge
ayafngl;evgvgeayafnglw;vgvgeayafnglwv;gvgeayafnglwvk;vgeayafnglwvks;geayafnglw
vksp;eayafnglwvkspg;ayafnglwvkspgr;yafnglwvkspgrd;afnglwvkspgrdf;fnglwvkspgrdff;
nglwvkspgrdffn;glwvkspgrdffnf;lwvkspgrdffnfi;wvkspgrdffnfil;vkspgrdffnfiln;kspgrdffnf
ilne;spgrdffnfilnei;pgrdffnfilneik;grdffnfilneikd;rdffnfilneikdl;dffnfilneikdlk;ffnfilneikdlk
i;fnfilneikdlkiw;nfilneikdlkiwv;filneikdlkiwve;ilneikdlkiwved;lneikdlkiwvedf;neikdlkiwv
edfe;eikdlkiwvedfen;ikdlkiwvedfend;kdlkiwvedfendl;dlkiwvedfendle;lkiwvedfendled;ki
wvedfendledv;iwvedfendledvs;wvedfendledvsr;vedfendledvsrl;edfendledvsrlr;dfendledvs
rlrd;fendledvsrlrdf;endledvsrlrdff;ndledvsrlrdffn;dledvsrlrdffnf;ledvsrlrdffnfp;edvsrlrdffn
fpg;dvsrlrdffnfpgm;vsrlrdffnfpgmr;srlrdffnfpgmri;rlrdffnfpgmrim;lrdffnfpgmrimk;rdffnfp
gmrimkl;dffnfpgmrimkla;ffnfpgmrimklaf;fnfpgmrimklafd;nfpgmrimklafdf;fpgmrimklafd
fd;pgmrimklafdfds;gmrimklafdfdsd;mrimklafdfdsdn;rimklafdfdsdnq;imklafdfdsdnqn;mkl

afdfdsdnqnl;klafdfdsdnqnlp;lafdfdsdnqnlph;afdfdsdnqnlphn;fdfdsdnqnlphny;dfdsdnqnlph
nyi;fdsdnqnlphnyik;dsdnqnlphnyikn;sdnqnlphnyiknc;dnqnlphnyiknci;nqnlphnyiknciv;qnl
phnyikncivy;nlphnyikncivyt;lphnyikncivytg;phnyikncivytgi;hnyikncivytgig;nyikncivytgi
gd;yikncivytgigdn;ikncivytgigdns;kncivytgigdnst;ncivytgigdnsti;civytgigdnstir;ivytgigdn
stire;vytgigdnstiref;ytgigdnstirefv;tgigdnstirefvn;gigdnstirefvns;igdnstirefvnsl;gdnstirefvn
sld;dnstirefvnsldd;nstirefvnslddl;stirefvnslddlh;tirefvnslddlhk;irefvnslddlhkk;refvnslddlh
kky;efvnslddlhkkyi;fvnslddlhkkyif;vnslddlhkkyifd;nslddlhkkyifdy;slddlhkkyifdyl;lddlhk
kyifdyln;ddlhkkyifdylnt;dlhkkyifdylntn;lhkkyifdylntne;hkkyifdylntned;kkyifdylntnedf;ky
ifdylntnedfv;yifdylntnedfvv;ifdylntnedfvvw;fdylntnedfvvwd;dylntnedfvvwdm;ylntnedfvv
wdmi;lntnedfvvwdmir;ntnedfvvwdmirs;tnedfvvwdmirsa;nedfvvwdmirsam;edfvvwdmirsa
ms;dfvvwdmirsamss;fvvwdmirsamssv;vvwdmirsamssvs;vwdmirsamssvsd;wdmirsamssvs
ds;dmirsamssvsdsv;mirsamssvsdsvi;irsamssvsdsvii;rsamssvsdsviip;samssvsdsviipm;amss
vsdsviipmq;mssvsdsviipmqd;ssvsdsviipmqdy;svsdsviipmqdyi;vsdsviipmqdyin;sdsviipmq
dyinl;dsviipmqdyinlg;sviipmqdyinlgn;viipmqdyinlgne;iipmqdyinlgnef;ipmqdyinlgnefr;p
mqdyinlgnefra;mqdyinlgnefran;qdyinlgnefrani;dyinlgnefranip;yinlgnefranipk;inlgnefrani
pkn;nlgnefranipknt;lgnefranipkntl;gnefranipkntln;nefranipkntlnn;efranipkntlnnw;franipkn
tlnnwi;ranipkntlnnwif;anipkntlnnwifr;nipkntlnnwifrl;ipkntlnnwifrll;pkntlnnwifrlle;kntlnn
wifrlles;ntlnnwifrllesd;tlnnwifrllesdl;lnnwifrllesdld;nnwifrllesdlda;nwifrllesdldat;wifrlles
dldatl;ifrllesdldatls;frllesdldatlsk;rllesdldatlskn;llesdldatlskni;lesdldatlsknis;esdldatlsknisf
;sdldatlsknisfi;dldatlsknisfit;ldatlsknisfitr;datlsknisfitrl;atlsknisfitrly;tlsknisfitrlyg;lsknisfit
rlygr;sknisfitrlygrt;

15 mers:
mkykkirinlnlkrk;kykkirinlnlkrks;ykkirinlnlkrksg;kkirinlnlkrksgi;kirinlnlkrksgil;irinlnlkrk
sgill;rinlnlkrksgilln;inlnlkrksgillni;nlnlkrksgillnis;lnlkrksgillniss;nlkrksgillnissl;lkrksgilln
isslp;krksgillnisslps;rksgillnisslpsk;ksgillnisslpsky;sgillnisslpskyg;gillnisslpskygi;illnisslp
skygig;llnisslpskygigd;lnisslpskygigdl;nisslpskygigdlg;isslpskygigdlgk;sslpskygigdlgkg;s
lpskygigdlgkga;lpskygigdlgkgay;pskygigdlgkgayk;skygigdlgkgaykf;kygigdlgkgaykfi;ygi
gdlgkgaykfid;gigdlgkgaykfidf;igdlgkgaykfidfl;gdlgkgaykfidflf;dlgkgaykfidflfa;lgkgaykfi
dflfas;gkgaykfidflfass;kgaykfidflfassq;gaykfidflfassqs;aykfidflfassqsy;ykfidflfassqsyw;kf
idflfassqsywq;fidflfassqsywqm;idflfassqsywqmf;dflfassqsywqmfa;flfassqsywqmfay;lfass
qsywqmfays;fassqsywqmfaysp;assqsywqmfayspi;ssqsywqmfayspid;sqsywqmfayspidf;qs
ywqmfayspidft;sywqmfayspidftr;ywqmfayspidftrs;wqmfayspidftrsp;qmfayspidftrspp;mfa
yspidftrsppy;fayspidftrsppys;ayspidftrsppysi;yspidftrsppysif;spidftrsppysifs;pidftrsppysif
sa;idftrsppysifsaf;dftrsppysifsafa;ftrsppysifsafag;trsppysifsafagn;rsppysifsafagnv;sppysifs
afagnvy;ppysifsafagnvyy;pysifsafagnvyyi;ysifsafagnvyyid;sifsafagnvyyidl;ifsafagnvyyidl
e;fsafagnvyyidlea;safagnvyyidleal;afagnvyyidleald;fagnvyyidlealdk;agnvyyidlealdkf;gnv
yyidlealdkfi;nvyyidlealdkfid;vyyidlealdkfids;yyidlealdkfidsd;yidlealdkfidsdl;idlealdkfids
dln;dlealdkfidsdlnl;lealdkfidsdlnll;ealdkfidsdlnllk;aldkfidsdlnllke;ldkfidsdlnllken;dkfidsd
lnllkene;kfidsdlnllkenet;fidsdlnllkenetr;idsdlnllkenetry;dsdlnllkenetrys;sdlnllkenetrysd;dl
nllkenetrysdl;lnllkenetrysdlk;nllkenetrysdlkk;llkenetrysdlkkl;lkenetrysdlkkls;kenetrysdlk
klsf;enetrysdlkklsfk;netrysdlkklsfkd;etrysdlkklsfkdk;trysdlkklsfkdkf;rysdlkklsfkdkfl;ysdl
kklsfkdkflk;sdlkklsfkdkflke;dlkklsfkdkflkea;lkklsfkdkflkeaa;kklsfkdkflkeaal;klsfkdkflkea
aln;lsfkdkflkeaalnf;sfkdkflkeaalnfi;fkdkflkeaalnfin;kdkflkeaalnfinr;dkflkeaalnfinra;kflkea
alnfinras;flkeaalnfinrasv;lkeaalnfinrasvd;keaalnfinrasvde;eaalnfinrasvdev;aalnfinrasvdevr
;alnfinrasvdevrs;lnfinrasvdevrsf;nfinrasvdevrsfe;finrasvdevrsfek;inrasvdevrsfekf;nrasvde

vrsfekfk;rasvdevrsfekfkk;asvdevrsfekfkkk;svdevrsfekfkkks;vdevrsfekfkkkss;devrsfekfkk
kssy;evrsfekfkkkssyw;vrsfekfkkkssywl;rsfekfkkkssywll;sfekfkkkssywlld;fekfkkkssywlldf
;ekfkkkssywlldfa;kfkkkssywlldfas;fkkkssywlldfasf;kkkssywlldfasfv;kkssywlldfasfva;kss
ywlldfasfvaf;ssywlldfasfvafk;sywlldfasfvafke;ywlldfasfvafkey;wlldfasfvafkeyf;lldfasfvaf
keyfl;ldfasfvafkeyflk;dfasfvafkeyflkd;fasfvafkeyflkds;asfvafkeyflkdsr;sfvafkeyflkdsrd;fv
afkeyflkdsrda;vafkeyflkdsrdaf;afkeyflkdsrdafn;fkeyflkdsrdafnv;keyflkdsrdafnvl;eyflkdsr
dafnvlf;yflkdsrdafnvlfd;flkdsrdafnvlfdr;lkdsrdafnvlfdrg;kdsrdafnvlfdrgi;dsrdafnvlfdrgil;sr
dafnvlfdrgili;rdafnvlfdrgilir;dafnvlfdrgilirn;afnvlfdrgilirne;fnvlfdrgilirnek;nvlfdrgilirnekd
;vlfdrgilirnekdl;lfdrgilirnekdlf;fdrgilirnekdlfk;drgilirnekdlfkl;rgilirnekdlfklr;gilirnekdlfklr
n;ilirnekdlfklrni;lirnekdlfklrnil;irnekdlfklrnils;rnekdlfklrnilsk;nekdlfklrnilske;ekdlfklrnils
kei;kdlfklrnilskeik;dlfklrnilskeikv;lfklrnilskeikvq;fklrnilskeikvqe;klrnilskeikvqev;lrnilske
ikvqevl;rnilskeikvqevlq;nilskeikvqevlqy;ilskeikvqevlqyf;lskeikvqevlqyff;skeikvqevlqyfff
;keikvqevlqyfffs;eikvqevlqyfffsq;ikvqevlqyfffsqf;kvqevlqyfffsqfq;vqevlqyfffsqfqa;qevlq
yfffsqfqal;evlqyfffsqfqalk;vlqyfffsqfqalkr;lqyfffsqfqalkry;qyfffsqfqalkrya;yfffsqfqalkrya
n;fffsqfqalkryand;ffsqfqalkryandk;fsqfqalkryandkg;sqfqalkryandkgi;qfqalkryandkgie;fqal
kryandkgiel;qalkryandkgielv;alkryandkgielvm;lkryandkgielvmn;kryandkgielvmnl;ryandk
gielvmnlp;yandkgielvmnlpl;andkgielvmnlplf;ndkgielvmnlplfi;dkgielvmnlplfia;kgielvmnl
plfiay;gielvmnlplfiayd;ielvmnlplfiayds;elvmnlplfiaydsa;lvmnlplfiaydsad;vmnlplfiaydsad
v;mnlplfiaydsadvw;nlplfiaydsadvwa;lplfiaydsadvwah;plfiaydsadvwahq;lfiaydsadvwahqk;
fiaydsadvwahqky;iaydsadvwahqkyf;aydsadvwahqkyfk;ydsadvwahqkyfkl;dsadvwahqkyfk
lr;sadvwahqkyfklrf;advwahqkyfklrfd;dvwahqkyfklrfda;vwahqkyfklrfdas;wahqkyfklrfdas
k;ahqkyfklrfdaskd;hqkyfklrfdaskdk;qkyfklrfdaskdkv;kyfklrfdaskdkva;yfklrfdaskdkvag;fk
lrfdaskdkvagi;klrfdaskdkvagis;lrfdaskdkvagisp;rfdaskdkvagispd;fdaskdkvagispdy;daskdk
vagispdyf;askdkvagispdyfl;skdkvagispdyfle;kdkvagispdyfleq;dkvagispdyfleqe;kvagispdy
fleqeq;vagispdyfleqeqa;agispdyfleqeqaw;gispdyfleqeqawd;ispdyfleqeqawds;spdyfleqeqa
wdsp;pdyfleqeqawdspa;dyfleqeqawdspay;yfleqeqawdspays;fleqeqawdspaysw;leqeqawds
payswn;eqeqawdspayswnv;qeqawdspayswnvl;eqawdspayswnvlk;qawdspayswnvlkn;awd
spayswnvlknv;wdspayswnvlknvk;dspayswnvlknvky;spayswnvlknvkye;payswnvlknvkye
w;ayswnvlknvkyeww;yswnvlknvkyewwa;swnvlknvkyewwak;wnvlknvkyewwakr;nvlknv
kyewwakri;vlknvkyewwakrig;lknvkyewwakrigi;knvkyewwakrigil;nvkyewwakrigilr;vkye
wwakrigilrk;kyewwakrigilrky;yewwakrigilrkyi;ewwakrigilrkyid;wwakrigilrkyidv;wakrig
ilrkyidvi;akrigilrkyidvik;krigilrkyidviki;rigilrkyidvikid;igilrkyidvikidh;gilrkyidvikidhf;ilr
kyidvikidhfr;lrkyidvikidhfrg;rkyidvikidhfrgf;kyidvikidhfrgfv;yidvikidhfrgfvs;idvikidhfrg
fvst;dvikidhfrgfvstw;vikidhfrgfvstwe;ikidhfrgfvstwev;kidhfrgfvstwevg;idhfrgfvstwevgv;
dhfrgfvstwevgvg;hfrgfvstwevgvge;frgfvstwevgvgea;rgfvstwevgvgeay;gfvstwevgvgeaya;f
vstwevgvgeayaf;vstwevgvgeayafn;stwevgvgeayafng;twevgvgeayafngl;wevgvgeayafnglw
;evgvgeayafnglwv;vgvgeayafnglwvk;gvgeayafnglwvks;vgeayafnglwvksp;geayafnglwvks
pg;eayafnglwvkspgr;ayafnglwvkspgrd;yafnglwvkspgrdf;afnglwvkspgrdff;fnglwvkspgrdff
n;nglwvkspgrdffnf;glwvkspgrdffnfi;lwvkspgrdffnfil;wvkspgrdffnfiln;vkspgrdffnfilne;ksp
grdffnfilnei;spgrdffnfilneik;pgrdffnfilneikd;grdffnfilneikdl;rdffnfilneikdlk;dffnfilneikdlki;
ffnfilneikdlkiw;fnfilneikdlkiwv;nfilneikdlkiwve;filneikdlkiwved;ilneikdlkiwvedf;lneikdlk
iwvedfe;neikdlkiwvedfen;eikdlkiwvedfend;ikdlkiwvedfendl;kdlkiwvedfendle;dlkiwvedfe
ndled;lkiwvedfendledv;kiwvedfendledvs;iwvedfendledvsr;wvedfendledvsrl;vedfendledvs
rlr;edfendledvsrlrd;dfendledvsrlrdf;fendledvsrlrdff;endledvsrlrdffn;ndledvsrlrdffnf;dledvs
rlrdffnfp;ledvsrlrdffnfpg;edvsrlrdffnfpgm;dvsrlrdffnfpgmr;vsrlrdffnfpgmri;srlrdffnfpgmri
m;rlrdffnfpgmrimk;lrdffnfpgmrimkl;rdffnfpgmrimkla;dffnfpgmrimklaf;ffnfpgmrimklafd;

fnfpgmrimklafdf;nfpgmrimklafdfd;fpgmrimklafdfds;pgmrimklafdfdsd;gmrimklafdfdsdn; mrimklafdfdsdnq;rimklafdfdsdnqn;imklafdfdsdnqnl;mklafdfdsdnqnlp;klafdfdsdnqnlph;la fdfdsdnqnlphn;afdfdsdnqnlphny;fdfdsdnqnlphnyi;dfdsdnqnlphnyik;fdsdnqnlphnyikn;dsd nqnlphnyiknc;sdnqnlphnyiknci;dnqnlphnyiknciv;nqnlphnyikncivy;qnlphnyikncivyt;nlphn yikncivytg;lphnyikncivytgi;phnyikncivytgig;hnyikncivytgigd;nyikncivytgigdn;yikncivytg igdns;ikncivytgigdnst;kncivytgigdnsti;ncivytgigdnstir;civytgigdnstire;ivytgigdnstiref;vytg igdnstirefv;ytgigdnstirefvn;tgigdnstirefvns;gigdnstirefvnsl;igdnstirefvnsld;gdnstirefvnsld d;dnstirefvnslddl;nstirefvnslddlh;stirefvnslddlhk;tirefvnslddlhkk;irefvnslddlhkky;refvnsld dlhkkyi;efvnslddlhkkyif;fvnslddlhkkyifd;vnslddlhkkyifdy;nslddlhkkyifdyl;slddlhkkyifdyl n;lddlhkkyifdylnt;ddlhkkyifdylntn;dlhkkyifdylntne;lhkkyifdylntned;hkkyifdylntnedf;kkyi fdylntnedfv;kyifdylntnedfvv;yifdylntnedfvvw;ifdylntnedfvvwd;fdylntnedfvvwdm;dylntne dfvvwdmi;ylntnedfvvwdmir;lntnedfvvwdmirs;ntnedfvvwdmirsa;tnedfvvwdmirsam;nedfv vwdmirsams;edfvvwdmirsamss;dfvvwdmirsamssv;fvvwdmirsamssvs;vvwdmirsamssvsd; vwdmirsamssvsds;wdmirsamssvsdsv;dmirsamssvsdsvi;mirsamssvsdsvii;irsamssvsdsviip; rsamssvsdsviipm;samssvsdsviipmq;amssvsdsviipmqd;mssvsdsviipmqdy;ssvsdsviipmqdyi ;svsdsviipmqdyin;vsdsviipmqdyinl;sdsviipmqdyinlg;dsviipmqdyinlgn;sviipmqdyinlgne;v iipmqdyinlgnef;iipmqdyinlgnefr;ipmqdyinlgnefra;pmqdyinlgnefran;mqdyinlgnefrani;qdy inlgnefranip;dyinlgnefranipk;yinlgnefranipkn;inlgnefranipknt;nlgnefranipkntl;lgnefranipk ntln;gnefranipkntlnn;nefranipkntlnnw;efranipkntlnnwi;franipkntlnnwif;ranipkntlnnwifr;a nipkntlnnwifrl;nipkntlnnwifrll;ipkntlnnwifrlle;pkntlnnwifrlles;kntlnnwifrllesd;ntlnnwifrll esdl;tlnnwifrllesdld;lnnwifrllesdlda;nnwifrllesdldat;nwifrllesdldatl;wifrllesdldatls;ifrllesdl datlsk;frllesdldatlskn;rllesdldatlskni;llesdldatlsknis;lesdldatlsknisf;esdldatlsknisfi;sdldatls knisfit;dldatlsknisfitr;ldatlsknisfitrl;datlsknisfitrly;atlsknisfitrlyg;tlsknisfitrlygr;lsknisfitrly grt;

16 mers:
mkykkirinlnlkrks;kykkirinlnlkrksg;ykkirinlnlkrksgi;kkirinlnlkrksgil;kirinlnlkrksgill;irinln lkrksgilln;rinlnlkrksgillni;inlnlkrksgillnis;nlnlkrksgillniss;lnlkrksgillnissl;nlkrksgillnisslp; lkrksgillnisslps;krksgillnisslpsk;rksgillnisslpsky;ksgillnisslpskyg;sgillnisslpskygi;gillnissl pskygig;illnisslpskygigd;llnisslpskygigdl;lnisslpskygigdlg;nisslpskygigdlgk;isslpskygigdl gkg;sslpskygigdlgka;slpskygigdlgkay;lpskygigdlgkayk;pskygigdlgkaykf;skygigdlgk gaykfi;kygigdlgkgaykfid;ygigdlgkgaykfidf;gigdlgkgaykfidfl;igdlgkgaykfidflf;gdlgkgaykf idflfa;dlgkgaykfidflfas;lgkgaykfidflfass;gkgaykfidflfassq;kgaykfidflfassqs;gaykfidflfassq sy;aykfidflfassqsyw;ykfidflfassqsywq;kfidflfassqsywqm;fidflfassqsywqmf;idflfassqsywq mfa;dflfassqsywqmfay;flfassqsywqmfays;lfassqsywqmfaysp;fassqsywqmfayspi;assqsyw qmfayspid;ssqsywqmfayspidf;sqsywqmfayspidft;qsywqmfayspidftr;sywqmfayspidftrs;y wqmfayspidftrsp;wqmfayspidftrspp;qmfayspidftrsppy;mfayspidftrsppys;fayspidftrsppysi; ayspidftrsppysif;yspidftrsppysifs;spidftrsppysifsa;pidftrsppysifsaf;idftrsppysifsafa;dftrspp ysifsafag;ftrsppysifsafagn;trsppysifsafagnv;rsppysifsafagnvy;sppysifsafagnvyy;ppysifsafa gnvyyi;pysifsafagnvyyid;ysifsafagnvyyidl;sifsafagnvyyidle;ifsafagnvyyidlea;fsafagnvyyi dleal;safagnvyyidleald;afagnvyyidlealdk;fagnvyyidlealdkf;agnvyyidlealdkfi;gnvyyidleald kfid;nvyyidlealdkfids;vyyidlealdkfidsd;yyidlealdkfidsdl;yidlealdkfidsdln;idlealdkfidsdlnl; dlealdkfidsdlnll;lealdkfidsdlnllk;ealdkfidsdlnllke;aldkfidsdlnllken;ldkfidsdlnllkene;dkfids dlnllkenet;kfidsdlnllkenetr;fidsdlnllkenetry;idsdlnllkenetrys;dsdlnllkenetrysd;sdlnllkenetr ysdl;dlnllkenetrysdlk;lnllkenetrysdlkk;nllkenetrysdlkkl;llkenetrysdlkkls;lkenetrysdlkklsf; kenetrysdlkklsfk;enetrysdlkklsfkd;netrysdlkklsfkdk;etrysdlkklsfkdkf;trysdlkklsfkdkfl;rys

dlkklsfkdkflk;ysdlkklsfkdkflke;sdlkklsfkdkflkea;dlkklsfkdkflkeaa;lkklsfkdkflkeaal;kklsfk
dkflkeaaln;klsfkdkflkeaalnf;lsfkdkflkeaalnfi;sfkdkflkeaalnfin;fkdkflkeaalnfinr;kdkflkeaal
nfinra;dkflkeaalnfinras;kflkeaalnfinrasv;flkeaalnfinrasvd;lkeaalnfinrasvde;keaalnfinrasvd
ev;eaalnfinrasvdevr;aalnfinrasvdevrs;alnfinrasvdevrsf;lnfinrasvdevrsfe;nfinrasvdevrsfek;f
inrasvdevrsfekf;inrasvdevrsfekfk;nrasvdevrsfekfkk;rasvdevrsfekfkkk;asvdevrsfekfkkks;s
vdevrsfekfkkkss;vdevrsfekfkkkssy;devrsfekfkkkssyw;evrsfekfkkkssywl;vrsfekfkkkssywll
;rsfekfkkkssywlld;sfekfkkkssywlldf;fekfkkkssywlldfa;ekfkkkssywlldfas;kfkkkssywlldfasf
;fkkkssywlldfasfv;kkkssywlldfasfva;kkssywlldfasfvaf;kssywlldfasfvafk;ssywlldfasfvafke;
sywlldfasfvafkey;ywlldfasfvafkeyf;wlldfasfvafkeyfl;lldfasfvafkeyflk;ldfasfvafkeyflkd;dfa
sfvafkeyflkds;fasfvafkeyflkdsr;asfvafkeyflkdsrd;sfvafkeyflkdsrda;fvafkeyflkdsrdaf;vafke
yflkdsrdafn;afkeyflkdsrdafnv;fkeyflkdsrdafnvl;keyflkdsrdafnvlf;eyflkdsrdafnvlfd;yflkdsr
dafnvlfdr;flkdsrdafnvlfdrg;lkdsrdafnvlfdrgi;kdsrdafnvlfdrgil;dsrdafnvlfdrgili;srdafnvlfdrg
ilir;rdafnvlfdrgilirn;dafnvlfdrgilirne;afnvlfdrgilirnek;fnvlfdrgilirnekd;nvlfdrgilirnekdl;vlf
drgilirnekdlf;lfdrgilirnekdlfk;fdrgilirnekdlfkl;drgilirnekdlfklr;rgilirnekdlfklrn;gilirnekdlfk
lrni;ilirnekdlfklrnil;lirnekdlfklrnils;irnekdlfklrnilsk;rnekdlfklrnilske;nekdlfklrnilskei;ekdlf
klrnilskeik;kdlfklrnilskeikv;dlfklrnilskeikvq;lfklrnilskeikvqe;fklrnilskeikvqev;klrnilskeik
vqevl;lrnilskeikvqevlq;rnilskeikvqevlqy;nilskeikvqevlqyf;ilskeikvqevlqyff;lskeikvqevlqy
fff;skeikvqevlqyfffs;keikvqevlqyfffsq;eikvqevlqyfffsqf;ikvqevlqyfffsqfq;kvqevlqyfffsqfq
a;vqevlqyfffsqfqal;qevlqyfffsqfqalk;evlqyfffsqfqalkr;vlqyfffsqfqalkry;lqyfffsqfqalkrya;qy
fffsqfqalkryan;yfffsqfqalkryand;fffsqfqalkryandk;ffsqfqalkryandkg;fsqfqalkryandkgi;sqfq
alkryandkgie;qfqalkryandkgiel;fqalkryandkgielv;qalkryandkgielvm;alkryandkgielvmn;lkr
yandkgielvmnl;kryandkgielvmnlp;ryandkgielvmnlpl;yandkgielvmnlplf;andkgielvmnlplfi;
ndkgielvmnlplfia;dkgielvmnlplfiay;kgielvmnlplfiayd;gielvmnlplfiayds;ielvmnlplfiaydsa;e
lvmnlplfiaydsad;lvmnlplfiaydsadv;vmnlplfiaydsadvw;mnlplfiaydsadvwa;nlplfiaydsadvw
ah;lplfiaydsadvwahq;plfiaydsadvwahqk;lfiaydsadvwahqky;fiaydsadvwahqkyf;iaydsadvw
ahqkyfk;aydsadvwahqkyfkl;ydsadvwahqkyfklr;dsadvwahqkyfklrf;sadvwahqkyfklrfd;adv
wahqkyfklrfda;dvwahqkyfklrfdas;vwahqkyfklrfdask;wahqkyfklrfdaskd;ahqkyfklrfdaskdk
;hqkyfklrfdaskdkv;qkyfklrfdaskdkva;kyfklrfdaskdkvag;yfklrfdaskdkvagi;fklrfdaskdkvagi
s;klrfdaskdkvagisp;lrfdaskdkvagispd;rfdaskdkvagispdy;fdaskdkvagispdyf;daskdkvagispd
yfl;askdkvagispdyfle;skdkvagispdyfleq;kdkvagispdyfleqe;dkvagispdyfleqeq;kvagispdyfle
qeqa;vagispdyfleqeqaw;agispdyfleqeqawd;gispdyfleqeqawds;ispdyfleqeqawdsp;spdyfleq
eqawdspa;pdyfleqeqawdspay;dyfleqeqawdspays;yfleqeqawdspaysw;fleqeqawdspayswn;l
eqeqawdspayswnv;eqeqawdspayswnvl;qeqawdspayswnvlk;eqawdspayswnvlkn;qawdspa
yswnvlknv;awdspayswnvlknvk;wdspayswnvlknvky;dspayswnvlknvkye;spayswnvlknvky
ew;payswnvlknvkyeww;ayswnvlknvkyewwa;yswnvlknvkyewwak;swnvlknvkyewwakr;w
nvlknvkyewwakri;nvlknvkyewwakrig;vlknvkyewwakrigi;lknvkyewwakrigil;knvkyewwa
krigilr;nvkyewwakrigilrk;vkyewwakrigilrky;kyewwakrigilrkyi;yewwakrigilrkyid;ewwakr
igilrkyidv;wwakrigilrkyidvi;wakrigilrkyidvik;akrigilrkyidviki;krigilrkyidvikid;rigilrkyidv
ikidh;igilrkyidvikidhf;gilrkyidvikidhfr;ilrkyidvikidhfrg;lrkyidvikidhfrgf;rkyidvikidhfrgfv;
kyidvikidhfrgfvs;yidvikidhfrgfvst;idvikidhfrgfvstw;dvikidhfrgfvstwe;vikidhfrgfvstwev;ik
idhfrgfvstwevg;kidhfrgfvstwevgv;idhfrgfvstwevgvg;dhfrgfvstwevgvge;hfrgfvstwevgvgea
;frgfvstwevgvgeay;rgfvstwevgvgeaya;gfvstwevgvgeayaf;fvstwevgvgeayafn;vstwevgvgea
yafng;stwevgvgeayafngl;twevgvgeayafnglw;wevgvgeayafnglwv;evgvgeayafnglwvk;vgvg
eayafnglwvks;gvgeayafnglwvksp;vgeayafnglwvkspg;geayafnglwvkspgr;eayafnglwvkspg
rd;ayafnglwvkspgrdf;yafnglwvkspgrdff;afnglwvkspgrdffn;fnglwvkspgrdffnf;nglwvkspgr
dffnfi;glwvkspgrdffnfil;lwvkspgrdffnfiln;wvkspgrdffnfilne;vkspgrdffnfilnei;kspgrdffnfiln

eik;spgrdffnfilneikd;pgrdffnfilneikdl;grdffnfilneikdlk;rdffnfilneikdlki;dffnfilneikdlkiw;ff
nfilneikdlkiwv;fnfilneikdlkiwve;nfilneikdlkiwved;filneikdlkiwvedf;ilneikdlkiwvedfe;lnei
kdlkiwvedfen;neikdlkiwvedfend;eikdlkiwvedfendl;ikdlkiwvedfendle;kdlkiwvedfendled;d
lkiwvedfendledv;lkiwvedfendledvs;kiwvedfendledvsr;iwvedfendledvsrl;wvedfendledvsrl
r;vedfendledvsrlrd;edfendledvsrlrdf;dfendledvsrlrdff;fendledvsrlrdffn;endledvsrlrdffnf;nd
ledvsrlrdffnfp;dledvsrlrdffnfpg;ledvsrlrdffnfpgm;edvsrlrdffnfpgmr;dvsrlrdffnfpgmri;vsrlr
dffnfpgmrim;srlrdffnfpgmrimk;rlrdffnfpgmrimkl;lrdffnfpgmrimkla;rdffnfpgmrimklaf;dff
nfpgmrimklafd;ffnfpgmrimklafdf;fnfpgmrimklafdfd;nfpgmrimklafdfds;fpgmrimklafdfdsd
;pgmrimklafdfdsdn;gmrimklafdfdsdnq;mrimklafdfdsdnqn;rimklafdfdsdnqnl;imklafdfdsdn
qnlp;mklafdfdsdnqnlph;klafdfdsdnqnlphn;lafdfdsdnqnlphny;afdfdsdnqnlphnyi;fdfdsdnqnl
phnyik;dfdsdnqnlphnyikn;fdsdnqnlphnyiknc;dsdnqnlphnyiknci;sdnqnlphnyiknciv;dnqnlp
hnyiknciviy;nqnlphnyikncivyt;qnlphnyikncivytg;nlphnyikncivytgi;lphnyikncivytgig;phnyi
kncivytgigd;hnyikncivytgigdn;nyikncivytgigdns;yikncivytgigdnst;ikncivytgigdnsti;knciv
ytgigdnstir;ncivytgigdnstire;civytgigdnstiref;ivytgigdnstirefv;vytgigdnstirefvn;ytgigdnstir
efvns;tgigdnstirefvnsl;gigdnstirefvnsld;igdnstirefvnsldd;gdnstirefvnslddl;dnstirefvnslddlh
;nstirefvnslddlhk;stirefvnslddlhkk;tirefvnslddlhkky;irefvnslddlhkkyi;refvnslddlhkkyif;efv
nslddlhkkyifd;fvnslddlhkkyifdy;vnslddlhkkyifdyl;nslddlhkkyifdyln;slddlhkkyifdylnt;lddl
hkkyifdylntn;ddlhkkyifdylntne;dlhkkyifdylntned;lhkkyifdylntnedf;hkkyifdylntnedfv;kkyi
fdylntnedfvv;kyifdylntnedfvvw;yifdylntnedfvvwd;ifdylntnedfvvwdm;fdylntnedfvvwdmi;
dylntnedfvvwdmir;ylntnedfvvwdmirs;lntnedfvvwdmirsa;ntnedfvvwdmirsam;tnedfvvwdm
irsams;nedfvvwdmirsamss;edfvvwdmirsamssv;dfvvwdmirsamssvs;fvvwdmirsamssvsd;vv
wdmirsamssvsds;vwdmirsamssvsdsv;wdmirsamssvsdsvi;dmirsamssvsdsvii;mirsamssvsds
viip;irsamssvsdsviipm;rsamssvsdsviipmq;samssvsdsviipmqd;amssvsdsviipmqdy;mssvsds
viipmqdyi;ssvsdsviipmqdyin;svsdsviipmqdyinl;vsdsviipmqdyinlg;sdsviipmqdyinlgn;dsvi
ipmqdyinlgne;sviipmqdyinlgnef;viipmqdyinlgnefr;iipmqdyinlgnefra;ipmqdyinlgnefran;p
mqdyinlgnefrani;mqdyinlgnefranip;qdyinlgnefranipk;dyinlgnefranipkn;yinlgnefranipknt;i
nlgnefranipkntl;nlgnefranipkntln;lgnefranipkntlnn;gnefranipkntlnnw;nefranipkntlnnwi;efr
anipkntlnnwif;franipkntlnnwifr;ranipkntlnnwifrl;anipkntlnnwifrll;nipkntlnnwifrlle;ipkntln
nwifrlles;pkntlnnwifrllesd;kntlnnwifrllesdl;ntlnnwifrllesdld;tlnnwifrllesdlda;lnnwifrllesdl
dat;nnwifrllesdldatl;nwifrllesdldatls;wifrllesdldatlsk;ifrllesdldatlskn;frllesdldatlskni;rllesd
ldatlsknis;llesdldatlsknisf;lesdldatlsknisfi;esdldatlsknisfit;sdldatlsknisfitr;dldatlsknisfitrl;l
datlsknisfitrly;datlsknisfitrlyg;atlsknisfitrlygr;tlsknisfitrlygrt;

<CAA59725 outer surface protein A;Protein;Borrelia afzelii>

8 mers:
mkkyllgi;kkyllgig;kyllgigl;yllgigli;llgiglil;lgiglila;giglilal;iglilali;glilalia;lilaliac;ilaliack;l
aliackq;aliackqn;liackqnv;iackqnvs;ackqnvss;ckqnvssl;kqnvssld;qnvsslde;nvssldek;vsslde
kn;ssldekns;sldeknsa;ldeknsas;deknsasv;eknsasvd;knsasvdl;nsasvdlp;sasvdlpg;asvdlpge;s
vdlpgem;vdlpgemk;dlpgemkv;lpgemkvl;pgemkvlv;gemkvlvs;emkvlvsk;mkvlvske;kvlvsk
ek;vlvskekd;lvskekdk;vskekdkd;skekdkdg;kekdkdgk;ekdkdgky;kdkdgkys;dkdgkysl;kdgk
yslk;dgkyslka;gkyslkat;kyslkatv;yslkatvd;slkatvdk;lkatvdki;katvdkie;atvdkiel;tvdkielk;vd
kielkg;dkielkgt;kielkgts;ielkgtsd;elkgtsdk;lkgtsdkd;kgtsdkdn;gtsdkdng;tsdkdngs;sdkdngs
g;dkdngsgv;kdngsgvl;dngsgvle;ngsgvleg;gsgvlegt;sgvlegtk;gvlegtkd;vlegtkdd;legtkddk;e
gtkddks;gtkddksk;tkddkska;kddkskak;ddkskakl;dkskaklt;kskaklti;skakltia;kakltiad;akltiad
d;kltiaddl;ltiaddls;tiaddlsk;iaddlskt;addlsktt;ddlskttf;dlskttfe;lskttfel;skttfelf;kttfelfk;ttfelf

ke;tfelfked;felfkedg;elfkedgk;lfkedgkt;fkedgktl;kedgktlv;edgktlvs;dgktlvsr;gktlvsrk;ktlvs
rkv;tlvsrkvs;lvsrkvss;vsrkvssk;srkvsskd;rkvsskdk;kvsskdkt;vsskdkts;sskdktst;skdktstd;kd
ktstde;dktstdem;ktstdemf;tstdemfn;stdemfne;tdemfnek;demfnekg;emfnekge;mfnekgel;fne
kgels;nekgelsa;ekgelsak;kgelsakt;gelsaktm;elsaktmt;lsaktmtr;saktmtre;aktmtren;ktmtreng
;tmtrengt;mtrengtk;trengtkl;rengtkle;engtkley;ngtkleyt;gtkleyte;tkleytem;kleytemk;leyte
mks;eytemksd;ytemksdg;temksdgt;emksdgtg;mksdgtgk;ksdgtgka;sdgtgkak;dgtgkake;gtg
kakev;tgkakevl;gkakevlk;kakevlkn;akevlknf;kevlknft;evlknftl;vlknftle;lknftleg;knftlegk;n
ftlegkv;ftlegkva;tlegkvan;legkvand;egkvandk;gkvandkv;kvandkvt;vandkvtl;andkvtle;ndk
vtlev;dkvtlevk;kvtlevke;vtlevkeg;tlevkegt;levkegtv;evkegtvt;vkegtvtl;kegtvtls;egtvtlsk;gt
vtlske;tvtlskei;vtlskeia;tlskeiak;lskeiaks;skeiaksg;keiaksge;eiaksgev;iaksgevt;aksgevtv;ks
gevtva;sgevtval;gevtvaln;evtvalnd;vtvalndt;tvalndtn;valndtnt;alndtntt;lndtnttq;ndtnttqa;dt
nttqat;tnttqatk;nttqatkk;ttqatkkt;tqatkktg;qatkktga;atkktgaw;tkktgawd;kktgawds;ktgawdsk
;tgawdskt;gawdskts;awdsktst;wdsktstl;dsktstlt;sktstlti;ktstltis;tstltisv;stltisvn;tltisvns;ltisv
nsk;tisvnskk;isvnskkt;svnskktt;vnskkttq;nskkttql;skkttqlv;kkttqlvf;kttqlvft;ttqlvftk;tqlvftk
q;qlvftkqd;lvftkqdt;vftkqdti;ftkqdtit;tkqdtitv;kqdtitvq;qdtitvqk;dtitvqky;titvqkyd;itvqkyds
;tvqkydsa;vqkydsag;qkydsagt;kydsagtn;ydsagtnl;dsagtnle;sagtnleg;agtnlegt;gtnlegta;tnleg
tav;nlegtave;legtavei;egtaveik;gtaveikt;taveiktl;aveiktld;veiktlde;eiktldel;iktldelk;ktldelkn
;tldelkna;ldelknal;delknalk;

9 mers:
mkkyllgig;kkyllgigl;kyllgigli;yllgiglil;llgiglila;lgiglilal;giglilali;iglilalia;glilaliac;lilaliack;
ilaliackq;laliackqn;aliackqnv;liackqnvs;iackqnvss;ackqnvssl;ckqnvssld;kqnvsslde;qnvssld
ek;nvssldekn;vssldekns;ssldeknsa;sldeknsas;ldeknsasv;deknsasvd;eknsasvdl;knsasvdlp;ns
asvdlpg;sasvdlpge;asvdlpgem;svdlpgemk;vdlpgemkv;dlpgemkvl;lpgemkvlv;pgemkvlvs;g
emkvlvsk;emkvlvske;mkvlvskek;kvlvskekd;vlvskekdk;lvskekdkd;vskekdkdg;skekdkdgk;
kekdkdgky;ekdkdgkys;kdkdgkysl;dkdgkyslk;kdgkyslka;dgkyslkat;gkyslkatv;kyslkatvd;ys
lkatvdk;slkatvdki;lkatvdkie;katvdkiel;atvdkielk;tvdkielkg;vdkielkgt;dkielkgts;kielkgtsd;ie
lkgtsdk;elkgtsdkd;lkgtsdkdn;kgtsdkdng;gtsdkdngs;tsdkdngsg;sdkdngsgv;dkdngsgvl;kdng
sgvle;dngsgvleg;ngsgvlegt;gsgvlegtk;sgvlegtkd;gvlegtkdd;vlegtkddk;legtkddks;egtkddks
k;gtkddkska;tkddkskak;kddkskakl;ddkskaklt;dkskaklti;kskakltia;skakltiad;kakltiadd;aklti
addl;kltiaddls;ltiaddlsk;tiaddlskt;iaddlsktt;addlskttf;ddlskttfe;dlskttfel;lskttfelf;skttfelfk;kt
tfelfke;ttfelfked;tfelfkedg;felfkedgk;elfkedgkt;lfkedgktl;fkedgktlv;kedgktlvs;edgktlvsr;dg
ktlvsrk;gktlvsrkv;ktlvsrkvs;tlvsrkvss;lvsrkvssk;vsrkvsskd;srkvsskdk;rkvsskdkt;kvsskdkts;
vsskdktst;sskdktstd;skdktstde;kdktstdem;dktstdemf;ktstdemfn;tstdemfne;stdemfnek;tdem
fnekg;demfnekge;emfnekgel;mfnekgels;fnekgelsa;nekgelsak;ekgelsakt;kgelsaktm;gelsakt
mt;elsaktmtr;lsaktmtre;saktmtren;aktmtreng;ktmtrengt;tmtrengtk;mtrengtkl;trengtkle;reng
tkley;engtkleyt;ngtkleyte;gtkleytem;tkleytemk;kleytemks;leytemksd;eytemksdg;ytemksd
gt;temksdgtg;emksdgtgk;mksdgtgka;ksdgtgkak;sdgtgkake;dgtgkakev;gtgkakevl;tgkakevl
k;gkakevlkn;kakevlknf;akevlknft;kevlknftl;evlknftle;vlknftleg;lknftlegk;knftlegkv;nftlegk
va;ftlegkvan;tlegkvand;legkvandk;egkvandkv;gkvandkvt;kvandkvtl;vandkvtle;andkvtlev;
ndkvtlevk;dkvtlevke;kvtlevkeg;vtlevkegt;tlevkegtv;levkegtvt;evkegtvtl;vkegtvtls;kegtvtls
k;egtvtlske;gtvtlskei;tvtlskeia;vtlskeiak;tlskeiaks;lskeiaksg;skeiaksge;keiaksgev;eiaksgevt
;iaksgevtv;aksgevtva;ksgevtval;sgevtvaln;gevtvalnd;evtvalndt;vtvalndtn;tvalndtnt;valndtn
tt;alndtnttq;lndtnttqa;ndtnttqat;dtnttqatk;tnttqatkk;nttqatkkt;ttqatkktg;tqatkktga;qatkktgaw
;atkktgawd;tkktgawds;kktgawdsk;ktgawdskt;tgawdskts;gawdsktst;awdsktstl;wdsktstlt;dsk
tstlti;sktstltis;ktstltisv;tstltisvn;stltisvns;tltisvnsk;ltisvnskk;tisvnskkt;isvnskktt;svnskkttq;v

nskkttql;nskkttqlv;skkttqlvf;kkttqlvft;kttqlvftk;ttqlvftkq;tqlvftkqd;qlvftkqdt;lvftkqdti;vftk
qdtit;ftkqdtitv;tkqdtitvq;kqdtitvqk;qdtitvqky;dtitvqkyd;titvqkyds;itvqkydsa;tvqkydsag;vq
kydsagt;qkydsagtn;kydsagtnl;ydsagtnle;dsagtnleg;sagtnlegt;agtnlegta;gtnlegtav;tnlegtave;
nlegtavei;legtaveik;egtaveikt;gtaveiktl;taveiktld;aveiktlde;veiktldel;eiktldelk;iktldelkn;ktl
delkna;tldelknal;ldelknalk;

10 mers:
mkkyllgigl;kkyllgigli;kyllgiglil;yllgiglila;llgiglilal;lgiglilali;giglilalia;iglilaliac;glilaliack;l
ilaliackq;ilaliackqn;laliackqnv;aliackqnvs;liackqnvss;iackqnvssl;ackqnvssld;ckqnvsslde;k
qnvssldek;qnvssldekn;nvssldekns;vssldeknsa;ssldeknsas;sldeknsasv;ldeknsasvd;deknsasv
dl;eknsasvdlp;knsasvdlpg;nsasvdlpge;sasvdlpgem;asvdlpgemk;svdlpgemkv;vdlpgemkvl;
dlpgemkvlv;lpgemkvlvs;pgemkvlvsk;gemkvlvske;emkvlvskek;mkvlvskekd;kvlvskekdk;v
lvskekdk;lvskekdkdg;vskekdkdgk;skekdkdgky;kekdkdgkys;ekdkdgkysl;kdkdgkyslk;dkd
gkyslka;kdgkyslkat;dgkyslkatv;gkyslkatvd;kyslkatvdk;yslkatvdki;slkatvdkie;lkatvdkiel;k
atvdkielk;atvdkielkg;tvdkielkgt;vdkielkgts;dkielkgtsd;kielkgtsdk;ielkgtsdkd;elkgtsdkdn;l
kgtsdkdng;kgtsdkdngs;gtsdkdngsg;tsdkdngsgv;sdkdngsgvl;dkdngsgvle;kdngsgvleg;dngsg
vlegt;ngsgvlegtk;gsgvlegtkd;sgvlegtkdd;gvlegtkddk;vlegtkddks;legtkddksk;egtkddkska;gt
kddkskak;tkddkskakl;kddkskaklt;ddkskaklti;dkskakltia;kskakltiad;skakltiadd;kakltiaddl;a
kltiaddls;kltiaddlsk;ltiaddlskt;tiaddlsktt;iaddlskttf;addlskttfe;ddlskttfel;dlskttfelf;lskttfelfk
;skttfelfke;kttfelfked;ttfelfkedg;tfelfkedgk;felfkedgkt;elfkedgktl;lfkedgktlv;fkedgktlvs;ke
dgktlvsr;edgktlvsrk;dgktlvsrkv;gktlvsrkvs;ktlvsrkvss;tlvsrkvssk;lvsrkvsskd;vsrkvsskdk;sr
kvsskdkt;rkvsskdkts;kvsskdktst;vsskdktstd;sskdktstde;skdktstdem;kdktstdemf;dktstdemfn
;ktstdemfne;tstdemfnek;stdemfnekg;tdemfnekge;demfnekgel;emfnekgels;mfnekgelsa;fne
kgelsak;nekgelsakt;ekgelsaktm;kgelsaktmt;gelsaktmtr;elsaktmtre;lsaktmtren;saktmtreng;a
ktmtrengt;ktmtrengtk;tmtrengtkl;mtrengtkle;trengtkley;rengtkleyt;engtkleyte;ngtkleytem;
gtkleytemk;tkleytemks;kleytemksd;leytemksdg;eytemksdgt;ytemksdgtg;temksdgtgk;emk
sdgtgka;mksdgtgkak;ksdgtgkake;sdgtgkakev;dgtgkakevl;gtgkakevlk;tgkakevlkn;gkakevl
knf;kakevlknft;akevlknftl;kevlknftle;evlknftleg;vlknftlegk;lknftlegkv;knftlegkva;nftlegkv
an;ftlegkvand;tlegkvandk;legkvandkv;egkvandkvt;gkvandkvtl;kvandkvtle;vandkvtlev;an
dkvtlevk;ndkvtlevke;dkvtlevkeg;kvtlevkegt;vtlevkegtv;tlevkegtvt;levkegtvtl;evkegtvtls;v
kegtvtlsk;kegtvtlske;egtvtlskei;gtvtlskeia;tvtlskeiak;vtlskeiaks;tlskeiaksg;lskeiaksge;skeia
ksgev;keiaksgevt;eiaksgevtv;iaksgevtva;aksgevtval;ksgevtvaln;sgevtvalnd;gevtvalndt;evt
valndtn;vtvalndtnt;tvalndtntt;valndtnttq;alndtnttqa;lndtnttqat;ndtnttqatk;dtnttqatkk;tnttqat
kkt;nttqatkktg;ttqatkktga;tqatkktgaw;qatkktgawd;atkktgawds;tkktgawdsk;kktgawdskt;ktg
awdskts;tgawdsktst;gawdsktstl;awdsktstlt;wdsktstlti;dsktstltis;sktstltisv;ktstltisvn;tstltisvn
s;stltisvnsk;tltisvnskk;ltisvnskkt;tisvnskktt;isvnskkttq;svnskkttql;vnskkttqlv;nskkttqlvf;sk
kttqlvft;kkttqlvftk;kttqlvftkq;ttqlvftkqd;tqlvftkqdt;qlvftkqdti;lvftkqdtit;vftkqdtitv;ftkqdtitv
q;tkqdtitvqk;kqdtitvqky;qdtitvqkyd;dtitvqkyds;titvqkydsa;itvqkydsag;tvqkydsagt;vqkyds
agtn;qkydsagtnl;kydsagtnle;ydsagtnleg;dsagtnlegt;sagtnlegta;agtnlegtav;gtnlegtave;tnlegt
avei;nlegtaveik;legtaveikt;egtaveiktl;gtaveiktld;taveiktlde;aveiktldel;veiktldelk;eiktldelkn
;iktldelkna;ktldelknal;tldelknalk;

11 mers:
mkkyllgigli;kkyllgiglil;kyllgiglila;yllgiglilal;llgiglilali;lgiglilalia;giglilaliac;iglilaliack;glil
aliackq;lilaliackqn;ilaliackqnv;laliackqnvs;aliackqnvss;liackqnvssl;iackqnvssld;ackqnvssl
de;ckqnvssldek;kqnvssldekn;qnvssldekns;nvssldeknsa;vssldeknsas;ssldeknsasv;sldeknsas

EP 2 254 592 B1

vd;ldeknsasvdl;deknsasvdlp;eknsasvdlpg;knsasvdlpge;nsasvdlpgem;sasvdlpgemk;asvdlp
gemkv;svdlpgemkvl;vdlpgemkvlv;dlpgemkvlvs;lpgemkvlvsk;pgemkvlvske;gemkvlvskek
;emkvlvskekd;mkvlvskekdk;kvlvskekdkd;vlvskekdkdg;lvskekdkdgk;vskekdkdgky;skekd
kdgkys;kekdkdgkysl;ekdkdgkyslk;kdkdgkyslka;dkdgkyslkat;kdgkyslkatv;dgkyslkatvd;gk
yslkatvdk;kyslkatvdki;yslkatvdkie;slkatvdkiel;lkatvdkielk;katvdkielkg;atvdkielkgt;tvdkiel
kgts;vdkielkgtsd;dkielkgtsdk;kielkgtsdkd;ielkgtsdkdn;elkgtsdkdng;lkgtsdkdngs;kgtsdkdn
gsg;gtsdkdngsgv;tsdkdngsgvl;sdkdngsgvle;dkdngsgvleg;kdngsgvlegt;dngsgvlegtk;ngsgvl
egtkd;gsgvlegtkdd;sgvlegtkddk;gvlegtkddks;vlegtkddksk;legtkddkska;egtkddkskak;gtkdd
kskakl;tkddkskaklt;kddkskaklti;ddkskakltia;dkskakltiad;kskakltiadd;skakltiaddl;kakltiadd
ls;akltiaddlsk;kltiaddlskt;ltiaddlskt;tiaddlsktt;iaddlsktt;addlskttfel;ddlskttfelf;dlskttfelf
k;lskttfelfke;skttfelfked;kttfelfkedg;ttfelfkedgk;tfelfkedgkt;felfkedgktl;elfkedgktlv;lfkedg
ktlvs;fkedgktlvsr;kedgktlvsrk;edgktlvsrkv;dgktlvsrkvs;gktlvsrkvss;ktlvsrkvssk;tlvsrkvssk
d;lvsrkvsskdk;vsrkvsskdkt;srkvsskdkts;rkvsskdktst;kvsskdktstd;vsskdktstde;sskdktstdem;
skdktstdemf;kdktstdemfn;dktstdemfne;ktstdemfnek;tstdemfnekg;stdemfnekge;tdemfnekg
el;demfnekgels;emfnekgelsa;mfnekgelsak;fnekgelsakt;nekgelsaktm;ekgelsaktmt;kgelsakt
mtr;gelsaktmtre;elsaktmtren;lsaktmtreng;saktmtrengt;aktmtrengtk;ktmtrengtkl;tmtrengtkl
e;mtrengtkley;trengtkleyt;rengtkleyte;engtkleytem;ngtkleytemk;gtkleytemks;tkleytemksd
;kleytemksdg;leytemksdgt;eytemksdgtg;ytemksdgtgk;temksdgtgka;emksdgtgkak;mksdgt
gkake;ksdgtgkakev;sdgtgkakevl;dgtgkakevlk;gtgkakevlkn;tgkakevlknf;gkakevlknft;kake
vlknftl;akevlknftle;kevlknftleg;evlknftlegk;vlknftlegkv;lknftlegkva;knftlegkvan;nftlegkva
nd;ftlegkvandk;tlegkvandkv;legkvandkvt;egkvandkvtl;gkvandkvtle;kvandkvtlev;vandkvtl
evk;andkvtlevke;ndkvtlevkeg;dkvtlevkegt;kvtlevkegtv;vtlevkegtvt;tlevkegtvtl;levkegtvtls
;evkegtvtlsk;vkegtvtlske;kegtvtlskei;egtvtlskeia;gtvtlskeiak;tvtlskeiaks;vtlskeiaksg;tlskeia
ksge;lskeiaksgev;skeiaksgevt;keiaksgevtv;eiaksgevtva;iaksgevtval;aksgevtvaln;ksgevtval
nd;sgevtvalndt;gevtvalndtn;evtvalndtnt;vtvalndtntt;tvalndtnttq;valndtnttqa;alndtnttqat;lnd
tnttqatk;ndtnttqatkk;dtnttqatkkt;tnttqatkktg;nttqatkktga;ttqatkktgaw;tqatkktgawd;qatkktga
wds;atkktgawdsk;tkktgawdskt;kktgawdskts;ktgawdsktst;tgawdsktstl;gawdsktstlt;awdsktst
lti;wdsktstltis;dsktstltisv;sktstltisvn;ktstltisvns;tstltisvnsk;stltisvnskk;tltisvnskkt;ltisvnskkt
t;tisvnskkttq;isvnskkttql;svnskkttqlv;vnskkttqlvf;nskkttqlvft;skkttqlvftk;kkttqlvftkq;kttqlv
ftkqd;ttqlvftkqdt;tqlvftkqdti;qlvftkqdtit;lvftkqdtitv;vftkqdtitvq;ftkqdtitvqk;tkqdtitvqky;kq
dtitvqkyd;qdtitvqkyds;dtitvqkydsa;titvqkydsag;itvqkydsagt;tvqkydsagtn;vqkydsagtnl;qky
dsagtnle;kydsagtnleg;ydsagtnlegt;dsagtnlegta;sagtnlegtav;agtnlegtave;gtnlegtavei;tnlegta
veik;nlegtaveikt;legtaveiktl;egtaveiktld;gtaveiktlde;taveiktldel;aveiktldelk;veiktldelkn;eik
tldelkna;iktldelknal;ktldelknalk;

13 mers:

mkkyllgiglila;kkyllgiglilal;kyllgiglilali;yllgiglilalia;llgiglilaliac;lgiglilaliack;giglilaliackq;
iglilaliackqn;glilaliackqnv;lilaliackqnvs;ilaliackqnvss;laliackqnvssl;aliackqnvssld;liackqn
vsslde;iackqnvssldek;ackqnvssldekn;ckqnvssldekns;kqnvssldeknsa;qnvssldeknsas;nvssld
eknsasv;vssldeknsasvd;ssldeknsasvdl;sldeknsasvdlp;ldeknsasvdlpg;deknsasvdlpge;eknsas
vdlpgem;knsasvdlpgemk;nsasvdlpgemkv;sasvdlpgemkvl;asvdlpgemkvlv;svdlpgemkvlvs;
vdlpgemkvlvsk;dlpgemkvlvske;lpgemkvlvskek;pgemkvlvskekd;gemkvlvskekdk;emkvlvs
kekdkd;mkvlvskekdkdg;kvlvskekdkdgk;vlvskekdkdgky;lvskekdkdgkys;vskekdkdgkysl;s
kekdkdgkyslk;kekdkdgkyslka;ekdkdgkyslkat;kdkdgkyslkatv;dkdgkyslkatvd;kdgkyslkatv
dk;dgkyslkatvdki;gkyslkatvdkie;kyslkatvdkiel;yslkatvdkielk;slkatvdkielkg;lkatvdkielkgt;
katvdkielkgts;atvdkielkgtsd;tvdkielkgtsdk;vdkielkgtsdkd;dkielkgtsdkdn;kielkgtsdkdng;iel

kgtsdkdngs;elkgtsdkdngsg;lkgtsdkdngsgv;kgtsdkdngsgvl;gtsdkdngsgvle;tsdkdngsgvleg;s
dkdngsgvlegt;dkdngsgvlegtk;kdngsgvlegtkd;dngsgvlegtkdd;ngsgvlegtkddd;gsgvlegtkddk
s;sgvlegtkddksk;gvlegtkddkska;vlegtkddkskak;legtkddkskakl;egtkddkskaklt;gtkddkskaklt
i;tkddkskakltia;kddkskakltiad;ddkskakltiadd;dkskakltiaddl;kskakltiaddls;skakltiaddlsk;ka
kltiaddlskt;akltiaddlsktt;kltiaddlskttf;ltiaddlskttfe;tiaddlskttfel;iaddlskttfelf;addlskttfelfk;d
dlskttfelfke;dlskttfelfked;lskttfelfkedg;skttfelfkedgk;kttfelfkedgkt;ttfelfkedgktl;tfelfkedgk
tlv;felfkedgktlvs;elfkedgktlvsr;lfkedgktlvsrk;fkedgktlvsrkv;kedgktlvsrkvs;edgktlvsrkvss;
dgktlvsrkvssk;gktlvsrkvsskd;ktlvsrkvsskdk;tlvsrkvsskdkt;lvsrkvsskdkts;vsrkvsskdktst;srk
vsskdktstd;rkvsskdktstde;kvsskdktstdem;vsskdktstdemf;sskdktstdemfn;skdktstdemfne;kd
ktstdemfnek;dktstdemfnekg;ktstdemfnekge;tstdemfnekgel;stdemfnekgels;tdemfnekgelsa;
demfnekgelsak;emfnekgelsakt;mfnekgelsaktm;fnekgelsaktmt;nekgelsaktmtr;ekgelsaktmtr
e;kgelsaktmtren;gelsaktmtreng;elsaktmtrengt;lsaktmtrengtk;saktmtrengtkl;aktmtrengtkle;
ktmtrengtkley;tmtrengtkleyt;mtrengtkleyte;trengtkleytem;rengtkleytemk;engtkleytemks;n
gtkleytemksd;gtkleytemksdg;tkleytemksdgt;kleytemksdgtg;leytemksdgtgk;eytemksdgtgk
a;ytemksdgtgkak;temksdgtgkake;emksdgtgkakev;mksdgtgkakevl;ksdgtgkakevlk;sdgtgka
kevlkn;dgtgkakevlknf;gtgkakevlknft;tgkakevlknftl;gkakevlknftle;kakevlknftleg;akevlknft
legk;kevlknftlegkv;evlknftlegkva;vlknftlegkvan;lknftlegkvand;knftlegkvandk;nftlegkvan
dkv;ftlegkvandkvt;tlegkvandkvtl;legkvandkvtle;egkvandkvtlev;gkvandkvtlevk;kvandkvtl
evke;vandkvtlevkeg;andkvtlevkegt;ndkvtlevkegtv;dkvtlevkegtvt;kvtlevkegtvtl;vtlevkegtv
tls;tlevkegtvtlsk;levkegtvtlske;evkegtvtlskei;vkegtvtlskeia;kegtvtlskeiak;egtvtlskeiaks;gtv
tlskeiaksg;tvtlskeiaksge;vtlskeiaksgev;tlskeiaksgevt;lskeiaksgevtv;skeiaksgevtva;keiaksg
evtval;eiaksgevtvaln;iaksgevtvalnd;aksgevtvalndt;ksgevtvalndtn;sgevtvalndtnt;gevtvalndt
ntt;evtvalndtnttq;vtvalndtnttqa;tvalndtnttqat;valndtnttqatk;alndtnttqatkk;lndtnttqatkkt;ndt
nttqatkktg;dtnttqatkktga;tnttqatkktgaw;nttqatkktgawd;ttqatkktgawds;tqatkktgawdsk;qatkk
tgawdskt;atkktgawdskts;tkktgawdsktst;kktgawdsktstl;ktgawdsktstlt;tgawdsktstlti;gawdskt
stltis;awdsktstltisv;wdsktstltisvn;dsktstltisvns;sktstltisvnsk;ktstltisvnskk;tstltisvnskkt;stlti
svnskktt;tltisvnskkttq;ltisvnskkttql;tisvnskkttqlv;isvnskkttqlvf;svnskkttqlvft;vnskkttqlvftk
;nskkttqlvftkq;skkttqlvftkqd;kkttqlvftkqdt;kttqlvftkqdti;ttqlvftkqdtit;tqlvftkqdtitv;qlvftkq
dtitvq;lvftkqdtitvqk;vftkqdtitvqky;ftkqdtitvqkyd;tkqdtitvqkyds;kqdtitvqkydsa;qdtitvqkyd
sag;dtitvqkydsagt;titvqkydsagtn;itvqkydsagtnl;tvqkydsagtnle;vqkydsagtnleg;qkydsagtnle
gt;kydsagtnlegta;ydsagtnlegtav;dsagtnlegtave;sagtnlegtavei;agtnlegtaveik;gtnlegtaveikt;tn
legtaveiktl;nlegtaveiktld;legtaveiktlde;egtaveiktldel;gtaveiktldelk;taveiktldelkn;aveiktldel
kna;veiktldelknal;eiktldelknalk;

14 mers:
mkkyllgiglilal;kkyllgiglilali;kyllgiglilalia;yllgiglilaliac;llgiglilaliack;lgiglilaliackq;giglilal
iackqn;iglilaliackqnv;glilaliackqnvs;lilaliackqnvss;ilaliackqnvssl;laliackqnvssld;aliackqn
vsslde;liackqnvssldek;iackqnvssldekn;ackqnvssldekns;ckqnvssldeknsa;kqnvssldeknsas;q
nvssldeknsasv;nvssldeknsasvd;vssldeknsasvdl;ssldeknsasvdlp;sldeknsasvdlpg;ldeknsasvd
lpge;deknsasvdlpgem;eknsasvdlpgemk;knsasvdlpgemkv;nsasvdlpgemkvl;sasvdlpgemkvl
v;asvdlpgemkvlvs;svdlpgemkvlvsk;vdlpgemkvlvske;dlpgemkvlvskek;lpgemkvlvskekd;p
gemkvlvskekdk;gemkvlvskekdkd;emkvlvskekdkdg;mkvlvskekdkdgk;kvlvskekdkdgky;vl
vskekdkdgkys;lvskekdkdgkysl;vskekdkdgkyslk;skekdkdgkyslka;kekdkdgkyslkat;ekdkdg
kyslkatv;kdkdgkyslkatvd;dkdgkyslkatvdk;kdgkyslkatvdki;dgkyslkatvdkie;gkyslkatvdkiel;
kyslkatvdkielk;yslkatvdkielkg;slkatvdkielkgt;lkatvdkielkgts;katvdkielkgtsd;atvdkielkgtsd
k;tvdkielkgtsdkd;vdkielkgtsdkdn;dkielkgtsdkdng;kielkgtsdkdngs;ielkgtsdkdngsg;elkgtsdk

dngsgv;lkgtsdkdngsgvl;kgtsdkdngsgvle;gtsdkdngsgvleg;tsdkdngsgvlegt;sdkdngsgvlegtk; dkdngsgvlegtkd;kdngsgvlegtkdd;dngsgvlegtkddk;ngsgvlegtkddks;gsgvlegtkddksk;sgvlegt kddkska;gvlegtkddkskak;vlegtkddkskakl;legtkddkskaklt;egtkddkskaklti;gtkddkskakltia;tk ddkskakltiad;kddkskakltiadd;ddkskakltiaddl;dkskakltiaddls;kskakltiaddlsk;skakltiaddlskt; kakltiaddlsktt;akltiaddlskttf;kltiaddlskttfe;ltiaddlskttfel;tiaddlskttfelf;iaddlskttfelfk;addlsk ttfelfke;ddlskttfelfked;dlskttfelfkedg;lskttfelfkedgk;skttfelfkedgkt;kttfelfkedgktl;ttfelfked gktlv;tfelfkedgktlvs;felfkedgktlvsr;elfkedgktlvsrk;lfkedgktlvsrkv;fkedgktlvsrkvs;kedgktlv srkvss;edgktlvsrkvssk;dgktlvsrkvsskd;gktlvsrkvsskdk;ktlvsrkvsskdkt;tlvsrkvsskdkts;lvsrk vsskdktst;vsrkvsskdktstd;srkvsskdktstde;rkvsskdktstdem;kvsskdktstdemf;vsskdktstdemfn ;sskdktstdemfne;skdktstdemfnek;kdktstdemfnekg;dktstdemfnekge;ktstdemfnekgel;tstdem fnekgels;stdemfnekgelsa;tdemfnekgelsak;demfnekgelsakt;emfnekgelsaktm;mfnekgelsakt mt;fnekgelsaktmtr;nekgelsaktmtre;ekgelsaktmtren;kgelsaktmtreng;gelsaktmtrengt;elsakt mtrengtk;lsaktmtrengtkl;saktmtrengtkle;aktmtrengtkley;ktmtrengtkleyt;tmtrengtkleyte;mt rengtkleytem;trengtkleytemk;rengtkleytemks;engtkleytemksd;ngtkleytemksdg;gtkleytem ksdgt;tkleytemksdgtg;kleytemksdgtgk;leytemksdgtgka;eytemksdgtgkak;ytemksdgtgkake; temksdgtgkakev;emksdgtgkakevl;mksdgtgkakevlk;ksdgtgkakevlkn;sdgtgkakevlknf;dgtgk akevlknft;gtgkakevlknftl;tgkakevlknftle;gkakevlknftleg;kakevlknftlegk;akevlknftlegkv;ke vlknftlegkva;evlknftlegkvan;vlknftlegkvand;lknftlegkvandk;knftlegkvandkv;nftlegkvand kvt;ftlegkvandkvtl;tlegkvandkvtle;legkvandkvtlev;egkvandkvtlevk;gkvandkvtlevke;kvan dkvtlevkeg;vandkvtlevkegt;andkvtlevkegtv;ndkvtlevkegtvt;dkvtlevkegtvtl;kvtlevkegtvtls; vtlevkegtvtlsk;tlevkegtvtlske;levkegtvtlskei;evkegtvtlskeia;vkegtvtlskeiak;kegtvtlskeiaks; egtvtlskeiaksg;gtvtlskeiaksge;tvtlskeiaksgev;vtlskeiaksgevt;tlskeiaksgevtv;lskeiaksgevtva ;skeiaksgevtval;keiaksgevtvaln;eiaksgevtvalnd;iaksgevtvalndt;aksgevtvalndtn;ksgevtvaln dtnt;sgevtvalndtntt;gevtvalndtnttq;evtvalndtnttqa;vtvalndtnttqat;tvalndtnttqatk;valndtnttqa tkk;alndtnttqatkkt;lndtnttqatkktg;ndtnttqatkktga;dtnttqatkktgaw;tnttqatkktgawd;nttqatkktg awds;ttqatkktgawdsk;tqatkktgawdskt;qatkktgawdskts;atkktgawdsktst;tkktgawdsktstl;kktg awdsktstlt;ktgawdsktstlti;tgawdsktstltis;gawdsktstltisv;awdsktstltisvn;wdsktstltisvns;dskt stltisvnsk;sktstltisvnskk;ktstltisvnskkt;tstltisvnskktt;stltisvnskkttq;tltisvnskkttql;ltisvnskkt tqlv;tisvnskkttqlvf;isvnskkttqlvft;svnskkttqlvftk;vnskkttqlvftkq;nskkttqlvftkqd;skkttqlvftk qdt;kkttqlvftkqdti;kttqlvftkqdtit;ttqlvftkqdtitv;tqlvftkqdtitvq;qlvftkqdtitvqk;lvftkqdtitvqky ;vftkqdtitvqkyd;ftkqdtitvqkyds;tkqdtitvqkydsa;kqdtitvqkydsag;qdtitvqkydsagt;dtitvqkyds agtn;titvqkydsagtnl;itvqkydsagtnle;tvqkydsagtnleg;vqkydsagtnlegt;qkydsagtnlegta;kydsa gtnlegtav;ydsagtnlegtave;dsagtnlegtavei;sagtnlegtaveik;agtnlegtaveikt;gtnlegtaveiktl;tnle gtaveiktld;nlegtaveiktlde;legtaveiktldel;egtaveiktldelk;gtaveiktldelkn;taveiktldelkna;aveik tldelknal;veiktldelknalk;

15 mers:

mkkyllgiglilali;kkyllgiglilalia;kyllgiglilaliac;yllgiglilaliack;llgiglilaliackq;lgiglilaliackqn; giglilaliackqnv;iglilaliackqnvs;glilaliackqnvss;lilaliackqnvssl;ilaliackqnvssld;laliackqnvss lde;aliackqnvssldek;liackqnvssldekn;iackqnvssldekns;ackqnvssldeknsa;ckqnvssldeknsas; kqnvssldeknsasv;qnvssldeknsasvd;nvssldeknsasvdl;vssldeknsasvdlp;ssldeknsasvdlpg;slde knsasvdlpge;ldeknsasvdlpgem;deknsasvdlpgemk;eknsasvdlpgemkv;knsasvdlpgemkvl;nsa svdlpgemkvlv;sasvdlpgemkvlvs;asvdlpgemkvlvsk;svdlpgemkvlvske;vdlpgemkvlvskek;dl pgemkvlvskekd;lpgemkvlvskekdk;pgemkvlvskekdkd;gemkvlvskekdkdg;emkvlvskekdkd gk;mkvlvskekdkdgky;kvlvskekdkdgkys;vlvskekdkdgkysl;lvskekdkdgkyslk;vskekdkdgky slka;skekdkdgkyslkat;kekdkdgkyslkatv;ekdkdgkyslkatvd;kdkdgkyslkatvdk;dkdgkyslkatv

dki;kdgkyslkatvdkie;dgkyslkatvdkiel;gkyslkatvdkielk;kyslkatvdkielkg;yslkatvdkielkgt;sl
katvdkielkgts;lkatvdkielkgtsd;katvdkielkgtsdk;atvdkielkgtsdkd;tvdkielkgtsdkdn;vdkielkgt
sdkdng;dkielkgtsdkdngs;kielkgtsdkdngsg;ielkgtsdkdngsgv;elkgtsdkdngsgvl;lkgtsdkdngsg
vle;kgtsdkdngsgvleg;gtsdkdngsgvlegt;tsdkdngsgvlegtk;sdkdngsgvlegtkd;dkdngsgvlegtkd
d;kdngsgvlegtkddk;dngsgvlegtkddks;ngsgvlegtkddksk;gsgvlegtkddkska;sgvlegtkddkskak
;gvlegtkddkskakl;vlegtkddkskaklt;legtkddkskaklti;egtkddkskakltia;gtkddkskakltiad;tkddk
skakltiadd;kddkskakltiaddl;ddkskakltiaddls;dkskakltiaddlsk;kskakltiaddlskt;skakltiaddlsk
tt;kakltiaddlskttf;akltiaddlskttfe;kltiaddlskttfel;ltiaddlskttfelf;tiaddlskttfelfk;iaddlskttfelfk
e;addlskttfelfked;ddlskttfelfkedg;dlskttfelfkedgk;lskttfelfkedgkt;skttfelfkedgktl;kttfelfked
gktlv;ttfelfkedgktlvs;tfelfkedgktlvsr;felfkedgktlvsrk;elfkedgktlvsrkv;lfkedgktlvsrkvs;fked
gktlvsrkvss;kedgktlvsrkvssk;edgktlvsrkvsskd;dgktlvsrkvsskdk;gktlvsrkvsskdkt;ktlvsrkvss
kdkts;tlvsrkvsskdktst;lvsrkvsskdktstd;vsrkvsskdktstde;srkvsskdktstdem;rkvsskdktstdemf;
kvsskdktstdemfn;vsskdktstdemfne;sskdktstdemfnek;skdktstdemfnekg;kdktstdemfnekge;d
ktstdemfnekgel;ktstdemfnekgels;tstdemfnekgelsa;stdemfnekgelsak;tdemfnekgelsakt;demf
nekgelsaktm;emfnekgelsaktmt;mfnekgelsaktmtr;fnekgelsaktmtre;nekgelsaktmtren;ekgels
aktmtreng;kgelsaktmtrengt;gelsaktmtrengtk;elsaktmtrengtkl;lsaktmtrengtkle;saktmtrengtk
ley;aktmtrengtkleyt;ktmtrengtkleyte;tmtrengtkleytem;mtrengtkleytemk;trengtkleytemks;r
engtkleytemksd;engtkleytemksdg;ngtkleytemksdgt;gtkleytemksdgtg;tkleytemksdgtgk;kle
ytemksdgtgka;leytemksdgtgkak;eytemksdgtgkake;ytemksdgtgkakev;temksdgtgkakevl;em
ksdgtgkakevlk;mksdgtgkakevlkn;ksdgtgkakevlknf;sdgtgkakevlknft;dgtgkakevlknftl;gtgka
kevlknftle;tgkakevlknftleg;gkakevlknftlegk;kakevlknftlegkv;akevlknftlegkva;kevlknftleg
kvan;evlknftlegkvand;vlknftlegkvandk;lknftlegkvandkv;knftlegkvandkvt;nftlegkvandkvtl
;ftlegkvandkvtle;tlegkvandkvtlev;legkvandkvtlevk;egkvandkvtlevke;gkvandkvtlevkeg;kv
andkvtlevkegt;vandkvtlevkegtv;andkvtlevkegtvt;ndkvtlevkegtvtl;dkvtlevkegtvtls;kvtlevk
egtvtlsk;vtlevkegtvtlske;tlevkegtvtlskei;levkegtvtlskeia;evkegtvtlskeiak;vkegtvtlskeiaks;k
egtvtlskeiaksg;egtvtlskeiaksge;gtvtlskeiaksgev;tvtlskeiaksgevt;vtlskeiaksgevtv;tlskeiaksg
evtva;lskeiaksgevtval;skeiaksgevtvaln;keiaksgevtvalnd;eiaksgevtvalndt;iaksgevtvalndtn;a
ksgevtvalndtnt;ksgevtvalndtntt;sgevtvalndtnttq;gevtvalndtnttqa;evtvalndtnttqat;vtvalndtnt
tqatk;tvalndtnttqatkk;valndtnttqatkkt;alndtnttqatkktg;lndtnttqatkktga;ndtnttqatkktgaw;dtnt
tqatkktgawd;tnttqatkktgawds;nttqatkktgawdsk;ttqatkktgawdskt;tqatkktgawdskts;qatkktga
wdsktst;atkktgawdsktstl;tkktgawdsktstlt;kktgawdsktstlti;ktgawdsktstltis;tgawdsktstltisv;g
awdsktstltisvn;awdsktstltisvns;wdsktstltisvnsk;dsktstltisvnskk;sktstltisvnskkt;ktstltisvnsk
ktt;tstltisvnskkttq;stltisvnskkttql;tltisvnskkttqlv;ltisvnskkttqlvft;tisvnskkttqlvft;isvnskkttql
vftk;svnskkttqlvftkq;vnskkttqlvftkqd;nskkttqlvftkqdt;skkttqlvftkqdti;kkttqlvftkqdtit;kttqlv
ftkqdtitv;ttqlvftkqdtitvq;tqlvftkqdtitvqk;qlvftkqdtitvqky;lvftkqdtitvqkyd;vftkqdtitvqkyds;f
tkqdtitvqkydsa;tkqdtitvqkydsag;kqdtitvqkydsagt;qdtitvqkydsagtn;dtitvqkydsagtnl;titvqky
dsagtnle;itvqkydsagtnleg;tvqkydsagtnlegt;vqkydsagtnlegta;qkydsagtnlegtav;kydsagtnlegt
ave;ydsagtnlegtavei;dsagtnlegtaveik;sagtnlegtaveikt;agtnlegtaveiktl;gtnlegtaveiktld;tnlegt
aveiktlde;nlegtaveiktldel;legtaveiktldelk;egtaveiktldelkn;gtaveiktldelkna;taveiktldelknal;a
veiktldelknalk;

16 mers:
mkkyllgiglilalia;kkyllgiglilaliac;kyllgiglilaliack;yllgiglilaliackq;llgiglilaliackqn;lgiglilalia
ckqnv;giglilaliackqnvs;iglilaliackqnvss;glilaliackqnvssl;lilaliackqnvssld;ilaliackqnvsslde;
laliackqnvssldek;aliackqnvssldekn;liackqnvssldekns;iackqnvssldeknsa;ackqnvssldeknsas;
ckqnvssldeknsasv;kqnvssldeknsasvd;qnvssldeknsasvdl;nvssldeknsasvdlp;vssldeknsasvdlp

g;ssldeknsasvdlpge;sldeknsasvdlpgem;ldeknsasvdlpgemk;deknsasvdlpgemkv;eknsasvdlp
gemkvl;knsasvdlpgemkvlv;nsasvdlpgemkvlvs;sasvdlpgemkvlvsk;asvdlpgemkvlvske;svdl
pgemkvlvskek;vdlpgemkvlvskekd;dlpgemkvlvskekdk;lpgemkvlvskekdkd;pgemkvlvskek
dkdg;gemkvlvskekdkdgk;emkvlvskekdkdgky;mkvlvskekdkdgkys;kvlvskekdkdgkysl;vlvs
kekdkdgkyslk;lvskekdkdgkyslka;vskekdkdgkyslkat;skekdkdgkyslkatv;kekdkdgkyslkatvd;
ekdkdgkyslkatvdk;kdkdgkyslkatvdki;dkdgkyslkatvdkie;kdgkyslkatvdkiel;dgkyslkatvdkiel
k;gkyslkatvdkielkg;kyslkatvdkielkgt;yslkatvdkielkgts;slkatvdkielkgtsd;lkatvdkielkgtsdk;k
atvdkielkgtsdkd;atvdkielkgtsdkdn;tvdkielkgtsdkdng;vdkielkgtsdkdngs;dkielkgtsdkdngsg;
kielkgtsdkdngsgv;ielkgtsdkdngsgvl;elkgtsdkdngsgvle;lkgtsdkdngsgvleg;kgtsdkdngsgvleg
t;gtsdkdngsgvlegtk;tsdkdngsgvlegtkd;sdkdngsgvlegtkdd;dkdngsgvlegtkddk;kdngsgvlegtk
ddks;dngsgvlegtkddksk;ngsgvlegtkddkska;gsgvlegtkddkskak;sgvlegtkddkskakl;gvlegtkdd
kskaklt;vlegtkddkskaklti;legtkddkskakltia;egtkddkskakltiad;gtkddkskakltiadd;tkddkskaklt
iaddl;kddkskakltiaddls;ddkskakltiaddlsk;dkskakltiaddlskt;kskakltiaddlsktt;skakltiaddlskttf
;kakltiaddlskttfe;akltiaddlskttfel;kltiaddlskttfelf;ltiaddlskttfelfk;tiaddlskttfelfke;iaddlskttfe
lfked;addlskttfelfkedg;ddlskttfelfkedgk;dlskttfelfkedgkt;lskttfelfkedgktl;skttfelfkedgktlv;
kttfelfkedgktlvs;ttfelfkedgktlvsr;tfelfkedgktlvsrk;felfkedgktlvsrkv;elfkedgktlvsrkvs;lfked
gktlvsrkvss;fkedgktlvsrkvssk;kedgktlvsrkvsskd;edgktlvsrkvsskdk;dgktlvsrkvsskdkt;gktlv
srkvsskdkts;ktlvsrkvsskdktst;tlvsrkvsskdktstd;lvsrkvsskdktstde;vsrkvsskdktstdem;srkvssk
dktstdemf;rkvsskdktstdemfn;kvsskdktstdemfne;vsskdktstdemfnek;sskdktstdemfnekg;skd
ktstdemfnekge;kdktstdemfnekgel;dktstdemfnekgels;ktstdemfnekgelsa;tstdemfnekgelsak;s
tdemfnekgelsakt;tdemfnekgelsaktm;demfnekgelsaktmt;emfnekgelsaktmtr;mfnekgelsaktm
tre;fnekgelsaktmtren;nekgelsaktmtreng;ekgelsaktmtrengt;kgelsaktmtrengtk;gelsaktmtreng
tkl;elsaktmtrengtkle;lsaktmtrengtkley;saktmtrengtkleyt;aktmtrengtkleyte;ktmtrengtkleyte
m;tmtrengtkleytemk;mtrengtkleytemks;trengtkleytemksd;rengtkleytemksdg;engtkleytem
ksdgt;ngtkleytemksdgtg;gtkleytemksdgtgk;tkleytemksdgtgka;kleytemksdgtgkak;leytemks
dgtgkake;eytemksdgtgkakev;ytemksdgtgkakevl;temksdgtgkakevlk;emksdgtgkakevlkn;m
ksdgtgkakevlknf;ksdgtgkakevlknft;sdgtgkakevlknftl;dgtgkakevlknftle;gtgkakevlknftleg;t
gkakevlknftlegk;gkakevlknftlegkv;kakevlknftlegkva;akevlknftlegkvan;kevlknftlegkvand;
evlknftlegkvandk;vlknftlegkvandkv;lknftlegkvandkvt;knftlegkvandkvtl;nftlegkvandkvtle;
ftlegkvandkvtlev;tlegkvandkvtlevk;legkvandkvtlevke;egkvandkvtlevkeg;gkvandkvtlevke
gt;kvandkvtlevkegtv;vandkvtlevkegtvt;andkvtlevkegtvtl;ndkvtlevkegtvtls;dkvtlevkegtvtls
k;kvtlevkegtvtlske;vtlevkegtvtlskei;tlevkegtvtlskeia;levkegtvtlskeiak;evkegtvtlskeiaks;vk
egtvtlskeiaksg;kegtvtlskeiaksge;egtvtlskeiaksgev;gtvtlskeiaksgevt;tvtlskeiaksgevtv;vtlske
iaksgevtva;tlskeiaksgevtval;lskeiaksgevtvaln;skeiaksgevtvalnd;keiaksgevtvalndt;eiaksgev
tvalndtn;iaksgevtvalndtnt;aksgevtvalndtntt;ksgevtvalndtnttq;sgevtvalndtnttqa;gevtvalndtn
ttqat;evtvalndtnttqatk;vtvalndtnttqatkk;tvalndtnttqatkkt;valndtnttqatkktg;alndtnttqatkktga;
lndtnttqatkktgaw;ndtnttqatkktgawd;dtnttqatkktgawds;tnttqatkktgawdsk;nttqatkktgawdskt;
ttqatkktgawdskts;tqatkktgawdsktst;qatkktgawdsktstl;atkktgawdsktstlt;tkktgawdsktstlti;kkt
gawdsktstltis;ktgawdsktstltisv;tgawdsktstltisvn;gawdsktstltisvns;awdsktstltisvnsk;wdsktst
ltisvnskk;dsktstltisvnskkt;sktstltisvnskktt;ktstltisvnskkttq;tstltisvnskkttql;stltisvnskkttqlv;t
ltisvnskkttqlvf;ltisvnskkttqlvft;tisvnskkttqlvftk;isvnskkttqlvftkq;svnskkttqlvftkqd;vnskktt
qlvftkqdt;nskkttqlvftkqdti;skkttqlvftkqdtit;kkttqlvftkqdtitv;kttqlvftkqdtitvq;ttqlvftkqdtitvq
k;tqlvftkqdtitvqky;qlvftkqdtitvqkyd;lvftkqdtitvqkyds;vftkqdtitvqkydsa;ftkqdtitvqkydsag;t
kqdtitvqkydsagt;kqdtitvqkydsagtn;qdtitvqkydsagtnl;dtitvqkydsagtnle;titvqkydsagtnleg;itv
qkydsagtnlegt;tvqkydsagtnlegta;vqkydsagtnlegtav;qkydsagtnlegtave;kydsagtnlegtavei;yd
sagtnlegtaveik;dsagtnlegtaveikt;sagtnlegtaveiktl;agtnlegtaveiktld;gtnlegtaveiktlde;tnlegta

veiktldel;nlegtaveiktldelk;legtaveiktldelkn;egtaveiktldelkna;gtaveiktldelknal;taveiktldelk
nalk;

**<CAA59727 outer surface protein A;Protein;Borrelia garinii>**

8 mers:
mkkyllgi;kkyllgig;kyllgigl;yllgigli;llgiglil;lgiglila;giglilal;iglilali;glilalia;lilaliac;ilaliack;l
aliackq;aliackqn;liackqnv;iackqnvs;ackqnvss;ckqnvssl;kqnvssld;qnvsslde;nvssldek;vsslde
kn;ssldekns;sldeknsv;ldeknsvs;deknsvsv;eknsvsvd;knsvsvdl;nsvsvdlp;svsvdlpg;vsvdlpgg;
svdlpggm;vdlpggmk;dlpggmkv;lpggmkvl;pggmkvlv;ggmkvlvs;gmkvlvsk;mkvlvske;kvlv
skek;vlvskekd;lvskekdk;vskekdkd;skekdkdg;kekdkdgk;ekdkdgky;kdkdgkys;dkdgkysl;kd
gkyslm;dgkyslma;gkyslmat;kyslmatv;yslmatve;slmatvek;lmatvekl;matvekle;atveklel;tvek
lelk;veklelkg;eklelkgt;klelkgts;lelkgtsd;elkgtsdk;lkgtsdkn;kgtsdknn;gtsdknng;tsdknngs;sd
knngsg;dknngsgt;knngsgtl;nngsgtle;ngsgtleg;gsgtlege;sgtlegek;gtlegekt;tlegektd;legektdk
;egektdks;gektdksk;ektdkskv;ktdkskvk;tdkskvkl;dkskvklt;kskvklti;skvkltia;kvkltiae;vkltia
ed;kltiaedl;ltiaedls;tiaedlsk;iaedlskt;aedlsktt;edlskttf;dlskttfe;lskttfei;skttfeif;kttfeifk;ttfeif
ke;tfeifked;feifkedg;eifkedgk;ifkedgkt;fkedgktl;kedgktlv;edgktlvs;dgktlvsk;gktlvskk;ktlv
skkv;tlvskkvt;lvskkvtl;vskkvtlk;skkvtlkd;kkvtlkdk;kvtlkdks;vtlkdkss;tlkdksst;lkdksste;kd
ksstee;dkssteek;kssteekf;ssteekfn;steekfne;teekfnek;eekfnekg;ekfnekge;kfnekgei;fnekgeis
;nekgeise;ekgeisek;kgeisekt;geisekti;eisektiv;isektivr;sektivra;ektivran;ktivrang;tivrangt;i
vrangtr;vrangtrl;rangtrle;angtrley;ngtrleyt;gtrleytd;trleytdi;rleytdik;leytdiks;eytdiksd;ytdi
ksdg;tdiksdgs;diksdgsg;iksdgsgk;ksdgsgka;sdgsgkak;dgsgkake;gsgkakev;sgkakevl;gkake
vlk;kakevlkd;akevlkdf;kevlkdft;evlkdftl;vlkdftle;lkdftleg;kdftlegt;dftlegtl;ftlegtla;tlegtlaa;
legtlaad;egtlaadg;gtlaadgk;tlaadgkt;laadgktt;aadgkttl;adgkttlk;dgkttlkv;gkttlkvt;kttlkvte;tt
lkvteg;tlkvtegt;lkvtegtv;kvtegtvv;vtegtvvl;tegtvvls;egtvvlsk;gtvvlskn;tvvlskni;vvlsknil;vls
knilk;lsknilks;sknilksg;knilksge;nilksgei;ilksgeit;lksgeitv;ksgeitva;sgeitval;geitvald;eitval
dd;itvaldds;tvalddsd;valddsdt;alddsdtt;lddsdttq;ddsdttqa;dsdttqat;sdttqatk;dttqatkk;ttqatkk
t;tqatkktg;qatkktgk;atkktgkw;tkktgkwd;kktgkwds;ktgkwdsk;tgkwdskt;gkwdskts;kwdsktst
;wdsktstl;dsktstlt;sktstlti;ktstltis;tstltisv;stltisvn;tltisvns;ltisvnsq;tisvnsqk;isvnsqkt;svnsqkt
k;vnsqktkn;nsqktknl;sqktknlv;qktknlvf;ktknlvft;tknlvftk;knlvftke;nlvftked;lvftkedt;vftked
ti;ftkedtit;tkedtitv;kedtitvq;edtitvqk;dtitvqky;titvqkyd;itvqkyds;tvqkydsa;vqkydsag;qkyds
agt;kydsagtn;ydsagtnl;dsagtnle;sagtnleg;agtnlegk;gtnlegka;tnlegkav;nlegkave;legkavei;eg
kaveit;gkaveitt;kaveittl;aveittle;veittlek;eittlekl;ittleklk;ttleklkd;tleklkda;leklkdal;eklkdalk
;

9 mers:
mkkyllgig;kkyllgigl;kyllgigli;yllgiglil;llgiglila;lgiglilal;giglilali;iglilalia;glilaliac;lilaliack;
ilaliackq;laliackqn;aliackqnv;liackqnvs;iackqnvss;ackqnvssl;ckqnvssld;kqnvsslde;qnvssld
ek;nvssldekn;vssldekns;ssldeknsv;sldeknsvs;ldeknsvsv;deknsvsvd;eknsvsvdl;knsvsvdlp;n
svsvdlpg;svsvdlpgg;vsvdlpggm;svdlpggmk;vdlpggmkv;dlpggmkvl;lpggmkvlv;pggmkvlv
s;ggmkvlvsk;gmkvlvske;mkvlvskek;kvlvskekd;vlvskekdk;lvskekdkd;vskekdkdg;skekdkd
gk;kekdkdgky;ekdkdgkys;kdkdgkysl;dkdgkyslm;kdgkyslma;dgkyslmat;gkyslmatv;kyslm
atve;yslmatvek;slmatvekl;lmatvekle;matveklel;atveklelk;tveklelkg;veklelkgt;eklelkgts;kle
lkgtsd;lelkgtsdk;elkgtsdkn;lkgtsdknn;kgtsdknng;gtsdknngs;tsdknngsg;sdknngsgt;dknngsg
tl;knngsgtle;nngsgtleg;ngsgtlege;gsgtlegek;sgtlegekt;gtlegektd;tlegektdk;legektdks;egekt
dksk;gektdkskv;ektdkskvk;ktdkskvkl;tdkskvklt;dkskvklti;kskvkltia;skvkltiae;kvkltiaed;vk

ltiaedl;kltiaedls;ltiaedlsk;tiaedlskt;iaedlsktt;aedlskttf;edlskttfe;dlskttfei;lskttfeif;skttfeifk;k
ttfeifke;ttfeifked;tfeifkedg;feifkedgk;eifkedgkt;ifkedgktl;fkedgktlv;kedgktlvs;edgktlvsk;d
gktlvskk;gktlvskkv;ktlvskkvt;tlvskkvtl;lvskkvtlk;vskkvtlkd;skkvtlkdk;kkvtlkdks;kvtlkdks
s;vtlkdksst;tlkdksste;lkdksstee;kdkssteek;dkssteekf;kssteekfn;ssteekfne;steekfnek;teekfne
kg;eekfnekge;ekfnekgei;kfnekgeis;fnekgeise;nekgeisek;ekgeisekt;kgeisekti;geisektiv;eise
ktivr;isektivra;sektivran;ektivrang;ktivrangt;tivrangtr;ivrangtrl;vrangtrle;rangtrley;angtrle
yt;ngtrleytd;gtrleytdi;trleytdik;rleytdiks;leytdiksd;eytdiksdg;ytdiksdgs;tdiksdgsg;diksdgsg
k;iksdgsgka;ksdgsgkak;sdgsgkake;dgsgkakev;gsgkakevl;sgkakevlk;gkakevlkd;kakevlkdf;
akevlkdft;kevlkdftl;evlkdftle;vlkdftleg;lkdftlegt;kdftlegtl;dftlegtla;ftlegtlaa;tlegtlaad;legtl
aadg;egtlaadgk;gtlaadgkt;tlaadgktt;laadgkttl;aadgkttlk;adgkttlkv;dgkttlkvt;gkttlkvte;kttlkv
teg;ttlkvtegt;tlkvtegtv;lkvtegtvv;kvtegtvvl;vtegtvvls;tegtvvlsk;egtvvlskn;gtvvlskni;tvvlsk
nil;vvlsknilk;vlsknilks;lsknilksg;sknilksge;knilksgei;nilksgeit;ilksgeitv;lksgeitva;ksgeitva
l;sgeitvald;geitvaldd;eitvaldds;itvalddsd;tvalddsdt;valddsdtt;alddsdttq;lddsdttqa;ddsdttqat;
dsdttqatk;sdttqatkk;dttqatkkt;ttqatkktg;tqatkktgk;qatkktgkw;atkktgkwd;tkktgkwds;kktgk
wdsk;ktgkwdskt;tgkwdskts;gkwdsktst;kwdsktstl;wdsktstlt;dsktstlti;sktstltis;ktstltisv;tstltis
vn;stltisvns;tltisvnsq;ltisvnsqk;tisvnsqkt;isvnsqktk;svnsqktkn;vnsqktknl;nsqktknlv;sqktkn
lvf;qktknlvft;ktknlvftk;tknlvftke;knlvftked;nlvftkedt;lvftkedti;vftkedtit;ftkedtitv;tkedtitvq;
kedtitvqk;edtitvqky;dtitvqkyd;titvqkyds;itvqkydsa;tvqkydsag;vqkydsagt;qkydsagtn;kydsa
gtnl;ydsagtnle;dsagtnleg;sagtnlegk;agtnlegka;gtnlegkav;tnlegkave;nlegkavei;legkaveit;eg
kaveitt;gkaveittl;kaveittle;aveittlek;veittlekl;eittleklk;ittleklkd;ttleklkda;tleklkdal;leklkdal
k;

10 mers:
mkkyllgigl;kkyllgigli;kyllgiglil;yllgiglila;llgiglilal;lgiglilali;giglilalia;iglilaliac;glilaliack;l
ilaliackq;ilaliackqn;laliackqnv;aliackqnvs;liackqnvss;iackqnvssl;ackqnvssld;ckqnvsslde;k
qnvssldek;qnvssldekn;nvssldekns;vssldeknsv;ssldeknsvs;sldeknsvsv;ldeknsvsvd;deknsvs
vdl;eknsvsvdlp;knsvsvdlpg;nsvsvdlpgg;svsvdlpggm;vsvdlpggmk;svdlpggmkv;vdlpggmk
vl;dlpggmkvlv;lpggmkvlvs;pggmkvlvsk;ggmkvlvske;gmkvlvskek;mkvlvskekd;kvlvskek
dk;vlvskekdkd;lvskekdkdg;vskekdkdgk;skekdkdgky;kekdkdgkys;ekdkdgkysl;kdkdgkysl
m;dkdgkyslma;kdgkyslmat;dgkyslmatv;gkyslmatve;kyslmatvek;yslmatvekl;slmatvekle;l
matveklel;matveklelk;atveklelkg;tveklelkgt;veklelkgts;eklelkgtsd;klelkgtsdk;lelkgtsdkn;e
lkgtsdknn;lkgtsdknng;kgtsdknngs;gtsdknngsg;tsdknngsgt;sdknngsgtl;dknngsgtle;knngsgtl
eg;nngsgtlege;ngsgtlegek;gsgtlegekt;sgtlegektd;gtlegektdk;tlegektdks;legektdksk;egektdk
skv;gektdkskvk;ektdkskvkl;ktdkskvklt;tdkskvklti;dkskvkltia;kskvkltiae;skvkltiaed;kvkltia
edl;vkltiaedls;kltiaedlsk;ltiaedlskt;tiaedlskttt;iaedlskttf;aedlskttfe;edlskttfei;dlskttfeif;lskttf
eifk;skttfeifke;kttfeifked;ttfeifkedg;tfeifkedgk;feifkedgkt;eifkedgktl;ifkedgktlv;fkedgktlvs
;kedgktlvsk;edgktlvskk;dgktlvskkv;gktlvskkvt;ktlvskkvtl;tlvskkvtlk;lvskkvtlkd;vskkvtlkd
k;skkvtlkdks;kkvtlkdkss;kvtlkdksst;vtlkdksste;tlkdksstee;lkdkssteek;kdkssteekf;dkssteekf
n;kssteekfne;ssteekfnek;steekfnekg;teekfnekge;eekfnekgei;ekfnekgeis;kfnekgeise;fnekgei
sek;nekgeisekt;ekgeisekti;kgeisektiv;geisektivr;eisektivra;isektivran;sektivrang;ektivrangt
;ktivrangtr;tivrangtrl;ivrangtrle;vrangtrley;rangtrleyt;angtrleytd;ngtrleytdi;gtrleytdik;trleyt
diks;rleytdiksd;leytdiksdg;eytdiksdgs;ytdiksdgsg;tdiksdgsgk;diksdgsgka;iksdgsgkak;ksdg
sgkake;sdgsgkakev;dgsgkakevl;gsgkakevlk;sgkakevlkd;gkakevlkdf;kakevlkdft;akevlkdftl
;kevlkdftle;evlkdftleg;vlkdftlegt;lkdftlegtl;kdftlegtla;dftlegtlaa;ftlegtlaad;tlegtlaadg;legtla
adgk;egtlaadgkt;gtlaadgktt;tlaadgkttl;laadgkttlk;aadgkttlkv;adgkttlkvt;dgkttlkvte;gkttlkvte
g;kttlkvtegt;ttlkvtegtv;tlkvtegtvv;lkvtegtvvl;kvtegtvvls;vtegtvvlsk;tegtvvlskn;egtvvlskni;g

tvvlsknil;tvvlsknilk;vvlsknilks;vlsknilksg;lsknilksge;sknilksgei;knilksgeit;nilksgeitv;ilksg
eitva;lksgeitval;ksgeitvald;sgeitvaldd;geitvaldds;eitvalddsd;itvalddsdt;tvalddsdtt;valddsdt
tq;alddsdttqa;lddsdttqat;ddsdttqatk;dsdttqatkk;sdttqatkkt;dttqatkktg;ttqatkktgk;tqatkktgkw
;qatkktgkwd;atkktgkwds;tkktgkwdsk;kktgkwdskt;ktgkwdskts;tgkwdsktst;gkwdsktstl;kwd
sktstlt;wdsktstlti;dsktstltis;sktstltisv;ktstltisvn;tstltisvns;stltisvnsq;tltisvnsqk;ltisvnsqkt;tis
vnsqktk;isvnsqktkn;svnsqktknl;vnsqktknlv;nsqktknlvf;sqktknlvft;qktknlvftk;ktknlvftke;tk
nlvftked;knlvftkedt;nlvftkedti;lvftkedtit;vftkedtitv;ftkedtitvq;tkedtitvqk;kedtitvqky;edtitv
qkyd;dtitvqkyds;titvqkydsa;itvqkydsag;tvqkydsagt;vqkydsagtn;qkydsagtnl;kydsagtnle;yd
sagtnleg;dsagtnlegk;sagtnlegka;agtnlegkav;gtnlegkave;tnlegkavei;nlegkaveit;legkaveitt;e
gkaveittl;gkaveittle;kaveittlek;aveittlekl;veittleklk;eittleklkd;ittleklkda;ttleklkdal;tleklkdal
k;

11 mers:
mkkyllgigli;kkyllgiglil;kyllgiglila;yllgiglilal;llgiglilali;lgiglilalia;giglilaliac;iglilaliack;glil
aliackq;lilaliackqn;ilaliackqnv;laliackqnvs;aliackqnvss;liackqnvssl;iackqnvssld;ackqnvssl
de;ckqnvssldek;kqnvssldekn;qnvssldekns;nvssldeknsv;vssldeknsvs;ssldeknsvsv;sldeknsv
svd;ldeknsvsvdl;deknsvsvdlp;eknsvsvdlpg;knsvsvdlpgg;nsvsvdlpggm;svsvdlpggmk;vsvd
lpggmkv;svdlpggmkvl;vdlpggmkvlv;dlpggmkvlvs;lpggmkvlvsk;pggmkvlvske;ggmkvlvs
kek;gmkvlvskekd;mkvlvskekdk;kvlvskekdkd;vlvskekdkdg;lvskekdkdgk;vskekdkdgky;sk
ekdkdgkys;kekdkdgkysl;ekdkdgkyslm;kdkdgkyslma;dkdgkyslmat;kdgkyslmatv;dgkyslm
atve;gkyslmatvek;kyslmatvekl;yslmatvekle;slmatveklel;lmatveklelk;matveklelkg;atveklel
kgt;tveklelkgts;veklelkgtsd;eklelkgtsdk;klelkgtsdkn;lelkgtsdknn;elkgtsdknng;lkgtsdknngs
;kgtsdknngsg;gtsdknngsgt;tsdknngsgtl;sdknngsgtle;dknngsgtleg;knngsgtlege;nngsgtlegek
;ngsgtlegekt;gsgtlegektd;sgtlegektdk;gtlegektdks;tlegektdksk;legektdkskv;egektdkskvk;g
ektdkskvkl;ektdkskvklt;ktdkskvklti;tdkskvkltia;dkskvkltiae;kskvkltiaed;skvkltiaedl;kvklti
aedls;vkltiaedlsk;kltiaedlskt;ltiaedlsktt;tiaedlskttf;iaedlskttfe;aedlskttfei;edlskttfeif;dlskttf
eifk;lskttfeifke;skttfeifked;kttfeifkedg;ttfeifkedgk;tfeifkedgkt;feifkedgktl;eifkedgktlv;ifke
dgktlvs;fkedgktlvsk;kedgktlvskk;edgktlvskkv;dgktlvskkvt;gktlvskkvtl;ktlvskkvtlk;tlvskk
vtlkd;lvskkvtlkdk;vskkvtlkdks;skkvtlkdkss;kkvtlkdksst;kvtlkdksste;vtlkdksstee;tlkdksstee
k;lkdkssteekf;kdkssteekfn;dkssteekfne;kssteekfnek;ssteekfnekg;steekfnekge;teekfnekgei;
eekfnekgeis;ekfnekgeise;kfnekgeisek;fnekgeisekt;nekgeisekti;ekgeisektiv;kgeisektivr;gei
sektivra;eisektivran;isektivrang;sektivrangt;ektivrangtr;ktivrangtrl;tivrangtrle;ivrangtrley;
vrangtrleyt;rangtrleytd;angtrleytdi;ngtrleytdik;gtrleytdiks;trleytdiksd;rleytdiksdg;leytdiks
dgs;eytdiksdgsg;ytdiksdgsgk;tdiksdgsgka;diksdgsgkak;iksdgsgkake;ksdgsgkakev;sdgsgk
akevl;dgsgkakevlk;gsgkakevlkd;sgkakevlkdf;gkakevlkdft;kakevlkdftl;akevlkdftle;kevlkdf
tleg;evlkdftlegt;vlkdftlegtl;lkdftlegtla;kdftlegtlaa;dftlegtlaad;ftlegtlaadg;tlegtlaadgk;legtla
adgkt;egtlaadgktt;gtlaadgkttl;tlaadgkttlk;laadgkttlkv;aadgkttlkvt;adgkttlkvte;dgkttlkvteg;g
kttlkvtegt;kttlkvtegtv;ttlkvtegtvv;tlkvtegtvvl;lkvtegtvvls;kvtegtvvlsk;vtegtvvlskn;tegtvvls
kni;egtvvlsknil;gtvvlsknilk;tvvlsknilks;vvlsknilksg;vlsknilksge;lsknilksgei;sknilksgeit;kni
lksgeitv;nilksgeitva;ilksgeitval;lksgeitvald;ksgeitvaldd;sgeitvaldds;geitvalddsd;eitvalddsd
t;itvalddsdtt;tvalddsdttq;valddsdttqa;alddsdttqat;lddsdttqatk;ddsdttqatkk;dsdttqatkkt;sdttq
atkktg;dttqatkktgk;ttqatkktgkw;tqatkktgkwd;qatkktgkwds;atkktgkwdsk;tkktgkwdskt;kktg
kwdskts;ktgkwdsktst;tgkwdsktstl;gkwdsktstlt;kwdsktstlti;wdsktstltis;dsktstltisv;sktstltisv
n;ktstltisvns;tstltisvnsq;stltisvnsqk;tltisvnsqkt;ltisvnsqktk;tisvnsqktkn;isvnsqktknl;svnsqkt
knlv;vnsqktknlvf;nsqktknlvft;sqktknlvftk;qktknlvftke;ktknlvftked;tknlvftkedt;knlvftkedti;
nlvftkedtit;lvftkedtitv;vftkedtitvq;ftkedtitvqk;tkedtitvqky;kedtitvqkyd;edtitvqkyds;dtitvqk

ydsa;titvqkydsag;itvqkydsagt;tvqkydsagtn;vqkydsagtnl;qkydsagtnle;kydsagtnleg;ydsagtnl
egk;dsagtnlegka;sagtnlegkav;agtnlegkave;gtnlegkavei;tnlegkaveit;nlegkaveitt;legkaveittl;
egkaveittle;gkaveittlek;kaveittlekl;aveittleklk;veittleklkd;eittleklkda;ittleklkdal;ttleklkdal
k;

13 mers:
mkkyllgiglila;kkyllgiglilal;kyllgiglilali;yllgiglilalia;llgiglilaliac;lgiglilaliack;giglilaliackq;
iglilaliackqn;glilaliackqnv;lilaliackqnvs;ilaliackqnvss;laliackqnvssl;aliackqnvssld;liackqn
vsslde;iackqnvssldek;ackqnvssldekn;ckqnvssldekns;kqnvssldeknsv;qnvssldeknsvs;nvssld
eknsvsv;vssldeknsvsvd;ssldeknsvsvdl;sldeknsvsvdlp;ldeknsvsvdlpg;deknsvsvdlpgg;ekns
vsvdlpggm;knsvsvdlpggmk;nsvsvdlpggmkv;svsvdlpggmkvl;vsvdlpggmkvlv;svdlpggmkv
lvs;vdlpggmkvlvsk;dlpggmkvlvske;lpggmkvlvskek;pggmkvlvskekd;ggmkvlvskekdk;gmk
vlvskekdkd;mkvlvskekdkdg;kvlvskekdkdgk;vlvskekdkdgky;lvskekdkdgkys;vskekdkdgky
sl;skekdkdgkyslm;kekdkdgkyslma;ekdkdgkyslmat;kdkdgkyslmatv;dkdgkyslmatve;kdgky
slmatvek;dgkyslmatvekl;gkyslmatvekle;kyslmatveklel;yslmatveklelk;slmatveklelkg;lmat
veklelkgt;matveklelkgts;atveklelkgtsd;tveklelkgtsdk;veklelkgtsdkn;eklelkgtsdknn;klelkgt
sdknng;lelkgtsdknngs;elkgtsdknngsg;lkgtsdknngsgt;kgtsdknngsgtl;gtsdknngsgtle;tsdknng
sgtleg;sdknngsgtlege;dknngsgtlegek;knngsgtlegekt;nngsgtlegektd;ngsgtlegektdk;gsgtlege
ktdks;sgtlegektdksk;gtlegektdkskv;tlegektdkskvk;legektdkskvkl;egektdkskvklt;gektdkskv
klti;ektdkskvkltia;ktdkskvkltiae;tdkskvkltiaed;dkskvkltiaedl;kskvkltiaedls;skvkltiaedlsk;k
vkltiaedlskt;vkltiaedlsktt;kltiaedlskttf;ltiaedlskttfe;tiaedlskttfei;iaedlskttfeif;aedlskttfeifk;e
dlskttfeifke;dlskttfeifked;lskttfeifkedg;skttfeifkedgk;kttfeifkedgkt;ttfeifkedgktl;tfeifkedgk
tlv;feifkedgktlvs;eifkedgktlvsk;ifkedgktlvskk;fkedgktlvskkv;kedgktlvskkvt;edgktlvskkvtl
;dgktlvskkvtlk;gktlvskkvtlkd;ktlvskkvtlkdk;tlvskkvtlkdks;lvskkvtlkdkss;vskkvtlkdksst;sk
kvtlkdksste;kkvtlkdksstee;kvtlkdkssteek;vtlkdkssteekf;tlkdkssteekfn;lkdkssteekfne;kdksst
eekfnek;dkssteekfnekg;kssteekfnekge;ssteekfnekgei;steekfnekgeis;teekfnekgeise;eekfnek
geisek;ekfnekgeisekt;kfnekgeisekti;fnekgeisektiv;nekgeisektivr;ekgeisektivra;kgeisektivr
an;geisektivrang;eisektivrangt;isektivrangtr;sektivrangtrl;ektivrangtrle;ktivrangtrley;tivra
ngtrleyt;ivrangtrleytd;vrangtrleytdi;rangtrleytdik;angtrleytdiks;ngtrleytdiksd;gtrleytdiksd
g;trleytdiksdgs;rleytdiksdgsg;leytdiksdgsgk;eytdiksdgsgka;ytdiksdgsgkak;tdiksdgsgkake;
diksdgsgkakev;iksdgsgkakevl;ksdgsgkakevlk;sdgsgkakevlkd;dgsgkakevlkdf;gsgkakevlkd
ft;sgkakevlkdftl;gkakevlkdftle;kakevlkdftleg;akevlkdftlegt;kevlkdftlegtl;evlkdftlegtla;vlk
dftlegtlaa;lkdftlegtlaad;kdftlegtlaadg;dftlegtlaadgk;ftlegtlaadgkt;tlegtlaadgktt;legtlaadgktt
l;egtlaadgkttlk;gtlaadgkttlkv;tlaadgkttlkvt;laadgkttlkvte;aadgkttlkvteg;adgkttlkvtegt;dgktt
lkvtegtv;gkttlkvtegtvv;kttlkvtegtvvl;ttlkvtegtvvls;tlkvtegtvvlsk;lkvtegtvvlskn;kvtegtvvlsk
ni;vtegtvvlsknil;tegtvvlsknilk;egtvvlsknilks;gtvvlsknilksg;tvvlsknilksge;vvlsknilksgei;vls
knilksgeit;lsknilksgeitv;sknilksgeitva;knilksgeitval;nilksgeitvald;ilksgeitvaldd;lksgeitvald
ds;ksgeitvalddsd;sgeitvalddsdt;geitvalddsdtt;eitvalddsdttq;itvalddsdttqa;tvalddsdttqat;vald
dsdttqatk;alddsdttqatkk;lddsdttqatkkt;ddsdttqatkktg;dsdttqatkktgk;sdttqatkktgkw;dttqatkk
tgkwd;ttqatkktgkwds;tqatkktgkwdsk;qatkktgkwdskt;atkktgkwdskts;tkktgkwdsktst;kktgk
wdsktstl;ktgkwdsktstlt;tgkwdsktstlti;gkwdsktstltis;kwdsktstltisv;wdsktstltisvn;dsktstltisvn
s;sktstltisvnsq;ktstltisvnsqk;tstltisvnsqkt;stltisvnsqktk;tltisvnsqktkn;ltisvnsqktknl;tisvnsqk
tknlv;isvnsqktknlvf;svnsqktknlvft;vnsqktknlvftk;nsqktknlvftke;sqktknlvftked;qktknlvftke
dt;ktknlvftkedti;tknlvftkedtit;knlvftkedtitv;nlvftkedtitvq;lvftkedtitvqk;vftkedtitvqky;ftked
titvqkyd;tkedtitvqkyds;kedtitvqkydsa;edtitvqkydsag;dtitvqkydsagt;titvqkydsagtn;itvqkyds
agtnl;tvqkydsagtnle;vqkydsagtnleg;qkydsagtnlegk;kydsagtnlegka;ydsagtnlegkav;dsagtnle

gkave;sagtnlegkavei;agtnlegkaveit;gtnlegkaveitt;tnlegkaveittl;nlegkaveittle;legkaveittlek;
egkaveittlekl;gkaveittleklk;kaveittleklkd;aveittleklkda;veittleklkdal;eittleklkdalk;

14 mers:

mkkyllgiglilal;kkyllgiglilali;kyllgiglilalia;yllgiglilaliac;llgiglilaliack;lgiglilaliackq;giglilal
iackqn;iglilaliackqnv;glilaliackqnvs;lilaliackqnvss;ilaliackqnvssl;laliackqnvssld;aliackqn
vsslde;liackqnvssldek;iackqnvssldekn;ackqnvssldekns;ckqnvssldeknsv;kqnvssldeknsvs;q
nvssldeknsvsv;nvssldeknsvsvd;vssldeknsvsvdl;ssldeknsvsvdlp;sldeknsvsvdlpg;ldeknsvsv
dlpgg;deknsvsvdlpggm;eknsvsvdlpggmk;knsvsvdlpggmkv;nsvsvdlpggmkvl;svsvdlpggm
kvlv;vsvdlpggmkvlvs;svdlpggmkvlvsk;vdlpggmkvlvske;dlpggmkvlvskek;lpggmkvlvskek
d;pggmkvlvskekdk;ggmkvlvskekdkd;gmkvlvskekdkdg;mkvlvskekdkdgk;kvlvskekdkdgk
y;vlvskekdkdgkys;lvskekdkdgkysl;vskekdkdgkyslm;skekdkdgkyslma;kekdkdgkyslmat;ek
dkdgkyslmatv;kdkdgkyslmatve;dkdgkyslmatvek;kdgkyslmatvekl;dgkyslmatvekle;gkyslm
atveklel;kyslmatveklelk;yslmatveklelkg;slmatveklelkgt;lmatveklelkgts;matveklelkgtsd;at
veklelkgtsdk;tveklelkgtsdkn;veklelkgtsdknn;eklelkgtsdknng;klelkgtsdknngs;lelkgtsdknng
sg;elkgtsdknngsgt;lkgtsdknngsgtl;kgtsdknngsgtle;gtsdknngsgtleg;tsdknngsgtlege;sdknngs
gtlegek;dknngsgtlegekt;knngsgtlegektd;nngsgtlegektdk;ngsgtlegektdks;gsgtlegektdksk;sg
tlegektdkskv;gtlegektdkskvk;tlegektdkskvkl;legektdkskvklt;egektdkskvklti;gektdkskvklti
a;ektdkskvkltiae;ktdkskvkltiaed;tdkskvkltiaedl;dkskvkltiaedls;kskvkltiaedlsk;skvkltiaedls
kt;kvkltiaedlsktt;vkltiaedlskttf;kltiaedlskttfe;ltiaedlskttfei;tiaedlskttfeif;iaedlskttfeifk;aedl
skttfeifke;edlskttfeifked;dlskttfeifkedg;lskttfeifkedgk;skttfeifkedgkt;kttfeifkedgktl;ttfeifke
dgktlv;tfeifkedgktlvs;feifkedgktlvsk;eifkedgktlvskk;ifkedgktlvskkv;fkedgktlvskkvt;kedgk
tlvskkvtl;edgktlvskkvtlk;dgktlvskkvtlkd;gktlvskkvtlkdk;ktlvskkvtlkdks;tlvskkvtlkdkss;lvs
kkvtlkdksst;vskkvtlkdksste;skkvtlkdksstee;kkvtlkdkssteek;kvtlkdkssteekf;vtlkdkssteekfn;
tlkdkssteekfne;lkdkssteekfnek;kdkssteekfnekg;dkssteekfnekge;kssteekfnekgei;ssteekfnek
geis;steekfnekgeise;teekfnekgeisek;eekfnekgeisekt;ekfnekgeisekti;kfnekgeisektiv;fnekgei
sektivr;nekgeisektivra;ekgeisektivran;kgeisektivrang;geisektivrangt;eisektivrangtr;isektiv
rangtrl;sektivrangtrle;ektivrangtrley;ktivrangtrleyt;tivrangtrleytd;ivrangtrleytdi;vrangtrley
tdik;rangtrleytdiks;angtrleytdiksd;ngtrleytdiksdg;gtrleytdiksdgs;trleytdiksdgsg;rleytdiksd
gsgk;leytdiksdgsgka;eytdiksdgsgkak;ytdiksdgsgkake;tdiksdgsgkakev;diksdgsgkakevl;iks
dgsgkakevlk;ksdgsgkakevlkd;sdgsgkakevlkdf;dgsgkakevlkdft;gsgkakevlkdftl;sgkakevlkd
ftle;gkakevlkdftleg;kakevlkdftlegt;akevlkdftlegtl;kevlkdftlegtla;evlkdftlegtlaa;vlkdftlegtla
ad;lkdftlegtlaadg;kdftlegtlaadgk;dftlegtlaadgkt;ftlegtlaadgktt;tlegtlaadgkttl;legtlaadgkttlk;
egtlaadgkttlkv;gtlaadgkttlkvt;tlaadgkttlkvte;laadgkttlkvteg;aadgkttlkvtegt;adgkttlkvtegtv;
dgkttlkvtegtvv;gkttlkvtegtvvl;kttlkvtegtvvls;ttlkvtegtvvlsk;tlkvtegtvvlskn;lkvtegtvvlskni;
kvtegtvvlsknil;vtegtvvlsknilk;tegtvvlsknilks;egtvvlsknilksg;gtvvlsknilksge;tvvlsknilksgei
;vvlsknilksgeit;vlsknilksgeitv;lsknilksgeitva;sknilksgeitval;knilksgeitvald;nilksgeitvaldd;i
lksgeitvaldds;lksgeitvalddsd;ksgeitvalddsdt;sgeitvalddsdtt;geitvalddsdttq;eitvalddsdttqa;it
valddsdttqat;tvalddsdttqatk;valddsdttqatkk;alddsdttqatkkt;lddsdttqatkktg;ddsdttqatkktgk;d
sdttqatkktgkw;sdttqatkktgkwd;dttqatkktgkwds;ttqatkktgkwdsk;tqatkktgkwdskt;qatkktgkw
dskts;atkktgkwdsktst;tkktgkwdsktstl;kktgkwdsktstlt;ktgkwdsktstlti;tgkwdsktstltis;gkwdsk
tstltisv;kwdsktstltisvn;wdsktstltisvns;dsktstltisvnsq;sktstltisvnsqk;ktstltisvnsqkt;tstltisvns
qktk;stltisvnsqktkn;tltisvnsqktknl;ltisvnsqktknlv;tisvnsqktknlvf;isvnsqktknlvft;svnsqktknl
vftk;vnsqktknlvftke;nsqktknlvftked;sqktknlvftkedt;qktknlvftkedti;ktknlvftkedtit;tknlvftke
dtitv;knlvftkedtitvq;nlvftkedtitvqk;lvftkedtitvqky;vftkedtitvqkyd;ftkedtitvqkyds;tkedtitvq
kydsa;kedtitvqkydsag;edtitvqkydsagt;dtitvqkydsagtn;titvqkydsagtnl;itvqkydsagtnle;tvqky

dsagtnleg;vqkydsagtnlegk;qkydsagtnlegka;kydsagtnlegkav;ydsagtnlegkave;dsagtnlegkav
ei;sagtnlegkaveit;agtnlegkaveitt;gtnlegkaveittl;tnlegkaveittle;nlegkaveittlek;legkaveittlekl
;egkaveittleklk;gkaveittleklkd;kaveittleklkda;aveittleklkdal;veittleklkdalk;

15 mers:
mkkyllgiglilali;kkyllgiglilalia;kyllgiglilaliac;yllgiglilaliack;llgiglilaliackq;lgiglilaliackqn;
giglilaliackqnv;iglilaliackqnvs;glilaliackqnvss;lilaliackqnvssl;ilaliackqnvssld;laliackqnvss
lde;aliackqnvssldek;liackqnvssldekn;iackqnvssldekns;ackqnvssldeknsv;ckqnvssldeknsvs;
kqnvssldeknsvsv;qnvssldeknsvsvd;nvssldeknsvsvdl;vssldeknsvsvdlp;ssldeknsvsvdlpg;sld
eknsvsvdlpgg;ldeknsvsvdlpggm;deknsvsvdlpggmk;eknsvsvdlpggmkv;knsvsvdlpggmkvl;
nsvsvdlpggmkvlv;svsvdlpggmkvlvs;vsvdlpggmkvlvsk;svdlpggmkvlvske;vdlpggmkvlvsk
ek;dlpggmkvlvskekd;lpggmkvlvskekdk;pggmkvlvskekdkd;ggmkvlvskekdkdg;gmkvlvske
kdkdgk;mkvlvskekdkdgky;kvlvskekdkdgkys;vlvskekdkdgkysl;lvskekdkdgkyslm;vskekdk
dgkyslma;skekdkdgkyslmat;kekdkdgkyslmatv;ekdkdgkyslmatve;kdkdgkyslmatvek;dkdg
kyslmatvekl;kdgkyslmatvekle;dgkyslmatveklel;gkyslmatveklelk;kyslmatveklelkg;yslmat
veklelkgt;slmatveklelkgts;lmatveklelkgtsd;matveklelkgtsdk;atveklelkgtsdkn;tveklelkgtsd
knn;veklelkgtsdknng;eklelkgtsdknngs;klelkgtsdknngsg;lelkgtsdknngsgt;elkgtsdknngsgtl;l
kgtsdknngsgtle;kgtsdknngsgtleg;gtsdknngsgtlege;tsdknngsgtlegek;sdknngsgtlegekt;dknng
sgtlegektd;knngsgtlegektdk;nngsgtlegektdks;ngsgtlegektdksk;gsgtlegektdskv;sgtlegektd
kskvk;gtlegektdkskvkl;tlegektdkskvklt;legektdkskvklti;egektdkskvkltia;gektdkskvkltiae;e
ktdkskvkltiaed;ktdkskvkltiaedl;tdkskvkltiaedls;dkskvkltiaedlsk;kskvkltiaedlskt;skvkltiaed
lsktt;kvkltiaedlskttf;vkltiaedlskttfe;kltiaedlskttfei;ltiaedlskttfeif;tiaedlskttfeifk;iaedlskttfei
fke;aedlskttfeifked;edlskttfeifkedg;dlskttfeifkedgk;lskttfeifkedgkt;skttfeifkedgktl;kttfeifke
dgktlv;ttfeifkedgktlvs;tfeifkedgktlvsk;feifkedgktlvskk;eifkedgktlvskkv;ifkedgktlvskkvt;fk
edgktlvskkvtl;kedgktlvskkvtlk;edgktlvskkvtlkd;dgktlvskkvtlkdk;gktlvskkvtlkdks;ktlvskk
vtlkdkss;tlvskkvtlkdksst;lvskkvtlkdksste;vskkvtlkdksstee;skkvtlkdkssteek;kkvtlkdkssteek
f;kvtlkdkssteekfn;vtlkdkssteekfne;tlkdkssteekfnek;lkdkssteekfnekg;kdkssteekfnekge;dkss
teekfnekgei;kssteekfnekgeis;ssteekfnekgeise;steekfnekgeisek;teekfnekgeisekt;eekfnekgei
sekti;ekfnekgeisektiv;kfnekgeisektivr;fnekgeisektivra;nekgeisektivran;ekgeisektivrang;kg
eisektivrangt;geisektivrangtr;eisektivrangtrl;isektivrangtrle;sektivrangtrley;ektivrangtrleyt
;ktivrangtrleytd;tivrangtrleytdi;ivrangtrleytdik;vrangtrleytdiks;rangtrleytdiksd;angtrleytdi
ksdg;ngtrleytdiksdgs;gtrleytdiksdgsg;trleytdiksdgsgk;rleytdiksdgsgka;leytdiksdgsgkak;ey
tdiksdgsgkake;ytdiksdgsgkakev;tdiksdgsgkakevl;diksdgsgkakevlk;iksdgsgkakevlkd;ksdg
sgkakevlkdf;sdgsgkakevlkdft;dgsgkakevlkdftl;gsgkakevlkdftle;sgkakevlkdftleg;gkakevlk
dftlegt;kakevlkdftlegtl;akevlkdftlegtla;kevlkdftlegtlaa;evlkdftlegtlaad;vlkdftlegtlaadg;lkdf
tlegtlaadgk;kdftlegtlaadgkt;dftlegtlaadgktt;ftlegtlaadgkttl;tlegtlaadgkttlk;legtlaadgkttlkv;e
gtlaadgkttlkvt;gtlaadgkttlkvte;tlaadgkttlkvteg;laadgkttlkvtegt;aadgkttlkvtegtv;adgkttlkvte
gtvv;dgkttlkvtegtvvl;gkttlkvtegtvvls;kttlkvtegtvvlsk;ttlkvtegtvvlskn;tlkvtegtvvlskni;lkvte
gtvvlsknil;kvtegtvvlsknilk;vtegtvvlsknilks;tegtvvlsknilksg;egtvvlsknilksge;gtvvlsknilksge
i;tvvlsknilksgeit;vvlsknilksgeitv;vlsknilksgeitva;lsknilksgeitval;sknilksgeitvald;knilksgeit
valdd;nilksgeitvaldds;ilksgeitvalddsd;lksgeitvalddsdt;ksgeitvalddsdtt;sgeitvalddsdttq;geit
valddsdttqa;eitvalddsdttqat;itvalddsdttqatk;tvalddsdttqatkk;valddsdttqatkkt;alddsdttqatkkt
g;lddsdttqatkktgk;ddsdttqatkktgkw;dsdttqatkktgkwd;sdttqatkktgkwds;dttqatkktgkwdsk;ttq
atkktgkwdskt;tqatkktgkwdskts;qatkktgkwdsktst;atkktgkwdsktstl;tkktgkwdsktstlt;kktgkwd
sktstlti;ktgkwdsktstltis;tgkwdsktstltisv;gkwdsktstltisvn;kwdsktstltisvns;wdsktstltisvnsq;ds
ktstltisvnsqk;sktstltisvnsqkt;ktstltisvnsqktk;tstltisvnsqktkn;stltisvnsqktknl;tltisvnsqktknlv;

ltisvnsqktknlvf;tisvnsqktknlvft;isvnsqktknlvftk;svnsqktknlvftke;vnsqktknlvftked;nsqktknl
vftkedt;sqktknlvftkedti;qktknlvftkedtit;ktknlvftkedtitv;tknlvftkedtitvq;knlvftkedtitvqk;nlv
ftkedtitvqky;lvftkedtitvqkyd;vftkedtitvqkyds;ftkedtitvqkydsa;tkedtitvqkydsag;kedtitvqky
dsagt;edtitvqkydsagtn;dtitvqkydsagtnl;titvqkydsagtnle;itvqkydsagtnleg;tvqkydsagtnlegk;
vqkydsagtnlegka;qkydsagtnlegkav;kydsagtnlegkave;ydsagtnlegkavei;dsagtnlegkaveit;sag
tnlegkaveitt;agtnlegkaveittl;gtnlegkaveittle;tnlegkaveittlek;nlegkaveittlekl;legkaveittleklk
;egkaveittleklkd;gkaveittleklkda;kaveittleklkdal;aveittleklkdalk;

16 mers:
mkkyllgiglilalia;kkyllgiglilaliac;kyllgiglilaliack;yllgiglilaliackq;llgiglilaliackqn;lgiglilalia
ckqnv;giglilaliackqnvs;iglilaliackqnvss;glilaliackqnvssl;lilaliackqnvssld;ilaliackqnvsslde;
laliackqnvssldek;aliackqnvssldekn;liackqnvssldekns;iackqnvssldeknsv;ackqnvssldeknsvs;
ckqnvssldeknsvsv;kqnvssldeknsvsvd;qnvssldeknsvsvdl;nvssldeknsvsvdlp;vssldeknsvsvdl
pg;ssldeknsvsvdlpgg;sldeknsvsvdlpggm;ldeknsvsvdlpggmk;deknsvsvdlpggmkv;eknsvsvd
lpggmkvl;knsvsvdlpggmkvlv;nsvsvdlpggmkvlvs;svsvdlpggmkvlvsk;vsvdlpggmkvlvske;s
vdlpggmkvlvskek;vdlpggmkvlvskekd;dlpggmkvlvskekdk;lpggmkvlvskekdkd;pggmkvlvs
kekdkdg;ggmkvlvskekdkdgk;gmkvlvskekdkdgky;mkvlvskekdkdgkys;kvlvskekdkdgkysl;
vlvskekdkdgkyslm;lvskekdkdgkyslma;vskekdkdgkyslmat;skekdkdgkyslmatv;kekdkdgkys
lmatve;ekdkdgkyslmatvek;kdkdgkyslmatvekl;dkdgkyslmatvekle;kdgkyslmatveklel;dgkys
lmatveklelk;gkyslmatveklelkg;kyslmatveklelkgt;yslmatveklelkgts;slmatveklelkgtsd;lmatv
eklelkgtsdk;matveklelkgtsdkn;atveklelkgtsdknn;tveklelkgtsdknng;veklelkgtsdknngs;eklel
kgtsdknngsg;klelkgtsdknngsgt;lelkgtsdknngsgtl;elkgtsdknngsgtle;lkgtsdknngsgtleg;kgtsd
knngsgtlege;gtsdknngsgtlegek;tsdknngsgtlegekt;sdknngsgtlegektd;dknngsgtlegektdk;knn
gsgtlegektdks;nngsgtlegektdksk;ngsgtlegektdkskv;gsgtlegektdkskvk;sgtlegektdkskvkl;gtl
egektdkskvklt;tlegektdkskvklti;legektdkskvkltia;egektdkskvkltiae;gektdkskvkltiaed;ektdk
skvkltiaedl;ktdkskvkltiaedls;tdkskvkltiaedlsk;dkskvkltiaedlskt;kskvkltiaedlsktt;skvkltiaed
lskttf;kvkltiaedlskttfe;vkltiaedlskttfei;kltiaedlskttfeif;ltiaedlskttfeifk;tiaedlskttfeifke;iaedls
kttfeifked;aedlskttfeifkedg;edlskttfeifkedgk;dlskttfeifkedgkt;lskttfeifkedgktl;skttfeifkedgk
tlv;kttfeifkedgktlvs;ttfeifkedgktlvsk;tfeifkedgktlvskk;feifkedgktlvskkv;eifkedgktlvskkvt;i
fkedgktlvskkvtl;fkedgktlvskkvtlk;kedgktlvskkvtlkd;edgktlvskkvtlkdk;dgktlvskkvtlkdks;g
ktlvskkvtlkdkss;ktlvskkvtlkdksst;tlvskkvtlkdksste;lvskkvtlkdksstee;vskkvtlkdkssteek;skk
vtlkdkssteekf;kkvtlkdkssteekfn;kvtlkdkssteekfne;vtlkdkssteekfnek;tlkdkssteekfnekg;lkdk
ssteekfnekge;kdkssteekfnekgei;dkssteekfnekgeis;kssteekfnekgeise;ssteekfnekgeisek;steek
fnekgeisekt;teekfnekgeisekti;eekfnekgeisektiv;ekfnekgeisektivr;kfnekgeisektivra;fnekgei
sektivran;nekgeisektivrang;ekgeisektivrangt;kgeisektivrangtr;geisektivrangtrl;eisektivran
gtrle;isektivrangtrley;sektivrangtrleyt;ektivrangtrleytd;ktivrangtrleytdi;tivrangtrleytdik;ivr
angtrleytdiks;vrangtrleytdiksd;rangtrleytdiksdg;angtrleytdiksdgs;ngtrleytdiksdgsg;gtrleyt
diksdgsgk;trleytdiksdgsgka;rleytdiksdgsgkak;leytdiksdgsgkake;eytdiksdgsgkakev;ytdiksd
gsgkakevl;tdiksdgsgkakevlk;diksdgsgkakevlkd;iksdgsgkakevlkdf;ksdgsgkakevlkdft;sdgs
gkakevlkdftl;dgsgkakevlkdftle;gsgkakevlkdftleg;sgkakevlkdftlegt;gkakevlkdftlegtl;kakev
lkdftlegtla;akevlkdftlegtlaa;kevlkdftlegtlaad;evlkdftlegtlaadg;vlkdftlegtlaadgk;lkdftlegtla
adgkt;kdftlegtlaadgktt;dftlegtlaadgkttl;ftlegtlaadgkttlk;tlegtlaadgkttlkv;legtlaadgkttlkvt;eg
tlaadgkttlkvte;gtlaadgkttlkvteg;tlaadgkttlkvtegt;laadgkttlkvtegtv;aadgkttlkvtegtvv;adgkttl
kvtegtvvl;dgkttlkvtegtvvls;gkttlkvtegtvvlsk;kttlkvtegtvvlskn;ttlkvtegtvvlskni;tlkvtegtvvls
knil;lkvtegtvvlsknilk;kvtegtvvlsknilks;vtegtvvlsknilksg;tegtvvlsknilksge;egtvvlsknilksgei
;gtvvlsknilksgeit;tvvlsknilksgeitv;vvlsknilksgeitva;vlsknilksgeitval;lsknilksgeitvald;sknil

ksgeitvaldd;knilksgeitvaldds;nilksgeitvalddsd;ilksgeitvalddsdt;lksgeitvalddsdtt;ksgeitvald
dsdttq;sgeitvalddsdttqa;geitvalddsdttqat;eitvalddsdttqatk;itvalddsdttqatkk;tvalddsdttqatkkt
;valddsdttqatkktg;alddsdttqatkktgk;lddsdttqatkktgkw;ddsdttqatkktgkwd;dsdttqatkktgkwds
;sdttqatkktgkwdsk;dttqatkktgkwdskt;ttqatkktgkwdskts;tqatkktgkwdsktst;qatkktgkwdsktstl
;atkktgkwdsktstlt;tkktgkwdsktstlti;kktgkwdsktstltis;ktgkwdsktstltisv;tgkwdsktstltisvn;gk
wdsktstltisvns;kwdsktstltisvnsq;wdsktstltisvnsqk;dsktstltisvnsqkt;sktstltisvnsqktk;ktstltis
vnsqktkn;tstltisvnsqktknl;stltisvnsqktknlv;tltisvnsqktknlvf;ltisvnsqktknlvft;tisvnsqktknlvf
tk;isvnsqktknlvftke;svnsqktknlvftked;vnsqktknlvftkedt;nsqktknlvftkedti;sqktknlvftkedtit;
qktknlvftkedtitv;ktknlvftkedtitvq;tknlvftkedtitvqk;knlvftkedtitvqky;nlvftkedtitvqkyd;lvftk
edtitvqkyds;vftkedtitvqkydsa;ftkedtitvqkydsag;tkedtitvqkydsagt;kedtitvqkydsagtn;edtitvq
kydsagtnl;dtitvqkydsagtnle;titvqkydsagtnleg;itvqkydsagtnlegk;tvqkydsagtnlegka;vqkydsa
gtnlegkav;qkydsagtnlegkave;kydsagtnlegkavei;ydsagtnlegkaveit;dsagtnlegkaveitt;sagtnle
gkaveittl;agtnlegkaveittle;gtnlegkaveittlek;tnlegkaveittlekl;nlegkaveittleklk;legkaveittlekl
kd;egkaveittleklkda;gkaveittleklkdal;kaveittleklkdalk;

## <YP_853838 outer surface protein B;Protein;Borrelia afzelii PKo>

8 mers:
mkqyllvf;kqyllvfa;qyllvfal;yllvfalv;llvfalvl;lvfalvla;vfalvlal;falvlali
;alvlalia;lvlaliac;vlaliacs;laliacsq;aliacsqk;liacsqkg;iacsqkgt;acsqkgt
e;csqkgtep;sqkgtepk;qkgtepks;kgtepkst;gtepksts;tepkstsq;epkstsqd;pkstsq
dh;kstsqdhn;stsqdhnd;tsqdhndq;sqdhndqe;qdhndqei;dhndqeii;hndqeiin;ndqei
ins;dqeiinsd;qeiinsdn;eiinsdnt;iinsdntp;insdntpk;nsdntpkd;sdntpkds;dntp
kdsk;ntpkdskk;tpkdskkd;pkdskkdl;kdskkdlt;dskkdltv;skkdltvl;kkdltvla;kdl
tvlae;dltvlaee;ltvlaeen;tvlaeens;vlaeensv;laeensvp;aeensvpl;eensvplf;en
svplfn;nsvplfng;svplfngn;vplfngnk;plfngnki;lfngnkif;fngnkifv;ngnkifvs;g
nkifvsk;nkifvske;kifvskek;ifvskekn;fvskekns;vskeknsa;skeknsag;keknsagk;
eknsagky;knsagkye;nsagkyel;sagkyelr;agkyelra;gkyelrat;kyelratv;yelratvd
;elratvdt;lratvdtv;ratvdtve;atvdtvel;tvdtvelk;vdtvelkg;dtvelkgv;tvelkgv
s;velkgvsd;elkgvsdk;lkgvsdkn;kgvsdknn;gvsdknng;vsdknngs;sdknngsg;dknngs
gk;knngsgkl;nngsgkle;ngsgkleg;gsgklegt;sgklegtk;gklegtka;klegtkad;legtk
adk;egtkadkt;gtkadktk;tkadktkv;kadktkva;adktkvam;dktkvamt;ktkvamti;tkva
mtia;kvamtiad;vamtiadd;amtiaddl;mtiaddln;tiaddlnt;iaddlnti;addlntit;ddl
ntitv;dlntitve;lntitvet;ntitvety;titvetyd;itvetyda;tvetydas;vetydasn;et
ydasnk;tydasnkk;ydasnkkt;dasnkktg;asnkktgs;snkktgse;nkktgsev;kktgsevv;k
tgsevvk;tgsevvkk;gsevvkkq;sevvkkqg;evvkkqgs;vvkkqgsv;vkkqgsvi;kkqgsvik;
kqgsvike;qgsvikes;gsvikesy;svikesyk;vikesyka;ikesykan;kesykank;esykankl
;sykankld;ykanklds;kankldsk;ankldskk;nkldskki;kldskkit;ldskkitr;dskkitr
e;skkitren;kkitrene;kitrenet;itrenett;trenettl;renettle;enettley;nettle
ys;ettleyse;ttleysem;tleysemt;leysemtd;eysemtds;ysemtdss;semtdssn;emtds
sna;mtdssnat;tdssnatk;dssnatka;ssnatkav;snatkave;natkavet;atkavetl;tkav
etlk;kavetlkn;avetlkng;vetlkngi;etlkngik;tlkngikl;lkngikle;kngikleg;ngi
klegs;giklegsl;iklegslv;klegslvg;legslvgg;egslvggk;gslvggkt;slvggktt;lv
ggkttv;vggkttvk;ggkttvkl;gkttvklt;kttvklte;ttvklteg;tvkltegt;vkltegti;k
ltegtit;ltegtitl;tegtitlt;egtitltr;gtitltre;titltrei;itltreie;tltreieq;
ltreieqd;treieqdg;reieqdgk;eieqdgkv;ieqdgkvk;eqdgkvki;qdgkvkiy;dgkvkiyl
;gkvkiyln;kvkiylnd;vkiylndt;kiylndtt;iylndtts;ylndttsg;lndttsgs;ndttsgs
t;dttsgstk;ttsgstkk;tsgstkkt;sgstkkta;gstkktat;stkktatw;tkktatwn;kktatw
ne;ktatwnet;tatwnett;atwnettn;twnettnt;wnettntl;nettntlt;ettntlti;ttntl
tis;tntltisa;ntltisad;tltisads;ltisadsk;tisadskk;isadskkt;sadskktk;adsk
ktkd;dskktkdf;skktkdfv;kktkdfvf;ktkdfvfl;tkdfvflt;kdfvfltd;dfvfltdg;fvf
ltdgt;vfltdgti;fltdgtit;ltdgtitv;tdgtitvq;dgtitvqa;gtitvqay;titvqayd;it
vqaydt;tvqaydta;vqaydtag;qaydtagt;aydtagtk;ydtagtkl;dtagtkle;tagtkleg;a

gtklegn;gtklegns;tklegnss;klegnsse;legnssei;egnsseik;gnsseikd;nsseikdl;
sseikdla;seikdlaa;eikdlaal;ikdlaalk;kdlaalka;dlaalkaa;laalkaal;aalkaalk
;

9 mers:
mkqyllvfa;kqyllvfal;qyllvfalv;yllvfalvl;llvfalvla;lvfalvlal;vfalvlali;f
alvlalia;alvlaliac;lvlaliacs;vlaliacsq;laliacsqk;aliacsqkg;liacsqkgt;ia
csqkgte;acsqkgtep;csqkgtepk;sqkgtepks;qkgtepkst;kgtepksts;gtepkstsq;tep
kstsqd;epkstsqdh;pkstsqdhn;kstsqdhnd;stsqdhndq;tsqdhndqe;sqdhndqei;qdhn
dqeii;dhndqeiin;hndqeiins;ndqeiinsd;dqeiinsdn;qeiinsdnt;eiinsdntp;iinsd
ntpk;insdntpkd;nsdntpkds;sdntpkdsk;dntpkdskk;ntpkdskkd;tpkdskkdl;pkdskk
dlt;kdskkdltv;dskkdltvl;skkdltvla;kkdltvlae;kdltvlaee;dltvlaeen;ltvlaee
ns;tvlaeensv;vlaeensvp;laeensvpl;aeensvplf;eensvplfn;ensvplfng;nsvplfng
n;svplfngnk;vplfngnki;plfngnkif;lfngnkifv;fngnkifvs;ngnkifvsk;gnkifvske
;nkifvskek;kifvskekn;ifvskekns;fvskeknsa;vskeknsag;skeknsagk;keknsagky;
eknsagkye;knsagkyel;nsagkyelr;sagkyelra;agkyelrat;gkyelratv;kyelratvd;y
elratvdt;elratvdtv;lratvdtve;ratvdtvel;atvdtvelk;tvdtvelkg;vdtvelkgv;dt
velkgvs;tvelkgvsd;velkgvsdk;elkgvsdkn;lkgvsdknn;kgvsdknng;gvsdknngs;vsd
knngsg;sdknngsgk;dknngsgkl;knngsgkle;nngsgkleg;ngsgklegt;gsgklegtk;sgkl
egtka;gklegtkad;klegtkadk;legtkadkt;egtkadktk;gtkadktkv;tkadktkva;kadkt
kvam;adktkvamt;dktkvamti;ktkvamtia;tkvamtiad;kvamtiadd;vamtiaddl;amtiad
dln;mtiaddlnt;tiaddlnti;iaddlntit;addlntitv;ddlntitve;dlntitvet;lntitve
ty;ntitvetyd;titvetyda;itvetydas;tvetydasn;vetydasnk;etydasnkk;tydasnkk
t;ydasnkktg;dasnkktgs;asnkktgse;snkktgsev;nkktgsevv;kktgsevvk;ktgsevvkk
;tgsevvkkq;gsevvkkqg;sevvkkqgs;evvkkqgsv;vvkkqgsvi;vkkqgsvik;kkqgsvike;
kqgsvikes;qgsvikesy;gsvikesyk;svikesyka;vikesykan;ikesykank;kesykankl;e
sykankld;sykanklds;ykankldsk;kankldskk;ankldskki;nkldskkit;kldskkitr;ld
skkitre;dskkitren;skkitrene;kkitrenet;kitrenett;itrenettl;trenettle;ren
ettley;enettleys;nettleyse;ettleysem;ttleysemt;tleysemtd;leysemtds;eyse
mtdss;ysemtdssn;semtdssna;emtdssnat;mtdssnatk;tdssnatka;dssnatkav;ssnat
kave;snatkavet;natkavetl;atkavetlk;tkavetlkn;kavetlkng;avetlkngi;vetlkn
gik;etlkngikl;tlkngikle;lkngikleg;kngiklegs;ngiklegsl;giklegslv;iklegsl
vg;klegslvgg;legslvggk;egslvggkt;gslvggktt;slvggkttv;lvggkttvk;vggkttvk
l;ggkttvklt;gkttvklte;kttvklteg;ttvkltegt;tvkltegti;vkltegtit;kltegtitl
;ltegtitlt;tegtitltr;egtitltre;gtitltrei;titltreie;itltreieq;tltreieqd;
ltreieqdg;treieqdgk;reieqdgkv;eieqdgkvk;ieqdgkvki;eqdgkvkiy;qdgkvkiyl;d
gkvkiyln;gkvkiylnd;kvkiylndt;vkiylndtt;kiylndtts;iylndttsg;ylndttsgs;ln
dttsgst;ndttsgstk;dttsgstkk;ttsgstkkt;tsgstkkta;sgstkktat;gstkktatw;stk
ktatwn;tkktatwne;kktatwnet;ktatwnett;tatwnettn;atwnettnt;twnettntl;wnet
tntlt;nettntlti;ettntltis;ttntltisa;tntltisad;ntltisads;tltisadsk;ltisa
dskk;tisadskkt;isadskktk;sadskktkd;adskktkdf;dskktkdfv;skktkdfvf;kktkdf
vfl;ktkdfvflt;tkdfvfltd;kdfvfltdg;dfvfltdgt;fvfltdgti;vfltdgtit;fltdgti
tv;ltdgtitvq;tdgtitvqa;dgtitvqay;gtitvqayd;titvqaydt;itvqaydta;tvqaydta
g;vqaydtagt;qaydtagtk;aydtagtkl;ydtagtkle;dtagtkleg;tagtklegn;agtklegns
;gtklegnss;tklegnsse;klegnssei;legnsseik;egnsseikd;gnsseikdl;nsseikdla;
sseikdlaa;seikdlaal;eikdlaalk;ikdlaalka;kdlaalkaa;dlaalkaal;laalkaalk;

10 mers:
mkqyllvfal;kqyllvfalv;qyllvfalvl;yllvfalvla;llvfalvlal;lvfalvlali;vfalv
lalia;falvlaliac;alvlaliacs;lvlaliacsq;vlaliacsqk;laliacsqkg;aliacsqkgt
;liacsqkgte;iacsqkgtep;acsqkgtepk;csqkgtepks;sqkgtepkst;qkgtepksts;kgte
pkstsq;gtepkstsqd;tepkstsqdh;epkstsqdhn;pkstsqdhnd;kstsqdhndq;stsqdhndq
e;tsqdhndqei;sqdhndqeii;qdhndqeiin;dhndqeiins;hndqeiinsd;ndqeiinsdn;dqe
iinsdnt;qeiinsdntp;eiinsdntpk;iinsdntpkd;insdntpkds;nsdntpkdsk;sdntpkds
kk;dntpkdskkd;ntpkdskkdl;tpkdskkdlt;pkdskkdltv;kdskkdltvl;dskkdltvla;sk
kdltvlae;kkdltvlaee;kdltvlaeen;dltvlaeens;ltvlaeensv;tvlaeensvp;vlaeens
vpl;laeensvplf;aeensvplfn;eensvplfng;ensvplfngn;nsvplfngnk;svplfngnki;v

plfngnkif;plfngnkifv;lfngnkifvs;fngnkifvsk;ngnkifvske;gnkifvskek;nkifvs
kekn;kifvsekns;ifvskeknsa;fvskeknsag;vskeknsagk;skeknsagky;keknsagkye;
eknsagkyel;knsagkyelr;nsagkyelra;sagkyelrat;agkyelratv;gkyelratvd;kyelr
atvdt;yelratvdtv;elratvdtve;lratvdtvel;ratvdtvelk;atvdtvelkg;tvdtvelkgv
;vdtvelkgvs;dtvelkgvsd;tvelkgvsdk;velkgvsdkn;elkgvsdknn;lkgvsdknng;kgvs
dknngs;gvsdknngsg;vsdknngsgk;sdknngsgkl;dknngsgkle;knngsgkleg;nngsgkleg
t;ngsgklegtk;gsgklegtka;sgklegtkad;gklegtkadk;klegtkadkt;legtkadktk;egt
kadktkv;gtkadktkva;tkadktkvam;kadktkvamt;adktkvamti;dktkvamtia;ktkvamti
ad;tkvamtiadd;kvamtiaddl;vamtiaddln;amtiaddlnt;mtiaddlnti;tiaddlntit;ia
ddlntitv;addlntitve;ddlntitvet;dlntitvety;lntitvetyd;ntitvetyda;titvety
das;itvetydasn;tvetydasnk;vetydasnkk;etydasnkkt;tydasnkktg;ydasnkktgs;d
asnkktgse;asnkktgsev;snkktgsevv;nkktgsevvk;kktgsevvkk;ktgsevvkkq;tgsevv
kkqg;gsevvkkqgs;sevvkkqgsv;evvkkqgsvi;vvkkqgsvik;vkkqgsvike;kkqgsvikes;
kqgsvikesy;qgsvikesyk;gsvikesyka;svikesykan;vikesykank;ikesykankl;kesyk
ankld;esykanklds;sykankldsk;ykankldskk;kankldskki;ankldskkit;nkldskkitr
;kldskkitre;ldskkitren;dskkitrene;skkitrenet;kkitrenett;kitrenettl;itre
nettle;trenettley;renettleys;enettleyse;nettleysem;ettleysemt;ttleysemt
d;tleysemtds;leysemtdss;eysemtdssn;ysemtdssna;semtdssnat;emtdssnatk;mtd
ssnatka;tdssnatkav;dssnatkave;ssnatkavet;snatkavetl;natkavetlk;atkavetl
kn;tkavetlkng;kavetlkngi;avetlkngik;vetlkngikl;etlkngikle;tlkngikleg;lk
ngiklegs;kngiklegsl;ngiklegslv;giklegslvg;iklegslvgg;klegslvggk;legslvg
gkt;egslvggktt;gslvggkttv;slvggkttvk;lvggkttvkl;vggkttvklt;ggkttvklte;g
kttvklteg;kttvkltegt;ttvkltegti;tvkltegtit;vkltegtitl;kltegtitlt;ltegti
tltr;tegtitltre;egtitltrei;gtitltreie;titltreieq;itltreieqd;tltreieqdg;
ltreieqdgk;treieqdgkv;reieqdgkvk;eieqdgkvki;ieqdgkvkiy;eqdgkvkiyl;qdgkv
kiyln;dgkvkiylnd;gkvkiylndt;kvkiylndtt;vkiylndtts;kiylndttsg;iylndttsgs
;ylndttsgst;lndttsgstk;ndttsgstkk;dttsgstkkt;ttsgstkkta;tsgstkktat;sgst
kktatw;gstkktatwn;stkktatwne;tkktatwnet;kktatwnett;ktatwnettn;tatwnettn
t;atwnettntl;twnettntlt;wnettntlti;nettntltis;ettntltisa;ttntltisad;tnt
ltisads;ntltisadsk;tltisadskk;ltisadskkt;tisadskktk;isadskktkd;sadskktk
df;adskktkdfv;dskktkdfvf;skktkdfvfl;kktkdfvflt;ktkdfvfltd;tkdfvfltdg;kd
fvfltdgt;dfvfltdgti;fvfltdgtit;vfltdgtitv;fltdgtitvq;ltdgtitvqa;tdgtitv
qay;dgtitvqayd;gtitvqaydt;titvqaydta;itvqaydtag;tvqaydtagt;vqaydtagtk;q
aydtagtkl;aydtagtkle;ydtagtkleg;dtagtklegn;tagtklegns;agtklegnss;gtkleg
nsse;tklegnssei;klegnsseik;legnsseikd;egnsseikdl;gnsseikdla;nsseikdlaa;
sseikdlaal;seikdlaalk;eikdlaalka;ikdlaalkaa;kdlaalkaal;dlaalkaalk;

11 mers:
mkqyllvfalv;kqyllvfalvl;qyllvfalvla;yllvfalvlal;llvfalvlali;lvfalvlalia
;vfalvlaliac;falvlaliacs;alvlaliacsq;lvlaliacsqk;vlaliacsqkg;laliacsqkg
t;aliacsqkgte;liacsqkgtep;iacsqkgtepk;acsqkgtepks;csqkgtepkst;sqkgtepks
ts;qkgtepkstsq;kgtepkstsqd;gtepkstsqdh;tepkstsqdhn;epkstsqdhnd;pkstsqdh
ndq;kstsqdhndqe;stsqdhndqei;tsqdhndqeii;sqdhndqeiin;qdhndqeiins;dhndqei
insd;hndqeiinsdn;ndqeiinsdnt;dqeiinsdntp;qeiinsdntpk;eiinsdntpkd;iinsdn
tpkds;insdntpkdsk;nsdntpkdskk;sdntpkdskkd;dntpkdskkdl;ntpkdskkdlt;tpkds
kkdltv;pkdskkdltvl;kdskkdltvla;dskkdltvlae;skkdltvlaee;kkdltvlaeen;kdlt
vlaeens;dltvlaeensv;ltvlaeensvp;tvlaeensvpl;vlaeensvplf;laeensvplfn;aee
nsvplfng;eensvplfngn;ensvplfngnk;nsvplfngnki;svplfngnkif;vplfngnkifv;pl
fngnkifvs;lfngnkifvsk;fngnkifvske;ngnkifvskek;gnkifvskekn;nkifvskekns;k
ifvskeknsa;ifvskeknsag;fvskeknsagk;vskeknsagky;skeknsagkye;keknsagkyel;
eknsagkyelr;knsagkyelra;nsagkyelrat;sagkyelratv;agkyelratvd;gkyelratvdt
;kyelratvdtv;yelratvdtve;elratvdtvel;lratvdtvelk;ratvdtvelkg;atvdtvelkg
v;tvdtvelkgvs;vdtvelkgvsd;dtvelkgvsdk;tvelkgvsdkn;velkgvsdknn;elkgvsdkn
ng;lkgvsdknngs;kgvsdknngsg;gvsdknngsgk;vsdknngsgkl;sdknngsgkle;dknngsgk
leg;knngsgklegt;nngsgklegtk;ngsgklegtka;gsgklegtkad;sgklegtkadk;gklegtk
adkt;klegtkadktk;legtkadktkv;egtkadktkva;gtkadktkvam;tkadktkvamt;kadktk
vamti;adktkvamtia;dktkvamtiad;ktkvamtiadd;tkvamtiaddl;kvamtiaddln;vamti

addlnt;amtiaddlnti;mtiaddlntit;tiaddlntitv;iaddlntitve;addlntitvet;ddln
titvety;dlntitvetyd;lntitvetyda;ntitvetydas;titvetydasn;itvetydasnk;tve
tydasnkk;vetydasnkkt;etydasnkktg;tydasnkktgs;ydasnkktgse;dasnkktgsev;as
nkktgsevv;snkktgsevvk;nkktgsevvkk;kktgsevvkkq;ktgsevvkkqg;tgsevvkkqgs;g
sevvkkqgsv;sevvkkqgsvi;evvkkqgsvik;vvkkqgsvike;vkkqgsvikes;kkqgsvikesy;
kqgsvikesyk;qgsvikesyka;gsvikesykan;svikesykank;vikesykankl;ikesykankld
;kesykanklds;esykankldsk;sykankldskk;ykankldskki;kankldskkit;ankldskkit
r;nkldskkitre;kldskkitren;ldskkitrene;dskkitrenet;skkitrenett;kkitrenet
tl;kitrenettle;itrenettley;trenettleys;renettleyse;enettleysem;nettleys
emt;ettleysemtd;ttleysemtds;tleysemtdss;leysemtdssn;eysemtdssna;ysemtds
snat;semtdssnatk;emtdssnatka;mtdssnatkav;tdssnatkave;dssnatkavet;ssnatk
avetl;snatkavetlk;natkavetlkn;atkavetlkng;tkavetlkngi;kavetlkngik;avetl
kngikl;vetlkngikle;etlkngikleg;tlkngiklegs;lkngiklegsl;kngiklegslv;ngik
legslvg;giklegslvgg;iklegslvggk;klegslvggkt;legslvggktt;egslvggkttv;gsl
vggkttvk;slvggkttvkl;lvggkttvklt;vggkttvklte;ggkttvklteg;gkttvkltegt;kt
tvkltegti;ttvkltegtit;tvkltegtitl;vkltegtitlt;kltegtitltr;ltegtitltre;t
egtitltrei;egtitltreie;gtitltreieq;titltreieqd;itltreieqdg;tltreieqdgk;
ltreieqdgkv;treieqdgkvk;reieqdgkvki;eieqdgkvkiy;ieqdgkvkiyl;eqdgkvkiyln
;qdgkvkiylnd;dgkvkiylndt;gkvkiylndtt;kvkiylndtts;vkiylndttsg;kiylndttsg
s;iylndttsgst;ylndttsgstk;lndttsgstkk;ndttsgstkkt;dttsgstkkta;ttsgstkkt
at;tsgstkktatw;sgstkktatwn;gstkktatwne;stkktatwnet;tkktatwnett;kktatwne
ttn;ktatwnettnt;tatwnettntl;atwnettntlt;twnettntlti;wnettntltis;nettntl
tisa;ettntltisad;ttntltisads;tntltisadsk;ntltisadskk;tltisadskkt;ltisad
skktk;tisadskktkd;isadskktkdf;sadskktkdfv;adskktkdfvf;dskktkdfvfl;skktk
dfvflt;kktkdfvfltd;ktkdfvfltdg;tkdfvfltdgt;kdfvfltdgti;dfvfltdgtit;fvfl
tdgtitv;vfltdgtitvq;fltdgtitvqa;ltdgtitvqay;tdgtitvqayd;dgtitvqaydt;gti
tvqaydta;titvqaydtag;itvqaydtagt;tvqaydtagtk;vqaydtagtkl;qaydtagtkle;ay
dtagtkleg;ydtagtklegn;dtagtklegns;tagtklegnss;agtklegnsse;gtklegnssei;t
klegnsseik;klegnsseikd;legnsseikdl;egnsseikdla;gnsseikdlaa;nsseikdlaal;
sseikdlaalk;seikdlaalka;eikdlaalkaa;ikdlaalkaal;kdlaalkaalk;

13 mers:
mkqyllvfalvla;kqyllvfalvlal;qyllvfalvlali;yllvfalvlalia;llvfalvlaliac;l
vfalvlaliacs;vfalvlaliacsq;falvlaliacsqk;alvlaliacsqkg;lvlaliacsqkgt;vl
aliacsqkgte;laliacsqkgtep;aliacsqkgtepk;liacsqkgtepks;iacsqkgtepkst;acs
qkgtepksts;csqkgtepkstsq;sqkgtepkstsqd;qkgtepkstsqdh;kgtepkstsqdhn;gtep
kstsqdhnd;tepkstsqdhndq;epkstsqdhndqe;pkstsqdhndqei;kstsqdhndqeii;stsqd
hndqeiin;tsqdhndqeiins;sqdhndqeiinsd;qdhndqeiinsdn;dhndqeiinsdnt;hndqei
insdntp;ndqeiinsdntpk;dqeiinsdntpkd;qeiinsdntpkds;eiinsdntpkdsk;iinsdnt
pkdskk;insdntpkdskkd;nsdntpkdskkdl;sdntpkdskkdlt;dntpkdskkdltv;ntpkdskk
dltvl;tpkdskkdltvla;pkdskkdltvlae;kdskkdltvlaee;dskkdltvlaeen;skkdltvla
eens;kkdltvlaeensv;kdltvlaeensvp;dltvlaeensvpl;ltvlaeensvplf;tvlaeensvp
lfn;vlaeensvplfng;laeensvplfngn;aeensvplfngnk;eensvplfngnki;ensvplfngnk
if;nsvplfngnkifv;svplfngnkifvs;vplfngnkifvsk;plfngnkifvske;lfngnkifvske
k;fngnkifvskekn;ngnkifvskekns;gnkifvskeknsa;nkifvskeknsag;kifvskeknsagk
;ifvskeknsagky;fvskeknsagkye;vskeknsagkyel;skeknsagkyelr;keknsagkyelra;
eknsagkyelrat;knsagkyelratv;nsagkyelratvd;sagkyelratvdt;agkyelratvdtv;g
kyelratvdtve;kyelratvdtvel;yelratvdtvelk;elratvdtvelkg;lratvdtvelkgv;ra
tvdtvelkgvs;atvdtvelkgvsd;tvdtvelkgvsdk;vdtvelkgvsdkn;dtvelkgvsdknn;tve
lkgvsdknng;velkgvsdknngs;elkgvsdknngsg;lkgvsdknngsgk;kgvsdknngsgkl;gvsd
knngsgkle;vsdknngsgkleg;sdknngsgklegt;dknngsgklegtk;knngsgklegtka;nngsg
klegtkad;ngsgklegtkadk;gsgklegtkadkt;sgklegtkadktk;gklegtkadktkv;klegtk
adktkva;legtkadktkvam;egtkadktkvamt;gtkadktkvamti;tkadktkvamtia;kadktkv
amtiad;adktkvamtiadd;dktkvamtiaddl;ktkvamtiaddln;tkvamtiaddlnt;kvamtiad
dlnti;vamtiaddlntit;amtiaddlntitv;mtiaddlntitve;tiaddlntitvet;iaddlntit
vety;addlntitvetyd;ddlntitvetyda;dlntitvetydas;lntitvetydasn;ntitvetyda
snk;titvetydasnkk;itvetydasnkkt;tvetydasnkktg;vetydasnkktgs;etydasnkktg

se;tydasnkktgsev;ydasnkktgsevv;dasnkktgsevvk;asnkktgsevvkk;snkktgsevvkk
q;nkktgsevvkkqg;kktgsevvkkqgs;ktgsevvkkqgsv;tgsevvkkqgsvi;gsevvkkqgsvik
;sevvkkqgsvike;evvkkqgsvikes;vvkkqgsvikesy;vkkqgsvikesyk;kkqgsvikesyka;
kqgsvikesykan;qgsvikesykank;gsvikesykankl;svikesykankld;vikesykanklds;i
kesykankldsk;kesykankldskk;esykankldskki;sykankldskkit;ykankldskkitr;ka
nkldskkitre;ankldskkitren;nkldskkitrene;kldskkitrenet;ldskkitrenett;dsk
kitrenettl;skkitrenettle;kkitrenettley;kitrenettleys;itrenettleyse;tren
ettleysem;renettleysemt;enettleysemtd;nettleysemtds;ettleysemtdss;ttley
semtdssn;tleysemtdssna;leysemtdssnat;eysemtdssnatk;ysemtdssnatka;semtds
snatkav;emtdssnatkave;mtdssnatkavet;tdssnatkavetl;dssnatkavetlk;ssnatka
vetlkn;snatkavetlkng;natkavetlkngi;atkavetlkngik;tkavetlkngikl;kavetlkn
gikle;avetlkngikleg;vetlkngiklegs;etlkngiklegsl;tlkngiklegslv;lkngikleg
slvg;kngiklegslvgg;ngiklegslvggk;giklegslvggkt;iklegslvggktt;klegslvggk
ttv;legslvggkttvk;egslvggkttvkl;gslvggkttvklt;slvggkttvklte;lvggkttvklt
eg;vggkttvkltegt;ggkttvkltegti;gkttvkltegtit;kttvkltegtitl;ttvkltegtitl
t;tvkltegtitltr;vkltegtitltre;kltegtitltrei;ltegtitltreie;tegtitltreieq
;egtitltreieqd;gtitltreieqdg;titltreieqdgk;itltreieqdgkv;tltreieqdgkvk;
ltreieqdgkvki;treieqdgkvkiy;reieqdgkvkiyl;eieqdgkvkiyln;ieqdgkvkiylnd;e
qdgkvkiylndt;qdgkvkiylndtt;dgkvkiylndtts;gkvkiylndttsg;kvkiylndttsgs;vk
iylndttsgst;kiylndttsgstk;iylndttsgstkk;ylndttsgstkkt;lndttsgstkkta;ndt
tsgstkktat;dttsgstkktatw;ttsgstkktatwn;tsgstkktatwne;sgstkktatwnet;gstk
ktatwnett;stkktatwnettn;tkktatwnettnt;kktatwnettntl;ktatwnettntlt;tatwn
ettntlti;atwnettntltis;twnettntltisa;wnettntltisad;nettntltisads;ettntl
tisadsk;ttntltisadskk;tntltisadskkt;ntltisadskktk;tltisadskktkd;ltisads
kktkdf;tisadskktkdfv;isadskktkdfvf;sadskktkdfvfl;adskktkdfvflt;dskktkdf
vfltd;skktkdfvfltdg;kktkdfvfltdgt;ktkdfvfltdgti;tkdfvfltdgtit;kdfvfltdg
titv;dfvfltdgtitvq;fvfltdgtitvqa;vfltdgtitvqay;fltdgtitvqayd;ltdgtitvqa
ydt;tdgtitvqaydta;dgtitvqaydtag;gtitvqaydtagt;titvqaydtagtk;itvqaydtagt
kl;tvqaydtagtkle;vqaydtagtkleg;qaydtagtklegn;aydtagtklegns;ydtagtklegns
s;dtagtklegnsse;tagtklegnssei;agtklegnsseik;gtklegnsseikd;tklegnsseikdl
;klegnsseikdla;legnsseikdlaa;egnsseikdlaal;gnsseikdlaalk;nsseikdlaalka;
sseikdlaalkaa;seikdlaalkaal;eikdlaalkaalk;

14 mers:
mkqyllvfalvlal;kqyllvfalvlali;qyllvfalvlalia;yllvfalvlaliac;llvfalvlali
acs;lvfalvlaliacsq;vfalvlaliacsqk;falvlaliacsqkg;alvlaliacsqkgt;lvlalia
csqkgte;vlaliacsqkgtep;laliacsqkgtepk;aliacsqkgtepks;liacsqkgtepkst;iac
sqkgtepksts;acsqkgtepkstsq;csqkgtepkstsqd;sqkgtepkstsqdh;qkgtepkstsqdhn
;kgtepkstsqdhnd;gtepkstsqdhndq;tepkstsqdhndqe;epkstsqdhndqei;pkstsqdhnd
qeii;kstsqdhndqeiin;stsqdhndqeiins;tsqdhndqeiinsd;sqdhndqeiinsdn;qdhndq
eiinsdnt;dhndqeiinsdntp;hndqeiinsdntpk;ndqeiinsdntpkd;dqeiinsdntpkds;qe
iinsdntpkdsk;eiinsdntpkdskk;iinsdntpkdskkd;insdntpkdskkdl;nsdntpkdskkdl
t;sdntpkdskkdltv;dntpkdskkdltvl;ntpkdskkdltvla;tpkdskkdltvlae;pkdskkdlt
vlaee;kdskkdltvlaeen;dskkdltvlaeens;skkdltvlaeensv;kkdltvlaeensvp;kdltv
laeensvpl;dltvlaeensvplf;ltvlaeensvplfn;tvlaeensvplfng;vlaeensvplfngn;l
aeensvplfngnk;aeensvplfngnki;eensvplfngnkif;ensvplfngnkifv;nsvplfngnkif
vs;svplfngnkifvsk;vplfngnkifvske;plfngnkifvskek;lfngnkifvskekn;fngnkifv
skekns;ngnkifvskeknsa;gnkifvskeknsag;nkifvskeknsagk;kifvskeknsagky;ifvs
keknsagkye;fvskeknsagkyel;vskeknsagkyelr;skeknsagkyelra;keknsagkyelrat;
eknsagkyelratv;knsagkyelratvd;nsagkyelratvdt;sagkyelratvdtv;agkyelratvd
tve;gkyelratvdtvel;kyelratvdtvelk;yelratvdtvelkg;elratvdtvelkgv;lratvdt
velkgvs;ratvdtvelkgvsd;atvdtvelkgvsdk;tvdtvelkgvsdkn;vdtvelkgvsdknn;dtv
elkgvsdknng;tvelkgvsdknngs;velkgvsdknngsg;elkgvsdknngsgk;lkgvsdknngsgkl
;kgvsdknngsgkle;gvsdknngsgkleg;vsdknngsgklegt;sdknngsgklegtk;dknngsgkle
gtka;knngsgklegtkad;nngsgklegtkadk;ngsgklegtkadkt;gsgklegtkadktk;sgkleg
tkadktkv;gklegtkadktkva;klegtkadktkvam;legtkadktkvamt;egtkadktkvamti;gt
kadktkvamtia;tkadktkvamtiad;kadktkvamtiadd;adktkvamtiaddl;dktkvamtiaddl

n;ktkvamtiaddlnt;tkvamtiaddlnti;kvamtiaddlntit;vamtiaddlntitv;amtiaddln
titve;mtiaddlntitvet;tiaddlntitvety;iaddlntitvetyd;addlntitvetyda;ddlnt
itvetydas;dlntitvetydasn;lntitvetydasnk;ntitvetydasnkk;titvetydasnkkt;i
tvetydasnkktg;tvetydasnkktgs;vetydasnkktgse;etydasnkktgsev;tydasnkktgse
vv;ydasnkktgsevvk;dasnkktgsevvkk;asnkktgsevvkkq;snkktgsevvkkqg;nkktgsev
vkkqgs;kktgsevvkkqgsv;ktgsevvkkqgsvi;tgsevvkkqgsvik;gsevvkkqgsvike;sevv
kkqgsvikes;evvkkqgsvikesy;vvkkqgsvikesyk;vkkqgsvikesyka;kkqgsvikesykan;
kqgsvikesykank;qgsvikesykankl;gsvikesykankld;svikesykanklds;vikesykankl
dsk;ikesykankldskk;kesykankldskki;esykankldskkit;sykankldskkitr;ykankld
skkitre;kankldskkitren;ankldskkitrene;nkldskkitrenet;kldskkitrenett;lds
kkitrenettl;dskkitrenettle;skkitrenettley;kkitrenettleys;kitrenettleyse
;itrenettleysem;trenettleysemt;renettleysemtd;enettleysemtds;nettleysem
tdss;ettleysemtdssn;ttleysemtdssna;tleysemtdssnat;leysemtdssnatk;eysemt
dssnatka;ysemtdssnatkav;semtdssnatkave;emtdssnatkavet;mtdssnatkavetl;td
ssnatkavetlk;dssnatkavetlkn;ssnatkavetlkng;snatkavetlkngi;natkavetlkngi
k;atkavetlkngikl;tkavetlkngikle;kavetlkngikleg;avetlkngiklegs;vetlkngik
legsl;etlkngiklegslv;tlkngiklegslvg;lkngiklegslvgg;kngiklegslvggk;ngikl
egslvggkt;giklegslvggktt;iklegslvggkttv;klegslvggkttvk;legslvggkttvkl;e
gslvggkttvklt;gslvggkttvklte;slvggkttvklteg;lvggkttvkltegt;vggkttvklteg
ti;ggkttvkltegtit;gkttvkltegtitl;kttvkltegtitlt;ttvkltegtitltr;tvkltegt
itltre;vkltegtitltrei;kltegtitltreie;ltegtitltreieq;tegtitltreieqd;egti
tltreieqdg;gtitltreieqdgk;titltreieqdgkv;itltreieqdgkvk;tltreieqdgkvki;
ltreieqdgkvkiy;treieqdgkvkiyl;reieqdgkvkiyln;eieqdgkvkiylnd;ieqdgkvkiyl
ndt;eqdgkvkiylndtt;qdgkvkiylndtts;dgkvkiylndttsg;gkvkiylndttsgs;kvkiyln
dttsgst;vkiylndttsgstk;kiylndttsgstkk;iylndttsgstkkt;ylndttsgstkkta;lnd
ttsgstkktat;ndttsgstkktatw;dttsgstkktatwn;ttsgstkktatwne;tsgstkktatwnet
;sgstkktatwnett;gstkktatwnettn;stkktatwnettnt;tkktatwnettntl;kktatwnett
ntlt;ktatwnettntlti;tatwnettntltis;atwnettntltisa;twnettntltisad;wnettn
tltisads;nettntltisadsk;ettntltisadskk;ttntltisadskkt;tntltisadskktk;nt
ltisadskktkd;tltisadskktkdf;ltisadskktkdfv;tisadskktkdfvf;isadskktkdfvf
l;sadskktkdfvflt;adskktkdfvfltd;dskktkdfvfltdg;skktkdfvfltdgt;kktkdfvfl
tdgti;ktkdfvfltdgtit;tkdfvfltdgtitv;kdfvfltdgtitvq;dfvfltdgtitvqa;fvflt
dgtitvqay;vfltdgtitvqayd;fltdgtitvqaydt;ltdgtitvqaydta;tdgtitvqaydtag;d
gtitvqaydtagt;gtitvqaydtagtk;titvqaydtagtkl;itvqaydtagtkle;tvqaydtagtkl
eg;vqaydtagtklegn;qaydtagtklegns;aydtagtklegnss;ydtagtklegnsse;dtagtkle
gnssei;tagtklegnsseik;agtklegnsseikd;gtklegnsseikdl;tklegnsseikdla;kleg
nsseikdlaa;legnsseikdlaal;egnsseikdlaalk;gnsseikdlaalka;nsseikdlaalkaa;
sseikdlaalkaal;seikdlaalkaalk;

15 mers:
mkqyllvfalvlali;kqyllvfalvlalia;qyllvfalvlaliac;yllvfalvlaliacs;llvfalv
laliacsq;lvfalvlaliacsqk;vfalvlaliacsqkg;falvlaliacsqkgt;alvlaliacsqkgt
e;lvlaliacsqkgtep;vlaliacsqkgtepk;laliacsqkgtepks;aliacsqkgtepkst;liacs
qkgtepksts;iacsqkgtepkstsq;acsqkgtepkstsqd;csqkgtepkstsqdh;sqkgtepkstsq
dhn;qkgtepkstsqdhnd;kgtepkstsqdhndq;gtepkstsqdhndqe;tepkstsqdhndqei;epk
stsqdhndqeii;pkstsqdhndqeiin;kstsqdhndqeiins;stsqdhndqeiinsd;tsqdhndqei
insdn;sqdhndqeiinsdnt;qdhndqeiinsdntp;dhndqeiinsdntpk;hndqeiinsdntpkd;n
dqeiinsdntpkds;dqeiinsdntpkdsk;qeiinsdntpkdskk;eiinsdntpkdskkd;iinsdntp
kdskkdl;insdntpkdskkdlt;nsdntpkdskkdltv;sdntpkdskkdltvl;dntpkdskkdltvla
;ntpkdskkdltvlae;tpkdskkdltvlaee;pkdskkdltvlaeen;kdskkdltvlaeens;dskkdl
tvlaeensv;skkdltvlaeensvp;kkdltvlaeensvpl;kdltvlaeensvplf;dltvlaeensvpl
fn;ltvlaeensvplfng;tvlaeensvplfngn;vlaeensvplfngnk;laeensvplfngnki;aeen
svplfngnkif;eensvplfngnkifv;ensvplfngnkifvs;nsvplfngnkifvsk;svplfngnkif
vske;vplfngnkifvskek;plfngnkifvskekn;lfngnkifvskekns;fngnkifvskeknsa;ng
nkifvskeknsag;gnkifvskeknsagk;nkifvskeknsagky;kifvskeknsagkye;ifvsekns
agkyel;fvskeknsagkyelr;vskeknsagkyelra;skeknsagkyelrat;keknsagkyelratv;
eknsagkyelratvd;knsagkyelratvdt;nsagkyelratvdtv;sagkyelratvdtve;agkyelr

atvdtvel;gkyelratvdtvelk;kyelratvdtvelkg;yelratvdtvelkgv;elratvdtvelkgv
s;lratvdtvelkgvsd;ratvdtvelkgvsdk;atvdtvelkgvsdkn;tvdtvelkgvsdknn;vdtve
lkgvsdknng;dtvelkgvsdknngs;tvelkgvsdknngsg;velkgvsdknngsgk;elkgvsdknngs
gkl;lkgvsdknngsgkle;kgvsdknngsgkleg;gvsdknngsgklegt;vsdknngsgklegtk;sdk
nngsgklegtka;dknngsgklegtkad;knngsgklegtkadk;nngsgklegtkadkt;ngsgklegtk
adktk;gsgklegtkadktkv;sgklegtkadktkva;gklegtkadktkvam;klegtkadktkvamt;l
egtkadktkvamti;egtkadktkvamtia;gtkadktkvamtiad;tkadktkvamtiadd;kadktkva
mtiaddl;adktkvamtiaddln;dktkvamtiaddlnt;ktkvamtiaddlnti;tkvamtiaddlntit
;kvamtiaddlntitv;vamtiaddlntitve;amtiaddlntitvet;mtiaddlntitvety;tiaddl
ntitvetyd;iaddlntitvetyda;addlntitvetydas;ddlntitvetydasn;dlntitvetydas
nk;lntitvetydasnkk;ntitvetydasnkkt;titvetydasnkktg;itvetydasnkktgs;tvet
ydasnkktgse;vetydasnkktgsev;etydasnkktgsevv;tydasnkktgsevvk;ydasnkktgse
vvkk;dasnkktgsevvkkq;asnkktgsevvkkqg;snkktgsevvkkqgs;nkktgsevvkkqgsv;kk
tgsevvkkqgsvi;ktgsevvkkqgsvik;tgsevvkkqgsvike;gsevvkkqgsvikes;sevvkkqgs
vikesy;evvkkqgsvikesyk;vvkkqgsvikesyka;vkkqgsvikesykan;kkqgsvikesykank;
kqgsvikesykankl;qgsvikesykankld;gsvikesykanklds;svikesykankldsk;vikesyk
ankldskk;ikesykankldskki;kesykankldskkit;esykankldskkitr;sykankldskkitr
e;ykankldskkitren;kankldskkitrene;ankldskkitrenet;nkldskkitrenett;kldsk
kitrenettl;ldskkitrenettle;dskkitrenettley;skkitrenettleys;kkitrenettle
yse;kitrenettleysem;itrenettleysemt;trenettleysemtd;renettleysemtds;ene
ttleysemtdss;nettleysemtdssn;ettleysemtdssna;ttleysemtdssnat;tleysemtds
snatk;leysemtdssnatka;eysemtdssnatkav;ysemtdssnatkave;semtdssnatkavet;e
mtdssnatkavetlk;mtdssnatkavetlk;tdssnatkavetlkn;dssnatkavetlkng;ssnatkav
etlkngi;snatkavetlkngik;natkavetlkngikl;atkavetlkngikle;tkavetlkngikleg
;kavetlkngiklegs;avetlkngiklegsl;vetlkngiklegslv;etlkngiklegslvg;tlkngi
klegslvgg;lkngiklegslvggk;kngiklegslvggkt;ngiklegslvggktt;giklegslvggkt
tv;iklegslvggkttvk;klegslvggkttvkl;legslvggkttvklt;egslvggkttvklte;gslv
ggkttvklteg;slvggkttvkltegt;lvggkttvkltegti;vggkttvkltegtit;ggkttvklteg
titl;gkttvkltegtitlt;kttvkltegtitltr;ttvkltegtitltre;tvkltegtitltrei;vk
ltegtitltreie;kltegtitltreieq;ltegtitltreieqd;tegtitltreieqdg;egtitltre
ieqdgk;gtitltreieqdgkv;titltreieqdgkvk;itltreieqdgkvki;tltreieqdgkvkiy;
ltreieqdgkvkiyl;treieqdgkvkiyln;reieqdgkvkiylnd;eieqdgkvkiylndt;ieqdgkv
kiylndtt;eqdgkvkiylndtts;qdgkvkiylndttsg;dgkvkiylndttsgs;gkvkiylndttsgs
t;kvkiylndttsgstk;vkiylndttsgstkk;kiylndttsgstkkt;iylndttsgstkkta;ylndt
tsgstkktat;lndttsgstkktatw;ndttsgstkktatwn;dttsgstkktatwne;ttsgstkktatw
net;tsgstkktatwnett;sgstkktatwnettn;gstkktatwnettnt;stkktatwnettntl;tkk
tatwnettntlt;kktatwnettntlti;ktatwnettntltis;tatwnettntltisa;atwnettntl
tisad;twnettntltisads;wnettntltisadsk;nettntltisadskk;ettntltisadskkt;t
ntltisadskktk;tntltisadskktkd;ntltisadskktkdf;tltisadskktkdfv;ltisadsk
ktkdfvf;tisadskktkdfvfl;isadskktkdfvflt;sadskktkdfvfltd;adskktkdfvfltdg
;dskktkdfvfltdgt;skktkdfvfltdgti;kktkdfvfltdgtit;ktkdfvfltdgtitv;tkdfvf
ltdgtitvq;kdfvfltdgtitvqa;dfvfltdgtitvqay;fvfltdgtitvqayd;vfltdgtitvqay
dt;fltdgtitvqaydta;ltdgtitvqaydtag;tdgtitvqaydtagt;dgtitvqaydtagtk;gtit
vqaydtagtkl;titvqaydtagtkle;itvqaydtagtkleg;tvqaydtagtklegn;vqaydtagtkl
egns;qaydtagtklegnss;aydtagtklegnsse;ydtagtklegnssei;dtagtklegnsseik;ta
gtklegnsseikd;agtklegnsseikdl;gtklegnsseikdla;tklegnsseikdlaa;klegnssei
kdlaal;legnsseikdlaalk;egnsseikdlaalka;gnsseikdlaalkaa;nsseikdlaalkaal;
sseikdlaalkaalk;

16 mers:
mkqyllvfalvlalia;kqyllvfalvlaliac;qyllvfalvlaliacs;yllvfalvlaliacsq;llv
falvlaliacsqk;lvfalvlaliacsqkg;vfalvlaliacsqkgt;falvlaliacsqkgte;alvlal
iacsqkgtep;lvlaliacsqkgtepk;vlaliacsqkgtepks;laliacsqkgtepkst;aliacsqkg
tepksts;liacsqkgtepkstsq;iacsqkgtepkstsqd;acsqkgtepkstsqdh;csqkgtepksts
qdhn;sqkgtepkstsqdhnd;qkgtepkstsqdhndq;kgtepkstsqdhndqe;gtepkstsqdhndqe
i;tepkstsqdhndqeii;epkstsqdhndqeiin;pkstsqdhndqeiins;kstsqdhndqeiinsd;s
tsqdhndqeiinsdn;tsqdhndqeiinsdnt;sqdhndqeiinsdntp;qdhndqeiinsdntpk;dhnd

qeiinsdntpkd;hndqeiinsdntpkds;ndqeiinsdntpkdsk;dqeiinsdntpkdskk;qeiinsd
ntpkdskkd;eiinsdntpkdskkdl;iinsdntpkdskkdlt;insdntpkdskkdltv;nsdntpkdsk
kdltvl;sdntpkdskkdltvla;dntpkdskkdltvlae;ntpkdskkdltvlaee;tpkdskkdltvla
een;pkdskkdltvlaeens;kdskkdltvlaeensv;dskkdltvlaeensvp;skkdltvlaeensvpl
;kkdltvlaeensvplf;kdltvlaeensvplfn;dltvlaeensvplfng;ltvlaeensvplfngn;tv
laeensvplfngnk;vlaeensvplfngnki;laeensvplfngnkif;aeensvplfngnkifv;eensv
plfngnkifvs;ensvplfngnkifvsk;nsvplfngnkifvske;svplfngnkifvskek;vplfngnk
ifvskekn;plfngnkifvskekns;lfngnkifvskeknsa;fngnkifvskeknsag;ngnkifvskek
nsagk;gnkifvskeknsagky;nkifvskeknsagkye;kifvskeknsagkyel;ifvskeknsagkye
lr;fvskeknsagkyelra;vskeknsagkyelrat;skeknsagkyelratv;keknsagkyelratvd;
eknsagkyelratvdt;knsagkyelratvdtv;nsagkyelratvdtve;sagkyelratvdtvel;agk
yelratvdtvelk;gkyelratvdtvelkg;kyelratvdtvelkgv;yelratvdtvelkgvs;elratv
dtvelkgvsd;lratvdtvelkgvsdk;ratvdtvelkgvsdkn;atvdtvelkgvsdknn;tvdtvelkg
vsdknng;vdtvelkgvsdknngs;dtvelkgvsdknngsg;tvelkgvsdknngsgk;velkgvsdknng
sgkl;elkgvsdknngsgkle;lkgvsdknngsgkleg;kgvsdknngsgklegt;gvsdknngsgklegt
k;vsdknngsgklegtka;sdknngsgklegtkad;dknngsgklegtkadk;knngsgklegtkadkt;n
ngsgklegtkadktk;ngsgklegtkadktkv;gsgklegtkadktkva;sgklegtkadktkvam;gkle
gtkadktkvamt;klegtkadktkvamti;legtkadktkvamtia;egtkadktkvamtiad;gtkadkt
kvamtiadd;tkadktkvamtiaddl;kadktkvamtiaddln;adktkvamtiaddlnt;dktkvamtia
ddlnti;ktkvamtiaddlntit;tkvamtiaddlntitv;kvamtiaddlntitve;vamtiaddlntit
vet;amtiaddlntitvety;mtiaddlntitvetyd;tiaddlntitvetyda;iaddlntitvetydas
;addlntitvetydasn;ddlntitvetydasnk;dlntitvetydasnkk;lntitvetydasnkkt;nt
itvetydasnkktg;titvetydasnkktgs;itvetydasnkktgse;tvetydasnkktgsev;vetyd
asnkktgsevv;etydasnkktgsevvk;tydasnkktgsevvkk;ydasnkktgsevvkkq;dasnkktg
sevvkkqg;asnkktgsevvkkqgs;snkktgsevvkkqgsv;nkktgsevvkkqgsvi;kktgsevvkkq
gsvik;ktgsevvkkqgsvike;tgsevvkkqgsvikes;gsevvkkqgsvikesy;sevvkkqgsvikes
yk;evvkkqgsvikesyka;vvkkqgsvikesykan;vkkqgsvikesykank;kkqgsvikesykankl;
kqgsvikesykankld;qgsvikesykanklds;gsvikesykankldsk;svikesykankldskk;vik
esykankldskki;ikesykankldskkit;kesykankldskkitr;esykankldskkitre;sykank
ldskkitren;ykankldskkitrene;kankldskkitrenet;ankldskkitrenett;nkldskkit
renettl;kldskkitrenettle;ldskkitrenettley;dskkitrenettleys;skkitrenettl
eyse;kkitrenettleysem;kitrenettleysemt;itrenettleysemtd;trenettleysemtd
s;renettleysemtdss;enettleysemtdssn;nettleysemtdssna;ettleysemtdssnat;t
tleysemtdssnatk;tleysemtdssnatka;leysemtdssnatkav;eysemtdssnatkave;ysem
tdssnatkavet;semtdssnatkavetl;emtdssnatkavetlk;mtdssnatkavetlkn;tdssnat
kavetlkng;dssnatkavetlkngi;ssnatkavetlkngik;snatkavetlkngikl;natkavetlk
ngikle;atkavetlkngikleg;tkavetlkngiklegs;kavetlkngiklegsl;avetlkngikleg
slv;vetlkngiklegslvg;etlkngiklegslvgg;tlkngiklegslvggk;lkngiklegslvggkt
;kngiklegslvggktt;ngiklegslvggkttv;giklegslvggkttvk;iklegslvggkttvkl;kl
egslvggkttvklt;legslvggkttvklte;egslvggkttvklteg;gslvggkttvkltegt;slvgg
kttvkltegti;lvggkttvkltegtit;vggkttvkltegtitl;ggkttvkltegtitlt;gkttvklt
egtitltr;kttvkltegtitltre;ttvkltegtitltrei;tvkltegtitltreie;vkltegtitlt
reieq;kltegtitltreieqd;ltegtitltreieqdg;tegtitltreieqdgk;egtitltreieqdg
kv;gtitltreieqdgkvk;titltreieqdgkvki;itltreieqdgkvkiy;tltreieqdgkvkiyl;
ltreieqdgkvkiyln;treieqdgkvkiylnd;reieqdgkvkiylndt;eieqdgkvkiylndtt;ieq
dgkvkiylndtts;eqdgkvkiylndttsg;qdgkvkiylndttsgs;dgkvkiylndttsgst;gkvkiy
lndttsgstk;kvkiylndttsgstkk;vkiylndttsgstkkt;kiylndttsgstkkta;iylndttsg
stkktat;ylndttsgstkktatw;lndttsgstkktatwn;ndttsgstkktatwne;dttsgstkktat
wnet;ttsgstkktatwnett;tsgstkktatwnettn;sgstkktatwnettnt;gstkktatwnettnt
l;stkktatwnettntlt;tkktatwnettntlti;kktatwnettntltis;ktatwnettntltisa;t
atwnettntltisad;atwnettntltisads;twnettntltisadsk;wnettntltisadskk;nett
ntltisadskkt;ettntltisadskktk;ttntltisadskktkd;tntltisadskktkdf;ntltisa
dskktkdfv;tltisadskktkdfvf;ltisadskktkdfvfl;tisadskktkdfvflt;isadskktkd
fvfltd;sadskktkdfvfltdg;adskktkdfvfltdgt;dskktkdfvfltdgti;skktkdfvfltdg
tit;kktkdfvfltdgtitv;ktkdfvfltdgtitvq;tkdfvfltdgtitvqa;kdfvfltdgtitvqay
;dfvfltdgtitvqayd;fvfltdgtitvqaydt;vfltdgtitvqaydta;fltdgtitvqaydtag;lt
dgtitvqaydtagt;tdgtitvqaydtagtk;dgtitvqaydtagtkl;gtitvqaydtagtkle;titvq

aydtagtkleg;itvqaydtagtklegn;tvqaydtagtklegns;vqaydtagtklegnss;qaydtagt
klegnsse;aydtagtklegnssei;ydtagtklegnsseik;dtagtklegnsseikd;tagtklegnss
eikdl;agtklegnsseikdla;gtklegnsseikdlaa;tklegnsseikdlaal;klegnsseikdlaa
lk;legnsseikdlaalka;egnsseikdlaalkaa;gnsseikdlaalkaal;nsseikdlaalkaalk;


## <NP_045689 outer surface protein B;Protein;Borrelia burgdorferi B31>

8 mers:
mrlligfa;rlligfal;lligfala;ligfalal;igfalala;gfalalal;falalali;alalalig
;lalaligc;alaligca;laligcaq;aligcaqk;ligcaqkg;igcaqkga;gcaqkgae;caqkgae
s;aqkgaesi;qkgaesig;kgaesigs;gaesigsq;aesigsqk;esigsqke;sigsqken;igsqke
nd;gsqkendl;sqkendln;qkendlnl;kendlnle;endlnled;ndlnleds;dlnledss;lnled
ssk;nledsskk;ledsskks;edsskksh;dsskkshq;sskkshqn;skkshqna;kkshqnak;kshq
nakq;shqnakqd;hqnakqdl;qnakqdlp;nakqdlpa;akqdlpav;kqdlpavt;qdlpavte;dlp
avted;lpavteds;pavtedsv;avtedsvs;vtedsvsl;tedsvslf;edsvslfn;dsvslfng;sv
slfngn;vslfngnk;slfngnki;lfngnkif;fngnkifv;ngnkifvs;gnkifvsk;nkifvske;k
ifvskek;ifvskekn;fvskekns;vskeknss;skeknssg;keknssgk;eknssgky;knssgkyd;
nssgkydl;ssgkydlr;sgkydlra;gkydlrat;kydlrati;ydlratid;dlratidq;lratidqv
;ratidqve;atidqvel;tidqvelk;idqvelkg;dqvelkgt;qvelkgts;velkgtsd;elkgtsd
k;lkgtsdkn;kgtsdknn;gtsdknng;tsdknngs;sdknngsg;dknngsgt;knngsgtl;nngsgt
le;ngsgtleg;gsgtlegs;sgtlegsk;gtlegskp;tlegskpd;legskpdk;egskpdks;gskpd
ksk;skpdkskv;kpdkskvk;pdkskvkl;dkskvklt;kskvkltv;skvkltvs;kvkltvsa;vklt
vsad;kltvsadl;ltvsadln;tvsadlnt;vsadlntv;sadlntvt;adlntvtl;dlntvtle;lnt
vtlea;ntvtleaf;tvtleafd;vtleafda;tleafdas;leafdasn;eafdasnq;afdasnqk;fd
asnqki;dasnqkis;asnqkiss;snqkissk;nqkisskv;qkisskvt;kisskvtk;isskvtkk;s
skvtkkq;skvtkkqg;kvtkkqgs;vtkkqgsi;tkkqgsit;kkqgsitx;kqgsitxe;qgsitxet;
gsitxetl;sitxetlk;itxetlka;txetlkan;xetlkank;etlkankl;tlkankld;lkanklds
;kankldsk;ankldskk;nkldskkl;kldskklt;ldskkltr;dskkltrs;skkltrsn;kkltrsn
g;kltrsngt;ltrsngtt;trsngttl;rsngttle;sngttley;ngttleys;gttleysq;ttleys
qi;tleysqit;leysqitd;eysqitda;ysqitdad;sqitdadn;qitdadna;itdadnat;tdadn
atk;dadnatka;adnatkav;dnatkave;natkavet;atkavetl;tkavetlk;kavetlkn;avet
lkns;vetlknsi;etlknsik;tlknsikl;lknsikle;knsikleg;nsiklegs;siklegsl;ikl
egslv;klegslvg;legslvgg;egslvggk;gslvggkt;slvggktt;lvggkttv;vggkttve;gg
kttvei;gkttveik;kttveike;ttveikeg;tveikegt;veikegtv;eikegtvt;ikegtvtl;k
egtvtlk;egtvtlkr;gtvtlkre;tvtlkrei;vtlkreie;tlkreiek;lkreiekd;kreiekdg;
reiekdgk;eiekdgkv;iekdgkvk;ekdgkvkv;kdgkvkvf;dgkvkvfl;gkvkvfln;kvkvflnd
;vkvflndt;kvflndta;vflndtag;flndtags;lndtagsn;ndtagsnk;dtagsnkk;tagsnkk
t;agsnkktg;gsnkktgk;snkktgkw;nkktgkwe;kktgkwed;ktgkweds;tgkwedst;gkweds
ts;kwedstst;wedststl;edststlt;dststlti;ststltis;tstltisa;stltisad;tltis
ads;ltisadsk;tisadskk;isadskkt;sadskktk;adskktkd;dskktkdl;skktkdlv;kktk
dlvf;ktkdlvfl;tkdlvflt;kdlvfltd;dlvfltdg;lvfltdgt;vfltdgti;fltdgtit;ltd
gtitv;tdgtitvq;dgtitvqq;gtitvqqy;titvqqyn;itvqqynt;tvqqynta;vqqyntag;qq
yntagt;qyntagts;yntagtsl;ntagtsle;tagtsleg;agtslegs;gtslegsa;tslegsas;s
legsase;legsasei;egsaseik;gsaseikn;saseiknl;aseiknls;seiknlse;eiknlsel;
iknlselk;knlselkn;nlselkna;lselknal;selknalk;

9 mers:
mrlligfal;rlligfala;lligfalal;ligfalala;igfalalal;gfalalali;falalalig;a
lalaligc;lalaligca;alaligcaq;laligcaqk;aligcaqkg;ligcaqkga;igcaqkgae;gc
aqkgaes;caqkgaesi;aqkgaesig;qkgaesigs;kgaesigsq;gaesigsqk;aesigsqke;esi
gsqken;sigsqkend;igsqkendl;gsqkendln;sqkendlnl;qkendlnle;kendlnled;endl
nleds;ndlnledss;dlnledssk;lnledsskk;nledsskks;ledsskksh;edsskkshq;dsskk
shqn;sskkshqna;skkshqnak;kkshqnakq;kshqnakqd;shqnakqdl;hqnakqdlp;qnakqd
lpa;nakqdlpav;akqdlpavt;kqdlpavte;qdlpavted;dlpavteds;lpavtedsv;pavteds
vs;avtedsvsl;vtedsvslf;tedsvslfn;edsvslfng;dsvslfngn;svslfngnk;vslfngnk

i;slfngnkif;lfngnkifv;fngnkifvs;ngnkifvsk;gnkifvske;nkifvskek;kifvskekn
;ifvskekns;fvskeknss;vskeknssg;skeknssgk;keknssgky;eknssgkyd;knssgkydl;
nssgkydlr;ssgkydlra;sgkydlrat;gkydlrati;kydlratid;ydlratidq;dlratidqv;l
ratidqve;ratidqvel;atidqvelk;tidqvelkg;idqvelkgt;dqvelkgts;qvelkgtsd;ve
lkgtsdk;elkgtsdkn;lkgtsdknn;kgtsdknng;gtsdknngs;tsdknngsg;sdknngsgt;dkn
ngsgtl;knngsgtle;nngsgtleg;ngsgtlegs;gsgtlegsk;sgtlegskp;gtlegskpd;tleg
skpdk;legskpdks;egskpdksk;gskpdkskv;skpdkskvk;kpdkskvkl;pdkskvklt;dkskv
kltv;kskvkltvs;skvkltvsa;kvkltvsad;vkltvsadl;kltvsadln;ltvsadlnt;tvsadl
ntv;vsadlntvt;sadlntvtl;adlntvtle;dlntvtlea;lntvtleaf;ntvtleafd;tvtleaf
da;vtleafdas;tleafdasn;leafdasnq;eafdasnqk;afdasnqki;fdasnqkis;dasnqkis
s;asnqkissk;snqkisskv;nqkisskvt;qkisskvtk;kisskvtkk;isskvtkkq;sskvtkkqg
;skvtkkqgs;kvtkkqgsi;vtkkqgsit;tkkqgsitx;kkqgsitxe;kqgsitxet;qgsitxetl;
gsitxetlk;sitxetlka;itxetlkan;txetlkank;xetlkankl;etlkankld;tlkanklds;l
kankldsk;kankldskk;ankldskkl;nkldskklt;kldskkltr;ldskkltrs;dskkltrsn;sk
kltrsng;kkltrsngt;kltrsngtt;ltrsngttl;trsngttle;rsngttley;sngttleys;ngt
tleysq;gttleysqi;ttleysqit;tleysqitd;leysqitda;eysqitdad;ysqitdadn;sqit
dadna;qitdadnat;itdadnatk;tdadnatka;dadnatkav;adnatkave;dnatkavet;natka
vetl;atkavetlk;tkavetlkn;kavetlkns;avetlknsi;vetlknsik;etlknsikl;tlknsi
kle;lknsikleg;knsiklegs;nsiklegsl;siklegslv;iklegslvg;klegslvgg;legslvg
gk;egslvggkt;gslvggktt;slvggkttv;lvggkttve;vggkttvei;ggkttveik;gkttveik
e;kttveikeg;ttveikegt;tveikegtv;veikegtvt;eikegtvtl;ikegtvtlk;kegtvtlkr
;egtvtlkre;gtvtlkrei;tvtlkreie;vtlkreiek;tlkreiekd;lkreiekdg;kreiekdgk;
reiekdgkv;eiekdgkvk;iekdgkvkv;ekdgkvkvf;kdgkvkvfl;dgkvkvfln;gkvkvflnd;k
vkvflndt;vkvflndta;kvflndtag;vflndtags;flndtagsn;lndtagsnk;ndtagsnkk;dt
agsnkkt;tagsnkktg;agsnkktgk;gsnkktgkw;snkktgkwe;nkktgkwed;kktgkweds;ktg
kwedst;tgkwedsts;gkwedstst;kwedststl;wedststlt;edststlti;dststltis;stst
ltisa;tstltisad;stltisads;tltisadsk;ltisadskk;tisadskkt;isadskktk;sadsk
ktkd;adskktkdl;dskktkdlv;skktkdlvf;kktkdlvfl;ktkdlvflt;tkdlvfltd;kdlvfl
tdg;dlvfltdgt;lvfltdgti;vfltdgtit;fltdgtitv;ltdgtitvq;tdgtitvqq;dgtitvq
qy;gtitvqqyn;titvqqynt;itvqqynta;tvqqyntag;vqqyntagt;qqyntagts;qyntagts
l;yntagtsle;ntagtsleg;tagtslegs;agtslegsa;gtslegsas;tslegsase;slegsasei
;legsaseik;egsaseikn;gsaseiknl;saseiknls;aseiknlse;seiknlsel;eiknlselk;
iknlselkn;knlselkna;nlselknal;lselknalk;

10 mers:
mrlligfala;rlligfalal;lligfalala;ligfalalal;igfalalali;gfalalalig;falal
aligc;alalaligca;lalaligcaq;alaligcaqk;laligcaqkg;aligcaqkga;ligcaqkgae
;igcaqkgaes;gcaqkgaesi;caqkgaesig;aqkgaesigs;qkgaesigsq;kgaesigsqk;gaes
igsqke;aesigsqken;esigsqkend;sigsqkendl;igsqkendln;gsqkendlnl;sqkendlnl
e;qkendlnled;kendlnleds;endlnledss;ndlnledssk;dlnledsskk;lnledsskks;nle
dsskksh;ledsskkshq;edsskkshqn;dsskkshqna;sskkshqnak;skkshqnakq;kkshqnak
qd;kshqnakqdl;shqnakqdlp;hqnakqdlpa;qnakqdlpav;nakqdlpavt;akqdlpavte;kq
dlpavted;qdlpavteds;dlpavtedsv;lpavtedsvs;pavtedsvsl;avtedsvslf;vtedsvs
lfn;tedsvslfng;edsvslfngn;dsvslfngnk;svslfngnki;vslfngnkif;slfngnkifv;l
fngnkifvs;fngnkifvsk;ngnkifvske;gnkifvskek;nkifvskekn;kifvskekns;ifvske
knss;fvskeknssg;vskeknssgk;skeknssgky;keknssgkyd;eknssgkydl;knssgkydlr;
nssgkydlra;ssgkydlrat;sgkydlrati;gkydlratid;kydlratidq;ydlratidqv;dlrat
idqve;lratidqvel;ratidqvelk;atidqvelkg;tidqvelkgt;idqvelkgts;dqvelkgtsd
;qvelkgtsdk;velkgtsdkn;elkgtsdknn;lkgtsdknng;kgtsdknngs;gtsdknngsg;tsdk
nngsgt;sdknngsgtl;dknngsgtle;knngsgtleg;nngsgtlegs;ngsgtlegsk;gsgtlegsk
p;sgtlegskpd;gtlegskpdk;tlegskpdks;legskpdksk;egskpdkskv;gskpdkskvk;skp
dkskvkl;kpdkskvklt;pdkskvkltv;dkskvkltvs;kskvkltvsa;skvkltvsad;kvkltvsa
dl;vkltvsadln;kltvsadlnt;ltvsadlntv;tvsadlntvt;vsadlntvtl;sadlntvtle;ad
lntvtlea;dlntvtleaf;lntvtleafd;ntvtleafda;tvtleafdas;vtleafdasn;tleafda
snq;leafdasnqk;eafdasnqki;afdasnqkis;fdasnqkiss;dasnqkissk;asnqkisskv;s
nqkisskvt;nqkisskvtk;qkisskvtkk;kisskvtkkq;isskvtkkqg;sskvtkkqgs;skvtkk
qgsi;kvtkkqgsit;vtkkqgsitx;tkkqgsitxe;kkqgsitxet;kqgsitxetl;qgsitxetlk;

gsitxetlka;sitxetlkan;itxetlkank;txetlkankl;xetlkankld;etlkanklds;tlkan
kldsk;lkankldskk;kankldskkl;ankldskklt;nkldskkltr;kldskkltrs;ldskkltrsn
;dskkltrsng;skkltrsngt;kkltrsngtt;kltrsngttl;ltrsngttle;trsngttley;rsng
ttleys;sngttleysq;ngttleysqi;gttleysqit;ttleysqitd;tleysqitda;leysqitda
d;eysqitdadn;ysqitdadna;sqitdadnat;qitdadnatk;itdadnatka;tdadnatkav;dad
natkave;adnatkavet;dnatkavetl;natkavetlk;atkavetlkn;tkavetlkns;kavetlkn
si;avetlknsik;vetlknsikl;etlknsikle;tlknsikleg;lknsiklegs;knsiklegsl;ns
iklegslv;siklegslvg;iklegslvgg;klegslvggk;legslvggkt;egslvggktt;gslvggk
ttv;slvggkttve;lvggkttvei;vggkttveik;ggkttveike;gkttveikeg;kttveikegt;t
tveikegtv;tveikegtvt;veikegtvtl;eikegtvtlk;ikegtvtlkr;kegtvtlkre;egtvtl
krei;gtvtlkreie;tvtlkreiek;vtlkreiekd;tlkreiekdg;lkreiekdgk;kreiekdgkv;
reiekdgkvk;eiekdgkvkv;iekdgkvkvf;ekdgkvkvfl;kdgkvkvfln;dgkvkvflnd;gkvkv
flndt;kvkvflndta;vkvflndtag;kvflndtags;vflndtagsn;flndtagsnk;lndtagsnkk
;ndtagsnkkt;dtagsnkktg;tagsnkktgk;agsnkktgkw;gsnkktgkwe;snkktgkwed;nkkt
gkweds;kktgkwedst;ktgkwedsts;tgkwedstst;gkwedststl;kwedststlt;wedststlt
i;edststltis;dststltisa;ststltisad;tstltisads;stltisadsk;tltisadskk;lti
sadskkt;tisadskktk;isadskktkd;sadskktkdl;adskktkdlv;dskktkdlvf;skktkdlv
fl;kktkdlvflt;ktkdlvfltd;tkdlvfltdg;kdlvfltdgt;dlvfltdgti;lvfltdgtit;vf
ltdgtitv;fltdgtitvq;ltdgtitvqq;tdgtitvqqy;dgtitvqqyn;gtitvqqynt;titvqqy
nta;itvqqyntag;tvqqyntagt;vqqyntagts;qqyntagtsl;qyntagtsle;yntagtsleg;n
tagtslegs;tagtslegsa;agtslegsas;gtslegsase;tslegsasei;slegsaseik;legsas
eikn;egsaseiknl;gsaseiknls;saseiknlse;aseiknlsel;seiknlselk;eiknlselkn;
iknlselkna;knlselknal;nlselknalk;

11 mers:
mrlligfalal;rlligfalala;lligfalalal;ligfalalali;igfalalalig;gfalalaligc
;falalaligca;alalaligcaq;lalaligcaqk;alaligcaqkg;laligcaqkga;aligcaqkga
e;ligcaqkgaes;igcaqkgaesi;gcaqkgaesig;caqkgaesigs;aqkgaesigsq;qkgaesigs
qk;kgaesigsqke;gaesigsqken;aesigsqkend;esigsqkendl;sigsqkendln;igsqkend
lnl;gsqkendlnle;sqkendlnled;qkendlnleds;kendlnledss;endlnledssk;ndlnled
sskk;dlnledsskks;lnledsskksh;nledsskkshq;ledsskkshqn;edsskkshqna;dsskks
hqnak;sskkshqnakq;skkshqnakqd;kkshqnakqdl;kshqnakqdlp;shqnakqdlpa;hqnak
qdlpav;qnakqdlpavt;nakqdlpavte;akqdlpavted;kqdlpavteds;qdlpavtedsv;dlpa
vtedsvs;lpavtedsvsl;pavtedsvslf;avtedsvslfn;vtedsvslfng;tedsvslfngn;eds
vslfngnk;dsvslfngnki;svslfngnkif;vslfngnkifv;slfngnkifvs;lfngnkifvsk;fn
gnkifvske;ngnkifvskek;gnkifvskekn;nkifvskekns;kifvskeknss;ifvskeknssg;f
vskeknssgk;vskeknssgky;skeknssgkyd;keknssgkydl;eknssgkydlr;knssgkydlra;
nssgkydlrat;ssgkydlrati;sgkydlratid;gkydlratidq;kydlratidqv;ydlratidqve
;dlratidqvel;lratidqvelk;ratidqvelkg;atidqvelkgt;tidqvelkgts;idqvelkgts
d;dqvelkgtsdk;qvelkgtsdkn;velkgtsdknn;elkgtsdknng;lkgtsdknngs;kgtsdknng
sg;gtsdknngsgt;tsdknngsgtl;sdknngsgtle;dknngsgtleg;knngsgtlegs;nngsgtle
gsk;ngsgtlegskp;gsgtlegskpd;sgtlegskpdk;gtlegskpdks;tlegskpdksk;legskpd
kskv;egskpdkskvk;gskpdkskvkl;skpdkskvklt;kpdkskvkltv;pdkskvkltvs;dkskvk
ltvsa;kskvkltvsad;skvkltvsadl;kvkltvsadln;vkltvsadlnt;kltvsadlntv;ltvsa
dlntvt;tvsadlntvtl;vsadlntvtle;sadlntvtlea;adlntvtleaf;dlntvtleafd;lntv
tleafda;ntvtleafdas;tvtleafdasn;vtleafdasnq;tleafdasnqk;leafdasnqki;eaf
dasnqkis;afdasnqkiss;fdasnqkissk;dasnqkisskv;asnqkisskvt;snqkisskvtk;nq
kisskvtkk;qkisskvtkkq;kisskvtkkqg;isskvtkkqgs;sskvtkkqgsi;skvtkkqgsit;k
vtkkqgsitx;vtkkqgsitxe;tkkqgsitxet;kkqgsitxetl;kqgsitxetlk;qgsitxetlka;
gsitxetlkan;sitxetlkank;itxetlkankl;txetlkankld;xetlkanklds;etlkankldsk
;tlkankldskk;lkankldskkl;kankldskklt;ankldskkltr;nkldskkltrs;kldskkltrs
n;ldskkltrsng;dskkltrsngt;skkltrsngtt;kkltrsngttl;kltrsngttle;ltrsngttl
ey;trsngttleys;rsngttleysq;sngttleysqi;ngttleysqit;gttleysqitd;ttleysqi
tda;tleysqitdad;leysqitdadn;eysqitdadna;ysqitdadnat;sqitdadnatk;qitdadn
atka;itdadnatkav;tdadnatkave;dadnatkavet;adnatkavetl;dnatkavetlk;natkav
etlkn;atkavetlkns;tkavetlknsi;kavetlknsik;avetlknsikl;vetlknsikle;etlkn
sikleg;tlknsiklegs;lknsiklegsl;knsiklegslv;nsiklegslvg;siklegslvgg;ikle

gslvggk;klegslvggkt;legslvggktt;egslvggkttv;gslvggkttve;slvggkttvei;lvg
gkttveik;vggkttveike;ggkttveikeg;gkttveikegt;kttveikegtv;ttveikegtvt;tv
eikegtvtl;veikegtvtlk;eikegtvtlkr;ikegtvtlkre;kegtvtlkrei;egtvtlkreie;g
tvtlkreiek;tvtlkreiekd;vtlkreiekdg;tlkreiekdgk;lkreiekdgkv;kreiekdgkvk;
reiekdgkvkv;eiekdgkvkvf;iekdgkvkvfl;ekdgkvkvfln;kdgkvkvflnd;dgkvkvflndt
;gkvkvflndta;kvkvflndtag;vkvflndtags;kvflndtagsn;vflndtagsnk;flndtagsnk
k;lndtagsnkkt;ndtagsnkktg;dtagsnkktgk;tagsnkktgkw;agsnkktgkwe;gsnkktgkw
ed;snkktgkweds;nkktgkwedst;kktgkwedsts;ktgkwedstst;tgkwedststl;gkwedsts
tlt;kwedststlti;wedststltis;edststltisa;dststltisad;ststltisads;tstltis
adsk;stltisadskk;tltisadskkt;ltisadskktk;tisadskktkd;isadskktkdl;sadskk
tkdlv;adskktkdlvf;dskktkdlvfl;skktkdlvflt;kktkdlvfltd;ktkdlvfltdg;tkdlv
fltdgt;kdlvfltdgti;dlvfltdgtit;lvfltdgtitv;vfltdgtitvq;fltdgtitvqq;ltdg
titvqqy;tdgtitvqqyn;dgtitvqqynt;gtitvqqynta;titvqqyntag;itvqqyntagt;tvq
qyntagts;vqqyntagtsl;qqyntagtsle;qyntagtsleg;yntagtslegs;ntagtslegsa;ta
gtslegsas;agtslegsase;gtslegsasei;tslegsaseik;slegsaseikn;legsaseiknl;e
gsaseiknls;gsaseiknlse;saseiknlsel;aseiknlselk;seiknlselkn;eiknlselkna;
iknlselknal;knlselknalk;

13 mers:
mrlligfalalal;rlligfalalali;lligfalalalig;ligfalalaligc;igfalalaligca;g
falalaligcaq;falalaligcaqk;alalaligcaqkg;lalaligcaqkga;alaligcaqkgae;la
ligcaqkgaes;aligcaqkgaesi;ligcaqkgaesig;igcaqkgaesigs;gcaqkgaesigsq;caq
kgaesigsqk;aqkgaesigsqke;qkgaesigsqken;kgaesigsqkend;gaesigsqkendl;aesi
gsqkendln;esigsqkendlnl;sigsqkendlnle;igsqkendlnled;gsqkendlnleds;sqken
dlnledss;qkendlnledssk;kendlnledsskk;endlnledsskks;ndlnledsskksh;dlnled
sskkshq;lnledsskkshqn;nledsskkshqna;ledsskkshqnak;edsskkshqnakq;dsskksh
qnakqd;sskkshqnakqdl;skkshqnakqdlp;kkshqnakqdlpa;kshqnakqdlpav;shqnakqd
lpavt;hqnakqdlpavte;qnakqdlpavted;nakqdlpavteds;akqdlpavtedsv;kqdlpavte
dsvs;qdlpavtedsvsl;dlpavtedsvslf;lpavtedsvslfn;pavtedsvslfng;avtedsvslf
ngn;vtedsvslfngnk;tedsvslfngnki;edsvslfngnkif;dsvslfngnkifv;svslfngnkif
vs;vslfngnkifvsk;slfngnkifvske;lfngnkifvskek;fngnkifvskekn;ngnkifvskekn
s;gnkifvskeknss;nkifvskeknssg;kifvskeknssgk;ifvskeknssgky;fvskeknssgkyd
;vskeknssgkydl;skeknssgkydlr;keknssgkydlra;eknssgkydlrat;knssgkydlrati;
nssgkydlratid;ssgkydlratidq;sgkydlratidqv;gkydlratidqve;kydlratidqvel;y
dlratidqvelk;dlratidqvelkg;lratidqvelkgt;ratidqvelkgts;atidqvelkgtsd;ti
dqvelkgtsdk;idqvelkgtsdkn;dqvelkgtsdknn;qvelkgtsdknng;velkgtsdknngs;elk
gtsdknngsg;lkgtsdknngsgt;kgtsdknngsgtl;gtsdknngsgtle;tsdknngsgtleg;sdkn
ngsgtlegs;dknngsgtlegsk;knngsgtlegskp;nngsgtlegskpd;ngsgtlegskpdk;gsgtl
egskpdks;sgtlegskpdksk;gtlegskpdkskv;tlegskpdkskvk;legskpdkskvkl;egskpd
kskvklt;gskpdkskvkltv;skpdkskvkltvs;kpdkskvkltvsa;pdkskvkltvsad;dkskvkl
tvsadl;kskvkltvsadln;skvkltvsadlnt;kvkltvsadlntv;vkltvsadlntvt;kltvsadl
ntvtl;ltvsadlntvtle;tvsadlntvtlea;vsadlntvtleaf;sadlntvtleafd;adlntvtle
afda;dlntvtleafdas;lntvtleafdasn;ntvtleafdasnq;tvtleafdasnqk;vtleafdasn
qki;tleafdasnqkis;leafdasnqkiss;eafdasnqkissk;afdasnqkisskv;fdasnqkissk
vt;dasnqkisskvtk;asnqkisskvtkk;snqkisskvtkkq;nqkisskvtkkqg;qkisskvtkkqg
s;kisskvtkkqgsi;isskvtkkqgsit;sskvtkkqgsitx;skvtkkqgsitxe;kvtkkqgsitxet
;vtkkqgsitxetl;tkkqgsitxetlk;kkqgsitxetlka;kqgsitxetlkan;qgsitxetlkank;
gsitxetlkankl;sitxetlkankld;itxetlkanklds;txetlkankldsk;xetlkankldskk;e
tlkankldskkl;tlkankldskklt;lkankldskkltr;kankldskkltrs;ankldskkltrsn;nk
ldskkltrsng;kldskkltrsngt;ldskkltrsngtt;dskkltrsngttl;skkltrsngttle;kkl
trsngttley;kltrsngttleys;ltrsngttleysq;trsngttleysqi;rsngttleysqit;sngt
tleysqitd;ngttleysqitda;gttleysqitdad;ttleysqitdadn;tleysqitdadna;leysq
itdadnat;eysqitdadnatk;ysqitdadnatka;sqitdadnatkav;qitdadnatkave;itdadn
atkavet;tdadnatkavetl;dadnatkavetlk;adnatkavetlkn;dnatkavetlkns;natkave
tlknsi;atkavetlknsik;tkavetlknsikl;kavetlknsikle;avetlknsikleg;vetlknsi
klegs;etlknsiklegsl;tlknsiklegslv;lknsiklegslvg;knsiklegslvgg;nsiklegsl
vggk;siklegslvggkt;iklegslvggktt;klegslvggkttv;legslvggkttve;egslvggktt

vei;gslvggkttveik;slvggkttveike;lvggkttveikeg;vggkttveikegt;ggkttveikeg
tv;gkttveikegtvt;kttveikegtvtl;ttveikegtvtlk;tveikegtvtlkr;veikegtvtlkr
e;eikegtvtlkrei;ikegtvtlkreie;kegtvtlkreiek;egtvtlkreiekd;gtvtlkreiekdg
;tvtlkreiekdgk;vtlkreiekdgkv;tlkreiekdgkvk;lkreiekdgkvkv;kreiekdgkvkvf;
reiekdgkvkvfl;eiekdgkvkvfln;iekdgkvkvflnd;ekdgkvkvflndt;kdgkvkvflndta;d
gkvkvflndtag;gkvkvflndtags;kvkvflndtagsn;vkvflndtagsnk;kvflndtagsnkk;vf
lndtagsnkkt;flndtagsnkktg;lndtagsnkktgk;ndtagsnkktgkw;dtagsnkktgkwe;tag
snkktgkwed;agsnkktgkweds;gsnkktgkwedst;snkktgkwedsts;nkktgkwedstst;kktg
kwedststl;ktgkwedststlt;tgkwedststlti;gkwedststltis;kwedststltisa;wedst
stltisad;edststltisads;dststltisadsk;ststltisadskk;tstltisadskkt;stltis
adskktk;tltisadskktkd;ltisadskktkdl;tisadskktkdlv;isadskktkdlvf;sadskkt
kdlvfl;adskktkdlvflt;dskktkdlvfltd;skktkdlvfltdg;kktkdlvfltdgt;ktkdlvfl
tdgti;tkdlvfltdgtit;kdlvfltdgtitv;dlvfltdgtitvq;lvfltdgtitvqq;vfltdgtit
vqqy;fltdgtitvqqyn;ltdgtitvqqynt;tdgtitvqqynta;dgtitvqqyntag;gtitvqqynt
agt;titvqqyntagts;itvqqyntagtsl;tvqqyntagtsle;vqqyntagtsleg;qqyntagtsle
gs;qyntagtslegsa;yntagtslegsas;ntagtslegsase;tagtslegsasei;agtslegsasei
k;gtslegsaseikn;tslegsaseiknl;slegsaseiknls;legsaseiknlse;egsaseiknlsel
;gsaseiknlselk;saseiknlselkn;aseiknlselkna;seiknlselknal;eiknlselknalk;

14 mers:
mrlligfalalali;rlligfalalalig;lligfalalaligc;ligfalalaligca;igfalalalig
caq;gfalalaligcaqk;falalaligcaqkg;alalaligcaqkga;lalaligcaqkgae;alaligc
aqkgaes;laligcaqkgaesi;aligcaqkgaesig;ligcaqkgaesigs;igcaqkgaesigsq;gca
qkgaesigsqk;caqkgaesigsqke;aqkgaesigsqken;qkgaesigsqkend;kgaesigsqkendl
;gaesigsqkendln;aesigsqkendlnl;esigsqkendlnle;sigsqkendlnled;igsqkendln
leds;gsqkendlnledss;sqkendlnledssk;qkendlnledsskk;kendlnledsskks;endlnl
edsskksh;ndlnledsskkshq;dlnledsskkshqn;lnledsskkshqna;nledsskkshqnak;le
dsskkshqnakq;edsskkshqnakqd;dsskkshqnakqdl;sskkshqnakqdlp;skkshqnakqdlp
a;kkshqnakqdlpav;kshqnakqdlpavt;shqnakqdlpavte;hqnakqdlpavted;qnakqdlpa
vteds;nakqdlpavtedsv;akqdlpavtedsvs;kqdlpavtedsvsl;qdlpavtedsvslf;dlpav
tedsvslfn;lpavtedsvslfng;pavtedsvslfngn;avtedsvslfngnk;vtedsvslfngnki;t
edsvslfngnkif;edsvslfngnkifv;dsvslfngnkifvs;svslfngnkifvsk;vslfngnkifvs
ke;slfngnkifvskek;lfngnkifvskekn;fngnkifvskekns;ngnkifvskeknss;gnkifvsk
eknssg;nkifvskeknssgk;kifvskeknssgky;ifvskeknssgkyd;fvskeknssgkydl;vske
knssgkydlr;skeknssgkydlra;keknssgkydlrat;eknssgkydlrati;knssgkydlratid;
nssgkydlratidq;ssgkydlratidqv;sgkydlratidqve;gkydlratidqvel;kydlratidqv
elk;ydlratidqvelkg;dlratidqvelkgt;lratidqvelkgts;ratidqvelkgtsd;atidqve
lkgtsdk;tidqvelkgtsdkn;idqvelkgtsdknn;dqvelkgtsdknng;qvelkgtsdknngs;vel
kgtsdknngsg;elkgtsdknngsgt;lkgtsdknngsgtl;kgtsdknngsgtle;gtsdknngsgtleg
;tsdknngsgtlegs;sdknngsgtlegsk;dknngsgtlegskp;knngsgtlegskpd;nngsgtlegs
kpdk;ngsgtlegskpdks;gsgtlegskpdksk;sgtlegskpdkskv;gtlegskpdkskvk;tlegsk
pdkskvkl;legskpdkskvklt;egskpdkskvkltv;gskpdkskvkltvs;skpdkskvkltvsa;kp
dkskvkltvsad;pdkskvkltvsadl;dkskvkltvsadln;kskvkltvsadlnt;skvkltvsadlnt
v;kvkltvsadlntvt;vkltvsadlntvtl;kltvsadlntvtle;ltvsadlntvtlea;tvsadlntv
tleaf;vsadlntvtleafd;sadlntvtleafda;adlntvtleafdas;dlntvtleafdasn;lntvt
leafdasnq;ntvtleafdasnqk;tvtleafdasnqki;vtleafdasnqkis;tleafdasnqkiss;l
eafdasnqkissk;eafdasnqkisskv;afdasnqkisskvt;fdasnqkisskvtk;dasnqkisskvt
kk;asnqkisskvtkkq;snqkisskvtkkqg;nqkisskvtkkqgs;qkisskvtkkqgsi;kisskvtk
kqgsit;isskvtkkqgsitx;sskvtkkqgsitxe;skvtkkqgsitxet;kvtkkqgsitxetl;vtkk
qgsitxetlk;tkkqgsitxetlka;kkqgsitxetlkan;kqgsitxetlkank;qgsitxetlkankl;
gsitxetlkankld;sitxetlkanklds;itxetlkankldsk;txetlkankldskk;xetlkanklds
kkl;etlkankldskklt;tlkankldskkltr;lkankldskkltrs;kankldskkltrsn;ankldsk
kltrsng;nkldskkltrsngt;kldskkltrsngtt;ldskkltrsngttl;dskkltrsngttle;skk
ltrsngttley;kkltrsngttleys;kltrsngttleysq;ltrsngttleysqi;trsngttleysqit
;rsngttleysqitd;sngttleysqitda;ngttleysqitdad;gttleysqitdadn;ttleysqitd
adna;tleysqitdadnat;leysqitdadnatk;eysqitdadnatka;ysqitdadnatkav;sqitda
dnatkave;qitdadnatkavet;itdadnatkavetl;tdadnatkavetlk;dadnatkavetlkn;ad

natkavetlkns;dnatkavetlknsi;natkavetlknsik;atkavetlknsikl;tkavetlknsikl
e;kavetlknsikleg;avetlknsiklegs;vetlknsiklegsl;etlknsiklegslv;tlknsikle
gslvg;lknsiklegslvgg;knsiklegslvggk;nsiklegslvggkt;siklegslvggktt;ikleg
slvggkttv;klegslvggkttve;legslvggkttvei;egslvggkttveik;gslvggkttveike;s
lvggkttveikeg;lvggkttveikegt;vggkttveikegtv;ggkttveikegtvt;gkttveikegtv
tl;kttveikegtvtlk;ttveikegtvtlkr;tveikegtvtlkre;veikegtvtlkrei;eikegtvt
lkreie;ikegtvtlkreiek;kegtvtlkreiekd;egtvtlkreiekdg;gtvtlkreiekdgk;tvtl
kreiekdgkv;vtlkreiekdgkvk;tlkreiekdgkvkv;lkreiekdgkvkvf;kreiekdgkvkvfl;
reiekdgkvkvfln;eiekdgkvkvflnd;iekdgkvkvflndt;ekdgkvkvflndta;kdgkvkvflnd
tag;dgkvkvflndtags;gkvkvflndtagsn;kvkvflndtagsnk;vkvflndtagsnkk;kvflndt
agsnkkt;vflndtagsnkktg;flndtagsnkktgk;lndtagsnkktgkw;ndtagsnkktgkwe;dta
gsnkktgkwed;tagsnkktgkweds;agsnkktgkwedst;gsnkktgkwedsts;snkktgkwedstst
;nkktgkwedststl;kktgkwedststlt;ktgkwedststlti;tgkwedststltis;gkwedststl
tisa;kwedststltisad;wedststltisads;edststltisadsk;dststltisadskk;ststlt
isadskkt;tstltisadskktk;stltisadskktkd;tltisadskktkdl;ltisadskktkdlv;ti
sadskktkdlvf;isadskktkdlvfl;sadskktkdlvflt;adskktkdlvfltd;dskktkdlvfltd
g;skktkdlvfltdgt;kktkdlvfltdgti;ktkdlvfltdgtit;tkdlvfltdgtitv;kdlvfltdg
titvq;dlvfltdgtitvqq;lvfltdgtitvqqy;vfltdgtitvqqyn;fltdgtitvqqynt;ltdgt
itvqqynta;tdgtitvqqyntag;dgtitvqqyntagt;gtitvqqyntagts;titvqqyntagtsl;i
tvqqyntagtsle;tvqqyntagtsleg;vqqyntagtslegs;qqyntagtslegsa;qyntagtslegs
as;yntagtslegsase;ntagtslegsasei;tagtslegsaseik;agtslegsaseikn;gtslegsa
seiknl;tslegsaseiknls;slegsaseiknlse;legsaseiknlsel;egsaseiknlselk;gsas
eiknlselkn;saseiknlselkna;aseiknlselknal;seiknlselknalk;

15 mers:
mrlligfalalalig;rlligfalalaligc;lligfalalaligca;ligfalalaligcaq;igfalal
aligcaqk;gfalalaligcaqkg;falalaligcaqkga;alalaligcaqkgae;lalaligcaqkgae
s;alaligcaqkgaesi;laligcaqkgaesig;aligcaqkgaesigs;ligcaqkgaesigsq;igcaq
kgaesigsqk;gcaqkgaesigsqke;caqkgaesigsqken;aqkgaesigsqkend;qkgaesigsqke
ndl;kgaesigsqkendln;gaesigsqkendlnl;aesigsqkendlnle;esigsqkendlnled;sig
sqkendlnleds;igsqkendlnledss;gsqkendlnledssk;sqkendlnledsskk;qkendlnled
sskks;kendlnledsskksh;endlnledsskkshq;ndlnledsskkshqn;dlnledsskkshqna;l
nledsskkshqnak;nledsskkshqnakq;ledsskkshqnakqd;edsskkshqnakqdl;dsskkshq
nakqdlp;sskkshqnakqdlpa;skkshqnakqdlpav;kkshqnakqdlpavt;kshqnakqdlpavte
;shqnakqdlpavted;hqnakqdlpavteds;qnakqdlpavtedsv;nakqdlpavtedsvs;akqdlp
avtedsvsl;kqdlpavtedsvslf;qdlpavtedsvslfn;dlpavtedsvslfng;lpavtedsvslfn
gn;pavtedsvslfngnk;avtedsvslfngnki;vtedsvslfngnkif;tedsvslfngnkifv;edsv
slfngnkifvs;dsvslfngnkifvsk;svslfngnkifvske;vslfngnkifvskek;slfngnkifvs
kekn;lfngnkifvskekns;fngnkifvskeknss;ngnkifvskeknssg;gnkifvskeknssgk;nk
ifvskeknssgky;kifvskeknssgkyd;ifvskeknssgkydl;fvskeknssgkydlr;vskeknssg
kydlra;skeknssgkydlrat;keknssgkydlrati;eknssgkydlratid;knssgkydlratidq;
nssgkydlratidqv;ssgkydlratidqve;sgkydlratidqvel;gkydlratidqvelk;kydlrat
idqvelkg;ydlratidqvelkgt;dlratidqvelkgts;lratidqvelkgtsd;ratidqvelkgtsd
k;atidqvelkgtsdkn;tidqvelkgtsdknn;idqvelkgtsdknng;dqvelkgtsdknngs;qvelk
gtsdknngsg;velkgtsdknngsgt;elkgtsdknngsgtl;lkgtsdknngsgtle;kgtsdknngsgt
leg;gtsdknngsgtlegs;tsdknngsgtlegsk;sdknngsgtlegskp;dknngsgtlegskpd;knn
gsgtlegskpdk;nngsgtlegskpdks;ngsgtlegskpdksk;gsgtlegskpdkskv;sgtlegskpd
kskvk;gtlegskpdkskvkl;tlegskpdkskvklt;legskpdkskvkltv;egskpdkskvkltvs;g
skpdkskvkltvsa;skpdkskvkltvsad;kpdkskvkltvsadl;pdkskvkltvsadln;dkskvklt
vsadlnt;kskvkltvsadlntv;skvkltvsadlntvt;kvkltvsadlntvtl;vkltvsadlntvtle
;kltvsadlntvtlea;ltvsadlntvtleaf;tvsadlntvtleafd;vsadlntvtleafda;sadlnt
vtleafdas;adlntvtleafdasn;dlntvtleafdasnq;lntvtleafdasnqk;ntvtleafdasnq
ki;tvtleafdasnqkis;vtleafdasnqkiss;tleafdasnqkissk;leafdasnqkisskv;eafd
asnqkisskvt;afdasnqkisskvtk;fdasnqkisskvtkk;dasnqkisskvtkkq;asnqkisskvt
kkqg;snqkisskvtkkqgs;nqkisskvtkkqgsi;qkisskvtkkqgsit;kisskvtkkqgsitx;is
skvtkkqgsitxe;sskvtkkqgsitxet;skvtkkqgsitxetl;kvtkkqgsitxetlk;vtkkqgsit
xetlka;tkkqgsitxetlkan;kkqgsitxetlkank;kqgsitxetlkankl;qgsitxetlkankld;

gsitxetlkanklds;sitxetlkankldsk;itxetlkankldskk;txetlkankldskkl;xetlkan
kldskklt;etlkankldskkltr;tlkankldskkltrs;lkankldskkltrsn;kankldskkltrsn
g;ankldskkltrsngt;nkldskkltrsngtt;kldskkltrsngttl;ldskkltrsngttle;dskkl
trsngttley;skkltrsngttleys;kkltrsngttleysq;kltrsngttleysqi;ltrsngttleys
qit;trsngttleysqitd;rsngttleysqitda;sngttleysqitdad;ngttleysqitdadn;gtt
leysqitdadna;ttleysqitdadnat;tleysqitdadnatk;leysqitdadnatka;eysqitdadn
atkav;ysqitdadnatkave;sqitdadnatkavet;qitdadnatkavetl;itdadnatkavetlk;t
dadnatkavetlkn;dadnatkavetlkns;adnatkavetlknsi;dnatkavetlknsik;natkavet
lknsikl;atkavetlknsikle;tkavetlknsikleg;kavetlknsiklegs;avetlknsiklegsl
;vetlknsiklegslv;etlknsiklegslvg;tlknsiklegslvgg;lknsiklegslvggk;knsikl
egslvggkt;nsiklegslvggktt;siklegslvggkttv;iklegslvggkttve;klegslvggkttv
ei;legslvggkttveik;egslvggkttveike;gslvggkttveikeg;slvggkttveikegt;lvgg
kttveikegtv;vggkttveikegtvt;ggkttveikegtvtl;gkttveikegtvtlk;kttveikegtv
tlkr;ttveikegtvtlkre;tveikegtvtlkrei;veikegtvtlkreie;eikegtvtlkreiek;ik
egtvtlkreiekd;kegtvtlkreiekdg;egtvtlkreiekdgk;gtvtlkreiekdgkv;tvtlkreie
kdgkvk;vtlkreiekdgkvkv;tlkreiekdgkvkvf;lkreiekdgkvkvfl;kreiekdgkvkvfln;
reiekdgkvkvflnd;eiekdgkvkvflndt;iekdgkvkvflndta;ekdgkvkvflndtag;kdgkvkv
flndtags;dgkvkvflndtagsn;gkvkvflndtagsnk;kvkvflndtagsnkk;vkvflndtagsnkk
t;kvflndtagsnkktg;vflndtagsnkktgk;flndtagsnkktgkw;lndtagsnkktgkwe;ndtag
snkktgkwed;dtagsnkktgkweds;tagsnkktgkwedst;agsnkktgkwedsts;gsnkktgkweds
tst;snkktgkwedststl;nkktgkwedststlt;kktgkwedststlti;ktgkwedststltis;tgk
wedststltisa;gkwedststltisad;kwedststltisads;wedststltisadsk;edststltis
adskk;dststltisadskkt;ststltisadskktk;tstltisadskktkd;stltisadskktkdl;t
ltisadskktkdlv;ltisadskktkdlvf;tisadskktkdlvfl;isadskktkdlvflt;sadskktk
dlvfltd;adskktkdlvfltdg;dskktkdlvfltdgt;skktkdlvfltdgti;kktkdlvfltdgtit
;ktkdlvfltdgtitv;tkdlvfltdgtitvq;kdlvfltdgtitvqq;dlvfltdgtitvqqy;lvfltd
gtitvqqyn;vfltdgtitvqqynt;fltdgtitvqqynta;ltdgtitvqqyntag;tdgtitvqqynta
gt;dgtitvqqyntagts;gtitvqqyntagtsl;titvqqyntagtsle;itvqqyntagtsleg;tvqq
yntagtslegs;vqqyntagtslegsa;qqyntagtslegsas;qyntagtslegsase;yntagtslegs
asei;ntagtslegsaseik;tagtslegsaseikn;agtslegsaseiknl;gtslegsaseiknls;ts
legsaseiknlse;slegsaseiknlsel;legsaseiknlselk;egsaseiknlselkn;gsaseiknl
selkna;saseiknlselknal;aseiknlselknalk;

16 mers:
mrlligfalalaligc;rlligfalalaligca;lligfalalaligcaq;ligfalalaligcaqk;igf
alalaligcaqkg;gfalalaligcaqkga;falalaligcaqkgae;alalaligcaqkgaes;lalali
gcaqkgaesi;alaligcaqkgaesig;laligcaqkgaesigs;aligcaqkgaesigsq;ligcaqkga
esigsqk;igcaqkgaesigsqke;gcaqkgaesigsqken;caqkgaesigsqkend;aqkgaesigsqk
endl;qkgaesigsqkendln;kgaesigsqkendlnl;gaesigsqkendlnle;aesigsqkendlnle
d;esigsqkendlnleds;sigsqkendlnledss;igsqkendlnledssk;gsqkendlnledsskk;s
qkendlnledsskks;qkendlnledsskksh;kendlnledsskkshq;endlnledsskkshqn;ndln
ledsskkshqna;dlnledsskkshqnak;lnledsskkshqnakq;nledsskkshqnakqd;ledsskk
shqnakqdl;edsskkshqnakqdlp;dsskkshqnakqdlpa;sskkshqnakqdlpav;skkshqnakq
dlpavt;kkshqnakqdlpavte;kshqnakqdlpavted;shqnakqdlpavteds;hqnakqdlpavte
dsv;qnakqdlpavtedsvs;nakqdlpavtedsvsl;akqdlpavtedsvslf;kqdlpavtedsvslfn
;qdlpavtedsvslfng;dlpavtedsvslfngn;lpavtedsvslfngnk;pavtedsvslfngnki;av
tedsvslfngnkif;vtedsvslfngnkifv;tedsvslfngnkifvs;edsvslfngnkifvsk;dsvsl
fngnkifvske;svslfngnkifvskek;vslfngnkifvskekn;slfngnkifvskekns;lfngnkif
vskeknss;fngnkifvskeknssg;ngnkifvskeknssgk;gnkifvskeknssgky;nkifvskekns
sgkyd;kifvskeknssgkydl;ifvskeknssgkydlr;fvskeknssgkydlra;vskeknssgkydlr
at;skeknssgkydlrati;keknssgkydlratid;eknssgkydlratidq;knssgkydlratidqv;
nssgkydlratidqve;ssgkydlratidqvel;sgkydlratidqvelk;gkydlratidqvelkg;kyd
lratidqvelkgt;ydlratidqvelkgts;dlratidqvelkgtsd;lratidqvelkgtsdk;ratidq
velkgtsdkn;atidqvelkgtsdknn;tidqvelkgtsdknng;idqvelkgtsdknngs;dqvelkgts
dknngsg;qvelkgtsdknngsgt;velkgtsdknngsgtl;elkgtsdknngsgtle;lkgtsdknngsg
tleg;kgtsdknngsgtlegs;gtsdknngsgtlegsk;tsdknngsgtlegskp;sdknngsgtlegskp
d;dknngsgtlegskpdk;knngsgtlegskpdks;nngsgtlegskpdksk;ngsgtlegskpdkskv;g

sgtlegskpdkskvk;sgtlegskpdkskvkl;gtlegskpdkskvklt;tlegskpdkskvkltv;legs
kpdkskvkltvs;egskpdkskvkltvsa;gskpdkskvkltvsad;skpdkskvkltvsadl;kpdkskv
kltvsadln;pdkskvkltvsadlnt;dkskvkltvsadlntv;kskvkltvsadlntvt;skvkltvsad
lntvtl;kvkltvsadlntvtle;vkltvsadlntvtlea;kltvsadlntvtleaf;ltvsadlntvtle
afd;tvsadlntvtleafda;vsadlntvtleafdas;sadlntvtleafdasn;adlntvtleafdasnq
;dlntvtleafdasnqk;lntvtleafdasnqki;ntvtleafdasnqkis;tvtleafdasnqkiss;vt
leafdasnqkissk;tleafdasnqkisskv;leafdasnqkisskvt;eafdasnqkisskvtk;afdas
nqkisskvtkk;fdasnqkisskvtkkq;dasnqkisskvtkkqg;asnqkisskvtkkqgs;snqkissk
vtkkqgsi;nqkisskvtkkqgsit;qkisskvtkkqgsitx;kisskvtkkqgsitxe;isskvtkkqgs
itxet;sskvtkkqgsitxetl;skvtkkqgsitxetlk;kvtkkqgsitxetlka;vtkkqgsitxetlk
an;tkkqgsitxetlkank;kkqgsitxetlkankl;kqgsitxetlkankld;qgsitxetlkanklds;
gsitxetlkankldsk;sitxetlkankldskk;itxetlkankldskkl;txetlkankldskklt;xet
lkankldskkltr;etlkankldskkltrs;tlkankldskkltrsn;lkankldskkltrsng;kankld
skkltrsngt;ankldskkltrsngtt;nkldskkltrsngttl;kldskkltrsngttle;ldskkltrs
ngttley;dskkltrsngttleys;skkltrsngttleysq;kkltrsngttleysqi;kltrsngttley
sqit;ltrsngttleysqitd;trsngttleysqitda;rsngttleysqitdad;sngttleysqitdad
n;ngttleysqitdadna;gttleysqitdadnat;ttleysqitdadnatk;tleysqitdadnatka;l
eysqitdadnatkav;eysqitdadnatkave;ysqitdadnatkavet;sqitdadnatkavetl;qitd
adnatkavetlk;itdadnatkavetlkn;tdadnatkavetlkns;dadnatkavetlknsi;adnatka
vetlknsik;dnatkavetlknsikl;natkavetlknsikle;atkavetlknsikleg;tkavetlkns
iklegs;kavetlknsiklegsl;avetlknsiklegslv;vetlknsiklegslvg;etlknsiklegsl
vgg;tlknsiklegslvggk;lknsiklegslvggkt;knsiklegslvggktt;nsiklegslvggkttv
;siklegslvggkttve;iklegslvggkttvei;klegslvggkttveik;legslvggkttveike;eg
slvggkttveikeg;gslvggkttveikegt;slvggkttveikegtv;lvggkttveikegtvt;vggkt
tveikegtvtl;ggkttveikegtvtlk;gkttveikegtvtlkr;kttveikegtvtlkre;ttveikeg
tvtlkrei;tveikegtvtlkreie;veikegtvtlkreiek;eikegtvtlkreiekd;ikegtvtlkre
iekdg;kegtvtlkreiekdgk;egtvtlkreiekdgkv;gtvtlkreiekdgkvk;tvtlkreiekdgkv
kv;vtlkreiekdgkvkvf;tlkreiekdgkvkvfl;lkreiekdgkvkvfln;kreiekdgkvkvflnd;
reiekdgkvkvflndt;eiekdgkvkvflndta;iekdgkvkvflndtag;ekdgkvkvflndtags;kdg
kvkvflndtagsn;dgkvkvflndtagsnk;gkvkvflndtagsnkk;kvkvflndtagsnkkt;vkvfln
dtagsnkktg;kvflndtagsnkktgk;vflndtagsnkktgkw;flndtagsnkktgkwe;lndtagsnk
ktgkwed;ndtagsnkktgkweds;dtagsnkktgkwedst;tagsnkktgkwedsts;agsnkktgkwed
stst;gsnkktgkwedststl;snkktgkwedststlt;nkktgkwedststlti;kktgkwedststlti
s;ktgkwedststltisa;tgkwedststltisad;gkwedststltisads;kwedststltisadsk;w
edststltisadskk;edststltisadskkt;dststltisadskktk;ststltisadskktkd;tstl
tisadskktkdl;stltisadskktkdlv;tltisadskktkdlvf;ltisadskktkdlvfl;tisadsk
ktkdlvflt;isadskktkdlvfltd;sadskktkdlvfltdg;adskktkdlvfltdgt;dskktkdlvf
ltdgti;skktkdlvfltdgtit;kktkdlvfltdgtitv;ktkdlvfltdgtitvq;tkdlvfltdgtit
vqq;kdlvfltdgtitvqqy;dlvfltdgtitvqqyn;lvfltdgtitvqqynt;vfltdgtitvqqynta
;fltdgtitvqqyntag;ltdgtitvqqyntagt;tdgtitvqqyntagts;dgtitvqqyntagtsl;gt
itvqqyntagtsle;titvqqyntagtsleg;itvqqyntagtslegs;tvqqyntagtslegsa;vqqyn
tagtslegsas;qqyntagtslegsase;qyntagtslegsasei;yntagtslegsaseik;ntagtsle
gsaseikn;tagtslegsaseiknl;agtslegsaseiknls;gtslegsaseiknlse;tslegsaseik
nlsel;slegsaseiknlselk;legsaseiknlselkn;egsaseiknlselkna;gsaseiknlselkn
al;saseiknlselknalk;

## \<AAN87995 Outer Surface Protein C;Protein;Borrelia garinii\>

8 mers:
ilmtlflf;lmtlflfi;mtlflfis;tlflfisc;lflfiscn;flfiscnn;lfiscnns;fiscnnsg
;iscnnsgg;scnnsggd;cnnsggdt;nnsggdta;nsggdtas;sggdtast;ggdtastn;gdtastn
p;dtastnpd;tastnpde;astnpdes;stnpdesa;tnpdesak;npdesakg;pdesakgp;desakg
pn;esakgpnl;sakgpnlt;akgpnltv;kgpnltvi;gpnltvis;pnltvisk;nltviskk;ltvis
kki;tviskkit;viskkitd;iskkitds;skkitdsn;kkitdsna;kitdsnaf;itdsnafv;tdsn
afvl;dsnafvla;snafvlav;nafvlavk;afvlavke;fvlavkev;vlavkeve;lavkevea;avk
eveal;vkeveali;kevealis;evealiss;vealissi;ealissid;alisside;lissidel;is

sidela;ssidelan;sidelank;idelanka;delankai;elankaig;lankaigk;ankaigkv;n
kaigkvi;kaigkvih;aigkvihq;igkvihqn;gkvihqnn;kvihqnng;vihqnngl;ihqnngln;
hqnnglna;qnnglnan;nnglnana;nglnanag;glnanagq;lnanagqn;nanagqng;anagqngs
;nagqngsl;agqngsll;gqngslla;qngsllag;ngsllaga;gsllagay;sllagaya;llagaya
i;lagayais;agayaist;gayaistl;ayaistli;yaistlit;aistlite;istlitek;stlite
kl;tlitekls;liteklsk;iteklskl;teklsklk;eklsklkn;klsklkns;lsklknse;sklkn
see;klknseel;lknseeln;knseelnk;nseelnkk;seelnkki;eelnkkie;elnkkiee;lnkk
ieea;nkkieeak;kkieeakn;kieeaknh;ieeaknhs;eeaknhse;eaknhsea;aknhseaf;knh
seaft;nhseaftn;hseaftnr;seaftnrl;eaftnrlk;aftnrlkg;ftnrlkgs;tnrlkgsh;nr
lkgsha;rlkgshaq;lkgshaql;kgshaqlg;gshaqlgv;shaqlgva;haqlgvaa;aqlgvaaa;q
lgvaaat;lgvaaatd;gvaaatdd;vaaatddh;aaatddha;aatddhak;atddhake;tddhakea;
ddhakeai;dhakeail;hakeailk;akeailks;keailksn;eailksnp;ailksnpt;ilksnptk
;lksnptkd;ksnptkdk;snptkdkg;nptkdkga;ptkdkgak;tkdkgake;kdkgakel;dkgakel
k;kgakelkd;gakelkdl;akelkdls;kelkdlse;elkdlses;lkdlsesv;kdlsesve;dlsesv
es;lsesvesl;sesvesla;esveslak;sveslaka;veslakaa;eslakaaq;slakaaqe;lakaa
qea;akaaqeal;kaaqeala;aaqealan;aqealans;qealansv;ealansvk;alansvke;lans
vkel;ansvkelt;nsvkeltn;svkeltnp;vkeltnpv;keltnpvv;eltnpvva;ltnpvvae;tnp
vvaes;npvvaesp;pvvaespk;

9 mers:
ilmtlflfi;lmtlflfis;mtlflfisc;tlflfiscn;lflfiscnn;flfiscnns;lfiscnnsg;f
iscnnsgg;iscnnsggd;scnnsggdt;cnnsggdta;nnsggdtas;nsggdtast;sggdtastn;gg
dtastnp;gdtastnpd;dtastnpde;tastnpdes;astnpdesa;stnpdesak;tnpdesakg;npd
esakgp;pdesakgpn;desakgpnl;esakgpnlt;sakgpnltv;akgpnltvi;kgpnltvis;gpnl
tvisk;pnltviskk;nltviskki;ltviskkit;tviskkitd;viskkitds;iskkitdsn;skkit
dsna;kkitdsnaf;kitdsnafv;itdsnafvl;tdsnafvla;dsnafvlav;snafvlavk;nafvla
vke;afvlavkev;fvlavkeve;vlavkevea;lavkeveal;avkeveali;vkevealis;keveali
ss;evealissi;vealissid;ealisside;alissidel;lissidela;issidelan;ssidelan
k;sidelanka;idelankai;delankaig;elankaigk;lankaigkv;ankaigkvi;nkaigkvih
;kaigkvihq;aigkvihqn;igkvihqnn;gkvihqnng;kvihqnngl;vihqnngln;ihqnnglna;
hqnnglnan;qnnglnana;nnglnanag;nglnanagq;glnanagqn;lnanagqng;nanagqngs;a
nagqngsl;nagqngsll;agqngslla;gqngsllag;qngsllaga;ngsllagay;gsllagaya;sl
lagayai;llagayais;lagayaist;agayaistl;gayaistli;ayaistlit;yaistlite;ais
tlitek;istlitekl;stlitekls;tliteklsk;liteklskl;iteklsklk;teklsklkn;ekls
klkns;klsklknse;lsklknsee;sklknseel;klknseeln;lknseelnk;knseelnkk;nseel
nkki;seelnkkie;eelnkkiee;elnkkieea;lnkkieeak;nkkieeakn;kkieeaknh;kieeak
nhs;ieeaknhse;eeaknhsea;eaknhseaf;aknhseaft;knhseaftn;nhseaftnr;hseaftn
rl;seaftnrlk;eaftnrlkg;aftnrlkgs;ftnrlkgsh;tnrlkgsha;nrlkgshaq;rlkgshaq
l;lkgshaqlg;kgshaqlgv;gshaqlgva;shaqlgvaa;haqlgvaaa;aqlgvaaat;qlgvaaatd
;lgvaaatdd;gvaaatddh;vaaatddha;aaatddhak;aatddhake;atddhakea;tddhakeai;
ddhakeail;dhakeailk;hakeailks;akeailksn;keailksnp;eailksnpt;ailksnptk;i
lksnptkd;lksnptkdk;ksnptkdkg;snptkdkga;nptkdkgak;ptkdkgake;tkdkgakel;kd
kgakelk;dkgakelkd;kgakelkdl;gakelkdls;akelkdlse;kelkdlses;elkdlsesv;lkd
lsesve;kdlsesves;dlsesvesl;lsesvesla;sesveslak;esveslaka;sveslakaa;vesl
akaaq;eslakaaqe;slakaaqea;lakaaqeal;akaaqeala;kaaqealan;aaqealans;aqeal
ansv;qealansvk;ealansvke;alansvkel;lansvkelt;ansvkeltn;nsvkeltnp;svkelt
npv;vkeltnpvv;keltnpvva;eltnpvvae;ltnpvvaes;tnpvvaesp;npvvaespk;

10 mers:
ilmtlflfis;lmtlflfisc;mtlflfiscn;tlflfiscnn;lflfiscnns;flfiscnnsg;lfisc
nnsgg;fiscnnsggd;iscnnsggdt;scnnsggdta;cnnsggdtas;nnsggdtast;nsggdtastn
;sggdtastnp;ggdtastnpd;gdtastnpde;dtastnpdes;tastnpdesa;astnpdesak;stnp
desakg;tnpdesakgp;npdesakgpn;pdesakgpnl;desakgpnlt;esakgpnltv;sakgpnltv
i;akgpnltvis;kgpnltvisk;gpnltviskk;pnltviskki;nltviskkit;ltviskkitd;tvi
skkitds;viskkitdsn;iskkitdsna;skkitdsnaf;kkitdsnafv;kitdsnafvl;itdsnafv
la;tdsnafvlav;dsnafvlavk;snafvlavke;nafvlavkev;afvlavkeve;fvlavkevea;vl
avkeveal;lavkeveali;avkevealis;vkevealiss;kevealissi;evealissid;vealiss

ide;ealissidel;alissidela;lissidelan;issidelank;ssidelanka;sidelankai;i
delankaig;delankaigk;elankaigkv;lankaigkvi;ankaigkvih;nkaigkvihq;kaigkv
ihqn;aigkvihqnn;igkvihqnng;gkvihqnngl;kvihqnngln;vihqnnglna;ihqnnglnan;
hqnnglnana;qnnglnanag;nnglnanagq;nglnanagqn;glnanagqng;lnanagqngs;nanag
qngsl;anagqngsll;nagqngslla;agqngsllag;gqngsllaga;qngsllagay;ngsllagaya
;gsllagayai;sllagayais;llagayaist;lagayaistl;agayaistli;gayaistlit;ayai
stlite;yaistlitek;aistlitekl;istliteklss;stliteklsk;tliteklskl;liteklskl
k;iteklsklkn;teklsklkns;eklsklknse;klsklknsee;lsklknseel;sklknseeln;klk
nseelnk;lknseelnkk;knseelnkki;nseelnkkie;seelnkkiee;eelnkkieea;elnkkiee
ak;lnkkieeakn;nkkieeaknh;kkieeaknhs;kieeaknhse;ieeaknhsea;eeaknhseaf;ea
knhseaft;aknhseaftn;knhseaftnr;nhseaftnrl;hseaftnrlk;seaftnrlkg;eaftnrl
kgs;aftnrlkgsh;ftnrlkgsha;tnrlkgshaq;nrlkgshaql;rlkgshaqlg;lkgshaqlgv;k
gshaqlgva;gshaqlgvaa;shaqlgvaaa;haqlgvaaat;aqlgvaaatd;qlgvaaatdd;lgvaaa
tddh;gvaaatddha;vaaatddhak;aaatddhake;aatddhakea;atddhakeai;tddhakeail;
ddhakeailk;dhakeailks;hakeailksn;akeailksnp;keailksnpt;eailksnptk;ailks
nptkd;ilksnptkdk;lksnptkdkg;ksnptkdkga;snptkdkgak;nptkdkgake;ptkdkgakel
;tkdkgakelk;kdkgakelkd;dkgakelkdl;kgakelkdls;gakelkdlse;akelkdlses;kelk
dlsesv;elkdlsesve;lkdlsesves;kdlsesvesl;dlsesvesla;lsesveslak;sesveslak
a;esveslakaa;sveslakaaq;veslakaaqe;eslakaaqea;slakaaqeal;lakaaqeala;aka
aqealan;kaaqealans;aaqealansv;aqealansvk;qealansvke;ealansvkel;alansvke
lt;lansvkeltn;ansvkeltnp;nsvkeltnpv;svkeltnpvv;vkeltnpvva;keltnpvvae;el
tnpvvaes;ltnpvvaesp;tnpvvaespk;

11 mers:
ilmtlflfisc;lmtlflfiscn;mtlflfiscnn;tlflfiscnns;lflfiscnnsg;flfiscnnsgg
;lfiscnnsggd;fiscnnsggdt;iscnnsggdta;scnnsggdtas;cnnsggdtast;nnsggdtast
n;nsggdtastnp;sggdtastnpd;ggdtastnpde;gdtastnpdes;dtastnpdesa;tastnpdes
ak;astnpdesakg;stnpdesakgp;tnpdesakgpn;npdesakgpnl;pdesakgpnlt;desakgpn
ltv;esakgpnltvi;sakgpnltvis;akgpnltvisk;kgpnltviskk;gpnltviskki;pnltvis
kkit;nltviskkitd;ltviskkitds;tviskkitdsn;viskkitdsna;iskkitdsnaf;skkitd
snafv;kkitdsnafvl;kitdsnafvla;itdsnafvlav;tdsnafvlavk;dsnafvlavke;snafv
lavkev;nafvlavkeve;afvlavkevea;fvlavkeveal;vlavkeveali;lavkevealis;avke
vealiss;vkevealissi;kevealissid;evealisside;vealissidel;ealissidela;ali
ssidelan;lissidelank;issidelanka;ssidelankai;sidelankaig;idelankaigk;de
lankaigkv;elankaigkvi;lankaigkvih;ankaigkvihq;nkaigkvihqn;kaigkvihqnn;a
igkvihqnng;igkvihqnngl;gkvihqnngln;kvihqnnglna;vihqnnglnan;ihqnnglnana;
hqnnglnanag;qnnglnanagq;nnglnanagqn;nglnanagqng;glnanagqngs;lnanagqngsl
;nanagqngsll;anagqngslla;nagqngsllag;agqngsllaga;gqngsllagay;qngsllagay
a;ngsllagayai;gsllagayais;sllagayaist;llagayaistl;lagayaistli;agayaistl
it;gayaistlite;ayaistlitek;yaistlitekl;aistliteklss;istliteklsk;stlitekl
skl;tliteklsklk;liteklsklkn;iteklsklkns;teklsklknse;eklsklknsee;klsklkn
seel;lsklknseeln;sklknseelnk;klknseelnkk;lknseelnkki;knseelnkkie;nseeln
kkiee;seelnkkieea;eelnkkieeak;elnkkieeakn;lnkkieeaknh;nkkieeaknhs;kkiee
aknhse;kieeaknhsea;ieeaknhseaf;eeaknhseaft;eaknhseaftn;aknhseaftnr;knhs
eaftnrl;nhseaftnrlk;hseaftnrlkg;seaftnrlkgs;eaftnrlkgsh;aftnrlkgsha;ftn
rlkgshaq;tnrlkgshaql;nrlkgshaqlg;rlkgshaqlgv;lkgshaqlgva;kgshaqlgvaa;gs
haqlgvaaa;shaqlgvaaat;haqlgvaaatd;aqlgvaaatdd;qlgvaaatddh;lgvaaatddha;g
vaaatddhak;vaaatddhake;aaatddhakea;aatddhakeai;atddhakeail;tddhakeailk;
ddhakeailks;dhakeailksn;hakeailksnp;akeailksnpt;keailksnptk;eailksnptkd
;ailksnptkdk;ilksnptkdkg;lksnptkdkga;ksnptkdkgak;snptkdkgake;nptkdkgake
l;ptkdkgakelk;tkdkgakelkd;kdkgakelkdl;dkgakelkdls;kgakelkdlse;gakelkdls
es;akelkdlsesv;kelkdlsesve;elkdlsesves;lkdlsesvesl;kdlsesvesla;dlsesves
lak;lsesveslaka;sesveslakaa;esveslakaaq;sveslakaaqe;veslakaaqea;eslakaa
qeal;slakaaqeala;lakaaqealan;akaaqealans;kaaqealansv;aaqealansvk;aqeala
nsvke;qealansvkel;ealansvkelt;alansvkeltn;lansvkeltnp;ansvkeltnpv;nsvke
ltnpvv;svkeltnpvva;vkeltnpvvae;keltnpvvaes;eltnpvvaesp;ltnpvvaespk;

EP 2 254 592 B1

13 mers:
ilmtlflfiscnn;lmtlflfiscnns;mtlflfiscnnsg;tlflfiscnnsgg;lflfiscnnsggd;f
lfiscnnsggdt;lfiscnnsggdta;fiscnnsggdtas;iscnnsggdtast;scnnsggdtastn;cn
nsggdtastnp;nnsggdtastnpd;nsggdtastnpde;sggdtastnpdes;ggdtastnpdesa;gdt
astnpdesak;dtastnpdesakg;tastnpdesakgp;astnpdesakgpn;stnpdesakgpnl;tnpd
esakgpnlt;npdesakgpnltv;pdesakgpnltvi;desakgpnltvis;esakgpnltvisk;sakgp
nltviskk;akgpnltviskki;kgpnltviskkit;gpnltviskkitd;pnltviskkitds;nltvis
kkitdsn;ltviskkitdsna;tviskkitdsnaf;viskkitdsnafv;iskkitdsnafvl;skkitds
nafvla;kkitdsnafvlav;kitdsnafvlavk;itdsnafvlavke;tdsnafvlavkev;dsnafvla
vkeve;snafvlavkevea;nafvlavkeveal;afvlavkeveali;fvlavkevealis;vlavkevea
liss;lavkevealissi;avkevealissid;vkevealisside;kevealissidel;evealissid
ela;vealissidelan;ealissidelank;alissidelanka;lissidelankai;issidelanka
ig;ssidelankaigk;sidelankaigkv;idelankaigkvi;delankaigkvih;elankaigkvih
q;lankaigkvihqn;ankaigkvihqnn;nkaigkvihqnng;kaigkvihqnngl;aigkvihqnngln
;igkvihqnnglna;gkvihqnnglnan;kvihqnnglnana;vihqnnglnanag;ihqnnglnanagq;
hqnnglnanagqn;qnnglnanagqng;nnglnanagqngs;nglnanagqngsl;glnanagqngsll;l
nanagqngslla;nanagqngsllag;anagqngsllaga;nagqngsllagay;agqngsllagaya;gq
ngsllagayai;qngsllagayais;ngsllagayaist;gsllagayaistl;sllagayaistli;lla
gayaistlit;lagayaistlite;agayaistlitek;gayaistlitekl;ayaistliteklsl;yais
tliteklsk;aistliteklskl;istliteklsklk;stliteklsklkn;tliteklsklkns;litek
lsklknse;iteklsklknsee;teklsklknseel;eklsklknseeln;klsklknseelnk;lsklkn
seelnkk;sklknseelnkki;klknseelnkkie;lknseelnkkiee;knseelnkkieea;nseelnk
kieeak;seelnkkieeakn;eelnkkieeaknh;elnkkieeaknhs;lnkkieeaknhse;nkkieeak
nhsea;kkieeaknhseaf;kieeaknhseaft;ieeaknhseaftn;eeaknhseaftnr;eaknhseaf
tnrl;aknhseaftnrlk;knhseaftnrlkg;nhseaftnrlkgs;hseaftnrlkgsh;seaftnrlkg
sha;eaftnrlkgshaq;aftnrlkgshaql;ftnrlkgshaqlg;tnrlkgshaqlgv;nrlkgshaqlg
va;rlkgshaqlgvaa;lkgshaqlgvaaa;kgshaqlgvaaat;gshaqlgvaaatd;shaqlgvaaatd
d;haqlgvaaatddh;aqlgvaaatddha;qlgvaaatddhak;lgvaaatddhake;gvaaatddhakea
;vaaatddhakeai;aaatddhakeail;aatddhakeailk;atddhakeailks;tddhakeailksn;
ddhakeailksnp;dhakeailksnpt;hakeailksnptk;akeailksnptkd;keailksnptkdk;e
ailksnptkdkg;ailksnptkdkga;ilksnptkdkgak;lksnptkdkgake;ksnptkdkgakel;sn
ptkdkgakelk;nptkdkgakelkd;ptkdkgakelkdl;tkdkgakelkdls;kdkgakelkdlse;dkg
akelkdlses;kgakelkdlsesv;gakelkdlsesve;akelkdlsesves;kelkdlsesvesl;elkd
lsesvesla;lkdlsesveslak;kdlsesveslaka;dlsesveslakaa;lsesveslakaaq;sesve
slakaaqe;esveslakaaqea;sveslakaaqeal;veslakaaqeala;eslakaaqealan;slakaa
qealans;lakaaqealansv;akaaqealansvk;kaaqealansvke;aaqealansvkel;aqealan
svkelt;qealansvkeltn;ealansvkeltnp;alansvkeltnpv;lansvkeltnpvv;ansvkelt
npvva;nsvkeltnpvvae;svkeltnpvvaes;vkeltnpvvaesp;keltnpvvaespk;

14 mers:
ilmtlflfiscnns;lmtlflfiscnnsg;mtlflfiscnnsgg;tlflfiscnnsggd;lflfiscnnsg
gdt;flfiscnnsggdta;lfiscnnsggdtas;fiscnnsggdtast;iscnnsggdtastn;scnnsgg
dtastnp;cnnsggdtastnpd;nnsggdtastnpde;nsggdtastnpdes;sggdtastnpdesa;ggd
tastnpdesak;gdtastnpdesakg;dtastnpdesakgp;tastnpdesakgpn;astnpdesakgpnl
;stnpdesakgpnlt;tnpdesakgpnltv;npdesakgpnltvi;pdesakgpnltvis;desakgpnlt
visk;esakgpnltviskk;sakgpnltviskki;akgpnltviskkit;kgpnltviskkitd;gpnltv
iskkitds;pnltviskkitdsn;nltviskkitdsna;ltviskkitdsnaf;tviskkitdsnafv;vi
skkitdsnafvl;iskkitdsnafvla;skkitdsnafvlav;kkitdsnafvlavk;kitdsnafvlavk
e;itdsnafvlavkev;tdsnafvlavkeve;dsnafvlavkevea;snafvlavkeveal;nafvlavke
veali;afvlavkevealis;fvlavkevealiss;vlavkevealissi;lavkevealissid;avkev
ealisside;vkevealissidel;kevealissidela;evealissidelan;vealissidelank;e
alissidelanka;alissidelankai;lissidelankaig;issidelankaigk;ssidelankaig
kv;sidelankaigkvi;idelankaigkvih;delankaigkvihq;elankaigkvihqn;lankaigk
vihqnn;ankaigkvihqnng;nkaigkvihqnngl;kaigkvihqnngln;aigkvihqnnglna;igkv
ihqnnglnan;gkvihqnnglnana;kvihqnnglnanag;vihqnnglnanagq;ihqnnglnanagqn;
hqnnglnanagqng;qnnglnanagqngs;nnglnanagqngsl;nglnanagqngsll;glnanagqngs
lla;lnanagqngsllag;nanagqngsllaga;anagqngsllagay;nagqngsllagaya;agqngsl

lagayai;gqngsllagayais;qngsllagayaist;ngsllagayaistl;gsllagayaistli;sll
agayaistlit;llagayaistlite;lagayaistlitek;agayaistlitekl;gayaistlitekls
;ayaistliteklsk;yaistliteklskl;aistliteklsklk;istliteklsklkn;stliteklsk
lkns;tliteklsklknse;liteklsklknsee;iteklsklknseel;teklsklknseeln;eklskl
knseelnk;klsklknseelnkk;lsklknseelnkkie;sklknseelnkkie;klknseelnkkiee;lk
nseelnkkieea;knseelnkkieeak;nseelnkkieeakn;seelnkkieeaknh;eelnkkieeaknh
s;elnkkieeaknhse;lnkkieeaknhsea;nkkieeaknhseaf;kkieeaknhseaft;kieeaknhs
eaftn;ieeaknhseaftnr;eeaknhseaftnrl;eaknhseaftnrlk;aknhseaftnrlkg;knhse
aftnrlkgs;nhseaftnrlkgsh;hseaftnrlkgsha;seaftnrlkgshaq;eaftnrlkgshaql;a
ftnrlkgshaqlg;ftnrlkgshaqlgv;tnrlkgshaqlgva;nrlkgshaqlgvaa;rlkgshaqlgva
aa;lkgshaqlgvaaat;kgshaqlgvaaatd;gshaqlgvaaatdd;shaqlgvaaatddh;haqlgvaa
atddha;aqlgvaaatddhak;qlgvaaatddhake;lgvaaatddhakea;gvaaatddhakeai;vaaa
tddhakeail;aaatddhakeailk;aatddhakeailks;atddhakeailksn;tddhakeailksnp;
ddhakeailksnpt;dhakeailksnptk;hakeailksnptkd;akeailksnptkdk;keailksnptk
dkg;eailksnptkdkga;ailksnptkdkgak;ilksnptkdkgake;lksnptkdkgakel;ksnptkd
kgakelk;snptkdkgakelkd;nptkdkgakelkdl;ptkdkgakelkdls;tkdkgakelkdlse;kdk
gakelkdlses;dkgakelkdlsesv;kgakelkdlsesve;gakelkdlsesves;akelkdlsesvesl
;kelkdlsesvesla;elkdlsesveslak;lkdlsesveslaka;kdlsesveslakaa;dlsesvesla
kaaq;lsesveslakaaqe;sesveslakaaqea;esveslakaaqeal;sveslakaaqeala;veslak
aaqealan;eslakaaqealans;slakaaqealansv;lakaaqealansvk;akaaqealansvke;ka
aqealansvkel;aaqealansvkelt;aqealansvkeltn;qealansvkeltnp;ealansvkeltnp
v;alansvkeltnpvv;lansvkeltnpvva;ansvkeltnpvvae;nsvkeltnpvvaes;svkeltnpv
vaesp;vkeltnpvvaespk;

15 mers:
ilmtlflfiscnnsg;lmtlflfiscnnsgg;mtlflfiscnnsggd;tlflfiscnnsggdt;lflfisc
nnsggdta;flfiscnnsggdtas;lfiscnnsggdtast;fiscnnsggdtastn;iscnnsggdtastn
p;scnnsggdtastnpd;cnnsggdtastnpde;nnsggdtastnpdes;nsggdtastnpdesa;sggdt
astnpdesak;ggdtastnpdesakg;gdtastnpdesakgp;dtastnpdesakgpn;tastnpdesakg
pnl;astnpdesakgpnlt;stnpdesakgpnltv;tnpdesakgpnltvi;npdesakgpnltvis;pde
sakgpnltvisk;desakgpnltviskk;esakgpnltviskki;sakgpnltviskkit;akgpnltvis
kkitd;kgpnltviskkitds;gpnltviskkitdsn;pnltviskkitdsna;nltviskkitdsnaf;l
tviskkitdsnafv;tviskkitdsnafvl;viskkitdsnafvla;iskkitdsnafvlav;skkitdsn
afvlavk;kkitdsnafvlavke;kitdsnafvlavkev;itdsnafvlavkeve;tdsnafvlavkevea
;dsnafvlavkeveal;snafvlavkeveali;nafvlavkevealis;afvlavkevealiss;fvlavk
evealissi;vlavkevealissid;lavkevealisside;avkevealissidel;vkevealisside
la;kevealissidelan;evealissidelank;vealissidelanka;ealissidelankai;alis
sidelankaig;lissidelankaigk;issidelankaigkv;ssidelankaigkvi;sidelankaig
kvih;idelankaigkvihq;delankaigkvihqn;elankaigkvihqnn;lankaigkvihqnng;an
kaigkvihqnngl;nkaigkvihqnngln;kaigkvihqnnglna;aigkvihqnnglnan;igkvihqnn
glnana;gkvihqnnglnanag;kvihqnnglnanagq;vihqnnglnanagqn;ihqnnglnanagqng;
hqnnglnanagqngs;qnnglnanagqngsl;nnglnanagqngsll;nglnanagqngslla;glnanag
qngsllag;lnanagqngsllaga;nanagqngsllagay;anagqngsllagaya;nagqngsllagaya
i;agqngsllagayais;gqngsllagayaist;qngsllagayaistl;ngsllagayaistli;gslla
gayaistlit;sllagayaistlite;llagayaistlitek;lagayaistlitekl;agayaistlite
kls;gayaistliteklsk;ayaistliteklskl;yaistliteklsklk;aistliteklsklkn;ist
liteklsklkns;stliteklsklknse;tliteklsklknsee;liteklsklknseel;iteklsklkn
seeln;teklsklknseelnk;eklsklknseelnkk;klsklknseelnkki;lsklknseelnkkie;s
klknseelnkkiee;klknseelnkkieea;lknseelnkkieeak;knseelnkkieeakn;nseelnkk
ieeaknh;seelnkkieeaknhs;eelnkkieeaknhse;elnkkieeaknhsea;lnkkieeaknhseaf
;nkkieeaknhseaft;kkieeaknhseaftn;kieeaknhseaftnr;ieeaknhseaftnrl;eeaknh
seaftnrlk;eaknhseaftnrlkg;aknhseaftnrlkgs;knhseaftnrlkgsh;nhseaftnrlkgs
ha;hseaftnrlkgshaq;seaftnrlkgshaql;eaftnrlkgshaqlg;aftnrlkgshaqlgv;ftnr
lkgshaqlgva;tnrlkgshaqlgvaa;nrlkgshaqlgvaaa;rlkgshaqlgvaaat;lkgshaqlgva
aatd;kgshaqlgvaaatdd;gshaqlgvaaatddh;shaqlgvaaatddha;haqlgvaaatddhak;aq
lgvaaatddhake;qlgvaaatddhakea;lgvaaatddhakeai;gvaaatddhakeail;vaaatddha
keailk;aaatddhakeailks;aatddhakeailksn;atddhakeailksnp;tddhakeailksnpt;

ddhakeailksnptk;dhakeailksnptkd;hakeailksnptkdk;akeailksnptkdkg;keailks
nptkdkga;eailksnptkdkgak;ailksnptkdkgake;ilksnptkdkgakel;lksnptkdkgakel
k;ksnptkdkgakelkd;snptkdkgakelkdl;nptkdkgakelkdls;ptkdkgakelkdlse;tkdkg
akelkdlses;kdkgakelkdlsesv;dkgakelkdlsesve;kgakelkdlsesves;gakelkdlsesv
esl;akelkdlsesvesla;kelkdlsesveslak;elkdlsesveslaka;lkdlsesveslakaa;kdl
sesveslakaaq;dlsesveslakaaqe;lsesveslakaaqea;sesveslakaaqeal;esveslakaa
qeala;sveslakaaqealan;veslakaaqealans;eslakaaqealansv;slakaaqealansvk;l
akaaqealansvke;akaaqealansvkel;kaaqealansvkelt;aaqealansvkeltn;aqealans
vkeltnp;qealansvkeltnpv;ealansvkeltnpvv;alansvkeltnpvva;lansvkeltnpvvae
;ansvkeltnpvvaes;nsvkeltnpvvaesp;svkeltnpvvaespk;

16 mers:
ilmtlflfiscnnsgg;lmtlflfiscnnsggd;mtlflfiscnnsggdt;tlflfiscnnsggdta;lfl
fiscnnsggdtas;flfiscnnsggdtast;lfiscnnsggdtastn;fiscnnsggdtastnp;iscnns
ggdtastnpd;scnnsggdtastnpde;cnnsggdtastnpdes;nnsggdtastnpdesa;nsggdtast
npdesak;sggdtastnpdesakg;ggdtastnpdesakgp;gdtastnpdesakgpn;dtastnpdesak
gpnl;tastnpdesakgpnlt;astnpdesakgpnltv;stnpdesakgpnltvi;tnpdesakgpnltvi
s;npdesakgpnltvisk;pdesakgpnltviskk;desakgpnltviskki;esakgpnltviskkit;s
akgpnltviskkitd;akgpnltviskkitds;kgpnltviskkitdsn;gpnltviskkitdsna;pnlt
viskkitdsnaf;nltviskkitdsnafv;ltviskkitdsnafvl;tviskkitdsnafvla;viskkit
dsnafvlav;iskkitdsnafvlavk;skkitdsnafvlavke;kkitdsnafvlavkev;kitdsnafvl
avkeve;itdsnafvlavkevea;tdsnafvlavkeveal;dsnafvlavkeveali;snafvlavkevea
lis;nafvlavkevealiss;afvlavkevealissi;fvlavkevealissid;vlavkevealisside
;lavkevealissidel;avkevealissidela;vkevealissidelan;kevealissidelank;ev
ealissidelanka;vealissidelankai;ealissidelankaig;alissidelankaigk;lissi
delankaigkv;issidelankaigkvi;ssidelankaigkvih;sidelankaigkvihq;idelanka
igkvihqn;delankaigkvihqnn;elankaigkvihqnng;lankaigkvihqnngl;ankaigkvihq
nngln;nkaigkvihqnnglna;kaigkvihqnnglnan;aigkvihqnnglnana;igkvihqnnglnan
ag;gkvihqnnglnanagq;kvihqnnglnanagqn;vihqnnglnanagqng;ihqnnglnanagqngs;
hqnnglnanagqngsl;qnnglnanagqngsll;nnglnanagqngslla;nglnanagqngsllag;gln
anagqngsllaga;lnanagqngsllagay;nanagqngsllagaya;anagqngsllagayai;nagqng
sllagayais;agqngsllagayaist;gqngsllagayaistl;qngsllagayaistli;ngsllagay
aistlit;gsllagayaistlite;sllagayaistlitek;llagayaistlitekl;lagayaistlit
ekls;agayaistliteklsk;gayaistliteklskl;ayaistliteklsklk;yaistliteklsklk
n;aistliteklsklkns;istliteklsklknse;stliteklsklknsee;tliteklsklknseel;l
iteklsklknseeln;iteklsklknseelnk;teklsklknseelnkk;eklsklknseelnkki;klsk
lknseelnkkie;lsklknseelnkkiee;sklknseelnkkieea;klknseelnkkieeak;lknseel
nkkieeakn;knseelnkkieeaknh;nseelnkkieeaknhs;seelnkkieeaknhse;eelnkkieea
knhsea;elnkkieeaknhseaf;lnkkieeaknhseaft;nkkieeaknhseaftn;kkieeaknhseaf
tnr;kieeaknhseaftnrl;ieeaknhseaftnrlk;eeaknhseaftnrlkg;eaknhseaftnrlkgs
;aknhseaftnrlkgsh;knhseaftnrlkgsha;nhseaftnrlkgshaq;hseaftnrlkgshaql;se
aftnrlkgshaqlg;eaftnrlkgshaqlgv;aftnrlkgshaqlgva;ftnrlkgshaqlgvaa;tnrlk
gshaqlgvaaa;nrlkgshaqlgvaaat;rlkgshaqlgvaaatd;lkgshaqlgvaaatdd;kgshaqlg
vaaatddh;gshaqlgvaaatddha;shaqlgvaaatddhak;haqlgvaaatddhake;aqlgvaaatdd
hakea;qlgvaaatddhakeai;lgvaaatddhakeail;gvaaatddhakeailk;vaaatddhakeail
ks;aaatddhakeailksn;aatddhakeailksnp;atddhakeailksnpt;tddhakeailksnptk;
ddhakeailksnptkd;dhakeailksnptkdk;hakeailksnptkdkg;akeailksnptkdkga;kea
ilksnptkdkgak;eailksnptkdkgake;ailksnptkdkgakel;ilksnptkdkgakelk;lksnpt
kdkgakelkd;ksnptkdkgakelkdl;snptkdkgakelkdls;nptkdkgakelkdlse;ptkdkgake
lkdlses;tkdkgakelkdlsesv;kdkgakelkdlsesve;dkgakelkdlsesves;kgakelkdlses
vesl;gakelkdlsesvesla;akelkdlsesveslak;kelkdlsesveslaka;elkdlsesveslaka
a;lkdlsesveslakaaq;kdlsesveslakaaqe;dlsesveslakaaqea;lsesveslakaaqeal;s
esveslakaaqeala;esveslakaaqealan;sveslakaaqealans;veslakaaqealansv;esla
kaaqealansvk;slakaaqealansvke;lakaaqealansvkel;akaaqealansvkelt;kaaqeal
ansvkeltn;aaqealansvkeltnp;aqealansvkeltnpv;qealansvkeltnpvv;ealansvkel
tnpvva;alansvkeltnpvvae;lansvkeltnpvvaes;ansvkeltnpvvaesp;nsvkeltnpvvae
spk;

## <NP_047005 outer surface protein C;Protein;Borrelia burgdorferi B31>

8 mers:
mkkntlsa;kkntlsai;kntlsail;ntlsailm;tlsailmt;lsailmtl;sailmtlf;ailmtlfl
;ilmtlflf;lmtlflfi;mtlflfis;tlflfisc;lflfiscn;flfiscnn;lfiscnns;fiscnns
g;iscnnsgk;scnnsgkd;cnnsgkdg;nnsgkdgn;nsgkdgnt;sgkdgnts;gkdgntsa;kdgnts
an;dgntsans;gntsansa;ntsansad;tsansade;sansades;ansadesv;nsadesvk;sades
vkg;adesvkgp;desvkgpn;esvkgpnl;svkgpnlt;vkgpnlte;kgpnltei;gpnlteis;pnlt
eisk;nlteiskk;lteiskki;teiskkit;eiskkitd;iskkitds;skkitdsn;kkitdsna;kit
dsnav;itdsnavl;tdsnavll;dsnavlla;snavllav;navllavk;avllavke;vllavkev;ll
avkeve;lavkevea;avkeveal;vkeveall;kevealls;eveallss;veallssi;eallssid;a
llsside;llssidei;lssideia;ssideiaa;sideiaak;ideiaaka;deiaakai;eiaakaig;
iaakaigk;aakaigkk;akaigkki;kaigkkih;aigkkihq;igkkihqn;gkkihqnn;kkihqnng
;kihqnngl;ihqnngld;hqnngldt;qnngldte;nngldten;ngldtenn;gldtennh;ldtennh
n;dtennhng;tennhngs;ennhngsl;nnhngsll;nhngslla;hngsllag;ngsllaga;gsllag
ay;sllagaya;llagayai;lagayais;agayaist;gayaistl;ayaistli;yaistlik;aistl
ikq;istlikqk;stlikqkl;tlikqkld;likqkldg;ikqkldgl;kqkldglk;qkldglkn;kldg
lkne;ldglkneg;dglknegl;glkneglk;lkneglke;kneglkek;neglkeki;eglkekid;glk
ekida;lkekidaa;kekidaak;ekidaakk;kidaakkc;idaakkcs;daakkcse;aakkcset;ak
kcsetf;kkcsetft;kcsetftn;csetftnk;setftnkl;etftnklk;tftnklke;ftnklkek;t
nklkekh;nklkekht;klkekhtd;lkekhtdl;kekhtdlg;ekhtdlgk;khtdlgke;htdlgkeg;
tdlgkegv;dlgkegvt;lgkegvtd;gkegvtda;kegvtdad;egvtdada;gvtdadak;vtdadake
;tdadakea;dadakeai;adakeail;dakeailk;akeailkt;keailktn;eailktng;ailktng
t;ilktngtk;lktngtkt;ktngtktg;tngtktkg;ngtktkga;gtktkgae;tktkgaee;ktkgae
el;tkgaeelg;kgaeelgk;gaeelgkl;aeelgklf;eelgklfe;elgklfes;lgklfesv;gklfe
sve;klfesvev;lfesvevl;fesvevls;esvevlsk;svevlska;vevlskaa;evlskaak;vlsk
aake;lskaakem;skaakeml;kaakemla;aakemlan;akemlans;kemlansv;emlansvk;mla
nsvke;lansvkel;ansvkelt;nsvkelts;svkeltsp;vkeltspv;keltspvv;eltspvva;lt
spvvae;tspvvaes;spvvaesp;pvvaespk;vvaespkk;vaespkkp;

9 mers:
mkkntlsai;kkntlsail;kntlsailm;ntlsailmt;tlsailmtl;lsailmtlf;sailmtlfl;a
ilmtlflf;ilmtlflfi;lmtlflfis;mtlflfisc;tlflfiscn;lflfiscnn;flfiscnns;lf
iscnnsg;fiscnnsgk;iscnnsgkd;scnnsgkdg;cnnsgkdgn;nnsgkdgnt;nsgkdgnts;sgk
dgntsa;gkdgntsan;kdgntsans;dgntsansa;gntsansad;ntsansade;tsansades;sans
adesv;ansadesvk;nsadesvkg;sadesvkgp;adesvkgpn;desvkgpnl;esvkgpnlt;svkgp
nlte;vkgpnltei;kgpnlteis;gpnlteisk;pnlteiskk;nlteiskki;lteiskkit;teiskk
itd;eiskkitds;iskkitdsn;skkitdsna;kkitdsnav;kitdsnavl;itdsnavll;tdsnavl
la;dsnavllav;snavllavk;navllavke;avllavkev;vllavkeve;llavkevea;lavkevea
l;avkeveall;vkevealls;kevealltss;eveallssi;veallssid;eallsside;allssidei
;llssideia;lssideiaa;ssideiaak;sideiaaka;ideiaakai;deiaakaig;eiaakaigk;
iaakaigkk;aakaigkki;akaigkkih;kaigkkihq;aigkkihqn;igkkihqnn;gkkihqnng;k
kihqnngl;kihqnngld;ihqnngldt;hqnngldte;qnngldten;nngldtenn;ngldtennh;gl
dtennhn;ldtennhng;dtennhngs;tennhngsl;ennhngsll;nnhngslla;nhngsllag;hng
sllaga;ngsllagay;gsllagaya;sllagayai;llagayais;lagayaist;agayaistl;gaya
istli;ayaistlik;yaistlikq;aistlikqk;istlikqkl;stlikqkld;tlikqkldg;likqk
ldgl;ikqkldglk;kqkldglkn;qkldglkne;kldglkneg;ldglknegl;dglkneglk;glkneg
lke;lkneglkek;kneglkeki;neglkekid;eglkekida;glkekidaa;lkekidaak;kekidaa
kk;ekidaakkc;kidaakkcs;idaakkcse;daakkcset;aakkcsetf;akkcsetft;kkcsetft
n;kcsetftnk;csetftnkl;setftnkl;etftnklke;tftnklkek;ftnklkekh;tnklkekht
;nklkekhtd;klkekhtdl;lkekhtdlg;kekhtdlgk;ekhtdlgke;khtdlgkeg;htdlgkegv;
tdlgkegvt;dlgkegvtd;lgkegvtda;gkegvtdad;kegvtdada;egvtdadak;gvtdadake;v
tdadakea;tdadakeai;dadakeail;adakeailk;dakeailkt;akeailktn;keailktng;ea
ilktngt;ailktngtk;ilktngtkt;lktngtktk;ktngtktkg;tngtktkga;ngtktkgae;gtk
tkgaee;tktkgaeel;ktkgaeelg;tkgaeelgk;kgaeelgkl;gaeelgklf;aeelgklfe;eelg

klfes;elgklfesv;lgklfesve;gklfesvev;klfesvevl;lfesvevls;fesvevlsk;esvev
lska;svevlskaa;vevlskaak;evlskaake;vlskaakem;lskaakeml;skaakemla;kaakem
lan;aakemlans;akemlansv;kemlansvk;emlansvke;mlansvkel;lansvkelt;ansvkel
ts;nsvkeltsp;svkeltspv;vkeltspvv;keltspvva;eltspvvae;ltspvvaes;tspvvaes
p;spvvaespk;pvvaespkk;vvaespkkp;

10 mers:
mkkntlsail;kkntlsailm;kntlsailmt;ntlsailmtl;tlsailmtlf;lsailmtlfl;sailm
tlflf;ailmtlflfi;ilmtlflfis;lmtlflfisc;mtlflfiscn;tlflfiscnn;lflfiscnns
;flfiscnnsg;lfiscnnsgk;fiscnnsgkd;iscnnsgkdg;scnnsgkdgn;cnnsgkdgnt;nnsg
kdgnts;nsgkdgntsa;sgkdgntsan;gkdgntsans;kdgntsansa;dgntsansad;gntsansad
e;ntsansades;tsansadesv;sansadesvk;ansadesvkg;nsadesvkgp;sadesvkgpn;ade
svkgpnl;desvkgpnlt;esvkgpnlte;svkgpnltei;vkgpnlteis;kgpnlteisk;gpnlteis
kk;pnlteiskki;nlteiskkit;lteiskkitd;teiskkitds;eiskkitdsn;iskkitdsna;sk
kitdsnav;kkitdsnavl;kitdsnavll;itdsnavlla;tdsnavllav;dsnavllavk;snavlla
vke;navllavkev;avllavkeve;vllavkevea;llavkeveal;lavkeveall;avkevealls;v
keveallss;keveallssi;eveallssid;veallsside;eallssidei;allssideia;llssid
eiaa;lssideiaak;ssideiaaka;sideiaakai;ideiaakaig;deiaakaigk;eiaakaigkk;
iaakaigkki;aakaigkkih;akaigkkihq;kaigkkihqn;aigkkihqnn;igkkihqnng;gkkih
qnngl;kkihqnngld;kihqnngldt;ihqnngldte;hqnngldten;qnngldtenn;nngldtennh
;ngldtennhn;gldtennhng;ldtennhngs;dtennhngsl;tennhngsll;ennhngslla;nnhn
gsllag;nhngsllaga;hngsllagay;ngsllagaya;gsllagayai;sllagayais;llagayais
t;lagayaistl;agayaistli;gayaistlik;ayaistlikq;yaistlikqk;aistlikqkl;ist
likqkld;stlikqkldg;tlikqkldgl;likqkldglk;ikqkldglkn;kqkldglkne;qkldglkn
eg;kldglknegl;ldglkneglk;dglkneglke;glkneglkek;lkneglkeki;kneglkekid;ne
glkekida;eglkekidaa;glkekidaak;lkekidaakk;kekidaakkc;ekidaakkcs;kidaakk
cse;idaakkcset;daakkcsetf;aakkcsetft;akkcsetftn;kkcsetftnk;kcsetftnkl;c
setftnklk;setftnklke;etftnklkek;tftnklkekh;ftnklkekht;tnklkekhtd;nklkek
htdl;klkekhtdlg;lkekhtdlgk;kekhtdlgke;ekhtdlgkeg;khtdlgkegv;htdlgkegvt;
tdlgkegvtd;dlgkegvtda;lgkegvtdad;gkegvtdada;kegvtdadak;egvtdadake;gvtda
dakea;vtdadakeai;tdadakeail;dadakeailk;adakeailkt;dakeailktn;akeailktng
;keailktngt;eailktngtk;ailktngtkt;ilktngtktk;lktngtktkg;ktngtktkga;tngt
ktkgae;ngtktkgaee;gtktkgaeel;tktkgaeelg;ktkgaeelgk;tkgaeelgkl;kgaeelgkl
f;gaeelgklfe;aeelgklfes;eelgklfesv;elgklfesve;lgklfesvev;gklfesvevl;klf
esvevls;lfesvevlsk;fesvevlska;esvevlskaa;svevlskaak;vevlskaake;evlskaak
em;vlskaakeml;lskaakemla;skaakemlan;kaakemlans;aakemlansv;akemlansvk;ke
mlansvke;emlansvkel;mlansvkelt;lansvkelts;ansvkeltsp;nsvkeltspv;svkelts
pvv;vkeltspvva;keltspvvae;eltspvvaes;ltspvvaesp;tspvvaespk;spvvaespkk;p
vvaespkkp;

11 mers:
mkkntlsailm;kkntlsailmt;kntlsailmtl;ntlsailmtlf;tlsailmtlfl;lsailmtlflf
;sailmtlflfi;ailmtlflfis;ilmtlflfisc;lmtlflfiscn;mtlflfiscnn;tlflfiscnn
s;lflfiscnnsg;flfiscnnsgk;lfiscnnsgkd;fiscnnsgkdg;iscnnsgkdgn;scnnsgkdg
nt;cnnsgkdgnts;nnsgkdgntsa;nsgkdgntsan;sgkdgntsans;gkdgntsansa;kdgntsan
sad;dgntsansade;gntsansades;ntsansadesv;tsansadesvk;sansadesvkg;ansades
vkgp;nsadesvkgpn;sadesvkgpnl;adesvkgpnlt;desvkgpnlte;esvkgpnltei;svkgpn
lteis;vkgpnlteisk;kgpnlteiskk;gpnlteiskki;pnlteiskkit;nlteiskkitd;lteis
kkitds;teiskkitdsn;eiskkitdsna;iskkitdsnav;skkitdsnavl;kkitdsnavll;kitd
snavlla;itdsnavllav;tdsnavllavk;dsnavllavke;snavllavkev;navllavkeve;avl
lavkevea;vllavkeveal;llavkeveall;lavkevealls;avkeveallss;vkeveallssi;ke
veallssid;eveallsside;veallssidei;eallssideia;allssideiaa;llssideiaak;l
ssideiaaka;ssideiaakai;sideiaakaig;ideiaakaigk;deiaakaigkk;eiaakaigkki;
iaakaigkkih;aakaigkkihq;akaigkkihqn;kaigkkihqnn;aigkkihqnng;igkkihqnngl
;gkkihqnngld;kkihqnngldt;kihqnngldte;ihqnngldten;hqnngldtenn;qnngldtenn
h;nngldtennhn;ngldtennhng;gldtennhngs;ldtennhngsl;dtennhngsll;tennhngsl
la;ennhngsllag;nnhngsllaga;nhngsllagay;hngsllagaya;ngsllagayai;gsllagay

ais;sllagayaist;llagayaistl;lagayaistli;agayaistlik;gayaistlikq;ayaistl
ikqk;yaistlikqkl;aistlikqkld;istlikqkldg;stlikqkldgl;tlikqkldglk;likqkl
dglkn;ikqkldglkne;kqkldglkneg;qkldglknegl;kldglkneglk;ldglkneglke;dglkn
eglkek;glkneglkeki;lkneglkekid;kneglkekida;neglkekidaa;eglkekidaak;glke
kidaakk;lkekidaakkc;kekidaakkcs;ekidaakkcse;kidaakkcset;idaakkcsetf;daa
kkcsetft;aakkcsetftn;akkcsetftnk;kkcsetftnkl;kcsetftnklk;csetftnklke;se
tftnklkek;etftnklkekh;tftnklkekht;ftnklkekhtd;tnklkekhtdl;nklkekhtdlg;k
lkekhtdlgk;lkekhtdlgke;kekhtdlgkeg;ekhtdlgkegv;khtdlgkegvt;htdlgkegvtd;
tdlgkegvtda;dlgkegvtdad;lgkegvtdada;gkegvtdadak;kegvtdadake;egvtdadakea
;gvtdadakeai;vtdadakeail;tdadakeailk;dadakeailkt;adakeailktn;dakeailktn
g;akeailktngt;keailktngtk;eailktngtkt;ailktngtktk;ilktngtktkg;lktngtktk
ga;ktngtktkgae;tngtktkgaee;ngtktkgaeel;gtktkgaeelg;tktkgaeelgk;ktkgaeel
gkl;tkgaeelglf;kgaeelgklfe;gaeelgklfes;aeelgklfesv;eelgklfesve;elgklfe
svev;lgklfesvevl;gklfesvevls;klfesvevlsk;lfesvevlska;fesvevlskaa;esvevl
skaak;svevlskaake;vevlskaakem;evlskaakeml;vlskaakemla;lskaakemlan;skaak
emlans;kaakemlansv;aakemlansvk;akemlansvke;kemlansvkel;emlansvkelt;mlan
svkelts;lansvkeltsp;ansvkeltspv;nsvkeltspvv;svkeltspvva;vkeltspvvae;kel
tspvvaes;eltspvvaesp;ltspvvaespk;tspvvaespkk;spvvaespkkp;

13 mers:
mkkntlsailmtl;kkntlsailmtlf;kntlsailmtlfl;ntlsailmtlflf;tlsailmtlflfi;l
sailmtlflfis;sailmtlflfisc;ailmtlflfiscn;ilmtlflfiscnn;lmtlflfiscnns;mt
lflfiscnnsg;tlflfiscnnsgk;lflfiscnnsgkd;flfiscnnsgkdg;lfiscnnsgkdgn;fis
cnnsgkdgnt;iscnnsgkdgnts;scnnsgkdgntsa;cnnsgkdgntsan;nnsgkdgntsans;nsgk
dgntsansa;sgkdgntsansad;gkdgntsansade;kdgntsansades;dgntsansadesv;gntsa
nsadesvk;ntsansadesvkg;tsansadesvkgp;sansadesvkgpn;ansadesvkgpnl;nsades
vkgpnlt;sadesvkgpnlte;adesvkgpnltei;desvkgpnlteis;esvkgpnlteisk;svkgpnl
teiskk;vkgpnlteiskki;kgpnlteiskkit;gpnlteiskkitd;pnlteiskkitds;nlteiskk
itdsn;lteiskkitdsna;teiskkitdsnav;eiskkitdsnavl;iskkitdsnavll;skkitdsna
vlla;kkitdsnavllav;kitdsnavllavk;itdsnavllavke;tdsnavllavkev;dsnavllavk
eve;snavllavkevea;navllavkeveal;avllavkeveall;vllavkevealls;llavkeveall
ss;lavkeveallssi;avkeveallssid;vkeveallsside;keveallssidei;eveallssidei
a;veallssideiaa;eallssideiaak;allssideiaaka;llssideiaakai;lssideiaakaig
;ssideiaakaigk;sideiaakaigkk;ideiaakaigkki;deiaakaigkkih;eiaakaigkkihq;
iaakaigkkihqn;aakaigkkihqnn;akaigkkihqnng;kaigkkihqnngl;aigkkihqnngld;i
gkkihqnngldt;gkkihqnngldte;kkihqnngldten;kihqnngldtenn;ihqnngldtennh;hq
nngldtennhn;qnngldtennhng;nngldtennhngs;ngldtennhngsl;gldtennhngsll;ldt
ennhngslla;dtennhngsllag;tennhngsllaga;ennhngsllagay;nnhngsllagaya;nhng
sllagayai;hngsllagayais;ngsllagayaist;gsllagayaistl;sllagayaistli;llaga
yaistlik;lagayaistlikq;agayaistlikqk;gayaistlikqkl;ayaistlikqkld;yaistl
ikqkldg;aistlikqkldgl;istlikqkldglk;stlikqkldglkn;tlikqkldglkne;likqkld
glkneg;ikqkldglknegl;kqkldglkneglk;qkldglkneglke;kldglkneglkek;ldglkneg
lkeki;dglkneglkekid;glkneglkekida;lkneglkekidaa;kneglkekidaak;neglkekid
aakk;eglkekidaakkc;glkekidaakkcs;lkekidaakkcse;kekidaakkcset;ekidaakkcs
etf;kidaakkcsetft;idaakkcsetftn;daakkcsetftnk;aakkcsetftnkl;akkcsetftnk
lk;kkcsetftnklke;kcsetftnklkek;csetftnklkekh;setftnklkekht;etftnklkekht
d;tftnklkekhtdl;ftnklkekhtdlg;tnklkekhtdlgk;nklkekhtdlgke;klkekhtdlgkeg
;lkekhtdlgkegv;kekhtdlgkegvt;ekhtdlgkegvtd;khtdlgkegvtda;htdlgkegvtdad;
tdlgkegvtdada;dlgkegvtdadak;lgkegvtdadake;gkegvtdadakea;kegvtdadakeai;e
gvtdadakeail;gvtdadakeailkt;vtdadakeailkt;tdadakeailktn;dadakeailktng;ad
akeailktngt;dakeailktngtk;akeailktngtkt;keailktngtktk;eailktngtktkg;ail
ktngtktkga;ilktngtktkgae;lktngtktkgaee;ktngtktkgaeel;tngtktkgaeelg;ngtk
tkgaeelgk;gtktkgaeelgkl;tktkgaeelgklf;ktkgaeelgklfe;tkgaeelgklfes;kgaee
lgklfesv;gaeelgklfesve;aeelgklfesvev;eelgklfesvevl;elgklfesvevls;lgklfe
svevlsk;gklfesvevlska;klfesvevlskaa;lfesvevlskaak;fesvevlskaake;esvevls
kaakem;svevlskaakeml;vevlskaakemla;evlskaakemlan;vlskaakemlans;lskaakem
lansv;skaakemlansvk;kaakemlansvke;aakemlansvkel;akemlansvkelt;kemlansvk

elts;emlansvkeltsp;mlansvkeltspv;lansvkeltspvv;ansvkeltspvva;nsvkeltspv
vae;svkeltspvvaes;vkeltspvvaesp;keltspvvaespk;eltspvvaespkk;ltspvvaespk
kp;

14 mers:
mkkntlsailmtlf;kkntlsailmtlfl;kntlsailmtlflf;ntlsailmtlflfi;tlsailmtlfl
fis;lsailmtlflfisc;sailmtlflfiscn;ailmtlflfiscnn;ilmtlflfiscnns;lmtlflf
iscnnsg;mtlflfiscnnsgk;tlflfiscnnsgkd;lflfiscnnsgkdg;flfiscnnsgkdgn;lfi
scnnsgkdgnt;fiscnnsgkdgnts;iscnnsgkdgntsa;scnnsgkdgntsan;cnnsgkdgntsans
;nnsgkdgntsansa;nsgkdgntsansad;sgkdgntsansade;gkdgntsansades;kdgntsansa
desv;dgntsansadesvk;gntsansadesvkg;ntsansadesvkgp;tsansadesvkgpn;sansad
esvkgpnl;ansadesvkgpnlt;nsadesvkgpnlte;sadesvkgpnltei;adesvkgpnlteis;de
svkgpnlteisk;esvkgpnlteiskk;svkgpnlteiskki;vkgpnlteiskkit;kgpnlteiskkit
d;gpnlteiskkitds;pnlteiskkitdsn;nlteiskkitdsna;lteiskkitdsnav;teiskkitd
snavl;eiskkitdsnavll;iskkitdsnavlla;skkitdsnavllav;kkitdsnavllavk;kitds
navllavke;itdsnavllavkev;tdsnavllavkeve;dsnavllavkevea;snavllavkeveal;n
avllavkeveall;avllavkevealls;vllavkeveallss;llavkeveallssi;lavkeveallss
id;avkeveallsside;vkeveallssidei;keveallssideia;eveallssideiaa;veallssi
deiaak;eallssideiaaka;allssideiaakai;llssideiaakaig;lssideiaakaigk;ssid
eiaakaigkk;sideiaakaigkki;ideiaakaigkkih;deiaakaigkkihq;eiaakaigkkihqn;
iaakaigkkihqnn;aakaigkkihqnng;akaigkkihqnngl;kaigkkihqnngld;aigkkihqnng
ldt;igkkihqnngldte;gkkihqnngldten;kkihqnngldtenn;kihqnngldtennh;ihqnngl
dtennhn;hqnngldtennhng;qnngldtennhngs;nngldtennhngsl;ngldtennhngsll;gld
tennhngslla;ldtennhngsllag;dtennhngsllaga;tennhngsllagay;ennhngsllagaya
;nnhngsllagayai;nhngsllagayais;hngsllagayaist;ngsllagayaistl;gsllagayai
stli;sllagayaistlik;llagayaistlikq;lagayaistlikqk;agayaistlikqkl;gayais
tlikqkld;ayaistlikqkldg;yaistlikqkldgl;aistlikqkldglk;istlikqkldglkn;st
likqkldglkne;tlikqkldglkneg;likqkldglknegl;ikqkldglkneglk;kqkldglkneglk
e;qkldglkneglkek;kldglkneglkeki;ldglkneglkekid;dglkneglkekida;glkneglke
kidaa;lkneglkekidaak;kneglkekidaakk;neglkekidaakkc;eglkekidaakkcs;glkek
idaakkcse;lkekidaakkcset;kekidaakkcsetf;ekidaakkcsetft;kidaakkcsetftn;i
daakkcsetftnk;daakkcsetftnkl;aakkcsetftnklk;akkcsetftnklke;kkcsetftnklk
ek;kcsetftnklkekh;csetftnklkekht;setftnklkekhtd;etftnklkekhtdl;tftnklke
khtdlg;ftnklkekhtdlgk;tnklkekhtdlgke;nklkekhtdlgkeg;klkekhtdlgkegv;lkek
htdlgkegvt;kekhtdlgkegvtd;ekhtdlgkegvtda;khtdlgkegvtdad;htdlgkegvtdada;
tdlgkegvtdadak;dlgkegvtdadake;lgkegvtdadakea;gkegvtdadakeai;kegvtdadake
ail;egvtdadakeailk;gvtdadakeailkt;vtdadakeailktn;tdadakeailktng;dadakea
ilktngt;adakeailktngtk;dakeailktngtkt;akeailktngtktk;keailktngtktkg;eai
lktngtktkga;ailktngtktkgae;ilktngtktkgaee;lktngtktkgaeel;ktngtktkgaeelg
;tngtktkgaeelgk;ngtktkgaeelgkl;gtktkgaeelgklf;tktkgaeelgklfe;ktkgaeelgk
lfes;tkgaeelgklfesv;kgaeelgklfesve;gaeelgklfesvev;aeelgklfesvevl;eelgkl
fesvevls;elgklfesvevlsk;lgklfesvevlska;gklfesvevlskaa;klfesvevlskaak;lf
esvevlskaake;fesvevlskaakem;esvevlskaakeml;svevlskaakemla;vevlskaakemla
n;evlskaakemlans;vlskaakemlansv;lskaakemlansvk;skaakemlansvke;kaakemlan
svkel;aakemlansvkelt;akemlansvkelts;kemlansvkeltsp;emlansvkeltspv;mlans
vkeltspvv;lansvkeltspvva;ansvkeltspvvae;nsvkeltspvvaes;svkeltspvvaesp;v
keltspvvaespk;keltspvvaespkk;eltspvvaespkkp;

15 mers:
mkkntlsailmtlfl;kkntlsailmtlflf;kntlsailmtlflfi;ntlsailmtlflfis;tlsailm
tlflfisc;lsailmtlflfiscn;sailmtlflfiscnn;ailmtlflfiscnns;ilmtlflfiscnns
g;lmtlflfiscnnsgk;mtlflfiscnnsgkd;tlflfiscnnsgkdg;lflfiscnnsgkdgn;flfis
cnnsgkdgnt;lfiscnnsgkdgnts;fiscnnsgkdgntsa;iscnnsgkdgntsan;scnnsgkdgnts
ans;cnnsgkdgntsansa;nnsgkdgntsansad;nsgkdgntsansade;sgkdgntsansades;gkd
gntsansadesv;kdgntsansadesvk;dgntsansadesvkg;gntsansadesvkgp;ntsansades
vkgpn;tsansadesvkgpnl;sansadesvkgpnlt;ansadesvkgpnlte;nsadesvkgpnltei;s
adesvkgpnlteis;adesvkgpnlteisk;desvkgpnlteiskk;esvkgpnlteiskki;svkgpnlt

eiskkit;vkgpnlteiskkitd;kgpnlteiskkitds;gpnlteiskkitdsn;pnlteiskkitdsna
;nlteiskkitdsnav;lteiskkitdsnavl;teiskkitdsnavll;eiskkitdsnavlla;iskkit
dsnavllav;skkitdsnavllavk;kkitdsnavllavke;kitdsnavllavkev;itdsnavllavke
ve;tdsnavllavkevea;dsnavllavkeveal;snavllavkeveall;navllavkevealls;avll
avkeveallss;vllavkeveallssi;llavkeveallssid;lavkeveallsside;avkeveallss
idei;vkeveallssideia;keveallssideiaa;eveallssideiaak;veallssideiaaka;ea
llssideiaakai;allssideiaakaig;llssideiaakaigk;lssideiaakaigkk;ssideiaak
aigkki;sideiaakaigkkih;ideiaakaigkkihq;deiaakaigkkihqn;eiaakaigkkihqnn;
iaakaigkkihqnng;aakaigkkihqnngl;akaigkkihqnngld;kaigkkihqnngldt;aigkkih
qnngldte;igkkihqnngldten;gkkihqnngldtenn;kkihqnngldtennh;kihqnngldtennh
n;ihqnngldtennhng;hqnngldtennhngs;qnngldtennhngsl;nngldtennhngsll;ngldt
ennhngslla;gldtennhngsllag;ldtennhngsllaga;dtennhngsllagay;tennhngsllag
aya;ennhngsllagayai;nnhngsllagayais;nhngsllagayaist;hngsllagayaistl;ngs
llagayaistli;gsllagayaistlik;sllagayaistlikq;llagayaistlikqk;lagayaistl
ikqkl;agayaistlikqkld;gayaistlikqkldg;ayaistlikqkldgl;yaistlikqkldglk;a
istlikqkldglkn;istlikqkldglkne;stlikqkldglkneg;tlikqkldglknegl;likqkldg
lkneglk;ikqkldglkneglke;kqkldglkneglkek;qkldglkneglkeki;kldglkneglkekid
;ldglkneglkekida;dglkneglkekidaa;glkneglkekidaak;lkneglkekidaakk;kneglk
ekidaakkc;neglkekidaakkcs;eglkekidaakkcse;glkekidaakkcset;lkekidaakkcse
tf;kekidaakkcsetft;ekidaakkcsetftn;kidaakkcsetftnk;idaakkcsetftnkl;daak
kcsetftnklk;aakkcsetftnklke;akkcsetftnklkek;kkcsetftnklkekh;kcsetftnklk
ekht;csetftnklkekhtd;setftnklkekhtdl;etftnklkekhtdlg;tftnklkekhtdlgk;ft
nklkekhtdlgke;tnklkekhtdlgkeg;nklkekhtdlgkegv;klkekhtdlgkegvt;lkekhtdlg
kegvtd;kekhtdlgkegvtda;ekhtdlgkegvtdad;khtdlgkegvtdada;htdlgkegvtdadak;
tdlgkegvtdadake;dlgkegvtdadakea;lgkegvtdadakeai;gkegvtdadakeail;kegvtda
dakeailk;egvtdadakeailkt;gvtdadakeailktn;vtdadakeailktng;tdadakeailktng
t;dadakeailktngtk;adakeailktngtkt;dakeailktngtktk;akeailktngtktkg;keail
ktngtktkga;eailktngtktkgae;ailktngtktkgaee;ilktngtktkgaeel;lktngtktkgae
elg;ktngtktkgaeelgk;tngtktkgaeelgkl;ngtktkgaeelgklf;gtktkgaeelgklfe;tkt
kgaeelgklfes;ktkgaeelgklfesv;tkgaeelgklfesve;kgaeelgklfesvev;gaeelgklfe
svevl;aeelgklfesvevls;eelgklfesvevlsk;elgklfesvevlska;lgklfesvevlskaa;g
klfesvevlskaak;klfesvevlskaake;lfesvevlskaakem;fesvevlskaakeml;esvevlsk
aakemla;svevlskaakemlan;vevlskaakemlans;evlskaakemlansv;vlskaakemlansvk
;lskaakemlansvke;skaakemlansvkel;kaakemlansvkelt;aakemlansvkelts;akemla
nsvkeltsp;kemlansvkeltspv;emlansvkeltspvv;mlansvkeltspvva;lansvkeltspvv
ae;ansvkeltspvvaes;nsvkeltspvvaesp;svkeltspvvaespk;vkeltspvvaespkk;kelt
spvvaespkkp;

16 mers:
mkkntlsailmtlflf;kkntlsailmtlflfi;kntlsailmtlflfis;ntlsailmtlflfisc;tls
ailmtlflfiscn;lsailmtlflfiscnn;sailmtlflfiscnns;ailmtlflfiscnnsg;ilmtlf
lfiscnnsgk;lmtlflfiscnnsgkd;mtlflfiscnnsgkdg;tlflfiscnnsgkdgn;lflfiscnn
sgkdgnt;flfiscnnsgkdgnts;lfiscnnsgkdgntsa;fiscnnsgkdgntsan;iscnnsgkdgnt
sans;scnnsgkdgntsansa;cnnsgkdgntsansad;nnsgkdgntsansade;nsgkdgntsansade
s;sgkdgntsansadesv;gkdgntsansadesvk;kdgntsansadesvkg;dgntsansadesvkgp;g
ntsansadesvkgpn;ntsansadesvkgpnl;tsansadesvkgpnlt;sansadesvkgpnlte;ansa
desvkgpnltei;nsadesvkgpnlteis;sadesvkgpnlteisk;adesvkgpnlteiskk;desvkgp
nlteiskki;esvkgpnlteiskkit;svkgpnlteiskkitd;vkgpnlteiskkitds;kgpnlteisk
kitdsn;gpnlteiskkitdsna;pnlteiskkitdsnav;nlteiskkitdsnavl;lteiskkitdsna
vll;teiskkitdsnavlla;eiskkitdsnavllav;iskkitdsnavllavk;skkitdsnavllavke
;kkitdsnavllavkev;kitdsnavllavkeve;itdsnavllavkevea;tdsnavllavkeveal;ds
navllavkeveall;snavllavkevealls;navllavkeveallss;avllavkeveallssi;vllav
keveallssid;llavkeveallsside;lavkeveallssidei;avkeveallssideia;vkeveall
ssideiaa;keveallssideiaak;eveallssideiaaka;veallssideiaakai;eallssideia
akaig;allssideiaakaigk;llssideiaakaigkk;lssideiaakaigkki;ssideiaakaigkk
ih;sideiaakaigkkihq;ideiaakaigkkihqn;deiaakaigkkihqnn;eiaakaigkkihqnng;
iaakaigkkihqnngl;aakaigkkihqnngld;akaigkkihqnngldt;kaigkkihqnngldte;aig

kkihqnngldten;igkkihqnngldtenn;gkkihqnngldtennh;kkihqnngldtennhn;kihqnn
gldtennhng;ihqnngldtennhngs;hqnngldtennhngsl;qnngldtennhngsll;nngldtenn
hngslla;ngldtennhngsllag;gldtennhngsllaga;ldtennhngsllagay;dtennhngslla
gaya;tennhngsllagayai;ennhngsllagayais;nnhngsllagayaist;nhngsllagayaist
l;hngsllagayaistli;ngsllagayaistlik;gsllagayaistlikq;sllagayaistlikqk;l
lagayaistlikqkl;lagayaistlikqkld;agayaistlikqkldg;gayaistlikqkldgl;ayai
stlikqkldglk;yaistlikqkldglkn;aistlikqkldglkne;istlikqkldglkneg;stlikqk
ldglknegl;tlikqkldglkneglk;likqkldglkneglke;ikqkldglkneglkek;kqkldglkne
glkeki;qkldglkneglkekid;kldglkneglkekida;ldglkneglkekidaa;dglkneglkekid
aak;glkneglkekidaakk;lkneglkekidaakkc;kneglkekidaakkcs;neglkekidaakkcse
;eglkekidaakkcset;glkekidaakkcsetf;lkekidaakkcsetft;kekidaakkcsetftn;ek
idaakkcsetftnk;kidaakkcsetftnkl;idaakkcsetftnklk;daakkcsetftnklke;aakkc
setftnklkek;akkcsetftnklkekh;kkcsetftnklkekht;kcsetftnklkekhtd;csetftnk
lkekhtdl;setftnklkekhtdlg;etftnklkekhtdlgk;tftnklkekhtdlgke;ftnklkekhtd
lgkeg;tnklkekhtdlgkegv;nklkekhtdlgkegvt;klkekhtdlgkegvtd;lkekhtdlgkegvt
da;kekhtdlgkegvtdad;ekhtdlgkegvtdada;khtdlgkegvtdadak;htdlgkegvtdadake;
tdlgkegvtdadakea;dlgkegvtdadakeai;lgkegvtdadakeail;gkegvtdadakeailk;keg
vtdadakeailkt;egvtdadakeailktn;gvtdadakeailktng;vtdadakeailktngt;tdadak
eailktngtk;dadakeailktngtkt;adakeailktngtktk;dakeailktngtktkg;akeailktn
gtktkga;keailktngtktkgae;eailktngtktkgaee;ailktngtktkgaeel;ilktngtktkga
eelg;lktngtktkgaeelgk;ktngtktkgaeelgkl;tngtktkgaeelgklf;ngtktkgaeelgklf
e;gtktkgaeelgklfes;tktkgaeelgklfesv;ktkgaeelgklfesve;tkgaeelgklfesvev;k
gaeelgklfesvevl;gaeelgklfesvevls;aeelgklfesvevlsk;eelgklfesvevlska;elgk
lfesvevlskaa;lgklfesvevlskaak;gklfesvevlskaake;klfesvevlskaakem;lfesvev
lskaakeml;fesvevlskaakemla;esvevlskaakemlan;svevlskaakemlans;vevlskaake
mlansv;evlskaakemlansvk;vlskaakemlansvke;lskaakemlansvkel;skaakemlansvk
elt;kaakemlansvkelts;aakemlansvkeltsp;akemlansvkeltspv;kemlansvkeltspvv
;emlansvkeltspvva;mlansvkeltspvvae;lansvkeltspvvaes;ansvkeltspvvaesp;ns
vkeltspvvaespk;svkeltspvvaespkk;vkeltspvvaespkkp;


## \<CAF34024 outer surface protein VlsE;Protein;Borrelia garinii\>

8 mers:
mkkissai;kkissaif;kissaifi;issaifiv;ssaifiva;saifivaf;aifivafl;ifivafla
;fivaflal;ivaflali;vaflalig;aflaligc;flaligck;laligckn;aligcknn;ligcknn
v;igcknnvg;gcknnvgg;cknnvggd;knnvggdd;nnvggddk;nvggddkk;vggddkkd;ggddkk
dt;gddkkdta;ddkkdtaa;dkkdtaas;kkdtaasi;kdtaasif;dtaasify;taasifyq;aasif
yqs;asifyqsi;sifyqsii;ifyqsiin;fyqsiinl;yqsiinlg;qsiinlgn;siinlgng;iinl
gngf;inlgngfi;nlgngfie;lgngfiev;gngfievf;ngfievfn;gfievfna;fievfnaf;iev
fnafs;evfnafsg;vfnafsgl;fnafsglv;nafsglva;afsglvad;fsglvada;sglvadaf;gl
vadafs;lvadafsk;vadafska;adafskad;dafskadp;afskadpk;fskadpkk;skadpkks;k
adpkksd;adpkksdv;dpkksdvk;pkksdvkt;kksdvkty;ksdvktyf;sdvktyfd;dvktyfds;
vktyfdsi;ktyfdsit;tyfdsitk;yfdsitkt;fdsitktl;dsitktlk;sitktlkd;itktlkdt
;tktlkdtk;ktlkdtkt;tlkdtktk;lkdtktkl;kdtktkle;dtktkled;tktkledi;ktkledi
s;tkledisk;klediske;lediskek;ediskekt;diskektg;iskektgg;skektgge;kektgg
ek;ektggekt;ktggektp;tggektpa;ggektpav;gektpave;ektpaveg;ktpavegi;tpave
gia;pavegiae;avegiaev;vegiaevv;egiaevvk;giaevvkt;iaevvktv;aevvktvg;evvk
tvge;vvktvgew;vktvgewl;ktvgewld;tvgewldg;vgewldgl;gewldgli;ewldglik;wld
glika;ldglikaa;dglikaae;glikaaeg;likaaegg;ikaaeggg;kaaegggk;aaegggka;ae
gggkaa;egggkaad;gggkaadg;ggkaadgg;gkaadggg;kaadgggs;aadgggsd;adgggsdk;d
gggsdki;gggsdkig;ggsdkign;gsdkignv;sdkignva;dkignvaa;kignvaag;ignvaagg;
gnvaaggg;nvaaggga;vaagggag;aagggaga;agggagad;gggagadk;ggagadke;gagadkes
;agadkesv;gadkesvn;adkesvng;dkesvngi;kesvngia;esvngiag;svngiaga;vngiaga
i;ngiagaik;giagaikg;iagaikgi;agaikgiv;gaikgive;aikgivea;ikgiveaa;kgivea
ak;giveaakk;iveaakkv;veaakkve;eaakkveg;aakkvegv;akkvegvk;kkvegvkf;kvegv

kfa;vegvkfap;egvkfapk;gvkfapka;vkfapkaa;kfapkaaa;fapkaaad;apkaaada;pkaa
adaa;kaaadaaa;aaadaaaa;aadaaaad;adaaaadg;daaaadgn;aaaadgnk;aaadgnkk;aad
gnkka;adgnkkag;dgnkkagk;gnkkagkl;nkkagklf;kkagklfg;kagklfgt;agklfgta;gk
lfgtaa;klfgtaag;lfgtaaga;fgtaagad;gtaagada;taagadag;aagadagd;agadagdv;g
adagdvk;adagdvkd;dagdvkda;agdvkdaa;gdvkdaaa;dvkdaaaa;vkdaaaav;kdaaaavg;
daaaavga;aaaavgav;aaavgavs;aavgavsg;avgavsge;vgavsgeq;gavsgeqi;avsgeqil
;vsgeqiln;sgeqilna;geqilnai;eqilnaiv;qilnaivt;ilnaivta;lnaivtaa;naivtaa
g;aivtaagq;ivtaagqa;vtaagqag;taagqagq;aagqagqa;agqagqag;gqagqagk;qagqag
kk;agqagkka;gqagkkad;qagkkade;agkkadea;gkkadeak;kkadeakn;kadeakna;adeak
nai;deaknaie;eaknaiea;aknaieaa;knaieaai;naieaaig;aieaaiga;ieaaigaa;eaai
gaag;aaigaagd;aigaagda;igaagdad;gaagdadf;aagdadfg;agdadfgd;gdadfgdd;dad
fgddi;adfgddik;dfgddikk;fgddikkk;gddikkkn;ddikkknd;dikkkndq;ikkkndqi;kk
kndqia;kkndqiaa;kndqiaaa;ndqiaaal;dqiaaalv;qiaaalvl;iaaalvlr;aaalvlrg;a
aalvlrgv;alvlrgva;lvlrgvak;vlrgvakd;lrgvakdg;rgvakdgk;gvakdgkf;vakdgkfa;
akdgkfag;kdgkfaga;

9 mers:
mkkissaif;kkissaifi;kissaifiv;issaifiva;ssaifivaf;saifivafl;aifivafla;i
fivaflal;fivaflali;ivaflalig;vaflaligc;aflaligck;flaligckn;laligcknn;al
igcknnv;ligcknnvg;igcknnvgg;gcknnvggd;cknnvggdd;knnvggddk;nnvggddkk;nvg
gddkkd;vggddkkdt;ggddkkdta;gddkkdtaa;ddkkdtaas;dkkdtaasi;kkdtaasif;kdta
asify;dtaasifyq;taasifyqs;aasifyqsi;asifyqsii;sifyqsiin;ifyqsiinl;fyqsi
inlg;yqsiinlgn;qsiinlgng;siinlgngf;iinlgngfi;inlgngfie;nlgngfiev;lgngfi
evf;gngfievfn;ngfievfna;gfievfnaf;fievfnafs;ievfnafsg;evfnafsgl;vfnafsg
lv;fnafsglva;nafsglvad;afsglvada;fsglvadaf;sglvadafs;glvadafsk;lvadafsk
a;vadafskad;adafskadp;dafskadpk;afskadpkk;fskadpkks;skadpkksd;kadpkksdv
;adpkksdvk;dpkksdvkt;pkksdvkty;kksdvktyf;ksdvktyfd;sdvktyfds;dvktyfdsi;
vktyfdsit;ktyfdsitk;tyfdsitkt;yfdsitktl;fdsitktlk;dsitktlkd;sitktlkdt;i
tktlkdtk;tktlkdtkt;ktlkdtktk;tlkdtktkl;lkdtktkle;kdtktkled;dtktkledi;tk
tkledis;ktkledisk;tklediske;klediskek;lediskekt;ediskektg;diskektgg;isk
ektgge;skektggek;kektggekt;ektggektp;ktggektpa;tggektpav;ggektpave;gekt
paveg;ektpavegi;ktpavegia;tpavegiae;pavegiaev;avegiaevv;vegiaevvk;egiae
vvkt;giaevvktv;iaevvktvg;aevvktvge;evvktvgew;vvktvgewl;vktvgewld;ktvgew
ldg;tvgewldgl;vgewldgli;gewldglik;ewldglika;wldglikaa;ldglikaae;dglikaa
eg;glikaaegg;likaaeggg;ikaaegggk;kaaegggka;aaegggkaa;aegggkaad;egggkaad
g;gggkaadgg;ggkaadggg;gkaadgggs;kaadgggsd;aadgggsdk;adgggsdki;dgggsdkig
;gggsdkign;ggsdkignv;gsdkignva;sdkignvaa;dkignvaag;kignvaagg;ignvaaggg;
gnvaaggga;nvaagggag;vaagggaga;aagggagad;agggagadk;gggagadke;ggagadkes;g
agadkesv;agadkesvn;gadkesvng;adkesvngi;dkesvngia;kesvngiag;esvngiaga;sv
ngiagai;vngiagaik;ngiagaikg;giagaikgi;iagaikgiv;agaikgive;gaikgivea;aik
giveaa;ikgiveaak;kgiveaakk;giveaakkv;iveaakkve;veaakkveg;eaakkvegv;aakk
vegvk;akkvegvkf;kkvegvkfa;kvegvkfap;vegvkfapk;egvkfapka;gvkfapkaa;vkfap
kaaa;kfapkaaad;fapkaaada;apkaaadaa;pkaaadaaa;kaaadaaaa;aaadaaaad;aadaaa
adg;adaaaadgn;daaaadgnk;aaaadgnkk;aaadgnkka;aadgnkkag;adgnkkagk;dgnkkag
kl;gnkkagklf;nkkagklfg;kkagklfgt;kagklfgta;agklfgtaa;gklfgtaag;klfgtaag
a;lfgtaagad;fgtaagada;gtaagadag;taagadagd;aagadagdv;agadagdvk;gadagdvkd
;adagdvkda;dagdvkdaa;agdvkdaaa;gdvkdaaaa;dvkdaaaav;vkdaaaavg;kdaaaavga;
daaaavgav;aaaavgavs;aaavgavsg;aavgavsge;avgavsgeq;vgavsgeqi;gavsgeqil;a
vsgeqiln;vsgeqilna;sgeqilnai;geqilnaiv;eqilnaivt;qilnaivta;ilnaivtaa;ln
aivtaag;naivtaagq;aivtaagqa;ivtaagqag;vtaagqagq;taagqagqa;aagqagqag;agq
agqagk;gqagqagkk;qagqagkka;agqagkkad;gqagkkade;qagkkadea;agkkadeak;gkka
deakn;kkadeakna;kadeaknai;adeaknaie;deaknaiea;eaknaieaa;aknaieaai;knaie
aaig;naieaaiga;aieaaigaa;ieaaigaag;eaaigaagd;aaigaagda;aigaagdad;igaagd
adf;gaagdadfg;aagdadfgd;agdadfgdd;gdadfgddi;dadfgddik;adfgddikk;dfgddik
kk;fgddikkkn;gddikkknd;ddikkkndq;dikkkndqi;ikkkndqia;kkkndqiaa;kkndqiaa
a;kndqiaaal;ndqiaaalv;dqiaaalvl;qiaaalvlr;iaaalvlrg;aaalvlrgv;aalvlrgva

;alvlrgvak;lvlrgvakd;vlrgvakdg;lrgvakdgk;rgvakdgkf;gvakdgkfa;vakdgkfag;
akdgkfaga;

10 mers:
mkkissaifi;kkissaifiv;kissaifiva;issaifivaf;ssaifivafl;saifivafla;aifiv
aflal;ifivaflali;fivaflalig;ivaflaligc;vaflaligck;aflaligckn;flaligcknn
;laligcknnv;aligcknnvg;ligcknnvgg;igcknnvggd;gcknnvggdd;cknnvggddk;knnv
ggddkk;nnvggddkkd;nvggddkkdt;vggddkkdta;ggddkkdtaa;gddkkdtaas;ddkkdtaas
i;dkkdtaasif;kkdtaasify;kdtaasifyq;dtaasifyqs;taasifyqsi;aasifyqsii;asi
fyqsiin;sifyqsiinl;ifyqsiinlg;fyqsiinlgn;yqsiinlgng;qsiinlgngf;siinlgng
fi;iinlgngfie;inlgngfiev;nlgngfievf;lgngfievfn;gngfievfna;ngfievfnaf;gf
ievfnafs;fievfnafsg;ievfnafsgl;evfnafsglv;vfnafsglva;fnafsglvad;nafsglv
ada;afsglvadaf;fsglvadafs;sglvadafsk;glvadafska;lvadafskad;vadafskadp;a
dafskadpk;dafskadpkk;afskadpkks;fskadpkksd;skadpkksdv;kadpkksdvk;adpkks
dvkt;dpkksdvkty;pkksdvktyf;kksdvktyfd;ksdvktyfds;sdvktyfdsi;dvktyfdsit;
vktyfdsitk;ktyfdsitkt;tyfdsitktl;yfdsitktlk;fdsitktlkd;dsitktlkdt;sitkt
lkdtk;itktlkdtkt;tktlkdtktk;ktlkdtktkl;tlkdtktkle;lkdtktkled;kdtktkledi
;dtktkledis;tktkledisk;ktklediske;tklediskek;klediskekt;lediskektg;edis
kektgg;diskektgge;iskektggek;skektggekt;kektggektp;ektggektpa;ktggektpa
v;tggektpave;ggektpaveg;gektpavegi;ektpavegia;ktpavegiae;tpavegiaev;pav
egiaevv;avegiaevvk;vegiaevvkt;egiaevvktv;giaevvktvg;iaevvktvge;aevvktvg
ew;evvktvgewl;vvktvgewld;vktvgewldg;ktvgewldgl;tvgewldgli;vgewldglik;ge
wldglika;ewldglikaa;wldglikaae;ldglikaaeg;dglikaaegg;glikaaeggg;likaaeg
ggk;ikaaegggka;kaaegggkaa;aaegggkaad;aegggkaadg;egggkaadgg;gggkaadggg;g
gkaadgggs;gkaadgggsd;kaadgggsdk;aadgggsdki;adgggsdkig;dgggsdkign;gggsdk
ignv;ggsdkignva;gsdkignvaa;sdkignvaag;dkignvaagg;kignvaaggg;ignvaaggga;
gnvaagggag;nvaagggaga;vaagggagad;aagggagadk;agggagadke;gggagadkes;ggaga
dkesv;gagadkesvn;agadkesvng;gadkesvngi;adkesvngia;dkesvngiag;kesvngiaga
;esvngiagai;svngiagaik;vngiagaikg;ngiagaikgi;giagaikgiv;iagaikgive;agai
kgivea;gaikgiveaa;aikgiveaak;ikgiveaakk;kgiveaakkv;giveaakkve;iveaakkve
g;veaakkvegv;eaakkvegvk;aakkvegvkf;akkvegvkfa;kkvegvkfap;kvegvkfapk;veg
vkfapka;egvkfapkaa;gvkfapkaaa;vkfapkaaad;kfapkaaada;fapkaaadaa;apkaaada
aa;pkaaadaaaa;kaaadaaaad;aaadaaaadg;aadaaaadgn;adaaaadgnk;daaaadgnkk;aa
aadgnkka;aaadgnkkag;aadgnkkagk;adgnkkagkl;dgnkkagklf;gnkkagklfg;nkkagkl
fgt;kkagklfgta;kagklfgtaa;agklfgtaag;gklfgtaaga;klfgtaagad;lfgtaagada;f
gtaagadag;gtaagadagd;taagadagdv;aagadagdvk;agadagdvkd;gadagdvkda;adagdv
kdaa;dagdvkdaaa;agdvkdaaaa;gdvkdaaaav;dvkdaaaavg;vkdaaaavga;kdaaaavgav;
daaaavgavs;aaaavgavsg;aaavgavsge;aavgavsgeq;avgavsgeqi;vgavsgeqil;gavsg
eqiln;avsgeqilna;vsgeqilnai;sgeqilnaiv;geqilnaivt;eqilnaivta;qilnaivtaa
;ilnaivtaag;lnaivtaagq;naivtaagqa;aivtaagqag;ivtaagqagq;vtaagqagqa;taag
qagqag;aagqagqagk;agqagqagkk;gqagqagkka;qagqagkkad;agqagkkade;gqagkkade
a;qagkkadeak;agkkadeakn;gkkadeakna;kkadeaknai;kadeaknaie;adeaknaiea;dea
knaieaa;eaknaieaai;aknaieaaig;knaieaaiga;naieaaigaa;aieaaigaag;ieaaigaa
gd;eaaigaagda;aaigaagdad;aigaagdadf;igaagdadfg;gaagdadfgd;aagdadfgdd;ag
dadfgddi;gdadfgddik;dadfgddikk;adfgddikkk;dfgddikkkn;fgddikkknd;gddikkk
ndq;ddikkkndqi;dikkkndqia;ikkkndqiaa;kkkndqiaaa;kkndqiaaal;kndqiaaalv;n
dqiaaalvl;dqiaaalvlr;qiaaalvlrg;iaaalvlrgv;aaalvlrgva;aalvlrgvak;alvlrg
vakd;lvlrgvakdg;vlrgvakdgk;lrgvakdgkf;rgvakdgkfa;gvakdgkfag;vakdgkfaga;

11 mers:
mkkissaifiv;kkissaifiva;kissaifivaf;issaifivafl;ssaifivafla;saifivaflal
;aifivaflali;ifivaflalig;fivaflaligc;ivaflaligck;vaflaligckn;aflaligckn
n;flaligcknnv;laligcknnvg;aligcknnvgg;ligcknnvggd;igcknnvggdd;gcknnvggd
dk;cknnvggddkk;knnvggddkkd;nnvggddkkdt;nvggddkkdta;vggddkkdtaa;ggddkkdt
aas;gddkkdtaasi;ddkkdtaasif;dkkdtaasify;kkdtaasifyq;kdtaasifyqs;dtaasif
yqsi;taasifyqsii;aasifyqsiin;asifyqsiinl;sifyqsiinlg;ifyqsiinlgn;fyqsii
nlgng;yqsiinlgngf;qsiinlgngfi;siinlgngfie;iinlgngfiev;inlgngfievf;nlgng

fievfn;lgngfievfna;gngfievfnaf;ngfievfnafs;gfievfnafsg;fievfnafsgl;ievf
nafsglv;evfnafsglva;vfnafsglvad;fnafsglvada;nafsglvadaf;afsglvadafs;fsg
lvadafsk;sglvadafska;glvadafskad;lvadafskadp;vadafskadpk;adafskadpkk;da
fskadpkks;afskadpkksd;fskadpkksdv;skadpkksdvk;kadpkksdvkt;adpkksdvkty;d
pkksdvktyf;pkksdvktyfd;kksdvktyfds;ksdvktyfdsi;sdvktyfdsit;dvktyfdsitk;
vktyfdsitkt;ktyfdsitktl;tyfdsitktlk;yfdsitktlkd;fdsitktlkdt;dsitktlkdtk
;sitktlkdtkt;itktlkdtktk;tktlkdtktkl;ktlkdtktkle;tlkdtktkled;lkdtktkled
i;kdtktkledis;dtktkledisk;tktklediske;ktklediskek;tklediskekt;klediskek
tg;lediskektgg;ediskektgge;diskektggek;iskektggekt;skektggektp;kektggek
tpa;ektggektpav;ktggektpave;tggektpaveg;ggektpavegi;gektpavegia;ektpave
giae;ktpavegiaev;tpavegiaevv;pavegiaevvk;avegiaevvkt;vegiaevvktv;egiaev
vktvg;giaevvktvge;iaevvktvgew;aevvktvgewl;evvktvgewld;vvktvgewldg;vktvg
ewldgl;ktvgewldgli;tvgewldglik;vgewldglika;gewldglikaa;ewldglikaae;wldg
likaaeg;ldglikaaegg;dglikaaeggg;glikaaegggk;likaaegggka;ikaaegggkaa;kaa
egggkaad;aaegggkaadg;aegggkaadgg;egggkaadggg;gggkaadgggs;ggkaadgggsd;gk
aadgggsdk;kaadgggsdki;aadgggsdkig;adgggsdkign;dgggsdkignv;gggsdkignva;g
gsdkignvaa;gsdkignvaag;sdkignvaagg;dkignvaaggg;kignvaaggga;ignvaagggag;
gnvaagggaga;nvaagggagad;vaagggagadk;aagggagadke;agggagadkes;gggagadkesv
;ggagadkesvn;gagadkesvng;agadkesvngi;gadkesvngia;adkesvngiag;dkesvngiag
a;kesvngiagai;esvngiagaik;svngiagaikg;vngiagaikgi;ngiagaikgiv;giagaikgi
ve;iagaikgivea;agaikgiveaa;gaikgiveaak;aikgiveaakk;ikgiveaakkv;kgiveaak
kve;giveaakkveg;iveaakkvegv;veaakkvegvk;eaakkvegvkf;aakkvegvkfa;akkvegv
kfap;kkvegvkfapk;kvegvkfapka;vegvkfapkaa;egvkfapkaaa;gvkfapkaaad;vkfapk
aaada;kfapkaaadaa;fapkaaadaaa;apkaaadaaaa;pkaaadaaaad;kaaadaaaadg;aaada
aaadgn;aadaaaadgnk;adaaaadgnkk;daaaadgnkka;aaaadgnkkag;aaadgnkkagk;aadg
nkkagkl;adgnkkagklf;dgnkkagklfg;gnkkagklfgt;nkkagklfgta;kkagklfgtaa;kag
klfgtaag;agklfgtaaga;gklfgtaagad;klfgtaagada;lfgtaagadag;fgtaagadagd;gt
aagadagdv;taagadagdvk;aagadagdvkd;agadagdvkda;gadagdvkdaa;adagdvkdaaa;d
agdvkdaaaa;agdvkdaaaav;gdvkdaaaavg;dvkdaaaavga;vkdaaaavgav;kdaaaavgavs;
daaaavgavsg;aaaavgavsge;aaavgavsgeq;aavgavsgeqi;avgavsgeqil;vgavsgeqiln
;gavsgeqilna;avsgeqilnai;vsgeqilnaiv;sgeqilnaivt;geqilnaivta;eqilnaivta
a;qilnaivtaag;ilnaivtaagq;lnaivtaagqa;naivtaagqag;aivtaagqagq;ivtaagqag
qa;vtaagqagqag;taagqagqagk;aagqagqagkk;agqagqagkka;gqagqagkkad;qagqagkk
ade;agqagkkadea;gqagkkadeak;qagkkadeakn;agkkadeakna;gkkadeaknai;kkadeak
naie;kadeaknaiea;adeaknaieaa;deaknaieaai;eaknaieaaig;aknaieaaiga;knaiea
aigaa;naieaaigaag;aieaaigaagd;ieaaigaagda;eaaigaagdad;aaigaagdadf;aigaa
gdadfg;igaagdadfgd;gaagdadfgdd;aagdadfgddi;agdadfgddik;gdadfgddikk;dadf
gddikkk;adfgddikkkn;dfgddikkknd;fgddikkkndq;gddikkkndqi;ddikkkndqia;dik
kkndqiaa;ikkkndqiaaa;kkkndqiaaal;kkndqiaaalv;kndqiaaalvl;ndqiaaalvlr;dq
iaaalvlrg;qiaaalvlrgv;iaaalvlrgva;aaalvlrgvak;aalvlrgvakd;alvlrgvakdg;l
vlrgvakdgk;vlrgvakdgkf;lrgvakdgkfa;rgvakdgkfag;gvakdgkfaga;

13 mers:
mkkissaifivaf;kkissaifivafl;kissaifivafla;issaifivaflal;ssaifivaflali;s
aifivaflalig;aifivaflaligc;ifivaflaligck;fivaflaligckn;ivaflaligcknn;va
flaligcknnv;aflaligcknnvg;flaligcknnvgg;laligcknnvggd;aligcknnvggdd;lig
cknnvggddk;igcknnvggddkk;gcknnvggddkkd;cknnvggddkkdt;knnvggddkkdta;nnvg
gddkkdtaa;nvggddkkdtaas;vggddkkdtaasi;ggddkkdtaasif;gddkkdtaasify;ddkkd
taasifyq;dkkdtaasifyqs;kkdtaasifyqsi;kdtaasifyqsii;dtaasifyqsiin;taasif
yqsiinl;aasifyqsiinlg;asifyqsiinlgn;sifyqsiinlgng;ifyqsiinlgngf;fyqsiin
lgngfi;yqsiinlgngfie;qsiinlgngfiev;siinlgngfievf;iinlgngfievfn;inlgngfi
evfna;nlgngfievfnaf;lgngfievfnafs;gngfievfnafsg;ngfievfnafsgl;gfievfnaf
sglv;fievfnafsglva;ievfnafsglvad;evfnafsglvada;vfnafsglvadaf;fnafsglvad
afs;nafsglvadafsk;afsglvadafska;fsglvadafskad;sglvadafskadp;glvadafskad
pk;lvadafskadpkk;vadafskadpkks;adafskadpkksd;dafskadpkksdv;afskadpkksdv
k;fskadpkksdvkt;skadpkksdvkty;kadpkksdvktyf;adpkksdvktyfd;dpkksdvktyfds
;pkksdvktyfdsi;kksdvktyfdsit;ksdvktyfdsitk;sdvktyfdsitkt;dvktyfdsitktl;

vktyfdsitktlk;ktyfdsitktlkd;tyfdsitktlkdt;yfdsitktlkdtk;fdsitktlkdtkt;d
sitktlkdtktk;sitktlkdtktkl;itktlkdtktkle;tktlkdtktkled;ktlkdtktkledi;tl
kdtktkledis;lkdtktkledisk;kdtktklediske;dtktklediskek;tktklediskekt;ktk
lediskektg;tklediskektgg;klediskektgge;lediskektggek;ediskektggekt;disk
ektggektp;iskektggektpa;skektggektpav;kektggektpave;ektggektpaveg;ktgge
ktpavegi;tggektpavegia;ggektpavegiae;gektpavegiaev;ektpavegiaevv;ktpave
giaevvk;tpavegiaevvkt;pavegiaevvktv;avegiaevvktvg;vegiaevvktvge;egiaevv
ktvgew;giaevvktvgewl;iaevvktvgewld;aevvktvgewldg;evvktvgewldgl;vvktvgew
ldgli;vktvgewldglik;ktvgewldglika;tvgewldglikaa;vgewldglikaae;gewldglik
aaeg;ewldglikaaegg;wldglikaaeggg;ldglikaaegggk;dglikaaegggka;glikaaeggg
kaa;likaaegggkaad;ikaaegggkaadg;kaaegggkaadgg;aaegggkaadggg;aegggkaadgg
gs;egggkaadgggsd;gggkaadgggsdk;ggkaadgggsdki;gkaadgggsdkig;kaadgggsdkig
n;aadgggsdkignv;adgggsdkignva;dgggsdkignvaa;gggsdkignvaag;ggsdkignvaagg
;gsdkignvaaggg;sdkignvaaggga;dkignvaagggag;kignvaagggaga;ignvaagggagad;
gnvaagggagadk;nvaagggagadke;vaagggagadkes;aagggagadkesv;agggagadkesvn;g
ggagadkesvng;ggagadkesvngi;gagadkesvngia;agadkesvngiag;gadkesvngiaga;ad
kesvngiagai;dkesvngiagaik;kesvngiagaikg;esvngiagaikgi;svngiagaikgiv;vng
iagaikgive;ngiagaikgivea;giagaikgiveaa;iagaikgiveaak;agaikgiveaakk;gaik
giveaakkv;aikgiveaakkve;ikgiveaakkveg;kgiveaakkvegv;giveaakkvegvk;iveaa
kkvegvkf;veaakkvegvkfa;eaakkvegvkfap;aakkvegvkfapk;akkvegvkfapka;kkvegv
kfapkaa;kvegvkfapkaaa;vegvkfapkaaad;egvkfapkaaada;gvkfapkaaadaa;vkfapka
aadaaa;kfapkaaadaaaa;fapkaaadaaaad;apkaaadaaaadg;pkaaadaaaadgn;kaaadaaa
adgnk;aaadaaaadgnkk;aadaaaadgnkka;adaaaadgnkkag;daaaadgnkkagk;aaaadgnkk
agkl;aaadgnkkagklf;aadgnkkagklfg;adgnkkagklfgt;dgnkkagklfgta;gnkkagklfg
taa;nkkagklfgtaag;kkagklfgtaaga;kagklfgtaagad;agklfgtaagada;gklfgtaagad
ag;klfgtaagadagd;lfgtaagadagdv;fgtaagadagdvk;gtaagadagdvkd;taagadagdvkd
a;aagadagdvkdaa;agadagdvkdaaa;gadagdvkdaaaa;adagdvkdaaaav;dagdvkdaaaavg
;agdvkdaaaavga;gdvkdaaaavgav;dvkdaaaavgavs;vkdaaaavgavsg;kdaaaavgavsge;
daaaavgavsgeq;aaaavgavsgeqi;aaavgavsgeqil;aavgavsgeqiln;avgavsgeqilna;v
gavsgeqilnai;gavsgeqilnaiv;avsgeqilnaivt;vsgeqilnaivta;sgeqilnaivtaa;ge
qilnaivtaag;eqilnaivtaagq;qilnaivtaagqa;ilnaivtaagqag;lnaivtaagqagq;nai
vtaagqagqa;aivtaagqagqag;ivtaagqagqagk;vtaagqagqagkk;taagqagqagkka;aagq
agqagkkad;agqagqagkkade;gqagqagkkadea;qagqagkkadeak;agqagkkadeakn;gqagk
kadeakna;qagkkadeaknai;agkkadeaknaie;gkkadeaknaiea;kkadeaknaieaa;kadeak
naieaai;adeaknaieaaig;deaknaieaaiga;eaknaieaaigaa;aknaieaaigaag;knaieaa
igaagd;naieaaigaagda;aieaaigaagdad;ieaaigaagdadf;eaaigaagdadfg;aaigaagd
adfgd;aigaagdadfgdd;igaagdadfgddi;gaagdadfgddik;aagdadfgddikk;agdadfgdd
ikkk;gdadfgddikkkn;dadfgddikkknd;adfgddikkkndq;dfgddikkkndqi;fgddikkknd
qia;gddikkkndqiaa;ddikkkndqiaaa;dikkkndqiaaal;ikkkndqiaaalv;kkkndqiaaal
vl;kkndqiaaalvlr;kndqiaaalvlrg;ndqiaaalvlrgv;dqiaaalvlrgva;qiaaalvlrgva
k;iaaalvlrgvakd;aaalvlrgvakdg;aalvlrgvakdgk;alvlrgvakdgkf;lvlrgvakdgkfa
;vlrgvakdgkfag;lrgvakdgkfaga;

14 mers:
mkkissaifivafl;kkissaifivafla;kissaifivaflal;issaifivaflali;ssaifivafla
lig;saifivaflaligc;aifivaflaligck;ifivaflaligckn;fivaflaligcknn;ivaflal
igcknnv;vaflaligcknnvg;aflaligcknnvgg;flaligcknnvggd;laligcknnvggdd;ali
gcknnvggddk;ligcknnvggddkk;igcknnvggddkkd;gcknnvggddkkdt;cknnvggddkkdta
;knnvggddkkdtaa;nnvggddkkdtaas;nvggddkkdtaasi;vggddkkdtaasif;ggddkkdtaa
sify;gddkkdtaasifyq;ddkkdtaasifyqs;dkkdtaasifyqsi;kkdtaasifyqsii;kdtaas
ifyqsiin;dtaasifyqsiinl;taasifyqsiinlg;aasifyqsiinlgn;asifyqsiinlgng;si
fyqsiinlgngf;ifyqsiinlgngfi;fyqsiinlgngfie;yqsiinlgngfiev;qsiinlgngfiev
f;siinlgngfievfn;iinlgngfievfna;inlgngfievfnaf;nlgngfievfnafs;lgngfievf
nafsg;gngfievfnafsgl;ngfievfnafsglv;gfievfnafsglva;fievfnafsglvad;ievfn
afsglvada;evfnafsglvadaf;vfnafsglvadafs;fnafsglvadafsk;nafsglvadafska;a
fsglvadafskad;fsglvadafskadp;sglvadafskadpk;glvadafskadpkk;lvadafskadpk
ks;vadafskadpkksd;adafskadpkksdv;dafskadpkksdvk;afskadpkksdvkt;fskadpkk

sdvkty;skadpkksdvktyf;kadpkksdvktyfd;adpkksdvktyfds;dpkksdvktyfdsi;pkks
dvktyfdsit;kksdvktyfdsitk;ksdvktyfdsitkt;sdvktyfdsitktl;dvktyfdsitktlk;
vktyfdsitktlkd;ktyfdsitktlkdt;tyfdsitktlkdtk;yfdsitktlkdtkt;fdsitktlkdt
ktk;dsitktlkdtktkl;sitktlkdtktkle;itktlkdtktkled;tktlkdtktkledi;ktlkdtk
tkledis;tlkdtktkledisk;lkdtktklediske;kdtktklediskek;dtktklediskekt;tkt
klediskektg;ktklediskektgg;tklediskektgge;klediskektggek;lediskektggekt
;ediskektggektp;diskektggektpa;iskektggektpav;skektggektpave;kektggektp
aveg;ektggektpavegi;ktggektpavegia;tggektpavegiae;ggektpavegiaev;gektpa
vegiaevv;ektpavegiaevvk;ktpavegiaevvkt;tpavegiaevvktv;pavegiaevvktvg;av
egiaevvktvge;vegiaevvktvgew;egiaevvktvgewl;giaevvktvgewld;iaevvktvgewld
g;aevvktvgewldgl;evvktvgewldgli;vvktvgewldglik;vktvgewldglika;ktvgewldg
likaa;tvgewldglikaae;vgewldglikaaeg;gewldglikaaegg;ewldglikaaeggg;wldgl
ikaaegggk;ldglikaaegggka;dglikaaegggkaa;glikaaegggkaad;likaaegggkaadg;i
kaaegggkaadgg;kaaegggkaadggg;aaegggkaadgggs;aegggkaadgggsd;egggkaadgggs
dk;gggkaadgggsdki;ggkaadgggsdkig;gkaadgggsdkign;kaadgggsdkignv;aadgggsd
kignva;adgggsdkignvaa;dgggsdkignvaag;gggsdkignvaagg;ggsdkignvaaggg;gsdk
ignvaaggga;sdkignvaagggag;dkignvaagggaga;kignvaagggagad;ignvaagggagadk;
gnvaagggagadke;nvaagggagadkes;vaagggagadkesv;aagggagadkesvn;agggagadkes
vng;gggagadkesvngi;ggagadkesvngia;gagadkesvngiag;agadkesvngiaga;gadkesv
ngiagai;adkesvngiagaik;dkesvngiagaikg;kesvngiagaikgi;esvngiagaikgiv;svn
giagaikgive;vngiagaikgivea;ngiagaikgiveaa;giagaikgiveaak;iagaikgiveaakk
;agaikgiveaakkv;gaikgiveaakkve;aikgiveaakkveg;ikgiveaakkvegv;kgiveaakkv
egvk;giveaakkvegvkf;iveaakkvegvkfa;veaakkvegvkfap;eaakkvegvkfapk;aakkve
gvkfapka;akkvegvkfapkaa;kkvegvkfapkaaa;kvegvkfapkaaad;vegvkfapkaaada;eg
vkfapkaaadaa;gvkfapkaaadaaa;vkfapkaaadaaaa;kfapkaaadaaaad;fapkaaadaaaad
g;apkaaadaaaadgn;pkaaadaaaadgnk;kaaadaaaadgnkk;aaadaaaadgnkka;aadaaaadg
nkkag;adaaaadgnkkagk;daaaadgnkkagkl;aaaadgnkkagklf;aaadgnkkagklfg;aadgn
kkagklfgt;adgnkkagklfgta;dgnkkagklfgtaa;gnkkagklfgtaag;nkkagklfgtaaga;k
kagklfgtaagad;kagklfgtaagada;agklfgtaagadag;gklfgtaagadagd;klfgtaagadag
dv;lfgtaagadagdvk;fgtaagadagdvkd;gtaagadagdvkda;taagadagdvkdaa;aagadagd
vkdaaa;agadagdvkdaaaa;gadagdvkdaaaav;adagdvkdaaaavg;dagdvkdaaaavga;agdv
kdaaaavgav;gdvkdaaaavgavs;dvkdaaaavgavsg;vkdaaaavgavsge;kdaaaavgavsgeq;
daaaavgavsgeqi;aaaavgavsgeqil;aaavgavsgeqiln;aavgavsgeqilna;avgavsgeqil
nai;vgavsgeqilnaiv;gavsgeqilnaivt;avsgeqilnaivta;vsgeqilnaivtaa;sgeqiln
aivtaag;geqilnaivtaagq;eqilnaivtaagqa;qilnaivtaagqag;ilnaivtaagqagq;lna
ivtaagqagqa;naivtaagqagqag;aivtaagqagqagk;ivtaagqagqagkk;vtaagqagqagkka
;taagqagqagkkad;aagqagqagkkade;agqagqagkkadea;gqagqagkkadeak;qagqagkkad
eakn;agqagkkadeakna;gqagkkadeaknai;qagkkadeaknaie;agkkadeaknaiea;gkkade
aknaieaa;kkadeaknaieaai;kadeaknaieaaig;adeaknaieaaiga;deaknaieaaigaa;ea
knaieaaigaag;aknaieaaigaagd;knaieaaigaagda;naieaaigaagdad;aieaaigaagdad
f;ieaaigaagdadfg;eaaigaagdadfgd;aaigaagdadfgdd;aigaagdadfgddi;igaagdadf
gddik;gaagdadfgddikk;aagdadfgddikkk;agdadfgddikkkn;gdadfgddikkknd;dadfg
ddikkkndq;adfgddikkkndqi;dfgddikkkndqia;fgddikkkndqiaa;gddikkkndqiaaa;d
dikkkndqiaaal;dikkkndqiaaalv;ikkkndqiaaalvl;kkkndqiaaalvlr;kkndqiaaalvl
rg;kndqiaaalvlrgv;ndqiaaalvlrgva;dqiaaalvlrgvak;qiaaalvlrgvakd;iaaalvlr
gvakdg;aaalvlrgvakdgk;aalvlrgvakdgkf;alvlrgvakdgkfa;lvlrgvakdgkfag;vlrg
vakdgkfaga;

15 mers:
mkkissaifivafla;kkissaifivaflal;kissaifivaflali;issaifivaflalig;ssaifiv
aflaligc;saifivaflaligck;aifivaflaligckn;ifivaflaligcknn;fivaflaligcknn
v;ivaflaligcknnvg;vaflaligcknnvgg;aflaligcknnvggd;flaligcknnvggdd;lalig
cknnvggddk;aligcknnvggddkk;ligcknnvggddkkd;igcknnvggddkkdt;gcknnvggddkk
dta;cknnvggddkkdtaa;knnvggddkkdtaas;nnvggddkkdtaasi;nvggddkkdtaasif;vgg
ddkkdtaasifyq;gddkkdtaasifyqs;gddkkdtaasifyqs;ddkkdtaasifyqsi;dkkdtaasif
yqsii;kkdtaasifyqsiin;kdtaasifyqsiinl;dtaasifyqsiinlg;taasifyqsiinlgn;a
asifyqsiinlgng;asifyqsiinlgngf;sifyqsiinlgngfi;ifyqsiinlgngfie;fyqsiinl

gngfiev;yqsiinlgngfievf;qsiinlgngfievfn;siinlgngfievfna;iinlgngfievfnaf
;inlgngfievfnafs;nlgngfievfnafsg;lgngfievfnafsgl;gngfievfnafsglv;ngfiev
fnafsglva;gfievfnafsglvad;fievfnafsglvada;ievfnafsglvadaf;evfnafsglvada
fs;vfnafsglvadafsk;fnafsglvadafska;nafsglvadafskad;afsglvadafskadp;fsgl
vadafskadpk;sglvadafskadpkk;glvadafskadpkks;lvadafskadpkksd;vadafskadpk
ksdv;adafskadpkksdvk;dafskadpkksdvkt;afskadpkksdvkty;fskadpkksdvktyf;sk
adpkksdvktyfd;kadpkksdvktyfds;adpkksdvktyfdsi;dpkksdvktyfdsit;pkksdvkty
fdsitk;kksdvktyfdsitkt;ksdvktyfdsitktl;sdvktyfdsitktlk;dvktyfdsitktlkd;
vktyfdsitktlkdt;ktyfdsitktlkdtk;tyfdsitktlkdtkt;yfdsitktlkdtktk;fdsitkt
lkdtktkl;dsitktlkdtktkle;sitktlkdtktkled;itktlkdtktkledi;tktlkdtktkledi
s;ktlkdtktkledisk;tlkdtktklediske;lkdtktklediskek;kdtktklediskekt;dtktk
lediskektg;tktklediskektgg;ktklediskektgge;tklediskektggek;klediskektgg
ekt;lediskektggektp;ediskektggektpa;diskektggektpav;iskektggektpave;ske
ktggektpaveg;kektggektpavegi;ektggektpavegia;ktggektpavegiae;tggektpave
giaev;ggektpavegiaevv;gektpavegiaevvk;ektpavegiaevvkt;ktpavegiaevvktv;t
pavegiaevvktvg;pavegiaevvktvge;avegiaevvktvgew;vegiaevvktvgewl;egiaevvk
tvgewld;giaevvktvgewldg;iaevvktvgewldgl;aevvktvgewldgli;evvktvgewldglik
;vvktvgewldglika;vktvgewldglikaa;ktvgewldglikaae;tvgewldglikaaeg;vgewld
glikaaegg;gewldglikaaeggg;ewldglikaaegggk;wldglikaaegggka;ldglikaaegggk
aa;dglikaaegggkaad;glikaaegggkaadg;likaaegggkaadgg;ikaaegggkaadggg;kaae
gggkaadgggs;aaegggkaadgggsd;aegggkaadgggsdk;egggkaadgggsdki;gggkaadgggs
dkig;ggkaadgggsdkign;gkaadgggsdkignv;kaadgggsdkignva;aadgggsdkignvaa;ad
gggsdkignvaag;dgggsdkignvaagg;gggsdkignvaaggg;ggsdkignvaaggga;gsdkignva
agggag;sdkignvaagggaga;dkignvaagggagad;kignvaagggagadk;ignvaagggagadke;
gnvaagggagadkes;nvaagggagadkesv;vaagggagadkesvn;aagggagadkesvng;agggaga
dkesvngi;gggagadkesvngia;ggagadkesvngiag;gagadkesvngiaga;agadkesvngiaga
i;gadkesvngiagaik;adkesvngiagaikg;dkesvngiagaikgi;kesvngiagaikgiv;esvng
iagaikgive;svngiagaikgivea;vngiagaikgiveaa;ngiagaikgiveaak;giagaikgivea
akk;iagaikgiveaakkv;agaikgiveaakkve;gaikgiveaakkveg;aikgiveaakkvegv;ikg
iveaakkvegvk;kgiveaakkvegvkf;giveaakkvegvkfa;iveaakkvegvkfap;veaakkvegv
kfapk;eaakkvegvkfapka;aakkvegvkfapkaa;akkvegvkfapkaaa;kkvegvkfapkaaad;k
vegvkfapkaaada;vegvkfapkaaadaa;egvkfapkaaadaaa;gvkfapkaaadaaaa;vkfapkaa
adaaaad;kfapkaaadaaaadg;fapkaaadaaaadgn;apkaaadaaaadgnk;pkaaadaaaadgnkk
;kaaadaaaadgnkka;aaadaaaadgnkkag;aadaaaadgnkkagk;adaaaadgnkkagkl;daaaad
gnkkagklf;aaaadgnkkagklfg;aaadgnkkagklfgt;aadgnkkagklfgta;adgnkkagklfgt
aa;dgnkkagklfgtaag;gnkkagklfgtaaga;nkkagklfgtaagad;kkagklfgtaagada;kagk
lfgtaagadag;agklfgtaagadagd;gklfgtaagadagdv;klfgtaagadagdvk;lfgtaagadag
dvkd;fgtaagadagdvkda;gtaagadagdvkdaa;taagadagdvkdaaa;aagadagdvkdaaaa;ag
adagdvkdaaaav;gadagdvkdaaaavg;adagdvkdaaaavga;dagdvkdaaaavgav;agdvkdaaa
avgavs;gdvkdaaaavgavsg;dvkdaaaavgavsge;vkdaaaavgavsgeq;kdaaaavgavsgeqi;
daaaavgavsgeqil;aaaavgavsgeqiln;aaavgavsgeqilna;aavgavsgeqilnai;avgavsg
eqilnaiv;vgavsgeqilnaivt;gavsgeqilnaivta;avsgeqilnaivtaa;vsgeqilnaivtaa
g;sgeqilnaivtaagq;geqilnaivtaagqa;eqilnaivtaagqag;qilnaivtaagqagq;ilnai
vtaagqagqa;lnaivtaagqagqag;naivtaagqagqagk;aivtaagqagqagkk;ivtaagqagqag
kka;vtaagqagqagkkad;taagqagqagkkade;aagqagqagkkadea;agqagqagkkadeak;gqa
gqagkkadeakn;qagqagkkadeakna;agqagkkadeaknai;gqagkkadeaknaie;qagkkadeak
naiea;agkkadeaknaieaa;gkkadeaknaieaai;kkadeaknaieaaig;kadeaknaieaaiga;a
deaknaieaaigaa;deaknaieaaigaag;eaknaieaaigaagd;aknaieaaigaagda;knaieaai
gaagdad;naieaaigaagdadf;aieaaigaagdadfg;ieaaigaagdadfgd;eaaigaagdadfgdd
;aaigaagdadfgddi;aigaagdadfgddik;igaagdadfgddikk;gaagdadfgddikkk;aagdad
fgddikkkn;agdadfgddikkknd;gdadfgddikkkndq;dadfgddikkkndqi;adfgddikkkndq
ia;dfgddikkkndqiaa;fgddikkkndqiaaa;gddikkkndqiaaal;ddikkkndqiaaalv;dikk
kndqiaaalvl;ikkkndqiaaalvlr;kkkndqiaaalvlrg;kkndqiaaalvlrgv;kndqiaaalvl
rgva;ndqiaaalvlrgvak;dqiaaalvlrgvakd;qiaaalvlrgvakdg;iaaalvlrgvakdgk;aa
alvlrgvakdgkf;aalvlrgvakdgkfa;alvlrgvakdgkfag;lvlrgvakdgkfaga;

16 mers:

mkkissaifivaflal;kkissaifivaflali;kissaifivaflalig;issaifivaflaligc;ssa
ifivaflaligck;saifivaflaligckn;aifivaflaligcknn;ifivaflaligcknnv;fivafl
aligcknnvg;ivaflaligcknnvgg;vaflaligcknnvggd;aflaligcknnvggdd;flaligckn
nvggddk;laligcknnvggddkk;aligcknnvggddkkd;ligcknnvggddkkdt;igcknnvggddk
kdta;gcknnvggddkkdtaa;cknnvggddkkdtaas;knnvggddkkdtaasi;nnvggddkkdtaasi
f;nvggddkkdtaasify;vggddkkdtaasifyq;ggddkkdtaasifyqs;gddkkdtaasifyqsi;d
dkkdtaasifyqsii;dkkdtaasifyqsiin;kkdtaasifyqsiinl;kdtaasifyqsiinlg;dtaa
sifyqsiinlgn;taasifyqsiinlgng;aasifyqsiinlgngf;asifyqsiinlgngfi;sifyqsi
inlgngfie;ifyqsiinlgngfiev;fyqsiinlgngfievf;yqsiinlgngfievfn;qsiinlgngf
ievfna;siinlgngfievfnaf;iinlgngfievfnafs;inlgngfievfnafsg;nlgngfievfnaf
sgl;lgngfievfnafsglv;gngfievfnafsglva;ngfievfnafsglvad;gfievfnafsglvada
;fievfnafsglvadaf;ievfnafsglvadafs;evfnafsglvadafsk;vfnafsglvadafska;fn
afsglvadafskad;nafsglvadafskadp;afsglvadafskadpk;fsglvadafskadpkk;sglva
dafskadpkks;glvadafskadpkksd;lvadafskadpkksdv;vadafskadpkksdvk;adafskad
pkksdvkt;dafskadpkksdvkty;afskadpkksdvktyf;fskadpkksdvktyfd;skadpkksdvk
tyfds;kadpkksdvktyfdsi;adpkksdvktyfdsit;dpkksdvktyfdsitk;pkksdvktyfdsit
kt;kksdvktyfdsitktl;ksdvktyfdsitktlk;sdvktyfdsitktlkd;dvktyfdsitktlkdt;
vktyfdsitktlkdtk;ktyfdsitktlkdtkt;tyfdsitktlkdtktk;yfdsitktlkdtktkl;fds
itktlkdtktkle;dsitktlkdtktkled;sitktlkdtktkledi;itktlkdtktkledis;tktlkd
tktkledisk;ktlkdtktklediske;tlkdtktklediskek;lkdtktklediskekt;kdtktkled
iskektg;dtktklediskektgg;tktklediskektgge;ktklediskektggek;tklediskektg
gekt;klediskektggektp;lediskektggektpa;ediskektggektpav;diskektggektpav
e;iskektggektpaveg;skektggektpavegi;kektggektpavegia;ektggektpavegiae;k
tggektpavegiaev;tggektpavegiaevv;ggektpavegiaevvk;gektpavegiaevvkt;ektp
avegiaevvktv;ktpavegiaevvktvg;tpavegiaevvktvge;pavegiaevvktvgew;avegiae
vvktvgewl;vegiaevvktvgewld;egiaevvktvgewldg;giaevvktvgewldgl;iaevvktvge
wldgli;aevvktvgewldglik;evvktvgewldglika;vvktvgewldglikaa;vktvgewldglik
aae;ktvgewldglikaaeg;tvgewldglikaaegg;vgewldglikaaeggg;gewldglikaaegggk
;ewldglikaaegggka;wldglikaaegggkaa;ldglikaaegggkaad;dglikaaegggkaadg;gl
ikaaegggkaadgg;likaaegggkaadggg;ikaaegggkaadgggs;kaaegggkaadgggsd;aaegg
gkaadgggsdk;aegggkaadgggsdki;egggkaadgggsdkig;gggkaadgggsdkign;ggkaadgg
gsdkignv;gkaadgggsdkignva;kaadgggsdkignvaa;aadgggsdkignvaag;adgggsdkign
vaagg;dgggsdkignvaaggg;gggsdkignvaaggga;ggsdkignvaagggag;gsdkignvaaggga
ga;sdkignvaagggagad;dkignvaagggagadk;kignvaagggagadke;ignvaagggagadkes;
gnvaagggagadkesv;nvaagggagadkesvn;vaagggagadkesvng;aagggagadkesvngi;agg
gagadkesvngia;gggagadkesvngiag;ggagadkesvngiaga;gagadkesvngiagai;agadke
svngiagaik;gadkesvngiagaikg;adkesvngiagaikgi;dkesvngiagaikgiv;kesvngiag
aikgive;esvngiagaikgivea;svngiagaikgiveaa;vngiagaikgiveaak;ngiagaikgive
aakk;giagaikgiveaakkv;iagaikgiveaakkve;agaikgiveaakkveg;gaikgiveaakkveg
v;aikgiveaakkvegvk;ikgiveaakkvegvkf;kgiveaakkvegvkfa;giveaakkvegvkfap;i
veaakkvegvkfapk;veaakkvegvkfapka;eaakkvegvkfapkaa;aakkvegvkfapkaaa;akkv
egvkfapkaaad;kkvegvkfapkaaada;kvegvkfapkaaadaa;vegvkfapkaaadaaa;egvkfap
kaaadaaaa;gvkfapkaaadaaaad;vkfapkaaadaaaadg;kfapkaaadaaaadgn;fapkaaadaa
aadgnk;apkaaadaaaadgnkk;pkaaadaaaadgnkka;kaaadaaaadgnkkag;aaaadaaaadgnkk
agk;aadaaaadgnkkagkl;adaaaadgnkkagklf;daaaadgnkkagklfg;aaaadgnkkagklfgt
;aaadgnkkagklfgta;aadgnkkagklfgtaa;adgnkkagklfgtaag;dgnkkagklfgtaaga;gn
kkagklfgtaagad;nkkagklfgtaagada;kkagklfgtaagadag;kagklfgtaagadagd;agklf
gtaagadagdv;gklfgtaagadagdvk;klfgtaagadagdvkd;lfgtaagadagdvkda;fgtaagad
agdvkdaa;gtaagadagdvkdaaa;taagadagdvkdaaaa;aagadagdvkdaaaav;agadagdvkda
aaavg;gadagdvkdaaaavga;adagdvkdaaaavgav;dagdvkdaaaavgavs;agdvkdaaaavgav
sg;gdvkdaaaavgavsge;dvkdaaaavgavsgeq;vkdaaaavgavsgeqi;kdaaaavgavsgeqil;
daaaavgavsgeqiln;aaaavgavsgeqilna;aaavgavsgeqilnai;aavgavsgeqilnaiv;avg
avsgeqilnaivt;vgavsgeqilnaivta;gavsgeqilnaivtaa;avsgeqilnaivtaag;vsgeqi
lnaivtaagq;sgeqilnaivtaagqa;geqilnaivtaagqag;eqilnaivtaagqagq;qilnaivta
agqagqa;ilnaivtaagqagqag;lnaivtaagqagqagk;naivtaagqagqagkk;aivtaagqagqa
gkka;ivtaagqagqagkkad;vtaagqagqagkkade;taagqagqagkkadea;aagqagqagkkadea
k;agqagqagkkadeakn;gqagqagkkadeakna;qagqagkkadeaknai;agqagkkadeaknaie;g

qagkkadeaknaiea;qagkkadeaknaieaaa;agkkadeaknaieaai;gkkadeaknaieaaig;kkad
eaknaieaaiga;kadeaknaieaaigaa;adeaknaieaaigaag;deaknaieaaigaagd;eaknaie
aaigaagda;aknaieaaigaagdad;knaieaaigaagdadf;naieaaigaagdadfg;aieaaigaag
dadfgd;ieaaigaagdadfgdd;eaaigaagdadfgddi;aaigaagdadfgddik;aigaagdadfgdd
ikk;igaagdadfgddikkk;gaagdadfgddikkkn;aagdadfgddikkknd;agdadfgddikkkndq
;gdadfgddikkkndqi;dadfgddikkkndqia;adfgddikkkndqiaa;dfgddikkkndqiaaa;fg
ddikkkndqiaaal;gddikkkndqiaaalv;ddikkkndqiaaalvl;dikkkndqiaaalvlr;ikkkn
dqiaaalvlrg;kkkndqiaaalvlrgv;kkndqiaaalvlrgva;kndqiaaalvlrgvak;ndqiaaal
vlrgvakd;dqiaaalvlrgvakdg;qiaaalvlrgvakdgk;iaaalvlrgvakdgkf;aaalvlrgvak
dgkfa;aalvlrgvakdgkfag;alvlrgvakdgkfaga;


## <CAF34027 outer surface protein VlsE;Protein;Borrelia afzelii PKo>

8 mers:
mkkissai;kkissaif;kissaifl;issaiflt;ssaiflta;saifltal;aifltall;ifltallv
;fltallvf;ltallvfi;tallvfin;allvfinc;llvfinck;lvfinckn;vfincknn;fincknn
a;incknnav;ncknnavg;cknnavgk;knnavgkg;nnavgkgn;navgkgnd;avgkgndd;vgkgnd
dk;gkgnddkd;kgnddkds;gnddkdsv;nddkdsvk;ddkdsvkt;dkdsvktf;kdsvktfy;dsvkt
fye;svktfyes;vktfyesi;ktfyesii;tfyesiin;fyesiinl;yesiinlg;esiinlgn;siin
lgng;iinlgngf;inlgngfi;nlgngfid;lgngfidv;gngfidvf;ngfidvfn;gfidvfna;fid
vfnaf;idvfnafs;dvfnafsg;vfnafsgl;fnafsglv;nafsglva;afsglvad;fsglvadt;sg
lvadtf;glvadtff;lvadtffk;vadtffks;adtffksd;dtffksdp;tffksdpk;ffksdpkk;f
ksdpkks;ksdpkksd;sdpkksdv;dpkksdvk;pkksdvkt;kksdvkty;ksdvktyf;sdvktyfe;
dvktyfes;vktyfesi;ktyfesis;tyfesiss;yfesisst;fesisstl;esisstlk;sisstlka
;isstlkat;sstlkatk;stlkatkg;tlkatkgk;lkatkgkl;katkgkld;atkgklde;tkgklde
l;kgkldelv;gkldelvs;kldelvsa;ldelvsak;delvsakk;elvsakkg;lvsakkge;vsakkg
eg;sakkgegg;akkgeggs;kkgeggsv;kgeggsvk;geggsvka;eggsvkas;ggsvkasv;gsvka
sve;svkasves;vkasvesa;kasvesav;asvesavd;svesavde;vesavdev;esavdevs;savd
evsk;avdevskw;vdevskwl;devskwle;evskwlee;vskwleem;skwleemi;kwleemik;wle
emika;leemikaa;eemikaae;emikaaee;mikaaeea;ikaaeeaa;kaaeeaak;aaeeaakv;ae
eeaakvg;eeaakvgg;eaakvggt;aakvggtg;akvggtgg;kvggtggd;vggtggdg;ggtggdgk;g
tggdgki;tggdgkig;ggdgkigd;gdgkigds;dgkigdsa;gkigdsaa;kigdsaan;igdsaanh;
gdsaanhg;dsaanhga;saanhgak;aanhgaka;anhgakad;nhgakadk;hgakadkd;gakadkds
;akadkdsv;kadkdsvk;adkdsvkg;dkdsvkgi;kdsvkgia;dsvkgiak;svkgiakg;vkgiakg
i;kgiakgik;giakgikg;iakgikgi;akgikgiv;kgikgivd;gikgivda;ikgivdaa;kgivda
ag;givdaagk;ivdaagka;vdaagkal;daagkalg;aagkalge;agkalgek;gkalgekg;kalge
kga;algekgal;lgekgalk;gekgalkd;ekgalkdv;kgalkdvk;galkdvka;alkdvkaa;lkdv
kaaa;kdvkaaad;dvkaaadd;vkaaadde;kaaaddea;aaaddean;aaddeana;addeanad;dde
anada;deanadag;eanadagk;anadagkl;nadagklf;adagklfa;dagklfag;agklfagn;gk
lfagna;klfagnan;lfagnana;fagnanaa;agnanaav;gnanaavg;nanaavga;anaavgaa;n
aavgaaa;aavgaaad;avgaaadi;vgaaadia;gaaadiak;aaadiaka;aadiakaa;adiakaag;
diakaaga;iakaagav;akaagavt;kaagavta;aagavtav;agavtavs;gavtavsg;avtavsge
;vtavsgeq;tavsgeqi;avsgeqil;vsgeqilk;sgeqilka;geqilkai;eqilkaiv;qilkaiv
e;ilkaivea;lkaiveaa;kaiveaag;aiveaagd;iveaagdp;veaagdpa;eaagdpan;aagdpa
nq;agdpanqa;gdpanqag;dpanqagk;panqagkk;anqagkka;nqagkkae;qagkkaee;agkka
eea;gkkaeeak;kkaeeakn;kaeeaknp;aeeaknpi;eeaknpia;eaknpiaa;aknpiaaa;knpi
aaai;npiaaaig;piaaaigt;iaaaigtd;aaaigtdd;aaigtddd;aigtdddn;igtdddng;gtd
ddnga;tdddngaa;dddngaaf;ddngaafk;dngaafkd;ngaafkde;gaafkdem;aafkdemk;af
kdemkk;fkdemkks;kdemkksd;demkksdk;emkksdki;mkksdkia;kksdkiaa;ksdkiaaa;s
dkiaaai;dkiaaaiv;kiaaaivl;iaaaivlr;aaaivlrg;aaivlrgv;aivlrgva;ivlrgvak;
vlrgvakd;lrgvakdg;rgvakdgk;gvakdgkf;vakdgkfa;akdgkfav;kdgkfavk;

9 mers:
mkkissaif;kkissaifl;kissaiflt;issaiflta;ssaifltal;saifltall;aifltallv;i
fltallvf;fltallvfi;ltallvfin;tallvfinc;allvfinck;llvfinckn;lvfincknn;vf

incknnna;fincknnav;incknnavg;nckknnavgk;cknnavgkg;knnavgkgn;nnavgkgnd;nav
gkgndd;avgkgnddk;vgkgnddkd;gkgnddkds;kgnddkdsv;gnddkdsvk;nddkdsvkt;ddkd
svktf;dkdsvktfy;kdsvktfye;dsvktfyes;svktfyesi;vktfyesii;ktfyesiin;tfyes
iinl;fyesiinlg;yesiinlgn;esiinlgng;siinlgngf;iinlgngfi;inlgngfid;nlgngf
idv;lgngfidvf;gngfidvfn;ngfidvfna;gfidvfnaf;fidvfnafs;idvfnafsg;dvfnafs
gl;vfnafsglv;fnafsglva;nafsglvad;afsglvadt;fsglvadtf;sglvadtff;glvadtff
k;lvadtffks;vadtffksd;adtffksdp;dtffksdpk;tffksdpkk;ffksdpkks;fksdpkksd
;ksdpkksdv;sdpkksdvk;dpkksdvkt;pkksdvkty;kksdvktyf;ksdvktyfe;sdvktyfes;
dvktyfesi;vktyfesis;ktyfesiss;tyfesisst;yfesisstl;fesisstlk;esisstlka;s
isstlkat;isstlkatk;sstlkatkg;stlkatkgk;tlkatkgkl;lkatkgkld;katkgklde;at
kgkldel;tkgkldelv;kgkldelvs;gkldelvsa;kldelvsak;ldelvsakk;delvsakkg;elv
sakkge;lvsakkgeg;vsakkgegg;sakkgeggs;akkgeggsv;kkgeggsvk;kgeggsvka;gegg
svkas;eggsvkasv;ggsvkasve;gsvkasves;svkasvesa;vkasvesav;kasvesavd;asves
avde;svesavdev;vesavdevs;esavdevsk;savdevskw;avdevskwl;vdevskwle;devskw
lee;evskwleem;vskwleemi;skwleemik;kwleemika;wleemikaa;leemikaae;eemikaa
ee;emikaaeea;mikaaeeaa;ikaaeeaak;kaaeeaakv;aaeeaakvg;aeeaakvgg;eeaakvgg
t;eaakvggtg;aakvggtgg;akvggtggd;kvggtggdg;vggtggdgk;ggtggdgki;gtggdgkig
;tggdgkigd;ggdgkigds;gdgkigdsa;dgkigdsaa;gkigdsaan;kigdsaanh;igdsaanhg;
gdsaanhga;dsaanhgak;saanhgaka;aanhgakad;anhgakadk;nhgakadkd;hgakadkds;g
akadkdsv;akadkdsvk;kadkdsvkg;adkdsvkgi;dkdsvkgia;kdsvkgiak;dsvkgiakg;sv
kgiakgi;vkgiakgik;kgiakgikg;giakgikgi;iakgikgiv;akgikgivd;kgikgivda;gik
givdaa;ikgivdaag;kgivdaagk;givdaagka;ivdaagkal;vdaagkalg;daagkalge;aagk
algek;agkalgekg;gkalgekga;kalgekgal;algekgalk;lgekgalkd;gekgalkdv;ekgal
kdvk;kgalkdvka;galkdvkaa;alkdvkaaa;lkdvkaaad;kdvkaaadd;dvkaaadde;vkaaad
dea;kaaaddean;aaaddeana;aaddeanad;addeanada;ddeanadag;deanadagk;eanadag
kl;anadagklf;nadagklfa;adagklfag;dagklfagn;agklfagna;gklfagnan;klfagnan
a;lfagnanaa;fagnanaav;agnanaavg;gnanaavga;nanaavgaa;anaavgaaa;naavgaaad
;aavgaaadi;avgaaadia;vgaaadiak;gaaadiaka;aaadiakaa;aadiakaag;adiakaaga;
diakaagav;iakaagavt;akaagavta;kaagavtav;aagavtavs;agavtavsg;gavtavsge;a
vtavsgeq;vtavsgeqi;tavsgeqil;avsgeqilk;vsgeqilka;sgeqilkai;geqilkaiv;eq
ilkaive;qilkaivea;ilkaiveaa;lkaiveaag;kaiveaagd;aiveaagdp;iveaagdpa;vea
agdpan;eaagdpanq;aagdpanqa;agdpanqag;gdpanqagk;dpanqagkk;panqagkka;anqa
gkkae;nqagkkaee;qagkkaeea;agkkaeeak;gkkaeeakn;kkaeeaknp;kaeeaknpi;aeeak
npia;eeaknpiaa;eaknpiaaa;aknpiaaai;knpiaaaig;npiaaaigt;piaaaigtd;iaaaig
tdd;aaaigtddd;aaigtdddn;aigtdddng;igtdddnga;gtdddngaa;tdddngaaf;dddngaa
fk;ddngaafkd;dngaafkde;ngaafkdem;gaafkdemk;aafkdemkk;afkdemkks;fkdemkks
d;kdemkksdk;demkksdki;emkksdkia;mkksdkiaa;kksdkiaaa;ksdkiaaai;sdkiaaaiv
;dkiaaaivl;kiaaaivlr;iaaaivlrg;aaaivlrgv;aaivlrgva;aivlrgvak;ivlrgvakd;
vlrgvakdg;lrgvakdgk;rgvakdgkf;gvakdgkfa;vakdgkfav;akdgkfavk;

10 mers:
mkkissaifl;kkissaiflt;kissaiflta;issaifltal;ssaifltall;saifltallv;aiflt
allvf;ifltallvfi;fltallvfin;ltallvfinc;tallvfinck;allvfinckn;llvfincknn
;lvfincknna;vfincknnav;fincknnavg;incknnavgk;ncknnavgkg;cknnavgkgn;knna
vgkgnd;nnavgkgndd;navgkgnddk;avgkgnddkd;vgkgnddkds;gkgnddkdsv;kgnddkdsv
k;gnddkdsvkt;nddkdsvktf;ddkdsvktfy;dkdsvktfye;kdsvktfyes;dsvktfyesi;svk
tfyesii;vktfyesiin;ktfyesiinl;tfyesiinlg;fyesiinlgn;yesiinlgng;esiinlgn
gf;siinlgngfi;iinlgngfid;inlgngfidv;nlgngfidvf;lgngfidvfn;gngfidvfna;ng
fidvfnaf;gfidvfnafs;fidvfnafsg;idvfnafsgl;dvfnafsglv;vfnafsglva;fnafsgl
vad;nafsglvadt;afsglvadtf;fsglvadtff;sglvadtffk;glvadtffks;lvadtffksd;v
adtffksdp;adtffksdpk;dtffksdpkk;tffksdpkks;ffksdpkksd;fksdpkksdv;ksdpkk
sdvk;sdpkksdvkt;dpkksdvkty;pkksdvktyf;kksdvktyfe;ksdvktyfes;sdvktyfesi;
dvktyfesis;vktyfesiss;ktyfesisst;tyfesisstl;yfesisstlk;fesisstlka;esiss
tlkat;sisstlkatk;isstlkatkg;sstlkatkgk;stlkatkgkl;tlkatkgkld;lkatkgklde
;katkgkldel;atkgkldelv;tkgkldelvs;kgkldelvsa;gkldelvsak;kldelvsakk;ldel
vsakkg;delvsakkge;elvsakkgeg;lvsakkgegg;vsakkgeggs;sakkgeggsv;akkgeggsv
k;kkgeggsvka;kgeggsvkas;geggsvkasv;eggsvkasve;ggsvkasves;gsvkasvesa;svk

asvesav;vkasvesavd;kasvesavde;asvesavdev;svesavdevs;vesavdevsk;esavdevs
kw;savdevskwl;avdevskwle;vdevskwlee;devskwleem;evskwleemi;vskwleemik;sk
wleemika;kwleemikaa;wleemikaae;leemikaaee;eemikaaeea;emikaaeeaa;mikaaee
aak;ikaaeeaakv;kaaeeaakvg;aaeeaakvgg;aeeaakvggt;eeaakvggtg;eaakvggtgg;a
akvggtggd;akvggtggdg;kvggtggdgk;vggtggdgki;ggtggdgkig;gtggdgkigd;tggdgk
igds;ggdgkigdsa;gdgkigdsaa;dgkigdsaan;gkigdsaanh;kigdsaanhg;igdsaanhga;
gdsaanhgak;dsaanhgaka;saanhgakad;aanhgakadk;anhgakadkd;nhgakadkds;hgaka
dkdsv;gakadkdsvk;akadkdsvkg;kadkdsvkgi;adkdsvkgia;dkdsvkgiak;kdsvkgiakg
;dsvkgiakgi;svkgiakgik;vkgiakgikg;kgiakgikgi;giakgikgiv;iakgikgivd;akgi
kgivda;kgikgivdaa;gikgivdaag;ikgivdaagk;kgivdaagka;givdaagkal;ivdaagkal
g;vdaagkalge;daagkalgek;aagkalgekg;agkalgekga;gkalgekgal;kalgekgalk;alg
ekgalkd;lgekgalkdv;gekgalkdvk;ekgalkdvka;kgalkdvkaa;galkdvkaaa;alkdvkaa
ad;lkdvkaaadd;kdvkaaadde;dvkaaaddea;vkaaaddean;kaaaddeana;aaaddeanad;aa
ddeanada;addeanadag;ddeanadagk;deanadagkl;eanadagklf;anadagklfa;nadagkl
fag;adagklfagn;dagklfagna;agklfagnan;gklfagnana;klfagnanaa;lfagnanaav;f
agnanaavg;agnanaavga;gnanaavgaa;nanaavgaaa;anaavgaaad;naavgaaadi;aavgaa
adia;avgaaadiak;vgaaadiaka;gaaadiakaa;aaadiakaag;aadiakaaga;adiakaagav;
diakaagavt;iakaagavta;akaagavtav;kaagavtavs;aagavtavsg;agavtavsge;gavta
vsgeq;avtavsgeqi;vtavsgeqil;tavsgeqilk;avsgeqilka;vsgeqilkai;sgeqilkaiv
;geqilkaive;eqilkaivea;qilkaiveaa;ilkaiveaag;lkaiveaagd;kaiveaagdp;aive
aagdpa;iveaagdpan;veaagdpanq;eaagdpanqa;aagdpanqag;agdpanqagk;gdpanqagk
k;dpanqagkka;panqagkkae;anqagkkaee;nqagkkaeea;qagkkaeeak;agkkaeeakn;gkk
aeeaknp;kkaeeaknpi;kaeeaknpia;aeeaknpiaa;eeaknpiaaa;eaknpiaaai;aknpiaaa
ig;knpiaaaigt;npiaaaigtd;piaaaigtdd;iaaaigtddd;aaaigtdddn;aaigtdddng;ai
gtdddnga;igtdddngaa;gtdddngaaf;tdddngaafk;dddngaafkd;ddngaafkde;dngaafk
dem;ngaafkdemk;gaafkdemkk;aafkdemkks;afkdemkksd;fkdemkksdk;kdemkksdki;d
emkksdkia;emkksdkiaa;mkksdkiaaa;kksdkiaaai;ksdkiaaaiv;sdkiaaaivl;dkiaaa
ivlr;kiaaaivlrg;iaaaivlrgv;aaaivlrgva;aaivlrgvak;aivlrgvakd;ivlrgvakdg;
vlrgvakdgk;lrgvakdgkf;rgvakdgkfa;gvakdgkfav;vakdgkfavk;

11 mers:
mkkissaiflt;kkissaiflta;kissaifltal;issaifltall;ssaifltallv;saifltallvf
;aifltallvfi;ifltallvfin;fltallvfinc;ltallvfinck;tallvfinckn;allvfinckn
n;llvfincknna;lvfincknnav;vfincknnavg;fincknnavgk;incknnavgkg;ncknnavgk
gn;cknnavgkgnd;knnavgkgndd;nnavgkgnddk;navgkgnddkd;avgkgnddkds;vgkgnddk
dsv;gkgnddkdsvk;kgnddkdsvkt;gnddkdsvktf;nddkdsvktfy;ddkdsvktfye;dkdsvkt
fyes;kdsvktfyesi;dsvktfyesii;svktfyesiin;vktfyesiinl;ktfyesiinlg;tfyesi
inlgn;fyesiinlgng;yesiinlgngf;esiinlgngfi;siinlgngfid;iinlgngfidv;inlgn
gfidvf;nlgngfidvfn;lgngfidvfna;gngfidvfnaf;ngfidvfnafs;gfidvfnafsg;fidv
fnafsgl;idvfnafsglv;dvfnafsglva;vfnafsglvad;fnafsglvadt;nafsglvadtf;afs
glvadtff;fsglvadtffk;sglvadtffks;glvadtffksd;lvadtffksdp;vadtffksdpk;ad
tffksdpkk;dtffksdpkks;tffksdpkksd;ffksdpkksdv;fksdpkksdvk;ksdpkksdvkt;s
dpkksdvkty;dpkksdvktyf;pkksdvktyfe;kksdvktyfes;ksdvktyfesi;sdvktyfesis;
dvktyfesiss;vktyfesisst;ktyfesisstl;tyfesisstlk;yfesisstlka;fesisstlkat
;esisstlkatk;sisstlkatkg;isstlkatkgk;sstlkatkgkl;stlkatkgkld;tlkatkgkld
e;lkatkgkldel;katkgkldelv;atkgkldelvs;tkgkldelvsa;kgkldelvsak;gkldelvsa
kk;kldelvsakkg;ldelvsakkge;delvsakkgeg;elvsakkgegg;lvsakkgeggs;vsakkgeg
gsv;sakkgeggsvk;akkgeggsvka;kkgeggsvkas;kgeggsvkasv;geggsvkasve;eggsvka
sves;ggsvkasvesa;gsvkasvesav;svkasvesavd;vkasvesavde;kasvesavdev;asvesa
vdevs;svesavdevsk;vesavdevskw;esavdevskwl;savdevskwle;avdevskwlee;vdevs
kwleem;devskwleemi;evskwleemik;vskwleemika;skwleemikaa;kwleemikaae;wlee
mikaaee;leemikaaeea;eemikaaeeaa;emikaaeeaak;mikaaeeaakv;ikaaeeaakvg;kaa
eeaakvgg;aaeeaakvggt;aeeaakvggtg;eeaakvggtgg;eaakvggtggd;aakvggtggdg;ak
vggtggdgk;kvggtggdgki;vggtggdgkig;ggtggdgkigd;gtggdgkigds;tggdgkigdsa;g
gdgkigdsaa;gdgkigdsaan;dgkigdsaanh;gkigdsaanhg;kigdsaanhga;igdsaanhgak;
gdsaanhgaka;dsaanhgakad;saanhgakadk;aanhgakadkd;anhgakadkds;nhgakadkdsv
;hgakadkdsvk;gakadkdsvkg;akadkdsvkgi;kadkdsvkgia;adkdsvkgiak;dkdsvkgiak

g;kdsvkgiakgi;dsvkgiakgik;svkgiakgikg;vkgiakgikgi;kgiakgikgiv;giakgikgi
vd;iakgikgivda;akgikgivdaa;kgikgivdaag;gikgivdaagk;ikgivdaagka;kgivdaag
kal;givdaagkalg;ivdaagkalge;vdaagkalgek;daagkalgekg;aagkalgekga;agkalge
kgal;gkalgekgalk;kalgekgalkd;algekgalkdv;lgekgalkdvk;gekgalkdvka;ekgalk
dvkaa;kgalkdvkaaa;galkdvkaaad;alkdvkaaadd;lkdvkaaadde;kdvkaaaddea;dvkaa
addean;vkaaaddeana;kaaaddeanad;aaaddeanada;aaddeanadag;addeanadagk;ddea
nadagkl;deanadagklf;eanadagklfa;anadagklfag;nadagklfagn;adagklfagna;dag
klfagnan;agklfagnana;gklfagnanaa;klfagnanaav;lfagnanaavg;fagnanaavga;ag
nanaavgaa;gnanaavgaaa;nanaavgaaad;anaavgaaadi;naavgaaadia;aavgaaadiak;a
vgaaadiaka;vgaaadiakaa;gaaadiakaag;aaadiakaaga;aadiakaagav;adiakaagavt;
diakaagavta;iakaagavtav;akaagavtavs;kaagavtavsg;aagavtavsge;agavtavsgeq
;gavtavsgeqi;avtavsgeqil;vtavsgeqilk;tavsgeqilka;avsgeqilkai;vsgeqilkai
v;sgeqilkaive;geqilkaivea;eqilkaiveaa;qilkaiveaag;ilkaiveaagd;lkaiveaag
dp;kaiveaagdpa;aiveaagdpan;iveaagdpanq;veaagdpanqa;eaagdpanqag;aagdpanq
agk;agdpanqagkk;gdpanqagkka;dpanqagkkae;panqagkkaee;anqagkkaeea;nqagkka
eeak;qagkkaeeakn;agkkaeeaknp;gkkaeeaknpi;kkaeeaknpia;kaeeaknpiaa;aeeakn
piaaa;eeaknpiaaai;eaknpiaaaig;aknpiaaaigt;knpiaaaigtd;npiaaaigtdd;piaaa
igtddd;iaaaigtdddn;aaaigtdddng;aaigtdddnga;aigtdddngaa;igtdddngaaf;gtdd
dngaafk;tdddngaafkd;dddngaafkde;ddngaafkdem;dngaafkdemk;ngaafkdemkk;gaa
fkdemkks;aafkdemkksd;afkdemkksdk;fkdemkksdki;kdemkksdkia;demkksdkiaa;em
kksdkiaaa;mkksdkiaaai;kksdkiaaaiv;ksdkiaaaivl;sdkiaaaivlr;dkiaaaivlrg;k
iaaaivlrgv;iaaaivlrgva;aaaivlrgvak;aaivlrgvakd;aivlrgvakdg;ivlrgvakdgk;
vlrgvakdgkf;lrgvakdgkfa;rgvakdgkfav;gvakdgkfavk;

13 mers:
mkkissaifltal;kkissaifltall;kissaifltallv;issaifltallvf;ssaifltallvfi;s
aifltallvfin;aifltallvfinc;ifltallvfinck;fltallvfinckn;ltallvfincknn;ta
llvfincknna;allvfincknnav;llvfincknnavg;lvfincknnavgk;vfincknnavgkg;fin
cknnavgkgn;incknnavgkgnd;ncknnavgkgndd;cknnavgkgnddk;knnavgkgnddkd;nnav
gkgnddkds;navgkgnddkdsv;avgkgnddkdsvk;vgkgnddkdsvkt;gkgnddkdsvktf;kgndd
kdsvktfy;gnddkdsvktfye;nddkdsvktfyes;ddkdsvktfyesi;dkdsvktfyesii;kdsvkt
fyesiin;dsvktfyesiinl;svktfyesiinlg;vktfyesiinlgn;ktfyesiinlgng;tfyesii
nlgngf;fyesiinlgngfi;yesiinlgngfid;esiinlgngfidv;siinlgngfidvf;iinlgngf
idvfn;inlgngfidvfna;nlgngfidvfnaf;lgngfidvfnafs;gngfidvfnafsg;ngfidvfna
fsgl;gfidvfnafsglv;fidvfnafsglva;idvfnafsglvad;dvfnafsglvadt;vfnafsglva
dtf;fnafsglvadtff;nafsglvadtffk;afsglvadtffks;fsglvadtffksd;sglvadtffks
dp;glvadtffksdpk;lvadtffksdpkk;vadtffksdpkks;adtffksdpkksd;dtffksdpkksd
v;tffksdpkksdvk;ffksdpkksdvkt;fksdpkksdvkty;ksdpkksdvktyf;sdpkksdvktyfe
;dpkksdvktyfes;pkksdvktyfesi;kksdvktyfesis;ksdvktyfesiss;sdvktyfesisst;
dvktyfesisstl;vktyfesisstlk;ktyfesisstlka;tyfesisstlkat;yfesisstlkatk;f
esisstlkatkg;esisstlkatkgk;sisstlkatkgkl;isstlkatkgkld;sstlkatkgklde;st
lkatkgkldel;tlkatkgkldelv;lkatkgkldelvs;katkgkldelvsa;atkgkldelvsak;tkg
kldelvsakk;kgkldelvsakkg;gkldelvsakkge;kldelvsakkgeg;ldelvsakkgegg;delv
sakkgeggs;elvsakkgeggsv;lvsakkgeggsvk;vsakkgeggsvka;sakkgeggsvkas;akkge
ggsvkasv;kkgeggsvkasve;kgeggsvkasves;geggsvkasvesa;eggsvkasvesav;ggsvka
svesavd;gsvkasvesavde;svkasvesavdev;vkasvesavdevs;kasvesavdevsk;asvesav
devskw;svesavdevskwl;vesavdevskwle;esavdevskwlee;savdevskwleem;avdevskw
leemi;vdevskwleemik;devskwleemika;evskwleemikaa;vskwleemikaae;skwleemik
aaee;kwleemikaaeea;wleemikaaeeaa;leemikaaeeaak;eemikaaeeaakv;emikaaeeaa
kvg;mikaaeeaakvgg;ikaaeeaakvggt;kaaeeaakvggtg;aaeeaakvggtgg;aeeaakvggtg
gd;eeaakvggtggdg;eaakvggtggdgk;aakvggtggdgki;akvggtggdgkig;kvggtggdgkig
d;vggtggdgkigds;ggtggdgkigdsa;gtggdgkigdsaa;tggdgkigdsaan;ggdgkigdsaanh
;gdgkigdsaanhg;dgkigdsaanhga;gkigdsaanhgak;kigdsaanhgaka;igdsaanhgakad;
gdsaanhgakadk;dsaanhgakadkd;saanhgakadkds;aanhgakadkdsv;anhgakadkdsvk;n
hgakadkdsvkg;hgakadkdsvkgi;gakadkdsvkgia;akadkdsvkgiak;kadkdsvkgiakg;ad
kdsvkgiakgi;dkdsvkgiakgik;kdsvkgiakgikg;dsvkgiakgikgi;svkgiakgikgiv;vkg
iakgikgivd;kgiakgikgivda;giakgikgivdaa;iakgikgivdaag;akgikgivdaagk;kgik

givdaagka;gikgivdaagkal;ikgivdaagkalg;kgivdaagkalge;givdaagkalgek;ivdaa
gkalgekg;vdaagkalgekga;daagkalgekgal;aagkalgekgalk;agkalgekgalkd;gkalge
kgalkdv;kalgekgalkdvk;algekgalkdvka;lgekgalkdvkaa;gekgalkdvkaaa;ekgalkd
vkaaad;kgalkdvkaaadd;galkdvkaaadde;alkdvkaaaddea;lkdvkaaaddean;kdvkaaad
deana;dvkaaaddeanad;vkaaaddeanada;kaaaddeanadag;aaaddeanadagk;aaddeanad
agkl;addeanadagklf;ddeanadagklfa;deanadagklfag;eanadagklfagn;anadagklfa
gna;nadagklfagnan;adagklfagnana;dagklfagnanaa;agklfagnanaav;gklfagnanaa
vg;klfagnanaavga;lfagnanaavgaa;fagnanaavgaaa;agnanaavgaaad;gnanaavgaaad
i;nanaavgaaadia;anaavgaaadiak;naavgaaadiaka;aavgaaadiakaa;avgaaadiakaag
;vgaaadiakaaga;gaaadiakaagav;aaadiakaagavt;aadiakaagavta;adiakaagavtav;
diakaagavtavs;iakaagavtavsg;akaagavtavsge;kaagavtavsgeq;aagavtavsgeqi;a
gavtavsgeqil;gavtavsgeqilk;avtavsgeqilka;vtavsgeqilkai;tavsgeqilkaiv;av
sgeqilkaive;vsgeqilkaivea;sgeqilkaiveaa;geqilkaiveaag;eqilkaiveaagd;qil
kaiveaagdp;ilkaiveaagdpa;lkaiveaagdpan;kaiveaagdpanq;aiveaagdpanqa;ivea
agdpanqag;veaagdpanqagk;eaagdpanqagkk;aagdpanqagkka;agdpanqagkkae;gdpan
qagkkaee;dpanqagkkaeea;panqagkkaeeak;anqagkkaeeakn;nqagkkaeeaknp;qagkka
eeaknpi;agkkaeeaknpia;gkkaeeaknpiaa;kkaeeaknpiaaa;kaeeaknpiaaai;aeeaknp
iaaaig;eeaknpiaaaigt;eaknpiaaaigtd;aknpiaaaigtdd;knpiaaaigtddd;npiaaaig
tdddn;piaaaigtdddng;iaaaigtdddnga;aaaigtdddngaa;aaigtdddngaaf;aigtdddng
aafk;igtdddngaafkd;gtdddngaafkde;tdddngaafkdem;dddngaafkdemk;ddngaafkde
mkk;dngaafkdemkks;ngaafkdemkksd;gaafkdemkksdk;aafkdemkksdki;afkdemkksdk
ia;fkdemkksdkiaa;kdemkksdkiaaa;demkksdkiaaai;emkksdkiaaaiv;mkksdkiaaaiv
l;kksdkiaaaivlr;ksdkiaaaivlrg;sdkiaaaivlrgv;dkiaaaivlrgva;kiaaaivlrgvak
;iaaaivlrgvakd;aaaivlrgvakdg;aaivlrgvakdgk;aivlrgvakdgkf;ivlrgvakdgkfa;
vlrgvakdgkfav;lrgvakdgkfavk;

14 mers:
mkkissaifltall;kkissaifltallv;kissaifltallvf;issaifltallvfi;ssaifltallv
fin;saifltallvfinc;aifltallvfinck;ifltallvfinckn;fltallvfincknn;ltallvf
incknna;tallvfincknnav;allvfincknnavg;llvfincknnavgk;lvfincknnavgkg;vfi
ncknnavgkgn;fincknnavgkgnd;incknnavgkgndd;ncknnavgkgnddk;cknnavgkgnddkd
;knnavgkgnddkds;nnavgkgnddkdsv;navgkgnddkdsvk;avgkgnddkdsvkt;vgkgnddkds
vktf;gkgnddkdsvktfy;kgnddkdsvktfye;gnddkdsvktfyes;nddkdsvktfyesi;ddkdsv
ktfyesii;dkdsvktfyesiin;kdsvktfyesiinl;dsvktfyesiinlg;svktfyesiinlgn;vk
tfyesiinlgng;ktfyesiinlgngf;tfyesiinlgngfi;fyesiinlgngfid;yesiinlgngfid
v;esiinlgngfidvf;siinlgngfidvfn;iinlgngfidvfna;inlgngfidvfnaf;nlgngfidv
fnafs;lgngfidvfnafsg;gngfidvfnafsgl;ngfidvfnafsglv;gfidvfnafsglva;fidvf
nafsglvad;idvfnafsglvadt;dvfnafsglvadtf;vfnafsglvadtff;fnafsglvadtffk;n
afsglvadtffks;afsglvadtffksd;fsglvadtffksdp;sglvadtffksdpk;glvadtffksdp
kk;lvadtffksdpkks;vadtffksdpkksd;adtffksdpkksdv;dtffksdpkksdvk;tffksdpk
ksdvkt;ffksdpkksdvkty;fksdpkksdvktyf;ksdpkksdvktyfe;sdpkksdvktyfes;dpkk
sdvktyfesi;pkksdvktyfesis;kksdvktyfesiss;ksdvktyfesisst;sdvktyfesisstl;
dvktyfesisstlk;vktyfesisstlka;ktyfesisstlkat;tyfesisstlkatk;yfesisstlka
tkg;fesisstlkatkgk;esisstlkatkgkl;sisstlkatkgkld;isstlkatkgklde;sstlkat
kgkldel;stlkatkgkldelv;tlkatkgkldelvs;lkatkgkldelvsa;katkgkldelvsak;atk
gkldelvsakk;tkgkldelvsakkg;kgkldelvsakkge;gkldelvsakkgeg;kldelvsakkgegg
;ldelvsakkgeggs;delvsakkgeggsv;elvsakkgeggsvk;lvsakkgeggsvka;vsakkgeggs
vkas;sakkgeggsvkasv;akkgeggsvkasve;kkgeggsvkasves;kgeggsvkasvesa;geggsv
kasvesav;eggsvkasvesavd;ggsvkasvesavde;gsvkasvesavdev;svkasvesavdevs;vk
asvesavdevsk;kasvesavdevskw;asvesavdevskwl;svesavdevskwle;vesavdevskwle
e;esavdevskwleem;savdevskwleemi;avdevskwleemik;vdevskwleemika;devskwlee
mikaa;evskwleemikaae;vskwleemikaaee;skwleemikaaeea;kwleemikaaeeaa;wleem
ikaaeeaak;leemikaaeeaakv;eemikaaeeaakvg;emikaaeeaakvgg;mikaaeeaakvggt;i
kaaeeaakvggtg;kaaeeaakvggtgg;aaeeaakvggtggd;aeeaakvggtggdg;eeaakvggtggd
gk;eaakvggtggdgki;aakvggtggdgkig;akvggtggdgkigd;kvggtggdgkigds;vggtggdg
kigdsa;ggtggdgkigdsaa;gtggdgkigdsaan;tggdgkigdsaanh;ggdgkigdsaanhg;gdgk
igdsaanhga;dgkigdsaanhgak;gkigdsaanhgaka;kigdsaanhgakad;igdsaanhgakadk;

gdsaanhgakadkd;dsaanhgakadkds;saanhgakadkdsv;aanhgakadkdsvk;anhgakadkds
vkg;nhgakadkdsvkgi;hgakadkdsvkgia;gakadkdsvkgiak;akadkdsvkgiakg;kadkdsv
kgiakgi;adkdsvkgiakgik;dkdsvkgiakgikg;kdsvkgiakgikgi;dsvkgiakgikgiv;svk
giakgikgivd;vkgiakgikgivda;kgiakgikgivdaa;giakgikgivdaag;iakgikgivdaagk
;akgikgivdaagka;kgikgivdaagkal;gikgivdaagkalg;ikgivdaagkalge;kgivdaagka
lgek;givdaagkalgekg;ivdaagkalgekga;vdaagkalgekgal;daagkalgekgalk;aagkal
gekgalkd;agkalgekgalkdv;gkalgekgalkdvk;kalgekgalkdvka;algekgalkdvkaa;lg
ekgalkdvkaaa;gekgalkdvkaaad;ekgalkdvkaaadd;kgalkdvkaaadde;galkdvkaaadde
a;alkdvkaaaddean;lkdvkaaaddeana;kdvkaaaddeanad;dvkaaaddeanada;vkaaaddea
nadag;kaaaddeanadagk;aaaddeanadagkl;aaddeanadagklf;addeanadagklfa;ddean
adagklfag;deanadagklfagn;eanadagklfagna;anadagklfagnan;nadagklfagnana;a
dagklfagnanaa;dagklfagnanaav;agklfagnanaavg;gklfagnanaavga;klfagnanaavg
aa;lfagnanaavgaaa;fagnanaavgaaad;agnanaavgaaadi;gnanaavgaaadia;nanaavga
aadiak;anaavgaaadiaka;naavgaaadiakaa;aavgaaadiakaag;avgaaadiakaaga;vgaa
adiakaagav;gaaadiakaagavt;aaadiakaagavta;aadiakaagavtav;adiakaagavtavs;
diakaagavtavsg;iakaagavtavsge;akaagavtavsgeq;kaagavtavsgeqi;aagavtavsge
qil;agavtavsgeqilk;gavtavsgeqilka;avtavsgeqilkai;vtavsgeqilkaiv;tavsgeq
ilkaive;avsgeqilkaivea;vsgeqilkaiveaa;sgeqilkaiveaag;geqilkaiveaagd;eqi
lkaiveaagdp;qilkaiveaagdpa;ilkaiveaagdpan;lkaiveaagdpanq;kaiveaagdpanqa
;aiveaagdpanqag;iveaagdpanqagk;veaagdpanqagkk;eaagdpanqagkka;aagdpanqag
kkae;agdpanqagkkaee;gdpanqagkkaeea;dpanqagkkaeeak;panqagkkaeeakn;anqagk
kaeeaknp;nqagkkaeeaknpi;qagkkaeeaknpia;agkkaeeaknpiaa;gkkaeeaknpiaaa;kk
aeeaknpiaaai;kaeeaknpiaaaig;aeeaknpiaaaigt;eeaknpiaaaigtd;eaknpiaaaigtd
d;aknpiaaaigtddd;knpiaaaigtdddn;npiaaaigtdddng;piaaaigtdddnga;iaaaigtdd
dngaa;aaaigtdddngaaf;aaigtdddngaafk;aigtdddngaafkd;igtdddngaafkde;gtddd
ngaafkdem;tdddngaafkdemk;dddngaafkdemkk;ddngaafkdemkks;dngaafkdemkksd;n
gaafkdemkksdk;gaafkdemkksdki;aafkdemkksdkia;afkdemkksdkiaa;fkdemkksdkia
aa;kdemkksdkiaaai;demkksdkiaaaiv;emkksdkiaaaivl;mkksdkiaaaivlr;kksdkiaa
aivlrg;ksdkiaaaivlrgv;sdkiaaaivlrgva;dkiaaaivlrgvak;kiaaaivlrgvakd;iaaa
ivlrgvakdg;aaaivlrgvakdgk;aaivlrgvakdgkf;aivlrgvakdgkfa;ivlrgvakdgkfav;
vlrgvakdgkfavk;

15 mers:
mkkissaifltallv;kkissaifltallvf;kissaifltallvfi;issaifltallvfin;ssaiflt
allvfinc;saifltallvfinck;aifltallvfinckn;ifltallvfincknn;fltallvfincknn
a;ltallvfincknnav;tallvfincknnavg;allvfincknnavgk;llvfincknnavgkg;lvfin
cknnavgkgn;vfincknnavgkgnd;fincknnavgkgndd;incknnavgkgnddk;ncknnavgkgnd
dkd;cknnavgkgnddkds;knnavgkgnddkdsv;nnavgkgnddkdsvk;navgkgnddkdsvkt;avg
kgnddkdsvktf;vgkgnddkdsvktfy;gkgnddkdsvktfye;kgnddkdsvktfyes;gnddkdsvkt
fyesi;nddkdsvktfyesii;ddkdsvktfyesiin;dkdsvktfyesiinl;kdsvktfyesiinlg;d
svktfyesiinlgn;svktfyesiinlgng;vktfyesiinlgngf;ktfyesiinlgngfi;tfyesiin
lgngfid;fyesiinlgngfidv;yesiinlgngfidvf;esiinlgngfidvfn;siinlgngfidvfna
;iinlgngfidvfnaf;inlgngfidvfnafs;nlgngfidvfnafsg;lgngfidvfnafsgl;gngfid
vfnafsglv;ngfidvfnafsglva;gfidvfnafsglvad;fidvfnafsglvadt;idvfnafsglvad
tf;dvfnafsglvadtff;vfnafsglvadtffk;fnafsglvadtffks;nafsglvadtffksd;afsg
lvadtffksdp;fsglvadtffksdpk;sglvadtffksdpkk;glvadtffksdpkks;lvadtffksdp
kksd;vadtffksdpkksdv;adtffksdpkksdvk;dtffksdpkksdvkt;tffksdpkksdvkty;ff
ksdpkksdvktyf;fksdpkksdvktyfe;ksdpkksdvktyfes;sdpkksdvktyfesi;dpkksdvkt
yfesis;pkksdvktyfesiss;kksdvktyfesisst;ksdvktyfesisstl;sdvktyfesisstlk;
dvktyfesisstlka;vktyfesisstlkat;ktyfesisstlkatk;tyfesisstlkatkg;yfesiss
tlkatkgk;fesisstlkatkgkl;esisstlkatkgkld;sisstlkatkgklde;isstlkatkgklde
l;sstlkatkgkldelv;stlkatkgkldelvs;tlkatkgkldelvsa;lkatkgkldelvsak;katkg
kldelvsakk;atkgkldelvsakkg;tkgkldelvsakkge;kgkldelvsakkgeg;gkldelvsakkg
egg;kldelvsakkgeggs;ldelvsakkgeggsv;delvsakkgeggsvk;elvsakkgeggsvka;lvs
akkgeggsvkas;vsakkgeggsvkasv;sakkgeggsvkasve;akkgeggsvkasves;kkgeggsvka
svesa;kgeggsvkasvesav;geggsvkasvesavd;eggsvkasvesavde;ggsvkasvesavdev;g
svkasvesavdevs;svkasvesavdevsk;vkasvesavdevskw;kasvesavdevskwl;asvesavd

evskwle;svesavdevskwlee;vesavdevskwleem;esavdevskwleemi;savdevskwleemik
;avdevskwleemika;vdevskwleemikaa;devskwleemikaae;evskwleemikaaee;vskwle
emikaaeea;skwleemikaaeeaa;kwleemikaaeeaak;wleemikaaeeaakv;leemikaaeeaak
vg;eemikaaeeaakvgg;emikaaeeaakvggt;mikaaeeaakvggtg;ikaaeeaakvggtgg;kaae
eaakvggtggd;aaeeaakvggtggdg;aeeaakvggtggdgk;eeaakvggtggdgki;eaakvggtggd
gkig;aakvggtggdgkigd;akvggtggdgkigds;kvggtggdgkigdsa;vggtggdgkigdsaa;gg
tggdgkigdsaan;gtggdgkigdsaanh;tggdgkigdsaanhg;ggdgkigdsaanhga;gdgkigdsa
anhgak;dgkigdsaanhgaka;gkigdsaanhgakad;kigdsaanhgakadk;igdsaanhgakadkd;
gdsaanhgakadkds;dsaanhgakadkdsv;saanhgakadkdsvk;aanhgakadkdsvkg;anhgaka
dkdsvkgi;nhgakadkdsvkgia;hgakadkdsvkgiak;gakadkdsvkgiakg;akadkdsvkgiakg
i;kadkdsvkgiakgik;adkdsvkgiakgikg;dkdsvkgiakgikgi;kdsvkgiakgikgiv;dsvkg
iakgikgivd;svkgiakgikgivda;vkgiakgikgivdaa;kgiakgikgivdaag;giakgikgivda
agk;iakgikgivdaagka;akgikgivdaagkal;kgikgivdaagkalg;gikgivdaagkalge;ikg
ivdaagkalgek;kgivdaagkalgekg;givdaagkalgekga;ivdaagkalgekgal;vdaagkalge
kgalk;daagkalgekgalkd;aagkalgekgalkdv;agkalgekgalkdvk;gkalgekgalkdvka;k
algekgalkdvkaa;algekgalkdvkaaa;lgekgalkdvkaaad;gekgalkdvkaaadd;ekgalkdv
kaaadde;kgalkdvkaaaddea;galkdvkaaaddean;alkdvkaaaddeana;lkdvkaaaddeanad
;kdvkaaaddeanada;dvkaaaddeanadag;vkaaaddeanadagk;kaaaddeanadagkl;aaadde
anadagklf;aaddeanadagklfa;addeanadagklfag;ddeanadagklfagn;deanadagklfag
na;eanadagklfagnan;anadagklfagnana;nadagklfagnanaa;adagklfagnanaav;dagk
lfagnanaavg;agklfagnanaavga;gklfagnanaavgaa;klfagnanaavgaaa;lfagnanaavg
aaad;fagnanaavgaaadi;agnanaavgaaadia;gnanaavgaaadiak;nanaavgaaadiaka;an
aavgaaadiakaa;naavgaaadiakaag;aavgaaadiakaaga;avgaaadiakaagav;vgaaadiak
aagavt;gaaadiakaagavta;aaadiakaagavtav;aadiakaagavtavs;adiakaagavtavsg;
diakaagavtavsge;iakaagavtavsgeq;akaagavtavsgeqi;kaagavtavsgeqil;aagavta
vsgeqilk;agavtavsgeqilka;gavtavsgeqilkai;avtavsgeqilkaiv;vtavsgeqilkaiv
e;tavsgeqilkaivea;avsgeqilkaiveaa;vsgeqilkaiveaag;sgeqilkaiveaagd;geqil
kaiveaagdp;eqilkaiveaagdpa;qilkaiveaagdpan;ilkaiveaagdpanq;lkaiveaagdpa
nqa;kaiveaagdpanqag;aiveaagdpanqagk;iveaagdpanqagkk;veaagdpanqagkka;eaa
gdpanqagkkae;aagdpanqagkkaee;agdpanqagkkaeea;gdpanqagkkaeeak;dpanqagkka
eeakn;panqagkkaeeaknp;anqagkkaeeaknpi;nqagkkaeeaknpia;qagkkaeeaknpiaa;a
gkkaeeaknpiaaa;gkkaeeaknpiaaai;kkaeeaknpiaaaig;kaeeaknpiaaaigt;aeeaknpi
aaaigtd;eeaknpiaaaigtdd;eaknpiaaaigtddd;aknpiaaaigtdddn;knpiaaaigtdddng
;npiaaaigtdddnga;piaaaigtdddngaa;iaaaigtdddngaaf;aaaigtdddngaafk;aaigtd
ddngaafkd;aigtdddngaafkde;igtdddngaafkdem;gtdddngaafkdemk;tdddngaafkdem
kk;dddngaafkdemkks;ddngaafkdemkksd;dngaafkdemkksdk;ngaafkdemkksdki;gaaf
kdemkksdkia;aafkdemkksdkiaa;afkdemkksdkiaaa;fkdemkksdkiaaai;kdemkksdkia
aaiv;demkksdkiaaaivl;emkksdkiaaaivlr;mkksdkiaaaivlrg;kksdkiaaaivlrgv;ks
dkiaaaivlrgva;sdkiaaaivlrgvak;dkiaaaivlrgvakd;kiaaaivlrgvakdg;iaaaivlrg
vakdgk;aaaivlrgvakdgkf;aaivlrgvakdgkfa;aivlrgvakdgkfav;ivlrgvakdgkfavk;

16 mers:
mkkissaifltallvf;kkissaifltallvfi;kissaifltallvfin;issaifltallvfinc;ssa
ifltallvfinck;saifltallvfinckn;aifltallvfincknn;ifltallvfincknna;fltall
vfincknnav;ltallvfincknnavg;tallvfincknnavgk;allvfincknnavgkg;llvfinckn
navgkgn;lvfincknnavgknd;vfincknnavgkndd;fincknnavgknddk;incknnavgkgn
ddkd;ncknnavgkgnddkds;cknnavgkgnddkdsv;knnavgkgnddkdsvk;nnavgkgnddkdsvk
t;navgkgnddkdsvktf;avgkgnddkdsvktfy;vgkgnddkdsvktfye;gkgnddkdsvktfyes;k
gnddkdsvktfyesi;gnddkdsvktfyesii;nddkdsvktfyesiin;ddkdsvktfyesiinl;dkds
vktfyesiinlg;kdsvktfyesiinlgn;dsvktfyesiinlgng;svktfyesiinlgngf;vktfyes
iinlgngfi;ktfyesiinlgngfid;tfyesiinlgngfidv;fyesiinlgngfidvf;yesiinlgng
fidvfn;esiinlgngfidvfna;siinlgngfidvfnaf;iinlgngfidvfnafs;inlgngfidvfna
fsg;nlgngfidvfnafsgl;lgngfidvfnafsglv;gngfidvfnafsglva;ngfidvfnafsglvad
;gfidvfnafsglvadt;fidvfnafsglvadtf;idvfnafsglvadtff;dvfnafsglvadtffk;vf
nafsglvadtffks;fnafsglvadtffksd;nafsglvadtffksdp;afsglvadtffksdpk;fsglv
adtffksdpkk;sglvadtffksdpkks;glvadtffksdpkksd;lvadtffksdpkksdv;vadtffks
dpkksdvk;adtffksdpkksdvkt;dtffksdpkksdvkty;tffksdpkksdvktyf;ffksdpkksdv

ktyfe;fksdpkksdvktyfes;ksdpkksdvktyfesi;sdpkksdvktyfesis;dpkksdvktyfesi
ss;pkksdvktyfesisst;kksdvktyfesisstl;ksdvktyfesisstlk;sdvktyfesisstlka;
dvktyfesisstlkat;vktyfesisstlkatk;ktyfesisstlkatkg;tyfesisstlkatkgk;yfe
sisstlkatkgkl;fesisstlkatkgkld;esisstlkatkgklde;sisstlkatkgkldel;isstlk
atkgkldelv;sstlkatkgkldelvs;stlkatkgkldelvsa;tlkatkgkldelvsak;lkatkgkld
elvsakk;katkgkldelvsakkg;atkgkldelvsakkge;tkgkldelvsakkgeg;kgkldelvsakk
gegg;gkldelvsakkgeggs;kldelvsakkgeggsv;ldelvsakkgeggsvk;delvsakkgeggsvk
a;elvsakkgeggsvkas;lvsakkgeggsvkasv;vsakkgeggsvkasve;sakkgeggsvkasves;a
kkgeggsvkasvesa;kkgeggsvkasvesav;kgeggsvkasvesavd;geggsvkasvesavde;eggs
vkasvesavdev;ggsvkasvesavdevs;gsvkasvesavdevsk;svkasvesavdevskw;vkasves
avdevskwl;kasvesavdevskwle;asvesavdevskwlee;svesavdevskwleem;vesavdevsk
wleemi;esavdevskwleemik;savdevskwleemika;avdevskwleemikaa;vdevskwleemik
aae;devskwleemikaaee;evskwleemikaaeea;vskwleemikaaeeaa;skwleemikaaeeaak
;kwleemikaaeeaakv;wleemikaaeeaakvg;leemikaaeeaakvgg;eemikaaeeaakvggt;em
ikaaeeaakvggtg;mikaaeeaakvggtgg;ikaaeeaakvggtggd;kaaeeaakvggtggdg;aaeea
akvggtggdgk;aeeaakvggtggdgki;eeaakvggtggdgkig;eaakvggtggdgkigd;aakvggtg
gdgkigds;akvggtggdgkigdsa;kvggtggdgkigdsaa;vggtggdgkigdsaan;ggtggdgkigd
saanh;gtggdgkigdsaanhg;tggdgkigdsaanhga;ggdgkigdsaanhgak;gdgkigdsaanhga
ka;dgkigdsaanhgakad;gkigdsaanhgakadk;kigdsaanhgakadkd;igdsaanhgakadkds;
gdsaanhgakadkdsv;dsaanhgakadkdsvk;saanhgakadkdsvkg;aanhgakadkdsvkgi;anh
gakadkdsvkgia;nhgakadkdsvkgiak;hgakadkdsvkgiakg;gakadkdsvkgiakgi;akadkd
svkgiakgik;kadkdsvkgiakgikg;adkdsvkgiakgikgi;dkdsvkgiakgikgiv;kdsvkgiak
gikgivd;dsvkgiakgikgivda;svkgiakgikgivdaa;vkgiakgikgivdaag;kgiakgikgivd
aagk;giakgikgivdaagka;iakgikgivdaagkal;akgikgivdaagkalg;kgikgivdaagkalg
e;gikgivdaagkalgek;ikgivdaagkalgekg;kgivdaagkalgekga;givdaagkalgekgal;i
vdaagkalgekgalk;vdaagkalgekgalkd;daagkalgekgalkdv;aagkalgekgalkdvk;agka
lgekgalkdvka;gkalgekgalkdvkaa;kalgekgalkdvkaaa;algekgalkdvkaaad;lgekgal
kdvkaaadd;gekgalkdvkaaadde;ekgalkdvkaaaddea;kgalkdvkaaaddean;galkdvkaaa
ddeana;alkdvkaaaddeanad;lkdvkaaaddeanada;kdvkaaaddeanadag;dvkaaaddeanad
agk;vkaaaddeanadagkl;kaaaddeanadagklf;aaaddeanadagklfa;aaddeanadagklfag
;addeanadagklfagn;ddeanadagklfagna;deanadagklfagnan;eanadagklfagnana;an
adagklfagnanaa;nadagklfagnanaav;adagklfagnanaavg;dagklfagnanaavga;agklf
agnanaavgaa;gklfagnanaavgaaa;klfagnanaavgaaad;lfagnanaavgaaadi;fagnanaa
vgaaadia;agnanaavgaaadiak;gnanaavgaaadiaka;nanaavgaaadiakaa;anaavgaaadi
akaag;naavgaaadiakaaga;aavgaaadiakaagav;avgaaadiakaagavt;vgaaadiakaagav
ta;gaaadiakaagavtav;aaadiakaagavtavs;aadiakaagavtavsg;adiakaagavtavsge;
diakaagavtavsgeq;iakaagavtavsgeqi;akaagavtavsgeqil;kaagavtavsgeqilk;aag
avtavsgeqilka;agavtavsgeqilkai;gavtavsgeqilkaiv;avtavsgeqilkaive;vtavsg
eqilkaivea;tavsgeqilkaiveaa;avsgeqilkaiveaag;vsgeqilkaiveaagd;sgeqilkai
veaagdp;geqilkaiveaagdpa;eqilkaiveaagdpan;qilkaiveaagdpanq;ilkaiveaagdp
anqa;lkaiveaagdpanqag;kaiveaagdpanqagk;aiveaagdpanqagkk;iveaagdpanqagkk
a;veaagdpanqagkkae;eaagdpanqagkkaee;aagdpanqagkkaeea;agdpanqagkkaeeak;g
dpanqagkkaeeakn;dpanqagkkaeeaknp;panqagkkaeeaknpi;anqagkkaeeaknpia;nqag
kkaeeaknpiaa;qagkkaeeaknpiaaa;agkkaeeaknpiaaai;gkkaeeaknpiaaaig;kkaeeak
npiaaaigt;kaeeaknpiaaaigtd;aeeaknpiaaaigtdd;eeaknpiaaaigtddd;eaknpiaaai
gtdddn;aknpiaaaigtdddng;knpiaaaigtdddnga;npiaaaigtdddngaa;piaaaigtdddng
aaf;iaaaigtdddngaafk;aaaigtdddngaafkd;aaigtdddngaafkde;aigtdddngaafkdem
;igtdddngaafkdemk;gtdddngaafkdemkk;tdddngaafkdemkks;dddngaafkdemkksd;dd
ngaafkdemkksdk;dngaafkdemkksdki;ngaafkdemkksdkia;gaafkdemkksdkiaa;aafkd
emkksdkiaaa;afkdemkksdkiaaai;fkdemkksdkiaaaiv;kdemkksdkiaaaivl;demkksdk
iaaaivlr;emkksdkiaaaivlrg;mkksdkiaaaivlrgv;kksdkiaaaivlrgva;ksdkiaaaivl
rgvak;sdkiaaaivlrgvakd;dkiaaaivlrgvakdg;kiaaaivlrgvakdgk;iaaaivlrgvakdg
kf;aaaivlrgvakdgkfa;aaivlrgvakdgkfav;aivlrgvakdgkfavk;

**Fig. 40.**

## A) Borrelia burgdorferi MHC class1 & 2 epitopes

**<ABX71745 CRASP-2;Protein;Borrelia burgdorferi>**

**Class 1**
MKKSFLSI;KSFLSIYM;SFLSIYML;FLSIYMLI;SIYMLISI;YMLISISL;MLISISLL;LLSCDVSR;DVSRL
NQR;NIDELKIF;ELKIFVEK;KIFVEKAK;IFVEKAKY;FVEKAKYY;EKAKYYSI;KAKYYSIK;YSIKLDAI
;SIKLDAIY;KLDAIYSE;YSEYTGAY;AYNDIMTY;IMTYIMTY;MTYIMTYS;GTSSDKSK;TSSDKSKV;
KSKVNQAI;KVNQAISI;NQAISILK;QAISILKK;SILKKDNK;EEYKPMFL;KPMFLSKL;PMFLSKLI;KLI
DDFAI;FAIELDQA;AIELDQAV;DVSNARHV;HVADSYEK;DSYEKLRK;KLRKSVAL;KSVALAYI;AL
AYIESF;AYIESFDV;YIESFDVI;ESFDVISS;DVISSKFV;SSKFVDSK;KFVDSKFV;KFVEASKK;ASK
KFVNK;KKFVNKAK;FVNKAKEF;FVEENDLI;EENDLIAL;DLIALKCI;LIALKCIV;IALKCIVK;ALKCIV
KT;REINSRSR;EINSRSRY;RSRYNNFY;SRYNNFYK;RYNNFYKK;FYKKEADF;KEADFLGA;EAD
FLGAA;FLGAAVEL;AAVELEGA;AVELEGAY;ELEGAYKA;EGAYKAIK;YKAIKQTL;KAIKQTLL

KSFLSIYML;SFLSIYMLI;FLSIYMLIS;LSIYMLISI;IYMLISISL;YMLISISLL;MLISISLLS;SISLLSCDV;
SLLSCDVSR;LLSCDVSRL;NIDELKIFV;KIFVEKAKY;EKAKYYSIK;YYSIKLDAI;YSIKLDAIY;KLDA
IYSEY;AIYSEYTGA;IYSEYTGAY;EYTGAYNDI;YTGAYNDIM;GAYNDIMTY;AYNDIMTYI;YNDIMT
YIM;DIMTYIMTY;IMTYIMTYS;MTYIMTYSE;YIMTYSEGT;YSEGTSSDK;KVNQAISIL;VNQAISILK
;NQAISILKK;ISILKKDNK;ILKKDNKIV;KIVNKFKEL;ELEKIIEEY;KIIEEYKPM;IEEYKPMFL;EYKPM
FLSK;KPMFLSKLI;FLSKLIDDF;KLIDDFAIE;LIDDFAIEL;FAIELDQAV;DVSNARHVA;NARHVADS
Y;HVADSYEKL;YEKLRKSVA;KLRKSVALA;VALAYIESF;LAYIESFDV;AYIESFDVI;ESFDVISSK;S
FDVISSKF;ISSKFVDSK;FVDSKFVEA;DSKFVEASK;KFVEASKKF;FVEASKKFV;EASKKFVNK;K
FVNKAKEF;FVNKAKEFV;KEFVEENDL;VEENDLIAL;EENDLIALK;DLIALKCIV;LIALKCIVK;ALKCI
VKTI;IVKTIGDMV;TIGDMVNDR;REINSRSRY;RSRYNNFYK;SRYNNFYKK;NFYKKEADF;FYKKE
ADFL;KEADFLGAA;EADFLGAAV;DFLGAAVEL;FLGAAVELE;AAVELEGAY;AVELEGAYK;VELE
GAYKA;ELEGAYKAI;AYKAIKQTL;YKAIKQTLL

KSFLSIYMLI;FLSIYMLISI;SIYMLISISL;IYMLISISLL;YMLISISLLS;MLISISLLSC;ISLLSCDVSR;SL
LSCDVSRL;RLNQRNIDEL;ELKIFVEKAK;KIFVEKAKYY;FVEKAKYYSI;KYYSIKLDAI;YYSIKLDAI
Y;KLDAIYSEYT;AIYSEYTGAY;SEYTGAYNDI;TGAYNDIMTY;GAYNDIMTYI;AYNDIMTYIM;DIMT
YIMTYS;TYSEGTSSDK;KVNQAISILK;VNQAISILKK;AISILKKDNK;SILKKDNKIV;KDNKIVNKFK;I
VNKFKELEK;KELEKIIEEY;ELEKIIEEYK;KIIEEYKPMF;IIEEYKPMFL;EEYKPMFLSK;EYKPMFLS
KL;MFLSKLIDDF;FLSKLIDDFA;KLIDDFAIEL;ELDQAVDNDV;AVDNDVSNAR;ARHVADSYEK;H
VADSYEKLR;VADSYEKLRK;YEKLRKSVAL;KLRKSVALAY;SVALAYIESF;ALAYIESFDV;LAYIES
FDVI;ESFDVISSKF;SFDVISSKFV;VISSKFVDSK;ISSKFVDSKF;DSKFVEASKK;EASKKFVNKA;A
SKKFVNKAK;KEFVEENDLI;FVEENDLIAL;EENDLIALKC;DLIALKCIVK;CIVKTIGDMV;KTIGDMV
NDR;MVNDREINSR;NSRSRYNNFY;RSRYNNFYKK;KEADFLGAAV;FLGAAVELEG;GAAVELEG
AY;AAVELEGAYK;VELEGAYKAI;ELEGAYKAIK;GAYKAIKQTL;AYKAIKQTLL

SFLSIYMLISI;FLSIYMLISIS;LSIYMLISISL;SIYMLISISLL;IYMLISISLLS;YMLISISLLSC;LISISLLSC
DV;SISLLSCDVSR;LSCDVSRLNQR;RLNQRNIDELK;NQRNIDELKIF;NIDELKIFVEK;ELKIFVEK
AKY;FVEKAKYYSIK;VEKAKYYSIKL;KYYSIKLDAIY;SIKLDAIYSEY;KLDAIYSEYTG;DAIYSEYTG
AY;SEYTGAYNDIM;YTGAYNDIMTY;TGAYNDIMTYI;GAYNDIMTYIM;YNDIMTYIMTY;MTYIMTY
SEGT;YIMTYSEGTSS;MTYSEGTSSDK;KVNQAISILKK;QAISILKKDNK;ILKKDNKIVNK;KIVNKF
KELEK;KIIEEYKPMFL;EEYKPMFLSKL;EYKPMFLSKLI;PMFLSKLIDDF;FLSKLIDDFAI;LIDDFAI
ELDQ;QAVDNDVSNAR;VSNARHVADSY;NARHVADSYEK;HVADSYEKLRK;SYEKLRKSVAL;KL
RKSVALAYI;KSVALAYIESF;VALAYIESFDV;ALAYIESFDVI;YIESFDVISSK;IESFDVISSKF;ESFD
VISSKFV;DVISSKFVDSK;FVDSKFVEASK;FVEASKKFVNK;EASKKFVNKAK;KEFVEENDLIA;EF
VEENDLIAL;FVEENDLIALK;EENDLIALKCI;LIALKCIVKTI;TIGDMVNDREI;DMVNDREINSR;EINS
RSRYNNF;NSRSRYNNFYK;EADFLGAAVEL;FLGAAVELEGA;LGAAVELEGAY;GAAVELEGAYK
;EGAYKAIKQTL;GAYKAIKQTLL

**Class 2**

ADFLGAAVELEGAYK;FLGAAVELE;ADSYEKLRKSVALAY;EKLRKSVAL;ADSYEKLRKSVALAY;
YEKLRKSVA;AISILKKDNKIVNKF;LKKDNKIVN;AYIESFDVISSKFVD;FDVISSKFV;AYNDIMTYIM
TYSEG;YNDIMTYIM;DAIYSEYTGAYNDIM;IYSEYTGAY;DDFAIELDQAVDNDV;FAIELDQAV;DF
LGAAVELEGAYKA;FLGAAVELE;DIMTYIMTYSEGTSS;IMTYSEGTS;DIMTYIMTYSEGTSS;YIMT
YSEGT;DKSKVNQAISILKKD;VNQAISILK;DLIALKCIVKTIGDM;IALKCIVKT;DQAVDNDVSNARH
VA;VDNDVSNAR;DSKFVEASKKFVNKA;FVEASKKFV;DSYEKLRKSVALAYI;LRKSVALAY;DSY
EKLRKSVALAYI;YEKLRKSVA;EADFLGAAVELEGAY;FLGAAVELE;EENDLIALKCIVKTI;IALKCIV
KT;EEYKPMFLSKLIDDF;YKPMFLSKL;EKIIEEYKPMFLSKL;IEEYKPMFL;EKLRKSVALAYIESF;L
RKSVALAY;ELDQAVDNDVSNARH;VDNDVSNAR;ELEKIIEEYKPMFLS;IEEYKPMFL;ENDLIALK
CIVKTIG;IALKCIVKT;ESFDVISSKFVDSKF;FDVISSKFV;EYKPMFLSKLIDDFA;YKPMFLSKL;EY
TGAYNDIMTYIMT;YNDIMTYIM;FDVISSKFVDSKFVE;FDVISSKFV;FLGAAVELEGAYKAI;VELE
GAYKA;FLSIYMLISISLLSC;FLSIYMLIS;FLSIYMLISISLLSC;IYMLISISL;FLSIYMLISISLLSC;MLIS
ISLLS;FVDSKFVEASKKFVN;FVEASKKFV;GAYNDIMTYIMTYSE;YNDIMTYIM;IALKCIVKTIGDM
VN;IALKCIVKT;IALKCIVKTIGDMVN;LKCIVKTIG;IDDFAIELDQAVDND;FAIELDQAV;IEEYKPMF
LSKLIDD;YKPMFLSKL;IELDQAVDNDVSNAR;IELDQAVDN;IESFDVISSKFVDSK;FDVISSKFV;II
EEYKPMFLSKLID;YKPMFLSKL;IKLDAIYSEYTGAYN;IYSEYTGAY;IKLDAIYSEYTGAYN;LDAIY
SEYT;IMTYIMTYSEGTSSD;IMTYSEGTS;IMTYIMTYSEGTSSD;YIMTYSEGT;ISILKKDNKIVNKF
K;LKKDNKIVN;IYMLISISLLSCDVS;IYMLISISL;IYMLISISLLSCDVS;LISISLLSC;IYMLISISLLSCD
VS;MLISISLLS;KEADFLGAAVELEGA;FLGAAVELE;KELEKIIEEYKPMFL;IIEEYKPMF;KFVDSKF
VEASKKFV;FVDSKFVEA;KIIEEYKPMFLSKLI;IEEYKPMFL;KIIEEYKPMFLSKLI;YKPMFLSKL;KK
EADFLGAAVELEG;FLGAAVELE;KKSFLSIYMLISISL;FLSIYMLIS;KKSFLSIYMLISISL;LSIYMLISI
;KLDAIYSEYTGAYND;IYSEYTGAY;KLDAIYSEYTGAYND;LDAIYSEYT;KLIDDFAIELDQAVD;FAI
ELDQAV;KLRKSVALAYIESFD;LRKSVALAY;KSFLSIYMLISISLL;FLSIYMLIS;KSFLSIYMLISISLL;
IYMLISISL;KSKVNQAISILKKDN;VNQAISILK;KVNQAISILKKDNKI;VNQAISILK;KYYSIKLDAIYSE
YT;YSIKLDAIY;LAYIESFDVISSKFV;SFDVISSKF;LAYIESFDVISSKFV;YIESFDVIS;LDAIYSEYT
GAYNDI;IYSEYTGAY;LDAIYSEYTGAYNDI;LDAIYSEYT;LDQAVDNDVSNARHV;VDNDVSNAR;
LEKIIEEYKPMFLSK;IEEYKPMFL;LIALKCIVKTIGDMV;LKCIVKTIG;LIDDFAIELDQAVDN;FAIELD
QAV;LRKSVALAYIESFDV;LRKSVALAY;LSIYMLISISLLSCD;IYMLISISL;LSIYMLISISLLSCD;MLI
SISLLS;MKKSFLSIYMLISIS;FLSIYMLIS;MLISISLLSCDVSRL;ISLLSCDVS;MLISISLLSCDVSRL;
MLISISLLS;MTYIMTYSEGTSSDK;YIMTYSEGT;MVNDREINSRSRYNN;REINSRSRY;NDIMTYIM
TYSEGTS;IMTYIMTYS;NDIMTYIMTYSEGTS;YIMTYSEGT;NDLIALKCIVKTIGD;IALKCIVKT;ND
REINSRSRYNNFY;REINSRSRY;NQAISILKKDNKIVN;ISILKKDNK;QAISILKKDNKIVNK;ISILKKD
NK;QAVDNDVSNARHVAD;VDNDVSNAR;SDKSKVNQAISILKK;VNQAISILK;SEYTGAYNDIMTYI
M;AYNDIMTYI;SEYTGAYNDIMTYIM;YTGAYNDIM;SFDVISSKFVDSKFV;FDVISSKFV;SFLSIYM
LISISLLS;IYMLISISL;SFLSIYMLISISLLS;YMLISISLL;SIKLDAIYSEYTGAY;LDAIYSEYT;SIYMLISI
SLLSCDV;IYMLISISL;SIYMLISISLLSCDV;MLISISLLS;SIYMLISISLLSCDV;YMLISISLL;SKFVEA
SKKFVNKAK;FVEASKKFV;SKLIDDFAIELDQAV;LIDDFAIEL;SKVNQAISILKKDNK;VNQAISILK;S
SDKSKVNQAISILK;KVNQAISIL;SYEKLRKSVALAYIE;LRKSVALAY;TGAYNDIMTYIMTYS;YNDI
MTYIM;TYIMTYSEGTSSDKS;YIMTYSEGT;VDSKFVEASKKFVNK;FVEASKKFV;VEENDLIALKC
IVKT;ENDLIALKC;VELEGAYKAIKQTLL;VELEGAYKA;VNDREINSRSRYNNF;REINSRSRY;VNQ
AISILKKDNKIV;ISILKKDNK;YEKLRKSVALAYIES;LRKSVALAY;YIESFDVISSKFVDS;FDVISSKF
V;YIMTYSEGTSSDKSK;YIMTYSEGT;YKKEADFLGAAVELE;YKKEADFLG;YKPMFLSKLIDDFAI;
YKPMFLSKL;YMLISISLLSCDVSR;ISLLSCDVS;YMLISISLLSCDVSR;MLISISLLS;YNDIMTYIMTY
SEGT;DIMTYIMTY;YNDIMTYIMTYSEGT;IMTYIMTYS;YNDIMTYIMTYSEGT;YNDIMTYIM;YSIKL
DAIYSEYTGA;LDAIYSEYT;YTGAYNDIMTYIMTY;YNDIMTYIM;YYSIKLDAIYSEYTG;LDAIYSEY
T

**<NP_045689 outer surface protein B;Protein;Borrelia burgdorferi B31>**

**Class 1**

RLLIGFAL;LLIGFALA;LIGFALAL;FALALALI;ALALIGCA;ALIGCAQK;AQKGAESI;LPAVTEDS;VSL
FNGNK;SLFNGNKI;LFNGNKIF;KIFVSKEK;KEKNSSGK;SSGKYDLR;KYDLRATI;ATIDQVEL;TID
QVELK;ELKGTSDK;GSKPDKSK;KVKLTVSA;KLTVSADL;VSADLNTV;NTVTLEAF;EAFDASNQ;
NQKISSKV;KISSKVTK;ISSKVTKK;SITXETLK;ETLKANKL;KANKLDSK;KLDSKKLT;TRSNGTTL;

TTLEYSQI;ATKAVETL;ETLKNSIK;TLKNSIKL;SIKLEGSL;IKLEGSLV;SLVGGKTT;LVGGKTTV;IK
EGTVTL;EGTVTLKR;TLKREIEK;FLNDTAGS;DTAGSNKK;GSNKKTGK;WEDSTSTL;TSTLTISA;
LTISADSK;TISADSKK;SADSKKTK;KTKDLVFL;VFLTDGTI;FLTDGTIT;LTDGTITV;GTITVQQY;Q
QYNTAGT;NTAGTSLE;EIKNLSEL;NLSELKNA

MRLLIGFAL;RLLIGFALA;LLIGFALAL;LIGFALALA;ALALALIGC;LALALIGCA;LALIGCAQK;GAESI
GSQK;SQKENDLNL;SKKSHQNAK;LPAVTEDSV;AVTEDSVSL;VTEDSVSLF;SVSLFNGNK;SLF
NGNKIF;LFNGNKIFV;NSSGKYDLR;DLRATIDQV;ATIDQVELK;GSGTLEGSK;KPDKSKVKL;TVS
ADLNTV;SADLNTVTL;DLNTVTLEA;VTLEAFDAS;EAFDASNQK;ASNQKISSK;KISSKVTKK;KQG
SITXET;GSITXETLK;SITXETLKA;KANKLDSKK;KLDSKKLTR;LTRSNGTTL;RSNGTTLEY;TTLEY
SQIT;SQITDADNA;ITDADNATK;DADNATKAV;ATKAVETLK;ETLKNSIKL;SIKLEGSLV;SLVGGK
TTV;TTVEIKEGT;EIKEGTVTL;VTLKREIEK;GSNKKTGKW;STSTLTISA;TLTISADSK;LTISADSKK
;ISADSKKTK;KTKDLVFLT;FLTDGTITV;VQQYNTAGT;QYNTAGTSL;SLEGSASEI;SEIKNLSEL;E
IKNLSELK;NLSELKNAL;LSELKNALK

RLLIGFALAL;LLIGFALALA;LIGFALALAL;IGFALALALI;FALALALIGC;ALALALIGCA;ALALIGCAQ
K;ALIGCAQKGA;DLNLEDSSKK;SSKKSHQNAK;DLPAVTEDSV;LPAVTEDSVS;AVTEDSVSLF;D
SVSLFNGNK;SVSLFNGNKI;VSLFNGNKIF;SLFNGNKIFV;VSKEKNSSGK;RATIDQVELK;GTLEG
SKPDK;GSKPDKSKVK;LTVSADLNTV;DLNTVTLEAF;EAFDASNQKI;DASNQKISSK;KQGSITXE
TL;ITXETLKANK;TLKANKLDSK;KLDSKKLTRS;KLTRSNGTTL;SQITDADNAT;QITDADNATK;NA
TKAVETLK;KAVETLKNSI;AVETLKNSIK;VETLKNSIKL;NSIKLEGSLV;KLEGSLVGGK;SLVGGKT
TVE;LVGGKTTVEI;TTVEIKEGTV;VEIKEGTVTL;EIKEGTVTLK;TVTLKREIEK;EIEKDGKVKV;IEK
DGKVKVF;FLNDTAGSNK;TAGSNKKTGK;WEDSTSTLTI;STLTISADSK;TLTISADSKK;TISADSK
KTK;VFLTDGTITV;FLTDGTITVQ;QQYNTAGTSL;NTAGTSLEGS;SLEGSASEIK;NLSELKNALK

MRLLIGFALAL;RLLIGFALALA;LLIGFALALAL;LIGFALALALI;FALALALIGCA;ALALALIGCAQ;LAL
ALIGCAQK;DSSKKSHQNAK;HQNAKQDLPAV;LPAVTEDSVSL;DSVSLFNGNKI;SVSLFNGNKIF;
SLFNGNKIFVS;LFNGNKIFVSK;FVSKEKNSSGK;VSKEKNSSGKY;KEKNSSGKYDL;DLRATIDQ
VEL;KPDKSKVKLTV;KLTVSADLNTV;TVSADLNTVTL;TLEAFDASNQK;DASNQKISSKV;KQGSI
TXETLK;SITXETLKANK;ETLKANKLDSK;TLKANKLDSKK;LTRSNGTTLEY;TTLEYSQITDA;SQIT
DADNATK;ITDADNATKAV;DNATKAVETLK;KAVETLKNSIK;EGSLVGGKTTV;SLVGGKTTVEI;LV
GGKTTVEIK;EIKEGTVTLKR;GTVTLKREIEK;EIEKDGKVKVF;IEKDGKVKVFL;VFLNDTAGSNK;F
LNDTAGSNKK;DTAGSNKKTGK;KWEDSTSTLTI;TSTLTISADSK;STLTISADSKK;LTISADSKKTK
;SADSKKTKDLV;LVFLTDGTITV;FLTDGTITVQQ;LTDGTITVQQY;VQQYNTAGTSL;NTAGTSLE
GSA;TSLEGSASEIK;LEGSASEIKNL;SASEIKNLSEL;ASEIKNLSELK

## Class 2

ALIGCAQKGAESIGS;LIGCAQKGA;ANKLDSKKLTRSNGT;LDSKKLTRS;ASEIKNLSELKNALK;IK
NLSELKN;ASNQKISSKVTKKQG;ISSKVTKKQ;AVETLKNSIKLEGSL;LKNSIKLEG;DASNQKISSK
VTKKQ;NQKISSKVT;DGKVKVFLNDTAGSN;VKVFLNDTA;DGTITVQQYNTAGTS;VQQYNTAGT;
DKSKVKLTVSADLNT;VKLTVSADL;DLVFLTDGTITVQQY;FLTDGTITV;DSVSLFNGNKIFVSK;VS
LFNGNKI;EDSVSLFNGNKIFVS;VSLFNGNKI;EGSASEIKNLSELKN;SASEIKNLS;EGSLVGGKTT
VEIKE;VGGKTTVEI;ETLKNSIKLEGSLVG;KNSIKLEGS;ETLKNSIKLEGSLVG;LKNSIKLEG;FALA
LALIGCAQKGA;FALALALIG;FLTDGTITVQQYNTA;FLTDGTITV;FNGNKIFVSKEKNSS;FNGNKIF
VS;GFALALALIGCAQKG;FALALALIG;GKVKVFLNDTAGSNK;VKVFLNDTA;GKWEDSTSTLTISA
D;WEDSTSTLT;GNKIFVSKEKNSSGK;FVSKEKNSS;GSASEIKNLSELKNA;IKNLSELKN;GSITXE
TLKANKLDS;XETLKANKL;GSLVGGKTTVEIKEG;VGGKTTVEI;GTITVQQYNTAGTSL;VQQYNT
AGT;IGFALALALIGCAQK;FALALALIG;IKLEGSLVGGKTTVE;IKLEGSLVG;ITVQQYNTAGTSLEG
;VQQYNTAGT;ITVQQYNTAGTSLEG;YNTAGTSLE;KANKLDSKKLTRSNG;LDSKKLTRS;KAVET
LKNSIKLEGS;LKNSIKLEG;KDGKVKVFLNDTAGS;VKVFLNDTA;KDLVFLTDGTITVQQ;FLTDGTI
TV;KIFVSKEKNSSGKYD;FVSKEKNSS;KKTGKWEDSTSTLTI;WEDSTSTLT;KLEGSLVGGKTTV
EI;LVGGKTTVE;KLTVSADLNTVTLEA;VSADLNTVT;KNSIKLEGSLVGGKT;IKLEGSLVG;KPDKS
KVKLTVSADL;SKVKLTVSA;KQGSITXETLKANKL;ITXETLKAN;KSKVKLTVSADLNTV;VKLTVSA
DL;KTGKWEDSTSTLTIS;WEDSTSTLT;KTKDLVFLTDGTITV;VFLTDGTIT;KVKLTVSADLNTVTL;
LTVSADLNT;KVKVFLNDTAGSNKK;FLNDTAGSN;KWEDSTSTLTISADS;WEDSTSTLT;LDSKKL

TRSNGTTLE;LTRSNGTTL;LEGSLVGGKTTVEIK;VGGKTTVEI;LIGFALALALIGCAQ;FALALALIG
;LKANKLDSKKLTRSN;LDSKKLTRS;LKNSIKLEGSLVGGK;IKLEGSLVG;LKNSIKLEGSLVGGK;L
KNSIKLEG;LLIGFALALALIGCA;FALALALIG;LLIGFALALALIGCA;IGFALALAL;LTVSADLNTVTLE
AF;VSADLNTVT;LVFLTDGTITVQQYN;FLTDGTITV;LVGGKTTVEIKEGTV;VGGKTTVEI;MRLLIG
FALALALIG;IGFALALAL;MRLLIGFALALALIG;LIGFALALA;NGNKIFVSKEKNSSG;FVSKEKNSS;
NKIFVSKEKNSSGKY;FVSKEKNSS;NKKTGKWEDSTSTLT;GKWEDSTST;NKLDSKKLTRSNGT
T;LDSKKLTRS;NSIKLEGSLVGGKTT;IKLEGSLVG;NTAGTSLEGSASEIK;GTSLEGSAS;PDKSKV
KLTVSADLN;VKLTVSADL;QGSITXETLKANKLD;XETLKANKL;QQYNTAGTSLEGSAS;YNTAGT
SLE;QYNTAGTSLEGSASE;YNTAGTSLE;RLLIGFALALALIGC;FALALALIG;RLLIGFALALALIGC;I
GFALALAL;RLLIGFALALALIGC;LIGFALALA;SASEIKNLSELKNAL;IKNLSELKN;SIKLEGSLVGG
KTTV;IKLEGSLVG;SKVKLTVSADLNTVT;VKLTVSADL;SKVTKKQGSITXETL;VTKKQGSIT;SLV
GGKTTVEIKEGT;VGGKTTVEI;SSKVTKKQGSITXET;VTKKQGSIT;SVSLFNGNKIFVSKE;VSLFN
GNKI;TAGTSLEGSASEIKN;LEGSASEIK;TDGTITVQQYNTAGT;ITVQQYNTA;TEDSVSLFNGNKI
FV;VSLFNGNKI;TGKWEDSTSTLTISA;WEDSTSTLT;TITVQQYNTAGTSLE;VQQYNTAGT;TKAV
ETLKNSIKLEG;VETLKNSIK;TKDLVFLTDGTITVQ;FLTDGTITV;TLKANKLDSKKLTRS;ANKLDSK
KL;TLKNSIKLEGSLVGG;IKLEGSLVG;TLKNSIKLEGSLVGG;LKNSIKLEG;TVQQYNTAGTSLEG
S;YNTAGTSLE;VETLKNSIKLEGSLV;LKNSIKLEG;VFLTDGTITVQQYNT;FLTDGTITV;VGGKTTV
EIKEGTVT;VGGKTTVEI;VKLTVSADLNTVTLE;LTVSADLNT;VKVFLNDTAGSNKKT;FLNDTAGS
N;VQQYNTAGTSLEGSA;YNTAGTSLE;VSLFNGNKIFVSKEK;VSLFNGNKI;VTEDSVSLFNGNKI
F;VSLFNGNKI;WEDSTSTLTISADSK;WEDSTSTLT;YNTAGTSLEGSASEI;YNTAGTSLE

**<NP_047005 outer surface protein C;Protein;Borrelia burgdorferi B31>**

**Class 1**
NTLSAILM;TLSAILMT;LSAILMTL;SAILMTLF;AILMTLFL;ILMTLFLF;LMTLFLFI;TLFLFISC;ISCNN
SGK;NSADESVK;NLTEISKK;KITDSNAV;NAVLLAVK;VLLAVKEV;AVKEVEAL;KEVEALLS;LLSSI
DEI;SIDEIAAK;IAAKAIGK;AAKAIGKK;KIHQNNGL;GSLLAGAY;SLLAGAYA;LLAGAYAI;GAYAIST
L;AYAISTLI;YAISTLIK;ISTLIKQK;KQKLDGLK;KLDGLKNE;GLKNEGLK;GLKEKIDA;EKIDAAKK;C
SETFTNK;SETFTNKL;ETFTNKLK;FTNKLKEK;GVTDADAK;DAKEAILK;ILKTNGTK;KTNGTKTK;
AEELGKLF;KLFESVEV;ESVEVLSK;EVLSKAAK;KEMLANSV;EMLANSVK;KELTSPVV;ELTSPV
VA;PVVAESPK;VVAESPKK

MKKNTLSAI;NTLSAILMT;TLSAILMTL;LSAILMTLF;SAILMTLFL;AILMTLFLF;ILMTLFLFI;FLFISC
NNS;FISCNNSGK;SANSADESV;DESVKGPNL;NLTEISKKI;KKITDSNAV;KITDSNAVL;ITDSNAV
LL;DSNAVLLAV;AVLLAVKEV;LLAVKEVEA;LAVKEVEAL;AVKEVEALL;EVEALLSSI;ALLSSIDEI;
LLSSIDEIA;SSIDEIAAK;SIDEIAAKA;EIAAKAIGK;IAAKAIGKK;HQNNGLDTE;TENNHNGSL;NGS
LLAGAY;SLLAGAYAI;LLAGAYAIS;GAYAISTLI;AYAISTLIK;AISTLIKQK;LIKQKLDGL;KLDGLKNE
G;GLKEKIDAA;ETFTNKLKE;KLKEKHTDL;KEKHTDLGK;HTDLGKEGV;AILKTNGTK;ELGKLFES
V;KLFESVEVL;ESVEVLSKA;VLSKAAKEM;MLANSVKEL;SVKELTSPV;LTSPVVAES;PVVAESP
KK

NTLSAILMTL;TLSAILMTLF;LSAILMTLFL;SAILMTLFLF;AILMTLFLFI;ILMTLFLFIS;LMTLFLFISC;
FLFISCNNSG;LFISCNNSGK;TSANSADESV;SANSADESVK;SVKGPNLTEI;KGPNLTEISK;KITD
SNAVLL;DSNAVLLAVK;NAVLLAVKEV;VLLAVKEVEA;LLAVKEVEAL;KEVEALLSSI;EALLSSIDEI
;ALLSSIDEIA;LLSSIDEIAA;LSSIDEIAAK;SSIDEIAAKA;SIDEIAAKAI;EIAAKAIGKK;DTENNHNGS
L;TENNHNGSLL;SLLAGAYAIS;LLAGAYAIST;LAGAYAISTL;AGAYAISTLI;GAYAISTLIK;YAISTLI
KQK;AISTLIKQKL;TLIKQKLDGL;KLDGLKNEGL;GLKNEGLKEK;CSETFTNKLK;ETFTNKLKEK;E
AILKTNGTK;ILKTNGTKTK;KTKGAEELGK;KLFESVEVLS;ESVEVLSKAA;SVEVLSKAAK;EVLSK
AAKEM;VLSKAAKEML;AAKEMLANSV;EMLANSVKEL;MLANSVKELT;NSVKELTSPV;SVKELTS
PVV;TSPVVAESPK

NTLSAILMTLF;TLSAILMTLFL;LSAILMTLFLF;SAILMTLFLFI;ILMTLFLFISC;FLFISCNNSGK;NTS
ANSADESV;TSANSADESVK;ESVKGPNLTEI;KITDSNAVLLA;ITDSNAVLLAV;VLLAVKEVEAL;LL
AVKEVEALL;ALLSSIDEIAA;LLSSIDEIAAK;EIAAKAIGKKI;DTENNHNGSLL;NHNGSLLAGAY;NG
SLLAGAYAI;SLLAGAYAIST;LLAGAYAISTL;LAGAYAISTLI;AYAISTLIKQK;YAISTLIKQKL;TLIKQ

KLDGLK;KLDGLKNEGLK;GLKEKIDAAKK;KCSETFTNKLK;ETFTNKLKEKH;KLKEKHTDLGK;GV
TDADAKEAI;AILKTNGTKTK;AEELGKLFESV;ELGKLFESVEV;KLFESVEVLSK;ESVEVLSKAAK;
VEVLSKAAKEM;EVLSKAAKEML;VLSKAAKEMLA;KAAKEMLANSV;AAKEMLANSVK;KEMLANS
VKEL;MLANSVKELTS;SVKELTSPVVA;LTSPVVAESPK;TSPVVAESPKK

**Class 2**
ADAKEAILKTNGTKT;EAILKTNGT;ADESVKGPNLTEISK;VKGPNLTEI;AGAYAISTLIKQKLD;ISTL
IKQKL;AGAYAISTLIKQKLD;YAISTLIKQ;AILKTNGTKTKGAEE;LKTNGTKTK;AILMTLFLFISCNN
S;LMTLFLFIS;AKEAILKTNGTKTKG;ILKTNGTKT;AKEAILKTNGTKTKG;LKTNGTKTK;AKEMLAN
SVKELTSP;LANSVKELT;AKEMLANSVKELTSP;MLANSVKEL;ANSVKELTSPVVAES;VKELTSP
VV;AVKEVEALLSSIDEI;VKEVEALLS;AVLLAVKEVEALLSS;VKEVEALLS;AYAISTLIKQKLDGL;I
STLIKQKL;CSETFTNKLKEKHTD;FTNKLKEKH;DAKEAILKTNGTKTK;EAILKTNGT;DESVKGPNL
TEISKK;VKGPNLTEI;DGLKNEGLKEKIDAA;LKNEGLKEK;DLGKEGVTDADAKEA;LGKEGVTDA;
EAILKTNGTKTKGAE;ILKTNGTKT;EAILKTNGTKTKGAE;LKTNGTKTK;EGVTDADAKEAILKT;VT
DADAKEA;EISKKITDSNAVLLA;ITDSNAVLL;EMLANSVKELTSPVV;LANSVKELT;EMLANSVKEL
TSPVV;MLANSVKEL;EMLANSVKELTSPVV;NSVKELTSP;EMLANSVKELTSPVV;SVKELTSPV;
ESVEVLSKAAKEMLA;LSKAAKEML;ESVEVLSKAAKEMLA;VLSKAAKEM;ESVKGPNLTEISKKI;V
KGPNLTEI;ETFTNKLKEKHTDLG;FTNKLKEKH;EVLSKAAKEMLANSV;VLSKAAKEM;FESVEVL
SKAAKEML;EVLSKAAKE;FESVEVLSKAAKEML;VLSKAAKEM;GAYAISTLIKQKLDG;ISTLIKQKL
;GKEGVTDADAKEAIL;VTDADAKEA;GSLLAGAYAISTLIK;LAGAYAIST;HNGSLLAGAYAISTL;LA
GAYAIST;ILKTNGTKTKGAEEL;LKTNGTKTK;ILMTLFLFISCNNSG;FLFISCNNS;ISKKITDSNAVL
LAV;ITDSNAVLL;ITDSNAVLLAVKEVE;ITDSNAVLL;KCSETFTNKLKEKHT;FTNKLKEKH;KEAILK
TNGTKTKGA;ILKTNGTKT;KEAILKTNGTKTKGA;LKTNGTKTK;KEGVTDADAKEAILK;VTDADAK
EA;KELTSPVVAESPKKP;LTSPVVAES;KEMLANSVKELTSPV;LANSVKELT;KEMLANSVKELTS
PV;MLANSVKEL;KITDSNAVLLAVKEV;ITDSNAVLL;KKCSETFTNKLKEKH;KKCSETFTN;KKITD
SNAVLLAVKE;ITDSNAVLL;KKNTLSAILMTLFLF;LSAILMTLF;KLDGLKNEGLKEKID;LKNEGLKE
K;KNTLSAILMTLFLFI;LSAILMTLF;KQKLDGLKNEGLKEK;KQKLDGLKN;KAAKEMLANSVKELT;
AKEMLANSV;KAAKEMLANSVKELT;MLANSVKEL;LAGAYAISTLIKQKL;LAGAYAIST;LAGAYAIS
TLIKQKL;YAISTLIKQ;LANSVKELTSPVVAE;LANSVKELT;LANSVKELTSPVVAE;VKELTSPVV;L
AVKEVEALLSSIDE;VKEVEALLS;LDGLKNEGLKEKIDA;LKNEGLKEK;LFESVEVLSKAAKEM;SV
EVLSKAA;LFLFISCNNSGKDGN;LFISCNNSG;LGKEGVTDADAKEAI;VTDADAKEA;LKTNGTKTK
GAEELG;LKTNGTKTK;LLAGAYAISTLIKQK;LAGAYAIST;LLAVKEVEALLSSID;VKEVEALLS;LM
TLFLFISCNNSGK;LFISCNNSG;LSAILMTLFLFISCN;LMTLFLFIS;LSKAAKEMLANSVKE;AKEML
ANSV;LSSIDEIAAKAIGKK;IDEIAAKAI;MKKNTLSAILMTLFL;KNTLSAILM;MKKNTLSAILMTLFL;
MKKNTLSAI;MLANSVKELTSPVVA;LANSVKELT;MLANSVKELTSPVVA;VKELTSPVV;MTLFLFI
SCNNSGKD;LFISCNNSG;NAVLLAVKEVEALLS;LAVKEVEAL;NGSLLAGAYAISTLI;LAGAYAIST;
NHNGSLLAGAYAIST;LLAGAYAIS;NNHNGSLLAGAYAIS;SLLAGAYAI;NSADESVKGPNLTEI;ES
VKGPNLT;NSVKELTSPVVAESP;VKELTSPVV;NTLSAILMTLFLFIS;LSAILMTLF;QKLDGLKNEG
LKEKI;LKNEGLKEK;SADESVKGPNLTEIS;VKGPNLTEI;SAILMTLFLFISCNN;LMTLFLFIS;SETF
TNKLKEKHTDL;FTNKLKEKH;SKKITDSNAVLLAVK;ITDSNAVLL;SKAAKEMLANSVKEL;AKEML
ANSV;SLLAGAYAISTLIKQ;LAGAYAIST;SSIDEIAAKAIGKKI;IDEIAAKAI;SVEVLSKAAKEMLAN;L
SKAAKEML;SVEVLSKAAKEMLAN;VLSKAAKEM;SVKELTSPVVAESPK;VKELTSPVV;TEISKKIT
DSNAVLL;KKITDSNAV;TLFLFISCNNSGKDG;LFISCNNSG;TLSAILMTLFLFISC;LMTLFLFIS;VE
VLSKAAKEMLANS;VLSKAAKEM;VKELTSPVVAESPKK;VKELTSPVV;VKEVEALLSSIDEIA;VKE
VEALLS;VLLAVKEVEALLSSI;VKEVEALLS;VLSKAAKEMLANSVK;VLSKAAKEM;YAISTLIKQKL
DGLK;ISTLIKQKL;AAKEMLANSVKELTS;LANSVKELT;AAKEMLANSVKELTS;MLANSVKEL

## B) Borrelia afzelii MHC class1 & 2 epitopes

**<CAA59725 outer surface protein A;Protein;Borrelia afzelii>**

**Class 1**
KYLLGIGL;YLLGIGLI;LLGIGLIL;GIGLILAL;GLILALIA;ILALIACK;LIACKQNV;NVSSLDEK;SLDEK
NSA;SVDLPGEM;DLPGEMKV;LPGEMKVL;EMKVLVSK;KVLVSKEK;SLKATVDK;ATVDKIEL;TV
DKIELK;ELKGTSDK;GTKDDKSK;KLTIADDL;TIADDLSK;KTTFELFK;TTFELFKE;ELFKEDGK;FK

EDGKTL;KEDGKTLV;VSRKVSSK;KTSTDEMF;GELSAKTM;LSAKTMTR;MTRENGTK;TRENGT
KL;KLEYTEMK;GTGKAKEV;EVLKNFTL;FTLEGKVA;VANDKVTL;VKEGTVTL;TLSKEIAK;EIAKS
GEV;ALNDTNTT;NTTQATKK;ATKKTGAW;KTGAWDSK;TSTLTISV;LTISVNSK;TISVNSKK;TTQ
LVFTK;TKQDTITV;DTITVQKY;EGTAVEIK;TAVEIKTL;EIKTLDEL;TLDELKNA

KYLLGIGLI;YLLGIGLIL;LLGIGLILA;GIGLILALI;LILALIACK;ALIACKQNV;SLDEKNSAS;SVDLPGE
MK;VDLPGEMKV;DLPGEMKVL;LPGEMKVLV;GKYSLKATV;YSLKATVDK;SLKATVDKI;ATVDKI
ELK;GSGVLEGTK;LTIADDLSK;TIADDLSKT;DLSKTTFEL;SKTTFELFK;FKEDGKTLV;LVSRKVS
SK;SRKVSSKDK;STDEMFNEK;EMFNEKGEL;ELSAKTMTR;TMTRENGTK;MTRENGTKL;RENG
TKLEY;KSDGTGKAK;TGKAKEVLK;KEVLKNFTL;KVANDKVTL;ANDKVTLEV;TLEVKEGTV;EVK
EGTVTL;VTLSKEIAK;KEIAKSGEV;IAKSGEVTV;KSGEVTVAL;TVALNDTNT;ALNDTNTTQ;DTNT
TQATK;KKTGAWDSK;KTSTLTISV;TLTISVNSK;LTISVNSKK;KTTQLVFTK;FTKQDTITV;KQDTIT
VQK;KYDSAGTNL;GTNLEGTAV;NLEGTAVEI;VEIKTLDEL;EIKTLDELK;TLDELKNAL

KYLLGIGLIL;YLLGIGLILA;LLGIGLILAL;LGIGLILALI;GLILALIACK;KQNVSSLDEK;SLDEKNSASV
;SASVDLPGEM;ASVDLPGEMK;SVDLPGEMKV;DLPGEMKVLV;LPGEMKVLVS;EMKVLVSKEK;
KVLVSKEKDK;KDKDGKYSLK;KYSLKATVDK;KATVDKIELK;KIELKGTSDK;GVLEGTKDDK;GTK
DDKSKAK;KLTIADDLSK;TIADDLSKTT;IADDLSKTTF;DLSKTTFELF;LSKTTFELFK;ELFKEDGK
TL;TLVSRKVSSK;VSRKVSSKDK;TSTDEMFNEK;DEMFNEKGEL;KTMTRENGTK;TMTRENGTK
L;GTKLEYTEMK;GTGKAKEVLK;VLKNFTLEGK;VANDKVTLEV;VTLEVKEGTV;LEVKEGTVTL;T
VTLSKEIAK;EIAKSGEVTV;AKSGEVTVAL;TVALNDTNTT;ALNDTNTTQA;DTNTTQATKK;GAWD
SKTSTL;SKTSTLTISV;STLTISVNSK;TLTISVNSKK;SVNSKKTTQL;KKTTQLVFTK;QLVFTKQDTI
;KTLDELKNAL;TLDELKNALK

YLLGIGLILAL;LLGIGLILALI;ILALIACKQNV;LIACKQNVSSL;NVSSLDEKNSA;SSLDEKNSASV;S
ASVDLPGEMK;SVDLPGEMKVL;VDLPGEMKVLV;LPGEMKVLVSK;LVSKEKDKDGK;KYSLKATV
DKI;SLKATVDKIEL;GTSDKDNGSGV;TIADDLSKTTF;DLSKTTFELFK;TTFELFKEDGK;FELFKED
GKTL;ELFKEDGKTLV;KTLVSRKVSSK;LVSRKVSSKDK;KTSTDEMFNEK;EMFNEKGELSA;MFN
EKGELSAK;KTMTRENGTKL;MTRENGTKLEY;YTEMKSDGTGK;EVLKNFTLEGK;VLKNFTLEGK
V;FTLEGKVANDK;TLEGKVANDKV;KVANDKVTLEV;VANDKVTLEVK;KVTLEVKEGTV;TLEVKE
GTVTL;EVKEGTVTLSK;GTVTLSKEIAK;KEIAKSGEVTV;EIAKSGEVTVA;IAKSGEVTVAL;ALNDT
NTTQAT;TTQATKKTGAW;ATKKTGAWDSK;AWDSKTSTLTI;TSTLTISVNSK;STLTISVNSKK;SV
NSKKTTQLV;SKKTTQLVFTK;TQLVFTKQDTI;LVFTKQDTITV;FTKQDTITVQK;DTITVQKYDSA;V
QKYDSAGTNL;SAGTNLEGTAV;NLEGTAVEIKT;LEGTAVEIKTL;TAVEIKTLDEL;AVEIKTLDELK;
KTLDELKNALK

**Class 2**
AKEVLKNFTLEGKVA;LKNFTLEGK;AKEVLKNFTLEGKVA;VLKNFTLEG;ALNDTNTTQATKKTG;
NTTQATKKT;AWDSKTSTLTISVNS;WDSKTSTLT;DEMFNEKGELSAKTM;MFNEKGELS;DGKTL
VSRKVSSKDK;GKTLVSRKV;DKVTLEVKEGTVTLS;LEVKEGTVT;DLPGEMKVLVSKEKD;MKVL
VSKEK;DTITVQKYDSAGTNL;VQKYDSAGT;EDGKTLVSRKVSSKD;GKTLVSRKV;ELFKEDGKT
LVSRKV;FKEDGKTLV;EMFNEKGELSAKTMT;EKGELSAKT;EVKEGTVTLSKEIAK;VKEGTVTLS;
EVLKNFTLEGKVAND;LKNFTLEGK;EVTVALNDTNTTQAT;LNDTNTTQA;EVTVALNDTNTTQAT;
VALNDTNTT;EYTEMKSDGTGKAKE;MKSDGTGKA;EYTEMKSDGTGKAKE;YTEMKSDGT;FKE
DGKTLVSRKVSS;GKTLVSRKV;FNEKGELSAKTMTRE;EKGELSAKT;FTKQDTITVQKYDSA;FTK
QDTITV;GAWDSKTSTLTISVN;WDSKTSTLT;GEMKVLVSKEKDKDG;MKVLVSKEK;GEVTVALN
DTNTTQA;VALNDTNTT;GIGLILALIACKQNV;ILALIACKQ;GKAKEVLKNFTLEGK;VLKNFTLEG;G
KTLVSRKVSSKDKT;GKTLVSRKV;GLILALIACKQNVSS;ILALIACKQ;GTKLEYTEMKSDGTG;YT
EMKSDGT;IGLILALIACKQNVS;ILALIACKQ;ISVNSKKTTQLVFTK;ISVNSKKTT;ISVNSKKTTQLV
FTK;VNSKKTTQL;ITVQKYDSAGTNLEG;VQKYDSAGT;ITVQKYDSAGTNLEG;YDSAGTNLE;KA
KEVLKNFTLEGKV;LKNFTLEGK;KAKEVLKNFTLEGKV;VLKNFTLEG;KEDGKTLVSRKVSSK;GK
TLVSRKV;KEVLKNFTLEGKVAN;LKNFTLEGK;KEVLKNFTLEGKVAN;VLKNFTLEG;KKTGAWDS
KTSTLTI;WDSKTSTLT;KKYLLGIGLILALIA;LLGIGLILA;KLEYTEMKSDGTGKA;YTEMKSDGT;KQ
DTITVQKYDSAGT;ITVQKYDSA;KSGEVTVALNDTNTT;EVTVALNDT;KTGAWDSKTSTLTIS;WD
SKTSTLT;KTSTLTISVNSKKTT;LTISVNSKK;KTTQLVFTKQDTITV;KTTQLVFTK;KTTQLVFTKQD

TITV;VFTKQDTIT;KVTLEVKEGTVTLSK;VKEGTVTLS;KYLLGIGLILALIAC;LLGIGLILA;LEVKEGT
VTLSKEIA;VKEGTVTLS;LEYTEMKSDGTGKAK;MKSDGTGKA;LEYTEMKSDGTGKAK;YTEMKS
DGT;LFKEDGKTLVSRKVS;GKTLVSRKV;LGIGLILALIACKQN;IGLILALIA;LKNFTLEGKVANDKV;
LKNFTLEGK;LLGIGLILALIACKQ;IGLILALIA;LNDTNTTQATKKTGA;NTTQATKKT;LPGEMKVLVS
KEKDK;MKVLVSKEK;LTISVNSKKTTQLVF;ISVNSKKTT;LTISVNSKKTTQLVF;VNSKKTTQL;LVF
TKQDTITVQKYD;FTKQDTITV;MKKYLLGIGLILALI;LLGIGLILA;NGTKLEYTEMKSDGT;TKLEYTE
MK;NVSSLDEKNSASVDL;LDEKNSASV;PGEMKVLVSKEKDKD;MKVLVSKEK;QDTITVQKYDSA
GTN;VQKYDSAGT;QKYDSAGTNLEGTAV;YDSAGTNLE;QLVFTKQDTITVQKY;FTKQDTITV;SG
EVTVALNDTNTTQ;VALNDTNTT;SSLDEKNSASVDLPG;EKNSASVDL;STLTISVNSKKTTQL;ISV
NSKKTT;STLTISVNSKKTTQL;TISVNSKKT;SVNSKKTTQLVFTKQ;VNSKKTTQL;TDEMFNEKGE
LSAKT;MFNEKGELS;TEMKSDGTGKAKEVL;MKSDGTGKA;TGAWDSKTSTLTISV;WDSKTSTLT
;TGKAKEVLKNFTLEG;AKEVLKNFT;TISVNSKKTTQLVFT;ISVNSKKTT;TISVNSKKTTQLVFT;VN
SKKTTQL;TITVQKYDSAGTNLE;VQKYDSAGT;TKKTGAWDSKTSTLT;KTGAWDSKT;TKLEYTE
MKSDGTGK;YTEMKSDGT;TLEVKEGTVTLSKEI;VKEGTVTLS;TLTISVNSKKTTQLV;ISVNSKKT
T;TLTISVNSKKTTQLV;VNSKKTTQL;TQLVFTKQDTITVQK;FTKQDTITV;TSTLTISVNSKKTTQ;IS
VNSKKTT;TTQLVFTKQDTITVQ;FTKQDTITV;TVALNDTNTTQATKK;LNDTNTTQA;TVALNDTNT
TQATKK;VALNDTNTT;TVQKYDSAGTNLEGT;VQKYDSAGT;TVQKYDSAGTNLEGT;YDSAGTN
LE;VALNDTNTTQATKK;LNDTNTTQA;VALNDTNTTQATKK;VALNDTNTT;VDLPGEMKVLVSK
EK;LPGEMKVLV;VDLPGEMKVLVSKEK;VDLPGEMKV;VFTKQDTITVQKYDS;FTKQDTITV;VKE
GTVTLSKEIAKS;VKEGTVTLS;VLKNFTLEGKVANDK;LKNFTLEGK;VNSKKTTQLVFTKQD;VNS
KKTTQL;VQKYDSAGTNLEGTA;VQKYDSAGT;VQKYDSAGTNLEGTA;YDSAGTNLE;VSSLDEK
NSASVDLP;EKNSASVDL;VTLEVKEGTVTLSKE;VKEGTVTLS;VTVALNDTNTTQATK;LNDTNTT
QA;VTVALNDTNTTQATK;VALNDTNTT;WDSKTSTLTISVNSK;WDSKTSTLT;YLLGIGLILALIACK
;IGLILALIA;YTEMKSDGTGKAKEV;MKSDGTGKA;YTEMKSDGTGKAKEV;YTEMKSDGT

**<YP_853838 outer surface protein B;Protein;Borrelia afzelii PKo>**

**Class 1**
KQYLLVFA;QYLLVFAL;YLLVFALV;LLVFALVL;LVFALVLA;VFALVLAL;FALVLALI;ALVLALIA;ALI
ACSQK;SQKGTEPK;NTPKDSKK;VLAEENSV;AEENSVPL;EENSVPLF;PLFNGNKI;LFNGNKIF;K
IFVSKEK;KEKNSAGK;NSAGKYEL;SAGKYELR;ATVDTVEL;TVDTVELK;DTVELKGV;ELKGVSD
K;SGKLEGTK;GTKADKTK;KTKVAMTI;AMTIADDL;IADDLNTI;NTITVETY;ETYDASNK;TYDASN
KK;KTGSEVVK;VVKKQGSV;KKQGSVIK;SVIKESYK;KANKLDSK;KLDSKKIT;TRENETTL;TTLEY
SEM;EMTDSSNA;ATKAVETL;ETLKNGIK;TLKNGIKL;IKLEGSLV;SLVGGKTT;LVGGKTTV;KLTE
GTIT;EGTITLTR;TLTREIEQ;YLNDTTSG;DTTSGSTK;TTSGSTKK;STKKTATW;ETTNTLTI;LTISA
DSK;TISADSKK;SADSKKTK;KTKDFVFL;FVFLTDGT;VFLTDGTI;FLTDGTIT;LTDGTITV;GTITVQ
AY;AYDTAGTK;SEIKDLAA;EIKDLAAL;DLAALKAA;LAALKAAL;AALKAALK

KQYLLVFAL;QYLLVFALV;YLLVFALVL;LLVFALVLA;LVFALVLAL;VFALVLALI;FALVLALIA;VLALI
ACSQ;LALIACSQK;CSQKGTEPK;SQDHNDQEI;IINSDNTPK;TPKDSKKDL;TVLAEENSV;LAEEN
SVPL;AEENSVPLF;EENSVPLFN;SVPLFNGNK;VPLFNGNKI;LFNGNKIFV;FVSKEKNSA;NSAGK
YELR;GKYELRATV;ELRATVDTV;RATVDTVEL;ATVDTVELK;KADKTKVAM;KTKVAMTIA;TIADD
LNTI;ETYDASNKK;KTGSEVVKK;EVVKKQGSV;GSVIKESYK;SVIKESYKA;KANKLDSKK;KLDSK
KITR;ITRENETTL;RENETTLEY;ETTLEYSEM;TTLEYSEMT;EMTDSSNAT;MTDSSNATK;ATKAV
ETLK;ETLKNGIKL;GIKLEGSLV;SLVGGKTTV;LVGGKTTVK;TTVKLTEGT;KLTEGTITL;REIEQD
GKV;IEQDGKVKI;YLNDTTSGS;DTTSGSTKK;GSTKKTATW;TWNETTNTL;NETTNTLTI;TTNTLT
ISA;TLTISADSK;LTISADSKK;ISADSKKTK;KTKDFVFLT;FVFLTDGTI;FLTDGTITV;ITVQAYDTA;
VQAYDTAGT;QAYDTAGTK;AYDTAGTKL;KLEGNSSEI;NSSEIKDLA;SEIKDLAAL;EIKDLAALK;D
LAALKAAL;LAALKAALK

KQYLLVFALV;QYLLVFALVL;YLLVFALVLA;LLVFALVLAL;LVFALVLALI;VLALIACSQK;ACSQKG
TEPK;SQDHNDQEII;EIINSDNTPK;LTVLAEENSV;VLAEENSVPL;LAEENSVPLF;NSVPLFNGNK;
SVPLFNGNKI;VPLFNGNKIF;PLFNGNKIFV;VSKEKNSAGK;KNSAGKYELR;YELRATVDTV;RAT
VDTVELK;TVDTVELKGV;TVELKGVSDK;VSDKNNGSGK;MTIADDLNTI;IADDLNTITV;DLNTITV
ETY;NTITVETYDA;TVETYDASNK;ETYDASNKKT;EVVKKQGSVI;VVKKQGSVIK;KQGSVIKESY;

VIKESYKANK;SYKANKLDSK;KITRENETTL;ETTLEYSEMT;EMTDSSNATK;MTDSSNATKA;NAT
KAVETLK;KAVETLKNGI;AVETLKNGIK;VETLKNGIKL;KLEGSLVGGK;SLVGGKTTVK;LVGGKTT
VKL;TTVKLTEGTI;VKLTEGTITL;KLTEGTITLT;LTEGTITLTR;LTREIEQDGK;IEQDGKVKIY;YLND
TTSGST;SGSTKKTATW;ATWNETTNTL;ETTNTLTISA;NTLTISADSK;TLTISADSKK;TISADSKKT
K;VFLTDGTITV;FLTDGTITVQ;LTDGTITVQA;VQAYDTAGTK;QAYDTAGTKL;KLEGNSSEIK;DLA
ALKAALK

MKQYLLVFALV;KQYLLVFALVL;YLLVFALVLAL;LLVFALVLALI;ALVLALIACSQ;LVLALIACSQK;I
ACSQKGTEPK;STSQDHNDQEI;DLTVLAEENSV;TVLAEENSVPL;VLAEENSVPLF;NSVPLFNGN
KI;SVPLFNGNKIF;VPLFNGNKIFV;LFNGNKIFVSK;FVSKEKNSAGK;VSKEKNSAGKY;KEKNSA
GKYEL;EKNSAGKYELR;SAGKYELRATV;ELRATVDTVEL;ATVDTVELKGV;DTVELKGVSDK;GV
SDKNNGSGK;KLEGTKADKTK;KADKTKVAMTI;AMTIADDLNTI;MTIADDLNTIT;TIADDLNTITV;IT
VETYDASNK;TVETYDASNKK;EVVKKQGSVIK;KQGSVIKESYK;SVIKESYKANK;VIKESYKANKL
;ESYKANKLDSK;SYKANKLDSKK;ITRENETTLEY;EMTDSSNATKA;MTDSSNATKAV;KAVETLK
NGIK;TLKNGIKLEGS;EGSLVGGKTTV;GSLVGGKTTVK;SLVGGKTTVKL;KLTEGTITLTR;TLTRE
IEQDGK;REIEQDGKVKI;EIEQDGKVKIY;IEQDGKVKIYL;YLNDTTSGSTK;TSGSTKKTATW;TAT
WNETTNTL;TWNETTNTLTI;TNTLTISADSK;NTLTISADSKK;LTISADSKKTK;SADSKKTKDFV;FV
FLTDGTITV;FLTDGTITVQA;LTDGTITVQAY;TVQAYDTAGTK;VQAYDTAGTKL;LEGNSSEIKDL;
NSSEIKDLAAL;SSEIKDLAALK

## Class 2
AEENSVPLFNGNKIF;VPLFNGNKI;AGKYELRATVDTVEL;YELRATVDT;ALVLALIACSQKGTE;V
LALIACSQ;ATWNETTNTLTISAD;WNETTNTLT;AVETLKNGIKLEGSL;LKNGIKLEG;DASNKKTG
SEVVKKQ;NKKTGSEVV;DFVFLTDGTITVQAY;FLTDGTITV;DGKVKIYLNDTTSGS;IYLNDTTSG;
DGKVKIYLNDTTSGS;VKIYLNDTT;DGTITVQAYDTAGTK;VQAYDTAGT;DKTKVAMTIADDLNT;V
AMTIADDL;DNTPKDSKKDLTVLA;DNTPKDSKK;DQEIINSDNTPKDSK;INSDNTPKD;DSKKDLTV
LAEENSV;SKKDLTVLA;EENSVPLFNGNKIFV;VPLFNGNKI;EGNSSEIKDLAALKA;EGNSSEIKD;
EGSLVGGKTTVKLTE;VGGKTTVKL;EIINSDNTPKDSKKD;INSDNTPKD;ENSVPLFNGNKIFVS;V
PLFNGNKI;ETLKNGIKLEGSLVG;LKNGIKLEG;ETYDASNKKTGSEVV;SNKKTGSEV;EVVKKQG
SVIKESYK;VKKQGSVIK;FALVLALIACSQKGT;LVLALIACS;FLTDGTITVQAYDTA;FLTDGTITV;F
NGNKIFVSKEKNSA;FNGNKIFVS;FVFLTDGTITVQAYD;FLTDGTITV;GGKTTVKLTEGTITL;VKL
TEGTIT;GIKLEGSLVGGKTTV;IKLEGSLVG;GKTTVKLTEGTITLT;VKLTEGTIT;GKVKIYLNDTTS
GST;IYLNDTTSG;GKVKIYLNDTTSGST;YLNDTTSGS;GKYELRATVDTVELK;YELRATVDT;GNK
IFVSKEKNSAGK;FVSKEKNSA;GNSSEIKDLAALKAA;IKDLAALKA;GSEVVKKQGSVIKES;VKKQ
GSVIK;GSLVGGKTTVKLTEG;VGGKTTVKL;GTITVQAYDTAGTKL;VQAYDTAGT;HNDQEIINSD
NTPKD;IINSDNTPK;IINSDNTPKDSKKDL;INSDNTPKD;IKLEGSLVGGKTTVK;IKLEGSLVG;INSD
NTPKDSKKDLT;INSDNTPKD;ITVQAYDTAGTKLEG;VQAYDTAGT;IYLNDTTSGSTKKTA;TTSGS
TKKT;KAVETLKNGIKLEGS;LKNGIKLEG;KDFVFLTDGTITVQA;FLTDGTITV;KDSKKDLTVLAEE
NS;SKKDLTVLA;KIFVSKEKNSAGKYE;FVSKEKNSA;KIYLNDTTSGSTKKT;YLNDTTSGS;KKTA
TWNETTNTLTI;WNETTNTLT;KLEGSLVGGKTTVKL;LVGGKTTVK;KNGIKLEGSLVGGKT;IKLEG
SLVG;KNSAGKYELRATVDT;AGKYELRAT;KQYLLVFALVLALIA;LLVFALVLA;KQYLLVFALVLALI
A;LVFALVLAL;KQYLLVFALVLALIA;VFALVLALI;KTATWNETTNTLTIS;WNETTNTLT;KTGSEVVK
KQGSVIK;VVKKQGSVI;KTKDFVFLTDGTITV;FVFLTDGTI;KTTVKLTEGTITLTR;LTEGTITLT;KV
KIYLNDTTSGSTK;IYLNDTTSG;KVKIYLNDTTSGSTK;YLNDTTSGS;KYELRATVDTVELKG;YEL
RATVDT;LAEENSVPLFNGNKI;ENSVPLFNG;LEGSLVGGKTTVKLT;VGGKTTVKL;LKNGIKLEG
SLVGGK;IKLEGSLVG;LKNGIKLEGSLVGGK;LKNGIKLEG;LLVFALVLALIACSQ;FALVLALIA;LLV
FALVLALIACSQ;LLVFALVLA;LNDTTSGSTKKTATW;TTSGSTKKT;LVFALVLALIACSQK;FALVL
ALIA;LVGGKTTVKLTEGTI;VGGKTTVKL;LVLALIACSQKGTEP;LVLALIACS;MKQYLLVFALVLALI
;LLVFALVLA;MKQYLLVFALVLALI;LVFALVLAL;NDQEIINSDNTPKDS;INSDNTPKD;NGIKLEGSL
VGGKTT;IKLEGSLVG;NGNKIFVSKEKNSAG;FVSKEKNSA;NKIFVSKEKNSAGKY;FVSKEKNSA;
NSAGKYELRATVDTV;YELRATVDT;NSSEIKDLAALKAAL;IKDLAALKA;NSVPLFNGNKIFVSK;VP
LFNGNKI;NTPKDSKKDLTVLAE;SKKDLTVLA;PKDSKKDLTVLAEEN;SKKDLTVLA;QAYDTAGTK
LEGNSS;YDTAGTKLE;QDGKVKIYLNDTTSG;KVKIYLNDT;QDGKVKIYLNDTTSG;VKIYLNDTT;
QEIINSDNTPKDSKK;INSDNTPKD;QYLLVFALVLALIAC;FALVLALIA;QYLLVFALVLALIAC;LLVF
ALVLA;QYLLVFALVLALIAC;LVFALVLAL;SAGKYELRATVDTVE;YELRATVDT;SEVVKKQGSVIK

ESY;VKKQGSVIK;SKKDLTVLAEENSVP;SKKDLTVLA;SLVGGKTTVKLTEGT;VGGKTTVKL;SSE
IKDLAALKAALK;IKDLAALKA;SVPLFNGNKIFVSKE;VPLFNGNKI;TATWNETTNTLTISA;WNETT
NTLT;TDGTITVQAYDTAGT;TITVQAYDT;TGSEVVKKQGSVIKE;VKKQGSVIK;TITVQAYDTAGT
KLE;VQAYDTAGT;TKAVETLKNGIKLEG;VETLKNGIK;TKDFVFLTDGTITVQ;FLTDGTITV;TKKTA
TWNETTNTLT;ATWNETTNT;TKKTATWNETTNTLT;TKKTATWNE;TLKNGIKLEGSLVGG;IKLEG
SLVG;TLKNGIKLEGSLVGG;LKNGIKLEG;TPKDSKKDLTVLAEE;SKKDLTVLA;TTVKLTEGTITLT
RE;LTEGTITLT;TVKLTEGTITLTREI;LTEGTITLT;TVQAYDTAGTKLEGN;VQAYDTAGT;TYDASN
KKTGSEVVK;NKKTGSEVV;VETLKNGIKLEGSLV;LKNGIKLEG;VFALVLALIACSQKG;LVLALIAC
S;VFLTDGTITVQAYDT;FLTDGTITV;VGGKTTVKLTEGTIT;VGGKTTVKL;VKIYLNDTTSGSTKK;Y
LNDTTSGS;VKLTEGTITLTREIE;LTEGTITLT;VLALIACSQKGTEPK;VLALIACSQ;VPLFNGNKIFV
SKEK;VPLFNGNKI;VQAYDTAGTKLEGNS;VQAYDTAGT;YDASNKKTGSEVVKK;NKKTGSEVV;
YELRATVDTVELKGV;YELRATVDT;YLLVFALVLALIACS;FALVLALIA;YLLVFALVLALIACS;LLVF
ALVLA;YLLVFALVLALIACS;LVFALVLAL;YLNDTTSGSTKKTAT;TTSGSTKKT

**<CAF34027 outer surface protein VlsE;Protein;Borrelia afzelii PKo>**

**Class 1**
KISSAIFL;SAIFLTAL;AIFLTALL;IFLTALLV;FLTALLVF;LTALLVFI;ALLVFINC;LLVFINCK;FINCKN
NA;FYESIINL;IINLGNGF;FIDVFNAF;FNAFSGLV;GLVADTFF;LVADTFFK;TFFKSDPK;KSDVKTY
F;FESISSTL;ESISSTLK;SISSTLKA;SSTLKATK;TLKATKGK;KLDELVSA;KASVESAV;SAVDEVS
K;KWLEEMIK;WLEEMIKA;MIKAAEEA;KAAEEAAK;DSAANHGA;SAANHGAK;DSVKGIAK;KGIA
KGIK;GIKGIVDA;GIVDAAGK;AGKALGEK;ALGEKGAL;ALKDVKAA;EANADAGK;FAGNANAA;NA
AVGAAA;GAAADIAK;IAKAAGAV;KAAGAVTA;AAGAVTAV;AVSGEQIL;VSGEQILK;ILKAIVEA;D
PANQAGK;AEEAKNPI;DDNGAAFK;AAFKDEMK;AFKDEMKK;KIAAAIVL;IAAAIVLR;AAIVLRGV;I
VLRGVAK;AKDGKFAV

KISSAIFLT;ISSAIFLTA;SSAIFLTAL;SAIFLTALL;AIFLTALLV;IFLTALLVF;FLTALLVFI;ALLVFINC
K;FINCKNNAV;AVGKGNDDK;SVKTFYESI;TFYESIINL;SIINLGNGF;IINLGNGFI;NLGNGFIDV;G
FIDVFNAF;DVFNAFSGL;FSGLVADTF;GLVADTFFK;LVADTFFKS;DTFFKSDPK;TFFKSDPKK;D
VKTYFESI;YFESISSTL;ISSTLKATK;STLKATKGK;TLKATKGKL;ATKGKLDEL;KLDELVSAK;EGG
SVKASV;SVKASVESA;SVESAVDEV;ESAVDEVSK;SAVDEVSKW;AVDEVSKWL;EVSKWLEEM;
WLEEMIKAA;EMIKAAEEA;MIKAAEEAA;KAAEEAAKV;DSAANHGAK;ANHGAKADK;SVKGIAKGI
;GIAKGIKGI;GIKGIVDAA;IVDAAGKAL;AAGKALGEK;ALGEKGALK;ALKDVKAAA;NADAGKLFA;
KLFAGNANA;FAGNANAAV;NANAAVGAA;DIAKAAGAV;KAAGAVTAV;TAVSGEQIL;AVSGEQIL
K;QILKAIVEA;ILKAIVEAA;AGKKAEEAK;AEEAKNPIA;EEAKNPIAA;NPIAAAIGT;TDDDNGAAF;G
AAFKDEMK;AAFKDEMKK;KIAAAIVLR;AAAIVLRGV;AIVLRGVAK;VAKDGKFAV

KISSAIFLTA;ISSAIFLTAL;SSAIFLTALL;SAIFLTALLV;AIFLTALLVF;IFLTALLVFI;FLTALLVFIN;TA
LLVFINCK;DSVKTFYESI;SVKTFYESII;KTFYESIINL;ESIINLGNGF;SIINLGNGFI;NGFIDVFNAF;
DVFNAFSGLV;AFSGLVADTF;FSGLVADTFF;SGLVADTFFK;GLVADTFFKS;ADTFFKSDPK;DTF
FKSDPKK;DPKKSDVKTY;KTYFESISST;TYFESISSTL;YFESISSTLK;SISSTLKATK;SSTLKATKG
K;ATKGKLDELV;KLDELVSAKK;SAKKGEGGSV;SVKASVESAV;ASVESAVDEV;ESAVDEVSKW;
SAVDEVSKWL;EVSKWLEEMI;VSKWLEEMIK;EEMIKAAEEA;MIKAAEEAAK;AEEAAKVGGT;KV
GGTGGDGK;AANHGAKADK;HGAKADKDSV;SVKGIAKGIK;GIAKGIKGIV;GIVDAAGKAL;DAAG
KALGEK;KALGEKGALK;EANADAGKLF;KLFAGNANAA;LFAGNANAAV;NANAAVGAAA;AVGAA
ADIAK;AKAAGAVTAV;TAVSGEQILK;AVSGEQILKA;QILKAIVEAA;AIVEAAGDPA;EAAGDPANQ
A;AEEAKNPIAA;EEAKNPIAAA;EAKNPIAAAI;GTDDDNGAAF;NGAAFKDEMK;GAAFKDEMKK;D
KIAAAIVLR;IAAAIVLRGV;AAIVLRGVAK;VLRGVAKDGK;GVAKDGKFAV;VAKDGKFAVK

KKISSAIFLTA;KISSAIFLTAL;ISSAIFLTALL;SSAIFLTALLV;SAIFLTALLVF;AIFLTALLVFI;FLTALL
VFINC;LTALLVFINCK;LLVFINCKNNA;LVFINCKNNAV;FINCKNNAVGK;YESIINLGNGF;ESIINLG
NGFI;IINLGNGFIDV;FIDVFNAFSGL;DVFNAFSGLVA;NAFSGLVADTF;AFSGLVADTFF;FSGLVA
DTFFK;VADTFFKSDPK;DPKKSDVKTYF;KTYFESISSTL;TYFESISSTLK;ESISSTLKATK;ISSTLK
ATKGK;KATKGKLDELV;KGKLDELVSAK;SAKKGEGGSVK;KASVESAVDEV;SVESAVDEVSK;E
SAVDEVSKWL;EVSKWLEEMIK;EEMIKAAEEAA;EMIKAAEEAAK;MIKAAEEAAKV;KIGDSAANH

GA;SAANHGAKADK;DSVKGIAKGIK;GIKGIVDAAGK;ALGEKGALKDV;DEANADAGKLF;KLFAG
NANAAV;FAGNANAAVGA;AAVGAAADIAK;AAADIAKAAGA;IAKAAGAVTAV;AVTAVSGEQIL;VT
AVSGEQILK;AVSGEQILKAI;AAGDPANQAGK;AEEAKNPIAAA;EEAKNPIAAAI;IGTDDDNGAAF;
GTDDDNGAAFK;NGAAFKDEMKK;AFKDEMKKSDK;KSDKIAAAIVL;KIAAAIVLRGV;IAAAIVLRG
VA;AAAIVLRGVAK;IVLRGVAKDGK;VLRGVAKDGKF;GVAKDGKFAVK

**Class 2**
ADAGKLFAGNANAAV;LFAGNANAA;ADIAKAAGAVTAVSG;IAKAAGAVT;ADTFFKSDPKKSDVK;
FFKSDPKKS;ADTFFKSDPKKSDVK;FKSDPKKSD;AEEAKNPIAAAIGTD;AKNPIAAAI;AGAVTAV
SGEQILKA;VTAVSGEQI;AGKLFAGNANAAVGA;FAGNANAAV;AGNANAAVGAAADIA;ANAAVG
AAA;AIFLTALLVFINCKN;FLTALLVFI;AIVLRGVAKDGKFAV;LRGVAKDGK;AKGIKGIVDAAGKAL;
IKGIVDAAG;AKAAGAVTAVSGEQI;AKAAGAVTA;ALLVFINCKNNAVGK;FINCKNNAV;ALLVFINC
KNNAVGK;VFINCKNNA;ANAAVGAAADIAKAA;AVGAAADIA;DAGKLFAGNANAAVG;FAGNANA
AV;DGKIGDSAANHGAKA;IGDSAANHG;DIAKAAGAVTAVSGE;AKAAGAVTA;DKIAAAIVLRGVA
KD;IAAAIVLRG;DSVKTFYESIINLGN;FYESIINLG;DSVKTFYESIINLGN;VKTFYESII;DTFFKSDPK
KSDVKT;FFKSDPKKS;DTFFKSDPKKSDVKT;FKSDPKKSD;DVFNAFSGLVADTFF;FNAFSGLVA
;DVFNAFSGLVADTFF;VFNAFSGLV;DVKTYFESISSTLKA;FESISSTLK;DVKTYFESISSTLKA;YF
ESISSTL;EAKNPIAAAIGTDDD;IAAAIGTDD;EEAKNPIAAAIGTDD;AKNPIAAAI;EQILKAIVEAAGD
PA;LKAIVEAAG;ESIINLGNGFIDVFN;IINLGNGFI;FAGNANAAVGAAADI;FAGNANAAV;FESISST
LKATKGKL;FESISSTLK;FIDVFNAFSGLVADT;FNAFSGLVA;FIDVFNAFSGLVADT;VFNAFSGLV
;FKSDPKKSDVKTYFE;FKSDPKKSD;FLTALLVFINCKNNA;FLTALLVFI;FNAFSGLVADTFFKS;F
NAFSGLVA;FYESIINLGNGFIDV;FYESIINLG;FYESIINLGNGFIDV;IINLGNGFI;GAVTAVSGEQIL
KAI;VTAVSGEQI;GDGKIGDSAANHGAK;IGDSAANHG;GEQILKAIVEAAGDP;LKAIVEAAG;GFID
VFNAFSGLVAD;FNAFSGLVA;GFIDVFNAFSGLVAD;VFNAFSGLV;GGDGKIGDSAANHGA;IGDS
AANHG;GIAKGIKGIVDAAGK;IKGIVDAAG;GIKGIVDAAGKALGE;IVDAAGKAL;GIVDAAGKALGE
KGA;IVDAAGKAL;GKIGDSAANHGAKAD;IGDSAANHG;GKLFAGNANAAVGAA;FAGNANAAV;G
LVADTFFKSDPKKS;DTFFKSDPK;GNANAAVGAAADIAK;AVGAAADIA;GNGFIDVFNAFSGLV;FI
DVFNAFS;GAAADIAKAAGAVTA;IAKAAGAVT;IAKGIKGIVDAAGKA;IKGIVDAAG;IAKAAGAVTA
VSGEQ;IAKAAGAVT;IDVFNAFSGLVADTF;FNAFSGLVA;IDVFNAFSGLVADTF;VFNAFSGLV;IF
LTALLVFINCKNN;FLTALLVFI;IKGIVDAAGKALGEK;IVDAAGKAL;ISSAIFLTALLVFIN;FLTALLVF
I;IVDAAGKALGEKGAL;IVDAAGKAL;IAAAIVLRGVAKDGK;AIVLRGVAK;IAAAIVLRGVAKDGK;IA
AAIVLRG;KAEEAKNPIAAAIGT;AKNPIAAAI;KAIVEAAGDPANQAG;AAGDPANQA;KDSVKTFYE
SIINLG;KTFYESIIN;KGIAKGIKGIVDAAG;IAKGIKGIV;KGIKGIVDAAGKALG;IVDAAGKAL;KGIVD
AAGKALGEKG;IVDAAGKAL;KISSAIFLTALLVFI;AIFLTALLV;KIAAAIVLRGVAKDG;IAAAIVLRG;K
KISSAIFLTALLVF;ISSAIFLTA;KKSDKIAAAIVLRGV;IAAAIVLRG;KLFAGNANAAVGAAA;FAGNA
NAAV;KSDKIAAAIVLRGVA;IAAAIVLRG;KSDVKTYFESISSTL;KSDVKTYFE;KTFYESIINLGNGFI
;FYESIINLG;KTFYESIINLGNGFI;YESIINLGN;KTYFESISSTLKATK;FESISSTLK;KTYFESISSTLK
ATK;YFESISSTL;KAAGAVTAVSGEQIL;VTAVSGEQI;LFAGNANAAVGAAAD;FAGNANAAV;LKAI
VEAAGDPANQA;LKAIVEAAG;LLVFINCKNNAVGKG;FINCKNNAV;LLVFINCKNNAVGKG;VFINC
KNNA;LTALLVFINCKNNAV;VFINCKNNA;LVADTFFKSDPKKSD;DTFFKSDPK;LVADTFFKSDPK
KSD;FFKSDPKKS;LVFINCKNNAVGKGN;CKNNAVGKG;LVFINCKNNAVGKGN;FINCKNNAV;M
KKISSAIFLTALLV;MKKISSAIF;MKKSDKIAAAIVLRG;DKIAAAIVL;NADAGKLFAGNANAA;AGKLF
AGNA;NANAAVGAAADIAKA;AVGAAADIA;NGFIDVFNAFSGLVA;FIDVFNAFS;NGFIDVFNAFSG
LVA;VFNAFSGLV;NAAVGAAADIAKAAG;AVGAAADIA;SAIFLTALLVFINCK;FLTALLVFI;SDKIAA
AIVLRGVAK;IAAAIVLRG;SDVKTYFESISSTLK;YFESISSTL;SGEQILKAIVEAAGD;LKAIVEAAG;S
IINLGNGFIDVFNA;IINLGNGFI;SSAIFLTALLVFINC;FLTALLVFI;SVKTFYESIINLGNG;FYESIINL
G;SVKTFYESIINLGNG;YESIINLGN;TALLVFINCKNNAVG;FINCKNNAV;TALLVFINCKNNAVG;V
FINCKNNA;TFFKSDPKKSDVKTY;FKSDPKKSD;TFYESIINLGNGFID;FYESIINLG;TFYESIINLGN
GFID;IINLGNGFI;TFYESIINLGNGFID;YESIINLGN;TGGDGKIGDSAANHG;GKIGDSAAN;TYFESI
SSTLKATKG;FESISSTLK;TYFESISSTLKATKG;YFESISSTL;VADTFFKSDPKKSDV;FFKSDPKK
S;VADTFFKSDPKKSDV;FKSDPKKSD;VFINCKNNAVGKGND;CKNNAVGKG;VFNAFSGLVADTF
FK;VFNAFSGLV;VGAAADIAKAAGAVT;DIAKAAGAV;VKTFYESIINLGNGF;FYESIINLG;VKTFYE
SIINLGNGF;YESIINLGN;VKTYFESISSTLKAT;FESISSTLK;VKTYFESISSTLKAT;YFESISSTL;VS
GEQILKAIVEAAG;ILKAIVEAA;YESIINLGNGFIDVF;IINLGNGFI;YFESISSTLKATKGK;FESISSTL
K;AAADIAKAAGAVTAV;IAKAAGAVT;AAAIVLRGVAKDGKF;LRGVAKDGK;AAAIVLRGVAKDGK

F;VLRGVAKDG;AADIAKAAGAVTAVS;IAKAAGAVT;AAGAVTAVSGEQILK;VTAVSGEQI;AAIVLR
GVAKDGKFA;LRGVAKDGK;AAIVLRGVAKDGKFA;VLRGVAKDG

## C) Borrelia garinii MHC class 1 & 2 epitopes

### <CAH10086 CRASP-1;Protein;Borrelia garinii>

### Class 1
KTKLNIIK;NIIKLNIL;KLNILTTI;ILTTILTL;LTTILTLI;TILTLICI;TLICISCA;LICISCAV;CISCAVDK;NT
KDFENK;FENKSQDL;EPSNTKNK;KETIISKL;TIISKLEK;LEKMIKDL;EIAKITNT;ITNTQIDF;QIDFL
ENF;TISEDIEM;ISEDIEMK;DIEMKIKR;KIKRIIYS;KRIIYSSL;IIYSSLNY;YSSLNYEI;SLNYEIEK;EI
EKINTL;KINTLKEI;TLKEILDK;KEILDKLY;EILDKLYK;KLYKNHKY;LYKNHKYK;HKYKTTAR;TTAR
NFIL;NFILNISI;FILNISIK;ILNISIKI;SIKIQFQI;IQFQIENA;QFQIENAL;FQIENALE;QIENALEL;ENAL
ELMK;IEDASEIL;EILNQERY;NQERYEIL;QERYEILL;ERYEILLK;YEILLKHV;NLKQKFEK;KILNETI
K;ILNETIKA;ETIKAYNQ;LAHMDENY;HMDENYKE;NYKEFDPL;KEFDPLNL

KLNIIKLNI;IKLNILTTI;KLNILTTIL;NILTTILTL;ILTTILTLI;TTILTLICI;ILTLICISC;TLICISCAV;CISCA
VDKI;ESKSKTNTK;NSKETIISK;ETIISKLEK;TIISKLEKM;ISKLEKMIK;MIKDLEDQK;EIAKITNTQ;K
ITNTQIDF;ITNTQIDFL;TQIDFLENF;QIDFLENFK;DTISEDIEM;TISEDIEMK;EDIEMKIKR;EMKIKR
IIY;KIKRIIYSS;RIIYSSLNY;IYSSLNYEI;SSLNYEIEK;SLNYEIEKI;YEIEKINTL;EIEKINTLK;NTLKEI
LDK;TLKEILDKL;LDKLYKNHK;KLYKNHKYK;NHKYKTTAR;KYKTTARNF;KTTARNFIL;NFILNISI
K;FILNISIKI;ISIKIQFQI;KIQFQIENA;IQFQIENAL;FQIENALEL;QIENALELM;ALELMKEEI;LMKEEI
EDA;EEIEDASEI;EIEDASEIL;ASEILNQER;SEILNQERY;ILNQERYEI;LLKHVEPSL;KHVEPSLNL;
HVEPSLNLK;EPSLNLKQK;NLKQKFEKI;KILNETIKA;ILNETIKAY;AYNQDLDNI;QLAHMDENY;LA
HMDENYK;HMDENYKEF;YKEFDPLNL;EFDPLNLDY

KLNIIKLNIL;IIKLNILTTI;KLNILTTILT;NILTTILTLI;ILTTILTLIC;ILTLICISCA;LTLICISCAV;LICISCA
VDK;AVDKIDPESK;KTNTKDFENK;SQDLEPSNTK;KNKDLEPLEK;PLEKNSKETI;ETIISKLEKM;T
IISKLEKMI;IISKLEKMIK;KMIKDLEDQK;EIAKITNTQI;KITNTQIDFL;NTQIDFLENF;TQIDFLENFK;
DTISEDIEMK;TISEDIEMKI;IEMKIKRIIY;KIKRIIYSSL;KRIIYSSLNY;IIYSSLNYEI;YSSLNYEIEK;N
YEIEKINTL;ILDKLYKNHK;KLYKNHKYT;KNHKYKTTAR;KYKTTARNFI;YKTTARNFIL;TTARNFI
LNI;NFILNISIKI;NISIKIQFQI;QFQIENALEL;FQIENALELM;QIENALELMK;NALELMKEEI;EEIEDA
SEIL;DASEILNQER;ASEILNQERY;EILNQERYEI;ILNQERYEIL;NQERYEILLK;ILLKHVEPSL;EPS
LNLKQKF;SLNLKQKFEK;NLKQKFEKIL;KILNETIKAY;ETIKAYNQDL;KAYNQDLDNI;AYNQDLDN
IK;KSNEDQLAHM;QLAHMDENYK;AHMDENYKEF;NYKEFDPLNL;KEFDPLNLDY

KLNIIKLNILT;NIIKLNILTTI;IIKLNILTTIL;KLNILTTILTL;ILTTILTLICI;ILTLICISCAV;TLICISCAVDK;
CAVDKIDPESK;KSQDLEPSNTK;EPLEKNSKETI;ETIISKLEKMI;TIISKLEKMIK;NTQIDFLENFK;T
QIDFLENFKS;DTISEDIEMKI;TISEDIEMKIK;ISEDIEMKIKR;DIEMKIKRIIY;MKIKRIIYSSL;RIIYSS
LNYEI;IYSSLNYEIEK;NYEIEKINTLK;IEKINTLKEIL;KINTLKEILDK;NTLKEILDKLY;TLKEILDKLYK
;EILDKLYKNHK;ILDKLYKNHKY;KLYKNHKYTT;KYKTTARNFIL;TTARNFILNIS;FILNISIKIQF;IL
NISIKIQFQ;SIKIQFQIENA;IQFQIENALEL;FQIENALELMK;KEEIEDASEIL;EEIEDASEILN;EDASE
ILNQER;DASEILNQERY;SEILNQERYEI;EILNQERYEIL;ILNQERYEILL;EILLKHVEPSL;LLKHVE
PSLNL;HVEPSLNLKQK;VEPSLNLKQKF;PSLNLKQKFEK;SLNLKQKFEKI;KFEKILNETIK;EKILN
ETIKAY;ILNETIKAYNQ;NETIKAYNQDL;KAYNQDLDNIK;LAHMDENYKEF

### Class 2
ARNFILNISIKIQFQ;FILNISIKI;AYNQDLDNIKSNEDQ;LDNIKSNED;DFLENFKSEPHDTIS;LENFK
SEPH;DIEMKIKRIIYSSLN;MKIKRIIYS;DKLYKNHKYKTTARN;YKNHKYKTT;DLDNIKSNEDQLAH
M;IKSNEDQLA;EDIEMKIKRIIYSSL;MKIKRIIYS;EIEKINTLKEILDKL;INTLKEILD;EILDKLYKNHKY
KTT;LDKLYKNHK;EILDKLYKNHKYKTT;LYKNHKYKT;EILLKHVEPSLNLKQ;LKHVEPSLN;EMKI
KRIIYSSLNYE;MKIKRIIYS;ENKSQDLEPSNTKNK;QDLEPSNTK;ERYEILLKHVEPSLN;YEILLKH
VE;ETIKAYNQDLDNIKS;IKAYNQDLD;FILNISIKIQFQIEN;LNISIKIQF;HKYKTTARNFILNIS;YKTT
ARNFI;HMDENYKEFDPLNLD;YKEFDPLNL;IDFLENFKSEPHDTI;LENFKSEPH;IEKINTLKEILDK
LY;INTLKEILD;IEMKIKRIIYSSLNY;MKIKRIIYS;IIKLNILTTILTLIC;ILTTILTLI;IIKLNILTTILTLIC;LNI

LTTILT;IKIQFQIENALELMK;FQIENALEL;IKLNILTTILTLICI;LNILTTILT;ILDKLYKNHKYKTTA;YK
NHKYKTT;ILLKHVEPSLNLKQK;LKHVEPSLN;ILNISIKIQFQIENA;LNISIKIQF;ILTTILTLICISCAV;I
LTTILTLI;ILTTILTLICISCAV;LTLICISCA;IQFQIENALELMKEE;FQIENALEL;ISEDIEMKIKRIIYS;IE
MKIKRII;ISIKIQFQIENALEL;IKIQFQIEN;ISIKIQFQIENALEL;IQFQIENAL;KAYNQDLDNIKSNED;
YNQDLDNIK;KEILDKLYKNHKYKT;LDKLYKNHK;KIQFQIENALELMKE;FQIENALEL;KKTKLNIIK
LNILTT;LNIIKLNIL;KLNIIKLNILTTILT;IIKLNILTT;KLNIIKLNILTTILT;IKLNILTTI;KLNIIKLNILTTILT;
KLNILTTIL;KLNILTTILTLICIS;LNILTTILT;KLYKNHKYKTTARNF;YKNHKYKTT;KNHKYKTTARNF
ILN;YKTTARNFI;KSQDLEPSNTKNKDL;LEPSNTKNK;KTKLNIIKLNILTTI;IIKLNILTT;KTKLNIIKLN
ILTTI;LNIIKLNIL;KTTARNFILNISIKI;ARNFILNIS;KYKTTARNFILNISI;ARNFILNIS;KYKTTARNFIL
NISI;YKTTARNFI;LDKLYKNHKYKTTAR;YKNHKYKTT;LDNIKSNEDQLAHMD;IKSNEDQLA;LNII
KLNILTTILTL;LNILTTILT;LNILTTILTLICISC;LNILTTILT;LNISIKIQFQIENAL;LNISIKIQF;LNYEIEKI
NTLKEIL;IEKINTLKE;LNYEIEKINTLKEIL;YEIEKINTL;LTTILTLICISCAVD;LTLICISCA;LYKNHKY
KTTARNFI;KYKTTARNF;LYKNHKYKTTARNFI;NHKYKTTAR;LYKNHKYKTTARNFI;YKNHKYKT
T;MDENYKEFDPLNLDY;KEFDPLNLD;MKIKRIIYSSLNYEI;KRIIYSSLN;MKIKRIIYSSLNYEI;MKI
KRIIYS;MKKTKLNIIKLNILT;LNIIKLNIL;NFILNISIKIQFQIE;LNISIKIQF;NHKYKTTARNFILNI;YKTT
ARNFI;NIIKLNILTTILTLI;LNILTTILT;NILTTILTLICISCA;ILTTILTLI;NILTTILTLICISCA;LTTILTLIC;
NKSQDLEPSNTKNKD;LEPSNTKNK;NQDLDNIKSNEDQLA;LDNIKSNED;NTQIDFLENFKSEPH;
FLENFKSEP;NTQIDFLENFKSEPH;IDFLENFKS;NYEIEKINTLKEILD;EKINTLKEI;NYEIEKINTLK
EILD;IEKINTLKE;QDLDNIKSNEDQLAH;IKSNEDQLA;QDLEPSNTKNKDLEP;LEPSNTKNK;QIDF
LENFKSEPHDT;LENFKSEPH;RNFILNISIKIQFQI;FILNISIKI;RYEILLKHVEPSLNL;LKHVEPSLN;
SEDIEMKIKRIIYSS;MKIKRIIYS;SIKIQFQIENALELM;FQIENALEL;SLNYEIEKINTLKEI;IEKINTLK
E;SLNYEIEKINTLKEI;YEIEKINTL;SQDLEPSNTKNKDLE;LEPSNTKNK;SSLNYEIEKINTLKE;YEI
EKINTL;TARNFILNISIKIQF;FILNISIKI;TKLNIIKLNILTTIL;IIKLNILTT;TKLNIIKLNILTTIL;IKLNILTTI
;TQIDFLENFKSEPHD;LENFKSEPH;TTARNFILNISIKIQ;FILNISIKI;TTILTLICISCAVDK;LTLICISC
A;YEIEKINTLKEILDK;IEKINTLKE;YEIEKINTLKEILDK;INTLKEILD;YEILLKHVEPSLNLK;LKHVEP
SLN;YKNHKYKTTARNFIL;YKNHKYKTT;YKNHKYKTTARNFIL;YKTTARNFI;YKTTARNFILNISIK;
ARNFILNIS;YNQDLDNIKSNEDQL;LDNIKSNED

**<AAU07022 4-alpha-glucanotransferase;Protein;Borrelia garinii PBi>**

**Class 1**
KIRINLNL;RINLNLKR;NLKRKSGI;ILLNISSL;NISSLPSK;SLPSKYGI;GAYKFIDF;AYKFIDFL;KFID
FLFA;FIDFLFAS;FLFASSQS;LFASSQSY;FASSQSYW;SQSYWQMF;QSYWQMFA;SYWQMFAY
;WQMFAYSP;QMFAYSPI;FAYSPIDF;YSPIDFTR;TRSPPYSI;RSPPYSIF;PPYSIFSA;PYSIFSAF;
YSIFSAFA;FSAFAGNV;SAFAGNVY;AFAGNVYY;FAGNVYYI;NVYYIDLE;YYIDLEAL;FIDSDLNL;
LLKENETR;NETRYSDL;ETRYSDLK;RYSDLKKL;DLKKLSFK;SFKDKFLK;KFLKEAAL;KEAALNFI
;AALNFINR;ALNFINRA;NRASVDEV;RASVDEVR;SVDEVRSF;EVRSFEKF;RSFEKFKK;KKSSY
WLL;SSYWLLDF;WLLDFASF;LLDFASFV;DFASFVAF;FASFVAFK;FVAFKEYF;VAFKEYFL;AFK
EYFLK;EYFLKDSR;FLKDSRDA;DSRDAFNV;SRDAFNVL;AFNVLFDR;VLFDRGIL;GILIRNEK;NE
KDLFKL;DLFKLRNI;KLRNILSK;ILSKEIKV;KEIKVQEV;EIKVQEVL;KVQEVLQY;VQEVLQYF;QEV
LQYFF;EVLQYFFF;LQYFFFSQ;QYFFFSQF;FFSQFQAL;FSQFQALK;SQFQALKR;FQALKRYA;
RYANDKGI;ELVMNLPL;LVMNLPLF;VMNLPLFI;NLPLFIAY;LPLFIAYD;IAYDSADV;AYDSADVW;
DVWAHQKY;VWAHQKYF;WAHQKYFK;HQKYFKLR;KLRFDASK;GISPDYFL;QAWDSPAY;SPA
YSWNV;AYSWNVLK;NVLKNVKY;KYEWWAKR;YEWWAKRI;WWAKRIGI;WAKRIGIL;KRIGILRK;
ILRKYIDV;KYIDVIKI;DVIKIDHF;VIKIDHFR;GFVSTWEV;WEVGVGEA;EVGVGEAY;EAYAFNGL;
AYAFNGLW;YAFNGLWV;AFNGLWVK;RDFFNFIL;NFILNEIK;ILNEIKDL;EIKDLKIW;IKDLKIWV;V
EDFENDL;FENDLEDV;DLEDVSRL;DVSRLRDF;RLRDFFNF;FFNFPGMR;GMRIMKLA;MRIMKL
AF;IMKLAFDF;FDSDNQNL;NQNLPHNY;YIKNCIVY;NCIVYTGI;GIGDNSTI;NSTIREFV;IREFVNS
L;FVNSLDDL;NSLDDLHK;SLDDLHKK;YIFDYLNT;YLNTNEDF;NTNEDFVV;FVVWDMIR;MSSVS
DSV;SVSDSVII;VIIPMQDY;PMQDYINL;YINLGNEF;NLGNEFRA;EFRANIPK;NTLNNWIF;TLNNW
IFR;TLSKNISF;KNISFITR;NISFITRL;ISFITRLY;FITRLYGR

KYKKIRINL;KIRINLNLK;IRINLNLKR;RINLNLKRK;NLKRKSGIL;GILLNISSL;NISSLPSKY;KYGIG
DLGK;DLGKGAYKF;GAYKFIDFL;AYKFIDFLF;YKFIDFLFA;FIDFLFASS;FLFASSQSY;LFASSQS
YW;FASSQSYWQ;ASSQSYWQM;SQSYWQMFA;QSYWQMFAY;SYWQMFAYS;WQMFAYSPI;

MFAYSPIDF;FAYSPIDFT;AYSPIDFTR;FTRSPPYSI;SPPYSIFSA;PPYSIFSAF;FSAFAGNVY;SA
FAGNVYY;AFAGNVYYI;NVYYIDLEA;VYYIDLEAL;KFIDSDLNL;FIDSDLNLL;LLKENETRY;ETRY
SDLKK;YSDLKKLSF;KLSFKDKFL;LSFKDKFLK;FLKEAALNF;EAALNFINR;AALNFINRA;ASVDE
VRSF;DEVRSFEKF;EVRSFEKFK;RSFEKFKKK;KFKKKSSYW;KSSYWLLDF;SSYWLLDFA;YWL
LDFASF;WLLDFASFV;LLDFASFVA;LDFASFVAF;DFASFVAFK;FASFVAFKE;ASFVAFKEY;SFV
AFKEYF;FVAFKEYFL;VAFKEYFLK;KEYFLKDSR;FLKDSRDAF;DAFNVLFDR;VLFDRGILI;RGILI
RNEK;ILIRNEKDL;DLFKLRNIL;RNILSKEIK;NILSKEIKV;KEIKVQEVL;IKVQEVLQY;KVQEVLQYF
;VQEVLQYFF;QEVLQYFFF;EVLQYFFFS;VLQYFFFSQ;LQYFFFSQF;FFFSQFQAL;FFSQFQAL
K;FSQFQALKR;SQFQALKRY;ALKRYANDK;YANDKGIEL;ANDKGIELV;IELVMNLPL;ELVMNLPL
F;LVMNLPLFI;VMNLPLFIA;MNLPLFIAY;LPLFIAYDS;PLFIAYDSA;FIAYDSADV;IAYDSADVW;S
ADVWAHQK;DVWAHQKYF;VWAHQKYFK;HQKYFKLRF;ASKDKVAGI;KVAGISPDY;VAGISPDY
F;YFLEQEQAW;EQAWDSPAY;QAWDSPAYS;AWDSPAYSW;DSPAYSWNV;SPAYSWNVL;PAY
SWNVLK;YSWNVLKNV;SWNVLKNVK;NVKYEWWAK;KYEWWAKRI;WWAKRIGIL;WAKRIGILR;
AKRIGILRK;KRIGILRKY;GILRKYIDV;ILRKYIDVI;DVIKIDHFR;KIDHFRGFV;RGFVSTWEV;FVST
WEVGV;WEVGVGEAY;EVGVGEAYA;VGVGEAYAF;GEAYAFNGL;EAYAFNGLW;AYAFNGLWV
;YAFNGLWVK;GLWVKSPGR;WVKSPGRDF;SPGRDFFNF;GRDFFNFIL;FFNFILNEI;FNFILNEIK
;FILNEIKDL;ILNEIKDLK;EIKDLKIWV;WVEDFENDL;DLEDVSRLR;DVSRLRDFF;SRLRDFFNF;R
LRDFFNFP;DFFNFPGMR;NFPGMRIMK;FPGMRIMKL;GMRIMKLAF;RIMKLAFDF;NYIKNCIVY;Y
IKNCIVYT;TGIGDNSTI;DNSTIREFV;TIREFVNSL;EFVNSLDDL;NSLDDLHKK;SLDDLHKKY;HKK
YIFDYL;YIFDYLNTN;DYLNTNEDF;YLNTNEDFV;NTNEDFVVW;EDFVVWDMI;DFVVWDMIR;FV
VWDMIRS;VVWDMIRSA;MIRSAMSSV;AMSSVSDSV;MSSVSDSVI;SVIIPMQDY;VIIPMQDYI;IP
MQDYINL;DYINLGNEF;YINLGNEFR;RANIPKNTL;IPKNTLNNW;NTLNNWIFR;TLNNWIFRL;RLL
ESDLDA;LLESDLDAT;LESDLDATL;SDLDATLSK;ATLSKNISF;TLSKNISFI;NISFITRLY;SFITRLY
GR

KYKKIRINLN;KIRINLNLKR;NLNLKRKSGI;NLKRKSGILL;SGILLNISSL;ILLNISSLPS;LLNISSLPS
K;LPSKYGIGDL;DLGKGAYKFI;GAYKFIDFLF;FIDFLFASSQ;DFLFASSQSY;FLFASSQSYW;FAS
SQSYWQM;ASSQSYWQMF;SQSYWQMFAY;YWQMFAYSPI;QMFAYSPIDF;FAYSPIDFTR;PID
FTRSPPY;FTRSPPYSIF;RSPPYSIFSA;SPPYSIFSAF;PPYSIFSAFA;SIFSAFAGNV;IFSAFAGNV
Y;FSAFAGNVYY;SAFAGNVYYI;FAGNVYYIDL;NVYYIDLEAL;YYIDLEALDK;YIDLEALDKF;ALDK
FIDSDL;KFIDSDLNLL;FIDSDLNLLK;KENETRYSDL;ETRYSDLKKL;RYSDLKKLSF;YSDLKKLSF
K;KLSFKDKFLK;KFLKEAALNF;FLKEAALNFI;EAALNFINRA;ALNFINRASV;FINRASVDEV;RASV
DEVRSF;SVDEVRSFEK;EVRSFEKFKK;KFKKKSSYWL;KSSYWLLDFA;SYWLLDFASF;YWLLD
FASFV;WLLDFASFVA;LLDFASFVAF;LDFASFVAFK;DFASFVAFKE;FASFVAFKEY;ASFVAFKE
YF;SFVAFKEYFL;FVAFKEYFLK;YFLKDSRDAF;RDAFNVLFDR;NVLFDRGILI;VLFDRGILIR;ILIR
NEKDLF;LIRNEKDLFK;LFKLRNILSK;KLRNILSKEI;EIKVQEVLQY;IKVQEVLQYF;KVQEVLQYFF
;VQEVLQYFFF;QEVLQYFFFS;EVLQYFFFSQ;VLQYFFFSQF;YFFFSQFQAL;FFFSQFQALK;FF
SQFQALKR;SQFQALKRYA;RYANDKGIEL;YANDKGIELV;IELVMNLPLF;ELVMNLPLFI;LVMNLP
LFIA;VMNLPLFIAY;LPLFIAYDSA;FIAYDSADVW;DSADVWAHQK;SADVWAHQKY;DVWAHQKY
FK;VWAHQKYFKL;WAHQKYFKLR;AHQKYFKLRF;YFKLRFDASK;KLRFDASKDK;KVAGISPDYF
;GISPDYFLEQ;DYFLEQEQAW;QEQAWDSPAY;QAWDSPAYSW;WDSPAYSWNV;SPAYSWNV
LK;AYSWNVLKNV;YSWNVLKNVK;SWNVLKNVKY;KNVKYEWWAK;NVKYEWWAKR;YEWWAK
RIGI;WWAKRIGILR;WAKRIGILRK;KRIGILRKYI;ILRKYIDVIK;YIDVIKIDHF;VIKIDHFRGF;FRGFV
STWEV;STWEVGVGEA;WEVGVGEAYA;EVGVGEAYAF;GEAYAFNGLW;EAYAFNGLWV;AYAF
NGLWVK;LWVKSPGRDF;WVKSPGRDFF;KSPGRDFFNF;SPGRDFFNFI;DFFNFILNEI;FFNFILN
EIK;NFILNEIKDL;FILNEIKDLK;ILNEIKDLKI;NEIKDLKIWV;IWVEDFENDL;VSRLRDFFNF;RLRDF
FNFPG;FNFPGMRIMK;FPGMRIMKLA;NQNLPHNYIK;LPHNYIKNCI;HNYIKNCIVY;YTGIGDNSTI
;TGIGDNSTIR;STIREFVNSL;REFVNSLDDL;FVNSLDDLHK;SLDDLHKKYI;DLHKKYIFDY;YIFDY
LNTNE;YLNTNEDFVV;EDFVVWDMIR;FVVWDMIRSA;VVWDMIRSAM;DMIRSAMSSV;SAMSSV
SDSV;AMSSVSDSVI;SVSDSVIIPM;DSVIIPMQDY;SVIIPMQDYI;IIPMQDYINL;DYINLGNEFR;YI
NLGNEFRA;NLGNEFRANI;FRANIPKNTL;IPKNTLNNWI;KNTLNNWIFR;NTLNNWIFRL;TLNNWI
FRLL;WIFRLLESDL;RLLESDLDAT;LLESDLDATL;ESDLDATLSK;DLDATLSKNI;ATLSKNISFI;TL
SKNISFIT;LSKNISFITR;ISFITRLYGR

KYKKIRINLNL;KIRINLNLKRK;NLNLKRKSGIL;ILLNISSLPSK;LLNISSLPSKY;NISSLPSKYGI;SLP
SKYGIGDL;YGIGDLGKGAY;GIGDLGKGAYK;KGAYKFIDFLF;IDFLFASSQSY;DFLFASSQSYW;
FLFASSQSYWQ;FASSQSYWQMF;SSQSYWQMFAY;SQSYWQMFAYS;SYWQMFAYSPI;WQM
FAYSPIDF;QMFAYSPIDFT;MFAYSPIDFTR;SPIDFTRSPPY;DFTRSPPYSIF;TRSPPYSIFSA;RS
PPYSIFSAF;YSIFSAFAGNV;SIFSAFAGNVY;IFSAFAGNVYY;FSAFAGNVYYI;AFAGNVYYIDL;V
YYIDLEALDK;YYIDLEALDKF;YIDLEALDKFI;KFIDSDLNLLK;DLNLLKENETR;KENETRYSDLK;N
ETRYSDLKKL;TRYSDLKKLSF;RYSDLKKLSFK;KKLSFKDKFLK;FKDKFLKEAAL;KFLKEAALNFI
;EAALNFINRAS;AALNFINRASV;FINRASVDEVR;ASVDEVRSFEK;SVDEVRSFEKF;EVRSFEKF
KKK;KFKKKSSYWLL;SSYWLLDFASF;SYWLLDFASFV;WLLDFASFVAF;LLDFASFVAFK;DFAS
FVAFKEY;FASFVAFKEYF;SFVAFKEYFLK;AFKEYFLKDSR;EYFLKDSRDAF;FLKDSRDAFNV;D
AFNVLFDRGI;NVLFDRGILIR;VLFDRGILIRN;ILIRNEKDLFK;EKDLFKLRNIL;DLFKLRNILSK;KLR
NILSKEIK;ILSKEIKVQEV;KEIKVQEVLQY;EIKVQEVLQYF;KVQEVLQYFFF;EVLQYFFFSQF;VL
QYFFFSQFQ;LQYFFFSQFQA;QYFFFSQFQAL;YFFFSQFQALK;FFFSQFQALKR;FFSQFQALK
RY;ALKRYANDKGI;KRYANDKGIEL;RYANDKGIELV;YANDKGIELVM;IELVMNLPLFI;ELVMNLP
LFIA;LVMNLPLFIAY;NLPLFIAYDSA;LPLFIAYDSAD;PLFIAYDSADV;LFIAYDSADVW;FIAYDSAD
VWA;IAYDSADVWAH;DSADVWAHQKY;DVWAHQKYFKL;VWAHQKYFKLR;WAHQKYFKLRF;K
YFKLRFDASK;KLRFDASKDKV;KVAGISPDYFL;EQEQAWDSPAY;AWDSPAYSWNV;DSPAYSW
NVLK;PAYSWNVLKNV;AYSWNVLKNVK;YSWNVLKNVKY;VLKNVKYEWWA;KNVKYEWWAKR;
KYEWWAKRIGI;YEWWAKRIGIL;EWWAKRIGILR;WWAKRIGILRK;RIGILRKYIDV;GILRKYIDVIK;
ILRKYIDVIKI;KYIDVIKIDHF;YIDVIKIDHFR;DVIKIDHFRGF;VIKIDHFRGFV;KIDHFRGFVST;HFR
GFVSTWEV;STWEVGVGEAY;WEVGVGEAYAF;GVGEAYAFNGL;GEAYAFNGLWV;EAYAFNGL
WVK;LWVKSPGRDFF;SPGRDFFNFIL;DFFNFILNEIK;NFILNEIKDLK;FILNEIKDLKI;ILNEIKDLKI
W;KIWVEDFENDL;DFENDLEDVSR;FENDLEDVSRL;DVSRLRDFFNF;RLRDFFNFPGM;DFFNF
PGMRIM;FFNFPGMRIMK;FPGMRIMKLAF;GMRIMKLAFDF;KLAFDFDSDNQ;DSDNQNLPHNY;
NLPHNYIKNCI;LPHNYIKNCIV;YIKNCIVYTGI;VYTGIGDNSTI;YTGIGDNSTIR;IGDNSTIREFV;EF
VNSLDDLHK;FVNSLDDLHKK;SLDDLHKKYIF;DLHKKYIFDYL;YIFDYLNTNED;IFDYLNTNEDF;F
DYLNTNEDFV;DYLNTNEDFVV;YLNTNEDFVVW;NTNEDFVVWDM;FVVWDMIRSAM;WDMIRS
AMSSV;RSAMSSVSDSV;SAMSSVSDSVI;AMSSVSDSVII;SSVSDSVIIPM;SVSDSVIIPMQ;DSVII
PMQDYI;VIIPMQDYINL;MQDYINLGNEF;QDYINLGNEFR;LGNEFRANIPK;NIPKNTLNNWI;IPKN
TLNNWIF;NTLNNWIFRLL;TLNNWIFRLLE;NWIFRLLESDL;RLLESDLDATL;DATLSKNISFI;TLSK
NISFITR;NISFITRLYGR

## Class 2

AFDFDSDNQNLPHNY;FDSDNQNLP;AFKEYFLKDSRDAFN;FLKDSRDAF;AFKEYFLKDSRDAF
N;YFLKDSRDA;AFNGLWVKSPGRDFF;FNGLWVKSP;AFNVLFDRGILIRNE;FDRGILIRN;AFNVL
FDRGILIRNE;VLFDRGILI;AGNVYYIDLEALDKF;YYIDLEALD;AHQKYFKLRFDASKD;FKLRFDAS
K;AKRIGILRKYIDVIK;AKRIGILRK;AKRIGILRKYIDVIK;IGILRKYID;AKRIGILRKYIDVIK;ILRKYIDV
I;ALDKFIDSDLNLLKE;FIDSDLNLL;ALNFINRASVDEVRS;FINRASVDE;ANIPKNTLNNWIFRL;PK
NTLNNWI;ASSQSYWQMFAYSPI;YWQMFAYSP;ATLSKNISFITRLYG;LSKNISFIT;AWDSPAYS
WNVLKNV;DSPAYSWNV;AYAFNGLWVKSPGRD;FNGLWVKSP;AYAFNGLWVKSPGRD;GLWV
KSPGR;AYKFIDFLFASSQSY;FIDFLFASS;AYKFIDFLFASSQSY;IDFLFASSQ;AYSPIDFTRSPPY
SI;IDFTRSPPY;AYSPIDFTRSPPYSI;YSPIDFTRS;AYSWNVLKNVKYEWW;WNVLKNVKY;CIVYT
GIGDNSTIRE;VYTGIGDNS;CIVYTGIGDNSTIRE;YTGIGDNST;DAFNVLFDRGILIRN;FNVLFDR
GI;DATLSKNISFITRLY;LSKNISFIT;DDLHKKYIFDYLNTN;DDLHKKYIF;DFDSDNQNLPHNYIK;F
DSDNQNLP;DFFNFILNEIKDLKI;ILNEIKDLK;DFFNFPGMRIMKLAF;FPGMRIMKL;DFLFASSQSY
WQMFA;FASSQSYWQ;DFLFASSQSYWQMFA;LFASSQSYW;DFTRSPPYSIFSAFA;FTRSPPYS
I;DFVVWDMIRSAMSSV;FVVWDMIRS;DFVVWDMIRSAMSSV;WDMIRSAMS;DHFRGFVSTWEV
GVG;FRGFVSTWE;DKFLKEAALNFINRA;LKEAALNFI;DKGIELVMNLPLFIA;LVMNLPLFI;DLDAT
LSKNISFITR;LSKNISFIT;DLEALDKFIDSDLNL;LEALDKFID;DLFKLRNILSKEIKV;FKLRNILSK;DL
FKLRNILSKEIKV;LFKLRNILS;DLFKLRNILSKEIKV;LRNILSKEI;DLHKKYIFDYLNTNE;YIFDYLNT
N;DLNLLKENETRYSDL;LKENETRYS;DMIRSAMSSVSDSVI;IRSAMSSVS;DNSTIREFVNSLDDL
;IREFVNSLD;DRGILIRNEKDLFKL;LIRNEKDLF;DSDLNLLKENETRYS;LNLLKENET;DSPAYSW
NVLKNVKY;AYSWNVLKN;DSPAYSWNVLKNVKY;SWNVLKNVK;DSPAYSWNVLKNVKY;YSWN
VLKNV;DVWAHQKYFKLRFDA;WAHQKYFKL;DYFLEQEQAWDSPAY;YFLEQEQAW;EAYAFNG
LWVKSPGR;FNGLWVKSP;EAYAFNGLWVKSPGR;YAFNGLWVK;EDFENDLEDVSRLRD;FEND

LEDVS;EDFVVWDMIRSAMSS;FVVWDMIRS;EDFVVWDMIRSAMSS;WDMIRSAMS;EFRANIPK
NTLNNWI;FRANIPKNT;EKDLFKLRNILSKEI;LFKLRNILS;EKFKKKSSYWLLDFA;FKKKSSYWL;E
LVMNLPLFIAYDSA;VMNLPLFIA;ENETRYSDLKKLSFK;RYSDLKKLS;ETRYSDLKKLSFKDK;RY
SDLKKLS;EVLQYFFFSQFQALK;YFFFSQFQA;EVRSFEKFKKKSSYW;FEKFKKKSS;EWWAKRI
GILRKYID;WAKRIGILR;EYFLKDSRDAFNVLF;FLKDSRDAF;EAALNFINRASVDEV;ALNFINRAS;
EAALNFINRASVDEV;FINRASVDE;FASSQSYWQMFAYSP;FASSQSYWQ;FAYSPIDFTRSPPYS
;YSPIDFTRS;FDASKDKVAGISPDY;FDASKDKVA;FDFDSDNQNLPHNYI;FDSDNQNLP;FDRGILI
RNEKDLFK;RGILIRNEK;FDYLNTNEDFVVWDM;FDYLNTNED;FDYLNTNEDFVVWDM;YLNTNE
DFV;FEKFKKKSSYWLLDF;FKKKSSYWL;FENDLEDVSRLRDFF;DLEDVSRLR;FENDLEDVSRL
RDFF;FENDLEDVS;FFFSQFQALKRYAND;FQALKRYAN;FFFSQFQALKRYAND;FSQFQALKR;F
FNFILNEIKDLKIW;ILNEIKDLK;FFNFPGMRIMKLAFD;FPGMRIMKL;FFNFPGMRIMKLAFD;GMRI
MKLAF;FFSQFQALKRYANDK;FQALKRYAN;FIAYDSADVWAHQKY;FIAYDSADV;FIDFLFASSQ
SYWQM;FASSQSYWQ;FIDFLFASSQSYWQM;FLFASSQSY;FIDSDLNLLKENETR;IDSDLNLLK;
FIDSDLNLLKENETR;LNLLKENET;FKDKFLKEAALNFIN;FLKEAALNF;FKDKFLKEAALNFIN;LKE
AALNFI;FKEYFLKDSRDAFNV;FLKDSRDAF;FKEYFLKDSRDAFNV;YFLKDSRDA;FKKKSSYWL
LDFASF;FKKKSSYWL;FKLRFDASKDKVAGI;LRFDASKDK;FKLRNILSKEIKVQE;FKLRNILSK;FK
LRNILSKEIKVQE;LRNILSKEI;FLEQEQAWDSPAYSW;QAWDSPAYS;FLFASSQSYWQMFAY;FA
SSQSYWQ;FLFASSQSYWQMFAY;FLFASSQSY;FLKEAALNFINRASV;ALNFINRAS;FLKEAALN
FINRASV;LKEAALNFI;FNFILNEIKDLKIWV;ILNEIKDLK;FNFPGMRIMKLAFDF;FPGMRIMKL;FN
GLWVKSPGRDFFN;FNGLWVKSP;FNVLFDRGILIRNEK;FDRGILIRN;FNVLFDRGILIRNEK;VLF
DRGILI;FPGMRIMKLAFDFDS;FPGMRIMKL;FRANIPKNTLNNWIF;FRANIPKNT;FRGFVSTWEV
GVGEA;FRGFVSTWE;FRLLESDLDATLSKN;FRLLESDLD;FSQFQALKRYANDKG;FQALKRYAN
;FTRSPPYSIFSAFAG;FTRSPPYSI;FVAFKEYFLKDSRDA;FKEYFLKDS;FVVWDMIRSAMSSVS;
MIRSAMSSV;FVVWDMIRSAMSSVS;VWDMIRSAM;FVVWDMIRSAMSSVS;WDMIRSAMS;GDL
GKGAYKFIDFLF;LGKGAYKFI;GDNSTIREFVNSLDD;IREFVNSLD;GEAYAFNGLWVKSPG;AYA
FNGLWVKSPG;GEAYAFNGLWVKSPG;FNGLWVKSP;GIELVMNLPLFIAYD;LVMNLPLFI;GIELVMNLP
LFIAYD;VMNLPLFIA;GILLNISSLPSKYGI;ILLNISSLP;GILLNISSLPSKYGI;ISSLPSKYG;GILLNIS
SLPSKYGI;LLNISSLPS;GNEFRANIPKNTLNN;FRANIPKNT;GRDFFNFILNEIKDL;FFNFILNEI;HF
RGFVSTWEVGVGE;FRGFVSTWE;HKKYIFDYLNTNEDF;FDYLNTNED;HKKYIFDYLNTNEDF;YI
FDYLNTN;HNYIKNCIVYTGIGD;IKNCIVYTG;HNYIKNCIVYTGIGD;YIKNCIVYT;IAYDSADVWAH
QKYF;YDSADVWAH;IDFLFASSQSYWQMF;FASSQSYWQ;IDFLFASSQSYWQMF;FLFASSQSY
;IDFTRSPPYSIFSAF;FTRSPPYSI;IDHFRGFVSTWEVGV;FRGFVSTWE;IDLEALDKFIDSDLN;EA
LDKFIDS;IDSDLNLLKENETRY;LNLLKENET;IELVMNLPLFIAYDS;LVMNLPLFI;IELVMNLPLFIAY
DS;VMNLPLFIA;IFDYLNTNEDFVVWD;FDYLNTNED;IFDYLNTNEDFVVWD;YLNTNEDFV;IFRLL
ESDLDATLSK;FRLLESDLD;IFSAFAGNVYYIDLE;FSAFAGNVY;IGDNSTIREFVNSLD;NSTIREF
VN;IGILRKYIDVIKIDH;IGILRKYID;IGILRKYIDVIKIDH;ILRKYIDVI;IIPMQDYINLGNEFR;PMQDYI
NLG;IKIDHFRGFVSTWEV;FRGFVSTWE;IKNCIVYTGIGDNST;IKNCIVYTG;ILLNISSLPSKYGIG;
ILLNISSLP;ILLNISSLPSKYGIG;ISSLPSKYG;ILLNISSLPSKYGIG;LLNISSLPS;ILNEIKDLKIWVE
DF;EIKDLKIWV;ILNEIKDLKIWVEDF;ILNEIKDLK;INLGNEFRANIPKNT;LGNEFRANI;INLNLKRK
SGILLNI;LKRKSGILL;IPKNTLNNWIFRLLE;LNNWIFRLL;IPKNTLNNWIFRLLE;PKNTLNNWI;IPM
QDYINLGNEFRA;YINLGNEFR;IREFVNSLDDLHKKY;FVNSLDDLH;IREFVNSLDDLHKKY;IREFV
NSLD;IRINLNLKRKSGILL;INLNLKRKS;IRINLNLKRKSGILL;IRINLNLKR;IRSAMSSVSDSVIIP;IR
SAMSSVS;ISSLPSKYGIGDLGK;ISSLPSKYG;IVYTGIGDNSTIREF;IGDNSTIRE;IVYTGIGDNSTI
REF;IVYTGIGDN;IVYTGIGDNSTIREF;YTGIGDNST;KDKFLKEAALNFINR;LKEAALNFI;KDKVAG
ISPDYFLEQ;VAGISPDYF;KDLFKLRNILSKEIK;LFKLRNILS;KDLFKLRNILSKEIK;LRNILSKEI;KE
YFLKDSRDAFNVL;FLKDSRDAF;KEYFLKDSRDAFNVL;YFLKDSRDA;KEAALNFINRASVDE;AL
NFINRAS;KEAALNFINRASVDE;AALNFINRA;KFIDFLFASSQSYWQ;FLFASSQSY;KFIDSDLNLL
KENET;FIDSDLNLL;KFKKKSSYWLLDFAS;FKKKSSYWL;KFLKEAALNFINRAS;FLKEAALNF;KF
LKEAALNFINRAS;LKEAALNFI;KGIELVMNLPLFIAY;VMNLPLFIA;KIDHFRGFVSTWEVG;FRGFV
STWE;KIRINLNLKRKSGIL;INLNLKRKS;KIRINLNLKRKSGIL;IRINLNLKR;KKIRINLNLKRKSGI;IN
LNLKRKS;KKIRINLNLKRKSGI;IRINLNLKR;KKSSYWLLDFASFVA;WLLDFASFV;KKSSYWLLDF
ASFVA;YWLLDFASF;KKYIFDYLNTNEDFV;FDYLNTNED;KKYIFDYLNTNEDFV;YIFDYLNTN;KL
RNILSKEIKVQEV;LRNILSKEI;KNCIVYTGIGDNSTI;YTGIGDNST;KNTLNNWIFRLLESD;LNNWIF
RLL;KNVKYEWWAKRIGIL;VKYEWWAKR;KNVKYEWWAKRIGIL;YEWWAKRIG;KRIGILRKYIDV
IKI;IGILRKYID;KRIGILRKYIDVIKI;ILRKYIDVI;KRKSGILLNISSLPS;GILLNISSL;KRKSGILLNISSL

PS;ILLNISSLP;KSGILLNISSLPSKY;ILLNISSLP;KSGILLNISSLPSKY;LLNISSLPS;KSSYWLLDFA
SFVAF;LLDFASFVA;KSSYWLLDFASFVAF;WLLDFASFV;KYEWWAKRIGILRKY;WAKRIGILR;K
YEWWAKRIGILRKY;YEWWAKRIG;KYGIGDLGKGAYKFI;IGDLGKGAY;KYIFDYLNTNEDFVV;F
DYLNTNED;KYIFDYLNTNEDFVV;YIFDYLNTN;KYIFDYLNTNEDFVV;YLNTNEDFV;KYKKIRINL
NLKRKS;IRINLNLKR;KYKKIRINLNLKRKS;YKKIRINLN;LDATLSKNISFITRL;LSKNISFIT;LDDLH
KKYIFDYLNT;HKKYIFDYL;LEDVSRLRDFFNFPG;LEDVSRLRD;LEDVSRLRDFFNFPG;RLRDFF
NFP;LEQEQAWDSPAYSWN;EQAWDSPAY;LESDLDATLSKNISF;LESDLDATL;LFDRGILIRNEK
DLF;ILIRNEKDL;LFDRGILIRNEKDLF;RGILIRNEK;LFIAYDSADVWAHQK;FIAYDSADV;LFKLRNI
LSKEIKVQ;FKLRNILSK;LFKLRNILSKEIKVQ;LRNILSKEI;LGNEFRANIPKNTLN;FRANIPKNT;LH
KKYIFDYLNTNED;YIFDYLNTN;LKDSRDAFNVLFDRG;DSRDAFNVL;LKEAALNFINRASVD;ALN
FINRAS;LKNVKYEWWAKRIGI;VKYEWWAKR;LKNVKYEWWAKRIGI;YEWWAKRIG;LKRKSGIL
LNISSLP;GILLNISSL;LKRKSGILLNISSLP;LKRKSGILL;LLDFASFVAFKEYFL;LLDFASFVA;LLES
DLDATLSKNIS;LDATLSKNI;LLNISSLPSKYGIGD;ISSLPSKYG;LLNISSLPSKYGIGD;LLNISSLPS
;LNFINRASVDEVRSF;FINRASVDE;LNISSLPSKYGIGDL;ISSLPSKYG;LNLKRKSGILLNISS;LKR
KSGILL;LNLLKENETRYSDLK;LKENETRYS;LNNWIFRLLESDLDA;FRLLESDLD;LNTNEDFVVW
DMIRS;TNEDFVVWD;LPHNYIKNCIVYTGI;IKNCIVYTG;LPHNYIKNCIVYTGI;LPHNYIKNC;LPHN
YIKNCIVYTGI;YIKNCIVYT;LPLFIAYDSADVWAH;FIAYDSADV;LQYFFFSQFQALKRY;FFSQFQA
LK;LQYFFFSQFQALKRY;FSQFQALKR;LRDFFNFPGMRIMKL;FFNFPGMRI;LRNILSKEIKVQEV
L;LRNILSKEI;LRNILSKEIKVQEVL;LSKEIKVQE;LSFKDKFLKEAALNF;FKDKFLKEA;LSKEIKVQE
VLQYFF;IKVQEVLQY;LSKNISFITRLYGRT;LSKNISFIT;LSKNISFITRLYGRT;SFITRLYGR;LVMN
LPLFIAYDSAD;LVMNLPLFI;LVMNLPLFIAYDSAD;VMNLPLFIA;LWVKSPGRDFFNFIL;LWVKSP
GRD;LWVKSPGRDFFNFIL;PGRDFFNFI;MFAYSPIDFTRSPPY;YSPIDFTRS;MIRSAMSSVSDSV
II;IRSAMSSVS;MKYKKIRINLNLKRK;IRINLNLKR;MKYKKIRINLNLKRK;YKKIRINLN;MNLPLFIAY
DSADVW;FIAYDSADV;MQDYINLGNEFRANI;YINLGNEFR;NCIVYTGIGDNSTIR;YTGIGDNST;N
DKGIELVMNLPLFI;DKGIELVMN;NDKGIELVMNLPLFI;IELV
MNLPL;NDLEDVSRLRDFFNF;VSRLRDFFN;NEDFVVWDMIRSAMS;FVVWDMIRS;NEDFVVWD
MIRSAMS;VVWDMIRSA;NEFRANIPKNTLNNW;FRANIPKNT;NEKDLFKLRNILSKE;LFKLRNILS;
NETRYSDLKKLSFKD;RYSDLKKLS;NFILNEIKDLKIWVE;FILNEIKDL;NFILNEIKDLKIWVE;ILNEI
KDLK;NFPGMRIMKLAFDFD;FPGMRIMKL;NILSKEIKVQEVLQY;EIKVQEVLQ;NIPKNTLNNWIFR
LL;IPKNTLNNW;NISSLPSKYGIGDLG;ISSLPSKYG;NLGNEFRANIPKNTL;FRANIPKNT;NLKRKS
GILLNISSL;LKRKSGILL;NLLKENETRYSDLKK;LKENETRYS;NLNLKRKSGILLNIS;LKRKSGILL;
NLPHNYIKNCIVYTG;YIKNCIVYT;NLPLFIAYDSADVWA;FIAYDSADV;NNWIFRLLESDLDAT;FRL
LESDLD;NQNLPHNYIKNCIVY;LPHNYIKNC;NSLDDLHKKYIFDYL;LDDLHKKYI;NSTIREFVNSL
DDLH;IREFVNSLD;NTLNNWIFRLLESDL;LNNWIFRLL;NTNEDFVVWDMIRSA;FVVWDMIRS;NV
KYEWWAKRIGILR;VKYEWWAKR;NVKYEWWAKRIGILR;WWAKRIGIL;NVKYEWWAKRIGILR;Y
EWWAKRIG;NVLFDRGILIRNEKD;RGILIRNEK;NVLKNVKYEWWAKRI;VKYEWWAKR;NVYYIDL
EALDKFID;YIDLEALDK;NWIFRLLESDLDATL;FRLLESDLD;NYIKNCIVYTGIGDN;IKNCIVYTG;P
AYSWNVLKNVKYEW;WNVLKNVKY;PAYSWNVLKNVKYEW;YSWNVLKNV;PHNYIKNCIVYTGI
G;IKNCIVYTG;PHNYIKNCIVYTGIG;YIKNCIVYT;PIDFTRSPPYSIFSA;FTRSPPYSI;PKNTLNNWI
FRLLES;LNNWIFRLL;PLFIAYDSADVWAHQ;FIAYDSADV;PMQDYINLGNEFRAN;YINLGNEFR;
PPYSIFSAFAGNVYY;IFSAFAGNV;PYSIFSAFAGNVYYI;IFSAFAGNV;QALKRYANDKGIELV;LK
RYANDKG;QAWDSPAYSWNVLKN;QAWDSPAYS;QDYINLGNEFRANIP;LGNEFRANI;QDYINL
GNEFRANIP;YINLGNEFR;QMFAYSPIDFTRSPP;YSPIDFTRS;QNLPHNYIKNCIVYT;LPHNYIKN
C;QNLPHNYIKNCIVYT;PHNYIKNCI;QSYWQMFAYSPIDFT;MFAYSPIDF;QSYWQMFAYSPIDFT
;WQMFAYSPI;QSYWQMFAYSPIDFT;YWQMFAYSP;QYFFFSQFQALKRYA;FFSQFQALK;RDFF
NFILNEIKDLK;FFNFILNEI;RDFFNFPGMRIMKLA;FPGMRIMKL;RGILIRNEKDLFKLR;LIRNEKDL
F;RIGILRKYIDVIKID;IGILRKYID;RIGILRKYIDVIKID;ILRKYIDVI;RIGILRKYIDVIKID;LRKYIDVIK;R
INLNLKRKSGILLN;LKRKSGILL;RKSGILLNISSLPSK;ILLNISSLP;RKSGILLNISSLPSK;LLNISSLP
S;RLRDFFNFPGMRIMK;FFNFPGMRI;RNEKDLFKLRNILSK;LFKLRNILS;RNILSKEIKVQEVLQ;L
SKEIKVQE;RSFEKFKKKSSYWLL;FKKKSSYWL;RSPPYSIFSAFAGNV;YSIFSAFAG;SDLDATLS
KNISFIT;DATLSKNIS;SDLNLLKENETRYSD;LKENETRYS;SDLNLLKENETRYSD;LNLLKENET;S
FEKFKKKSSYWLLD;FKKKSSYWL;SFKDKFLKEAALNFI;FLKEAALNF;SGILLNISSLPSKYG;ILLN
ISSLP;SGILLNISSLPSKYG;LLNISSLPS;SLPSKYGIGDLGKGA;PSKYGIGDL;SPAYSWNVLKNV
KYE;AYSWNVLKN;SPAYSWNVLKNVKYE;WNVLKNVKY;SPAYSWNVLKNVKYE;YSWNVLKNV;
SPIDFTRSPPYSIFS;FTRSPPYSI;SPPYSIFSAFAGNVY;IFSAFAGNV;SQFQALKRYANDKGI;FQ

ALKRYAN;SQSYWQMFAYSPIDF;WQMFAYSPI;SQSYWQMFAYSPIDF;YWQMFAYSP;SRDAF
NVLFDRGILI;FNVLFDRGI;SRLRDFFNFPGMRIM;FFNFPGMRI;SSQSYWQMFAYSPID;WQMFA
YSPI;SSQSYWQMFAYSPID;YWQMFAYSP;SSYWLLDFASFVAFK;LLDFASFVA;SSYWLLDFAS
FVAFK;WLLDFASFV;STIREFVNSLDDLHK;IREFVNSLD;STWEVGVGEAYAFNG;VGVGEAYAF;
SWNVLKNVKYEWWAK;WNVLKNVKY;SYWLLDFASFVAFKE;WLLDFASFV;SYWQMFAYSPIDF
TR;MFAYSPIDF;SYWQMFAYSPIDFTR;WQMFAYSPI;SYWQMFAYSPIDFTR;YWQMFAYSP;TG
IGDNSTIREFVNS;IGDNSTIRE;TIREFVNSLDDLHKK;IREFVNSLD;TLNNWIFRLLESDLD;IFRLLE
SDL;TLNNWIFRLLESDLD;NNWIFRLLE;TLSKNISFITRLYGR;LSKNISFIT;TLSKNISFITRLYGR;S
KNISFITR;TNEDFVVWDMIRSAM;FVVWDMIRS;TNEDFVVWDMIRSAM;VVWDMIRSA;TWEVG
VGEAYAFNGL;VGVGEAYAF;VAFKEYFLKDSRDAF;FKEYFLKDS;VAFKEYFLKDSRDAF;YFLKD
SRDA;VGEAYAFNGLWVKSP;AFNGLWVKS;VGEAYAFNGLWVKSP;AYAFNGLWV;VGVGEAYA
FNGLWVK;GEAYAFNGL;VIKIDHFRGFVSTWE;IDHFRGFVS;VKSPGRDFFNFILNE;PGRDFFNF
I;VKYEWWAKRIGILRK;VKYEWWAKR;VKYEWWAKRIGILRK;WAKRIGILR;VKYEWWAKRIGILR
K;YEWWAKRIG;VLFDRGILIRNEKDL;RGILIRNEK;VLKNVKYEWWAKRIG;VKYEWWAKR;VLQY
FFFSQFQALKR;FFSQFQALK;VMNLPLFIAYDSADV;VMNLPLFIA;VRSFEKFKKKSSYWL;FEKFK
KKSS;VRSFEKFKKKSSYWL;KFKKKSSYW;VRSFEKFKKKSSYWL;VRSFEKFKK;VSRLRDFFNF
PGMRI;LRDFFNFPG;VSRLRDFFNFPGMRI;RLRDFFNFP;VSRLRDFFNFPGMRI;VSRLRDFFN;
VVWDMIRSAMSSVSD;IRSAMSSVS;VVWDMIRSAMSSVSD;MIRSAMSSV;VVWDMIRSAMSSV
SD;WDMIRSAMS;VWAHQKYFKLRFDAS;QKYFKLRFD;VWAHQKYFKLRFDAS;WAHQKYFKL;V
WDMIRSAMSSVSDS;IRSAMSSVS;VWDMIRSAMSSVSDS;MIRSAMSSV;VWDMIRSAMSSVSD
S;WDMIRSAMS;VYTGIGDNSTIREFV;IGDNSTIRE;VYTGIGDNSTIREFV;YTGIGDNST;VYYIDLE
ALDKFIDS;YIDLEALDK;WAKRIGILRKYIDVI;IGILRKYID;WAKRIGILRKYIDVI;WAKRIGILR;WDMI
RSAMSSVSDSV;IRSAMSSVS;WDMIRSAMSSVSDSV;WDMIRSAMS;WEVGVGEAYAFNGLW;V
GVGEAYAF;WIFRLLESDLDATLS;FRLLESDLD;WNVLKNVKYEWWAKR;NVKYEWWAK;WNVL
KNVKYEWWAKR;WNVLKNVKY;WQMFAYSPIDFTRSP;MFAYSPIDF;WQMFAYSPIDFTRSP;YS
PIDFTRS;WWAKRIGILRKYIDV;IGILRKYID;YAFNGLWVKSPGRDF;FNGLWVKSP;YDSADVWA
HQKYFKL;VWAHQKYFK;YEWWAKRIGILRKYI;WAKRIGILR;YEWWAKRIGILRKYI;YEWWAKRI
G;YFFFSQFQALKRYAN;FFSQFQALK;YFFFSQFQALKRYAN;FSQFQALKR;YFLEQEQAWDSP
AYS;EQAWDSPAY;YGIGDLGKGAYKFID;LGKGAYKFI;YIFDYLNTNEDFVVW;FDYLNTNED;YIF
DYLNTNEDFVVW;YIFDYLNTN;YIFDYLNTNEDFVVW;YLNTNEDFV;YIKNCIVYTGIGDNS;IKNCI
VYTG;YINLGNEFRANIPKN;YINLGNEFR;YKFIDFLFASSQSYW;FIDFLFASS;YKFIDFLFASSQS
YW;FLFASSQSY;YKKIRINLNLKRKSG;INLNLKRKS;YKKIRINLNLKRKSG;IRINLNLKR;YKKIRINL
NLKRKSG;YKKIRINLN;YSIFSAFAGNVYYID;IFSAFAGNV;YSPIDFTRSPPYSIF;FTRSPPYSI;YS
WNVLKNVKYEWWA;WNVLKNVKY;YTGIGDNSTIREFVN;IGDNSTIRE;YTGIGDNSTIREFVN;YT
GIGDNST;YWQMFAYSPIDFTRS;MFAYSPIDF;YWQMFAYSPIDFTRS;WQMFAYSPI;YWQMFAY
SPIDFTRS;YWQMFAYSP;AALNFINRASVDEVR;FINRASVDE;AALNFINRASVDEVR;INRASVDE
V

**<CAA59727 outer surface protein A;Protein;Borrelia garinii>**

**Class 1**
KYLLGIGL;YLLGIGLI;LLGIGLIL;GIGLILAL;GLILALIA;ILALIACK;LIACKQNV;NVSSLDEK;SLDEK
NSV;SVDLPGGM;DLPGGMKV;LPGGMKVL;GMKVLVSK;KVLVSKEK;KYSLMATV;SLMATVEK;L
MATVEKL;ATVEKLEL;TVEKLELK;ELKGTSDK;KTDKSKVK;KLTIAEDL;TIAEDLSK;AEDLSKTT;K
TTFEIFK;TTFEIFKE;EIFKEDGK;FKEDGKTL;KEDGKTLV;LVSKKVTL;VSKKVTLK;TLKDKSST;K
SSTEEKF;GEISEKTI;EISEKTIV;ISEKTIVR;IVRANGTR;RLEYTDIK;EVLKDFTL;FTLEGTLA;TLE
GTLAA;GTLAADGK;AADGKTTL;KVTEGTVV;EGTVVLSK;TVVLSKNI;VLSKNILK;ALDDSDTT;DT
TQATKK;KTGKWDSK;TSTLTISV;TISVNSQK;SVNSQKTK;SQKTKNLV;TKNLVFTK;EDTITVQK;
DTITVQKY;KAVEITTL;EITTLEKL;ITTLEKLK

KYLLGIGLI;YLLGIGLIL;LLGIGLILA;GIGLILALI;LILALIACK;ALIACKQNV;SSLDEKNSV;SLDEKNS
VS;SVDLPGGMK;VDLPGGMKV;DLPGGMKVL;LPGGMKVLV;GGMKVLVSK;GKYSLMATV;YSL
MATVEK;SLMATVEKL;ATVEKLELK;GSGTLEGEK;LTIAEDLSK;AEDLSKTTF;DLSKTTFEI;FKED
GKTLV;TLVSKKVTL;LVSKKVTLK;KDKSSTEEK;STEEKFNEK;GEISEKTIV;EISEKTIVR;TIVRAN
GTR;IVRANGTRL;RANGTRLEY;KSDGSGKAK;SGKAKEVLK;KEVLKDFTL;VLKDFTLEG;FTLEG

TLAA;LAADGKTTL;AADGKTTLK;TTLKVTEGT;TLKVTEGTV;KVTEGTVVL;VVLSKNILK;VLSKNI
LKS;ILKSGEITV;KSGEITVAL;ALDDSDTTQ;TQATKKTGK;KTSTLTISV;LTISVNSQK;ISVNSQKT
K;SQKTKNLVF;KTKNLVFTK;FTKEDTITV;KYDSAGTNL;SAGTNLEGK;NLEGKAVEI;AVEITTLEK
;VEITTLEKL;EITTLEKLK;TLEKLKDAL

KYLLGIGLIL;YLLGIGLILA;LLGIGLILAL;LGIGLILALI;GLILALIACK;KQNVSSLDEK;SLDEKNSVSV
;SVSVDLPGGM;VSVDLPGGMK;SVDLPGGMKV;VDLPGGMKVL;DLPGGMKVLV;LPGGMKVLV
S;GMKVLVSKEK;KVLVSKEKDK;KYSLMATVEK;YSLMATVEKL;LMATVEKLEL;MATVEKLELK;K
LELKGTSDK;GTLEGEKTDK;KLTIAEDLSK;IAEDLSKTTF;DLSKTTFEIF;LSKTTFEIFK;EIFKEDG
KTL;TLVSKKVTLK;VSKKVTLKDK;SSTEEKFNEK;EEKFNEKGEI;EISEKTIVRA;KTIVRANGTR;TI
VRANGTRL;GTRLEYTDIK;GSGKAKEVLK;VLKDFTLEGT;FTLEGTLAAD;TLAADGKTTL;LAADG
KTTLK;AADGKTTLKV;TTLKVTEGTV;TLKVTEGTVV;VTEGTVVLSK;TVVLSKNILK;NILKSGEITV;
ILKSGEITVA;ALDDSDTTQA;TTQATKKTGK;SKTSTLTISV;TLTISVNSQK;TISVNSQKTK;NSQKT
KNLVF;NLVFTKEDTI;VFTKEDTITV;DSAGTNLEGK;KAVEITTLEK;TTLEKLKDAL;TLEKLKDALK

YLLGIGLILAL;LLGIGLILALI;ILALIACKQNV;LIACKQNVSSL;NVSSLDEKNSV;SSLDEKNSVSV;S
VSVDLPGGMK;SVDLPGGMKVL;VDLPGGMKVLV;LPGGMKVLVSK;LVSKEKDKDGK;KYSLMAT
VEKL;SLMATVEKLEL;LMATVEKLELK;GEKTDKSKVKL;KTDKSKVKLTI;TIAEDLSKTTF;AEDLSK
TTFEI;DLSKTTFEIFK;TTFEIFKEDGK;FEIFKEDGKTL;EIFKEDGKTLV;KTLVSKKVTLK;LVSKKV
TLKDK;TLKDKSSTEEK;KSSTEEKFNEK;KFNEKGEISEK;EKTIVRANGTR;KTIVRANGTRL;IVRA
NGTRLEY;YTDIKSDGSGK;VLKDFTLEGTL;TLEGTLAADGK;TLAADGKTTLK;LAADGKTTLKV;K
TTLKVTEGTV;TTLKVTEGTVV;TLKVTEGTVVL;KVTEGTVVLSK;GTVVLSKNILK;ILKSGEITVAL;
ALDDSDTTQAT;DTTQATKKTGK;ATKKTGKWDSK;KWDSKTSTLTI;STLTISVNSQK;LTISVNSQ
KTK;SVNSQKTKNLV;SQKTKNLVFTK;LVFTKEDTITV;FTKEDTITVQK;DTITVQKYDSA;VQKYDS
AGTNL;NLEGKAVEITT;LEGKAVEITTL;KAVEITTLEKL;AVEITTLEKLK;TTLEKLKDALK

**Class 2**
ADGKTTLKVTEGTVV;ADGKTTLKV;ADGKTTLKVTEGTVV;TLKVTEGTV;AGTNLEGKAVEITTL;L
EGKAVEIT;AKEVLKDFTLEGTLA;VLKDFTLEG;DFTLEGTLAADGKTT;FTLEGTLAA;DGKTLVSK
KVTLKDK;LVSKKVTLK;DGKTTLKVTEGTVVL;LKVTEGTVV;DGKYSLMATVEKLEL;YSLMATVE
K;DKDGKYSLMATVEKL;YSLMATVEK;DLPGGMKVLVSKEKD;MKVLVSKEK;DSAGTNLEGKAV
EIT;GTNLEGKAV;DTITVQKYDSAGTNL;VQKYDSAGT;EDGKTLVSKKVTLKD;GKTLVSKKV;ED
GKTLVSKKVTLKD;LVSKKVTLK;EDTITVQKYDSAGTN;VQKYDSAGT;EGTVVLSKNILKSGE;TV
VLSKNIL;EGTVVLSKNILKSGE;VLSKNILKS;EIFKEDGKTLVSKKV;FKEDGKTLV;EISEKTIVRAN
GTRL;KTIVRANGT;EKTIVRANGTRLEYT;IVRANGTRL;EVLKDFTLEGTLAAD;FTLEGTLAA;EYT
DIKSDGSGKAKE;IKSDGSGKA;FKEDGKTLVSKKVTL;GKTLVSKKV;FTLEGTLAADGKTTL;FTLE
GTLAA;GGMKVLVSKEKDKDG;MKVLVSKEK;GIGLILALIACKQNV;ILALIACKQ;GKAKEVLKDFTL
EGT;VLKDFTLEG;GKTLVSKKVTLKDKS;LVSKKVTLK;GKTTLKVTEGTVVLS;LKVTEGTVV;GKW
DSKTSTLTISVN;WDSKTSTLT;GKYSLMATVEKLELK;YSLMATVEK;GLILALIACKQNVSS;ILALIA
CKQ;GTNLEGKAVEITTLE;LEGKAVEIT;GTVVLSKNILKSGEI;VVLSKNILK;IFKEDGKTLVSKKVT;
GKTLVSKKV;IGLILALIACKQNVS;ILALIACKQ;ILKSGEITVALDDSD;LKSGEITVA;ISEKTIVRANG
TRLE;IVRANGTRL;ISVNSQKTKNLVFTK;VNSQKTKNL;ITVQKYDSAGTNLEG;VQKYDSAGT;ITV
QKYDSAGTNLEG;YDSAGTNLE;IVRANGTRLEYTDIK;IVRANGTRL;KAKEVLKDFTLEGTL;VLKD
FTLEG;KDFTLEGTLAADGKT;FTLEGTLAA;KDGKYSLMATVEKLE;YSLMATVEK;KDKDGKYSL
MATVEK;KDGKYSLMA;KEDGKTLVSKKVTLK;GKTLVSKKV;KEDTITVQKYDSAGT;ITVQKYDSA
;KEVLKDFTLEGTLAA;LKDFTLEGT;KEVLKDFTLEGTLAA;VLKDFTLEG;KKTGKWDSKTSTLTI;
WDSKTSTLT;KKYLLGIGLILALIA;LLGIGLILA;KNILKSGEITVALDD;LKSGEITVA;KNLVFTKEDTIT
VQK;FTKEDTITV;KTGKWDSKTSTLTIS;WDSKTSTLT;KTIVRANGTRLEYTD;IVRANGTRL;KTKN
LVFTKEDTITV;KTKNLVFTK;KTKNLVFTKEDTITV;VFTKEDTIT;KTTLKVTEGTVVLSK;LKVTEGT
VV;KTTLKVTEGTVVLSK;VTEGTVVLS;KVTEGTVVLSKNILK;TVVLSKNIL;KVTEGTVVLSKNILK;
VTEGTVVLS;KWDSKTSTLTISVNS;WDSKTSTLT;KYLLGIGLILALIAC;LLGIGLILA;LEGTLAADG
KTTLKV;LEGTLAADG;LEYTDIKSDGSGKAK;IKSDGSGKA;LGIGLILALIACKQN;IGLILALIA;LKDF
TLEGTLAADGK;FTLEGTLAA;LKSGEITVALDDSDT;LKSGEITVA;LKVTEGTVVLSKNIL;LKVTEG
TVV;LKVTEGTVVLSKNIL;VTEGTVVLS;LLGIGLILALIACKQ;IGLILALIA;LPGGMKVLVSKEKDK;M
KVLVSKEK;LSKNILKSGEITVAL;LKSGEITVA;LTISVNSQKTKNLVF;VNSQKTKNL;LVFTKEDTIT

VQKYD;FTKEDTITV;MKKYLLGIGLILALI;LLGIGLILA;NILKSGEITVALDDS;LKSGEITVA;NLVFTK
EDTITVQKY;FTKEDTITV;NSVSVDLPGGMKVLV;VDLPGGMKV;PGGMKVLVSKEKDKD;MKVLV
SKEK;QKYDSAGTNLEGKAV;YDSAGTNLE;RLEYTDIKSDGSGKA;YTDIKSDGS;SAGTNLEGKA
VEITT;LEGKAVEIT;SEKTIVRANGTRLEY;IVRANGTRL;SGKAKEVLKDFTLEG;AKEVLKDFT;SK
NILKSGEITVALD;LKSGEITVA;SKVKLTIAEDLSKTT;VKLTIAEDL;STLTISVNSQKTKNL;ISVNSQ
KTK;STLTISVNSQKTKNL;TISVNSQKT;SVDLPGGMKVLVSKE;LPGGMKVLV;SVSVDLPGGMKV
LVS;LPGGMKVLV;TEGTVVLSKNILKSG;TVVLSKNIL;TEGTVVLSKNILKSG;VLSKNILKS;TGKW
DSKTSTLTISV;WDSKTSTLT;TISVNSQKTKNLVFT;VNSQKTKNL;TITVQKYDSAGTNLE;VQKYD
SAGT;TIVRANGTRLEYTDI;VRANGTRLE;TKKTGKWDSKTSTLT;TGKWDSKTS;TKNLVFTKEDT
ITVQ;FTKEDTITV;TLEGTLAADGKTTLK;LEGTLAADG;TLKVTEGTVVLSKNI;VTEGTVVLS;TLTIS
VNSQKTKNLV;VNSQKTKNL;TNLEGKAVEITTLEK;LEGKAVEIT;TTLKVTEGTVVLSKN;LKVTEG
TVV;TTLKVTEGTVVLSKN;VTEGTVVLS;TVQKYDSAGTNLEGK;VQKYDSAGT;TVQKYDSAGTN
LEGK;YDSAGTNLE;TVVLSKNILKSGEIT;TVVLSKNIL;VALDDSDTTQATKKT;LDDSDTTQA;VDL
PGGMKVLVSKEK;LPGGMKVLV;VDLPGGMKVLVSKEK;VDLPGGMKV;VLKDFTLEGTLAADG;F
TLEGTLAA;VLSKNILKSGEITVA;KNILKSGEI;VQKYDSAGTNLEGKA;VQKYDSAGT;VQKYDSAG
TNLEGKA;YDSAGTNLE;VRANGTRLEYTDIKS;VRANGTRLE;VSVDLPGGMKVLVSK;LPGGMKV
LV;VTEGTVVLSKNILKS;TVVLSKNIL;VTEGTVVLSKNILKS;VTEGTVVLS;VVLSKNILKSGEITV;V
VLSKNILK;WDSKTSTLTISVNSQ;WDSKTSTLT;YLLGIGLILALIACK;IGLILALIA;YSLMATVEKLEL
KGT;YSLMATVEK;YTDIKSDGSGKAKEV;IKSDGSGKA

**<AAN87995 Outer Surface Protein C;Protein;Borrelia garinii>**

**Class 1**

ILMTLFLF;LMTLFLFI;TLFLFISC;STNPDESA;ESAKGPNL;AKGPNLTV;NLTVISKK;KITDSNAF;IT
DSNAFV;SNAFVLAV;NAFVLAVK;FVLAVKEV;AVKEVEAL;KEVEALIS;LISSIDEL;SIDELANK;DE
LANKAI;LANKAIGK;VIHQNNGL;HQNNGLNA;GQNGSLLA;GSLLAGAY;SLLAGAYA;LLAGAYAI;
GAYAISTL;AYAISTLI;ISTLITEK;LITEKLSK;TEKLSKLK;KLKNSEEL;HSEAFTNR;SEAFTNRL;EA
FTNRLK;RLKGSHAQ;AQLGVAAA;AATDDHAK;DHAKEAIL;HAKEAILK;ILKSNPTK;KSNPTKDK;
PTKDKGAK;ESVESLAK;EALANSVK;KELTNPVV;ELTNPVVA;PVVAESPK

ILMTLFLFI;FLFISCNNS;STNPDESAK;SAKGPNLTV;NLTVISKKI;KITDSNAFV;ITDSNAFVL;DSN
AFVLAV;SNAFVLAVK;VLAVKEVEA;LAVKEVEAL;AVKEVEALI;EVEALISSI;ALISSIDEL;LISSIDE
LA;SSIDELANK;SIDELANKA;ELANKAIGK;LANKAIGKV;KVIHQNNGL;NGSLLAGAY;SLLAGAYAI
;LLAGAYAIS;GAYAISTLI;AISTLITEK;ISTLITEKL;TLITEKLSK;LITEKLSKL;ITEKLSKLK;ELNKKIE
EA;EEAKNHSEA;EAKNHSEAF;HSEAFTNRL;RLKGSHAQL;AQLGVAAAT;AAATDDHAK;ATDDH
AKEA;AILKSNPTK;KDKGAKELK;ELKDLSESV;DLSESVESL;ESVESLAKA;SLAKAAQEA;AQEAL
ANSV;ALANSVKEL;SVKELTNPV

ILMTLFLFIS;LMTLFLFISC;FLFISCNNSG;TASTNPDESA;ASTNPDESAK;ESAKGPNLTV;KGPNL
TVISK;KKITDSNAFV;KITDSNAFVL;DSNAFVLAVK;NAFVLAVKEV;FVLAVKEVEA;VLAVKEVEAL
;LAVKEVEALI;KEVEALISSI;EALISSIDEL;ALISSIDELA;ISSIDELANK;SIDELANKAI;ELANKAIGK
V;HQNNGLNANA;NANAGQNGSL;GQNGSLLAGA;SLLAGAYAIS;LLAGAYAIST;LAGAYAISTL;A
GAYAISTLI;YAISTLITEK;AISTLITEKL;STLITEKLSK;TLITEKLSKL;LITEKLSKLK;KLKNSEELNK;
ELNKKIEEAK;EEAKNHSEAF;NHSEAFTNRL;HSEAFTNRLK;VAAATDDHAK;ATDDHAKEAI;EAI
LKSNPTK;ILKSNPTKDK;KELKDLSESV;DLSESVESLA;LSESVESLAK;SLAKAAQEAL;AAQEALA
NSV;AQEALANSVK;EALANSVKEL;ALANSVKELT;NSVKELTNPV;SVKELTNPVV

ILMTLFLFISC;FLFISCNNSGG;DTASTNPDESA;TASTNPDESAK;ESAKGPNLTVI;ISKKITDSNAF
;KITDSNAFVLA;ITDSNAFVLAV;SNAFVLAVKEV;FVLAVKEVEAL;VLAVKEVEALI;VEALISSIDEL;
EALISSIDELA;LISSIDELANK;ELANKAIGKVI;GQNGSLLAGAY;NGSLLAGAYAI;SLLAGAYAIST;L
LAGAYAISTL;LAGAYAISTLI;AYAISTLITEK;YAISTLITEKL;ISTLITEKLSK;TLITEKLSKLK;KLSKL
KNSEEL;KLKNSEELNKK;EEAKNHSEAFT;RLKGSHAQLGV;GVAAATDDHAK;ATDDHAKEAIL;AI
LKSNPTKDK;KSNPTKDKGAK;NPTKDKGAKEL;PTKDKGAKELK;SLAKAAQEALA;KAAQEALAN
SV;AAQEALANSVK;QEALANSVKEL;SVKELTNPVVA;LTNPVVAESPK

**Class 2**
AFTNRLKGSHAQLGV;LKGSHAQLG;AFVLAVKEVEALISS;VKEVEALIS;AGAYAISTLITEKLS;IST
LITEKL;AGQNGSLLAGAYAIS;GQNGSLLAG;AIGKVIHQNNGLNAN;IHQNNGLNA;AILKSNPTKD
KGAKE;LKSNPTKDK;AISTLITEKLSKLKN;ISTLITEKL;AKEAILKSNPTKDKG;EAILKSNPT;AKEAI
LKSNPTKDKG;LKSNPTKDK;AKGPNLTVISKKITD;LTVISKKIT;ALANSVKELTNPVVA;LANSVKE
LT;ALANSVKELTNPVVA;SVKELTNPV;ALANSVKELTNPVVA;VKELTNPVV;ANSVKELTNPVVA
ES;SVKELTNPV;ANSVKELTNPVVAES;VKELTNPVV;AQEALANSVKELTNP;LANSVKELT;AQL
GVAAATDDHAKE;LGVAAATDD;AYAISTLITEKLSKL;ISTLITEKL;DELANKAIGKVIHQN;LANKAI
GKV;EAFTNRLKGSHAQLG;TNRLKGSHA;EAILKSNPTKDKGAK;LKSNPTKDK;EALANSVKELT
NPVV;LANSVKELT;EALANSVKELTNPVV;SVKELTNPV;ESLAKAAQEALANSV;LAKAAQEAL;ES
VESLAKAAQEALA;SLAKAAQEA;FTNRLKGSHAQLGVA;LKGSHAQLG;FVLAVKEVEALISSI;VK
EVEALIS;GAYAISTLITEKLSK;ISTLITEKL;GKVIHQNNGLNANAG;HQNNGLNAN;GLNANAGQN
GSLLAG;LNANAGQNG;GPNLTVISKKITDSN;LTVISKKIT;GQNGSLLAGAYAIST;LLAGAYAIS;GS
HAQLGVAAATDDH;LGVAAATDD;GSLLAGAYAISTLIT;LAGAYAIST;HAKEAILKSNPTKDK;EAIL
KSNPT;HAQLGVAAATDDHAK;LGVAAATDD;HQNNGLNANAGQNGS;LNANAGQNG;IDELANKA
IGKVIHQ;LANKAIGKV;IGKVIHQNNGLNANA;HQNNGLNAN;IHQNNGLNANAGQNG;NNGLNAN
AG;ILMTLFLFISCNNSG;FLFISCNNS;ISKKITDSNAFVLAV;ITDSNAFVL;ISSIDELANKAIGKV;IDE
LANKAI;ISTLITEKLSKLKNS;ISTLITEKL;ITDSNAFVLAVKEVE;ITDSNAFVL;KEAILKSNPTKDKG
A;LKSNPTKDK;KGPNLTVISKKITDS;LTVISKKIT;KGSHAQLGVAAATDD;AQLGVAAAT;KITDSN
AFVLAVKEV;ITDSNAFVL;KKITDSNAFVLAVKE;ITDSNAFVL;KVIHQNNGLNANAGQ;HQNNGLN
AN;KAAQEALANSVKELT;EALANSVKE;LAGAYAISTLITEKL;LAGAYAIST;LAGAYAISTLITEKL;Y
AISTLITE;LANSVKELTNPVVAE;LANSVKELT;LANSVKELTNPVVAE;SVKELTNPV;LANSVKELT
NPVVAE;VKELTNPVV;LAVKEVEALISSIDE;VKEVEALIS;LFLFISCNNSGGDTA;LFISCNNSG;LK
GSHAQLGVAAATD;LKGSHAQLG;LLAGAYAISTLITEK;LAGAYAIST;LMTLFLFISCNNSGG;LFIS
CNNSG;LNANAGQNGSLLAGA;LNANAGQNG;LTVISKKITDSNAFV;LTVISKKIT;MTLFLFISCNN
SGGD;LFISCNNSG;NAFVLAVKEVEALIS;LAVKEVEAL;NGLNANAGQNGSLLA;LNANAGQNG;N
GSLLAGAYAISTLI;LAGAYAIST;NLTVISKKITDSNAF;LTVISKKIT;NNGLNANAGQNGSLL;LNANA
GQNG;NRLKGSHAQLGVAAA;LKGSHAQLG;NSVKELTNPVVAESP;LTNPVVAES;NSVKELTNP
VVAESP;VKELTNPVV;PNLTVISKKITDSNA;LTVISKKIT;QEALANSVKELTNPV;LANSVKELT;QN
GSLLAGAYAISTL;LAGAYAIST;QNNGLNANAGQNGSL;LNANAGQNG;RLKGSHAQLGVAAAT;L
KGSHAQLG;SAKGPNLTVISKKIT;KGPNLTVIS;SESVESLAKAAQEAL;SLAKAAQEA;SHAQLGVA
AATDDHA;LGVAAATDD;SIDELANKAIGKVIH;LANKAIGKV;SKKITDSNAFVLAVK;ITDSNAFVL;S
LAKAAQEALANSVK;AQEALANSV;SLLAGAYAISTLITE;LAGAYAIST;SSIDELANKAIGKVI;LANK
AIGKV;SVESLAKAAQEALAN;LAKAAQEAL;SVKELTNPVVAESPK;LTNPVVAES;TLFLFISCNNS
GGDT;LFISCNNSG;TNRLKGSHAQLGVAA;LKGSHAQLG;TVISKKITDSNAFVL;SKKITDSNA;VE
SLAKAAQEALANS;LAKAAQEAL;VISKKITDSNAFVLA;ITDSNAFVL;VLAVKEVEALISSID;VKEVE
ALIS;YAISTLITEKLSKLK;ISTLITEKL;AAQEALANSVKELTN;LANSVKELT

**<CAF34024 outer surface protein VlsE;Protein;Borrelia garinii>**

**Class 1**
KISSAIFL;SAIFLTAL;AIFLTALL;IFLTALLV;FLTALLVF;LTALLVFI;ALLVFINC;LLVFINCK;FINCKN
NA;FYESIINL;IINLGNGF;FIDVFNAF;FNAFSGLV;GLVADTFF;LVADTFFK;TFFKSDPK;KSDVKTY
F;FESISSTL;ESISSTLK;SISSTLKA;SSTLKATK;TLKATKGK;KLDELVSA;KASVESAV;SAVDEVS
K;KWLEEMIK;WLEEMIKA;MIKAAEEA;KAAEEAAK;DSAANHGA;SAANHGAK;DSVKGIAK;KGIA
KGIK;GIKGIVDA;GIVDAAGK;AGKALGEK;ALGEKGAL;ALKDVKAA;EANADAGK;FAGNANAA;NA
AVGAAA;GAAADIAK;IAKAAGAV;KAAGAVTA;AAGAVTAV;AVSGEQIL;VSGEQILK;ILKAIVEA;D
PANQAGK;AEEAKNPI;DDNGAAFK;AAFKDEMK;AFKDEMKK;KIAAAIVL;IAAAIVLR;AAIVLRGV;I
VLRGVAK;AKDGKFAV

KISSAIFLT;ISSAIFLTA;SSAIFLTAL;SAIFLTALL;AIFLTALLV;IFLTALLVF;FLTALLVFI;ALLVFINC
K;FINCKNNAV;AVGKGNDDK;SVKTFYESI;TFYESIINL;SIINLGNGF;IINLGNGFI;NLGNGFIDV;G
FIDVFNAF;DVFNAFSGL;FSGLVADTF;GLVADTFFK;LVADTFFKS;DTFFKSDPK;TFFKSDPKK;D
VKTYFESI;YFESISSTL;ISSTLKATK;STLKATKGK;TLKATKGKL;ATKGKLDEL;KLDELVSAK;EGG
SVKASV;SVKASVESA;SVESAVDEV;ESAVDEVSK;SAVDEVSKW;AVDEVSKWL;EVSKWLEEM;

WLEEMIKAA;EMIKAAEEA;MIKAAEEAA;KAAEEAAKV;DSAANHGAK;ANHGAKADK;SVKGIAKGI
;GIAKGIKGI;GIKGIVDAA;IVDAAGKAL;AAGKALGEK;ALGEKGALK;ALKDVKAAA;NADAGKLFA;
KLFAGNANA;FAGNANAAV;NANAAVGAA;DIAKAAGAV;KAAGAVTAV;TAVSGEQIL;AVSGEQIL
K;QILKAIVEA;ILKAIVEAA;AGKKAEEAK;AEEAKNPIA;EEAKNPIAA;NPIAAAIGT;TDDDNGAAF;G
AAFKDEMK;AAFKDEMKK;KIAAAIVLR;AAAIVLRGV;AIVLRGVAK;VAKDGKFAV

KISSAIFLTA;ISSAIFLTAL;SSAIFLTALL;SAIFLTALLV;AIFLTALLVF;IFLTALLVFI;FLTALLVFIN;TA
LLVFINCK;DSVKTFYESI;SVKTFYESII;KTFYESIINL;ESIINLGNGF;SIINLGNGFI;NGFIDVFNAF;
DVFNAFSGLV;AFSGLVADTF;FSGLVADTFF;SGLVADTFFK;GLVADTFFKS;ADTFFKSDPK;DTF
FKSDPKK;DPKKSDVKTY;KTYFESISST;TYFESISSTL;YFESISSTLK;SISSTLKATK;SSTLKATKG
K;ATKGKLDELV;KLDELVSAKK;SAKKGEGGSV;SVKASVESAV;ASVESAVDEV;ESAVDEVSKW;
SAVDEVSKWL;EVSKWLEEMI;VSKWLEEMIK;EEMIKAAEEA;MIKAAEEAAK;AEEAAKVGGT;KV
GGTGGDGK;AANHGAKADK;HGAKADKDSV;SVKGIAKGIK;GIAKGIKGIV;GIVDAAGKAL;DAAG
KALGEK;KALGEKGALK;EANADAGKLF;KLFAGNANAA;LFAGNANAAV;NANAAVGAAA;AVGAA
ADIAK;AKAAGAVTAV;TAVSGEQILK;AVSGEQILKA;QILKAIVEAA;AIVEAAGDPA;EAAGDPANQ
A;AEEAKNPIAA;EEAKNPIAAA;EAKNPIAAAI;GTDDDNGAAF;NGAAFKDEMK;GAAFKDEMKK;D
KIAAAIVLR;IAAAIVLRGV;AAIVLRGVAK;VLRGVAKDGK;GVAKDGKFAV;VAKDGKFAVK

KKISSAIFLTA;KISSAIFLTAL;ISSAIFLTALL;SSAIFLTALLV;SAIFLTALLVF;AIFLTALLVFI;FLTALL
VFINC;LTALLVFINCK;LLVFINCKNNA;LVFINCKNNAV;FINCKNNAVGK;YESIINLGNGF;ESIINLG
NGFI;IINLGNGFIDV;FIDVFNAFSGL;DVFNAFSGLVA;NAFSGLVADTF;AFSGLVADTFF;FSGLVA
DTFFK;VADTFFKSDPK;DPKKSDVKTYF;KTYFESISSTL;TYFESISSTLK;ESISSTLKATK;ISSTLK
ATKGK;KATKGKLDELV;KGKLDELVSAK;SAKKGEGGSVK;KASVESAVDEV;SVESAVDEVSK;E
SAVDEVSKWL;EVSKWLEEMIK;EEMIKAAEEAA;EMIKAAEEAAK;MIKAAEEAAKV;KIGDSAANH
GA;SAANHGAKADK;DSVKGIAKGIK;GIKGIVDAAGK;ALGEKGALKDV;DEANADAGKLF;KLFAG
NANAAV;FAGNANAAVGA;AAVGAAADIAK;AAADIAKAAGA;IAKAAGAVTAV;AVTAVSGEQIL;VT
AVSGEQILK;AVSGEQILKAI;AAGDPANQAGK;AEEAKNPIAAA;EEAKNPIAAAI;IGTDDDNGAAF;
GTDDDNGAAFK;NGAAFKDEMKK;AFKDEMKKSDK;KSDKIAAAIVL;KIAAAIVLRGV;IAAAIVLRG
VA;AAAIVLRGVAK;IVLRGVAKDGK;VLRGVAKDGKF;GVAKDGKFAVK

**Class 2**
ADAFSKADPKKSDVK;FSKADPKKS;ADAGDVKDAAAAVGA;VKDAAAAVG;ADKESVNGIAGAIK
G;VNGIAGAIK;AFSGLVADAFSKADP;LVADAFSKA;AFSKADPKKSDVKTY;FSKADPKKS;AGDV
KDAAAAVGAVS;VKDAAAAVG;AGKLFGTAAGADAGD;FGTAAGADA;AIFIVAFLALIGCKN;IVAFL
ALIG;AIVTAAGQAGQAGKK;IVTAAGQAG;AKNAIEAAIGAAGDA;IEAAIGAAG;ALVLRGVAKDGK
FAG;LRGVAKDGK;ASIFYQSIINLGNGF;FYQSIINLG;ASIFYQSIINLGNGF;YQSIINLGN;AVSGEQ
ILNAIVTAA;EQILNAIVT;AVSGEQILNAIVTAA;VSGEQILNA;DAFSKADPKKSDVKT;FSKADPKKS;
DAGDVKDAAAAVGAV;VKDAAAAVG;DEAKNAIEAAIGAAG;KNAIEAAIG;DIKKKNDQIAAALVL;IK
KKNDQIA;DKESVNGIAGAIKGI;VNGIAGAIK;DKIGNVAAGGGAGAD;IGNVAAGGG;DQIAAALVL
RGVAKD;IAAALVLRG;DTAASIFYQSIINLG;IFYQSIINL;DTAASIFYQSIINLG;SIFYQSIIN;DVKDA
AAAVGAVSGE;VKDAAAAVG;DAAAAVGAVSGEQIL;VGAVSGEQI;EAKNAIEAAIGAAGD;IEAAIG
AAG;EGVKFAPKAAADAAA;FAPKAAADA;EQILNAIVTAAGQAG;ILNAIVTAA;EQILNAIVTAAGQA
G;LNAIVTAAG;ESVNGIAGAIKGIVE;VNGIAGAIK;EVFNAFSGLVADAFS;FNAFSGLVA;EVFNAF
SGLVADAFS;VFNAFSGLV;FAPKAAADAAAADGN;FAPKAAADA;FGTAAGADAGDVKDA;FGTA
AGADA;FIEVFNAFSGLVADA;FNAFSGLVA;FIEVFNAFSGLVADA;VFNAFSGLV;FIVAFLALIGCK
NNV;IVAFLALIG;FNAFSGLVADAFSKA;FSGLVADAF;FSGLVADAFSKADPK;LVADAFSKA;FSK
ADPKKSDVKTYF;FSKADPKKS;FYQSIINLGNGFIEV;FYQSIINLG;FYQSIINLGNGFIEV;IINLGNG
FI;FYQSIINLGNGFIEV;YQSIINLGN;GADAGDVKDAAAAVG;AGDVKDAAA;GDVKDAAAAVGAVS
G;VKDAAAAVG;GEQILNAIVTAAGQA;ILNAIVTAA;GEQILNAIVTAAGQA;LNAIVTAAG;GFIEVFN
AFSGLVAD;FNAFSGLVA;GFIEVFNAFSGLVAD;VFNAFSGLV;GGGSDKIGNVAAGGG;KIGNVA
AGG;GGGSDKIGNVAAGGGA;IGNVAAGGG;GKLFGTAAGADAGDV;FGTAAGADA;GLVADAFSK
ADPKKS;AFSKADPKK;GLVADAFSKADPKKS;VADAFSKAD;GNGFIEVFNAFSGLV;FIEVFNAFS;
GNVAAGGGAGADKES;VAAGGGAGA;GSDKIGNVAAGGGAG;IGNVAAGGG;GVKFAPKAAADA
AAA;FAPKAAADA;IEVFNAFSGLVADAF;FNAFSGLVA;IEVFNAFSGLVADAF;VFNAFSGLV;IFIVA
FLALIGCKNN;IVAFLALIG;IFYQSIINLGNGFIE;FYQSIINLG;IFYQSIINLGNGFIE;IINLGNGFI;IFYQ

SIINLGNGFIE;YQSIINLGN;IGNVAAGGGAGADKE;VAAGGGAGA;IKKKNDQIAAALVLR;DQIAAA
LVL;ILNAIVTAAGQAGQA;ILNAIVTAA;ILNAIVTAAGQAGQA;IVTAAGQAG;ISSAIFIVAFLALIG;FI
VAFLALI;IVAFLALIGCKNNVG;IVAFLALIG;IVTAAGQAGQAGKKA;IVTAAGQAG;IAAALVLRGVA
KDGK;ALVLRGVAK;IAAALVLRGVAKDGK;IAAALVLRG;KAGKLFGTAAGADAG;FGTAAGADA;K
DAAAAVGAVSGEQI;AAAVGAVSG;KESVNGIAGAIKGIV;VNGIAGAIK;KFAPKAAADAAAADG;FA
PKAAADA;KIGNVAAGGGAGADK;VAAGGGAGA;KISSAIFIVAFLALI;IFIVAFLAL;KKAGKLFGTAA
GADA;LFGTAAGAD;KKKNDQIAAALVLRG;DQIAAALVL;KKNDQIAAALVLRGV;IAAALVLRG;KLF
GTAAGADAGDVK;FGTAAGADA;KNAIEAAIGAAGDAD;IEAAIGAAG;KNDQIAAALVLRGVA;IAAA
LVLRG;KVEGVKFAPKAAADA;VKFAPKAAA;LFGTAAGADAGDVKD;FGTAAGADA;LNAIVTAAG
QAGQAG;IVTAAGQAG;LVADAFSKADPKKSD;FSKADPKKS;MKKISSAIFIVAFLA;MKKISSAIF;N
AFSGLVADAFSKAD;FSGLVADAF;NAIEAAIGAAGDADF;IEAAIGAAG;NAIVTAAGQAGQAGK;IV
TAAGQAG;NDQIAAALVLRGVAK;IAAALVLRG;NGFIEVFNAFSGLVA;FIEVFNAFS;NGFIEVFNAF
SGLVA;VFNAFSGLV;QILNAIVTAAGQAGQ;ILNAIVTAA;QILNAIVTAAGQAGQ;IVTAAGQAG;QIA
AALVLRGVAKDG;IAAALVLRG;QSIINLGNGFIEVFN;IINLGNGFI;SAIFIVAFLALIGCK;IVAFLALIG;
SDKIGNVAAGGGAGA;IGNVAAGGG;SGEQILNAIVTAAGQ;ILNAIVTAA;SGEQILNAIVTAAGQ;L
NAIVTAAG;SIFYQSIINLGNGFI;FYQSIINLG;SIFYQSIINLGNGFI;YQSIINLGN;SIINLGNGFIEVFN
A;IINLGNGFI;SSAIFIVAFLALIGC;IVAFLALIG;TAASIFYQSIINLGN;FYQSIINLG;VADAFSKADPK
KSDV;FSKADPKKS;VEGVKFAPKAAADAA;FAPKAAADA;VFNAFSGLVADAFSK;VFNAFSGLV;V
GAVSGEQILNAIVT;VSGEQILNA;VKDAAAAVGAVSGEQ;VKDAAAAVG;VKFAPKAAADAAAAD;F
APKAAADA;VSGEQILNAIVTAAG;ILNAIVTAA;YQSIINLGNGFIEVF;IINLGNGFI;YQSIINLGNGFIE
VF;YQSIINLGN;AAALVLRGVAKDGKF;LRGVAKDGK;AAALVLRGVAKDGKF;VLRGVAKDG;AAA
VGAVSGEQILNA;VGAVSGEQI;AALVLRGVAKDGKFA;LRGVAKDGK;AALVLRGVAKDGKFA;VL
RGVAKDG;AASIFYQSIINLGNG;FYQSIINLG;AASIFYQSIINLGNG;YQSIINLGN;AAAAVGAVSGE
QILN;VGAVSGEQI

# Fig. 41.

**<CAA59725 outer surface protein A;Protein;Borrelia afzelii>**

**MHC Type 8-mer**
A0101
A0201 YLLGIGLI;SLDEKNSA
A0202 YLLGIGLI;LIACKQNV;LLGIGLIL;KLTIADDL;SLDEKNSA;GIGLILAL;ALNDTNTT
A0203 YLLGIGLI;LIACKQNV;ALNDTNTT;LLGIGLIL;GLILALIA;SLDEKNSA
A0204 LLGIGLIL;ATVDKIEL;LIACKQNV
A0206 YLLGIGLI;ATVDKIEL;FTLEGKVA;LIACKQNV
A0211
YLLGIGLI;DLPGEMKV;SLDEKNSA;TLDELKNA;LLGIGLIL;ALNDTNTT;LIACKQNV;EIAKSGEV
;KEDGKTLV;EVLKNFTL;GLILALIA;GIGLILAL;TKQDTITV;ATVDKIEL;KLTIADDL;VANDKVT
L
A0212
      YLLGIGLI;DLPGEMKV;SLDEKNSA;LLGIGLIL;TLDELKNA;LIACKQNV;ALNDTNTT;FT
LEGKVA
A0216
DLPGEMKV;YLLGIGLI;LLGIGLIL;SLDEKNSA;EIAKSGEV;ALNDTNTT;LIACKQNV;GTGKAKEV
;TLDELKNA;EVLKNFTL;GIGLILAL;ATVDKIEL;GLILALIA
A0219 YLLGIGLI;DLPGEMKV;LLGIGLIL;SLDEKNSA;LIACKQNV;ALNDTNTT;TKQDTITV
A0301
ILALIACK;KTTFELFK;VSRKVSSK;LTISVNSK;TTQLVFTK;TLSKEIAK;KTGAWDSK;KVLVSKEK
;TISVNSKK;MTRENGTK;SLKATVDK;TIADDLSK;KLEYTEMK
A1101
TTQLVFTK;KTTFELFK;LTISVNSK;TIADDLSK;KTGAWDSK;TISVNSKK;TVDKIELK;KVLVSKEK
;TLSKEIAK;ILALIACK;NVSSLDEK;SLKATVDK;NTTQATKK;VSRKVSSK;MTRENGTK;KLEYTEM
K;GTKDDKSK
A2301 KYLLGIGL;KTSTDEMF
A2402
A2403 KYLLGIGL
A2601 DTITVQKY
A2602 DTITVQKY;EIKTLDEL;EVLKNFTL;KTSTDEMF;SVDLPGEM;EIAKSGEV
A2902 DTITVQKY
A3001
MTRENGTK;KVLVSKEK;VSRKVSSK;KTTFELFK;TTQLVFTK;SLKATVDK;KTGAWDSK;GTKDDKSK
;KLEYTEMK;LTISVNSK
A3002
A3101 TTQLVFTK;LSAKTMTR;KTTFELFK;MTRENGTK;KVLVSKEK
A3301 EMKVLVSK;TTQLVFTK
A6801
LTISVNSK;NTTQATKK;TTQLVFTK;NVSSLDEK;LSAKTMTR;KTTFELFK;TISVNSKK;MTRENGTK
;ILALIACK;TIADDLSK;TVDKIELK;DTITVQKY;ELFKEDGK;EMKVLVSK;ELKGTSDK;EGTAVEI
K;TTFELFKE;TLSKEIA
A6802 EIAKSGEV;TSTLTISV;LIACKQNV;EVLKNFTL
A6901 EVLKNFTL;EIAKSGEV;FTLEGKVA;YLLGIGLI;LIACKQNV
B0702 LPGEMKVL
B0801
B0802
B1501
B1801
B2705
B3501 LPGEMKVL;TAVEIKTL
B3901 FKEDGKTL;TRENGTKL;VKEGTVTL
B4001 GELSAKTM

```
B4002
B4402
B4403
B4501
B5101 LPGEMKVL
B5301 LPGEMKVL
B5401
B5701 ATKKTGAW
B5801 KTSTDEMF
```

**Allele 9-mer**
```
A0101
A0201 YLLGIGLIL;KTSTLTISV;ALIACKQNV;LLGIGLILA;FTKQDTITV
A0202
LIACKQNV;TLDELKNAL;YLLGIGLIL;KTSTLTISV;GIGLILALI;LLGIGLILA;KSGEVTVAL;SL
KATVDKI;FTKQDTITV;DLSKTTFEL;ALNDTNTTQ
A0203
FTKQDTITV;ALIACKQNV;SLKATVDKI;YLLGIGLIL;KTSTLTISV;LLGIGLILA;GIGLILALI;G
TNLEGTAV;TIADDLSKT
A0204 ALIACKQNV;YLLGIGLIL;FTKQDTITV
A0206
FTKQDTITV;KTSTLTISV;YLLGIGLIL;ALIACKQNV;LLGIGLILA;KEVLKNFTL;KVANDKVTL;F
KEDGKTLV
A0211
YLLGIGLIL;TLDELKNAL;ALIACKQNV;LLGIGLILA;DLSKTTFEL;NLEGTAVEI;ALNDTNTTQ;K
TSTLTISV;FTKQDTITV;KVANDKVTL;EVKEGTVTL;EMFNEKGEL;FKEDGKTLV;DLPGEMKVL;SL
DEKNSAS;TLEVKEGTV;SLKATVDKI;VDLPGEMKV;IAKSGEVTV;ANDKVTLEV;GKYSLKATV
A0212
YLLGIGLIL;TLDELKNAL;ALIACKQNV;ALNDTNTTQ;VDLPGEMKV;LLGIGLILA;SLDEKNSAS;F
KEDGKTLV;DLSKTTFEL;FTKQDTITV;IAKSGEVTV;NLEGTAVEI;EVKEGTVTL;TLEVKEGTV
A0216
YLLGIGLIL;DLSKTTFEL;TLDELKNAL;ALIACKQNV;NLEGTAVEI;LLGIGLILA;ALNDTNTTQ;T
LEVKEGTV;EMFNEKGEL;FTKQDTITV;FKEDGKTLV;IAKSGEVTV;GKYSLKATV;EVKEGTVTL;KV
ANDKVTL;KTSTLTISV;SLKATVDKI;DLPGEMKVL
A0219
YLLGIGLIL;DLSKTTFEL;TLDELKNAL;ALIACKQNV;ALNDTNTTQ;NLEGTAVEI;FKEDGKTLV;L
LGIGLILA
A0301
       LVSRKVSSK;KTTQLVFTK;LTISVNSKK;LILALIACK;TLTISVNSK;KQDTITVQK;VTLSK
EIAK
A1101
KTTQLVFTK;STDEMFNEK;LTISVNSKK;ATVDKIELK;LTIADDLSK;LILALIACK;VTLSKEIAK;L
VSRKVSSK;SVDLPGEMK;TLTISVNSK;KQDTITVQK;TMTRENGTK;YSLKATVDK;SKTTFELFK;GS
GVLEGTK;DTNTTQATK
A2301 KYDSAGTNL;KYLLGIGLI
A2402 KYLLGIGLI
A2403 KYLLGIGLI;KYDSAGTNL
A2601 EVKEGTVTL
A2602 EVKEGTVTL;DLPGEMKVL
A2902
A3001
       KTTQLVFTK;YSLKATVDK;ATVDKIELK;SRKVSSKDK;TGKAKEVLK;KSDGTGKAK;KKTGA
WDSK
A3002
A3101 KTTQLVFTK;LTISVNSKK;LILALIACK;KQDTITVQK;VTLSKEIAK
A3301 ELSAKTMTR;DTNTTQATK
```

A6801
LTISVNSKK;DTNTTQATK;ELSAKTMTR;KTTQLVFTK;EIKTLDELK;LTIADDLSK;TLTISVNSK;S
TDEMFNEK;ATVDKIELK;LVSRKVSSK;YSLKATVDK
A6802 KTSTLTISV;FTKQDTITV;DLSKTTFEL;TVALNDTNT
A6901 FTKQDTITV;EVKEGTVTL;LPGEMKVLV;DLSKTTFEL
B0702 MTRENGTKL;KVANDKVTL
B0801
B0802
B1501 YLLGIGLIL
B1801 RENGTKLEY
B2705
B3501
B3901
B4001 KEVLKNFTL;VEIKTLDEL
B4002 KEIAKSGEV;KEVLKNFTL
B4402 RENGTKLEY
B4403
B4501
B5101 LPGEMKVLV
B5301 LPGEMKVLV
B5401 LPGEMKVLV
B5701
B5801

**Allele 10-mer**
A0101
A0201 YLLGIGLILA;SLDEKNSASV;ALNDTNTTQA;LLGIGLILAL
A0202 YLLGIGLILA;LLGIGLILAL;SLDEKNSASV;ALNDTNTTQA
A0203 ALNDTNTTQA;SLDEKNSASV;YLLGIGLILA;LLGIGLILAL;VANDKVTLEV;SVNSKKTTQL
A0204 SLDEKNSASV;ALNDTNTTQA;VTLEVKEGTV;VANDKVTLEV;LLGIGLILAL;SVDLPGEMKV
A0206
SLDEKNSASV;YLLGIGLILA;LLGIGLILAL;KTLDELKNAL;GAWDSKTSTL;VANDKVTLEV;ALNDT
NTTQA
A0211
SLDEKNSASV;YLLGIGLILA;DLPGEMKVLV;LLGIGLILAL;ALNDTNTTQA;SVDLPGEMKV;VANDK
VTLEV;EIAKSGEVTV;ELFKEDGKTL;VTLEVKEGTV;TMTRENGTKL;TLDELKNALK;GAWDSKTSTL
;KTLDELKNAL;TIADDLSKTT;SKTSTLTISV;QLVFTKQDTI;SVNSKKTTQL
A0212
SLDEKNSASV;YLLGIGLILA;LLGIGLILAL;DLPGEMKVLV;ALNDTNTTQA;VTLEVKEGTV;VANDK
VTLEV;SVDLPGEMKV;EIAKSGEVTV;ELFKEDGKTL;TMTRENGTKL;KTLDELKNAL;GAWDSKTSTL
;QLVFTKQDTI
A0216
SLDEKNSASV;YLLGIGLILA;LLGIGLILAL;ALNDTNTTQA;DLPGEMKVLV;EIAKSGEVTV;SVDLP
GEMKV;VANDKVTLEV;GAWDSKTSTL;TMTRENGTKL;ELFKEDGKTL;SVNSKKTTQL;VTLEVKEGTV
;QLVFTKQDTI
A0219
SLDEKNSASV;LLGIGLILAL;YLLGIGLILA;ALNDTNTTQA;VANDKVTLEV;GAWDSKTSTL;DLPGE
MKVLV;SVDLPGEMKV;EIAKSGEVTV
A0301
KTMTRENGTK;VLKNFTLEGK;TLVSRKVSSK;STLTISVNSK;TLTISVNSKK;GLILALIACK;LSKTT
FELFK;KYSLKATVDK;TLDELKNALK;KLTIADDLSK;KIELKGTSDK;ASVDLPGEMK;GTGKAKEVLK
A1101
STLTISVNSK;KTMTRENGTK;TSTDEMFNEK;TVTLSKEIAK;GLILALIACK;KQNVSSLDEK;LSKTT
FELFK;ASVDLPGEMK;TLVSRKVSSK;TLTISVNSKK;GTGKAKEVLK;KATVDKIELK;TLDELKNALK
;GTKLEYTEMK;KLTIADDLSK;KVLVSKEKDK;VLKNFTLEGK;GVLEGTKDDK;KIELKGTSDK;GTKD
DKSKAK

```
A2301 KYLLGIGLIL;KYSLKATVDK;DLSKTTFELF
A2402 KYLLGIGLIL
A2403 KYLLGIGLIL
A2601
A2602 EIAKSGEVTV
A2902
A3001
KTMTRENGTK;VSRKVSSKDK;LSKTTFELFK;GTKLEYTEMK;KQNVSSLDEK;GTKDDKSKAK;KVLVS
KEKDK;KDKDGKYSLK;VLKNFTLEGK;STLTISVNSK;KATVDKIELK;KYSLKATVDK;EMKVLVSKEK
A3002
A3101 KYSLKATVDK;KKTTQLVFTK
A3301
A6801
DTNTTQATKK;TSTDEMFNEK;TVTLSKEIAK;STLTISVNSK;TLTISVNSKK;TLVSRKVSSK;LSKTT
FELFK;EMKVLVSKEK;GTKLEYTEMK;KTMTRENGTK
A6802 EIAKSGEVTV;TVALNDTNTT;LGIGLILALI
A6901 EIAKSGEVTV;SVDLPGEMKV;VTLEVKEGTV;YLLGIGLILA;SLDEKNSASV
B0702 GAWDSKTSTL;SVNSKKTTQL
B0801
B0802
B1501
B1801 LEVKEGTVTL
B2705
B3501 IADDLSKTTF;SASVDLPGEM
B3901 AKSGEVTVAL
B4001 LEVKEGTVTL;DEMFNEKGEL
B4002
B4402
B4403
B4501
B5101
B5301
B5401 LPGEMKVLVS
B5701
B5801
```

**Allele 11-mer**
```
A0101 MTRENGTKLEY
A0201 YLLGIGLILAL;LVFTKQDTITV;KVANDKVTLEV;LLGIGLILALI;ILALIACKQNV
A0202
ILALIACKQNV;YLLGIGLILAL;LIACKQNVSSL;LLGIGLILALI;ALNDTNTTQAT;GTSDKDNGSGV
;SLKATVDKIEL;SVNSKKTTQLV;LVFTKQDTITV;TLEGKVANDKV;KVANDKVTLEV;ELFKEDGKTL
V;KTMTRENGTKL;VLKNFTLEGKV
A0203
VLKNFTLEGKV;LIACKQNVSSL;YLLGIGLILAL;ILALIACKQNV;LLGIGLILALI;SVNSKKTTQLV
;ALNDTNTTQAT;LVFTKQDTITV;SLKATVDKIEL;GTSDKDNGSGV;KVANDKVTLEV;TLEGKVANDK
V
A0204
KVANDKVTLEV;KTMTRENGTKL;LVFTKQDTITV;YLLGIGLILAL;ILALIACKQNV;SVNSKKTTQLV
;SLKATVDKIEL;VLKNFTLEGKV
A0206
YLLGIGLILAL;KVANDKVTLEV;LVFTKQDTITV;TQLVFTKQDTI;KEIAKSGEVTV;TAVEIKTLDEL
;SAGTNLEGTAV;LLGIGLILALI;ILALIACKQNV;LIACKQNVSSL
A0211
YLLGIGLILAL;ILALIACKQNV;ELFKEDGKTLV;KVANDKVTLEV;LLGIGLILALI;LVFTKQDTITV
;TLEGKVANDKV;SVNSKKTTQLV;VLKNFTLEGKV;SLKATVDKIEL;ALNDTNTTQAT;EMFNEKGELS
```

A;SSLDEKNSASV;TLEVKEGTVTL;GTSDKDNGSGV;VDLPGEMKVLV;SVDLPGEMKVL;KVTLEVKEG
TV
A0212
YLLGIGLILAL;ELFKEDGKTLV;VLKNFTLEGKV;ILALIACKQNV;LVFTKQDTITV;ALNDTNTTQAT
;TLEGKVANDKV;LLGIGLILALI;EMFNEKGELSA;GTSDKDNGSGV;KVANDKVTLEV;SLKATVDKIE
L;SVNSKKTTQLV;SSLDEKNSASV;VDLPGEMKVLV;TLEVKEGTVTL
A0216
YLLGIGLILAL;ELFKEDGKTLV;TLEGKVANDKV;ILALIACKQNV;VLKNFTLEGKV;LVFTKQDTITV
;KVANDKVTLEV;SLKATVDKIEL;SVNSKKTTQLV;LLGIGLILALI;TLEVKEGTVTL;ALNDTNTTQA
T;EMFNEKGELSA;GTSDKDNGSGV;SSLDEKNSASV;NLEGTAVEIKT;LIACKQNVSSL
A0219
YLLGIGLILAL;ILALIACKQNV;TLEGKVANDKV;ALNDTNTTQAT;LLGIGLILALI;LVFTKQDTITV
;ELFKEDGKTLV;SVNSKKTTQLV;LIACKQNVSSL
A0301 KTLVSRKVSSK;STLTISVNSKK;KTLDELKNALK;KTSTDEMFNEK;FTKQDTITVQK
A1101
STLTISVNSKK;TTFELFKEDGK;KTSTDEMFNEK;KTLDELKNALK;KTLVSRKVSSK;TSTLTISVNSK
;GTVTLSKEIAK;SASVDLPGEMK;ATKKTGAWDSK;AVEIKTLDELK;VANDKVTLEVK;DLSKTTFELF
K;FTKQDTITVQK;LVSRKVSSKDK;EVLKNFTLEGK;YTEMKSDGTGK;FTLEGKVANDK
A2301 KYSLKATVDKI
A2402 KYSLKATVDKI;AWDSKTSTLTI
A2403 KYSLKATVDKI
A2601
A2602 TIADDLSKTTF;MTRENGTKLEY
A2902 MTRENGTKLEY
A3001
ATKKTGAWDSK;KTLVSRKVSSK;KTLDELKNALK;MTRENGTKLEY;EVKEGTVTLSK;KTSTDEMFNEK
;FTKQDTITVQK;STLTISVNSKK;SKKTTQLVFTK
A3002
A3101 KTLVSRKVSSK;KTLDELKNALK;MFNEKGELSAK;STLTISVNSKK;KTSTDEMFNEK
A3301 EVKEGTVTLSK;DLSKTTFELFK
A6801
TTFELFKEDGK;STLTISVNSKK;FTKQDTITVQK;TSTLTISVNSK;EVKEGTVTLSK;EVLKNFTLEGK
;DLSKTTFELFK;FTLEGKVANDK;KTSTDEMFNEK;YTEMKSDGTGK;LVSKEKDKDGK;SASVDLPGEM
K;GTVTLSKEIAK
A6802
TAVEIKTLDEL;DTITVQKYDSA;LVFTKQDTITV;ELFKEDGKTLV;SVNSKKTTQLV;ILALIACKQNV
;LIACKQNVSSL;EIAKSGEVTVA;NVSSLDEKNSA
A6901 LVFTKQDTITV;YLLGIGLILAL;ELFKEDGKTLV
B0702 IAKSGEVTVAL
B0801
B0802
B1501 MTRENGTKLEY;YLLGIGLILAL;VQKYDSAGTNL
B1801 FELFKEDGKTL;MTRENGTKLEY
B2705
B3501 TAVEIKTLDEL;IAKSGEVTVAL;TIADDLSKTTF;MTRENGTKLEY
B3901
B4001 FELFKEDGKTL;LEGTAVEIKTL
B4002 KEIAKSGEVTV
B4402
B4403
B4501 KEIAKSGEVTV
B5101
B5301
B5401 LPGEMKVLVSK
B5701 TTQATKKTGAW

B5801

**Allele 15-mer plus 9-mer core**
DRB1_0101
KKYLLGIGLILALIA-LLGIGLILA;KYLLGIGLILALIAC-LLGIGLILA;MKKYLLGIGLILALI-
LLGIGLILA;KTGAWDSKTSTLTIS-WDSKTSTLT;TGAWDSKTSTLTISV-
WDSKTSTLT;TKKTGAWDSKTSTLT-KTGAWDSKT;GAWDSKTSTLTISVN-
WDSKTSTLT;KKTGAWDSKTSTLTI-WDSKTSTLT;YLLGIGLILALIACK-
IGLILALIA;LLGIGLILALIACKQ-IGLILALIA;LGIGLILALIACKQN-
IGLILALIA;QLVFTKQDTITVQKY-FTKQDTITV;TQLVFTKQDTITVQK-
FTKQDTITV;TTQLVFTKQDTITVQ-FTKQDTITV;KTTQLVFTKQDTITV-
VFTKQDTIT;LVFTKQDTITVQKYD-FTKQDTITV;VTLEVKEGTVTLSKE-
VKEGTVTLS;KVTLEVKEGTVTLSK-VKEGTVTLS;KLEYTEMKSDGTGKA-
YTEMKSDGT;DKVTLEVKEGTVTLS-LEVKEGTVT;LEVKEGTVTLSKEIA-
VKEGTVTLS;LEYTEMKSDGTGKAK-YTEMKSDGT;TISVNSKKTTQLVFT-
VNSKKTTQL;ISVNSKKTTQLVFTK-VNSKKTTQL;TLTISVNSKKTTQLV-
VNSKKTTQL;TLEVKEGTVTLSKEI-VKEGTVTLS;STLTISVNSKKTTQL-
TISVNSKKT;AWDSKTSTLTISVNS-WDSKTSTLT;LTISVNSKKTTQLVF-
VNSKKTTQL;WDSKTSTLTISVNSK-WDSKTSTLT;ITVQKYDSAGTNLEG-
VQKYDSAGT;TITVQKYDSAGTNLE-VQKYDSAGT;ELFKEDGKTLVSRKV-
FKEDGKTLV;EDGKTLVSRKVSSKD-GKTLVSRKV;GIGLILALIACKQNV-
ILALIACKQ;KEDGKTLVSRKVSSK-GKTLVSRKV;VALNDTNTTQATKKT-
LNDTNTTQA;IGLILALIACKQNVS-ILALIACKQ;YTEMKSDGTGKAKEV-
YTEMKSDGT;LFKEDGKTLVSRKVS-GKTLVSRKV;DTITVQKYDSAGTNL-
VQKYDSAGT;EYTEMKSDGTGKAKE-YTEMKSDGT;QDTITVQKYDSAGTN-
VQKYDSAGT;TKLEYTEMKSDGTGK-YTEMKSDGT;FKEDGKTLVSRKVSS-
GKTLVSRKV;KQDTITVQKYDSAGT-ITVQKYDSA;EVTVALNDTNTTQAT-
VALNDTNTT;GTKLEYTEMKSDGTG-YTEMKSDGT;GEVTVALNDTNTTQA-
VALNDTNTT;NGTKLEYTEMKSDGT-TKLEYTEMK;VTVALNDTNTTQATK-
VALNDTNTT;VFTKQDTITVQKYDS-FTKQDTITV;SVNSKKTTQLVFTKQ-
VNSKKTTQL;QKYDSAGTNLEGTAV-YDSAGTNLE;VNSKKTTQLVFTKQD-
VNSKKTTQL;GLILALIACKQNVSS-ILALIACKQ;FTKQDTITVQKYDSA-
FTKQDTITV;VKEGTVTLSKEIAKS-VKEGTVTLS;EVKEGTVTLSKEIAK-
VKEGTVTLS;KSGEVTVALNDTNTT-EVTVALNDT;DEMFNEKGELSAKTM-
MFNEKGELS;SGEVTVALNDTNTTQ-VALNDTNTT;TDEMFNEKGELSAKT-
MFNEKGELS;TVQKYDSAGTNLEGT-VQKYDSAGT;VQKYDSAGTNLEGTA-
VQKYDSAGT;ALNDTNTTQATKKTG-NTTQATKKT;VDLPGEMKVLVSKEK-
LPGEMKVLV;DGKTLVSRKVSSKDK-GKTLVSRKV;GKTLVSRKVSSKDKT-
GKTLVSRKV;FNEKGELSAKTMTRE-EKGELSAKT;SSLDEKNSASVDLPG-
EKNSASVDL;LNDTNTTQATKKTGA-NTTQATKKT;VSSLDEKNSASVDLP-
EKNSASVDL;NVSSLDEKNSASVDL-LDEKNSASV;TVALNDTNTTQATKK-
LNDTNTTQA;EMFNEKGELSAKTMT-EKGELSAKT
DRB1_0301
DRB1_0401
QLVFTKQDTITVQKY-FTKQDTITV;TQLVFTKQDTITVQK-FTKQDTITV;LVFTKQDTITVQKYD-
FTKQDTITV;TTQLVFTKQDTITVQ-FTKQDTITV;KTTQLVFTKQDTITV-
KTTQLVFTK;LEYTEMKSDGTGKAK-MKSDGTGKA;KLEYTEMKSDGTGKA-
YTEMKSDGT;EYTEMKSDGTGKAKE-MKSDGTGKA;YTEMKSDGTGKAKEV-
MKSDGTGKA;TEMKSDGTGKAKEVL-MKSDGTGKA
DRB1_0404
GEVTVALNDTNTTQA-VALNDTNTT;EVTVALNDTNTTQAT-LNDTNTTQA;VTVALNDTNTTQATK-
LNDTNTTQA;TVALNDTNTTQATKK-LNDTNTTQA;GKAKEVLKNFTLEGK-
VLKNFTLEG;VALNDTNTTQATKKT-LNDTNTTQA;AKEVLKNFTLEGKVA-
VLKNFTLEG;KAKEVLKNFTLEGKV-VLKNFTLEG;TGKAKEVLKNFTLEG-
AKEVLKNFT;KEVLKNFTLEGKVAN-VLKNFTLEG
DRB1_0405

TITVQKYDSAGTNLE-VQKYDSAGT;ITVQKYDSAGTNLEG-YDSAGTNLE;TVQKYDSAGTNLEGT-
YDSAGTNLE;VQKYDSAGTNLEGTA-YDSAGTNLE;QKYDSAGTNLEGTAV-YDSAGTNLE
DRB1_0701
DRB1_0802
DRB1_0901
DRB1_1101
DRB1_1302
STLTISVNSKKTTQL-ISVNSKKTT;TLTISVNSKKTTQLV-ISVNSKKTT;LTISVNSKKTTQLVF-
ISVNSKKTT;EVTVALNDTNTTQAT-VALNDTNTT;GEVTVALNDTNTTQA-
VALNDTNTT;KTSTLTISVNSKKTT-LTISVNSKK;VTVALNDTNTTQATK-
VALNDTNTT;TSTLTISVNSKKTTQ-ISVNSKKTT;KSGEVTVALNDTNTT-
EVTVALNDT;SGEVTVALNDTNTTQ-VALNDTNTT;TISVNSKKTTQLVFT-
ISVNSKKTT;ISVNSKKTTQLVFTK-ISVNSKKTT;VALNDTNTTQATKKT-
VALNDTNTT;TVALNDTNTTQATKK-VALNDTNTT
DRB1_1501
AKEVLKNFTLEGKVA-LKNFTLEGK;KEVLKNFTLEGKVAN-LKNFTLEGK;GKAKEVLKNFTLEGK-
VLKNFTLEG;KAKEVLKNFTLEGKV-LKNFTLEGK;EVLKNFTLEGKVAND-
LKNFTLEGK;VLKNFTLEGKVANDK-LKNFTLEGK;LKNFTLEGKVANDKV-LKNFTLEGK
DRB3_0101
DRB4_0101
DRB5_0101
LPGEMKVLVSKEKDK-MKVLVSKEK;GEMKVLVSKEKDKDG-MKVLVSKEK;VDLPGEMKVLVSKEK-
VDLPGEMKV;PGEMKVLVSKEKDKD-MKVLVSKEK;DLPGEMKVLVSKEKD-MKVLVSKEK

**<YP_853838 outer surface protein B;Protein;Borrelia afzelii PKo**

**Allele 8-mer**
A0101
A0201
YLLVFALV;VLAEENSV;FLTDGTIT;KQYLLVFA;LTDGTITV;LLVFALVL;LVFALVLA;FALVLALI
A0202
VLAEENSV;YLLVFALV;LLVFALVL;FLTDGTIT;LVFALVLA;KTKDFVFL;YLNDTTSG;KQYLLVFA
;ATVDTVEL;AMTIADDL;FVFLTDGT;LVGGKTTV
A0203
VLAEENSV;YLLVFALV;LVGGKTTV;FLTDGTIT;KQYLLVFA;LLVFALVL;KTKDFVFL;TLKNGIKL
;VVKKQGSV;ALVLALIA;YLNDTTSG;KTKVAMTI;KLTEGTIT;LTDGTITV;LVFALVLA;ATKAVET
L;ATVDTVEL;
A0204
VLAEENSV;YLLVFALV;LVGGKTTV;ATVDTVEL;LTDGTITV
A0206
YLLVFALV;KQYLLVFA;VLAEENSV;LTDGTITV;ATVDTVEL;FLTDGTIT;FALVLALI;LVGGKTTV
;LLVFALVL;IADDLNTI;FVFLTDGT;KTKDFVFL;LVFALVLA
A0211
YLLVFALV;VLAEENSV;LTDGTITV;LVGGKTTV;FLTDGTIT;LLVFALVL;TLKNGIKL;SLVGGKTT
;PLFNGNKI;YLNDTTSG;KLTEGTIT;EMTDSSNA;LVFALVLA;ATVDTVEL;IADDLNTI;KTKDFVF
L;ALVLALIA;TLTREIEQ;DLAALKAA;EIKDLAAL;IKLEGSLV;KLDSKKIT
A0212
YLLVFALV;VLAEENSV;LTDGTITV;LVGGKTTV;FLTDGTIT;LLVFALVL;YLNDTTSG;TLKNGIKL
;PLFNGNKI;SLVGGKTT;EIKDLAAL
A0216
YLLVFALV;VLAEENSV;LVGGKTTV;LTDGTITV;TLKNGIKL;FLTDGTIT;LLVFALVL;PLFNGNKI
;SLVGGKTT;ALVLALIA;ATVDTVEL;TLTREIEQ;KLTEGTIT;VVKKQGSV
A0219
YLLVFALV;VLAEENSV;LTDGTITV;LVGGKTTV;FLTDGTIT;LLVFALVL;YLNDTTSG;IADDLNTI
A0301
ALIACSQK;KIFVSKEK;TTSGSTKK;KTGSEVVK;SVIKESYK;AALKAALK;ETYDASNK;TISADSKK

A1101
SVIKESYK;TTSGSTKK;ALIACSQK;TVDTVELK;AALKAALK;TISADSKK;KIFVSKEK;LTISADSK
;ETYDASNK;KTGSEVVK;KANKLDSK;SQKGTEPK;GTITVQAY;GTKADKTK;SADSKKTK
A2301
QYLLVFAL;TYDASNKK;AYDTAGTK;LFNGNKIF
A2402 QYLLVFAL;VFLTDGTI;VFALVLAL
A2403 QYLLVFAL
A2601 NTITVETY;EIKDLAAL;GTITVQAY;DTVELKGV
A2602 EIKDLAAL;NTITVETY;GTITVQAY;TTLEYSEM;KTKDFVFL;ETTNTLTI
A2902 GTITVQAY
A3001
KIFVSKEK;SQKGTEPK;KTKVAMTI;AALKAALK;GTKADKTK;KTKDFVFL;TTSGSTKK;KEKNSAGK
;KANKLDSK;KTGSEVVK;SGKLEGTK;KKQGSVIK;SVIKESYK
A3002
A3101 KTKDFVFL;SAGKYELR;KIFVSKEK;KANKLDSK
A3301 DTTSGSTK
A6801
ETYDASNK;TTSGSTKK;LTISADSK;DTTSGSTK;SVIKESYK;NTITVETY;TISADSKK;TVDTVELK
;ETLKNGIK;NTPKDSKK;SAGKYELR;EGTITLTR;ELKGVSDK
A6802 NSAGKYEL;DTVELKGV;ETTNTLTI;LVFALVLA;FVFLTDGT;ATVDTVEL
A6901
LTDGTITV;YLLVFALV;ETTNTLTI;TTLEYSEM;DTVELKGV;FALVLALI;ATVDTVEL;LVFALVLA
B0702 LAALKAAL
B0801
B0802
B1501 GTITVQAY
B1801
B2705
B3501 LAALKAAL;NTITVETY
B3901 TRENETTLB4001 AEENSVPL
B4002 EENSVPLF;AEENSVPL
B4402 EENSVPLF
B4403 EENSVPLF;SEIKDLAA
B4501 AEENSVPL;EENSVPLF
B5101 FALVLALI
B5301 FALVLALI
B5401 FALVLALI
B5701 STKKTATW
B5801 STKKTATW

**Allele 9-mer**
A0101 MTDSSNATK
A0201
FLTDGTITV;KLTEGTITL;YLLVFALVL;KQYLLVFAL;LVFALVLAL;LLVFALVLA;YLNDTTSGS;T
IADDLNTI;SLVGGKTTV
A0202
FLTDGTITV;LLVFALVLA;YLNDTTSGS;KLTEGTITL;TIADDLNTI;KQYLLVFAL;LVFALVLAL;K
LEGNSSEI;FVSKEKNSA;SLVGGKTTV;YLLVFALVL
A0203
FLTDGTITV;TIADDLNTI;SLVGGKTTV;KLTEGTITL;LLVFALVLA;YLNDTTSGS;KQYLLVFAL;G
IKLEGSLV;ELRATVDTV;KTKVAMTIA;LVFALVLAL;YLLVFALVL;TTNTLTISA
A0204 FLTDGTITV;SLVGGKTTV;KLTEGTITL;KLEGNSSEI;TVLAEENSV;YLLVFALVL
A0206
FLTDGTITV;KLTEGTITL;KQYLLVFAL;TIADDLNTI;SLVGGKTTV;VQAYDTAGT;SEIKDLAAL;L
LVFALVLA;SQDHNDQEI;TVLAEENSV;YLLVFALVL;LVFALVLAL;ITVQAYDTA;FVFLTDGTI;YL
NDTTSGS

A0211
FLTDGTITV;KLTEGTITL;SLVGGKTTV;YLLVFALVL;KLEGNSSEI;TIADDLNTI;DLAALKAAL;E
LRATVDTV;YLNDTTSGS;LVFALVLAL;LLVFALVLA;GKYELRATV;LFNGNKIFV;TVLAEENSV;VL
ALIACSQ;KQYLLVFAL;EMTDSSNAT;KLDSKKITR
A0212
FLTDGTITV;SLVGGKTTV;YLLVFALVL;KLTEGTITL;YLNDTTSGS;ELRATVDTV;DLAALKAAL;L
VFALVLAL;TVLAEENSV;KLEGNSSEI;TIADDLNTI;GKYELRATV;ITRENETTL;LFNGNKIFV;LL
VFALVLA
A0216
FLTDGTITV;SLVGGKTTV;KLTEGTITL;ELRATVDTV;YLLVFALVL;KLEGNSSEI;DLAALKAAL;L
FNGNKIFV;TVLAEENSV;GKYELRATV;TIADDLNTI;LLVFALVLA;EVVKKQGSV;VLALIACSQ;LV
FALVLAL;YLNDTTSGS
A0219
FLTDGTITV;SLVGGKTTV;KLTEGTITL;ELRATVDTV;YLNDTTSGS;YLLVFALVL;TIADDLNTI;T
VLAEENSV;KLEGNSSEI;DLAALKAAL
A0301
LVGGKTTVK;IINSDNTPK;KTGSEVVKK;LAALKAALK;MTDSSNATK;ATKAVETLK;KANKLDSKK;
A1101
ATVDTVELK;ATKAVETLK;SVPLFNGNK;MTDSSNATK;IINSDNTPK;KTGSEVVKK;QAYDTAGTK;L
TISADSKK;ETYDASNKK;KANKLDSKK;TLTISADSK;LAALKAALK;CSQKGTEPK;GSVIKESYK;LV
GGKTTVK;ISADSKKTK;LALIACSQK;
A2301 AYDTAGTKL;TWNETTNTL;ETYDASNKK
A2402 VFALVLALI;QYLLVFALV;TWNETTNTL
A2403 AYDTAGTKL
A2601 ETTLEYSEM;ETLKNGIKL
A2602 ETTLEYSEM;EVVKKQGSV;ETLKNGIKL
A2902
A3001
ATKAVETLK;ATVDTVELK;KTKDFVFLT;CSQKGTEPK;QAYDTAGTK;ETYDASNKK;KTGSEVVKK;K
TKVAMTIA;KANKLDSKK;MTDSSNATK;IINSDNTPK;SVPLFNGNK
A3002
A3101 KLDSKKITR;ATKAVETLK;MTDSSNATK;KANKLDSKK;KTKDFVFLT
A3301 EIKDLAALK;DTTSGSTKK;NSAGKYELR
A6801
ETYDASNKK;EIKDLAALK;LTISADSKK;DTTSGSTKK;NSAGKYELR;MTDSSNATK;QAYDTAGTK;A
TVDTVELK;LAALKAALK;SVPLFNGNK;TLTISADSK;ATKAVETLK;LALIACSQK;IINSDNTPK
A6802
TTNTLTISA;EVVKKQGSV;TTVKLTEGT;LVFALVLAL;TIADDLNTI;DLAALKAAL;FVFLTDGTI;E
TTLEYSEM;TVLAEENSV;NSSEIKDLA;FLTDGTITV;SVIKESYKA
A6901
FLTDGTITV;ETLKNGIKL;TTLEYSEMT;TIADDLNTI;EVVKKQGSV;LVFALVLAL;TVLAEENSV;T
TNTLTISA
B0702 VPLFNGNKI;TPKDSKKDL;KADKTKVAM;RATVDTVEL;LAEENSVPL
B0801
B0802
B1501 KQYLLVFAL;LVFALVLAL;RENETTLEY
B1801 RENETTLEY;SEIKDLAAL;NETTNTLTI;IEQDGKVKI
B2705 KQYLLVFAL;
B35 01 LAEENSVPL;
B3901
B4001 SEIKDLAAL;AEENSVPLF
B4002 AEENSVPLF;REIEQDGKV;RENETTLEY
B4402 RENETTLEY
B4403 RENETTLEY;EENSVPLFN;AEENSVPLF;REIEQDGKV;
B4501 RENETTLEY;AEENSVPLF;REIEQDGKV
B5101 VPLFNGNKI

B5301 VPLFNGNKI
B5401 FALVLALIA;VPLFNGNKI
B5701
B5801 GSTKKTATW

**Allele 10-mer**
A0101
A0201
YLLVFALVLA;KQYLLVFALV;VLAEENSVPL;LLVFALVLAL;KLTEGTITLT;YLNDTTSGST;LVFAL
VLALI;FLTDGTITVQ;IADDLNTITV;
A0202
YLLVFALVLA;VLAEENSVPL;LVFALVLALI;KQYLLVFALV;LLVFALVLAL;PLFNGNKIFV;FLTDG
TITVQ;MTIADDLNTI;KLTEGTITLT;IADDLNTITV
A0203
VLAEENSVPL;YLNDTTSGST;LLVFALVLAL;KQYLLVFALV;KLTEGTITLT;MTIADDLNTI;YLLVF
ALVLA;FLTDGTITVQ;LVFALVLALI;
A0204 VLAEENSVPL;LLVFALVLAL
A0206
KQYLLVFALV;MTIADDLNTI;VLAEENSVPL;LLVFALVLAL;ATWNETTNTL;KLTEGTITLT;IADDL
NTITV;YLLVFALVLA;YELRATVDTV;FLTDGTITVQ;KAVETLKNGI;YLNDTTSGST;LVFALVLALI
;SQDHNDQEII;LTVLAEENSV
A0211
PLFNGNKIFV;VLAEENSVPL;YLLVFALVLA;IADDLNTITV;FLTDGTITVQ;KLTEGTITLT;LLVFA
LVLAL;YLNDTTSGST;TVDTVELKGV;LVFALVLALI;KQYLLVFALV;KITRENETTL;VFLTDGTITV
;LTDGTITVQA;SLVGGKTTVK;ATWNETTNTL;YELRATVDTV
A0212
PLFNGNKIFV;VLAEENSVPL;FLTDGTITVQ;YLLVFALVLA;LLVFALVLAL;YLNDTTSGST;IADDL
NTITV;TVDTVELKGV;KLTEGTITLT;VFLTDGTITV;KITRENETTL;YELRATVDTV
A0216
PLFNGNKIFV;VLAEENSVPL;FLTDGTITVQ;IADDLNTITV;YLLVFALVLA;LLVFALVLAL;KLTEG
TITLT;YLNDTTSGST;KITRENETTL;LVGGKTTVKL;ATWNETTNTL;TVDTVELKGV;VFLTDGTITV
A0219
FLTDGTITVQ;IADDLNTITV;PLFNGNKIFV;VLAEENSVPL;YLNDTTSGST;LLVFALVLAL;TVDTV
ELKGV;YLLVFALVLA;ATWNETTNTL;KLTEGTITLT;YELRATVDTV;
A0301
SLVGGKTTVK;VLALIACSQK;DLAALKAALK;KLEGSLVGGK;NTLTISADSK;VVKKQGSVIK;KLEGN
SSEIK;VQAYDTAGTK
A1101
TVETYDASNK;NTLTISADSK;TISADSKKTK;RATVDTVELK;VLALIACSQK;AVETLKNGIK;TVELK
GVSDK;NSVPLFNGNK;TLTISADSKK;SLVGGKTTVK;VQAYDTAGTK;KLEGSLVGGK;VIKESYKANK
;EIINSDNTPK;VVKKQGSVIK;VSKEKNSAGK;VSDKNNGSGK
A2301 QYLLVFALVL;SYKANKLDSK
A2402 QYLLVFALVL
A2403 QYLLVFALVL
A2601
A2602 EVVKKQGSVI
A2902
A3001
RATVDTVELK;KLEGNSSEIK;LTREIEQDGK;VVKKQGSVIK;SYKANKLDSK;VSKEKNSAGK;VIKES
YKANK;ACSQKGTEPK;KLEGSLVGGK
A3002
A3101 KNSAGKYELR
A3301
A6801
NATKAVETLK;NTLTISADSK;TVETYDASNK;LTEGTITLTR;EIINSDNTPK;DLAALKAALK;NSVPL

FNGNK;EMTDSSNATK;TLTISADSKK;VLALIACSQK;TISADSKKTK;LTREIEQDGK;RATVDTVELK
;TVELKGVSDK
A6802
MTIADDLNTI;LVFALVLALI;ETTNTLTISA;TTVKLTEGTI;NTITVETYDA;LTVLAEENSV;EVVKK
QGSVI;SVPLFNGNKI;ETTLEYSEMT
A6901
ETTNTLTISA;MTIADDLNTI;ATWNETTNTL;ETYDASNKKT;LVFALVLALI;IADDLNTITV;MTDSS
NATKA;QAYDTAGTKL
B0702 VPLFNGNKIF
B0801
B0802
B1501 KQGSVIKESY;VLAEENSVPL
B1801 IEQDGKVKIY;YELRATVDTV;VKLTEGTITL;
B2705
B3501 VPLFNGNKIF;LAEENSVPLF;DLNTITVETY;
B3901
B4001 VETLKNGIKL
B4002
B4402
B4403
B4501
B5101
B5301 VPLFNGNKIF
B5401
B5701
B5801 SGSTKKTATW

**Allele 11-mer**
A0101 LTDGTITVQAY;MTDSSNATKAV
A0201
FLTDGTITVQA;YLLVFALVLAL;TIADDLNTITV;FVFLTDGTITV;LLVFALVLALI;SLVGGKTTVKL
;KQYLLVFALVL;AMTIADDLNTI;
A0202
FLTDGTITVQA;LLVFALVLALI;TIADDLNTITV;SLVGGKTTVKL;YLLVFALVLAL;FVFLTDGTITV
;AMTIADDLNTI;MTDSSNATKAV;VQAYDTAGTKL;NSSEIKDLAAL;KQYLLVFALVL
A0203
FLTDGTITVQA;TIADDLNTITV;LLVFALVLALI;ATVDTVELKGV;SLVGGKTTVKL;FVFLTDGTITV
;AMTIADDLNTI;YLLVFALVLAL;MTDSSNATKAV;VQAYDTAGTKL;TLKNGIKLEGS;VIKESYKANK
L;MKQYLLVFALV;
A0204
SLVGGKTTVKL;TIADDLNTITV;FLTDGTITVQA;FVFLTDGTITV;YLLVFALVLAL;ATVDTVELKGV
A0206
FLTDGTITVQA;TIADDLNTITV;FVFLTDGTITV;ATVDTVELKGV;KQYLLVFALVL;YLLVFALVLAL
;VQAYDTAGTKL;LLVFALVLALI;MKQYLLVFALV;MTDSSNATKAV;SLVGGKTTVKL;TVLAEENSVP
L;AMTIADDLNTI;
A0211
FLTDGTITVQA;YLLVFALVLAL;TIADDLNTITV;SLVGGKTTVKL;FVFLTDGTITV;DLTVLAEENSV
;LLVFALVLALI;MTDSSNATKAV;SAGKYELRATV;YLNDTTSGSTK;KLTEGTITLTR;EMTDSSNATK
A;ELRATVDTVEL;SADSKKTKDFV;ATVDTVELKGV;AMTIADDLNTI;VLAEENSVPLF;TVLAEENSV
PL;KADKTKVAMTI
A0212
YLLVFALVLAL;FLTDGTITVQA;SLVGGKTTVKL;TIADDLNTITV;FVFLTDGTITV;YLNDTTSGSTK
;SAGKYELRATV;MTDSSNATKAV;DLTVLAEENSV;LLVFALVLALI;ELRATVDTVEL;ATVDTVELKG
V
A0216
SLVGGKTTVKL;FLTDGTITVQA;TIADDLNTITV;FVFLTDGTITV;SAGKYELRATV;YLLVFALVLAL

;SADSKKTKDFV;DLTVLAEENSV;ELRATVDTVEL;LLVFALVLALI;AMTIADDLNTI;MTDSSNATKA
V;YLNDTTSGSTK;EMTDSSNATKA;ALVLALIACSQ;ATVDTVELKGV
A0219
FLTDGTITVQA;TIADDLNTITV;YLLVFALVLAL;SLVGGKTTVKL;MTDSSNATKAV;FVFLTDGTITV
;SADSKKTKDFV;YLNDTTSGSTK;ATVDTVELKGV;DLTVLAEENSV;ELRATVDTVEL;LLVFALVLAL
I;EMTDSSNATKA;
A0301
LVLALIACSQK;YLNDTTSGSTK;KLTEGTITLTR;KQGSVIKESYK;SVIKESYKANK;LFNGNKIFVSK
;GSLVGGKTTVK
A1101
SVIKESYKANK;TVQAYDTAGTK;LVLALIACSQK;SSEIKDLAALK;ITVETYDASNK;GVSDKNNGSGK
;TVETYDASNKK;IACSQKGTEPK;NTLTISADSKK;KQGSVIKESYK;LTISADSKKTK;YLNDTTSGST
K;GSLVGGKTTVK;KAVETLKNGIK;ESYKANKLDSK;FVSKEKNSAGK
A2301 SYKANKLDSKK
A2402 TWNETTNTLTI
A2403
A2601 EIEQDGKVKIY
A2602 EIEQDGKVKIY;LTDGTITVQAY;SVPLFNGNKIF;ITRENETTLEY
A2902 ITRENETTLEY
A3001
ITRENETTLEY;TVQAYDTAGTK;SYKANKLDSKK;YLNDTTSGSTK;KLEGTKADKTK;IACSQKGTEPK
;LTISADSKKTK;LFNGNKIFVSK;SVIKESYKANK;GSLVGGKTTVK;KAVETLKNGIK
A3002
A3101
KLTEGTITLTR;KQGSVIKESYK;LFNGNKIFVSK;SVIKESYKANK;SYKANKLDSKK;LVLALIACSQK
A3301
A6801
NTLTISADSKK;ITVETYDASNK;ESYKANKLDSK;DTVELKGVSDK;TVQAYDTAGTK;SVIKESYKANK
;TVETYDASNKK;LTISADSKKTK;FVSKEKNSAGK;EVVKKQGSVIK;LVLALIACSQK;YLNDTTSGST
K;IACSQKGTEPK;TLTREIEQDGK;TNTLTISADSK;EKNSAGKYELR
A6802
FVFLTDGTITV;TIADDLNTITV;MKQYLLVFALV;NSVPLFNGNKI;NSSEIKDLAAL;MTDSSNATKAV
;TVLAEENSVPL;STSQDHNDQEI;EGSLVGGKTTV;MTIADDLNTIT;ATVDTVELKGV
A6901
TIADDLNTITV;MTDSSNATKAV;FVFLTDGTITV;TVLAEENSVPL;ATVDTVELKGV;VPLFNGNKIFV
B0702 VPLFNGNKIFV
B0801 YLLVFALVLAL
B0802
B1501 ITRENETTLEY;KQYLLVFALVL;VLAEENSVPLF;VSKEKNSAGKY;YLLVFALVLAL
B1801 IEQDGKVKIYL
B2705 KQYLLVFALVL
B3501 TATWNETTNTL;LTDGTITVQAY;TVLAEENSVPL
B3901 KQYLLVFALVL
B4001 IEQDGKVKIYL;REIEQDGKVKI;KEKNSAGKYEL;LEGNSSEIKDL
B4002 REIEQDGKVKI;KEKNSAGKYEL
B4402
B4403
B4501
B5101 VPLFNGNKIFV
B5301
B5401 VPLFNGNKIFV
B5701 TSGSTKKTATW
B5801 TSGSTKKTATW

**Allele 15-mer plus 9-mer core**
DRB1_0101

SSEIKDLAALKAALK-IKDLAALKA;GNSSEIKDLAALKAA-IKDLAALKA;NSSEIKDLAALKAAL-
IKDLAALKA;EGNSSEIKDLAALKA-EGNSSEIKD;KQYLLVFALVLALIA-
VFALVLALI;YLLVFALVLALIACS-FALVLALIA;EGSLVGGKTTVKLTE-
VGGKTTVKL;KLEGSLVGGKTTVKL-LVGGKTTVK;LEGSLVGGKTTVKLT-
VGGKTTVKL;LLVFALVLALIACSQ-FALVLALIA;GSLVGGKTTVKLTEG-
VGGKTTVKL;QYLLVFALVLALIAC-FALVLALIA;LVFALVLALIACSQK-
FALVLALIA;SLVGGKTTVKLTEGT-VGGKTTVKL;MKQYLLVFALVLALI-
LVFALVLAL;VFALVLALIACSQKG-LVLALIACS;FALVLALIACSQKGT-
LVLALIACS;ETLKNGIKLEGSLVG-LKNGIKLEG;KNGIKLEGSLVGGKT-
IKLEGSLVG;TITVQAYDTAGTKLE-VQAYDTAGT;TLKNGIKLEGSLVGG-
IKLEGSLVG;LKNGIKLEGSLVGGK-IKLEGSLVG;NGIKLEGSLVGGKTT-
IKLEGSLVG;ITVQAYDTAGTKLEG-VQAYDTAGT;VGGKTTVKLTEGTIT-
VGGKTTVKL;DGTITVQAYDTAGTK-VQAYDTAGT;GTITVQAYDTAGTKL-
VQAYDTAGT;TDGTITVQAYDTAGT-TITVQAYDT;IKLEGSLVGGKTTVK-
IKLEGSLVG;LVGGKTTVKLTEGTI-VGGKTTVKL;TTVKLTEGTITLTRE-
LTEGTITLT;GKTTVKLTEGTITLT-VKLTEGTIT;KTTVKLTEGTITLTR-
LTEGTITLT;ALVLALIACSQKGTE-VLALIACSQ;KIYLNDTTSGSTKKT-
YLNDTTSGS;KDFVFLTDGTITVQA-FLTDGTITV;KVKIYLNDTTSGSTK-
IYLNDTTSG;GKVKIYLNDTTSGST-IYLNDTTSG;TVKLTEGTITLTREI-
LTEGTITLT;VKIYLNDTTSGSTKK-YLNDTTSGS;DFVFLTDGTITVQAY-
FLTDGTITV;FVFLTDGTITVQAYD-FLTDGTITV;VKLTEGTITLTREIE-
LTEGTITLT;DGKVKIYLNDTTSGS-IYLNDTTSG;AGKYELRATVDTVEL-
YELRATVDT;TVQAYDTAGTKLEGN-VQAYDTAGT;TKDFVFLTDGTITVQ-
FLTDGTITV;VQAYDTAGTKLEGNS-VQAYDTAGT;IYLNDTTSGSTKKTA-
TTSGSTKKT;NSAGKYELRATVDTV-YELRATVDT;GKYELRATVDTVELK-
YELRATVDT;KNSAGKYELRATVDT-AGKYELRAT;SAGKYELRATVDTVE-
YELRATVDT;LVLALIACSQKGTEP-LVLALIACS;GIKLEGSLVGGKTTV-
IKLEGSLVG;KTKDFVFLTDGTITV-FVFLTDGTI;GSEVVKKQGSVIKES-
VKKQGSVIK;SEVVKKQGSVIKESY-VKKQGSVIK;TGSEVVKKQGSVIKE-
VKKQGSVIK;KTGSEVVKKQGSVIK-VVKKQGSVI;VFLTDGTITVQAYDT-
FLTDGTITV;EVVKKQGSVIKESYK-VKKQGSVIK;YLNDTTSGSTKKTAT-
TTSGSTKKT;GGKTTVKLTEGTITL-VKLTEGTIT;KTATWNETTNTLTIS-
WNETTNTLT;KKTATWNETTNTLTI-WNETTNTLT;TKKTATWNETTNTLT-
ATWNETTNT;FLTDGTITVQAYDTA-FLTDGTITV;TATWNETTNTLTISA-
WNETTNTLT;LNDTTSGSTKKTATW-TTSGSTKKT;YELRATVDTVELKGV-
YELRATVDT;ATWNETTNTLTISAD-WNETTNTLT;QDGKVKIYLNDTTSG-
KVKIYLNDT;VLALIACSQKGTEPK-VLALIACSQ;QAYDTAGTKLEGNSS-
YDTAGTKLE;DKTKVAMTIADDLNT-VAMTIADDL;KYELRATVDTVELKG-YELRATVDT
DRB1_0301
DRB1_0401
KDSKKDLTVLAEENS-SKKDLTVLA;PKDSKKDLTVLAEEN-SKKDLTVLA;NTPKDSKKDLTVLAE-
SKKDLTVLA;TPKDSKKDLTVLAEE-SKKDLTVLA;DNTPKDSKKDLTVLA-
DNTPKDSKK;FNGNKIFVSKEKNSA-FNGNKIFVS;NKIFVSKEKNSAGKY-
FVSKEKNSA;GNKIFVSKEKNSAGK-FVSKEKNSA;KIFVSKEKNSAGKYE-
FVSKEKNSA;NGNKIFVSKEKNSAG-FVSKEKNSA;VKIYLNDTTSGSTKK-
YLNDTTSGS;GKVKIYLNDTTSGST-YLNDTTSGS;KVKIYLNDTTSGSTK-
YLNDTTSGS;DGKVKIYLNDTTSGS-IYLNDTTSG;KIYLNDTTSGSTKKT-
YLNDTTSGS;DSKKDLTVLAEENSV-SKKDLTVLA;SKKDLTVLAEENSVP-SKKDLTVLA
DRB1_0404
GKVKIYLNDTTSGST-YLNDTTSGS;KVKIYLNDTTSGSTK-YLNDTTSGS;VKIYLNDTTSGSTKK-
YLNDTTSGS
DRB1_0405 HNDQEIINSDNTPKD-IINSDNTPK;DQEIINSDNTPKDSK-
INSDNTPKD;NDQEIINSDNTPKDS-INSDNTPKD;QEIINSDNTPKDSKK-
INSDNTPKD;EIINSDNTPKDSKKD-INSDNTPKD;IINSDNTPKDSKKDL-
INSDNTPKD;INSDNTPKDSKKDLT-INSDNTPKD;TKKTATWNETTNTLT-TKKTATWNE
DRB1_0701

ETYDASNKKTGSEVV-SNKKTGSEV;TYDASNKKTGSEVVK-NKKTGSEVV;YDASNKKTGSEVVKK-
NKKTGSEVV;DASNKKTGSEVVKKQ-NKKTGSEVV
DRB1_0802
DRB1_0901 KQYLLVFALVLALIA-LVFALVLAL;YLLVFALVLALIACS-
LVFALVLAL;MKQYLLVFALVLALI-LVFALVLAL;QYLLVFALVLALIAC-LVFALVLAL
DRB1_1101
DRB1_1302
NSVPLFNGNKIFVSK-VPLFNGNKI;ENSVPLFNGNKIFVS-VPLFNGNKI;DGKVKIYLNDTTSGS-
VKIYLNDTT;GKVKIYLNDTTSGST-YLNDTTSGS;ETLKNGIKLEGSLVG-
LKNGIKLEG;VPLFNGNKIFVSKEK-VPLFNGNKI;SVPLFNGNKIFVSKE-
VPLFNGNKI;KAVETLKNGIKLEGS-LKNGIKLEG;TKAVETLKNGIKLEG-
VETLKNGIK;KVKIYLNDTTSGSTK-YLNDTTSGS;VKIYLNDTTSGSTKK-
YLNDTTSGS;EENSVPLFNGNKIFV-VPLFNGNKI;AVETLKNGIKLEGSL-
LKNGIKLEG;VETLKNGIKLEGSLV-LKNGIKLEG;YLLVFALVLALIACS-
LLVFALVLA;LLVFALVLALIACSQ-LLVFALVLA;QDGKVKIYLNDTTSG-
VKIYLNDTT;KQYLLVFALVLALIA-LLVFALVLA;QYLLVFALVLALIAC-
LLVFALVLA;SEVVKKQGSVIKESY-VKKQGSVIK;TGSEVVKKQGSVIKE-
VKKQGSVIK;MKQYLLVFALVLALI-LLVFALVLA;TLKNGIKLEGSLVGG-
LKNGIKLEG;KTGSEVVKKQGSVIK-VVKKQGSVI;LAEENSVPLFNGNKI-
ENSVPLFNG;GSEVVKKQGSVIKES-VKKQGSVIK;AEENSVPLFNGNKIF-
VPLFNGNKI;LKNGIKLEGSLVGGK-LKNGIKLEG;NGIKLEGSLVGGKTT-
IKLEGSLVG;EVVKKQGSVIKESYK-VKKQGSVIK;KNGIKLEGSLVGGKT-IKLEGSLVG
DRB1_1501 IKLEGSLVGGKTTVK-IKLEGSLVG;KQYLLVFALVLALIA-
LLVFALVLA;MKQYLLVFALVLALI-LLVFALVLA;QYLLVFALVLALIAC-
LLVFALVLA;YLLVFALVLALIACS-LLVFALVLA;LLVFALVLALIACSQ-LLVFALVLA;
DRB3_0101
DRB4_0101
DRB5_0101

**<CAF34027 outer surface protein VlsE;Protein;Borrelia afzelii PKo>**

**Allele 8-mer**
A0101
A0201 KLDELVSA;KISSAIFL;KIAAAIVL;ILKAIVEA;
A0202
KISSAIFL;KLDELVSA;KIAAAIVL;ILKAIVEA;LTALLVFI;ALGEKGAL;FINCKNNA;SAIFLTAL
;FLTALLVF;AIFLTALL;AAGAVTAV;AVSGEQIL;GLVADTFF
A0203
ILKAIVEA;KLDELVSA;ALKDVKAA;FINCKNNA;ALGEKGAL;KISSAIFL;AAGAVTAV;KIAAAIVL
;SISSTLKA;MIKAAEEA;SAIFLTAL;LTALLVFI;AAIVLRGV;GIKGIVDA;IAKAAGAV
A0204 ALGEKGAL;AAGAVTAV;KIAAAIVL;KLDELVSA;
A0206
KLDELVSA;KISSAIFL;SAIFLTAL;AAGAVTAV;KIAAAIVL;FIDVFNAF;FAGNANAA;AAIVLRGV
;KAAGAVTA;FLTALLVF;FESISSTL;WLEEMIKA;KASVESAV;AIFLTALL;
A0211
KLDELVSA;KISSAIFL;WLEEMIKA;ALGEKGAL;KIAAAIVL;AIFLTALL;ALKDVKAA;ILKAIVEA
;FLTALLVF;AKDGKFAV;AVSGEQIL;ALLVFINC;IFLTALLV;SISSTLKA;AAGAVTAV
A0212 KLDELVSA;ALGEKGAL;ALKDVKAA;WLEEMIKA;FLTALLVF;AKDGKFAV;AVSGEQIL
A0216
ALGEKGAL;KLDELVSA;KISSAIFL;AAGAVTAV;AIFLTALL;ILKAIVEA;WLEEMIKA;ALKDVKAA
;KIAAAIVL;AVSGEQIL
A0219 KLDELVSA;KISSAIFL;AKDGKFAV
A0301 LVADTFFK;IVLRGVAK;LLVFINCK;VSGEQILK;ESISSTLK;TLKATKGK
A1101
LVADTFFK;SAANHGAK;SSTLKATK;IVLRGVAK;VSGEQILK;ESISSTLK;GAAADIAK;AAFKDEMK
;SAVDEVSK;TFFKSDPK;GIVDAAGK;KAAEEAAK;IAAAIVLR;LLVFINCK;TLKATKGK

```
A2301 FYESIINL;KWLEEMIK;GLVADTFF
A2402 FYESIINL;IFLTALLV;
A2403 FYESIINL
A2601
A2602 FIDVFNAF
A2902
A3001
TLKATKGK;AGKALGEK;TFFKSDPK;AFKDEMKK;KGIAKGIK;IVLRGVAK;LVADTFFK;KWLEEMIK
A3002
A3101 IAAAIVLR;LVADTFFK
A3301 IAAAIVLR
A6801
ESISSTLK;IAAAIVLR;LVADTFFK;EANADAGK;LLVFINCK;SAVDEVSK;SAANHGAK;SSTLKATK
;TFFKSDPK;DSVKGIAK;AAFKDEMK;DDNGAAFK;DPANQAGK
A6802 LTALLVFI;SAIFLTAL;DSAANHGA;FNAFSGLV;NAAVGAAA
A6901 LTALLVFI
B0702 SAIFLTAL
B0801
B0802
B1501 FLTALLVF;IINLGNGF
B1801 FESISSTL
B2705
B3501 FIDVFNAF;SAIFLTAL;FAGNANAA;NAAVGAAA
B3901 FESISSTL
B4001 FESISSTL
B4002
B4402
B4403
B4501 AEEAKNPI
B5101
B5301
B5401 FAGNANAA
B5701
B5801 KSDVKTYF
```

**Allele 9-mer**

```
A0101
A0201
FLTALLVFI;KLFAGNANA;KISSAIFLT;FINCKNNAV;AIFLTALLV;QILKAIVEA;FAGNANAAV;K
AAGAVTAV
A0202
FLTALLVFI;FINCKNNAV;ILKAIVEAA;KAAGAVTAV;KLFAGNANA;FAGNANAAV;SSAIFLTAL;S
AIFLTALL;KISSAIFLT;SVESAVDEV;KAAEEAAKV;GIKGIVDAA;ALKDVKAAA;GLVADTFFK;
A0203
FINCKNNAV;FLTALLVFI;ILKAIVEAA;KAAGAVTAV;KLFAGNANA;ALKDVKAAA;GIAKGIKGI;N
LGNGFIDV;SVKTFYESI;SVKASVESA;AAAIVLRGV;IINLGNGFI;FAGNANAAV;GIKGIVDAA;SA
IFLTALL;MIKAAEEAA;ATKGKLDEL;SVKGIAKGI;AIFLTALLV;KAAEEAAKV
A0204
FLTALLVFI;KAAGAVTAV;KAAEEAAKV;FINCKNNAV;FAGNANAAV;SVESAVDEV;KLFAGNANA
A0206
FAGNANAAV;FINCKNNAV;FLTALLVFI;KAAGAVTAV;NLGNGFIDV;AIFLTALLV;KAAEEAAKV;K
LFAGNANA;SVESAVDEV;SAIFLTALL;AAAIVLRGV;QILKAIVEA;LVADTFFKS;KISSAIFLT;SS
AIFLTAL
A0211
FLTALLVFI;NLGNGFIDV;AIFLTALLV;KLFAGNANA;AVDEVSKWL;VAKDGKFAV;ALKDVKAAA;K
LDELVSAK;WLEEMIKAA;KAAGAVTAV;KAAEEAAKV;SVESAVDEV;FINCKNNAV;FAGNANAAV;IL
KAIVEAA;QILKAIVEA;GIAKGIKGI;GLVADTFFK;NADAGKLFA;
```

A0212
FLTALLVFI;NLGNGFIDV;VAKDGKFAV;ALKDVKAAA;KLFAGNANA;FINCKNNAV;FAGNANAAV;A
VDEVSKWL;AIFLTALLV;WLEEMIKAA;KLDELVSAK;GIAKGIKGI;KAAGAVTAV
A0216
NLGNGFIDV;FLTALLVFI;AIFLTALLV;ALKDVKAAA;KLFAGNANA;SVESAVDEV;FAGNANAAV;V
AKDGKFAV;AVDEVSKWL;FINCKNNAV;KAAGAVTAV;EMIKAAEEA;TLKATKGKL;QILKAIVEA;DI
AKAAGAV;WLEEMIKAA
A0219
FLTALLVFI;NLGNGFIDV;VAKDGKFAV;KAAGAVTAV;FAGNANAAV;FINCKNNAV;SVESAVDEV;A
IFLTALLV
A0301
GLVADTFFK;KLDELVSAK;KIAAAIVLR;ALGEKGALK;AVSGEQILK;ISSTLKATK;AAFKDEMKK;A
LLVFINCK
A1101
GLVADTFFK;AVSGEQILK;AAFKDEMKK;ALLVFINCK;STLKATKGK;AIVLRGVAK;KIAAAIVLR;D
TFFKSDPK;ISSTLKATK;KLDELVSAK;AAGKALGEK;ALGEKGALK;TFFKSDPKK;AVGKGNDDK;
A2301  IFLTALLVF;GFIDVFNAF;TFYESIINL;TFFKSDPKK;
A2402  IFLTALLVF;GFIDVFNAF;YFESISSTL
A2403  IFLTALLVF;GFIDVFNAF;YFESISSTL
A2601  SIINLGNGF;EVSKWLEEM;DVFNAFSGL
A2602  EVSKWLEEM;DVFNAFSGL;SIINLGNGF
A2902
A3001
STLKATKGK;AGKKAEEAK;TFFKSDPKK;GLVADTFFK;ALLVFINCK;ANHGAKADK;AAFKDEMKK;D
TFFKSDPK;AAGKALGEK;AVSGEQILK
A3002
A3101  KIAAAIVLR;ISSTLKATK
A3301  DTFFKSDPK
A6801
DTFFKSDPK;ESAVDEVSK;DSAANHGAK;KIAAAIVLR;TFFKSDPKK;GLVADTFFK;ISSTLKATK;S
TLKATKGK;AAFKDEMKK;GAAFKDEMK
A6802
DVFNAFSGL;SAIFLTALL;FINCKNNAV;SSAIFLTAL;DIAKAAGAV;DVKTYFESI;FLTALLVFI;L
VADTFFKS;FAGNANAAV;SVKTFYESI;EGGSVKASV;KAAGAVTAV;NANAAVGAA;AAAIVLRGV;IS
SAIFLTA
A6901
FAGNANAAV;FINCKNNAV;EVSKWLEEM;DIAKAAGAV;DVFNAFSGL;NLGNGFIDV;QILKAIVEA
B0702  KAAGAVTAV;IVDAAGKAL
B0801
B0802
B1501  SIINLGNGF;GFIDVFNAF
B1801
B2705
B3501  FAGNANAAV;TAVSGEQIL;IFLTALLVF;TDDDNGAAF;FSGLVADTF;GFIDVFNAF
B3901  YFESISSTL
B4001
B4002  EEAKNPIAA
B4402
B4403  EEAKNPIAA
B4501  EEAKNPIAA;AEEAKNPIA
B5101
B5301
B5401  NPIAAAIGT
B5701
B5801  SAVDEVSKW

**Allele 10-mer**
A0101
A0201 KISSAIFLTA;KLFAGNANAA;KTFYESIINL
A0202 KLFAGNANAA;KTFYESIINL;KISSAIFLTA;SSAIFLTALL;SIINLGNGFI;IAAAIVLRGV
A0203
SVKASVESAV;KISSAIFLTA;KLFAGNANAA;IAAAIVLRGV;GIAKGIKGIV;SIINLGNGFI;ATKGK
LDELV;SVKTFYESII;SAIFLTALLV;KTYFESISST;SAKKGEGGSV
A0204 KLFAGNANAA
A0206
KISSAIFLTA;SAIFLTALLV;KLFAGNANAA;AIVEAAGDPA;GVAKDGKFAV;AKAAGAVTAV;ASVES
AVDEV;QILKAIVEAA;SIINLGNGFI;KTFYESIINL;IAAAIVLRGV;GLVADTFFKS
A0211
KLFAGNANAA;GVAKDGKFAV;GIAKGIKGIV;KLDELVSAKK;KISSAIFLTA;SAIFLTALLV;DVFNA
FSGLV;SVKASVESAV;SAVDEVSKWL;IAAAIVLRGV;LFAGNANAAV;SIINLGNGFI;AVSGEQILKA
;GIVDAAGKAL;FLTALLVFIN
A0212 KLFAGNANAA;GVAKDGKFAV;GIAKGIKGIV;LFAGNANAAV;KLDELVSAKK
A0216
KLFAGNANAA;GVAKDGKFAV;GIAKGIKGIV;SVKASVESAV;DVFNAFSGLV;LFAGNANAAV;
A0219 IAAAIVLRGV;LFAGNANAAV
A0301
KALGEKGALK;KLDELVSAKK;AAIVLRGVAK;VLRGVAKDGK;SISSTLKATK;KVGGTGGDGK;SVKGI
AKGIK;ADTFFKSDPK;TAVSGEQILK;VAKDGKFAVK;SSTLKATKGK;
A1101
TALLVFINCK;SISSTLKATK;AAIVLRGVAK;AVGAAADIAK;TAVSGEQILK;KALGEKGALK;SSTLK
ATKGK;SGLVADTFFK;SVKGIAKGIK;DTFFKSDPKK;VSKWLEEMIK;KVGGTGGDGK;KLDELVSAKK
;GAAFKDEMKK;MIKAAEEAAK;AANHGAKADK;VAKDGKFAVK
A2301 TYFESISSTL;IFLTALLVFI;AFSGLVADTF;AIFLTALLVF;FSGLVADTFF
A2402 IFLTALLVFI;TYFESISSTL;AFSGLVADTF
A2403 TYFESISSTL;AFSGLVADTF;IFLTALLVFI
A2601 ESIINLGNGF;SIINLGNGFI
A2602 ESIINLGNGF;GTDDDNGAAF;AIFLTALLVF;EVSKWLEEMI
A2902
A3001
SGLVADTFFK;SVKGIAKGIK;KALGEKGALK;MIKAAEEAAK;AAIVLRGVAK;VAKDGKFAVK;VLRGV
AKDGK;AVGAAADIAK;AANHGAKADK;SSTLKATKGK;KVGGTGGDGK;
A3002
A3101 AAIVLRGVAK;
A3301
A6801
DTFFKSDPKK;TAVSGEQILK;MIKAAEEAAK;DAAGKALGEK;TALLVFINCK;DKIAAAIVLR;YFESI
SSTLK;SISSTLKATK;SVKGIAKGIK;NGAAFKDEMK;GAAFKDEMKK;SSTLKATKGK;
A6802
DVFNAFSGLV;IAAAIVLRGV;SSAIFLTALL;SAIFLTALLV;EAAGDPANQA;DSVKTFYESI;EVSKW
LEEMI;EAKNPIAAAI;SVKASVESAV;ISSAIFLTAL;NANAAVGAAA;HGAKADKDSV;SIINLGNGFI
A6901 DVFNAFSGLV;EVSKWLEEMI;EAAGDPANQA;SAIFLTALLV
B0702
B0801
B0802
B1501 AIFLTALLVF
B1801
B2705
B3501 EANADAGKLF;DPKKSDVKTY;NGFIDVFNAF;FSGLVADTFF;NANAAVGAAA
B3901
B4001
B4002
B4402

B4403
B4501 AEEAKNPIAA;EEMIKAAEEA;AEEAAKVGGT;EEAKNPIAAA
B5101
B5301
B5401
B5701 ESAVDEVSKW
B5801 ESAVDEVSKW;ISSAIFLTAL

**Allele 11-mer**
A0101
A0201 KLFAGNANAAV;FIDVFNAFSGL;KISSAIFLTAL;KIAAAIVLRGV;AIFLTALLVFI;
A0202
KISSAIFLTAL;KLFAGNANAAV;FIDVFNAFSGL;KIAAAIVLRGV;LLVFINCKNNA;FLTALLVFINC
;ISSAIFLTALL;MIKAAEEAAKV;KTYFESISSTL;LVFINCKNNAV;AIFLTALLVFI;KIGDSAANHG
A;KASVESAVDEV;ALGEKGALKDV;AVSGEQILKAI
A0203
KIAAAIVLRGV;KLFAGNANAAV;MIKAAEEAAKV;ALGEKGALKDV;KISSAIFLTAL;FIDVFNAFSGL
;IAKAAGAVTAV;LLVFINCKNNA;IINLGNGFIDV;KTYFESISSTL;LVFINCKNNAV;FLTALLVFIN
C;KIGDSAANHGA;AAADIAKAAGA
A0204 KLFAGNANAAV;KIAAAIVLRGV;ALGEKGALKDV
A0206
KLFAGNANAAV;FIDVFNAFSGL;KISSAIFLTAL;KIAAAIVLRGV;FAGNANAAVGA;KIGDSAANHGA
;KTYFESISSTL;FLTALLVFINC;KASVESAVDEV;LVFINCKNNAV;KKISSAIFLTA;AIFLTALLVF
I;SSAIFLTALLV;ALGEKGALKDV
A0211
KLFAGNANAAV;ALGEKGALKDV;KIAAAIVLRGV;FIDVFNAFSGL;KISSAIFLTAL;AIFLTALLVFI
;FLTALLVFINC;IINLGNGFIDV;KIGDSAANHGA;KATKGKLDELV;LVFINCKNNAV;MIKAAEEAAK
V;SSAIFLTALLV;AVSGEQILKAI;AVTAVSGEQIL;KTYFESISSTL
A0212
KLFAGNANAAV;ALGEKGALKDV;FIDVFNAFSGL;KIAAAIVLRGV;FLTALLVFINC;MIKAAEEAAKV
;LVFINCKNNAV;KISSAIFLTAL;IINLGNGFIDV
A0216
KLFAGNANAAV;ALGEKGALKDV;MIKAAEEAAKV;FIDVFNAFSGL;KISSAIFLTAL;KIAAAIVLRGV
;AIFLTALLVFI;LVFINCKNNAV;IINLGNGFIDV;KIGDSAANHGA;KATKGKLDELV;LLVFINCKNN
A
A0219 KLFAGNANAAV;FIDVFNAFSGL;KIAAAIVLRGV;KISSAIFLTAL;ALGEKGALKDV
A0301
FSGLVADTFFK;VTAVSGEQILK;GVAKDGKFAVK;FINCKNNAVGK;TYFESISSTLK;LTALLVFINCK
A1101
LTALLVFINCK;VTAVSGEQILK;SVESAVDEVSK;GTDDDNGAAFK;FSGLVADTFFK;TYFESISSTLK
;AAAIVLRGVAK;GVAKDGKFAVK;SAANHGAKADK;VADTFFKSDPK;AAVGAAADIAK;IVLRGVAKDG
K;AAGDPANQAGK;ESISSTLKATK;ISSTLKATKGK;FINCKNNAVGK;EVSKWLEEMIK;GIKGIVDAA
GK
A2301 TYFESISSTLK;AFSGLVADTFF;SAIFLTALLVF
A2402 AFSGLVADTFF
A2403 AFSGLVADTFF
A2601
A2602 KISSAIFLTAL;FIDVFNAFSGL
A2902
A3001 KGKLDELVSAK;TYFESISSTLK;SAKKGEGGSVK;AFKDEMKKSDK;IVLRGVAKDGK
A3002 AFSGLVADTFF
A3101 TYFESISSTLK
A3301
A6801
ESISSTLKATK;LTALLVFINCK;EVSKWLEEMIK;TYFESISSTLK;FSGLVADTFFK;EMIKAAEEAAK

;FINCKNNAVGK;VTAVSGEQILK;SVESAVDEVSK;DSVKGIAKGIK;SAANHGAKADK;NGAAFKDEMK
K;ISSTLKATKGK
A6802
ESIINLGNGFI;ISSAIFLTALL;SSAIFLTALLV;LVFINCKNNAV;FIDVFNAFSGL;KIAAAIVLRGV
;ESAVDEVSKWL;EEAKNPIAAAI;IAAAIVLRGVA;DVFNAFSGLVA;MIKAAEEAAKV
A6901 LVFINCKNNAV;FIDVFNAFSGL;
B0702
B0801
B0802
B1501 VLRGVAKDGKF;KLFAGNANAAV
B1801 YESIINLGNGF;DEANADAGKLF
B2705
B3501 NAFSGLVADTF;SAIFLTALLVF;IGTDDDNGAAF;DPKKSDVKTYF
B3901
B4001 YESIINLGNGF
B4002 EEAKNPIAAAI
B4402
B4403 EEAKNPIAAAI
B4501 EEMIKAAEEAA;AEEAKNPIAAA;EEAKNPIAAAI
B5101
B5301
B5401
B5701
B5801 SAIFLTALLVF;KTYFESISSTL;KSDKIAAAIVL;ISSAIFLTALL

**Allele 15-mer plus 9-mer core**
DRB1_0101
GKLFAGNANAAVGAA-FAGNANAAV;AGKLFAGNANAAVGA-FAGNANAAV;ADIAKAAGAVTAVSG-
IAKAAGAVT;AADIAKAAGAVTAVS-IAKAAGAVT;GAAADIAKAAGAVTA-
IAKAAGAVT;AAADIAKAAGAVTAV-IAKAAGAVT;DAGKLFAGNANAAVG-
FAGNANAAV;ADAGKLFAGNANAAV-LFAGNANAA;KLFAGNANAAVGAAA-
FAGNANAAV;KSDKIAAAIVLRGVA-IAAAIVLRG;SDKIAAAIVLRGVAK-
IAAAIVLRG;KKSDKIAAAIVLRGV-IAAAIVLRG;DKIAAAIVLRGVAKD-
IAAAIVLRG;MKKSDKIAAAIVLRG-DKIAAAIVL;IDVFNAFSGLVADTF-
VFNAFSGLV;DIAKAAGAVTAVSGE-AKAAGAVTA;FIDVFNAFSGLVADT-
VFNAFSGLV;NGFIDVFNAFSGLVA-VFNAFSGLV;GFIDVFNAFSGLVAD-
VFNAFSGLV;LFAGNANAAVGAAAD-FAGNANAAV;ISSAIFLTALLVFIN-
FLTALLVFI;SSAIFLTALLVFINC-FLTALLVFI;KISSAIFLTALLVFI-
AIFLTALLV;SAIFLTALLVFINCK-FLTALLVFI;AIFLTALLVFINCKN-
FLTALLVFI;IAKAAGAVTAVSGEQ-IAKAAGAVT;VGAAADIAKAAGAVT-
DIAKAAGAV;DVFNAFSGLVADTFF-FNAFSGLVA;GNGFIDVFNAFSGLV-
FIDVFNAFS;FAGNANAAVGAAADI-FAGNANAAV;KIAAAIVLRGVAKDG-
IAAAIVLRG;IAAAIVLRGVAKDGK-IAAAIVLRG;VFNAFSGLVADTFFK-
VFNAFSGLV;AKAAGAVTAVSGEQI-AKAAGAVTA;NADAGKLFAGNANAA-
AGKLFAGNA;TYFESISSTLKATKG-FESISSTLK;KTYFESISSTLKATK-
FESISSTLK;VKTYFESISSTLKAT-FESISSTLK;DVKTYFESISSTLKA-
FESISSTLK;SDVKTYFESISSTLK-YFESISSTL;KGIVDAAGKALGEKG-
IVDAAGKAL;KGIKGIVDAAGKALG-IVDAAGKAL;IFLTALLVFINCKNN-
FLTALLVFI;IKGIVDAAGKALGEK-IVDAAGKAL;GIKGIVDAAGKALGE-
IVDAAGKAL;AKGIKGIVDAAGKAL-IKGIVDAAG;FLTALLVFINCKNNA-
FLTALLVFI;MKKISSAIFLTALLV-MKKISSAIF;FNAFSGLVADTFFKS-
FNAFSGLVA;GKIGDSAANHGAKAD-IGDSAANHG;DGKIGDSAANHGAKA-
IGDSAANHG;GDGKIGDSAANHGAK-IGDSAANHG;KAAGAVTAVSGEQIL-
VTAVSGEQI;GGDGKIGDSAANHGA-IGDSAANHG;GNANAAVGAAADIAK-
AVGAAADIA;YFESISSTLKATKGK-FESISSTLK;AGAVTAVSGEQILKA-
VTAVSGEQI;ANAAVGAAADIAKAA-AVGAAADIA;AAGAVTAVSGEQILK-

VTAVSGEQI;TGGDGKIGDSAANHG-GKIGDSAAN;EEAKNPIAAAIGTDD-
AKNPIAAAI;FESISSTLKATKGKL-FESISSTLK;NANAAVGAAADIAKA-
AVGAAADIA;GIVDAAGKALGEKGA-IVDAAGKAL;GAVTAVSGEQILKAI-
VTAVSGEQI;NAAVGAAADIAKAAG-AVGAAADIA;EQILKAIVEAAGDPA-
LKAIVEAAG;VSGEQILKAIVEAAG-ILKAIVEAA;KKISSAIFLTALLVF-
ISSAIFLTA;IVDAAGKALGEKGAL-IVDAAGKAL;SGEQILKAIVEAAGD-
LKAIVEAAG;IAKGIKGIVDAAGKA-IKGIVDAAG;GEQILKAIVEAAGDP-
LKAIVEAAG;AAAIVLRGVAKDGKF-VLRGVAKDG;LKAIVEAAGDPANQA-
LKAIVEAAG;KGIAKGIKGIVDAAG-IAKGIKGIV;GIAKGIKGIVDAAGK-
IKGIVDAAG;EAKNPIAAAIGTDDD-IAAAIGTDD;AEEAKNPIAAAIGTD-
AKNPIAAAI;KAEEAKNPIAAAIGT-AKNPIAAAI;AAIVLRGVAKDGKFA-
VLRGVAKDG;AGNANAAVGAAADIA-ANAAVGAAA;KAIVEAAGDPANQAG-AAGDPANQA
DRB1_0301
DRB1_0401 DSVKTFYESIINLGN-FYESIINLG;SVKTFYESIINLGNG-
FYESIINLG;KTFYESIINLGNGFI-FYESIINLG;VKTFYESIINLGNGF-
FYESIINLG;KDSVKTFYESIINLG-KTFYESIIN;TFYESIINLGNGFID-
FYESIINLG;FYESIINLGNGFIDV-FYESIINLG;KTYFESISSTLKATK-
FESISSTLK;DVKTYFESISSTLKA-FESISSTLK;VKTYFESISSTLKAT-
FESISSTLK;SDVKTYFESISSTLK-YFESISSTL;TYFESISSTLKATKG-FESISSTLK
DRB1_0404
NGFIDVFNAFSGLVA-VFNAFSGLV;GNGFIDVFNAFSGLV-FIDVFNAFS;GFIDVFNAFSGLVAD-
VFNAFSGLV;FIDVFNAFSGLVADT-VFNAFSGLV;IDVFNAFSGLVADTF-
VFNAFSGLV;DVFNAFSGLVADTFF-VFNAFSGLV;VFNAFSGLVADTFFK-VFNAFSGLV
DRB1_0405
LVADTFFKSDPKKSD-DTFFKSDPK;VADTFFKSDPKKSDV-FKSDPKKSD;DSVKTFYESIINLGN-
VKTFYESII;ADTFFKSDPKKSDVK-FKSDPKKSD;DTFFKSDPKKSDVKT-
FKSDPKKSD;NGFIDVFNAFSGLVA-FIDVFNAFS;SVKTFYESIINLGNG-
YESIINLGN;VKTFYESIINLGNGF-YESIINLGN;TFFKSDPKKSDVKTY-
FKSDPKKSD;KTFYESIINLGNGFI-YESIINLGN;TFYESIINLGNGFID-
YESIINLGN;GFIDVFNAFSGLVAD-FNAFSGLVA;FIDVFNAFSGLVADT-
FNAFSGLVA;IDVFNAFSGLVADTF-FNAFSGLVA;FKSDPKKSDVKTYFE-FKSDPKKSD
DRB1_0701
KTYFESISSTLKATK-YFESISSTL;VKTYFESISSTLKAT-YFESISSTL;DVKTYFESISSTLKA-
YFESISSTL;KSDVKTYFESISSTL-KSDVKTYFE;SDVKTYFESISSTLK-
YFESISSTL;IDVFNAFSGLVADTF-VFNAFSGLV;TYFESISSTLKATKG-
YFESISSTL;LVFINCKNNAVGKGN-FINCKNNAV;TALLVFINCKNNAVG-
FINCKNNAV;NGFIDVFNAFSGLVA-VFNAFSGLV;ALLVFINCKNNAVGK-
FINCKNNAV;LLVFINCKNNAVGKG-FINCKNNAV;GFIDVFNAFSGLVAD-VFNAFSGLV
DRB1_0802
LVADTFFKSDPKKSD-FFKSDPKKS;VADTFFKSDPKKSDV-FFKSDPKKS;DTFFKSDPKKSDVKT-
FFKSDPKKS;ADTFFKSDPKKSDVK-FFKSDPKKS;GLVADTFFKSDPKKS-DTFFKSDPKDRB1_0901
KTYFESISSTLKATK-YFESISSTL;AAADIAKAAGAVTAV-IAKAAGAVT;GAAADIAKAAGAVTA-
IAKAAGAVT;VKTYFESISSTLKAT-YFESISSTL;AADIAKAAGAVTAVS-
IAKAAGAVT;DVKTYFESISSTLKA-YFESISSTL;KTFYESIINLGNGFI-
FYESIINLGDRB1_1101DRB1_1302
TFYESIINLGNGFID-IINLGNGFI;FYESIINLGNGFIDV-IINLGNGFI;LLVFINCKNNAVGKG-
VFINCKNNA;YESIINLGNGFIDVF-IINLGNGFI;ESIINLGNGFIDVFN-
IINLGNGFI;KTFYESIINLGNGFI-FYESIINLG;LVFINCKNNAVGKGN-
CKNNAVGKG;VFINCKNNAVGKGND-CKNNAVGKG;ALLVFINCKNNAVGK-
VFINCKNNA;SIINLGNGFIDVFNA-IINLGNGFI;TALLVFINCKNNAVG-
VFINCKNNA;LTALLVFINCKNNAV-VFINCKNNA
DRB1_1501
IAAAIVLRGVAKDGK-AIVLRGVAK;AAAIVLRGVAKDGKF-LRGVAKDGK;GFIDVFNAFSGLVAD-
VFNAFSGLV;NGFIDVFNAFSGLVA-VFNAFSGLV;AIVLRGVAKDGKFAV-
LRGVAKDGK;AAIVLRGVAKDGKFA-LRGVAKDGK;IDVFNAFSGLVADTF-
VFNAFSGLV;FIDVFNAFSGLVADT-VFNAFSGLV

DRB3_0101
DRB4_0101
DRB5_0101

**<ABX71745 CRASP-2;Protein;Borrelia burgdorferi>**

**Allele 8-mer**
A0101 YSEYTGAY
A0201 YMLISISL;KLIDDFAI;FLSIYMLI;MLISISLL;FLGAAVEL;SIYMLISI
A0202
FLSIYMLI;MLISISLL;FLGAAVEL;KLIDDFAI;YMLISISL;KLRKSVAL;FVEENDLI;ALAYIESF
;SIYMLISI;FAIELDQA;KAIKQTLL;LIALKCIV;KVNQAISI
A0203
FLSIYMLI;MLISISLL;FLGAAVEL;YMLISISL;KLIDDFAI;SIYMLISI;KLRKSVAL;LIALKCIV
;KVNQAISI;ALKCIVKT;MKKSFLSI
A0204 FLGAAVEL;KLIDDFAI;YMLISISL;FLSIYMLI;MLISISLL
A0206
KLIDDFAI;YMLISISL;FLGAAVEL;MLISISLL;FAIELDQA;FLSIYMLI;KVNQAISI;FVEENDLI
;AAVELEGA;KAIKQTLL;KSVALAYI;SIYMLISI;KEADFLGA;YSIKLDAI;YIESFDVI
A0211
KLIDDFAI;YMLISISL;FLGAAVEL;MLISISLL;FLSIYMLI;SIYMLISI;KLRKSVAL;ELEGAYKA
;LIALKCIV;KLDAIYSE;AIELDQAV;KFVDSKFV;PMFLSKLI;KVNQAISI;SFLSIYML;DLIALKC
I;YIESFDVI;DVISSKFV
A0212
YMLISISL;FLGAAVEL;KLIDDFAI;FLSIYMLI;MLISISLL;SIYMLISI;KLRKSVAL;AIELDQAV
;LIALKCIV
A0216
FLGAAVEL;MLISISLL;YMLISISL;FLSIYMLI;KLIDDFAI;LIALKCIV;KLRKSVAL;SIYMLISI
;ELEGAYKA;AIELDQAV;DVISSKFV;KFVDSKFV;DVSNARHV
A0219 FLGAAVEL;YMLISISL;MLISISLL;FLSIYMLI;KLIDDFAI
A0301 KIFVEKAK;IMTYIMTY;KAKYYSIK;HVADSYEK;IALKCIVK;RSRYNNFY;ASKKFVNK;
A1101
HVADSYEK;ASKKFVNK;SSKFVDSK;SILKKDNK;QAISILKK;GTSSDKSK;KIFVEKAK;NQAISILK
;IALKCIVK;SRYNNFYK;KAKYYSIK;RYNNFYKK;DSYEKLRK
A2301 FYKKEADF;RYNNFYKK;SFLSIYML;KFVEASKK
A2402 SFLSIYML;FYKKEADF;AYIESFDV
A2403 FYKKEADF;SFLSIYML;AYIESFDV;FVNKAKEF
A2601 EINSRSRY;
A2602 EINSRSRY;SIKLDAIY;FVEKAKYY;AVELEGAY;DVISSKFV;YSEYTGAY;NIDELKIF
A2902 IMTYIMTY;AYNDIMTY;IFVEKAKY;YSEYTGAY;FVEKAKYY
A3001
KAKYYSIK;RYNNFYKK;RSRYNNFY;ASKKFVNK;KIFVEKAK;SSKFVDSK;KFVEASKK;KKFVNKAK
;KLRKSVAL;KSKVNQAI
A3002 AYNDIMTY;IMTYIMTY;RSRYNNFY
A3101 RYNNFYKK;KAKYYSIK;ASKKFVNK;MTYIMTYS;REINSRSR
A3301 DVSRLNQR
A6801
HVADSYEK;DVSRLNQR;QAISILKK;ELKIFVEK;SSKFVDSK;DSYEKLRK;MTYIMTYS;LLSCDVSR
;EGAYKAIK;NQAISILK;EINSRSRY;SRYNNFYK
A6802
DVISSKFV;MLISISLL;EKAKYYSI;MTYIMTYS;ESFDVISS;TSSDKSKV;FLSIYMLI;YSIKLDAI
A6901 DVISSKFV;YMLISISL;EADFLGAA
B0702 KPMFLSKL;KLRKSVAL
B0801 KLRKSVAL
B0802
B1501 YSEYTGAY;IMTYIMTY;RSRYNNFY;YMLISISL;ALAYIESF

B1801 EEYKPMFL;EENDLIAL
B2705 SRYNNFYK
B3501 YSEYTGAY;FVNKAKEF;AVELEGAY;FVEKAKYY;
B3901 YKAIKQTL;YMLISISL
B4001 EEYKPMFL;EENDLIAL
B4002 EEYKPMFL;EENDLIAL
B4402
B4403 EENDLIAL
B4501 EENDLIAL
B5101
B5301
B5401 FAIELDQA
B5701
B5801 KSFLSIYM;KSVALAYI;KAIKQTLL

**Allele 9-mer**
A0101 KLDAIYSEY;YSIKLDAIY
A0201
YMLISISLL;LIDDFAIEL;YIMTYSEGT;LLSCDVSRL;FVDSKFVEA;NIDELKIFV;FVNKAKEFV;F
LSIYMLIS;FAIELDQAV;MLISISLLS;AIYSEYTGA
A0202
LLSCDVSRL;YMLISISLL;FVNKAKEFV;HVADSYEKL;FAIELDQAV;MLISISLLS;AIYSEYTGA;F
LSIYMLIS;FLSKLIDDF;FVDSKFVEA;IMTYIMTYS;LIDDFAIEL;YIMTYSEGT;KVNQAISIL;AY
NDIMTYI;FLGAAVELE;KLRKSVALA;KSFLSIYML;YKAIKQTLL
A0203
LLSCDVSRL;KLRKSVALA;ALKCIVKTI;AIYSEYTGA;YMLISISLL;FVNKAKEFV;KVNQAISIL;S
ISLLSCDV;FLSIYMLIS;YIMTYSEGT;AYNDIMTYI;FAIELDQAV;IMTYIMTYS;MLISISLLS;FV
DSKFVEA;IVKTIGDMV;ILKKDNKIV
A0204 FAIELDQAV;LIDDFAIEL;YMLISISLL
A0206
FAIELDQAV;FVDSKFVEA;FVNKAKEFV;YIMTYSEGT;LIDDFAIEL;YMLISISLL;AIYSEYTGA;N
IDELKIFV;KLIDDFAIE;KEADFLGAA;LSIYMLISI;KIVNKFKEL
A0211
LIDDFAIEL;NIDELKIFV;YMLISISLL;DLIALKCIV;FVNKAKEFV;FAIELDQAV;LLSCDVSRL;K
LDAIYSEY;SISLLSCDV;FVDSKFVEA;ILKKDNKIV;HVADSYEKL;FVEASKKFV;MLISISLLS;KL
RKSVALA;ELEGAYKAI;FLSIYMLIS;ALKCIVKTI;EADFLGAAV;SFLSIYMLI
A0212
LIDDFAIEL;NIDELKIFV;FAIELDQAV;YMLISISLL;FVNKAKEFV;DLIALKCIV;SISLLSCDV;Y
IMTYSEGT;AIYSEYTGA;FVDSKFVEA;ILKKDNKIV;FLSIYMLIS;LLSCDVSRL;LAYIESFDV;
A0216
NIDELKIFV;LIDDFAIEL;FVNKAKEFV;DLIALKCIV;YMLISISLL;LLSCDVSRL;FVEASKKFV;I
LKKDNKIV;FAIELDQAV;SISLLSCDV;HVADSYEKL;KLRKSVALA;FVDSKFVEA
A0219 LIDDFAIEL;YMLISISLL;NIDELKIFV;FVNKAKEFV;LLSCDVSRL;HVADSYEKL
A0301 RSRYNNFYK;ESFDVISSK;LIALKCIVK;NQAISILKK;KLDAIYSEY
A1101
ESFDVISSK;RSRYNNFYK;ISSKFVDSK;AVELEGAYK;ISILKKDNK;VNQAISILK;NQAISILKK;L
IALKCIVK;GAYNDIMTY;SRYNNFYKK;EASKKFVNK;SLLSCDVSR
A2301
IYMLISISL;KFVEASKKF;FYKKEADFL;EYKPMFLSK;NFYKKEADF;SFDVISSKF;AYIESFDVI;E
YTGAYNDI;KFVNKAKEF;AYNDIMTYI;AYKAIKQTL;SFLSIYMLI;DFLGAAVEL;KSFLSIYML;FL
SKLIDDF
A2402
IYMLISISL;SFLSIYMLI;AYIESFDVI;YYSIKLDAI;EYTGAYNDI;AYNDIMTYI;SFDVISSKF;F
YKKEADFL;AYKAIKQTL;

A2403
FYKKEADFL;YYSIKLDAI;KFVNKAKEF;AYIESFDVI;AYKAIKQTL;IYMLISISL;KFVEASKKF;A
YNDIMTYI;SFLSIYMLI;NFYKKEADF;SFDVISSKF;EYTGAYNDI
A2601 YSIKLDAIY;DIMTYIMTY;ELEKIIEEY
A2602
DIMTYIMTY;KIIEEYKPM;YSIKLDAIY;YTGAYNDIM;ELEKIIEEY;KIFVEKAKY;HVADSYEKL;K
VNQAISIL;KIVNKFKEL
A2902 YSIKLDAIY;IYSEYTGAY;KIFVEKAKY
A3001 RSRYNNFYK;KLRKSVALA;EYKPMFLSK
A3002 KIFVEKAKY;AYNDIMTYI
A3101 RSRYNNFYK;SLLSCDVSR;MTYIMTYSE;KSFLSIYML
A3301 DSKFVEASK;ESFDVISSK;EYKPMFLSK
A6801
ESFDVISSK;DSKFVEASK;MTYIMTYSE;EASKKFVNK;ISSKFVDSK;TIGDMVNDR;DIMTYIMTY;R
SRYNNFYK;EENDLIALK;YSEGTSSDK;SLLSCDVSR;ISILKKDNK;EKAKYYSIK;EYKPMFLSK
A6802
HVADSYEKL;FAIELDQAV;FVNKAKEFV;EADFLGAAV;LSIYMLISI;DVSNARHVA;YIMTYSEGT
A6901
EADFLGAAV;NIDELKIFV;FAIELDQAV;FVNKAKEFV;HVADSYEKL;LIDDFAIEL;FVDSKFVEA
B0702 KPMFLSKLI;KVNQAISIL
B0801 YEKLRKSVA
B0802
B1501 YSIKLDAIY;AAVELEGAY;YMLISISLL;GAYNDIMTY
B1801 VEENDLIAL;REINSRSRY;VELEGAYKA
B2705 SRYNNFYKK
B3501
AAVELEGAY;FAIELDQAV;YSIKLDAIY;DIMTYIMTY;IYSEYTGAY;NARHVADSY;GAYNDIMTY;Y
TGAYNDIM;VALAYIESF
B3901 YNDIMTYIM
B4001 KEFVEENDL;VEENDLIAL;IEEYKPMFL
B4002 KEFVEENDL
B4402
B4403
B4501 REINSRSRY
B5101 KPMFLSKLI
B5301
B5401 FAIELDQAV
B5701
B5801 VALAYIESF;YSIKLDAIY

**Allele 10-mer**
A0101
A0201
FLSIYMLISI;KLIDDFAIEL;FLSKLIDDFA;ALAYIESFDV;SLLSCDVSRL;KLDAIYSEYT;SIYML
ISISL;YMLISISLLS;RLNQRNIDEL;MLISISLLSC;GAYNDIMTYI
A0202
FLSIYMLISI;SLLSCDVSRL;FLSKLIDDFA;KLIDDFAIEL;KLDAIYSEYT;GAYNDIMTYI;SIYML
ISISL;ALAYIESFDV;MLISISLLSC;RLNQRNIDEL;KSFLSIYMLI;FVEENDLIAL;IYMLISISLL
;FLGAAVELEG;YMLISISLLS;KYYSIKLDAI
A0203
FLSIYMLISI;KLIDDFAIEL;FLSKLIDDFA;ALAYIESFDV;RLNQRNIDEL;SIYMLISISL;GAYND
IMTYI;MLISISLLSC;SLLSCDVSRL;CIVKTIGDMV;
A0204 KLIDDFAIEL;SLLSCDVSRL;ALAYIESFDV;RLNQRNIDEL;FLSIYMLISI
A0206
KLIDDFAIEL;FLSIYMLISI;FVEENDLIAL;ALAYIESFDV;MLISISLLSC;FLSKLIDDFA;GAYND
IMTYI;FVEKAKYYSI;KEADFLGAAV;SIYMLISISL;KLDAIYSEYT;CIVKTIGDMV;RLNQRNIDEL

A0211
KLIDDFAIEL;FLSIYMLISI;SLLSCDVSRL;SIYMLISISL;ALAYIESFDV;ELDQAVDNDV;RLNQR
NIDEL;KLDAIYSEYT;FLSKLIDDFA;MLISISLLSC;FVEENDLIAL;SILKKDNKIV;IIEEYKPMFL
;YMLISISLLS;SFDVISSKFV;KEADFLGAAV;GAYNDIMTYI
A0212
KLIDDFAIEL;FLSIYMLISI;ALAYIESFDV;ELDQAVDNDV;SIYMLISISL;FLSKLIDDFA;RLNQR
NIDEL;FVEENDLIAL;SLLSCDVSRL;MLISISLLSC;SILKKDNKIV;KLDAIYSEYT;IIEEYKPMFL
A0216
KLIDDFAIEL;SLLSCDVSRL;FLSIYMLISI;ALAYIESFDV;RLNQRNIDEL;SIYMLISISL;ELDQA
VDNDV;FLSKLIDDFA;IIEEYKPMFL;KLDAIYSEYT;SILKKDNKIV;CIVKTIGDMV;FVEKAKYYSI
;GAYKAIKQTL;MLISISLLSC;FVEENDLIAL;
A0219
ELDQAVDNDV;KLIDDFAIEL;SLLSCDVSRL;FLSIYMLISI;RLNQRNIDEL;FLSKLIDDFA;ALAYI
ESFDV
A0301
KVNQAISILK;RSRYNNFYKK;IVNKFKELEK;KLRKSVALAY;DLIALKCIVK;AIYSEYTGAY;AAVEL
EGAYK;VISSKFVDSK;AISILKKDNK
A1101
KVNQAISILK;IVNKFKELEK;VISSKFVDSK;RSRYNNFYKK;AAVELEGAYK;MVNDREINSR;AISIL
KKDNK;AIYSEYTGAY;ASKKFVNKAK;KTIGDMVNDR;VADSYEKLRK;VNQAISILKK;AVDNDVSNAR
A2301
IYMLISISLL;MFLSKLIDDF;TYSEGTSSDK;EYKPMFLSKL;KYYSIKLDAI;AYKAIKQTLL;ESFDV
ISSKF;ISSKFVDSKF;AYNDIMTYIM;SVALAYIESF
A2402 IYMLISISLL;KYYSIKLDAI;EYKPMFLSKL;AYKAIKQTLL
A2403
MFLSKLIDDF;KYYSIKLDAI;IYMLISISLL;AYKAIKQTLL;EYKPMFLSKL;AYNDIMTYIM;YYSIK
LDAIY
A2601 ESFDVISSKF;
A2602 KIIEEYKPMF;AIYSEYTGAY;KIFVEKAKYY;SVALAYIESF;FVEENDLIAL
A2902 YYSIKLDAIY;AIYSEYTGAY;KIFVEKAKYY
A3001
RSRYNNFYKK;KVNQAISILK;ASKKFVNKAK;KLRKSVALAY;IVNKFKELEK;ARHVADSYEK;KDNKI
VNKFK;KSFLSIYMLI
A3002 KIFVEKAKYY;NSRSRYNNFY
A3101
KTIGDMVNDR;RSRYNNFYKK;ISLLSCDVSR;MVNDREINSR;KVNQAISILK;ASKKFVNKAK;AVDND
VSNAR
A3301
A6801
HVADSYEKLR;MVNDREINSR;DSKFVEASKK;ELEKIIEEYK;KTIGDMVNDR;IVNKFKELEK;KVNQA
ISILK;ISLLSCDVSR;DLIALKCIVK;ELKIFVEKAK;EEYKPMFLSK;TYSEGTSSDK;ESFDVISSKF
;ELEGAYKAIK
A6802 FVEKAKYYSI;SIYMLISISL;EASKKFVNKA;DIMTYIMTYS
A6901
B0702
B0801 YEKLRKSVAL
B0802
B1501 KLRKSVALAY;SVALAYIESF;AIYSEYTGAY;KIIEEYKPMF;KIFVEKAKYY
B1801 VELEGAYKAI;KELEKIIEEY
B2705
B3501 FVEENDLIAL;TGAYNDIMTY;GAAVELEGAY;YYSIKLDAIY;SVALAYIESF
B3901 FVEENDLIAL
B4001 SEYTGAYNDI;KEFVEENDLI;VELEGAYKAI;YEKLRKSVAL
B4002 KEFVEENDLI
B4402 KELEKIIEEY
B4403

B4501 EENDLIALKC
B5101 LAYIESFDVI
B5301
B5401
B5701
B5801 ISSKFVDSKF;KIIEEYKPMF;

**Allele 11-mer**
A0101 YTGAYNDIMTY;YNDIMTYIMTY;VSNARHVADSY
A0201
FLSKLIDDFAI;FLGAAVELEGA;KLRKSVALAYI;ALAYIESFDVI;KIIEEYKPMFL;VALAYIESFDV
;SIYMLISISLL;YMLISISLLSC;FLSIYMLISIS
A0202
FLSKLIDDFAI;FLGAAVELEGA;ALAYIESFDVI;FLSIYMLISIS;SIYMLISISLL;KLRKSVALAYI
;KIIEEYKPMFL;GAYKAIKQTLL
A0203
FLGAAVELEGA;KLRKSVALAYI;FLSKLIDDFAI;SIYMLISISLL;ALAYIESFDVI;KIIEEYKPMFL
;LISISLLSCDV;FLSIYMLISIS;LIALKCIVKTI;MTYIMTYSEGT;YMLISISLLSC
A0204 FLGAAVELEGA;KIIEEYKPMFL;VALAYIESFDV
A0206
FLSKLIDDFAI;FLGAAVELEGA;KIIEEYKPMFL;ALAYIESFDVI;VALAYIESFDV;YIMTYSEGTSS
;LSIYMLISISL;YMLISISLLSC;LISISLLSCDV
A0211
FLSKLIDDFAI;SIYMLISISLL;FLGAAVELEGA;YMLISISLLSC;KLRKSVALAYI;KIIEEYKPMFL
;ALAYIESFDVI;ESFDVISSKFV;LIDDFAIELDQ;LISISLLSCDV;VALAYIESFDV;KLDAIYSEYT
G;TIGDMVNDREI
A0212
FLSKLIDDFAI;FLGAAVELEGA;VALAYIESFDV;YMLISISLLSC;SIYMLISISLL;LISISLLSCDV
;LIDDFAIELDQ;KIIEEYKPMFL
A0216
FLSKLIDDFAI;SIYMLISISLL;KLRKSVALAYI;FLGAAVELEGA;ALAYIESFDVI;KIIEEYKPMFL
;ESFDVISSKFV;LISISLLSCDV
A0219 FLGAAVELEGA;FLSKLIDDFAI;VALAYIESFDV;YMLISISLLSC;
A0301
MTYSEGTSSDK;KVNQAISILKK;RLNQRNIDELK;ILKKDNKIVNK;HVADSYEKLRK;KIVNKFKELEK
;NSRSRYNNFYK;FVDSKFVEASK
A1101
MTYSEGTSSDK;KVNQAISILKK;HVADSYEKLRK;KIVNKFKELEK;RLNQRNIDELK;FVEASKKFVNK
;GAAVELEGAYK;SISLLSCDVSR;DVISSKFVDSK;NSRSRYNNFYK;FVDSKFVEASK;YIESFDVISS
K;QAISILKKDNK;NIDELKIFVEK;FVEENDLIALK;VSNARHVADSY
A2301 EYKPMFLSKLI;SYEKLRKSVAL;PMFLSKLIDDF
A2402 EYKPMFLSKLI;SFLSIYMLISI;IYMLISISLLS
A2403 SYEKLRKSVAL;SFLSIYMLISI
A2601 ELKIFVEKAKY;YTGAYNDIMTY;DAIYSEYTGAY;ESFDVISSKFV;
A2602
EINSRSRYNNF;YTGAYNDIMTY;KIIEEYKPMFL;SIKLDAIYSEY;DAIYSEYTGAY;ELKIFVEKAKY
;EFVEENDLIAL;SIYMLISISLL
A2902 KYYSIKLDAIY;YTGAYNDIMTY
A3001
KVNQAISILKK;NSRSRYNNFYK;KLRKSVALAYI;RLNQRNIDELK;NARHVADSYEK;MTYSEGTSSDK
;KIVNKFKELEK
A3002 KYYSIKLDAIY
A3101
NSRSRYNNFYK;LSCDVSRLNQR;KVNQAISILKK;RLNQRNIDELK;SISLLSCDVSR;KYYSIKLDAIY
A3301 DMVNDREINSR;NSRSRYNNFYK;LSCDVSRLNQR

A6801
MTYSEGTSSDK;DVISSKFVDSK;HVADSYEKLRK;LSCDVSRLNQR;SISLLSCDVSR;QAVDNDVSNAR
;YIESFDVISSK;NSRSRYNNFYK;FVEENDLIALK;DMVNDREINSR;NARHVADSYEK;EASKKFVNKA
K;QAISILKKDNK;FVEKAKYYSIK;FVEASKKFVNK;DAIYSEYTGAY;FVDSKFVEASK;MTYIMTYSE
GT;NIDELKIFVEK;YTGAYNDIMTY
A6802
ESFDVISSKFV;TGAYNDIMTYI;MTYIMTYSEGT;LSIYMLISISL;EGAYKAIKQTL;FLSKLIDDFAI
A6901 ESFDVISSKFV;EADFLGAAVEL
B0702
B0801
B0802
B1501
VSNARHVADSY;KSVALAYIESF;YTGAYNDIMTY;NQRNIDELKIF;SIKLDAIYSEY;LGAAVELEGAY
B1801 EEYKPMFLSKL;IESFDVISSKF;SEYTGAYNDIM
B2705
B3501
DAIYSEYTGAY;YTGAYNDIMTY;EADFLGAAVEL;EFVEENDLIAL;YNDIMTYIMTY;GAYNDIMTYIM
B3901
B4001 SEYTGAYNDIM;EEYKPMFLSKL;VEKAKYYSIKL
B4002 KEFVEENDLIA;EENDLIALKCI
B4402
B4403 EENDLIALKCI;SEYTGAYNDIM;KEFVEENDLIA
B4501 EENDLIALKCI
B5101
B5301
B5401
B5701
B5801 KSVALAYIESF;VSNARHVADSY

**Allele 15-mer plus 9-mer core**
DRB1_0101
SFLSIYMLISISLLS-IYMLISISL;FLSIYMLISISLLSC-IYMLISISL;KSFLSIYMLISISLL-
IYMLISISL;LSIYMLISISLLSCD-IYMLISISL;KKSFLSIYMLISISL-
FLSIYMLIS;EEYKPMFLSKLIDDF-YKPMFLSKL;EKIIEEYKPMFLSKL-
IEEYKPMFL;IEEYKPMFLSKLIDD-YKPMFLSKL;IIEEYKPMFLSKLID-
YKPMFLSKL;KIIEEYKPMFLSKLI-YKPMFLSKL;YNDIMTYIMTYSEGT-
IMTYIMTYS;NDIMTYIMTYSEGTS-YIMTYSEGT;IMTYIMTYSEGTSSD-
YIMTYSEGT;SIYMLISISLLSCDV-IYMLISISL;DIMTYIMTYSEGTSS-
YIMTYSEGT;MTYIMTYSEGTSSDK-YIMTYSEGT;IYMLISISLLSCDVS-
IYMLISISL;EYKPMFLSKLIDDFA-YKPMFLSKL;YKPMFLSKLIDDFAI-
YKPMFLSKL;GAYNDIMTYIMTYSE-YNDIMTYIM;TGAYNDIMTYIMTYS-
YNDIMTYIM;TYIMTYSEGTSSDKS-YIMTYSEGT;YIMTYSEGTSSDKSK-
YIMTYSEGT;SEYTGAYNDIMTYIM-YTGAYNDIM;EYTGAYNDIMTYIMT-
YNDIMTYIM;YTGAYNDIMTYIMTY-YNDIMTYIM;KYYSIKLDAIYSEYT-
YSIKLDAIY;IESFDVISSKFVDSK-FDVISSKFV;ESFDVISSKFVDSKF-
FDVISSKFV;YIESFDVISSKFVDS-FDVISSKFV;YYSIKLDAIYSEYTG-
LDAIYSEYT;IKLDAIYSEYTGAYN-LDAIYSEYT;SIKLDAIYSEYTGAY-
LDAIYSEYT;VELEGAYKAIKQTLL-VELEGAYKA;AYNDIMTYIMTYSEG-
YNDIMTYIM;YSIKLDAIYSEYTGA-LDAIYSEYT;LAYIESFDVISSKFV-
YIESFDVIS;AYIESFDVISSKFVD-FDVISSKFV;DSYEKLRKSVALAYI-
LRKSVALAY;YEKLRKSVALAYIES-LRKSVALAY;SYEKLRKSVALAYIE-
LRKSVALAY;EKLRKSVALAYIESF-LRKSVALAY;DKSKVNQAISILKKD-
VNQAISILK;SDKSKVNQAISILKK-VNQAISILK;SSDKSKVNQAISILK-
KVNQAISIL;KSKVNQAISILKKDN-VNQAISILK;ADSYEKLRKSVALAY-
EKLRKSVAL;YMLISISLLSCDVSR-MLISISLLS;KEADFLGAAVELEGA-
FLGAAVELE;EADFLGAAVELEGAY-FLGAAVELE;SKVNQAISILKKDNK-

VNQAISILK;ADFLGAAVELEGAYK-FLGAAVELE;KKEADFLGAAVELEG-
FLGAAVELE;DFLGAAVELEGAYKA-FLGAAVELE;YKKEADFLGAAVELE-
YKKEADFLG;FLGAAVELEGAYKAI-VELEGAYKA;MLISISLLSCDVSRL-
MLISISLLS;VEENDLIALKCIVKT-ENDLIALKC;NDLIALKCIVKTIGD-
IALKCIVKT;ENDLIALKCIVKTIG-IALKCIVKT;LIDDFAIELDQAVDN-
FAIELDQAV;SKLIDDFAIELDQAV-LIDDFAIEL;DLIALKCIVKTIGDM-
IALKCIVKT;IDDFAIELDQAVDND-FAIELDQAV;SFDVISSKFVDSKFV-
FDVISSKFV;FDVISSKFVDSKFVE-FDVISSKFV;EENDLIALKCIVKTI-
IALKCIVKT;DDFAIELDQAVDNDV-FAIELDQAV;IALKCIVKTIGDMVN-
IALKCIVKT;KLIDDFAIELDQAVD-FAIELDQAV;MKKSFLSIYMLISIS-
FLSIYMLIS;LRKSVALAYIESFDV-LRKSVALAY;KLDAIYSEYTGAYND-
LDAIYSEYT;LDAIYSEYTGAYNDI-LDAIYSEYT;KLRKSVALAYIESFD-LRKSVALAY
DRB1_0301
DRB1_0401 SFLSIYMLISISLLS-IYMLISISL;FLSIYMLISISLLSC-
MLISISLLS;LSIYMLISISLLSCD-MLISISLLS;IYMLISISLLSCDVS-
MLISISLLS;SIYMLISISLLSCDV-MLISISLLS;YMLISISLLSCDVSR-
MLISISLLS;MLISISLLSCDVSRL-MLISISLLS;KKSFLSIYMLISISL-
FLSIYMLIS;KSFLSIYMLISISLL-FLSIYMLIS;IELDQAVDNDVSNAR-
IELDQAVDN;ELDQAVDNDVSNARH-VDNDVSNAR;QAVDNDVSNARHVAD-
VDNDVSNAR;LDQAVDNDVSNARHV-VDNDVSNAR;MKKSFLSIYMLISIS-
FLSIYMLIS;DQAVDNDVSNARHVA-VDNDVSNAR;DIMTYIMTYSEGTSS-
YIMTYSEGT;YNDIMTYIMTYSEGT-DIMTYIMTY;DAIYSEYTGAYNDIM-
IYSEYTGAY;IKLDAIYSEYTGAYN-IYSEYTGAY;MTYIMTYSEGTSSDK-
YIMTYSEGT;IMTYIMTYSEGTSSD-YIMTYSEGT;SIKLDAIYSEYTGAY-
LDAIYSEYT;KLDAIYSEYTGAYND-IYSEYTGAY;NDIMTYIMTYSEGTS-
YIMTYSEGT;LDAIYSEYTGAYNDI-IYSEYTGAY;ADSYEKLRKSVALAY-
YEKLRKSVA;DSYEKLRKSVALAYI-LRKSVALAY
DRB1_0404 FLSIYMLISISLLSC-MLISISLLS;LSIYMLISISLLSCD-
MLISISLLS;SIYMLISISLLSCDV-MLISISLLS;SFLSIYMLISISLLS-
YMLISISLL;NDIMTYIMTYSEGTS-IMTYIMTYS;IYMLISISLLSCDVS-
MLISISLLS;DIMTYIMTYSEGTSS-IMTYSEGTS;YNDIMTYIMTYSEGT-
IMTYIMTYS;IMTYIMTYSEGTSSD-IMTYSEGTS;
DRB1_0405 FLSIYMLISISLLSC-FLSIYMLIS
DRB1_0701 DSYEKLRKSVALAYI-LRKSVALAY;ADSYEKLRKSVALAY-
YEKLRKSVA;SYEKLRKSVALAYIE-LRKSVALAY;YEKLRKSVALAYIES-
LRKSVALAY;EKLRKSVALAYIESF-LRKSVALAY;LAYIESFDVISSKFV-
SFDVISSKF;AYIESFDVISSKFVD-FDVISSKFV;YIESFDVISSKFVDS-
FDVISSKFV;IESFDVISSKFVDSK-FDVISSKFV;ESFDVISSKFVDSKF-FDVISSKFV
DRB1_0802
DRB1_0901 SFLSIYMLISISLLS-YMLISISLL;FLSIYMLISISLLSC-
MLISISLLS;LSIYMLISISLLSCD-MLISISLLS;SIYMLISISLLSCDV-
YMLISISLL;FVDSKFVEASKKFVN-FVEASKKFV;VDSKFVEASKKFVNK-
FVEASKKFV;SKFVEASKKFVNKAK-FVEASKKFV;DSKFVEASKKFVNKA-
FVEASKKFV;KFVDSKFVEASKKFV-FVDSKFVEA;
DRB1_1101 DSYEKLRKSVALAYI-YEKLRKSVA;ADSYEKLRKSVALAY-YEKLRKSVA
DRB1_1302 LSIYMLISISLLSCD-IYMLISISL;FLSIYMLISISLLSC-
IYMLISISL;SFLSIYMLISISLLS-IYMLISISL;TGAYNDIMTYIMTYS-
YNDIMTYIM;GAYNDIMTYIMTYSE-YNDIMTYIM;SIYMLISISLLSCDV-
MLISISLLS;IYMLISISLLSCDVS-MLISISLLS;KSFLSIYMLISISLL-
IYMLISISL;AYNDIMTYIMTYSEG-YNDIMTYIM;EYTGAYNDIMTYIMT-
YNDIMTYIM;YTGAYNDIMTYIMTY-YNDIMTYIM;YNDIMTYIMTYSEGT-
YNDIMTYIM;SEYTGAYNDIMTYIM-AYNDIMTYI;SKVNQAISILKKDNK-
VNQAISILK;SDKSKVNQAISILKK-VNQAISILK;SSDKSKVNQAISILK-
KVNQAISIL;DKSKVNQAISILKKD-VNQAISILK;KSKVNQAISILKKDN-
VNQAISILK;YMLISISLLSCDVSR-MLISISLLS;KKSFLSIYMLISISL-
LSIYMLISI;NDLIALKCIVKTIGD-IALKCIVKT;KVNQAISILKKDNKI-

VNQAISILK;QAISILKKDNKIVNK-ISILKKDNK;IALKCIVKTIGDMVN-
LKCIVKTIG;NQAISILKKDNKIVN-ISILKKDNK;ENDLIALKCIVKTIG-
IALKCIVKT;DLIALKCIVKTIGDM-IALKCIVKT;LIALKCIVKTIGDMV-
LKCIVKTIG;AISILKKDNKIVNKF-LKKDNKIVN;ISILKKDNKIVNKFK-
LKKDNKIVN;VNQAISILKKDNKIV-ISILKKDNK;MVNDREINSRSRYNN-
REINSRSRY;MLISISLLSCDVSRL-MLISISLLS;VNDREINSRSRYNNF-
REINSRSRY;NDREINSRSRYNNFY-REINSRSRY
DRB1_1501 FLSIYMLISISLLSC-MLISISLLS;LSIYMLISISLLSCD-
MLISISLLS;SFLSIYMLISISLLS-IYMLISISL;SIYMLISISLLSCDV-
MLISISLLS;IYMLISISLLSCDVS-MLISISLLS
DRB3_0101
DRB4_0101 EKIIEEYKPMFLSKL-IEEYKPMFL;KELEKIIEEYKPMFL-
IIEEYKPMF;LEKIIEEYKPMFLSK-IEEYKPMFL;ELEKIIEEYKPMFLS-
IEEYKPMFL;IYMLISISLLSCDVS-LISISLLSC;KIIEEYKPMFLSKLI-
IEEYKPMFL;YMLISISLLSCDVSR-ISLLSCDVS;MLISISLLSCDVSRL-ISLLSCDVS
DRB5_0101

**<NP_045689 outer surface protein B;Protein;Borrelia burgdorferi B31>**

**Allele 8-mer**
A0101
A0201 FLTDGTIT;LLIGFALA;RLLIGFAL;FALALALI;LTDGTITV;SLFNGNKI;FLNDTAGS
A0202
FLNDTAGS;FLTDGTIT;ALALIGCA;LLIGFALA;SLFNGNKI;KTKDLVFL;RLLIGFAL;ATIDQVEL
;LIGFALAL;KLTVSADL;VSADLNTV;NLSELKNA;FALALALI;LVGGKTTV;
A0203
LLIGFALA;ALALIGCA;LVGGKTTV;FLTDGTIT;SLFNGNKI;KTKDLVFL;FLNDTAGS;RLLIGFAL
;NLSELKNA;TLKNSIKL;KVKLTVSA;LIGFALAL;FALALALI;SIKLEGSL;KLTVSADL;LTDGTIT
V;NQKISSKV;ATKAVETL;VSADLNTV;AQKGAESI
A0204 SLFNGNKI;ATIDQVEL;LVGGKTTV;RLLIGFAL;LTDGTITV
A0206
RLLIGFAL;FALALALI;LTDGTITV;LLIGFALA;FLTDGTIT;ATIDQVEL;LVGGKTTV;QQYNTAGT
;FLNDTAGS;LIGFALAL;AQKGAESI;KTKDLVFL
A0211
RLLIGFAL;LTDGTITV;LLIGFALA;SLFNGNKI;LVGGKTTV;FLTDGTIT;SLVGGKTT;TLKNSIKL
;NLSELKNA;KLDSKKLT;FLNDTAGS;KLTVSADL;VSADLNTV;ALALIGCA;KTKDLVFL;ATIDQVE
L;IKLEGSLV;TTLEYSQI;LIGFALAL;ALIGCAQK
A0212
RLLIGFAL;LTDGTITV;SLFNGNKI;LLIGFALA;LVGGKTTV;FLTDGTIT;FLNDTAGS;SLVGGKTT
;VSADLNTV;LIGFALAL;NLSELKNA
A0216
LVGGKTTV;RLLIGFAL;LTDGTITV;SLFNGNKI;LLIGFALA;TLKNSIKL;FLTDGTIT;SLVGGKTT
;LIGFALAL;ALALIGCA;ATIDQVEL
A0219 LTDGTITV;LVGGKTTV;FLTDGTIT;RLLIGFAL;LLIGFALA;FLNDTAGS
A0301
KISSKVTK;ISSKVTKK;KIFVSKEK;ALIGCAQK;GSNKKTGK;VSLFNGNK;SITXETLK;TISADSKK
A1101
SITXETLK;VSLFNGNK;TISADSKK;KIFVSKEK;LTISADSK;KISSKVTK;ALIGCAQK;TIDQVELK
;KANKLDSK;ISSKVTKK;GSNKKTGK;TLKREIEK;ETLKNSIK;SADSKKTK
A2301 LFNGNKIF;KYDLRATI
A2402 KYDLRATI;VFLTDGTI
A2403 KYDLRATI
A2601 GTITVQQY;EIKNLSEL
A2602 NTVTLEAF;EIKNLSEL;ATIDQVEL;KTKDLVFL;GTITVQQY;ETLKANKL
A2902

A3001
KIFVSKEK;KANKLDSK;KEKNSSGK;TLKREIEK;GSKPDKSK;KISSKVTK;GSNKKTGK;KVKLTVSA
;
A3002
A3101 KISSKVTK;SSGKYDLR;KTKDLVFL;KIFVSKEK;KANKLDSK
A3301
A6801
DTAGSNKK;LTISADSK;ETLKNSIK;TISADSKK;SITXETLK;TIDQVELK;SSGKYDLR;ISSKVTKK
;ELKGTSDK;EGTVTLKR;NTAGTSLE;EAFDASNQ
A6802 TTLEYSQI;ATIDQVEL;VSADLNTV;TSTLTISA
A6901 LTDGTITV;FALALALI;TTLEYSQI;ETLKANKL
B0702
B0801
B0802
B1501
B1801 WEDSTSTL;
B2705
B3501 NTVTLEAF;
B3901 TRSNGTTL;IKEGTVTL
B4001 WEDSTSTL;
B4002
B4402
B4403
B4501
B5101
B5301 FALALALI;
B5401 FALALALI;LPAVTEDS
B5701
B5801

**Allele 9-mer**
A0101 RSNGTTLEY;VTEDSVSLF
A0201 FLTDGTITV;LLIGFALAL;RLLIGFALA;SLVGGKTTV
A0202
FLTDGTITV;NLSELKNAL;LLIGFALAL;TVSADLNTV;LIGFALALA;SLVGGKTTV;AVTEDSVSL;A
LALALIGC;SLEGSASEI
A0203
FLTDGTITV;SLVGGKTTV;TVSADLNTV;LLIGFALAL;LIGFALALA;RLLIGFALA;SIKLEGSLV;S
TSTLTISA;NLSELKNAL;ALALALIGC
A0204 FLTDGTITV;SLVGGKTTV;LLIGFALAL
A0206
FLTDGTITV;TVSADLNTV;SLVGGKTTV;LLIGFALAL;RLLIGFALA;AVTEDSVSL;SQITDADNA;K
QGSITXET;VQQYNTAGT;ALALALIGC;SEIKNLSEL;VTLEAFDAS
A0211
FLTDGTITV;SLVGGKTTV;LLIGFALAL;NLSELKNAL;RLLIGFALA;AVTEDSVSL;SLEGSASEI;E
IKEGTVTL;TVSADLNTV;DLRATIDQV;DLNTVTLEA;LFNGNKIFV;SADLNTVTL;SLFNGNKIF;SI
TXETLKA;ALALALIGC
A0212
FLTDGTITV;SLVGGKTTV;LLIGFALAL;NLSELKNAL;RLLIGFALA;EIKEGTVTL;DLRATIDQV;A
VTEDSVSL;TVSADLNTV;SLEGSASEI;LFNGNKIFV
A0216
FLTDGTITV;SLVGGKTTV;LLIGFALAL;DLRATIDQV;SLEGSASEI;NLSELKNAL;LFNGNKIFV;R
LLIGFALA;EIKEGTVTL;TVSADLNTV;DLNTVTLEA;AVTEDSVSL
A0219 FLTDGTITV;SLVGGKTTV;LLIGFALAL;DLRATIDQV;TVSADLNTV;NLSELKNAL

A0301
KISSKVTKK;ASNQKISSK;RSNGTTLEY;ATKAVETLK;VTLKREIEK;KANKLDSKK;GSITXETLK;S
VSLFNGNK;KLDSKKLTR
A1101
SVSLFNGNK;ATIDQVELK;ATKAVETLK;ASNQKISSK;VTLKREIEK;GSITXETLK;KISSKVTKK;L
TISADSKK;ITDADNATK;KANKLDSKK;TLTISADSK;RSNGTTLEY;GSGTLEGSK;LSELKNALK;GA
ESIGSQK;EAFDASNQK;ISADSKKTK;LALIGCAQK
A2301 QYNTAGTSL
A2402 QYNTAGTSL
A2403 QYNTAGTSL
A2601
A2602 EIKEGTVTL;VTEDSVSLF;ETLKNSIKL
A2902
A3001
ATKAVETLK;RSNGTTLEY;ATIDQVELK;KANKLDSKK;KTKDLVFLT;SKKSHQNAK;SVSLFNGNK
A3002
A3101 KLDSKKLTR;ATKAVETLK;KISSKVTKK;KANKLDSKK;ASNQKISSK
A3301
A6801
EAFDASNQK;LTISADSKK;SVSLFNGNK;EIKNLSELK;NSSGKYDLR;ATIDQVELK;TLTISADSK;A
TKAVETLK;GSITXETLK;ITDADNATK
A6802 TVSADLNTV;STSTLTISA;TTVEIKEGT;FLTDGTITV;ETLKNSIKL
A6901 ETLKNSIKL;FLTDGTITV;TVSADLNTV;DADNATKAV;EIKEGTVTL;TTLEYSQIT
B0702 KPDKSKVKL;LTRSNGTTL;LPAVTEDSV;AVTEDSVSL
B0801
B0802
B1501 RSNGTTLEY;SLFNGNKIF;LLIGFALAL;SQKENDLNL
B1801
B2705 MRLLIGFAL
B3501
B3901 SADLNTVTL
B4001 SEIKNLSEL
B4002 SEIKNLSEL
B4402
B4403
B4501
B5101 LPAVTEDSV
B5301 LPAVTEDSV
B5401 LPAVTEDSV;LALALIGCA
B5701
B5801 RSNGTTLEY;GSNKKTGKW

**Allele 10-mer**
A0101
A0201 SLFNGNKIFV;LLIGFALALA;RLLIGFALAL;FLTDGTITVQ;ALALALIGCA
A0202
LLIGFALALA;SLFNGNKIFV;RLLIGFALAL;KQGSITXETL;ALIGCAQKGA;ALALALIGCA;LTVSA
DLNTV;FLTDGTITVQ;QQYNTAGTSL;FALALALIGC;KLTRSNGTTL;IGFALALALI;DLPAVTEDSV
A0203
LLIGFALALA;ALALALIGCA;SLFNGNKIFV;ALIGCAQKGA;LTVSADLNTV;RLLIGFALAL;KLTRS
NGTTL;FLTDGTITVQ;LIGFALALAL;QQYNTAGTSL;LVGGKTTVEI
A0204 SLFNGNKIFV;LTVSADLNTV;KLTRSNGTTL
A0206
RLLIGFALAL;KQGSITXETL;LTVSADLNTV;QQYNTAGTSL;LLIGFALALA;SLFNGNKIFV;SQITD
ADNAT;FLTDGTITVQ;KAVETLKNSI;LIGFALALAL;TTVEIKEGTV;ALIGCAQKGA;FALALALIGC

A0211

SLFNGNKIFV;RLLIGFALAL;FLTDGTITVQ;LLIGFALALA;DLPAVTEDSV;KLTRSNGTTL;ALIGC
AQKGA;ALALALIGCA;VFLTDGTITV;EIEKDGKVKV;TTVEIKEGTV;LIGFALALAL;SLVGGKTTVE
;KLDSKKLTRS

A0212

SLFNGNKIFV;RLLIGFALAL;FLTDGTITVQ;DLPAVTEDSV;LLIGFALALA;KLTRSNGTTL;VFLTD
GTITV;LIGFALALAL

A0216

SLFNGNKIFV;RLLIGFALAL;DLPAVTEDSV;FLTDGTITVQ;KLTRSNGTTL;LLIGFALALA;EIEKD
GKVKV;ALIGCAQKGA;LTVSADLNTV;LVGGKTTVEI;TTVEIKEGTV;ALALALIGCA;LIGFALALAL
;VFLTDGTITV

A0219 FLTDGTITVQ;SLFNGNKIFV;DLPAVTEDSV;RLLIGFALAL;LLIGFALALA;LTVSADLNTV

A0301

ALALIGCAQK;FLNDTAGSNK;STLTISADSK;NLSELKNALK;TLKANKLDSK;KLEGSLVGGK;TVTLK
REIEK;ITXETLKANK

A1101

STLTISADSK;TVTLKREIEK;ITXETLKANK;TISADSKKTK;SSKKSHQNAK;GTLEGSKPDK;ALALI
GCAQK;AVETLKNSIK;RATIDQVELK;TLKANKLDSK;TLTISADSKK;KLEGSLVGGK;FLNDTAGSNK
;SLEGSASEIK;NLSELKNALK;VSKEKNSSGK;TAGSNKKTGK;QITDADNATK

A2301 VSLFNGNKIF

A2402

A2403

A2601

A2602 AVTEDSVSLF

A2902

A3001

SSKKSHQNAK;GTLEGSKPDK;GSKPDKSKVK;TLKANKLDSK;VSKEKNSSGK;KLEGSLVGGK;RATID
QVELK

A3002

A3101

A3301

A6801

NATKAVETLK;TVTLKREIEK;DSVSLFNGNK;STLTISADSK;EIKEGTVTLK;TLTISADSKK;DASNQ
KISSK;NLSELKNALK;ITXETLKANK;QITDADNATK;TISADSKKTK;FLNDTAGSNK;RATIDQVELK
;DLNLEDSSKK

A6802

EAFDASNQKI;TTVEIKEGTV;LTVSADLNTV;SVSLFNGNKI;NTAGTSLEGS;DLPAVTEDSV;NSIKL
EGSLV

A6901 EAFDASNQKI;TTVEIKEGTV;LTVSADLNTV;SLFNGNKIFV

B0702

B0801

B0802

B1501 QQYNTAGTSL

B1801 VEIKEGTVTL;IEKDGKVKVF;WEDSTSTLTI

B2705

B3501 LPAVTEDSVS;DLNTVTLEAF

B3901

B4001 VEIKEGTVTL;WEDSTSTLTI;VETLKNSIKL

B4002 VEIKEGTVTL

B4402

B4403

B4501

B5101

B5301

B5401 LPAVTEDSVS

B5701

B5801

**Allele 11-mer**
A0101 LTDGTITVQQY;LTRSNGTTLEY
A0201
LLIGFALALAL;FLTDGTITVQQ;KLTVSADLNTV;RLLIGFALALA;SLVGGKTTVEI;LVFLTDGTITV
A0202
FLTDGTITVQQ;LLIGFALALAL;KLTVSADLNTV;SASEIKNLSEL;LIGFALALALI;SLVGGKTTVEI
;TVSADLNTVTL;RLLIGFALALA;FLNDTAGSNKK;HQNAKQDLPAV;LVFLTDGTITV;ALALALIGCA
Q
A0203
LLIGFALALAL;SLVGGKTTVEI;RLLIGFALALA;LIGFALALALI;KLTVSADLNTV;LVFLTDGTITV
;HQNAKQDLPAV;FLTDGTITVQQ;TVSADLNTVTL
A0204 SLVGGKTTVEI;LLIGFALALAL;KLTVSADLNTV;LVFLTDGTITV
A0206
LLIGFALALAL;HQNAKQDLPAV;KLTVSADLNTV;RLLIGFALALA;VQQYNTAGTSL;LVFLTDGTITV
;SLVGGKTTVEI;FLTDGTITVQQ;TVSADLNTVTL;FALALALIGCA;LIGFALALALI
A0211
LLIGFALALAL;SLVGGKTTVEI;FLTDGTITVQQ;KLTVSADLNTV;RLLIGFALALA;LVFLTDGTITV
;SLFNGNKIFVS;ITDADNATKAV;SADSKKTKDLV;TVSADLNTVTL;DLRATIDQVEL;FLNDTAGSNK
K;SASEIKNLSEL;ALALALIGCAQ
A0212
LLIGFALALAL;SLVGGKTTVEI;FLTDGTITVQQ;LVFLTDGTITV;KLTVSADLNTV;RLLIGFALALA
;FLNDTAGSNKK;SLFNGNKIFVS;DLRATIDQVEL;ITDADNATKAV
A0216
SLVGGKTTVEI;LLIGFALALAL;FLTDGTITVQQ;KLTVSADLNTV;LVFLTDGTITV;RLLIGFALALA
;DLRATIDQVEL;SADSKKTKDLV;SLFNGNKIFVS
A0219
FLTDGTITVQQ;LLIGFALALAL;SLVGGKTTVEI;KLTVSADLNTV;LVFLTDGTITV;ITDADNATKAV
;DLRATIDQVEL
A0301
FLNDTAGSNKK;KQGSITXETLK;TLKANKLDSKK;STLTISADSKK;LFNGNKIFVSK;SITXETLKANK
A1101
STLTISADSKK;SITXETLKANK;TSTLTISADSK;TSLEGSASEIK;SQITDADNATK;ASEIKNLSELK
;GTVTLKREIEK;LALALIGCAQK;KQGSITXETLK;TLEAFDASNQK;LTISADSKKTK;KAVETLKNSI
K;TLKANKLDSKK;FVSKEKNSSGK;LVGGKTTVEIK;FLNDTAGSNKK
A2301 VFLNDTAGSNK
A2402 KWEDSTSTLTI
A2403
A2601
A2602 EIEKDGKVKVF;LTDGTITVQQY
A2902
A3001
LTRSNGTTLEY;TLKANKLDSKK;TSLEGSASEIK;LTISADSKKTK;LFNGNKIFVSK;STLTISADSKK
;KQGSITXETLK
A3002
A3101 LFNGNKIFVSK;KQGSITXETLK;STLTISADSKK
A3301 EIKEGTVTLKR
A6801
TSTLTISADSK;EIKEGTVTLKR;DTAGSNKKTGK;STLTISADSKK;LTISADSKKTK;FVSKEKNSSGK
;ETLKANKLDSK;TLEAFDASNQK;SITXETLKANK;GTVTLKREIEK;DSSKKSHQNAK;FLNDTAGSNK
K;TSLEGSASEIK;TLKANKLDSKK;DNATKAVETLK;LALALIGCAQK
A6802
NTAGTSLEGSA;TVSADLNTVTL;LVFLTDGTITV;SASEIKNLSEL;DASNQKISSKV;EGSLVGGKTTV
;DSVSLFNGNKI
A6901 ITDADNATKAV;FALALALIGCA;TTLEYSQITDA;LVFLTDGTITV;NTAGTSLEGSA

```
B0702  LPAVTEDSVSL;KPDKSKVKLTV
B0801
B0802
B1501  LTRSNGTTLEY;LLIGFALALAL;VQQYNTAGTSL;VSKEKNSSGKY;SVSLFNGNKIF
B1801  IEKDGKVKVFL;MRLLIGFALAL
B2705  MRLLIGFALAL
B3501  LPAVTEDSVSL;TVSADLNTVTL
B3901
B4001  LEGSASEIKNL;IEKDGKVKVFL;KEKNSSGKYDL
B4002
B4402
B4403
B4501
B5101  LPAVTEDSVSL
B5301  LPAVTEDSVSL
B5401  FALALALIGCA
B5701
B5801
```

**Allele 15-mer plus 9-mer core**
```
DRB1_0101 MRLLIGFALALALIG-IGFALALAL;RLLIGFALALALIGC-
FALALALIG;LLIGFALALALIGCA-FALALALIG;LIGFALALALIGCAQ-
FALALALIG;IGFALALALIGCAQK-FALALALIG;GFALALALIGCAQKG-
FALALALIG;KNSIKLEGSLVGGKT-IKLEGSLVG;NSIKLEGSLVGGKTT-
IKLEGSLVG;LKNSIKLEGSLVGGK-IKLEGSLVG;ETLKNSIKLEGSLVG-
KNSIKLEGS;TLKNSIKLEGSLVGG-IKLEGSLVG;ITVQQYNTAGTSLEG-
VQQYNTAGT;TITVQQYNTAGTSLE-VQQYNTAGT;FALALALIGCAQKGA-
FALALALIG;KTGKWEDSTSTLTIS-WEDSTSTLT;KKTGKWEDSTSTLTI-
WEDSTSTLT;NKKTGKWEDSTSTLT-GKWEDSTST;KLEGSLVGGKTTVEI-
LVGGKTTVE;LEGSLVGGKTTVEIK-VGGKTTVEI;EGSLVGGKTTVEIKE-
VGGKTTVEI;GSLVGGKTTVEIKEG-VGGKTTVEI;IKLEGSLVGGKTTVE-
IKLEGSLVG;TGKWEDSTSTLTISA-WEDSTSTLT;VQQYNTAGTSLEGSA-
YNTAGTSLE;GKWEDSTSTLTISAD-WEDSTSTLT;TVQQYNTAGTSLEGS-
YNTAGTSLE;QQYNTAGTSLEGSAS-YNTAGTSLE;SLVGGKTTVEIKEGT-
VGGKTTVEI;GTITVQQYNTAGTSL-VQQYNTAGT;DGTITVQQYNTAGTS-
VQQYNTAGT;KDLVFLTDGTITVQQ-FLTDGTITV;DLVFLTDGTITVQQY-
FLTDGTITV;TDGTITVQQYNTAGT-ITVQQYNTA;TKDLVFLTDGTITVQ-
FLTDGTITV;LVFLTDGTITVQQYN-FLTDGTITV;SIKLEGSLVGGKTTV-
IKLEGSLVG;SKVKLTVSADLNTVT-VKLTVSADL;KSKVKLTVSADLNTV-
VKLTVSADL;KTKDLVFLTDGTITV-VFLTDGTIT;DKSKVKLTVSADLNT-
VKLTVSADL;QYNTAGTSLEGSASE-YNTAGTSLE;KVKLTVSADLNTVTL-
LTVSADLNT;VKLTVSADLNTVTLE-LTVSADLNT;YNTAGTSLEGSASEI-
YNTAGTSLE;VGGKTTVEIKEGTVT-VGGKTTVEI;PDKSKVKLTVSADLN-
VKLTVSADL;ANKLDSKKLTRSNGT-LDSKKLTRS;LVGGKTTVEIKEGTV-
VGGKTTVEI;KPDKSKVKLTVSADL-SKVKLTVSA;LKANKLDSKKLTRSN-
LDSKKLTRS;QGSITXETLKANKLD-XETLKANKL;KQGSITXETLKANKL-
ITXETLKAN;GSITXETLKANKLDS-XETLKANKL;TLKANKLDSKKLTRS-
ANKLDSKKL;NKLDSKKLTRSNGTT-LDSKKLTRS;KWEDSTSTLTISADS-
WEDSTSTLT;VFLTDGTITVQQYNT-FLTDGTITV;KANKLDSKKLTRSNG-
LDSKKLTRS;NTAGTSLEGSASEIK-GTSLEGSAS;WEDSTSTLTISADSK-
WEDSTSTLT;DASNQKISSKVTKKQ-NQKISSKVT;FLTDGTITVQQYNTA-
FLTDGTITV;LDSKKLTRSNGTTLE-LTRSNGTTL;ASNQKISSKVTKKQG-
ISSKVTKKQ;TAGTSLEGSASEIKN-LEGSASEIK;SKVTKKQGSITXETL-
VTKKQGSIT;LTVSADLNTVTLEAF-VSADLNTVT;SSKVTKKQGSITXET-
VTKKQGSIT;KLTVSADLNTVTLEA-VSADLNTVT;ALIGCAQKGAESIGS-LIGCAQKGA
DRB1_0301
```

DRB1_0401 FNGNKIFVSKEKNSS-FNGNKIFVS;NKIFVSKEKNSSGKY-
FVSKEKNSS;GNKIFVSKEKNSSGK-FVSKEKNSS;KIFVSKEKNSSGKYD-
FVSKEKNSS;NGNKIFVSKEKNSSG-FVSKEKNSS;ETLKNSIKLEGSLVG-LKNSIKLEG
DRB1_0404 GKVKVFLNDTAGSNK-VKVFLNDTA;DGKVKVFLNDTAGSN-
VKVFLNDTA;KVKVFLNDTAGSNKK-FLNDTAGSN;VKVFLNDTAGSNKKT-FLNDTAGSN;
DRB1_0405 TITVQQYNTAGTSLE-VQQYNTAGT;ITVQQYNTAGTSLEG-
YNTAGTSLE;TVQQYNTAGTSLEGS-YNTAGTSLE;VQQYNTAGTSLEGSA-
YNTAGTSLE;QQYNTAGTSLEGSAS-YNTAGTSLE
DRB1_0701
DRB1_0802
DRB1_0901 MRLLIGFALALALIG-IGFALALAL;RLLIGFALALALIGC-
IGFALALAL;LLIGFALALALIGCA-IGFALALAL;ITVQQYNTAGTSLEG-
VQQYNTAGT;TITVQQYNTAGTSLE-VQQYNTAGT;LIGFALALALIGCAQ-
FALALALIG;TGKWEDSTSTLTISA-WEDSTSTLT;KTGKWEDSTSTLTIS-
WEDSTSTLT;KKTGKWEDSTSTLTI-WEDSTSTLT;
DRB1_1101
DRB1_1302 ETLKNSIKLEGSLVG-LKNSIKLEG;KAVETLKNSIKLEGS-
LKNSIKLEG;TKAVETLKNSIKLEG-VETLKNSIK;AVETLKNSIKLEGSL-
LKNSIKLEG;VETLKNSIKLEGSLV-LKNSIKLEG;TLKNSIKLEGSLVGG-
LKNSIKLEG;LKNSIKLEGSLVGGK-LKNSIKLEG;DSVSLFNGNKIFVSK-
VSLFNGNKI;EDSVSLFNGNKIFVS-VSLFNGNKI;TITVQQYNTAGTSLE-
VQQYNTAGT;ITVQQYNTAGTSLEG-YNTAGTSLE;VSLFNGNKIFVSKEK-
VSLFNGNKI;SVSLFNGNKIFVSKE-VSLFNGNKI;TEDSVSLFNGNKIFV-
VSLFNGNKI;VQQYNTAGTSLEGSA-YNTAGTSLE;TVQQYNTAGTSLEGS-
YNTAGTSLE;EGSASEIKNLSELKN-SASEIKNLS;ASEIKNLSELKNALK-
IKNLSELKN;SASEIKNLSELKNAL-IKNLSELKN;GSASEIKNLSELKNA-
IKNLSELKN;QQYNTAGTSLEGSAS-YNTAGTSLE;LDSKKLTRSNGTTLE-
LTRSNGTTL;NSIKLEGSLVGGKTT-IKLEGSLVG;VTEDSVSLFNGNKIF-
VSLFNGNKI;KNSIKLEGSLVGGKT-IKLEGSLVG
DRB1_1501 MRLLIGFALALALIG-LIGFALALA;RLLIGFALALALIGC-
LIGFALALA;LKNSIKLEGSLVGGK-IKLEGSLVG;KDGKVKVFLNDTAGS-
VKVFLNDTA;DGKVKVFLNDTAGSN-VKVFLNDTA;KNSIKLEGSLVGGKT-IKLEGSLVG
DRB3_0101
DRB4_0101
DRB5_0101

**<NP_047005 outer surface protein C;Protein;Borrelia burgdorferi B31>**

**Allele 8-mer**
A0101
A0201
KLFESVEV;LLSSIDEI;LMTLFLFI;LLAGAYAI;VLLAVKEV;SLLAGAYA;AILMTLFL;KITDSNAV
;TLSAILMT
A0202
KLFESVEV;LLSSIDEI;LLAGAYAI;LMTLFLFI;KITDSNAV;KIHQNNGL;TLSAILMT;GAYAISTL
;ILMTLFLF;SLLAGAYA;VLLAVKEV;LSAILMTL
A0203
KLFESVEV;LLAGAYAI;LLSSIDEI;LMTLFLFI;VLLAVKEV;KITDSNAV;SLLAGAYA;TLSAILMT
;GAYAISTL;GLKEKIDA;AVKEVEAL;KIHQNNGL;AILMTLFL
A0204 KLFESVEV;VLLAVKEV;SLLAGAYA;LLAGAYAI;
A0206
KLFESVEV;KITDSNAV;LLAGAYAI;AILMTLFL;KEMLANSV;VLLAVKEV;LMTLFLFI;SLLAGAYA
;ILMTLFLF;LLSSIDEI;GAYAISTL
A0211
KLFESVEV;VLLAVKEV;LLAGAYAI;SLLAGAYA;KITDSNAV;LMTLFLFI;AILMTLFL;LLSSIDEI
;TLFLFISC;TLSAILMT;ELTSPVVA;ILMTLFLF;KIHQNNGL;SIDEIAAK;KLDGLKNE

A0212
KLFESVEV;VLLAVKEV;LLAGAYAI;SLLAGAYA;KITDSNAV;LMTLFLFI;LLSSIDEI;AILMTLFL
;TLFLFISC
A0216
KLFESVEV;VLLAVKEV;SLLAGAYA;LLAGAYAI;AILMTLFL;KITDSNAV;LLSSIDEI;LMTLFLFI
;TLFLFISC;TLSAILMT;GAYAISTL
A0219
LLAGAYAI;VLLAVKEV;KLFESVEV;LLSSIDEI;SLLAGAYA;KITDSNAV;LMTLFLFI;AILMTLFL
A0301 KTNGTKTK;ILKTNGTK;VVAESPKK;GLKNEGLK;IAAKAIGK;NLTEISKK
A1101
KTNGTKTK;VVAESPKK;ISCNNSGK;YAISTLIK;SIDEIAAK;GVTDADAK;ETFTNKLK;CSETFTNK
;IAAKAIGK;AAKAIGKK;FTNKLKEK;ISTLIKQK;PVVAESPK;EVLSKAAK;NSADESVK;ESVEVLS
K;KQKLDGLK;EMLANSVK;NAVLLAVK
A2301 ILMTLFLF;AYAISTLI
A2402 AYAISTLI;ILMTLFLF
A2403 AYAISTLI
A2601
A2602
A2902 ILMTLFLF
A3001 KTNGTKTK;KQKLDGLK;AAKAIGKK;ILKTNGTK
A3002
A3101 KTNGTKTK
A3301 DAKEAILK;EVLSKAAK
A6801
ETFTNKLK;ESVEVLSK;YAISTLIK;NAVLLAVK;EVLSKAAK;FTNKLKEK;NSADESVK;NLTEISKK
;ISCNNSGK;DAKEAILK;IAAKAIGK;ISTLIKQK;EMLANSVK;CSETFTNK;VVAESPKK;GVTDADA
K;EKIDAAKK
A6802 LLAGAYAI
A6901 NTLSAILM
B0702
B0801
B0802
B1501 ILMTLFLF;GSLLAGAY;
B1801
B2705
B3501 SAILMTLF
B3901
B4001 SETFTNKL;AEELGKLF;KEMLANSV
B4002 KELTSPVV;KEMLANSV;
B4402
B4403 AEELGKLF;KEVEALLS;
B4501 AEELGKLF
B5101
B5301 SAILMTLF
B5401
B5701
B5801

**Allele 9-mer**
A0101
A0201
ILMTLFLFI;ALLSSIDEI;KLFESVEVL;TLSAILMTL;SLLAGAYAI;MLANSVKEL;LLSSIDEIA;K
ITDSNAVL
A0202
TLSAILMTL;ILMTLFLFI;KLFESVEVL;LLSSIDEIA;MLANSVKEL;ALLSSIDEI;SVKELTSPV;F
LFISCNNS;LLAVKEVEA;GLKEKIDAA;GAYAISTLI;SLLAGAYAI;LIKQKLDGL;ELGKLFESV;LL

AGAYAIS;KITDSNAVL;KLKEKHTDL;SANSADESV;NLTEISKKI;SAILMTLFL;LAVKEVEAL;AVK
EVEALL

A0203
SVKELTSPV;MLANSVKEL;TLSAILMTL;GLKEKIDAA;LIKQKLDGL;ILMTLFLFI;KLFESVEVL;K
LKEKHTDL;ALLSSIDEI;SLLAGAYAI;LLAVKEVEA;LLSSIDEIA;ELGKLFESV;GAYAISTLI;MK
KNTLSAI;AVKEVEALL;AVLLAVKEV;LLAGAYAIS;FLFISCNNS

A0204
SLLAGAYAI;TLSAILMTL;KLFESVEVL;MLANSVKEL;ALLSSIDEI;LLAVKEVEA;KLKEKHTDL;I
LMTLFLFI

A0206
SLLAGAYAI;ILMTLFLFI;ALLSSIDEI;KLFESVEVL;TLSAILMTL;SVKELTSPV;SAILMTLFL;G
AYAISTLI;KITDSNAVL;HQNNGLDTE;AVLLAVKEV;KKITDSNAV;AILMTLFLF;SIDEIAAKA;EL
GKLFESV

A0211
SLLAGAYAI;KLFESVEVL;TLSAILMTL;ELGKLFESV;MLANSVKEL;ILMTLFLFI;SIDEIAAKA;A
LLSSIDEI;KITDSNAVL;SVKELTSPV;NLTEISKKI;HTDLGKEGV;AVLLAVKEV;LLAVKEVEA;IT
DSNAVLL;KLDGLKNEG;LLSSIDEIA;GLKEKIDAA;KLKEKHTDL;VLSKAAKEM;AVKEVEALL;SAN
SADESV

A0212
SLLAGAYAI;ELGKLFESV;TLSAILMTL;KLFESVEVL;ILMTLFLFI;KLKEKHTDL;ALLSSIDEI;M
LANSVKEL;GLKEKIDAA;HTDLGKEGV;LLAVKEVEA;SVKELTSPV;SIDEIAAKA

A0216
ELGKLFESV;TLSAILMTL;SLLAGAYAI;ALLSSIDEI;MLANSVKEL;KLFESVEVL;ILMTLFLFI;S
VKELTSPV;LLAVKEVEA;AVLLAVKEV;SIDEIAAKA;AVKEVEALL;KITDSNAVL;VLSKAAKEM;SA
NSADESV;LLSSIDEIA;NLTEISKKI;HTDLGKEGV

A0219
SLLAGAYAI;TLSAILMTL;ALLSSIDEI;ILMTLFLFI;ELGKLFESV;HTDLGKEGV;MLANSVKEL;K
LFESVEVL;SANSADESV;ITDSNAVLL

A0301  IAAKAIGKK;AILKTNGTK;AISTLIKQK;FISCNNSGK
A1101
SSIDEIAAK;AILKTNGTK;AISTLIKQK;AYAISTLIK;IAAKAIGKK;EIAAKAIGK;FISCNNSGK;P
VVAESPKK

A2301  AILMTLFLF;AYAISTLIK
A2402  AILMTLFLF;
A2403
A2601
A2602  EVEALLSSI
A2902
A3001  AILKTNGTK;AYAISTLIK;KEKHTDLGK
A3002
A3101
A3301
A6801  FISCNNSGK;EIAAKAIGK;SSIDEIAAK;IAAKAIGKK;ETFTNKLKE;
A6802
SVKELTSPV;ELGKLFESV;TLSAILMTL;EVEALLSSI;DSNAVLLAV;ESVEVLSKA;SANSADESV;S
AILMTLFL;ILMTLFLFI;HTDLGKEGV;LTSPVVAES

A6901  ELGKLFESV;HTDLGKEGV;SLLAGAYAI;NTLSAILMT;DSNAVLLAV;
B0702
B0801
B0802
B1501  KLFESVEVL
B1801  DESVKGPNL
B2705
B3501  LAVKEVEAL;NGSLLAGAY
B3901
B4001  TENNHNGSL

```
B4002 TENNHNGSL
B4402
B4403
B4501
B5101
B5301
B5401
B5701
B5801 LSAILMTLF
```

**Allele 10-mer**
```
A0101
A0201
ALLSSIDEIA;AILMTLFLFI;LLAGAYAIST;LLSSIDEIAA;NTLSAILMTL;KITDSNAVLL;ILMTL
FLFIS;VLLAVKEVEA;LLAVKEVEAL;KLDGLKNEGL;KLFESVEVLS
A0202
LLAVKEVEAL;LLSSIDEIAA;KLDGLKNEGL;ALLSSIDEIA;LLAGAYAIST;NTLSAILMTL;FLFIS
CNNSG;LAGAYAISTL;ILMTLFLFIS;MLANSVKELT;LSAILMTLFL;VLSKAAKEML;KITDSNAVLL
;KLFESVEVLS;AILMTLFLFI;TSANSADESV;TLIKQKLDGL;VLLAVKEVEA;LMTLFLFISC
A0203
AAKEMLANSV;LLAVKEVEAL;LLAGAYAIST;SVKELTSPVV;TLIKQKLDGL;MLANSVKELT;LLSSI
DEIAA;SVKGPNLTEI;VLSKAAKEML;ALLSSIDEIA;NTLSAILMTL;NSVKELTSPV
A0204 VLLAVKEVEA;LLAVKEVEAL;EMLANSVKEL
A0206
KEVEALLSSI;AILMTLFLFI;NTLSAILMTL;KITDSNAVLL;LLAGAYAIST;TLIKQKLDGL;SVKEL
TSPVV;AAKEMLANSV;ALLSSIDEIA;KLDGLKNEGL;NSVKELTSPV
A0211
KLDGLKNEGL;KITDSNAVLL;VLLAVKEVEA;EMLANSVKEL;SIDEIAAKAI;LLAGAYAIST;VLSKA
AKEML;SVKELTSPVV;AILMTLFLFI;LLAVKEVEAL;LLSSIDEIAA;KLFESVEVLS;TLIKQKLDGL
;ALLSSIDEIA;SLLAGAYAIS;MLANSVKELT;LMTLFLFISC;AISTLIKQKL;TLSAILMTLF;NTLS
AILMTL;SVKGPNLTEI
A0212
KLDGLKNEGL;VLLAVKEVEA;LLAVKEVEAL;TLIKQKLDGL;VLSKAAKEML;LLSSIDEIAA;LLAGA
YAIST;EMLANSVKEL;SVKELTSPVV;SIDEIAAKAI;ALLSSIDEIA;SLLAGAYAIS;AILMTLFLFI
A0216
KLDGLKNEGL;VLSKAAKEML;VLLAVKEVEA;LLAGAYAIST;TLIKQKLDGL;LLAVKEVEAL;EMLAN
SVKEL;SVKELTSPVV;KITDSNAVLL;ALLSSIDEIA;LLSSIDEIAA;AILMTLFLFI;MLANSVKELT
;AISTLIKQKL;SVKGPNLTEI;
A0219
KLDGLKNEGL;VLSKAAKEML;LLAGAYAIST;LLAVKEVEAL;EMLANSVKEL;NTLSAILMTL;TLIKQ
KLDGL;VLLAVKEVEA
A0301 GAYAISTLIK;ILKTNGTKTK;KTKGAEELGK;GLKNEGLKEK
A1101
GAYAISTLIK;SANSADESVK;SVEVLSKAAK;TSPVVAESPK;KTKGAEELGK;DSNAVLLAVK;YAIST
LIKQK;LSSIDEIAAK;ETFTNKLKEK;CSETFTNKLK;EIAAKAIGKK
A2301 SAILMTLFLF;LFISCNNSGK
A2402 SAILMTLFLF
A2403
A2601
A2602 EVLSKAAKEM;DTENNHNGSL
A2902
A3001
KTKGAEELGK;ILKTNGTKTK;GAYAISTLIK;SVEVLSKAAK;TSPVVAESPK;KGPNLTEISK;
A3002 TLSAILMTLF;
A3101
A3301
```

A6801
ETFTNKLKEK;EIAAKAIGKK;YAISTLIKQK;EAILKTNGTK;DSNAVLLAVK;TSPVVAESPK;SANSA
DESVK;GAYAISTLIK;CSETFTNKLK;LFISCNNSGK;LSSIDEIAAK;SVEVLSKAAK
A6802
TSANSADESV;NSVKELTSPV;LSAILMTLFL;NTLSAILMTL;NAVLLAVKEV;ESVEVLSKAA;AGAYA
ISTLI;SSIDEIAAKA
A6901 NTLSAILMTL;EMLANSVKEL;EALLSSIDEI;EVLSKAAKEM
B0702
B0801
B0802
B1501
B1801 KEVEALLSSI;
B2705
B3501 SAILMTLFLF;
B3901
B4001 KEVEALLSSI;TENNHNGSLL
B4002 TENNHNGSLL
B4402
B4403
B4501
B5101
B5301 SAILMTLFLF
B5401
B5701B5801 SAILMTLFLF

**Allele 11-mer**
A0101
A0201
TLSAILMTLFL;LLAGAYAISTL;ALLSSIDEIAA;VLLAVKEVEAL;KITDSNAVLLA;LLAVKEVEALL
;SLLAGAYAIST;ITDSNAVLLAV
A0202
TLSAILMTLFL;LLAGAYAISTL;LLAVKEVEALL;VLLAVKEVEAL;FLFISCNNSGK;KAAKEMLANSV
;ELGKLFESVEV;LLSSIDEIAAK;ALLSSIDEIAA;VLSKAAKEMLA;LAGAYAISTLI;NTSANSADES
V;ILMTLFLFISC
A0203
LLAGAYAISTL;TLSAILMTLFL;LLAVKEVEALL;KAAKEMLANSV;VLSKAAKEMLA;SVKELTSPVVA
;ALLSSIDEIAA;VLLAVKEVEAL;MLANSVKELTS;ELGKLFESVEV;ILMTLFLFISCA0204
LLAGAYAISTL;VLLAVKEVEAL;LLAVKEVEALL;TLSAILMTLFL;SLLAGAYAISTA0206
KAAKEMLANSV;TLSAILMTLFL;LLAGAYAISTL;ALLSSIDEIAA;SAILMTLFLFI;ITDSNAVLLAV
;SLLAGAYAIST;ILMTLFLFISC;YAISTLIKQKL;KITDSNAVLLA;VLLAVKEVEAL
A0211
TLSAILMTLFL;LLAGAYAISTL;SLLAGAYAIST;ELGKLFESVEV;VLLAVKEVEAL;ITDSNAVLLAV
;ALLSSIDEIAA;LLAVKEVEALL;KITDSNAVLLA;VLSKAAKEMLA;KLFESVEVLSK;ILMTLFLFIS
C;KLDGLKNEGLK;KAAKEMLANSV;GVTDADAKEAI;YAISTLIKQKL
A0212
VLLAVKEVEAL;LLAGAYAISTL;ELGKLFESVEV;TLSAILMTLFL;SLLAGAYAIST;ITDSNAVLLAV
;LLAVKEVEALL;ALLSSIDEIAA;VLSKAAKEMLA;ILMTLFLFISC
A0216
LLAGAYAISTL;ELGKLFESVEV;TLSAILMTLFL;SLLAGAYAIST;LLAVKEVEALL;VLLAVKEVEAL
;ALLSSIDEIAA;VLSKAAKEMLA;ITDSNAVLLAV
A0219
LLAGAYAISTL;TLSAILMTLFL;VLLAVKEVEAL;ITDSNAVLLAV;ELGKLFESVEV;SLLAGAYAIST
;NTSANSADESV;ALLSSIDEIAA;LLAVKEVEALL
A0301
KLFESVEVLSK;FLFISCNNSGK;TLIKQKLDGLK;KLDGLKNEGLK;KLKEKHTDLGK;AILKTNGTKTK
;GLKEKIDAAKK;LTSPVVAESPK;LLSSIDEIAAK

A1101
KLFESVEVLSK;TSANSADESVK;LTSPVVAESPK;TLIKQKLDGLK;TSPVVAESPKK;AILKTNGTKTK
;AAKEMLANSVK;KLDGLKNEGLK;FLFISCNNSGK;LLSSIDEIAAK
A2301 AYAISTLIKQK;NTLSAILMTLF
A2402
A2403
A2601
A2602 EVLSKAAKEML;DTENNHNGSLL;NTLSAILMTLF
A2902
A3001
KLFESVEVLSK;KLKEKHTDLGK;LTSPVVAESPK;AILKTNGTKTK;AAKEMLANSVK;TSPVVAESPKK
;KCSETFTNKLK
A3002
A3101
A3301
A6801
ESVEVLSKAAK;LTSPVVAESPK;TSANSADESVK;FLFISCNNSGK;TSPVVAESPKK;TLIKQKLDGLK
;ETFTNKLKEKH;LLSSIDEIAAK
A6802
NTSANSADESV;TLSAILMTLFL;SAILMTLFLFI;EIAAKAIGKKI;ESVKGPNLTEI;ELGKLFESVEV
;NGSLLAGAYAI;
A6901 ITDSNAVLLAV;EVLSKAAKEML;ELGKLFESVEV;NTSANSADESV;EIAAKAIGKKI
B0702
B0801
B0802
B1501
B1801 VEVLSKAAKEM
B2705
B3501 NHNGSLLAGAY
B3901
B4001 KEMLANSVKEL;AEELGKLFESV;
B4002 KEMLANSVKEL
B4402
B4403
B4501 AEELGKLFESV
B5101
B5301 SAILMTLFLFI
B5401
B5701
B5801 LSAILMTLFLF

**Allele 15-mer plus 9-mer core**
DRB1_0101
GSLLAGAYAISTLIK-LAGAYAIST;HNGSLLAGAYAISTL-LAGAYAIST;NGSLLAGAYAISTLI-
LAGAYAIST;NHNGSLLAGAYAIST-LLAGAYAIS;SLLAGAYAISTLIKQ-
LAGAYAIST;LLAGAYAISTLIKQK-LAGAYAIST;LAGAYAISTLIKQKL-
LAGAYAIST;ANSVKELTSPVVAES-VKELTSPVV;NSVKELTSPVVAESP-
VKELTSPVV;LANSVKELTSPVVAE-VKELTSPVV;MLANSVKELTSPVVA-
VKELTSPVV;EMLANSVKELTSPVV-MLANSVKEL;SVKELTSPVVAESPK-
VKELTSPVV;VKELTSPVVAESPKK-VKELTSPVV;SVEVLSKAAKEMLAN-
VLSKAAKEM;ESVEVLSKAAKEMLA-VLSKAAKEM;VEVLSKAAKEMLANS-
VLSKAAKEM;FESVEVLSKAAKEML-VLSKAAKEM;EVLSKAAKEMLANSV-
VLSKAAKEM;LFESVEVLSKAAKEM-SVEVLSKAA;VLSKAAKEMLANSVK-
VLSKAAKEM;LAVKEVEALLSSIDE-VKEVEALLS;LLAVKEVEALLSSID-
VKEVEALLS;AVLLAVKEVEALLSS-VKEVEALLS;VLLAVKEVEALLSSI-
VKEVEALLS;NAVLLAVKEVEALLS-LAVKEVEAL;SKKITDSNAVLLAVK-

```
ITDSNAVLL;ISKKITDSNAVLLAV-ITDSNAVLL;KKITDSNAVLLAVKE-
ITDSNAVLL;MKKNTLSAILMTLFL-KNTLSAILM;NTLSAILMTLFLFIS-
LSAILMTLF;AKEAILKTNGTKTKG-LKTNGTKTK;DAKEAILKTNGTKTK-
EAILKTNGT;EISKKITDSNAVLLA-ITDSNAVLL;KNTLSAILMTLFLFI-
LSAILMTLF;KAAKEMLANSVKELT-AKEMLANSV;TLSAILMTLFLFISC-
LMTLFLFIS;TEISKKITDSNAVLL-KKITDSNAV;EAILKTNGTKTKGAE-
LKTNGTKTK;KEAILKTNGTKTKGA-LKTNGTKTK;KELTSPVVAESPKKP-
LTSPVVAES;LSAILMTLFLFISCN-LMTLFLFIS;SKAAKEMLANSVKEL-
AKEMLANSV;SAILMTLFLFISCNN-LMTLFLFIS;KKNTLSAILMTLFLF-
LSAILMTLF;NNHNGSLLAGAYAIS-SLLAGAYAI;AILKTNGTKTKGAEE-
LKTNGTKTK;AAKEMLANSVKELTS-MLANSVKEL;VKEVEALLSSIDEIA-
VKEVEALLS;AKEMLANSVKELTSP-MLANSVKEL;AILMTLFLFISCNNS-
LMTLFLFIS;AVKEVEALLSSIDEI-VKEVEALLS;KITDSNAVLLAVKEV-
ITDSNAVLL;ITDSNAVLLAVKEVE-ITDSNAVLL;EGVTDADAKEAILKT-
VTDADAKEA;KEGVTDADAKEAILK-VTDADAKEA;ADESVKGPNLTEISK-
VKGPNLTEI;LGKEGVTDADAKEAI-VTDADAKEA;DLGKEGVTDADAKEA-
LGKEGVTDA;GKEGVTDADAKEAIL-VTDADAKEA;SADESVKGPNLTEIS-
VKGPNLTEI;DESVKGPNLTEISKK-VKGPNLTEI;ESVKGPNLTEISKKI-
VKGPNLTEI;ILKTNGTKTKGAEEL-LKTNGTKTK;NSADESVKGPNLTEI-
ESVKGPNLT;LKTNGTKTKGAEELG-LKTNGTKTK;SSIDEIAAKAIGKKI-
IDEIAAKAI;LSKAAKEMLANSVKE-AKEMLANSV;KEMLANSVKELTSPV-
MLANSVKEL;LSSIDEIAAKAIGKK-IDEIAAKAI;AGAYAISTLIKQKLD-YAISTLIKQ
DRB1_0301
DRB1_0401
DRB1_0404 ILMTLFLFISCNNSG-FLFISCNNS;LMTLFLFISCNNSGK-
LFISCNNSG;MTLFLFISCNNSGKD-LFISCNNS;TLFLFISCNNSGKDG-
LFISCNNSG;LFLFISCNNSGKDGN-LFISCNNS;KEAILKTNGTKTKGA-
ILKTNGTKT;AKEAILKTNGTKTKG-ILKTNGTKT;EAILKTNGTKTKGAE-ILKTNGTKT
DRB1_0405 LAVKEVEALLSSIDE-VKEVEALLS;ANSVKELTSPVVAES-
VKELTSPVV;LANSVKELTSPVVAE-VKELTSPVV;NSVKELTSPVVAESP-
VKELTSPVV;LLAVKEVEALLSSID-VKEVEALLS;
DRB1_0701 MLANSVKELTSPVVA-VKELTSPVV;ANSVKELTSPVVAES-
VKELTSPVV;LANSVKELTSPVVAE-VKELTSPVV;EMLANSVKELTSPVV-
SVKELTSPV;NSVKELTSPVVAESP-VKELTSPVV;SVKELTSPVVAESPK-
VKELTSPVV;VKELTSPVVAESPKK-VKELTSPVV
DRB1_0802
DRB1_0901 FESVEVLSKAAKEML-EVLSKAAKE;ESVEVLSKAAKEMLA-
LSKAAKEML;SVEVLSKAAKEMLAN-LSKAAKEML
DRB1_1101 KCSETFTNKLKEKHT-FTNKLKEKH;SETFTNKLKEKHTDL-
FTNKLKEKH;ETFTNKLKEKHTDLG-FTNKLKEKH;KKCSETFTNKLKEKH-
KKCSETFTN;CSETFTNKLKEKHTD-FTNKLKEKH;
DRB1_1302 AKEAILKTNGTKTKG-LKTNGTKTK;DAKEAILKTNGTKTK-
EAILKTNGT;KEAILKTNGTKTKGA-LKTNGTKTK;EAILKTNGTKTKGAE-
LKTNGTKTK;AILKTNGTKTKGAEE-LKTNGTKTK;EMLANSVKELTSPVV-
LANSVKELT;KEMLANSVKELTSPV-LANSVKELT;KAAKEMLANSVKELT-
MLANSVKEL;AAKEMLANSVKELTS-LANSVKELT;AKEMLANSVKELTSP-
LANSVKELT;ILKTNGTKTKGAEEL-LKTNGTKTK;MLANSVKELTSPVVA-
LANSVKELT;MKKNTLSAILMTLFL-MKKNTLSAI;LANSVKELTSPVVAE-
LANSVKELT;LAGAYAISTLIKQKL-YAISTLIKQ;AGAYAISTLIKQKLD-
ISTLIKQKL;LKTNGTKTKGAEELG-LKTNGTKTK;GAYAISTLIKQKLDG-
ISTLIKQKL;ADAKEAILKTNGTKT-EAILKTNGT;AYAISTLIKQKLDGL-
ISTLIKQKL;YAISTLIKQKLDGLK-ISTLIKQKL
DRB1_1501 MLANSVKELTSPVVA-VKELTSPVV;EMLANSVKELTSPVV-
NSVKELTSP;LANSVKELTSPVVAE-VKELTSPVV;ANSVKELTSPVVAES-
VKELTSPVV;NSVKELTSPVVAESP-VKELTSPVV;VKELTSPVVAESPKK-VKELTSPVV
DRB3_0101
```

```
DRB4_0101
DRB5_0101 KQKLDGLKNEGLKEK-KQKLDGLKN;DGLKNEGLKEKIDAA-
LKNEGLKEK;QKLDGLKNEGLKEKI-LKNEGLKEK;KLDGLKNEGLKEKID-
LKNEGLKEK;LDGLKNEGLKEKIDA-LKNEGLKEK
```

**<CAH10086 CRASP-1;Protein;Borrelia garinii>**

**Allele 8-mer**
```
A0101
A0201 ILTTILTL;KLNILTTI;ILNETIKA;TILTLICI;TLICISCA;ILNISIKI
A0202 KLNILTTI;ILTTILTL;ILNETIKA;TLICISCA;ILNISIKI
A0203
KLNILTTI;ILNETIKA;TLICISCA;ILTTILTL;ILNISIKI;LICISCAV;KINTLKEI;SIKIQFQI
;NIIKLNIL
A0204 KLNILTTI;ILTTILTL
A0206 FQIENALE;IQFQIENA;ILTTILTL;KLNILTTI;TLICISCA;LICISCAV
A0211
ILTTILTL;ILNETIKA;KLNILTTI;ILNISIKI;KLYKNHKY;TILTLICI;LICISCAV;TLICISCA
;TISEDIEM;KINTLKEI
A0212 ILTTILTL;ILNETIKA;KLNILTTI;KLYKNHKY;LICISCAV;HMDENYKE
A0216
ILTTILTL;KLNILTTI;ILNETIKA;LICISCAV;TLICISCA;QIENALEL;KLYKNHKY;ILNISIKI
;EIEKINTL
A0219 ILTTILTL;ILNETIKA
A0301
IIYSSLNY;KLYKNHKY;FILNISIK;KILNETIK;KTKLNIIK;TIISKLEK;SLNYEIEK;TLKEILDK
;
A1101
TIISKLEK;SLNYEIEK;KTKLNIIK;IIYSSLNY;KILNETIK;EILDKLYK;FILNISIK;NTKDFENK
;ISEDIEMK;CISCAVDK;TLKEILDK
A2301 LYKNHKYK;NYKEFDPL
A2402 NFILNISI
A2403 QFQIENAL
A2601 EILNQERY
A2602 EILNQERY;IIYSSLNY;TTARNFIL;EIEKINTL;NIIKLNIL;TISEDIEM;QIDFLENF
A2902 KLYKNHKY;IIYSSLNY
A3001 KTKLNIIK;LYKNHKYK;KILNETIK;
A3002 IIYSSLNY
A3101 LYKNHKYK;KTKLNIIK;KIKRIIYS;
A3301 DIEMKIKR
A6801
NTKDFENK;TIISKLEK;FILNISIK;EILDKLYK;CISCAVDK;ETIKAYNQ;DIEMKIKR;SLNYEIEK
;ISEDIEMK;ENALELMK;HKYKTTAR;ERYEILLK;NLKQKFEK;EPSNTKNK
A6802 TTARNFIL;YSSLNYEI;EIAKITNT;TLICISCA;LTTILTLI
A6901 TILTLICI;TTARNFIL
B0702
B0801
B0802
B1501 KLYKNHKY;IIYSSLNY
B1801 QERYEILL;KEFDPLNL;YEILLKHV
B2705 KRIIYSSL;ERYEILLK
B3501 LAHMDENY;TISEDIEM
B3901 TTARNFIL;NQERYEIL
B4001 KEFDPLNL;QERYEILL;IEDASEIL;FENKSQDL;KETIISKL;YEILLKHV;LEKMIKDL
B4002 KEFDPLNL;QERYEILL;FENKSQDL
B4402 KEILDKLY;
```

B4403 KEILDKLY;KEFDPLNL
B4501
B5101
B5301
B5401
B5701
B5801 ITNTQIDF

**Allele 9-mer**
A0101
A0201
FILNISIKI;ILTTILTLI;TLICISCAV;FQIENALEL;SLNYEIEKI;KLNILTTIL;NILTTILTL;K
ILNETIKA;KLNIIKLNI
A0202
ILTTILTLI;KLNILTTIL;TLKEILDKL;SLNYEIEKI;TLICISCAV;IQFQIENAL;ILNQERYEI;F
QIENALEL;ITNTQIDFL;FILNISIKI;KLNIIKLNI;CISCAVDKI;LLKHVEPSL
A0203
ILTTILTLI;TLICISCAV;KLNILTTIL;TLKEILDKL;SLNYEIEKI;LMKEEIEDA;LLKHVEPSL;I
LNQERYEI;NLKQKFEKI;KLNIIKLNI;FILNISIKI;FQIENALEL;CISCAVDKI;KIQFQIENA
A0204 FQIENALEL;ILNQERYEI
A0206
FQIENALEL;TLICISCAV;TQIDFLENF;FILNISIKI;YEIEKINTL;ILTTILTLI;IQFQIENAL;N
ILTTILTL;KILNETIKA;IKLNILTTI;
A0211
TLICISCAV;ILNQERYEI;ILTTILTLI;FILNISIKI;TLKEILDKL;KLNILTTIL;NILTTILTL;K
LNIIKLNI;LLKHVEPSL;SLNYEIEKI;HMDENYKEF;KILNETIKA;FQIENALEL;ILNETIKAY;IL
TLICISC;ALELMKEEI
A0212
LLKHVEPSL;TLICISCAV;TLKEILDKL;FILNISIKI;NILTTILTL;FQIENALEL;ILNQERYEI;L
MKEEIEDA;ILTTILTLI;KLNILTTIL;SLNYEIEKI;HMDENYKEF;KLNIIKLNI
A0216
TLICISCAV;LLKHVEPSL;ILNQERYEI;TLKEILDKL;ILTTILTLI;NILTTILTL;FILNISIKI;K
LNILTTIL;ALELMKEEI;KLNIIKLNI;
A0219 NILTTILTL;TLICISCAV;ILTTILTLI;ILNQERYEI;LLKHVEPSL;
A0301 KLYKNHKYK;RIIYSSLNY;HVEPSLNLK
A1101
SSLNYEIEK;RIIYSSLNY;KLYKNHKYK;TISEDIEMK;HVEPSLNLK;QIDFLENFK;ETIISKLEK;N
TLKEILDK;NSKETIISK;MIKDLEDQK;LAHMDENYK;ISKLEKMIK
A2301 KYKTTARNF;IYSSLNYEI;NFILNISIK;TQIDFLENF
A2402 IYSSLNYEI;KYKTTARNF
A2403 KYKTTARNF;AYNQDLDNI;HMDENYKEF;IYSSLNYEI;
A2601 TIISKLEKM;DTISEDIEM
A2602
TIISKLEKM;QIENALELM;DTISEDIEM;RIIYSSLNY;EIEDASEIL;ITNTQIDFL;EMKIKRIIY;T
TILTLICI
A2902 EFDPLNLDY;RIIYSSLNY;QLAHMDENY
A3001 KLYKNHKYK;ISKLEKMIK;ESKSKTNTK;SSLNYEIEK;LAHMDENYK;LDKLYKNHK
A3002 IYSSLNYEI;RIIYSSLNY;EFDPLNLDY;QLAHMDENY;KITNTQIDF;KYKTTARNF
A3101 KLYKNHKYK;ASEILNQER;KIKRIIYSS;KTTARNFIL;
A3301 NHKYKTTAR
A6801
ETIISKLEK;EIEKINTLK;TISEDIEMK;HVEPSLNLK;MIKDLEDQK;LAHMDENYK;QIDFLENFK;E
SKSKTNTK;NFILNISIK;SSLNYEIEK;EDIEMKIKR;EIAKITNTQ;NSKETIISK;NTLKEILDK;EP
SLNLKQK
A6802
TLICISCAV;TTILTLICI;ISIKIQFQI;ITNTQIDFL;CISCAVDKI;FILNISIKI;EIAKITNTQ

```
A6901 FILNISIKI;TLICISCAV;NILTTILTL;TTILTLICI;
B0702
B0801
B0802
B1501
ILNETIKAY;FQIENALEL;IQFQIENAL;RIIYSSLNY;TQIDFLENF;EMKIKRIIY;QLAHMDENY
B1801 YEIEKINTL;FQIENALEL
B2705
B3501 ILNETIKAY;DTISEDIEM
B3901 FQIENALEL;YKEFDPLNL;YEIEKINTL;KHVEPSLNL
B4001 YEIEKINTL;FQIENALEL;IQFQIENAL;
B4002 EEIEDASEI;YEIEKINTL
B4402 SEILNQERY
B4403 SEILNQERY;EEIEDASEI
B4501 EEIEDASEI
B5101
B5301
B5401
B5701
B5801 ISIKIQFQI;RIIYSSLNY
```

**Allele 10-mer**
```
A0101 ASEILNQERY
A0201
ILLKHVEPSL;IIYSSLNYEI;FQIENALELM;NILTTILTLI;LTLICISCAV;ILTLICISCA;KLNIL
TTILT
A0202
IIYSSLNYEI;FQIENALELM;LTLICISCAV;ILNQERYEIL;ILLKHVEPSL;TISEDIEMKI;KLNII
KLNIL;NILTTILTLI;KITNTQIDFL;ILTLICISCA;KIKRIIYSSL;KAYNQDLDNI;KLNILTTILT
A0203
KIKRIIYSSL;IIYSSLNYEI;KLNIIKLNIL;IIKLNILTTI;ILNQERYEIL;KLNILTTILT;ILLKH
VEPSL;ILTLICISCA;NILTTILTLI;LTLICISCAV;TIISKLEKMI
A0204 ILLKHVEPSL
A0206 FQIENALELM;IIYSSLNYEI;ILLKHVEPSL;LTLICISCAV;NILTTILTLI;KITNTQIDFL
A0211
ILLKHVEPSL;ILNQERYEIL;IIYSSLNYEI;TISEDIEMKI;KLYKNHKYKT;KLNIIKLNIL;KITNT
QIDFL;ILTLICISCA;NILTTILTLI;KLNILTTILT;LTLICISCAV;EILNQERYEI;NISIKIQFQI
;KIKRIIYSSL;ILTTILTLIC
A0212 ILLKHVEPSL;KLYKNHKYKT;ILNQERYEIL;IIYSSLNYEI;TISEDIEMKI;KLNIIKLNIL
A0216
ILLKHVEPSL;KLYKNHKYKT;ILNQERYEIL;IIYSSLNYEI;KITNTQIDFL;KLNIIKLNIL;KIKRI
IYSSL;PLEKNSKETI;EIAKITNTQI;NLKQKFEKIL;ILTLICISCA
A0219 ILLKHVEPSL;ILNQERYEIL;IIYSSLNYEI
A0301 SLNLKQKFEK;IISKLEKMIK;LICISCAVDK;ILDKLYKNHK;TQIDFLENFK
A1101
TQIDFLENFK;KTNTKDFENK;SLNLKQKFEK;AVDKIDPESK;KMIKDLEDQK;YSSLNYEIEK;IISKL
EKMIK;LICISCAVDK;SQDLEPSNTK;QIENALELMK;QLAHMDENYK;NQERYEILLK
A2301
KYKTTARNFI;NYKEFDPLNL;NYEIEKINTL;AYNQDLDNIK;NFILNISIKI;AHMDENYKEF;NTQID
FLENF
A2402 KYKTTARNFI;NFILNISIKI
A2403 AHMDENYKEF;QFQIENALEL;KYKTTARNFI
A2601 ETIISKLEKM;ETIKAYNQDL
A2602 ETIISKLEKM;ETIKAYNQDL;KILNETIKAY;NTQIDFLENF;KIKRIIYSSL
A2902
A3001 KNKDLEPLEK;SLNLKQKFEK;KTNTKDFENK;KNHKYKTTAR;KYKTTARNFI
```

A3002
A3101 KNHKYKTTAR
A3301
A6801 DASEILNQER;DTISEDIEMK;YSSLNYEIEK;TQIDFLENFK;QLAHMDENYK;LICISCAVDK
A6802 TTARNFILNI;EIAKITNTQI;ETIKAYNQDL;LTLICISCAV;TISEDIEMKI;NISIKIQFQI
A6901
LTLICISCAV;TTARNFILNI;EIAKITNTQI;NALELMKEEI;NILTTILTLI;EILNQERYEI;ETIKA
YNQDL;ETIISKLEKM;IIYSSLNYEI
B0702
B0801
B0802
B1501 FQIENALELM;KILNETIKAY
B1801 IEMKIKRIIY;KEFDPLNLDY;EEIEDASEIL;
B2705 KRIIYSSLNY
B3501 EPSLNLKQKF
B3901 YKTTARNFIL
B4001 EEIEDASEIL
B4002 EEIEDASEIL;KEFDPLNLDY;
B4402 KEFDPLNLDY
B4403 KEFDPLNLDY;EEIEDASEIL;
B4501 EEIEDASEIL
B5101
B5301 EPSLNLKQKF
B5401
B5701
B5801 KSNEDQLAHM

**Allele 11-mer**
A0101 ILDKLYKNHKY
A0201
ILTTILTLICI;KLNILTTILTL;ILTLICISCAV;RIIYSSLNYEI;ILNQERYEILL;NIIKLNILTTI
A0202
ILNQERYEILL;ILTLICISCAV;KLNILTTILTL;ILTTILTLICI;SLNLKQKFEKI;IQFQIENALEL
;KLNIIKLNILT;LLKHVEPSLNL
A0203
KLNILTTILTL;ILTLICISCAV;LLKHVEPSLNL;ILTTILTLICI;SLNLKQKFEKI;NIIKLNILTTI
;ILNQERYEILL;SIKIQFQIENA;RIIYSSLNYEI;IIKLNILTTIL;MKIKRIIYSSL;ILNISIKIQF
Q;KLYKNHKYKTT
A0204 KLNILTTILTL;ILNQERYEILL
A0206
RIIYSSLNYEI;IQFQIENALEL;NIIKLNILTTI;ILTLICISCAV;TQIDFLENFKS;FILNISIKIQF
;ILTTILTLICI;MKIKRIIYSSL
A0211
ILTLICISCAV;ILNQERYEILL;KLNILTTILTL;ILTTILTLICI;LLKHVEPSLNL;SLNLKQKFEKI
;KLNIIKLNILT;KLYKNHKYKTT;ILDKLYKNHKY;ILNETIKAYNQ;NIIKLNILTTI;FILNISIKIQ
F;EILLKHVEPSL
A0212
ILTLICISCAV;ILNQERYEILL;LLKHVEPSLNL;KLNILTTILTL;ILTTILTLICI;KLYKNHKYKTT
;ILNETIKAYNQ
A0216
ILTLICISCAV;ILNQERYEILL;KLNILTTILTL;LLKHVEPSLNL;ILTTILTLICI;KLYKNHKYKTT
;NIIKLNILTTI;EILLKHVEPSL
A0219 ILTLICISCAV;ILTTILTLICI;ILNQERYEILL;KLNILTTILTL
A0301 KINTLKEILDK;TLICISCAVDK;TLKEILDKLYK;TIISKLEKMIK;KAYNQDLDNIK
A1101
TIISKLEKMIK;KSQDLEPSNTK;NTQIDFLENFK;TLICISCAVDK;KINTLKEILDK;KAYNQDLDNIK

;TLKEILDKLYK;TISEDIEMKIK;FQIENALELMK;HVEPSLNLKQK;PSLNLKQKFEK;IYSSLNYEIE
K;EILDKLYKNHK
A2301 IYSSLNYEIEK;KYKTTARNFIL;NYEIEKINTLK;FILNISIKIQF;KFEKILNETIK
A2402 KYKTTARNFIL
A2403 KYKTTARNFIL;FILNISIKIQF
A2601 EKILNETIKAY;NTLKEILDKLY
A2602 DIEMKIKRIIY;EILNQERYEIL;EKILNETIKAY;ETIISKLEKMI;EILLKHVEPSL
A2902 ILDKLYKNHKY
A3001 KSQDLEPSNTK;TLKEILDKLYK;KINTLKEILDK
A3002
A3101 IYSSLNYEIEK;ISEDIEMKIKR
A3301 EILDKLYKNHK
A6801
NTQIDFLENFK;TLICISCAVDK;TIISKLEKMIK;EILDKLYKNHK;ISEDIEMKIKR;HVEPSLNLKQK
;EDASEILNQER;CAVDKIDPESK;IYSSLNYEIEK;TLKEILDKLYK;TISEDIEMKIK
A6802
DTISEDIEMKI;TTARNFILNIS;NIIKLNILTTI;RIIYSSLNYEI;ILTLICISCAV;MKIKRIIYSSL
A6901 EPLEKNSKETI;ETIISKLEKMI;EILLKHVEPSL;NIIKLNILTTI
B0702
B0801
B0802
B1501 IQFQIENALEL
B1801 VEPSLNLKQKF
B2705
B3501 DASEILNQERY;LAHMDENYKEF
B3901
B4001 KEEIEDASEIL;SEILNQERYEI;IEKINTLKEIL;NETIKAYNQDL
B4002 SEILNQERYEI
B4402
B4403 SEILNQERYEI;EEIEDASEILN
B4501
B5101 EPLEKNSKETI
B5301 EPLEKNSKETI
B5401
B5701
B5801

**Allele 15-mer plus 9-mer core**
DRB1_0101 LNIIKLNILTTILTL-LNILTTILT;NIIKLNILTTILTLI-
LNILTTILT;IIKLNILTTILTLIC-LNILTTILT;IKLNILTTILTLICI-
LNILTTILT;KLNIIKLNILTTILT-KLNILTTIL;KLNILTTILTLICIS-
LNILTTILT;LNILTTILTLICISC-LNILTTILT;NILTTILTLICISCA-
LTTILTLIC;DKLYKNHKYKTTARN-YKNHKYKTT;KLYKNHKYKTTARNF-
YKNHKYKTT;LDKLYKNHKYKTTAR-YKNHKYKTT;ILDKLYKNHKYKTTA-
YKNHKYKTT;EILDKLYKNHKYKTT-LYKNHKYKT;TKLNIIKLNILTTIL-
IIKLNILTT;DIEMKIKRIIYSSLN-MKIKRIIYS;IEMKIKRIIYSSLNY-
MKIKRIIYS;EDIEMKIKRIIYSSL-MKIKRIIYS;ILTTILTLICISCAV-
LTLICISCA;KIQFQIENALELMKE-FQIENALEL;KTKLNIIKLNILTTI-
IIKLNILTT;IQFQIENALELMKEE-FQIENALEL;NQDLDNIKSNEDQLA-
LDNIKSNED;QDLDNIKSNEDQLAH-IKSNEDQLA;IKIQFQIENALELMK-
FQIENALEL;SIKIQFQIENALELM-FQIENALEL;ISIKIQFQIENALEL-
IQFQIENAL;TTARNFILNISIKIQ-FILNISIKI;SEDIEMKIKRIIYSS-
MKIKRIIYS;ISEDIEMKIKRIIYS-IEMKIKRII;YKNHKYKTTARNFIL-
YKNHKYKTT;LTTILTLICISCAVD-LTLICISCA;ARNFILNISIKIQFQ-
FILNISIKI;LYKNHKYKTTARNFI-YKNHKYKTT;TARNFILNISIKIQF-
FILNISIKI;LDNIKSNEDQLAHMD-IKSNEDQLA;DLDNIKSNEDQLAHM-

```
IKSNEDQLA;RNFILNISIKIQFQI-FILNISIKI;YEILLKHVEPSLNLK-
LKHVEPSLN;EILLKHVEPSLNLKQ-LKHVEPSLN;MKIKRIIYSSLNYEI-
MKIKRIIYS;RYEILLKHVEPSLNL-LKHVEPSLN;ILLKHVEPSLNLKQK-
LKHVEPSLN;KKTKLNIIKLNILTT-LNIIKLNIL;TTILTLICISCAVDK-
LTLICISCA;KTTARNFILNISIKI-ARNFILNIS
DRB1_0301
DRB1_0401 IIKLNILTTILTLIC-LNILTTILT;KLNIIKLNILTTILT-
IIKLNILTT;IKLNILTTILTLICI-LNILTTILT;LNIIKLNILTTILTL-
LNILTTILT;NIIKLNILTTILTLI-LNILTTILT
DRB1_0404 IIKLNILTTILTLIC-ILTTILTLI;NTQIDFLENFKSEPH-FLENFKSEP
DRB1_0405 YEIEKINTLKEILDK-IEKINTLKE;NYEIEKINTLKEILD-
IEKINTLKE;IEKINTLKEILDKLY-INTLKEILD;EIEKINTLKEILDKL-
INTLKEILD;SSLNYEIEKINTLKE-YEIEKINTL;IIKLNILTTILTLIC-
LNILTTILT;IKLNILTTILTLICI-LNILTTILT;SLNYEIEKINTLKEI-
IEKINTLKE;LNYEIEKINTLKEIL-IEKINTLKE;KAYNQDLDNIKSNED-
YNQDLDNIK;AYNQDLDNIKSNEDQ-LDNIKSNED;HMDENYKEFDPLNLD-
YKEFDPLNL;MKIKRIIYSSLNYEI-KRIIYSSLN;MDENYKEFDPLNLDY-
KEFDPLNLD;YNQDLDNIKSNEDQL-LDNIKSNED;ISIKIQFQIENALEL-
IKIQFQIEN;ETIKAYNQDLDNIKS-IKAYNQDLD
DRB1_0701 YKNHKYKTTARNFIL-YKTTARNFI;NHKYKTTARNFILNI-
YKTTARNFI;KNHKYKTTARNFILN-YKTTARNFI;LYKNHKYKTTARNFI-
KYKTTARNF;HKYKTTARNFILNIS-YKTTARNFI;KYKTTARNFILNISI-
YKTTARNFI;KLYKNHKYKTTARNF-YKNHKYKTT
DRB1_0802
DRB1_0901 YKNHKYKTTARNFIL-YKTTARNFI;LYKNHKYKTTARNFI-
NHKYKTTAR;NHKYKTTARNFILNI-YKTTARNFI;KNHKYKTTARNFILN-
YKTTARNFI;HKYKTTARNFILNIS-YKTTARNFI
DRB1_1101
DRB1_1302 LNIIKLNILTTILTL-LNILTTILT;NIIKLNILTTILTLI-
LNILTTILT;IIKLNILTTILTLIC-LNILTTILT;KLNIIKLNILTTILT-
IKLNILTTI;IKLNILTTILTLICI-LNILTTILT;KLNILTTILTLICIS-
LNILTTILT;LNILTTILTLICISC-LNILTTILT;KTKLNIIKLNILTTI-
LNIIKLNIL;TKLNIIKLNILTTIL-IKLNILTTI;RNFILNISIKIQFQI-
FILNISIKI;TARNFILNISIKIQF-FILNISIKI;KKTKLNIIKLNILTT-
LNIIKLNIL;ARNFILNISIKIQFQ-FILNISIKI;NFILNISIKIQFQIE-
LNISIKIQF;TTARNFILNISIKIQ-FILNISIKI;MKKTKLNIIKLNILT-
LNIIKLNIL;FILNISIKIQFQIEN-LNISIKIQF;KTTARNFILNISIKI-
ARNFILNIS;EILDKLYKNHKYKTT-LYKNHKYKT;ILDKLYKNHKYKTTA-
YKNHKYKTT;LYKNHKYKTTARNFI-YKNHKYKTT;LDKLYKNHKYKTTAR-
YKNHKYKTT;EDIEMKIKRIIYSSL-MKIKRIIYS;DKLYKNHKYKTTARN-
YKNHKYKTT;ILNISIKIQFQIENA-LNISIKIQF;IEMKIKRIIYSSLNY-
MKIKRIIYS;DIEMKIKRIIYSSLN-MKIKRIIYS;HKYKTTARNFILNIS-
YKTTARNFI;SEDIEMKIKRIIYSS-MKIKRIIYS;ISEDIEMKIKRIIYS-
IEMKIKRII;KLYKNHKYKTTARNF-YKNHKYKTT;YKTTARNFILNISIK-
ARNFILNIS;YKNHKYKTTARNFIL-YKNHKYKTT;KYKTTARNFILNISI-
ARNFILNIS;EMKIKRIIYSSLNYE-MKIKRIIYS;MKIKRIIYSSLNYEI-
MKIKRIIYS;LNISIKIQFQIENAL-LNISIKIQF;ERYEILLKHVEPSLN-
YEILLKHVE;NYEIEKINTLKEILD-EKINTLKEI;YEIEKINTLKEILDK-
INTLKEILD;NILTTILTLICISCA-ILTTILTLI;LNYEIEKINTLKEIL-
YEIEKINTL;RYEILLKHVEPSLNL-LKHVEPSLN;SLNYEIEKINTLKEI-
YEIEKINTL;YEILLKHVEPSLNLK-LKHVEPSLN;ILTTILTLICISCAV-
ILTTILTLI;KEILDKLYKNHKYKT-LDKLYKNHK
DRB1_1501 NTQIDFLENFKSEPH-IDFLENFKS;TQIDFLENFKSEPHD-
LENFKSEPH;IDFLENFKSEPHDTI-LENFKSEPH;QIDFLENFKSEPHDT-
LENFKSEPH;DFLENFKSEPHDTIS-LENFKSEPH;IEMKIKRIIYSSLNY-
MKIKRIIYS;EDIEMKIKRIIYSSL-MKIKRIIYS;DIEMKIKRIIYSSLN-MKIKRIIYS
```

```
DRB3_0101
DRB4_0101
DRB5_0101 YKNHKYKTTARNFIL-YKTTARNFI;LYKNHKYKTTARNFI-
YKNHKYKTT;DKLYKNHKYKTTARN-YKNHKYKTT;KLYKNHKYKTTARNF-
YKNHKYKTT;EILDKLYKNHKYKTT-LDKLYKNHK;LDKLYKNHKYKTTAR-
YKNHKYKTT;ENKSQDLEPSNTKNK-QDLEPSNTK;NKSQDLEPSNTKNKD-
LEPSNTKNK;KSQDLEPSNTKNKDL-LEPSNTKNK;ILDKLYKNHKYKTTA-
YKNHKYKTT;SQDLEPSNTKNKDLE-LEPSNTKNK;QDLEPSNTKNKDLEP-LEPSNTKNK
```

**<AAU07022 4-alpha-glucanotransferase;Protein;Borrelia garinii PBi>**

**Allele 8-mer**
```
A0101
A0201
QMFAYSPI;LLDFASFV;ILLNISSL;FIDSDLNL;YIFDYLNT;YAFNGLWV;ILSKEIKV;VMNLPLFI
;FAGNVYYI;VLFDRGIL;GISPDYFL;ILRKYIDV;SLPSKYGI
A0202
LLDFASFV;GISPDYFL;ILSKEIKV;QMFAYSPI;FVNSLDDL;FIDSDLNL;ILLNISSL;ALNFINRA
;FLFASSQS;SLPSKYGI;NISFITRL;FAGNVYYI;ILNEIKDL;VLFDRGIL;FDSDNQNL;FSAFAGN
V;YIFDYLNT;VAFKEYFL;FFSQFQAL;ILRKYIDV;SVSDSVII;VMNLPLFI;YAFNGLWV;FIDFLF
AS;MSSVSDSV;KKSSYWLL;YYIDLEAL
A0203
ALNFINRA;LLDFASFV;ILRKYIDV;QMFAYSPI;ILLNISSL;ILSKEIKV;VMNLPLFI;SLPSKYGI
;VLFDRGIL;YAFNGLWV;FLKDSRDA;ILNEIKDL;NLKRKSGI;GMRIMKLA;YIFDYLNT;NISFITR
L;FAGNVYYI;FQALKRYA;GISPDYFL;SVSDSVII;FIDSDLNL;FVNSLDDL;IAYDSADV;KIRINL
NL;GIGDNSTI;FLFASSQS;ELVMNLPL
A0204
LLDFASFV;YAFNGLWV;ILSKEIKV;VMNLPLFI;ILLNISSL;VLFDRGIL;FAGNVYYI;QMFAYSPI
A0206
YAFNGLWV;LLDFASFV;WLLDFASF;FIDSDLNL;FQALKRYA;QMFAYSPI;FAGNVYYI;ILLNISSL
;WQMFAYSP;YIFDYLNT;LQYFFFSQ;SQSYWQMF;GISPDYFL;NTNEDFVV;KEIKVQEV;KEAALNF
I;KFIDFLFA;ILSKEIKV;YYIDLEAL;YSIFSAFA;FIDFLFAS;FVNSLDDL;SVSDSVII
A0211
LLDFASFV;YAFNGLWV;ILLNISSL;ILSKEIKV;VLFDRGIL;SLDDLHKK;GISPDYFL;SLPSKYGI
;FIDSDLNL;PMQDYINL;WLLDFASF;QMFAYSPI;YIFDYLNT;ELVMNLPL;ILRKYIDV;VMNLPLF
I;DLEDVSRL;ILNEIKDL;SVSDSVII;GIGDNSTI;NLGNEFRA;ALNFINRA;NTNEDFVV;YYIDLE
AL;IAYDSADV;FLFASSQS;GFVSTWEV;DLFKLRNI;FLKDSRDA;RLRDFFNF;NLPLFIAY;KFIDF
LFA;NISFITRL;IKDLKIWV;FAGNVYYI
A0212
LLDFASFV;YAFNGLWV;ILSKEIKV;ILRKYIDV;ILLNISSL;VLFDRGIL;PMQDYINL;FIDSDLNL
;YIFDYLNT;SLPSKYGI;SLDDLHKK;QMFAYSPI;ILNEIKDL;WLLDFASF;YYIDLEAL;GISPDYF
L;VMNLPLFI;FLKDSRDA;ELVMNLPL;GIGDNSTI;IAYDSADV;DLEDVSRL;FFSQFQAL;FLFASS
QS;RLRDFFNF;FENDLEDV;VAFKEYFL
A0216
LLDFASFV;ILSKEIKV;ILLNISSL;YAFNGLWV;ILRKYIDV;VLFDRGIL;GISPDYFL;PMQDYINL
;DLEDVSRL;ELVMNLPL;QMFAYSPI;SLPSKYGI;NLGNEFRA;GIGDNSTI;FIDSDLNL;ALNFINR
A;VMNLPLFI;YIFDYLNT;SLDDLHKK;GFVSTWEV;FAGNVYYI;WLLDFASF;FLFASSQS;ILNEIK
DL;IAYDSADV;VAFKEYFL;NISFITRL
A0219
LLDFASFV;ILLNISSL;YAFNGLWV;ILSKEIKV;FIDSDLNL;PMQDYINL;DLEDVSRL;VMNLPLFI
;WLLDFASF;VLFDRGIL;SLPSKYGI;GISPDYFL;SLDDLHKK;ILRKYIDV;IAYDSADV;ELVMNLP
L
A0301
KLRNILSK;RSFEKFKK;KLRFDASK;TLNNWIFR;FSQFQALK;FASFVAFK;NISSLPSK;RINLNLKR
;KVQEVLQY;ISFITRLY
```

A1101
RSFEKFKK;TLNNWIFR;FASFVAFK;NISSLPSK;FSQFQALK;AALNFINR;GILIRNEK;SLDDLHKK
;AYSWNVLK;KVQEVLQY;NSLDDLHK;AFNGLWVK;SQFQALKR;SFKDKFLK;AFKEYFLK;KLRNILS
K;WAHQKYFK;SAFAGNVY;RINLNLKR;ISFITRLY
A2301
QYFFFSQF;PYSIFSAF;KYIDVIKI;AYKFIDFL;AYSWNVLK;NTLNNWIF;VWAHQKYF;YYIDLEAL
;EVLQYFFF;AYAFNGLW;RYSDLKKL;DVIKIDHF;RLRDFFNF;IMKLAFDF;RYANDKGI;DFASFVA
F;GAYKFIDF;AYDSADVW;WLLDFASF;TLSKNISF;NFILNEIK;AFKEYFLK;SFKDKFLK;FAYSPI
DF
A2402
KYIDVIKI;PYSIFSAF;QYFFFSQF;YYIDLEAL;DFASFVAF;VWAHQKYF;AYAFNGLW;AYKFIDFL
;RYANDKGI;AYSWNVLK;EVLQYFFF;WWAKRIGI;LVMNLPLF
A2403
QYFFFSQF;YYIDLEAL;AYDSADVW;AYKFIDFL;AYAFNGLW;RSPPYSIF;KYIDVIKI;KFLKEAAL
;RYANDKGI;VWAHQKYF;PYSIFSAF;RYSDLKKL;EVLQYFFF;SYWQMFAY;FVAFKEYF;DFASFVA
F;FFSQFQAL
A2601 YIKNCIVY;DVIKIDHF;EVLQYFFF;DVWAHQKY;EVGVGEAY;NVLKNVKY
A2602
EVLQYFFF;DVIKIDHF;EVGVGEAY;VIIPMQDY;YIKNCIVY;DVWAHQKY;EVRSFEKF;FVAFKEYF
;EIKVQEVL;DVSRLRDF;EIKDLKIW;ELVMNLPL;KVQEVLQY;DFASFVAF;NLPLFIAY;YINLGNE
F;NTLNNWIF;FIDSDLNL;NVLKNVKY;SVDEVRSF;LVMNLPLF
A2902
SYWQMFAY;VIIPMQDY;AFAGNVYY;YIKNCIVY;KVQEVLQY;SAFAGNVY;LFASSQSY;EVGVGEAY
;NVLKNVKY;QYFFFSQF;NLPLFIAY
A3001
KLRFDASK;KLRNILSK;AFKEYFLK;RSFEKFKK;EFRANIPK;SFKDKFLK;ETRYSDLK;WAHQKYFK
;AFNGLWVK
A3002 RYSDLKKL;VIIPMQDY
A3101
TLNNWIFR;HQKYFKLR;VIKIDHFR;AFNVLFDR;AALNFINR;KYEWWAKR;SFKDKFLK;AFKEYFLK
;RSFEKFKK;RINLNLKR;KNISFITR;FFNFPGMR;KLRNILSK;FITRLYGR;AFNGLWVK;SQFQALK
R;LLKENETR;RASVDEVR;YSPIDFTR;WAHQKYFK;RLRDFFNF
A3301
EYFLKDSR;TLNNWIFR;DLKKLSFK;VIKIDHFR;HQKYFKLR;FFNFPGMR;EFRANIPK;FITRLYGR
;AFNVLFDR;KYEWWAKR;SFKDKFLK
A6801
FASFVAFK;FVVWDMIR;ETRYSDLK;TLNNWIFR;FITRLYGR;FSQFQALK;YSPIDFTR;EYFLKDSR
;NISSLPSK;WAHQKYFK;VIKIDHFR;FFNFPGMR;DLKKLSFK;RSFEKFKK;NVYYIDLE;NFILNEI
K;SQFQALKR;HQKYFKLR;ISFITRLY;RINLNLKR;RASVDEVR;AALNFINR;NSLDDLHK
A6802
EAYAFNGL;FSAFAGNV;NTNEDFVV;MSSVSDSV;NISFITRL;QMFAYSPI;YSIFSAFA;NRASVDEV
;NSTIREFV;NCIVYTGI;FAGNVYYI;SPAYSWNV;QSYWQMFA;TRSPPYSI;YAFNGLWV;SVSDSVI
I;DSRDAFNV
A6901
YAFNGLWV;EAYAFNGL;NTNEDFVV;FSAFAGNV;SPAYSWNV;QMFAYSPI;FAGNVYYI;QSYWQMFA
;ELVMNLPL
B0702 SPAYSWNV
B0801 WAKRIGIL
B0802
B1501
SAFAGNVY;SQSYWQMF;YINLGNEF;YIKNCIVY;WLLDFASF;IMKLAFDF;RLRDFFNF;NQNLPHNY
;ISFITRLY;KVQEVLQY;VQEVLQYF;YLNTNEDF;QMFAYSPI;TLSKNISF;QAWDSPAY;LFASSQS
Y;LVMNLPLF
B1801 QEVLQYFF;YEWWAKRI;WEVGVGEA
B2705 KRIGILRK;MRIMKLAF

B3501
QAWDSPAY;SAFAGNVY;EVGVGEAY;SYWQMFAY;YINLGNEF;DFASFVAF;EVLQYFFF;FFSQFQAL
;DVWAHQKY;WLLDFASF;LVMNLPLF;FAYSPIDF;LFASSQSY;FVAFKEYF;YIKNCIVY;FASSQSY
W;
B3901 SRDAFNVL;IREFVNSL
B4001 VEDFENDL;QEVLQYFF;NETRYSDL;RDFFNFIL;NEKDLFKL
B4002 QEVLQYFF;KEIKVQEV
B4402 QEVLQYFF
B4403 QEVLQYFF
B4501
B5101
B5301 FASSQSYW;FAGNVYYI
B5401 PPYSIFSA;LPLFIAYD
B5701
B5801 FASSQSYW;SSYWLLDF;FAYSPIDF

**Allele 9-mer**
A0101 FSAFAGNVY;YSDLKKLSF;SLDDLHKKY
A0201
TLNNWIFRL;WLLDFASFV;YLNTNEDFV;VLFDRGILI;FVSTWEVGV;FIDSDLNLL;AMSSVSDSV;F
IAYDSADV;LVMNLPLFI;TLSKNISFI;LLDFASFVA;FVAFKEYFL;RLLESDLDA;SQSYWQMFA;NI
LSKEIKV;VIIPMQDYI;FILNEIKDL;YIKNCIVYT;VVWDMIRSA;VMNLPLFIA;WQMFAYSPI
A0202
YLNTNEDFV;FIDSDLNLL;FVAFKEYFL;AMSSVSDSV;TLSKNISFI;FVSTWEVGV;MIRSAMSSV;W
LLDFASFV;TLNNWIFRL;KLSFKDKFL;FIAYDSADV;VLFDRGILI;GAYKFIDFL;VIIPMQDYI;FF
NFILNEI;ILRKYIDVI;WQMFAYSPI;LLDFASFVA;FFFSQFQAL;LVMNLPLFI;FLFASSQSY;TIR
EFVNSL;LLESDLDAT;FLKDSRDAF;FIDFLFASS;SSYWLLDFA;YANDKGIEL;FASSQSYWQ
A0203
MIRSAMSSV;AMSSVSDSV;YLNTNEDFV;TLNNWIFRL;FVSTWEVGV;WLLDFASFV;TLSKNISFI;F
IAYDSADV;TIREFVNSL;FIDSDLNLL;WQMFAYSPI;FVAFKEYFL;VLFDRGILI;ILRKYIDVI;FT
RSPPYSI;SQSYWQMFA;VMNLPLFIA;LVMNLPLFI;YIKNCIVYT;VVWDMIRSA;FFNFILNEI;VII
PMQDYI;GILLNISSL;KIDHFRGFV;KLSFKDKFL;LLDFASFVA;RLRDFFNFP;SSYWLLDFA;ASKD
KVAGI;NLKRKSGIL
A0204
WLLDFASFV;TLNNWIFRL;FVSTWEVGV;YLNTNEDFV;VLFDRGILI;AMSSVSDSV;LVMNLPLFI;F
TRSPPYSI;FVAFKEYFL;VMNLPLFIA;YANDKGIEL
A0206
WLLDFASFV;WQMFAYSPI;FIDSDLNLL;SQSYWQMFA;FVSTWEVGV;AMSSVSDSV;FIAYDSADV;M
IRSAMSSV;TLNNWIFRL;YLNTNEDFV;VIIPMQDYI;FVAFKEYFL;VLFDRGILI;LQYFFFSQF;LL
DFASFVA;VVWDMIRSA;KIDHFRGFV;RGFVSTWEV;LVMNLPLFI;RLLESDLDA;FVVWDMIRS;KVQ
EVLQYF;YKFIDFLFA;GEAYAFNGL;FFFSQFQAL;AALNFINRA;SSYWLLDFA;FAYSPIDFT;GILL
NISSL;YIFDYLNTN;FIDFLFASS;GAYKFIDFL;
A0211
WLLDFASFV;YLNTNEDFV;VLFDRGILI;AMSSVSDSV;TLNNWIFRL;KIDHFRGFV;EIKDLKIWV;L
LDFASFVA;SLDDLHKKY;FIDSDLNLL;FVSTWEVGV;TLSKNISFI;VVWDMIRSA;FLFASSQSY;NI
LSKEIKV;YANDKGIEL;RLLESDLDA;MIRSAMSSV;YIFDYLNTN;FVAFKEYFL;VIIPMQDYI;TIR
EFVNSL;VMNLPLFIA;DLFKLRNIL;GILRKYIDV;YSWNVLKNV;KLSFKDKFL;FIAYDSADV;VLQY
FFFSQ;RGFVSTWEV;GILLNISSL;ILRKYIDVI;PLFIAYDSA;ANDKGIELV;AFAGNVYYI;YAFNG
LWVK;GAYKFIDFL;LVMNLPLFI
A0212
WLLDFASFV;YLNTNEDFV;VLFDRGILI;AMSSVSDSV;FVSTWEVGV;TLNNWIFRL;FLFASSQSY;M
IRSAMSSV;FIDSDLNLL;EIKDLKIWV;LLDFASFVA;VVWDMIRSA;GILRKYIDV;VLQYFFFSQ;SL
DDLHKKY;YIFDYLNTN;RLLESDLDA;NILSKEIKV;ILIRNEKDL;FVAFKEYFL;ILRKYIDVI;FIA
YDSADV;VIIPMQDYI;FFFSQFQAL;TLSKNISFI;TIREFVNSL;FILNEIKDL;KIDHFRGFV;WVED
FENDL;YANDKGIEL;VMNLPLFIA;GILLNISSL;YSWNVLKNV;LLKENETRY;DLFKLRNIL;FLKDS
RDAF

A0216
WLLDFASFV;YLNTNEDFV;AMSSVSDSV;TLNNWIFRL;EIKDLKIWV;TLSKNISFI;VLFDRGILI;K
IDHFRGFV;MIRSAMSSV;NILSKEIKV;FVSTWEVGV;FVAFKEYFL;DLFKLRNIL;GAYKFIDFL;FI
DSDLNLL;LLDFASFVA;VIIPMQDYI;ILRKYIDVI;GILRKYIDV;TIREFVNSL;KLSFKDKFL;GIL
LNISSL;PLFIAYDSA;YANDKGIEL;VVWDMIRSA;YSWNVLKNV;FLFASSQSY;
A0219
WLLDFASFV;YLNTNEDFV;AMSSVSDSV;TLNNWIFRL;FIDSDLNLL;FVSTWEVGV;TLSKNISFI;M
IRSAMSSV;LLDFASFVA;VLFDRGILI;VVWDMIRSA;FLFASSQSY;NILSKEIKV;YANDKGIEL;FV
AFKEYFL;YSWNVLKNV
A0301
RSFEKFKKK;LSFKDKFLK;VAFKEYFLK;FLFASSQSY;RINLNLKRK;KIRINLNLK;QSYWQMFAY;I
LNEIKDLK;KVAGISPDY;PAYSWNVLK;ALKRYANDK
A1101
VAFKEYFLK;LSFKDKFLK;SADVWAHQK;RSFEKFKKK;QSYWQMFAY;NTLNNWIFR;PAYSWNVLK;S
AFAGNVYY;YAFNGLWVK;RINLNLKRK;NSLDDLHKK;ILNEIKDLK;KIRINLNLK;SVIIPMQDY;NV
KYEWWAK;ASFVAFKEY;KVAGISPDY;
A2301
AYKFIDFLF;DYLNTNEDF;DYINLGNEF;KYKKIRINL;YWLLDFASF;SFVAFKEYF;MFAYSPIDF;V
VYYIDLEAL;VWAHQKYFK;KYGIGDLGK;KFIDSDLNL;RIMKLAFDF;SRLRDFFNF;AYSPIDFTR;FL
KEAALNF;KVQEVLQYF;NYIKNCIVY;AFAGNVYYI;LQYFFFSQF;ELVMNLPLF;DLGKGAYKF;FFN
FILNEI;EFVNSLDDL
A2402
AYKFIDFLF;DYINLGNEF;YWLLDFASF;AFAGNVYYI;DYLNTNEDF;AYAFNGLWV;MFAYSPIDF;K
YEWWAKRI;VYYIDLEAL;KYKKIRINL;WWAKRIGIL
A2403
YWLLDFASF;AYKFIDFLF;VYYIDLEAL;YFLEQEQAW;KVQEVLQYF;DYINLGNEF;DYLNTNEDF;S
FVAFKEYF;KYKKIRINL;RIMKLAFDF;KFKKKSSYW;FFFSQFQAL;AFAGNVYYI;MFAYSPIDF;AT
LSKNISF;KYEWWAKRI;AYAFNGLWV;FFNFILNEI;AWDSPAYSW
A2601  ELVMNLPLF;SVIIPMQDY
A2602
SVIIPMQDY;DVWAHQKYF;ELVMNLPLF;DVSRLRDFF;QSYWQMFAY;KVQEVLQYF;FIDSDLNLL;F
VAFKEYFL;KVAGISPDY;EFVNSLDDL;NISFITRLY;NISSLPSKY
A2902
SAFAGNVYY;NYIKNCIVY;FLFASSQSY;QSYWQMFAY;SVIIPMQDY;MNLPLFIAY;AYKFIDFLF;F
SAFAGNVY;DYINLGNEF;NISSLPSKY
A3001
KIRINLNLK;RSFEKFKKK;EVRSFEKFK;RGILIRNEK;ALKRYANDK;ETRYSDLKK;RLRDFFNFP;R
INLNLKRK;NVKYEWWAK;LSFKDKFLK;VAFKEYFLK;SDLDATLSK;AKRIGILRK;SWNVLKNVK;RN
ILSKEIK;RGFVSTWEV
A3002
KVAGISPDY;NISSLPSKY;DVSRLRDFF;KSSYWLLDF;AYKFIDFLF;KYKKIRINL;VQEVLQYFF
A3101
SFITRLYGR;AYSPIDFTR;NTLNNWIFR;YINLGNEFR;WAKRIGILR;RSFEKFKKK;DVIKIDHFR;K
YKKIRINL;GLWVKSPGR;LSFKDKFLK;DFFNFPGMR;KIRINLNLK;VWAHQKYFK;DAFNVLFDR;KE
YFLKDSR;VAFKEYFLK;YAFNGLWVK;SYWQMFAYS
A3301
DVIKIDHFR;DFFNFPGMR;DAFNVLFDR;DFVVWDMIR;DFASFVAFK;SFITRLYGR;YINLGNEFR;N
TLNNWIFR;DLEDVSRLR;EVRSFEKFK;NVKYEWWAK;AYSPIDFTR;EAALNFINR;FFSQFQALK
A6801
DVIKIDHFR;DAFNVLFDR;DFASFVAFK;EAALNFINR;YINLGNEFR;NTLNNWIFR;LSFKDKFLK;Y
AFNGLWVK;EVRSFEKFK;ETRYSDLKK;DFFNFPGMR;VAFKEYFLK;FSQFQALKR;QSYWQMFAY;FF
SQFQALK;NISFITRLY;NVKYEWWAK;SAFAGNVYY;DFVVWDMIR;DLEDVSRLR;ILNEIKDLK;WAK
RIGILR;PAYSWNVLK;AYSPIDFTR;SFITRLYGR;FASFVAFKE;SADVWAHQK;NFPGMRIMK;NSLD
DLHKK;EAYAFNGLW;FNFILNEIK
A6802
FVAFKEYFL;FTRSPPYSI;DSPAYSWNV;FIAYDSADV;WQMFAYSPI;NVYYIDLEA;MIRSAMSSV;F

VSTWEVGV;DNSTIREFV;EVLQYFFFS;WLLDFASFV;LVMNLPLFI;RGFVSTWEV;TLSKNISFI;TL
NNWIFRL;EDFVVWDMI;MSSVSDSVI;HKKYIFDYL;EIKDLKIWV;TGIGDNSTI
A6901
FVSTWEVGV;YSWNVLKNV;WLLDFASFV;LVMNLPLFI;FIAYDSADV;TLNNWIFRL;FTRSPPYSI;D
SPAYSWNV;FIDSDLNLL;EVLQYFFFS;QAWDSPAYS;NILSKEIKV;FAYSPIDFT;EVGVGEAYA;EI
KDLKIWV;
B0702
SPAYSWNVL;FPGMRIMKL;PPYSIFSAF;MIRSAMSSV;SPPYSIFSA;IPMQDYINL;RANIPKNTL;T
IREFVNSL
B0801 NLKRKSGIL
B0802
B1501
FLFASSQSY;EQAWDSPAY;FSAFAGNVY;SAFAGNVYY;FLKEAALNF;FLKDSRDAF;SQFQALKRY;G
MRIMKLAF;KVAGISPDY;WVKSPGRDF;LQYFFFSQF;QSYWQMFAY;HQKYFKLRF;KVQEVLQYF;WQ
MFAYSPI;SVIIPMQDY;ASVDEVRSF;ASFVAFKEY;LLKENETRY;WEVGVGEAY
B1801
WEVGVGEAY;IELVMNLPL;MNLPLFIAY;QEVLQYFFF;IKVQEVLQY;DEVRSFEKF;LESDLDATL;Q
SYWQMFAY
B2705 KRIGILRKY;IRINLNLKR
B3501
EQAWDSPAY;FSAFAGNVY;PPYSIFSAF;QSYWQMFAY;SPAYSWNVL;FFFSQFQAL;WEVGVGEAY;M
NLPLFIAY;SAFAGNVYY;YANDKGIEL;YFLEQEQAW;FLFASSQSY;IAYDSADVW;IPMQDYINL;LD
FASFVAF;FLKDSRDAF;VGVGEAYAF;SPGRDFFNF
B3901 GRDFFNFIL
B4001 GEAYAFNGL;IELVMNLPL;KEIKVQEVL;QEVLQYFFF;LESDLDATL
B4002 QEVLQYFFF;KEIKVQEVL
B4402 QEVLQYFFF
B4403 QEVLQYFFF
B4501
B5101 IPMQDYINL
B5301 SPGRDFFNF;IPKNTLNNW;FPGMRIMKL;PPYSIFSAF;IPMQDYINL
B5401 LPLFIAYDS;SPPYSIFSA
B5701 NTNEDFVVW;IAYDSADVW
B5801
IAYDSADVW;KSSYWLLDF;LFASSQSYW;EAYAFNGLW;NTNEDFVVW;QSYWQMFAY;RIMKLAFDF;F
SAFAGNVY;KVAGISPDY;ASSQSYWQM;VAGISPDYF

**Allele 10-mer**
A0101 SADVWAHQKY;FSAFAGNVYY
A0201
YLNTNEDFVV;FLKEAALNFI;WLLDFASFVA;FINRASVDEV;TLNNWIFRLL;ALNFINRASV;SIFSA
FAGNV;YANDKGIELV;ALDKFIDSDL;SAMSSVSDSV;ILNEIKDLKI;SAFAGNVYYI;LVMNLPLFIA
;FVVWDMIRSA;AMSSVSDSVI;SLDDLHKKYI;RLLESDLDAT;LLESDLDATL;SVSDSVIIPM;ATLS
KNISFI
A0202
FLKEAALNFI;WLLDFASFVA;YLNTNEDFVV;SAFAGNVYYI;LLESDLDATL;AMSSVSDSVI;LVMNL
PLFIA;FLFASSQSYW;TLNNWIFRLL;FAGNVYYIDL;SAMSSVSDSV;ALDKFIDSDL;NVYYIDLEAL
;SLDDLHKKYI;SVSDSVIIPM;WIFRLLESDL;ALNFINRASV;NLGNEFRANI;FINRASVDEV;ILNE
IKDLKI;RLLESDLDAT;SIFSAFAGNV;YANDKGIELV;SVIIPMQDYI;ILLNISSLPS;ATLSKNISF
I;FASSQSYWQM;RLRDFFNFPG;NTLNNWIFRL
A0203
FLKEAALNFI;ALNFINRASV;FINRASVDEV;YLNTNEDFVV;FVVWDMIRSA;TLNNWIFRLL;SIFSA
FAGNV;YANDKGIELV;KLRNILSKEI;SAMSSVSDSV;AMSSVSDSVI;DMIRSAMSSV;ILNEIKDLKI
;NLNLKRKSGI;STIREFVNSL;NLGNEFRANI;YINLGNEFRA;WLLDFASFVA;NLKRKSGILL;NVYY
IDLEAL;LLESDLDATL;LVMNLPLFIA;SLDDLHKKYI;SAFAGNVYYI

A0204

YLNTNEDFVV;FINRASVDEV;FLKEAALNFI;STWEVGVGEA;ALNFINRASV;SIFSAFAGNV;YANDK
GIELV;TLNNWIFRLL;LVMNLPLFIA;SAFAGNVYYI

A0206

WLLDFASFVA;FVVWDMIRSA;FINRASVDEV;FLKEAALNFI;SAMSSVSDSV;YLNTNEDFVV;SIFSA
FAGNV;YANDKGIELV;SQFQALKRYA;SAFAGNVYYI;STIREFVNSL;RLLESDLDAT;YWLLDFASFV
;NTLNNWIFRL;YINLGNEFRA;LLDFASFVAF;NVYYIDLEAL;SQSYWQMFAY;STWEVGVGEA;LVMN
LPLFIA;FASSQSYWQM;ATLSKNISFI;TLNNWIFRLL;KSSYWLLDFA;YIFDYLNTNE;SVIIPMQDY
I;ALNFINRASV;YTGIGDNSTI;RSPPYSIFSA

A0211

YLNTNEDFVV;SLDDLHKKYI;WLLDFASFVA;ALNFINRASV;YANDKGIELV;TLNNWIFRLL;FINRA
SVDEV;FLKEAALNFI;SIFSAFAGNV;ALDKFIDSDL;DMIRSAMSSV;LLESDLDATL;ILNEIKDLKI
;ELVMNLPLFI;YINLGNEFRA;SAFAGNVYYI;SVSDSVIIPM;VVWDMIRSAM;DLGKGAYKFI;EAYA
FNGLWV;KLRNILSKEI;DLDATLSKNI;RLLESDLDAT;STWEVGVGEA;AMSSVSDSVI;FVVWDMIRS
A;NLGNEFRANI;GISPDYFLEQ;LLDFASFVAF;VLFDRGILIR;TLSKNISFIT;NTLNNWIFRL;NLK
RKSGILL;FLFASSQSYW;YFFFSQFQAL;FIDFLFASSQ;SAMSSVSDSV;YWLLDFASFV;WIFRLLES
DL;SVDEVRSFEK;IIPMQDYINL;YIFDYLNTNE

A0212

YLNTNEDFVV;SLDDLHKKYI;WLLDFASFVA;ALNFINRASV;ALDKFIDSDL;FLKEAALNFI;YANDK
GIELV;FINRASVDEV;LLESDLDATL;SIFSAFAGNV;ILNEIKDLKI;YINLGNEFRA;VVWDMIRSAM
;DMIRSAMSSV;TLNNWIFRLL;WIFRLLESDL;VLFDRGILIR;YIFDYLNTNE;RLLESDLDAT;YFFF
SQFQAL;ELVMNLPLFI;AMSSVSDSVI;FLFASSQSYW;SAFAGNVYYI

A0216

YLNTNEDFVV;SLDDLHKKYI;ALNFINRASV;DMIRSAMSSV;WLLDFASFVA;FINRASVDEV;FLKEA
ALNFI;SIFSAFAGNV;LLESDLDATL;ALDKFIDSDL;YANDKGIELV;DLGKGAYKFI;TLNNWIFRLL
;ELVMNLPLFI;ILNEIKDLKI;EAYAFNGLWV;SAFAGNVYYI;YINLGNEFRA;NLKRKSGILL;TLSK
NISFIT;AMSSVSDSVI;SAMSSVSDSV;KLRNILSKEI;NLGNEFRANI;FRGFVSTWEV;WIFRLLESD
L

A0219

YLNTNEDFVV;WLLDFASFVA;YANDKGIELV;FINRASVDEV;LLESDLDATL;SLDDLHKKYI;DMIRS
AMSSV;FLKEAALNFI;TLNNWIFRLL;ALNFINRASV;SAMSSVSDSV;ALDKFIDSDL;DLDATLSKNI
;ELVMNLPLFI

A0301

KLSFKDKFLK;FVAFKEYFLK;LLNISSLPSK;AYAFNGLWVK;LIRNEKDLFK;KLRFDASKDK;YSDLK
KLSFK;ILRKYIDVIK;FILNEIKDLK;VMNLPLFIAY;DVWAHQKYFK;FFFSQFQALK;SVDEVRSFEK
;LFKLRNILSK;FSAFAGNVYY;SPAYSWNVLK;VLFDRGILIR;LDFASFVAFK;SQSYWQMFAY;FIDS
DLNLLK

A1101

SVDEVRSFEK;FVAFKEYFLK;KLSFKDKFLK;LLNISSLPSK;ISFITRLYGR;SQSYWQMFAY;FVNSL
DDLHK;YSDLKKLSFK;FIDSDLNLLK;DVWAHQKYFK;VMNLPLFIAY;SPAYSWNVLK;NQNLPHNYIK
;DSADVWAHQK;VLFDRGILIR;AYAFNGLWVK;YSWNVLKNVK;LDFASFVAFK;FFFSQFQALK

A2301

SYWLLDFASF;KFLKEAALNF;AYAFNGLWVK;RYSDLKKLSF;YFLKDSRDAF;YYIDLEALDK;GAYKF
IDFLF;RYANDKGIEL;VSRLRDFFNF;VLQYFFFSQF;VWAHQKYFKL;LWVKSPGRDF;KVQEVLQYFF
;KFIDSDLNLL;DFFNFILNEI;SFVAFKEYFL;ILIRNEKDLF;DYFLEQEQAW;KFKKKSSYWL;IWVE
DFENDL;YFFFSQFQAL;NFILNEIKDL;FFFSQFQALK;KSPGRDFFNF

A2402

SYWLLDFASF;RYSDLKKLSF;YWQMFAYSPI;KFLKEAALNF;VWAHQKYFKL;YFLKDSRDAF;AYSWN
VLKNV;RYANDKGIEL;VQEVLQYFFF;KFIDSDLNLL

A2403

YFLKDSRDAF;KFLKEAALNF;SYWLLDFASF;RYANDKGIEL;RYSDLKKLSF;YFFFSQFQAL;KVQEV
LQYFF;KFKKKSSYWL;FTRSPPYSIF;AYSWNVLKNV;YWQMFAYSPI;AHQKYFKLRF

A2601  EIKVQEVLQY;SVSDSVIIPM;STIREFVNSL

A2602

EIKVQEVLQY;FTRSPPYSIF;EVGVGEAYAF;STIREFVNSL;ETRYSDLKKL;SVSDSVIIPM;YIDLE

ALDKF;WVKSPGRDFF;KVQEVLQYFF;YIDVIKIDHF;KVAGISPDYF;PIDFTRSPPY;SVIIPMQDYI
;VVWDMIRSAM;VIKIDHFRGF;DLHKKYIFDY;DSVIIPMQDY
A2902
VMNLPLFIAY;SWNVLKNVKY;FSAFAGNVYY;SQSYWQMFAY;IFSAFAGNVY;FASFVAFKEY;SYWLL
DFASF;HNYIKNCIVY
A3001
KLRFDASKDK;LLNISSLPSK;ILRKYIDVIK;LFKLRNILSK;EVRSFEKFKK;LIRNEKDLFK;KLSFK
DKFLK;RLRDFFNFPG;KIRINLNLKR;YFKLRFDASK;SVDEVRSFEK;KNVKYEWWAK;WAKRIGILRK
A3002 RYSDLKKLSF;SQSYWQMFAY;KVQEVLQYFF;SWNVLKNVKY
A3101
KIRINLNLKR;ISFITRLYGR;LSKNISFITR;FAYSPIDFTR;KLSFKDKFLK;DYINLGNEFR;FFSQF
QALKR;KNTLNNWIFR;WWAKRIGILR;RDAFNVLFDR;FFFSQFQALK;FVAFKEYFLK;AYAFNGLWVK
;KYKKIRINLN
A3301
DYINLGNEFR;FFSQFQALKR;FAYSPIDFTR;LSKNISFITR;FFFSQFQALK;ISFITRLYGR;DFASF
VAFKE;VLFDRGILIR
A6801
FAYSPIDFTR;FVAFKEYFLK;DSADVWAHQK;ISFITRLYGR;EVRSFEKFKK;NVKYEWWAKR;DVWAH
QKYFK;DYINLGNEFR;FVNSLDDLHK;FFFSQFQALK;FFSQFQALKR;SVDEVRSFEK;LSKNISFITR
;SPAYSWNVLK;FSAFAGNVYY;EDFVVWDMIR;FFNFILNEIK;WAHQKYFKLR;FASFVAFKEY;LDFA
SFVAFK;TGIGDNSTIR;FILNEIKDLK;FNFPGMRIMK;ESDLDATLSK;EVLQYFFFSQ;YYIDLEALD
K;VLFDRGILIR
A6802
EAALNFINRA;SAFAGNVYYI;NVYYIDLEAL;SIFSAFAGNV;STIREFVNSL;EAYAFNGLWV;SAMSS
VSDSV;SVIIPMQDYI;NTLNNWIFRL;FINRASVDEV;DMIRSAMSSV;SVSDSVIIPM;FVVWDMIRSA
;FRGFVSTWEV;YANDKGIELV;LVMNLPLFIA;STWEVGVGEA;WDSPAYSWNV;SGILLNISSL
A6901
EAYAFNGLWV;STWEVGVGEA;NTLNNWIFRL;YANDKGIELV;SIFSAFAGNV;SAFAGNVYYI;FINRA
SVDEV;ELVMNLPLFI;SVSDSVIIPM;NVLFDRGILI;WLLDFASFVA;DMIRSAMSSV;SAMSSVSDSV
;LVMNLPLFIA;FVVWDMIRSA;NVYYIDLEAL;FASSQSYWQM;EVLQYFFFSQ
B0702 SPPYSIFSAF;LPSKYGIGDL;VVWDMIRSAM;SVSDSVIIPM
B0801
B0802
B1501
VMNLPLFIAY;SQSYWQMFAY;QMFAYSPIDF;FSAFAGNVYY;IFSAFAGNVY;QEQAWDSPAY;WVKSP
GRDFF;KVAGISPDYF;FLFASSQSYW;FTRSPPYSIF
B1801
IELVMNLPLF;YEWWAKRIGI;WEVGVGEAYA;IKVQEVLQYF;NEIKDLKIWV;QEQAWDSPAY;
B2705 KRIGILRKYI
B3501
FASFVAFKEY;SPPYSIFSAF;FASSQSYWQM;VVWDMIRSAM;FSAFAGNVYY;EVGVGEAYAF;LLDFA
SFVAF;SVSDSVIIPM;IFSAFAGNVY;DFLFASSQSY;YFLKDSRDAF;YFFFSQFQAL;SADVWAHQKY
;NVYYIDLEAL;VMNLPLFIAY;QAWDSPAYSW;QEQAWDSPAY;SYWLLDFASF;FIAYDSADVW
B3901 FRANIPKNTL
B4001 REFVNSLDDL;YEWWAKRIGI;KENETRYSDL;NEIKDLKIWV;IELVMNLPLF
B4002 REFVNSLDDL;KENETRYSDL;QEVLQYFFFS
B4402 GEAYAFNGLW
B4403 QEVLQYFFFS;GEAYAFNGLW
B4501
B5101 LPLFIAYDSA;IPKNTLNNWI;LPHNYIKNCI;
B5301 SPGRDFFNFI;SPPYSIFSAF;LPHNYIKNCI;IPKNTLNNWI;LPSKYGIGDL
B5401 LPLFIAYDSA;FPGMRIMKLA;LPHNYIKNCI;PPYSIFSAFA;IPKNTLNNWI
B5701
B5801
QAWDSPAYSW;FLFASSQSYW;KVAGISPDYF;RASVDEVRSF;GAYKFIDFLF;FIAYDSADVW;ASSQS
YWQMF;ASFVAFKEYF;FSAFAGNVYY;KSPGRDFFNF;FASSQSYWQM

**Allele 11-mer**

A0101 DSDNQNLPHNY;SSQSYWQMFAY;DSADVWAHQKY

A0201
RLLESDLDATL;FLKDSRDAFNV;QMFAYSPIDFT;FILNEIKDLKI;AMSSVSDSVII;ILSKEIKVQEV
;FLFASSQSYWQ;KVAGISPDYFL;FIAYDSADVWA;RLRDFFNFPGM;YIKNCIVYTGI;VIIPMQDYIN
L;ILRKYIDVIKI;KIWVEDFENDL

A0202
FLKDSRDAFNV;FLFASSQSYWQ;ILSKEIKVQEV;AMSSVSDSVII;FIAYDSADVWA;KVAGISPDYFL
;FSAFAGNVYYI;GVGEAYAFNGL;YIKNCIVYTGI;FDYLNTNEDFV;VIIPMQDYINL;RLLESDLDAT
L;RLRDFFNFPGM;KLAFDFDSDNQ;KLRFDASKDKV;NISSLPSKYGI;QMFAYSPIDFT;ILRKYIDVI
KI;NLPLFIAYDSA;FENDLEDVSRL;SLPSKYGIGDL;LQYFFFSQFQA;FVVWDMIRSAM;YLNTNEDF
VVW

A0203
FLKDSRDAFNV;YIKNCIVYTGI;ILSKEIKVQEV;RLRDFFNFPGM;AMSSVSDSVII;ILRKYIDVIKI
;FIAYDSADVWA;SLPSKYGIGDL;VIKIDHFRGFV;NLPLFIAYDSA;KLRFDASKDKV;ALKRYANDKG
I;GVGEAYAFNGL;VLKNVKYEWWA;RLLESDLDATL;FLFASSQSYWQ;AALNFINRASV;VIIPMQDYI
NL;LQYFFFSQFQA;FVVWDMIRSAM;NISSLPSKYGI;NLNLKRKSGIL;PLFIAYDSADV

A0204 ILSKEIKVQEV;RLLESDLDATL;FLKDSRDAFNV;KVAGISPDYFL

A0206
FLKDSRDAFNV;WLLDFASFVAF;RLLESDLDATL;LQYFFFSQFQA;FVVWDMIRSAM;AALNFINRASV
;FIAYDSADVWA;MQDYINLGNEF;KVAGISPDYFL;GVGEAYAFNGL;SQSYWQMFAYS;FILNEIKDLK
I;RLRDFFNFPGM;YIKNCIVYTGI;VIIPMQDYINL;WQMFAYSPIDF;ILSKEIKVQEV;KVQEVLQYF
FF;GEAYAFNGLWV;FLFASSQSYWQ;FSAFAGNVYYI;RSAMSSVSDSV;AMSSVSDSVII

A0211
FLKDSRDAFNV;RLLESDLDATL;ILSKEIKVQEV;FLFASSQSYWQ;VLKNVKYEWWA;SLDDLHKKYIF
;WLLDFASFVAF;SYWLLDFASFV;KVAGISPDYFL;PLFIAYDSADV;AMSSVSDSVII;RLRDFFNFPG
M;AALNFINRASV;YIDLEALDKFI;KLRFDASKDKV;VIIPMQDYINL;NLPLFIAYDSA;ELVMNLPLF
IA;ILRKYIDVIKI;SLPSKYGIGDL;NLPHNYIKNCI;VLFDRGILIRN;LLDFASFVAFK;VIKIDHFR
GFV;VLQYFFFSQFQ;QMFAYSPIDFT;DLHKKYIFDYL;KIDHFRGFVST;HFRGFVSTWEV;RIGILRK
YIDV;DVWAHQKYFKL;AWDSPAYSWNV;YIKNCIVYTGI;RYANDKGIELV;TLNNWIFRLLE;FILNEI
KDLKI;KIWVEDFENDL;IGDNSTIREFV;FIAYDSADVWA;SVSDSVIIPMQ;ILLNISSLPSK;YIFDY
LNTNED

A0212
FLKDSRDAFNV;RLLESDLDATL;ILSKEIKVQEV;FLFASSQSYWQ;VLKNVKYEWWA;RLRDFFNFPGM
;PLFIAYDSADV;WLLDFASFVAF;VIIPMQDYINL;AMSSVSDSVII;VLQYFFFSQFQ;SLDDLHKKYI
F;KLRFDASKDKV;FILNEIKDLKI;AALNFINRASV;ILRKYIDVIKI;SYWLLDFASFV;SLPSKYGIG
DL;VLFDRGILIRN;QMFAYSPIDFT;KVAGISPDYFL;KIWVEDFENDL;ALKRYANDKGI;YIDLEALD
KFI;NLPHNYIKNCI

A0216
FLKDSRDAFNV;ILSKEIKVQEV;RLLESDLDATL;VLKNVKYEWWA;FLFASSQSYWQ;SYWLLDFASFV
;DLHKKYIFDYL;AALNFINRASV;KVAGISPDYFL;PLFIAYDSADV;KLRFDASKDKV;ILRKYIDVIK
I;AMSSVSDSVII;VIKIDHFRGFV;NLPLFIAYDSA;QMFAYSPIDFT;VIIPMQDYINL;NLPHNYIKN
CI;DVWAHQKYFKL;HFRGFVSTWEV;RLRDFFNFPGM;SLPSKYGIGDL;RIGILRKYIDV;VLQYFFFS
QFQ;ELVMNLPLFIA;GVGEAYAFNGL

A0219
FLKDSRDAFNV;ILSKEIKVQEV;FLFASSQSYWQ;RLLESDLDATL;AMSSVSDSVII;KVAGISPDYFL
;WLLDFASFVAF

A0301
ILLNISSLPSK;LLDFASFVAFK;KLRNILSKEIK;ILIRNEKDLFK;YFFFSQFQALK;FFNFPGMRIMK
;KIRINLNLKRK;DLFKLRNILSK;LVMNLPLFIAY;TLSKNISFITR;KYFKLRFDASK;LLNISSLPSK
Y;GILRKYIDVIK;FVNSLDDLHKK;GIGDLGKGAYK

A1101
ILLNISSLPSK;SSQSYWQMFAY;ASVDEVRSFEK;LVMNLPLFIAY;SFVAFKEYFLK;FVNSLDDLHKK
;KFIDSDLNLLK;LLDFASFVAFK;EAYAFNGLWVK;SIFSAFAGNVY;ILIRNEKDLFK;YFFFSQFQAL

K;GILRKYIDVIK;GIGDLGKGAYK;KYFKLRFDASK;RYSDLKKLSFK;TLSKNISFITR;DSPAYSWNV
LK;AYSWNVLKNVK
A2301
KYIDVIKIDHF;YYIDLEALDKF;EYFLKDSRDAF;KYFKLRFDASK;VYYIDLEALDK;KYKKIRINLNL
;AYSWNVLKNVK;LWVKSPGRDFF;RYSDLKKLSFK;IFDYLNTNEDF;KVQEVLQYFFF;QYFFFSQFQA
L;SFVAFKEYFLK;EFVNSLDDLHK;KFIDSDLNLLK;VYTGIGDNSTI;WQMFAYSPIDF;YFFFSQFQA
LK;DFTRSPPYSIF;EVLQYFFFSQF;DYLNTNEDFVV;NFILNEIKDLK;WLLDFASFVAF;KFLKEAAL
NFI;SYWQMFAYSPI;FFNFPGMRIMK;DFFNFILNEIK;WWAKRIGILRK;DVIKIDHFRGF;KFKKKSS
YWLL;DVSRLRDFFNF;DFLFASSQSYW;NWIFRLLESDL
A2402
YYIDLEALDKF;KYIDVIKIDHF;SYWQMFAYSPI;VYTGIGDNSTI;QYFFFSQFQAL;KFLKEAALNFI
;KFKKKSSYWLL;LWVKSPGRDFF;KYKKIRINLNL;DFTRSPPYSIF;KVQEVLQYFFF;EYFLKDSRDA
F;DYLNTNEDFVV;KYEWWAKRIGI
A2403
YYIDLEALDKF;KYIDVIKIDHF;KVQEVLQYFFF;RSPPYSIFSAF;QYFFFSQFQAL;KFLKEAALNFI
;KYKKIRINLNL;KYEWWAKRIGI;KFKKKSSYWLL;EYFLKDSRDAF;AFAGNVYYIDL;SYWQMFAYSP
I;IFDYLNTNEDF
A2601
DVIKIDHFRGF;STWEVGVGEAY;DSADVWAHQKY;YGIGDLGKGAY;SIFSAFAGNVY;EVLQYFFFSQF
;FVVWDMIRSAM;YSWNVLKNVKY
A2602
EIKVQEVLQYF;DVIKIDHFRGF;EVLQYFFFSQF;FVVWDMIRSAM;YGIGDLGKGAY;KVQEVLQYFFF
;DVSRLRDFFNF;SIFSAFAGNVY;LVMNLPLFIAY;DVWAHQKYFKL;VIIPMQDYINL;NTNEDFVVWD
M;STWEVGVGEAY;SSQSYWQMFAY;DFTRSPPYSIF;SVDEVRSFEKF;DFFNFPGMRIM;DFASFVAFK
EY;KVAGISPDYFL;DSADVWAHQKY
A2902
LVMNLPLFIAY;IFSAFAGNVYY;YSWNVLKNVKY;YYIDLEALDKF;FFSQFQALKRY;SIFSAFAGNVY
;STWEVGVGEAY;SSQSYWQMFAY
A3001
KLRNILSKEIK;KYFKLRFDASK;KIRINLNLKRK;LGNEFRANIPK;KFIDSDLNLLK;YFFFSQFQALK
;RYSDLKKLSFK;EVRSFEKFKKK;SFVAFKEYFLK;RLRDFFNFPGM;KENETRYSDLK;ASVDEVRSFE
K;ILLNISSLPSK;LLDFASFVAFK;KKLSFKDKFLK;ILIRNEKDLFK
A3002
RYSDLKKLSFK;KYIDVIKIDHF;DVSRLRDFFNF;KVQEVLQYFFF;IFSAFAGNVYY;KYKKIRINLNL
A3101
KYFKLRFDASK;MFAYSPIDFTR;AFKEYFLKDSR;RYSDLKKLSFK;YFFFSQFQALK;FFFSQFQALKR
;TLSKNISFITR;SFVAFKEYFLK;KNVKYEWWAKR;YIDVIKIDHFR;NISFITRLYGR;KLRNILSKEI
K;EWWAKRIGILR;VWAHQKYFKLR;KFIDSDLNLLK;KFKKKSSYWLL;KYKKIRINLNL;KVQEVLQYF
FF;YTGIGDNSTIR
A3301
MFAYSPIDFTR;YFFFSQFQALK;EWWAKRIGILR;DLNLLKENETR;NISFITRLYGR;DFENDLEDVSR
;YIDVIKIDHFR;DFFNFILNEIK;AFKEYFLKDSR;FFFSQFQALKR;NVLFDRGILIR;EAYAFNGLWV
K;DLFKLRNILSK
A6801
NISFITRLYGR;EAYAFNGLWVK;YFFFSQFQALK;MFAYSPIDFTR;YIDVIKIDHFR;FINRASVDEVR
;DSPAYSWNVLK;FVNSLDDLHKK;TLSKNISFITR;FFFSQFQALKR;SFVAFKEYFLK;NVLFDRGILI
R;DFFNFILNEIK;DLFKLRNILSK;YTGIGDNSTIR;DLNLLKENETR;NFILNEIKDLK;EVRSFEKFK
KK;LLDFASFVAFK;EFVNSLDDLHK;FFNFPGMRIMK;DSADVWAHQKY;QDYINLGNEFR;EWWAKRIG
ILR;FASFVAFKEYF;SIFSAFAGNVY;ILIRNEKDLFK
A6802
FSAFAGNVYYI;YSIFSAFAGNV;KVAGISPDYFL;FIAYDSADVWA;DSVIIPMQDYI;FVVWDMIRSAM
;RSAMSSVSDSV;NISSLPSKYGI;DAFNVLFDRGI;EAALNFINRAS;SAMSSVSDSVI;NTLNNWIFRL
L;WDMIRSAMSSV;TRSPPYSIFSA;DATLSKNISFI;QYFFFSQFQAL;NIPKNTLNNWI;DVWAHQKYF
KL

A6901
DVWAHQKYFKL;NTLNNWIFRLL;PAYSWNVLKNV;YSIFSAFAGNV;NTNEDFVVWDM;AALNFINRASV
;FVVWDMIRSAM;FSAFAGNVYYI
B0702 FPGMRIMKLAF;IPKNTLNNWIF;FVVWDMIRSAM;SPGRDFFNFIL;SPIDFTRSPPY
B0801
B0802
B1501
WQMFAYSPIDF;WLLDFASFVAF;STWEVGVGEAY;LVMNLPLFIAY;MQDYINLGNEF;YGIGDLGKGAY
;SIFSAFAGNVY;IFSAFAGNVYY;GMRIMKLAFDF;EQEQAWDSPAY;LLNISSLPSKY;YSWNVLKNVK
Y
B1801
WEVGVGEAYAF;IELVMNLPLFI;YEWWAKRIGIL;KEIKVQEVLQY;FENDLEDVSRL;IDFLFASSQSY
;WLLDFASFVAF
B2705 KRYANDKGIEL;TRYSDLKKLSF
B3501
SPIDFTRSPPY;FPGMRIMKLAF;FVVWDMIRSAM;FASSQSYWQMF;YANDKGIELVM;LVMNLPLFIAY
;STWEVGVGEAY;IPKNTLNNWIF;EQEQAWDSPAY;FASFVAFKEYF;WLLDFASFVAF;SIFSAFAGNV
Y;WEVGVGEAYAF;EVLQYFFFSQF;YSWNVLKNVKY;IAYDSADVWAH;YGIGDLGKGAY;FFSQFQALK
RY;WAHQKYFKLRF;SSVSDSVIIPM;DFASFVAFKEY;MQDYINLGNEF;SSQSYWQMFAY
B3901 FKDKFLKEAAL;EKDLFKLRNIL
B4001
WEVGVGEAYAF;GEAYAFNGLWV;YEWWAKRIGIL;FENDLEDVSRL;IELVMNLPLFI;NETRYSDLKKL
B4002 WEVGVGEAYAF
B4402 KEIKVQEVLQY
B4403 KEIKVQEVLQY
B4501
B5101 LPHNYIKNCIV
B5301 FPGMRIMKLAF;IPKNTLNNWIF;SPGRDFFNFIL;FASFVAFKEYF;SPIDFTRSPPY
B5401 LPHNYIKNCIV;FPGMRIMKLAF;LPLFIAYDSAD
B5701
B5801
FASFVAFKEYF;LFIAYDSADVW;FASSQSYWQMF;YLNTNEDFVVW;ILNEIKDLKIW;KGAYKFIDFLF
;SSYWLLDFASF

**Allele 15-mer plus 9-mer core**
DRB1_0101 GILLNISSLPSKYGI-ISSLPSKYG;LRDFFNFPGMRIMKL-
FFNFPGMRI;RDFFNFPGMRIMKLA-FPGMRIMKL;SGILLNISSLPSKYG-
LLNISSLPS;FVVWDMIRSAMSSVS-WDMIRSAMS;VVWDMIRSAMSSVSD-
IRSAMSSVS;DFFNFPGMRIMKLAF-FPGMRIMKL;FFNFPGMRIMKLAFD-
FPGMRIMKL;ILLNISSLPSKYGIG-ISSLPSKYG;LLNISSLPSKYGIGD-
ISSLPSKYG;VWDMIRSAMSSVSDS-IRSAMSSVS;WDMIRSAMSSVSDSV-
IRSAMSSVS;DKGIELVMNLPLFIA-LVMNLPLFI;GIELVMNLPLFIAYD-
VMNLPLFIA;IELVMNLPLFIAYDS-VMNLPLFIA;KGIELVMNLPLFIAY-
VMNLPLFIA;DMIRSAMSSVSDSVI-IRSAMSSVS;FNFPGMRIMKLAFDF-
FPGMRIMKL;LNISSLPSKYGIGDL-ISSLPSKYG;ELVMNLPLFIAYDSA-
VMNLPLFIA;ASSQSYWQMFAYSPI-YWQMFAYSP;QSYWQMFAYSPIDFT-
YWQMFAYSP;SQSYWQMFAYSPIDF-YWQMFAYSP;SSQSYWQMFAYSPID-
YWQMFAYSP;DFVVWDMIRSAMSSV-WDMIRSAMS;RKSGILLNISSLPSK-
LLNISSLPS;FASSQSYWQMFAYSP-FASSQSYWQ;KSGILLNISSLPSKY-
LLNISSLPS;EDFVVWDMIRSAMSS-WDMIRSAMS;KRKSGILLNISSLPS-
ILLNISSLP;AYSPIDFTRSPPYSI-YSPIDFTRS;IDFTRSPPYSIFSAF-
FTRSPPYSI;NEDFVVWDMIRSAMS-VVWDMIRSA;PIDFTRSPPYSIFSA-
FTRSPPYSI;YSPIDFTRSPPYSIF-FTRSPPYSI;SPIDFTRSPPYSIFS-
FTRSPPYSI;KDLFKLRNILSKEIK-LRNILSKEI;DLFKLRNILSKEIKV-
LRNILSKEI;EKDLFKLRNILSKEI-LFKLRNILS;LFKLRNILSKEIKVQ-
LRNILSKEI;FKLRNILSKEIKVQE-LRNILSKEI;YWQMFAYSPIDFTRS-

YWQMFAYSP;GNEFRANIPKNTLNN-FRANIPKNT;LGNEFRANIPKNTLN-
FRANIPKNT;LVMNLPLFIAYDSAD-VMNLPLFIA;NEFRANIPKNTLNNW-
FRANIPKNT;MIRSAMSSVSDSVII-IRSAMSSVS;NLGNEFRANIPKNTL-
FRANIPKNT;NFPGMRIMKLAFDFD-FPGMRIMKL;INLGNEFRANIPKNT-
LGNEFRANI;IRSAMSSVSDSVIIP-IRSAMSSVS;FPGMRIMKLAFDFDS-
FPGMRIMKL;RLRDFFNFPGMRIMK-FFNFPGMRI;SRLRDFFNFPGMRIM-
FFNFPGMRI;NDKGIELVMNLPLFI-DKGIELVMN;NISSLPSKYGIGDLG-
ISSLPSKYG;ISSLPSKYGIGDLGK-ISSLPSKYG;VSRLRDFFNFPGMRI-
VSRLRDFFN;SYWQMFAYSPIDFTR-YWQMFAYSP;FTRSPPYSIFSAFAG-
FTRSPPYSI;RSFEKFKKKSSYWLL-FKKKSSYWL;SFEKFKKKSSYWLLD-
FKKKSSYWL;FEKFKKKSSYWLLDF-FKKKSSYWL;DFTRSPPYSIFSAFA-
FTRSPPYSI;EKFKKKSSYWLLDFA-FKKKSSYWL;VRSFEKFKKKSSYWL-
FEKFKKKSS;VMNLPLFIAYDSADV-VMNLPLFIA;IKNCIVYTGIGDNST-
IKNCIVYTG;KFIDFLFASSQSYWQ-FLFASSQSY;FIDFLFASSQSYWQM-
FLFASSQSY;IDFLFASSQSYWQMF-FLFASSQSY;YAFNGLWVKSPGRDF-
FNGLWVKSP;IVYTGIGDNSTIREF-YTGIGDNST;CIVYTGIGDNSTIRE-
YTGIGDNST;KLRNILSKEIKVQEV-LRNILSKEI;EFRANIPKNTLNNWI-
FRANIPKNT;LRNILSKEIKVQEVL-LRNILSKEI;FKKKSSYWLLDFASF-
FKKKSSYWL;FRANIPKNTLNNWIF-FRANIPKNT;AYKFIDFLFASSQSY-
FIDFLFASS;DKFLKEAALNFINRA-LKEAALNFI;FKDKFLKEAALNFIN-
LKEAALNFI;KDKFLKEAALNFINR-LKEAALNFI;YKFIDFLFASSQSYW-
FLFASSQSY;LNLKRKSGILLNISS-LKRKSGILL;SFKDKFLKEAALNFI-
FLKEAALNF;NCIVYTGIGDNSTIR-YTGIGDNST;NLNLKRKSGILLNIS-
LKRKSGILL;KNCIVYTGIGDNSTI-YTGIGDNST;WQMFAYSPIDFTRSP-
YSPIDFTRS;HNYIKNCIVYTGIGD-YIKNCIVYT;PHNYIKNCIVYTGIG-
YIKNCIVYT;AYAFNGLWVKSPGRD-FNGLWVKSP;KFKKKSSYWLLDFAS-
FKKKSSYWL;LPHNYIKNCIVYTGI-YIKNCIVYT;NLPHNYIKNCIVYTG-
YIKNCIVYT;QMFAYSPIDFTRSPP-YSPIDFTRS;EAYAFNGLWVKSPGR-
FNGLWVKSP;MKYKKIRINLNLKRK-YKKIRINLN;LPLFIAYDSADVWAH-
FIAYDSADV;GEAYAFNGLWVKSPG-FNGLWVKSP;VGEAYAFNGLWVKSP-
AFNGLWVKS;PLFIAYDSADVWAHQ-FIAYDSADV;KFLKEAALNFINRAS-
LKEAALNFI;INLNLKRKSGILLNI-LKRKSGILL;MFAYSPIDFTRSPPY-
YSPIDFTRS;DFLFASSQSYWQMFA-FASSQSYWQ;FAYSPIDFTRSPPYS-
YSPIDFTRS;MNLPLFIAYDSADVW-FIAYDSADV;SPPYSIFSAFAGNVY-
IFSAFAGNV;IRINLNLKRKSGILL-INLNLKRKS;NLPLFIAYDSADVWA-
FIAYDSADV;ALNFINRASVDEVRS-FINRASVDE;FLFASSQSYWQMFAY-
FASSQSYWQ;RSPPYSIFSAFAGNV-YSIFSAFAG;RINLNLKRKSGILLN-
LKRKSGILL;YFFFSQFQALKRYAN-FSQFQALKR;LNFINRASVDEVRSF-
FINRASVDE;PPYSIFSAFAGNVYY-IFSAFAGNV;FFFSQFQALKRYAND-
FQALKRYAN;AFNGLWVKSPGRDFF-FNGLWVKSP;SDLNLLKENETRYSD-
LKENETRYS;LNLLKENETRYSDLK-LKENETRYS;DSDLNLLKENETRYS-
LNLLKENET;AALNFINRASVDEVR-FINRASVDE;EAALNFINRASVDEV-
FINRASVDE;DLNLLKENETRYSDL-LKENETRYS;FNGLWVKSPGRDFFN-
FNGLWVKSP;TNEDFVVWDMIRSAM-VVWDMIRSA;NLLKENETRYSDLKK-
LKENETRYS;LKRKSGILLNISSLP-LKRKSGILL;NYIKNCIVYTGIGDN-
IKNCIVYTG;NTNEDFVVWDMIRSA-FVVWDMIRS;DAFNVLFDRGILIRN-
FNVLFDRGI;LSFKDKFLKEAALNF-FKDKFLKEA;YSIFSAFAGNVYYID-
IFSAFAGNV;FKEYFLKDSRDAFNV-YFLKDSRDA;AFKEYFLKDSRDAFN-
YFLKDSRDA;DSPAYSWNVLKNVKY-YSWNVLKNV;VYTGIGDNSTIREFV-
YTGIGDNST;FVAFKEYFLKDSRDA-FKEYFLKDS;YTGIGDNSTIREFVN-
YTGIGDNST;PYSIFSAFAGNVYYI-IFSAFAGNV;SPAYSWNVLKNVKYE-
YSWNVLKNV;VAFKEYFLKDSRDAF-YFLKDSRDA;QNLPHNYIKNCIVYT-
PHNYIKNCI;PAYSWNVLKNVKYEW-YSWNVLKNV;KEYFLKDSRDAFNVL-
YFLKDSRDA;NLKRKSGILLNISSL-LKRKSGILL;KSSYWLLDFASFVAF-
LLDFASFVA;TWEVGVGEAYAFNGL-VGVGEAYAF;SSYWLLDFASFVAFK-
LLDFASFVA;STWEVGVGEAYAFNG-VGVGEAYAF;KEAALNFINRASVDE-

AALNFINRA;YKKIRINLNLKRKSG-YKKIRINLN;KKSSYWLLDFASFVA-
YWLLDFASF;WEVGVGEAYAFNGLW-VGVGEAYAF;KYKKIRINLNLKRKS-
YKKIRINLN;FSQFQALKRYANDKG-FQALKRYAN;FFSQFQALKRYANDK-
FQALKRYAN;RNEKDLFKLRNILSK-LFKLRNILS;LFIAYDSADVWAHQK-
FIAYDSADV;NEKDLFKLRNILSKE-LFKLRNILS;FNVLFDRGILIRNEK-
FDRGILIRN;AWDSPAYSWNVLKNV-DSPAYSWNV;AFNVLFDRGILIRNE-
FDRGILIRN;FLKEAALNFINRASV-LKEAALNFI;FIAYDSADVWAHQKY-
FIAYDSADV;VKYEWWAKRIGILRK-YEWWAKRIG
DRB1_0301 ILNEIKDLKIWVEDF-EIKDLKIWV;FFNFPGMRIMKLAFD-
GMRIMKLAF;LWVKSPGRDFFNFIL-PGRDFFNFI;QDYINLGNEFRANIP-
LGNEFRANI;LRNILSKEIKVQEVL-LSKEIKVQE;RNILSKEIKVQEVLQ-
LSKEIKVQE;DFDSDNQNLPHNYIK-FDSDNQNLP;KFIDFLFASSQSYWQ-
FLFASSQSY;LKDSRDAFNVLFDRG-DSRDAFNVL
DRB1_0401 MKYKKIRINLNLKRK-YKKIRINLN;KFKKKSSYWLLDFAS-
FKKKSSYWL;RKSGILLNISSLPSK-LLNISSLPS;KSGILLNISSLPSKY-
LLNISSLPS;KRKSGILLNISSLPS-ILLNISSLP;SGILLNISSLPSKYG-
LLNISSLPS;GILLNISSLPSKYGI-LLNISSLPS;KYKKIRINLNLKRKS-
YKKIRINLN;YKKIRINLNLKRKSG-YKKIRINLN;ENETRYSDLKKLSFK-
RYSDLKKLS;NETRYSDLKKLSFKD-RYSDLKKLS;LHKKYIFDYLNTNED-
YIFDYLNTN;KKYIFDYLNTNEDFV-YIFDYLNTN;HKKYIFDYLNTNEDF-
YIFDYLNTN;NFPGMRIMKLAFDFD-FPGMRIMKL;ETRYSDLKKLSFKDK-
RYSDLKKLS;QDYINLGNEFRANIP-YINLGNEFR;DLHKKYIFDYLNTNE-
YIFDYLNTN;DDLHKKYIFDYLNTN-DDLHKKYIF;WNVLKNVKYEWWAKR-
WNVLKNVKY;FVVWDMIRSAMSSVS-WDMIRSAMS;NEDFVVWDMIRSAMS-
FVVWDMIRS;EDFVVWDMIRSAMSS-FVVWDMIRS;NEKDLFKLRNILSKE-
LFKLRNILS;RNEKDLFKLRNILSK-LFKLRNILS;VVWDMIRSAMSSVSD-
IRSAMSSVS;KDLFKLRNILSKEIK-LFKLRNILS;EKDLFKLRNILSKEI-
LFKLRNILS;KFIDFLFASSQSYWQ-FLFASSQSY;VLQYFFFSQFQALKR-
FFSQFQALK;LQYFFFSQFQALKRY-FFSQFQALK;FIDFLFASSQSYWQM-
FASSQSYWQ;ILLNISSLPSKYGIG-LLNISSLPS;YFFFSQFQALKRYAN-
FFSQFQALK;DFVVWDMIRSAMSSV-WDMIRSAMS;IDFLFASSQSYWQMF-
FASSQSYWQ;ILNEIKDLKIWVEDF-ILNEIKDLK;VLKNVKYEWWAKRIG-
VKYEWWAKR;LLNISSLPSKYGIGD-LLNISSLPS;QYFFFSQFQALKRYA-
FFSQFQALK;YINLGNEFRANIPKN-YINLGNEFR;KNVKYEWWAKRIGIL-
VKYEWWAKR;EVLQYFFFSQFQALK-YFFFSQFQA;LKNVKYEWWAKRIGI-
VKYEWWAKR;KYIFDYLNTNEDFVV-YIFDYLNTN;WDMIRSAMSSVSDSV-
IRSAMSSVS;VWDMIRSAMSSVSDS-IRSAMSSVS;NVLKNVKYEWWAKRI-
VKYEWWAKR;YIFDYLNTNEDFVVW-YIFDYLNTN
DRB1_0404 SGILLNISSLPSKYG-ILLNISSLP;KRKSGILLNISSLPS-
ILLNISSLP;RKSGILLNISSLPSK-ILLNISSLP;KSGILLNISSLPSKY-
ILLNISSLP;GILLNISSLPSKYGI-ILLNISSLP;ILLNISSLPSKYGIG-
ILLNISSLP;LKRKSGILLNISSLP-GILLNISSL;AFDFDSDNQNLPHNY-
FDSDNQNLP;KKYIFDYLNTNEDFV-FDYLNTNED;KYIFDYLNTNEDFVV-
YLNTNEDFV;YIFDYLNTNEDFVVW-YLNTNEDFV;IFDYLNTNEDFVVWD-
YLNTNEDFV;FDYLNTNEDFVVWDM-YLNTNEDFV;DLFKLRNILSKEIKV-
LFKLRNILS;VSRLRDFFNFPGMRI-RLRDFFNFP;WAKRIGILRKYIDVI-
WAKRIGILR;YKFIDFLFASSQSYW-FIDFLFASS;AYKFIDFLFASSQSY-
FIDFLFASS;KKSSYWLLDFASFVA-WLLDFASFV;SSYWLLDFASFVAFK-
WLLDFASFV;SYWLLDFASFVAFKE-WLLDFASFV;KSSYWLLDFASFVAF-
WLLDFASFV;RDFFNFILNEIKDLK-FFNFILNEI;KFIDSDLNLLKENET-
FIDSDLNLL;IIPMQDYINLGNEFR-PMQDYINLG;IPKNTLNNWIFRLLE-
PKNTLNNWI;DFFNFILNEIKDLKI-ILNEIKDLK;FFNFILNEIKDLKIW-
ILNEIKDLK;LEDVSRLRDFFNFPG-RLRDFFNFP
DRB1_0405 GDNSTIREFVNSLDD-IREFVNSLD;IGDNSTIREFVNSLD-
NSTIREFVN;STIREFVNSLDDLHK-IREFVNSLD;DNSTIREFVNSLDDL-
IREFVNSLD;NSTIREFVNSLDDLH-IREFVNSLD;TIREFVNSLDDLHKK-

IREFVNSLD;IREFVNSLDDLHKKY-IREFVNSLD;MKYKKIRINLNLKRK-
YKKIRINLN;TLNNWIFRLLESDLD-NNWIFRLLE;LNNWIFRLLESDLDA-
FRLLESDLD;NNWIFRLLESDLDAT-FRLLESDLD;FVVWDMIRSAMSSVS-
WDMIRSAMS;IDHFRGFVSTWEVGV-FRGFVSTWE;VVWDMIRSAMSSVSD-
WDMIRSAMS;DHFRGFVSTWEVGVG-FRGFVSTWE;NWIFRLLESDLDATL-
FRLLESDLD;IKIDHFRGFVSTWEV-FRGFVSTWE;WIFRLLESDLDATLS-
FRLLESDLD;VIKIDHFRGFVSTWE-IDHFRGFVS;LHKKYIFDYLNTNED-
YIFDYLNTN;HKKYIFDYLNTNEDF-FDYLNTNED;NEDFVVWDMIRSAMS-
FVVWDMIRS;KKYIFDYLNTNEDFV-FDYLNTNED;KIDHFRGFVSTWEVG-
FRGFVSTWE;KYKKIRINLNLKRKS-YKKIRINLN;EDFVVWDMIRSAMSS-
WDMIRSAMS;YKKIRINLNLKRKSG-YKKIRINLN;IFSAFAGNVYYIDLE-
FSAFAGNVY;KYIFDYLNTNEDFVV-FDYLNTNED;IVYTGIGDNSTIREF-
IGDNSTIRE;YIFDYLNTNEDFVVW-FDYLNTNED;DFVVWDMIRSAMSSV-
WDMIRSAMS;SGILLNISSLPSKYG-LLNISSLPS;GILLNISSLPSKYGI-
LLNISSLPS;VWDMIRSAMSSVSDS-WDMIRSAMS;WDMIRSAMSSVSDSV-
WDMIRSAMS;IFDYLNTNEDFVVWD-FDYLNTNED;FDYLNTNEDFVVWDM-
FDYLNTNED;HFRGFVSTWEVGVGE-FRGFVSTWE;LLNISSLPSKYGIGD-
ISSLPSKYG;FRGFVSTWEVGVGEA-FRGFVSTWE;YFLEQEQAWDSPAYS-
EQAWDSPAY;FLEQEQAWDSPAYSW-QAWDSPAYS;LWVKSPGRDFFNFIL-
LWVKSPGRD;QSYWQMFAYSPIDFT-WQMFAYSPI;VWAHQKYFKLRFDAS-
QKYFKLRFD;SYWQMFAYSPIDFTR-WQMFAYSPI;SQSYWQMFAYSPIDF-
WQMFAYSPI;VSRLRDFFNFPGMRI-LRDFFNFPG;SSQSYWQMFAYSPID-
WQMFAYSPI;DSPAYSWNVLKNVKY-AYSWNVLKN;ILLNISSLPSKYGIG-
ISSLPSKYG;YFFFSQFQALKRYAN-FSQFQALKR;SPAYSWNVLKNVKYE-
AYSWNVLKN;FRLLESDLDATLSKN-FRLLESDLD;IFRLLESDLDATLSK-
FRLLESDLD;QAWDSPAYSWNVLKN-QAWDSPAYS;FKLRFDASKDKVAGI-
LRFDASKDK;SRLRDFFNFPGMRIM-FFNFPGMRI;LNTNEDFVVWDMIRS-
TNEDFVVWD;VLQYFFFSQFQALKR-FFSQFQALK;LQYFFFSQFQALKRY-
FSQFQALKR;FFFSQFQALKRYAND-FSQFQALKR;EKDLFKLRNILSKEI-
LFKLRNILS;NTNEDFVVWDMIRSA-FVVWDMIRS;KDLFKLRNILSKEIK-
LFKLRNILS;YWQMFAYSPIDFTRS-WQMFAYSPI;TNEDFVVWDMIRSAM-FVVWDMIRS;
DRB1_0701 RSFEKFKKKSSYWLL-FKKKSSYWL;SFEKFKKKSSYWLLD-
FKKKSSYWL;FEKFKKKSSYWLLDF-FKKKSSYWL;VRSFEKFKKKSSYWL-
KFKKKSSYW;YSPIDFTRSPPYSIF-FTRSPPYSI;SPIDFTRSPPYSIFS-
FTRSPPYSI;EKDLFKLRNILSKEI-LFKLRNILS;KDLFKLRNILSKEIK-
LRNILSKEI;EKFKKKSSYWLLDFA-FKKKSSYWL;AYSPIDFTRSPPYSI-
IDFTRSPPY;DLFKLRNILSKEIKV-LRNILSKEI;LFKLRNILSKEIKVQ-
LRNILSKEI;IDFLFASSQSYWQMF-FLFASSQSY;FIDFLFASSQSYWQM-
FLFASSQSY;FKLRNILSKEIKVQE-LRNILSKEI;KFIDFLFASSQSYWQ-
FLFASSQSY;WDMIRSAMSSVSDSV-IRSAMSSVS;PIDFTRSPPYSIFSA-
FTRSPPYSI;YKFIDFLFASSQSYW-FLFASSQSY;NDLEDVSRLRDFFNF-
VSRLRDFFN;IDFTRSPPYSIFSAF-FTRSPPYSI;GDLGKGAYKFIDFLF-
LGKGAYKFI;VVWDMIRSAMSSVSD-IRSAMSSVS;FVVWDMIRSAMSSVS-
MIRSAMSSV;DYFLEQEQAWDSPAY-YFLEQEQAW;VWDMIRSAMSSVSDS-
IRSAMSSVS;DMIRSAMSSVSDSVI-IRSAMSSVS;FDYLNTNEDFVVWDM-
YLNTNEDFV;YIFDYLNTNEDFVVW-YLNTNEDFV;IFDYLNTNEDFVVWD-
YLNTNEDFV;KFKKKSSYWLLDFAS-FKKKSSYWL;KYIFDYLNTNEDFVV-
YLNTNEDFV;DFLFASSQSYWQMFA-LFASSQSYW;
DRB1_0802 FNVLFDRGILIRNEK-FDRGILIRN;VLFDRGILIRNEKDL-
RGILIRNEK;LFDRGILIRNEKDLF-RGILIRNEK;LEQEQAWDSPAYSWN-
EQAWDSPAY;ASSQSYWQMFAYSPI-YWQMFAYSP;PLFIAYDSADVWAHQ-
FIAYDSADV;YDSADVWAHQKYFKL-VWAHQKYFK;SSQSYWQMFAYSPID-
WQMFAYSPI;SQFQALKRYANDKGI-FQALKRYAN;FSQFQALKRYANDKG-
FQALKRYAN;YFFFSQFQALKRYAN-FFSQFQALK;DATLSKNISFITRLY-
LSKNISFIT;FFSQFQALKRYANDK-FQALKRYAN;FFFSQFQALKRYAND-FQALKRYAN

```
DRB1_0901 FDASKDKVAGISPDY-FDASKDKVA;YGIGDLGKGAYKFID-
LGKGAYKFI;IDFLFASSQSYWQMF-FLFASSQSY;FIDFLFASSQSYWQM-
FLFASSQSY;KFIDFLFASSQSYWQ-FLFASSQSY;IVYTGIGDNSTIREF-
IVYTGIGDN;ANIPKNTLNNWIFRL-PKNTLNNWI;KYGIGDLGKGAYKFI-
IGDLGKGAY;QSYWQMFAYSPIDFT-MFAYSPIDF;SYWQMFAYSPIDFTR-
MFAYSPIDF;DFLFASSQSYWQMFA-FASSQSYWQ;SLPSKYGIGDLGKGA-
PSKYGIGDL;FLFASSQSYWQMFAY-FASSQSYWQ;NFILNEIKDLKIWVE-
FILNEIKDL;FKDKFLKEAALNFIN-LKEAALNFI;SFKDKFLKEAALNFI-
FLKEAALNF;DKFLKEAALNFINRA-LKEAALNFI;KDKFLKEAALNFINR-
LKEAALNFI;DVWAHQKYFKLRFDA-WAHQKYFKL;LDDLHKKYIFDYLNT-
HKKYIFDYL;YWQMFAYSPIDFTRS-MFAYSPIDF;WQMFAYSPIDFTRSP-
MFAYSPIDF;YKFIDFLFASSQSYW-FLFASSQSY;KFLKEAALNFINRAS-
LKEAALNFI;NILSKEIKVQEVLQY-EIKVQEVLQ;IREFVNSLDDLHKKY-
FVNSLDDLH;YSWNVLKNVKYEWWA-WNVLKNVKY;SQSYWQMFAYSPIDF-
WQMFAYSPI;AYKFIDFLFASSQSY-IDFLFASSQ;VWAHQKYFKLRFDAS-
WAHQKYFKL;FEKFKKKSSYWLLDF-FKKKSSYWL;EKFKKKSSYWLLDFA-
FKKKSSYWL;SFEKFKKKSSYWLLD-FKKKSSYWL;RIGILRKYIDVIKID-
LRKYIDVIK;NVKYEWWAKRIGILR-WWAKRIGIL;RSFEKFKKKSSYWLL-
FKKKSSYWL;FVVWDMIRSAMSSVS-WDMIRSAMS;AGNVYYIDLEALDKF-
YYIDLEALD;HKKYIFDYLNTNEDF-FDYLNTNED
DRB1_1101 LKNVKYEWWAKRIGI-YEWWAKRIG;KNVKYEWWAKRIGIL-
YEWWAKRIG;VLKNVKYEWWAKRIG-VKYEWWAKR;NVKYEWWAKRIGILR-
YEWWAKRIG;VKYEWWAKRIGILRK-YEWWAKRIG;VYYIDLEALDKFIDS-
YIDLEALDK;QALKRYANDKGIELV-LKRYANDKG;NVYYIDLEALDKFID-
YIDLEALDK;IAYDSADVWAHQKYF-YDSADVWAH;FKLRNILSKEIKVQE-
FKLRNILSK;FDRGILIRNEKDLFK-RGILIRNEK;EVRSFEKFKKKSSYW-
FEKFKKKSS;KYEWWAKRIGILRKY-YEWWAKRIG;HNYIKNCIVYTGIGD-
YIKNCIVYT;YEWWAKRIGILRKYI-YEWWAKRIG;DFLFASSQSYWQMFA-
FASSQSYWQ;SRDAFNVLFDRGILI-FNVLFDRGI;EFRANIPKNTLNNWI-
FRANIPKNT;YFLEQEQAWDSPAYS-EQAWDSPAY;FSQFQALKRYANDKG-
FQALKRYAN;SQFQALKRYANDKGI-FQALKRYAN;LEDVSRLRDFFNFPG-LEDVSRLRD
DRB1_1302 IRINLNLKRKSGILL-INLNLKRKS;RINLNLKRKSGILLN-
LKRKSGILL;INLNLKRKSGILLNI-LKRKSGILL;KSGILLNISSLPSKY-
ILLNISSLP;RKSGILLNISSLPSK-ILLNISSLP;SGILLNISSLPSKYG-
ILLNISSLP;LKRKSGILLNISSLP-LKRKSGILL;EWWAKRIGILRKYID-
WAKRIGILR;KKIRINLNLKRKSGI-INLNLKRKS;KRKSGILLNISSLPS-
ILLNISSLP;YKKIRINLNLKRKSG-INLNLKRKS;KIRINLNLKRKSGIL-
INLNLKRKS;WWAKRIGILRKYIDV-IGILRKYID;WAKRIGILRKYIDVI-
IGILRKYID;KYKKIRINLNLKRKS-IRINLNLKR;AKRIGILRKYIDVIK-
IGILRKYID;KRIGILRKYIDVIKI-IGILRKYID;GILLNISSLPSKYGI-
ILLNISSLP;LPHNYIKNCIVYTGI-IKNCIVYTG;LNLKRKSGILLNISS-
LKRKSGILL;NLPHNYIKNCIVYTG-YIKNCIVYT;NLNLKRKSGILLNIS-
LKRKSGILL;PHNYIKNCIVYTGIG-IKNCIVYTG;HNYIKNCIVYTGIGD-
IKNCIVYTG;KGIELVMNLPLFIAY-VMNLPLFIA;ILLNISSLPSKYGIG-
ILLNISSLP;PAYSWNVLKNVKYEW-WNVLKNVKY;DKGIELVMNLPLFIA-
LVMNLPLFI;AYSWNVLKNVKYEWW-WNVLKNVKY;NYIKNCIVYTGIGDN-
IKNCIVYTG;IELVMNLPLFIAYDS-LVMNLPLFI;GIELVMNLPLFIAYD-
LVMNLPLFI;YSWNVLKNVKYEWWA-WNVLKNVKY;DSPAYSWNVLKNVKY-
SWNVLKNVK;SPAYSWNVLKNVKYE-WNVLKNVKY;MKYKKIRINLNLKRK-
IRINLNLKR;RIGILRKYIDVIKID-IGILRKYID;IGILRKYIDVIKIDH-
IGILRKYID;RDFFNFILNEIKDLK-FFNFILNEI;DLDATLSKNISFITR-
LSKNISFIT;NDKGIELVMNLPLFI-IELVMNLPL;DATLSKNISFITRLY-
LSKNISFIT;LDATLSKNISFITRL-LSKNISFIT;ATLSKNISFITRLYG-
LSKNISFIT;YIKNCIVYTGIGDNS-IKNCIVYTG;KDKVAGISPDYFLEQ-
VAGISPDYF;NIPKNTLNNWIFRLL-IPKNTLNNW;SDLDATLSKNISFIT-
DATLSKNIS;IPKNTLNNWIFRLLE-LNNWIFRLL;ELVMNLPLFIAYDSA-
```

VMNLPLFIA;FVVWDMIRSAMSSVS-VWDMIRSAM;NLKRKSGILLNISSL-
LKRKSGILL;DFFNFILNEIKDLKI-ILNEIKDLK;WEVGVGEAYAFNGLW-
VGVGEAYAF;CIVYTGIGDNSTIRE-VYTGIGDNS;FFNFILNEIKDLKIW-
ILNEIKDLK;KFIDSDLNLLKENET-FIDSDLNLL;IVYTGIGDNSTIREF-
IGDNSTIRE;VYTGIGDNSTIREFV-IGDNSTIRE;LFDRGILIRNEKDLF-
RGILIRNEK;FIDSDLNLLKENETR-LNLLKENET;SWNVLKNVKYEWWAK-
WNVLKNVKY;KDLFKLRNILSKEIK-LFKLRNILS;SDLNLLKENETRYSD-
LNLLKENET;DSDLNLLKENETRYS-LNLLKENET;PKNTLNNWIFRLLES-
LNNWIFRLL;IKNCIVYTGIGDNST-IKNCIVYTG;FDRGILIRNEKDLFK-
RGILIRNEK;FNVLFDRGILIRNEK-VLFDRGILI;YTGIGDNSTIREFVN-
IGDNSTIRE;TGIGDNSTIREFVNS-IGDNSTIRE;NSLDDLHKKYIFDYL-
LDDLHKKYI;VAFKEYFLKDSRDAF-FKEYFLKDS;KNTLNNWIFRLLESD-
LNNWIFRLL;EDFENDLEDVSRLRD-FENDLEDVS;EKDLFKLRNILSKEI-
LFKLRNILS;VVWDMIRSAMSSVSD-IRSAMSSVS;NEFRANIPKNTLNNW-
FRANIPKNT;VWDMIRSAMSSVSDS-IRSAMSSVS;LVMNLPLFIAYDSAD-
LVMNLPLFI;NTLNNWIFRLLESDL-LNNWIFRLL;DLFKLRNILSKEIKV-
LRNILSKEI;WNVLKNVKYEWWAKR-WNVLKNVKY;DLEALDKFIDSDLNL-
LEALDKFID;NFILNEIKDLKIWVE-ILNEIKDLK;NVLFDRGILIRNEKD-
RGILIRNEK;LGNEFRANIPKNTLN-FRANIPKNT;GRDFFNFILNEIKDL-
FFNFILNEI;NLGNEFRANIPKNTL-FRANIPKNT;FNFILNEIKDLKIWV-
ILNEIKDLK;VLFDRGILIRNEKDL-RGILIRNEK;TLSKNISFITRLYGR-
LSKNISFIT;MQDYINLGNEFRANI-YINLGNEFR;FLFASSQSYWQMFAY-
FLFASSQSY;IDSDLNLLKENETRY-LNLLKENET;EAALNFINRASVDEV-
FINRASVDE;WDMIRSAMSSVSDSV-IRSAMSSVS;LSKNISFITRLYGRT-
LSKNISFIT;LFKLRNILSKEIKVQ-LRNILSKEI;YEWWAKRIGILRKYI-
WAKRIGILR;KYEWWAKRIGILRKY-WAKRIGILR;RGILIRNEKDLFKLR-
LIRNEKDLF;DRGILIRNEKDLFKL-LIRNEKDLF;YKFIDFLFASSQSYW-
FLFASSQSY;VKYEWWAKRIGILRK-WAKRIGILR;QDYINLGNEFRANIP-
YINLGNEFR;AALNFINRASVDEVR-INRASVDEV;IPMQDYINLGNEFRA-
YINLGNEFR;LLNISSLPSKYGIGD-LLNISSLPS;INLGNEFRANIPKNT-
LGNEFRANI;GNEFRANIPKNTLNN-FRANIPKNT;EFRANIPKNTLNNWI-FRANIPKNT
DRB1_1501 KDLFKLRNILSKEIK-LFKLRNILS;EKDLFKLRNILSKEI-
LFKLRNILS;NEKDLFKLRNILSKE-LFKLRNILS;SGILLNISSLPSKYG-
LLNISSLPS;GILLNISSLPSKYGI-LLNISSLPS;RNEKDLFKLRNILSK-
LFKLRNILS;KSGILLNISSLPSKY-LLNISSLPS;RKSGILLNISSLPSK-
LLNISSLPS;DLFKLRNILSKEIKV-FKLRNILSK;YKKIRINLNLKRKSG-
IRINLNLKR;LFKLRNILSKEIKVQ-FKLRNILSK;KYKKIRINLNLKRKS-
IRINLNLKR;KKIRINLNLKRKSGI-IRINLNLKR;MKYKKIRINLNLKRK-
IRINLNLKR;ILLNISSLPSKYGIG-LLNISSLPS;LEQEQAWDSPAYSWN-
EQAWDSPAY;KRKSGILLNISSLPS-GILLNISSL;WNVLKNVKYEWWAKR-
NVKYEWWAK;FVVWDMIRSAMSSVS-MIRSAMSSV;LSKNISFITRLYGRT-
SFITRLYGR;AKRIGILRKYIDVIK-ILRKYIDVI;VLKNVKYEWWAKRIG-
VKYEWWAKR;NVLKNVKYEWWAKRI-VKYEWWAKR;LKNVKYEWWAKRIGI-
VKYEWWAKR;FKLRNILSKEIKVQE-LRNILSKEI;KRIGILRKYIDVIKI-
ILRKYIDVI;LLNISSLPSKYGIGD-LLNISSLPS;WAKRIGILRKYIDVI-
WAKRIGILR;VKYEWWAKRIGILRK-WAKRIGILR;VRSFEKFKKKSSYWL-
VRSFEKFKK;NVKYEWWAKRIGILR-VKYEWWAKR;IGILRKYIDVIKIDH-
ILRKYIDVI;KIRINLNLKRKSGIL-IRINLNLKR;KNVKYEWWAKRIGIL-
VKYEWWAKR;RIGILRKYIDVIKID-ILRKYIDVI;IRINLNLKRKSGILL-
IRINLNLKR;DFVVWDMIRSAMSSV-FVVWDMIRS;TLSKNISFITRLYGR-
SKNISFITR;EAALNFINRASVDEV-ALNFINRAS;KEAALNFINRASVDE-
ALNFINRAS;EAYAFNGLWVKSPGR-YAFNGLWVK;VVWDMIRSAMSSVSD-
MIRSAMSSV;LKEAALNFINRASVD-ALNFINRAS;FLKEAALNFINRASV-
ALNFINRAS;AYAFNGLWVKSPGRD-GLWVKSPGR;IDHFRGFVSTWEVGV-
FRGFVSTWE;ISSLPSKYGIGDLGK-ISSLPSKYG;KIDHFRGFVSTWEVG-
FRGFVSTWE;WDMIRSAMSSVSDSV-IRSAMSSVS;DHFRGFVSTWEVGVG-

FRGFVSTWE;VWDMIRSAMSSVSDS-MIRSAMSSV;LLESDLDATLSKNIS-
LDATLSKNI;VGEAYAFNGLWVKSP-AYAFNGLWV;GEAYAFNGLWVKSPG-
AYAFNGLWV;IIPMQDYINLGNEFR-PMQDYINLG;EWWAKRIGILRKYID-
WAKRIGILR;ALDKFIDSDLNLLKE-FIDSDLNLL;NEDFVVWDMIRSAMS-
FVVWDMIRS;EDFVVWDMIRSAMSS-FVVWDMIRS;RSFEKFKKKSSYWLL-
FKKKSSYWL;TLNNWIFRLLESDLD-IFRLLESDL;IPMQDYINLGNEFRA-
YINLGNEFR;GNEFRANIPKNTLNN-FRANIPKNT;PMQDYINLGNEFRAN-
YINLGNEFR;VIKIDHFRGFVSTWE-IDHFRGFVS;LNNWIFRLLESDLDA-FRLLESDLD
DRB3_0101 LESDLDATLSKNISF-LESDLDATL;VKSPGRDFFNFILNE-
PGRDFFNFI;LFDRGILIRNEKDLF-ILIRNEKDL;IDLEALDKFIDSDLN-
EALDKFIDS;FDFDSDNQNLPHNYI-FDSDNQNLP;RKSGILLNISSLPSK-
LLNISSLPS;KEYFLKDSRDAFNVL-FLKDSRDAF;AFDFDSDNQNLPHNY-
FDSDNQNLP;EYFLKDSRDAFNVLF-FLKDSRDAF;FKDKFLKEAALNFIN-
FLKEAALNF;FENDLEDVSRLRDFF-FENDLEDVS;VAFKEYFLKDSRDAF-
FKEYFLKDS;FIDSDLNLLKENETR-IDSDLNLLK;AFKEYFLKDSRDAFN-
FLKDSRDAF;FKEYFLKDSRDAFNV-FLKDSRDAF;QDYINLGNEFRANIP-
LGNEFRANI;AFNVLFDRGILIRNE-VLFDRGILI
DRB4_0101 FFNFILNEIKDLKIW-ILNEIKDLK;VGVGEAYAFNGLWVK-
GEAYAFNGL;NVKYEWWAKRIGILR-YEWWAKRIG;VKYEWWAKRIGILRK-
YEWWAKRIG;SDLDATLSKNISFIT-DATLSKNIS;QNLPHNYIKNCIVYT-
LPHNYIKNC;KNVKYEWWAKRIGIL-YEWWAKRIG;LLDFASFVAFKEYFL-
LLDFASFVA;NVLKNVKYEWWAKRI-VKYEWWAKR;VLKNVKYEWWAKRIG-
VKYEWWAKR;LKNVKYEWWAKRIGI-YEWWAKRIG;NQNLPHNYIKNCIVY-
LPHNYIKNC;LPHNYIKNCIVYTGI-LPHNYIKNC;KYEWWAKRIGILRKY-
YEWWAKRIG;YEWWAKRIGILRKYI-YEWWAKRIG
DRB5_0101 KNVKYEWWAKRIGIL-VKYEWWAKR;VLKNVKYEWWAKRIG-
VKYEWWAKR;LKNVKYEWWAKRIGI-VKYEWWAKR;NVLKNVKYEWWAKRI-
VKYEWWAKR;WNVLKNVKYEWWAKR-WNVLKNVKY;AHQKYFKLRFDASKD-
FKLRFDASK;NVKYEWWAKRIGILR-VKYEWWAKR;VKYEWWAKRIGILRK-
VKYEWWAKR;LSKEIKVQEVLQYFF-IKVQEVLQY;NDKGIELVMNLPLFI-
ELVMNLPLF;LPHNYIKNCIVYTGI-YIKNCIVYT;IFDYLNTNEDFVVWD-
YLNTNEDFV;WIFRLLESDLDATLS-FRLLESDLD;AKRIGILRKYIDVIK-
AKRIGILRK;KFLKEAALNFINRAS-FLKEAALNF;IDHFRGFVSTWEVGV-
FRGFVSTWE;FENDLEDVSRLRDFF-DLEDVSRLR

**<CAA59727 outer surface protein A;Protein;Borrelia garinii>**

**Allele 8-mer**
A0101
A0201 YLLGIGLI;SLDEKNSV;FTLEGTLA;LMATVEKL
A0202
LMATVEKL;SLDEKNSV;YLLGIGLI;LIACKQNV;LLGIGLIL;KAVEITTL;KLTIAEDL;GIGLILAL
;LVSKKVTL;SLMATVEK
A0203
YLLGIGLI;LMATVEKL;SLDEKNSV;LIACKQNV;LLGIGLIL;GLILALIA;SQKTKNLV;KVTEGTVV
;FTLEGTLA;TLKDKSST;KAVEITTL;LVSKKVTL
A0204 SLDEKNSV;LMATVEKL;KVTEGTVV;LLGIGLIL;FTLEGTLA;LIACKQNV;
A0206 KAVEITTL;FTLEGTLA;KVTEGTVV;YLLGIGLI;SLDEKNSV;ATVEKLEL;LIACKQNV
A0211
YLLGIGLI;SLDEKNSV;DLPGGMKV;KVTEGTVV;ALDDSDTT;TLEGTLAA;LLGIGLIL;LMATVEKL
;EISEKTIV;LIACKQNV;KEDGKTLV;FTLEGTLA;KLTIAEDL;GLILALIA;AADGKTTL;GIGLILA
L;EVLKDFTL;SLMATVEK
A0212
SLDEKNSV;YLLGIGLI;DLPGGMKV;LMATVEKL;LLGIGLIL;ALDDSDTT;TLEGTLAA;KVTEGTVV
;LIACKQNV;EISEKTIV;FTLEGTLA

A0216
SLDEKNSV;DLPGGMKV;YLLGIGLI;LLGIGLIL;LMATVEKL;TLEGTLAA;KVTEGTVV;ALDDSDTT
;EISEKTIV;LIACKQNV;AADGKTTL;GIGLILAL;LVSKKVTL;TLKDKSST;EVLKDFTL;GLILALI
A
A0219
SLDEKNSV;YLLGIGLI;DLPGGMKV;LMATVEKL;ALDDSDTT;LLGIGLIL;TLEGTLAA;LIACKQNV
;AADGKTTL
A0301
VLSKNILK;ILALIACK;KTTFEIFK;SLMATVEK;VSKKVTLK;GMKVLVSK;SVNSQKTK;KTGKWDSK
;TISVNSQK;KVLVSKEK;TIAEDLSK;KTDKSKVK
A1101
KTTFEIFK;SLMATVEK;SVNSQKTK;TIAEDLSK;TISVNSQK;GTLAADGK;TVEKLELK;KTGKWDSK
;KVLVSKEK;VLSKNILK;VSKKVTLK;ITTLEKLK;ILALIACK;NVSSLDEK;GMKVLVSK;KTDKSKV
K;TKNLVFTK
A2301 KYLLGIGL
A2402 KYSLMATV
A2403 KYLLGIGL
A2601 DTITVQKY
A2602 DTITVQKY;EVLKDFTL;EITTLEKL;SVDLPGGM;EISEKTIV
A2902 DTITVQKY
A3001
KVLVSKEK;KTTFEIFK;SLMATVEK;VSKKVTLK;KTGKWDSK;KTDKSKVK;SVNSQKTK;GMKVLVSK
;IVRANGTR;RLEYTDIK
A3002
A3101 IVRANGTR;KTTFEIFK;GMKVLVSK;ISEKTIVR;VSKKVTLK;KVLVSKEK
A3301 IVRANGTR
A6801
DTTQATKK;NVSSLDEK;TISVNSQK;KTTFEIFK;EIFKEDGK;ILALIACK;TVEKLELK;TIAEDLSK
;ITTLEKLK;SLMATVEK;DTITVQKY;TTFEIFKE;SVNSQKTK;IVRANGTR;ELKGTSDK;EDTITVQ
K;EGTVVLSK
A6802 TSTLTISV;LIACKQNV;EISEKTIV;EVLKDFTL;TVVLSKNI
A6901 FTLEGTLA;EVLKDFTL;EISEKTIV;YLLGIGLI;LIACKQNV
B0702 LPGGMKVL;KAVEITTL
B0801
B0802
B1501
B1801
B2705
B3501 LPGGMKVL
B3901 FKEDGKTL
B4001 GEISEKTI
B4002
B4402
B4403
B4501 AEDLSKTT
B5101
B5301 LPGGMKVL
B5401
B5701
B5801 KSSTEEKF;KAVEITTL

**Allele 9-mer**
A0101
A0201
YLLGIGLIL;FTLEGTLAA;SLMATVEKL;KTSTLTISV;FTKEDTITV;ALIACKQNV;LLGIGLILA

A0202
SLMATVEKL;ALIACKQNV;KSGEITVAL;YLLGIGLIL;KTSTLTISV;FTKEDTITV;GIGLILALI;L
LGIGLILA;KVTEGTVVL;ILKSGEITV;TLEKLKDAL;FTLEGTLAA;TLVSKKVTL
A0203
FTKEDTITV;ILKSGEITV;SLMATVEKL;ALIACKQNV;YLLGIGLIL;KTSTLTISV;LLGIGLILA;F
TLEGTLAA;GKYSLMATV;GIGLILALI;TLKVTEGTV;TLVSKKVTL
A0204 SLMATVEKL;ALIACKQNV;ILKSGEITV;FTKEDTITV;YLLGIGLIL;FTLEGTLAA
A0206
FTLEGTLAA;FTKEDTITV;KTSTLTISV;KVTEGTVVL;YLLGIGLIL;ALIACKQNV;LLGIGLILA;L
AADGKTTL;GKYSLMATV;FKEDGKTLV;SLMATVEKL
A0211
YLLGIGLIL;ILKSGEITV;ALIACKQNV;LLGIGLILA;SLMATVEKL;KVTEGTVVL;TLVSKKVTL;A
LDDSDTTQ;DLPGGMKVL;FTKEDTITV;DLSKTTFEI;FTLEGTLAA;KTSTLTISV;LAADGKTTL;NL
EGKAVEI;TLKVTEGTV;FKEDGKTLV;SSLDEKNSV;TLEKLKDAL;SLDEKNSVS;VLKDFTLEG;GKY
SLMATV;VDLPGGMKV
A0212
YLLGIGLIL;ILKSGEITV;FTLEGTLAA;SLMATVEKL;ALIACKQNV;TLVSKKVTL;FTKEDTITV;L
AADGKTTL;ALDDSDTTQ;DLPGGMKVL;LLGIGLILA;TLEKLKDAL;FKEDGKTLV;KVTEGTVVL;VD
LPGGMKV;TLKVTEGTV;SSLDEKNSV;VLSKNILKS;VLKDFTLEG
A0216
ILKSGEITV;YLLGIGLIL;TLVSKKVTL;ALIACKQNV;SLMATVEKL;DLSKTTFEI;LLGIGLILA;A
LDDSDTTQ;TLKVTEGTV;NLEGKAVEI;TLEKLKDAL;LAADGKTTL;DLPGGMKVL;FTKEDTITV;FK
EDGKTLV;KVTEGTVVL;GKYSLMATV;KTSTLTISV
A0219
ILKSGEITV;YLLGIGLIL;SLMATVEKL;LAADGKTTL;ALIACKQNV;DLSKTTFEI;ALDDSDTTQ;T
LVSKKVTL;FKEDGKTLV;FTLEGTLAA;LLGIGLILA;DLPGGMKVL
A0301 VVLSKNILK;KTKNLVFTK;LVSKKVTLK;LILALIACK;LTISVNSQK
A1101
VVLSKNILK;KTKNLVFTK;LTISVNSQK;AVEITTLEK;LTIAEDLSK;LILALIACK;STEEKFNEK;A
TVEKLELK;SVDLPGGMK;YSLMATVEK;LVSKKVTLK;AADGKTTLK;SAGTNLEGK;GSGTLEGEK;TQ
ATKKTGK;GGMKVLVSK;RANGTRLEY;ISVNSQKTK;KSDGSGKAK
A2301 KYDSAGTNL;KYLLGIGLI
A2402 KYLLGIGLI
A2403 KYLLGIGLI;KYDSAGTNL
A2601
A2602 DLPGGMKVL
A2902
A3001
KTKNLVFTK;YSLMATVEK;AVEITTLEK;RANGTRLEY;KDKSSTEEK;SGKAKEVLK;KSDGSGKAK;I
SVNSQKTK;
A3002 RANGTRLEY
A3101 KTKNLVFTK;LTISVNSQK;VVLSKNILK;LILALIACK
A3301 EISEKTIVR
A6801
LTISVNSQK;TIVRANGTR;YSLMATVEK;EISEKTIVR;EITTLEKLK;LTIAEDLSK;KTKNLVFTK;S
TEEKFNEK;LVSKKVTLK;ATVEKLELK
A6802 KTSTLTISV;FTKEDTITV;TTLKVTEGT;DLSKTTFEI
A6901 FTLEGTLAA;FTKEDTITV;DLSKTTFEI
B0702 IVRANGTRL;KVTEGTVVL;LAADGKTTL
B0801
B0802
B1501 SQKTKNLVF;RANGTRLEY;YLLGIGLIL
B1801 VEITTLEKL
B2705
B3501 LAADGKTTL;RANGTRLEY;
B3901

```
B4001 KEVLKDFTL;AEDLSKTTF;GEISEKTIV
B4002 GEISEKTIV;AEDLSKTTF;KEVLKDFTL
B4402 AEDLSKTTF
B4403 KEVLKDFTL
B4501 AEDLSKTTF
B5101 LPGGMKVLV
B5301 LPGGMKVLV
B5401 LPGGMKVLV
B5701
B5801 RANGTRLEY
```

**Allele 10-mer**
```
A0101
A0201 YLLGIGLILA;SLDEKNSVSV;ALDDSDTTQA;LMATVEKLEL;LLGIGLILAL
A0202
YLLGIGLILA;LLGIGLILAL;LMATVEKLEL;TLAADGKTTL;SLDEKNSVSV;ALDDSDTTQA;VLKDF
TLEGT;ILKSGEITVA;YSLMATVEKL
A0203
SLDEKNSVSV;YLLGIGLILA;LLGIGLILAL;VLKDFTLEGT;ILKSGEITVA;TLAADGKTTL;LMATV
EKLEL;TLKVTEGTVV;ALDDSDTTQA;NILKSGEITV
A0204 SLDEKNSVSV;LMATVEKLEL;TLAADGKTTL;LLGIGLILAL;SVDLPGGMKV
A0206
SLDEKNSVSV;YLLGIGLILA;NILKSGEITV;ALDDSDTTQA;LLGIGLILAL;FTLEGTLAAD;AADGK
TTLKV;TTLKVTEGTV
A0211
SLDEKNSVSV;YLLGIGLILA;DLPGGMKVLV;ALDDSDTTQA;LLGIGLILAL;TLAADGKTTL;LMATV
EKLEL;SVDLPGGMKV;NILKSGEITV;AADGKTTLKV;VLKDFTLEGT;TLKVTEGTVV;ILKSGEITVA
;TTLKVTEGTV;SKTSTLTISV;EISEKTIVRA;VFTKEDTITV
A0212
SLDEKNSVSV;YLLGIGLILA;LLGIGLILAL;DLPGGMKVLV;LMATVEKLEL;ALDDSDTTQA;TLAAD
GKTTL;VLKDFTLEGT;NILKSGEITV;SVDLPGGMKV;ILKSGEITVA;AADGKTTLKV;TLKVTEGTVV
A0216
SLDEKNSVSV;DLPGGMKVLV;YLLGIGLILA;LLGIGLILAL;ALDDSDTTQA;NILKSGEITV;TLAAD
GKTTL;LMATVEKLEL;AADGKTTLKV;SVDLPGGMKV;TLKVTEGTVV;TTLKVTEGTV;TIVRANGTRL
;ILKSGEITVA;NLVFTKEDTI
A0219
SLDEKNSVSV;ALDDSDTTQA;LLGIGLILAL;YLLGIGLILA;DLPGGMKVLV;TLAADGKTTL;AADGK
TTLKV;LMATVEKLEL;NILKSGEITV
A0301
TLVSKKVTLK;TLTISVNSQK;KYSLMATVEK;TVVLSKNILK;KLTIAEDLSK;GLILALIACK;LAADG
KTTLK;VTEGTVVLSK;KLELKGTSDK;GMKVLVSKEK;KAVEITTLEK;LSKTTFEIFK;VSVDLPGGMK
;TTQATKKTGK
A1101
TVVLSKNILK;TTQATKKTGK;VTEGTVVLSK;SSTEEKFNEK;KAVEITTLEK;GLILALIACK;KQNVS
SLDEK;TLTISVNSQK;TLVSKKVTLK;VSVDLPGGMK;LSKTTFEIFK;TISVNSQKTK;MATVEKLELK
;LAADGKTTLK;KTIVRANGTR;KLTIAEDLSK;KVLVSKEKDK;GSGKAKEVLK;GTLEGEKTDK;KYSL
MATVEK;TLEKLKDALK;GMKVLVSKEK
A2301 KYSLMATVEK;KYLLGIGLIL;DLSKTTFEIF;NSQKTKNLVF
A2402 KYLLGIGLIL;
A2403 KYLLGIGLIL;
A2601
A2602 SVSVDLPGGM;EIFKEDGKTL
A2902
A3001
KAVEITTLEK;GTRLEYTDIK;LSKTTFEIFK;KQNVSSLDEK;KYSLMATVEK;GMKVLVSKEK;KVLVS
KEKDK;TTQATKKTGK;VSKKVTLKDK;TVVLSKNILK;KTIVRANGTR;KLELKGTSDK
```

```
A3002
A3101 KYSLMATVEK;KTIVRANGTR;LSKTTFEIFK
A3301
A6801
TVVLSKNILK;MATVEKLELK;TLTISVNSQK;DSAGTNLEGK;TTQATKKTGK;LAADGKTTLK;KTIVR
ANGTR;TISVNSQKTK;TLVSKKVTLK;SSTEEKFNEK;KAVEITTLEK;LSKTTFEIFK
A6802 TTLKVTEGTV;EISEKTIVRA;LGIGLILALI
A6901
TTLKVTEGTV;NILKSGEITV;SVDLPGGMKV;EISEKTIVRA;YLLGIGLILA;TTLEKLKDAL;
B0702
B0801
B0802
B1501
B1801 VDLPGGMKVL;
B2705
B3501 IAEDLSKTTF
B3901
B4001
B4002
B4402
B4403
B4501 EEKFNEKGEI
B5101
B5301
B5401 LPGGMKVLVS
B5701
B5801
```

**Allele 11-mer**
```
A0101
A0201
YLLGIGLILAL;SLMATVEKLEL;LVFTKEDTITV;LLGIGLILALI;ILALIACKQNV;ALDDSDTTQAT
A0202
ILALIACKQNV;SLMATVEKLEL;YLLGIGLILAL;LIACKQNVSSL;ILKSGEITVAL;LLGIGLILALI
;KAVEITTLEKL;VLKDFTLEGTL;LVFTKEDTITV;SVNSQKTKNLV;NVSSLDEKNSV;ALDDSDTTQA
T;LAADGKTTLKV
A0203
LIACKQNVSSL;VLKDFTLEGTL;YLLGIGLILAL;ILALIACKQNV;LLGIGLILALI;ILKSGEITVAL
;SLMATVEKLEL;LVFTKEDTITV;LAADGKTTLKV;SVNSQKTKNLV;TLKVTEGTVVL;KTTLKVTEGT
V
A0204
SLMATVEKLEL;YLLGIGLILAL;LVFTKEDTITV;LAADGKTTLKV;ILALIACKQNV;SVNSQKTKNLV
A0206
YLLGIGLILAL;KAVEITTLEKL;LVFTKEDTITV;LAADGKTTLKV;LLGIGLILALI;ALDDSDTTQAT
;ILALIACKQNV;LIACKQNVSSL;SLMATVEKLEL;KTIVRANGTRL;TTLKVTEGTVV
A0211
YLLGIGLILAL;ILALIACKQNV;SLMATVEKLEL;ALDDSDTTQAT;LLGIGLILALI;ILKSGEITVAL
;VLKDFTLEGTL;LAADGKTTLKV;LVFTKEDTITV;EIFKEDGKTLV;SVNSQKTKNLV;SSLDEKNSVS
V;NVSSLDEKNSV;TLKVTEGTVVL;SVDLPGGMKVL;VDLPGGMKVLV;TTLKVTEGTVV;NLEGKAVEI
TT;KTDKSKVKLTI
A0212
YLLGIGLILAL;VLKDFTLEGTL;ILKSGEITVAL;SLMATVEKLEL;ALDDSDTTQAT;ILALIACKQNV
;LVFTKEDTITV;LAADGKTTLKV;LLGIGLILALI;EIFKEDGKTLV;SSLDEKNSVSV;SVNSQKTKNL
V;NVSSLDEKNSV;TLKVTEGTVVL
A0216
YLLGIGLILAL;SLMATVEKLEL;ILALIACKQNV;ILKSGEITVAL;VLKDFTLEGTL;LVFTKEDTITV
```

;LAADGKTTLKV;LLGIGLILALI;EIFKEDGKTLV;SVNSQKTKNLV;ALDDSDTTQAT;NVSSLDEKNS
V;TLKVTEGTVVL;NLEGKAVEITT;TTLKVTEGTVV;LIACKQNVSSL
A0219
YLLGIGLILAL;LAADGKTTLKV;SLMATVEKLEL;ILALIACKQNV;ALDDSDTTQAT;ILKSGEITVAL
;LLGIGLILALI;NVSSLDEKNSV;LVFTKEDTITV;VLKDFTLEGTL;LIACKQNVSSL
A0301
TLAADGKTTLK;STLTISVNSQK;KTLVSKKVTLK;KVTEGTVVLSK;LMATVEKLELK;LTISVNSQKTK
;GTVVLSKNILK;FTKEDTITVQK;IVRANGTRLEY;TTLEKLKDALK
A1101
STLTISVNSQK;TTFEIFKEDGK;SVSVDLPGGMK;KVTEGTVVLSK;KTLVSKKVTLK;TTLEKLKDALK
;GTVVLSKNILK;TLAADGKTTLK;LTISVNSQKTK;SQKTKNLVFTK;KSSTEEKFNEK;AVEITTLEKL
K;ATKKTGKWDSK;LMATVEKLELK;TLKDKSSTEEK;FTKEDTITVQK;DLSKTTFEIFK;TLEGTLAAD
GK;LVSKKVTLDK
A2301 KYSLMATVEKL
A2402 KYSLMATVEKL;KWDSKTSTLTI
A2403 KYSLMATVEKL
A2601
A2602 TIAEDLSKTTF;KTIVRANGTRL;SVDLPGGMKVL;
A2902 IVRANGTRLEY
A3001
KTLVSKKVTLK;ATKKTGKWDSK;TLKDKSSTEEK;KVTEGTVVLSK;IVRANGTRLEY;SQKTKNLVFTK
;KFNEKGEISEK;FTKEDTITVQK;GTVVLSKNILK;AVEITTLEKLK;LTISVNSQKTK
A3002
A3101 KTLVSKKVTLK;SQKTKNLVFTK;KFNEKGEISEK;STLTISVNSQK
A3301 DLSKTTFEIFK;EKTIVRANGTR
A6801
TTFEIFKEDGK;STLTISVNSQK;LTISVNSQKTK;DTTQATKKTGK;FTKEDTITVQK;DLSKTTFEIFK
;TTLEKLKDALK;TLAADGKTTLK;LMATVEKLELK;EKTIVRANGTR;GTVVLSKNILK;SVSVDLPGGM
K;LVSKEKDKDGK;TLKDKSSTEEK;YTDIKSDGSGK
A6802
NVSSLDEKNSV;EIFKEDGKTLV;TTLKVTEGTVV;DTITVQKYDSA;SVNSQKTKNLV;ILALIACKQNV
;LIACKQNVSSL;LVFTKEDTITV;LAADGKTTLKV;
A6901
LAADGKTTLKV;TTLKVTEGTVV;EIFKEDGKTLV;YLLGIGLILAL;LVFTKEDTITV;SVNSQKTKNLV
B0702
B0801
B0802
B1501 IVRANGTRLEY;YLLGIGLILAL;VQKYDSAGTNL
B1801
B2705
B3501 TIAEDLSKTTF
B3901
B4001 FEIFKEDGKTL;LEGKAVEITTL;AEDLSKTTFEI;GEKTDKSKVKL
B4002
B4402
B4403
B4501 AEDLSKTTFEI
B5101
B5301
B5401 LPGGMKVLVSK
B5701
B5801

**Allele 15-mer plus 9-mer core**
DRB1_0101 KKYLLGIGLILALIA-LLGIGLILA;KYLLGIGLILALIAC-
LLGIGLILA;MKKYLLGIGLILALI-LLGIGLILA;KTGKWDSKTSTLTIS-

WDSKTSTLT;TGKWDSKTSTLTISV-WDSKTSTLT;GKWDSKTSTLTISVN-
WDSKTSTLT;KKTGKWDSKTSTLTI-WDSKTSTLT;TKKTGKWDSKTSTLT-
TGKWDSKTS;LKDFTLEGTLAADGK-FTLEGTLAA;VLKDFTLEGTLAADG-
FTLEGTLAA;KDFTLEGTLAADGKT-FTLEGTLAA;YLLGIGLILALIACK-
IGLILALIA;LLGIGLILALIACKQ-IGLILALIA;KEVLKDFTLEGTLAA-
LKDFTLEGT;EVLKDFTLEGTLAAD-FTLEGTLAA;ISEKTIVRANGTRLE-
IVRANGTRL;SEKTIVRANGTRLEY-IVRANGTRL;TTLKVTEGTVVLSKN-
VTEGTVVLS;KTTLKVTEGTVVLSK-VTEGTVVLS;EKTIVRANGTRLEYT-
IVRANGTRL;GKTTLKVTEGTVVLS-LKVTEGTVV;KTIVRANGTRLEYTD-
IVRANGTRL;KNILKSGEITVALDD-LKSGEITVA;LSKNILKSGEITVAL-
LKSGEITVA;SKNILKSGEITVALD-LKSGEITVA;NILKSGEITVALDDS-
LKSGEITVA;DFTLEGTLAADGKTT-FTLEGTLAA;FTLEGTLAADGKTTL-
FTLEGTLAA;TLKVTEGTVVLSKNI-VTEGTVVLS;LGIGLILALIACKQN-
IGLILALIA;VLSKNILKSGEITVA-KNILKSGEI;LKVTEGTVVLSKNIL-
VTEGTVVLS;TIVRANGTRLEYTDI-VRANGTRLE;KEDGKTLVSKKVTLK-
GKTLVSKKV;EDGKTLVSKKVTLKD-GKTLVSKKV;SAGTNLEGKAVEITT-
LEGKAVEIT;DSAGTNLEGKAVEIT-GTNLEGKAV;KWDSKTSTLTISVNS-
WDSKTSTLT;WDSKTSTLTISVNSQ-WDSKTSTLT;AGTNLEGKAVEITTL-
LEGKAVEIT;GTNLEGKAVEITTLE-LEGKAVEIT;VDLPGGMKVLVSKEK-
LPGGMKVLV;DGKTTLKVTEGTVVL-LKVTEGTVV;VSVDLPGGMKVLVSK-
LPGGMKVLV;ITVQKYDSAGTNLEG-VQKYDSAGT;TITVQKYDSAGTNLE-
VQKYDSAGT;SVSVDLPGGMKVLVS-LPGGMKVLV;TNLEGKAVEITTLEK-
LEGKAVEIT;NLVFTKEDTITVQKY-FTKEDTITV;EISEKTIVRANGTRL-
KTIVRANGT;GIGLILALIACKQNV-ILALIACKQ;KNLVFTKEDTITVQK-
FTKEDTITV;IGLILALIACKQNVS-ILALIACKQ;TKNLVFTKEDTITVQ-
FTKEDTITV;KTKNLVFTKEDTITV-VFTKEDTIT;ADGKTTLKVTEGTVV-
ADGKTTLKV;IVRANGTRLEYTDIK-IVRANGTRL;LVFTKEDTITVQKYD-
FTKEDTITV;LKSGEITVALDDSDT-LKSGEITVA;DKDGKYSLMATVEKL-
YSLMATVEK;ILKSGEITVALDDSD-LKSGEITVA;KDKDGKYSLMATVEK-
KDGKYSLMA;KDGKYSLMATVEKLE-YSLMATVEK;DTITVQKYDSAGTNL-
VQKYDSAGT;SVDLPGGMKVLVSKE-LPGGMKVLV;GKTLVSKKVTLKDKS-
LVSKKVTLK;DGKTLVSKKVTLKDK-LVSKKVTLK;DGKYSLMATVEKLEL-
YSLMATVEK;EDTITVQKYDSAGTN-VQKYDSAGT;KEDTITVQKYDSAGT-
ITVQKYDSA;GKYSLMATVEKLELK-YSLMATVEK;EIFKEDGKTLVSKKV-
FKEDGKTLV;NSVSVDLPGGMKVLV-VDLPGGMKV;DLPGGMKVLVSKEKD-
MKVLVSKEK;LEGTLAADGKTTLKV-LEGTLAADG;TISVNSQKTKNLVFT-
VNSQKTKNL;YSLMATVEKLELKGT-YSLMATVEK;LEYTDIKSDGSGKAK-
IKSDGSGKA;RLEYTDIKSDGSGKA-YTDIKSDGS;TLTISVNSQKTKNLV-
VNSQKTKNL;GLILALIACKQNVSS-ILALIACKQ;STLTISVNSQKTKNL-
TISVNSQKT;ISVNSQKTKNLVFTK-VNSQKTKNL;TVQKYDSAGTNLEGK-
VQKYDSAGT;VQKYDSAGTNLEGKA-VQKYDSAGT;IFKEDGKTLVSKKVT-
GKTLVSKKV;VTEGTVVLSKNILKS-VTEGTVVLS;LPGGMKVLVSKEKDK-
MKVLVSKEK;VALDDSDTTQATKKT-LDDSDTTQA;YTDIKSDGSGKAKEV-
IKSDGSGKA;FKEDGKTLVSKKVTL-GKTLVSKKV;QKYDSAGTNLEGKAV-
YDSAGTNLE;TLEGTLAADGKTTLK-LEGTLAADG;LTISVNSQKTKNLVF-
VNSQKTKNL;KVTEGTVVLSKNILK-VTEGTVVLS;EYTDIKSDGSGKAKE-
IKSDGSGKA;SKVKLTIAEDLSKTT-VKLTIAEDL;VRANGTRLEYTDIKS-VRANGTRLE
DRB1_0301
DRB1_0401 KEVLKDFTLEGTLAA-VLKDFTLEG;EVLKDFTLEGTLAAD-
FTLEGTLAA;VLKDFTLEGTLAADG-FTLEGTLAA;LKDFTLEGTLAADGK-
FTLEGTLAA;KDFTLEGTLAADGKT-FTLEGTLAA;KNLVFTKEDTITVQK-
FTKEDTITV;NLVFTKEDTITVQKY-FTKEDTITV;LVFTKEDTITVQKYD-
FTKEDTITV;DFTLEGTLAADGKTT-FTLEGTLAA;LEYTDIKSDGSGKAK-
IKSDGSGKA;TKNLVFTKEDTITVQ-FTKEDTITV;FTLEGTLAADGKTTL-
FTLEGTLAA;RLEYTDIKSDGSGKA-YTDIKSDGS;KTKNLVFTKEDTITV-
KTKNLVFTK;AKEVLKDFTLEGTLA-VLKDFTLEG;EYTDIKSDGSGKAKE-IKSDGSGKA

DRB1_0404 KEVLKDFTLEGTLAA-VLKDFTLEG;GKAKEVLKDFTLEGT-
VLKDFTLEG;AKEVLKDFTLEGTLA-VLKDFTLEG;KAKEVLKDFTLEGTL-
VLKDFTLEG;SGKAKEVLKDFTLEG-AKEVLKDFT
DRB1_0405 TITVQKYDSAGTNLE-VQKYDSAGT;ITVQKYDSAGTNLEG-
YDSAGTNLE;TVQKYDSAGTNLEGK-YDSAGTNLE;VQKYDSAGTNLEGKA-
YDSAGTNLE;QKYDSAGTNLEGKAV-YDSAGTNLE
DRB1_0701 DGKTTLKVTEGTVVL-LKVTEGTVV;GKTTLKVTEGTVVLS-
LKVTEGTVV;KTTLKVTEGTVVLSK-LKVTEGTVV;ADGKTTLKVTEGTVV-
TLKVTEGTV;TTLKVTEGTVVLSKN-LKVTEGTVV
DRB1_0802
DRB1_0901
DRB1_1101
DRB1_1302 TEGTVVLSKNILKSG-TVVLSKNIL;EGTVVLSKNILKSGE-
TVVLSKNIL;GTVVLSKNILKSGEI-VVLSKNILK;VTEGTVVLSKNILKS-
TVVLSKNIL;TVVLSKNILKSGEIT-TVVLSKNIL;ISEKTIVRANGTRLE-
IVRANGTRL;SEKTIVRANGTRLEY-IVRANGTRL;EKTIVRANGTRLEYT-
IVRANGTRL;KTIVRANGTRLEYTD-IVRANGTRL;TLTISVNSQKTKNLV-
VNSQKTKNL;STLTISVNSQKTKNL-ISVNSQKTK;LTISVNSQKTKNLVF-
VNSQKTKNL;KVTEGTVVLSKNILK-TVVLSKNIL;TISVNSQKTKNLVFT-
VNSQKTKNL;LKVTEGTVVLSKNIL-LKVTEGTVV;TIVRANGTRLEYTDI-
VRANGTRLE;ISVNSQKTKNLVFTK-VNSQKTKNL;VVLSKNILKSGEITV-
VVLSKNILK;EISEKTIVRANGTRL-KTIVRANGT;EDGKTLVSKKVTLKD-
LVSKKVTLK;DGKTLVSKKVTLKDK-LVSKKVTLK
DRB1_1501 TEGTVVLSKNILKSG-VLSKNILKS;EGTVVLSKNILKSGE-VLSKNILKS
DRB3_0101
DRB4_0101
DRB5_0101 LPGGMKVLVSKEKDK-MKVLVSKEK;GGMKVLVSKEKDKDG-
MKVLVSKEK;VDLPGGMKVLVSKEK-VDLPGGMKV;PGGMKVLVSKEKDKD-
MKVLVSKEK;DLPGGMKVLVSKEKD-MKVLVSKEK;

**\<AAN87995 Outer Surface Protein C;Protein;Borrelia garinii\>**

**Allele 8-mer**
A0101 ITDSNAFV
A0201 LMTLFLFI;LLAGAYAI;FVLAVKEV;SLLAGAYA;ITDSNAFV;LISSIDEL
A0202
LLAGAYAI;LISSIDEL;LMTLFLFI;KLKNSEEL;GAYAISTL;ILMTLFLF;SLLAGAYA;FVLAVKEV
;ITDSNAFV;VIHQNNGL
A0203
LLAGAYAI;LMTLFLFI;FVLAVKEV;SLLAGAYA;GAYAISTL;KLKNSEEL;LISSIDEL;AVKEVEAL
;GQNGSLLA;VIHQNNGL;HQNNGLNA
A0204 FVLAVKEV;SLLAGAYA;LLAGAYAI
A0206
AQLGVAAA;FVLAVKEV;LLAGAYAI;GQNGSLLA;ITDSNAFV;LMTLFLFI;SLLAGAYA;ILMTLFLF
;HQNNGLNA;GAYAISTL;
A0211
LLAGAYAI;SLLAGAYA;FVLAVKEV;LMTLFLFI;ITDSNAFV;TLFLFISC;KLKNSEEL;ELTNPVVA
;RLKGSHAQ;LISSIDEL;ILMTLFLF;AKGPNLTV
A0212
LLAGAYAI;FVLAVKEV;SLLAGAYA;LMTLFLFI;VIHQNNGL;ITDSNAFV;TLFLFISC;KLKNSEEL
;RLKGSHAQ
A0216
SLLAGAYA;LLAGAYAI;FVLAVKEV;ITDSNAFV;KLKNSEEL;LMTLFLFI;LISSIDEL;TLFLFISC
;GAYAISTL;AKGPNLTV;ELTNPVVA
A0219 LLAGAYAI;ITDSNAFV;FVLAVKEV;SLLAGAYA;LMTLFLFI;LISSIDEL
A0301 ILKSNPTK;LITEKLSK;ISTLITEK;NLTVISKK;KSNPTKDK

A1101
SIDELANK;ISTLITEK;NAFVLAVK;AATDDHAK;LITEKLSK;KSNPTKDK;LANKAIGK;HAKEAILK
;PVVAESPK;EAFTNRLK;ESVESLAK
A2301 ILMTLFLF;AYAISTLI
A2402 AYAISTLI;ILMTLFLF
A2403 AYAISTLI
A2601
A2602 KITDSNAF;
A2902 ILMTLFLF;
A3001 KSNPTKDK;ILKSNPTK;PTKDKGAK;HAKEAILK;LANKAIGK;RLKGSHAQ;TEKLSKLK
A3002
A3101 HSEAFTNR;
A3301 HSEAFTNR;
A6801
EAFTNRLK;HSEAFTNR;NAFVLAVK;ESVESLAK;ISTLITEK;EALANSVK;NLTVISKK;HAKEAILK
;LANKAIGK
A6802 FVLAVKEV;LLAGAYAI;SNAFVLAV;STNPDESA
A6901 FVLAVKEV;ITDSNAFV;ESAKGPNL
B0702
B0801
B0802
B1501 KITDSNAF;ILMTLFLF;GSLLAGAY
B1801 DELANKAI
B2705
B3501
B3901 DHAKEAIL
B4001 SEAFTNRL
B4002 KELTNPVV
B4402
B4403 KEVEALIS
B4501
B5101
B5301
B5401
B5701
B5801

**Allele 9-mer**
A0101
A0201 ILMTLFLFI;ALISSIDEL;KITDSNAFV;SLLAGAYAI;ALANSVKEL
A0202
ALISSIDEL;ILMTLFLFI;KITDSNAFV;SVKELTNPV;LISSIDELA;SLAKAAQEA;ALANSVKEL;F
LFISCNNS;LITEKLSKL;RLKGSHAQL;KVIHQNNGL;GAYAISTLI;SLLAGAYAI;LLAGAYAIS;EL
KDLSESV;VLAVKEVEA;DLSESVESL;LAVKEVEAL
A0203
SVKELTNPV;ALISSIDEL;ALANSVKEL;ILMTLFLFI;LITEKLSKL;KITDSNAFV;RLKGSHAQL;E
LKDLSESV;SLAKAAQEA;SLLAGAYAI;LANKAIGKV;GAYAISTLI;VLAVKEVEA;AVKEVEALI;LL
AGAYAIS;ELNKKIEEA;FLFISCNNS;
A0204
SLLAGAYAI;SLAKAAQEA;ALANSVKEL;ALISSIDEL;KITDSNAFV;ILMTLFLFI;VLAVKEVEA
A0206
KITDSNAFV;SLLAGAYAI;ALISSIDEL;ILMTLFLFI;AQEALANSV;AQLGVAAAT;SVKELTNPV;G
AYAISTLI;KVIHQNNGL;LITEKLSKL
A0211
KITDSNAFV;SLLAGAYAI;ELKDLSESV;SLAKAAQEA;RLKGSHAQL;ILMTLFLFI;ALANSVKEL;D

LSESVESL;ALISSIDEL;SVKELTNPV;SIDELANKA;SAKGPNLTV;VLAVKEVEA;ITDSNAFVL;EL
NKKIEEA;LITEKLSKL;ATDDHAKEA;NLTVISKKI
A0212
SLLAGAYAI;ELKDLSESV;ILMTLFLFI;KITDSNAFV;SLAKAAQEA;ALISSIDEL;RLKGSHAQL;V
LAVKEVEA;DLSESVESL;SVKELTNPV;SIDELANKA;LITEKLSKL;SAKGPNLTV;ALANSVKEL
A0216
ELKDLSESV;KITDSNAFV;SLLAGAYAI;SLAKAAQEA;ALISSIDEL;RLKGSHAQL;ALANSVKEL;I
LMTLFLFI;SVKELTNPV;DLSESVESL;SAKGPNLTV;ELNKKIEEA;VLAVKEVEA;LITEKLSKL
A0219
SLLAGAYAI;ELKDLSESV;KITDSNAFV;ALISSIDEL;DLSESVESL;ILMTLFLFI;ITDSNAFVL;S
LAKAAQEA;ALANSVKEL
A0301 AILKSNPTK;TLITEKLSK;AISTLITEK;ELANKAIGK;ITEKLSKLK
A1101
SSIDELANK;AISTLITEK;STNPDESAK;AILKSNPTK;TLITEKLSK;AAATDDHAK;ITEKLSKLK;S
NAFVLAVK;
A2301
A2402
A2403
A2601
A2602 EAKNHSEAF;KVIHQNNGL;EVEALISSI
A2902
A3001 AILKSNPTK;ITEKLSKLK;KDKGAKELK;SVKELTNPV;STNPDESAK
A3002
A3101
A3301
A6801 STNPDESAK;ELANKAIGK;SSIDELANK;SNAFVLAVK;ITEKLSKLK
A6802
SVKELTNPV;EVEALISSI;DSNAFVLAV;ELKDLSESV;ILMTLFLFI;HSEAFTNRL;ISTLITEKL;L
ISSIDELA;ESVESLAKA
A6901 SLLAGAYAI;SVKELTNPV;ESVESLAKA;DSNAFVLAV
B0702
B0801
B0802
B1501
B1801
B2705
B3501 EAKNHSEAF;LAVKEVEAL;NGSLLAGAY
B3901 HSEAFTNRL
B4001
B4002
B4402
B4403
B4501 EEAKNHSEA
B5101
B5301
B5401
B5701
B5801 ITDSNAFVL

**Allele 10-mer**
A0101
A0201
ALISSIDELA;LLAGAYAIST;KITDSNAFVL;SLAKAAQEAL;ILMTLFLFIS;FVLAVKEVEA;VLAVK
EVEAL;TLITEKLSKL;AISTLITEKL

A0202
ALISSIDELA;SLAKAAQEAL;LLAGAYAIST;VLAVKEVEAL;TLITEKLSKL;AISTLITEKL;FLFIS
CNNSG;LAGAYAISTL;ILMTLFLFIS;KITDSNAFVL;ELANKAIGKV;FVLAVKEVEA;LMTLFLFISC
A0203
GQNGSLLAGA;LLAGAYAIST;ALISSIDELA;TLITEKLSKL;SLAKAAQEAL;VLAVKEVEAL;ELANK
AIGKV;AISTLITEKL;SVKELTNPVV;AAQEALANSV;HQNNGLNANA
A0204  SLAKAAQEAL;ELANKAIGKV;VLAVKEVEAL;FVLAVKEVEA;AAQEALANSV
A0206
GQNGSLLAGA;KEVEALISSI;AAQEALANSV;KITDSNAFVL;HQNNGLNANA;FVLAVKEVEA;ALISS
IDELA;KELKDLSESV;KKITDSNAFV;LLAGAYAIST;SLAKAAQEAL;SVKELTNPVV
A0211
SLAKAAQEAL;KITDSNAFVL;ELANKAIGKV;LLAGAYAIST;SIDELANKAI;VLAVKEVEAL;SVKEL
TNPVV;TLITEKLSKL;ALISSIDELA;DLSESVESLA;SLLAGAYAIS;LMTLFLFISC;ATDDHAKEAI
;AISTLITEKL;FVLAVKEVEA;AAQEALANSV;ALANSVKELT
A0212
SLAKAAQEAL;VLAVKEVEAL;LLAGAYAIST;TLITEKLSKL;ELANKAIGKV;SIDELANKAI;FVLAV
KEVEA;SVKELTNPVV;KITDSNAFVL;AAQEALANSV;SLLAGAYAIS
A0216
SLAKAAQEAL;ELANKAIGKV;TLITEKLSKL;LLAGAYAIST;VLAVKEVEAL;KITDSNAFVL;ALISS
IDELA;SVKELTNPVV;AAQEALANSV;AISTLITEKL;FVLAVKEVEA;ALANSVKELT;DLSESVESLA
A0219
SLAKAAQEAL;LLAGAYAIST;ELANKAIGKV;KITDSNAFVL;VLAVKEVEAL;TLITEKLSKL;AAQEA
LANSV
A0301  STLITEKLSK;KLKNSEELNK;LITEKLSKLK;ILKSNPTKDK;HSEAFTNRLK;LSESVESLAK
A1101
STLITEKLSK;YAISTLITEK;ISSIDELANK;HSEAFTNRLK;ASTNPDESAK;DSNAFVLAVK;VAAAT
DDHAK;LSESVESLAK;AQEALANSVK;LITEKLSKLK;KLKNSEELNK
A2301
A2402
A2403
A2601
A2602
A2902
A3001  KLKNSEELNK;KGPNLTVISK;STLITEKLSK;ILKSNPTKDK;HSEAFTNRLK
A3002
A3101
A3301
A6801
YAISTLITEK;DSNAFVLAVK;EAILKSNPTK;HSEAFTNRLK;ELNKKIEEAK;VAAATDDHAK;STLIT
EKLSK;LSESVESLAK
A6802
NSVKELTNPV;ESAKGPNLTV;ELANKAIGKV;NAFVLAVKEV;TASTNPDESA;AGAYAISTLI;NANAG
QNGSL;
A6901  ESAKGPNLTV;EALANSVKEL;ELANKAIGKV;EALISSIDEL;NAFVLAVKEV;FVLAVKEVEA
B0702
B0801
B0802
B1501
B1801  KEVEALISSI
B2705
B3501
B3901  NHSEAFTNRL
B4001  KEVEALISSI
B4002  EEAKNHSEAF
B4402
B4403  EEAKNHSEAF

```
B4501 EEAKNHSEAF
B5101
B5301 LAVKEVEALI
B5401
B5701
B5801
```

**Allele 11-mer**
```
A0101 ITDSNAFVLAV
A0201
LLAGAYAISTL;FVLAVKEVEAL;KITDSNAFVLA;SLLAGAYAIST;ITDSNAFVLAV;VLAVKEVEALI
;YAISTLITEKL;KLSKLKNSEEL
A0202
LLAGAYAISTL;KLSKLKNSEEL;SLAKAAQEALA;VLAVKEVEALI;RLKGSHAQLGV;FVLAVKEVEAL
;YAISTLITEKL;KITDSNAFVLA;FLFISCNNSGG;KAAQEALANSV;LAGAYAISTLI;ILMTLFLFIS
C
A0203
LLAGAYAISTL;RLKGSHAQLGV;SLAKAAQEALA;VLAVKEVEALI;YAISTLITEKL;KAAQEALANSV
;SVKELTNPVVA;KLSKLKNSEEL;KITDSNAFVLA;ILMTLFLFISC;FVLAVKEVEAL
A0204 LLAGAYAISTL;SLAKAAQEALA;SLLAGAYAIST;FVLAVKEVEAL;VLAVKEVEALI
A0206
FVLAVKEVEAL;KAAQEALANSV;YAISTLITEKL;KITDSNAFVLA;LLAGAYAISTL;SLLAGAYAIST
;ILMTLFLFISC;ITDSNAFVLAV;RLKGSHAQLGV
A0211
RLKGSHAQLGV;LLAGAYAISTL;SLLAGAYAIST;KLSKLKNSEEL;ITDSNAFVLAV;SLAKAAQEALA
;KITDSNAFVLA;VLAVKEVEALI;FVLAVKEVEAL;ILMTLFLFISC;ATDDHAKEAIL;KAAQEALANS
V;ELANKAIGKVI
A0212
RLKGSHAQLGV;LLAGAYAISTL;KLSKLKNSEEL;SLLAGAYAIST;FVLAVKEVEAL;ITDSNAFVLAV
;VLAVKEVEALI;SLAKAAQEALA;ILMTLFLFISC;ATDDHAKEAIL
A0216
LLAGAYAISTL;RLKGSHAQLGV;KLSKLKNSEEL;SLAKAAQEALA;SLLAGAYAIST;VLAVKEVEALI
;ITDSNAFVLAV;FVLAVKEVEAL
A0219
LLAGAYAISTL;ITDSNAFVLAV;RLKGSHAQLGV;FVLAVKEVEAL;SLLAGAYAIST;KLSKLKNSEEL
;YAISTLITEKL;
A0301 TLITEKLSKLK;KLKNSEELNKK;LTNPVVAESPK;ISTLITEKLSK;AILKSNPTKDK
A1101
LTNPVVAESPK;GVAAATDDHAK;AAQEALANSVK;TLITEKLSKLK;ISTLITEKLSK;TASTNPDESAK
;LISSIDELANK;KSNPTKDKGAK;AILKSNPTKDK;AYAISTLITEK
A2301 AYAISTLITEK
A2402
A2403
A2601
A2602 ATDDHAKEAIL
A2902
A3001
KSNPTKDKGAK;LTNPVVAESPK;KLKNSEELNKK;PTKDKGAKELK;AILKSNPTKDK;RLKGSHAQLGV
A3002
A3101 LTNPVVAESPK;KSNPTKDKGAK
A3301
A6801
LTNPVVAESPK;TASTNPDESAK;LISSIDELANK;ISTLITEKLSK;GVAAATDDHAK;TLITEKLSKLK
A6802
DTASTNPDESA;FVLAVKEVEAL;ESAKGPNLTVI;YAISTLITEKL;NGSLLAGAYAI;SNAFVLAVKEV
A6901 ITDSNAFVLAV;FVLAVKEVEAL;YAISTLITEKL;DTASTNPDESA;EALISSIDELA
```

```
B0702 NPTKDKGAKEL
B0801
B0802
B1501 GQNGSLLAGAY;ISKKITDSNAF
B1801
B2705
B3501 YAISTLITEKL;FVLAVKEVEAL
B3901
B4001 VEALISSIDEL;QEALANSVKEL
B4002
B4402
B4403
B4501 EEAKNHSEAFT
B5101
B5301
B5401
B5701
B5801
```

**Allele 15-mer plus 9-mer core**
```
DRB1_0101 GSLLAGAYAISTLIT-LAGAYAIST;NGSLLAGAYAISTLI-
LAGAYAIST;QNGSLLAGAYAISTL-LAGAYAIST;SLLAGAYAISTLITE-
LAGAYAIST;GQNGSLLAGAYAIST-LLAGAYAIS;NRLKGSHAQLGVAAA-
LKGSHAQLG;TNRLKGSHAQLGVAA-LKGSHAQLG;AFTNRLKGSHAQLGV-
LKGSHAQLG;EAFTNRLKGSHAQLG-TNRLKGSHA;FTNRLKGSHAQLGVA-
LKGSHAQLG;LAGAYAISTLITEKL-LAGAYAIST;LLAGAYAISTLITEK-
LAGAYAIST;LKGSHAQLGVAAATD-LKGSHAQLG;RLKGSHAQLGVAAAT-
LKGSHAQLG;AGAYAISTLITEKLS-ISTLITEKL;HQNNGLNANAGQNGS-
LNANAGQNG;IHQNNGLNANAGQNG-NNGLNANAG;YAISTLITEKLSKLK-
ISTLITEKL;AYAISTLITEKLSKL-ISTLITEKL;GAYAISTLITEKLSK-
ISTLITEKL;NNGLNANAGQNGSLL-LNANAGQNG;QNNGLNANAGQNGSL-
LNANAGQNG;NGLNANAGQNGSLLA-LNANAGQNG;LNANAGQNGSLLAGA-
LNANAGQNG;AKEAILKSNPTKDKG-LKSNPTKDK;GLNANAGQNGSLLAG-
LNANAGQNG;ANSVKELTNPVVAES-VKELTNPVV;HAKEAILKSNPTKDK-
EAILKSNPT;NSVKELTNPVVAESP-LTNPVVAES;KGSHAQLGVAAATDD-
AQLGVAAAT;GSHAQLGVAAATDDH-LGVAAATDD;AGQNGSLLAGAYAIS-
GQNGSLLAG;EAILKSNPTKDKGAK-LKSNPTKDK;KEAILKSNPTKDKGA-
LKSNPTKDK;SHAQLGVAAATDDHA-LGVAAATDD;AISTLITEKLSKLKN-
ISTLITEKL;ISTLITEKLSKLKNS-ISTLITEKL;SVKELTNPVVAESPK-
LTNPVVAES;VLAVKEVEALISSID-VKEVEALIS;AFVLAVKEVEALISS-
VKEVEALIS;FVLAVKEVEALISSI-VKEVEALIS;LAVKEVEALISSIDE-
VKEVEALIS;NAFVLAVKEVEALIS-LAVKEVEAL;HAQLGVAAATDDHAK-
LGVAAATDD;SSIDELANKAIGKVI-LANKAIGKV;ESLAKAAQEALANSV-
LAKAAQEAL;ISSIDELANKAIGKV-IDELANKAI;EALANSVKELTNPVV-
LANSVKELT;AQLGVAAATDDHAKE-LGVAAATDD;IDELANKAIGKVIHQ-
LANKAIGKV;SIDELANKAIGKVIH-LANKAIGKV;LANSVKELTNPVVAE-
VKELTNPVV;ALANSVKELTNPVVA-VKELTNPVV;VESLAKAAQEALANS-
LAKAAQEAL;ESVESLAKAAQEALA-SLAKAAQEA;AILKSNPTKDKGAKE-
LKSNPTKDK;DELANKAIGKVIHQN-LANKAIGKV;SVESLAKAAQEALAN-
LAKAAQEAL;SLAKAAQEALANSVK-AQEALANSV;SESVESLAKAAQEAL-SLAKAAQEA
DRB1_0301
DRB1_0401 EALANSVKELTNPVV-SVKELTNPV;QEALANSVKELTNPV-
LANSVKELT;LANSVKELTNPVVAE-SVKELTNPV;ALANSVKELTNPVVA-
SVKELTNPV;ANSVKELTNPVVAES-SVKELTNPV
```

DRB1_0404 LMTLFLFISCNNSGG-LFISCNNSG;MTLFLFISCNNSGGD-
LFISCNNSG;ILMTLFLFISCNNSG-FLFISCNNS;TLFLFISCNNSGGDT-
LFISCNNSG;LFLFISCNNSGGDTA-LFISCNNSG
DRB1_0405 ANSVKELTNPVVAES-VKELTNPVV;LANSVKELTNPVVAE-
VKELTNPVV;EALANSVKELTNPVV-SVKELTNPV;NSVKELTNPVVAESP-
VKELTNPVV;ALANSVKELTNPVVA-VKELTNPVV
DRB1_0701 VISKKITDSNAFVLA-ITDSNAFVL;ISKKITDSNAFVLAV-
ITDSNAFVL;TVISKKITDSNAFVL-SKKITDSNA;SKKITDSNAFVLAVK-
ITDSNAFVL;KKITDSNAFVLAVKE-ITDSNAFVL;ALANSVKELTNPVVA-
VKELTNPVV;LANSVKELTNPVVAE-VKELTNPVV;ANSVKELTNPVVAES-
VKELTNPVV;NSVKELTNPVVAESP-VKELTNPVV;EALANSVKELTNPVV-
SVKELTNPV;KITDSNAFVLAVKEV-ITDSNAFVL;ITDSNAFVLAVKEVE-ITDSNAFVL
DRB1_0802
DRB1_0901
DRB1_1101
DRB1_1302 EALANSVKELTNPVV-LANSVKELT;SAKGPNLTVISKKIT-
KGPNLTVIS;KGPNLTVISKKITDS-LTVISKKIT;AKGPNLTVISKKITD-
LTVISKKIT;QEALANSVKELTNPV-LANSVKELT;GPNLTVISKKITDSN-
LTVISKKIT;PNLTVISKKITDSNA-LTVISKKIT;AAQEALANSVKELTN-
LANSVKELT;KAAQEALANSVKELT-EALANSVKE;AQEALANSVKELTNP-
LANSVKELT;ALANSVKELTNPVVA-LANSVKELT;LANSVKELTNPVVAE-
LANSVKELT;AKEAILKSNPTKDKG-EAILKSNPT;HAKEAILKSNPTKDK-
EAILKSNPT;NLTVISKKITDSNAF-LTVISKKIT;LTVISKKITDSNAFV-
LTVISKKIT;AIGKVIHQNNGLNAN-IHQNNGLNA;IGKVIHQNNGLNANA-
HQNNGLNAN;KEAILKSNPTKDKGA-LKSNPTKDK;EAILKSNPTKDKGAK-
LKSNPTKDK;GKVIHQNNGLNANAG-HQNNGLNAN;LAGAYAISTLITEKL-
YAISTLITE;KVIHQNNGLNANAGQ-HQNNGLNAN;AGAYAISTLITEKLS-
ISTLITEKL;HQNNGLNANAGQNGS-LNANAGQNG;YAISTLITEKLSKLK-
ISTLITEKL;AYAISTLITEKLSKL-ISTLITEKL;NGLNANAGQNGSLLA-LNANAGQNG
DRB1_1501 ALANSVKELTNPVVA-VKELTNPVV;ANSVKELTNPVVAES-
VKELTNPVV;LANSVKELTNPVVAE-VKELTNPVV;EALANSVKELTNPVV-
SVKELTNPV;NSVKELTNPVVAESP-VKELTNPVV
DRB3_0101
DRB4_0101
DRB5_0101

**<CAF34024 outer surface protein VlsE;Protein;Borrelia garinii>**

**Allele 8-mer**
A0101
A0201 KLDELVSA;KISSAIFL;KIAAAIVL;ILKAIVEA
A0202
KISSAIFL;KLDELVSA;KIAAAIVL;ILKAIVEA;LTALLVFI;ALGEKGAL;FINCKNNA;SAIFLTAL
;FLTALLVF;AIFLTALL;AAGAVTAV;AVSGEQIL;GLVADTFF
A0203
ILKAIVEA;KLDELVSA;ALKDVKAA;FINCKNNA;ALGEKGAL;KISSAIFL;AAGAVTAV;KIAAAIVL
;SISSTLKA;MIKAAEEA;SAIFLTAL;LTALLVFI;AAIVLRGV;GIKGIVDA;IAKAAGAV
A0204 ALGEKGAL;AAGAVTAV;KIAAAIVL;KLDELVSA
A0206
KLDELVSA;KISSAIFL;SAIFLTAL;AAGAVTAV;KIAAAIVL;FIDVFNAF;FAGNANAA;AAIVLRGV
;KAAGAVTA;FLTALLVF;FESISSTL;WLEEMIKA;KASVESAV;AIFLTALL;
A0211
KLDELVSA;KISSAIFL;WLEEMIKA;ALGEKGAL;KIAAAIVL;AIFLTALL;ALKDVKAA;ILKAIVEA
;FLTALLVF;AKDGKFAV;AVSGEQIL;ALLVFINC;IFLTALLV;SISSTLKA;AAGAVTAV
A0212 KLDELVSA;ALGEKGAL;ALKDVKAA;WLEEMIKA;FLTALLVF;AKDGKFAV;AVSGEQIL

A0216
ALGEKGAL;KLDELVSA;KISSAIFL;AAGAVTAV;AIFLTALL;ILKAIVEA;WLEEMIKA;ALKDVKAA
;KIAAAIVL;AVSGEQIL
A0219 KLDELVSA;KISSAIFL;AKDGKFAV
A0301 LVADTFFK;IVLRGVAK;LLVFINCK;VSGEQILK;ESISSTLK;TLKATKGK
A1101
LVADTFFK;SAANHGAK;SSTLKATK;IVLRGVAK;VSGEQILK;ESISSTLK;GAAADIAK;AAFKDEMK
;SAVDEVSK;TFFKSDPK;GIVDAAGK;KAAEEAAK;IAAAIVLR;LLVFINCK;TLKATKGK
A2301 FYESIINL;KWLEEMIK;GLVADTFF
A2402 FYESIINL;IFLTALLV
A2403 FYESIINL
A2601
A2602 FIDVFNAF
A2902
A3001
TLKATKGK;AGKALGEK;TFFKSDPK;AFKDEMKK;KGIAKGIK;IVLRGVAK;LVADTFFK;KWLEEMIK
A3002
A3101 IAAAIVLR;LVADTFFK
A3301 IAAAIVLR
A6801
ESISSTLK;IAAAIVLR;LVADTFFK;EANADAGK;LLVFINCK;SAVDEVSK;SAANHGAK;SSTLKATK
;TFFKSDPK;DSVKGIAK;AAFKDEMK;DDNGAAFK;DPANQAGK
A6802 LTALLVFI;SAIFLTAL;DSAANHGA;FNAFSGLV;NAAVGAAA
A6901 LTALLVFI
B0702 SAIFLTAL
B0801
B0802
B1501 FLTALLVF;IINLGNGF
B1801 FESISSTL
B2705
B3501 FIDVFNAF;SAIFLTAL;FAGNANAA;NAAVGAAA
B3901 FESISSTL
B4001 FESISSTL
B4002
B4402
B4403
B4501 AEEAKNPI
B5101
B5301
B5401 FAGNANAA
B5701
B5801 KSDVKTYF

**Allele 9-mer**
A0101
A0201
FLTALLVFI;KLFAGNANA;KISSAIFLT;FINCKNNAV;AIFLTALLV;QILKAIVEA;FAGNANAAV;K
AAGAVTAV
A0202
FLTALLVFI;FINCKNNAV;ILKAIVEAA;KAAGAVTAV;KLFAGNANA;FAGNANAAV;SSAIFLTAL;S
AIFLTALL;KISSAIFLT;SVESAVDEV;KAAEEAAKV;GIKGIVDAA;ALKDVKAAA;GLVADTFFK
A0203
FINCKNNAV;FLTALLVFI;ILKAIVEAA;KAAGAVTAV;KLFAGNANA;ALKDVKAAA;GIAKGIKGI;N
LGNGFIDV;SVKTFYESI;SVKASVESA;AAAIVLRGV;IINLGNGFI;FAGNANAAV;GIKGIVDAA;SA
IFLTALL;MIKAAEEAA;ATKGKLDEL;SVKGIAKGI;AIFLTALLV;KAAEEAAKV

A0204
FLTALLVFI;KAAGAVTAV;KAAEEAAKV;FINCKNNAV;FAGNANAAV;SVESAVDEV;KLFAGNANA
A0206
FAGNANAAV;FINCKNNAV;FLTALLVFI;KAAGAVTAV;NLGNGFIDV;AIFLTALLV;KAAEEAAKV;K
LFAGNANA;SVESAVDEV;SAIFLTALL;AAAIVLRGV;QILKAIVEA;LVADTFFKS;KISSAIFLT;SS
AIFLTAL
A0211
FLTALLVFI;NLGNGFIDV;AIFLTALLV;KLFAGNANA;AVDEVSKWL;VAKDGKFAV;ALKDVKAAA;K
LDELVSAK;WLEEMIKAA;KAAGAVTAV;KAAEEAAKV;SVESAVDEV;FINCKNNAV;FAGNANAAV;IL
KAIVEAA;QILKAIVEA;GIAKGIKGI;GLVADTFFK;NADAGKLFA
A0212
FLTALLVFI;NLGNGFIDV;VAKDGKFAV;ALKDVKAAA;KLFAGNANA;FINCKNNAV;FAGNANAAV;A
VDEVSKWL;AIFLTALLV;WLEEMIKAA;KLDELVSAK;GIAKGIKGI;KAAGAVTAV
A0216
NLGNGFIDV;FLTALLVFI;AIFLTALLV;ALKDVKAAA;KLFAGNANA;SVESAVDEV;FAGNANAAV;V
AKDGKFAV;AVDEVSKWL;FINCKNNAV;KAAGAVTAV;EMIKAAEEA;TLKATKGKL;QILKAIVEA;DI
AKAAGAV;WLEEMIKAA
A0219
FLTALLVFI;NLGNGFIDV;VAKDGKFAV;KAAGAVTAV;FAGNANAAV;FINCKNNAV;SVESAVDEV;A
IFLTALLV
A0301
GLVADTFFK;KLDELVSAK;KIAAAIVLR;ALGEKGALK;AVSGEQILK;ISSTLKATK;AAFKDEMKK;A
LLVFINCK
A1101
GLVADTFFK;AVSGEQILK;AAFKDEMKK;ALLVFINCK;STLKATKGK;AIVLRGVAK;KIAAAIVLR;D
TFFKSDPK;ISSTLKATK;KLDELVSAK;AAGKALGEK;ALGEKGALK;TFFKSDPKK;AVGKGNDDK
A2301  IFLTALLVF;GFIDVFNAF;TFYESIINL;TFFKSDPKK
A2402  IFLTALLVF;GFIDVFNAF;YFESISSTL
A2403  IFLTALLVF;GFIDVFNAF;YFESISSTL
A2601  SIINLGNGF;EVSKWLEEM;DVFNAFSGL
A2602  EVSKWLEEM;DVFNAFSGL;SIINLGNGF
A2902
A3001
STLKATKGK;AGKKAEEAK;TFFKSDPKK;GLVADTFFK;ALLVFINCK;ANHGAKADK;AAFKDEMKK;D
TFFKSDPK;AAGKALGEK;AVSGEQILK
A3002
A3101  KIAAAIVLR;ISSTLKATK
A3301  DTFFKSDPK
A6801
DTFFKSDPK;ESAVDEVSK;DSAANHGAK;KIAAAIVLR;TFFKSDPKK;GLVADTFFK;ISSTLKATK;S
TLKATKGK;AAFKDEMKK;GAAFKDEMK
A6802
DVFNAFSGL;SAIFLTALL;FINCKNNAV;SSAIFLTAL;DIAKAAGAV;DVKTYFESI;FLTALLVFI;L
VADTFFKS;FAGNANAAV;SVKTFYESI;EGGSVKASV;KAAGAVTAV;NANAAVGAA;AAAIVLRGV;IS
SAIFLTA
A6901
FAGNANAAV;FINCKNNAV;EVSKWLEEM;DIAKAAGAV;DVFNAFSGL;NLGNGFIDV;QILKAIVEA
B0702 KAAGAVTAV;IVDAAGKAL
B0801
B0802
B1501 SIINLGNGF;GFIDVFNAF
B1801
B2705
B3501 FAGNANAAV;TAVSGEQIL;IFLTALLVF;TDDDNGAAF;FSGLVADTF;GFIDVFNAF
B3901 YFESISSTL
B4001

```
B4002 EEAKNPIAA
B4402
B4403 EEAKNPIAA
B4501 EEAKNPIAA;AEEAKNPIA
B5101
B5301
B5401 NPIAAAIGT
B5701
B5801 SAVDEVSKW;
```

**Allele 10-mer**
```
A0101
A0201 KISSAIFLTA;KLFAGNANAA;KTFYESIINL
A0202 KLFAGNANAA;KTFYESIINL;KISSAIFLTA;SSAIFLTALL;SIINLGNGFI;IAAAIVLRGV
A0203
SVKASVESAV;KISSAIFLTA;KLFAGNANAA;IAAAIVLRGV;GIAKGIKGIV;SIINLGNGFI;ATKGK
LDELV;SVKTFYESII;SAIFLTALLV;KTYFESISST;SAKKGEGGSV
A0204 KLFAGNANAA;
A0206
KISSAIFLTA;SAIFLTALLV;KLFAGNANAA;AIVEAAGDPA;GVAKDGKFAV;AKAAGAVTAV;ASVES
AVDEV;QILKAIVEAA;SIINLGNGFI;KTFYESIINL;IAAAIVLRGV;GLVADTFFKS
A0211
KLFAGNANAA;GVAKDGKFAV;GIAKGIKGIV;KLDELVSAKK;KISSAIFLTA;SAIFLTALLV;DVFNA
FSGLV;SVKASVESAV;SAVDEVSKWL;IAAAIVLRGV;LFAGNANAAV;SIINLGNGFI;AVSGEQILKA
;GIVDAAGKAL;FLTALLVFIN
A0212 KLFAGNANAA;GVAKDGKFAV;GIAKGIKGIV;LFAGNANAAV;KLDELVSAKK
A0216 KLFAGNANAA;GVAKDGKFAV;GIAKGIKGIV;SVKASVESAV;DVFNAFSGLV;LFAGNANAAV
A0219 IAAAIVLRGV;LFAGNANAAV
A0301
KALGEKGALK;KLDELVSAKK;AAIVLRGVAK;VLRGVAKDGK;SISSTLKATK;KVGGTGGDGK;SVKGI
AKGIK;ADTFFKSDPK;TAVSGEQILK;VAKDGKFAVK;SSTLKATKGK
A1101
TALLVFINCK;SISSTLKATK;AAIVLRGVAK;AVGAAADIAK;TAVSGEQILK;KALGEKGALK;SSTLK
ATKGK;SGLVADTFFK;SVKGIAKGIK;DTFFKSDPKK;VSKWLEEMIK;KVGGTGGDGK;KLDELVSAKK
;GAAFKDEMKK;MIKAAEEAAK;AANHGAKADK;VAKDGKFAVK
A2301 TYFESISSTL;IFLTALLVFI;AFSGLVADTF;AIFLTALLVF;FSGLVADTFF
A2402 IFLTALLVFI;TYFESISSTL;AFSGLVADTF
A2403 TYFESISSTL;AFSGLVADTF;IFLTALLVFI
A2601 ESIINLGNGF;SIINLGNGFI;
A2602 ESIINLGNGF;GTDDDNGAAF;AIFLTALLVF;EVSKWLEEMI
A2902
A3001
SGLVADTFFK;SVKGIAKGIK;KALGEKGALK;MIKAAEEAAK;AAIVLRGVAK;VAKDGKFAVK;VLRGV
AKDGK;AVGAAADIAK;AANHGAKADK;SSTLKATKGK;KVGGTGGDGK
A3002
A3101 AAIVLRGVAK
A3301
A6801
DTFFKSDPKK;TAVSGEQILK;MIKAAEEAAK;DAAGKALGEK;TALLVFINCK;DKIAAAIVLR;YFESI
SSTLK;SISSTLKATK;SVKGIAKGIK;NGAAFKDEMK;GAAFKDEMKK;SSTLKATKGK
A6802
DVFNAFSGLV;IAAAIVLRGV;SSAIFLTALL;SAIFLTALLV;EAAGDPANQA;DSVKTFYESI;EVSKW
LEEMI;EAKNPIAAAI;SVKASVESAV;ISSAIFLTAL;NANAAVGAAA;HGAKADKDSV;SIINLGNGFI
A6901 DVFNAFSGLV;EVSKWLEEMI;EAAGDPANQA;SAIFLTALLV
B0702
B0801
```

```
B0802
B1501  AIFLTALLVF
B1801
B2705
B3501  EANADAGKLF;DPKKSDVKTY;NGFIDVFNAF;FSGLVADTFF;NANAAVGAAA
B3901
B4001
B4002
B4402
B4403
B4501  AEEAKNPIAA;EEMIKAAEEA;AEEAAKVGGT;EEAKNPIAAA
B5101
B5301
B5401
B5701  ESAVDEVSKW
B5801  ESAVDEVSKW;ISSAIFLTAL
```

**Allele 11-mer**
```
A0101
A0201  KLFAGNANAAV;FIDVFNAFSGL;KISSAIFLTAL;KIAAAIVLRGV;AIFLTALLVFI
A0202
KISSAIFLTAL;KLFAGNANAAV;FIDVFNAFSGL;KIAAAIVLRGV;LLVFINCKNNA;FLTALLVFINC
;ISSAIFLTALL;MIKAAEEAAKV;KTYFESISSTL;LVFINCKNNAV;AIFLTALLVFI;KIGDSAANHG
A;KASVESAVDEV;ALGEKGALKDV;AVSGEQILKAI
A0203
KIAAAIVLRGV;KLFAGNANAAV;MIKAAEEAAKV;ALGEKGALKDV;KISSAIFLTAL;FIDVFNAFSGL
;IAKAAGAVTAV;LLVFINCKNNA;IINLGNGFIDV;KTYFESISSTL;LVFINCKNNAV;FLTALLVFIN
C;KIGDSAANHGA;AAADIAKAAGA
A0204  KLFAGNANAAV;KIAAAIVLRGV;ALGEKGALKDV
A0206
KLFAGNANAAV;FIDVFNAFSGL;KISSAIFLTAL;KIAAAIVLRGV;FAGNANAAVGA;KIGDSAANHGA
;KTYFESISSTL;FLTALLVFINC;KASVESAVDEV;LVFINCKNNAV;KKISSAIFLTA;AIFLTALLVF
I;SSAIFLTALLV;ALGEKGALKDV
A0211
KLFAGNANAAV;ALGEKGALKDV;KIAAAIVLRGV;FIDVFNAFSGL;KISSAIFLTAL;AIFLTALLVFI
;FLTALLVFINC;IINLGNGFIDV;KIGDSAANHGA;KATKGKLDELV;LVFINCKNNAV;MIKAAEEAAK
V;SSAIFLTALLV;AVSGEQILKAI;AVTAVSGEQIL;KTYFESISSTL
A0212
KLFAGNANAAV;ALGEKGALKDV;FIDVFNAFSGL;KIAAAIVLRGV;FLTALLVFINC;MIKAAEEAAKV
;LVFINCKNNAV;KISSAIFLTAL;IINLGNGFIDV
A0216
KLFAGNANAAV;ALGEKGALKDV;MIKAAEEAAKV;FIDVFNAFSGL;KISSAIFLTAL;KIAAAIVLRGV
;AIFLTALLVFI;LVFINCKNNAV;IINLGNGFIDV;KIGDSAANHGA;KATKGKLDELV;LLVFINCKNN
A
A0219  KLFAGNANAAV;FIDVFNAFSGL;KIAAAIVLRGV;KISSAIFLTAL;ALGEKGALKDV
A0301
FSGLVADTFFK;VTAVSGEQILK;GVAKDGKFAVK;FINCKNNAVGK;TYFESISSTLK;LTALLVFINCK
A1101
LTALLVFINCK;VTAVSGEQILK;SVESAVDEVSK;GTDDDNGAAFK;FSGLVADTFFK;TYFESISSTLK
;AAAIVLRGVAK;GVAKDGKFAVK;SAANHGAKADK;VADTFFKSDPK;AAVGAAADIAK;IVLRGVAKDG
K;AAGDPANQAGK;ESISSTLKATK;ISSTLKATKGK;FINCKNNAVGK;EVSKWLEEMIK;GIKGIVDAA
GK
A2301  TYFESISSTLK;AFSGLVADTFF;SAIFLTALLVF
A2402  AFSGLVADTFF
A2403  AFSGLVADTFF
A2601
```

```
A2602 KISSAIFLTAL;FIDVFNAFSGL
A2902
A3001 KGKLDELVSAK;TYFESISSTLK;SAKKGEGGSVK;AFKDEMKKSDK;IVLRGVAKDGK
A3002 AFSGLVADTFF
A3101 TYFESISSTLK
A3301
A6801
ESISSTLKATK;LTALLVFINCK;EVSKWLEEMIK;TYFESISSTLK;FSGLVADTFFK;EMIKAAEEAAK
;FINCKNNAVGK;VTAVSGEQILK;SVESAVDEVSK;DSVKGIAKGIK;SAANHGAKADK;NGAAFKDEMK
K;ISSTLKATKGK
A6802
ESIINLGNGFI;ISSAIFLTALL;SSAIFLTALLV;LVFINCKNNAV;FIDVFNAFSGL;KIAAAIVLRGV
;ESAVDEVSKWL;EEAKNPIAAAI;IAAAIVLRGVA;DVFNAFSGLVA;MIKAAEEAAKV
A6901 LVFINCKNNAV;FIDVFNAFSGL
B0702
B0801
B0802
B1501 VLRGVAKDGKF;KLFAGNANAAV
B1801 YESIINLGNGF;DEANADAGKLF
B2705
B3501 NAFSGLVADTF;SAIFLTALLVF;IGTDDDNGAAF;DPKKSDVKTYF
B3901
B4001 YESIINLGNGF
B4002 EEAKNPIAAAI
B4402
B4403 EEAKNPIAAAI
B4501 EEMIKAAEEAA;AEEAKNPIAAA;EEAKNPIAAAI
B5101
B5301
B5401
B5701
B5801 SAIFLTALLVF;KTYFESISSTL;KSDKIAAAIVL;ISSAIFLTALL
```

**Allele 15-mer plus 9-mer core**
```
DRB1_0101 EQILNAIVTAAGQAG-LNAIVTAAG;AGDVKDAAAAVGAVS-
VKDAAAAVG;GDVKDAAAAVGAVSG-VKDAAAAVG;QILNAIVTAAGQAGQ-
IVTAAGQAG;ADAGDVKDAAAAVGA-VKDAAAAVG;DAGDVKDAAAAVGAV-
VKDAAAAVG;ILNAIVTAAGQAGQA-IVTAAGQAG;LNAIVTAAGQAGQAG-
IVTAAGQAG;EGVKFAPKAAADAAA-FAPKAAADA;GADAGDVKDAAAAVG-
AGDVKDAAA;GVKFAPKAAADAAAA-FAPKAAADA;VEGVKFAPKAAADAA-
FAPKAAADA;VKFAPKAAADAAAAD-FAPKAAADA;NAIVTAAGQAGQAGK-
IVTAAGQAG;DQIAAALVLRGVAKD-IAAALVLRG;KKNDQIAAALVLRGV-
IAAALVLRG;KNDQIAAALVLRGVA-IAAALVLRG;KVEGVKFAPKAAADA-
VKFAPKAAA;NDQIAAALVLRGVAK-IAAALVLRG;KKKNDQIAAALVLRG-
DQIAAALVL;SGEQILNAIVTAAGQ-LNAIVTAAG;GEQILNAIVTAAGQA-
LNAIVTAAG;VSGEQILNAIVTAAG-ILNAIVTAA;DVKDAAAAVGAVSGE-
VKDAAAAVG;VKDAAAAVGAVSGEQ-VKDAAAAVG;IEVFNAFSGLVADAF-
VFNAFSGLV;FIEVFNAFSGLVADA-VFNAFSGLV;GFIEVFNAFSGLVAD-
VFNAFSGLV;NGFIEVFNAFSGLVA-VFNAFSGLV;AIVTAAGQAGQAGKK-
IVTAAGQAG;KFAPKAAADAAAADG-FAPKAAADA;IVTAAGQAGQAGKKA-
IVTAAGQAG;FAPKAAADAAAADGN-FAPKAAADA;QIAAALVLRGVAKDG-
IAAALVLRG;AVSGEQILNAIVTAA-EQILNAIVT;GKLFGTAAGADAGDV-
FGTAAGADA;KAGKLFGTAAGADAG-FGTAAGADA;KLFGTAAGADAGDVK-
FGTAAGADA;AGKLFGTAAGADAGD-FGTAAGADA;EVFNAFSGLVADAFS-
FNAFSGLVA;IAAALVLRGVAKDGK-IAAALVLRG;KKAGKLFGTAAGADA-
LFGTAAGAD;AIFIVAFLALIGCKN-IVAFLALIG;ISSAIFIVAFLALIG-
```

```
FIVAFLALI;SAIFIVAFLALIGCK-IVAFLALIG;SSAIFIVAFLALIGC-
IVAFLALIG;SDKIGNVAAGGGAGA-IGNVAAGGG;DKIGNVAAGGGAGAD-
IGNVAAGGG;GNGFIEVFNAFSGLV-FIEVFNAFS;IFIVAFLALIGCKNN-
IVAFLALIG;VFNAFSGLVADAFSK-VFNAFSGLV;KIGNVAAGGGAGADK-
VAAGGGAGA;IGNVAAGGGAGADKE-VAAGGGAGA;LFGTAAGADAGDVKD-
FGTAAGADA;FNAFSGLVADAFSKA-FSGLVADAF;FGTAAGADAGDVKDA-
FGTAAGADA;FIVAFLALIGCKNNV-IVAFLALIG;NAFSGLVADAFSKAD-
FSGLVADAF;KISSAIFIVAFLALI-IFIVAFLAL;GLVADAFSKADPKKS-
VADAFSKAD;LVADAFSKADPKKSD-FSKADPKKS;ADAFSKADPKKSDVK-
FSKADPKKS;GSDKIGNVAAGGGAG-IGNVAAGGG;VADAFSKADPKKSDV-
FSKADPKKS;DAFSKADPKKSDVKT-FSKADPKKS;GNVAAGGGAGADKES-
VAAGGGAGA;GGSDKIGNVAAGGGA-IGNVAAGGG;KDAAAAVGAVSGEQI-
AAAVGAVSG;IKKKNDQIAAALVLR-DQIAAALVL;GGGSDKIGNVAAGGG-
KIGNVAAGG;IVAFLALIGCKNNVG-IVAFLALIG;EAKNAIEAAIGAAGD-
IEAAIGAAG;DEAKNAIEAAIGAAG-KNAIEAAIG;SIFYQSIINLGNGFI-
YQSIINLGN;AKNAIEAAIGAAGDA-IEAAIGAAG;DAAAAVGAVSGEQIL-
VGAVSGEQI;KNAIEAAIGAAGDAD-IEAAIGAAG;DIKKKNDQIAAALVL-
IKKKNDQIA;AAALVLRGVAKDGKF-VLRGVAKDG;AFSGLVADAFSKADP-
LVADAFSKA;FSGLVADAFSKADPK-LVADAFSKA;NAIEAAIGAAGDADF-
IEAAIGAAG;AAAVGAVSGEQILNA-VGAVSGEQI;KESVNGIAGAIKGIV-
VNGIAGAIK;AASIFYQSIINLGNG-FYQSIINLG;ASIFYQSIINLGNGF-
FYQSIINLG;TAASIFYQSIINLGN-FYQSIINLG;ESVNGIAGAIKGIVE-
VNGIAGAIK;DKESVNGIAGAIKGI-VNGIAGAIK;MKKISSAIFIVAFLA-
MKKISSAIF;IFYQSIINLGNGFIE-YQSIINLGN;VGAVSGEQILNAIVT-
VSGEQILNA;AAAAVGAVSGEQILN-VGAVSGEQI;AALVLRGVAKDGKFA-
VLRGVAKDG;ADKESVNGIAGAIKG-VNGIAGAIK
DRB1_0301
DRB1_0401 TAASIFYQSIINLGN-FYQSIINLG;AASIFYQSIINLGNG-
FYQSIINLG;SIFYQSIINLGNGFI-FYQSIINLG;ASIFYQSIINLGNGF-
FYQSIINLG;DTAASIFYQSIINLG-SIFYQSIIN;IFYQSIINLGNGFIE-
FYQSIINLG;FYQSIINLGNGFIEV-FYQSIINLG;SGEQILNAIVTAAGQ-
LNAIVTAAG;VSGEQILNAIVTAAG-ILNAIVTAA;GEQILNAIVTAAGQA-
LNAIVTAAG;EQILNAIVTAAGQAG-LNAIVTAAG
DRB1_0404 NGFIEVFNAFSGLVA-VFNAFSGLV;GNGFIEVFNAFSGLV-
FIEVFNAFS;GFIEVFNAFSGLVAD-VFNAFSGLV;FIEVFNAFSGLVADA-
VFNAFSGLV;IEVFNAFSGLVADAF-VFNAFSGLV;EQILNAIVTAAGQAG-
ILNAIVTAA;VSGEQILNAIVTAAG-ILNAIVTAA;GEQILNAIVTAAGQA-
ILNAIVTAA;SGEQILNAIVTAAGQ-ILNAIVTAA;AVSGEQILNAIVTAA-
VSGEQILNA;EVFNAFSGLVADAFS-VFNAFSGLV;VFNAFSGLVADAFSK-
VFNAFSGLV;QILNAIVTAAGQAGQ-ILNAIVTAA;ILNAIVTAAGQAGQA-ILNAIVTAA
DRB1_0405 TAASIFYQSIINLGN-FYQSIINLG;AASIFYQSIINLGNG-
YQSIINLGN;ASIFYQSIINLGNGF-YQSIINLGN;SIFYQSIINLGNGFI-
YQSIINLGN;IFYQSIINLGNGFIE-YQSIINLGN;NGFIEVFNAFSGLVA-
FIEVFNAFS;GFIEVFNAFSGLVAD-FNAFSGLVA;FIEVFNAFSGLVADA-
FNAFSGLVA;FYQSIINLGNGFIEV-YQSIINLGN;YQSIINLGNGFIEVF-
YQSIINLGN;IEVFNAFSGLVADAF-FNAFSGLVA;EVFNAFSGLVADAFS-FNAFSGLVA
DRB1_0701 IEVFNAFSGLVADAF-VFNAFSGLV;NGFIEVFNAFSGLVA-
VFNAFSGLV;GFIEVFNAFSGLVAD-VFNAFSGLV;FIEVFNAFSGLVADA-VFNAFSGLV
DRB1_0802 GLVADAFSKADPKKS-AFSKADPKK;LVADAFSKADPKKSD-
FSKADPKKS;VADAFSKADPKKSDV-FSKADPKKS;ADAFSKADPKKSDVK-
FSKADPKKS;DAFSKADPKKSDVKT-FSKADPKKS;AFSKADPKKSDVKTY-
FSKADPKKS;FSKADPKKSDVKTYF-FSKADPKKS
DRB1_0901 SIFYQSIINLGNGFI-FYQSIINLG;DTAASIFYQSIINLG-
IFYQSIINL;ASIFYQSIINLGNGF-FYQSIINLG;AASIFYQSIINLGNG-
FYQSIINLG;TAASIFYQSIINLGN-FYQSIINLG
DRB1_1101
```

```
DRB1_1302 SIFYQSIINLGNGFI-FYQSIINLG;VSGEQILNAIVTAAG-
ILNAIVTAA;SGEQILNAIVTAAGQ-ILNAIVTAA;IFYQSIINLGNGFIE-
IINLGNGFI;FYQSIINLGNGFIEV-IINLGNGFI;GEQILNAIVTAAGQA-
ILNAIVTAA;EQILNAIVTAAGQAG-ILNAIVTAA;AVSGEQILNAIVTAA-
EQILNAIVT;YQSIINLGNGFIEVF-IINLGNGFI;QSIINLGNGFIEVFN-
IINLGNGFI;ASIFYQSIINLGNGF-FYQSIINLG;AASIFYQSIINLGNG-
FYQSIINLG;TAASIFYQSIINLGN-FYQSIINLG;QILNAIVTAAGQAGQ-
ILNAIVTAA;SIINLGNGFIEVFNA-IINLGNGFI;DTAASIFYQSIINLG-IFYQSIINL
DRB1_1501 GFIEVFNAFSGLVAD-VFNAFSGLV;NGFIEVFNAFSGLVA-
VFNAFSGLV;SAIFIVAFLALIGCK-IVAFLALIG;SSAIFIVAFLALIGC-
IVAFLALIG;IAAALVLRGVAKDGK-ALVLRGVAK;IEVFNAFSGLVADAF-
VFNAFSGLV;AAALVLRGVAKDGKF-LRGVAKDGK;FIEVFNAFSGLVADA-
VFNAFSGLV;AIFIVAFLALIGCKN-IVAFLALIG;IVAFLALIGCKNNVG-
IVAFLALIG;ALVLRGVAKDGKFAG-LRGVAKDGK;AALVLRGVAKDGKFA-
LRGVAKDGK;IFIVAFLALIGCKNN-IVAFLALIG;SIFYQSIINLGNGFI-FYQSIINLG
DRB3_0101
DRB4_0101
DRB5_0101
```

## Fig. 42.

A) Special selected HLA-A*0201 binding peptides from Borrelia proteins

<OspA7;Protein;Borrelia species>FTLEGTLAA
<OspA 8;Protein;Borrelia species>TLVSKKVTL
<OspB 8;Protein;Borrelia species>IMLEGNLV
<OspB 9;Protein;Borrelia species>TMSITDDL
<OspC 7;Protein;Borrelia species>LMTLFLFI
<OspC 8;Protein;Borrelia species>LLAGAYAI
<FlaB 6;Protein;Borrelia species>QASWTLRV
<FlaB 7;Protein;Borrelia species>IAVNIYAA
<VlsE 8;Protein;Borrelia species>LSAIVTAA
<VlsE 9;Protein;Borrelia species>ILSAIVTA
<OspG 6;Protein;Borrelia species>IICAVFVL
<FlaA 6;Protein;Borrelia species>IWSNPNYI
<OspE 4;Protein;Borrelia species>IICAVFVL
<OspE 5;Protein;Borrelia species>FSEFTVNI
<BmpA 3;Protein;Borrelia species>MYSDGIDI
<BmpA 4;Protein;Borrelia species>LAPNNVIT
<BmpB 3;Protein;Borrelia species>LIGVVFRI
<BmpB 4;Protein;Borrelia species>VGDALYLI
<BmpC 3;Protein;Borrelia species>MTEDAYEV
<BmpC 4;Protein;Borrelia species>LNQDQSYI
<BmpD 2;Protein;Borrelia species>MYGYEAGA
<BmpD 3;Protein;Borrelia species>LAPNNVLV
<DbpA 3;Protein;Borrelia species>ILKAKIKA
<DbpA 4;Protein;Borrelia species>TADGIIAI
<DbpB 2;Protein;Borrelia species>LAACNFGL
<DbpB 3;Protein;Borrelia species>LVACSIGL
<BapA 4;Protein;Borrelia species>LFILSLSA
<CRASP-1a;Protein;Borrelia species>KLNIIKLNI
<CRASP-1b;Protein;Borrelia species>LNYEIEKI
<CRASP-1c;Protein;Borrelia species>KLNILTTIL
<CRASP-2a;Protein;Borrelia species>MLISISLL
<CRASP-2b;Protein;Borrelia species>LIDDFAIEL
<CRASP-2c;Protein;Borrelia species>LSCDVSRL
<MalQ 4;Protein;Borrelia species>LLDFASFV
<MalQ 5;Protein;Borrelia species>LNTNEDFV
<MalQ 6;Protein;Borrelia species>IAYDSADV

B) Special selected HLA-A*0301 binding peptides from Borrelia proteins

<BmpA 5;Protein;Borrelia species>IVFLSCSGK
<BmpA 6;Protein;Borrelia species>FLTGYIAAK
<BmpB 5;Protein;Borrelia species>EIFIKQILK
<BmpB 6;Protein;Borrelia species>ALYLITGEY
<BmpC 5;Protein;Borrelia species>KEMARFMYK
<BmpC 6;Protein;Borrelia species>YIAAKMSRK
<BmpD 5;Protein;Borrelia species>SLMYSLTKK

<BmpD 6;Protein;Borrelia species>RSTASNMYR
<DbpA 5;Protein;Borrelia species>IIAIVKVMK
<DbpA 6;Protein;Borrelia species>FTNTQTGSK
<DbpB 5;Protein;Borrelia species>FTGLKTGSK
<DbpB 6;Protein;Borrelia species>LFEAFTGLK
<HSP90 6;Protein;Borrelia species>LLTSGMPSK
<OspA 7;Protein;Borrelia species>LILALIACK
<OspA 8;Protein;Borrelia species>KTKNLVFTK
<Osp A 9;Protein;Borrelia species>ISVNSQKTK
<OspB 9;Protein;Borrelia species>VTLKKEIEK
<OspB 10;Protein;Borrelia species>RTNGTTLEY
<OspB 11;Protein;Borrelia species>MTDADNATK
<OspC 9;Protein;Borrelia species>LANKAIGKK
<OspC 10;Protein;Borrelia species>ILMTLFLFI
<OspC 11;Protein;Borrelia species>AISTLITEK
<OspE 6;Protein;Borrelia species>GSFKTSLYY
<OspE 7;Protein;Borrelia species>NLGTLVIRK
<OspG 7;Protein;Borrelia species>VFVLIISCK
<FlaA 7;Protein;Borrelia species>RVSKSHSSK
<FlaB  8;Protein;Borrelia species>SINAANLSK
<FlaB 9;Protein;Borrelia species>KINTPASLS
<FlaB 10;Protein;Borrelia species>SQASRNTSK
<FlaB 11;Protein;Borrelia species>FQNRLESIK
<VlsE 10;Protein;Borrelia species>AVSGEQILK
<VlsE 11;Protein;Borrelia species>AIVLRGLAK
<BapA 5;Protein;Borrelia species>KQILADLPK
<BapA 6;Protein;Borrelia species>QLNSDKIDY
<CRASP-1d;Protein;Borrelia species> RIIYSSLNY
<CRASP-1e;Protein;Borrelia species>SSLNYEIEK
<CRASP-2d;Protein;Borrelia species>RSRYNNFYK
<CRASP-2e;Protein;Borrelia species> ESFDVISSK
<CRASP-2f;Protein;Borrelia species>LIALKCIVK
<MalQ 1;Protein;Borrelia species>RSFEKFKKK
<MalQ 2;Protein;Borrelia species>FLFASSQSY
<MalQ 3;Protein;Borrelia species>RINLNLKRK

<Nonsense 3;Protein;artificial>GLFGAGAFK
<Nonsense 4;Protein;artificial>GVYAGAVMK

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 5635363 A
- US 2004209295 A
- WO 02072631 A2
- WO 0021989 A
- US 20050255552 A1
- US 4485045 A
- US 4544545 A
- WO 9942597 A
- US 6387622 B, Siiman

- US 4336173 A
- EP 0106873 A
- US 4459378 A
- US 4654267 A
- WO 2007015168 A2, Lohse
- US 20040209295 A
- US 6221363 B
- WO 9425596 A

### Non-patent literature cited in the description

- **KNABEL M et al.** Reversibel MHC multimer staining for functional isolation of T-cell populations and effective adoptive transfer. *Nature medicine,* 2002, vol. 6, 631-637
- **MEYER ABBIE et al.** *PNAS,* 2000, vol. 97 (21
- *J. Biol. Chem.,* 1969, vol. 243, 3552-59
- **EPSTEIN et al.** *Proc. Natl. Acad. Sci. USA,* 1985, vol. 82, 3688
- **HWANG et al.** *Proc. Natl. Acad. Sci. USA,* 1980, vol. 77, 4030
- **LIVINGSTONE ; BARTON.** *CABIOS,* 1993, vol. 9, 745-756
- **SCHATZ, P. J.** *Biotechnology,* 1993, vol. 11 (10), 1138-1143
- **GARBOCZI et al.** *Proc. Natl. Acad. Sci.,* 1992, vol. 89, 3429-33
- **MANZ, R. et al.** *Proc. Natl. Acad. Sci. USA,* 1995, vol. 92, 1921
- **RUSSANO et al.** CD1-restricted recognition of exogenous and self-lipid antigens by duodenal gammadelta+ T lymphocytes. *J Immunol.,* 2007, vol. 178 (6), 3620-6

- **NEVEU et al.** *Int Immunol.,* 2006, vol. 18, 1139-45
- **BANCHEREAU ; PALUCKA.** Nature Reviews. *Immunology,* 2005, vol. 5, 296-306
- **SORENSEN RB ; HADRUP SR ; KOLLGAARD T ; SVANE IM ; THOR STRATEN P ; ANDERSEN MH.** Efficient tumor cell lysis mediated by a Bcl-X(L) specific T cell clone isolated from a breast cancer patient. *Cancer Immunol Immunother,* April 2006, vol. 56 (4), 527-33
- **GARBOCZI et al.** *Nature,* 1996, vol. 384, 134-141
- **SVENDSEN et al.** *J. Immunol.,* 2004, vol. 173 (11), 7037-45
- **SVENDSEN et al.** Tracking of Proinflammatory Collagen-Specific T cells in Early and Late Collagen-Induced Arthritis in Humanized mice. *J. Immunol.,* 2004, vol. 173, 7037-7045
- **EFINOV et al.** *FEBS Letters,* 1994, vol. 341, 54-58
- *Cancer Immunol Immunother,* vol. 56 (4), 527-33